# EUROPEAN PATENT SPECIFICATION

(11) **EP 4 098 650 B1**
(45) Date of publication and mention of the grant of the patent: **10.12.2025**
(21) Application number: 21748308.0
(22) Date of filing: 29.01.2021
(51) Int. Cl.: C07C 235/46, C07C 271/16, C07C 311/09, C07C 317/18, C07C 323/12, C07C 323/17, C07C 327/48, C07C 333/04, A01N 37/18, A01P 5/00, A01P 7/02, A01P 7/04, C07F 7/12, C07C 251/40, C07C 317/28

(54) **3-ALKOXY BENZOIC ACID AMIDE DERIVATIVE, AND PEST CONTROL AGENT**
3-ALKOXYBENZOESÄUREAMIDDERIVAT UND SCHÄDLINGSBEKÄMPFUNGSMITTEL
DÉRIVÉ D'AMIDE D'ACIDE 3-ALCOXYBENZOÏQUE ET AGENT DE LUTTE CONTRE LES NUISIBLES

(30) Priority: 31.01.2020 JP 2020014653
(43) Date of publication of application: 07.12.2022
(73) Proprietor: Kumiai Chemical Industry Co., Ltd., Taito-ku Tokyo 110-8782 (JP)
(72) Inventor: TANAKA, Kosuke, Tokyo 110-8782 (JP); SAKURAI, Sakae, Tokyo 110-8782 (JP); ENOMOTO, Yukihiro, Tokyo 110-8782 (JP); TORIYABE, Keiji, Tokyo 110-8782 (JP); ARAI, Hirokazu, Tokyo 110-8782 (JP); NAKANE, Yuhsuke, Tokyo 110-8782 (JP); MATSUDA, Takeshi, Tokyo 110-8782 (JP); TAKANEZAWA, Akira, Tokyo 110-8782 (JP); KOIKE, Shusuke, Tokyo 110-8782 (JP); KITAHARA, Shunsuke, Tokyo 110-8782 (JP); MUKAWA, Shigeyuki, Tokyo 110-8782 (JP)
(74) Representative: Kraus & Lederer PartGmbB
(86) International application number: PCT/JP2021/003204
(87) International publication number: WO 2021/153720

(56) References cited:
- WO-A1-02/092584
- WO-A1-2019/099703
- WO-A1-2019/099703
- JP-A- 2006 525 964
- JP-A- 2008 525 345
- JP-A- 2015 535 843
- JP-A- 2015 535 843
- JP-A- H0 848 662
- JP-A- S51 125 243
- JP-A- S51 125 243
- JP-A- S51 131 847
- JP-A- S51 131 847
- US-A1- 2007 112 035

## Description

### Technical Field

The present invention relates to a novel 3-alkoxybenzamide derivative or an agriculturally acceptable salt thereof, a pest control agent comprising the derivative as an active ingredient, and a production intermediate of the derivative.

### Background Art

Patent Documents 1 and 2 describe 3-alkoxybenzamide derivatives, but in the compound described in Patent Document 1, the amine moiety of the amide bond is limited to an adamantylalkyl group. In addition, in the compound described in Patent Document 2, the substituent on the alkoxy group is limited to a pyridopyrimidinone ring. Patent Document 3 describes a 3-alkoxybenzamide derivative having herbicidal effect, but does not describe a pest control effect. Among further prior art, JP 2015/535843 A discloses heterocyclic compounds for controlling animal pests.

### Citation List

### Patent Document

Patent Document 1: WO 01/042194 A1
Patent Document 2: WO 2016/068580 A2
Patent Document 3: WO 94/05153 A1

### Summary of the Invention

### Problems to be Solved by the Invention

It is desired that the pest control agent to be used for useful crops is a chemical agent which is applied to soil or foliage and capable of exhibiting a sufficient pest control effect at a low dosage. In addition, due to an increasing demand for safety and influence on the environment of chemical substances, and development of a safer pest control agent is desired. Furthermore, in recent years, pests having acquired resistance to pest control agents have appeared due to long use of pest control agents such as insecticides and acaricides for many years, and therefore it is difficult to completely control pests. In addition, the use of pest control agents having high human and animal toxicity is also a problem in terms of safety for workers and the like.

Under such circumstances, an object of the present invention is to solve the above-described problems of conventional pest control agents, and to provide a pest control agent excellent in safety, control effect, residual effect, and the like.

### Means for Solving the Problems

In order to develop a pest control agent having the above-described preferable properties, the present inventors synthesized various 3-alkoxybenzamide derivatives, and conducted intensive studies on their physiological activity. As a result, the present inventors have found that 3-alkoxybenzamide derivatives (hereinafter, referred to as compounds of the present invention) of formula [I] below exhibit excellent effects on various pests, and have further continued the research to complete the present invention.

The invention is defined by the scope of the appended claims. Any disclosure falling outside the claims is merely provided for illustrative purposes. Also disclosed herein are the following aspects.
(1) A 3-alkoxybenzamide derivative of formula [I], or an agriculturally acceptable salt thereof, 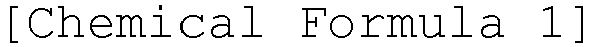 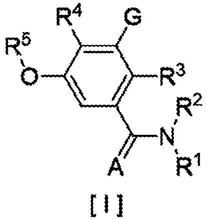
   wherein in the formula [I],
   A represents an oxygen atom or a sulfur atom,
   G represents a hydrogen atom; a halogen atom; or a (C1-C6)alkyl,
   R¹ represents a (C1-C6)alkyl; a (C1-C6)haloalkyl; a (C3-C6)cycloalkyl; a (C3-C6)halocycloalkyl; a (C2-C6)alkenyl; a (C2-C6)haloalkenyl; a (C2-C6)alkynyl; a (C2-C6)haloalkynyl; a (C1-C6)alkoxy; a (C1-C6)haloalkoxy; a (C1-C6)alkoxy (C1-C6)alkyl; a (C3-C6)cycloalkyl (C1-C6)alkyl; a (C3-C6)halocycloalkyl (C1-C6)alkyl; a cyano (C1-C6)alkyl; a cyano (C3-C6)cycloalkyl; a cyano (C3-C6)cycloalkyl (C1-C6)alkyl; a cyano heterocycloalkyl; a hydroxy (C3-C6)cycloalkyl; a (C1-C6)alkylcarbonyloxy (C3-C6)cycloalkyl; a (C1-C6)alkylsulfonyloxy (C3-C6)cycloalkyl; a (C1-C6)alkoxy (C3-C6)cycloalkyl; a (C1-C6)haloalkoxy (C3-C6)cycloalkyl; a (C1-C6)alkylthio (C3-C6)cycloalkyl; a (C1-C6)haloalkylthio (C3-C6)cycloalkyl; a (C1-C6)alkylsulfinyl (C3-C6)cycloalkyl; a (C1-C6)haloalkylsulfinyl (C3-C6)cycloalkyl; a (C1-C6)alkylsulfonyl (C3-C6)cycloalkyl; a (C1-C6)haloalkylsulfonyl (C3-C6)cycloalkyl; a (C2-C6)alkynyl (C3-C6)cycloalkyl; a (C1-C6)alkoxycarbonyl (C3-C6)cycloalkyl; a carboxy (C3-C6)cycloalkyl; a carbamoyl (C3-C6)cycloalkyl; a (C1-C6)alkylaminocarbonyl (C3-C6)cycloalkyl (the amino moiety is optionally substituted with R¹⁰); a (C4-C8)bicycloalkyl; a (C1-C6)alkoxyimino; a phenyl (the phenyl is optionally mono-substituted or poly-substituted with R⁷); a phenyl (C1-C6)alkyl (the phenyl moiety is optionally mono-substituted or poly-substituted with R⁷); a heteroaryl (the heteroaryl is optionally mono-substituted or poly-substituted with R⁷); a heteroaryl (C1-C3)alkyl (the heteroaryl moiety is optionally mono-substituted or poly-substituted with R⁷); a heteroaryl (C3-C6)cycloalkyl (the heteroaryl moiety is optionally mono-substituted or poly-substituted with R⁷); a heterocycloalkyl (the heterocycloalkyl is optionally substituted with R¹⁰); a heterocycloalkyl (C1-C6)alkyl (the heterocycloalkyl moiety is optionally substituted with R¹⁰); or a heterocycloalkyl (C3-C6)cycloalkyl (the heterocycloalkyl moiety is optionally substituted with R¹⁰),
   R² represents a hydrogen atom; a (C1-C6)alkyl; a (C1-C6)haloalkyl; a (C3-C6)cycloalkyl; a (C3-C6)halocycloalkyl; a (C2-C6)alkenyl; a (C2-C6)haloalkenyl; a (C2-C6)alkynyl; a (C2-C6)haloalkynyl; a (C1-C6)alkoxy; a (C1-C6)haloalkoxy; a (C3-C6)cycloalkyl (C1-C6)alkyl; a (C3-C6)halocycloalkyl (C1-C6)alkyl; a phenyl (the phenyl is optionally mono-substituted or poly-substituted with R⁷); a phenyl (C1-C6)alkyl (the phenyl moiety is optionally mono-substituted or poly-substituted with R⁷); a heteroaryl (the heteroaryl is optionally mono-substituted or poly-substituted with R⁷); a heteroaryl (C1-C3)alkyl (the heteroaryl moiety is optionally mono-substituted or poly-substituted with R⁷); a cyano; a cyano (C1-C6)alkyl; a cyano (C3-C6)cycloalkyl; a hydroxy; a hydroxy (C1-C6)alkyl; a (C1-C6)alkoxy (C1-C6)alkyl; a formyl; an amino; a (C1-C6)alkylamino (the amino moiety is optionally substituted with R¹⁰); a (C1-C6)alkylcarbonyl; a (C1-C6)alkoxy (C1-C6)alkylcarbonyl; a (C3-C6)cycloalkylcarbonyl; a (C3-C6)cycloalkyl (C1-C6)alkylcarbonyl; a benzoyl (the benzoyl is optionally mono-substituted or poly-substituted with R⁷); a (C1-C6)alkoxycarbonyl; a (C1-C6)haloalkoxycarbonyl; a (C2-C6)alkenyloxycarbonyl; a carbamoyl; a (C1-C6)alkylaminocarbonyl (the amino moiety is optionally substituted with R¹⁰); a (C1-C6)alkoxycarbonyl (C1-C6)alkylamino (the amino moiety is optionally substituted with R¹⁰); a (C1-C6)alkoxycarbonylamino (the amino moiety is optionally substituted with R¹⁰); a ((C1-C6)alkylthio)carbonyl; a ((C1-C6)alkyl)thiocarbonyl; a ((C1-C6)alkoxy)thiocarbonyl; a thiocarbamoyl; a (C1-C6)alkylaminothiocarbonyl (the amino moiety is optionally substituted with R¹⁰); a (C1-C6)alkylsulfonyl; a (C1-C6)haloalkylsulfonyl; a sulfamoyl; a (C1-C6)alkylaminosulfonyl (the amino moiety is optionally substituted with R¹⁰); a (C1-C6)alkoxycarbonyl (C1-C6)alkyl; an aminocarbonyl (C1-C6)alkyl; a (C1-C6)alkylaminocarbonyl (C1-C6)alkyl (the amino moiety is optionally substituted with R¹⁰); a (C3-C6)cycloalkylaminocarbonyl (C1-C6)alkyl (the amino moiety is optionally substituted with R¹⁰); a (C1-C6)haloalkylaminocarbonyl (C1-C6)alkyl (the amino moiety is optionally substituted with R¹⁰); a (C1-C6)alkylcarbonyl (C1-C6)alkyl; a (C1-C6)haloalkylcarbonyl (C1-C6)alkyl; a (C1-C6)alkylcarbonyloxy (C1-C6)alkyl; a (C1-C6)alkoxycarbonyloxy (C1-C6)alkyl; a (C1-C6)alkoxycarbonylamino (C1-C6)alkyl (the amino moiety is optionally substituted with a (C1-C6)alkyl); a hydroxyimino; a hydroxyimino (C1-C6)alkyl; a (C1-C6)alkoxyimino; a (C1-C6)alkoxyimino (C1-C6)alkyl; or a (C1-C6)haloalkoxyimino (C1-C6)alkyl, or
   R¹ and R², together with the nitrogen atom to which R¹ and R² are attached form a 3- to 8-membered heterocyclic ring or a 3- to 8-membered heterocyclic ring having 1 to 4 heteroatoms selected from an oxygen atom, a sulfur atom, and a nitrogen atom, wherein the formed heterocyclic ring is optionally substituted with a halogen atom, a cyano, a nitro, a (C1-C6)alkyl, a (C1-C6)alkoxy, a (C1-C6)haloalkyl, or an oxo,
   R³ represents a halogen atom; a cyano; a (C1-C6)alkyl; a (C1-C6)haloalkyl; a (C3-C6)cycloalkyl; a (C1-C6)alkoxy; or a (C1-C6)haloalkoxy,
   R⁴ represents a hydrogen atom; a halogen atom; a (C1-C6)alkyl; or a (C1-C6)haloalkyl,
   R⁵ represents a (C1-C12)alkyl (the (C1-C12)alkyl is optionally mono-substituted or poly-substituted with R⁶); a (C3-C6)cycloalkyl (the (C3-C6)cycloalkyl is optionally mono-substituted or poly-substituted with R⁶); a (C2-C6)alkenyl (the (C2-C6)alkenyl is optionally mono-substituted or poly-substituted with R⁶); or a (C2-C6)alkynyl (the (C2-C6)alkynyl is optionally mono-substituted or poly-substituted with R⁶),
   R⁶ represents a halogen atom; a (C1-C6)alkyl; a (C1-C6)haloalkyl; a (C3-C6)cycloalkyl; a (C3-C6)halocycloalkyl; a hydroxy; a (C1-C6)alkoxy; a (C1-C6)haloalkoxy; a (C3-C6)cycloalkoxy; a (C3-C6)halocycloalkoxy; a (C1-C6)alkoxy (C1-C6)alkoxy; a (C1-C6)haloalkoxy (C1-C6)alkoxy; a (C1-C6)haloalkoxy (C1-C6)haloalkoxy; a thiol; a (C1-C6)alkylthio; a (C1-C6)haloalkylthio; a (C2-C6)alkenylthio; a (C2-C6)haloalkenylthio; a (C3-C6)cycloalkylthio; a (C3-C6)halocycloalkylthio; a (C3-C6)cycloalkyl (C1-C6)alkylthio; a (C3-C6)halocycloalkyl (C1-C6)alkylthio; a tri((C1-C6)alkyl)silyl (C1-C6)alkylthio; a (C1-C6)alkylthio (C1-C6)alkoxy; a (C1-C6)haloalkylthio (C1-C6)alkoxy; a (C1-C6)alkylsulfinyl; a (C1-C6)haloalkylsulfinyl; a (C3-C6)cycloalkylsulfinyl; a (C3-C6)halocycloalkylsulfinyl; a (C1-C6)alkylsulfonyl; a (C1-C6)haloalkylsulfonyl; a (C3-C6)cycloalkylsulfonyl; a (C3-C6)halocycloalkylsulfonyl; a formyl; a (C1-C6)alkylcarbonyl; a (C1-C6)haloalkylcarbonyl; a benzoyl (the benzoyl is optionally mono-substituted or poly-substituted with R⁷); a (C1-C6)alkoxycarbonyl; a (C1-C6)haloalkoxycarbonyl; a (C1-C6)alkylaminocarbonyl (the amino moiety is optionally substituted with R¹⁰); a (C1-C6)haloalkylaminocarbonyl (the amino moiety is optionally substituted with R¹⁰); a (C1-C6)alkylaminocarbonylthio (the amino moiety is optionally substituted with R¹⁰); a (C1-C6)haloalkylaminocarbonylthio (the amino moiety is optionally substituted with R¹⁰); a formyloxy; a (C1-C6)alkylcarbonyloxy; a (C1-C6)haloalkylcarbonyloxy; a benzoyloxy (the benzoyloxy is optionally mono-substituted or poly-substituted with R⁷); a (C1-C6)alkylsulfinyloxy; a (C1-C6)haloalkylsulfinyloxy; a (C3-C6)cycloalkylsulfinyloxy; a (C3-C6)halocycloalkylsulfinyloxy; a phenylsulfinyloxy (the phenylsulfinyloxy is optionally mono-substituted or poly-substituted with R⁷); a (C1-C6)alkylsulfonyloxy; a (C1-C6)haloalkylsulfonyloxy; a (C3-C6)cycloalkylsulfonyloxy; a (C3-C6)halocycloalkylsulfonyloxy; a phenylsulfonyloxy (the phenyl moiety is optionally mono-substituted or poly-substituted with R⁷); an amino; a (C1-C6)alkylamino (the amino moiety is optionally substituted with R¹⁰); a (C1-C6)haloalkylamino (the amino moiety is optionally substituted with R¹⁰); a (C1-C6)alkylcarbonylamino (the amino moiety is optionally substituted with R¹⁰); a (C1-C6)haloalkylcarbonylamino (the amino moiety is optionally substituted with R¹⁰); a phenylcarbonylamino (the phenyl moiety is optionally mono-substituted or poly-substituted with R⁷, and the amino moiety is optionally substituted with R¹⁰); a (C1-C6)alkoxycarbonylamino (the amino moiety is optionally substituted with R¹⁰); a (C1-C6)haloalkoxycarbonylamino (the amino moiety is optionally substituted with R¹⁰); a (C1-C6)alkylaminocarbonylamino (the amino moiety is optionally substituted with R¹⁰); a (C1-C6)haloalkylaminocarbonylamino (the amino moiety is optionally substituted with R¹⁰); a (C1-C6)alkylsulfinylamino (the amino moiety is optionally substituted with R¹⁰); a (C1-C6)haloalkylsulfinylamino (the amino moiety is optionally substituted with R¹⁰); a phenylsulfinylamino (the phenyl moiety is optionally mono-substituted or poly-substituted with R⁷, and the amino moiety is optionally substituted with R¹⁰); a (C1-C6)alkylsulfonylamino (the amino moiety is optionally substituted with R¹⁰); a (C1-C6)haloalkylsulfonylamino (the amino moiety is optionally substituted with R¹⁰); a phenylsulfonylamino (the phenyl moiety is optionally mono-substituted or poly-substituted with R⁷, and the amino moiety is optionally substituted with R¹⁰); a (C1-C6)alkoxyimino; a tri((C1-C6)alkyl)silyl; a phenyl (the phenyl is optionally mono-substituted or poly-substituted with R⁷); a phenoxy (the phenoxy is optionally mono-substituted or poly-substituted with R⁷); a phenyl (C1-C6)alkoxy (the phenyl moiety is optionally mono-substituted or poly-substituted with R⁷); a phenylthio (the phenylthio is optionally mono-substituted or poly-substituted with R⁷); a phenylsulfinyl (the phenylsulfinyl is optionally mono-substituted or poly-substituted with R⁷); a phenylsulfonyl (the phenylsulfonyl is optionally mono-substituted or poly-substituted with R⁷); a phenyl (C1-C6)alkylthio (the phenyl moiety is optionally mono-substituted or poly-substituted with R⁷); a phenyl (C1-C6)alkylsulfinyl (the phenyl moiety is optionally mono-substituted or poly-substituted with R⁷); a phenyl (C1-C6)alkylsulfonyl (the phenyl moiety is optionally mono-substituted or poly-substituted with R⁷); a -O-N=C(R⁸)(R⁹) group; an adamantyl; an azetidinyl (a nitrogen atom of the azetidinyl is optionally substituted with R¹⁰); a pyrrolidinyl (a nitrogen atom of the pyrrolidinyl is optionally substituted with R¹⁰); a piperidinyl (a nitrogen atom of the piperidinyl is optionally substituted with R¹⁰); a 1,3-dioxolanyl; a 1,3-dioxanyl; a pyrrolyl (the pyrrolyl is optionally mono-substituted or poly-substituted with R⁷); a pyrazolyl (the pyrazolyl is optionally mono-substituted or poly-substituted with R⁷); an imidazolyl (the imidazolyl is optionally mono-substituted or poly-substituted with R⁷); a triazolyl (the triazolyl is optionally mono-substituted or poly-substituted with R⁷); an oxazolyl (the oxazolyl is optionally mono-substituted or poly-substituted with R⁷); an isoxazolyl (the isoxazolyl is optionally mono-substituted or poly-substituted with R⁷); a thiazolyl (the thiazolyl is optionally mono-substituted or poly-substituted with R⁷); an isothiazolyl (the isothiazolyl is optionally mono-substituted or poly-substituted with R⁷); a pyridyl (the pyridyl is optionally mono-substituted or poly-substituted with R⁷, and further a nitrogen atom of the pyridyl is optionally oxidized to form a N-oxide); a pyrimidinyl (the pyrimidinyl is optionally mono-substituted or poly-substituted with R⁷); a pyridyloxy (the pyridyloxy is optionally mono-substituted or poly-substituted with R⁷); a tetrahydrofuranyl (the tetrahydrofuranyl is optionally mono-substituted or poly-substituted with R⁷); a 1,3-dioxoisoindolinyl (the 1,3-dioxoisoindolinyl is optionally mono-substituted or poly-substituted with R⁷); a cyano; a nitro; a carboxy; a thiocyanato; or an aminooxy,
   R⁷ represents a halogen atom; a (C1-C6)alkyl; a (C1-C6)haloalkyl; a (C3-C6)cycloalkyl; a (C3-C6)halocycloalkyl; a (C3-C6)cycloalkyl (C1-C6)alkyl; a (C3-C6)halocycloalkyl (C1-C6)alkyl; a (C1-C6)alkoxy; a (C1-C6)haloalkoxy; a (C1-C6)alkylthio; a (C1-C6)haloalkylthio; a (C1-C6)alkylsulfinyl; a (C1-C6)haloalkylsulfinyl; a (C1-C6)alkylsulfonyl; a (C1-C6)haloalkylsulfonyl; a (C1-C6)alkylthio (C1-C6)alkyl; a (C1-C6)haloalkylthio (C1-C6)alkyl; a (C1-C6)alkylsulfonyloxy; a (C1-C6)haloalkylsulfonyloxy; a phenyl (the phenyl is optionally mono-substituted or poly-substituted with a halogen atom, an alkyl, or a haloalkyl); a phenyl (C1-C6)alkyl; a phenyl (C1-C6)alkoxy; a cyano; or a nitro,
   R⁸ and R⁹ are identical to or different from each other, and represent a hydrogen atom; a (C1-C6)alkyl; a (C1-C6)haloalkyl; a (C3-C6)cycloalkyl; a (C3-C6)halocycloalkyl; or a phenyl (the phenyl is optionally mono-substituted or poly-substituted with R⁷); or R⁸ and R⁹ ,together with the carbon atom to which R⁸ and R⁹ are attached form a 3- to 6-membered ring, and
   R¹⁰ represents a (C1-C6)alkyl; a (C1-C6)haloalkyl; a (C3-C6)cycloalkyl; a (C3-C6)halocycloalkyl; a (C1-C6)alkoxy; a (C1-C6)alkoxy (C1-C6)alkyl; a (C1-C6)haloalkoxy (C1-C6)alkyl; a (C1-C6)alkoxy (C1-C6)alkoxy (C1-C6)alkyl; a (C1-C6)alkylthio (C1-C6)alkyl; a (C1-C6)alkylsulfinyl (C1-C6)alkyl; a (C1-C6)alkylsulfonyl (C1-C6)alkyl; a (C1-C6)alkylthio (C1-C6)alkoxy (C1-C6)alkyl; a (C1-C6)alkylsulfinyl (C1-C6)alkoxy (C1-C6)alkyl; a (C1-C6)alkylsulfonyl (C1-C6)alkoxy (C1-C6)alkyl; a cyano (C1-C6)alkyl; a cyano (C1-C6)alkoxy (C1-C6)alkyl; a (C1-C6)alkylcarbonyl; a (C1-C6)haloalkylcarbonyl; a (C1-C6)alkoxycarbonyl; a (C1-C6)haloalkoxycarbonyl; a (C1-C6)alkylaminocarbonyl; a (C1-C6)haloalkylaminocarbonyl; a benzoyl (the benzoyl is optionally mono-substituted or poly-substituted with R⁷); a (C1-C6)alkylsulfonyl; a (C1-C6)haloalkylsulfonyl; a (C1-C6)alkylcarbonyl (C1-C6)alkyl; a (C1-C6)alkoxycarbonyl (C1-C6)alkyl; a (C1-C6)alkylaminocarbonyl (C1-C6)alkyl; a (C1-C6)alkylcarbonyloxy (C1-C6)alkyl; a (C1-C6)alkoxycarbonyloxy (C1-C6)alkyl; a (C1-C6)alkoxycarbonylamino (C1-C6)alkyl (the amino moiety is optionally substituted with a (C1-C6)alkyl); or a 5-methyl-1,3-dioxole-2-one-4-ylmethyl.
(2) An agrochemical composition comprising the 3-alkoxybenzamide derivative or the agriculturally acceptable salt thereof according to (1) as an active ingredient.
(3) The agrochemical composition according to (2), which further comprises a surfactant.
(4) A pest control agent comprising the 3-alkoxybenzamide derivative or the agriculturally acceptable salt thereof according to (1) as an active ingredient.
(5) The pest control agent according to (4), wherein the pest control agent is an insecticide, a nematicide, and an acaricide.
(6) The pest control agent according to (5), wherein the pest control agent is capable of controlling pests in a paddy field, a dry field, a lawn, an orchard, a non-crop land, a greenhouse, a raising seeding facility, and a plant factory where an agricultural or horticultural plant is cultivated.
(7) The pest control agent according to (6), wherein the agricultural or horticultural plant is a plant to which resistance is imparted by a breeding or genetic modification techniques.
(8) A process for controlling a pest, comprising a step of applying an effective amount of the 3-alkoxybenzamide derivative or the agriculturally acceptable salt thereof according to (1).
(9) A process for controlling a pest, comprising a step of applying an agrochemical composition comprising the 3-alkoxybenzamide derivative or the agriculturally acceptable salt thereof according to (1) as an active ingredient to an agricultural or horticultural crop or a place where the agricultural or horticultural crop is to be grown or being grown, in a single or divided dose.
(10) The process for controlling a pest according to (8) or (9), wherein a place where a pest control agent is applied is a paddy field, a dry field, a lawn, an orchard, a non-crop land, a greenhouse, a raising seeding facility, and a plant factory.
(11) The process for controlling a pest according to any one of (8) to (10), wherein the 3-alkoxybenzamide derivative or agriculturally acceptable salt thereof according to (1) is used as an insecticide, a nematicide, and an acaricide.
(12) A process for using a pest control agent, characterized in that, in the process, the pest control agent according to any one of (4) to (7) is applied for controlling a pest harmful to an agricultural or horticultural crop.
(13) A hydroxybenzamide derivative of formula [II], or a salt thereof, 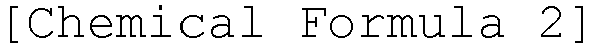 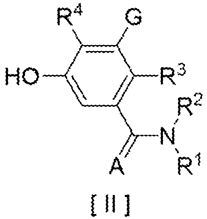 wherein in the formula [II],
   A represents an oxygen atom or a sulfur atom,
   G represents a hydrogen atom; a halogen atom; or a (C1-C6)alkyl,
   R¹ represents a (C1-C6)alkyl; a (C1-C6)haloalkyl; a (C3-C6)cycloalkyl; a (C3-C6)halocycloalkyl; a (C2-C6)alkenyl; a (C2-C6)haloalkenyl; a (C2-C6)alkynyl; a (C2-C6)haloalkynyl; a (C1-C6)alkoxy; a (C1-C6)haloalkoxy; a (C1-C6)alkoxy (C1-C6)alkyl; a (C3-C6)cycloalkyl (C1-C6)alkyl; a (C3-C6)halocycloalkyl (C1-C6)alkyl; a cyano (C1-C6)alkyl; a cyano (C3-C6)cycloalkyl; a cyano (C3-C6)cycloalkyl (C1-C6)alkyl; a cyano heterocycloalkyl; a hydroxy (C3-C6)cycloalkyl; a (C1-C6)alkylcarbonyloxy (C3-C6)cycloalkyl; a (C1-C6)alkylsulfonyloxy (C3-C6)cycloalkyl; a (C1-C6)alkoxy (C3-C6)cycloalkyl; a (C1-C6)haloalkoxy (C3-C6)cycloalkyl; a (C1-C6)alkylthio (C3-C6)cycloalkyl; a (C1-C6)haloalkylthio (C3-C6)cycloalkyl; a (C1-C6)alkylsulfinyl (C3-C6)cycloalkyl; a (C1-C6)haloalkylsulfinyl (C3-C6)cycloalkyl; a (C1-C6)alkylsulfonyl (C3-C6)cycloalkyl; a (C1-C6)haloalkylsulfonyl (C3-C6)cycloalkyl; a (C2-C6)alkynyl (C3-C6)cycloalkyl; a (C1-C6)alkoxycarbonyl (C3-C6)cycloalkyl; a carboxy (C3-C6)cycloalkyl; a carbamoyl (C3-C6)cycloalkyl; a (C1-C6)alkylaminocarbonyl (C3-C6)cycloalkyl (the amino moiety is optionally substituted with R¹⁰); a (C4-C8)bicycloalkyl; a (C1-C6)alkoxyimino; a phenyl (the phenyl is optionally mono-substituted or poly-substituted with R⁷); a phenyl (C1-C6)alkyl (the phenyl moiety is optionally mono-substituted or poly-substituted with R⁷); a heteroaryl (the heteroaryl is optionally mono-substituted or poly-substituted with R⁷); a heteroaryl (C1-C3)alkyl (the heteroaryl moiety is optionally mono-substituted or poly-substituted with R⁷); a heteroaryl (C3-C6)cycloalkyl (the heteroaryl moiety is optionally mono-substituted or poly-substituted with R⁷); a heterocycloalkyl (the heterocycloalkyl is optionally substituted with R¹⁰); a heterocycloalkyl (C1-C6)alkyl (the heterocycloalkyl moiety is optionally substituted with R¹⁰); or a heterocycloalkyl (C3-C6)cycloalkyl (the heterocycloalkyl moiety is optionally substituted with R¹⁰),
   R² represents a hydrogen atom; a (C1-C6)alkyl; a (C1-C6)haloalkyl; a (C3-C6)cycloalkyl; a (C3-C6)halocycloalkyl; a (C2-C6)alkenyl; a (C2-C6)haloalkenyl; a (C2-C6)alkynyl; a (C2-C6)haloalkynyl; a (C1-C6)alkoxy; a (C1-C6)haloalkoxy; a (C3-C6)cycloalkyl (C1-C6)alkyl; a (C3-C6)halocycloalkyl (C1-C6)alkyl; a phenyl (the phenyl is optionally mono-substituted or poly-substituted with R⁷); a phenyl (C1-C6)alkyl (the phenyl moiety is optionally mono-substituted or poly-substituted with R⁷); a heteroaryl (the heteroaryl is optionally mono-substituted or poly-substituted with R⁷); a heteroaryl (C1-C3)alkyl (the heteroaryl moiety is optionally mono-substituted or poly-substituted with R⁷); a cyano; a cyano (C1-C6)alkyl; a cyano (C3-C6)cycloalkyl; a hydroxy; a hydroxy (C1-C6)alkyl; a (C1-C6)alkoxy (C1-C6)alkyl; a formyl; an amino; a (C1-C6)alkylamino (the amino moiety is optionally substituted with R¹⁰); a (C1-C6)alkylcarbonyl; a (C1-C6)alkoxy (C1-C6)alkylcarbonyl; a (C3-C6)cycloalkylcarbonyl; a (C3-C6)cycloalkyl (C1-C6)alkylcarbonyl; a benzoyl (the benzoyl is optionally mono-substituted or poly-substituted with R⁷); a (C1-C6)alkoxycarbonyl; a (C1-C6)haloalkoxycarbonyl; a (C2-C6)alkenyloxycarbonyl; a carbamoyl; a (C1-C6)alkylaminocarbonyl (the amino moiety is optionally substituted with R¹⁰); a (C1-C6)alkoxycarbonyl (C1-C6)alkylamino (the amino moiety is optionally substituted with R¹⁰); a (C1-C6)alkoxycarbonylamino (the amino moiety is optionally substituted with R¹⁰); a ((C1-C6)alkylthio)carbonyl; a ((C1-C6)alkyl)thiocarbonyl; a ((C1-C6)alkoxy)thiocarbonyl; a thiocarbamoyl; a (C1-C6)alkylaminothiocarbonyl (the amino moiety is optionally substituted with R¹⁰); a (C1-C6)alkylsulfonyl; a (C1-C6)haloalkylsulfonyl; a sulfamoyl; a (C1-C6)alkylaminosulfonyl (the amino moiety is optionally substituted with R¹⁰); a (C1-C6)alkoxycarbonyl (C1-C6)alkyl; an aminocarbonyl (C1-C6)alkyl; a (C1-C6)alkylaminocarbonyl (C1-C6)alkyl (the amino moiety is optionally substituted with R¹⁰); a (C3-C6)cycloalkylaminocarbonyl (C1-C6)alkyl (the amino moiety is optionally substituted with R¹⁰); a (C1-C6)haloalkylaminocarbonyl (C1-C6)alkyl (the amino moiety is optionally substituted with R¹⁰); a (C1-C6)alkylcarbonyl (C1-C6)alkyl; a (C1-C6)haloalkylcarbonyl (C1-C6)alkyl; a (C1-C6)alkylcarbonyloxy (C1-C6)alkyl; a (C1-C6)alkoxycarbonyloxy (C1-C6)alkyl; a (C1-C6)alkoxycarbonylamino (C1-C6)alkyl (the amino moiety is optionally substituted with a (C1-C6)alkyl); a hydroxyimino; a hydroxyimino (C1-C6)alkyl; a (C1-C6)alkoxyimino; a (C1-C6)alkoxyimino (C1-C6)alkyl; or a (C1-C6)haloalkoxyimino (C1-C6)alkyl, or
   R¹ and R², together with the nitrogen atom to which R¹ and R² are attached form a 3- to 8-membered heterocyclic ring or a 3- to 8-membered heterocyclic ring having 1 to 4 heteroatoms selected from an oxygen atom, a sulfur atom, and a nitrogen atom, and the heterocyclic ring is optionally substituted with a halogen atom, a cyano, a nitro, a (C1-C6)alkyl, a (C1-C6)alkoxy, a (C1-C6)haloalkyl, or an oxo,
   R³ represents a halogen atom; a cyano; a (C1-C6)alkyl; a (C1-C6)haloalkyl; a (C3-C6)cycloalkyl; a (C1-C6)alkoxy; or a (C1-C6)haloalkoxy,
   R⁴ represents a hydrogen atom; a halogen atom; a (C1-C6)alkyl; or a (C1-C6)haloalkyl,
   R⁷ represents a halogen atom; a (C1-C6)alkyl; a (C1-C6)haloalkyl; a (C3-C6)cycloalkyl; a (C3-C6)halocycloalkyl; a (C3-C6)cycloalkyl (C1-C6)alkyl; a (C3-C6)halocycloalkyl (C1-C6)alkyl; a (C1-C6)alkoxy; a (C1-C6)haloalkoxy; a (C1-C6)alkylthio; a (C1-C6)haloalkylthio; a (C1-C6)alkylsulfinyl; a (C1-C6)haloalkylsulfinyl; a (C1-C6)alkylsulfonyl; a (C1-C6)haloalkylsulfonyl; a (C1-C6)alkylthio (C1-C6)alkyl; a (C1-C6)haloalkylthio (C1-C6)alkyl; a (C1-C6)alkylsulfonyloxy; a (C1-C6)haloalkylsulfonyloxy; a phenyl (the phenyl is optionally mono-substituted or poly-substituted with a halogen atom, an alkyl, or a haloalkyl); a phenyl (C1-C6)alkyl; a phenyl (C1-C6)alkoxy; a cyano; or a nitro, and
   R¹⁰ represents a (C1-C6)alkyl; a (C1-C6)haloalkyl; a (C3-C6)cycloalkyl; a (C3-C6)halocycloalkyl; a (C1-C6)alkoxy; a (C1-C6)alkoxy (C1-C6)alkyl; a (C1-C6)haloalkoxy (C1-C6)alkyl; a (C1-C6)alkoxy (C1-C6)alkoxy (C1-C6)alkyl; a (C1-C6)alkylthio (C1-C6)alkyl; a (C1-C6)alkylsulfinyl (C1-C6)alkyl; a (C1-C6)alkylsulfonyl (C1-C6)alkyl; a (C1-C6)alkylthio (C1-C6)alkoxy (C1-C6)alkyl; a (C1-C6)alkylsulfinyl (C1-C6)alkoxy (C1-C6)alkyl; a (C1-C6)alkylsulfonyl (C1-C6)alkoxy (C1-C6)alkyl; a cyano (C1-C6)alkyl; a cyano (C1-C6)alkoxy (C1-C6)alkyl; a (C1-C6)alkylcarbonyl; a (C1-C6)haloalkylcarbonyl; a (C1-C6)alkoxycarbonyl; a (C1-C6)haloalkoxycarbonyl; a (C1-C6)alkylaminocarbonyl; a (C1-C6)haloalkylaminocarbonyl; a benzoyl (the benzoyl is optionally mono-substituted or poly-substituted with R⁷); a (C1-C6)alkylsulfonyl; a (C1-C6)haloalkylsulfonyl; a (C1-C6)alkylcarbonyl (C1-C6)alkyl; a (C1-C6)alkoxycarbonyl (C1-C6)alkyl; a (C1-C6)alkylaminocarbonyl (C1-C6)alkyl; a (C1-C6)alkylcarbonyloxy (C1-C6)alkyl; a (C1-C6)alkoxycarbonyloxy (C1-C6)alkyl; a (C1-C6)alkoxycarbonylamino (C1-C6)alkyl (the amino moiety is optionally substituted with a (C1-C6)alkyl); or a 5-methyl-1,3-dioxole-2-one-4-ylmethyl.
(14) An alkoxybenzoic acid derivative of formula [III] or a salt thereof, 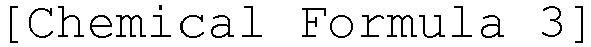 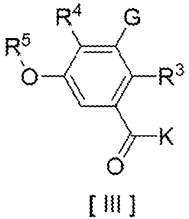 wherein in the formula [III],
   G represents a hydrogen atom; a halogen atom; or a (C1-C6)alkyl,
   K represents a hydroxy; a (C1-C6)alkoxy; or a phenoxy (the phenoxy is optionally mono-substituted or poly-substituted with R⁷),
   R³ represents a halogen atom; a cyano; a (C1-C6)alkyl; a (C1-C6)haloalkyl; a (C3-C6)cycloalkyl; a (C1-C6)alkoxy; or a (C1-C6)haloalkoxy,
   R⁴ represents a hydrogen atom; a halogen atom; a (C1-C6)alkyl; or a (C1-C6)haloalkyl,
   R⁵ represents a (C1-C12)alkyl (the (C1-C12)alkyl is optionally mono-substituted or poly-substituted with R⁶); a (C3-C6)cycloalkyl (the (C3-C6)cycloalkyl is optionally mono-substituted or poly-substituted with R⁶); a (C2-C6)alkenyl (the (C2-C6)alkenyl is optionally mono-substituted or poly-substituted with R⁶); or a (C2-C6)alkynyl (the (C2-C6)alkynyl is optionally mono-substituted or poly-substituted with R⁶),
   R⁶ represents a halogen atom; a (C1-C6)alkyl; a (C1-C6)haloalkyl; a (C3-C6)cycloalkyl; a (C3-C6)halocycloalkyl; a hydroxy; a (C1-C6)alkoxy; a (C1-C6)haloalkoxy; a (C3-C6)cycloalkoxy; a (C3-C6)halocycloalkoxy; a (C1-C6)alkoxy (C1-C6)alkoxy; a (C1-C6)haloalkoxy (C1-C6)alkoxy; a (C1-C6)haloalkoxy (C1-C6)haloalkoxy; a thiol; a (C1-C6)alkylthio; a (C1-C6)haloalkylthio; a (C2-C6)alkenylthio; a (C2-C6)haloalkenylthio; a (C3-C6)cycloalkylthio; a (C3-C6)halocycloalkylthio; a (C3-C6)cycloalkyl (C1-C6)alkylthio; a (C3-C6)halocycloalkyl (C1-C6)alkylthio; a tri((C1-C6)alkyl)silyl (C1-C6)alkylthio; a (C1-C6)alkylthio (C1-C6)alkoxy; a (C1-C6)haloalkylthio (C1-C6)alkoxy; a (C1-C6)alkylsulfinyl; a (C1-C6)haloalkylsulfinyl; a (C3-C6)cycloalkylsulfinyl; a (C3-C6)halocycloalkylsulfinyl; a (C1-C6)alkylsulfonyl; a (C1-C6)haloalkylsulfonyl; a (C3-C6)cycloalkylsulfonyl; a (C3-C6)halocycloalkylsulfonyl; a formyl; a (C1-C6)alkylcarbonyl; a (C1-C6)haloalkylcarbonyl; a benzoyl (the benzoyl is optionally mono-substituted or poly-substituted with R⁷); a (C1-C6)alkoxycarbonyl; a (C1-C6)haloalkoxycarbonyl; a (C1-C6)alkylaminocarbonyl (the amino moiety is optionally substituted with R¹⁰); a (C1-C6)haloalkylaminocarbonyl (the amino moiety is optionally substituted with R¹⁰); a (C1-C6)alkylaminocarbonylthio (the amino moiety is optionally substituted with R¹⁰); a (C1-C6)haloalkylaminocarbonylthio (the amino moiety is optionally substituted with R¹⁰); a formyloxy; a (C1-C6)alkylcarbonyloxy; a (C1-C6)haloalkylcarbonyloxy; a benzoyloxy (the benzoyloxy is optionally mono-substituted or poly-substituted with R⁷); a (C1-C6)alkylsulfinyloxy; a (C1-C6)haloalkylsulfinyloxy; a (C3-C6)cycloalkylsulfinyloxy; a (C3-C6)halocycloalkylsulfinyloxy; a phenylsulfinyloxy (the phenylsulfinyloxy is optionally mono-substituted or poly-substituted with R⁷); a (C1-C6)alkylsulfonyloxy; a (C1-C6)haloalkylsulfonyloxy; a (C3-C6)cycloalkylsulfonyloxy; a (C3-C6)halocycloalkylsulfonyloxy; a phenylsulfonyloxy (the phenyl moiety is optionally mono-substituted or poly-substituted with R⁷); an amino; a (C1-C6)alkylamino (the amino moiety is optionally substituted with R¹⁰); a (C1-C6)haloalkylamino (the amino moiety is optionally substituted with R¹⁰); a (C1-C6)alkylcarbonylamino (the amino moiety is optionally substituted with R¹⁰); a (C1-C6)haloalkylcarbonylamino (the amino moiety is optionally substituted with R¹⁰); a phenylcarbonylamino (the phenyl moiety is optionally mono-substituted or poly-substituted with R⁷, and the amino moiety is optionally substituted with R¹⁰); a (C1-C6)alkoxycarbonylamino (the amino moiety is optionally substituted with R¹⁰); a (C1-C6)haloalkoxycarbonylamino (the amino moiety is optionally substituted with R¹⁰); a (C1-C6)alkylaminocarbonylamino (the amino moiety is optionally substituted with R¹⁰); a (C1-C6)haloalkylaminocarbonylamino (the amino moiety is optionally substituted with R¹⁰); a (C1-C6)alkylsulfinylamino (the amino moiety is optionally substituted with R¹⁰); a (C1-C6)haloalkylsulfinylamino (the amino moiety is optionally substituted with R¹⁰); a phenylsulfinylamino (the phenyl moiety is optionally mono-substituted or poly-substituted with R⁷, and the amino moiety is optionally substituted with R¹⁰); a (C1-C6)alkylsulfonylamino (the amino moiety is optionally substituted with R¹⁰); a (C1-C6)haloalkylsulfonylamino (the amino moiety is optionally substituted with R¹⁰); a phenylsulfonylamino (the phenyl moiety is optionally mono-substituted or poly-substituted with R⁷, and the amino moiety is optionally substituted with R¹⁰); a (C1-C6)alkoxyimino; a tri((C1-C6)alkyl)silyl; a phenyl (the phenyl is optionally mono-substituted or poly-substituted with R⁷); a phenoxy (the phenoxy is optionally mono-substituted or poly-substituted with R⁷); a phenyl (C1-C6)alkoxy (the phenyl moiety is optionally mono-substituted or poly-substituted with R⁷); a phenylthio (the phenylthio is optionally mono-substituted or poly-substituted with R⁷); a phenylsulfinyl (the phenylsulfinyl is optionally mono-substituted or poly-substituted with R⁷); a phenylsulfonyl (the phenylsulfonyl is optionally mono-substituted or poly-substituted with R⁷); a phenyl (C1-C6)alkylthio (the phenyl moiety is optionally mono-substituted or poly-substituted with R⁷); a phenyl (C1-C6)alkylsulfinyl (the phenyl moiety is optionally mono-substituted or poly-substituted with R⁷); a phenyl (C1-C6)alkylsulfonyl (the phenyl moiety is optionally mono-substituted or poly-substituted with R⁷); a -O-N=C(R⁸) (R⁹) group; an adamantyl; an azetidinyl (a nitrogen atom of the azetidinyl is optionally substituted with R¹⁰); a pyrrolidinyl (a nitrogen atom of the pyrrolidinyl is optionally substituted with R¹⁰); a piperidinyl (a nitrogen atom of the piperidinyl is optionally substituted with R¹⁰); a 1,3-dioxolanyl; a 1,3-dioxanyl; a pyrrolyl (the pyrrolyl is optionally mono-substituted or poly-substituted with R⁷); a pyrazolyl (the pyrazolyl is optionally mono-substituted or poly-substituted with R⁷); an imidazolyl (the imidazolyl is optionally mono-substituted or poly-substituted with R⁷); a triazolyl (the triazolyl is optionally mono-substituted or poly-substituted with R⁷); an oxazolyl (the oxazolyl is optionally mono-substituted or poly-substituted with R⁷); an isoxazolyl (the isoxazolyl is optionally mono-substituted or poly-substituted with R⁷); a thiazolyl (the thiazolyl is optionally mono-substituted or poly-substituted with R⁷); an isothiazolyl (the isothiazolyl is optionally mono-substituted or poly-substituted with R⁷); a pyridyl (the pyridyl is optionally mono-substituted or poly-substituted with R⁷, and further a nitrogen atom of the pyridyl is optionally oxidized to form a N-oxide); a pyrimidinyl (the pyrimidinyl is optionally mono-substituted or poly-substituted with R⁷); a pyridyloxy (the pyridyloxy is optionally mono-substituted or poly-substituted with R⁷); a tetrahydrofuranyl (the tetrahydrofuranyl is optionally mono-substituted or poly-substituted with R⁷); a 1,3-dioxoisoindolinyl (the 1,3-dioxoisoindolinyl is optionally mono-substituted or poly-substituted with R⁷); a cyano; a nitro; a carboxy; a thiocyanato; or an aminooxy,
   R⁷ represents a halogen atom; a (C1-C6)alkyl; a (C1-C6)haloalkyl; a (C3-C6)cycloalkyl; a (C3-C6)halocycloalkyl; a (C3-C6)cycloalkyl (C1-C6)alkyl; a (C3-C6)halocycloalkyl (C1-C6)alkyl; a (C1-C6)alkoxy; a (C1-C6)haloalkoxy; a (C1-C6)alkylthio; a (C1-C6)haloalkylthio; a (C1-C6)alkylsulfinyl; a (C1-C6)haloalkylsulfinyl; a (C1-C6)alkylsulfonyl; a (C1-C6)haloalkylsulfonyl; a (C1-C6)alkylthio (C1-C6)alkyl; a (C1-C6)haloalkylthio (C1-C6)alkyl; a (C1-C6)alkylsulfonyloxy; a (C1-C6)haloalkylsulfonyloxy; a phenyl (the phenyl is optionally mono-substituted or poly-substituted with a halogen atom, an alkyl, or a haloalkyl); a phenyl (C1-C6)alkyl; a phenyl (C1-C6)alkoxy; a cyano; or a nitro,
   R⁸ and R⁹ are identical to or different from each other, and represent a hydrogen atom; a (C1-C6)alkyl; a (C1-C6)haloalkyl; a (C3-C6)cycloalkyl; a (C3-C6)halocycloalkyl; or a phenyl (the phenyl is optionally mono-substituted or poly-substituted with R⁷), or R⁸ and R⁹, together with the carbon atom to which R⁸ and R⁹ are attached form a 3- to 6-membered ring, and
   R¹⁰ represents a (C1-C6)alkyl; a (C1-C6)haloalkyl; a (C3-C6)cycloalkyl; a (C3-C6)halocycloalkyl; a (C1-C6)alkoxy; a (C1-C6)alkoxy (C1-C6)alkyl; a (C1-C6)haloalkoxy (C1-C6)alkyl; a (C1-C6)alkoxy (C1-C6)alkoxy (C1-C6)alkyl; a (C1-C6)alkylthio (C1-C6)alkyl; a (C1-C6)alkylsulfinyl (C1-C6)alkyl; a (C1-C6)alkylsulfonyl (C1-C6)alkyl; a (C1-C6)alkylthio (C1-C6)alkoxy (C1-C6)alkyl; a (C1-C6)alkylsulfinyl (C1-C6)alkoxy (C1-C6)alkyl; a (C1-C6)alkylsulfonyl (C1-C6)alkoxy (C1-C6)alkyl; a cyano (C1-C6)alkyl; a cyano (C1-C6)alkoxy (C1-C6)alkyl; a (C1-C6)alkylcarbonyl; a (C1-C6)haloalkylcarbonyl; a (C1-C6)alkoxycarbonyl; a (C1-C6)haloalkoxycarbonyl; a (C1-C6)alkylaminocarbonyl; a (C1-C6)haloalkylaminocarbonyl; a benzoyl (the benzoyl is optionally mono-substituted or poly-substituted with R⁷); a (C1-C6)alkylsulfonyl; a (C1-C6)haloalkylsulfonyl; a (C1-C6)alkylcarbonyl (C1-C6)alkyl; a (C1-C6)alkoxycarbonyl (C1-C6)alkyl; a (C1-C6)alkylaminocarbonyl (C1-C6)alkyl; a (C1-C6)alkylcarbonyloxy (C1-C6)alkyl; a (C1-C6)alkoxycarbonyloxy (C1-C6)alkyl; a (C1-C6)alkoxycarbonylamino (C1-C6)alkyl (the amino moiety is optionally substituted with a (C1-C6)alkyl); or a 5-methyl-1,3-dioxole-2-one-4-ylmethyl.

### Effects of the Invention

The pest control agent comprising the compound of the present invention exhibits an excellent control effect on a wide range of pests such as *Hemiptera* pests, *Lepidoptera* pests, *Coleoptera* pests, *Diptera* pests, *Hymenoptera* pests, *Orthoptera* pests, *Isoptera* pests, *Thysanoptera* pests, *Acari* pests, and plant parasitic nematodes, and can also control pests having acquired chemical resistance.

### Description of Embodiments

Symbols and terms described in the present specification will be described.

In the present invention, the "pest control agent" means insecticides, acaricides, nematicides, and the like for agricultural or horticultural fields, animals such as domestic animals and pets, household use, or prevention of epidemics.

In the present invention, the "halogen atom" represents a fluorine atom; a chlorine atom; a bromine atom; or an iodine atom.

In the present invention, the description of "(C1-C6)" or the like indicates that the number of carbon atoms of the substituent following this description is 1 to 6 in this case.

In the present invention, unless otherwise specified, the "(C1-C6)alkyl" refers to a linear or branched alkyl group having 1 to 6 carbon atoms. Examples thereof include methyl, ethyl, n-propyl, isopropyl, n-butyl, sec-butyl, isobutyl, tert-butyl, n-pentyl, 1-methylbutyl, 2-methylbutyl, 3-methylbutyl, 1-ethylpropyl, 1,1-dimethylpropyl, 1,2-dimethylpropyl, neopentyl, n-hexyl, 1-methylpentyl, 2-methylpentyl, 3-methylpentyl, 4-methylpentyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1,2,2-trimethylpropyl, 1-ethyl-1-methylpropyl, and 1-ethyl-2-methylpropyl groups.

In the present invention, unless otherwise specified, the "(C1-C6)haloalkyl" refers to a linear or branched alkyl group having 1 to 6 carbon atoms and being substituted with 1 to 13 halogen atoms and which are identical or different. Examples thereof include fluoromethyl, difluoromethyl, trifluoromethyl, chloromethyl, dichloromethyl, trichloromethyl, bromomethyl, dibromomethyl, tribromomethyl, iodomethyl, chlorodifluoromethyl, dichlorofluoromethyl, 1-fluoroethyl, 2-fluoroethyl, 1,1-difluoroethyl, 2,2-difluoroethyl, 2,2,2-trifluoroethyl, 1,1,2,2-tetrafluoroethyl, pentafluoroethyl, 1-chloroethyl, 2-chloroethyl, 1,1-dichloroethyl, 2,2-dichloroethyl, 2,2,2-trichloroethyl, 1,1,2,2-tetrachloroethyl, pentachloroethyl, 1-bromoethyl, 2-bromoethyl, 2,2,2-tribromoethyl, 1-iodoethyl, 2-iodoethyl, 2-chloro-2,2-difluoroethyl, 2,2-dichloro-2-fluoroethyl, 1-fluoropropyl, 2-fluoropropyl, 3-fluoropropyl, 1,1-difluoropropyl, 2,2-difluoropropyl, 3,3-difluoropropyl, 3,3,3-trifluoropropyl, 2,2,3,3,3-pentafluoropropyl, 1,1,2,3,3,3-hexafluoropropyl, heptafluoropropyl, 1-fluoro-2-propyl, 2-fluoro-2-propyl, 1,1-difluoro-2-propyl, 1,2-difluoro-2-propyl, 1,3-difluoro-2-propyl, 1,2,3-trifluoro-2-propyl, 1,1,3,3-tetrafluoro-2-propyl, 1,1,1,3,3,3-hexafluoro-2-propyl, heptafluoro-2-propyl, 1-chloropropyl, 2-chloropropyl, 3-chloropropyl, 1,1-dichloropropyl, 2,2-dichloropropyl, 3,3-dichloropropyl, 3,3,3-trichloropropyl, 2,2,3,3,3-pentachloropropyl, heptachloropropyl, 1-chloro-2-propyl, 2-chloro-2-propyl, 1,1-dichloro-2-propyl, 1,2-dichloro-2-propyl, 1,3-dichloro-2-propyl, 1,2,3-trichloro-2-propyl, 1,1,3,3-tetrachloro-2-propyl, 1,1,1,3,3,3-hexachloro-2-propyl, heptachloro-2-propyl, 1-bromopropyl, 2-bromopropyl, 3-bromopropyl, 1-bromo-2-propyl, 2-bromo-2-propyl, 1-iodopropyl, 2-iodopropyl, 3-iodopropyl, 1-iodo-2-propyl, 2-iodo-2-propyl, 1-fluorobutyl, 2-fluorobutyl, 3-fluorobutyl, 4-fluorobutyl, 4,4-difluorobutyl, 4,4,4-trifluorobutyl, 4,4,4-trifluoro-3-methylbutyl, 3,3,4,4,4-pentafluorobutyl, 2,2,3,4,4,4-hexafluorobutyl, 2,2,3,3,4,4,4-heptafluorobutyl, nonafluorobutyl, 1,1,1-trifluoro-2-butyl, 4,4,4-trifluoro-2-butyl, 3,3,4,4,4-pentafluoro-2-butyl, nonafluoro-2-butyl, 1,1,1,3,3,3-hexafluoro-2-(trifluoromethyl)-2-propyl, 1-chlorobutyl, 2-chlorobutyl, 3-chlorobutyl, 4-chlorobutyl, 4,4-dichlorobutyl, 4,4,4-trichlorobutyl, nonachlorobutyl, 1,1,1-trichloro-2-butyl, 4,4,4-trichloro-2-butyl, nonachloro-2-butyl, 1-bromobutyl, 2-bromobutyl, 3-bromobutyl, 4-bromobutyl, 1-iodobutyl, 2-iodobutyl, 3-iodobutyl, 4-iodobutyl, 4-chloro-1,1,2,2,3,3,4,4-octafluorobutyl, 4-bromo-1,1,2,2,3,3,4,4-octafluorobutyl, 1-fluoropentyl, 2-fluoropentyl, 3-fluoropentyl, 4-fluoropentyl, 5-fluoropentyl, 5,5,5-trifluoropentyl, 4,4,5,5,5-pentafluoropentyl, 3,3,4,4,5,5,5-heptafluoropentyl, 2,2,3,3,4,4,5,5-octafluoropentyl, 2,2,3,3,4,4,5,5,5-nonafluoropentyl, undecafluoropentyl, 1-chloropentyl, 2-chloropentyl, 3-chloropentyl, 4-chloropentyl, 5-chloropentyl, 5,5,5-trichloropentyl, 4,4,5,5,5-pentachloropentyl, 3,3,4,4,5,5,5-heptachloropentyl, 2,2,3,3,4,4,5,5,5-nonachloropentyl, undecachloropentyl, 1-bromopentyl, 2-bromopentyl, 3-bromopentyl, 4-bromopentyl, 5-bromopentyl, 5-iodopentyl, 1-fluorohexyl, 2-fluorohexyl, 3-fluorohexyl, 4-fluorohexyl, 5-fluorohexyl, 6-fluorohexyl, 6,6,6-trifluorohexyl, 5,5,6,6,6-pentafluorohexyl, 4,4,5,5,6,6,6-heptafluorohexyl, 3,3,4,4,5,5,6,6,6-nonafluorohexyl, 2,2,3,3,4,4,5,5,6,6,6-undecafluorohexyl, 2,2,3,3,4,4,5,5,6,6-decafluorohexyl, tridecafluorohexyl, 1-chlorohexyl, 2-chlorohexyl, 3-chlorohexyl, 4-chlorohexyl, 5-chlorohexyl, 6-chlorohexyl, 5-bromohexyl, 6-bromohexyl, 5-iodohexyl, and 6-iodohexyl groups.

In the present invention, unless otherwise specified, the "(C3-C6)cycloalkyl" refers to a cycloalkyl group having 3 to 6 carbon atoms, and the ring may be arbitrarily substituted with alkyl groups in the range of the designated number of carbon atoms. Examples thereof include cyclopropyl, 1-methylcyclopropyl, 2-methylcyclopropyl, 2,2-dimethylcyclopropyl, cyclobutyl, 1-methylcyclobutyl, cyclopentyl, 1-methylcyclopentyl, and cyclohexyl groups.

In the present invention, unless otherwise specified, the "(C3-C6)halocycloalkyl" refers to a cycloalkyl group having 3 to 6 carbon atoms and being substituted with 1 to 11 halogen atoms and which are identical or different, and the ring may be arbitrarily substituted with alkyl groups or haloalkyl groups within the specified range of carbon atoms. Examples thereof include 1-fluorocyclopropyl, 2-fluorocyclopropyl, 2,2-difluorocyclopropyl, 2,2,3,3-tetrafluorocyclopropyl, 1-chlorocyclopropyl, 2-chlorocyclopropyl, 2,2-dichlorocyclopropyl, 2,2,3,3-tetrachlorocyclopropyl, 1-bromocyclopropyl, 2,2-dibromocyclopropyl, 1-iodocyclopropyl, 1-fluoromethylcyclopropyl, 1-difluoromethylcyclopropyl, 1-trifluoromethylcyclopropyl, 1-chloromethylcyclopropyl, 1-bromomethylcyclopropyl, 2,2-diiodocyclopropyl, 1-fluorocyclobutyl, 2-fluorocyclobutyl, 3-fluorocyclobutyl, 3,3-difluorocyclobutyl, heptafluorocyclobutyl, 1-difluoromethylcyclobutyl, 1-trifluoromethylcyclobutyl, 2-chlorocyclobutyl, 3-chlorocyclobutyl, 3,3-dichlorocyclobutyl, 3,3-dibromocyclobutyl, 3,3-diiodocyclobutyl, 1-fluorocyclopentyl, 2-fluorocyclopentyl, 3-fluorocyclopentyl, 2,2-difluorocyclopentyl, 3,3-difluorocyclopentyl, nonafluorocyclopentyl, 2,2-dichlorocyclopentyl, 3,3-dichlorocyclopentyl, 2,2-dibromocyclopentyl, 3,3-dibromocyclopentyl, 2,2-diiodocyclopentyl, 3,3-diiodocyclopentyl, 1-fluorocyclohexyl, 2-fluorocyclohexyl, 3-fluorocyclohexyl, 4-fluorocyclohexyl, 2,2-difluorocyclohexyl, 3,3-difluorocyclohexyl, 4,4-difluorocyclohexyl, 1-chlorocyclohexyl, 2-chlorocyclohexyl, 3-chlorocyclohexyl, 4-chlorocyclohexyl, 2,2-dichlorocyclohexyl, 3,3-dichlorocyclohexyl, 4,4-dichlorocyclohexyl, 3,3-dibromocyclohexyl, 4,4-dibromocyclohexyl, 3,3-diiodocyclohexyl, and 4,4-diiodocyclohexyl groups.

In the present invention, unless otherwise specified, the "(C2-C6)alkenyl" refers to a linear or branched alkenyl group having 2 to 6 carbon atoms. Examples thereof include vinyl, 1-propenyl, isopropenyl, 2-propenyl, 1-butenyl, 1-methyl-1-propenyl, 2-butenyl, 1-methyl-2-propenyl, 3-butenyl, 2-methyl-1-propenyl, 2-methyl-2-propenyl, 1,3-butadienyl, 1-pentenyl, 1-ethyl-2-propenyl, 2-pentenyl, 1-methyl-1-butenyl, 3-pentenyl, 1-methyl-2-butenyl, 4-pentenyl, 1-methyl-3-butenyl, 3-methyl-1-butenyl, 1,2-dimethyl-2-propenyl, 1,1-dimethyl-2-propenyl, 2-methyl-2-butenyl, 3-methyl-2-butenyl, 1,2-dimethyl-1-propenyl, 2-methyl-3-butenyl, 3-methyl-3-butenyl, 1,3-pentadienyl, 1-vinyl-2-propenyl, 1-hexenyl, 1-propyl-2-propenyl, 2-hexenyl, 1-methyl-1-pentenyl, 1-ethyl-2-butenyl, 3-hexenyl, 4-hexenyl, 5-hexenyl, 1-methyl-4-pentenyl, 1-ethyl-3-butenyl, 1-(isobutyl)vinyl, 1-ethyl-1-methyl-2-propenyl, 1-ethyl-2-methyl-2-propenyl, 1-(isopropyl)-2-propenyl, 2-methyl-2-pentenyl, 3-methyl-3-pentenyl, 4-methyl-3-pentenyl, 1,3-dimethyl-2-butenyl, 1,1-dimethyl-3-butenyl, 3-methyl-4-pentenyl, 4-methyl-4-pentenyl, 1,2-dimethyl-3-butenyl, 1,3-dimethyl-3-butenyl, 1,1,2-trimethyl-2-propenyl, 1,5-hexadienyl, 1-vinyl-3-butenyl, and 2,4-hexadienyl groups.

In the present invention, unless otherwise specified, the "(C2-C6)haloalkenyl" refers to a linear or branched alkenyl group having 2 to 6 carbon atoms and being substituted with 1 to 11 halogen atoms and which are identical or different. Examples thereof include 1-fluorovinyl, 2-fluorovinyl, 1,2-difluorovinyl, 2,2-difluorovinyl, trifluorovinyl, 1-chlorovinyl, 2-chlorovinyl, 1,2-dichlorovinyl, 2,2-dichlorovinyl, trichlorovinyl, 1,2-dibromovinyl, 2,2-dibromovinyl, tribromovinyl, 1,2-diiodovinyl, 2,2-diiodovinyl, triiodovinyl, 1-fluoro-2-propenyl, 2-fluoro-2-propenyl, 3-fluoro-2-propenyl, 2,3-difluoro-2-propenyl, 3,3-difluoro-2-propenyl, 3,3-difluoro-1-propenyl, 2,3,3-trifluoro-2-propenyl, 3,3,3-trifluoro-1-propenyl, 2-chloro-3,3,3-trifluoro-1-propenyl, 1,2,3,3,3-pentafluoro-1-propenyl, 1-chloro-2-propenyl, 2-chloro-2-propenyl, 3-chloro-2-propenyl, 2,3-dichloro-2-propenyl, 3,3-dichloro-2-propenyl, 3,3-dichloro-1-propenyl, 2,3,3-trichloro-2-propenyl, 3,3,3-trichloro-1-propenyl, 3-bromo-2-propenyl, 3,3-dibromo-2-propenyl, 3,3-diiodo-2-propenyl, 2,2-difluoro-1-propene-2-yl, 3,3,3-trifluoro-1-propene-2-yl, 3,3,3-trichloro-1-propene-2-yl, 4-fluoro-3-butenyl, 4,4-difluoro-3-butenyl, 4,4-difluoro-3-butene-2-yl, 4,4,4-trifluoro-2-butenyl, 3,4,4-trifluoro-3-butenyl, 2-trifluoromethyl-2-propenyl, 2-trifluoromethyl-3,3-difluoro-2-propenyl, 4,4,4-trifluoro-3-chloro-2-butenyl, 4,4-dichloro-3-butenyl, 4,4,4-trichloro-2-butenyl, 2-trichloromethyl-2-propenyl, 5,5-difluoro-4-pentenyl, 4,5,5-trifluoro-4-pentenyl, 5,5,5-trifluoro-3-pentenyl, 4,4,4-trifluoro-3-methyl-2-butenyl, 4,4,4-trifluoro-3-trifluoromethyl-2-butenyl, 5,5-dichloro-4-pentenyl, 4,4,4-trichloro-3-methyl-2-butenyl, 6,6-difluoro-5-hexenyl, 5,6,6-trifluoro-5-pentenyl, 6,6,6-trifluoro-4-pentenyl, 5,5,5-trifluoro-4-methyl-3-pentenyl, 5,5,5-trifluoro-4-trifluoromethyl-3-pentenyl, 6,6-dichloro-5-hexenyl, and 5,5,5-trichloro-4-methyl-3-pentenyl groups.

In the present invention, unless otherwise specified, the "(C2-C6)alkynyl" refers to a linear or branched alkynyl group having 2 to 6 carbon atoms. Examples thereof include ethynyl, 1-propynyl, 2-propynyl, 1-butynyl, 1-methyl-2-propynyl, 2-butynyl, 3-butynyl, 1-pentynyl, 1-ethyl-2-propynyl, 2-pentynyl, 3-pentynyl, 1-methyl-2-butynyl, 4-pentynyl, 1-methyl-3-butynyl, 2-methyl-3-butynyl, 1-hexynyl, 1-(n-propyl)-2-propynyl, 2-hexynyl, 1-ethyl-2-butynyl, 3-hexynyl, 1-methyl-2-pentynyl, 1-methyl-3-pentynyl, 4-methyl-1-pentynyl, 3-methyl-1-pentynyl, 5-hexynyl, 1-ethyl-3-butynyl, 1-ethyl-1-methyl-2-propynyl, 1-(isopropyl)-2-propynyl, 1,1-dimethyl-2-butynyl, and 2,2-dimethyl-3-butynyl groups.

In the present invention, unless otherwise specified, the "(C2-C6)haloalkynyl" refers to a linear or branched alkynyl group having 2 to 6 carbon atoms and being substituted with 1 to 9 halogen atoms and which are identical or different. Examples thereof include fluoroethynyl, chloroethynyl, bromoethynyl, iodoethynyl, 3-fluoro-2-propynyl, 3-chloro-2-propynyl, 3-bromo-2-propynyl, 3-iodo-2-propynyl, 4-fluoro-3-butynyl, 4-chloro-3-butynyl, 4-bromo-3-butynyl, 4-iodo-3-butynyl, 4,4-difluoro-2-butynyl, 4,4-dichloro-2-butynyl, 4,4,4-trifluoro-2-butynyl, 4,4,4-trichloro-2-butynyl, 3-fluoro-1-methyl-2-propynyl, 3-chloro-1-methyl-2-propynyl, 5-fluoro-4-pentynyl, 5-chloro-4-pentynyl, 5,5,5-trifluoro-3-pentynyl, 5,5,5-trichloro-3-pentynyl, 4-fluoro-2-methyl-3-butynyl, 4-chloro-2-methyl-3-butynyl, 6-fluoro-5-hexynyl, 6-chloro-5-hexynyl, 6,6,6-trifluoro-4-hexynyl, 6,6,6-trichloro-4-hexynyl, 5-fluoro-3-methyl-4-pentynyl, and 5-chloro-3-methyl-4-pentynyl groups.

In the present invention, unless otherwise specified, the " (C1-C6) alkoxy" refers to a ((C₁-C₆)alkyl)-O- group in which the alkyl moiety is as defined above. Examples thereof include methoxy, ethoxy, n-propoxy, isopropoxy, n-butoxy, isobutoxy, sec-butoxy, tert-butoxy, n-pentoxy, 1-methylbutoxy, 2-methylbutoxy, 3-methylbutoxy, 1-ethylpropoxy, 1,1-dimethylpropoxy, 1,2-dimethylpropoxy, and n-hexyloxy groups.

In the present invention, unless otherwise specified, the "(C1-C6)haloalkoxy" refers to a ((C₁-C₆)haloalkyl)-O-group in which the haloalkyl moiety is as defined above. Examples thereof include difluoromethoxy, dichloromethoxy, trifluoromethoxy, trichloromethoxy, tribromomethoxy, chlorodifluoromethoxy, bromodifluoromethoxy, 2-fluoroethoxy, 1-chloroethoxy, 2-chloroethoxy, 1-bromoethoxy, 2-bromoethoxy, 2,2-difluoroethoxy, 1,2-dichloroethoxy, 2,2-dichloroethoxy, 2,2,2-trifluoroethoxy, 2,2,2-trichloroethoxy, 1,1,2,2-tetrafluoroethoxy, pentafluoroethoxy, 2-bromo-2-chloroethoxy, 2-chloro-1,1,2,2-tetrafluoroethoxy, 1-chloro-1,2,2,2-tetrafluoroethoxy, 1-chloropropoxy, 2-chloropropoxy, 3-chloropropoxy, 2-bromopropoxy, 3-bromopropoxy, 2-bromo-1-methylethoxy, 3-iodopropoxy, 2,3-dichloropropoxy, 2,3-dibromopropoxy, 3,3,3-trifluoropropoxy, 3,3,3-trifluoro-2-propoxy, 3,3,3-trichloropropoxy, 3-bromo-3,3-difluoropropoxy, 2,2-difluoropropoxy, 3,3-dichloro-3-fluoropropoxy, 2,2,3,3-tetrafluoropropoxy, 1-bromo-3,3,3-trifluoropropoxy, 2,2,3,3,3-pentafluoropropoxy, 2,2,2-trifluoro-1-trifluoromethylethoxy, heptafluoropropoxy, heptafluoro-2-propoxy, 1,2,2,2-tetrafluoro-1-trifluoromethylethoxy, 1,1,2,3,3,3-hexafluoropropoxy, 2-chlorobutoxy, 3-chlorobutoxy, 4-chlorobutoxy, 2-chloro-1,1-dimethylethoxy, 4-bromobutoxy, 3-bromo-2-methylpropoxy, 2-bromo-1,1-dimethylethoxy, 2,2-dichloro-1,1-dimethylethoxy, 2-chloro-1-chloromethyl-2-methylethoxy, 4,4,4-trifluorobutoxy, 3,3,3-trifluoro-1-methylpropoxy, 3,3,3-trifluoro-2-methylpropoxy, 2,3,4-trichlorobutoxy, 2,2,2-trichloro-1,1-dimethylethoxy, 4-chloro-4,4-difluorobutoxy, 4,4-dichloro-4-fluorobutoxy, 4-bromo-4,4-difluorobutoxy, 2,4-dibromo-4,4-difluorobutoxy, 3,4-dichloro-3,4,4-trifluorobutoxy, 3,3-dichloro-4,4,4-trifluorobutoxy, 4-bromo-3,3,4,4-tetrafluorobutoxy, 4-bromo-3-chloro-3,4,4-trifluorobutoxy, 2,2,3,3,4,4-hexafluorobutoxy, 2,2,3,4,4,4-hexafluorobutoxy, 2,2,2-trifluoro-1-methyl-1-trifluoromethylethoxy, 3,3,3-trifluoro-2-trifluoromethylpropoxy, 2,2,3,3,4,4,4-heptafluorobutoxy, 3,3,4,4,4-pentafluoro-2-butoxy, 2,3,3,3-tetrafluoro-2-trifluoromethylpropoxy, 1,1,2,2,3,3,4,4-octafluorobutoxy, nonafluorobutoxy, perfluoro-tert-butoxy, 4-chloro-1,1,2,2,3,3,4,4-octafluorobutoxy, 5,5,5-trifluoropentoxy, 4,4,5,5,5-pentafluoropentoxy, 3,3,4,4,5,5,5-heptafluoropentoxy, 3,3,4,4,5,5,5-heptafluoro-2-pentoxy, 2,2,3,3,4,4,5,5,5-nonafluoropentoxy, 2,2,3,3,4,4,5,5-octafluoropentoxy, perfluoropentoxy, 4,4,5,5,5-pentafluoro-2-butoxy, 2,2-bis(trifluoromethyl)propoxy, 2,2,3,3,4,4,5,5,6,6,6-undecafluorohexyloxy, 3,3,4,4,5,5,6,6,6-nonafluorohexyloxy, 4,4,5,5,6,6,6-heptafluorohexyloxy, 2,2,3,3,4,4,5,5,6,6-decafluorohexyloxy, 4,4,4-trifluoro-3,3-bis(trifluoromethyl)butyloxy, and perfluorohexyloxy groups.

In the present invention, unless otherwise specified, the "(C3-C6)cycloalkyl C1-C6 alkyl" refers to a ((C3-C6)cycloalkyl)-((C1-C6)alkyl)- group in which the cycloalkyl moiety and the alkyl moiety are as defined above. Examples thereof include cyclopropylmethyl, cyclobutylmethyl, cyclopentylmethyl, cyclohexylmethyl, 1-(cyclopropyl)ethyl, 2-(cyclopropyl)ethyl, and 1-(cyclopropyl)propyl groups.

In the present invention, unless otherwise specified, the "(C3-C6)halocycloalkyl (C1-C6)alkyl" refers to a ((C3-C6)halocycloalkyl)-((C1-C6)alkyl)- group in which the halocycloalkyl moiety and the alkyl moiety are as defined above. Examples thereof include 2,2-difluorocyclopropylmethyl, 2,2-dichlorocyclopropylmethyl, 1-(2,2-difluorocyclopropyl)ethyl, 2-(2,2-difluorocyclopropyl)ethyl, 1-(2,2-dichlorocyclopropyl)ethyl, 2-(2,2-dichlorocyclopropyl)ethyl, 2-(2,2-difluorocyclopropyl)propyl, 3,3-difluorocyclobutylmethyl, 2,2,3,3-tetrafluorocyclobutylmethyl, 2-(2,2,3,3-tetrafluorocyclobutyl)ethyl, 2-(3,3-difluorocyclobutyl)ethyl, 2-(3,3-difluorocyclobutyl)propyl, 3,3-difluorocyclopentylmethyl, and 2-(3,3-difluorocyclopentyl)propyl groups.

In the present invention, unless otherwise specified, the "phenyl (C1-C6)alkyl" refers to a (phenyl)-((C1-C6)alkyl)-group. Examples thereof include benzyl, 1-phenylethyl, 2-phenylethyl, 2-phenylpropyl, 2-phenylbutyl, and 1-phenylpentyl groups.

In the present invention, unless otherwise specified, the "heteroaryl" refers to a 5- or 6-membered monocyclic heterocyclic ring having 1 to 6 heteroatoms selected from a nitrogen atom, an oxygen atom, and a sulfur atom, in addition to a carbon atom. Examples thereof include thiophene-2-yl, thiophene-3-yl, furan-2-yl, furan-3-yl, pyrrole-1-yl, pyrrole-2-yl, pyrrole-3-yl, oxazole-2-yl, oxazole-4-yl, oxazole-5-yl, isoxazole-3-yl, isoxazole-4-yl, isoxazole-5-yl, thiazole-2-yl, thiazole-4-yl, thiazole-5-yl, isothiazole-3-yl, isothiazole-4-yl, isothiazole-5-yl, imidazole-1-yl, imidazole-2-yl, imidazole-4-yl, pyrazole-1-yl, pyrazole-3-yl, pyrazole-4-yl, pyrazole-5-yl, 1,3,4-oxadiazole-2-yl, 1,2,3-oxadiazole-4-yl, 1,2,3-oxadiazole-5-yl, 1,2,4-oxadiazole-3-yl, 1,2,4-oxadiazole-5-yl, 1,2,5-oxadiazole-3-yl, 1,3,4-thiadiazole-2-yl, 1,2,3-thiadiazole-4-yl, 1,2,3-thiadiazole-5-yl, 1,2,4-thiadiazole-3-yl, 1,2,4-thiadiazole-5-yl, 1,2,5-thiadiazole-3-yl, 1,2,4-triazole-3-yl, 1,2,4-triazole-1-yl, 1,2,3-triazole-1-yl, 1,2,3-triazole-2-yl, 1,3,4-triazole-1-yl, tetrazole-1-yl, tetrazole-2-yl, tetrazole-5-yl, pyridine-2-yl, pyridine-3-yl, pyridine-4-yl, pyrimidine-2-yl, pyrimidine-4-yl, pyrimidine-5-yl, pyridazine-3-yl, pyridazine-4-yl, pyrazine-2-yl, and 1,3,5-triazine-2-yl groups.

In the present invention, unless otherwise specified, the "heteroaryl (C1-C3)alkyl" refers to a (heteroaryl)-((C1-C3)alkyl)- group in which the heteroaryl moiety is as defined above. Examples thereof include (pyridine-2-yl)methyl, (pyridine-3-yl)methyl, (pyridine-4-yl)methyl, (pyrimidine-2-yl)methyl, (pyrimidine-4-yl)methyl, (thiazole-2-yl)methyl, (thiazole-4-yl)methyl, (thiazole-5-yl)methyl, (pyrazole-1-yl)methyl, (pyrazole-3-yl)methyl, (pyrazole-4-yl)methyl, and (pyrazole-5-yl)methyl groups.

In the present invention, unless otherwise specified, the "heteroaryl (C3-C6)cycloalkyl" refers to a (heteroaryl)-((C3-C6)cycloalkyl)- group in which the heteroaryl moiety and the cycloalkyl moiety are as defined above. Examples thereof include 1-(pyridine-2-yl)cyclopropyl, 1-(pyridine-3-yl)cyclopropyl, 1-(pyridine-4-yl)cyclopropyl, 1-(pyrimidine-2-yl)cyclopropyl, 1-(pyrimidine-4-yl)cyclopropyl, 1-(thiazole-2-yl)cyclopropyl, 1-(thiazole-4-yl)cyclopropyl, 1-(thiazole-5-yl)cyclopropyl, 1-(pyrazole-1-yl)cyclopropyl, 1-(pyrazole-3-yl)cyclopropyl, 1-(pyrazole-4-yl)cyclopropyl, and 1-(pyrazole-5-yl)cyclopropyl groups.

In the present invention, unless otherwise specified, the "heterocycloalkyl" refers to a 3- to 6-membered saturated heterocyclic ring containing one or more atoms selected from an oxygen atom, a nitrogen atom, and a sulfur atom, in addition to a carbon atom. Examples thereof include oxetane-2-yl, oxetane-3-yl, thietane-3-yl, tetrahydrofuran-2-yl, tetrahydrofuran-3-yl, tetrahydrothiophene-3-yl, tetrahydropyran-2-yl, tetrahydropyran-3-yl, tetrahydropyran-4-yl, tetrahydrothiopyran-4-yl, morpholine-4-yl, azetidine-1-yl, azetidine-3-yl, pyrrolidine-3-yl, and piperidine-4-yl groups.

In the present invention, unless otherwise specified, the "cyano heterocycloalkyl" refers to a (cyano)-(heterocycloalkyl)- group in which the heterocycloalkyl moiety is as defined above. Examples thereof include 3-cyanooxetane-3-yl, 3-cyanothietane-3-yl, 3-cyanotetrahydrofuran-3-yl, 3-cyanotetrahydrothiophene-3-yl, 3-cyanotetrahydropyran-3-yl, 4-cyanotetrahydrothiopyran-4-yl, 3-cyanoazetidine-3-yl, 3-cyanopyrrolidine-3-yl, and 4-cyanopiperidine-4-yl groups.

In the present invention, unless otherwise specified, the "heterocycloalkyl (C1-C6)alkyl" refers to a (3- to 6-membered heterocycloalkyl)-((C1-C6)alkyl)- group in which the heterocycloalkyl moiety and the alkyl moiety are as defined above. Examples thereof include (oxetane-3-yl)methyl, (tetrahydrofuran-2-yl)methyl, (tetrahydrofuran-3-yl)methyl, (tetrahydropyran-2-yl)methyl, (tetrahydropyran-3-yl)methyl, and (tetrahydropyran-4-yl)methyl groups.

In the present invention, unless otherwise specified, the "cyano (C1-C6)alkyl" refers to a (cyano)-((C1-C6)alkyl)- group in which the alkyl moiety is as defined above. Examples thereof include cyanomethyl, 1-cyanoethyl, 2-cyanoethyl, 1-cyanopropyl, 3-cyanopropyl, 2-cyano-2-propyl, 1-cyanobutyl, 4-cyanobutyl, 5-cyanopentyl, and 6-cyanohexyl groups.

In the present invention, unless otherwise specified, the "cyano (C3-C6)cycloalkyl" refers to a (cyano)-((C3-C6)cycloalkyl)- group in which the cycloalkyl moiety is as defined above. Examples thereof include 1-cyanocyclopropyl, 2-cyanocyclopropyl, 1-cyanomethylcyclopropyl, 1-cyanocyclobutyl, 3-cyanocyclobutyl, 1-cyanocyclopentyl, and 1-cyanocyclohexyl groups.

In the present invention, unless otherwise specified, the "hydroxy (C1-C6)alkyl" refers to a (hydroxy)-((C1-C6)alkyl)- group in which the alkyl moiety is as defined above. Examples thereof include hydroxymethyl, 1-hydroxyethyl, 2-hydroxyethyl, 1-hydroxypropyl, 2-hydroxypropyl, 3-hydroxypropyl, 1-hydroxybutyl, and 4-hydroxybutyl groups.

In the present invention, unless otherwise specified, the "(C1-C6)alkoxy (C1-C6)alkyl" refers to a ((C1-C6)alkoxy)-((C1-C6)alkyl)- group in which the alkoxy moiety and the alkyl moiety are as defined above. Examples thereof include methoxymethyl, 1-methoxyethyl, 2-methoxyethyl, 2-ethoxyethyl, 2-methoxypropyl, 3-methoxypropyl, 2-ethoxypropyl, 3-ethoxypropyl, 1-methyl-3-methoxybutyl, and 3-butoxybutyl groups.

In the present invention, unless otherwise specified, the "(C1-C6)alkylamino" refers to a ((C1-C6)alkyl)-NH-group in which the alkyl moiety is as defined above. Examples thereof include methylamino, ethylamino, n-propylamino, and isopropylamino groups.

In the present invention, unless otherwise specified, the "(C1-C6)alkylcarbonyl" refers to a ((C1-C6)alkyl)-C(=O)- group in which the alkyl moiety is as defined above. Examples thereof include acetyl, propionyl, 2-methylpropionyl, 2,2-dimethylpropionyl, butanoyl, pivaloyl, 2-methylbutanoyl, 3-methylbutanoyl, 2-ethylbutanoyl, 2,2-dimethylbutanoyl, 2,3-dimethylbutanoyl, 3,3-dimethylbutanoyl, pentanoyl, 2-methylpentanoyl, 3-methylpentanoyl, 4-methylpentanoyl, and hexanoyl groups.

In the present invention, unless otherwise specified, the "(C1-C6)alkoxy (C1-C6)alkylcarbonyl" refers to a ((C1-C6)alkyl)-O-((C1-C6)alkyl)-C(=O)- group in which the alkyl moiety is as defined above. Examples thereof include methoxyacetyl, ethoxyacetyl, propoxyacetyl, isopropoxyacetyl, butoxyacetyl, 2-methoxypropionyl, 3-methoxypropionyl, 2-ethoxypropionyl, 3-ethoxypropionyl, 2-methoxybutanoyl, 4-methoxybutanoyl, 2-methoxypentanoyl, and 5-methoxypentanoyl groups.

In the present invention, unless otherwise specified, the "(C3-C6)cycloalkylcarbonyl" refers to a ((C3-C6)cycloalkyl)-C(=O)- group in which the cycloalkyl moiety is as defined above. Examples thereof include cyclopropanecarbonyl, cyclobutanecarbonyl, cyclopentanecarbonyl, and cyclohexanecarbonyl groups.

In the present invention, unless otherwise specified, the "(C3-C6)cycloalkyl (C1-C6)alkylcarbonyl" refers to a ((C3-C6)cycloalkyl)-((C1-C6)alkyl)-C(=O)- group in which the cycloalkyl and alkyl moieties are as defined above. Examples thereof include 2-cyclopropylacetyl, 2-cyclobutylacetyl, 2-cyclopentylacetyl, 2-cyclohexylacetyl, 2-cyclopropylpropanol, and 2-cyclopropylbutanoyl groups.

In the present invention, unless otherwise specified, the "(C1-C6)alkoxycarbonyl" refers to a ((C1-C6)alkoxy)-C(=O)- group in which the alkoxy moiety is as defined above. Examples thereof include methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, n-pentoxycarbonyl, 1-methylbutoxycarbonyl, 2-methylbutoxycarbonyl, 3-methylbutoxycarbonyl, 1-ethylpropoxycarbonyl, 1,1-dimethylpropoxycarbonyl, 1,2-dimethylpropoxycarbonyl, and 2,2-dimethylpropoxycarbonyl groups.

In the present invention, unless otherwise specified, the "(C1-C6)alkylaminocarbonyl" refers to a ((C1-C6)alkyl)-NH-C(=O)- group in which the alkyl moiety is as defined above. Examples thereof include methylaminocarbonyl, ethylaminocarbonyl, n-propylaminocarbonyl, isopropylaminocarbonyl, n-butylaminocarbonyl, isobutylaminocarbonyl, sec-butylaminocarbonyl, tert-butylaminocarbonyl, n-pentylaminocarbonyl, 1-methylbutylaminocarbonyl, 2-methylbutylaminocarbonyl, 3-methylbutylaminocarbonyl, 1-ethylpropylaminocarbonyl, 1,1-dimethylpropylaminocarbonyl, 1,2-dimethylpropylaminocarbonyl, 2,2-dimethylpropylaminocarbonyl, and n-hexylaminocarbonyl groups.

In the present invention, unless otherwise specified, the "(C1-C6)alkoxycarbonyl (C1-C6)alkylamino" refers to a ((C1-C6)alkyl)-O-C(=O)-((C1-C6)alkyl)-NH- group in which the alkyl moiety is as defined above. Examples thereof include methoxycarbonylmethylamino, ethoxycarbonylmethylamino, tert-butoxycarbonylmethylamino, and 1-(tert-butoxycarbonyl)ethylamino groups.

In the present invention, unless otherwise specified, the "(C1-C6)alkoxycarbonylamino" refers to a ((C1-C6)alkyl)-O-C(=O)-NH- group in which the alkyl moiety is as defined above. Examples thereof include methoxycarbonylamino, ethoxycarbonylamino, isopropoxycarbonylamino, tert-butoxycarbonylamino, and isobutoxycarbonylamino groups.

In the present invention, unless otherwise specified, the "(C1-C6)alkylthio" refers to a ((C1-C6)alkyl)-S- group in which the alkyl moiety is as defined above. Examples thereof include methylthio, ethylthio, n-propylthio, isopropylthio, n-butylthio, isobutylthio, sec-butylthio, and tert-butylthio groups.

In the present invention, unless otherwise specified, the "((C1-C)alkylthio)carbonyl" refers to a ((C1-C)alkylthio)-C(=O)- group in which the alkylthio moiety is as defined above. Examples thereof include methylthiocarbonyl, ethylthiocarbonyl, and isopropylthiocarbonyl groups.

In the present invention, unless otherwise specified, the "((C1-C6)alkyl) thiocarbonyl" refers to a ((C1-C6)alkyl)-C(=S)- group in which the alkyl moiety is as defined above. Examples thereof include methyl(thiocarbonyl), ethyl(thiocarbonyl), n-propyl(thiocarbonyl), isopropyl(thiocarbonyl), n-butyl(thiocarbonyl), isobutyl(thiocarbonyl), sec-butyl(thiocarbonyl), tert-butyl(thiocarbonyl), and n-pentyl(thiocarbonyl) groups.

In the present invention, unless otherwise specified, the "((C1-C6)alkoxy) thiocarbonyl" refers to a ((C1-C6)alkyl)-O-C(=S)- group in which the alkyl moiety is as defined above. Examples thereof include methoxy(thiocarbonyl), ethoxy(thiocarbonyl), n-propoxy(thiocarbonyl), isopropoxy(thiocarbonyl), n-butoxy(thiocarbonyl), isobutoxy(thiocarbonyl), sec-butoxy(thiocarbonyl), tert-butoxy(thiocarbonyl), n-pentoxy(thiocarbonyl), 1-methylbutoxy(thiocarbonyl), 2-methylbutoxy(thiocarbonyl), 3-methylbutoxy(thiocarbonyl), 1-ethylpropoxy(thiocarbonyl), 1,1-dimethylpropoxy(thiocarbonyl), 1,2-dimethylpropoxy(thiocarbonyl), and 2,2-dimethylpropoxy(thiocarbonyl) groups.

In the present invention, unless otherwise specified, the "(C1-C6)alkylaminothiocarbonyl" refers to a ((C1-C6)alkyl)-NH-C(=S)- group in which the alkyl moiety is as defined above. Examples thereof include methylamino(thiocarbonyl), ethylamino(thiocarbonyl), n-propylamino(thiocarbonyl), isopropylamino(thiocarbonyl), n-butylamino(thiocarbonyl), isobutylamino(thiocarbonyl), sec-butylamino(thiocarbonyl), tert-butylamino(thiocarbonyl), n-pentylamino(thiocarbonyl), 1-methylbutylamino(thiocarbonyl), 2-methylbutylamino(thiocarbonyl), 3-methylbutylamino(thiocarbonyl), 1-ethylpropylamino(thiocarbonyl), 1,1-dimethylpropylamino(thiocarbonyl), 1,2-dimethylpropylamino(thiocarbonyl), 2,2-dimethylpropylamino(thiocarbonyl), and n-hexylamino(thiocarbonyl) groups.

In the present invention, unless otherwise specified, the "(C1-C6)alkylsulfonyl" refers to a ((C1-C6)alkyl)-S(=O)₂- group in which the alkyl moiety is as defined above. Examples thereof include methylsulfonyl, ethylsulfonyl, n-propylsulfonyl, isopropylsulfonyl, n-butylsulfonyl, isobutylsulfonyl, sec-butylsulfonyl, and tert-butylsulfonyl groups.

In the present invention, unless otherwise specified, the "(C1-C6)haloalkylsulfonyl" refers to a ((C1-C6)haloalkyl)-S(=O)₂- group in which the haloalkyl moiety is as defined above. Examples thereof include difluoromethylsulfonyl, trifluoromethylsulfonyl, trichloromethylsulfonyl, 2,2,2-trifluoroethylsulfonyl, pentafluoroethylsulfonyl, 3,3,3-trifluoropropylsulfonyl, heptafluoropropylsulfonyl, heptafluoro-2-propylsulfonyl, and nonafluorobutylsulfonyl groups.

In the present invention, unless otherwise specified, the "(C1-C6)alkylaminosulfonyl" refers to a ((C1-C6)alkyl)-NH-S(=O)₂- group in which the alkyl moiety is as defined above. Examples thereof include methylaminosulfonyl, ethylaminosulfonyl, n-propylaminosulfonyl, isopropylaminosulfonyl, n-butylaminosulfonyl, isobutylaminosulfonyl, sec-butylaminosulfonyl, tert-butylaminosulfonyl, n-pentylaminosulfonyl, 1-methylbutylaminosulfonyl, 2-methylbutylaminosulfonyl, 3-methylbutylaminosulfonyl, 1-ethylpropylaminosulfonyl, 1,1-dimethylpropylaminosulfonyl, 1,2-dimethylpropylaminosulfonyl, 2,2-dimethylpropylaminosulfonyl, and n-hexylaminosulfonyl groups.

In the present invention, unless otherwise specified, the "(C1-C6)alkoxycarbonyl (C1-C6)alkyl" refers to a ((C1-C6)alkoxy)-C(=O)-((C1-C6)alkyl)- group in which the alkoxy moiety and the alkyl moiety are as defined above. Examples thereof include methoxycarbonylmethyl, ethoxycarbonylmethyl, n-propoxycarbonylmethyl, isopropoxycarbonylmethyl, tert-butoxycarbonylmethyl, 1-(methoxycarbonyl)ethyl, 2-(methoxycarbonyl)ethyl, 1-(ethoxycarbonyl)ethyl, 2-(ethoxycarbonyl)ethyl, 1-(methoxycarbonyl)-1-methylethyl, 1-(ethoxycarbonyl)-1-methylethyl, and 2-(tert-butoxycarbonyl)ethyl groups.

In the present invention, unless otherwise specified, the "aminocarbonyl (C1-C6)alkyl" refers to a H₂N-C(=O)-((C1-C6)alkyl)- group in which the alkyl moiety is as defined above. Examples thereof include carbamoylmethyl, 1-carbamoylethyl, and 2-carbamoylethyl groups.

In the present invention, unless otherwise specified, the "(C1-C6)alkylaminocarbonyl (C1-C6)alkyl" refers to a ((C1-C6)alkyl)-NH-C(=O)-((C1-C6)alkyl)- group in which the alkyl moiety is as defined above. Examples thereof include N-methylcarbamoylmethyl, N-ethylcarbamoylmethyl, and N-(tert-butyl)carbamoylmethyl groups.

In the present invention, unless otherwise specified, the "(C3-C6)cycloalkylaminocarbonyl (C1-C6)alkyl" refers to a ((C3-C6)cycloalkyl)-NH-C(=O)-((C1-C6)alkyl)- group in which the cycloalkyl moiety and the alkyl moiety are as defined above. Examples thereof include N-cyclopropylcarbamoylmethyl, N-cyclobutylcarbamoylmethyl, N-cyclopentylcarbamoylmethyl, and N-cyclohexylcarbamoylmethyl groups.

In the present invention, unless otherwise specified, the "(C1-C6)haloalkylaminocarbonyl (C1-C6)alkyl" refers to a ((C1-C6)haloalkyl)-NH-C (=O)-((C1-C6)alkyl)- group in which the haloalkyl moiety and the alkyl moiety are as defined above. Examples thereof include N-(2,2-difluoroethyl)carbamoylmethyl, N-(2,2,2-trifluoroethyl)carbamoylmethyl, 1-[N-(2,2-difluoroethyl)carbamoyl]ethyl, 1-[N-(2,2,2-trifluoroethyl)carbamoyl]ethyl, 1-[N-(2,2-difluoroethyl)carbamoyl]-1-methylethyl, and 1-[N-(2,2,2-trifluoroethyl)carbamoyl]-1-methylethyl groups.

In the present invention, unless otherwise specified, the "(C1-C6)alkylcarbonyl (C1-C6)alkyl" refers to a ((C1-C6)alkyl)-C(=O)-((C1-C6)alkyl)- group in which the alkyl moiety is as defined above. Examples thereof include acetonyl, propionylmethyl, 2-methylpropionylmethyl, pivaloylmethyl, 2-acetylethyl, 2-propionylethyl, 2-(2-methylpropionyl)ethyl, 2-pivaloylethyl, 3-acetylpropyl, 3-propionylpropyl, 3-(2-methylpropionyl)propyl, and 3-pivaloylpropyl groups.

In the present invention, unless otherwise specified, the "(C1-C6)haloalkylcarbonyl (C1-C6)alkyl group" refers to a ((C1-C6)haloalkyl)-C(=O)-(C1-C6)alkyl)- group in which the haloalkyl moiety and the alkyl moiety are as defined above. Examples thereof include 2-oxo-3,3-difluoropropyl, 3-chloro-2-oxo-3,3-difluoropropyl, 2-oxo-3,3,3-trifluoropropyl, 3-oxo-4,4-difluorobutyl, 4-chloro-3-oxo-4,4-difluorobutyl, 3-oxo-4,4,4-trifluorobutyl, 3-oxo-4,4,5,5,5-pentafluoropentyl, 4-oxo-5,5,5-trifluoropentyl, and 4-oxo-5,5,6,6,6-pentafluorohexyl groups.

In the present invention, unless otherwise specified, the "hydroxyimino (C1-C6)alkyl" refers to a HO-N=((C1-C6)alkyl)- group in which the alkyl moiety is as defined above. Examples thereof include 1-(hydroxyimino)ethyl, 2-(hydroxyimino)ethyl, 1-(hydroxyimino)propyl, 2-(hydroxyimino)propyl, and 3-(hydroxyimino)propyl groups.

In the present invention, unless otherwise specified, the "(C1-C6)alkoxyimino" refers to a ((C1-C6)alkyl)-O-N=C-group in which the alkyl moiety is as defined above. Examples thereof include methoxyimino, ethoxyimino, and isopropoxyimino groups.

In the present invention, unless otherwise specified, the "(C1-C6)alkoxyimino (C1-C6)alkyl" refers to a ((C1-C6)alkyl)-O-N=C-((C1-C6)alkyl)- group in which the alkyl moiety is as defined above. Examples thereof include 1-(methoxyimino)ethyl, 2-(methoxyimino)ethyl, 1-(methoxyimino)propyl, 2-(methoxyimino)propyl, 3-(methoxyimino)propyl, 1-(ethoxyimino)ethyl, 2-(ethoxyimino)ethyl, 1-(ethoxyimino)propyl, 2-(ethoxyimino)propyl, 3-(ethoxyimino)propyl, 1-(isopropoxyimino)ethyl, and 2-(isopropoxyimino)ethyl groups.

In the present invention, unless otherwise specified, the "(C1-C6)haloalkoxyimino (C1-C6)alkyl" refers to a ((C1-C6)haloalkyl)-O-N=((C1-C6)alkyl)- group in which the haloalkyl moiety and the alkyl moiety are as defined above. Examples thereof include (2,2,2-trifluoroethoxyimino)methyl, 2-(2,2-difluoroethoxyimino)ethyl, 2-(2,2,2-trifluoroethoxyimino)ethyl, 2-(2,2,2-trifluoroethoxyimino)propyl, and 3-(2,2,2-trifluoroethoxyimino)propyl groups.

In the present invention, unless otherwise specified, the "(C1-C12)alkyl" refers to a linear or branched alkyl group having 1 to 12 carbon atoms. Examples thereof include, in addition to the examples of the (C1-C6)alkyl group, n-heptyl, n-octyl, n-nonyl, n-decyl, n-undecyl, n-dodecyl, 4,4-dimethylpentyl, 5-methylhexyl, 5,5-dimethylhexyl, 3,5,5-trimethylhexyl, 6-methylheptyl, 6,6-dimethylheptyl, 3,6,6-trimethylheptyl, 7-methyloctyl, 7,7-dimethyloctyl, 8-methylnonyl, 8,8-dimethylnonyl, 9-methyldecyl, 9,9-dimethyldecyl, and 10-methylundecyl groups.

In the present invention, unless otherwise specified, the "(C3-C6)cycloalkoxy" refers to a ((C3-C6)cycloalkyl)-O-group in which the cycloalkyl moiety is as defined above. Examples thereof include cyclopropoxy, cyclobutoxy, cyclopentyloxy, and cyclohexyloxy groups.

In the present invention, unless otherwise specified, the "(C3-C6)halocycloalkoxy" refers to a ((C3-C6)halocycloalkyl)-O- group in which the halocycloalkyl moiety is as defined above. Examples thereof include 2,2-difluorocyclopropoxy, 2,2-dichlorocyclopropoxy, 3,3-difluorocyclobutoxy, 3,3-dichlorocyclobutoxy, 3-fluorocyclopentyloxy, 3,3-difluorocyclopentyloxy, nonafluorocyclopentyloxy, 3,3-dichlorocyclopentyloxy, 4,4-difluorocyclohexyloxy, and 4,4-dichlorocyclohexyloxy groups.

In the present invention, unless otherwise specified, the "(C1-C6)alkoxy (C1-C6)alkoxy" refers to a ((C1-C6)alkoxy)-((C1-C6)alkoxy)- group in which the alkoxy moiety is as defined above. Examples thereof include 2-methoxyethoxy, 3-methoxypropoxy, 2-ethoxyisopropoxy, 2-isopropoxybutoxy, 5-ethoxypentyloxy, 6-ethoxyhexyloxy, 2-(tert-butoxy)ethoxy, 2-methoxyisopentyloxy, and 2-isopropoxyisobutoxy groups.

In the present invention, unless otherwise specified, the "(C1-C6)haloalkoxy (C1-C6)alkoxy" refers to a ((C1-C6)haloalkoxy)-((C1-C6)alkoxy)- group in which the haloalkoxy and alkoxy moieties are as defined above. Examples thereof include 2-difluoromethoxyethoxy, 2-trifluoromethoxyethoxy, 3-trifluoromethoxypropoxy, and 2-(2,2,2-trifluoroethoxy)ethoxy groups.

In the present invention, unless otherwise specified, the "(C1-C6)haloalkoxy (C1-C6)haloalkoxy" refers to a ((C1-C6)haloalkoxy)-((C1-C6)haloalkoxy)- group in which the haloalkoxy moiety is as defined above. Examples thereof include 2-(difluoromethoxy)-1,1,2,2-tetrafluoroethoxy, 2-(trifluoromethoxy)-1,1,2,2-tetrafluoroethoxy, 1,1,2,3,3,3-hexafluoro-2-(heptafluoropropoxy)propoxy, and 2-(2,2,2-trifluoroethoxy)-1,1,2,2-tetrafluoroethoxy groups.

In the present invention, unless otherwise specified, the "(C1-C6)haloalkylthio" refers to a ((C1-C6)haloalkyl)-S- group in which the haloalkyl moiety is as defined above. Examples thereof include fluoromethylthio, difluoromethylthio, trifluoromethylthio, trichloromethylthio, 2,2,2-trifluoroethylthio, pentafluoroethylthio, 2,2,2-trichloroethylthio, 3,3,3-trifluoropropylthio, heptafluoropropylthio, 1,1,1,3,3,3-hexafluoropropan-2-ylthio, heptafluoropropan-2-ylthio, and 4,4,4-trifluorobutylthio groups.

In the present invention, unless otherwise specified, the "(C2-C6)alkenylthio" refers to a ((C2-C6)alkenyl)-S-group in which the alkenyl moiety is as defined above. Examples thereof include vinylthio, 1-propenylthio, isopropenylthio, 2-propenylthio, 2-butenylthio, 3-butenylthio, 2-pentenylthio, 3-pentenylthio, 4-pentenylthio, 2-methyl-2-butenylthio, 2,4-pentadienylthio, 2-hexenylthio, 3-hexenylthio, 4-hexenylthio, 5-hexenylthio, and 2,4-hexadienylthio groups.

In the present invention, unless otherwise specified, the "(C2-C6)haloalkenylthio" refers to a ((C2-C6)haloalkenyl)-S- group in which the haloalkenyl moiety is as defined above. Examples of thereof include 2,2-difluorovinylthio, 2,2-dichlorovinylthio, 3,3-difluoro-2-propenylthio, 2,3,3-trifluoro-2-propenylthio, 3-chloro-2-propenylthio, 3,3-dichloro-2-propenylthio, 3-bromo-2-propenylthio, 4,4-difluoro-3-butenylthio, 4,4-difluoro-3-butene-2-ylthio, 3,4,4-trifluoro-3-butenylthio, 4,4,4-trifluoro-3-chloro-2-butenylthio, 4,4-dichloro-3-butenylthio, 4,5,5-trifluoro-4-pentenylthio, 5,5,5-trifluoro-3-pentenylthio, 4,4,4-trifluoro-3-trifluoromethyl-2-butenylthio, 6,6-difluoro-5-hexenylthio, 5,6,6-trifluoro-5-hexenylthio, and 6,6-dichloro-5-hexenylthio groups.

In the present invention, unless otherwise specified, the "(C3-C6)cycloalkylthio" refers to a ((C3-C6)cycloalkyl)-S- group in which the cycloalkyl moiety is as defined above. Examples thereof include cyclopropylthio, cyclobutylthio, cyclopentylthio, and cyclohexylthio groups.

In the present invention, unless otherwise specified, the "(C3-C6)halocycloalkylthio" refers to a ((C3-C6)halocycloalkyl)-S- group in which the halocycloalkyl moiety is as defined above. Examples thereof include 2,2-difluorocyclopropylthio, 2,2-dichlorocyclopropylthio, 3,3-difluorocyclobutylthio, 3,3-difluorocyclopentylthio, and 4,4-difluorocyclohexylthio groups.

In the present invention, unless otherwise specified, the "(C3-C6)cycloalkyl (C1-C6)alkylthio" refers to a ((C3-C6)cycloalkyl)-((C1-C6)alkyl)-S- group in which the cycloalkyl and alkyl moieties are as defined above. Examples thereof include cyclopropylmethylthio, 2-cyclopropylethylthio, 3-cyclopropylpropylthio, 4-cyclopropylbutylthio, 5-cyclopropylpentylthio, cyclobutylmethylthio, cyclopentylmethylthio, and cyclohexylmethylthio groups.

In the present invention, unless otherwise specified, the "(C3-C6)halocycloalkyl (C1-C6)alkylthio" refers to a ((C3-C6)halocycloalkyl)-((C1-C6)alkyl)-5- group in which the halocycloalkyl and alkyl moieties are as defined above. Examples thereof include 2,2-difluorocyclopropylmethylthio, 2,2-dichlorocyclopropylmethylthio, 2-(2,2-difluorocyclopropyl)ethylthio, 2-(2,2-dichlorocyclopropyl)ethylthio, 2,2-difluorocyclobutylmethylthio, and 4,4-difluorocyclohexylmethylthio groups.

In the present invention, unless otherwise specified, the "tri((C1-C6)alkyl)silyl (C1-C6)alkylthio" refers to a ((C1-C6)alkyl)₃Si-((C1-C6)alkyl)-S- group in which the alkyl moiety is as defined above, and the three alkyl groups of the ((C1-C6)alkyl)₃ moiety are identical to or different from each other. Examples thereof include trimethylsilylmethylthio, triethylsilylmethylthio, trimethylsilylethylthio, tert-butyldimethylsilylmethylthio, and trimethylsilylpropylthio groups.

In the present invention, unless otherwise specified, the "(C1-C6)alkylthio (C1-C6)alkoxy" refers to a ((C1-C6)alkyl)-S-((C1-C6)alkyl)-O- group in which the alkyl moiety is as defined above. Examples thereof include methylthiomethoxy, 2-methylthioethoxy, ethylthiomethoxy, 2-ethylthioethoxy, tert-butylthiomethoxy, and 2-(tert-butylthio)ethoxy groups.

In the present invention, unless otherwise specified, the "(C1-C6)haloalkylthio (C1-C6)alkoxy" refers to a ((C1-C6)haloalkyl)-S-((C1-C6)alkyl)-O- group in which the haloalkyl and alkyl moieties are as defined above. Examples thereof include fluoromethylthiomethoxy, difluoromethylthiomethoxy, trifluoromethylthiomethoxy, 1-(fluoromethylthio)ethoxy, 1-(difluoromethylthio)ethoxy, 1-(trifluoromethylthio)ethoxy, 2-(fluoromethylthio)ethoxy, 2-(difluoromethylthio)ethoxy, and 2-(trifluoromethylthio)ethoxy groups.

In the present invention, unless otherwise specified, the "(C1-C6)alkylsulfinyl" refers to a ((C1-C6)alkyl)-S(=O)- group in which the alkyl moiety is as defined above. Examples thereof include methylsulfinyl, ethylsulfinyl, n-propylsulfinyl, isopropylsulfinyl, n-butylsulfinyl, isobutylsulfinyl, sec-butylsulfinyl, and tert-butylsulfinyl groups.

In the present invention, unless otherwise specified, the "(C1-C6)haloalkylsulfinyl" refers to a ((C1-C6)haloalkyl)-S(=O)- group in which the haloalkyl moiety is as defined above. Examples thereof include difluoromethylsulfinyl, trifluoromethylsulfinyl, 2,2,2-trifluoroethylsulfinyl, 2,2,2-trichloroethylsulfinyl, pentafluoroethylsulfinyl, heptafluoropropylsulfinyl, trichloromethylsulfinyl, and heptafluoro-2-propylsulfinyl groups.

In the present invention, unless otherwise specified, the "(C3-C6)cycloalkylsulfinyl" refers to a ((C3-C6)cycloalkyl)-S(=O)- group in which the cycloalkyl moiety is as defined above. Examples thereof include cyclopropylsulfinyl, cyclobutylsulfinyl, cyclopentylsulfinyl, and cyclohexylsulfinyl groups.

In the present invention, unless otherwise specified, the "(C3-C6)halocycloalkylsulfinyl" refers to a ((C3-C6)halocycloalkyl)-S(=O)- group in which the halocycloalkyl moiety is as defined above. Examples thereof include 2,2-difluorocyclopropylsulfinyl, 2,2-dichlorocyclopropylsulfinyl, 3,3-difluorocyclobutylsulfinyl, 3,3-difluorocyclopentylsulfinyl, and 4,4-difluorocyclohexylsulfinyl groups.

In the present invention, unless otherwise specified, the "(C3-C6)cycloalkylsulfonyl" refers to a ((C3-C6)cycloalkyl)-S(=O)₂- group in which the cycloalkyl moiety is as defined above. Examples thereof include cyclopropylsulfonyl, cyclobutylsulfonyl, cyclopentylsulfonyl, and cyclohexylsulfonyl groups.

In the present invention, unless otherwise specified, the "(C3-C6)halocycloalkylsulfonyl" refers to a ((C3-C6)halocycloalkyl)-S(=O)₂- group in which the halocycloalkyl moiety is as defined above. Examples thereof include 2,2-difluorocyclopropylsulfonyl, 2,2-dichlorocyclopropylsulfonyl, 3,3-difluorocyclobutylsulfonyl, 3,3-difluorocyclopentylsulfonyl, and 4,4-difluorocyclohexylsulfonyl groups.

In the present invention, unless otherwise specified, the "(C1-C6)haloalkylcarbonyl" refers to a ((C1-C6)haloalkyl)-C(=O)- group in which the haloalkyl moiety is as defined above. Examples thereof include fluoroacetyl, difluoroacetyl, trifluoroacetyl, chloroacetyl, trichloroacetyl, tribromoacetyl, 3,3,3-trifluoropropionyl, 3,3-difluoropropionyl, and 4,4,4-trifluorobutyryl groups.

In the present invention, unless otherwise specified, the "(C1-C6)haloalkoxycarbonyl" refers to a ((C1-C6)haloalkyl)-O-C(=O)- group in which the haloalkyl moiety is as defined above. Examples thereof include 2-fluoroethoxycarbonyl, 2,2,2-trifluoroethoxycarbonyl, 2,2,2-trichloroethoxycarbonyl, pentafluoroethoxycarbonyl, 3,3,3-trifluoropropoxycarbonyl, and heptafluoro-2-propoxycarbonyl groups.

In the present invention, unless otherwise specified, the "(C1-C6)haloalkylaminocarbonyl" refers to a ((C1-C6)haloalkyl)-NH-C(=O)- group in which the haloalkyl moiety is as defined above. Examples thereof include 2-fluoroethylaminocarbonyl, 2,2,2-trifluoroethylaminocarbonyl, 2,2,2-trichloroethylaminocarbonyl, pentafluoroethylaminocarbonyl, and 1,1,1,3,3,3-hexafluoro-2-propylaminocarbonyl groups.

In the present invention, unless otherwise specified, the "(C1-C6)alkylaminocarbonylthio" refers to a ((C1-C6)alkyl)-NH-C(=O)-S- group in which the alkyl moiety is as defined above. Examples thereof include methylaminocarbonylthio, ethylaminocarbonylthio, propylaminocarbonylthio, and isopropylaminocarbonylthio groups.

In the present invention, unless otherwise specified, the "(C1-C6)haloalkylaminocarbonylthio" refers to a ((C1-C6)haloalkyl)-NH-C(=O)-S- group in which the haloalkyl moiety is as defined above. Examples thereof include 2-fluoroethylaminocarbonylthio, 2,2,2-trifluoroethylaminocarbonylthio, 2,2,2-trichloroethylaminocarbonylthio, pentafluoroethylaminocarbonylthio, and 1,1,1,3,3,3-hexafluoro-2-propylaminocarbonylthio groups.

In the present invention, unless otherwise specified, the "(C1-C6)alkylcarbonyloxy" refers to a ((C1-C6)alkyl)-C(=O)O- group in which the alkyl moiety is as defined above. Examples thereof include acetoxy and propionyloxy groups.

In the present invention, unless otherwise specified, the "(C1-C6)haloalkylcarbonyloxy" refers to a ((C1-C6)haloalkyl)-C(=O)O- group in which the haloalkyl moiety is as defined above. Examples thereof include fluoroacetoxy, difluoroacetoxy, trifluoroacetoxy, chloroacetoxy, trichloroacetoxy, tribromoacetoxy, 3,3,3-trifluoropropionyloxy, 3,3-difluoropropionyloxy, and 4,4,4-trifluorobutyryloxy groups.

In the present invention, unless otherwise specified, the "(C1-C6)alkylsulfinyloxy" refers to a ((C1-C6)alkyl)-S(=O)-O- group in which the alkyl moiety is as defined above. Examples thereof include methylsulfinyloxy, ethylsulfinyloxy, n-propylsulfinyloxy, and isopropylsulfinyloxy groups.

In the present invention, unless otherwise specified, the "(C1-C6)haloalkylsulfinyloxy" refers to a ((C1-C6)haloalkyl)-S(=O)-O- group in which the haloalkyl moiety is as defined above. Examples thereof include difluoromethylsulfinyloxy, trifluoromethylsulfinyloxy, 2,2,2-trifluoroethylsulfinyloxy, pentafluoroethylsulfinyloxy, heptafluoropropylsulfinyloxy, trichloromethylsulfinyloxy, and heptafluoro-2-propylsulfinyloxy groups.

In the present invention, unless otherwise specified, the "(C3-C6)cycloalkylsulfinyloxy" refers to a ((C3-C6)cycloalkyl)-S(=O)-O- group in which the cycloalkyl moiety is as defined above. Examples thereof include cyclopropylfinyloxy, cyclobutylsulfinyloxy, cyclopentylsulfinyloxy, and cyclohexylsulfinyloxy groups.

In the present invention, unless otherwise specified, the "(C3-C6)halocycloalkylsulfinyloxy" refers to a ((C3-C6)halocycloalkyl)-S(=O)-O- group in which the halocycloalkyl moiety is as defined above. Examples thereof include 2,2-difluorocyclopropylsulfinyloxy, 2,2-dichlorocyclopropylsulfinyloxy, 3,3-difluorocyclobutylsulfinyloxy, 3,3-difluorocyclopentylsulfinyloxy, and 4,4-difluorocyclohexylsulfinyloxy groups.

In the present invention, unless otherwise specified, the "(C1-C6)alkylsulfonyloxy" refers to a ((C1-C6)alkyl)-S(=O)₂-O- group in which the alkyl moiety is as defined above. Examples thereof include methylsulfonyloxy, ethylsulfonyloxy, n-propylsulfonyloxy, and isopropylsulfonyloxy groups.

In the present invention, unless otherwise specified, the "(C1-C6)haloalkylsulfonyloxy" refers to a ((C1-C6)haloalkyl)-S(=O)₂-O- group in which the haloalkyl moiety is as defined above. Examples thereof include difluoromethylsulfonyloxy, trifluoromethylsulfonyloxy, trichloromethylsulfonyloxy, 2,2,2-trifluoroethylsulfonyloxy, 2,2,2-trichloroethylsulfonyloxy, 3,3,3-trifluoropropylsulfonyloxy, and heptafluoro-2-propylsulfonyloxy groups.

In the present invention, unless otherwise specified, the "(C3-C6)cycloalkylsulfonyloxy" refers to a ((C3-C6)cycloalkyl)-S(=O)₂-O- group in which the cycloalkyl moiety is as defined above. Examples thereof include cyclopropylsulfonyloxy, cyclobutylsulfonyloxy, cyclopentylsulfonyloxy, and cyclohexylsulfonyloxy groups.

In the present invention, unless otherwise specified, the "(C3-C6)halocycloalkylsulfonyloxy" refers to a ((C3-C6)halocycloalkyl)-S(=O)₂-O- group in which the halocycloalkyl moiety is as defined above. Examples thereof include 2,2-difluorocyclopropylsulfonyloxy, 2,2-dichlorocyclopropylsulfonyloxy, 3,3-difluorocyclobutylsulfonyloxy, 3,3-difluorocyclopentylsulfonyloxy, and 4,4-difluorocyclohexylsulfonyloxy groups.

In the present invention, unless otherwise specified, the "(C1-C6)haloalkylamino" refers to a ((C1-C6)haloalkyl)-NH- group in which the haloalkyl moiety is as defined above. Examples thereof include 2-fluoroethylamino, 2,2-difluoroethylamino, 2,2,2-trifluoroethylamino, 2,2,2-trichloroethylamino, pentafluoroethylamino, 3,3,3-trifluoropropylamino, and 1,1,1,3,3,3-hexafluoro-2-propylamino groups.

In the present invention, unless otherwise specified, the "(C1-C6)alkylcarbonylamino" refers to a ((C1-C6)alkyl)-C(=O)-NH- group in which the alkyl moiety is as defined above. Examples thereof include acetylamino, propionylamino, butyrylamino, and isobutyrylamino groups.

In the present invention, unless otherwise specified, the "(C1-C6)haloalkylcarbonylamino" refers to a ((C1-C6)haloalkyl)-C(=O)-NH- group in which the haloalkyl moiety is as defined above. Examples thereof include fluoroacetylamino, difluoroacetylamino, trifluoroacetylamino, chloroacetylamino, trichloroacetylamino, tribromoacetylamino, 3,3,3-trifluoropropionylamino, pentafluoropropionylamino, and 3,3-difluoropropionylamino group.

In the present invention, unless otherwise specified, the "(C1-C6)haloalkoxycarbonylamino" refers to a ((C1-C6)haloalkoxy)-C(=O)-NH- group in which the haloalkoxy moiety is as defined above. Examples thereof include 2-fluoroethoxycarbonylamino, 2,2,2-trifluoroethoxycarbonylamino, 2,2,2-trichloroethoxycarbonylamino, pentafluoroethoxycarbonylamino, 3,3,3-trifluoropropoxycarbonylamino, and heptafluoro-2-propoxycarbonylamino groups.

In the present invention, unless otherwise specified, the "(C1-C6)alkylaminocarbonylamino" refers to a ((C1-C6)alkyl)-NH-C(=O)-NH- group in which the alkyl moiety is as defined above. Examples thereof include methylaminocarbonylamino, ethylaminocarbonylamino, n-propylaminocarbonylamino, isopropylaminocarbonylamino, and tert-butylaminocarbonylamino groups.

In the present invention, unless otherwise specified, the "(C1-C6)haloalkylaminocarbonylamino" refers to a ((C1-C6)haloalkyl)-NH-C(=O)-NH- group in which the haloalkyl moiety is as defined above. Examples thereof include 2-fluoroethylaminocarbonylamino, 2,2,2-trifluoroethylaminocarbonylamino, 2,2,2-trichloroethylaminocarbonylamino, pentafluoroethylaminocarbonylamino, and 1,1,1,3,3,3-hexafluoro-2-propylaminocarbonylamino groups.

In the present invention, unless otherwise specified, the "(C1-C6)alkylsulfinylamino" refers to a ((C1-C6)alkyl)-SO-NH- group in which the alkyl moiety is as defined above. Examples thereof include methylsulfinylamino, ethylsulfinylamino, n-propylsulfinylamino, isopropylsulfinylamino, and tert-butylsulfinylamino groups.

In the present invention, unless otherwise specified, the "(C1-C6)haloalkylsulfinylamino" refers to a ((C1-C6)haloalkyl)-S(=O)-NH- group in which the haloalkyl moiety is as defined above. Examples thereof include fluoromethylsulfinylamino, difluoromethylsulfinylamino, trifluoromethylsulfinylamino, chloromethylsulfinylamino, trichloromethylsulfinylamino, 2,2,2-trifluoroethylsulfinylamino, 2,2-difluoroethylsulfinylamino, and 3,3,3-trifluoropropylsulfinylamino groups.

In the present invention, unless otherwise specified, the "(C1-C6)alkylsulfonylamino" refers to a ((C1-C6)alkyl)-S(=O)₂-NH- group in which the alkyl moiety is as defined above. Examples thereof include methylsulfonylamino, ethylsulfonylamino, n-propylsulfonylamino, isopropylsulfonylamino, and tert-butylsulfonylamino groups.

In the present invention, unless otherwise specified, the "(C1-C6)haloalkylsulfonylamino" refers to a ((C1-C6)haloalkyl)-S(=O)₂-NH- group in which the haloalkyl moiety is as defined above. Examples thereof include fluoromethylsulfonylamino, difluoromethylsulfonylamino, trifluoromethylsulfonylamino, chloromethylsulfonylamino, trichloromethylsulfonylamino, 2,2,2-trifluoroethylsulfonylamino, 2,2-difluoroethylsulfonylamino, and 3,3,3-trifluoropropylsulfonylamino groups.

In the present invention, unless otherwise specified, the "tri((C1-C6)alkyl)silyl" refers to a ((C1-C6)alkyl)₃-Si- group in which the alkyl moiety is as defined above, and the three alkyl groups are optionally identical or different from each other. Examples thereof include trimethylsilyl, triethylsilyl, triisopropylsilyl, dimethylisopropylsilyl, and tert-butyldimethylsilyl groups.

In the present invention, unless otherwise specified, the "phenyl (C1-C6)alkoxy" refers to a phenyl-((C1-C6)alkyl)-O- group in which the alkyl moiety is as defined above. Examples thereof include phenylmethoxy, 1-phenylethoxy, 2-phenylethoxy, 1-phenylpropoxy, 2-phenylbutoxy, and 1-phenylpentoxy groups.

In the present invention, unless otherwise specified, the "phenyl (C1-C6)alkylthio" refers to a phenyl-((C1-C6)alkyl)-S- group in which the alkyl moiety is as defined above. Examples thereof include phenylmethylthio, 1-phenylethylthio, 2-phenylethylthio, 1-phenylpropylthio, 2-phenylbutylthio, and 1-phenylpentylthio groups.

In the present invention, unless otherwise specified, the "phenyl (C1-C6)alkylsulfinyl" refers to a phenyl-((C1-C6)alkyl)-S(=O)- group in which the alkyl moiety is as defined above. Examples thereof include phenylmethylsulfinyl, 1-phenylethylsulfinyl, 2-phenylethylsulfinyl, 1-phenylpropylsulfinyl, 2-phenylbutylsulfinyl, and 1-phenylpentylsulfinyl groups.

In the present invention, unless otherwise specified, the "phenyl (C1-C6)alkylsulfonyl" refers to a phenyl-((C1-C6)alkyl)-S(=O)₂- group in which the alkyl moiety is as defined above. Examples thereof include phenylmethylsulfonyl, 1-phenylethylsulfonyl, 2-phenylethylsulfonyl, 1-phenylpropylsulfonyl, 2-phenylbutylsulfonyl, and 1-phenylpentylsulfonyl groups.

In the present invention, unless otherwise specified, the "(C1-C6)alkylthio (C1-C6)alkyl" refers to a ((C1-C6)alkyl)-S-((C1-C6)alkyl)- group in which the alkyl moiety is as defined above. Examples thereof include methylthiomethyl, ethylthiomethyl, n-propylthiomethyl, isopropylthiomethyl, 1-(methylthio)ethyl, 2-(methylthio)ethyl, 2-(ethylthio)ethyl, 1-(n-propylthio)ethyl, 2-(n-propylthio)ethyl, 1-(isopropylthio)ethyl, 2-(isopropylthio)ethyl, 1-(methylthio)propyl, 2-(methylthio)propyl, 3-(methylthio)propyl, 1-(ethylthio)propyl, 2-(ethylthio)propyl, 3-(ethylthio)propyl, 1-(n-propylthio)propyl, 2-(n-propylthio)propyl, 3-(n-propylthio)propyl, 1-(methylthio)butyl, 2-(methylthio)butyl, 3-(methylthio)butyl, 4-(methylthio)butyl, 1-(methylthio)pentyl, 2-(methylthio)pentyl, 3-(methylthio)pentyl, 4-(methylthio)pentyl, 5-(methylthio)pentyl, 2-(n-butylthio)ethyl, 2-(isobutylthio)ethyl, 2-(sec-butylthio)ethyl, 2-(tert-butylthio)ethyl, pentylthiomethyl, and hexylthiomethyl groups.

In the present invention, unless otherwise specified, the "(C1-C6)haloalkylthio (C1-C6)alkyl" refers to a ((C1-C6) haloalkyl)-S-((C1-C6)alkyl)- group in which the haloalkyl moiety and the alkyl moiety are as defined above. Examples thereof include 2-(difluoromethylthio)ethyl, 2-(trifluoromethylthio)ethyl, 2-(2,2-difluoroethylthio)ethyl, 2-(2,2,2-trifluoroethylthio)ethyl, 2-(3,3-difluoropropylthio)ethyl, 2-(3,3,3-trifluoropropylthio)ethyl, 3-(difluoromethylthio)propyl, 3-(trifluoromethylthio)propyl, 3-(2,2-difluoroethylthio)propyl, 3-(2,2,2-trifluoroethylthio)propyl, 3-(3,3-difluoropropylthio)propyl, 3-(3,3,3-trifluoropropylthio)propyl, 4-(trifluoromethylthio)butyl, and 5-(trifluoromethylthio)pentyl groups.

In the present invention, unless otherwise specified, the "(C1-C6)haloalkoxy (C1-C6)alkyl" refers to a ((C1-C6)haloalkyl)-O-((C1-C6)alkyl)- group in which the haloalkoxy moiety and the alkyl moiety are as defined above. Examples thereof include 2-(difluoromethoxy)ethyl, 2-(trifluoromethoxy)ethyl, 2-(2,2-difluoroethoxy)ethyl, 2-(2,2,2-trifluoroethoxy)ethyl, 2-(3,3-difluoropropioxy)ethyl, 2-(3,3,3-trifluoropropioxy)ethyl, 3-(difluoromethoxy)propyl, 3-(trifluoromethoxy)propyl, 3-(2,2-difluoroethoxy)propyl, 3-(2,2,2-trifluoroethoxy)propyl, 3-(3,3-difluoropropioxy)propyl, 3-(3,3,3-trifluoropropioxy)propyl, 4-(trifluoromethoxy)butyl, and 5-(trifluoromethoxy)pentyl groups.

In the present invention, unless otherwise specified, the "cyano (C3-C6)cycloalkyl (C1-C6)alkyl" refers to a (cyano)-((C3-C6)cycloalkyl)-((C1-C6)alkyl group)- group in which the cycloalkyl moiety and the alkyl moiety are as defined above. Examples thereof include (1-cyanocyclopropane-1-yl)methyl, 2-(1-cyanocyclopropane-1-yl)ethyl, (1-cyanocyclobutane-1-yl)methyl, 2-(1-cyanocyclobutane-1-yl)ethyl, (1-cyanocyclopentane-1-yl)methyl, 2-(1-cyanocyclopentane-1-yl)ethyl, (1-cyanocyclohexane-1-yl)methyl, and 2-(1-cyanocyclohexane-1-yl)ethyl groups.

In the present invention, unless otherwise specified, the "hydroxy (C3-C6)cycloalkyl" refers to a (hydroxy)-(C3-C6)cycloalkyl)- group in which the cycloalkyl moiety is as defined above. Examples thereof include 2-(hydroxy)cyclopropyl, 1-(hydroxymethyl)cyclopropyl, 3-(hydroxy)cyclobutyl, 3-(hydroxy)cyclopentyl, 4-(hydroxy)cyclohexyl, 2-(hydroxy)cyclopropyl, 1-(hydroxymethyl)cyclopropyl, 3-(hydroxy)cyclobutyl, 3-(hydroxy)cyclopentyl, and 4-(hydroxy)cyclohexyl groups.

In the present invention, unless otherwise specified, the " (C1-C6)alkylcarbonyloxy (C3-C6)cycloalkyl" refers to a ((C1-C6)alkyl)-C(=O)O-((C3-C6)cycloalkyl)- group in which the alkyl moiety and the cycloalkyl moiety are as defined above. Examples thereof include 2-(acetoxy)cyclopropyl, 1-(acetoxymethyl)cyclopropyl, 3-(acetoxy)cyclobutyl, 3-(acetoxy)cyclopentyl, 4-(acetoxy)cyclohexyl, 2-(propionyloxy)cyclopropyl, 1-(propionyloxymethyl)cyclopropyl, 3-(propionyloxy)cyclobutyl, 3-(propionyloxy)cyclopentyl, and 4-(propionyloxy)cyclohexyl groups.

In the present invention, unless otherwise specified, the " (C1-C6)alkylsulfonyloxy (C3-C6)cycloalkyl" refers to a ((C1-C6)alkyl)-S(=O)₂-O-((C3-C6)cycloalkyl)- group in which the alkyl moiety is as defined above. Examples thereof include 2-(methylsulfonyloxy)cyclopropyl, 1-(methylsulfonyloxymethyl)cyclopropyl, 3-(methylsulfonyloxy)cyclobutyl, 3-(methylsulfonyloxy)cyclopentyl, 4-(methylsulfonyloxy)cyclohexyl, 2-(ethylsulfonyloxy)cyclopropyl, 1-(ethylsulfonyloxymethyl)cyclopropyl, 3-(ethylsulfonyloxy)cyclobutyl, 3-(ethylsulfonyloxy)cyclopentyl, and 4-(ethylsulfonyloxy)cyclohexyl groups.

In the present invention, unless otherwise specified, the "(C1-C6)alkoxy (C3-C6)cycloalkyl" refers to a ((C1-C6)alkoxy)-((C3-C6)cycloalkyl)- group in which the alkoxy moiety and the cycloalkyl moiety are as defined above. Examples thereof include 1-(methoxy)cyclopropyl, 1-(ethoxy)cyclopropyl, 1-(methoxymethyl)cyclopropyl, 1-(ethoxymethyl)cyclopropyl, 1-(n-propoxy)cyclopropyl, 1-(isopropoxy)cyclopropyl, 1-(methoxy)cyclobutyl, 1-(ethoxy)cyclobutyl, 1-(n-propoxy)cyclobutyl, 1-(isopropoxy)cyclobutyl, 1-(methoxy)cyclopentyl, 1-(ethoxy)cyclopentyl, 1-(methoxy)cyclohexyl, and 1-(ethoxy)cyclohexyl groups.

In the present invention, unless otherwise specified, the "(C1-C6)haloalkoxy (C3-C6)cycloalkyl" refers to a ((C1-C6)haloalkoxy)-((C3-C6)cycloalkyl)- group in which the haloalkoxy moiety and the cycloalkyl moiety are as defined above. Examples thereof include 1-(difluoromethoxy)cyclopropyl, 1-(trifluoromethoxy)cyclopropyl, 1-(2,2-difluoroethoxy)cyclopropyl, 1-(2,2,2-trifluoroethoxy)cyclopropyl, 1-(difluoromethoxymethyl)cyclopropyl, 1-(trifluoromethoxymethyl)cyclopropyl, 1-(2,2-difluoroethoxymethyl)cyclopropyl, 1-(2,2,2-trifluoroethoxymethyl)cyclopropyl, 1-(difluoromethoxy)cyclobutyl, 1-(trifluoromethoxy)cyclobutyl, 1-(2,2-difluoroethoxy)cyclobutyl, 1-(2,2,2-trifluoroethoxy)cyclobutyl, 1-(difluoromethoxy)cyclopentyl, 1-(trifluoromethoxy)cyclopentyl, 1-(2,2-difluoroethoxy)cyclopentyl, 1-(2,2,2-trifluoroethoxy)cyclopentyl, 1-(difluoromethoxy)cyclohexyl, 1-(trifluoromethoxy)cyclohexyl, 1-(2,2-difluoroethoxy)cyclohexyl, and 1-(2,2,2-trifluoroethoxy)cyclohexyl groups.

In the present invention, unless otherwise specified, the "(C1-C6)alkylthio (C3-C6)cycloalkyl" refers to a ((C1-C6)alkyl)-S-((C3-C6)cycloalkyl group)- group in which the alkyl moiety and the cycloalkyl moiety are as defined above. Examples thereof include 1-(methylthio)cyclopropyl, 1-(ethylthio)cyclopropyl, 1-(methylthiomethyl)cyclopropyl, 1-(ethylthiomethyl)cyclopropyl, 1-(n-propylthio)cyclopropyl, 1-(isopropylthio)cyclopropyl, 1-(pentylthio)cyclopropyl, 1-(hexylthio)cyclopropyl, 1-(methylthio)cyclobutyl, 1-(ethylthio)cyclobutyl, 1-(n-propylthio)cyclobutyl, 1-(isopropylthio)cyclobutyl, 1-(pentylthio)cyclobutyl, 1-(hexylthio)cyclobutyl, 1-(methylthio)cyclopentyl, 1-(ethylthio)cyclopentyl, 1-(methylthio)cyclohexyl, and 1-(ethylthio)cyclohexyl groups.

In the present invention, unless otherwise specified, the "(C1-C6)haloalkylthio (C3-C6)cycloalkyl" refers to a ((C1-C6)haloalkyl)-S-((C3-C6)cycloalkyl group)- group in which the haloalkyl moiety and the cycloalkyl moiety are as defined above. Examples thereof include 1-(difluoromethylthio)cyclopropyl, 1-(trifluoromethylthio)cyclopropyl, 1-(2,2-difluoroethylthio)cyclopropyl, 1-(2,2,2-trifluoroethylthio)cyclopropyl, 1-(difluoromethylthiomethyl)cyclopropyl, 1-(trifluoromethylthiomethyl)cyclopropyl, 1-(2,2-difluoroethylthiomethyl)cyclopropyl, 1-(2,2,2-trifluoroethylthiomethyl)cyclopropyl, 1-(difluoromethylthio)cyclobutyl, 1-(trifluoromethylthio)cyclobutyl, 1-(2,2-difluoroethylthio)cyclobutyl, 1-(2,2,2-trifluoroethylthio)cyclobutyl, 1-(difluoromethylthio)cyclopentyl, 1-(trifluoromethylthio)cyclopentyl, 1-(2,2-difluoroethylthio)cyclopentyl, 1-(2,2,2-trifluoroethylthio)cyclopentyl, 1-(difluoromethylthio)cyclohexyl, 1-(trifluoromethylthio)cyclohexyl, 1-(2,2-difluoroethylthio)cyclohexyl, and 1-(2,2,2-trifluoroethylthio)cyclohexyl groups.

In the present invention, unless otherwise specified, the " (C1-C6)alkylsulfinyl (C3-C6)cycloalkyl" refers to a ((C1-C6)alkyl)-S(=O)-((C3-C6)cycloalkyl group)- group in which the alkyl moiety and the cycloalkyl moiety are as defined above. Examples thereof include 1-(methylsulfinyl)cyclopropyl, 1-(ethylsulfinyl)cyclopropyl, 1-(methylsulfinylmethyl)cyclopropyl, 1-(ethylsulfinylmethyl)cyclopropyl, 1-(n-propylsulfinyl)cyclopropyl, 1-(isopropylsulfinyl)cyclopropyl, 1-(pentylsulfinyl)cyclopropyl, 1-(hexylsulfinyl)cyclopropyl, 1-(methylsulfinyl)cyclobutyl, 1-(ethylsulfinyl)cyclobutyl, 1-(n-propylsulfinyl)cyclobutyl, 1-(isopropylsulfinyl)cyclobutyl, 1-(pentylsulfinyl)cyclobutyl, 1-(hexylsulfinyl)cyclobutyl, 1-(methylsulfinyl)cyclopentyl, 1-(ethylsulfinyl)cyclopentyl, 1-(methylsulfinyl)cyclohexyl, and 1-(ethylsulfinyl)cyclohexyl groups.

In the present invention, unless otherwise specified, the "(C1-C6)haloalkylsulfinyl (C3-C6)cycloalkyl" refers to a ((C1-C6)haloalkyl)-S(=O)-((C3-C6)cycloalkyl group)- group in which the haloalkyl moiety and the cycloalkyl moiety are as defined above. Examples thereof include 1-(difluoromethylsulfinyl)cyclopropyl, 1-(trifluoromethylsulfinyl)cyclopropyl, 1-(2,2-difluoroethylsulfinyl)cyclopropyl, 1-(2,2,2-trifluoroethylsulfinyl)cyclopropyl, 1-(difluoromethylsulfinylmethyl)cyclopropyl, 1-(trifluoromethylsulfinylmethyl)cyclopropyl, 1-(2,2-difluoroethylsulfinylmethyl)cyclopropyl, 1-(2,2,2-trifluoroethylsulfinylmethyl)cyclopropyl, 1-(difluoromethylsulfinyl)cyclobutyl, 1-(trifluoromethylsulfinyl)cyclobutyl, 1-(2,2-difluoroethylsulfinyl)cyclobutyl, 1-(2,2,2-trifluoroethylsulfinyl)cyclobutyl, 1-(difluoromethylsulfinyl)cyclopentyl, 1-(trifluoromethylsulfinyl)cyclopentyl, 1-(2,2-difluoroethylsulfinyl)cyclopentyl, 1-(2,2,2-trifluoroethylsulfinyl)cyclopentyl, 1-(difluoromethylsulfinyl)cyclohexyl, 1-(trifluoromethylsulfinyl)cyclohexyl, 1-(2,2-difluoroethylsulfinyl)cyclohexyl, and 1-(2,2,2-trifluoroethylsulfinyl)cyclohexyl groups.

In the present invention, unless otherwise specified, the "(C1-C6)alkylsulfonyl (C3-C6) cycloalkyl" refers to a ((C1-C6)alkyl) -S(=O)₂-((C3-C6) cycloalkyl group)- group in which the alkyl moiety and the cycloalkyl moiety are as defined above. Examples thereof include 1-(methylsulfonyl)cyclopropyl, 1-(ethylsulfonyl)cyclopropyl, 1-(methylsulfonylmethyl)cyclopropyl, 1-(ethylsulfonylmethyl)cyclopropyl, 1-(n-propylsulfonyl)cyclopropyl, 1-(isopropylsulfonyl)cyclopropyl, 1-(pentylsulfonyl)cyclopropyl, 1-(hexylsulfonyl)cyclopropyl, 1-(methylsulfonyl)cyclobutyl, 1-(ethylsulfonyl)cyclobutyl, 1-(n-propylsulfonyl)cyclobutyl, 1-(isopropylsulfonyl)cyclobutyl, 1-(pentylsulfonyl)cyclobutyl, 1-(hexylsulfonyl)cyclobutyl, 1-(methylsulfonyl)cyclopentyl, 1-(ethylsulfonyl)cyclopentyl, 1-(methylsulfonyl)cyclohexyl, and 1-(ethylsulfonyl)cyclohexyl groups.

In the present invention, unless otherwise specified, the "(C1-C6)haloalkylsulfonyl (C3-C6)cycloalkyl" refers to a ((C1-C6)haloalkyl)-S(=O)₂-((C3-C6)cycloalkyl group)-group in which the haloalkyl moiety and the cycloalkyl moiety are as defined above. Examples thereof include 1-(difluoromethylsulfonyl)cyclopropyl, 1-(trifluoromethylsulfonyl)cyclopropyl, 1-(2,2-difluoroethylsulfonyl)cyclopropyl, 1-(2,2,2-trifluoroethylsulfonyl)cyclopropyl, 1-(difluoromethylsulfonylmethyl)cyclopropyl, 1-(trifluoromethylsulfonylmethyl)cyclopropyl, 1-(2,2-difluoroethylsulfonylmethyl)cyclopropyl, 1-(2,2,2-trifluoroethylsulfonylmethyl)cyclopropyl, 1-(difluoromethylsulfonyl)cyclobutyl, 1-(trifluoromethylsulfonyl)cyclobutyl, 1-(2,2-difluoroethylsulfonyl)cyclobutyl, 1-(2,2,2-trifluoroethylsulfonyl)cyclobutyl, 1-(difluoromethylsulfonyl)cyclopentyl, 1-(trifluoromethylsulfonyl)cyclopentyl, 1-(2,2-difluoroethylsulfonyl)cyclopentyl, 1-(2,2,2-trifluoroethylsulfonyl)cyclopentyl, 1-(difluoromethylsulfonyl)cyclohexyl, 1-(trifluoromethylsulfonyl)cyclohexyl, 1-(2,2-difluoroethylsulfonyl)cyclohexyl, and 1-(2,2,2-trifluoroethylsulfonyl)cyclohexyl groups.

In the present invention, unless otherwise specified, the "(C1-C6)alkoxycarbonyl (C3-C6)cycloalkyl" refers to a ((C1-C6)alkyl)-O-C(=O)-((C3-C6)cycloalkyl)- group in which the alkyl moiety and the cycloalkyl moiety are as defined above. Examples thereof include 1-(methoxycarbonyl)cyclopropyl, 1-(ethoxycarbonyl)cyclopropyl, 2-(methoxycarbonyl)cyclopropyl, 2-(ethoxycarbonyl)cyclopropyl, 1-(methoxycarbonyl)cyclobutyl, 1-(ethoxycarbonyl)cyclobutyl, 1-(methoxycarbonyl)cyclopentyl, 1-(ethoxycarbonyl)cyclopentyl, 1-(methoxycarbonyl)cyclohexyl, 1-(ethoxycarbonyl)cyclohexyl, 1-(isopropoxycarbonyl)cyclohexyl, and 1-(tert-butoxycarbonyl)cyclohexyl groups.

In the present invention, unless otherwise specified, the "carboxy (C3-C6)cycloalkyl" refers to a (carboxy)-((C3-C6)cycloalkyl group)- group in which the cycloalkyl moiety is as defined above. Examples thereof include 1-(carboxy)cyclopropyl, 2-(carboxy)cyclopropyl, 1-(carboxy)cyclobutyl, 1-(carboxy)cyclopentyl, and 1-(carboxy)cyclohexyl groups.

In the present invention, unless otherwise specified, the "carbamoyl (C3-C6)cycloalkyl" refers to a (carbamoyl)-((C3-C6)cycloalkyl group)- group in which the cycloalkyl moiety is as defined above. Examples thereof include 1-(carbamoyl)cyclopropyl, 2-(carbamoyl)cyclopropyl, 1-(carbamoyl)cyclobutyl, 1-(carbamoyl)cyclopentyl, and 1-(carbamoyl)cyclohexyl groups.

In the present invention, unless otherwise specified, the "(C1-C6)alkylaminocarbonyl (C3-C6)cycloalkyl" refers to a ((C1-C6)alkyl)-NH-C(=O)-((C3-C6)cycloalkyl)- group in which the alkyl moiety and the cycloalkyl moiety are as defined above. Examples thereof include 1-(methylaminocarbonyl)cyclopropyl, 1-(ethylaminocarbonyl)cyclopropyl, 1-(n-propylaminocarbonyl)cyclopropyl, 1-(isopropylaminocarbonyl)cyclopropyl, 1-(tert-butylaminocarbonyl)cyclopropyl, 1-(methylaminocarbonyl)cyclobutyl, 1-(methylaminocarbonyl)cyclopentyl, and 1-(methylaminocarbonyl)cyclohexyl groups.

In the present invention, unless otherwise specified, the "heterocycloalkyl (C3-C6)cycloalkyl" refers to a (3- to 6-membered heterocycloalkyl)-((C3-C6)cycloalkyl group)-group in which the heterocycloalkyl moiety and the cycloalkyl moiety are as defined above. Examples thereof include 1-(oxetane-2-yl)cyclopropyl, 1-(oxetane-3-yl)cyclopropyl, 1-(tetrahydrofuran-2-yl)cyclopropyl, 1-(tetrahydrofuran-3-yl)cyclopropyl, 1-(tetrahydrothiophene-3-yl)cyclopropyl, 1-(tetrahydropyran-2-yl)cyclopropyl, 1-(tetrahydropyran-3-yl)cyclopropyl, 1-(tetrahydropyran-4-yl)cyclopropyl, 1-(tetrahydrothiopyran-4-yl)cyclopropyl, 1-(morpholine-4-yl)cyclopropyl, 1-(azetidine-3-yl)cyclopropyl, 1-(pyrrolidine-3-yl)cyclopropyl, and 1-(piperidine-4-yl)cyclopropyl groups.

In the present invention, unless otherwise specified, the "(C4-C8)bicycloalkyl" refers to a bicyclic cyclic alkyl group having 4 to 8 carbon atoms. Examples thereof include bicyclo[1.1.0]butane-2-yl, bicyclo[1.1.1]pentane-1-yl, bicyclo [2.1.0]pentane-2-yl, and bicyclo[2.1.0]pentane-5-yl groups.

In the present invention, unless otherwise specified, the "(C1-C6)alkylsulfinyl (C1-C6)alkyl" refers to a ((C1-C6)alkylsulfinyl)-((C1-C6)alkyl)- group in which the alkylsulfinyl moiety and the alkyl moiety are as defined above. Examples thereof include methylsulfinylmethyl, ethylsulfinylmethyl, n-propylsulfinylmethyl, isopropylsulfinylmethyl, 1-(methylsulfinyl)ethyl, 2-(methylsulfinyl)ethyl, 2-(ethylsulfinyl)ethyl, 1-(n-propylsulfinyl)ethyl, 2-(n-propylsulfinyl)ethyl, 1-(isopropylsulfinyl)ethyl, 2-(isopropylsulfinyl)ethyl, 1-(methylsulfinyl)propyl, 2-(methylsulfinyl)propyl, 3-(methylsulfinyl)propyl, 1-(ethylsulfinyl)propyl, 2-(ethylsulfinyl)propyl, 3-(ethylsulfinyl)propyl, 1-(n-propylsulfinyl)propyl, 2-(n-propylsulfinyl)propyl, 3-(n-propylsulfinyl)propyl, 1-(methylsulfinyl)butyl, 2-(methylsulfinyl)butyl, 3-(methylsulfinyl)butyl, 4-(methylsulfinyl)butyl, 1-(methylsulfinyl)pentyl, 2-(methylsulfinyl)pentyl, 3-(methylsulfinyl)pentyl, 4-(methylsulfinyl)pentyl, 5-(methylsulfinyl)pentyl, 2-(n-butylsulfinyl)ethyl, 2-(isobutylsulfinyl)ethyl, 2-(sec-butylsulfinyl)ethyl, 2-(tert-butylsulfinyl)ethyl, pentylsulfinylmethyl, and hexylsulfinylmethyl groups.

In the present invention, unless otherwise specified, the "(C1-C6)alkylsulfonyl (C1-C6)alkyl" refers to a ((C1-C6)alkylsulfonyl)-((C1-C6)alkyl)- group in which the alkylsulfonyl moiety and the alkyl moiety are as defined above. Examples thereof include methylsulfonylmethyl, ethylsulfonylmethyl, n-propylsulfonylmethyl, isopropylsulfonylmethyl, 1-(methylsulfonyl)ethyl, 2-(methylsulfonyl)ethyl, 2-(ethylsulfonyl)ethyl, 1-(n-propylsulfonyl)ethyl, 2-(n-propylsulfonyl)ethyl, 1-(isopropylsulfonyl)ethyl, 2-(isopropylsulfonyl)ethyl, 1-(methylsulfonyl)propyl, 2-(methylsulfonyl)propyl, 3-(methylsulfonyl)propyl, 1-(ethylsulfonyl)propyl, 2-(ethylsulfonyl)propyl, 3-(ethylsulfonyl)propyl, 1-(n-propylsulfonyl)propyl, 2-(n-propylsulfonyl)propyl, 3-(n-propylsulfonyl)propyl, 1-(methylsulfonyl)butyl, 2-(methylsulfonyl)butyl, 3-(methylsulfonyl)butyl, 4-(methylsulfonyl)butyl, 1-(methylsulfonyl)pentyl, 2-(methylsulfonyl)pentyl, 3-(methylsulfonyl)pentyl, 4-(methylsulfonyl)pentyl, 5-(methylsulfonyl)pentyl, 2-(n-butylsulfonyl)ethyl, 2-(isobutylsulfonyl)ethyl, 2-(sec-butylsulfonyl)ethyl, 2-(tert-butylsulfonyl)ethyl, pentylsulfonylmethyl, and hexylsulfonylmethyl groups.

In the present invention, unless otherwise specified, the "(C1-C6)alkoxy (C1-C6)alkoxy (C1-C6)alkyl" refers to a ((C1-C6)alkoxy)-((C1-C6)alkoxy)-((C1-C6)alkyl)- group in which the alkoxy moiety and the alkyl moiety are as defined above. Examples thereof include (2-methoxyethoxy)methyl and (2-ethoxyethoxy)methyl groups.

In the present invention, unless otherwise specified, the "cyano (C1-C6)alkoxy (C1-C6)alkyl" refers to a (cyano)-((C1-C6)alkoxy)-((C1-C6)alkyl)- group in which the alkoxy moiety and the alkyl moiety are as defined above. Examples thereof include a (2-cyanoethoxy)methyl group.

In the present invention, unless otherwise specified, the "(C1-C6)alkylthio (C1-C6)alkoxy (C1-C6)alkyl" refers to a ((C1-C6)alkylthio)-((C1-C6)alkoxy)-((C1-C6)alkyl)- group in which the alkylthio moiety, the alkoxy moiety, and the alkyl moiety are as defined above. Examples thereof include (2-methylthioethoxy)methyl and (2-ethylthioethoxy)methyl groups.

In the present invention, unless otherwise specified, the "(C1-C6)alkylsulfinyl (C1-C6)alkoxy (C1-C6)alkyl" refers to a ((C1-C6)alkylsulfinyl)-((C1-C6)alkoxy)-((C1-C6)alkyl)- group in which the alkylsulfinyl moiety, the alkoxy moiety, and the alkyl moiety are as defined above. Examples thereof include (2-methylsulfinylethoxy)methyl and (2-ethylsulfinylethoxy)methyl groups.

In the present invention, unless otherwise specified, the "(C1-C6) alkylsulfonyl (C1-C6)alkoxy (C1-C6)alkyl" refers to a ((C1-C6)alkylsulfonyl)-((C1-C6)alkoxy)-((C1-C6)alkyl)- group in which the alkylsulfonyl moiety, the alkoxy moiety, and the alkyl moiety are as defined above. Examples thereof include (2-methylsulfonylethoxy)methyl and (2-ethylsulfonylethoxy)methyl groups.

In the present invention, unless otherwise specified, the "(C1-C6)alkylcarbonyloxy (C1-C6)alkyl" refers to a ((C1-C6)alkyl)-C(=O)O-((C1-C6)alkyl)- group in which the alkyl moiety is as defined above. Examples thereof include acetoxymethyl, propionyloxymethyl, isobutyryloxymethyl, and pivaloyloxymethyl groups.

In the present invention, unless otherwise specified, the "(C1-C6)alkoxycarbonyloxy (C1-C6)alkyl" refers to a ((C1-C6)alkyl)-O-C(=O)-O-((C1-C6)alkyl)- group in which the alkyl moiety is as defined above. Examples thereof include methoxycarbonyloxymethyl, ethoxycarbonyloxymethyl, isopropoxycarbonyloxymethyl, tert-butoxycarbonyloxymethyl, and isobutoxycarbonyloxymethyl groups.

In the present invention, unless otherwise specified, the "(C1-C6)alkoxycarbonylamino (C1-C6)alkyl" refers to a ((C1-C6)alkyl)-O-C(=O)-NH-((C1-C6)alkyl)- group in which the alkyl moiety is as defined above. Examples thereof include methoxycarbonylaminomethyl, ethoxycarbonylaminomethyl, isopropoxycarbonylaminomethyl, tert-butoxycarbonylaminomethyl, and isobutoxycarbonylaminomethyl groups.

In the present invention, unless otherwise specified, the "(C2-C6)alkynyl (C3-C6)cycloalkyl" refers to a ((C2-C6)alkynyl)-((C1-C6)cycloalkyl)- group in which the alkynyl moiety and the cycloalkyl moiety are as defined above. Examples thereof include 1-(ethynyl)cyclopropyl, 1-(1-propynyl)cyclopropyl, 1-(2-propynyl)cyclopropyl, 1-(1-butynyl)cyclopropyl, 1-(2-butynyl)cyclopropyl, 1-(3-butynyl)cyclopropyl, and 1-(1-pentynyl)cyclopropyl groups.

In the present invention, unless otherwise specified, the "(C2-C6)alkenyloxycarbonyl" refers to a ((C1-C6)alkenyl)-O-C(=O)- group in which the alkenyl moiety is as defined above. Examples thereof include 1-propenyloxycarbonyl, isopropenyloxycarbonyl, 2-propenyloxycarbonyl, 2-butenyloxycarbonyl, 3-butenyloxycarbonyl, 2-pentenyloxycarbonyl, 3-pentenyloxycarbonyl, 4-pentenyloxycarbonyl, 2-methyl-2-butenyloxycarbonyl, 2,4-pentadienyloxycarbonyl, 2-hexenyloxycarbonyl, 3-hexenyloxycarbonyl, 4-hexenyloxycarbonyl, 5-hexenyloxycarbonyl, and 2,4-hexadienyloxycarbonyl groups.

In the present invention, unless otherwise specified, the "tri((C1-C6)alkyl)silyl (C1-C6)alkyl" refers to a ((C1-C6)alkyl)₃-Si-((C1-C6)alkyl)- group in which the tri(C₁-C₆ alkyl)silyl moiety and the alkyl moiety are as defined above, and three alkyl groups of the ((C1-C6)alkyl)₃ moiety are optionally identical or different from each other. Examples thereof include trimethylsilylmethyl, 1-trimethylsilylethyl, 1-trimethylsilylpropyl, 1-trimethylsilylbutyl, 1-trimethylsilylpentyl, 1-trimethylsilylhexyl, triethylsilylmethyl, 1-triethylsilylethyl, triisopropylsilylmethyl, 1-triisopropylsilylethyl, dimethylisilylmethyl, and tert-butyldimethylsilylmethyl groups.

In the present invention, unless otherwise specified, the "(C1-C6)alkoxymethyl" refers to a ((C1-C6)alkoxy)-methyl group in which the alkoxy moiety is as defined above. Examples thereof include methoxymethyl, ethoxymethyl, n-propoxymethyl, isopropoxymethyl, n-butoxymethyl, isobutoxymethyl, sec-butoxymethyl, tert-butoxymethyl, n-pentoxymethyl, 1-methylbutoxymethyl, 2-methylbutoxymethyl, 3-methylbutoxymethyl, 1-ethylpropoxymethyl, 1,1-dimethylpropoxymethyl, 1,2-dimethylpropoxymethyl, and 2,2-dimethylpropoxymethyl groups.

In the present invention, unless otherwise specified, the "(C1-C12)alkylene" refers to alkylene having 1 to 12 carbon atoms. Examples thereof include methylene, ethylene, n-propylene, n-butylene, n-hexylene, n-heptylene, n-octylene, and n-dodecylene.

In the present invention, unless otherwise specified, the "(C3-C6)cycloalkylene" refers to cycloalkylene having 3 to 6 carbon atoms. Examples thereof include cyclopropylene, cyclobutylene, cyclopentylene, and cyclohexylene.

In the present invention, unless otherwise specified, the "(C1-C6)alkyleneoxy (C1-C12)alkylene" refers to ((C1-C6)alkylene)-O-(C₁-C₁₂ alkylene) in which the alkylene moiety is as defined above. Examples thereof include methyleneoxymethylene (-CH₂-O-CH₂-), methyleneoxyethylene (-CH₂-O-CH₂CH₂-), and ethyleneoxyethylene (-CH₂CH₂-O-CH₂CH₂-).

In the present invention, unless otherwise specified, the " (C2-C6)alkenylene" refers to alkenylene having 2 to 6 carbon atoms. Examples thereof include vinylene, propenylene, and butenylene.

In the present invention, unless otherwise specified, the " (C2-C6)alkynylene" refers to alkynylene having 2 to 6 carbon atoms. Examples thereof include ethynylene, propynylene, butynylene, and pentynylene.

In the present invention, "optionally mono-substituted or poly-substituted with R⁶" means "optionally substituted with one or two or more R⁶". When the group is substituted with two or more R⁶, the two or more R⁶ are identical to or different from each other, and each represent a halogen atom; a (C1-C6)alkyl; a (C1-C6)haloalkyl; a (C3-C6)cycloalkyl; a (C3-C6)halocycloalkyl; a hydroxy; a (C1-C6)alkoxy; a (C1-C6)haloalkoxy; a (C3-C6)cycloalkoxy; a (C3-C6)halocycloalkoxy; a (C1-C6)alkoxy (C1-C6)alkoxy; a (C1-C6)haloalkoxy (C1-C6)alkoxy; a (C1-C6)haloalkoxy (C1-C6)haloalkoxy; a thiol; a (C1-C6)alkylthio; a (C1-C6)haloalkylthio; a (C2-C6)alkenylthio; a (C2-C6)haloalkenylthio; a (C3-C6)cycloalkylthio; a (C3-C6)halocycloalkylthio; a (C3-C6)cycloalkyl (C1-C6)alkylthio; a (C3-C6)halocycloalkyl (C1-C6)alkylthio; a tri((C1-C6)alkyl)silyl (C1-C6)alkylthio; a (C1-C6)alkylthio (C1-C6)alkoxy; a (C1-C6)haloalkylthio (C1-C6)alkoxy; a (C1-C6)alkylsulfinyl; a (C1-C6)haloalkylsulfinyl; a (C3-C6)cycloalkylsulfinyl; a (C3-C6)halocycloalkylsulfinyl; a (C1-C6)alkylsulfonyl; a (C1-C6)haloalkylsulfonyl; a (C3-C6)cycloalkylsulfonyl; a (C3-C6)halocycloalkylsulfonyl; a formyl; a (C1-C6)alkylcarbonyl; a (C1-C6)haloalkylcarbonyl; a benzoyl (the benzoyl is optionally mono-substituted or poly-substituted with R⁷); a (C1-C6)alkoxycarbonyl; a (C1-C6)haloalkoxycarbonyl; a (C1-C6)alkylaminocarbonyl (the amino moiety is optionally substituted with R¹⁰); a (C1-C6)haloalkylaminocarbonyl (the amino moiety is optionally substituted with R¹⁰); a (C1-C6)alkylaminocarbonylthio (the amino moiety is optionally substituted with R¹⁰); a (C1-C6)haloalkylaminocarbonylthio (the amino moiety is optionally substituted with R¹⁰); a formyloxy; a (C1-C6)alkylcarbonyloxy; a (C1-C6)haloalkylcarbonyloxy; a benzoyloxy (the benzoyloxy is optionally mono-substituted or poly-substituted with R⁷); a (C1-C6)alkylsulfinyloxy; a (C1-C6)haloalkylsulfinyloxy; a (C3-C6)cycloalkylsulfinyloxy; a (C3-C6)halocycloalkylsulfinyloxy; a phenylsulfinyloxy (the phenylsulfinyloxy is optionally mono-substituted or poly-substituted with R⁷); a (C1-C6)alkylsulfonyloxy; a (C1-C6)haloalkylsulfonyloxy; a (C3-C6)cycloalkylsulfonyloxy; a (C3-C6)halocycloalkylsulfonyloxy; a phenylsulfonyloxy (the phenyl moiety is optionally mono-substituted or poly-substituted with R⁷); an amino; a (C1-C6)alkylamino (the amino moiety is optionally substituted with R¹⁰); a (C1-C6)haloalkylamino (the amino moiety is optionally substituted with R¹⁰); a (C1-C6)alkylcarbonylamino (the amino moiety is optionally substituted with R¹⁰); a (C1-C6)haloalkylcarbonylamino (the amino moiety is optionally substituted with R¹⁰); a phenylcarbonylamino (the phenyl moiety is optionally mono-substituted or poly-substituted with R⁷, and the amino moiety is optionally substituted with R¹⁰); a (Cl-C6)alkoxycarbonylamino (the amino moiety is optionally substituted with R¹⁰); a (Cl-C6)haloalkoxycarbonylamino (the amino moiety is optionally substituted with R¹⁰); a (Cl-C6)alkylaminocarbonylamino (the amino moiety is optionally substituted with R¹⁰); a (Cl-C6)haloalkylaminocarbonylamino (the amino moiety is optionally substituted with R¹⁰); a (Cl-C6)alkylsulfinylamino (the amino moiety is optionally substituted with R¹⁰); a (C1-C6)haloalkylsulfinylamino (the amino moiety is optionally substituted with R¹⁰); a phenylsulfinylamino (the phenyl moiety is optionally mono-substituted or poly-substituted with R⁷, and the amino moiety is optionally substituted with R¹⁰); a (Cl-C6)alkylsulfonylamino (the amino moiety is optionally substituted with R¹⁰); a (C1-C6)haloalkylsulfonylamino (the amino moiety is optionally substituted with R¹⁰); a phenylsulfonylamino (the phenyl moiety is optionally mono-substituted or poly-substituted with R⁷, and the amino moiety is optionally substituted with R¹⁰); a (Cl-C6)alkoxyimino; a tri((C1-C6)alkyl)silyl; a phenyl (the phenyl is optionally mono-substituted or poly-substituted with R⁷); a phenoxy (the phenoxy is optionally mono-substituted or poly-substituted with R⁷); a phenyl (Cl-C6)alkoxy (the phenyl moiety is optionally mono-substituted or poly-substituted with R⁷); a phenylthio (the phenylthio is optionally mono-substituted or poly-substituted with R⁷); a phenylsulfinyl (the phenylsulfinyl is optionally mono-substituted or poly-substituted with R⁷); a phenylsulfonyl (the phenylsulfonyl is optionally mono-substituted or poly-substituted with R⁷); a phenyl (Cl-C6)alkylthio (the phenyl moiety is optionally mono-substituted or poly-substituted with R⁷); a phenyl (Cl-C6)alkylsulfinyl (the phenyl moiety is optionally mono-substituted or poly-substituted with R⁷); a phenyl (Cl-C6)alkylsulfonyl (the phenyl moiety is optionally mono-substituted or poly-substituted with R⁷); a -O-N=C(R⁸)(R⁹) group; an adamantyl; an azetidinyl (a nitrogen atom of the azetidinyl is optionally substituted with R¹⁰); a pyrrolidinyl (a nitrogen atom of the pyrrolidinyl is optionally substituted with R¹⁰); a piperidinyl (a nitrogen atom of the piperidinyl is optionally substituted with R¹⁰); a 1,3-dioxolanyl; a 1,3-dioxanyl; a pyrrolyl (the pyrrolyl is optionally mono-substituted or poly-substituted with R⁷); a pyrazolyl (the pyrazolyl is optionally mono-substituted or poly-substituted with R⁷); an imidazolyl (the imidazolyl is optionally mono-substituted or poly-substituted with R⁷); a triazolyl (the triazolyl is optionally mono-substituted or poly-substituted with R⁷); an oxazolyl (the oxazolyl is optionally mono-substituted or poly-substituted with R⁷); an isoxazolyl (the isoxazolyl is optionally mono-substituted or poly-substituted with R⁷); a thiazolyl (the thiazolyl is optionally mono-substituted or poly-substituted with R⁷); an isothiazolyl (the isothiazolyl is optionally mono-substituted or poly-substituted with R⁷); a pyridyl (the pyridyl is optionally mono-substituted or poly-substituted with R⁷, and further a nitrogen atom of the pyridyl is optionally oxidized to form an N-oxide); a pyrimidinyl (the pyrimidinyl is optionally mono-substituted or poly-substituted with R⁷); a pyridyloxy (the pyridyloxy is optionally mono-substituted or poly-substituted with R⁷); a tetrahydrofuranyl (the tetrahydrofuranyl is optionally mono-substituted or poly-substituted with R⁷); a 1,3-dioxoisoindolinyl group (the dioxoisoindolinyl group is optionally mono-substituted or poly-substituted with R⁷); a cyano; a nitro; a carboxy; a thiocyanato; or an aminooxy.

In the present invention, "optionally mono-substituted or poly-substituted with R⁷" means "optionally substituted with one or two or more R⁷". When the group is substituted with two or more R⁷, the two or more R⁷ are identical to or different from each other, and each represent a halogen atom; a (C1-C6)alkyl; a (C1-C6)haloalkyl; a (C3-C6)cycloalkyl; a (C3-C6)halocycloalkyl; a (C3-C6)cycloalkyl (Cl-C6)alkyl; a (C3-C6)halocycloalkyl (C1-C6)alkyl; a (C1-C6)alkoxy; a (C1-C6)haloalkoxy; a (C1-C6)alkylthio; a (C1-C6)haloalkylthio; a (C1-C6)alkylsulfinyl; a (C1-C6)haloalkylsulfinyl; a (C1-C6)alkylsulfonyl; a (C1-C6)haloalkylsulfonyl; a (C1-C6)alkylthio (C1-C6)alkyl; a (C1-C6)haloalkylthio (C1-C6)alkyl; a (C1-C6)alkylsulfonyloxy; a (C1-C6)haloalkylsulfonyloxy; a phenyl (the phenyl is optionally mono-substituted or poly-substituted with a halogen atom, an alkyl, or a haloalkyl); a phenyl (C1-C6)alkyl; a phenyl (C1-C6)alkoxy; a cyano; or a nitro group.

In the present invention, the agriculturally acceptable salt or salt means a salt of any compound of the present invention of formula [I], [II], or [III], which contains a hydroxyl group, a carboxyl group, an amino group, or the like, or a nitrogen atom of a pyridine ring in the structure thereof, with a metal or an organic base, or with a mineral acid or an organic acid. Examples of the metal include an alkali metal such as sodium or potassium, or an alkaline earth metal such as magnesium or calcium. Examples of the organic base include triethylamine or diisopropylamine. Examples of the mineral acid include phosphoric acid, hydrochloric acid, hydrobromic acid, hydroiodic acid, boric acid, or sulfuric acid. Examples of the organic acid include formic acid, acetic acid, lactic acid, ascorbic acid, succinic acid, fumaric acid, maleic acid, oxalic acid, citric acid, benzoic acid, salicylic acid, tartaric acid, methanesulfonic acid, 4-toluenesulfonic acid, and trifluoromethanesulfonic acid.

Next, representative compounds of the compounds included in the 3-alkoxybenzamide derivative of the present invention of formula [I] are shown in Tables 1 to 1062, representative compounds of the compounds included in the hydroxybenzamide derivative of the present invention of formula [II] are shown in Tables 1063 to 1064, and representative compounds of the compounds included in the alkoxybenzoic acid derivative of the present invention of formula [III] are shown in Tables 1065 to 1233. Any compound falling outside the appended claims is merely provided for illustrative purposes. In addition, the compound numbers in the tables are referred to in the following description.

The compounds included in the 3-alkoxybenzamide derivative, alkoxybenzoic acid derivative, or hydroxybenzderivative of the present invention may have E- and Z-form geometric isomers depending on the type of the substituent, but the present invention includes a mixture containing these E-, Z-, or E- and Z-forms in an arbitrary ratio. In addition, the compounds included in the present invention may have optical isomers resulting from the presence of one or two or more asymmetric carbon atoms and asymmetric sulfur atoms, but the present invention includes all optically active forms, racemates, or diastereomers.

The following notations in the tables in the present specification represent, for example, corresponding groups as follows.
Me: Methyl;
Et: Ethyl;
n-Pr: n-Propyl;
i-Pr: Isopropyl;
n-Bu: n-Butyl;
t-Bu: tert-Butyl;
n-Pen: n-Pentyl;
t-Pen: tert-Pentyl;
n-Hex: n-Hexyl;
n-Heptyl: n-Heptyl;
n-Octyl: n-Octyl;
n-Nonyl: n-Nonyl;
n-Decyl: n-Decyl;
c-Pr: Cyclopropyl;
c-Pen: Cyclopentyl;
c-Hex: Cyclohexyl;
CHF₂: Difluoromethyl;
CF₃: Trifluoromethyl;
Ph(4-CF₃): 4-Trifluoromethylphenyl;
Ph(2-F-4-CF₃): 2-Fluoro-4-trifluoromethylphenyl;
Ph(3,4,5-Cl₃): 3, 4, 5-Trichlorophenyl;
c-Pr(2,2-F₂): 2,2-Difluorocyclopropyl;
N(Phth): Phthalimide;
THF: Tetrahydrofuran;

**[Table 1]**

| 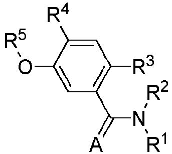 | | | | | | |
|---|---|---|---|---|---|---|
| Compound Number | R¹ | R² | R³ | R⁴ | R⁵ | A |
| A-0001 | CH₂CF₃ | H | Cl | F | Me | O |
| A-0002 | CH₂CF₃ | H | Cl | F | Et | O |
| A-0003 | CH₂CF₃ | H | Cl | F | *n*-Pr | O |
| A-0004 | CH₂CF₃ | H | Cl | F | *i*-Pr | O |
| A-0005 | CH₂CF₃ | H | Cl | F | *n*-Bu | O |
| A-0006 | CH₂CF₃ | H | Cl | F | *n* -Pen | O |
| A-0007 | CH₂CF₃ | H | Cl | F | *n -Hex* | O |
| A-0008 | CH₂CF₃ | H | Cl | F | *n*-Heptyl | O |
| A-0009 | CH₂CF₃ | H | Cl | F | *n*-Octyl | O |
| A-0010 | CH₂CF₃ | H | Cl | F | *n*-Nonyl | O |
| A-0011 | CH₂CF₃ | H | Cl | F | *n*-Decyl | O |
| A-0012 | CH₂CF₃ | H | Cl | F | *c*-Pr | O |
| A-0013 | CH₂CF₃ | H | Cl | F | *c*-Pen | O |
| A-0014 | CH₂CF₃ | H | Cl | F | *c*-Hex | O |
| A-0015 | CH₂CF₃ | H | Cl | F | CH₂C(Me)=CH₂ | O |
| A-0016 | CH₂CF₃ | H | Cl | F | CH₂CH₂CH=CH₂ | O |
| A-0017 | CH₂CF₃ | H | Cl | F | CH₂CH₂CH=C(CH₃)₂ | O |
| A-0018 | CH₂CF₃ | H | Cl | F | CH₂CH₂CH₂CH=CH₂ | O |
| A-0019 | CH₂CF₃ | H | Cl | F | CH₂CH₂CH₂CH=C(CH₃)₂ | O |
| A-0020 | CH₂CF₃ | H | Cl | F | CH₂CH₂CH₂CH₂CH=CH₂ | O |
| A-0021 | CH₂CF₃ | H | Cl | F | CH₂CH₂CH₂CH₂CH=C(CH₃)₂ | O |
| A-0022 | CH₂CF₃ | H | Cl | F | CH₂CH₂CH=CH | O |
| A-0023 | CH₂CF₃ | H | Cl | F | CH₂CH₂CH₂CH=CH | O |
| A-0024 | CH₂CF₃ | H | Cl | F | CH₂CH₂CH₂CH₂CH≡CH | O |
| A-0025 | CH₂CF₃ | H | Cl | F | CH₂CH₂CH₂Cl | O |
| A-0026 | CH₂CF₃ | H | Cl | F | CH₂CH₂CH₂Br | O |
| A-0027 | CH₂CF₃ | H | Cl | F | CH₂CH₂CH₂CH₂Cl | O |
| A-0028 | CH₂CF₃ | H | Cl | F | CH₂CH₂CH₂CH₂Br | O |
| A-0029 | CH₂CF₃ | H | Cl | F | CH₂CH₂CH₂CH₂CH₂Cl | O |
| A-0030 | CH₂CF₃ | H | Cl | F | CH₂CH₂CH₂CH₂CH₂Br | O |
| A-0031 | CH₂CF₃ | H | Cl | Cl | CH₂CH₂CH₂CH₂CH₂Br | O |
| A-0032 | CH₂CF₃ | H | Cl | F | CH₂CH₂CH₂CH₂CH₂CH₂Cl | O |
| A-0033 | CH₂CF₃ | H | Cl | F | CH₂CH₂CH₂CH₂CH₂CH₂Br | O |
| A-0034 | CH₂CF₃ | H | Cl | F | CH₂CH₂CH₂CH₂CH₂CH₂CH₂Cl | O |
| A-0035 | CH₂CF₃ | H | Cl | F | CH₂CH₂CH₂CH₂CH₂CH₂CH₂Br | O |
| A-0036 | CH₂CF₃ | H | Cl | F | CH₂CH₂CH₂CH₂CH₂CH₂CH₂CH₂Br | O |
| A-0037 | CH₂CF₃ | H | Cl | F | CH₂CH(Me)CH₂Cl | O |
| A-0038 | CH₂CF₃ | H | Cl | F | CH₂CH(Me)CH₂Br | O |

**[Table 2]**

| Compound Number | R¹ | R² | R³ | R⁴ | R⁵ | A |
|---|---|---|---|---|---|---|
| A-0039 | CH₂CF₃ | H | Cl | F | CH₂CH₂CH(Me)CH₂CH₂Cl | O |
| A-0040 | CH₂CF₃ | H | Cl | F | CH₂CH₂CH(Me)CH₂CH₂Br | O |
| A-0041 | CH₂CF₃ | H | Cl | F | CH₂CH₂CF=CF₂ | O |
| A-0042 | CH₂CF₃ | H | Cl | F | CH₂CH=C(Cl)CF₃ | O |
| A-0043 | CH₂CF₃ | H | Cl | F | CH₂CH₂CH₂CF=CF₂ | O |
| A-0044 | CH₂CF₃ | H | Cl | F | CH₂CH₂CH₂CH₂CF=CF₂ | O |
| A-0045 | CH₂CF₃ | H | Cl | F | CH₂CH₂CH₂CH₂CH₂CF=CF₂ | O |
| A-0046 | CH₂CF₃ | H | Cl | F | CH₂(*t*-Bu) | O |
| A-0047 | CH₂CF₃ | H | Cl | F | CH₂CH₂(*t*-Bu) | O |
| A-0048 | CH₂CF₃ | H | Cl | F | CH₂CH₂CH₂(*t*-Bu) | O |
| A-0049 | CH₂CF₃ | H | Cl | F | CH₂CH₂CH₂CH₂(*t*-Bu) | O |
| A-0050 | CH₂CF₃ | H | Cl | F | CH₂CH₂CH₂CH₂CH₂(*t*-Bu) | O |
| A-0051 | CH₂CF₃ | H | Cl | F | CH₂CH₂CH₂CH₂CH₂CH₂(*t*-Bu) | O |
| A-0052 | CH₂CF₃ | H | Cl | F | CH₂CF₃ | O |
| A-0053 | CH₂CF₃ | H | Cl | F | CH₂CH₂CF₃ | O |
| A-0054 | CH₂CF₃ | H | Cl | F | CH₂CH₂CH₂CF₃ | O |
| A-0055 | CH₂CF₃ | H | Cl | F | CH₂CH₂CH₂CH₂CF₃ | O |
| A-0056 | CH₂CF₃ | H | Cl | F | CH₂CH₂CH₂CH₂CH₂CF₃ | O |
| A-0057 | CH₂CF₃ | H | Cl | F | CH₂CH₂CH₂CH₂CH₂CH₂CF₃ | O |
| A-0058 | CH₂CF₃ | H | Cl | F | CH₂CH₂CH₂CH₂CH₂CH₂CH₂CF₃ | O |
| A-0059 | CH₂CF₃ | H | Cl | F | CH₂CF₂CF₃ | O |
| A-0060 | CH₂CF₃ | H | Cl | F | CF₂CHFCF₃ | O |
| A-0061 | CH₂CF₃ | H | Cl | F | CF₂CF₂CF₃ | O |
| A-0062 | CH₂CF₃ | H | Cl | F | CH₂CF₂CF₂CF₃ | O |
| A-0063 | CH₂CF₃ | H | Cl | F | CH₂CF₂CF₂CF₂CF₃ | O |
| A-0064 | CH₂CF₃ | H | Cl | F | CH₂CF₂CF₂CF₂CHF₂ | O |
| A-0065 | CH₂CF₃ | H | Cl | F | CH(CF₃)CF₃ | O |
| A-0066 | CH₂CF₃ | H | Cl | F | CH₂CF(CF₃)CF₃ | O |
| A-0067 | CH₂CF₃ | H | Cl | F | CH₂CF₂CF(CF₃)CF₃ | O |
| A-0068 | CH₂CF₃ | H | Cl | F | CH₂CF(CF₃)CF₂CF₃ | O |
| A-0069 | CH₂CF₃ | H | Cl | F | CH₂CF₂CF₂CF(CF₃)CF₃ | O |
| A-0070 | CH₂CF₃ | H | Cl | F | CH₂CF₂CF(CF₃)CF₂CF₃ | O |
| A-0071 | CH₂CF₃ | H | Cl | F | CH₂CF(CF₃)CF₂CF₂CF₃ | O |
| A-0072 | CH₂CF₃ | H | Cl | F | CH₂(*c*-Pr) | O |
| A-0073 | CH₂CF₃ | H | Cl | F | CH₂[*c*-Pr(1-Me)] | O |
| A-0074 | CH₂CF₃ | H | Cl | F | CH₂[*c*-Pr(1-Ph)] | O |
| A-0075 | CH₂CF₃ | H | Cl | F | CH₂[*c*-Pr(1-NH₂)] | O |
| A-0076 | CH₂CF₃ | H | Cl | F | CH₂[*c*-Pr(1-NHC(=O)O(t-Bu)] | O |
| A-0077 | CH₂CF₃ | H | Cl | F | CH₂{*c*-Pr[1-NHS(=O)₂CF₃]} | O |
| A-0078 | CH₂CF₃ | H | Cl | F | CH₂{*c*-Pr[1-Ph(4-CF₃)]} | O |
| A-0079 | CH₂CF₃ | H | Cl | F | CH₂{*c*-Pr[1-Ph(3,4,5-F₃)]} | O |
| A-0080 | CH₂CF₃ | H | Cl | F | CH₂{*c*-Hex[4-(*t*-Bu)]} | O |
| A-0081 | CH₂CF₃ | H | Cl | F | CH₂[*c*-Hex(4-CF₃)] | O |
| A-0082 | CH₂CF₃ | H | Cl | F | CH₂CH₂(*c*-Pr) | O |

**[Table 3]**

| Compound Number | R¹ | R² | R³ | R⁴ | R⁵ | A |
|---|---|---|---|---|---|---|
| A-0083 | CH₂CF₃ | H | Cl | F | CH₂CH₂(*c*-Hex) | O |
| A-0084 | CH₂CF₃ | H | Cl | F | CH₂CH₂[*c*-Hex(4-CF₃)] | O |
| A-0085 | CH₂CF₃ | H | Cl | F | CH₂CH₂[*c*-Hex(4-SCF₃)] | O |
| A-0086 | CH₂CF₃ | H | Cl | F | CH₂CH₂CH₂(*c*-Pr) | O |
| A-0087 | CH₂CF₃ | H | Cl | F | CH₂CH₂CH₂(*c*-Hex) | O |
| A-0088 | CH₂CF₃ | H | Cl | F | CH₂CH₂CH₂{*c-*Hex[4-(*t*-Bu)]) | O |
| A-0089 | CH₂CF₃ | H | Cl | F | CH₂CH₂CH₂[*c*-Hex(4-CF₃)] | O |
| A-0090 | CH₂CF₃ | H | Cl | F | CH₂CH₂CH₂[*c*-Hex(4-SCF₃)] | O |
| A-0091 | CH₂CF₃ | H | Cl | F | CH₂CH₂CH₂CH₂(*c*-Pr) | O |
| A-0092 | CH₂CF₃ | H | Cl | F | CH₂CH₂CH₂CH₂(*c*-Hex) | O |
| A-0093 | CH₂CF₃ | H | Cl | F | CH₂CH₂CH₂CH₂[*c*-Hex(4-CF₃)] | O |
| A-0094 | CH₂CF₃ | H | Cl | F | CH₂[*c*-Pr(2,2-F₂)] | O |
| A-0095 | CH₂CF₃ | H | Cl | F | CH₂[*c*-Hex(4,4-F₂)] | O |
| A-0096 | CH₂CF₃ | H | Cl | F | CH₂CH₂[*c*-Pr(2,2-F₂)] | O |
| A-0097 | CH₂CF₃ | H | Cl | F | CH₂CH₂[*c*-Hex(4,4-F₂)] | O |
| A-0098 | CH₂CF₃ | H | Cl | F | CH₂CH₂CH₂[*c*-Pr(2,2-F₂) | O |
| A-0099 | CH₂CF₃ | H | Cl | F | CH₂CH₂CH₂CH₂[*c*-Pr(2,2-F₂)] | O |
| A-0100 | CH₂CF₃ | H | Cl | F | CH₂CH₂OH | O |
| A-0101 | CH₂CF₃ | H | Cl | F | CH₂CH₂CH₂OH | O |
| A-0102 | CH₂CF₃ | H | Cl | F | CH₂CH₂CH₂CH₂OH | O |
| A-0103 | CH₂CF₃ | H | Cl | F | CH₂CH₂CH₂CH₂CH₂OH | O |
| A-0104 | CH₂CF₃ | H | Cl | F | CH₂CH₂CH₂CH₂CH₂CH₂OH | O |
| A-0105 | CH₂CF₃ | H | Cl | F | CH₂C(=O)Me | O |
| A-0106 | CH₂CF₃ | H | Cl | F | CH₂CH(OH)Me | O |
| A-0107 | CH₂CF₃ | H | Cl | F | CH₂CH(OH)CF₃ | O |
| A-0108 | CH₂CF₃ | H | Cl | F | CH₂C(OH)₂CF₃ | O |
| A-0109 | CH₂CF₃ | H | Cl | F | CH₂C(CF₃)=NOH | O |
| A-0110 | CH₂CF₃ | H | Cl | F | CH₂C(CF₃)=NOMe | O |
| A-0111 | CH₂CF₃ | H | Cl | F | CH₂CH(CF₃)NH₂ | O |
| A-0112 | CH₂CF₃ | H | Cl | F | CH₂CH₂OMe | O |
| A-0113 | CH₂CF₃ | H | Cl | F | CH₂CH₂CH₂OMe | O |
| A-0114 | CH₂CF₃ | H | Cl | F | CH₂CH₂CH₂CH₂OMe | O |
| A-0115 | CH₂CF₃ | H | Cl | F | CH₂CH₂CH₂CH₂CH₂OMe | O |
| A-0116 | CH₂CF₃ | H | Cl | F | CH₂CH₂CH₂CH₂CH₂CH₂OMe | O |
| A-0117 | CH₂CF₃ | H | Cl | F | CH₂CH₂OCHF₂ | O |
| A-0118 | CH₂CF₃ | H | Cl | F | CH₂CH₂CH₂OCHF₂ | O |
| A-0119 | CH₂CF₃ | H | Cl | F | CH₂CH₂CH₂CH₂OCHF₂ | O |
| A-0120 | CH₂CF₃ | H | Cl | F | CH₂CH₂CH₂CH₂CH₂OCHF₂ | O |
| A-0121 | CH₂CF₃ | H | Cl | F | CH₂CH₂CH₂CH₂CH₂CH₂OCHF₂ | O |
| A-0122 | CH₂CF₃ | H | Cl | F | CH₂CH₂OCH₂CF₃ | O |
| A-0123 | CH₂CF₃ | H | Cl | F | CH₂CH₂CH₂OCH₂CF₃ | O |
| A-0124 | CH₂CF₃ | H | Cl | F | CH₂CH₂CH₂CH₂OCH₂CF₃ | O |
| A-0125 | CH₂CF₃ | H | Cl | F | CH₂CH₂CH₂CH₂CH₂OCHCF₃ | O |
| A-0126 | CH₂CF₃ | H | Cl | F | CH₂CH₂CH₂CH₂CH₂CH₂OCH₂CF₃ | O |

**[Table 4]**

| Compound Number | R¹ | R² | R³ | R⁴ | R⁵ | A |
|---|---|---|---|---|---|---|
| A-0127 | CH₂CF₃ | H | Cl | F | CH₂CH₂CH₂CH₂CH₂CH₂CH₂OCH₂CF₃ | O |
| A-0128 | CH₂CF₃ | H | Cl | F | CH₂CH₂OC(CF₃)₃ | O |
| A-0129 | CH₂CF₃ | H | Cl | F | CH₂CH₂CH₂OC(CF₃)₃ | O |
| A-0130 | CH₂CF₃ | H | Cl | F | CH₂CH₂CH₂CH₂OC(CF₃)₃ | O |
| A-0131 | CH₂CF₃ | H | Cl | F | CH₂CH₂CH₂CH₂CH₂OC(CF₃)₃ | O |
| A-0132 | CH₂CF₃ | H | Cl | F | CF₂CHFOCF₂CF₂CF₃ | O |
| A-0133 | CH₂CF₃ | H | Cl | F | CH₂CH₂O(*c*-Pr) | O |
| A-0134 | CH₂CF₃ | H | Cl | F | CH₂CH₂O(*c*-Pen) | O |
| A-0135 | CH₂CF₃ | H | Cl | F | CH₂CH₂O(*c*-Hex) | O |
| A-0136 | CH₂CF₃ | H | Cl | F | CH₂CH₂CH₂O(*c*-Pr) | O |
| A-0137 | CH₂CF₃ | H | Cl | F | CH₂CH₂CH₂O(*c*-Pen) | O |
| A-0138 | CH₂CF₃ | H | Cl | F | CH₂CH₂CH₂O(*c*-Hex) | O |
| A-0139 | CH₂CF₃ | H | Cl | F | CH₂CH₂CH₂CH₂O(*c*-Pr) | O |
| A-0140 | CH₂CF₃ | H | Cl | F | CH₂CH₂CH₂CH₂O(*c*-Pen) | O |
| A-0141 | CH₂CF₃ | H | Cl | F | CH₂CH₂CH₂CH₂O(*c*-Hex) | O |
| A-0142 | CH₂CF₃ | H | Cl | F | CH₂CH₂O[*c*-Pr(2,2-F₂)] | O |
| A-0143 | CH₂CF₃ | H | Cl | F | CH₂CH₂CH₂O[*c*-Pr(2,2-F₂)] | O |
| A-0144 | CH₂CF₃ | H | Cl | F | CH₂CH₂CH₂CH₂O[*c*-Pr(2,2-F₂)] | O |
| A-0145 | CH₂CF₃ | H | Cl | F | CH₂CH₂OCH₂CH₂OCH₃ | O |
| A-0146 | CH₂CF₃ | H | Cl | F | CH₂CH₂OCH₂CH₂OCH₂CF₃ | O |
| A-0147 | CH₂CF₃ | H | Cl | F | CH₂CF₂OCF₂CF₂OCF₃ | O |
| A-0148 | CH₂CF₃ | H | Cl | F | CF₂CHFOCF₂CF₂OCF₃ | O |
| A-0149 | CH₂CF₃ | H | Cl | F | CF₂CHFOCF₂CF(CF₃)OCF₂CF₂CF₃ | O |
| A-0150 | CH₂CF₃ | H | Cl | F | CH₂CH₂SH | O |
| A-0151 | CH₂CF₃ | H | Cl | F | CH₂CH₂CH₂SH | O |
| A-0152 | CH₂CF₃ | H | Cl | F | CH₂CH₂CH₂CH₂SH | O |
| A-0153 | CH₂CF₃ | H | Cl | F | CH₂CH₂CH₂CH₂CH₂SH | O |
| A-0154 | CH₂CF₃ | H | Cl | F | CH₂CH₂CH₂CH₂CH₂CH₂SH | O |
| A-0155 | CH₂CF₃ | H | Cl | F | CH₂CH(SH)Me | O |
| A-0156 | CH₂CF₃ | H | Cl | F | CH₂CH[SC(=O)Me]Me | O |
| A-0157 | CH₂CF₃ | H | Cl | F | CH₂CH₂S(*t*-Bu) | O |
| A-0158 | CH₂CF₃ | H | Cl | F | CH₂CH₂CH₂S(*t*-Bu) | O |
| A-0159 | CH₂CF₃ | H | Cl | F | CH₂CH₂CH₂CH₂S(*t*-Bu) | O |
| A-0160 | CH₂CF₃ | H | Cl | F | CH₂CH₂CH₂CH₂CH₂SCH₃ | O |
| A-0161 | CH₂CF₃ | H | Cl | F | CH₂CH₂CH₂CH₂CH₂SCH(CH₃)₂ | O |
| A-0162 | CH₂CF₃ | H | Cl | F | CH₂CH₂CH₂CH₂CH₂S(*t*-Bu) | O |
| A-0163 | CH₂CF₃ | H | Cl | F | CH₂CH₂CH₂CH₂CH₂CH₂SCH(CH₃)₂ | O |
| A-0164 | CH₂CF₃ | H | Cl | F | CH₂CH₂CH₂CH₂CH₂CH₂S(*t*-Bu) | O |
| A-0165 | CH₂CF₃ | H | Cl | F | CH₂CH₂CH₂CH₂CH₂CH₂CH₂S(*t*-Bu) | O |
| A-0166 | CH₂CF₃ | H | Cl | F | CH₂CH₂SCHF₂ | O |
| A-0167 | CH₂CF₃ | H | Cl | F | CH₂CH₂CH₂SCHF₂ | O |
| A-0168 | CH₂CF₃ | H | Cl | F | CH₂CH₂CH₂CH₂SCHF₂ | O |
| A-0169 | CH₂CF₃ | H | Cl | H | CH₂CH₂CH₂CH₂CH₂SCHF₂ | O |
| A-0170 | CH₂CF₃ | H | Cl | F | CH₂CH₂CH₂CH₂CH₂SCHF₂ | O |

**[Table 5]**

| Compound Number | R¹ | R² | R³ | R⁴ | R⁵ | A |
|---|---|---|---|---|---|---|
| A-0171 | CH₂CF₃ | H | Cl | F | CH₂CH₂CH₂CH₂CH₂CH₂SCHF₂ | O |
| A-0172 | CH₂CF₃ | H | Cl | F | CH₂CH₂CH₂CH₂CH₂CH₂CH₂SCHF₂ | O |
| A-0173 | CH₂CF₃ | H | Cl | F | CH₂CH₂SCH₂CF₃ | O |
| A-0174 | CH₂CF₃ | H | Cl | F | CH₂CH₂CH₂SCH₂CF₃ | O |
| A-0175 | CH₂CF₃ | H | Cl | F | CH₂CH₂CH₂CH₂SCH₂CF₃ | O |
| A-0176 | CH₂CF₃ | H | Cl | F | CH₂CH₂CH₂CH₂CH₂SCH₂CF₃ | O |
| A-0177 | CH₂CF₃ | H | Cl | F | CH₂CH₂CH₂CH₂CH₂CH₂SCH₂CF₃ | O |
| A-0178 | CH₂CF₃ | H | Cl | F | CH₂CH₂CH₂CH₂CH₂CH₂CH₂SCH₂CF₃ | O |
| A-0179 | CH₂CF₃ | H | Cl | F | CH₂CH(Me)SCH₂CF₃ | O |
| A-0180 | CH₂CF₃ | H | Cl | F | CH₂CH₂SCH(CF₃)₂ | O |
| A-0181 | CH₂CF₃ | H | Cl | F | CH₂CH₂CH₂SCH(CF₃)₂ | O |
| A-0182 | CH₂CF₃ | H | Cl | F | CH₂CH₂CH₂CH₂SCH(CF₃)₂ | O |
| A-0183 | CH₂CF₃ | H | Cl | F | CH₂CH₂CH₂CH₂CH₂SCH(CF₃)₂ | O |
| A-0184 | CH₂CF₃ | H | Cl | F | CH₂CH₂CH₂CH₂CH₂CH₂SCH(CF₃)₂ | O |
| A-0185 | CH₂CF₃ | H | Cl | F | CH₂CH₂CH₂CH₂CH₂SCF₂CF₃ | O |
| A-0186 | CH₂CF₃ | H | Cl | F | CH₂CH₂CH₂CH₂CH₂CH₂SCF₂CF₃ | O |
| A-0187 | CH₂CF₃ | H | Cl | F | CH₂CH₂CH₂CH₂CH₂CH₂SCF(CF₃)₂ | O |
| A-0188 | CH₂CF₃ | H | Cl | F | CH₂CH₂CH₂CH₂CH₂CH₂CH₂SCF(CF₃)₂ | O |
| A-0189 | CH₂CF₃ | H | Cl | F | CH₂CH₂CH₂CH₂CH₂SCCl₃ | O |
| A-0190 | CH₂CF₃ | H | Cl | F | CH₂CH₂CH₂CH₂CH₂CH₂SCCl₃ | O |
| A-0191 | CH₂CF₃ | H | Cl | F | CH₂CH₂SCH₂CH=CH₂ | O |
| A-0192 | CH₂CF₃ | H | Cl | F | CH₂CH₂CH₂SCH₂CH=CH₂ | O |
| A-0193 | CH₂CF₃ | H | Cl | F | CH₂CH₂CH₂CH₂SCH₂CH=CH₂ | O |
| A-0194 | CH₂CF₃ | H | Cl | F | CH₂CH₂SCF=CFCF₃ | O |
| A-0195 | CH₂CF₃ | H | Cl | F | CH₂CH₂CH₂SCF=CFCF₃ | O |
| A-0196 | CH₂CF₃ | H | Cl | F | CH₂CH₂CH₂CH₂SCF=CFCF₃ | O |
| A-0197 | CH₂CF₃ | H | Cl | F | CH₂CH₂CH₂S(*c*-Pr) | O |
| A-0198 | CH₂CF₃ | H | Cl | F | CH₂CH₂CH₂S(*c*-Hex) | O |
| A-0199 | CH₂CF₃ | H | Cl | F | CH₂CH₂CH₂CH₂S(*c*-Pr) | O |
| A-0200 | CH₂CF₃ | H | Cl | F | CH₂CH₂CH₂CH₂S(*c*-Hex) | O |
| A-0201 | CH₂CF₃ | H | Cl | F | CH₂CH₂CH₂CH₂CH₂S(*c*-Pr) | O |
| A-0202 | CH₂CF₃ | H | Cl | F | CH₂CH₂CH₂CH₂CH₂S(*c*-Hex) | O |
| A-0203 | CH₂CF₃ | H | Cl | F | CH₂CH₂CH₂S[*c*-Hex(4,4-F₂)] | O |
| A-0204 | CH₂CF₃ | H | Cl | F | CH₂CH₂CH₂CH₂S[*c*-Hex(4,4-F₂)] | O |
| A-0205 | CH₂CF₃ | H | Cl | F | CH₂CH₂CH₂CH₂CH₂S[*c*-Hex(4,4-F₂)] | O |
| A-0206 | CH₂CF₃ | H | Cl | F | CH₂CH₂CH₂SCH₂(*c*-Pr) | O |
| A-0207 | CH₂CF₃ | H | Cl | F | CH₂CH₂CH₂SCH₂(*c*-Hex) | O |
| A-0208 | CH₂CF₃ | H | Cl | F | CH₂CH₂CH₂CH₂SCH₂(*c*-Pr) | O |
| A-0209 | CH₂CF₃ | H | Cl | F | CH₂CH₂CH₂CH₂SCH₂(*c*-Hex) | O |
| A-0210 | CH₂CF₃ | H | Cl | F | CH₂CH₂CH₂CH₂CH₂SCH₂(*c*-Pr) | O |
| A-0211 | CH₂CF₃ | H | Cl | F | CH₂CH₂CH₂CH₂CH₂SCH₂(*c*-Hex) | O |
| A-0212 | CH₂CF₃ | H | Cl | F | CH₂CH₂CH₂SCH₂[*c*-Hex(4,4-F₂)] | O |
| A-0213 | CH₂CF₃ | H | Cl | F | CH₂CH₂CH₂CH₂SCH₂[*c*-Hex(4,4-F₂)] | O |
| A-0214 | CH₂CF₃ | H | Cl | F | CH₂CH₂CH₂CH₂CH₂SCH₂[*c*-Hex(4,4-F₂)] | O |

**[Table 6]**

| Compound Number | R¹ | R² | R³ | R⁴ | R⁵ | A |
|---|---|---|---|---|---|---|
| A-0215 | CH₂CF₃ | H | Cl | F | CH₂CH₂CH₂SCH₂SiMe₃ | O |
| A-0216 | CH₂CF₃ | H | Cl | F | CH₂CH₂CH₂CH₂SCH₂SiMe₃ | O |
| A-0217 | CH₂CF₃ | H | Cl | F | CH₂CH₂CH₂CH₂CH₂SCH₂SiMe₃ | O |
| A-0218 | CH₂CF₃ | H | Cl | F | CH₂CH₂OCH₂CH₂SCH₃ | O |
| A-0219 | CH₂CF₃ | H | Cl | F | CH₂CH₂OCH₂CH₂SCHF₂ | O |
| A-0220 | CH₂CF₃ | H | Cl | F | CH₂CH₂OCH₂CH₂SCF₃ | O |
| A-0221 | CH₂CF₃ | H | Cl | F | CH₂CH₂OCH₂CH₂SCH₂CF₃ | O |
| A-0222 | CH₂CF₃ | H | Cl | F | CH₂CH₂CH₂S(=O)(*t*-Bu) | O |
| A-0223 | CH₂CF₃ | H | Cl | F | CH₂CH₂CH₂CH₂S(=O)(*t*-Bu) | O |
| A-0224 | CH₂CF₃ | H | Cl | F | CH₂CH₂CH₂CH₂CH₂CH₂S(=O)(*t*-Bu) | O |
| A-0225 | CH₂CF₃ | H | Cl | F | CH₂CH₂S(=O)CHF₂ | O |
| A-0226 | CH₂CF₃ | H | Cl | F | CH₂CH₂CH₂S(=O)CHF₂ | O |
| A-0227 | CH₂CF₃ | H | Cl | F | CH₂CH₂CH₂CH₂S(=O)CHF₂ | O |
| A-0228 | CH₂CF₃ | H | Cl | F | CH₂CH₂CH₂CH₂CH₂S(=O)CHF₂ | O |
| A-0229 | CH₂CF₃ | H | Cl | F | CH₂CH₂CH₂CH₂CH₂CH₂S(=O)CHF₂ | O |
| A-0230 | CH₂CF₃ | H | Cl | F | CH₂CH₂CH₂CH₂CH₂CH₂CH₂S(=O)CHF₂ | O |
| A-0231 | CH₂CF₃ | H | Cl | F | CH₂CH₂S(=O)CF₃ | O |
| A-0232 | CH₂CF₃ | H | Cl | F | CH₂CH₂CH₂S(=O)CF₃ | O |
| A-0233 | CH₂CF₃ | H | Cl | F | CH₂CH₂CH₂CH₂S(=O)CF₃ | O |
| A-0234 | CH₂CF₃ | H | Cl | F | CH₂CH₂CH₂CH₂CH₂S(=O)CF₃ | O |
| A-0235 | CH₂CF₃ | H | Cl | F | CH₂CH₂CH₂CH₂CH₂CH₂S(=O)CF₃ | O |
| A-0236 | CH₂CF₃ | H | Cl | F | CH₂CH₂CH₂CH₂CH₂CH₂CH₂S(=O)CF₃ | O |
| A-0237 | CH₂CF₃ | H | Cl | F | CH₂CH₂S(=O)CH₂CF₃ | O |
| A-0238 | CH₂CF₃ | H | Cl | F | CH₂CH₂CH₂S(=O)CH₂CF₃ | O |
| A-0239 | CH₂CF₃ | H | Cl | F | CH₂CH₂CH₂CH₂S(=O)CH₂CF₃ | O |
| A-0240 | CH₂CF₃ | H | Cl | F | CH₂CH₂CH₂CH₂CH₂S(=O)CH₂CF₃ | O |
| A-0241 | CH₂CF₃ | H | Cl | F | CH₂CH₂CH₂CH₂CH₂CH₂S(=O)CH₂CF₃ | O |
| A-0242 | CH₂CF₃ | H | Cl | F | CH₂CH₂CH₂CH₂CH₂CH₂CH₂S(=O)CH₂CF₃ | O |
| A-0243 | CH₂CF₃ | H | Cl | F | CH₂CH(Me)S(=O)CH₂CF₃ | O |
| A-0244 | CH₂CF₃ | H | Cl | F | CH₂CH₂CH₂S(=O)(*c*-Pr) | O |
| A-0245 | CH₂CF₃ | H | Cl | F | CH₂CH₂CH₂CH₂S(=O)(*c*-Pr) | O |
| A-0246 | CH₂CF₃ | H | Cl | F | CH₂CH₂CH₂CH₂S(=O)(*c*-Pen) | O |
| A-0247 | CH₂CF₃ | H | Cl | F | CH₂CH₂CH₂CH₂S(=O)(*c*-Hex) | O |
| A-0248 | CH₂CF₃ | H | Cl | F | CH₂CH₂CH₂CH₂CH₂S(=O)(*c*-Pr) | O |
| A-0249 | CH₂CF₃ | H | Cl | F | CH₂CH₂CH₂CH₂CH₂S(=O)(*c*-Hex) | O |
| A-0250 | CH₂CF₃ | H | Cl | F | CH₂CH₂CH₂S(=O)[*c*-Hex(4,4-F₂)] | O |
| A-0251 | CH₂CF₃ | H | Cl | F | CH₂CH₂CH₂CH₂S(=O)[*c*-Hex(4,4-F₂)] | O |
| A-0252 | CH₂CF₃ | H | Cl | F | CH₂CH₂CH₂CH₂CH₂S(=O)[*c*-Hex(4,4-F₂)] | O |
| A-0253 | CH₂CF₃ | H | Cl | F | CH₂CH₂CH₂CH₂S(=O)₂Me | O |
| A-0254 | CH₂CF₃ | H | Cl | F | CH₂CH₂CH₂CH₂CH₂S(=O)₂Me | O |
| A-0255 | CH₂CF₃ | H | Cl | F | CH₂CH₂CH₂CH₂CH₂S(=O)₂(*t*-Bu) | O |
| A-0256 | CH₂CF₃ | H | Cl | F | CH₂CH₂S(=O)₂CHF₂ | O |
| A-0257 | CH₂CF₃ | H | Cl | F | CH₂CH₂CH₂S(=O)₂CHF₂ | O |
| A-0258 | CH₂CF₃ | H | Cl | F | CH₂CH₂CH₂CH₂S(=O)₂CHF₂ | O |

**[Table 7]**

| Compound Number | R¹ | R² | R³ | R⁴ | R⁵ | A |
|---|---|---|---|---|---|---|
| A-0259 | CH₂CF₃ | H | Cl | F | CH₂CH₂CH₂CH₂CH₂S(=O)₂CHF₂ | O |
| A-0260 | CH₂CF₃ | H | Cl | F | CH₂CH₂CH₂CH₂CH₂CH₂S(=O)₂CHF₂ | O |
| A-0261 | CH₂CF₃ | H | Cl | F | CH₂CH₂S(=O)₂CF₃ | O |
| A-0262 | CH₂CF₃ | H | Cl | F | CH₂CH₂CH₂S(=O)₂CF₃ | O |
| A-0263 | CH₂CF₃ | H | Cl | F | CH₂CH₂CH₂CH₂S(=O)₂CF₃ | O |
| A-0264 | CH₂CF₃ | H | Cl | F | CH₂CH₂CH₂CH₂CH₂S(=O)₂CF₃ | O |
| A-0265 | CH₂CF₃ | H | Cl | F | CH₂CH₂CH₂CH₂CH₂CH₂S(=O)₂CF₃ | O |
| A-0266 | CH₂CF₃ | H | Cl | F | CH₂CH₂CH₂CH₂CH₂CH₂CH₂S(=O)₂CF₃ | O |
| A-0267 | CH₂CF₃ | H | Cl | F | CH₂CH₂CH₂CH₂CH₂CH₂CH₂CH₂S(=O)₂CF₃ | O |
| A-0268 | CH₂CF₃ | H | Cl | F | CH₂CH₂S(=O)₂CH₂CF₃ | O |
| A-0269 | CH₂CF₃ | H | Cl | F | CH₂CH₂CH₂S(=O)₂CH₂CF₃ | O |
| A-0270 | CH₂CF₃ | H | Cl | F | CH₂CH₂CH₂CH₂S(=O)₂CH₂CF₃ | O |
| A-0271 | CH₂CF₃ | H | Cl | F | CH₂CH₂CH₂CH₂CH₂S(=O)₂CH₂CF₃ | O |
| A-0272 | CH₂CF₃ | H | Cl | F | CH₂CH₂CH₂CH₂CH₂CH₂S(=O)₂CH₂CF₃ | O |
| A-0273 | CH₂CF₃ | H | Cl | F | CH₂CH(Me)S(=O)₂CH₂CF₃ | O |
| A-0274 | CH₂CF₃ | H | Cl | F | CH₂CH₂CH₂S(=O)₂(*c*-Pr) | O |
| A-0275 | CH₂CF₃ | H | Cl | F | CH₂CH₂CH₂CH₂S(=O)₂(*c*-Pr) | O |
| A-0276 | CH₂CF₃ | H | Cl | F | CH₂CH₂CH₂CH₂CH₂S(=O)₂(*c*-Pr) | O |
| A-0277 | CH₂CF₃ | H | Cl | F | CH₂CH₂CH₂S(=O)₂[*c*-Hex(4,4-F₂)] | O |
| A-0278 | CH₂CF₃ | H | Cl | F | CH₂CH₂CH₂CH₂S(=O)₂[*c*-Hex(4,4-F₂)] | O |
| A-0279 | CH₂CF₃ | H | Cl | F | CH₂CH₂CH₂CH₂CH₂S(=O)₂[*c*-Hex(4,4-F₂)] | O |
| A-0280 | CH₂CF₃ | H | Cl | F | CH₂CH₂CH₂C(=O)H | O |
| A-0281 | CH₂CF₃ | H | Cl | F | CH₂CH₂CH₂CH₂C(=O)H | O |
| A-0282 | CH₂CF₃ | H | Cl | F | CH₂CH₂CH₂CH₂CH₂C(=O)H | O |
| A-0283 | CH₂CF₃ | H | Cl | F | CH₂CH₂CH₂C(=O)(*t*-Bu) | O |
| A-0284 | CH₂CF₃ | H | Cl | F | CH₂CH₂CH₂CH₂C(=O)(*t*-Bu) | O |
| A-0285 | CH₂CF₃ | H | Cl | F | CH₂CH₂CH₂CH₂CH₂C(=O)(*t-*Bu) | O |
| A-0286 | CH₂CF₃ | H | Cl | F | CH₂CH₂CH₂C(=O)CF₃ | O |
| A-0287 | CH₂CF₃ | H | Cl | F | CH₂CH₂CH₂CH₂C(=O)CF₃ | O |
| A-0288 | CH₂CF₃ | H | Cl | F | CH₂CH₂CH₂CH₂CH₂C(=O)CF₃ | O |
| A-0289 | CH₂CF₃ | H | Cl | F | CH₂C(=O)Ph | O |
| A-0290 | CH₂CF₃ | H | Cl | F | CH₂C(=O)Ph(4-Cl) | O |
| A-0291 | CH₂CF₃ | H | Cl | F | CH₂C(=O)Ph(4-CF₃) | O |
| A-0292 | CH₂CF₃ | H | Cl | F | CH₂CH₂C(=O)Ph(4-CF₃) | O |
| A-0293 | CH₂CF₃ | H | Cl | F | CH₂CH₂CH₂C(=O)Ph(4-CF₃) | O |
| A-0294 | CH₂CF₃ | H | Cl | F | CH₂CH₂CH₂CH₂C(=O)Ph(4-CF₃) | O |
| A-0295 | CH₂CF₃ | H | Cl | F | CH₂CH₂CH₂CH₂CH₂C(=O)Ph(4-CF₃) | O |
| A-0296 | CH₂CF₃ | H | Cl | F | CH₂C(=O)OEt | O |
| A-0297 | CH₂CF₃ | H | Cl | F | CH₂CH₂C(=O)O(*t*-Bu) | O |
| A-0298 | CH₂CF₃ | H | Cl | F | CH₂CH₂CH₂C(=O)OEt | O |
| A-0299 | CH₂CF₃ | H | Cl | F | CH₂CH₂CH₂C(=O)O(*t*-Bu) | O |
| A-0300 | CH₂CF₃ | H | Cl | F | CH₂CH₂CH₂CH₂C(=O)O(*t*-Bu) | O |
| A-0301 | CH₂CF₃ | H | Cl | F | CH₂CH₂CH₂CH₂CH₂C(=O)O(*t*-Bu) | O |
| A-0302 | CH₂CF₃ | H | Cl | F | CH₂CH₂CH₂CH₂CH₂CH₂C(=O)O(*t*-Bu) | O |

**[Table 8]**

| Compound Number | R¹ | R² | R³ | R⁴ | R⁵ | A |
|---|---|---|---|---|---|---|
| A-0303 | CH₂CF₃ | H | Cl | F | CH₂CH₂CH₂CH₂CH₂CH₂CH₂C(=O)OEt | O |
| A-0304 | CH₂CF₃ | H | Cl | F | CH₂CH₂C(=O)OCH₂CF₃ | O |
| A-0305 | CH₂CF₃ | H | Cl | F | CH₂CH₂CH₂C(=O)OCH₂CF₃ | O |
| A-0306 | CH₂CF₃ | H | Cl | F | CH₂CH₂CH₂CH₂C(=O)OCH₂CF₃ | O |
| A-0307 | CH₂CF₃ | H | Cl | F | CH₂CH₂CH₂CH₂CH₂C(=O)OCH₂CF₃ | O |
| A-0308 | CH₂CF₃ | H | Cl | F | CH₂CH₂C(=O)NH(*t* -Bu) | O |
| A-0309 | CH₂CF₃ | H | Cl | F | CH₂CH₂CH₂C(=O)NH(*t*-Bu) | O |
| A-0310 | CH₂CF₃ | H | Cl | F | CH₂CH₂CH₂C(=O)NH(*t*-Pen) | O |
| A-0311 | CH₂CF₃ | H | Cl | F | CH₂CH₂CH₂CH₂C(=O)NH(*t*-Bu) | O |
| A-0312 | CH₂CF₃ | H | Cl | F | CH₂CH₂C(=O)NHCH₂CF₃ | O |
| A-0313 | CH₂CF₃ | H | Cl | F | CH₂CH₂CH₂C(=O)NHCH₂CF₃ | O |
| A-0314 | CH₂CF₃ | H | Cl | F | CH₂CH₂CH₂CH₂C(=O)NHCH₂CF₃ | O |
| A-0315 | CH₂CF₃ | H | Cl | F | CH₂CH₂SC(=O)N(Me)₂ | O |
| A-0316 | CH₂CF₃ | H | Cl | F | CH₂CH₂CH₂SC(=O)N(Me)₂ | O |
| A-0317 | CH₂CF₃ | H | Cl | F | CH₂CH₂CH₂SC(=O)NH(*t*-Bu) | O |
| A-0318 | CH₂CF₃ | H | Cl | F | CH₂CH₂CH₂CH₂CH₂SC(=O)NH(*t*-Bu) | O |
| A-0319 | CH₂CF₃ | H | Cl | F | CH₂CH₂SC(=O)NHCH₂CF₃ | O |
| A-0320 | CH₂CF₃ | H | Cl | F | CH₂CH₂CH₂SC(=O)NHCH₂CF₃ | O |
| A-0321 | CH₂CF₃ | H | Cl | F | CH₂CH₂CH₂CH₂SC(=O)NHCH₂CF₃ | O |
| A-0322 | CH₂CF₃ | H | Cl | F | CH₂CH₂CH₂OC(=O)H | O |
| A-0323 | CH₂CF₃ | H | Cl | F | CH₂CH₂CH₂CH₂OC(=O)H | O |
| A-0324 | CH₂CF₃ | H | Cl | F | CH₂CH₂CH₂CH₂CH₂OC(=O)H | O |
| A-0325 | CH₂CF₃ | H | Cl | F | CH₂CH₂CH₂OC(=O)Me | O |
| A-0326 | CH₂CF₃ | H | Cl | F | CH₂CH₂CH₂CH₂OC(=O)Me | O |
| A-0327 | CH₂CF₃ | H | Cl | F | CH₂CH₂CH₂CH₂CH₂OC(=O)Me | O |
| A-0328 | CH₂CF₃ | H | Cl | F | CH₂CH₂OC(=O)CF₃ | O |
| A-0329 | CH₂CF₃ | H | Cl | F | CH₂CH₂CH₂OC(=O)CF₃ | O |
| A-0330 | CH₂CF₃ | H | Cl | F | CH₂CH₂CH₂CH₂OC(=O)CF₃ | O |
| A-0331 | CH₂CF₃ | H | Cl | F | CH₂CH₂CH_{>}CH₂CH₂OC(=O)Ph(4-CF₃) | O |
| A-0332 | CH₂CF₃ | H | Cl | F | CH₂CH₂OC(=O)Ph | O |
| A-0333 | CH₂CF₃ | H | Cl | F | CH₂CH₂OC(=O)Ph(4-CF₃) | O |
| A-0334 | CH₂CF₃ | H | Cl | F | CH₂CH₂CH₂OC(=O)Ph(4-CF₃) | O |
| A-0335 | CH₂CF₃ | H | Cl | F | CH₂CH₂CH₂CH₂OC(=O)Ph(4-CF₃) | O |
| A-0336 | CH₂CF₃ | H | Cl | F | CH₂CH₂CH₂CH₂CH₂OC(=O)Ph(4-CF₃) | O |
| A-0337 | CH₂CF₃ | H | Cl | F | CH₂CH₂OS(=O)Me | O |
| A-0338 | CH₂CF₃ | H | Cl | F | CH₂CH₂CH₂OS(=O)Me | O |
| A-0339 | CH₂CF₃ | H | Cl | F | CH₂CH₂CH₂CH₂OS(=O)Me | O |
| A-0340 | CH₂CF₃ | H | Cl | F | CH₂CH₂CH₂CH₂CH₂OS(=O)Me | O |
| A-0341 | CH₂CF₃ | H | Cl | F | CH₂CH₂CH₂CH₂CH₂CH₂OS(=O)Me | O |
| A-0342 | CH₂CF₃ | H | Cl | F | CH₂CH₂OS(=O)CF₃ | O |
| A-0343 | CH₂CF₃ | H | Cl | F | CH₂CH₂CH₂OS(=O)CF₃ | O |
| A-0344 | CH₂CF₃ | H | Cl | F | CH₂CH₂CH₂CH₂OS(=O)CF₃ | O |
| A-0345 | CH₂CF₃ | H | Cl | F | CH₂CH₂CH₂CH₂CH₂OS(=O)CF₃ | O |
| A-0346 | CH₂CF₃ | H | Cl | F | CH₂CH₂CH₂CH₂CH₂CH₂OS(=O)CF₃ | O |

**[Table 9]**

| Compound Number | R¹ | R² | R³ | R⁴ | R⁵ | A |
|---|---|---|---|---|---|---|
| A-0347 | CH₂CF₃ | H | Cl | F | CH₂CH₂OS(=O)Ph | O |
| A-0348 | CH₂CF₃ | H | Cl | F | CH₂CH₂OS(=O)Ph(4-Me) | O |
| A-0349 | CH₂CF₃ | H | Cl | F | CH₂CH₂CH₂OS(=O)Ph | O |
| A-0350 | CH₂CF₃ | H | Cl | F | CH₂CH₂CH₂OS(=O)Ph(4-Me) | O |
| A-0351 | CH₂CF₃ | H | Cl | F | CH₂CH₂OS(=O)₂Me | O |
| A-0352 | CH₂CF₃ | H | Cl | F | CH₂CH₂CH₂OS(=O)₂Me | O |
| A-0353 | CH₂CF₃ | H | Cl | F | CH₂CH₂CH₂OS(=O)₂CF₃ | O |
| A-0354 | CH₂CF₃ | H | Cl | F | CH₂CH₂CH₂CH₂OS(=O)₂CF₃ | O |
| A-0355 | CH₂CF₃ | H | Cl | F | CH₂CH₂CH₂CH₂CH₂OS(=O)₂CF₃ | O |
| A-0356 | CH₂CF₃ | H | Cl | F | CH₂CH₂CH₂CH₂CH₂OS(=O)₂CF₂CF₂CF₂CF₃ | O |
| A-0357 | CH₂CF₃ | H | Cl | F | CH₂CH₂OS(=O)₂Ph | O |
| A-0358 | CH₂CF₃ | H | Cl | F | CH₂CH₂OS(=O)₂Ph(4-Me) | O |
| A-0359 | CH₂CF₃ | H | Cl | F | CH₂CH₂CH₂OS(=O)₂Ph | O |
| A-0360 | CH₂CF₃ | H | Cl | F | CH₂CH₂CH₂OS(=O)₂Ph(4-Me) | O |
| A-0361 | CH₂CF₃ | H | Cl | F | CH₂CH₂NH₂ | O |
| A-0362 | CH₂CF₃ | H | Cl | F | CH₂CH₂CH₂NH₂ | O |
| A-0363 | CH₂CF₃ | H | Cl | F | CH₂CH₂CH₂CH₂NH₂ | O |
| A-0364 | CH₂CF₃ | H | Cl | F | CH₂CH₂CH₂CH₂CH₂NH₂ | O |
| A-0365 | CH₂CF₃ | H | Cl | F | CH₂CH₂CH₂CH₂CH₂CH₂NH₂ | O |
| A-0366 | CH₂CF₃ | H | Cl | F | CH₂CH₂CH₂CH₂CH₂CH₂CH₂NH₂ | O |
| A-0367 | CH₂CF₃ | H | Cl | F | CH₂CH(Me)NH₂ | O |
| A-0368 | CH₂CF₃ | H | Cl | F | CH₂C*H(Me)NH₂: (R) | O |
| A-0369 | CH₂CF₃ | H | Cl | F | CH₂C*H(Me)NH₂: (S) | O |
| A-0370 | CH₂CF₃ | H | Cl | F | CH₂CH(Et)NH₂ | O |
| A-0371 | CH₂CF₃ | H | Cl | F | CH₂CH(*i*-Pr)NH₂ | O |
| A-0372 | CH₂CF₃ | H | Cl | F | CH(Me)CH₂NH₂ | O |
| A-0373 | CH₂CF₃ | H | Cl | F | CH(Et)CH₂NH₂ | O |
| A-0374 | CH₂CF₃ | H | Cl | F | CH(*i*-Pr)CH₂NH₂ | O |
| A-0375 | CH₂CF₃ | H | Cl | F | CH₂CH₂CH(Me)NH₂ | O |
| A-0376 | CH₂CF₃ | H | Cl | F | CH₂CH(Me)CH₂NH₂ | O |
| A-0377 | CH₂CF₃ | H | Cl | F | CH(Me)CH₂CH₂NH₂ | O |
| A-0378 | CH₂CF₃ | H | Cl | F | CH₂CH(Me)NH(*c*-Pr) | O |
| A-0379 | CH₂CF₃ | H | Cl | F | CH₂CH(Me)NHOMe | O |
| A-0380 | CH₂CF₃ | H | Cl | F | CH₂CH(Me)N(OMe)S(=O)₂Ph(2-NO₂) | O |
| A-0381 | CH₂CF₃ | H | Cl | F | CH₂CH(Me)N(OMe)S(=O)₂Ph(4-NO₂) | O |
| A-0382 | CH₂CF₃ | H | Cl | F | CH₂CH₂N(Me)(*t*-Bᵤ) | O |
| A-0383 | CH₂CF₃ | H | Cl | F | CH₂CH₂CH₂N(Me)(*t*-Bᵤ) | O |
| A-0384 | CH₂CF₃ | H | Cl | F | CH₂CH₂CH₂CH₂N(Me)(*t*-Bu) | O |
| A-0385 | CH₂CF₃ | H | Cl | F | CH₂CH₂NHCH₂CF₃ | O |
| A-0386 | CH₂CF₃ | H | Cl | F | CH₂CH₂CH₂NHCH₂CF₃ | O |
| A-0387 | CH₂CF₃ | H | Cl | F | CH₂CH₂CH₂CH₂NHCH₂CF₃ | O |
| A-0388 | CH₂CF₃ | H | Cl | F | CH₂CH₂NHC(=O)C(Me)(CF₃)₂ | O |
| A-0389 | CH₂CF₃ | H | Cl | F | CH₂CH₂NHC(=O)Me | O |
| A-0390 | CH₂CF₃ | H | Cl | F | CH₂CH₂NHC(=O)(*t* -Bu) | O |

**[Table 10]**

| Compound Number | R¹ | R² | R³ | R⁴ | R⁵ | A |
|---|---|---|---|---|---|---|
| A-0391 | CH₂CF₃ | H | Cl | F | CH₂CH₂CH₂CH₂NHC(=O)CH(CH₃)₂ | O |
| A-0392 | CH₂CF₃ | H | Cl | F | CH₂CH₂CH₂CH₂NHC(=O)(*t*-Bu) | O |
| A-0393 | CH₂CF₃ | H | Cl | F | CH₂CH₂CH₂CH₂NHC(=O)CH₂(*t*-Bu) | O |
| A-0394 | CH₂CF₃ | H | Cl | F | CH₂CH₂NHC(=O)CF₃ | O |
| A-0395 | CH₂CF₃ | H | Cl | F | CH₂CH₂NHC(=O)CH₂CF₃ | O |
| A-0396 | CH₂CF₃ | H | Cl | F | CH₂CH₂NHC(=O)CF₂CF₃ | O |
| A-0397 | CH₂CF₃ | H | Cl | F | CH₂CH₂CH₂NHC(=O)CF₃ | O |
| A-0398 | CH₂CF₃ | H | Cl | F | CH₂CH₂CH₂NHC(=O)CH₂CF₃ | O |
| A-0399 | CH₂CF₃ | H | Cl | F | CH₂CH₂CH₂NHC(=O)CF₂CF₃ | O |
| A-0400 | CH₂CF₃ | H | Cl | F | CH₂CH₂CH₂CH₂NHC(=O)CF₃ | O |
| A-0401 | CH₂CF₃ | H | Cl | F | CH₂CH₂CH₂CH₂NHC(=O)CH₂CF₃ | O |
| A-0402 | CH₂CF₃ | H | Cl | F | CH₂CH₂CH₂CH₂NHC(=O)CF₂CF₃ | O |
| A-0403 | CH₂CF₃ | H | Cl | F | CH₂CH₂CH₂CH₂NHC(=O)CF(CF₃)₂ | O |
| A-0404 | CH₂CF₃ | H | Cl | F | CH₂CH₂CH₂CH₂NHC(=O)CCl₃ | O |
| A-0405 | CH₂CF₃ | H | Cl | F | CH₂CH(Me)NHC(=O)CF₃ | O |
| A-0406 | CH₂CF₃ | H | Cl | F | CH₂CH₂NHC(=O)Ph | O |
| A-0407 | CH₂CF₃ | H | Cl | F | CH₂CH₂NHC(=O)Ph(4-CF₃) | O |
| A-0408 | CH₂CF₃ | H | Cl | F | CH₂CH₂CH₂NHC(=O)Ph(4-CF₃) | O |
| A-0409 | CH₂CF₃ | H | Cl | F | CH₂CH₂CH₂CH₂NHC(=O)Ph(4-CF₃) | O |
| A-0410 | CH₂CF₃ | H | Cl | F | CH₂CH₂NHC(=O)O(*t*-Bu) | O |
| A-0411 | CH₂CF₃ | H | Cl | F | CH₂CH₂CH₂NHC(=O)O(*t*-Bu) | O |
| A-0412 | CH₂CF₃ | H | Cl | F | CH₂CH₂CH₂CH₂NHC(=O)O(*t*-Bu) | O |
| A-0413 | CH₂CF₃ | H | Cl | F | CH₂CH₂CH₂CH₂NHC(=O)OCH(CH₃)₂ | O |
| A-0414 | CH₂CF₃ | H | Cl | F | CH₂CH(Me)NHC(=O)O(*t*-Bu) | O |
| A-0415 | CH₂CF₃ | H | Cl | F | CH₂C*H(Me)NHC(=O)O(*t*-Bu) : (R) | O |
| A-0416 | CH₂CF₃ | H | Cl | F | CH₂C*H(Me)NHC(=O)O(*t*-Bu) : (S) | O |
| A-0417 | CH₂CF₃ | H | Cl | F | CH₂CH(Et)NHC(=O)O(*t*-Bu) | O |
| A-0418 | CH₂CF₃ | H | Cl | F | CH₂CH(*i*-Pr)NHC(=O)O(*t*-Bu) | O |
| A-0419 | CH₂CF₃ | H | Cl | F | CH(Me)CH₂NHC(=O)O(*t*-Bu) | O |
| A-0420 | CH₂CF₃ | H | Cl | F | CH(Et)CH₂NHC(=O)O(*t*-Bu) | O |
| A-0421 | CH₂CF₃ | H | Cl | F | CH(*i*-Pr)CH₂NHC(=O)O(*t*-Bu) | O |
| A-0422 | CH₂CF₃ | H | Cl | F | CH₂CH(Me)CH₂NHC(=O)O(*t*-Bu) | O |
| A-0423 | CH₂CF₃ | H | Cl | F | CH₂CH₂NHC(=O)OC(Me)₂CF₃ | O |
| A-0424 | CH₂CF₃ | H | Cl | F | CH₂CH₂CH₂NHC(=O)OCH₂CF₃ | O |
| A-0425 | CH₂CF₃ | H | Cl | F | CH₂CH₂CH₂NHC(=O)OCH₂CH₂CF₃ | O |
| A-0426 | CH₂CF₃ | H | Cl | F | CH₂CH₂CH₂CH₂NHC(=O)OCH₂C Cl ₃ | O |
| A-0427 | CH₂CF₃ | H | Cl | F | CH₂CH₂CH₂CH₂NHC(=O)OCH₂CF₃ | O |
| A-0428 | CH₂CF₃ | H | Cl | F | CH₂CH₂NHC(=O)NH(*t*-Bu) | O |
| A-0429 | CH₂CF₃ | H | Cl | F | CH₂CH₂CH₂NHC(=O)NH(*t*-Bu) | O |
| A-0430 | CH₂CF₃ | H | Cl | F | CH₂CH₂CH₂CH₂NHC(=O)NHEt | O |
| A-0431 | CH₂CF₃ | H | Cl | F | CH₂CH₂CH₂CH₂NHC(=O)NH(*t*-Bu) | O |
| A-0432 | CH₂CF₃ | H | Cl | F | CH₂CH₂CH₂NHC(=O)NHCH₂CCl₃ | O |
| A-0433 | CH₂CF₃ | H | Cl | F | CH₂CH₂CH₂NHC(=O)NHCH₂CH₂F | O |
| A-0434 | CH₂CF₃ | H | Cl | F | CH₂CH₂CH₂NHC(=O)NHCH₂CF₃ | O |

**[Table 11]**

| Compound Number | R¹ | R² | R³ | R⁴ | R⁵ | A |
|---|---|---|---|---|---|---|
| A-0435 | CH₂CF₃ | H | Cl | F | CH₂CH₂CHCH₂NHC (=O)NHCH₂CCl₃ | O |
| A-0436 | CH₂CF₃ | H | Cl | F | CH₂CH₂CH₂CH₂NHC(=O)NHCH₂CH₂F | O |
| A-0437 | CH₂CF₃ | H | Cl | F | CH₂CH₂CH₂CH₂NHC(=O)NHCH₂CF₃ | O |
| A-0438 | CH₂CF₃ | H | Cl | F | CH₂CH₂NHS(=O)Me | O |
| A-0439 | CH₂CF₃ | H | Cl | F | CH₂CH₂CH₂NHS(=O)Me | O |
| A-0440 | CH₂CF₃ | H | Cl | F | CH₂CH₂CH₂CH₂NHS(=O)Me | O |
| A-0441 | CH₂CF₃ | H | Cl | F | CH₂CH₂CH₂CH₂NHS(=O)CH(CH₃)₂ | O |
| A-0442 | CH₂CF₃ | H | Cl | F | CH₂CH₂CH₂CH₂CH₂NHS(=O)Me | O |
| A-0443 | CH₂CF₃ | H | Cl | F | CH₂CH₂NHS(=O)CHF₂ | O |
| A-0444 | CH₂CF₃ | H | Cl | F | CH₂CH₂CH₂NHS(=O)CHF₂ | O |
| A-0445 | CH₂CF₃ | H | Cl | F | CH₂CH₂CH₂CH₂NHS(=O)CHF₂ | O |
| A-0446 | CH₂CF₃ | H | Cl | F | CH₂CH₂CH₂CH₂CH₂NHS(=O)CHF₂ | O |
| A-0447 | CH₂CF₃ | H | Cl | F | CH₂CH(Me)NHS(=O)CHF₂ | O |
| A-0448 | CH₂CF₃ | H | Cl | F | CH(Me)CH₂NHS(=O)CHF₂ | O |
| A-0449 | CH₂CF₃ | H | Cl | F | CH₂CH₂CH(Me)NHS(=O)CHF₂ | O |
| A-0450 | CH₂CF₃ | H | Cl | F | CH₂CH(Me)CH₂NHS(=O)CHF₂ | O |
| A-0451 | CH₂CF₃ | H | Cl | F | CH(Me)CH₂CH₂NHS(=O)CHF₂ | O |
| A-0452 | CH₂CF₃ | H | Cl | F | CH₂CH₂NHS(=O)CF₃ | O |
| A-0453 | CH₂CF₃ | H | Cl | F | CH₂CH₂CH₂NHS(=O)CF₃ | O |
| A-0454 | CH₂CF₃ | H | Cl | F | CH₂CH₂CH₂CH₂NHS(=O)CF₃ | O |
| A-0455 | CH₂CF₃ | H | Cl | F | CH₂CH₂CH₂CH₂CH₂NHS(=O)CF₃ | O |
| A-0456 | CH₂CF₃ | H | Cl | F | CH₂CH₂CH₂NHS(=O)Ph | O |
| A-0457 | CH₂CF₃ | H | Cl | F | CH₂CH₂CH₂CH₂NHS(=O)Ph | O |
| A-0458 | CH₂CF₃ | H | Cl | F | CH₂CH₂CH₂CH₂CH₂NHS(=O)Ph | O |
| A-0459 | CH₂CF₃ | H | Cl | F | CH₂CH₂CH₂CH₂CH₂NHS(=O)Ph(4-CF₃) | O |
| A-0460 | CH₂CF₃ | H | Cl | F | CH₂CH(Me)NHS(=O)Ph(4-CF₃) | O |
| A-0461 | CH₂CF₃ | H | Cl | F | CH₂CH(Et)NHS(=O)Ph(4-CF₃) | O |
| A-0462 | CH₂CF₃ | H | Cl | F | CH(Me)CH₂NHS(=O)Ph(4-CF₃) | O |
| A-0463 | CH₂CF₃ | H | Cl | F | CH(Et)CH₂NHS(=O)Ph(4-CF₃) | O |
| A-0464 | CH₂CF₃ | H | Cl | F | CH₂CH(Et)CH₂NHS (=O)Ph(4-CF₃) | O |
| A-0465 | CH₂CF₃ | H | Cl | F | CH₂CH₂NHS(=O)₂Me | O |
| A-0466 | CH₂CF₃ | H | Cl | F | CH₂CH₂CH₂NHS(=O)₂Me | O |
| A-0467 | CH₂CF₃ | H | Cl | F | CH₂CH₂CH₂CH₂NHS(=O)₂Me | O |
| A-0468 | CH₂CF₃ | H | Cl | F | CH₂CH₂CH₂CH₂NHS(=O)₂CH(CH₃)₂ | O |
| A-0469 | CH₂CF₃ | H | Cl | F | CH₂CH₂CH₂CH₂CH₂NHS(=O)₂Me | O |
| A-0470 | CH₂CF₃ | H | Cl | F | CH₂CH₂NHS(=O)₂CHF₂ | O |
| A-0471 | CH₂CF₃ | H | Cl | F | CH₂CH₂CH₂NHS(=O)₂CHF₂ | O |
| A-0472 | CH₂CF₃ | H | Cl | F | CH₂CH₂CH₂CH₂NHS(=O)₂CHF₂ | O |
| A-0473 | CH₂CF₃ | H | Cl | F | CH₂CH₂CH₂CH₂CH₂NHS(=O)₂CHF₂ | O |
| A-0474 | CH₂CF₃ | H | Cl | F | CH₂CH(Me)NHS(=O)₂CHF₂ | O |
| A-0475 | CH₂CF₃ | H | Cl | F | CH(Me)CH₂NHS(=O)₂CHF₂ | O |
| A-0476 | CH₂CF₃ | H | Cl | F | CH₂CH₂CH(Me)NHS(=O)₂CHF₂ | O |
| A-0477 | CH₂CF₃ | H | Cl | F | CH₂CH(Me)CH₂NHS(=O)₂CHF₂ | O |
| A-0478 | CH₂CF₃ | H | Cl | F | CH(Me)CH₂CH₂NHS(=O)₂CHF₂ | O |

**[Table 12]**

| Compound Number | R¹ | R² | R³ | R⁴ | R⁵ | A |
|---|---|---|---|---|---|---|
| A-0479 | CH₂CF₃ | H | Cl | F | CH₂CH(Me)N(Me)S(=O)₂CHF₂ | O |
| A-0480 | CH₂CF₃ | H | Cl | F | CH(Me)CH₂N(Me)S(=O)₂CHF₂ | O |
| A-0481 | CH₂CF₃ | H | Cl | F | CH₂CH₂CH(Me)N(Me)S(=O)₂CHF₂ | O |
| A-0482 | CH₂CF₃ | H | Cl | F | CH₂CH(Me)CH₂N(Me)S(=O)₂CHF₂ | O |
| A-0483 | CH₂CF₃ | H | Cl | F | CH(Me)CH₂CH₂N(Me)S(=O)₂CHF₂ | O |
| A-0484 | CH₂CF₃ | H | Cl | F | CH₂CH₂CH₂NHS(=O)₂Ph | O |
| A-0485 | CH₂CF₃ | H | Cl | F | CH₂CH₂CH₂CH₂NHS(=O)₂Ph | O |
| A-0486 | CH₂CF₃ | H | Cl | F | CH₂CH₂CH₂CH₂CH₂NHS(=O)₂Ph | O |
| A-0487 | CH₂CF₃ | H | Cl | F | CH₂CH₂CH₂CH₂CH₂NHS(=O)₂Ph(4-CF₃) | O |
| A-0488 | CH₂CF₃ | H | Cl | F | CH₂CH(Me)NHS(=O)₂Ph(4-CF₃) | a |
| A-0489 | CH₂CF₃ | H | Cl | F | CH₂CH(Et)NHS(=O)₂Ph(4-CF₃) | O |
| A-0490 | CH₂CF₃ | H | Cl | F | CH(Me)CH₂NHS(=O)₂Ph(4-CF₃) | O |
| A-0491 | CH₂CF₃ | H | Cl | F | CH(Et)CH₂NHS(=O)₂Ph(4-CF₃) | O |
| A-0492 | CH₂CF₃ | H | Cl | F | CH₂CH(Me)CH₂NHS(=O)₂Ph(4-CF₃) | O |
| A-0493 | CH₂CF₃ | H | Cl | F | CH₂CH₂SiMe₃ | O |
| A-0494 | CH₂CF₃ | H | Cl | F | CH₂CH₂CH₂SiMe₃ | O |
| A-0495 | CH₂CF₃ | H | Cl | F | CH₂CH₂CH₂CH₂SiMe₃ | O |
| A-0496 | CH₂CF₃ | H | Cl | F | CH₂Ph | O |
| A-0497 | CH₂CF₃ | H | Cl | F | CH₂Ph(2-CF₃) | O |
| A-0498 | CH₂CF₃ | H | Cl | F | CH₂Ph(3-CF₃) | O |
| A-0499 | CH₂CF₃ | H | Cl | F | CH₂Ph(4-CF₃) | O |
| A-0500 | CH₂CF₃ | H | Cl | F | CH₂Ph(2-OCF₃) | O |
| A-0501 | CH₂CF₃ | H | Cl | F | CH₂Ph(3-OCF₃) | O |
| A-0502 | CH₂CF₃ | H | Cl | F | CH₂Ph(4-OCF₃) | O |
| A-0503 | CH₂CF₃ | H | Cl | F | CH₂Ph(2-SCF₃) | O |
| A-0504 | CH₂CF₃ | H | Cl | F | CH₂Ph(3-SCF₃) | O |
| A-0505 | CH₂CF₃ | H | Cl | F | CH₂Ph(4-SCF₃) | O |
| A-0506 | CH₂CF₃ | H | Cl | F | CH₂Ph(3-CH₂SCF₃) | O |
| A-0507 | CH₂CF₃ | H | Cl | F | CH₂Ph(4-F) | O |
| A-0508 | CH₂CF₃ | H | Cl | F | CH₂Ph(4-Cl) | O |
| A-0509 | CH₂CF₃ | H | Cl | F | CH₂Ph(4-Br) | O |
| A-0510 | CH₂CF₃ | H | Cl | F | CH₂Ph(4-Me) | O |
| A-0511 | CH₂CF₃ | H | Cl | F | CH₂Ph[4-(*t*-Bu)] | O |
| A-0512 | CH₂CF₃ | H | Cl | F | CH₂Ph(4-CN) | O |
| A-0513 | CH₂CF₃ | H | Cl | F | CH₂Ph(4-NO₂) | O |
| A-0514 | CH₂CF₃ | H | Cl | F | CH₂Ph(4-OCHF₂) | O |
| A-0515 | CH₂CF₃ | H | Cl | F | CH₂Ph(4-SCHF₂) | O |
| A-0516 | CH₂CF₃ | H | Cl | F | CH₂Ph(4-CH₂SCF₃) | O |
| A-0517 | CH₂CF₃ | H | Cl | F | CH₂Ph[4-CF(CF₃)_{2]} | O |
| A-0518 | CH₂CF₃ | H | Cl | F | CH₂Ph(4-CH₂SCF₃) | O |
| A-0519 | CH₂CF₃ | H | Cl | F | CH₂Ph[4-Ph(4-CF₃)] | O |
| A-0520 | CH₂CF₃ | H | Cl | F | CH₂Ph(2,4-Cl₂) | O |
| A-0521 | CH₂CF₃ | H | Cl | F | CH₂Ph[2,5-(CF₃)₂] | O |
| A-0522 | CH₂CF₃ | H | Cl | F | CH₂Ph(3,4-Cl₂) | O |

**[Table 13]**

| Compound Number | R¹ | R² | R³ | R⁴ | R⁵ | A |
|---|---|---|---|---|---|---|
| A-0523 | CH₂CF₃ | H | Cl | F | CH₂Ph(3-CF₃-4-F) | O |
| A-0524 | CH₂CF₃ | H | Cl | F | CH₂Ph(3-CF₃-4- Cl ) | O |
| A-0525 | CH₂CF₃ | H | Cl | F | CH₂Ph(3-F-4-CF₃) | O |
| A-0526 | CH₂CF₃ | H | Cl | F | CH₂Ph(2,4,6-F₃) | O |
| A-0527 | CH₂CF₃ | H | Cl | F | CH₂Ph(3,4,5-F₃) | O |
| A-0528 | CH₂CF₃ | H | Cl | F | CH₂Ph(2,3,4-F₃) | O |
| A-0529 | CH₂CF₃ | H | Cl | F | CH₂Ph(3,4,5- Cl ₃) | O |
| A-0530 | CH₂CF₃ | H | Cl | F | CH₂CH₂Ph | O |
| A-0531 | CH₂CF₃ | H | Cl | F | CH₂CH₂Ph(4-F) | O |
| A-0532 | CH₂CF₃ | H | Cl | F | CH₂CH₂Ph(4-Cl) | O |
| A-0533 | CH₂CF₃ | H | Cl | F | CH₂CH₂Ph(4-Br) | O |
| A-0534 | CH₂CF₃ | H | Cl | F | CH₂CH₂Ph[4-(*t*-Bu)] | O |
| A-0535 | CH₂CF₃ | H | Cl | F | CH₂CH₂Ph(2-CF₃) | O |
| A-0536 | CH₂CF₃ | H | Cl | F | CH₂CH₂Ph(3-CF₃) | O |
| A-0537 | CH₂CF₃ | H | Cl | H | CH₂CH₂Ph(4-CF₃) | O |
| A-0538 | CH₂CF₃ | H | Cl | F | CH₂CH₂Ph(4-CF₃) | O |
| A-0539 | CH₂CF₃ | H | Cl | F | CH₂CH₂Ph(4-CF(CF₃)₂) | O |
| A-0540 | CH₂CF₃ | H | Cl | F | CH₂CH₂Ph[4-(c-Pr)] | O |
| A-0541 | CH₂CF₃ | H | Cl | F | CH₂CH₂Ph{4-[*c* -Pr(2,2-F₂)]} | O |
| A-0542 | CH₂CF₃ | H | Cl | F | CH₂CH₂Ph(4-OMe) | O |
| A-0543 | CH₂CF₃ | H | Cl | F | CH₂CH₂Ph(3-OCF₃) | O |
| A-0544 | CH₂CF₃ | H | Cl | F | CH₂CH₂Ph(4-OCHF₂) | O |
| A-0545 | CH₂CF₃ | H | Cl | F | CH₂CH₂Ph(4-OCF₃) | O |
| A-0546 | CH₂CF₃ | H | Cl | F | CH₂CH₂Ph(4-SMe) | O |
| A-0547 | CH₂CF₃ | H | Cl | F | CH₂CH₂Ph(4-SCHF₂) | O |
| A-0548 | CH₂CF₃ | H | Cl | F | CH₂CH₂Ph(4-SCF₃) | O |
| A-0549 | CH₂CF₃ | H | Cl | F | CH₂CH₂Ph[4-S(=O)Me] | O |
| A-0550 | CH₂CF₃ | H | Cl | F | CH₂CH₂Ph[4-S(=O)CF_{3]} | O |
| A-0551 | CH₂CF₃ | H | Cl | F | CH₂CH₂Ph[4-S(=O)₂Me] | O |
| A-0552 | CH₂CF₃ | H | Cl | F | CH₂CH₂Ph[4-S(=O)₂CF_{3]} | O |
| A-0553 | CH₂CF₃ | H | Cl | F | CH₂CH₂Ph(4-CH₂SMe) | O |
| A-0554 | CH₂CF₃ | H | Cl | F | CH₂CH₂Ph(4-CH₂SCF₃) | O |
| A-0555 | CH₂CF₃ | H | Cl | F | CH₂CH₂Ph[4-OS(=O)₂Me] | O |
| A-0556 | CH₂CF₃ | H | Cl | F | CH₂CH₂Ph[4-OS(=O)₂CF_{3]} | O |
| A-0557 | CH₂CF₃ | H | Cl | F | CH₂CH₂Ph[4-Ph(4-CF₃)] | O |
| A-0558 | CH₂CF₃ | H | Cl | F | CH₂CH₂Ph(4-CH₂Ph) | O |
| A-0559 | CH₂CF₃ | H | Cl | F | CH₂CH₂Ph(4-OCH₂Ph) | O |
| A-0560 | CH₂CF₃ | H | Cl | F | CH₂CH₂Ph(4-CN) | O |
| A-0561 | CH₂CF₃ | H | Cl | F | CH₂CH₂Ph(4-NO₂) | O |
| A-0562 | CH₂CF₃ | H | Cl | F | CH₂CH₂Ph(2,4-Cl₂) | O |
| A-0563 | CH₂CF₃ | H | Cl | F | CH₂CH₂Ph(3,4-Cl₂) | O |
| A-0564 | CH₂CF₃ | H | Cl | F | CH₂CH₂Ph(3-CF₃-4-F) | O |
| A-0565 | CH₂CF₃ | H | Cl | F | CH₂CH₂Ph(2-CF₃-4-F) | O |
| A-0566 | CH₂CF₃ | H | Cl | F | CH₂CH₂Ph(3-F-4-CF₃) | O |

**[Table 14]**

| Compound Number | R¹ | R² | R³ | R⁴ | R⁵ | A |
|---|---|---|---|---|---|---|
| A-0567 | CH₂CF₃ | H | Cl | F | CH₂CH₂Ph(2-F-4-CF₃) | O |
| A-0568 | CH₂CF₃ | H | Cl | F | CH₂CH₂Ph(3- Cl -4-OCHF₂) | O |
| A-0569 | CH₂CF₃ | H | Cl | F | CH₂CH₂Ph(3,4,5-Cl₃) | O |
| A-0570 | CH₂CF₃ | H | Cl | F | CH₂CH₂Ph(2,3,4-F₃) | O |
| A-0571 | CH₂CF₃ | H | Cl | F | CH₂CH₂Ph(2,4,5-F₃) | O |
| A-0572 | CH₂CF₃ | H | Cl | F | CH₂CH₂Ph(3,4,5-F₃) | O |
| A-0573 | CH₂CF₃ | H | Cl | F | CH₂CH₂Ph(2,4,6-F₃) | O |
| A-0574 | CH₂CF₃ | H | Cl | F | CH₂CH₂CH₂Ph | O |
| A-0575 | CH₂CF₃ | H | Cl | F | CH₂CH₂CH₂Ph(3-CF₃) | O |
| A-0576 | CH₂CF₃ | H | Cl | F | CH₂CH₂CH₂Ph(4-CF₃) | O |
| A-0577 | CH₂CF₃ | H | Cl | F | CH₂CH₂CH₂Ph(4-F) | O |
| A-0578 | CH₂CF₃ | H | Cl | F | CH₂CH₂CH₂Ph[4-(*t*-Bu)] | O |
| A-0579 | CH₂CF₃ | H | Cl | F | CH₂CH₂CH₂Ph(4-CN) | O |
| A-0580 | CH₂CF₃ | H | Cl | F | CH₂CH₂CH₂Ph(4-CF₃) | O |
| A-0581 | CH₂CF₃ | H | Cl | F | CH₂CH₂CH₂Ph(4-OCHF₂) | O |
| A-0582 | CH₂CF₃ | H | Cl | F | CH₂CH₂CH₂Ph(4-OCF₃) | O |
| A-0583 | CH₂CF₃ | H | Cl | F | CH₂CH₂CH₂Ph(4-SCHF₂) | O |
| A-0584 | CH₂CF₃ | H | Cl | F | CH₂CH₂CH₂Ph(4-SCF₃) | O |
| A-0585 | CH₂CF₃ | H | Cl | F | CH₂CH₂CH₂Ph[4-CF(CF₃)₂] | O |
| A-0586 | CH₂CF₃ | H | Cl | F | CH₂CH₂CH₂Ph(3,4,5-F₃) | O |
| A-0587 | CH₂CF₃ | H | Cl | F | CH₂CH₂CH₂Ph(2,4,6-F₃) | O |
| A-0588 | CH₂CF₃ | H | Cl | F | CH₂CH₂CH₂CH₂Ph | O |
| A-0589 | CH₂CF₃ | H | Cl | F | CH₂CH₂CH₂CH₂Ph(4-F) | O |
| A-0590 | CH₂CF₃ | H | Cl | F | CH₂CH₂CH₂CH₂Ph(4-CF₃) | O |
| A-0591 | CH₂CF₃ | H | Cl | F | CH₂CH₂CH₂CH₂Ph(4-OCF₃) | O |
| A-0592 | CH₂CF₃ | H | Cl | F | CH₂CH₂CH₂CH₂Ph(4-SCF₃) | O |
| A-0593 | CH₂CF₃ | H | Cl | F | CH₂CH₂CH₂CH₂CH₂Ph | O |
| A-0594 | CH₂CF₃ | H | Cl | F | CH₂CH₂CH₂CH₂CH₂CH₂Ph | O |
| A-0595 | CH₂CF₃ | H | Cl | F | CH₂CF₂Ph(4-F) | O |
| A-0596 | CH₂CF₃ | H | Cl | F | CH₂CF₂Ph(4-CF₃) | O |
| A-0597 | CH₂CF₃ | H | Cl | H | CH₂CF₂Ph(3,4,5-F₃) | O |
| A-0598 | CH₂CF₃ | H | Cl | F | CH₂CF₂Ph(3,4,5-F₃) | O |
| A-0599 | CH₂CF₃ | H | Cl | F | CH₂CH₂OPh | O |
| A-0600 | CH₂CF₃ | H | Cl | F | CH₂CH₂OPh(4-F) | O |
| A-0601 | CH₂CF₃ | H | Cl | F | CH₂CH₂OPh(4-CF₃) | O |
| A-0602 | CH₂CF₃ | H | Cl | F | CH₂CH₂CH₂OPh | O |
| A-0603 | CH₂CF₃ | H | Cl | F | CH₂CH₂CH₂OPh(4- Cl ) | O |
| A-0604 | CH₂CF₃ | H | Cl | F | CH₂CH₂CH₂OPh(4-CF₃) | O |
| A-0605 | CH₂CF₃ | H | Cl | F | CH₂CH₂CH₂CH₂OPh(4-CF₃) | O |
| A-0606 | CH₂CF₃ | H | Cl | F | CH₂CH₂CH₂CH₂OPh(4-OCF₃) | O |
| A-0607 | CH₂CF₃ | H | Cl | F | CH₂CH₂CH₂CH₂CH₂OPh(4-CF₃) | O |
| A-0608 | CH₂CF₃ | H | Cl | F | CH₂CH₂OCH₂Ph | O |
| A-0609 | CH₂CF₃ | H | Cl | F | CH₂CH₂CH₂OCH₂Ph | O |
| A-0610 | CH₂CF₃ | H | Cl | F | CH₂CH₂CH₂CH₂OCH₂Ph | O |

**[Table 15]**

| Compound Number | R¹ | R² | R³ | R⁴ | R⁵ | A |
|---|---|---|---|---|---|---|
| A-0611 | CH₂CF₃ | H | Cl | F | CH₂CH₂CH₂CH₂CH₂OCH₂Ph | O |
| A-0612 | CH₂CF₃ | H | Cl | F | CH₂CH₂SPh | O |
| A-0613 | CH₂CF₃ | H | Cl | F | CH₂CH₂CH₂SPh | O |
| A-0614 | CH₂CF₃ | H | Cl | F | CH₂CH₂CH₂SPh(4-F) | O |
| A-0615 | CH₂CF₃ | H | Cl | F | CH₂CH₂CH₂SPh(4-Cl) | O |
| A-0616 | CH₂CF₃ | H | Cl | F | CH₂CH₂CH₂SPh(4-Br) | O |
| A-0617 | CH₂CF₃ | H | Cl | F | CH₂CH₂CH₂SPh[4-(*t*-Bu)] | O |
| A-0618 | CH₂CF₃ | H | Cl | F | CH₂CH₂CH₂SPh(3-CF₃) | O |
| A-0619 | CH₂CF₃ | H | Cl | F | CH₂CH₂CH₂SPh(4-CF₃) | O |
| A-0620 | CH₂CF₃ | H | Cl | F | CH₂CH₂CH₂SPh(3-SCF₃) | O |
| A-0621 | CH₂CF₃ | H | Cl | F | CH₂CH₂CH₂SPh(4-SCF₃) | O |
| A-0622 | CH₂CF₃ | H | Cl | F | CH₂CH₂CH₂CH₂SPh | O |
| A-0623 | CH₂CF₃ | H | Cl | F | CH₂CH₂CH₂CH₂SPh(4-F) | O |
| A-0624 | CH₂CF₃ | H | Cl | F | CH₂CH₂CH₂CH₂SPh(4- Cl ) | O |
| A-0625 | CH₂CF₃ | H | Cl | F | CH₂CH₂CH₂CH₂SPh[4-(*t*-Bu)] | O |
| A-0626 | CH₂CF₃ | H | Cl | F | CH₂CH₂CH₂CH₂SPh(4-CF₃) | O |
| A-0627 | CH₂CF₃ | H | Cl | F | CH₂CH₂CH₂CH₂CH₂SPh | O |
| A-0628 | CH₂CF₃ | H | Cl | F | CH₂CH₂CH₂CH₂CH₂SPh(4-F) | O |
| A-0629 | CH₂CF₃ | H | Cl | F | CH₂CH₂CH₂CH₂CH₂SPh(4- Cl ) | O |
| A-0630 | CH₂CF₃ | H | Cl | F | CH₂CH₂CH₂CH₂CH₂SPh(4-CF₃) | O |
| A-0631 | CH₂CF₃ | H | Cl | F | CH₂CH₂S(=O)Ph | O |
| A-0632 | CH₂CF₃ | H | Cl | F | CH₂CH₂CH₂S(=O)Ph | O |
| A-0633 | CH₂CF₃ | H | Cl | F | CH₂CH₂CH₂S(=O)Ph(4-F) | O |
| A-0634 | CH₂CF₃ | H | Cl | F | CH₂CH₂CH₂S(=O)Ph[4-(*t*-Bu)] | O |
| A-0635 | CH₂CF₃ | H | Cl | F | CH₂CH₂CH₂S(=O)Ph(4-CF₃) | O |
| A-0636 | CH₂CF₃ | H | Cl | F | CH₂CH₂S(=O)₂Ph | O |
| A-0637 | CH₂CF₃ | H | Cl | F | CH₂CH₂S(=O)₂Ph(4-CF₃) | O |
| A-0638 | CH₂CF₃ | H | Cl | F | CH₂CH₂CH₂S(=O)₂Ph(4-F) | O |
| A-0639 | CH₂CF₃ | H | Cl | F | CH₂CH₂CH₂S(=O)₂Ph(4- Cl ) | O |
| A-0640 | CH₂CF₃ | H | Cl | F | CH₂CH₂CH₂S(=O)₂Ph(4-CF₃) | O |
| A-0641 | CH₂CF₃ | H | Cl | F | CH₂CH₂CH₂CH₂S(=O)₂Ph | O |
| A-0642 | CH₂CF₃ | H | Cl | F | CH₂CH₂CH₂CH₂S(=O)₂Ph(4- Cl ) | O |
| A-0643 | CH₂CF₃ | H | Cl | F | CH₂CH₂CH₂CH₂S(=O)₂Ph(4-CF₃) | O |
| A-0644 | CH₂CF₃ | H | Cl | F | CH₂CH₂SCH₂Ph | O |
| A-0645 | CH₂CF₃ | H | Cl | F | CH₂CH₂CH₂SCH₂Ph | O |
| A-0646 | CH₂CF₃ | H | Cl | F | CH₂CH₂CH₂SCH₂Ph(2- Cl ) | O |
| A-0647 | CH₂CF₃ | H | Cl | F | CH₂CH₂CH₂SCH₂Ph(3- Cl ) | O |
| A-0648 | CH₂CF₃ | H | Cl | F | CH₂CH₂CH₂SCH₂Ph(4- Cl ) | O |
| A-0649 | CH₂CF₃ | H | Cl | F | CH₂CH₂CH₂SCH₂Ph(2-SCF₃) | O |
| A-0650 | CH₂CF₃ | H | Cl | F | CH₂CH₂CH₂SCH₂Ph(3-CF₃) | O |
| A-0651 | CH₂CF₃ | H | Cl | F | CH₂CH₂CH₂SCH₂Ph(4-CF₃) | O |
| A-0652 | CH₂CF₃ | H | Cl | F | CH₂CH₂CH₂SCH₂Ph(4-NO₂) | O |
| A-0653 | CH₂CF₃ | H | Cl | F | CH₂CH₂CH₂CH₂SCH₂Ph | O |
| A-0654 | CH₂CF₃ | H | Cl | F | CH₂CH₂CH₂CH₂SCH₂Ph(4- Cl ) | O |

**[Table 16]**

| Compound Number | R¹ | R² | R³ | R⁴ | R⁵ | A |
|---|---|---|---|---|---|---|
| A-0655 | CH₂CF₃ | H | Cl | F | CH₂CH₂CH₂CH₂SCH₂Ph(4-CF₃) | O |
| A-0656 | CH₂CF₃ | H | Cl | F | CH₂CH₂CH₂CH₂SCH₂Ph(4-CN) | O |
| A-0657 | CH₂CF₃ | H | Cl | F | CH₂CH₂CH₂CH₂CH₂SCH₂Ph | O |
| A-0658 | CH₂CF₃ | H | Cl | F | CH₂CH₂SCH₂CH₂Ph | O |
| A-0659 | CH₂CF₃ | H | Cl | F | CH₂CH₂CH₂SCH₂CH₂Ph | O |
| A-0660 | CH₂CF₃ | H | Cl | F | CH₂CH₂CH₂CH₂SCH₂CH₂Ph | O |
| A-0661 | CH₂CF₃ | H | Cl | F | CH₂CH₂SCH(Me)Ph | O |
| A-0662 | CH₂CF₃ | H | Cl | F | CH₂CH₂CH₂SCH(Me)Ph | O |
| A-0663 | CH₂CF₃ | H | Cl | F | CH₂CH₂CH₂CH₂SCH(Me)Ph | O |
| A-0664 | CH₂CF₃ | H | Cl | F | CH₂CH₂S(=O)CH₂Ph | O |
| A-0665 | CH₂CF₃ | H | Cl | F | CH₂CH₂S(=O)CH₂Ph(4-CF₃) | O |
| A-0666 | CH₂CF₃ | H | Cl | F | CH₂CH₂CH₂S(=O)CH₂Ph | O |
| A-0667 | CH₂CF₃ | H | Cl | F | CH₂CH₂CH₂S(=O)CH₂Ph(2-SCF₃) | O |
| A-0668 | CH₂CF₃ | H | Cl | F | CH₂CH₂CH₂S(=O)CH₂Ph(4-SCF₃) | O |
| A-0669 | CH₂CF₃ | H | Cl | F | CH₂CH₂CH₂S(=O)CH₂Ph(4-Cl) | O |
| A-0670 | CH₂CF₃ | H | Cl | F | CH₂CH₂CH₂S(=O)CH₂Ph(4-CF₃) | O |
| A-0671 | CH₂CF₃ | H | Cl | F | CH₂CH₂CH₂CH₂S(=O)CH₂Ph | O |
| A-0672 | CH₂CF₃ | H | Cl | F | CH₂CH₂CH₂CH₂S(=O)CH₂Ph(4-CF₃) | O |
| A-0673 | CH₂CF₃ | H | Cl | F | CH₂CH₂CH₂CH₂CH₂S(=O)CH₂Ph(4-CF₃) | O |
| A-0674 | CH₂CF₃ | H | Cl | F | CH₂CH₂S(=O)₂CH₂Ph | O |
| A-0675 | CH₂CF₃ | H | Cl | F | CH₂CH₂S(=O)₂CH₂Ph(4-CF₃) | O |
| A-0676 | CH₂CF₃ | H | Cl | F | CH₂CH₂CH₂S(=O)₂CH₂Ph | O |
| A-0677 | CH₂CF₃ | H | Cl | F | CH₂CH₂CH₂S(=O)₂CH₂Ph(4-Cl) | O |
| A-0678 | CH₂CF₃ | H | Cl | F | CH₂CH₂CH₂S(=O)₂CH₂Ph(4-CF₃) | O |
| A-0679 | CH₂CF₃ | H | Cl | F | CH₂CH₂CH₂CH₂S(=O)₂CH₂Ph | O |
| A-0680 | CH₂CF₃ | H | Cl | F | CH₂CH₂CH₂CH₂S(=O)₂CH₂Ph(4-CF₃) | O |
| A-0681 | CH₂CF₃ | H | Cl | F | CH₂CH₂CH₂CH₂CH₂S(=O)₂CH₂Ph | O |
| A-0682 | CH₂CF₃ | H | Cl | F | CH₂CH₂CH₂CH₂CH₂S(=O)₂CH₂Ph(4-CF₃) | O |
| A-0683 | CH₂CF₃ | H | Cl | F | CH₂CH₂ON=CH(*t*-Bu) | O |
| A-0684 | CH₂CF₃ | H | Cl | F | CH₂CH₂CH₂ON=CH(*t*-Bu) | O |
| A-0685 | CH₂CF₃ | H | Cl | F | CH₂CH₂CH₂CH₂ON=C(Me)(*c* -Pr) | O |
| A-0686 | CH₂CF₃ | H | Cl | F | CH₂CH₂ON=C(Me)CF₃ | O |
| A-0687 | CH₂CF₃ | H | Cl | F | CH₂CH₂CH₂ON=CHCF₃ | O |
| A-0688 | CH₂CF₃ | H | Cl | F | CH₂CH₂CH₂ON=C(Me)CF₃ | O |
| A-0689 | CH₂CF₃ | H | Cl | F | CH₂CH₂CH₂ON=C(Me)CCl₃ | O |
| A-0690 | CH₂CF₃ | H | Cl | F | CH₂CH₂CH₂CH₂ON=CHCF₃ | O |
| A-0691 | CH₂CF₃ | H | Cl | F | CH₂CH₂CH₂CH₂ON=C(Me)CF₃ | O |
| A-0692 | CH₂CF₃ | H | Cl | F | CH₂CH₂CH₂CH₂CH₂ON=C(Me)CF₃ | O |
| A-0693 | CH₂CF₃ | H | Cl | F | CH₂CH₂ON=CHPh | O |
| A-0694 | CH₂CF₃ | H | Cl | F | CH₂CH₂ON=CHPh(4-CF₃) | O |
| A-0695 | CH₂CF₃ | H | Cl | F | CH₂CH₂ON=CHPh(4-SCF₃) | O |
| A-0696 | CH₂CF₃ | H | Cl | F | CH₂CH₂CH₂ON=CHPh(3-CF₃) | O |
| A-0697 | CH₂CF₃ | H | Cl | F | CH₂CH₂CH₂ON=CHPh(4-CF₃) | O |
| A-0698 | CH₂CF₃ | H | Cl | F | CH₂CH₂CH₂ON=CHPh(4-SCF₃) | O |

**[Table 17]**

| Compound Number | R¹ | R² | R³ | R⁴ | R⁵ | A |
|---|---|---|---|---|---|---|
| A-0699 | CH₂CF₃ | H | Cl | F | CH₂CH₂CH₂ON=C(Me)Ph(4-CF₃) | O |
| A-0700 | CH₂CF₃ | H | Cl | F | CH₂CH₂CH₂CH₂ON=CHPh(4-SCF₃) | O |
| A-0701 | CH₂CF₃ | H | Cl | F | CH₂CH₂(adamant-1-yl) | O |
| A-0702 | CH₂CF₃ | H | Cl | F | CH₂CH₂CH₂(3-CF₃-pyrazol-1-yl) | O |
| A-0703 | CH₂CF₃ | H | Cl | F | CH₂CH₂CH₂CH₂(3-CF₃-pyrazol-1-yl) | O |
| A-0704 | CH₂CF₃ | H | Cl | F | CH₂CH₂CH₂CH₂CH₂(3-CF₃-pyrazol-1-yl) | O |
| A-0705 | CH₂CF₃ | H | Cl | F | CH₂(1-Me-4-CF₃-imidazol-5-yl) | O |
| A-0706 | CH₂CF₃ | H | Cl | F | CH₂CH₂(1-Me-4-CF₃-imidazol-5-yl) | O |
| A-0707 | CH₂CF₃ | H | Cl | F | CH₂CH₂CH₂(1-Me-4-CF₃-imidazol-5-yl) | O |
| A-0708 | CH₂CF₃ | H | Cl | F | CH₂CH₂CH₂(3-CF₃-1,2,4-triazol-1-yl) | O |
| A-0709 | CH₂CF₃ | H | Cl | F | CH₂CH₂CH₂CH₂(3-CF₃-1,2,4-triazol-1-yl) | O |
| A-0710 | CH₂CF₃ | H | Cl | F | CH₂CH₂CH₂CH₂CH₂(3-CF₃-1,2,4-triazol-1-yl) | O |
| A-0711 | CH₂CF₃ | H | Cl | F | CH₂CH₂CH₂(4-CF₃-thiazol-2-yl) | O |
| A-0712 | CH₂CF₃ | H | Cl | F | CH₂CH₂CH₂CH₂(4-CF₃-thiazol-2-yl) | O |
| A-0713 | CH₂CF₃ | H | Cl | F | CH₂CH₂CH₂CH₂CH₂(4-CF₃-thiazol-2-yl) | O |
| A-0714 | CH₂CF₃ | H | Cl | F | CH₂CH₂CH₂(2-CF₃-pyridin-5-yl) | O |
| A-0715 | CH₂CF₃ | H | Cl | F | CH₂CH₂CH₂CH₂(2-CF₃-pyridin-5-yl) | O |
| A-0716 | CH₂CF₃ | H | Cl | F | CH₂CH₂CH₂CH₂CH₂(2-CF₃-pyridin-5-yl) | O |
| A-0717 | CH₂CF₃ | H | Cl | F | CH₂(4-CF₃-pyrimidin-2-yl) | O |
| A-0718 | CH₂CF₃ | H | Cl | F | CH₂CH₂(4-CF₃-pyrimidin-2-yl) | O |
| A-0719 | CH₂CF₃ | H | Cl | F | CH₂CH₂CH₂(4-CF₃-pyrimidin-2-yl) | O |
| A-0720 | CH₂CF₃ | H | Cl | F | CH₂CH₂O(3-Cl-5-CF₃-pyridin-2-yl) | O |
| A-0721 | CH₂CF₃ | H | Cl | F | CH₂CH₂CH₂O(3-Cl-5-CF₃-pyridin-2-yl) | O |
| A-0722 | CH₂CF₃ | H | Cl | F | CH₂CH₂CH₂CH₂O(3-Cl-5-CF₃-pyridin-2-yl) | O |
| A-0723 | CH₂CF₃ | H | Cl | F | CH₂CH₂CH₂CH₂CH₂O(3-Cl-5-CF₃-pyridin-2-yl) | O |
| A-0724 | CH₂CF₃ | H | Cl | F | CH₂CH₂N(Phth) | O |
| A-0725 | CH₂CF₃ | H | Cl | F | CH₂CH₂CH₂N(Phth) | O |
| A-0726 | CH₂CF₃ | H | Cl | F | CH₂CH₂CH₂CH₂N(Phth) | O |
| A-0727 | CH₂CF₃ | H | Cl | F | CH₂CH₂CH₂CH₂CH₂N(Phth) | O |
| A-0728 | CH₂CF₃ | H | Cl | F | CH₂CH(Me)CH₂N(Phth) | O |
| A-0729 | CH₂CF₃ | H | Cl | F | CH₂[1,3-dioxolan(2-CF₃)-2-yl] | O |
| A-0730 | CH₂CF₃ | H | Cl | F | CH₂(azetidin-3-yl) | O |
| A-0731 | CH₂CF₃ | H | Cl | F | CH₂(pyrrolidin-3-yl) | O |
| A-0732 | CH₂CF₃ | H | Cl | F | CH₂(piperidin-3-yl) | O |
| A-0733 | CH₂CF₃ | H | Cl | F | CH₂{azetidin[1-C(=O)O(*t*-Bu)]-3-yl} | O |
| A-0734 | CH₂CF₃ | H | Cl | F | CH₂{pyrrolidin[1-C(=O)O(*t*-Bu)]-3-yl} | O |
| A-0735 | CH₂CF₃ | H | Cl | F | CH₂{piperidin[1-C(=O)O(*t*-Bu)]-3-yl} | O |
| A-0736 | CH₂CF₃ | H | Cl | F | CH₂{azetidin[1-C(=O)CF₃]-3-yl} | O |
| A-0737 | CH₂CF₃ | H | Cl | F | CH₂{azetidin[1-S(=O)₂CF₃]-3-yl} | O |
| A-0738 | CH₂CF₃ | H | Cl | F | CH₂{pyrrolidin[1-C(=O)CF₃]-3-yl} | O |
| A-0739 | CH₂CF₃ | H | Cl | F | CH₂{pyrrolidin[1-S(=O)₂CF₃]-3-yl} | O |
| A-0740 | CH₂CF₃ | H | Cl | F | CH₂{piperidin[1-C(=O)CF₃]-3-yl} | O |
| A-0741 | CH₂CF₃ | H | Cl | F | CH₂{piperidin[1-S(=O)₂CF₃]-3-yl} | O |
| A-0742 | CH₂CF₃ | H | Cl | F | CH₂CH₂CH₂CN | O |

**[Table 18]**

| Compound Number | R¹ | R² | R³ | R⁴ | R⁵ | A |
|---|---|---|---|---|---|---|
| A-0743 | CH₂CF₃ | H | Cl | F | CH₂CH₂CH₂CH₂CN | O |
| A-0744 | CH₂CF₃ | H | Cl | F | CH₂CH₂CH₂CH₂CH₂CN | O |
| A-0745 | CH₂CF₃ | H | Cl | F | CH₂CH₂CH₂CH₂CH₂CH₂CN | O |
| A-0746 | CH₂CF₃ | H | Cl | F | CH₂CH₂CH₂C(=O)OH | O |
| A-0747 | CH₂CF₃ | H | Cl | F | CH₂CH₂CH₂CH₂C(=O)OH | O |
| A-0748 | CH₂CF₃ | H | Cl | F | CH₂CH₂CH₂CH₂CH₂C(=O)OH | O |
| A-0749 | CH₂CF₃ | H | Cl | F | CH₂CH₂SCN | O |
| A-0750 | CH₂CF₃ | H | Cl | F | CH₂CH₂CH₂SCN | O |
| A-0751 | CH₂CF₃ | H | Cl | F | CH₂CH₂CH₂CH₂SCN | O |
| A-0752 | CH₂CF₃ | H | Cl | F | CH₂CH₂CH₂CH₂CH₂SCN | O |
| A-0753 | CH₂CF₃ | H | Cl | Cl | CH₂CH₂CH₂CH₂CH₂SCN | O |
| A-0754 | CH₂CF₃ | H | Cl | F | CH₂CH₂CH₂CH₂CH₂CH₂SCN | O |
| A-0755 | CH₂CF₃ | H | Cl | F | CH₂CH₂CH₂CH₂CH₂CH₂CH₂SCN | O |
| A-0756 | CH₂CF₃ | H | Cl | F | CH₂CH₂CH₂CH₂CH₂CH₂CH₂CH₂SCN | O |
| A-0757 | CH₂CF₃ | H | Cl | F | CH₂CH₂CH(Me)SCN | O |
| A-0758 | CH₂CF₃ | H | Cl | F | CH₂CH(Me)CH₂SCN | O |
| A-0759 | CH₂CF₃ | H | Cl | F | CH(Me)CH₂CH₂SCN | O |
| A-0760 | CH₂CF₃ | H | Cl | F | CH₂CH₂CH₂CH(Me)SCN | O |
| A-0761 | CH₂CF₃ | H | Cl | F | CH₂CH₂CH(Me)CH₂SCN | O |
| A-0762 | CH₂CF₃ | H | Cl | F | CH₂CH(Me)CH₂CH₂SCN | O |
| A-0763 | CH₂CF₃ | H | Cl | F | CH(Me)CH₂CH₂CH₂SCN | O |
| A-0764 | CH₂CF₃ | H | Cl | F | CH₂CH₂CH₂CH₂CH(Me)SCN | O |
| A-0765 | CH₂CF₃ | H | Cl | F | CH₂CH₂CH₂CH(Me)CH₂SCN | O |
| A-0766 | CH₂CF₃ | H | Cl | F | CH₂CH₂CH(Me)CH₂CH₂SCN | O |
| A-0767 | CH₂CF₃ | H | Cl | F | CH₂CH(Me)CH₂CH₂CH₂SCN | O |
| A-0768 | CH₂CF₃ | H | Cl | F | CH(Me)CH₂CH₂CH₂CH₂SCN | O |
| A-0769 | CH₂CF₃ | H | Cl | F | CH₂CH₂ONH₂ | O |
| A-0770 | CH₂CF₃ | H | Cl | F | CH₂CH₂CH₂ONH₂ | O |
| A-0771 | CH₂CF₃ | H | Cl | F | CH₂CH₂CH₂CH₂ONH₂ | O |
| A-0772 | CH₂CF₃ | H | Cl | F | CH₂CH₂CH₂CH₂CH₂ONH₂ | O |
| A-0773 | CH₂CF₃ | H | Cl | F | CH₂CH₂CH₂CH₂CH₂CH₂ONH₂ | O |
| A-0774 | CH₂CF₃ | H | Me | F | Me | O |
| A-0775 | CH₂CF₃ | H | Me | F | Et | O |
| A-0776 | CH₂CF₃ | H | Me | F | *n* -Pr | O |
| A-0777 | CH₂CF₃ | H | Me | F | *i-*Pr | O |
| A-0778 | CH₂CF₃ | H | Me | F | *n*-Bu | O |
| A-0779 | CH₂CF₃ | H | Me | F | *n*-Pen | O |
| A-0780 | CH₂CF₃ | H | Me | F | *n*-Hex | O |
| A-0781 | CH₂CF₃ | H | Me | F | *n*-Heptyl | O |
| A-0782 | CH₂CF₃ | H | Me | F | *n*-Octyl | O |
| A-0783 | CH₂CF₃ | H | Me | F | *n*-Nonyl | O |
| A-0784 | CH₂CF₃ | H | Me | F | *n*-Decyl | O |
| A-0785 | CH₂CF₃ | H | Me | F | *c*-Pr | O |
| A-0786 | CH₂CF₃ | H | Me | F | *c*-Pen | O |

**[Table 19]**

| Compound Number | R¹ | R² | R³ | R⁴ | R⁵ | A |
|---|---|---|---|---|---|---|
| A-0787 | CH₂CF₃ | H | Me | F | c -Hex | O |
| A-0788 | CH₂CF₃ | H | Me | F | CH₂C(Me)=CH₂ | O |
| A-0789 | CH₂CF₃ | H | Me | F | CH₂CH₂CH=CH₂ | O |
| A-0790 | CH₂CF₃ | H | Me | F | CH₂CH₂CH=C(CH₃)₂ | O |
| A-0791 | CH₂CF₃ | H | Me | F | CH₂CH₂CH₂CH=CH₂ | O |
| A-0792 | CH₂CF₃ | H | Me | F | CH₂CH₂CH₂CH=C(CH₃)₂ | O |
| A-0793 | CH₂CF₃ | H | Me | F | CH₂CH₂CH₂CH₂CH=CH₂ | O |
| A-0794 | CH₂CF₃ | H | Me | F | CH₂CH₂CH₂CH₂CH=C(CH₃)₂ | O |
| A-0795 | CH₂CF₃ | H | Me | F | CH₂CH₂CH=CH | O |
| A-0796 | CH₂CF₃ | H | Me | F | CH₂CH₂CH₂CH=CH | O |
| A-0797 | CH₂CF₃ | H | Me | F | CH₂CH₂CH₂CH₂CH≡CH | O |
| A-0798 | CH₂CF₃ | H | Me | F | CH₂CH₂CH₂Cl | O |
| A-0799 | CH₂CF₃ | H | Me | F | CH₂CH₂CH₂Br | O |
| A-0800 | CH₂CF₃ | H | Me | F | CH₂CH₂CH₂CH₂Cl | O |
| A-0801 | CH₂CF₃ | H | Me | F | CH₂CH₂CH₂CH₂Br | O |
| A-0802 | CH₂CF₃ | H | Me | F | CH₂CH₂CH₂CH₂CH₂Cl | O |
| A-0803 | CH₂CF₃ | H | Me | F | CH₂CH₂CH₂CH₂CH₂Br | O |
| A-0804 | CH₂CF₃ | H | Me | Cl | CH₂CH₂CH₂CH₂CH₂Br | O |
| A-0805 | CH₂CF₃ | H | Me | F | CH₂CH₂CH₂CH₂CH₂CH₂Cl | O |
| A-0806 | CH₂CF₃ | H | Me | F | CH₂CH₂CH₂CH₂CH₂CH₂Br | O |
| A-0807 | CH₂CF₃ | H | Me | F | CH₂CH₂CH₂CH₂CH₂CH₂CH₂Cl | O |
| A-0808 | CH₂CF₃ | H | Me | F | CH₂CH₂CH₂CH₂CH₂CH₂CH₂Br | O |
| A-0809 | CH₂CF₃ | H | Me | F | CH₂CH₂CH₂CH₂CH₂CH₂CH₂CH₂Br | O |
| A-0810 | CH₂CF₃ | H | Me | F | CH₂CH(Me)CH₂Cl | O |
| A-0811 | CH₂CF₃ | H | Me | F | CH₂CH(Me)CH₂Br | O |
| A-0812 | CH₂CF₃ | H | Me | F | CH₂CH₂CH(Me)CH₂CH₂Cl | O |
| A-0813 | CH₂CF₃ | H | Me | F | CH₂CH₂CH(Me)CH₂CH₂Br | O |
| A-0814 | CH₂CF₃ | H | Me | F | CH₂CH₂CF=CF₂ | O |
| A-0815 | CH₂CF₃ | H | Me | F | CH₂CH=C(Cl)CF₃ | O |
| A-0816 | CH₂CF₃ | H | Me | F | CH₂CH₂CH₂CF=CF₂ | O |
| A-0817 | CH₂CF₃ | H | Me | F | CH₂CH₂CH₂CH₂CF=CF₂ | O |
| A-0818 | CH₂CF₃ | H | Me | F | CH₂CH₂CH₂CH₂CH₂CF=CF₂ | O |
| A-0819 | CH₂CF₃ | H | Me | F | CH₂(*t*-Bu) | O |
| A-0820 | CH₂CF₃ | H | Me | F | CH₂CH₂(*t*-Bu) | O |
| A-0821 | CH₂CF₃ | H | Me | F | CH₂CH₂CH₂(*t*-Bu) | O |
| A-0822 | CH₂CF₃ | H | Me | F | CH₂CH₂CH₂CH₂(*t*-Bu) | O |
| A-0823 | CH₂CF₃ | H | Me | F | CH₂CH₂CH₂CH₂CH₂(*t*-Bu) | O |
| A-0824 | CH₂CF₃ | H | Me | F | CH₂CH₂CH₂CH₂CH₂CH₂(*t*-Bu) | O |
| A-0825 | CH₂CF₃ | H | Me | F | CH₂CF₃ | O |
| A-0826 | CH₂CF₃ | H | Me | F | CH₂CH₂CF₃ | O |
| A-0827 | CH₂CF₃ | H | Me | F | CH₂CH₂CH₂CF₃ | O |
| A-0828 | CH₂CF₃ | H | Me | F | CH₂CH₂CH₂CH₂CF₃ | O |
| A-0829 | CH₂CF₃ | H | Me | F | CH₂CH₂CH₂CH₂CH₂CF₃ | O |
| A-0830 | CH₂CF₃ | H | Me | F | CH₂CH₂CH₂CH₂CH₂CH₂CF₃ | O |

**[Table 20]**

| Compound Number | R¹ | R² | R³ | R⁴ | R⁵ | A |
|---|---|---|---|---|---|---|
| A-0831 | CH₂CF₃ | H | Me | F | CH₂CH₂CH₂CH₂CH₂CH₂CH₂CF₃ | O |
| A-0832 | CH₂CF₃ | H | Me | F | CH₂CF₂CF₃ | O |
| A-0833 | CH₂CF₃ | H | Me | F | CF₂CHFCF₃ | O |
| A-0834 | CH₂CF₃ | H | Me | F | CF₂CF₂CF₃ | O |
| A-0835 | CH₂CF₃ | H | Me | F | CH₂CF₂CF₂CF₃ | O |
| A-0836 | CH₂CF₃ | H | Me | F | CH₂CF₂CF₂CF₂CF₃ | O |
| A-0837 | CH₂CF₃ | H | Me | F | CH₂CF₂CF₂CF₂CHF₂ | O |
| A-0838 | CH₂CF₃ | H | Me | F | CH(CF₃)CF₃ | O |
| A-0839 | CH₂CF₃ | H | Me | F | CH₂CF(CF₃)CF₃ | O |
| A-0840 | CH₂CF₃ | H | Me | F | CH₂CF₂CF(CF₃)CF₃ | O |
| A-0841 | CH₂CF₃ | H | Me | F | CH₂CF(CF₃)CF₂CF₃ | O |
| A-0842 | CH₂CF₃ | H | Me | F | CH₂CF₂CF₂CF(CF₃)CF₃ | O |
| A-0843 | CH₂CF₃ | H | Me | F | CH₂CF₂CF(CF₃)CF₂CF₃ | O |
| A-0844 | CH₂CF₃ | H | Me | F | CH₂CF(CF₃)CF₂CF₂CF₃ | O |
| A-0845 | CH₂CF₃ | H | Me | F | CH₂(*c*-Pr) | O |
| A-0846 | CH₂CF₃ | H | Me | F | CH₂[*c*-Pr(1-Me)] | O |
| A-0847 | CH₂CF₃ | H | Me | F | CH₂[*c*-Pr(1-Ph)] | O |
| A-0848 | CH₂CF₃ | H | Me | F | CH₂[*c*-Pr(1-NH₂)] | O |
| A-0849 | CH₂CF₃ | H | Me | F | CH₂[*c*-Pr(1-NHC(=O)O(*t* -Bu)] | O |
| A-0850 | CH₂CF₃ | H | Me | F | CH₂{*c-*Pr[1-NHS(=O)₂CF₃]} | O |
| A-0851 | CH₂CF₃ | H | Me | F | CH₂{*c*-Pr[1-Ph(4-CF₃)]} | O |
| A-0852 | CH₂CF₃ | H | Me | F | CH₂{*c*-Pr[1-Ph(3,4,5-F₃)]} | O |
| A-0853 | CH₂CF₃ | H | Me | F | CH₂{*c*-Hex[4-(*t*-Bu)]} | O |
| A-0854 | CH₂CF₃ | H | Me | F | CH₂[*c*-Hex(4-CF₃)] | O |
| A-0855 | CH₂CF₃ | H | Me | F | CH₂CH₂(*c*-Pr) | O |
| A-0856 | CH₂CF₃ | H | Me | F | CH₂CH₂(*c*-Hex) | O |
| A-0857 | CH₂CF₃ | H | Me | F | CH₂CH₂[*c-*Hex(4-CF₃)] | O |
| A-0858 | CH₂CF₃ | H | Me | F | CH₂CH₂[*c*-Hex(4-SCF₃)] | O |
| A-0859 | CH₂CF₃ | H | Me | F | CH₂CH₂CH₂(*c*-Pr) | O |
| A-0860 | CH₂CF₃ | H | Me | F | CH₂CH₂CH₂(*c*-Hex) | O |
| A-0861 | CH₂CF₃ | H | Me | F | CH₂CH₂CH₂{*c*-Hex[4-(*t* -Bu)]} | O |
| A-0862 | CH₂CF₃ | H | Me | F | CH₂CH₂CH₂[*c*-Hex(4-CF₃)] | O |
| A-0863 | CH₂CF₃ | H | Me | F | CH₂CH₂CH₂[*c*-Hex(4-SCF₃)] | O |
| A-0864 | CH₂CF₃ | H | Me | F | CH₂CH₂CH₂CH₂(*c*-Pr) | O |
| A-0865 | CH₂CF₃ | H | Me | F | CH₂CH₂CH₂CH₂(*c*-Hex) | O |
| A-0866 | CH₂CF₃ | H | Me | F | CH₂CH₂CH₂CH₂[*c* -Hex(4-CF₃)] | O |
| A-0867 | CH₂CF₃ | H | Me | F | CH₂[*c*-Pr(2,2-F₂)] | O |
| A-0868 | CH₂CF₃ | H | Me | F | CH₂[*c*-Hex(4,4-F₂)] | O |
| A-0869 | CH₂CF₃ | H | Me | F | CH₂CH₂[*c*-Pr(2,2-F₂)] | O |
| A-0870 | CH₂CF₃ | H | Me | F | CH₂CH₂[C-Hex(4,4-F₂)] | O |
| A-0871 | CH₂CF₃ | H | Me | F | CH₂CH₂CH₂[*c*-Pr(2,2-F₂)] | O |
| A-0872 | CH₂CF₃ | H | Me | F | CH₂CH₂CH₂CH₂[*c*-Pr(2,2-F₂)] | O |
| A-0873 | CH₂CF₃ | H | Me | F | CH₂CH₂OH | O |
| A-0874 | CH₂CF₃ | H | Me | F | CH₂CH₂CH₂OH | O |
| | | | | | | |

**[Table 21]**

| Compound Number | R¹ | R² | R³ | R⁴ | R5 | A |
|---|---|---|---|---|---|---|
| A-0875 | CH₂CF₃ | H | Me | F | CH₂CH₂CH₂CH₂OH | O |
| A-0876 | CH₂CF₃ | H | Me | F | CH₂CH₂CH₂CH₂CH₂OH | O |
| A-0877 | CH₂CF₃ | H | Me | F | CH₂CH₂CH₂CH₂CH₂CH₂OH | O |
| A-0878 | CH₂CF₃ | H | Me | F | CH₂C(=O)Me | O |
| A-0879 | CH₂CF₃ | H | Me | F | CH₂CH(OH)Me | O |
| A-0880 | CH₂CF₃ | H | Me | F | CH₂CH(OH)CF₃ | O |
| A-0881 | CH₂CF₃ | H | Me | F | CH₂C(OH)₂CF₃ | O |
| A-0882 | CH₂CF₃ | H | Me | F | CH₂C(CF₃)=NOH | O |
| A-0883 | CH₂CF₃ | H | Me | F | CH₂C(CF₃)=NOMe | O |
| A-0884 | CH₂CF₃ | H | Me | F | CH₂CH(CF₃)NH₂ | O |
| A-0885 | CH₂CF₃ | H | Me | F | CH₂CH₂OMe | O |
| A-0886 | CH₂CF₃ | H | Me | F | CH₂CH₂CH₂OMe | O |
| A-0887 | CH₂CF₃ | H | Me | F | CH₂CH₂CH₂CH₂OMe | O |
| A-0888 | CH₂CF₃ | H | Me | F | CH₂CH₂CH₂CH₂CH₂OMe | O |
| A-0889 | CH₂CF₃ | H | Me | F | CH₂CH₂CH₂CH₂CH₂CH₂OMe | O |
| A-0890 | CH₂CF₃ | H | Me | F | CH₂CH₂OCHF₂ | O |
| A-0891 | CH₂CF₃ | H | Me | F | CH₂CH₂CH₂OCHF₂ | O |
| A-0892 | CH₂CF₃ | H | Me | F | CH₂CH₂CH₂CH₂OCHF₂ | O |
| A-0893 | CH₂CF₃ | H | Me | F | CH₂CH₂CH₂CH₂CH₂OCHF₂ | O |
| A-0894 | CH₂CF₃ | H | Me | F | CH₂CH₂CH₂CH₂CH₂CH₂OCHF₂ | O |
| A-0895 | CH₂CF₃ | H | Me | F | CH₂CH₂OCH₂CF₃ | O |
| A-0896 | CH₂CF₃ | H | Me | F | CH₂CH₂CH₂OCH₂CF₃ | O |
| A-0897 | CH₂CF₃ | H | Me | F | CH₂CH₂CH₂CH₂OCH₂CF₃ | O |
| A-0898 | CH₂CF₃ | H | Me | F | CH₂CH₂CH₂CH₂CH₂OCH₂CF₃ | O |
| A-0899 | CH₂CF₃ | H | Me | F | CH₂CH₂CH₂CH₂CH₂CH₂OCH₂CF₃ | O |
| A-0900 | CH₂CF₃ | H | Me | F | CH₂CH₂CH₂CH₂CH₂CH₂CH₂OCH₂CF₃ | O |
| A-0901 | CH₂CF₃ | H | Me | F | CH₂CH₂OC(CF₃)₃ | O |
| A-0902 | CH₂CF₃ | H | Me | F | CH₂CH₂CH₂OC(CF₃)₃ | O |
| A-0903 | CH₂CF₃ | H | Me | F | CH₂CH₂CH₂CH₂OC(CF₃)₃ | O |
| A-0904 | CH₂CF₃ | H | Me | F | CH₂CH₂CH₂CH₂CH₂OC(CF₃)₃ | O |
| A-0905 | CH₂CF₃ | H | Me | F | CF₂CHFOCF₂CF₂CF₃ | O |
| A-0906 | CH₂CF₃ | H | Me | F | CH₂CH₂O(*c*-Pr) | O |
| A-0907 | CH₂CF₃ | H | Me | F | CH₂CH₂O(*c*-Pen) | O |
| A-0908 | CH₂CF₃ | H | Me | F | CH₂CH₂O(*c*-Hex) | O |
| A-0909 | CH₂CF₃ | H | Me | F | CH₂CH₂CH₂O(*c*-Pr) | O |
| A-0910 | CH₂CF₃ | H | Me | F | CH₂CH₂CH₂O(*c*-Pen) | O |
| A-0911 | CH₂CF₃ | H | Me | F | CH₂CH₂CH₂O(*c*-Hex) | O |
| A-0912 | CH₂CF₃ | H | Me | F | CH₂CH₂CH₂CH₂O(*c*-Pr) | O |
| A-0913 | CH₂CF₃ | H | Me | F | CH₂CH₂CH₂CH₂O(*c*-Pen) | O |
| A-0914 | CH₂CF₃ | H | Me | F | CH₂CH₂CH₂CH₂O(*c*-Hex) | O |
| A-0915 | CH₂CF₃ | H | Me | F | CH₂CH₂O[*c*-Pr(2,2-F₂)] | O |
| A-0916 | CH₂CF₃ | H | Me | F | CH₂CH₂CH₂O[*c*-Pr(2,2-F₂)] | O |
| A-0917 | CH₂CF₃ | H | Me | F | CH₂CH₂CH₂CH₂O[*c*-Pr(2,2-F₂)] | O |
| A-0918 | CH₂CF₃ | H | Me | F | CH₂CH₂OCH₂CH₂OCH₃ | O |

**[Table 22]**

| Compound Number | R¹ | R² | R³ | R⁴ | R5 | A |
|---|---|---|---|---|---|---|
| A-0919 | CH₂CF₃ | H | Me | F | CH₂CH₂OCH₂CH₂OCH₂CF₃ | O |
| A-0920 | CH₂CF₃ | H | Me | F | CH₂CF₂OCF₂CF₂OCF₃ | O |
| A-0921 | CH₂CF₃ | H | Me | F | CF₂CHFOCF₂CF₂OCF₃ | O |
| A-0922 | CH₂CF₃ | H | Me | F | CF₂CHFOCF₂CF(CF₃)OCF₂CF₂CF₃ | O |
| A-0923 | CH₂CF₃ | H | Me | F | CH₂CH₂SH | O |
| A-0924 | CH₂CF₃ | H | Me | F | CH₂CH₂CH₂SH | O |
| A-0925 | CH₂CF₃ | H | Me | F | CH₂CH₂CH₂CH₂SH | O |
| A-0926 | CH₂CF₃ | H | Me | F | CH₂CH₂CH₂CH₂CH₂SH | O |
| A-0927 | CH₂CF₃ | H | Me | F | CH₂CH₂CH₂CH₂CH₂CH₂SH | O |
| A-0928 | CH₂CF₃ | H | Me | F | CH₂CH(SH)Me | O |
| A-0929 | CH₂CF₃ | H | Me | F | CH₂CH[SC(=O)Me]Me | O |
| A-0930 | CH₂CF₃ | H | Me | F | CH₂CH₂S(*t*-Bu) | O |
| A-0931 | CH₂CF₃ | H | Me | F | CH₂CH₂CH₂S(*t* -Bu) | O |
| A-0932 | CH₂CF₃ | H | Me | F | CH₂CH₂CH₂CH₂S(*t*-Bu) | O |
| A-0933 | CH₂CF₃ | H | Me | F | CH₂CH₂CH₂CH₂CH₂SCH₃ | O |
| A-0934 | CH₂CF₃ | H | Me | F | CH₂CH₂CH₂CH₂CH₂SCH(CH₃)₂ | O |
| A-0935 | CH₂CF₃ | H | Me | F | CH₂CH₂CH₂CH₂CH₂S(*t*-Bu) | O |
| A-0936 | CH₂CF₃ | H | Me | F | CH₂CH₂CH₂CH₂CH₂CH₂SCH(CH₃)₂ | O |
| A-0937 | CH₂CF₃ | H | Me | F | CH₂CH₂CH₂CH₂CH₂CH₂S(*t*-Bu) | O |
| A-0938 | CH₂CF₃ | H | Me | F | CH₂CH₂CH₂CH₂CH₂CH₂CH₂S(*t* -Bu) | O |
| A-0939 | CH₂CF₃ | H | Me | F | CH₂CH₂SCHF₂ | O |
| A-0940 | CH₂CF₃ | H | Me | F | CH₂CH₂CH₂SCHF₂ | O |
| A-0941 | CH₂CF₃ | H | Me | F | CH₂CH₂CH₂CH₂SCHF₂ | O |
| A-0942 | CH₂CF₃ | H | Me | H | CH₂CH₂CH₂CH₂CH₂SCHF₂ | O |
| A-0943 | CH₂CF₃ | H | Me | F | CH₂CH₂CH₂CH₂CH₂SCHF₂ | O |
| A-0944 | CH₂CF₃ | H | Me | F | CH₂CH₂CH₂CH₂CH₂CH₂SCHF₂ | O |
| A-0945 | CH₂CF₃ | H | Me | F | CH₂CH₂CH₂CH₂CH₂CH₂CH₂SCHF₂ | O |
| A-0946 | CH₂CF₃ | H | Me | F | CH₂CH₂SCH₂CF₃ | O |
| A-0947 | CH₂CF₃ | H | Me | F | CH₂CH₂CH₂SCH₂CF₃ | O |
| A-0948 | CH₂CF₃ | H | Me | F | CH₂CH₂CH₂CH₂SCH₂CF₃ | O |
| A-0949 | CH₂CF₃ | H | Me | F | CH₂CH₂CH₂CH₂CH₂SCH₂CF₃ | O |
| A-0950 | CH₂CF₃ | H | Me | F | CH₂CH₂CH₂CH₂CH₂CH₂SCH₂CF₃ | O |
| A-0951 | CH₂CF₃ | H | Me | F | CH₂CH₂CH₂CH₂CH₂CH₂CH₂SCH₂CF₃ | O |
| A-0952 | CH₂CF₃ | H | Me | F | CH₂CH(Me)SCH₂CF₃ | O |
| A-0953 | CH₂CF₃ | H | Me | F | CH₂CH₂SCH(CF₃)₂ | O |
| A-0954 | CH₂CF₃ | H | Me | F | CH₂CH₂CH₂SCH(CF₃)₂ | O |
| A-0955 | CH₂CF₃ | H | Me | F | CH₂CH₂CH₂CH₂SCH(CF₃)₂ | O |
| A-0956 | CH₂CF₃ | H | Me | F | CH₂CH₂CH₂CH₂CH₂SCH(CF₃)₂ | O |
| A-0957 | CH₂CF₃ | H | Me | F | CH₂CH₂CH₂CH₂CH₂CH₂SCH(CF₃)₂ | O |
| A-0958 | CH₂CF₃ | H | Me | F | CH₂CH₂CH₂CH₂CH₂SCF₂CF₃ | O |
| A-0959 | CH₂CF₃ | H | Me | F | CH₂CH₂CH₂CH₂CH₂CH₂SCF₂CF₃ | O |
| A-0960 | CH₂CF₃ | H | Me | F | CH₂CH₂CH₂CH₂CH₂CH₂SCF(CF₃)₂ | O |
| A-0961 | CH₂CF₃ | H | Me | F | CH₂CH₂CH₂CH₂CH₂CH₂CH₂SCF(CF₃)₂ | O |
| A-0962 | CH₂CF₃ | H | Me | F | CH₂CH₂CH₂CH₂CH₂SCCl₃ | O |

**[Table 23]**

| Compound Number | R¹ | R² | R³ | R⁴ | R5 | A |
|---|---|---|---|---|---|---|
| A-0963 | CH₂CF₃ | H | Me | F | CH₂CH₂CH₂CH₂CH₂CH₂SCCl₃ | O |
| A-0964 | CH₂CF₃ | H | Me | F | CH₂CH₂SCH₂CH=CH₂ | O |
| A-0965 | CH₂CF₃ | H | Me | F | CH₂CH₂CH₂SCH₂CH=CH₂ | O |
| A-0966 | CH₂CF₃ | H | Me | F | CH₂CH₂CH₂CH₂SCH₂CH=CH₂ | O |
| A-0967 | CH₂CF₃ | H | Me | F | CH₂CH₂SCF=CFCF₃ | O |
| A-0968 | CH₂CF₃ | H | Me | F | CH₂CH₂CH₂SCF=CFCF₃ | O |
| A-0969 | CH₂CF₃ | H | Me | F | CH₂CH₂CH₂CH₂SCF=CFCF₃ | O |
| A-0970 | CH₂CF₃ | H | Me | F | CH₂CH₂CH₂S(*c*-Pr) | O |
| A-0971 | CH₂CF₃ | H | Me | F | CH₂CH₂CH₂S(*c*-Hex) | O |
| A-0972 | CH₂CF₃ | H | Me | F | CH₂CH₂CH₂CH₂S(*c*-Pr) | O |
| A-0973 | CH₂CF₃ | H | Me | F | CH₂CH₂CH₂CH₂S(*c*-Hex) | O |
| A-0974 | CH₂CF₃ | H | Me | F | CH₂CH₂CH₂CH₂CH₂S(*c*-Pr) | O |
| A-0975 | CH₂CF₃ | H | Me | F | CH₂CH₂CH₂CH₂CH₂S(*c*-Hex) | O |
| A-0976 | CH₂CF₃ | H | Me | F | CH₂CH₂CH₂S[*c*-Hex(4,4-F₂)] | O |
| A-0977 | CH₂CF₃ | H | Me | F | CH₂CH₂CH₂CH₂S[*c*-Hex(4,4-F₂)] | O |
| A-0978 | CH₂CF₃ | H | Me | F | CH₂CH₂CH₂CH₂CH₂S[*c*-Hex(4,4-F₂)] | O |
| A-0979 | CH₂CF₃ | H | Me | F | CH₂CH₂CH₂SCH₂(*c*-Pr) | O |
| A-0980 | CH₂CF₃ | H | Me | F | CH₂CH₂CH₂SCH₂(*c*-Hex) | O |
| A-0981 | CH₂CF₃ | H | Me | F | CH₂CH₂CH₂CH₂SCH₂(*c*-Pr) | O |
| A-0982 | CH₂CF₃ | H | Me | F | CH₂CH₂CH₂CH₂SCH₂(*c*-Hex) | O |
| A-0983 | CH₂CF₃ | H | Me | F | CH₂CH₂CH₂CH₂CH₂SCH₂(*c*-Pr) | O |
| A-0984 | CH₂CF₃ | H | Me | F | CH₂CH₂CH₂CH₂CH₂SCH₂(*c*-Hex) | O |
| A-0985 | CH₂CF₃ | H | Me | F | CH₂CH₂CH₂SCH₂[*c*-Hex(4,4-F₂)] | O |
| A-0986 | CH₂CF₃ | H | Me | F | CH₂CH₂CH₂CH₂SCH₂[*c*-Hex(4,4-F₂)] | O |
| A-0987 | CH₂CF₃ | H | Me | F | CH₂CH₂CH₂CH₂CH₂SCH₂[*c*-Hex(4,4-F₂)] | O |
| A-0988 | CH₂CF₃ | H | Me | F | CH₂CH₂CH₂SCH₂SiMe₃ | O |
| A-0989 | CH₂CF₃ | H | Me | F | CH₂CH₂CH₂CH₂SCH₂SiMe₃ | O |
| A-0990 | CH₂CF₃ | H | Me | F | CH₂CH₂CH₂CH₂CH₂SCH₂SiMe₃ | O |
| A-0991 | CH₂CF₃ | H | Me | F | CH₂CH₂OCH₂CH₂SCH₃ | O |
| A-0992 | CH₂CF₃ | H | Me | F | CH₂CH₂OCH₂CH₂SCHF₂ | O |
| A-0993 | CH₂CF₃ | H | Me | F | CH₂CH₂OCH₂CH₂SCF₃ | O |
| A-0994 | CH₂CF₃ | H | Me | F | CH₂CH₂OCH₂CH₂SCH₂CF₃ | O |
| A-0995 | CH₂CF₃ | H | Me | F | CH₂CH₂CH₂S(=O)(*t*-Bu) | O |
| A-0996 | CH₂CF₃ | H | Me | F | CH₂CH₂CH₂CH₂S(=O)(*t*-Bu) | O |
| A-0997 | CH₂CF₃ | H | Me | F | CH₂CH₂CH₂CH₂CH₂CH₂S(=O)(*t* Bu) | O |
| A-0998 | CH₂CF₃ | H | Me | F | CH₂CH₂S(=O)CHF₂ | O |
| A-0999 | CH₂CF₃ | H | Me | F | CH₂CH₂CH₂S(=O)CHF₂ | O |
| A-1000 | CH₂CF₃ | H | Me | F | CH₂CH₂CH₂CH₂S(=O)CHF₂ | O |
| A-1001 | CH₂CF₃ | H | Me | F | CH₂CH₂CH₂CH₂CH₂S(=O)CHF₂ | O |
| A-1002 | CH₂CF₃ | H | Me | F | CH₂CH₂CH₂CH₂CH₂CH₂S(=O)CHF₂ | O |
| A-1003 | CH₂CF₃ | H | Me | F | CH₂CH₂CH₂CH₂CH₂CH₂CH₂S(=O)CHF₂ | O |
| A-1004 | CH₂CF₃ | H | Me | F | CH₂CH₂S(=O)CF₃ | O |
| A-1005 | CH₂CF₃ | H | Me | F | CH₂CH₂CH₂S(=O)CF₃ | O |
| A-1006 | CH₂CF₃ | H | Me | F | CH₂CH₂CH₂CH₂S(=O)CF₃ | O |

**[Table 24]**

| Compound Number | R¹ | R² | R³ | R⁴ | R⁵ | A |
|---|---|---|---|---|---|---|
| A-1007 | CH₂CF₃ | H | Me | F | CH₂CH₂CH₂CH₂CH₂S(=O)CF₃ | O |
| A-1008 | CH₂CF₃ | H | Me | F | CH₂CH₂CH₂CH₂CH₂CH₂S(=O)CF₃ | O |
| A-1009 | CH₂CF₃ | H | Me | F | CH₂CH₂CH₂CH₂CH₂CH₂CH₂S(=O)CF₃ | O |
| A-1010 | CH₂CF₃ | H | Me | F | CH₂CH₂S(=O)CH₂CF₃ | O |
| A-1011 | CH₂CF₃ | H | Me | F | CH₂CH₂CH₂S(=O)CH₂CF₃ | O |
| A-1012 | CH₂CF₃ | H | Me | F | CH₂CH₂CH₂CH₂S(=O)CH₂CF₃ | O |
| A-1013 | CH₂CF₃ | H | Me | F | CH₂CH₂CH₂CH₂CH₂S(=O)CH₂CF₃ | O |
| A-1014 | CH₂CF₃ | H | Me | F | CH₂CH₂CH₂CH₂CH₂CH₂S(=O)CH₂CF₃ | O |
| A-1015 | CH₂CF₃ | H | Me | F | CH₂CH₂CH₂CH₂CH₂CH₂CH₂S(=O)CH₂CF₃ | O |
| A-1016 | CH₂CF₃ | H | Me | F | CH₂CH(Me)S(=O)CH₂CF₃ | O |
| A-1017 | CH₂CF₃ | H | Me | F | CH₂CH₂CH₂S(=O)(*c* -Pr) | O |
| A-1018 | CH₂CF₃ | H | Me | F | CH₂CH₂CH₂CH₂S(=O)(*c* -Pr) | O |
| A-1019 | CH₂CF₃ | H | Me | F | CH₂CH₂CH₂CH₂S(=O)(*c* -Pen) | O |
| A-1020 | CH₂CF₃ | H | Me | F | CH₂CH₂CH₂CH₂S(=O)(*c*-Hex) | O |
| A-1021 | CH₂CF₃ | H | Me | F | CH₂CH₂CH₂CH₂CH₂S(=O)(*c* -Pr) | O |
| A-1022 | CH₂CF₃ | H | Me | F | CH₂CH₂CH₂CH₂CH₂S(=O)(*c* - Hex) | O |
| A-1023 | CH₂CF₃ | H | Me | F | CH₂CH₂CH₂S(=O)[*c* -Hex(4,4-F₂)] | O |
| A-1024 | CH₂CF₃ | H | Me | F | CH₂CH₂CH₂CH₂S(=O)[*c*-Hex(4,4-F₂)] | O |
| A-1025 | CH₂CF₃ | H | Me | F | CH₂CH₂CH₂CH₂CH₂S(=O)[*c -*Hex(4,4-F₂)] | O |
| A-1026 | CH₂CF₃ | H | Me | F | CH₂CH₂CH₂CH₂S(=O)₂Me | O |
| A-1027 | CH₂CF₃ | H | Me | F | CH₂CH₂CH₂CH₂CH₂S(=O)₂Me | O |
| A-1028 | CH₂CF₃ | H | Me | F | CH₂CH₂CH₂CH₂CH₂S(=O)₂(*t* -Bu) | O |
| A-1029 | CH₂CF₃ | H | Me | F | CH₂CH₂S(=O)₂CHF₂ | O |
| A-1030 | CH₂CF₃ | H | Me | F | CH₂CH₂CH₂S(=O)₂CHF₂ | O |
| A-1031 | CH₂CF₃ | H | Me | F | CH₂CH₂CH₂CH₂S(=O)₂CHF₂ | O |
| A-1032 | CH₂CF₃ | H | Me | F | CH₂CH₂CH₂CH₂CH₂S(=O)₂CHF₂ | O |
| A-1033 | CH₂CF₃ | H | Me | F | CH₂CH₂CH₂CH₂CH₂CH₂S(=O)₂CHF₂ | O |
| A-1034 | CH₂CF₃ | H | Me | F | CH₂CH₂S(=O)₂CF₃ | O |
| A-1035 | CH₂CF₃ | H | Me | F | CH₂CH₂CH₂S(=O)₂CF₃ | O |
| A-1036 | CH₂CF₃ | H | Me | F | CH₂CH₂C H₂C H₂S(=O)₂CF₃ | O |
| A-1037 | CH₂CF₃ | H | Me | F | CH₂CH₂CH₂CH₂CH₂S(=O)₂CF₃ | O |
| A-1038 | CH₂CF₃ | H | Me | F | CH₂CH₂CH₂CH₂CH₂CH₂S(=O)₂CF₃ | O |
| A-1039 | CH₂CF₃ | H | Me | F | CH₂CH₂CH₂CH₂CH₂CH₂CH₂S(=O)₂CF₃ | O |
| A-1040 | CH₂CF₃ | H | Me | F | CH₂CH₂CH₂CH₂CH₂CH₂CH₂CH₂S(=O)₂CF₃ | O |
| A-1041 | CH₂CF₃ | H | Me | F | C H₂C H₂S(=O)₂CH₂CF₃ | O |
| A-1042 | CH₂CF₃ | H | Me | F | CH₂CH₂CH₂S(=O)₂CH₂CF₃ | O |
| A-1043 | CH₂CF₃ | H | Me | F | CH₂CH₂CH₂CH₂S(=O)₂CH₂CF₃ | O |
| A-1044 | CH₂CF₃ | H | Me | F | CH₂CH₂CH₂CH₂CH₂S(=O)₂CH₂CF₃ | O |
| A-1045 | CH₂CF₃ | H | Me | F | CH₂CH₂CH₂CH₂CH₂CH₂S(=O)₂CH₂CF₃ | O |
| A-1046 | CH₂CF₃ | H | Me | F | CH₂CH(Me)S(=O)₂CH₂CF₃ | O |
| A-1047 | CH₂CF₃ | H | Me | F | CH₂CH₂CH₂S(=O)₂(*c* -Pr) | O |
| A-1048 | CH₂CF₃ | H | Me | F | CH₂CH₂CH₂CH₂S(=O)₂(*c* -Pr) | O |
| A-1049 | CH₂CF₃ | H | Me | F | CH₂CH₂CH₂CH₂CH₂S(=O)₂(*c* -Pr) | O |
| A-1050 | CH₂CF₃ | H | Me | F | CH₂CH₂CH₂S(=O)₂[*c* -Hex(4,4-F₂)] | O |

**[Table 25]**

| Compound Number | R¹ | R² | R³ | R⁴ | R⁵ | A |
|---|---|---|---|---|---|---|
| A-1051 | CH₂CF₃ | H | Me | F | CH₂CH₂CH₂CH₂S(=O)₂[*c* -Hex(4,4-F₂)] | O |
| A-1052 | CH₂CF₃ | H | Me | F | CH₂CH₂CH₂CH₂CH₂S(=O)₂[*c* -Hex(4,4-F₂)] | O |
| A-1053 | CH₂CF₃ | H | Me | F | CH₂CH₂CH₂C(=O)H | O |
| A-1054 | CH₂CF₃ | H | Me | F | CH₂CH₂CH₂CH₂C(=O)H | O |
| A-1055 | CH₂CF₃ | H | Me | F | CH₂CH₂CH₂CH₂CH₂C(=O)H | O |
| A-1056 | CH₂CF₃ | H | Me | F | CH₂CH₂CH₂C(=O)(*t* -Bu) | O |
| A-1057 | CH₂CF₃ | H | Me | F | CH₂CH₂CH₂CH₂C(=O)(*t* -Bu) | O |
| A-1058 | CH₂CF₃ | H | Me | F | CH₂CH₂CH₂CH₂CH₂C(=O)(*t* -Bu) | O |
| A-1059 | CH₂CF₃ | H | Me | F | CH₂CH₂CH₂C(=O)CF₃ | O |
| A-1060 | CH₂CF₃ | H | Me | F | CH₂CH₂CH₂CH₂C(=O)CF₃ | O |
| A-1061 | CH₂CF₃ | H | Me | F | CH₂CH₂CH₂CH₂CH₂C(=O)CF₃ | O |
| A-1062 | CH₂CF₃ | H | Me | F | CH₂C(=O)Ph | O |
| A-1063 | CH₂CF₃ | H | Me | F | CH₂C(=O)Ph(4-Cl) | O |
| A-1064 | CH₂CF₃ | H | Me | F | CH₂C(=O)Ph(4-CF₃) | O |
| A-1065 | CH₂CF₃ | H | Me | F | CH₂CH₂C(=O)Ph(4-CF₃) | O |
| A-1066 | CH₂CF₃ | H | Me | F | CH₂CH₂CH₂C(=O)Ph(4-CF₃) | O |
| A-1067 | CH₂CF₃ | H | Me | F | CH₂CH₂CH₂CH₂C(=O)Ph(4-CF₃) | O |
| A-1068 | CH₂CF₃ | H | Me | F | H₂CH₂CH₂CH₂CH₂C(=O)Ph(4-CF₃) | O |
| A-1069 | CH₂CF₃ | H | Me | F | CH₂C(=O)OEt | O |
| A-1070 | CH₂CF₃ | H | Me | F | CH₂CH₂C(=O)O(*t* -Bu) | O |
| A-1071 | CH₂CF₃ | H | Me | F | CH₂CH₂CH₂C(=O)OEt | O |
| A-1072 | CH₂CF₃ | H | Me | F | CH₂CH₂CH₂C(=O)O(*t* -Bu) | O |
| A-1073 | CH₂CF₃ | H | Me | F | CH₂CH₂CH₂CH₂C(=O)O(*t* -Bu) | O |
| A-1074 | CH₂CF₃ | H | Me | F | CH₂CH₂CH₂CH₂CH₂C(=O)O(*t* -Bu) | O |
| A-1075 | CH₂CF₃ | H | Me | F | CH₂CH₂CH₂CH₂CH₂CH₂C(=O)O(*t* -Bu) | O |
| A-1076 | CH₂CF₃ | H | Me | F | CH₂CH₂CH₂CH₂CH₂CH₂CH₂C(=O)OEt | O |
| A-1077 | CH₂CF₃ | H | Me | F | CH₂CH₂C(=O)OCH₂CF₃ | O |
| A-1078 | CH₂CF₃ | H | Me | F | CH₂CH₂CH₂C(=O)OCH₂CF₃ | O |
| A-1079 | CH₂CF₃ | H | Me | F | CH₂CH₂CH₂CH₂C(=O)OCH₂CF₃ | O |
| A-1080 | CH₂CF₃ | H | Me | F | CH₂CH₂CH₂CH₂CH₂C(=O)OCH₂CF₃ | O |
| A-1081 | CH₂CF₃ | H | Me | F | CH₂CH₂C(=O)NH(*t* -Bu) | O |
| A-1082 | CH₂CF₃ | H | Me | F | CH₂CH₂CH₂C(=O)NH(*t* -Bu) | O |
| A-1083 | CH₂CF₃ | H | Me | F | CH₂CH₂CH₂C(=O)NH(*t* -Pen) | O |
| A-1084 | CH₂CF₃ | H | Me | F | CH₂CH₂CH₂CH₂C(=O)NH(*t* -Bu) | O |
| A-1085 | CH₂CF₃ | H | Me | F | CH₂CH₂C(=O)NHCH₂CF₃ | O |
| A-1086 | CH₂CF₃ | H | Me | F | CH₂CH₂CH₂C(=O)NHCH₂CF₃ | O |
| A-1087 | CH₂CF₃ | H | Me | F | CH₂CH₂CH₂CH₂C(=O)NHCH₂CF₃ | O |
| A-1088 | CH₂CF₃ | H | Me | F | CH₂CH₂SC(=O)N(Me)₂ | O |
| A-1089 | CH₂CF₃ | H | Me | F | CH₂CH₂CH₂SC(=O)N(Me)₂ | O |
| A-1090 | CH₂CF₃ | H | Me | F | CH₂CH₂CH₂SC(=O)NH(*t* -Bu) | O |
| A-1091 | CH₂CF₃ | H | Me | F | CH₂CH₂CH₂CH₂CH₂SC(=O)NH(*t* -Bu) | O |
| A-1092 | CH₂CF₃ | H | Me | F | CH₂CH₂SC(=O)NHCH₂CF₃ | O |
| A-1093 | CH₂CF₃ | H | Me | F | CH₂CH₂CH₂SC(=O)NHCH₂CF₃ | O |
| A-1094 | CH₂CF₃ | H | Me | F | CH₂CH₂CH₂CH₂SC(=O)NHCH₂CF₃ | O |

**[Table 26]**

| Compound Number | R¹ | R² | R³ | R⁴ | R⁵ | A |
|---|---|---|---|---|---|---|
| A-1095 | CH₂CF₃ | H | Me | F | CH₂CH₂CH₂OC(=O)H | O |
| A-1096 | CH₂CF₃ | H | Me | F | CH₂CH₂CH₂CH₂OC(=O)H | O |
| A-1097 | CH₂CF₃ | H | Me | F | CH₂CH₂CH₂CH₂CH₂OC(=O)H | O |
| A-1098 | CH₂CF₃ | H | Me | F | CH₂CH₂CH₂OC(=O)Me | O |
| A-1099 | CH₂CF₃ | H | Me | F | CH₂CH₂CH₂CH₂OC(=O)Me | O |
| A-1100 | CH₂CF₃ | H | Me | F | CH₂CH₂CH₂CH₂CH₂OC(=O)Me | O |
| A-1101 | CH₂CF₃ | H | Me | F | CH₂CH₂OC(=O)CF₃ | O |
| A-1102 | CH₂CF₃ | H | Me | F | CH₂CH₂CH₂OC(=O)CF₃ | O |
| A-1103 | CH₂CF₃ | H | Me | F | CH₂CH₂CH₂CH₂OC(=O)CF₃ | O |
| A-1104 | CH₂CF₃ | H | Me | F | CH₂CH₂CH₂CH₂CH₂OC(=O)Ph(4-CF₃) | O |
| A-1105 | CH₂CF₃ | H | Me | F | CH₂CH₂OC(=O)Ph | O |
| A-1106 | CH₂CF₃ | H | Me | F | CH₂CH₂OC(=O)Ph(4-CF₃) | O |
| A-1107 | CH₂CF₃ | H | Me | F | CH₂CH₂CH₂OC(=O)Ph(4-CF₃) | O |
| A-1108 | CH₂CF₃ | H | Me | F | CH₂CH₂CH₂CH₂OC(=O)Ph(4-CF₃) | O |
| A-1109 | CH₂CF₃ | H | Me | F | CH₂CH₂CH₂CH₂CH₂OC(=O)Ph(4-CF₃) | O |
| A-1110 | CH₂CF₃ | H | Me | F | CH₂CH₂OS(=O)Me | O |
| A-1111 | CH₂CF₃ | H | Me | F | CH₂CH₂CH₂OS(=O)Me | O |
| A-1112 | CH₂CF₃ | H | Me | F | CH₂CH₂CH₂CH₂OS(=O)Me | O |
| A-1113 | CH₂CF₃ | H | Me | F | CH₂CH₂CH₂CH₂CH₂OS(=O)Me | O |
| A-1114 | CH₂CF₃ | H | Me | F | CH₂CH₂CH₂CH₂CH₂CH₂OS(=O)Me | O |
| A-1115 | CH₂CF₃ | H | Me | F | CH₂CH₂OS(=O)CF₃ | O |
| A-1116 | CH₂CF₃ | H | Me | F | CH₂CH₂CH₂OS(=O)CF₃ | O |
| A-1117 | CH₂CF₃ | H | Me | F | CH₂CH₂CH₂CH₂OS(=O)CF₃ | O |
| A-1118 | CH₂CF₃ | H | Me | F | CH₂CH₂CH₂CH₂CH₂OS(=O)CF₃ | O |
| A-1119 | CH₂CF₃ | H | Me | F | CH₂CH₂CH₂CH₂CH₂CH₂OS(=O)CF₃ | O |
| A-1120 | CH₂CF₃ | H | Me | F | CH₂CH₂OS(=O)Ph | O |
| A-1121 | CH₂CF₃ | H | Me | F | CH₂CH₂OS(=O)Ph(4-Me) | O |
| A-1122 | CH₂CF₃ | H | Me | F | CH₂CH₂CH₂OS(=O)Ph | O |
| A-1123 | CH₂CF₃ | H | Me | F | CH₂CH₂CH₂OS(=O)Ph(4-Me) | O |
| A-1124 | CH₂CF₃ | H | Me | F | CH₂CH₂OS(=O)₂Me | O |
| A-1125 | CH₂CF₃ | H | Me | F | CH₂CH₂CH₂OS(=O)₂Me | O |
| A-1126 | CH₂CF₃ | H | Me | F | CH₂CH₂CH₂OS(=O)₂CF₃ | O |
| A-1127 | CH₂CF₃ | H | Me | F | CH₂CH₂CH₂CH₂OS(=O)₂CF₃ | O |
| A-1128 | CH₂CF₃ | H | Me | F | CH₂CH₂CH₂CH₂CH₂OS(=O)₂CF₃ | O |
| A-1129 | CH₂CF₃ | H | Me | F | CH₂CH₂CH₂CH₂CH₂OS(=O)₂CF₂CF₂CF₂CF₃ | O |
| A-1130 | CH₂CF₃ | H | Me | F | CH₂CH₂OS(=O)₂Ph | O |
| A-1131 | CH₂CF₃ | H | Me | F | CH₂CH₂OS(=O)₂Ph(4-Me) | O |
| A-1132 | CH₂CF₃ | H | Me | F | CH₂CH₂CH₂OS(=O)₂Ph | O |
| A-1133 | CH₂CF₃ | H | Me | F | CH₂CH₂CH₂OS(=O)₂Ph(4-Me) | O |
| A-1134 | CH₂CF₃ | H | Me | F | CH₂CH₂NH₂ | O |
| A-1135 | CH₂CF₃ | H | Me | F | CH₂CH₂CH₂NH₂ | O |
| A-1136 | CH₂CF₃ | H | Me | F | CH₂CH₂CH₂CH₂NH₂ | O |
| A-1137 | CH₂CF₃ | H | Me | F | CH₂CH₂CH₂CH₂CH₂NH₂ | O |
| A-1138 | CH₂CF₃ | H | Me | F | CH₂CH₂CH₂CH₂CH₂CH₂NH₂ | O |

**[Table 27]**

| Compound Number | R¹ | R² | R³ | R⁴ | R⁵ | A |
|---|---|---|---|---|---|---|
| A-1139 | CH₂CF₃ | H | Me | F | CH₂CH₂CH₂CH₂CH₂CH₂CH₂NH₂ | O |
| A-1140 | CH₂CF₃ | H | Me | F | CH₂CH(Me)NH₂ | O |
| A-1141 | CH₂CF₃ | H | Me | F | CH₂C*H(Me)NH₂ : (R) | O |
| A-1142 | CH₂CF₃ | H | Me | F | CH₂C*H(Me)NH₂ : (S) | O |
| A-1143 | CH₂CF₃ | H | Me | F | CH₂CH(Et)NH₂ | O |
| A-1144 | CH₂CF₃ | H | Me | F | CH₂CH(*i* -Pr)NH₂ | O |
| A-1145 | CH₂CF₃ | H | Me | F | CH(Me)CH₂NH₂ | O |
| A-1146 | CH₂CF₃ | H | Me | F | CH(Et)CH₂NH₂ | O |
| A-1147 | CH₂CF₃ | H | Me | F | CH(*i* -Pr)CH₂NH₂ | O |
| A-1148 | CH₂CF₃ | H | Me | F | CH₂CH₂CH(Me)NH₂ | O |
| A-1149 | CH₂CF₃ | H | Me | F | CH₂CH(Me)CH₂NH₂ | O |
| A-1150 | CH₂CF₃ | H | Me | F | CH(Me)CH₂CH₂NH₂ | O |
| A-1151 | CH₂CF₃ | H | Me | F | CH₂CH(Me)NH(*c* -Pr) | O |
| A-1152 | CH₂CF₃ | H | Me | F | CH₂CH(Me)NHOMe | O |
| A-1153 | CH₂CF₃ | H | Me | F | CH₂CH(Me)N(OMe)S(=O)₂Ph(2-NO₂) | O |
| A-1154 | CH₂CF₃ | H | Me | F | CH₂CH(Me)N(OMe)S(=O)₂Ph(4-NO₂) | O |
| A-1155 | CH₂CF₃ | H | Me | F | CH₂CH₂N(Me)(*t* -Bu) | O |
| A-1156 | CH₂CF₃ | H | Me | F | CH₂CH₂CH₂N(Me)(*t* -Bu) | O |
| A-1157 | CH₂CF₃ | H | Me | F | CH₂CH₂CH₂CH₂N(Me)(*t* -Bu) | O |
| A-1158 | CH₂CF₃ | H | Me | F | CH₂CH₂NHCH₂CF₃ | O |
| A-1159 | CH₂CF₃ | H | Me | F | CH₂CH₂CH₂NHCH₂CF₃ | O |
| A-1160 | CH₂CF₃ | H | Me | F | CH₂CH₂CH₂CH₂NHCH₂CF₃ | O |
| A-1161 | CH₂CF₃ | H | Me | F | CH₂CH₂NHC(=O)C(Me)(CF₃)₂ | O |
| A-1162 | CH₂CF₃ | H | Me | F | CH₂CH₂NHC(=O)Me | O |
| A-1163 | CH₂CF₃ | H | Me | F | CH₂CH₂NHC(=O)(*t* -Bu) | O |
| A-1164 | CH₂CF₃ | H | Me | F | CH₂CH₂CH₂CH₂NHC(=O)CH(CH₃)₂ | O |
| A-1165 | CH₂CF₃ | H | Me | F | CH₂CH₂CH₂CH₂NHC(=O)(*t* -Bu) | O |
| A-1166 | CH₂CF₃ | H | Me | F | CH₂CH₂CH₂CH₂NHC(=O)CH₂(*t* -Bu) | O |
| A-1167 | CH₂CF₃ | H | Me | F | CH₂CH₂NHC(=O)CF₃ | O |
| A-1168 | CH₂CF₃ | H | Me | F | CH₂CH₂NHC(=O)CH₂CF₃ | O |
| A-1169 | CH₂CF₃ | H | Me | F | CH₂CH₂NHC(=O)CF₂CF₃ | O |
| A-1170 | CH₂CF₃ | H | Me | F | CH₂CH₂CH₂NHC(=O)CF₃ | O |
| A-1171 | CH₂CF₃ | H | Me | F | CH₂CH₂CH₂NHC(=O)CH₂CF₃ | O |
| A-1172 | CH₂CF₃ | H | Me | F | CH₂CH₂CH₂NHC(=O)CF₂CF₃ | O |
| A-1173 | CH₂CF₃ | H | Me | F | CH₂CH₂CH₂CH₂NHC(=O)CF₃ | O |
| A-1174 | CH₂CF₃ | H | Me | F | CH₂CH₂CH₂CH₂NHC(=O)CH₂CF₃ | O |
| A-1175 | CH₂CF₃ | H | Me | F | CH₂CH₂CH₂CH₂NHC(=O)CF₂CF₃ | O |
| A-1176 | CH₂CF₃ | H | Me | F | CH₂CH₂CH₂CH₂NHC(=O)CF(CF₃)₂ | O |
| A-1177 | CH₂CF₃ | H | Me | F | CH₂CH₂CH₂CH₂NHC(=O)CCl₃ | O |
| A-1178 | CH₂CF₃ | H | Me | F | CH₂CH(Me)NHC(=O)CF₃ | O |
| A-1179 | CH₂CF₃ | H | Me | F | CH₂CH₂NHC(=O)Ph | O |
| A-1180 | CH₂CF₃ | H | Me | F | CH₂CH₂NHC(=O)Ph(4-CF₃) | O |
| A-1181 | CH₂CF₃ | H | Me | F | CH₂CH₂CH₂NHC(=O)Ph(4-CF₃) | O |
| A-1182 | CH₂CF₃ | H | Me | F | CH₂CH₂CH₂CH₂NHC(=O)Ph(4-CF₃) | O |

**[Table 28]**

| Compound Number | R¹ | R² | R³ | R⁴ | R⁵ | A |
|---|---|---|---|---|---|---|
| A-1183 | CH₂CF₃ | H | Me | F | CH₂CH₂NHC(=O)O(*t* -Bu) | O |
| A-1184 | CH₂CF₃ | H | Me | F | CH₂CH₂CH₂NHC(=O)O(*t* -Bu) | O |
| A-1185 | CH₂CF₃ | H | Me | F | CH₂CH₂CH₂CH₂NHC(=O)O(*t* -Bu) | O |
| A-1186 | CH₂CF₃ | H | Me | F | CH₂CH₂CH₂CH₂NHC(=O)OCH(CH₃)₂ | O |
| A-1187 | CH₂CF₃ | H | Me | F | CH₂CH(Me)NHC(=O)O(*t* -Bu) | O |
| A-1188 | CH₂CF₃ | H | Me | F | CH₂C*H(Me)NHC(=O)O(*t* -Bu) : (R) | O |
| A-1189 | CH₂CF₃ | H | Me | F | CH₂C*H(Me)NHC(=O)O(*t* -Bu) : (S) | O |
| A-1190 | CH₂CF₃ | H | Me | F | CH₂CH(Et)NHC(=O)O(*t* -Bu) | O |
| A-1191 | CH₂CF₃ | H | Me | F | CH₂CH(*i* -Pr)NHC(=O)O(*t* -Bu) | O |
| A-1192 | CH₂CF₃ | H | Me | F | CH(Me)CH₂NHC(=O)O(*t* -Bu) | O |
| A-1193 | CH₂CF₃ | H | Me | F | CH(Et)CH₂NHC(=O)O(*t* -Bu) | O |
| A-1194 | CH₂CF₃ | H | Me | F | CH(*i* -Pr)CH₂NHC(=O)O(*t* -Bu) | O |
| A-1195 | CH₂CF₃ | H | Me | F | CH₂CH(Me)CH₂NHC(=O)O(*t* -Bu) | O |
| A-1196 | CH₂CF₃ | H | Me | F | CH₂CH₂NHC(=O)OC(Me)₂CF₃ | O |
| A-1197 | CH₂CF₃ | H | Me | F | CH₂CH₂CH₂NHC(=O)OCH₂CF₃ | O |
| A-1198 | CH₂CF₃ | H | Me | F | CH₂CH₂CH₂NHC(=O)OCH₂CH₂CF₃ | O |
| A-1199 | CH₂CF₃ | H | Me | F | CH₂CH₂CH₂CH₂NHC(=O)OCH₂CCl₃ | O |
| A-1200 | CH₂CF₃ | H | Me | F | CH₂CH₂CH₂CH₂NHC(=O)OCH₂CF₃ | O |
| A-1201 | CH₂CF₃ | H | Me | F | CH₂CH₂NHC(=O)NH(*t* -Bu) | O |
| A-1202 | CH₂CF₃ | H | Me | F | CH₂CH₂CH₂NHC(=O)NH(*t* -Bu) | O |
| A-1203 | CH₂CF₃ | H | Me | F | CH2CH2CH2CH2NHC(=O)NHEt | O |
| A-1204 | CH₂CF₃ | H | Me | F | CH₂CH₂CH₂CH₂NHC(=O)NH(*t* -Bu) | O |
| A-1205 | CH₂CF₃ | H | Me | F | CH₂CH₂CH₂NHC(=O)NHCH₂CCl₃ | O |
| A-1206 | CH₂CF₃ | H | Me | F | CH₂CH₂CH₂NHC(=O)NHCH₂CH₂F | O |
| A-1207 | CH₂CF₃ | H | Me | F | CH₂CH₂CH₂NHC(=O)NHCH₂CF₃ | O |
| A-1208 | CH₂CF₃ | H | Me | F | CH₂CH₂CH₂CH₂NHC(=O)NHCH₂CCl₃ | O |
| A-1209 | CH₂CF₃ | H | Me | F | CH₂CH₂CH₂CH₂NHC(=O)NHCH₂CH₂F | O |
| A-1210 | CH₂CF₃ | H | Me | F | CH₂CH₂CH₂CH₂NHC(=O)NHCH₂CF₃ | O |
| A-1211 | CH₂CF₃ | H | Me | F | CH₂CH₂NHS(=O)Me | O |
| A-1212 | CH₂CF₃ | H | Me | F | CH₂CH₂CH₂NHS(=O)Me | O |
| A-1213 | CH₂CF₃ | H | Me | F | CH₂CH₂CH₂CH₂NHS(=O)Me | O |
| A-1214 | CH₂CF₃ | H | Me | F | CH₂CH₂CH₂CH₂NHS(=O)CH(CH₃)₂ | O |
| A-1215 | CH₂CF₃ | H | Me | F | CH₂CH₂CH₂CH₂CH₂NHS(=O)Me | O |
| A-1216 | CH₂CF₃ | H | Me | F | CH₂CH₂NHS(=O)CHF₂ | O |
| A-1217 | CH₂CF₃ | H | Me | F | CH₂CH₂CH₂NHS(=O)CHF₂ | O |
| A-1218 | CH₂CF₃ | H | Me | F | CH₂CH₂CH₂CH₂NHS(=O)CHF₂ | O |
| A-1219 | CH₂CF₃ | H | Me | F | CH₂CH₂CH₂CH₂CH₂NHS(=O)CHF₂ | O |
| A-1220 | CH₂CF₃ | H | Me | F | CH₂CH(Me)NHS(=O)CHF₂ | O |
| A-1221 | CH₂CF₃ | H | Me | F | CH(Me)CH₂NHS(=O)CHF₂ | O |
| A-1222 | CH₂CF₃ | H | Me | F | CH₂CH₂CH(Me)NHS(=O)CHF₂ | O |
| A-1223 | CH₂CF₃ | H | Me | F | CH₂CH(Me)CH₂NHS(=O)CHF₂ | O |
| A-1224 | CH₂CF₃ | H | Me | F | CH(Me)CH₂CH₂NHS(=O)CHF₂ | O |
| A-1225 | CH₂CF₃ | H | Me | F | CH₂CH₂NHS(=O)CF₃ | O |
| A-1226 | CH₂CF₃ | H | Me | F | CH₂CH₂CH₂NHS(=O)CF₃ | O |

**[Table 29]**

| Compound Number | R¹ | R² | R³ | R⁴ | R⁵ | A |
|---|---|---|---|---|---|---|
| A-1227 | CH₂CF₃ | H | Me | F | CH₂CH₂CH₂CH₂NHS(=O)CF₃ | O |
| A-1228 | CH₂CF₃ | H | Me | F | CH₂CH₂CH₂CH₂CH₂NHS(=O)CF₃ | O |
| A-1229 | CH₂CF₃ | H | Me | F | CH₂CH₂CH₂NHS(=O)Ph | O |
| A-1230 | CH₂CF₃ | H | Me | F | CH₂CH₂CH₂CH₂NHS(=O)Ph | O |
| A-1231 | CH₂CF₃ | H | Me | F | CH₂CH₂CH₂CH₂CH₂NHS(=O)Ph | O |
| A-1232 | CH₂CF₃ | H | Me | F | CH₂CH₂CH₂CH₂CH₂NHS(=O)Ph(4-CF₃) | O |
| A-1233 | CH₂CF₃ | H | Me | F | CH₂CH(Me)NHS(=O)Ph(4-CF₃) | O |
| A-1234 | CH₂CF₃ | H | Me | F | CH₂CH(Et)NHS(=O)Ph(4-CF₃) | O |
| A-1235 | CH₂CF₃ | H | Me | F | CH(Me)CH₂NHS(=O)Ph(4-CF₃) | O |
| A-1236 | CH₂CF₃ | H | Me | F | CH(Et)CH₂NHS(=O)Ph(4-CF₃) | O |
| A-1237 | CH₂CF₃ | H | Me | F | CH₂CH(Et)CH₂NHS(=O)Ph(4-CF₃) | O |
| A-1238 | CH₂CF₃ | H | Me | F | CH₂CH₂NHS(=O)₂Me | O |
| A-1239 | CH₂CF₃ | H | Me | F | CH₂CH₂CH₂NHS(=O)₂Me | O |
| A-1240 | CH₂CF₃ | H | Me | F | CH₂CH₂CH₂CH₂NHS(=O)₂Me | O |
| A-1241 | CH₂CF₃ | H | Me | F | CH₂CH₂CH₂CH₂NHS(=O)₂CH(CH₃)₂ | O |
| A-1242 | CH₂CF₃ | H | Me | F | CH₂CH₂CH₂CH₂CH₂NHS(=O)₂Me | O |
| A-1243 | CH₂CF₃ | H | Me | F | CH₂CH₂NHS(=O)₂CHF₂ | O |
| A-1244 | CH₂CF₃ | H | Me | F | CH₂CH₂CH₂NHS(=O)₂CHF₂ | O |
| A-1245 | CH₂CF₃ | H | Me | F | CH₂CH₂CH₂CH₂NHS(=O)₂CHF₂ | O |
| A-1246 | CH₂CF₃ | H | Me | F | CH₂CH₂CH₂CH₂CH₂NHS(=O)₂CHF₂ | O |
| A-1247 | CH₂CF₃ | H | Me | F | CH₂CH(Me)NHS(=O)₂CHF₂ | O |
| A-1248 | CH₂CF₃ | H | Me | F | CH(Me)CH₂NHS(=O)₂CHF₂ | O |
| A-1249 | CH₂CF₃ | H | Me | F | CH₂CH₂CH(Me)NHS(=O)₂CHF₂ | O |
| A-1250 | CH₂CF₃ | H | Me | F | CH₂CH(Me)CH₂NHS(=O)₂CHF₂ | O |
| A-1251 | CH₂CF₃ | H | Me | F | CH(Me)CH₂CH₂NHS(=O)₂CHF₂ | O |
| A-1252 | CH₂CF₃ | H | Me | F | CH₂CH(Me)N(Me)S(=O)₂CHF₂ | O |
| A-1253 | CH₂CF₃ | H | Me | F | CH(Me)CH₂N(Me)S(=O)₂CHF₂ | O |
| A-1254 | CH₂CF₃ | H | Me | F | CH₂CH₂CH(Me)N(Me)S(=O)₂CHF₂ | O |
| A-1255 | CH₂CF₃ | H | Me | F | CH₂CH(Me)CH₂N(Me)S(=O)₂CHF₂ | O |
| A-1256 | CH₂CF₃ | H | Me | F | CH(Me)CH₂CH₂N(Me)S(=O)₂CHF₂ | O |
| A-1257 | CH₂CF₃ | H | Me | F | CH₂CH₂CH₂NHS(=O)₂Ph | O |
| A-1258 | CH₂CF₃ | H | Me | F | CH₂CH₂CH₂CH₂NHS(=O)₂Ph | O |
| A-1259 | CH₂CF₃ | H | Me | F | CH₂CH₂CH₂CH₂CH₂NHS(=O)₂Ph | O |
| A-1260 | CH₂CF₃ | H | Me | F | CH₂CH₂CH₂CH₂CH₂NHS(=O)₂Ph(4-CF₃) | O |
| A-1261 | CH₂CF₃ | H | Me | F | CH₂CH(Me)NHS(=O)₂Ph(4-CF₃) | O |
| A-1262 | CH₂CF₃ | H | Me | F | CH₂CH(Et)NHS(=O)₂Ph(4-CF₃) | O |
| A-1263 | CH₂CF₃ | H | Me | F | CH(Me)CH₂NHS(=OhPh(4-CF₃) | O |
| A-1264 | CH₂CF₃ | H | Me | F | CH(Et)CH₂NHS(=O)₂Ph(4-CF₃) | O |
| A-1265 | CH₂CF₃ | H | Me | F | CH₂CH(Me)CH₂NHS(=O)₂Ph(4-CF₃) | O |
| A-1266 | CH₂CF₃ | H | Me | F | CH₂CH₂SiMe₃ | O |
| A-1267 | CH₂CF₃ | H | Me | F | CH₂CH₂CH₂SiMe₃ | O |
| A-1268 | CH₂CF₃ | H | Me | F | CH₂CH₂CH₂CH₂SiMe₃ | O |
| A-1269 | CH₂CF₃ | H | Me | F | CH₂Ph | O |
| A-1270 | CH₂CF₃ | H | Me | F | CH₂Ph(2-CF₃) | O |

**[Table 30]**

| Compound Number | R¹ | R² | R³ | R⁴ | R⁵ | A |
|---|---|---|---|---|---|---|
| A-1271 | CH₂CF₃ | H | Me | F | CH₂Ph(3-CF₃) | O |
| A-1272 | CH₂CF₃ | H | Me | F | CH₂Ph(4-CF₃) | O |
| A-1273 | CH₂CF₃ | H | Me | F | CH₂Ph(2-OCF₃) | O |
| A-1274 | CH₂CF₃ | H | Me | F | CH₂Ph(3-OCF₃) | O |
| A-1275 | CH₂CF₃ | H | Me | F | CH₂Ph(4-OCF₃) | O |
| A-1276 | CH₂CF₃ | H | Me | F | CH₂Ph(2-SCF₃) | O |
| A-1277 | CH₂CF₃ | H | Me | F | CH₂Ph(3-SCF₃) | O |
| A-1278 | CH₂CF₃ | H | Me | F | CH₂Ph(4-SCF₃) | O |
| A-1279 | CH₂CF₃ | H | Me | F | CH₂Ph(3-CH₂SCF₃) | O |
| A-1280 | CH₂CF₃ | H | Me | F | CH₂Ph(4-F) | O |
| A-1281 | CH₂CF₃ | H | Me | F | CH₂Ph(4-Cl) | O |
| A-1282 | CH₂CF₃ | H | Me | F | CH₂Ph(4-Br) | O |
| A-1283 | CH₂CF₃ | H | Me | F | CH₂Ph(4-Me) | O |
| A-1284 | CH₂CF₃ | H | Me | F | CH₂Ph[4-(*t* -Bu)] | O |
| A-1285 | CH₂CF₃ | H | Me | F | CH₂Ph(4-CN) | O |
| A-1286 | CH₂CF₃ | H | Me | F | CH₂Ph(4-NO₂) | O |
| A-1287 | CH₂CF₃ | H | Me | F | CH₂Ph(4-OCHF₂) | O |
| A-1288 | CH₂CF₃ | H | Me | F | CH₂Ph(4-SCHF₂) | O |
| A-1289 | CH₂CF₃ | H | Me | F | CH₂Ph(4-CH₂SCF₃) | O |
| A-1290 | CH₂CF₃ | H | Me | F | CH₂Ph[4-CF(CF₃)₂] | O |
| A-1291 | CH₂CF₃ | H | Me | F | CH₂Ph(4-CH₂SCF₃) | O |
| A-1292 | CH₂CF₃ | H | Me | F | CH₂Ph[4-Ph(4-CF₃)] | O |
| A-1293 | CH₂CF₃ | H | Me | F | CH₂Ph(2,4-Cl₂) | O |
| A-1294 | CH₂CF₃ | H | Me | F | CH₂Ph[2,5-(CF₃)₂] | O |
| A-1295 | CH₂CF₃ | H | Me | F | CH₂Ph(3,4-Cl₂) | O |
| A-1296 | CH₂CF₃ | H | Me | F | CH₂Ph(3-CF₃-4-F) | O |
| A-1297 | CH₂CF₃ | H | Me | F | CH₂Ph(3-CF₃-4-Cl) | O |
| A-1298 | CH₂CF₃ | H | Me | F | CH₂Ph(3-F-4-CF₃) | O |
| A-1299 | CH₂CF₃ | H | Me | F | CH₂Ph(2,4,6-F₃) | O |
| A-1300 | CH₂CF₃ | H | Me | F | CH₂Ph(3,4,5-F₃) | O |
| A-1301 | CH₂CF₃ | H | Me | F | CH₂Ph(2,3,4-F₃) | O |
| A-1302 | CH₂CF₃ | H | Me | F | CH₂Ph(3,4,5-Cl₃) | O |
| A-1303 | CH₂CF₃ | H | Me | F | CH₂CH₂Ph | O |
| A-1304 | CH₂CF₃ | H | Me | F | CH₂CH₂Ph(4-F) | O |
| A-1305 | CH₂CF₃ | H | Me | F | CH₂CH₂Ph(4-Cl) | O |
| A-1306 | CH₂CF₃ | H | Me | F | CH₂CH₂Ph(4-Br) | O |
| A-1307 | CH₂CF₃ | H | Me | F | CH₂CH₂Ph[4-(*t* -Bu)] | O |
| A-1308 | CH₂CF₃ | H | Me | F | CH₂CH₂Ph(2-CF₃) | O |
| A-1309 | CH₂CF₃ | H | Me | F | CH₂CH₂Ph(3-CF₃) | O |
| A-1310 | CH₂CF₃ | H | Me | H | CH₂CH₂Ph(4-CF₃) | O |
| A-1311 | CH₂CF₃ | H | Me | F | CH₂CH₂Ph(4-CF₃) | O |
| A-1312 | CH₂CF₃ | H | Me | F | CH₂CH₂Ph(4-CF(CF₃)₂) | O |
| A-1313 | CH₂CF₃ | H | Me | F | CH₂CH₂Ph[4-(c-Pr)] | O |
| A-1314 | CH₂CF₃ | H | Me | F | CH₂CH₂Ph{4-[*c* -Pr(2,2-F₂)]} | O |

**[Table 31]**

| Compound Number | R¹ | R² | R³ | R⁴ | R⁵ | A |
|---|---|---|---|---|---|---|
| A-1315 | CH₂CF₃ | H | Me | F | CH₂CH₂Ph(4-OMe) | O |
| A-1316 | CH₂CF₃ | H | Me | F | CH₂CH₂Ph(3-OCF₃) | O |
| A-1317 | CH₂CF₃ | H | Me | F | CH₂CH₂Ph(4-OCHF₂) | O |
| A-1318 | CH₂CF₃ | H | Me | F | CH₂CH₂Ph(4-OCF₃) | O |
| A-1319 | CH₂CF₃ | H | Me | F | CH₂CH₂Ph(4-SMe) | O |
| A-1320 | CH₂CF₃ | H | Me | F | CH₂CH₂Ph(4-SCHF₂) | O |
| A-1321 | CH₂CF₃ | H | Me | F | CH₂CH₂Ph(4-SCF₃) | O |
| A-1322 | CH₂CF₃ | H | Me | F | CH₂CH₂Ph[4-S(=O)Me] | O |
| A-1323 | CH₂CF₃ | H | Me | F | CH₂CH₂Ph[4-S(=O)CF₃] | O |
| A-1324 | CH₂CF₃ | H | Me | F | CH₂CH₂Ph[4-S(=O)₂Me] | O |
| A-1325 | CH₂CF₃ | H | Me | F | CH₂CH₂Ph[4-S(=O)₂CF₃] | O |
| A-1326 | CH₂CF₃ | H | Me | F | CH₂CH₂Ph(4-CH₂SMe) | O |
| A-1327 | CH₂CF₃ | H | Me | F | CH₂CH₂Ph(4-CH₂SCF₃) | O |
| A-1328 | CH₂CF₃ | H | Me | F | CH₂CH₂Ph[4-OS(=O)₂Me] | O |
| A-1329 | CH₂CF₃ | H | Me | F | CH₂CH₂Ph[4-OS(=O)₂CF₃] | O |
| A-1330 | CH₂CF₃ | H | Me | F | CH₂CH₂Ph[4-Ph(4-CF₃)] | O |
| A-1331 | CH₂CF₃ | H | Me | F | CH₂CH₂Ph(4-CH₂Ph) | O |
| A-1332 | CH₂CF₃ | H | Me | F | CH₂CH₂Ph(4-OCH₂Ph) | O |
| A-1333 | CH₂CF₃ | H | Me | F | CH₂CH₂Ph(4-CN) | O |
| A-1334 | CH₂CF₃ | H | Me | F | CH₂CH₂Ph(4-NO₂) | O |
| A-1335 | CH₂CF₃ | H | Me | F | CH₂CH₂Ph(2,4-Cl₂) | O |
| A-1336 | CH₂CF₃ | H | Me | F | CH₂CH₂Ph(3,4-Cl₂) | O |
| A-1337 | CH₂CF₃ | H | Me | F | CH₂CH₂Ph(3-CF₃-4-F) | O |
| A-1338 | CH₂CF₃ | H | Me | F | CH₂CH₂Ph(2-CF₃-4-F) | O |
| A-1339 | CH₂CF₃ | H | Me | F | CH₂CH₂Ph(3-F-4-CF₃) | O |
| A-1340 | CH₂CF₃ | H | Me | F | CH₂CH₂Ph(2-F-4-CF₃) | O |
| A-1341 | CH₂CF₃ | H | Me | F | CH₂CH₂Ph(3-Cl-4-OCHF₂) | O |
| A-1342 | CH₂CF₃ | H | Me | F | CH₂CH₂Ph(3,4,5-Cl₃) | O |
| A-1343 | CH₂CF₃ | H | Me | F | CH₂CH₂Ph(2,3,4-F₃) | O |
| A-1344 | CH₂CF₃ | H | Me | F | CH₂CH₂Ph(2,4,5-F₃) | O |
| A-1345 | CH₂CF₃ | H | Me | F | CH₂CH₂Ph(3,4,5-F₃) | O |
| A-1346 | CH₂CF₃ | H | Me | F | CH₂CH₂Ph(2,4,6-F₃) | O |
| A-1347 | CH₂CF₃ | H | Me | F | CH₂CH₂CH₂Ph | O |
| A-1348 | CH₂CF₃ | H | Me | F | CH₂CH₂CH₂Ph(3-CF₃) | O |
| A-1349 | CH₂CF₃ | H | Me | F | CH₂CH₂CH₂Ph(4-CF₃) | O |
| A-1350 | CH₂CF₃ | H | Me | F | CH₂CH₂CH₂Ph(4-F) | O |
| A-1351 | CH₂CF₃ | H | Me | F | CH₂CH₂CH₂Ph[4-(*t* -Bu)] | O |
| A-1352 | CH₂CF₃ | H | Me | F | CH₂CH₂CH₂Ph(4-CN) | O |
| A-1353 | CH₂CF₃ | H | Me | F | CH₂CH₂CH₂Ph(4-CF₃) | O |
| A-1354 | CH₂CF₃ | H | Me | F | CH₂CH₂CH₂Ph(4-OCHF₂) | O |
| A-1355 | CH₂CF₃ | H | Me | F | CH₂CH₂CH₂Ph(4-OCF₃) | O |
| A-1356 | CH₂CF₃ | H | Me | F | CH₂CH₂CH₂Ph(4-SCHF₂) | O |
| A-1357 | CH₂CF₃ | H | Me | F | CH₂CH₂CH₂Ph(4-SCF₃) | O |
| A-1358 | CH₂CF₃ | H | Me | F | CH₂CH₂CH₂Ph[4-CF(CF₃)₂] | O |

**[Table 32]**

| Compound Number | R¹ | R² | R³ | R⁴ | R⁵ | A |
|---|---|---|---|---|---|---|
| A-1359 | CH₂CF₃ | H | Me | F | CH₂CH₂CH₂Ph(3,4,5-F₃) | O |
| A-1360 | CH₂CF₃ | H | Me | F | CH₂CH₂CH₂Ph(2,4,6-F₃) | O |
| A-1361 | CH₂CF₃ | H | Me | F | CH₂CH₂CH₂CH₂Ph | O |
| A-1362 | CH₂CF₃ | H | Me | F | CH₂CH₂CH₂CH₂Ph(4-F) | O |
| A-1363 | CH₂CF₃ | H | Me | F | CH₂CH₂CH₂CH₂Ph(4-CF₃) | O |
| A-1364 | CH₂CF₃ | H | Me | F | CH₂CH₂CH₂CH₂Ph(4-OCF₃) | O |
| A-1365 | CH₂CF₃ | H | Me | F | CH₂CH₂CH₂CH₂Ph(4-SCF₃) | O |
| A-1366 | CH₂CF₃ | H | Me | F | CH₂CH₂CH₂CH₂CH₂Ph | O |
| A-1367 | CH₂CF₃ | H | Me | F | CH₂CH₂CH₂CH₂CH₂CH₂Ph | O |
| A-1368 | CH₂CF₃ | H | Me | F | CH₂CF₂Ph(4-F) | O |
| A-1369 | CH₂CF₃ | H | Me | F | CH₂CF₂Ph(4-CF₃) | O |
| A-1370 | CH₂CF₃ | H | Me | H | CH₂CF₂Ph(3,4,5-F₃) | O |
| A-1371 | CH₂CF₃ | H | Me | F | CH₂CF₂Ph(3,4,5-F₃) | O |
| A-1372 | CH₂CF₃ | H | Me | F | CH₂CH₂OPh | O |
| A-1373 | CH₂CF₃ | H | Me | F | CH₂CH₂OPh(4-F) | O |
| A-1374 | CH₂CF₃ | H | Me | F | CH₂CH₂OPh(4-CF₃) | O |
| A-1375 | CH₂CF₃ | H | Me | F | CH₂CH₂CH₂OPh | O |
| A-1376 | CH₂CF₃ | H | Me | F | CH₂CH₂CH₂OPh(4-Cl) | O |
| A-1377 | CH₂CF₃ | H | Me | F | CH₂CH₂CH₂OPh(4-CF₃) | O |
| A-1378 | CH₂CF₃ | H | Me | F | CH₂CH₂CH₂CH₂OPh(4-CF₃) | O |
| A-1379 | CH₂CF₃ | H | Me | F | CH₂CH₂CH₂CH₂OPh(4-OCF₃) | O |
| A-1380 | CH₂CF₃ | H | Me | F | CH₂CH₂CH₂CH₂CH₂OPh(4-CF₃) | O |
| A-1381 | CH₂CF₃ | H | Me | F | CH₂CH₂OCH₂Ph | O |
| A-1382 | CH₂CF₃ | H | Me | F | CH₂CH₂CH₂OCH₂Ph | O |
| A-1383 | CH₂CF₃ | H | Me | F | CH₂CH₂CH₂CH₂OCH₂Ph | O |
| A-1384 | CH₂CF₃ | H | Me | F | CH₂CH₂CH₂CH₂CH₂OCH₂Ph | O |
| A-1385 | CH₂CF₃ | H | Me | F | CH₂CH₂SPh | O |
| A-1386 | CH₂CF₃ | H | Me | F | CH₂CH₂CH₂SPh | O |
| A-1387 | CH₂CF₃ | H | Me | F | CH₂CH₂CH₂SPh(4-F) | O |
| A-1388 | CH₂CF₃ | H | Me | F | CH₂CH₂CH₂SPh(4-Cl) | O |
| A-1389 | CH₂CF₃ | H | Me | F | CH₂CH₂CH₂SPh(4-Br) | O |
| A-1390 | CH₂CF₃ | H | Me | F | CH₂CH₂CH₂SPh[4-(*t*-Bu)] | O |
| A-1391 | CH₂CF₃ | H | Me | F | CH₂CH₂CH₂SPh(3-CF₃) | O |
| A-1392 | CH₂CF₃ | H | Me | F | CH₂CH₂CH₂SPh(4-CF₃) | O |
| A-1393 | CH₂CF₃ | H | Me | F | CH₂CH₂CH₂SPh(3-SCF₃) | O |
| A-1394 | CH₂CF₃ | H | Me | F | CH₂CH₂CH₂SPh(4-SCF₃) | O |
| A-1395 | CH₂CF₃ | H | Me | F | CH₂CH₂CH₂CH₂SPh | O |
| A-1396 | CH₂CF₃ | H | Me | F | CH₂CH₂CH₂CH₂SPh(4-F) | O |
| A-1397 | CH₂CF₃ | H | Me | F | CH₂CH₂CH₂CH₂SPh(4-Cl) | O |
| A-1398 | CH₂CF₃ | H | Me | F | CH₂CH₂CH₂CH₂SPh[4-(*t*-Bu)] | O |
| A-1399 | CH₂CF₃ | H | Me | F | CH₂CH₂CH₂CH₂SPh(4-CF₃) | O |
| A-1400 | CH₂CF₃ | H | Me | F | CH₂CH₂CH₂CH₂CH₂SPh | O |
| A-1401 | CH₂CF₃ | H | Me | F | CH₂CH₂CH₂CH₂CH₂SPh(4-F) | O |
| A-1402 | CH₂CF₃ | H | Me | F | CH₂CH₂CH₂CH₂CH₂SPh(4-Cl) | O |

**[Table 33]**

| Compound Number | R¹ | R² | R³ | R⁴ | R⁵ | A |
|---|---|---|---|---|---|---|
| A-1403 | CH₂CF₃ | H | Me | F | CH₂CH₂CH₂CH₂CH₂SPh(4-CF₃) | O |
| A-1404 | CH₂CF₃ | H | Me | F | CH₂CH₂S(=O)Ph | O |
| A-1405 | CH₂CF₃ | H | Me | F | CH₂CH₂CH₂S(=O)Ph | O |
| A-1406 | CH₂CF₃ | H | Me | F | CH₂CH₂CH₂S(=O)Ph(4-F) | O |
| A-1407 | CH₂CF₃ | H | Me | F | CH₂CH₂CH₂S(=O)Ph[4-(*t*-Bu)] | O |
| A-1408 | CH₂CF₃ | H | Me | F | CH₂CH₂CH₂S(=O)Ph(4-CF₃) | O |
| A-1409 | CH₂CF₃ | H | Me | F | CH₂CH₂S(=O)₂Ph | O |
| A-1410 | CH₂CF₃ | H | Me | F | CH₂CH₂S(=O)₂Ph(4-CF₃) | O |
| A-1411 | CH₂CF₃ | H | Me | F | CH₂CH₂CH₂S(=O)₂Ph(4-F) | O |
| A-1412 | CH₂CF₃ | H | Me | F | CH₂CH₂CH₂S(=O)₂Ph(4-Cl) | O |
| A-1413 | CH₂CF₃ | H | Me | F | CH₂CH₂CH₂S(=O)₂Ph(4-CF₃) | O |
| A-1414 | CH₂CF₃ | H | Me | F | CH₂CH₂CH₂CH₂S(=O)₂Ph | O |
| A-1415 | CH₂CF₃ | H | Me | F | CH₂CH₂CH₂CH₂S(=O)₂Ph(4-Cl) | O |
| A-1416 | CH₂CF₃ | H | Me | F | CH₂CH₂CH₂CH₂S(=O)₂Ph(4-CF₃) | O |
| A-1417 | CH₂CF₃ | H | Me | F | CH₂CH₂SCH₂Ph | O |
| A-1418 | CH₂CF₃ | H | Me | F | CH₂CH₂CH₂SCH₂Ph | O |
| A-1419 | CH₂CF₃ | H | Me | F | CH₂CH₂CH₂SCH₂Ph(2-Cl) | O |
| A-1420 | CH₂CF₃ | H | Me | F | CH₂CH₂CH₂SCH₂Ph(3-Cl) | O |
| A-1421 | CH₂CF₃ | H | Me | F | CH₂CH₂CH₂SCH₂Ph(4-Cl) | O |
| A-1422 | CH₂CF₃ | H | Me | F | CH₂CH₂CH₂SCH₂Ph(2-SCF₃) | O |
| A-1423 | CH₂CF₃ | H | Me | F | CH₂CH₂CH₂SCH₂Ph(3-CF₃) | O |
| A-1424 | CH₂CF₃ | H | Me | F | CH₂CH₂CH₂SCH₂Ph(4-CF₃) | O |
| A-1425 | CH₂CF₃ | H | Me | F | CH₂CH₂CH₂SCH₂Ph(4-NO₂) | O |
| A-1426 | CH₂CF₃ | H | Me | F | CH₂CH₂CH₂CH₂SCH₂Ph | O |
| A-1427 | CH₂CF₃ | H | Me | F | CH₂CH₂CH₂CH₂SCH₂Ph(4-Cl) | O |
| A-1428 | CH₂CF₃ | H | Me | F | CH₂CH₂CH₂CH₂SCH₂Ph(4-CF₃) | O |
| A-1429 | CH₂CF₃ | H | Me | F | CH₂CH₂CH₂CH₂SCH₂Ph(4-CN) | O |
| A-1430 | CH₂CF₃ | H | Me | F | CH₂CH₂CH₂CH₂CH₂SCH₂Ph | O |
| A-1431 | CH₂CF₃ | H | Me | F | CH₂CH₂SCH₂CH₂Ph | O |
| A-1432 | CH₂CF₃ | H | Me | F | CH₂CH₂CH₂SCH₂CH₂Ph | O |
| A-1433 | CH₂CF₃ | H | Me | F | CH₂CH₂CH₂CH₂SCH₂CH₂Ph | O |
| A-1434 | CH₂CF₃ | H | Me | F | CH₂CH₂SCH(Me)Ph | O |
| A-1435 | CH₂CF₃ | H | Me | F | CH₂CH₂CH₂SCH(Me)Ph | O |
| A-1436 | CH₂CF₃ | H | Me | F | CH₂CH₂CH₂CH₂SCH(Me)Ph | O |
| A-1437 | CH₂CF₃ | H | Me | F | CH₂CH₂S(=O)CH₂Ph | O |
| A-1438 | CH₂CF₃ | H | Me | F | CH₂CH₂S(=O)CH₂Ph(4-CF₃) | O |
| A-1439 | CH₂CF₃ | H | Me | F | CH₂CH₂CH₂S(=O)CH₂Ph | O |
| A-1440 | CH₂CF₃ | H | Me | F | CH₂CH₂CH₂S(=O)CH₂Ph(2-SCF₃) | O |
| A-1441 | CH₂CF₃ | H | Me | F | CH₂CH₂CH₂S(=O)CH₂Ph(4-SCF₃) | O |
| A-1442 | CH₂CF₃ | H | Me | F | CH₂CH₂CH₂S(=O)CH₂Ph(4-Cl) | O |
| A-1443 | CH₂CF₃ | H | Me | F | CH₂CH₂CH₂S(=O)CH₂Ph(4-CF₃) | O |
| A-1444 | CH₂CF₃ | H | Me | F | CH₂CH₂CH₂CH₂S(=O)CH₂Ph | O |
| A-1445 | CH₂CF₃ | H | Me | F | CH₂CH₂CH₂CH₂S(=O)CH₂Ph(4-CF₃) | O |
| A-1446 | CH₂CF₃ | H | Me | F | CH₂CH₂CH₂CH₂CH₂S(=O)CH₂Ph(4-CF₃) | O |

**[Table 34]**

| Compound Number | R¹ | R² | R³ | R⁴ | R⁵ | A |
|---|---|---|---|---|---|---|
| A-1447 | CH₂CF₃ | H | Me | F | CH₂CH₂S(=O)₂CH₂Ph | O |
| A-1448 | CH₂CF₃ | H | Me | F | CH₂CH₂S(=O)₂CH₂Ph(4-CF₃) | O |
| A-1449 | CH₂CF₃ | H | Me | F | CH₂CH₂CH₂S(=O)₂CH₂Ph | O |
| A-1450 | CH₂CF₃ | H | Me | F | CH₂CH₂CH₂S(=O)₂CH₂Ph(4-Cl) | O |
| A-1451 | CH₂CF₃ | H | Me | F | CH₂CH₂CH₂S(=O)₂CH₂Ph(4-CF₃) | O |
| A-1452 | CH₂CF₃ | H | Me | F | CH₂CH₂CH₂CH₂S(=O)₂CH₂Ph | O |
| A-1453 | CH₂CF₃ | H | Me | F | CH₂CH₂CH₂CH₂S(=O)₂CH₂Ph(4-CF₃) | O |
| A-1454 | CH₂CF₃ | H | Me | F | CH₂CH₂CH₂CH₂CH₂S(=O)₂CH₂Ph | O |
| A-1455 | CH₂CF₃ | H | Me | F | CH₂CH₂CH₂CH₂CH₂S(=O)₂CH₂Ph(4-CF₃) | O |
| A-1456 | CH₂CF₃ | H | Me | F | CH₂CH₂ON=CH(*t*-Bu) | O |
| A-1457 | CH₂CF₃ | H | Me | F | CH₂CH₂CH₂ON=CH(*t*-Bu) | O |
| A-1458 | CH₂CF₃ | H | Me | F | CH₂CH₂CH₂CH₂ON=C(Me)(*c*-Pr) | O |
| A-1459 | CH₂CF₃ | H | Me | F | CH₂CH₂ON=C(Me)CF₃ | O |
| A-1460 | CH₂CF₃ | H | Me | F | CH₂CH₂CH₂ON=CHCF₃ | O |
| A-1461 | CH₂CF₃ | H | Me | F | CH₂CH₂CH₂ON=C(Me)CF₃ | O |
| A-1462 | CH₂CF₃ | H | Me | F | CH₂CH₂CH₂ON=C(Me)CCl₃ | O |
| A-1463 | CH₂CF₃ | H | Me | F | CH₂CH₂CH₂CH₂ON=CHCF₃ | O |
| A-1464 | CH₂CF₃ | H | Me | F | CH₂CH₂CH₂CH₂ON=C(Me)CF₃ | O |
| A-1465 | CH₂CF₃ | H | Me | F | CH₂CH₂CH₂CH₂CH₂ON=C(Me)CF₃ | O |
| A-1466 | CH₂CF₃ | H | Me | F | CH₂CH₂ON=CHPh | O |
| A-1467 | CH₂CF₃ | H | Me | F | CH₂CH₂ON=CHPh(4-CF₃) | O |
| A-1468 | CH₂CF₃ | H | Me | F | CH₂CH₂ON=CHPh(4-SCF₃) | O |
| A-1469 | CH₂CF₃ | H | Me | F | CH₂CH₂CH₂ON=CHPh(3-CF₃) | O |
| A-1470 | CH₂CF₃ | H | Me | F | CH₂CH₂CH₂ON=CHPh(4-CF₃) | O |
| A-1471 | CH₂CF₃ | H | Me | F | CH₂CH₂CH₂ON=CHPh(4-SCF₃) | O |
| A-1472 | CH₂CF₃ | H | Me | F | CH₂CH₂CH₂ON=C(Me)Ph(4-CF₃) | O |
| A-1473 | CH₂CF₃ | H | Me | F | CH₂CH₂CH₂CH₂ON=CHPh(4-SCF₃) | O |
| A-1474 | CH₂CF₃ | H | Me | F | CH₂CH₂(adamant-1-yl) | O |
| A-1475 | CH₂CF₃ | H | Me | F | CH₂CH₂CH₂(3-CF₃-pyrazol-1-yl) | O |
| A-1476 | CH₂CF₃ | H | Me | F | CH₂CH₂CH₂CH₂(3-CF₃-pyrazol-1-yl) | O |
| A-1477 | CH₂CF₃ | H | Me | F | CH₂CH₂CH₂CH₂CH₂(3-CF₃-pyrazol-1-yl) | O |
| A-1478 | CH₂CF₃ | H | Me | F | CH₂(1-Me-4-CF₃-imidazol-5-yl) | O |
| A-1479 | CH₂CF₃ | H | Me | F | CH₂CH₂(1-Me-4-CF₃-imidazol-5-yl) | O |
| A-1480 | CH₂CF₃ | H | Me | F | CH₂CH₂CH₂(1-Me-4-CF₃-imidazol-5-yl) | O |
| A-1481 | CH₂CF₃ | H | Me | F | CH₂CH₂CH₂(3-CF₃-1,2,4-triazol-1-yl) | O |
| A-1482 | CH₂CF₃ | H | Me | F | CH₂CH₂CH₂CH₂(3-CF₃-1,2,4-triazol-1-yl) | O |
| A-1483 | CH₂CF₃ | H | Me | F | CH₂CH₂CH₂CH₂CH₂(3-CF₃-1,2,4-triazol-1-yl) | O |
| A-1484 | CH₂CF₃ | H | Me | F | CH₂CH₂CH₂(4-CF₃-thiazol-2-yl) | O |
| A-1485 | CH₂CF₃ | H | Me | F | CH₂CH_{2C}H₂CH₂(4-CF₃-thiazol-2-yl) | O |
| A-1486 | CH₂CF₃ | H | Me | F | CH₂CH₂CH₂CH₂CH₂(4-CF₃-thiazol-2-yl) | O |
| A-1487 | CH₂CF₃ | H | Me | F | CH₂CH₂CH₂(2-CF₃-pyridin-5-yl) | O |
| A-1488 | CH₂CF₃ | H | Me | F | CH₂CH₂CH₂CH₂(2-CF₃-pyridin-5-yl) | O |
| A-1489 | CH₂CF₃ | H | Me | F | CH₂CH₂CH₂CH₂CH₂(2-CF₃-pyridin-5-yl) | O |
| A-1490 | CH₂CF₃ | H | Me | F | CH₂(4-CF₃-pyrimidin-2-yl) | O |

**[Table 35]**

| Compound Number | R¹ | R² | R³ | R⁴ | R⁵ | A |
|---|---|---|---|---|---|---|
| A-1491 | CH₂CF₃ | H | Me | F | CH₂CH₂(4-CF₃-pyrimidin-2-yl) | O |
| A-1492 | CH₂CF₃ | H | Me | F | CH₂CH₂CH₂(4-CF₃-pyrimidin-2-yl) | O |
| A-1493 | CH₂CF₃ | H | Me | F | CH₂CH₂O(3-Cl-5-CF₃-pyridin-2-yl) | O |
| A-1494 | CH₂CF₃ | H | Me | F | CH₂CH₂CH₂O(3-Cl-5-CF₃-pyridin-2-yl) | O |
| A-1495 | CH₂CF₃ | H | Me | F | CH₂CH₂CH₂CH₂O(3-Cl-5-CF₃-pyridin-2-yl) | O |
| A-1496 | CH₂CF₃ | H | Me | F | CH₂CH₂CH₂CH₂CH₂O(3-Cl-5-CF₃-pyridin-2-yl) | O |
| A-1497 | CH₂CF₃ | H | Me | F | CH₂CH₂N(Phth) | O |
| A-1498 | CH₂CF₃ | H | Me | F | CH₂CH₂CH₂N(Phth) | O |
| A-1499 | CH₂CF₃ | H | Me | F | CH₂CH₂CH₂CH₂N(Phth) | O |
| A-1500 | CH₂CF₃ | H | Me | F | CH₂CH₂CH₂CH₂CH₂N(Phth) | O |
| A-1501 | CH₂CF₃ | H | Me | F | CH₂CH(Me)CH₂N(Phth) | O |
| A-1502 | CH₂CF₃ | H | Me | F | CH₂[1,3-dioxolan(2-CF₃)-2-yl] | O |
| A-1503 | CH₂CF₃ | H | Me | F | CH₂(azetidin-3-yl) | O |
| A-1504 | CH₂CF₃ | H | Me | F | CH₂(pyrrolidin-3-yl) | O |
| A-1505 | CH₂CF₃ | H | Me | F | CH₂(piperidin-3-yl) | O |
| A-1506 | CH₂CF₃ | H | Me | F | CH₂{azetidin[1-C(=O)O(*t*-Bu)]-3-yl} | O |
| A-1507 | CH₂CF₃ | H | Me | F | CH₂{pyrrolidin[1-C(=O)O(*t*-Bu)]-3-yl} | O |
| A-1508 | CH₂CF₃ | H | Me | F | CH₂{piperidin[1-C(=O)O(*t*-Bu)]-3-yl} | O |
| A-1509 | CH₂CF₃ | H | Me | F | CH₂{azetidin[1-C(=O)CF₃]-3-yl} | O |
| A-1510 | CH₂CF₃ | H | Me | F | CH₂{azetidin[1-S(=O)₂CF₃]-3-yl} | O |
| A-1511 | CH₂CF₃ | H | Me | F | CH₂{pyrrolidin[1-C(=O)CF₃]-3-yl} | O |
| A-1512 | CH₂CF₃ | H | Me | F | CH₂{pyrrolidin[1-S(=O)₂CF₃]-3-yl} | O |
| A-1513 | CH₂CF₃ | H | Me | F | CH₂{piperidin[1-C(=O)CF₃]-3-yl} | O |
| A-1514 | CH₂CF₃ | H | Me | F | CH₂{piperidin[1-S(=O)₂CF₃]-3-yl} | O |
| A-1515 | CH₂CF₃ | H | Me | F | CH₂CH₂CH₂CN | O |
| A-1516 | CH₂CF₃ | H | Me | F | CH₂CH₂CH₂CH₂CN | O |
| A-1517 | CH₂CF₃ | H | Me | F | CH₂CH₂CH₂CH₂CH₂CN | O |
| A-1518 | CH₂CF₃ | H | Me | F | CH₂CH₂CH₂CH₂CH₂CH₂CN | O |
| A-1519 | CH₂CF₃ | H | Me | F | CH₂CH₂CH₂C(=O)OH | O |
| A-1520 | CH₂CF₃ | H | Me | F | CH₂CH₂CH₂CH₂C(=O)OH | O |
| A-1521 | CH₂CF₃ | H | Me | F | CH₂CH₂CH₂CH₂CH₂C(=O)OH | O |
| A-1522 | CH₂CF₃ | H | Me | F | CH₂CH₂SCN | O |
| A-1523 | CH₂CF₃ | H | Me | F | CH₂CH₂CH₂SCN | O |
| A-1524 | CH₂CF₃ | H | Me | F | CH₂CH₂CH₂CH₂SCN | O |
| A-1525 | CH₂CF₃ | H | Me | F | CH₂CH₂CH₂CH₂CH₂SCN | O |
| A-1526 | CH₂CF₃ | H | Me | Cl | CH₂CH₂CH₂CH₂CH₂SCN | O |
| A-1527 | CH₂CF₃ | H | Me | F | CH₂CH₂CH₂CH₂CH₂CH₂SCN | O |
| A-1528 | CH₂CF₃ | H | Me | F | CH₂CH₂CH₂CH₂CH₂CH₂CH₂SCN | O |
| A-1529 | CH₂CF₃ | H | Me | F | CH₂CH₂CH₂CH₂CH₂CH₂CH₂CH₂SCN | O |
| A-1530 | CH₂CF₃ | H | Me | F | CH₂CH₂CH(Me)SCN | O |
| A-1531 | CH₂CF₃ | H | Me | F | CH₂CH(Me)CH₂SCN | O |
| A-1532 | CH₂CF₃ | H | Me | F | CH(Me)CH₂CH₂SCN | O |
| A-1533 | CH₂CF₃ | H | Me | F | CH₂CH₂CH₂CH(Me)SCN | O |
| A-1534 | CH₂CF₃ | H | Me | F | CH₂CH₂CH(Me)CH₂SCN | O |

**[Table 36]**

| Compound Number | R¹ | R² | R³ | R⁴ | R⁵ | A |
|---|---|---|---|---|---|---|
| A-1535 | CH₂CF₃ | H | Me | F | CH₂CH(Me)CH₂CH₂SCN | O |
| A-1536 | CH₂CF₃ | H | Me | F | CH(Me)CH₂CH₂CH₂SCN | O |
| A-1537 | CH₂CF₃ | H | Me | F | CH₂CH₂CH₂CH₂CH(Me)SCN | O |
| A-1538 | CH₂CF₃ | H | Me | F | CH₂CH₂CH₂CH(Me)CH₂SCN | O |
| A-1539 | CH₂CF₃ | H | Me | F | CH₂CH₂CH(Me)CH₂CH₂SCN | O |
| A-1540 | CH₂CF₃ | H | Me | F | CH₂CH(Me)CH₂CH₂CH₂SCN | O |
| A-1541 | CH₂CF₃ | H | Me | F | CH(Me)CH₂CH₂CH₂CH₂SCN | O |
| A-1542 | CH₂CF₃ | H | Me | F | CH₂CH₂ONH₂ | O |
| A-1543 | CH₂CF₃ | H | Me | F | CH₂CH₂CH₂ONH₂ | O |
| A-1544 | CH₂CF₃ | H | Me | F | CH₂CH₂CH₂CH₂ONH₂ | O |
| A-1545 | CH₂CF₃ | H | Me | F | CH₂CH₂CH₂CH₂CH₂ONH₂ | O |
| A-1546 | CH₂CF₃ | H | Me | F | CH₂CH₂CH₂CH₂CH₂CH₂ONH₂ | O |
| A-1547 | Et | H | Cl | F | Me | O |
| A-1548 | Et | H | Cl | F | Et | O |
| A-1549 | Et | H | Cl | F | *n*-Pr | O |
| A-1550 | Et | H | Cl | F | *i* -Pr | O |
| A-1551 | Et | H | Cl | F | *n* -Bu | O |
| A-1552 | Et | H | Cl | F | *n* -Pen | O |
| A-1553 | Et | H | Cl | F | *n -Hex* | O |
| A-1554 | Et | H | Cl | F | *n* -Heptyl | O |
| A-1555 | Et | H | Cl | F | *n* -Octyl | O |
| A-1556 | Et | H | Cl | F | *n* -Nonyl | O |
| A-1557 | Et | H | Cl | F | n -Decyl | O |
| A-1558 | Et | H | Cl | F | c-Pr | O |
| A-1559 | Et | H | Cl | F | c-Pen | O |
| A-1560 | Et | H | Cl | F | *c* -Hex | O |
| A-1561 | Et | H | Cl | F | CH₂C(Me)=CH₂ | O |
| A-1562 | Et | H | Cl | F | CH₂CH₂CH=CH₂ | O |
| A-1563 | Et | H | Cl | F | CH₂CH₂CH=C(CH₃)₂ | O |
| A-1564 | Et | H | Cl | F | CH₂CH₂CH₂CH=CH₂ | O |
| A-1565 | Et | H | Cl | F | CH₂CH₂CH₂CH=C(CH₃)₂ | O |
| A-1566 | Et | H | Cl | F | CH₂CH₂CH₂CH₂CH=CH₂ | O |
| A-1567 | Et | H | Cl | F | CH₂CH₂CH₂CH₂CH=C(CH₃)₂ | O |
| A-1568 | Et | H | Cl | F | CH₂CH₂CH=CH | O |
| A-1569 | Et | H | Cl | F | CH₂CH₂CH₂CH=CH | O |
| A-1570 | Et | H | Cl | F | CH₂CH₂CH₂CH₂CH≡CH | O |
| A-1571 | Et | H | Cl | F | CH₂CH₂CH₂Cl | O |
| A-1572 | Et | H | Cl | F | CH₂CH₂CH₂Br | O |
| A-1573 | Et | H | Cl | F | CH₂CH₂CH₂CH₂Cl | O |
| A-1574 | Et | H | Cl | F | CH₂CH₂CH₂CH₂Br | O |
| A-1575 | Et | H | Cl | F | CH₂CH₂CH₂CH₂CH₂Cl | O |
| A-1576 | Et | H | Cl | F | CH₂CH₂CH₂CH₂CH₂Br | O |
| A-1577 | Et | H | Cl | Cl | CH₂CH₂CH₂CH₂CH₂Br | O |
| A-1578 | Et | H | Cl | F | CH₂CH₂CH₂CH₂CH₂CH₂Cl | O |

**[Table 37]**

| Compound Number | R¹ | R² | R³ | R⁴ | R⁵ | A |
|---|---|---|---|---|---|---|
| A-1579 | Et | H | Cl | F | CH₂CH₂CH₂CH₂CH₂CH₂Br | O |
| A-1580 | Et | H | Cl | F | CH₂CH₂CH₂CH₂CH₂CH₂CH₂Cl | O |
| A-1581 | Et | H | Cl | F | CH₂CH₂CH₂CH₂CH₂CH₂CH₂Br | O |
| A-1582 | Et | H | Cl | F | CH₂CH₂CH₂CH₂CH₂CH₂CH₂CH₂Br | O |
| A-1583 | Et | H | Cl | F | CH₂CH(Me)CH₂Cl | O |
| A-1584 | Et | H | Cl | F | CH₂CH(Me)CH₂Br | O |
| A-1585 | Et | H | Cl | F | CH₂CH₂CH(Me)CH₂CH₂Cl | O |
| A-1586 | Et | H | Cl | F | CH₂CH₂CH(Me)CH₂CH₂Br | O |
| A-1587 | Et | H | Cl | F | CH₂CH₂CF=CF₂ | O |
| A-1588 | Et | H | Cl | F | CH₂CH=C(Cl)CF₃ | O |
| A-1589 | Et | H | Cl | F | CH₂CH₂CH₂CF=CF₂ | O |
| A-1590 | Et | H | Cl | F | CH₂CH₂CH₂CH₂CF=CF₂ | O |
| A-1591 | Et | H | Cl | F | CH₂CH₂CH₂CH₂CH₂CF=CF₂ | O |
| A-1592 | Et | H | Cl | F | CH₂(*t*-Bu) | O |
| A-1593 | Et | H | Cl | F | CH₂CH₂(*t*-Bu) | O |
| A-1594 | Et | H | Cl | F | CH₂CH₂CH₂(*t*-Bu) | O |
| A-1595 | Et | H | Cl | F | CH₂CH₂CH₂CH₂(*t*-Bu) | O |
| A-1596 | Et | H | Cl | F | CH₂CH₂CH₂CH₂CH₂(*t*-Bu) | O |
| A-1597 | Et | H | Cl | F | CH₂CH₂CH₂CH₂CH₂CH₂(*t*-Bu) | O |
| A-1598 | Et | H | Cl | F | CH₂CF₃ | O |
| A-1599 | Et | H | Cl | F | CH₂CH₂CF₃ | O |
| A-1600 | Et | H | Cl | F | CH₂CH₂CH₂CF₃ | O |
| A-1601 | Et | H | Cl | F | CH₂CH₂CH₂CH₂CF₃ | O |
| A-1602 | Et | H | Cl | F | CH₂CH₂CH₂CH₂CH₂CF₃ | O |
| A-1603 | Et | H | Cl | F | CH₂CH₂CH₂CH₂CH₂CH₂CF₃ | O |
| A-1604 | Et | H | Cl | F | CH₂CH₂CH₂CH₂CH₂CH₂CH₂CF₃ | O |
| A-1605 | Et | H | Cl | F | CH₂CF₂CF₃ | O |
| A-1606 | Et | H | Cl | F | CF₂CHFCF₃ | O |
| A-1607 | Et | H | Cl | F | CF₂CF₂CF₃ | O |
| A-1608 | Et | H | Cl | F | CH₂CF₂CF₂CF₃ | O |
| A-1609 | Et | H | Cl | F | CH₂CF₂CF₂CF₂CF₃ | O |
| A-1610 | Et | H | Cl | F | CH₂CF₂CF₂CF₂CHF₂ | O |
| A-1611 | Et | H | Cl | F | CH(CF₃)CF₃ | O |
| A-1612 | Et | H | Cl | F | CH₂CF(CF₃)CF₃ | O |
| A-1613 | Et | H | Cl | F | CH₂CF₂CF(CF₃)CF₃ | O |
| A-1614 | Et | H | Cl | F | CH₂CF(CF₃)CF₂CF₃ | O |
| A-1615 | Et | H | Cl | F | CH₂CF₂CF₂CF(CF₃)CF₃ | O |
| A-1616 | Et | H | Cl | F | CH₂CF₂CF(CF₃)CF₂CF₃ | O |
| A-1617 | Et | H | Cl | F | CH₂CF(CF₃)CF₂CF₂CF₃ | O |
| A-1618 | Et | H | Cl | F | CH₂(*c*-Pr) | O |
| A-1619 | Et | H | Cl | F | CH₂[*c*-Pr(1-Me)] | O |
| A-1620 | Et | H | Cl | F | CH₂[*c*-Pr(1-Ph)] | O |
| A-1621 | Et | H | Cl | F | CH₂[*c*-Pr(1-NH₂)] | O |
| A-1622 | Et | H | Cl | F | CH₂[*c* -Pr(1-NHC(=O)O(*t*-Bu)] | O |

**[Table 38]**

| Compound Number | R¹ | R² | R³ | R⁴ | R⁵ | A |
|---|---|---|---|---|---|---|
| A-1623 | Et | H | Cl | F | CH2{*c*-Pr[1-NHS(=O)₂CF₃]} | O |
| A-1624 | Et | H | Cl | F | CH₂{*c*-Pr[1-Ph(4-CF₃)]} | O |
| A-1625 | Et | H | Cl | F | CH₂{*c*-Pr[1-Ph(3,4,5-F₃)]} | O |
| A-1626 | Et | H | Cl | F | CH₂{*c*-Hex[4-(*t*-Bu)]} | O |
| A-1627 | Et | H | Cl | F | CH₂[*c*-Hex(4-CF₃)] | O |
| A-1628 | Et | H | Cl | F | CH₂CH₂(*c*-Pr) | O |
| A-1629 | Et | H | Cl | F | CH₂CH₂(*c*-Hex) | O |
| A-1630 | Et | H | Cl | F | CH₂CH₂[*c*-Hex(4-CF₃)] | O |
| A-1631 | Et | H | Cl | F | CH₂CH₂[*c*-Hex(4-SCF₃)] | O |
| A-1632 | Et | H | Cl | F | CH₂CH₂CH₂(*c*-Pr) | O |
| A-1633 | Et | H | Cl | F | CH₂CH₂CH₂(*c*-Hex) | O |
| A-1634 | Et | H | Cl | F | CH₂CH₂CH₂{*c*-Hex[4-(*t*-Bu)]) | O |
| A-1635 | Et | H | Cl | F | CH₂CH₂CH₂[*c*-Hex(4-CF₃)] | O |
| A-1636 | Et | H | Cl | F | CH₂CH₂CH₂[*c*-Hex(4-SCF₃)] | O |
| A-1637 | Et | H | Cl | F | CH₂CH₂CH₂CH₂(*c*-Pr) | O |
| A-1638 | Et | H | Cl | F | CH₂CH₂CH₂CH₂(*c*-Hex) | O |
| A-1639 | Et | H | Cl | F | CH₂CH₂CH₂CH₂[*c*-Hex(4-CF₃)] | O |
| A-1640 | Et | H | Cl | F | CH₂[*c*-Pr(2,2-F₂)] | O |
| A-1641 | Et | H | Cl | F | CH₂[*c*-Hex(4,4-F₂)] | O |
| A-1642 | Et | H | Cl | F | CH₂CH₂[*c*-Pr(2,2-F₂)] | O |
| A-1643 | Et | H | Cl | F | CH₂CH₂[*c*-Hex(4,4-F₂)] | O |
| A-1644 | Et | H | Cl | F | CH₂CH₂CH₂[*c*-Pr(2,2-F₂)] | O |
| A-1645 | Et | H | Cl | F | CH₂CH₂CH₂CH₂[*c*-Pr(2,2-F₂)] | O |
| A-1646 | Et | H | Cl | F | CH₂CH₂OH | O |
| A-1647 | Et | H | Cl | F | CH₂CH₂CH₂OH | O |
| A-1648 | Et | H | Cl | F | CH₂CH₂CH₂CH₂OH | O |
| A-1649 | Et | H | Cl | F | CH₂CH₂CH₂CH₂CH₂OH | O |
| A-1650 | Et | H | Cl | F | CH₂CH₂CH₂CH₂CH₂CH₂OH | O |
| A-1651 | Et | H | Cl | F | CH₂C(=O)Me | O |
| A-1652 | Et | H | Cl | F | CH₂CH(OH)Me | O |
| A-1653 | Et | H | Cl | F | CH₂CH(OH)CF₃ | O |
| A-1654 | Et | H | Cl | F | CH₂C(OH)₂CF₃ | O |
| A-1655 | Et | H | Cl | F | CH₂C(CF₃)=NOH | O |
| A-1656 | Et | H | Cl | F | CH₂C(CF₃)=NOMe | O |
| A-1657 | Et | H | Cl | F | CH₂CH(CF₃)NH₂ | O |
| A-1658 | Et | H | Cl | F | CH₂CH₂OMe | O |
| A-1659 | Et | H | Cl | F | CH₂CH₂CH₂OMe | O |
| A-1660 | Et | H | Cl | F | CH₂CH₂CH₂CH₂OMe | O |
| A-1661 | Et | H | Cl | F | CH₂CH₂CH₂CH₂CH₂OMe | O |
| A-1662 | Et | H | Cl | F | CH₂CH₂CH₂CH₂CH₂CH₂OMe | O |
| A-1663 | Et | H | Cl | F | CH₂CH₂OCHF₂ | O |
| A-1664 | Et | H | Cl | F | CH₂CH₂CH₂OCHF₂ | O |
| A-1665 | Et | H | Cl | F | CH₂CH₂CH₂CH₂OCHF₂ | O |
| A-1666 | Et | H | Cl | F | CH₂CH₂CH₂CH₂CH₂OCHF₂ | O |

**[Table 39]**

| Compound Number | R¹ | R² | R³ | R⁴ | R5 | A |
|---|---|---|---|---|---|---|
| A-1667 | Et | H | Cl | F | CH₂CH₂CH₂CH₂CH₂CH₂OCHF₂ | O |
| A-1668 | Et | H | Cl | F | CH₂CH₂OCH₂CF₃ | O |
| A-1669 | Et | H | Cl | F | CH₂CH₂CH₂OCH₂CF₃ | O |
| A-1670 | Et | H | Cl | F | CH₂CH₂CH₂CH₂OCH₂CF₃ | O |
| A-1671 | Et | H | Cl | F | CH₂CH₂CH₂CH₂CH₂OCH₂CF₃ | O |
| A-1672 | Et | H | Cl | F | CH₂CH₂CH₂CH₂CH₂CH₂OCH₂CF₃ | O |
| A-1673 | Et | H | Cl | F | CH₂CH₂CH₂CH₂CH₂CH₂CH₂OCH₂CF₃ | O |
| A-1674 | Et | H | Cl | F | CH₂CH₂OC(CF₃)₃ | O |
| A-1675 | Et | H | Cl | F | CH₂CH₂CH₂OC(CF₃)₃ | O |
| A-1676 | Et | H | Cl | F | CH₂CH₂CH₂CH₂OC(CF₃)₃ | O |
| A-1677 | Et | H | Cl | F | CH₂CH₂CH₂CH₂CH₂OC(CF₃)₃ | O |
| A-1678 | Et | H | Cl | F | CF₂CHFOCF₂CF₂CF₃ | O |
| A-1679 | Et | H | Cl | F | CH₂CH₂O(*c*-Pr) | O |
| A-1680 | Et | H | Cl | F | CH₂CH₂O(*c*-Pen) | O |
| A-1681 | Et | H | Cl | F | CH₂CH₂O(*c*-Hex) | O |
| A-1682 | Et | H | Cl | F | CH₂CH₂CH₂O(*c*-Pr) | O |
| A-1683 | Et | H | Cl | F | CH₂CH₂CH₂O(*c*-Pen) | O |
| A-1684 | Et | H | Cl | F | CH₂CH₂CH₂O(*c*-Hex) | O |
| A-1685 | Et | H | Cl | F | CH₂CH₂CH₂CH₂O(*c*-Pr) | O |
| A-1686 | Et | H | Cl | F | CH₂CH₂CH₂CH₂O(*c*-Pen) | O |
| A-1687 | Et | H | Cl | F | CH₂CH₂CH₂CH₂O(*c*-Hex) | O |
| A-1688 | Et | H | Cl | F | CH₂CH₂O[*c*-Pr(2,2-F₂)] | O |
| A-1689 | Et | H | Cl | F | CH₂CH₂CH₂O[*c*-Pr(2,2-F₂)] | O |
| A-1690 | Et | H | Cl | F | CH₂CH₂CH₂CH₂O[*c*-Pr(2,2-F₂)] | O |
| A-1691 | Et | H | Cl | F | CH₂CH₂OCH₂CH₂OCH₃ | O |
| A-1692 | Et | H | Cl | F | CH₂CH₂OCH₂CH₂OCH₂CF₃ | O |
| A-1693 | Et | H | Cl | F | CH₂CF₂OCF₂CF₂OCF₃ | O |
| A-1694 | Et | H | Cl | F | CF₂CHFOCF₂CF₂OCF₃ | O |
| A-1695 | Et | H | Cl | F | CF₂CHFOCF₂CF(CF₃)OCF₂CF₂CF₃ | O |
| A-1696 | Et | H | Cl | F | CH₂CH₂SH | O |
| A-1697 | Et | H | Cl | F | CH₂CH₂CH₂SH | O |
| A-1698 | Et | H | Cl | F | CH₂CH₂CH₂CH₂SH | O |
| A-1699 | Et | H | Cl | F | CH₂CH₂CH₂CH₂CH₂SH | O |
| A-1700 | Et | H | Cl | F | CH₂CH₂CH₂CH₂CH₂CH₂SH | O |
| A-1701 | Et | H | Cl | F | CH₂CH(SH)Me | O |
| A-1702 | Et | H | Cl | F | CH₂CH[SC(=O)Me]Me | O |
| A-1703 | Et | H | Cl | F | CH₂CH₂S(*t*-Bu) | O |
| A-1704 | Et | H | Cl | F | CH₂CH₂CH₂S(*t*-Bu) | O |
| A-1705 | Et | H | Cl | F | CH₂CH₂CH₂CH₂S(*t*-Bu) | O |
| A-1706 | Et | H | Cl | F | CH₂CH₂CH₂CH₂CH₂SCH₃ | O |
| A-1707 | Et | H | Cl | F | CH₂CH₂CH₂CH₂CH₂SCH(CH₃)₂ | O |
| A-1708 | Et | H | Cl | F | CH₂CH₂CH₂CH₂CH₂S(*t*-Bu) | O |
| A-1709 | Et | H | Cl | F | CH₂CH₂CH₂CH₂CH₂CH₂SCH(CH₃)₂ | O |
| A-1710 | Et | H | Cl | F | CH₂CH₂CH₂CH₂CH₂CH₂S(*t*-Bu) | O |

**[Table 40]**

| Compound Number | R¹ | R² | R³ | R⁴ | R⁵ | A |
|---|---|---|---|---|---|---|
| A-1711 | Et | H | Cl | F | CH₂CH₂CH₂CH₂CH₂CH₂CH₂S(*t*-Bu) | O |
| A-1712 | Et | H | Cl | F | CH₂CH₂SCHF₂ | O |
| A-1713 | Et | H | Cl | F | CH₂CH₂CH₂SCHF₂ | O |
| A-1714 | Et | H | Cl | F | CH₂CH₂CH₂CH₂SCHF₂ | O |
| A-1715 | Et | H | Cl | H | CH₂CH₂CH₂CH₂CH₂SCHF₂ | O |
| A-1716 | Et | H | Cl | F | CH₂CH₂CH₂CH₂CH₂SCHF₂ | O |
| A-1717 | Et | H | Cl | F | CH₂CH₂CH₂CH₂CH₂CH₂SCHF₂ | O |
| A-1718 | Et | H | Cl | F | CH₂CH₂CH₂CH₂CH₂CH₂CH₂SCHF₂ | O |
| A-1719 | Et | H | Cl | F | CH₂CH₂SCH₂CF₃ | O |
| A-1720 | Et | H | Cl | F | CH₂CH₂CH₂SCH₂CF₃ | O |
| A-1721 | Et | H | Cl | F | CH₂CH₂CH₂CH₂SCH₂CF₃ | O |
| A-1722 | Et | H | Cl | F | CH₂CH₂CH₂CH₂CH₂SCH₂CF₃ | O |
| A-1723 | Et | H | Cl | F | CH₂CH₂CH₂CH₂CH₂CH₂SCH₂CF₃ | O |
| A-1724 | Et | H | Cl | F | CH₂CH₂CH₂CH₂CH₂CH₂CH₂SCH₂CF₃ | O |
| A-1725 | Et | H | Cl | F | CH₂CH(Me)SCH₂CF₃ | O |
| A-1726 | Et | H | Cl | F | CH₂CH₂SCH(CF₃)₂ | O |
| A-1727 | Et | H | Cl | F | CH₂CH₂CH₂SCH(CF₃)₂ | O |
| A-1728 | Et | H | Cl | F | CH₂CH₂CH₂CH₂SCH(CF₃)₂ | O |
| A-1729 | Et | H | Cl | F | CH₂CH₂CH₂CH₂CH₂SCH(CF₃)₂ | O |
| A-1730 | Et | H | Cl | F | CH₂CH₂CH₂CH₂CH₂CH₂SCH(CF₃)₂ | O |
| A-1731 | Et | H | Cl | F | CH₂CH₂CH₂CH₂CH₂SCF₂CF₃ | O |
| A-1732 | Et | H | Cl | F | CH₂CH₂CH₂CH₂CH₂CH₂SCF₂CF₃ | O |
| A-1733 | Et | H | Cl | F | CH₂CH₂CH₂CH₂CH₂CH₂SCF(CF₃)₂ | O |
| A-1734 | Et | H | Cl | F | CH₂CH₂CH₂CH₂CH₂CH₂CH₂SCF(CF₃)₂ | O |
| A-1735 | Et | H | Cl | F | CH₂CH₂CH₂CH₂CH₂SCCl₃ | O |
| A-1736 | Et | H | Cl | F | CH₂CH₂CH₂CH₂CH₂CH₂SCCl₃ | O |
| A-1737 | Et | H | Cl | F | CH₂CH₂SCH₂CH=CH₂ | O |
| A-1738 | Et | H | Cl | F | CH₂CH₂CH₂SCH₂CH=CH₂ | O |
| A-1739 | Et | H | Cl | F | CH₂CH₂CH₂CH₂SCH₂CH=CH₂ | O |
| A-1740 | Et | H | Cl | F | CH₂CH₂SCF=CFCF₃ | O |
| A-1741 | Et | H | Cl | F | CH₂CH₂CH₂SCF=CFCF₃ | O |
| A-1742 | Et | H | Cl | F | CH₂CH₂CH₂CH₂SCF=CFCF₃ | O |
| A-1743 | Et | H | Cl | F | CH₂CH₂CH₂S(*c*-Pr) | O |
| A-1744 | Et | H | Cl | F | CH₂CH₂CH₂S(*c*-Hex) | O |
| A-1745 | Et | H | Cl | F | CH₂CH₂CH₂CH₂S(*c*-Pr) | O |
| A-1746 | Et | H | Cl | F | CH₂CH₂CH₂CH₂S(*c*-Hex) | O |
| A-1747 | Et | H | Cl | F | CH₂CH₂CH₂CH₂CH₂S(*c*-Pr) | O |
| A-1748 | Et | H | Cl | F | CH₂CH₂CH₂CH₂CH₂S(*c*-Hex) | O |
| A-1749 | Et | H | Cl | F | CH₂CH₂CH₂S[*c*-Hex(4,4-F₂)] | O |
| A-1750 | Et | H | Cl | F | CH₂CH₂CH₂CH₂S[*c*-Hex(4,4-F*2*)] | O |
| A-1751 | Et | H | Cl | F | CH₂CH₂CH₂CH₂CH₂S[*c*-Hex(4,4-F₂)] | O |
| A-1752 | Et | H | Cl | F | CH₂CH₂CH₂SCH₂(*c*-Pr) | O |
| A-1753 | Et | H | Cl | F | CH₂CH₂CH₂SCH₂(*c*-Hex) | O |
| A-1754 | Et | H | Cl | F | CH₂CH₂CH₂CH₂SCH₂(*c*-Pr) | O |

**[Table 41]**

| Compound Number | R¹ | R² | R³ | R⁴ | R⁵ | A |
|---|---|---|---|---|---|---|
| A-1755 | Et | H | Cl | F | CH₂CH₂CH₂CH₂SCH₂(*c*-Hex) | O |
| A-1756 | Et | H | Cl | F | CH₂CH₂CH₂CH₂CH₂SCH₂(*c*-Pr) | O |
| A-1757 | Et | H | Cl | F | CH₂CH₂CH₂CH₂CH₂SCH₂(*c*-Hex) | O |
| A-1758 | Et | H | Cl | F | CH₂CH₂CH₂SCH₂[*c*-Hex(4,4-F₂)] | O |
| A-1759 | Et | H | Cl | F | CH₂CH₂CH₂CH₂SCH₂[*c*-Hex(4,4-F₂)] | O |
| A-1760 | Et | H | Cl | F | C H₂CH₂CH₂CH₂CH₂SCH₂[*c*-Hex(4,4-F₂)] | O |
| A-1761 | Et | H | Cl | F | CH₂CH₂CH₂SCH₂SiMe₃ | O |
| A-1762 | Et | H | Cl | F | CH₂CH₂CH₂CH₂SCH₂SiMe₃ | O |
| A-1763 | Et | H | Cl | F | CH₂CH₂CH₂CH₂CH₂SCH₂SiMe₃ | O |
| A-1764 | Et | H | Cl | F | CH₂CH₂OCH₂CH₂SCH₃ | O |
| A-1765 | Et | H | Cl | F | CH₂CH₂OCH₂CH₂SCHF₂ | O |
| A-1766 | Et | H | Cl | F | CH₂CH₂OCH₂CH₂SCF₃ | O |
| A-1767 | Et | H | Cl | F | CH₂CH₂OCH₂CH₂SCH₂CF₃ | O |
| A-1768 | Et | H | Cl | F | CH₂CH₂CH₂S(=O)(*t*-Bu) | O |
| A-1769 | Et | H | Cl | F | CH₂CH₂CH₂CH₂S(=O)(*t*-Bu) | O |
| A-1770 | Et | H | Cl | F | CH₂CH₂CH₂CH₂CH₂CH₂S(=O)(*t*-Bu) | O |
| A-1771 | Et | H | Cl | F | CH₂CH₂S(=O)CHF₂ | O |
| A-1772 | Et | H | Cl | F | CH₂CH₂CH₂S(=O)CHF₂ | O |
| A-1773 | Et | H | Cl | F | CH₂CH₂CH₂CH₂S(=O)CHF₂ | O |
| A-1774 | Et | H | Cl | F | CH₂CH₂CH₂CH₂CH₂S(=O)CHF₂ | O |
| A-1775 | Et | H | Cl | F | CH₂CH₂CH₂CH₂CH₂CH₂S(=O)CHF₂ | O |
| A-1776 | Et | H | Cl | F | CH₂CH₂CH₂CH₂CH₂CH₂CH₂S(=O)CHF₂ | O |
| A-1777 | Et | H | Cl | F | CH₂CH₂S(=O)CF₃ | O |
| A-1778 | Et | H | Cl | F | CH₂CH₂CH₂S(=O)CF₃ | O |
| A-1779 | Et | H | Cl | F | CH₂CH₂CH₂CH₂S(=O)CF₃ | O |
| A-1780 | Et | H | Cl | F | CH₂CH₂CH₂CH₂CH₂S(=O)CF₃ | O |
| A-1781 | Et | H | Cl | F | CH₂CH₂CH₂CH₂CH₂CH₂S(=O)CF₃ | O |
| A-1782 | Et | H | Cl | F | CH₂CH₂CH₂CH₂CH₂CH₂CH₂S(=O)CF₃ | O |
| A-1783 | Et | H | Cl | F | CH₂CH₂S(=O)CH₂CF₃ | O |
| A-1784 | Et | H | Cl | F | CH₂CH₂CH₂S(=O)CH₂CF₃ | O |
| A-1785 | Et | H | Cl | F | CH₂CH₂CH₂CH₂S(=O)CH₂CF₃ | O |
| A-1786 | Et | H | Cl | F | CH₂CH₂CH₂CH₂CH₂S(=O)CH₂CF₃ | O |
| A-1787 | Et | H | Cl | F | CH₂CH₂CH₂CH₂CH₂CH₂S(=O)CH₂CF₃ | O |
| A-1788 | Et | H | Cl | F | CH₂CH₂CH₂CH₂CH₂CH₂CH₂S(=O)CH₂CF₃ | O |
| A-1789 | Et | H | Cl | F | CH₂CH(Me)S(=O)CH₂CF₃ | O |
| A-1790 | Et | H | Cl | F | CH₂CH₂CH₂S(=O)(*c*-Pr) | O |
| A-1791 | Et | H | Cl | F | CH₂CH₂CH₂CH₂S(=O)(*c*-Pr) | O |
| A-1792 | Et | H | Cl | F | CH₂CH₂CH₂CH₂S(=O)(*c*-Pen) | O |
| A-1793 | Et | H | Cl | F | CH₂CH₂CH₂CH₂S(=O)(*c*-Hex) | O |
| A-1794 | Et | H | Cl | F | CH₂CH₂CH₂CH₂CH₂S(=O)(*c*-Pr) | O |
| A-1795 | Et | H | Cl | F | CH₂CH₂CH₂CH₂CH₂S(=O)(*c*-Hex) | O |
| A-1796 | Et | H | Cl | F | CH₂CH₂CH₂S(=O)[*c*-Hex(4,4-F₂)] | O |
| A-1797 | Et | H | Cl | F | CH₂CH₂CH₂CH₂S(=O)[*c*-Hex(4,4-F₂)] | O |
| A-1798 | Et | H | Cl | F | CH₂CH₂CH₂CH₂CH₂S(=O)[*c*-Hex(4,4-F₂)] | O |

**[Table 42]**

| Compound Number | R¹ | R² | R³ | R⁴ | R⁵ | A |
|---|---|---|---|---|---|---|
| A-1799 | Et | H | Cl | F | CH₂CH₂CH₂CH₂S(=O)₂Me | O |
| A-1800 | Et | H | Cl | F | CH₂CH₂CH₂CH₂CH₂S(=O)₂Me | O |
| A-1801 | Et | H | Cl | F | CH₂CH₂CH₂CH₂CH₂S(=O)₂(*t*-Bu) | O |
| A-1802 | Et | H | Cl | F | CH₂CH₂S(=O)₂CHF₂ | O |
| A-1803 | Et | H | Cl | F | CH₂CH₂CH₂S(=O)₂CHF₂ | O |
| A-1804 | Et | H | Cl | F | CH₂CH₂CH₂CH₂S(=O)₂CHF₂ | O |
| A-1805 | Et | H | Cl | F | CH₂CH₂CH₂CH₂CH₂S(=O)₂CHF₂ | O |
| A-1806 | Et | H | Cl | F | CH₂CH₂CH₂CH₂CH₂CH₂S(=O)₂CHF₂ | O |
| A-1807 | Et | H | Cl | F | CH₂CH₂S(=O)₂CF₃ | O |
| A-1808 | Et | H | Cl | F | CH₂CH₂CH₂S(=O)₂CF₃ | O |
| A-1809 | Et | H | Cl | F | CH₂CH₂CH₂CH₂S(=O)₂CF₃ | O |
| A-1810 | Et | H | Cl | F | CH₂CH₂CH₂CH₂CH₂S(=O)₂CF₃ | O |
| A-1811 | Et | H | Cl | F | CH₂CH₂CH₂CH₂CH₂CH₂S(=O)₂CF₃ | O |
| A-1812 | Et | H | Cl | F | CH₂CH₂CH_{2C}H₂CH₂CH₂CH₂S(=O)₂CF₃ | O |
| A-1813 | Et | H | Cl | F | CH₂CH₂CH₂CH₂CH₂CH₂CH₂CH₂S(=O)₂CF₃ | O |
| A-1814 | Et | H | Cl | F | CH₂CH₂S(=O)₂CH₂CF₃ | O |
| A-1815 | Et | H | Cl | F | CH₂CH₂CH₂S(=O)₂CH₂CF₃ | O |
| A-1816 | Et | H | Cl | F | CH₂CH₂CH₂CH₂S(=O)₂CH₂CF₃ | O |
| A-1817 | Et | H | Cl | F | CH₂CH₂CH₂CH₂CH₂S(=O)₂CH₂CF₃ | O |
| A-1818 | Et | H | Cl | F | CH₂CH₂CH₂CH₂CH₂CH₂S(=O)₂CH₂CF₃ | O |
| A-1819 | Et | H | Cl | F | CH₂CH(Me)S(=O)₂CH₂CF₃ | O |
| A-1820 | Et | H | Cl | F | CH₂CH₂CH₂S(=O)₂(*c*-Pr) | O |
| A-1821 | Et | H | Cl | F | CH₂CH₂CH₂CH₂S(=O)₂(*c*-Pr) | O |
| A-1822 | Et | H | Cl | F | CH₂CH₂CH₂CH₂CH₂S(=O)₂(*c*-Pr) | O |
| A-1823 | Et | H | Cl | F | CH₂CH₂CH₂S(=O)₂[*c*-Hex(4,4-F₂)] | O |
| A-1824 | Et | H | Cl | F | CH₂CH₂CH₂CH₂S(=O)₂[*c*-Hex(4,4-F₂)] | O |
| A-1825 | Et | H | Cl | F | CH₂CH₂CH₂CH₂CH₂S(=O)₂[*c*-Hex(4,4-F₂)] | O |
| A-1826 | Et | H | Cl | F | CH₂CH₂CH₂C(=O)H | O |
| A-1827 | Et | H | Cl | F | CH₂CH₂CH₂CH₂C(=O)H | O |
| A-1828 | Et | H | Cl | F | CH₂CH₂CH₂CH₂CH₂C(=O)H | O |
| A-1829 | Et | H | Cl | F | CH₂CH₂CH₂C(=O)(*t*-Bu) | O |
| A-1830 | Et | H | Cl | F | CH₂CH₂CH₂CH₂C(=O)(*t*-Bu) | O |
| A-1831 | Et | H | Cl | F | CH₂CH₂CH₂CH₂CH₂C(=O)(*t*-Bu) | O |
| A-1832 | Et | H | Cl | F | CH₂CH₂CH₂C(=O)CF₃ | O |
| A-1833 | Et | H | Cl | F | CH₂CH₂CH₂CH₂C(=O)CF₃ | O |
| A-1834 | Et | H | Cl | F | CH₂CH₂CH₂CH₂CH₂C(=O)CF₃ | O |
| A-1835 | Et | H | Cl | F | CH₂C(=O)Ph | O |
| A-1836 | Et | H | Cl | F | CH₂C(=O)Ph(4-Cl) | O |
| A-1837 | Et | H | Cl | F | CH₂C(=O)Ph(4-CF₃) | O |
| A-1838 | Et | H | Cl | F | CH₂CH₂C(=O)Ph(4-CF₃) | O |
| A-1839 | Et | H | Cl | F | CH₂CH₂CH₂C(=O)Ph(4-CF₃) | O |
| A-1840 | Et | H | Cl | F | CH₂CH₂CH₂CH₂C(=O)Ph(4-CF₃) | O |
| A-1841 | Et | H | Cl | F | CH₂CH₂CH₂CH₂CH₂C(=O)Ph(4-CF₃) | O |
| A-1842 | Et | H | Cl | F | CH₂C(=O)OEt | O |

**[Table 43]**

| Compound Number | R¹ | R² | R³ | R⁴ | R⁵ | A |
|---|---|---|---|---|---|---|
| A-1843 | Et | H | Cl | F | CH₂CH₂C(=O)O(*t*-Bu) | O |
| A-1844 | Et | H | Cl | F | CH₂CH₂CH₂C(=O)OEt | O |
| A-1845 | Et | H | Cl | F | CH₂CH₂CH₂C(=O)O(*t*-Bu) | O |
| A-1846 | Et | H | Cl | F | CH₂CH₂CH₂CH₂C(=O)O(*t*-Bu) | O |
| A-1847 | Et | H | Cl | F | CH₂CH₂CH₂CH₂CH₂C(=O)O(*t*-Bu) | O |
| A-1848 | Et | H | Cl | F | CH₂CH₂CH₂CH₂CH₂CH₂C(=O)O(*t*-Bu) | O |
| A-1849 | Et | H | Cl | F | CH₂CH₂CH₂CH₂CH₂CH₂CH₂C(=O)OEt | O |
| A-1850 | Et | H | Cl | F | CH₂CH₂C(=O)OCH₂CF₃ | O |
| A-1851 | Et | H | Cl | F | CH₂CH₂CH₂C(=O)OCH₂CF₃ | O |
| A-1852 | Et | H | Cl | F | CH₂CH₂CH₂CH₂C(=O)OCH₂CF₃ | O |
| A-1853 | Et | H | Cl | F | CH₂CH₂CH₂CH₂CH₂C(=O)OCH₂CF₃ | O |
| A-1854 | Et | H | Cl | F | CH₂CH₂C(=O)NH(*t*-Bu) | O |
| A-1855 | Et | H | Cl | F | CH₂CH₂CH₂C(=O)NH(*t*-Bu) | O |
| A-1856 | Et | H | Cl | F | CH₂CH₂CH₂C(=O)NH(*t*-Pen) | O |
| A-1857 | Et | H | Cl | F | CH₂CH₂CH₂CH₂C(=O)NH(*t*-Bu) | O |
| A-1858 | Et | H | Cl | F | CH₂CH₂C(=O)NHCH₂CF₃ | O |
| A-1859 | Et | H | Cl | F | CH₂CH₂CH₂C(=O)NHCH₂CF₃ | O |
| A-1860 | Et | H | Cl | F | CH₂CH₂CH₂CH₂C(=O)NHCH₂CF₃ | O |
| A-1861 | Et | H | Cl | F | CH₂CH₂SC(=O)N(Me)₂ | O |
| A-1862 | Et | H | Cl | F | CH₂CH₂CH₂SC(=O)N(Me)₂ | O |
| A-1863 | Et | H | Cl | F | CH₂CH₂CH₂SC(=O)NH(*t*-Bu) | O |
| A-1864 | Et | H | Cl | F | CH₂CH₂CH₂CH₂CH₂SC(=O)NH(*t*-Bu) | O |
| A-1865 | Et | H | Cl | F | CH₂CH₂SC(=O)NHCH₂CF₃ | O |
| A-1866 | Et | H | Cl | F | CH₂CH₂CH₂SC(=O)NHCH₂CF₃ | O |
| A-1867 | Et | H | Cl | F | CH₂CH₂CH₂CH₂SC(=O)NHCH₂CF₃ | O |
| A-1868 | Et | H | Cl | F | CH₂CH₂CH₂OC(=O)H | O |
| A-1869 | Et | H | Cl | F | CH₂CH₂CH₂CH₂OC(=O)H | O |
| A-1870 | Et | H | Cl | F | CH₂CH₂CH₂CH₂CH₂OC(=O)H | O |
| A-1871 | Et | H | Cl | F | CH₂CH₂CH₂OC(=O)Me | O |
| A-1872 | Et | H | Cl | F | CH₂CH₂CH₂CH₂OC(=O)Me | O |
| A-1873 | Et | H | Cl | F | CH₂CH₂CH₂CH₂CH₂OC(=O)Me | O |
| A-1874 | Et | H | Cl | F | CH₂CH₂OC(=O)CF₃ | O |
| A-1875 | Et | H | Cl | F | CH₂CH₂CH₂OC(=O)CF₃ | O |
| A-1876 | Et | H | Cl | F | CH₂CH₂CH₂CH₂OC(=O)CF₃ | O |
| A-1877 | Et | H | Cl | F | CH₂CH₂CH₂CH₂CH₂OC(=O)Ph(4-CF₃) | O |
| A-1878 | Et | H | Cl | F | CH₂CH₂OC(=O)Ph | O |
| A-1879 | Et | H | Cl | F | CH₂CH₂OC(=O)Ph(4-CF₃) | O |
| A-1880 | Et | H | Cl | F | CH₂CH₂CH₂OC(=O)Ph(4-CF₃) | O |
| A-1881 | Et | H | Cl | F | CH₂CH₂CH₂CH₂OC(=O)Ph(4-CF₃) | O |
| A-1882 | Et | H | Cl | F | CH₂CH₂CH₂CH₂CH₂OC(=O)Ph(4-CF₃) | O |
| A-1883 | Et | H | Cl | F | CH₂CH₂OS(=O)Me | O |
| A-1884 | Et | H | Cl | F | CH₂CH₂CH₂OS(=O)Me | O |
| A-1885 | Et | H | Cl | F | CH₂CH₂CH₂CH₂OS(=O)Me | O |
| A-1886 | Et | H | Cl | F | CH₂CH₂CH₂CH₂CH₂OS(=O)Me | O |

**[Table 44]**

| Compound Number | R¹ | R² | R³ | R⁴ | R⁵ | A |
|---|---|---|---|---|---|---|
| A-1887 | Et | H | Cl | F | CH₂CH₂CH₂CH₂CH₂CH₂OS(=O)Me | O |
| A-1888 | Et | H | Cl | F | CH₂CH₂OS(=O)CF₃ | O |
| A-1889 | Et | H | Cl | F | CH₂CH₂CH₂OS(=O)CF₃ | O |
| A-1890 | Et | H | Cl | F | CH₂CH₂CH₂CH₂OS(=O)CF₃ | O |
| A-1891 | Et | H | Cl | F | CH₂CH₂CH₂CH₂CH₂OS(=O)CF₃ | O |
| A-1892 | Et | H | Cl | F | CH₂CH₂CH₂CH₂CH₂CH₂OS(=O)CF₃ | O |
| A-1893 | Et | H | Cl | F | CH₂CH₂OS(=O)Ph | O |
| A-1894 | Et | H | Cl | F | CH₂CH₂OS(=O)Ph(4-Me) | O |
| A-1895 | Et | H | Cl | F | CH₂CH₂CH₂OS(=O)Ph | O |
| A-1896 | Et | H | Cl | F | CH₂CH₂CH₂OS(=O)Ph(4-Me) | O |
| A-1897 | Et | H | Cl | F | CH₂CH₂OS(=O)₂Me | O |
| A-1898 | Et | H | Cl | F | CH₂CH₂CH₂OS(=O)₂Me | O |
| A-1899 | Et | H | Cl | F | CH₂CH₂CH₂OS(=O)₂CF₃ | O |
| A-1900 | Et | H | Cl | F | CH₂CH₂CH₂CH₂OS(=O)₂CF₃ | O |
| A-1901 | Et | H | Cl | F | CH₂CH₂CH₂CH₂CH₂OS(=O)₂CF₃ | O |
| A-1902 | Et | H | Cl | F | CH₂CH₂CH₂CH₂CH₂OS(=O)₂CF₂CF₂CF₂CF₃ | O |
| A-1903 | Et | H | Cl | F | CH₂CH₂OS(=O)₂Ph | O |
| A-1904 | Et | H | Cl | F | CH₂CH₂OS(=O)₂Ph(4-Me) | O |
| A-1905 | Et | H | Cl | F | CH₂CH₂CH₂OS(=O)₂Ph | O |
| A-1906 | Et | H | Cl | F | CH₂CH₂CH₂OS(=O)₂Ph(4-Me) | O |
| A-1907 | Et | H | Cl | F | CH₂CH₂NH₂ | O |
| A-1908 | Et | H | Cl | F | CH₂CH₂CH₂NH₂ | O |
| A-1909 | Et | H | Cl | F | CH₂CH₂CH₂CH₂NH₂ | O |
| A-1910 | Et | H | Cl | F | CH₂CH₂CH₂CH₂CH₂NH₂ | O |
| A-1911 | Et | H | Cl | F | CH₂CH₂CH₂CH₂CH₂CH₂NH₂ | O |
| A-1912 | Et | H | Cl | F | CH₂CH₂CH₂CH₂CH₂CH₂CH₂NH₂ | O |
| A-1913 | Et | H | Cl | F | CH₂CH(Me)NH₂ | O |
| A-1914 | Et | H | Cl | F | CH₂C*H(Me)NH₂ : (R) | O |
| A-1915 | Et | H | Cl | F | CH₂C*H(Me)NH₂ : (S) | O |
| A-1916 | Et | H | Cl | F | CH₂CH(Et)NH₂ | O |
| A-1917 | Et | H | Cl | F | CH_{2CH}(*i*-Pr)NH₂ | O |
| A-1918 | Et | H | Cl | F | CH(Me)CH₂NH₂ | O |
| A-1919 | Et | H | Cl | F | CH(Et)CH₂NH₂ | O |
| A-1920 | Et | H | Cl | F | CH(*i*-Pr)CH₂NH₂ | O |
| A-1921 | Et | H | Cl | F | CH₂CH₂CH(Me)NH₂ | O |
| A-1922 | Et | H | Cl | F | CH₂CH(Me)CH₂NH₂ | O |
| A-1923 | Et | H | Cl | F | CH(Me)CH₂CH₂NH₂ | O |
| A-1924 | Et | H | Cl | F | CH₂CH(Me)NH(*c*-Pr) | O |
| A-1925 | Et | H | Cl | F | CH₂CH(Me)NHOMe | O |
| A-1926 | Et | H | Cl | F | CH₂CH(Me)N(OMe)S(=O)₂Ph(2-NO₂) | O |
| A-1927 | Et | H | Cl | F | CH₂CH(Me)N(OMe)S(=O)₂Ph(4-NO₂) | O |
| A-1928 | Et | H | Cl | F | CH₂CH₂N(Me)(*t*-Bu) | O |
| A-1929 | Et | H | Cl | F | CH₂CH₂CH₂N(Me)(*t*-Bu) | O |
| A-1930 | Et | H | Cl | F | CH₂CH₂CH₂CH₂N(Me)(*t*-Bu) | O |

**[Table 45]**

| Compound Number | R¹ | R² | R³ | R⁴ | R⁵ | A |
|---|---|---|---|---|---|---|
| A-1931 | Et | H | Cl | F | CH₂CH₂NHCH₂CF₃ | O |
| A-1932 | Et | H | Cl | F | CH₂CH₂CH₂NHCH₂CF₃ | O |
| A-1933 | Et | H | Cl | F | CH₂CH₂CH₂CH₂NHCH₂CF₃ | O |
| A-1934 | Et | H | Cl | F | CH₂CH₂NHC(=O)C(Me)(CF₃)₂ | O |
| A-1935 | Et | H | Cl | F | CH₂CH₂NHC(=O)Me | O |
| A-1936 | Et | H | Cl | F | CH₂CH₂NHC(=O)(*t*-Bu) | O |
| A-1937 | Et | H | Cl | F | CH₂CH₂CH₂CH₂NHC(=O)CH(CH₃)₂ | O |
| A-1938 | Et | H | Cl | F | CH₂CH₂CH₂CH₂NHC(=O)(*t*-Bu) | O |
| A-1939 | Et | H | Cl | F | CH₂CH₂CH₂CH₂NHC(=O)CH₂(*t*-Bu) | O |
| A-1940 | Et | H | Cl | F | CH₂CH₂NHC(=O)CF₃ | O |
| A-1941 | Et | H | Cl | F | CH₂CH₂NHC(=O)CH₂CF₃ | O |
| A-1942 | Et | H | Cl | F | CH₂CH₂NHC(=O)CF₂CF₃ | O |
| A-1943 | Et | H | Cl | F | CH₂CH₂CH₂NHC(=O)CF₃ | O |
| A-1944 | Et | H | Cl | F | CH₂CH₂CH₂NHC(=O)CH₂CF₃ | O |
| A-1945 | Et | H | Cl | F | CH₂CH₂CH₂NHC(=O)CF₂CF₃ | O |
| A-1946 | Et | H | Cl | F | CH₂CH₂CH₂CH₂NHC(=O)CF₃ | O |
| A-1947 | Et | H | Cl | F | CH₂CH₂CH₂CH₂NHC(=O)CH₂CF₃ | O |
| A-1948 | Et | H | Cl | F | CH₂CH₂CH₂CH₂NHC(=O)CF₂CF₃ | O |
| A-1949 | Et | H | Cl | F | CH₂CH₂CH₂CH₂NHC(=O)CF(CF₃)₂ | O |
| A-1950 | Et | H | Cl | F | CH₂CH₂CH₂CH₂NHC(=O)CCl₃ | O |
| A-1951 | Et | H | Cl | F | CH₂CH(Me)NHC(=O)CF₃ | O |
| A-1952 | Et | H | Cl | F | CH₂CH₂NHC(=O)Ph | O |
| A-1953 | Et | H | Cl | F | CH₂CH₂NHC(=O)Ph(4-CF₃) | O |
| A-1954 | Et | H | Cl | F | CH₂CH₂CH₂NHC(=O)Ph(4-CF₃) | O |
| A-1955 | Et | H | Cl | F | CH₂CH₂CH₂CH₂NHC(=O)Ph(4-CF₃) | O |
| A-1956 | Et | H | Cl | F | CH₂CH₂NHC(=O)O(*t*-Bu) | O |
| A-1957 | Et | H | Cl | F | CH₂CH₂CH₂NHC(=O)O(*t*-Bu) | O |
| A-1958 | Et | H | Cl | F | CH₂CH₂CH₂CH₂NHC(=O)O(*t*-Bu) | O |
| A-1959 | Et | H | Cl | F | CH₂CH₂CH₂CH₂NHC(=O)OCH(CH₃)₂ | O |
| A-1960 | Et | H | Cl | F | CH₂CH(Me)NHC(=O)O(*t*-Bu) | O |
| A-1961 | Et | H | Cl | F | CH₂C*H(Me)NHC(=O)O(*t*-Bu) : (R) | O |
| A-1962 | Et | H | Cl | F | CH₂C*H(Me)NHC(=O)O(*t*-Bu) : (S) | O |
| A-1963 | Et | H | Cl | F | CH₂CH(Et)NHC(=O)O(*t*-Bu) | O |
| A-1964 | Et | H | Cl | F | CH₂CH(*i*-Pr)NHC(=O)O(t-Bu) | O |
| A-1965 | Et | H | Cl | F | CH(Me)CH₂NHC(=O)O(*t*-Bu) | O |
| A-1966 | Et | H | Cl | F | CH(Et)CH₂NHC(=O)O(*t*-Bu) | O |
| A-1967 | Et | H | Cl | F | CH(*i*-Pr)CH₂NHC(=O)O(t-Bu) | O |
| A-1968 | Et | H | Cl | F | CH₂CH(Me)CH₂NHC(=O)O(*t*-Bu) | O |
| A-1969 | Et | H | Cl | F | CH₂CH₂NHC(=O)OC(Me)₂CF₃ | O |
| A-1970 | Et | H | Cl | F | CH₂CH₂CH₂NHC(=O)OCH₂CF₃ | O |
| A-1971 | Et | H | Cl | F | CH₂CH₂CH₂NHC(=O)OCH₂CH₂CF₃ | O |
| A-1972 | Et | H | Cl | F | CH₂CH₂CH₂CH₂NHC(=O)OCH₂CCl₃ | O |
| A-1973 | Et | H | Cl | F | CH₂CH₂CH₂CH₂NHC(=O)OCH₂CF₃ | O |
| A-1974 | Et | H | Cl | F | CH₂CH₂NHC(=O)NH(*t*-Bu) | O |

**[Table 46]**

| Compound Number | R¹ | R² | R³ | R⁴ | R⁵ | A |
|---|---|---|---|---|---|---|
| A-1975 | Et | H | Cl | F | CH₂CH₂CH₂NHC(=O)NH(*t*-Bu) | O |
| A-1976 | Et | H | Cl | F | CH₂CH₂CH₂CH₂NHC(=O)NHEt | O |
| A-1977 | Et | H | Cl | F | CH₂CH₂CH₂CH₂NHC(=O)NH(*t*-Bu) | O |
| A-1978 | Et | H | Cl | F | CH₂CH₂CH₂NHC(=O)NHCH₂CCl₃ | O |
| A-1979 | Et | H | Cl | F | CH₂CH₂CH₂NHC(=O)NHCH₂CH₂F | O |
| A-1980 | Et | H | Cl | F | CH₂CH₂CH₂NHC(=O)NHCH₂CF₃ | O |
| A-1981 | Et | H | Cl | F | CH₂CH₂CH₂CH₂NHC(=O)NHCH₂CCl₃ | O |
| A-1982 | Et | H | Cl | F | CH₂CH₂CH₂CH₂NHC(=O)NHCH₂CH₂F | O |
| A-1983 | Et | H | Cl | F | CH₂CH₂CH₂CH₂NHC(=O)NHCH₂CF₃ | O |
| A-1984 | Et | H | Cl | F | CH₂CH₂NHS(=O)Me | O |
| A-1985 | Et | H | Cl | F | CH₂CH₂CH₂NHS(=O)Me | O |
| A-1986 | Et | H | Cl | F | CH₂CH₂CH₂CH₂NHS(=O)Me | O |
| A-1987 | Et | H | Cl | F | CH₂CH₂CH₂CH₂NHS(=O)CH(CH₃)₂ | O |
| A-1988 | Et | H | Cl | F | CH₂CH₂CH₂CH₂CH₂NHS(=O)Me | O |
| A-1989 | Et | H | Cl | F | CH₂CH₂NHS(=O)CHF₂ | O |
| A-1990 | Et | H | Cl | F | CH₂CH₂CH₂NHS(=O)CHF₂ | O |
| A-1991 | Et | H | Cl | F | CH₂CH₂CH₂CH₂NHS(=O)CHF₂ | O |
| A-1992 | Et | H | Cl | F | CH₂CH₂CH₂CH₂CH₂NHS(=O)CHF₂ | O |
| A-1993 | Et | H | Cl | F | CH₂CH(Me)NHS(=O)CHF₂ | O |
| A-1994 | Et | H | Cl | F | CH(Me)CH₂NHS(=O)CHF₂ | O |
| A-1995 | Et | H | Cl | F | CH₂CH₂CH(Me)NHS(=O)CHF₂ | O |
| A-1996 | Et | H | Cl | F | CH₂CH(Me)CH₂NHS(=O)CHF₂ | O |
| A-1997 | Et | H | Cl | F | CH(Me)CH₂CH₂NHS(=O)CHF₂ | O |
| A-1998 | Et | H | Cl | F | CH₂CH₂NHS(=O)CF₃ | O |
| A-1999 | Et | H | Cl | F | CH₂CH₂CH₂NHS(=O)CF₃ | O |
| A-2000 | Et | H | Cl | F | CH₂CH₂CH₂CH₂NHS(=O)CF₃ | O |
| A-2001 | Et | H | Cl | F | CH₂CH₂CH₂CH₂CH₂NHS(=O)CF₃ | O |
| A-2002 | Et | H | Cl | F | CH₂CH₂CH₂NHS(=O)Ph | O |
| A-2003 | Et | H | Cl | F | CH₂CH₂CH₂CH₂NHS(=O)Ph | O |
| A-2004 | Et | H | Cl | F | CH₂CH₂CH₂CH₂CH₂NHS(=O)Ph | O |
| A-2005 | Et | H | Cl | F | CH₂CH₂CH₂CH₂CH₂NHS(=O)Ph(4-CF₃) | O |
| A-2006 | Et | H | Cl | F | CH₂CH(Me)NHS(=O)Ph(4-CF₃) | O |
| A-2007 | Et | H | Cl | F | CH₂CH(Et)NHS(=O)Ph(4-CF₃) | O |
| A-2008 | Et | H | Cl | F | CH(Me)CH₂NHS(=O)Ph(4-CF₃) | O |
| A-2009 | Et | H | Cl | F | CH(Et)CH₂NHS(=O)Ph(4-CF₃) | O |
| A-2010 | Et | H | Cl | F | CH₂CH(Et)CH₂NHS(=O)Ph(4-CF₃) | O |
| A-2011 | Et | H | Cl | F | CH₂CH₂NHS(=O)₂Me | O |
| A-2012 | Et | H | Cl | F | CH₂CH₂CH₂NHS(=O)₂Me | O |
| A-2013 | Et | H | Cl | F | CH₂CH₂CH₂CH₂NHS(=O)₂Me | O |
| A-2014 | Et | H | Cl | F | CH₂CH₂CH₂CH₂NHS(=O)₂CH(CH₃)₂ | O |
| A-2015 | Et | H | Cl | F | CH₂CH₂CH₂CH₂CH₂NHS(=O)₂Me | O |
| A-2016 | Et | H | Cl | F | CH₂CH₂NHS(=O)₂CHF₂ | O |
| A-2017 | Et | H | Cl | F | CH₂CH₂CH₂NHS(=O)₂CHF₂ | O |
| A-2018 | Et | H | Cl | F | CH₂CH₂CH₂CH₂NHS(=O)₂CHF₂ | O |

**[Table 47]**

| Compound Number | R¹ | R² | R³ | R⁴ | R⁵ | A |
|---|---|---|---|---|---|---|
| A-2019 | Et | H | Cl | F | CH₂CH₂CH₂CH₂CH₂NHS(=O)₂CHF₂ | O |
| A-2020 | Et | H | Cl | F | CH₂CH(Me)NHS(=O)₂CHF₂ | O |
| A-2021 | Et | H | Cl | F | CH(Me)CH₂NHS(=O)₂CHF₂ | O |
| A-2022 | Et | H | Cl | F | CH₂CH₂CH(Me)NHS(=O)₂CHF₂ | O |
| A-2023 | Et | H | Cl | F | CH₂CH(Me)CH₂NHS(=O)₂CHF₂ | O |
| A-2024 | Et | H | Cl | F | CH(Me)CH₂CH₂NHS(=O)₂CHF₂ | O |
| A-2025 | Et | H | Cl | F | CH₂CH(Me)N(Me)S(=O)₂CHF₂ | O |
| A-2026 | Et | H | Cl | F | CH(Me)CH₂N(Me)S(=O)₂CHF₂ | O |
| A-2027 | Et | H | Cl | F | CH₂CH₂CH(Me)N(Me)S(=O)₂CHF₂ | O |
| A-2028 | Et | H | Cl | F | CH₂CH(Me)CH₂N(Me)S(=O)₂CHF₂ | O |
| A-2029 | Et | H | Cl | F | CH(Me)CH₂CH₂N(Me)S(=O)₂CHF₂ | O |
| A-2030 | Et | H | Cl | F | CH₂CH₂CH₂NHS(=O)₂Ph | O |
| A-2031 | Et | H | Cl | F | C H₂CH₂CH₂CH₂NHS(=O)₂Ph | O |
| A-2032 | Et | H | Cl | F | CH₂CH₂CH₂CH₂CH₂NHS(=O)₂Ph | O |
| A-2033 | Et | H | Cl | F | CH₂CH₂CH₂CH₂CH₂NHS(=O)₂Ph(4-CF₃) | O |
| A-2034 | Et | H | Cl | F | CH₂CH(Me)NHS(=O)₂Ph(4-CF₃) | O |
| A-2035 | Et | H | Cl | F | CH₂CH(Et)NHS(=O)₂Ph(4-CF₃) | O |
| A-2036 | Et | H | Cl | F | CH(Me)CH₂NHS(=O)₂Ph(4-CF₃) | O |
| A-2037 | Et | H | Cl | F | CH(Et)CH₂NHS(=O)₂Ph(4-CF₃) | O |
| A-2038 | Et | H | Cl | F | CH₂CH(Me)CH₂NHS(=O)₂Ph(4-CF₃) | O |
| A-2039 | Et | H | Cl | F | CH₂CH₂SiMe₃ | O |
| A-2040 | Et | H | Cl | F | CH₂CH₂CH₂SiMe₃ | O |
| A-2041 | Et | H | Cl | F | CH₂CH₂CH₂CH₂SiMe₃ | O |
| A-2042 | Et | H | Cl | F | CH₂Ph | O |
| A-2043 | Et | H | Cl | F | CH₂Ph(2-CF₃) | O |
| A-2044 | Et | H | Cl | F | CH₂Ph(3-CF₃) | O |
| A-2045 | Et | H | Cl | F | CH₂Ph(4-CF₃) | O |
| A-2046 | Et | H | Cl | F | CH₂Ph(2-OCF₃) | O |
| A-2047 | Et | H | Cl | F | CH₂Ph(3-OCF₃) | O |
| A-2048 | Et | H | Cl | F | CH₂Ph(4-OCF₃) | O |
| A-2049 | Et | H | Cl | F | CH₂Ph(2-SCF₃) | O |
| A-2050 | Et | H | Cl | F | CH₂Ph(3-SCF₃) | O |
| A-2051 | Et | H | Cl | F | CH₂Ph(4-SCF₃) | O |
| A-2052 | Et | H | Cl | F | CH₂Ph(3-CH₂SCF₃) | O |
| A-2053 | Et | H | Cl | F | CH₂Ph(4-F) | O |
| A-2054 | Et | H | Cl | F | CH₂Ph(4-Cl) | O |
| A-2055 | Et | H | Cl | F | CH₂Ph(4-Br) | O |
| A-2056 | Et | H | Cl | F | CH₂Ph(4-Me) | O |
| A-2057 | Et | H | Cl | F | CH₂Ph[4-(*t*-Bu)] | O |
| A-2058 | Et | H | Cl | F | CH₂Ph(4-CN) | O |
| A-2059 | Et | H | Cl | F | CH₂Ph(4-NO₂) | O |
| A-2060 | Et | H | Cl | F | CH₂Ph(4-OCHF₂) | O |
| A-2061 | Et | H | Cl | F | CH₂Ph(4-SCHF₂) | O |
| A-2062 | Et | H | Cl | F | CH₂Ph(4-CH₂SCF₃) | O |

**[Table 48]**

| Compound Number | R¹ | R² | R³ | R⁴ | R⁵ | A |
|---|---|---|---|---|---|---|
| A-2063 | Et | H | Cl | F | CH₂Ph[4-CF(CF₃)₂] | O |
| A-2064 | Et | H | Cl | F | CH₂Ph(4-CH₂SCF₃) | O |
| A-2065 | Et | H | Cl | F | CH₂Ph[4-Ph(4-CF₃)] | O |
| A-2066 | Et | H | Cl | F | CH₂Ph(2,4-Cl₂) | O |
| A-2067 | Et | H | Cl | F | CH₂Ph[2,5-(CF₃)₂] | O |
| A-2068 | Et | H | Cl | F | CH₂Ph(3,4-Cl₂) | O |
| A-2069 | Et | H | Cl | F | CH₂Ph(3-CF₃-4-F) | O |
| A-2070 | Et | H | Cl | F | CH₂Ph(3-CF₃-4-Cl) | O |
| A-2071 | Et | H | Cl | F | CH₂Ph(3-F-4-CF₃) | O |
| A-2072 | Et | H | Cl | F | CH₂Ph(2,4,6-F₃) | O |
| A-2073 | Et | H | Cl | F | CH₂Ph(3,4,5-F₃) | O |
| A-2074 | Et | H | Cl | F | CH₂Ph(2,3,4-F₃) | O |
| A-2075 | Et | H | Cl | F | CH₂Ph(3,4,5-Cl₃) | O |
| A-2076 | Et | H | Cl | F | CH₂CH₂Ph | O |
| A-2077 | Et | H | Cl | F | CH₂CH₂Ph(4-F) | O |
| A-2078 | Et | H | Cl | F | CH₂CH₂Ph(4-Cl) | O |
| A-2079 | Et | H | Cl | F | CH₂CH₂Ph(4-Br) | O |
| A-2080 | Et | H | Cl | F | CH₂CH₂Ph[4-(*t*-Bu)] | O |
| A-2081 | Et | H | Cl | F | CH₂CH₂Ph(2-CF₃) | O |
| A-2082 | Et | H | Cl | F | CH₂CH₂Ph(3-CF₃) | O |
| A-2083 | Et | H | Cl | H | CH₂CH₂Ph(4-CF₃) | O |
| A-2084 | Et | H | Cl | F | CH₂CH₂Ph(4-CF₃) | O |
| A-2085 | Et | H | Cl | F | CH₂CH₂Ph(4-CF(CF₃)₂) | O |
| A-2086 | Et | H | Cl | F | CH₂CH₂Ph[4-(*c*-Pr)] | O |
| A-2087 | Et | H | Cl | F | CH₂CH₂Ph{4-[*c*-Pr(2,2-F₂)]} | O |
| A-2088 | Et | H | Cl | F | CH₂CH₂Ph(4-OMe) | O |
| A-2089 | Et | H | Cl | F | CH₂CH₂Ph(3-OCF₃) | O |
| A-2090 | Et | H | Cl | F | CH₂CH₂Ph(4-OCHF₂) | O |
| A-2091 | Et | H | Cl | F | CH₂CH₂Ph(4-OCF₃) | O |
| A-2092 | Et | H | Cl | F | CH₂CH₂Ph(4-SMe) | O |
| A-2093 | Et | H | Cl | F | CH₂CH₂Ph(4-SCHF₂) | O |
| A-2094 | Et | H | Cl | F | CH₂CH₂Ph(4-SCF₃) | O |
| A-2095 | Et | H | Cl | F | CH₂CH₂Ph[4-S(=O)Me] | O |
| A-2096 | Et | H | Cl | F | CH₂CH₂Ph[4-S(=O)CF₃] | O |
| A-2097 | Et | H | Cl | F | CH₂CH₂Ph[4-S(=O)₂Me] | O |
| A-2098 | Et | H | Cl | F | CH₂CH₂Ph[4-S(=O)₂CF₃] | O |
| A-2099 | Et | H | Cl | F | CH₂CH₂Ph(4-CH₂SMe) | O |
| A-2100 | Et | H | Cl | F | CH₂CH₂Ph(4-CH₂SCF₃) | O |
| A-2101 | Et | H | Cl | F | CH₂CH₂Ph[4-OS(=O)₂Me] | O |
| A-2102 | Et | H | Cl | F | CH₂CH₂Ph[4-OS(=O)₂CF₃] | O |
| A-2103 | Et | H | Cl | F | CH₂CH₂Ph[4-Ph(4-CF₃)] | O |
| A-2104 | Et | H | Cl | F | CH₂CH₂Ph(4-CH₂Ph) | O |
| A-2105 | Et | H | Cl | F | CH₂CH₂Ph(4-OCH₂Ph) | O |
| A-2106 | Et | H | Cl | F | CH₂CH₂Ph(4-CN) | O |

**[Table 49]**

| Compound Number | R¹ | R² | R³ | R⁴ | R⁵ | A |
|---|---|---|---|---|---|---|
| A-2107 | Et | H | Cl | F | CH₂CH₂Ph(4-NO₂) | O |
| A-2108 | Et | H | Cl | F | CH₂CH₂Ph(2,4-Cl₂) | O |
| A-2109 | Et | H | Cl | F | CH₂CH₂Ph(3,4-Cl₂) | O |
| A-2110 | Et | H | Cl | F | CH₂CH₂Ph(3-CF₃-4-F) | O |
| A-2111 | Et | H | Cl | F | CH₂CH₂Ph(2-CF₃-4-F) | O |
| A-2112 | Et | H | Cl | F | CH₂CH₂Ph(3-F-4-CF₃) | O |
| A-2113 | Et | H | Cl | F | CH₂CH₂Ph(2-F-4-CF₃) | O |
| A-2114 | Et | H | Cl | F | CH₂CH₂Ph(3-Cl-4-OCHF₂) | O |
| A-2115 | Et | H | Cl | F | CH₂CH₂Ph(3,4,5-Cl₃) | O |
| A-2116 | Et | H | Cl | F | CH₂CH₂Ph(2,3,4-F₃) | O |
| A-2117 | Et | H | Cl | F | CH₂CH₂Ph(2,4,5-F₃) | O |
| A-2118 | Et | H | Cl | F | CH₂CH₂Ph(3,4,5-F₃) | O |
| A-2119 | Et | H | Cl | F | CH₂CH₂Ph(2,4,6-F₃) | O |
| A-2120 | Et | H | Cl | F | CH₂CH₂CH₂Ph | O |
| A-2121 | Et | H | Cl | F | CH₂CH₂CH₂Ph(3-CF₃) | O |
| A-2122 | Et | H | Cl | F | CH₂CH₂CH₂Ph(4-CF₃) | O |
| A-2123 | Et | H | Cl | F | CH₂CH₂CH₂Ph(4-F) | O |
| A-2124 | Et | H | Cl | F | CH₂CH₂CH₂Ph[4-(*t*-Bu)] | O |
| A-2125 | Et | H | Cl | F | CH₂CH₂CH₂Ph(4-CN) | O |
| A-2126 | Et | H | Cl | F | CH₂CH₂CH₂Ph(4-CF₃) | O |
| A-2127 | Et | H | Cl | F | CH₂CH₂CH₂Ph(4-OCHF₂) | O |
| A-2128 | Et | H | Cl | F | CH₂CH₂CH₂Ph(4-OCF₃) | O |
| A-2129 | Et | H | Cl | F | CH₂CH₂CH₂Ph(4-SCHF₂) | O |
| A-2130 | Et | H | Cl | F | CH₂CH₂CH₂Ph(4-SCF₃) | O |
| A-2131 | Et | H | Cl | F | CH₂CH₂CH₂Ph[4-CF(CF)₃)₂] | O |
| A-2132 | Et | H | Cl | F | CH₂CH₂CH₂Ph(3,4,5-F₃) | O |
| A-2133 | Et | H | Cl | F | CH₂CH₂CH₂Ph(2,4,6-F₃) | O |
| A-2134 | Et | H | Cl | F | CH₂CH₂CH₂CH₂Ph | O |
| A-2135 | Et | H | Cl | F | CH₂CH₂CH₂CH₂Ph(4-F) | O |
| A-2136 | Et | H | Cl | F | CH₂CH₂CH₂CH₂Ph(4-CF₃) | O |
| A-2137 | Et | H | Cl | F | CH₂CH₂CH₂CH₂Ph(4-OCF₃) | O |
| A-2138 | Et | H | Cl | F | CH₂CH₂CH₂CH₂Ph(4-SCF₃) | O |
| A-2139 | Et | H | Cl | F | CH₂CH₂CH₂CH₂CH₂Ph | O |
| A-2140 | Et | H | Cl | F | CH₂CH₂CH₂CH₂CH₂CH₂Ph | O |
| A-2141 | Et | H | Cl | F | CH₂CF₂Ph(4-F) | O |
| A-2142 | Et | H | Cl | F | CH₂CF₂Ph(4-CF₃) | O |
| A-2143 | Et | H | Cl | H | CH₂CF₂Ph(3,4,5-F₃) | O |
| A-2144 | Et | H | Cl | F | CH₂CF₂Ph(3,4,5-F₃) | O |
| A-2145 | Et | H | Cl | F | CH₂CH₂OPh | O |
| A-2146 | Et | H | Cl | F | CH₂CH₂OPh(4-F) | O |
| A-2147 | Et | H | Cl | F | CH₂CH₂OPh(4-CF₃) | O |
| A-2148 | Et | H | Cl | F | CH₂CH₂CH₂OPh | O |
| A-2149 | Et | H | Cl | F | CH₂CH₂CH₂OPh(4-Cl) | O |
| A-2150 | Et | H | Cl | F | CH₂CH₂CH₂OPh(4-CF₃) | O |

**[Table 50]**

| Compound Number | R¹ | R² | R³ | R⁴ | R⁵ | A |
|---|---|---|---|---|---|---|
| A-2151 | Et | H | Cl | F | CH₂CH₂CH₂CH₂OPh(4-CF₃) | O |
| A-2152 | Et | H | Cl | F | CH₂CH₂CH₂CH₂OPh(4-OCF₃) | O |
| A-2153 | Et | H | Cl | F | CH₂CH₂CH₂CH₂CH₂OPh(4-CF₃) | O |
| A-2154 | Et | H | Cl | F | CH₂CH₂OCH₂Ph | O |
| A-2155 | Et | H | Cl | F | CH₂CH₂CH₂OCH₂Ph | O |
| A-2156 | Et | H | Cl | F | CH₂CH₂CH₂CH₂OCH₂Ph | O |
| A-2157 | Et | H | Cl | F | CH₂CH₂CH₂CH₂CH₂OCH₂Ph | O |
| A-2158 | Et | H | Cl | F | CH₂CH₂SPh | O |
| A-2159 | Et | H | Cl | F | CH₂CH₂CH₂SPh | O |
| A-2160 | Et | H | Cl | F | CH₂CH₂CH₂SPh(4-F) | O |
| A-2161 | Et | H | Cl | F | CH₂CH₂CH₂SPh(4-Cl) | O |
| A-2162 | Et | H | Cl | F | CH₂CH₂CH₂SPh(4-Br) | O |
| A-2163 | Et | H | Cl | F | CH₂CH₂CH₂SPh[4-(*t*-Bu)] | O |
| A-2164 | Et | H | Cl | F | CH₂CH₂CH₂SPh(3-CF₃) | O |
| A-2165 | Et | H | Cl | F | CH₂CH₂CH₂SPh(4-CF₃) | O |
| A-2166 | Et | H | Cl | F | CH₂CH₂CH₂SPh(3-SCF₃) | O |
| A-2167 | Et | H | Cl | F | CH₂CH₂CH₂SPh(4-SCF₃) | O |
| A-2168 | Et | H | Cl | F | CH₂CH₂CH₂CH₂SPh | O |
| A-2169 | Et | H | Cl | F | CH₂CH₂CH₂CH₂SPh(4-F) | O |
| A-2170 | Et | H | Cl | F | CH₂CH₂CH₂CH₂SPh(4-Cl) | O |
| A-2171 | Et | H | Cl | F | CH₂CH₂CH₂CH₂SPh[4-(*t*-Bu)] | O |
| A-2172 | Et | H | Cl | F | CH₂CH₂CH₂CH₂SPh(4-CF₃) | O |
| A-2173 | Et | H | Cl | F | CH₂CH₂CH₂CH₂CH₂SPh | O |
| A-2174 | Et | H | Cl | F | CH₂CH₂CH₂CH₂CH₂SPh(4-F) | O |
| A-2175 | Et | H | Cl | F | CH₂CH₂CH₂CH₂CH₂SPh(4-Cl) | O |
| A-2176 | Et | H | Cl | F | CH₂CH₂CH₂CH₂CH₂SPh(4-CF₃) | O |
| A-2177 | Et | H | Cl | F | CH₂CH₂S(=O)Ph | O |
| A-2178 | Et | H | Cl | F | CH₂CH₂CH₂S(=O)Ph | O |
| A-2179 | Et | H | Cl | F | CH₂CH₂CH₂S(=O)Ph(4-F) | O |
| A-2180 | Et | H | Cl | F | CH₂CH₂CH₂S(=O)Ph[4-(*t*-Bu)] | O |
| A-2181 | Et | H | Cl | F | CH₂CH₂CH₂S(=O)Ph(4-CF₃) | O |
| A-2182 | Et | H | Cl | F | CH₂CH₂S(=O)₂Ph | O |
| A-2183 | Et | H | Cl | F | CH₂CH₂S(=O)₂Ph(4-CF₃) | O |
| A-2184 | Et | H | Cl | F | CH₂CH₂CH₂S(=O)₂Ph(4-F) | O |
| A-2185 | Et | H | Cl | F | CH₂CH₂CH₂S(=O)₂Ph(4-Cl) | O |
| A-2186 | Et | H | Cl | F | CH₂CH₂CH₂S(=O)₂Ph(4-CF₃) | O |
| A-2187 | Et | H | Cl | F | CH₂CH₂CH₂CH₂S(=O)₂Ph | O |
| A-2188 | Et | H | Cl | F | CH₂CH₂CH₂CH₂S(=O)₂Ph(4-Cl) | O |
| A-2189 | Et | H | Cl | F | CH₂CH₂CH₂CH₂S(=O)₂Ph(4-CF₃) | O |
| A-2190 | Et | H | Cl | F | CH₂CH₂SCH₂Ph | O |
| A-2191 | Et | H | Cl | F | CH₂CH₂CH₂SCH₂Ph | O |
| A-2192 | Et | H | Cl | F | CH₂CH₂CH₂SCH₂Ph(2-Cl) | O |
| A-2193 | Et | H | Cl | F | CH₂CH₂CH₂SCH₂Ph(3-Cl) | O |
| A-2194 | Et | H | Cl | F | CH₂CH₂CH₂SCH₂Ph(4-Cl) | O |

**[Table 51]**

| Compound Number | R¹ | R² | R³ | R⁴ | R⁵ | A |
|---|---|---|---|---|---|---|
| A-2195 | Et | H | Cl | F | CH₂CH₂CH₂SCH₂Ph(2-SCF₃) | O |
| A-2196 | Et | H | Cl | F | CH₂CH₂CH₂SCH₂Ph(3-CF₃) | O |
| A-2197 | Et | H | Cl | F | CH₂CH₂CH₂SCH₂Ph(4-CF₃) | O |
| A-2198 | Et | H | Cl | F | CH₂CH₂CH₂SCH₂Ph(4-NO₂) | O |
| A-2199 | Et | H | Cl | F | CH₂CH₂CH₂CH₂SCH₂Ph | O |
| A-2200 | Et | H | Cl | F | CH₂CH₂CH₂CH₂SCH₂Ph(4-Cl) | O |
| A-2201 | Et | H | Cl | F | CH₂CH₂CH₂CH₂SCH₂Ph(4-CF₃) | O |
| A-2202 | Et | H | Cl | F | CH₂CH₂CH₂CH₂SCH₂Ph(4-CN) | O |
| A-2203 | Et | H | Cl | F | CH₂CH₂CH₂CH₂CH₂SCH₂Ph | O |
| A-2204 | Et | H | Cl | F | CH₂CH₂SCH₂CH₂Ph | O |
| A-2205 | Et | H | Cl | F | CH₂CH₂CH₂SCH₂CH₂Ph | O |
| A-2206 | Et | H | Cl | F | CH₂CH₂CH₂CH₂SCH₂CH₂Ph | O |
| A-2207 | Et | H | Cl | F | CH₂CH₂SCH(Me)Ph | O |
| A-2208 | Et | H | Cl | F | CH₂CH₂CH₂SCH(Me)Ph | O |
| A-2209 | Et | H | Cl | F | CH₂CH₂CH₂CH₂SCH(Me)Ph | O |
| A-2210 | Et | H | Cl | F | CH₂CH₂S(=O)CH₂Ph | O |
| A-2211 | Et | H | Cl | F | CH₂CH₂S(=O)CH₂Ph(4-CF₃) | O |
| A-2212 | Et | H | Cl | F | CH₂CH₂CH₂S(=O)CH₂Ph | O |
| A-2213 | Et | H | Cl | F | CH₂CH₂CH₂S(=O)CH₂Ph(2-SCF₃) | O |
| A-2214 | Et | H | Cl | F | CH₂CH₂CH₂S(=O)CH₂Ph(4-SCF₃) | O |
| A-2215 | Et | H | Cl | F | CH₂CH₂CH₂S(=O)CH₂Ph(4-Cl) | O |
| A-2216 | Et | H | Cl | F | CH₂CH₂CH₂S(=O)CH₂Ph(4-CF₃) | O |
| A-2217 | Et | H | Cl | F | CH₂CH₂CH₂CH₂S(=O)CH₂Ph | O |
| A-2218 | Et | H | Cl | F | CH₂CH₂CH₂CH₂S(=O)CH₂Ph(4-CF₃) | O |
| A-2219 | Et | H | Cl | F | CH₂CH₂CH₂CH₂CH₂S(=O)CH₂Ph(4-CF₃) | O |
| A-2220 | Et | H | Cl | F | CH₂CH₂S(=O)₂CH₂Ph | O |
| A-2221 | Et | H | Cl | F | CH₂CH₂S(=O)₂CH₂Ph(4-CF₃) | O |
| A-2222 | Et | H | Cl | F | CH₂CH₂CH₂S(=O)₂CH₂Ph | O |
| A-2223 | Et | H | Cl | F | CH₂CH₂CH₂S(=O)₂CH₂Ph(4-Cl) | O |
| A-2224 | Et | H | Cl | F | CH₂CH₂CH₂S(=O)₂CH₂Ph(4-CF₃) | O |
| A-2225 | Et | H | Cl | F | CH₂CH₂CH₂CH₂S(=O)₂CH₂Ph | O |
| A-2226 | Et | H | Cl | F | CH₂CH₂CH₂CH₂S(=O)₂CH₂Ph(4-CF₃) | O |
| A-2227 | Et | H | Cl | F | CH₂CH₂CH₂CH₂CH₂S(=O)₂CH₂Ph | O |
| A-2228 | Et | H | Cl | F | CH₂CH₂CH₂CH₂CH₂S(=O)₂CH₂Ph(4-CF₃) | O |
| A-2229 | Et | H | Cl | F | CH₂CH₂ON=CH(*t*-Bu) | O |
| A-2230 | Et | H | Cl | F | CH₂CH₂CH₂ON=CH(*t*-Bu) | O |
| A-2231 | Et | H | Cl | F | CH₂CH₂CH₂CH₂ON=C(Me)(*c* -Pr) | O |
| A-2232 | Et | H | Cl | F | CH₂CH₂ON=C(Me)CF₃ | O |
| A-2233 | Et | H | Cl | F | CH₂CH₂CH₂ON=CHCF₃ | O |
| A-2234 | Et | H | Cl | F | CH₂CH₂CH₂ON=C(Me)CF₃ | O |
| A-2235 | Et | H | Cl | F | CH₂CH₂CH₂ON=C(Me)CCl₃ | O |
| A-2236 | Et | H | Cl | F | CH₂CH₂CH₂CH₂ON=CHCF₃ | O |
| A-2237 | Et | H | Cl | F | CH₂CH₂CH₂CH₂ON=C(Me)CF₃ | O |
| A-2238 | Et | H | Cl | F | CH₂CH₂CH₂CH₂CH₂ON=C(Me)CF₃ | O |
| | | | | | | |

**[Table 52]**

| Compound Number | R¹ | R² | R³ | R⁴ | R⁵ | A |
|---|---|---|---|---|---|---|
| A-2239 | Et | H | Cl | F | CH₂CH₂ON=CHPh | O |
| A-2240 | Et | H | Cl | F | CH₂CH₂ON=CHPh(4-CF₃) | O |
| A-2241 | Et | H | Cl | F | CH₂CH₂ON=CHPh(4-SCF₃) | O |
| A-2242 | Et | H | Cl | F | CH₂CH₂CH₂ON=CHPh(3-CF₃) | O |
| A-2243 | Et | H | Cl | F | CH₂CH₂CH₂ON=CHPh(4-CF₃) | O |
| A-2244 | Et | H | Cl | F | CH₂CH₂CH₂ON=CHPh(4-SCF₃) | O |
| A-2245 | Et | H | Cl | F | CH₂CH₂CH₂ON=C(Me)Ph(4-CF₃) | O |
| A-2246 | Et | H | Cl | F | CH₂CH₂CH₂CH₂ON=CHPh(4-SCF₃) | O |
| A-2247 | Et | H | Cl | F | CH₂CH₂(adamant-1-yl) | O |
| A-2248 | Et | H | Cl | F | CH₂CH₂CH₂(3-CF₃-pyrazol-1-yl) | O |
| A-2249 | Et | H | Cl | F | CH₂CH₂CH₂CH₂(3-CF₃-pyrazol-1-yl) | O |
| A-2250 | Et | H | Cl | F | CH₂CH₂CH₂CH₂CH₂(3-CF₃-pyrazol-1-yl) | O |
| A-2251 | Et | H | Cl | F | CH₂(1-Me-4-CF₃-imidazol-5-yl) | O |
| A-2252 | Et | H | Cl | F | CH₂CH₂(1-Me-4-CF₃-imidazol-5-yl) | O |
| A-2253 | Et | H | Cl | F | CH₂CH₂CH₂(1-Me-4-CF₃-imidazol-5-yl) | O |
| A-2254 | Et | H | Cl | F | CH₂CH₂CH₂(3-CF₃-1,2,4-triazol-1-yl) | O |
| A-2255 | Et | H | Cl | F | CH₂CH₂CH₂CH₂(3-CF₃-1,2,4-triazol-1-yl) | O |
| A-2256 | Et | H | Cl | F | CH₂CH₂CH₂CH₂CH₂(3-CF₃-1,2,4-triazol-1-yl) | O |
| A-2257 | Et | H | Cl | F | CH₂CH₂CH₂(4-CF₃-thiazol-2-yl) | O |
| A-2258 | Et | H | Cl | F | CH₂CH₂CH₂CH₂(4-CF₃-thiazol-2-yl) | O |
| A-2259 | Et | H | Cl | F | CH₂CH₂CH₂CH₂CH₂(4-CF₃-thiazol-2-yl) | O |
| A-2260 | Et | H | Cl | F | CH₂CH₂CH₂(2-CF₃-pyridin-5-yl) | O |
| A-2261 | Et | H | Cl | F | CH₂CH₂CH₂CH₂(2-CF₃-pyridin-5-yl) | O |
| A-2262 | Et | H | Cl | F | CH₂CH₂CH₂CH₂CH₂(2-CF₃-pyridin-5-yl) | O |
| A-2263 | Et | H | Cl | F | CH₂(4-CF₃-pyrimidin-2-yl) | O |
| A-2264 | Et | H | Cl | F | CH₂CH₂(4-CF₃-pyrimidin-2-yl) | O |
| A-2265 | Et | H | Cl | F | CH₂CH₂CH₂(4-CF₃-pyrimidin-2-yl) | O |
| A-2266 | Et | H | Cl | F | CH₂CH₂O(3-Cl-5-CF₃-pyridin-2-yl) | O |
| A-2267 | Et | H | Cl | F | CH₂CH₂CH₂O(3-Cl-5-CF₃-pyridin-2-yl) | O |
| A-2268 | Et | H | Cl | F | CH₂CH₂CH₂CH₂O(3-Cl-5-CF₃-pyridin-2-yl) | O |
| A-2269 | Et | H | Cl | F | CH₂CH₂CH₂CH₂CH₂O(3-Cl-5-CF₃-pyridin-2-yl) | O |
| A-2270 | Et | H | Cl | F | CH₂CH₂N(Phth) | O |
| A-2271 | Et | H | Cl | F | CH₂CH₂CH₂N(Phth) | O |
| A-2272 | Et | H | Cl | F | CH₂CH₂CH₂CH₂N(Phth) | O |
| A-2273 | Et | H | Cl | F | CH₂CH₂CH₂CH₂CH₂N(Phth) | O |
| A-2274 | Et | H | Cl | F | CH₂CH(Me)CH₂N(Phth) | O |
| A-2275 | Et | H | Cl | F | CH₂[1,3-dioxolan(2-CF₃)-2-yl] | O |
| A-2276 | Et | H | Cl | F | CH₂(azetidin-3-yl) | O |
| A-2277 | Et | H | Cl | F | CH₂(pyrrolidin-3-yl) | O |
| A-2278 | Et | H | Cl | F | CH₂(piperidin-3-yl) | O |
| A-2279 | Et | H | Cl | F | CH₂{azetidin[1-C(=O)O(*t*-Bu)]-3-yl} | O |
| A-2280 | Et | H | Cl | F | CH₂{pyrrolidin[1-C(=O)O(*t*-Bu)]-3-yl} | O |
| A-2281 | Et | H | Cl | F | CH₂{piperidin[1-C(=O)O(*t*-Bu)]-3-yl} | O |
| A-2282 | Et | H | Cl | F | CH₂{azetidin[1-C(=O)CF₃]-3-yl} | O |

**[Table 53]**

| Compound Number | R¹ | R² | R³ | R⁴ | R⁵ | A |
|---|---|---|---|---|---|---|
| A-2283 | Et | H | Cl | F | CH₂{azetidin[1-S(=O)₂CF₃]-3-yl} | O |
| A-2284 | Et | H | Cl | F | CH₂{pyrrolidin[1-C(=O)CF₃]-3-yl} | O |
| A-2285 | Et | H | Cl | F | CH₂{pyrrolidin[1-S(=O)₂CF₃]-3-yl} | O |
| A-2286 | Et | H | Cl | F | CH₂{piperidin[1-C(=O)CF₃]-3-yl} | O |
| A-2287 | Et | H | Cl | F | CH₂{piperidin[1-S(=O)₂CF₃]-3-yl} | O |
| A-2288 | Et | H | Cl | F | CH₂CH₂CH₂CN | O |
| A-2289 | Et | H | Cl | F | CH₂CH₂CH₂CH₂CN | O |
| A-2290 | Et | H | Cl | F | CH₂CH₂CH₂CH₂CH₂CN | O |
| A-2291 | Et | H | Cl | F | CH₂CH₂CH₂CH₂CH₂CH₂CN | O |
| A-2292 | Et | H | Cl | F | CH₂CH₂CH₂C(=O)OH | O |
| A-2293 | Et | H | Cl | F | CH₂CH₂CH₂CH₂C(=O)OH | O |
| A-2294 | Et | H | Cl | F | CH₂CH₂CH₂CH₂CH₂C(=O)OH | O |
| A-2295 | Et | H | Cl | F | CH₂CH₂SCN | O |
| A-2296 | Et | H | Cl | F | CH₂CH₂CH₂SCN | O |
| A-2297 | Et | H | Cl | F | CH₂CH₂CH₂CH₂SCN | O |
| A-2298 | Et | H | Cl | F | CH₂CH₂CH₂CH₂CH₂SCN | O |
| A-2299 | Et | H | Cl | Cl | CH₂CH₂CH₂CH₂CH₂SCN | O |
| A-2300 | Et | H | Cl | F | CH₂CH₂CH₂CH₂CH₂CH₂SCN | O |
| A-2301 | Et | H | Cl | F | CH₂CH₂CH₂CH₂CH₂CH₂CH₂SCN | O |
| A-2302 | Et | H | Cl | F | CH₂CH₂CH₂CH₂CH₂CH₂CH₂CH₂SCN | O |
| A-2303 | Et | H | Cl | F | CH₂CH₂CH(Me)SCN | O |
| A-2304 | Et | H | Cl | F | CH₂CH(Me)CH₂SCN | O |
| A-2305 | Et | H | Cl | F | CH(Me)CH₂CH₂SCN | O |
| A-2306 | Et | H | Cl | F | CH₂CH₂CH₂CH(Me)SCN | O |
| A-2307 | Et | H | Cl | F | CH₂CH₂CH(Me)CH₂SCN | O |
| A-2308 | Et | H | Cl | F | CH₂CH(Me)CH₂CH₂SCN | O |
| A-2309 | Et | H | Cl | F | CH(Me)CH₂CH₂CH₂SCN | O |
| A-2310 | Et | H | Cl | F | CH₂CH₂CH₂CH₂CH(Me)SCN | O |
| A-2311 | Et | H | Cl | F | CH₂CH₂CH₂CH(Me)CH₂SCN | O |
| A-2312 | Et | H | Cl | F | CH₂CH₂CH(Me)CH₂CH₂SCN | O |
| A-2313 | Et | H | Cl | F | CH₂CH(Me)CH₂CH₂CH₂SCN | O |
| A-2314 | Et | H | Cl | F | CH(Me)CH₂CH₂CH₂CH₂SCN | O |
| A-2315 | Et | H | Cl | F | CH₂CH₂ONH₂ | O |
| A-2316 | Et | H | Cl | F | CH₂CH₂CH₂ONH₂ | O |
| A-2317 | Et | H | Cl | F | CH₂CH₂CH₂CH₂ONH₂ | O |
| A-2318 | Et | H | Cl | F | CH₂CH₂CH₂CH₂CH₂ONH₂ | O |
| A-2319 | Et | H | Cl | F | CH₂CH₂CH₂CH₂CH₂CH₂ONH₂ | O |
| A-2320 | Et | H | Me | F | Me | O |
| A-2321 | Et | H | Me | F | Et | O |
| A-2322 | Et | H | Me | F | *n*-Pr | O |
| A-2323 | Et | H | Me | F | *i*-Pr | O |
| A-2324 | Et | H | Me | F | *n*-Bu | O |
| A-2325 | Et | H | Me | F | *n*-Pen | O |
| A-2326 | Et | H | Me | F | *n*-Hex | O |

**[Table 54]**

| Compound Number | R¹ | R² | R³ | R⁴ | R⁵ | A |
|---|---|---|---|---|---|---|
| A-2327 | Et | H | Me | F | *n*-Heptyl | O |
| A-2328 | Et | H | Me | F | *n*-Octyl | O |
| A-2329 | Et | H | Me | F | *n*-Nonyl | O |
| A-2330 | Et | H | Me | F | *n*-Decyl | O |
| A-2331 | Et | H | Me | F | *c*-Pr | O |
| A-2332 | Et | H | Me | F | *c*-Pen | O |
| A-2333 | Et | H | Me | F | *c*-Hex | O |
| A-2334 | Et | H | Me | F | CH₂C(Me)=CH₂ | O |
| A-2335 | Et | H | Me | F | CH₂CH₂CH=CH₂ | O |
| A-2336 | Et | H | Me | F | CH₂CH₂CH=C(CH₃)₂ | O |
| A-2337 | Et | H | Me | F | CH₂CH₂CH₂CH=CH₂ | O |
| A-2338 | Et | H | Me | F | CH₂CH₂CH₂CH=C(CH₃)₂ | O |
| A-2339 | Et | H | Me | F | CH₂CH₂CH₂CH₂CH=CH₂ | O |
| A-2340 | Et | H | Me | F | CH₂CH₂CH₂CH₂CH=C(CH₃)₂ | O |
| A-2341 | Et | H | Me | F | CH₂CH₂CH≡CH | O |
| A-2342 | Et | H | Me | F | CH₂CH₂CH₂CH≡CH | O |
| A-2343 | Et | H | Me | F | CH₂CH₂CH₂CH₂CH≡CH | O |
| A-2344 | Et | H | Me | F | CH₂CH₂CH₂Cl | O |
| A-2345 | Et | H | Me | F | CH₂CH₂CH₂Br | O |
| A-2346 | Et | H | Me | F | CH₂CH₂CH₂CH₂Cl | O |
| A-2347 | Et | H | Me | F | CH₂CH₂CH₂CH₂Br | O |
| A-2348 | Et | H | Me | F | CH₂CH₂CH₂CH₂CH₂Cl | O |
| A-2349 | Et | H | Me | F | CH₂CH₂CH₂CH₂CH₂Br | O |
| A-2350 | Et | H | Me | Cl | CH₂CH₂CH₂CH₂CH₂Br | O |
| A-2351 | Et | H | Me | F | CH₂CH₂CH₂CH₂CH₂CH₂Cl | O |
| A-2352 | Et | H | Me | F | CH₂CH₂CH₂CH₂CH₂CH₂Br | O |
| A-2353 | Et | H | Me | F | CH₂CH₂CH₂CH₂CH₂CH₂CH₂Cl | O |
| A-2354 | Et | H | Me | F | CH₂CH₂CH₂CH₂CH₂CH₂CH₂Br | O |
| A-2355 | Et | H | Me | F | CH₂CH₂CH₂CH₂CH₂CH₂CH₂CH₂Br | O |
| A-2356 | Et | H | Me | F | CH₂CH(Me)CH₂Cl | O |
| A-2357 | Et | H | Me | F | CH₂CH(Me)CH₂Br | O |
| A-2358 | Et | H | Me | F | CH₂CH₂CH(Me)CH₂CH₂Cl | O |
| A-2359 | Et | H | Me | F | CH₂CH₂CH(Me)CH₂CH₂Br | O |
| A-2360 | Et | H | Me | F | CH₂CH₂CF=CF₂ | O |
| A-2361 | Et | H | Me | F | CH₂CH=C(Cl)CF₃ | O |
| A-2362 | Et | H | Me | F | CH₂CH₂CH₂CF=CF₂ | O |
| A-2363 | Et | H | Me | F | CH₂CH₂CH₂CH₂CF=CF₂ | O |
| A-2364 | Et | H | Me | F | CH₂CH₂CH₂CH₂CH₂CF=CF₂ | O |
| A-2365 | Et | H | Me | F | CH₂(*t*-Bu) | O |
| A-2366 | Et | H | Me | F | CH₂CH₂(*t*-Bu) | O |
| A-2367 | Et | H | Me | F | CH₂CH₂CH₂(*t*-Bu) | O |
| A-2368 | Et | H | Me | F | CH₂CH₂CH₂CH₂(*t*-Bu) | O |
| A-2369 | Et | H | Me | F | CH₂CH₂CH₂CH₂CH₂(*t*-Bu) | O |
| A-2370 | Et | H | Me | F | CH₂CH₂CH₂CH₂CH₂CH₂(*t*-Bu) | O |

**[Table 55]**

| Compound Number | R¹ | R² | R³ | R⁴ | R⁵ | A |
|---|---|---|---|---|---|---|
| A-2371 | Et | H | Me | F | CH₂CF₃ | O |
| A-2372 | Et | H | Me | F | CH₂CH₂CF₃ | O |
| A-2373 | Et | H | Me | F | CH₂CH₂CH₂CF₃ | O |
| A-2374 | Et | H | Me | F | CH₂CH₂CH₂CH₂CF₃ | O |
| A-2375 | Et | H | Me | F | CH₂CH₂CH₂CH₂CH₂CF₃ | O |
| A-2376 | Et | H | Me | F | CH₂CH₂CH₂CH₂CH₂CH₂CF₃ | O |
| A-2377 | Et | H | Me | F | CH₂CH₂CH₂CH₂CH₂CH₂CH₂CF₃ | O |
| A-2378 | Et | H | Me | F | CH₂CF₂CF₃ | O |
| A-2379 | Et | H | Me | F | CF₂CHFCF₃ | O |
| A-2380 | Et | H | Me | F | CF₂CF₂CF₃ | O |
| A-2381 | Et | H | Me | F | CH₂CF₂CF₂CF₃ | O |
| A-2382 | Et | H | Me | F | CH₂CF₂CF₂CF₂CF₃ | O |
| A-2383 | Et | H | Me | F | CH₂CF₂CF₂CF₂CHF₂ | O |
| A-2384 | Et | H | Me | F | CH(CF₃)CF₃ | O |
| A-2385 | Et | H | Me | F | CH₂CF(CF₃)CF₃ | O |
| A-2386 | Et | H | Me | F | CH₂CF₂CF(CF₃)CF₃ | O |
| A-2387 | Et | H | Me | F | CH₂CF(CF₃)CF₂CF₃ | O |
| A-2388 | Et | H | Me | F | CH₂CF₂CF₂CF(CF₃)CF₃ | O |
| A-2389 | Et | H | Me | F | CH₂CF₂CF(CF₃)CF₂CF₃ | O |
| A-2390 | Et | H | Me | F | CH₂CF(CF₃)CF₂CF₂CF₃ | O |
| A-2391 | Et | H | Me | F | CH₂(c-Pr) | O |
| A-2392 | Et | H | Me | F | CH₂[*c*-Pr(1-Me)] | O |
| A-2393 | Et | H | Me | F | CH₂[*c*-Pr(1-Ph)] | O |
| A-2394 | Et | H | Me | F | CH₂[*c*-Pr(1-NH₂)] | O |
| A-2395 | Et | H | Me | F | CH₂[*c*-Pr(1-NHC(=O)O(*t*-Bu)] | O |
| A-2396 | Et | H | Me | F | CH₂{*c*-Pr[1-NHS(=O)₂CF₃]} | O |
| A-2397 | Et | H | Me | F | CH₂{*c*-pr[1-Ph(4-CF₃)]} | O |
| A-2398 | Et | H | Me | F | CH₂{*c*-Pr[1-Ph(3,4,5-F₃)]} | O |
| A-2399 | Et | H | Me | F | CH₂{*c*-Hex[4-(*t*-Bu)]} | O |
| A-2400 | Et | H | Me | F | CH₂[*c*-Hex(4-CF₃)] | O |
| A-2401 | Et | H | Me | F | CH₂CH₂(*c*-Pr) | O |
| A-2402 | Et | H | Me | F | CH₂CH₂(*c*-Hex) | O |
| A-2403 | Et | H | Me | F | CH₂CH₂[*c*-Hex(4-CF₃)] | O |
| A-2404 | Et | H | Me | F | CH₂CH₂[*c*-Hex(4-SCF₃)] | O |
| A-2405 | Et | H | Me | F | CH₂CH₂CH₂(*c*-Pr) | O |
| A-2406 | Et | H | Me | F | CH₂CH₂CH₂(*c*-Hex) | O |
| A-2407 | Et | H | Me | F | CH₂CH₂CH₂{*c*-Hex[4-(*t*-Bu)]} | O |
| A-2408 | Et | H | Me | F | CH₂CH₂CH₂[*c*-Hex(4-CF₃)] | O |
| A-2409 | Et | H | Me | F | CH₂CH₂CH₂[*c*-Hex(4-SCF₃)] | O |
| A-2410 | Et | H | Me | F | CH₂CH₂CH₂CH₂(*c*-Pr) | O |
| A-2411 | Et | H | Me | F | CH₂CH₂CH₂CH₂(*c*-Hex) | O |
| A-2412 | Et | H | Me | F | CH₂CH₂CH₂CH₂[*c*-Hex(4-CF₃)] | O |
| A-2413 | Et | H | Me | F | CH₂[*c*-Pr(2,2-F₂)] | O |
| A-2414 | Et | H | Me | F | CH₂[*c*-Hex(4,4-F₂)] | O |

**[Table 56]**

| Compound Number | R¹ | R² | R³ | R⁴ | R⁵ | A |
|---|---|---|---|---|---|---|
| A-2415 | Et | H | Me | F | CH₂CH₂[*c*-Pr(2,2-F₂)] | O |
| A-2416 | Et | H | Me | F | CH₂CH₂[*c*-Hex(4,4-F₂)] | O |
| A-2417 | Et | H | Me | F | CH₂CH₂CH₂[*c*-Pr(2,2-F₂) | O |
| A-2418 | Et | H | Me | F | CH₂CH₂CH₂CH₂[*c*-Pr(2,2-F₂)] | O |
| A-2419 | Et | H | Me | F | CH₂CH₂OH | O |
| A-2420 | Et | H | Me | F | CH₂CH₂CH₂OH | O |
| A-2421 | Et | H | Me | F | CH₂CH₂CH₂CH₂OH | O |
| A-2422 | Et | H | Me | F | CH₂CH₂CH₂CH₂CH₂OH | O |
| A-2423 | Et | H | Me | F | CH₂CH₂CH₂CH₂CH₂CH₂OH | O |
| A-2424 | Et | H | Me | F | CH₂C(=O)Me | O |
| A-2425 | Et | H | Me | F | CH₂CH(OH)Me | O |
| A-2426 | Et | H | Me | F | CH₂CH(OH)CF₃ | O |
| A-2427 | Et | H | Me | F | CH₂C(OH)₂CF₃ | O |
| A-2428 | Et | H | Me | F | CH₂C(CF₃)=NOH | O |
| A-2429 | Et | H | Me | F | CH₂C(CF₃)=NOMe | O |
| A-2430 | Et | H | Me | F | CH₂CH(CF₃)NH₂ | O |
| A-2431 | Et | H | Me | F | CH₂CH₂OMe | O |
| A-2432 | Et | H | Me | F | CH₂CH₂CH₂OMe | O |
| A-2433 | Et | H | Me | F | CH₂CH₂CH₂CH₂OMe | O |
| A-2434 | Et | H | Me | F | CH₂CH₂CH₂CH₂CH₂OMe | O |
| A-2435 | Et | H | Me | F | CH₂CH₂CH₂CH₂CH₂CH₂OMe | O |
| A-2436 | Et | H | Me | F | CH₂CH₂OCHF₂ | O |
| A-2437 | Et | H | Me | F | CH₂CH₂CH₂OCHF₂ | O |
| A-2438 | Et | H | Me | F | CH₂CH₂CH₂CH₂OCHF₂ | O |
| A-2439 | Et | H | Me | F | CH₂CH₂CH₂CH₂CH₂OCHF₂ | O |
| A-2440 | Et | H | Me | F | CH₂CH₂CH₂CH₂CH₂CH₂OCHF₂ | O |
| A-2441 | Et | H | Me | F | CH₂CH₂OCH₂CF₃ | O |
| A-2442 | Et | H | Me | F | CH₂CH₂CH₂OCH₂CF₃ | O |
| A-2443 | Et | H | Me | F | CH₂CH₂CH₂CH₂OCH₂CF₃ | O |
| A-2444 | Et | H | Me | F | CH₂CH₂CH₂CH₂CH₂OCH₂CF₃ | O |
| A-2445 | Et | H | Me | F | CH₂CH₂CH₂CH₂CH₂CH₂OCH₂CF₃ | O |
| A-2446 | Et | H | Me | F | CH₂CH₂CH₂CH₂CH₂CH₂CH₂OCH₂CF₃ | O |
| A-2447 | Et | H | Me | F | CH₂CH₂OC(CF₃)₃ | O |
| A-2448 | Et | H | Me | F | CH₂CH₂CH₂OC(CF₃)₃ | O |
| A-2449 | Et | H | Me | F | CH₂CH₂CH₂CH₂OC(CF₃)₃ | O |
| A-2450 | Et | H | Me | F | CH₂CH₂CH₂CH₂CH₂OC(CF₃)₃ | O |
| A-2451 | Et | H | Me | F | CF₂CHFOCF₂CF₂CF₃ | O |
| A-2452 | Et | H | Me | F | CH₂CH₂O(*c*-Pr) | O |
| A-2453 | Et | H | Me | F | CH₂CH₂O(*c*-Pen) | O |
| A-2454 | Et | H | Me | F | CH₂CH₂O(*c*-Hex) | O |
| A-2455 | Et | H | Me | F | CH₂CH₂CH₂O(*c*-Pr) | O |
| A-2456 | Et | H | Me | F | CH₂CH₂CH₂O(*c*-Pen) | O |
| A-2457 | Et | H | Me | F | CH₂CH₂CH₂O(*c*-Hex) | O |
| A-2458 | Et | H | Me | F | CH₂CH₂CH₂CH₂O(*c* -Pr) | O |

**[Table 57]**

| Compound Number | R¹ | R² | R³ | R⁴ | R⁵ | A |
|---|---|---|---|---|---|---|
| A-2459 | Et | H | Me | F | CH₂CH₂CH₂CH₂O(*c*-Pen) | O |
| A-2460 | Et | H | Me | F | CH₂CH₂CH₂CH₂O(*c*-Hex) | O |
| A-2461 | Et | H | Me | F | CH₂CH₂O[*c*-Pr(2,2-F₂)] | O |
| A-2462 | Et | H | Me | F | CH₂CH₂CH₂O[*c*-Pr(2,2-F₂)] | O |
| A-2463 | Et | H | Me | F | CH₂CH₂CH₂CH₂O[*c*-Pr(2,2-F₂)] | O |
| A-2464 | Et | H | Me | F | CH₂CH₂OCH₂CH₂OCH₃ | O |
| A-2465 | Et | H | Me | F | CH₂CH₂OCH₂CH₂OCH₂CF₃ | O |
| A-2466 | Et | H | Me | F | CH₂CF₂OCF₂CF₂OCF₃ | O |
| A-2467 | Et | H | Me | F | CF₂CHFOCF₂CF₂OCF₃ | O |
| A-2468 | Et | H | Me | F | CF₂CHFOCF₂CF(CF₃)OCF₂CF₂CF₃ | O |
| A-2469 | Et | H | Me | F | CH₂CH₂SH | O |
| A-2470 | Et | H | Me | F | CH₂CH₂CH₂SH | O |
| A-2471 | Et | H | Me | F | CH₂CH₂CH₂CH₂SH | O |
| A-2472 | Et | H | Me | F | CH₂CH₂CH₂CH₂CH₂SH | O |
| A-2473 | Et | H | Me | F | CH₂CH₂CH₂CH₂CH₂CH₂SH | O |
| A-2474 | Et | H | Me | F | CH₂CH(SH)Me | O |
| A-2475 | Et | H | Me | F | CH₂CH[SC(=O)Me]Me | O |
| A-2476 | Et | H | Me | F | CH₂CH₂S(*t*-Bu) | O |
| A-2477 | Et | H | Me | F | CH₂CH₂CH₂S(*t*-Bu) | O |
| A-2478 | Et | H | Me | F | CH₂CH₂CH₂CH₂S(*t*-Bu) | O |
| A-2479 | Et | H | Me | F | CH₂CH₂CH₂CH₂CH₂SCH₃ | O |
| A-2480 | Et | H | Me | F | CH₂CH₂CH₂CH₂CH₂SCH(CH₃)₂ | O |
| A-2481 | Et | H | Me | F | CH₂CH₂CH₂CH₂CH₂S(*t*-Bu) | O |
| A-2482 | Et | H | Me | F | CH₂CH₂CH₂CH₂CH₂CH₂SCH(CH₃)₂ | O |
| A-2483 | Et | H | Me | F | CH₂CH₂CH₂CH₂CH₂CH₂S(*t*-Bu) | O |
| A-2484 | Et | H | Me | F | CH₂CH₂CH₂CH₂CH₂CH₂CH₂S(*t*-Bu) | O |
| A-2485 | Et | H | Me | F | CH₂CH₂SCHF₂ | O |
| A-2486 | Et | H | Me | F | CH₂CH₂CH₂SCHF₂ | O |
| A-2487 | Et | H | Me | F | CH₂CH₂CH₂CH₂SCHF₂ | O |
| A-2488 | Et | H | Me | H | CH₂CH₂CH₂CH₂CH₂SCHF₂ | O |
| A-2489 | Et | H | Me | F | CH₂CH₂CH₂CH₂CH₂SCHF₂ | O |
| A-2490 | Et | H | Me | F | CH₂CH₂CH₂CH₂CH₂CH₂SCHF₂ | O |
| A-2491 | Et | H | Me | F | CH₂CH₂CH₂CH₂CH₂CH₂CH₂SCHF₂ | O |
| A-2492 | Et | H | Me | F | CH₂CH₂SCH₂CF₃ | O |
| A-2493 | Et | H | Me | F | CH₂CH₂CH₂SCH₂CF₃ | O |
| A-2494 | Et | H | Me | F | CH₂CH₂CH₂CH₂SCH₂CF₃ | O |
| A-2495 | Et | H | Me | F | CH₂CH₂CH₂CH₂CH₂SCH₂CF₃ | O |
| A-2496 | Et | H | Me | F | CH₂CH₂CH₂CH₂CH₂CH₂SCH₂CF₃ | O |
| A-2497 | Et | H | Me | F | CH₂CH₂CH₂CH₂CH₂CH₂CH₂SCH₂CF₃ | O |
| A-2498 | Et | H | Me | F | CH₂CH(Me)SCH₂CF₃ | O |
| A-2499 | Et | H | Me | F | CH₂CH₂SCH(CF₃)₂ | O |
| A-2500 | Et | H | Me | F | CH₂CH₂CH₂SCH(CF₃)₂ | O |
| A-2501 | Et | H | Me | F | CH₂CH₂CH₂CH₂SCH(CF₃)₂ | O |
| A-2502 | Et | H | Me | F | CH₂CH₂CH₂CH₂CH₂SCH(CF₃)₂ | O |

**[Table 58]**

| Compound Number | R¹ | R² | R³ | R⁴ | R⁵ | A |
|---|---|---|---|---|---|---|
| A-2503 | Et | H | Me | F | CH₂CH₂CH₂CH₂CH₂CH₂SCH(CF₃)₂ | O |
| A-2504 | Et | H | Me | F | CH₂CH₂CH₂CH₂CH₂SCF₂CF₃ | O |
| A-2505 | Et | H | Me | F | CH₂CH₂CH₂CH₂CH₂CH₂SCF₂CF₃ | O |
| A-2506 | Et | H | Me | F | CH₂CH₂CH₂CH₂CH₂CH₂SCF(CF₃)₂ | O |
| A-2507 | Et | H | Me | F | CH₂CH₂CH₂CH₂CH₂CH₂CH₂SCF(CF₃)₂ | O |
| A-2508 | Et | H | Me | F | CH₂CH₂CH₂CH₂CH₂SCCl₃ | O |
| A-2509 | Et | H | Me | F | CH₂CH₂CH₂CH₂CH₂CH₂SCCl₃ | O |
| A-2510 | Et | H | Me | F | CH₂CH₂SCH₂CH=CH₂ | O |
| A-2511 | Et | H | Me | F | CH₂CH₂CH₂SCH₂CH=CH₂ | O |
| A-2512 | Et | H | Me | F | CH₂CH₂CH₂CH₂SCH₂CH=CH₂ | O |
| A-2513 | Et | H | Me | F | CH₂CH₂SCF=CFCF₃ | O |
| A-2514 | Et | H | Me | F | CH₂CH₂CH₂SCF=CFCF₃ | O |
| A-2515 | Et | H | Me | F | CH₂CH₂CH₂CH₂SCF=CFCF₃ | O |
| A-2516 | Et | H | Me | F | CH₂CH₂CH₂S(*c*-Pr) | O |
| A-2517 | Et | H | Me | F | CH₂CH₂CH₂S(*c*-Hex) | O |
| A-2518 | Et | H | Me | F | CH₂CH₂CH₂CH₂S(*c*-Pr) | O |
| A-2519 | Et | H | Me | F | CH₂CH₂CH₂CH₂S(*c*-Hex) | O |
| A-2520 | Et | H | Me | F | CH₂CH₂CH₂CH₂CH₂S(*c*-Pr) | O |
| A-2521 | Et | H | Me | F | CH₂CH₂CH₂CH₂CH₂S(*c*-Hex) | O |
| A-2522 | Et | H | Me | F | CH₂CH₂CH₂S[*c*-Hex(4,4-F₂)] | O |
| A-2523 | Et | H | Me | F | CH₂CH₂CH₂CH₂S[*c*-Hex(4,4-F₂)] | O |
| A-2524 | Et | H | Me | F | CH₂CH₂CH₂CH₂CH₂S[*c*-Hex(4,4-F₂)] | O |
| A-2525 | Et | H | Me | F | CH₂CH₂CH₂SCH₂(*c*-Pr) | O |
| A-2526 | Et | H | Me | F | CH₂CH₂CH₂SCH₂(*c*-Hex) | O |
| A-2527 | Et | H | Me | F | CH₂CH₂CH₂CH₂SCH₂(*c*-Pr) | O |
| A-2528 | Et | H | Me | F | CH₂CH₂CH₂CH₂SCH₂(*c*-Hex) | O |
| A-2529 | Et | H | Me | F | CH₂CH₂CH₂CH₂CH₂SCH₂(*c*-Pr) | O |
| A-2530 | Et | H | Me | F | CH₂CH₂CH₂CH₂CH₂SCH₂(*c*-Hex) | O |
| A-2531 | Et | H | Me | F | CH₂CH₂CH₂SCH₂[*c*-Hex(4,4-F₂)] | O |
| A-2532 | Et | H | Me | F | CH₂CH₂CH₂CH₂SCH₂[*c*-Hex(4,4-F₂)] | O |
| A-2533 | Et | H | Me | F | CH₂CH₂CH₂CH₂CH₂SCH₂[*c*-Hex(4,4-F₂)] | O |
| A-2534 | Et | H | Me | F | CH₂CH₂CH₂SCH₂SiMe₃ | O |
| A-2535 | Et | H | Me | F | CH₂CH₂CH₂CH₂SCH₂SiMe₃ | O |
| A-2536 | Et | H | Me | F | CH₂CH₂CH₂CH₂CH₂SCH₂SiMe₃ | O |
| A-2537 | Et | H | Me | F | CH₂CH₂OCH₂CH₂SCH₃ | O |
| A-2538 | Et | H | Me | F | CH₂CH₂OCH₂CH₂SCHF₂ | O |
| A-2539 | Et | H | Me | F | CH₂CH₂OCH₂CH₂SCF₃ | O |
| A-2540 | Et | H | Me | F | CH₂CH₂OCH₂CH₂SCH₂CF₃ | O |
| A-2541 | Et | H | Me | F | CH₂CH₂CH₂S(=O)(*t* -Bu) | O |
| A-2542 | Et | H | Me | F | CH₂CH₂CH₂CH₂S(=O)(*t*-Bu) | O |
| A-2543 | Et | H | Me | F | CH₂CH₂CH₂CH₂CH₂CH₂S(=O)(*t*-Bu) | O |
| A-2544 | Et | H | Me | F | CH₂CH₂S(=O)CHF₂ | O |
| A-2545 | Et | H | Me | F | CH₂CH₂CH₂S(=O)CHF₂ | O |
| A-2546 | Et | H | Me | F | CH₂CH₂CH₂CH₂S(=O)CHF₂ | O |

**[Table 59]**

| Compound Number | R¹ | R² | R³ | R⁴ | R⁵ | A |
|---|---|---|---|---|---|---|
| A-2547 | Et | H | Me | F | CH₂CH₂CH₂CH₂CH₂S(=O)CHF₂ | O |
| A-2548 | Et | H | Me | F | CH₂CH₂CH₂CH₂CH₂CH₂S(=O)CHF₂ | O |
| A-2549 | Et | H | Me | F | CH₂CH₂CH₂CH₂CH₂CH₂CH₂S(=O)CHF₂ | O |
| A-2550 | Et | H | Me | F | CH₂CH₂S(=O)CF₃ | O |
| A-2551 | Et | H | Me | F | CH₂CH₂CH₂S(=O)CF₃ | O |
| A-2552 | Et | H | Me | F | CH₂CH₂CH₂CH₂S(=O)CF₃ | O |
| A-2553 | Et | H | Me | F | CH₂CH₂CH₂CH₂CH₂S(=O)CF₃ | O |
| A-2554 | Et | H | Me | F | CH₂CH₂CH₂CH₂CH₂CH₂S(=O)CF₃ | O |
| A-2555 | Et | H | Me | F | CH₂CH₂CH₂CH₂CH₂CH₂CH₂S(=O)CF₃ | O |
| A-2556 | Et | H | Me | F | CH₂CH₂S(=O)CH₂CF₃ | O |
| A-2557 | Et | H | Me | F | CH₂CH₂CH₂S(=O)CH₂CF₃ | O |
| A-2558 | Et | H | Me | F | CH₂CH₂CH₂CH₂S(=O)CH₂CF₃ | O |
| A-2559 | Et | H | Me | F | CH₂CH₂CH₂CH₂CH₂S(=O)CH₂CF₃ | O |
| A-2560 | Et | H | Me | F | CH₂CH₂CH₂CH₂CH₂CH₂S(=O)CH₂CF₃ | O |
| A-2561 | Et | H | Me | F | CH₂CH₂CH₂CH₂CH₂CH₂CH₂S(=O)CH₂CF₃ | O |
| A-2562 | Et | H | Me | F | CH₂CH(Me)S(=O)CH₂CF₃ | O |
| A-2563 | Et | H | Me | F | CH₂CH₂CH₂S(=O)(*c*-Pr) | O |
| A-2564 | Et | H | Me | F | CH₂CH₂CH₂CH₂S(=O)(*c*-Pr) | O |
| A-2565 | Et | H | Me | F | CH₂CH₂CH₂CH₂S(=O)(*c*-Pen) | O |
| A-2566 | Et | H | Me | F | CH₂CH₂CH₂CH₂S(=O)(*c*-Hex) | O |
| A-2567 | Et | H | Me | F | CH₂CH₂CH₂CH₂CH₂S(=O)(*c*-Pr) | O |
| A-2568 | Et | H | Me | F | CH₂CH₂CH₂CH₂CH₂S(=O)(*c*-Hex) | O |
| A-2569 | Et | H | Me | F | CH₂CH₂CH₂S(=O)[*c*-Hex(4,4-F₂)] | O |
| A-2570 | Et | H | Me | F | CH₂CH₂CH₂CH₂S(=O)[*c*-Hex(4,4-F₂)] | O |
| A-2571 | Et | H | Me | F | CH₂CH₂CH₂CH₂CH₂S(=O)[*c*-Hex(4,4-F₂)] | O |
| A-2572 | Et | H | Me | F | CH₂CH₂CH₂CH₂S(=O)₂Me | O |
| A-2573 | Et | H | Me | F | CH₂CH₂CH₂CH₂CH₂S(=O)₂Me | O |
| A-2574 | Et | H | Me | F | CH₂CH₂CH₂CH₂CH₂S(=O)₂(*t*-Bu) | O |
| A-2575 | Et | H | Me | F | CH₂CH₂S(=O)₂CHF₂ | O |
| A-2576 | Et | H | Me | F | CH₂CH₂CH₂S(=O)₂CHF₂ | O |
| A-2577 | Et | H | Me | F | CH₂CH₂CH₂CH₂S(=O)₂CHF₂ | O |
| A-2578 | Et | H | Me | F | CH₂CH₂CH₂CH₂CH₂S(=O)₂CHF₂ | O |
| A-2579 | Et | H | Me | F | CH₂CH₂CH₂CH₂CH₂CH₂S(=O)₂CHF₂ | O |
| A-2580 | Et | H | Me | F | CH₂CH₂S(=O)₂CF₃ | O |
| A-2581 | Et | H | Me | F | CH₂CH₂CH₂S(=O)₂CF₃ | O |
| A-2582 | Et | H | Me | F | CH₂CH₂CH₂CH₂S(=O)₂CF₃ | O |
| A-2583 | Et | H | Me | F | CH₂CH₂CH₂CH₂CH₂S(=O)₂CF₃ | O |
| A-2584 | Et | H | Me | F | CH₂CH₂CH₂CH₂CH₂CH₂S(=O)₂CF₃ | O |
| A-2585 | Et | H | Me | F | CH₂CH₂CH₂CH₂CH₂CH₂CH₂S(=O)₂CF₃ | O |
| A-2586 | Et | H | Me | F | CH₂CH₂CH₂CH₂CH₂CH₂CH₂CH₂S(=O)₂CF₃ | O |
| A-2587 | Et | H | Me | F | CH₂CH₂S(=O)₂CH₂CF₃ | O |
| A-2588 | Et | H | Me | F | CH₂CH₂CH₂S(=O)₂CH₂CF₃ | O |
| A-2589 | Et | H | Me | F | CH₂CH₂CH₂CH₂S(=O)₂CH₂CF₃ | O |
| A-2590 | Et | H | Me | F | CH₂CH₂CH₂CH₂CH₂S(=O)₂CH₂CF₃ | O |

**[Table 60]**

| Compound Number | R¹ | R² | R³ | R⁴ | R⁵ | A |
|---|---|---|---|---|---|---|
| A-2591 | Et | H | Me | F | CH₂CH₂CH₂CH₂CH₂CH₂S(=O)₂CH₂CF₃ | O |
| A-2592 | Et | H | Me | F | CH₂CH(Me)S(=O)₂CH₂CF₃ | O |
| A-2593 | Et | H | Me | F | CH₂CH₂CH₂S(=O)₂(*c*-pr) | O |
| A-2594 | Et | H | Me | F | CH₂CH₂CH₂CH₂S(=O)₂(*c*-Pr) | O |
| A-2595 | Et | H | Me | F | CH₂CH₂CH₂CH₂CH₂S(=O)₂(*c*-Pr) | O |
| A-2596 | Et | H | Me | F | CH₂CH₂CH₂S(=O)₂[*c*-Hex(4,4-F₂)] | O |
| A-2597 | Et | H | Me | F | CH₂CH₂CH₂CH₂S(=O)₂[*c*-Hex(4,4-F₂)] | O |
| A-2598 | Et | H | Me | F | CH₂CH₂CH₂CH₂CH₂S(=O)₂[*c*-Hex(4,4-F₂)] | O |
| A-2599 | Et | H | Me | F | CH₂CH₂CH₂C(=O)H | O |
| A-2600 | Et | H | Me | F | CH₂CH₂CH₂CH₂C(=O)H | O |
| A-2601 | Et | H | Me | F | CH₂CH₂CH₂CH₂CH₂C(=O)H | O |
| A-2602 | Et | H | Me | F | CH₂CH₂CH₂C(=O)(*t*-Bu) | O |
| A-2603 | Et | H | Me | F | CH₂CH₂CH₂CH₂C(=O)(*t*-Bu) | O |
| A-2604 | Et | H | Me | F | CH₂CH₂CH₂CH₂CH₂C(=O)(*t*-Bu) | O |
| A-2605 | Et | H | Me | F | CH₂CH₂CH₂C(=O)CF₃ | O |
| A-2606 | Et | H | Me | F | CH₂CH₂CH₂CH₂C(=O)CF₃ | O |
| A-2607 | Et | H | Me | F | CH₂CH₂CH₂CH₂CH₂C(=O)CF₃ | O |
| A-2608 | Et | H | Me | F | CH₂C(=O)Ph | O |
| A-2609 | Et | H | Me | F | CH₂C(=O)Ph(4-Cl) | O |
| A-2610 | Et | H | Me | F | CH₂C(=O)Ph(4-CF₃) | O |
| A-2611 | Et | H | Me | F | CH₂CH₂C(=O)Ph(4-CF₃) | O |
| A-2612 | Et | H | Me | F | CH₂CH₂CH₂C(=O)Ph(4-CF₃) | O |
| A-2613 | Et | H | Me | F | CH₂CH₂CH₂CH₂C(=O)Ph(4-CF₃) | O |
| A-2614 | Et | H | Me | F | CH₂CH₂CH₂CH₂CH₂C(=O)Ph(4-CF₃) | O |
| A-2615 | Et | H | Me | F | CH₂C(=O)OEt | O |
| A-2616 | Et | H | Me | F | CH₂CH₂C(=O)O(*t*-Bu) | O |
| A-2617 | Et | H | Me | F | CH₂CH₂CH₂C(=O)OEt | O |
| A-2618 | Et | H | Me | F | CH₂CH₂CH₂C(=O)O(*t-*Bu) | O |
| A-2619 | Et | H | Me | F | CH₂CH₂CH₂CH₂C(=O)O(*t*-Bu) | O |
| A-2620 | Et | H | Me | F | CH₂CH₂CH₂CH₂CH₂C(=O)O(*t*-Bu) | O |
| A-2621 | Et | H | Me | F | CH₂CH₂CH₂CH₂CH₂CH₂C(=O)O(*t*-Bu) | O |
| A-2622 | Et | H | Me | F | CH₂CH₂CH₂CH₂CH₂CH₂CH₂C(=O)OEt | O |
| A-2623 | Et | H | Me | F | CH₂CH₂C(=O)OCH₂CF₃ | O |
| A-2624 | Et | H | Me | F | CH₂CH₂CH₂C(=O)OCH₂CF₃ | O |
| A-2625 | Et | H | Me | F | CH₂CH₂CH₂CH₂C(=O)OCH₂CF₃ | O |
| A-2626 | Et | H | Me | F | CH₂CH₂CH₂CH₂CH₂C(=O)OCH₂CF₃ | O |
| A-2627 | Et | H | Me | F | CH₂CH₂C(=O)NH(*t*-Bu) | O |
| A-2628 | Et | H | Me | F | CH₂CH₂CH₂C(=O)NH(*t*-Bu) | O |
| A-2629 | Et | H | Me | F | CH₂CH_{2C}H₂C(=O)NH(*t*-Pen) | O |
| A-2630 | Et | H | Me | F | CH₂CH₂CH₂CH₂C(=O)NH(*t*-Bu) | O |
| A-2631 | Et | H | Me | F | CH₂CH₂C(=O)NHCH₂CF₃ | O |
| A-2632 | Et | H | Me | F | CH₂CH₂CH₂C(=O)NHCH₂CF₃ | O |
| A-2633 | Et | H | Me | F | CH₂CH₂CH₂CH₂C(=O)NHCH₂CF₃ | O |
| A-2634 | Et | H | Me | F | CH₂CH₂SC(=O)N(Me)₂ | O |

**[Table 61]**

| Compound Number | R¹ | R² | R³ | R⁴ | R⁵ | A |
|---|---|---|---|---|---|---|
| A-2635 | Et | H | Me | F | CH₂CH₂CH₂SC(=O)N(Me)₂ | O |
| A-2636 | Et | H | Me | F | CH₂CH₂CH₂SC(=O)NH(*t*-Bᵤ) | O |
| A-2637 | Et | H | Me | F | CH₂CH₂CH₂CH₂CH₂SC(=O)NH(*t*-Bu) | O |
| A-2638 | Et | H | Me | F | CH₂CH₂SC(=O)NHCH₂CF₃ | O |
| A-2639 | Et | H | Me | F | CH₂CH₂CH₂SC(=O)NHCH₂CF₃ | O |
| A-2640 | Et | H | Me | F | CH₂CH₂CH₂CH₂SC(=O)NHCH₂CF₃ | O |
| A-2641 | Et | H | Me | F | CH₂CH₂CH₂OC(=O)H | O |
| A-2642 | Et | H | Me | F | CH₂CH₂CH₂CH₂OC(=O)H | O |
| A-2643 | Et | H | Me | F | CH₂CH₂CH₂CH₂CH₂OC(=O)H | O |
| A-2644 | Et | H | Me | F | CH₂CH₂CH₂OC(=O)Me | O |
| A-2645 | Et | H | Me | F | CH₂CH₂CH₂CH₂OC(=O)Me | O |
| A-2646 | Et | H | Me | F | CH₂CH₂CH₂CH₂CH₂OC(=O)Me | O |
| A-2647 | Et | H | Me | F | CH₂CH₂OC(=O)CF₃ | O |
| A-2648 | Et | H | Me | F | CH₂CH₂CH₂0C(=O)CF₃ | O |
| A-2649 | Et | H | Me | F | CH₂CH₂CH₂CH₂OC(=O)CF₃ | O |
| A-2650 | Et | H | Me | F | CH₂CH₂CH₂CH₂CH₂OC(=O)Ph(4-CF₃) | O |
| A-2651 | Et | H | Me | F | CH₂CH₂OC(=O)Ph | O |
| A-2652 | Et | H | Me | F | CH₂CH₂OC(=O)Ph(4-CF₃) | O |
| A-2653 | Et | H | Me | F | CH₂CH₂CH₂OC(=O)Ph(4-CF₃) | O |
| A-2654 | Et | H | Me | F | CH₂CH₂CH₂CH₂OC(=O)Ph(4-CF₃) | O |
| A-2655 | Et | H | Me | F | CH₂CH₂CH₂CH₂CH₂OC(=O)Ph(4-CF₃) | O |
| A-2656 | Et | H | Me | F | CH₂CH₂OS(=O)Me | O |
| A-2657 | Et | H | Me | F | CH₂CH₂CH₂OS(=O)Me | O |
| A-2658 | Et | H | Me | F | CH₂CH₂CH₂CH₂OS(=O)Me | O |
| A-2659 | Et | H | Me | F | CH₂CH₂CH₂CH₂CH₂OS(=O)Me | O |
| A-2660 | Et | H | Me | F | CH₂CH₂CH₂CH₂CH₂CH₂OS(=O)Me | O |
| A-2661 | Et | H | Me | F | CH₂CH₂OS(=O)CF₃ | O |
| A-2662 | Et | H | Me | F | CH₂CH₂CH₂OS(=O)CF₃ | O |
| A-2663 | Et | H | Me | F | CH₂CH₂CH₂CH₂OS(=O)CF₃ | O |
| A-2664 | Et | H | Me | F | CH₂CH₂CH₂CH₂CH₂OS(=O)CF₃ | O |
| A-2665 | Et | H | Me | F | CH₂CH₂CH₂CH₂CH₂CH₂OS(=O)CF₃ | O |
| A-2666 | Et | H | Me | F | CH₂CH₂OS(=O)Ph | O |
| A-2667 | Et | H | Me | F | CH₂CH₂OS(=O)Ph(4-Me) | O |
| A-2668 | Et | H | Me | F | CH₂CH₂CH₂OS(=O)Ph | O |
| A-2669 | Et | H | Me | F | CH₂CH₂CH₂OS(=O)Ph(4-Me) | O |
| A-2670 | Et | H | Me | F | CH₂CH₂OS(=O)₂Me | O |
| A-2671 | Et | H | Me | F | CH₂CH₂CH₂OS(=O)₂Me | O |
| A-2672 | Et | H | Me | F | CH₂CH₂CH₂OS(=O)₂CF₃ | O |
| A-2673 | Et | H | Me | F | CH₂CH₂CH₂CH₂OS(=O)₂CF₃ | O |
| A-2674 | Et | H | Me | F | CH₂CH₂CH₂CH₂CH₂OS(=O)₂CF₃ | O |
| A-2675 | Et | H | Me | F | CH₂CH₂CH₂CH₂CH₂OS(=O)₂CF₂CF₂CF₂CF₃ | O |
| A-2676 | Et | H | Me | F | CH₂CH₂OS(=O)₂Ph | O |
| A-2677 | Et | H | Me | F | CH₂CH₂OS(=O)₂Ph(4-Me) | O |
| A-2678 | Et | H | Me | F | CH₂CH₂CH₂OS(=O)₂Ph | O |
| | | | | | | |

**[Table 62]**

| Compound Number | R¹ | R² | R³ | R⁴ | R⁵ | A |
|---|---|---|---|---|---|---|
| A-2679 | Et | H | Me | F | CH₂CH₂CH₂OS(=O)₂Ph(4-Me) | O |
| A-2680 | Et | H | Me | F | CH₂CH₂NH₂ | O |
| A-2681 | Et | H | Me | F | CH₂CH₂CH₂NH₂ | O |
| A-2682 | Et | H | Me | F | CH₂CH₂CH₂CH₂NH₂ | O |
| A-2683 | Et | H | Me | F | CH₂CH₂CH₂CH₂CH₂NH₂ | O |
| A-2684 | Et | H | Me | F | CH₂CH₂CH₂CH₂CH₂CH₂NH₂ | O |
| A-2685 | Et | H | Me | F | CH₂CH₂CH₂CH₂CH₂CH₂CH₂NH₂ | O |
| A-2686 | Et | H | Me | F | CH₂CH(Me)NH₂ | O |
| A-2687 | Et | H | Me | F | CH₂C*H(Me)NH₂: (R) | O |
| A-2688 | Et | H | Me | F | CH₂C*H(Me)NH₂: (S) | O |
| A-2689 | Et | H | Me | F | CH₂CH(Et)NH₂ | O |
| A-2690 | Et | H | Me | F | CH₂CH(*i*-Pr)NH₂ | O |
| A-2691 | Et | H | Me | F | CH(Me)CH₂NH₂ | O |
| A-2692 | Et | H | Me | F | CH(Et)CH₂NH₂ | O |
| A-2693 | Et | H | Me | F | CH(*i*-Pr)CH₂NH₂ | O |
| A-2694 | Et | H | Me | F | CH₂CH₂CH(Me)NH₂ | O |
| A-2695 | Et | H | Me | F | CH₂CH(Me)CH₂NH₂ | O |
| A-2696 | Et | H | Me | F | CH(Me)CH₂CH₂NH₂ | O |
| A-2697 | Et | H | Me | F | CH₂CH(Me)NH(*c*-Pᵣ) | O |
| A-2698 | Et | H | Me | F | CH_{2CH}(Me)NHOMe | O |
| A-2699 | Et | H | Me | F | CH₂CH(Me)N(OMe)S(=O)₂Ph(2-NO₂) | O |
| A-2700 | Et | H | Me | F | CH₂CH(Me)N(OMe)S(=O)₂Ph(4-NO₂) | O |
| A-2701 | Et | H | Me | F | CH₂CH₂N(Me)(t-Bu) | O |
| A-2702 | Et | H | Me | F | CH₂CH₂CH₂N(Me)(*t*-Bu) | O |
| A-2703 | Et | H | Me | F | CH₂CH₂CH₂CH₂N(Me)(*t*-Bu) | O |
| A-2704 | Et | H | Me | F | CH₂CH₂NHCH₂CF₃ | O |
| A-2705 | Et | H | Me | F | CH₂CH₂CH₂NHCH₂CF₃ | O |
| A-2706 | Et | H | Me | F | CH₂CH₂CH₂CH₂NHCH₂CF₃ | O |
| A-2707 | Et | H | Me | F | CH₂CH₂NHC(=O)C(Me)(CF₃)₂ | O |
| A-2708 | Et | H | Me | F | CH₂CH₂NHC(=O)Me | O |
| A-2709 | Et | H | Me | F | CH₂CH₂NHC(=O)(*t*-Bu) | O |
| A-2710 | Et | H | Me | F | CH₂CH₂CH₂CH₂NHC(=O)CH(CH₃)₂ | O |
| A-2711 | Et | H | Me | F | CH₂CH₂CH₂CH₂NHC(=O)(*t*-Bu) | O |
| A-2712 | Et | H | Me | F | CH₂CH₂CH₂CH₂NHC(=O)CH₂(*t*-Bu) | O |
| A-2713 | Et | H | Me | F | CH₂CH₂NHC(=O)CF₃ | O |
| A-2714 | Et | H | Me | F | CH₂CH₂NHC(=O)CH₂CF₃ | O |
| A-2715 | Et | H | Me | F | CH₂CH₂NHC(=O)CF₂CF₃ | O |
| A-2716 | Et | H | Me | F | CH₂CH₂CH₂NHC(=O)CF₃ | O |
| A-2717 | Et | H | Me | F | CH₂CH₂CH₂NHC(=O)CH₂CF₃ | O |
| A-2718 | Et | H | Me | F | CH₂CH₂CH₂NHC(=O)CF₂CF₃ | O |
| A-2719 | Et | H | Me | F | CH₂CH₂CH₂CH₂NHC(=O)CF₃ | O |
| A-2720 | Et | H | Me | F | CH₂CH₂CH₂CH₂NHC(=O)CH₂CF₃ | O |
| A-2721 | Et | H | Me | F | CH₂CH₂CH₂CH₂NHC(=O)CF₂CF₃ | O |
| A-2722 | Et | H | Me | F | CH₂CH₂CH₂CH₂NHC(=O)CF(CF₃)₂ | O |

**[Table 63]**

| Compound Number | R¹ | R² | R³ | R⁴ | R⁵ | A |
|---|---|---|---|---|---|---|
| A-2723 | Et | H | Me | F | CH₂CH₂CH₂CH₂NHC(=O)CCl₃ | O |
| A-2724 | Et | H | Me | F | CH₂CH(Me)NHC(=O)CF₃ | O |
| A-2725 | Et | H | Me | F | CH₂CH₂NHC(=O)Ph | O |
| A-2726 | Et | H | Me | F | CH₂CH₂NHC(=O)Ph(4-CF₃) | O |
| A-2727 | Et | H | Me | F | CH₂CH₂CH₂NHC(=O)Ph(4-CF₃) | O |
| A-2728 | Et | H | Me | F | CH₂CH₂r-H₂CH₂NHC(=O)Ph(4-CF₃) | O |
| A-2729 | Et | H | Me | F | CH₂CH₂NHC(=O)O(*t*-Bu) | O |
| A-2730 | Et | H | Me | F | CH₂CH₂CH₂NHC(=O)O(*t*-Bu) | O |
| A-2731 | Et | H | Me | F | CH₂CH₂CH₂CH₂NHC(=O)O(*t*-Bu) | O |
| A-2732 | Et | H | Me | F | CH₂CH₂CH₂CH₂NHC(=O)OCH(CH₃)₂ | O |
| A-2733 | Et | H | Me | F | CH₂CH(Me)NHC(=O)O(*t*-Bu) | O |
| A-2734 | Et | H | Me | F | CH₂C*H(Me)NHC(=O)O(*t*-Bu) : (R) | O |
| A-2735 | Et | H | Me | F | CH₂C*H(Me)NHC(=O)O(*t*-Bu) : (S) | O |
| A-2736 | Et | H | Me | F | CH₂CH(Et)NHC(=O)O(*t*-Bu) | O |
| A-2737 | Et | H | Me | F | CH₂CH(*i*-Pr)NHC(=O)O(*t*-Bu) | O |
| A-2738 | Et | H | Me | F | CH(Me)CH₂NHC(=O)O(*t*-Bu) | O |
| A-2739 | Et | H | Me | F | CH(Et)CH₂NHC(=O)O(*t*-Bu) | O |
| A-2740 | Et | H | Me | F | CH(*i*-Pr)CH₂NHC(=O)O(*t*-Bu) | O |
| A-2741 | Et | H | Me | F | CH₂CH(Me)CH₂NHC(=O)O(*t*-Bu) | O |
| A-2742 | Et | H | Me | F | CH₂CH₂NHC(=O)OC(Me)₂CF₃ | O |
| A-2743 | Et | H | Me | F | CH₂CH₂CH₂NHC(=O)OCH₂CF₃ | O |
| A-2744 | Et | H | Me | F | CH₂CH₂CH₂NHC(=O)OCH₂CH₂CF₃ | O |
| A-2745 | Et | H | Me | F | CH₂CH₂CH₂CH₂NH_{C}(=O)OCH₂CCl₃ | O |
| A-2746 | Et | H | Me | F | CH₂CH₂CH₂CH₂NHC(=O)OCH₂CF₃ | O |
| A-2747 | Et | H | Me | F | CH₂CH₂NHC(=O)NH(*t*-Bu) | O |
| A-2748 | Et | H | Me | F | CH₂CH₂CH₂NHC(=O)NH(*t*-Bu) | O |
| A-2749 | Et | H | Me | F | CH₂CH₂CH₂CH₂NHC(=O)NHEt | O |
| A-2750 | Et | H | Me | F | CH₂CH₂CH₂CH₂NHC(=O)NH(*t*-Bu) | O |
| A-2751 | Et | H | Me | F | CH₂CH₂CH₂NHC(=O)NHCH₂CCl₃ | O |
| A-2752 | Et | H | Me | F | CH₂CH₂CH₂NHC(=O)NHCH₂CH₂F | O |
| A-2753 | Et | H | Me | F | CH₂CH₂CH₂NHC(=O)NHCH₂CF₃ | O |
| A-2754 | Et | H | Me | F | CH₂CH₂CH₂CH₂NHC(=O)NHCH₂CCl₃ | O |
| A-2755 | Et | H | Me | F | CH₂CH₂CH₂CH₂NHC(=O)NHCH₂CH₂F | O |
| A-2756 | Et | H | Me | F | CH₂CH₂CH₂CH₂NHC(=O)NHCH₂CF₃ | O |
| A-2757 | Et | H | Me | F | CH₂CH₂NHS(=O)Me | O |
| A-2758 | Et | H | Me | F | CH₂CH₂CH₂NHS(=O)Me | O |
| A-2759 | Et | H | Me | F | CH2CH₂CH₂CH₂NHS(=O)Me | O |
| A-2760 | Et | H | Me | F | CH₂CH₂CH₂CH₂NHS(=O)CH(CH₃)₂ | O |
| A-2761 | Et | H | Me | F | CH₂CH₂CH₂CH₂CH₂NHS(=O)Me | O |
| A-2762 | Et | H | Me | F | CH₂CH₂NHS(=O)CHF₂ | O |
| A-2763 | Et | H | Me | F | CH₂CH₂CH₂NHS(=O)CHF₂ | O |
| A-2764 | Et | H | Me | F | CH₂CH₂CH₂CH₂NHS(=O)CHF₂ | O |
| A-2765 | Et | H | Me | F | CH₂CH₂CH₂CH₂CH₂NHS(=O)CHF₂ | O |
| A-2766 | Et | H | Me | F | CH₂CH(Me)NHS(=O)CHF₂ | O |

**[Table 64]**

| Compound Number | R¹ | R² | R³ | R⁴ | R⁵ | A |
|---|---|---|---|---|---|---|
| A-2767 | Et | H | Me | F | CH(Me)CH₂NHS(=O)CHF₂ | O |
| A-2768 | Et | H | Me | F | CH₂CH₂CH(Me)NHS(=O)CHF₂ | O |
| A-2769 | Et | H | Me | F | CH₂CH(Me)CH₂NHS(=O)CHF₂ | O |
| A-2770 | Et | H | Me | F | CH(Me)CH₂CH₂NHS(=O)CHF₂ | O |
| A-2771 | Et | H | Me | F | CH₂CH₂NHS(=O)CF₃ | O |
| A-2772 | Et | H | Me | F | CH₂CH₂CH₂NHS(=O)CF₃ | O |
| A-2773 | Et | H | Me | F | CH₂CH₂CH₂CH₂NHS(=O)CF₃ | O |
| A-2774 | Et | H | Me | F | CH₂CH₂CH₂CH₂CH₂NHS(=O)CF₃ | O |
| A-2775 | Et | H | Me | F | CH₂CH₂CH₂NHS(=O)Ph | O |
| A-2776 | Et | H | Me | F | CH₂CH₂CH₂CH₂NHS(=O)Ph | O |
| A-2777 | Et | H | Me | F | CH₂CH₂CH₂CH₂CH₂NHS(=O)Ph | O |
| A-2778 | Et | H | Me | F | CH₂CH₂CH₂CH₂CH₂NHS(=O)Ph(4-CF₃) | O |
| A-2779 | Et | H | Me | F | CH₂CH(Me)NHS(=O)Ph(4-CF₃) | O |
| A-2780 | Et | H | Me | F | CH₂CH(Et)NHS(=O)Ph(4-CF₃) | O |
| A-2781 | Et | H | Me | F | CH(Me)CH₂NHS(=O)Ph(4-CF₃) | O |
| A-2782 | Et | H | Me | F | CH(Et)CH₂NHS(=O)Ph(4-CF₃) | O |
| A-2783 | Et | H | Me | F | CH₂CH(Et)CH₂NHS(=O)Ph(4-CF₃) | O |
| A-2784 | Et | H | Me | F | CH₂CH₂NHS(=O)₂Me | O |
| A-2785 | Et | H | Me | F | CH₂CH₂CH₂NHS(=O)₂Me | O |
| A-2786 | Et | H | Me | F | CH₂CH₂CH₂CH₂NHS(=O)₂Me | O |
| A-2787 | Et | H | Me | F | CH₂CH₂CH₂CH₂NHS(=O)₂CH(CH₃)₂ | O |
| A-2788 | Et | H | Me | F | CH₂CH₂CH₂CH₂CH₂NHS(=O)₂Me | O |
| A-2789 | Et | H | Me | F | CH₂CH₂NHS(=O)₂CHF₂ | O |
| A-2790 | Et | H | Me | F | CH₂CH₂CH₂NHS(=O)₂CHF₂ | O |
| A-2791 | Et | H | Me | F | CH₂CH_{2C}H₂CH₂NHS(=O)₂CHF₂ | O |
| A-2792 | Et | H | Me | F | CH₂CH₂CH₂CH₂CH₂NHS(=O)₂CHF₂ | O |
| A-2793 | Et | H | Me | F | CH₂CH(Me)NHS(=O)₂CHF₂ | O |
| A-2794 | Et | H | Me | F | CH(Me)CH₂NHS(=O)₂CHF₂ | O |
| A-2795 | Et | H | Me | F | CH₂CH₂CH(Me)NHS(=O)₂CHF₂ | O |
| A-2796 | Et | H | Me | F | CH₂CH(Me)CH₂NHS(=O)₂CHF₂ | O |
| A-2797 | Et | H | Me | F | CH(Me)CH₂CH₂NHS(=O)₂CHF₂ | O |
| A-2798 | Et | H | Me | F | CH₂CH(Me)N(Me)S(=O)₂CHF₂ | O |
| A-2799 | Et | H | Me | F | CH(Me)CH₂N(Me)S(=O)₂CHF₂ | O |
| A-2800 | Et | H | Me | F | CH₂CH₂CH(Me)N(Me)S(=O)₂CHF₂ | O |
| A-2801 | Et | H | Me | F | CH₂CH(Me)CH₂N(Me)S(=O)₂CHF₂ | O |
| A-2802 | Et | H | Me | F | CH(Me)CH₂CH₂N(Me)S(=O)₂CHF₂ | O |
| A-2803 | Et | H | Me | F | CH₂CH₂CH₂NHS(=O)₂Ph | O |
| A-2804 | Et | H | Me | F | CH₂CH₂CH₂CH₂NHS(=O)₂Ph | O |
| A-2805 | Et | H | Me | F | CH₂CH₂CH₂CH₂CH₂NHS(=O)₂Ph | O |
| A-2806 | Et | H | Me | F | CH₂CH₂CH₂CH₂CH₂NHS(=O)₂Ph(4-CF₃) | O |
| A-2807 | Et | H | Me | F | CH₂CH(Me)NHS(=O)₂Ph(4-CF₃) | O |
| A-2808 | Et | H | Me | F | CH₂CH(Et)NHS(=O)₂Ph(4-CF₃) | O |
| A-2809 | Et | H | Me | F | CH(Me)CH₂NHS(=O)₂Ph(4-CF₃) | O |
| A-2810 | Et | H | Me | F | CH(Et)CH₂NHS(=O)₂Ph(4-CF₃) | O |

**[Table 65]**

| Compound Number | R¹ | R² | R³ | R⁴ | R5 | A |
|---|---|---|---|---|---|---|
| A-2811 | Et | H | Me | F | CH₂CH(Me)CH₂NHS(=O)₂Ph(4-CF₃) | O |
| A-2812 | Et | H | Me | F | CH₂CH₂SiMe₃ | O |
| A-2813 | Et | H | Me | F | CH₂CH₂CH₂SiMe₃ | O |
| A-2814 | Et | H | Me | F | CH₂CH₂CH₂CH₂SiMe₃ | O |
| A-2815 | Et | H | Me | F | CH₂Ph | O |
| A-2816 | Et | H | Me | F | CH₂Ph(2-CF₃) | O |
| A-2817 | Et | H | Me | F | CH₂Ph(3-CF₃) | O |
| A-2818 | Et | H | Me | F | CH₂Ph(4-CF₃) | O |
| A-2819 | Et | H | Me | F | CH₂Ph(2-OCF₃) | O |
| A-2820 | Et | H | Me | F | CH₂Ph(3-OCF₃) | O |
| A-2821 | Et | H | Me | F | CH₂Ph(4-OCF₃) | O |
| A-2822 | Et | H | Me | F | CH₂Ph(2-SCF₃) | O |
| A-2823 | Et | H | Me | F | CH₂Ph(3-SCF₃) | O |
| A-2824 | Et | H | Me | F | CH₂Ph(4-SCF₃) | O |
| A-2825 | Et | H | Me | F | CH₂Ph(3-CH₂SCF₃) | O |
| A-2826 | Et | H | Me | F | CH₂Ph(4-F) | O |
| A-2827 | Et | H | Me | F | CH₂Ph(4-Cl) | O |
| A-2828 | Et | H | Me | F | CH₂Ph(4-Br) | O |
| A-2829 | Et | H | Me | F | CH₂Ph(4-Me) | O |
| A-2830 | Et | H | Me | F | CH₂Ph[4-(*t*-Bu)] | O |
| A-2831 | Et | H | Me | F | CH₂Ph(4-CN) | O |
| A-2832 | Et | H | Me | F | CH₂Ph(4-NO₂) | O |
| A-2833 | Et | H | Me | F | CH₂Ph(4-OCHF₂) | O |
| A-2834 | Et | H | Me | F | CH₂Ph(4-SCHF₂) | O |
| A-2835 | Et | H | Me | F | CH₂Ph(4-CH₂SCF₃) | O |
| A-2836 | Et | H | Me | F | CH₂Ph[4-CF(CF₃)₂] | O |
| A-2837 | Et | H | Me | F | CH₂Ph(4-CH₂SCF₃) | O |
| A-2838 | Et | H | Me | F | CH₂Ph[4-Ph(4-CF₃)] | O |
| A-2839 | Et | H | Me | F | CH₂Ph(2,4-Cl₂) | O |
| A-2840 | Et | H | Me | F | CH₂Ph[2,5-(CF₃)₂] | O |
| A-2841 | Et | H | Me | F | CH₂Ph(3,4-Cl₂) | O |
| A-2842 | Et | H | Me | F | CH₂Ph(3-CF₃-4-F) | O |
| A-2843 | Et | H | Me | F | CH₂Ph(3-CF₃-4- Cl ) | O |
| A-2844 | Et | H | Me | F | CH₂Ph(3-F-4-CF₃) | O |
| A-2845 | Et | H | Me | F | CH₂Ph(2,4,6-F₃) | O |
| A-2846 | Et | H | Me | F | CH₂Ph(3,4,5-F₃) | O |
| A-2847 | Et | H | Me | F | CH₂Ph(2,3,4-F₃) | O |
| A-2848 | Et | H | Me | F | CH₂Ph(3,4,5-Cl₃) | O |
| A-2849 | Et | H | Me | F | CH₂CH₂Ph | O |
| A-2850 | Et | H | Me | F | CH₂CH₂Ph(4-F) | O |
| A-2851 | Et | H | Me | F | CH₂CH₂Ph(4-Cl) | O |
| A-2852 | Et | H | Me | F | CH₂CH₂Ph(4-Br) | O |
| A-2853 | Et | H | Me | F | CH₂CH₂Ph[4-(*t*-Bu)] | O |
| A-2854 | Et | H | Me | F | CH₂CH₂Ph(2-CF₃) | O |

**[Table 66]**

| Compound Number | R¹ | R² | R³ | R⁴ | R⁵ | A |
|---|---|---|---|---|---|---|
| A-2855 | Et | H | Me | F | CH₂CH₂Ph(3-CF₃) | O |
| A-2856 | Et | H | Me | H | CH₂CH₂Ph(4-CF₃) | O |
| A-2857 | Et | H | Me | F | CH₂CH₂Ph(4-CF₃) | O |
| A-2858 | Et | H | Me | F | CH₂CH₂Ph(4-CF(CF₃)₂) | O |
| A-2859 | Et | H | Me | F | CH₂CH₂Ph[4-(*c*-Pr)] | O |
| A-2860 | Et | H | Me | F | CH₂CH₂Ph{4-[*c*-Pr(2,2-F₂)]} | O |
| A-2861 | Et | H | Me | F | CH₂CH₂Ph(4-OMe) | O |
| A-2862 | Et | H | Me | F | CH₂CH₂Ph(3-OCF₃) | O |
| A-2863 | Et | H | Me | F | CH₂CH₂Ph(4-OCHF₂) | O |
| A-2864 | Et | H | Me | F | CH₂CH₂Ph(4-OCF₃) | O |
| A-2865 | Et | H | Me | F | CH₂CH₂Ph(4-SMe) | O |
| A-2866 | Et | H | Me | F | CH₂CH₂Ph(4-SCHF₂) | O |
| A-2867 | Et | H | Me | F | CH₂CH₂Ph(4-SCF₃) | O |
| A-2868 | Et | H | Me | F | CH₂CH₂Ph[4-S(=O)Me] | O |
| A-2869 | Et | H | Me | F | CH₂CH₂Ph[4-S(=O)CF₃] | O |
| A-2870 | Et | H | Me | F | CH₂CH₂Ph[4-S(=O)₂Me] | O |
| A-2871 | Et | H | Me | F | CH₂CH₂Ph[4-S(=O)₂CF₃] | O |
| A-2872 | Et | H | Me | F | CH₂CH₂Ph(4-CH₂SMe) | O |
| A-2873 | Et | H | Me | F | CH₂CH₂Ph(4-CH₂SCF₃) | O |
| A-2874 | Et | H | Me | F | CH₂CH₂Ph[4-OS(=O)₂Me] | O |
| A-2875 | Et | H | Me | F | CH₂CH₂Ph[4-OS(=O)₂CF_{3]} | O |
| A-2876 | Et | H | Me | F | CH₂CH₂Ph[4-Ph(4-CF₃)] | O |
| A-2877 | Et | H | Me | F | CH₂CH₂Ph(4-CH₂Ph) | O |
| A-2878 | Et | H | Me | F | CH₂CH₂Ph(4-OCH₂Ph) | O |
| A-2879 | Et | H | Me | F | CH₂CH₂Ph(4-CN) | O |
| A-2880 | Et | H | Me | F | CH₂CH₂Ph(4-NO₂) | O |
| A-2881 | Et | H | Me | F | CH₂CH₂Ph(2,4-Cl₂) | O |
| A-2882 | Et | H | Me | F | CH₂CH₂Ph(3,4-Cl₂) | O |
| A-2883 | Et | H | Me | F | CH₂CH₂Ph(3-CF₃-4-F) | O |
| A-2884 | Et | H | Me | F | CH₂CH₂Ph(2-CF₃-4-F) | O |
| A-2885 | Et | H | Me | F | CH₂CH₂Ph(3-F-4-CF₃) | O |
| A-2886 | Et | H | Me | F | CH₂CH₂Ph(2-F-4-CF₃) | O |
| A-2887 | Et | H | Me | F | CH₂CH₂Ph(3- Cl -4-OCHF₂) | O |
| A-2888 | Et | H | Me | F | CH₂CH₂Ph(3,4,5-Cl₃) | O |
| A-2889 | Et | H | Me | F | CH₂CH₂Ph(2,3,4-F₃) | O |
| A-2890 | Et | H | Me | F | CH₂CH₂Ph(2,4,5-F₃) | O |
| A-2891 | Et | H | Me | F | CH₂CH₂Ph(3,4,5-F₃) | O |
| A-2892 | Et | H | Me | F | CH₂CH₂Ph(2,4,6-F₃) | O |
| A-2893 | Et | H | Me | F | CH₂CH₂CH₂Ph | O |
| A-2894 | Et | H | Me | F | CH₂CH₂CH₂Ph(3-CF₃) | O |
| A-2895 | Et | H | Me | F | CH₂CH₂CH₂Ph(4-CF₃) | O |
| A-2896 | Et | H | Me | F | CH₂CH₂CH₂Ph(4-F) | O |
| A-2897 | Et | H | Me | F | CH₂CH₂CH₂Ph[4-(*t*-Bu)] | O |
| A-2898 | Et | H | Me | F | CH₂CH₂CH₂Ph(4-CN) | O |

**[Table 67]**

| Compound Number | R¹ | R² | R³ | R⁴ | R⁵ | A |
|---|---|---|---|---|---|---|
| A-2899 | Et | H | Me | F | CH₂CH₂CH₂Ph(4-CF₃) | O |
| A-2900 | Et | H | Me | F | CH₂CH₂CH₂Ph(4-OCHF₂) | O |
| A-2901 | Et | H | Me | F | CH₂CH₂CH₂Ph(4-OCF₃) | O |
| A-2902 | Et | H | Me | F | CH₂CH₂CH₂Ph(4-SCHF₂) | O |
| A-2903 | Et | H | Me | F | CH₂CH₂CH₂Ph(4-SCF₃) | O |
| A-2904 | Et | H | Me | F | 3CH₂CH₂CH₂Ph[4-CF(CF₃)₂] | O |
| A-2905 | Et | H | Me | F | CH₂CH₂CH₂Ph(3,4,5-F₃) | O |
| A-2906 | Et | H | Me | F | CH₂CH₂CH₂Ph(2,4,6-F₃) | O |
| A-2907 | Et | H | Me | F | CH₂CH₂CH₂CH₂Ph | O |
| A-2908 | Et | H | Me | F | CH₂CH₂CH₂CH₂Ph(4-F) | O |
| A-2909 | Et | H | Me | F | CH₂CH₂CH₂CH₂Ph(4-CF₃) | O |
| A-291 0 | Et | H | Me | F | CH₂CH₂CH₂CH₂Ph(4-OCF₃) | O |
| A-2911 | Et | H | Me | F | CH₂CH₂CH₂CH₂Ph(4-SCF₃) | O |
| A-2912 | Et | H | Me | F | CH₂CH₂CH₂CH₂CH₂Ph | O |
| A-2913 | Et | H | Me | F | CH₂CH₂CH₂CH₂CH₂CH₂Ph | O |
| A-2914 | Et | H | Me | F | CH₂CF₂Ph(4-F) | O |
| A-2915 | Et | H | Me | F | CH₂CF₂Ph(4-CF₃) | O |
| A-2916 | Et | H | Me | H | CH₂CF₂Ph(3,4,5-F₃) | O |
| A-2917 | Et | H | Me | F | CH₂CF₂Ph(3,4,5-F₃) | O |
| A-2918 | Et | H | Me | F | CH₂CH₂OPh | O |
| A-2919 | Et | H | Me | F | CH₂CH₂OPh(4-F) | O |
| A-2920 | Et | H | Me | F | CH₂CH₂OPh(4-CF₃) | O |
| A-2921 | Et | H | Me | F | CH₂CH₂CH₂OPh | O |
| A-2922 | Et | H | Me | F | CH₂CH₂CH₂OPh(4-Cl) | O |
| A-2923 | Et | H | Me | F | CH₂CH₂CH₂OPh(4-CF₃) | O |
| A-2924 | Et | H | Me | F | CH₂CH₂CH₂CH₂OPh(4-CF₃) | O |
| A-2925 | Et | H | Me | F | CH₂CH₂CH₂CH₂OPh(4-OCF₃) | O |
| A-2926 | Et | H | Me | F | CH₂CH₂CH₂CH₂CH₂OPh(4-CF₃) | O |
| A-2927 | Et | H | Me | F | CH₂CH₂OCH₂Ph | O |
| A-2928 | Et | H | Me | F | CH₂CH₂CH₂OCH₂Ph | O |
| A-2929 | Et | H | Me | F | CH₂CH₂CH₂CH₂OCH₂Ph | O |
| A-2930 | Et | H | Me | F | CH₂CH₂CH₂CH₂CH₂OCH₂Ph | O |
| A-2931 | Et | H | Me | F | CH₂CH₂SPh | O |
| A-2932 | Et | H | Me | F | CH₂CH₂CH₂SPh | O |
| A-2933 | Et | H | Me | F | CH₂CH₂CH₂SPh(4-F) | O |
| A-2934 | Et | H | Me | F | CH₂CH₂CH₂SPh(4-Cl) | O |
| A-2935 | Et | H | Me | F | CH₂CH₂CH₂SPh(4-Br) | O |
| A-2936 | Et | H | Me | F | CH₂CH₂CH₂SPh[4-(*t*-Bu)] | O |
| A-2937 | Et | H | Me | F | CH₂CH₂CH₂SPh(3-CF₃) | O |
| A-2938 | Et | H | Me | F | CH₂CH₂CH₂SPh(4-CF₃) | O |
| A-2939 | Et | H | Me | F | CH₂CH₂CH₂SPh(3-SCF₃) | O |
| A-2940 | Et | H | Me | F | CH₂CH₂CH₂SPh(4-SCF₃) | O |
| A-2941 | Et | H | Me | F | CH₂CH₂CH₂CH₂SPh | O |
| A-2942 | Et | H | Me | F | CH₂CH₂CH₂CH₂SPh(4-F) | O |

**[Table 68]**

| Compound Number | R¹ | R² | R³ | R⁴ | R⁵ | A |
|---|---|---|---|---|---|---|
| A-2943 | Et | H | Me | F | CH₂CH₂CH₂CH₂SPh(4-Cl) | O |
| A-2944 | Et | H | Me | F | CH₂CH₂CH₂CH₂SPh[4-(*t*-Bu)] | O |
| A-2945 | Et | H | Me | F | CH₂CH₂CH₂CH₂SPh(4-CF₃) | O |
| A-2946 | Et | H | Me | F | CH₂CH₂CH₂CH₂CH₂SPh | O |
| A-2947 | Et | H | Me | F | CH₂CH₂CH₂CH₂CH₂SPh(4-F) | O |
| A-2948 | Et | H | Me | F | CH₂CH₂CH₂CH₂CH₂SPh(4-Cl) | O |
| A-2949 | Et | H | Me | F | CH₂CH₂CH₂CH₂CH₂SPh(4-CF₃) | O |
| A-2950 | Et | H | Me | F | CH₂CH₂S(=O)Ph | O |
| A-2951 | Et | H | Me | F | CH₂CH₂CH₂S(=O)Ph | O |
| A-2952 | Et | H | Me | F | CH₂CH₂CH₂S(=O)Ph(4-F) | O |
| A-2953 | Et | H | Me | F | CH₂CH₂CH₂S(=O)Ph[4-(*t*-Bu)] | O |
| A-2954 | Et | H | Me | F | CH₂CH₂CH₂S(=O)Ph(4-CF₃) | O |
| A-2955 | Et | H | Me | F | CH₂CH₂S(=O)₂Ph | O |
| A-2956 | Et | H | Me | F | CH₂CH₂S(=O)₂Ph(4-CF₃) | O |
| A-2957 | Et | H | Me | F | CH₂CH₂CH₂S(=O)₂Ph(4-F) | O |
| A-2958 | Et | H | Me | F | CH₂CH₂CH₂S(=O)₂Ph(4-Cl) | O |
| A-2959 | Et | H | Me | F | CH₂CH₂CH₂S(=O)₂Ph(4-CF₃) | O |
| A-2960 | Et | H | Me | F | CH₂CH₂CH₂CH₂S(=O)₂Ph | O |
| A-2961 | Et | H | Me | F | CH₂CH₂CH₂CH₂S(=O)₂Ph(4-Cl) | O |
| A-2962 | Et | H | Me | F | CH₂CH₂CH₂CH₂S(=O)₂Ph(4-CF₃) | O |
| A-2963 | Et | H | Me | F | CH₂CH₂SCH₂Ph | O |
| A-2964 | Et | H | Me | F | CH₂CH₂CH₂SCH₂Ph | O |
| A-2965 | Et | H | Me | F | CH₂CH₂CH₂SCH₂Ph(2-Cl) | O |
| A-2966 | Et | H | Me | F | CH₂CH₂CH₂SCH₂Ph(3-Cl) | O |
| A-2967 | Et | H | Me | F | CH₂CH₂CH₂SCH₂Ph(4-Cl) | O |
| A-2968 | Et | H | Me | F | CH₂CH₂CH₂SCH₂Ph(2-SCF₃) | O |
| A-2969 | Et | H | Me | F | CH₂CH₂CH₂SCH₂Ph(3-CF₃) | O |
| A-2970 | Et | H | Me | F | CH₂CH₂CH₂SCH₂Ph(4-CF₃) | O |
| A-2971 | Et | H | Me | F | CH₂CH₂CH₂SCH₂Ph(4-NO₂) | O |
| A-2972 | Et | H | Me | F | CH₂CH₂CH₂CH₂SCH₂Ph | O |
| A-2973 | Et | H | Me | F | CH₂CH₂CH₂CH₂SCH₂Ph(4-Cl) | O |
| A-2974 | Et | H | Me | F | CH₂CH₂CH₂CH₂SCH₂Ph(4-CF₃) | O |
| A-2975 | Et | H | Me | F | CH₂CH₂CH₂CH₂SCH₂Ph(4-CN) | O |
| A-2976 | Et | H | Me | F | CH₂CH₂CH₂CH₂CH₂SCH₂Ph | O |
| A-2977 | Et | H | Me | F | CH₂CH₂SCH₂CH₂Ph | O |
| A-2978 | Et | H | Me | F | CH₂CH₂CH₂SCH₂CH₂Ph | O |
| A-2979 | Et | H | Me | F | CH₂CH₂CH₂CH₂SCH₂CH₂Ph | O |
| A-2980 | Et | H | Me | F | CH₂CH₂SCH(Me)Ph | O |
| A-2981 | Et | H | Me | F | CH₂CH₂CH₂SCH(Me)Ph | O |
| A-2982 | Et | H | Me | F | CH₂CH₂CH₂CH₂SCH(Me)Ph | O |
| A-2983 | Et | H | Me | F | CH₂CH₂S(=O)CH₂Ph | O |
| A-2984 | Et | H | Me | F | CH₂CH₂S(=O)CH₂Ph(4-CF₃) | O |
| A-2985 | Et | H | Me | F | CH₂CH₂CH₂S(=O)CH₂Ph | O |
| A-2986 | Et | H | Me | F | CH₂CH₂CH₂S(=O)CH₂Ph(2-SCF₃) | O |

**[Table 69]**

| Compound Number | R¹ | R² | R³ | R⁴ | R⁵ | A |
|---|---|---|---|---|---|---|
| A-2987 | Et | H | Me | F | CH₂CH₂CH₂S(=O)CH₂Ph(4-SCF₃) | O |
| A-2988 | Et | H | Me | F | CH₂CH₂CH₂S(=O)CH₂Ph(4-Cl) | O |
| A-2989 | Et | H | Me | F | CH₂CH₂CH₂S(=O)CH₂Ph(4-CF₃) | O |
| A-2990 | Et | H | Me | F | CH₂CH₂CH₂CH₂S(=O)CH₂Ph | O |
| A-2991 | Et | H | Me | F | CH₂CH₂CH₂CH₂S(=O)CH₂Ph(4-CF₃) | O |
| A-2992 | Et | H | Me | F | CH₂CH₂CH₂CH₂CH₂S(=O)CH₂Ph(4-CF₃) | O |
| A-2993 | Et | H | Me | F | CH₂CH₂S(=)₂CH₂Ph | O |
| A-2994 | Et | H | Me | F | CH₂CH₂S(=O)₂CH₂Ph(4-CF₃) | O |
| A-2995 | Et | H | Me | F | CH₂CH₂CH₂S(=O)₂CH₂Ph | O |
| A-2996 | Et | H | Me | F | CH₂CH₂CH₂S(=O)₂CH₂Ph(4-Cl) | O |
| A-2997 | Et | H | Me | F | CH₂CH₂CH₂S(=O)2CH₂Ph(4-CF3) | O |
| A-2998 | Et | H | Me | F | CH₂CH₂CH₂CH₂S(=O)₂CH₂Ph | O |
| A-2999 | Et | H | Me | F | CH₂CH₂CH₂CH₂S(=O)₂CH₂Ph(4-CF₃) | O |
| A-3000 | Et | H | Me | F | CH₂CH₂CH₂CH₂CH₂S(=O)₂CH₂Ph | O |
| A-3001 | Et | H | Me | F | CH₂CH₂CH₂CH₂CH₂S(=O)₂CH₂Ph(4-CF₃) | O |
| A-3002 | Et | H | Me | F | CH₂CH₂ON=CH(*t*-Bᵤ) | O |
| A-3003 | Et | H | Me | F | CH₂CH₂CH₂ON=CH(*t*-Bu) | O |
| A-3004 | Et | H | Me | F | CH₂CH₂CH₂CH₂ON=C(Me)(*c*-Pr) | O |
| A-3005 | Et | H | Me | F | CH₂CH₂ON=C(Me)CF₃ | O |
| A-3006 | Et | H | Me | F | CH₂CH₂CH₂ON=CHCF₃ | O |
| A-3007 | Et | H | Me | F | CH₂CH₂CH₂ON=C(Me)CF₃ | O |
| A-3008 | Et | H | Me | F | CH₂CH₂CH₂ON=C(Me)CCl₃ | O |
| A-3009 | Et | H | Me | F | CH₂CH₂CH₂CH₂ON=CHCF₃ | O |
| A-3010 | Et | H | Me | F | CH₂CH₂CH₂CH₂ON=C(Me)CF₃ | O |
| A-3011 | Et | H | Me | F | CH₂CH₂CH₂CH₂CH₂ON=C(Me)CF₃ | O |
| A-3012 | Et | H | Me | F | CH₂CH₂ON=CHPh | O |
| A-3013 | Et | H | Me | F | CH₂CH₂ON=CHPh(4-CF₃) | O |
| A-3014 | Et | H | Me | F | CH₂CH₂ON=CHPh(4-SCF₃) | O |
| A-3015 | Et | H | Me | F | CH₂CH₂CH₂ON=CHPh(3-CF₃) | O |
| A-3016 | Et | H | Me | F | CH₂CH₂CH₂ON=CHPh(4-CF₃) | O |
| A-3017 | Et | H | Me | F | CH₂CH₂CH₂O_{N}=CHPh(4-SCF₃) | O |
| A-3018 | Et | H | Me | F | CH₂CH2CH₂ON=C(Me)Ph(4-CF₃) | O |
| A-3019 | Et | H | Me | F | CH₂CH₂CH₂CH₂ON=CHPh(4-SCF₃) | O |
| A-3020 | Et | H | Me | F | CH₂CH₂(adamant-1-yl) | O |
| A-3021 | Et | H | Me | F | CH₂CH₂CH₂(3-CF₃-pyrazol-1-yl) | O |
| A-3022 | Et | H | Me | F | CH₂CH₂CH₂CH₂(3-CF₃-pyrazol-1-yl) | O |
| A-3023 | Et | H | Me | F | CH₂CH₂CH₂CH₂CH₂(3-CF₃-pyrazol-1-yl) | O |
| A-3024 | Et | H | Me | F | CH₂(1-Me-4-CF₃-imidazol-5-yl) | O |
| A-3025 | Et | H | Me | F | CH₂CH₂(1-Me-4-CF₃-imidazol-5-yl) | O |
| A-3026 | Et | H | Me | F | CH₂CH₂CH₂(1-Me-4-CF₃-imidazol-5-yl) | O |
| A-3027 | Et | H | Me | F | CH₂CH₂CH₂(3-CF₃-1,2,4-triazol-1-yl) | O |
| A-3028 | Et | H | Me | F | CH₂CH₂CH₂CH₂(3-CF₃-1,2,4-triazol-1-yl) | O |
| A-3029 | Et | H | Me | F | CH₂CH₂CH₂CH₂CH₂(3-CF₃-1,2,4-triazol-1-yl) | O |
| A-3030 | Et | H | Me | F | CH₂CH₂CH₂(4-CF₃-thiazol-2-yl) | O |

**[Table 70]**

| Compound Number | R¹ | R² | R³ | R⁴ | R⁵ | A |
|---|---|---|---|---|---|---|
| A-3031 | Et | H | Me | F | CH₂CH₂CH₂CH₂(4-CF₃-thiazol-2-yl) | O |
| A-3032 | Et | H | Me | F | CH₂CH₂CH₂CH₂CH₂(4-CF₃-thiazol-2-yl) | O |
| A-3033 | Et | H | Me | F | CH₂CH₂CH₂(2-CF₃-pyridin-5-yl) | O |
| A-3034 | Et | H | Me | F | CH₂CH₂CH₂CH₂(2-CF₃-pyridin-5-yl) | O |
| A-3035 | Et | H | Me | F | CH₂CH₂CH₂CH₂CH₂(2-CF₃-pyridin-5-yl) | O |
| A-3036 | Et | H | Me | F | CH₂(4-CF₃-pyrimidin-2-yl) | O |
| A-3037 | Et | H | Me | F | CH₂CH₂(4-CF₃-pyrimidin-2-yl) | O |
| A-3038 | Et | H | Me | F | CH₂CH₂CH₂(4-CF₃-pyrimidin-2-yl) | O |
| A-3039 | Et | H | Me | F | CH₂CH₂O(3-Cl-5-CF₃-pyridin-2-yl) | O |
| A-3040 | Et | H | Me | F | CH₂CH₂CH₂O(3-Cl-5-CF₃-pyridin-2-yl) | O |
| A-3041 | Et | H | Me | F | CH₂CH₂CH₂CH₂O(3-Cl-5-CF₃-pyridin-2-yl) | O |
| A-3042 | Et | H | Me | F | CH₂CH₂CH₂CH₂CH₂O(3-Cl-5-CF₃-pyridin-2-yl) | O |
| A-3043 | Et | H | Me | F | CH₂CH₂N(Phth) | O |
| A-3044 | Et | H | Me | F | CH₂CH₂CH₂N(Phth) | O |
| A-3045 | Et | H | Me | F | CH₂CH₂CH₂CH₂N(Phth) | O |
| A-3046 | Et | H | Me | F | CH₂CH₂CH₂CH₂CH₂N(Phth) | O |
| A-3047 | Et | H | Me | F | CH₂CH(Me)CH₂N(Phth) | O |
| A-3048 | Et | H | Me | F | CH₂[1,3-dioxolan(2-CF₃)-2-yl] | O |
| A-3049 | Et | H | Me | F | CH₂(azetidin-3-yl) | O |
| A-3050 | Et | H | Me | F | CH₂(pyrrolidin-3-yl) | O |
| A-3051 | Et | H | Me | F | CH₂(piperidin-3-yl) | O |
| A-3052 | Et | H | Me | F | CH₂{azetidin[1-C(=O)O(*t*-Bu)]-3-yl} | O |
| A-3053 | Et | H | Me | F | CH₂{pyrrolidin[1-C(=O)O(*t*-Bu)]-3-yl} | O |
| A-3054 | Et | H | Me | F | CH₂{piperidin[1-C(=O)O(*t*-Bu)]-3-yl} | O |
| A-3055 | Et | H | Me | F | CH₂{azetidin[1-C(=O)CF₃]-3-yl} | O |
| A-3056 | Et | H | Me | F | CH₂{azetidin[1-S(=O)₂CF₃]-3-yl} | O |
| A-3057 | Et | H | Me | F | CH₂{pyrrolidin[1-C(=O)CF₃]-3-yl} | O |
| A-3058 | Et | H | Me | F | CH₂{pyrrolidin[1-S(=O)₂CF₃]-3-yl} | O |
| A-3059 | Et | H | Me | F | CH₂{piperidin[1-C(=O)CF₃]-3-yl} | O |
| A-3060 | Et | H | Me | F | CH₂{piperidin[1-S(=O)₂CF₃]-3-yl} | O |
| A-3061 | Et | H | Me | F | CH₂CH₂CH₂CN | O |
| A-3062 | Et | H | Me | F | CH₂CH₂CH₂CH₂CN | O |
| A-3063 | Et | H | Me | F | CH₂CH₂CH₂CH₂CH₂CN | O |
| A-3064 | Et | H | Me | F | CH₂CH₂CH₂CH₂CH₂CH₂CN | O |
| A-3065 | Et | H | Me | F | CH₂CH₂CH₂C(=O)OH | O |
| A-3066 | Et | H | Me | F | CH₂CH₂CH₂CH₂C(=O)OH | O |
| A-3067 | Et | H | Me | F | CH₂CH₂CH₂CH₂CH₂C(=O)OH | O |
| A-3068 | Et | H | Me | F | CH₂CH₂SCN | O |
| A-3069 | Et | H | Me | F | CH₂CH₂CH₂SCN | O |
| A-3070 | Et | H | Me | F | CH₂CH₂CH₂CH₂SCN | O |
| A-3071 | Et | H | Me | F | CH₂CH₂CH₂CH₂CH₂SCN | O |
| A-3072 | Et | H | Me | Cl | CH₂CH₂CH₂CH₂CH₂SCN | O |
| A-3073 | Et | H | Me | F | CH₂CH₂CH₂CH₂CH₂CH₂SCN | O |
| A-3074 | Et | H | Me | F | CH₂CH₂CH₂CH₂CH₂CH₂CH₂SCN | O |

**[Table 71]**

| Compound Number | R¹ | R² | R³ | R⁴ | R⁵ | A |
|---|---|---|---|---|---|---|
| A-3075 | Et | H | Me | F | CH₂CH₂CH₂CH₂CH₂CH₂CH₂CH₂SCN | O |
| A-3076 | Et | H | Me | F | CH₂CH₂CH(Me)SCN | O |
| A-3077 | Et | H | Me | F | CH₂CH(Me)CH₂SCN | O |
| A-3078 | Et | H | Me | F | CH(Me)CH₂CH₂SCN | O |
| A-3079 | Et | H | Me | F | CH₂CH₂CH₂CH(Me)SCN | O |
| A-3080 | Et | H | Me | F | CH₂CH₂CH(Me)CH₂SCN | O |
| A-3081 | Et | H | Me | F | CH₂CH(Me)CH₂CH₂SCN | O |
| A-3082 | Et | H | Me | F | CH(Me)CH₂CH₂CH₂SCN | O |
| A-3083 | Et | H | Me | F | CH₂CH₂CH₂CH₂CH(Me)SCN | O |
| A-3084 | Et | H | Me | F | CH₂CH₂CH₂CH(Me)CH₂SCN | O |
| A-3085 | Et | H | Me | F | CH₂CH₂CH(Me)CH₂CH₂SCN | O |
| A-3086 | Et | H | Me | F | CH₂CH(Me)CH₂CH₂CH₂SCN | O |
| A-3087 | Et | H | Me | F | CH(Me)CH₂CH₂CH₂CH₂SCN | O |
| A-3088 | Et | H | Me | F | CH₂CH₂ONH₂ | O |
| A-3089 | Et | H | Me | F | CH₂CH₂CH₂ONH₂ | O |
| A-3090 | Et | H | Me | F | CH₂CH₂CH₂CH₂ONH₂ | O |
| A-3091 | Et | H | Me | F | CH₂CH₂CH₂CH₂CH₂ONH₂ | O |
| A-3092 | Et | H | Me | F | CH₂CH₂CH₂CH₂CH₂CH₂ONH₂ | O |
| A-3093 | c-Pr | H | Cl | F | Me | O |
| A-3094 | *c*-Pr | H | Cl | F | Et | O |
| A-3095 | *c*-Pr | H | Cl | F | *n*-Pr | O |
| A-3096 | *c*-Pr | H | Cl | F | *i*-Pr | O |
| A-3097 | *c*-Pr | H | Cl | F | *n*-Bu | O |
| A-3098 | *c*-Pr | H | Cl | F | *n*-Pen | O |
| A-3099 | *c*-Pr | H | Cl | F | *n*-Hex | O |
| A-3100 | *c*-Pr | H | Cl | F | *n*-Heptyl | O |
| A-3101 | *c*-Pr | H | Cl | F | *n*-Octyl | O |
| A-3102 | *c*-Pr | H | Cl | F | *n*-Nonyl | O |
| A-3103 | *c*-Pr | H | Cl | F | *n*-Decyl | O |
| A-3104 | *c*-Pr | H | Cl | F | *c*-Pr | O |
| A-3105 | *c*-Pr | H | Cl | F | *c*-Pen | O |
| A-3106 | *c*-Pr | H | Cl | F | *c*-Hex | O |
| A-3107 | *c*-Pr | H | Cl | F | CH₂C(Me)=CH₂ | O |
| A-3108 | *c*-Pr | H | Cl | F | CH₂CH₂CH=CH₂ | O |
| A-3109 | *c*-Pr | H | Cl | F | CH₂CH₂CH=C(CH₃)₂ | O |
| A-3110 | *c*-Pr | H | Cl | F | CH₂CH₂CH₂CH=CH₂ | O |
| A-3111 | *c*-Pr | H | Cl | F | CH₂CH₂CH₂CH=C(CH₃)₂ | O |
| A-3112 | *c*-Pr | H | Cl | F | CH₂CH₂CH₂CH₂CH=CH₂ | O |
| A-3113 | *c*-Pr | H | Cl | F | CH₂CH₂CH₂CH₂CH=C(CH₃)₂ | O |
| A-3114 | *c*-Pr | H | Cl | F | CH₂CH₂CH≡CH | O |
| A-3115 | *c*-Pr | H | Cl | F | CH₂CH₂CH₂CH≡CH | O |
| A-3116 | *c*-Pr | H | Cl | F | CH₂CH₂CH₂CH₂CH≡CH | O |
| A-3117 | *c*-Pr | H | Cl | F | CH₂CH₂CH₂Cl | O |
| A-3118 | *c*-Pr | H | Cl | F | CH₂CH₂CH₂Br | O |

**[Table 72]**

| Compound Number | R¹ | R² | R³ | R⁴ | R⁵ | A |
|---|---|---|---|---|---|---|
| A-3119 | *c*-Pr | H | Cl | F | CH₂CH₂CH₂CH₂Cl | O |
| A-3120 | c*-*Pr | H | Cl | F | CH₂CH₂CH₂CH₂Br | O |
| A-3121 | *c*-Pr | H | Cl | F | CH₂CH₂CH₂CH₂CH₂Cl | O |
| A-3122 | *c*-Pr | H | Cl | F | CH₂CH₂CH₂CH₂CH₂Br | O |
| A-3123 | *c*-Pr | H | Cl | Cl | CH₂CH₂CH₂CH₂CH₂Br | O |
| A-3124 | *c*-Pr | H | Cl | F | CH₂CH₂CH₂CH₂CH₂CH₂Cl | O |
| A-3125 | *c*-Pr | H | Cl | F | CH₂CH₂CH₂CH₂CH₂CH₂Br | O |
| A-3126 | *c*-Pr | H | Cl | F | CH₂CH₂CH₂CH₂CH₂CH₂CH₂Cl | O |
| A-3127 | *c*-Pr | H | Cl | F | CH₂CH₂CH₂CH₂CH₂CH₂CH₂Br | O |
| A-3128 | *c*-Pr | H | Cl | F | CH₂CH₂CH₂CH₂CH₂CH₂CH₂CH₂Br | O |
| A-3129 | *c*-Pr | H | Cl | F | CH₂CH(Me)CH₂Cl | O |
| A-3130 | *c*-Pr | H | Cl | F | CH₂CH(Me)CH₂Br | O |
| A-3131 | *c*-Pr | H | Cl | F | CH₂CH₂CH(Me)CH₂CH₂Cl | O |
| A-3132 | *c*-Pr | H | Cl | F | CH₂CH₂CH(Me)CH₂CH₂Br | O |
| A-3133 | *c*-Pr | H | Cl | F | CH₂CH₂CF=CF₂ | O |
| A-3134 | *c*-Pr | H | Cl | F | CH₂CH=C(Cl)CF₃ | O |
| A-3135 | *c*-Pr | H | Cl | F | CH₂CH₂CH₂CF=CF₂ | O |
| A-3136 | *c*-Pr | H | Cl | F | CH₂CH₂CH₂CH₂CF=CF₂ | O |
| A-3137 | *c*-Pr | H | Cl | F | CH₂CH₂CH₂CH₂CH₂CF=CF₂ | O |
| A-3138 | *c*-Pr | H | Cl | F | CH₂(t-Bu) | O |
| A-3139 | *c*-Pr | H | Cl | F | CH₂CH₂(t-Bu) | O |
| A-3140 | *c*-Pr | H | Cl | F | CH₂CH₂CH₂(*t*-Bu) | O |
| A-3141 | *c*-Pr | H | Cl | F | CH₂CH₂CH₂CH₂(t-Bu) | O |
| A-3142 | *c*-Pr | H | Cl | F | CH₂CH₂CH₂CH₂CH₂(t-Bu) | O |
| A-3143 | *c*-Pr | H | Cl | F | CH₂CH₂CH₂CH₂CH₂CH₂(t-Bu) | O |
| A-3144 | *c*-Pr | H | Cl | F | CH₂CF₃ | O |
| A-3145 | *c*-Pr | H | Cl | F | CH₂CH₂CF₃ | O |
| A-3146 | *c*-Pr | H | Cl | F | CH₂CH₂CH₂CF₃ | O |
| A-3147 | *c*-Pr | H | Cl | F | CH₂CH₂CH₂CH₂CF₃ | O |
| A-3148 | *c*-Pr | H | Cl | F | CH₂CH₂CH₂CH₂CH₂CF₃ | O |
| A-3149 | *c*-Pr | H | Cl | F | CH₂CH₂CH₂CH₂CH₂CH₂CF₃ | O |
| A-3150 | *c*-Pr | H | Cl | F | CH₂CH₂CH₂CH₂CH₂CH₂CH₂CF₃ | O |
| A-3151 | *c*-Pr | H | Cl | F | CH₂CF₂CF₃ | O |
| A-3152 | *c*-Pr | H | Cl | F | CF₂CHFCF₃ | O |
| A-3153 | *c*-Pr | H | Cl | F | CF₂CF₂CF₃ | O |
| A-3154 | *c*-Pr | H | Cl | F | CH₂CF₂CF₂CF₃ | O |
| A-3155 | *c*-Pr | H | Cl | F | CH₂CF₂CF₂CF₂CF₃ | O |
| A-3156 | *c*-Pr | H | Cl | F | CH₂CF₂CF₂CF₂CHF₂ | O |
| A-3157 | *c*-Pr | H | Cl | F | CH(CF₃)CF₃ | O |
| A-3158 | *c*-Pr | H | Cl | F | CH₂CF(CF₃)CF₃ | O |
| A-3159 | *c*-Pr | H | Cl | F | CH₂CF₂CF(CF₃)CF₃ | O |
| A-3160 | *c*-Pr | H | Cl | F | CH₂CF(CF₃)CF₂CF₃ | O |
| A-3161 | *c*-Pr | H | Cl | F | CH₂CF₂CF₂CF(CF₃)CF₃ | O |
| A-3162 | *c*-Pr | H | Cl | F | CH₂CF₂CF(CF₃)CF₂CF₃ | O |

**[Table 73]**

| Compound Number | R¹ | R² | R³ | R⁴ | R⁵ | A |
|---|---|---|---|---|---|---|
| A-3163 | *c*-Pr | H | Cl | F | CH₂CF(CF₃)CF₂CF₂CF₃ | O |
| A-3164 | *c*-Pr | H | Cl | F | CH₂(*c*-Pr) | O |
| A-3165 | *c*-Pr | H | Cl | F | CH₂[*c*-Pr(1-Me)] | O |
| A-3166 | *c*-Pr | H | Cl | F | CH₂[*c*-Pr(1-Ph)] | O |
| A-3167 | *c-*Pr | H | Cl | F | CH₂[*c*-Pr(1-NH₂)] | O |
| A-3168 | *c*-Pr | H | Cl | F | CH₂[*c*-Pr(1-NHC(=O)O(t-Bu)] | O |
| A-3169 | *c*-Pr | H | Cl | F | CH₂{*c*-Pr[1-NHS(=O)₂CF₃]} | O |
| A-3170 | *c*-Pr | H | Cl | F | CH₂{*c*-Pr[1-Ph(4-CF₃)]} | O |
| A-3171 | *c*-Pr | H | Cl | F | CH₂{*c*-Pr[1-Ph(3,4,5-F₃)]} | O |
| A-3172 | *c*-Pr | H | Cl | F | CH₂{*c*-Hex[4-(*t*-Bu)]} | O |
| A-3173 | *c*-Pr | H | Cl | F | CH₂[*c*-Hex(4-CF₃)] | O |
| A-3174 | *c*-Pr | H | Cl | F | CH₂CH₂(*c*-Pr) | O |
| A-3175 | *c* -Pr | H | Cl | F | CH₂CH₂(*c*-Hex) | O |
| A-3176 | *c*-Pr | H | Cl | F | CH₂CH₂[*c*-Hex(4-CF₃)] | O |
| A-3177 | *c*-Pr | H | Cl | F | CH₂CH₂[*c*-Hex(4-SCF₃)] | O |
| A-3178 | *c*-Pr | H | Cl | F | CH₂CH₂CH₂(*c* -Pr) | O |
| A-3179 | *c*-Pr | H | Cl | F | CH₂CH₂CH₂(*c*-Hex) | O |
| A-3180 | *c*-Pr | H | Cl | F | CH₂CH₂CH₂{*c*-Hex[4-(t-Bu)]} | O |
| A-3181 | *c*-Pr | H | Cl | F | CH₂CH₂CH₂[*c*-Hex(4-CF₃)] | O |
| A-3182 | *c*-Pr | H | Cl | F | CH₂CH₂CH₂[*c*-Hex(4-SCF₃)] | O |
| A-3183 | *c*-Pr | H | Cl | F | CH₂CH₂CH₂CH₂(*c*-Pr) | O |
| A-3184 | *c*-Pr | H | Cl | F | CH₂CH₂CH₂CH₂(*c*-Hex) | O |
| A-3185 | *c*-Pr | H | Cl | F | CH₂CH₂CH₂CH₂[c-Hex(4-CF₃)] | O |
| A-3186 | *c*-Pr | H | Cl | F | CH₂[*c*-Pr(2,2-F₂)] | O |
| A-3187 | *c*-Pr | H | Cl | F | CH₂[*c*-Hex(4,4-F₂)] | O |
| A-3188 | *c*-Pr | H | Cl | F | CH₂CH₂[*c*-Pr(2,2-F₂)] | O |
| A-3189 | *c* -Pr | H | Cl | F | CH₂CH₂[*c*-Hex(4,4-F₂)] | O |
| A-3190 | *c*-Pr | H | Cl | F | CH₂CH₂CH₂[*c*-Pr(2,2-F₂)] | O |
| A-3191 | *c*-Pr | H | Cl | F | CH₂CH₂CH₂CH₂[*c*-Pr(2,2-F₂)] | O |
| A-3192 | *c*-Pr | H | Cl | F | CH₂CH₂OH | O |
| A-3193 | *c*-Pr | H | Cl | F | CH₂CH₂CH₂OH | O |
| A-3194 | *c*-Pr | H | Cl | F | CH₂CH₂CH₂CH₂OH | O |
| A-3195 | *c*-Pr | H | Cl | F | CH₂CH₂CH₂CH₂CH₂OH | O |
| A-3196 | *c*-Pr | H | Cl | F | CH₂CH₂CH₂CH₂CH₂CH₂0H | O |
| A-3197 | *c*-Pr | H | Cl | F | CH₂C(=O)Me | O |
| A-3198 | *c*-Pr | H | Cl | F | CH₂CH(OH)Me | O |
| A-3199 | *c*-Pr | H | Cl | F | CH₂CH(OH)CF₃ | O |
| A-3200 | *c*-Pr | H | Cl | F | CH₂C(OH)₂CF₃ | O |
| A-3201 | *c*-Pr | H | Cl | F | CH₂C(CF₃)=NOH | O |
| A-3202 | *c*-Pr | H | Cl | F | CH₂C(CF₃)=NOMe | O |
| A-3203 | *c*-Pr | H | Cl | F | CH₂CH(CF₃)NH₂ | O |
| A-3204 | *c*-Pr | H | Cl | F | CH₂CH₂OMe | O |
| A-3205 | *c*-Pr | H | Cl | F | CH₂CH₂CH₂OMe | O |
| A-3206 | *c* -Pr | H | Cl | F | CH₂CH₂CH₂CH₂OMe | O |

**[Table 74]**

| Compound Number | R¹ | R² | R³ | R⁴ | R⁵ | A |
|---|---|---|---|---|---|---|
| A-3207 | *c*-Pr | H | Cl | F | CH₂CH₂CH₂CH₂CH₂OMe | O |
| A-3208 | *c*-Pr | H | Cl | F | CH₂CH₂CH₂CH₂CH₂CH₂OMe | O |
| A-3209 | *c*-Pr | H | Cl | F | CH₂CH₂OCHF₂ | O |
| A-3210 | *c*-Pr | H | Cl | F | CH₂CH₂CH₂OCHF₂ | O |
| A-3211 | *c*-Pr | H | Cl | F | CH₂CH₂CH₂CH₂OCHF₂ | O |
| A-3212 | *c*-Pr | H | Cl | F | CH₂CH₂CH₂CH₂CH₂OCHF₂ | O |
| A-3213 | *c*-Pr | H | Cl | F | CH₂CH₂CH₂CH₂CH₂CH₂OCHF₂ | O |
| A-3214 | *c*-Pr | H | Cl | F | CH₂CH₂OCH₂CF₃ | O |
| A-3215 | *c*-Pr | H | Cl | F | CH₂CH₂CH₂OCH₂CF₃ | O |
| A-3216 | *c*-Pr | H | Cl | F | CH₂CH₂CH₂CH₂OCH₂CF₃ | O |
| A-3217 | *c*-Pr | H | Cl | F | CH₂CH₂CH₂CH₂CH₂OCH₂CF₃ | O |
| A-3218 | *c*-Pr | H | Cl | F | CH₂CH₂CH₂CH₂CH₂CH₂OCH₂CF₃ | O |
| A-3219 | *c*-Pr | H | Cl | F | CH₂CH₂CH₂CH₂CH₂CH₂CH₂OCH₂CF₃ | O |
| A-3220 | *c*-Pr | H | Cl | F | CH₂CH₂OC(CF₃)₃ | O |
| A-3221 | *c*-Pr | H | Cl | F | CH₂CH₂CH₂OC(CF₃)₃ | O |
| A-3222 | *c*-Pr | H | Cl | F | CH₂CH₂CH₂CH₂OC(CF₃)₃ | O |
| A-3223 | *c*-Pr | H | Cl | F | CH₂CH₂CH₂CH₂CH₂OC(CF₃)₃ | O |
| A-3224 | *c*-Pr | H | Cl | F | CF₂CHFOCF₂CF₂CF₃ | O |
| A-3225 | *c*-Pr | H | Cl | F | CH₂CH₂O(*c*-Pr) | O |
| A-3226 | *c*-Pr | H | Cl | F | CH₂CH₂O(*c*-Pen) | O |
| A-3227 | *c*-Pr | H | Cl | F | CH₂CH₂O(*c*-Hex) | O |
| A-3228 | *c*-Pr | H | Cl | F | CH₂CH₂CH₂O(*c*-Pr) | O |
| A-3229 | *c*-Pr | H | Cl | F | CH₂CH₂CH₂O(*c*-Pen) | O |
| A-3230 | *c*-Pr | H | Cl | F | CH₂CH₂CH₂O(*c*-Hex) | O |
| A-3231 | *c*-Pr | H | Cl | F | CH₂CH₂CH₂CH₂O(*c*-Pr) | O |
| A-3232 | *c*-Pr | H | Cl | F | CH₂CH₂CH₂CH₂O(*c*-Pen) | O |
| A-3233 | *c*-Pr | H | Cl | F | CH₂CH₂CH₂CH₂O(*c*-Hex) | O |
| A-3234 | *c*-Pr | H | Cl | F | CH₂CH₂O[*c*-Pr(2,2-F₂)] | O |
| A-3235 | *c*-Pr | H | Cl | F | CH₂CH₂CH₂O[*c*-Pr(2,2-F₂)] | O |
| A-3236 | *c*-Pr | H | Cl | F | CH₂CH₂CH₂CH₂O[*c*-Pr(2,2-F₂)] | O |
| A-3237 | *c*-Pr | H | Cl | F | CH₂CH₂OCH₂CH₂OCH₃ | O |
| A-3238 | *c*-Pr | H | Cl | F | CH₂CH₂OCH₂CH₂OCH₂CF₃ | O |
| A-3239 | *c*-Pr | H | Cl | F | CH₂CF₂OCF₂CF₂OCF₃ | O |
| A-3240 | *c*-Pr | H | Cl | F | CF₂CHFOCF₂CF₂OCF₃ | O |
| A-3241 | *c*-Pr | H | Cl | F | CF₂CHFOCF₂CF(CF₃)OCF₂CF₂CF₃ | O |
| A-3242 | *c*-Pr | H | Cl | F | CH₂CH₂SH | O |
| A-3243 | *c*-Pr | H | Cl | F | CH₂CH₂CH₂SH | O |
| A-3244 | *c*-Pr | H | Cl | F | CH₂CH₂CH₂CH₂SH | O |
| A-3245 | *c*-Pr | H | Cl | F | CH₂CH₂CH₂CH₂CH₂SH | O |
| A-3246 | *c*-Pr | H | Cl | F | CH₂CH₂CH₂CH₂CH₂CH₂SH | O |
| A-3247 | *c*-Pr | H | Cl | F | CH₂CH(SH)Me | O |
| A-3248 | *c*-Pr | H | Cl | F | CH₂CH[SC(=O)Me]Me | O |
| A-3249 | *c*-Pr | H | Cl | F | CH₂CH₂S(*t*-Bu) | O |
| A-3250 | *c*-Pr | H | Cl | F | CH₂CH₂CH₂S(*t-*Bu) | O |

**[Table 75]**

| Compound Number | R¹ | R² | R³ | R⁴ | R⁵ | A |
|---|---|---|---|---|---|---|
| A-3251 | *c*-Pr | H | Cl | F | CH₂CH₂CH₂CH₂S(t-Bu) | O |
| A-3252 | *c*-Pr | H | Cl | F | CH₂CH₂CH₂CH₂CH₂SCH₃ | O |
| A-3253 | *c*-Pr | H | Cl | F | CH₂CH₂CH₂CH₂CH₂SCH(CH₃)₂ | O |
| A-3254 | *c*-Pr | H | Cl | F | CH₂CH₂CH₂CH₂CH₂S(t-Bu) | O |
| A-3255 | *c*-Pr | H | Cl | F | CH₂CH₂CH₂CH₂CH₂CH₂SCH(CH₃)₂ | O |
| A-3256 | *c*-Pr | H | Cl | F | CH₂CH₂CH₂CH₂CH₂CH₂S(*t*-Bu) | O |
| A-3257 | *c*-Pr | H | Cl | F | CH₂CH₂CH₂CH₂CH₂CH₂CH₂S(*t*-Bu) | O |
| A-3258 | *c*-Pr | H | Cl | F | CH₂CH₂SCHF₂ | O |
| A-3259 | *c*-Pr | H | Cl | F | CH₂CH₂CH₂SCHF₂ | O |
| A-3260 | *c*-Pr | H | Cl | F | CH₂CH₂CH₂CH₂SCHF₂ | O |
| A-3261 | *c*-Pr | H | Cl | H | CH₂CH₂CH₂CH₂CH₂SCHF₂ | O |
| A-3262 | *c*-Pr | H | Cl | F | CH₂CH₂CH₂CH₂CH₂SCHF₂ | O |
| A-3263 | *c*-Pr | H | Cl | F | CH₂CH₂CH₂CH₂CH₂CH₂SCHF₂ | O |
| A-3264 | *c*-Pr | H | Cl | F | CH₂CH₂CH₂CH₂CH₂CH₂CH₂SCHF₂ | O |
| A-3265 | *c*-Pr | H | Cl | F | CH₂CH₂SCH₂CF₃ | O |
| A-3266 | *c*-Pr | H | Cl | F | CH₂CH₂CH₂SCH₂CF₃ | O |
| A-3267 | *c*-Pr | H | Cl | F | CH₂CH₂CH₂CH₂SCH₂CF₃ | O |
| A-3268 | *c*-Pr | H | Cl | F | CH₂CH₂CH₂CH₂CH₂SCH₂CF₃ | O |
| A-3269 | *c*-Pr | H | Cl | F | CH₂CH₂CH₂CH₂CH₂CH₂SCH₂CF₃ | O |
| A-3270 | *c*-Pr | H | Cl | F | CH₂CH₂CH₂CH₂CH₂CH₂CH₂SCH₂CF₃ | O |
| A-3271 | *c*-Pr | H | Cl | F | CH₂CH(Me)SCH₂CF₃ | O |
| A-3272 | *c*-Pr | H | Cl | F | CH₂CH₂SCH(CF₃)₂ | O |
| A-3273 | *c*-Pr | H | Cl | F | CH₂CH₂CH₂SCH(CF₃)₂ | O |
| A-3274 | *c*-Pr | H | Cl | F | CH₂CH₂CH₂CH₂SCH(CF₃)₂ | O |
| A-3275 | *c*-Pr | H | Cl | F | CH₂CH₂CH₂CH₂CH₂SCH(CF₃)₂ | O |
| A-3276 | *c*-Pr | H | Cl | F | CH₂CH₂CH₂CH₂CH₂CH₂SCH(CF₃)₂ | O |
| A-3277 | *c*-Pr | H | Cl | F | CH₂CH₂CH₂CH₂CH₂SCF₂CF₃ | O |
| A-3278 | *c*-Pr | H | Cl | F | CH₂CH₂CH₂CH₂CH₂CH₂SCF₂CF₃ | O |
| A-3279 | *c-*Pr | H | Cl | F | CH₂CH₂CH₂CH₂CH₂CH₂SCF(CF₃)₂ | O |
| A-3280 | *c*-Pr | H | Cl | F | CH₂CH₂CH₂CH₂CH₂CH₂CH₂SCF(CF₃)₂ | O |
| A-3281 | *c*-Pr | H | Cl | F | CH₂CH₂CH₂CH₂CH₂SCCl₃ | O |
| A-3282 | *c*-Pr | H | Cl | F | CH₂CH₂CH₂CH₂CH₂CH₂SCCl₃ | O |
| A-3283 | *c*-Pr | H | Cl | F | CH₂CH₂SCH₂CH=CH₂ | O |
| A-3284 | *c*-Pr | H | Cl | F | CH₂CH₂CH₂SCH₂CH=CH₂ | O |
| A-3285 | *c*-Pr | H | Cl | F | CH₂CH₂CH₂CH₂SCH₂CH=CH₂ | O |
| A-3286 | *c*-Pr | H | Cl | F | CH₂CH₂SCF=CFCF₃ | O |
| A-3287 | *c*-Pr | H | Cl | F | CH₂CH₂CH₂SCF=CFCF₃ | O |
| A-3288 | *c*-Pr | H | Cl | F | CH₂CH₂CH₂CH₂SCF=CFCF₃ | O |
| A-3289 | *c*-Pr | H | Cl | F | CH₂CH₂CH₂S(*c*-Pr) | O |
| A-3290 | *c*-Pr | H | Cl | F | CH₂CH₂CH₂S(c-Hex) | O |
| A-3291 | *c*-Pr | H | Cl | F | CH₂CH₂CH₂CH₂S(*c*-Pr) | O |
| A-3292 | *c*-Pr | H | Cl | F | CH₂CH₂CH₂CH₂S(*c*-Hex) | O |
| A-3293 | *c*-Pr | H | Cl | F | CH₂CH₂CH₂CH₂CH₂S(*c*-Pr) | O |
| A-3294 | *c*-Pr | H | Cl | F | CH₂CH₂CH₂CH₂CH₂S(*c*-Hex) | O |

**[Table 76]**

| Compound Number | R¹ | R² | R³ | R⁴ | R⁵ | A |
|---|---|---|---|---|---|---|
| A-3295 | *c*-Pr | H | Cl | F | CH₂CH₂CH₂S[c-Hex(4,4-F₂)] | O |
| A-3296 | *c*-Pr | H | Cl | F | CH₂CH₂CH₂CH₂S[c-Hex(4,4-F₂)] | O |
| A-3297 | *c*-Pr | H | Cl | F | CH₂C H₂CH₂CH₂CH₂S[*c*-Hex(4,4-F₂)] | O |
| A-3298 | *c*-Pr | H | Cl | F | CH₂CH₂CH₂SCH₂(*c*-Pr) | O |
| A-3299 | *c*-Pr | H | Cl | F | CH₂CH₂CH₂SCH₂(*c*-Hex) | O |
| A-3300 | *c*-Pr | H | Cl | F | CH₂CH₂CH₂CH₂SCH₂(*c*-Pr) | O |
| A-3301 | *c*-Pr | H | Cl | F | CH₂CH₂CH₂CH₂SCH₂(*c*-Hex) | O |
| A-3302 | *c*-Pr | H | Cl | F | CH₂CH₂CH₂CH₂CH₂SCH₂(*c*-Pr) | O |
| A-3303 | *c*-Pr | H | Cl | F | CH₂CH₂CH₂CH₂CH₂SCH₂(c-Hex) | O |
| A-3304 | *c*-Pr | H | Cl | F | CH₂CH₂CH₂SCH₂[c-Hex(4,4-F₂)] | O |
| A-3305 | *c*-Pr | H | Cl | F | CH₂CH₂CH₂CH₂SCH₂[c-Hex(4,4-F₂)] | O |
| A-3306 | *c*-Pr | H | Cl | F | CH₂CH₂CH₂CH₂CH₂SCH₂[C-Hex(4,4-F₂)] | O |
| A-3307 | *c*-Pr | H | Cl | F | CH₂CH₂CH₂SCH₂SiMe₃ | O |
| A-3308 | *c*-Pr | H | Cl | F | CH₂CH₂CH₂CH₂SCH₂SiMe₃ | O |
| A-3309 | *c*-Pr | H | Cl | F | CH₂CH₂CH₂CH₂CH₂SCH₂SiMe₃ | O |
| A-3310 | *c*-Pr | H | Cl | F | CH₂CH₂OCH₂CH₂SCH₃ | O |
| A-3311 | *c*-Pr | H | Cl | F | CH₂CH₂OCH₂CH₂SCHF₂ | O |
| A-3312 | *c*-Pr | H | Cl | F | CH₂CH₂OCH₂CH₂SCF₃ | O |
| A-3313 | *c*-Pr | H | Cl | F | CH₂CH₂OCH₂CH₂SCH₂CF₃ | O |
| A-3314 | *c*-Pr | H | Cl | F | CH₂CH₂CH₂S(=O)(t-Bu) | O |
| A-3315 | *c*-Pr | H | Cl | F | CH₂CH₂CH₂CH₂S(=O)(t-Bu) | O |
| A-3316 | *c*-Pr | H | Cl | F | CH₂CH₂CH₂CH₂CH₂CH₂S(=O)(t-Bu) | O |
| A-3317 | *c*-Pr | H | Cl | F | CH₂CH₂S(=O)CHF₂ | O |
| A-3318 | *c*-Pr | H | Cl | F | CH₂CH₂CH₂S(=O)CHF₂ | O |
| A-3319 | *c*-Pr | H | Cl | F | CH₂C H₂CH₂CH₂S(=O)CHF₂ | O |
| A-3320 | *c*-Pr | H | Cl | F | CH₂CH₂CH₂CH₂CH₂S(=O)CHF₂ | O |
| A-3321 | *c*-Pr | H | Cl | F | CH₂CH₂CH₂CH₂CH₂CH₂S(=O)CHF₂ | O |
| A-3322 | *c-*Pr | H | Cl | F | CH₂CH₂CH₂CH₂CH₂CH₂CH₂S(=O)CHF₂ | O |
| A-3323 | *c*-Pr | H | Cl | F | CH₂CH₂S(=O)CF₃ | O |
| A-3324 | *c*-Pr | H | Cl | F | CH₂CH₂CH₂S(=O)CF₃ | O |
| A-3325 | *c*-Pr | H | Cl | F | CH₂CH₂CH₂CH₂S(=O)CF₃ | O |
| A-3326 | *c*-Pr | H | Cl | F | CH₂CH₂CH₂CH₂CH₂S(=O)CF₃ | O |
| A-3327 | *c*-Pr | H | Cl | F | CH₂CH₂CH₂CH₂CH₂CH₂S(=O)CF₃ | O |
| A-3328 | *c*-Pr | H | Cl | F | CH₂CH₂CH₂CH₂CH₂CH₂CH₂S(=O)CF₃ | O |
| A-3329 | *c*-Pr | H | Cl | F | CH₂CH₂S(=O)CH₂CF₃ | O |
| A-3330 | *c*-Pr | H | Cl | F | CH₂CH₂CH₂S(=O)CH₂CF₃ | O |
| A-3331 | *c*-Pr | H | Cl | F | CH₂CH₂CH₂CH₂S(=O)CH₂CF₃ | O |
| A-3332 | *c*-Pr | H | Cl | F | CH₂CH₂CH₂CH₂CH₂S(=O)CH₂CF₃ | O |
| A-3333 | *c*-Pr | H | Cl | F | CH₂CH₂CH₂CH₂CH₂CH₂S(=O)CH₂CF₃ | O |
| A-3334 | *c*-Pr | H | Cl | F | CH₂CH₂CH₂CH₂CH₂CH₂CH₂S(=O)CH₂CF₃ | O |
| A-3335 | *c*-Pr | H | Cl | F | CH₂CH(Me)S(=O)CH₂CF₃ | O |
| A-3336 | *c*-Pr | H | Cl | F | CH₂CH₂CH₂S(=O)(c-Pr) | O |
| A-3337 | *c*-Pr | H | Cl | F | CH₂CH₂CH₂CH₂S(=O)(c-Pr) | O |
| A-3338 | *c*-Pr | H | Cl | F | CH₂CH₂CH₂CH₂S(=O)(c-Pen) | O |

**[Table 77]**

| Compound Number | R¹ | R² | R³ | R⁴ | R⁵ | A |
|---|---|---|---|---|---|---|
| A-3339 | *c*-Pr | H | Cl | F | CH₂CH₂CH₂CH₂S(=O)(c-Hex) | O |
| A-3340 | *c*-Pr | H | Cl | F | CH₂CH₂CH₂CH₂CH₂S(=O)(c-Pr) | O |
| A-3341 | *c*-Pr | H | Cl | F | CH₂CH₂CH₂CH₂CH₂S(=O)(c-Hex) | O |
| A-3342 | *c*-Pr | H | Cl | F | CH₂C H₂CH₂S(=O)[*c*-Hex(4,4-F₂)] | O |
| A-3343 | *c*-Pr | H | Cl | F | CH₂CH₂CH₂CH₂S(=O)[c-Hex(4,4-F₂)] | O |
| A-3344 | *c*-Pr | H | Cl | F | CH₂CH₂CH₂CH₂CH₂S(=O)[c-Hex(4,4-F₂)] | O |
| A-3345 | *c*-Pr | H | Cl | F | CH₂CH₂CH₂CH₂S(=O)₂Me | O |
| A-3346 | *c*-Pr | H | Cl | F | CH₂CH₂CH₂CH₂CH₂S(=O)₂Me | O |
| A-3347 | *c*-Pr | H | Cl | F | CH₂CH₂CH₂CH₂CH₂S(=O)₂(t-Bu) | O |
| A-3348 | *c*-Pr | H | Cl | F | CH₂CH₂S(=O)₂CHF₂ | O |
| A-3349 | *c*-Pr | H | Cl | F | CH₂CH₂CH₂S(=O)₂CHF₂ | O |
| A-3350 | *c*-Pr | H | Cl | F | CH₂CH₂CH₂CH₂S(=O)₂CHF₂ | O |
| A-3351 | *c*-Pr | H | Cl | F | CH₂CH₂CH₂CH₂CH₂S(=O)₂CHF₂ | O |
| A-3352 | *c*-Pr | H | Cl | F | CH₂CH₂CH₂CH₂CH₂CH₂S(=O)₂CHF₂ | O |
| A-3353 | *c*-Pr | H | Cl | F | CH₂CH₂S(=O)₂CF₃ | O |
| A-3354 | *c*-Pr | H | Cl | F | CH₂CH₂CH₂S(=O)₂CF₃ | O |
| A-3355 | *c*-Pr | H | Cl | F | CH₂CH₂CH₂CH₂S(=O)₂CF₃ | O |
| A-3356 | *c*-Pr | H | Cl | F | CH₂CH₂CH₂CH₂CH₂S(=O)₂CF₃ | O |
| A-3357 | *c*-Pr | H | Cl | F | CH₂CH₂CH₂CH₂CH₂CH₂S(=O)₂CF₃ | O |
| A-3358 | *c*-Pr | H | Cl | F | CH₂CH₂CH₂CH₂CH₂CH₂CH₂S(=O)₂CF₃ | O |
| A-3359 | *c*-Pr | H | Cl | F | CH₂CH₂CH₂CH₂CH₂CH₂CH₂CH₂S(=O)₂CF₃ | O |
| A-3360 | *c-*Pr | H | Cl | F | CH₂CH₂S(=O)₂CH₂CF₃ | O |
| A-3361 | *c*-Pr | H | Cl | F | CH₂CH₂CH₂S(=O)₂CH₂CF₃ | O |
| A-3362 | *c*-Pr | H | Cl | F | CH₂CH₂CH₂CH₂S(=O)₂CH₂CF₃ | O |
| A-3363 | *c*-Pr | H | Cl | F | CH₂CH₂CH₂CH₂CH₂S(=O)₂CH₂CF₃ | O |
| A-3364 | *c*-Pr | H | Cl | F | CH₂CH₂CH₂CH₂CH₂CH₂S(=O)₂CH₂CF₃ | O |
| A-3365 | *c*-Pr | H | Cl | F | CH₂CH(Me)S(=O)₂CH₂CF₃ | O |
| A-3366 | *c*-Pr | H | Cl | F | CH₂CH₂CH₂S(=O)₂(c-Pr) | O |
| A-3367 | *c*-Pr | H | Cl | F | CH₂CH₂CH₂CH₂S(=O)₂(C-Pr) | O |
| A-3368 | *c*-Pr | H | Cl | F | CH₂CH₂CH₂CH₂CH₂S(=O)₂(c-Pr) | O |
| A-3369 | *c*-Pr | H | Cl | F | CH₂CH₂CH₂S(=O)₂[c-Hex(4,4-F₂)] | O |
| A-3370 | *c*-Pr | H | Cl | F | CH₂CH₂CH_{2C}H₂S(=O)₂[c-Hex(4,4-F₂)] | O |
| A-3371 | *c*-Pr | H | Cl | F | CH₂CH₂CH₂CH₂CH₂S(=O)₂[c-Hex(4,4-F₂)] | O |
| A-3372 | *c*-Pr | H | Cl | F | CH₂CH₂CH₂C(=O)H | O |
| A-3373 | *c*-Pr | H | Cl | F | CH₂CH₂CH₂CH₂C(=O)H | O |
| A-3374 | *c*-Pr | H | Cl | F | CH₂CH₂CH₂CH₂CH₂C(=O)H | O |
| A-3375 | *c*-Pr | H | Cl | F | CH₂CH₂CH₂C(=O)(t-Bu) | O |
| A-3376 | *c*-Pr | H | Cl | F | CH₂CH₂CH₂CH₂C(=O)(t-Bu) | O |
| A-3377 | *c*-Pr | H | Cl | F | CH₂CH₂CH₂CH₂CH₂C(=O)(t-Bu) | O |
| A-3378 | *c*-Pr | H | Cl | F | CH₂CH₂CH₂C(=O)CF₃ | O |
| A-3379 | *c*-Pr | H | Cl | F | CH₂CH₂CH₂CH₂C(=O)CF₃ | O |
| A-3380 | *c*-Pr | H | Cl | F | CH₂CH₂CH₂CH₂CH₂C(=O)CF₃ | O |
| A-3381 | *c*-Pr | H | Cl | F | CH₂C(=O)Ph | O |
| A-3382 | *c*-Pr | H | Cl | F | CH₂C(=O)Ph(4-Cl) | O |

**[Table 78]**

| Compound Number | R¹ | R² | R³ | R⁴ | R⁵ | A |
|---|---|---|---|---|---|---|
| A-3383 | *c*-Pr | H | Cl | F | CH₂C(=O)Ph(4-CF₃) | O |
| A-3384 | *c*-Pr | H | Cl | F | CH₂CH₂C(=O)Ph(4-CF₃) | O |
| A-3385 | *c*-Pr | H | Cl | F | CH₂CH₂CH₂C(=O)Ph(4-CF₃) | O |
| A-3386 | *c*-Pr | H | Cl | F | CH₂CH₂CH₂CH₂C(=O)Ph(4-CF₃) | O |
| A-3387 | *c*-Pr | H | Cl | F | CH₂CH₂CH₂CH₂CH₂C(=O)Ph(4-CF₃) | O |
| A-3388 | *c*-Pr | H | Cl | F | CH₂C(=O)OEt | O |
| A-3389 | *c*-Pr | H | Cl | F | CH₂CH₂C(=O)O(t-Bu) | O |
| A-3390 | *c*-Pr | H | Cl | F | CH₂CH₂CH₂C(=O)OEt | O |
| A-3391 | *c*-Pr | H | Cl | F | CH₂CH₂CH₂C(=O)O(t-Bu) | O |
| A-3392 | *c*-Pr | H | Cl | F | CH₂CH₂CH₂CH₂C(=O)O(t-Bu) | O |
| A-3393 | *c*-Pr | H | Cl | F | CH₂CH₂CH₂CH₂CH₂C(=O)O(t-Bu) | O |
| A-3394 | *c*-Pr | H | Cl | F | CH₂CH₂CH₂CH₂CH₂CH₂C(=O)O(t-Bu) | O |
| A-3395 | *c*-Pr | H | Cl | F | CH₂CH₂CH₂CH₂CH₂CH₂CH₂C(=O)OEt | O |
| A-3396 | *c*-Pr | H | Cl | F | CH₂CH₂C(=O)OCH₂CF₃ | O |
| A-3397 | *c*-Pr | H | Cl | F | CH₂CH₂CH₂C(=O)OCH₂CF₃ | O |
| A-3398 | *c*-Pr | H | Cl | F | CH₂CH₂CH₂CH₂C(=O)OCH₂CF₃ | O |
| A-3399 | *c*-Pr | H | Cl | F | CH₂CH₂CH₂CH₂CH₂C(=O)OCH₂CF₃ | O |
| A-3400 | *c*-Pr | H | Cl | F | CH₂CH₂C(=O)NH(t-Bu) | O |
| A-3401 | *c*-Pr | H | Cl | F | CH₂CH₂CH₂C(=O)NH(t-Bu) | O |
| A-3402 | *c*-Pr | H | Cl | F | CH₂CH₂CH₂C(=O)NH(t-Pen) | O |
| A-3403 | *c*-Pr | H | Cl | F | CH₂CH₂CH₂CH₂C(=O)NH(t-Bu) | O |
| A-3404 | *c*-Pr | H | Cl | F | CH₂CH₂C(=O)NHCH₂CF₃ | O |
| A-3405 | *c*-Pr | H | Cl | F | CH₂CH₂CH₂C(=O)NHCH₂CF₃ | O |
| A-3406 | *c*-Pr | H | Cl | F | CH₂CH₂CH₂CH₂C(=O)NHCH₂CF₃ | O |
| A-3407 | *c*-Pr | H | Cl | F | CH₂CH₂SC(=O)N(Me)₂ | O |
| A-3408 | *c*-Pr | H | Cl | F | CH₂CH₂CH₂SC(=O)N(Me)₂ | O |
| A-3409 | *c*-Pr | H | Cl | F | CH₂CH₂CH₂SC(=O)NH(t-Bu) | O |
| A-3410 | *c*-Pr | H | Cl | F | CH₂CH₂CH₂CH₂CH₂SC(=O)NH(t-Bu) | O |
| A-3411 | *c*-Pr | H | Cl | F | CH₂CH₂SC(=O)NHCH₂CF₃ | O |
| A-3412 | c-Pr | H | Cl | F | CH₂CH₂CH₂SC(=O)NHCH₂CF₃ | O |
| A-3413 | c-Pr | H | Cl | F | CH₂CH₂CH₂CH₂SC(=O)NHCH₂CF₃ | O |
| A-3414 | c-Pr | H | Cl | F | CH₂CH₂CH₂OC(=O)H | O |
| A-3415 | c-Pr | H | Cl | F | CH₂CH₂CH₂CH₂OC(=O)H | O |
| A-3416 | c-Pr | H | Cl | F | CH₂CH₂CH₂CH₂CH₂OC(=O)H | O |
| A-3417 | *c*-Pr | H | Cl | F | CH₂CH₂CH₂OC(=O)Me | O |
| A-3418 | *c*-Pr | H | Cl | F | CH₂CH₂CH₂CH₂OC(=O)Me | O |
| A-3419 | *c*-Pr | H | Cl | F | CH₂CH₂CH₂CH₂CH₂OC(=O)Me | O |
| A-3420 | *c*-Pr | H | Cl | F | CH₂CH₂OC(=O)CF₃ | O |
| A-3421 | *c*-Pr | H | Cl | F | CH₂CH₂CH₂OC(=O)CF₃ | O |
| A-3422 | *c*-Pr | H | Cl | F | CH₂CH₂CH₂CH₂OC(=O)CF₃ | O |
| A-3423 | *c*-Pr | H | Cl | F | CH₂CH₂CH₂CH₂CH₂OC(=O)Ph(4-CF₃) | O |
| A-3424 | *c*-Pr | H | Cl | F | CH₂CH₂OC(=O)Ph | O |
| A-3425 | *c*-Pr | H | Cl | F | CH₂CH₂OC(=O)Ph(4-CF₃) | O |
| A-3426 | *c*-Pr | H | Cl | F | CH₂CH₂CH₂OC(=O)Ph(4-CF₃) | O |

**[Table 79]**

| Compound Number | R¹ | R² | R³ | R⁴ | R⁵ | A |
|---|---|---|---|---|---|---|
| A-3427 | c-Pr | H | Cl | F | CH₂CH₂CH₂CH₂OC(=O)Ph(4-CF₃) | O |
| A-3428 | c-Pr | H | Cl | F | CH₂CH₂CH₂CH₂CH₂OC(=O)Ph(4-CF₃) | O |
| A-3429 | c-Pr | H | Cl | F | CH₂CH₂OS(=O)Me | O |
| A-3430 | c-Pr | H | Cl | F | CH₂CH₂CH₂OS(=O)Me | O |
| A-3431 | c-Pr | H | Cl | F | CH₂CH₂CH₂CH₂OS(=O)Me | O |
| A-3432 | *c*-Pr | H | Cl | F | CH₂CH₂CH₂CH₂CH₂OS(=O)Me | O |
| A-3433 | c-Pr | H | Cl | F | CH₂CH₂CH₂CH₂CH₂CH₂OS(=O)Me | O |
| A-3434 | c-Pr | H | Cl | F | CH₂CH₂OS(=O)CF₃ | O |
| A-3435 | c-Pr | H | Cl | F | CH₂CH₂CH₂OS(=O)CF₃ | O |
| A-3436 | c-Pr | H | Cl | F | CH₂CH₂CH₂CH₂OS(=O)CF₃ | O |
| A-3437 | c-Pr | H | Cl | F | CH₂CH₂CH₂CH₂CH₂OS(=O)CF₃ | O |
| A-3438 | c-Pr | H | Cl | F | CH₂CH₂CH₂CH₂CH₂CH₂OS(=O)CF₃ | O |
| A-3439 | *c*-Pr | H | Cl | F | CH₂CH₂OS(=O)Ph | O |
| A-3440 | c-Pr | H | Cl | F | CH₂CH₂OS(=O)Ph(4-Me) | O |
| A-3441 | c-Pr | H | Cl | F | CH₂CH₂CH₂OS(=O)Ph | O |
| A-3442 | c-Pr | H | Cl | F | CH₂CH₂CH₂OS(=O)Ph(4-Me) | O |
| A-3443 | c-Pr | H | Cl | F | CH₂CH₂OS(=O)₂Me | O |
| A-3444 | *c*-Pr | H | Cl | F | CH₂CH₂CH₂OS(=O)₂Me | O |
| A-3445 | c-Pr | H | Cl | F | CH₂CH₂CH₂OS(=O)₂CF₃ | O |
| A-3446 | c-Pr | H | Cl | F | CH₂CH₂CH₂CH₂OS(=O)₂CF₃ | O |
| A-3447 | c-Pr | H | Cl | F | CH₂CH₂CH₂CH₂CH₂OS(=O)₂CF₃ | O |
| A-3448 | c-Pr | H | Cl | F | CH₂CH₂CH₂CH₂CH₂OS(=O)₂CF₂CF₂CF₂CF₃ | O |
| A-3449 | c-Pr | H | Cl | F | CH₂CH₂OS(=O)₂Ph | O |
| A-3450 | c-Pr | H | Cl | F | CH₂CH₂OS(=O)₂Ph(4-Me) | O |
| A-3451 | c-Pr | H | Cl | F | CH₂CH₂CH₂OS(=O)₂Ph | O |
| A-3452 | c-Pr | H | Cl | F | CH₂CH₂CH₂OS(=O)₂Ph(4-Me) | O |
| A-3453 | c-Pr | H | Cl | F | CH₂CH₂NH₂ | O |
| A-3454 | c-Pr | H | Cl | F | CH₂CH₂CH₂NH₂ | O |
| A-3455 | c-Pr | H | Cl | F | CH₂CH₂CH₂CH₂NH₂ | O |
| A-3456 | c-Pr | H | Cl | F | CH₂CH₂CH₂CH₂CH₂NH₂ | O |
| A-3457 | c-Pr | H | Cl | F | CH₂CH₂CH₂CH₂CH₂CH₂NH₂ | O |
| A-3458 | c-Pr | H | Cl | F | CH₂CH₂CH₂CH₂CH₂CH₂CH₂NH₂ | O |
| A-3459 | c-Pr | H | Cl | F | CH₂CH(Me)NH₂ | O |
| A-3460 | c-Pr | H | Cl | F | CH₂C*H(Me)NH₂: (R) | O |
| A-3461 | c-Pr | H | Cl | F | CH₂C*H(Me)NH₂: (S) | O |
| A-3462 | c-Pr | H | Cl | F | CH₂CH(Et)NH₂ | O |
| A-3463 | c-Pr | H | Cl | F | CH₂CH(*i*-Pr)NH₂ | O |
| A-3464 | c-Pr | H | Cl | F | CH(Me)CH₂NH₂ | O |
| A-3465 | c-Pr | H | Cl | F | CH(Et)CH₂NH₂ | O |
| A-3466 | c-Pr | H | Cl | F | CH(*i*-Pr)CH₂NH₂ | O |
| A-3467 | c-Pr | H | Cl | F | CH₂CH₂CH(Me)NH₂ | O |
| A-3468 | c-Pr | H | Cl | F | CH₂CH(Me)CH₂NH₂ | O |
| A-3469 | c-Pr | H | Cl | F | CH(Me)CH₂CH₂NH₂ | O |
| A-3470 | *c*-Pr | H | Cl | F | CH₂CH(Me)NH(*c*-Pr) | O |

**[Table 80]**

| Compound Number | R¹ | R² | R³ | R⁴ | R⁵ | A |
|---|---|---|---|---|---|---|
| A-3471 | c-Pr | H | Cl | F | CH₂CH(Me)NHOMe | O |
| A-3472 | c-Pr | H | Cl | F | CH₂CH(Me)N(OMe)S(=O)₂Ph(2-NO₂) | O |
| A-3473 | c-Pr | H | Cl | F | CH₂CH(Me)N(OMe)S(=O)₂Ph(4-NO₂) | O |
| A-3474 | c-Pr | H | Cl | F | CH₂CH₂N(Me)(*t*-Bᵤ) | O |
| A-3475 | c-Pr | H | Cl | F | CH₂CH₂CH₂N(Me)(*t*-Bu) | O |
| A-3476 | c-Pr | H | Cl | F | CH₂CH₂CH₂CH₂N(Me)(*t*-Bu) | O |
| A-3477 | c-Pr | H | Cl | F | CH₂CH₂NHCH₂CF₃ | O |
| A-3478 | c-Pr | H | Cl | F | CH₂CH₂CH₂NHCH₂CF₃ | O |
| A-3479 | c-Pr | H | Cl | F | CH₂CH₂CH₂CH₂NHCH₂CF₃ | O |
| A-3480 | c-Pr | H | Cl | F | CH₂CH₂NHC(=O)C(Me)(CF₃)₂ | O |
| A-3481 | c-Pr | H | Cl | F | CH₂CH₂NHC(=O)Me | O |
| A-3482 | c-Pr | H | Cl | F | CH₂CH₂NHC(=O)(*t*-Bu) | O |
| A-3483 | c-Pr | H | Cl | F | CH₂CH₂CH₂CH₂NHC(=O)CH(CH₃)₂ | O |
| A-3484 | c-Pr | H | Cl | F | CH₂CH₂CH₂CH₂NHC(=O)(*t*-Bu) | O |
| A-3485 | c-Pr | H | Cl | F | CH₂CH₂CH₂CH₂NHC(=O)CH₂(*t*-Bu) | O |
| A-3486 | c-Pr | H | Cl | F | CH₂CH₂NHC(=O)CF₃ | O |
| A-3487 | c-Pr | H | Cl | F | CH₂CH₂NHC(=O)CH₂CF₃ | O |
| A-3488 | c-Pr | H | Cl | F | CH₂CH₂NHC(=O)CF₂CF₃ | O |
| A-3489 | c-Pr | H | Cl | F | CH₂CH₂CH₂NHC(=O)CF₃ | O |
| A-3490 | c-Pr | H | Cl | F | CH₂CH₂CH₂NHC(=O)CH₂CF₃ | O |
| A-3491 | c-Pr | H | Cl | F | CH₂CH₂CH₂NHC(=O)CF₂CF₃ | O |
| A-3492 | c-Pr | H | Cl | F | CH₂CH₂CH₂CH₂NHC(=O)CF₃ | O |
| A-3493 | c-Pr | H | Cl | F | CH₂CH₂CH₂CH₂NHC(=O)CH₂CF₃ | O |
| A-3494 | c-Pr | H | Cl | F | CH₂CH₂CH₂CH₂NHC(=O)CF₂CF₃ | O |
| A-3495 | c-Pr | H | Cl | F | CH₂CH₂CH₂CH₂NHC(=O)CF(CF₃)₂ | O |
| A-3496 | c-Pr | H | Cl | F | CH₂CH₂CH₂CH₂NHC(=O)CCl₃ | O |
| A-3497 | c-Pr | H | Cl | F | CH₂CH(Me)NHC(=O)CF₃ | O |
| A-3498 | c-Pr | H | Cl | F | CH₂CH₂NHC(=O)Ph | O |
| A-3499 | c-Pr | H | Cl | F | CH₂CH₂NHC(=O)Ph(4-CF₃) | O |
| A-3500 | c-Pr | H | Cl | F | CH₂CH₂CH₂NHC(=O)Ph(4-CF₃) | O |
| A-3501 | c-Pr | H | Cl | F | CH₂CH₂CH₂CH₂NHC(=O)Ph(4-CF₃) | O |
| A-3502 | c-Pr | H | Cl | F | CH₂CH₂NHC(=O)O(*t*-Bu) | O |
| A-3503 | c-Pr | H | Cl | F | CH₂CH₂CH₂NHC(=O)O(*t*-Bu) | O |
| A-3504 | c-Pr | H | Cl | F | CH₂CH₂CH₂CH₂NHC(=O)O(*t*-Bu) | O |
| A-3505 | c-Pr | H | Cl | F | CH₂CH₂CH₂CH₂NHC(=O)OCH(CH₃)₂ | O |
| A-3506 | c-Pr | H | Cl | F | CH₂CH(Me)NHC(=O)O(t-Bu) | O |
| A-3507 | c-Pr | H | Cl | F | CH₂C*H(Me)NHC(=O)O(*t*-Bu) : (R) | O |
| A-3508 | c-Pr | H | Cl | F | CH₂C*H(Me)NHC(=O)O(*t*-Bu) : (S) | O |
| A-3509 | c-Pr | H | Cl | F | CH₂CH(Et)NHC(=O)O(*t*-Bu) | O |
| A-351 0 | c-Pr | H | Cl | F | CH₂CH(*i*-Pr)NHC(=O)O(t-Bu) | O |
| A-3511 | c-Pr | H | Cl | F | CH(Me)CH₂NHC(=O)O(*t*-Bu) | O |
| A-3512 | c-Pr | H | Cl | F | CH(Et)CH₂NHC(=O)O(*t*-Bu) | O |
| A-3513 | c-Pr | H | Cl | F | CH(*i*-Pr)CH₂NHC(=O)O(t-Bu) | O |
| A-3514 | *c*-Pr | H | Cl | F | CH₂CH(Me)CH₂NHC(=O)O(*t*-Bu) | O |

**[Table 81]**

| Compound Number | R¹ | R² | R³ | R⁴ | R⁵ | A |
|---|---|---|---|---|---|---|
| A-3515 | c-Pr | H | Cl | F | CH₂CH₂NHC(=O)OC(Me)₂CF₃ | O |
| A-3516 | c-Pr | H | Cl | F | CH₂CH₂CH₂NHC(=O)OCH₂CF₃ | O |
| A-3517 | c-Pr | H | Cl | F | CH₂CH₂CH₂NHC(=O)OCH₂CH₂CF₃ | O |
| A-3518 | c-Pr | H | Cl | F | CH₂CH₂CH₂CH₂NHC(=O)OCH₂CCl₃ | O |
| A-3519 | c-Pr | H | Cl | F | CH₂CH₂CH₂CH₂NHC(=O)OCH₂CF₃ | O |
| A-3520 | c-Pr | H | Cl | F | CH₂CH₂NHC(=O)NH(*t*-Bu) | O |
| A-3521 | c-Pr | H | Cl | F | CH₂CH₂CH₂NHC(=O)NH(*t*-Bu) | O |
| A-3522 | c-Pr | H | Cl | F | CH₂CH₂CH₂CH₂NHC(=O)NHEt | O |
| A-3523 | c-Pr | H | Cl | F | CH₂CH₂CH₂CH₂NHC(=O)NH(*t*-Bu) | O |
| A-3524 | c-Pr | H | Cl | F | CH₂CH₂CH₂NHC(=O)NHCH₂CCl₃ | O |
| A-3525 | c-Pr | H | Cl | F | CH₂CH₂CH₂NHC(=O)NHCH₂CH₂F | O |
| A-3526 | c-Pr | H | Cl | F | CH₂CH₂CH₂NHC(=O)NHCH₂CF₃ | O |
| A-3527 | c-Pr | H | Cl | F | CH₂CH₂CH₂CH₂NHC(=O)NHCH₂CCl₃ | O |
| A-3528 | c-Pr | H | Cl | F | CH₂CH₂CH₂CH₂NHC(=O)NHCH₂CH₂F | O |
| A-3529 | c-Pr | H | Cl | F | CH₂CH₂CH₂CH₂NHC(=O)NHCH₂CF₃ | O |
| A-3530 | c-Pr | H | Cl | F | CH₂CH₂NHS(=O)Me | O |
| A-3531 | c-Pr | H | Cl | F | CH₂CH₂CH₂NHS(=O)Me | O |
| A-3532 | c-Pr | H | Cl | F | CH₂CH₂CH₂CH₂NHS(=O)Me | O |
| A-3533 | c-Pr | H | Cl | F | CH₂CH₂CH₂CH₂NHS(=O)CH(CH₃)₂ | O |
| A-3534 | c-Pr | H | Cl | F | CH₂CH₂CH₂CH₂CH₂NHS(=O)Me | O |
| A-3535 | c-Pr | H | Cl | F | CH₂CH₂NHS(=O)CHF₂ | O |
| A-3536 | c-Pr | H | Cl | F | CH₂CH₂CH₂NHS(=O)CHF₂ | O |
| A-3537 | c-Pr | H | Cl | F | CH₂CH₂CH₂CH₂NHS(=O)CHF₂ | O |
| A-3538 | c-Pr | H | Cl | F | CH₂CH₂CH₂CH₂CH₂NHS(=O)CHF₂ | O |
| A-3539 | c-Pr | H | Cl | F | CH₂CH(Me)NHS(=O)CHF₂ | O |
| A-3540 | c-Pr | H | Cl | F | CH(Me)CH₂NHS(=O)CHF₂ | O |
| A-3541 | c-Pr | H | Cl | F | CH₂CH₂CH(Me)NHS(=O)CHF₂ | O |
| A-3542 | c-Pr | H | Cl | F | CH₂CH(Me)CH₂NHS(=O)CHF₂ | O |
| A-3543 | c-Pr | H | Cl | F | CH(Me)CH₂CH₂NHS(=O)CHF₂ | O |
| A-3544 | c-Pr | H | Cl | F | CH₂CH₂NHS(=O)CF₃ | O |
| A-3545 | c-Pr | H | Cl | F | CH₂CH₂CH₂NHS(=O)CF₃ | O |
| A-3546 | c-Pr | H | Cl | F | CH₂CH₂CH₂CH₂NHS(=O)CF₃ | O |
| A-3547 | c-Pr | H | Cl | F | CH₂CH₂CH₂CH₂CH₂NHS(=O)CF₃ | O |
| A-3548 | c-Pr | H | Cl | F | CH₂CH₂CH₂NHS(=O)Ph | O |
| A-3549 | c-Pr | H | Cl | F | CH₂CH₂CH₂CH₂NHS(=O)Ph | O |
| A-3550 | c-Pr | H | Cl | F | CH₂CH₂CH₂CH₂CH₂NHS(=O)Ph | O |
| A-3551 | c-Pr | H | Cl | F | CH₂CH₂CH₂CH₂CH₂NHS(=O)Ph(4-CF₃) | O |
| A-3552 | c-Pr | H | Cl | F | CH₂CH(Me)NHS(=O)Ph(4-CF₃) | O |
| A-3553 | c-Pr | H | Cl | F | CH₂CH(Et)NHS(=O)Ph(4-CF₃) | O |
| A-3554 | c-Pr | H | Cl | F | CH(Me)CH₂NHS(=O)Ph(4-CF₃) | O |
| A-3555 | c-Pr | H | Cl | F | CH(Et)CH₂NHS(=O)Ph(4-CF₃) | O |
| A-3556 | c-Pr | H | Cl | F | CH₂CH(Et)CH₂NHS(=O)Ph(4-CF₃) | O |
| A-3557 | c-Pr | H | Cl | F | CH₂CH₂NHS(=O)₂Me | O |
| A-3558 | c-Pr | H | Cl | F | CH₂CH₂CH₂NHS(=O)₂Me | O |

**[Table 82]**

| Compound Number | R¹ | R² | R³ | R⁴ | R⁵ | A |
|---|---|---|---|---|---|---|
| A-3559 | c-Pr | H | Cl | F | CH₂CH₂CH₂CH₂NHS(=O)₂Me | O |
| A-3560 | c-Pr | H | Cl | F | CH₂CH₂CH₂CH₂NHS(=O)₂CH(CH₃)₂ | O |
| A-3561 | c-Pr | H | Cl | F | CH₂CH₂CH₂CH₂CH₂NHS(=O)₂Me | O |
| A-3562 | c-Pr | H | Cl | F | CH₂CH₂NHS(=O)₂CHF₂ | O |
| A-3563 | c-Pr | H | Cl | F | CH₂CH₂CH₂NHS(=O)₂CHF₂ | O |
| A-3564 | *c*-Pr | H | Cl | F | CH₂CH₂CH₂CH₂NHS(=O)₂CHF₂ | O |
| A-3565 | c-Pr | H | Cl | F | CH₂CH₂CH₂CH₂CH₂NHS(=O)₂CHF₂ | O |
| A-3566 | c-Pr | H | Cl | F | CH₂CH(Me)NHS(=O)₂CHF₂ | O |
| A-3567 | c-Pr | H | Cl | F | CH(Me)CH₂NHS(=O)₂CHF₂ | O |
| A-3568 | c-Pr | H | Cl | F | CH₂CH₂CH(Me)NHS(=O)₂CHF₂ | O |
| A-3569 | c-Pr | H | Cl | F | CH₂CH(Me)CH₂NHS(=O)₂CHF₂ | O |
| A-3570 | c-Pr | H | Cl | F | CH(Me)CH₂CH₂NHS(=O)₂CHF₂ | O |
| A-3571 | *c*-Pr | H | Cl | F | CH₂CH(Me)N(Me)S(=O)₂CHF₂ | O |
| A-3572 | c-Pr | H | Cl | F | CH(Me)CH₂N(Me)S(=O)₂CHF₂ | O |
| A-3573 | c-Pr | H | Cl | F | CH₂CH₂CH(Me)N(Me)S(=O)₂CHF₂ | O |
| A-3574 | c-Pr | H | Cl | F | CH₂CH(Me)CH₂N(Me)S(=O)₂CHF₂ | O |
| A-3575 | c-Pr | H | Cl | F | CH(Me)CH₂CH₂N(Me)S(=O)₂CHF₂ | O |
| A-3576 | *c*-Pr | H | Cl | F | CH₂CH₂CH₂NHS(=O)₂Ph | O |
| A-3577 | c-Pr | H | Cl | F | CH₂CH₂CH₂CH₂NHS(=O)₂Ph | O |
| A-3578 | c-Pr | H | Cl | F | CH₂CH₂CH₂CH₂CH₂NHS(=O)₂Ph | O |
| A-3579 | c-Pr | H | Cl | F | CH₂CH₂CH₂CH₂CH₂NHS(=O)₂Ph(4-CF₃) | O |
| A-3580 | c-Pr | H | Cl | F | CH₂CH(Me)NHS(=O)₂Ph(4-CF₃) | O |
| A-3581 | c-Pr | H | Cl | F | CH₂CH(Et)NHS(=O)₂Ph(4-CF₃) | O |
| A-3582 | c-Pr | H | Cl | F | CH(Me)CH₂NHS(=O)₂Ph(4-CF₃) | O |
| A-3583 | c-Pr | H | Cl | F | CH(Et)CH₂NHS(=O)₂Ph(4-CF₃) | O |
| A-3584 | c-Pr | H | Cl | F | CH₂CH(Me)CH₂NHS(=O)₂Ph(4-CF₃) | O |
| A-3585 | c-Pr | H | Cl | F | CH₂CH₂SiMe₃ | O |
| A-3586 | c-Pr | H | Cl | F | CH₂CH₂CH₂SiMe₃ | O |
| A-3587 | c-Pr | H | Cl | F | CH₂CH₂CH₂CH₂SiMe₃ | O |
| A-3588 | c-Pr | H | Cl | F | CH₂Ph | O |
| A-3589 | c-Pr | H | Cl | F | CH₂Ph(2-CF₃) | O |
| A-3590 | c-Pr | H | Cl | F | CH₂Ph(3-CF₃) | O |
| A-3591 | c-Pr | H | Cl | F | CH₂Ph(4-CF₃) | O |
| A-3592 | c-Pr | H | Cl | F | CH₂Ph(2-OCF₃) | O |
| A-3593 | c-Pr | H | Cl | F | CH₂Ph(3-OCF₃) | O |
| A-3594 | c-Pr | H | Cl | F | CH₂Ph(4-OCF₃) | O |
| A-3595 | c-Pr | H | Cl | F | CH₂Ph(2-SCF₃) | O |
| A-3596 | c-Pr | H | Cl | F | CH₂Ph(3-SCF₃) | O |
| A-3597 | c-Pr | H | Cl | F | CH₂Ph(4-SCF₃) | O |
| A-3598 | c-Pr | H | Cl | F | CH₂Ph(3-CH₂SCF₃) | O |
| A-3599 | c-Pr | H | Cl | F | CH₂Ph(4-F) | O |
| A-3600 | c-Pr | H | Cl | F | CH₂Ph(4-Cl) | O |
| A-3601 | c-Pr | H | Cl | F | CH₂Ph(4-Br) | O |
| A-3602 | *c*-Pr | H | Cl | F | CH₂Ph(4-Me) | O |

**[Table 83]**

| Compound Number | R¹ | R² | R³ | R⁴ | R⁵ | A |
|---|---|---|---|---|---|---|
| A-3603 | c-Pr | H | Cl | F | CH₂Ph[4-(*t*-Bu)] | O |
| A-3604 | c-Pr | H | Cl | F | CH₂Ph(4-CN) | O |
| A-3605 | c-Pr | H | Cl | F | CH₂Ph(4-NO₂) | O |
| A-3606 | c-Pr | H | Cl | F | CH₂Ph(4-OCHF₂) | O |
| A-3607 | c-Pr | H | Cl | F | CH₂Ph(4-SCHF₂) | O |
| A-3608 | c-Pr | H | Cl | F | CH₂Ph(4-CH₂SCF₃) | O |
| A-3609 | c-Pr | H | Cl | F | CH₂Ph[4-CF(CF₃)₂] | O |
| A-3610 | c-Pr | H | Cl | F | CH₂Ph(4-CH₂SCF₃) | O |
| A-3611 | c-Pr | H | Cl | F | CH₂Ph[4-Ph(4-CF₃)] | O |
| A-3612 | c-Pr | H | Cl | F | CH₂Ph(2,4-Cl₂) | O |
| A-3613 | c-Pr | H | Cl | F | CH₂Ph[2,5-(CF₃)₂] | O |
| A-3614 | c-Pr | H | Cl | F | CH₂Ph(3,4-Cl₂) | O |
| A-3615 | c-Pr | H | Cl | F | CH₂Ph(3-CF₃-4-F) | O |
| A-3616 | c-Pr | H | Cl | F | CH₂Ph(3-CF₃-4-Cl) | O |
| A-3617 | c-Pr | H | Cl | F | CH₂Ph(3-F-4-CF₃) | O |
| A-3618 | c-Pr | H | Cl | F | CH₂Ph(2,4,6-F₃) | O |
| A-3619 | c-Pr | H | Cl | F | CH₂Ph(3,4,5-F₃) | O |
| A-3620 | c-Pr | H | Cl | F | CH₂Ph(2,3,4-F₃) | O |
| A-3621 | c-Pr | H | Cl | F | CH₂Ph(3,4,5-Cl₃) | O |
| A-3622 | c-Pr | H | Cl | F | CH₂CH₂Ph | O |
| A-3623 | c-Pr | H | Cl | F | CH₂CH₂Ph(4-F) | O |
| A-3624 | c-Pr | H | Cl | F | CH₂CH₂Ph(4-Cl) | O |
| A-3625 | c-Pr | H | Cl | F | CH₂CH₂Ph(4-Br) | O |
| A-3626 | c-Pr | H | Cl | F | CH₂CH₂Ph[4-(*t*-Bu)] | O |
| A-3627 | c-Pr | H | Cl | F | CH₂CH₂Ph(2-CF₃) | O |
| A-3628 | c-Pr | H | Cl | F | CH₂CH₂Ph(3-CF₃) | O |
| A-3629 | c-Pr | H | Cl | H | CH₂CH₂Ph(4-CF₃) | O |
| A-3630 | c-Pr | H | Cl | F | CH₂CH₂Ph(4-CF₃) | O |
| A-3631 | c-Pr | H | Cl | F | CH₂CH₂Ph(4-CF(CF₃)₂) | O |
| A-3632 | c-Pr | H | Cl | F | CH₂CH₂Ph[4-(c-Pr)] | O |
| A-3633 | c-Pr | H | Cl | F | CH₂CH₂Ph{4-[*c*-Pr(2,2-F₂)]} | O |
| A-3634 | c-Pr | H | Cl | F | CH₂CH₂Ph(4-OMe) | O |
| A-3635 | c-Pr | H | Cl | F | CH₂CH₂Ph(3-OCF₃) | O |
| A-3636 | c-Pr | H | Cl | F | CH₂CH₂Ph(4-OCHF₂) | O |
| A-3637 | c-Pr | H | Cl | F | CH₂CH₂Ph(4-OCF₃) | O |
| A-3638 | c-Pr | H | Cl | F | CH₂CH₂Ph(4-SMe) | O |
| A-3639 | c-Pr | H | Cl | F | CH₂CH₂Ph(4-SCHF₂) | O |
| A-3640 | c-Pr | H | Cl | F | CH₂CH₂Ph(4-SCF₃) | O |
| A-3641 | c-Pr | H | Cl | F | CH₂CH₂Ph[4-S(=O)Me] | O |
| A-3642 | c-Pr | H | Cl | F | CH₂CH₂Ph[4-S(=O)CF₃] | O |
| A-3643 | c-Pr | H | Cl | F | CH₂CH₂Ph[4-S(=O)₂Me] | O |
| A-3644 | c-Pr | H | Cl | F | CH₂CH₂Ph[4-S(=O)₂CF₃] | O |
| A-3645 | c-Pr | H | Cl | F | CH₂CH₂Ph(4-CH₂SMe) | O |
| A-3646 | c-Pr | H | Cl | F | CH₂CH₂Ph(4-CH₂SCF₃) | O |

**[Table 84]**

| Compound Number | R¹ | R² | R³ | R⁴ | R⁵ | A |
|---|---|---|---|---|---|---|
| A-3647 | c-Pr | H | Cl | F | CH₂CH₂Ph[4-OS(=O)₂Me] | O |
| A-3648 | c-Pr | H | Cl | F | CH₂CH₂Ph[4-OS(=O)₂CF₃] | O |
| A-3649 | c-Pr | H | Cl | F | CH₂CH₂Ph[4-Ph(4-CF₃)] | O |
| A-3650 | c-Pr | H | Cl | F | CH₂CH₂Ph(4-CH₂Ph) | O |
| A-3651 | c-Pr | H | Cl | F | CH₂CH₂Ph(4-OCH₂Ph) | O |
| A-3652 | c-Pr | H | Cl | F | CH₂CH₂Ph(4-CN) | O |
| A-3653 | c-Pr | H | Cl | F | CH₂CH₂Ph(4-NO₂) | O |
| A-3654 | c-Pr | H | Cl | F | CH₂CH₂Ph(2,4-Cl₂) | O |
| A-3655 | c-Pr | H | Cl | F | CH₂CH₂Ph(3,4-Cl₂) | O |
| A-3656 | c-Pr | H | Cl | F | CH₂CH₂Ph(3-CF₃-4-F) | O |
| A-3657 | c-Pr | H | Cl | F | CH₂CH₂Ph(2-CF₃-4-F) | O |
| A-3658 | c-Pr | H | Cl | F | CH₂CH₂Ph(3-F-4-CF₃) | O |
| A-3659 | c-Pr | H | Cl | F | CH₂CH₂Ph(2-F-4-CF₃) | O |
| A-3660 | c-Pr | H | Cl | F | CH₂CH₂Ph(3-Cl-4-OCHF₂) | O |
| A-3661 | c-Pr | H | Cl | F | CH₂CH₂Ph(3,4,5-Cl₃) | O |
| A-3662 | c-Pr | H | Cl | F | CH₂CH₂Ph(2,3,4-F₃) | O |
| A-3663 | c-Pr | H | Cl | F | CH₂CH₂Ph(2,4,5-F₃) | O |
| A-3664 | c-Pr | H | Cl | F | CH₂CH₂Ph(3,4,5-F₃) | O |
| A-3665 | c-Pr | H | Cl | F | CH₂CH₂Ph(2,4,6-F₃) | O |
| A-3666 | c-Pr | H | Cl | F | CH₂CH₂CH₂Ph | O |
| A-3667 | c-Pr | H | Cl | F | CH₂CH₂CH₂Ph(3-CF₃) | O |
| A-3668 | c-Pr | H | Cl | F | CH₂CH₂CH₂Ph(4-CF₃) | O |
| A-3669 | c-Pr | H | Cl | F | CH₂CH₂CH₂Ph(4-F) | O |
| A-3670 | c-Pr | H | Cl | F | CH₂CH₂CH₂Ph[4-(*t*-Bu)] | O |
| A-3671 | c-Pr | H | Cl | F | CH₂CH₂CH₂Ph(4-CN) | O |
| A-3672 | c-Pr | H | Cl | F | CH₂CH₂CH₂Ph(4-CF₃) | O |
| A-3673 | c-Pr | H | Cl | F | CH₂CH₂CH₂Ph(4-OCHF₂) | O |
| A-3674 | c-Pr | H | Cl | F | CH₂CH₂CH₂Ph(4-OCF₃) | O |
| A-3675 | c-Pr | H | Cl | F | CH₂CH₂CH₂Ph(4-SCHF₂) | O |
| A-3676 | c-Pr | H | Cl | F | CH₂CH₂CH₂Ph(4-SCF₃) | O |
| A-3677 | c-Pr | H | Cl | F | CH₂CH₂CH₂Ph[4-CF(CF₃)₂] | O |
| A-3678 | c-Pr | H | Cl | F | CH₂CH₂CH₂Ph(3,4,5-F₃) | O |
| A-3679 | c-Pr | H | Cl | F | CH₂CH₂CH₂Ph(2,4,6-F₃) | O |
| A-3680 | c-Pr | H | Cl | F | CH₂CH₂CH₂CH₂Ph | O |
| A-3681 | c-Pr | H | Cl | F | CH₂CH₂CH₂CH₂Ph(4-F) | O |
| A-3682 | c-Pr | H | Cl | F | CH₂CH₂CH₂CH₂Ph(4-CF₃) | O |
| A-3683 | c-Pr | H | Cl | F | CH₂CH₂CH₂CH₂Ph(4-OCF₃) | O |
| A-3684 | c-Pr | H | Cl | F | CH₂CH₂CH₂CH₂Ph(4-SCF₃) | O |
| A-3685 | c-Pr | H | Cl | F | CH₂CH₂CH₂CH₂CH₂Ph | O |
| A-3686 | c-Pr | H | Cl | F | CH₂CH₂CH₂CH₂CH₂CH₂Ph | O |
| A-3687 | c-Pr | H | Cl | F | CH₂CF₂Ph(4-F) | O |
| A-3688 | c-Pr | H | Cl | F | CH₂CF₂Ph(4-CF₃) | O |
| A-3689 | c-Pr | H | Cl | H | CH₂CF₂Ph(3,4,5-F₃) | O |
| A-3690 | c-Pr | H | Cl | F | CH₂CF₂Ph(3,4,5-F₃) | O |

**[Table 85]**

| Compound Number | R¹ | R² | R³ | R⁴ | R⁵ | A |
|---|---|---|---|---|---|---|
| A-3691 | c-Pr | H | Cl | F | CH₂CH₂OPh | O |
| A-3692 | c-Pr | H | Cl | F | CH₂CH₂OPh(4-F) | O |
| A-3693 | c-Pr | H | Cl | F | CH₂CH₂OPh(4-CF₃) | O |
| A-3694 | c-Pr | H | Cl | F | CH₂CH₂CH₂OPh | O |
| A-3695 | c-Pr | H | Cl | F | CH₂CH₂CH₂OPh(4-Cl) | O |
| A-3696 | c-Pr | H | Cl | F | CH₂CH₂CH₂OPh(4-CF₃) | O |
| A-3697 | c-Pr | H | Cl | F | CH₂CH₂CH₂CH₂0Ph(4-CF₃) | O |
| A-3698 | c-Pr | H | Cl | F | CH₂CH₂CH₂CH₂OPh(4-OCF₃) | O |
| A-3699 | c-Pr | H | Cl | F | CH₂CH₂CH₂CH₂CH₂OPh(4-CF₃) | O |
| A-3700 | c-Pr | H | Cl | F | CH₂CH₂OCH₂Ph | O |
| A-3701 | c-Pr | H | Cl | F | CH₂CH₂CH₂OCH₂Ph | O |
| A-3702 | c-Pr | H | Cl | F | CH₂CH₂CH₂CH₂OCH₂Ph | O |
| A-3703 | c-Pr | H | Cl | F | CH₂CH₂CH₂CH₂CH₂OCH₂Ph | O |
| A-3704 | c-Pr | H | Cl | F | CH₂CH₂SPh | O |
| A-3705 | c-Pr | H | Cl | F | CH₂CH₂CH₂SPh | O |
| A-3706 | c-Pr | H | Cl | F | CH₂CH₂CH₂SPh(4-F) | O |
| A-3707 | c-Pr | H | Cl | F | CH₂CH₂CH₂SPh(4-Cl) | O |
| A-3708 | c-Pr | H | Cl | F | CH₂CH₂CH₂SPh(4-Br) | O |
| A-3709 | c-Pr | H | Cl | F | CH₂CH₂CH₂SPh[4-(*t*-Bu)] | O |
| A-3710 | c-Pr | H | Cl | F | CH₂CH₂CH₂SPh(3-CF₃) | O |
| A-3711 | c-Pr | H | Cl | F | CH₂CH₂CH₂SPh(4-CF₃) | O |
| A-3712 | c-Pr | H | Cl | F | CH₂CH₂CH₂SPh(3-SCF₃) | O |
| A-3713 | c-Pr | H | Cl | F | CH₂CH₂CH₂SPh(4-SCF₃) | O |
| A-3714 | c-Pr | H | Cl | F | CH₂CH₂CH₂CH₂SPh | O |
| A-3715 | c-Pr | H | Cl | F | CH₂CH₂CH₂CH₂SPh(4-F) | O |
| A-3716 | c-Pr | H | Cl | F | CH₂CH₂CH₂CH₂SPh(4-Cl) | O |
| A-3717 | c-Pr | H | Cl | F | CH₂CH₂CH₂CH₂SPh[4-(*t*-Bu)] | O |
| A-3718 | c-Pr | H | Cl | F | CH₂CH₂CH₂CH₂SPh(4-CF₃) | O |
| A-3719 | c-Pr | H | Cl | F | CH₂CH₂CH₂CH₂CH₂SPh | O |
| A-3720 | c-Pr | H | Cl | F | CH₂CH₂CH₂CH₂CH₂SPh(4-F) | O |
| A-3721 | c-Pr | H | Cl | F | CH₂CH₂CH₂CH₂CH₂SPh(4-Cl) | O |
| A-3722 | c-Pr | H | Cl | F | CH₂CH₂CH₂CH₂CH₂SPh(4-CF₃) | O |
| A-3723 | c-Pr | H | Cl | F | CH₂CH₂S(=O)Ph | O |
| A-3724 | c-Pr | H | Cl | F | CH₂CH₂CH₂S(=O)Ph | O |
| A-3725 | c-Pr | H | Cl | F | CH₂CH₂CH₂S(=O)Ph(4-F) | O |
| A-3726 | c-Pr | H | Cl | F | CH₂CH₂CH₂S(=O)Ph[4-(*t-*Bu)] | O |
| A-3727 | c-Pr | H | Cl | F | CH₂CH₂CH₂S(=O)Ph(4-CF₃) | O |
| A-3728 | c-Pr | H | Cl | F | CH₂CH₂S(=O)₂Ph | O |
| A-3729 | c-Pr | H | Cl | F | CH₂CH₂S(=O)₂Ph(4-CF₃) | O |
| A-3730 | c-Pr | H | Cl | F | CH₂CH₂CH₂S(=O)₂Ph(4-F) | O |
| A-3731 | c-Pr | H | Cl | F | CH₂CH₂CH₂S(=O)₂Ph(4-Cl) | O |
| A-3732 | c-Pr | H | Cl | F | CH₂CH₂CH₂S(=O)₂Ph(4-CF₃) | O |
| A-3733 | c-Pr | H | Cl | F | CH₂CH₂CH₂CH₂S(=O)₂Ph | O |
| A-3734 | c-Pr | H | Cl | F | CH₂CH₂CH₂CH₂S(=O)₂Ph(4-Cl) | O |

**[Table 86]**

| Compound Number | R¹ | R² | R³ | R⁴ | R5 | A |
|---|---|---|---|---|---|---|
| A-3735 | c-Pr | H | Cl | F | CH₂CH₂CH₂CH₂S(=O)₂Ph(4-CF₃) | O |
| A-3736 | c-Pr | H | Cl | F | CH₂CH₂SCH₂Ph | O |
| A-3737 | c-Pr | H | Cl | F | CH₂CH₂CH₂SCH₂Ph | O |
| A-3738 | c-Pr | H | Cl | F | CH₂CH₂CH₂SCH₂Ph(2-Cl) | O |
| A-3739 | c-Pr | H | Cl | F | CH₂CH₂CH₂SCH₂Ph(3-Cl) | O |
| A-3740 | *c*-Pr | H | Cl | F | CH₂CH₂CH₂SCH₂Ph(4-Cl) | O |
| A-3741 | c-Pr | H | Cl | F | CH₂CH₂CH₂SCH₂Ph(2-SCF₃) | O |
| A-3742 | c-Pr | H | Cl | F | CH₂CH₂CH₂SCH₂Ph(3-CF₃) | O |
| A-3743 | c-Pr | H | Cl | F | CH₂CH₂CH₂SCH₂Ph(4-CF₃) | O |
| A-3744 | c-Pr | H | Cl | F | CH₂CH₂CH₂SCH₂Ph(4-NO₂) | O |
| A-3745 | c-Pr | H | Cl | F | CH₂CH₂CH₂CH₂SCH₂Ph | O |
| A-3746 | c-Pr | H | Cl | F | CH₂CH₂CH₂CH₂SCH₂Ph(4-Cl) | O |
| A-3747 | *c*-Pr | H | Cl | F | CH₂CH₂CH₂CH₂SCH₂Ph(4-CF₃) | O |
| A-3748 | c-Pr | H | Cl | F | CH₂CH₂CH₂CH₂SCH₂Ph(4-CN) | O |
| A-3749 | c-Pr | H | Cl | F | CH₂CH₂CH₂CH₂CH₂SCH₂Ph | O |
| A-3750 | c-Pr | H | Cl | F | CH₂CH₂SCH₂CH₂Ph | O |
| A-3751 | c-Pr | H | Cl | F | CH₂CH₂CH₂SCH₂CH₂Ph | O |
| A-3752 | *c*-Pr | H | Cl | F | CH₂CH₂CH₂CH₂SCH₂CH₂Ph | O |
| A-3753 | c-Pr | H | Cl | F | CH₂CH₂SCH(Me)Ph | O |
| A-3754 | c-Pr | H | Cl | F | CH₂CH₂CH₂SCH(Me)Ph | O |
| A-3755 | c-Pr | H | Cl | F | CH₂CH₂CH₂CH₂SCH(Me)Ph | O |
| A-3756 | c-Pr | H | Cl | F | CH₂CH₂S(=O)CH₂Ph | O |
| A-3757 | c-Pr | H | Cl | F | CH₂CH_{2S}(=O)CH₂Ph(4-CF₃) | O |
| A-3758 | c-Pr | H | Cl | F | CH₂CH₂CH₂S(=O)CH₂Ph | O |
| A-3759 | c-Pr | H | Cl | F | CH₂CH₂CH₂S(=O)CH₂Ph(2-SCF₃) | O |
| A-3760 | c-Pr | H | Cl | F | CH₂CH₂CH₂S(=O)CH₂Ph(4-SCF₃) | O |
| A-3761 | c-Pr | H | Cl | F | CH₂CH₂CH₂S(=O)CH₂Ph(4-Cl) | O |
| A-3762 | c-Pr | H | Cl | F | CH₂CH₂CH₂S(=O)CH₂Ph(4-CF₃) | O |
| A-3763 | c-Pr | H | Cl | F | CH₂CH₂CH₂CH₂S(=O)CH₂Ph | O |
| A-3764 | c-Pr | H | Cl | F | CH₂CH₂CH₂CH₂S(=O)CH₂Ph(4-CF₃) | O |
| A-3765 | c-Pr | H | Cl | F | CH₂CH₂CH₂CH₂CH₂S(=O)CH₂Ph(4-CF₃) | O |
| A-3766 | c-Pr | H | Cl | F | CH₂CH₂S(=O)₂CH₂Ph | O |
| A-3767 | c-Pr | H | Cl | F | CH₂CH₂S(=O)₂CH₂Ph(4-CF₃) | O |
| A-3768 | c-Pr | H | Cl | F | CH₂CH₂CH₂S(=O)₂CH₂Ph | O |
| A-3769 | c-Pr | H | Cl | F | CH₂CH₂CH₂S(=O)₂CH₂Ph(4-Cl) | O |
| A-3770 | c-Pr | H | Cl | F | CH₂CH₂CH₂S(=O)₂CH₂Ph(4-CF₃) | O |
| A-3771 | c-Pr | H | Cl | F | CH₂CH₂CH₂CH₂S(=O)₂CH₂Ph | O |
| A-3772 | c-Pr | H | Cl | F | CH₂CH₂CH₂CH₂S(=O)₂CH₂Ph(4-CF₃) | O |
| A-3773 | c-Pr | H | Cl | F | CH₂CH₂CH₂CH₂CH₂S(=O)₂CH₂Ph | O |
| A-3774 | c-Pr | H | Cl | F | CH₂CH₂CH₂CH₂CH₂S(=O)₂CH₂Ph(4-CF₃) | O |
| A-3775 | c-Pr | H | Cl | F | CH₂CH₂ON=CH(*t*-Bu) | O |
| A-3776 | c-Pr | H | Cl | F | CH₂CH₂CH₂ON=CH(*t*-Bu) | O |
| A-3777 | c-Pr | H | Cl | F | CH₂CH₂CH₂CH₂ON=C(Me)(*c*-Pr) | O |
| A-3778 | *c*-Pr | H | Cl | F | CH₂CH₂ON=C(Me)CF₃ | O |

**[Table 87]**

| Compound Number | R¹ | R² | R³ | R⁴ | R⁵ | A |
|---|---|---|---|---|---|---|
| A-3779 | *c*-Pr | H | Cl | F | CH₂CH₂CH₂ON=CHCF₃ | O |
| A-3780 | *c*-Pr | H | Cl | F | CH₂CH₂CH₂ON=C(Me)CF₃ | O |
| A-3781 | *c*-Pr | H | Cl | F | CH₂CH₂CH₂ON=C(Me)CCl₃ | O |
| A-3782 | *c*-Pr | H | Cl | F | CH₂CH₂CH₂CH₂ON=CHCF₃ | O |
| A-3783 | *c*-Pr | H | Cl | F | CH₂CH₂CH₂CH₂ON=C(Me)CF₃ | O |
| A-3784 | *c*-Pr | H | Cl | F | CH₂CH₂CH₂CH₂CH₂ON=C(Me)CF₃ | O |
| A-3785 | *c*-Pr | H | Cl | F | CH₂CH₂ON=CHPh | O |
| A-3786 | *c*-Pr | H | Cl | F | CH₂CH₂ON=CHPh(4-CF₃) | O |
| A-3787 | *c*-Pr | H | Cl | F | CH₂CH₂ON=CHPh(4-SCF₃) | O |
| A-3788 | *c*-Pr | H | Cl | F | CH₂CH₂CH₂ON=CHPh(3-CF₃) | O |
| A-3789 | *c*-Pr | H | Cl | F | CH₂CH₂CH₂ON=CHPh(4-CF₃) | O |
| A-3790 | *c*-Pr | H | Cl | F | CH₂CH₂CH₂ON=CHPh(4-SCF₃) | O |
| A-3791 | *c*-Pr | H | Cl | F | CH₂CH₂CH₂ON=C(Me)Ph(4-CF₃) | O |
| A-3792 | *c*-Pr | H | Cl | F | CH₂CH₂CH₂CH₂ON=CHPh(4-SCF₃) | O |
| A-3793 | *c*-Pr | H | Cl | F | CH₂CH₂(adamant-1-yl) | O |
| A-3794 | *c*-Pr | H | Cl | F | CH₂CH₂CH₂(3-CF₃-pyrazol-1-yl) | O |
| A-3795 | *c*-Pr | H | Cl | F | CH₂CH₂CH₂CH₂(3-CF₃-pyrazol-1-yl) | O |
| A-3796 | *c*-Pr | H | Cl | F | CH₂CH₂CH₂CH₂CH₂(3-CF₃-pyrazol-1-yl) | O |
| A-3797 | *c*-Pr | H | Cl | F | CH₂(1-Me-4-CF₃-imidazol-5-yl) | O |
| A-3798 | *c*-Pr | H | Cl | F | CH₂CH₂(1-Me-4-CF₃-imidazol-5-yl) | O |
| A-3799 | *c*-Pr | H | Cl | F | CH₂CH₂CH₂(1-Me-4-CF₃-imidazol-5-yl) | O |
| A-3800 | *c*-Pr | H | Cl | F | CH₂CH₂CH₂(3-CF₃-1,2,4-triazol-1-yl) | O |
| A-3801 | *c*-Pr | H | Cl | F | CH₂CH₂CH₂CH₂(3-CF₃-1,2,4-triazol-1-yl) | O |
| A-3802 | *c*-Pr | H | Cl | F | CH₂CH₂CH₂CH₂CH₂(3-CF₃-1,2,4-triazol-1-yl) | O |
| A-3803 | *c*-Pr | H | Cl | F | CH₂CH₂CH₂(4-CF₃-thiazol-2-yl) | O |
| A-3804 | *c*-Pr | H | Cl | F | CH₂CH₂CH₂CH₂(4-CF₃-thiazol-2-yl) | O |
| A-3805 | *c*-Pr | H | Cl | F | CH₂CH₂CH₂CH₂CH₂(4-CF₃-thiazol-2-yl) | O |
| A-3806 | *c*-Pr | H | Cl | F | CH₂CH₂CH₂(2-CF₃-pyridin-5-yl) | O |
| A-3807 | *c*-Pr | H | Cl | F | CH₂CH₂CH₂CH₂(2-CF₃-pyridin-5-yl) | O |
| A-3808 | *c*-Pr | H | Cl | F | CH₂CH₂CH₂CH₂CH₂(2-CF₃-pyridin-5-yl) | O |
| A-3809 | *c*-Pr | H | Cl | F | CH₂(4-CF₃-pyrimidin-2-yl) | O |
| A-3810 | *c*-Pr | H | Cl | F | CH₂CH₂(4-CF₃-pyrimidin-2-yl) | O |
| A-3811 | *c*-Pr | H | Cl | F | CH₂CH₂CH₂(4-CF₃-pyrimidin-2-yl) | O |
| A-3812 | *c*-Pr | H | Cl | F | CH₂CH₂O(3-Cl-5-CF₃-pyridin-2-yl) | O |
| A-3813 | *c*-Pr | H | Cl | F | CH₂CH₂CH₂O(3-Cl-5-CF₃-pyridin-2-yl) | O |
| A-3814 | *c*-Pr | H | Cl | F | CH₂CH₂CH₂CH₂O(3-Cl-5-CF₃-pyridin-2-yl) | O |
| A-3815 | *c*-Pr | H | Cl | F | CH₂CH₂CH₂CH₂CH₂O(3-Cl-5-CF₃-pyridin-2-yl) | O |
| A-3816 | *c*-Pr | H | Cl | F | CH₂CH₂N(Phth) | O |
| A-3817 | *c*-Pr | H | Cl | F | CH₂CH₂CH₂N(Phth) | O |
| A-3818 | *c*-Pr | H | Cl | F | CH₂CH₂CH₂CH₂N(Phth) | O |
| A-3819 | *c*-Pr | H | Cl | F | CH₂CH₂CH₂CH₂CH₂N(Phth) | O |
| A-3820 | *c*-Pr | H | Cl | F | CH₂CH(Me)CH₂N(Phth) | O |
| A-3821 | *c*-Pr | H | Cl | F | CH₂[1,3-dioxolan(2-CF₃)-2-yl] | O |
| A-3822 | c*-*Pr | H | Cl | F | CH₂(azetidin-3-yl) | O |

**[Table 88]**

| Compound Number | R¹ | R² | R³ | R⁴ | R⁵ | A |
|---|---|---|---|---|---|---|
| A-3823 | *c*-Pr | H | Cl | F | CH₂(pyrrolidin-3-yl) | O |
| A-3824 | *c*-Pr | H | Cl | F | CH₂(piperidin-3-yl) | O |
| A-3825 | *c*-Pr | H | Cl | F | CH₂{azetidin[1-C(=O)O(*t*-Bu)]-3-yl} | O |
| A-3826 | *c*-Pr | H | Cl | F | CH₂{pyrrolidin[1-C(=O)O(*t*-Bu)]-3-yl} | O |
| A-3827 | *c*-Pr | H | Cl | F | CH₂{piperidin[1-C(=O)O(*t*-Bu)]-3-yl} | O |
| A-3828 | *c*-Pr | H | Cl | F | CH₂{azetidin[1-C(=O)CF₃]-3-yl} | O |
| A-3829 | *c*-Pr | H | Cl | F | CH₂{azetidin[1-S(=O)₂CF₃]-3-yl} | O |
| A-3830 | *c*-Pr | H | Cl | F | CH₂{pyrrolidin[1-C(=O)CF₃]-3-yl} | O |
| A-3831 | *c*-Pr | H | Cl | F | CH₂{pyrrolidin[1-S(=O)₂CF₃]-3-yl} | O |
| A-3832 | *c*-Pr | H | Cl | F | CH₂{piperidin[1-C(=O)CF₃]-3-yl} | O |
| A-3833 | *c*-Pr | H | Cl | F | CH₂{piperidin[1-S(=O)₂CF₃]-3-yl} | O |
| A-3834 | *c*-Pr | H | Cl | F | CH₂CH₂CH₂CN | O |
| A-3835 | *c*-Pr | H | Cl | F | CH₂CH₂CH₂CH₂CN | O |
| A-3836 | *c*-Pr | H | Cl | F | CH₂CH₂CH₂CH₂CH₂CN | O |
| A-3837 | *c*-Pr | H | Cl | F | CH₂CH₂CH₂CH₂CH₂CH₂CN | O |
| A-3838 | *c*-Pr | H | Cl | F | CH₂CH₂CH₂C(=O)OH | O |
| A-3839 | *c*-Pr | H | Cl | F | CH₂CH₂CH₂CH₂C(=O)OH | O |
| A-3840 | *c*-Pr | H | Cl | F | CH₂CH₂CH₂CH₂CH₂C(=O)OH | O |
| A-3841 | *c*-Pr | H | Cl | F | CH₂CH₂SCN | O |
| A-3842 | *c*-Pr | H | Cl | F | CH₂CH₂CH₂SCN | O |
| A-3843 | *c*-Pr | H | Cl | F | CH₂CH₂CH₂CH₂SCN | O |
| A-3844 | *c*-Pr | H | Cl | F | CH₂CH₂CH₂CH₂CH₂SCN | O |
| A-3845 | *c*-Pr | H | Cl | Cl | CH₂CH₂CH₂CH₂CH₂SCN | O |
| A-3846 | *c*-Pr | H | Cl | F | CH₂CH₂CH₂CH₂CH₂CH₂SCN | O |
| A-3847 | *c*-Pr | H | Cl | F | CH₂CH₂CH₂CH₂CH₂CH₂CH₂SCN | O |
| A-3848 | *c*-Pr | H | Cl | F | CH₂CH₂CH₂CH₂CH₂CH₂CH₂CH₂SCN | O |
| A-3849 | *c*-Pr | H | Cl | F | CH₂CH₂CH(Me)SCN | O |
| A-3850 | *c*-Pr | H | Cl | F | CH₂CH(Me)CH₂SCN | O |
| A-3851 | *c*-Pr | H | Cl | F | CH(Me)CH₂CH₂SCN | O |
| A-3852 | *c*-Pr | H | Cl | F | CH₂CH₂CH₂CH(Me)SCN | O |
| A-3853 | *c*-Pr | H | Cl | F | CH₂CH₂CH(Me)CH₂SCN | O |
| A-3854 | *c*-Pr | H | Cl | F | CH₂CH(Me)CH₂CH₂SCN | O |
| A-3855 | *c*-Pr | H | Cl | F | CH(Me)CH₂CH₂CH₂SCN | O |
| A-3856 | *c*-Pr | H | Cl | F | CH₂CH₂CH₂CH₂CH(Me)SCN | O |
| A-3857 | *c*-Pr | H | Cl | F | CH₂CH₂CH₂CH(Me)CH₂SCN | O |
| A-3858 | *c*-Pr | H | Cl | F | CH₂CH₂CH(Me)CH₂CH₂SCN | O |
| A-3859 | *c*-Pr | H | Cl | F | CH₂CH(Me)CH₂CH₂CH₂SCN | O |
| A-3860 | *c*-Pr | H | Cl | F | CH(Me)CH₂CH₂CH₂CH₂SCN | O |
| A-3861 | *c-*Pr | H | Cl | F | CH₂CH₂ONH₂ | O |
| A-3862 | *c-*Pr | H | Cl | F | CH₂CH₂CH₂ONH₂ | O |
| A-3863 | *c*-Pr | H | Cl | F | CH₂CH₂CH₂CH₂ONH₂ | O |
| A-3864 | *c*-Pr | H | Cl | F | CH₂CH₂CH₂CH₂CH₂ONH₂ | O |
| A-3865 | *c*-Pr | H | Cl | F | CH₂CH₂CH₂CH₂CH₂CH₂ONH₂ | O |
| A-3866 | *c*-Pr | H | Me | F | Me | O |

**[Table 89]**

| Compound Number | R¹ | R² | R³ | R⁴ | R⁵ | A |
|---|---|---|---|---|---|---|
| A-3867 | *c*-Pr | H | Me | F | Et | O |
| A-3868 | *c*-Pr | H | Me | F | *n*-Pr | O |
| A-3869 | *c*-Pr | H | Me | F | *i*-Pr | O |
| A-3870 | *c*-Pr | H | Me | F | *n*-Bu | O |
| A-3871 | *c*-Pr | H | Me | F | *n* -Pen | O |
| A-3872 | *c*-Pr | H | Me | F | *n* -Hex | O |
| A-3873 | *c*-Pr | H | Me | F | *n* -Heptyl | O |
| A-3874 | *c*-Pr | H | Me | F | *n* -Octyl | O |
| A-3875 | *c*-Pr | H | Me | F | *n* -Nonyl | O |
| A-3876 | *c*-Pr | H | Me | F | *n* -Decyl | O |
| A-3877 | *c*-Pr | H | Me | F | *c*-Pr | O |
| A-3878 | *c*-Pr | H | Me | F | *c*-Pen | O |
| A-3879 | *c-*Pr | H | Me | F | *c* -Hex | O |
| A-3880 | *c*-Pr | H | Me | F | CH₂C(Me)=CH₂ | O |
| A-3881 | *c*-Pr | H | Me | F | CH₂CH₂CH=CH₂ | O |
| A-3882 | *c*-Pr | H | Me | F | CH₂CH₂CH⁼C(CH₃)₂ | O |
| A-3883 | *c*-Pr | H | Me | F | CH₂CH₂CH₂CH=CH₂ | O |
| A-3884 | *c*-Pr | H | Me | F | CH₂CH₂CH₂CH=C(CH₃)₂ | O |
| A-3885 | *c*-Pr | H | Me | F | CH₂CH₂CH₂CH₂CH=CH₂ | O |
| A-3886 | *c*-Pr | H | Me | F | CH₂CH₂CH₂CH₂CH=C(CH₃)₂ | O |
| A-3887 | *c*-Pr | H | Me | F | CH₂CH₂CH≡CH | O |
| A-3888 | *c*-Pr | H | Me | F | CH₂CH₂CH₂CH≡CH | O |
| A-3889 | *c*-Pr | H | Me | F | CH₂CH₂CH₂CH₂CH≡CH | O |
| A-3890 | *c-*Pr | H | Me | F | CH₂CH₂CH₂Cl | O |
| A-3891 | *c*-Pr | H | Me | F | CH₂CH₂CH₂Br | O |
| A-3892 | *c*-Pr | H | Me | F | CH₂CH₂CH₂CH₂Cl | O |
| A-3893 | *c*-Pr | H | Me | F | CH₂CH₂CH₂CH₂Br | O |
| A-3894 | *c*-Pr | H | Me | F | CH₂CH₂CH₂CH₂CH₂Cl | O |
| A-3895 | *c*-Pr | H | Me | F | CH₂CH₂CH₂CH₂CH₂Br | O |
| A-3896 | *c*-Pr | H | Me | Cl | CH₂CH₂CH₂CH₂CH₂Br | O |
| A-3897 | *c*-Pr | H | Me | F | CH₂CH₂CH₂CH₂CH₂CH₂Cl | O |
| A-3898 | *c*-Pr | H | Me | F | CH₂CH₂CH₂CH₂CH₂CH₂Br | O |
| A-3899 | *c*-Pr | H | Me | F | CH₂CH₂CH₂CH₂CH₂CH₂CH₂Cl | O |
| A-3900 | *c*-Pr | H | Me | F | CH₂CH₂CH₂CH₂CH₂CH₂CH₂Br | O |
| A-3901 | *c*-Pr | H | Me | F | CH₂CH₂CH₂CH₂CH₂CH₂CH₂CH₂Br | O |
| A-3902 | *c*-Pr | H | Me | F | CH₂CH(Me)CH₂Cl | O |
| A-3903 | *c*-Pr | H | Me | F | CH₂CH(Me)CH₂Br | O |
| A-3904 | *c*-Pr | H | Me | F | CH₂CH₂CH(Me)CH₂CH₂Cl | O |
| A-3905 | *c*-Pr | H | Me | F | CH₂CH₂CH(Me)CH₂CH₂Br | O |
| A-3906 | *c*-Pr | H | Me | F | CH₂CH₂CF=CF₂ | O |
| A-3907 | *c*-Pr | H | Me | F | CH₂CH=C(Cl)CF₃ | O |
| A-3908 | *c*-Pr | H | Me | F | CH₂CH₂CH₂CF=CF₂ | O |
| A-3909 | *c*-Pr | H | Me | F | CH₂CH₂CH₂CH₂CF=CF₂ | O |
| A-3910 | *c*-Pr | H | Me | F | CH₂CH₂CH₂CH₂CH₂CF=CF₂ | O |

**[Table 90]**

| Compound Number | R¹ | R² | R³ | R⁴ | R⁵ | A |
|---|---|---|---|---|---|---|
| A-3911 | *c*-Pr | H | Me | F | CH₂(*t*-Bu) | O |
| A-3912 | *c*-Pr | H | Me | F | CH₂CH₂(*t*-Bu) | O |
| A-3913 | *c*-Pr | H | Me | F | CH₂CH₂CH₂(*t*-Bu) | O |
| A-3914 | *c*-Pr | H | Me | F | CH₂CH₂CH₂CH₂(*t*-Bu) | O |
| A-3915 | *c*-Pr | H | Me | F | CH₂CH₂CH₂CH₂CH₂(*t*-Bu) | O |
| A-3916 | *c*-Pr | H | Me | F | CH₂CH₂CH₂CH₂CH₂CH₂(*t*-Bu) | O |
| A-3917 | *c*-Pr | H | Me | F | CH₂CF₃ | O |
| A-3918 | *c*-Pr | H | Me | F | CH₂CH₂CF₃ | O |
| A-3919 | *c*-Pr | H | Me | F | CH₂CH₂CH₂CF₃ | O |
| A-3920 | *c*-Pr | H | Me | F | CH₂CH₂CH₂CH₂CF₃ | O |
| A-3921 | *c*-Pr | H | Me | F | CH₂CH₂CH₂CH₂CH₂CF₃ | O |
| A-3922 | *c*-Pr | H | Me | F | CH₂CH₂CH₂CH₂CH₂CH₂CF₃ | O |
| A-3923 | *c*-Pr | H | Me | F | CH₂CH₂CH₂CH₂CH₂CH₂CH₂CF₃ | O |
| A-3924 | *c*-Pr | H | Me | F | CH₂CF₂CF₃ | O |
| A-3925 | *c*-Pr | H | Me | F | CF₂CHFCF₃ | O |
| A-3926 | *c*-Pr | H | Me | F | CF₂CF₂CF₃ | O |
| A-3927 | *c*-Pr | H | Me | F | CH₂CF₂CF₂CF₃ | O |
| A-3928 | *c*-Pr | H | Me | F | CH₂CF₂CF₂CF₂CF₃ | O |
| A-3929 | *c*-Pr | H | Me | F | CH₂CF₂CF₂CF₂CHF₂ | O |
| A-3930 | *c*-Pr | H | Me | F | CH(CF₃)CF₃ | O |
| A-3931 | *c*-Pr | H | Me | F | CH₂CF(CF₃)CF₃ | O |
| A-3932 | *c*-Pr | H | Me | F | CH₂CF₂CF(CF₃)CF₃ | O |
| A-3933 | *c*-Pr | H | Me | F | CH₂CF(CF₃)CF₂CF₃ | O |
| A-3934 | *c*-Pr | H | Me | F | CH₂CF₂CF₂CF(CF₃)CF₃ | O |
| A-3935 | *c*-Pr | H | Me | F | CH₂CF₂CF(CF₃)CF₂CF₃ | O |
| A-3936 | *c*-Pr | H | Me | F | CH₂CF(CF₃)CF₂CF₂CF₃ | O |
| A-3937 | *c*-Pr | H | Me | F | CH₂(*c*-Pr) | O |
| A-3938 | *c*-Pr | H | Me | F | CH₂[*c*-Pr(1-Me)] | O |
| A-3939 | *c-*Pr | H | Me | F | CH₂[*c*-Pr(1-Ph)] | O |
| A-3940 | *c*-Pr | H | Me | F | CH₂[*c*-Pr(1-NH₂)] | O |
| A-3941 | *c*-Pr | H | Me | F | CH₂[*c*-Pr(1 -NHC(=O)O(t-Bu)] | O |
| A-3942 | *c*-Pr | H | Me | F | CH₂{*c*-Pr[1-NHS(=O)₂CF₃]} | O |
| A-3943 | *c*-Pr | H | Me | F | CH₂{*c*-Pr[1-Ph(4-CF₃)]} | O |
| A-3944 | *c*-Pr | H | Me | F | CH₂{*c*-Pr[1-Ph(3,4,5-F₃)]} | O |
| A-3945 | *c*-Pr | H | Me | F | CH₂{*c*-Hex[4-(t-Bu)]} | O |
| A-3946 | *c*-Pr | H | Me | F | CH₂[*c*-Hex(4-CF₃)] | O |
| A-3947 | *c*-Pr | H | Me | F | CH₂CH₂(*c*-Pr) | O |
| A-3948 | *c*-Pr | H | Me | F | CH₂CH₂(*c*-Hex) | O |
| A-3949 | *c*-Pr | H | Me | F | CH₂CH₂[*c*-Hex(4-CF₃)] | O |
| A-3950 | *c*-Pr | H | Me | F | CH₂CH₂[*c*-Hex(4-SCF₃)] | O |
| A-3951 | *c*-Pr | H | Me | F | CH₂CH₂CH₂(*c*-Pr) | O |
| A-3952 | *c*-Pr | H | Me | F | CH₂CH₂CH₂(*c*-Hex) | O |
| A-3953 | *c*-Pr | H | Me | F | CH₂CH₂CH₂{*c*-Hex[4-(t-Bu)]) | O |
| A-3954 | *c*-Pr | H | Me | F | CH₂CH₂CH₂[*c*-Hex(4-CF₃)] | O |

**[Table 91]**

| Compound Number | R¹ | R² | R³ | R⁴ | R⁵ | A |
|---|---|---|---|---|---|---|
| A-3955 | *c*-Pr | H | Me | F | CH₂CH₂CH₂[*c*-Hex(4-SCF₃)] | O |
| A-3956 | *c*-Pr | H | Me | F | CH₂CH₂CH₂CH₂(*c*-Pr) | O |
| A-3957 | *c*-Pr | H | Me | F | CH₂CH₂CH₂CH₂(*c*-Hex) | O |
| A-3958 | *c*-Pr | H | Me | F | CH₂CH₂CH₂CH₂[*c*-Hex(4-CF₃)] | O |
| A-3959 | *c*-Pr | H | Me | F | CH₂[*c*-Pr(2,2-F₂)] | O |
| A-3960 | *c*-Pr | H | Me | F | CH₂[*c*-Hex(4,4-F₂)] | O |
| A-3961 | *c*-Pr | H | Me | F | CH₂CH₂[*c*-Pr(2,2-F₂)] | O |
| A-3962 | *c*-Pr | H | Me | F | CH₂CH₂[*c*-Hex(4,4-F₂)] | O |
| A-3963 | *c*-Pr | H | Me | F | CH₂CH₂CH₂[*c*-Pr(2,2-F₂) | O |
| A-3964 | *c*-Pr | H | Me | F | CH₂CH₂CH₂CH₂[*c*-Pr(2,2-F₂)] | O |
| A-3965 | *c*-Pr | H | Me | F | CH₂CH₂OH | O |
| A-3966 | *c*-Pr | H | Me | F | CH₂CH₂CH₂OH | O |
| A-3967 | *c*-Pr | H | Me | F | CH₂CH₂CH₂CH₂OH | O |
| A-3968 | *c*-Pr | H | Me | F | CH₂CH₂CH₂CH₂CH₂OH | O |
| A-3969 | *c*-Pr | H | Me | F | CH₂CH₂CH₂CH₂CH₂CH₂OH | O |
| A-3970 | *c*-Pr | H | Me | F | CH₂C(=O)Me | O |
| A-3971 | *c*-Pr | H | Me | F | CH₂CH(OH)Me | O |
| A-3972 | *c*-Pr | H | Me | F | CH₂CH(OH)CF₃ | O |
| A-3973 | *c*-Pr | H | Me | F | CH₂C(OH)₂CF₃ | O |
| A-3974 | *c*-Pr | H | Me | F | CH₂C(CF₃)=NOH | O |
| A-3975 | *c*-Pr | H | Me | F | CH₂C(CF₃)=NOMe | O |
| A-3976 | *c*-Pr | H | Me | F | CH₂CH(CF₃)NH₂ | O |
| A-3977 | *c*-Pr | H | Me | F | CH₂CH₂OMe | O |
| A-3978 | *c*-Pr | H | Me | F | CH₂CH₂CH₂OMe | O |
| A-3979 | *c*-Pr | H | Me | F | CH₂CH₂CH₂CH₂OMe | O |
| A-3980 | *c*-Pr | H | Me | F | CH₂CH₂CH₂CH₂CH₂OMe | O |
| A-3981 | *c*-Pr | H | Me | F | CH₂CH₂CH₂CH₂CH₂CH₂OMe | O |
| A-3982 | *c*-Pr | H | Me | F | CH₂CH₂OCHF₂ | O |
| A-3983 | *c*-Pr | H | Me | F | CH₂CH₂CH₂OCHF₂ | O |
| A-3984 | *c*-Pr | H | Me | F | CH₂CH₂CH₂CH₂OCHF₂ | O |
| A-3985 | *c*-Pr | H | Me | F | CH₂CH₂CH₂CH₂CH₂OCHF₂ | O |
| A-3986 | *c*-Pr | H | Me | F | CH₂CH₂CH₂CH₂CH₂CH₂OCHF₂ | O |
| A-3987 | *c*-Pr | H | Me | F | CH₂CH₂OCH₂CF₃ | O |
| A-3988 | *c*-Pr | H | Me | F | CH₂CH₂CH₂OCH₂CF₃ | O |
| A-3989 | *c*-Pr | H | Me | F | CH₂CH₂CH₂CH₂OCH₂CF₃ | O |
| A-3990 | *c*-Pr | H | Me | F | CH₂CH₂CH₂CH₂CH₂OCH₂CF₃ | O |
| A-3991 | *c*-Pr | H | Me | F | CH₂CH₂CH₂CH₂CH₂CH₂OCH₂CF₃ | O |
| A-3992 | *c*-Pr | H | Me | F | CH₂CH₂CH₂CH₂CH₂CH₂CH₂OCH₂CF₃ | O |
| A-3993 | *c*-Pr | H | Me | F | CH₂CH₂OC(CF₃)₃ | O |
| A-3994 | *c*-Pr | H | Me | F | CH₂CH₂CH₂OC(CF₃)₃ | O |
| A-3995 | *c*-Pr | H | Me | F | CH₂CH₂CH₂CH₂OC(CF₃)₃ | O |
| A-3996 | *c*-Pr | H | Me | F | CH₂CH₂CH₂CH₂CH₂OC(CF₃)₃ | O |
| A-3997 | *c*-Pr | H | Me | F | CF₂CHFOCF₂CF₂CF₃ | O |
| A-3998 | *c*-Pr | H | Me | F | CH₂CH₂O(*c*-Pr) | O |

**[Table 92]**

| Compound Number | R¹ | R² | R³ | R⁴ | R⁵ | A |
|---|---|---|---|---|---|---|
| A-3999 | *c*-Pr | H | Me | F | CH₂CH₂O(*c*-Pen) | O |
| A-4000 | *c*-Pr | H | Me | F | CH₂CH₂O(*c*-Hex) | O |
| A-4001 | *c*-Pr | H | Me | F | CH₂CH₂CH₂O(*c*-Pr) | O |
| A-4002 | *c*-Pr | H | Me | F | CH₂CH₂CH₂O(*c*-Pen) | O |
| A-4003 | *c*-Pr | H | Me | F | CH₂CH₂CH₂O(*c*-Hex) | O |
| A-4004 | *c*-Pr | H | Me | F | CH₂CH₂CH₂CH₂O(*c*-Pr) | O |
| A-4005 | *c*-Pr | H | Me | F | CH₂CH₂CH₂CH₂O(*c*-Pen) | O |
| A-4006 | *c*-Pr | H | Me | F | CH₂CH₂CH₂CH₂O(*c*-Hex) | O |
| A-4007 | *c*-Pr | H | Me | F | CH₂CH₂O[*c*-Pr(2,2-F₂)] | O |
| A-4008 | *c*-Pr | H | Me | F | CH₂CH₂CH₂O[*c*-Pr(2,2-F₂)] | O |
| A-4009 | *c*-Pr | H | Me | F | CH₂CH₂CH₂CH₂O[*c*-Pr(2,2-F₂)] | O |
| A-4010 | *c*-Pr | H | Me | F | CH₂CH₂OCH₂CH₂OCH₃ | O |
| A-4011 | *c*-Pr | H | Me | F | CH₂CH₂OCH₂CH₂OCH₂CF₃ | O |
| A-4012 | *c*-Pr | H | Me | F | CH₂CF₂OCF₂CF₂OCF₃ | O |
| A-4013 | *c*-Pr | H | Me | F | CF₂CHFOCF₂CF₂OCF₃ | O |
| A-4014 | *c*-Pr | H | Me | F | CF₂CHFOCF₂CF(CF₃)OCF₂CF₂CF₃ | O |
| A-4015 | *c*-Pr | H | Me | F | CH₂CH₂SH | O |
| A-4016 | *c*-Pr | H | Me | F | CH₂CH₂CH₂SH | O |
| A-4017 | *c*-Pr | H | Me | F | CH₂CH₂CH₂CH₂SH | O |
| A-4018 | *c*-Pr | H | Me | F | CH₂CH₂CH₂CH₂CH₂SH | O |
| A-4019 | *c*-Pr | H | Me | F | CH₂CH₂CH₂CH₂CH₂CH₂SH | O |
| A-4020 | *c*-Pr | H | Me | F | CH₂CH(SH)Me | O |
| A-4021 | *c*-Pr | H | Me | F | CH₂CH[SC(=O)Me]Me | O |
| A-4022 | *c*-Pr | H | Me | F | CH₂CH₂S(t-Bu) | O |
| A-4023 | *c*-Pr | H | Me | F | CH₂CH₂CH₂S(*t*-Bu) | O |
| A-4024 | *c*-Pr | H | Me | F | CH₂CH₂CH₂CH₂S(*t*-Bu) | O |
| A-4025 | *c*-Pr | H | Me | F | CH₂CH₂CH₂CH₂CH₂SCH₃ | O |
| A-4026 | *c*-Pr | H | Me | F | CH₂CH₂CH₂CH₂CH₂SCH(CH₃)₂ | O |
| A-4027 | *c*-Pr | H | Me | F | CH₂CH₂CH₂CH₂CH₂S(*t*-Bu) | O |
| A-4028 | *c*-Pr | H | Me | F | CH₂CH₂CH₂CH₂CH₂CH₂SCH(CH₃)₂ | O |
| A-4029 | *c*-Pr | H | Me | F | CH₂CH₂CH₂CH₂CH₂CH₂S(*t*-Bu) | O |
| A-4030 | *c*-Pr | H | Me | F | CH₂CH₂CH₂CH₂CH₂CH₂CH₂S(*t*-Bu) | O |
| A-4031 | *c*-Pr | H | Me | F | CH₂CH₂SCHF₂ | O |
| A-4032 | *c*-Pr | H | Me | F | CH₂CH₂CH₂SCHF₂ | O |
| A-4033 | *c*-Pr | H | Me | F | CH₂CH₂CH₂CH₂SCHF₂ | O |
| A-4034 | *c*-Pr | H | Me | H | CH₂CH₂CH₂CH₂CH₂SCHF₂ | O |
| A-4035 | *c*-Pr | H | Me | F | CH₂CH₂CH₂CH₂CH₂SCHF₂ | O |
| A-4036 | *c*-Pr | H | Me | F | CH₂CH₂CH₂CH₂CH₂CH₂SCHF₂ | O |
| A-4037 | *c*-Pr | H | Me | F | CH₂CH₂CH₂CH₂CH₂CH₂CH₂SCHF₂ | O |
| A-4038 | *c*-Pr | H | Me | F | CH₂CH₂SCH₂CF₃ | O |
| A-4039 | *c*-Pr | H | Me | F | CH₂CH₂CH₂SCH₂CF₃ | O |
| A-4040 | *c*-Pr | H | Me | F | CH₂CH₂CH₂CH₂SCH₂CF₃ | O |
| A-4041 | *c*-Pr | H | Me | F | CH₂CH₂CH₂CH₂CH₂SCH₂CF₃ | O |
| A-4042 | *c*-Pr | H | Me | F | CH₂CH₂CH₂CH₂CH₂CH₂SCH₂CF₃ | O |

**[Table 93]**

| Compound Number | R¹ | R² | R³ | R⁴ | R⁵ | A |
|---|---|---|---|---|---|---|
| A-4043 | *c*-Pr | H | Me | F | CH₂CH₂CH₂CH₂CH₂CH₂CH₂SCH₂CF₃ | O |
| A-4044 | *c*-Pr | H | Me | F | CH₂CH(Me)SCH₂CF₃ | O |
| A-4045 | *c*-Pr | H | Me | F | CH₂CH₂SCH(CF₃)₂ | O |
| A-4046 | *c*-Pr | H | Me | F | CH₂CH₂CH₂SCH(CF₃)₂ | O |
| A-4047 | *c*-Pr | H | Me | F | CH₂CH₂CH₂CH₂SCH(CF₃)₂ | O |
| A-4048 | *c*-Pr | H | Me | F | CH₂CH₂CH₂CH₂CH₂SCH(CF₃)₂ | O |
| A-4049 | *c*-Pr | H | Me | F | CH₂CH₂CH₂CH₂CH₂CH₂SCH(CF₃)₂ | O |
| A-4050 | *c*-Pr | H | Me | F | CH₂CH₂CH₂CH₂CH₂SCF₂CF₃ | O |
| A-4051 | *c*-Pr | H | Me | F | CH₂CH₂CH₂CH₂CH₂CH₂SCF₂CF₃ | O |
| A-4052 | *c*-Pr | H | Me | F | CH₂CH₂CH₂CH₂CH₂CH₂SCF(CF₃)₂ | O |
| A-4053 | *c*-Pr | H | Me | F | CH₂CH₂CH₂CH₂CH₂CH₂CH₂SCF(CF₃)₂ | O |
| A-4054 | *c*-Pr | H | Me | F | CH₂CH₂CH₂CH₂CH₂SCCl₃ | O |
| A-4055 | *c*-Pr | H | Me | F | CH₂CH₂CH₂CH₂CH₂CH₂SCCl₃ | O |
| A-4056 | *c*-Pr | H | Me | F | CH₂CH₂SCH₂CH=CH₂ | O |
| A-4057 | *c*-Pr | H | Me | F | CH₂CH₂CH₂SCH₂CH=CH₂ | O |
| A-4058 | *c*-Pr | H | Me | F | CH₂CH₂CH₂CH₂SCH₂CH=CH₂ | O |
| A-4059 | *c*-Pr | H | Me | F | CH₂CH₂SCF=CFCF₃ | O |
| A-4060 | *c*-Pr | H | Me | F | CH₂CH₂CH₂SCF=CFCF₃ | O |
| A-4061 | *c*-Pr | H | Me | F | CH₂CH₂CH₂CH₂SCF=CFCF₃ | O |
| A-4062 | *c*-Pr | H | Me | F | CH₂CH₂CH₂S(*c*-Pr) | O |
| A-4063 | *c*-Pr | H | Me | F | CH₂CH₂CH₂S(*c*-Hex) | O |
| A-4064 | *c*-Pr | H | Me | F | CH₂CH₂CH₂CH₂S(*c*-Pr) | O |
| A-4065 | *c*-Pr | H | Me | F | CH₂CH₂CH₂CH₂S(*c*-Hex) | O |
| A-4066 | *c*-Pr | H | Me | F | CH₂CH₂CH₂CH₂CH₂S(*c*-Pr) | O |
| A-4067 | *c*-Pr | H | Me | F | CH₂CH₂CH₂CH₂CH₂S(*c*-Hex) | O |
| A-4068 | *c*-Pr | H | Me | F | CH₂CH₂CH₂S[*c*-Hex(4,4-F₂) | O |
| A-4069 | *c*-Pr | H | Me | F | CH₂CH₂CH₂CH₂S[*c*-Hex(4,4-F₂)] | O |
| A-4070 | *c*-Pr | H | Me | F | CH₂CH₂CH₂CH₂CH₂S[*c*-Hex(4,4-F₂)] | O |
| A-4071 | *c*-Pr | H | Me | F | CH₂CH₂CH₂SCH₂(*c*-Pr) | O |
| A-4072 | *c*-Pr | H | Me | F | CH₂CH₂CH₂SCH₂(*c*-Hex) | O |
| A-4073 | *c*-Pr | H | Me | F | CH₂CH₂CH₂CH₂SCH₂(*c*-Pr) | O |
| A-4074 | *c*-Pr | H | Me | F | CH₂CH₂CH₂CH₂SCH₂(*c*-Hex) | O |
| A-4075 | *c*-Pr | H | Me | F | CH₂CH₂CH₂CH₂CH₂SCH₂(*c*-Pr) | O |
| A-4076 | *c*-Pr | H | Me | F | CH₂CH₂CH₂CH₂CH₂SCH₂(*c*-Hex) | O |
| A-4077 | *c*-Pr | H | Me | F | CH₂CH₂CH₂SCH₂[*c*-Hex(4,4-F₂)] | O |
| A-4078 | *c*-Pr | H | Me | F | CH₂CH₂CH₂CH₂SCH₂[*c*-Hex(4,4-F₂)] | O |
| A-4079 | *c*-Pr | H | Me | F | CH₂CH₂CH₂CH₂CH₂SCH₂[*c*-Hex(4,4-F₂)] | O |
| A-4080 | *c*-Pr | H | Me | F | CH₂CH₂CH₂SCH₂SiMe₃ | O |
| A-4081 | *c*-Pr | H | Me | F | CH₂CH₂CH₂CH₂SCH₂SiMe₃ | O |
| A-4082 | *c*-Pr | H | Me | F | CH₂CH₂CH₂CH₂CH₂SCH₂SiMe₃ | O |
| A-4083 | *c*-Pr | H | Me | F | CH₂CH₂OCH₂CH₂SCH₃ | O |
| A-4084 | *c*-Pr | H | Me | F | CH₂CH₂OCH₂CH₂SCHF₂ | O |
| A-4085 | *c*-Pr | H | Me | F | CH₂CH₂OCH₂CH₂SCF₃ | O |
| A-4086 | *c*-Pr | H | Me | F | CH₂CH₂OCH₂CH₂SCH₂CF₃ | O |

**[Table 94]**

| Compound Number | R¹ | R² | R³ | R⁴ | R⁵ | A |
|---|---|---|---|---|---|---|
| A-4087 | *c*-Pr | H | Me | F | CH₂CH₂CH₂S(=O)(*t*-Bu) | O |
| A-4088 | *c*-Pr | H | Me | F | CH₂CH₂CH₂CH₂S(=O)(*t*-Bu) | O |
| A-4089 | *c*-Pr | H | Me | F | CH₂CH₂CH₂CH₂CH₂CH₂S(=O)(*t*-Bu) | O |
| A-4090 | *c*-Pr | H | Me | F | CH₂CH₂S(=O)CHF₂ | O |
| A-4091 | *c*-Pr | H | Me | F | CH₂CH₂CH₂S(=O)CHF₂ | O |
| A-4092 | *c*-Pr | H | Me | F | CH₂CH₂CH₂CH₂S(=O)CHF₂ | O |
| A-4093 | *c*-Pr | H | Me | F | CH₂CH₂CH₂CH₂CH₂S(=O)CHF₂ | O |
| A-4094 | *c*-Pr | H | Me | F | CH₂CH₂CH₂CH₂CH₂CH₂S(=O)CHF₂ | O |
| A-4095 | *c*-Pr | H | Me | F | CH₂CH₂CH₂CH₂CH₂CH₂CH₂S(=O)CHF₂ | O |
| A-4096 | *c*-Pr | H | Me | F | CH₂CH₂S(=O)CF₃ | O |
| A-4097 | *c*-Pr | H | Me | F | CH₂CH₂CH₂S(=O)CF₃ | O |
| A-4098 | *c*-Pr | H | Me | F | CH₂CH₂CH₂CH₂S(=O)CF₃ | O |
| A-4099 | *c*-Pr | H | Me | F | CH₂CH₂CH₂CH₂CH₂S(=O)CF₃ | O |
| A-4100 | *c*-Pr | H | Me | F | CH₂CH₂CH₂CH₂CH₂CH₂S(=O)CF₃ | O |
| A-4101 | *c*-Pr | H | Me | F | CH₂CH₂CH₂CH₂CH₂CH₂CH₂S(=O)CF₃ | O |
| A-4102 | *c*-Pr | H | Me | F | CH₂CH₂S(=O)CH₂CF₃ | O |
| A-4103 | *c*-Pr | H | Me | F | CH₂CH₂CH₂S(=O)CH₂CF₃ | O |
| A-4104 | *c*-Pr | H | Me | F | CH₂CH₂CH₂CH₂S(=O)CH₂CF₃ | O |
| A-4105 | *c*-Pr | H | Me | F | CH₂CH₂CH₂CH₂CH₂S(=O)CH₂CF₃ | O |
| A-4106 | *c*-Pr | H | Me | F | CH₂CH₂CH₂CH₂CH₂CH₂S(=O)CH₂CF₃ | O |
| A-4107 | *c*-Pr | H | Me | F | CH₂CH₂CH₂CH₂CH₂CH₂CH₂S(=O)CH₂CF₃ | O |
| A-4108 | *c*-Pr | H | Me | F | CH₂CH(Me)S(=O)CH₂CF₃ | O |
| A-4109 | *c*-Pr | H | Me | F | CH₂CH₂CH₂S(=O)(*c*-Pr) | O |
| A-4110 | *c*-Pr | H | Me | F | CH₂CH₂CH₂CH₂S(=O)(*c*-Pr) | O |
| A-4111 | *c*-Pr | H | Me | F | CH₂CH₂CH₂CH₂S(=O)(*c*-Pen) | O |
| A-4112 | *c*-Pr | H | Me | F | CH₂CH₂CH₂CH₂S(=O)(*c*-Hex) | O |
| A-4113 | *c*-Pr | H | Me | F | CH₂CH₂CH₂CH₂CH₂S(=O)(*c*-Pr) | O |
| A-4114 | *c*-Pr | H | Me | F | CH₂CH₂CH₂CH₂CH₂S(=O)(*c*-Hex) | O |
| A-4115 | *c*-Pr | H | Me | F | CH₂CH₂CH₂S(=O)[*c*-Hex(4,4-F₂)] | O |
| A-4116 | *c*-Pr | H | Me | F | CH₂CH₂CH₂CH₂S(=O)[*c*-Hex(4,4-F₂)] | O |
| A-4117 | *c*-Pr | H | Me | F | CH₂CH₂CH₂CH₂CH₂S(=O)[*c*-Hex(4,4-F₂)] | O |
| A-4118 | *c*-Pr | H | Me | F | CH₂CH₂CH₂CH₂S(=O)₂Me | O |
| A-4119 | *c*-Pr | H | Me | F | CH₂CH₂CH₂CH₂CH₂S(=O)₂Me | O |
| A-4120 | *c*-Pr | H | Me | F | CH₂CH₂CH₂CH₂CH₂S(=O)₂(*t*-Bu) | O |
| A-4121 | *c*-Pr | H | Me | F | CH₂CH₂S(=O)₂CHF₂ | O |
| A-4122 | *c*-Pr | H | Me | F | CH₂CH₂CH₂S(=O)₂CHF₂ | O |
| A-4123 | *c*-Pr | H | Me | F | CH₂CH₂CH₂CH₂S(=O)₂CHF₂ | O |
| A-4124 | *c*-Pr | H | Me | F | CH₂CH₂CH₂CH₂CH₂S(=O)₂CHF₂ | O |
| A-4125 | *c*-Pr | H | Me | F | CH₂CH₂CH₂CH₂CH₂CH₂S(=O)₂CHF₂ | O |
| A-4126 | *c*-Pr | H | Me | F | CH₂CH₂S(=O)₂CF₃ | O |
| A-4127 | *c*-Pr | H | Me | F | CH₂CH₂CH₂S(=O)₂CF₃ | O |
| A-4128 | *c*-Pr | H | Me | F | CH₂CH₂CH₂CH₂S(=O)₂CF₃ | O |
| A-4129 | *c*-Pr | H | Me | F | CH₂CH₂CH₂CH₂CH₂S(=O)₂CF₃ | O |
| A-4130 | *c*-Pr | H | Me | F | CH₂CH₂CH₂CH₂CH₂CH₂S(=O)₂CF₃ | O |

**[Table 95]**

| Compound Number | R¹ | R² | R³ | R⁴ | R⁵ | A |
|---|---|---|---|---|---|---|
| A-4131 | *c*-Pr | H | Me | F | CH₂CH₂CH₂CH₂CH₂CH₂CH₂S(=O)₂CF₃ | O |
| A-4132 | *c*-Pr | H | Me | F | CH₂CH₂CH₂CH₂CH₂CH₂CH₂CH₂S(=O)₂CF₃ | O |
| A-4133 | *c*-Pr | H | Me | F | CH₂CH₂S(=O)₂CH₂CF₃ | O |
| A-4134 | *c*-Pr | H | Me | F | CH₂CH₂CH₂S(=O)₂CH₂CF₃ | O |
| A-4135 | *c*-Pr | H | Me | F | CH₂CH₂CH₂CH₂S(=O)₂CH₂CF₃ | O |
| A-4136 | *c*-Pr | H | Me | F | CH₂CH₂CH₂CH₂CH₂S(=O)₂CH₂CF₃ | O |
| A-4137 | *c*-Pr | H | Me | F | CH₂CH₂CH₂CH₂CH₂CH₂S(=O)₂CH₂CF₃ | O |
| A-4138 | *c*-Pr | H | Me | F | CH₂CH(Me)S(=O)₂CH₂CF₃ | O |
| A-4139 | *c*-Pr | H | Me | F | CH₂CH₂CH₂S(=O)₂(*c*-Pr) | O |
| A-4140 | *c*-Pr | H | Me | F | CH₂CH₂CH₂CH₂S(=O)₂(*c*-Pr) | O |
| A-4141 | *c*-Pr | H | Me | F | CH₂CH₂CH₂CH₂CH₂S(=O)₂(*c*-Pr) | O |
| A-4142 | *c*-Pr | H | Me | F | CH₂CH₂CH₂S(=O)₂[*c*-Hex(4,4-F₂)] | O |
| A-4143 | *c*-Pr | H | Me | F | CH₂CH₂CH₂CH₂S(=O)₂[*c*-Hex(4,4-F₂)] | O |
| A-4144 | *c*-Pr | H | Me | F | CH₂CH₂CH₂CH₂CH₂S(=O)₂[*c*-Hex(4,4-F₂)] | O |
| A-4145 | *c-*Pr | H | Me | F | CH₂CH₂CH₂C(=O)H | O |
| A-4146 | *c*-Pr | H | Me | F | CH₂CH₂CH₂CH₂C(=O)H | O |
| A-4147 | *c*-Pr | H | Me | F | CH₂CH₂CH₂CH₂CH₂C(=O)H | O |
| A-4148 | *c*-Pr | H | Me | F | CH₂CH₂CH₂C(=O)(*t*-Bu) | O |
| A-4149 | *c*-Pr | H | Me | F | CH₂CH₂CH₂CH₂C(=O)(*t*-Bu) | O |
| A-4150 | *c*-Pr | H | Me | F | CH₂CH₂CH₂CH₂CH₂C(=O)(*t*-Bu) | O |
| A-4151 | *c*-Pr | H | Me | F | CH₂CH₂CH₂C(=O)CF₃ | O |
| A-4152 | *c*-Pr | H | Me | F | CH₂CH₂CH₂CH₂C(=O)CF₃ | O |
| A-4153 | *c*-Pr | H | Me | F | CH₂CH₂CH₂CH₂CH₂C(=O)CF₃ | O |
| A-4154 | *c*-Pr | H | Me | F | CH₂C(=O)Ph | O |
| A-4155 | *c*-Pr | H | Me | F | CH₂C(=O)Ph(4-Cl) | O |
| A-4156 | *c*-Pr | H | Me | F | CH₂C(=O)Ph(4-CF₃) | O |
| A-4157 | *c*-Pr | H | Me | F | CH₂CH₂C(=O)Ph(4-CF₃) | O |
| A-4158 | *c*-Pr | H | Me | F | CH₂CH₂CH₂C(=O)Ph(4-CF₃) | O |
| A-4159 | *c*-Pr | H | Me | F | CH₂CH₂CH₂CH₂C(=O)Ph(4-CF₃) | O |
| A-4160 | *c*-Pr | H | Me | F | CH₂CH₂CH₂CH₂CH₂C(=O)Ph(4-CF₃) | O |
| A-4161 | *c*-Pr | H | Me | F | CH₂C(=O)OEt | O |
| A-4162 | *c*-Pr | H | Me | F | CH₂CH₂C(=O)O(*t*-Bu) | O |
| A-4163 | *c*-Pr | H | Me | F | CH₂CH₂CH₂C(=O)OEt | O |
| A-4164 | *c*-Pr | H | Me | F | CH₂CH₂CH₂C(=O)O(*t*-Bu) | O |
| A-4165 | *c*-Pr | H | Me | F | CH₂CH₂CH₂CH₂C(=O)O(*t*-Bu) | O |
| A-4166 | *c*-Pr | H | Me | F | CH₂CH₂CH₂CH₂CH₂C(=O)O(*t*-Bu) | O |
| A-4167 | *c*-Pr | H | Me | F | CH₂CH₂CH₂CH₂CH₂CH₂C(=O)O(*t*-Bu) | O |
| A-4168 | *c*-Pr | H | Me | F | CH₂CH₂CH₂CH₂CH₂CH₂CH₂C(=O)OEt | O |
| A-4169 | *c*-Pr | H | Me | F | CH₂CH₂C(=O)OCH₂CF₃ | O |
| A-4170 | *c*-Pr | H | Me | F | CH₂CH₂CH₂C(=O)OCH₂CF₃ | O |
| A-4171 | *c*-Pr | H | Me | F | CH₂CH₂CH₂CH₂C(=O)OCH₂CF₃ | O |
| A-4172 | *c*-Pr | H | Me | F | CH₂CH₂CH₂CH₂CH₂C(=O)OCH₂CF₃ | O |
| A-4173 | *c*-Pr | H | Me | F | CH₂CH₂C(=O)NH(*t*-Bu) | O |
| A-4174 | *c*-Pr | H | Me | F | CH₂CH₂CH₂C(=O)NH(*t*-Bu) | O |

**[Table 96]**

| Compound Number | R¹ | R² | R³ | R⁴ | R⁵ | A |
|---|---|---|---|---|---|---|
| A-4175 | *.c*-Pr | H | Me | F | CH₂CH₂CH₂C(=O)NH(*t*-Pen) | O |
| A-4176 | *c*-Pr | H | Me | F | CH₂CH₂CH₂CH₂C(=O)NH(*t*-Bu) | O |
| A-4177 | *c*-Pr | H | Me | F | CH₂CH₂C(=O)NHCH₂CF₃ | O |
| A-4178 | *c*-Pr | H | Me | F | CH₂CH₂CH₂C(=O)NHCH₂CF₃ | O |
| A-4179 | *c*-Pr | H | Me | F | CH₂CH₂CH₂CH₂C(=O)NHCH₂CF₃ | O |
| A-4180 | *c*-Pr | H | Me | F | CH₂CH₂SC(=O)N(Me)₂ | O |
| A-4181 | *c*-Pr | H | Me | F | CH₂CH₂CH₂SC(=O)N(Me)₂ | O |
| A-4182 | *c*-Pr | H | Me | F | CH₂CH₂CH₂SC(=O)NH(t-Bu) | O |
| A-4183 | *c*-Pr | H | Me | F | CH₂CH₂CH₂CH₂CH₂SC(=O)NH(*t*-Bu) | O |
| A-4184 | *c*-Pr | H | Me | F | CH₂CH₂SC(=O)NHCH₂CF₃ | O |
| A-4185 | *c*-Pr | H | Me | F | CH₂CH₂CH₂SC(=O)NHCH₂CF₃ | O |
| A-4186 | *c*-Pr | H | Me | F | CH₂CH₂CH₂CH₂SC(=O)NHCH₂CF₃ | O |
| A-4187 | *c*-Pr | H | Me | F | CH₂CH₂CH₂OC(=O)H | O |
| A-4188 | *c*-Pr | H | Me | F | CH₂CH₂CH₂CH₂OC(=O)H | O |
| A-4189 | *c*-Pr | H | Me | F | CH₂CH₂CH₂CH₂CH₂OC(=O)H | O |
| A-4190 | *c*-Pr | H | Me | F | CH₂CH₂CH₂OC(=O)Me | O |
| A-4191 | *c*-Pr | H | Me | F | CH₂CH₂CH₂CH₂OC(=O)Me | O |
| A-4192 | *c*-Pr | H | Me | F | CH₂CH₂CH₂CH₂CH₂OC(=O)Me | O |
| A-4193 | *c*-Pr | H | Me | F | CH₂CH₂OC(=O)CF₃ | O |
| A-4194 | *c*-Pr | H | Me | F | CH₂CH₂CH₂OC(=O)CF₃ | O |
| A-4195 | *c*-Pr | H | Me | F | CH₂CH₂CH₂CH₂OC(=O)CF₃ | O |
| A-4196 | *c*-Pr | H | Me | F | CH₂CH₂CH₂CH₂CH₂OC(=O)Ph(4-CF₃) | O |
| A-4197 | *c-*Pr | H | Me | F | CH₂CH₂OC(=O)Ph | O |
| A-4198 | *c*-Pr | H | Me | F | CH₂CH₂OC(=O)Ph(4-CF₃) | O |
| A-4199 | *c*-Pr | H | Me | F | CH₂CH₂CH₂OC(=O)Ph(4-CF₃) | O |
| A-4200 | *c*-Pr | H | Me | F | CH₂CH₂CH₂CH₂OC(=O)Ph(4-CF₃) | O |
| A-4201 | *c*-Pr | H | Me | F | CH₂CH₂CH₂CH₂CH₂OC(=O)Ph(4-CF₃) | O |
| A-4202 | *c*-Pr | H | Me | F | CH₂CH₂OS(=O)Me | O |
| A-4203 | *c*-Pr | H | Me | F | CH₂CH₂CH₂OS(=O)Me | O |
| A-4204 | *c*-Pr | H | Me | F | CH₂CH₂CH₂CH₂OS(=O)Me | O |
| A-4205 | *c*-Pr | H | Me | F | CH₂CH₂CH₂CH₂CH₂OS(=O)Me | O |
| A-4206 | *c*-Pr | H | Me | F | CH₂CH₂CH₂CH₂CH₂CH₂OS(=O)Me | O |
| A-4207 | *c*-Pr | H | Me | F | CH₂CH₂OS(=O)CF₃ | O |
| A-4208 | *c*-Pr | H | Me | F | CH₂CH₂CH₂OS(=O)CF₃ | O |
| A-4209 | *c*-Pr | H | Me | F | CH₂CH₂CH₂CH₂OS(=O)CF₃ | O |
| A-4210 | *c*-Pr | H | Me | F | CH₂CH₂CH₂CH₂CH₂OS(=O)CF₃ | O |
| A-4211 | *c*-Pr | H | Me | F | CH₂CH₂CH₂CH₂CH₂CH₂OS(=O)CF₃ | O |
| A-4212 | *c*-Pr | H | Me | F | CH₂CH₂OS(=O)Ph | O |
| A-4213 | *c*-Pr | H | Me | F | CH₂CH₂OS(=O)Ph(4-Me) | O |
| A-4214 | *c*-Pr | H | Me | F | CH₂CH₂CH₂OS(=O)Ph | O |
| A-4215 | *c*-Pr | H | Me | F | CH₂CH₂CH₂OS(=O)Ph(4-Me) | O |
| A-4216 | *c*-Pr | H | Me | F | CH₂CH₂OS(=O)₂Me | O |
| A-4217 | *c*-Pr | H | Me | F | CH₂CH₂CH₂OS(=O)₂Me | O |
| A-4218 | *c*-Pr | H | Me | F | CH₂CH₂CH₂OS(=O)₂CF₃ | O |

**[Table 97]**

| Compound Number | R¹ | R² | R³ | R⁴ | R⁵ | A |
|---|---|---|---|---|---|---|
| A-4219 | *c*-Pr | H | Me | F | CH₂CH₂CH₂CH₂OS(=O)₂CF₃ | O |
| A-4220 | *c*-Pr | H | Me | F | CH₂CH₂CH₂CH₂CH₂OS(=O)₂CF₃ | O |
| A-4221 | *c*-Pr | H | Me | F | CH₂CH₂CH₂CH₂CH₂OS(=O)₂CF₂CF₂CF₂CF₃ | O |
| A-4222 | *c*-Pr | H | Me | F | CH₂CH₂OS(=O)₂Ph | O |
| A-4223 | *c*-Pr | H | Me | F | CH₂CH₂OS(=O)₂Ph(4-Me) | O |
| A-4224 | *c*-Pr | H | Me | F | CH₂CH₂CH₂OS(=O)₂Ph | O |
| A-4225 | *c*-Pr | H | Me | F | CH₂CH₂CH₂OS(=O)₂Ph(4-Me) | O |
| A-4226 | *c*-Pr | H | Me | F | CH₂CH₂NH₂ | O |
| A-4227 | *c*-Pr | H | Me | F | CH₂CH₂CH₂NH₂ | O |
| A-4228 | *c*-Pr | H | Me | F | CH₂CH₂CH₂CH₂NH₂ | O |
| A-4229 | *c*-Pr | H | Me | F | CH₂CH₂CH₂CH₂CH₂NH₂ | O |
| A-4230 | *c*-Pr | H | Me | F | CH₂CH₂CH₂CH₂CH₂CH₂NH₂ | O |
| A-4231 | *c*-Pr | H | Me | F | CH₂CH₂CH₂CH₂CH₂CH₂CH₂NH₂ | O |
| A-4232 | *c*-Pr | H | Me | F | CH₂CH(Me)NH₂ | O |
| A-4233 | *c*-Pr | H | Me | F | CH₂C*H(Me)NH₂: (R) | O |
| A-4234 | *c*-Pr | H | Me | F | CH₂C*H(Me)NH₂: (S) | O |
| A-4235 | *c*-Pr | H | Me | F | CH₂CH(Et)NH₂ | O |
| A-4236 | *c*-Pr | H | Me | F | CH₂CH(*i*-Pr)NH₂ | O |
| A-4237 | *c*-Pr | H | Me | F | CH(Me)CH₂NH₂ | O |
| A-4238 | *c*-Pr | H | Me | F | CH(Et)CH₂NH₂ | O |
| A-4239 | *c*-Pr | H | Me | F | CH(*i*-Pr)CH₂NH₂ | O |
| A-4240 | *c*-Pr | H | Me | F | CH₂CH₂CH(Me)NH₂ | O |
| A-4241 | *c*-Pr | H | Me | F | CH₂CH(Me)CH₂NH₂ | O |
| A-4242 | *c*-Pr | H | Me | F | CH(Me)CH₂CH₂NH₂ | O |
| A-4243 | *c*-Pr | H | Me | F | CH₂CH(Me)NH(*c*-Pr) | O |
| A-4244 | *c*-Pr | H | Me | F | CH₂CH(Me)NHOMe | O |
| A-4245 | *c*-Pr | H | Me | F | CH₂CH(Me)N(OMe)S(=O)₂Ph(2-NO₂) | O |
| A-4246 | *c*-Pr | H | Me | F | CH₂CH(Me)N(OMe)S(=O)₂Ph(4-NO₂) | O |
| A-4247 | *c*-Pr | H | Me | F | CH₂CH₂N(Me)(*t*-Bu) | O |
| A-4248 | *c*-Pr | H | Me | F | CH₂CH₂CH₂N(Me)(*t*-Bu) | O |
| A-4249 | *c*-Pr | H | Me | F | CH₂CH₂CH₂CH₂N(Me)(*t*-Bu) | O |
| A-4250 | *c*-Pr | H | Me | F | CH₂CH₂NHCH₂CF₃ | O |
| A-4251 | *c*-Pr | H | Me | F | CH₂CH₂CH₂NHCH₂CF₃ | O |
| A-4252 | *c*-Pr | H | Me | F | CH₂CH₂CH₂CH₂NHCH₂CF₃ | O |
| A-4253 | *c*-Pr | H | Me | F | CH₂CH₂NHC(=O)C(Me)(CF₃)₂ | O |
| A-4254 | *c*-Pr | H | Me | F | CH₂CH₂NHC(=O)Me | O |
| A-4255 | *c*-Pr | H | Me | F | CH₂CH₂NHC(=O)(*t*-Bu) | O |
| A-4256 | *c*-Pr | H | Me | F | CH₂CH₂CH₂CH₂NHC(=O)CH(CH₃)₂ | O |
| A-4257 | *c*-Pr | H | Me | F | CH₂CH₂CH₂CH₂NHC(=O)(*t*-Bu) | O |
| A-4258 | *c*-Pr | H | Me | F | CH₂CH₂CH₂CH₂NHC(=O)CH₂(*t*-Bu) | O |
| A-4259 | *c*-Pr | H | Me | F | CH₂CH₂NHC(=O)CF₃ | O |
| A-4260 | *c*-Pr | H | Me | F | CH₂CH₂NHC(=O)CH₂CF₃ | O |
| A-4261 | *c*-Pr | H | Me | F | CH₂CH₂NHC(=O)CF₂CF₃ | O |
| A-4262 | *c*-Pr | H | Me | F | CH₂CH₂CH₂NHC(=O)CF₃ | O |

**[Table 98]**

| Compound Number | R¹ | R² | R³ | R⁴ | R⁵ | A |
|---|---|---|---|---|---|---|
| A-4263 | *c*-Pr | H | Me | F | CH₂CH₂CH₂NHC(=O)CH₂CF₃ | O |
| A-4264 | *c*-Pr | H | Me | F | CH₂CH₂CH₂NHC(=O)CF₂CF₃ | O |
| A-4265 | *c*-Pr | H | Me | F | CH₂CH₂CH₂CH₂NHC(=O)CF₃ | O |
| A-4266 | *c*-Pr | H | Me | F | CH₂CH₂CH₂CH₂NHC(=O)CH₂CF₃ | O |
| A-4267 | *c*-Pr | H | Me | F | CH₂CH₂CH₂CH₂NHC(=O)CF₂CF₃ | O |
| A-4268 | *c*-Pr | H | Me | F | CH₂CH₂CH₂CH₂NHC(=O)CF(CF₃)₂ | O |
| A-4269 | *c*-Pr | H | Me | F | CH₂CH₂CH₂CH₂NHC(=O)CCl₃ | O |
| A-4270 | *c*-Pr | H | Me | F | CH₂CH(Me)NHC(=O)CF₃ | O |
| A-4271 | *c*-Pr | H | Me | F | CH₂CH₂NHC(=O)Ph | O |
| A-4272 | *c*-Pr | H | Me | F | CH₂CH₂NHC(=O)Ph(4-CF₃) | O |
| A-4273 | *c*-Pr | H | Me | F | CH₂CH₂CH₂NHC(=O)Ph(4-CF₃) | O |
| A-4274 | *c*-Pr | H | Me | F | CH₂CH₂CH₂CH₂NHC(=O)Ph(4-CF₃) | O |
| A-4275 | *c*-Pr | H | Me | F | CH₂CH₂NHC(=O)O(*t*-Bu) | O |
| A-4276 | *c*-Pr | H | Me | F | CH₂CH₂CH₂NHC(=O)O(*t*-Bu) | O |
| A-4277 | *c*-Pr | H | Me | F | CH₂CH₂CH₂CH₂NHC(=O)O(*t*-Bu) | O |
| A-4278 | *c*-Pr | H | Me | F | CH₂CH₂CH₂CH₂NHC(=O)OCH(CH₃)₂ | O |
| A-4279 | *c*-Pr | H | Me | F | CH₂CH(Me)NHC(=O)O(*t*-Bu) | O |
| A-4280 | *c*-Pr | H | Me | F | CH₂C*H(Me)NHC(=O)O(*t*-Bu) : (R) | O |
| A-4281 | *c*-Pr | H | Me | F | CH₂C*H(Me)NHC(=O)O(*t*-Bu) : (S) | O |
| A-4282 | *c*-Pr | H | Me | F | CH₂CH(Et)NHC(=O)O(*t*-Bu) | O |
| A-4283 | *c*-Pr | H | Me | F | CH₂CH(*i*-Pr)NHC(=O)O(*t*-Bu) | O |
| A-4284 | *c*-Pr | H | Me | F | CH(Me)CH₂NHC(=O)O(*t*-Bu) | O |
| A-4285 | *c*-Pr | H | Me | F | CH(Et)CH₂NHC(=O)O(*t*-Bu) | O |
| A-4286 | *c*-Pr | H | Me | F | CH(*i*-Pr)CH₂NHC(=O)O(*t*-Bu) | O |
| A-4287 | *c*-Pr | H | Me | F | CH₂CH(Me)CH₂NHC(=O)O(*t*-Bu) | O |
| A-4288 | *c*-Pr | H | Me | F | CH₂CH₂NHC(=O)OC(Me)₂CF₃ | O |
| A-4289 | *c*-Pr | H | Me | F | CH₂CH₂CH₂NHC(=O)OCH₂CF₃ | O |
| A-4290 | *c*-Pr | H | Me | F | CH₂CH₂CH₂NHC(=O)OCH₂CH₂CF₃ | O |
| A-4291 | *c*-Pr | H | Me | F | CH₂CH₂CH₂CH₂NHC(=O)OCH₂CCl₃ | O |
| A-4292 | *c*-Pr | H | Me | F | CH₂CH₂CH₂CH₂NHC(=O)OCH₂CF₃ | O |
| A-4293 | *c*-Pr | H | Me | F | CH₂CH₂NHC(=O)NH(*t*-Bu) | O |
| A-4294 | *c*-Pr | H | Me | F | CH₂CH₂CH₂NHC(=O)NH(*t*-Bu) | O |
| A-4295 | *c*-Pr | H | Me | F | CH₂CH₂CH₂CH₂NHC(=O)NHEt | O |
| A-4296 | *c*-Pr | H | Me | F | CH₂CH₂CH₂CH₂NHC(=O)NH(*t*-Bu) | O |
| A-4297 | *c*-Pr | H | Me | F | CH₂CH₂CH₂NHC(=O)NHCH₂CCl₃ | O |
| A-4298 | *c*-Pr | H | Me | F | CH₂CH₂CH₂NHC(=O)NHCH₂CH₂F | O |
| A-4299 | *c*-Pr | H | Me | F | CH₂CH₂CH₂NHC(=O)NHCH₂CF₃ | O |
| A-4300 | *c*-Pr | H | Me | F | CH₂CH₂CH₂CH₂NHC(=O)NHCH₂CCl₃ | O |
| A-4301 | *c*-Pr | H | Me | F | CH₂CH₂CH₂CH₂NHC(=O)NHCH₂CH₂F | O |
| A-4302 | *c*-Pr | H | Me | F | CH₂CH₂CH₂CH₂NHC(=O)NHCH₂CF₃ | O |
| A-4303 | *c*-Pr | H | Me | F | CH₂CH₂NHS(=O)Me | O |
| A-4304 | *c*-Pr | H | Me | F | CH₂CH₂CH₂NHS(=O)Me | O |
| A-4305 | *c*-Pr | H | Me | F | CH₂CH₂CH₂CH₂NHS(=O)Me | O |
| A-4306 | *c*-Pr | H | Me | F | CH₂CH₂CH₂CH₂NHS(=O)CH(CH₃)₂ | O |

**[Table 99]**

| Compound Number | R¹ | R² | R³ | R⁴ | R⁵ | A |
|---|---|---|---|---|---|---|
| A-4307 | *c*-Pr | H | Me | F | CH₂CH₂CH₂CH₂CH₂NHS(=O)Me | O |
| A-4308 | *c*-Pr | H | Me | F | CH₂CH₂NHS(=O)CHF₂ | O |
| A-4309 | *c*-Pr | H | Me | F | CH₂CH₂CH₂NHS(=O)CHF₂ | O |
| A-431 0 | *c*-Pr | H | Me | F | CH₂CH₂CH₂CH₂NHS(=O)CHF₂ | O |
| A-4311 | *c*-Pr | H | Me | F | CH₂CH₂CH₂CH₂CH₂NHS(=O)CHF₂ | O |
| A-4312 | *c*-Pr | H | Me | F | CH₂CH(Me)NHS(=O)CHF₂ | O |
| A-4313 | *c*-Pr | H | Me | F | CH(Me)CH₂NHS(=O)CHF₂ | O |
| A-4314 | *c*-Pr | H | Me | F | CH₂CH₂CH(Me)NHS(=O)CHF₂ | O |
| A-4315 | *c*-Pr | H | Me | F | CH₂CH(Me)CH₂NHS(=O)CHF₂ | O |
| A-4316 | *c*-Pr | H | Me | F | CH(Me)CH₂CH₂NHS(=O)CHF₂ | O |
| A-4317 | *c*-Pr | H | Me | F | CH₂CH₂NHS(=O)CF₃ | O |
| A-4318 | *c*-Pr | H | Me | F | CH₂CH₂CH₂NHS(=O)CF₃ | O |
| A-4319 | *c*-Pr | H | Me | F | CH₂CH₂CH₂CH₂NHS(=O)CF₃ | O |
| A-4320 | *c*-Pr | H | Me | F | CH₂CH₂CH₂CH₂CH₂NHS(=O)CF₃ | O |
| A-4321 | *c*-Pr | H | Me | F | CH₂CH₂CH₂NHS(=O)Ph | O |
| A-4322 | *c*-Pr | H | Me | F | CH₂CH₂CH₂CH₂NHS(=O)Ph | O |
| A-4323 | *c*-Pr | H | Me | F | CH₂CH₂CH₂CH₂CH₂NHS(=O)Ph | O |
| A-4324 | *c*-Pr | H | Me | F | CH₂CH₂CH₂CH₂CH₂NHS(=O)Ph(4-CF₃) | O |
| A-4325 | *c*-Pr | H | Me | F | CH₂CH(Me)NHS(=O)Ph(4-CF₃) | O |
| A-4326 | *c*-Pr | H | Me | F | CH₂CH(Et)NHS(=O)Ph(4-CF₃) | O |
| A-4327 | *c*-Pr | H | Me | F | CH(Me)CH₂NHS(=O)Ph(4-CF₃) | O |
| A-4328 | *c*-Pr | H | Me | F | CH(Et)CH₂NHS(=O)Ph(4-CF₃) | O |
| A-4329 | *c*-Pr | H | Me | F | CH₂CH(Et)CH₂NHS(=O)Ph(4-CF₃) | O |
| A-4330 | *c*-Pr | H | Me | F | CH₂CH₂NHS(=O)₂Me | O |
| A-4331 | *c*-Pr | H | Me | F | CH₂CH₂CH₂NHS(=O)₂Me | O |
| A-4332 | *c*-Pr | H | Me | F | CH₂CH₂CH₂CH₂NHS(=O)₂Me | O |
| A-4333 | *c*-Pr | H | Me | F | CH₂CH₂CH₂CH₂NHS(=O)₂CH(CH₃)₂ | O |
| A-4334 | *c*-Pr | H | Me | F | CH₂CH₂CH₂CH₂CH₂NHS(=O)₂Me | O |
| A-4335 | *c*-Pr | H | Me | F | CH₂CH₂NHS(=O)₂CHF₂ | O |
| A-4336 | *c*-Pr | H | Me | F | CH₂CH₂CH₂NHS(=O)₂CHF₂ | O |
| A-4337 | *c*-Pr | H | Me | F | CH₂CH₂CH₂CH₂NHS(=O)₂CHF₂ | O |
| A-4338 | *c*-Pr | H | Me | F | CH₂CH₂CH₂CH₂CH₂NHS(=O)₂CHF₂ | O |
| A-4339 | *c*-Pr | H | Me | F | CH₂CH(Me)NHS(=O)₂CHF₂ | O |
| A-4340 | *c*-Pr | H | Me | F | CH(Me)CH₂NHS(=O)₂CHF₂ | O |
| A-4341 | *c*-Pr | H | Me | F | CH₂CH₂CH(Me)NHS(=O)₂CHF₂ | O |
| A-4342 | *c*-Pr | H | Me | F | CH₂CH(Me)CH₂NHS(=O)₂CHF₂ | O |
| A-4343 | *c*-Pr | H | Me | F | CH(Me)CH₂CH₂NHS(=O)₂CHF₂ | O |
| A-4344 | *c*-Pr | H | Me | F | CH₂CH(Me)N(Me)S(=O)₂CHF₂ | O |
| A-4345 | *c*-Pr | H | Me | F | CH(Me)CH₂N(Me)S(=O)₂CHF₂ | O |
| A-4346 | *c*-Pr | H | Me | F | CH₂CH₂CH(Me)N(Me)S(=O)₂CHF₂ | O |
| A-4347 | *c*-Pr | H | Me | F | CH₂CH(Me)CH₂N(Me)S(=O)₂CHF₂ | O |
| A-4348 | *c*-Pr | H | Me | F | CH(Me)CH₂CH₂N(Me)S(=O)₂CHF₂ | O |
| A-4349 | *c*-Pr | H | Me | F | CH₂CH₂CH₂NHS(=O)₂Ph | O |
| A-4350 | *c*-Pr | H | Me | F | CH₂CH₂CH₂CH₂NHS(=O)₂Ph | O |

**[Table 100]**

| Compound Number | R¹ | R² | R³ | R⁴ | R⁵ | A |
|---|---|---|---|---|---|---|
| A-4351 | *c*-Pr | H | Me | F | CH₂CH₂CH₂CH₂CH₂NHS(=O)₂Ph | O |
| A-4352 | *c*-Pr | H | Me | F | CH₂CH₂CH₂CH₂CH₂NHS(=O)₂Ph(4-CF₃) | O |
| A-4353 | *c*-Pr | H | Me | F | CH₂CH(Me)NHS(=O)₂Ph(4-CF₃) | O |
| A-4354 | *c*-Pr | H | Me | F | CH₂CH(Et)NHS(=O)₂Ph(4-CF₃) | O |
| A-4355 | *c*-Pr | H | Me | F | CH(Me)CH₂NHS(=O)₂Ph(4-CF₃) | O |
| A-4356 | *c*-Pr | H | Me | F | CH(Et)CH₂NHS(=O)₂Ph(4-CF₃) | O |
| A-4357 | *c*-Pr | H | Me | F | CH₂CH(Me)CH₂NHS(=O)₂Ph(4-CF₃) | O |
| A-4358 | *c*-Pr | H | Me | F | CH₂CH₂SiMe₃ | O |
| A-4359 | *c*-Pr | H | Me | F | CH₂CH₂CH₂SiMe₃ | O |
| A-4360 | *c*-Pr | H | Me | F | CH₂CH₂CH₂CH₂SiMe₃ | O |
| A-4361 | *c*-Pr | H | Me | F | CH₂Ph | O |
| A-4362 | *c*-Pr | H | Me | F | CH₂Ph(2-CF₃) | O |
| A-4363 | *c*-Pr | H | Me | F | CH₂Ph(3-CF₃) | O |
| A-4364 | *c*-Pr | H | Me | F | CH₂Ph(4-CF₃) | O |
| A-4365 | *c*-Pr | H | Me | F | CH₂Ph(2-OCF₃) | O |
| A-4366 | *c*-Pr | H | Me | F | CH₂Ph(3-OCF₃) | O |
| A-4367 | *c*-Pr | H | Me | F | CH₂Ph(4-OCF₃) | O |
| A-4368 | *c*-Pr | H | Me | F | CH₂Ph(2-SCF₃) | O |
| A-4369 | *c*-Pr | H | Me | F | CH₂Ph(3-SCF₃) | O |
| A-4370 | *c*-Pr | H | Me | F | CH₂Ph(4-SCF₃) | O |
| A-4371 | *c*-Pr | H | Me | F | CH₂Ph(3-CH₂SCF₃) | O |
| A-4372 | *c*-Pr | H | Me | F | CH₂Ph(4-F) | O |
| A-4373 | *c*-Pr | H | Me | F | CH₂Ph(4-Cl) | O |
| A-4374 | *c*-Pr | H | Me | F | CH₂Ph(4-Br) | O |
| A-4375 | *c*-Pr | H | Me | F | CH₂Ph(4-Me) | O |
| A-4376 | *c*-Pr | H | Me | F | CH₂Ph[4-(*t*-Bu)] | O |
| A-4377 | *c*-Pr | H | Me | F | CH₂Ph(4-CN) | O |
| A-4378 | *c*-Pr | H | Me | F | CH₂Ph(4-NO₂) | O |
| A-4379 | *c*-Pr | H | Me | F | CH₂Ph(4-OCHF₂) | O |
| A-4380 | *c*-Pr | H | Me | F | CH₂Ph(4-SCHF₂) | O |
| A-4381 | *c*-Pr | H | Me | F | CH₂Ph(4-CH₂SCF₃) | O |
| A-4382 | *c*-Pr | H | Me | F | CH₂Ph[4-CF(CF₃)₂] | O |
| A-4383 | *c*-Pr | H | Me | F | CH₂Ph(4-CH₂SCF₃) | O |
| A-4384 | *c*-Pr | H | Me | F | CH₂Ph[4-Ph(4-CF₃)] | O |
| A-4385 | *c*-Pr | H | Me | F | CH₂Ph(2,4-Cl₂) | O |
| A-4386 | *c*-Pr | H | Me | F | CH₂Ph[2,5-(CF₃)₂] | O |
| A-4387 | *c*-Pr | H | Me | F | CH₂Ph(3,4-Cl₂) | O |
| A-4388 | *c*-Pr | H | Me | F | CH₂Ph(3-CF₃-4-F) | O |
| A-4389 | *c*-Pr | H | Me | F | CH₂Ph(3-CF₃-4-Cl) | O |
| A-4390 | *c*-Pr | H | Me | F | CH₂Ph(3-F-4-CF₃) | O |
| A-4391 | *c*-Pr | H | Me | F | CH₂Ph(2,4,6-F₃) | O |
| A-4392 | *c*-Pr | H | Me | F | CH₂Ph(3,4,5-F₃) | O |
| A-4393 | *c*-Pr | H | Me | F | CH₂Ph(2,3,4-F₃) | O |
| A-4394 | *c*-Pr | H | Me | F | CH₂Ph(3,4,5-Cl₃) | O |

**[Table 101]**

| Compound Number | R¹ | R² | R³ | R⁴ | R⁵ | A |
|---|---|---|---|---|---|---|
| A-4395 | *c*-Pr | H | Me | F | CH₂CH₂Ph | O |
| A-4396 | *c*-Pr | H | Me | F | CH₂CH₂Ph(4-F) | O |
| A-4397 | *c*-Pr | H | Me | F | CH₂CH₂Ph(4-Cl) | O |
| A-4398 | *c*-Pr | H | Me | F | CH₂CH₂Ph(4-Br) | O |
| A-4399 | *c*-Pr | H | Me | F | CH₂CH₂Ph[4-(*t*-Bu)] | O |
| A-4400 | *c*-Pr | H | Me | F | CH₂CH₂Ph(2-CF₃) | O |
| A-4401 | *c*-Pr | H | Me | F | CH₂CH₂Ph(3-CF₃) | O |
| A-4402 | *c*-Pr | H | Me | H | CH₂CH₂Ph(4-CF₃) | O |
| A-4403 | *c*-Pr | H | Me | F | CH₂CH₂Ph(4-CF₃) | O |
| A-4404 | *c*-Pr | H | Me | F | CH₂CH₂Ph(4-CF(CF₃)₂) | O |
| A-4405 | *c*-Pr | H | Me | F | CH₂CH₂Ph[4-(c-Pr)] | O |
| A-4406 | *c*-Pr | H | Me | F | CH₂CH₂Ph{4-[*c*-Pr(2,2-F₂)]} | O |
| A-4407 | *c*-Pr | H | Me | F | CH₂CH₂Ph(4-OMe) | O |
| A-4408 | *c*-Pr | H | Me | F | CH₂CH₂Ph(3-OCF₃) | O |
| A-4409 | *c*-Pr | H | Me | F | CH₂CH₂Ph(4-OCHF₂) | O |
| A-4410 | *c*-Pr | H | Me | F | CH₂CH₂Ph(4-OCF₃) | O |
| A-4411 | *c*-Pr | H | Me | F | CH₂CH₂Ph(4-SMe) | O |
| A-4412 | *c*-Pr | H | Me | F | CH₂CH₂Ph(4-SCHF₂) | O |
| A-4413 | *c*-Pr | H | Me | F | CH₂CH₂Ph(4-SCF₃) | O |
| A-4414 | *c*-Pr | H | Me | F | CH₂CH₂Ph[4-S(=O)Me] | O |
| A-4415 | *c*-Pr | H | Me | F | CH₂CH₂Ph[4-S(=O)CF₃] | O |
| A-4416 | *c*-Pr | H | Me | F | CH₂CH₂Ph[4-S(=O)₂Me] | O |
| A-4417 | *c*-Pr | H | Me | F | CH₂CH₂Ph[4-S(=O)₂CF₃] | O |
| A-4418 | *c*-Pr | H | Me | F | CH₂CH₂Ph(4-CH₂SMe) | O |
| A-4419 | *c*-Pr | H | Me | F | CH₂CH₂Ph(4-CH₂SCF₃) | O |
| A-4420 | *c*-Pr | H | Me | F | CH₂CH₂Ph[4-OS(=O)₂Me] | O |
| A-4421 | *c*-Pr | H | Me | F | CH₂CH₂Ph[4-OS(=O)₂CF₃] | O |
| A-4422 | *c*-Pr | H | Me | F | CH₂CH₂Ph[4-Ph(4-CF₃)] | O |
| A-4423 | *c*-Pr | H | Me | F | CH₂CH₂Ph(4-CH₂Ph) | O |
| A-4424 | *c*-Pr | H | Me | F | CH₂CH₂Ph(4-OCH₂Ph) | O |
| A-4425 | *c*-Pr | H | Me | F | CH₂CH₂Ph(4-CN) | O |
| A-4426 | *c*-Pr | H | Me | F | CH₂CH₂Ph(4-NO₂) | O |
| A-4427 | *c*-Pr | H | Me | F | CH₂CH₂Ph(2,4-Cl₂) | O |
| A-4428 | *c*-Pr | H | Me | F | CH₂CH₂Ph(3,4-Cl₂) | O |
| A-4429 | *c*-Pr | H | Me | F | CH₂CH₂Ph(3-CF₃-4-F) | O |
| A-4430 | *c*-Pr | H | Me | F | CH₂CH₂Ph(2-CF₃-4-F) | O |
| A-4431 | *c*-Pr | H | Me | F | CH₂CH₂Ph(3-F-4-CF₃) | O |
| A-4432 | *c*-Pr | H | Me | F | CH₂CH₂Ph(2-F-4-CF₃) | O |
| A-4433 | *c*-Pr | H | Me | F | CH₂CH₂Ph(3-Cl-4-OCHF₂) | O |
| A-4434 | *c*-Pr | H | Me | F | CH₂CH₂Ph(3,4,5-Cl₃) | O |
| A-4435 | *c*-Pr | H | Me | F | CH₂CH₂Ph(2,3,4-F₃) | O |
| A-4436 | *c*-Pr | H | Me | F | CH₂CH₂Ph(2,4,5-F₃) | O |
| A-4437 | *c*-Pr | H | Me | F | CH₂CH₂Ph(3,4,5-F₃) | O |
| A-4438 | *c*-Pr | H | Me | F | CH₂CH₂Ph(2,4,6-F₃) | O |

**[Table 102]**

| Compound Number | R¹ | R² | R³ | R⁴ | R⁵ | A |
|---|---|---|---|---|---|---|
| A-4439 | *c*-Pr | H | Me | F | CH₂CH₂CH₂Ph | O |
| A-4440 | *c*-Pr | H | Me | F | CH₂CH₂CH₂Ph(3-CF₃) | O |
| A-4441 | *c*-Pr | H | Me | F | CH₂CH₂CH₂Ph(4-CF₃) | O |
| A-4442 | *c*-Pr | H | Me | F | CH₂CH₂CH₂Ph(4-F) | O |
| A-4443 | *c*-Pr | H | Me | F | CH₂CH₂CH₂Ph[4-(*t*-Bu)] | O |
| A-4444 | *c*-Pr | H | Me | F | CH₂CH₂CH₂Ph(4-CN) | O |
| A-4445 | *c*-Pr | H | Me | F | CH₂CH₂CH₂Ph(4-CF₃) | O |
| A-4446 | *c*-Pr | H | Me | F | CH₂CH₂CH₂Ph(4-OCHF₂) | O |
| A-4447 | *c*-Pr | H | Me | F | CH₂CH₂CH₂Ph(4-OCF₃) | O |
| A-4448 | *c*-Pr | H | Me | F | CH₂CH₂CH₂Ph(4-SCHF₂) | O |
| A-4449 | *c*-Pr | H | Me | F | CH₂CH₂CH₂Ph(4-SCF₃) | O |
| A-4450 | *c*-Pr | H | Me | F | CH₂CH₂CH₂Ph[4-CF(CF₃)₂] | O |
| A-4451 | *c*-Pr | H | Me | F | CH₂CH₂CH₂Ph(3,4,5-F₃) | O |
| A-4452 | *c*-Pr | H | Me | F | CH₂CH₂CH₂Ph(2,4,6-F₃) | O |
| A-4453 | *c*-Pr | H | Me | F | CH₂CH₂CH₂CH₂Ph | O |
| A-4454 | *c*-Pr | H | Me | F | CH₂CH₂CH₂CH₂Ph(4-F) | O |
| A-4455 | *c*-Pr | H | Me | F | CH₂CH₂CH₂CH₂Ph(4-CF₃) | O |
| A-4456 | *c*-Pr | H | Me | F | CH₂CH₂CH₂CH₂Ph(4-OCF₃) | O |
| A-4457 | *c*-Pr | H | Me | F | CH₂CH₂CH₂CH₂Ph(4-SCF₃) | O |
| A-4458 | *c*-Pr | H | Me | F | CH₂CH₂CH₂CH₂CH₂Ph | O |
| A-4459 | *c*-Pr | H | Me | F | CH₂CH₂CH₂CH₂CH₂CH₂Ph | O |
| A-4460 | *c-*Pr | H | Me | F | CH₂CF₂Ph(4-F) | O |
| A-4461 | *c*-Pr | H | Me | F | CH₂CF₂Ph(4-CF₃) | O |
| A-4462 | *c*-Pr | H | Me | H | CH₂CF₂Ph(3,4,5-F₃) | O |
| A-4463 | *c*-Pr | H | Me | F | CH₂CF₂Ph(3,4,5-F₃) | O |
| A-4464 | *c*-Pr | H | Me | F | CH₂CH₂OPh | O |
| A-4465 | *c*-Pr | H | Me | F | CH₂CH₂OPh(4-F) | O |
| A-4466 | *c*-Pr | H | Me | F | CH₂CH₂OPh(4-CF₃) | O |
| A-4467 | *c*-Pr | H | Me | F | CH₂CH₂CH₂OPh | O |
| A-4468 | *c*-Pr | H | Me | F | CH₂CH₂CH₂OPh(4-Cl) | O |
| A-4469 | *c*-Pr | H | Me | F | CH₂CH₂CH₂OPh(4-CF₃) | O |
| A-4470 | *c*-Pr | H | Me | F | CH₂CH₂CH₂CH₂OPh(4-CF₃) | O |
| A-4471 | *c*-Pr | H | Me | F | CH₂CH₂CH₂CH₂OPh(4-OCF₃) | O |
| A-4472 | *c*-Pr | H | Me | F | CH₂CH₂CH₂CH₂CH₂OPh(4-CF₃) | O |
| A-4473 | *c*-Pr | H | Me | F | CH₂CH₂OCH₂Ph | O |
| A-4474 | *c*-Pr | H | Me | F | CH₂CH₂CH₂OCH₂Ph | O |
| A-4475 | *c*-Pr | H | Me | F | CH₂CH₂CH₂CH₂OCH₂Ph | O |
| A-4476 | *c*-Pr | H | Me | F | CH₂CH₂CH₂CH₂CH₂OCH₂Ph | O |
| A-4477 | *c*-Pr | H | Me | F | CH₂CH₂SPh | O |
| A-4478 | *c*-Pr | H | Me | F | CH₂CH₂CH₂SPh | O |
| A-4479 | *c*-Pr | H | Me | F | CH₂CH₂CH₂SPh(4-F) | O |
| A-4480 | *c*-Pr | H | Me | F | CH₂CH₂CH₂SPh(4-Cl) | O |
| A-4481 | *c*-Pr | H | Me | F | CH₂CH₂CH₂SPh(4-Br) | O |
| A-4482 | *c*-Pr | H | Me | F | CH₂CH₂CH₂SPh[4-(*t*-Bu)] | O |

**[Table 103]**

| Compound Number | R¹ | R² | R³ | R⁴ | R⁵ | A |
|---|---|---|---|---|---|---|
| A-4483 | *c*-Pr | H | Me | F | CH₂CH₂CH₂SPh(3-CF₃) | O |
| A-4484 | *c*-Pr | H | Me | F | CH₂CH₂CH₂SPh(4-CF₃) | O |
| A-4485 | *c*-Pr | H | Me | F | CH₂CH₂CH₂SPh(3-SCF₃) | O |
| A-4486 | *c*-Pr | H | Me | F | CH₂CH₂CH₂SPh(4-SCF₃) | O |
| A-4487 | *c*-Pr | H | Me | F | CH₂CH₂CH₂CH₂SPh | O |
| A-4488 | *c*-Pr | H | Me | F | CH₂CH₂CH₂CH₂SPh(4-F) | O |
| A-4489 | *c*-Pr | H | Me | F | CH₂CH₂CH₂CH₂SPh(4-Cl) | O |
| A-4490 | *c*-Pr | H | Me | F | CH₂CH₂CH₂CH₂SPh[4-(*t*-Bu)] | O |
| A-4491 | *c*-Pr | H | Me | F | CH₂CH₂CH₂CH₂SPh(4-CF₃) | O |
| A-4492 | *c*-Pr | H | Me | F | CH₂CH₂CH₂CH₂CH₂SPh | O |
| A-4493 | *c*-Pr | H | Me | F | CH₂CH₂CH₂CH₂CH₂SPh(4-F) | O |
| A-4494 | *c*-Pr | H | Me | F | CH₂CH₂CH₂CH₂CH₂SPh(4-Cl) | O |
| A-4495 | *c*-Pr | H | Me | F | CH₂CH₂CH₂CH₂CH₂SPh(4-CF₃) | O |
| A-4496 | *c*-Pr | H | Me | F | CH₂CH₂S(=O)Ph | O |
| A-4497 | *c*-Pr | H | Me | F | CH₂CH₂CH₂S(=O)Ph | O |
| A-4498 | *c*-Pr | H | Me | F | CH₂CH₂CH₂S(=O)Ph(4-F) | O |
| A-4499 | *c*-Pr | H | Me | F | CH₂CH₂CH₂S(=O)Ph[4-(*t*-Bu)] | O |
| A-4500 | *c*-Pr | H | Me | F | CH₂CH₂CH₂S(=O)Ph(4-CF₃) | O |
| A-4501 | *c*-Pr | H | Me | F | CH₂CH₂S(=O)₂Ph | O |
| A-4502 | *c*-Pr | H | Me | F | CH₂CH₂S(=O)₂Ph(4-CF₃) | O |
| A-4503 | *c*-Pr | H | Me | F | CH₂CH₂CH₂S(=O)₂Ph(4-F) | O |
| A-4504 | *c*-Pr | H | Me | F | CH₂CH₂CH₂S(=O)₂Ph(4-Cl) | O |
| A-4505 | *c*-Pr | H | Me | F | CH₂CH₂CH₂S(=O)₂Ph(4-CF₃) | O |
| A-4506 | *c*-Pr | H | Me | F | CH₂CH₂CH₂CH₂S(=O)₂Ph | O |
| A-4507 | *c*-Pr | H | Me | F | CH₂CH₂CH₂CH₂S(=O)₂Ph(4-Cl) | O |
| A-4508 | *c*-Pr | H | Me | F | CH₂CH₂CH₂CH₂S(=O)₂Ph(4-CF₃) | O |
| A-4509 | *c*-Pr | H | Me | F | CH₂CH₂SCH₂Ph | O |
| A-451 0 | *c*-Pr | H | Me | F | CH₂CH₂CH₂SCH₂Ph | O |
| A-4511 | *c*-Pr | H | Me | F | CH₂CH₂CH₂SCH₂Ph(2-Cl) | O |
| A-4512 | *c*-Pr | H | Me | F | CH₂CH₂CH₂SCH₂Ph(3-Cl) | O |
| A-4513 | *c*-Pr | H | Me | F | CH₂CH₂CH₂SCH₂Ph(4-Cl) | O |
| A-4514 | *c*-Pr | H | Me | F | CH₂CH₂CH₂SCH₂Ph(2-SCF₃) | O |
| A-4515 | *c*-Pr | H | Me | F | CH₂CH₂CH₂SCH₂Ph(3-CF₃) | O |
| A-4516 | *c*-Pr | H | Me | F | CH₂CH₂CH₂SCH₂Ph(4-CF₃) | O |
| A-4517 | *c*-Pr | H | Me | F | CH₂CH₂CH₂SCH₂Ph(4-NO₂) | O |
| A-4518 | *c*-Pr | H | Me | F | CH₂CH₂CH₂CH₂SCH₂Ph | O |
| A-4519 | *c*-Pr | H | Me | F | CH₂CH₂CH₂CH₂SCH₂Ph(4-Cl) | O |
| A-4520 | *c*-Pr | H | Me | F | CH₂CH₂CH₂CH₂SCH₂Ph(4-CF₃) | O |
| A-4521 | *c*-Pr | H | Me | F | CH₂CH₂CH₂CH₂SCH₂Ph(4-CN) | O |
| A-4522 | *c*-Pr | H | Me | F | CH₂CH₂CH₂CH₂CH₂SCH₂Ph | O |
| A-4523 | *c*-Pr | H | Me | F | CH₂CH₂SCH₂CH₂Ph | O |
| A-4524 | *c*-Pr | H | Me | F | CH₂CH₂CH₂SCH₂CH₂Ph | O |
| A-4525 | *c*-Pr | H | Me | F | CH₂CH₂CH₂CH₂SCH₂CH₂Ph | O |
| A-4526 | *c*-Pr | H | Me | F | CH₂CH₂SCH(Me)Ph | O |

**[Table 104]**

| Compound Number | R¹ | R² | R³ | R⁴ | R⁵ | A |
|---|---|---|---|---|---|---|
| A-4527 | *c*-Pr | H | Me | F | CH₂CH₂CH₂SCH(Me)Ph | O |
| A-4528 | *c*-Pr | H | Me | F | CH₂CH₂CH₂CH₂SCH(Me)Ph | O |
| A-4529 | *c*-Pr | H | Me | F | CH₂CH₂S(=O)CH₂Ph | O |
| A-4530 | *c*-Pr | H | Me | F | CH₂CH₂S(=O)CH₂Ph(4-CF₃) | O |
| A-4531 | *c*-Pr | H | Me | F | CH₂CH₂CH₂S(=O)CH₂Ph | O |
| A-4532 | *c*-Pr | H | Me | F | CH₂CH₂CH₂S(=O)CH₂Ph(2-SCF₃) | O |
| A-4533 | *c*-Pr | H | Me | F | CH₂CH₂CH₂S(=O)CH₂Ph(4-SCF₃) | O |
| A-4534 | *c*-Pr | H | Me | F | CH₂CH₂CH₂S(=O)CH₂Ph(4-Cl) | O |
| A-4535 | *c*-Pr | H | Me | F | CH₂CH₂CH₂S(=O)CH₂Ph(4-CF₃) | O |
| A-4536 | *c*-Pr | H | Me | F | CH₂CH₂CH₂CH₂S(=O)CH₂Ph | O |
| A-4537 | *c*-Pr | H | Me | F | CH₂CH₂CH₂CH₂S(=O)CH₂Ph(4-CF₃) | O |
| A-4538 | *c*-Pr | H | Me | F | CH₂CH₂CH₂CH₂CH₂S(=O)CH₂Ph(4-CF₃) | O |
| A-4539 | *c*-Pr | H | Me | F | CH₂CH₂S(=O)₂CH₂Ph | O |
| A-4540 | *c*-Pr | H | Me | F | CH₂CH₂S(=O)₂CH₂Ph(4-CF₃) | O |
| A-4541 | *c*-Pr | H | Me | F | CH₂CH₂CH₂S(=O)₂CH₂Ph | O |
| A-4542 | *c*-Pr | H | Me | F | CH₂CH₂CH₂S(=O)₂CH₂Ph(4-Cl) | O |
| A-4543 | *c*-Pr | H | Me | F | CH₂CH₂CH₂S(=O)₂CH₂Ph(4-CF₃) | O |
| A-4544 | *c*-Pr | H | Me | F | CH₂CH₂CH₂CH₂S(=O)₂CH₂Ph | O |
| A-4545 | *c*-Pr | H | Me | F | CH₂CH₂CH₂CH₂S(=O)₂CH₂Ph(4-CF₃) | O |
| A-4546 | *c*-Pr | H | Me | F | CH₂CH₂CH₂CH₂CH₂S(=O)₂CH₂Ph | O |
| A-4547 | *c*-Pr | H | Me | F | CH₂CH₂CH₂CH₂CH₂S(=O)₂CH₂Ph(4-CF₃) | O |
| A-4548 | *c*-Pr | H | Me | F | CH₂CH₂ON=CH(*t*-Bu) | O |
| A-4549 | *c*-Pr | H | Me | F | CH₂CH₂CH₂ON=CH(*t*-Bu) | O |
| A-4550 | *c*-Pr | H | Me | F | CH₂CH₂CH₂CH₂ON=C(Me)(*c*-Pr) | O |
| A-4551 | *c*-Pr | H | Me | F | CH₂CH₂ON=C(Me)CF₃ | O |
| A-4552 | *c*-Pr | H | Me | F | CH₂CH₂CH₂ON=CHCF₃ | O |
| A-4553 | *c*-Pr | H | Me | F | CH₂CH₂CH₂ON=C(Me)CF₃ | O |
| A-4554 | *c*-Pr | H | Me | F | CH₂CH₂CH₂ON=C(Me)CCl₃ | O |
| A-4555 | *c*-Pr | H | Me | F | CH₂CH₂CH₂CH₂ON=CHCF₃ | O |
| A-4556 | *c*-Pr | H | Me | F | CH₂CH₂CH₂CH₂ON=C(Me)CF₃ | O |
| A-4557 | *c*-Pr | H | Me | F | CH₂CH₂CH₂CH₂CH₂ON=C(Me)CF₃ | O |
| A-4558 | *c*-Pr | H | Me | F | CH₂CH₂ON=CHPh | O |
| A-4559 | *c*-Pr | H | Me | F | CH₂CH₂ON=CHPh(4-CF₃) | O |
| A-4560 | *c*-Pr | H | Me | F | CH₂CH₂ON=CHPh(4-SCF₃) | O |
| A-4561 | *c*-Pr | H | Me | F | CH₂CH₂CH₂ON=CHPh(3-CF₃) | O |
| A-4562 | *c*-Pr | H | Me | F | CH₂CH₂CH₂ON=CHPh(4-CF₃) | O |
| A-4563 | *c*-Pr | H | Me | F | CH₂CH₂CH₂ON=CHPh(4-SCF₃) | O |
| A-4564 | *c*-Pr | H | Me | F | CH₂CH₂CH₂ON=C(Me)Ph(4-CF₃) | O |
| A-4565 | *c*-Pr | H | Me | F | CH₂CH₂CH₂CH₂ON=CHPh(4-SCF₃) | O |
| A-4566 | *c*-Pr | H | Me | F | CH₂CH₂(adamant-1-yl) | O |
| A-4567 | *c*-Pr | H | Me | F | CH₂CH₂CH₂(3-CF₃-pyrazol-1-yl) | O |
| A-4568 | *c*-Pr | H | Me | F | CH₂CH₂CH₂CH₂(3-CF₃-pyrazol-1-yl) | O |
| A-4569 | *c*-Pr | H | Me | F | CH₂CH₂CH₂CH₂CH₂(3-CF₃-pyrazol-1-yl) | O |
| A-4570 | *c*-Pr | H | Me | F | CH₂(1-Me-4-CF₃-imidazol-5-yl) | O |

**[Table 105]**

| Compound Number | R¹ | R² | R³ | R⁴ | R⁵ | A |
|---|---|---|---|---|---|---|
| A-4571 | *c*-Pr | H | Me | F | CH₂CH₂(1-Me-4-CF₃-imidazol-5-yl) | O |
| A-4572 | *c*-Pr | H | Me | F | CH₂CH₂CH₂(1-Me-4-CF₃-imidazol-5-yl) | O |
| A-4573 | *c*-Pr | H | Me | F | CH₂CH₂CH₂(3-CF₃-1,2,4-triazol-1-yl) | O |
| A-4574 | *c*-Pr | H | Me | F | CH₂CH₂CH₂CH₂(3-CF₃-1,2,4-triazol-1-yl) | O |
| A-4575 | *c*-Pr | H | Me | F | CH₂CH₂CH₂CH₂CH₂(3-CF₃-1,2,4-triazol-1-yl) | O |
| A-4576 | *c*-Pr | H | Me | F | CH₂CH₂CH₂(4-CF₃-thiazol-2-yl) | O |
| A-4577 | *c*-Pr | H | Me | F | CH₂CH₂CH₂CH₂(4-CF₃-thiazol-2-yl) | O |
| A-4578 | *c*-Pr | H | Me | F | CH₂CH₂CH₂CH₂CH₂(4-CF₃-thiazol-2-yl) | O |
| A-4579 | *c*-Pr | H | Me | F | CH₂CH₂CH₂(2-CF₃-pyridin-5-yl) | O |
| A-4580 | *c*-Pr | H | Me | F | CH₂CH₂CH₂CH₂(2-CF₃-pyridin-5-yl) | O |
| A-4581 | *c*-Pr | H | Me | F | CH₂CH₂CH₂CH₂CH₂(2-CF₃-pyridin-5-yl) | O |
| A-4582 | *c*-Pr | H | Me | F | CH₂(4-CF₃-pyrimidin-2-yl) | O |
| A-4583 | *c*-Pr | H | Me | F | CH₂CH₂(4-CF₃-pyrimidin-2-yl) | O |
| A-4584 | *c*-Pr | H | Me | F | CH₂CH₂CH₂(4-CF₃-pyrimidin-2-yl) | O |
| A-4585 | *c*-Pr | H | Me | F | CH₂CH₂O(3-Cl-5-CF₃-pyridin-2-yl) | O |
| A-4586 | *c*-Pr | H | Me | F | CH₂CH₂CH₂O(3-Cl-5-CF₃-pyridin-2-yl) | O |
| A-4587 | *c*-Pr | H | Me | F | CH₂CH₂CH₂CH₂O(3-Cl-5-CF₃-pyridin-2-yl) | O |
| A-4588 | *c*-Pr | H | Me | F | CH₂CH₂CH₂CH₂CH₂O(3-Cl-5-CF₃-pyridin-2-yl) | O |
| A-4589 | *c*-Pr | H | Me | F | CH₂CH₂N(Phth) | O |
| A-4590 | *c*-Pr | H | Me | F | CH₂CH₂CH₂N(Phth) | O |
| A-4591 | *c*-Pr | H | Me | F | CH₂CH₂CH₂CH₂N(Phth) | O |
| A-4592 | *c*-Pr | H | Me | F | CH₂CH₂CH₂CH₂CH₂N(Phth) | O |
| A-4593 | *c*-Pr | H | Me | F | CH₂CH(Me)CH₂N(Phth) | O |
| A-4594 | *c*-Pr | H | Me | F | CH₂[1,3-dioxolan(2-CF₃)-2-yl] | O |
| A-4595 | *c*-Pr | H | Me | F | CH₂(azetidin-3-yl) | O |
| A-4596 | *c*-Pr | H | Me | F | CH₂(pyrrolidin-3-yl) | O |
| A-4597 | *c*-Pr | H | Me | F | CH₂(piperidin-3-yl) | O |
| A-4598 | *c*-Pr | H | Me | F | CH₂{azetidin[1-C(=O)O(t-Bu)]-3-yl} | O |
| A-4599 | *c*-Pr | H | Me | F | CH₂{pyrrolidin[1-C(=O)O(*t*-Bu)]-3-yl} | O |
| A-4600 | *c*-Pr | H | Me | F | CH₂{piperidin[1-C(=O)O(*t*-Bu)]-3-yl} | O |
| A-4601 | *c*-Pr | H | Me | F | CH₂{azetidin[1-C(=O)CF₃]-3-yl} | O |
| A-4602 | *c*-Pr | H | Me | F | CH₂{azetidin[1-S(=O)₂CF₃]-3-yl} | O |
| A-4603 | *c*-Pr | H | Me | F | CH₂{pyrrolidin[1-C(=O)CF₃]-3-yl} | O |
| A-4604 | *c*-Pr | H | Me | F | CH₂{pyrrolidin[1-S(=O)₂CF₃]-3-yl} | O |
| A-4605 | *c*-Pr | H | Me | F | CH₂{piperidin[1-C(=O)CF₃]-3-yl} | O |
| A-4606 | *c*-Pr | H | Me | F | CH₂{piperidin[1-S(=O)₂CF₃]-3-yl} | O |
| A-4607 | *c*-Pr | H | Me | F | CH₂CH₂CH₂CN | O |
| A-4608 | *c*-Pr | H | Me | F | CH₂CH₂CH₂CH₂CN | O |
| A-4609 | *c*-Pr | H | Me | F | CH₂CH₂CH₂CH₂CH₂CN | O |
| A-4610 | *c*-Pr | H | Me | F | CH₂CH₂CH₂CH₂CH₂CH₂CN | O |
| A-4611 | *c*-Pr | H | Me | F | CH₂CH₂CH₂C(=O)OH | O |
| A-4612 | *c*-Pr | H | Me | F | CH₂CH₂CH₂CH₂C(=O)OH | O |
| A-4613 | *c*-Pr | H | Me | F | CH₂CH₂CH₂CH₂CH₂C(=O)OH | O |
| A-4614 | *c*-Pr | H | Me | F | CH₂CH₂SCN | O |

**[Table 106]**

| Compound Number | R¹ | R² | R³ | R⁴ | R⁵ | A |
|---|---|---|---|---|---|---|
| A-4615 | *c*-Pr | H | Me | F | CH₂CH₂CH₂SCN | O |
| A-4616 | *c*-Pr | H | Me | F | CH₂CH₂CH₂CH₂SCN | O |
| A-4617 | *c*-Pr | H | Me | F | CH₂CH₂CH₂CH₂CH₂SCN | O |
| A-4618 | *c*-Pr | H | Me | Cl | CH₂CH₂CH₂CH₂CH₂SCN | O |
| A-4619 | *c*-Pr | H | Me | F | CH₂CH₂CH₂CH₂CH₂CH₂SCN | O |
| A-4620 | *c*-Pr | H | Me | F | CH₂CH₂CH₂CH₂CH₂CH₂CH₂SCN | O |
| A-4621 | *c*-Pr | H | Me | F | CH₂CH₂CH₂CH₂CH₂CH₂CH₂CH₂SCN | O |
| A-4622 | *c*-Pr | H | Me | F | CH₂CH₂CH(Me)SCN | O |
| A-4623 | *c*-Pr | H | Me | F | CH₂CH(Me)CH₂SCN | O |
| A-4624 | *c*-Pr | H | Me | F | CH(Me)CH₂CH₂SCN | O |
| A-4625 | *c*-Pr | H | Me | F | CH₂CH₂CH₂CH(Me)SCN | O |
| A-4626 | *c*-Pr | H | Me | F | CH₂CH₂CH(Me)CH₂SCN | O |
| A-4627 | *c*-Pr | H | Me | F | CH₂CH(Me)CH₂CH₂SCN | O |
| A-4628 | *c*-Pr | H | Me | F | CH(Me)CH₂CH₂CH₂SCN | O |
| A-4629 | *c*-Pr | H | Me | F | CH₂CH₂CH₂CH₂CH(Me)SCN | O |
| A-4630 | *c*-Pr | H | Me | F | CH₂CH₂CH₂CH(Me)CH₂SCN | O |
| A-4631 | *c*-Pr | H | Me | F | CH₂CH₂CH(Me)CH₂CH₂SCN | O |
| A-4632 | *c*-Pr | H | Me | F | CH₂CH(Me)CH₂CH₂CH₂SCN | O |
| A-4633 | *c*-Pr | H | Me | F | CH(Me)CH₂CH₂CH₂CH₂SCN | O |
| A-4634 | *c*-Pr | H | Me | F | CH₂CH₂ONH₂ | O |
| A-4635 | *c*-Pr | H | Me | F | CH₂CH₂CH₂ONH₂ | O |
| A-4636 | *c*-Pr | H | Me | F | CH₂CH₂CH₂CH₂ONH₂ | O |
| A-4637 | *c*-Pr | H | Me | F | CH₂CH₂CH₂CH₂CH₂ONH₂ | O |
| A-4638 | *c*-Pr | H | Me | F | CH₂CH₂CH₂CH₂CH₂CH₂ONH₂ | O |
| A-4639 | CH₂CF₃ | H | Cl | H | CH₂CH₂SCF₃ | O |
| A-4640 | CH₂CF₃ | H | Cl | H | CH₂CH₂CH₂SCF₃ | O |
| A-4641 | CH₂CF₃ | H | Cl | H | CH₂CH₂CH₂CH₂SCF₃ | O |
| A-4642 | CH₂CF₃ | H | Cl | H | CH₂CH₂CH₂CH₂CH₂SCF₃ | O |
| A-4643 | CH₂CF₃ | H | Cl | H | CH₂CH₂CH₂CH₂CH₂CH₂SCF₃ | O |
| A-4644 | CH₂CF₃ | H | Cl | H | CH₂CH₂CH₂CH₂CH₂CH₂CH₂SCF₃ | O |
| A-4645 | CH₂CF₃ | H | Cl | H | CH₂CH₂CH₂CH₂CH₂CH₂CH₂CH₂SCF₃ | O |
| A-4646 | CH₂CF₃ | H | Cl | H | CH₂CH₂NHS(=O)₂CF₃ | O |
| A-4647 | CH₂CF₃ | H | Cl | H | CH₂CH₂CH₂NHS(=O)₂CF₃ | O |
| A-4648 | CH₂CF₃ | H | Cl | H | CH₂CH₂CH₂CH₂NHS(=O)₂CF₃ | O |
| A-4649 | CH₂CF₃ | H | Cl | H | CH₂CH₂CH₂CH₂CH₂NHS(=O)₂CF₃ | O |
| A-4650 | CH₂CF₃ | H | Cl | H | CH₂CH₂N(Me)S(=O)₂CF₃ | O |
| A-4651 | CH₂CF₃ | H | Cl | H | CH₂CH₂N(Et)S(=O)₂CF₃ | O |
| A-4652 | CH₂CF₃ | H | Cl | H | CH₂CH₂N(CH₂CN)S(=O)₂CF₃ | O |
| A-4653 | CH₂CF₃ | H | Cl | H | CH₂CH₂N(CH₂OMe)S(=O)₂CF₃ | O |
| A-4654 | CH₂CF₃ | H | Cl | H | CH₂CH₂CH₂N(Me)S(=O)₂CF₃ | O |
| A-4655 | CH₂CF₃ | H | Cl | H | CH₂CH₂CH₂N(Et)S(=O)₂CF₃ | O |
| A-4656 | CH₂CF₃ | H | Cl | H | CH₂CH₂CH₂N(CH₂CN)S(=O)₂CF₃ | O |
| A-4657 | CH₂CF₃ | H | Cl | H | CH₂CH₂CH₂N(CH₂OMe)S(=O)₂CF₃ | O |
| A-4658 | CH₂CF₃ | H | Cl | H | CH₂CH₂CH₂CH₂N(Me)S(=O)₂CF₃ | O |

**[Table 107]**

| Compound Number | R¹ | R² | R³ | R⁴ | R⁵ | A |
|---|---|---|---|---|---|---|
| A-4659 | CH₂CF₃ | H | Cl | H | CH₂CH₂CH₂CH₂N(Et)S(=O)₂CF₃ | O |
| A-4660 | CH₂CF₃ | H | Cl | H | CH₂CH₂CH₂CH₂N(CH₂CN)S(=O)₂CF₃ | O |
| A-4661 | CH₂CF₃ | H | Cl | H | CH₂CH₂CH₂CH₂N(CH₂OMe)S(=O)₂CF₃ | O |
| A-4662 | CH₂CF₃ | H | Me | H | CH₂CH₂SCF₃ | O |
| A-4663 | CH₂CF₃ | H | Me | H | CH₂CH₂CH₂SCF₃ | O |
| A-4664 | CH₂CF₃ | H | Me | H | CH₂CH₂CH₂CH₂SCF₃ | O |
| A-4665 | CH₂CF₃ | H | Me | H | CH₂CH₂CH₂CH₂CH₂SCF₃ | O |
| A-4666 | CH₂CF₃ | H | Me | H | CH₂CH₂CH₂CH₂CH₂CH₂SCF₃ | O |
| A-4667 | CH₂CF₃ | H | Me | H | CH₂CH₂CH₂CH₂CH₂CH₂CH₂SCF₃ | O |
| A-4668 | CH₂CF₃ | H | Me | H | CH₂CH₂CH₂CH₂CH₂CH₂CH₂CH₂SCF₃ | O |
| A-4669 | CH₂CF₃ | H | Me | H | CH₂CH₂NHS(=O)₂CF₃ | O |
| A-4670 | CH₂CF₃ | H | Me | H | CH₂CH₂CH₂NHS(=O)₂CF₃ | O |
| A-4671 | CH₂CF₃ | H | Me | H | CH₂CH₂CH₂CH₂NHS(=O)₂CF₃ | O |
| A-4672 | CH₂CF₃ | H | Me | H | CH₂CH₂CH₂CH₂CH₂NHS(=O)₂CF₃ | O |
| A-4673 | CH₂CF₃ | H | Me | H | CH₂CH₂N(Me)S(=O)₂CF₃ | O |
| A-4674 | CH₂CF₃ | H | Me | H | CH₂CH₂N(Et)S(=O)₂CF₃ | O |
| A-4675 | CH₂CF₃ | H | Me | H | CH₂CH₂N(CH₂CN)S(=O)₂CF₃ | O |
| A-4676 | CH₂CF₃ | H | Me | H | CH₂CH₂N(CH₂OMe)S(=O)₂CF₃ | O |
| A-4677 | CH₂CF₃ | H | Me | H | CH₂CH₂CH₂N(Me)S(=O)₂CF₃ | O |
| A-4678 | CH₂CF₃ | H | Me | H | CH₂CH₂CH₂N(Et)S(=O)₂CF₃ | O |
| A-4679 | CH₂CF₃ | H | Me | H | CH₂CH₂CH₂N(CH₂CN)S(=O)₂CF₃ | O |
| A-4680 | CH₂CF₃ | H | Me | H | CH₂CH₂CH₂N(CH₂OMe)S(=O)₂CF₃ | O |
| A-4681 | CH₂CF₃ | H | Me | H | CH₂CH₂CH₂CH₂N(Me)S(=O)₂CF₃ | O |
| A-4682 | CH₂CF₃ | H | Me | H | CH₂CH₂CH₂CH₂N(Et)S(=O)₂CF₃ | O |
| A-4683 | CH₂CF₃ | H | Me | H | CH₂CH₂CH₂CH₂N(CH₂CN)S(=O)₂CF₃ | O |
| A-4684 | CH₂CF₃ | H | Me | H | CH₂CH₂CH₂CH₂N(CH₂OMe)S(=O)₂CF₃ | O |
| A-4685 | CH₂CF₃ | H | Cl | F | CH₂CH₂SCF₃ | O |
| A-4686 | CH₂CF₃ | H | Cl | F | CH₂CH₂CH₂SCF₃ | O |
| A-4687 | CH₂CF₃ | H | Cl | F | CH₂CH₂CH₂CH₂SCF₃ | O |
| A-4688 | CH₂CF₃ | H | F | F | CH₂CH₂CH₂CH₂CH₂SCF₃ | O |
| A-4689 | CH₂CF₃ | H | Cl | F | CH₂CH₂CH₂CH₂CH₂SCF₃ | O |
| A-4690 | CH₂CF₃ | H | Cl | F | CH₂CH₂CH₂CH₂CH₂CH₂SCF₃ | O |
| A-4691 | CH₂CF₃ | H | Cl | F | CH₂CH₂CH₂CH₂CH₂CH₂CH₂SCF₃ | O |
| A-4692 | CH₂CF₃ | H | Cl | F | CH₂CH₂CH₂CH₂CH₂CH₂CH₂CH₂SCF₃ | O |
| A-4693 | CH₂CF₃ | H | Cl | F | CH₂CH₂NHS(=O)₂CF₃ | O |
| A-4694 | CH₂CF₃ | H | Cl | F | CH₂CH₂CH₂NHS(=O)₂CF₃ | O |
| A-4695 | CH₂CF₃ | H | Cl | F | CH₂CH₂CH₂CH₂NHS(=O)₂CF₃ | O |
| A-4696 | CH₂CF₃ | H | Cl | F | CH₂CH₂CH₂CH₂CH₂NHS(=O)₂CF₃ | O |
| A-4697 | CH₂CF₃ | H | Cl | F | CH₂CH₂N(Me)S(=O)₂CF₃ | O |
| A-4698 | CH₂CF₃ | H | Cl | F | CH₂CH₂N(Et)S(=O)₂CF₃ | O |
| A-4699 | CH₂CF₃ | H | Cl | F | CH₂CH₂N(CH₂CN)S(=O)₂CF₃ | O |
| A-4700 | CH₂CF₃ | H | Cl | F | CH₂CH₂N(CH₂OMe)S(=O)₂CF₃ | O |
| A-4701 | CH₂CF₃ | H | Cl | F | CH₂CH₂CH₂N(Me)S(=O)₂CF₃ | O |
| A-4702 | CH₂CF₃ | H | Cl | F | CH₂CH₂CH₂N(Et)S(=O)₂CF₃ | O |

**[Table 108]**

| Compound Number | R¹ | R² | R³ | R⁴ | R⁵ | A |
|---|---|---|---|---|---|---|
| A-4703 | CH₂CF₃ | H | Cl | F | CH₂CH₂CH₂N(CH₂CN)S(=O)₂CF₃ | O |
| A-4704 | CH₂CF₃ | H | Cl | F | CH₂CH₂CH₂N(CH₂OMe)S(=O)₂CF₃ | O |
| A-4705 | CH₂CF₃ | H | Cl | F | CH₂CH₂CH₂CH₂N(Me)S(=O)₂CF₃ | O |
| A-4706 | CH₂CF₃ | H | Cl | F | CH₂CH₂CH₂CH₂N(Et)S(=O)₂CF₃ | O |
| A-4707 | CH₂CF₃ | H | Cl | F | CH₂CH₂CH₂CH₂N(CH₂CN)S(=O)₂CF₃ | O |
| A-4708 | CH₂CF₃ | H | Cl | F | CH₂CH₂CH₂CH₂N(CH₂OMe)S(=O)₂CF₃ | O |
| A-4709 | CH₂CF₃ | H | Me | F | CH₂CH₂SCF₃ | O |
| A-4710 | CH₂CF₃ | H | Me | F | CH₂CH₂CH₂SCF₃ | O |
| A-4711 | CH₂CF₃ | H | Me | F | CH₂CH₂CH₂CH₂SCF₃ | O |
| A-4712 | CH₂CF₃ | H | Me | F | CH₂CH₂CH₂CH₂CH₂SCF₃ | O |
| A-4713 | CH₂CF₃ | H | Me | F | CH₂CH₂CH₂CH₂CH₂CH₂SCF₃ | O |
| A-4714 | CH₂CF₃ | H | Me | F | CH₂CH₂CH₂CH₂CH₂CH₂CH₂SCF₃ | O |
| A-4715 | CH₂CF₃ | H | Me | F | CH₂CH₂CH₂CH₂CH₂CH₂CH₂CH₂SCF₃ | O |
| A-4716 | CH₂CF₃ | H | Me | F | CH₂CH₂NHS(=O)₂CF₃ | O |
| A-4717 | CH₂CF₃ | H | Me | F | CH₂CH₂CH₂NHS(=O)₂CF₃ | O |
| A-4718 | CH₂CF₃ | H | Me | F | CH₂CH₂CH₂CH₂NHS(=O)₂CF₃ | O |
| A-4719 | CH₂CF₃ | H | Me | F | CH₂CH₂CH₂CH₂CH₂NHS(=O)₂CF₃ | O |
| A-4720 | CH₂CF₃ | H | Me | F | CH₂CH₂N(Me)S(=O)₂CF₃ | O |
| A-4721 | CH₂CF₃ | H | Me | F | CH₂CH₂N(Et)S(=O)₂CF₃ | O |
| A-4722 | CH₂CF₃ | H | Me | F | CH₂CH₂N(CH₂CN)S(=O)₂CF₃ | O |
| A-4723 | CH₂CF₃ | H | Me | F | CH₂CH₂N(CH₂OMe)S(=O)₂CF₃ | O |
| A-4724 | CH₂CF₃ | H | Me | F | CH₂CH₂CH₂N(Me)S(=O)₂CF₃ | O |
| A-4725 | CH₂CF₃ | H | Me | F | CH₂CH₂CH₂N(Et)S(=O)₂CF₃ | O |
| A-4726 | CH₂CF₃ | H | Me | F | CH₂CH₂CH₂N(CH₂CN)S(=O)₂CF₃ | O |
| A-4727 | CH₂CF₃ | H | Me | F | CH₂CH₂CH₂N(CH₂OMe)S(=O)₂CF₃ | O |
| A-4728 | CH₂CF₃ | H | Me | F | CH₂CH₂CH₂CH₂N(Me)S(=O)₂CF₃ | O |
| A-4729 | CH₂CF₃ | H | Me | F | CH₂CH₂CH₂CH₂N(Et)S(=O)₂CF₃ | O |
| A-4730 | CH₂CF₃ | H | Me | F | CH₂CH₂CH₂CH₂N(CH₂CN)S(=O)₂CF₃ | O |
| A-4731 | CH₂CF₃ | H | Me | F | CH₂CH₂CH₂CH₂N(CH₂OMe)S(=O)₂CF₃ | O |
| A-4732 | CH₂CF₃ | H | Cl | Cl | CH₂CH₂SCF₃ | O |
| A-4733 | CH₂CF₃ | H | Cl | Cl | CH₂CH₂CH₂SCF₃ | O |
| A-4734 | CH₂CF₃ | H | Cl | Cl | CH₂CH₂CH₂CH₂SCF₃ | O |
| A-4735 | CH₂CF₃ | H | Cl | Cl | CH₂CH₂CH₂CH₂CH₂SCF₃ | O |
| A-4736 | CH₂CF₃ | H | Cl | Cl | CH₂CH₂CH₂CH₂CH₂CH₂SCF₃ | O |
| A-4737 | CH₂CF₃ | H | Cl | Cl | CH₂CH₂CH₂CH₂CH₂CH₂CH₂SCF₃ | O |
| A-4738 | CH₂CF₃ | H | Cl | Cl | CH₂CH₂CH₂CH₂CH₂CH₂CH₂CH₂SCF₃ | O |
| A-4739 | CH₂CF₃ | H | Cl | Cl | CH₂CH₂NHS(=O)₂CF₃ | O |
| A-4740 | CH₂CF₃ | H | Cl | Cl | CH₂CH₂CH₂NHS(=O)₂CF₃ | O |
| A-4741 | CH₂CF₃ | H | Cl | Cl | CH₂CH₂CH₂CH₂NHS(=O)₂CF₃ | O |
| A-4742 | CH₂CF₃ | H | Cl | Cl | CH₂CH₂CH₂CH₂CH₂NHS(=O)₂CF₃ | O |
| A-4743 | CH₂CF₃ | H | Cl | Cl | CH₂CH₂N(Me)S(=O)₂CF₃ | O |
| A-4744 | CH₂CF₃ | H | Cl | Cl | CH₂CH₂N(Et)S(=O)₂CF₃ | O |
| A-4745 | CH₂CF₃ | H | Cl | Cl | CH₂CH₂N(CH₂CN)S(=O)₂CF₃ | O |
| A-4746 | CH₂CF₃ | H | Cl | Cl | CH₂CH₂N(CH₂OMe)S(=O)₂CF₃ | O |
| | | | | | | |

**[Table 109]**

| Compound Number | R¹ | R² | R³ | R⁴ | R⁵ | A |
|---|---|---|---|---|---|---|
| A-4747 | CH₂CF₃ | H | Cl | Cl | CH₂CH₂CH₂N(Me)S(=O)₂CF₃ | O |
| A-4748 | CH₂CF₃ | H | Cl | Cl | CH₂CH₂CH₂N(Et)S(=O)₂CF₃ | O |
| A-4749 | CH₂CF₃ | H | Cl | Cl | CH₂CH₂CH₂N(CH₂CN)S(=O)₂CF₃ | O |
| A-4750 | CH₂CF₃ | H | Cl | Cl | CH₂CH₂CH₂N(CH₂OMe)S(=O)₂CF₃ | O |
| A-4751 | CH₂CF₃ | H | Cl | Cl | CH₂CH₂CH₂CH₂N(Me)S(=O)₂CF₃ | O |
| A-4752 | CH₂CF₃ | H | Cl | Cl | CH₂CH₂CH₂CH₂N(Et)S(=O)₂CF₃ | O |
| A-4753 | CH₂CF₃ | H | Cl | Cl | CH₂CH₂CH₂CH₂N(CH₂CN)S(=O)₂CF₃ | O |
| A-4754 | CH₂CF₃ | H | Cl | Cl | CH₂CH₂CH₂CH₂N(CH₂OMe)S(=O)₂CF₃ | O |
| A-4755 | CH₂CF₃ | H | Me | Cl | CH₂CH₂SCF₃ | O |
| A-4756 | CH₂CF₃ | H | Me | Cl | CH₂CH₂CH₂SCF₃ | O |
| A-4757 | CH₂CF₃ | H | Me | Cl | CH₂CH₂CH₂CH₂SCF₃ | O |
| A-4758 | CH₂CF₃ | H | Me | Cl | CH₂CH₂CH₂CH₂CH₂SCF₃ | O |
| A-4759 | CH₂CF₃ | H | Me | Cl | CH₂CH₂CH₂CH₂CH₂CH₂SCF₃ | O |
| A-4760 | CH₂CF₃ | H | Me | Cl | CH₂CH₂CH₂CH₂CH₂CH₂CH₂SCF₃ | O |
| A-4761 | CH₂CF₃ | H | Me | Cl | CH₂CH₂CH₂CH₂CH₂CH₂CH₂CH₂SCF₃ | O |
| A-4762 | CH₂CF₃ | H | Me | Cl | CH₂CH₂NHS(=O)₂CF₃ | O |
| A-4763 | CH₂CF₃ | H | Me | Cl | CH₂CH₂CH₂NHS(=O)₂CF₃ | O |
| A-4764 | CH₂CF₃ | H | Me | Cl | CH₂CH₂CH₂CH₂NHS(=O)₂CF₃ | O |
| A-4765 | CH₂CF₃ | H | Me | Cl | CH₂CH₂CH₂CH₂CH₂NHS(=O)₂CF₃ | O |
| A-4766 | CH₂CF₃ | H | Me | Cl | CH₂CH₂N(Me)S(=O)₂CF₃ | O |
| A-4767 | CH₂CF₃ | H | Me | Cl | CH₂CH₂N(Et)S(=O)₂CF₃ | O |
| A-4768 | CH₂CF₃ | H | Me | Cl | CH₂CH₂N(CH₂CN)S(=O)₂CF₃ | O |
| A-4769 | CH₂CF₃ | H | Me | Cl | CH₂CH₂N(CH₂OMe)S(=O)₂CF₃ | O |
| A-4770 | CH₂CF₃ | H | Me | Cl | CH₂CH₂CH₂N(Me)S(=O)₂CF₃ | O |
| A-4771 | CH₂CF₃ | H | Me | Cl | CH₂CH₂CH₂N(Et)S(=O)₂CF₃ | O |
| A-4772 | CH₂CF₃ | H | Me | Cl | CH₂CH₂CH₂N(CH₂CN)S(=O)₂CF₃ | O |
| A-4773 | CH₂CF₃ | H | Me | Cl | CH₂CH₂CH₂N(CH₂OMe)S(=O)₂CF₃ | O |
| A-4774 | CH₂CF₃ | H | Me | Cl | CH₂CH₂CH₂CH₂N(Me)S(=O)₂CF₃ | O |
| A-4775 | CH₂CF₃ | H | Me | Cl | CH₂CH₂CH₂CH₂N(Et)S(=O)₂CF₃ | O |
| A-4776 | CH₂CF₃ | H | Me | Cl | CH₂CH₂CH₂CH₂N(CH₂CN)S(=O)₂CF₃ | O |
| A-4777 | CH₂CF₃ | H | Me | Cl | CH₂CH₂CH₂CH₂N(CH₂OMe)S(=O)₂CF₃ | O |
| A-4778 | CH₂CF₃ | H | Me | Me | CH₂CH₂SCF₃ | O |
| A-4779 | CH₂CF₃ | H | Me | Me | CH₂CH₂CH₂SCF₃ | O |
| A-4780 | CH₂CF₃ | H | Me | Me | CH₂CH₂CH₂CH₂SCF₃ | O |
| A-4781 | CH₂CF₃ | H | Me | Me | CH₂CH₂CH₂CH₂CH₂SCF₃ | O |
| A-4782 | CH₂CF₃ | H | Me | Me | CH₂CH₂CH₂CH₂CH₂CH₂SCF₃ | O |
| A-4783 | CH₂CF₃ | H | Me | Me | CH₂CH₂CH₂CH₂CH₂CH₂CH₂SCF₃ | O |
| A-4784 | CH₂CF₃ | H | Me | Me | CH₂CH₂CH₂CH₂CH₂CH₂CH₂CH₂SCF₃ | O |
| A-4785 | CH₂CF₃ | H | Me | Me | CH₂CH₂NHS(=O)₂CF₃ | O |
| A-4786 | CH₂CF₃ | H | Me | Me | CH₂CH₂CH₂NHS(=O)₂CF₃ | O |
| A-4787 | CH₂CF₃ | H | Me | Me | CH₂CH₂CH₂CH₂NHS(=O)₂CF₃ | O |
| A-4788 | CH₂CF₃ | H | Me | Me | CH₂CH₂CH₂CH₂CH₂NHS(=O)₂CF₃ | O |
| A-4789 | CH₂CF₃ | H | Me | Me | CH₂CH₂N(Me)S(=O)₂CF₃ | O |
| A-4790 | CH₂CF₃ | H | Me | Me | CH₂CH₂N(Et)S(=O)₂CF₃ | O |

**[Table 110]**

| Compound Number | R¹ | R² | R³ | R⁴ | R⁵ | A |
|---|---|---|---|---|---|---|
| A-4791 | CH₂CF₃ | H | Me | Me | CH₂CH₂N(CH₂CN)S(=O)₂CF₃ | O |
| A-4792 | CH₂CF₃ | H | Me | Me | CH₂CH₂N(CH₂OMe)S(=O)₂CF₃ | O |
| A-4793 | CH₂CF₃ | H | Me | Me | CH₂CH₂CH₂N(Me)S(=O)₂CF₃ | O |
| A-4794 | CH₂CF₃ | H | Me | Me | CH₂CH₂CH₂N(Et)S(=O)₂CF₃ | O |
| A-4795 | CH₂CF₃ | H | Me | Me | CH₂CH₂CH₂N(CH₂CN)S(=O)₂CF₃ | O |
| A-4796 | CH₂CF₃ | H | Me | Me | CH₂CH₂CH₂N(CH₂OMe)S(=O)₂CF₃ | O |
| A-4797 | CH₂CF₃ | H | Me | Me | CH₂CH₂CH₂CH₂N(Me)S(=O)₂CF₃ | O |
| A-4798 | CH₂CF₃ | H | Me | Me | CH₂CH₂CH₂CH₂N(Et)S(=O)₂CF₃ | O |
| A-4799 | CH₂CF₃ | H | Me | Me | CH₂CH₂CH₂CH₂N(CH₂CN)S(=O)₂CF₃ | O |
| A-4800 | CH₂CF₃ | H | Me | Me | CH₂CH₂CH₂CH₂N(CH₂OMe)S(=O)₂CF₃ | O |
| A-4801 | Et | H | Cl | H | CH₂CH₂SCF₃ | O |
| A-4802 | Et | H | Cl | H | CH₂CH₂CH₂SCF₃ | O |
| A-4803 | Et | H | Cl | H | CH₂CH₂CH₂CH₂SCF₃ | O |
| A-4804 | Et | H | Cl | H | CH₂CH₂CH₂CH₂CH₂SCF₃ | O |
| A-4805 | Et | H | Cl | H | CH₂CH₂CH₂CH₂CH₂CH₂SCF₃ | O |
| A-4806 | Et | H | Cl | H | CH₂CH₂CH₂CH₂CH₂CH₂CH₂SCF₃ | O |
| A-4807 | Et | H | Cl | H | CH₂CH₂CH₂CH₂CH₂CH₂CH₂CH₂SCF₃ | O |
| A-4808 | Et | H | Cl | H | CH₂CH₂NHS(=O)₂CF₃ | O |
| A-4809 | Et | H | Cl | H | CH₂CH₂CH₂NHS(=O)₂CF₃ | O |
| A-4810 | Et | H | Cl | H | CH₂CH₂CH₂CH₂NHS(=O)₂CF₃ | O |
| A-4811 | Et | H | Cl | H | CH₂CH₂CH₂CH₂CH₂NHS(=O)₂CF₃ | O |
| A-4812 | Et | H | Cl | H | CH₂CH₂N(Me)S(=O)₂CF₃ | O |
| A-4813 | Et | H | Cl | H | CH₂CH₂N(Et)S(=O)₂CF₃ | O |
| A-4814 | Et | H | Cl | H | CH₂CH₂N(CH₂CN)S(=O)₂CF₃ | O |
| A-4815 | Et | H | Cl | H | CH₂CH₂N(CH₂OMe)S(=O)₂CF₃ | O |
| A-4816 | Et | H | Cl | H | CH₂CH₂CH₂N(Me)S(=O)₂CF₃ | O |
| A-4817 | Et | H | Cl | H | CH₂CH₂CH₂N(Et)S(=O)₂CF₃ | O |
| A-4818 | Et | H | Cl | H | CH₂CH₂CH₂N(CH₂CN)S(=O)₂CF₃ | O |
| A-4819 | Et | H | Cl | H | CH₂CH₂CH₂N(CH₂OMe)S(=O)₂CF₃ | O |
| A-4820 | Et | H | Cl | H | CH₂CH₂CH₂CH₂N(Me)S(=O)₂CF₃ | O |
| A-4821 | Et | H | Cl | H | CH₂CH₂CH₂CH₂N(Et)S(=O)₂CF₃ | O |
| A-4822 | Et | H | Cl | H | CH₂CH₂CH₂CH₂N(CH₂CN)S(=O)₂CF₃ | O |
| A-4823 | Et | H | Cl | H | CH₂CH₂CH₂CH₂N(CH₂OMe)S(=O)₂CF₃ | O |
| A-4824 | Et | H | Me | H | CH₂CH₂SCF₃ | O |
| A-4825 | Et | H | Me | H | CH₂CH₂CH₂SCF₃ | O |
| A-4826 | Et | H | Me | H | CH₂CH₂CH₂CH₂SCF₃ | O |
| A-4827 | Et | H | Me | H | CH₂CH₂CH₂CH₂CH₂SCF₃ | O |
| A-4828 | Et | H | Me | H | CH₂CH₂CH₂CH₂CH₂CH₂SCF₃ | O |
| A-4829 | Et | H | Me | H | CH₂CH₂CH₂CH₂CH₂CH₂CH₂SCF₃ | O |
| A-4830 | Et | H | Me | H | CH₂CH₂CH₂CH₂CH₂CH₂CH₂CH₂SCF₃ | O |
| A-4831 | Et | H | Me | H | CH₂CH₂NHS(=O)₂CF₃ | O |
| A-4832 | Et | H | Me | H | CH₂CH₂CH₂NHS(=O)₂CF₃ | O |
| A-4833 | Et | H | Me | H | CH₂CH₂CH₂CH₂NHS(=O)₂CF₃ | O |
| A-4834 | Et | H | Me | H | CH₂CH₂CH₂CH₂CH₂NHS(=O)₂CF₃ | O |

**[Table 111]**

| Compound Number | R¹ | R² | R³ | R⁴ | R⁵ | A |
|---|---|---|---|---|---|---|
| A-4835 | Et | H | Me | H | CH₂CH₂N(Me)S(=O)₂CF₃ | O |
| A-4836 | Et | H | Me | H | CH₂CH₂N(Et)S(=O)₂CF₃ | O |
| A-4837 | Et | H | Me | H | CH₂CH₂N(CH₂CN)S(=O)₂CF₃ | O |
| A-4838 | Et | H | Me | H | CH₂CH₂N(CH₂OMe)S(=O)₂CF₃ | O |
| A-4839 | Et | H | Me | H | CH₂CH₂CH₂N(Me)S(=O)₂CF₃ | O |
| A-4840 | Et | H | Me | H | CH₂CH₂CH₂N(Et)S(=O)₂CF₃ | O |
| A-4841 | Et | H | Me | H | CH₂CH₂CH₂N(CH₂CN)S(=O)₂CF₃ | O |
| A-4842 | Et | H | Me | H | CH₂CH₂CH₂N(CH₂OMe)S(=O)₂CF₃ | O |
| A-4843 | Et | H | Me | H | CH₂CH₂CH₂CH₂N(Me)S(=O)₂CF₃ | O |
| A-4844 | Et | H | Me | H | CH₂CH₂CH₂CH₂N(Et)S(=O)₂CF₃ | O |
| A-4845 | Et | H | Me | H | CH₂CH₂CH₂CH₂N(CH₂CN)S(=O)₂CF₃ | O |
| A-4846 | Et | H | Me | H | CH₂CH₂CH₂CH₂N(CH₂OMe)S(=O)₂CF₃ | O |
| A-4847 | Et | H | Cl | F | CH₂CH₂SCF₃ | O |
| A-4848 | Et | H | Cl | F | CH₂CH₂CH₂SCF₃ | O |
| A-4849 | Et | H | Cl | F | CH₂CH₂CH₂CH₂SCF₃ | O |
| A-4850 | Et | H | Cl | F | CH₂CH₂CH₂CH₂CH₂SCF₃ | O |
| A-4851 | Et | H | Cl | F | CH₂CH₂CH₂CH₂CH₂CH₂SCF₃ | O |
| A-4852 | Et | H | Cl | F | CH₂CH₂CH₂CH₂CH₂CH₂CH₂SCF₃ | O |
| A-4853 | Et | H | Cl | F | CH₂CH₂CH₂CH₂CH₂CH₂CH₂CH₂SCF₃ | O |
| A-4854 | Et | H | Cl | F | CH₂CH₂NHS(=O)₂CF₃ | O |
| A-4855 | Et | H | Cl | F | CH₂CH₂CH₂NHS(=O)₂CF₃ | O |
| A-4856 | Et | H | Cl | F | CH₂CH₂CH₂CH₂NHS(=O)₂CF₃ | O |
| A-4857 | Et | H | Cl | F | CH₂CH₂CH₂CH₂CH₂NHS(=O)₂CF₃ | O |
| A-4858 | Et | H | Cl | F | CH₂CH₂N(Me)S(=O)₂CF₃ | O |
| A-4859 | Et | H | Cl | F | CH₂CH₂N(Et)S(=O)₂CF₃ | O |
| A-4860 | Et | H | Cl | F | CH₂CH₂N(CH₂CN)S(=O)₂CF₃ | O |
| A-4861 | Et | H | Cl | F | CH₂CH₂N(CH₂OMe)S(=O)₂CF₃ | O |
| A-4862 | Et | H | Cl | F | CH₂CH₂CH₂N(Me)S(=O)₂CF₃ | O |
| A-4863 | Et | H | Cl | F | CH₂CH₂CH₂N(Et)S(=O)₂CF₃ | O |
| A-4864 | Et | H | Cl | F | CH₂CH₂CH₂N(CH₂CN)S(=O)₂CF₃ | O |
| A-4865 | Et | H | Cl | F | CH₂CH₂CH₂N(CH₂OMe)S(=O)₂CF₃ | O |
| A-4866 | Et | H | Cl | F | CH₂CH₂CH₂CH₂N(Me)S(=O)₂CF₃ | O |
| A-4867 | Et | H | Cl | F | CH₂CH₂CH₂CH₂N(Et)S(=O)₂CF₃ | O |
| A-4868 | Et | H | Cl | F | CH₂CH₂CH₂CH₂N(CH₂CN)S(=O)₂CF₃ | O |
| A-4869 | Et | H | Cl | F | CH₂CH₂CH₂CH₂N(CH₂OMe)S(=O)₂CF₃ | O |
| A-4870 | Et | H | Me | F | CH₂CH₂SCF₃ | O |
| A-4871 | Et | H | Me | F | CH₂CH₂CH₂SCF₃ | O |
| A-4872 | Et | H | Me | F | CH₂CH₂CH₂CH₂SCF₃ | O |
| A-4873 | Et | H | Me | F | CH₂CH₂CH₂CH₂CH₂SCF₃ | O |
| A-4874 | Et | H | Me | F | CH₂CH₂CH₂CH₂CH₂CH₂SCF₃ | O |
| A-4875 | Et | H | Me | F | CH₂CH₂CH₂CH₂CH₂CH₂CH₂SCF₃ | O |
| A-4876 | Et | H | Me | F | CH₂CH₂CH₂CH₂CH₂CH₂CH₂CH₂SCF₃ | O |
| A-4877 | Et | H | Me | F | CH₂CH₂NHS(=O)₂CF₃ | O |
| A-4878 | Et | H | Me | F | CH₂CH₂CH₂NHS(=O)₂CF₃ | O |

**[Table 112]**

| Compound Number | R¹ | R² | R³ | R⁴ | R⁵ | A |
|---|---|---|---|---|---|---|
| A-4879 | Et | H | Me | F | CH₂CH₂CH₂CH₂NHS(=O)₂CF₃ | O |
| A-4880 | Et | H | Me | F | CH₂CH₂CH₂CH₂CH₂NHS(=O)₂CF₃ | O |
| A-4881 | Et | H | Me | F | CH₂CH₂N(Me)S(=O)₂CF₃ | O |
| A-4882 | Et | H | Me | F | CH₂CH₂N(Et)S(=O)₂CF₃ | O |
| A-4883 | Et | H | Me | F | CH₂CH₂N(CH₂CN)S(=O)₂CF₃ | O |
| A-4884 | Et | H | Me | F | CH₂CH₂N(CH₂OMe)S(=O)₂CF₃ | O |
| A-4885 | Et | H | Me | F | CH₂CH₂CH₂N(Me)S(=O)₂CF₃ | O |
| A-4886 | Et | H | Me | F | CH₂CH₂CH₂N(Et)S(=O)₂CF₃ | O |
| A-4887 | Et | H | Me | F | CH₂CH₂CH₂N(CH₂CN)S(=O)₂CF₃ | O |
| A-4888 | Et | H | Me | F | CH₂CH₂CH₂N(CH₂OMe)S(=O)₂CF₃ | O |
| A-4889 | Et | H | Me | F | CH₂CH₂CH₂CH₂N(Me)S(=O)₂CF₃ | O |
| A-4890 | Et | H | Me | F | CH₂CH₂CH₂CH₂N(Et)S(=O)₂CF₃ | O |
| A-4891 | Et | H | Me | F | CH₂CH₂CH₂CH₂N(CH₂CN)S(=O)₂CF₃ | O |
| A-4892 | Et | H | Me | F | CH₂CH₂CH₂CH₂N(CH₂OMe)S(=O)₂CF₃ | O |
| A-4893 | Et | H | Cl | Cl | CH₂CH₂SCF₃ | O |
| A-4894 | Et | H | Cl | Cl | CH₂CH₂CH₂SCF₃ | O |
| A-4895 | Et | H | Cl | Cl | CH₂CH₂CH₂CH₂SCF₃ | O |
| A-4896 | Et | H | Cl | Cl | CH₂CH₂CH₂CH₂CH₂SCF₃ | O |
| A-4897 | Et | H | Cl | Cl | CH₂CH₂CH₂CH₂CH₂CH₂SCF₃ | O |
| A-4898 | Et | H | Cl | Cl | CH₂CH₂CH₂CH₂CH₂CH₂CH₂SCF₃ | O |
| A-4899 | Et | H | Cl | Cl | CH₂CH₂CH₂CH₂CH₂CH₂CH₂CH₂SCF₃ | O |
| A-4900 | Et | H | Cl | Cl | CH₂CH₂NHS(=O)₂CF₃ | O |
| A-4901 | Et | H | Cl | Cl | CH₂CH₂CH₂NHS(=O)₂CF₃ | O |
| A-4902 | Et | H | Cl | Cl | CH₂CH₂CH₂CH₂NHS(=O)₂CF₃ | O |
| A-4903 | Et | H | Cl | Cl | CH₂CH₂CH₂CH₂CH₂NHS(=O)₂CF₃ | O |
| A-4904 | Et | H | Cl | Cl | CH₂CH₂N(Me)S(=O)₂CF₃ | O |
| A-4905 | Et | H | Cl | Cl | CH₂CH₂N(Et)S(=O)₂CF₃ | O |
| A-4906 | Et | H | Cl | Cl | CH₂CH₂N(CH₂CN)S(=O)₂CF₃ | O |
| A-4907 | Et | H | Cl | Cl | CH₂CH₂N(CH₂OMe)S(=O)₂CF₃ | O |
| A-4908 | Et | H | Cl | Cl | CH₂CH₂CH₂N(Me)S(=O)₂CF₃ | O |
| A-4909 | Et | H | Cl | Cl | CH₂CH₂CH₂N(Et)S(=O)₂CF₃ | O |
| A-4910 | Et | H | Cl | Cl | CH₂CH₂CH₂N(CH₂CN)S(=O)₂CF₃ | O |
| A-4911 | Et | H | Cl | Cl | CH₂CH₂CH₂N(CH₂OMe)S(=O)₂CF₃ | O |
| A-4912 | Et | H | Cl | Cl | CH₂CH₂CH₂CH₂N(Me)S(=O)₂CF₃ | O |
| A-4913 | Et | H | Cl | Cl | CH₂CH₂CH₂CH₂N(Et)S(=O)₂CF₃ | O |
| A-4914 | Et | H | Cl | Cl | CH₂CH₂CH₂CH₂N(CH₂CN)S(=O)₂CF₃ | O |
| A-4915 | Et | H | Cl | Cl | CH₂CH₂CH₂CH₂N(CH₂OMe)S(=O)₂CF₃ | O |
| A-4916 | Et | H | Me | Cl | CH₂CH₂SCF₃ | O |
| A-4917 | Et | H | Me | Cl | CH₂CH₂CH₂SCF₃ | O |
| A-4918 | Et | H | Me | Cl | CH₂CH₂CH₂CH₂SCF₃ | O |
| A-4919 | Et | H | Me | Cl | CH₂CH₂CH₂CH₂CH₂SCF₃ | O |
| A-4920 | Et | H | Me | Cl | CH₂CH₂CH₂CH₂CH₂CH₂SCF₃ | O |
| A-4921 | Et | H | Me | Cl | CH₂CH₂CH₂CH₂CH₂CH₂CH₂SCF₃ | O |
| A-4922 | Et | H | Me | Cl | CH₂CH₂CH₂CH₂CH₂CH₂CH₂CH₂SCF₃ | O |

**[Table 113]**

| Compound Number | R¹ | R² | R³ | R⁴ | R⁵ | A |
|---|---|---|---|---|---|---|
| A-4923 | Et | H | Me | Cl | CH₂CH₂NHS(=O)₂CF₃ | O |
| A-4924 | Et | H | Me | Cl | CH₂CH₂CH₂NHS(=O)₂CF₃ | O |
| A-4925 | Et | H | Me | Cl | CH₂CH₂CH₂CH₂NHS(=O)₂CF₃ | O |
| A-4926 | Et | H | Me | Cl | CH₂CH₂CH₂CH₂CH₂NHS(=O)₂CF₃ | O |
| A-4927 | Et | H | Me | Cl | CH₂CH₂N(Me)S(=O)₂CF₃ | O |
| A-4928 | Et | H | Me | Cl | CH₂CH₂N(Et)S(=O)₂CF₃ | O |
| A-4929 | Et | H | Me | Cl | CH₂CH₂N(CH₂CN)S(=O)₂CF₃ | O |
| A-4930 | Et | H | Me | Cl | CH₂CH₂N(CH₂OMe)S(=O)₂CF₃ | O |
| A-4931 | Et | H | Me | Cl | CH₂CH₂CH₂N(Me)S(=O)₂CF₃ | O |
| A-4932 | Et | H | Me | Cl | CH₂CH₂CH₂N(Et)S(=O)₂CF₃ | O |
| A-4933 | Et | H | Me | Cl | CH₂CH₂CH₂N(CH₂CN)S(=O)₂CF₃ | O |
| A-4934 | Et | H | Me | Cl | CH₂CH₂CH₂N(CH₂OMe)S(=O)₂CF₃ | O |
| A-4935 | Et | H | Me | Cl | CH₂CH₂CH₂CH₂N(Me)S(=O)₂CF₃ | O |
| A-4936 | Et | H | Me | Cl | CH₂CH₂CH₂CH₂N(Et)S(=O)₂CF₃ | O |
| A-4937 | Et | H | Me | Cl | CH₂CH₂CH₂CH₂N(CH₂CN)S(=O)₂CF₃ | O |
| A-4938 | Et | H | Me | Cl | CH₂CH₂CH₂CH₂N(CH₂OMe)S(=O)₂CF₃ | O |
| A-4939 | Et | H | Me | Me | CH₂CH₂SCF₃ | O |
| A-4940 | Et | H | Me | Me | CH₂CH₂CH₂SCF₃ | O |
| A-4941 | Et | H | Me | Me | CH₂CH₂CH₂CH₂SCF₃ | O |
| A-4942 | Et | H | Me | Me | CH₂CH₂CH₂CH₂CH₂SCF₃ | O |
| A-4943 | Et | H | Me | Me | CH₂CH₂CH₂CH₂CH₂CH₂SCF₃ | O |
| A-4944 | Et | H | Me | Me | CH₂CH₂CH₂CH₂CH₂CH₂CH₂SCF₃ | O |
| A-4945 | Et | H | Me | Me | CH₂CH₂CH₂CH₂CH₂CH₂CH₂CH₂SCF₃ | O |
| A-4946 | Et | H | Me | Me | CH₂CH₂NHS(=O)₂CF₃ | O |
| A-4947 | Et | H | Me | Me | CH₂CH₂CH₂NHS(=O)₂CF₃ | O |
| A-4948 | Et | H | Me | Me | CH₂CH₂CH₂CH₂NHS(=O)₂CF₃ | O |
| A-4949 | Et | H | Me | Me | CH₂CH₂CH₂CH₂CH₂NHS(=O)₂CF₃ | O |
| A-4950 | Et | H | Me | Me | CH₂CH₂N(Me)S(=O)₂CF₃ | O |
| A-4951 | Et | H | Me | Me | CH₂CH₂N(Et)S(=O)₂CF₃ | O |
| A-4952 | Et | H | Me | Me | CH₂CH₂N(CH₂CN)S(=O)₂CF₃ | O |
| A-4953 | Et | H | Me | Me | CH₂CH₂N(CH₂OMe)S(=O)₂CF₃ | O |
| A-4954 | Et | H | Me | Me | CH₂CH₂CH₂N(Me)S(=O)₂CF₃ | O |
| A-4955 | Et | H | Me | Me | CH₂CH₂CH₂N(Et)S(=O)₂CF₃ | O |
| A-4956 | Et | H | Me | Me | CH₂CH₂CH₂N(CH₂CN)S(=O)₂CF₃ | O |
| A-4957 | Et | H | Me | Me | CH₂CH₂CH₂N(CH₂OMe)S(=O)₂CF₃ | O |
| A-4958 | Et | H | Me | Me | CH₂CH₂CH₂CH₂N(Me)S(=O)₂CF₃ | O |
| A-4959 | Et | H | Me | Me | CH₂CH₂CH₂CH₂N(Et)S(=O)₂CF₃ | O |
| A-4960 | Et | H | Me | Me | CH₂CH₂CH₂CH₂N(CH₂CN)S(=O)₂CF₃ | O |
| A-4961 | Et | H | Me | Me | CH₂CH₂CH₂CH₂N(CH₂OMe)S(=O)₂CF₃ | O |
| A-4962 | *c*-Pr | H | Cl | H | CH₂CH₂SCF₃ | O |
| A-4963 | *c*-Pr | H | Cl | H | CH₂CH₂CH₂SCF₃ | O |
| A-4964 | *c*-Pr | H | Cl | H | CH₂CH₂CH₂CH₂SCF₃ | O |
| A-4965 | *c*-Pr | H | Cl | H | CH₂CH₂CH₂CH₂CH₂SCF₃ | O |
| A-4966 | *c*-Pr | H | Cl | H | CH₂CH₂CH₂CH₂CH₂CH₂SCF₃ | O |

**[Table 114]**

| Compound Number | R¹ | R² | R³ | R⁴ | R⁵ | A |
|---|---|---|---|---|---|---|
| A-4967 | *c*-Pr | H | Cl | H | CH₂CH₂CH₂CH₂CH₂CH₂CH₂SCF₃ | O |
| A-4968 | *c*-Pr | H | Cl | H | CH₂CH₂CH₂CH₂CH₂CH₂CH₂CH₂SCF₃ | O |
| A-4969 | *c*-Pr | H | Cl | H | CH₂CH₂NHS(=O)₂CF₃ | O |
| A-4970 | *c*-Pr | H | Cl | H | CH₂CH₂CH₂NHS(=O)₂CF₃ | O |
| A-4971 | *c*-Pr | H | Cl | H | CH₂CH₂CH₂CH₂NHS(=O)₂CF₃ | O |
| A-4972 | *c*-Pr | H | Cl | H | CH₂CH₂CH₂CH₂CH₂NHS(=O)₂CF₃ | O |
| A-4973 | *c*-Pr | H | Cl | H | CH₂CH₂N(Me)S(=O)₂CF₃ | O |
| A-4974 | *c*-Pr | H | Cl | H | CH₂CH₂N(Et)S(=O)₂CF₃ | O |
| A-4975 | *c*-Pr | H | Cl | H | CH₂CH₂N(CH₂CN)S(=O)₂CF₃ | O |
| A-4976 | *c*-Pr | H | Cl | H | CH₂CH₂N(CH₂OMe)S(=O)₂CF₃ | O |
| A-4977 | *c*-Pr | H | Cl | H | CH₂CH₂CH₂N(Me)S(=O)₂CF₃ | O |
| A-4978 | *c*-Pr | H | Cl | H | CH₂CH₂CH₂N(Et)S(=O)₂CF₃ | O |
| A-4979 | *c*-Pr | H | Cl | H | CH₂CH₂CH₂N(CH₂CN)S(=O)₂CF₃ | O |
| A-4980 | *c*-Pr | H | Cl | H | CH₂CH₂CH₂N(CH₂OMe)S(=O)₂CF₃ | O |
| A-4981 | *c*-Pr | H | Cl | H | CH₂CH₂CH₂CH₂N(Me)S(=O)₂CF₃ | O |
| A-4982 | *c*-Pr | H | Cl | H | CH₂CH₂CH₂CH₂N(Et)S(=O)₂CF₃ | O |
| A-4983 | *c*-Pr | H | Cl | H | CH₂CH₂CH₂CH₂N(CH₂CN)S(=O)₂CF₃ | O |
| A-4984 | *c*-Pr | H | Cl | H | CH₂CH₂CH₂CH₂N(CH₂OMe)S(=O)₂CF₃ | O |
| A-4985 | *c*-Pr | H | Me | H | CH₂CH₂SCF₃ | O |
| A-4986 | *c*-Pr | H | Me | H | CH₂CH₂CH₂SCF₃ | O |
| A-4987 | *c*-Pr | H | Me | H | CH₂CH₂CH₂CH₂SCF₃ | O |
| A-4988 | *c*-Pr | H | Me | H | CH₂CH₂CH₂CH₂CH₂SCF₃ | O |
| A-4989 | *c*-Pr | H | Me | H | CH₂CH₂CH₂CH₂CH₂CH₂SCF₃ | O |
| A-4990 | *c*-Pr | H | Me | H | CH₂CH₂CH₂CH₂CH₂CH₂CH₂SCF₃ | O |
| A-4991 | *c*-Pr | H | Me | H | CH₂CH₂CH₂CH₂CH₂CH₂CH₂CH₂SCF₃ | O |
| A-4992 | *c*-Pr | H | Me | H | CH₂CH₂NHS(=O)₂CF₃ | O |
| A-4993 | *c*-Pr | H | Me | H | CH₂CH₂CH₂NHS(=O)₂CF₃ | O |
| A-4994 | *c*-Pr | H | Me | H | CH₂CH₂CH₂CH₂NHS(=O)₂CF₃ | O |
| A-4995 | *c*-Pr | H | Me | H | CH₂CH₂CH₂CH₂CH₂NHS(=O)₂CF₃ | O |
| A-4996 | *c*-Pr | H | Me | H | CH₂CH₂N(Me)S(=O)₂CF₃ | O |
| A-4997 | *c*-Pr | H | Me | H | CH₂CH₂N(Et)S(=O)₂CF₃ | O |
| A-4998 | *c*-Pr | H | Me | H | CH₂CH₂N(CH₂CN)S(=O)₂CF₃ | O |
| A-4999 | *c*-Pr | H | Me | H | CH₂CH₂N(CH₂₀Me)S(=O)₂CF₃ | O |
| A-5000 | *c*-Pr | H | Me | H | CH₂CH₂CH₂N(Me)S(=O)₂CF₃ | O |
| A-5001 | *c*-Pr | H | Me | H | CH₂CH₂CH₂N(Et)S(=O)₂CF₃ | O |
| A-5002 | *c*-Pr | H | Me | H | CH₂CH₂CH₂N(CH₂CN)S(=O)₂CF₃ | O |
| A-5003 | *c*-Pr | H | Me | H | CH₂CH₂CH₂N(CH₂OMe)S(=O)₂CF₃ | O |
| A-5004 | *c*-Pr | H | Me | H | CH₂CH₂CH₂CH₂N(Me)S(=O)₂CF₃ | O |
| A-5005 | *c*-Pr | H | Me | H | CH₂CH₂CH₂CH₂N(Et)S(=O)₂CF₃ | O |
| A-5006 | *c*-Pr | H | Me | H | CH₂CH₂CH₂CH₂N(CH₂CN)S(=O)₂CF₃ | O |
| A-5007 | *c*-Pr | H | Me | H | CH₂CH₂CH₂CH₂N(CH₂OMe)S(=O)₂CF₃ | O |
| A-5008 | *c*-Pr | H | Cl | F | CH₂CH₂SCF₃ | O |
| A-5009 | *c*-Pr | H | Cl | F | CH₂CH₂CH₂SCF₃ | O |
| A-5010 | *c*-Pr | H | Cl | F | CH₂CH₂CH₂CH₂SCF₃ | O |

**[Table 115]**

| Compound Number | R¹ | R² | R³ | R⁴ | R⁵ | A |
|---|---|---|---|---|---|---|
| A-5011 | *c*-Pr | H | Cl | F | CH₂CH₂CH₂CH₂CH₂SCF₃ | O |
| A-5012 | *c*-Pr | H | Cl | F | CH₂CH₂CH₂CH₂CH₂CH₂SCF₃ | O |
| A-5013 | *c*-Pr | H | Cl | F | CH₂CH₂CH₂CH₂CH₂CH₂CH₂SCF₃ | O |
| A-5014 | *c*-Pr | H | Cl | F | CH₂CH₂CH₂CH₂CH₂CH₂CH₂CH₂SCF₃ | O |
| A-5015 | *c*-Pr | H | Cl | F | CH₂CH₂NHS(=O)₂CF₃ | O |
| A-5016 | *c*-Pr | H | Cl | F | CH₂CH₂CH₂NHS(=O)₂CF₃ | O |
| A-5017 | *c*-Pr | H | Cl | F | CH₂CH₂CH₂CH₂NHS(=O)₂CF₃ | O |
| A-5018 | *c*-Pr | H | Cl | F | CH₂CH₂CH₂CH₂CH₂NHS(=O)₂CF₃ | O |
| A-5019 | *c*-Pr | H | Cl | F | CH₂CH₂N(Me)S(=O)₂CF₃ | O |
| A-5020 | *c*-Pr | H | Cl | F | CH₂CH₂N(Et)S(=O)₂CF₃ | O |
| A-5021 | *c*-Pr | H | Cl | F | CH₂CH₂N(CH₂CN)S(=O)₂CF₃ | O |
| A-5022 | *c*-Pr | H | Cl | F | CH₂CH₂N(CH₂OMe)S(=O)₂CF₃ | O |
| A-5023 | *c*-Pr | H | Cl | F | CH₂CH₂CH₂N(Me)S(=O)₂CF₃ | O |
| A-5024 | *c*-Pr | H | Cl | F | CH₂CH₂CH₂N(Et)S(=O)₂CF₃ | O |
| A-5025 | *c*-Pr | H | Cl | F | CH₂CH₂CH₂N(CH₂CN)S(=O)₂CF₃ | O |
| A-5026 | *c*-Pr | H | Cl | F | CH₂CH₂CH₂N(CH₂OMe)S(=O)₂CF₃ | O |
| A-5027 | *c*-Pr | H | Cl | F | CH₂CH₂CH₂CH₂N(Me)S(=O)₂CF₃ | O |
| A-5028 | *c*-Pr | H | Cl | F | CH₂CH₂CH₂CH₂N(Et)S(=O)₂CF₃ | O |
| A-5029 | *c*-Pr | H | Cl | F | CH₂CH₂CH₂CH₂N(CH₂CN)S(=O)₂CF₃ | O |
| A-5030 | *c*-Pr | H | Cl | F | CH₂CH₂CH₂CH₂N(CH₂OMe)S(=O)₂CF₃ | O |
| A-5031 | *c*-Pr | H | Me | F | CH₂CH₂SCF₃ | O |
| A-5032 | *c*-Pr | H | Me | F | CH₂CH₂CH₂SCF₃ | O |
| A-5033 | *c*-Pr | H | Me | F | CH₂CH₂CH₂CH₂SCF₃ | O |
| A-5034 | *c*-Pr | H | Me | F | CH₂CH₂CH₂CH₂CH₂SCF₃ | O |
| A-5035 | *c*-Pr | H | Me | F | CH₂CH₂CH₂CH₂CH₂CH₂SCF₃ | O |
| A-5036 | *c*-Pr | H | Me | F | CH₂CH₂CH₂CH₂CH₂CH₂CH₂SCF₃ | O |
| A-5037 | *c*-Pr | H | Me | F | CH₂CH₂CH₂CH₂CH₂CH₂CH₂CH₂SCF₃ | O |
| A-5038 | *c*-Pr | H | Me | F | CH₂CH₂NHS(=O)₂CF₃ | O |
| A-5039 | *c*-Pr | H | Me | F | CH₂CH₂CH₂NHS(=O)₂CF₃ | O |
| A-5040 | *c*-Pr | H | Me | F | CH₂CH₂CH₂CH₂NHS(=O)₂CF₃ | O |
| A-5041 | *c*-Pr | H | Me | F | CH₂CH₂CH₂CH₂CH₂NHS(=O)₂CF₃ | O |
| A-5042 | *c*-Pr | H | Me | F | CH₂CH₂N(Me)S(=O)₂CF₃ | O |
| A-5043 | *c*-Pr | H | Me | F | CH₂CH₂N(Et)S(=O)₂CF₃ | O |
| A-5044 | *c*-Pr | H | Me | F | CH₂CH₂N(CH₂CN)S(=O)₂CF₃ | O |
| A-5045 | *c*-Pr | H | Me | F | CH₂CH₂N(CH₂OMe)S(=O)₂CF₃ | O |
| A-5046 | *c*-Pr | H | Me | F | CH₂CH₂CH₂N(Me)S(=O)₂CF₃ | O |
| A-5047 | *c*-Pr | H | Me | F | CH₂CH₂CH₂N(Et)S(=O)₂CF₃ | O |
| A-5048 | *c*-Pr | H | Me | F | CH₂CH₂CH₂N(CH₂CN)S(=O)₂CF₃ | O |
| A-5049 | *c*-Pr | H | Me | F | CH₂CH₂CH₂N(CH₂OMe)S(=O)₂CF₃ | O |
| A-5050 | *c*-Pr | H | Me | F | CH₂CH₂CH₂CH₂N(Me)S(=O)₂CF₃ | O |
| A-5051 | *c*-Pr | H | Me | F | CH₂CH₂CH₂CH₂N(Et)S(=O)₂CF₃ | O |
| A-5052 | *c*-Pr | H | Me | F | CH₂CH₂CH₂CH₂N(CH₂CN)S(=O)₂CF₃ | O |
| A-5053 | *c*-Pr | H | Me | F | CH₂CH₂CH₂CH₂N(CH₂OMe)S(=O)₂CF₃ | O |
| A-5054 | *c*-Pr | H | Cl | Cl | CH₂CH₂SCF₃ | O |

**[Table 116]**

| Compound Number | R¹ | R² | R³ | R⁴ | R5 | A |
|---|---|---|---|---|---|---|
| A-5055 | *c*-Pr | H | Cl | Cl | CH₂CH₂CH₂SCF₃ | O |
| A-5056 | *c*-Pr | H | Cl | Cl | CH₂CH₂CH₂CH₂SCF₃ | O |
| A-5057 | *c*-Pr | H | Cl | Cl | CH₂CH₂CH₂CH₂CH₂SCF₃ | O |
| A-5058 | *c*-Pr | H | Cl | Cl | CH₂CH₂CH₂CH₂CH₂CH₂SCF₃ | O |
| A-5059 | *c*-Pr | H | Cl | Cl | CH₂CH₂CH₂CH₂CH₂CH₂CH₂SCF₃ | O |
| A-5060 | *c*-Pr | H | Cl | Cl | CH₂CH₂CH₂CH₂CH₂CH₂CH₂CH₂SCF₃ | O |
| A-5061 | *c*-Pr | H | Cl | Cl | CH₂CH₂NHS(=O)₂CF₃ | O |
| A-5062 | *c*-Pr | H | Cl | Cl | CH₂CH₂CH₂NHS(=O)₂CF₃ | O |
| A-5063 | *c*-Pr | H | Cl | Cl | CH₂CH₂CH₂CH₂NHS(=O)₂CF₃ | O |
| A-5064 | *c*-Pr | H | Cl | Cl | CH₂CH₂CH₂CH₂CH₂NHS(=O)₂CF₃ | O |
| A-5065 | *c*-Pr | H | Cl | Cl | CH₂CH₂N(Me)S(=O)₂CF₃ | O |
| A-5066 | *c*-Pr | H | Cl | Cl | CH₂CH₂N(Et)S(=O)₂CF₃ | O |
| A-5067 | *c*-Pr | H | Cl | Cl | CH₂CH₂N(CH₂CN)S(=O)₂CF₃ | O |
| A-5068 | *c*-Pr | H | Cl | Cl | CH₂CH₂N(CH₂OMe)S(=O)₂CF₃ | O |
| A-5069 | *c*-Pr | H | Cl | Cl | CH₂CH₂CH₂N(Me)S(=O)₂CF₃ | O |
| A-5070 | *c*-Pr | H | Cl | Cl | CH₂CH₂CH₂N(Et)S(=O)₂CF₃ | O |
| A-5071 | *c*-Pr | H | Cl | Cl | CH₂CH₂CH₂N(CH₂CN)S(=O)₂CF₃ | O |
| A-5072 | *c*-Pr | H | Cl | Cl | CH₂CH₂CH₂N(CH₂OMe)S(=O)₂CF₃ | O |
| A-5073 | *c*-Pr | H | Cl | Cl | CH₂CH₂CH₂CH₂N(Me)S(=O)₂CF₃ | O |
| A-5074 | *c*-Pr | H | Cl | Cl | CH₂CH₂CH₂CH₂N(Et)S(=O)₂CF₃ | O |
| A-5075 | *c*-Pr | H | Cl | Cl | CH₂CH₂CH₂CH₂N(CH₂CN)S(=O)₂CF₃ | O |
| A-5076 | *c*-Pr | H | Cl | Cl | CH₂CH₂CH₂CH₂N(CH₂OMe)S(=O)₂CF₃ | O |
| A-5077 | *c*-Pr | H | Me | Cl | CH₂CH₂SCF₃ | O |
| A-5078 | *c*-Pr | H | Me | Cl | CH₂CH₂CH₂SCF₃ | O |
| A-5079 | *c*-Pr | H | Me | Cl | CH₂CH₂CH₂CH₂SCF₃ | O |
| A-5080 | *c*-Pr | H | Me | Cl | CH₂CH₂CH₂CH₂CH₂SCF₃ | O |
| A-5081 | *c*-Pr | H | Me | Cl | CH₂CH₂CH₂CH₂CH₂CH₂SCF₃ | O |
| A-5082 | *c*-Pr | H | Me | Cl | CH₂CH₂CH₂CH₂CH₂CH₂CH₂SCF₃ | O |
| A-5083 | *c*-Pr | H | Me | Cl | CH₂CH₂CH₂CH₂CH₂CH₂CH₂CH₂SCF₃ | O |
| A-5084 | *c*-Pr | H | Me | Cl | CH₂CH₂NHS(=O)₂CF₃ | O |
| A-5085 | *c*-Pr | H | Me | Cl | CH₂CH₂CH₂NHS(=O)₂CF₃ | O |
| A-5086 | *c*-Pr | H | Me | Cl | CH₂CH₂CH₂CH₂NHS(=O)₂CF₃ | O |
| A-5087 | *c*-Pr | H | Me | Cl | CH₂CH₂CH₂CH₂CH₂NHS(=O)₂CF₃ | O |
| A-5088 | *c*-Pr | H | Me | Cl | CH₂CH₂N(Me)S(=O)₂CF₃ | O |
| A-5089 | *c*-Pr | H | Me | Cl | CH₂CH₂N(Et)S(=O)₂CF₃ | O |
| A-5090 | *c*-Pr | H | Me | Cl | CH₂CH₂N(CH₂CN)S(=O)₂CF₃ | O |
| A-5091 | *c*-Pr | H | Me | Cl | CH₂CH₂N(CH₂OMe)S(=O)₂CF₃ | O |
| A-5092 | *c*-Pr | H | Me | Cl | CH₂CH₂CH₂N(Me)S(=O)₂CF₃ | O |
| A-5093 | *c*-Pr | H | Me | Cl | CH₂CH₂CH₂N(Et)S(=O)₂CF₃ | O |
| A-5094 | *c*-Pr | H | Me | Cl | CH₂CH₂CH₂N(CH₂CN)S(=O)₂CF₃ | O |
| A-5095 | *c*-Pr | H | Me | Cl | CH₂CH₂CH₂N(CH₂OMe)S(=O)₂CF₃ | O |
| A-5096 | *c*-Pr | H | Me | Cl | CH₂CH₂CH₂CH₂N(Me)S(=O)₂CF₃ | O |
| A-5097 | *c*-Pr | H | Me | Cl | CH₂CH₂CH₂CH₂N(Et)S(=O)₂CF₃ | O |
| A-5098 | *c*-Pr | H | Me | Cl | CH₂CH₂CH₂CH₂N(CH₂CN)S(=O)₂CF₃ | O |

**[Table 117]**

| Compound Number | R¹ | R² | R³ | R⁴ | R⁵ | A |
|---|---|---|---|---|---|---|
| A-5099 | *c*-Pr | H | Me | Cl | CH₂CH₂CH₂CH₂N(CH₂OMe)S(=O)₂CF₃ | O |
| A-5100 | *c*-Pr | H | Me | Me | CH₂CH₂SCF₃ | O |
| A-5101 | *c*-Pr | H | Me | Me | CH₂CH₂CH₂SCF₃ | O |
| A-5102 | *c*-Pr | H | Me | Me | CH₂CH₂CH₂CH₂SCF₃ | O |
| A-5103 | *c*-Pr | H | Me | Me | CH₂CH₂CH₂CH₂CH₂SCF₃ | O |
| A-5104 | *c*-Pr | H | Me | Me | CH₂CH₂CH₂CH₂CH₂CH₂SCF₃ | O |
| A-5105 | *c*-Pr | H | Me | Me | CH₂CH₂CH₂CH₂CH₂CH₂CH₂SCF₃ | O |
| A-5106 | *c*-Pr | H | Me | Me | CH₂CH₂CH₂CH₂CH₂CH₂CH₂CH₂SCF₃ | O |
| A-5107 | *c*-Pr | H | Me | Me | CH₂CH₂NHS(=O)₂CF₃ | O |
| A-5108 | *c*-Pr | H | Me | Me | CH₂CH₂CH₂NHS(=O)₂CF₃ | O |
| A-5109 | *c*-Pr | H | Me | Me | CH₂CH₂CH₂CH₂NHS(=O)₂CF₃ | O |
| A-5110 | *c*-Pr | H | Me | Me | CH₂CH₂CH₂CH₂CH₂NHS(=O)₂CF₃ | O |
| A-5111 | *c*-Pr | H | Me | Me | CH₂CH₂N(Me)S(=O)₂CF₃ | O |
| A-5112 | *c*-Pr | H | Me | Me | CH₂CH₂N(Et)S(=O)₂CF₃ | O |
| A-5113 | *c*-Pr | H | Me | Me | CH₂CH₂N(CH₂CN)S(=O)₂CF₃ | O |
| A-5114 | *c*-Pr | H | Me | Me | CH₂CH₂N(CH₂OMe)S(=O)₂CF₃ | O |
| A-5115 | *c*-Pr | H | Me | Me | CH₂CH₂CH₂N(Me)S(=O)₂CF₃ | O |
| A-5116 | *c*-Pr | H | Me | Me | CH₂CH₂CH₂N(Et)S(=O)₂CF₃ | O |
| A-5117 | *c*-Pr | H | Me | Me | CH₂CH₂CH₂N(CH₂CN)S(=O)₂CF₃ | O |
| A-5118 | *c*-Pr | H | Me | Me | CH₂CH₂CH₂N(CH₂OMe)S(=O)₂CF₃ | O |
| A-5119 | *c*-Pr | H | Me | Me | CH₂CH₂CH₂CH₂N(Me)S(=O)₂CF₃ | O |
| A-5120 | *c*-Pr | H | Me | Me | CH₂CH₂CH₂CH₂N(Et)S(=O)₂CF₃ | O |
| A-5121 | *c*-Pr | H | Me | Me | CH₂CH₂CH₂CH₂N(CH₂CN)S(=O)₂CF₃ | O |
| A-5122 | *c*-Pr | H | Me | Me | CH₂CH₂CH₂CH₂N(CH₂OMe)S(=O)₂CF₃ | O |
| A-5123 | CH₂CF₃ | H | Cl | H | CH₂CH₂SCF₃ | S |
| A-5124 | CH₂CF₃ | H | Cl | H | CH₂CH₂CH₂SCF₃ | S |
| A-5125 | CH₂CF₃ | H | Cl | H | CH₂CH₂CH₂CH₂SCF₃ | S |
| A-5126 | CH₂CF₃ | H | Cl | H | CH₂CH₂CH₂CH₂CH₂SCF₃ | S |
| A-5127 | CH₂CF₃ | H | Cl | H | CH₂CH₂CH₂CH₂CH₂CH₂SCF₃ | S |
| A-5128 | CH₂CF₃ | H | Cl | H | CH₂CH₂CH₂CH₂CH₂CH₂CH₂SCF₃ | S |
| A-5129 | CH₂CF₃ | H | Cl | H | CH₂CH₂CH₂CH₂CH₂CH₂CH₂CH₂SCF₃ | S |
| A-5130 | CH₂CF₃ | H | Cl | H | CH₂CH₂NHS(=O)₂CF₃ | S |
| A-5131 | CH₂CF₃ | H | Cl | H | CH₂CH₂CH₂NHS(=O)₂CF₃ | S |
| A-5132 | CH₂CF₃ | H | Cl | H | CH₂CH₂CH₂CH₂NHS(=O)₂CF₃ | S |
| A-5133 | CH₂CF₃ | H | Cl | H | CH₂CH₂CH₂CH₂CH₂NHS(=O)₂CF₃ | S |
| A-5134 | CH₂CF₃ | H | Cl | H | CH₂CH₂N(Me)S(=O)₂CF₃ | S |
| A-5135 | CH₂CF₃ | H | Cl | H | CH₂CH₂N(Et)S(=O)₂CF₃ | S |
| A-5136 | CH₂CF₃ | H | Cl | H | CH₂CH₂N(CH₂CN)S(=O)₂CF₃ | S |
| A-5137 | CH₂CF₃ | H | Cl | H | CH₂CH₂N(CH₂OMe)S(=O)₂CF₃ | S |
| A-5138 | CH₂CF₃ | H | Cl | H | CH₂CH₂CH₂N(Me)S(=O)₂CF₃ | S |
| A-5139 | CH₂CF₃ | H | Cl | H | CH₂CH₂CH₂N(Et)S(=O)₂CF₃ | S |
| A-5140 | CH₂CF₃ | H | Cl | H | CH₂CH₂CH₂N(CH₂CN)S(=O)₂CF₃ | S |
| A-5141 | CH₂CF₃ | H | Cl | H | CH₂CH₂CH₂N(CH₂OMe)S(=O)₂CF₃ | S |
| A-5142 | CH₂CF₃ | H | Cl | H | CH₂CH₂CH₂CH₂N(Me)S(=O)₂CF₃ | S |

**[Table 118]**

| Compound Number | R¹ | R² | R³ | R⁴ | R⁵ | A |
|---|---|---|---|---|---|---|
| A-5143 | CH₂CF₃ | H | Cl | H | CH₂CH₂CH₂CH₂N(Et)S(=O)₂CF₃ | S |
| A-5144 | CH₂CF₃ | H | Cl | H | CH₂CH₂CH₂CH₂N(CH₂CN)S(=O)₂CF₃ | S |
| A-5145 | CH₂CF₃ | H | Cl | H | CH₂CH₂CH₂CH₂N(CH₂OMe)S(=O)₂CF₃ | S |
| A-5146 | CH₂CF₃ | H | Me | H | CH₂CH₂SCF₃ | S |
| A-5147 | CH₂CF₃ | H | Me | H | CH₂CH₂CH₂SCF₃ | S |
| A-5148 | CH₂CF₃ | H | Me | H | CH₂CH₂CH₂CH₂SCF₃ | S |
| A-5149 | CH₂CF₃ | H | Me | H | CH₂CH₂CH₂CH₂CH₂SCF₃ | S |
| A-5150 | CH₂CF₃ | H | Me | H | CH₂CH₂CH₂CH₂CH₂CH₂SCF₃ | S |
| A-5151 | CH₂CF₃ | H | Me | H | CH₂CH₂CH₂CH₂CH₂CH₂CH₂SCF₃ | S |
| A-5152 | CH₂CF₃ | H | Me | H | CH₂CH₂CH₂CH₂CH₂CH₂CH₂CH₂SCF₃ | S |
| A-5153 | CH₂CF₃ | H | Me | H | CH₂CH₂NHS(=O)₂CF₃ | S |
| A-5154 | CH₂CF₃ | H | Me | H | CH₂CH₂CH₂NHS(=O)₂CF₃ | S |
| A-5155 | CH₂CF₃ | H | Me | H | CH₂CH₂CH₂CH₂NHS(=O)₂CF₃ | S |
| A-5156 | CH₂CF₃ | H | Me | H | CH₂CH₂CH₂CH₂CH₂NHS(=O)₂CF₃ | S |
| A-5157 | CH₂CF₃ | H | Me | H | CH₂CH₂N(Me)S(=O)₂CF₃ | S |
| A-5158 | CH₂CF₃ | H | Me | H | CH₂CH₂N(Et)S(=O)₂CF₃ | S |
| A-5159 | CH₂CF₃ | H | Me | H | CH₂CH₂N(CH₂CN)S(=O)₂CF₃ | S |
| A-5160 | CH₂CF₃ | H | Me | H | CH₂CH₂N(CH₂OMe)S(=O)₂CF₃ | S |
| A-5161 | CH₂CF₃ | H | Me | H | CH₂CH₂CH₂N(Me)S(=O)₂CF₃ | S |
| A-5162 | CH₂CF₃ | H | Me | H | CH₂CH₂CH₂N(Et)S(=O)₂CF₃ | S |
| A-5163 | CH₂CF₃ | H | Me | H | CH₂CH₂CH₂N(CH₂CN)S(=O)₂CF₃ | S |
| A-5164 | CH₂CF₃ | H | Me | H | CH₂CH₂CH₂N(CH₂OMe)S(=O)₂CF₃ | S |
| A-5165 | CH₂CF₃ | H | Me | H | CH₂CH₂CH₂CH₂N(Me)S(=O)₂CF₃ | S |
| A-5166 | CH₂CF₃ | H | Me | H | CH₂CH₂CH₂CH₂N(Et)S(=O)₂CF₃ | S |
| A-5167 | CH₂CF₃ | H | Me | H | CH₂CH₂CH₂CH₂N(CH₂CN)S(=O)₂CF₃ | S |
| A-5168 | CH₂CF₃ | H | Me | H | CH₂CH₂CH₂CH₂N(CH₂OMe)S(=O)₂CF₃ | S |
| A-5169 | CH₂CF₃ | H | Cl | F | CH₂CH₂SCF₃ | S |
| A-5170 | CH₂CF₃ | H | Cl | F | CH₂CH₂CH₂SCF₃ | S |
| A-5171 | CH₂CF₃ | H | Cl | F | CH₂CH₂CH₂CH₂SCF₃ | S |
| A-5172 | CH₂CF₃ | H | Cl | F | CH₂CH₂CH₂CH₂CH₂SCF₃ | S |
| A-5173 | CH₂CF₃ | H | Cl | F | CH₂CH₂CH₂CH₂CH₂CH₂SCF₃ | S |
| A-5174 | CH₂CF₃ | H | Cl | F | CH₂CH₂CH₂CH₂CH₂CH₂CH₂SCF₃ | S |
| A-5175 | CH₂CF₃ | H | Cl | F | CH₂CH₂CH₂CH₂CH₂CH₂CH₂CH₂SCF₃ | S |
| A-5176 | CH₂CF₃ | H | Cl | F | CH₂CH₂NHS(=O)₂CF₃ | S |
| A-5177 | CH₂CF₃ | H | Cl | F | CH₂CH₂CH₂NHS(=O)₂CF₃ | S |
| A-5178 | CH₂CF₃ | H | Cl | F | CH₂CH₂CH₂CH₂NHS(=O)₂CF₃ | S |
| A-5179 | CH₂CF₃ | H | Cl | F | CH₂CH₂CH₂CH₂CH₂NHS(=O)₂CF₃ | S |
| A-5180 | CH₂CF₃ | H | Cl | F | CH₂CH₂N(Me)S(=O)₂CF₃ | S |
| A-5181 | CH₂CF₃ | H | Cl | F | CH₂CH₂N(Et)S(=O)₂CF₃ | S |
| A-5182 | CH₂CF₃ | H | Cl | F | CH₂CH₂N(CH₂CN)S(=O)₂CF₃ | S |
| A-5183 | CH₂CF₃ | H | Cl | F | CH₂CH₂N(CH₂OMe)S(=O)₂CF₃ | S |
| A-5184 | CH₂CF₃ | H | Cl | F | CH₂CH₂CH₂N(Me)S(=O)₂CF₃ | S |
| A-5185 | CH₂CF₃ | H | Cl | F | CH₂CH₂CH₂N(Et)S(=O)₂CF₃ | S |
| A-5186 | CH₂CF₃ | H | Cl | F | CH₂CH₂CH₂N(CH₂CN)S(=O)₂CF₃ | S |

**[Table 119]**

| Compound Number | R¹ | R² | R³ | R⁴ | R⁵ | A |
|---|---|---|---|---|---|---|
| A-5187 | CH₂CF₃ | H | Cl | F | CH₂CH₂CH₂N(CH₂OMe)S(=O)₂CF₃ | S |
| A-5188 | CH₂CF₃ | H | Cl | F | CH₂CH₂CH₂CH₂N(Me)S(=O)₂CF₃ | S |
| A-5189 | CH₂CF₃ | H | Cl | F | CH₂CH₂CH₂CH₂N(Et)S(=O)₂CF₃ | S |
| A-5190 | CH₂CF₃ | H | Cl | F | CH₂CH₂CH₂CH₂N(CH₂CN)S(=O)₂CF₃ | S |
| A-5191 | CH₂CF₃ | H | Cl | F | CH₂CH₂CH₂CH₂N(CH₂OMe)S(=O)₂CF₃ | S |
| A-5192 | CH₂CF₃ | H | Me | F | CH₂CH₂SCF₃ | S |
| A-5193 | CH₂CF₃ | H | Me | F | CH₂CH₂CH₂SCF₃ | S |
| A-5194 | CH₂CF₃ | H | Me | F | CH₂CH₂CH₂CH₂SCF₃ | S |
| A-5195 | CH₂CF₃ | H | Me | F | CH₂CH₂CH₂CH₂CH₂SCF₃ | S |
| A-5196 | CH₂CF₃ | H | Me | F | CH₂CH₂CH₂CH₂CH₂CH₂SCF₃ | S |
| A-5197 | CH₂CF₃ | H | Me | F | CH₂CH₂CH₂CH₂CH₂CH₂CH₂SCF₃ | S |
| A-5198 | CH₂CF₃ | H | Me | F | CH₂CH₂CH₂CH₂CH₂CH₂CH₂CH₂SCF₃ | S |
| A-5199 | CH₂CF₃ | H | Me | F | CH₂CH₂NHS(=O)₂CF₃ | S |
| A-5200 | CH₂CF₃ | H | Me | F | CH₂CH₂CH₂NHS(=O)₂CF₃ | S |
| A-5201 | CH₂CF₃ | H | Me | F | CH₂CH₂CH₂CH₂NHS(=O)₂CF₃ | S |
| A-5202 | CH₂CF₃ | H | Me | F | CH₂CH₂CH₂CH₂CH₂NHS(=O)₂CF₃ | S |
| A-5203 | CH₂CF₃ | H | Me | F | CH₂CH₂N(Me)S(=O)₂CF₃ | S |
| A-5204 | CH₂CF₃ | H | Me | F | CH₂CH₂N(Et)S(=O)₂CF₃ | S |
| A-5205 | CH₂CF₃ | H | Me | F | CH₂CH₂N(CH₂CN)S(=O)₂CF₃ | S |
| A-5206 | CH₂CF₃ | H | Me | F | CH₂CH₂N(CH₂OMe)S(=O)₂CF₃ | S |
| A-5207 | CH₂CF₃ | H | Me | F | CH₂CH₂CH₂N(Me)S(=O)₂CF₃ | S |
| A-5208 | CH₂CF₃ | H | Me | F | CH₂CH₂CH₂N(Et)S(=O)₂CF₃ | S |
| A-5209 | CH₂CF₃ | H | Me | F | CH₂CH₂CH₂N(CH₂CN)S(=O)₂CF₃ | S |
| A-5210 | CH₂CF₃ | H | Me | F | CH₂CH₂CH₂N(CH₂OMe)S(=O)₂CF₃ | S |
| A-5211 | CH₂CF₃ | H | Me | F | CH₂CH₂CH₂CH₂N(Me)S(=O)₂CF₃ | S |
| A-5212 | CH₂CF₃ | H | Me | F | CH₂CH₂CH₂CH₂N(Et)S(=O)₂CF₃ | S |
| A-5213 | CH₂CF₃ | H | Me | F | CH₂CH₂CH₂CH₂N(CH₂CN)S(=O)₂CF₃ | S |
| A-5214 | CH₂CF₃ | H | Me | F | CH₂CH₂CH₂CH₂N(CH₂OMe)S(=O)₂CF₃ | S |
| A-5215 | CH₂CF₃ | H | Cl | Cl | CH₂CH₂SCF₃ | S |
| A-5216 | CH₂CF₃ | H | Cl | Cl | CH₂CH₂CH₂SCF₃ | S |
| A-5217 | CH₂CF₃ | H | Cl | Cl | CH₂CH₂CH₂CH₂SCF₃ | S |
| A-5218 | CH₂CF₃ | H | Cl | Cl | CH₂CH₂CH₂CH₂CH₂SCF₃ | S |
| A-5219 | CH₂CF₃ | H | Cl | Cl | CH₂CH₂CH₂CH₂CH₂CH₂SCF₃ | S |
| A-5220 | CH₂CF₃ | H | Cl | Cl | CH₂CH₂CH₂CH₂CH₂CH₂CH₂SCF₃ | S |
| A-5221 | CH₂CF₃ | H | Cl | Cl | CH₂CH₂CH₂CH₂CH₂CH₂CH₂CH₂SCF₃ | S |
| A-5222 | CH₂CF₃ | H | Cl | Cl | CH₂CH₂NHS(=O)₂CF₃ | S |
| A-5223 | CH₂CF₃ | H | Cl | Cl | CH₂CH₂CH₂NHS(=O)₂CF₃ | S |
| A-5224 | CH₂CF₃ | H | Cl | Cl | CH₂CH₂CH₂CH₂NHS(=O)₂CF₃ | S |
| A-5225 | CH₂CF₃ | H | Cl | Cl | CH₂CH₂CH₂CH₂CH₂NHS(=O)₂CF₃ | S |
| A-5226 | CH₂CF₃ | H | Cl | Cl | CH₂CH₂N(Me)S(=O)₂CF₃ | S |
| A-5227 | CH₂CF₃ | H | Cl | Cl | CH₂CH₂N(Et)S(=O)₂CF₃ | S |
| A-5228 | CH₂CF₃ | H | Cl | Cl | CH₂CH₂N(CH₂CN)S(=O)₂CF₃ | S |
| A-5229 | CH₂CF₃ | H | Cl | Cl | CH₂CH₂N(CH₂OMe)S(=O)₂CF₃ | S |
| A-5230 | CH₂CF₃ | H | Cl | Cl | CH₂CH₂CH₂N(Me)S(=O)₂CF₃ | S |

**[Table 120]**

| Compound Number | R¹ | R² | R³ | R⁴ | R⁵ | A |
|---|---|---|---|---|---|---|
| A-5231 | CH₂CF₃ | H | Cl | Cl | CH₂CH₂CH₂N(Et)S(=O)₂CF₃ | S |
| A-5232 | CH₂CF₃ | H | Cl | Cl | CH₂CH₂CH₂N(CH₂CN)S(=O)₂CF₃ | S |
| A-5233 | CH₂CF₃ | H | Cl | Cl | CH₂CH₂CH₂N(CH₂OMe)S(=O)₂CF₃ | S |
| A-5234 | CH₂CF₃ | H | Cl | Cl | CH₂CH₂CH₂CH₂N(Me)S(=O)₂CF₃ | S |
| A-5235 | CH₂CF₃ | H | Cl | Cl | CH₂CH₂CH₂CH₂N(Et)S(=O)₂CF₃ | S |
| A-5236 | CH₂CF₃ | H | Cl | Cl | CH₂CH₂CH₂CH₂N(CH₂CN)S(=O)₂CF₃ | S |
| A-5237 | CH₂CF₃ | H | Cl | Cl | CH₂CH₂CH₂CH₂N(CH₂OMe)S(=O)₂CF₃ | S |
| A-5238 | CH₂CF₃ | H | Me | Cl | CH₂CH₂SCF₃ | S |
| A-5239 | CH₂CF₃ | H | Me | Cl | CH₂CH₂CH₂SCF₃ | S |
| A-5240 | CH₂CF₃ | H | Me | Cl | CH₂CH₂CH₂CH₂SCF₃ | S |
| A-5241 | CH₂CF₃ | H | Me | Cl | CH₂CH₂CH₂CH₂CH₂SCF₃ | S |
| A-5242 | CH₂CF₃ | H | Me | Cl | CH₂CH₂CH₂CH₂CH₂CH₂SCF₃ | S |
| A-5243 | CH₂CF₃ | H | Me | Cl | CH₂CH₂CH₂CH₂CH₂CH₂CH₂SCF₃ | S |
| A-5244 | CH₂CF₃ | H | Me | Cl | CH₂CH₂CH₂CH₂CH₂CH₂CH₂CH₂SCF₃ | S |
| A-5245 | CH₂CF₃ | H | Me | Cl | CH₂CH₂NHS(=O)₂CF₃ | S |
| A-5246 | CH₂CF₃ | H | Me | Cl | CH₂CH₂CH₂NHS(=O)₂CF₃ | S |
| A-5247 | CH₂CF₃ | H | Me | Cl | CH₂CH₂CH₂CH₂NHS(=O)₂CF₃ | S |
| A-5248 | CH₂CF₃ | H | Me | Cl | CH₂CH₂CH₂CH₂CH₂NHS(=O)₂CF₃ | S |
| A-5249 | CH₂CF₃ | H | Me | Cl | CH₂CH₂N(Me)S(=O)₂CF₃ | S |
| A-5250 | CH₂CF₃ | H | Me | Cl | CH₂CH₂N(Et)S(=O)₂CF₃ | S |
| A-5251 | CH₂CF₃ | H | Me | Cl | CH₂CH₂N(CH₂CN)S(=O)₂CF₃ | S |
| A-5252 | CH₂CF₃ | H | Me | Cl | CH₂CH₂N(CH₂OMe)S(=O)₂CF₃ | S |
| A-5253 | CH₂CF₃ | H | Me | Cl | CH₂CH₂CH₂N(Me)S(=O)₂CF₃ | S |
| A-5254 | CH₂CF₃ | H | Me | Cl | CH₂CH₂CH₂N(Et)S(=O)₂CF₃ | S |
| A-5255 | CH₂CF₃ | H | Me | Cl | CH₂CH₂CH₂N(CH₂CN)S(=O)₂CF₃ | S |
| A-5256 | CH₂CF₃ | H | Me | Cl | CH₂CH₂CH₂N(CH₂OMe)S(=O)₂CF₃ | S |
| A-5257 | CH₂CF₃ | H | Me | Cl | CH₂CH₂CH₂CH₂N(Me)S(=O)₂CF₃ | S |
| A-5258 | CH₂CF₃ | H | Me | Cl | CH₂CH₂CH₂CH₂N(Et)S(=O)₂CF₃ | S |
| A-5259 | CH₂CF₃ | H | Me | Cl | CH₂CH₂CH₂CH₂N(CH₂CN)S(=O)₂CF₃ | S |
| A-5260 | CH₂CF₃ | H | Me | Cl | CH₂CH₂CH₂CH₂N(CH₂OMe)S(=O)₂CF₃ | S |
| A-5261 | CH₂CF₃ | H | Me | Me | CH₂CH₂SCF₃ | S |
| A-5262 | CH₂CF₃ | H | Me | Me | CH₂CH₂CH₂SCF₃ | S |
| A-5263 | CH₂CF₃ | H | Me | Me | CH₂CH₂CH₂CH₂SCF₃ | S |
| A-5264 | CH₂CF₃ | H | Me | Me | CH₂CH₂CH₂CH₂CH₂SCF₃ | S |
| A-5265 | CH₂CF₃ | H | Me | Me | CH₂CH₂CH₂CH₂CH₂CH₂SCF₃ | S |
| A-5266 | CH₂CF₃ | H | Me | Me | CH₂CH₂CH₂CH₂CH₂CH₂CH₂SCF₃ | S |
| A-5267 | CH₂CF₃ | H | Me | Me | CH₂CH₂CH₂CH₂CH₂CH₂CH₂CH₂SCF₃ | S |
| A-5268 | CH₂CF₃ | H | Me | Me | CH₂CH₂NHS(=O)₂CF₃ | S |
| A-5269 | CH₂CF₃ | H | Me | Me | CH₂CH₂CH₂NHS(=O)₂CF₃ | S |
| A-5270 | CH₂CF₃ | H | Me | Me | CH₂CH₂CH₂CH₂NHS(=O)₂CF₃ | S |
| A-5271 | CH₂CF₃ | H | Me | Me | CH₂CH₂CH₂CH₂CH₂NHS(=O)₂CF₃ | S |
| A-5272 | CH₂CF₃ | H | Me | Me | CH₂CH₂N(Me)S(=O)₂CF₃ | S |
| A-5273 | CH₂CF₃ | H | Me | Me | CH₂CH₂N(Et)S(=O)₂CF₃ | S |
| A-5274 | CH₂CF₃ | H | Me | Me | CH₂CH₂N(CH₂CN)S(=O)₂CF₃ | S |

**[Table 121]**

| Compound Number | R¹ | R² | R³ | R⁴ | R⁵ | A |
|---|---|---|---|---|---|---|
| A-5275 | CH₂CF₃ | H | Me | Me | CH₂CH₂N(CH₂OMe)S(=O)₂CF₃ | S |
| A-5276 | CH₂CF₃ | H | Me | Me | CH₂CH₂CH₂N(Me)S(=O)₂CF₃ | S |
| A-5277 | CH₂CF₃ | H | Me | Me | CH₂CH₂CH₂N(Et)S(=O)₂CF₃ | S |
| A-5278 | CH₂CF₃ | H | Me | Me | CH₂CH₂CH₂N(CH₂CN)S(=O)₂CF₃ | S |
| A-5279 | CH₂CF₃ | H | Me | Me | CH₂CH₂CH₂N(CH₂OMe)S(=O)₂CF₃ | S |
| A-5280 | CH₂CF₃ | H | Me | Me | CH₂CH₂CH₂CH₂N(Me)S(=O)₂CF₃ | S |
| A-5281 | CH₂CF₃ | H | Me | Me | CH₂CH₂CH₂CH₂N(Et)S(=O)₂CF₃ | S |
| A-5282 | CH₂CF₃ | H | Me | Me | CH₂CH₂CH₂CH₂N(CH₂CN)S(=O)₂CF₃ | S |
| A-5283 | CH₂CF₃ | H | Me | Me | CH₂CH₂CH₂CH₂N(CH₂OMe)S(=O)₂CF₃ | S |
| A-5284 | Et | H | Cl | H | CH₂CH₂SCF₃ | S |
| A-5285 | Et | H | Cl | H | CH₂CH₂CH₂SCF₃ | S |
| A-5286 | Et | H | Cl | H | CH₂CH₂CH₂CH₂SCF₃ | S |
| A-5287 | Et | H | Cl | H | CH₂CH₂CH₂CH₂CH₂SCF₃ | S |
| A-5288 | Et | H | Cl | H | CH₂CH₂CH₂CH₂CH₂CH₂SCF₃ | S |
| A-5289 | Et | H | Cl | H | CH₂CH₂CH₂CH₂CH₂CH₂CH₂SCF₃ | S |
| A-5290 | Et | H | Cl | H | CH₂CH₂CH₂CH₂CH₂CH₂CH₂CH₂SCF₃ | S |
| A-5291 | Et | H | Cl | H | CH₂CH₂NHS(=O)₂CF₃ | S |
| A-5292 | Et | H | Cl | H | CH₂CH₂CH₂NHS(=O)₂CF₃ | S |
| A-5293 | Et | H | Cl | H | CH₂CH₂CH₂CH₂NHS(=O)₂CF₃ | S |
| A-5294 | Et | H | Cl | H | CH₂CH₂CH₂CH₂CH₂NHS(=O)₂CF₃ | S |
| A-5295 | Et | H | Cl | H | CH₂CH₂N(Me)S(=O)₂CF₃ | S |
| A-5296 | Et | H | Cl | H | CH₂CH₂N(Et)S(=O)₂CF₃ | S |
| A-5297 | Et | H | Cl | H | CH₂CH₂N(CH₂CN)S(=O)₂CF₃ | S |
| A-5298 | Et | H | Cl | H | CH₂CH₂N(CH₂OMe)S(=O)₂CF₃ | S |
| A-5299 | Et | H | Cl | H | CH₂CH₂CH₂N(Me)S(=O)₂CF₃ | S |
| A-5300 | Et | H | Cl | H | CH₂CH₂CH₂N(Et)S(=O)₂CF₃ | S |
| A-5301 | Et | H | Cl | H | CH₂CH₂CH₂N(CH₂CN)S(=O)₂CF₃ | S |
| A-5302 | Et | H | Cl | H | CH₂CH₂CH₂N(CH₂OMe)S(=O)₂CF₃ | S |
| A-5303 | Et | H | Cl | H | CH₂CH₂CH₂CH₂N(Me)S(=O)₂CF₃ | S |
| A-5304 | Et | H | Cl | H | CH₂CH₂CH₂CH₂N(Et)S(=O)₂CF₃ | S |
| A-5305 | Et | H | Cl | H | CH₂CH₂CH₂CH₂N(CH₂CN)S(=O)₂CF₃ | S |
| A-5306 | Et | H | Cl | H | CH₂CH₂CH₂CH₂N(CH₂OMe)S(=O)₂CF₃ | S |
| A-5307 | Et | H | Me | H | CH₂CH₂SCF₃ | S |
| A-5308 | Et | H | Me | H | CH₂CH₂CH₂SCF₃ | S |
| A-5309 | Et | H | Me | H | CH₂CH₂CH₂CH₂SCF₃ | S |
| A-5310 | Et | H | Me | H | CH₂CH₂CH₂CH₂CH₂SCF₃ | S |
| A-5311 | Et | H | Me | H | CH₂CH₂CH₂CH₂CH₂CH₂SCF₃ | S |
| A-5312 | Et | H | Me | H | CH₂CH₂CH₂CH₂CH₂CH₂CH₂SCF₃ | S |
| A-5313 | Et | H | Me | H | CH₂CH₂CH₂CH₂CH₂CH₂CH₂CH₂SCF₃ | S |
| A-5314 | Et | H | Me | H | CH₂CH₂NHS(=O)₂CF₃ | S |
| A-5315 | Et | H | Me | H | CH₂CH₂CH₂NHS(=O)₂CF₃ | S |
| A-5316 | Et | H | Me | H | CH₂CH₂CH₂CH₂NHS(=O)₂CF₃ | S |
| A-5317 | Et | H | Me | H | CH₂CH₂CH₂CH₂CH₂NHS(=O)₂CF₃ | S |
| A-5318 | Et | H | Me | H | CH₂CH₂N(Me)S(=O)₂CF₃ | S |

**[Table 122]**

| Compound Number | R¹ | R² | R³ | R⁴ | R⁵ | A |
|---|---|---|---|---|---|---|
| A-5319 | Et | H | Me | H | CH₂CH₂N(Et)S(=O)₂CF₃ | S |
| A-5320 | Et | H | Me | H | CH₂CH₂N(CH₂CN)S(=O)₂CF₃ | S |
| A-5321 | Et | H | Me | H | CH₂CH₂N(CH₂OMe)S(=O)₂CF₃ | S |
| A-5322 | Et | H | Me | H | CH₂CH₂CH₂N(Me)S(=O)₂CF₃ | S |
| A-5323 | Et | H | Me | H | CH₂CH₂CH₂N(Et)S(=O)₂CF₃ | S |
| A-5324 | Et | H | Me | H | CH₂CH₂CH₂N(CH₂CN)S(=O)₂CF₃ | S |
| A-5325 | Et | H | Me | H | CH₂CH₂CH₂N(CH₂OMe)S(=O)₂CF₃ | S |
| A-5326 | Et | H | Me | H | CH₂CH₂CH₂CH₂N(Me)S(=O)₂CF₃ | S |
| A-5327 | Et | H | Me | H | CH₂CH₂CH₂CH₂N(Et)S(=O)₂CF₃ | S |
| A-5328 | Et | H | Me | H | CH₂CH₂CH₂CH₂N(CH₂CN)S(=O)₂CF₃ | S |
| A-5329 | Et | H | Me | H | CH₂CH₂CH₂CH₂N(CH₂OMe)S(=O)₂CF₃ | S |
| A-5330 | Et | H | Cl | F | CH₂CH₂SCF₃ | S |
| A-5331 | Et | H | Cl | F | CH₂CH₂CH₂SCF₃ | S |
| A-5332 | Et | H | Cl | F | CH₂CH₂CH₂CH₂SCF₃ | S |
| A-5333 | Et | H | Cl | F | CH₂CH₂CH₂CH₂CH₂SCF₃ | S |
| A-5334 | Et | H | Cl | F | CH₂CH₂CH₂CH₂CH₂CH₂SCF₃ | S |
| A-5335 | Et | H | Cl | F | CH₂CH₂CH₂CH₂CH₂CH₂CH₂SCF₃ | S |
| A-5336 | Et | H | Cl | F | CH₂CH₂CH₂CH₂CH₂CH₂CH₂CH₂SCF₃ | S |
| A-5337 | Et | H | Cl | F | CH₂CH₂NHS(=O)₂CF₃ | S |
| A-5338 | Et | H | Cl | F | CH₂CH₂CH₂NHS(=O)₂CF₃ | S |
| A-5339 | Et | H | Cl | F | CH₂CH₂CH₂CH₂NHS(=O)₂CF₃ | S |
| A-5340 | Et | H | Cl | F | CH₂CH₂CH₂CH₂CH₂NHS(=O)₂CF₃ | S |
| A-5341 | Et | H | Cl | F | CH₂CH₂N(Me)S(=O)₂CF₃ | S |
| A-5342 | Et | H | Cl | F | CH₂CH₂N(Et)S(=O)₂CF₃ | S |
| A-5343 | Et | H | Cl | F | CH₂CH₂N(CH₂CN)S(=O)₂CF₃ | S |
| A-5344 | Et | H | Cl | F | CH₂CH₂N(CH₂OMe)S(=O)₂CF₃ | S |
| A-5345 | Et | H | Cl | F | CH₂CH₂CH₂N(Me)S(=O)₂CF₃ | S |
| A-5346 | Et | H | Cl | F | CH₂CH₂CH₂N(Et)S(=O)₂CF₃ | S |
| A-5347 | Et | H | Cl | F | CH₂CH₂CH₂N(CH₂CN)S(=O)₂CF₃ | S |
| A-5348 | Et | H | Cl | F | CH₂CH₂CH₂N(CH₂OMe)S(=O)₂CF₃ | S |
| A-5349 | Et | H | Cl | F | CH₂CH₂CH₂CH₂N(Me)S(=O)₂CF₃ | S |
| A-5350 | Et | H | Cl | F | CH₂CH₂CH₂CH₂N(Et)S(=O)₂CF₃ | S |
| A-5351 | Et | H | Cl | F | CH₂CH₂CH₂CH₂N(CH₂CN)S(=O)₂CF₃ | S |
| A-5352 | Et | H | Cl | F | CH₂CH₂CH₂CH₂N(CH₂OMe)S(=O)₂CF₃ | S |
| A-5353 | Et | H | Me | F | CH₂CH₂SCF₃ | S |
| A-5354 | Et | H | Me | F | CH₂CH₂CH₂SCF₃ | S |
| A-5355 | Et | H | Me | F | CH₂CH₂CH₂CH₂SCF₃ | S |
| A-5356 | Et | H | Me | F | CH₂CH₂CH₂CH₂CH₂SCF₃ | S |
| A-5357 | Et | H | Me | F | CH₂CH₂CH₂CH₂CH₂CH₂SCF₃ | S |
| A-5358 | Et | H | Me | F | CH₂CH₂CH₂CH₂CH₂CH₂CH₂SCF₃ | S |
| A-5359 | Et | H | Me | F | CH₂CH₂CH₂CH₂CH₂CH₂CH₂CH₂SCF₃ | S |
| A-5360 | Et | H | Me | F | CH₂CH₂NHS(=O)₂CF₃ | S |
| A-5361 | Et | H | Me | F | CH₂CH₂CH₂NHS(=O)₂CF₃ | S |
| A-5362 | Et | H | Me | F | CH₂CH₂CH₂CH₂NHS(=O)₂CF₃ | S |

**[Table 123]**

| Compound Number | R¹ | R² | R³ | R⁴ | R⁵ | A |
|---|---|---|---|---|---|---|
| A-5363 | Et | H | Me | F | CH₂CH₂CH₂CH₂CH₂NHS(=O)₂CF₃ | S |
| A-5364 | Et | H | Me | F | CH₂CH₂N(Me)S(=O)₂CF₃ | S |
| A-5365 | Et | H | Me | F | CH₂CH₂N(Et)S(=O)₂CF₃ | S |
| A-5366 | Et | H | Me | F | CH₂CH₂N(CH₂CN)S(=O)₂CF₃ | S |
| A-5367 | Et | H | Me | F | CH₂CH₂N(CH₂OMe)S(=O)₂CF₃ | S |
| A-5368 | Et | H | Me | F | CH₂CH₂CH₂N(Me)S(=O)₂CF₃ | S |
| A-5369 | Et | H | Me | F | CH₂CH₂CH₂N(Et)S(=O)₂CF₃ | S |
| A-5370 | Et | H | Me | F | CH₂CH₂CH₂N(CH₂CN)S(=O)₂CF₃ | S |
| A-5371 | Et | H | Me | F | CH₂CH₂CH₂N(CH₂OMe)S(=O)₂CF₃ | S |
| A-5372 | Et | H | Me | F | CH₂CH₂CH₂CH₂N(Me)S(=O)₂CF₃ | S |
| A-5373 | Et | H | Me | F | CH₂CH₂CH₂CH₂N(Et)S(=O)₂CF₃ | S |
| A-5374 | Et | H | Me | F | CH₂CH₂CH₂CH₂N(CH₂CN)S(=O)₂CF₃ | S |
| A-5375 | Et | H | Me | F | CH₂CH₂CH₂CH₂N(CH₂OMe)S(=O)₂CF₃ | S |
| A-5376 | Et | H | Cl | Cl | CH₂CH₂SCF₃ | S |
| A-5377 | Et | H | Cl | Cl | CH₂CH₂CH₂SCF₃ | S |
| A-5378 | Et | H | Cl | Cl | CH₂CH₂CH₂CH₂SCF₃ | S |
| A-5379 | Et | H | Cl | Cl | CH₂CH₂CH₂CH₂CH₂SCF₃ | S |
| A-5380 | Et | H | Cl | Cl | CH₂CH₂CH₂CH₂CH₂CH₂SCF₃ | S |
| A-5381 | Et | H | Cl | Cl | CH₂CH₂CH₂CH₂CH₂CH₂CH₂SCF₃ | S |
| A-5382 | Et | H | Cl | Cl | CH₂CH₂CH₂CH₂CH₂CH₂CH₂CH₂SCF₃ | S |
| A-5383 | Et | H | Cl | Cl | CH₂CH₂NHS(=O)₂CF₃ | S |
| A-5384 | Et | H | Cl | Cl | CH₂CH₂CH₂NHS(=O)₂CF₃ | S |
| A-5385 | Et | H | Cl | Cl | CH₂CH₂CH₂CH₂NHS(=O)₂CF₃ | S |
| A-5386 | Et | H | Cl | Cl | CH₂CH₂CH₂CH₂CH₂NHS(=O)₂CF₃ | S |
| A-5387 | Et | H | Cl | Cl | CH₂CH₂N(Me)S(=O)₂CF₃ | S |
| A-5388 | Et | H | Cl | Cl | CH₂CH₂N(Et)S(=O)₂CF₃ | S |
| A-5389 | Et | H | Cl | Cl | CH₂CH₂N(CH₂CN)S(=O)₂CF₃ | S |
| A-5390 | Et | H | Cl | Cl | CH₂CH₂N(CH₂OMe)S(=O)₂CF₃ | S |
| A-5391 | Et | H | Cl | Cl | CH₂CH₂CH₂N(Me)S(=O)₂CF₃ | S |
| A-5392 | Et | H | Cl | Cl | CH₂CH₂CH₂N(Et)S(=O)₂CF₃ | S |
| A-5393 | Et | H | Cl | Cl | CH₂CH₂CH₂N(CH₂CN)S(=O)₂CF₃ | S |
| A-5394 | Et | H | Cl | Cl | CH₂CH₂CH₂N(CH₂OMe)S(=O)₂CF₃ | S |
| A-5395 | Et | H | Cl | Cl | CH₂CH₂CH₂CH₂N(Me)S(=O)₂CF₃ | S |
| A-5396 | Et | H | Cl | Cl | CH₂CH₂CH₂CH₂N(Et)S(=O)₂CF₃ | S |
| A-5397 | Et | H | Cl | Cl | CH₂CH₂CH₂CH₂N(CH₂CN)S(=O)₂CF₃ | S |
| A-5398 | Et | H | Cl | Cl | CH₂CH₂CH₂CH₂N(CH₂OMe)S(=O)₂CF₃ | S |
| A-5399 | Et | H | Me | Cl | CH₂CH₂SCF₃ | S |
| A-5400 | Et | H | Me | Cl | CH₂CH₂CH₂SCF₃ | S |
| A-5401 | Et | H | Me | Cl | CH₂CH₂CH₂CH₂SCF₃ | S |
| A-5402 | Et | H | Me | Cl | CH₂CH₂CH₂CH₂CH₂SCF₃ | S |
| A-5403 | Et | H | Me | Cl | CH₂CH₂CH₂CH₂CH₂CH₂SCF₃ | S |
| A-5404 | Et | H | Me | Cl | CH₂CH₂CH₂CH₂CH₂CH₂CH₂SCF₃ | S |
| A-5405 | Et | H | Me | Cl | CH₂CH₂CH₂CH₂CH₂CH₂CH₂CH₂SCF₃ | S |
| A-5406 | Et | H | Me | Cl | CH₂CH₂NHS(=O)₂CF₃ | S |

**[Table 124]**

| Compound Number | R¹ | R² | R³ | R⁴ | R⁵ | A |
|---|---|---|---|---|---|---|
| A-5407 | Et | H | Me | Cl | CH₂CH₂CH₂NHS(=O)₂CF₃ | S |
| A-5408 | Et | H | Me | Cl | CH₂CH₂CH₂CH₂NHS(=O)₂CF₃ | S |
| A-5409 | Et | H | Me | Cl | CH₂CH₂CH₂CH₂CH₂NHS(=O)₂CF₃ | S |
| A-5410 | Et | H | Me | Cl | CH₂CH₂N(Me)S(=O)₂CF₃ | S |
| A-5411 | Et | H | Me | Cl | CH₂CH₂N(Et)S(=O)₂CF₃ | S |
| A-5412 | Et | H | Me | Cl | CH₂CH₂N(CH₂CN)S(=O)₂CF₃ | S |
| A-5413 | Et | H | Me | Cl | CH₂CH₂N(CH₂OMe)S(=O)₂CF₃ | S |
| A-5414 | Et | H | Me | Cl | CH₂CH₂CH₂N(Me)S(=O)₂CF₃ | S |
| A-5415 | Et | H | Me | Cl | CH₂CH₂CH₂N(Et)S(=O)₂CF₃ | S |
| A-5416 | Et | H | Me | Cl | CH₂CH₂CH₂N(CH₂CN)S(=O)₂CF₃ | S |
| A-5417 | Et | H | Me | Cl | CH₂CH₂CH₂N(CH₂OMe)S(=O)₂CF₃ | S |
| A-5418 | Et | H | Me | Cl | CH₂CH₂CH₂CH₂N(Me)S(=O)₂CF₃ | S |
| A-5419 | Et | H | Me | Cl | CH₂CH₂CH₂CH₂N(Et)S(=O)₂CF₃ | S |
| A-5420 | Et | H | Me | Cl | CH₂CH₂CH₂CH₂N(CH₂CN)S(=O)₂CF₃ | S |
| A-5421 | Et | H | Me | Cl | CH₂CH₂CH₂CH₂N(CH₂OMe)S(=O)₂CF₃ | S |
| A-5422 | Et | H | Me | Me | CH₂CH₂SCF₃ | S |
| A-5423 | Et | H | Me | Me | CH₂CH₂CH₂SCF₃ | S |
| A-5424 | Et | H | Me | Me | CH₂CH₂CH₂CH₂SCF₃ | S |
| A-5425 | Et | H | Me | Me | CH₂CH₂CH₂CH₂CH₂SCF₃ | S |
| A-5426 | Et | H | Me | Me | CH₂CH₂CH₂CH₂CH₂CH₂SCF₃ | S |
| A-5427 | Et | H | Me | Me | CH₂CH₂CH₂CH₂CH₂CH₂CH₂SCF₃ | S |
| A-5428 | Et | H | Me | Me | CH₂CH₂CH₂CH₂CH₂CH₂CH₂CH₂SCF₃ | S |
| A-5429 | Et | H | Me | Me | CH₂CH₂NHS(=O)₂CF₃ | S |
| A-5430 | Et | H | Me | Me | CH₂CH₂CH₂NHS(=O)₂CF₃ | S |
| A-5431 | Et | H | Me | Me | CH₂CH₂CH₂CH₂NHS(=O)₂CF₃ | S |
| A-5432 | Et | H | Me | Me | CH₂CH₂CH₂CH₂CH₂NHS(=O)₂CF₃ | S |
| A-5433 | Et | H | Me | Me | CH₂CH₂N(Me)S(=O)₂CF₃ | S |
| A-5434 | Et | H | Me | Me | CH₂CH₂N(Et)S(=O)₂CF₃ | S |
| A-5435 | Et | H | Me | Me | CH₂CH₂N(CH₂CN)S(=O)₂CF₃ | S |
| A-5436 | Et | H | Me | Me | CH₂CH₂N(CH₂OMe)S(=O)₂CF₃ | S |
| A-5437 | Et | H | Me | Me | CH₂CH₂CH₂N(Me)S(=O)₂CF₃ | S |
| A-5438 | Et | H | Me | Me | CH₂CH₂CH₂N(Et)S(=O)₂CF₃ | S |
| A-5439 | Et | H | Me | Me | CH₂CH₂CH₂N(CH₂CN)S(=O)₂CF₃ | S |
| A-5440 | Et | H | Me | Me | CH₂CH₂CH₂N(CH₂OMe)S(=O)₂CF₃ | S |
| A-5441 | Et | H | Me | Me | CH₂CH₂CH₂CH₂N(Me)S(=O)₂CF₃ | S |
| A-5442 | Et | H | Me | Me | CH₂CH₂CH₂CH₂N(Et)S(=O)₂CF₃ | S |
| A-5443 | Et | H | Me | Me | CH₂CH₂CH₂CH₂N(CH₂CN)S(=O)₂CF₃ | S |
| A-5444 | Et | H | Me | Me | CH₂CH₂CH₂CH₂N(CH₂OMe)S(=O)₂CF₃ | S |
| A-5445 | *c*-Pr | H | Cl | H | CH₂CH₂SCF₃ | S |
| A-5446 | *c*-Pr | H | Cl | H | CH₂CH₂CH₂SCF₃ | S |
| A-5447 | *c*-Pr | H | Cl | H | CH₂CH₂CH₂CH₂SCF₃ | S |
| A-5448 | *c*-Pr | H | Cl | H | CH₂CH₂CH₂CH₂CH₂SCF₃ | S |
| A-5449 | *c*-Pr | H | Cl | H | CH₂CH₂CH₂CH₂CH₂CH₂SCF₃ | S |
| A-5450 | *c*-Pr | H | Cl | H | CH₂CH₂CH₂CH₂CH₂CH₂CH₂SCF₃ | S |

**[Table 125]**

| Compound Number | R¹ | R² | R³ | R⁴ | R⁵ | A |
|---|---|---|---|---|---|---|
| A-5451 | *c*-Pr | H | Cl | H | CH₂CH₂CH₂CH₂CH₂CH₂CH₂CH₂SCF₃ | S |
| A-5452 | *c*-Pr | H | Cl | H | CH₂CH₂NHS(=O)₂CF₃ | S |
| A-5453 | *c*-Pr | H | Cl | H | CH₂CH₂CH₂NHS(=O)₂CF₃ | S |
| A-5454 | *c*-Pr | H | Cl | H | CH₂CH₂CH₂CH₂NHS(=O)₂CF₃ | S |
| A-5455 | *c*-Pr | H | Cl | H | CH₂CH₂CH₂CH₂CH₂NHS(=O)₂CF₃ | S |
| A-5456 | *c*-Pr | H | Cl | H | CH₂CH₂N(Me)S(=O)₂CF₃ | S |
| A-5457 | *c*-Pr | H | Cl | H | CH₂CH₂N(Et)S(=O)₂CF₃ | S |
| A-5458 | *c*-Pr | H | Cl | H | CH₂CH₂N(CH₂CN)S(=O)₂CF₃ | S |
| A-5459 | *c*-Pr | H | Cl | H | CH₂CH₂N(CH₂OMe)S(=O)₂CF₃ | S |
| A-5460 | *c*-Pr | H | Cl | H | CH₂CH₂CH₂N(Me)S(=O)₂CF₃ | S |
| A-5461 | *c*-Pr | H | Cl | H | CH₂CH₂CH₂N(Et)S(=O)₂CF₃ | S |
| A-5462 | *c*-Pr | H | Cl | H | CH₂CH₂CH₂N(CH₂CN)S(=O)₂CF₃ | S |
| A-5463 | *c*-Pr | H | Cl | H | CH₂CH₂CH₂N(CH₂OMe)S(=O)₂CF₃ | S |
| A-5464 | *c*-Pr | H | Cl | H | CH₂CH₂CH₂CH₂N(Me)S(=O)₂CF₃ | S |
| A-5465 | *c*-Pr | H | Cl | H | CH₂CH₂CH₂CH₂N(Et)S(=O)₂CF₃ | S |
| A-5466 | *c*-Pr | H | Cl | H | CH₂CH₂CH₂CH₂N(CH₂CN)S(=O)₂CF₃ | S |
| A-5467 | *c*-Pr | H | Cl | H | CH₂CH₂CH₂CH₂N(CH₂OMe)S(=O)₂CF₃ | S |
| A-5468 | *c*-Pr | H | Me | H | CH₂CH₂SCF₃ | S |
| A-5469 | *c*-Pr | H | Me | H | CH₂CH₂CH₂SCF₃ | S |
| A-5470 | *c*-Pr | H | Me | H | CH₂CH₂CH₂CH₂SCF₃ | S |
| A-5471 | *c*-Pr | H | Me | H | CH₂CH₂CH₂CH₂CH₂SCF₃ | S |
| A-5472 | *c*-Pr | H | Me | H | CH₂CH₂CH₂CH₂CH₂CH₂SCF₃ | S |
| A-5473 | *c*-Pr | H | Me | H | CH₂CH₂CH₂CH₂CH₂CH₂CH₂SCF₃ | S |
| A-5474 | *c*-Pr | H | Me | H | CH₂CH₂CH₂CH₂CH₂CH₂CH₂CH₂SCF₃ | S |
| A-5475 | *c*-Pr | H | Me | H | CH₂CH₂NHS(=O)₂CF₃ | S |
| A-5476 | *c*-Pr | H | Me | H | CH₂CH₂CH₂NHS(=O)₂CF₃ | S |
| A-5477 | *c*-Pr | H | Me | H | CH₂CH₂CH₂CH₂NHS(=O)₂CF₃ | S |
| A-5478 | *c*-Pr | H | Me | H | CH₂CH₂CH₂CH₂CH₂NHS(=O)₂CF₃ | S |
| A-5479 | *c*-Pr | H | Me | H | CH₂CH₂N(Me)S(=O)₂CF₃ | S |
| A-5480 | *c*-Pr | H | Me | H | CH₂CH₂N(Et)S(=O)₂CF₃ | S |
| A-5481 | *c*-Pr | H | Me | H | CH₂CH₂N(CH₂CN)S(=O)₂CF₃ | S |
| A-5482 | *c*-Pr | H | Me | H | CH₂CH₂N(CH₂OMe)S(=O)₂CF₃ | S |
| A-5483 | *c*-Pr | H | Me | H | CH₂CH₂CH₂N(Me)S(=O)₂CF₃ | S |
| A-5484 | *c*-Pr | H | Me | H | CH₂CH₂CH₂N(Et)S(=O)₂CF₃ | S |
| A-5485 | *c*-Pr | H | Me | H | CH₂CH₂CH₂N(CH₂CN)S(=O)₂CF₃ | S |
| A-5486 | *c*-Pr | H | Me | H | CH₂CH₂CH₂N(CH₂OMe)S(=O)₂CF₃ | S |
| A-5487 | *c*-Pr | H | Me | H | CH₂CH₂CH₂CH₂N(Me)S(=O)₂CF₃ | S |
| A-5488 | *c*-Pr | H | Me | H | CH₂CH₂CH₂CH₂N(Et)S(=O)₂CF₃ | S |
| A-5489 | *c*-Pr | H | Me | H | CH₂CH₂CH₂CH₂N(CH₂CN)S(=O)₂CF₃ | S |
| A-5480 | *c*-Pr | H | Me | H | CH₂CH₂CH₂CH₂N(CH₂OMe)S(=O)₂CF₃ | S |
| A-5491 | *c*-Pr | H | Cl | F | CH₂CH₂SCF₃ | S |
| A-5492 | *c*-Pr | H | Cl | F | CH₂CH₂CH₂SCF₃ | S |
| A-5493 | *c*-Pr | H | Cl | F | CH₂CH₂CH₂CH₂SCF₃ | S |
| A-5494 | *c*-Pr | H | Cl | F | CH₂CH₂CH₂CH₂CH₂SCF₃ | S |

**[Table 126]**

| Compound Number | R¹ | R² | R³ | R⁴ | R⁵ | A |
|---|---|---|---|---|---|---|
| A-5495 | *c*-Pr | H | Cl | F | CH₂CH₂CH₂CH₂CH₂CH₂SCF₃ | S |
| A-5496 | *c*-Pr | H | Cl | F | CH₂CH₂CH₂CH₂CH₂CH₂CH₂SCF₃ | S |
| A-5497 | *c*-Pr | H | Cl | F | CH₂CH₂CH₂CH₂CH₂CH₂CH₂CH₂SCF₃ | S |
| A-5498 | *c*-Pr | H | Cl | F | CH₂CH₂NHS(=O)₂CF₃ | S |
| A-5499 | *c*-Pr | H | Cl | F | CH₂CH₂CH₂NHS(=O)₂CF₃ | S |
| A-5500 | *c*-Pr | H | Cl | F | CH₂CH₂CH₂CH₂NHS(=O)₂CF₃ | S |
| A-5501 | *c*-Pr | H | Cl | F | CH₂CH₂CH₂CH₂CH₂NHS(=O)₂CF₃ | S |
| A-5502 | *c*-Pr | H | Cl | F | CH₂CH₂N(Me)S(=O)₂CF₃ | S |
| A-5503 | *c*-Pr | H | Cl | F | CH₂CH₂N(Et)S(=O)₂CF₃ | S |
| A-5504 | *c*-Pr | H | Cl | F | CH₂CH₂N(CH₂CN)S(=O)₂CF₃ | S |
| A-5505 | *c*-Pr | H | Cl | F | CH₂CH₂N(CH₂OMe)S(=O)₂CF₃ | S |
| A-5506 | *c*-Pr | H | Cl | F | CH₂CH₂CH₂N(Me)S(=O)₂CF₃ | S |
| A-5507 | *c*-Pr | H | Cl | F | CH₂CH₂CH₂N(Et)S(=O)₂CF₃ | S |
| A-5508 | *c*-Pr | H | Cl | F | CH₂CH₂CH₂N(CH₂CN)S(=O)₂CF₃ | S |
| A-5509 | *c*-Pr | H | Cl | F | CH₂CH₂CH₂N(CH₂OMe)S(=O)₂CF₃ | S |
| A-5510 | *c*-Pr | H | Cl | F | CH₂CH₂CH₂CH₂N(Me)S(=O)₂CF₃ | S |
| A-5511 | *c*-Pr | H | Cl | F | CH₂CH₂CH₂CH₂N(Et)S(=O)₂CF₃ | S |
| A-5512 | *c*-Pr | H | Cl | F | CH₂CH₂CH₂CH₂N(CH₂CN)S(=O)₂CF₃ | S |
| A-5513 | *c*-Pr | H | Cl | F | CH₂CH₂CH₂CH₂N(CH₂OMe)S(=O)₂CF₃ | S |
| A-5514 | *c*-Pr | H | Me | F | CH₂CH₂SCF₃ | S |
| A-5515 | *c*-Pr | H | Me | F | CH₂CH₂CH₂SCF₃ | S |
| A-5516 | *c*-Pr | H | Me | F | CH₂CH₂CH₂CH₂SCF₃ | S |
| A-5517 | *c*-Pr | H | Me | F | CH₂CH₂CH₂CH₂CH₂SCF₃ | S |
| A-5518 | *c*-Pr | H | Me | F | CH₂CH₂CH₂CH₂CH₂CH₂SCF₃ | S |
| A-5519 | *c*-Pr | H | Me | F | CH₂CH₂CH₂CH₂CH₂CH₂CH₂SCF₃ | S |
| A-5520 | *c*-Pr | H | Me | F | CH₂CH₂CH₂CH₂CH₂CH₂CH₂CH₂SCF₃ | S |
| A-5521 | *c*-Pr | H | Me | F | CH₂CH₂NHS(=O)₂CF₃ | S |
| A-5522 | *c*-Pr | H | Me | F | CH₂CH₂CH₂NHS(=O)₂CF₃ | S |
| A-5523 | *c*-Pr | H | Me | F | CH₂CH₂CH₂CH₂NHS(=O)₂CF₃ | S |
| A-5524 | *c*-Pr | H | Me | F | CH₂CH₂CH₂CH₂CH₂NHS(=O)₂CF₃ | S |
| A-5525 | *c*-Pr | H | Me | F | CH₂CH₂N(Me)S(=O)₂CF₃ | S |
| A-5526 | *c*-Pr | H | Me | F | CH₂CH₂N(Et)S(=O)₂CF₃ | S |
| A-5527 | *c*-Pr | H | Me | F | CH₂CH₂N(CH₂CN)S(=O)₂CF₃ | S |
| A-5528 | *c*-Pr | H | Me | F | CH₂CH₂N(CH₂OMe)S(=O)₂CF₃ | S |
| A-5529 | *c*-Pr | H | Me | F | CH₂CH₂CH₂N(Me)S(=O)₂CF₃ | S |
| A-5530 | *c*-Pr | H | Me | F | CH₂CH₂CH₂N(Et)S(=O)₂CF₃ | S |
| A-5531 | *c*-Pr | H | Me | F | CH₂CH₂CH₂N(CH₂CN)S(=O)₂CF₃ | S |
| A-5532 | *c*-Pr | H | Me | F | CH₂CH₂CH₂N(CH₂OMe)S(=O)₂CF₃ | S |
| A-5533 | *c*-Pr | H | Me | F | CH₂CH₂CH₂CH₂N(Me)S(=O)₂CF₃ | S |
| A-5534 | *c*-Pr | H | Me | F | CH₂CH₂CH₂CH_{2N}(Et)S(=O)₂CF₃ | S |
| A-5535 | *c*-Pr | H | Me | F | CH₂CH₂CH₂CH₂N(CH₂CN)S(=O)₂CF₃ | S |
| A-5536 | *c*-Pr | H | Me | F | CH₂CH₂CH₂CH₂N(CH₂OMe)S(=O)₂CF₃ | S |
| A-5537 | *c*-Pr | H | Cl | Cl | CH₂CH₂SCF₃ | S |
| A-5538 | *c*-Pr | H | Cl | Cl | CH₂CH₂CH₂SCF₃ | S |

**[Table 127]**

| Compound Number | R¹ | R² | R³ | R⁴ | R⁵ | A |
|---|---|---|---|---|---|---|
| A-5539 | *c*-Pr | H | Cl | Cl | CH₂CH₂CH₂CH₂SCF₃ | S |
| A-5540 | *c*-Pr | H | Cl | Cl | CH₂CH₂CH₂CH₂CH₂SCF₃ | S |
| A-5541 | *c*-Pr | H | Cl | Cl | CH₂CH₂CH₂CH₂CH₂CH₂SCF₃ | S |
| A-5542 | *c*-Pr | H | Cl | Cl | CH₂CH₂CH₂CH₂CH₂CH₂CH₂SCF₃ | S |
| A-5543 | *c*-Pr | H | Cl | Cl | CH₂CH₂CH₂CH₂CH₂CH₂CH₂CH₂SCF₃ | S |
| A-5544 | *c*-Pr | H | Cl | Cl | CH₂CH₂NHS(=O)₂CF₃ | S |
| A-5545 | *c*-Pr | H | Cl | Cl | CH₂CH₂CH₂NHS(=O)₂CF₃ | S |
| A-5546 | *c*-Pr | H | Cl | Cl | CH₂CH₂CH₂CH₂NHS(=O)₂CF₃ | S |
| A-5547 | *c*-Pr | H | Cl | Cl | CH₂CH₂CH₂CH₂CH₂NHS(=O)₂CF₃ | S |
| A-5548 | *c*-Pr | H | Cl | Cl | CH₂CH₂N(Me)S(=O)₂CF₃ | S |
| A-5549 | *c*-Pr | H | Cl | Cl | CH₂CH₂N(Et)S(=O)₂CF₃ | S |
| A-5550 | *c*-Pr | H | Cl | Cl | CH₂CH₂N(CH₂CN)S(=O)₂CF₃ | S |
| A-5551 | *c*-Pr | H | Cl | Cl | CH₂CH₂N(CH₂OMe)S(=O)₂CF₃ | S |
| A-5552 | *c*-Pr | H | Cl | Cl | CH₂CH₂CH₂N(Me)S(=O)₂CF₃ | S |
| A-5553 | *c*-Pr | H | Cl | Cl | CH₂CH₂CH₂N(Et)S(=O)₂CF₃ | S |
| A-5554 | *c*-Pr | H | Cl | Cl | CH₂CH₂CH₂N(CH₂CN)S(=O)₂CF₃ | S |
| A-5555 | *c*-Pr | H | Cl | Cl | CH₂CH₂CH₂N(CH₂OMe)S(=O)₂CF₃ | S |
| A-5556 | *c*-Pr | H | Cl | Cl | CH₂CH₂CH₂CH₂N(Me)S(=O)₂CF₃ | S |
| A-5557 | *c*-Pr | H | Cl | Cl | CH₂CH₂CH₂CH₂N(Et)S(=O)₂CF₃ | S |
| A-5558 | *c*-Pr | H | Cl | Cl | CH₂CH₂CH₂CH₂N(CH₂CN)S(=O)₂CF₃ | S |
| A-5559 | *c*-Pr | H | Cl | Cl | CH₂CH₂CH₂CH₂N(CH₂OMe)S(=O)₂CF₃ | S |
| A-5560 | *c*-Pr | H | Me | Cl | CH₂CH₂SCF₃ | S |
| A-5561 | *c*-Pr | H | Me | Cl | CH₂CH₂CH₂SCF₃ | S |
| A-5562 | *c*-Pr | H | Me | Cl | CH₂CH₂CH₂CH₂SCF₃ | S |
| A-5563 | *c*-Pr | H | Me | Cl | CH₂CH₂CH₂CH₂CH₂SCF₃ | S |
| A-5564 | *c*-Pr | H | Me | Cl | CH₂CH₂CH₂CH₂CH₂CH₂SCF₃ | S |
| A-5565 | *c*-Pr | H | Me | Cl | CH₂CH₂CH₂CH₂CH₂CH₂CH₂SCF₃ | S |
| A-5566 | *c*-Pr | H | Me | Cl | CH₂CH₂CH₂CH₂CH₂CH₂CH₂CH₂SCF₃ | S |
| A-5567 | *c*-Pr | H | Me | Cl | CH₂CH₂NHS(=O)₂CF₃ | S |
| A-5568 | *c*-Pr | H | Me | Cl | CH₂CH₂CH₂NHS(=O)₂CF₃ | S |
| A-5569 | *c*-Pr | H | Me | Cl | CH₂CH₂CH₂CH₂NHS(=O)₂CF₃ | S |
| A-5570 | *c*-Pr | H | Me | Cl | CH₂CH₂CH₂CH₂CH₂NHS(=O)₂CF₃ | S |
| A-5571 | *c*-Pr | H | Me | Cl | CH₂CH₂N(Me)S(=O)₂CF₃ | S |
| A-5572 | *c*-Pr | H | Me | Cl | CH₂CH₂N(Et)S(=O)₂CF₃ | S |
| A-5573 | *c*-Pr | H | Me | Cl | CH₂CH₂N(CH₂CN)S(=O)₂CF₃ | S |
| A-5574 | *c*-Pr | H | Me | Cl | CH₂CH₂N(CH₂OMe)S(=O)₂CF₃ | S |
| A-5575 | *c*-Pr | H | Me | Cl | CH₂CH₂CH₂N(Me)S(=O)₂CF₃ | S |
| A-5576 | *c*-Pr | H | Me | Cl | CH₂CH₂CH₂N(Et)S(=O)₂CF₃ | S |
| A-5577 | *c*-Pr | H | Me | Cl | CH₂CH₂CH₂N(CH₂CN)S(=O)₂CF₃ | S |
| A-5578 | *c*-Pr | H | Me | Cl | CH₂CH₂CH₂N(CH₂OMe)S(=O)₂CF₃ | S |
| A-5579 | *c*-Pr | H | Me | Cl | CH₂CH₂CH₂CH₂N(Me)S(=O)₂CF₃ | S |
| A-5580 | *c*-Pr | H | Me | Cl | CH₂CH₂CH₂CH₂N(Et)S(=O)₂CF₃ | S |
| A-5581 | *c*-Pr | H | Me | Cl | CH₂CH₂CH₂CH₂N(CH₂CN)S(=O)₂CF₃ | S |
| A-5582 | *c*-Pr | H | Me | Cl | CH₂CH₂CH₂CH₂N(CH₂OMe)S(=O)₂CF₃ | S |

**[Table 128]**

| Compound Number | R¹ | R² | R³ | R⁴ | R⁵ | A |
|---|---|---|---|---|---|---|
| A-5583 | *c*-Pr | H | Me | Me | CH₂CH₂SCF₃ | S |
| A-5584 | *c*-Pr | H | Me | Me | CH₂CH₂CH₂SCF₃ | S |
| A-5585 | *c*-Pr | H | Me | Me | CH₂CH₂CH₂CH₂SCF₃ | S |
| A-5586 | *c*-Pr | H | Me | Me | CH₂CH₂CH₂CH₂CH₂SCF₃ | S |
| A-5587 | *c*-Pr | H | Me | Me | CH₂CH₂CH₂CH₂CH₂CH₂SCF₃ | S |
| A-5588 | *c*-Pr | H | Me | Me | CH₂CH₂CH₂CH₂CH₂CH₂CH₂SCF₃ | S |
| A-5589 | *c*-Pr | H | Me | Me | CH₂CH₂CH₂CH₂CH₂CH₂CH₂CH₂SCF₃ | S |
| A-5590 | *c*-Pr | H | Me | Me | CH₂CH₂NHS(=O)₂CF₃ | S |
| A-5591 | *c*-Pr | H | Me | Me | CH₂CH₂CH₂NHS(=O)₂CF₃ | S |
| A-5592 | *c*-Pr | H | Me | Me | CH₂CH₂CH₂CH₂NHS(=O)₂CF₃ | S |
| A-5593 | *c*-Pr | H | Me | Me | CH₂CH₂CH₂CH₂CH₂NHS(=O)₂CF₃ | S |
| A-5594 | *c*-Pr | H | Me | Me | CH₂CH₂N(Me)S(=O)₂CF₃ | S |
| A-5595 | *c*-Pr | H | Me | Me | CH₂CH₂N(Et)S(=O)₂CF₃ | S |
| A-5596 | *c*-Pr | H | Me | Me | CH₂CH₂N(CH₂CN)S(=O)₂CF₃ | S |
| A-5597 | *c*-Pr | H | Me | Me | CH₂CH₂N(CH₂OMe)S(=O)₂CF₃ | S |
| A-5598 | *c*-Pr | H | Me | Me | CH₂CH₂CH₂N(Me)S(=O)₂CF₃ | S |
| A-5599 | *c*-Pr | H | Me | Me | CH₂CH₂CH₂N(Et)S(=O)₂CF₃ | S |
| A-5600 | *c*-Pr | H | Me | Me | CH₂CH₂CH₂N(CH₂CN)S(=O)₂CF₃ | S |
| A-5601 | *c*-Pr | H | Me | Me | CH₂CH₂CH₂N(CH₂OMe)S(=O)₂CF₃ | S |
| A-5602 | *c*-Pr | H | Me | Me | CH₂CH₂CH₂CH₂N(Me)S(=O)₂CF₃ | S |
| A-5603 | *c*-Pr | H | Me | Me | CH₂CH₂CH₂CH₂N(Et)S(=O)₂CF₃ | S |
| A-5604 | *c*-Pr | H | Me | Me | CH₂CH₂CH₂CH₂N(CH₂CN)S(=O)₂CF₃ | S |
| A-5605 | *c*-Pr | H | Me | Me | CH₂CH₂CH₂CH₂N(CH₂OMe)S(=O)₂CF₃ | S |
| A-5606 | Me | H | Cl | F | CH₂CH₂SCF₃ | O |
| A-5607 | Me | H | Cl | F | CH₂CH₂CH₂SCF₃ | O |
| A-5608 | Me | H | Cl | F | CH₂CH₂CH₂CH₂SCF₃ | O |
| A-5609 | Me | H | Cl | F | CH₂CH₂CH₂CH₂CH₂SCF₃ | O |
| A-5610 | Me | H | Cl | F | CH₂CH₂CH₂CH₂CH₂CH₂SCF₃ | O |
| A-5611 | Me | H | Cl | F | CH₂CH₂CH₂CH₂CH₂CH₂CH₂SCF₃ | O |
| A-5612 | Me | H | Cl | F | CH₂CH₂CH₂CH₂CH₂CH₂CH₂CH₂SCF₃ | O |
| A-5613 | Me | H | Cl | F | CH₂CH₂NHS(=O)₂CF₃ | O |
| A-5614 | Me | H | Cl | F | CH₂CH₂CH₂NHS(=O)₂CF₃ | O |
| A-5615 | Me | H | Cl | F | CH₂CH₂CH₂CH₂NHS(=O)₂CF₃ | O |
| A-5616 | Me | H | Cl | F | CH₂CH₂CH₂CH₂CH₂NHS(=O)₂CF₃ | O |
| A-5617 | Me | H | Cl | F | CH₂CH₂N(Me)S(=O)₂CF₃ | O |
| A-5618 | Me | H | Cl | F | CH₂CH₂N(Et)S(=O)₂CF₃ | O |
| A-5619 | Me | H | Cl | F | CH₂CH₂N(CH₂CN)S(=O)₂CF₃ | O |
| A-5620 | Me | H | Cl | F | CH₂CH₂N(CH₂OMe)S(=O)₂CF₃ | O |
| A-5621 | Me | H | Cl | F | CH₂CH₂CH₂N(Me)S(=O)₂CF₃ | O |
| A-5622 | Me | H | Cl | F | CH₂CH₂CH₂N(Et)S(=O)₂CF₃ | O |
| A-5623 | Me | H | Cl | F | CH₂CH₂CH₂N(CH₂CN)S(=O)₂CF₃ | O |
| A-5624 | Me | H | Cl | F | CH₂CH₂CH₂N(CH₂OMe)S(=O)₂CF₃ | O |
| A-5625 | Me | H | Cl | F | CH₂CH₂CH₂CH₂N(Me)S(=O)₂CF₃ | O |
| A-5626 | Me | H | Cl | F | CH₂CH₂CH₂CH₂N(Et)S(=O)₂CF₃ | O |

**[Table 129]**

| Compound Number | R¹ | R² | R³ | R⁴ | R⁵ | A |
|---|---|---|---|---|---|---|
| A-5627 | Me | H | Cl | F | CH₂CH₂CH₂CH₂N(CH₂CN)S(=O)₂CF₃ | O |
| A-5628 | Me | H | Cl | F | CH₂CH₂CH₂CH₂N(CH₂OMe)S(=O)₂CF₃ | O |
| A-5629 | Me | H | Me | F | CH₂CH₂SCF₃ | O |
| A-5630 | Me | H | Me | F | CH₂CH₂CH₂SCF₃ | O |
| A-5631 | Me | H | Me | F | CH₂CH₂CH₂CH₂SCF₃ | O |
| A-5632 | Me | H | Me | F | CH₂CH₂CH₂CH₂CH₂SCF₃ | O |
| A-5633 | Me | H | Me | F | CH₂CH₂CH₂CH₂CH₂CH₂SCF₃ | O |
| A-5634 | Me | H | Me | F | CH₂CH₂CH₂CH₂CH₂CH₂CH₂SCF₃ | O |
| A-5635 | Me | H | Me | F | CH₂CH₂CH₂CH₂CH₂CH₂CH₂CH₂SCF₃ | O |
| A-5636 | Me | H | Me | F | CH₂CH₂NHS(=O)₂CF₃ | O |
| A-5637 | Me | H | Me | F | CH₂CH₂CH₂NHS(=O)₂CF₃ | O |
| A-5638 | Me | H | Me | F | CH₂CH₂CH₂CH₂NHS(=O)₂CF₃ | O |
| A-5639 | Me | H | Me | F | CH₂CH₂CH₂CH₂CH₂NHS(=O)₂CF₃ | O |
| A-5640 | Me | H | Me | F | CH₂CH₂N(Me)S(=O)₂CF₃ | O |
| A-5641 | Me | H | Me | F | CH₂CH₂N(Et)S(=O)₂CF₃ | O |
| A-5642 | Me | H | Me | F | CH₂CH₂N(CH₂CN)S(=O)₂CF₃ | O |
| A-5643 | Me | H | Me | F | CH₂CH₂N(CH₂OMe)S(=O)₂CF₃ | O |
| A-5644 | Me | H | Me | F | CH₂CH₂CH₂N(Me)S(=O)₂CF₃ | O |
| A-5645 | Me | H | Me | F | CH₂CH₂CH₂N(Et)S(=O)₂CF₃ | O |
| A-5646 | Me | H | Me | F | CH₂CH₂CH₂N(CH₂CN)S(=O)₂CF₃ | O |
| A-5647 | Me | H | Me | F | CH₂CH₂CH₂N(CH₂OMe)S(=O)₂CF₃ | O |
| A-5648 | Me | H | Me | F | CH₂CH₂CH₂CH₂N(Me)S(=O)₂CF₃ | O |
| A-5649 | Me | H | Me | F | CH₂CH₂CH₂CH₂N(Et)S(=O)₂CF₃ | O |
| A-5650 | Me | H | Me | F | CH₂CH₂CH₂CH₂N(CH₂CN)S(=O)₂CF₃ | O |
| A-5651 | Me | H | Me | F | CH₂CH₂CH₂CH₂N(CH₂OMe)S(=O)₂CF₃ | O |
| A-5652 | *n*-Pr | H | Cl | F | CH₂CH₂SCF₃ | O |
| A-5653 | *n*-Pr | H | Cl | F | CH₂CH₂CH₂SCF₃ | O |
| A-5654 | *n*-Pr | H | Cl | F | CH₂CH₂CH₂CH₂SCF₃ | O |
| A-5655 | *n*-Pr | H | Cl | F | CH₂CH₂CH₂CH₂CH₂SCF₃ | O |
| A-5656 | *n*-Pr | H | Cl | F | CH₂CH₂CH₂CH₂CH₂CH₂SCF₃ | O |
| A-5657 | *n*-Pr | H | Cl | F | CH₂CH₂CH₂CH₂CH₂CH₂CH₂SCF₃ | O |
| A-5658 | *n*-Pr | H | Cl | F | CH₂CH₂CH₂CH₂CH₂CH₂CH₂CH₂SCF₃ | O |
| A-5659 | *n*-Pr | H | Cl | F | CH₂CH₂NHS(=O)₂CF₃ | O |
| A-5660 | *n*-Pr | H | Cl | F | CH₂CH₂CH₂NHS(=O)₂CF₃ | O |
| A-5661 | *n*-Pr | H | Cl | F | CH₂CH₂CH₂CH₂NHS(=O)₂CF₃ | O |
| A-5662 | *n*-Pr | H | Cl | F | CH₂CH₂CH₂CH₂CH₂NHS(=O)₂CF₃ | O |
| A-5663 | *n*-Pr | H | Cl | F | CH₂CH₂N(Me)S(=O)₂CF₃ | O |
| A-5664 | *n*-Pr | H | Cl | F | CH₂CH₂N(Et)S(=O)₂CF₃ | O |
| A-5665 | *n*-Pr | H | Cl | F | CH₂CH₂N(CH₂CN)S(=O)₂CF₃ | O |
| A-5666 | *n*-Pr | H | Cl | F | CH₂CH₂N(CH₂OMe)S(=O)₂CF₃ | O |
| A-5667 | *n*-Pr | H | Cl | F | CH₂CH₂CH₂N(Me)S(=O)₂CF₃ | O |
| A-5668 | *n*-Pr | H | Cl | F | CH₂CH₂CH₂N(Et)S(=O)₂CF₃ | O |
| A-5669 | *n*-Pr | H | Cl | F | CH₂CH₂CH₂N(CH₂CN)S(=O)₂CF₃ | O |
| A-5670 | *n*-Pr | H | Cl | F | CH₂CH₂CH₂N(CH₂OMe)S(=O)₂CF₃ | O |

**[Table 130]**

| Compound Number | R¹ | R² | R³ | R⁴ | R⁵ | A |
|---|---|---|---|---|---|---|
| A-5671 | *n*-Pr | H | Cl | F | CH₂CH₂CH₂CH₂N(Me)S(=O)₂CF₃ | O |
| A-5672 | *n*-Pr | H | Cl | F | CH₂CH₂CH₂CH₂N(Et)S(=O)₂CF₃ | O |
| A-5673 | *n*-Pr | H | Cl | F | CH₂CH₂CH₂CH₂N(CH₂CN)S(=O)₂CF₃ | O |
| A-5674 | *n*-Pr | H | Cl | F | CH₂CH₂CH₂CH₂N(CH₂OMe)S(=O)₂CF₃ | O |
| A-5675 | *n*-Pr | H | Me | F | CH₂CH₂SCF₃ | O |
| A-5676 | *n*-Pr | H | Me | F | CH₂CH₂CH₂SCF₃ | O |
| A-5677 | *n*-Pr | H | Me | F | CH₂CH₂CH₂CH₂SCF₃ | O |
| A-5678 | *n*-Pr | H | Me | F | CH₂CH₂CH₂CH₂CH₂SCF₃ | O |
| A-5679 | *n*-Pr | H | Me | F | CH₂CH₂CH₂CH₂CH₂CH₂SCF₃ | O |
| A-5680 | *n*-Pr | H | Me | F | CH₂CH₂CH₂CH₂CH₂CH₂CH₂SCF₃ | O |
| A-5681 | *n-Pr* | H | Me | F | CH₂CH₂CH₂CH₂CH₂CH₂CH₂CH₂SCF₃ | O |
| A-5682 | *n*-Pr | H | Me | F | CH₂CH₂NHS(=O)₂CF₃ | O |
| A-5683 | *n*-Pr | H | Me | F | CH₂CH₂CH₂NHS(=O)₂CF₃ | O |
| A-5684 | *n*-Pr | H | Me | F | CH₂CH₂CH₂CH₂NHS(=O)₂CF₃ | O |
| A-5685 | *n*-Pr | H | Me | F | CH₂CH₂CH₂CH₂CH₂NHS(=O)₂CF₃ | O |
| A-5686 | *n*-Pr | H | Me | F | CH₂CH₂N(Me)S(=O)₂CF₃ | O |
| A-5687 | *n*-Pr | H | Me | F | CH₂CH₂N(Et)S(=O)₂CF₃ | O |
| A-5688 | *n*-Pr | H | Me | F | CH₂CH₂N(CH₂CN)S(=O)₂CF₃ | O |
| A-5689 | *n*-Pr | H | Me | F | CH₂CH₂N(CH₂OMe)S(=O)₂CF₃ | O |
| A-5690 | *n*-Pr | H | Me | F | CH₂CH₂CH₂N(Me)S(=O)₂CF₃ | O |
| A-5691 | *n*-Pr | H | Me | F | CH₂CH₂CH₂N(Et)S(=O)₂CF₃ | O |
| A-5692 | *n*-Pr | H | Me | F | CH₂CH₂CH₂N(CH₂CN)S(=O)₂CF₃ | O |
| A-5693 | *n*-Pr | H | Me | F | CH₂CH₂CH₂N(CH₂OMe)S(=O)₂CF₃ | O |
| A-5694 | *n*-Pr | H | Me | F | CH₂CH₂CH₂CH₂N(Me)S(=O)₂CF₃ | O |
| A-5695 | *n*-Pr | H | Me | F | CH₂CH₂CH₂CH₂N(Et)S(=O)₂CF₃ | O |
| A-5696 | *n*-Pr | H | Me | F | CH₂CH₂CH₂CH₂N(CH₂CN)S(=O)₂CF₃ | O |
| A-5697 | *n*-Pr | H | Me | F | CH₂CH₂CH₂CH₂N(CH₂OMe)S(=O)₂CF₃ | O |
| A-5698 | *i*-Pr | H | Cl | F | CH₂CH₂SCF₃ | O |
| A-5699 | *i*-Pr | H | Cl | F | CH₂CH₂CH₂SCF₃ | O |
| A-5700 | *i*-Pr | H | Cl | F | CH₂CH₂CH₂CH₂SCF₃ | O |
| A-5701 | *i*-Pr | H | Cl | F | CH₂CH₂CH₂CH₂CH₂SCF₃ | O |
| A-5702 | *i*-Pr | H | Cl | F | CH₂CH₂CH₂CH₂CH₂CH₂SCF₃ | O |
| A-5703 | *i*-Pr | H | Cl | F | CH₂CH₂CH₂CH₂CH₂CH₂CH₂SCF₃ | O |
| A-5704 | *i*-Pr | H | Cl | F | CH₂CH₂CH₂CH₂CH₂CH₂CH₂CH₂SCF₃ | O |
| A-5705 | *i*-Pr | H | Cl | F | CH₂CH₂NHS(=O)₂CF₃ | O |
| A-5706 | *i*-Pr | H | Cl | F | CH₂CH₂CH₂NHS(=O)₂CF₃ | O |
| A-5707 | *i*-Pr | H | Cl | F | CH₂CH₂CH₂CH₂NHS(=O)₂CF₃ | O |
| A-5708 | *i*-Pr | H | Cl | F | CH₂CH₂CH₂CH₂CH₂NHS(=O)₂CF₃ | O |
| A-5709 | *i*-Pr | H | Cl | F | CH₂CH₂N(Me)S(=O)₂CF₃ | O |
| A-5710 | *i*-Pr | H | Cl | F | CH₂CH₂N(Et)S(=O)₂CF₃ | O |
| A-5711 | *i*-Pr | H | Cl | F | CH₂CH₂N(CH₂CN)S(=O)₂CF₃ | O |
| A-5712 | *i*-Pr | H | Cl | F | CH₂CH₂N(CH₂OMe)S(=O)₂CF₃ | O |
| A-5713 | *i*-Pr | H | Cl | F | CH₂CH₂CH₂N(Me)S(=O)₂CF₃ | O |
| A-5714 | *i*-Pr | H | Cl | F | CH₂CH₂CH₂N(Et)S(=O)₂CF₃ | O |

**[Table 131]**

| Compound Number | R¹ | R² | R³ | R⁴ | R⁵ | A |
|---|---|---|---|---|---|---|
| A-5715 | *i*-Pr | H | Cl | F | CH₂CH₂CH₂N(CH₂CN)S(=O)₂CF₃ | O |
| A-5716 | *i*-Pr | H | Cl | F | CH₂CH₂CH₂N(CH₂OMe)S(=O)₂CF₃ | O |
| A-5717 | *i*-Pr | H | Cl | F | CH₂CH₂CH₂CH₂N(Me)S(=O)₂CF₃ | O |
| A-5718 | *i*-Pr | H | Cl | F | CH₂CH₂CH₂CH₂N(Et)S(=O)₂CF₃ | O |
| A-5719 | *i*-Pr | H | Cl | F | CH₂CH₂CH₂CH₂N(CH₂CN)S(=O)₂CF₃ | O |
| A-5720 | *i*-Pr | H | Cl | F | CH₂CH₂CH₂CH₂N(CH₂OMe)S(=O)₂CF₃ | O |
| A-5721 | *i*-Pr | H | Me | F | CH₂CH₂SCF₃ | O |
| A-5722 | *i*-Pr | H | Me | F | CH₂CH₂CH₂SCF₃ | O |
| A-5723 | *i*-Pr | H | Me | F | CH₂CH₂CH₂CH₂SCF₃ | O |
| A-5724 | *i*-Pr | H | Me | F | CH₂CH₂CH₂CH₂CH₂SCF₃ | O |
| A-5725 | *i*-Pr | H | Me | F | CH₂CH₂CH₂CH₂CH₂CH₂SCF₃ | O |
| A-5726 | *i*-Pr | H | Me | F | CH₂CH₂CH₂CH₂CH₂CH₂CH₂SCF₃ | O |
| A-5727 | *i*-Pr | H | Me | F | CH₂CH₂CH₂CH₂CH₂CH₂CH₂CH₂SCF₃ | O |
| A-5728 | *i*-Pr | H | Me | F | CH₂CH₂NHS(=O)₂CF₃ | O |
| A-5729 | *i*-Pr | H | Me | F | CH₂CH₂CH₂NHS(=O)₂CF₃ | O |
| A-5730 | *i*-Pr | H | Me | F | CH₂CH₂CH₂CH₂NHS(=O)₂CF₃ | O |
| A-5731 | *i*-Pr | H | Me | F | CH₂CH₂CH₂CH₂CH₂NHS(=O)₂CF₃ | O |
| A-5732 | *i*-Pr | H | Me | F | CH₂CH₂N(Me)S(=O)₂CF₃ | O |
| A-5733 | *i*-Pr | H | Me | F | CH₂CH₂N(Et)S(=O)₂CF₃ | O |
| A-5734 | *i*-Pr | H | Me | F | CH₂CH₂N(CH₂CN)S(=O)₂CF₃ | O |
| A-5735 | *i*-Pr | H | Me | F | CH₂CH₂N(CH₂OMe)S(=O)₂CF₃ | O |
| A-5736 | *i*-Pr | H | Me | F | CH₂CH₂CH₂N(Me)S(=O)₂CF₃ | O |
| A-5737 | *i*-Pr | H | Me | F | CH₂CH₂CH₂N(Et)S(=O)₂CF₃ | O |
| A-5738 | *i*-Pr | H | Me | F | CH₂CH₂CH₂N(CH₂CN)S(=O)₂CF₃ | O |
| A-5739 | *i*-Pr | H | Me | F | CH₂CH₂CH₂N(CH₂OMe)S(=O)₂CF₃ | O |
| A-5740 | *i*-Pr | H | Me | F | CH₂CH₂CH₂CH₂N(Me)S(=O)₂CF₃ | O |
| A-5741 | *i*-Pr | H | Me | F | CH₂CH₂CH₂CH₂N(Et)S(=O)₂CF₃ | O |
| A-5742 | *i*-Pr | H | Me | F | CH₂CH₂CH₂CH₂N(CH₂CN)S(=O)₂CF₃ | O |
| A-5743 | *i*-Pr | H | Me | F | CH₂CH₂CH₂CH₂N(CH₂OMe)S(=O)₂CF₃ | O |
| A-5744 | *c*-Pr(1-CN) | H | Cl | F | CH₂CH₂SCF₃ | O |
| A-5745 | *c*-Pr(1-CN) | H | Cl | F | CH₂CH₂CH₂SCF₃ | O |
| A-5746 | *c*-Pr(1-CN) | H | Cl | F | CH₂CH₂CH₂CH₂SCF₃ | O |
| A-5747 | *c*-Pr(1-CN) | H | Cl | F | CH₂CH₂CH₂CH₂CH₂SCF₃ | O |
| A-5748 | *c*-Pr(1-CN) | H | Cl | F | CH₂CH₂CH₂CH₂CH₂CH₂SCF₃ | O |
| A-5749 | *c*-Pr(1-CN) | H | Cl | F | CH₂CH₂CH₂CH₂CH₂CH₂CH₂SCF₃ | O |
| A-5750 | *c*-Pr(1-CN) | H | Cl | F | CH₂CH₂CH₂CH₂CH₂CH₂CH₂CH₂SCF₃ | O |
| A-5751 | *c*-Pr(1-CN) | H | Cl | F | CH₂CH₂NHS(=O)₂CF₃ | O |
| A-5752 | *c*-Pr(1-CN) | H | Cl | F | CH₂CH₂CH₂NHS(=O)₂CF₃ | O |
| A-5753 | *c*-Pr(1-CN) | H | Cl | F | CH₂CH₂CH₂CH₂NHS(=O)₂CF₃ | O |
| A-5754 | *c*-Pr(1-CN) | H | Cl | F | CH₂CH₂CH₂CH₂CH₂NHS(=O)₂CF₃ | O |
| A-5755 | *c*-Pr(1-CN) | H | Cl | F | CH₂CH₂N(Me)S(=O)₂CF₃ | O |
| A-5756 | *c*-Pr(1-CN) | H | Cl | F | CH₂CH₂N(Et)S(=O)₂CF₃ | O |
| A-5757 | *c*-Pr(1-CN) | H | Cl | F | CH₂CH₂N(CH₂CN)S(=O)₂CF₃ | O |
| A-5758 | *c*-Pr(1-CN) | H | Cl | F | CH₂CH₂N(CH₂OMe)S(=O)₂CF₃ | O |

**[Table 132]**

| Compound Number | R¹ | R² | R³ | R⁴ | R⁵ | A |
|---|---|---|---|---|---|---|
| A-5759 | *c*-Pr(1-CN) | H | Cl | F | CH₂CH₂CH₂N(Me)S(=O)₂CF₃ | O |
| A-5760 | *c*-Pr(1-CN) | H | Cl | F | CH₂CH₂CH₂N(Et)S(=O)₂CF₃ | O |
| A-5761 | *c*-Pr(1-CN) | H | Cl | F | CH₂CH₂CH₂N(CH₂CN)S(=O)₂CF₃ | O |
| A-5762 | *c*-Pr(1-CN) | H | Cl | F | CH₂CH₂CH₂N(CH₂OMe)S(=O)₂CF₃ | O |
| A-5763 | *c*-Pr(1-CN) | H | Cl | F | CH₂CH₂CH₂CH₂N(Me)S(=O)₂CF₃ | O |
| A-5764 | *c*-Pr(1-CN) | H | Cl | F | CH₂CH₂CH₂CH₂N(Et)S(=O)₂CF₃ | O |
| A-5765 | *c*-Pr(1-CN) | H | Cl | F | CH₂CH₂CH₂CH₂N(CH₂CN)S(=O)₂CF₃ | O |
| A-5766 | *c*-Pr(1-CN) | H | Cl | F | CH₂CH₂CH₂CH₂N(CH₂OMe)S(=O)₂CF₃ | O |
| A-5767 | *c*-Pr(1-CN) | H | Me | F | CH₂CH₂SCF₃ | O |
| A-5768 | *c*-Pr(1-CN) | H | Me | F | CH₂CH₂CH₂SCF₃ | O |
| A-5769 | *c*-Pr(1-CN) | H | Me | F | CH₂CH₂CH₂CH₂SCF₃ | O |
| A-5770 | *c*-Pr(1-CN) | H | Me | F | CH₂CH₂CH₂CH₂CH₂SCF₃ | O |
| A-5771 | *c*-Pr(1-CN) | H | Me | F | CH₂CH₂CH₂CH₂CH₂CH₂SCF₃ | O |
| A-5772 | *c*-Pr(1-CN) | H | Me | F | CH₂CH₂CH₂CH₂CH₂CH₂CH₂SCF₃ | O |
| A-5773 | *c*-Pr(1-CN) | H | Me | F | CH₂CH₂CH₂CH₂CH₂CH₂CH₂CH₂SCF₃ | O |
| A-5774 | *c*-Pr(1-CN) | H | Me | F | CH₂CH₂NHS(=O)₂CF₃ | O |
| A-5775 | *c*-Pr(1-CN) | H | Me | F | CH₂CH₂CH₂NHS(=O)₂CF₃ | O |
| A-5776 | *c*-Pr(1-CN) | H | Me | F | CH₂CH₂CH₂CH₂NHS(=O)₂CF₃ | O |
| A-5777 | *c*-Pr(1-CN) | H | Me | F | CH₂CH₂CH₂CH₂CH₂NHS(=O)₂CF₃ | O |
| A-5778 | *c*-Pr(1-CN) | H | Me | F | CH₂CH₂N(Me)S(=O)₂CF₃ | O |
| A-5779 | *c*-Pr(1-CN) | H | Me | F | CH₂CH₂N(Et)S(=O)₂CF₃ | O |
| A-5780 | *c*-Pr(1-CN) | H | Me | F | CH₂CH₂N(CH₂CN)S(=O)₂CF₃ | O |
| A-5781 | *c*-Pr(1-CN) | H | Me | F | CH₂CH₂N(CH₂OMe)S(=O)₂CF₃ | O |
| A-5782 | *c*-Pr(1-CN) | H | Me | F | CH₂CH₂CH₂N(Me)S(=O)₂CF₃ | O |
| A-5783 | *c*-Pr(1-CN) | H | Me | F | CH₂CH₂CH₂N(Et)S(=O)₂CF₃ | O |
| A-5784 | *c*-Pr(1-CN) | H | Me | F | CH₂CH₂CH₂N(CH₂CN)S(=O)₂CF₃ | O |
| A-5785 | *c*-Pr(1-CN) | H | Me | F | CH₂CH₂CH₂N(CH₂OMe)S(=O)₂CF₃ | O |
| A-5786 | *c*-Pr(1-CN) | H | Me | F | CH₂CH₂CH₂CH₂N(Me)S(=O)₂CF₃ | O |
| A-5787 | *c*-Pr(1-CN) | H | Me | F | CH₂CH₂CH₂CH₂N(Et)S(=O)₂CF₃ | O |
| A-5788 | *c*-Pr(1-CN) | H | Me | F | CH₂CH₂CH₂CH₂N(CH₂CN)S(=O)₂CF₃ | O |
| A-5789 | *c*-Pr(1-CN) | H | Me | F | CH₂CH₂CH₂CH₂N(CH₂OMe)S(=O)₂CF₃ | O |
| A-5790 | *c*-Pr(1-CN) | H | Cl | H | CH₂CH₂SCF₃ | O |
| A-5791 | *c*-Pr(1-CN) | H | Cl | H | CH₂CH₂CH₂SCF₃ | O |
| A-5792 | *c*-Pr(1-CN) | H | Cl | H | CH₂CH₂CH₂CH₂SCF₃ | O |
| A-5793 | *c*-Pr(1-CN) | H | Cl | H | CH₂CH₂CH₂CH₂CH₂SCF₃ | O |
| A-5794 | *c*-Pr(1-CN) | H | Cl | H | CH₂CH₂CH₂CH₂CH₂CH₂SCF₃ | O |
| A-5795 | *c*-Pr(1-CN) | H | Cl | H | CH₂CH₂CH₂CH₂CH₂CH₂CH₂SCF₃ | O |
| A-5796 | *c*-Pr(1-CN) | H | Cl | H | CH₂CH₂CH₂CH₂CH₂CH₂CH₂CH₂SCF₃ | O |
| A-5797 | *c*-Pr(1-CN) | H | Cl | H | CH₂CH₂NHS(=O)₂CF₃ | O |
| A-5798 | *c*-Pr(1-CN) | H | Cl | H | CH₂CH₂CH₂NHS(=O)₂CF₃ | O |
| A-5799 | *c*-Pr(1-CN) | H | Cl | H | CH₂CH₂CH₂CH₂NHS(=O)₂CF₃ | O |
| A-5800 | *c*-Pr(1-CN) | H | Cl | H | CH₂CH₂CH₂CH₂CH₂NHS(=O)₂CF₃ | O |
| A-5801 | *c*-Pr(1-CN) | H | Cl | H | CH₂CH₂N(Me)S(=O)₂CF₃ | O |
| A-5802 | *c*-Pr(1-CN) | H | Cl | H | CH₂CH₂N(Et)S(=O)₂CF₃ | O |

**[Table 133]**

| Compound Number | R¹ | R² | R³ | R⁴ | R⁵ | A |
|---|---|---|---|---|---|---|
| A-5803 | *c*-Pr(1-CN) | H | Cl | H | CH₂CH₂N(CH₂CN)S(=O)₂CF₃ | O |
| A-5804 | *c*-Pr(1-CN) | H | Cl | H | CH₂CH₂N(CH₂OMe)S(=O)₂CF₃ | O |
| A-5805 | *c*-Pr(1-CN) | H | Cl | H | CH₂CH₂CH₂N(Me)S(=O)₂CF₃ | O |
| A-5806 | *c*-Pr(1-CN) | H | Cl | H | CH₂CH₂CH₂N(Et)S(=O)₂CF₃ | O |
| A-5807 | *c*-Pr(1-CN) | H | Cl | H | CH₂CH₂CH₂N(CH₂CN)S(=O)₂CF₃ | O |
| A-5808 | *c*-Pr(1-CN) | H | Cl | H | CH₂CH₂CH₂N(CH₂OMe)S(=O)₂CF₃ | O |
| A-5809 | *c*-Pr(1-CN) | H | Cl | H | CH₂CH₂CH₂CH₂N(Me)S(=O)₂CF₃ | O |
| A-5810 | *c*-Pr(1-CN) | H | Cl | H | CH₂CH₂CH₂CH₂N(Et)S(=O)₂CF₃ | O |
| A-5811 | *c*-Pr(1-CN) | H | Cl | H | CH₂CH₂CH₂CH₂N(CH₂CN)S(=O)₂CF₃ | O |
| A-5812 | *c*-Pr(1-CN) | H | Cl | H | CH₂CH₂CH₂CH₂N(CH₂OMe)S(=O)₂CF₃ | O |
| A-5813 | *c*-Pr(1-CN) | H | Me | H | CH₂CH₂SCF₃ | O |
| A-5814 | *c*-Pr(1-CN) | H | Me | H | CH₂CH₂CH₂SCF₃ | O |
| A-5815 | *c*-Pr(1-CN) | H | Me | H | CH₂CH₂CH₂CH₂SCF₃ | O |
| A-5816 | *c*-Pr(1-CN) | H | Me | H | CH₂CH₂CH₂CH₂CH₂SCF₃ | O |
| A-5817 | *c*-Pr(1-CN) | H | Me | H | CH₂CH₂CH₂CH₂CH₂CH₂SCF₃ | O |
| A-5818 | *c*-Pr(1-CN) | H | Me | H | CH₂CH₂CH₂CH₂CH₂CH₂CH₂SCF₃ | O |
| A-5819 | *c*-Pr(1-CN) | H | Me | H | CH₂CH₂CH₂CH₂CH₂CH₂CH₂CH₂SCF₃ | O |
| A-5820 | *c*-Pr(1-CN) | H | Me | H | CH₂CH₂NHS(=O)₂CF₃ | O |
| A-5821 | c-Pr(1-CN) | H | Me | H | CH₂CH₂CH₂NHS(=O)₂CF₃ | O |
| A-5822 | *c*-Pr(1-CN) | H | Me | H | CH₂CH₂CH₂CH₂NHS(=O)₂CF₃ | O |
| A-5823 | c-Pr(1-CN) | H | Me | H | CH₂CH₂CH₂CH₂CH₂NHS(=O)₂CF₃ | O |
| A-5824 | *c*-Pr(1-CN) | H | Me | H | CH₂CH₂N(Me)S(=O)₂CF₃ | O |
| A-5825 | *c*-Pr(1-CN) | H | Me | H | CH₂CH₂N(Et)S(=O)₂CF₃ | O |
| A-5826 | *c*-Pr(1-CN) | H | Me | H | CH₂CH₂N(CH₂CN)S(=O)₂CF₃ | O |
| A-5827 | c-Pr(1-CN) | H | Me | H | CH₂CH₂N(CH₂OMe)S(=O)₂CF₃ | O |
| A-5828 | *c*-Pr(1-CN) | H | Me | H | CH₂CH₂CH₂N(Me)S(=O)₂CF₃ | O |
| A-5829 | *c*-Pr(1-CN) | H | Me | H | CH₂CH₂CH₂N(Et)S(=O)₂CF₃ | O |
| A-5830 | *c*-Pr(1-CN) | H | Me | H | CH₂CH₂CH₂N(CH₂CN)S(=O)₂CF₃ | O |
| A-5831 | *c*-Pr(1-CN) | H | Me | H | CH₂CH₂CH₂N(CH₂OMe)S(=O)₂CF₃ | O |
| A-5832 | *c*-Pr(1-CN) | H | Me | H | CH₂CH₂CH₂CH₂N(Me)S(=O)₂CF₃ | O |
| A-5833 | *c*-Pr(1-CN) | H | Me | H | CH₂CH₂CH₂CH₂N(Et)S(=O)₂CF₃ | O |
| A-5834 | *c*-Pr(1-CN) | H | Me | H | CH₂CH₂CH₂CH₂N(CH₂CN)S(=O)₂CF₃ | O |
| A-5835 | *c*-Pr(1-CN) | H | Me | H | CH₂CH₂CH₂CH₂N(CH₂OMe)S(=O)₂CF₃ | O |
| A-5836 | *c*-Pr(2,2-F₂) | H | Cl | F | CH₂CH₂SCF₃ | O |
| A-5837 | *c*-Pr(2,2-F₂) | H | Cl | F | CH₂CH₂CH₂SCF₃ | O |
| A-5838 | *c*-Pr(2,2-F₂) | H | Cl | F | CH₂CH₂CH₂CH₂SCF₃ | O |
| A-5839 | *c*-Pr(2,2-F₂) | H | Cl | F | CH₂CH₂CH₂CH₂CH₂SCF₃ | O |
| A-5840 | *c*-Pr(2,2-F₂) | H | Cl | F | CH₂CH₂CH₂CH₂CH₂CH₂SCF₃ | O |
| A-5841 | *c*-Pr(2,2-F₂) | H | Cl | F | CH₂CH₂CH₂CH₂CH₂CH₂CH₂SCF₃ | O |
| A-5842 | *c*-Pr(2,2-F₂) | H | Cl | F | CH₂CH₂CH₂CH₂CH₂CH₂CH₂CH₂SCF₃ | O |
| A-5843 | *c*-Pr(2,2-F₂) | H | Cl | F | CH₂CH₂NHS(=O)₂CF₃ | O |
| A-5844 | *c*-Pr(2,2-F₂) | H | Cl | F | CH₂CH₂CH₂NHS(=O)₂CF₃ | O |
| A-5845 | *c*-Pr(2,2-F₂) | H | Cl | F | CH₂CH₂CH₂CH₂NHS(=O)₂CF₃ | O |
| A-5846 | *c*-Pr(2,2-F₂) | H | Cl | F | CH₂CH₂CH₂CH₂CH₂NHS(=O)₂CF₃ | O |

**[Table 134]**

| Compound Number | R¹ | R² | R³ | R⁴ | R⁵ | A |
|---|---|---|---|---|---|---|
| A-5847 | *c*-Pr(2,2-F₂) | H | Cl | F | CH₂CH₂N(Me)S(=O)₂CF₃ | O |
| A-5848 | *c*-Pr(2,2-F₂) | H | Cl | F | CH₂CH₂N(Et)S(=O)₂CF₃ | O |
| A-5849 | *c*-Pr(2,2-F₂) | H | Cl | F | CH₂CH₂N(CH₂CN)S(=O)₂CF₃ | O |
| A-5850 | *c*-Pr(2,2-F₂) | H | Cl | F | CH₂CH₂N(CH₂OMe)S(=O)₂CF₃ | O |
| A-5851 | *c*-Pr(2,2-F₂) | H | Cl | F | CH₂CH₂CH₂N(Me)S(=O)₂CF₃ | O |
| A-5852 | c -Pr(2,2-F₂) | H | Cl | F | CH₂CH₂CH₂N(Et)S(=O)₂CF₃ | O |
| A-5853 | *c*-Pr(2,2-F₂) | H | Cl | F | CH₂CH₂CH₂N(CH₂CN)S(=O)₂CF₃ | O |
| A-5854 | *c*-Pr(2,2-F₂) | H | Cl | F | CH₂CH₂CH₂N(CH₂OMe)S(=O)₂CF₃ | O |
| A-5855 | *c*-Pr(2,2-F₂) | H | Cl | F | CH₂CH₂CH₂CH₂N(Me)S(=O)₂CF₃ | O |
| A-5856 | *c*-Pr(2,2-F₂) | H | Cl | F | CH₂CH₂CH₂CH₂N(Et)S(=O)₂CF₃ | O |
| A-5857 | *c*-Pr(2,2-F₂) | H | Cl | F | CH₂CH₂CH₂CH₂N(CH₂CN)S(=O)₂CF₃ | O |
| A-5858 | *c*-Pr(2,2-F₂) | H | Cl | F | CH₂CH₂CH₂CH₂N(CH₂OMe)S(=O)₂CF₃ | O |
| A-5859 | *c*-Pr(2,2-F₂) | H | Me | F | CH₂CH₂SCF₃ | O |
| A-5860 | *c*-Pr(2,2-F₂) | H | Me | F | CH₂CH₂CH₂SCF₃ | O |
| A-5861 | *c*-Pr(2,2-F₂) | H | Me | F | CH₂CH₂CH₂CH₂SCF₃ | O |
| A-5862 | *c*-Pr(2,2-F₂) | H | Me | F | CH₂CH₂CH₂CH₂CH₂SCF₃ | O |
| A-5863 | *c*-Pr(2,2-F₂) | H | Me | F | CH₂CH₂CH₂CH₂CH₂CH₂SCF₃ | O |
| A-5864 | *c*-Pr(2,2-F₂) | H | Me | F | CH₂CH₂CH₂CH₂CH₂CH₂CH₂SCF₃ | O |
| A-5865 | *c*-Pr(2,2-F₂) | H | Me | F | CH₂CH₂CH₂CH₂CH₂CH₂CH₂CH₂SCF₃ | O |
| A-5866 | *c*-Pr(2,2-F₂) | H | Me | F | CH₂CH₂NHS(=O)₂CF₃ | O |
| A-5867 | *c* -Pr(2,2-F₂) | H | Me | F | CH₂CH₂CH₂NHS(=O)₂CF₃ | O |
| A-5868 | *c*-Pr(2,2-F₂) | H | Me | F | CH₂CH₂CH₂CH₂NHS(=O)₂CF₃ | O |
| A-5869 | *c*-Pr(2,2-F₂) | H | Me | F | CH₂CH₂CH₂CH₂CH₂NHS(=O)₂CF₃ | O |
| A-5870 | *c*-Pr(2,2-F₂) | H | Me | F | CH₂CH₂N(Me)S(=O)₂CF₃ | O |
| A-5871 | *c*-Pr(2,2-F₂) | H | Me | F | CH₂CH₂N(Et)S(=O)₂CF₃ | O |
| A-5872 | *c*-Pr(2,2-F₂) | H | Me | F | CH₂CH₂N(CH₂CN)S(=O)₂CF₃ | O |
| A-5873 | *c*-Pr(2,2-F₂) | H | Me | F | CH₂CH₂N(CH₂OMe)S(=O)₂CF₃ | O |
| A-5874 | *c*-Pr(2,2-F₂) | H | Me | F | CH₂CH₂CH₂N(Me)S(=O)₂CF₃ | O |
| A-5875 | c -Pr(2,2-F₂) | H | Me | F | CH₂CH₂CH₂N(Et)S(=O)₂CF₃ | O |
| A-5876 | *c*-Pr(2,2-F₂) | H | Me | F | CH₂CH₂CH₂N(CH₂CN)S(=O)₂CF₃ | O |
| A-5877 | *c*-Pr(2,2-F₂) | H | Me | F | CH₂CH₂CH₂N(CH₂OMe)S(=O)₂CF₃ | O |
| A-5878 | *c*-Pr(2,2-F₂) | H | Me | F | CH₂CH₂CH₂CH₂N(Me)S(=O)₂CF₃ | O |
| A-5879 | *c*-Pr(2,2-F₂) | H | Me | F | CH₂CH₂CH₂CH₂N(Et)S(=O)₂CF₃ | O |
| A-5880 | *c*-Pr(2,2-F₂) | H | Me | F | CH₂CH₂CH₂CH₂N(CH₂CN)S(=O)₂CF₃ | O |
| A-5881 | *c*-Pr(2,2-F₂) | H | Me | F | CH₂CH₂CH₂CH₂N(CH₂OMe)S(=O)₂CF₃ | O |
| A-5882 | CH₂CH=CH₂ | H | Cl | F | CH₂CH₂SCF₃ | O |
| A-5883 | CH₂CH=CH₂ | H | Cl | F | CH₂CH₂CH₂SCF₃ | O |
| A-5884 | CH₂CH=CH₂ | H | Cl | F | CH₂CH₂CH₂CH₂SCF₃ | O |
| A-5885 | CH₂CH=CH₂ | H | Cl | F | CH₂CH₂CH₂CH₂CH₂SCF₃ | O |
| A-5886 | CH₂CH=CH₂ | H | Cl | F | CH₂CH₂CH₂CH₂CH₂CH₂SCF₃ | O |
| A-5887 | CH₂CH=CH₂ | H | Cl | F | CH₂CH₂CH₂CH₂CH₂CH₂CH₂SCF₃ | O |
| A-5888 | CH₂CH=CH₂ | H | Cl | F | CH₂CH₂CH₂CH₂CH₂CH₂CH₂CH₂SCF₃ | O |
| A-5889 | CH₂CH=CH₂ | H | Cl | F | CH₂CH₂NHS(=O)₂CF₃ | O |
| A-5890 | CH₂CH=CH₂ | H | Cl | F | CH₂CH₂CH₂NHS(=O)₂CF₃ | O |

**[Table 135]**

| Compound Number | R¹ | R² | R³ | R⁴ | R⁵ | A |
|---|---|---|---|---|---|---|
| A-5891 | CH₂CH=CH₂ | H | Cl | F | CH₂CH₂CH₂CH₂NHS(=O)₂CF₃ | O |
| A-5892 | CH₂CH=CH₂ | H | Cl | F | CH₂CH₂CH₂CH₂CH₂NHS(=O)₂CF₃ | O |
| A-5893 | CH₂CH=CH₂ | H | Cl | F | CH₂CH₂N(Me)S(=O)₂CF₃ | O |
| A-5894 | CH₂CH=CH₂ | H | Cl | F | CH₂CH₂N(Et)S(=O)₂CF₃ | O |
| A-5895 | CH₂CH=CH₂ | H | Cl | F | CH₂CH₂N(CH₂CN)S(=O)₂CF₃ | O |
| A-5896 | CH₂CH=CH₂ | H | Cl | F | CH₂CH₂N(CH₂OMe)S(=O)₂CF₃ | O |
| A-5897 | CH₂CH=CH₂ | H | Cl | F | CH₂CH₂CH₂N(Me)S(=O)₂CF₃ | O |
| A-5898 | CH₂CH=CH₂ | H | Cl | F | CH₂CH₂CH₂N(Et)S(=O)₂CF₃ | O |
| A-5899 | CH₂CH=CH₂ | H | Cl | F | CH₂CH₂CH₂N(CH₂CN)S(=O)₂CF₃ | O |
| A-5900 | CH₂CH=CH₂ | H | Cl | F | CH₂CH₂CH₂N(CH₂OMe)S(=O)₂CF₃ | O |
| A-5901 | CH₂CH=CH₂ | H | Cl | F | CH₂CH₂CH₂CH₂N(Me)S(=O)₂CF₃ | O |
| A-5902 | CH₂CH=CH₂ | H | Cl | F | CH₂CH₂CH₂CH₂N(Et)S(=O)₂CF₃ | O |
| A-5903 | CH₂CH=CH₂ | H | Cl | F | CH₂CH₂CH₂CH₂N(CH₂CN)S(=O)₂CF₃ | O |
| A-5904 | CH₂CH=CH₂ | H | Cl | F | CH₂CH₂CH₂CH₂N(CH₂OMe)S(=O)₂CF₃ | O |
| A-5905 | CH₂CH=CH₂ | H | Me | F | CH₂CH₂SCF₃ | O |
| A-5906 | CH₂CH=CH₂ | H | Me | F | CH₂CH₂CH₂SCF₃ | O |
| A-5907 | CH₂CH=CH₂ | H | Me | F | CH₂CH₂CH₂CH₂SCF₃ | O |
| A-5908 | CH₂CH=CH₂ | H | Me | F | CH₂CH₂CH₂CH₂CH₂SCF₃ | O |
| A-5909 | CH₂CH=CH₂ | H | Me | F | CH₂CH₂CH₂CH₂CH₂CH₂SCF₃ | O |
| A-5910 | CH₂CH=CH₂ | H | Me | F | CH₂CH₂CH₂CH₂CH₂CH₂CH₂SCF₃ | O |
| A-5911 | CH₂CH=CH₂ | H | Me | F | CH₂CH₂CH₂CH₂CH₂CH₂CH₂CH₂SCF₃ | O |
| A-5912 | CH₂CH=CH₂ | H | Me | F | CH₂CH₂NHS(=O)₂CF₃ | O |
| A-5913 | CH₂CH=CH₂ | H | Me | F | CH₂CH₂CH₂NHS(=O)₂CF₃ | O |
| A-5914 | CH₂CH=CH₂ | H | Me | F | CH₂CH₂CH₂CH₂NHS(=O)₂CF₃ | O |
| A-5915 | CH₂CH=CH₂ | H | Me | F | CH₂CH₂CH₂CH₂CH₂NHS(=O)₂CF₃ | O |
| A-5916 | CH₂CH=CH₂ | H | Me | F | CH₂CH₂N(Me)S(=O)₂CF₃ | O |
| A-5917 | CH₂CH=CH₂ | H | Me | F | CH₂CH₂N(Et)S(=O)₂CF₃ | O |
| A-5918 | CH₂CH=CH₂ | H | Me | F | CH₂CH₂N(CH₂CN)S(=O)₂CF₃ | O |
| A-5919 | CH₂CH=CH₂ | H | Me | F | CH₂CH₂N(CH₂OMe)S(=O)₂CF₃ | O |
| A-5920 | CH₂CH=CH₂ | H | Me | F | CH₂CH₂CH₂N(Me)S(=O)₂CF₃ | O |
| A-5921 | CH₂CH=CH₂ | H | Me | F | CH₂CH₂CH₂N(Et)S(=O)₂CF₃ | O |
| A-5922 | CH₂CH=CH₂ | H | Me | F | CH₂CH₂CH₂N(CH₂CN)S(=O)₂CF₃ | O |
| A-5923 | CH₂CH=CH₂ | H | Me | F | CH₂CH₂CH₂N(CH₂OMe)S(=O)₂CF₃ | O |
| A-5924 | CH₂CH=CH₂ | H | Me | F | CH₂CH₂CH₂CH₂N(Me)S(=O)₂CF₃ | O |
| A-5925 | CH₂CH=CH₂ | H | Me | F | CH₂CH₂CH₂CH₂N(Et)S(=O)₂CF₃ | O |
| A-5926 | CH₂CH=CH₂ | H | Me | F | CH₂CH₂CH₂CH₂N(CH₂CN)S(=O)₂CF₃ | O |
| A-5927 | CH₂CH=CH₂ | H | Me | F | CH₂CH₂CH₂CH₂N(CH₂OMe)S(=O)₂CF₃ | O |
| A-5928 | CH₂CH≡CH | H | Cl | F | CH₂CH₂SCF₃ | O |
| A-5929 | CH₂CH≡CH | H | Cl | F | CH₂CH₂CH₂SCF₃ | O |
| A-5930 | CH₂CH≡CH | H | Cl | F | CH₂CH₂CH₂CH₂SCF₃ | O |
| A-5931 | CH₂CH≡CH | H | Cl | F | CH₂CH₂CH₂CH₂CH₂SCF₃ | O |
| A-5932 | CH₂CH≡CH | H | Cl | F | CH₂CH₂CH₂CH₂CH₂CH₂SCF₃ | O |
| A-5933 | CH₂CH≡CH | H | Cl | F | CH₂CH₂CH₂CH₂CH₂CH₂CH₂SCF₃ | O |
| A-5934 | CH₂CH≡CH | H | Cl | F | CH₂CH₂CH₂CH₂CH₂CH₂CH₂CH₂SCF₃ | O |

**[Table 136]**

| Compound Number | R¹ | R² | R³ | R⁴ | R⁵ | A |
|---|---|---|---|---|---|---|
| A-5935 | CH₂CH≡CH | H | Cl | F | CH₂CH₂NHS(=O)₂CF₃ | O |
| A-5936 | CH₂CH≡CH | H | Cl | F | CH₂CH₂CH₂NHS(=O)₂CF₃ | O |
| A-5937 | CH₂CH≡CH | H | Cl | F | CH₂CH₂CH₂CH₂NHS(=O)₂CF₃ | O |
| A-5938 | CH₂CH≡CH | H | Cl | F | CH₂CH₂CH₂CH₂CH₂NHS(=O)₂CF₃ | O |
| A-5939 | CH₂CH≡CH | H | Cl | F | CH₂CH₂N(Me)S(=O)₂CF₃ | O |
| A-5940 | CH₂CH≡CH | H | Cl | F | CH₂CH₂N(Et)S(=O)₂CF₃ | O |
| A-5941 | CH₂CH≡CH | H | Cl | F | CH₂CH₂N(CH₂CN)S(=O)₂CF₃ | O |
| A-5942 | CH₂CH≡CH | H | Cl | F | CH₂CH₂N(CH₂OMe)S(=O)₂CF₃ | O |
| A-5943 | CH₂CH≡CH | H | Cl | F | CH₂CH₂CH₂N(Me)S(=O)₂CF₃ | O |
| A-5944 | CH₂CH≡CH | H | Cl | F | CH₂CH₂CH₂N(Et)S(=O)₂CF₃ | O |
| A-5945 | CH₂CH≡CH | H | Cl | F | CH₂CH₂CH₂N(CH₂CN)S(=O)₂CF₃ | O |
| A-5946 | CH₂CH≡CH | H | Cl | F | CH₂CH₂CH₂N(CH₂OMe)S(=O)₂CF₃ | O |
| A-5947 | CH₂CH≡CH | H | Cl | F | CH₂CH₂CH₂CH₂N(Me)S(=O)₂CF₃ | O |
| A-5948 | CH₂CH≡CH | H | Cl | F | CH₂CH₂CH₂CH₂N(Et)S(=O)₂CF₃ | O |
| A-5949 | CH₂CH≡CH | H | Cl | F | CH₂CH₂CH₂CH₂N(CH₂CN)S(=O)₂CF₃ | O |
| A-5950 | CH₂CH≡CH | H | Cl | F | CH₂CH₂CH₂CH₂N(CH₂OMe)S(=O)₂CF₃ | O |
| A-5951 | CH₂CH≡CH | H | Me | F | CH₂CH₂SCF₃ | O |
| A-5952 | CH₂CH≡CH | H | Me | F | CH₂CH₂CH₂SCF₃ | O |
| A-5953 | CH₂CH≡CH | H | Me | F | CH₂CH₂CH₂CH₂SCF₃ | O |
| A-5954 | CH₂CH≡CH | H | Me | F | CH₂CH₂CH₂CH₂CH₂SCF₃ | O |
| A-5955 | CH₂CH≡CH | H | Me | F | CH₂CH₂CH₂CH₂CH₂CH₂SCF₃ | O |
| A-5956 | CH₂CH≡CH | H | Me | F | CH₂CH₂CH₂CH₂CH₂CH₂CH₂SCF₃ | O |
| A-5957 | CH₂CH≡CH | H | Me | F | CH₂CH₂CH₂CH₂CH₂CH₂CH₂CH₂SCF₃ | O |
| A-5958 | CH₂CH≡CH | H | Me | F | CH₂CH₂NHS(=O)₂CF₃ | O |
| A-5959 | CH₂CH≡CH | H | Me | F | CH₂CH₂CH₂NHS(=O)₂CF₃ | O |
| A-5960 | CH₂CH≡CH | H | Me | F | CH₂CH₂CH₂CH₂NHS(=O)₂CF₃ | O |
| A-5961 | CH₂CH≡CH | H | Me | F | CH₂CH₂CH₂CH₂CH₂NHS(=O)₂CF₃ | O |
| A-5962 | CH₂CH≡CH | H | Me | F | CH₂CH₂N(Me)S(=O)₂CF₃ | O |
| A-5963 | CH₂CH≡CH | H | Me | F | CH₂CH₂N(Et)S(=O)₂CF₃ | O |
| A-5964 | CH₂CH≡CH | H | Me | F | CH₂CH₂N(CH₂CN)S(=O)₂CF₃ | O |
| A-5965 | CH₂CH≡CH | H | Me | F | CH₂CH₂N(CH₂OMe)S(=O)₂CF₃ | O |
| A-5966 | CH₂CH≡CH | H | Me | F | CH₂CH₂CH₂N(Me)S(=O)₂CF₃ | O |
| A-5967 | CH₂CH≡CH | H | Me | F | CH₂CH₂CH₂N(Et)S(=O)₂CF₃ | O |
| A-5968 | CH₂CH≡CH | H | Me | F | CH₂CH₂CH₂N(CH₂CN)S(=O)₂CF₃ | O |
| A-5969 | CH₂CH≡CH | H | Me | F | CH₂CH₂CH₂N(CH₂OMe)S(=O)₂CF₃ | O |
| A-5970 | CH₂CH≡CH | H | Me | F | CH₂CH₂CH₂CH₂N(Me)S(=O)₂CF₃ | O |
| A-5971 | CH₂CH≡CH | H | Me | F | CH₂CH₂CH₂CH₂N(Et)S(=O)₂CF₃ | O |
| A-5972 | CH₂CH≡CH | H | Me | F | CH₂CH₂CH₂CH₂N(CH₂CN)S(=O)₂CF₃ | O |
| A-5973 | CH₂CH≡CH | H | Me | F | CH₂CH₂CH₂CH₂N(CH₂OMe)S(=O)₂CF₃ | O |
| A-5974 | CH₂CHF₂ | H | Cl | F | CH₂CH₂SCF₃ | O |
| A-5975 | CH₂CHF₂ | H | Cl | F | CH₂CH₂CH₂SCF₃ | O |
| A-5976 | CH₂CHF₂ | H | Cl | F | CH₂CH₂CH₂CH₂SCF₃ | O |
| A-5977 | CH₂CHF₂ | H | Cl | F | CH₂CH₂CH₂CH₂CH₂SCF₃ | O |
| A-5978 | CH₂CHF₂ | H | Cl | F | CH₂CH₂CH₂CH₂CH₂CH₂SCF₃ | O |

**[Table 137]**

| Compound Number | R¹ | R² | R³ | R⁴ | R⁵ | A |
|---|---|---|---|---|---|---|
| A-5979 | CH₂CHF₂ | H | Cl | F | CH₂CH₂CH₂CH₂CH₂CH₂CH₂SCF₃ | O |
| A-5980 | CH₂CHF₂ | H | Cl | F | CH₂CH₂CH₂CH₂CH₂CH₂CH₂CH₂SCF₃ | O |
| A-5981 | CH₂CHF₂ | H | Cl | F | CH₂CH₂NHS(=O)₂CF₃ | O |
| A-5982 | CH₂CHF₂ | H | Cl | F | CH₂CH₂CH₂NHS(=O)₂CF₃ | O |
| A-5983 | CH₂CHF₂ | H | Cl | F | CH₂CH₂CH₂CH₂NHS(=O)₂CF₃ | O |
| A-5984 | CH₂CHF₂ | H | Cl | F | CH₂CH₂CH₂CH₂CH₂NHS(=O)₂CF₃ | O |
| A-5985 | CH₂CHF₂ | H | Cl | F | CH₂CH₂N(Me)S(=O)₂CF₃ | O |
| A-5986 | CH₂CHF₂ | H | Cl | F | CH₂CH₂N(Et)S(=O)₂CF₃ | O |
| A-5987 | CH₂CHF₂ | H | Cl | F | CH₂CH₂N(CH₂CN)S(=O)₂CF₃ | O |
| A-5988 | CH₂CHF₂ | H | Cl | F | CH₂CH₂N(CH₂OMe)S(=O)₂CF₃ | O |
| A-5989 | CH₂CHF₂ | H | Cl | F | CH₂CH₂CH₂N(Me)S(=O)₂CF₃ | O |
| A-5990 | CH₂CHF₂ | H | Cl | F | CH₂CH₂CH₂N(Et)S(=O)₂CF₃ | O |
| A-5991 | CH₂CHF₂ | H | Cl | F | CH₂CH₂CH₂N(CH₂CN)S(=O)₂CF₃ | O |
| A-5992 | CH₂CHF₂ | H | Cl | F | CH₂CH₂CH₂N(CH₂OMe)S(=O)₂CF₃ | O |
| A-5993 | CH₂CHF₂ | H | Cl | F | CH₂CH₂CH₂CH₂N(Me)S(=O)₂CF₃ | O |
| A-5994 | CH₂CHF₂ | H | Cl | F | CH₂CH₂CH₂CH₂N(Et)S(=O)₂CF₃ | O |
| A-5995 | CH₂CHF₂ | H | Cl | F | CH₂CH₂CH₂CH₂N(CH₂CN)S(=O)₂CF₃ | O |
| A-5996 | CH₂CHF₂ | H | Cl | F | CH₂CH₂CH₂CH₂N(CH₂OMe)S(=O)₂CF₃ | O |
| A-5997 | CH₂CHF₂ | H | Me | F | CH₂CH₂SCF₃ | O |
| A-5998 | CH₂CHF₂ | H | Me | F | CH₂CH₂CH₂SCF₃ | O |
| A-5999 | CH₂CHF₂ | H | Me | F | CH₂CH₂CH₂CH₂SCF₃ | O |
| A-6000 | CH₂CHF₂ | H | Me | F | CH₂CH₂CH₂CH₂CH₂SCF₃ | O |
| A-6001 | CH₂CHF₂ | H | Me | F | CH₂CH₂CH₂CH₂CH₂CH₂SCF₃ | O |
| A-6002 | CH₂CHF₂ | H | Me | F | CH₂CH₂CH₂CH₂CH₂CH₂CH₂SCF₃ | O |
| A-6003 | CH₂CHF₂ | H | Me | F | CH₂CH₂CH₂CH₂CH₂CH₂CH₂CH₂SCF₃ | O |
| A-6004 | CH₂CHF₂ | H | Me | F | CH₂CH₂NHS(=O)₂CF₃ | O |
| A-6005 | CH₂CHF₂ | H | Me | F | CH₂CH₂CH₂NHS(=O)₂CF₃ | O |
| A-6006 | CH₂CHF₂ | H | Me | F | CH₂CH₂CH₂CH₂NHS(=O)₂CF₃ | O |
| A-6007 | CH₂CHF₂ | H | Me | F | CH₂CH₂CH₂CH₂CH₂NHS(=O)₂CF₃ | O |
| A-6008 | CH₂CHF₂ | H | Me | F | CH₂CH₂N(Me)S(=O)₂CF₃ | O |
| A-6009 | CH₂CHF₂ | H | Me | F | CH₂CH₂N(Et)S(=O)₂CF₃ | O |
| A-6010 | CH₂CHF₂ | H | Me | F | CH₂CH₂N(CH₂CN)S(=O)₂CF₃ | O |
| A-6011 | CH₂CHF₂ | H | Me | F | CH₂CH₂N(CH₂OMe)S(=O)₂CF₃ | O |
| A-6012 | CH₂CHF₂ | H | Me | F | CH₂CH₂CH₂N(Me)S(=O)₂CF₃ | O |
| A-6013 | CH₂CHF₂ | H | Me | F | CH₂CH₂CH₂N(Et)S(=O)₂CF₃ | O |
| A-6014 | CH₂CHF₂ | H | Me | F | CH₂CH₂CH₂N(CH₂CN)S(=O)₂CF₃ | O |
| A-6015 | CH₂CHF₂ | H | Me | F | CH₂CH₂CH₂N(CH₂OMe)S(=O)₂CF₃ | O |
| A-6016 | CH₂CHF₂ | H | Me | F | CH₂CH₂CH₂CH₂N(Me)S(=O)₂CF₃ | O |
| A-6017 | CH₂CHF₂ | H | Me | F | CH₂CH₂CH₂CH₂N(Et)S(=O)₂CF₃ | O |
| A-6018 | CH₂CHF₂ | H | Me | F | CH₂CH₂CH₂CH₂N(CH₂CN)S(=O)₂CF₃ | O |
| A-6019 | CH₂CHF₂ | H | Me | F | CH₂CH₂CH₂CH₂N(CH₂OMe)S(=O)₂CF₃ | O |
| A-6020 | OMe | H | Cl | F | CH₂CH₂SCF₃ | O |
| A-6021 | OMe | H | Cl | F | CH₂CH₂CH₂SCF₃ | O |
| A-6022 | OMe | H | Cl | F | CH₂CH₂CH₂CH₂SCF₃ | O |

**[Table 138]**

| Compound Number | R¹ | R² | R³ | R⁴ | R⁵ | A |
|---|---|---|---|---|---|---|
| A-6023 | OMe | H | Cl | F | CH₂CH₂CH₂CH₂CH₂SCF₃ | O |
| A-6024 | OMe | H | Cl | F | CH₂CH₂CH₂CH₂CH₂CH₂SCF₃ | O |
| A-6025 | OMe | H | Cl | F | CH₂CH₂CH₂CH₂CH₂CH₂CH₂SCF₃ | O |
| A-6026 | OMe | H | Cl | F | CH₂CH₂CH₂CH₂CH₂CH₂CH₂CH₂SCF₃ | O |
| A-6027 | OMe | H | Cl | F | CH₂CH₂NHS(=O)₂CF₃ | O |
| A-6028 | OMe | H | Cl | F | CH₂CH₂CH₂NHS(=O)₂CF₃ | O |
| A-6029 | OMe | H | Cl | F | CH₂CH₂CH₂CH₂NHS(=O)₂CF₃ | O |
| A-6030 | OMe | H | Cl | F | CH₂CH₂CH₂CH₂CH₂NHS(=O)₂CF₃ | O |
| A-6031 | OMe | H | Cl | F | CH₂CH₂N(Me)S(=O)₂CF₃ | O |
| A-6032 | OMe | H | Cl | F | CH₂CH₂N(Et)S(=O)₂CF₃ | O |
| A-6033 | OMe | H | Cl | F | CH₂CH₂N(CH₂CN)S(=O)₂CF₃ | O |
| A-6034 | OMe | H | Cl | F | CH₂CH₂N(CH₂OMe)S(=O)₂CF₃ | O |
| A-6035 | OMe | H | Cl | F | CH₂CH₂CH₂N(Me)S(=O)₂CF₃ | O |
| A-6036 | OMe | H | Cl | F | CH₂CH₂CH₂N(Et)S(=O)₂CF₃ | O |
| A-6037 | OMe | H | Cl | F | CH₂CH₂CH₂N(CH₂CN)S(=O)₂CF₃ | O |
| A-6038 | OMe | H | Cl | F | CH₂CH₂CH₂N(CH₂OMe)S(=O)₂CF₃ | O |
| A-6039 | OMe | H | Cl | F | CH₂CH₂CH₂CH₂N(Me)S(=O)₂CF₃ | O |
| A-6040 | OMe | H | Cl | F | CH₂CH₂CH₂CH₂N(Et)S(=O)₂CF₃ | O |
| A-6041 | OMe | H | Cl | F | CH₂CH₂CH₂CH₂N(CH₂CN)S(=O)₂CF₃ | O |
| A-6042 | OMe | H | Cl | F | CH₂CH₂CH₂CH₂N(CH₂OMe)S(=O)₂CF₃ | O |
| A-6043 | OMe | H | Me | F | CH₂CH₂SCF₃ | O |
| A-6044 | OMe | H | Me | F | CH₂CH₂CH₂SCF₃ | O |
| A-6045 | OMe | H | Me | F | CH₂CH₂CH₂CH₂SCF₃ | O |
| A-6046 | OMe | H | Me | F | CH₂CH₂CH₂CH₂CH₂SCF₃ | O |
| A-6047 | OMe | H | Me | F | CH₂CH₂CH₂CH₂CH₂CH₂SCF₃ | O |
| A-6048 | OMe | H | Me | F | CH₂CH₂CH₂CH₂CH₂CH₂CH₂SCF₃ | O |
| A-6049 | OMe | H | Me | F | CH₂CH₂CH₂CH₂CH₂CH₂CH₂CH₂SCF₃ | O |
| A-6050 | OMe | H | Me | F | CH₂CH₂NHS(=O)₂CF₃ | O |
| A-6051 | OMe | H | Me | F | CH₂CH₂CH₂NHS(=O)₂CF₃ | O |
| A-6052 | OMe | H | Me | F | CH₂CH₂CH₂CH₂NHS(=O)₂CF₃ | O |
| A-6053 | OMe | H | Me | F | CH₂CH₂CH₂CH₂CH₂NHS(=O)₂CF₃ | O |
| A-6054 | OMe | H | Me | F | CH₂CH₂N(Me)S(=O)₂CF₃ | O |
| A-6055 | OMe | H | Me | F | CH₂CH₂N(Et)S(=O)₂CF₃ | O |
| A-6056 | OMe | H | Me | F | CH₂CH₂N(CH₂CN)S(=O)₂CF₃ | O |
| A-6057 | OMe | H | Me | F | CH₂CH₂N(CH₂OMe)S(=O)₂CF₃ | O |
| A-6058 | OMe | H | Me | F | CH₂CH₂CH₂N(Me)S(=O)₂CF₃ | O |
| A-6059 | OMe | H | Me | F | CH₂CH₂CH₂N(Et)S(=O)₂CF₃ | O |
| A-6060 | OMe | H | Me | F | CH₂CH₂CH₂N(CH₂CN)S(=O)₂CF₃ | O |
| A-6061 | OMe | H | Me | F | CH₂CH₂CH₂N(CH₂OMe)S(=O)₂CF₃ | O |
| A-6062 | OMe | H | Me | F | CH₂CH₂CH₂CH₂N(Me)S(=O)₂CF₃ | O |
| A-6063 | OMe | H | Me | F | CH₂CH₂CH₂CH₂N(Et)S(=O)₂CF₃ | O |
| A-6064 | OMe | H | Me | F | CH₂CH₂CH₂CH₂N(CH₂CN)S(=O)₂CF₃ | O |
| A-6065 | OMe | H | Me | F | CH₂CH₂CH₂CH₂N(CH₂OMe)S(=O)₂CF₃ | O |
| A-6066 | OEt | H | Cl | F | CH₂CH₂SCF₃ | O |

**[Table 139]**

| Compound Number | R¹ | R² | R³ | R⁴ | R⁵ | A |
|---|---|---|---|---|---|---|
| A-6067 | OEt | H | Cl | F | CH₂CH₂CH₂SCF₃ | O |
| A-6068 | OEt | H | Cl | F | CH₂CH₂CH₂CH₂SCF₃ | O |
| A-6069 | OEt | H | Cl | F | CH₂CH₂CH₂CH₂CH₂SCF₃ | O |
| A-6070 | OEt | H | Cl | F | CH₂CH₂CH₂CH₂CH₂CH₂SCF₃ | O |
| A-6071 | OEt | H | Cl | F | CH₂CH₂CH₂CH₂CH₂CH₂CH₂SCF₃ | O |
| A-6072 | OEt | H | Cl | F | CH₂CH₂CH₂CH₂CH₂CH₂CH₂CH₂SCF₃ | O |
| A-6073 | OEt | H | Cl | F | CH₂CH₂NHS(=O)₂CF₃ | O |
| A-6074 | OEt | H | Cl | F | CH₂CH₂CH₂NHS(=O)₂CF₃ | O |
| A-6075 | OEt | H | Cl | F | CH₂CH₂CH₂CH₂NHS(=O)₂CF₃ | O |
| A-6076 | OEt | H | Cl | F | CH₂CH₂CH₂CH₂CH₂NHS(=O)₂CF₃ | O |
| A-6077 | OEt | H | Cl | F | CH₂CH₂N(Me)S(=O)₂CF₃ | O |
| A-6078 | OEt | H | Cl | F | CH₂CH₂N(Et)S(=O)₂CF₃ | O |
| A-6079 | OEt | H | Cl | F | CH₂CH₂N(CH₂CN)S(=O)₂CF₃ | O |
| A-6080 | OEt | H | Cl | F | CH₂CH₂N(CH₂OMe)S(=O)₂CF₃ | O |
| A-6081 | OEt | H | Cl | F | CH₂CH₂CH₂N(Me)S(=O)₂CF₃ | O |
| A-6082 | OEt | H | Cl | F | CH₂CH₂CH₂N(Et)S(=O)₂CF₃ | O |
| A-6083 | OEt | H | Cl | F | CH₂CH₂CH₂N(CH₂CN)S(=O)₂CF₃ | O |
| A-6084 | OEt | H | Cl | F | CH₂CH₂CH₂N(CH₂OMe)S(=O)₂CF₃ | O |
| A-6085 | OEt | H | Cl | F | CH₂CH₂CH₂CH₂N(Me)S(=O)₂CF₃ | O |
| A-6086 | OEt | H | Cl | F | CH₂CH₂CH₂CH₂N(Et)S(=O)₂CF₃ | O |
| A-6087 | OEt | H | Cl | F | CH₂CH₂CH₂CH₂N(CH₂CN)S(=O)₂CF₃ | O |
| A-6088 | OEt | H | Cl | F | CH₂CH₂CH₂CH₂N(CH₂OMe)S(=O)₂CF₃ | O |
| A-6089 | OEt | H | Me | F | CH₂CH₂SCF₃ | O |
| A-6090 | OEt | H | Me | F | CH₂CH₂CH₂SCF₃ | O |
| A-6091 | OEt | H | Me | F | CH₂CH₂CH₂CH₂SCF₃ | O |
| A-6092 | OEt | H | Me | F | CH₂CH₂CH₂CH₂CH₂SCF₃ | O |
| A-6093 | OEt | H | Me | F | CH₂CH₂CH₂CH₂CH₂CH₂SCF₃ | O |
| A-6094 | OEt | H | Me | F | CH₂CH₂CH₂CH₂CH₂CH₂CH₂SCF₃ | O |
| A-6095 | OEt | H | Me | F | CH₂CH₂CH₂CH₂CH₂CH₂CH₂CH₂SCF₃ | O |
| A-6096 | OEt | H | Me | F | CH₂CH₂NHS(=O)₂CF₃ | O |
| A-6097 | OEt | H | Me | F | CH₂CH₂CH₂NHS(=O)₂CF₃ | O |
| A-6098 | OEt | H | Me | F | CH₂CH₂CH₂CH₂NHS(=O)₂CF₃ | O |
| A-6099 | OEt | H | Me | F | CH₂CH₂CH₂CH₂CH₂NHS(=O)₂CF₃ | O |
| A-6100 | OEt | H | Me | F | CH₂CH₂N(Me)S(=O)₂CF₃ | O |
| A-6101 | OEt | H | Me | F | CH₂CH₂N(Et)S(=O)₂CF₃ | O |
| A-6102 | OEt | H | Me | F | CH₂CH₂N(CH₂CN)S(=O)₂CF₃ | O |
| A-6103 | OEt | H | Me | F | CH₂CH₂N(CH₂OMe)S(=O)₂CF₃ | O |
| A-6104 | OEt | H | Me | F | CH₂CH₂CH₂N(Me)S(=O)₂CF₃ | O |
| A-6105 | OEt | H | Me | F | CH₂CH₂CH₂N(Et)S(=O)₂CF₃ | O |
| A-6106 | OEt | H | Me | F | CH₂CH₂CH₂N(CH₂CN)S(=O)₂CF₃ | O |
| A-6107 | OEt | H | Me | F | CH₂CH₂CH₂N(CH₂OMe)S(=O)₂CF₃ | O |
| A-6108 | OEt | H | Me | F | CH₂CH₂CH₂CH₂N(Me)S(=O)₂CF₃ | O |
| A-6109 | OEt | H | Me | F | CH₂CH₂CH₂CH₂N(Et)S(=O)₂CF₃ | O |
| A-6110 | OEt | H | Me | F | CH₂CH₂CH₂CH₂N(CH₂CN)S(=O)₂CF₃ | O |

**[Table 140]**

| Compound Number | R¹ | R² | R³ | R⁴ | R⁵ | A |
|---|---|---|---|---|---|---|
| A-6111 | OEt | H | Me | F | CH₂CH₂CH₂CH₂N(CH₂OMe)S(=O)₂CF₃ | O |
| A-6112 | OCH₂CF₃ | H | Cl | F | CH₂CH₂SCF₃ | O |
| A-6113 | OCH₂CF₃ | H | Cl | F | CH₂CH₂CH₂SCF₃ | O |
| A-6114 | OCH₂CF₃ | H | Cl | F | CH₂CH₂CH₂CH₂SCF₃ | O |
| A-6115 | OCH₂CF₃ | H | Cl | F | CH₂CH₂CH₂CH₂CH₂SCF₃ | O |
| A-6116 | OCH₂CF₃ | H | Cl | F | CH₂CH₂CH₂CH₂CH₂CH₂SCF₃ | O |
| A-6117 | OCH₂CF₃ | H | Cl | F | CH₂CH₂CH₂CH₂CH₂CH₂CH₂SCF₃ | O |
| A-6118 | OCH₂CF₃ | H | Cl | F | CH₂CH₂CH₂CH₂CH₂CH₂CH₂CH₂SCF₃ | O |
| A-6119 | OCH₂CF₃ | H | Cl | F | CH₂CH₂NHS(=O)₂CF₃ | O |
| A-6120 | OCH₂CF₃ | H | Cl | F | CH₂CH₂CH₂NHS(=O)₂CF₃ | O |
| A-6121 | OCH₂CF₃ | H | Cl | F | CH₂CH₂CH₂CH₂NHS(=O)₂CF₃ | O |
| A-6122 | OCH₂CF₃ | H | Cl | F | CH₂CH₂CH₂CH₂CH₂NHS(=O)₂CF₃ | O |
| A-6123 | OCH₂CF₃ | H | Cl | F | CH₂CH₂N(Me)S(=O)₂CF₃ | O |
| A-6124 | OCH₂CF₃ | H | Cl | F | CH₂CH₂N(Et)S(=O)₂CF₃ | O |
| A-6125 | OCH₂CF₃ | H | Cl | F | CH₂CH₂N(CH₂CN)S(=O)₂CF₃ | O |
| A-6126 | OCH₂CF₃ | H | Cl | F | CH₂CH₂N(CH₂OMe)S(=O)₂CF₃ | O |
| A-6127 | OCH₂CF₃ | H | Cl | F | CH₂CH₂CH₂N(Me)S(=O)₂CF₃ | O |
| A-6128 | OCH₂CF₃ | H | Cl | F | CH₂CH₂CH₂N(Et)S(=O)₂CF₃ | O |
| A-6129 | OCH₂CF₃ | H | Cl | F | CH₂CH₂CH₂N(CH₂CN)S(=O)₂CF₃ | O |
| A-6130 | OCH₂CF₃ | H | Cl | F | CH₂CH₂CH₂N(CH₂OMe)S(=O)₂CF₃ | O |
| A-6131 | OCH₂CF₃ | H | Cl | F | CH₂CH₂CH₂CH₂N(Me)S(=O)₂CF₃ | O |
| A-6132 | OCH₂CF₃ | H | Cl | F | CH₂CH₂CH₂CH₂N(Et)S(=O)₂CF₃ | O |
| A-6133 | OCH₂CF₃ | H | Cl | F | CH₂CH₂CH₂CH₂N(CH₂CN)S(=O)₂CF₃ | O |
| A-6134 | OCH₂CF₃ | H | Cl | F | CH₂CH₂CH₂CH₂N(CH₂OMe)S(=O)₂CF₃ | O |
| A-6135 | OCH₂CF₃ | H | Me | F | CH₂CH₂SCF₃ | O |
| A-6136 | OCH₂CF₃ | H | Me | F | CH₂CH₂CH₂SCF₃ | O |
| A-6137 | OCH₂CF₃ | H | Me | F | CH₂CH₂CH₂CH₂SCF₃ | O |
| A-6138 | OCH₂CF₃ | H | Me | F | CH₂CH₂CH₂CH₂CH₂SCF₃ | O |
| A-6139 | OCH₂CF₃ | H | Me | F | CH₂CH₂CH₂CH₂CH₂CH₂SCF₃ | O |
| A-6140 | OCH₂CF₃ | H | Me | F | CH₂CH₂CH₂CH₂CH₂CH₂CH₂SCF₃ | O |
| A-6141 | OCH₂CF₃ | H | Me | F | CH₂CH₂CH₂CH₂CH₂CH₂CH₂CH₂SCF₃ | O |
| A-6142 | OCH₂CF₃ | H | Me | F | CH₂CH₂NHS(=O)₂CF₃ | O |
| A-6143 | OCH₂CF₃ | H | Me | F | CH₂CH₂CH₂NHS(=O)₂CF₃ | O |
| A-6144 | OCH₂CF₃ | H | Me | F | CH₂CH₂CH₂CH₂NHS(=O)₂CF₃ | O |
| A-6145 | OCH₂CF₃ | H | Me | F | CH₂CH₂CH₂CH₂CH₂NHS(=O)₂CF₃ | O |
| A-6146 | OCH₂CF₃ | H | Me | F | CH₂CH₂N(Me)S(=O)₂CF₃ | O |
| A-6147 | OCH₂CF₃ | H | Me | F | CH₂CH₂N(Et)S(=O)₂CF₃ | O |
| A-6148 | OCH₂CF₃ | H | Me | F | CH₂CH₂N(CH₂CN)S(=O)₂CF₃ | O |
| A-6149 | OCH₂CF₃ | H | Me | F | CH₂CH₂N(CH₂OMe)S(=O)₂CF₃ | O |
| A-6150 | OCH₂CF₃ | H | Me | F | CH₂CH₂CH₂N(Me)S(=O)₂CF₃ | O |
| A-6151 | OCH₂CF₃ | H | Me | F | CH₂CH₂CH₂N(Et)S(=O)₂CF₃ | O |
| A-6152 | OCH₂CF₃ | H | Me | F | CH₂CH₂CH₂N(CH₂CN)S(=O)₂CF₃ | O |
| A-6153 | OCH₂CF₃ | H | Me | F | CH₂CH₂CH₂N(CH₂OMe)S(=O)₂CF₃ | O |
| A-6154 | OCH₂CF₃ | H | Me | F | CH₂CH₂CH₂CH₂N(Me)S(=O)₂CF₃ | O |

**[Table 141]**

| Compound Number | R¹ | R² | R³ | R⁴ | R⁵ | A |
|---|---|---|---|---|---|---|
| A-6155 | OCH₂CF₃ | H | Me | F | CH₂CH₂CH₂CH₂N(Et)S(=O)₂CF₃ | O |
| A-6156 | OCH₂CF₃ | H | Me | F | CH₂CH₂CH₂CH₂N(CH₂CN)S(=O)₂CF₃ | O |
| A-6157 | OCH₂CF₃ | H | Me | F | CH₂CH₂CH₂CH₂N(CH₂OMe)S(=O)₂CF₃ | O |
| A-6158 | CH₂(*c*-Pr) | H | Cl | F | CH₂CH₂SCF₃ | O |
| A-6159 | CH₂(*c*-Pr) | H | Cl | F | CH₂CH₂CH₂SCF₃ | O |
| A-6160 | CH₂(*c*-Pr) | H | Cl | F | CH₂CH₂CH₂CH₂SCF₃ | O |
| A-6161 | CH₂(*c*-Pr) | H | Cl | F | CH₂CH₂CH₂CH₂CH₂SCF₃ | O |
| A-6162 | CH₂(*c*-Pr) | H | Cl | F | CH₂CH₂CH₂CH₂CH₂CH₂SCF₃ | O |
| A-6163 | CH₂(*c*-Pr) | H | Cl | F | CH₂CH₂CH₂CH₂CH₂CH₂CH₂SCF₃ | O |
| A-6164 | CH₂(*c*-Pr) | H | Cl | F | CH₂CH₂CH₂CH₂CH₂CH₂CH₂CH₂SCF₃ | O |
| A-6165 | CH₂(*c*-Pr) | H | Cl | F | CH₂CH₂NHS(=O)₂CF₃ | O |
| A-6166 | CH₂(*c*-Pr) | H | Cl | F | CH₂CH₂CH₂NHS(=O)₂CF₃ | O |
| A-6167 | CH₂(*c*-Pr) | H | Cl | F | CH₂CH₂CH₂CH₂NHS(=O)₂CF₃ | O |
| A-6168 | CH₂(*c*-Pr) | H | Cl | F | CH₂CH₂CH₂CH₂CH₂NHS(=O)₂CF₃ | O |
| A-6169 | CH₂(*c*-Pr) | H | Cl | F | CH₂CH₂N(Me)S(=O)₂CF₃ | O |
| A-6170 | CH₂(*c*-Pr) | H | Cl | F | CH₂CH₂N(Et)S(=O)₂CF₃ | O |
| A-6171 | CH₂(*c*-Pr) | H | Cl | F | CH₂CH₂N(CH₂CN)S(=O)₂CF₃ | O |
| A-6172 | CH₂(*c*-Pr) | H | Cl | F | CH₂CH₂N(CH₂OMe)S(=O)₂CF₃ | O |
| A-6173 | CH₂(*c*-Pr) | H | Cl | F | CH₂CH₂CH₂N(Me)S(=O)₂CF₃ | O |
| A-6174 | CH₂(*c*-Pr) | H | Cl | F | CH₂CH₂CH₂N(Et)S(=O)₂CF₃ | O |
| A-6175 | CH₂(*c*-Pr) | H | Cl | F | CH₂CH₂CH₂N(CH₂CN)S(=O)₂CF₃ | O |
| A-6176 | CH₂(*c*-Pr) | H | Cl | F | CH₂CH₂CH₂N(CH₂OMe)S(=O)₂CF₃ | O |
| A-6177 | CH₂(*c*-Pr) | H | Cl | F | CH₂CH₂CH₂CH₂N(Me)S(=O)₂CF₃ | O |
| A-6178 | CH₂(*c*-Pr) | H | Cl | F | CH₂CH₂CH₂CH₂N(Et)S(=O)₂CF₃ | O |
| A-6179 | CH₂(*c*-Pr) | H | Cl | F | CH₂CH₂CH₂CH₂N(CH₂CN)S(=O)₂CF₃ | O |
| A-6180 | CH₂(*c*-Pr) | H | Cl | F | CH₂CH₂CH₂CH₂N(CH₂OMe)S(=O)₂CF₃ | O |
| A-6181 | CH₂(*c*-Pr) | H | Me | F | CH₂CH₂SCF₃ | O |
| A-6182 | CH₂(*c*-Pr) | H | Me | F | CH₂CH₂CH₂SCF₃ | O |
| A-6183 | CH₂(*c*-Pr) | H | Me | F | CH₂CH₂CH₂CH₂SCF₃ | O |
| A-6184 | CH₂(*c*-Pr) | H | Me | F | CH₂CH₂CH₂CH₂CH₂SCF₃ | O |
| A-6185 | CH₂(*c*-Pr) | H | Me | F | CH₂CH₂CH₂CH₂CH₂CH₂SCF₃ | O |
| A-6186 | CH₂(*c*-Pr) | H | Me | F | CH₂CH₂CH₂CH₂CH₂CH₂CH₂SCF₃ | O |
| A-6187 | CH₂(*c*-Pr) | H | Me | F | CH₂CH₂CH₂CH₂CH₂CH₂CH₂CH₂SCF₃ | O |
| A-6188 | CH₂(*c*-Pr) | H | Me | F | CH₂CH₂NHS(=O)₂CF₃ | O |
| A-6189 | CH₂(*c*-Pr) | H | Me | F | CH₂CH₂CH₂NHS(=O)₂CF₃ | O |
| A-6190 | CH₂(*c*-Pr) | H | Me | F | CH₂CH₂CH₂CH₂NHS(=O)₂CF₃ | O |
| A-6191 | CH₂(*c*-Pr) | H | Me | F | CH₂CH₂CH₂CH₂CH₂NHS(=O)₂CF₃ | O |
| A-6192 | CH₂(*c*-Pr) | H | Me | F | CH₂CH₂N(Me)S(=O)₂CF₃ | O |
| A-6193 | CH₂(*c*-Pr) | H | Me | F | CH₂CH₂N(Et)S(=O)₂CF₃ | O |
| A-6194 | CH₂(*c*-Pr) | H | Me | F | CH₂CH₂N(CH₂CN)S(=O)₂CF₃ | O |
| A-6195 | CH₂(*c*-Pr) | H | Me | F | CH₂CH₂N(CH₂OMe)S(=O)₂CF₃ | O |
| A-6196 | CH₂(*c*-Pr) | H | Me | F | CH₂CH₂CH₂N(Me)S(=O)₂CF₃ | O |
| A-6197 | CH₂(*c*-Pr) | H | Me | F | CH₂CH₂CH₂N(Et)S(=O)₂CF₃ | O |
| A-6198 | CH₂(*c*-Pr) | H | Me | F | CH₂CH₂CH₂N(CH₂CN)S(=O)₂CF₃ | O |

**[Table 142]**

| Compound Number | R¹ | R² | R³ | R⁴ | R⁵ | A |
|---|---|---|---|---|---|---|
| A-6199 | CH₂(*c*-Pr) | H | Me | F | CH₂CH₂CH₂N(CH₂OMe)S(=O)₂CF₃ | O |
| A-6200 | CH₂(*c*-Pr) | H | Me | F | CH₂CH₂CH₂CH₂N(Me)S(=O)₂CF₃ | O |
| A-6201 | CH₂(*c*-Pr) | H | Me | F | CH₂CH₂CH₂CH₂N(Et)S(=O)₂CF₃ | O |
| A-6202 | CH₂(*c*-Pr) | H | Me | F | CH₂CH₂CH₂CH₂N(CH₂CN)S(=O)₂CF₃ | O |
| A-6203 | CH₂(*c*-Pr) | H | Me | F | CH₂CH₂CH₂CH₂N(CH₂OMe)S(=O)₂CF₃ | O |
| A-6204 | Ph | H | Cl | F | CH₂CH₂SCF₃ | O |
| A-6205 | Ph | H | Cl | F | CH₂CH₂CH₂SCF₃ | O |
| A-6206 | Ph | H | Cl | F | CH₂CH₂CH₂CH₂SCF₃ | O |
| A-6207 | Ph | H | Cl | F | CH₂CH₂CH₂CH₂CH₂SCF₃ | O |
| A-6208 | Ph | H | Cl | F | CH₂CH₂CH₂CH₂CH₂CH₂SCF₃ | O |
| A-6209 | Ph | H | Cl | F | CH₂CH₂CH₂CH₂CH₂CH₂CH₂SCF₃ | O |
| A-6210 | Ph | H | Cl | F | CH₂CH₂CH₂CH₂CH₂CH₂CH₂CH₂SCF₃ | O |
| A-6211 | Ph | H | Cl | F | CH₂CH₂NHS(=O)₂CF₃ | O |
| A-6212 | Ph | H | Cl | F | CH₂CH₂CH₂NHS(=O)₂CF₃ | O |
| A-6213 | Ph | H | Cl | F | CH₂CH₂CH₂CH₂NHS(=O)₂CF₃ | O |
| A-6214 | Ph | H | Cl | F | CH₂CH₂CH₂CH₂CH₂NHS(=O)₂CF₃ | O |
| A-6215 | Ph | H | Cl | F | CH₂CH₂N(Me)S(=O)₂CF₃ | O |
| A-6216 | Ph | H | Cl | F | CH₂CH₂N(Et)S(=O)₂CF₃ | O |
| A-6217 | Ph | H | Cl | F | CH₂CH₂N(CH₂CN)S(=O)₂CF₃ | O |
| A-6218 | Ph | H | Cl | F | CH₂CH₂N(CH₂OMe)S(=O)₂CF₃ | O |
| A-6219 | Ph | H | Cl | F | CH₂CH₂CH₂N(Me)S(=O)₂CF₃ | O |
| A-6220 | Ph | H | Cl | F | CH₂CH₂CH₂N(Et)S(=O)₂CF₃ | O |
| A-6221 | Ph | H | Cl | F | CH₂CH₂CH₂N(CH₂CN)S(=O)₂CF₃ | O |
| A-6222 | Ph | H | Cl | F | CH₂CH₂CH₂N(CH₂OMe)S(=O)₂CF₃ | O |
| A-6223 | Ph | H | Cl | F | CH₂CH₂CH₂CH₂N(Me)S(=O)₂CF₃ | O |
| A-6224 | Ph | H | Cl | F | CH₂CH₂CH₂CH₂N(Et)S(=O)₂CF₃ | O |
| A-6225 | Ph | H | Cl | F | CH₂CH₂CH₂CH₂N(CH₂CN)S(=O)₂CF₃ | O |
| A-6226 | Ph | H | Cl | F | CH₂CH₂CH₂CH₂N(CH₂OMe)S(=O)₂CF₃ | O |
| A-6227 | Ph | H | Me | F | CH₂CH₂SCF₃ | O |
| A-6228 | Ph | H | Me | F | CH₂CH₂CH₂SCF₃ | O |
| A-6229 | Ph | H | Me | F | CH₂CH₂CH₂CH₂SCF₃ | O |
| A-6230 | Ph | H | Me | F | CH₂CH₂CH₂CH₂CH₂SCF₃ | O |
| A-6231 | Ph | H | Me | F | CH₂CH₂CH₂CH₂CH₂CH₂SCF₃ | O |
| A-6232 | Ph | H | Me | F | CH₂CH₂CH₂CH₂CH₂CH₂CH₂SCF₃ | O |
| A-6233 | Ph | H | Me | F | CH₂CH₂CH₂CH₂CH₂CH₂CH₂CH₂SCF₃ | O |
| A-6234 | Ph | H | Me | F | CH₂CH₂NHS(=O)₂CF₃ | O |
| A-6235 | Ph | H | Me | F | CH₂CH₂CH₂NHS(=O)₂CF₃ | O |
| A-6236 | Ph | H | Me | F | CH₂CH₂CH₂CH₂NHS(=O)₂CF₃ | O |
| A-6237 | Ph | H | Me | F | CH₂CH₂CH₂CH₂CH₂NHS(=O)₂CF₃ | O |
| A-6238 | Ph | H | Me | F | CH₂CH₂N(Me)S(=O)₂CF₃ | O |
| A-6239 | Ph | H | Me | F | CH₂CH₂N(Et)S(=O)₂CF₃ | O |
| A-6240 | Ph | H | Me | F | CH₂CH₂N(CH₂CN)S(=O)₂CF₃ | O |
| A-6241 | Ph | H | Me | F | CH₂CH₂N(CH₂OMe)S(=O)₂CF₃ | O |
| A-6242 | Ph | H | Me | F | CH₂CH₂CH₂N(Me)S(=O)₂CF₃ | O |

**[Table 143]**

| Compound Number | R¹ | R² | R³ | R⁴ | R⁵ | A |
|---|---|---|---|---|---|---|
| A-6243 | Ph | H | Me | F | CH₂CH₂CH₂N(Et)S(=O)₂CF₃ | O |
| A-6244 | Ph | H | Me | F | CH₂CH₂CH₂N(CH₂CN)S(=O)₂CF₃ | O |
| A-6245 | Ph | H | Me | F | CH₂CH₂CH₂N(CH₂OMe)S(=O)₂CF₃ | O |
| A-6246 | Ph | H | Me | F | CH₂CH₂CH₂CH₂N(Me)S(=O)₂CF₃ | O |
| A-6247 | Ph | H | Me | F | CH₂CH₂CH₂CH₂N(Et)S(=O)₂CF₃ | O |
| A-6248 | Ph | H | Me | F | CH₂CH₂CH₂CH₂N(CH₂CN)S(=O)₂CF₃ | O |
| A-6249 | Ph | H | Me | F | CH₂CH₂CH₂CH₂N(CH₂OMe)S(=O)₂CF₃ | O |
| A-6250 | CH₂Ph | H | Cl | F | CH₂CH₂SCF₃ | O |
| A-6251 | CH₂Ph | H | Cl | F | CH₂CH₂CH₂SCF₃ | O |
| A-6252 | CH₂Ph | H | Cl | F | CH₂CH₂CH₂CH₂SCF₃ | O |
| A-6253 | CH₂Ph | H | Cl | F | CH₂CH₂CH₂CH₂CH₂SCF₃ | O |
| A-6254 | CH₂Ph | H | Cl | F | CH₂CH₂CH₂CH₂CH₂CH₂SCF₃ | O |
| A-6255 | CH₂Ph | H | Cl | F | CH₂CH₂CH₂CH₂CH₂CH₂CH₂SCF₃ | O |
| A-6256 | CH₂Ph | H | Cl | F | CH₂CH₂CH₂CH₂CH₂CH₂CH₂CH₂SCF₃ | O |
| A-6257 | CH₂Ph | H | Cl | F | CH₂CH₂NHS(=O)₂CF₃ | O |
| A-6258 | CH₂Ph | H | Cl | F | CH₂CH₂CH₂NHS(=O)₂CF₃ | O |
| A-6259 | CH₂Ph | H | Cl | F | CH₂CH₂CH₂CH₂NHS(=O)₂CF₃ | O |
| A-6260 | CH₂Ph | H | Cl | F | CH₂CH₂CH₂CH₂CH₂NHS(=O)₂CF₃ | O |
| A-6261 | CH₂Ph | H | Cl | F | CH₂CH₂N(Me)S(=O)₂CF₃ | O |
| A-6262 | CH₂Ph | H | Cl | F | CH₂CH₂N(Et)S(=O)₂CF₃ | O |
| A-6263 | CH₂Ph | H | Cl | F | CH₂CH₂N(CH₂CN)S(=O)₂CF₃ | O |
| A-6264 | CH₂Ph | H | Cl | F | CH₂CH₂N(CH₂OMe)S(=O)₂CF₃ | O |
| A-6265 | CH₂Ph | H | Cl | F | CH₂CH₂CH₂N(Me)S(=O)₂CF₃ | O |
| A-6266 | CH₂Ph | H | Cl | F | CH₂CH₂CH₂N(Et)S(=O)₂CF₃ | O |
| A-6267 | CH₂Ph | H | Cl | F | CH₂CH₂CH₂N(CH₂CN)S(=O)₂CF₃ | O |
| A-6268 | CH₂Ph | H | Cl | F | CH₂CH₂CH₂N(CH₂OMe)S(=O)₂CF₃ | O |
| A-6269 | CH₂Ph | H | Cl | F | CH₂CH₂CH₂CH₂N(Me)S(=O)₂CF₃ | O |
| A-6270 | CH₂Ph | H | Cl | F | CH₂CH₂CH₂CH₂N(Et)S(=O)₂CF₃ | O |
| A-6271 | CH₂Ph | H | Cl | F | CH₂CH₂CH₂CH₂N(CH₂CN)S(=O)₂CF₃ | O |
| A-6272 | CH₂Ph | H | Cl | F | CH₂CH₂CH₂CH₂N(CH₂OMe)S(=O)₂CF₃ | O |
| A-6273 | CH₂Ph | H | Me | F | CH₂CH₂SCF₃ | O |
| A-6274 | CH₂Ph | H | Me | F | CH₂CH₂CH₂SCF₃ | O |
| A-6275 | CH₂Ph | H | Me | F | CH₂CH₂CH₂CH₂SCF₃ | O |
| A-6276 | CH₂Ph | H | Me | F | CH₂CH₂CH₂CH₂CH₂SCF₃ | O |
| A-6277 | CH₂Ph | H | Me | F | CH₂CH₂CH₂CH₂CH₂CH₂SCF₃ | O |
| A-6278 | CH₂Ph | H | Me | F | CH₂CH₂CH₂CH₂CH₂CH₂CH₂SCF₃ | O |
| A-6279 | CH₂Ph | H | Me | F | CH₂CH₂CH₂CH₂CH₂CH₂CH₂CH₂SCF₃ | O |
| A-6280 | CH₂Ph | H | Me | F | CH₂CH₂NHS(=O)₂CF₃ | O |
| A-6281 | CH₂Ph | H | Me | F | CH₂CH₂CH₂NHS(=O)₂CF₃ | O |
| A-6282 | CH₂Ph | H | Me | F | CH₂CH₂CH₂CH₂NHS(=O)₂CF₃ | O |
| A-6283 | CH₂Ph | H | Me | F | CH₂CH₂CH₂CH₂CH₂NHS(=O)₂CF₃ | O |
| A-6284 | CH₂Ph | H | Me | F | CH₂CH₂N(Me)S(=O)₂CF₃ | O |
| A-6285 | CH₂Ph | H | Me | F | CH₂CH₂N(Et)S(=O)₂CF₃ | O |
| A-6286 | CH₂Ph | H | Me | F | CH₂CH₂N(CH₂CN)S(=O)₂CF₃ | O |

**[Table 144]**

| Compound Number | R¹ | R² | R³ | R⁴ | R⁵ | A |
|---|---|---|---|---|---|---|
| A-6287 | CH₂Ph | H | Me | F | CH₂CH₂N(CH₂OMe)S(=O)₂CF₃ | O |
| A-6288 | CH₂Ph | H | Me | F | CH₂CH₂CH₂N(Me)S(=O)₂CF₃ | O |
| A-6289 | CH₂Ph | H | Me | F | CH₂CH₂CH₂N(Et)S(=O)₂CF₃ | O |
| A-6290 | CH₂Ph | H | Me | F | CH₂CH₂CH₂N(CH₂CN)S(=O)₂CF₃ | O |
| A-6291 | CH₂Ph | H | Me | F | CH₂CH₂CH₂N(CH₂OMe)S(=O)₂CF₃ | O |
| A-6292 | CH₂Ph | H | Me | F | CH₂CH₂CH₂CH₂N(Me)S(=O)₂CF₃ | O |
| A-6293 | CH₂Ph | H | Me | F | CH₂CH₂CH₂CH_{2N}(Et)S(=O)₂CF₃ | O |
| A-6294 | CH₂Ph | H | Me | F | CH₂CH₂CH₂CH₂N(CH₂CN)S(=O)₂CF₃ | O |
| A-6295 | CH₂Ph | H | Me | F | CH₂CH₂CH₂CH₂N(CH₂OMe)S(=O)₂CF₃ | O |
| A-6296 | THF-3-yl | H | Cl | F | CH₂CH₂SCF₃ | O |
| A-6297 | THF-3-yl | H | Cl | F | CH₂CH₂CH₂SCF₃ | O |
| A-6298 | THF-3-yl | H | Cl | F | CH₂CH₂CH₂CH₂SCF₃ | O |
| A-6299 | THF-3-yl | H | Cl | F | CH₂CH₂CH₂CH₂CH₂SCF₃ | O |
| A-6300 | THF-3-yl | H | Cl | F | CH₂CH₂CH₂CH₂CH₂CH₂SCF₃ | O |
| A-6301 | THF-3-yl | H | Cl | F | CH₂CH₂CH₂CH₂CH₂CH₂CH₂SCF₃ | O |
| A-6302 | THF-3-yl | H | Cl | F | CH₂CH₂CH₂CH₂CH₂CH₂CH₂CH₂SCF₃ | O |
| A-6303 | THF-3-yl | H | Cl | F | CH₂CH₂NHS(=O)₂CF₃ | O |
| A-6304 | THF-3-yl | H | Cl | F | CH₂CH₂CH₂NHS(=O)₂CF₃ | O |
| A-6305 | THF-3-yl | H | Cl | F | CH₂CH₂CH₂CH₂NHS(=O)₂CF₃ | O |
| A-6306 | THF-3-yl | H | Cl | F | CH₂CH₂CH₂CH₂CH₂NHS(=O)₂CF₃ | O |
| A-6307 | THF-3-yl | H | Cl | F | CH₂CH₂N(Me)S(=O)₂CF₃ | O |
| A-6308 | THF-3-yl | H | Cl | F | CH₂CH₂N(Et)S(=O)₂CF₃ | O |
| A-6309 | THF-3-yl | H | Cl | F | CH₂CH₂N(CH₂CN)S(=O)₂CF₃ | O |
| A-6310 | THF-3-yl | H | Cl | F | CH₂CH₂N(CH₂OMe)S(=O)₂CF₃ | O |
| A-6311 | THF-3-yl | H | Cl | F | CH₂CH₂CH₂N(Me)S(=O)₂CF₃ | O |
| A-6312 | THF-3-yl | H | Cl | F | CH₂CH₂CH₂N(Et)S(=O)₂CF₃ | O |
| A-6313 | THF-3-yl | H | Cl | F | CH₂CH₂CH₂N(CH₂CN)S(=O)₂CF₃ | O |
| A-6314 | THF-3-yl | H | Cl | F | CH₂CH₂CH₂N(CH₂OMe)S(=O)₂CF₃ | O |
| A-6315 | THF-3-yl | H | Cl | F | CH₂CH₂CH₂CH₂N(Me)S(=O)₂CF₃ | O |
| A-6316 | THF-3-yl | H | Cl | F | CH₂CH₂CH₂CH₂N(Et)S(=O)₂CF₃ | O |
| A-6317 | THF-3-yl | H | Cl | F | CH₂CH₂CH₂CH₂N(CH₂CN)S(=O)₂CF₃ | O |
| A-6318 | THF-3-yl | H | Cl | F | CH₂CH₂CH₂CH₂N(CH₂OMe)S(=O)₂CF₃ | O |
| A-6319 | THF-3-yl | H | Me | F | CH₂CH₂SCF₃ | O |
| A-6320 | THF-3-yl | H | Me | F | CH₂CH₂CH₂SCF₃ | O |
| A-6321 | THF-3-yl | H | Me | F | CH₂CH₂CH₂CH₂SCF₃ | O |
| A-6322 | THF-3-yl | H | Me | F | CH₂CH₂CH₂CH₂CH₂SCF₃ | O |
| A-6323 | THF-3-yl | H | Me | F | CH₂CH₂CH₂CH₂CH₂CH₂SCF₃ | O |
| A-6324 | THF-3-yl | H | Me | F | CH₂CH₂CH₂CH₂CH₂CH₂CH₂SCF₃ | O |
| A-6325 | THF-3-yl | H | Me | F | CH₂CH₂CH₂CH₂CH₂CH₂CH₂CH₂SCF₃ | O |
| A-6326 | THF-3-yl | H | Me | F | CH₂CH₂NHS(=O)₂CF₃ | O |
| A-6327 | THF-3-yl | H | Me | F | CH₂CH₂CH₂NHS(=O)₂CF₃ | O |
| A-6328 | THF-3-yl | H | Me | F | CH₂CH₂CH₂CH₂NHS(=O)₂CF₃ | O |
| A-6329 | THF-3-yl | H | Me | F | CH₂CH₂CH₂CH₂CH₂NHS(=O)₂CF₃ | O |
| A-6330 | THF-3-yl | H | Me | F | CH₂CH₂N(Me)S(=O)₂CF₃ | O |

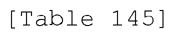

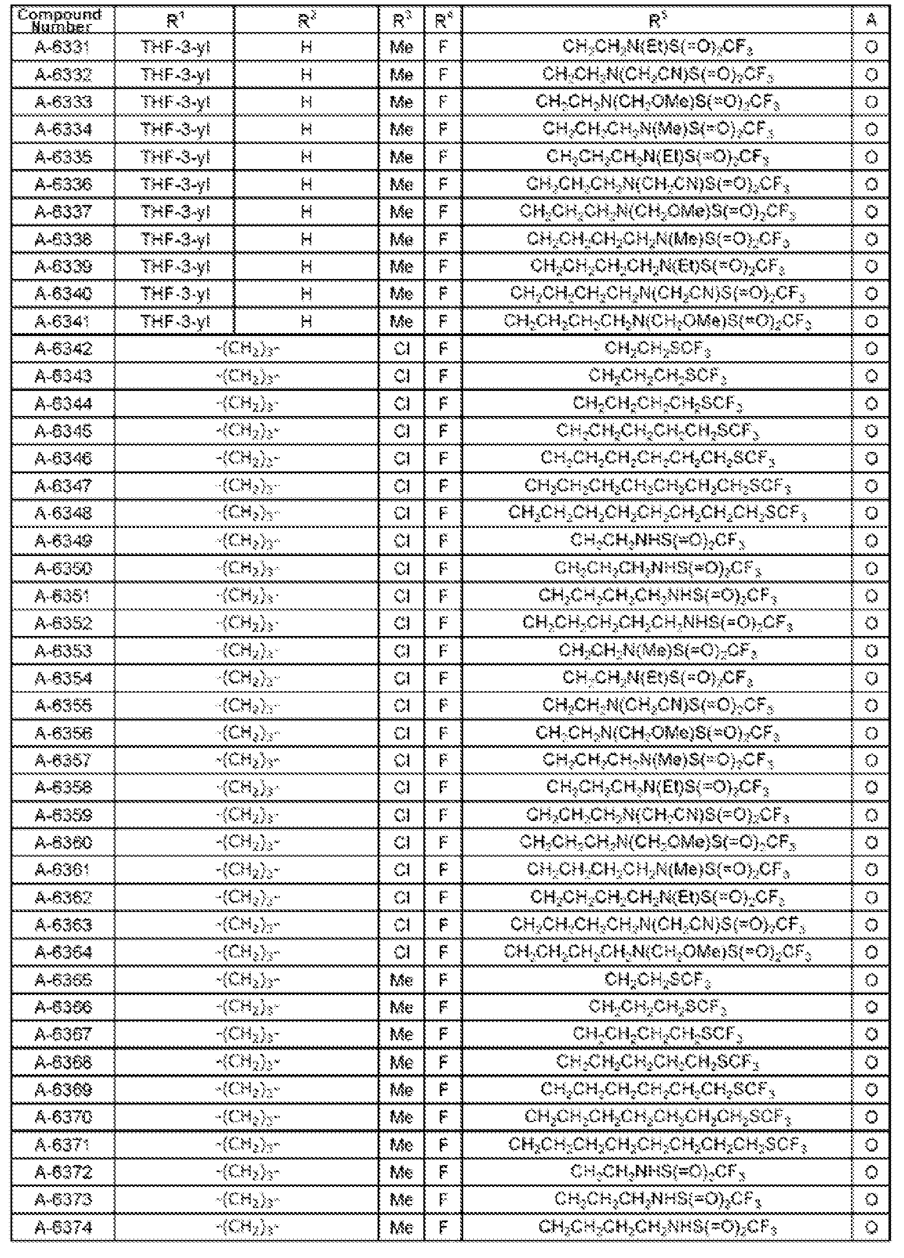

**[Table 146]**

| Compound Number | R¹ | R² | R³ | R⁴ | R⁵ | A |
|---|---|---|---|---|---|---|
| A-6375 | -(CH₂)₃- | | Me | F | CH₂CH₂CH₂CH₂CH₂NHS(=O)₂CF₃ | O |
| A-6376 | -(CH₂)₃- | | Me | F | CH₂CH₂N(Me)S(=O)₂CF₃ | O |
| A-6377 | -(CH₂)₃- | | Me | F | CH₂CH₂N(Et)S(=O)₂CF₃ | O |
| A-6378 | -(CH₂)₃- | | Me | F | CH₂CH₂N(CH₂CN)S(=O)₂CF₃ | O |
| A-6379 | -(CH₂)₃- | | Me | F | CH₂CH₂N(CH₂OMe)S(=O)₂CF₃ | |
| A-6380 | -(CH₂)₃- | | Me | F | CH₂CH₂CH₂N(Me)S(=O)₂CF₃ | O |
| A-6381 | -(CH₂)₃- | | Me | F | CH₂CH₂CH₂N(Et)S(=O)₂CF₃ | O |
| A-6382 | -(CH₂)₃- | | Me | F | CH₂CH₂CH₂N(CH₂CN)S(=O)₂CF₃ | O |
| A-6383 | -(CH₂)₃- | | Me | F | CH₂CH₂CH₂N(CH₂OMe)S(=O)₂CF₃ | O |
| A-6384 | -(CH₂)₃- | | Me | F | CH₂CH₂CH₂CH₂N(Me)S(=O)₂CF₃ | O |
| A-6385 | -(CH₂)₃- | | Me | F | CH₂CH₂CH₂CH₂N(Et)S(=O)₂CF₃ | O |
| A-6386 | -(CH₂)₃- | | Me | F | CH₂CH₂CH₂CH₂N(CH₂CN)S(=O)₂CF₃ | O |
| A-6387 | -(CH₂)₃- | | Me | F | CH₂CH₂CH₂CH₂N(CH₂OMe)S(=O)₂CF₃ | O |
| A-6388 | -(CH₂)₄- | | Cl | F | CH₂CH₂SCF₃ | O |
| A-6389 | -(CH₂)₄- | | Cl | F | CH₂CH₂CH₂SCF₃ | O |
| A-6390 | -(CH₂)₄- | | Cl | F | CH₂CH₂CH₂CH₂SCF₃ | O |
| A-6391 | -(CH₂)₄- | | Cl | F | CH₂CH₂CH₂CH₂CH₂SCF₃ | O |
| A-6392 | -(CH₂)₄- | | Cl | F | CH₂CH₂CH₂CH₂CH₂CH₂SCF₃ | O |
| A-6393 | -(CH₂)₄- | | Cl | F | CH₂CH₂CH₂CH₂CH₂CH₂CH₂SCF₃ | O |
| A-6394 | -(CH₂)₄- | | Cl | F | CH₂CH₂CH₂CH₂CH₂CH₂CH₂CH₂SCF₃ | O |
| A-6395 | -(CH₂)₄- | | Cl | F | CH₂CH₂NHS(=O)₂CF₃ | O |
| A-6396 | -(CH₂)₄- | | Cl | F | CH₂CH₂CH₂NHS(=O)₂CF₃ | O |
| A-6397 | -(CH₂)₄- | | Cl | F | CH₂CH₂CH₂CH₂NHS(=O)₂CF₃ | O |
| A-6398 | -(CH₂)₄- | | Cl | F | CH₂CH₂CH₂CH₂CH₂NHS(=O)₂CF₃ | O |
| A-6399 | -(CH₂)₄- | | Cl | F | CH₂CH₂N(Me)S(=O)₂CF₃ | O |
| A-6400 | -(CH₂)₄- | | Cl | F | CH₂CH₂N(Et)S(=O)₂CF₃ | O |
| A-6401 | -(CH₂)₄- | | Cl | F | CH₂CH₂N(CH₂CN)S(=O)₂CF₃ | O |
| A-6402 | -(CH₂)₄- | | Cl | F | CH₂CH₂N(CH₂OMe)S(=O)₂CF₃ | O |
| A-6403 | -(CH₂)₄- | | Cl | F | CH₂CH₂CH₂N(Me)S(=O)₂CF₃ | O |
| A-6404 | -(CH₂)₄- | | Cl | F | CH₂CH₂CH₂N(Et)S(=O)₂CF₃ | O |
| A-6405 | -(CH₂)₄- | | Cl | F | CH₂CH₂CH₂N(CH₂CN)S(=O)₂CF₃ | O |
| A-6406 | -(CH₂)₄- | | Cl | F | CH₂CH₂CH₂N(CH₂OMe)S(=O)₂CF₃ | O |
| A-6407 | -(CH₂)₄- | | Cl | F | CH₂CH₂CH₂CH₂N(Me)S(=O)₂CF₃ | O |
| A-6408 | -(CH₂)₄- | | Cl | F | CH₂CH₂CH₂CH₂N(Et)S(=O)₂CF₃ | O |
| A-6409 | -(CH₂)₄- | | Cl | F | CH₂CH₂CH₂CH₂N(CH₂CN)S(=O)₂CF₃ | O |
| A-6410 | -(CH₂)₄- | | Cl | F | CH₂CH₂CH₂CH₂N(CH₂OMe)S(=O)₂CF₃ | O |
| A-6411 | -(CH₂)₄- | | Me | F | CH₂CH₂SCF₃ | O |
| A-6412 | -(CH₂)₄- | | Me | F | CH₂CH₂CH₂SCF₃ | O |
| A-6413 | -(CH₂)₄- | | Me | F | CH₂CH₂CH₂CH₂SCF₃ | O |
| A-6414 | -(CH₂)₄- | | Me | F | CH₂CH₂CH₂CH₂CH₂SCF₃ | O |
| A-6415 | -(CH₂)₄- | | Me | F | CH₂CH₂CH₂CH₂CH₂CH₂SCF₃ | O |
| A-6416 | -(CH₂)₄- | | Me | F | CH₂CH₂CH₂CH₂CH₂CH₂CH₂SCF₃ | O |
| A-6417 | -(CH₂)₄- | | Me | F | CH₂CH₂CH₂CH₂CH₂CH₂CH₂CH₂SCF₃ | O |
| A-6418 | -(CH₂)₄- | | Me | F | CH₂CH₂NHS(=O)₂CF₃ | O |

**[Table 147]**

| Compound Number | R¹ | R² | R³ | R⁴ | R⁵ | A |
|---|---|---|---|---|---|---|
| A-6419 | -(CH₂)₄- | | Me | F | CH₂CH₂CH₂NHS(=O)₂CF₃ | O |
| A-6420 | -(CH₂)₄- | | Me | F | CH₂CH₂CH₂CH₂NHS(=O)₂CF₃ | O |
| A-6421 | -(CH₂)₄- | | Me | F | CH₂CH₂CH₂CH₂CH₂NHS(=O)₂CF₃ | O |
| A-6422 | -(CH₂)₄- | | Me | F | CH₂CH₂N(Me)S(=O)₂CF₃ | O |
| A-6423 | -(CH₂)₄- | | Me | F | CH₂CH₂N(Et)S(=O)₂CF₃ | O |
| A-6424 | -(CH₂)₄- | | Me | F | CH₂CH₂N(CH₂CN)S(=O)₂CF₃ | O |
| A-6425 | -(CH₂)₄- | | Me | F | CH₂CH₂N(CH₂OMe)S(=O)₂CF₃ | O |
| A-6426 | -(CH₂)₄- | | Me | F | CH₂CH₂CH₂N(Me)S(=O)₂CF₃ | O |
| A-6427 | -(CH₂)₄- | | Me | F | CH₂CH₂CH₂N(Et)S(=O)₂CF₃ | O |
| A-6428 | -(CH₂)₄- | | Me | F | CH₂CH₂CH₂N(CH₂CN)S(=O)₂CF₃ | O |
| A-6429 | -(CH₂)₄- | | Me | F | CH₂CH₂CH₂N(CH₂OMe)S(=O)₂CF₃ | O |
| A-6430 | -(CH₂)₄- | | Me | F | CH₂CH₂CH₂CH₂N(Me)S(=O)₂CF₃ | O |
| A-6431 | -(CH₂)₄- | | Me | F | CH₂CH₂CH₂CH₂N(Et)S(=O)₂CF₃ | O |
| A-6432 | -(CH₂)₄- | | Me | F | CH₂CH₂CH₂CH₂N(CH₂CN)S(=O)₂CF₃ | O |
| A-6433 | -(CH₂)₄- | | Me | F | CH₂CH₂CH₂CH₂N(CH₂OMe)S(=O)₂CF₃ | O |
| A-6434 | -(CH₂)₂O(CH₂)₂- | | Cl | F | CH₂CH₂SCF₃ | O |
| A-6435 | -(CH₂)₂O(CH₂)₂- | | Cl | F | CH₂CH₂CH₂SCF₃ | O |
| A-6436 | -(CH₂)₂O(CH₂)₂- | | Cl | F | CH₂CH₂CH₂CH₂SCF₃ | O |
| A-6437 | -(CH₂)₂O(CH₂)₂- | | Cl | F | CH₂CH₂CH₂CH₂CH₂SCF₃ | O |
| A-6438 | -(CH₂)₂O(CH₂)₂- | | Cl | F | CH₂CH₂CH₂CH₂CH₂CH₂SCF₃ | O |
| A-6439 | -(CH₂)₂O(CH₂)₂- | | Cl | F | CH₂CH₂CH₂CH₂CH₂CH₂CH₂SCF₃ | O |
| A-6440 | -(CH₂)₂O(CH₂)₂- | | Cl | F | CH₂CH₂CH₂CH₂CH₂CH₂CH₂CH₂SCF₃ | O |
| A-6441 | -(CH₂)₂O(CH₂)₂- | | Cl | F | CH₂CH₂NHS(=O)₂CF₃ | O |
| A-6442 | -(CH₂)₂O(CH₂)₂- | | Cl | F | CH₂CH₂CH₂NHS(=O)₂CF₃ | O |
| A-6443 | -(CH₂)₂O(CH₂)₂- | | Cl | F | CH₂CH₂CH₂CH₂NHS(=O)₂CF₃ | O |
| A-6444 | -(CH₂)₂O(CH₂)₂- | | Cl | F | CH₂CH₂CH₂CH₂CH₂NHS(=O)₂CF₃ | O |
| A-6445 | -(CH₂)₂O(CH₂)₂- | | Cl | F | CH₂CH₂N(Me)S(=O)₂CF₃ | O |
| A-6446 | -(CH₂)₂O(CH₂)₂- | | Cl | F | CH₂CH₂N(Et)S(=O)₂CF₃ | O |
| A-6447 | -(CH₂)₂O(CH₂)₂- | | Cl | F | CH₂CH₂N(CH₂CN)S(=O)₂CF₃ | O |
| A-6448 | -(CH₂)₂O(CH₂)₂- | | Cl | F | CH₂CH₂N(CH₂OMe)S(=O)₂CF₃ | O |
| A-6449 | -(CH₂)₂O(CH₂)₂- | | Cl | F | CH₂CH₂CH₂N(Me)S(=O)₂CF₃ | O |
| A-6450 | -(CH₂)₂O(CH₂)₂- | | Cl | F | CH₂CH₂CH₂N(Et)S(=O)₂CF₃ | O |
| A-6451 | -(CH₂)₂O(CH₂)₂- | | Cl | F | CH₂CH₂CH₂N(CH₂CN)S(=O)₂CF₃ | O |
| A-6452 | -(CH₂)₂O(CH₂)₂- | | Cl | F | CH₂CH₂CH₂N(CH₂OMe)S(=O)₂CF₃ | O |
| A-6453 | -(CH₂)₂O(CH₂)₂- | | Cl | F | CH₂CH₂CH₂CH₂N(Me)S(=O)₂CF₃ | O |
| A-6454 | -(CH₂)₂O(CH₂)₂- | | Cl | F | CH₂CH₂CH₂CH₂N(Et)S(=O)₂CF₃ | O |
| A-6455 | -(CH₂)₂O(CH₂)₂- | | Cl | F | CH₂CH₂CH₂CH₂N(CH₂CN)S(=O)₂CF₃ | O |
| A-6456 | -(CH₂)₂O(CH₂)₂- | | Cl | F | CH₂CH₂CH₂CH₂N(CH₂OMe)S(=O)₂CF₃ | O |
| A-6457 | -(CH₂)₂O(CH₂)₂- | | Me | F | CH₂CH₂SCF₃ | O |
| A-6458 | -(CH₂)₂O(CH₂)₂- | | Me | F | CH₂CH₂CH₂SCF₃ | O |
| A-6459 | -(CH₂)₂O(CH₂)₂- | | Me | F | CH₂CH₂CH₂CH₂SCF₃ | O |
| A-6460 | -(CH₂)₂O(CH₂)₂- | | Me | F | CH₂CH₂CH₂CH₂CH₂SCF₃ | O |
| A-6461 | -(CH₂)₂O(CH₂)₂- | | Me | F | CH₂CH₂CH₂CH₂CH₂CH₂SCF₃ | O |
| A-6462 | -(CH₂)₂O(CH₂)₂- | | Me | F | CH₂CH₂CH₂CH₂CH₂CH₂CH₂SCF₃ | O |

**[Table 148]**

| Compound Number | R¹ | R² | R³ | R⁴ | R⁵ | A |
|---|---|---|---|---|---|---|
| A-6463 | -(CH₂)₂O(CH₂)₂- | | Me | F | CH₂CH₂CH₂CH₂CH₂CH₂CH₂CH₂SCF₃ | O |
| A-6464 | -(CH₂)₂O(CH₂)₂- | | Me | F | CH₂CH₂NHS(=O)₂CF₃ | O |
| A-6465 | -(CH₂)₂O(CH₂)₂- | | Me | F | CH₂CH₂CH₂NHS(=O)₂CF₃ | O |
| A-6466 | -(CH₂)₂O(CH₂)₂- | | Me | F | CH₂CH₂CH₂CH₂NHS(=O)₂CF₃ | O |
| A-6467 | -(CH₂)₂O(CH₂)₂- | | Me | F | CH₂CH₂CH₂CH₂CH₂NHS(=O)₂CF₃ | O |
| A-6468 | -(CH₂)₂O(CH₂)₂- | | Me | F | CH₂CH₂N(Me)S(=O)₂CF₃ | O |
| A-6469 | -(CH₂)₂O(CH₂)₂- | | Me | F | CH₂CH₂N(Et)S(=O)₂CF₃ | O |
| A-6470 | -(CH₂)₂O(CH₂)₂- | | Me | F | CH₂CH₂N(CH₂CN)S(=O)₂CF₃ | O |
| A-6471 | -(CH₂)₂O(CH₂)₂- | | Me | F | CH₂CH₂N(CH₂OMe)S(=O)₂CF₃ | O |
| A-6472 | -(CH₂)₂O(CH₂)₂- | | Me | F | CH₂CH₂CH₂N(Me)S(=O)₂CF₃ | O |
| A-6473 | -(CH₂)₂O(CH₂)₂- | | Me | F | CH₂CH₂CH₂N(Et)S(=O)₂CF₃ | O |
| A-6474 | -(CH₂)₂O(CH₂)₂- | | Me | F | CH₂CH₂CH₂N(CH₂CN)S(=O)₂CF₃ | O |
| A-6475 | -(CH₂)₂O(CH₂)₂- | | Me | F | CH₂CH₂CH₂N(CH₂OMe)S(=O)₂CF₃ | O |
| A-6476 | -(CH₂)₂O(CH₂)₂- | | Me | F | CH₂CH₂CH₂CH₂N(Me)S(=O)₂CF₃ | O |
| A-6477 | -(CH₂)₂O(CH₂)₂- | | Me | F | CH₂CH₂CH₂CH₂N(Et)S(=O)₂CF₃ | O |
| A-6478 | -(CH₂)₂O(CH₂)₂- | | Me | F | CH₂CH₂CH₂CH₂N(CH₂CN)S(=O)₂CF₃ | O |
| A-6479 | -(CH₂)₂O(CH₂)₂- | | Me | F | CH₂CH₂CH₂CH₂N(CH₂OMe)S(=O)₂CF₃ | O |
| A-6480 | CH₂CF₃ | Me | Cl | F | CH₂CH₂CH₂SCF₃ | O |
| A-6481 | CH₂CF₃ | Me | Cl | F | CH₂CH₂CH₂CH₂SCF₃ | O |
| A-6482 | CH₂CF₃ | Me | Cl | F | CH₂CH₂CH₂CH₂CH₂SCF₃ | O |
| A-6483 | CH₂CF₃ | Me | Cl | F | CH₂CH₂CH₂CH₂CH₂CH₂SCF₃ | O |
| A-6484 | CH₂CF₃ | Me | Cl | F | CH₂CH₂CH₂CH₂CH₂CH₂CH₂SCF₃ | O |
| A-6485 | CH₂CF₃ | Me | Cl | F | CH₂CH₂NHS(=O)₂CF₃ | O |
| A-6486 | CH₂CF₃ | Me | Cl | F | CH₂CH₂CH₂NHS(=O)₂CF₃ | O |
| A-6487 | CH₂CF₃ | Me | Cl | F | CH₂CH₂N(Me)S(=O)₂CF₃ | O |
| A-6488 | CH₂CF₃ | Me | Cl | F | CH₂CH₂N(Et)S(=O)₂CF₃ | O |
| A-6489 | CH₂CF₃ | Me | Cl | F | CH₂CH₂N(CH₂CN)S(=O)₂CF₃ | O |
| A-6490 | CH₂CF₃ | Me | Cl | F | CH₂CH₂N(CH₂OMe)S(=O)₂CF₃ | O |
| A-6491 | CH₂CF₃ | Me | Cl | F | CH₂CH₂CH₂N(Me)S(=O)₂CF₃ | O |
| A-6492 | CH₂CF₃ | Me | Cl | F | CH₂CH₂CH₂N(Et)S(=O)₂CF₃ | O |
| A-6493 | CH₂CF₃ | Me | Cl | F | CH₂CH₂CH₂N(CH₂CN)S(=O)₂CF₃ | O |
| A-6494 | CH₂CF₃ | Me | Cl | F | CH₂CH₂CH₂N(CH₂OMe)S(=O)₂CF₃ | O |
| A-6495 | CH₂CF₃ | Me | Me | F | CH₂CH₂CH₂SCF₃ | O |
| A-6496 | CH₂CF₃ | Me | Me | F | CH₂CH₂CH₂CH₂SCF₃ | O |
| A-6497 | CH₂CF₃ | Me | Me | H | CH₂CH₂CH₂CH₂CH₂SCF₃ | O |
| A-6498 | CH₂CF₃ | Me | Me | F | CH₂CH₂CH₂CH₂CH₂CH₂SCF₃ | O |
| A-6499 | CH₂CF₃ | Me | Me | F | CH₂CH₂CH₂CH₂CH₂CH₂CH₂SCF₃ | O |
| A-6500 | CH₂CF₃ | Me | Me | F | CH₂CH₂NHS(=O)₂CF₃ | O |
| A-6501 | CH₂CF₃ | Me | Me | F | CH₂CH₂CH₂NHS(=O)₂CF₃ | O |
| A-6502 | CH₂CF₃ | Me | Me | F | CH₂CH₂N(Me)S(=O)₂CF₃ | O |
| A-6503 | CH₂CF₃ | Me | Me | F | CH₂CH₂N(Et)S(=O)₂CF₃ | O |
| A-6504 | CH₂CF₃ | Me | Me | F | CH₂CH₂N(CH₂CN)S(=O)₂CF₃ | O |
| A-6505 | CH₂CF₃ | Me | Me | F | CH₂CH₂N(CH₂OMe)S(=O)₂CF₃ | O |
| A-6506 | CH₂CF₃ | Me | Me | F | CH₂CH₂CH₂N(Me)S(=O)₂CF₃ | O |

**[Table 149]**

| Compound Number | R¹ | R² | R³ | R⁴ | R⁵ | A |
|---|---|---|---|---|---|---|
| A-6507 | CH₂CF₃ | Me | Me | F | CH₂CH₂CH₂N(Et)S(=O)₂CF₃ | O |
| A-6508 | CH₂CF₃ | Me | Me | F | CH₂CH₂CH₂N(CH₂CN)S(=O)₂CF₃ | O |
| A-6509 | CH₂CF₃ | Me | Me | F | CH₂CH₂CH₂N(CH₂OMe)S(=O)₂CF₃ | O |
| A-6510 | CH₂CF₃ | Et | Cl | F | CH₂CH₂CH₂SCF₃ | O |
| A-6511 | CH₂CF₃ | Et | Cl | F | CH₂CH₂CH₂CH₂SCF₃ | O |
| A-6512 | CH₂CF₃ | Et | Cl | F | CH₂CH₂CH₂CH₂CH₂SCF₃ | O |
| A-6513 | CH₂CF₃ | Et | Cl | F | CH₂CH₂CH₂CH₂CH₂CH₂SCF₃ | O |
| A-6514 | CH₂CF₃ | Et | Cl | F | CH₂CH₂CH₂CH₂CH₂CH₂CH₂SCF₃ | O |
| A-6515 | CH₂CF₃ | Et | Cl | F | CH₂CH₂NHS(=O)₂CF₃ | O |
| A-6516 | CH₂CF₃ | Et | Cl | F | CH₂CH₂CH₂NHS(=O)₂CF₃ | O |
| A-6517 | CH₂CF₃ | Et | Cl | F | CH₂CH₂N(Me)S(=O)₂CF₃ | O |
| A-6518 | CH₂CF₃ | Et | Cl | F | CH₂CH₂N(Et)S(=O)₂CF₃ | O |
| A-6519 | CH₂CF₃ | Et | Cl | F | CH₂CH₂N(CH₂CN)S(=O)₂CF₃ | O |
| A-6520 | CH₂CF₃ | Et | Cl | F | CH₂CH₂N(CH₂OMe)S(=O)₂CF₃ | O |
| A-6521 | CH₂CF₃ | Et | Cl | F | CH₂CH₂CH₂N(Me)S(=O)₂CF₃ | O |
| A-6522 | CH₂CF₃ | Et | Cl | F | CH₂CH₂CH₂N(Et)S(=O)₂CF₃ | O |
| A-6523 | CH₂CF₃ | Et | Cl | F | CH₂CH₂CH₂N(CH₂CN)S(=O)₂CF₃ | O |
| A-6524 | CH₂CF₃ | Et | Cl | F | CH₂CH₂CH₂N(CH₂OMe)S(=O)₂CF₃ | O |
| A-6525 | CH₂CF₃ | Et | Me | F | CH₂CH₂CH₂SCF₃ | O |
| A-6526 | CH₂CF₃ | Et | Me | F | CH₂CH₂CH₂CH₂SCF₃ | O |
| A-6527 | CH₂CF₃ | Et | Me | F | CH₂CH₂CH₂CH₂CH₂SCF₃ | O |
| A-6528 | CH₂CF₃ | Et | Me | F | CH₂CH₂CH₂CH₂CH₂CH₂SCF₃ | O |
| A-6529 | CH₂CF₃ | Et | Me | F | CH₂CH₂CH₂CH₂CH₂CH₂CH₂SCF₃ | O |
| A-6530 | CH₂CF₃ | Et | Me | F | CH₂CH₂NHS(=O)₂CF₃ | O |
| A-6531 | CH₂CF₃ | Et | Me | F | CH₂CH₂CH₂NHS(=O)₂CF₃ | O |
| A-6532 | CH₂CF₃ | Et | Me | F | CH₂CH₂N(Me)S(=O)₂CF₃ | O |
| A-6533 | CH₂CF₃ | Et | Me | F | CH₂CH₂N(Et)S(=O)₂CF₃ | O |
| A-6534 | CH₂CF₃ | Et | Me | F | CH₂CH₂N(CH₂CN)S(=O)₂CF₃ | O |
| A-6535 | CH₂CF₃ | Et | Me | F | CH₂CH₂N(CH₂OMe)S(=O)₂CF₃ | O |
| A-6536 | CH₂CF₃ | Et | Me | F | CH₂CH₂CH₂N(Me)S(=O)₂CF₃ | O |
| A-6537 | CH₂CF₃ | Et | Me | F | CH₂CH₂CH₂N(Et)S(=O)₂CF₃ | O |
| A-6538 | CH₂CF₃ | Et | Me | F | CH₂CH₂CH₂N(CH₂CN)S(=O)₂CF₃ | O |
| A-6539 | CH₂CF₃ | Et | Me | F | CH₂CH₂CH₂N(CH₂OMe)S(=O)₂CF₃ | O |
| A-6540 | CH₂CF₃ | *n* -Hex | Cl | F | CH₂CH₂CH₂SCF₃ | O |
| A-6541 | CH₂CF₃ | *n* -Hex | Cl | F | CH₂CH₂CH₂CH₂SCF₃ | O |
| A-6542 | CH₂CF₃ | *n* -Hex | Cl | F | CH₂CH₂CH₂CH₂CH₂SCF₃ | O |
| A-6543 | CH₂CF₃ | *n* -Hex | Cl | F | CH₂CH₂CH₂CH₂CH₂CH₂SCF₃ | O |
| A-6544 | CH₂CF₃ | *n -Hex* | Cl | F | CH₂CH₂CH₂CH₂CH₂CH₂CH₂SCF₃ | O |
| A-6545 | CH₂CF₃ | *n* -Hex | Cl | F | CH₂CH₂NHS(=O)₂CF₃ | O |
| A-6546 | CH₂CF₃ | *n* -Hex | Cl | F | CH₂CH₂CH₂NHS(=O)₂CF₃ | O |
| A-6547 | CH₂CF₃ | *n* -Hex | Cl | F | CH₂CH₂N(Me)S(=O)₂CF₃ | O |
| A-6548 | CH₂CF₃ | *n* -Hex | Cl | F | CH₂CH₂N(Et)S(=O)₂CF₃ | O |
| A-6549 | CH₂CF₃ | *n* -Hex | Cl | F | CH₂CH₂N(CH₂CN)S(=O)₂CF₃ | O |
| A-6550 | CH₂CF₃ | *n*-Hex | Cl | F | CH₂CH₂N(CH₂OMe)S(=O)₂CF₃ | O |

**[Table 150]**

| Compound Number | R¹ | R² | R³ | R⁴ | R⁵ | A |
|---|---|---|---|---|---|---|
| A-6551 | CH₂CF₃ | *n -Hex* | Cl | F | CH₂CH₂CH₂N(Me)S(=O)₂CF₃ | O |
| A-6552 | CH₂CF₃ | *n -Hex* | Cl | F | CH₂CH₂CH₂N(Et)S(-O)₂CF₃ | O |
| A-6553 | CH₂CF₃ | *n -Hex* | Cl | F | CH₂CH₂CH₂N(CH₂CN)S(=O)₂CF₃ | O |
| A-6554 | CH₂CF₃ | *n -Hex* | Cl | F | CH₂CH₂CH₂N(CH₂OMe)S(=O)₂CF₃ | O |
| A-6555 | CH₂CF₃ | *n* -Hex | Me | F | CH₂CH₂CH₂SCF₃ | O |
| A-6556 | CH₂CF₃ | *n -Hex* | Me | F | CH₂CH₂CH₂CH₂SCF₃ | O |
| A-6557 | CH₂CF₃ | *n* -Hex | Me | F | CH₂CH₂CH₂CH₂CH₂SCF₃ | O |
| A-6558 | CH₂CF₃ | *n* -Hex | Me | F | CH₂CH₂CH₂CH₂CH₂CH₂SCF₃ | O |
| A-6559 | CH₂CF₃ | *n* -Hex | Me | F | CH₂CH₂CH₂CH₂CH₂CH₂CH₂SCF₃ | O |
| A-6560 | CH₂CF₃ | *n* -Hex | Me | F | CH₂CH₂NHS(=O)₂CF₃ | O |
| A-6561 | CH₂CF₃ | *n -Hex* | Me | F | CH₂CH₂CH₂NHS(=O)₂CF₃ | O |
| A-6562 | CH₂CF₃ | *n* -Hex | Me | F | CH₂CH₂N(Me)S(=O)₂CF₃ | O |
| A-6563 | CH₂CF₃ | *n -Hex* | Me | F | CH₂CH₂N(Et)S(=O)₂CF₃ | O |
| A-6564 | CH₂CF₃ | *n* -Hex | Me | F | CH₂CH₂N(CH₂CN)S(=O)₂CF₃ | O |
| A-6565 | CH₂CF₃ | *n* -Hex | Me | F | CH₂CH₂N(CH₂OMe)S(=O)₂CF₃ | O |
| A-6566 | CH₂CF₃ | *n*-Hex | Me | F | CH₂CH₂CH₂N(Me)S(=O)₂CF₃ | O |
| A-6567 | CH₂CF₃ | *n* -Hex | Me | F | CH₂CH₂CH₂N(Et)S(=O)₂CF₃ | O |
| A-6568 | CH₂CF₃ | *n -Hex* | Me | F | CH₂CH₂CH₂N(CH₂CN)S(=O)₂CF₃ | O |
| A-6569 | CH₂CF₃ | *n* -Hex | Me | F | CH₂CH₂CH₂N(CH₂OMe)S(=O)₂CF₃ | O |
| A-6570 | CH₂CF₃ | CH₂CH₂F | Cl | F | CH₂CH₂CH₂SCF₃ | O |
| A-6571 | CH₂CF₃ | CH₂CH₂F | Cl | F | CH₂CH₂CH₂CH₂SCF₃ | O |
| A-6572 | CH₂CF₃ | CH₂CH₂F | Cl | F | CH₂CH₂CH₂CH₂CH₂SCF₃ | O |
| A-6573 | CH₂CF₃ | CH₂CH₂F | Cl | F | CH₂CH₂CH₂CH₂CH₂CH₂SCF₃ | O |
| A-6574 | CH₂CF₃ | CH₂CH₂F | Cl | F | CH₂CH₂CH₂CH₂CH₂CH₂CH₂SCF₃ | O |
| A-6575 | CH₂CF₃ | CH₂CH₂F | Cl | F | CH₂CH₂NHS(=O)₂CF₃ | O |
| A-6576 | CH₂CF₃ | CH₂CH₂F | Cl | F | CH₂CH₂CH₂NHS(=O)₂CF₃ | O |
| A-6577 | CH₂CF₃ | CH₂CH₂F | Cl | F | CH₂CH₂N(Me)S(=O)₂CF₃ | O |
| A-6578 | CH₂CF₃ | CH₂CH₂F | Cl | F | CH₂CH₂N(Et)S(=O)₂CF₃ | O |
| A-6579 | CH₂CF₃ | CH₂CH₂F | Cl | F | CH₂CH₂N(CH₂CN)S(=O)₂CF₃ | O |
| A-6580 | CH₂CF₃ | CH₂CH₂F | Cl | F | CH₂CH₂N(CH₂OMe)S(=O)₂CF₃ | O |
| A-6581 | CH₂CF₃ | CH₂CH₂F | Cl | F | CH₂CH₂CH₂N(Me)S(=O)₂CF₃ | O |
| A-6582 | CH₂CF₃ | CH₂CH₂F | Cl | F | CH₂CH₂CH₂N(Et)S(=O)₂CF₃ | O |
| A-6583 | CH₂CF₃ | CH₂CH₂F | Cl | F | CH₂CH₂CH₂N(CH₂CN)S(=O)₂CF₃ | O |
| A-6584 | CH₂CF₃ | CH₂CH₂F | Cl | F | CH₂CH₂CH₂N(CH₂OMe)S(=O)₂CF₃ | O |
| A-6585 | CH₂CF₃ | CH₂CH₂F | Me | F | CH₂CH₂CH₂SCF₃ | O |
| A-6586 | CH₂CF₃ | CH₂CH₂F | Me | F | CH₂CH₂CH₂CH₂SCF₃ | O |
| A-6587 | CH₂CF₃ | CH₂CH₂F | Me | F | CH₂CH₂CH₂CH₂CH₂SCF₃ | O |
| A-6588 | CH₂CF₃ | CH₂CH₂F | Me | F | CH₂CH₂CH₂CH₂CH₂CH₂SCF₃ | O |
| A-6589 | CH₂CF₃ | CH₂CH₂F | Me | F | CH₂CH₂CH₂CH₂CH₂CH₂CH₂SCF₃ | O |
| A-6590 | CH₂CF₃ | CH₂CH₂F | Me | F | CH₂CH₂NHS(=O)₂CF₃ | O |
| A-6591 | CH₂CF₃ | CH₂CH₂F | Me | F | CH₂CH₂CH₂NHS(=O)₂CF₃ | O |
| A-6592 | CH₂CF₃ | CH₂CH₂F | Me | F | CH₂CH₂N(Me)S(=O)₂CF₃ | O |
| A-6593 | CH₂CF₃ | CH₂CH₂F | Me | F | CH₂CH₂N(Et)S(=O)₂CF₃ | O |
| A-6594 | CH₂CF₃ | CH₂CH₂F | Me | F | CH₂CH₂N(CH₂CN)S(=O)₂CF₃ | O |

**[Table 151]**

| Compound Number | R¹ | R² | R³ | R⁴ | R⁵ | A |
|---|---|---|---|---|---|---|
| A-6595 | CH₂CF₃ | CH₂CH₂F | Me | F | CH₂CH₂N(CH₂OMe)S(=O)₂CF₃ | O |
| A-6596 | CH₂CF₃ | CH₂CH₂F | Me | F | CH₂CH₂CH₂N(Me)S(=O)₂CF₃ | O |
| A-6597 | CH₂CF₃ | CH₂CH₂F | Me | F | CH₂CH₂CH₂N(Et)S(=O)₂CF₃ | O |
| A-6598 | CH₂CF₃ | CH₂CH₂F | Me | F | CH₂CH₂CH₂N(CH₂CN)S(=O)₂CF₃ | O |
| A-6599 | CH₂CF₃ | CH₂CH₂F | Me | F | CH₂CH₂CH₂N(CH₂OMe)S(=O)₂CF₃ | O |
| A-6600 | CH₂CF₃ | CH₂CHF₂ | Cl | F | CH₂CH₂CH₂SCF₃ | O |
| A-6601 | CH₂CF₃ | CH₂CHF₂ | Cl | F | CH₂CH₂CH₂CH₂SCF₃ | O |
| A-6602 | CH₂CF₃ | CH₂CHF₂ | Cl | F | CH₂CH₂CH₂CH₂CH₂SCF₃ | O |
| A-6603 | CH₂CF₃ | CH₂CHF₂ | Cl | F | CH₂CH₂CH₂CH₂CH₂CH₂SCF₃ | O |
| A-6604 | CH₂CF₃ | CH₂CHF₂ | Cl | F | CH₂CH₂CH₂CH₂CH₂CH₂CH₂SCF₃ | O |
| A-6605 | CH₂CF₃ | CH₂CHF₂ | Cl | F | CH₂CH₂NHS(=O)₂CF₃ | O |
| A-6606 | CH₂CF₃ | CH₂CHF₂ | Cl | F | CH₂CH₂CH₂NHS(=O)₂CF₃ | O |
| A-6607 | CH₂CF₃ | CH₂CHF₂ | Cl | F | CH₂CH₂N(Me)S(=O)₂CF₃ | O |
| A-6608 | CH₂CF₃ | CH₂CHF₂ | Cl | F | CH₂CH₂N(Et)S(=O)₂CF₃ | O |
| A-6609 | CH₂CF₃ | CH₂CHF₂ | Cl | F | CH₂CH₂N(CH₂CN)S(=O)₂CF₃ | O |
| A-6610 | CH₂CF₃ | CH₂CHF₂ | Cl | F | CH₂CH₂N(CH₂OMe)S(=O)₂CF₃ | O |
| A-6611 | CH₂CF₃ | CH₂CHF₂ | Cl | F | CH₂CH₂CH₂N(Me)S(=O)₂CF₃ | O |
| A-6612 | CH₂CF₃ | CH₂CHF₂ | Cl | F | CH₂CH₂CH₂N(Et)S(=O)₂CF₃ | O |
| A-6613 | CH₂CF₃ | CH₂CHF₂ | Cl | F | CH₂CH₂CH₂N(CH₂CN)S(=O)₂CF₃ | O |
| A-6614 | CH₂CF₃ | CH₂CHF₂ | Cl | F | CH₂CH₂CH₂N(CH₂OMe)S(=O)₂CF₃ | O |
| A-6615 | CH₂CF₃ | CH₂CHF₂ | Me | F | CH₂CH₂CH₂SCF₃ | O |
| A-6616 | CH₂CF₃ | CH₂CHF₂ | Me | F | CH₂CH₂CH₂CH₂SCF₃ | O |
| A-6617 | CH₂CF₃ | CH₂CHF₂ | Me | F | CH₂CH₂CH₂CH₂CH₂SCF₃ | O |
| A-6618 | CH₂CF₃ | CH₂CHF₂ | Me | F | CH₂CH₂CH₂CH₂CH₂CH₂SCF₃ | O |
| A-6619 | CH₂CF₃ | CH₂CHF₂ | Me | F | CH₂CH₂CH₂CH₂CH₂CH₂CH₂SCF₃ | O |
| A-6620 | CH₂CF₃ | CH₂CHF₂ | Me | F | CH₂CH₂NHS(=O)₂CF₃ | O |
| A-6621 | CH₂CF₃ | CH₂CHF₂ | Me | F | CH₂CH₂CH₂NHS(=O)₂CF₃ | O |
| A-6622 | CH₂CF₃ | CH₂CHF₂ | Me | F | CH₂CH₂N(Me)S(=O)₂CF₃ | O |
| A-6623 | CH₂CF₃ | CH₂CHF₂ | Me | F | CH₂CH₂N(Et)S(=O)₂CF₃ | O |
| A-6624 | CH₂CF₃ | CH₂CHF₂ | Me | F | CH₂CH₂N(CH₂CN)S(=O)₂CF₃ | O |
| A-6625 | CH₂CF₃ | CH₂CHF₂ | Me | F | CH₂CH₂N(CH₂OMe)S(=O)₂CF₃ | O |
| A-6626 | CH₂CF₃ | CH₂CHF₂ | Me | F | CH₂CH₂CH₂N(Me)S(=O)₂CF₃ | O |
| A-6627 | CH₂CF₃ | CH₂CHF₂ | Me | F | CH₂CH₂CH₂N(Et)S(=O)₂CF₃ | O |
| A-6628 | CH₂CF₃ | CH₂CHF₂ | Me | F | CH₂CH₂CH₂N(CH₂CN)S(=O)₂CF₃ | O |
| A-6629 | CH₂CF₃ | CH₂CHF₂ | Me | F | CH₂CH₂CH₂N(CH₂OMe)S(=O)₂CF₃ | O |
| A-6630 | CH₂CF₃ | CH₂CF₃ | Cl | F | CH₂CH₂CH₂SCF₃ | O |
| A-6631 | CH₂CF₃ | CH₂CF₃ | Cl | F | CH₂CH₂CH₂CH₂SCF₃ | O |
| A-6632 | CH₂CF₃ | CH₂CF₃ | Cl | F | CH₂CH₂CH₂CH₂CH₂SCF₃ | O |
| A-6633 | CH₂CF₃ | CH₂CF₃ | Cl | F | CH₂CH₂CH₂CH₂CH₂CH₂SCF₃ | O |
| A-6634 | CH₂CF₃ | CH₂CF₃ | Cl | F | CH₂CH₂CH₂CH₂CH₂CH₂CH₂SCF₃ | O |
| A-6635 | CH₂CF₃ | CH₂CF₃ | Cl | F | CH₂CH₂NHS(=O)₂CF₃ | O |
| A-6636 | CH₂CF₃ | CH₂CF₃ | Cl | F | CH₂CH₂CH₂NHS(=O)₂CF₃ | O |
| A-6637 | CH₂CF₃ | CH₂CF₃ | Cl | F | CH₂CH₂N(Me)S(=O)₂CF₃ | O |
| A-6638 | CH₂CF₃ | CH₂CF₃ | Cl | F | CH₂CH₂N(Et)S(=O)₂CF₃ | O |

**[Table 152]**

| Compound Number | R¹ | R² | R³ | R⁴ | R⁵ | A |
|---|---|---|---|---|---|---|
| A-6639 | CH₂CF₃ | CH₂CF₃ | Cl | F | CH₂CH₂N(CH₂CN)S(=O)₂CF₃ | O |
| A-6640 | CH₂CF₃ | CH₂CF₃ | Cl | F | CH₂CH₂N(CH₂OMe)S(=O)₂CF₃ | O |
| A-6641 | CH₂CF₃ | CH₂CF₃ | Cl | F | CH₂CH₂CH₂N(Me)S(=O)₂CF₃ | O |
| A-6642 | CH₂CF₃ | CH₂CF₃ | Cl | F | CH₂CH_{Z}CH₂N(Et)S(=O)₂CF₃ | O |
| A-6643 | CH₂CF₃ | CH₂CF₃ | Cl | F | CH₂CH₂CH₂N(CH₂CN)S(=O)₂CF₃ | O |
| A-6644 | CH₂CF₃ | CH₂CF₃ | Cl | F | CH₂CH₂CH₂N(CH₂OMe)S(=O)₂CF₃ | O |
| A-6645 | CH₂CF₃ | CH₂CF₃ | Me | F | CH₂CH₂CH₂SCF₃ | O |
| A-6646 | CH₂CF₃ | CH₂CF₃ | Me | F | CH₂CH₂CH₂CH₂SCF₃ | O |
| A-6647 | CH₂CF₃ | CH₂CF₃ | Me | F | CH₂CH₂CH₂CH₂CH₂SCF₃ | O |
| A-6648 | CH₂CF₃ | CH₂CF₃ | Me | F | CH₂CH₂CH₂CH₂CH₂CH₂SCF₃ | O |
| A-6649 | CH₂CF₃ | CH₂CF₃ | Me | F | CH₂CH₂CH₂CH₂CH₂CH₂CH₂SCF₃ | O |
| A-6650 | CH₂CF₃ | CH₂CF₃ | Me | F | CH₂CH₂NHS(=O)₂CF₃ | O |
| A-6651 | CH₂CF₃ | CH₂CF₃ | Me | F | CH₂CH₂CH₂NHS(=O)₂CF₃ | O |
| A-6652 | CH₂CF₃ | CH₂CF₃ | Me | F | CH₂CH₂N(Me)S(=O)₂CF₃ | O |
| A-6653 | CH₂CF₃ | CH₂CF₃ | Me | F | CH₂CH₂N(Et)S(=O)₂CF₃ | O |
| A-6654 | CH₂CF₃ | CH₂CF₃ | Me | F | CH₂CH₂N(CH₂CN)S(=O)₂CF₃ | O |
| A-6655 | CH₂CF₃ | CH₂CF₃ | Me | F | CH₂CH₂N(CH₂OMe)S(=O)₂CF₃ | O |
| A-6656 | CH₂CF₃ | CH₂CF₃ | Me | F | CH₂CH₂CH₂N(Me)S(=O)₂CF₃ | O |
| A-6657 | CH₂CF₃ | CH₂CF₃ | Me | F | CH₂CH₂CH₂N(Et)S(=O)₂CF₃ | O |
| A-6658 | CH₂CF₃ | CH₂CF₃ | Me | F | CH₂CH₂CH₂N(CH₂CN)S(=O)₂CF₃ | O |
| A-6659 | CH₂CF₃ | CH₂CF₃ | Me | F | CH₂CH₂CH₂N(CH₂OMe)S(=O)₂CF₃ | O |
| A-6660 | CH₂CF₃ | CH₂C≡CH | Cl | F | CH₂CH₂CH₂SCF₃ | O |
| A-6661 | CH₂CF₃ | CH₂C≡CH | Cl | F | CH₂CH₂CH₂CH₂SCF₃ | O |
| A-6662 | CH₂CF₃ | CH₂C≡CH | Cl | F | CH₂CH₂CH₂CH₂CH₂SCF₃ | O |
| A-6663 | CH₂CF₃ | CH₂C≡CH | Cl | F | CH₂CH₂CH₂CH₂CH₂CH₂SCF₃ | O |
| A-6664 | CH₂CF₃ | CH₂C≡CH | Cl | F | CH₂CH₂CH₂CH₂CH₂CH₂CH₂SCF₃ | O |
| A-6665 | CH₂CF₃ | CH₂C≡CH | Cl | F | CH₂CH₂NHS(=O)₂CF₃ | O |
| A-6666 | CH₂CF₃ | CH₂C≡CH | Cl | F | CH₂CH₂CH₂NHS(=O)₂CF₃ | O |
| A-6667 | CH₂CF₃ | CH₂C≡CH | Cl | F | CH₂CH₂N(Me)S(=O)₂CF₃ | O |
| A-6668 | CH₂CF₃ | CH₂C≡CH | Cl | F | CH₂CH₂N(Et)S(=O)₂CF₃ | O |
| A-6669 | CH₂CF₃ | CH₂C≡CH | Cl | F | CH₂CH₂N(CH₂CN)S(=O)₂CF₃ | O |
| A-6670 | CH₂CF₃ | CH₂C≡CH | Cl | F | CH₂CH₂N(CH₂OMe)S(=O)₂CF₃ | O |
| A-6671 | CH₂CF₃ | CH₂C≡CH | Cl | F | CH₂CH₂CH₂N(Me)S(=O)₂CF₃ | O |
| A-6672 | CH₂CF₃ | CH₂C≡CH | Cl | F | CH₂CH₂CH₂N(Et)S(=O)₂CF₃ | O |
| A-6673 | CH₂CF₃ | CH₂C≡CH | Cl | F | CH₂CH₂CH₂N(CH₂CN)S(=O)₂CF₃ | O |
| A-6674 | CH₂CF₃ | CH₂C≡CH | Cl | F | CH₂CH₂CH₂N(CH₂OMe)S(=O)₂CF₃ | O |
| A-6675 | CH₂CF₃ | CH₂C≡CH | Me | F | CH₂CH₂CH₂SCF₃ | O |
| A-6676 | CH₂CF₃ | CH₂C≡CH | Me | F | CH₂CH₂CH₂CH₂SCF₃ | O |
| A-6677 | CH₂CF₃ | CH₂C≡CH | Me | F | CH₂CH₂CH₂CH₂CH₂SCF₃ | O |
| A-6678 | CH₂CF₃ | CH₂C≡CH | Me | F | CH₂CH₂CH₂CH₂CH₂CH₂SCF₃ | O |
| A-6679 | CH₂CF₃ | CH₂C≡CH | Me | F | CH₂CH₂CH₂CH₂CH₂CH₂CH₂SCF₃ | O |
| A-6680 | CH₂CF₃ | CH₂C≡CH | Me | F | CH₂CH₂NHS(=O)₂CF₃ | O |
| A-6681 | CH₂CF₃ | CH₂C≡CH | Me | F | CH₂CH₂CH₂NHS(=O)₂CF₃ | O |
| A-6682 | CH₂CF₃ | CH₂C≡CH | Me | F | CH₂CH₂N(Me)S(=O)₂CF₃ | O |

**[Table 153]**

| Compound Number | R¹ | R² | R³ | R⁴ | R⁵ | A |
|---|---|---|---|---|---|---|
| A-6683 | CH₂CF₃ | CH₂C≡CH | Me | F | CH₂CH₂N(Et)S(=O)₂CF₃ | O |
| A-6684 | CH₂CF₃ | CH₂C≡CH | Me | F | CH₂CH₂N(CH₂CN)S(=O)₂CF₃ | O |
| A-6685 | CH₂CF₃ | CH₂C≡CH | Me | F | CH₂CH₂N(CH₂OMe)S(=O)₂CF₃ | O |
| A-6686 | CH₂CF₃ | CH₂C≡CH | Me | F | CH₂CH₂CH₂N(Me)S(=O)₂CF₃ | O |
| A-6687 | CH₂CF₃ | CH₂C≡CH | Me | F | CH₂CH₂CH₂N(Et)S(=O)₂CF₃ | O |
| A-6688 | CH₂CF₃ | CH₂C≡CH | Me | F | CH₂CH₂CH₂N(CH₂CN)S(=O)₂CF₃ | O |
| A-6689 | CH₂CF₃ | CH₂C≡CH | Me | F | CH₂CH₂CH₂N(CH₂OMe)S(=O)₂CF₃ | O |
| A-6690 | CH₂CF₃ | CH₂CN | Cl | F | CH₂CH₂CH₂SCF₃ | O |
| A-6691 | CH₂CF₃ | CH₂CN | Cl | F | CH₂CH₂CH₂CH₂SCF₃ | O |
| A-6692 | CH₂CF₃ | CH₂CN | Cl | F | CH₂CH₂CH₂CH₂CH₂SCF₃ | O |
| A-6693 | CH₂CF₃ | CH₂CN | Cl | F | CH₂CH₂CH₂CH₂CH₂CH₂SCF₃ | O |
| A-6694 | CH₂CF₃ | CH₂CN | Cl | F | CH₂CH₂CH₂CH₂CH₂CH₂CH₂SCF₃ | O |
| A-6695 | CH₂CF₃ | CH₂CN | Cl | F | CH₂CH₂NHS(=O)₂CF₃ | O |
| A-6696 | CH₂CF₃ | CH₂CN | Cl | F | CH₂CH₂CH₂NHS(=O)₂CF₃ | O |
| A-6697 | CH₂CF₃ | CH₂CN | Cl | F | CH₂CH₂N(Me)S(=O)₂CF₃ | O |
| A-6698 | CH₂CF₃ | CH₂CN | Cl | F | CH₂CH₂N(Et)S(=O)₂CF₃ | O |
| A-6699 | CH₂CF₃ | CH₂CN | Cl | F | CH₂CH₂N(CH₂CN)S(=O)₂CF₃ | O |
| A-6700 | CH₂CF₃ | CH₂CN | Cl | F | CH₂CH₂N(CH₂OMe)S(=O)₂CF₃ | O |
| A-6701 | CH₂CF₃ | CH₂CN | Cl | F | CH₂CH₂CH₂N(Me)S(=O)₂CF₃ | O |
| A-6702 | CH₂CF₃ | CH₂CN | Cl | F | CH₂CH₂CH₂N(Et)S(=O)₂CF₃ | O |
| A-6703 | CH₂CF₃ | CH₂CN | Cl | F | CH₂CH₂CH₂N(CH₂CN)S(=O)₂CF₃ | O |
| A-6704 | CH₂CF₃ | CH₂CN | Cl | F | CH₂CH₂CH₂N(CH₂OMe)S(=O)₂CF₃ | O |
| A-6705 | CH₂CF₃ | CH₂CN | Me | F | CH₂CH₂CH₂SCF₃ | O |
| A-6706 | CH₂CF₃ | CH₂CN | Me | F | CH₂CH₂CH₂CH₂SCF₃ | O |
| A-6707 | CH₂CF₃ | CH₂CN | Me | F | CH₂CH₂CH₂CH₂CH₂SCF₃ | O |
| A-6708 | CH₂CF₃ | CH₂CN | Me | Cl | CH₂CH₂CH₂CH₂CH₂SCF₃ | O |
| A-6709 | CH₂CF₃ | CH₂CN | Me | Me | CH₂CH₂CH₂CH₂CH₂SCF₃ | O |
| A-6710 | CH₂CF₃ | CH₂CN | Me | F | CH₂CH₂CH₂CH₂CH₂CH₂SCF₃ | O |
| A-6711 | CH₂CF₃ | CH₂CN | Me | F | CH₂CH₂CH₂CH₂CH₂CH₂CH₂SCF₃ | O |
| A-6712 | CH₂CF₃ | CH₂CN | Me | F | CH₂CH₂NHS(=O)₂CF₃ | O |
| A-6713 | CH₂CF₃ | CH₂CN | Me | F | CH₂CH₂CH₂NHS(=O)₂CF₃ | O |
| A-6714 | CH₂CF₃ | CH₂CN | Me | F | CH₂CH₂N(Me)S(=O)₂CF₃ | O |
| A-6715 | CH₂CF₃ | CH₂CN | Me | F | CH₂CH₂N(Et)S(=O)₂CF₃ | O |
| A-6716 | CH₂CF₃ | CH₂CN | Me | F | CH₂CH₂N(CH₂CN)S(=O)₂CF₃ | O |
| A-6717 | CH₂CF₃ | CH₂CN | Me | F | CH₂CH₂N(CH₂OMe)S(=O)₂CF₃ | O |
| A-6718 | CH₂CF₃ | CH₂CN | Me | F | CH₂CH₂CH₂N(Me)S(=O)₂CF₃ | O |
| A-6719 | CH₂CF₃ | CH₂CN | Me | F | CH₂CH₂CH₂N(Et)S(=O)₂CF₃ | O |
| A-6720 | CH₂CF₃ | CH₂CN | Me | F | CH₂CH₂CH₂N(CH₂CN)S(=O)₂CF₃ | O |
| A-6721 | CH₂CF₃ | CH₂CN | Me | F | CH₂CH₂CH₂N(CH₂OMe)S(=O)₂CF₃ | O |
| A-6722 | CH₂CF₃ | CH₂CH(OH)CH₃ | Cl | F | CH₂CH₂CH₂SCF₃ | O |
| A-6723 | CH₂CF₃ | CH₂CH(OH)CH₃ | Cl | F | CH₂CH₂CH₂CH₂SCF₃ | O |
| A-6724 | CH₂CF₃ | CH₂CH(OH)CH₃ | Cl | F | CH₂CH₂CH₂CH₂CH₂SCF₃ | O |
| A-6725 | CH₂CF₃ | CH₂CH(OH)CH₃ | Cl | F | CH₂CH₂CH₂CH₂CH₂CH₂SCF₃ | O |
| A-6726 | CH₂CF₃ | CH₂CH(OH)CH₃ | Cl | F | CH₂CH₂CH₂CH₂CH₂CH₂CH₂SCF₃ | O |

**[Table 154]**

| Compound Number | R¹ | R² | R³ | R⁴ | R⁵ | A |
|---|---|---|---|---|---|---|
| A-6727 | CH₂CF₃ | CH₂CH(OH)CH₃ | Cl | F | CH₂CH₂NHS(=O)₂CF₃ | O |
| A-6728 | CH₂CF₃ | CH₂CH(OH)CH₃ | Cl | F | CH₂CH₂CH₂NHS(=O)₂CF₃ | O |
| A-6729 | CH₂CF₃ | CH₂CH(OH)CH₃ | Cl | F | CH₂CH₂N(Me)S(=O)₂CF₃ | O |
| A-6730 | CH₂CF₃ | CH₂CH(OH)CH₃ | Cl | F | CH₂CH₂N(Et)S(=O)₂CF₃ | O |
| A-6731 | CH₂CF₃ | CH₂CH(OH)CH₃ | Cl | F | CH₂CH₂N(CH₂CN)S(=O)₂CF₃ | O |
| A-6732 | CH₂CF₃ | CH₂CH(OH)CH₃ | Cl | F | CH₂CH₂N(CH₂OMe)S(=O)₂CF₃ | O |
| A-6733 | CH₂CF₃ | CH₂CH(OH)CH₃ | Cl | F | CH₂CH₂CH₂N(Me)S(=O)₂CF₃ | ○ |
| A-6734 | CH₂CF₃ | CH₂CH(OH)CH₃ | Cl | F | CH₂CH₂CH₂N(Et)S(=O)₂CF₃ | O |
| A-6735 | CH₂CF₃ | CH₂CH(OH)CH₃ | Cl | F | CH₂CH₂CH₂N(CH₂CN)S(=O)₂CF₃ | O |
| A-6736 | CH₂CF₃ | CH₂CH(OH)CH₃ | Cl | F | CH₂CH₂CH₂N(CH₂OMe)S(=O)₂CF₃ | O |
| A-6737 | CH₂CF₃ | CH₂CH(OH)CH₃ | Me | F | CH₂CH₂CH₂SCF₃ | O |
| A-6738 | CH₂CF₃ | CH₂CH(OH)CH₃ | Me | F | CH₂CH₂CH₂CH₂SCF₃ | O |
| A-6739 | CH₂CF₃ | CH₂CH(OH)CH₃ | Me | F | CH₂CH₂CH₂CH₂CH₂SCF₃ | O |
| A-6740 | CH₂CF₃ | CH₂CH(OH)CH₃ | Me | F | CH₂CH₂CH₂CH₂CH₂CH₂SCF₃ | O |
| A-6741 | CH₂CF₃ | CH₂CH(OH)CH₃ | Me | F | CH₂CH₂CH₂CH₂CH₂CH₂CH₂SCF₃ | O |
| A-6742 | CH₂CF₃ | CH₂CH(OH)CH₃ | Me | F | CH₂CH₂NHS(=O)₂CF₃ | O |
| A-6743 | CH₂CF₃ | CH₂CH(OH)CH₃ | Me | F | CH₂CH₂CH₂NHS(=O)₂CF₃ | O |
| A-6744 | CH₂CF₃ | CH₂CH(OH)CH₃ | Me | F | CH₂CH₂N(Me)S(=O)₂CF₃ | O |
| A-6745 | CH₂CF₃ | CH₂CH(OH)CH₃ | Me | F | CH₂CH₂N(Et)S(=O)₂CF₃ | O |
| A-6746 | CH₂CF₃ | CH₂CH(OH)CH₃ | Me | F | CH₂CH₂N(CH₂CN)S(=O)₂CF₃ | O |
| A-6747 | CH₂CF₃ | CH₂CH(OH)CH₃ | Me | F | CH₂CH₂N(CH₂OMe)S(=O)₂CF₃ | O |
| A-6748 | CH₂CF₃ | CH₂CH(OH)CH₃ | Me | F | CH₂CH₂CH₂N(Me)S(=O)₂CF₃ | O |
| A-6749 | CH₂CF₃ | CH₂CH(OH)CH₃ | Me | F | CH₂CH₂CH₂N(Et)S(=O)₂CF₃ | O |
| A-6750 | CH₂CF₃ | CH₂CH(OH)CH₃ | Me | F | CH₂CH₂CH₂N(CH₂CN)S(=O)₂CF₃ | O |
| A-6751 | CH₂CF₃ | CH₂CH(OH)CH₃ | Me | F | CH₂CH₂CH₂N(CH₂OMe)S(=O)₂CF₃ | O |
| A-6752 | CH₂CF₃ | CH₂OMe | Cl | F | CH₂CH₂CH₂SCF₃ | O |
| A-6753 | CH₂CF₃ | CH₂OMe | Cl | F | CH₂CH₂CH₂CH₂SCF₃ | O |
| A-6754 | CH₂CF₃ | CH₂OMe | Cl | F | CH₂CH₂CH₂CH₂CH₂SCF₃ | O |
| A-6755 | CH₂CF₃ | CH₂OMe | Cl | F | CH₂CH₂CH₂CH₂CH₂CH₂SCF₃ | O |
| A-6756 | CH₂CF₃ | CH₂OMe | Cl | F | CH₂CH₂CH₂CH₂CH₂CH₂CH₂SCF₃ | O |
| A-6757 | CH₂CF₃ | CH₂OMe | Cl | F | CH₂CH₂NHS(=O)₂CF₃ | O |
| A-6758 | CH₂CF₃ | CH₂OMe | Cl | F | CH₂CH₂CH₂NHS(=O)₂CF₃ | O |
| A-6759 | CH₂CF₃ | CH₂OMe | Cl | F | CH₂CH₂N(Me)S(=O)₂CF₃ | O |
| A-6760 | CH₂CF₃ | CH₂OMe | Cl | F | CH₂CH₂N(Et)S(=O)₂CF₃ | O |
| A-6761 | CH₂CF₃ | CH₂OMe | Cl | F | CH₂CH₂N(CH₂CN)S(=O)₂CF₃ | O |
| A-6762 | CH₂CF₃ | CH₂OMe | Cl | F | CH₂CH₂N(CH₂OMe)S(=O)₂CF₃ | O |
| A-6763 | CH₂CF₃ | CH₂OMe | Cl | F | CH₂CH₂CH₂N(Me)S(=O)₂CF₃ | O |
| A-6764 | CH₂CF₃ | CH₂OMe | Cl | F | CH₂CH₂CH₂N(Et)S(=O)₂CF₃ | O |
| A-6765 | CH₂CF₃ | CH₂OMe | Cl | F | CH₂CH₂CH₂N(CH₂CN)S(=O)₂CF₃ | O |
| A-6766 | CH₂CF₃ | CH₂OMe | Cl | F | CH₂CH₂CH₂N(CH₂OMe)S(=O)₂CF₃ | O |
| A-6767 | CH₂CF₃ | CH₂OMe | Me | F | CH₂CH₂CH₂SCF₃ | O |
| A-6768 | CH₂CF₃ | CH₂OMe | Me | F | CH₂CH₂CH₂CH₂SCF₃ | O |
| A-6769 | CH₂CF₃ | CH₂OMe | Me | F | CH₂CH₂CH₂CH₂CH₂SCF₃ | O |
| A-6770 | CH₂CF₃ | CH₂OMe | Me | F | CH₂CH₂CH₂CH₂CH₂CH₂SCF₃ | O |

**[Table 155]**

| Compound Number | R¹ | R² | R³ | R⁴ | R⁵ | A |
|---|---|---|---|---|---|---|
| A-6771 | CH₂CF₃ | CH₂OMe | Me | F | CH₂CH₂CH₂CH₂CH₂CH₂CH₂SCF₃ | O |
| A-6772 | CH₂CF₃ | CH₂OMe | Me | F | CH₂CH₂NHS(=O)₂CF₃ | O |
| A-6773 | CH₂CF₃ | CH₂OMe | Me | F | CH₂CH₂CH₂NHS(=O)₂CF₃ | O |
| A-6774 | CH₂CF₃ | CH₂OMe | Me | F | CH₂CH₂N(Me)S(=O)₂CF₃ | O |
| A-6775 | CH₂CF₃ | CH₂OMe | Me | F | CH₂CH₂N(Et)S(=O)₂CF₃ | O |
| A-6776 | CH₂CF₃ | CH₂OMe | Me | F | CH₂CH₂N(CH₂CN)S(=O)₂CF₃ | O |
| A-6777 | CH₂CF₃ | CH₂OMe | Me | F | CH₂CH₂N(CH₂OMe)S(=O)₂CF₃ | O |
| A-6778 | CH₂CF₃ | CH₂OMe | Me | F | CH₂CH₂CH₂N(Me)S(=O)₂CF₃ | O |
| A-6779 | CH₂CF₃ | CH₂OMe | Me | F | CH₂CH₂CH₂N(Et)S(=O)₂CF₃ | O |
| A-6780 | CH₂CF₃ | CH₂OMe | Me | F | CH₂CH₂CH₂N(CH₂CN)S(=O)₂CF₃ | O |
| A-6781 | CH₂CF₃ | CH₂OMe | Me | F | CH₂CH₂CH₂N(CH₂OMe)S(=O)₂CF₃ | O |
| A-6782 | CH₂CF₃ | CH₂CH₂OMe | Cl | F | CH₂CH₂CH₂SCF₃ | O |
| A-6783 | CH₂CF₃ | CH₂CH₂OMe | Cl | F | CH₂CH₂CH₂CH₂SCF₃ | O |
| A-6784 | CH₂CF₃ | CH₂CH₂OMe | Cl | F | CH₂CH₂CH₂CH₂CH₂SCF₃ | O |
| A-6785 | CH₂CF₃ | CH₂CH₂OMe | Cl | F | CH₂CH₂CH₂CH₂CH₂CH₂SCF₃ | O |
| A-6786 | CH₂CF₃ | CH₂CH₂OMe | Cl | F | CH₂CH₂CH₂CH₂CH₂CH₂CH₂SCF₃ | O |
| A-6787 | CH₂CF₃ | CH₂CH₂OMe | Cl | F | CH₂CH₂NHS(=O)₂CF₃ | O |
| A-6788 | CH₂CF₃ | CH₂CH₂OMe | Cl | F | CH₂CH₂CH₂NHS(=O)₂CF₃ | O |
| A-6789 | CH₂CF₃ | CH₂CH₂OMe | Cl | F | CH₂CH₂N(Me)S(=O)₂CF₃ | O |
| A-6790 | CH₂CF₃ | CH₂CH₂OMe | Cl | F | CH₂CH₂N(Et)S(=O)₂CF₃ | O |
| A-6791 | CH₂CF₃ | CH₂CH₂OMe | Cl | F | CH₂CH₂N(CH₂CN)S(=O)₂CF₃ | O |
| A-6792 | CH₂CF₃ | CH₂CH₂OMe | Cl | F | CH₂CH₂N(CH₂OMe)S(=O)₂CF₃ | O |
| A-6793 | CH₂CF₃ | CH₂CH₂OMe | Cl | F | CH₂CH₂CH₂N(Me)S(=O)₂CF₃ | O |
| A-6794 | CH₂CF₃ | CH₂CH₂OMe | Cl | F | CH₂CH₂CH₂N(Et)S(=O)₂CF₃ | O |
| A-6795 | CH₂CF₃ | CH₂CH₂OMe | Cl | F | CH₂CH₂CH₂N(CH₂CN)S(=O)₂CF₃ | O |
| A-6796 | CH₂CF₃ | CH₂CH₂OMe | Cl | F | CH₂CH₂CH₂N(CH₂OMe)S(=O)₂CF₃ | O |
| A-6797 | CH₂CF₃ | CH₂CH₂OMe | Me | F | CH₂CH₂CH₂SCF₃ | O |
| A-6798 | CH₂CF₃ | CH₂CH₂OMe | Me | F | CH₂CH₂CH₂CH₂SCF₃ | O |
| A-6799 | CH₂CF₃ | CH₂CH₂OMe | Me | F | CH₂CH₂CH₂CH₂CH₂SCF₃ | O |
| A-6800 | CH₂CF₃ | CH₂CH₂OMe | Me | F | CH₂CH₂CH₂CH₂CH₂CH₂SCF₃ | O |
| A-6801 | CH₂CF₃ | CH₂CH₂OMe | Me | F | CH₂CH₂CH₂CH₂CH₂CH₂CH₂SCF₃ | O |
| A-6802 | CH₂CF₃ | CH₂CH₂OMe | Me | F | CH₂CH₂NHS(=O)₂CF₃ | O |
| A-6803 | CH₂CF₃ | CH₂CH₂OMe | Me | F | CH₂CH₂CH₂NHS(=O)₂CF₃ | O |
| A-6804 | CH₂CF₃ | CH₂CH₂OMe | Me | F | CH₂CH₂N(Me)S(=O)₂CF₃ | O |
| A-6805 | CH₂CF₃ | CH₂CH₂OMe | Me | F | CH₂CH₂N(Et)S(=O)₂CF₃ | O |
| A-6806 | CH₂CF₃ | CH₂CH₂OMe | Me | F | CH₂CH₂N(CH₂CN)S(=O)₂CF₃ | O |
| A-6807 | CH₂CF₃ | CH₂CH₂OMe | Me | F | CH₂CH₂N(CH₂OMe)S(=O)₂CF₃ | O |
| A-6808 | CH₂CF₃ | CH₂CH₂OMe | Me | F | CH₂CH₂CH₂N(Me)S(=O)₂CF₃ | O |
| A-6809 | CH₂CF₃ | CH₂CH₂OMe | Me | F | CH₂CH₂CH₂N(Et)S(=O)₂CF₃ | O |
| A-6810 | CH₂CF₃ | CH₂CH₂OMe | Me | F | CH₂CH₂CH₂N(CH₂CN)S(=O)₂CF₃ | O |
| A-6811 | CH₂CF₃ | CH₂CH₂OMe | Me | F | CH₂CH₂CH₂N(CH₂OMe)S(=O)₂CF₃ | O |
| A-6812 | CH₂CF₃ | C(=O)Me | Cl | F | CH₂CH₂CH₂SCF₃ | O |
| A-6813 | CH₂CF₃ | C(=O)Me | Cl | F | CH₂CH₂CH₂CH₂SCF₃ | O |
| A-6814 | CH₂CF₃ | C(=O)Me | Cl | F | CH₂CH₂CH₂CH₂CH₂SCF₃ | O |

**[Table 156]**

| Compound Number | R¹ | R² | R³ | R⁴ | R⁵ | A |
|---|---|---|---|---|---|---|
| A-6815 | CH₂CF₃ | C(=O)Me | Cl | F | CH₂CH₂CH₂CH₂CH₂CH₂SCF₃ | O |
| A-6816 | CH₂CF₃ | C(=O)Me | Cl | F | CH₂CH₂CH₂CH₂CH₂CH₂CH₂SCF₃ | O |
| A-6817 | CH₂CF₃ | C(=O)Me | Cl | F | CH₂CH₂NHS(=O)₂CF₃ | O |
| A-6818 | CH₂CF₃ | C(=O)Me | Cl | F | CH₂CH₂CH₂NHS(=O)₂CF₃ | O |
| A-6819 | CH₂CF₃ | C(=O)Me | Cl | F | CH₂CH₂N(Me)S(=O)₂CF₃ | O |
| A-6820 | CH₂CF₃ | C(=O)Me | Cl | F | CH₂CH₂N(Et)S(=O)₂CF₃ | O |
| A-6821 | CH₂CF₃ | C(=O)Me | Cl | F | CH₂CH₂N(CH₂CN)S(=O)₂CF₃ | O |
| A-6822 | CH₂CF₃ | C(=O)Me | Cl | F | CH₂CH₂N(CH₂OMe)S(=O)₂CF₃ | O |
| A-6823 | CH₂CF₃ | C(=O)Me | Cl | F | CH₂CH₂CH₂N(Me)S(=O)₂CF₃ | O |
| A-6824 | CH₂CF₃ | C(=O)Me | Cl | F | CH₂CH₂CH₂N(Et)S(=O)₂CF₃ | O |
| A-6825 | CH₂CF₃ | C(=O)Me | Cl | F | CH₂CH₂CH₂N(CH₂CN)S(=O)₂CF₃ | O |
| A-6826 | CH₂CF₃ | C(=O)Me | Cl | F | CH₂CH₂CH₂N(CH₂OMe)S(=O)₂CF₃ | O |
| A-6827 | CH₂CF₃ | C(=O)Me | Me | F | CH₂CH₂CH₂SCF₃ | O |
| A-6828 | CH₂CF₃ | C(=O)Me | Me | F | CH₂CH₂CH₂CH₂SCF₃ | O |
| A-6829 | CH₂CF₃ | C(=O)Me | Me | F | CH₂CH₂CH₂CH₂CH₂SCF₃ | O |
| A-6830 | CH₂CF₃ | C(=O)Me | Me | F | CH₂CH₂CH₂CH₂CH₂CH₂SCF₃ | O |
| A-6831 | CH₂CF₃ | C(=O)Me | Me | F | CH₂CH₂CH₂CH₂CH₂CH₂CH₂SCF₃ | O |
| A-6832 | CH₂CF₃ | C(=O)Me | Me | F | CH₂CH₂NHS(=O)₂CF₃ | O |
| A-6833 | CH₂CF₃ | C(=O)Me | Me | F | CH₂CH₂CH₂NHS(=O)₂CF₃ | O |
| A-6834 | CH₂CF₃ | C(=O)Me | Me | F | CH₂CH₂N(Me)S(=O)₂CF₃ | O |
| A-6835 | CH₂CF₃ | C(=O)Me | Me | F | CH₂CH₂N(Et)S(=O)₂CF₃ | O |
| A-6836 | CH₂CF₃ | C(=O)Me | Me | F | CH₂CH₂N(CH₂CN)S(=O)₂CF₃ | O |
| A-6837 | CH₂CF₃ | C(=O)Me | Me | F | CH₂CH₂N(CH₂OMe)S(=O)₂CF₃ | O |
| A-6838 | CH₂CF₃ | C(=O)Me | Me | F | CH₂CH₂CH₂N(Me)S(=O)₂CF₃ | O |
| A-6839 | CH₂CF₃ | C(=O)Me | Me | F | CH₂CH₂CH₂N(Et)S(=O)₂CF₃ | O |
| A-6840 | CH₂CF₃ | C(=O)Me | Me | F | CH₂CH₂CH₂N(CH₂CN)S(=O)₂CF₃ | O |
| A-6841 | CH₂CF₃ | C(=O)Me | Me | F | CH₂CH₂CH₂N(CH₂OMe)S(=O)₂CF₃ | O |
| A-6842 | CH₂CF₃ | C(=O)(*i* -Pr) | Cl | F | CH₂CH₂CH₂SCF₃ | O |
| A-6843 | CH₂CF₃ | C(=O)(*i* -Pr) | Cl | F | CH₂CH₂CH₂CH₂SCF₃ | O |
| A-6844 | CH₂CF₃ | C(=O)(i -Pr) | Cl | F | CH₂CH₂CH₂CH₂CH₂SCF₃ | O |
| A-6845 | CH₂CF₃ | C(=O)(*i* -Pr) | Cl | F | CH₂CH₂CH₂CH₂CH₂CH₂SCF₃ | O |
| A-6846 | CH₂CF₃ | C(=O)(i -Pr) | Cl | F | CH₂CH₂CH₂CH₂CH₂CH₂CH₂SCF₃ | O |
| A-6847 | CH₂CF₃ | C(=O)(*i* -Pr) | Cl | F | CH₂CH₂NHS(=O)₂CF₃ | O |
| A-6848 | CH₂CF₃ | C(=O)(*i* -Pr) | Cl | F | CH₂CH₂CH₂NHS(=O)₂CF₃ | O |
| A-6849 | CH₂CF₃ | C(=O)(i -Pr) | Cl | F | CH₂CH₂N(Me)S(=O)₂CF₃ | O |
| A-6850 | CH₂CF₃ | C(=O)(í -Pr) | Cl | F | CH₂CH₂N(Et)S(=O)₂CF₃ | O |
| A-6851 | CH₂CF₃ | C(=O)(i -Pr) | Cl | F | CH₂CH₂N(CH₂CN)S(=O)₂CF₃ | O |
| A-6852 | CH₂CF₃ | *C(=O)(i* -Pr) | Cl | F | CH₂CH₂N(CH₂OMe)S(=O)₂CF₃ | O |
| A-6853 | CH₂CF₃ | C(=O)(i -Pr) | Cl | F | CH₂CH₂CH₂N(Me)S(=O)₂CF₃ | O |
| A-6854 | CH₂CF₃ | C(=O)(í -Pr) | Cl | F | CH₂CH₂CH₂N(Et)S(=O)₂CF₃ | O |
| A-6855 | CH₂CF₃ | C(=O)(*i* -Pr) | Cl | F | CH₂CH₂CH₂N(CH₂CN)S(=O)₂CF₃ | O |
| A-6856 | CH₂CF₃ | C(=O)(i -Pr) | Cl | F | CH₂CH₂CH₂N(CH₂OMe)S(=O)₂CF₃ | O |
| A-6857 | CH₂CF₃ | C(=O)(i -Pr) | Me | F | CH₂CH₂CH₂SCF₃ | O |
| A-6858 | CH₂CF₃ | C(=O)(*i* -Pr) | Me | F | CH₂CH₂CH₂CH₂SCF₃ | O |

**[Table 157]**

| Compound Number | R¹ | R² | R³ | R⁴ | R⁵ | A |
|---|---|---|---|---|---|---|
| A-6859 | CH₂CF₃ | C(=O)(*i* -Pr) | Me | F | CH₂CH₂CH₂CH₂CH₂SCF₃ | O |
| A-6860 | CH₂CF₃ | C(=O)(*i* -Pr) | Me | F | CH₂CH₂CH₂CH₂CH₂CH₂SCF₃ | O |
| A-6861 | CH₂CF₃ | C(=O)(*i* -Pr) | Me | F | CH₂CH₂CH₂CH₂CH₂CH₂CH₂SCF₃ | O |
| A-6862 | CH₂CF₃ | C(=O)(i -Pr) | Me | F | CH₂CH₂NHS(=O)₂CF₃ | O |
| A-6863 | CH₂CF₃ | C(=O)(i -Pr) | Me | F | CH₂CH₂CH₂NHS(=O)₂CF₃ | O |
| A-6864 | CH₂CF₃ | C(=O)(*i* -Pr) | Me | F | CH₂CH₂N(Me)S(=O)₂CF₃ | O |
| A-6865 | CH₂CF₃ | C(=O)(i -pr) | Me | F | CH₂CH₂N(Et)S(=O)₂CF₃ | O |
| A-6866 | CH₂CF₃ | C(=O)(i -Pr) | Me | F | CH₂CH₂N(CH₂CN)S(=O)₂CF₃ | O |
| A-6867 | CH₂CF₃ | C(=O)(i -pr) | Me | F | CH₂CH₂N(CH₂OMe)S(=O)₂CF₃ | O |
| A-6868 | CH₂CF₃ | C(=O)(i -pr) | Me | F | CH₂CH₂CH₂N(Me)S(=O)₂CF₃ | O |
| A-6869 | CH₂CF₃ | *C(=O)(i* -Pr) | Me | F | CH₂CH₂CH₂N(Et)S(=O)₂CF₃ | O |
| A-6870 | CH₂CF₃ | C(=O)(i -Pr) | Me | F | CH₂CH₂CH₂N(CH₂CN)S(=O)₂CF₃ | O |
| A-6871 | CH₂CF₃ | C(=O)(*i* -Pr) | Me | F | CH₂CH₂CH₂N(CH₂OMe)S(=O)₂CF₃ | O |
| A-6872 | CH₂CF₃ | C(=O)(*t* -Bu) | Cl | F | CH₂CH₂CH₂SCF₃ | O |
| A-6873 | CH₂CF₃ | C(=O)(*t* -Bu) | Cl | F | CH₂CH₂CH₂CH₂SCF₃ | O |
| A-6874 | CH₂CF₃ | C(=O)(*t* -Bu) | Cl | F | CH₂CH₂CH₂CH₂CH₂SCF₃ | O |
| A-6875 | CH₂CF₃ | C(=O)(*t* -Bu) | Cl | F | CH₂CH₂CH₂CH₂CH₂CH₂SCF₃ | O |
| A-6876 | CH₂CF₃ | C(=O)(*t* -Bu) | Cl | F | CH₂CH₂CH₂CH₂CH₂CH₂CH₂SCF₃ | O |
| A-6877 | CH₂CF₃ | C(=O)(*t* -Bu) | Cl | F | CH₂CH₂NHS(=O)₂CF₃ | O |
| A-6878 | CH₂CF₃ | C(=O)(*t* -Bu) | Cl | F | CH₂CH₂CH₂NHS(=O)₂CF₃ | O |
| A-6879 | CH₂CF₃ | C(=O)(*t* -Bu) | Cl | F | CH₂CH₂N(Me)S(=O)₂CF₃ | O |
| A-6880 | CH₂CF₃ | C(=O)(*t* -Bu) | Cl | F | CH₂CH₂N(Et)S(=O)₂CF₃ | O |
| A-6881 | CH₂CF₃ | C(=O)(*t* -Bu) | Cl | F | CH₂CH₂N(CH₂CN)S(=O)₂CF₃ | O |
| A-6882 | CH₂CF₃ | C(=O)(*t* -Bu) | Cl | F | CH₂CH₂N(CH₂OMe)S(=O)₂CF₃ | O |
| A-6883 | CH₂CF₃ | C(=O)(*t* -Bu) | Cl | F | CH₂CH₂CH₂N(Me)S(=O)₂CF₃ | O |
| A-6884 | CH₂CF₃ | C(=O)(*t* -Bu) | Cl | F | CH₂CH₂CH₂N(Et)S(=O)₂CF₃ | O |
| A-6885 | CH₂CF₃ | C(=O)(*t* -Bu) | Cl | F | CH₂CH₂CH₂N(CH₂CN)S(=O)₂CF₃ | O |
| A-6886 | CH₂CF₃ | C(=O)(*t* -Bu) | Cl | F | CH₂CH₂CH₂N(CH₂OMe)S(=O)₂CF₃ | O |
| A-6887 | CH₂CF₃ | C(=O)(*t* -Bu) | Me | F | CH₂CH₂CH₂SCF₃ | O |
| A-6888 | CH₂CF₃ | C(=O)(*t* -Bu) | Me | F | CH₂CH₂CH₂CH₂SCF₃ | O |
| A-6889 | CH₂CF₃ | C(=O)(*t* -Bu) | Me | F | CH₂CH₂CH₂CH₂CH₂SCF₃ | O |
| A-6890 | CH₂CF₃ | C(=O)(*t* -Bu) | Me | F | CH₂CH₂CH₂CH₂CH₂CH₂SCF₃ | O |
| A-6891 | CH₂CF₃ | C(=O)(t-Bu) | Me | F | CH₂CH₂CH₂CH₂CH₂CH₂CH₂SCF₃ | O |
| A-6892 | CH₂CF₃ | C(=O)(*t* -Bu) | Me | F | CH₂CH₂NHS(=O)₂CF₃ | O |
| A-6893 | CH₂CF₃ | C(=O)(*t* -Bu) | Me | F | CH₂CH₂CH₂NHS(=O)₂CF₃ | O |
| A-6894 | CH₂CF₃ | C(=O)(*t* -Bu) | Me | F | CH₂CH₂N(Me)S(=O)₂CF₃ | O |
| A-6895 | CH₂CF₃ | C(=O)(*t* -Bu) | Me | F | CH₂CH₂N(Et)S(=O)₂CF₃ | O |
| A-6896 | CH₂CF₃ | C(=O)(*t* -Bu) | Me | F | CH₂CH₂N(CH₂CN)S(=O)₂CF₃ | O |
| A-6897 | CH₂CF₃ | C(=O)(*t* -Bu) | Me | F | CH₂CH₂N(CH₂OMe)S(=O)₂CF₃ | O |
| A-6898 | CH₂CF₃ | C(=O)(*t* -Bu) | Me | F | CH₂CH₂CH₂N(Me)S(=O)₂CF₃ | O |
| A-6899 | CH₂CF₃ | C(=O)(*t* -Bu) | Me | F | CH₂CH₂CH₂N(Et)S(=O)₂CF₃ | O |
| A-6900 | CH₂CF₃ | C(=O)(*t* -Bu) | Me | F | CH₂CH₂CH₂N(CH₂CN)S(=O)₂CF₃ | O |
| A-6901 | CH₂CF₃ | C(=O)(*t* -Bu) | Me | F | CH₂CH₂CH₂N(CH₂OMe)S(=O)₂CF₃ | O |
| A-6902 | CH₂CF₃ | C(=O)Ph | Cl | F | CH₂CH₂CH₂SCF₃ | O |

**[Table 158]**

| Compound Number | R¹ | R² | R³ | R⁴ | R⁵ | A |
|---|---|---|---|---|---|---|
| A-6903 | CH₂CF₃ | C(=O)Ph | Cl | F | CH₂CH₂CH₂CH₂SCF₃ | O |
| A-6904 | CH₂CF₃ | C(=O)Ph | Cl | F | CH₂CH₂CH₂CH₂CH₂SCF₃ | O |
| A-6905 | CH₂CF₃ | C(=O)Ph | Cl | F | CH₂CH₂CH₂CH₂CH₂CH₂SCF₃ | O |
| A-6906 | CH₂CF₃ | C(=O)Ph | Cl | F | CH₂CH₂CH₂CH₂CH₂CH₂CH₂SCF₃ | O |
| A-6907 | CH₂CF₃ | C(=O)Ph | Cl | F | CH₂CH₂NHS(=O)₂CF₃ | O |
| A-6908 | CH₂CF₃ | C(=O)Ph | Cl | F | CH₂CH₂CH₂NHS(=O)₂CF₃ | O |
| A-6909 | CH₂CF₃ | C(=O)Ph | Cl | F | CH₂CH₂N(Me)S(=O)₂CF₃ | O |
| A-6910 | CH₂CF₃ | C(=O)Ph | Cl | F | CH₂CH₂N(Et)S(=O)₂CF₃ | O |
| A-6911 | CH₂CF₃ | C(=O)Ph | Cl | F | CH₂CH₂N(CH₂CN)S(=O)₂CF₃ | O |
| A-6912 | CH₂CF₃ | C(=O)Ph | Cl | F | CH₂CH₂N(CH₂OMe)S(=O)₂CF₃ | O |
| A-6913 | CH₂CF₃ | C(=O)Ph | Cl | F | CH₂CH₂CH₂N(Me)S(=O)₂CF₃ | O |
| A-6914 | CH₂CF₃ | C(=O)Ph | Cl | F | CH₂CH₂CH₂N(Et)S(=O)₂CF₃ | O |
| A-6915 | CH₂CF₃ | C(=O)Ph | Cl | F | CH₂CH₂CH₂N(CH₂CN)S(=O)₂CF₃ | O |
| A-6916 | CH₂CF₃ | C(=O)Ph | Cl | F | CH₂CH₂CH₂N(CH₂OMe)S(=O)₂CF₃ | O |
| A-6917 | CH₂CF₃ | C(=O)Ph | Me | F | CH₂CH₂CH₂SCF₃ | O |
| A-6918 | CH₂CF₃ | C(=O)Ph | Me | F | CH₂CH₂CH₂CH₂SCF₃ | O |
| A-6919 | CH₂CF₃ | C(=O)Ph | Me | F | CH₂CH₂CH₂CH₂CH₂SCF₃ | O |
| A-6920 | CH₂CF₃ | C(=O)Ph | Me | F | CH₂CH₂CH₂CH₂CH₂CH₂SCF₃ | O |
| A-6921 | CH₂CF₃ | C(=O)Ph | Me | F | CH₂CH₂CH₂CH₂CH₂CH₂CH₂SCF₃ | O |
| A-6922 | CH₂CF₃ | C(=O)Ph | Me | F | CH₂CH₂NHS(=O)₂CF₃ | O |
| A-6923 | CH₂CF₃ | C(=O)Ph | Me | F | CH₂CH₂CH₂NHS(=O)₂CF₃ | O |
| A-6924 | CH₂CF₃ | C(=O)Ph | Me | F | CH₂CH₂N(Me)S(=O)₂CF₃ | O |
| A-6925 | CH₂CF₃ | C(=O)Ph | Me | F | CH₂CH₂N(Et)S(=O)₂CF₃ | O |
| A-6926 | CH₂CF₃ | C(=O)Ph | Me | F | CH₂CH₂N(CH₂CN)S(=O)₂CF₃ | O |
| A-6927 | CH₂CF₃ | C(=O)Ph | Me | F | CH₂CH₂N(CH₂OMe)S(=O)₂CF₃ | O |
| A-6928 | CH₂CF₃ | C(=O)Ph | Me | F | CH₂CH₂CH₂N(Me)S(=O)₂CF₃ | O |
| A-6929 | CH₂CF₃ | C(=O)Ph | Me | F | CH₂CH₂CH₂N(Et)S(=O)₂CF₃ | O |
| A-6930 | CH₂CF₃ | C(=O)Ph | Me | F | CH₂CH₂CH₂N(CH₂CN)S(=O)₂CF₃ | O |
| A-6931 | CH₂CF₃ | C(=O)Ph | Me | F | CH₂CH₂CH₂N(CH₂OMe)S(=O)₂CF₃ | O |
| A-6932 | CH₂CF₃ | C(=O)OEt | Cl | F | CH₂CH₂CH₂SCF₃ | O |
| A-6933 | CH₂CF₃ | C(=O)OEt | Cl | F | CH₂CH₂CH₂CH₂SCF₃ | O |
| A-6934 | CH₂CF₃ | C(=O)OEt | Cl | F | CH₂CH₂CH₂CH₂CH₂SCF₃ | O |
| A-6935 | CH₂CF₃ | C(=O)OEt | Cl | F | CH₂CH₂CH₂CH₂CH₂CH₂SCF₃ | O |
| A-6936 | CH₂CF₃ | C(=O)OEt | Cl | F | CH₂CH₂CH₂CH₂CH₂CH₂CH₂SCF₃ | O |
| A-6937 | CH₂CF₃ | C(=O)OEt | Cl | F | CH₂CH₂NHS(=O)₂CF₃ | O |
| A-6938 | CH₂CF₃ | C(=O)OEt | Cl | F | CH₂CH₂CH₂NHS(=O)₂CF₃ | O |
| A-6939 | CH₂CF₃ | C(=O)OEt | Cl | F | CH₂CH₂N(Me)S(=O)₂CF₃ | O |
| A-6940 | CH₂CF₃ | C(=O)OEt | Cl | F | CH₂CH₂N(Et)S(=O)₂CF₃ | O |
| A-6941 | CH₂CF₃ | C(=O)OEt | Cl | F | CH₂CH₂N(CH₂CN)S(=O)₂CF₃ | O |
| A-6942 | CH₂CF₃ | C(=O)OEt | Cl | F | CH₂CH₂N(CH₂OMe)S(=O)₂CF₃ | O |
| A-6943 | CH₂CF₃ | C(=O)OEt | Cl | F | CH₂CH₂CH₂N(Me)S(=O)₂CF₃ | O |
| A-6944 | CH₂CF₃ | C(=O)OEt | Cl | F | CH₂CH₂CH₂N(Et)S(=O)₂CF₃ | O |
| A-6945 | CH₂CF₃ | C(=O)OEt | Cl | F | CH₂CH₂CH₂N(CH₂CN)S(=O)₂CF₃ | O |
| A-6946 | CH₂CF₃ | C(=O)OEt | Cl | F | CH₂CH₂CH₂N(CH₂OMe)S(=O)₂CF₃ | O |

**[Table 159]**

| Compound Number | R¹ | R² | R³ | R⁴ | R⁵ | A |
|---|---|---|---|---|---|---|
| A-6947 | CH₂CF₃ | C(=O)OEt | Me | F | CH₂CH₂CH₂SCF₃ | O |
| A-6948 | CH₂CF₃ | C(=O)OEt | Me | F | CH₂CH₂CH₂CH₂SCF₃ | O |
| A-6949 | CH₂CF₃ | C(=O)OEt | Me | F | CH₂CH₂CH₂CH₂CH₂SCF₃ | O |
| A-6950 | CH₂CF₃ | C(=O)OEt | Me | F | CH₂CH₂CH₂CH₂CH₂CH₂SCF₃ | O |
| A-6951 | CH₂CF₃ | C(=O)OEt | Me | F | CH₂CH₂CH₂CH₂CH₂CH₂CH₂SCF₃ | O |
| A-6952 | CH₂CF₃ | C(=O)OEt | Me | F | CH₂CH₂NHS(=O)₂CF₃ | O |
| A-6953 | CH₂CF₃ | C(=O)OEt | Me | F | CH₂CH₂CH₂NHS(=O)₂CF₃ | O |
| A-6954 | CH₂CF₃ | C(=O)OEt | Me | F | CH₂CH₂N(Me)S(=O)₂CF₃ | O |
| A-6955 | CH₂CF₃ | C(=O)OEt | Me | F | CH₂CH₂N(Et)S(=O)₂CF₃ | O |
| A-6956 | CH₂CF₃ | C(=O)OEt | Me | F | CH₂CH₂N(CH₂CN)S(=O)₂CF₃ | O |
| A-6957 | CH₂CF₃ | C(=O)OEt | Me | F | CH₂CH₂N(CH₂OMe)S(=O)₂CF₃ | O |
| A-6958 | CH₂CF₃ | C(=O)OEt | Me | F | CH₂CH₂CH₂N(Me)S(=O)₂CF₃ | O |
| A-6959 | CH₂CF₃ | C(=O)OEt | Me | F | CH₂CH₂CH₂N(Et)S(=O)₂CF₃ | O |
| A-6960 | CH₂CF₃ | C(=O)OEt | Me | F | CH₂CH₂CH₂N(CH₂CN)S(=O)₂CF₃ | O |
| A-6961 | CH₂CF₃ | C(=O)OEt | Me | F | CH₂CH₂CH₂N(CH₂OMe)S(=O)₂CF₃ | O |
| A-6962 | CH₂CF₃ | S(=O)₂Me | Cl | F | CH₂CH₂CH₂SCF₃ | O |
| A-6963 | CH₂CF₃ | S(=O)₂Me | Cl | F | CH₂CH₂CH₂CH₂SCF₃ | O |
| A-6964 | CH₂CF₃ | S(=O)₂Me | Cl | F | CH₂CH₂CH₂CH₂CH₂SCF₃ | O |
| A-6965 | CH₂CF₃ | S(=O)₂Me | Cl | F | CH₂CH₂CH₂CH₂CH₂CH₂SCF₃ | O |
| A-6966 | CH₂CF₃ | S(=O)₂Me | Cl | F | CH₂CH₂CH₂CH₂CH₂CH₂CH₂SCF₃ | O |
| A-6967 | CH₂CF₃ | S(=O)₂Me | Cl | F | CH₂CH₂NHS(=O)₂CF₃ | O |
| A-6968 | CH₂CF₃ | S(=O)₂Me | Cl | F | CH₂CH₂CH₂NHS(=O)₂CF₃ | O |
| A-6969 | CH₂CF₃ | S(=O)₂Me | Cl | F | CH₂CH₂N(Me)S(=O)₂CF₃ | O |
| A-6970 | CH₂CF₃ | S(=O)₂Me | Cl | F | CH₂CH₂N(Et)S(=O)₂CF₃ | O |
| A-6971 | CH₂CF₃ | S(=O)₂Me | Cl | F | CH₂CH₂N(CH₂CN)S(=O)₂CF₃ | O |
| A-6972 | CH₂CF₃ | S(=O)₂Me | Cl | F | CH₂CH₂N(CH₂OMe)S(=O)₂CF₃ | O |
| A-6973 | CH₂CF₃ | S(=O)₂Me | Cl | F | CH₂CH₂CH₂N(Me)S(=O)₂CF₃ | O |
| A-6974 | CH₂CF₃ | S(=O)₂Me | Cl | F | CH₂CH₂CH₂N(Et)S(=O)₂CF₃ | O |
| A-6975 | CH₂CF₃ | S(=O)₂Me | Cl | F | CH₂CH₂CH₂N(CH₂CN)S(=O)₂CF₃ | O |
| A-6976 | CH₂CF₃ | S(=O)₂Me | Cl | F | CH₂CH₂CH₂N(CH₂OMe)S(=O)₂CF₃ | O |
| A-6977 | CH₂CF₃ | S(=O)₂Me | Me | F | CH₂CH₂CH₂SCF₃ | O |
| A-6978 | CH₂CF₃ | S(=O)₂Me | Me | F | CH₂CH₂CH₂CH₂SCF₃ | O |
| A-6979 | CH₂CF₃ | S(=O)₂Me | Me | F | CH₂CH₂CH₂CH₂CH₂SCF₃ | O |
| A-6980 | CH₂CF₃ | S(=O)₂Me | Me | F | CH₂CH₂CH₂CH₂CH₂CH₂SCF₃ | O |
| A-6981 | CH₂CF₃ | S(=O)₂Me | Me | F | CH₂CH₂CH₂CH₂CH₂CH₂CH₂SCF₃ | O |
| A-6982 | CH₂CF₃ | S(=O)₂Me | Me | F | CH₂CH₂NHS(=O)₂CF₃ | O |
| A-6983 | CH₂CF₃ | S(=O)₂Me | Me | F | CH₂CH₂CH₂NHS(=O)₂CF₃ | O |
| A-6984 | CH₂CF₃ | S(=O)₂Me | Me | F | CH₂CH₂N(Me)S(=O)₂CF₃ | O |
| A-6985 | CH₂CF₃ | S(=O)₂Me | Me | F | CH₂CH₂N(Et)S(=O)₂CF₃ | O |
| A-6986 | CH₂CF₃ | S(=O)₂Me | Me | F | CH₂CH₂N(CH₂CN)S(=O)₂CF₃ | O |
| A-6987 | CH₂CF₃ | S(=O)₂Me | Me | F | CH₂CH₂N(CH₂OMe)S(=O)₂CF₃ | O |
| A-6988 | CH₂CF₃ | S(=O)₂Me | Me | F | CH₂CH₂CH₂N(Me)S(=O)₂CF₃ | O |
| A-6989 | CH₂CF₃ | S(=O)₂Me | Me | F | CH₂CH₂CH₂N(Et)S(=O)₂CF₃ | O |
| A-6990 | CH₂CF₃ | S(=O)₂Me | Me | F | CH₂CH₂CH₂N(CH₂CN)S(=O)₂CF₃ | O |

**[Table 160]**

| Compound Number | R¹ | R² | R³ | R⁴ | R⁵ | A |
|---|---|---|---|---|---|---|
| A-6991 | CH₂CF₃ | S(=O)₂Me | Me | F | CH₂CH₂CH₂N(CH₂OMe)S(=O)₂CF₃ | O |
| A-6992 | CH₂CF₃ | S(=O)₂CF₃ | Cl | F | CH₂CH₂CH₂SCF₃ | O |
| A-6993 | CH₂CF₃ | S(=O)₂CF₃ | Cl | F | CH₂CH₂CH₂CH₂SCF₃ | O |
| A-6994 | CH₂CF₃ | S(=O)₂CF₃ | Cl | F | CH₂CH₂CH₂CH₂CH₂SCF₃ | O |
| A-6995 | CH₂CF₃ | S(=O)₂CF₃ | Cl | F | CH₂CH₂CH₂CH₂CH₂CH₂SCF₃ | O |
| A-6996 | CH₂CF₃ | S(=O)₂CF₃ | Cl | F | CH₂CH₂CH₂CH₂CH₂CH₂CH₂SCF₃ | O |
| A-6997 | CH₂CF₃ | S(=O)₂CF₃ | Cl | F | CH₂CH₂NHS(=O)₂CF₃ | O |
| A-6998 | CH₂CF₃ | S(=O)₂CF₃ | Cl | F | CH₂CH₂CH₂NHS(=O)₂CF₃ | O |
| A-6999 | CH₂CF₃ | S(=O)₂CF₃ | Cl | F | CH₂CH₂N(Me)S(=O)₂CF₃ | O |
| A-7000 | CH₂CF₃ | S(=O)₂CF₃ | Cl | F | CH₂CH₂N(Et)S(=O)₂CF₃ | O |
| A-7001 | CH₂CF₃ | S(=O)₂CF₃ | Cl | F | CH₂CH₂N(CH₂CN)S(=O)₂CF₃ | O |
| A-7002 | CH₂CF₃ | S(=O)₂CF₃ | Cl | F | CH₂CH₂N(CH₂OMe)S(=O)₂CF₃ | O |
| A-7003 | CH₂CF₃ | S(=O)₂CF₃ | Cl | F | CH₂CH₂CH₂N(Me)S(=O)₂CF₃ | O |
| A-7004 | CH₂CF₃ | S(=O)₂CF₃ | Cl | F | CH₂CH₂CH₂N(Et)S(=O)₂CF₃ | O |
| A-7005 | CH₂CF₃ | S(=O)₂CF₃ | Cl | F | CH₂CH₂CH₂N(CH₂CN)S(=O)₂CF₃ | O |
| A-7006 | CH₂CF₃ | S(=O)₂CF₃ | Cl | F | CH₂CH₂CH₂N(CH₂OMe)S(=O)₂CF₃ | O |
| A-7007 | CH₂CF₃ | S(=O)₂CF₃ | Me | F | CH₂CH₂CH₂SCF₃ | O |
| A-7008 | CH₂CF₃ | S(=O)₂CF₃ | Me | F | CH₂CH₂CH₂CH₂SCF₃ | O |
| A-7009 | CH₂CF₃ | S(=O)₂CF₃ | Me | F | CH₂CH₂CH₂CH₂CH₂SCF₃ | O |
| A-7010 | CH₂CF₃ | S(=O)₂CF₃ | Me | F | CH₂CH₂CH₂CH₂CH₂CH₂SCF₃ | O |
| A-7011 | CH₂CF₃ | S(=O)₂CF₃ | Me | F | CH₂CH₂CH₂CH₂CH₂CH₂CH₂SCF₃ | O |
| A-7012 | CH₂CF₃ | S(=O)₂CF₃ | Me | F | CH₂CH₂NHS(=O)₂CF₃ | O |
| A-7013 | CH₂CF₃ | S(=O)₂CF₃ | Me | F | CH₂CH₂CH₂NHS(=O)₂CF₃ | O |
| A-7014 | CH₂CF₃ | S(=O)₂CF₃ | Me | F | CH₂CH₂N(Me)S(=O)₂CF₃ | O |
| A-7015 | CH₂CF₃ | S(=O)₂CF₃ | Me | F | CH₂CH₂N(Et)S(=O)₂CF₃ | O |
| A-7016 | CH₂CF₃ | S(=O)₂CF₃ | Me | F | CH₂CH₂N(CH₂CN)S(=O)₂CF₃ | O |
| A-7017 | CH₂CF₃ | S(=O)₂CF₃ | Me | F | CH₂CH₂N(CH₂OMe)S(=O)₂CF₃ | O |
| A-7018 | CH₂CF₃ | S(=O)₂CF₃ | Me | F | CH₂CH₂CH₂N(Me)S(=O)₂CF₃ | O |
| A-7019 | CH₂CF₃ | S(=O)₂CF₃ | Me | F | CH₂CH₂CH₂N(Et)S(=O)₂CF₃ | O |
| A-7020 | CH₂CF₃ | S(=O)₂CF₃ | Me | F | CH₂CH₂CH₂N(CH₂CN)S(=O)₂CF₃ | O |
| A-7021 | CH₂CF₃ | S(=O)₂CF₃ | Me | F | CH₂CH₂CH₂N(CH₂OMe)S(=O)₂CF₃ | O |
| A-7022 | CH₂CF₃ | CH₂C(=O)OEt | Cl | F | CH₂CH₂CH₂SCF₃ | O |
| A-7023 | CH₂CF₃ | CH₂C(=O)OEt | Cl | F | CH₂CH₂CH₂CH₂SCF₃ | O |
| A-7024 | CH₂CF₃ | CH₂C(=O)OEt | Cl | F | CH₂CH₂CH₂CH₂CH₂SCF₃ | O |
| A-7025 | CH₂CF₃ | CH₂C(=O)OEt | Cl | F | CH₂CH₂CH₂CH₂CH₂CH₂SCF₃ | O |
| A-7026 | CH₂CF₃ | CH₂C(=O)OEt | Cl | F | CH₂CH₂CH₂CH₂CH₂CH₂CH₂SCF₃ | O |
| A-7027 | CH₂CF₃ | CH₂C(=O)OEt | Cl | F | CH₂CH₂NHS(=O)₂CF₃ | O |
| A-7028 | CH₂CF₃ | CH₂C(=O)OEt | Cl | F | CH₂CH₂CH₂NHS(=O)₂CF₃ | O |
| A-7029 | CH₂CF₃ | CH₂C(=O)OEt | Cl | F | CH₂CH₂N(Me)S(=O)₂CF₃ | O |
| A-7030 | CH₂CF₃ | CH₂C(=O)OEt | Cl | F | CH₂CH₂N(Et)S(=O)₂CF₃ | O |
| A-7031 | CH₂CF₃ | CH₂C(=O)OEt | Cl | F | CH₂CH₂N(CH₂CN)S(=O)₂CF₃ | O |
| A-7032 | CH₂CF₃ | CH₂C(=O)OEt | Cl | F | CH₂CH₂N(CH₂OMe)S(=O)₂CF₃ | O |
| A-7033 | CH₂CF₃ | CH₂C(=O)OEt | Cl | F | CH₂CH₂CH₂N(Me)S(=O)₂CF₃ | O |
| A-7034 | CH₂CF₃ | CH₂C(=O)OEt | Cl | F | CH₂CH₂CH₂N(Et)S(=O)₂CF₃ | O |

**[Table 161]**

| Compound Number | R¹ | R² | R³ | R⁴ | R⁵ | A |
|---|---|---|---|---|---|---|
| A-7035 | CH₂CF₃ | CH₂C(=O)OEt | Cl | F | CH₂CH₂CH₂N(CH₂CN)S(=O)₂CF₃ | O |
| A-7036 | CH₂CF₃ | CH₂C(=O)OEt | Cl | F | CH₂CH₂CH₂N(CH₂OMe)S(=O)₂CF₃ | O |
| A-7037 | CH₂CF₃ | CH₂C(=O)OEt | Me | F | CH₂CH₂CH₂SCF₃ | O |
| A-7038 | CH₂CF₃ | CH₂C(=O)OEt | Me | F | CH₂CH₂CH₂CH₂SCF₃ | O |
| A-7039 | CH₂CF₃ | CH₂C(=O)OEt | Me | F | CH₂CH₂CH₂CH₂CH₂SCF₃ | O |
| A-7040 | CH₂CF₃ | CH₂C(=O)OEt | Me | F | CH₂CH₂CH₂CH₂CH₂CH₂SCF₃ | O |
| A-7041 | CH₂CF₃ | CH₂C(=O)OEt | Me | F | CH₂CH₂CH₂CH₂CH₂CH₂CH₂SCF₃ | O |
| A-7042 | CH₂CF₃ | CH₂C(=O)OEt | Me | F | CH₂CH₂NHS(=O)₂CF₃ | O |
| A-7043 | CH₂CF₃ | CH₂C(=O)OEt | Me | F | CH₂CH₂CH₂NHS(=O)₂CF₃ | O |
| A-7044 | CH₂CF₃ | CH₂C(=O)OEt | Me | F | CH₂CH₂N(Me)S(=O)₂CF₃ | O |
| A-7045 | CH₂CF₃ | CH₂C(=O)OEt | Me | F | CH₂CH₂N(Et)S(=O)₂CF₃ | O |
| A-7046 | CH₂CF₃ | CH₂C(=O)OEt | Me | F | CH₂CH₂N(CH₂CN)S(=O)₂CF₃ | O |
| A-7047 | CH₂CF₃ | CH₂C(=O)OEt | Me | F | CH₂CH₂N(CH₂OMe)S(=O)₂CF₃ | O |
| A-7048 | CH₂CF₃ | CH₂C(=O)OEt | Me | F | CH₂CH₂CH₂N(Me)S(=O)₂CF₃ | O |
| A-7049 | CH₂CF₃ | CH₂C(=O)OEt | Me | F | CH₂CH₂CH₂N(Et)S(=O)₂CF₃ | O |
| A-7050 | CH₂CF₃ | CH₂C(=O)OEt | Me | F | CH₂CH₂CH₂N(CH₂CN)S(=O)₂CF₃ | O |
| A-7051 | CH₂CF₃ | CH₂C(=O)OEt | Me | F | CH₂CH₂CH₂N(CH₂OMe)S(=O)₂CF₃ | O |
| A-7052 | CH₂CF₃ | CH₂C(=O)NH₂ | Cl | F | CH₂CH₂CH₂SCF₃ | O |
| A-7053 | CH₂CF₃ | CH₂C(=O)NH₂ | Cl | F | CH₂CH₂CH₂CH₂SCF₃ | O |
| A-7054 | CH₂CF₃ | CH₂C(=O)NH₂ | Cl | F | CH₂CH₂CH₂CH₂CH₂SCF₃ | O |
| A-7055 | CH₂CF₃ | CH₂C(=O)NH₂ | Cl | F | CH₂CH₂CH₂CH₂CH₂CH₂SCF₃ | O |
| A-7056 | CH₂CF₃ | CH₂C(=O)NH₂ | Cl | F | CH₂CH₂CH₂CH₂CH₂CH₂CH₂SCF₃ | O |
| A-7057 | CH₂CF₃ | CH₂C(=O)NH₂ | Cl | F | CH₂CH₂NHS(=O)₂CF₃ | O |
| A-7058 | CH₂CF₃ | CH₂C(=O)NH₂ | Cl | F | CH₂CH₂CH₂NHS(=O)₂CF₃ | O |
| A-7059 | CH₂CF₃ | CH₂C(=O)NH₂ | Cl | F | CH₂CH₂N(Me)S(=O)₂CF₃ | O |
| A-7060 | CH₂CF₃ | CH₂C(=O)NH₂ | Cl | F | CH₂CH₂N(Et)S(=O)₂CF₃ | O |
| A-7061 | CH₂CF₃ | CH₂C(=O)NH₂ | Cl | F | CH₂CH₂N(CH₂CN)S(=O)₂CF₃ | O |
| A-7062 | CH₂CF₃ | CH₂C(=O)NH₂ | Cl | F | CH₂CH₂N(CH₂OMe)S(=O)₂CF₃ | O |
| A-7063 | CH₂CF₃ | CH₂C(=O)NH₂ | Cl | F | CH₂CH₂CH₂N(Me)S(=O)₂CF₃ | O |
| A-7064 | CH₂CF₃ | CH₂C(=O)NH₂ | Cl | F | CH₂CH₂CH₂N(Et)S(=O)₂CF₃ | O |
| A-7065 | CH₂CF₃ | CH₂C(=O)NH₂ | Cl | F | CH₂CH₂CH₂N(CH₂CN)S(=O)₂CF₃ | O |
| A-7066 | CH₂CF₃ | CH₂C(=O)NH₂ | Cl | F | CH₂CH₂CH₂N(CH₂OMe)S(=O)₂CF₃ | O |
| A-7067 | CH₂CF₃ | CH₂C(=O)NH₂ | Me | F | CH₂CH₂CH₂SCF₃ | O |
| A-7068 | CH₂CF₃ | CH₂C(=O)NH₂ | Me | F | CH₂CH₂CH₂CH₂SCF₃ | O |
| A-7069 | CH₂CF₃ | CH₂C(=O)NH₂ | Me | F | CH₂CH₂CH₂CH₂CH₂SCF₃ | O |
| A-7070 | CH₂CF₃ | CH₂C(=O)NH₂ | Me | F | CH₂CH₂CH₂CH₂CH₂CH₂SCF₃ | O |
| A-7071 | CH₂CF₃ | CH₂C(=O)NH₂ | Me | F | CH₂CH₂CH₂CH₂CH₂CH₂CH₂SCF₃ | O |
| A-7072 | CH₂CF₃ | CH₂C(=O)NH₂ | Me | F | CH₂CH₂NHS(=O)₂CF₃ | O |
| A-7073 | CH₂CF₃ | CH₂C(=O)NH₂ | Me | F | CH₂CH₂CH₂NHS(=O)₂CF₃ | O |
| A-7074 | CH₂CF₃ | CH₂C(=O)NH₂ | Me | F | CH₂CH₂N(Me)S(=O)₂CF₃ | O |
| A-7075 | CH₂CF₃ | CH₂C(=O)NH₂ | Me | F | CH₂CH₂N(Et)S(=O)₂CF₃ | O |
| A-7076 | CH₂CF₃ | CH₂C(=O)NH₂ | Me | F | CH₂CH₂N(CH₂CN)S(=O)₂CF₃ | O |
| A-7077 | CH₂CF₃ | CH₂C(=O)NH₂ | Me | F | CH₂CH₂N(CH₂OMe)S(=O)₂CF₃ | O |
| A-7078 | CH₂CF₃ | CH₂C(=O)NH₂ | Me | F | CH₂CH₂CH₂N(Me)S(=O)₂CF₃ | O |

**[Table 162]**

| Compound Number | R¹ | R² | R³ | R⁴ | R⁵ | A |
|---|---|---|---|---|---|---|
| A-7079 | CH₂CF₃ | CH₂C(=O)NH₂ | Me | F | CH₂CH₂CH₂N(Et)S(=O)₂CF₃ | O |
| A-7080 | CH₂CF₃ | CH₂C(=O)NH₂ | Me | F | CH₂CH₂CH₂N(CH₂CN)S(=O)₂CF₃ | O |
| A-7081 | CH₂CF₃ | CH₂C(=O)NH₂ | Me | F | CH₂CH₂CH₂N(CH₂OMe)S(=O)₂CF₃ | O |
| A-7082 | CH₂CF₃ | CH₂C(=O)NMe₂ | Cl | F | CH₂CH₂CH₂SCF₃ | O |
| A-7083 | CH₂CF₃ | CH₂C(=O)NMe₂ | Cl | F | CH₂CH₂CH₂CH₂SCF₃ | O |
| A-7084 | CH₂CF₃ | CH₂C(=O)NMe₂ | Cl | F | CH₂CH₂CH₂CH₂CH₂SCF₃ | O |
| A-7085 | CH₂CF₃ | CH₂C(=O)NMe₂ | Cl | F | CH₂CH₂CH₂CH₂CH₂CH₂SCF₃ | O |
| A-7086 | CH₂CF₃ | CH₂C(=O)NMe₂ | Cl | F | CH₂CH₂CH₂CH₂CH₂CH₂CH₂SCF₃ | O |
| A-7087 | CH₂CF₃ | CH₂C(=O)NMe₂ | Cl | F | CH₂CH₂NHS(=O)₂CF₃ | O |
| A-7088 | CH₂CF₃ | CH₂C(=O)NMe₂ | Cl | F | CH₂CH₂CH₂NHS(=O)₂CF₃ | O |
| A-7089 | CH₂CF₃ | CH₂C(=O)NMe₂ | Cl | F | CH₂CH₂N(Me)S(=O)₂CF₃ | O |
| A-7090 | CH₂CF₃ | CH₂C(=O)NMe₂ | Cl | F | CH₂CH₂N(Et)S(=O)₂CF₃ | O |
| A-7091 | CH₂CF₃ | CH₂C(=O)NMe₂ | Cl | F | CH₂CH₂N(CH₂CN)S(=O)₂CF₃ | O |
| A-7092 | CH₂CF₃ | CH₂C(=O)NMe₂ | Cl | F | CH₂CH₂N(CH₂OMe)S(=O)₂CF₃ | O |
| A-7093 | CH₂CF₃ | CH₂C(=O)NMe₂ | Cl | F | CH₂CH₂CH₂N(Me)S(=O)₂CF₃ | O |
| A-7094 | CH₂CF₃ | CH₂C(=O)NMe₂ | Cl | F | CH₂CH₂CH₂N(Et)S(=O)₂CF₃ | O |
| A-7095 | CH₂CF₃ | CH₂C(=O)NMe₂ | Cl | F | CH₂CH₂CH₂N(CH₂CN)S(=O)₂CF₃ | O |
| A-7096 | CH₂CF₃ | CH₂C(=O)NMe₂ | Cl | F | CH₂CH₂CH₂N(CH₂OMe)S(=O)₂CF₃ | O |
| A-7097 | CH₂CF₃ | CH₂C(=O)NMe₂ | Me | F | CH₂CH₂CH₂SCF₃ | O |
| A-7098 | CH₂CF₃ | CH₂C(=O)NMe₂ | Me | F | CH₂CH₂CH₂CH₂SCF₃ | O |
| A-7099 | CH₂CF₃ | CH₂C(=O)NMe₂ | Me | F | CH₂CH₂CH₂CH₂CH₂SCF₃ | O |
| A-7100 | CH₂CF₃ | CH₂C(=O)NMe₂ | Me | F | CH₂CH₂CH₂CH₂CH₂CH₂SCF₃ | O |
| A-7101 | CH₂CF₃ | CH₂C(=O)NMe₂ | Me | F | CH₂CH₂CH₂CH₂CH₂CH₂CH₂SCF₃ | O |
| A-7102 | CH₂CF₃ | CH₂C(=O)NMe₂ | Me | F | CH₂CH₂NHS(=O)₂CF₃ | O |
| A-7103 | CH₂CF₃ | CH₂C(=O)NMe₂ | Me | F | CH₂CH₂CH₂NHS(=O)₂CF₃ | O |
| A-7104 | CH₂CF₃ | CH₂C(=O)NMe₂ | Me | F | CH₂CH₂N(Me)S(=O)₂CF₃ | O |
| A-7105 | CH₂CF₃ | CH₂C(=O)NMe₂ | Me | F | CH₂CH₂N(Et)S(=O)₂CF₃ | O |
| A-7106 | CH₂CF₃ | CH₂C(=O)NMe₂ | Me | F | CH₂CH₂N(CH₂CN)S(=O)₂CF₃ | O |
| A-7107 | CH₂CF₃ | CH₂C(=O)NMe₂ | Me | F | CH₂CH₂N(CH₂OMe)S(=O)₂CF₃ | O |
| A-7108 | CH₂CF₃ | CH₂C(=O)NMe₂ | Me | F | CH₂CH₂CH₂N(Me)S(=O)₂CF₃ | O |
| A-7109 | CH₂CF₃ | CH₂C(=O)NMe₂ | Me | F | CH₂CH₂CH₂N(Et)S(=O)₂CF₃ | O |
| A-7110 | CH₂CF₃ | CH₂C(=O)NMe₂ | Me | F | CH₂CH₂CH₂N(CH₂CN)S(=O)₂CF₃ | O |
| A-7111 | CH₂CF₃ | CH₂C(=O)NMe₂ | Me | F | CH₂CH₂CH₂N(CH₂OMe)S(=O)₂CF₃ | O |
| A-7112 | CH₂CF₃ | CH=NOMe | Cl | F | CH₂CH₂CH₂SCF₃ | O |
| A-7113 | CH₂CF₃ | CH=NOMe | Cl | F | CH₂CH₂CH₂CH₂SCF₃ | O |
| A-7114 | CH₂CF₃ | CH=NOMe | Cl | F | CH₂CH₂CH₂CH₂CH₂SCF₃ | O |
| A-7115 | CH₂CF₃ | CH=NOMe | Cl | F | CH₂CH₂CH₂CH₂CH₂CH₂SCF₃ | O |
| A-7116 | CH₂CF₃ | CH=NOMe | Cl | F | CH₂CH₂CH₂CH₂CH₂CH₂CH₂SCF₃ | O |
| A-7117 | CH₂CF₃ | CH=NOMe | Cl | F | CH₂CH₂NHS(=O)₂CF₃ | O |
| A-7118 | CH₂CF₃ | CH=NOMe | Cl | F | CH₂CH₂CH₂NHS(=O)₂CF₃ | O |
| A-7119 | CH₂CF₃ | CH=NOMe | Cl | F | CH₂CH₂N(Me)S(=O)₂CF₃ | O |
| A-7120 | CH₂CF₃ | CH=NOMe | Cl | F | CH₂CH₂N(Et)S(=O)₂CF₃ | O |
| A-7121 | CH₂CF₃ | CH=NOMe | Cl | F | CH₂CH₂N(CH₂CN)S(=O)₂CF₃ | O |
| A-7122 | CH₂CF₃ | CH=NOMe | Cl | F | CH₂CH₂N(CH₂OMe)S(=O)₂CF₃ | O |

**[Table 163]**

| Compound Number | R¹ | R² | R³ | R⁴ | R⁵ | A |
|---|---|---|---|---|---|---|
| A-7123 | CH₂CF₃ | CH=NOMe | Cl | F | CH₂CH₂CH₂N(Me)S(=O)₂CF₃ | O |
| A-7124 | CH₂CF₃ | CH=NOMe | Cl | F | CH₂CH₂CH₂N(Et)S(=O)₂CF₃ | O |
| A-7125 | CH₂CF₃ | CH=NOMe | Cl | F | CH₂CH₂CH₂N(CH₂CN)S(=O)₂CF₃ | O |
| A-7126 | CH₂CF₃ | CH=NOMe | Cl | F | CH₂CH₂CH₂N(CH₂OMe)S(=O)₂CF₃ | O |
| A-7127 | CH₂CF₃ | CH=NOMe | Me | F | CH₂CH₂CH₂SCF₃ | O |
| A-7128 | CH₂CF₃ | CH=NOMe | Me | F | CH₂CH₂CH₂CH₂SCF₃ | O |
| A-7129 | CH₂CF₃ | CH=NOMe | Me | F | CH₂CH₂CH₂CH₂CH₂SCF₃ | O |
| A-7130 | CH₂CF₃ | CH=NOMe | Me | F | CH₂CH₂CH₂CH₂CH₂CH₂SCF₃ | O |
| A-7131 | CH₂CF₃ | CH=NOMe | Me | F | CH₂CH₂CH₂CH₂CH₂CH₂CH₂SCF₃ | O |
| A-7132 | CH₂CF₃ | CH=NOMe | Me | F | CH₂CH₂NHS(=O)₂CF₃ | O |
| A-7133 | CH₂CF₃ | CH=NOMe | Me | F | CH₂CH₂CH₂NHS(=O)₂CF₃ | O |
| A-7134 | CH₂CF₃ | CH=NOMe | Me | F | CH₂CH₂N(Me)S(=O)₂CF₃ | O |
| A-7135 | CH₂CF₃ | CH=NOMe | Me | F | CH₂CH₂N(Et)S(=O)₂CF₃ | O |
| A-7136 | CH₂CF₃ | CH=NOMe | Me | F | CH₂CH₂N(CH₂CN)S(=O)₂CF₃ | O |
| A-7137 | CH₂CF₃ | CH=NOMe | Me | F | CH₂CH₂N(CH₂OMe)S(=O)₂CF₃ | O |
| A-7138 | CH₂CF₃ | CH=NOMe | Me | F | CH₂CH₂CH₂N(Me)S(=O)₂CF₃ | O |
| A-7139 | CH₂CF₃ | CH=NOMe | Me | F | CH₂CH₂CH₂N(Et)S(=O)₂CF₃ | O |
| A-7140 | CH₂CF₃ | CH=NOMe | Me | F | CH₂CH₂CH₂N(CH₂CN)S(=O)₂CF₃ | O |
| A-7141 | CH₂CF₃ | CH=NOMe | Me | F | CH₂CH₂CH₂N(CH₂OMe)S(=O)₂CF₃ | O |
| A-7142 | c -Pr(1-CN) | H | Cl | F | CH₂{*c*-Pr[1-NHS(=O)₂CF₃]} | O |

**[Table 164]**

| 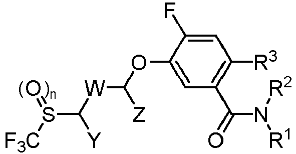 | | | | | | | |
|---|---|---|---|---|---|---|---|
| Compound Number | n | Y | W | Z | R¹ | R² | R³ |
| B-0001 | 0 | H | single bond | Me | CH₂CF₃ | H | Cl |
| B-0002 | 0 | H | single bond | Et | CH₂CF₃ | H | Cl |
| B-0003 | 0 | Me | single bond | H | CH₂CF₃ | H | Cl |
| B-0004 | 0 | Et | single bond | H | CH₂CF₃ | H | Cl |
| B-0005 | 0 | Me | single bond | Me | CH₂CF₃ | H | Cl |
| B-0006 | 0 | H | CH₂ | Me | CH₂CF₃ | H | Cl |
| B-0007 | 0 | Me | CH₂ | H | CH₂CF₃ | H | Cl |
| B-0008 | 0 | Me | CH₂ | Me | CH₂CF₃ | H | Cl |
| B-0009 | 0 | H | CH(Me) | H | CH₂CF₃ | H | Cl |
| B-0010 | 0 | H | CH(Me) | Me | CH₂CF₃ | H | Cl |
| B-0011 | 0 | Me | CH(Me) | H | CH₂CF₃ | H | Cl |
| B-0012 | 0 | Me | CH(Me) | Me | CH₂CF₃ | H | Cl |
| B-0013 | 0 | H | CH₂CH₂ | Me | CH₂CF₃ | H | Cl |
| B-0014 | 0 | Me | CH₂CH₂ | H | CH₂CF₃ | H | Cl |
| B-0015 | 0 | Me | CH₂CH₂ | Me | CH₂CF₃ | H | Cl |
| B-0016 | 0 | H | CH₂CH(Me) | H | CH₂CF₃ | H | Cl |
| B-0017 | 0 | H | CH₂CH(Me) | Me | CH₂CF₃ | H | Cl |
| B-0018 | 0 | Me | CH₂CH(Me) | H | CH₂CF₃ | H | Cl |
| B-0019 | 0 | Me | CH₂CH(Me) | Me | CH₂CF₃ | H | Cl |
| B-0020 | 0 | H | CH(Me)CH₂ | H | CH₂CF₃ | H | Cl |
| B-0021 | 0 | H | CH(Me)CH₂ | Me | CH₂CF₃ | H | Cl |
| B-0022 | 0 | Me | CH(Me)CH₂ | H | CH₂CF₃ | H | Cl |
| B-0023 | 0 | Me | CH(Me)CH₂ | Me | CH₂CF₃ | H | Cl |
| B-0024 | 0 | H | CH₂CH₂CH₂ | Me | CH₂CF₃ | H | Cl |
| B-0025 | 0 | Me | CH₂CH₂CH₂ | H | CH₂CF₃ | H | Cl |
| B-0026 | 0 | Me | CH₂CH₂CH₂ | Me | CH₂CF₃ | H | Cl |
| B-0027 | 0 | H | CH₂CH₂CH(Me) | H | CH₂CF₃ | H | Cl |
| B-0028 | 0 | H | CH₂CH₂CH(Me) | Me | CH₂CF₃ | H | Cl |
| B-0029 | 0 | Me | CH₂CH₂CH(Me) | H | CH₂CF₃ | H | Cl |
| B-0030 | 0 | Me | CH₂CH₂CH(Me) | Me | CH₂CF₃ | H | Cl |
| B-0031 | 0 | H | CH₂CH(Me)CH₂ | H | CH₂CF₃ | H | Cl |
| B-0032 | 0 | H | CH₂CH(Me)CH₂ | Me | CH₂CF₃ | H | Cl |
| B-0033 | 0 | Me | CH₂CH(Me)CH₂ | H | CH₂CF₃ | H | Cl |
| B-0034 | 0 | Me | CH₂CH(Me)CH₂ | Me | CH₂CF₃ | H | Cl |
| B-0035 | 0 | H | CH(Me)CH₂CH₂ | H | CH₂CF₃ | H | Cl |
| B-0036 | 0 | H | CH(Me)CH₂CH₂ | Me | CH₂CF₃ | H | Cl |
| B-0037 | 0 | Me | CH(Me)CH₂CH₂ | H | CH₂CF₃ | H | Cl |
| B-0038 | 0 | Me | CH(Me)CH₂CH₂ | Me | CH₂CF₃ | H | Cl |

**[Table 165]**

| Compound Number | n | Y | W | Z | R¹ | R² | R³ |
|---|---|---|---|---|---|---|---|
| B-0039 | 0 | H | single bond | Me | CH₂CF₃ | H | Me |
| B-0040 | 0 | H | single bond | Et | CH₂CF₃ | H | Me |
| B-0041 | 0 | Me | single bond | H | CH₂CF₃ | H | Me |
| B-0042 | 0 | Et | single bond | H | CH₂CF₃ | H | Me |
| B-0043 | 0 | Me | single bond | Me | CH₂CF₃ | H | Me |
| B-0044 | 0 | H | CH₂ | Me | CH₂CF₃ | H | Me |
| B-0045 | 0 | Me | CH₂ | H | CH₂CF₃ | H | Me |
| B-0046 | 0 | Me | CH₂ | Me | CH₂CF₃ | H | Me |
| B-0047 | 0 | H | CH(Me) | H | CH₂CF₃ | H | Me |
| B-0048 | 0 | H | CH(Me) | Me | CH₂CF₃ | H | Me |
| B-0049 | 0 | Me | CH(Me) | H | CH₂CF₃ | H | Me |
| B-0050 | 0 | Me | CH(Me) | Me | CH₂CF₃ | H | Me |
| B-0051 | 0 | H | CH₂CH₂ | Me | CH₂CF₃ | H | Me |
| B-0052 | 0 | Me | CH₂CH₂ | H | CH₂CF₃ | H | Me |
| B-0053 | 0 | Me | CH₂CH₂ | Me | CH₂CF₃ | H | Me |
| B-0054 | 0 | H | CH₂CH(Me) | H | CH₂CF₃ | H | Me |
| B-0055 | 0 | H | CH₂CH(Me) | Me | CH₂CF₃ | H | Me |
| B-0056 | 0 | Me | CH₂CH(Me) | H | CH₂CF₃ | H | Me |
| B-0057 | 0 | Me | CH₂CH(Me) | Me | CH₂CF₃ | H | Me |
| B-0058 | 0 | H | CH(Me)CH₂ | H | CH₂CF₃ | H | Me |
| B-0059 | 0 | H | CH(Me)CH₂ | Me | CH₂CF₃ | H | Me |
| B-0060 | 0 | Me | CH(Me)CH₂ | H | CH₂CF₃ | H | Me |
| B-0061 | 0 | Me | CH(Me)CH₂ | Me | CH₂CF₃ | H | Me |
| B-0062 | 0 | H | CH₂CH₂CH₂ | Me | CH₂CF₃ | H | Me |
| B-0063 | 0 | Me | CH₂CH₂CH₂ | H | CH₂CF₃ | H | Me |
| B-0064 | 0 | Me | CH₂CH₂CH₂ | Me | CH₂CF₃ | H | Me |
| B-0065 | 0 | H | CH₂CH₂CH(Me) | H | CH₂CF₃ | H | Me |
| B-0066 | 0 | H | CH₂CH₂CH(Me) | Me | CH₂CF₃ | H | Me |
| B-0067 | 0 | Me | CH₂CH₂CH(Me) | H | CH₂CF₃ | H | Me |
| B-0068 | 0 | Me | CH₂CH₂CH(Me) | Me | CH₂CF₃ | H | Me |
| B-0069 | 0 | H | CH₂CH(Me)CH₂ | H | CH₂CF₃ | H | Me |
| B-0070 | 0 | H | CH₂CH(Me)CH₂ | Me | CH₂CF₃ | H | Me |
| B-0071 | 0 | Me | CH₂CH(Me)CH₂ | H | CH₂CF₃ | H | Me |
| B-0072 | 0 | Me | CH₂CH(Me)CH₂ | Me | CH₂CF₃ | H | Me |
| B-0073 | 0 | H | CH(Me)CH₂CH₂ | H | CH₂CF₃ | H | Me |
| B-0074 | 0 | H | CH(Me)CH₂CH₂ | Me | CH₂CF₃ | H | Me |
| B-0075 | 0 | Me | CH(Me)CH₂CH₂ | H | CH₂CF₃ | H | Me |
| B-0076 | 0 | Me | CH(Me)CH₂CH₂ | Me | CH₂CF₃ | H | Me |
| B-0077 | 0 | H | single bond | Me | Et | H | Cl |
| B-0078 | 0 | H | single bond | Et | Et | H | Cl |
| B-0079 | 0 | Me | single bond | H | Et | H | Cl |
| B-0080 | 0 | Et | single bond | H | Et | H | Cl |
| B-0081 | 0 | Me | single bond | Me | Et | H | Cl |
| B-0082 | 0 | H | CH₂ | Me | Et | H | Cl |

**[Table 166]**

| Compound Number | n | Y | W | Z | R¹ | R² | R³ |
|---|---|---|---|---|---|---|---|
| B-0083 | 0 | Me | CH₂ | H | Et | H | Cl |
| B-0084 | 0 | Me | CH₂ | Me | Et | H | Cl |
| B-0085 | 0 | H | CH(Me) | H | Et | H | Cl |
| B-0086 | 0 | H | CH(Me) | Me | Et | H | Cl |
| B-0087 | 0 | Me | CH(Me) | H | Et | H | Cl |
| B-0088 | 0 | Me | CH(Me) | Me | Et | H | Cl |
| B-0089 | 0 | H | CH₂CH₂ | Me | Et | H | Cl |
| B-0090 | 0 | Me | CH₂CH₂ | H | Et | H | Cl |
| B-0091 | 0 | Me | CH₂CH₂ | Me | Et | H | Cl |
| B-0092 | 0 | H | CH₂CH(Me) | H | Et | H | Cl |
| B-0093 | 0 | H | CH₂CH(Me) | Me | Et | H | Cl |
| B-0094 | 0 | Me | CH₂CH(Me) | H | Et | H | Cl |
| B-0095 | 0 | Me | CH₂CH(Me) | Me | Et | H | Cl |
| B-0096 | 0 | H | CH(Me)CH₂ | H | Et | H | Cl |
| B-0097 | 0 | H | CH(Me)CH₂ | Me | Et | H | Cl |
| B-0098 | 0 | Me | CH(Me)CH₂ | H | Et | H | Cl |
| B-0099 | 0 | Me | CH(Me)CH₂ | Me | Et | H | Cl |
| B-0100 | 0 | H | CH₂CH₂CH₂ | Me | Et | H | Cl |
| B-0101 | 0 | Me | CH₂CH₂CH₂ | H | Et | H | Cl |
| B-0102 | 0 | Me | CH₂CH₂CH₂ | Me | Et | H | Cl |
| B-0103 | 0 | H | CH₂CH₂CH(Me) | H | Et | H | Cl |
| B-0104 | 0 | H | CH₂CH₂CH(Me) | Me | Et | H | Cl |
| B-0105 | 0 | Me | CH₂CH₂CH(Me) | H | Et | H | Cl |
| B-0106 | 0 | Me | CH₂CH₂CH(Me) | Me | Et | H | Cl |
| B-0107 | 0 | H | CH₂CH(Me)CH₂ | H | Et | H | Cl |
| B-0108 | 0 | H | CH₂CH(Me)CH₂ | Me | Et | H | Cl |
| B-0109 | 0 | Me | CH₂CH(Me)CH₂ | H | Et | H | Cl |
| B-0110 | 0 | Me | CH₂CH(Me)CH₂ | Me | Et | H | Cl |
| B-0111 | 0 | H | CH(Me)CH₂CH₂ | H | Et | H | Cl |
| B-0112 | 0 | H | CH(Me)CH₂CH₂ | Me | Et | H | Cl |
| B-0113 | 0 | Me | CH(Me)CH₂CH₂ | H | Et | H | Cl |
| B-0114 | 0 | Me | CH(Me)CH₂CH₂ | Me | Et | H | Cl |
| B-0115 | 0 | H | single bond | Me | Et | H | Me |
| B-0116 | 0 | H | single bond | Et | Et | H | Me |
| B-0117 | 0 | Me | single bond | H | Et | H | Me |
| B-0118 | 0 | Et | single bond | H | Et | H | Me |
| B-0119 | 0 | Me | single bond | Me | Et | H | Me |
| B-0120 | 0 | H | CH₂ | Me | Et | H | Me |
| B-0121 | 0 | Me | CH₂ | H | Et | H | Me |
| B-0122 | 0 | Me | CH₂ | Me | Et | H | Me |
| B-0123 | 0 | H | CH(Me) | H | Et | H | Me |
| B-0124 | 0 | H | CH(Me) | Me | Et | H | Me |
| B-0125 | 0 | Me | CH(Me) | H | Et | H | Me |
| B-0126 | 0 | Me | CH(Me) | Me | Et | H | Me |

**[Table 167]**

| Compound Number | n | Y | W | Z | R¹ | R² | R³ |
|---|---|---|---|---|---|---|---|
| B-0127 | 0 | H | CH₂CH₂ | Me | Et | H | Me |
| B-0128 | 0 | Me | CH₂CH₂ | H | Et | H | Me |
| B-0129 | 0 | Me | CH₂CH₂ | Me | Et | H | Me |
| B-0130 | 0 | H | CH₂CH(Me) | H | Et | H | Me |
| B-0131 | 0 | H | CH₂CH(Me) | Me | Et | H | Me |
| B-0132 | 0 | Me | CH₂CH(Me) | H | Et | H | Me |
| B-0133 | 0 | Me | CH₂CH(Me) | Me | Et | H | Me |
| B-0134 | 0 | H | CH(Me)CH₂ | H | Et | H | Me |
| B-0135 | 0 | H | CH(Me)CH₂ | Me | Et | H | Me |
| B-0136 | 0 | Me | CH(Me)CH₂ | H | Et | H | Me |
| B-0137 | 0 | Me | CH(Me)CH₂ | Me | Et | H | Me |
| B-0138 | 0 | H | CH₂CH₂CH₂ | Me | Et | H | Me |
| B-0139 | 0 | Me | CH₂CH₂CH₂ | H | Et | H | Me |
| B-0140 | 0 | Me | CH₂CH₂CH₂ | Me | Et | H | Me |
| B-0141 | 0 | H | CH₂CH₂CH(Me) | H | Et | H | Me |
| B-0142 | 0 | H | CH₂CH₂CH(Me) | Me | Et | H | Me |
| B-0143 | 0 | Me | CH₂CH₂CH(Me) | H | Et | H | Me |
| B-0144 | 0 | Me | CH₂CH₂CH(Me) | Me | Et | H | Me |
| B-0145 | 0 | H | CH₂CH(Me)CH₂ | H | Et | H | Me |
| B-0146 | 0 | H | CH₂CH(Me)CH₂ | Me | Et | H | Me |
| B-0147 | 0 | Me | CH₂CH(Me)CH₂ | H | Et | H | Me |
| B-0148 | 0 | Me | CH₂CH(Me)CH₂ | Me | Et | H | Me |
| B-0149 | 0 | H | CH(Me)CH₂CH₂ | H | Et | H | Me |
| B-0150 | 0 | H | CH(Me)CH₂CH₂ | Me | Et | H | Me |
| B-0151 | 0 | Me | CH(Me)CH₂CH₂ | H | Et | H | Me |
| B-0152 | 0 | Me | CH(Me)CH₂CH₂ | Me | Et | H | Me |
| B-0153 | 0 | H | single bond | Me | c-Pr | H | Cl |
| B-0154 | 0 | H | single bond | Et | c-Pr | H | Cl |
| B-0155 | 0 | Me | single bond | H | c-Pr | H | Cl |
| B-0156 | 0 | Et | single bond | H | c-Pr | H | Cl |
| B-0157 | 0 | Me | single bond | Me | c-Pr | H | Cl |
| B-0158 | 0 | H | CH₂ | Me | c-Pr | H | Cl |
| B-0159 | 0 | Me | CH₂ | H | c-Pr | H | Cl |
| B-0160 | 0 | Me | CH₂ | Me | c-Pr | H | Cl |
| B-0161 | 0 | H | CH(Me) | H | c-Pr | H | Cl |
| B-0162 | 0 | H | CH(Me) | Me | c-Pr | H | Cl |
| B-0163 | 0 | Me | CH(Me) | H | c-Pr | H | Cl |
| B-0164 | 0 | Me | CH(Me) | Me | c-Pr | H | Cl |
| B-0165 | 0 | H | CH₂CH₂ | Me | c-Pr | H | Cl |
| B-0166 | 0 | Me | CH₂CH₂ | H | c-Pr | H | Cl |
| B-0167 | 0 | Me | CH₂CH₂ | Me | c-Pr | H | Cl |
| B-0168 | 0 | H | CH₂CH(Me) | H | c-Pr | H | Cl |
| B-0169 | 0 | H | CH₂CH(Me) | Me | c-Pr | H | Cl |
| B-0170 | 0 | Me | CH₂CH(Me) | H | c-Pr | H | Cl |

**[Table 168]**

| Compound Number | n | Y | W | Z | R¹ | R² | R³ |
|---|---|---|---|---|---|---|---|
| B-0171 | 0 | Me | CH₂CH(Me) | Me | *c*-Pr | H | Cl |
| B-0172 | 0 | H | CH(Me)CH₂ | H | *c*-Pr | H | Cl |
| B-0173 | 0 | H | CH(Me)CH₂ | Me | *c*-Pr | H | Cl |
| B-0174 | 0 | Me | CH(Me)CH₂ | H | *c*-Pr | H | Cl |
| B-0175 | 0 | Me | CH(Me)CH₂ | Me | *c*-Pr | H | Cl |
| B-0176 | 0 | H | CH₂CH₂CH₂ | Me | *c*-Pr | H | Cl |
| B-0177 | 0 | Me | CH₂CH₂CH₂ | H | *c*-Pr | H | Cl |
| B-0178 | 0 | Me | CH₂CH₂CH₂ | Me | *c*-Pr | H | Cl |
| B-0179 | 0 | H | CH₂CH₂CH(Me) | H | *c-*Pr | H | Cl |
| B-0180 | 0 | H | CH₂CH₂CH(Me) | Me | *c*-Pr | H | Cl |
| B-0181 | 0 | Me | CH₂CH₂CH(Me) | H | *c*-Pr | H | Cl |
| B-0182 | 0 | Me | CH₂CH₂CH(Me) | Me | *c*-Pr | H | Cl |
| B-0183 | 0 | H | CH₂CH(Me)CH₂ | H | *c*-Pr | H | Cl |
| B-0184 | 0 | H | CH₂CH(Me)CH₂ | Me | *c*-Pr | H | Cl |
| B-0185 | 0 | Me | CH₂CH(Me)CH₂ | H | *c*-Pr | H | Cl |
| B-0186 | 0 | Me | CH₂CH(Me)CH₂ | Me | *c*-Pr | H | Cl |
| B-0187 | 0 | H | CH(Me)CH₂CH₂ | H | *c*-Pr | H | Cl |
| B-0188 | 0 | H | CH(Me)CH₂CH₂ | Me | *c*-Pr | H | Cl |
| B-0189 | 0 | Me | CH(Me)CH₂CH₂ | H | *c*-Pr | H | Cl |
| B-0190 | 0 | Me | CH(Me)CH₂CH₂ | Me | *c*-Pr | H | Cl |
| B-0191 | 0 | H | single bond | Me | *c*-Pr | H | Me |
| B-0192 | 0 | H | single bond | Et | *c*-Pr | H | Me |
| B-0193 | 0 | Me | single bond | H | *c*-Pr | H | Me |
| B-0194 | 0 | Et | single bond | H | *c*-Pr | H | Me |
| B-0195 | 0 | Me | single bond | Me | *c*-Pr | H | Me |
| B-0196 | 0 | H | CH₂ | Me | *c*-Pr | H | Me |
| B-0197 | 0 | Me | CH₂ | H | *c*-Pr | H | Me |
| B-0198 | 0 | Me | CH₂ | Me | *c-*Pr | H | Me |
| B-0199 | 0 | H | CH(Me) | H | *c*-Pr | H | Me |
| B-0200 | 0 | H | CH(Me) | Me | *c*-Pr | H | Me |
| B-0201 | 0 | Me | CH(Me) | H | *c*-Pr | H | Me |
| B-0202 | 0 | Me | CH(Me) | Me | *c*-Pr | H | Me |
| B-0203 | 0 | H | CH₂CH₂ | Me | *c*-Pr | H | Me |
| B-0204 | 0 | Me | CH₂CH₂ | H | *c*-Pr | H | Me |
| B-0205 | 0 | Me | CH₂CH₂ | Me | *c*-Pr | H | Me |
| B-0206 | 0 | H | CH₂CH(Me) | H | *c*-Pr | H | Me |
| B-0207 | 0 | H | CH₂CH(Me) | Me | *c*-Pr | H | Me |
| B-0208 | 0 | Me | CH₂CH(Me) | H | *c*-Pr | H | Me |
| B-0209 | 0 | Me | CH₂CH(Me) | Me | *c*-Pr | H | Me |
| B-0210 | 0 | H | CH(Me)CH₂ | H | *c*-Pr | H | Me |
| B-0211 | 0 | H | CH(Me)CH₂ | Me | *c*-Pr | H | Me |
| B-0212 | 0 | Me | CH(Me)CH₂ | H | *c*-Pr | H | Me |
| B-0213 | 0 | Me | CH(Me)CH₂ | Me | *c*-Pr | H | Me |
| B-0214 | 0 | H | CH₂CH₂CH₂ | Me | *c*-Pr | H | Me |

**[Table 169]**

| Compound Number | n | Y | W | Z | R¹ | R² | R³ |
|---|---|---|---|---|---|---|---|
| B-0215 | 0 | Me | CH₂CH₂CH₂ | H | *c*-Pr | H | Me |
| B-0216 | 0 | Me | CH₂CH₂CH₂ | Me | *c*-Pr | H | Me |
| B-0217 | 0 | H | CH₂CH₂CH(Me) | H | *c*-Pr | H | Me |
| B-0218 | 0 | H | CH₂CH₂CH(Me) | Me | *c*-Pr | H | Me |
| B-0219 | 0 | Me | CH₂CH₂CH(Me) | H | *c*-Pr | H | Me |
| B-0220 | 0 | Me | CH₂CH₂CH(Me) | Me | *c*-Pr | H | Me |
| B-0221 | 0 | H | CH₂CH(Me)CH₂ | H | *c*-Pr | H | Me |
| B-0222 | 0 | H | CH₂CH(Me)CH₂ | Me | *c*-Pr | H | Me |
| B-0223 | 0 | Me | CH₂CH(Me)CH₂ | H | *c*-Pr | H | Me |
| B-0224 | 0 | Me | CH₂CH(Me)CH₂ | Me | *c*-Pr | H | Me |
| B-0225 | 0 | H | CH(Me)CH₂CH₂ | H | *c*-Pr | H | Me |
| B-0226 | 0 | H | CH(Me)CH₂CH₂ | Me | *c*-Pr | H | Me |
| B-0227 | 0 | Me | CH(Me)CH₂CH₂ | H | *c*-Pr | H | Me |
| B-0228 | 0 | Me | CH(Me)CH₂CH₂ | Me | *c*-Pr | H | Me |
| B-0229 | 1 | H | single bond | Me | CH₂CF₃ | H | Cl |
| B-0230 | 1 | H | single bond | Et | CH₂CF₃ | H | Cl |
| B-0231 | 1 | Me | single bond | H | CH₂CF₃ | H | Cl |
| B-0232 | 1 | Et | single bond | H | CH₂CF₃ | H | Cl |
| B-0233 | 1 | Me | single bond | Me | CH₂CF₃ | H | Cl |
| B-0234 | 1 | H | CH₂ | Me | CH₂CF₃ | H | Cl |
| B-0235 | 1 | Me | CH₂ | H | CH₂CF₃ | H | Cl |
| B-0236 | 1 | Me | CH₂ | Me | CH₂CF₃ | H | Cl |
| B-0237 | 1 | H | CH(Me) | H | CH₂CF₃ | H | Cl |
| B-0238 | 1 | H | CH(Me) | Me | CH₂CF₃ | H | Cl |
| B-0239 | 1 | Me | CH(Me) | H | CH₂CF₃ | H | Cl |
| B-0240 | 1 | Me | CH(Me) | Me | CH₂CF₃ | H | Cl |
| B-0241 | 1 | H | CH₂CH₂ | Me | CH₂CF₃ | H | Cl |
| B-0242 | 1 | Me | CH₂CH₂ | H | CH₂CF₃ | H | Cl |
| B-0243 | 1 | Me | CH₂CH₂ | Me | CH₂CF₃ | H | Cl |
| B-0244 | 1 | H | CH₂CH(Me) | H | CH₂CF₃ | H | Cl |
| B-0245 | 1 | H | CH₂CH(Me) | Me | CH₂CF₃ | H | Cl |
| B-0246 | 1 | Me | CH₂CH(Me) | H | CH₂CF₃ | H | Cl |
| B-0247 | 1 | Me | CH₂CH(Me) | Me | CH₂CF₃ | H | Cl |
| B-0248 | 1 | H | CH(Me)CH₂ | H | CH₂CF₃ | H | Cl |
| B-0249 | 1 | H | CH(Me)CH₂ | Me | CH₂CF₃ | H | Cl |
| B-0250 | 1 | Me | CH(Me)CH₂ | H | CH₂CF₃ | H | Cl |
| B-0251 | 1 | Me | CH(Me)CH₂ | Me | CH₂CF₃ | H | Cl |
| B-0252 | 1 | H | CH₂CH₂CH₂ | Me | CH₂CF₃ | H | Cl |
| B-0253 | 1 | Me | CH₂CH₂CH₂ | H | CH₂CF₃ | H | Cl |
| B-0254 | 1 | Me | CH₂CH₂CH₂ | Me | CH₂CF₃ | H | Cl |
| B-0255 | 1 | H | CH₂CH₂CH(Me) | H | CH₂CF₃ | H | Cl |
| B-0256 | 1 | H | CH₂CH₂CH(Me) | Me | CH₂CF₃ | H | Cl |
| B-0257 | 1 | Me | CH₂CH₂CH(Me) | H | CH₂CF₃ | H | Cl |
| B-0258 | 1 | Me | CH₂CH₂CH(Me) | Me | CH₂CF₃ | H | Cl |

**[Table 170]**

| Compound Number | n | Y | W | Z | R¹ | R² | R³ |
|---|---|---|---|---|---|---|---|
| B-0259 | 1 | H | CH₂CH(Me)CH₂ | H | CH₂CF₃ | H | Cl |
| B-0260 | 1 | H | CH₂CH(Me)CH₂ | Me | CH₂CF₃ | H | Cl |
| B-0261 | 1 | Me | CH₂CH(Me)CH₂ | H | CH₂CF₃ | H | Cl |
| B-0262 | 1 | Me | CH₂CH(Me)CH₂ | Me | CH₂CF₃ | H | Cl |
| B-0263 | 1 | H | CH(Me)CH₂CH₂ | H | CH₂CF₃ | H | Cl |
| B-0264 | 1 | H | CH(Me)CH₂CH₂ | Me | CH₂CF₃ | H | Cl |
| B-0265 | 1 | Me | CH(Me)CH₂CH₂ | H | CH₂CF₃ | H | Cl |
| B-0266 | 1 | Me | CH(Me)CH₂CH₂ | Me | CH₂CF₃ | H | Cl |
| B-0267 | 1 | H | single bond | Me | CH₂CF₃ | H | Me |
| B-0268 | 1 | H | single bond | Et | CH₂CF₃ | H | Me |
| B-0269 | 1 | Me | single bond | H | CH₂CF₃ | H | Me |
| B-0270 | 1 | Et | single bond | H | CH₂CF₃ | H | Me |
| B-0271 | 1 | Me | single bond | Me | CH₂CF₃ | H | Me |
| B-0272 | 1 | H | CH₂ | Me | CH₂CF₃ | H | Me |
| B-0273 | 1 | Me | CH₂ | H | CH₂CF₃ | H | Me |
| B-0274 | 1 | Me | CH₂ | Me | CH₂CF₃ | H | Me |
| B-0275 | 1 | H | CH(Me) | H | CH₂CF₃ | H | Me |
| B-0276 | 1 | H | CH(Me) | Me | CH₂CF₃ | H | Me |
| B-0277 | 1 | Me | CH(Me) | H | CH₂CF₃ | H | Me |
| B-0278 | 1 | Me | CH(Me) | Me | CH₂CF₃ | H | Me |
| B-0279 | 1 | H | CH₂CH₂ | Me | CH₂CF₃ | H | Me |
| B-0280 | 1 | Me | CH₂CH₂ | H | CH₂CF₃ | H | Me |
| B-0281 | 1 | Me | CH₂CH₂ | Me | CH₂CF₃ | H | Me |
| B-0282 | 1 | H | CH₂CH(Me) | H | CH₂CF₃ | H | Me |
| B-0283 | 1 | H | CH₂CH(Me) | Me | CH₂CF₃ | H | Me |
| B-0284 | 1 | Me | CH₂CH(Me) | H | CH₂CF₃ | H | Me |
| B-0285 | 1 | Me | CH₂CH(Me) | Me | CH₂CF₃ | H | Me |
| B-0286 | 1 | H | CH(Me)CH₂ | H | CH₂CF₃ | H | Me |
| B-0287 | 1 | H | CH(Me)CH₂ | Me | CH₂CF₃ | H | Me |
| B-0288 | 1 | Me | CH(Me)CH₂ | H | CH₂CF₃ | H | Me |
| B-0289 | 1 | Me | CH(Me)CH₂ | Me | CH₂CF₃ | H | Me |
| B-0290 | 1 | H | CH₂CH₂CH₂ | Me | CH₂CF₃ | H | Me |
| B-0291 | 1 | Me | CH₂CH₂CH₂ | H | CH₂CF₃ | H | Me |
| B-0292 | 1 | Me | CH₂CH₂CH₂ | Me | CH₂CF₃ | H | Me |
| B-0293 | 1 | H | CH₂CH₂CH(Me) | H | CH₂CF₃ | H | Me |
| B-0294 | 1 | H | CH₂CH₂CH(Me) | Me | CH₂CF₃ | H | Me |
| B-0295 | 1 | Me | CH₂CH₂CH(Me) | H | CH₂CF₃ | H | Me |
| B-0296 | 1 | Me | CH₂CH₂CH(Me) | Me | CH₂CF₃ | H | Me |
| B-0297 | 1 | H | CH₂CH(Me)CH₂ | H | CH₂CF₃ | H | Me |
| B-0298 | 1 | H | CH₂CH(Me)CH₂ | Me | CH₂CF₃ | H | Me |
| B-0299 | 1 | Me | CH₂CH(Me)CH₂ | H | CH₂CF₃ | H | Me |
| B-0300 | 1 | Me | CH₂CH(Me)CH₂ | Me | CH₂CF₃ | H | Me |
| B-0301 | 1 | H | CH(Me)CH₂CH₂ | H | CH₂CF₃ | H | Me |
| B-0302 | 1 | H | CH(Me)CH₂CH₂ | Me | CH₂CF₃ | H | Me |

**[Table 171]**

| Compound Number | n | Y | W | Z | R¹ | R² | R³ |
|---|---|---|---|---|---|---|---|
| B-0303 | 1 | Me | CH(Me)CH₂CH₂ | H | CH₂CF₃ | H | Me |
| B-0304 | 1 | Me | CH(Me)CH₂CH₂ | Me | CH₂CF₃ | H | Me |
| B-0305 | 1 | H | single bond | Me | Et | H | Cl |
| B-0306 | 1 | H | single bond | Et | Et | H | Cl |
| B-0307 | 1 | Me | single bond | H | Et | H | Cl |
| B-0308 | 1 | Et | single bond | H | Et | H | Cl |
| B-0309 | 1 | Me | single bond | Me | Et | H | Cl |
| B-0310 | 1 | H | CH₂ | Me | Et | H | Cl |
| B-0311 | 1 | Me | CH₂ | H | Et | H | Cl |
| B-0312 | 1 | Me | CH₂ | Me | Et | H | Cl |
| B-0313 | 1 | H | CH(Me) | H | Et | H | Cl |
| B-0314 | 1 | H | CH(Me) | Me | Et | H | Cl |
| B-0315 | 1 | Me | CH(Me) | H | Et | H | Cl |
| B-0316 | 1 | Me | CH(Me) | Me | Et | H | Cl |
| B-0317 | 1 | H | CH₂CH₂ | Me | Et | H | Cl |
| B-0318 | 1 | Me | CH₂CH₂ | H | Et | H | Cl |
| B-0319 | 1 | Me | CH₂CH₂ | Me | Et | H | Cl |
| B-0320 | 1 | H | CH₂CH(Me) | H | Et | H | Cl |
| B-0321 | 1 | H | CH₂CH(Me) | Me | Et | H | Cl |
| B-0322 | 1 | Me | CH₂CH(Me) | H | Et | H | Cl |
| B-0323 | 1 | Me | CH₂CH(Me) | Me | Et | H | Cl |
| B-0324 | 1 | H | CH(Me)CH₂ | H | Et | H | Cl |
| B-0325 | 1 | H | CH(Me)CH₂ | Me | Et | H | Cl |
| B-0326 | 1 | Me | CH(Me)CH₂ | H | Et | H | Cl |
| B-0327 | 1 | Me | CH(Me)CH₂ | Me | Et | H | Cl |
| B-0328 | 1 | H | CH₂CH₂CH₂ | Me | Et | H | Cl |
| B-0329 | 1 | Me | CH₂CH₂CH₂ | H | Et | H | Cl |
| B-0330 | 1 | Me | CH₂CH₂CH₂ | Me | Et | H | Cl |
| B-0331 | 1 | H | CH₂CH₂CH(Me) | H | Et | H | Cl |
| B-0332 | 1 | H | CH₂CH₂CH(Me) | Me | Et | H | Cl |
| B-0333 | 1 | Me | CH₂CH₂CH(Me) | H | Et | H | Cl |
| B-0334 | 1 | Me | CH₂CH₂CH(Me) | Me | Et | H | Cl |
| B-0335 | 1 | H | CH₂CH(Me)CH₂ | H | Et | H | Cl |
| B-0336 | 1 | H | CH₂CH(Me)CH₂ | Me | Et | H | Cl |
| B-0337 | 1 | Me | CH₂CH(Me)CH₂ | H | Et | H | Cl |
| B-0338 | 1 | Me | CH₂CH(Me)CH₂ | Me | Et | H | Cl |
| B-0339 | 1 | H | CH(Me)CH₂CH₂ | H | Et | H | Cl |
| B-0340 | 1 | H | CH(Me)CH₂CH₂ | Me | Et | H | Cl |
| B-0341 | 1 | Me | CH(Me)CH₂CH₂ | H | Et | H | Cl |
| B-0342 | 1 | Me | CH(Me)CH₂CH₂ | Me | Et | H | Cl |
| B-0343 | 1 | H | single bond | Me | Et | H | Me |
| B-0344 | 1 | H | single bond | Et | Et | H | Me |
| B-0345 | 1 | Me | single bond | H | Et | H | Me |
| B-0346 | 1 | Et | single bond | H | Et | H | Me |

**[Table 172]**

| Compound Number | n | Y | W | Z | R¹ | R² | R³ |
|---|---|---|---|---|---|---|---|
| B-0347 | 1 | Me | single bond | Me | Et | H | Me |
| B-0348 | 1 | H | CH₂ | Me | Et | H | Me |
| B-0349 | 1 | Me | CH₂ | H | Et | H | Me |
| B-0350 | 1 | Me | CH₂ | Me | Et | H | Me |
| B-0351 | 1 | H | CH(Me) | H | Et | H | Me |
| B-0352 | 1 | H | CH(Me) | Me | Et | H | Me |
| B-0353 | 1 | Me | CH(Me) | H | Et | H | Me |
| B-0354 | 1 | Me | CH(Me) | Me | Et | H | Me |
| B-0355 | 1 | H | CH₂CH₂ | Me | Et | H | Me |
| B-0356 | 1 | Me | CH₂CH₂ | H | Et | H | Me |
| B-0357 | 1 | Me | CH₂CH₂ | Me | Et | H | Me |
| B-0358 | 1 | H | CH₂CH(Me) | H | Et | H | Me |
| B-0359 | 1 | H | CH₂CH(Me) | Me | Et | H | Me |
| B-0360 | 1 | Me | CH₂CH(Me) | H | Et | H | Me |
| B-0361 | 1 | Me | CH₂CH(Me) | Me | Et | H | Me |
| B-0362 | 1 | H | CH(Me)CH₂ | H | Et | H | Me |
| B-0363 | 1 | H | CH(Me)CH₂ | Me | Et | H | Me |
| B-0364 | 1 | Me | CH(Me)CH₂ | H | Et | H | Me |
| B-0365 | 1 | Me | CH(Me)CH₂ | Me | Et | H | Me |
| B-0366 | 1 | H | CH₂CH₂CH₂ | Me | Et | H | Me |
| B-0367 | 1 | Me | CH₂CH₂CH₂ | H | Et | H | Me |
| B-0368 | 1 | Me | CH₂CH₂CH₂ | Me | Et | H | Me |
| B-0369 | 1 | H | CH₂CH₂CH(Me) | H | Et | H | Me |
| B-0370 | 1 | H | CH₂CH₂CH(Me) | Me | Et | H | Me |
| B-0371 | 1 | Me | CH₂CH₂CH(Me) | H | Et | H | Me |
| B-0372 | 1 | Me | CH₂CH₂CH(Me) | Me | Et | H | Me |
| B-0373 | 1 | H | CH₂CH(Me)CH₂ | H | Et | H | Me |
| B-0374 | 1 | H | CH₂CH(Me)CH₂ | Me | Et | H | Me |
| B-0375 | 1 | Me | CH₂CH(Me)CH₂ | H | Et | H | Me |
| B-0376 | 1 | Me | CH₂CH(Me)CH₂ | Me | Et | H | Me |
| B-0377 | 1 | H | CH(Me)CH₂CH₂ | H | Et | H | Me |
| B-0378 | 1 | H | CH(Me)CH₂CH₂ | Me | Et | H | Me |
| B-0379 | 1 | Me | CH(Me)CH₂CH₂ | H | Et | H | Me |
| B-0380 | 1 | Me | CH(Me)CH₂CH₂ | Me | Et | H | Me |
| B-0381 | 1 | H | single bond | Me | *c*-Pr | H | Cl |
| B-0382 | 1 | H | single bond | Et | *c*-Pr | H | Cl |
| B-0383 | 1 | Me | single bond | H | *c*-Pr | H | Cl |
| B-0384 | 1 | Et | single bond | H | *c*-Pr | H | Cl |
| B-0385 | 1 | Me | single bond | Me | *c*-Pr | H | Cl |
| B-0386 | 1 | H | CH₂ | Me | *c*-Pr | H | Cl |
| B-0387 | 1 | Me | CH₂ | H | *c*-Pr | H | Cl |
| B-0388 | 1 | Me | CH₂ | Me | *c*-Pr | H | Cl |
| B-0389 | 1 | H | CH(Me) | H | *c*-Pr | H | Cl |
| B-0390 | 1 | H | CH(Me) | Me | *c*-Pr | H | Cl |

**[Table 173]**

| Compound Number | n | Y | W | Z | R¹ | R² | R³ |
|---|---|---|---|---|---|---|---|
| B-0391 | 1 | Me | CH(Me) | H | *c*-Pr | H | Cl |
| B-0392 | 1 | Me | CH(Me) | Me | *c*-Pr | H | Cl |
| B-0393 | 1 | H | CH₂CH₂ | Me | *c*-Pr | H | Cl |
| B-0394 | 1 | Me | CH₂CH₂ | H | *c*-Pr | H | Cl |
| B-0395 | 1 | Me | CH₂CH₂ | Me | *c*-Pr | H | Cl |
| B-0396 | 1 | H | CH₂CH(Me) | H | *c*-Pr | H | Cl |
| B-0397 | 1 | H | CH₂CH(Me) | Me | *c*-Pr | H | Cl |
| B-0398 | 1 | Me | CH₂CH(Me) | H | *c*-Pr | H | Cl |
| B-0399 | 1 | Me | CH₂CH(Me) | Me | *c*-Pr | H | Cl |
| B-0400 | 1 | H | CH(Me)CH₂ | H | *c*-Pr | H | Cl |
| B-0401 | 1 | H | CH(Me)CH₂ | Me | *c*-Pr | H | Cl |
| B-0402 | 1 | Me | CH(Me)CH₂ | H | *c*-Pr | H | Cl |
| B-0403 | 1 | Me | CH(Me)CH₂ | Me | *c*-Pr | H | Cl |
| B-0404 | 1 | H | CH₂CH₂CH₂ | Me | *c*-Pr | H | Cl |
| B-0405 | 1 | Me | CH₂CH₂CH₂ | H | *c*-Pr | H | Cl |
| B-0406 | 1 | Me | CH₂CH₂CH₂ | Me | *c*-Pr | H | Cl |
| B-0407 | 1 | H | CH₂CH₂CH(Me) | H | *c*-Pr | H | Cl |
| B-0408 | 1 | H | CH₂CH₂CH(Me) | Me | *c*-Pr | H | Cl |
| B-0409 | 1 | Me | CH₂CH₂CH(Me) | H | *c*-Pr | H | Cl |
| B-0410 | 1 | Me | CH₂CH₂CH(Me) | Me | *c*-Pr | H | Cl |
| B-0411 | 1 | H | CH₂CH(Me)CH₂ | H | *c-*Pr | H | Cl |
| B-0412 | 1 | H | CH₂CH(Me)CH₂ | Me | *c*-Pr | H | Cl |
| B-0413 | 1 | Me | CH₂CH(Me)CH₂ | H | *c*-Pr | H | Cl |
| B-0414 | 1 | Me | CH₂CH(Me)CH₂ | Me | *c*-Pr | H | Cl |
| B-0415 | 1 | H | CH(Me)CH₂CH₂ | H | *c*-Pr | H | Cl |
| B-0416 | 1 | H | CH(Me)CH₂CH₂ | Me | *c*-Pr | H | Cl |
| B-0417 | 1 | Me | CH(Me)CH₂CH₂ | H | *c*-Pr | H | Cl |
| B-0418 | 1 | Me | CH(Me)CH₂CH₂ | Me | *c*-Pr | H | Cl |
| B-0419 | 1 | H | single bond | Me | *c*-Pr | H | Me |
| B-0420 | 1 | H | single bond | Et | *c*-Pr | H | Me |
| B-0421 | 1 | Me | single bond | H | *c*-Pr | H | Me |
| B-0422 | 1 | Et | single bond | H | *c*-Pr | H | Me |
| B-0423 | 1 | Me | single bond | Me | *c*-Pr | H | Me |
| B-0424 | 1 | H | CH₂ | Me | *c*-Pr | H | Me |
| B-0425 | 1 | Me | CH₂ | H | *c*-Pr | H | Me |
| B-0426 | 1 | Me | CH₂ | Me | *c*-Pr | H | Me |
| B-0427 | 1 | H | CH(Me) | H | *c*-Pr | H | Me |
| B-0428 | 1 | H | CH(Me) | Me | *c*-Pr | H | Me |
| B-0429 | 1 | Me | CH(Me) | H | *c*-Pr | H | Me |
| B-0430 | 1 | Me | CH(Me) | Me | *c*-Pr | H | Me |
| B-0431 | 1 | H | CH₂CH₂ | Me | *c*-Pr | H | Me |
| B-0432 | 1 | Me | CH₂CH₂ | H | *c*-Pr | H | Me |
| B-0433 | 1 | Me | CH₂CH₂ | Me | *c*-Pr | H | Me |
| B-0434 | 1 | H | CH₂CH(Me) | H | *c*-Pr | H | Me |

**[Table 174]**

| Compound Number | n | Y | W | Z | R¹ | R² | R³ |
|---|---|---|---|---|---|---|---|
| B-0435 | 1 | H | CH₂CH(Me) | Me | *c*-Pr | H | Me |
| B-0436 | 1 | Me | CH₂CH(Me) | H | *c*-Pr | H | Me |
| B-0437 | 1 | Me | CH₂CH(Me) | Me | *c*-Pr | H | Me |
| B-0438 | 1 | H | CH(Me)CH₂ | H | *c*-Pr | H | Me |
| B-0439 | 1 | H | CH(Me)CH₂ | Me | *c*-Pr | H | Me |
| B-0440 | 1 | Me | CH(Me)CH₂ | H | *c*-Pr | H | Me |
| B-0441 | 1 | Me | CH(Me)CH₂ | Me | *c*-Pr | H | Me |
| B-0442 | 1 | H | CH₂CH₂CH₂ | Me | *c*-Pr | H | Me |
| B-0443 | 1 | Me | CH₂CH₂CH₂ | H | *c*-Pr | H | Me |
| B-0444 | 1 | Me | CH₂CH₂CH₂ | Me | *c*-Pr | H | Me |
| B-0445 | 1 | H | CH₂CH₂CH(Me) | H | *c*-Pr | H | Me |
| B-0446 | 1 | H | CH₂CH₂CH(Me) | Me | *c*-Pr | H | Me |
| B-0447 | 1 | Me | CH₂CH₂CH(Me) | H | *c*-Pr | H | Me |
| B-0448 | 1 | Me | CH₂CH₂CH(Me) | Me | *c*-Pr | H | Me |
| B-0449 | 1 | H | CH₂CH(Me)CH₂ | H | *c*-Pr | H | Me |
| B-0450 | 1 | H | CH₂CH(Me)CH₂ | Me | *c*-Pr | H | Me |
| B-0451 | 1 | Me | CH₂CH(Me)CH₂ | H | *c*-Pr | H | Me |
| B-0452 | 1 | Me | CH₂CH(Me)CH₂ | Me | *c*-Pr | H | Me |
| B-0453 | 1 | H | CH(Me)CH₂CH₂ | H | *c*-Pr | H | Me |
| B-0454 | 1 | H | CH(Me)CH₂CH₂ | Me | *c*-Pr | H | Me |
| B-0455 | 1 | Me | CH(Me)CH₂CH₂ | H | *c*-Pr | H | Me |
| B-0456 | 1 | Me | CH(Me)CH₂CH₂ | Me | *c*-Pr | H | Me |
| B-0457 | 2 | H | single bond | Me | CH₂CF₃ | H | Cl |
| B-0458 | 2 | H | single bond | Et | CH₂CF₃ | H | Cl |
| B-0459 | 2 | Me | single bond | H | CH₂CF₃ | H | Cl |
| B-0460 | 2 | Et | single bond | H | CH₂CF₃ | H | Cl |
| B-0461 | 2 | Me | single bond | Me | CH₂CF₃ | H | Cl |
| B-0462 | 2 | H | CH₂ | Me | CH₂CF₃ | H | Cl |
| B-0463 | 2 | Me | CH₂ | H | CH₂CF₃ | H | Cl |
| B-0464 | 2 | Me | CH₂ | Me | CH₂CF₃ | H | Cl |
| B-0465 | 2 | H | CH(Me) | H | CH₂CF₃ | H | Cl |
| B-0466 | 2 | H | CH(Me) | Me | CH₂CF₃ | H | Cl |
| B-0467 | 2 | Me | CH(Me) | H | CH₂CF₃ | H | Cl |
| B-0468 | 2 | Me | CH(Me) | Me | CH₂CF₃ | H | Cl |
| B-0469 | 2 | H | CH₂CH₂ | Me | CH₂CF₃ | H | Cl |
| B-0470 | 2 | Me | CH₂CH₂ | H | CH₂CF₃ | H | Cl |
| B-0471 | 2 | Me | CH₂CH₂ | Me | CH₂CF₃ | H | Cl |
| B-0472 | 2 | H | CH₂CH(Me) | H | CH₂CF₃ | H | Cl |
| B-0473 | 2 | H | CH₂CH(Me) | Me | CH₂CF₃ | H | Cl |
| B-0474 | 2 | Me | CH₂CH(Me) | H | CH₂CF₃ | H | Cl |
| B-0475 | 2 | Me | CH₂CH(Me) | Me | CH₂CF₃ | H | Cl |
| B-0476 | 2 | H | CH(Me)CH₂ | H | CH₂CF₃ | H | Cl |
| B-0477 | 2 | H | CH(Me)CH₂ | Me | CH₂CF₃ | H | Cl |
| B-0478 | 2 | Me | CH(Me)CH₂ | H | CH₂CF₃ | H | Cl |

**[Table 175]**

| Compound Number | n | Y | W | Z | R¹ | R² | R³ |
|---|---|---|---|---|---|---|---|
| B-0479 | 2 | Me | CH(Me)CH₂ | Me | CH₂CF₃ | H | Cl |
| B-0480 | 2 | H | CH₂CH₂CH₂ | Me | CH₂CF₃ | H | Cl |
| B-0481 | 2 | Me | CH₂CH₂CH₂ | H | CH₂CF₃ | H | Cl |
| B-0482 | 2 | Me | CH₂CH₂CH₂ | Me | CH₂CF₃ | H | Cl |
| B-0483 | 2 | H | CH₂CH₂CH(Me) | H | CH₂CF₃ | H | Cl |
| B-0484 | 2 | H | CH₂CH₂CH(Me) | Me | CH₂CF₃ | H | Cl |
| B-0485 | 2 | Me | CH₂CH₂CH(Me) | H | CH₂CF₃ | H | Cl |
| B-0486 | 2 | Me | CH₂CH₂CH(Me) | Me | CH₂CF₃ | H | Cl |
| B-0487 | 2 | H | CH₂CH(Me)CH₂ | H | CH₂CF₃ | H | Cl |
| B-0488 | 2 | H | CH₂CH(Me)CH₂ | Me | CH₂CF₃ | H | Cl |
| B-0489 | 2 | Me | CH₂CH(Me)CH₂ | H | CH₂CF₃ | H | Cl |
| B-0490 | 2 | Me | CH₂CH(Me)CH₂ | Me | CH₂CF₃ | H | Cl |
| B-0491 | 2 | H | CH(Me)CH₂CH₂ | H | CH₂CF₃ | H | Cl |
| B-0492 | 2 | H | CH(Me)CH₂CH₂ | Me | CH₂CF₃ | H | Cl |
| B-0493 | 2 | Me | CH(Me)CH₂CH₂ | H | CH₂CF₃ | H | Cl |
| B-0494 | 2 | Me | CH(Me)CH₂CH₂ | Me | CH₂CF₃ | H | Cl |
| B-0495 | 2 | H | single bond | Me | CH₂CF₃ | H | Me |
| B-0496 | 2 | H | single bond | Et | CH₂CF₃ | H | Me |
| B-0497 | 2 | Me | single bond | H | CH₂CF₃ | H | Me |
| B-0498 | 2 | Et | single bond | H | CH₂CF₃ | H | Me |
| B-0499 | 2 | Me | single bond | Me | CH₂CF₃ | H | Me |
| B-0500 | 2 | H | CH₂ | Me | CH₂CF₃ | H | Me |
| B-0501 | 2 | Me | CH₂ | H | CH₂CF₃ | H | Me |
| B-0502 | 2 | Me | CH₂ | Me | CH₂CF₃ | H | Me |
| B-0503 | 2 | H | CH(Me) | H | CH₂CF₃ | H | Me |
| B-0504 | 2 | H | CH(Me) | Me | CH₂CF₃ | H | Me |
| B-0505 | 2 | Me | CH(Me) | H | CH₂CF₃ | H | Me |
| B-0506 | 2 | Me | CH(Me) | Me | CH₂CF₃ | H | Me |
| B-0507 | 2 | H | CH₂CH₂ | Me | CH₂CF₃ | H | Me |
| B-0508 | 2 | Me | CH₂CH₂ | H | CH₂CF₃ | H | Me |
| B-0509 | 2 | Me | CH₂CH₂ | Me | CH₂CF₃ | H | Me |
| B-0510 | 2 | H | CH₂CH(Me) | H | CH₂CF₃ | H | Me |
| B-0511 | 2 | H | CH₂CH(Me) | Me | CH₂CF₃ | H | Me |
| B-0512 | 2 | Me | CH₂CH(Me) | H | CH₂CF₃ | H | Me |
| B-0513 | 2 | Me | CH₂CH(Me) | Me | CH₂CF₃ | H | Me |
| B-0514 | 2 | H | CH(Me)CH₂ | H | CH₂CF₃ | H | Me |
| B-0515 | 2 | H | CH(Me)CH₂ | Me | CH₂CF₃ | H | Me |
| B-0516 | 2 | Me | CH(Me)CH₂ | H | CH₂CF₃ | H | Me |
| B-0517 | 2 | Me | CH(Me)CH₂ | Me | CH₂CF₃ | H | Me |
| B-0518 | 2 | H | CH₂CH₂CH₂ | Me | CH₂CF₃ | H | Me |
| B-0519 | 2 | Me | CH₂CH₂CH₂ | H | CH₂CF₃ | H | Me |
| B-0520 | 2 | Me | CH₂CH₂CH₂ | Me | CH₂CF₃ | H | Me |
| B-0521 | 2 | H | CH₂CH₂CH(Me) | H | CH₂CF₃ | H | Me |
| B-0522 | 2 | H | CH₂CH₂CH(Me) | Me | CH₂CF₃ | H | Me |

**[Table 176]**

| Compound Number | n | Y | W | Z | R¹ | R² | R³ |
|---|---|---|---|---|---|---|---|
| B-0523 | 2 | Me | CH₂CH₂CH(Me) | H | CH₂CF₃ | H | Me |
| B-0524 | 2 | Me | CH₂CH₂CH(Me) | Me | CH₂CF₃ | H | Me |
| B-0525 | 2 | H | CH₂CH(Me)CH₂ | H | CH₂CF₃ | H | Me |
| B-0526 | 2 | H | CH₂CH(Me)CH₂ | Me | CH₂CF₃ | H | Me |
| B-0527 | 2 | Me | CH₂CH(Me)CH₂ | H | CH₂CF₃ | H | Me |
| B-0528 | 2 | Me | CH₂CH(Me)CH₂ | Me | CH₂CF₃ | H | Me |
| B-0529 | 2 | H | CH(Me)CH₂CH₂ | H | CH₂CF₃ | H | Me |
| B-0530 | 2 | H | CH(Me)CH₂CH₂ | Me | CH₂CF₃ | H | Me |
| B-0531 | 2 | Me | CH(Me)CH₂CH₂ | H | CH₂CF₃ | H | Me |
| B-0532 | 2 | Me | CH(Me)CH₂CH₂ | Me | CH₂CF₃ | H | Me |
| B-0533 | 2 | H | single bond | Me | Et | H | Cl |
| B-0534 | 2 | H | single bond | Et | Et | H | Cl |
| B-0535 | 2 | Me | single bond | H | Et | H | Cl |
| B-0536 | 2 | Et | single bond | H | Et | H | Cl |
| B-0537 | 2 | Me | single bond | Me | Et | H | Cl |
| B-0538 | 2 | H | CH₂ | Me | Et | H | Cl |
| B-0539 | 2 | Me | CH₂ | H | Et | H | Cl |
| B-0540 | 2 | Me | CH₂ | Me | Et | H | Cl |
| B-0541 | 2 | H | CH(Me) | H | Et | H | Cl |
| B-0542 | 2 | H | CH(Me) | Me | Et | H | Cl |
| B-0543 | 2 | Me | CH(Me) | H | Et | H | Cl |
| B-0544 | 2 | Me | CH(Me) | Me | Et | H | Cl |
| B-0545 | 2 | H | CH₂CH₂ | Me | Et | H | Cl |
| B-0546 | 2 | Me | CH₂CH₂ | H | Et | H | Cl |
| B-0547 | 2 | Me | CH₂CH₂ | Me | Et | H | Cl |
| B-0548 | 2 | H | CH₂CH(Me) | H | Et | H | Cl |
| B-0549 | 2 | H | CH₂CH(Me) | Me | Et | H | Cl |
| B-0550 | 2 | Me | CH₂CH(Me) | H | Et | H | Cl |
| B-0551 | 2 | Me | CH₂CH(Me) | Me | Et | H | Cl |
| B-0552 | 2 | H | CH(Me)CH₂ | H | Et | H | Cl |
| B-0553 | 2 | H | CH(Me)CH₂ | Me | Et | H | Cl |
| B-0554 | 2 | Me | CH(Me)CH₂ | H | Et | H | Cl |
| B-0555 | 2 | Me | CH(Me)CH₂ | Me | Et | H | Cl |
| B-0556 | 2 | H | CH₂CH₂CH₂ | Me | Et | H | Cl |
| B-0557 | 2 | Me | CH₂CH₂CH₂ | H | Et | H | Cl |
| B-0558 | 2 | Me | CH₂CH₂CH₂ | Me | Et | H | Cl |
| B-0559 | 2 | H | CH₂CH₂CH(Me) | H | Et | H | Cl |
| B-0560 | 2 | H | CH₂CH₂CH(Me) | Me | Et | H | Cl |
| B-0561 | 2 | Me | CH₂CH₂CH(Me) | H | Et | H | Cl |
| B-0562 | 2 | Me | CH₂CH₂CH(Me) | Me | Et | H | Cl |
| B-0563 | 2 | H | CH₂CH(Me)CH₂ | H | Et | H | Cl |
| B-0564 | 2 | H | CH₂CH(Me)CH₂ | Me | Et | H | Cl |
| B-0565 | 2 | Me | CH₂CH(Me)CH₂ | H | Et | H | Cl |
| B-0566 | 2 | Me | CH₂CH(Me)CH₂ | Me | Et | H | Cl |

**[Table 177]**

| Compound Number | n | Y | W | Z | R¹ | R² | R³ |
|---|---|---|---|---|---|---|---|
| B-0567 | 2 | H | CH(Me)CH₂CH₂ | H | Et | H | Cl |
| B-0568 | 2 | H | CH(Me)CH₂CH₂ | Me | Et | H | Cl |
| B-0569 | 2 | Me | CH(Me)CH₂CH₂ | H | Et | H | Cl |
| B-0570 | 2 | Me | CH(Me)CH₂CH₂ | Me | Et | H | Cl |
| B-0571 | 2 | H | single bond | Me | Et | H | Me |
| B-0572 | 2 | H | single bond | Et | Et | H | Me |
| B-0573 | 2 | Me | single bond | H | Et | H | Me |
| B-0574 | 2 | Et | single bond | H | Et | H | Me |
| B-0575 | 2 | Me | single bond | Me | Et | H | Me |
| B-0576 | 2 | H | CH₂ | Me | Et | H | Me |
| B-0577 | 2 | Me | CH₂ | H | Et | H | Me |
| B-0578 | 2 | Me | CH₂ | Me | Et | H | Me |
| B-0579 | 2 | H | CH(Me) | H | Et | H | Me |
| B-0580 | 2 | H | CH(Me) | Me | Et | H | Me |
| B-0581 | 2 | Me | CH(Me) | H | Et | H | Me |
| B-0582 | 2 | Me | CH(Me) | Me | Et | H | Me |
| B-0583 | 2 | H | CH₂CH₂ | Me | Et | H | Me |
| B-0584 | 2 | Me | CH₂CH₂ | H | Et | H | Me |
| B-0585 | 2 | Me | CH₂CH₂ | Me | Et | H | Me |
| B-0586 | 2 | H | CH₂CH(Me) | H | Et | H | Me |
| B-0587 | 2 | H | CH₂CH(Me) | Me | Et | H | Me |
| B-0588 | 2 | Me | CH₂CH(Me) | H | Et | H | Me |
| B-0589 | 2 | Me | CH₂CH(Me) | Me | Et | H | Me |
| B-0590 | 2 | H | CH(Me)CH₂ | H | Et | H | Me |
| B-0591 | 2 | H | CH(Me)CH₂ | Me | Et | H | Me |
| B-0592 | 2 | Me | CH(Me)CH₂ | H | Et | H | Me |
| B-0593 | 2 | Me | CH(Me)CH₂ | Me | Et | H | Me |
| B-0594 | 2 | H | CH₂CH₂CH₂ | Me | Et | H | Me |
| B-0595 | 2 | Me | CH₂CH₂CH₂ | H | Et | H | Me |
| B-0596 | 2 | Me | CH₂CH₂CH₂ | Me | Et | H | Me |
| B-0597 | 2 | H | CH₂CH₂CH(Me) | H | Et | H | Me |
| B-0598 | 2 | H | CH₂CH₂CH(Me) | Me | Et | H | Me |
| B-0599 | 2 | Me | CH₂CH₂CH(Me) | H | Et | H | Me |
| B-0600 | 2 | Me | CH₂CH₂CH(Me) | Me | Et | H | Me |
| B-0601 | 2 | H | CH₂CH(Me)CH₂ | H | Et | H | Me |
| B-0602 | 2 | H | CH₂CH(Me)CH₂ | Me | Et | H | Me |
| B-0603 | 2 | Me | CH₂CH(Me)CH₂ | H | Et | H | Me |
| B-0604 | 2 | Me | CH₂CH(Me)CH₂ | Me | Et | H | Me |
| B-0605 | 2 | H | CH(Me)CH₂CH₂ | H | Et | H | Me |
| B-0606 | 2 | H | CH(Me)CH₂CH₂ | Me | Et | H | Me |
| B-0607 | 2 | Me | CH(Me)CH₂CH₂ | H | Et | H | Me |
| B-0608 | 2 | Me | CH(Me)CH₂CH₂ | Me | Et | H | Me |
| B-0609 | 2 | H | single bond | Me | *c*-Pr | H | Cl |
| B-0610 | 2 | H | single bond | Et | *c*-Pr | H | Cl |

**[Table 178]**

| Compound Number | n | Y | W | Z | R¹ | R² | R³ |
|---|---|---|---|---|---|---|---|
| B-0611 | 2 | Me | single bond | H | *c*-Pr | H | Cl |
| B-0612 | 2 | Et | single bond | H | *c*-Pr | H | Cl |
| B-0613 | 2 | Me | single bond | Me | *c*-Pr | H | Cl |
| B-0614 | 2 | H | CH₂ | Me | *c*-Pr | H | Cl |
| B-0615 | 2 | Me | CH₂ | H | *c*-Pr | H | Cl |
| B-0616 | 2 | Me | CH₂ | Me | *c*-Pr | H | Cl |
| B-0617 | 2 | H | CH(Me) | H | *c*-Pr | H | Cl |
| B-0618 | 2 | H | CH(Me) | Me | *c*-Pr | H | Cl |
| B-0619 | 2 | Me | CH(Me) | H | *c*-Pr | H | Cl |
| B-0620 | 2 | Me | CH(Me) | Me | *c*-Pr | H | Cl |
| B-0621 | 2 | H | CH₂CH₂ | Me | *c*-Pr | H | Cl |
| B-0622 | 2 | Me | CH₂CH₂ | H | *c*-Pr | H | Cl |
| B-0623 | 2 | Me | CH₂CH₂ | Me | *c*-Pr | H | Cl |
| B-0624 | 2 | H | CH₂CH(Me) | H | *c*-Pr | H | Cl |
| B-0625 | 2 | H | CH₂CH(Me) | Me | *c*-Pr | H | Cl |
| B-0626 | 2 | Me | CH₂CH(Me) | H | *c*-Pr | H | Cl |
| B-0627 | 2 | Me | CH₂CH(Me) | Me | *c*-Pr | H | Cl |
| B-0628 | 2 | H | CH(Me)CH₂ | H | *c*-Pr | H | Cl |
| B-0629 | 2 | H | CH(Me)CH₂ | Me | *c*-Pr | H | Cl |
| B-0630 | 2 | Me | CH(Me)CH₂ | H | *c*-Pr | H | Cl |
| B-0631 | 2 | Me | CH(Me)CH₂ | Me | *c*-Pr | H | Cl |
| B-0632 | 2 | H | CH₂CH₂CH₂ | Me | *c*-Pr | H | Cl |
| B-0633 | 2 | Me | CH₂CH₂CH₂ | H | *c*-Pr | H | Cl |
| B-0634 | 2 | Me | CH₂CH₂CH₂ | Me | *c*-Pr | H | Cl |
| B-0635 | 2 | H | CH₂CH₂CH(Me) | H | *c*-Pr | H | Cl |
| B-0636 | 2 | H | CH₂CH₂CH(Me) | Me | *c*-Pr | H | Cl |
| B-0637 | 2 | Me | CH₂CH₂CH(Me) | H | *c*-Pr | H | Cl |
| B-0638 | 2 | Me | CH₂CH₂CH(Me) | Me | *c*-Pr | H | Cl |
| B-0639 | 2 | H | CH₂CH(Me)CH₂ | H | *c*-Pr | H | Cl |
| B-0640 | 2 | H | CH₂CH(Me)CH₂ | Me | *c*-Pr | H | Cl |
| B-0641 | 2 | Me | CH₂CH(Me)CH₂ | H | *c*-Pr | H | Cl |
| B-0642 | 2 | Me | CH₂CH(Me)CH₂ | Me | *c*-Pr | H | Cl |
| B-0643 | 2 | H | CH(Me)CH₂CH₂ | H | *c*-Pr | H | Cl |
| B-0644 | 2 | H | CH(Me)CH₂CH₂ | Me | *c*-Pr | H | Cl |
| B-0645 | 2 | Me | CH(Me)CH₂CH₂ | H | *c*-Pr | H | Cl |
| B-0646 | 2 | Me | CH(Me)CH₂CH₂ | Me | *c*-Pr | H | Cl |
| B-0647 | 2 | H | single bond | Me | *c*-Pr | H | Me |
| B-0648 | 2 | H | single bond | Et | *c*-Pr | H | Me |
| B-0649 | 2 | Me | single bond | H | *c*-Pr | H | Me |
| B-0650 | 2 | Et | single bond | H | *c*-Pr | H | Me |
| B-0651 | 2 | Me | single bond | Me | *c*-Pr | H | Me |
| B-0652 | 2 | H | CH₂ | Me | *c*-Pr | H | Me |
| B-0653 | 2 | Me | CH₂ | H | *c*-Pr | H | Me |
| B-0654 | 2 | Me | CH₂ | Me | *c*-Pr | H | Me |

**[Table 179]**

| Compound Number | n | Y | W | Z | R¹ | R² | R³ |
|---|---|---|---|---|---|---|---|
| B-0655 | 2 | H | CH(Me) | H | *c*-Pr | H | Me |
| B-0656 | 2 | H | CH(Me) | Me | *c*-Pr | H | Me |
| B-0657 | 2 | Me | CH(Me) | H | *c*-Pr | H | Me |
| B-0658 | 2 | Me | CH(Me) | Me | *c*-Pr | H | Me |
| B-0659 | 2 | H | CH₂CH₂ | Me | *c*-Pr | H | Me |
| B-0660 | 2 | Me | CH₂CH₂ | H | *c*-Pr | H | Me |
| B-0661 | 2 | Me | CH₂CH₂ | Me | *c*-Pr | H | Me |
| B-0662 | 2 | H | CH₂CH(Me) | H | *c*-Pr | H | Me |
| B-0663 | 2 | H | CH₂CH(Me) | Me | *c*-Pr | H | Me |
| B-0664 | 2 | Me | CH₂CH(Me) | H | *c*-Pr | H | Me |
| B-0665 | 2 | Me | CH₂CH(Me) | Me | *c*-Pr | H | Me |
| B-0666 | 2 | H | CH(Me)CH₂ | H | *c*-Pr | H | Me |
| B-0667 | 2 | H | CH(Me)CH₂ | Me | *c*-Pr | H | Me |
| B-0668 | 2 | Me | CH(Me)CH₂ | H | *c*-Pr | H | Me |
| B-0669 | 2 | Me | CH(Me)CH₂ | Me | *c*-Pr | H | Me |
| B-0670 | 2 | H | CH₂CH₂CH₂ | Me | *c*-Pr | H | Me |
| B-0671 | 2 | Me | CH₂CH₂CH₂ | H | *c*-Pr | H | Me |
| B-0672 | 2 | Me | CH₂CH₂CH₂ | Me | *c*-Pr | H | Me |
| B-0673 | 2 | H | CH₂CH₂CH(Me) | H | *c*-Pr | H | Me |
| B-0674 | 2 | H | CH₂CH₂CH(Me) | Me | *c*-Pr | H | Me |
| B-0675 | 2 | Me | CH₂CH₂CH(Me) | H | *c*-Pr | H | Me |
| B-0676 | 2 | Me | CH₂CH₂CH(Me) | Me | *c*-Pr | H | Me |
| B-0677 | 2 | H | CH₂CH(Me)CH₂ | H | *c*-Pr | H | Me |
| B-0678 | 2 | H | CH₂CH(Me)CH₂ | Me | *c*-Pr | H | Me |
| B-0679 | 2 | Me | CH₂CH(Me)CH₂ | H | *c*-Pr | H | Me |
| B-0680 | 2 | Me | CH₂CH(Me)CH₂ | Me | *c*-Pr | H | Me |
| B-0681 | 2 | H | CH(Me)CH₂CH₂ | H | *c*-Pr | H | Me |
| B-0682 | 2 | H | CH(Me)CH₂CH₂ | Me | *c*-Pr | H | Me |
| B-0683 | 2 | Me | CH(Me)CH₂CH₂ | H | *c*-Pr | H | Me |
| B-0684 | 2 | Me | CH(Me)CH₂CH₂ | Me | *c*-Pr | H | Me |
| B-0685 | 0 | H | single bond | Me | CH₂CF₃ | Me | Cl |
| B-0686 | 0 | H | single bond | Et | CH₂CF₃ | Me | Cl |
| B-0687 | 0 | Me | single bond | H | CH₂CF₃ | Me | Cl |
| B-0688 | 0 | Et | single bond | H | CH₂CF₃ | Me | Cl |
| B-0689 | 0 | Me | single bond | Me | CH₂CF₃ | Me | Cl |
| B-0690 | 0 | H | CH₂ | Me | CH₂CF₃ | Me | Cl |
| B-0691 | 0 | Me | CH₂ | H | CH₂CF₃ | Me | Cl |
| B-0692 | 0 | Me | CH₂ | Me | CH₂CF₃ | Me | Cl |
| B-0693 | 0 | H | CH(Me) | H | CH₂CF₃ | Me | Cl |
| B-0694 | 0 | H | CH(Me) | Me | CH₂CF₃ | Me | Cl |
| B-0695 | 0 | Me | CH(Me) | H | CH₂CF₃ | Me | Cl |
| B-0696 | 0 | Me | CH(Me) | Me | CH₂CF₃ | Me | Cl |
| B-0697 | 0 | H | CH₂CH₂ | Me | CH₂CF₃ | Me | Cl |
| B-0698 | 0 | Me | CH₂CH₂ | H | CH₂CF₃ | Me | Cl |

**[Table 180]**

| Compound Number | n | Y | W | Z | R¹ | R² | R³ |
|---|---|---|---|---|---|---|---|
| B-0699 | 0 | Me | CH₂CH₂ | Me | CH₂CF₃ | Me | Cl |
| B-0700 | 0 | H | CH₂CH(Me) | H | CH₂CF₃ | Me | Cl |
| B-0701 | 0 | H | CH₂CH(Me) | Me | CH₂CF₃ | Me | Cl |
| B-0702 | 0 | Me | CH₂CH(Me) | H | CH₂CF₃ | Me | Cl |
| B-0703 | 0 | Me | CH₂CH(Me) | Me | CH₂CF₃ | Me | Cl |
| B-0704 | 0 | H | CH(Me)CH₂ | H | CH₂CF₃ | Me | Cl |
| B-0705 | 0 | H | CH(Me)CH₂ | Me | CH₂CF₃ | Me | Cl |
| B-0706 | 0 | Me | CH(Me)CH₂ | H | CH₂CF₃ | Me | Cl |
| B-0707 | 0 | Me | CH(Me)CH₂ | Me | CH₂CF₃ | Me | Cl |
| B-0708 | 0 | H | CH₂CH₂CH₂ | Me | CH₂CF₃ | Me | Cl |
| B-0709 | 0 | Me | CH₂CH₂CH₂ | H | CH₂CF₃ | Me | Cl |
| B-0710 | 0 | Me | CH₂CH₂CH₂ | Me | CH₂CF₃ | Me | Cl |
| B-0711 | 0 | H | CH₂CH₂CH(Me) | H | CH₂CF₃ | Me | Cl |
| B-0712 | 0 | H | CH₂CH₂CH(Me) | Me | CH₂CF₃ | Me | Cl |
| B-0713 | 0 | Me | CH₂CH₂CH(Me) | H | CH₂CF₃ | Me | Cl |
| B-0714 | 0 | Me | CH₂CH₂CH(Me) | Me | CH₂CF₃ | Me | Cl |
| B-0715 | 0 | H | CH₂CH(Me)CH₂ | H | CH₂CF₃ | Me | Cl |
| B-0716 | 0 | H | CH₂CH(Me)CH₂ | Me | CH₂CF₃ | Me | Cl |
| B-0717 | 0 | Me | CH₂CH(Me)CH₂ | H | CH₂CF₃ | Me | Cl |
| B-0718 | 0 | Me | CH₂CH(Me)CH₂ | Me | CH₂CF₃ | Me | Cl |
| B-0719 | 0 | H | CH(Me)CH₂CH₂ | H | CH₂CF₃ | Me | Cl |
| B-0720 | 0 | H | CH(Me)CH₂CH₂ | Me | CH₂CF₃ | Me | Cl |
| B-0721 | 0 | Me | CH(Me)CH₂CH₂ | H | CH₂CF₃ | Me | Cl |
| B-0722 | 0 | Me | CH(Me)CH₂CH₂ | Me | CH₂CF₃ | Me | Cl |
| B-0723 | 0 | H | single bond | Me | CH₂CF₃ | Me | Me |
| B-0724 | 0 | H | single bond | Et | CH₂CF₃ | Me | Me |
| B-0725 | 0 | Me | single bond | H | CH₂CF₃ | Me | Me |
| B-0726 | 0 | Et | single bond | H | CH₂CF₃ | Me | Me |
| B-0727 | 0 | Me | single bond | Me | CH₂CF₃ | Me | Me |
| B-0728 | 0 | H | CH₂ | Me | CH₂CF₃ | Me | Me |
| B-0729 | 0 | Me | CH₂ | H | CH₂CF₃ | Me | Me |
| B-0730 | 0 | Me | CH₂ | Me | CH₂CF₃ | Me | Me |
| B-0731 | 0 | H | CH(Me) | H | CH₂CF₃ | Me | Me |
| B-0732 | 0 | H | CH(Me) | Me | CH₂CF₃ | Me | Me |
| B-0733 | 0 | Me | CH(Me) | H | CH₂CF₃ | Me | Me |
| B-0734 | 0 | Me | CH(Me) | Me | CH₂CF₃ | Me | Me |
| B-0735 | 0 | H | CH₂CH₂ | Me | CH₂CF₃ | Me | Me |
| B-0736 | 0 | Me | CH₂CH₂ | H | CH₂CF₃ | Me | Me |
| B-0737 | 0 | Me | CH₂CH₂ | Me | CH₂CF₃ | Me | Me |
| B-0738 | 0 | H | CH₂CH(Me) | H | CH₂CF₃ | Me | Me |
| B-0739 | 0 | H | CH₂CH(Me) | Me | CH₂CF₃ | Me | Me |
| B-0740 | 0 | Me | CH₂CH(Me) | H | CH₂CF₃ | Me | Me |
| B-0741 | 0 | Me | CH₂CH(Me) | Me | CH₂CF₃ | Me | Me |
| B-0742 | 0 | H | CH(Me)CH₂ | H | CH₂CF₃ | Me | Me |

**[Table 181]**

| Compound Number | n | Y | W | Z | R¹ | R² | R³ |
|---|---|---|---|---|---|---|---|
| B-0743 | 0 | H | CH(Me)CH₂ | Me | CH₂CF₃ | Me | Me |
| B-0744 | 0 | Me | CH(Me)CH₂ | H | CH₂CF₃ | Me | Me |
| B-0745 | 0 | Me | CH(Me)CH₂ | Me | CH₂CF₃ | Me | Me |
| B-0746 | 0 | H | CH₂CH₂CH₂ | Me | CH₂CF₃ | Me | Me |
| B-0747 | 0 | Me | CH₂CH₂CH₂ | H | CH₂CF₃ | Me | Me |
| B-0748 | 0 | Me | CH₂CH₂CH₂ | Me | CH₂CF₃ | Me | Me |
| B-0749 | 0 | H | CH₂CH₂CH(Me) | H | CH₂CF₃ | Me | Me |
| B-0750 | 0 | H | CH₂CH₂CH(Me) | Me | CH₂CF₃ | Me | Me |
| B-0751 | 0 | Me | CH₂CH₂CH(Me) | H | CH₂CF₃ | Me | Me |
| B-0752 | 0 | Me | CH₂CH₂CH(Me) | Me | CH₂CF₃ | Me | Me |
| B-0753 | 0 | H | CH₂CH(Me)CH₂ | H | CH₂CF₃ | Me | Me |
| B-0754 | 0 | H | CH₂CH(Me)CH₂ | Me | CH₂CF₃ | Me | Me |
| B-0755 | 0 | Me | CH₂CH(Me)CH₂ | H | CH₂CF₃ | Me | Me |
| B-0756 | 0 | Me | CH₂CH(Me)CH₂ | Me | CH₂CF₃ | Me | Me |
| B-0757 | 0 | H | CH(Me)CH₂CH₂ | H | CH₂CF₃ | Me | Me |
| B-0758 | 0 | H | CH(Me)CH₂CH₂ | Me | CH₂CF₃ | Me | Me |
| B-0759 | 0 | Me | CH(Me)CH₂CH₂ | H | CH₂CF₃ | Me | Me |
| B-0760 | 0 | Me | CH(Me)CH₂CH₂ | Me | CH₂CF₃ | Me | Me |
| B-0761 | 0 | H | single bond | Me | CH₂CF₃ | Et | Cl |
| B-0762 | 0 | H | single bond | Et | CH₂CF₃ | Et | Cl |
| B-0763 | 0 | Me | single bond | H | CH₂CF₃ | Et | Cl |
| B-0764 | 0 | Et | single bond | H | CH₂CF₃ | Et | Cl |
| B-0765 | 0 | Me | single bond | Me | CH₂CF₃ | Et | Cl |
| B-0766 | 0 | H | CH₂ | Me | CH₂CF₃ | Et | Cl |
| B-0767 | 0 | Me | CH₂ | H | CH₂CF₃ | Et | Cl |
| B-0768 | 0 | Me | CH₂ | Me | CH₂CF₃ | Et | Cl |
| B-0769 | 0 | H | CH(Me) | H | CH₂CF₃ | Et | Cl |
| B-0770 | 0 | H | CH(Me) | Me | CH₂CF₃ | Et | Cl |
| B-0771 | 0 | Me | CH(Me) | H | CH₂CF₃ | Et | Cl |
| B-0772 | 0 | Me | CH(Me) | Me | CH₂CF₃ | Et | Cl |
| B-0773 | 0 | H | CH₂CH₂ | Me | CH₂CF₃ | Et | Cl |
| B-0774 | 0 | Me | CH₂CH₂ | H | CH₂CF₃ | Et | Cl |
| B-0775 | 0 | Me | CH₂CH₂ | Me | CH₂CF₃ | Et | Cl |
| B-0776 | 0 | H | CH₂CH(Me) | H | CH₂CF₃ | Et | Cl |
| B-0777 | 0 | H | CH₂CH(Me) | Me | CH₂CF₃ | Et | Cl |
| B-0778 | 0 | Me | CH₂CH(Me) | H | CH₂CF₃ | Et | Cl |
| B-0779 | 0 | Me | CH₂CH(Me) | Me | CH₂CF₃ | Et | Cl |
| B-0780 | 0 | H | CH(Me)CH₂ | H | CH₂CF₃ | Et | Cl |
| B-0781 | 0 | H | CH(Me)CH₂ | Me | CH₂CF₃ | Et | Cl |
| B-0782 | 0 | Me | CH(Me)CH₂ | H | CH₂CF₃ | Et | Cl |
| B-0783 | 0 | Me | CH(Me)CH₂ | Me | CH₂CF₃ | Et | Cl |
| B-0784 | 0 | H | CH₂CH₂CH₂ | Me | CH₂CF₃ | Et | Cl |
| B-0785 | 0 | Me | CH₂CH₂CH₂ | H | CH₂CF₃ | Et | Cl |
| B-0786 | 0 | Me | CH₂CH₂CH₂ | Me | CH₂CF₃ | Et | Cl |

**[Table 182]**

| Compound Number | n | Y | W | Z | R¹ | R² | R³ |
|---|---|---|---|---|---|---|---|
| B-0787 | 0 | H | CH₂CH₂CH(Me) | H | CH₂CF₃ | Et | Cl |
| B-0788 | 0 | H | CH₂CH₂CH(Me) | Me | CH₂CF₃ | Et | Cl |
| B-0789 | 0 | Me | CH₂CH₂CH(Me) | H | CH₂CF₃ | Et | Cl |
| B-0790 | 0 | Me | CH₂CH₂CH(Me) | Me | CH₂CF₃ | Et | Cl |
| B-0791 | 0 | H | CH₂CH(Me)CH₂ | H | CH₂CF₃ | Et | Cl |
| B-0792 | 0 | H | CH₂CH(Me)CH₂ | Me | CH₂CF₃ | Et | Cl |
| B-0793 | 0 | Me | CH₂CH(Me)CH₂ | H | CH₂CF₃ | Et | Cl |
| B-0794 | 0 | Me | CH₂CH(Me)CH₂ | Me | CH₂CF₃ | Et | Cl |
| B-0795 | 0 | H | CH(Me)CH₂CH₂ | H | CH₂CF₃ | Et | Cl |
| B-0796 | 0 | H | CH(Me)CH₂CH₂ | Me | CH₂CF₃ | Et | Cl |
| B-0797 | 0 | Me | CH(Me)CH₂CH₂ | H | CH₂CF₃ | Et | Cl |
| B-0798 | 0 | Me | CH(Me)CH₂CH₂ | Me | CH₂CF₃ | Et | Cl |
| B-0799 | 0 | H | single bond | Me | CH₂CF₃ | Et | Me |
| B-0800 | 0 | H | single bond | Et | CH₂CF₃ | Et | Me |
| B-0801 | 0 | Me | single bond | H | CH₂CF₃ | Et | Me |
| B-0802 | 0 | Et | single bond | H | CH₂CF₃ | Et | Me |
| B-0803 | 0 | Me | single bond | Me | CH₂CF₃ | Et | Me |
| B-0804 | 0 | H | CH₂ | Me | CH₂CF₃ | Et | Me |
| B-0805 | 0 | Me | CH₂ | H | CH₂CF₃ | Et | Me |
| B-0806 | 0 | Me | CH₂ | Me | CH₂CF₃ | Et | Me |
| B-0807 | 0 | H | CH(Me) | H | CH₂CF₃ | Et | Me |
| B-0808 | 0 | H | CH(Me) | Me | CH₂CF₃ | Et | Me |
| B-0809 | 0 | Me | CH(Me) | H | CH₂CF₃ | Et | Me |
| B-0810 | 0 | Me | CH(Me) | Me | CH₂CF₃ | Et | Me |
| B-0811 | 0 | H | CH₂CH₂ | Me | CH₂CF₃ | Et | Me |
| B-0812 | 0 | Me | CH₂CH₂ | H | CH₂CF₃ | Et | Me |
| B-0813 | 0 | Me | CH₂CH₂ | Me | CH₂CF₃ | Et | Me |
| B-0814 | 0 | H | CH₂CH(Me) | H | CH₂CF₃ | Et | Me |
| B-0815 | 0 | H | CH₂CH(Me) | Me | CH₂CF₃ | Et | Me |
| B-0816 | 0 | Me | CH₂CH(Me) | H | CH₂CF₃ | Et | Me |
| B-0817 | 0 | Me | CH₂CH(Me) | Me | CH₂CF₃ | Et | Me |
| B-0818 | 0 | H | CH(Me)CH₂ | H | CH₂CF₃ | Et | Me |
| B-0819 | 0 | H | CH(Me)CH₂ | Me | CH₂CF₃ | Et | Me |
| B-0820 | 0 | Me | CH(Me)CH₂ | H | CH₂CF₃ | Et | Me |
| B-0821 | 0 | Me | CH(Me)CH₂ | Me | CH₂CF₃ | Et | Me |
| B-0822 | 0 | H | CH₂CH₂CH₂ | Me | CH₂CF₃ | Et | Me |
| B-0823 | 0 | Me | CH₂CH₂CH₂ | H | CH₂CF₃ | Et | Me |
| B-0824 | 0 | Me | CH₂CH₂CH₂ | Me | CH₂CF₃ | Et | Me |
| B-0825 | 0 | H | CH₂CH₂CH(Me) | H | CH₂CF₃ | Et | Me |
| B-0826 | 0 | H | CH₂CH₂CH(Me) | Me | CH₂CF₃ | Et | Me |
| B-0827 | 0 | Me | CH₂CH₂CH(Me) | H | CH₂CF₃ | Et | Me |
| B-0828 | 0 | Me | CH₂CH₂CH(Me) | Me | CH₂CF₃ | Et | Me |
| B-0829 | 0 | H | CH₂CH(Me)CH₂ | H | CH₂CF₃ | Et | Me |
| B-0830 | 0 | H | CH₂CH(Me)CH₂ | Me | CH₂CF₃ | Et | Me |

**[Table 183]**

| Compound Number | n | Y | W | Z | R¹ | R² | R³ |
|---|---|---|---|---|---|---|---|
| B-0831 | 0 | Me | CH₂CH(Me)CH₂ | H | CH₂CF₃ | Et | Me |
| B-0832 | 0 | Me | CH₂CH(Me)CH₂ | Me | CH₂CF₃ | Et | Me |
| B-0833 | 0 | H | CH(Me)CH₂CH₂ | H | CH₂CF₃ | Et | Me |
| B-0834 | 0 | H | CH(Me)CH₂CH₂ | Me | CH₂CF₃ | Et | Me |
| B-0835 | 0 | Me | CH(Me)CH₂CH₂ | H | CH₂CF₃ | Et | Me |
| B-0836 | 0 | Me | CH(Me)CH₂CH₂ | Me | CH₂CF₃ | Et | Me |
| B-0837 | 0 | H | single bond | Me | CH₂CF₃ | CH₂OMe | Cl |
| B-0838 | 0 | H | single bond | Et | CH₂CF₃ | CH₂OMe | Cl |
| B-0839 | 0 | Me | single bond | H | CH₂CF₃ | CH₂OMe | Cl |
| B-0840 | 0 | Et | single bond | H | CH₂CF₃ | CH₂OMe | Cl |
| B-0841 | 0 | Me | single bond | Me | CH₂CF₃ | CH₂OMe | Cl |
| B-0842 | 0 | H | CH₂ | Me | CH₂CF₃ | CH₂OMe | Cl |
| B-0843 | 0 | Me | CH₂ | H | CH₂CF₃ | CH₂OMe | Cl |
| B-0844 | 0 | Me | CH₂ | Me | CH₂CF₃ | CH₂OMe | Cl |
| B-0845 | 0 | H | CH(Me) | H | CH₂CF₃ | CH₂OMe | Cl |
| B-0846 | 0 | H | CH(Me) | Me | CH₂CF₃ | CH₂OMe | Cl |
| B-0847 | 0 | Me | CH(Me) | H | CH₂CF₃ | CH₂OMe | Cl |
| B-0848 | 0 | Me | CH(Me) | Me | CH₂CF₃ | CH₂OMe | Cl |
| B-0849 | 0 | H | CH₂CH₂ | Me | CH₂CF₃ | CH₂OMe | Cl |
| B-0850 | 0 | Me | CH₂CH₂ | H | CH₂CF₃ | CH₂OMe | Cl |
| B-0851 | 0 | Me | CH₂CH₂ | Me | CH₂CF₃ | CH₂OMe | Cl |
| B-0852 | 0 | H | CH₂CH(Me) | H | CH₂CF₃ | CH₂OMe | Cl |
| B-0853 | 0 | H | CH₂CH(Me) | Me | CH₂CF₃ | CH₂OMe | Cl |
| B-0854 | 0 | Me | CH₂CH(Me) | H | CH₂CF₃ | CH₂OMe | Cl |
| B-0855 | 0 | Me | CH₂CH(Me) | Me | CH₂CF₃ | CH₂OMe | Cl |
| B-0856 | 0 | H | CH(Me)CH₂ | H | CH₂CF₃ | CH₂OMe | Cl |
| B-0857 | 0 | H | CH(Me)CH₂ | Me | CH₂CF₃ | CH₂OMe | Cl |
| B-0858 | 0 | Me | CH(Me)CH₂ | H | CH₂CF₃ | CH₂OMe | Cl |
| B-0859 | 0 | Me | CH(Me)CH₂ | Me | CH₂CF₃ | CH₂OMe | Cl |
| B-0860 | 0 | H | CH₂CH₂CH₂ | Me | CH₂CF₃ | CH₂OMe | Cl |
| B-0861 | 0 | Me | CH₂CH₂CH₂ | H | CH₂CF₃ | CH₂OMe | Cl |
| B-0862 | 0 | Me | CH₂CH₂CH₂ | Me | CH₂CF₃ | CH₂OMe | Cl |
| B-0863 | 0 | H | CH₂CH₂CH(Me) | H | CH₂CF₃ | CH₂OMe | Cl |
| B-0864 | 0 | H | CH₂CH₂CH(Me) | Me | CH₂CF₃ | CH₂OMe | Cl |
| B-0865 | 0 | Me | CH₂CH₂CH(Me) | H | CH₂CF₃ | CH₂OMe | Cl |
| B-0866 | 0 | Me | CH₂CH₂CH(Me) | Me | CH₂CF₃ | CH₂OMe | Cl |
| B-0867 | 0 | H | CH₂CH(Me)CH₂ | H | CH₂CF₃ | CH₂OMe | Cl |
| B-0868 | 0 | H | CH₂CH(Me)CH₂ | Me | CH₂CF₃ | CH₂OMe | Cl |
| B-0869 | 0 | Me | CH₂CH(Me)CH₂ | H | CH₂CF₃ | CH₂OMe | Cl |
| B-0870 | 0 | Me | CH₂CH(Me)CH₂ | Me | CH₂CF₃ | CH₂OMe | Cl |
| B-0871 | 0 | H | CH(Me)CH₂CH₂ | H | CH₂CF₃ | CH₂OMe | Cl |
| B-0872 | 0 | H | CH(Me)CH₂CH₂ | Me | CH₂CF₃ | CH₂OMe | Cl |
| B-0873 | 0 | Me | CH(Me)CH₂CH₂ | H | CH₂CF₃ | CH₂OMe | Cl |
| B-0874 | 0 | Me | CH(Me)CH₂CH₂ | Me | CH₂CF₃ | CH₂OMe | Cl |

**[Table 184]**

| Compound Number | n | Y | W | z | R¹ | R² | R³ |
|---|---|---|---|---|---|---|---|
| B-0875 | 0 | H | single bond | Me | CH₂CF₃ | CH₂OMe | Me |
| B-0876 | 0 | H | single bond | Et | CH₂CF₃ | CH₂OMe | Me |
| B-0877 | 0 | Me | single bond | H | CH₂CF₃ | CH₂OMe | Me |
| B-0878 | 0 | Et | single bond | H | CH₂CF₃ | CH₂OMe | Me |
| B-0879 | 0 | Me | single bond | Me | CH₂CF₃ | CH₂OMe | Me |
| B-0880 | 0 | H | CH₂ | Me | CH₂CF₃ | CH₂OMe | Me |
| B-0881 | 0 | Me | CH₂ | H | CH₂CF₃ | CH₂OMe | Me |
| B-0882 | 0 | Me | CH₂ | Me | CH₂CF₃ | CH₂OMe | Me |
| B-0883 | 0 | H | CH(Me) | H | CH₂CF₃ | CH₂OMe | Me |
| B-0884 | 0 | H | CH(Me) | Me | CH₂CF₃ | CH₂OMe | Me |
| B-0885 | 0 | Me | CH(Me) | H | CH₂CF₃ | CH₂OMe | Me |
| B-0886 | 0 | Me | CH(Me) | Me | CH₂CF₃ | CH₂OMe | Me |
| B-0887 | 0 | H | CH₂CH₂ | Me | CH₂CF₃ | CH₂OMe | Me |
| B-0888 | 0 | Me | CH₂CH₂ | H | CH₂CF₃ | CH₂OMe | Me |
| B-0889 | 0 | Me | CH₂CH₂ | Me | CH₂CF₃ | CH₂OMe | Me |
| B-0890 | 0 | H | CH₂CH(Me) | H | CH₂CF₃ | CH₂OMe | Me |
| B-0891 | 0 | H | CH₂CH(Me) | Me | CH₂CF₃ | CH₂OMe | Me |
| B-0892 | 0 | Me | CH₂CH(Me) | H | CH₂CF₃ | CH₂OMe | Me |
| B-0893 | 0 | Me | CH₂CH(Me) | Me | CH₂CF₃ | CH₂OMe | Me |
| B-0894 | 0 | H | CH(Me)CH₂ | H | CH₂CF₃ | CH₂OMe | Me |
| B-0895 | 0 | H | CH(Me)CH₂ | Me | CH₂CF₃ | CH₂OMe | Me |
| B-0896 | 0 | Me | CH(Me)CH₂ | H | CH₂CF₃ | CH₂OMe | Me |
| B-0897 | 0 | Me | CH(Me)CH₂ | Me | CH₂CF₃ | CH₂OMe | Me |
| B-0898 | 0 | H | CH₂CH₂CH₂ | Me | CH₂CF₃ | CH₂OMe | Me |
| B-0899 | 0 | Me | CH₂CH₂CH₂ | H | CH₂CF₃ | CH₂OMe | Me |
| B-0900 | 0 | Me | CH₂CH₂CH₂ | Me | CH₂CF₃ | CH₂OMe | Me |
| B-0901 | 0 | H | CH₂CH₂CH(Me) | H | CH₂CF₃ | CH₂OMe | Me |
| B-0902 | 0 | H | CH₂CH₂CH(Me) | Me | CH₂CF₃ | CH₂OMe | Me |
| B-0903 | 0 | Me | CH₂CH₂CH(Me) | H | CH₂CF₃ | CH₂OMe | Me |
| B-0904 | 0 | Me | CH₂CH₂CH(Me) | Me | CH₂CF₃ | CH₂OMe | Me |
| B-0905 | 0 | H | CH₂CH(Me)CH₂ | H | CH₂CF₃ | CH₂OMe | Me |
| B-0906 | 0 | H | CH₂CH(Me)CH₂ | Me | CH₂CF₃ | CH₂OMe | Me |
| B-0907 | 0 | Me | CH₂CH(Me)CH₂ | H | CH₂CF₃ | CH₂OMe | Me |
| B-0908 | 0 | Me | CH₂CH(Me)CH₂ | Me | CH₂CF₃ | CH₂OMe | Me |
| B-0909 | 0 | H | CH(Me)CH₂CH₂ | H | CH₂CF₃ | CH₂OMe | Me |
| B-0910 | 0 | H | CH(Me)CH₂CH₂ | Me | CH₂CF₃ | CH₂OMe | Me |
| B-0911 | 0 | Me | CH(Me)CH₂CH₂ | H | CH₂CF₃ | CH₂OMe | Me |
| B-0912 | 0 | Me | CH(Me)CH₂CH₂ | Me | CH₂CF₃ | CH₂OMe | Me |
| B-0913 | 0 | H | single bond | Me | CH₂CF₃ | CH₂CN | Cl |
| B-0914 | 0 | H | single bond | Et | CH₂CF₃ | CH₂CN | Cl |
| B-0915 | 0 | Me | single bond | H | CH₂CF₃ | CH₂CN | Cl |
| B-0916 | 0 | Et | single bond | H | CH₂CF₃ | CH₂CN | Cl |
| B-0917 | 0 | Me | single bond | Me | CH₂CF₃ | CH₂CN | Cl |
| B-0918 | 0 | H | CH₂ | Me | CH₂CF₃ | CH₂CN | Cl |

**[Table 185]**

| Compound Number | n | Y | W | z | R¹ | R² | R³ |
|---|---|---|---|---|---|---|---|
| B-0919 | 0 | Me | CH₂ | H | CH₂CF₃ | CH₂CN | Cl |
| B-0920 | 0 | Me | CH₂ | Me | CH₂CF₃ | CH₂CN | Cl |
| B-0921 | 0 | H | CH(Me) | H | CH₂CF₃ | CH₂CN | Cl |
| B-0922 | 0 | H | CH(Me) | Me | CH₂CF₃ | CH₂CN | Cl |
| B-0923 | 0 | Me | CH(Me) | H | CH₂CF₃ | CH₂CN | Cl |
| B-0924 | 0 | Me | CH(Me) | Me | CH₂CF₃ | CH₂CN | Cl |
| B-0925 | 0 | H | CH₂CH₂ | Me | CH₂CF₃ | CH₂CN | Cl |
| B-0926 | 0 | Me | CH₂CH₂ | H | CH₂CF₃ | CH₂CN | Cl |
| B-0927 | 0 | Me | CH₂CH₂ | Me | CH₂CF₃ | CH₂CN | Cl |
| B-0928 | 0 | H | CH₂CH(Me) | H | CH₂CF₃ | CH₂CN | Cl |
| B-0929 | 0 | H | CH₂CH(Me) | Me | CH₂CF₃ | CH₂CN | Cl |
| B-0930 | 0 | Me | CH₂CH(Me) | H | CH₂CF₃ | CH₂CN | Cl |
| B-0931 | 0 | Me | CH₂CH(Me) | Me | CH₂CF₃ | CH₂CN | Cl |
| B-0932 | 0 | H | CH(Me)CH₂ | H | CH₂CF₃ | CH₂CN | Cl |
| B-0933 | 0 | H | CH(Me)CH₂ | Me | CH₂CF₃ | CH₂CN | Cl |
| B-0934 | 0 | Me | CH(Me)CH₂ | H | CH₂CF₃ | CH₂CN | Cl |
| B-0935 | 0 | Me | CH(Me)CH₂ | Me | CH₂CF₃ | CH₂CN | Cl |
| B-0936 | 0 | H | CH₂CH₂CH₂ | Me | CH₂CF₃ | CH₂CN | Cl |
| B-0937 | 0 | Me | CH₂CH₂CH₂ | H | CH₂CF₃ | CH₂CN | Cl |
| B-0938 | 0 | Me | CH₂CH₂CH₂ | Me | CH₂CF₃ | CH₂CN | Cl |
| B-0939 | 0 | H | CH₂CH₂CH(Me) | H | CH₂CF₃ | CH₂CN | Cl |
| B-0940 | 0 | H | CH₂CH₂CH(Me) | Me | CH₂CF₃ | CH₂CN | Cl |
| B-0941 | 0 | Me | CH₂CH₂CH(Me) | H | CH₂CF₃ | CH₂CN | Cl |
| B-0942 | 0 | Me | CH₂CH₂CH(Me) | Me | CH₂CF₃ | CH₂CN | Cl |
| B-0943 | 0 | H | CH₂CH(Me)CH₂ | H | CH₂CF₃ | CH₂CN | Cl |
| B-0944 | 0 | H | CH₂CH(Me)CH₂ | Me | CH₂CF₃ | CH₂CN | Cl |
| B-0945 | 0 | Me | CH₂CH(Me)CH₂ | H | CH₂CF₃ | CH₂CN | Cl |
| B-0946 | 0 | Me | CH₂CH(Me)CH₂ | Me | CH₂CF₃ | CH₂CN | Cl |
| B-0947 | 0 | H | CH(Me)CH₂CH₂ | H | CH₂CF₃ | CH₂CN | Cl |
| B-0948 | 0 | H | CH(Me)CH₂CH₂ | Me | CH₂CF₃ | CH₂CN | Cl |
| B-0949 | 0 | Me | CH(Me)CH₂CH₂ | H | CH₂CF₃ | CH₂CN | Cl |
| B-0950 | 0 | Me | CH(Me)CH₂CH₂ | Me | CH₂CF₃ | CH₂CN | Cl |
| B-0951 | 0 | H | single bond | Me | CH₂CF₃ | CH₂CN | Me |
| B-0952 | 0 | H | single bond | Et | CH₂CF₃ | CH₂CN | Me |
| B-0953 | 0 | Me | single bond | H | CH₂CF₃ | CH₂CN | Me |
| B-0954 | 0 | Et | single bond | H | CH₂CF₃ | CH₂CN | Me |
| B-0955 | 0 | Me | single bond | Me | CH₂CF₃ | CH₂CN | Me |
| B-0956 | 0 | H | CH₂ | Me | CH₂CF₃ | CH₂CN | Me |
| B-0957 | 0 | Me | CH₂ | H | CH₂CF₃ | CH₂CN | Me |
| B-0958 | 0 | Me | CH₂ | Me | CH₂CF₃ | CH₂CN | Me |
| B-0959 | 0 | H | CH(Me) | H | CH₂CF₃ | CH₂CN | Me |
| B-0960 | 0 | H | CH(Me) | Me | CH₂CF₃ | CH₂CN | Me |
| B-0961 | 0 | Me | CH(Me) | H | CH₂CF₃ | CH₂CN | Me |
| B-0962 | 0 | Me | CH(Me) | Me | CH₂CF₃ | CH₂CN | Me |

**[Table 186]**

| Compound Number | n | Y | W | z | R¹ | R² | R³ |
|---|---|---|---|---|---|---|---|
| B-0963 | 0 | H | CH₂CH₂ | Me | CH₂CF₃ | CH₂CN | Me |
| B-0964 | 0 | Me | CH₂CH₂ | H | CH₂CF₃ | CH₂CN | Me |
| B-0965 | 0 | Me | CH₂CH₂ | Me | CH₂CF₃ | CH₂CN | Me |
| B-0966 | 0 | H | CH₂CH(Me) | H | CH₂CF₃ | CH₂CN | Me |
| B-0967 | 0 | H | CH₂CH(Me) | Me | CH₂CF₃ | CH₂CN | Me |
| B-0968 | 0 | Me | CH₂CH(Me) | H | CH₂CF₃ | CH₂CN | Me |
| B-0969 | 0 | Me | CH₂CH(Me) | Me | CH₂CF₃ | CH₂CN | Me |
| B-0970 | 0 | H | CH(Me)CH₂ | H | CH₂CF₃ | CH₂CN | Me |
| B-0971 | 0 | H | CH(Me)CH₂ | Me | CH₂CF₃ | CH₂CN | Me |
| B-0972 | 0 | Me | CH(Me)CH₂ | H | CH₂CF₃ | CH₂CN | Me |
| B-0973 | 0 | Me | CH(Me)CH₂ | Me | CH₂CF₃ | CH₂CN | Me |
| B-0974 | 0 | H | CH₂CH₂CH₂ | Me | CH₂CF₃ | CH₂CN | Me |
| B-0975 | 0 | Me | CH₂CH₂CH₂ | H | CH₂CF₃ | CH₂CN | Me |
| B-0976 | 0 | Me | CH₂CH₂CH₂ | Me | CH₂CF₃ | CH₂CN | Me |
| B-0977 | 0 | H | CH₂CH₂CH(Me) | H | CH₂CF₃ | CH₂CN | Me |
| B-0978 | 0 | H | CH₂CH₂CH(Me) | Me | CH₂CF₃ | CH₂CN | Me |
| B-0979 | 0 | Me | CH₂CH₂CH(Me) | H | CH₂CF₃ | CH₂CN | Me |
| B-0980 | 0 | Me | CH₂CH₂CH(Me) | Me | CH₂CF₃ | CH₂CN | Me |
| B-0981 | 0 | H | CH₂CH(Me)CH₂ | H | CH₂CF₃ | CH₂CN | Me |
| B-0982 | 0 | H | CH₂CH(Me)CH₂ | Me | CH₂CF₃ | CH₂CN | Me |
| B-0983 | 0 | Me | CH₂CH(Me)CH₂ | H | CH₂CF₃ | CH₂CN | Me |
| B-0984 | 0 | Me | CH₂CH(Me)CH₂ | Me | CH₂CF₃ | CH₂CN | Me |
| B-0985 | 0 | H | CH(Me)CH₂CH₂ | H | CH₂CF₃ | CH₂CN | Me |
| B-0986 | 0 | H | CH(Me)CH₂CH₂ | Me | CH₂CF₃ | CH₂CN | Me |
| B-0987 | 0 | Me | CH(Me)CH₂CH₂ | H | CH₂CF₃ | CH₂CN | Me |
| B-0988 | 0 | Me | CH(Me)CH₂CH₂ | Me | CH₂CF₃ | CH₂CN | Me |
| B-0989 | 0 | H | single bond | Me | CH₂CF₃ | C(=O)Me | Cl |
| B-0990 | 0 | H | single bond | Et | CH₂CF₃ | C(=O)Me | Cl |
| B-0991 | 0 | Me | single bond | H | CH₂CF₃ | C(=O)Me | Cl |
| B-0992 | 0 | Et | single bond | H | CH₂CF₃ | C(=O)Me | Cl |
| B-0993 | 0 | Me | single bond | Me | CH₂CF₃ | C(=O)Me | Cl |
| B-0994 | 0 | H | CH₂ | Me | CH₂CF₃ | C(=O)Me | Cl |
| B-0995 | 0 | Me | CH₂ | H | CH₂CF₃ | C(=O)Me | Cl |
| B-0996 | 0 | Me | CH₂ | Me | CH₂CF₃ | C(=O)Me | Cl |
| B-0997 | 0 | H | CH(Me) | H | CH₂CF₃ | C(=O)Me | Cl |
| B-0998 | 0 | H | CH(Me) | Me | CH₂CF₃ | C(=O)Me | Cl |
| B-0999 | 0 | Me | CH(Me) | H | CH₂CF₃ | C(=O)Me | Cl |
| B-1000 | 0 | Me | CH(Me) | Me | CH₂CF₃ | C(=O)Me | Cl |
| B-1001 | 0 | H | CH₂CH₂ | Me | CH₂CF₃ | C(=O)Me | Cl |
| B-1002 | 0 | Me | CH₂CH₂ | H | CH₂CF₃ | C(=O)Me | Cl |
| B-1003 | 0 | Me | CH₂CH₂ | Me | CH₂CF₃ | C(=O)Me | Cl |
| B-1004 | 0 | H | CH₂CH(Me) | H | CH₂CF₃ | C(=O)Me | Cl |
| B-1005 | 0 | H | CH₂CH(Me) | Me | CH₂CF₃ | C(=O)Me | Cl |
| B-1006 | 0 | Me | CH₂CH(Me) | H | CH₂CF₃ | C(=O)Me | Cl |

**[Table 187]**

| Compound Number | n | Y | W | z | R¹ | R² | R³ |
|---|---|---|---|---|---|---|---|
| B-1007 | 0 | Me | CH₂CH(Me) | Me | CH₂CF₃ | C(=O)Me | Cl |
| B-1008 | 0 | H | CH(Me)CH₂ | H | CH₂CF₃ | C(=O)Me | Cl |
| B-1009 | 0 | H | CH(Me)CH₂ | Me | CH₂CF₃ | C(=O)Me | Cl |
| B-1010 | 0 | Me | CH(Me)CH₂ | H | CH₂CF₃ | C(=O)Me | Cl |
| B-1011 | 0 | Me | CH(Me)CH₂ | Me | CH₂CF₃ | C(=O)Me | Cl |
| B-1012 | 0 | H | CH₂CH₂CH₂ | Me | CH₂CF₃ | C(=O)Me | Cl |
| B-1013 | 0 | Me | CH₂CH₂CH₂ | H | CH₂CF₃ | C(=O)Me | Cl |
| B-1014 | 0 | Me | CH₂CH₂CH₂ | Me | CH₂CF₃ | C(=O)Me | Cl |
| B-1015 | 0 | H | CH₂CH₂CH(Me) | H | CH₂CF₃ | C(=O)Me | Cl |
| B-1016 | 0 | H | CH₂CH₂CH(Me) | Me | CH₂CF₃ | C(=O)Me | Cl |
| B-1017 | 0 | Me | CH₂CH₂CH(Me) | H | CH₂CF₃ | C(=O)Me | Cl |
| B-1018 | 0 | Me | CH₂CH₂CH(Me) | Me | CH₂CF₃ | C(=O)Me | Cl |
| B-1019 | 0 | H | CH₂CH(Me)CH₂ | H | CH₂CF₃ | C(=O)Me | Cl |
| B-1020 | 0 | H | CH₂CH(Me)CH₂ | Me | CH₂CF₃ | C(=O)Me | Cl |
| B-1021 | 0 | Me | CH₂CH(Me)CH₂ | H | CH₂CF₃ | C(=O)Me | Cl |
| B-1022 | 0 | Me | CH₂CH(Me)CH₂ | Me | CH₂CF₃ | C(=O)Me | Cl |
| B-1023 | 0 | H | CH(Me)CH₂CH₂ | H | CH₂CF₃ | C(=O)Me | Cl |
| B-1024 | 0 | H | CH(Me)CH₂CH₂ | Me | CH₂CF₃ | C(=O)Me | Cl |
| B-1025 | 0 | Me | CH(Me)CH₂CH₂ | H | CH₂CF₃ | C(=O)Me | Cl |
| B-1026 | 0 | Me | CH(Me)CH₂CH₂ | Me | CH₂CF₃ | C(=O)Me | Cl |
| B-1027 | 0 | H | single bond | Me | CH₂CF₃ | C(=O)Me | Me |
| B-1028 | 0 | H | single bond | Et | CH₂CF₃ | C(=O)Me | Me |
| B-1 029 | 0 | Me | single bond | H | CH₂CF₃ | C(=O)Me | Me |
| B-1030 | 0 | Et | single bond | H | CH₂CF₃ | C(=O)Me | Me |
| B-1031 | 0 | Me | single bond | Me | CH₂CF₃ | C(=O)Me | Me |
| B-1032 | 0 | H | CH₂ | Me | CH₂CF₃ | C(=O)Me | Me |
| B-1033 | 0 | Me | CH₂ | H | CH₂CF₃ | C(=O)Me | Me |
| B-1034 | 0 | Me | CH₂ | Me | CH₂CF₃ | C(=O)Me | Me |
| B-1035 | 0 | H | CH(Me) | H | CH₂CF₃ | C(=O)Me | Me |
| B-1036 | 0 | H | CH(Me) | Me | CH₂CF₃ | C(=O)Me | Me |
| B-1037 | 0 | Me | CH(Me) | H | CH₂CF₃ | C(=O)Me | Me |
| B-1038 | 0 | Me | CH(Me) | Me | CH₂CF₃ | C(=O)Me | Me |
| B-1 039 | 0 | H | CH₂CH₂ | Me | CH₂CF₃ | C(=O)Me | Me |
| B-1040 | 0 | Me | CH₂CH₂ | H | CH₂CF₃ | C(=O)Me | Me |
| B-1041 | 0 | Me | CH₂CH₂ | Me | CH₂CF₃ | C(=O)Me | Me |
| B-1042 | 0 | H | CH₂CH(Me) | H | CH₂CF₃ | C(=O)Me | Me |
| B-1043 | 0 | H | CH₂CH(Me) | Me | CH₂CF₃ | C(=O)Me | Me |
| B-1044 | 0 | Me | CH₂CH(Me) | H | CH₂CF₃ | C(=O)Me | Me |
| B-1045 | 0 | Me | CH₂CH(Me) | Me | CH₂CF₃ | C(=O)Me | Me |
| B-1046 | 0 | H | CH(Me)CH₂ | H | CH₂CF₃ | C(=O)Me | Me |
| B-1 047 | 0 | H | CH(Me)CH₂ | Me | CH₂CF₃ | C(=O)Me | Me |
| B-1048 | 0 | Me | CH(Me)CH₂ | H | CH₂CF₃ | C(=O)Me | Me |
| B-1 049 | 0 | Me | CH(Me)CH₂ | Me | CH₂CF₃ | C(=O)Me | Me |
| B-1050 | 0 | H | CH₂CH₂CH₂ | Me | CH₂CF₃ | C(=O)Me | Me |

**[Table 188]**

| Compound Number | n | Y | W | z | R¹ | R² | R³ |
|---|---|---|---|---|---|---|---|
| B-1051 | 0 | Me | CH₂CH₂CH₂ | H | CH₂CF₃ | C(=O)Me | Me |
| B-1052 | 0 | Me | CH₂CH₂CH₂ | Me | CH₂CF₃ | C(=O)Me | Me |
| B-1053 | 0 | H | CH₂CH₂CH(Me) | H | CH₂CF₃ | C(=O)Me | Me |
| B-1054 | 0 | H | CH₂CH₂CH(Me) | Me | CH₂CF₃ | C(=O)Me | Me |
| B-1055 | 0 | Me | CH₂CH₂CH(Me) | H | CH₂CF₃ | C(=O)Me | Me |
| B-1056 | 0 | Me | CH₂CH₂CH(Me) | Me | CH₂CF₃ | C(=O)Me | Me |
| B-1 057 | 0 | H | CH₂CH(Me)CH₂ | H | CH₂CF₃ | C(=O)Me | Me |
| B-1058 | 0 | H | CH₂CH(Me)CH₂ | Me | CH₂CF₃ | C(=O)Me | Me |
| B-1 059 | 0 | Me | CH₂CH(Me)CH₂ | H | CH₂CF₃ | C(=O)Me | Me |
| B-1 060 | 0 | Me | CH₂CH(Me)CH₂ | Me | CH₂CF₃ | C(=O)Me | Me |
| B-1061 | 0 | H | CH(Me)CH₂CH₂ | H | CH₂CF₃ | C(=O)Me | Me |
| B-1 062 | 0 | H | CH(Me)CH₂CH₂ | Me | CH₂CF₃ | C(=O)Me | Me |
| B-1 063 | 0 | Me | CH(Me)CH₂CH₂ | H | CH₂CF₃ | C(=O)Me | Me |
| B-1 064 | 0 | Me | CH(Me)CH₂CH₂ | Me | CH₂CF₃ | C(=O)Me | Me |
| B-1065 | 0 | H | single bond | Me | CH₂CF₃ | C(=O)Ph | Cl |
| B-1 066 | 0 | H | single bond | Et | CH₂CF₃ | C(=O)Ph | Cl |
| B-1 067 | 0 | Me | single bond | H | CH₂CF₃ | C(=O)Ph | Cl |
| B-1068 | 0 | Et | single bond | H | CH₂CF₃ | C(=O)Ph | Cl |
| B-1 069 | 0 | Me | single bond | Me | CH₂CF₃ | C(=O)Ph | Cl |
| B-1070 | 0 | H | CH₂ | Me | CH₂CF₃ | C(=O)Ph | Cl |
| B-1071 | 0 | Me | CH₂ | H | CH₂CF₃ | C(=O)Ph | Cl |
| B-1072 | 0 | Me | CH₂ | Me | CH₂CF₃ | C(=O)Ph | Cl |
| B-1073 | 0 | H | CH(Me) | H | CH₂CF₃ | C(=O)Ph | Cl |
| B-1074 | 0 | H | CH(Me) | Me | CH₂CF₃ | C(=O)Ph | Cl |
| B-1075 | 0 | Me | CH(Me) | H | CH₂CF₃ | C(=O)Ph | Cl |
| B-1076 | 0 | Me | CH(Me) | Me | CH₂CF₃ | C(=O)Ph | Cl |
| B-1 077 | 0 | H | CH₂CH₂ | Me | CH₂CF₃ | C(=O)Ph | Cl |
| B-1078 | 0 | Me | CH₂CH₂ | H | CH₂CF₃ | C(=O)Ph | Cl |
| B-1 079 | 0 | Me | CH₂CH₂ | Me | CH₂CF₃ | C(=O)Ph | Cl |
| B-1080 | 0 | H | CH₂CH(Me) | H | CH₂CF₃ | C(=O)Ph | Cl |
| B-1081 | 0 | H | CH₂CH(Me) | Me | CH₂CF₃ | C(=O)Ph | Cl |
| B-1082 | 0 | Me | CH₂CH(Me) | H | CH₂CF₃ | C(=O)Ph | Cl |
| B-1083 | 0 | Me | CH₂CH(Me) | Me | CH₂CF₃ | C(=O)Ph | Cl |
| B-1084 | 0 | H | CH(Me)CH₂ | H | CH₂CF₃ | C(=O)Ph | Cl |
| B-1085 | 0 | H | CH(Me)CH₂ | Me | CH₂CF₃ | C(=O)Ph | Cl |
| B-1086 | 0 | Me | CH(Me)CH₂ | H | CH₂CF₃ | C(=O)Ph | Cl |
| B-1 087 | 0 | Me | CH(Me)CH₂ | Me | CH₂CF₃ | C(=O)Ph | Cl |
| B-1088 | 0 | H | CH₂CH₂CH₂ | Me | CH₂CF₃ | C(=O)Ph | Cl |
| B-1 089 | 0 | Me | CH₂CH₂CH₂ | H | CH₂CF₃ | C(=O)Ph | Cl |
| B-1090 | 0 | Me | CH₂CH₂CH₂ | Me | CH₂CF₃ | C(=O)Ph | Cl |
| B-1091 | 0 | H | CH₂CH₂CH(Me) | H | CH₂CF₃ | C(=O)Ph | Cl |
| B-1092 | 0 | H | CH₂CH₂CH(Me) | Me | CH₂CF₃ | C(=O)Ph | Cl |
| B-1093 | 0 | Me | CH₂CH₂CH(Me) | H | CH₂CF₃ | C(=O)Ph | Cl |
| B-1094 | 0 | Me | CH₂CH₂CH(Me) | Me | CH₂CF₃ | C(=O)Ph | Cl |

**[Table 189]**

| Compound Number | n | Y | W | z | R¹ | R² | R³ |
|---|---|---|---|---|---|---|---|
| B-1095 | 0 | H | CH₂CH(Me)CH₂ | H | CH₂CF₃ | C(=O)Ph | Cl |
| B-1096 | 0 | H | CH₂CH(Me)CH₂ | Me | CH₂CF₃ | C(=O)Ph | Cl |
| B-1 097 | 0 | Me | CH₂CH(Me)CH₂ | H | CH₂CF₃ | C(=O)Ph | Cl |
| B-1098 | 0 | Me | CH₂CH(Me)CH₂ | Me | CH₂CF₃ | C(=O)Ph | Cl |
| B-1 099 | 0 | H | CH(Me)CH₂CH₂ | H | CH₂CF₃ | C(=O)Ph | Cl |
| B-1100 | 0 | H | CH(Me)CH₂CH₂ | Me | CH₂CF₃ | C(=O)Ph | Cl |
| B-1101 | 0 | Me | CH(Me)CH₂CH₂ | H | CH₂CF₃ | C(=O)Ph | Cl |
| B-1102 | 0 | Me | CH(Me)CH₂CH₂ | Me | CH₂CF₃ | C(=O)Ph | Cl |
| B-1103 | 0 | H | single bond | Me | CH₂CF₃ | C(=O)Ph | Me |
| B-1104 | 0 | H | single bond | Et | CH₂CF₃ | C(=O)Ph | Me |
| B-1105 | 0 | Me | single bond | H | CH₂CF₃ | C(=O)Ph | Me |
| B-1106 | 0 | Et | single bond | H | CH₂CF₃ | C(=O)Ph | Me |
| B-1107 | 0 | Me | single bond | Me | CH₂CF₃ | C(=O)Ph | Me |
| B-1108 | 0 | H | CH₂ | Me | CH₂CF₃ | C(=O)Ph | Me |
| B-1109 | 0 | Me | CH₂ | H | CH₂CF₃ | C(=O)Ph | Me |
| B-1110 | 0 | Me | CH₂ | Me | CH₂CF₃ | C(=O)Ph | Me |
| B-1111 | 0 | H | CH(Me) | H | CH₂CF₃ | C(=O)Ph | Me |
| B-1112 | 0 | H | CH(Me) | Me | CH₂CF₃ | C(=O)Ph | Me |
| B-1113 | 0 | Me | CH(Me) | H | CH₂CF₃ | C(=O)Ph | Me |
| B-1114 | 0 | Me | CH(Me) | Me | CH₂CF₃ | C(=O)Ph | Me |
| B-1115 | 0 | H | CH₂CH₂ | Me | CH₂CF₃ | C(=O)Ph | Me |
| B-1116 | 0 | Me | CH₂CH₂ | H | CH₂CF₃ | C(=O)Ph | Me |
| B-1117 | 0 | Me | CH₂CH₂ | Me | CH₂CF₃ | C(=O)Ph | Me |
| B-1118 | 0 | H | CH₂CH(Me) | H | CH₂CF₃ | C(=O)Ph | Me |
| B-1119 | 0 | H | CH₂CH(Me) | Me | CH₂CF₃ | C(=O)Ph | Me |
| B-1120 | 0 | Me | CH₂CH(Me) | H | CH₂CF₃ | C(=O)Ph | Me |
| B-1121 | 0 | Me | CH₂CH(Me) | Me | CH₂CF₃ | C(=O)Ph | Me |
| B-1122 | 0 | H | CH(Me)CH₂ | H | CH₂CF₃ | C(=O)Ph | Me |
| B-1123 | 0 | H | CH(Me)CH₂ | Me | CH₂CF₃ | C(=O)Ph | Me |
| B-1124 | 0 | Me | CH(Me)CH₂ | H | CH₂CF₃ | C(=O)Ph | Me |
| B-1125 | 0 | Me | CH(Me)CH₂ | Me | CH₂CF₃ | C(=O)Ph | Me |
| B-1126 | 0 | H | CH₂CH₂CH₂ | Me | CH₂CF₃ | C(=O)Ph | Me |
| B-1127 | 0 | Me | CH₂CH₂CH₂ | H | CH₂CF₃ | C(=O)Ph | Me |
| B-1128 | 0 | Me | CH₂CH₂CH₂ | Me | CH₂CF₃ | C(=O)Ph | Me |
| B-1129 | 0 | H | CH₂CH₂CH(Me) | H | CH₂CF₃ | C(=O)Ph | Me |
| B-1130 | 0 | H | CH₂CH₂CH(Me) | Me | CH₂CF₃ | C(=O)Ph | Me |
| B-1131 | 0 | Me | CH₂CH₂CH(Me) | H | CH₂CF₃ | C(=O)Ph | Me |
| B-1132 | 0 | Me | CH₂CH₂CH(Me) | Me | CH₂CF₃ | C(=O)Ph | Me |
| B-1133 | 0 | H | CH₂CH(Me)CH₂ | H | CH₂CF₃ | C(=O)Ph | Me |
| B-1134 | 0 | H | CH₂CH(Me)CH₂ | Me | CH₂CF₃ | C(=O)Ph | Me |
| B-1135 | 0 | Me | CH₂CH(Me)CH₂ | H | CH₂CF₃ | C(=O)Ph | Me |
| B-1136 | 0 | Me | CH₂CH(Me)CH₂ | Me | CH₂CF₃ | C(=O)Ph | Me |
| B-1137 | 0 | H | CH(Me)CH₂CH₂ | H | CH₂CF₃ | C(=O)Ph | Me |
| B-1138 | 0 | H | CH(Me)CH₂CH₂ | Me | CH₂CF₃ | C(=O)Ph | Me |

**[Table 190]**

| Compound Number | n | Y | W | z | R¹ | R² | R³ |
|---|---|---|---|---|---|---|---|
| B-1139 | 0 | Me | CH(Me)CH₂CH₂ | H | CH₂CF₃ | C(=O)Ph | Me |
| B-1140 | 0 | Me | CH(Me)CH₂CH₂ | Me | CH₂CF₃ | C(=O)Ph | Me |
| B-1141 | 0 | H | single bond | Me | CH₂CF₃ | C(=O)OEt | Cl |
| B-1142 | 0 | H | single bond | Et | CH₂CF₃ | C(=O)OEt | Cl |
| B-1143 | 0 | Me | single bond | H | CH₂CF₃ | C(=O)OEt | Cl |
| B-1144 | 0 | Et | single bond | H | CH₂CF₃ | C(=O)OEt | Cl |
| B-1145 | 0 | Me | single bond | Me | CH₂CF₃ | C(=O)OEt | Cl |
| B-1146 | 0 | H | CH₂ | Me | CH₂CF₃ | C(=O)OEt | Cl |
| B-1147 | 0 | Me | CH₂ | H | CH₂CF₃ | C(=O)OEt | Cl |
| B-1148 | 0 | Me | CH₂ | Me | CH₂CF₃ | C(=O)OEt | Cl |
| B-1149 | 0 | H | CH(Me) | H | CH₂CF₃ | C(=O)OEt | Cl |
| B-1150 | 0 | H | CH(Me) | Me | CH₂CF₃ | C(=O)OEt | Cl |
| B-1151 | 0 | Me | CH(Me) | H | CH₂CF₃ | C(=O)OEt | Cl |
| B-1152 | 0 | Me | CH(Me) | Me | CH₂CF₃ | C(=O)OEt | Cl |
| B-1153 | 0 | H | CH₂CH₂ | Me | CH₂CF₃ | C(=O)OEt | Cl |
| B-1154 | 0 | Me | CH₂CH₂ | H | CH₂CF₃ | C(=O)OEt | Cl |
| B-1155 | 0 | Me | CH₂CH₂ | Me | CH₂CF₃ | C(=O)OEt | Cl |
| B-1156 | 0 | H | CH₂CH(Me) | H | CH₂CF₃ | C(=O)OEt | Cl |
| B-1157 | 0 | H | CH₂CH(Me) | Me | CH₂CF₃ | C(=O)OEt | Cl |
| B-1158 | 0 | Me | CH₂CH(Me) | H | CH₂CF₃ | C(=O)OEt | Cl |
| B-1159 | 0 | Me | CH₂CH(Me) | Me | CH₂CF₃ | C(=O)OEt | Cl |
| B-1160 | 0 | H | CH(Me)CH₂ | H | CH₂CF₃ | C(=O)OEt | Cl |
| B-1161 | 0 | H | CH(Me)CH₂ | Me | CH₂CF₃ | C(=O)OEt | Cl |
| B-1162 | 0 | Me | CH(Me)CH₂ | H | CH₂CF₃ | C(=O)OEt | Cl |
| B-1163 | 0 | Me | CH(Me)CH₂ | Me | CH₂CF₃ | C(=O)OEt | Cl |
| B-1164 | 0 | H | CH₂CH₂CH₂ | Me | CH₂CF₃ | C(=O)OEt | Cl |
| B-1165 | 0 | Me | CH₂CH₂CH₂ | H | CH₂CF₃ | C(=O)OEt | Cl |
| B-1166 | 0 | Me | CH₂CH₂CH₂ | Me | CH₂CF₃ | C(=O)OEt | Cl |
| B-1167 | 0 | H | CH₂CH₂CH(Me) | H | CH₂CF₃ | C(=O)OEt | Cl |
| B-1168 | 0 | H | CH₂CH₂CH(Me) | Me | CH₂CF₃ | C(=O)OEt | Cl |
| B-1169 | 0 | Me | CH₂CH₂CH(Me) | H | CH₂CF₃ | C(=O)OEt | Cl |
| B-1170 | 0 | Me | CH₂CH₂CH(Me) | Me | CH₂CF₃ | C(=O)OEt | Cl |
| B-1171 | 0 | H | CH₂CH(Me)CH₂ | H | CH₂CF₃ | C(=O)OEt | Cl |
| B-1172 | 0 | H | CH₂CH(Me)CH₂ | Me | CH₂CF₃ | C(=O)OEt | Cl |
| B-1173 | 0 | Me | CH₂CH(Me)CH₂ | H | CH₂CF₃ | C(=O)OEt | Cl |
| B-1174 | 0 | Me | CH₂CH(Me)CH₂ | Me | CH₂CF₃ | C(=O)OEt | Cl |
| B-1175 | 0 | H | CH(Me)CH₂CH₂ | H | CH₂CF₃ | C(=O)OEt | Cl |
| B-1176 | 0 | H | CH(Me)CH₂CH₂ | Me | CH₂CF₃ | C(=O)OEt | Cl |
| B-1177 | 0 | Me | CH(Me)CH₂CH₂ | H | CH₂CF₃ | C(=O)OEt | Cl |
| B-1178 | 0 | Me | CH(Me)CH₂CH₂ | Me | CH₂CF₃ | C(=O)OEt | Cl |
| B-1179 | 0 | H | single bond | Me | CH₂CF₃ | C(=O)OEt | Me |
| B-1180 | 0 | H | single bond | Et | CH₂CF₃ | C(=O)OEt | Me |
| B-1181 | 0 | Me | single bond | H | CH₂CF₃ | C(=O)OEt | Me |
| B-1182 | 0 | Et | single bond | H | CH₂CF₃ | C(=O)OEt | Me |

**[Table 191]**

| Compound Number | n | Y | W | Z | R¹ | R² | R³ |
|---|---|---|---|---|---|---|---|
| B-1183 | 0 | Me | single bond | Me | CH₂CF₃ | C(=O)OEt | Me |
| B-1184 | 0 | H | CH₂ | Me | CH₂CF₃ | C(=O)OEt | Me |
| B-1185 | 0 | Me | CH₂ | H | CH₂CF₃ | C(=O)OEt | Me |
| B-1186 | 0 | Me | CH₂ | Me | CH₂CF₃ | C(=O)OEt | Me |
| B-1187 | 0 | H | CH(Me) | H | CH₂CF₃ | C(=O)OEt | Me |
| B-1188 | 0 | H | CH(Me) | Me | CH₂CF₃ | C(=O)OEt | Me |
| B-1189 | 0 | Me | CH(Me) | H | CH₂CF₃ | C(=O)OEt | Me |
| B-1190 | 0 | Me | CH(Me) | Me | CH₂CF₃ | C(=O)OEt | Me |
| B-1191 | 0 | H | CH₂CH₂ | Me | CH₂CF₃ | C(=O)OEt | Me |
| B-1192 | 0 | Me | CH₂CH₂ | H | CH₂CF₃ | C(=O)OEt | Me |
| B-1193 | 0 | Me | CH₂CH₂ | Me | CH₂CF₃ | C(=O)OEt | Me |
| B-1194 | 0 | H | CH₂CH(Me) | H | CH₂CF₃ | C(=O)OEt | Me |
| B-1195 | 0 | H | CH₂CH(Me) | Me | CH₂CF₃ | C(=O)OEt | Me |
| B-1196 | 0 | Me | CH₂CH(Me) | H | CH₂CF₃ | C(=O)OEt | Me |
| B-1197 | 0 | Me | CH₂CH(Me) | Me | CH₂CF₃ | C(=O)OEt | Me |
| B-1198 | 0 | H | CH(Me)CH₂ | H | CH₂CF₃ | C(=O)OEt | Me |
| B-1199 | 0 | H | CH(Me)CH₂ | Me | CH₂CF₃ | C(=O)OEt | Me |
| B-1200 | 0 | Me | CH(Me)CH₂ | H | CH₂CF₃ | C(=O)OEt | Me |
| B-1201 | 0 | Me | CH(Me)CH₂ | Me | CH₂CF₃ | C(=O)OEt | Me |
| B-1202 | 0 | H | CH₂CH₂CH₂ | Me | CH₂CF₃ | C(=O)OEt | Me |
| B-1203 | 0 | Me | CH₂CH₂CH₂ | H | CH₂CF₃ | C(=O)OEt | Me |
| B-1204 | 0 | Me | CH₂CH₂CH₂ | Me | CH₂CF₃ | C(=O)OEt | Me |
| B-1205 | 0 | H | CH₂CH₂CH(Me) | H | CH₂CF₃ | C(=O)OEt | Me |
| B-1206 | 0 | H | CH₂CH₂CH(Me) | Me | CH₂CF₃ | C(=O)OEt | Me |
| B-1207 | 0 | Me | CH₂CH₂CH(Me) | H | CH₂CF₃ | C(=O)OEt | Me |
| B-1208 | 0 | Me | CH₂CH₂CH(Me) | Me | CH₂CF₃ | C(=O)OEt | Me |
| B-1209 | 0 | H | CH₂CH(Me)CH₂ | H | CH₂CF₃ | C(=O)OEt | Me |
| B-1210 | 0 | H | CH₂CH(Me)CH₂ | Me | CH₂CF₃ | C(=O)OEt | Me |
| B-1211 | 0 | Me | CH₂CH(Me)CH₂ | H | CH₂CF₃ | C(=O)OEt | Me |
| B-1212 | 0 | Me | CH₂CH(Me)CH₂ | Me | CH₂CF₃ | C(=O)OEt | Me |
| B-1213 | 0 | H | CH(Me)CH₂CH₂ | H | CH₂CF₃ | C(=O)OEt | Me |
| B-1214 | 0 | H | CH(Me)CH₂CH₂ | Me | CH₂CF₃ | C(=O)OEt | Me |
| B-1215 | 0 | Me | CH(Me)CH₂CH₂ | H | CH₂CF₃ | C(=O)OEt | Me |
| B-1216 | 0 | Me | CH(Me)CH₂CH₂ | Me | CH₂CF₃ | C(=O)OEt | Me |
| B-1217 | 0 | H | single bond | Me | CH₂CF₃ | S(=O)₂Me | Cl |
| B-1218 | 0 | H | single bond | Et | CH₂CF₃ | S(=O)₂Me | Cl |
| B-1219 | 0 | Me | single bond | H | CH₂CF₃ | S(=O)₂Me | Cl |
| B-1220 | 0 | Et | single bond | H | CH₂CF₃ | S(=O)₂Me | Cl |
| B-1221 | 0 | Me | single bond | Me | CH₂CF₃ | S(=O)₂Me | Cl |
| B-1222 | 0 | H | CH₂ | Me | CH₂CF₃ | S(=O)₂Me | Cl |
| B-1223 | 0 | Me | CH₂ | H | CH₂CF₃ | S(=O)₂Me | Cl |
| B-1224 | 0 | Me | CH₂ | Me | CH₂CF₃ | S(=O)₂Me | Cl |
| B-1225 | 0 | H | CH(Me) | H | CH₂CF₃ | S(=O)₂Me | Cl |
| B-1226 | 0 | H | CH(Me) | Me | CH₂CF₃ | S(=O)₂Me | Cl |

**[Table 192]**

| Compound Number | n | Y | W | Z | R¹ | R² | R³ |
|---|---|---|---|---|---|---|---|
| B-1227 | 0 | Me | CH(Me) | H | CH₂CF₃ | S(=O)₂Me | Cl |
| B-1228 | 0 | Me | CH(Me) | Me | CH₂CF₃ | S(=O)₂Me | Cl |
| B-1229 | 0 | H | CH₂CH₂ | Me | CH₂CF₃ | S(=O)₂Me | Cl |
| B-1230 | 0 | Me | CH₂CH₂ | H | CH₂CF₃ | S(=O)₂Me | Cl |
| B-1231 | 0 | Me | CH₂CH₂ | Me | CH₂CF₃ | S(=O)₂Me | Cl |
| B-1232 | 0 | H | CH₂CH(Me) | H | CH₂CF₃ | S(=O)₂Me | Cl |
| B-1233 | 0 | H | CH₂CH(Me) | Me | CH₂CF₃ | S(=O)₂Me | Cl |
| B-1234 | 0 | Me | CH₂CH(Me) | H | CH₂CF₃ | S(=O)₂Me | Cl |
| B-1235 | 0 | Me | CH₂CH(Me) | Me | CH₂CF₃ | S(=O)₂Me | Cl |
| B-1236 | 0 | H | CH(Me)CH₂ | H | CH₂CF₃ | S(=O)₂Me | Cl |
| B-1237 | 0 | H | CH(Me)CH₂ | Me | CH₂CF₃ | S(=O)₂Me | Cl |
| B-1238 | 0 | Me | CH(Me)CH₂ | H | CH₂CF₃ | S(=O)₂Me | Cl |
| B-1239 | 0 | Me | CH(Me)CH₂ | Me | CH₂CF₃ | S(=O)₂Me | Cl |
| B-1240 | 0 | H | CH₂CH₂CH₂ | Me | CH₂CF₃ | S(=O)₂Me | Cl |
| B-1241 | 0 | Me | CH₂CH₂CH₂ | H | CH₂CF₃ | S(=O)₂Me | Cl |
| B-1242 | 0 | Me | CH₂CH₂CH₂ | Me | CH₂CF₃ | S(=O)₂Me | Cl |
| B-1243 | 0 | H | CH₂CH₂CH(Me) | H | CH₂CF₃ | S(=O)₂Me | Cl |
| B-1244 | 0 | H | CH₂CH₂CH(Me) | Me | CH₂CF₃ | S(=O)₂Me | Cl |
| B-1245 | 0 | Me | CH₂CH₂CH(Me) | H | CH₂CF₃ | S(=O)₂Me | Cl |
| B-1246 | 0 | Me | CH₂CH₂CH(Me) | Me | CH₂CF₃ | S(=O)₂Me | Cl |
| B-1247 | 0 | H | CH₂CH(Me)CH₂ | H | CH₂CF₃ | S(=O)₂Me | Cl |
| B-1248 | 0 | H | CH₂CH(Me)CH₂ | Me | CH₂CF₃ | S(=O)₂Me | Cl |
| B-1249 | 0 | Me | CH₂CH(Me)CH₂ | H | CH₂CF₃ | S(=O)₂Me | Cl |
| B-1250 | 0 | Me | CH₂CH(Me)CH₂ | Me | CH₂CF₃ | S(=O)₂Me | Cl |
| B-1251 | 0 | H | CH(Me)CH₂CH₂ | H | CH₂CF₃ | S(=O)₂Me | Cl |
| B-1252 | 0 | H | CH(Me)CH₂CH₂ | Me | CH₂CF₃ | S(=O)₂Me | Cl |
| B-1253 | 0 | Me | CH(Me)CH₂CH₂ | H | CH₂CF₃ | S(=O)₂Me | Cl |
| B-1254 | 0 | Me | CH(Me)CH₂CH₂ | Me | CH₂CF₃ | S(=O)₂Me | Cl |
| B-1255 | 0 | H | single bond | Me | CH₂CF₃ | S(=O)₂Me | Me |
| B-1256 | 0 | H | single bond | Et | CH₂CF₃ | S(=O)₂Me | Me |
| B-1257 | 0 | Me | single bond | H | CH₂CF₃ | S(=O)₂Me | Me |
| B-1258 | 0 | Et | single bond | H | CH₂CF₃ | S(=O)₂Me | Me |
| B-1259 | 0 | Me | single bond | Me | CH₂CF₃ | S(=O)₂Me | Me |
| B-1260 | 0 | H | CH₂ | Me | CH₂CF₃ | S(=O)₂Me | Me |
| B-1261 | 0 | Me | CH₂ | H | CH₂CF₃ | S(=O)₂Me | Me |
| B-1262 | 0 | Me | CH₂ | Me | CH₂CF₃ | S(=O)₂Me | Me |
| B-1263 | 0 | H | CH(Me) | H | CH₂CF₃ | S(=O)₂Me | Me |
| B-1264 | 0 | H | CH(Me) | Me | CH₂CF₃ | S(=O)₂Me | Me |
| B-1265 | 0 | Me | CH(Me) | H | CH₂CF₃ | S(=O)₂Me | Me |
| B-1266 | 0 | Me | CH(Me) | Me | CH₂CF₃ | S(=O)₂Me | Me |
| B-1267 | 0 | H | CH₂CH₂ | Me | CH₂CF₃ | S(=O)₂Me | Me |
| B-1268 | 0 | Me | CH₂CH₂ | H | CH₂CF₃ | S(=O)₂Me | Me |
| B-1269 | 0 | Me | CH₂CH₂ | Me | CH₂CF₃ | S(=O)₂Me | Me |
| B-1270 | 0 | H | CH₂CH(Me) | H | CH₂CF₃ | S(=O)₂Me | Me |

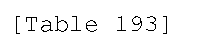

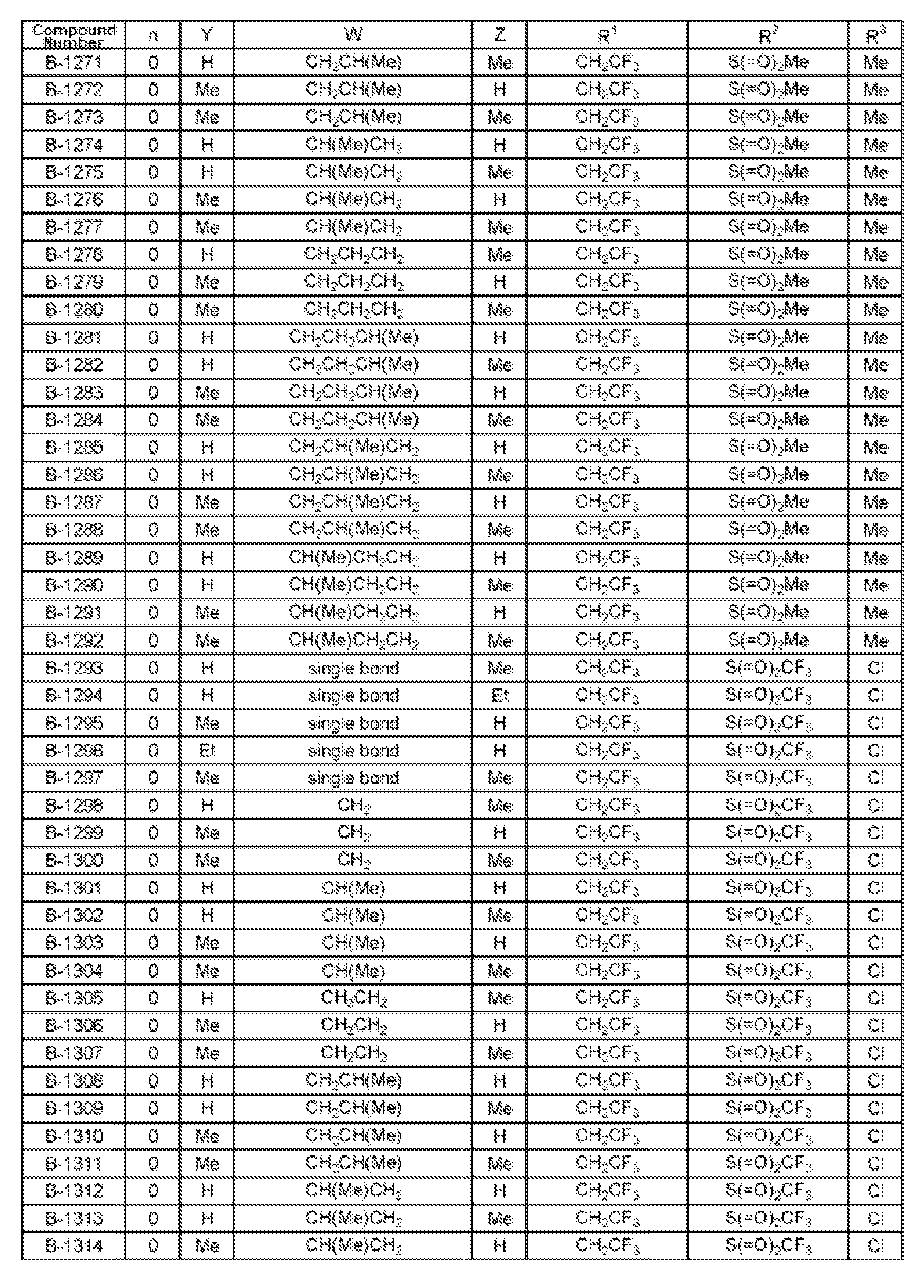

**[Table 194]**

| Compound Number | n | Y | W | Z | R¹ | R² | R³ |
|---|---|---|---|---|---|---|---|
| B-1315 | 0 | Me | CH(Me)CH₂ | Me | CH₂CF₃ | S(=O)₂CF₃ | Cl |
| B-1316 | 0 | H | CH₂CH₂CH₂ | Me | CH₂CF₃ | S(=O)₂CF₃ | Cl |
| B-1317 | 0 | Me | CH₂CH₂CH₂ | H | CH₂CF₃ | S(=O)₂CF₃ | Cl |
| B-1318 | 0 | Me | CH₂CH₂CH₂ | Me | CH₂CF₃ | S(=O)₂CF₃ | Cl |
| B-1319 | 0 | H | CH₂CH₂CH(Me) | H | CH₂CF₃ | S(=O)₂CF₃ | Cl |
| B-1320 | 0 | H | CH₂CH₂CH(Me) | Me | CH₂CF₃ | S(=O)₂CF₃ | Cl |
| B-1321 | 0 | Me | CH₂CH₂CH(Me) | H | CH₂CF₃ | S(=O)₂CF₃ | Cl |
| B-1322 | 0 | Me | CH₂CH₂CH(Me) | Me | CH₂CF₃ | S(=O)₂CF₃ | Cl |
| B-1323 | 0 | H | CH₂CH(Me)CH₂ | H | CH₂CF₃ | S(=O)₂CF₃ | Cl |
| B-1324 | 0 | H | CH₂CH(Me)CH₂ | Me | CH₂CF₃ | S(=O)₂CF₃ | Cl |
| B-1325 | 0 | Me | CH₂CH(Me)CH₂ | H | CH₂CF₃ | S(=O)₂CF₃ | Cl |
| B-1326 | 0 | Me | CH₂CH(Me)CH₂ | Me | CH₂CF₃ | S(=O)₂CF₃ | Cl |
| B-1327 | 0 | H | CH(Me)CH₂CH₂ | H | CH₂CF₃ | S(=O)₂CF₃ | Cl |
| B-1328 | 0 | H | CH(Me)CH₂CH₂ | Me | CH₂CF₃ | S(=O)₂CF₃ | Cl |
| B-1329 | 0 | Me | CH(Me)CH₂CH₂ | H | CH₂CF₃ | S(=O)₂CF₃ | Cl |
| B-1330 | 0 | Me | CH(Me)CH₂CH₂ | Me | CH₂CF₃ | S(=O)₂CF₃ | Cl |
| B-1331 | 0 | H | single bond | Me | CH₂CF₃ | S(=O)₂CF₃ | Me |
| B-1332 | 0 | H | single bond | Et | CH₂CF₃ | S(=O)₂CF₃ | Me |
| B-1333 | 0 | Me | single bond | H | CH₂CF₃ | S(=O)₂CF₃ | Me |
| B-1334 | 0 | Et | single bond | H | CH₂CF₃ | S(=O)₂CF₃ | Me |
| B-1335 | 0 | Me | single bond | Me | CH₂CF₃ | S(=O)₂CF₃ | Me |
| B-1336 | 0 | H | CH₂ | Me | CH₂CF₃ | S(=O)₂CF₃ | Me |
| B-1337 | 0 | Me | CH₂ | H | CH₂CF₃ | S(=O)₂CF₃ | Me |
| B-1338 | 0 | Me | CH₂ | Me | CH₂CF₃ | S(=O)₂CF₃ | Me |
| B-1339 | 0 | H | CH(Me) | H | CH₂CF₃ | S(=O)₂CF₃ | Me |
| B-1340 | 0 | H | CH(Me) | Me | CH₂CF₃ | S(=O)₂CF₃ | Me |
| B-1341 | 0 | Me | CH(Me) | H | CH₂CF₃ | S(=O)₂CF₃ | Me |
| B-1342 | 0 | Me | CH(Me) | Me | CH₂CF₃ | S(=O)₂CF₃ | Me |
| B-1343 | 0 | H | CH₂CH₂ | Me | CH₂CF₃ | S(=O)₂CF₃ | Me |
| B-1344 | 0 | Me | CH₂CH₂ | H | CH₂CF₃ | S(=O)₂CF₃ | Me |
| B-1345 | 0 | Me | CH₂CH₂ | Me | CH₂CF₃ | S(=O)₂CF₃ | Me |
| B-1346 | 0 | H | CH₂CH(Me) | H | CH₂CF₃ | S(=O)₂CF₃ | Me |
| B-1347 | 0 | H | CH₂CH(Me) | Me | CH₂CF₃ | S(=O)₂CF₃ | Me |
| B-1348 | 0 | Me | CH₂CH(Me) | H | CH₂CF₃ | S(=O)₂CF₃ | Me |
| B-1349 | 0 | Me | CH₂CH(Me) | Me | CH₂CF₃ | S(=O)₂CF₃ | Me |
| B-1350 | 0 | H | CH(Me)CH₂ | H | CH₂CF₃ | S(=O)₂CF₃ | Me |
| B-1351 | 0 | H | CH(Me)CH₂ | Me | CH₂CF₃ | S(=O)₂CF₃ | Me |
| B-1352 | 0 | Me | CH(Me)CH₂ | H | CH₂CF₃ | S(=O)₂CF₃ | Me |
| B-1353 | 0 | Me | CH(Me)CH₂ | Me | CH₂CF₃ | S(=O)₂CF₃ | Me |
| B-1354 | 0 | H | CH₂CH₂CH₂ | Me | CH₂CF₃ | S(=O)₂CF₃ | Me |
| B-1355 | 0 | Me | CH₂CH₂CH₂ | H | CH₂CF₃ | S(=O)₂CF₃ | Me |
| B-1356 | 0 | Me | CH₂CH₂CH₂ | Me | CH₂CF₃ | S(=O)₂CF₃ | Me |
| B-1357 | 0 | H | CH₂CH₂CH(Me) | H | CH₂CF₃ | S(=O)₂CF₃ | Me |
| B-1358 | 0 | H | CH₂CH₂CH(Me) | Me | CH₂CF₃ | S(=O)₂CF₃ | Me |

**[Table 195]**

| Compound Number | n | Y | W | Z | R¹ | R² | R³ |
|---|---|---|---|---|---|---|---|
| B-1359 | 0 | Me | CH₂CH₂CH(Me) | H | CH₂CF₃ | S(=O)₂CF₃ | Me |
| B-1360 | 0 | Me | CH₂CH₂CH(Me) | Me | CH₂CF₃ | S(=O)₂CF₃ | Me |
| B-1361 | 0 | H | CH₂CH(Me)CH₂ | H | CH₂CF₃ | S(=O)₂CF₃ | Me |
| B-1362 | 0 | H | CH₂CH(Me)CH₂ | Me | CH₂CF₃ | S(=O)₂CF₃ | Me |
| B-1363 | 0 | Me | CH₂CH(Me)CH₂ | H | CH₂CF₃ | S(=O)₂CF₃ | Me |
| B-1364 | 0 | Me | CH₂CH(Me)CH₂ | Me | CH₂CF₃ | S(=O)₂CF₃ | Me |
| B-1365 | 0 | H | CH(Me)CH₂CH₂ | H | CH₂CF₃ | S(=O)₂CF₃ | Me |
| B-1366 | 0 | H | CH(Me)CH₂CH₂ | Me | CH₂CF₃ | S(=O)₂CF₃ | Me |
| B-1367 | 0 | Me | CH(Me)CH₂CH₂ | H | CH₂CF₃ | S(=O)₂CF₃ | Me |
| B-1368 | 0 | Me | CH(Me)CH₂CH₂ | Me | CH₂CF₃ | S(=O)₂CF₃ | Me |
| B-1369 | 1 | H | single bond | Me | CH₂CF₃ | Me | Cl |
| B-1370 | 1 | H | single bond | Et | CH₂CF₃ | Me | Cl |
| B-1371 | 1 | Me | single bond | H | CH₂CF₃ | Me | Cl |
| B-1372 | 1 | Et | single bond | H | CH₂CF₃ | Me | Cl |
| B-1373 | 1 | Me | single bond | Me | CH₂CF₃ | Me | Cl |
| B-1374 | 1 | H | CH₂ | Me | CH₂CF₃ | Me | Cl |
| B-1375 | 1 | Me | CH₂ | H | CH₂CF₃ | Me | Cl |
| B-1376 | 1 | Me | CH₂ | Me | CH₂CF₃ | Me | Cl |
| B-1377 | 1 | H | CH(Me) | H | CH₂CF₃ | Me | Cl |
| B-1378 | 1 | H | CH(Me) | Me | CH₂CF₃ | Me | Cl |
| B-1379 | 1 | Me | CH(Me) | H | CH₂CF₃ | Me | Cl |
| B-1380 | 1 | Me | CH(Me) | Me | CH₂CF₃ | Me | Cl |
| B-1381 | 1 | H | CH₂CH₂ | Me | CH₂CF₃ | Me | Cl |
| B-1382 | 1 | Me | CH₂CH₂ | H | CH₂CF₃ | Me | Cl |
| B-1383 | 1 | Me | CH₂CH₂ | Me | CH₂CF₃ | Me | Cl |
| B-1384 | 1 | H | CH₂CH(Me) | H | CH₂CF₃ | Me | Cl |
| B-1385 | 1 | H | CH₂CH(Me) | Me | CH₂CF₃ | Me | Cl |
| B-1386 | 1 | Me | CH₂CH(Me) | H | CH₂CF₃ | Me | Cl |
| B-1387 | 1 | Me | CH₂CH(Me) | Me | CH₂CF₃ | Me | Cl |
| B-1388 | 1 | H | CH(Me)CH₂ | H | CH₂CF₃ | Me | Cl |
| B-1389 | 1 | H | CH(Me)CH₂ | Me | CH₂CF₃ | Me | Cl |
| B-1390 | 1 | Me | CH(Me)CH₂ | H | CH₂CF₃ | Me | Cl |
| B-1391 | 1 | Me | CH(Me)CH₂ | Me | CH₂CF₃ | Me | Cl |
| B-1392 | 1 | H | CH₂CH₂CH₂ | Me | CH₂CF₃ | Me | Cl |
| B-1393 | 1 | Me | CH₂CH₂CH₂ | H | CH₂CF₃ | Me | Cl |
| B-1394 | 1 | Me | CH₂CH₂CH₂ | Me | CH₂CF₃ | Me | Cl |
| B-1395 | 1 | H | CH₂CH₂CH(Me) | H | CH₂CF₃ | Me | Cl |
| B-1396 | 1 | H | CH₂CH₂CH(Me) | Me | CH₂CF₃ | Me | Cl |
| B-1397 | 1 | Me | CH₂CH₂CH(Me) | H | CH₂CF₃ | Me | Cl |
| B-1398 | 1 | Me | CH₂CH₂CH(Me) | Me | CH₂CF₃ | Me | Cl |
| B-1399 | 1 | H | CH₂CH(Me)CH₂ | H | CH₂CF₃ | Me | Cl |
| B-1400 | 1 | H | CH₂CH(Me)CH₂ | Me | CH₂CF₃ | Me | Cl |
| B-1401 | 1 | Me | CH₂CH(Me)CH₂ | H | CH₂CF₃ | Me | Cl |
| B-1402 | 1 | Me | CH₂CH(Me)CH₂ | Me | CH₂CF₃ | Me | Cl |

**[Table 196]**

| Compound Number | n | Y | W | Z | R¹ | R² | R³ |
|---|---|---|---|---|---|---|---|
| B-1403 | 1 | H | CH(Me)CH₂CH₂ | H | CH₂CF₃ | Me | Cl |
| B-1404 | 1 | H | CH(Me)CH₂CH₂ | Me | CH₂CF₃ | Me | Cl |
| B-1405 | 1 | Me | CH(Me)CH₂CH₂ | H | CH₂CF₃ | Me | Cl |
| B-1406 | 1 | Me | CH(Me)CH₂CH₂ | Me | CH₂CF₃ | Me | Cl |
| B-1407 | 1 | H | single bond | Me | CH₂CF₃ | Me | Me |
| B-1408 | 1 | H | single bond | Et | CH₂CF₃ | Me | Me |
| B-1409 | 1 | Me | single bond | H | CH₂CF₃ | Me | Me |
| B-1410 | 1 | Et | single bond | H | CH₂CF₃ | Me | Me |
| B-1411 | 1 | Me | single bond | Me | CH₂CF₃ | Me | Me |
| B-1412 | 1 | H | CH₂ | Me | CH₂CF₃ | Me | Me |
| B-1413 | 1 | Me | CH₂ | H | CH₂CF₃ | Me | Me |
| B-1414 | 1 | Me | CH₂ | Me | CH₂CF₃ | Me | Me |
| B-1415 | 1 | H | CH(Me) | H | CH₂CF₃ | Me | Me |
| B-1416 | 1 | H | CH(Me) | Me | CH₂CF₃ | Me | Me |
| B-1417 | 1 | Me | CH(Me) | H | CH₂CF₃ | Me | Me |
| B-1418 | 1 | Me | CH(Me) | Me | CH₂CF₃ | Me | Me |
| B-1419 | 1 | H | CH₂CH₂ | Me | CH₂CF₃ | Me | Me |
| B-1420 | 1 | Me | CH₂CH₂ | H | CH₂CF₃ | Me | Me |
| B-1421 | 1 | Me | CH₂CH₂ | Me | CH₂CF₃ | Me | Me |
| B-1422 | 1 | H | CH₂CH(Me) | H | CH₂CF₃ | Me | Me |
| B-1423 | 1 | H | CH₂CH(Me) | Me | CH₂CF₃ | Me | Me |
| B-1424 | 1 | Me | CH₂CH(Me) | H | CH₂CF₃ | Me | Me |
| B-1425 | 1 | Me | CH₂CH(Me) | Me | CH₂CF₃ | Me | Me |
| B-1426 | 1 | H | CH(Me)CH₂ | H | CH₂CF₃ | Me | Me |
| B-1427 | 1 | H | CH(Me)CH₂ | Me | CH₂CF₃ | Me | Me |
| B-1428 | 1 | Me | CH(Me)CH₂ | H | CH₂CF₃ | Me | Me |
| B-1429 | 1 | Me | CH(Me)CH₂ | Me | CH₂CF₃ | Me | Me |
| B-1430 | 1 | H | CH₂CH₂CH₂ | Me | CH₂CF₃ | Me | Me |
| B-1431 | 1 | Me | CH₂CH₂CH₂ | H | CH₂CF₃ | Me | Me |
| B-1432 | 1 | Me | CH₂CH₂CH₂ | Me | CH₂CF₃ | Me | Me |
| B-1433 | 1 | H | CH₂CH₂CH(Me) | H | CH₂CF₃ | Me | Me |
| B-1434 | 1 | H | CH₂CH₂CH(Me) | Me | CH₂CF₃ | Me | Me |
| B-1435 | 1 | Me | CH₂CH₂CH(Me) | H | CH₂CF₃ | Me | Me |
| B-1436 | 1 | Me | CH₂CH₂CH(Me) | Me | CH₂CF₃ | Me | Me |
| B-1437 | 1 | H | CH₂CH(Me)CH₂ | H | CH₂CF₃ | Me | Me |
| B-1438 | 1 | H | CH₂CH(Me)CH₂ | Me | CH₂CF₃ | Me | Me |
| B-1439 | 1 | Me | CH₂CH(Me)CH₂ | H | CH₂CF₃ | Me | Me |
| B-1440 | 1 | Me | CH₂CH(Me)CH₂ | Me | CH₂CF₃ | Me | Me |
| B-1441 | 1 | H | CH(Me)CH₂CH₂ | H | CH₂CF₃ | Me | Me |
| B-1442 | 1 | H | CH(Me)CH₂CH₂ | Me | CH₂CF₃ | Me | Me |
| B-1443 | 1 | Me | CH(Me)CH₂CH₂ | H | CH₂CF₃ | Me | Me |
| B-1444 | 1 | Me | CH(Me)CH₂CH₂ | Me | CH₂CF₃ | Me | Me |
| B-1445 | 1 | H | single bond | Me | CH₂CF₃ | Et | Cl |
| B-1446 | 1 | H | single bond | Et | CH₂CF₃ | Et | Cl |

**[Table 197]**

| Compound Number | n | Y | W | Z | R¹ | R² | R³ |
|---|---|---|---|---|---|---|---|
| B-1447 | 1 | Me | single bond | H | CH₂CF₃ | Et | Cl |
| B-1448 | 1 | Et | single bond | H | CH₂CF₃ | Et | Cl |
| B-1449 | 1 | Me | single bond | Me | CH₂CF₃ | Et | Cl |
| B-1450 | 1 | H | CH₂ | Me | CH₂CF₃ | Et | Cl |
| B-1451 | 1 | Me | CH₂ | H | CH₂CF₃ | Et | Cl |
| B-1452 | 1 | Me | CH₂ | Me | CH₂CF₃ | Et | Cl |
| B-1453 | 1 | H | CH(Me) | H | CH₂CF₃ | Et | Cl |
| B-1454 | 1 | H | CH(Me) | Me | CH₂CF₃ | Et | Cl |
| B-1455 | 1 | Me | CH(Me) | H | CH₂CF₃ | Et | Cl |
| B-1456 | 1 | Me | CH(Me) | Me | CH₂CF₃ | Et | Cl |
| B-1457 | 1 | H | CH₂CH₂ | Me | CH₂CF₃ | Et | Cl |
| B-1458 | 1 | Me | CH₂CH₂ | H | CH₂CF₃ | Et | Cl |
| B-1459 | 1 | Me | CH₂CH₂ | Me | CH₂CF₃ | Et | Cl |
| B-1460 | 1 | H | CH₂CH(Me) | H | CH₂CF₃ | Et | Cl |
| B-1461 | 1 | H | CH₂CH(Me) | Me | CH₂CF₃ | Et | Cl |
| B-1462 | 1 | Me | CH₂CH(Me) | H | CH₂CF₃ | Et | Cl |
| B-1463 | 1 | Me | CH₂CH(Me) | Me | CH₂CF₃ | Et | Cl |
| B-1464 | 1 | H | CH(Me)CH₂ | H | CH₂CF₃ | Et | Cl |
| B-1465 | 1 | H | CH(Me)CH₂ | Me | CH₂CF₃ | Et | Cl |
| B-1466 | 1 | Me | CH(Me)CH₂ | H | CH₂CF₃ | Et | Cl |
| B-1467 | 1 | Me | CH(Me)CH₂ | Me | CH₂CF₃ | Et | Cl |
| B-1468 | 1 | H | CH₂CH₂CH₂ | Me | CH₂CF₃ | Et | Cl |
| B-1469 | 1 | Me | CH₂CH₂CH₂ | H | CH₂CF₃ | Et | Cl |
| B-1470 | 1 | Me | CH₂CH₂CH₂ | Me | CH₂CF₃ | Et | Cl |
| B-1471 | 1 | H | CH₂CH₂CH(Me) | H | CH₂CF₃ | Et | Cl |
| B-1472 | 1 | H | CH₂CH₂CH(Me) | Me | CH₂CF₃ | Et | Cl |
| B-1473 | 1 | Me | CH₂CH₂CH(Me) | H | CH₂CF₃ | Et | Cl |
| B-1474 | 1 | Me | CH₂CH₂CH(Me) | Me | CH₂CF₃ | Et | Cl |
| B-1475 | 1 | H | CH₂CH(Me)CH₂ | H | CH₂CF₃ | Et | Cl |
| B-1476 | 1 | H | CH₂CH(Me)CH₂ | Me | CH₂CF₃ | Et | Cl |
| B-1477 | 1 | Me | CH₂CH(Me)CH₂ | H | CH₂CF₃ | Et | Cl |
| B-1478 | 1 | Me | CH₂CH(Me)CH₂ | Me | CH₂CF₃ | Et | Cl |
| B-1479 | 1 | H | CH(Me)CH₂CH₂ | H | CH₂CF₃ | Et | Cl |
| B-1480 | 1 | H | CH(Me)CH₂CH₂ | Me | CH₂CF₃ | Et | Cl |
| B-1481 | 1 | Me | CH(Me)CH₂CH₂ | H | CH₂CF₃ | Et | Cl |
| B-1482 | 1 | Me | CH(Me)CH₂CH₂ | Me | CH₂CF₃ | Et | Cl |
| B-1483 | 1 | H | single bond | Me | CH₂CF₃ | Et | Me |
| B-1484 | 1 | H | single bond | Et | CH₂CF₃ | Et | Me |
| B-1485 | 1 | Me | single bond | H | CH₂CF₃ | Et | Me |
| B-1486 | 1 | Et | single bond | H | CH₂CF₃ | Et | Me |
| B-1487 | 1 | Me | single bond | Me | CH₂CF₃ | Et | Me |
| B-1488 | 1 | H | CH₂ | Me | CH₂CF₃ | Et | Me |
| B-1489 | 1 | Me | CH₂ | H | CH₂CF₃ | Et | Me |
| B-1490 | 1 | Me | CH₂ | Me | CH₂CF₃ | Et | Me |

**[Table 198]**

| Compound Number | n | Y | W | Z | R¹ | R² | R³ |
|---|---|---|---|---|---|---|---|
| B-1491 | 1 | H | CH(Me) | H | CH₂CF₃ | Et | Me |
| B-1492 | 1 | H | CH(Me) | Me | CH₂CF₃ | Et | Me |
| B-1493 | 1 | Me | CH(Me) | H | CH₂CF₃ | Et | Me |
| B-1494 | 1 | Me | CH(Me) | Me | CH₂CF₃ | Et | Me |
| B-1495 | 1 | H | CH₂CH₂ | Me | CH₂CF₃ | Et | Me |
| B-1496 | 1 | Me | CH₂CH₂ | H | CH₂CF₃ | Et | Me |
| B-1497 | 1 | Me | CH₂CH₂ | Me | CH₂CF₃ | Et | Me |
| B-1498 | 1 | H | CH₂CH(Me) | H | CH₂CF₃ | Et | Me |
| B-1499 | 1 | H | CH₂CH(Me) | Me | CH₂CF₃ | Et | Me |
| B-1500 | 1 | Me | CH₂CH(Me) | H | CH₂CF₃ | Et | Me |
| B-1501 | 1 | Me | CH₂CH(Me) | Me | CH₂CF₃ | Et | Me |
| B-1502 | 1 | H | CH(Me)CH₂ | H | CH₂CF₃ | Et | Me |
| B-1503 | 1 | H | CH(Me)CH₂ | Me | CH₂CF₃ | Et | Me |
| B-1504 | 1 | Me | CH(Me)CH₂ | H | CH₂CF₃ | Et | Me |
| B-1505 | 1 | Me | CH(Me)CH₂ | Me | CH₂CF₃ | Et | Me |
| B-1506 | 1 | H | CH₂CH₂CH₂ | Me | CH₂CF₃ | Et | Me |
| B-1507 | 1 | Me | CH₂CH₂CH₂ | H | CH₂CF₃ | Et | Me |
| B-1508 | 1 | Me | CH₂CH₂CH₂ | Me | CH₂CF₃ | Et | Me |
| B-1509 | 1 | H | CH₂CH₂CH(Me) | H | CH₂CF₃ | Et | Me |
| B-1510 | 1 | H | CH₂CH₂CH(Me) | Me | CH₂CF₃ | Et | Me |
| B-1511 | 1 | Me | CH₂CH₂CH(Me) | H | CH₂CF₃ | Et | Me |
| B-1512 | 1 | Me | CH₂CH₂CH(Me) | Me | CH₂CF₃ | Et | Me |
| B-1513 | 1 | H | CH₂CH(Me)CH₂ | H | CH₂CF₃ | Et | Me |
| B-1514 | 1 | H | CH₂CH(Me)CH₂ | Me | CH₂CF₃ | Et | Me |
| B-1515 | 1 | Me | CH₂CH(Me)CH₂ | H | CH₂CF₃ | Et | Me |
| B-1516 | 1 | Me | CH₂CH(Me)CH₂ | Me | CH₂CF₃ | Et | Me |
| B-1517 | 1 | H | CH(Me)CH₂CH₂ | H | CH₂CF₃ | Et | Me |
| B-1518 | 1 | H | CH(Me)CH₂CH₂ | Me | CH₂CF₃ | Et | Me |
| B-1519 | 1 | Me | CH(Me)CH₂CH₂ | H | CH₂CF₃ | Et | Me |
| B-1520 | 1 | Me | CH(Me)CH₂CH₂ | Me | CH₂CF₃ | Et | Me |
| B-1521 | 1 | H | single bond | Me | CH₂CF₃ | CH₂OMe | Cl |
| B-1522 | 1 | H | single bond | Et | CH₂CF₃ | CH₂OMe | Cl |
| B-1523 | 1 | Me | single bond | H | CH₂CF₃ | CH₂OMe | Cl |
| B-1524 | 1 | Et | single bond | H | CH₂CF₃ | CH₂OMe | Cl |
| B-1525 | 1 | Me | single bond | Me | CH₂CF₃ | CH₂OMe | Cl |
| B-1526 | 1 | H | CH₂ | Me | CH₂CF₃ | CH₂OMe | Cl |
| B-1527 | 1 | Me | CH₂ | H | CH₂CF₃ | CH₂OMe | Cl |
| B-1528 | 1 | Me | CH₂ | Me | CH₂CF₃ | CH₂OMe | Cl |
| B-1529 | 1 | H | CH(Me) | H | CH₂CF₃ | CH₂OMe | Cl |
| B-1530 | 1 | H | CH(Me) | Me | CH₂CF₃ | CH₂OMe | Cl |
| B-1531 | 1 | Me | CH(Me) | H | CH₂CF₃ | CH₂OMe | Cl |
| B-1532 | 1 | Me | CH(Me) | Me | CH₂CF₃ | CH₂OMe | Cl |
| B-1533 | 1 | H | CH₂CH₂ | Me | CH₂CF₃ | CH₂OMe | Cl |
| B-1534 | 1 | Me | CH₂CH₂ | H | CH₂CF₃ | CH₂OMe | Cl |

**[Table 199]**

| Compound Number | n | Y | W | Z | R¹ | R² | R³ |
|---|---|---|---|---|---|---|---|
| B-1535 | 1 | Me | CH₂CH₂ | Me | CH₂CF₃ | CH₂OMe | Cl |
| B-1536 | 1 | H | CH₂CH(Me) | H | CH₂CF₃ | CH₂OMe | Cl |
| B-1537 | 1 | H | CH₂CH(Me) | Me | CH₂CF₃ | CH₂OMe | Cl |
| B-1538 | 1 | Me | CH₂CH(Me) | H | CH₂CF₃ | CH₂OMe | Cl |
| B-1539 | 1 | Me | CH₂CH(Me) | Me | CH₂CF₃ | CH₂OMe | Cl |
| B-1540 | 1 | H | CH(Me)CH₂ | H | CH₂CF₃ | CH₂OMe | Cl |
| B-1541 | 1 | H | CH(Me)CH₂ | Me | CH₂CF₃ | CH₂OMe | Cl |
| B-1542 | 1 | Me | CH(Me)CH₂ | H | CH₂CF₃ | CH₂OMe | Cl |
| B-1543 | 1 | Me | CH(Me)CH₂ | Me | CH₂CF₃ | CH₂OMe | Cl |
| B-1544 | 1 | H | CH₂CH₂CH₂ | Me | CH₂CF₃ | CH₂OMe | Cl |
| B-1545 | 1 | Me | CH₂CH₂CH₂ | H | CH₂CF₃ | CH₂OMe | Cl |
| B-1546 | 1 | Me | CH₂CH₂CH₂ | Me | CH₂CF₃ | CH₂OMe | Cl |
| B-1547 | 1 | H | CH₂CH₂CH(Me) | H | CH₂CF₃ | CH₂OMe | Cl |
| B-1548 | 1 | H | CH₂CH₂CH(Me) | Me | CH₂CF₃ | CH₂OMe | Cl |
| B-1549 | 1 | Me | CH₂CH₂CH(Me) | H | CH₂CF₃ | CH₂OMe | Cl |
| B-1550 | 1 | Me | CH₂CH₂CH(Me) | Me | CH₂CF₃ | CH₂OMe | Cl |
| B-1551 | 1 | H | CH₂CH(Me)CH₂ | H | CH₂CF₃ | CH₂OMe | Cl |
| B-1552 | 1 | H | CH₂CH(Me)CH₂ | Me | CH₂CF₃ | CH₂OMe | Cl |
| B-1553 | 1 | Me | CH₂CH(Me)CH₂ | H | CH₂CF₃ | CH₂OMe | Cl |
| B-1554 | 1 | Me | CH₂CH(Me)CH₂ | Me | CH₂CF₃ | CH₂OMe | Cl |
| B-1555 | 1 | H | CH(Me)CH₂CH₂ | H | CH₂CF₃ | CH₂OMe | Cl |
| B-1556 | 1 | H | CH(Me)CH₂CH₂ | Me | CH₂CF₃ | CH₂OMe | Cl |
| B-1557 | 1 | Me | CH(Me)CH₂CH₂ | H | CH₂CF₃ | CH₂OMe | Cl |
| B-1558 | 1 | Me | CH(Me)CH₂CH₂ | Me | CH₂CF₃ | CH₂OMe | Cl |
| B-1559 | 1 | H | single bond | Me | CH₂CF₃ | CH₂OMe | Me |
| B-1560 | 1 | H | single bond | Et | CH₂CF₃ | CH₂OMe | Me |
| B-1561 | 1 | Me | single bond | H | CH₂CF₃ | CH₂OMe | Me |
| B-1562 | 1 | Et | single bond | H | CH₂CF₃ | CH₂OMe | Me |
| B-1563 | 1 | Me | single bond | Me | CH₂CF₃ | CH₂OMe | Me |
| B-1564 | 1 | H | CH₂ | Me | CH₂CF₃ | CH₂OMe | Me |
| B-1565 | 1 | Me | CH₂ | H | CH₂CF₃ | CH₂OMe | Me |
| B-1566 | 1 | Me | CH₂ | Me | CH₂CF₃ | CH₂OMe | Me |
| B-1567 | 1 | H | CH(Me) | H | CH₂CF₃ | CH₂OMe | Me |
| B-1568 | 1 | H | CH(Me) | Me | CH₂CF₃ | CH₂OMe | Me |
| B-1569 | 1 | Me | CH(Me) | H | CH₂CF₃ | CH₂OMe | Me |
| B-1570 | 1 | Me | CH(Me) | Me | CH₂CF₃ | CH₂OMe | Me |
| B-1571 | 1 | H | CH₂CH₂ | Me | CH₂CF₃ | CH₂OMe | Me |
| B-1572 | 1 | Me | CH₂CH₂ | H | CH₂CF₃ | CH₂OMe | Me |
| B-1573 | 1 | Me | CH₂CH₂ | Me | CH₂CF₃ | CH₂OMe | Me |
| B-1574 | 1 | H | CH₂CH(Me) | H | CH₂CF₃ | CH₂OMe | Me |
| B-1575 | 1 | H | CH₂CH(Me) | Me | CH₂CF₃ | CH₂OMe | Me |
| B-1576 | 1 | Me | CH₂CH(Me) | H | CH₂CF₃ | CH₂OMe | Me |
| B-1577 | 1 | Me | CH₂CH(Me) | Me | CH₂CF₃ | CH₂OMe | Me |
| B-1578 | 1 | H | CH(Me)CH₂ | H | CH₂CF₃ | CH₂OMe | Me |

**[Table 200]**

| Compound Number | n | Y | W | Z | R¹ | R² | R³ |
|---|---|---|---|---|---|---|---|
| B-1579 | 1 | H | CH(Me)CH₂ | Me | CH₂CF₃ | CH₂OMe | Me |
| B-1580 | 1 | Me | CH(Me)CH₂ | H | CH₂CF₃ | CH₂OMe | Me |
| B-1581 | 1 | Me | CH(Me)CH₂ | Me | CH₂CF₃ | CH₂OMe | Me |
| B-1582 | 1 | H | CH₂CH₂CH₂ | Me | CH₂CF₃ | CH₂OMe | Me |
| B-1583 | 1 | Me | CH₂CH₂CH₂ | H | CH₂CF₃ | CH₂OMe | Me |
| B-1584 | 1 | Me | CH₂CH₂CH₂ | Me | CH₂CF₃ | CH₂OMe | Me |
| B-1585 | 1 | H | CH₂CH₂CH(Me) | H | CH₂CF₃ | CH₂OMe | Me |
| B-1586 | 1 | H | CH₂CH₂CH(Me) | Me | CH₂CF₃ | CH₂OMe | Me |
| B-1587 | 1 | Me | CH₂CH₂CH(Me) | H | CH₂CF₃ | CH₂OMe | Me |
| B-1588 | 1 | Me | CH₂CH₂CH(Me) | Me | CH₂CF₃ | CH₂OMe | Me |
| B-1589 | 1 | H | CH₂CH(Me)CH₂ | H | CH₂CF₃ | CH₂OMe | Me |
| B-1590 | 1 | H | CH₂CH(Me)CH₂ | Me | CH₂CF₃ | CH₂OMe | Me |
| B-1591 | 1 | Me | CH₂CH(Me)CH₂ | H | CH₂CF₃ | CH₂OMe | Me |
| B-1592 | 1 | Me | CH₂CH(Me)CH₂ | Me | CH₂CF₃ | CH₂OMe | Me |
| B-1593 | 1 | H | CH(Me)CH₂CH₂ | H | CH₂CF₃ | CH₂OMe | Me |
| B-1594 | 1 | H | CH(Me)CH₂CH₂ | Me | CH₂CF₃ | CH₂OMe | Me |
| B-1595 | 1 | Me | CH(Me)CH₂CH₂ | H | CH₂CF₃ | CH₂OMe | Me |
| B-1596 | 1 | Me | CH(Me)CH₂CH₂ | Me | CH₂CF₃ | CH₂OMe | Me |
| B-1597 | 1 | H | single bond | Me | CH₂CF₃ | CH₂CN | Cl |
| B-1598 | 1 | H | single bond | Et | CH₂CF₃ | CH₂CN | Cl |
| B-1599 | 1 | Me | single bond | H | CH₂CF₃ | CH₂CN | Cl |
| B-1600 | 1 | Et | single bond | H | CH₂CF₃ | CH₂CN | Cl |
| B-1601 | 1 | Me | single bond | Me | CH₂CF₃ | CH₂CN | Cl |
| B-1602 | 1 | H | CH₂ | Me | CH₂CF₃ | CH₂CN | Cl |
| B-1603 | 1 | Me | CH₂ | H | CH₂CF₃ | CH₂CN | Cl |
| B-1604 | 1 | Me | CH₂ | Me | CH₂CF₃ | CH₂CN | Cl |
| B-1605 | 1 | H | CH(Me) | H | CH₂CF₃ | CH₂CN | Cl |
| B-1606 | 1 | H | CH(Me) | Me | CH₂CF₃ | CH₂CN | Cl |
| B-1607 | 1 | Me | CH(Me) | H | CH₂CF₃ | CH₂CN | Cl |
| B-1608 | 1 | Me | CH(Me) | Me | CH₂CF₃ | CH₂CN | Cl |
| B-1609 | 1 | H | CH₂CH₂ | Me | CH₂CF₃ | CH₂CN | Cl |
| B-1610 | 1 | Me | CH₂CH₂ | H | CH₂CF₃ | CH₂CN | Cl |
| B-1611 | 1 | Me | CH₂CH₂ | Me | CH₂CF₃ | CH₂CN | Cl |
| B-1612 | 1 | H | CH₂CH(Me) | H | CH₂CF₃ | CH₂CN | Cl |
| B-1613 | 1 | H | CH₂CH(Me) | Me | CH₂CF₃ | CH₂CN | Cl |
| B-1614 | 1 | Me | CH₂CH(Me) | H | CH₂CF₃ | CH₂CN | Cl |
| B-1615 | 1 | Me | CH₂CH(Me) | Me | CH₂CF₃ | CH₂CN | Cl |
| B-1616 | 1 | H | CH(Me)CH₂ | H | CH₂CF₃ | CH₂CN | Cl |
| B-1617 | 1 | H | CH(Me)CH₂ | Me | CH₂CF₃ | CH₂CN | Cl |
| B-1618 | 1 | Me | CH(Me)CH₂ | H | CH₂CF₃ | CH₂CN | Cl |
| B-1619 | 1 | Me | CH(Me)CH₂ | Me | CH₂CF₃ | CH₂CN | Cl |
| B-1620 | 1 | H | CH₂CH₂CH₂ | Me | CH₂CF₃ | CH₂CN | Cl |
| B-1621 | 1 | Me | CH₂CH₂CH₂ | H | CH₂CF₃ | CH₂CN | Cl |
| B-1622 | 1 | Me | CH₂CH₂CH₂ | Me | CH₂CF₃ | CH₂CN | Cl |

**[Table 201]**

| Compound Number | n | Y | W | Z | R¹ | R² | R³ |
|---|---|---|---|---|---|---|---|
| B-1623 | 1 | H | CH₂CH₂CH(Me) | H | CH₂CF₃ | CH₂CN | Cl |
| B-1624 | 1 | H | CH₂CH₂CH(Me) | Me | CH₂CF₃ | CH₂CN | Cl |
| B-1625 | 1 | Me | CH₂CH₂CH(Me) | H | CH₂CF₃ | CH₂CN | Cl |
| B-1626 | 1 | Me | CH₂CH₂CH(Me) | Me | CH₂CF₃ | CH₂CN | Cl |
| B-1627 | 1 | H | CH₂CH(Me)CH₂ | H | CH₂CF₃ | CH₂CN | Cl |
| B-1628 | 1 | H | CH₂CH(Me)CH₂ | Me | CH₂CF₃ | CH₂CN | Cl |
| B-1629 | 1 | Me | CH₂CH(Me)CH₂ | H | CH₂CF₃ | CH₂CN | Cl |
| B-1630 | 1 | Me | CH₂CH(Me)CH₂ | Me | CH₂CF₃ | CH₂CN | Cl |
| B-1631 | 1 | H | CH(Me)CH₂CH₂ | H | CH₂CF₃ | CH₂CN | Cl |
| B-1632 | 1 | H | CH(Me)CH₂CH₂ | Me | CH₂CF₃ | CH₂CN | Cl |
| B-1633 | 1 | Me | CH(Me)CH₂CH₂ | H | CH₂CF₃ | CH₂CN | Cl |
| B-1634 | 1 | Me | CH(Me)CH₂CH₂ | Me | CH₂CF₃ | CH₂CN | Cl |
| B-1635 | 1 | H | single bond | Me | CH₂CF₃ | CH₂CN | Me |
| B-1636 | 1 | H | single bond | Et | CH₂CF₃ | CH₂CN | Me |
| B-1637 | 1 | Me | single bond | H | CH₂CF₃ | CH₂CN | Me |
| B-1638 | 1 | Et | single bond | H | CH₂CF₃ | CH₂CN | Me |
| B-1639 | 1 | Me | single bond | Me | CH₂CF₃ | CH₂CN | Me |
| B-1640 | 1 | H | CH₂ | Me | CH₂CF₃ | CH₂CN | Me |
| B-1641 | 1 | Me | CH₂ | H | CH₂CF₃ | CH₂CN | Me |
| B-1642 | 1 | Me | CH₂ | Me | CH₂CF₃ | CH₂CN | Me |
| B-1643 | 1 | H | CH(Me) | H | CH₂CF₃ | CH₂CN | Me |
| B-1644 | 1 | H | CH(Me) | Me | CH₂CF₃ | CH₂CN | Me |
| B-1645 | 1 | Me | CH(Me) | H | CH₂CF₃ | CH₂CN | Me |
| B-1646 | 1 | Me | CH(Me) | Me | CH₂CF₃ | CH₂CN | Me |
| B-1647 | 1 | H | CH₂CH₂ | Me | CH₂CF₃ | CH₂CN | Me |
| B-1648 | 1 | Me | CH₂CH₂ | H | CH₂CF₃ | CH₂CN | Me |
| B-1649 | 1 | Me | CH₂CH₂ | Me | CH₂CF₃ | CH₂CN | Me |
| B-1650 | 1 | H | CH₂CH(Me) | H | CH₂CF₃ | CH₂CN | Me |
| B-1651 | 1 | H | CH₂CH(Me) | Me | CH₂CF₃ | CH₂CN | Me |
| B-1652 | 1 | Me | CH₂CH(Me) | H | CH₂CF₃ | CH₂CN | Me |
| B-1653 | 1 | Me | CH₂CH(Me) | Me | CH₂CF₃ | CH₂CN | Me |
| B-1654 | 1 | H | CH(Me)CH₂ | H | CH₂CF₃ | CH₂CN | Me |
| B-1655 | 1 | H | CH(Me)CH₂ | Me | CH₂CF₃ | CH₂CN | Me |
| B-1656 | 1 | Me | CH(Me)CH₂ | H | CH₂CF₃ | CH₂CN | Me |
| B-1657 | 1 | Me | CH(Me)CH₂ | Me | CH₂CF₃ | CH₂CN | Me |
| B-1658 | 1 | H | CH₂CH₂CH₂ | Me | CH₂CF₃ | CH₂CN | Me |
| B-1659 | 1 | Me | CH₂CH₂CH₂ | H | CH₂CF₃ | CH₂CN | Me |
| B-1660 | 1 | Me | CH₂CH₂CH₂ | Me | CH₂CF₃ | CH₂CN | Me |
| B-1661 | 1 | H | CH₂CH₂CH(Me) | H | CH₂CF₃ | CH₂CN | Me |
| B-1662 | 1 | H | CH₂CH₂CH(Me) | Me | CH₂CF₃ | CH₂CN | Me |
| B-1663 | 1 | Me | CH₂CH₂CH(Me) | H | CH₂CF₃ | CH₂CN | Me |
| B-1664 | 1 | Me | CH₂CH₂CH(Me) | Me | CH₂CF₃ | CH₂CN | Me |
| B-1665 | 1 | H | CH₂CH(Me)CH₂ | H | CH₂CF₃ | CH₂CN | Me |
| B-1666 | 1 | H | CH₂CH(Me)CH₂ | Me | CH₂CF₃ | CH₂CN | Me |

**[Table 202]**

| Compound Number | n | Y | W | Z | R¹ | R² | R³ |
|---|---|---|---|---|---|---|---|
| B-1667 | 1 | Me | CH₂CH(Me)CH₂ | H | CH₂CF₃ | CH₂CN | Me |
| B-1668 | 1 | Me | CH₂CH(Me)CH₂ | Me | CH₂CF₃ | CH₂CN | Me |
| B-1669 | 1 | H | CH(Me)CH₂CH₂ | H | CH₂CF₃ | CH₂CN | Me |
| B-1670 | 1 | H | CH(Me)CH₂CH₂ | Me | CH₂CF₃ | CH₂CN | Me |
| B-1671 | 1 | Me | CH(Me)CH₂CH₂ | H | CH₂CF₃ | CH₂CN | Me |
| B-1672 | 1 | Me | CH(Me)CH₂CH₂ | Me | CH₂CF₃ | CH₂CN | Me |
| B-1673 | 1 | H | single bond | Me | CH₂CF₃ | C(=O)Me | Cl |
| B-1674 | 1 | H | single bond | Et | CH₂CF₃ | C(=O)Me | Cl |
| B-1675 | 1 | Me | single bond | H | CH₂CF₃ | C(=O)Me | Cl |
| B-1676 | 1 | Et | single bond | H | CH₂CF₃ | C(=O)Me | Cl |
| B-1677 | 1 | Me | single bond | Me | CH₂CF₃ | C(=O)Me | Cl |
| B-1678 | 1 | H | CH₂ | Me | CH₂CF₃ | C(=O)Me | Cl |
| B-1679 | 1 | Me | CH₂ | H | CH₂CF₃ | C(=O)Me | Cl |
| B-1680 | 1 | Me | CH₂ | Me | CH₂CF₃ | C(=O)Me | Cl |
| B-1681 | 1 | H | CH(Me) | H | CH₂CF₃ | C(=O)Me | Cl |
| B-1682 | 1 | H | CH(Me) | Me | CH₂CF₃ | C(=O)Me | Cl |
| B-1683 | 1 | Me | CH(Me) | H | CH₂CF₃ | C(=O)Me | Cl |
| B-1684 | 1 | Me | CH(Me) | Me | CH₂CF₃ | C(=O)Me | Cl |
| B-1685 | 1 | H | CH₂CH₂ | Me | CH₂CF₃ | C(=O)Me | Cl |
| B-1686 | 1 | Me | CH₂CH₂ | H | CH₂CF₃ | C(=O)Me | Cl |
| B-1687 | 1 | Me | CH₂CH₂ | Me | CH₂CF₃ | C(=O)Me | Cl |
| B-1688 | 1 | H | CH₂CH(Me) | H | CH₂CF₃ | C(=O)Me | Cl |
| B-1689 | 1 | H | CH₂CH(Me) | Me | CH₂CF₃ | C(=O)Me | Cl |
| B-1690 | 1 | Me | CH₂CH(Me) | H | CH₂CF₃ | C(=O)Me | Cl |
| B-1691 | 1 | Me | CH₂CH(Me) | Me | CH₂CF₃ | C(=O)Me | Cl |
| B-1692 | 1 | H | CH(Me)CH₂ | H | CH₂CF₃ | C(=O)Me | Cl |
| B-1693 | 1 | H | CH(Me)CH₂ | Me | CH₂CF₃ | C(=O)Me | Cl |
| B-1694 | 1 | Me | CH(Me)CH₂ | H | CH₂CF₃ | C(=O)Me | Cl |
| B-1695 | 1 | Me | CH(Me)CH₂ | Me | CH₂CF₃ | C(=O)Me | Cl |
| B-1696 | 1 | H | CH₂CH₂CH₂ | Me | CH₂CF₃ | C(=O)Me | Cl |
| B-1697 | 1 | Me | CH₂CH₂CH₂ | H | CH₂CF₃ | C(=O)Me | Cl |
| B-1698 | 1 | Me | CH₂CH₂CH₂ | Me | CH₂CF₃ | C(=O)Me | Cl |
| B-1699 | 1 | H | CH₂CH₂CH(Me) | H | CH₂CF₃ | C(=O)Me | Cl |
| B-1700 | 1 | H | CH₂CH₂CH(Me) | Me | CH₂CF₃ | C(=O)Me | Cl |
| B-1701 | 1 | Me | CH₂CH₂CH(Me) | H | CH₂CF₃ | C(=O)Me | Cl |
| B-1702 | 1 | Me | CH₂CH₂CH(Me) | Me | CH₂CF₃ | C(=O)Me | Cl |
| B-1703 | 1 | H | CH₂CH(Me)CH₂ | H | CH₂CF₃ | C(=O)Me | Cl |
| B-1704 | 1 | H | CH₂CH(Me)CH₂ | Me | CH₂CF₃ | C(=O)Me | Cl |
| B-1705 | 1 | Me | CH₂CH(Me)CH₂ | H | CH₂CF₃ | C(=O)Me | Cl |
| B-1706 | 1 | Me | CH₂CH(Me)CH₂ | Me | CH₂CF₃ | C(=O)Me | Cl |
| B-1707 | 1 | H | CH(Me)CH₂CH₂ | H | CH₂CF₃ | C(=O)Me | Cl |
| B-1708 | 1 | H | CH(Me)CH₂CH₂ | Me | CH₂CF₃ | C(=O)Me | Cl |
| B-1709 | 1 | Me | CH(Me)CH₂CH₂ | H | CH₂CF₃ | C(=O)Me | Cl |
| B-1 710 | 1 | Me | CH(Me)CH₂CH₂ | Me | CH₂CF₃ | C(=O)Me | Cl |

**[Table 203]**

| Compound Number | n | Y | W | Z | R¹ | R² | R³ |
|---|---|---|---|---|---|---|---|
| B-1711 | 1 | H | single bond | Me | CH₂CF₃ | C(=O)Me | Me |
| B-1712 | 1 | H | single bond | Et | CH₂CF₃ | C(=O)Me | Me |
| B-1 713 | 1 | Me | single bond | H | CH₂CF₃ | C(=O)Me | Me |
| B-1714 | 1 | Et | single bond | H | CH₂CF₃ | C(=O)Me | Me |
| B-1 715 | 1 | Me | single bond | Me | CH₂CF₃ | C(=O)Me | Me |
| B-1716 | 1 | H | CH₂ | Me | CH₂CF₃ | C(=O)Me | Me |
| B-1717 | 1 | Me | CH₂ | H | CH₂CF₃ | C(=O)Me | Me |
| B-1 718 | 1 | Me | CH₂ | Me | CH₂CF₃ | C(=O)Me | Me |
| B-1 719 | 1 | H | CH(Me) | H | CH₂CF₃ | C(=O)Me | Me |
| B-1720 | 1 | H | CH(Me) | Me | CH₂CF₃ | C(=O)Me | Me |
| B-1721 | 1 | Me | CH(Me) | H | CH₂CF₃ | C(=O)Me | Me |
| B-1722 | 1 | Me | CH(Me) | Me | CH₂CF₃ | C(=O)Me | Me |
| B-1723 | 1 | H | CH₂CH₂ | Me | CH₂CF₃ | C(=O)Me | Me |
| B-1724 | 1 | Me | CH₂CH₂ | H | CH₂CF₃ | C(=O)Me | Me |
| B-1725 | 1 | Me | CH₂CH₂ | Me | CH₂CF₃ | C(=O)Me | Me |
| B-1726 | 1 | H | CH₂CH(Me) | H | CH₂CF₃ | C(=O)Me | Me |
| B-1727 | 1 | H | CH₂CH(Me) | Me | CH₂CF₃ | C(=O)Me | Me |
| B-1728 | 1 | Me | CH₂CH(Me) | H | CH₂CF₃ | C(=O)Me | Me |
| B-1729 | 1 | Me | CH₂CH(Me) | Me | CH₂CF₃ | C(=O)Me | Me |
| B-1730 | 1 | H | CH(Me)CH₂ | H | CH₂CF₃ | C(=O)Me | Me |
| B-1731 | 1 | H | CH(Me)CH₂ | Me | CH₂CF₃ | C(=O)Me | Me |
| B-1732 | 1 | Me | CH(Me)CH₂ | H | CH₂CF₃ | C(=O)Me | Me |
| B-1733 | 1 | Me | CH(Me)CH₂ | Me | CH₂CF₃ | C(=O)Me | Me |
| B-1734 | 1 | H | CH₂CH₂CH₂ | Me | CH₂CF₃ | C(=O)Me | Me |
| B-1735 | 1 | Me | CH₂CH₂CH₂ | H | CH₂CF₃ | C(=O)Me | Me |
| B-1736 | 1 | Me | CH₂CH₂CH₂ | Me | CH₂CF₃ | C(=O)Me | Me |
| B-1737 | 1 | H | CH₂CH₂CH(Me) | H | CH₂CF₃ | C(=O)Me | Me |
| B-1738 | 1 | H | CH₂CH₂CH(Me) | Me | CH₂CF₃ | C(=O)Me | Me |
| B-1739 | 1 | Me | CH₂CH₂CH(Me) | H | CH₂CF₃ | C(=O)Me | Me |
| B-1740 | 1 | Me | CH₂CH₂CH(Me) | Me | CH₂CF₃ | C(=O)Me | Me |
| B-1741 | 1 | H | CH₂CH(Me)CH₂ | H | CH₂CF₃ | C(=O)Me | Me |
| B-1742 | 1 | H | CH₂CH(Me)CH₂ | Me | CH₂CF₃ | C(=O)Me | Me |
| B-1743 | 1 | Me | CH₂CH(Me)CH₂ | H | CH₂CF₃ | C(=O)Me | Me |
| B-1744 | 1 | Me | CH₂CH(Me)CH₂ | Me | CH₂CF₃ | C(=O)Me | Me |
| B-1745 | 1 | H | CH(Me)CH₂CH₂ | H | CH₂CF₃ | C(=O)Me | Me |
| B-1746 | 1 | H | CH(Me)CH₂CH₂ | Me | CH₂CF₃ | C(=O)Me | Me |
| B-1747 | 1 | Me | CH(Me)CH₂CH₂ | H | CH₂CF₃ | C(=O)Me | Me |
| B-1748 | 1 | Me | CH(Me)CH₂CH₂ | Me | CH₂CF₃ | C(=O)Me | Me |
| B-1749 | 1 | H | single bond | Me | CH₂CF₃ | C(=O)Ph | Cl |
| B-1750 | 1 | H | single bond | Et | CH₂CF₃ | C(=O)Ph | Cl |
| B-1751 | 1 | Me | single bond | H | CH₂CF₃ | C(=O)Ph | Cl |
| B-1752 | 1 | Et | single bond | H | CH₂CF₃ | C(=O)Ph | Cl |
| B-1753 | 1 | Me | single bond | Me | CH₂CF₃ | C(=O)Ph | Cl |
| B-1754 | 1 | H | CH₂ | Me | CH₂CF₃ | C(=O)Ph | Cl |

**[Table 204]**

| Compound Number | n | Y | W | Z | R¹ | R² | R³ |
|---|---|---|---|---|---|---|---|
| B-1755 | 1 | Me | CH₂ | H | CH₂CF₃ | C(=O)Ph | Cl |
| B-1756 | 1 | Me | CH₂ | Me | CH₂CF₃ | C(=O)Ph | Cl |
| B-1757 | 1 | H | CH(Me) | H | CH₂CF₃ | C(=O)Ph | Cl |
| B-1758 | 1 | H | CH(Me) | Me | CH₂CF₃ | C(=O)Ph | Cl |
| B-1759 | 1 | Me | CH(Me) | H | CH₂CF₃ | C(=O)Ph | Cl |
| B-1760 | 1 | Me | CH(Me) | Me | CH₂CF₃ | C(=O)Ph | Cl |
| B-1761 | 1 | H | CH₂CH₂ | Me | CH₂CF₃ | C(=O)Ph | Cl |
| B-1762 | 1 | Me | CH₂CH₂ | H | CH₂CF₃ | C(=O)Ph | Cl |
| B-1763 | 1 | Me | CH₂CH₂ | Me | CH₂CF₃ | C(=O)Ph | Cl |
| B-1764 | 1 | H | CH₂CH(Me) | H | CH₂CF₃ | C(=O)Ph | Cl |
| B-1765 | 1 | H | CH₂CH(Me) | Me | CH₂CF₃ | C(=O)Ph | Cl |
| B-1766 | 1 | Me | CH₂CH(Me) | H | CH₂CF₃ | C(=O)Ph | Cl |
| B-1767 | 1 | Me | CH₂CH(Me) | Me | CH₂CF₃ | C(=O)Ph | Cl |
| B-1768 | 1 | H | CH(Me)CH₂ | H | CH₂CF₃ | C(=O)Ph | Cl |
| B-1769 | 1 | H | CH(Me)CH₂ | Me | CH₂CF₃ | C(=O)Ph | Cl |
| B-1770 | 1 | Me | CH(Me)CH₂ | H | CH₂CF₃ | C(=O)Ph | Cl |
| B-1771 | 1 | Me | CH(Me)CH₂ | Me | CH₂CF₃ | C(=O)Ph | Cl |
| B-1772 | 1 | H | CH₂CH₂CH₂ | Me | CH₂CF₃ | C(=O)Ph | Cl |
| B-1773 | 1 | Me | CH₂CH₂CH₂ | H | CH₂CF₃ | C(=O)Ph | Cl |
| B-1774 | 1 | Me | CH₂CH₂CH₂ | Me | CH₂CF₃ | C(=O)Ph | Cl |
| B-1775 | 1 | H | CH₂CH₂CH(Me) | H | CH₂CF₃ | C(=O)Ph | Cl |
| B-1776 | 1 | H | CH₂CH₂CH(Me) | Me | CH₂CF₃ | C(=O)Ph | Cl |
| B-1777 | 1 | Me | CH₂CH₂CH(Me) | H | CH₂CF₃ | C(=O)Ph | Cl |
| B-1778 | 1 | Me | CH₂CH₂CH(Me) | Me | CH₂CF₃ | C(=O)Ph | Cl |
| B-1779 | 1 | H | CH₂CH(Me)CH₂ | H | CH₂CF₃ | C(=O)Ph | Cl |
| B-1780 | 1 | H | CH₂CH(Me)CH₂ | Me | CH₂CF₃ | C(=O)Ph | Cl |
| +B-1781 | 1 | Me | CH₂CH(Me)CH₂ | H | CH₂CF₃ | C(=O)Ph | Cl |
| B-1782 | 1 | Me | CH₂CH(Me)CH₂ | Me | CH₂CF₃ | C(=O)Ph | Cl |
| B-1783 | 1 | H | CH(Me)CH₂CH₂ | H | CH₂CF₃ | C(=O)Ph | Cl |
| B-1784 | 1 | H | CH(Me)CH₂CH₂ | Me | CH₂CF₃ | C(=O)Ph | Cl |
| B-1785 | 1 | Me | CH(Me)CH₂CH₂ | H | CH₂CF₃ | C(=O)Ph | Cl |
| B-1786 | 1 | Me | CH(Me)CH₂CH₂ | Me | CH₂CF₃ | C(=O)Ph | Cl |
| B-1787 | 1 | H | single bond | Me | CH₂CF₃ | C(=O)Ph | Me |
| B-1788 | 1 | H | single bond | Et | CH₂CF₃ | C(=O)Ph | Me |
| B-1789 | 1 | Me | single bond | H | CH₂CF₃ | C(=O)Ph | Me |
| B-1790 | 1 | Et | single bond | H | CH₂CF₃ | C(=O)Ph | Me |
| B-1791 | 1 | Me | single bond | Me | CH₂CF₃ | C(=O)Ph | Me |
| B-1792 | 1 | H | CH₂ | Me | CH₂CF₃ | C(=O)Ph | Me |
| B-1793 | 1 | Me | CH₂ | H | CH₂CF₃ | C(=O)Ph | Me |
| B-1794 | 1 | Me | CH₂ | Me | CH₂CF₃ | C(=O)Ph | Me |
| B-1795 | 1 | H | CH(Me) | H | CH₂CF₃ | C(=O)Ph | Me |
| B-1796 | 1 | H | CH(Me) | Me | CH₂CF₃ | C(=O)Ph | Me |
| B-1797 | 1 | Me | CH(Me) | H | CH₂CF₃ | C(=O)Ph | Me |
| B-1798 | 1 | Me | CH(Me) | Me | CH₂CF₃ | C(=O)Ph | Me |

**[Table 205]**

| Compound Number | n | Y | W | Z | R¹ | R² | R³ |
|---|---|---|---|---|---|---|---|
| B-1799 | 1 | H | CH₂CH₂ | Me | CH₂CF₃ | C(=O)Ph | Me |
| B-1800 | 1 | Me | CH₂CH₂ | H | CH₂CF₃ | C(=O)Ph | Me |
| B-1801 | 1 | Me | CH₂CH₂ | Me | CH₂CF₃ | C(=O)Ph | Me |
| B-1802 | 1 | H | CH₂CH(Me) | H | CH₂CF₃ | C(=O)Ph | Me |
| B-1803 | 1 | H | CH₂CH(Me) | Me | CH₂CF₃ | C(=O)Ph | Me |
| B-1804 | 1 | Me | CH₂CH(Me) | H | CH₂CF₃ | C(=O)Ph | Me |
| B-1805 | 1 | Me | CH₂CH(Me) | Me | CH₂CF₃ | C(=O)Ph | Me |
| B-1806 | 1 | H | CH(Me)CH₂ | H | CH₂CF₃ | C(=O)Ph | Me |
| B-1807 | 1 | H | CH(Me)CH₂ | Me | CH₂CF₃ | C(=O)Ph | Me |
| B-1808 | 1 | Me | CH(Me)CH₂ | H | CH₂CF₃ | C(=O)Ph | Me |
| B-1809 | 1 | Me | CH(Me)CH₂ | Me | CH₂CF₃ | C(=O)Ph | Me |
| B-1810 | 1 | H | CH₂CH₂CH₂ | Me | CH₂CF₃ | C(=O)Ph | Me |
| B-1811 | 1 | Me | CH₂CH₂CH₂ | H | CH₂CF₃ | C(=O)Ph | Me |
| B-1812 | 1 | Me | CH₂CH₂CH₂ | Me | CH₂CF₃ | C(=O)Ph | Me |
| B-1813 | 1 | H | CH₂CH₂CH(Me) | H | CH₂CF₃ | C(=O)Ph | Me |
| B-1814 | 1 | H | CH₂CH₂CH(Me) | Me | CH₂CF₃ | C(=O)Ph | Me |
| B-1815 | 1 | Me | CH₂CH₂CH(Me) | H | CH₂CF₃ | C(=O)Ph | Me |
| B-1816 | 1 | Me | CH₂CH₂CH(Me) | Me | CH₂CF₃ | C(=O)Ph | Me |
| B-1817 | 1 | H | CH₂CH(Me)CH₂ | H | CH₂CF₃ | C(=O)Ph | Me |
| B-1818 | 1 | H | CH₂CH(Me)CH₂ | Me | CH₂CF₃ | C(=O)Ph | Me |
| B-1819 | 1 | Me | CH₂CH(Me)CH₂ | H | CH₂CF₃ | C(=O)Ph | Me |
| B-1820 | 1 | Me | CH₂CH(Me)CH₂ | Me | CH₂CF₃ | C(=O)Ph | Me |
| B-1821 | 1 | H | CH(Me)CH₂CH₂ | H | CH₂CF₃ | C(=O)Ph | Me |
| B-1822 | 1 | H | CH(Me)CH₂CH₂ | Me | CH₂CF₃ | C(=O)Ph | Me |
| B-1823 | 1 | Me | CH(Me)CH₂CH₂ | H | CH₂CF₃ | C(=O)Ph | Me |
| B-1824 | 1 | Me | CH(Me)CH₂CH₂ | Me | CH₂CF₃ | C(=O)Ph | Me |
| B-1825 | 1 | H | single bond | Me | CH₂CF₃ | C(=O)OEt | Cl |
| B-1826 | 1 | H | single bond | Et | CH₂CF₃ | C(=O)OEt | Cl |
| B-1827 | 1 | Me | single bond | H | CH₂CF₃ | C(=O)OEt | Cl |
| B-1828 | 1 | Et | single bond | H | CH₂CF₃ | C(=O)OEt | Cl |
| B-1829 | 1 | Me | single bond | Me | CH₂CF₃ | C(=O)OEt | Cl |
| B-1830 | 1 | H | CH₂ | Me | CH₂CF₃ | C(=O)OEt | Cl |
| B-1831 | 1 | Me | CH₂ | H | CH₂CF₃ | C(=O)OEt | Cl |
| B-1832 | 1 | Me | CH₂ | Me | CH₂CF₃ | C(=O)OEt | Cl |
| B-1833 | 1 | H | CH(Me) | H | CH₂CF₃ | C(=O)OEt | Cl |
| B-1834 | 1 | H | CH(Me) | Me | CH₂CF₃ | C(=O)OEt | Cl |
| B-1835 | 1 | Me | CH(Me) | H | CH₂CF₃ | C(=O)OEt | Cl |
| B-1836 | 1 | Me | CH(Me) | Me | CH₂CF₃ | C(=O)OEt | Cl |
| B-1837 | 1 | H | CH₂CH₂ | Me | CH₂CF₃ | C(=O)OEt | Cl |
| B-1838 | 1 | Me | CH₂CH₂ | H | CH₂CF₃ | C(=O)OEt | Cl |
| B-1839 | 1 | Me | CH₂CH₂ | Me | CH₂CF₃ | C(=O)OEt | Cl |
| B-1840 | 1 | H | CH₂CH(Me) | H | CH₂CF₃ | C(=O)OEt | Cl |
| B-1841 | 1 | H | CH₂CH(Me) | Me | CH₂CF₃ | C(=O)OEt | Cl |
| B-1842 | 1 | Me | CH₂CH(Me) | H | CH₂CF₃ | C(=O)OEt | Cl |

**[Table 206]**

| Compound Number | n | Y | W | Z | R¹ | R² | R³ |
|---|---|---|---|---|---|---|---|
| B-1843 | 1 | Me | CH₂CH(Me) | Me | CH₂CF₃ | C(=O)OEt | Cl |
| B-1844 | 1 | H | CH(Me)CH₂ | H | CH₂CF₃ | C(=O)OEt | Cl |
| B-1845 | 1 | H | CH(Me)CH₂ | Me | CH₂CF₃ | C(=O)OEt | Cl |
| B-1846 | 1 | Me | CH(Me)CH₂ | H | CH₂CF₃ | C(=O)OEt | Cl |
| B-1847 | 1 | Me | CH(Me)CH₂ | Me | CH₂CF₃ | C(=O)OEt | Cl |
| B-1848 | 1 | H | CH₂CH₂CH₂ | Me | CH₂CF₃ | C(=O)OEt | Cl |
| B-1849 | 1 | Me | CH₂CH₂CH₂ | H | CH₂CF₃ | C(=O)OEt | Cl |
| B-1850 | 1 | Me | CH₂CH₂CH₂ | Me | CH₂CF₃ | C(=O)OEt | Cl |
| B-1851 | 1 | H | CH₂CH₂CH(Me) | H | CH₂CF₃ | C(=O)OEt | Cl |
| B-1852 | 1 | H | CH₂CH₂CH(Me) | Me | CH₂CF₃ | C(=O)OEt | Cl |
| B-1853 | 1 | Me | CH₂CH₂CH(Me) | H | CH₂CF₃ | C(=O)OEt | Cl |
| B-1854 | 1 | Me | CH₂CH₂CH(Me) | Me | CH₂CF₃ | C(=O)OEt | Cl |
| B-1855 | 1 | H | CH₂CH(Me)CH₂ | H | CH₂CF₃ | C(=O)OEt | Cl |
| B-1856 | 1 | H | CH₂CH(Me)CH₂ | Me | CH₂CF₃ | C(=O)OEt | Cl |
| B-1857 | 1 | Me | CH₂CH(Me)CH₂ | H | CH₂CF₃ | C(=O)OEt | Cl |
| B-1858 | 1 | Me | CH₂CH(Me)CH₂ | Me | CH₂CF₃ | C(=O)OEt | Cl |
| B-1859 | 1 | H | CH(Me)CH₂CH₂ | H | CH₂CF₃ | C(=O)OEt | Cl |
| B-1860 | 1 | H | CH(Me)CH₂CH₂ | Me | CH₂CF₃ | C(=O)OEt | Cl |
| B-1861 | 1 | Me | CH(Me)CH₂CH₂ | H | CH₂CF₃ | C(=O)OEt | Cl |
| B-1862 | 1 | Me | CH(Me)CH₂CH₂ | Me | CH₂CF₃ | C(=O)OEt | Cl |
| B-1863 | 1 | H | single bond | Me | CH₂CF₃ | C(=O)OEt | Me |
| B-1864 | 1 | H | single bond | Et | CH₂CF₃ | C(=O)OEt | Me |
| B-1865 | 1 | Me | single bond | H | CH₂CF₃ | C(=O)OEt | Me |
| B-1866 | 1 | Et | single bond | H | CH₂CF₃ | C(=O)OEt | Me |
| B-1867 | 1 | Me | single bond | Me | CH₂CF₃ | C(=O)OEt | Me |
| B-1868 | 1 | H | CH₂ | Me | CH₂CF₃ | C(=O)OEt | Me |
| B-1869 | 1 | Me | CH₂ | H | CH₂CF₃ | C(=O)OEt | Me |
| B-1870 | 1 | Me | CH₂ | Me | CH₂CF₃ | C(=O)OEt | Me |
| B-1871 | 1 | H | CH(Me) | H | CH₂CF₃ | C(=O)OEt | Me |
| B-1872 | 1 | H | CH(Me) | Me | CH₂CF₃ | C(=O)OEt | Me |
| B-1873 | 1 | Me | CH(Me) | H | CH₂CF₃ | C(=O)OEt | Me |
| B-1874 | 1 | Me | CH(Me) | Me | CH₂CF₃ | C(=O)OEt | Me |
| B-1875 | 1 | H | CH₂CH₂ | Me | CH₂CF₃ | C(=O)OEt | Me |
| B-1876 | 1 | Me | CH₂CH₂ | H | CH₂CF₃ | C(=O)OEt | Me |
| B-1877 | 1 | Me | CH₂CH₂ | Me | CH₂CF₃ | C(=O)OEt | Me |
| B-1878 | 1 | H | CH₂CH(Me) | H | CH₂CF₃ | C(=O)OEt | Me |
| B-1879 | 1 | H | CH₂CH(Me) | Me | CH₂CF₃ | C(=O)OEt | Me |
| B-1880 | 1 | Me | CH₂CH(Me) | H | CH₂CF₃ | C(=O)OEt | Me |
| B-1881 | 1 | Me | CH₂CH(Me) | Me | CH₂CF₃ | C(=O)OEt | Me |
| B-1882 | 1 | H | CH(Me)CH₂ | H | CH₂CF₃ | C(=O)OEt | Me |
| B-1883 | 1 | H | CH(Me)CH₂ | Me | CH₂CF₃ | C(=O)OEt | Me |
| B-1884 | 1 | Me | CH(Me)CH₂ | H | CH₂CF₃ | C(=O)OEt | Me |
| B-1885 | 1 | Me | CH(Me)CH₂ | Me | CH₂CF₃ | C(=O)OEt | Me |
| B-1886 | 1 | H | CH₂CH₂CH₂ | Me | CH₂CF₃ | C(=O)OEt | Me |

**[Table 207]**

| Compound Number | n | Y | W | Z | R¹ | R² | R³ |
|---|---|---|---|---|---|---|---|
| B-1887 | 1 | Me | CH₂CH₂CH₂ | H | CH₂CF₃ | C(=O)OEt | Me |
| B-1888 | 1 | Me | CH₂CH₂CH₂ | Me | CH₂CF₃ | C(=O)OEt | Me |
| B-1889 | 1 | H | CH₂CH₂CH(Me) | H | CH₂CF₃ | C(=O)OEt | Me |
| B-1890 | 1 | H | CH₂CH₂CH(Me) | Me | CH₂CF₃ | C(=O)OEt | Me |
| B-1891 | 1 | Me | CH₂CH₂CH(Me) | H | CH₂CF₃ | C(=O)OEt | Me |
| B-1892 | 1 | Me | CH₂CH₂CH(Me) | Me | CH₂CF₃ | C(=O)OEt | Me |
| B-1893 | 1 | H | CH₂CH(Me)CH₂ | H | CH₂CF₃ | C(=O)OEt | Me |
| B-1894 | 1 | H | CH₂CH(Me)CH₂ | Me | CH₂CF₃ | C(=O)OEt | Me |
| B-1895 | 1 | Me | CH₂CH(Me)CH₂ | H | CH₂CF₃ | C(=O)OEt | Me |
| B-1896 | 1 | Me | CH₂CH(Me)CH₂ | Me | CH₂CF₃ | C(=O)OEt | Me |
| B-1897 | 1 | H | CH(Me)CH₂CH₂ | H | CH₂CF₃ | C(=O)OEt | Me |
| B-1898 | 1 | H | CH(Me)CH₂CH₂ | Me | CH₂CF₃ | C(=O)OEt | Me |
| B-1899 | 1 | Me | CH(Me)CH₂CH₂ | H | CH₂CF₃ | C(=O)OEt | Me |
| B-1900 | 1 | Me | CH(Me)CH₂CH₂ | Me | CH₂CF₃ | C(=O)OEt | Me |
| B-1901 | 1 | H | single bond | Me | CH₂CF₃ | S(=O)₂Me | Cl |
| B-1902 | 1 | H | single bond | Et | CH₂CF₃ | S(=O)₂Me | Cl |
| B-1903 | 1 | Me | single bond | H | CH₂CF₃ | S(=O)₂Me | Cl |
| B-1904 | 1 | Et | single bond | H | CH₂CF₃ | S(=O)₂Me | Cl |
| B-1905 | 1 | Me | single bond | Me | CH₂CF₃ | S(=O)₂Me | Cl |
| B-1906 | 1 | H | CH₂ | Me | CH₂CF₃ | S(=O)₂Me | Cl |
| B-1907 | 1 | Me | CH₂ | H | CH₂CF₃ | S(=O)₂Me | Cl |
| B-1908 | 1 | Me | CH₂ | Me | CH₂CF₃ | S(=O)₂Me | Cl |
| B-1909 | 1 | H | CH(Me) | H | CH₂CF₃ | S(=O)₂Me | Cl |
| B-1910 | 1 | H | CH(Me) | Me | CH₂CF₃ | S(=O)₂Me | Cl |
| B-1911 | 1 | Me | CH(Me) | H | CH₂CF₃ | S(=O)₂Me | Cl |
| B-1912 | 1 | Me | CH(Me) | Me | CH₂CF₃ | S(=O)₂Me | Cl |
| B-1913 | 1 | H | CH₂CH₂ | Me | CH₂CF₃ | S(=O)₂Me | Cl |
| B-1914 | 1 | Me | CH₂CH₂ | H | CH₂CF₃ | S(=O)₂Me | Cl |
| B-1915 | 1 | Me | CH₂CH₂ | Me | CH₂CF₃ | S(=O)₂Me | Cl |
| B-1916 | 1 | H | CH₂CH(Me) | H | CH₂CF₃ | S(=O)₂Me | Cl |
| B-1917 | 1 | H | CH₂CH(Me) | Me | CH₂CF₃ | S(=O)₂Me | Cl |
| B-1918 | 1 | Me | CH₂CH(Me) | H | CH₂CF₃ | S(=O)₂Me | Cl |
| B-1919 | 1 | Me | CH₂CH(Me) | Me | CH₂CF₃ | S(=O)₂Me | Cl |
| B-1920 | 1 | H | CH(Me)CH₂ | H | CH₂CF₃ | S(=O)₂Me | Cl |
| B-1921 | 1 | H | CH(Me)CH₂ | Me | CH₂CF₃ | S(=O)₂Me | Cl |
| B-1922 | 1 | Me | CH(Me)CH₂ | H | CH₂CF₃ | S(=O)₂Me | Cl |
| B-1923 | 1 | Me | CH(Me)CH₂ | Me | CH₂CF₃ | S(=O)₂Me | Cl |
| B-1924 | 1 | H | CH₂CH₂CH₂ | Me | CH₂CF₃ | S(=O)₂Me | Cl |
| B-1925 | 1 | Me | CH₂CH₂CH₂ | H | CH₂CF₃ | S(=O)₂Me | Cl |
| B-1926 | 1 | Me | CH₂CH₂CH₂ | Me | CH₂CF₃ | S(=O)₂Me | Cl |
| B-1927 | 1 | H | CH₂CH₂CH(Me) | H | CH₂CF₃ | S(=O)₂Me | Cl |
| B-1928 | 1 | H | CH₂CH₂CH(Me) | Me | CH₂CF₃ | S(=O)₂Me | Cl |
| B-1929 | 1 | Me | CH₂CH₂CH(Me) | H | CH₂CF₃ | S(=O)₂Me | Cl |
| B-1930 | 1 | Me | CH₂CH₂CH(Me) | Me | CH₂CF₃ | S(=O)₂Me | Cl |

**[Table 208]**

| Compound Number | n | Y | W | Z | R¹ | R² | R³ |
|---|---|---|---|---|---|---|---|
| B-1931 | 1 | H | CH₂CH(Me)CH₂ | H | CH₂CF₃ | S(=O)₂Me | Cl |
| B-1932 | 1 | H | CH₂CH(Me)CH₂ | Me | CH₂CF₃ | S(=O)₂Me | Cl |
| B-1933 | 1 | Me | CH₂CH(Me)CH₂ | H | CH₂CF₃ | S(=O)₂Me | Cl |
| B-1934 | 1 | Me | CH₂CH(Me)CH₂ | Me | CH₂CF₃ | S(=O)₂Me | Cl |
| B-1935 | 1 | H | CH(Me)CH₂CH₂ | H | CH₂CF₃ | S(=O)₂Me | Cl |
| B-1936 | 1 | H | CH(Me)CH₂CH₂ | Me | CH₂CF₃ | S(=O)₂Me | Cl |
| B-1937 | 1 | Me | CH(Me)CH₂CH₂ | H | CH₂CF₃ | S(=O)₂Me | Cl |
| B-1938 | 1 | Me | CH(Me)CH₂CH₂ | Me | CH₂CF₃ | S(=O)₂Me | Cl |
| B-1939 | 1 | H | single bond | Me | CH₂CF₃ | S(=O)₂Me | Me |
| B-1940 | 1 | H | single bond | Et | CH₂CF₃ | S(=O)₂Me | Me |
| B-1941 | 1 | Me | single bond | H | CH₂CF₃ | S(=O)₂Me | Me |
| B-1942 | 1 | Et | single bond | H | CH₂CF₃ | S(=O)₂Me | Me |
| B-1943 | 1 | Me | single bond | Me | CH₂CF₃ | S(=O)₂Me | Me |
| B-1944 | 1 | H | CH₂ | Me | CH₂CF₃ | S(=O)₂Me | Me |
| B-1945 | 1 | Me | CH₂ | H | CH₂CF₃ | S(=O)₂Me | Me |
| B-1946 | 1 | Me | CH₂ | Me | CH₂CF₃ | S(=O)₂Me | Me |
| B-1947 | 1 | H | CH(Me) | H | CH₂CF₃ | S(=O)₂Me | Me |
| B-1948 | 1 | H | CH(Me) | Me | CH₂CF₃ | S(=O)₂Me | Me |
| B-1949 | 1 | Me | CH(Me) | H | CH₂CF₃ | S(=O)₂Me | Me |
| B-1950 | 1 | Me | CH(Me) | Me | CH₂CF₃ | S(=O)₂Me | Me |
| B-1951 | 1 | H | CH₂CH₂ | Me | CH₂CF₃ | S(=O)₂Me | Me |
| B-1952 | 1 | Me | CH₂CH₂ | H | CH₂CF₃ | S(=O)₂Me | Me |
| B-1953 | 1 | Me | CH₂CH₂ | Me | CH₂CF₃ | S(=O)₂Me | Me |
| B-1954 | 1 | H | CH₂CH(Me) | H | CH₂CF₃ | S(=O)₂Me | Me |
| B-1955 | 1 | H | CH₂CH(Me) | Me | CH₂CF₃ | S(=O)₂Me | Me |
| B-1956 | 1 | Me | CH₂CH(Me) | H | CH₂CF₃ | S(=O)₂Me | Me |
| B-1957 | 1 | Me | CH₂CH(Me) | Me | CH₂CF₃ | S(=O)₂Me | Me |
| B-1958 | 1 | H | CH(Me)CH₂ | H | CH₂CF₃ | S(=O)₂Me | Me |
| B-1959 | 1 | H | CH(Me)CH₂ | Me | CH₂CF₃ | S(=O)₂Me | Me |
| B-1960 | 1 | Me | CH(Me)CH₂ | H | CH₂CF₃ | S(=O)₂Me | Me |
| B-1961 | 1 | Me | CH(Me)CH₂ | Me | CH₂CF₃ | S(=O)₂Me | Me |
| B-1962 | 1 | H | CH₂CH₂CH₂ | Me | CH₂CF₃ | S(=O)₂Me | Me |
| B-1963 | 1 | Me | CH₂CH₂CH₂ | H | CH₂CF₃ | S(=O)₂Me | Me |
| B-1964 | 1 | Me | CH₂CH₂CH₂ | Me | CH₂CF₃ | S(=O)₂Me | Me |
| B-1965 | 1 | H | CH₂CH₂CH(Me) | H | CH₂CF₃ | S(=O)₂Me | Me |
| B-1966 | 1 | H | CH₂CH₂CH(Me) | Me | CH₂CF₃ | S(=O)₂Me | Me |
| B-1967 | 1 | Me | CH₂CH₂CH(Me) | H | CH₂CF₃ | S(=O)₂Me | Me |
| B-1968 | 1 | Me | CH₂CH₂CH(Me) | Me | CH₂CF₃ | S(=O)₂Me | Me |
| B-1969 | 1 | H | CH₂CH(Me)CH₂ | H | CH₂CF₃ | S(=O)₂Me | Me |
| B-1970 | 1 | H | CH₂CH(Me)CH₂ | Me | CH₂CF₃ | S(=O)₂Me | Me |
| B-1971 | 1 | Me | CH₂CH(Me)CH₂ | H | CH₂CF₃ | S(=O)₂Me | Me |
| B-1972 | 1 | Me | CH₂CH(Me)CH₂ | Me | CH₂CF₃ | S(=O)₂Me | Me |
| B-1973 | 1 | H | CH(Me)CH₂CH₂ | H | CH₂CF₃ | S(=O)₂Me | Me |
| B-1974 | 1 | H | CH(Me)CH₂CH₂ | Me | CH₂CF₃ | S(=O)₂Me | Me |

**[Table 209]**

| Compound Number | n | Y | W | Z | R¹ | R² | R³ |
|---|---|---|---|---|---|---|---|
| B-1975 | 1 | Me | CH(Me)CH₂CH₂ | H | CH₂CF₃ | S(=O)₂Me | Me |
| B-1976 | 1 | Me | CH(Me)CH₂CH₂ | Me | CH₂CF₃ | S(=O)₂Me | Me |
| B-1977 | 1 | H | single bond | Me | CH₂CF₃ | S(=O)₂CF₃ | Cl |
| B-1978 | 1 | H | single bond | Et | CH₂CF₃ | S(=O)₂CF₃ | Cl |
| B-1979 | 1 | Me | single bond | H | CH₂CF₃ | S(=O)₂CF₃ | Cl |
| B-1980 | 1 | Et | single bond | H | CH₂CF₃ | S(=O)₂CF₃ | Cl |
| B-1981 | 1 | Me | single bond | Me | CH₂CF₃ | S(=O)₂CF₃ | Cl |
| B-1982 | 1 | H | CH₂ | Me | CH₂CF₃ | S(=O)₂CF₃ | Cl |
| B-1983 | 1 | Me | CH₂ | H | CH₂CF₃ | S(=O)₂CF₃ | Cl |
| B-1984 | 1 | Me | CH₂ | Me | CH₂CF₃ | S(=O)₂CF₃ | Cl |
| B-1985 | 1 | H | CH(Me) | H | CH₂CF₃ | S(=O)₂CF₃ | Cl |
| B-1986 | 1 | H | CH(Me) | Me | CH₂CF₃ | S(=O)₂CF₃ | Cl |
| B-1987 | 1 | Me | CH(Me) | H | CH₂CF₃ | S(=O)₂CF₃ | Cl |
| B-1988 | 1 | Me | CH(Me) | Me | CH₂CF₃ | S(=O)₂CF₃ | Cl |
| B-1989 | 1 | H | CH₂CH₂ | Me | CH₂CF₃ | S(=O)₂CF₃ | Cl |
| B-1990 | 1 | Me | CH₂CH₂ | H | CH₂CF₃ | S(=O)₂CF₃ | Cl |
| B-1991 | 1 | Me | CH₂CH₂ | Me | CH₂CF₃ | S(=O)₂CF₃ | Cl |
| B-1992 | 1 | H | CH₂CH(Me) | H | CH₂CF₃ | S(=O)₂CF₃ | Cl |
| B-1993 | 1 | H | CH₂CH(Me) | Me | CH₂CF₃ | S(=O)₂CF₃ | Cl |
| B-1994 | 1 | Me | CH₂CH(Me) | H | CH₂CF₃ | S(=O)₂CF₃ | Cl |
| B-1995 | 1 | Me | CH₂CH(Me) | Me | CH₂CF₃ | S(=O)₂CF₃ | Cl |
| B-1996 | 1 | H | CH(Me)CH₂ | H | CH₂CF₃ | S(=O)₂CF₃ | Cl |
| B-1997 | 1 | H | CH(Me)CH₂ | Me | CH₂CF₃ | S(=O)₂CF₃ | Cl |
| B-1998 | 1 | Me | CH(Me)CH₂ | H | CH₂CF₃ | S(=O)₂CF₃ | Cl |
| B-1999 | 1 | Me | CH(Me)CH₂ | Me | CH₂CF₃ | S(=O)₂CF₃ | Cl |
| B-2000 | 1 | H | CH₂CH₂CH₂ | Me | CH₂CF₃ | S(=O)₂CF₃ | Cl |
| B-2001 | 1 | Me | CH₂CH₂CH₂ | H | CH₂CF₃ | S(=O)₂CF₃ | Cl |
| B-2002 | 1 | Me | CH₂CH₂CH₂ | Me | CH₂CF₃ | S(=O)₂CF₃ | Cl |
| B-2003 | 1 | H | CH₂CH₂CH(Me) | H | CH₂CF₃ | S(=O)₂CF₃ | Cl |
| B-2004 | 1 | H | CH₂CH₂CH(Me) | Me | CH₂CF₃ | S(=O)₂CF₃ | Cl |
| B-2005 | 1 | Me | CH₂CH₂CH(Me) | H | CH₂CF₃ | S(=O)₂CF₃ | Cl |
| B-2006 | 1 | Me | CH₂CH₂CH(Me) | Me | CH₂CF₃ | S(=O)₂CF₃ | Cl |
| B-2007 | 1 | H | CH₂CH(Me)CH₂ | H | CH₂CF₃ | S(=O)₂CF₃ | Cl |
| B-2008 | 1 | H | CH₂CH(Me)CH₂ | Me | CH₂CF₃ | S(=O)₂CF₃ | Cl |
| B-2009 | 1 | Me | CH₂CH(Me)CH₂ | H | CH₂CF₃ | S(=O)₂CF₃ | Cl |
| B-2010 | 1 | Me | CH₂CH(Me)CH₂ | Me | CH₂CF₃ | S(=O)₂CF₃ | Cl |
| B-2011 | 1 | H | CH(Me)CH₂CH₂ | H | CH₂CF₃ | S(=O)₂CF₃ | Cl |
| B-2012 | 1 | H | CH(Me)CH₂CH₂ | Me | CH₂CF₃ | S(=O)₂CF₃ | Cl |
| B-2013 | 1 | Me | CH(Me)CH₂CH₂ | H | CH₂CF₃ | S(=O)₂CF₃ | Cl |
| B-2014 | 1 | Me | CH(Me)CH₂CH₂ | Me | CH₂CF₃ | S(=O)₂CF₃ | Cl |
| B-2015 | 1 | H | single bond | Me | CH₂CF₃ | S(=O)₂CF₃ | Me |
| B-2016 | 1 | H | single bond | Et | CH₂CF₃ | S(=O)₂CF₃ | Me |
| B-2017 | 1 | Me | single bond | H | CH₂CF₃ | S(=O)₂CF₃ | Me |
| B-2018 | 1 | Et | single bond | H | CH₂CF₃ | S(=O)₂CF₃ | Me |

**[Table 210]**

| Compound Number | n | Y | W | Z | R¹ | R² | R³ |
|---|---|---|---|---|---|---|---|
| B-2019 | 1 | Me | single bond | Me | CH₂CF₃ | S(=O)₂CF₃ | Me |
| B-2020 | 1 | H | CH₂ | Me | CH₂CF₃ | S(=O)₂CF₃ | Me |
| B-2021 | 1 | Me | CH₂ | H | CH₂CF₃ | S(=O)₂CF₃ | Me |
| B-2022 | 1 | Me | CH₂ | Me | CH₂CF₃ | S(=O)₂CF₃ | Me |
| B-2023 | 1 | H | CH(Me) | H | CH₂CF₃ | S(=O)₂CF₃ | Me |
| B-2024 | 1 | H | CH(Me) | Me | CH₂CF₃ | S(=O)₂CF₃ | Me |
| B-2025 | 1 | Me | CH(Me) | H | CH₂CF₃ | S(=O)₂CF₃ | Me |
| B-2026 | 1 | Me | CH(Me) | Me | CH₂CF₃ | S(=O)₂CF₃ | Me |
| B-2027 | 1 | H | CH₂CH₂ | Me | CH₂CF₃ | S(=O)₂CF₃ | Me |
| B-2028 | 1 | Me | CH₂CH₂ | H | CH₂CF₃ | S(=O)₂CF₃ | Me |
| B-2029 | 1 | Me | CH₂CH₂ | Me | CH₂CF₃ | S(=O)₂CF₃ | Me |
| B-2030 | 1 | H | CH₂CH(Me) | H | CH₂CF₃ | S(=O)₂CF₃ | Me |
| B-2031 | 1 | H | CH₂CH(Me) | Me | CH₂CF₃ | S(=O)₂CF₃ | Me |
| B-2032 | 1 | Me | CH₂CH(Me) | H | CH₂CF₃ | S(=O)₂CF₃ | Me |
| B-2033 | 1 | Me | CH₂CH(Me) | Me | CH₂CF₃ | S(=O)₂CF₃ | Me |
| B-2034 | 1 | H | CH(Me)CH₂ | H | CH₂CF₃ | S(=O)₂CF₃ | Me |
| B-2035 | 1 | H | CH(Me)CH₂ | Me | CH₂CF₃ | S(=O)₂CF₃ | Me |
| B-2036 | 1 | Me | CH(Me)CH₂ | H | CH₂CF₃ | S(=O)₂CF₃ | Me |
| B-2037 | 1 | Me | CH(Me)CH₂ | Me | CH₂CF₃ | S(=O)₂CF₃ | Me |
| B-2038 | 1 | H | CH₂CH₂CH₂ | Me | CH₂CF₃ | S(=O)₂CF₃ | Me |
| B-2039 | 1 | Me | CH₂CH₂CH₂ | H | CH₂CF₃ | S(=O)₂CF₃ | Me |
| B-2040 | 1 | Me | CH₂CH₂CH₂ | Me | CH₂CF₃ | S(=O)₂CF₃ | Me |
| B-2041 | 1 | H | CH₂CH₂CH(Me) | H | CH₂CF₃ | S(=O)₂CF₃ | Me |
| B-2042 | 1 | H | CH₂CH₂CH(Me) | Me | CH₂CF₃ | S(=O)₂CF₃ | Me |
| B-2043 | 1 | Me | CH₂CH₂CH(Me) | H | CH₂CF₃ | S(=O)₂CF₃ | Me |
| B-2044 | 1 | Me | CH₂CH₂CH(Me) | Me | CH₂CF₃ | S(=O)₂CF₃ | Me |
| B-2045 | 1 | H | CH₂CH(Me)CH₂ | H | CH₂CF₃ | S(=O)₂CF₃ | Me |
| B-2046 | 1 | H | CH₂CH(Me)CH₂ | Me | CH₂CF₃ | S(=O)₂CF₃ | Me |
| B-2047 | 1 | Me | CH₂CH(Me)CH₂ | H | CH₂CF₃ | S(=O)₂CF₃ | Me |
| B-2048 | 1 | Me | CH₂CH(Me)CH₂ | Me | CH₂CF₃ | S(=O)₂CF₃ | Me |
| B-2049 | 1 | H | CH(Me)CH₂CH₂ | H | CH₂CF₃ | S(=O)₂CF₃ | Me |
| B-2050 | 1 | H | CH(Me)CH₂CH₂ | Me | CH₂CF₃ | S(=O)₂CF₃ | Me |
| B-2051 | 1 | Me | CH(Me)CH₂CH₂ | H | CH₂CF₃ | S(=O)₂CF₃ | Me |
| B-2052 | 1 | Me | CH(Me)CH₂CH₂ | Me | CH₂CF₃ | S(=O)₂CF₃ | Me |
| B-2053 | 2 | H | single bond | Me | CH₂CF₃ | Me | Cl |
| B-2054 | 2 | H | single bond | Et | CH₂CF₃ | Me | Cl |
| B-2055 | 2 | Me | single bond | H | CH₂CF₃ | Me | Cl |
| B-2056 | 2 | Et | single bond | H | CH₂CF₃ | Me | Cl |
| B-2057 | 2 | Me | single bond | Me | CH₂CF₃ | Me | Cl |
| B-2058 | 2 | H | CH₂ | Me | CH₂CF₃ | Me | Cl |
| B-2059 | 2 | Me | CH₂ | H | CH₂CF₃ | Me | Cl |
| B-2060 | 2 | Me | CH₂ | Me | CH₂CF₃ | Me | Cl |
| B-2061 | 2 | H | CH(Me) | H | CH₂CF₃ | Me | Cl |
| B-2062 | 2 | H | CH(Me) | Me | CH₂CF₃ | Me | Cl |

**[Table 211]**

| Compound Number | n | Y | W | Z | R¹ | R² | R³ |
|---|---|---|---|---|---|---|---|
| B-2063 | 2 | Me | CH(Me) | H | CH₂CF₃ | Me | Cl |
| B-2064 | 2 | Me | CH(Me) | Me | CH₂CF₃ | Me | Cl |
| B-2065 | 2 | H | CH₂CH₂ | Me | CH₂CF₃ | Me | Cl |
| B-2066 | 2 | Me | CH₂CH₂ | H | CH₂CF₃ | Me | Cl |
| B-2067 | 2 | Me | CH₂CH₂ | Me | CH₂CF₃ | Me | Cl |
| B-2068 | 2 | H | CH₂CH(Me) | H | CH₂CF₃ | Me | Cl |
| B-2069 | 2 | H | CH₂CH(Me) | Me | CH₂CF₃ | Me | Cl |
| B-2070 | 2 | Me | CH₂CH(Me) | H | CH₂CF₃ | Me | Cl |
| B-2071 | 2 | Me | CH₂CH(Me) | Me | CH₂CF₃ | Me | Cl |
| B-2072 | 2 | H | CH(Me)CH₂ | H | CH₂CF₃ | Me | Cl |
| B-2073 | 2 | H | CH(Me)CH₂ | Me | CH₂CF₃ | Me | Cl |
| B-2074 | 2 | Me | CH(Me)CH₂ | H | CH₂CF₃ | Me | Cl |
| B-2075 | 2 | Me | CH(Me)CH₂ | Me | CH₂CF₃ | Me | Cl |
| B-2076 | 2 | H | CH₂CH₂CH₂ | Me | CH₂CF₃ | Me | Cl |
| B-2077 | 2 | Me | CH₂CH₂CH₂ | H | CH₂CF₃ | Me | Cl |
| B-2078 | 2 | Me | CH₂CH₂CH₂ | Me | CH₂CF₃ | Me | Cl |
| B-2079 | 2 | H | CH₂CH₂CH(Me) | H | CH₂CF₃ | Me | Cl |
| B-2080 | 2 | H | CH₂CH₂CH(Me) | Me | CH₂CF₃ | Me | Cl |
| B-2081 | 2 | Me | CH₂CH₂CH(Me) | H | CH₂CF₃ | Me | Cl |
| B-2082 | 2 | Me | CH₂CH₂CH(Me) | Me | CH₂CF₃ | Me | Cl |
| B-2083 | 2 | H | CH₂CH(Me)CH₂ | H | CH₂CF₃ | Me | Cl |
| B-2084 | 2 | H | CH₂CH(Me)CH₂ | Me | CH₂CF₃ | Me | Cl |
| B-2085 | 2 | Me | CH₂CH(Me)CH₂ | H | CH₂CF₃ | Me | Cl |
| B-2086 | 2 | Me | CH₂CH(Me)CH₂ | Me | CH₂CF₃ | Me | Cl |
| B-2087 | 2 | H | CH(Me)CH₂CH₂ | H | CH₂CF₃ | Me | Cl |
| B-2088 | 2 | H | CH(Me)CH₂CH₂ | Me | CH₂CF₃ | Me | Cl |
| B-2089 | 2 | Me | CH(Me)CH₂CH₂ | H | CH₂CF₃ | Me | Cl |
| B-2090 | 2 | Me | CH(Me)CH₂CH₂ | Me | CH₂CF₃ | Me | Cl |
| B-2091 | 2 | H | single bond | Me | CH₂CF₃ | Me | Me |
| B-2092 | 2 | H | single bond | Et | CH₂CF₃ | Me | Me |
| B-2003 | 2 | Me | single bond | H | CH₂CF₃ | Me | Me |
| B-2094 | 2 | Et | single bond | H | CH₂CF₃ | Me | Me |
| B-2095 | 2 | Me | single bond | Me | CH₂CF₃ | Me | Me |
| B-2096 | 2 | H | CH₂ | Me | CH₂CF₃ | Me | Me |
| B-2097 | 2 | Me | CH₂ | H | CH₂CF₃ | Me | Me |
| B-2098 | 2 | Me | CH₂ | Me | CH₂CF₃ | Me | Me |
| B-2099 | 2 | H | CH(Me) | H | CH₂CF₃ | Me | Me |
| B-2100 | 2 | H | CH(Me) | Me | CH₂CF₃ | Me | Me |
| B-2101 | 2 | Me | CH(Me) | H | CH₂CF₃ | Me | Me |
| B-2102 | 2 | Me | CH(Me) | Me | CH₂CF₃ | Me | Me |
| B-2103 | 2 | H | CH₂CH₂ | Me | CH₂CF₃ | Me | Me |
| B-2104 | 2 | Me | CH₂CH₂ | H | CH₂CF₃ | Me | Me |
| B-2105 | 2 | Me | CH₂CH₂ | Me | CH₂CF₃ | Me | Me |
| B-2106 | 2 | H | CH₂CH(Me) | H | CH₂CF₃ | Me | Me |

**[Table 212]**

| Compound Number | n | Y | W | Z | R¹ | R² | R³ |
|---|---|---|---|---|---|---|---|
| B-2107 | 2 | H | CH₂CH(Me) | Me | CH₂CF₃ | Me | Me |
| B-2108 | 2 | Me | CH₂CH(Me) | H | CH₂CF₃ | Me | Me |
| B-2109 | 2 | Me | CH₂CH(Me) | Me | CH₂CF₃ | Me | Me |
| B-2110 | 2 | H | CH(Me)CH₂ | H | CH₂CF₃ | Me | Me |
| B-2111 | 2 | H | CH(Me)CH₂ | Me | CH₂CF₃ | Me | Me |
| B-2112 | 2 | Me | CH(Me)CH₂ | H | CH₂CF₃ | Me | Me |
| B-2113 | 2 | Me | CH(Me)CH₂ | Me | CH₂CF₃ | Me | Me |
| B-2114 | 2 | H | CH₂CH₂CH₂ | Me | CH₂CF₃ | Me | Me |
| B-2115 | 2 | Me | CH₂CH₂CH₂ | H | CH₂CF₃ | Me | Me |
| B-2116 | 2 | Me | CH₂CH₂CH₂ | Me | CH₂CF₃ | Me | Me |
| B-2117 | 2 | H | CH₂CH₂CH(Me) | H | CH₂CF₃ | Me | Me |
| B-2118 | 2 | H | CH₂CH₂CH(Me) | Me | CH₂CF₃ | Me | Me |
| B-2119 | 2 | Me | CH₂CH₂CH(Me) | H | CH₂CF₃ | Me | Me |
| B-2120 | 2 | Me | CH₂CH₂CH(Me) | Me | CH₂CF₃ | Me | Me |
| B-2121 | 2 | H | CH₂CH(Me)CH₂ | H | CH₂CF₃ | Me | Me |
| B-2122 | 2 | H | CH₂CH(Me)CH₂ | Me | CH₂CF₃ | Me | Me |
| B-2123 | 2 | Me | CH₂CH(Me)CH₂ | H | CH₂CF₃ | Me | Me |
| B-2124 | 2 | Me | CH₂CH(Me)CH₂ | Me | CH₂CF₃ | Me | Me |
| B-2125 | 2 | H | CH(Me)CH₂CH₂ | H | CH₂CF₃ | Me | Me |
| B-2126 | 2 | H | CH(Me)CH₂CH₂ | Me | CH₂CF₃ | Me | Me |
| B-2127 | 2 | Me | CH(Me)CH₂CH₂ | H | CH₂CF₃ | Me | Me |
| B-2128 | 2 | Me | CH(Me)CH₂CH₂ | Me | CH₂CF₃ | Me | Me |
| B-2129 | 2 | H | single bond | Me | CH₂CF₃ | Et | Cl |
| B-2130 | 2 | H | single bond | Et | CH₂CF₃ | Et | Cl |
| B-2131 | 2 | Me | single bond | H | CH₂CF₃ | Et | Cl |
| B-2132 | 2 | Et | single bond | H | CH₂CF₃ | Et | Cl |
| B-2133 | 2 | Me | single bond | Me | CH₂CF₃ | Et | Cl |
| B-2134 | 2 | H | CH₂ | Me | CH₂CF₃ | Et | Cl |
| B-2135 | 2 | Me | CH₂ | H | CH₂CF₃ | Et | Cl |
| B-2136 | 2 | Me | CH₂ | Me | CH₂CF₃ | Et | Cl |
| B-2137 | 2 | H | CH(Me) | H | CH₂CF₃ | Et | Cl |
| B-2138 | 2 | H | CH(Me) | Me | CH₂CF₃ | Et | Cl |
| B-2139 | 2 | Me | CH(Me) | H | CH₂CF₃ | Et | Cl |
| B-2140 | 2 | Me | CH(Me) | Me | CH₂CF₃ | Et | Cl |
| B-2141 | 2 | H | CH₂CH₂ | Me | CH₂CF₃ | Et | Cl |
| B-2142 | 2 | Me | CH₂CH₂ | H | CH₂CF₃ | Et | Cl |
| B-2143 | 2 | Me | CH₂CH₂ | Me | CH₂CF₃ | Et | Cl |
| B-2144 | 2 | H | CH₂CH(Me) | H | CH₂CF₃ | Et | Cl |
| B-2145 | 2 | H | CH₂CH(Me) | Me | CH₂CF₃ | Et | Cl |
| B-2146 | 2 | Me | CH₂CH(Me) | H | CH₂CF₃ | Et | Cl |
| B-2147 | 2 | Me | CH₂CH(Me) | Me | CH₂CF₃ | Et | Cl |
| B-2148 | 2 | H | CH(Me)CH₂ | H | CH₂CF₃ | Et | Cl |
| B-2149 | 2 | H | CH(Me)CH₂ | Me | CH₂CF₃ | Et | Cl |
| B-2150 | 2 | Me | CH(Me)CH₂ | H | CH₂CF₃ | Et | Cl |

**[Table 213]**

| Compound Number | n | Y | W | Z | R¹ | R² | R³ |
|---|---|---|---|---|---|---|---|
| B-2151 | 2 | Me | CH(Me)CH₂ | Me | CH₂CF₃ | Et | Cl |
| B-2152 | 2 | H | CH₂CH₂CH₂ | Me | CH₂CF₃ | Et | Cl |
| B-2153 | 2 | Me | CH₂CH₂CH₂ | H | CH₂CF₃ | Et | Cl |
| B-2154 | 2 | Me | CH₂CH₂CH₂ | Me | CH₂CF₃ | Et | Cl |
| B-2155 | 2 | H | CH₂CH₂CH(Me) | H | CH₂CF₃ | Et | Cl |
| B-2156 | 2 | H | CH₂CH₂CH(Me) | Me | CH₂CF₃ | Et | Cl |
| B-2157 | 2 | Me | CH₂CH₂CH(Me) | H | CH₂CF₃ | Et | Cl |
| B-2158 | 2 | Me | CH₂CH₂CH(Me) | Me | CH₂CF₃ | Et | Cl |
| B-2159 | 2 | H | CH₂CH(Me)CH₂ | H | CH₂CF₃ | Et | Cl |
| B-2160 | 2 | H | CH₂CH(Me)CH₂ | Me | CH₂CF₃ | Et | Cl |
| B-2161 | 2 | Me | CH₂CH(Me)CH₂ | H | CH₂CF₃ | Et | Cl |
| B-2162 | 2 | Me | CH₂CH(Me)CH₂ | Me | CH₂CF₃ | Et | Cl |
| B-2163 | 2 | H | CH(Me)CH₂CH₂ | H | CH₂CF₃ | Et | Cl |
| B-2164 | 2 | H | CH(Me)CH₂CH₂ | Me | CH₂CF₃ | Et | Cl |
| B-2165 | 2 | Me | CH(Me)CH₂CH₂ | H | CH₂CF₃ | Et | Cl |
| B-2166 | 2 | Me | CH(Me)CH₂CH₂ | Me | CH₂CF₃ | Et | Cl |
| B-2167 | 2 | H | single bond | Me | CH₂CF₃ | Et | Me |
| B-2168 | 2 | H | single bond | Et | CH₂CF₃ | Et | Me |
| B-2169 | 2 | Me | single bond | H | CH₂CF₃ | Et | Me |
| B-2170 | 2 | Et | single bond | H | CH₂CF₃ | Et | Me |
| B-2171 | 2 | Me | single bond | Me | CH₂CF₃ | Et | Me |
| B-2172 | 2 | H | CH₂ | Me | CH₂CF₃ | Et | Me |
| B-2173 | 2 | Me | CH₂ | H | CH₂CF₃ | Et | Me |
| B-2174 | 2 | Me | CH₂ | Me | CH₂CF₃ | Et | Me |
| B-2175 | 2 | H | CH(Me) | H | CH₂CF₃ | Et | Me |
| B-2176 | 2 | H | CH(Me) | Me | CH₂CF₃ | Et | Me |
| B-2177 | 2 | Me | CH(Me) | H | CH₂CF₃ | Et | Me |
| B-2178 | 2 | Me | CH(Me) | Me | CH₂CF₃ | Et | Me |
| B-2179 | 2 | H | CH₂CH₂ | Me | CH₂CF₃ | Et | Me |
| B-2180 | 2 | Me | CH₂CH₂ | H | CH₂CF₃ | Et | Me |
| B-2181 | 2 | Me | CH₂CH₂ | Me | CH₂CF₃ | Et | Me |
| B-2182 | 2 | H | CH₂CH(Me) | H | CH₂CF₃ | Et | Me |
| B-2183 | 2 | H | CH₂CH(Me) | Me | CH₂CF₃ | Et | Me |
| B-2184 | 2 | Me | CH₂CH(Me) | H | CH₂CF₃ | Et | Me |
| B-2185 | 2 | Me | CH₂CH(Me) | Me | CH₂CF₃ | Et | Me |
| B-2186 | 2 | H | CH(Me)CH₂ | H | CH₂CF₃ | Et | Me |
| B-2187 | 2 | H | CH(Me)CH₂ | Me | CH₂CF₃ | Et | Me |
| B-2188 | 2 | Me | CH(Me)CH₂ | H | CH₂CF₃ | Et | Me |
| B-2189 | 2 | Me | CH(Me)CH₂ | Me | CH₂CF₃ | Et | Me |
| B-2190 | 2 | H | CH₂CH₂CH₂ | Me | CH₂CF₃ | Et | Me |
| B-2191 | 2 | Me | CH₂CH₂CH₂ | H | CH₂CF₃ | Et | Me |
| B-2192 | 2 | Me | CH₂CH₂CH₂ | Me | CH₂CF₃ | Et | Me |
| B-2193 | 2 | H | CH₂CH₂CH(Me) | H | CH₂CF₃ | Et | Me |
| B-2194 | 2 | H | CH₂CH₂CH(Me) | Me | CH₂CF₃ | Et | Me |

**[Table 214]**

| Compound Number | n | Y | W | Z | R¹ | R² | R³ |
|---|---|---|---|---|---|---|---|
| B-2195 | 2 | Me | CH₂CH₂CH(Me) | H | CH₂CF₃ | Et | Me |
| B-2196 | 2 | Me | CH₂CH₂CH(Me) | Me | CH₂CF₃ | Et | Me |
| B-2197 | 2 | H | CH₂CH(Me)CH₂ | H | CH₂CF₃ | Et | Me |
| B-2198 | 2 | H | CH₂CH(Me)CH₂ | Me | CH₂CF₃ | Et | Me |
| B-2199 | 2 | Me | CH₂CH(Me)CH₂ | H | CH₂CF₃ | Et | Me |
| B-2200 | 2 | Me | CH₂CH(Me)CH₂ | Me | CH₂CF₃ | Et | Me |
| B-2201 | 2 | H | CH(Me)CH₂CH₂ | H | CH₂CF₃ | Et | Me |
| B-2202 | 2 | H | CH(Me)CH₂CH₂ | Me | CH₂CF₃ | Et | Me |
| B-2203 | 2 | Me | CH(Me)CH₂CH₂ | H | CH₂CF₃ | Et | Me |
| B-2204 | 2 | Me | CH(Me)CH₂CH₂ | Me | CH₂CF₃ | Et | Me |
| B-2205 | 2 | H | single bond | Me | CH₂CF₃ | CH₂OMe | Cl |
| B-2206 | 2 | H | single bond | Et | CH₂CF₃ | CH₂OMe | Cl |
| B-2207 | 2 | Me | single bond | H | CH₂CF₃ | CH₂OMe | Cl |
| B-2208 | 2 | Et | single bond | H | CH₂CF₃ | CH₂OMe | Cl |
| B-2209 | 2 | Me | single bond | Me | CH₂CF₃ | CH₂OMe | Cl |
| B-2210 | 2 | H | CH₂ | Me | CH₂CF₃ | CH₂OMe | Cl |
| B-2211 | 2 | Me | CH₂ | H | CH₂CF₃ | CH₂OMe | Cl |
| B-2212 | 2 | Me | CH₂ | Me | CH₂CF₃ | CH₂OMe | Cl |
| B-2213 | 2 | H | CH(Me) | H | CH₂CF₃ | CH₂OMe | Cl |
| B-2214 | 2 | H | CH(Me) | Me | CH₂CF₃ | CH₂OMe | Cl |
| B-2215 | 2 | Me | CH(Me) | H | CH₂CF₃ | CH₂OMe | Cl |
| B-2216 | 2 | Me | CH(Me) | Me | CH₂CF₃ | CH₂OMe | Cl |
| B-2217 | 2 | H | CH₂CH₂ | Me | CH₂CF₃ | CH₂OMe | Cl |
| B-2218 | 2 | Me | CH₂CH₂ | H | CH₂CF₃ | CH₂OMe | Cl |
| B-2219 | 2 | Me | CH₂CH₂ | Me | CH₂CF₃ | CH₂OMe | Cl |
| B-2220 | 2 | H | CH₂CH(Me) | H | CH₂CF₃ | CH₂OMe | Cl |
| B-2221 | 2 | H | CH₂CH(Me) | Me | CH₂CF₃ | CH₂OMe | Cl |
| B-2222 | 2 | Me | CH₂CH(Me) | H | CH₂CF₃ | CH₂OMe | Cl |
| B-2223 | 2 | Me | CH₂CH(Me) | Me | CH₂CF₃ | CH₂OMe | Cl |
| B-2224 | 2 | H | CH(Me)CH₂ | H | CH₂CF₃ | CH₂OMe | Cl |
| B-2225 | 2 | H | CH(Me)CH₂ | Me | CH₂CF₃ | CH₂OMe | Cl |
| B-2226 | 2 | Me | CH(Me)CH₂ | H | CH₂CF₃ | CH₂OMe | Cl |
| B-2227 | 2 | Me | CH(Me)CH₂ | Me | CH₂CF₃ | CH₂OMe | Cl |
| B-2228 | 2 | H | CH₂CH₂CH₂ | Me | CH₂CF₃ | CH₂OMe | Cl |
| B-2229 | 2 | Me | CH₂CH₂CH₂ | H | CH₂CF₃ | CH₂OMe | Cl |
| B-2230 | 2 | Me | CH₂CH₂CH₂ | Me | CH₂CF₃ | CH₂OMe | Cl |
| B-2231 | 2 | H | CH₂CH₂CH(Me) | H | CH₂CF₃ | CH₂OMe | Cl |
| B-2232 | 2 | H | CH₂CH₂CH(Me) | Me | CH₂CF₃ | CH₂OMe | Cl |
| B-2233 | 2 | Me | CH₂CH₂CH(Me) | H | CH₂CF₃ | CH₂OMe | Cl |
| B-2234 | 2 | Me | CH₂CH₂CH(Me) | Me | CH₂CF₃ | CH₂OMe | Cl |
| B-2235 | 2 | H | CH₂CH(Me)CH₂ | H | CH₂CF₃ | CH₂OMe | Cl |
| B-2236 | 2 | H | CH₂CH(Me)CH₂ | Me | CH₂CF₃ | CH₂OMe | Cl |
| B-2237 | 2 | Me | CH₂CH(Me)CH₂ | H | CH₂CF₃ | CH₂OMe | Cl |
| B-2238 | 2 | Me | CH₂CH(Me)CH₂ | Me | CH₂CF₃ | CH₂OMe | Cl |

**[Table 215]**

| Compound Number | n | Y | W | Z | R¹ | R² | R³ |
|---|---|---|---|---|---|---|---|
| B-2239 | 2 | H | CH(Me)CH₂CH₂ | H | CH₂CF₃ | CH₂OMe | Cl |
| B-2240 | 2 | H | CH(Me)CH₂CH₂ | Me | CH₂CF₃ | CH₂OMe | Cl |
| B-2241 | 2 | Me | CH(Me)CH₂CH₂ | H | CH₂CF₃ | CH₂OMe | Cl |
| B-2242 | 2 | Me | CH(Me)CH₂CH₂ | Me | CH₂CF₃ | CH₂OMe | Cl |
| B-2243 | 2 | H | single bond | Me | CH₂CF₃ | CH₂OMe | Me |
| B-2244 | 2 | H | single bond | Et | CH₂CF₃ | CH₂OMe | Me |
| B-2245 | 2 | Me | single bond | H | CH₂CF₃ | CH₂OMe | Me |
| B-2246 | 2 | Et | single bond | H | CH₂CF₃ | CH₂OMe | Me |
| B-2247 | 2 | Me | single bond | Me | CH₂CF₃ | CH₂OMe | Me |
| B-2248 | 2 | H | CH₂ | Me | CH₂CF₃ | CH₂OMe | Me |
| B-2249 | 2 | Me | CH₂ | H | CH₂CF₃ | CH₂OMe | Me |
| B-2250 | 2 | Me | CH₂ | Me | CH₂CF₃ | CH₂OMe | Me |
| B-2251 | 2 | H | CH(Me) | H | CH₂CF₃ | CH₂OMe | Me |
| B-2252 | 2 | H | CH(Me) | Me | CH₂CF₃ | CH₂OMe | Me |
| B-2253 | 2 | Me | CH(Me) | H | CH₂CF₃ | CH₂OMe | Me |
| B-2254 | 2 | Me | CH(Me) | Me | CH₂CF₃ | CH₂OMe | Me |
| B-2255 | 2 | H | CH₂CH₂ | Me | CH₂CF₃ | CH₂OMe | Me |
| B-2256 | 2 | Me | CH₂CH₂ | H | CH₂CF₃ | CH₂OMe | Me |
| B-2257 | 2 | Me | CH₂CH₂ | Me | CH₂CF₃ | CH₂OMe | Me |
| B-2258 | 2 | H | CH₂CH(Me) | H | CH₂CF₃ | CH₂OMe | Me |
| B-2259 | 2 | H | CH₂CH(Me) | Me | CH₂CF₃ | CH₂OMe | Me |
| B-2260 | 2 | Me | CH₂CH(Me) | H | CH₂CF₃ | CH₂OMe | Me |
| B-2261 | 2 | Me | CH₂CH(Me) | Me | CH₂CF₃ | CH₂OMe | Me |
| B-2262 | 2 | H | CH(Me)CH₂ | H | CH₂CF₃ | CH₂OMe | Me |
| B-2263 | 2 | H | CH(Me)CH₂ | Me | CH₂CF₃ | CH₂OMe | Me |
| B-2264 | 2 | Me | CH(Me)CH₂ | H | CH₂CF₃ | CH₂OMe | Me |
| B-2265 | 2 | Me | CH(Me)CH₂ | Me | CH₂CF₃ | CH₂OMe | Me |
| B-2266 | 2 | H | CH₂CH₂CH₂ | Me | CH₂CF₃ | CH₂OMe | Me |
| B-2267 | 2 | Me | CH₂CH₂CH₂ | H | CH₂CF₃ | CH₂OMe | Me |
| B-2268 | 2 | Me | CH₂CH₂CH₂ | Me | CH₂CF₃ | CH₂OMe | Me |
| B-2269 | 2 | H | CH₂CH₂CH(Me) | H | CH₂CF₃ | CH₂OMe | Me |
| B-2270 | 2 | H | CH₂CH₂CH(Me) | Me | CH₂CF₃ | CH₂OMe | Me |
| B-2271 | 2 | Me | CH₂CH₂CH(Me) | H | CH₂CF₃ | CH₂OMe | Me |
| B-2272 | 2 | Me | CH₂CH₂CH(Me) | Me | CH₂CF₃ | CH₂OMe | Me |
| B-2273 | 2 | H | CH₂CH(Me)CH₂ | H | CH₂CF₃ | CH₂OMe | Me |
| B-2274 | 2 | H | CH₂CH(Me)CH₂ | Me | CH₂CF₃ | CH₂OMe | Me |
| B-2275 | 2 | Me | CH₂CH(Me)CH₂ | H | CH₂CF₃ | CH₂OMe | Me |
| B-2276 | 2 | Me | CH₂CH(Me)CH₂ | Me | CH₂CF₃ | CH₂OMe | Me |
| B-2277 | 2 | H | CH(Me)CH₂CH₂ | H | CH₂CF₃ | CH₂OMe | Me |
| B-2278 | 2 | H | CH(Me)CH₂CH₂ | Me | CH₂CF₃ | CH₂OMe | Me |
| B-2279 | 2 | Me | CH(Me)CH₂CH₂ | H | CH₂CF₃ | CH₂OMe | Me |
| B-2280 | 2 | Me | CH(Me)CH₂CH₂ | Me | CH₂CF₃ | CH₂OMe | Me |
| B-2281 | 2 | H | single bond | Me | CH₂CF₃ | CH₂CN | Cl |
| B-2282 | 2 | H | single bond | Et | CH₂CF₃ | CH₂CN | Cl |

**[Table 216]**

| Compound Number | n | Y | W | Z | R¹ | R² | R³ |
|---|---|---|---|---|---|---|---|
| B-2283 | 2 | Me | single bond | H | CH₂CF₃ | CH₂CN | Cl |
| B-2284 | 2 | Et | single bond | H | CH₂CF₃ | CH₂CN | Cl |
| B-2285 | 2 | Me | single bond | Me | CH₂CF₃ | CH₂CN | Cl |
| B-2286 | 2 | H | CH₂ | Me | CH₂CF₃ | CH₂CN | Cl |
| B-2287 | 2 | Me | CH₂ | H | CH₂CF₃ | CH₂CN | Cl |
| B-2288 | 2 | Me | CH₂ | Me | CH₂CF₃ | CH₂CN | Cl |
| B-2289 | 2 | H | CH(Me) | H | CH₂CF₃ | CH₂CN | Cl |
| B-2290 | 2 | H | CH(Me) | Me | CH₂CF₃ | CH₂CN | Cl |
| B-2291 | 2 | Me | CH(Me) | H | CH₂CF₃ | CH₂CN | Cl |
| B-2292 | 2 | Me | CH(Me) | Me | CH₂CF₃ | CH₂CN | Cl |
| B-2293 | 2 | H | CH₂CH₂ | Me | CH₂CF₃ | CH₂CN | Cl |
| B-2294 | 2 | Me | CH₂CH₂ | H | CH₂CF₃ | CH₂CN | Cl |
| B-2295 | 2 | Me | CH₂CH₂ | Me | CH₂CF₃ | CH₂CN | Cl |
| B-2296 | 2 | H | CH₂CH(Me) | H | CH₂CF₃ | CH₂CN | Cl |
| B-2297 | 2 | H | CH₂CH(Me) | Me | CH₂CF₃ | CH₂CN | Cl |
| B-2298 | 2 | Me | CH₂CH(Me) | H | CH₂CF₃ | CH₂CN | Cl |
| B-2299 | 2 | Me | CH₂CH(Me) | Me | CH₂CF₃ | CH₂CN | Cl |
| B-2300 | 2 | H | CH(Me)CH₂ | H | CH₂CF₃ | CH₂CN | Cl |
| B-2301 | 2 | H | CH(Me)CH₂ | Me | CH₂CF₃ | CH₂CN | Cl |
| B-2302 | 2 | Me | CH(Me)CH₂ | H | CH₂CF₃ | CH₂CN | Cl |
| B-2303 | 2 | Me | CH(Me)CH₂ | Me | CH₂CF₃ | CH₂CN | Cl |
| B-2304 | 2 | H | CH₂CH₂CH₂ | Me | CH₂CF₃ | CH₂CN | Cl |
| B-2305 | 2 | Me | CH₂CH₂CH₂ | H | CH₂CF₃ | CH₂CN | Cl |
| B-2306 | 2 | Me | CH₂CH₂CH₂ | Me | CH₂CF₃ | CH₂CN | Cl |
| B-2307 | 2 | H | CH₂CH₂CH(Me) | H | CH₂CF₃ | CH₂CN | Cl |
| B-2308 | 2 | H | CH₂CH₂CH(Me) | Me | CH₂CF₃ | CH₂CN | Cl |
| B-2309 | 2 | Me | CH₂CH₂CH(Me) | H | CH₂CF₃ | CH₂CN | Cl |
| B-2310 | 2 | Me | CH₂CH₂CH(Me) | Me | CH₂CF₃ | CH₂CN | Cl |
| B-2311 | 2 | H | CH₂CH(Me)CH₂ | H | CH₂CF₃ | CH₂CN | Cl |
| B-2312 | 2 | H | CH₂CH(Me)CH₂ | Me | CH₂CF₃ | CH₂CN | Cl |
| B-2313 | 2 | Me | CH₂CH(Me)CH₂ | H | CH₂CF₃ | CH₂CN | Cl |
| B-2314 | 2 | Me | CH₂CH(Me)CH₂ | Me | CH₂CF₃ | CH₂CN | Cl |
| B-2315 | 2 | H | CH(Me)CH₂CH₂ | H | CH₂CF₃ | CH₂CN | Cl |
| B-2316 | 2 | H | CH(Me)CH₂CH₂ | Me | CH₂CF₃ | CH₂CN | Cl |
| B-2317 | 2 | Me | CH(Me)CH₂CH₂ | H | CH₂CF₃ | CH₂CN | Cl |
| B-2318 | 2 | Me | CH(Me)CH₂CH₂ | Me | CH₂CF₃ | CH₂CN | Cl |
| B-2319 | 2 | H | single bond | Me | CH₂CF₃ | CH₂CN | Me |
| B-2320 | 2 | H | single bond | Et | CH₂CF₃ | CH₂CN | Me |
| B-2321 | 2 | Me | single bond | H | CH₂CF₃ | CH₂CN | Me |
| B-2322 | 2 | Et | single bond | H | CH₂CF₃ | CH₂CN | Me |
| B-2323 | 2 | Me | single bond | Me | CH₂CF₃ | CH₂CN | Me |
| B-2324 | 2 | H | CH₂ | Me | CH₂CF₃ | CH₂CN | Me |
| B-2325 | 2 | Me | CH₂ | H | CH₂CF₃ | CH₂CN | Me |
| B-2326 | 2 | Me | CH₂ | Me | CH₂CF₃ | CH₂CN | Me |

**[Table 217]**

| Compound Number | n | Y | W | Z | R¹ | R² | R³ |
|---|---|---|---|---|---|---|---|
| B-2327 | 2 | H | CH(Me) | H | CH₂CF₃ | CH₂CN | Me |
| B-2328 | 2 | H | CH(Me) | Me | CH₂CF₃ | CH₂CN | Me |
| B-2329 | 2 | Me | CH(Me) | H | CH₂CF₃ | CH₂CN | Me |
| B-2330 | 2 | Me | CH(Me) | Me | CH₂CF₃ | CH₂CN | Me |
| B-2331 | 2 | H | CH₂CH₂ | Me | CH₂CF₃ | CH₂CN | Me |
| B-2332 | 2 | Me | CH₂CH₂ | H | CH₂CF₃ | CH₂CN | Me |
| B-2333 | 2 | Me | CH₂CH₂ | Me | CH₂CF₃ | CH₂CN | Me |
| B-2334 | 2 | H | CH₂CH(Me) | H | CH₂CF₃ | CH₂CN | Me |
| B-2335 | 2 | H | CH₂CH(Me) | Me | CH₂CF₃ | CH₂CN | Me |
| B-2336 | 2 | Me | CH₂CH(Me) | H | CH₂CF₃ | CH₂CN | Me |
| B-2337 | 2 | Me | CH₂CH(Me) | Me | CH₂CF₃ | CH₂CN | Me |
| B-2338 | 2 | H | CH(Me)CH₂ | H | CH₂CF₃ | CH₂CN | Me |
| B-2339 | 2 | H | CH(Me)CH₂ | Me | CH₂CF₃ | CH₂CN | Me |
| B-2340 | 2 | Me | CH(Me)CH₂ | H | CH₂CF₃ | CH₂CN | Me |
| B-2341 | 2 | Me | CH(Me)CH₂ | Me | CH₂CF₃ | CH₂CN | Me |
| B-2342 | 2 | H | CH₂CH₂CH₂ | Me | CH₂CF₃ | CH₂CN | Me |
| B-2343 | 2 | Me | CH₂CH₂CH₂ | H | CH₂CF₃ | CH₂CN | Me |
| B-2344 | 2 | Me | CH₂CH₂CH₂ | Me | CH₂CF₃ | CH₂CN | Me |
| B-2345 | 2 | H | CH₂CH₂CH(Me) | H | CH₂CF₃ | CH₂CN | Me |
| B-2346 | 2 | H | CH₂CH₂CH(Me) | Me | CH₂CF₃ | CH₂CN | Me |
| B-2347 | 2 | Me | CH₂CH₂CH(Me) | H | CH₂CF₃ | CH₂CN | Me |
| B-2348 | 2 | Me | CH₂CH₂CH(Me) | Me | CH₂CF₃ | CH₂CN | Me |
| B-2349 | 2 | H | CH₂CH(Me)CH₂ | H | CH₂CF₃ | CH₂CN | Me |
| B-2350 | 2 | H | CH₂CH(Me)CH₂ | Me | CH₂CF₃ | CH₂CN | Me |
| B-2351 | 2 | Me | CH₂CH(Me)CH₂ | H | CH₂CF₃ | CH₂CN | Me |
| B-2352 | 2 | Me | CH₂CH(Me)CH₂ | Me | CH₂CF₃ | CH₂CN | Me |
| B-2353 | 2 | H | CH(Me)CH₂CH₂ | H | CH₂CF₃ | CH₂CN | Me |
| B-2354 | 2 | H | CH(Me)CH₂CH₂ | Me | CH₂CF₃ | CH₂CN | Me |
| B-2355 | 2 | Me | CH(Me)CH₂CH₂ | H | CH₂CF₃ | CH₂CN | Me |
| B-2356 | 2 | Me | CH(Me)CH₂CH₂ | Me | CH₂CF₃ | CH₂CN | Me |
| B-2357 | 2 | H | single bond | Me | CH₂CF₃ | C(=O)Me | Cl |
| B-2358 | 2 | H | single bond | Et | CH₂CF₃ | C(=O)Me | Cl |
| B-2359 | 2 | Me | single bond | H | CH₂CF₃ | C(=O)Me | Cl |
| B-2360 | 2 | Et | single bond | H | CH₂CF₃ | C(=O)Me | Cl |
| B-2361 | 2 | Me | single bond | Me | CH₂CF₃ | C(=O)Me | Cl |
| B-2362 | 2 | H | CH₂ | Me | CH₂CF₃ | C(=O)Me | Cl |
| B-2363 | 2 | Me | CH₂ | H | CH₂CF₃ | C(=O)Me | Cl |
| B-2364 | 2 | Me | CH₂ | Me | CH₂CF₃ | C(=O)Me | Cl |
| B-2365 | 2 | H | CH(Me) | H | CH₂CF₃ | C(=O)Me | Cl |
| B-2366 | 2 | H | CH(Me) | Me | CH₂CF₃ | C(=O)Me | Cl |
| B-2367 | 2 | Me | CH(Me) | H | CH₂CF₃ | C(=O)Me | Cl |
| B-2368 | 2 | Me | CH(Me) | Me | CH₂CF₃ | C(=O)Me | Cl |
| B-2369 | 2 | H | CH₂CH₂ | Me | CH₂CF₃ | C(=O)Me | Cl |
| B-2370 | 2 | Me | CH₂CH₂ | H | CH₂CF₃ | C(=O)Me | Cl |

**[Table 218]**

| Compound Number | n | Y | W | Z | R¹ | R² | R³ |
|---|---|---|---|---|---|---|---|
| B-2371 | 2 | Me | CH₂CH₂ | Me | CH₂CF₃ | C(=O)Me | Cl |
| B-2372 | 2 | H | CH₂CH(Me) | H | CH₂CF₃ | C(=O)Me | Cl |
| B-2373 | 2 | H | CH₂CH(Me) | Me | CH₂CF₃ | C(=O)Me | Cl |
| B-2374 | 2 | Me | CH₂CH(Me) | H | CH₂CF₃ | C(=O)Me | Cl |
| B-2375 | 2 | Me | CH₂CH(Me) | Me | CH₂CF₃ | C(=O)Me | Cl |
| B-2376 | 2 | H | CH(Me)CH₂ | H | CH₂CF₃ | C(=O)Me | Cl |
| B-2377 | 2 | H | CH(Me)CH₂ | Me | CH₂CF₃ | C(=O)Me | Cl |
| B-2378 | 2 | Me | CH(Me)CH₂ | H | CH₂CF₃ | C(=O)Me | Cl |
| B-2379 | 2 | Me | CH(Me)CH₂ | Me | CH₂CF₃ | C(=O)Me | Cl |
| B-2380 | 2 | H | CH₂CH₂CH₂ | Me | CH₂CF₃ | C(=O)Me | Cl |
| B-2381 | 2 | Me | CH₂CH₂CH₂ | H | CH₂CF₃ | C(=O)Me | Cl |
| B-2382 | 2 | Me | CH₂CH₂CH₂ | Me | CH₂CF₃ | C(=O)Me | Cl |
| B-2383 | 2 | H | CH₂CH₂CH(Me) | H | CH₂CF₃ | C(=O)Me | Cl |
| B-2384 | 2 | H | CH₂CH₂CH(Me) | Me | CH₂CF₃ | C(=O)Me | Cl |
| B-2385 | 2 | Me | CH₂CH₂CH(Me) | H | CH₂CF₃ | C(=O)Me | Cl |
| B-2386 | 2 | Me | CH₂CH₂CH(Me) | Me | CH₂CF₃ | C(=O)Me | Cl |
| B-2387 | 2 | H | CH₂CH(Me)CH₂ | H | CH₂CF₃ | C(=O)Me | Cl |
| B-2388 | 2 | H | CH₂CH(Me)CH₂ | Me | CH₂CF₃ | C(=O)Me | Cl |
| B-2389 | 2 | Me | CH₂CH(Me)CH₂ | H | CH₂CF₃ | C(=O)Me | Cl |
| B-2390 | 2 | Me | CH₂CH(Me)CH₂ | Me | CH₂CF₃ | C(=O)Me | Cl |
| B-2391 | 2 | H | CH(Me)CH₂CH₂ | H | CH₂CF₃ | C(=O)Me | Cl |
| B-2392 | 2 | H | CH(Me)CH₂CH₂ | Me | CH₂CF₃ | C(=O)Me | Cl |
| B-2393 | 2 | Me | CH(Me)CH₂CH₂ | H | CH₂CF₃ | C(=O)Me | Cl |
| B-2394 | 2 | Me | CH(Me)CH₂CH₂ | Me | CH₂CF₃ | C(=O)Me | Cl |
| B-2395 | 2 | H | single bond | Me | CH₂CF₃ | C(=O)Me | Me |
| B-2396 | 2 | H | single bond | Et | CH₂CF₃ | C(=O)Me | Me |
| B-2397 | 2 | Me | single bond | H | CH₂CF₃ | C(=O)Me | Me |
| B-2398 | 2 | Et | single bond | H | CH₂CF₃ | C(=O)Me | Me |
| B-2399 | 2 | Me | single bond | Me | CH₂CF₃ | C(=O)Me | Me |
| B-2400 | 2 | H | CH₂ | Me | CH₂CF₃ | C(=O)Me | Me |
| B-2401 | 2 | Me | CH₂ | H | CH₂CF₃ | C(=O)Me | Me |
| B-2402 | 2 | Me | CH₂ | Me | CH₂CF₃ | C(=O)Me | Me |
| B-2403 | 2 | H | CH(Me) | H | CH₂CF₃ | C(=O)Me | Me |
| B-2404 | 2 | H | CH(Me) | Me | CH₂CF₃ | C(=O)Me | Me |
| B-2405 | 2 | Me | CH(Me) | H | CH₂CF₃ | C(=O)Me | Me |
| B-2406 | 2 | Me | CH(Me) | Me | CH₂CF₃ | C(=O)Me | Me |
| B-2407 | 2 | H | CH₂CH₂ | Me | CH₂CF₃ | C(=O)Me | Me |
| B-2408 | 2 | Me | CH₂CH₂ | H | CH₂CF₃ | C(=O)Me | Me |
| B-2409 | 2 | Me | CH₂CH₂ | Me | CH₂CF₃ | C(=O)Me | Me |
| B-2410 | 2 | H | CH₂CH(Me) | H | CH₂CF₃ | C(=O)Me | Me |
| B-2411 | 2 | H | CH₂CH(Me) | Me | CH₂CF₃ | C(=O)Me | Me |
| B-2412 | 2 | Me | CH₂CH(Me) | H | CH₂CF₃ | C(=O)Me | Me |
| B-2413 | 2 | Me | CH₂CH(Me) | Me | CH₂CF₃ | C(=O)Me | Me |
| B-2414 | 2 | H | CH(Me)CH₂ | H | CH₂CF₃ | C(=O)Me | Me |

**[Table 219]**

| Compound Number | n | Y | W | Z | R¹ | R² | R³ |
|---|---|---|---|---|---|---|---|
| B-2415 | 2 | H | CH(Me)CH₂ | Me | CH₂CF₃ | C(=O)Me | Me |
| B-2416 | 2 | Me | CH(Me)CH₂ | H | CH₂CF₃ | C(=O)Me | Me |
| B-2417 | 2 | Me | CH(Me)CH₂ | Me | CH₂CF₃ | C(=O)Me | Me |
| B-2418 | 2 | H | CH₂CH₂CH₂ | Me | CH₂CF₃ | C(=O)Me | Me |
| B-2419 | 2 | Me | CH₂CH₂CH₂ | H | CH₂CF₃ | C(=O)Me | Me |
| B-2420 | 2 | Me | CH₂CH₂CH₂ | Me | CH₂CF₃ | C(=O)Me | Me |
| B-2421 | 2 | H | CH₂CH₂CH(Me) | H | CH₂CF₃ | C(=O)Me | Me |
| B-2422 | 2 | H | CH₂CH₂CH(Me) | Me | CH₂CF₃ | C(=O)Me | Me |
| B-2423 | 2 | Me | CH₂CH₂CH(Me) | H | CH₂CF₃ | C(=O)Me | Me |
| B-2424 | 2 | Me | CH₂CH₂CH(Me) | Me | CH₂CF₃ | C(=O)Me | Me |
| B-2425 | 2 | H | CH₂CH(Me)CH₂ | H | CH₂CF₃ | C(=O)Me | Me |
| B-2426 | 2 | H | CH₂CH(Me)CH₂ | Me | CH₂CF₃ | C(=O)Me | Me |
| B-2427 | 2 | Me | CH₂CH(Me)CH₂ | H | CH₂CF₃ | C(=O)Me | Me |
| B-2428 | 2 | Me | CH₂CH(Me)CH₂ | Me | CH₂CF₃ | C(=O)Me | Me |
| B-2429 | 2 | H | CH(Me)CH₂CH₂ | H | CH₂CF₃ | C(=O)Me | Me |
| B-2430 | 2 | H | CH(Me)CH₂CH₂ | Me | CH₂CF₃ | C(=O)Me | Me |
| B-2431 | 2 | Me | CH(Me)CH₂CH₂ | H | CH₂CF₃ | C(=O)Me | Me |
| B-2432 | 2 | Me | CH(Me)CH₂CH₂ | Me | CH₂CF₃ | C(=O)Me | Me |
| B-2433 | 2 | H | single bond | Me | CH₂CF₃ | C(=O)Ph | Cl |
| B-2434 | 2 | H | single bond | Et | CH₂CF₃ | C(=O)Ph | Cl |
| B-2435 | 2 | Me | single bond | H | CH₂CF₃ | C(=O)Ph | Cl |
| B-2436 | 2 | Et | single bond | H | CH₂CF₃ | C(=O)Ph | Cl |
| B-2437 | 2 | Me | single bond | Me | CH₂CF₃ | C(=O)Ph | Cl |
| B-2438 | 2 | H | CH₂ | Me | CH₂CF₃ | C(=O)Ph | Cl |
| B-2439 | 2 | Me | CH₂ | H | CH₂CF₃ | C(=O)Ph | Cl |
| B-2440 | 2 | Me | CH₂ | Me | CH₂CF₃ | C(=O)Ph | Cl |
| B-2441 | 2 | H | CH(Me) | H | CH₂CF₃ | C(=O)Ph | Cl |
| B-2442 | 2 | H | CH(Me) | Me | CH₂CF₃ | C(=O)Ph | Cl |
| B-2443 | 2 | Me | CH(Me) | H | CH₂CF₃ | C(=O)Ph | Cl |
| B-2444 | 2 | Me | CH(Me) | Me | CH₂CF₃ | C(=O)Ph | Cl |
| B-2445 | 2 | H | CH₂CH₂ | Me | CH₂CF₃ | C(=O)Ph | Cl |
| B-2446 | 2 | Me | CH₂CH₂ | H | CH₂CF₃ | C(=O)Ph | Cl |
| B-2447 | 2 | Me | CH₂CH₂ | Me | CH₂CF₃ | C(=O)Ph | Cl |
| B-2448 | 2 | H | CH₂CH(Me) | H | CH₂CF₃ | C(=O)Ph | Cl |
| B-2449 | 2 | H | CH₂CH(Me) | Me | CH₂CF₃ | C(=O)Ph | Cl |
| B-2450 | 2 | Me | CH₂CH(Me) | H | CH₂CF₃ | C(=O)Ph | Cl |
| B-2451 | 2 | Me | CH₂CH(Me) | Me | CH₂CF₃ | C(=O)Ph | Cl |
| B-2452 | 2 | H | CH(Me)CH₂ | H | CH₂CF₃ | C(=O)Ph | Cl |
| B-2453 | 2 | H | CH(Me)CH₂ | Me | CH₂CF₃ | C(=O)Ph | Cl |
| B-2454 | 2 | Me | CH(Me)CH₂ | H | CH₂CF₃ | C(=O)Ph | Cl |
| B-2455 | 2 | Me | CH(Me)CH₂ | Me | CH₂CF₃ | C(=O)Ph | Cl |
| B-2456 | 2 | H | CH₂CH₂CH₂ | Me | CH₂CF₃ | C(=O)Ph | Cl |
| B-2457 | 2 | Me | CH₂CH₂CH₂ | H | CH₂CF₃ | C(=O)Ph | Cl |
| B-2458 | 2 | Me | CH₂CH₂CH₂ | Me | CH₂CF₃ | C(=O)Ph | Cl |

**[Table 220]**

| Compound Number | n | Y | W | Z | R¹ | R² | R³ |
|---|---|---|---|---|---|---|---|
| B-2459 | 2 | H | CH₂CH₂CH(Me) | H | CH₂CF₃ | C(=O)Ph | Cl |
| B-2460 | 2 | H | CH₂CH₂CH(Me) | Me | CH₂CF₃ | C(=O)Ph | Cl |
| B-2461 | 2 | Me | CH₂CH₂CH(Me) | H | CH₂CF₃ | C(=O)Ph | Cl |
| B-2462 | 2 | Me | CH₂CH₂CH(Me) | Me | CH₂CF₃ | C(=O)Ph | Cl |
| B-2463 | 2 | H | CH₂CH(Me)CH₂ | H | CH₂CF₃ | C(=O)Ph | Cl |
| B-2464 | 2 | H | CH₂CH(Me)CH₂ | Me | CH₂CF₃ | C(=O)Ph | Cl |
| B-2465 | 2 | Me | CH₂CH(Me)CH₂ | H | CH₂CF₃ | C(=O)Ph | Cl |
| B-2466 | 2 | Me | CH₂CH(Me)CH₂ | Me | CH₂CF₃ | C(=O)Ph | Cl |
| B-2467 | 2 | H | CH(Me)CH₂CH₂ | H | CH₂CF₃ | C(=O)Ph | Cl |
| B-2468 | 2 | H | CH(Me)CH₂CH₂ | Me | CH₂CF₃ | C(=O)Ph | Cl |
| B-2469 | 2 | Me | CH(Me)CH₂CH₂ | H | CH₂CF₃ | C(=O)Ph | Cl |
| B-2470 | 2 | Me | CH(Me)CH₂CH₂ | Me | CH₂CF₃ | C(=O)Ph | Cl |
| B-2471 | 2 | H | single bond | Me | CH₂CF₃ | C(=O)Ph | Me |
| B-2472 | 2 | H | single bond | Et | CH₂CF₃ | C(=O)Ph | Me |
| B-2473 | 2 | Me | single bond | H | CH₂CF₃ | C(=O)Ph | Me |
| B-2474 | 2 | Et | single bond | H | CH₂CF₃ | C(=O)Ph | Me |
| B-2475 | 2 | Me | single bond | Me | CH₂CF₃ | C(=O)Ph | Me |
| B-2476 | 2 | H | CH₂ | Me | CH₂CF₃ | C(=O)Ph | Me |
| B-2477 | 2 | Me | CH₂ | H | CH₂CF₃ | C(=O)Ph | Me |
| B-2478 | 2 | Me | CH₂ | Me | CH₂CF₃ | C(=O)Ph | Me |
| B-2479 | 2 | H | CH(Me) | H | CH₂CF₃ | C(=O)Ph | Me |
| B-2480 | 2 | H | CH(Me) | Me | CH₂CF₃ | C(=O)Ph | Me |
| B-2481 | 2 | Me | CH(Me) | H | CH₂CF₃ | C(=O)Ph | Me |
| B-2482 | 2 | Me | CH(Me) | Me | CH₂CF₃ | C(=O)Ph | Me |
| B-2483 | 2 | H | CH₂CH₂ | Me | CH₂CF₃ | C(=O)Ph | Me |
| B-2484 | 2 | Me | CH₂CH₂ | H | CH₂CF₃ | C(=O)Ph | Me |
| B-2485 | 2 | Me | CH₂CH₂ | Me | CH₂CF₃ | C(=O)Ph | Me |
| B-2486 | 2 | H | CH₂CH(Me) | H | CH₂CF₃ | C(=O)Ph | Me |
| B-2487 | 2 | H | CH₂CH(Me) | Me | CH₂CF₃ | C(=O)Ph | Me |
| B-2488 | 2 | Me | CH₂CH(Me) | H | CH₂CF₃ | C(=O)Ph | Me |
| B-2489 | 2 | Me | CH₂CH(Me) | Me | CH₂CF₃ | C(=O)Ph | Me |
| B-2490 | 2 | H | CH(Me)CH₂ | H | CH₂CF₃ | C(=O)Ph | Me |
| B-2491 | 2 | H | CH(Me)CH₂ | Me | CH₂CF₃ | C(=O)Ph | Me |
| B-2492 | 2 | Me | CH(Me)CH₂ | H | CH₂CF₃ | C(=O)Ph | Me |
| B-2493 | 2 | Me | CH(Me)CH₂ | Me | CH₂CF₃ | C(=O)Ph | Me |
| B-2494 | 2 | H | CH₂CH₂CH₂ | Me | CH₂CF₃ | C(=O)Ph | Me |
| B-2495 | 2 | Me | CH₂CH₂CH₂ | H | CH₂CF₃ | C(=O)Ph | Me |
| B-2496 | 2 | Me | CH₂CH₂CH₂ | Me | CH₂CF₃ | C(=O)Ph | Me |
| B-2497 | 2 | H | CH₂CH₂CH(Me) | H | CH₂CF₃ | C(=O)Ph | Me |
| B-2498 | 2 | H | CH₂CH₂CH(Me) | Me | CH₂CF₃ | C(=O)Ph | Me |
| B-2499 | 2 | Me | CH₂CH₂CH(Me) | H | CH₂CF₃ | C(=O)Ph | Me |
| B-2500 | 2 | Me | CH₂CH₂CH(Me) | Me | CH₂CF₃ | C(=O)Ph | Me |
| B-2501 | 2 | H | CH₂CH(Me)CH₂ | H | CH₂CF₃ | C(=O)Ph | Me |
| B-2502 | 2 | H | CH₂CH(Me)CH₂ | Me | CH₂CF₃ | C(=O)Ph | Me |

**[Table 221]**

| Compound Number | n | Y | W | Z | R¹ | R² | R³ |
|---|---|---|---|---|---|---|---|
| B-2503 | 2 | Me | CH₂CH(Me)CH₂ | H | CH₂CF₃ | C(=O)Ph | Me |
| B-2504 | 2 | Me | CH₂CH(Me)CH₂ | Me | CH₂CF₃ | C(=O)Ph | Me |
| B-2505 | 2 | H | CH(Me)CH₂CH₂ | H | CH₂CF₃ | C(=O)Ph | Me |
| B-2506 | 2 | H | CH(Me)CH₂CH₂ | Me | CH₂CF₃ | C(=O)Ph | Me |
| B-2507 | 2 | Me | CH(Me)CH₂CH₂ | H | CH₂CF₃ | C(=O)Ph | Me |
| B-2508 | 2 | Me | CH(Me)CH₂CH₂ | Me | CH₂CF₃ | C(=O)Ph | Me |
| B-2509 | 2 | H | single bond | Me | CH₂CF₃ | C(=O)OEt | Cl |
| B-2510 | 2 | H | single bond | Et | CH₂CF₃ | C(=O)OEt | Cl |
| B-2511 | 2 | Me | single bond | H | CH₂CF₃ | C(=O)OEt | Cl |
| B-2512 | 2 | Et | single bond | H | CH₂CF₃ | C(=O)OEt | Cl |
| B-2513 | 2 | Me | single bond | Me | CH₂CF₃ | C(=O)OEt | Cl |
| B-2514 | 2 | H | CH₂ | Me | CH₂CF₃ | C(=O)OEt | Cl |
| B-2515 | 2 | Me | CH₂ | H | CH₂CF₃ | C(=O)OEt | Cl |
| B-2516 | 2 | Me | CH₂ | Me | CH₂CF₃ | C(=O)OEt | Cl |
| B-2517 | 2 | H | CH(Me) | H | CH₂CF₃ | C(=O)OEt | Cl |
| B-2518 | 2 | H | CH(Me) | Me | CH₂CF₃ | C(=O)OEt | Cl |
| B-2519 | 2 | Me | CH(Me) | H | CH₂CF₃ | C(=O)OEt | Cl |
| B-2520 | 2 | Me | CH(Me) | Me | CH₂CF₃ | C(=O)OEt | Cl |
| B-2521 | 2 | H | CH₂CH₂ | Me | CH₂CF₃ | C(=O)OEt | Cl |
| B-2522 | 2 | Me | CH₂CH₂ | H | CH₂CF₃ | C(=O)OEt | Cl |
| B-2523 | 2 | Me | CH₂CH₂ | Me | CH₂CF₃ | C(=O)OEt | Cl |
| B-2524 | 2 | H | CH₂CH(Me) | H | CH₂CF₃ | C(=O)OEt | Cl |
| B-2525 | 2 | H | CH₂CH(Me) | Me | CH₂CF₃ | C(=O)OEt | Cl |
| B-2526 | 2 | Me | CH₂CH(Me) | H | CH₂CF₃ | C(=O)OEt | Cl |
| B-2527 | 2 | Me | CH₂CH(Me) | Me | CH₂CF₃ | C(=O)OEt | Cl |
| B-2528 | 2 | H | CH(Me)CH₂ | H | CH₂CF₃ | C(=O)OEt | Cl |
| B-2529 | 2 | H | CH(Me)CH₂ | Me | CH₂CF₃ | C(=O)OEt | Cl |
| B-2530 | 2 | Me | CH(Me)CH₂ | H | CH₂CF₃ | C(=O)OEt | Cl |
| B-2531 | 2 | Me | CH(Me)CH₂ | Me | CH₂CF₃ | C(=O)OEt | Cl |
| B-2532 | 2 | H | CH₂CH₂CH₂ | Me | CH₂CF₃ | C(=O)OEt | Cl |
| B-2533 | 2 | Me | CH₂CH₂CH₂ | H | CH₂CF₃ | C(=O)OEt | Cl |
| B-2534 | 2 | Me | CH₂CH₂CH₂ | Me | CH₂CF₃ | C(=O)OEt | Cl |
| B-2535 | 2 | H | CH₂CH₂CH(Me) | H | CH₂CF₃ | C(=O)OEt | Cl |
| B-2536 | 2 | H | CH₂CH₂CH(Me) | Me | CH₂CF₃ | C(=O)OEt | Cl |
| B-2537 | 2 | Me | CH₂CH₂CH(Me) | H | CH₂CF₃ | C(=O)OEt | Cl |
| B-2538 | 2 | Me | CH₂CH₂CH(Me) | Me | CH₂CF₃ | C(=O)OEt | Cl |
| B-2539 | 2 | H | CH₂CH(Me)CH₂ | H | CH₂CF₃ | C(=O)OEt | Cl |
| B-2540 | 2 | H | CH₂CH(Me)CH₂ | Me | CH₂CF₃ | C(=O)OEt | Cl |
| B-2541 | 2 | Me | CH₂CH(Me)CH₂ | H | CH₂CF₃ | C(=O)OEt | Cl |
| B-2542 | 2 | Me | CH₂CH(Me)CH₂ | Me | CH₂CF₃ | C(=O)OEt | Cl |
| B-2543 | 2 | H | CH(Me)CH₂CH₂ | H | CH₂CF₃ | C(=O)OEt | Cl |
| B-2544 | 2 | H | CH(Me)CH₂CH₂ | Me | CH₂CF₃ | C(=O)OEt | Cl |
| B-2545 | 2 | Me | CH(Me)CH₂CH₂ | H | CH₂CF₃ | C(=O)OEt | Cl |
| B-2546 | 2 | Me | CH(Me)CH₂CH₂ | Me | CH₂CF₃ | C(=O)OEt | Cl |

**[Table 222]**

| Compound Number | n | Y | W | Z | R¹ | R² | R³ |
|---|---|---|---|---|---|---|---|
| B-2547 | 2 | H | single bond | Me | CH₂CF₃ | C(=O)OEt | Me |
| B-2548 | 2 | H | single bond | Et | CH₂CF₃ | C(=O)OEt | Me |
| B-2549 | 2 | Me | single bond | H | CH₂CF₃ | C(=O)OEt | Me |
| B-2550 | 2 | Et | single bond | H | CH₂CF₃ | C(=O)OEt | Me |
| B-2551 | 2 | Me | single bond | Me | CH₂CF₃ | C(=O)OEt | Me |
| B-2552 | 2 | H | CH₂ | Me | CH₂CF₃ | C(=O)OEt | Me |
| B-2553 | 2 | Me | CH₂ | H | CH₂CF₃ | C(=O)OEt | Me |
| B-2554 | 2 | Me | CH₂ | Me | CH₂CF₃ | C(=O)OEt | Me |
| B-2555 | 2 | H | CH(Me) | H | CH₂CF₃ | C(=O)OEt | Me |
| B-2556 | 2 | H | CH(Me) | Me | CH₂CF₃ | C(=O)OEt | Me |
| B-2557 | 2 | Me | CH(Me) | H | CH₂CF₃ | C(=O)OEt | Me |
| B-2558 | 2 | Me | CH(Me) | Me | CH₂CF₃ | C(=O)OEt | Me |
| B-2559 | 2 | H | CH₂CH₂ | Me | CH₂CF₃ | C(=O)OEt | Me |
| B-2560 | 2 | Me | CH₂CH₂ | H | CH₂CF₃ | C(=O)OEt | Me |
| B-2561 | 2 | Me | CH₂CH₂ | Me | CH₂CF₃ | C(=O)OEt | Me |
| B-2562 | 2 | H | CH₂CH(Me) | H | CH₂CF₃ | C(=O)OEt | Me |
| B-2563 | 2 | H | CH₂CH(Me) | Me | CH₂CF₃ | C(=O)OEt | Me |
| B-2564 | 2 | Me | CH₂CH(Me) | H | CH₂CF₃ | C(=O)OEt | Me |
| B-2565 | 2 | Me | CH₂CH(Me) | Me | CH₂CF₃ | C(=O)OEt | Me |
| B-2566 | 2 | H | CH(Me)CH₂ | H | CH₂CF₃ | C(=O)OEt | Me |
| B-2567 | 2 | H | CH(Me)CH₂ | Me | CH₂CF₃ | C(=O)OEt | Me |
| B-2568 | 2 | Me | CH(Me)CH₂ | H | CH₂CF₃ | C(=O)OEt | Me |
| B-2569 | 2 | Me | CH(Me)CH₂ | Me | CH₂CF₃ | C(=O)OEt | Me |
| B-2570 | 2 | H | CH₂CH₂CH₂ | Me | CH₂CF₃ | C(=O)OEt | Me |
| B-2571 | 2 | Me | CH₂CH₂CH₂ | H | CH₂CF₃ | C(=O)OEt | Me |
| B-2572 | 2 | Me | CH₂CH₂CH₂ | Me | CH₂CF₃ | C(=O)OEt | Me |
| B-2573 | 2 | H | CH₂CH₂CH(Me) | H | CH₂CF₃ | C(=O)OEt | Me |
| B-2574 | 2 | H | CH₂CH₂CH(Me) | Me | CH₂CF₃ | C(=O)OEt | Me |
| B-2575 | 2 | Me | CH₂CH₂CH(Me) | H | CH₂CF₃ | C(=O)OEt | Me |
| B-2576 | 2 | Me | CH₂CH₂CH(Me) | Me | CH₂CF₃ | C(=O)OEt | Me |
| B-2577 | 2 | H | CH₂CH(Me)CH₂ | H | CH₂CF₃ | C(=O)OEt | Me |
| B-2578 | 2 | H | CH₂CH(Me)CH₂ | Me | CH₂CF₃ | C(=O)OEt | Me |
| B-2579 | 2 | Me | CH₂CH(Me)CH₂ | H | CH₂CF₃ | C(=O)OEt | Me |
| B-2580 | 2 | Me | CH₂CH(Me)CH₂ | Me | CH₂CF₃ | C(=O)OEt | Me |
| B-2581 | 2 | H | CH(Me)CH₂CH₂ | H | CH₂CF₃ | C(=O)OEt | Me |
| B-2582 | 2 | H | CH(Me)CH₂CH₂ | Me | CH₂CF₃ | C(=O)OEt | Me |
| B-2583 | 2 | Me | CH(Me)CH₂CH₂ | H | CH₂CF₃ | C(=O)OEt | Me |
| B-2584 | 2 | Me | CH(Me)CH₂CH₂ | Me | CH₂CF₃ | C(=O)OEt | Me |
| B-2585 | 2 | H | single bond | Me | CH₂CF₃ | S(=O)₂Me | Cl |
| B-2586 | 2 | H | single bond | Et | CH₂CF₃ | S(=O)₂Me | Cl |
| B-2587 | 2 | Me | single bond | H | CH₂CF₃ | S(=O)₂Me | Cl |
| B-2588 | 2 | Et | single bond | H | CH₂CF₃ | S(=O)₂Me | Cl |
| B-2589 | 2 | Me | single bond | Me | CH₂CF₃ | S(=O)₂Me | Cl |
| B-2590 | 2 | H | CH₂ | Me | CH₂CF₃ | S(=O)₂Me | Cl |

**[Table 223]**

| Compound Number | n | Y | W | Z | R¹ | R² | R³ |
|---|---|---|---|---|---|---|---|
| B-2591 | 2 | Me | CH₂ | H | CH₂CF₃ | S(=O)₂Me | Cl |
| B-2592 | 2 | Me | CH₂ | Me | CH₂CF₃ | S(=O)₂Me | Cl |
| B-2593 | 2 | H | CH(Me) | H | CH₂CF₃ | S(=O)₂Me | Cl |
| B-2594 | 2 | H | CH(Me) | Me | CH₂CF₃ | S(=O)₂Me | Cl |
| B-2595 | 2 | Me | CH(Me) | H | CH₂CF₃ | S(=O)₂Me | Cl |
| B-2596 | 2 | Me | CH(Me) | Me | CH₂CF₃ | S(=O)₂Me | Cl |
| B-2597 | 2 | H | CH₂CH₂ | Me | CH₂CF₃ | S(=O)₂Me | Cl |
| B-2598 | 2 | Me | CH₂CH₂ | H | CH₂CF₃ | S(=O)₂Me | Cl |
| B-2599 | 2 | Me | CH₂CH₂ | Me | CH₂CF₃ | S(=O)₂Me | Cl |
| B-2600 | 2 | H | CH₂CH(Me) | H | CH₂CF₃ | S(=O)₂Me | Cl |
| B-2601 | 2 | H | CH₂CH(Me) | Me | CH₂CF₃ | S(=O)₂Me | Cl |
| B-2602 | 2 | Me | CH₂CH(Me) | H | CH₂CF₃ | S(=O)₂Me | Cl |
| B-2603 | 2 | Me | CH₂CH(Me) | Me | CH₂CF₃ | S(=O)₂Me | Cl |
| B-2604 | 2 | H | CH(Me)CH₂ | H | CH₂CF₃ | S(=O)₂Me | Cl |
| B-2605 | 2 | H | CH(Me)CH₂ | Me | CH₂CF₃ | S(=O)₂Me | Cl |
| B-2606 | 2 | Me | CH(Me)CH₂ | H | CH₂CF₃ | S(=O)₂Me | Cl |
| B-2607 | 2 | Me | CH(Me)CH₂ | Me | CH₂CF₃ | S(=O)₂Me | Cl |
| B-2608 | 2 | H | CH₂CH₂CH₂ | Me | CH₂CF₃ | S(=O)₂Me | Cl |
| B-2609 | 2 | Me | CH₂CH₂CH₂ | H | CH₂CF₃ | S(=O)₂Me | Cl |
| B-2610 | 2 | Me | CH₂CH₂CH₂ | Me | CH₂CF₃ | S(=O)₂Me | Cl |
| B-2611 | 2 | H | CH₂CH₂CH(Me) | H | CH₂CF₃ | S(=O)₂Me | Cl |
| B-2612 | 2 | H | CH₂CH₂CH(Me) | Me | CH₂CF₃ | S(=O)₂Me | Cl |
| B-2613 | 2 | Me | CH₂CH₂CH(Me) | H | CH₂CF₃ | S(=O)₂Me | Cl |
| B-2614 | 2 | Me | CH₂CH₂CH(Me) | Me | CH₂CF₃ | S(=O)₂Me | Cl |
| B-2615 | 2 | H | CH₂CH(Me)CH₂ | H | CH₂CF₃ | S(=O)₂Me | Cl |
| B-2616 | 2 | H | CH₂CH(Me)CH₂ | Me | CH₂CF₃ | S(=O)₂Me | Cl |
| B-2617 | 2 | Me | CH₂CH(Me)CH₂ | H | CH₂CF₃ | S(=O)₂Me | Cl |
| B-2618 | 2 | Me | CH₂CH(Me)CH₂ | Me | CH₂CF₃ | S(=O)₂Me | Cl |
| B-2619 | 2 | H | CH(Me)CH₂CH₂ | H | CH₂CF₃ | S(=O)₂Me | Cl |
| B-2620 | 2 | H | CH(Me)CH₂CH₂ | Me | CH₂CF₃ | S(=O)₂Me | Cl |
| B-2621 | 2 | Me | CH(Me)CH₂CH₂ | H | CH₂CF₃ | S(=O)₂Me | Cl |
| B-2622 | 2 | Me | CH(Me)CH₂CH₂ | Me | CH₂CF₃ | S(=O)₂Me | Cl |
| B-2623 | 2 | H | single bond | Me | CH₂CF₃ | S(=O)₂Me | Me |
| B-2624 | 2 | H | single bond | Et | CH₂CF₃ | S(=O)₂Me | Me |
| B-2625 | 2 | Me | single bond | H | CH₂CF₃ | S(=O)₂Me | Me |
| B-2626 | 2 | Et | single bond | H | CH₂CF₃ | S(=O)₂Me | Me |
| B-2627 | 2 | Me | single bond | Me | CH₂CF₃ | S(=O)₂Me | Me |
| B-2628 | 2 | H | CH₂ | Me | CH₂CF₃ | S(=O)₂Me | Me |
| B-2629 | 2 | Me | CH₂ | H | CH₂CF₃ | S(=O)₂Me | Me |
| B-2630 | 2 | Me | CH₂ | Me | CH₂CF₃ | S(=O)₂Me | Me |
| B-2631 | 2 | H | CH(Me) | H | CH₂CF₃ | S(=O)₂Me | Me |
| B-2632 | 2 | H | CH(Me) | Me | CH₂CF₃ | S(=O)₂Me | Me |
| B-2633 | 2 | Me | CH(Me) | H | CH₂CF₃ | S(=O)₂Me | Me |
| B-2634 | 2 | Me | CH(Me) | Me | CH₂CF₃ | S(=O)₂Me | Me |

**[Table 224]**

| Compound Number | n | Y | W | Z | R¹ | R² | R³ |
|---|---|---|---|---|---|---|---|
| B-2635 | 2 | H | CH₂CH₂ | Me | CH₂CF₃ | S(=O)₂Me | Me |
| B-2636 | 2 | Me | CH₂CH₂ | H | CH₂CF₃ | S(=O)₂Me | Me |
| B-2637 | 2 | Me | CH₂CH₂ | Me | CH₂CF₃ | S(=O)₂Me | Me |
| B-2638 | 2 | H | CH₂CH(Me) | H | CH₂CF₃ | S(=O)₂Me | Me |
| B-2639 | 2 | H | CH₂CH(Me) | Me | CH₂CF₃ | S(=O)₂Me | Me |
| B-2640 | 2 | Me | CH₂CH(Me) | H | CH₂CF₃ | S(=O)₂Me | Me |
| B-2641 | 2 | Me | CH₂CH(Me) | Me | CH₂CF₃ | S(=O)₂Me | Me |
| B-2642 | 2 | H | CH(Me)CH₂ | H | CH₂CF₃ | S(=O)₂Me | Me |
| B-2643 | 2 | H | CH(Me)CH₂ | Me | CH₂CF₃ | S(=O)₂Me | Me |
| B-2644 | 2 | Me | CH(Me)CH₂ | H | CH₂CF₃ | S(=O)₂Me | Me |
| B-2645 | 2 | Me | CH(Me)CH₂ | Me | CH₂CF₃ | S(=O)₂Me | Me |
| B-2646 | 2 | H | CH₂CH₂CH₂ | Me | CH₂CF₃ | S(=O)₂Me | Me |
| B-2647 | 2 | Me | CH₂CH₂CH₂ | H | CH₂CF₃ | S(=O)₂Me | Me |
| B-2648 | 2 | Me | CH₂CH₂CH₂ | Me | CH₂CF₃ | S(=O)₂Me | Me |
| B-2649 | 2 | H | CH₂CH₂CH(Me) | H | CH₂CF₃ | S(=O)₂Me | Me |
| B-2650 | 2 | H | CH₂CH₂CH(Me) | Me | CH₂CF₃ | S(=O)₂Me | Me |
| B-2651 | 2 | Me | CH₂CH₂CH(Me) | H | CH₂CF₃ | S(=O)₂Me | Me |
| B-2652 | 2 | Me | CH₂CH₂CH(Me) | Me | CH₂CF₃ | S(=O)₂Me | Me |
| B-2653 | 2 | H | CH₂CH(Me)CH₂ | H | CH₂CF₃ | S(=O)₂Me | Me |
| B-2654 | 2 | H | CH₂CH(Me)CH₂ | Me | CH₂CF₃ | S(=O)₂Me | Me |
| B-2655 | 2 | Me | CH₂CH(Me)CH₂ | H | CH₂CF₃ | S(=O)₂Me | Me |
| B-2656 | 2 | Me | CH₂CH(Me)CH₂ | Me | CH₂CF₃ | S(=O)₂Me | Me |
| B-2657 | 2 | H | CH(Me)CH₂CH₂ | H | CH₂CF₃ | S(=O)₂Me | Me |
| B-2658 | 2 | H | CH(Me)CH₂CH₂ | Me | CH₂CF₃ | S(=O)₂Me | Me |
| B-2659 | 2 | Me | CH(Me)CH₂CH₂ | H | CH₂CF₃ | S(=O)₂Me | Me |
| B-2660 | 2 | Me | CH(Me)CH₂CH₂ | Me | CH₂CF₃ | S(=O)₂Me | Me |
| B-2661 | 2 | H | single bond | Me | CH₂CF₃ | S(=O)₂CF₃ | Cl |
| B-2662 | 2 | H | single bond | Et | CH₂CF₃ | S(=O)₂CF₃ | Cl |
| B-2663 | 2 | Me | single bond | H | CH₂CF₃ | S(=O)₂CF₃ | Cl |
| B-2664 | 2 | Et | single bond | H | CH₂CF₃ | S(=O)₂CF₃ | Cl |
| B-2665 | 2 | Me | single bond | Me | CH₂CF₃ | S(=O)₂CF₃ | Cl |
| B-2666 | 2 | H | CH₂ | Me | CH₂CF₃ | S(=O)₂CF₃ | Cl |
| B-2667 | 2 | Me | CH₂ | H | CH₂CF₃ | S(=O)₂CF₃ | Cl |
| B-2668 | 2 | Me | CH₂ | Me | CH₂CF₃ | S(=O)₂CF₃ | Cl |
| B-2669 | 2 | H | CH(Me) | H | CH₂CF₃ | S(=O)₂CF₃ | Cl |
| B-2670 | 2 | H | CH(Me) | Me | CH₂CF₃ | S(=O)₂CF₃ | Cl |
| B-2671 | 2 | Me | CH(Me) | H | CH₂CF₃ | S(=O)₂CF₃ | Cl |
| B-2672 | 2 | Me | CH(Me) | Me | CH₂CF₃ | S(=O)₂CF₃ | Cl |
| B-2673 | 2 | H | CH₂CH₂ | Me | CH₂CF₃ | S(=O)₂CF₃ | Cl |
| B-2674 | 2 | Me | CH₂CH₂ | H | CH₂CF₃ | S(=O)₂CF₃ | Cl |
| B-2675 | 2 | Me | CH₂CH₂ | Me | CH₂CF₃ | S(=O)₂CF₃ | Cl |
| B-2676 | 2 | H | CH₂CH(Me) | H | CH₂CF₃ | S(=O)₂CF₃ | Cl |
| B-2677 | 2 | H | CH₂CH(Me) | Me | CH₂CF₃ | S(=O)₂CF₃ | Cl |
| B-2678 | 2 | Me | CH₂CH(Me) | H | CH₂CF₃ | S(=O)₂CF₃ | Cl |

**[Table 225]**

| Compound Number | n | Y | W | Z | R¹ | R² | R³ |
|---|---|---|---|---|---|---|---|
| B-2679 | 2 | Me | CH₂CH(Me) | Me | CH₂CF₃ | S(=O)₂CF₃ | Cl |
| B-2680 | 2 | H | CH(Me)CH₂ | H | CH₂CF₃ | S(=O)₂CF₃ | Cl |
| B-2681 | 2 | H | CH(Me)CH₂ | Me | CH₂CF₃ | S(=O)₂CF₃ | Cl |
| B-2682 | 2 | Me | CH(Me)CH₂ | H | CH₂CF₃ | S(=O)₂CF₃ | Cl |
| B-2683 | 2 | Me | CH(Me)CH₂ | Me | CH₂CF₃ | S(=O)₂CF₃ | Cl |
| B-2684 | 2 | H | CH₂CH₂CH₂ | Me | CH₂CF₃ | S(=O)₂CF₃ | Cl |
| B-2685 | 2 | Me | CH₂CH₂CH₂ | H | CH₂CF₃ | S(=O)₂CF₃ | Cl |
| B-2686 | 2 | Me | CH₂CH₂CH₂ | Me | CH₂CF₃ | S(=O)₂CF₃ | Cl |
| B-2687 | 2 | H | CH₂CH₂CH(Me) | H | CH₂CF₃ | S(=O)₂CF₃ | Cl |
| B-2688 | 2 | H | CH₂CH₂CH(Me) | Me | CH₂CF₃ | S(=O)₂CF₃ | Cl |
| B-2689 | 2 | Me | CH₂CH₂CH(Me) | H | CH₂CF₃ | S(=O)₂CF₃ | Cl |
| B-2690 | 2 | Me | CH₂CH₂CH(Me) | Me | CH₂CF₃ | S(=O)₂CF₃ | Cl |
| B-2691 | 2 | H | CH₂CH(Me)CH₂ | H | CH₂CF₃ | S(=O)₂CF₃ | Cl |
| B-2692 | 2 | H | CH₂CH(Me)CH₂ | Me | CH₂CF₃ | S(=O)₂CF₃ | Cl |
| B-2693 | 2 | Me | CH₂CH(Me)CH₂ | H | CH₂CF₃ | S(=O)₂CF₃ | Cl |
| B-2694 | 2 | Me | CH₂CH(Me)CH₂ | Me | CH₂CF₃ | S(=O)₂CF₃ | Cl |
| B-2695 | 2 | H | CH(Me)CH₂CH₂ | H | CH₂CF₃ | S(=O)₂CF₃ | Cl |
| B-2696 | 2 | H | CH(Me)CH₂CH₂ | Me | CH₂CF₃ | S(=O)₂CF₃ | Cl |
| B-2697 | 2 | Me | CH(Me)CH₂CH₂ | H | CH₂CF₃ | S(=O)₂CF₃ | Cl |
| B-2698 | 2 | Me | CH(Me)CH₂CH₂ | Me | CH₂CF₃ | S(=O)₂CF₃ | Cl |
| B-2699 | 2 | H | single bond | Me | CH₂CF₃ | S(=O)₂CF₃ | Me |
| B-2700 | 2 | H | single bond | Et | CH₂CF₃ | S(=O)₂CF₃ | Me |
| B-2701 | 2 | Me | single bond | H | CH₂CF₃ | S(=O)₂CF₃ | Me |
| B-2702 | 2 | Et | single bond | H | CH₂CF₃ | S(=O)₂CF₃ | Me |
| B-2703 | 2 | Me | single bond | Me | CH₂CF₃ | S(=O)₂CF₃ | Me |
| B-2704 | 2 | H | CH₂ | Me | CH₂CF₃ | S(=O)₂CF₃ | Me |
| B-2705 | 2 | Me | CH₂ | H | CH₂CF₃ | S(=O)₂CF₃ | Me |
| B-2706 | 2 | Me | CH₂ | Me | CH₂CF₃ | S(=O)₂CF₃ | Me |
| B-2707 | 2 | H | CH(Me) | H | CH₂CF₃ | S(=O)₂CF₃ | Me |
| B-2708 | 2 | H | CH(Me) | Me | CH₂CF₃ | S(=O)₂CF₃ | Me |
| B-2709 | 2 | Me | CH(Me) | H | CH₂CF₃ | S(=O)₂CF₃ | Me |
| B-2710 | 2 | Me | CH(Me) | Me | CH₂CF₃ | S(=O)₂CF₃ | Me |
| B-2711 | 2 | H | CH₂CH₂ | Me | CH₂CF₃ | S(=O)₂CF₃ | Me |
| B-2712 | 2 | Me | CH₂CH₂ | H | CH₂CF₃ | S(=O)₂CF₃ | Me |
| B-2713 | 2 | Me | CH₂CH₂ | Me | CH₂CF₃ | S(=O)₂CF₃ | Me |
| B-2714 | 2 | H | CH₂CH(Me) | H | CH₂CF₃ | S(=O)₂CF₃ | Me |
| B-2715 | 2 | H | CH₂CH(Me) | Me | CH₂CF₃ | S(=O)₂CF₃ | Me |
| B-2716 | 2 | Me | CH₂CH(Me) | H | CH₂CF₃ | S(=O)₂CF₃ | Me |
| B-2717 | 2 | Me | CH₂CH(Me) | Me | CH₂CF₃ | S(=O)₂CF₃ | Me |
| B-2718 | 2 | H | CH(Me)CH₂ | H | CH₂CF₃ | S(=O)₂CF₃ | Me |
| B-2719 | 2 | H | CH(Me)CH₂ | Me | CH₂CF₃ | S(=O)₂CF₃ | Me |
| B-2720 | 2 | Me | CH(Me)CH₂ | H | CH₂CF₃ | S(=O)₂CF₃ | Me |
| B-2721 | 2 | Me | CH(Me)CH₂ | Me | CH₂CF₃ | S(=O)₂CF₃ | Me |
| B-2722 | 2 | H | CH₂CH₂CH₂ | Me | CH₂CF₃ | S(=O)₂CF₃ | Me |

**[Table 226]**

| Compound Number | n | Y | W | Z | R¹ | R² | R³ |
|---|---|---|---|---|---|---|---|
| B-2723 | 2 | Me | CH₂CH₂CH₂ | H | CH₂CF₃ | S(=O)₂CF₃ | Me |
| B-2724 | 2 | Me | CH₂CH₂CH₂ | Me | CH₂CF₃ | S(=O)₂CF₃ | Me |
| B-2725 | 2 | H | CH₂CH₂CH(Me) | H | CH₂CF₃ | S(=O)₂CF₃ | Me |
| B-2726 | 2 | H | CH₂CH₂CH(Me) | Me | CH₂CF₃ | S(=O)₂CF₃ | Me |
| B-2727 | 2 | Me | CH₂CH₂CH(Me) | H | CH₂CF₃ | S(=O)₂CF₃ | Me |
| B-2728 | 2 | Me | CH₂CH₂CH(Me) | Me | CH₂CF₃ | S(=O)₂CF₃ | Me |
| B-2729 | 2 | H | CH₂CH(Me)CH₂ | H | CH₂CF₃ | S(=O)₂CF₃ | Me |
| B-2730 | 2 | H | CH₂CH(Me)CH₂ | Me | CH₂CF₃ | S(=O)₂CF₃ | Me |
| B-2731 | 2 | Me | CH₂CH(Me)CH₂ | H | CH₂CF₃ | S(=O)₂CF₃ | Me |
| B-2732 | 2 | Me | CH₂CH(Me)CH₂ | Me | CH₂CF₃ | S(=O)₂CF₃ | Me |
| B-2733 | 2 | H | CH(Me)CH₂CH₂ | H | CH₂CF₃ | S(=O)₂CF₃ | Me |
| B-2734 | 2 | H | CH(Me)CH₂CH₂ | Me | CH₂CF₃ | S(=O)₂CF₃ | Me |
| B-2735 | 2 | Me | CH(Me)CH₂CH₂ | H | CH₂CF₃ | S(=O)₂CF₃ | Me |
| B-2736 | 2 | Me | CH(Me)CH₂CH₂ | Me | CH₂CF₃ | S(=O)₂CF₃ | Me |

**[Table 227]**

| 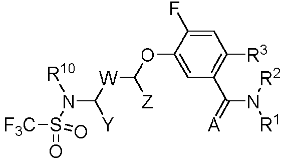 | | | | | | | | |
|---|---|---|---|---|---|---|---|---|
| Compound Number | Y | W | Z | R¹ | R² | R³ | R¹⁰ | A |
| C-0001 | H | single bond | Me | CH₂CF₃ | H | Cl | H | O |
| C-0002 | H | single bond | Et | CH₂CF₃ | H | Cl | H | O |
| C-0003 | H | single bond | *i*-Pr | CH₂CF₃ | H | Cl | H | O |
| C-0004 | Me | single bond | H | CH₂CF₃ | H | Cl | H | O |
| C-0005 | Me(R) | single bond | H | CH₂CF₃ | H | Cl | H | O |
| C-0006 | Me(S) | single bond | H | CH₂CF₃ | H | Cl | H | O |
| C-0007 | Me | single bond | H | CH₂CF₃ | H | Cl | H | S |
| C-0008 | Me(R) | single bond | H | CH₂CF₃ | H | Cl | H | S |
| C-0009 | Me(S) | single bond | H | CH₂CF₃ | H | Cl | H | S |
| C-0010 | Et | single bond | H | CH₂CF₃ | H | Cl | H | O |
| C-0011 | *i*-Pr | single bond | H | CH₂CF₃ | H | Cl | H | O |
| C-0012 | CF₃ | single bond | H | CH₂CF₃ | H | Cl | H | O |
| C-0013 | Me | single bond | Me | CH₂CF₃ | H | Cl | H | O |
| C-0014 | H | CH₂ | Me | CH₂CF₃ | H | Cl | H | O |
| C-0015 | Me | CH₂ | H | CH₂CF₃ | H | Cl | H | O |
| C-0016 | Me | CH₂ | Me | CH₂CF₃ | H | Cl | H | O |
| C-0017 | H | CH(Me) | H | CH₂CF₃ | H | Cl | H | O |
| C-0018 | H | CH(Me) | Me | CH₂CF₃ | H | Cl | H | O |
| C-0019 | Me | CH(Me) | H | CH₂CF₃ | H | Cl | H | O |
| C-0020 | Me | CH(Me) | Me | CH₂CF₃ | H | Cl | H | O |
| C-0021 | H | CH₂CH₂ | Me | CH₂CF₃ | H | Cl | H | O |
| C-0022 | Me | CH₂CH₂ | H | CH₂CF₃ | H | Cl | H | O |
| C-0023 | Me | CH₂CH₂ | Me | CH₂CF₃ | H | Cl | H | O |
| C-0024 | H | CH₂CH(Me) | H | CH₂CF₃ | H | Cl | H | O |
| C-0025 | H | CH₂CH(Me) | Me | CH₂CF₃ | H | Cl | H | O |
| C-0026 | Me | CH₂CH(Me) | H | CH₂CF₃ | H | Cl | H | O |
| C-0027 | Me | CH₂CH(Me) | Me | CH₂CF₃ | H | Cl | H | O |
| C-0028 | H | CH(Me)CH₂ | H | CH₂CF₃ | H | Cl | H | O |
| C-0029 | H | CH(Me)CH₂ | Me | CH₂CF₃ | H | Cl | H | O |
| C-0030 | Me | CH(Me)CH₂ | H | CH₂CF₃ | H | Cl | H | O |
| C-0031 | Me | CH(Me)CH₂ | Me | CH₂CF₃ | H | Cl | H | O |
| C-0032 | H | single bond | Me | CH₂CF₃ | H | Me | H | O |
| C-0033 | H | single bond | Et | CH₂CF₃ | H | Me | H | O |
| C-0034 | H | single bond | *i*-Pr | CH₂CF₃ | H | Me | H | O |
| C-0035 | Me | single bond | H | CH₂CF₃ | H | Me | H | O |
| C-0036 | Me(R) | single bond | H | CH₂CF₃ | H | Me | H | O |
| C-0037 | Me(S) | single bond | H | CH₂CF₃ | H | Me | H | O |
| C-0038 | Me | single bond | H | CH₂CF₃ | H | Me | H | S |

**[Table 228]**

| Compound Number | Y | W | Z | R¹ | R² | R³ | R¹⁰ | A |
|---|---|---|---|---|---|---|---|---|
| C-0039 | Me(R) | single bond | H | CH₂CF₃ | H | Me | H | S |
| C-0040 | Me(S) | single bond | H | CH₂CF₃ | H | Me | H | S |
| C-0041 | Et | single bond | H | CH₂CF₃ | H | Me | H | O |
| C-0042 | *i*-Pr | single bond | H | CH₂CF₃ | H | Me | H | O |
| C-0043 | CF₃ | single bond | H | CH₂CF₃ | H | Me | H | O |
| C-0044 | Me | single bond | Me | CH₂CF₃ | H | Me | H | O |
| C-0045 | H | CH₂ | Me | CH₂CF₃ | H | Me | H | O |
| C-0046 | Me | CH₂ | H | CH₂CF₃ | H | Me | H | O |
| C-0047 | Me | CH₂ | Me | CH₂CF₃ | H | Me | H | O |
| C-0048 | H | CH(Me) | H | CH₂CF₃ | H | Me | H | O |
| C-0049 | H | CH(Me) | Me | CH₂CF₃ | H | Me | H | O |
| C-0050 | Me | CH(Me) | H | CH₂CF₃ | H | Me | H | O |
| C-0051 | Me | CH(Me) | Me | CH₂CF₃ | H | Me | H | O |
| C-0052 | H | CH₂CH₂ | Me | CH₂CF₃ | H | Me | H | O |
| C-0053 | Me | CH₂CH₂ | H | CH₂CF₃ | H | Me | H | O |
| C-0054 | Me | CH₂CH₂ | Me | CH₂CF₃ | H | Me | H | O |
| C-0055 | H | CH₂CH(Me) | H | CH₂CF₃ | H | Me | H | O |
| C-0056 | H | CH₂CH(Me) | Me | CH₂CF₃ | H | Me | H | O |
| C-0057 | Me | CH₂CH(Me) | H | CH₂CF₃ | H | Me | H | O |
| C-0058 | Me | CH₂CH(Me) | Me | CH₂CF₃ | H | Me | H | O |
| C-0059 | H | CH(Me)CH₂ | H | CH₂CF₃ | H | Me | H | O |
| C-0060 | H | CH(Me)CH₂ | Me | CH₂CF₃ | H | Me | H | O |
| C-0061 | Me | CH(Me)CH₂ | H | CH₂CF₃ | H | Me | H | O |
| C-0062 | Me | CH(Me)CH₂ | Me | CH₂CF₃ | H | Me | H | O |
| C-0063 | H | single bond | Me | Et | H | Cl | H | O |
| C-0064 | H | single bond | Et | Et | H | Cl | H | O |
| C-0065 | H | single bond | *i*-Pr | Et | H | Cl | H | O |
| C-0066 | Me | single bond | H | Et | H | Cl | H | O |
| C-0067 | Me(R) | single bond | H | Et | H | Cl | H | O |
| C-0068 | Me(S) | single bond | H | Et | H | Cl | H | O |
| C-0069 | Me | single bond | H | Et | H | Cl | H | S |
| C-0070 | Me(R) | single bond | H | Et | H | Cl | H | S |
| C-0071 | Me(S) | single bond | H | Et | H | Cl | H | S |
| C-0072 | Et | single bond | H | Et | H | Cl | H | O |
| C-0073 | *i*-Pr | single bond | H | Et | H | Cl | H | O |
| C-0074 | CF₃ | single bond | H | Et | H | Cl | H | O |
| C-0075 | Me | single bond | Me | Et | H | Cl | H | O |
| C-0076 | H | CH₂ | Me | Et | H | Cl | H | O |
| C-0077 | Me | CH₂ | H | Et | H | Cl | H | O |
| C-0078 | Me | CH₂ | Me | Et | H | Cl | H | O |
| C-0079 | H | CH(Me) | H | Et | H | Cl | H | O |
| C-0080 | H | CH(Me) | Me | Et | H | Cl | H | O |
| C-0081 | Me | CH(Me) | H | Et | H | Cl | H | O |
| C-0082 | Me | CH(Me) | Me | Et | H | Cl | H | O |

**[Table 229]**

| Compound Number | Y | W | Z | R¹ | R² | R³ | R¹⁰ | A |
|---|---|---|---|---|---|---|---|---|
| C-0083 | H | CH₂CH₂ | Me | Et | H | Cl | H | O |
| C-0084 | Me | CH₂CH₂ | H | Et | H | Cl | H | O |
| C-0085 | Me | CH₂CH₂ | Me | Et | H | Cl | H | O |
| C-0086 | H | CH₂CH(Me) | H | Et | H | Cl | H | O |
| C-0087 | H | CH₂CH(Me) | Me | Et | H | Cl | H | O |
| C-0088 | Me | CH₂CH(Me) | H | Et | H | Cl | H | O |
| C-0089 | Me | CH₂CH(Me) | Me | Et | H | Cl | H | O |
| C-0090 | H | CH(Me)CH₂ | H | Et | H | Cl | H | O |
| C-0091 | H | CH(Me)CH₂ | Me | Et | H | Cl | H | O |
| C-0092 | Me | CH(Me)CH₂ | H | Et | H | Cl | H | O |
| C-0093 | Me | CH(Me)CH₂ | Me | Et | H | Cl | H | O |
| C-0094 | H | single bond | Me | Et | H | Me | H | O |
| C-0095 | H | single bond | Et | Et | H | Me | H | O |
| C-0096 | H | single bond | *i*-Pr | Et | H | Me | H | O |
| C-0097 | Me | single bond | H | Et | H | Me | H | O |
| C-0098 | Me(R) | single bond | H | Et | H | Me | H | O |
| C-0099 | Me(S) | single bond | H | Et | H | Me | H | O |
| C-0100 | Me | single bond | H | Et | H | Me | H | S |
| C-0101 | Me(R) | single bond | H | Et | H | Me | H | S |
| C-0102 | Me(S) | single bond | H | Et | H | Me | H | S |
| C-0103 | Et | single bond | H | Et | H | Me | H | O |
| C-0104 | *i*-Pr | single bond | H | Et | H | Me | H | O |
| C-0105 | CF₃ | single bond | H | Et | H | Me | H | O |
| C-0106 | Me | single bond | Me | Et | H | Me | H | O |
| C-0107 | H | CH₂ | Me | Et | H | Me | H | O |
| C-0108 | Me | CH₂ | H | Et | H | Me | H | O |
| C-0109 | Me | CH₂ | Me | Et | H | Me | H | O |
| C-0110 | H | CH(Me) | H | Et | H | Me | H | O |
| C-0111 | H | CH(Me) | Me | Et | H | Me | H | O |
| C-0112 | Me | CH(Me) | H | Et | H | Me | H | O |
| C-0113 | Me | CH(Me) | Me | Et | H | Me | H | O |
| C-0114 | H | CH₂CH₂ | Me | Et | H | Me | H | O |
| C-0115 | Me | CH₂CH₂ | H | Et | H | Me | H | O |
| C-0116 | Me | CH₂CH₂ | Me | Et | H | Me | H | O |
| C-0117 | H | CH₂CH(Me) | H | Et | H | Me | H | O |
| C-0118 | H | CH₂CH(Me) | Me | Et | H | Me | H | O |
| C-0119 | Me | CH₂CH(Me) | H | Et | H | Me | H | O |
| C-0120 | Me | CH₂CH(Me) | Me | Et | H | Me | H | O |
| C-0121 | H | CH(Me)CH₂ | H | Et | H | Me | H | O |
| C-0122 | H | CH(Me)CH₂ | Me | Et | H | Me | H | O |
| C-0123 | Me | CH(Me)CH₂ | H | Et | H | Me | H | O |
| C-0124 | Me | CH(Me)CH₂ | Me | Et | H | Me | H | O |
| C-0125 | H | single bond | Me | *c*-Pr | H | Cl | H | O |
| C-0126 | H | single bond | Et | *c*-Pr | H | Cl | H | O |

**[Table 230]**

| Compound Number | Y | W | Z | R¹ | R² | R³ | R¹⁰ | A |
|---|---|---|---|---|---|---|---|---|
| C-0127 | H | single bond | *i*-Pr | *c*-Pr | H | Cl | H | O |
| C-0128 | Me | single bond | H | *c*-Pr | H | Cl | H | O |
| C-0129 | Me(R) | single bond | H | *c*-Pr | H | Cl | H | O |
| C-0130 | Me(S) | single bond | H | *c*-Pr | H | Cl | H | O |
| C-0131 | Me | single bond | H | *c*-Pr | H | Cl | H | S |
| C-0132 | Me(R) | single bond | H | *c*-Pr | H | Cl | H | S |
| C-0133 | Me(S) | single bond | H | *c*-Pr | H | Cl | H | S |
| C-0134 | Et | single bond | H | *c*-Pr | H | Cl | H | O |
| C-0135 | *i*-Pr | single bond | H | *c*-Pr | H | Cl | H | O |
| C-0136 | CF₃ | single bond | H | *c*-Pr | H | Cl | H | O |
| C-0137 | Me | single bond | Me | *c*-Pr | H | Cl | H | O |
| C-0138 | H | CH₂ | Me | *c*-Pr | H | Cl | H | O |
| C-0139 | Me | CH₂ | H | *c*-Pr | H | Cl | H | O |
| C-0140 | Me | CH₂ | Me | *c*-Pr | H | Cl | H | O |
| C-0141 | H | CH(Me) | H | *c*-Pr | H | Cl | H | O |
| C-0142 | H | CH(Me) | Me | *c*-Pr | H | Cl | H | O |
| C-0143 | Me | CH(Me) | H | *c*-Pr | H | Cl | H | O |
| C-0144 | Me | CH(Me) | Me | *c*-Pr | H | Cl | H | O |
| C-0145 | H | CH₂CH₂ | Me | *c*-Pr | H | Cl | H | O |
| C-0146 | Me | CH₂CH₂ | H | *c*-Pr | H | Cl | H | O |
| C-0147 | Me | CH₂CH₂ | Me | *c*-Pr | H | Cl | H | O |
| C-0148 | H | CH₂CH(Me) | H | *c*-Pr | H | Cl | H | O |
| C-0149 | H | CH₂CH(Me) | Me | *c*-Pr | H | Cl | H | O |
| C-0150 | Me | CH₂CH(Me) | H | *c*-Pr | H | Cl | H | O |
| C-0151 | Me | CH₂CH(Me) | Me | *c*-Pr | H | Cl | H | O |
| C-0152 | H | CH(Me)CH₂ | H | *c*-Pr | H | Cl | H | O |
| C-0153 | H | CH(Me)CH₂ | Me | *c*-Pr | H | Cl | H | O |
| C-0154 | Me | CH(Me)CH₂ | H | *c*-Pr | H | Cl | H | O |
| C-0155 | Me | CH(Me)CH₂ | Me | *c*-Pr | H | Cl | H | O |
| C-0156 | H | single bond | Me | *c*-Pr | H | Me | H | O |
| C-0157 | H | single bond | Et | *c*-Pr | H | Me | H | O |
| C-0158 | H | single bond | *i*-Pr | *c*-Pr | H | Me | H | O |
| C-0159 | Me | single bond | H | *c*-Pr | H | Me | H | O |
| C-0160 | Me(R) | single bond | H | *c*-Pr | H | Me | H | O |
| C-0161 | Me(S) | single bond | H | *c*-Pr | H | Me | H | O |
| C-0162 | Me | single bond | H | *c*-Pr | H | Me | H | S |
| C-0163 | Me(R) | single bond | H | *c*-Pr | H | Me | H | S |
| C-0164 | Me(S) | single bond | H | *c*-Pr | H | Me | H | S |
| C-0165 | Et | single bond | H | *c*-Pr | H | Me | H | O |
| C-0166 | *i*-Pr | single bond | H | *c*-Pr | H | Me | H | O |
| C-0167 | CF₃ | single bond | H | *c*-Pr | H | Me | H | O |
| C-0168 | Me | single bond | Me | *c*-Pr | H | Me | H | O |
| C-0169 | H | CH₂ | Me | *c*-Pr | H | Me | H | O |
| C-0170 | Me | CH₂ | H | *c*-Pr | H | Me | H | O |

**[Table 231]**

| Compound Number | Y | W | Z | R¹ | R² | R³ | R¹⁰ | A |
|---|---|---|---|---|---|---|---|---|
| C-0171 | Me | CH₂ | Me | *c*-Pr | H | Me | H | O |
| C-0172 | H | CH(Me) | H | *c*-Pr | H | Me | H | O |
| C-0173 | H | CH(Me) | Me | *c*-Pr | H | Me | H | O |
| C-0174 | Me | CH(Me) | H | *c*-Pr | H | Me | H | O |
| C-0175 | Me | CH(Me) | Me | *c*-Pr | H | Me | H | O |
| C-0176 | H | CH₂CH₂ | Me | *c*-Pr | H | Me | H | O |
| C-0177 | Me | CH₂CH₂ | H | *c*-Pr | H | Me | H | O |
| C-0178 | Me | CH₂CH₂ | Me | *c*-Pr | H | Me | H | O |
| C-0179 | H | CH₂CH(Me) | H | *c*-Pr | H | Me | H | O |
| C-0180 | H | CH₂CH(Me) | Me | *c*-Pr | H | Me | H | O |
| C-0181 | Me | CH₂CH(Me) | H | *c*-Pr | H | Me | H | O |
| C-0182 | Me | CH₂CH(Me) | Me | *c*-Pr | H | Me | H | O |
| C-0183 | H | CH(Me)CH₂ | H | *c*-Pr | H | Me | H | O |
| C-0184 | H | CH(Me)CH₂ | Me | *c*-Pr | H | Me | H | O |
| C-0185 | Me | CH(Me)CH₂ | H | *c*-Pr | H | Me | H | O |
| C-0186 | Me | CH(Me)CH₂ | Me | *c*-Pr | H | Me | H | O |
| C-0187 | H | single bond | Me | CH₂CF₃ | H | Cl | Me | O |
| C-0188 | H | single bond | Et | CH₂CF₃ | H | Cl | Me | O |
| C-0189 | H | single bond | *i*-Pr | CH₂CF₃ | H | Cl | Me | O |
| C-0190 | Me | single bond | H | CH₂CF₃ | H | Cl | Me | O |
| C-0191 | Me (R) | single bond | H | CH₂CF₃ | H | Cl | Me | O |
| C-0192 | Me (S) | single bond | H | CH₂CF₃ | H | Cl | Me | O |
| C-0193 | Me | single bond | H | CH₂CF₃ | H | Cl | Me | S |
| C-0194 | Me(R) | single bond | H | CH₂CF₃ | H | Cl | Me | S |
| C-0195 | Me(S) | single bond | H | CH₂CF₃ | H | Cl | Me | S |
| C-0196 | Et | single bond | H | CH₂CF₃ | H | Cl | Me | O |
| C-0197 | *i*-Pr | single bond | H | CH₂CF₃ | H | Cl | Me | O |
| C-0198 | CF₃ | single bond | H | CH₂CF₃ | H | Cl | Me | O |
| C-0199 | Me | single bond | Me | CH₂CF₃ | H | Cl | Me | O |
| C-0200 | H | CH₂ | Me | CH₂CF₃ | H | Cl | Me | O |
| C-0201 | Me | CH₂ | H | CH₂CF₃ | H | Cl | Me | O |
| C-0202 | Me | CH₂ | Me | CH₂CF₃ | H | Cl | Me | O |
| C-0203 | H | CH(Me) | H | CH₂CF₃ | H | Cl | Me | O |
| C-0204 | H | CH(Me) | Me | CH₂CF₃ | H | Cl | Me | O |
| C-0205 | Me | CH(Me) | H | CH₂CF₃ | H | Cl | Me | O |
| C-0206 | Me | CH(Me) | Me | CH₂CF₃ | H | Cl | Me | O |
| C-0207 | H | CH₂CH₂ | Me | CH₂CF₃ | H | Cl | Me | O |
| C-0208 | Me | CH₂CH₂ | H | CH₂CF₃ | H | Cl | Me | O |
| C-0209 | Me | CH₂CH₂ | Me | CH₂CF₃ | H | Cl | Me | O |
| C-0210 | H | CH₂CH(Me) | H | CH₂CF₃ | H | Cl | Me | O |
| C-0211 | H | CH₂CH(Me) | Me | CH₂CF₃ | H | Cl | Me | O |
| C-0212 | Me | CH₂CH(Me) | H | CH₂CF₃ | H | Cl | Me | O |
| C-0213 | Me | CH₂CH(Me) | Me | CH₂CF₃ | H | Cl | Me | O |
| C-0214 | H | CH(Me)CH₂ | H | CH₂CF₃ | H | Cl | Me | O |

**[Table 232]**

| Compound Number | Y | W | Z | R¹ | R² | R³ | R¹⁰ | A |
|---|---|---|---|---|---|---|---|---|
| C-0215 | H | CH(Me)CH₂ | Me | CH₂CF₃ | H | Cl | Me | O |
| C-0216 | Me | CH(Me)CH₂ | H | CH₂CF₃ | H | Cl | Me | O |
| C-0217 | Me | CH(Me)CH₂ | Me | CH₂CF₃ | H | Cl | Me | O |
| C-0218 | H | single bond | Me | CH₂CF₃ | H | Me | Me | O |
| C-0219 | H | single bond | Et | CH₂CF₃ | H | Me | Me | O |
| C-0220 | H | single bond | *i*-Pr | CH₂CF₃ | H | Me | Me | O |
| C-0221 | Me | single bond | H | CH₂CF₃ | H | Me | Me | O |
| C-0222 | Me(R) | single bond | H | CH₂CF₃ | H | Me | Me | O |
| C-0223 | Me(S) | single bond | H | CH₂CF₃ | H | Me | Me | O |
| C-0224 | Me | single bond | H | CH₂CF₃ | H | Me | Me | S |
| C-0225 | Me(R) | single bond | H | CH₂CF₃ | H | Me | Me | S |
| C-0226 | Me(S) | single bond | H | CH₂CF₃ | H | Me | Me | S |
| C-0227 | Et | single bond | H | CH₂CF₃ | H | Me | Me | O |
| C-0228 | *i*-Pr | single bond | H | CH₂CF₃ | H | Me | Me | O |
| C-0229 | CF₃ | single bond | H | CH₂CF₃ | H | Me | Me | O |
| C-0230 | Me | single bond | Me | CH₂CF₃ | H | Me | Me | O |
| C-0231 | H | CH₂ | Me | CH₂CF₃ | H | Me | Me | O |
| C-0232 | Me | CH₂ | H | CH₂CF₃ | H | Me | Me | O |
| C-0233 | Me | CH₂ | Me | CH₂CF₃ | H | Me | Me | O |
| C-0234 | H | CH(Me) | H | CH₂CF₃ | H | Me | Me | O |
| C-0235 | H | CH(Me) | Me | CH₂CF₃ | H | Me | Me | O |
| C-0236 | Me | CH(Me) | H | CH₂CF₃ | H | Me | Me | O |
| C-0237 | Me | CH(Me) | Me | CH₂CF₃ | H | Me | Me | O |
| C-0238 | H | CH₂CH₂ | Me | CH₂CF₃ | H | Me | Me | O |
| C-0239 | Me | CH₂CH₂ | H | CH₂CF₃ | H | Me | Me | O |
| C-0240 | Me | CH₂CH₂ | Me | CH₂CF₃ | H | Me | Me | O |
| C-0241 | H | CH₂CH(Me) | H | CH₂CF₃ | H | Me | Me | O |
| C-0242 | H | CH₂CH(Me) | Me | CH₂CF₃ | H | Me | Me | O |
| C-0243 | Me | CH₂CH(Me) | H | CH₂CF₃ | H | Me | Me | O |
| C-0244 | Me | CH₂CH(Me) | Me | CH₂CF₃ | H | Me | Me | O |
| C-0245 | H | CH(Me)CH₂ | H | CH₂CF₃ | H | Me | Me | O |
| C-0246 | H | CH(Me)CH₂ | Me | CH₂CF₃ | H | Me | Me | O |
| C-0247 | Me | CH(Me)CH₂ | H | CH₂CF₃ | H | Me | Me | O |
| C-0248 | Me | CH(Me)CH₂ | Me | CH₂CF₃ | H | Me | Me | O |
| C-0249 | H | single bond | Me | Et | H | Cl | Me | O |
| C-0250 | H | single bond | Et | Et | H | Cl | Me | O |
| C-0251 | H | single bond | *i*-Pr | Et | H | Cl | Me | O |
| C-0252 | Me | single bond | H | Et | H | Cl | Me | O |
| C-0253 | Me(R) | single bond | H | Et | H | Cl | Me | O |
| C-0254 | Me(S) | single bond | H | Et | H | Cl | Me | O |
| C-0255 | Me | single bond | H | Et | H | Cl | Me | S |
| C-0256 | Me(R) | single bond | H | Et | H | Cl | Me | S |
| C-0257 | Me(S) | single bond | H | Et | H | Cl | Me | S |
| C-0258 | Et | single bond | H | Et | H | Cl | Me | O |

**[Table 233]**

| Compound Number | Y | W | Z | R¹ | R² | R³ | R¹⁰ | A |
|---|---|---|---|---|---|---|---|---|
| C-0259 | *i*-Pr | single bond | H | Et | H | Cl | Me | O |
| C-0260 | CF₃ | single bond | H | Et | H | Cl | Me | O |
| C-0261 | Me | single bond | Me | Et | H | Cl | Me | O |
| C-0262 | H | CH₂ | Me | Et | H | Cl | Me | O |
| C-0263 | Me | CH₂ | H | Et | H | Cl | Me | O |
| C-0264 | Me | CH₂ | Me | Et | H | Cl | Me | O |
| C-0265 | H | CH(Me) | H | Et | H | Cl | Me | O |
| C-0266 | H | CH(Me) | Me | Et | H | Cl | Me | O |
| C-0267 | Me | CH(Me) | H | Et | H | Cl | Me | O |
| C-0268 | Me | CH(Me) | Me | Et | H | Cl | Me | O |
| C-0269 | H | CH₂CH₂ | Me | Et | H | Cl | Me | O |
| C-0270 | Me | CH₂CH₂ | H | Et | H | Cl | Me | O |
| C-0271 | Me | CH₂CH₂ | Me | Et | H | Cl | Me | O |
| C-0272 | H | CH₂CH(Me) | H | Et | H | Cl | Me | O |
| C-0273 | H | CH₂CH(Me) | Me | Et | H | Cl | Me | O |
| C-0274 | Me | CH₂CH(Me) | H | Et | H | Cl | Me | O |
| C-0275 | Me | CH₂CH(Me) | Me | Et | H | Cl | Me | O |
| C-0276 | H | CH(Me)CH₂ | H | Et | H | Cl | Me | O |
| C-0277 | H | CH(Me)CH₂ | Me | Et | H | Cl | Me | O |
| C-0278 | Me | CH(Me)CH₂ | H | Et | H | Cl | Me | O |
| C-0279 | Me | CH(Me)CH₂ | Me | Et | H | Cl | Me | O |
| C-0280 | H | single bond | Me | Et | H | Me | Me | O |
| C-0281 | H | single bond | Et | Et | H | Me | Me | O |
| C-0282 | H | single bond | i-Pr | Et | H | Me | Me | O |
| C-0283 | Me | single bond | H | Et | H | Me | Me | O |
| C-0284 | Me(R) | single bond | H | Et | H | Me | Me | O |
| C-0285 | Me(S) | single bond | H | Et | H | Me | Me | O |
| C-0286 | Me | single bond | H | Et | H | Me | Me | S |
| C-0287 | Me(R) | single bond | H | Et | H | Me | Me | S |
| C-0288 | Me(S) | single bond | H | Et | H | Me | Me | S |
| C-0289 | Et | single bond | H | Et | H | Me | Me | O |
| C-0290 | *i*-Pr | single bond | H | Et | H | Me | Me | O |
| C-0291 | CF₃ | single bond | H | Et | H | Me | Me | O |
| C-0292 | Me | single bond | Me | Et | H | Me | Me | O |
| C-0293 | H | CH₂ | Me | Et | H | Me | Me | O |
| C-0294 | Me | CH₂ | H | Et | H | Me | Me | O |
| C-0295 | Me | CH₂ | Me | Et | H | Me | Me | O |
| C-0296 | H | CH(Me) | H | Et | H | Me | Me | O |
| C-0297 | H | CH(Me) | Me | Et | H | Me | Me | O |
| C-0298 | Me | CH(Me) | H | Et | H | Me | Me | O |
| C-0299 | Me | CH(Me) | Me | Et | H | Me | Me | O |
| C-0300 | H | CH₂CH₂ | Me | Et | H | Me | Me | O |
| C-0301 | Me | CH₂CH₂ | H | Et | H | Me | Me | O |
| C-0302 | Me | CH₂CH₂ | Me | Et | H | Me | Me | O |

**[Table 234]**

| Compound Number | Y | W | Z | R¹ | R² | R³ | R¹⁰ | A |
|---|---|---|---|---|---|---|---|---|
| C-0303 | H | CH₂CH(Me) | H | Et | H | Me | Me | O |
| C-0304 | H | CH₂CH(Me) | Me | Et | H | Me | Me | O |
| C-0305 | Me | CH₂CH(Me) | H | Et | H | Me | Me | O |
| C-0306 | Me | CH₂CH(Me) | Me | Et | H | Me | Me | O |
| C-0307 | H | CH(Me)CH₂ | H | Et | H | Me | Me | O |
| C-0308 | H | CH(Me)CH₂ | Me | Et | H | Me | Me | O |
| C-0309 | Me | CH(Me)CH₂ | H | Et | H | Me | Me | O |
| C-0310 | Me | CH(Me)CH₂ | Me | Et | H | Me | Me | O |
| C-0311 | H | single bond | Me | *c*-Pr | H | Cl | Me | O |
| C-0312 | H | single bond | Et | *c*-Pr | H | Cl | Me | O |
| C-0313 | H | single bond | *i*-Pr | *c*-Pr | H | Cl | Me | O |
| C-0314 | Me | single bond | H | *c*-Pr | H | Cl | Me | O |
| C-0315 | Me(R) | single bond | H | *c*-Pr | H | Cl | Me | O |
| C-0316 | Me(S) | single bond | H | *c*-Pr | H | Cl | Me | O |
| C-0317 | Me | single bond | H | *c*-Pr | H | Cl | Me | S |
| C-0318 | Me(R) | single bond | H | *c*-Pr | H | Cl | Me | S |
| C-0319 | Me(S) | single bond | H | *c*-Pr | H | Cl | Me | S |
| C-0320 | Et | single bond | H | *c*-Pr | H | Cl | Me | O |
| C-0321 | *i*-Pr | single bond | H | *c*-Pr | H | Cl | Me | O |
| C-0322 | CF₃ | single bond | H | *c*-Pr | H | Cl | Me | O |
| C-0323 | Me | single bond | Me | *c*-Pr | H | Cl | Me | O |
| C-0324 | H | CH₂ | Me | *c*-Pr | H | Cl | Me | O |
| C-0325 | Me | CH₂ | H | *c*-Pr | H | Cl | Me | O |
| C-0326 | Me | CH₂ | Me | *c*-Pr | H | Cl | Me | O |
| C-0327 | H | CH(Me) | H | *c*-Pr | H | Cl | Me | O |
| C-0328 | H | CH(Me) | Me | *c*-Pr | H | Cl | Me | O |
| C-0329 | Me | CH(Me) | H | *c*-Pr | H | Cl | Me | O |
| C-0330 | Me | CH(Me) | Me | *c*-Pr | H | Cl | Me | O |
| C-0331 | H | CH₂CH₂ | Me | *c*-Pr | H | Cl | Me | O |
| C-0332 | Me | CH₂CH₂ | H | *c*-Pr | H | Cl | Me | O |
| C-0333 | Me | CH₂CH₂ | Me | *c*-Pr | H | Cl | Me | O |
| C-0334 | H | CH₂CH(Me) | H | *c*-Pr | H | Cl | Me | O |
| C-0335 | H | CH₂CH(Me) | Me | *c*-Pr | H | Cl | Me | O |
| C-0336 | Me | CH₂CH(Me) | H | *c*-Pr | H | Cl | Me | O |
| C-0337 | Me | CH₂CH(Me) | Me | *c*-Pr | H | Cl | Me | O |
| C-0338 | H | CH(Me)CH₂ | H | *c*-Pr | H | Cl | Me | O |
| C-0339 | H | CH(Me)CH₂ | Me | *c*-Pr | H | Cl | Me | O |
| C-0340 | Me | CH(Me)CH₂ | H | *c*-Pr | H | Cl | Me | O |
| C-0341 | Me | CH(Me)CH₂ | Me | *c*-Pr | H | Cl | Me | O |
| C-0342 | H | single bond | Me | *c*-Pr | H | Me | Me | O |
| C-0343 | H | single bond | Et | *c*-Pr | H | Me | Me | O |
| C-0344 | H | single bond | *i*-Pr | *c*-Pr | H | Me | Me | O |
| C-0345 | Me | single bond | H | *c*-Pr | H | Me | Me | O |
| C-0346 | Me(R) | single bond | H | *c*-Pr | H | Me | Me | O |

**[Table 235]**

| Compound Number | Y | W | Z | R¹ | R² | R³ | R¹⁰ | A |
|---|---|---|---|---|---|---|---|---|
| C-0347 | Me(S) | single bond | H | *c*-Pr | H | Me | Me | O |
| C-0348 | Me | single bond | H | *c*-Pr | H | Me | Me | S |
| C-0349 | Me(R) | single bond | H | *c*-Pr | H | Me | Me | S |
| C-0350 | Me(S) | single bond | H | *c*-Pr | H | Me | Me | S |
| C-0351 | Et | single bond | H | *c*-Pr | H | Me | Me | O |
| C-0352 | *i*-Pr | single bond | H | *c*-Pr | H | Me | Me | O |
| C-0353 | CF₃ | single bond | H | *c*-Pr | H | Me | Me | O |
| C-0354 | Me | single bond | Me | *c*-Pr | H | Me | Me | O |
| C-0355 | H | CH₂ | Me | *c*-Pr | H | Me | Me | O |
| C-0356 | Me | CH₂ | H | *c*-Pr | H | Me | Me | O |
| C-0357 | Me | CH₂ | Me | *c*-Pr | H | Me | Me | *O* |
| C-0358 | H | CH(Me) | H | *c*-Pr | H | Me | Me | O |
| C-0359 | H | CH(Me) | Me | *c*-Pr | H | Me | Me | O |
| C-0360 | Me | CH(Me) | H | *c*-Pr | H | Me | Me | O |
| C-0361 | Me | CH(Me) | Me | *c*-Pr | H | Me | Me | *O* |
| C-0362 | H | CH₂CH₂ | Me | *c*-Pr | H | Me | Me | O |
| C-0363 | Me | CH₂CH₂ | H | *c*-Pr | H | Me | Me | O |
| C-0364 | Me | CH₂CH₂ | Me | *c*-Pr | H | Me | Me | O |
| C-0365 | H | CH₂CH(Me) | H | *c*-Pr | H | Me | Me | O |
| C-0366 | H | CH₂CH(Me) | Me | *c*-Pr | H | Me | Me | O |
| C-0367 | Me | CH₂CH(Me) | H | *c*-Pr | H | Me | Me | O |
| C-0368 | Me | CH₂CH(Me) | Me | *c*-Pr | H | Me | Me | O |
| C-0369 | H | CH(Me)CH₂ | H | *c*-Pr | H | Me | Me | O |
| C-0370 | H | CH(Me)CH₂ | Me | *c*-Pr | H | Me | Me | O |
| C-0371 | Me | CH(Me)CH₂ | H | *c*-Pr | H | Me | Me | O |
| C-0372 | Me | CH(Me)CH₂ | Me | *c*-Pr | H | Me | Me | O |
| C-0373 | H | single bond | Me | CH₂CF₃ | H | Cl | Et | O |
| C-0374 | Me | single bond | H | CH₂CF₃ | H | Cl | Et | O |
| C-0375 | Me(R) | single bond | H | CH₂CF₃ | H | Cl | Et | O |
| C-0376 | Me(S) | single bond | H | CH₂CF₃ | H | Cl | Et | O |
| C-0377 | Me | single bond | H | CH₂CF₃ | H | Cl | Et | S |
| C-0378 | Me(R) | single bond | H | CH₂CF₃ | H | Cl | Et | S |
| C-0379 | Me(S) | single bond | H | CH₂CF₃ | H | Cl | Et | S |
| C-0380 | Me | single bond | Me | CH₂CF₃ | H | Cl | Et | O |
| C-0381 | H | CH₂ | Me | CH₂CF₃ | H | Cl | Et | O |
| C-0382 | Me | CH₂ | H | CH₂CF₃ | H | Cl | Et | O |
| C-0383 | Me | CH₂ | Me | CH₂CF₃ | H | Cl | Et | O |
| C-0384 | H | CH(Me) | H | CH₂CF₃ | H | Cl | Et | O |
| C-0385 | H | CH(Me) | Me | CH₂CF₃ | H | Cl | Et | O |
| C-0386 | Me | CH(Me) | H | CH₂CF₃ | H | Cl | Et | O |
| C-0387 | Me | CH(Me) | Me | CH₂CF₃ | H | Cl | Et | O |
| C-0388 | H | CH₂CH₂ | Me | CH₂CF₃ | H | Cl | Et | O |
| C-0389 | Me | CH₂CH₂ | H | CH₂CF₃ | H | Cl | Et | O |
| C-0390 | Me | CH₂CH₂ | Me | CH₂CF₃ | H | Cl | Et | O |

**[Table 236]**

| Compound Number | Y | W | Z | R¹ | R² | R³ | R¹⁰ | A |
|---|---|---|---|---|---|---|---|---|
| C-0391 | H | single bond | Me | CH₂CF₃ | H | Me | Et | O |
| C-0392 | Me | single bond | H | CH₂CF₃ | H | Me | Et | O |
| C-0393 | Me(R) | single bond | H | CH₂CF₃ | H | Me | Et | O |
| C-0394 | Me(S) | single bond | H | CH₂CF₃ | H | Me | Et | O |
| C-0395 | Me | single bond | H | CH₂CF₃ | H | Me | Et | S |
| C-0396 | Me(R) | single bond | H | CH₂CF₃ | H | Me | Et | S |
| C-0397 | Me(S) | single bond | H | CH₂CF₃ | H | Me | Et | S |
| C-0398 | Me | single bond | Me | CH₂CF₃ | H | Me | Et | O |
| C-0399 | H | CH₂ | Me | CH₂CF₃ | H | Me | Et | O |
| C-0400 | Me | CH₂ | H | CH₂CF₃ | H | Me | Et | O |
| C-0401 | Me | CH₂ | Me | CH₂CF₃ | H | Me | Et | O |
| C-0402 | H | CH(Me) | H | CH₂CF₃ | H | Me | Et | O |
| C-0403 | H | CH(Me) | Me | CH₂CF₃ | H | Me | Et | O |
| C-0404 | Me | CH(Me) | H | CH₂CF₃ | H | Me | Et | O |
| C-0405 | Me | CH(Me) | Me | CH₂CF₃ | H | Me | Et | O |
| C-0406 | H | CH₂CH₂ | Me | CH₂CF₃ | H | Me | Et | O |
| C-0407 | Me | CH₂CH₂ | H | CH₂CF₃ | H | Me | Et | O |
| C-0408 | Me | CH₂CH₂ | Me | CH₂CF₃ | H | Me | Et | O |
| C-0409 | H | single bond | Me | Et | H | Cl | Et | O |
| C-0410 | Me | single bond | H | Et | H | Cl | Et | O |
| C-0411 | Me(R) | single bond | H | Et | H | Cl | Et | O |
| C-0412 | Me(S) | single bond | H | Et | H | Cl | Et | O |
| C-0413 | Me | single bond | H | Et | H | Cl | Et | S |
| C-0414 | Me(R) | single bond | H | Et | H | Cl | Et | S |
| C-0415 | Me(S) | single bond | H | Et | H | Cl | Et | S |
| C-0416 | Me | single bond | Me | Et | H | Cl | Et | O |
| C-0417 | H | CH₂ | Me | Et | H | Cl | Et | O |
| C-0418 | Me | CH₂ | H | Et | H | Cl | Et | O |
| C-0419 | Me | CH₂ | Me | Et | H | Cl | Et | O |
| C-0420 | H | CH(Me) | H | Et | H | Cl | Et | O |
| C-0421 | H | CH(Me) | Me | Et | H | Cl | Et | O |
| C-0422 | Me | CH(Me) | H | Et | H | Cl | Et | O |
| C-0423 | Me | CH(Me) | Me | Et | H | Cl | Et | O |
| C-0424 | H | CH₂CH₂ | Me | Et | H | Cl | Et | O |
| C-0425 | Me | CH₂CH₂ | H | Et | H | Cl | Et | O |
| C-0426 | Me | CH₂CH₂ | Me | Et | H | Cl | Et | O |
| C-0427 | H | single bond | Me | Et | H | Me | Et | O |
| C-0428 | Me | single bond | H | Et | H | Me | Et | O |
| C-0429 | Me(R) | single bond | H | Et | H | Me | Et | O |
| C-0430 | Me(S) | single bond | H | Et | H | Me | Et | O |
| C-0431 | Me | single bond | H | Et | H | Me | Et | S |
| C-0432 | Me(R) | single bond | H | Et | H | Me | Et | S |
| C-0433 | Me(S) | single bond | H | Et | H | Me | Et | S |
| C-0434 | Me | single bond | Me | Et | H | Me | Et | O |

**[Table 237]**

| Compound Number | Y | W | Z | R¹ | R² | R³ | R¹⁰ | A |
|---|---|---|---|---|---|---|---|---|
| C-0435 | H | CH₂ | Me | Et | H | Me | Et | O |
| C-0436 | Me | CH₂ | H | Et | H | Me | Et | O |
| C-0437 | Me | CH₂ | Me | Et | H | Me | Et | O |
| C-0438 | H | CH(Me) | H | Et | H | Me | Et | O |
| C-0439 | H | CH(Me) | Me | Et | H | Me | Et | O |
| C-0440 | Me | CH(Me) | H | Et | H | Me | Et | O |
| C-0441 | Me | CH(Me) | Me | Et | H | Me | Et | O |
| C-0442 | H | CH₂CH₂ | Me | Et | H | Me | Et | O |
| C-0443 | Me | CH₂CH₂ | H | Et | H | Me | Et | O |
| C-0444 | Me | CH₂CH₂ | Me | Et | H | Me | Et | O |
| C-0445 | H | single bond | Me | *c*-Pr | H | Cl | Et | O |
| C-0446 | Me | single bond | H | *c*-Pr | H | Cl | Et | O |
| C-0447 | Me(R) | single bond | H | *c*-Pr | H | Cl | Et | O |
| C-0448 | Me(S) | single bond | H | *c*-Pr | H | Cl | Et | O |
| C-0449 | Me | single bond | H | *c*-Pr | H | Cl | Et | S |
| C-0450 | Me(R) | single bond | H | *c*-Pr | H | Cl | Et | S |
| C-0451 | Me(S) | single bond | H | *c*-Pr | H | Cl | Et | S |
| C-0452 | Me | single bond | Me | *c*-Pr | H | Cl | Et | O |
| C-0453 | H | CH₂ | Me | *c*-Pr | H | Cl | Et | O |
| C-0454 | Me | CH₂ | H | *c*-Pr | H | Cl | Et | O |
| C-0455 | Me | CH₂ | Me | *c*-Pr | H | Cl | Et | O |
| C-0456 | H | CH(Me) | H | *c*-Pr | H | Cl | Et | O |
| C-0457 | H | CH(Me) | Me | *c*-Pr | H | Cl | Et | O |
| C-0458 | Me | CH(Me) | H | *c*-Pr | H | Cl | Et | O |
| C-0459 | Me | CH(Me) | Me | *c*-Pr | H | Cl | Et | O |
| C-0460 | H | CH₂CH₂ | Me | *c*-Pr | H | Cl | Et | O |
| C-0461 | Me | CH₂CH₂ | H | *c*-Pr | H | Cl | Et | O |
| C-0462 | Me | CH₂CH₂ | Me | *c*-Pr | H | Cl | Et | O |
| C-0463 | H | single bond | Me | *c*-Pr | H | Me | Et | O |
| C-0464 | Me | single bond | H | *c*-Pr | H | Me | Et | O |
| C-0465 | Me(R) | single bond | H | *c*-Pr | H | Me | Et | O |
| C-0466 | Me(S) | single bond | H | *c*-Pr | H | Me | Et | O |
| C-0467 | Me | single bond | H | *c*-Pr | H | Me | Et | S |
| C-0468 | Me(R) | single bond | H | *c*-Pr | H | Me | Et | S |
| C-0469 | Me(S) | single bond | H | *c*-Pr | H | Me | Et | S |
| C-0470 | Me | single bond | Me | *c*-Pr | H | Me | Et | O |
| C-0471 | H | CH₂ | Me | *c*-Pr | H | Me | Et | O |
| C-0472 | Me | CH₂ | H | *c*-Pr | H | Me | Et | O |
| C-0473 | Me | CH₂ | Me | *c*-Pr | H | Me | Et | O |
| C-0474 | H | CH(Me) | H | *c*-Pr | H | Me | Et | O |
| C-0475 | H | CH(Me) | Me | *c*-Pr | H | Me | Et | O |
| C-0476 | Me | CH(Me) | H | *c*-Pr | H | Me | Et | O |
| C-0477 | Me | CH(Me) | Me | *c*-Pr | H | Me | Et | O |
| C-0478 | H | CH₂CH₂ | Me | *c*-Pr | H | Me | Et | O |

**[Table 238]**

| Compound Number | Y | W | Z | R¹ | R² | R³ | R¹⁰ | A |
|---|---|---|---|---|---|---|---|---|
| C-0479 | Me | CH₂CH₂ | H | *c-*Pr | H | Me | Et | O |
| C-0480 | Me | CH₂CH₂ | Me | *c*-Pr | H | Me | Et | O |
| C-0481 | H | single bond | Me | CH₂CF₃ | H | Cl | CH₂CF₃ | O |
| C-0482 | Me | single bond | H | CH₂CF₃ | H | Cl | CH₂CF₃ | O |
| C-0483 | Me(R) | single bond | H | CH₂CF₃ | H | Cl | CH₂CF₃ | O |
| C-0484 | Me(S) | single bond | H | CH₂CF₃ | H | Cl | CH₂CF₃ | O |
| C-0485 | Me | single bond | H | CH₂CF₃ | H | Cl | CH₂CF₃ | S |
| C-0486 | Me(R) | single bond | H | CH₂CF₃ | H | Cl | CH₂CF₃ | S |
| C-0487 | Me(S) | single bond | H | CH₂CF₃ | H | Cl | CH₂CF₃ | S |
| C-0488 | Me | single bond | Me | CH₂CF₃ | H | Cl | CH₂CF₃ | O |
| C-0489 | H | CH₂ | Me | CH₂CF₃ | H | Cl | CH₂CF₃ | O |
| C-0490 | Me | CH₂ | H | CH₂CF₃ | H | Cl | CH₂CF₃ | O |
| C-0491 | Me | CH₂ | Me | CH₂CF₃ | H | Cl | CH₂CF₃ | O |
| C-0492 | H | CH(Me) | H | CH₂CF₃ | H | Cl | CH₂CF₃ | O |
| C-0493 | H | CH(Me) | Me | CH₂CF₃ | H | Cl | CH₂CF₃ | O |
| C-0494 | Me | CH(Me) | H | CH₂CF₃ | H | Cl | CH₂CF₃ | O |
| C-0495 | Me | CH(Me) | Me | CH₂CF₃ | H | Cl | CH₂CF₃ | O |
| C-0496 | H | CH₂CH₂ | Me | CH₂CF₃ | H | Cl | CH₂CF₃ | O |
| C-0497 | Me | CH₂CH₂ | H | CH₂CF₃ | H | Cl | CH₂CF₃ | O |
| C-0498 | Me | CH₂CH₂ | Me | CH₂CF₃ | H | Cl | CH₂CF₃ | O |
| C-0499 | H | single bond | Me | CH₂CF₃ | H | Me | CH₂CF₃ | O |
| C-0500 | Me | single bond | H | CH₂CF₃ | H | Me | CH₂CF₃ | O |
| C-0501 | Me(R) | single bond | H | CH₂CF₃ | H | Me | CH₂CF₃ | O |
| C-0502 | Me(S) | single bond | H | CH₂CF₃ | H | Me | CH₂CF₃ | O |
| C-0503 | Me | single bond | H | CH₂CF₃ | H | Me | CH₂CF₃ | S |
| C-0504 | Me(R) | single bond | H | CH₂CF₃ | H | Me | CH₂CF₃ | S |
| C-0505 | Me(S) | single bond | H | CH₂CF₃ | H | Me | CH₂CF₃ | S |
| C-0506 | Me | single bond | Me | CH₂CF₃ | H | Me | CH₂CF₃ | O |
| C-0507 | H | CH₂ | Me | CH₂CF₃ | H | Me | CH₂CF₃ | O |
| C-0508 | Me | CH₂ | H | CH₂CF₃ | H | Me | CH₂CF₃ | O |
| C-0509 | Me | CH₂ | Me | CH₂CF₃ | H | Me | CH₂CF₃ | O |
| C-0510 | H | CH(Me) | H | CH₂CF₃ | H | Me | CH₂CF₃ | O |
| C-0511 | H | CH(Me) | Me | CH₂CF₃ | H | Me | CH₂CF₃ | O |
| C-0512 | Me | CH(Me) | H | CH₂CF₃ | H | Me | CH₂CF₃ | O |
| C-0513 | Me | CH(Me) | Me | CH₂CF₃ | H | Me | CH₂CF₃ | O |
| C-0514 | H | CH₂CH₂ | Me | CH₂CF₃ | H | Me | CH₂CF₃ | O |
| C-0515 | Me | CH₂CH₂ | H | CH₂CF₃ | H | Me | CH₂CF₃ | O |
| C-0516 | Me | CH₂CH₂ | Me | CH₂CF₃ | H | Me | CH₂CF₃ | O |
| C-0517 | H | single bond | Me | Et | H | Cl | CH₂CF₃ | O |
| C-0518 | Me | single bond | H | Et | H | Cl | CH₂CF₃ | O |
| C-0519 | Me(R) | single bond | H | Et | H | Cl | CH₂CF₃ | O |
| C-0520 | Me(S) | single bond | H | Et | H | Cl | CH₂CF₃ | O |
| C-0521 | Me | single bond | H | Et | H | Cl | CH₂CF₃ | S |
| C-0522 | Me(R) | single bond | H | Et | H | Cl | CH₂CF₃ | S |

**[Table 239]**

| Compound Number | Y | W | Z | R¹ | R² | R³ | R¹⁰ | A |
|---|---|---|---|---|---|---|---|---|
| C-0523 | Me(S) | single bond | H | Et | H | Cl | CH₂CF₃ | S |
| C-0524 | Me | single bond | Me | Et | H | Cl | CH₂CF₃ | O |
| C-0525 | H | CH₂ | Me | Et | H | Cl | CH₂CF₃ | O |
| C-0526 | Me | CH₂ | H | Et | H | Cl | CH₂CF₃ | O |
| C-0527 | Me | CH₂ | Me | Et | H | Cl | CH₂CF₃ | O |
| C-0528 | H | CH(Me) | H | Et | H | Cl | CH₂CF₃ | O |
| C-0529 | H | CH(Me) | Me | Et | H | Cl | CH₂CF₃ | O |
| C-0530 | Me | CH(Me) | H | Et | H | Cl | CH₂CF₃ | O |
| C-0531 | Me | CH(Me) | Me | Et | H | Cl | CH₂CF₃ | O |
| C-0532 | H | CH₂CH₂ | Me | Et | H | Cl | CH₂CF₃ | O |
| C-0533 | Me | CH₂CH₂ | H | Et | H | Cl | CH₂CF₃ | O |
| C-0534 | Me | CH₂CH₂ | Me | Et | H | Cl | CH₂CF₃ | O |
| C-0535 | H | single bond | Me | Et | H | Me | CH₂CF₃ | O |
| C-0536 | Me | single bond | H | Et | H | Me | CH₂CF₃ | O |
| C-0537 | Me(R) | single bond | H | Et | H | Me | CH₂CF₃ | O |
| C-0538 | Me(S) | single bond | H | Et | H | Me | CH₂CF₃ | O |
| C-0539 | Me | single bond | H | Et | H | Me | CH₂CF₃ | S |
| C-0540 | Me(R) | single bond | H | Et | H | Me | CH₂CF₃ | S |
| C-0541 | Me(S) | single bond | H | Et | H | Me | CH₂CF₃ | S |
| C-0542 | Me | single bond | Me | Et | H | Me | CH₂CF₃ | O |
| C-0543 | H | CH₂ | Me | Et | H | Me | CH₂CF₃ | O |
| C-0544 | Me | CH₂ | H | Et | H | Me | CH₂CF₃ | O |
| C-0545 | Me | CH₂ | Me | Et | H | Me | CH₂CF₃ | O |
| C-0546 | H | CH(Me) | H | Et | H | Me | CH₂CF₃ | O |
| C-0547 | H | CH(Me) | Me | Et | H | Me | CH₂CF₃ | O |
| C-0548 | Me | CH(Me) | H | Et | H | Me | CH₂CF₃ | O |
| C-0549 | Me | CH(Me) | Me | Et | H | Me | CH₂CF₃ | O |
| C-0550 | H | CH₂CH₂ | Me | Et | H | Me | CH₂CF₃ | O |
| C-0551 | Me | CH₂CH₂ | H | Et | H | Me | CH₂CF₃ | O |
| C-0552 | Me | CH₂CH₂ | Me | Et | H | Me | CH₂CF₃ | O |
| C-0553 | H | single bond | Me | *c*-Pr | H | Cl | CH₂CF₃ | O |
| C-0554 | Me | single bond | H | *c*-Pr | H | Cl | CH₂CF₃ | O |
| C-0555 | Me(R) | single bond | H | *c*-Pr | H | Cl | CH₂CF₃ | O |
| C-0556 | Me(S) | single bond | H | *c*-Pr | H | Cl | CH₂CF₃ | O |
| C-0557 | Me | single bond | H | *c*-Pr | H | Cl | CH₂CF₃ | S |
| C-0558 | Me(R) | single bond | H | *c*-Pr | H | Cl | CH₂CF₃ | S |
| C-0559 | Me(S) | single bond | H | *c*-Pr | H | Cl | CH₂CF₃ | S |
| C-0560 | Me | single bond | Me | c-Pr | H | Cl | CH₂CF₃ | O |
| C-0561 | H | CH₂ | Me | *c*-Pr | H | Cl | CH₂CF₃ | O |
| C-0562 | Me | CH₂ | H | *c*-Pr | H | Cl | CH₂CF₃ | O |
| C-0563 | Me | CH₂ | Me | *c*-Pr | H | Cl | CH₂CF₃ | O |
| C-0564 | H | CH(Me) | H | *c*-Pr | H | Cl | CH₂CF₃ | O |
| C-0565 | H | CH(Me) | Me | *c*-Pr | H | Cl | CH₂CF₃ | O |
| C-0566 | Me | CH(Me) | H | *c*-Pr | H | Cl | CH₂CF₃ | O |

**[Table 240]**

| Compound Number | Y | W | Z | R¹ | R² | R³ | R¹⁰ | A |
|---|---|---|---|---|---|---|---|---|
| C-0567 | Me | CH(Me) | Me | *c*-Pr | H | Cl | CH₂CF₃ | O |
| C-0568 | H | CH₂CH₂ | Me | *c*-Pr | H | Cl | CH₂CF₃ | O |
| C-0569 | Me | CH₂CH₂ | H | *c*-Pr | H | Cl | CH₂CF₃ | O |
| C-0570 | Me | CH₂CH₂ | Me | *c*-Pr | H | Cl | CH₂CF₃ | O |
| C-0571 | H | single bond | Me | *c*-Pr | H | Me | CH₂CF₃ | O |
| C-0572 | Me | single bond | H | *c*-Pr | H | Me | CH₂CF₃ | O |
| C-0573 | Me(R) | single bond | H | *c*-Pr | H | Me | CH₂CF₃ | O |
| C-0574 | Me(S) | single bond | H | *c*-Pr | H | Me | CH₂CF₃ | O |
| C-0575 | Me | single bond | H | *c*-Pr | H | Me | CH₂CF₃ | S |
| C-0576 | Me(R) | single bond | H | *c*-Pr | H | Me | CH₂CF₃ | S |
| C-0577 | Me(S) | single bond | H | *c*-Pr | H | Me | CH₂CF₃ | S |
| C-0578 | Me | single bond | Me | *c*-Pr | H | Me | CH₂CF₃ | O |
| C-0579 | H | CH₂ | Me | *c*-Pr | H | Me | CH₂CF₃ | O |
| C-0580 | Me | CH₂ | H | *c*-Pr | H | Me | CH₂CF₃ | O |
| C-0581 | Me | CH₂ | Me | *c*-Pr | H | Me | CH₂CF₃ | O |
| C-0582 | H | CH(Me) | H | *c*-Pr | H | Me | CH₂CF₃ | O |
| C-0583 | H | CH(Me) | Me | *c*-Pr | H | Me | CH₂CF₃ | O |
| C-0584 | Me | CH(Me) | H | *c*-Pr | H | Me | CH₂CF₃ | O |
| C-0585 | Me | CH(Me) | Me | *c*-Pr | H | Me | CH₂CF₃ | O |
| C-0586 | H | CH₂CH₂ | Me | *c*-Pr | H | Me | CH₂CF₃ | O |
| C-0587 | Me | CH₂CH₂ | H | *c*-Pr | H | Me | CH₂CF₃ | O |
| C-0588 | Me | CH₂CH₂ | Me | *c*-Pr | H | Me | CH₂CF₃ | O |
| C-0589 | H | single bond | Me | CH₂CF₃ | H | Cl | CH₂OMe | O |
| C-0590 | Me | single bond | H | CH₂CF₃ | H | Cl | CH₂OMe | O |
| C-0591 | Me(R) | single bond | H | CH₂CF₃ | H | Cl | CH₂OMe | O |
| C-0592 | Me(S) | single bond | H | CH₂CF₃ | H | Cl | CH₂OMe | O |
| C-0593 | Me | single bond | H | CH₂CF₃ | H | Cl | CH₂OMe | S |
| C-0594 | Me(R) | single bond | H | CH₂CF₃ | H | Cl | CH₂OMe | S |
| C-0595 | Me(S) | single bond | H | CH₂CF₃ | H | Cl | CH₂OMe | S |
| C-0596 | Me | single bond | Me | CH₂CF₃ | H | Cl | CH₂OMe | O |
| C-0597 | H | CH₂ | Me | CH₂CF₃ | H | Cl | CH₂OMe | O |
| C-0598 | Me | CH₂ | H | CH₂CF₃ | H | Cl | CH₂OMe | O |
| C-0599 | Me | CH₂ | Me | CH₂CF₃ | H | Cl | CH₂OMe | O |
| C-0600 | H | CH(Me) | H | CH₂CF₃ | H | Cl | CH₂OMe | O |
| C-0601 | H | CH(Me) | Me | CH₂CF₃ | H | Cl | CH₂OMe | O |
| C-0602 | Me | CH(Me) | H | CH₂CF₃ | H | Cl | CH₂OMe | O |
| C-0603 | Me | CH(Me) | Me | CH₂CF₃ | H | Cl | CH₂OMe | O |
| C-0604 | H | CH₂CH₂ | Me | CH₂CF₃ | H | Cl | CH₂OMe | O |
| C-0605 | Me | CH₂CH₂ | H | CH₂CF₃ | H | Cl | CH₂OMe | O |
| C-0606 | Me | CH₂CH₂ | Me | CH₂CF₃ | H | Cl | CH₂OMe | O |
| C-0607 | H | single bond | Me | CH₂CF₃ | H | Me | CH₂OMe | O |
| C-0608 | Me | single bond | H | CH₂CF₃ | H | Me | CH₂OMe | O |
| C-0609 | Me(R) | single bond | H | CH₂CF₃ | H | Me | CH₂OMe | O |
| C-0610 | Me(S) | single bond | H | CH₂CF₃ | H | Me | CH₂OMe | O |

**[Table 241]**

| Compound Number | Y | W | z | R¹ | R² | R³ | R¹⁰ | A |
|---|---|---|---|---|---|---|---|---|
| C-0611 | Me | single bond | H | CH₂CF₃ | H | Me | CH₂OMe | S |
| C-0612 | Me(R) | single bond | H | CH₂CF₃ | H | Me | CH₂OMe | S |
| C-0613 | Me(S) | single bond | H | CH₂CF₃ | H | Me | CH₂OMe | S |
| C-0614 | Me | single bond | Me | CH₂CF₃ | H | Me | CH₂OMe | O |
| C-0615 | H | CH₂ | Me | CH₂CF₃ | H | Me | CH₂OMe | O |
| C-0616 | Me | CH₂ | H | CH₂CF₃ | H | Me | CH₂OMe | O |
| C-0617 | Me | CH₂ | Me | CH₂CF₃ | H | Me | CH₂OMe | O |
| C-0618 | H | CH(Me) | H | CH₂CF₃ | H | Me | CH₂OMe | O |
| C-0619 | H | CH(Me) | Me | CH₂CF₃ | H | Me | CH₂OMe | O |
| C-0620 | Me | CH(Me) | H | CH₂CF₃ | H | Me | CH₂OMe | O |
| C-0621 | Me | CH(Me) | Me | CH₂CF₃ | H | Me | CH₂OMe | O |
| C-0622 | H | CH₂CH₂ | Me | CH₂CF₃ | H | Me | CH₂OMe | O |
| C-0623 | Me | CH₂CH₂ | H | CH₂CF₃ | H | Me | CH₂OMe | O |
| C-0624 | Me | CH₂CH₂ | Me | CH₂CF₃ | H | Me | CH₂OMe | O |
| C-0625 | H | single bond | Me | Et | H | Cl | CH₂OMe | O |
| C-0626 | Me | single bond | H | Et | H | Cl | CH₂OMe | O |
| C-0627 | Me(R) | single bond | H | Et | H | Cl | CH₂OMe | O |
| C-0628 | Me(S) | single bond | H | Et | H | Cl | CH₂OMe | O |
| C-0629 | Me | single bond | H | Et | H | Cl | CH₂OMe | S |
| C-0630 | Me(R) | single bond | H | Et | H | Cl | CH₂OMe | S |
| C-0631 | Me(S) | single bond | H | Et | H | Cl | CH₂OMe | S |
| C-0632 | Me | single bond | Me | Et | H | Cl | CH₂OMe | O |
| C-0633 | H | CH₂ | Me | Et | H | Cl | CH₂OMe | O |
| C-0634 | Me | CH₂ | H | Et | H | Cl | CH₂OMe | O |
| C-0635 | Me | CH₂ | Me | Et | H | Cl | CH₂OMe | O |
| C-0636 | H | CH(Me) | H | Et | H | Cl | CH₂OMe | O |
| C-0637 | H | CH(Me) | Me | Et | H | Cl | CH₂OMe | O |
| C-0638 | Me | CH(Me) | H | Et | H | Cl | CH₂OMe | O |
| C-0639 | Me | CH(Me) | Me | Et | H | Cl | CH₂OMe | O |
| C-0640 | H | CH₂CH₂ | Me | Et | H | Cl | CH₂OMe | O |
| C-0641 | Me | CH₂CH₂ | H | Et | H | Cl | CH₂OMe | O |
| C-0642 | Me | CH₂CH₂ | Me | Et | H | Cl | CH₂OMe | O |
| C-0643 | H | single bond | Me | Et | H | Me | CH₂OMe | O |
| C-0644 | Me | single bond | H | Et | H | Me | CH₂OMe | O |
| C-0645 | Me(R) | single bond | H | Et | H | Me | CH₂OMe | O |
| C-0646 | Me(S) | single bond | H | Et | H | Me | CH₂OMe | O |
| C-0647 | Me | single bond | H | Et | H | Me | CH₂OMe | S |
| C-0648 | Me(R) | single bond | H | Et | H | Me | CH₂OMe | S |
| C-0649 | Me(S) | single bond | H | Et | H | Me | CH₂OMe | S |
| C-0650 | Me | single bond | Me | Et | H | Me | CH₂OMe | O |
| C-0651 | H | CH₂ | Me | Et | H | Me | CH₂OMe | O |
| C-0652 | Me | CH₂ | H | Et | H | Me | CH₂OMe | O |
| C-0653 | Me | CH₂ | Me | Et | H | Me | CH₂OMe | O |
| C-0654 | H | CH(Me) | H | Et | H | Me | CH₂OMe | O |

**[Table 242]**

| Compound Number | Y | W | Z | R¹ | R² | R³ | R¹⁰ | A |
|---|---|---|---|---|---|---|---|---|
| C-0655 | H | CH(Me) | Me | Et | H | Me | CH₂OMe | O |
| C-0656 | Me | CH(Me) | H | Et | H | Me | CH₂OMe | O |
| C-0657 | Me | CH(Me) | Me | Et | H | Me | CH₂OMe | O |
| C-0658 | H | CH₂CH₂ | Me | Et | H | Me | CH₂OMe | O |
| C-0659 | Me | CH₂CH₂ | H | Et | H | Me | CH₂OMe | O |
| C-0660 | Me | CH₂CH₂ | Me | Et | H | Me | CH₂OMe | O |
| C-0661 | H | single bond | Me | *c*-Pr | H | Cl | CH₂OMe | O |
| C-0662 | Me | single bond | H | *c*-Pr | H | Cl | CH₂OMe | O |
| C-0663 | Me(R) | single bond | H | *c*-Pr | H | Cl | CH₂OMe | O |
| C-0664 | Me(S) | single bond | H | *c*-Pr | H | Cl | CH₂OMe | O |
| C-0665 | Me | single bond | H | *c*-Pr | H | Cl | CH₂OMe | S |
| C-0666 | Me(R) | single bond | H | *c*-Pr | H | Cl | CH₂OMe | S |
| C-0667 | Me(S) | single bond | H | *c*-Pr | H | Cl | CH₂OMe | S |
| C-0668 | Me | single bond | Me | *c*-Pr | H | Cl | CH₂OMe | O |
| C-0669 | H | CH₂ | Me | *c*-Pr | H | Cl | CH₂OMe | O |
| C-0670 | Me | CH₂ | H | *c*-Pr | H | Cl | CH₂OMe | O |
| C-0671 | Me | CH₂ | Me | *c*-Pr | H | Cl | CH₂OMe | O |
| C-0672 | H | CH(Me) | H | *c*-Pr | H | Cl | CH₂OMe | O |
| C-0673 | H | CH(Me) | Me | *c*-Pr | H | Cl | CH₂OMe | O |
| C-0674 | Me | CH(Me) | H | *c*-Pr | H | Cl | CH₂OMe | O |
| C-0675 | Me | CH(Me) | Me | *c*-Pr | H | Cl | CH₂OMe | O |
| C-0676 | H | CH₂CH₂ | Me | *c*-Pr | H | Cl | CH₂OMe | O |
| C-0677 | Me | CH₂CH₂ | H | *c*-Pr | H | Cl | CH₂OMe | O |
| C-0678 | Me | CH₂CH₂ | Me | *c*-Pr | H | Cl | CH₂OMe | O |
| C-0679 | H | single bond | Me | *c*-Pr | H | Me | CH₂OMe | O |
| C-0680 | Me | single bond | H | *c*-Pr | H | Me | CH₂OMe | O |
| C-0681 | Me(R) | single bond | H | *c*-Pr | H | Me | CH₂OMe | O |
| C-0682 | Me(S) | single bond | H | *c*-Pr | H | Me | CH₂OMe | O |
| C-0683 | Me | single bond | H | *c*-Pr | H | Me | CH₂OMe | S |
| C-0684 | Me(R) | single bond | H | *c*-Pr | H | Me | CH₂OMe | S |
| C-0685 | Me(S) | single bond | H | *c*-Pr | H | Me | CH₂OMe | S |
| C-0686 | Me | single bond | Me | *c*-Pr | H | Me | CH₂OMe | O |
| C-0687 | H | CH₂ | Me | *c*-Pr | H | Me | CH₂OMe | O |
| C-0688 | Me | CH₂ | H | *c*-Pr | H | Me | CH₂OMe | O |
| C-0689 | Me | CH₂ | Me | *c*-Pr | H | Me | CH₂OMe | O |
| C-0690 | H | CH(Me) | H | *c*-Pr | H | Me | CH₂OMe | O |
| C-0691 | H | CH(Me) | Me | *c*-Pr | H | Me | CH₂OMe | O |
| C-0692 | Me | CH(Me) | H | *c*-Pr | H | Me | CH₂OMe | O |
| C-0693 | Me | CH(Me) | Me | *c*-Pr | H | Me | CH₂OMe | O |
| C-0694 | H | CH₂CH₂ | Me | *c*-Pr | H | Me | CH₂OMe | O |
| C-0695 | Me | CH₂CH₂ | H | *c*-Pr | H | Me | CH₂OMe | O |
| C-0696 | Me | CH₂CH₂ | Me | *c*-Pr | H | Me | CH₂OMe | O |
| C-0697 | H | single bond | Me | CH₂CF₃ | H | Cl | CH₂CN | O |
| C-0698 | Me | single bond | H | CH₂CF₃ | H | Cl | CH₂CN | O |

**[Table 243]**

| Compound Number | Y | W | Z | R¹ | R² | R³ | R¹⁰ | A |
|---|---|---|---|---|---|---|---|---|
| C-0699 | Me(R) | single bond | H | CH₂CF₃ | H | Cl | CH₂CN | O |
| C-0700 | Me(S) | single bond | H | CH₂CF₃ | H | Cl | CH₂CN | O |
| C-0701 | Me | single bond | H | CH₂CF₃ | H | Cl | CH₂CN | S |
| C-0702 | Me(R) | single bond | H | CH₂CF₃ | H | Cl | CH₂CN | S |
| C-0703 | Me(S) | single bond | H | CH₂CF₃ | H | Cl | CH₂CN | S |
| C-0704 | Me | single bond | Me | CH₂CF₃ | H | Cl | CH₂CN | O |
| C-0705 | H | CH₂ | Me | CH₂CF₃ | H | Cl | CH₂CN | O |
| C-0706 | Me | CH₂ | H | CH₂CF₃ | H | Cl | CH₂CN | O |
| C-0707 | Me | CH₂ | Me | CH₂CF₃ | H | Cl | CH₂CN | O |
| C-0708 | H | CH(Me) | H | CH₂CF₃ | H | Cl | CH₂CN | O |
| C-0709 | H | CH(Me) | Me | CH₂CF₃ | H | Cl | CH₂CN | O |
| C-0710 | Me | CH(Me) | H | CH₂CF₃ | H | Cl | CH₂CN | O |
| C-0711 | Me | CH(Me) | Me | CH₂CF₃ | H | Cl | CH₂CN | O |
| C-0712 | H | CH₂CH₂ | Me | CH₂CF₃ | H | Cl | CH₂CN | O |
| C-0713 | Me | CH₂CH₂ | H | CH₂CF₃ | H | Cl | CH₂CN | O |
| C-0714 | Me | CH₂CH₂ | Me | CH₂CF₃ | H | Cl | CH₂CN | O |
| C-0715 | H | single bond | Me | CH₂CF₃ | H | Me | CH₂CN | O |
| C-0716 | Me | single bond | H | CH₂CF₃ | H | Me | CH₂CN | O |
| C-0717 | Me(R) | single bond | H | CH₂CF₃ | H | Me | CH₂CN | O |
| C-0718 | Me(S) | single bond | H | CH₂CF₃ | H | Me | CH₂CN | O |
| C-0719 | Me | single bond | H | CH₂CF₃ | H | Me | CH₂CN | S |
| C-0720 | Me(R) | single bond | H | CH₂CF₃ | H | Me | CH₂CN | S |
| C-0721 | Me(S) | single bond | H | CH₂CF₃ | H | Me | CH₂CN | S |
| C-0722 | Me | single bond | Me | CH₂CF₃ | H | Me | CH₂CN | O |
| C-0723 | H | CH₂ | Me | CH₂CF₃ | H | Me | CH₂CN | O |
| C-0724 | Me | CH₂ | H | CH₂CF₃ | H | Me | CH₂CN | O |
| C-0725 | Me | CH₂ | Me | CH₂CF₃ | H | Me | CH₂CN | O |
| C-0726 | H | CH(Me) | H | CH₂CF₃ | H | Me | CH₂CN | O |
| C-0727 | H | CH(Me) | Me | CH₂CF₃ | H | Me | CH₂CN | O |
| C-0728 | Me | CH(Me) | H | CH₂CF₃ | H | Me | CH₂CN | O |
| C-0729 | Me | CH(Me) | Me | CH₂CF₃ | H | Me | CH₂CN | O |
| C-0730 | H | CH₂CH₂ | Me | CH₂CF₃ | H | Me | CH₂CN | O |
| C-0731 | Me | CH₂CH₂ | H | CH₂CF₃ | H | Me | CH₂CN | O |
| C-0732 | Me | CH₂CH₂ | Me | CH₂CF₃ | H | Me | CH₂CN | O |
| C-0733 | H | single bond | Me | Et | H | Cl | CH₂CN | O |
| C-0734 | Me | single bond | H | Et | H | Cl | CH₂CN | O |
| C-0735 | Me(R) | single bond | H | Et | H | Cl | CH₂CN | O |
| C-0736 | Me(S) | single bond | H | Et | H | Cl | CH₂CN | O |
| C-0737 | Me | single bond | H | Et | H | Cl | CH₂CN | S |
| C-0738 | Me(R) | single bond | H | Et | H | Cl | CH₂CN | S |
| C-0739 | Me(S) | single bond | H | Et | H | Cl | CH₂CN | S |
| C-0740 | Me | single bond | Me | Et | H | Cl | CH₂CN | O |
| C-0741 | H | CH₂ | Me | Et | H | Cl | CH₂CN | O |
| C-0742 | Me | CH₂ | H | Et | H | Cl | CH₂CN | O |

**[Table 244]**

| Compound Number | Y | W | Z | R¹ | R² | R³ | R¹⁰ | A |
|---|---|---|---|---|---|---|---|---|
| C-0743 | Me | CH₂ | Me | Et | H | Cl | CH₂CN | O |
| C-0744 | H | CH(Me) | H | Et | H | Cl | CH₂CN | O |
| C-0745 | H | CH(Me) | Me | Et | H | Cl | CH₂CN | O |
| C-0746 | Me | CH(Me) | H | Et | H | Cl | CH₂CN | O |
| C-0747 | Me | CH(Me) | Me | Et | H | Cl | CH₂CN | O |
| C-0748 | H | CH₂CH₂ | Me | Et | H | Cl | CH₂CN | O |
| C-0749 | Me | CH₂CH₂ | H | Et | H | Cl | CH₂CN | O |
| C-0750 | Me | CH₂CH₂ | Me | Et | H | Cl | CH₂CN | O |
| C-0751 | H | single bond | Me | Et | H | Me | CH₂CN | O |
| C-0752 | Me | single bond | H | Et | H | Me | CH₂CN | O |
| C-0753 | Me(R) | single bond | H | Et | H | Me | CH₂CN | O |
| C-0754 | Me(S) | single bond | H | Et | H | Me | CH₂CN | O |
| C-0755 | Me | single bond | H | Et | H | Me | CH₂CN | S |
| C-0756 | Me(R) | single bond | H | Et | H | Me | CH₂CN | S |
| C-0757 | Me(S) | single bond | H | Et | H | Me | CH₂CN | S |
| C-0758 | Me | single bond | Me | Et | H | Me | CH₂CN | O |
| C-0759 | H | CH₂ | Me | Et | H | Me | CH₂CN | O |
| C-0760 | Me | CH₂ | H | Et | H | Me | CH₂CN | O |
| C-0761 | Me | CH₂ | Me | Et | H | Me | CH₂CN | O |
| C-0762 | H | CH(Me) | H | Et | H | Me | CH₂CN | O |
| C-0763 | H | CH(Me) | Me | Et | H | Me | CH₂CN | O |
| C-0764 | Me | CH(Me) | H | Et | H | Me | CH₂CN | O |
| C-0765 | Me | CH(Me) | Me | Et | H | Me | CH₂CN | O |
| C-0766 | H | CH₂CH₂ | Me | Et | H | Me | CH₂CN | O |
| C-0767 | Me | CH₂CH₂ | H | Et | H | Me | CH₂CN | O |
| C-0768 | Me | CH₂CH₂ | Me | Et | H | Me | CH₂CN | O |
| C-0769 | H | single bond | Me | *c*-Pr | H | Cl | CH₂CN | O |
| C-0770 | Me | single bond | H | *c*-Pr | H | Cl | CH₂CN | O |
| C-0771 | Me(R) | single bond | H | *c*-Pr | H | Cl | CH₂CN | O |
| C-0772 | Me(S) | single bond | H | *c*-Pr | H | Cl | CH₂CN | O |
| C-0773 | Me | single bond | H | *c*-Pr | H | Cl | CH₂CN | S |
| C-0774 | Me(R) | single bond | H | *c*-Pr | H | Cl | CH₂CN | S |
| C-0775 | Me(S) | single bond | H | *c*-Pr | H | Cl | CH₂CN | S |
| C-0776 | Me | single bond | Me | *c*-Pr | H | Cl | CH₂CN | O |
| C-0777 | H | CH₂ | Me | *c*-Pr | H | Cl | CH₂CN | O |
| C-0778 | Me | CH₂ | H | *c*-Pr | H | Cl | CH₂CN | O |
| C-0779 | Me | CH₂ | Me | *c*-Pr | H | Cl | CH₂CN | O |
| C-0780 | H | CH(Me) | H | *c*-Pr | H | Cl | CH₂CN | O |
| C-0781 | H | CH(Me) | Me | *c*-Pr | H | Cl | CH₂CN | O |
| C-0782 | Me | CH(Me) | H | *c*-Pr | H | Cl | CH₂CN | O |
| C-0783 | Me | CH(Me) | Me | *c*-Pr | H | Cl | CH₂CN | O |
| C-0784 | H | CH₂CH₂ | Me | *c*-Pr | H | Cl | CH₂CN | O |
| C-0785 | Me | CH₂CH₂ | H | *c*-Pr | H | Cl | CH₂CN | O |
| C-0786 | Me | CH₂CH₂ | Me | *c*-Pr | H | Cl | CH₂CN | O |

**[Table 245]**

| Compound Number | Y | W | Z | R¹ | R² | R³ | R¹⁰ | A |
|---|---|---|---|---|---|---|---|---|
| C-0787 | H | single bond | Me | *c*-Pr | H | Me | CH₂CN | O |
| C-0788 | Me | single bond | H | *c*-Pr | H | Me | CH₂CN | O |
| C-0789 | Me(R) | single bond | H | *c*-Pr | H | Me | CH₂CN | O |
| C-0790 | Me(S) | single bond | H | *c*-Pr | H | Me | CH₂CN | O |
| C-0791 | Me | single bond | H | *c*-Pr | H | Me | CH₂CN | S |
| C-0792 | Me(R) | single bond | H | *c*-Pr | H | Me | CH₂CN | S |
| C-0793 | Me(S) | single bond | H | *c*-Pr | H | Me | CH₂CN | S |
| C-0794 | Me | single bond | Me | *c*-Pr | H | Me | CH₂CN | O |
| C-0795 | H | CH₂ | Me | *c*-Pr | H | Me | CH₂CN | O |
| C-0796 | Me | CH₂ | H | *c*-Pr | H | Me | CH₂CN | O |
| C-0797 | Me | CH₂ | Me | *c*-Pr | H | Me | CH₂CN | O |
| C-0798 | H | CH(Me) | H | *c*-Pr | H | Me | CH₂CN | O |
| C-0799 | H | CH(Me) | Me | *c*-Pr | H | Me | CH₂CN | O |
| C-0800 | Me | CH(Me) | H | *c*-Pr | H | Me | CH₂CN | O |
| C-0801 | Me | CH(Me) | Me | *c*-Pr | H | Me | CH₂CN | O |
| C-0802 | H | CH₂CH₂ | Me | *c*-Pr | H | Me | CH₂CN | O |
| C-0803 | Me | CH₂CH₂ | H | *c*-Pr | H | Me | CH₂CN | O |
| C-0804 | Me | CH₂CH₂ | Me | *c*-Pr | H | Me | CH₂CN | O |
| C-0805 | H | single bond | Me | CH₂CF₃ | H | Cl | C(=O)Me | O |
| C-0806 | Me | single bond | H | CH₂CF₃ | H | Cl | C(=O)Me | O |
| C-0807 | Me(R) | single bond | H | CH₂CF₃ | H | Cl | C(=O)Me | O |
| C-0808 | Me(S) | single bond | H | CH₂CF₃ | H | Cl | C(=O)Me | O |
| C-0809 | Me | single bond | H | CH₂CF₃ | H | Cl | C(=O)Me | S |
| C-0810 | Me(R) | single bond | H | CH₂CF₃ | H | Cl | C(=O)Me | S |
| C-0811 | Me(S) | single bond | H | CH₂CF₃ | H | Cl | C(=O)Me | S |
| C-0812 | Me | single bond | Me | CH₂CF₃ | H | Cl | C(=O)Me | O |
| C-0813 | H | CH₂ | Me | CH₂CF₃ | H | Cl | C(=O)Me | O |
| C-0814 | Me | CH₂ | H | CH₂CF₃ | H | Cl | C(=O)Me | O |
| C-0815 | Me | CH₂ | Me | CH₂CF₃ | H | Cl | C(=O)Me | O |
| C-0816 | H | CH(Me) | H | CH₂CF₃ | H | Cl | C(=O)Me | O |
| C-0817 | H | CH(Me) | Me | CH₂CF₃ | H | Cl | C(=O)Me | O |
| C-0818 | Me | CH(Me) | H | CH₂CF₃ | H | Cl | C(=O)Me | O |
| C-0819 | Me | CH(Me) | Me | CH₂CF₃ | H | Cl | C(=O)Me | O |
| C-0820 | H | CH₂CH₂ | Me | CH₂CF₃ | H | Cl | C(=O)Me | O |
| C-0821 | Me | CH₂CH₂ | H | CH₂CF₃ | H | Cl | C(=O)Me | O |
| C-0822 | Me | CH₂CH₂ | Me | CH₂CF₃ | H | Cl | C(=O)Me | O |
| C-0823 | H | single bond | Me | CH₂CF₃ | H | Me | C(=O)Me | O |
| C-0824 | Me | single bond | H | CH₂CF₃ | H | Me | C(=O)Me | O |
| C-0825 | Me(R) | single bond | H | CH₂CF₃ | H | Me | C(=O)Me | O |
| C-0826 | Me(S) | single bond | H | CH₂CF₃ | H | Me | C(=O)Me | O |
| C-0827 | Me | single bond | H | CH₂CF₃ | H | Me | C(=O)Me | S |
| C-0828 | Me(R) | single bond | H | CH₂CF₃ | H | Me | C(=O)Me | S |
| C-0829 | Me(S) | single bond | H | CH₂CF₃ | H | Me | C(=O)Me | S |
| C-0830 | Me | single bond | Me | CH₂CF₃ | H | Me | C(=O)Me | O |

**[Table 246]**

| Compound Number | Y | W | Z | R¹ | R² | R³ | R¹⁰ | A |
|---|---|---|---|---|---|---|---|---|
| C-0831 | H | CH₂ | Me | CH₂CF₃ | H | Me | C(=O)Me | O |
| C-0832 | Me | CH₂ | H | CH₂CF₃ | H | Me | C(=O)Me | O |
| C-0833 | Me | CH₂ | Me | CH₂CF₃ | H | Me | C(=O)Me | O |
| C-0834 | H | CH(Me) | H | CH₂CF₃ | H | Me | C(=O)Me | O |
| C-0835 | H | CH(Me) | Me | CH₂CF₃ | H | Me | C(=O)Me | O |
| C-0836 | Me | CH(Me) | H | CH₂CF₃ | H | Me | C(=O)Me | O |
| C-0837 | Me | CH(Me) | Me | CH₂CF₃ | H | Me | C(=O)Me | O |
| C-0838 | H | CH₂CH₂ | Me | CH₂CF₃ | H | Me | C(=O)Me | O |
| C-0839 | Me | CH₂CH₂ | H | CH₂CF₃ | H | Me | C(=O)Me | O |
| C-0840 | Me | CH₂CH₂ | Me | CH₂CF₃ | H | Me | C(=O)Me | O |
| C-0841 | H | single bond | Me | Et | H | Cl | C(=O)Me | O |
| C-0842 | Me | single bond | H | Et | H | Cl | C(=O)Me | O |
| C-0843 | Me(R) | single bond | H | Et | H | Cl | C(=O)Me | O |
| C-0844 | Me(S) | single bond | H | Et | H | Cl | C(=O)Me | O |
| C-0845 | Me | single bond | H | Et | H | Cl | C(=O)Me | S |
| C-0846 | Me(R) | single bond | H | Et | H | Cl | C(=O)Me | S |
| C-0847 | Me(S) | single bond | H | Et | H | Cl | C(=O)Me | S |
| C-0848 | Me | single bond | Me | Et | H | Cl | C(=O)Me | O |
| C-0849 | H | CH₂ | Me | Et | H | Cl | C(=O)Me | O |
| C-0850 | Me | CH₂ | H | Et | H | Cl | C(=O)Me | O |
| C-0851 | Me | CH₂ | Me | Et | H | Cl | C(=O)Me | O |
| C-0852 | H | CH(Me) | H | Et | H | Cl | C(=O)Me | O |
| C-0853 | H | CH(Me) | Me | Et | H | Cl | C(=O)Me | O |
| C-0854 | Me | CH(Me) | H | Et | H | Cl | C(=O)Me | O |
| C-0855 | Me | CH(Me) | Me | Et | H | Cl | C(=O)Me | O |
| C-0856 | H | CH₂CH₂ | Me | Et | H | Cl | C(=O)Me | O |
| C-0857 | Me | CH₂CH₂ | H | Et | H | Cl | C(=O)Me | O |
| C-0858 | Me | CH₂CH₂ | Me | Et | H | Cl | C(=O)Me | O |
| C-0859 | H | single bond | Me | Et | H | Me | C(=O)Me | O |
| C-0860 | Me | single bond | H | Et | H | Me | C(=O)Me | O |
| C-0861 | Me(R) | single bond | H | Et | H | Me | C(=O)Me | O |
| C-0862 | Me(S) | single bond | H | Et | H | Me | C(=O)Me | O |
| C-0863 | Me | single bond | H | Et | H | Me | C(=O)Me | S |
| C-0864 | Me(R) | single bond | H | Et | H | Me | C(=O)Me | S |
| C-0865 | Me(S) | single bond | H | Et | H | Me | C(=O)Me | S |
| C-0866 | Me | single bond | Me | Et | H | Me | C(=O)Me | O |
| C-0867 | H | CH₂ | Me | Et | H | Me | C(=O)Me | O |
| C-0868 | Me | CH₂ | H | Et | H | Me | C(=O)Me | O |
| C-0869 | Me | CH₂ | Me | Et | H | Me | C(=O)Me | O |
| C-0870 | H | CH(Me) | H | Et | H | Me | C(=O)Me | O |
| C-0871 | H | CH(Me) | Me | Et | H | Me | C(=O)Me | O |
| C-0872 | Me | CH(Me) | H | Et | H | Me | C(=O)Me | O |
| C-0873 | Me | CH(Me) | Me | Et | H | Me | C(=O)Me | O |
| C-0874 | H | CH₂CH₂ | Me | Et | H | Me | C(=O)Me | O |

**[Table 247]**

| Compound Number | Y | W | Z | R¹ | R² | R³ | R¹⁰ | A |
|---|---|---|---|---|---|---|---|---|
| C-0875 | Me | CH₂CH₂ | H | Et | H | Me | C(=O)Me | O |
| C-0876 | Me | CH₂CH₂ | Me | Et | H | Me | C(=O)Me | O |
| C-0877 | H | single bond | Me | *c*-Pr | H | Cl | C(=O)Me | O |
| C-0878 | Me | single bond | H | *c*-Pr | H | Cl | C(=O)Me | O |
| C-0879 | Me(R) | single bond | H | *c*-Pr | H | Cl | C(=O)Me | O |
| C-0880 | Me(S) | single bond | H | *c*-Pr | H | Cl | C(=O)Me | O |
| C-0881 | Me | single bond | H | *c*-Pr | H | Cl | C(=O)Me | S |
| C-0882 | Me(R) | single bond | H | *c*-Pr | H | Cl | C(=O)Me | S |
| C-0883 | Me(S) | single bond | H | *c*-Pr | H | Cl | C(=O)Me | S |
| C-0884 | Me | single bond | Me | *c*-Pr | H | Cl | C(=O)Me | O |
| C-0885 | H | CH₂ | Me | *c*-Pr | H | Cl | C(=O)Me | O |
| C-0886 | Me | CH₂ | H | *c*-Pr | H | Cl | C(=O)Me | O |
| C-0887 | Me | CH₂ | Me | *c*-Pr | H | Cl | C(=O)Me | O |
| C-0888 | H | CH(Me) | H | *c*-Pr | H | Cl | C(=O)Me | O |
| C-0889 | H | CH(Me) | Me | *c*-Pr | H | Cl | C(=O)Me | O |
| C-0890 | Me | CH(Me) | H | *c*-Pr | H | Cl | C(=O)Me | O |
| C-0891 | Me | CH(Me) | Me | *c*-Pr | H | Cl | C(=O)Me | O |
| C-0892 | H | CH₂CH₂ | Me | *c*-Pr | H | Cl | C(=O)Me | O |
| C-0893 | Me | CH₂CH₂ | H | *c*-Pr | H | Cl | C(=O)Me | O |
| C-0894 | Me | CH₂CH₂ | Me | *c*-Pr | H | Cl | C(=O)Me | O |
| C-0895 | H | single bond | Me | *c*-Pr | H | Me | C(=O)Me | O |
| C-0896 | Me | single bond | H | *c*-Pr | H | Me | C(=O)Me | O |
| C-0897 | Me(R) | single bond | H | *c*-Pr | H | Me | C(=O)Me | O |
| C-0898 | Me(S) | single bond | H | *c*-Pr | H | Me | C(=O)Me | O |
| C-0899 | Me | single bond | H | *c*-Pr | H | Me | C(=O)Me | S |
| C-0900 | Me(R) | single bond | H | *c*-Pr | H | Me | C(=O)Me | S |
| C-0901 | Me(S) | single bond | H | *c*-Pr | H | Me | C(=O)Me | S |
| C-0902 | Me | single bond | Me | *c*-Pr | H | Me | C(=O)Me | O |
| C-0903 | H | CH₂ | Me | *c*-Pr | H | Me | C(=O)Me | O |
| C-0904 | Me | CH₂ | H | *c*-Pr | H | Me | C(=O)Me | O |
| C-0905 | Me | CH₂ | Me | *c*-Pr | H | Me | C(=O)Me | O |
| C-0906 | H | CH(Me) | H | *c*-Pr | H | Me | C(=O)Me | O |
| C-0907 | H | CH(Me) | Me | *c*-Pr | H | Me | C(=O)Me | O |
| C-0908 | Me | CH(Me) | H | *c*-Pr | H | Me | C(=O)Me | O |
| C-0909 | Me | CH(Me) | Me | *c*-Pr | H | Me | C(=O)Me | O |
| C-0910 | H | CH₂CH₂ | Me | *c*-Pr | H | Me | C(=O)Me | O |
| C-0911 | Me | CH₂CH₂ | H | *c*-Pr | H | Me | C(=O)Me | O |
| C-0912 | Me | CH₂CH₂ | Me | *c*-Pr | H | Me | C(=O)Me | O |
| C-0913 | H | single bond | Me | CH₂CF₃ | H | Cl | C(=O)OEt | O |
| C-0914 | Me | single bond | H | CH₂CF₃ | H | Cl | C(=O)OEt | O |
| C-0915 | Me(R) | single bond | H | CH₂CF₃ | H | Cl | C(=O)OEt | O |
| C-0916 | Me(S) | single bond | H | CH₂CF₃ | H | Cl | C(=O)OEt | O |
| C-0917 | Me | single bond | H | CH₂CF₃ | H | Cl | C(=O)OEt | S |
| C-0918 | Me(R) | single bond | H | CH₂CF₃ | H | Cl | C(=O)OEt | S |

**[Table 248]**

| Compound Number | Y | W | Z | R¹ | R² | R³ | R¹⁰ | A |
|---|---|---|---|---|---|---|---|---|
| C-0919 | Me(S) | single bond | H | CH₂CF₃ | H | Cl | C(=O)OEt | S |
| C-0920 | Me | single bond | Me | CH₂CF₃ | H | Cl | C(=O)OEt | O |
| C-0921 | H | CH₂ | Me | CH₂CF₃ | H | Cl | C(=O)OEt | O |
| C-0922 | Me | CH₂ | H | CH₂CF₃ | H | Cl | C(=O)OEt | O |
| C-0923 | Me | CH₂ | Me | CH₂CF₃ | H | Cl | C(=O)OEt | O |
| C-0924 | H | CH(Me) | H | CH₂CF₃ | H | Cl | C(=O)OEt | O |
| C-0925 | H | CH(Me) | Me | CH₂CF₃ | H | Cl | C(=O)OEt | O |
| C-0926 | Me | CH(Me) | H | CH₂CF₃ | H | Cl | C(=O)OEt | O |
| C-0927 | Me | CH(Me) | Me | CH₂CF₃ | H | Cl | C(=O)OEt | O |
| C-0928 | H | CH₂CH₂ | Me | CH₂CF₃ | H | Cl | C(=O)OEt | O |
| C-0929 | Me | CH₂CH₂ | H | CH₂CF₃ | H | Cl | C(=O)OEt | O |
| C-0930 | Me | CH₂CH₂ | Me | CH₂CF₃ | H | Cl | C(=O)OEt | O |
| C-0931 | H | single bond | Me | CH₂CF₃ | H | Me | C(=O)OEt | O |
| C-0932 | Me | single bond | H | CH₂CF₃ | H | Me | C(=O)OEt | O |
| C-0933 | Me(R) | single bond | H | CH₂CF₃ | H | Me | C(=O)OEt | O |
| C-0934 | Me(S) | single bond | H | CH₂CF₃ | H | Me | C(=O)OEt | O |
| C-0935 | Me | single bond | H | CH₂CF₃ | H | Me | C(=O)OEt | S |
| C-0936 | Me(R) | single bond | H | CH₂CF₃ | H | Me | C(=O)OEt | S |
| C-0937 | Me(S) | single bond | H | CH₂CF₃ | H | Me | C(=O)OEt | S |
| C-0938 | Me | single bond | Me | CH₂CF₃ | H | Me | C(=O)OEt | O |
| C-0939 | H | CH₂ | Me | CH₂CF₃ | H | Me | C(=O)OEt | O |
| C-0940 | Me | CH₂ | H | CH₂CF₃ | H | Me | C(=O)OEt | O |
| C-0941 | Me | CH₂ | Me | CH₂CF₃ | H | Me | C(=O)OEt | O |
| C-0942 | H | CH(Me) | H | CH₂CF₃ | H | Me | C(=O)OEt | O |
| C-0943 | H | CH(Me) | Me | CH₂CF₃ | H | Me | C(=O)OEt | O |
| C-0944 | Me | CH(Me) | H | CH₂CF₃ | H | Me | C(=O)OEt | O |
| C-0945 | Me | CH(Me) | Me | CH₂CF₃ | H | Me | C(=O)OEt | O |
| C-0946 | H | CH₂CH₂ | Me | CH₂CF₃ | H | Me | C(=O)OEt | O |
| C-0947 | Me | CH₂CH₂ | H | CH₂CF₃ | H | Me | C(=O)OEt | O |
| C-0948 | Me | CH₂CH₂ | Me | CH₂CF₃ | H | Me | C(=O)OEt | O |
| C-0949 | H | single bond | Me | Et | H | Cl | C(=O)OEt | O |
| C-0950 | Me | single bond | H | Et | H | Cl | C(=O)OEt | O |
| C-0951 | Me(R) | single bond | H | Et | H | Cl | C(=O)OEt | O |
| C-0952 | Me(S) | single bond | H | Et | H | Cl | C(=O)OEt | O |
| C-0953 | Me | single bond | H | Et | H | Cl | C(=O)OEt | S |
| C-0954 | Me(R) | single bond | H | Et | H | Cl | C(=O)OEt | S |
| C-0955 | Me(S) | single bond | H | Et | H | Cl | C(=O)OEt | S |
| C-0956 | Me | single bond | Me | Et | H | Cl | C(=O)OEt | O |
| C-0957 | H | CH₂ | Me | Et | H | Cl | C(=O)OEt | O |
| C-0958 | Me | CH₂ | H | Et | H | Cl | C(=O)OEt | O |
| C-0959 | Me | CH₂ | Me | Et | H | Cl | C(=O)OEt | O |
| C-0960 | H | CH(Me) | H | Et | H | Cl | C(=O)OEt | O |
| C-0961 | H | CH(Me) | Me | Et | H | Cl | C(=O)OEt | O |
| C-0962 | Me | CH(Me) | H | Et | H | Cl | C(=O)OEt | O |

**[Table 249]**

| Compound Number | Y | W | Z | R¹ | R² | R³ | R¹⁰ | A |
|---|---|---|---|---|---|---|---|---|
| C-0963 | Me | CH(Me) | Me | Et | H | Cl | C(=O)OEt | O |
| C-0964 | H | CH₂CH₂ | Me | Et | H | Cl | C(=O)OEt | O |
| C-0965 | Me | CH₂CH₂ | H | Et | H | Cl | C(=O)OEt | O |
| C-0966 | Me | CH₂CH₂ | Me | Et | H | Cl | C(=O)OEt | O |
| C-0967 | H | single bond | Me | Et | H | Me | C(=O)OEt | O |
| C-0968 | Me | single bond | H | Et | H | Me | C(=O)OEt | O |
| C-0969 | Me(R) | single bond | H | Et | H | Me | C(=O)OEt | O |
| C-0970 | Me(S) | single bond | H | Et | H | Me | C(=O)OEt | O |
| C-0971 | Me | single bond | H | Et | H | Me | C(=O)OEt | S |
| C-0972 | Me(R) | single bond | H | Et | H | Me | C(=O)OEt | S |
| C-0973 | Me(S) | single bond | H | Et | H | Me | C(=O)OEt | S |
| C-0974 | Me | single bond | Me | Et | H | Me | C(=O)OEt | O |
| C-0975 | H | CH₂ | Me | Et | H | Me | C(=O)OEt | O |
| C-0976 | Me | CH₂ | H | Et | H | Me | C(=O)OEt | O |
| C-0977 | Me | CH₂ | Me | Et | H | Me | C(=O)OEt | O |
| C-0978 | H | CH(Me) | H | Et | H | Me | C(=O)OEt | O |
| C-0979 | H | CH(Me) | Me | Et | H | Me | C(=O)OEt | O |
| C-0980 | Me | CH(Me) | H | Et | H | Me | C(=O)OEt | O |
| C-0981 | Me | CH(Me) | Me | Et | H | Me | C(=O)OEt | O |
| C-0982 | H | CH₂CH₂ | Me | Et | H | Me | C(=O)OEt | O |
| C-0983 | Me | CH₂CH₂ | H | Et | H | Me | C(=O)OEt | O |
| C-0984 | Me | CH₂CH₂ | Me | Et | H | Me | C(=O)OEt | O |
| C-0985 | H | single bond | Me | *c*-Pr | H | Cl | C(=O)OEt | O |
| C-0986 | Me | single bond | H | *c*-Pr | H | Cl | C(=O)OEt | O |
| C-0987 | Me(R) | single bond | H | *c*-Pr | H | Cl | C(=O)OEt | O |
| C-0988 | Me(S) | single bond | H | *c*-Pr | H | Cl | C(=O)OEt | O |
| C-0989 | Me | single bond | H | *c*-Pr | H | Cl | C(=O)OEt | S |
| C-0990 | Me(R) | single bond | H | *c*-Pr | H | Cl | C(=O)OEt | S |
| C-0991 | Me(S) | single bond | H | *c*-Pr | H | Cl | C(=O)OEt | S |
| C-0992 | Me | single bond | Me | *c*-Pr | H | Cl | C(=O)OEt | O |
| C-0993 | H | CH₂ | Me | *c*-Pr | H | Cl | C(=O)OEt | O |
| C-0994 | Me | CH₂ | H | *c*-Pr | H | Cl | C(=O)OEt | O |
| C-0995 | Me | CH₂ | Me | *c*-Pr | H | Cl | C(=O)OEt | O |
| C-0996 | H | CH(Me) | H | *c*-Pr | H | Cl | C(=O)OEt | O |
| C-0997 | H | CH(Me) | Me | *c*-Pr | H | Cl | C(=O)OEt | O |
| C-0998 | Me | CH(Me) | H | *c*-Pr | H | Cl | C(=O)OEt | O |
| C-0999 | Me | CH(Me) | Me | *c*-Pr | H | Cl | C(=O)OEt | O |
| C-1000 | H | CH₂CH₂ | Me | *c*-Pr | H | Cl | C(=O)OEt | O |
| C-1001 | Me | CH₂CH₂ | H | *c*-Pr | H | Cl | C(=O)OEt | O |
| C-1002 | Me | CH₂CH₂ | Me | *c*-Pr | H | Cl | C(=O)OEt | O |
| C-1003 | H | single bond | Me | *c*-Pr | H | Me | C(=O)OEt | O |
| C-1 004 | Me | single bond | H | *c*-Pr | H | Me | C(=O)OEt | O |
| C-1005 | Me(R) | single bond | H | *c*-Pr | H | Me | C(=O)OEt | O |
| C-1 006 | Me(S) | single bond | H | *c*-Pr | H | Me | C(=O)OEt | O |

**[Table 250]**

| Compound Number | Y | W | Z | R¹ | R² | R³ | R¹⁰ | A |
|---|---|---|---|---|---|---|---|---|
| C-1007 | Me | single bond | H | *c*-Pr | H | Me | C(=O)OEt | S |
| C-1008 | Me(R) | single bond | H | *c*-Pr | H | Me | C(=O)OEt | S |
| C-1009 | Me(S) | single bond | H | *c*-Pr | H | Me | C(=O)OEt | S |
| C-1010 | Me | single bond | Me | *c*-Pr | H | Me | C(=O)OEt | O |
| C-1011 | H | CH₂ | Me | *c*-Pr | H | Me | C(=O)OEt | O |
| C-1012 | Me | CH₂ | H | *c*-Pr | H | Me | C(=O)OEt | O |
| C-1013 | Me | CH₂ | Me | *c*-Pr | H | Me | C(=O)OEt | O |
| C-1014 | H | CH(Me) | H | *c*-Pr | H | Me | C(=O)OEt | O |
| C-1015 | H | CH(Me) | Me | *c*-Pr | H | Me | C(=O)OEt | O |
| C-1016 | Me | CH(Me) | H | *c*-Pr | H | Me | C(=O)OEt | O |
| C-1017 | Me | CH(Me) | Me | *c*-Pr | H | Me | C(=O)OEt | O |
| C-1018 | H | CH₂CH₂ | Me | *c*-Pr | H | Me | C(=O)OEt | O |
| C-1019 | Me | CH₂CH₂ | H | *c*-Pr | H | Me | C(=O)OEt | O |
| C-1020 | Me | CH₂CH₂ | Me | *c*-Pr | H | Me | C(=O)OEt | O |
| C-1021 | H | single bond | Me | CH₂CF₃ | Me | Cl | Me | O |
| C-1022 | H | single bond | Et | CH₂CF₃ | Me | Cl | Me | O |
| C-1023 | Me | single bond | H | CH₂CF₃ | Me | Cl | Me | O |
| C-1024 | Me(R) | single bond | H | CH₂CF₃ | Me | Cl | Me | O |
| C-1025 | Me(S) | single bond | H | CH₂CF₃ | Me | Cl | Me | O |
| C-1026 | H | CH₂ | Me | CH₂CF₃ | Me | Cl | Me | O |
| C-1027 | Me | CH₂ | H | CH₂CF₃ | Me | Cl | Me | O |
| C-1028 | H | CH(Me) | H | CH₂CF₃ | Me | Cl | Me | O |
| C-1029 | H | CH₂CH₂ | Me | CH₂CF₃ | Me | Cl | Me | O |
| C-1030 | Me | CH₂CH₂ | H | CH₂CF₃ | Me | Cl | Me | O |
| C-1031 | H | single bond | Me | CH₂CF₃ | Me | Me | Me | O |
| C-1032 | Me | single bond | H | CH₂CF₃ | Me | Me | Me | O |
| C-1033 | Me(R) | single bond | H | CH₂CF₃ | Me | Me | Me | O |
| C-1034 | Me(S) | single bond | H | CH₂CF₃ | Me | Me | Me | O |
| C-1035 | H | CH₂ | Me | CH₂CF₃ | Me | Me | Me | O |
| C-1036 | Me | CH₂ | H | CH₂CF₃ | Me | Me | Me | O |
| C-1037 | H | CH(Me) | H | CH₂CF₃ | Me | Me | Me | O |
| C-1038 | H | CH₂CH₂ | Me | CH₂CF₃ | Me | Me | Me | O |
| C-1039 | Me | CH₂CH₂ | H | CH₂CF₃ | Me | Me | Me | O |
| C-1040 | H | single bond | Me | CH₂CF₃ | Me | Cl | Et | O |
| C-1041 | Me | single bond | H | CH₂CF₃ | Me | Cl | Et | O |
| C-1042 | Me(R) | single bond | H | CH₂CF₃ | Me | Cl | Et | O |
| C-1043 | Me(S) | single bond | H | CH₂CF₃ | Me | Cl | Et | O |
| C-1044 | H | CH₂ | Me | CH₂CF₃ | Me | Cl | Et | O |
| C-1045 | Me | CH₂ | H | CH₂CF₃ | Me | Cl | Et | O |
| C-1046 | H | CH(Me) | H | CH₂CF₃ | Me | Cl | Et | O |
| C-1047 | H | CH₂CH₂ | Me | CH₂CF₃ | Me | Cl | Et | O |
| C-1048 | Me | CH₂CH₂ | H | CH₂CF₃ | Me | Cl | Et | O |
| C-1049 | H | single bond | Me | CH₂CF₃ | Me | Me | Et | O |
| C-1050 | Me | single bond | H | CH₂CF₃ | Me | Me | Et | O |

**[Table 251]**

| Compound Number | Y | W | Z | R¹ | R² | R³ | R¹⁰ | A |
|---|---|---|---|---|---|---|---|---|
| C-1051 | Me(R) | single bond | H | CH₂CF₃ | Me | Me | Et | O |
| C-1052 | Me(S) | single bond | H | CH₂CF₃ | Me | Me | Et | O |
| C-1053 | H | CH₂ | Me | CH₂CF₃ | Me | Me | Et | O |
| C-1054 | Me | CH₂ | H | CH₂CF₃ | Me | Me | Et | O |
| C-1055 | H | CH(Me) | H | CH₂CF₃ | Me | Me | Et | O |
| C-1056 | H | CH₂CH₂ | Me | CH₂CF₃ | Me | Me | Et | O |
| C-1057 | Me | CH₂CH₂ | H | CH₂CF₃ | Me | Me | Et | O |
| C-1058 | H | single bond | Me | CH₂CF₃ | Me | Cl | CH₂CF₃ | O |
| C-1059 | Me | single bond | H | CH₂CF₃ | Me | Cl | CH₂CF₃ | O |
| C-1060 | Me(R) | single bond | H | CH₂CF₃ | Me | Cl | CH₂CF₃ | O |
| C-1061 | Me(S) | single bond | H | CH₂CF₃ | Me | Cl | CH₂CF₃ | O |
| C-1062 | H | CH₂ | Me | CH₂CF₃ | Me | Cl | CH₂CF₃ | O |
| C-1063 | Me | CH₂ | H | CH₂CF₃ | Me | Cl | CH₂CF₃ | O |
| C-1064 | H | CH(Me) | H | CH₂CF₃ | Me | Cl | CH₂CF₃ | O |
| C-1065 | H | CH₂CH₂ | Me | CH₂CF₃ | Me | Cl | CH₂CF₃ | O |
| C-1066 | Me | CH₂CH₂ | H | CH₂CF₃ | Me | Cl | CH₂CF₃ | O |
| C-1067 | H | single bond | Me | CH₂CF₃ | Me | Me | CH₂CF₃ | O |
| C-1068 | Me | single bond | H | CH₂CF₃ | Me | Me | CH₂CF₃ | O |
| C-1069 | Me(R) | single bond | H | CH₂CF₃ | Me | Me | CH₂CF₃ | O |
| C-1070 | Me(S) | single bond | H | CH₂CF₃ | Me | Me | CH₂CF₃ | O |
| C-1071 | H | CH₂ | Me | CH₂CF₃ | Me | Me | CH₂CF₃ | O |
| C-1072 | Me | CH₂ | H | CH₂CF₃ | Me | Me | CH₂CF₃ | O |
| C-1073 | H | CH(Me) | H | CH₂CF₃ | Me | Me | CH₂CF₃ | O |
| C-1074 | H | CH₂CH₂ | Me | CH₂CF₃ | Me | Me | CH₂CF₃ | O |
| C-1075 | Me | CH₂CH₂ | H | CH₂CF₃ | Me | Me | CH₂CF₃ | O |
| C-1076 | H | single bond | Me | CH₂CF₃ | Me | Cl | CH₂OMe | O |
| C-1077 | Me | single bond | H | CH₂CF₃ | Me | Cl | CH₂OMe | O |
| C-1078 | Me(R) | single bond | H | CH₂CF₃ | Me | Cl | CH₂OMe | O |
| C-1079 | Me(S) | single bond | H | CH₂CF₃ | Me | Cl | CH₂OMe | O |
| C-1080 | H | CH₂ | Me | CH₂CF₃ | Me | Cl | CH₂OMe | O |
| C-1081 | Me | CH₂ | H | CH₂CF₃ | Me | Cl | CH₂OMe | O |
| C-1082 | H | CH(Me) | H | CH₂CF₃ | Me | Cl | CH₂OMe | O |
| C-1083 | H | CH₂CH₂ | Me | CH₂CF₃ | Me | Cl | CH₂OMe | O |
| C-1084 | Me | CH₂CH₂ | H | CH₂CF₃ | Me | Cl | CH₂OMe | O |
| C-1085 | H | single bond | Me | CH₂CF₃ | Me | Me | CH₂OMe | O |
| C-1086 | Me | single bond | H | CH₂CF₃ | Me | Me | CH₂OMe | O |
| C-1087 | Me(R) | single bond | H | CH₂CF₃ | Me | Me | CH₂OMe | O |
| C-1088 | Me(S) | single bond | H | CH₂CF₃ | Me | Me | CH₂OMe | O |
| C-1089 | H | CH₂ | Me | CH₂CF₃ | Me | Me | CH₂OMe | O |
| C-1090 | Me | CH₂ | H | CH₂CF₃ | Me | Me | CH₂OMe | O |
| C-1091 | H | CH(Me) | H | CH₂CF₃ | Me | Me | CH₂OMe | O |
| C-1092 | H | CH₂CH₂ | Me | CH₂CF₃ | Me | Me | CH₂OMe | O |
| C-1093 | Me | CH₂CH₂ | H | CH₂CF₃ | Me | Me | CH₂OMe | O |
| C-1094 | H | single bond | Me | CH₂CF₃ | Me | Cl | CH₂CN | O |

**[Table 252]**

| Compound Number | Y | W | Z | R¹ | R² | R³ | R¹⁰ | A |
|---|---|---|---|---|---|---|---|---|
| C-1095 | Me | single bond | H | CH₂CF₃ | Me | Cl | CH₂CN | O |
| C-1096 | Me(R) | single bond | H | CH₂CF₃ | Me | Cl | CH₂CN | O |
| C-1097 | Me(S) | single bond | H | CH₂CF₃ | Me | Cl | CH₂CN | O |
| C-1098 | H | CH₂ | Me | CH₂CF₃ | Me | Cl | CH₂CN | O |
| C-1099 | Me | CH₂ | H | CH₂CF₃ | Me | Cl | CH₂CN | O |
| C-1100 | H | CH(Me) | H | CH₂CF₃ | Me | Cl | CH₂CN | O |
| C-1101 | H | CH₂CH₂ | Me | CH₂CF₃ | Me | Cl | CH₂CN | O |
| C-1102 | Me | CH₂CH₂ | H | CH₂CF₃ | Me | Cl | CH₂CN | O |
| C-1103 | H | single bond | Me | CH₂CF₃ | Me | Me | CH₂CN | O |
| C-1104 | Me | single bond | H | CH₂CF₃ | Me | Me | CH₂CN | O |
| C-1105 | Me(R) | single bond | H | CH₂CF₃ | Me | Me | CH₂CN | O |
| C-1106 | Me(S) | single bond | H | CH₂CF₃ | Me | Me | CH₂CN | O |
| C-1107 | H | CH₂ | Me | CH₂CF₃ | Me | Me | CH₂CN | O |
| C-1108 | Me | CH₂ | H | CH₂CF₃ | Me | Me | CH₂CN | O |
| C-1109 | H | CH(Me) | H | CH₂CF₃ | Me | Me | CH₂CN | O |
| C-1110 | H | CH₂CH₂ | Me | CH₂CF₃ | Me | Me | CH₂CN | O |
| C-1111 | Me | CH₂CH₂ | H | CH₂CF₃ | Me | Me | CH₂CN | O |
| C-1112 | H | single bond | Me | CH₂CF₃ | Me | Cl | C(=O)Me | O |
| C-1113 | Me | single bond | H | CH₂CF₃ | Me | Cl | C(=O)Me | O |
| C-1114 | Me(R) | single bond | H | CH₂CF₃ | Me | Cl | C(=O)Me | O |
| C-1115 | Me(S) | single bond | H | CH₂CF₃ | Me | Cl | C(=O)Me | O |
| C-1116 | H | CH₂ | Me | CH₂CF₃ | Me | Cl | C(=O)Me | O |
| C-1117 | Me | CH₂ | H | CH₂CF₃ | Me | Cl | C(=O)Me | O |
| C-1118 | H | CH(Me) | H | CH₂CF₃ | Me | Cl | C(=O)Me | O |
| C-1119 | H | CH₂CH₂ | Me | CH₂CF₃ | Me | Cl | C(=O)Me | O |
| C-1120 | Me | CH₂CH₂ | H | CH₂CF₃ | Me | Cl | C(=O)Me | O |
| C-1121 | H | single bond | Me | CH₂CF₃ | Me | Me | C(=O)Me | O |
| C-1122 | Me | single bond | H | CH₂CF₃ | Me | Me | C(=O)Me | O |
| C-1123 | Me(R) | single bond | H | CH₂CF₃ | Me | Me | C(=O)Me | O |
| C-1124 | Me(S) | single bond | H | CH₂CF₃ | Me | Me | C(=O)Me | O |
| C-1125 | H | CH₂ | Me | CH₂CF₃ | Me | Me | C(=O)Me | O |
| C-1126 | Me | CH₂ | H | CH₂CF₃ | Me | Me | C(=O)Me | O |
| C-1127 | H | CH(Me) | H | CH₂CF₃ | Me | Me | C(=O)Me | O |
| C-1128 | H | CH₂CH₂ | Me | CH₂CF₃ | Me | Me | C(=O)Me | O |
| C-1129 | Me | CH₂CH₂ | H | CH₂CF₃ | Me | Me | C(=O)Me | O |
| C-1130 | H | single bond | Me | CH₂CF₃ | CH₂OMe | Cl | Me | O |
| C-1131 | H | single bond | Et | CH₂CF₃ | CH₂OMe | Cl | Me | O |
| C-1132 | Me | single bond | H | CH₂CF₃ | CH₂OMe | Cl | Me | O |
| C-1133 | Me(R) | single bond | H | CH₂CF₃ | CH₂OMe | Cl | Me | O |
| C-1134 | Me(S) | single bond | H | CH₂CF₃ | CH₂OMe | Cl | Me | O |
| C-1135 | H | CH₂ | Me | CH₂CF₃ | CH₂OMe | Cl | Me | O |
| C-1136 | Me | CH₂ | H | CH₂CF₃ | CH₂OMe | Cl | Me | O |
| C-1137 | H | CH(Me) | H | CH₂CF₃ | CH₂OMe | Cl | Me | O |
| C-1138 | H | CH₂CH₂ | Me | CH₂CF₃ | CH₂OMe | Cl | Me | O |

**[Table 253]**

| Compound Number | Y | W | Z | R¹ | R² | R³ | R¹⁰ | A |
|---|---|---|---|---|---|---|---|---|
| C-1139 | Me | CH₂CH₂ | H | CH₂CF₃ | CH₂OMe | Cl | Me | O |
| C-1140 | H | single bond | Me | CH₂CF₃ | CH₂OMe | Me | Me | O |
| C-1141 | Me | single bond | H | CH₂CF₃ | CH₂OMe | Me | Me | O |
| C-1142 | Me(R) | single bond | H | CH₂CF₃ | CH₂OMe | Me | Me | O |
| C-1143 | Me(S) | single bond | H | CH₂CF₃ | CH₂OMe | Me | Me | O |
| C-1144 | H | CH₂ | Me | CH₂CF₃ | CH₂OMe | Me | Me | O |
| C-1145 | Me | CH₂ | H | CH₂CF₃ | CH₂OMe | Me | Me | O |
| C-1146 | H | CH(Me) | H | CH₂CF₃ | CH₂OMe | Me | Me | O |
| C-1147 | H | CH₂CH₂ | Me | CH₂CF₃ | CH₂OMe | Me | Me | O |
| C-1148 | Me | CH₂CH₂ | H | CH₂CF₃ | CH₂OMe | Me | Me | O |
| C-1149 | H | single bond | Me | CH₂CF₃ | CH₂OMe | Cl | Et | O |
| C-1150 | Me | single bond | H | CH₂CF₃ | CH₂OMe | Cl | Et | O |
| C-1151 | Me(R) | single bond | H | CH₂CF₃ | CH₂OMe | Cl | Et | O |
| C-1152 | Me(S) | single bond | H | CH₂CF₃ | CH₂OMe | Cl | Et | O |
| C-1153 | H | CH₂ | Me | CH₂CF₃ | CH₂OMe | Cl | Et | O |
| C-1154 | Me | CH₂ | H | CH₂CF₃ | CH₂OMe | Cl | Et | O |
| C-1155 | H | CH(Me) | H | CH₂CF₃ | CH₂OMe | Cl | Et | O |
| C-1156 | H | CH₂CH₂ | Me | CH₂CF₃ | CH₂OMe | Cl | Et | O |
| C-1157 | Me | CH₂CH₂ | H | CH₂CF₃ | CH₂OMe | Cl | Et | O |
| C-1158 | H | single bond | Me | CH₂CF₃ | CH₂OMe | Me | Et | O |
| C-1159 | Me | single bond | H | CH₂CF₃ | CH₂OMe | Me | Et | O |
| C-1160 | Me(R) | single bond | H | CH₂CF₃ | CH₂OMe | Me | Et | O |
| C-1161 | Me(S) | single bond | H | CH₂CF₃ | CH₂OMe | Me | Et | O |
| C-1162 | H | CH₂ | Me | CH₂CF₃ | CH₂OMe | Me | Et | O |
| C-1163 | Me | CH₂ | H | CH₂CF₃ | CH₂OMe | Me | Et | O |
| C-1164 | H | CH(Me) | H | CH₂CF₃ | CH₂OMe | Me | Et | O |
| C-1165 | H | CH₂CH₂ | Me | CH₂CF₃ | CH₂OMe | Me | Et | O |
| C-1166 | Me | CH₂CH₂ | H | CH₂CF₃ | CH₂OMe | Me | Et | O |
| C-1167 | H | single bond | Me | CH₂CF₃ | CH₂OMe | Cl | CH₂CF₃ | O |
| C-1168 | Me | single bond | H | CH₂CF₃ | CH₂OMe | Cl | CH₂CF₃ | O |
| C-1169 | Me(R) | single bond | H | CH₂CF₃ | CH₂OMe | Cl | CH₂CF₃ | O |
| C-1170 | Me(S) | single bond | H | CH₂CF₃ | CH₂OMe | Cl | CH₂CF₃ | O |
| C-1171 | H | CH₂ | Me | CH₂CF₃ | CH₂OMe | Cl | CH₂CF₃ | O |
| C-1172 | Me | CH₂ | H | CH₂CF₃ | CH₂OMe | Cl | CH₂CF₃ | O |
| C-1173 | H | CH(Me) | H | CH₂CF₃ | CH₂OMe | Cl | CH₂CF₃ | O |
| C-1174 | H | CH₂CH₂ | Me | CH₂CF₃ | CH₂OMe | Cl | CH₂CF₃ | O |
| C-1175 | Me | CH₂CH₂ | H | CH₂CF₃ | CH₂OMe | Cl | CH₂CF₃ | O |
| C-1176 | H | single bond | Me | CH₂CF₃ | CH₂OMe | Me | CH₂CF₃ | O |
| C-1177 | Me | single bond | H | CH₂CF₃ | CH₂OMe | Me | CH₂CF₃ | O |
| C-1178 | Me(R) | single bond | H | CH₂CF₃ | CH₂OMe | Me | CH₂CF₃ | O |
| C-1179 | Me(S) | single bond | H | CH₂CF₃ | CH₂OMe | Me | CH₂CF₃ | O |
| C-1180 | H | CH₂ | Me | CH₂CF₃ | CH₂OMe | Me | CH₂CF₃ | O |
| C-1181 | Me | CH₂ | H | CH₂CF₃ | CH₂OMe | Me | CH₂CF₃ | O |
| C-1182 | H | CH(Me) | H | CH₂CF₃ | CH₂OMe | Me | CH₂CF₃ | O |

**[Table 254]**

| Compound Number | Y | W | Z | R¹ | R² | R³ | R¹⁰ | A |
|---|---|---|---|---|---|---|---|---|
| C-1183 | H | CH₂CH₂ | Me | CH₂CF₃ | CH₂OMe | Me | CH₂CF₃ | O |
| C-1184 | Me | CH₂CH₂ | H | CH₂CF₃ | CH₂OMe | Me | CH₂CF₃ | O |
| C-1185 | H | single bond | Me | CH₂CF₃ | CH₂OMe | Cl | CH₂OMe | O |
| C-1186 | Me | single bond | H | CH₂CF₃ | CH₂OMe | Cl | CH₂OMe | O |
| C-1187 | Me(R) | single bond | H | CH₂CF₃ | CH₂OMe | Cl | CH₂OMe | O |
| C-1188 | Me(S) | single bond | H | CH₂CF₃ | CH₂OMe | Cl | CH₂OMe | O |
| C-1189 | H | CH₂ | Me | CH₂CF₃ | CH₂OMe | Cl | CH₂OMe | O |
| C-1190 | Me | CH₂ | H | CH₂CF₃ | CH₂OMe | Cl | CH₂OMe | O |
| C-1191 | H | CH(Me) | H | CH₂CF₃ | CH₂OMe | Cl | CH₂OMe | O |
| C-1192 | H | CH₂CH₂ | Me | CH₂CF₃ | CH₂OMe | Cl | CH₂OMe | O |
| C-1193 | Me | CH₂CH₂ | H | CH₂CF₃ | CH₂OMe | Cl | CH₂OMe | O |
| C-1194 | H | single bond | Me | CH₂CF₃ | CH₂OMe | Me | CH₂OMe | O |
| C-1195 | Me | single bond | H | CH₂CF₃ | CH₂OMe | Me | CH₂OMe | O |
| C-1196 | Me(R) | single bond | H | CH₂CF₃ | CH₂OMe | Me | CH₂OMe | O |
| C-1197 | Me(S) | single bond | H | CH₂CF₃ | CH₂OMe | Me | CH₂OMe | O |
| C-1198 | H | CH₂ | Me | CH₂CF₃ | CH₂OMe | Me | CH₂OMe | O |
| C-1199 | Me | CH₂ | H | CH₂CF₃ | CH₂OMe | Me | CH₂OMe | O |
| C-1200 | H | CH(Me) | H | CH₂CF₃ | CH₂OMe | Me | CH₂OMe | O |
| C-1201 | H | CH₂CH₂ | Me | CH₂CF₃ | CH₂OMe | Me | CH₂OMe | O |
| C-1202 | Me | CH₂CH₂ | H | CH₂CF₃ | CH₂OMe | Me | CH₂OMe | O |
| C-1203 | H | single bond | Me | CH₂CF₃ | CH₂OMe | Cl | CH₂CN | O |
| C-1204 | Me | single bond | H | CH₂CF₃ | CH₂OMe | Cl | CH₂CN | O |
| C-1205 | Me(R) | single bond | H | CH₂CF₃ | CH₂OMe | Cl | CH₂CN | O |
| C-1206 | Me(S) | single bond | H | CH₂CF₃ | CH₂OMe | Cl | CH₂CN | O |
| C-1207 | H | CH₂ | Me | CH₂CF₃ | CH₂OMe | Cl | CH₂CN | O |
| C-1208 | Me | CH₂ | H | CH₂CF₃ | CH₂OMe | Cl | CH₂CN | O |
| C-1209 | H | CH(Me) | H | CH₂CF₃ | CH₂OMe | Cl | CH₂CN | O |
| C-1210 | H | CH₂CH₂ | Me | CH₂CF₃ | CH₂OMe | Cl | CH₂CN | O |
| C-1211 | Me | CH₂CH₂ | H | CH₂CF₃ | CH₂OMe | Cl | CH₂CN | O |
| C-1212 | H | single bond | Me | CH₂CF₃ | CH₂OMe | Me | CH₂CN | O |
| C-1213 | Me | single bond | H | CH₂CF₃ | CH₂OMe | Me | CH₂CN | O |
| C-1214 | Me(R) | single bond | H | CH₂CF₃ | CH₂OMe | Me | CH₂CN | O |
| C-1215 | Me(S) | single bond | H | CH₂CF₃ | CH₂OMe | Me | CH₂CN | O |
| C-1216 | H | CH₂ | Me | CH₂CF₃ | CH₂OMe | Me | CH₂CN | O |
| C-1217 | Me | CH₂ | H | CH₂CF₃ | CH₂OMe | Me | CH₂CN | O |
| C-1218 | H | CH(Me) | H | CH₂CF₃ | CH₂OMe | Me | CH₂CN | O |
| C-1219 | H | CH₂CH₂ | Me | CH₂CF₃ | CH₂OMe | Me | CH₂CN | O |
| C-1220 | Me | CH₂CH₂ | H | CH₂CF₃ | CH₂OMe | Me | CH₂CN | O |
| C-1221 | H | single bond | Me | CH₂CF₃ | CH₂OMe | Cl | C(=O)Me | O |
| C-1222 | Me | single bond | H | CH₂CF₃ | CH₂OMe | Cl | C(=O)Me | O |
| C-1223 | Me(R) | single bond | H | CH₂CF₃ | CH₂OMe | Cl | C(=O)Me | O |
| C-1224 | Me(S) | single bond | H | CH₂CF₃ | CH₂OMe | Cl | C(=O)Me | O |
| C-1225 | H | CH₂ | Me | CH₂CF₃ | CH₂OMe | Cl | C(=O)Me | O |
| C-1226 | Me | CH₂ | H | CH₂CF₃ | CH₂OMe | Cl | C(=O)Me | O |

**[Table 255]**

| Compound Number | Y | W | Z | R¹ | R² | R³ | R¹⁰ | A |
|---|---|---|---|---|---|---|---|---|
| C-1227 | H | CH(Me) | H | CH₂CF₃ | CH₂OMe | Cl | C(=O)Me | O |
| C-1228 | H | CH₂CH₂ | Me | CH₂CF₃ | CH₂OMe | Cl | C(=O)Me | O |
| C-1229 | Me | CH₂CH₂ | H | CH₂CF₃ | CH₂OMe | Cl | C(=O)Me | O |
| C-1230 | H | single bond | Me | CH₂CF₃ | CH₂OMe | Me | C(=O)Me | O |
| C-1231 | Me | single bond | H | CH₂CF₃ | CH₂OMe | Me | C(=O)Me | O |
| C-1232 | Me(R) | single bond | H | CH₂CF₃ | CH₂OMe | Me | C(=O)Me | O |
| C-1233 | Me(S) | single bond | H | CH₂CF₃ | CH₂OMe | Me | C(=O)Me | O |
| C-1234 | H | CH₂ | Me | CH₂CF₃ | CH₂OMe | Me | C(=O)Me | O |
| C-1235 | Me | CH₂ | H | CH₂CF₃ | CH₂OMe | Me | C(=O)Me | O |
| C-1236 | H | CH(Me) | H | CH₂CF₃ | CH₂OMe | Me | C(=O)Me | O |
| C-1237 | H | CH₂CH₂ | Me | CH₂CF₃ | CH₂OMe | Me | C(=O)Me | O |
| C-1238 | Me | CH₂CH₂ | H | CH₂CF₃ | CH₂OMe | Me | C(=O)Me | O |
| C-1239 | H | single bond | Me | CH₂CF₃ | CH₂CN | Cl | Me | O |
| C-1240 | H | single bond | Et | CH₂CF₃ | CH₂CN | Cl | Me | O |
| C-1241 | Me | single bond | H | CH₂CF₃ | CH₂CN | Cl | Me | O |
| C-1242 | Me(R) | single bond | H | CH₂CF₃ | CH₂CN | Cl | Me | O |
| C-1243 | Me(S) | single bond | H | CH₂CF₃ | CH₂CN | Cl | Me | O |
| C-1244 | H | CH₂ | Me | CH₂CF₃ | CH₂CN | Cl | Me | O |
| C-1245 | Me | CH₂ | H | CH₂CF₃ | CH₂CN | Cl | Me | O |
| C-1246 | H | CH(Me) | H | CH₂CF₃ | CH₂CN | Cl | Me | O |
| C-1247 | H | CH₂CH₂ | Me | CH₂CF₃ | CH₂CN | Cl | Me | O |
| C-1248 | Me | CH₂CH₂ | H | CH₂CF₃ | CH₂CN | Cl | Me | O |
| C-1249 | H | single bond | Me | CH₂CF₃ | CH₂CN | Me | Me | O |
| C-1250 | Me | single bond | H | CH₂CF₃ | CH₂CN | Me | Me | O |
| C-1251 | Me(R) | single bond | H | CH₂CF₃ | CH₂CN | Me | Me | O |
| C-1252 | Me(S) | single bond | H | CH₂CF₃ | CH₂CN | Me | Me | O |
| C-1253 | H | CH₂ | Me | CH₂CF₃ | CH₂CN | Me | Me | O |
| C-1254 | Me | CH₂ | H | CH₂CF₃ | CH₂CN | Me | Me | O |
| C-1255 | H | CH(Me) | H | CH₂CF₃ | CH₂CN | Me | Me | O |
| C-1256 | H | CH₂CH₂ | Me | CH₂CF₃ | CH₂CN | Me | Me | O |
| C-1257 | Me | CH₂CH₂ | H | CH₂CF₃ | CH₂CN | Me | Me | O |
| C-1258 | H | single bond | Me | CH₂CF₃ | CH₂CN | Cl | Et | O |
| C-1259 | Me | single bond | H | CH₂CF₃ | CH₂CN | Cl | Et | O |
| C-1260 | Me(R) | single bond | H | CH₂CF₃ | CH₂CN | Cl | Et | O |
| C-1261 | Me(S) | single bond | H | CH₂CF₃ | CH₂CN | Cl | Et | O |
| C-1262 | H | CH₂ | Me | CH₂CF₃ | CH₂CN | Cl | Et | O |
| C-1263 | Me | CH₂ | H | CH₂CF₃ | CH₂CN | Cl | Et | O |
| C-1264 | H | CH(Me) | H | CH₂CF₃ | CH₂CN | Cl | Et | O |
| C-1265 | H | CH₂CH₂ | Me | CH₂CF₃ | CH₂CN | Cl | Et | O |
| C-1266 | Me | CH₂CH₂ | H | CH₂CF₃ | CH₂CN | Cl | Et | O |
| C-1267 | H | single bond | Me | CH₂CF₃ | CH₂CN | Me | Et | O |
| C-1268 | Me | single bond | H | CH₂CF₃ | CH₂CN | Me | Et | O |
| C-1269 | Me(R) | single bond | H | CH₂CF₃ | CH₂CN | Me | Et | O |
| C-1270 | Me(S) | single bond | H | CH₂CF₃ | CH₂CN | Me | Et | O |

**[Table 256]**

| Compound Number | Y | W | Z | R¹ | R² | R³ | R¹⁰ | A |
|---|---|---|---|---|---|---|---|---|
| C-1271 | H | CH₂ | Me | CH₂CF₃ | CH₂CN | Me | Et | O |
| C-1272 | Me | CH₂ | H | CH₂CF₃ | CH₂CN | Me | Et | O |
| C-1273 | H | CH(Me) | H | CH₂CF₃ | CH₂CN | Me | Et | O |
| C-1274 | H | CH₂CH₂ | Me | CH₂CF₃ | CH₂CN | Me | Et | O |
| C-1275 | Me | CH₂CH₂ | H | CH₂CF₃ | CH₂CN | Me | Et | O |
| C-1276 | H | single bond | Me | CH₂CF₃ | CH₂CN | Cl | CH₂CF₃ | O |
| C-1277 | Me | single bond | H | CH₂CF₃ | CH₂CN | Cl | CH₂CF₃ | O |
| C-1278 | Me(R) | single bond | H | CH₂CF₃ | CH₂CN | Cl | CH₂CF₃ | O |
| C-1279 | Me(S) | single bond | H | CH₂CF₃ | CH₂CN | Cl | CH₂CF₃ | O |
| C-1280 | H | CH₂ | Me | CH₂CF₃ | CH₂CN | Cl | CH₂CF₃ | O |
| C-1281 | Me | CH₂ | H | CH₂CF₃ | CH₂CN | Cl | CH₂CF₃ | O |
| C-1282 | H | CH(Me) | H | CH₂CF₃ | CH₂CN | Cl | CH₂CF₃ | O |
| C-1283 | H | CH₂CH₂ | Me | CH₂CF₃ | CH₂CN | Cl | CH₂CF₃ | O |
| C-1284 | Me | CH₂CH₂ | H | CH₂CF₃ | CH₂CN | Cl | CH₂CF₃ | O |
| C-1285 | H | single bond | Me | CH₂CF₃ | CH₂CN | Me | CH₂CF₃ | O |
| C-1286 | Me | single bond | H | CH₂CF₃ | CH₂CN | Me | CH₂CF₃ | O |
| C-1287 | Me(R) | single bond | H | CH₂CF₃ | CH₂CN | Me | CH₂CF₃ | O |
| C-1288 | Me(S) | single bond | H | CH₂CF₃ | CH₂CN | Me | CH₂CF₃ | O |
| C-1289 | H | CH₂ | Me | CH₂CF₃ | CH₂CN | Me | CH₂CF₃ | O |
| C-1290 | Me | CH₂ | H | CH₂CF₃ | CH₂CN | Me | CH₂CF₃ | O |
| C-1291 | H | CH(Me) | H | CH₂CF₃ | CH₂CN | Me | CH₂CF₃ | O |
| C-1292 | H | CH₂CH₂ | Me | CH₂CF₃ | CH₂CN | Me | CH₂CF₃ | O |
| C-1293 | Me | CH₂CH₂ | H | CH₂CF₃ | CH₂CN | Me | CH₂CF₃ | O |
| C-1294 | H | single bond | Me | CH₂CF₃ | CH₂CN | Cl | CH₂OMe | O |
| C-1295 | Me | single bond | H | CH₂CF₃ | CH₂CN | Cl | CH₂OMe | O |
| C-1296 | Me(R) | single bond | H | CH₂CF₃ | CH₂CN | Cl | CH₂OMe | O |
| C-1297 | Me(S) | single bond | H | CH₂CF₃ | CH₂CN | Cl | CH₂OMe | O |
| C-1298 | H | CH₂ | Me | CH₂CF₃ | CH₂CN | Cl | CH₂OMe | O |
| C-1299 | Me | CH₂ | H | CH₂CF₃ | CH₂CN | Cl | CH₂OMe | O |
| C-1300 | H | CH(Me) | H | CH₂CF₃ | CH₂CN | Cl | CH₂OMe | O |
| C-1301 | H | CH₂CH₂ | Me | CH₂CF₃ | CH₂CN | Cl | CH₂OMe | O |
| C-1302 | Me | CH₂CH₂ | H | CH₂CF₃ | CH₂CN | Cl | CH₂OMe | O |
| C-1303 | H | single bond | Me | CH₂CF₃ | CH₂CN | Me | CH₂OMe | O |
| C-1304 | Me | single bond | H | CH₂CF₃ | CH₂CN | Me | CH₂OMe | O |
| C-1305 | Me(R) | single bond | H | CH₂CF₃ | CH₂CN | Me | CH₂OMe | O |
| C-1306 | Me(S) | single bond | H | CH₂CF₃ | CH₂CN | Me | CH₂OMe | O |
| C-1307 | H | CH₂ | Me | CH₂CF₃ | CH₂CN | Me | CH₂OMe | O |
| C-1308 | Me | CH₂ | H | CH₂CF₃ | CH₂CN | Me | CH₂OMe | O |
| C-1309 | H | CH(Me) | H | CH₂CF₃ | CH₂CN | Me | CH₂OMe | O |
| C-1310 | H | CH₂CH₂ | Me | CH₂CF₃ | CH₂CN | Me | CH₂OMe | O |
| C-1311 | Me | CH₂CH₂ | H | CH₂CF₃ | CH₂CN | Me | CH₂OMe | O |
| C-1312 | H | single bond | Me | CH₂CF₃ | CH₂CN | Cl | CH₂CN | O |
| C-1313 | Me | single bond | H | CH₂CF₃ | CH₂CN | Cl | CH₂CN | O |
| C-1314 | Me(R) | single bond | H | CH₂CF₃ | CH₂CN | Cl | CH₂CN | O |

**[Table 257]**

| Compound Number | Y | W | Z | R¹ | R² | R³ | R¹⁰ | A |
|---|---|---|---|---|---|---|---|---|
| C-1315 | Me(S) | single bond | H | CH₂CF₃ | CH₂CN | Cl | CH₂CN | O |
| C-1316 | H | CH₂ | Me | CH₂CF₃ | CH₂CN | Cl | CH₂CN | O |
| C-1317 | Me | CH₂ | H | CH₂CF₃ | CH₂CN | Cl | CH₂CN | O |
| C-1318 | H | CH(Me) | H | CH₂CF₃ | CH₂CN | Cl | CH₂CN | O |
| C-1319 | H | CH₂CH₂ | Me | CH₂CF₃ | CH₂CN | Cl | CH₂CN | O |
| C-1320 | Me | CH₂CH₂ | H | CH₂CF₃ | CH₂CN | Cl | CH₂CN | O |
| C-1321 | H | single bond | Me | CH₂CF₃ | CH₂CN | Me | CH₂CN | O |
| C-1322 | Me | single bond | H | CH₂CF₃ | CH₂CN | Me | CH₂CN | O |
| C-1323 | Me(R) | single bond | H | CH₂CF₃ | CH₂CN | Me | CH₂CN | O |
| C-1324 | Me(S) | single bond | H | CH₂CF₃ | CH₂CN | Me | CH₂CN | O |
| C-1325 | H | CH₂ | Me | CH₂CF₃ | CH₂CN | Me | CH₂CN | O |
| C-1326 | Me | CH₂ | H | CH₂CF₃ | CH₂CN | Me | CH₂CN | O |
| C-1327 | H | CH(Me) | H | CH₂CF₃ | CH₂CN | Me | CH₂CN | O |
| C-1328 | H | CH₂CH₂ | Me | CH₂CF₃ | CH₂CN | Me | CH₂CN | O |
| C-1329 | Me | CH₂CH₂ | H | CH₂CF₃ | CH₂CN | Me | CH₂CN | O |
| C-1330 | H | single bond | Me | CH₂CF₃ | CH₂CN | Cl | C(=O)Me | O |
| C-1331 | Me | single bond | H | CH₂CF₃ | CH₂CN | Cl | C(=O)Me | O |
| C-1332 | Me(R) | single bond | H | CH₂CF₃ | CH₂CN | Cl | C(=O)Me | O |
| C-1333 | Me(S) | single bond | H | CH₂CF₃ | CH₂CN | Cl | C(=O)Me | O |
| C-1334 | H | CH₂ | Me | CH₂CF₃ | CH₂CN | Cl | C(=O)Me | O |
| C-1335 | Me | CH₂ | H | CH₂CF₃ | CH₂CN | Cl | C(=O)Me | O |
| C-1336 | H | CH(Me) | H | CH₂CF₃ | CH₂CN | Cl | C(=O)Me | O |
| C-1337 | H | CH₂CH₂ | Me | CH₂CF₃ | CH₂CN | Cl | C(=O)Me | O |
| C-1338 | Me | CH₂CH₂ | H | CH₂CF₃ | CH₂CN | Cl | C(=O)Me | O |
| C-1339 | H | single bond | Me | CH₂CF₃ | CH₂CN | Me | C(=O)Me | O |
| C-1340 | Me | single bond | H | CH₂CF₃ | CH₂CN | Me | C(=O)Me | O |
| C-1341 | Me(R) | single bond | H | CH₂CF₃ | CH₂CN | Me | C(=O)Me | O |
| C-1342 | Me(S) | single bond | H | CH₂CF₃ | CH₂CN | Me | C(=O)Me | O |
| C-1343 | H | CH₂ | Me | CH₂CF₃ | CH₂CN | Me | C(=O)Me | O |
| C-1344 | Me | CH₂ | H | CH₂CF₃ | CH₂CN | Me | C(=O)Me | O |
| C-1345 | H | CH(Me) | H | CH₂CF₃ | CH₂CN | Me | C(=O)Me | O |
| C-1346 | H | CH₂CH₂ | Me | CH₂CF₃ | CH₂CN | Me | C(=O)Me | O |
| C-1347 | Me | CH₂CH₂ | H | CH₂CF₃ | CH₂CN | Me | C(=O)Me | O |
| C-1348 | H | single bond | Me | CH₂CF₃ | C(=O)Me | Cl | Me | O |
| C-1349 | Me | single bond | H | CH₂CF₃ | C(=O)Me | Cl | Me | O |
| C-1350 | Me(R) | single bond | H | CH₂CF₃ | C(=O)Me | Cl | Me | O |
| C-1351 | Me(S) | single bond | H | CH₂CF₃ | C(=O)Me | Cl | Me | O |
| C-1352 | H | CH₂ | Me | CH₂CF₃ | C(=O)Me | Cl | Me | O |
| C-1353 | Me | CH₂ | H | CH₂CF₃ | C(=O)Me | Cl | Me | O |
| C-1354 | H | CH(Me) | H | CH₂CF₃ | C(=O)Me | Cl | Me | O |
| C-1355 | H | CH₂CH₂ | Me | CH₂CF₃ | C(=O)Me | Cl | Me | O |
| C-1356 | Me | CH₂CH₂ | H | CH₂CF₃ | C(=O)Me | Cl | Me | O |
| C-1357 | H | single bond | Me | CH₂CF₃ | C(=O)Me | Me | Me | O |
| C-1358 | Me | single bond | H | CH₂CF₃ | C(=O)Me | Me | Me | O |

**[Table 258]**

| Compound Number | Y | W | Z | R¹ | R² | R³ | R¹⁰ | A |
|---|---|---|---|---|---|---|---|---|
| C-1359 | Me(R) | single bond | H | CH₂CF₃ | C(=O)Me | Me | Me | O |
| C-1360 | Me(S) | single bond | H | CH₂CF₃ | C(=O)Me | Me | Me | O |
| C-1361 | H | CH₂ | Me | CH₂CF₃ | C(=O)Me | Me | Me | O |
| C-1362 | Me | CH₂ | H | CH₂CF₃ | C(=O)Me | Me | Me | O |
| C-1363 | H | CH(Me) | H | CH₂CF₃ | C(=O)Me | Me | Me | O |
| C-1364 | H | CH₂CH₂ | Me | CH₂CF₃ | C(=O)Me | Me | Me | O |
| C-1365 | Me | CH₂CH₂ | H | CH₂CF₃ | C(=O)Me | Me | Me | O |
| C-1366 | H | single bond | Me | CH₂CF₃ | C(=O)Me | Cl | Et | O |
| C-1367 | Me | single bond | H | CH₂CF₃ | C(=O)Me | Cl | Et | O |
| C-1368 | Me(R) | single bond | H | CH₂CF₃ | C(=O)Me | Cl | Et | O |
| C-1369 | Me(S) | single bond | H | CH₂CF₃ | C(=O)Me | Cl | Et | O |
| C-1370 | H | CH₂ | Me | CH₂CF₃ | C(=O)Me | Cl | Et | O |
| C-1371 | Me | CH₂ | H | CH₂CF₃ | C(=O)Me | Cl | Et | O |
| C-1372 | H | CH(Me) | H | CH₂CF₃ | C(=O)Me | Cl | Et | O |
| C-1373 | H | CH₂CH₂ | Me | CH₂CF₃ | C(=O)Me | Cl | Et | O |
| C-1374 | Me | CH₂CH₂ | H | CH₂CF₃ | C(=O)Me | Cl | Et | O |
| C-1375 | H | single bond | Me | CH₂CF₃ | C(=O)Me | Me | Et | O |
| C-1376 | Me | single bond | H | CH₂CF₃ | C(=O)Me | Me | Et | O |
| C-1377 | Me(R) | single bond | H | CH₂CF₃ | C(=O)Me | Me | Et | O |
| C-1378 | Me(S) | single bond | H | CH₂CF₃ | C(=O)Me | Me | Et | O |
| C-1379 | H | CH₂ | Me | CH₂CF₃ | C(=O)Me | Me | Et | O |
| C-1380 | Me | CH₂ | H | CH₂CF₃ | C(=O)Me | Me | Et | O |
| C-1381 | H | CH(Me) | H | CH₂CF₃ | C(=O)Me | Me | Et | O |
| C-1382 | H | CH₂CH₂ | Me | CH₂CF₃ | C(=O)Me | Me | Et | O |
| C-1383 | Me | CH₂CH₂ | H | CH₂CF₃ | C(=O)Me | Me | Et | O |
| C-1384 | H | single bond | Me | CH₂CF₃ | C(=O)Me | Cl | CH₂CF₃ | O |
| C-1385 | Me | single bond | H | CH₂CF₃ | C(=O)Me | Cl | CH₂CF₃ | O |
| C-1386 | Me(R) | single bond | H | CH₂CF₃ | C(=O)Me | Cl | CH₂CF₃ | O |
| C-1387 | Me(S) | single bond | H | CH₂CF₃ | C(=O)Me | Cl | CH₂CF₃ | O |
| C-1388 | H | CH₂ | Me | CH₂CF₃ | C(=O)Me | Cl | CH₂CF₃ | O |
| C-1389 | Me | CH₂ | H | CH₂CF₃ | C(=O)Me | Cl | CH₂CF₃ | O |
| C-1390 | H | CH(Me) | H | CH₂CF₃ | C(=O)Me | Cl | CH₂CF₃ | O |
| C-1391 | H | CH₂CH₂ | Me | CH₂CF₃ | C(=O)Me | Cl | CH₂CF₃ | O |
| C-1392 | Me | CH₂CH₂ | H | CH₂CF₃ | C(=O)Me | Cl | CH₂CF₃ | O |
| C-1393 | H | single bond | Me | CH₂CF₃ | C(=O)Me | Me | CH₂CF₃ | O |
| C-1394 | Me | single bond | H | CH₂CF₃ | C(=O)Me | Me | CH₂CF₃ | O |
| C-1395 | Me(R) | single bond | H | CH₂CF₃ | C(=O)Me | Me | CH₂CF₃ | O |
| C-1396 | Me(S) | single bond | H | CH₂CF₃ | C(=O)Me | Me | CH₂CF₃ | O |
| C-1397 | H | CH₂ | Me | CH₂CF₃ | C(=O)Me | Me | CH₂CF₃ | O |
| C-1398 | Me | CH₂ | H | CH₂CF₃ | C(=O)Me | Me | CH₂CF₃ | O |
| C-1399 | H | CH(Me) | H | CH₂CF₃ | C(=O)Me | Me | CH₂CF₃ | O |
| C-1400 | H | CH₂CH₂ | Me | CH₂CF₃ | C(=O)Me | Me | CH₂CF₃ | O |
| C-1401 | Me | CH₂CH₂ | H | CH₂CF₃ | C(=O)Me | Me | CH₂CF₃ | O |
| C-1402 | H | single bond | Me | CH₂CF₃ | C(=O)Me | Cl | CH₂OMe | O |

**[Table 259]**

| Compound Number | Y | W | Z | R¹ | R² | R³ | R¹⁰ | A |
|---|---|---|---|---|---|---|---|---|
| C-1403 | Me | single bond | H | CH₂CF₃ | C(=O)Me | Cl | CH₂OMe | O |
| C-1404 | Me(R) | single bond | H | CH₂CF₃ | C(=O)Me | Cl | CH₂OMe | O |
| C-1405 | Me(S) | single bond | H | CH₂CF₃ | C(=O)Me | Cl | CH₂OMe | O |
| C-1406 | H | CH₂ | Me | CH₂CF₃ | C(=O)Me | Cl | CH₂OMe | O |
| C-1407 | Me | CH₂ | H | CH₂CF₃ | C(=O)Me | Cl | CH₂OMe | O |
| C-1408 | H | CH(Me) | H | CH₂CF₃ | C(=O)Me | Cl | CH₂OMe | O |
| C-1409 | H | CH₂CH₂ | Me | CH₂CF₃ | C(=O)Me | Cl | CH₂OMe | O |
| C-1410 | Me | CH₂CH₂ | H | CH₂CF₃ | C(=O)Me | Cl | CH₂OMe | O |
| C-1411 | H | single bond | Me | CH₂CF₃ | C(=O)Me | Me | CH₂OMe | O |
| C-1412 | Me | single bond | H | CH₂CF₃ | C(=O)Me | Me | CH₂OMe | O |
| C-1413 | Me(R) | single bond | H | CH₂CF₃ | C(=O)Me | Me | CH₂OMe | O |
| C-1414 | Me(S) | single bond | H | CH₂CF₃ | C(=O)Me | Me | CH₂OMe | O |
| C-1415 | H | CH₂ | Me | CH₂CF₃ | C(=O)Me | Me | CH₂OMe | O |
| C-1416 | Me | CH₂ | H | CH₂CF₃ | C(=O)Me | Me | CH₂OMe | O |
| C-1417 | H | CH(Me) | H | CH₂CF₃ | C(=O)Me | Me | CH₂OMe | O |
| C-1418 | H | CH₂CH₂ | Me | CH₂CF₃ | C(=O)Me | Me | CH₂OMe | O |
| C-1419 | Me | CH₂CH₂ | H | CH₂CF₃ | C(=O)Me | Me | CH₂OMe | O |
| C-1420 | H | single bond | Me | CH₂CF₃ | C(=O)Me | Cl | CH₂CN | O |
| C-1421 | Me | single bond | H | CH₂CF₃ | C(=O)Me | Cl | CH₂CN | O |
| C-1422 | Me(R) | single bond | H | CH₂CF₃ | C(=O)Me | Cl | CH₂CN | O |
| C-1423 | Me(S) | single bond | H | CH₂CF₃ | C(=O)Me | Cl | CH₂CN | O |
| C-1424 | H | CH₂ | Me | CH₂CF₃ | C(=O)Me | Cl | CH₂CN | O |
| C-1425 | Me | CH₂ | H | CH₂CF₃ | C(=O)Me | Cl | CH₂CN | O |
| C-1426 | H | CH(Me) | H | CH₂CF₃ | C(=O)Me | Cl | CH₂CN | O |
| C-1427 | H | CH₂CH₂ | Me | CH₂CF₃ | C(=O)Me | Cl | CH₂CN | O |
| C-1428 | Me | CH₂CH₂ | H | CH₂CF₃ | C(=O)Me | Cl | CH₂CN | O |
| C-1429 | H | single bond | Me | CH₂CF₃ | C(=O)Me | Me | CH₂CN | O |
| C-1430 | Me | single bond | H | CH₂CF₃ | C(=O)Me | Me | CH₂CN | O |
| C-1431 | Me(R) | single bond | H | CH₂CF₃ | C(=O)Me | Me | CH₂CN | O |
| C-1432 | Me(S) | single bond | H | CH₂CF₃ | C(=O)Me | Me | CH₂CN | O |
| C-1433 | H | CH₂ | Me | CH₂CF₃ | C(=O)Me | Me | CH₂CN | O |
| C-1434 | Me | CH₂ | H | CH₂CF₃ | C(=O)Me | Me | CH₂CN | O |
| C-1435 | H | CH(Me) | H | CH₂CF₃ | C(=O)Me | Me | CH₂CN | O |
| C-1436 | H | CH₂CH₂ | Me | CH₂CF₃ | C(=O)Me | Me | CH₂CN | O |
| C-1437 | Me | CH₂CH₂ | H | CH₂CF₃ | C(=O)Me | Me | CH₂CN | O |
| C-1438 | H | single bond | Me | CH₂CF₃ | C(=O)Me | Cl | C(=O)Me | O |
| C-1439 | Me | single bond | H | CH₂CF₃ | C(=O)Me | Cl | C(=O)Me | O |
| C-1440 | Me(R) | single bond | H | CH₂CF₃ | C(=O)Me | Cl | C(=O)Me | O |
| C-1441 | Me(S) | single bond | H | CH₂CF₃ | C(=O)Me | Cl | C(=O)Me | O |
| C-1442 | H | CH₂ | Me | CH₂CF₃ | C(=O)Me | Cl | C(=O)Me | O |
| C-1443 | Me | CH₂ | H | CH₂CF₃ | C(=O)Me | Cl | C(=O)Me | O |
| C-1444 | H | CH(Me) | H | CH₂CF₃ | C(=O)Me | Cl | C(=O)Me | O |
| C-1445 | H | CH₂CH₂ | Me | CH₂CF₃ | C(=O)Me | Cl | C(=O)Me | O |
| C-1446 | Me | CH₂CH₂ | H | CH₂CF₃ | C(=O)Me | Cl | C(=O)Me | O |

**[Table 260]**

| Compound Number | Y | W | Z | R¹ | R² | R³ | R¹⁰ | A |
|---|---|---|---|---|---|---|---|---|
| C-1447 | H | single bond | Me | CH₂CF₃ | C(=O)Me | Me | C(=O)Me | O |
| C-1448 | Me | single bond | H | CH₂CF₃ | C(=O)Me | Me | C(=O)Me | O |
| C-1449 | Me(R) | single bond | H | CH₂CF₃ | C(=O)Me | Me | C(=O)Me | O |
| C-1450 | Me(S) | single bond | H | CH₂CF₃ | C(=O)Me | Me | C(=O)Me | O |
| C-1451 | H | CH₂ | Me | CH₂CF₃ | C(=O)Me | Me | C(=O)Me | O |
| C-1452 | Me | CH₂ | H | CH₂CF₃ | C(=O)Me | Me | C(=O)Me | O |
| C-1453 | H | CH(Me) | H | CH₂CF₃ | C(=O)Me | Me | C(=O)Me | O |
| C-1454 | H | CH₂CH₂ | Me | CH₂CF₃ | C(=O)Me | Me | C(=O)Me | O |
| C-1455 | Me | CH₂CH₂ | H | CH₂CF₃ | C(=O)Me | Me | C(=O)Me | O |
| C-1456 | H | single bond | Me | CH₂CF₃ | C(=O)(*i*-Pr) | Cl | Me | O |
| C-1457 | Me | single bond | H | CH₂CF₃ | C(=O)(*i*-Pr) | Cl | Me | O |
| C-1458 | Me(R) | single bond | H | CH₂CF₃ | C(=O)(*i*-Pr) | Cl | Me | O |
| C-1459 | Me(S) | single bond | H | CH₂CF₃ | C(=O)(*i*-Pr) | Cl | Me | O |
| C-1460 | H | CH₂ | Me | CH₂CF₃ | C(=O)(*i*-Pr) | Cl | Me | O |
| C-1461 | Me | CH₂ | H | CH₂CF₃ | C(=O)(*i*-Pr) | Cl | Me | O |
| C-1462 | H | CH(Me) | H | CH₂CF₃ | C(=O)(*i*-Pr) | Cl | Me | O |
| C-1463 | H | CH₂CH₂ | Me | CH₂CF₃ | C(=O)(*i*-Pr) | Cl | Me | O |
| C-1464 | Me | CH₂CH₂ | H | CH₂CF₃ | C(=O)(*i*-Pr) | Cl | Me | O |
| C-1465 | H | single bond | Me | CH₂CF₃ | C(=O)(*i*-Pr) | Me | Me | O |
| C-1466 | Me | single bond | H | CH₂CF₃ | C(=O)(*i*-Pr) | Me | Me | O |
| C-1467 | Me(R) | single bond | H | CH₂CF₃ | C(=O)(*i*-Pr) | Me | Me | O |
| C-1468 | Me(S) | single bond | H | CH₂CF₃ | C(=O)(*i*-Pr) | Me | Me | O |
| C-1469 | H | CH₂ | Me | CH₂CF₃ | C(=O)(*i*-Pr) | Me | Me | O |
| C-1470 | Me | CH₂ | H | CH₂CF₃ | C(=O)(*i*-Pr) | Me | Me | O |
| C-1471 | H | CH(Me) | H | CH₂CF₃ | C(=O)(*i*-Pr) | Me | Me | O |
| C-1472 | H | CH₂CH₂ | Me | CH₂CF₃ | C(=O)(*i*-Pr) | Me | Me | O |
| C-1473 | Me | CH₂CH₂ | H | CH₂CF₃ | C(=O)(*i*-Pr) | Me | Me | O |
| C-1474 | H | single bond | Me | CH₂CF₃ | C(=O)(*i*-Pr) | Cl | Et | O |
| C-1475 | Me | single bond | H | CH₂CF₃ | C(=O)(*i*-Pr) | Cl | Et | O |
| C-1476 | Me(R) | single bond | H | CH₂CF₃ | C(=O)(*i*-Pr) | Cl | Et | O |
| C-1477 | Me(S) | single bond | H | CH₂CF₃ | C(=O)(*i*-Pr) | Cl | Et | O |
| C-1478 | H | CH₂ | Me | CH₂CF₃ | C(=O)(*i*-Pr) | Cl | Et | O |
| C-1479 | Me | CH₂ | H | CH₂CF₃ | C(=O)(*i*-Pr) | Cl | Et | O |
| C-1480 | H | CH(Me) | H | CH₂CF₃ | C(=O)(*i*-Pr) | Cl | Et | O |
| C-1481 | H | CH₂CH₂ | Me | CH₂CF₃ | C(=O)(*i*-Pr) | Cl | Et | O |
| C-1482 | Me | CH₂CH₂ | H | CH₂CF₃ | C(=0)(i-Pr) | Cl | Et | O |
| C-1483 | H | single bond | Me | CH₂CF₃ | C(=O)(*i*-Pr) | Me | Et | O |
| C-1484 | Me | single bond | H | CH₂CF₃ | C(=O)(*i*-Pr) | Me | Et | O |
| C-1485 | Me(R) | single bond | H | CH₂CF₃ | C(=O)(*i*-Pr) | Me | Et | O |
| C-1486 | Me(S) | single bond | H | CH₂CF₃ | C(=O)(*i*-Pr) | Me | Et | O |
| C-1487 | H | CH₂ | Me | CH₂CF₃ | C(=O)(*i*-Pr) | Me | Et | O |
| C-1488 | Me | CH₂ | H | CH₂CF₃ | C(=O)(*i*-Pr) | Me | Et | O |
| C-1489 | H | CH(Me) | H | CH₂CF₃ | C(=O)(*i*-Pr) | Me | Et | O |
| C-1490 | H | CH₂CH₂ | Me | CH₂CF₃ | C(=O)(*i*-Pr) | Me | Et | O |

**[Table 261]**

| Compound Number | Y | W | Z | R¹ | R² | R³ | R¹⁰ | A |
|---|---|---|---|---|---|---|---|---|
| C-1491 | Me | CH₂CH₂ | H | CH₂CF₃ | C(=O)(*i*-Pr) | Me | Et | O |
| C-1492 | H | single bond | Me | CH₂CF₃ | C(=O)(*i*-Pr) | Cl | CH₂CF₃ | O |
| C-1493 | Me | single bond | H | CH₂CF₃ | C(=O)(*i*-Pr) | Cl | CH₂CF₃ | O |
| C-1494 | Me(R) | single bond | H | CH₂CF₃ | C(=O)(*i*-Pr) | Cl | CH₂CF₃ | O |
| C-1495 | Me(S) | single bond | H | CH₂CF₃ | C(=O)(*i*-Pr) | Cl | CH₂CF₃ | O |
| C-1496 | H | CH₂ | Me | CH₂CF₃ | C(=O)(*i*-Pr) | Cl | CH₂CF₃ | O |
| C-1497 | Me | CH₂ | H | CH₂CF₃ | C(=O)(*i*-Pr) | Cl | CH₂CF₃ | O |
| C-1498 | H | CH(Me) | H | CH₂CF₃ | C(=O)(*i*-Pr) | Cl | CH₂CF₃ | O |
| C-1499 | H | CH₂CH₂ | Me | CH₂CF₃ | C(=O)(*i*-Pr) | Cl | CH₂CF₃ | O |
| C-1500 | Me | CH₂CH₂ | H | CH₂CF₃ | C(=O)(*i*-Pr) | Cl | CH₂CF₃ | O |
| C-1501 | H | single bond | Me | CH₂CF₃ | C(=O)(*i*-Pr) | Me | CH₂CF₃ | O |
| C-1502 | Me | single bond | H | CH₂CF₃ | C(=O)(*i*-Pr) | Me | CH₂CF₃ | O |
| C-1503 | Me(R) | single bond | H | CH₂CF₃ | C(=O)(*i*-Pr) | Me | CH₂CF₃ | O |
| C-1504 | Me(S) | single bond | H | CH₂CF₃ | C(=O)(*i*-Pr) | Me | CH₂CF₃ | O |
| C-1505 | H | CH₂ | Me | CH₂CF₃ | C(=O)(*i*-Pr) | Me | CH₂CF₃ | O |
| C-1506 | Me | CH₂ | H | CH₂CF₃ | C(=O)(*i*-Pr) | Me | CH₂CF₃ | O |
| C-1507 | H | CH(Me) | H | CH₂CF₃ | C(=O)(*i*-Pr) | Me | CH₂CF₃ | O |
| C-1508 | H | CH₂CH₂ | Me | CH₂CF₃ | C(=O)(*i*-Pr) | Me | CH₂CF₃ | O |
| C-1509 | Me | CH₂CH₂ | H | CH₂CF₃ | C(=O)(*i*-Pr) | Me | CH₂CF₃ | O |
| C-1510 | H | single bond | Me | CH₂CF₃ | C(=O)(*i*-Pr) | Cl | CH₂OMe | O |
| C-1511 | Me | single bond | H | CH₂CF₃ | C(=O)(*i*-Pr) | Cl | CH₂OMe | O |
| C-1512 | Me(R) | single bond | H | CH₂CF₃ | C(=O)(*i*-Pr) | Cl | CH₂OMe | O |
| C-1513 | Me(S) | single bond | H | CH₂CF₃ | C(=O)(*i*-Pr) | Cl | CH₂OMe | O |
| C-1514 | H | CH₂ | Me | CH₂CF₃ | C(=O)(*i*-Pr) | Cl | CH₂OMe | O |
| C-1515 | Me | CH₂ | H | CH₂CF₃ | C(=O)(*i*-Pr) | Cl | CH₂OMe | O |
| C-1516 | H | CH(Me) | H | CH₂CF₃ | C(=O)(*i*-Pr) | Cl | CH₂OMe | O |
| C-1517 | H | CH₂CH₂ | Me | CH₂CF₃ | C(=O)(*i*-Pr) | Cl | CH₂OMe | O |
| C-1518 | Me | CH₂CH₂ | H | CH₂CF₃ | C(=O)(*i*-Pr) | Cl | CH₂OMe | O |
| C-1519 | H | single bond | Me | CH₂CF₃ | C(=O)(*i*-Pr) | Me | CH₂OMe | O |
| C-1520 | Me | single bond | H | CH₂CF₃ | C(=O)(*i*-Pr) | Me | CH₂OMe | O |
| C-1521 | Me(R) | single bond | H | CH₂CF₃ | C(=O)(*i*-Pr) | Me | CH₂OMe | O |
| C-1522 | Me(S) | single bond | H | CH₂CF₃ | C(=O)(*i*-Pr) | Me | CH₂OMe | O |
| C-1523 | H | CH₂ | Me | CH₂CF₃ | C(=O)(*i*-Pr) | Me | CH₂OMe | O |
| C-1524 | Me | CH₂ | H | CH₂CF₃ | C(=O)(*i*-Pr) | Me | CH₂OMe | O |
| C-1525 | H | CH(Me) | H | CH₂CF₃ | C(=O)(*i*-Pr) | Me | CH₂OMe | O |
| C-1526 | H | CH₂CH₂ | Me | CH₂CF₃ | C(=O)(*i*-Pr) | Me | CH₂OMe | O |
| C-1527 | Me | CH₂CH₂ | H | CH₂CF₃ | C(=O)(*i*-Pr) | Me | CH₂OMe | O |
| C-1528 | H | single bond | Me | CH₂CF₃ | C(=O)(*i*-Pr) | Cl | CH₂CN | O |
| C-1529 | Me | single bond | H | CH₂CF₃ | C(=O)(*i*-Pr) | Cl | CH₂CN | O |
| C-1530 | Me(R) | single bond | H | CH₂CF₃ | C(=O)(*i*-Pr) | Cl | CH₂CN | O |
| C-1531 | Me(S) | single bond | H | CH₂CF₃ | C(=O)(*i*-Pr) | Cl | CH₂CN | O |
| C-1532 | H | CH₂ | Me | CH₂CF₃ | C(=O)(*i*-Pr) | Cl | CH₂CN | O |
| C-1533 | Me | CH₂ | H | CH₂CF₃ | C(=O)(*i*-Pr) | Cl | CH₂CN | O |
| C-1534 | H | CH(Me) | H | CH₂CF₃ | C(=O)(*i*-Pr) | Cl | CH₂CN | O |

**[Table 262]**

| Compound Number | Y | W | Z | R¹ | R² | R³ | R¹⁰ | A |
|---|---|---|---|---|---|---|---|---|
| C-1535 | H | CH₂CH₂ | Me | CH₂CF₃ | C(=o)(*i*-Pr) | Cl | CH₂CN | O |
| C-1536 | Me | CH₂CH₂ | H | CH₂CF₃ | C(=o)(*i*-Pr) | Cl | CH₂CN | O |
| C-1537 | H | single bond | Me | CH₂CF₃ | C(=o)(*i*-Pr) | Me | CH₂CN | O |
| C-1538 | Me | single bond | H | CH₂CF₃ | C(=o)(*i*-Pr) | Me | CH₂CN | O |
| C-1539 | Me(R) | single bond | H | CH₂CF₃ | C(=o)(*i*-Pr) | Me | CH₂CN | O |
| C-1540 | Me(S) | single bond | H | CH₂CF₃ | C(=o)(*i*-Pr) | Me | CH₂CN | O |
| C-1541 | H | CH₂ | Me | CH₂CF₃ | C(=o)(*i*-Pr) | Me | CH₂CN | O |
| C-1542 | Me | CH₂ | H | CH₂CF₃ | C(=o)(*i*-Pr) | Me | CH₂CN | O |
| C-1543 | H | CH(Me) | H | CH₂CF₃ | C(=o)(*i*-Pr) | Me | CH₂CN | O |
| C-1544 | H | CH₂CH₂ | Me | CH₂CF₃ | C(=o)(*i*-Pr) | Me | CH₂CN | O |
| C-1545 | Me | CH₂CH₂ | H | CH₂CF₃ | C(=o)(*i*-Pr) | Me | CH₂CN | O |
| C-1546 | H | single bond | Me | CH₂CF₃ | C(=o)(*i*-Pr) | Cl | C(=O)Me | O |
| C-1547 | Me | single bond | H | CH₂CF₃ | C(=o)(*i*-Pr) | Cl | C(=O)Me | O |
| C-1548 | Me(R) | single bond | H | CH₂CF₃ | C(=o)(*i*-Pr) | Cl | C(=O)Me | O |
| C-1549 | Me(S) | single bond | H | CH₂CF₃ | C(=o)(*i*-Pr) | Cl | C(=O)Me | O |
| C-1550 | H | CH₂ | Me | CH₂CF₃ | C(=o)(*i*-Pr) | Cl | C(=O)Me | O |
| C-1551 | Me | CH₂ | H | CH₂CF₃ | C(=O)(*i*-Pr) | Cl | C(=O)Me | O |
| C-1552 | H | CH(Me) | H | CH₂CF₃ | C(=o)(*i*-Pr) | Cl | C(=O)Me | O |
| C-1553 | H | CH₂CH₂ | Me | CH₂CF₃ | C(=o)(*i*-Pr) | Cl | C(=O)Me | O |
| C-1554 | Me | CH₂CH₂ | H | CH₂CF₃ | C(=o)(*i*-Pr) | Cl | C(=O)Me | O |
| C-1555 | H | single bond | Me | CH₂CF₃ | C(=O)(*i*-Pr) | Me | C(=O)Me | O |
| C-1556 | Me | single bond | H | CH₂CF₃ | C(=o)(*i*-Pr) | Me | C(=O)Me | O |
| C-1557 | Me(R) | single bond | H | CH₂CF₃ | C(=o)(*i*-Pr) | Me | C(=O)Me | O |
| C-1558 | Me(S) | single bond | H | CH₂CF₃ | C(=o)(*i*-Pr) | Me | C(=O)Me | O |
| C-1559 | H | CH₂ | Me | CH₂CF₃ | C(=O)(*i*-Pr) | Me | C(=O)Me | O |
| C-1560 | Me | CH₂ | H | CH₂CF₃ | C(=o)(*i*-Pr) | Me | C(=O)Me | O |
| C-1561 | H | CH(Me) | H | CH₂CF₃ | C(=o)(*i*-Pr) | Me | C(=O)Me | O |
| C-1562 | H | CH₂CH₂ | Me | CH₂CF₃ | C(=o)(*i*-Pr) | Me | C(=O)Me | O |
| C-1563 | Me | CH₂CH₂ | H | CH₂CF₃ | C(=O)(*i*-Pr) | Me | C(=O)Me | O |
| C-1564 | H | single bond | Me | CH₂CF₃ | C(=O)Ph | Cl | Me | O |
| C-1565 | Me | single bond | H | CH₂CF₃ | C(=O)Ph | Cl | Me | O |
| C-1566 | Me(R) | single bond | H | CH₂CF₃ | C(=O)Ph | Cl | Me | O |
| C-1567 | Me(S) | single bond | H | CH₂CF₃ | C(=O)Ph | Cl | Me | O |
| C-1568 | H | CH₂ | Me | CH₂CF₃ | C(=O)Ph | Cl | Me | O |
| C-1569 | Me | CH₂ | H | CH₂CF₃ | C(=O)Ph | Cl | Me | O |
| C-1570 | H | CH(Me) | H | CH₂CF₃ | C(=O)Ph | Cl | Me | O |
| C-1571 | H | CH₂CH₂ | Me | CH₂CF₃ | C(=O)Ph | Cl | Me | O |
| C-1572 | Me | CH₂CH₂ | H | CH₂CF₃ | C(=O)Ph | Cl | Me | O |
| C-1573 | H | single bond | Me | CH₂CF₃ | C(=O)Ph | Me | Me | O |
| C-1574 | Me | single bond | H | CH₂CF₃ | C(=O)Ph | Me | Me | O |
| C-1575 | Me(R) | single bond | H | CH₂CF₃ | C(=O)Ph | Me | Me | O |
| C-1576 | Me(S) | single bond | H | CH₂CF₃ | C(=O)Ph | Me | Me | O |
| C-1577 | H | CH₂ | Me | CH₂CF₃ | C(=O)Ph | Me | Me | O |
| C-1578 | Me | CH₂ | H | CH₂CF₃ | C(=O)Ph | Me | Me | O |

**[Table 263]**

| Compound Number | Y | W | Z | R¹ | R² | R³ | R¹⁰ | A |
|---|---|---|---|---|---|---|---|---|
| C-1579 | H | CH(Me) | H | CH₂CF₃ | C(=O)Ph | Me | Me | O |
| C-1580 | H | CH₂CH₂ | Me | CH₂CF₃ | C(=O)Ph | Me | Me | O |
| C-1581 | Me | CH₂CH₂ | H | CH₂CF₃ | C(=O)Ph | Me | Me | O |
| C-1582 | H | single bond | Me | CH₂CF₃ | C(=O)Ph | Cl | Et | O |
| C-1583 | Me | single bond | H | CH₂CF₃ | C(=O)Ph | Cl | Et | O |
| C-1584 | Me(R) | single bond | H | CH₂CF₃ | C(=O)Ph | Cl | Et | O |
| C-1585 | Me(S) | single bond | H | CH₂CF₃ | C(=O)Ph | Cl | Et | O |
| C-1586 | H | CH₂ | Me | CH₂CF₃ | C(=O)Ph | Cl | Et | O |
| C-1587 | Me | CH₂ | H | CH₂CF₃ | C(=O)Ph | Cl | Et | O |
| C-1588 | H | CH(Me) | H | CH₂CF₃ | C(=O)Ph | Cl | Et | O |
| C-1589 | H | CH₂CH₂ | Me | CH₂CF₃ | C(=O)Ph | Cl | Et | O |
| C-1590 | Me | CH₂CH₂ | H | CH₂CF₃ | C(=O)Ph | Cl | Et | O |
| C-1591 | H | single bond | Me | CH₂CF₃ | C(=O)Ph | Me | Et | O |
| C-1592 | Me | single bond | H | CH₂CF₃ | C(=O)Ph | Me | Et | O |
| C-1593 | Me(R) | single bond | H | CH₂CF₃ | C(=O)Ph | Me | Et | O |
| C-1594 | Me(S) | single bond | H | CH₂CF₃ | C(=O)Ph | Me | Et | O |
| C-1595 | H | CH₂ | Me | CH₂CF₃ | C(=O)Ph | Me | Et | O |
| C-1596 | Me | CH₂ | H | CH₂CF₃ | C(=O)Ph | Me | Et | O |
| C-1597 | H | CH(Me) | H | CH₂CF₃ | C(=O)Ph | Me | Et | O |
| C-1598 | H | CH₂CH₂ | Me | CH₂CF₃ | C(=O)Ph | Me | Et | O |
| C-1599 | Me | CH₂CH₂ | H | CH₂CF₃ | C(=O)Ph | Me | Et | O |
| C-1600 | H | single bond | Me | CH₂CF₃ | C(=O)Ph | Cl | CH₂CF₃ | O |
| C-1601 | Me | single bond | H | CH₂CF₃ | C(=O)Ph | Cl | CH₂CF₃ | O |
| C-1602 | Me(R) | single bond | H | CH₂CF₃ | C(=O)Ph | Cl | CH₂CF₃ | O |
| C-1603 | Me(S) | single bond | H | CH₂CF₃ | C(=O)Ph | Cl | CH₂CF₃ | O |
| C-1604 | H | CH₂ | Me | CH₂CF₃ | C(=O)Ph | Cl | CH₂CF₃ | O |
| C-1605 | Me | CH₂ | H | CH₂CF₃ | C(=O)Ph | Cl | CH₂CF₃ | O |
| C-1606 | H | CH(Me) | H | CH₂CF₃ | C(=O)Ph | Cl | CH₂CF₃ | O |
| C-1607 | H | CH₂CH₂ | Me | CH₂CF₃ | C(=O)Ph | Cl | CH₂CF₃ | O |
| C-1608 | Me | CH₂CH₂ | H | CH₂CF₃ | C(=O)Ph | Cl | CH₂CF₃ | O |
| C-1609 | H | single bond | Me | CH₂CF₃ | C(=O)Ph | Me | CH₂CF₃ | O |
| C-1610 | Me | single bond | H | CH₂CF₃ | C(=O)Ph | Me | CH₂CF₃ | O |
| C-1611 | Me(R) | single bond | H | CH₂CF₃ | C(=O)Ph | Me | CH₂CF₃ | O |
| C-1612 | Me(S) | single bond | H | CH₂CF₃ | C(=O)Ph | Me | CH₂CF₃ | O |
| C-1613 | H | CH₂ | Me | CH₂CF₃ | C(=O)Ph | Me | CH₂CF₃ | O |
| C-1614 | Me | CH₂ | H | CH₂CF₃ | C(=O)Ph | Me | CH₂CF₃ | O |
| C-1615 | H | CH(Me) | H | CH₂CF₃ | C(=O)Ph | Me | CH₂CF₃ | O |
| C-1616 | H | CH₂CH₂ | Me | CH₂CF₃ | C(=O)Ph | Me | CH₂CF₃ | O |
| C-1617 | Me | CH₂CH₂ | H | CH₂CF₃ | C(=O)Ph | Me | CH₂CF₃ | O |
| C-1618 | H | single bond | Me | CH₂CF₃ | C(=O)Ph | Cl | CH₂OMe | O |
| C-1619 | Me | single bond | H | CH₂CF₃ | C(=O)Ph | Cl | CH₂OMe | O |
| C-1620 | Me(R) | single bond | H | CH₂CF₃ | C(=O)Ph | Cl | CH₂OMe | O |
| C-1621 | Me(S) | single bond | H | CH₂CF₃ | C(=O)Ph | Cl | CH₂OMe | O |
| C-1622 | H | CH₂ | Me | CH₂CF₃ | C(=O)Ph | Cl | CH₂OMe | O |

**[Table 264]**

| Compound Number | Y | W | Z | R¹ | R² | R³ | R¹⁰ | A |
|---|---|---|---|---|---|---|---|---|
| C-1623 | Me | CH₂ | H | CH₂CF₃ | C(=O)Ph | Cl | CH₂OMe | O |
| C-1624 | H | CH(Me) | H | CH₂CF₃ | C(=O)Ph | Cl | CH₂OMe | O |
| C-1625 | H | CH₂CH₂ | Me | CH₂CF₃ | C(=O)Ph | Cl | CH₂OMe | O |
| C-1626 | Me | CH₂CH₂ | H | CH₂CF₃ | C(=O)Ph | Cl | CH₂OMe | O |
| C-1627 | H | single bond | Me | CH₂CF₃ | C(=O)Ph | Me | CH₂OMe | O |
| C-1628 | Me | single bond | H | CH₂CF₃ | C(=O)Ph | Me | CH₂OMe | O |
| C-1629 | Me(R) | single bond | H | CH₂CF₃ | C(=O)Ph | Me | CH₂OMe | O |
| C-1630 | Me(S) | single bond | H | CH₂CF₃ | C(=O)Ph | Me | CH₂OMe | O |
| C-1631 | H | CH₂ | Me | CH₂CF₃ | C(=O)Ph | Me | CH₂OMe | O |
| C-1632 | Me | CH₂ | H | CH₂CF₃ | C(=O)Ph | Me | CH₂OMe | O |
| C-1633 | H | CH(Me) | H | CH₂CF₃ | C(=O)Ph | Me | CH₂OMe | O |
| C-1634 | H | CH₂CH₂ | Me | CH₂CF₃ | C(=O)Ph | Me | CH₂OMe | O |
| C-1635 | Me | CH₂CH₂ | H | CH₂CF₃ | C(=O)Ph | Me | CH₂OMe | O |
| C-1636 | H | single bond | Me | CH₂CF₃ | C(=O)Ph | Cl | CH₂CN | O |
| C-1637 | Me | single bond | H | CH₂CF₃ | C(=O)Ph | Cl | CH₂CN | O |
| C-1638 | Me(R) | single bond | H | CH₂CF₃ | C(=O)Ph | Cl | CH₂CN | O |
| C-1639 | Me(S) | single bond | H | CH₂CF₃ | C(=O)Ph | Cl | CH₂CN | O |
| C-1640 | H | CH₂ | Me | CH₂CF₃ | C(=O)Ph | Cl | CH₂CN | O |
| C-1641 | Me | CH₂ | H | CH₂CF₃ | C(=O)Ph | Cl | CH₂CN | O |
| C-1642 | H | CH(Me) | H | CH₂CF₃ | C(=O)Ph | Cl | CH₂CN | O |
| C-1643 | H | CH₂CH₂ | Me | CH₂CF₃ | C(=O)Ph | Cl | CH₂CN | O |
| C-1644 | Me | CH₂CH₂ | H | CH₂CF₃ | C(=O)Ph | Cl | CH₂CN | O |
| C-1645 | H | single bond | Me | CH₂CF₃ | C(=O)Ph | Me | CH₂CN | O |
| C-1646 | Me | single bond | H | CH₂CF₃ | C(=O)Ph | Me | CH₂CN | O |
| C-1647 | Me(R) | single bond | H | CH₂CF₃ | C(=O)Ph | Me | CH₂CN | O |
| C-1648 | Me(S) | single bond | H | CH₂CF₃ | C(=O)Ph | Me | CH₂CN | O |
| C-1649 | H | CH₂ | Me | CH₂CF₃ | C(=O)Ph | Me | CH₂CN | O |
| C-1650 | Me | CH₂ | H | CH₂CF₃ | C(=O)Ph | Me | CH₂CN | O |
| C-1651 | H | CH(Me) | H | CH₂CF₃ | C(=O)Ph | Me | CH₂CN | O |
| C-1652 | H | CH₂CH₂ | Me | CH₂CF₃ | C(=O)Ph | Me | CH₂CN | O |
| C-1653 | Me | CH₂CH₂ | H | CH₂CF₃ | C(=O)Ph | Me | CH₂CN | O |
| C-1654 | H | single bond | Me | CH₂CF₃ | C(=O)Ph | Cl | C(=O)Me | O |
| C-1655 | Me | single bond | H | CH₂CF₃ | C(=O)Ph | Cl | C(=O)Me | O |
| C-1656 | Me(R) | single bond | H | CH₂CF₃ | C(=O)Ph | Cl | C(=O)Me | O |
| C-1657 | Me(S) | single bond | H | CH₂CF₃ | C(=O)Ph | Cl | C(=O)Me | O |
| C-1658 | H | CH₂ | Me | CH₂CF₃ | C(=O)Ph | Cl | C(=O)Me | O |
| C-1659 | Me | CH₂ | H | CH₂CF₃ | C(=O)Ph | Cl | C(=O)Me | O |
| C-1660 | H | CH(Me) | H | CH₂CF₃ | C(=O)Ph | Cl | C(=O)Me | O |
| C-1661 | H | CH₂CH₂ | Me | CH₂CF₃ | C(=O)Ph | Cl | C(=O)Me | O |
| C-1662 | Me | CH₂CH₂ | H | CH₂CF₃ | C(=O)Ph | Cl | C(=O)Me | O |
| C-1663 | H | single bond | Me | CH₂CF₃ | C(=O)Ph | Me | C(=O)Me | O |
| C-1664 | Me | single bond | H | CH₂CF₃ | C(=O)Ph | Me | C(=O)Me | O |
| C-1665 | Me(R) | single bond | H | CH₂CF₃ | C(=O)Ph | Me | C(=O)Me | O |
| C-1666 | Me(S) | single bond | H | CH₂CF₃ | C(=O)Ph | Me | C(=O)Me | O |

**[Table 265]**

| Compound Number | Y | W | Z | R¹ | R² | R³ | R¹⁰ | A |
|---|---|---|---|---|---|---|---|---|
| C-1667 | H | CH₂ | Me | CH₂CF₃ | C(=O)Ph | Me | C(=O)Me | O |
| C-1668 | Me | CH₂ | H | CH₂CF₃ | C(=O)Ph | Me | C(=O)Me | O |
| C-1669 | H | CH(Me) | H | CH₂CF₃ | C(=O)Ph | Me | C(=O)Me | O |
| C-1670 | H | CH₂CH₂ | Me | CH₂CF₃ | C(=O)Ph | Me | C(=O)Me | O |
| C-1671 | Me | CH₂CH₂ | H | CH₂CF₃ | C(=O)Ph | Me | C(=O)Me | O |
| C-1672 | H | single bond | Me | CH₂CF₃ | C(=O)OEt | Cl | Me | O |
| C-1673 | H | single bond | Et | CH₂CF₃ | C(=O)OEt | Cl | Me | O |
| C-1674 | Me | single bond | H | CH₂CF₃ | C(=O)OEt | Cl | Me | O |
| C-1675 | Me(R) | single bond | H | CH₂CF₃ | C(=O)OEt | Cl | Me | O |
| C-1676 | Me(S) | single bond | H | CH₂CF₃ | C(=O)OEt | Cl | Me | O |
| C-1677 | H | CH₂ | Me | CH₂CF₃ | C(=O)OEt | Cl | Me | O |
| C-1678 | Me | CH₂ | H | CH₂CF₃ | C(=O)OEt | Cl | Me | O |
| C-1679 | H | CH(Me) | H | CH₂CF₃ | C(=O)OEt | Cl | Me | O |
| C-1680 | H | CH₂CH₂ | Me | CH₂CF₃ | C(=O)OEt | Cl | Me | O |
| C-1681 | Me | CH₂CH₂ | H | CH₂CF₃ | C(=O)OEt | Cl | Me | O |
| C-1682 | H | single bond | Me | CH₂CF₃ | C(=O)OEt | Me | Me | O |
| C-1683 | Me | single bond | H | CH₂CF₃ | C(=O)OEt | Me | Me | O |
| C-1684 | Me(R) | single bond | H | CH₂CF₃ | C(=O)OEt | Me | Me | O |
| C-1685 | Me(S) | single bond | H | CH₂CF₃ | C(=O)OEt | Me | Me | O |
| C-1686 | H | CH₂ | Me | CH₂CF₃ | C(=O)OEt | Me | Me | O |
| C-1687 | Me | CH₂ | H | CH₂CF₃ | C(=O)OEt | Me | Me | O |
| C-1688 | H | CH(Me) | H | CH₂CF₃ | C(=O)OEt | Me | Me | O |
| C-1689 | H | CH₂CH₂ | Me | CH₂CF₃ | C(=O)OEt | Me | Me | O |
| C-1690 | Me | CH₂CH₂ | H | CH₂CF₃ | C(=O)OEt | Me | Me | O |
| C-1691 | H | single bond | Me | CH₂CF₃ | C(=O)OEt | Cl | Et | O |
| C-1692 | Me | single bond | H | CH₂CF₃ | C(=O)OEt | Cl | Et | O |
| C-1693 | Me(R) | single bond | H | CH₂CF₃ | C(=O)OEt | Cl | Et | O |
| C-1694 | Me(S) | single bond | H | CH₂CF₃ | C(=O)OEt | Cl | Et | O |
| C-1695 | H | CH₂ | Me | CH₂CF₃ | C(=O)OEt | Cl | Et | O |
| C-1696 | Me | CH₂ | H | CH₂CF₃ | C(=O)OEt | Cl | Et | O |
| C-1697 | H | CH(Me) | H | CH₂CF₃ | C(=O)OEt | Cl | Et | O |
| C-1698 | H | CH₂CH₂ | Me | CH₂CF₃ | C(=O)OEt | Cl | Et | O |
| C-1699 | Me | CH₂CH₂ | H | CH₂CF₃ | C(=O)OEt | Cl | Et | O |
| C-1700 | H | single bond | Me | CH₂CF₃ | C(=O)OEt | Me | Et | O |
| C-1701 | Me | single bond | H | CH₂CF₃ | C(=O)OEt | Me | Et | O |
| C-1702 | Me(R) | single bond | H | CH₂CF₃ | C(=O)OEt | Me | Et | O |
| C-1703 | Me(S) | single bond | H | CH₂CF₃ | C(=O)OEt | Me | Et | O |
| C-1704 | H | CH₂ | Me | CH₂CF₃ | C(=O)OEt | Me | Et | O |
| C-1705 | Me | CH₂ | H | CH₂CF₃ | C(=O)OEt | Me | Et | O |
| C-1706 | H | CH(Me) | H | CH₂CF₃ | C(=O)OEt | Me | Et | O |
| C-1707 | H | CH₂CH₂ | Me | CH₂CF₃ | C(=O)OEt | Me | Et | O |
| C-1708 | Me | CH₂CH₂ | H | CH₂CF₃ | C(=O)OEt | Me | Et | O |
| C-1709 | H | single bond | Me | CH₂CF₃ | C(=O)OEt | Cl | CH₂CF₃ | O |
| C-1710 | Me | single bond | H | CH₂CF₃ | C(=O)OEt | Cl | CH₂CF₃ | O |

**[Table 266]**

| Compound Number | Y | W | Z | R¹ | R² | R³ | R¹⁰ | A |
|---|---|---|---|---|---|---|---|---|
| C-1711 | Me(R) | single bond | H | CH₂CF₃ | C(=O)OEt | Cl | CH₂CF₃ | O |
| C-1712 | Me(S) | single bond | H | CH₂CF₃ | C(=O)OEt | Cl | CH₂CF₃ | O |
| C-1713 | H | CH₂ | Me | CH₂CF₃ | C(=O)OEt | Cl | CH₂CF₃ | O |
| C-1714 | Me | CH₂ | H | CH₂CF₃ | C(=O)OEt | Cl | CH₂CF₃ | O |
| C-1715 | H | CH(Me) | H | CH₂CF₃ | C(=O)OEt | Cl | CH₂CF₃ | O |
| C-1716 | H | CH₂CH₂ | Me | CH₂CF₃ | C(=O)OEt | Cl | CH₂CF₃ | O |
| C-1717 | Me | CH₂CH₂ | H | CH₂CF₃ | C(=O)OEt | Cl | CH₂CF₃ | O |
| C-1718 | H | single bond | Me | CH₂CF₃ | C(=O)OEt | Me | CH₂CF₃ | O |
| C-1719 | Me | single bond | H | CH₂CF₃ | C(=O)OEt | Me | CH₂CF₃ | O |
| C-1720 | Me(R) | single bond | H | CH₂CF₃ | C(=O)OEt | Me | CH₂CF₃ | O |
| C-1721 | Me(S) | single bond | H | CH₂CF₃ | C(=O)OEt | Me | CH₂CF₃ | O |
| C-1722 | H | CH₂ | Me | CH₂CF₃ | C(=O)OEt | Me | CH₂CF₃ | O |
| C-1723 | Me | CH₂ | H | CH₂CF₃ | C(=O)OEt | Me | CH₂CF₃ | O |
| C-1724 | H | CH(Me) | H | CH₂CF₃ | C(=O)OEt | Me | CH₂CF₃ | O |
| C-1725 | H | CH₂CH₂ | Me | CH₂CF₃ | C(=O)OEt | Me | CH₂CF₃ | O |
| C-1726 | Me | CH₂CH₂ | H | CH₂CF₃ | C(=O)OEt | Me | CH₂CF₃ | O |
| C-1727 | H | single bond | Me | CH₂CF₃ | C(=O)OEt | Cl | CH₂OMe | O |
| C-1728 | Me | single bond | H | CH₂CF₃ | C(=O)OEt | Cl | CH₂OMe | O |
| C-1729 | Me(R) | single bond | H | CH₂CF₃ | C(=O)OEt | Cl | CH₂OMe | O |
| C-1730 | Me(S) | single bond | H | CH₂CF₃ | C(=O)OEt | Cl | CH₂OMe | O |
| C-1731 | H | CH₂ | Me | CH₂CF₃ | C(=O)OEt | Cl | CH₂OMe | O |
| C-1732 | Me | CH₂ | H | CH₂CF₃ | C(=O)OEt | Cl | CH₂OMe | O |
| C-1733 | H | CH(Me) | H | CH₂CF₃ | C(=O)OEt | Cl | CH₂OMe | O |
| C-1734 | H | CH₂CH₂ | Me | CH₂CF₃ | C(=O)OEt | Cl | CH₂OMe | O |
| C-1735 | Me | CH₂CH₂ | H | CH₂CF₃ | C(=O)OEt | Cl | CH₂OMe | O |
| C-1736 | H | single bond | Me | CH₂CF₃ | C(=O)OEt | Me | CH₂OMe | O |
| C-1737 | Me | single bond | H | CH₂CF₃ | C(=O)OEt | Me | CH₂OMe | O |
| C-1738 | Me(R) | single bond | H | CH₂CF₃ | C(=O)OEt | Me | CH₂OMe | O |
| C-1739 | Me(S) | single bond | H | CH₂CF₃ | C(=O)OEt | Me | CH₂OMe | O |
| C-1740 | H | CH₂ | Me | CH₂CF₃ | C(=O)OEt | Me | CH₂OMe | O |
| C-1741 | Me | CH₂ | H | CH₂CF₃ | C(=O)OEt | Me | CH₂OMe | O |
| C-1742 | H | CH(Me) | H | CH₂CF₃ | C(=O)OEt | Me | CH₂OMe | O |
| C-1743 | H | CH₂CH₂ | Me | CH₂CF₃ | C(=O)OEt | Me | CH₂OMe | O |
| C-1744 | Me | CH₂CH₂ | H | CH₂CF₃ | C(=O)OEt | Me | CH₂OMe | O |
| C-1745 | H | single bond | Me | CH₂CF₃ | C(=O)OEt | Cl | CH₂CN | O |
| C-1746 | Me | single bond | H | CH₂CF₃ | C(=O)OEt | Cl | CH₂CN | O |
| C-1747 | Me(R) | single bond | H | CH₂CF₃ | C(=O)OEt | Cl | CH₂CN | O |
| C-1748 | Me(S) | single bond | H | CH₂CF₃ | C(=O)OEt | Cl | CH₂CN | O |
| C-1749 | H | CH₂ | Me | CH₂CF₃ | C(=O)OEt | Cl | CH₂CN | O |
| C-1750 | Me | CH₂ | H | CH₂CF₃ | C(=O)OEt | Cl | CH₂CN | O |
| C-1751 | H | CH(Me) | H | CH₂CF₃ | C(=O)OEt | Cl | CH₂CN | O |
| C-1752 | H | CH₂CH₂ | Me | CH₂CF₃ | C(=O)OEt | Cl | CH₂CN | O |
| C-1753 | Me | CH₂CH₂ | H | CH₂CF₃ | C(=O)OEt | Cl | CH₂CN | O |
| C-1754 | H | single bond | Me | CH₂CF₃ | C(=O)OEt | Me | CH₂CN | O |

**[Table 267]**

| Compound Number | Y | W | Z | R¹ | R² | R³ | R¹⁰ | A |
|---|---|---|---|---|---|---|---|---|
| C-1755 | Me | single bond | H | CH₂CF₃ | C(=O)OEt | Me | CH₂CN | O |
| C-1756 | Me(R) | single bond | H | CH₂CF₃ | C(=O)OEt | Me | CH₂CN | O |
| C-1757 | Me(S) | single bond | H | CH₂CF₃ | C(=O)OEt | Me | CH₂CN | O |
| C-1758 | H | CH₂ | Me | CH₂CF₃ | C(=O)OEt | Me | CH₂CN | O |
| C-1759 | Me | CH₂ | H | CH₂CF₃ | C(=O)OEt | Me | CH₂CN | O |
| C-1760 | H | CH(Me) | H | CH₂CF₃ | C(=O)OEt | Me | CH₂CN | O |
| C-1761 | H | CH₂CH₂ | Me | CH₂CF₃ | C(=O)OEt | Me | CH₂CN | O |
| C-1762 | Me | CH₂CH₂ | H | CH₂CF₃ | C(=O)OEt | Me | CH₂CN | O |
| C-1763 | H | single bond | Me | CH₂CF₃ | C(=O)OEt | Cl | C(=O)Me | O |
| C-1764 | Me | single bond | H | CH₂CF₃ | C(=O)OEt | Cl | C(=O)Me | O |
| C-1765 | Me(R) | single bond | H | CH₂CF₃ | C(=O)OEt | Cl | C(=O)Me | O |
| C-1766 | Me(S) | single bond | H | CH₂CF₃ | C(=O)OEt | Cl | C(=O)Me | O |
| C-1767 | H | CH₂ | Me | CH₂CF₃ | C(=O)OEt | Cl | C(=O)Me | O |
| C-1768 | Me | CH₂ | H | CH₂CF₃ | C(=O)OEt | Cl | C(=O)Me | O |
| C-1769 | H | CH(Me) | H | CH₂CF₃ | C(=O)OEt | Cl | C(=O)Me | O |
| C-1770 | H | CH₂CH₂ | Me | CH₂CF₃ | C(=O)OEt | Cl | C(=O)Me | O |
| C-1771 | Me | CH₂CH₂ | H | CH₂CF₃ | C(=O)OEt | Cl | C(=O)Me | O |
| C-1772 | H | single bond | Me | CH₂CF₃ | C(=O)OEt | Me | C(=O)Me | O |
| C-1773 | Me | single bond | H | CH₂CF₃ | C(=O)OEt | Me | C(=O)Me | O |
| C-1774 | Me(R) | single bond | H | CH₂CF₃ | C(=O)OEt | Me | C(=O)Me | O |
| C-1775 | Me(S) | single bond | H | CH₂CF₃ | C(=O)OEt | Me | C(=O)Me | O |
| C-1776 | H | CH₂ | Me | CH₂CF₃ | C(=O)OEt | Me | C(=O)Me | O |
| C-1777 | Me | CH₂ | H | CH₂CF₃ | C(=O)OEt | Me | C(=O)Me | O |
| C-1778 | H | CH(Me) | H | CH₂CF₃ | C(=O)OEt | Me | C(=O)Me | O |
| C-1779 | H | CH₂CH₂ | Me | CH₂CF₃ | C(=O)OEt | Me | C(=O)Me | O |
| C-1780 | Me | CH₂CH₂ | H | CH₂CF₃ | C(=O)OEt | Me | C(=O)Me | O |
| C-1781 | H | single bond | Me | CH₂CF₃ | S(=O)₂Me | Cl | Me | O |
| C-1782 | H | single bond | Et | CH₂CF₃ | S(=O)₂Me | Cl | Me | O |
| C-1783 | Me | single bond | H | CH₂CF₃ | S(=O)₂Me | Cl | Me | O |
| C-1784 | Me(R) | single bond | H | CH₂CF₃ | S(=O)₂Me | Cl | Me | O |
| C-1785 | Me(S) | single bond | H | CH₂CF₃ | S(=O)₂Me | Cl | Me | O |
| C-1786 | H | CH₂ | Me | CH₂CF₃ | S(=O)₂Me | Cl | Me | O |
| C-1787 | Me | CH₂ | H | CH₂CF₃ | S(=O)₂Me | Cl | Me | O |
| C-1788 | H | CH(Me) | H | CH₂CF₃ | S(=O)₂Me | Cl | Me | O |
| C-1789 | H | CH₂CH₂ | Me | CH₂CF₃ | S(=O)₂Me | Cl | Me | O |
| C-1790 | Me | CH₂CH₂ | H | CH₂CF₃ | S(=O)₂Me | Cl | Me | O |
| C-1791 | H | single bond | Me | CH₂CF₃ | S(=O)₂Me | Me | Me | O |
| C-1792 | Me | single bond | H | CH₂CF₃ | S(=O)₂Me | Me | Me | O |
| C-1793 | Me(R) | single bond | H | CH₂CF₃ | S(=O)₂Me | Me | Me | O |
| C-1794 | Me(S) | single bond | H | CH₂CF₃ | S(=O)₂Me | Me | Me | O |
| C-1795 | H | CH₂ | Me | CH₂CF₃ | S(=O)₂Me | Me | Me | O |
| C-1796 | Me | CH₂ | H | CH₂CF₃ | S(=O)₂Me | Me | Me | O |
| C-1797 | H | CH(Me) | H | CH₂CF₃ | S(=O)₂Me | Me | Me | O |
| C-1798 | H | CH₂CH₂ | Me | CH₂CF₃ | S(=O)₂Me | Me | Me | O |

**[Table 268]**

| Compound Number | Y | W | Z | R¹ | R² | R³ | R¹⁰ | A |
|---|---|---|---|---|---|---|---|---|
| C-1799 | Me | CH₂CH₂ | H | CH₂CF₃ | S(=O)₂Me | Me | Me | O |
| C-1800 | H | single bond | Me | CH₂CF₃ | S(=O)₂Me | Cl | Et | O |
| C-1801 | Me | single bond | H | CH₂CF₃ | S(=O)₂Me | Cl | Et | O |
| C-1802 | Me(R) | single bond | H | CH₂CF₃ | S(=O)₂Me | Cl | Et | O |
| C-1803 | Me(S) | single bond | H | CH₂CF₃ | S(=O)₂Me | Cl | Et | O |
| C-1804 | H | CH₂ | Me | CH₂CF₃ | S(=O)₂Me | Cl | Et | O |
| C-1805 | Me | CH₂ | H | CH₂CF₃ | S(=O)₂Me | Cl | Et | O |
| C-1806 | H | CH(Me) | H | CH₂CF₃ | S(=O)₂Me | Cl | Et | O |
| C-1807 | H | CH₂CH₂ | Me | CH₂CF₃ | S(=O)₂Me | Cl | Et | O |
| C-1808 | Me | CH₂CH₂ | H | CH₂CF₃ | S(=O)₂Me | Cl | Et | O |
| C-1809 | H | single bond | Me | CH₂CF₃ | S(=O)₂Me | Me | Et | O |
| C-1810 | Me | single bond | H | CH₂CF₃ | S(=O)₂Me | Me | Et | O |
| C-1811 | Me(R) | single bond | H | CH₂CF₃ | S(=O)₂Me | Me | Et | O |
| C-1812 | Me(S) | single bond | H | CH₂CF₃ | S(=O)₂Me | Me | Et | O |
| C-1813 | H | CH₂ | Me | CH₂CF₃ | S(=O)₂Me | Me | Et | O |
| C-1814 | Me | CH₂ | H | CH₂CF₃ | S(=O)₂Me | Me | Et | O |
| C-1815 | H | CH(Me) | H | CH₂CF₃ | S(=O)₂Me | Me | Et | O |
| C-1816 | H | CH₂CH₂ | Me | CH₂CF₃ | S(=O)₂Me | Me | Et | O |
| C-1817 | Me | CH₂CH₂ | H | CH₂CF₃ | S(=O)₂Me | Me | Et | O |
| C-1818 | H | single bond | Me | CH₂CF₃ | S(=O)₂Me | Cl | CH₂CF₃ | O |
| C-1819 | Me | single bond | H | CH₂CF₃ | S(=O)₂Me | Cl | CH₂CF₃ | O |
| C-1820 | Me(R) | single bond | H | CH₂CF₃ | S(=O)₂Me | Cl | CH₂CF₃ | O |
| C-1821 | Me(S) | single bond | H | CH₂CF₃ | S(=O)₂Me | Cl | CH₂CF₃ | O |
| C-1822 | H | CH₂ | Me | CH₂CF₃ | S(=O)₂Me | Cl | CH₂CF₃ | O |
| C-1823 | Me | CH₂ | H | CH₂CF₃ | S(=O)₂Me | Cl | CH₂CF₃ | O |
| C-1824 | H | CH(Me) | H | CH₂CF₃ | S(=O)₂Me | Cl | CH₂CF₃ | O |
| C-1825 | H | CH₂CH₂ | Me | CH₂CF₃ | S(=O)₂Me | Cl | CH₂CF₃ | O |
| C-1826 | Me | CH₂CH₂ | H | CH₂CF₃ | S(=O)₂Me | Cl | CH₂CF₃ | O |
| C-1827 | H | single bond | Me | CH₂CF₃ | S(=O)₂Me | Me | CH₂CF₃ | O |
| C-1828 | Me | single bond | H | CH₂CF₃ | S(=O)₂Me | Me | CH₂CF₃ | O |
| C-1829 | Me(R) | single bond | H | CH₂CF₃ | S(=O)₂Me | Me | CH₂CF₃ | O |
| C-1830 | Me(S) | single bond | H | CH₂CF₃ | S(=O)₂Me | Me | CH₂CF₃ | O |
| C-1831 | H | CH₂ | Me | CH₂CF₃ | S(=O)₂Me | Me | CH₂CF₃ | O |
| C-1832 | Me | CH₂ | H | CH₂CF₃ | S(=O)₂Me | Me | CH₂CF₃ | O |
| C-1833 | H | CH(Me) | H | CH₂CF₃ | S(=O)₂Me | Me | CH₂CF₃ | O |
| C-1834 | H | CH₂CH₂ | Me | CH₂CF₃ | S(=O)₂Me | Me | CH₂CF₃ | O |
| C-1835 | Me | CH₂CH₂ | H | CH₂CF₃ | S(=O)₂Me | Me | CH₂CF₃ | O |
| C-1836 | H | single bond | Me | CH₂CF₃ | S(=O)₂Me | Cl | CH₂OMe | O |
| C-1837 | Me | single bond | H | CH₂CF₃ | S(=O)₂Me | Cl | CH₂OMe | O |
| C-1838 | Me(R) | single bond | H | CH₂CF₃ | S(=O)₂Me | Cl | CH₂OMe | O |
| C-1839 | Me(S) | single bond | H | CH₂CF₃ | S(=O)₂Me | Cl | CH₂OMe | O |
| C-1840 | H | CH₂ | Me | CH₂CF₃ | S(=O)₂Me | Cl | CH₂OMe | O |
| C-1841 | Me | CH₂ | H | CH₂CF₃ | S(=O)₂Me | Cl | CH₂OMe | O |
| C-1842 | H | CH(Me) | H | CH₂CF₃ | S(=O)₂Me | Cl | CH₂OMe | O |

**[Table 269]**

| Compound Number | Y | W | Z | R¹ | R² | R³ | R¹⁰ | A |
|---|---|---|---|---|---|---|---|---|
| C-1843 | H | CH₂CH₂ | Me | CH₂CF₃ | S(=O)₂Me | Cl | CH₂OMe | O |
| C-1844 | Me | CH₂CH₂ | H | CH₂CF₃ | S(=O)₂Me | Cl | CH₂OMe | O |
| C-1845 | H | single bond | Me | CH₂CF₃ | S(=O)₂Me | Me | CH₂OMe | O |
| C-1846 | Me | single bond | H | CH₂CF₃ | S(=O)₂Me | Me | CH₂OMe | O |
| C-1847 | Me(R) | single bond | H | CH₂CF₃ | S(=O)₂Me | Me | CH₂OMe | O |
| C-1848 | Me(S) | single bond | H | CH₂CF₃ | S(=O)₂Me | Me | CH₂OMe | O |
| C-1849 | H | CH₂ | Me | CH₂CF₃ | S(=O)₂Me | Me | CH₂OMe | O |
| C-1850 | Me | CH₂ | H | CH₂CF₃ | S(=O)₂Me | Me | CH₂OMe | O |
| C-1851 | H | CH(Me) | H | CH₂CF₃ | S(=O)₂Me | Me | CH₂OMe | O |
| C-1852 | H | CH₂CH₂ | Me | CH₂CF₃ | S(=O)₂Me | Me | CH₂OMe | O |
| C-1853 | Me | CH₂CH₂ | H | CH₂CF₃ | S(=O)₂Me | Me | CH₂OMe | O |
| C-1854 | H | single bond | Me | CH₂CF₃ | S(=O)₂Me | Cl | CH₂CN | O |
| C-1855 | Me | single bond | H | CH₂CF₃ | S(=O)₂Me | Cl | CH₂CN | O |
| C-1856 | Me(R) | single bond | H | CH₂CF₃ | S(=O)₂Me | Cl | CH₂CN | O |
| C-1857 | Me(S) | single bond | H | CH₂CF₃ | S(=O)₂Me | Cl | CH₂CN | O |
| C-1858 | H | CH₂ | Me | CH₂CF₃ | S(=O)₂Me | Cl | CH₂CN | O |
| C-1859 | Me | CH₂ | H | CH₂CF₃ | S(=O)₂Me | Cl | CH₂CN | O |
| C-1860 | H | CH(Me) | H | CH₂CF₃ | S(=O)₂Me | Cl | CH₂CN | O |
| C-1861 | H | CH₂CH₂ | Me | CH₂CF₃ | S(=O)₂Me | Cl | CH₂CN | O |
| C-1862 | Me | CH₂CH₂ | H | CH₂CF₃ | S(=O)₂Me | Cl | CH₂CN | O |
| C-1863 | H | single bond | Me | CH₂CF₃ | S(=O)₂Me | Me | CH₂CN | O |
| C-1864 | Me | single bond | H | CH₂CF₃ | S(=O)₂Me | Me | CH₂CN | O |
| C-1865 | Me(R) | single bond | H | CH₂CF₃ | S(=O)₂Me | Me | CH₂CN | O |
| C-1866 | Me(S) | single bond | H | CH₂CF₃ | S(=O)₂Me | Me | CH₂CN | O |
| C-1867 | H | CH₂ | Me | CH₂CF₃ | S(=O)₂Me | Me | CH₂CN | O |
| C-1868 | Me | CH₂ | H | CH₂CF₃ | S(=O)₂Me | Me | CH₂CN | O |
| C-1869 | H | CH(Me) | H | CH₂CF₃ | S(=O)₂Me | Me | CH₂CN | O |
| C-1870 | H | CH₂CH₂ | Me | CH₂CF₃ | S(=O)₂Me | Me | CH₂CN | O |
| C-1871 | Me | CH₂CH₂ | H | CH₂CF₃ | S(=O)₂Me | Me | CH₂CN | O |
| C-1872 | H | single bond | Me | CH₂CF₃ | S(=O)₂Me | Cl | C(=O)Me | O |
| C-1873 | Me | single bond | H | CH₂CF₃ | S(=O)₂Me | Cl | C(=O)Me | O |
| C-1874 | Me(R) | single bond | H | CH₂CF₃ | S(=O)₂Me | Cl | C(=O)Me | O |
| C-1875 | Me(S) | single bond | H | CH₂CF₃ | S(=O)₂Me | Cl | C(=O)Me | O |
| C-1876 | H | CH₂ | Me | CH₂CF₃ | S(=O)₂Me | Cl | C(=O)Me | O |
| C-1877 | Me | CH₂ | H | CH₂CF₃ | S(=O)₂Me | Cl | C(=O)Me | O |
| C-1878 | H | CH(Me) | H | CH₂CF₃ | S(=O)₂Me | Cl | C(=O)Me | O |
| C-1879 | H | CH₂CH₂ | Me | CH₂CF₃ | S(=O)₂Me | Cl | C(=O)Me | O |
| C-1880 | Me | CH₂CH₂ | H | CH₂CF₃ | S(=O)₂Me | Cl | C(=O)Me | O |
| C-1881 | H | single bond | Me | CH₂CF₃ | S(=O)₂Me | Me | C(=O)Me | O |
| C-1882 | Me | single bond | H | CH₂CF₃ | S(=O)₂Me | Me | C(=O)Me | O |
| C-1883 | Me(R) | single bond | H | CH₂CF₃ | S(=O)₂Me | Me | C(=O)Me | O |
| C-1884 | Me(S) | single bond | H | CH₂CF₃ | S(=O)₂Me | Me | C(=O)Me | O |
| C-1885 | H | CH₂ | Me | CH₂CF₃ | S(=O)₂Me | Me | C(=O)Me | O |
| C-1886 | Me | CH₂ | H | CH₂CF₃ | S(=O)₂Me | Me | C(=O)Me | O |

**[Table 270]**

| Compound Number | Y | W | Z | R¹ | R² | R³ | R¹⁰ | A |
|---|---|---|---|---|---|---|---|---|
| C-1887 | H | CH(Me) | H | CH₂CF₃ | S(=O)₂Me | Me | C(=O)Me | O |
| C-1888 | H | CH₂CH₂ | Me | CH₂CF₃ | S(=O)₂Me | Me | C(=O)Me | O |
| C-1889 | Me | CH₂CH₂ | H | CH₂CF₃ | S(=O)₂Me | Me | C(=O)Me | O |
| C-1890 | H | single bond | Me | CH₂CF₃ | S(=O)₂CF₃ | Cl | Me | O |
| C-1891 | H | single bond | Et | CH₂CF₃ | S(=O)₂CF₃ | Cl | Me | O |
| C-1892 | Me | single bond | H | CH₂CF₃ | S(=O)₂CF₃ | Cl | Me | O |
| C-1893 | Me(R) | single bond | H | CH₂CF₃ | S(=O)₂CF₃ | Cl | Me | O |
| C-1894 | Me(S) | single bond | H | CH₂CF₃ | S(=O)₂CF₃ | Cl | Me | O |
| C-1895 | H | CH₂ | Me | CH₂CF₃ | S(=O)₂CF₃ | Cl | Me | O |
| C-1896 | Me | CH₂ | H | CH₂CF₃ | S(=O)₂CF₃ | Cl | Me | O |
| C-1897 | H | CH(Me) | H | CH₂CF₃ | S(=O)₂CF₃ | Cl | Me | O |
| C-1898 | H | CH₂CH₂ | Me | CH₂CF₃ | S(=O)₂CF₃ | Cl | Me | O |
| C-1899 | Me | CH₂CH₂ | H | CH₂CF₃ | S(=O)₂CF₃ | Cl | Me | O |
| C-1900 | H | single bond | Me | CH₂CF₃ | S(=O)₂CF₃ | Me | Me | O |
| C-1901 | Me | single bond | H | CH₂CF₃ | S(=O)₂CF₃ | Me | Me | O |
| C-1902 | Me(R) | single bond | H | CH₂CF₃ | S(=O)₂CF₃ | Me | Me | O |
| C-1903 | Me(S) | single bond | H | CH₂CF₃ | S(=O)₂CF₃ | Me | Me | O |
| C-1904 | H | CH₂ | Me | CH₂CF₃ | S(=O)₂CF₃ | Me | Me | O |
| C-1905 | Me | CH₂ | H | CH₂CF₃ | S(=O)₂CF₃ | Me | Me | O |
| C-1906 | H | CH(Me) | H | CH₂CF₃ | S(=O)₂CF₃ | Me | Me | O |
| C-1907 | H | CH₂CH₂ | Me | CH₂CF₃ | S(=O)₂CF₃ | Me | Me | O |
| C-1908 | Me | CH₂CH₂ | H | CH₂CF₃ | S(=O)₂CF₃ | Me | Me | O |
| C-1909 | H | single bond | Me | CH₂CF₃ | S(=O)₂CF₃ | Cl | Et | O |
| C-1910 | Me | single bond | H | CH₂CF₃ | S(=O)₂CF₃ | Cl | Et | O |
| C-1911 | Me(R) | single bond | H | CH₂CF₃ | S(=O)₂CF₃ | Cl | Et | O |
| C-1912 | Me(S) | single bond | H | CH₂CF₃ | S(=O)₂CF₃ | Cl | Et | O |
| C-1913 | H | CH₂ | Me | CH₂CF₃ | S(=O)₂CF₃ | Cl | Et | O |
| C-1914 | Me | CH₂ | H | CH₂CF₃ | S(=O)₂CF₃ | Cl | Et | O |
| C-1915 | H | CH(Me) | H | CH₂CF₃ | S(=O)₂CF₃ | Cl | Et | O |
| C-1916 | H | CH₂CH₂ | Me | CH₂CF₃ | S(=O)₂CF₃ | Cl | Et | O |
| C-1917 | Me | CH₂CH₂ | H | CH₂CF₃ | S(=O)₂CF₃ | Cl | Et | O |
| C-1918 | H | single bond | Me | CH₂CF₃ | S(=O)₂CF₃ | Me | Et | O |
| C-1919 | Me | single bond | H | CH₂CF₃ | S(=O)₂CF₃ | Me | Et | O |
| C-1920 | Me(R) | single bond | H | CH₂CF₃ | S(=O)₂CF₃ | Me | Et | O |
| C-1921 | Me(S) | single bond | H | CH₂CF₃ | S(=O)₂CF₃ | Me | Et | O |
| C-1922 | H | CH₂ | Me | CH₂CF₃ | S(=O)₂CF₃ | Me | Et | O |
| C-1923 | Me | CH₂ | H | CH₂CF₃ | S(=O)₂CF₃ | Me | Et | O |
| C-1924 | H | CH(Me) | H | CH₂CF₃ | S(=O)₂CF₃ | Me | Et | O |
| C-1925 | H | CH₂CH₂ | Me | CH₂CF₃ | S(=O)₂CF₃ | Me | Et | O |
| C-1926 | Me | CH₂CH₂ | H | CH₂CF₃ | S(=O)₂CF₃ | Me | Et | O |
| C-1927 | H | single bond | Me | CH₂CF₃ | S(=O)₂CF₃ | Cl | CH₂CF₃ | O |
| C-1928 | Me | single bond | H | CH₂CF₃ | S(=O)₂CF₃ | Cl | CH₂CF₃ | O |
| C-1929 | Me(R) | single bond | H | CH₂CF₃ | S(=O)₂CF₃ | Cl | CH₂CF₃ | O |
| C-1930 | Me(S) | single bond | H | CH₂CF₃ | S(=O)₂CF₃ | Cl | CH₂CF₃ | O |

**[Table 271]**

| Compound Number | Y | W | Z | R¹ | R² | R³ | R¹⁰ | A |
|---|---|---|---|---|---|---|---|---|
| C-1931 | H | CH₂ | Me | CH₂CF₃ | S(=O)₂CF₃ | Cl | CH₂CF₃ | O |
| C-1932 | Me | CH₂ | H | CH₂CF₃ | S(=O)₂CF₃ | Cl | CH₂CF₃ | O |
| C-1933 | H | CH(Me) | H | CH₂CF₃ | S(=O)₂CF₃ | Cl | CH₂CF₃ | O |
| C-1934 | H | CH₂CH₂ | Me | CH₂CF₃ | S(=O)₂CF₃ | Cl | CH₂CF₃ | O |
| C-1935 | Me | CH₂CH₂ | H | CH₂CF₃ | S(=O)₂CF₃ | Cl | CH₂CF₃ | O |
| C-1936 | H | single bond | Me | CH₂CF₃ | S(=O)₂CF₃ | Me | CH₂CF₃ | O |
| C-1937 | Me | single bond | H | CH₂CF₃ | S(=O)₂CF₃ | Me | CH₂CF₃ | O |
| C-1938 | Me(R) | single bond | H | CH₂CF₃ | S(=O)₂CF₃ | Me | CH₂CF₃ | O |
| C-1939 | Me(S) | single bond | H | CH₂CF₃ | S(=O)₂CF₃ | Me | CH₂CF₃ | O |
| C-1940 | H | CH₂ | Me | CH₂CF₃ | S(=O)₂CF₃ | Me | CH₂CF₃ | O |
| C-1941 | Me | CH₂ | H | CH₂CF₃ | S(=O)₂CF₃ | Me | CH₂CF₃ | O |
| C-1942 | H | CH(Me) | H | CH₂CF₃ | S(=O)₂CF₃ | Me | CH₂CF₃ | O |
| C-1943 | H | CH₂CH₂ | Me | CH₂CF₃ | S(=O)₂CF₃ | Me | CH₂CF₃ | O |
| C-1944 | Me | CH₂CH₂ | H | CH₂CF₃ | S(=O)₂CF₃ | Me | CH₂CF₃ | O |
| C-1945 | H | single bond | Me | CH₂CF₃ | S(=O)₂CF₃ | Cl | CH₂OMe | O |
| C-1946 | Me | single bond | H | CH₂CF₃ | S(=O)₂CF₃ | Cl | CH₂OMe | O |
| C-1947 | Me(R) | single bond | H | CH₂CF₃ | S(=O)₂CF₃ | Cl | CH₂OMe | O |
| C-1948 | Me(S) | single bond | H | CH₂CF₃ | S(=O)₂CF₃ | Cl | CH₂OMe | O |
| C-1949 | H | CH₂ | Me | CH₂CF₃ | S(=O)₂CF₃ | Cl | CH₂OMe | O |
| C-1950 | Me | CH₂ | H | CH₂CF₃ | S(=O)₂CF₃ | Cl | CH₂OMe | O |
| C-1951 | H | CH(Me) | H | CH₂CF₃ | S(=O)₂CF₃ | Cl | CH₂OMe | O |
| C-1952 | H | CH₂CH₂ | Me | CH₂CF₃ | S(=O)₂CF₃ | Cl | CH₂OMe | O |
| C-1953 | Me | CH₂CH₂ | H | CH₂CF₃ | S(=O)₂CF₃ | Cl | CH₂OMe | O |
| C-1954 | H | single bond | Me | CH₂CF₃ | S(=O)₂CF₃ | Me | CH₂OMe | O |
| C-1955 | Me | single bond | H | CH₂CF₃ | S(=O)₂CF₃ | Me | CH₂OMe | O |
| C-1956 | Me(R) | single bond | H | CH₂CF₃ | S(=O)₂CF₃ | Me | CH₂OMe | O |
| C-1957 | Me(S) | single bond | H | CH₂CF₃ | S(=O)₂CF₃ | Me | CH₂OMe | O |
| C-1958 | H | CH₂ | Me | CH₂CF₃ | S(=O)₂CF₃ | Me | CH₂OMe | O |
| C-1959 | Me | CH₂ | H | CH₂CF₃ | S(=O)₂CF₃ | Me | CH₂OMe | O |
| C-1960 | H | CH(Me) | H | CH₂CF₃ | S(=O)₂CF₃ | Me | CH₂OMe | O |
| C-1961 | H | CH₂CH₂ | Me | CH₂CF₃ | S(=O)₂CF₃ | Me | CH₂OMe | O |
| C-1962 | Me | CH₂CH₂ | H | CH₂CF₃ | S(=O)₂CF₃ | Me | CH₂OMe | O |
| C-1963 | H | single bond | Me | CH₂CF₃ | S(=O)₂CF₃ | Cl | CH₂CN | O |
| C-1964 | Me | single bond | H | CH₂CF₃ | S(=O)₂CF₃ | Cl | CH₂CN | O |
| C-1965 | Me(R) | single bond | H | CH₂CF₃ | S(=O)₂CF₃ | Cl | CH₂CN | O |
| C-1966 | Me(S) | single bond | H | CH₂CF₃ | S(=O)₂CF₃ | Cl | CH₂CN | O |
| C-1967 | H | CH₂ | Me | CH₂CF₃ | S(=O)₂CF₃ | Cl | CH₂CN | O |
| C-1968 | Me | CH₂ | H | CH₂CF₃ | S(=O)₂CF₃ | Cl | CH₂CN | O |
| C-1969 | H | CH(Me) | H | CH₂CF₃ | S(=O)₂CF₃ | Cl | CH₂CN | O |
| C-1970 | H | CH₂CH₂ | Me | CH₂CF₃ | S(=O)₂CF₃ | Cl | CH₂CN | O |
| C-1971 | Me | CH₂CH₂ | H | CH₂CF₃ | S(=O)₂CF₃ | Cl | CH₂CN | O |
| C-1972 | H | single bond | Me | CH₂CF₃ | S(=O)₂CF₃ | Me | CH₂CN | O |
| C-1973 | Me | single bond | H | CH₂CF₃ | S(=O)₂CF₃ | Me | CH₂CN | O |
| C-1974 | Me(R) | single bond | H | CH₂CF₃ | S(=O)₂CF₃ | Me | CH₂CN | O |

**[Table 272]**

| Compound Number | Y | W | Z | R¹ | R² | R³ | R¹⁰ | A |
|---|---|---|---|---|---|---|---|---|
| C-1975 | Me(S) | single bond | H | CH₂CF₃ | S(=O)₂CF₃ | Me | CH₂CN | O |
| C-1976 | H | CH₂ | Me | CH₂CF₃ | S(=O)₂CF₃ | Me | CH₂CN | O |
| C-1977 | Me | CH₂ | H | CH₂CF₃ | S(=O)₂CF₃ | Me | CH₂CN | O |
| C-1978 | H | CH(Me) | H | CH₂CF₃ | S(=O)₂CF₃ | Me | CH₂CN | O |
| C-1979 | H | CH₂CH₂ | Me | CH₂CF₃ | S(=O)₂CF₃ | Me | CH₂CN | O |
| C-1980 | Me | CH₂CH₂ | H | CH₂CF₃ | S(=O)₂CF₃ | Me | CH₂CN | O |
| C-1981 | H | single bond | Me | CH₂CF₃ | S(=O)₂CF₃ | Cl | C(=O)Me | O |
| C-1982 | Me | single bond | H | CH₂CF₃ | S(=O)₂CF₃ | Cl | C(=O)Me | O |
| C-1983 | Me(R) | single bond | H | CH₂CF₃ | S(=O)₂CF₃ | Cl | C(=O)Me | O |
| C-1984 | Me(S) | single bond | H | CH₂CF₃ | S(=O)₂CF₃ | Cl | C(=O)Me | O |
| C-1985 | H | CH₂ | Me | CH₂CF₃ | S(=O)₂CF₃ | Cl | C(=O)Me | O |
| C-1986 | Me | CH₂ | H | CH₂CF₃ | S(=O)₂CF₃ | Cl | C(=O)Me | O |
| C-1987 | H | CH(Me) | H | CH₂CF₃ | S(=O)₂CF₃ | Cl | C(=O)Me | O |
| C-1988 | H | CH₂CH₂ | Me | CH₂CF₃ | S(=O)₂CF₃ | Cl | C(=O)Me | O |
| C-1989 | Me | CH₂CH₂ | H | CH₂CF₃ | S(=O)₂CF₃ | Cl | C(=O)Me | O |
| C-1990 | H | single bond | Me | CH₂CF₃ | S(=O)₂CF₃ | Me | C(=O)Me | O |
| C-1991 | Me | single bond | H | CH₂CF₃ | S(=O)₂CF₃ | Me | C(=O)Me | O |
| C-1992 | Me(R) | single bond | H | CH₂CF₃ | S(=O)₂CF₃ | Me | C(=O)Me | O |
| C-1993 | Me(S) | single bond | H | CH₂CF₃ | S(=O)₂CF₃ | Me | C(=O)Me | O |
| C-1994 | H | CH₂ | Me | CH₂CF₃ | S(=O)₂CF₃ | Me | C(=O)Me | O |
| C-1995 | Me | CH₂ | H | CH₂CF₃ | S(=O)₂CF₃ | Me | C(=O)Me | O |
| C-1996 | H | CH(Me) | H | CH₂CF₃ | S(=O)₂CF₃ | Me | C(=O)Me | O |
| C-1997 | H | CH₂CH₂ | Me | CH₂CF₃ | S(=O)₂CF₃ | Me | C(=O)Me | O |
| C-1998 | Me | CH₂CH₂ | H | CH₂CF₃ | S(=O)₂CF₃ | Me | C(=O)Me | O |
| C-1999 | H | single bond | Me | *c*-Pr | Me | Cl | Me | O |
| C-2000 | H | single bond | Et | *c*-Pr | Me | Cl | Me | O |
| C-2001 | Me | single bond | H | *c*-Pr | Me | Cl | Me | O |
| C-2002 | Me(R) | single bond | H | *c*-Pr | Me | Cl | Me | O |
| C-2003 | Me(S) | single bond | H | *c*-Pr | Me | Cl | Me | O |
| C-2004 | H | CH₂ | Me | *c*-Pr | Me | Cl | Me | O |
| C-2005 | Me | CH₂ | H | *c*-Pr | Me | Cl | Me | O |
| C-2006 | H | CH(Me) | H | *c*-Pr | Me | Cl | Me | O |
| C-2007 | H | CH₂CH₂ | Me | *c*-Pr | Me | Cl | Me | O |
| C-2008 | Me | CH₂CH₂ | H | *c*-Pr | Me | Cl | Me | O |
| C-2009 | H | single bond | Me | *c*-Pr | Me | Me | Me | O |
| C-2010 | Me | single bond | H | *c*-Pr | Me | Me | Me | O |
| C-2011 | Me(R) | single bond | H | *c*-Pr | Me | Me | Me | O |
| C-2012 | Me(S) | single bond | H | *c*-Pr | Me | Me | Me | O |
| C-2013 | H | CH₂ | Me | *c*-Pr | Me | Me | Me | O |
| C-2014 | Me | CH₂ | H | *c*-Pr | Me | Me | Me | O |
| C-2015 | H | CH(Me) | H | *c*-Pr | Me | Me | Me | O |
| C-2016 | H | CH₂CH₂ | Me | *c*-Pr | Me | Me | Me | O |
| C-2017 | Me | CH₂CH₂ | H | *c*-Pr | Me | Me | Me | O |
| C-2018 | H | single bond | Me | *c*-Pr | Me | Cl | Et | O |

**[Table 273]**

| Compound Number | Y | W | Z | R¹ | R² | R³ | R¹⁰ | A |
|---|---|---|---|---|---|---|---|---|
| C-2019 | Me | single bond | H | *c*-Pr | Me | Cl | Et | O |
| C-2020 | Me(R) | single bond | H | *c*-Pr | Me | Cl | Et | O |
| C-2021 | Me(S) | single bond | H | *c*-Pr | Me | Cl | Et | O |
| C-2022 | H | CH₂ | Me | *c*-Pr | Me | Cl | Et | O |
| C-2023 | Me | CH₂ | H | *c*-Pr | Me | Cl | Et | O |
| C-2024 | H | CH(Me) | H | *c*-Pr | Me | Cl | Et | O |
| C-2025 | H | CH₂CH₂ | Me | *c*-Pr | Me | Cl | Et | O |
| C-2026 | Me | CH₂CH₂ | H | *c*-Pr | Me | Cl | Et | O |
| C-2027 | H | single bond | Me | *c*-Pr | Me | Me | Et | O |
| C-2028 | Me | single bond | H | *c*-Pr | Me | Me | Et | O |
| C-2029 | Me(R) | single bond | H | *c*-Pr | Me | Me | Et | O |
| C-2030 | Me(S) | single bond | H | *c*-Pr | Me | Me | Et | O |
| C-2031 | H | CH₂ | Me | *c*-Pr | Me | Me | Et | O |
| C-2032 | Me | CH₂ | H | *c*-Pr | Me | Me | Et | O |
| C-2033 | H | CH(Me) | H | *c*-Pr | Me | Me | Et | O |
| C-2034 | H | CH₂CH₂ | Me | *c*-Pr | Me | Me | Et | O |
| C-2035 | Me | CH₂CH₂ | H | *c*-Pr | Me | Me | Et | O |
| C-2036 | H | single bond | Me | *c*-Pr | Me | Cl | CH₂CF₃ | O |
| C-2037 | Me | single bond | H | *c*-Pr | Me | Cl | CH₂CF₃ | O |
| C-2038 | Me(R) | single bond | H | *c*-Pr | Me | Cl | CH₂CF₃ | O |
| C-2039 | Me(S) | single bond | H | *c*-Pr | Me | Cl | CH₂CF₃ | O |
| C-2040 | H | CH₂ | Me | *c*-Pr | Me | Cl | CH₂CF₃ | O |
| C-2041 | Me | CH₂ | H | *c*-Pr | Me | Cl | CH₂CF₃ | O |
| C-2042 | H | CH(Me) | H | *c*-Pr | Me | Cl | CH₂CF₃ | O |
| C-2043 | H | CH₂CH₂ | Me | *c*-Pr | Me | Cl | CH₂CF₃ | O |
| C-2044 | Me | CH₂CH₂ | H | *c*-Pr | Me | Cl | CH₂CF₃ | O |
| C-2045 | H | single bond | Me | *c*-Pr | Me | Me | CH₂CF₃ | O |
| C-2046 | Me | single bond | H | *c*-Pr | Me | Me | CH₂CF₃ | O |
| C-2047 | Me(R) | single bond | H | *c*-Pr | Me | Me | CH₂CF₃ | O |
| C-2048 | Me(S) | single bond | H | *c*-Pr | Me | Me | CH₂CF₃ | O |
| C-2049 | H | CH₂ | Me | *c*-Pr | Me | Me | CH₂CF₃ | O |
| C-2050 | Me | CH₂ | H | *c*-Pr | Me | Me | CH₂CF₃ | O |
| C-2051 | H | CH(Me) | H | *c*-Pr | Me | Me | CH₂CF₃ | O |
| C-2052 | H | CH₂CH₂ | Me | *c*-Pr | Me | Me | CH₂CF₃ | O |
| C-2053 | Me | CH₂CH₂ | H | *c*-Pr | Me | Me | CH₂CF₃ | O |
| C-2054 | H | single bond | Me | *c*-Pr | Me | Cl | CH₂OMe | O |
| C-2055 | Me | single bond | H | *c*-Pr | Me | Cl | CH₂OMe | O |
| C-2056 | Me(R) | single bond | H | *c*-Pr | Me | Cl | CH₂OMe | O |
| C-2057 | Me(S) | single bond | H | *c*-Pr | Me | Cl | CH₂OMe | O |
| C-2058 | H | CH₂ | Me | *c*-Pr | Me | Cl | CH₂OMe | O |
| C-2059 | Me | CH₂ | H | *c*-Pr | Me | Cl | CH₂OMe | O |
| C-2060 | H | CH(Me) | H | *c*-Pr | Me | Cl | CH₂OMe | O |
| C-2061 | H | CH₂CH₂ | Me | *c*-Pr | Me | Cl | CH₂OMe | O |
| C-2062 | Me | CH₂CH₂ | H | *c*-Pr | Me | Cl | CH₂OMe | O |

**[Table 274]**

| Compound Number | Y | W | Z | R¹ | R² | R³ | R¹⁰ | A |
|---|---|---|---|---|---|---|---|---|
| C-2063 | H | single bond | Me | *c*-Pr | Me | Me | CH₂OMe | O |
| C-2064 | Me | single bond | H | *c*-Pr | Me | Me | CH₂OMe | O |
| C-2065 | Me(R) | single bond | H | *c*-Pr | Me | Me | CH₂OMe | O |
| C-2066 | Me(S) | single bond | H | *c*-Pr | Me | Me | CH₂OMe | O |
| C-2067 | H | CH₂ | Me | *c*-Pr | Me | Me | CH₂OMe | O |
| C-2068 | Me | CH₂ | H | *c*-Pr | Me | Me | CH₂OMe | O |
| C-2069 | H | CH(Me) | H | *c*-Pr | Me | Me | CH₂OMe | O |
| C-2070 | H | CH₂CH₂ | Me | *c*-Pr | Me | Me | CH₂OMe | O |
| C-2071 | Me | CH₂CH₂ | H | *c*-Pr | Me | Me | CH₂OMe | O |
| C-2072 | H | single bond | Me | *c*-Pr | Me | Cl | CH₂CN | O |
| C-2073 | Me | single bond | H | *c*-Pr | Me | Cl | CH₂CN | O |
| C-2074 | Me(R) | single bond | H | *c*-Pr | Me | Cl | CH₂CN | O |
| C-2075 | Me(S) | single bond | H | *c*-Pr | Me | Cl | CH₂CN | O |
| C-2076 | H | CH₂ | Me | *c*-Pr | Me | Cl | CH₂CN | O |
| C-2077 | Me | CH₂ | H | *c*-Pr | Me | Cl | CH₂CN | O |
| C-2078 | H | CH(Me) | H | *c*-Pr | Me | Cl | CH₂CN | O |
| C-2079 | H | CH₂CH₂ | Me | *c*-Pr | Me | Cl | CH₂CN | O |
| C-2080 | Me | CH₂CH₂ | H | *c*-Pr | Me | Cl | CH₂CN | O |
| C-2081 | H | single bond | Me | *c*-Pr | Me | Me | CH₂CN | O |
| C-2082 | Me | single bond | H | *c*-Pr | Me | Me | CH₂CN | O |
| C-2083 | Me(R) | single bond | H | *c*-Pr | Me | Me | CH₂CN | O |
| C-2084 | Me(S) | single bond | H | *c*-Pr | Me | Me | CH₂CN | O |
| C-2085 | H | CH₂ | Me | *c*-Pr | Me | Me | CH₂CN | O |
| C-2086 | Me | CH₂ | H | *c*-Pr | Me | Me | CH₂CN | O |
| C-2087 | H | CH(Me) | H | *c*-Pr | Me | Me | CH₂CN | O |
| C-2088 | H | CH₂CH₂ | Me | *c*-Pr | Me | Me | CH₂CN | O |
| C-2089 | Me | CH₂CH₂ | H | *c*-Pr | Me | Me | CH₂CN | O |
| C-2090 | H | single bond | Me | *c*-Pr | Me | Cl | C(=O)Me | O |
| C-2091 | Me | single bond | H | *c*-Pr | Me | Cl | C(=O)Me | O |
| C-2092 | Me(R) | single bond | H | *c*-Pr | Me | Cl | C(=O)Me | O |
| C-2093 | Me(S) | single bond | H | *c*-Pr | Me | Cl | C(=O)Me | O |
| C-2094 | H | CH₂ | Me | *c*-Pr | Me | Cl | C(=O)Me | O |
| C-2095 | Me | CH₂ | H | *c*-Pr | Me | Cl | C(=O)Me | O |
| C-2096 | H | CH(Me) | H | *c*-Pr | Me | Cl | C(=O)Me | O |
| C-2097 | H | CH₂CH₂ | Me | *c*-Pr | Me | Cl | C(=O)Me | O |
| C-2098 | Me | CH₂CH₂ | H | *c*-Pr | Me | Cl | C(=O)Me | O |
| C-2099 | H | single bond | Me | *c*-Pr | Me | Me | C(=O)Me | O |
| C-2100 | Me | single bond | H | *c*-Pr | Me | Me | C(=O)Me | O |
| C-2101 | Me(R) | single bond | H | *c*-Pr | Me | Me | C(=O)Me | O |
| C-2102 | Me(S) | single bond | H | *c*-Pr | Me | Me | C(=O)Me | O |
| C-2103 | H | CH₂ | Me | *c*-Pr | Me | Me | C(=O)Me | O |
| C-2104 | Me | CH₂ | H | *c*-Pr | Me | Me | C(=O)Me | O |
| C-2105 | H | CH(Me) | H | *c*-Pr | Me | Me | C(=O)Me | O |
| C-2106 | H | CH₂CH₂ | Me | *c*-Pr | Me | Me | C(=O)Me | O |

**[Table 275]**

| Compound Number | Y | W | Z | R¹ | R² | R³ | R¹⁰ | A |
|---|---|---|---|---|---|---|---|---|
| C-2107 | Me | CH₂CH₂ | H | *c*-Pr | Me | Me | C(=O)Me | O |
| C-2108 | H | single bond | Me | *c*-Pr | CH₂OMe | Cl | Me | O |
| C-2109 | H | single bond | Et | *c*-Pr | CH₂OMe | Cl | Me | O |
| C-2110 | Me | single bond | H | *c*-Pr | CH₂OMe | Cl | Me | O |
| C-2111 | Me(R) | single bond | H | *c*-Pr | CH₂OMe | Cl | Me | O |
| C-2112 | Me(S) | single bond | H | *c*-Pr | CH₂OMe | Cl | Me | O |
| C-2113 | H | CH₂ | Me | *c*-Pr | CH₂OMe | Cl | Me | O |
| C-2114 | Me | CH₂ | H | *c*-Pr | CH₂OMe | Cl | Me | O |
| C-2115 | H | CH(Me) | H | *c*-Pr | CH₂OMe | Cl | Me | O |
| C-2116 | H | CH₂CH₂ | Me | *c*-Pr | CH₂OMe | Cl | Me | O |
| C-2117 | Me | CH₂CH₂ | H | *c*-Pr | CH₂OMe | Cl | Me | O |
| C-2118 | H | single bond | Me | *c*-Pr | CH₂OMe | Me | Me | O |
| C-2119 | Me | single bond | H | *c*-Pr | CH₂OMe | Me | Me | O |
| C-2120 | Me(R) | single bond | H | *c*-Pr | CH₂OMe | Me | Me | O |
| C-2121 | Me(S) | single bond | H | *c*-Pr | CH₂OMe | Me | Me | O |
| C-2122 | H | CH₂ | Me | *c*-Pr | CH₂OMe | Me | Me | O |
| C-2123 | Me | CH₂ | H | *c*-Pr | CH₂OMe | Me | Me | O |
| C-2124 | H | CH(Me) | H | *c*-Pr | CH₂OMe | Me | Me | O |
| C-2125 | H | CH₂CH₂ | Me | *c*-Pr | CH₂OMe | Me | Me | O |
| C-2126 | Me | CH₂CH₂ | H | *c*-Pr | CH₂OMe | Me | Me | O |
| C-2127 | H | single bond | Me | *c*-Pr | CH₂OMe | Cl | Et | O |
| C-2128 | Me | single bond | H | *c*-Pr | CH₂OMe | Cl | Et | O |
| C-2129 | Me(R) | single bond | H | *c*-Pr | CH₂OMe | Cl | Et | O |
| C-2130 | Me(S) | single bond | H | *c*-Pr | CH₂OMe | Cl | Et | O |
| C-2131 | H | CH₂ | Me | *c*-Pr | CH₂OMe | Cl | Et | O |
| C-2132 | Me | CH₂ | H | c-Pr | CH₂OMe | Cl | Et | O |
| C-2133 | H | CH(Me) | H | c-Pr | CH₂OMe | Cl | Et | O |
| C-2134 | H | CH₂CH₂ | Me | c-Pr | CH₂OMe | Cl | Et | O |
| C-2135 | Me | CH₂CH₂ | H | *c*-Pr | CH₂OMe | Cl | Et | O |
| C-2136 | H | single bond | Me | *c*-Pr | CH₂OMe | Me | Et | O |
| C-2137 | Me | single bond | H | *c*-Pr | CH₂OMe | Me | Et | O |
| C-2138 | Me(R) | single bond | H | *c*-Pr | CH₂OMe | Me | Et | O |
| C-2139 | Me(S) | single bond | H | *c*-Pr | CH₂OMe | Me | Et | O |
| C-2140 | H | CH₂ | Me | *c*-Pr | CH₂OMe | Me | Et | O |
| C-2141 | Me | CH₂ | H | *c*-Pr | CH₂OMe | Me | Et | O |
| C-2142 | H | CH(Me) | H | *c*-Pr | CH₂OMe | Me | Et | O |
| C-2143 | H | CH₂CH₂ | Me | *c*-Pr | CH₂OMe | Me | Et | O |
| C-2144 | Me | CH₂CH₂ | H | *c*-Pr | CH₂OMe | Me | Et | O |
| C-2145 | H | single bond | Me | *c*-Pr | CH₂OMe | Cl | CH₂CF₃ | O |
| C-2146 | Me | single bond | H | *c*-Pr | CH₂OMe | Cl | CH₂CF₃ | O |
| C-2147 | Me(R) | single bond | H | *c*-Pr | CH₂OMe | Cl | CH₂CF₃ | O |
| C-2148 | Me(S) | single bond | H | *c*-Pr | CH₂OMe | Cl | CH₂CF₃ | O |
| C-2149 | H | CH₂ | Me | *c*-Pr | CH₂OMe | Cl | CH₂CF₃ | O |
| C-2150 | Me | CH₂ | H | *c*-Pr | CH₂OMe | Cl | CH₂CF₃ | O |

**[Table 276]**

| Compound Number | Y | W | Z | R¹ | R² | R³ | R¹⁰ | A |
|---|---|---|---|---|---|---|---|---|
| C-2151 | H | CH(Me) | H | *c*-Pr | CH₂OMe | Cl | CH₂CF₃ | O |
| C-2152 | H | CH₂CH₂ | Me | *c*-Pr | CH₂OMe | Cl | CH₂CF₃ | O |
| C-2153 | Me | CH₂CH₂ | H | *c*-Pr | CH₂OMe | Cl | CH₂CF₃ | O |
| C-2154 | H | single bond | Me | *c*-Pr | CH₂OMe | Me | CH₂CF₃ | O |
| C-2155 | Me | single bond | H | *c*-Pr | CH₂OMe | Me | CH₂CF₃ | O |
| C-2156 | Me(R) | single bond | H | *c*-Pr | CH₂OMe | Me | CH₂CF₃ | O |
| C-2157 | Me(S) | single bond | H | *c*-Pr | CH₂OMe | Me | CH₂CF₃ | O |
| C-2158 | H | CH₂ | Me | *c*-Pr | CH₂OMe | Me | CH₂CF₃ | O |
| C-2159 | Me | CH₂ | H | *c*-Pr | CH₂OMe | Me | CH₂CF₃ | O |
| C-2160 | H | CH(Me) | H | *c*-Pr | CH₂OMe | Me | CH₂CF₃ | O |
| C-2161 | H | CH₂CH₂ | Me | *c*-Pr | CH₂OMe | Me | CH₂CF₃ | O |
| C-2162 | Me | CH₂CH₂ | H | *c*-Pr | CH₂OMe | Me | CH₂CF₃ | O |
| C-2163 | H | single bond | Me | *c*-Pr | CH₂OMe | Cl | CH₂OMe | O |
| C-2164 | Me | single bond | H | *c*-Pr | CH₂OMe | Cl | CH₂OMe | O |
| C-2165 | Me(R) | single bond | H | *c*-Pr | CH₂OMe | Cl | CH₂OMe | O |
| C-2166 | Me(S) | single bond | H | *c*-Pr | CH₂OMe | Cl | CH₂OMe | O |
| C-2167 | H | CH₂ | Me | *c*-Pr | CH₂OMe | Cl | CH₂OMe | O |
| C-2168 | Me | CH₂ | H | *c*-Pr | CH₂OMe | Cl | CH₂OMe | O |
| C-2169 | H | CH(Me) | H | *c*-Pr | CH₂OMe | Cl | CH₂OMe | O |
| C-2170 | H | CH₂CH₂ | Me | *c*-Pr | CH₂OMe | Cl | CH₂OMe | O |
| C-2171 | Me | CH₂CH₂ | H | *c*-Pr | CH₂OMe | Cl | CH₂OMe | O |
| C-2172 | H | single bond | Me | *c*-Pr | CH₂OMe | Me | CH₂OMe | O |
| C-2173 | Me | single bond | H | *c*-Pr | CH₂OMe | Me | CH₂OMe | O |
| C-2174 | Me(R) | single bond | H | *c*-Pr | CH₂OMe | Me | CH₂OMe | O |
| C-2175 | Me(S) | single bond | H | *c*-Pr | CH₂OMe | Me | CH₂OMe | O |
| C-2176 | H | CH₂ | Me | *c*-Pr | CH₂OMe | Me | CH₂OMe | O |
| C-2177 | Me | CH₂ | H | *c*-Pr | CH₂OMe | Me | CH₂OMe | O |
| C-2178 | H | CH(Me) | H | *c*-Pr | CH₂OMe | Me | CH₂OMe | O |
| C-2179 | H | CH₂CH₂ | Me | *c*-Pr | CH₂OMe | Me | CH₂OMe | O |
| C-2180 | Me | CH₂CH₂ | H | *c*-Pr | CH₂OMe | Me | CH₂OMe | O |
| C-2181 | H | single bond | Me | *c*-Pr | CH₂OMe | Cl | CH₂CN | O |
| C-2182 | Me | single bond | H | *c*-Pr | CH₂OMe | Cl | CH₂CN | O |
| C-2183 | Me(R) | single bond | H | *c*-Pr | CH₂OMe | Cl | CH₂CN | O |
| C-2184 | Me(S) | single bond | H | *c*-Pr | CH₂OMe | Cl | CH₂CN | O |
| C-2185 | H | CH₂ | Me | *c*-Pr | CH₂OMe | Cl | CH₂CN | O |
| C-2186 | Me | CH₂ | H | *c*-Pr | CH₂OMe | Cl | CH₂CN | O |
| C-2187 | H | CH(Me) | H | *c*-Pr | CH₂OMe | Cl | CH₂CN | O |
| C-2188 | H | CH₂CH₂ | Me | *c*-Pr | CH₂OMe | Cl | CH₂CN | O |
| C-2189 | Me | CH₂CH₂ | H | *c*-Pr | CH₂OMe | Cl | CH₂CN | O |
| C-2190 | H | single bond | Me | *c*-Pr | CH₂OMe | Me | CH₂CN | O |
| C-2191 | Me | single bond | H | *c*-Pr | CH₂OMe | Me | CH₂CN | O |
| C-2192 | Me(R) | single bond | H | *c*-Pr | CH₂OMe | Me | CH₂CN | O |
| C-2193 | Me(S) | single bond | H | *c*-Pr | CH₂OMe | Me | CH₂CN | O |
| C-2194 | H | CH₂ | Me | *c*-Pr | CH₂OMe | Me | CH₂CN | O |

**[Table 277]**

| Compound Number | Y | W | Z | R¹ | R² | R³ | R¹⁰ | A |
|---|---|---|---|---|---|---|---|---|
| C-2195 | Me | CH₂ | H | *c*-Pr | CH₂OMe | Me | CH₂CN | O |
| C-2196 | H | CH(Me) | H | *c*-Pr | CH₂OMe | Me | CH₂CN | O |
| C-2197 | H | CH₂CH₂ | Me | *c*-Pr | CH₂OMe | Me | CH₂CN | O |
| C-2198 | Me | CH₂CH₂ | H | *c*-Pr | CH₂OMe | Me | CH₂CN | O |
| C-2199 | H | single bond | Me | *c*-Pr | CH₂OMe | Cl | C(=O)Me | O |
| C-2200 | Me | single bond | H | *c*-Pr | CH₂OMe | Cl | C(=O)Me | O |
| C-2201 | Me(R) | single bond | H | *c*-Pr | CH₂OMe | Cl | C(=O)Me | O |
| C-2202 | Me(S) | single bond | H | *c*-Pr | CH₂OMe | Cl | C(=O)Me | O |
| C-2203 | H | CH₂ | Me | *c*-Pr | CH₂OMe | Cl | C(=O)Me | O |
| C-2204 | Me | CH₂ | H | *c*-Pr | CH₂OMe | Cl | C(=O)Me | O |
| C-2205 | H | CH(Me) | H | *c*-Pr | CH₂OMe | Cl | C(=O)Me | O |
| C-2206 | H | CH₂CH₂ | Me | *c*-Pr | CH₂OMe | Cl | C(=O)Me | O |
| C-2207 | Me | CH₂CH₂ | H | *c*-Pr | CH₂OMe | Cl | C(=O)Me | O |
| C-2208 | H | single bond | Me | *c*-Pr | CH₂OMe | Me | C(=O)Me | O |
| C-2209 | Me | single bond | H | *c*-Pr | CH₂OMe | Me | C(=O)Me | O |
| C-2210 | Me(R) | single bond | H | *c*-Pr | CH₂OMe | Me | C(=O)Me | O |
| C-2211 | Me(S) | single bond | H | *c*-Pr | CH₂OMe | Me | C(=O)Me | O |
| C-2212 | H | CH₂ | Me | *c*-Pr | CH₂OMe | Me | C(=O)Me | O |
| C-2213 | Me | CH₂ | H | *c*-Pr | CH₂OMe | Me | C(=O)Me | O |
| C-2214 | H | CH(Me) | H | *c*-Pr | CH₂OMe | Me | C(=O)Me | O |
| C-2215 | H | CH₂CH₂ | Me | *c*-Pr | CH₂OMe | Me | C(=O)Me | O |
| C-2216 | Me | CH₂CH₂ | H | *c*-Pr | CH₂OMe | Me | C(=O)Me | O |
| C-2217 | H | single bond | Me | *c*-Pr | CH₂CN | Cl | Me | O |
| C-2218 | H | single bond | Et | *c*-Pr | CH₂CN | Cl | Me | O |
| C-2219 | Me | single bond | H | *c*-Pr | CH₂CN | Cl | Me | O |
| C-2220 | Me(R) | single bond | H | *c*-Pr | CH₂CN | Cl | Me | O |
| C-2221 | Me(S) | single bond | H | *c*-Pr | CH₂CN | Cl | Me | O |
| C-2222 | H | CH₂ | Me | *c*-Pr | CH₂CN | Cl | Me | O |
| C-2223 | Me | CH₂ | H | *c*-Pr | CH₂CN | Cl | Me | O |
| C-2224 | H | CH(Me) | H | *c*-Pr | CH₂CN | Cl | Me | O |
| C-2225 | H | CH₂CH₂ | Me | *c*-Pr | CH₂CN | Cl | Me | O |
| C-2226 | Me | CH₂CH₂ | H | *c*-Pr | CH₂CN | Cl | Me | O |
| C-2227 | H | single bond | Me | *c*-Pr | CH₂CN | Me | Me | O |
| C-2228 | Me | single bond | H | *c*-Pr | CH₂CN | Me | Me | O |
| C-2229 | Me(R) | single bond | H | *c*-Pr | CH₂CN | Me | Me | O |
| C-2230 | Me(S) | single bond | H | *c*-Pr | CH₂CN | Me | Me | O |
| C-2231 | H | CH₂ | Me | *c*-Pr | CH₂CN | Me | Me | O |
| C-2232 | Me | CH₂ | H | *c*-Pr | CH₂CN | Me | Me | O |
| C-2233 | H | CH(Me) | H | *c*-Pr | CH₂CN | Me | Me | O |
| C-2234 | H | CH₂CH₂ | Me | *c*-Pr | CH₂CN | Me | Me | O |
| C-2235 | Me | CH₂CH₂ | H | *c*-Pr | CH₂CN | Me | Me | O |
| C-2236 | H | single bond | Me | *c*-Pr | CH₂CN | Cl | Et | O |
| C-2237 | Me | single bond | H | *c*-Pr | CH₂CN | Cl | Et | O |
| C-2238 | Me(R) | single bond | H | *c*-Pr | CH₂CN | Cl | Et | O |

**[Table 278]**

| Compound Number | Y | W | Z | R¹ | R² | R³ | R¹⁰ | A |
|---|---|---|---|---|---|---|---|---|
| C-2239 | Me(S) | single bond | H | *c*-Pr | CH₂CN | Cl | Et | O |
| C-2240 | H | CH₂ | Me | *c*-Pr | CH₂CN | Cl | Et | O |
| C-2241 | Me | CH₂ | H | *c*-Pr | CH₂CN | Cl | Et | O |
| C-2242 | H | CH(Me) | H | *c*-Pr | CH₂CN | Cl | Et | O |
| C-2243 | H | CH₂CH₂ | Me | *c*-Pr | CH₂CN | Cl | Et | O |
| C-2244 | Me | CH₂CH₂ | H | *c*-Pr | CH₂CN | Cl | Et | O |
| C-2245 | H | single bond | Me | *c*-Pr | CH₂CN | Me | Et | O |
| C-2246 | Me | single bond | H | *c*-Pr | CH₂CN | Me | Et | O |
| C-2247 | Me(R) | single bond | H | *c*-Pr | CH₂CN | Me | Et | O |
| C-2248 | Me(S) | single bond | H | *c*-Pr | CH₂CN | Me | Et | O |
| C-2249 | H | CH₂ | Me | *c*-Pr | CH₂CN | Me | Et | O |
| C-2250 | Me | CH₂ | H | *c*-Pr | CH₂CN | Me | Et | O |
| C-2251 | H | CH(Me) | H | *c*-Pr | CH₂CN | Me | Et | O |
| C-2252 | H | CH₂CH₂ | Me | *c*-Pr | CH₂CN | Me | Et | O |
| C-2253 | Me | CH₂CH₂ | H | *c*-Pr | CH₂CN | Me | Et | O |
| C-2254 | H | single bond | Me | *c*-Pr | CH₂CN | Cl | CH₂CF₃ | O |
| C-2255 | Me | single bond | H | *c*-Pr | CH₂CN | Cl | CH₂CF₃ | O |
| C-2256 | Me(R) | single bond | H | *c*-Pr | CH₂CN | Cl | CH₂CF₃ | O |
| C-2257 | Me(S) | single bond | H | *c*-Pr | CH₂CN | Cl | CH₂CF₃ | O |
| C-2258 | H | CH₂ | Me | *c*-Pr | CH₂CN | Cl | CH₂CF₃ | O |
| C-2259 | Me | CH₂ | H | *c*-Pr | CH₂CN | Cl | CH₂CF₃ | O |
| C-2260 | H | CH(Me) | H | *c*-Pr | CH₂CN | Cl | CH₂CF₃ | O |
| C-2261 | H | CH₂CH₂ | Me | *c*-Pr | CH₂CN | Cl | CH₂CF₃ | O |
| C-2262 | Me | CH₂CH₂ | H | *c*-Pr | CH₂CN | Cl | CH₂CF₃ | O |
| C-2263 | H | single bond | Me | *c*-Pr | CH₂CN | Me | CH₂CF₃ | O |
| C-2264 | Me | single bond | H | *c*-Pr | CH₂CN | Me | CH₂CF₃ | O |
| C-2265 | Me(R) | single bond | H | *c*-Pr | CH₂CN | Me | CH₂CF₃ | O |
| C-2266 | Me(S) | single bond | H | *c*-Pr | CH₂CN | Me | CH₂CF₃ | O |
| C-2267 | H | CH₂ | Me | *c*-Pr | CH₂CN | Me | CH₂CF₃ | O |
| C-2268 | Me | CH₂ | H | *c*-Pr | CH₂CN | Me | CH₂CF₃ | O |
| C-2269 | H | CH(Me) | H | *c*-Pr | CH₂CN | Me | CH₂CF₃ | O |
| C-2270 | H | CH₂CH₂ | Me | *c*-Pr | CH₂CN | Me | CH₂CF₃ | O |
| C-2271 | Me | CH₂CH₂ | H | *c*-Pr | CH₂CN | Me | CH₂CF₃ | O |
| C-2272 | H | single bond | Me | *c*-Pr | CH₂CN | Cl | CH₂OMe | O |
| C-2273 | Me | single bond | H | *c*-Pr | CH₂CN | Cl | CH₂OMe | O |
| C-2274 | Me(R) | single bond | H | *c*-Pr | CH₂CN | Cl | CH₂OMe | O |
| C-2275 | Me(S) | single bond | H | *c*-Pr | CH₂CN | Cl | CH₂OMe | O |
| C-2276 | H | CH₂ | Me | *c*-Pr | CH₂CN | Cl | CH₂OMe | O |
| C-2277 | Me | CH₂ | H | *c*-Pr | CH₂CN | Cl | CH₂OMe | O |
| C-2278 | H | CH(Me) | H | *c*-Pr | CH₂CN | Cl | CH₂OMe | O |
| C-2279 | H | CH₂CH₂ | Me | *c*-Pr | CH₂CN | Cl | CH₂OMe | O |
| C-2280 | Me | CH₂CH₂ | H | *c*-Pr | CH₂CN | Cl | CH₂OMe | O |
| C-2281 | H | single bond | Me | *c*-Pr | CH₂CN | Me | CH₂OMe | O |
| C-2282 | Me | single bond | H | *c*-Pr | CH₂CN | Me | CH₂OMe | O |

**[Table 279]**

| Compound Number | Y | W | Z | R¹ | R² | R³ | R¹⁰ | A |
|---|---|---|---|---|---|---|---|---|
| C-2283 | Me(R) | single bond | H | *c*-Pr | CH₂CN | Me | CH₂OMe | O |
| C-2284 | Me(S) | single bond | H | *c*-Pr | CH₂CN | Me | CH₂OMe | O |
| C-2285 | H | CH₂ | Me | *c*-Pr | CH₂CN | Me | CH₂OMe | O |
| C-2286 | Me | CH₂ | H | *c*-Pr | CH₂CN | Me | CH₂OMe | O |
| C-2287 | H | CH(Me) | H | *c*-Pr | CH₂CN | Me | CH₂OMe | O |
| C-2288 | H | CH₂CH₂ | Me | *c*-Pr | CH₂CN | Me | CH₂OMe | O |
| C-2289 | Me | CH₂CH₂ | H | *c*-Pr | CH₂CN | Me | CH₂OMe | O |
| C-2290 | H | single bond | Me | *c*-Pr | CH₂CN | Cl | CH₂CN | O |
| C-2291 | Me | single bond | H | *c*-Pr | CH₂CN | Cl | CH₂CN | O |
| C-2292 | Me(R) | single bond | H | *c*-Pr | CH₂CN | Cl | CH₂CN | O |
| C-2293 | Me(S) | single bond | H | *c*-Pr | CH₂CN | Cl | CH₂CN | O |
| C-2294 | H | CH₂ | Me | *c*-Pr | CH₂CN | Cl | CH₂CN | O |
| C-2295 | Me | CH₂ | H | *c*-Pr | CH₂CN | Cl | CH₂CN | O |
| C-2296 | H | CH(Me) | H | *c*-Pr | CH₂CN | Cl | CH₂CN | O |
| C-2297 | H | CH₂CH₂ | Me | *c*-Pr | CH₂CN | Cl | CH₂CN | O |
| C-2298 | Me | CH₂CH₂ | H | *c*-Pr | CH₂CN | Cl | CH₂CN | O |
| C-2299 | H | single bond | Me | *c*-Pr | CH₂CN | Me | CH₂CN | O |
| C-2300 | Me | single bond | H | *c*-Pr | CH₂CN | Me | CH₂CN | O |
| C-2301 | Me(R) | single bond | H | *c*-Pr | CH₂CN | Me | CH₂CN | O |
| C-2302 | Me(S) | single bond | H | *c*-Pr | CH₂CN | Me | CH₂CN | O |
| C-2303 | H | CH₂ | Me | *c*-Pr | CH₂CN | Me | CH₂CN | O |
| C-2304 | Me | CH₂ | H | *c*-Pr | CH₂CN | Me | CH₂CN | O |
| C-2305 | H | CH(Me) | H | *c*-Pr | CH₂CN | Me | CH₂CN | O |
| C-2306 | H | CH₂CH₂ | Me | *c*-Pr | CH₂CN | Me | CH₂CN | O |
| C-2307 | Me | CH₂CH₂ | H | *c*-Pr | CH₂CN | Me | CH₂CN | O |
| C-2308 | H | single bond | Me | *c*-Pr | CH₂CN | Cl | C(=O)Me | O |
| C-2309 | Me | single bond | H | *c*-Pr | CH₂CN | Cl | C(=O)Me | O |
| C-2310 | Me(R) | single bond | H | *c*-Pr | CH₂CN | Cl | C(=O)Me | O |
| C-2311 | Me(S) | single bond | H | *c*-Pr | CH₂CN | Cl | C(=O)Me | O |
| C-2312 | H | CH₂ | Me | *c*-Pr | CH₂CN | Cl | C(=O)Me | O |
| C-2313 | Me | CH₂ | H | *c*-Pr | CH₂CN | Cl | C(=O)Me | O |
| C-2314 | H | CH(Me) | H | *c*-Pr | CH₂CN | Cl | C(=O)Me | O |
| C-2315 | H | CH₂CH₂ | Me | *c*-Pr | CH₂CN | Cl | C(=O)Me | O |
| C-2316 | Me | CH₂CH₂ | H | *c*-Pr | CH₂CN | Cl | C(=O)Me | O |
| C-2317 | H | single bond | Me | *c*-Pr | CH₂CN | Me | C(=O)Me | O |
| C-2318 | Me | single bond | H | *c*-Pr | CH₂CN | Me | C(=O)Me | O |
| C-2319 | Me(R) | single bond | H | *c*-Pr | CH₂CN | Me | C(=O)Me | O |
| C-2320 | Me(S) | single bond | H | *c*-Pr | CH₂CN | Me | C(=O)Me | O |
| C-2321 | H | CH₂ | Me | *c*-Pr | CH₂CN | Me | C(=O)Me | O |
| C-2322 | Me | CH₂ | H | *c*-Pr | CH₂CN | Me | C(=O)Me | O |
| C-2323 | H | CH(Me) | H | *c*-Pr | CH₂CN | Me | C(=O)Me | O |
| C-2324 | H | CH₂CH₂ | Me | *c*-Pr | CH₂CN | Me | C(=O)Me | O |
| C-2325 | Me | CH₂CH₂ | H | *c*-Pr | CH₂CN | Me | C(=O)Me | O |
| C-2326 | H | single bond | Me | *c*-Pr | C(=O)Me | Cl | Me | O |

**[Table 280]**

| Compound Number | Y | W | Z | R¹ | R² | R³ | R¹⁰ | A |
|---|---|---|---|---|---|---|---|---|
| C-2327 | Me | single bond | H | *c*-Pr | C(=O)Me | Cl | Me | O |
| C-2328 | Me(R) | single bond | H | *c*-Pr | C(=O)Me | Cl | Me | O |
| C-2329 | Me(S) | single bond | H | *c*-Pr | C(=O)Me | Cl | Me | O |
| C-2330 | H | CH₂ | Me | *c*-Pr | C(=O)Me | Cl | Me | O |
| C-2331 | Me | CH₂ | H | *c*-Pr | C(=O)Me | Cl | Me | O |
| C-2332 | H | CH(Me) | H | *c*-Pr | C(=O)Me | Cl | Me | O |
| C-2333 | H | CH₂CH₂ | Me | *c*-Pr | C(=O)Me | Cl | Me | O |
| C-2334 | Me | CH₂CH₂ | H | *c*-Pr | C(=O)Me | Cl | Me | O |
| C-2335 | H | single bond | Me | *c*-Pr | C(=O)Me | Me | Me | O |
| C-2336 | Me | single bond | H | *c*-Pr | C(=O)Me | Me | Me | O |
| C-2337 | Me(R) | single bond | H | *c*-Pr | C(=O)Me | Me | Me | O |
| C-2338 | Me(S) | single bond | H | *c*-Pr | C(=O)Me | Me | Me | O |
| C-2339 | H | CH₂ | Me | *c*-Pr | C(=O)Me | Me | Me | O |
| C-2340 | Me | CH₂ | H | *c*-Pr | C(=O)Me | Me | Me | O |
| C-2341 | H | CH(Me) | H | *c*-Pr | C(=O)Me | Me | Me | O |
| C-2342 | H | CH₂CH₂ | Me | *c*-Pr | C(=O)Me | Me | Me | O |
| C-2343 | Me | CH₂CH₂ | H | *c*-Pr | C(=O)Me | Me | Me | O |
| C-2344 | H | single bond | Me | *c*-Pr | C(=O)Me | Cl | Et | O |
| C-2345 | Me | single bond | H | *c*-Pr | C(=O)Me | Cl | Et | O |
| C-2346 | Me(R) | single bond | H | *c*-Pr | C(=O)Me | Cl | Et | O |
| C-2347 | Me(S) | single bond | H | *c*-Pr | C(=O)Me | Cl | Et | O |
| C-2348 | H | CH₂ | Me | *c*-Pr | C(=O)Me | Cl | Et | O |
| C-2349 | Me | CH₂ | H | *c*-Pr | C(=O)Me | Cl | Et | O |
| C-2350 | H | CH(Me) | H | *c*-Pr | C(=O)Me | Cl | Et | O |
| C-2351 | H | CH₂CH₂ | Me | *c*-Pr | C(=O)Me | Cl | Et | O |
| C-2352 | Me | CH₂CH₂ | H | *c*-Pr | C(=O)Me | Cl | Et | O |
| C-2353 | H | single bond | Me | *c*-Pr | C(=O)Me | Me | Et | O |
| C-2354 | Me | single bond | H | *c*-Pr | C(=O)Me | Me | Et | O |
| C-2355 | Me(R) | single bond | H | *c*-Pr | C(=O)Me | Me | Et | O |
| C-2356 | Me(S) | single bond | H | *c*-Pr | C(=O)Me | Me | Et | O |
| C-2357 | H | CH₂ | Me | *c*-Pr | C(=O)Me | Me | Et | O |
| C-2358 | Me | CH₂ | H | *c*-Pr | C(=O)Me | Me | Et | O |
| C-2359 | H | CH(Me) | H | *c*-Pr | C(=O)Me | Me | Et | O |
| C-2360 | H | CH₂CH₂ | Me | *c*-Pr | C(=O)Me | Me | Et | O |
| C-2361 | Me | CH₂CH₂ | H | *c*-Pr | C(=O)Me | Me | Et | O |
| C-2362 | H | single bond | Me | *c*-Pr | C(=O)Me | Cl | CH₂CF₃ | O |
| C-2363 | Me | single bond | H | *c*-Pr | C(=O)Me | Cl | CH₂CF₃ | O |
| C-2364 | Me(R) | single bond | H | *c*-Pr | C(=O)Me | Cl | CH₂CF₃ | O |
| C-2365 | Me(S) | single bond | H | *c*-Pr | C(=O)Me | Cl | CH₂CF₃ | O |
| C-2366 | H | CH₂ | Me | *c*-Pr | C(=O)Me | Cl | CH₂CF₃ | O |
| C-2367 | Me | CH₂ | H | *c*-Pr | C(=O)Me | Cl | CH₂CF₃ | O |
| C-2368 | H | CH(Me) | H | *c*-Pr | C(=O)Me | Cl | CH₂CF₃ | O |
| C-2369 | H | CH₂CH₂ | Me | *c*-Pr | C(=O)Me | Cl | CH₂CF₃ | O |
| C-2370 | Me | CH₂CH₂ | H | *c*-Pr | C(=O)Me | Cl | CH₂CF₃ | O |

**[Table 281]**

| Compound Number | Y | W | Z | R¹ | R² | R³ | R¹⁰ | A |
|---|---|---|---|---|---|---|---|---|
| C-2371 | H | single bond | Me | *c*-Pr | C(=O)Me | Me | CH₂CF₃ | O |
| C-2372 | Me | single bond | H | *c*-Pr | C(=O)Me | Me | CH₂CF₃ | O |
| C-2373 | Me(R) | single bond | H | *c*-Pr | C(=O)Me | Me | CH₂CF₃ | O |
| C-2374 | Me(S) | single bond | H | *c*-Pr | C(=O)Me | Me | CH₂CF₃ | O |
| C-2375 | H | CH₂ | Me | *c*-Pr | C(=O)Me | Me | CH₂CF₃ | O |
| C-2376 | Me | CH₂ | H | *c*-Pr | C(=O)Me | Me | CH₂CF₃ | O |
| C-2377 | H | CH(Me) | H | *c*-Pr | C(=O)Me | Me | CH₂CF₃ | O |
| C-2378 | H | CH₂CH₂ | Me | *c*-Pr | C(=O)Me | Me | CH₂CF₃ | O |
| C-2379 | Me | CH₂CH₂ | H | *c*-Pr | C(=O)Me | Me | CH₂CF₃ | O |
| C-2380 | H | single bond | Me | *c*-Pr | C(=O)Me | Cl | CH₂OMe | O |
| C-2381 | Me | single bond | H | *c*-Pr | C(=O)Me | Cl | CH₂OMe | O |
| C-2382 | Me(R) | single bond | H | *c*-Pr | C(=O)Me | Cl | CH₂OMe | O |
| C-2383 | Me(S) | single bond | H | *c*-Pr | C(=O)Me | Cl | CH₂OMe | O |
| C-2384 | H | CH₂ | Me | *c*-Pr | C(=O)Me | Cl | CH₂OMe | O |
| C-2385 | Me | CH₂ | H | *c*-Pr | C(=O)Me | Cl | CH₂OMe | O |
| C-2386 | H | CH(Me) | H | *c*-Pr | C(=O)Me | Cl | CH₂OMe | O |
| C-2387 | H | CH₂CH₂ | Me | *c*-Pr | C(=O)Me | Cl | CH₂OMe | O |
| C-2388 | Me | CH₂CH₂ | H | *c*-Pr | C(=O)Me | Cl | CH₂OMe | O |
| C-2389 | H | single bond | Me | *c*-Pr | C(=O)Me | Me | CH₂OMe | O |
| C-2390 | Me | single bond | H | *c*-Pr | C(=O)Me | Me | CH₂OMe | O |
| C-2391 | Me(R) | single bond | H | *c*-Pr | C(=O)Me | Me | CH₂OMe | O |
| C-2392 | Me(S) | single bond | H | *c*-Pr | C(=O)Me | Me | CH₂OMe | O |
| C-2393 | H | CH₂ | Me | *c*-Pr | C(=O)Me | Me | CH₂OMe | O |
| C-2394 | Me | CH₂ | H | *c*-Pr | C(=O)Me | Me | CH₂OMe | O |
| C-2395 | H | CH(Me) | H | *c*-Pr | C(=O)Me | Me | CH₂OMe | O |
| C-2396 | H | CH₂CH₂ | Me | *c*-Pr | C(=O)Me | Me | CH₂OMe | O |
| C-2397 | Me | CH₂CH₂ | H | *c*-Pr | C(=O)Me | Me | CH₂OMe | O |
| C-2398 | H | single bond | Me | *c*-Pr | C(=O)Me | Cl | CH₂CN | O |
| C-2399 | Me | single bond | H | *c*-Pr | C(=O)Me | Cl | CH₂CN | O |
| C-2400 | Me(R) | single bond | H | *c*-Pr | C(=O)Me | Cl | CH₂CN | O |
| C-2401 | Me(S) | single bond | H | *c*-Pr | C(=O)Me | Cl | CH₂CN | O |
| C-2402 | H | CH₂ | Me | *c*-Pr | C(=O)Me | Cl | CH₂CN | O |
| C-2403 | Me | CH₂ | H | *c*-Pr | C(=O)Me | Cl | CH₂CN | O |
| C-2404 | H | CH(Me) | H | *c*-Pr | C(=O)Me | Cl | CH₂CN | O |
| C-2405 | H | CH₂CH₂ | Me | *c*-Pr | C(=O)Me | Cl | CH₂CN | O |
| C-2406 | Me | CH₂CH₂ | H | *c*-Pr | C(=O)Me | Cl | CH₂CN | O |
| C-2407 | H | single bond | Me | *c*-Pr | C(=O)Me | Me | CH₂CN | O |
| C-2408 | Me | single bond | H | *c*-Pr | C(=O)Me | Me | CH₂CN | O |
| C-2409 | Me(R) | single bond | H | *c*-Pr | C(=O)Me | Me | CH₂CN | O |
| C-2410 | Me(S) | single bond | H | *c*-Pr | C(=O)Me | Me | CH₂CN | O |
| C-2411 | H | CH₂ | Me | *c*-Pr | C(=O)Me | Me | CH₂CN | O |
| C-2412 | Me | CH₂ | H | *c*-Pr | C(=O)Me | Me | CH₂CN | O |
| C-2413 | H | CH(Me) | H | *c*-Pr | C(=O)Me | Me | CH₂CN | O |
| C-2414 | H | CH₂CH₂ | Me | *c*-Pr | C(=O)Me | Me | CH₂CN | O |

**[Table 282]**

| Compound Number | Y | W | Z | R¹ | R² | R³ | R¹⁰ | A |
|---|---|---|---|---|---|---|---|---|
| C-2415 | Me | CH₂CH₂ | H | *c*-Pr | C(=O)Me | Me | CH₂CN | O |
| C-2416 | H | single bond | Me | *c*-Pr | C(=O)Me | Cl | C(=O)Me | O |
| C-2417 | Me | single bond | H | *c*-Pr | C(=O)Me | Cl | C(=O)Me | O |
| C-2418 | Me(R) | single bond | H | *c*-Pr | C(=O)Me | Cl | C(=O)Me | O |
| C-2419 | Me(S) | single bond | H | *c*-Pr | C(=O)Me | Cl | C(=O)Me | O |
| C-2420 | H | CH₂ | Me | *c*-Pr | C(=O)Me | Cl | C(=O)Me | O |
| C-2421 | Me | CH₂ | H | *c*-Pr | C(=O)Me | Cl | C(=O)Me | O |
| C-2422 | H | CH(Me) | H | *c*-Pr | C(=O)Me | Cl | C(=O)Me | O |
| C-2423 | H | CH₂CH₂ | Me | *c*-Pr | C(=O)Me | Cl | C(=O)Me | O |
| C-2424 | Me | CH₂CH₂ | H | *c*-Pr | C(=O)Me | Cl | C(=O)Me | O |
| C-2425 | H | single bond | Me | *c*-Pr | C(=O)Me | Me | C(=O)Me | O |
| C-2426 | Me | single bond | H | *c*-Pr | C(=O)Me | Me | C(=O)Me | O |
| C-2427 | Me(R) | single bond | H | *c*-Pr | C(=O)Me | Me | C(=O)Me | O |
| C-2428 | Me(S) | single bond | H | *c*-Pr | C(=O)Me | Me | C(=O)Me | O |
| C-2429 | H | CH₂ | Me | *c*-Pr | C(=O)Me | Me | C(=O)Me | O |
| C-2430 | Me | CH₂ | H | *c*-Pr | C(=O)Me | Me | C(=O)Me | O |
| C-2431 | H | CH(Me) | H | *c*-Pr | C(=O)Me | Me | C(=O)Me | O |
| C-2432 | H | CH₂CH₂ | Me | *c*-Pr | C(=O)Me | Me | C(=O)Me | O |
| C-2433 | Me | CH₂CH₂ | H | *c*-Pr | C(=O)Me | Me | C(=O)Me | O |
| C-2434 | H | single bond | Me | *c*-Pr | C(=O)(*i*-Pr) | Cl | Me | O |
| C-2435 | Me | single bond | H | *c*-Pr | C(=O)(*i*-Pr) | Cl | Me | O |
| C-2436 | Me(R) | single bond | H | *c*-Pr | C(=O)(*i*-Pr) | Cl | Me | O |
| C-2437 | Me(S) | single bond | H | *c*-Pr | C(=O)(*i* -Pr) | Cl | Me | O |
| C-2438 | H | CH₂ | Me | *c*-Pr | C(=O)(*i*-Pr) | Cl | Me | O |
| C-2439 | Me | CH₂ | H | *c*-Pr | C(=O)(*i*-Pr) | Cl | Me | O |
| C-2440 | H | CH(Me) | H | *c*-Pr | C(=O)(*i*-Pr) | Cl | Me | O |
| C-2441 | H | CH₂CH₂ | Me | *c*-Pr | C(=O)(*i*-Pr) | Cl | Me | O |
| C-2442 | Me | CH₂CH₂ | H | *c*-Pr | C(=O)(*i*-Pr) | Cl | Me | O |
| C-2443 | H | single bond | Me | *c*-Pr | C(=O)(*i*-Pr) | Me | Me | O |
| C-2444 | Me | single bond | H | *c*-Pr | C(=O)(*i*-Pr) | Me | Me | O |
| C-2445 | Me(R) | single bond | H | *c*-Pr | C(=O)(*i*-Pr) | Me | Me | O |
| C-2446 | Me(S) | single bond | H | *c*-Pr | C(=O)(*i*-Pr) | Me | Me | O |
| C-2447 | H | CH₂ | Me | *c*-Pr | C(=O)(*i*-Pr) | Me | Me | O |
| C-2448 | Me | CH₂ | H | *c*-Pr | C(=O)(*i*-Pr) | Me | Me | O |
| C-2449 | H | CH(Me) | H | *c*-Pr | C(=O)(*i*-Pr) | Me | Me | O |
| C-2450 | H | CH₂CH₂ | Me | *c*-Pr | C(=O)(*i*-Pr) | Me | Me | O |
| C-2451 | Me | CH₂CH₂ | H | *c*-Pr | C(=O)(*i*-Pr) | Me | Me | O |
| C-2452 | H | single bond | Me | *c*-Pr | C(=O)(*i*-Pr) | Cl | Et | O |
| C-2453 | Me | single bond | H | *c*-Pr | C(=O)(*i*-Pr) | Cl | Et | O |
| C-2454 | Me(R) | single bond | H | *c*-Pr | C(=O)(*i*-Pr) | Cl | Et | O |
| C-2455 | Me(S) | single bond | H | *c*-Pr | C(=O)(*i*-Pr) | Cl | Et | O |
| C-2456 | H | CH₂ | Me | *c*-Pr | C(=O)(*i*-Pr) | Cl | Et | O |
| C-2457 | Me | CH₂ | H | *c*-Pr | C(=O)(*i*-Pr) | Cl | Et | O |
| C-2458 | H | CH(Me) | H | *c*-Pr | C(=O)(*i*-Pr) | Cl | Et | O |

**[Table 283]**

| Compound Number | Y | W | Z | R¹ | R² | R³ | R¹⁰ | A |
|---|---|---|---|---|---|---|---|---|
| C-2459 | H | CH₂CH₂ | Me | *c*-Pr | C(=O)(*i*-Pr) | Cl | Et | O |
| C-2460 | Me | CH₂CH₂ | H | *c*-Pr | C(=O)(*i*-Pr) | Cl | Et | O |
| C-2461 | H | single bond | Me | *c*-Pr | C(=O)(*i*-Pr) | Me | Et | O |
| C-2462 | Me | single bond | H | *c*-Pr | C(=O)(*i*-Pr) | Me | Et | O |
| C-2463 | Me(R) | single bond | H | *c*-Pr | C(=O)(*i*-Pr) | Me | Et | O |
| C-2464 | Me(S) | single bond | H | *c*-Pr | C(=O)(*i*-Pr) | Me | Et | O |
| C-2465 | H | CH₂ | Me | *c*-Pr | C(=O)(*i*-Pr) | Me | Et | O |
| C-2466 | Me | CH₂ | H | *c*-Pr | C(=O)(*i*-Pr) | Me | Et | O |
| C-2467 | H | CH(Me) | H | *c*-Pr | C(=O)(*i*-Pr) | Me | Et | O |
| C-2468 | H | CH₂CH₂ | Me | *c*-Pr | C(=O)(*i*-Pr) | Me | Et | O |
| C-2469 | Me | CH₂CH₂ | H | *c*-Pr | C(=O)(*i*-Pr) | Me | Et | O |
| C-2470 | H | single bond | Me | *c*-Pr | C(=O)(*i*-Pr) | Cl | CH₂CF₃ | O |
| C-2471 | Me | single bond | H | *c*-Pr | C(=O)(*i*-Pr) | Cl | CH₂CF₃ | O |
| C-2472 | Me(R) | single bond | H | *c*-Pr | C(=O)(*i*-Pr) | Cl | CH₂CF₃ | O |
| C-2473 | Me(S) | single bond | H | *c*-Pr | C(=O)(*i*-Pr) | Cl | CH₂CF₃ | O |
| C-2474 | H | CH₂ | Me | *c*-Pr | C(=O)(*i*-Pr) | Cl | CH₂CF₃ | O |
| C-2475 | Me | CH₂ | H | *c*-Pr | C(=O)(*i*-Pr) | Cl | CH₂CF₃ | O |
| C-2476 | H | CH(Me) | H | *c*-Pr | C(=O)(*i*-Pr) | Cl | CH₂CF₃ | O |
| C-2477 | H | CH₂CH₂ | Me | *c*-Pr | C(=O)(*i*-Pr) | Cl | CH₂CF₃ | O |
| C-2478 | Me | CH₂CH₂ | H | *c*-Pr | C(=O)(*i*-Pr) | Cl | CH₂CF₃ | O |
| C-2479 | H | single bond | Me | *c*-Pr | C(=O)(*i*-Pr) | Me | CH₂CF₃ | O |
| C-2480 | Me | single bond | H | *c*-Pr | C(=O)(*i*-Pr) | Me | CH₂CF₃ | O |
| C-2481 | Me(R) | single bond | H | *c*-Pr | C(=O)(*i*-Pr) | Me | CH₂CF₃ | O |
| C-2482 | Me(S) | single bond | H | *c*-Pr | C(=O)(*i*-Pr) | Me | CH₂CF₃ | O |
| C-2483 | H | CH₂ | Me | *c-*Pr | C(=O)(*i*-Pr) | Me | CH₂CF₃ | O |
| C-2484 | Me | CH₂ | H | *c*-Pr | C(=O)(*i*-Pr) | Me | CH₂CF₃ | O |
| C-2485 | H | CH(Me) | H | *c*-Pr | C(=O)(*i*-Pr) | Me | CH₂CF₃ | O |
| C-2486 | H | CH₂CH₂ | Me | *c*-Pr | C(=O)(*i*-Pr) | Me | CH₂CF₃ | O |
| C-2487 | Me | CH₂CH₂ | H | *c*-Pr | C(=O)(*i*-Pr) | Me | CH₂CF₃ | O |
| C-2488 | H | single bond | Me | *c*-Pr | C(=O)(*i*-Pr) | Cl | CH₂OMe | O |
| C-2489 | Me | single bond | H | *c*-Pr | C(=O)(*i*-Pr) | Cl | CH₂OMe | O |
| C-2490 | Me(R) | single bond | H | *c*-Pr | C(=O)(*i*-Pr) | Cl | CH₂OMe | O |
| C-2491 | Me(S) | single bond | H | *c*-Pr | C(=O)(*i*-Pr) | Cl | CH₂OMe | O |
| C-2492 | H | CH₂ | Me | *c*-Pr | C(=O)(*i*-Pr) | Cl | CH₂OMe | O |
| C-2493 | Me | CH₂ | H | *c*-Pr | C(=O)(*i*-Pr) | Cl | CH₂OMe | O |
| C-2494 | H | CH(Me) | H | *c*-Pr | C(=O)(*i*-Pr) | Cl | CH₂OMe | O |
| C-2495 | H | CH₂CH₂ | Me | *c*-Pr | C(=O)(*i*-Pr) | Cl | CH₂OMe | O |
| C-2496 | Me | CH₂CH₂ | H | *c*-Pr | C(=O)(*i*-Pr) | Cl | CH₂OMe | O |
| C-2497 | H | single bond | Me | *c*-Pr | C(=O)(*i*-Pr) | Me | CH₂OMe | O |
| C-2498 | Me | single bond | H | *c*-Pr | C(=O)(*i*-Pr) | Me | CH₂OMe | O |
| C-2499 | Me(R) | single bond | H | *c*-Pr | C(=O)(*i*-Pr) | Me | CH₂OMe | O |
| C-2500 | Me(S) | single bond | H | *c*-Pr | C(=O)(*i*-Pr) | Me | CH₂OMe | O |
| C-2501 | H | CH₂ | Me | *c*-Pr | C(=O)(*i*-Pr) | Me | CH₂OMe | O |
| C-2502 | Me | CH₂ | H | *c*-Pr | C(=O)(*i*-Pr) | Me | CH₂OMe | O |

**[Table 284]**

| Compound Number | Y | W | Z | R¹ | R² | R³ | R¹⁰ | A |
|---|---|---|---|---|---|---|---|---|
| C-2503 | H | CH(Me) | H | *c*-Pr | C(=O)(*i*-Pr) | Me | CH₂OMe | O |
| C-2504 | H | CH₂CH₂ | Me | *c*-Pr | C(=O)(*i*-Pr) | Me | CH₂OMe | O |
| C-2505 | Me | CH₂CH₂ | H | *c*-Pr | C(=O)(*i*-Pr) | Me | CH₂OMe | O |
| C-2506 | H | single bond | Me | *c*-Pr | C(=O)(*i*-Pr) | Cl | CH₂CN | O |
| C-2507 | Me | single bond | H | *c*-Pr | C(=O)(*i*-Pr) | Cl | CH₂CN | O |
| C-2508 | Me(R) | single bond | H | *c*-Pr | C(=O)(*i*-Pr) | Cl | CH₂CN | O |
| C-2509 | Me(S) | single bond | H | *c*-Pr | C(=O)(*i*-Pr) | Cl | CH₂CN | O |
| C-2510 | H | CH₂ | Me | *c*-Pr | C(=O)(*i*-Pr) | Cl | CH₂CN | O |
| C-2511 | Me | CH₂ | H | *c*-Pr | C(=O)(*i*-Pr) | Cl | CH₂CN | O |
| C-2512 | H | CH(Me) | H | *c*-Pr | C(=O)(*i*-Pr) | Cl | CH₂CN | O |
| C-2513 | H | CH₂CH₂ | Me | *c*-Pr | C(=O)(*i*-Pr) | Cl | CH₂CN | O |
| C-2514 | Me | CH₂CH₂ | H | *c*-Pr | C(=O)(*i*-Pr) | Cl | CH₂CN | O |
| C-2515 | H | single bond | Me | *c*-Pr | C(=O)(*i*-Pr) | Me | CH₂CN | O |
| C-2516 | Me | single bond | H | *c*-Pr | C(=O)(*i*-Pr) | Me | CH₂CN | O |
| C-2517 | Me(R) | single bond | H | *c*-Pr | C(=O)(*i*-Pr) | Me | CH₂CN | O |
| C-2518 | Me(S) | single bond | H | *c*-Pr | C(=O)(*i*-Pr) | Me | CH₂CN | O |
| C-2519 | H | CH₂ | Me | *c*-Pr | C(=O)(*i*-Pr) | Me | CH₂CN | O |
| C-2520 | Me | CH₂ | H | *c*-Pr | C(=O)(*i*-Pr) | Me | CH₂CN | O |
| C-2521 | H | CH(Me) | H | *c*-Pr | C(=O)(*i*-Pr) | Me | CH₂CN | O |
| C-2522 | H | CH₂CH₂ | Me | *c*-Pr | C(=O)(*i*-Pr) | Me | CH₂CN | O |
| C-2523 | Me | CH₂CH₂ | H | *c*-Pr | C(=O)(*i*-Pr) | Me | CH₂CN | O |
| C-2524 | H | single bond | Me | *c*-Pr | C(=O)(*i*-Pr) | Cl | C(=O)Me | O |
| C-2525 | Me | single bond | H | *c*-Pr | C(=O)(*i*-Pr) | Cl | C(=O)Me | O |
| C-2526 | Me(R) | single bond | H | *c*-Pr | C(=O)(*i*-Pr) | Cl | C(=O)Me | O |
| C-2527 | Me(S) | single bond | H | *c*-Pr | C(=O)(*i*-Pr) | Cl | C(=O)Me | O |
| C-2528 | H | CH₂ | Me | *c*-Pr | C(=O)(*i*-Pr) | Cl | C(=O)Me | O |
| C-2529 | Me | CH₂ | H | *c*-Pr | C(=O)(*i*-Pr) | Cl | C(=O)Me | O |
| C-2530 | H | CH(Me) | H | *c*-Pr | C(=O)(*i*-Pr) | Cl | C(=O)Me | O |
| C-2531 | H | CH₂CH₂ | Me | *c*-Pr | C(=O)(*i*-Pr) | Cl | C(=O)Me | O |
| C-2532 | Me | CH₂CH₂ | H | *c*-Pr | C(=O)(*i*-Pr) | Cl | C(=O)Me | O |
| C-2533 | H | single bond | Me | *c*-Pr | C(=O)(*i*-Pr) | Me | C(=O)Me | O |
| C-2534 | Me | single bond | H | *c*-Pr | C(=O)(*i*-Pr) | Me | C(=O)Me | O |
| C-2535 | Me(R) | single bond | H | *c*-Pr | C(=O)(*i*-Pr) | Me | C(=O)Me | O |
| C-2536 | Me(S) | single bond | H | *c*-Pr | C(=O)(*i*-Pr) | Me | C(=O)Me | O |
| C-2537 | H | CH₂ | Me | *c*-Pr | C(=O)(*i*-Pr) | Me | C(=O)Me | O |
| C-2538 | Me | CH₂ | H | *c*-Pr | C(=O)(*i*-Pr) | Me | C(=O)Me | O |
| C-2539 | H | CH(Me) | H | *c*-Pr | C(=O)(*i*-Pr) | Me | C(=O)Me | O |
| C-2540 | H | CH₂CH₂ | Me | *c*-Pr | C(=O)(*i*-Pr) | Me | C(=O)Me | O |
| C-2541 | Me | CH₂CH₂ | H | *c*-Pr | C(=O)(*i*-Pr) | Me | C(=O)Me | O |
| C-2542 | H | single bond | Me | *c*-Pr | C(=O)Ph | Cl | Me | O |
| C-2543 | Me | single bond | H | *c*-Pr | C(=O)Ph | Cl | Me | O |
| C-2544 | Me(R) | single bond | H | *c*-Pr | C(=O)Ph | Cl | Me | O |
| C-2545 | Me(S) | single bond | H | *c*-Pr | C(=O)Ph | Cl | Me | O |
| C-2546 | H | CH₂ | Me | *c*-Pr | C(=O)Ph | Cl | Me | O |

**[Table 285]**

| Compound Number | Y | W | Z | R¹ | R² | R³ | R¹⁰ | A |
|---|---|---|---|---|---|---|---|---|
| C-2547 | Me | CH₂ | H | *c*-Pr | C(=O)Ph | Cl | Me | O |
| C-2548 | H | CH(Me) | H | *c*-Pr | C(=O)Ph | Cl | Me | O |
| C-2549 | H | CH₂CH₂ | Me | *c*-Pr | C(=O)Ph | Cl | Me | O |
| C-2550 | Me | CH₂CH₂ | H | *c*-Pr | C(=O)Ph | Cl | Me | O |
| C-2551 | H | single bond | Me | *c*-Pr | C(=O)Ph | Me | Me | O |
| C-2552 | Me | single bond | H | *c*-Pr | C(=O)Ph | Me | Me | O |
| C-2553 | Me(R) | single bond | H | *c*-Pr | C(=O)Ph | Me | Me | O |
| C-2554 | Me(S) | single bond | H | *c*-Pr | C(=O)Ph | Me | Me | O |
| C-2555 | H | CH₂ | Me | *c*-Pr | C(=O)Ph | Me | Me | O |
| C-2556 | Me | CH₂ | H | *c*-Pr | C(=O)Ph | Me | Me | O |
| C-2557 | H | CH(Me) | H | *c*-Pr | C(=O)Ph | Me | Me | O |
| C-2558 | H | CH₂CH₂ | Me | *c*-Pr | C(=O)Ph | Me | Me | O |
| C-2559 | Me | CH₂CH₂ | H | *c*-Pr | C(=O)Ph | Me | Me | O |
| C-2560 | H | single bond | Me | *c*-Pr | C(=O)Ph | Cl | Et | O |
| C-2561 | Me | single bond | H | *c*-Pr | C(=O)Ph | Cl | Et | O |
| C-2562 | Me(R) | single bond | H | *c*-Pr | C(=O)Ph | Cl | Et | O |
| C-2563 | Me(S) | single bond | H | *c*-Pr | C(=O)Ph | Cl | Et | O |
| C-2564 | H | CH₂ | Me | *c*-Pr | C(=O)Ph | Cl | Et | O |
| C-2565 | Me | CH₂ | H | *c*-Pr | C(=O)Ph | Cl | Et | O |
| C-2566 | H | CH(Me) | H | *c*-Pr | C(=O)Ph | Cl | Et | O |
| C-2567 | H | CH₂CH₂ | Me | *c*-Pr | C(=O)Ph | Cl | Et | O |
| C-2568 | Me | CH₂CH₂ | H | *c*-Pr | C(=O)Ph | Cl | Et | O |
| C-2569 | H | single bond | Me | *c*-Pr | C(=O)Ph | Me | Et | O |
| C-2570 | Me | single bond | H | *c*-Pr | C(=O)Ph | Me | Et | O |
| C-2571 | Me(R) | single bond | H | *c*-Pr | C(=O)Ph | Me | Et | O |
| C-2572 | Me(S) | single bond | H | *c*-Pr | C(=O)Ph | Me | Et | O |
| C-2573 | H | CH₂ | Me | *c*-Pr | C(=O)Ph | Me | Et | O |
| C-2574 | Me | CH₂ | H | *c*-Pr | C(=O)Ph | Me | Et | O |
| C-2575 | H | CH(Me) | H | *c*-Pr | C(=O)Ph | Me | Et | O |
| C-2576 | H | CH₂CH₂ | Me | *c*-Pr | C(=O)Ph | Me | Et | O |
| C-2577 | Me | CH₂CH₂ | H | *c*-Pr | C(=O)Ph | Me | Et | O |
| C-2578 | H | single bond | Me | *c*-Pr | C(=O)Ph | Cl | CH₂CF₃ | O |
| C-2579 | Me | single bond | H | *c*-Pr | C(=O)Ph | Cl | CH₂CF₃ | O |
| C-2580 | Me(R) | single bond | H | *c*-Pr | C(=O)Ph | Cl | CH₂CF₃ | O |
| C-2581 | Me(S) | single bond | H | *c*-Pr | C(=O)Ph | Cl | CH₂CF₃ | O |
| C-2582 | H | CH₂ | Me | *c*-Pr | C(=O)Ph | Cl | CH₂CF₃ | O |
| C-2583 | Me | CH₂ | H | *c*-Pr | C(=O)Ph | Cl | CH₂CF₃ | O |
| C-2584 | H | CH(Me) | H | *c*-Pr | C(=O)Ph | Cl | CH₂CF₃ | O |
| C-2585 | H | CH₂CH₂ | Me | *c*-Pr | C(=O)Ph | Cl | CH₂CF₃ | O |
| C-2586 | Me | CH₂CH₂ | H | *c*-Pr | C(=O)Ph | Cl | CH₂CF₃ | O |
| C-2587 | H | single bond | Me | *c*-Pr | C(=O)Ph | Me | CH₂CF₃ | O |
| C-2588 | Me | single bond | H | *c*-Pr | C(=O)Ph | Me | CH₂CF₃ | O |
| C-2589 | Me(R) | single bond | H | *c*-Pr | C(=O)Ph | Me | CH₂CF₃ | O |
| C-2590 | Me(S) | single bond | H | *c*-Pr | C(=O)Ph | Me | CH₂CF₃ | O |

**[Table 286]**

| Compound Number | Y | W | Z | R¹ | R² | R³ | R¹⁰ | A |
|---|---|---|---|---|---|---|---|---|
| C-2591 | H | CH₂ | Me | *c*-Pr | C(=O)Ph | Me | CH₂CF₃ | O |
| C-2592 | Me | CH₂ | H | *c*-Pr | C(=O)Ph | Me | CH₂CF₃ | O |
| C-2593 | H | CH(Me) | H | *c*-Pr | C(=O)Ph | Me | CH₂CF₃ | O |
| C-2594 | H | CH₂CH₂ | Me | *c*-Pr | C(=O)Ph | Me | CH₂CF₃ | O |
| C-2595 | Me | CH₂CH₂ | H | *c*-Pr | C(=O)Ph | Me | CH₂CF₃ | O |
| C-2596 | H | single bond | Me | *c*-Pr | C(=O)Ph | Cl | CH₂OMe | O |
| C-2597 | Me | single bond | H | *c*-Pr | C(=O)Ph | Cl | CH₂OMe | O |
| C-2598 | Me(R) | single bond | H | *c*-Pr | C(=O)Ph | Cl | CH₂OMe | O |
| C-2599 | Me(S) | single bond | H | *c*-Pr | C(=O)Ph | Cl | CH₂OMe | O |
| C-2600 | H | CH₂ | Me | *c*-Pr | C(=O)Ph | Cl | CH₂OMe | O |
| C-2601 | Me | CH₂ | H | *c*-Pr | C(=O)Ph | Cl | CH₂OMe | O |
| C-2602 | H | CH(Me) | H | *c*-Pr | C(=O)Ph | Cl | CH₂OMe | O |
| C-2603 | H | CH₂CH₂ | Me | *c*-Pr | C(=O)Ph | Cl | CH₂OMe | O |
| C-2604 | Me | CH₂CH₂ | H | *c*-Pr | C(=O)Ph | Cl | CH₂OMe | O |
| C-2605 | H | single bond | Me | *c*-Pr | C(=O)Ph | Me | CH₂OMe | O |
| C-2606 | Me | single bond | H | *c*-Pr | C(=O)Ph | Me | CH₂OMe | O |
| C-2607 | Me(R) | single bond | H | *c*-Pr | C(=O)Ph | Me | CH₂OMe | O |
| C-2608 | Me(S) | single bond | H | *c*-Pr | C(=O)Ph | Me | CH₂OMe | O |
| C-2609 | H | CH₂ | Me | *c*-Pr | C(=O)Ph | Me | CH₂OMe | O |
| C-2610 | Me | CH₂ | H | *c*-Pr | C(=O)Ph | Me | CH₂OMe | O |
| C-2611 | H | CH(Me) | H | *c*-Pr | C(=O)Ph | Me | CH₂OMe | O |
| C-2612 | H | CH₂CH₂ | Me | *c*-Pr | C(=O)Ph | Me | CH₂OMe | O |
| C-2613 | Me | CH₂CH₂ | H | *c*-Pr | C(=O)Ph | Me | CH₂OMe | O |
| C-2614 | H | single bond | Me | *c*-Pr | C(=O)Ph | Cl | CH₂CN | O |
| C-2615 | Me | single bond | H | *c*-Pr | C(=O)Ph | Cl | CH₂CN | O |
| C-2616 | Me(R) | single bond | H | *c*-Pr | C(=O)Ph | Cl | CH₂CN | O |
| C-2617 | Me(S) | single bond | H | *c*-Pr | C(=O)Ph | Cl | CH₂CN | O |
| C-2618 | H | CH₂ | Me | *c*-Pr | C(=O)Ph | Cl | CH₂CN | O |
| C-2619 | Me | CH₂ | H | *c*-Pr | C(=O)Ph | Cl | CH₂CN | O |
| C-2620 | H | CH(Me) | H | *c*-Pr | C(=O)Ph | Cl | CH₂CN | O |
| C-2621 | H | CH₂CH₂ | Me | *c*-Pr | C(=O)Ph | Cl | CH₂CN | O |
| C-2622 | Me | CH₂CH₂ | H | *c*-Pr | C(=O)Ph | Cl | CH₂CN | O |
| C-2623 | H | single bond | Me | *c*-Pr | C(=O)Ph | Me | CH₂CN | O |
| C-2624 | Me | single bond | H | *c*-Pr | C(=O)Ph | Me | CH₂CN | O |
| C-2625 | Me(R) | single bond | H | *c*-Pr | C(=O)Ph | Me | CH₂CN | O |
| C-2626 | Me(S) | single bond | H | *c*-Pr | C(=O)Ph | Me | CH₂CN | O |
| C-2627 | H | CH₂ | Me | *c*-Pr | C(=O)Ph | Me | CH₂CN | O |
| C-2628 | Me | CH₂ | H | *c*-Pr | C(=O)Ph | Me | CH₂CN | O |
| C-2629 | H | CH(Me) | H | *c*-Pr | C(=O)Ph | Me | CH₂CN | O |
| C-2630 | H | CH₂CH₂ | Me | *c*-Pr | C(=O)Ph | Me | CH₂CN | O |
| C-2631 | Me | CH₂CH₂ | H | *c*-Pr | C(=O)Ph | Me | CH₂CN | O |
| C-2632 | H | single bond | Me | *c*-Pr | C(=O)Ph | Cl | C(=O)Me | O |
| C-2633 | Me | single bond | H | *c*-Pr | C(=O)Ph | Cl | C(=O)Me | O |
| C-2634 | Me(R) | single bond | H | *c*-Pr | C(=O)Ph | Cl | C(=O)Me | O |

**[Table 287]**

| Compound Number | Y | W | Z | R¹ | R² | R³ | R¹⁰ | A |
|---|---|---|---|---|---|---|---|---|
| C-2635 | Me(S) | single bond | H | *c*-Pr | C(=O)Ph | Cl | C(=O)Me | O |
| C-2636 | H | CH₂ | Me | *c*-Pr | C(=O)Ph | Cl | C(=O)Me | O |
| C-2637 | Me | CH₂ | H | *c*-Pr | C(=O)Ph | Cl | C(=O)Me | O |
| C-2638 | H | CH(Me) | H | *c*-Pr | C(=O)Ph | Cl | C(=O)Me | O |
| C-2639 | H | CH₂CH₂ | Me | *c*-Pr | C(=O)Ph | Cl | C(=O)Me | O |
| C-2640 | Me | CH₂CH₂ | H | *c*-Pr | C(=O)Ph | Cl | C(=O)Me | O |
| C-2641 | H | single bond | Me | *c*-Pr | C(=O)Ph | Me | C(=O)Me | O |
| C-2642 | Me | single bond | H | *c*-Pr | C(=O)Ph | Me | C(=O)Me | O |
| C-2643 | Me(R) | single bond | H | *c*-Pr | C(=O)Ph | Me | C(=O)Me | O |
| C-2644 | Me(S) | single bond | H | *c*-Pr | C(=O)Ph | Me | C(=O)Me | O |
| C-2645 | H | CH₂ | Me | *c*-Pr | C(=O)Ph | Me | C(=O)Me | O |
| C-2646 | Me | CH₂ | H | *c*-Pr | C(=O)Ph | Me | C(=O)Me | O |
| C-2647 | H | CH(Me) | H | *c*-Pr | C(=O)Ph | Me | C(=O)Me | O |
| C-2648 | H | CH₂CH₂ | Me | *c*-Pr | C(=O)Ph | Me | C(=O)Me | O |
| C-2649 | Me | CH₂CH₂ | H | *c*-Pr | C(=O)Ph | Me | C(=O)Me | O |
| C-2650 | H | single bond | Me | *c*-Pr | C(=O)OEt | Cl | Me | O |
| C-2651 | H | single bond | Et | *c*-Pr | C(=O)OEt | Cl | Me | O |
| C-2652 | Me | single bond | H | *c*-Pr | C(=O)OEt | Cl | Me | O |
| C-2653 | Me(R) | single bond | H | *c*-Pr | C(=O)OEt | Cl | Me | O |
| C-2654 | Me(S) | single bond | H | *c*-Pr | C(=O)OEt | Cl | Me | O |
| C-2655 | H | CH₂ | Me | *c*-Pr | C(=O)OEt | Cl | Me | O |
| C-2656 | Me | CH₂ | H | *c*-Pr | C(=O)OEt | Cl | Me | O |
| C-2657 | H | CH(Me) | H | *c*-Pr | C(=O)OEt | Cl | Me | O |
| C-2658 | H | CH₂CH₂ | Me | *c*-Pr | C(=O)OEt | Cl | Me | O |
| C-2659 | Me | CH₂CH₂ | H | *c*-Pr | C(=O)OEt | Cl | Me | O |
| C-2660 | H | single bond | Me | *c*-Pr | C(=O)OEt | Me | Me | O |
| C-2661 | Me | single bond | H | *c*-Pr | C(=O)OEt | Me | Me | O |
| C-2662 | Me(R) | single bond | H | *c*-Pr | C(=O)OEt | Me | Me | O |
| C-2663 | Me(S) | single bond | H | *c*-Pr | C(=O)OEt | Me | Me | O |
| C-2664 | H | CH₂ | Me | *c*-Pr | C(=O)OEt | Me | Me | O |
| C-2665 | Me | CH₂ | H | *c*-Pr | C(=O)OEt | Me | Me | O |
| C-2666 | H | CH(Me) | H | *c*-Pr | C(=O)OEt | Me | Me | O |
| C-2667 | H | CH₂CH₂ | Me | *c*-Pr | C(=O)OEt | Me | Me | O |
| C-2668 | Me | CH₂CH₂ | H | *c*-Pr | C(=O)OEt | Me | Me | O |
| C-2669 | H | single bond | Me | *c*-Pr | C(=O)OEt | Cl | Et | O |
| C-2670 | Me | single bond | H | *c*-Pr | C(=O)OEt | Cl | Et | O |
| C-2671 | Me(R) | single bond | H | *c*-Pr | C(=O)OEt | Cl | Et | O |
| C-2672 | Me(S) | single bond | H | *c*-Pr | C(=O)OEt | Cl | Et | O |
| C-2673 | H | CH₂ | Me | *c*-Pr | C(=O)OEt | Cl | Et | O |
| C-2674 | Me | CH₂ | H | *c*-Pr | C(=O)OEt | Cl | Et | O |
| C-2675 | H | CH(Me) | H | *c*-Pr | C(=O)OEt | Cl | Et | O |
| C-2676 | H | CH₂CH₂ | Me | *c*-Pr | C(=O)OEt | Cl | Et | O |
| C-2677 | Me | CH₂CH₂ | H | *c*-Pr | C(=O)OEt | Cl | Et | O |
| C-2678 | H | single bond | Me | *c*-Pr | C(=O)OEt | Me | Et | O |

**[Table 288]**

| Compound Number | Y | W | Z | R¹ | R² | R³ | R¹⁰ | A |
|---|---|---|---|---|---|---|---|---|
| C-2679 | Me | single bond | H | *c*-Pr | C(=O)OEt | Me | Et | O |
| C-2680 | Me(R) | single bond | H | *c*-Pr | C(=O)OEt | Me | Et | O |
| C-2681 | Me(S) | single bond | H | *c-*Pr | C(=O)OEt | Me | Et | O |
| C-2682 | H | CH₂ | Me | *c*-Pr | C(=O)OEt | Me | Et | O |
| C-2683 | Me | CH₂ | H | *c*-Pr | C(=O)OEt | Me | Et | O |
| C-2684 | H | CH(Me) | H | *c*-Pr | C(=O)OEt | Me | Et | O |
| C-2685 | H | CH₂CH₂ | Me | *c*-Pr | C(=O)OEt | Me | Et | O |
| C-2686 | Me | CH₂CH₂ | H | *c*-Pr | C(=O)OEt | Me | Et | O |
| C-2687 | H | single bond | Me | *c*-Pr | C(=O)OEt | Cl | CH₂CF₃ | O |
| C-2688 | Me | single bond | H | *c*-Pr | C(=O)OEt | Cl | CH₂CF₃ | O |
| C-2689 | Me(R) | single bond | H | *c*-Pr | C(=O)OEt | Cl | CH₂CF₃ | O |
| C-2690 | Me(S) | single bond | H | *c*-Pr | C(=O)OEt | Cl | CH₂CF₃ | O |
| C-2691 | H | CH₂ | Me | *c*-Pr | C(=O)OEt | Cl | CH₂CF₃ | O |
| C-2692 | Me | CH₂ | H | *c*-Pr | C(=O)OEt | Cl | CH₂CF₃ | O |
| C-2693 | H | CH(Me) | H | *c*-Pr | C(=O)OEt | Cl | CH₂CF₃ | O |
| C-2694 | H | CH₂CH₂ | Me | *c*-Pr | C(=O)OEt | Cl | CH₂CF₃ | O |
| C-2695 | Me | CH₂CH₂ | H | *c*-Pr | C(=O)OEt | Cl | CH₂CF₃ | O |
| C-2696 | H | single bond | Me | *c*-Pr | C(=O)OEt | Me | CH₂CF₃ | O |
| C-2697 | Me | single bond | H | *c*-Pr | C(=O)OEt | Me | CH₂CF₃ | O |
| C-2698 | Me(R) | single bond | H | *c*-Pr | C(=O)OEt | Me | CH₂CF₃ | O |
| C-2699 | Me(S) | single bond | H | *c*-Pr | C(=O)OEt | Me | CH₂CF₃ | O |
| C-2700 | H | CH₂ | Me | *c*-Pr | C(=O)OEt | Me | CH₂CF₃ | O |
| C-2701 | Me | CH₂ | H | *c*-Pr | C(=O)OEt | Me | CH₂CF₃ | O |
| C-2702 | H | CH(Me) | H | *c*-Pr | C(=O)OEt | Me | CH₂CF₃ | O |
| C-2703 | H | CH₂CH₂ | Me | *c*-Pr | C(=O)OEt | Me | CH₂CF₃ | O |
| C-2704 | Me | CH₂CH₂ | H | *c*-Pr | C(=O)OEt | Me | CH₂CF₃ | O |
| C-2705 | H | single bond | Me | *c*-Pr | C(=O)OEt | Cl | CH₂OMe | O |
| C-2706 | Me | single bond | H | *c*-Pr | C(=O)OEt | Cl | CH₂OMe | O |
| C-2707 | Me(R) | single bond | H | *c*-Pr | C(=O)OEt | Cl | CH₂OMe | O |
| C-2708 | Me(S) | single bond | H | *c*-Pr | C(=O)OEt | Cl | CH₂OMe | O |
| C-2709 | H | CH₂ | Me | *c*-Pr | C(=O)OEt | Cl | CH₂OMe | O |
| C-2710 | Me | CH₂ | H | *c*-Pr | C(=O)OEt | Cl | CH₂OMe | O |
| C-2711 | H | CH(Me) | H | *c*-Pr | C(=O)OEt | Cl | CH₂OMe | O |
| C-2712 | H | CH₂CH₂ | Me | *c*-Pr | C(=O)OEt | Cl | CH₂OMe | O |
| C-2713 | Me | CH₂CH₂ | H | *c*-Pr | C(=O)OEt | Cl | CH₂OMe | O |
| C-2714 | H | single bond | Me | *c*-Pr | C(=O)OEt | Me | CH₂OMe | O |
| C-2715 | Me | single bond | H | *c*-Pr | C(=O)OEt | Me | CH₂OMe | O |
| C-2716 | Me(R) | single bond | H | *c*-Pr | C(=O)OEt | Me | CH₂OMe | O |
| C-2717 | Me(S) | single bond | H | *c*-Pr | C(=O)OEt | Me | CH₂OMe | O |
| C-2718 | H | CH₂ | Me | *c*-Pr | C(=O)OEt | Me | CH₂OMe | O |
| C-2719 | Me | CH₂ | H | *c*-Pr | C(=O)OEt | Me | CH₂OMe | O |
| C-2720 | H | CH(Me) | H | *c*-Pr | C(=O)OEt | Me | CH₂OMe | O |
| C-2721 | H | CH₂CH₂ | Me | *c*-Pr | C(=O)OEt | Me | CH₂OMe | O |
| C-2722 | Me | CH₂CH₂ | H | *c*-Pr | C(=O)OEt | Me | CH₂OMe | O |

**[Table 289]**

| Compound Number | Y | W | Z | R¹ | R² | R³ | R¹⁰ | A |
|---|---|---|---|---|---|---|---|---|
| C-2723 | H | single bond | Me | *c*-Pr | C(=O)OEt | Cl | CH₂CN | O |
| C-2724 | Me | single bond | H | *c*-Pr | C(=O)OEt | Cl | CH₂CN | O |
| C-2725 | Me(R) | single bond | H | *c*-Pr | C(=O)OEt | Cl | CH₂CN | O |
| C-2726 | Me(S) | single bond | H | *c*-Pr | C(=O)OEt | Cl | CH₂CN | O |
| C-2727 | H | CH₂ | Me | *c*-Pr | C(=O)OEt | Cl | CH₂CN | O |
| C-2728 | Me | CH₂ | H | *c*-Pr | C(=O)OEt | Cl | CH₂CN | O |
| C-2729 | H | CH(Me) | H | *c*-Pr | C(=O)OEt | Cl | CH₂CN | O |
| C-2730 | H | CH₂CH₂ | Me | *c*-Pr | C(=O)OEt | Cl | CH₂CN | O |
| C-2731 | Me | CH₂CH₂ | H | *c*-Pr | C(=O)OEt | Cl | CH₂CN | O |
| C-2732 | H | single bond | Me | *c*-Pr | C(=O)OEt | Me | CH₂CN | O |
| C-2733 | Me | single bond | H | *c*-Pr | C(=O)OEt | Me | CH₂CN | O |
| C-2734 | Me(R) | single bond | H | *c*-Pr | C(=O)OEt | Me | CH₂CN | O |
| C-2735 | Me(S) | single bond | H | *c*-Pr | C(=O)OEt | Me | CH₂CN | O |
| C-2736 | H | CH₂ | Me | *c*-Pr | C(=O)OEt | Me | CH₂CN | O |
| C-2737 | Me | CH₂ | H | *c*-Pr | C(=O)OEt | Me | CH₂CN | O |
| C-2738 | H | CH(Me) | H | *c*-Pr | C(=O)OEt | Me | CH₂CN | O |
| C-2739 | H | CH₂CH₂ | Me | *c*-Pr | C(=O)OEt | Me | CH₂CN | O |
| C-2740 | Me | CH₂CH₂ | H | *c*-Pr | C(=O)OEt | Me | CH₂CN | O |
| C-2741 | H | single bond | Me | *c*-Pr | C(=O)OEt | Cl | C(=O)Me | O |
| C-2742 | Me | single bond | H | *c*-Pr | C(=O)OEt | Cl | C(=O)Me | O |
| C-2743 | Me(R) | single bond | H | *c*-Pr | C(=O)OEt | Cl | C(=O)Me | O |
| C-2744 | Me(S) | single bond | H | *c*-Pr | C(=O)OEt | Cl | C(=O)Me | O |
| C-2745 | H | CH₂ | Me | *c*-Pr | C(=O)OEt | Cl | C(=O)Me | O |
| C-2746 | Me | CH₂ | H | *c*-Pr | C(=O)OEt | Cl | C(=O)Me | O |
| C-2747 | H | CH(Me) | H | *c*-Pr | C(=O)OEt | Cl | C(=O)Me | O |
| C-2748 | H | CH₂CH₂ | Me | *c*-Pr | C(=O)OEt | Cl | C(=O)Me | O |
| C-2749 | Me | CH₂CH₂ | H | *c*-Pr | C(=O)OEt | Cl | C(=O)Me | O |
| C-2750 | H | single bond | Me | *c*-Pr | C(=O)OEt | Me | C(=O)Me | O |
| C-2751 | Me | single bond | H | *c*-Pr | C(=O)OEt | Me | C(=O)Me | O |
| C-2752 | Me(R) | single bond | H | *c*-Pr | C(=O)OEt | Me | C(=O)Me | O |
| C-2753 | Me(S) | single bond | H | *c*-Pr | C(=O)OEt | Me | C(=O)Me | O |
| C-2754 | H | CH₂ | Me | *c*-Pr | C(=O)OEt | Me | C(=O)Me | O |
| C-2755 | Me | CH₂ | H | *c*-Pr | C(=O)OEt | Me | C(=O)Me | O |
| C-2756 | H | CH(Me) | H | *c*-Pr | C(=O)OEt | Me | C(=O)Me | O |
| C-2757 | H | CH₂CH₂ | Me | *c*-Pr | C(=O)OEt | Me | C(=O)Me | O |
| C-2758 | Me | CH₂CH₂ | H | *c*-Pr | C(=O)OEt | Me | C(=O)Me | O |
| C-2759 | H | single bond | Me | *c*-Pr | S(=O)₂Me | Cl | Me | O |
| C-2760 | H | single bond | Et | *c*-Pr | S(=O)₂Me | Cl | Me | O |
| C-2761 | Me | single bond | H | *c*-Pr | S(=O)₂Me | Cl | Me | O |
| C-2762 | Me(R) | single bond | H | *c*-Pr | S(=O)₂Me | Cl | Me | O |
| C-2763 | Me(S) | single bond | H | *c*-Pr | S(=O)₂Me | Cl | Me | O |
| C-2764 | H | CH₂ | Me | *c*-Pr | S(=O)₂Me | Cl | Me | O |
| C-2765 | Me | CH₂ | H | *c*-Pr | S(=O)₂Me | Cl | Me | O |
| C-2766 | H | CH(Me) | H | *c*-Pr | S(=O)₂Me | Cl | Me | O |

**[Table 290]**

| Compound Number | Y | W | Z | R¹ | R² | R³ | R¹⁰ | A |
|---|---|---|---|---|---|---|---|---|
| C-2767 | H | CH₂CH₂ | Me | *c*-Pr | S(=O)₂Me | Cl | Me | O |
| C-2768 | Me | CH₂CH₂ | H | *c*-Pr | S(=O)₂Me | Cl | Me | O |
| C-2769 | H | single bond | Me | *c*-Pr | S(=O)₂Me | Me | Me | O |
| C-2770 | Me | single bond | H | *c*-Pr | S(=O)₂Me | Me | Me | O |
| C-2771 | Me(R) | single bond | H | *c*-Pr | S(=O)₂Me | Me | Me | O |
| C-2772 | Me(S) | single bond | H | *c*-Pr | S(=O)₂Me | Me | Me | O |
| C-2773 | H | CH₂ | Me | *c*-Pr | S(=O)₂Me | Me | Me | O |
| C-2774 | Me | CH₂ | H | *c*-Pr | S(=O)₂Me | Me | Me | O |
| C-2775 | H | CH(Me) | H | *c*-Pr | S(=O)₂Me | Me | Me | O |
| C-2776 | H | CH₂CH₂ | Me | *c*-Pr | S(=O)₂Me | Me | Me | O |
| C-2777 | Me | CH₂CH₂ | H | *c*-Pr | S(=O)₂Me | Me | Me | O |
| C-2778 | H | single bond | Me | *c*-Pr | S(=O)₂Me | Cl | Et | O |
| C-2779 | Me | single bond | H | *c*-Pr | S(=O)₂Me | Cl | Et | O |
| C-2780 | Me(R) | single bond | H | *c*-Pr | S(=O)₂Me | Cl | Et | O |
| C-2781 | Me(S) | single bond | H | *c*-Pr | S(=O)₂Me | Cl | Et | O |
| C-2782 | H | CH₂ | Me | *c*-Pr | S(=O)₂Me | Cl | Et | O |
| C-2783 | Me | CH₂ | H | *c*-Pr | S(=O)₂Me | Cl | Et | O |
| C-2784 | H | CH(Me) | H | *c*-Pr | S(=O)₂Me | Cl | Et | O |
| C-2785 | H | CH₂CH₂ | Me | *c*-Pr | S(=O)₂Me | Cl | Et | O |
| C-2786 | Me | CH₂CH₂ | H | *c*-Pr | S(=O)₂Me | Cl | Et | O |
| C-2787 | H | single bond | Me | *c*-Pr | S(=O)₂Me | Me | Et | O |
| C-2788 | Me | single bond | H | *c*-Pr | S(=O)₂Me | Me | Et | O |
| C-2789 | Me(R) | single bond | H | *c*-Pr | S(=O)₂Me | Me | Et | O |
| C-2790 | Me(S) | single bond | H | *c*-Pr | S(=O)₂Me | Me | Et | O |
| C-2791 | H | CH₂ | Me | *c*-Pr | S(=O)₂Me | Me | Et | O |
| C-2792 | Me | CH₂ | H | *c*-Pr | S(=O)₂Me | Me | Et | O |
| C-2793 | H | CH(Me) | H | *c*-Pr | S(=O)₂Me | Me | Et | O |
| C-2794 | H | CH₂CH₂ | Me | *c*-Pr | S(=O)₂Me | Me | Et | O |
| C-2795 | Me | CH₂CH₂ | H | *c*-Pr | S(=O)₂Me | Me | Et | O |
| C-2796 | H | single bond | Me | *c*-Pr | S(=O)₂Me | Cl | CH₂CF₃ | O |
| C-2797 | Me | single bond | H | *c*-Pr | S(=O)₂Me | Cl | CH₂CF₃ | O |
| C-2798 | Me(R) | single bond | H | *c*-Pr | S(=O)₂Me | Cl | CH₂CF₃ | O |
| C-2799 | Me(S) | single bond | H | *c*-Pr | S(=O)₂Me | Cl | CH₂CF₃ | O |
| C-2800 | H | CH₂ | Me | *c*-Pr | S(=O)₂Me | Cl | CH₂CF₃ | O |
| C-2801 | Me | CH₂ | H | *c*-Pr | S(=O)₂Me | Cl | CH₂CF₃ | O |
| C-2802 | H | CH(Me) | H | *c*-Pr | S(=O)₂Me | Cl | CH₂CF₃ | O |
| C-2803 | H | CH₂CH₂ | Me | *c*-Pr | S(=O)₂Me | Cl | CH₂CF₃ | O |
| C-2804 | Me | CH₂CH₂ | H | *c*-Pr | S(=O)₂Me | Cl | CH₂CF₃ | O |
| C-2805 | H | single bond | Me | *c*-Pr | S(=O)₂Me | Me | CH₂CF₃ | O |
| C-2806 | Me | single bond | H | *c*-Pr | S(=O)₂Me | Me | CH₂CF₃ | O |
| C-2807 | Me(R) | single bond | H | *c*-Pr | S(=O)₂Me | Me | CH₂CF₃ | O |
| C-2808 | Me(S) | single bond | H | *c*-Pr | S(=O)₂Me | Me | CH₂CF₃ | O |
| C-2809 | H | CH₂ | Me | *c*-Pr | S(=O)₂Me | Me | CH₂CF₃ | O |
| C-2810 | Me | CH₂ | H | *c*-Pr | S(=O)₂Me | Me | CH₂CF₃ | O |

**[Table 291]**

| Compound Number | Y | W | Z | R¹ | R² | R³ | R¹⁰ | A |
|---|---|---|---|---|---|---|---|---|
| C-2811 | H | CH(Me) | H | *c*-Pr | S(=O)₂Me | Me | CH₂CF₃ | O |
| C-2812 | H | CH₂CH₂ | Me | *c*-Pr | S(=O)₂Me | Me | CH₂CF₃ | O |
| C-2813 | Me | CH₂CH₂ | H | *c*-Pr | S(=O)₂Me | Me | CH₂CF₃ | O |
| C-2814 | H | single bond | Me | *c*-Pr | S(=O)₂Me | Cl | CH₂OMe | O |
| C-2815 | Me | single bond | H | *c*-Pr | S(=O)₂Me | Cl | CH₂OMe | O |
| C-2816 | Me(R) | single bond | H | *c*-Pr | S(=O)₂Me | Cl | CH₂OMe | O |
| C-2817 | Me(S) | single bond | H | *c*-Pr | S(=O)₂Me | Cl | CH₂OMe | O |
| C-2818 | H | CH₂ | Me | *c*-Pr | S(=O)₂Me | Cl | CH₂OMe | O |
| C-2819 | Me | CH₂ | H | *c*-Pr | S(=O)₂Me | Cl | CH₂OMe | O |
| C-2820 | H | CH(Me) | H | *c*-Pr | S(=O)₂Me | Cl | CH₂OMe | O |
| C-2821 | H | CH₂CH₂ | Me | *c*-Pr | S(=O)₂Me | Cl | CH₂OMe | O |
| C-2822 | Me | CH₂CH₂ | H | *c*-Pr | S(=O)₂Me | Cl | CH₂OMe | O |
| C-2823 | H | single bond | Me | *c*-Pr | S(=O)₂Me | Me | CH₂OMe | O |
| C-2824 | Me | single bond | H | *c*-Pr | S(=O)₂Me | Me | CH₂OMe | O |
| C-2825 | Me(R) | single bond | H | *c*-Pr | S(=O)₂Me | Me | CH₂OMe | O |
| C-2826 | Me(S) | single bond | H | *c*-Pr | S(=O)₂Me | Me | CH₂OMe | O |
| C-2827 | H | CH₂ | Me | *c*-Pr | S(=O)₂Me | Me | CH₂0Me | O |
| C-2828 | Me | CH₂ | H | *c*-Pr | S(=O)₂Me | Me | CH₂OMe | O |
| C-2829 | H | CH(Me) | H | *c*-Pr | S(=O)₂Me | Me | CH₂OMe | O |
| C-2830 | H | CH₂CH₂ | Me | *c*-Pr | S(=O)₂Me | Me | CH₂OMe | O |
| C-2831 | Me | CH₂CH₂ | H | *c*-Pr | S(=O)₂Me | Me | CH₂OMe | O |
| C-2832 | H | single bond | Me | *c*-Pr | S(=O)₂Me | Cl | CH₂CN | O |
| C-2833 | Me | single bond | H | *c*-Pr | S(=O)₂Me | Cl | CH₂CN | O |
| C-2834 | Me(R) | single bond | H | *c*-Pr | S(=O)₂Me | Cl | CH₂CN | O |
| C-2835 | Me(S) | single bond | H | *c*-Pr | S(=O)₂Me | Cl | CH₂CN | O |
| C-2836 | H | CH₂ | Me | *c*-Pr | S(=O)₂Me | Cl | CH₂CN | O |
| C-2837 | Me | CH₂ | H | *c*-Pr | S(=O)₂Me | Cl | CH₂CN | O |
| C-2838 | H | CH(Me) | H | *c*-Pr | S(=O)₂Me | Cl | CH₂CN | O |
| C-2839 | H | CH₂CH₂ | Me | *c*-Pr | S(=O)₂Me | Cl | CH₂CN | O |
| C-2840 | Me | CH₂CH₂ | H | *c*-Pr | S(=O)₂Me | Cl | CH₂CN | O |
| C-2841 | H | single bond | Me | *c*-Pr | S(=O)₂Me | Me | CH₂CN | O |
| C-2842 | Me | single bond | H | *c*-Pr | S(=O)₂Me | Me | CH₂CN | O |
| C-2843 | Me(R) | single bond | H | *c*-Pr | S(=O)₂Me | Me | CH₂CN | O |
| C-2844 | Me(S) | single bond | H | *c*-Pr | S(=O)₂Me | Me | CH₂CN | O |
| C-2845 | H | CH₂ | Me | *c*-Pr | S(=O)₂Me | Me | CH₂CN | O |
| C-2846 | Me | CH₂ | H | *c*-Pr | S(=O)₂Me | Me | CH₂CN | O |
| C-2847 | H | CH(Me) | H | *c*-Pr | S(=O)₂Me | Me | CH₂CN | O |
| C-2848 | H | CH₂CH₂ | Me | *c*-Pr | S(=O)₂Me | Me | CH₂CN | O |
| C-2849 | Me | CH₂CH₂ | H | *c*-Pr | S(=O)₂Me | Me | CH₂CN | O |
| C-2850 | H | single bond | Me | *c*-Pr | S(=O)₂Me | Cl | C(=O)Me | O |
| C-2851 | Me | single bond | H | *c*-Pr | S(=O)₂Me | Cl | C(=O)Me | O |
| C-2852 | Me(R) | single bond | H | *c*-Pr | S(=O)₂Me | Cl | C(=O)Me | O |
| C-2853 | Me(S) | single bond | H | *c*-Pr | S(=O)₂Me | Cl | C(=O)Me | O |
| C-2854 | H | CH₂ | Me | *c*-Pr | S(=O)₂Me | Cl | C(=O)Me | O |

**[Table 292]**

| Compound Number | Y | W | Z | R¹ | R² | R³ | R¹⁰ | A |
|---|---|---|---|---|---|---|---|---|
| C-2855 | Me | CH₂ | H | *c*-Pr | S(=O)₂Me | Cl | C(=O)Me | O |
| C-2856 | H | CH(Me) | H | *c*-Pr | S(=O)₂Me | Cl | C(=O)Me | O |
| C-2857 | H | CH₂CH₂ | Me | *c*-Pr | S(=O)₂Me | Cl | C(=O)Me | O |
| C-2858 | Me | CH₂CH₂ | H | *c*-Pr | S(=O)₂Me | Cl | C(=O)Me | O |
| C-2859 | H | single bond | Me | *c-*Pr | S(=O)₂Me | Me | C(=O)Me | O |
| C-2860 | Me | single bond | H | *c*-Pr | S(=O)₂Me | Me | C(=O)Me | O |
| C-2861 | Me(R) | single bond | H | *c*-Pr | S(=O)₂Me | Me | C(=O)Me | O |
| C-2862 | Me(S) | single bond | H | *c*-Pr | S(=O)₂Me | Me | C(=O)Me | O |
| C-2863 | H | CH₂ | Me | *c*-Pr | S(=O)₂Me | Me | C(=O)Me | O |
| C-2864 | Me | CH₂ | H | *c*-Pr | S(=O)₂Me | Me | C(=O)Me | O |
| C-2865 | H | CH(Me) | H | *c*-Pr | S(=O)₂Me | Me | C(=O)Me | O |
| C-2866 | H | CH₂CH₂ | Me | *c*-Pr | S(=O)₂Me | Me | C(=O)Me | O |
| C-2867 | Me | CH₂CH₂ | H | *c*-Pr | S(=O)₂Me | Me | C(=O)Me | O |
| C-2868 | H | single bond | Me | *c*-Pr | S(=O)₂CF₃ | Cl | Me | O |
| C-2869 | H | single bond | Et | *c*-Pr | S(=O)₂CF₃ | Cl | Me | O |
| C-2870 | Me | single bond | H | *c*-Pr | S(=O)₂CF₃ | Cl | Me | O |
| C-2871 | Me(R) | single bond | H | *c*-Pr | S(=O)₂CF₃ | Cl | Me | O |
| C-2872 | Me(S) | single bond | H | *c*-Pr | S(=O)₂CF₃ | Cl | Me | O |
| C-2873 | H | CH₂ | Me | *c*-Pr | S(=O)₂CF₃ | Cl | Me | O |
| C-2874 | Me | CH₂ | H | *c*-Pr | S(=O)₂CF₃ | Cl | Me | O |
| C-2875 | H | CH(Me) | H | *c*-Pr | S(=O)₂CF₃ | Cl | Me | O |
| C-2876 | H | CH₂CH₂ | Me | *c*-Pr | S(=O)₂CF₃ | Cl | Me | O |
| C-2877 | Me | CH₂CH₂ | H | *c*-Pr | S(=O)₂CF₃ | Cl | Me | O |
| C-2878 | H | single bond | Me | *c*-Pr | S(=O)₂CF₃ | Me | Me | O |
| C-2879 | Me | single bond | H | *c*-Pr | S(=O)₂CF₃ | Me | Me | O |
| C-2880 | Me(R) | single bond | H | *c*-Pr | S(=O)₂CF₃ | Me | Me | O |
| C-2881 | Me(S) | single bond | H | *c*-Pr | S(=O)₂CF₃ | Me | Me | O |
| C-2882 | H | CH₂ | Me | *c*-Pr | S(=O)₂CF₃ | Me | Me | O |
| C-2883 | Me | CH₂ | H | *c*-Pr | S(=O)₂CF₃ | Me | Me | O |
| C-2884 | H | CH(Me) | H | *c*-Pr | S(=O)₂CF₃ | Me | Me | O |
| C-2885 | H | CH₂CH₂ | Me | *c*-Pr | S(=O)₂CF₃ | Me | Me | O |
| C-2886 | Me | CH₂CH₂ | H | *c*-Pr | S(=O)₂CF₃ | Me | Me | O |
| C-2887 | H | single bond | Me | *c*-Pr | S(=O)₂CF₃ | Cl | Et | O |
| C-2888 | Me | single bond | H | *c*-Pr | S(=O)₂CF₃ | Cl | Et | O |
| C-2889 | Me(R) | single bond | H | *c*-Pr | S(=O)₂CF₃ | Cl | Et | O |
| C-2890 | Me(S) | single bond | H | *c*-Pr | S(=O)₂CF₃ | Cl | Et | O |
| C-2891 | H | CH₂ | Me | *c*-Pr | S(=O)₂CF₃ | Cl | Et | O |
| C-2892 | Me | CH₂ | H | *c*-Pr | S(=O)₂CF₃ | Cl | Et | O |
| C-2893 | H | CH(Me) | H | *c*-Pr | S(=O)₂CF₃ | Cl | Et | O |
| C-2894 | H | CH₂CH₂ | Me | *c*-Pr | S(=O)₂CF₃ | Cl | Et | O |
| C-2895 | Me | CH₂CH₂ | H | *c*-Pr | S(=O)₂CF₃ | Cl | Et | O |
| C-2896 | H | single bond | Me | *c*-Pr | S(=O)₂CF₃ | Me | Et | O |
| C-2897 | Me | single bond | H | *c*-Pr | S(=O)₂CF₃ | Me | Et | O |
| C-2898 | Me(R) | single bond | H | *c*-Pr | S(=O)₂CF₃ | Me | Et | O |

**[Table 293]**

| Compound Number | Y | W | Z | R¹ | R² | R³ | R¹⁰ | A |
|---|---|---|---|---|---|---|---|---|
| C-2899 | Me(S) | single bond | H | *c*-Pr | S(=O)₂CF₃ | Me | Et | O |
| C-2900 | H | CH₂ | Me | *c-*Pr | S(=O)₂CF₃ | Me | Et | O |
| C-2901 | Me | CH₂ | H | *c*-Pr | S(=O)₂CF₃ | Me | Et | O |
| C-2902 | H | CH(Me) | H | *c*-Pr | S(=O)₂CF₃ | Me | Et | O |
| C-2903 | H | CH₂CH₂ | Me | *c*-Pr | S(=O)₂CF₃ | Me | Et | O |
| C-2904 | Me | CH₂CH₂ | H | *c*-Pr | S(=O)₂CF₃ | Me | Et | O |
| C-2905 | H | single bond | Me | *c*-Pr | S(=O)₂CF₃ | Cl | CH₂CF₃ | O |
| C-2906 | Me | single bond | H | *c*-Pr | S(=O)₂CF₃ | Cl | CH₂CF₃ | O |
| C-2907 | Me(R) | single bond | H | *c*-Pr | S(=O)₂CF₃ | Cl | CH₂CF₃ | O |
| C-2908 | Me(S) | single bond | H | *c*-Pr | S(=O)₂CF₃ | Cl | CH₂CF₃ | O |
| C-2909 | H | CH₂ | Me | *c*-Pr | S(=O)₂CF₃ | Cl | CH₂CF₃ | O |
| C-2910 | Me | CH₂ | H | *c*-Pr | S(=O)₂CF₃ | Cl | CH₂CF₃ | O |
| C-2911 | H | CH(Me) | H | *c*-Pr | S(=O)₂CF₃ | Cl | CH₂CF₃ | O |
| C-2912 | H | CH₂CH₂ | Me | *c*-Pr | S(=O)₂CF₃ | Cl | CH₂CF₃ | O |
| C-2913 | Me | CH₂CH₂ | H | *c*-Pr | S(=O)₂CF₃ | Cl | CH₂CF₃ | O |
| C-2914 | H | single bond | Me | *c*-Pr | S(=O)₂CF₃ | Me | CH₂CF₃ | O |
| C-2915 | Me | single bond | H | *c*-Pr | S(=O)₂CF₃ | Me | CH₂CF₃ | O |
| C-2916 | Me(R) | single bond | H | *c*-Pr | S(=O)₂CF₃ | Me | CH₂CF₃ | O |
| C-2917 | Me(S) | single bond | H | *c*-Pr | S(=O)₂CF₃ | Me | CH₂CF₃ | O |
| C-2918 | H | CH₂ | Me | *c*-Pr | S(=O)₂CF₃ | Me | CH₂CF₃ | O |
| C-2919 | Me | CH₂ | H | *c*-Pr | S(=O)₂CF₃ | Me | CH₂CF₃ | O |
| C-2920 | H | CH(Me) | H | *c*-Pr | S(=O)₂CF₃ | Me | CH₂CF₃ | O |
| C-2921 | H | CH₂CH₂ | Me | *c*-Pr | S(=O)₂CF₃ | Me | CH₂CF₃ | O |
| C-2922 | Me | CH₂CH₂ | H | *c*-Pr | S(=O)₂CF₃ | Me | CH₂CF₃ | O |
| C-2923 | H | single bond | Me | *c*-Pr | S(=O)₂CF₃ | Cl | CH₂OMe | O |
| C-2924 | Me | single bond | H | *c*-Pr | S(=O)₂CF₃ | Cl | CH₂OMe | O |
| C-2925 | Me(R) | single bond | H | *c*-Pr | S(=O)₂CF₃ | Cl | CH₂OMe | O |
| C-2926 | Me(S) | single bond | H | *c*-Pr | S(=O)₂CF₃ | Cl | CH₂OMe | O |
| C-2927 | H | CH₂ | Me | *c*-Pr | S(=O)₂CF₃ | Cl | CH₂OMe | O |
| C-2928 | Me | CH₂ | H | *c*-Pr | S(=O)₂CF₃ | Cl | CH₂OMe | O |
| C-2929 | H | CH(Me) | H | *c*-Pr | S(=O)₂CF₃ | Cl | CH₂OMe | O |
| C-2930 | H | CH₂CH₂ | Me | *c*-Pr | S(=O)₂CF₃ | Cl | CH₂OMe | O |
| C-2931 | Me | CH₂CH₂ | H | *c*-Pr | S(=O)₂CF₃ | Cl | CH₂OMe | O |
| C-2932 | H | single bond | Me | *c*-Pr | S(=O)₂CF₃ | Me | CH₂OMe | O |
| C-2933 | Me | single bond | H | *c*-Pr | S(=O)₂CF₃ | Me | CH₂OMe | O |
| C-2934 | Me(R) | single bond | H | *c*-Pr | S(=O)₂CF₃ | Me | CH₂OMe | O |
| C-2935 | Me(S) | single bond | H | *c*-Pr | S(=O)₂CF₃ | Me | CH₂OMe | O |
| C-2936 | H | CH₂ | Me | *c*-Pr | S(=O)₂CF₃ | Me | CH₂OMe | O |
| C-2937 | Me | CH₂ | H | *c*-Pr | S(=O)₂CF₃ | Me | CH₂OMe | O |
| C-2938 | H | CH(Me) | H | *c*-Pr | S(=O)₂CF₃ | Me | CH₂OMe | O |
| C-2939 | H | CH₂CH₂ | Me | *c*-Pr | S(=O)₂CF₃ | Me | CH₂OMe | O |
| C-2940 | Me | CH₂CH₂ | H | *c*-Pr | S(=O)₂CF₃ | Me | CH₂OMe | O |
| C-2941 | H | single bond | Me | *c*-Pr | S(=O)₂CF₃ | Cl | CH₂CN | O |
| C-2942 | Me | single bond | H | *c*-Pr | S(=O)₂CF₃ | Cl | CH₂CN | O |

**[Table 294]**

| Compound Number | Y | W | Z | R¹ | R² | R³ | R¹⁰ | A |
|---|---|---|---|---|---|---|---|---|
| C-2943 | Me(R) | single bond | H | *c*-Pr | S(=O)₂CF₃ | Cl | CH₂CN | O |
| C-2944 | Me(S) | single bond | H | *c*-Pr | S(=O)₂CF₃ | Cl | CH₂CN | O |
| C-2945 | H | CH₂ | Me | *c*-Pr | S(=O)₂CF₃ | Cl | CH₂CN | O |
| C-2946 | Me | CH₂ | H | *c*-Pr | S(=O)₂CF₃ | Cl | CH₂CN | O |
| C-2947 | H | CH(Me) | H | *c*-Pr | S(=O)₂CF₃ | Cl | CH₂CN | O |
| C-2948 | H | CH₂CH₂ | Me | *c*-Pr | S(=O)₂CF₃ | Cl | CH₂CN | O |
| C-2949 | Me | CH₂CH₂ | H | *c*-Pr | S(=O)₂CF₃ | Cl | CH₂CN | O |
| C-2950 | H | single bond | Me | *c*-Pr | S(=O)₂CF₃ | Me | CH₂CN | O |
| C-2951 | Me | single bond | H | *c*-Pr | S(=O)₂CF₃ | Me | CH₂CN | O |
| C-2952 | Me(R) | single bond | H | *c*-Pr | S(=O)₂CF₃ | Me | CH₂CN | O |
| C-2953 | Me(S) | single bond | H | *c*-Pr | S(=O)₂CF₃ | Me | CH₂CN | O |
| C-2954 | H | CH₂ | Me | *c*-Pr | S(=O)₂CF₃ | Me | CH₂CN | O |
| C-2955 | Me | CH₂ | H | *c*-Pr | S(=O)₂CF₃ | Me | CH₂CN | O |
| C-2956 | H | CH(Me) | H | *c*-Pr | S(=O)₂CF₃ | Me | CH₂CN | O |
| C-2957 | H | CH₂CH₂ | Me | *c*-Pr | S(=O)₂CF₃ | Me | CH₂CN | O |
| C-2958 | Me | CH₂CH₂ | H | *c*-Pr | S(=O)₂CF₃ | Me | CH₂CN | O |
| C-2959 | H | single bond | Me | *c*-Pr | S(=O)₂CF₃ | Cl | C(=O)Me | O |
| C-2960 | Me | single bond | H | *c*-Pr | S(=O)₂CF₃ | Cl | C(=O)Me | O |
| C-2961 | Me(R) | single bond | H | *c*-Pr | S(=O)₂CF₃ | Cl | C(=O)Me | O |
| C-2962 | Me(S) | single bond | H | *c*-Pr | S(=O)₂CF₃ | Cl | C(=O)Me | O |
| C-2963 | H | CH₂ | Me | *c*-Pr | S(=O)₂CF₃ | Cl | C(=O)Me | O |
| C-2964 | Me | CH₂ | H | *c*-Pr | S(=O)₂CF₃ | Cl | C(=O)Me | O |
| C-2965 | H | CH(Me) | H | *c*-Pr | S(=O)₂CF₃ | Cl | C(=O)Me | O |
| C-2966 | H | CH₂CH₂ | Me | *c*-Pr | S(=O)₂CF₃ | Cl | C(=O)Me | O |
| C-2967 | Me | CH₂CH₂ | H | *c*-Pr | S(=O)₂CF₃ | Cl | C(=O)Me | O |
| C-2968 | H | single bond | Me | *c*-Pr | S(=O)₂CF₃ | Me | C(=O)Me | O |
| C-2969 | Me | single bond | H | *c*-Pr | S(=O)₂CF₃ | Me | C(=O)Me | O |
| C-2970 | Me(R) | single bond | H | *c*-Pr | S(=O)₂CF₃ | Me | C(=O)Me | O |
| C-2971 | Me(S) | single bond | H | *c*-Pr | S(=O)₂CF₃ | Me | C(=O)Me | O |
| C-2972 | H | CH₂ | Me | *c*-Pr | S(=O)₂CF₃ | Me | C(=O)Me | O |
| C-2973 | Me | CH₂ | H | *c*-Pr | S(=O)₂CF₃ | Me | C(=O)Me | O |
| C-2974 | H | CH(Me) | H | *c*-Pr | S(=O)₂CF₃ | Me | C(=O)Me | O |
| C-2975 | H | CH₂CH₂ | Me | *c*-Pr | S(=O)₂CF₃ | Me | C(=O)Me | O |
| C-2976 | Me | CH₂CH₂ | H | *c*-Pr | S(=O)₂CF₃ | Me | C(=O)Me | O |
| C-2977 | H | single bond | Me | *c*-Pr(1-CN) | H | Cl | H | O |
| C-2978 | Me | single bond | H | *c*-Pr(1-CN) | H | Cl | H | O |
| C-2979 | Me(R) | single bond | H | *c*-Pr(1-CN) | H | Cl | H | O |
| C-2980 | Me(S) | single bond | H | *c*-Pr(1-CN) | H | Cl | H | O |
| C-2981 | Me | single bond | H | *c*-Pr(1-CN) | H | Cl | H | S |
| C-2982 | Me(R) | single bond | H | *c*-Pr(1-CN) | H | Cl | H | S |
| C-2983 | Me(S) | single bond | H | *c*-Pr(1-CN) | H | Cl | H | S |
| C-2984 | H | CH₂ | Me | *c*-Pr(1-CN) | H | Cl | H | O |
| C-2985 | Me | CH₂ | H | *c*-Pr(1-CN) | H | Cl | H | O |
| C-2986 | H | CH(Me) | H | *c*-Pr(1-CN) | H | Cl | H | O |

**[Table 295]**

| Compound Number | Y | W | Z | R¹ | R² | R³ | R¹⁰ | A |
|---|---|---|---|---|---|---|---|---|
| C-2987 | H | CH₂CH₂ | Me | c-Pr(1-CN) | H | Cl | H | O |
| C-2988 | Me | CH₂CH₂ | H | c-Pr(1-CN) | H | Cl | H | O |
| C-2989 | H | single bond | Me | c-Pr(1-CN) | H | Me | H | O |
| C-2990 | Me | single bond | H | c-Pr(1-CN) | H | Me | H | O |
| C-2991 | Me(R) | single bond | H | c-Pr(1-CN) | H | Me | H | O |
| C-2992 | Me(S) | single bond | H | c-Pr(1-CN) | H | Me | H | O |
| C-2993 | Me | single bond | H | c-Pr(1-CN) | H | Me | H | S |
| C-2994 | Me(R) | single bond | H | c-Pr(1-CN) | H | Me | H | S |
| C-2995 | Me(S) | single bond | H | c-Pr(1-CN) | H | Me | H | S |
| C-2996 | H | CH₂ | Me | c-Pr(1-CN) | H | Me | H | O |
| C-2997 | Me | CH₂ | H | c-Pr(1-CN) | H | Me | H | O |
| C-2998 | H | CH(Me) | H | c-Pr(1-CN) | H | Me | H | O |
| C-2999 | H | CH₂CH₂ | Me | c-Pr(1-CN) | H | Me | H | O |
| C-3000 | Me | CH₂CH₂ | H | c-Pr(1-CN) | H | Me | H | O |
| C-3001 | H | single bond | Me | c-Pr(1-CN) | H | Cl | Me | O |
| C-3002 | Me | single bond | H | c-Pr(1-CN) | H | Cl | Me | O |
| C-3003 | Me(R) | single bond | H | c-Pr(1-CN) | H | Cl | Me | O |
| C-3004 | Me(S) | single bond | H | c-Pr(1-CN) | H | Cl | Me | O |
| C-3005 | Me | single bond | H | c-Pr(1-CN) | H | Cl | Me | S |
| C-3006 | Me(R) | single bond | H | c-Pr(1-CN) | H | Cl | Me | S |
| C-3007 | Me(S) | single bond | H | c-Pr(1-CN) | H | Cl | Me | S |
| C-3008 | H | CH₂ | Me | c-Pr(1-CN) | H | Cl | Me | O |
| C-3009 | Me | CH₂ | H | c-Pr(1-CN) | H | Cl | Me | O |
| C-3010 | H | CH(Me) | H | c-Pr(1-CN) | H | Cl | Me | O |
| C-3011 | H | CH₂CH₂ | Me | c-Pr(1-CN) | H | Cl | Me | O |
| C-3012 | Me | CH₂CH₂ | H | c-Pr(1-CN) | H | Cl | Me | O |
| C-3013 | H | single bond | Me | c-Pr(1-CN) | H | Me | Me | O |
| C-3014 | Me | single bond | H | c-Pr(1-CN) | H | Me | Me | O |
| C-3015 | Me(R) | single bond | H | c-Pr(1-CN) | H | Me | Me | O |
| C-3016 | Me(S) | single bond | H | c-Pr(1-CN) | H | Me | Me | O |
| C-3017 | Me | single bond | H | c-Pr(1-CN) | H | Me | Me | S |
| C-3018 | Me(R) | single bond | H | c-Pr(1-CN) | H | Me | Me | S |
| C-3019 | Me(S) | single bond | H | c-Pr(1-CN) | H | Me | Me | S |
| C-3020 | H | CH₂ | Me | c-Pr(1-CN) | H | Me | Me | O |
| C-3021 | Me | CH₂ | H | c-Pr(1-CN) | H | Me | Me | O |
| C-3022 | H | CH(Me) | H | c-Pr(1-CN) | H | Me | Me | O |
| C-3023 | H | CH₂CH₂ | Me | c-Pr(1-CN) | H | Me | Me | O |
| C-3024 | Me | CH₂CH₂ | H | c-Pr(1-CN) | H | Me | Me | O |
| C-3025 | H | single bond | Me | c-Pr(1-CN) | H | Cl | Et | O |
| C-3026 | Me | single bond | H | c-Pr(1-CN) | H | Cl | Et | O |
| C-3027 | Me(R) | single bond | H | c-Pr(1-CN) | H | Cl | Et | O |
| C-3028 | Me(S) | single bond | H | c-Pr(1-CN) | H | Cl | Et | O |
| C-3029 | Me | single bond | H | c-Pr(1-CN) | H | Cl | Et | S |
| C-3030 | Me(R) | single bond | H | c-Pr(1-CN) | H | Cl | Et | S |

**[Table 296]**

| Compound Number | Y | W | Z | R¹ | R² | R³ | R¹⁰ | A |
|---|---|---|---|---|---|---|---|---|
| C-3031 | Me(S) | single bond | H | c-Pr(1-CN) | H | Cl | Et | S |
| C-3032 | H | CH₂ | Me | c-Pr(1-CN) | H | Cl | Et | O |
| C-3033 | Me | CH₂ | H | c-Pr(1-CN) | H | Cl | Et | O |
| C-3034 | H | CH(Me) | H | c-Pr(1-CN) | H | Cl | Et | O |
| C-3035 | H | CH₂CH₂ | Me | c-Pr(1-CN) | H | Cl | Et | O |
| C-3036 | Me | CH₂CH₂ | H | c-Pr(1-CN) | H | Cl | Et | O |
| C-3037 | H | single bond | Me | c-Pr(1-CN) | H | Me | Et | O |
| C-3038 | Me | single bond | H | c-Pr(1-CN) | H | Me | Et | O |
| C-3039 | Me(R) | single bond | H | c-Pr(1-CN) | H | Me | Et | O |
| C-3040 | Me(S) | single bond | H | c-Pr(1-CN) | H | Me | Et | O |
| C-3041 | Me | single bond | H | c-Pr(1-CN) | H | Me | Et | S |
| C-3042 | Me(R) | single bond | H | c-Pr(1-CN) | H | Me | Et | S |
| C-3043 | Me(S) | single bond | H | c-Pr(1-CN) | H | Me | Et | S |
| C-3044 | H | CH₂ | Me | c-Pr(1-CN) | H | Me | Et | O |
| C-3045 | Me | CH₂ | H | c-Pr(1-CN) | H | Me | Et | O |
| C-3046 | H | CH(Me) | H | c-Pr(1-CN) | H | Me | Et | O |
| C-3047 | H | CH₂CH₂ | Me | c-Pr(1-CN) | H | Me | Et | O |
| C-3048 | Me | CH₂CH₂ | H | c-Pr(1-CN) | H | Me | Et | O |
| C-3049 | H | single bond | Me | c-Pr(1-CN) | H | Cl | CH₂CF₃ | O |
| C-3050 | Me | single bond | H | c-Pr(1-CN) | H | Cl | CH₂CF₃ | O |
| C-3051 | Me(R) | single bond | H | c-Pr(1-CN) | H | Cl | CH₂CF₃ | O |
| C-3052 | Me(S) | single bond | H | c-Pr(1-CN) | H | Cl | CH₂CF₃ | O |
| C-3053 | Me | single bond | H | c-Pr(1-CN) | H | Cl | CH₂CF₃ | S |
| C-3054 | Me(R) | single bond | H | c-Pr(1-CN) | H | Cl | CH₂CF₃ | S |
| C-3055 | Me(S) | single bond | H | c-Pr(1-CN) | H | Cl | CH₂CF₃ | S |
| C-3056 | H | CH₂ | Me | c-Pr(1-CN) | H | Cl | CH₂CF₃ | O |
| C-3057 | Me | CH₂ | H | c-Pr(1-CN) | H | Cl | CH₂CF₃ | O |
| C-3058 | H | CH(Me) | H | c-Pr(1-CN) | H | Cl | CH₂CF₃ | O |
| C-3059 | H | CH₂CH₂ | Me | c-Pr(1-CN) | H | Cl | CH₂CF₃ | O |
| C-3060 | Me | CH₂CH₂ | H | c-Pr(1-CN) | H | Cl | CH₂CF₃ | O |
| C-3061 | H | single bond | Me | c-Pr(1-CN) | H | Me | CH₂CF₃ | O |
| C-3062 | Me | single bond | H | c-Pr(1-CN) | H | Me | CH₂CF₃ | O |
| C-3063 | Me(R) | single bond | H | c-Pr(1-CN) | H | Me | CH₂CF₃ | O |
| C-3064 | Me(S) | single bond | H | c-Pr(1-CN) | H | Me | CH₂CF₃ | O |
| C-3065 | Me | single bond | H | c-Pr(1-CN) | H | Me | CH₂CF₃ | S |
| C-3066 | Me(R) | single bond | H | c-Pr(1-CN) | H | Me | CH₂CF₃ | S |
| C-3067 | Me(S) | single bond | H | c-Pr(1-CN) | H | Me | CH₂CF₃ | S |
| C-3068 | H | CH₂ | Me | c-Pr(1-CN) | H | Me | CH₂CF₃ | O |
| C-3069 | Me | CH₂ | H | c-Pr(1-CN) | H | Me | CH₂CF₃ | O |
| C-3070 | H | CH(Me) | H | c-Pr(1-CN) | H | Me | CH₂CF₃ | O |
| C-3071 | H | CH₂CH₂ | Me | c-Pr(1-CN) | H | Me | CH₂CF₃ | O |
| C-3072 | Me | CH₂CH₂ | H | c-Pr(1-CN) | H | Me | CH₂CF₃ | O |
| C-3073 | H | single bond | Me | c-Pr(1-CN) | H | Cl | CH₂OMe | O |
| C-3074 | Me | single bond | H | c-Pr(1-CN) | H | Cl | CH₂OMe | O |

**[Table 297]**

| Compound Number | Y | W | Z | R¹ | R² | R³ | R¹⁰ | A |
|---|---|---|---|---|---|---|---|---|
| C-3075 | Me(R) | single bond | H | c-Pr(1-CN) | H | Cl | CH₂OMe | O |
| C-3076 | Me(S) | single bond | H | c-Pr(1-CN) | H | Cl | CH₂OMe | O |
| C-3077 | Me | single bond | H | c-Pr(1-CN) | H | Cl | CH₂OMe | S |
| C-3078 | Me(R) | single bond | H | c-Pr(1-CN) | H | Cl | CH₂OMe | S |
| C-3079 | Me(S) | single bond | H | c-Pr(1-CN) | H | Cl | CH₂OMe | S |
| C-3080 | H | CH₂ | Me | c-Pr(1-CN) | H | Cl | CH₂OMe | O |
| C-3081 | Me | CH₂ | H | c-Pr(1-CN) | H | Cl | CH₂OMe | O |
| C-3082 | H | CH(Me) | H | c-Pr(1-CN) | H | Cl | CH₂OMe | O |
| C-3083 | H | CH₂CH₂ | Me | c-Pr(1-CN) | H | Cl | CH₂OMe | O |
| C-3084 | Me | CH₂CH₂ | H | c-Pr(1-CN) | H | Cl | CH₂OMe | O |
| C-3085 | H | single bond | Me | c-Pr(1-CN) | H | Me | CH₂OMe | O |
| C-3086 | Me | single bond | H | c-Pr(1-CN) | H | Me | CH₂OMe | O |
| C-3087 | Me(R) | single bond | H | c-Pr(1-CN) | H | Me | CH₂OMe | O |
| C-3088 | Me(S) | single bond | H | c-Pr(1-CN) | H | Me | CH₂OMe | O |
| C-3089 | Me | single bond | H | c-Pr(1-CN) | H | Me | CH₂OMe | S |
| C-3090 | Me(R) | single bond | H | c-Pr(1-CN) | H | Me | CH₂OMe | S |
| C-3091 | Me(S) | single bond | H | c-Pr(1-CN) | H | Me | CH₂OMe | S |
| C-3092 | H | CH₂ | Me | c-Pr(1-CN) | H | Me | CH₂OMe | O |
| C-3093 | Me | CH₂ | H | c-Pr(1-CN) | H | Me | CH₂OMe | O |
| C-3094 | H | CH(Me) | H | c-Pr(1-CN) | H | Me | CH₂OMe | O |
| C-3095 | H | CH₂CH₂ | Me | c-Pr(1-CN) | H | Me | CH₂OMe | O |
| C-3096 | Me | CH₂CH₂ | H | c-Pr(1-CN) | H | Me | CH₂OMe | O |
| C-3097 | H | single bond | Me | c-Pr(1-CN) | H | Cl | CH₂CN | O |
| C-3098 | Me | single bond | H | c-Pr(1-CN) | H | Cl | CH₂CN | O |
| C-3099 | Me(R) | single bond | H | c-Pr(1-CN) | H | Cl | CH₂CN | O |
| C-3100 | Me(S) | single bond | H | c-Pr(1-CN) | H | Cl | CH₂CN | O |
| C-3101 | Me | single bond | H | c-Pr(1-CN) | H | Cl | CH₂CN | S |
| C-3102 | Me(R) | single bond | H | c-Pr(1-CN) | H | Cl | CH₂CN | S |
| C-3103 | Me(S) | single bond | H | c-Pr(1-CN) | H | Cl | CH₂CN | S |
| C-3104 | H | CH₂ | Me | c-Pr(1-CN) | H | Cl | CH₂CN | O |
| C-3105 | Me | CH₂ | H | c-Pr(1-CN) | H | Cl | CH₂CN | O |
| C-3106 | H | CH(Me) | H | c-Pr(1-CN) | H | Cl | CH₂CN | O |
| C-3107 | H | CH₂CH₂ | Me | c-Pr(1-CN) | H | Cl | CH₂CN | O |
| C-3108 | Me | CH₂CH₂ | H | c-Pr(1-CN) | H | Cl | CH₂CN | O |
| C-3109 | H | single bond | Me | c-Pr(1-CN) | H | Me | CH₂CN | O |
| C-3110 | Me | single bond | H | c-Pr(1-CN) | H | Me | CH₂CN | O |
| C-3111 | Me(R) | single bond | H | c-Pr(1-CN) | H | Me | CH₂CN | O |
| C-3112 | Me(S) | single bond | H | c-Pr(1-CN) | H | Me | CH₂CN | O |
| C-3113 | Me | single bond | H | c-Pr(1-CN) | H | Me | CH₂CN | S |
| C-3114 | Me(R) | single bond | H | c-Pr(1-CN) | H | Me | CH₂CN | S |
| C-3115 | Me(S) | single bond | H | c-Pr(1-CN) | H | Me | CH₂CN | S |
| C-3116 | H | CH₂ | Me | c-Pr(1-CN) | H | Me | CH₂CN | O |
| C-3117 | Me | CH₂ | H | c-Pr(1-CN) | H | Me | CH₂CN | O |
| C-3118 | H | CH(Me) | H | c-Pr(1-CN) | H | Me | CH₂CN | O |

**[Table 298]**

| Compound Number | Y | W | Z | R¹ | R² | R³ | R¹⁰ | A |
|---|---|---|---|---|---|---|---|---|
| C-3119 | H | CH₂CH₂ | Me | c-Pr(1-CN) | H | Me | CH₂CN | O |
| C-3120 | Me | CH₂CH₂ | H | c-Pr(1-CN) | H | Me | CH₂CN | O |
| C-3121 | H | single bond | Me | c-Pr(1-CN) | H | Cl | C(=O)Me | O |
| C-3122 | Me | single bond | H | c-Pr(1-CN) | H | Cl | C(=O)Me | O |
| C-3123 | Me(R) | single bond | H | c-Pr(1-CN) | H | Cl | C(=O)Me | O |
| C-3124 | Me(S) | single bond | H | c-Pr(1-CN) | H | Cl | C(=O)Me | O |
| C-3125 | Me | single bond | H | c-Pr(1-CN) | H | Cl | C(=O)Me | S |
| C-3126 | Me(R) | single bond | H | c-Pr(1-CN) | H | Cl | C(=O)Me | S |
| C-3127 | Me(S) | single bond | H | c-Pr(1-CN) | H | Cl | C(=O)Me | S |
| C-3128 | H | CH₂ | Me | c-Pr(1-CN) | H | Cl | C(=O)Me | O |
| C-3129 | Me | CH₂ | H | c-Pr(1-CN) | H | Cl | C(=O)Me | O |
| C-3130 | H | CH(Me) | H | c-Pr(1-CN) | H | Cl | C(=O)Me | O |
| C-3131 | H | CH₂CH₂ | Me | c-Pr(1-CN) | H | Cl | C(=O)Me | O |
| C-3132 | Me | CH₂CH₂ | H | c-Pr(1-CN) | H | Cl | C(=O)Me | O |
| C-3133 | H | single bond | Me | c-Pr(1-CN) | H | Me | C(=O)Me | O |
| C-3134 | Me | single bond | H | c-Pr(1-CN) | H | Me | C(=O)Me | O |
| C-3135 | Me(R) | single bond | H | c-Pr(1-CN) | H | Me | C(=O)Me | O |
| C-3136 | Me(S) | single bond | H | c-Pr(1-CN) | H | Me | C(=O)Me | O |
| C-3137 | Me | single bond | H | c-Pr(1-CN) | H | Me | C(=O)Me | S |
| C-3138 | Me(R) | single bond | H | c-Pr(1-CN) | H | Me | C(=O)Me | S |
| C-3139 | Me(S) | single bond | H | c-Pr(1-CN) | H | Me | C(=O)Me | S |
| C-3140 | H | CH₂ | Me | c-Pr(1-CN) | H | Me | C(=O)Me | O |
| C-3141 | Me | CH₂ | H | c-Pr(1-CN) | H | Me | C(=O)Me | O |
| C-3142 | H | CH(Me) | H | c-Pr(1-CN) | H | Me | C(=O)Me | O |
| C-3143 | H | CH₂CH₂ | Me | c-Pr(1-CN) | H | Me | C(=O)Me | O |
| C-3144 | Me | CH₂CH₂ | H | c-Pr(1-CN) | H | Me | C(=O)Me | O |
| C-3145 | H | single bond | Me | c-Pr(1-CN) | H | Cl | C(=O)OEt | O |
| C-3146 | Me | single bond | H | c-Pr(1-CN) | H | Cl | C(=O)OEt | O |
| C-3147 | Me(R) | single bond | H | c-Pr(1-CN) | H | Cl | C(=O)OEt | O |
| C-3148 | Me(S) | single bond | H | c-Pr(1-CN) | H | Cl | C(=O)OEt | O |
| C-3149 | Me | single bond | H | c-Pr(1-CN) | H | Cl | C(=O)OEt | S |
| C-3150 | Me(R) | single bond | H | c-Pr(1-CN) | H | Cl | C(=O)OEt | S |
| C-3151 | Me(S) | single bond | H | c-Pr(1-CN) | H | Cl | C(=O)OEt | S |
| C-3152 | H | CH₂ | Me | c-Pr(1-CN) | H | Cl | C(=O)OEt | O |
| C-3153 | Me | CH₂ | H | c-Pr(1-CN) | H | Cl | C(=O)OEt | O |
| C-3154 | H | CH(Me) | H | c-Pr(1-CN) | H | Cl | C(=O)OEt | O |
| C-3155 | H | CH₂CH₂ | Me | c-Pr(1-CN) | H | Cl | C(=O)OEt | O |
| C-3156 | Me | CH₂CH₂ | H | c-Pr(1-CN) | H | Cl | C(=O)OEt | O |
| C-3157 | H | single bond | Me | c-Pr(1-CN) | H | Me | C(=O)OEt | O |
| C-3158 | Me | single bond | H | c-Pr(1-CN) | H | Me | C(=O)OEt | O |
| C-3159 | Me(R) | single bond | H | c-Pr(1-CN) | H | Me | C(=O)OEt | O |
| C-3160 | Me(S) | single bond | H | c-Pr(1-CN) | H | Me | C(=O)OEt | O |
| C-3161 | Me | single bond | H | c-Pr(1-CN) | H | Me | C(=O)OEt | S |
| C-3162 | Me(R) | single bond | H | c-Pr(1-CN) | H | Me | C(=O)OEt | S |

**[Table 299]**

| Compound Number | Y | W | Z | R¹ | R² | R³ | R¹⁰ | A |
|---|---|---|---|---|---|---|---|---|
| C-3163 | Me(S) | single bond | H | c-Pr(1-CN) | H | Me | C(=O)OEt | S |
| C-3164 | H | CH₂ | Me | c-Pr(1-CN) | H | Me | C(=O)OEt | O |
| C-3165 | Me | CH₂ | H | c-Pr(1-CN) | H | Me | C(=O)OEt | O |
| C-3166 | H | CH(Me) | H | c-Pr(1-CN) | H | Me | C(=O)OEt | O |
| C-3167 | H | CH₂CH₂ | Me | c-Pr(1-CN) | H | Me | C(=O)OEt | O |
| C-3168 | Me | CH₂CH₂ | H | c-Pr(1-CN) | H | Me | C(=O)OEt | O |
| C-3169 | H | single bond | Me | c-Pr(1-CN) | Me | Cl | Me | O |
| C-3170 | Me | single bond | H | c-Pr(1-CN) | Me | Cl | Me | O |
| C-3171 | Me(R) | single bond | H | c-Pr(1-CN) | Me | Cl | Me | O |
| C-3172 | Me(S) | single bond | H | c-Pr(1-CN) | Me | Cl | Me | O |
| C-3173 | H | CH₂ | Me | c-Pr(1-CN) | Me | Cl | Me | O |
| C-3174 | Me | CH₂ | H | c-Pr(1-CN) | Me | Cl | Me | O |
| C-3175 | H | CH(Me) | H | c-Pr(1-CN) | Me | Cl | Me | O |
| C-3176 | H | CH₂CH₂ | Me | c-Pr(1-CN) | Me | Cl | Me | O |
| C-3177 | Me | CH₂CH₂ | H | c-Pr(1-CN) | Me | Cl | Me | O |
| C-3178 | H | single bond | Me | c-Pr(1-CN) | Me | Me | Me | O |
| C-3179 | Me | single bond | H | c-Pr(1-CN) | Me | Me | Me | O |
| C-3180 | Me(R) | single bond | H | c-Pr(1-CN) | Me | Me | Me | O |
| C-3181 | Me(S) | single bond | H | c-Pr(1-CN) | Me | Me | Me | O |
| C-3182 | H | CH₂ | Me | c-Pr(1-CN) | Me | Me | Me | O |
| C-3183 | Me | CH₂ | H | c-Pr(1-CN) | Me | Me | Me | O |
| C-3184 | H | CH(Me) | H | c-Pr(1-CN) | Me | Me | Me | O |
| C-3185 | H | CH₂CH₂ | Me | c-Pr(1-CN) | Me | Me | Me | O |
| C-3186 | Me | CH₂CH₂ | H | c-Pr(1-CN) | Me | Me | Me | O |
| C-3187 | H | single bond | Me | c-Pr(1-CN) | Me | Cl | Et | O |
| C-3188 | Me | single bond | H | c-Pr(1-CN) | Me | Cl | Et | O |
| C-3189 | Me(R) | single bond | H | c-Pr(1-CN) | Me | Cl | Et | O |
| C-3190 | Me(S) | single bond | H | c-Pr(1-CN) | Me | Cl | Et | O |
| C-3191 | H | CH₂ | Me | c-Pr(1-CN) | Me | Cl | Et | O |
| C-3192 | Me | CH₂ | H | c-Pr(1-CN) | Me | Cl | Et | O |
| C-3193 | H | CH(Me) | H | c-Pr(1-CN) | Me | Cl | Et | O |
| C-3194 | H | CH₂CH₂ | Me | c-Pr(1-CN) | Me | Cl | Et | O |
| C-3195 | Me | CH₂CH₂ | H | c-Pr(1-CN) | Me | Cl | Et | O |
| C-3196 | H | single bond | Me | c-Pr(1-CN) | Me | Me | Et | O |
| C-3197 | Me | single bond | H | c-Pr(1-CN) | Me | Me | Et | O |
| C-3198 | Me(R) | single bond | H | c-Pr(1-CN) | Me | Me | Et | O |
| C-3199 | Me(S) | single bond | H | c-Pr(1-CN) | Me | Me | Et | O |
| C-3200 | H | CH₂ | Me | c-Pr(1-CN) | Me | Me | Et | O |
| C-3201 | Me | CH₂ | H | c-Pr(1-CN) | Me | Me | Et | O |
| C-3202 | H | CH(Me) | H | c-Pr(1-CN) | Me | Me | Et | O |
| C-3203 | H | CH₂CH₂ | Me | c-Pr(1-CN) | Me | Me | Et | O |
| C-3204 | Me | CH₂CH₂ | H | c-Pr(1-CN) | Me | Me | Et | O |
| C-3205 | H | single bond | Me | c-Pr(1-CN) | Me | Cl | CH₂CF₃ | O |
| C-3206 | Me | single bond | H | c-Pr(1-CN) | Me | Cl | CH₂CF₃ | O |

**[Table 300]**

| Compound Number | Y | W | Z | R¹ | R² | R³ | R¹⁰ | A |
|---|---|---|---|---|---|---|---|---|
| C-3207 | Me(R) | single bond | H | c-Pr(1-CN) | Me | Cl | CH₂CF₃ | O |
| C-3208 | Me(S) | single bond | H | c-Pr(1-CN) | Me | Cl | CH₂CF₃ | O |
| C-3209 | H | CH₂ | Me | c-Pr(1-CN) | Me | Cl | CH₂CF₃ | O |
| C-3210 | Me | CH₂ | H | c-Pr(1-CN) | Me | Cl | CH₂CF₃ | O |
| C-3211 | H | CH(Me) | H | c-Pr(1-CN) | Me | Cl | CH₂CF₃ | O |
| C-3212 | H | CH₂CH₂ | Me | c-Pr(1-CN) | Me | Cl | CH₂CF₃ | O |
| C-3213 | Me | CH₂CH₂ | H | c-Pr(1-CN) | Me | Cl | CH₂CF₃ | O |
| C-3214 | H | single bond | Me | c-Pr(1-CN) | Me | Me | CH₂CF₃ | O |
| C-3215 | Me | single bond | H | c-Pr(1-CN) | Me | Me | CH₂CF₃ | O |
| C-3216 | Me(R) | single bond | H | c-Pr(1-CN) | Me | Me | CH₂CF₃ | O |
| C-3217 | Me(S) | single bond | H | c-Pr(1-CN) | Me | Me | CH₂CF₃ | O |
| C-3218 | H | CH₂ | Me | c-Pr(1-CN) | Me | Me | CH₂CF₃ | O |
| C-3219 | Me | CH₂ | H | c-Pr(1-CN) | Me | Me | CH₂CF₃ | O |
| C-3220 | H | CH(Me) | H | c-Pr(1-CN) | Me | Me | CH₂CF₃ | O |
| C-3221 | H | CH₂CH₂ | Me | c-Pr(1-CN) | Me | Me | CH₂CF₃ | O |
| C-3222 | Me | CH₂CH₂ | H | c-Pr(1-CN) | Me | Me | CH₂CF₃ | O |
| C-3223 | H | single bond | Me | c-Pr(1-CN) | Me | Cl | CH₂OMe | O |
| C-3224 | Me | single bond | H | c-Pr(1-CN) | Me | Cl | CH₂OMe | O |
| C-3225 | Me(R) | single bond | H | c-Pr(1-CN) | Me | Cl | CH₂OMe | O |
| C-3226 | Me(S) | single bond | H | c-Pr(1-CN) | Me | Cl | CH₂OMe | O |
| C-3227 | H | CH₂ | Me | c-Pr(1-CN) | Me | Cl | CH₂OMe | O |
| C-3228 | Me | CH₂ | H | c-Pr(1-CN) | Me | Cl | CH₂OMe | O |
| C-3229 | H | CH(Me) | H | c-Pr(1-CN) | Me | Cl | CH₂OMe | O |
| C-3230 | H | CH₂CH₂ | Me | c-Pr(1-CN) | Me | Cl | CH₂OMe | O |
| C-3231 | Me | CH₂CH₂ | H | c-Pr(1-CN) | Me | Cl | CH₂OMe | O |
| C-3232 | H | single bond | Me | c-Pr(1-CN) | Me | Me | CH₂OMe | O |
| C-3233 | Me | single bond | H | c-Pr(1-CN) | Me | Me | CH₂OMe | O |
| C-3234 | Me(R) | single bond | H | c-Pr(1-CN) | Me | Me | CH₂OMe | O |
| C-3235 | Me(S) | single bond | H | c-Pr(1-CN) | Me | Me | CH₂OMe | O |
| C-3236 | H | CH₂ | Me | c-Pr(1-CN) | Me | Me | CH₂OMe | O |
| C-3237 | Me | CH₂ | H | c-Pr(1-CN) | Me | Me | CH₂OMe | O |
| C-3238 | H | CH(Me) | H | c-Pr(1-CN) | Me | Me | CH₂OMe | O |
| C-3239 | H | CH₂CH₂ | Me | c-Pr(1-CN) | Me | Me | CH₂OMe | O |
| C-3240 | Me | CH₂CH₂ | H | c-Pr(1-CN) | Me | Me | CH₂OMe | O |
| C-3241 | H | single bond | Me | c-Pr(1-CN) | Me | Cl | CH₂CN | O |
| C-3242 | Me | single bond | H | c-Pr(1-CN) | Me | Cl | CH₂CN | O |
| C-3243 | Me(R) | single bond | H | c-Pr(1-CN) | Me | Cl | CH₂CN | O |
| C-3244 | Me(S) | single bond | H | c-Pr(1-CN) | Me | Cl | CH₂CN | O |
| C-3245 | H | CH₂ | Me | c-Pr(1-CN) | Me | Cl | CH₂CN | O |
| C-3246 | Me | CH₂ | H | c-Pr(1-CN) | Me | Cl | CH₂CN | O |
| C-3247 | H | CH(Me) | H | c-Pr(1-CN) | Me | Cl | CH₂CN | O |
| C-3248 | H | CH₂CH₂ | Me | c-Pr(1-CN) | Me | Cl | CH₂CN | O |
| C-3249 | Me | CH₂CH₂ | H | c-Pr(1-CN) | Me | Cl | CH₂CN | O |
| C-3250 | H | single bond | Me | c-Pr(1-CN) | Me | Me | CH₂CN | O |

**[Table 301]**

| Compound Number | Y | W | Z | R¹ | R² | R³ | R¹⁰ | A |
|---|---|---|---|---|---|---|---|---|
| C-3251 | Me | single bond | H | c-Pr(1-CN) | Me | Me | CH₂CN | O |
| C-3252 | Me(R) | single bond | H | c-Pr(1-CN) | Me | Me | CH₂CN | O |
| C-3253 | Me(S) | single bond | H | c-Pr(1-CN) | Me | Me | CH₂CN | O |
| C-3254 | H | CH₂ | Me | c-Pr(1-CN) | Me | Me | CH₂CN | O |
| C-3255 | Me | CH₂ | H | c-Pr(1-CN) | Me | Me | CH₂CN | O |
| C-3256 | H | CH(Me) | H | c-Pr(1-CN) | Me | Me | CH₂CN | O |
| C-3257 | H | CH₂CH₂ | Me | c-Pr(1-CN) | Me | Me | CH₂CN | O |
| C-3258 | Me | CH₂CH₂ | H | c-Pr(1-CN) | Me | Me | CH₂CN | O |
| C-3259 | H | single bond | Me | c-Pr(1-CN) | Me | Cl | C(=O)Me | O |
| C-3260 | Me | single bond | H | c-Pr(1-CN) | Me | Cl | C(=O)Me | O |
| C-3261 | Me(R) | single bond | H | c-Pr(1-CN) | Me | Cl | C(=O)Me | O |
| C-3262 | Me(S) | single bond | H | c-Pr(1-CN) | Me | Cl | C(=O)Me | O |
| C-3263 | H | CH₂ | Me | c-Pr(1-CN) | Me | Cl | C(=O)Me | O |
| C-3264 | Me | CH₂ | H | c-Pr(1-CN) | Me | Cl | C(=O)Me | O |
| C-3265 | H | CH(Me) | H | c-Pr(1-CN) | Me | Cl | C(=O)Me | O |
| C-3266 | H | CH₂CH₂ | Me | c-Pr(1-CN) | Me | Cl | C(=O)Me | O |
| C-3267 | Me | CH₂CH₂ | H | c-Pr(1-CN) | Me | Cl | C(=O)Me | O |
| C-3268 | H | single bond | Me | c-Pr(1-CN) | Me | Me | C(=O)Me | O |
| C-3269 | Me | single bond | H | c-Pr(1-CN) | Me | Me | C(=O)Me | O |
| C-3270 | Me(R) | single bond | H | c-Pr(1-CN) | Me | Me | C(=O)Me | O |
| C-3271 | Me(S) | single bond | H | c-Pr(1-CN) | Me | Me | C(=O)Me | O |
| C-3272 | H | CH₂ | Me | c-Pr(1-CN) | Me | Me | C(=O)Me | O |
| C-3273 | Me | CH₂ | H | c-Pr(1-CN) | Me | Me | C(=O)Me | O |
| C-3274 | H | CH(Me) | H | c-Pr(1-CN) | Me | Me | C(=O)Me | O |
| C-3275 | H | CH₂CH₂ | Me | c-Pr(1-CN) | Me | Me | C(=O)Me | O |
| C-3276 | Me | CH₂CH₂ | H | c-Pr(1-CN) | Me | Me | C(=O)Me | O |
| C-3277 | H | single bond | Me | c-Pr(1-CN) | CH₂OMe | Cl | Me | O |
| C-3278 | Me | single bond | H | c-Pr(1-CN) | CH₂OMe | Cl | Me | O |
| C-3279 | Me(R) | single bond | H | c-Pr(1-CN) | CH₂OMe | Cl | Me | O |
| C-3280 | Me(S) | single bond | H | c-Pr(1-CN) | CH₂OMe | Cl | Me | O |
| C-3281 | H | CH₂ | Me | c-Pr(1-CN) | CH₂OMe | Cl | Me | O |
| C-3282 | Me | CH₂ | H | c-Pr(1-CN) | CH₂OMe | Cl | Me | O |
| C-3283 | H | CH(Me) | H | c-Pr(1-CN) | CH₂OMe | Cl | Me | O |
| C-3284 | H | CH₂CH₂ | Me | c-Pr(1-CN) | CH₂OMe | Cl | Me | O |
| C-3285 | Me | CH₂CH₂ | H | c-Pr(1-CN) | CH₂OMe | Cl | Me | O |
| C-3286 | H | single bond | Me | c-Pr(1-CN) | CH₂OMe | Me | Me | O |
| C-3287 | Me | single bond | H | c-Pr(1-CN) | CH₂OMe | Me | Me | O |
| C-3288 | Me(R) | single bond | H | c-Pr(1-CN) | CH₂OMe | Me | Me | O |
| C-3289 | Me(S) | single bond | H | c-Pr(1-CN) | CH₂OMe | Me | Me | O |
| C-3290 | H | CH₂ | Me | c-Pr(1-CN) | CH₂OMe | Me | Me | O |
| C-3291 | Me | CH₂ | H | c-Pr(1-CN) | CH₂OMe | Me | Me | O |
| C-3292 | H | CH(Me) | H | c-Pr(1-CN) | CH₂OMe | Me | Me | O |
| C-3293 | H | CH₂CH₂ | Me | c-Pr(1-CN) | CH₂OMe | Me | Me | O |
| C-3294 | Me | CH₂CH₂ | H | c-Pr(1-CN) | CH₂OMe | Me | Me | O |

**[Table 302]**

| Compound Number | Y | W | Z | R¹ | R² | R³ | R¹⁰ | A |
|---|---|---|---|---|---|---|---|---|
| C-3295 | H | single bond | Me | c-Pr(1-CN) | CH₂OMe | Cl | Et | O |
| C-3296 | Me | single bond | H | c-Pr(1-CN) | CH₂OMe | Cl | Et | O |
| C-3297 | Me(R) | single bond | H | c-Pr(1-CN) | CH₂OMe | Cl | Et | O |
| C-3298 | Me(S) | single bond | H | c-Pr(1-CN) | CH₂OMe | Cl | Et | O |
| C-3299 | H | CH₂ | Me | c-Pr(1-CN) | CH₂OMe | Cl | Et | O |
| C-3300 | Me | CH₂ | H | c-Pr(1-CN) | CH₂OMe | Cl | Et | O |
| C-3301 | H | CH(Me) | H | c-Pr(1-CN) | CH₂OMe | Cl | Et | O |
| C-3302 | H | CH₂CH₂ | Me | c-Pr(1-CN) | CH₂OMe | Cl | Et | O |
| C-3303 | Me | CH₂CH₂ | H | c-Pr(1-CN) | CH₂OMe | Cl | Et | O |
| C-3304 | H | single bond | Me | c-Pr(1-CN) | CH₂OMe | Me | Et | O |
| C-3305 | Me | single bond | H | c-Pr(1-CN) | CH₂OMe | Me | Et | O |
| C-3306 | Me(R) | single bond | H | c-Pr(1-CN) | CH₂OMe | Me | Et | O |
| C-3307 | Me(S) | single bond | H | c-Pr(1-CN) | CH₂OMe | Me | Et | O |
| C-3308 | H | CH₂ | Me | c-Pr(1-CN) | CH₂OMe | Me | Et | O |
| C-3309 | Me | CH₂ | H | c-Pr(1-CN) | CH₂OMe | Me | Et | O |
| C-3310 | H | CH(Me) | H | c-Pr(1-CN) | CH₂OMe | Me | Et | O |
| C-3311 | H | CH₂CH₂ | Me | c-Pr(1-CN) | CH₂OMe | Me | Et | O |
| C-3312 | Me | CH₂CH₂ | H | c-Pr(1-CN) | CH₂OMe | Me | Et | O |
| C-3313 | H | single bond | Me | c-Pr(1-CN) | CH₂OMe | Cl | CH₂CF₃ | O |
| C-3314 | Me | single bond | H | c-Pr(1-CN) | CH₂OMe | Cl | CH₂CF₃ | O |
| C-3315 | Me(R) | single bond | H | c-Pr(1-CN) | CH₂OMe | Cl | CH₂CF₃ | O |
| C-3316 | Me(S) | single bond | H | c-Pr(1-CN) | CH₂OMe | Cl | CH₂CF₃ | O |
| C-3317 | H | CH₂ | Me | c-Pr(1-CN) | CH₂OMe | Cl | CH₂CF₃ | O |
| C-3318 | Me | CH₂ | H | c-Pr(1-CN) | CH₂OMe | Cl | CH₂CF₃ | O |
| C-3319 | H | CH(Me) | H | c-Pr(1-CN) | CH₂OMe | Cl | CH₂CF₃ | O |
| C-3320 | H | CH₂CH₂ | Me | c-Pr(1-CN) | CH₂OMe | Cl | CH₂CF₃ | O |
| C-3321 | Me | CH₂CH₂ | H | c-Pr(1-CN) | CH₂OMe | Cl | CH₂CF₃ | O |
| C-3322 | H | single bond | Me | c-Pr(1-CN) | CH₂OMe | Me | CH₂CF₃ | O |
| C-3323 | Me | single bond | H | c-Pr(1-CN) | CH₂OMe | Me | CH₂CF₃ | O |
| C-3324 | Me(R) | single bond | H | c-Pr(1-CN) | CH₂OMe | Me | CH₂CF₃ | O |
| C-3325 | Me(S) | single bond | H | c-Pr(1-CN) | CH₂OMe | Me | CH₂CF₃ | O |
| C-3326 | H | CH₂ | Me | c-Pr(1-CN) | CH₂OMe | Me | CH₂CF₃ | O |
| C-3327 | Me | CH₂ | H | c-Pr(1-CN) | CH₂OMe | Me | CH₂CF₃ | O |
| C-3328 | H | CH(Me) | H | c-Pr(1-CN) | CH₂OMe | Me | CH₂CF₃ | O |
| C-3329 | H | CH₂CH₂ | Me | c-Pr(1-CN) | CH₂OMe | Me | CH₂CF₃ | O |
| C-3330 | Me | CH₂CH₂ | H | c-Pr(1-CN) | CH₂OMe | Me | CH₂CF₃ | O |
| C-3331 | H | single bond | Me | c-Pr(1-CN) | CH₂OMe | Cl | CH₂OMe | O |
| C-3332 | Me | single bond | H | c-Pr(1-CN) | CH₂OMe | Cl | CH₂OMe | O |
| C-3333 | Me(R) | single bond | H | c-Pr(1-CN) | CH₂OMe | Cl | CH₂OMe | O |
| C-3334 | Me(S) | single bond | H | c-Pr(1-CN) | CH₂OMe | Cl | CH₂OMe | O |
| C-3335 | H | CH₂ | Me | c-Pr(1-CN) | CH₂OMe | Cl | CH₂OMe | O |
| C-3336 | Me | CH₂ | H | c-Pr(1-CN) | CH₂OMe | Cl | CH₂OMe | O |
| C-3337 | H | CH(Me) | H | c-Pr(1-CN) | CH₂OMe | Cl | CH₂OMe | O |
| C-3338 | H | CH₂CH₂ | Me | c-Pr(1-CN) | CH₂OMe | Cl | CH₂OMe | O |

**[Table 303]**

| Compound Number | Y | W | Z | R¹ | R² | R³ | R¹⁰ | A |
|---|---|---|---|---|---|---|---|---|
| C-3339 | Me | CH₂CH₂ | H | c-Pr(1-CN) | CH₂OMe | Cl | CH₂OMe | O |
| C-3340 | H | single bond | Me | c-Pr(1-CN) | CH₂OMe | Me | CH₂OMe | O |
| C-3341 | Me | single bond | H | c-Pr(1-CN) | CH₂OMe | Me | CH₂OMe | O |
| C-3342 | Me(R) | single bond | H | c-Pr(1-CN) | CH₂OMe | Me | CH₂OMe | O |
| C-3343 | Me(S) | single bond | H | c-Pr(1-CN) | CH₂OMe | Me | CH₂OMe | O |
| C-3344 | H | CH₂ | Me | c-Pr(1-CN) | CH₂OMe | Me | CH₂OMe | O |
| C-3345 | Me | CH₂ | H | c-Pr(1-CN) | CH₂OMe | Me | CH₂OMe | O |
| C-3346 | H | CH(Me) | H | c-Pr(1-CN) | CH₂OMe | Me | CH₂OMe | O |
| C-3347 | H | CH₂CH₂ | Me | c-Pr(1-CN) | CH₂OMe | Me | CH₂OMe | O |
| C-3348 | Me | CH₂CH₂ | H | c-Pr(1-CN) | CH₂OMe | Me | CH₂OMe | O |
| C-3349 | H | single bond | Me | c-Pr(1-CN) | CH₂OMe | Cl | CH₂CN | O |
| C-3350 | Me | single bond | H | c-Pr(1-CN) | CH₂OMe | Cl | CH₂CN | O |
| C-3351 | Me(R) | single bond | H | c-Pr(1-CN) | CH₂OMe | Cl | CH₂CN | O |
| C-3352 | Me(S) | single bond | H | c-Pr(1-CN) | CH₂OMe | Cl | CH₂CN | O |
| C-3353 | H | CH₂ | Me | c-Pr(1-CN) | CH₂OMe | Cl | CH₂CN | O |
| C-3354 | Me | CH₂ | H | c-Pr(1-CN) | CH₂OMe | Cl | CH₂CN | O |
| C-3355 | H | CH(Me) | H | c-Pr(1-CN) | CH₂OMe | Cl | CH₂CN | O |
| C-3356 | H | CH₂CH₂ | Me | c-Pr(1-CN) | CH₂OMe | Cl | CH₂CN | O |
| C-3357 | Me | CH₂CH₂ | H | c-Pr(1-CN) | CH₂OMe | Cl | CH₂CN | O |
| C-3358 | H | single bond | Me | c-Pr(1-CN) | CH₂OMe | Me | CH₂CN | O |
| C-3359 | Me | single bond | H | c-Pr(1-CN) | CH₂OMe | Me | CH₂CN | O |
| C-3360 | Me(R) | single bond | H | c-Pr(1-CN) | CH₂OMe | Me | CH₂CN | O |
| C-3361 | Me(S) | single bond | H | c-Pr(1-CN) | CH₂OMe | Me | CH₂CN | O |
| C-3362 | H | CH₂ | Me | c-Pr(1-CN) | CH₂OMe | Me | CH₂CN | O |
| C-3363 | Me | CH₂ | H | c-Pr(1-CN) | CH₂OMe | Me | CH₂CN | O |
| C-3364 | H | CH(Me) | H | c-Pr(1-CN) | CH₂OMe | Me | CH₂CN | O |
| C-3365 | H | CH₂CH₂ | Me | c-Pr(1-CN) | CH₂OMe | Me | CH₂CN | O |
| C-3366 | Me | CH₂CH₂ | H | c-Pr(1-CN) | CH₂OMe | Me | CH₂CN | O |
| C-3367 | H | single bond | Me | c-Pr(1-CN) | CH₂OMe | Cl | C(=O)Me | O |
| C-3368 | Me | single bond | H | c-Pr(1-CN) | CH₂OMe | Cl | C(=O)Me | O |
| C-3369 | Me(R) | single bond | H | c-Pr(1-CN) | CH₂OMe | Cl | C(=O)Me | O |
| C-3370 | Me(S) | single bond | H | c-Pr(1-CN) | CH₂OMe | Cl | C(=O)Me | O |
| C-3371 | H | CH₂ | Me | c-Pr(1-CN) | CH₂OMe | Cl | C(=O)Me | O |
| C-3372 | Me | CH₂ | H | c-Pr(1-CN) | CH₂OMe | Cl | C(=O)Me | O |
| C-3373 | H | CH(Me) | H | c-Pr(1-CN) | CH₂OMe | Cl | C(=O)Me | O |
| C-3374 | H | CH₂CH₂ | Me | c-Pr(1-CN) | CH₂OMe | Cl | C(=O)Me | O |
| C-3375 | Me | CH₂CH₂ | H | c-Pr(1-CN) | CH₂OMe | Cl | C(=O)Me | O |
| C-3376 | H | single bond | Me | c-Pr(1-CN) | CH₂OMe | Me | C(=O)Me | O |
| C-3377 | Me | single bond | H | c-Pr(1-CN) | CH₂OMe | Me | C(=O)Me | O |
| C-3378 | Me(R) | single bond | H | c-Pr(1-CN) | CH₂OMe | Me | C(=O)Me | O |
| C-3379 | Me(S) | single bond | H | c-Pr(1-CN) | CH₂OMe | Me | C(=O)Me | O |
| C-3380 | H | CH₂ | Me | c-Pr(1-CN) | CH₂OMe | Me | C(=O)Me | O |
| C-3381 | Me | CH₂ | H | c-Pr(1-CN) | CH₂OMe | Me | C(=O)Me | O |
| C-3382 | H | CH(Me) | H | c-Pr(1-CN) | CH₂OMe | Me | C(=O)Me | O |

**[Table 304]**

| Compound Number | Y | W | Z | R¹ | R² | R³ | R¹⁰ | A |
|---|---|---|---|---|---|---|---|---|
| C-3383 | H | CH₂CH₂ | Me | c-Pr(1-CN) | CH₂OMe | Me | C(=O)Me | O |
| C-3384 | Me | CH₂CH₂ | H | c-Pr(1-CN) | CH₂OMe | Me | C(=O)Me | O |
| C-3385 | H | single bond | Me | c-Pr(1-CN) | CH₂CN | Cl | Me | O |
| C-3386 | Me | single bond | H | c-Pr(1-CN) | CH₂CN | Cl | Me | O |
| C-3387 | Me(R) | single bond | H | c-Pr(1-CN) | CH₂CN | Cl | Me | O |
| C-3388 | Me(S) | single bond | H | c-Pr(1-CN) | CH₂CN | Cl | Me | O |
| C-3389 | H | CH₂ | Me | c-Pr(1-CN) | CH₂CN | Cl | Me | O |
| C-3390 | Me | CH₂ | H | c-Pr(1-CN) | CH₂CN | Cl | Me | O |
| C-3391 | H | CH(Me) | H | c-Pr(1-CN) | CH₂CN | Cl | Me | O |
| C-3392 | H | CH₂CH₂ | Me | c-Pr(1-CN) | CH₂CN | Cl | Me | O |
| C-3393 | Me | CH₂CH₂ | H | c-Pr(1-CN) | CH₂CN | Cl | Me | O |
| C-3394 | H | single bond | Me | c-Pr(1-CN) | CH₂CN | Me | Me | O |
| C-3395 | Me | single bond | H | c-Pr(1-CN) | CH₂CN | Me | Me | O |
| C-3396 | Me(R) | single bond | H | c-Pr(1-CN) | CH₂CN | Me | Me | O |
| C-3397 | Me(S) | single bond | H | c-Pr(1-CN) | CH₂CN | Me | Me | O |
| C-3398 | H | CH₂ | Me | c-Pr(1-CN) | CH₂CN | Me | Me | O |
| C-3399 | Me | CH₂ | H | c-Pr(1-CN) | CH₂CN | Me | Me | O |
| C-3400 | H | CH(Me) | H | c-Pr(1-CN) | CH₂CN | Me | Me | O |
| C-3401 | H | CH₂CH₂ | Me | c-Pr(1-CN) | CH₂CN | Me | Me | O |
| C-3402 | Me | CH₂CH₂ | H | c-Pr(1-CN) | CH₂CN | Me | Me | O |
| C-3403 | H | single bond | Me | c-Pr(1-CN) | CH₂CN | Cl | Et | O |
| C-3404 | Me | single bond | H | c-Pr(1-CN) | CH₂CN | Cl | Et | O |
| C-3405 | Me(R) | single bond | H | c-Pr(1-CN) | CH₂CN | Cl | Et | O |
| C-3406 | Me(S) | single bond | H | c-Pr(1-CN) | CH₂CN | Cl | Et | O |
| C-3407 | H | CH₂ | Me | c-Pr(1-CN) | CH₂CN | Cl | Et | O |
| C-3408 | Me | CH₂ | H | c-Pr(1-CN) | CH₂CN | Cl | Et | O |
| C-3409 | H | CH(Me) | H | c-Pr(1-CN) | CH₂CN | Cl | Et | O |
| C-3410 | H | CH₂CH₂ | Me | c-Pr(1-CN) | CH₂CN | Cl | Et | O |
| C-3411 | Me | CH₂CH₂ | H | c-Pr(1-CN) | CH₂CN | Cl | Et | O |
| C-3412 | H | single bond | Me | c-Pr(1-CN) | CH₂CN | Me | Et | O |
| C-3413 | Me | single bond | H | c-Pr(1-CN) | CH₂CN | Me | Et | O |
| C-3414 | Me(R) | single bond | H | c-Pr(1-CN) | CH₂CN | Me | Et | O |
| C-3415 | Me(S) | single bond | H | c-Pr(1-CN) | CH₂CN | Me | Et | O |
| C-3416 | H | CH₂ | Me | c-Pr(1-CN) | CH₂CN | Me | Et | O |
| C-3417 | Me | CH₂ | H | c-Pr(1-CN) | CH₂CN | Me | Et | O |
| C-3418 | H | CH(Me) | H | c-Pr(1-CN) | CH₂CN | Me | Et | O |
| C-3419 | H | CH₂CH₂ | Me | c-Pr(1-CN) | CH₂CN | Me | Et | O |
| C-3420 | Me | CH₂CH₂ | H | c-Pr(1-CN) | CH₂CN | Me | Et | O |
| C-3421 | H | single bond | Me | c-Pr(1-CN) | CH₂CN | Cl | CH₂CF₃ | O |
| C-3422 | Me | single bond | H | c-Pr(1-CN) | CH₂CN | Cl | CH₂CF₃ | O |
| C-3423 | Me(R) | single bond | H | c-Pr(1-CN) | CH₂CN | Cl | CH₂CF₃ | O |
| C-3424 | Me(S) | single bond | H | c-Pr(1-CN) | CH₂CN | Cl | CH₂CF₃ | O |
| C-3425 | H | CH₂ | Me | c-Pr(1-CN) | CH₂CN | Cl | CH₂CF₃ | O |
| C-3426 | Me | CH₂ | H | c-Pr(1-CN) | CH₂CN | Cl | CH₂CF₃ | O |

**[Table 305]**

| Compound Number | Y | W | Z | R¹ | R² | R³ | R¹⁰ | A |
|---|---|---|---|---|---|---|---|---|
| C-3427 | H | CH(Me) | H | c-Pr(1-CN) | CH₂CN | Cl | CH₂CF₃ | O |
| C-3428 | H | CH₂CH₂ | Me | c-Pr(1-CN) | CH₂CN | Cl | CH₂CF₃ | O |
| C-3429 | Me | CH₂CH₂ | H | c-Pr(1-CN) | CH₂CN | Cl | CH₂CF₃ | O |
| C-3430 | H | single bond | Me | c-Pr(1-CN) | CH₂CN | Me | CH₂CF₃ | O |
| C-3431 | Me | single bond | H | c-Pr(1-CN) | CH₂CN | Me | CH₂CF₃ | O |
| C-3432 | Me(R) | single bond | H | c-Pr(1-CN) | CH₂CN | Me | CH₂CF₃ | O |
| C-3433 | Me(S) | single bond | H | c-Pr(1-CN) | CH₂CN | Me | CH₂CF₃ | O |
| C-3434 | H | CH₂ | Me | c-Pr(1-CN) | CH₂CN | Me | CH₂CF₃ | O |
| C-3435 | Me | CH₂ | H | c-Pr(1-CN) | CH₂CN | Me | CH₂CF₃ | O |
| C-3436 | H | CH(Me) | H | c-Pr(1-CN) | CH₂CN | Me | CH₂CF₃ | O |
| C-3437 | H | CH₂CH₂ | Me | c-Pr(1-CN) | CH₂CN | Me | CH₂CF₃ | O |
| C-3438 | Me | CH₂CH₂ | H | c-Pr(1-CN) | CH₂CN | Me | CH₂CF₃ | O |
| C-3439 | H | single bond | Me | c-Pr(1-CN) | CH₂CN | Cl | CH₂OMe | O |
| C-3440 | Me | single bond | H | c-Pr(1-CN) | CH₂CN | Cl | CH₂OMe | O |
| C-3441 | Me(R) | single bond | H | c-Pr(1-CN) | CH₂CN | Cl | CH₂OMe | O |
| C-3442 | Me(S) | single bond | H | c-Pr(1-CN) | CH₂CN | Cl | CH₂OMe | O |
| C-3443 | H | CH₂ | Me | c-Pr(1-CN) | CH₂CN | Cl | CH₂OMe | O |
| C-3444 | Me | CH₂ | H | c-Pr(1-CN) | CH₂CN | Cl | CH₂OMe | O |
| C-3445 | H | CH(Me) | H | c-Pr(1-CN) | CH₂CN | Cl | CH₂OMe | O |
| C-3446 | H | CH₂CH₂ | Me | c-Pr(1-CN) | CH₂CN | Cl | CH₂OMe | O |
| C-3447 | Me | CH₂CH₂ | H | c-Pr(1-CN) | CH₂CN | Cl | CH₂OMe | O |
| C-3448 | H | single bond | Me | c-Pr(1-CN) | CH₂CN | Me | CH₂OMe | O |
| C-3449 | Me | single bond | H | c-Pr(1-CN) | CH₂CN | Me | CH₂OMe | O |
| C-3450 | Me(R) | single bond | H | c-Pr(1-CN) | CH₂CN | Me | CH₂OMe | O |
| C-3451 | Me(S) | single bond | H | c-Pr(1-CN) | CH₂CN | Me | CH₂OMe | O |
| C-3452 | H | CH₂ | Me | c-Pr(1-CN) | CH₂CN | Me | CH₂OMe | O |
| C-3453 | Me | CH₂ | H | c-Pr(1-CN) | CH₂CN | Me | CH₂OMe | O |
| C-3454 | H | CH(Me) | H | c-Pr(1-CN) | CH₂CN | Me | CH₂OMe | O |
| C-3455 | H | CH₂CH₂ | Me | c-Pr(1-CN) | CH₂CN | Me | CH₂OMe | O |
| C-3456 | Me | CH₂CH₂ | H | c-Pr(1-CN) | CH₂CN | Me | CH₂OMe | O |
| C-3457 | H | single bond | Me | c-Pr(1-CN) | CH₂CN | Cl | CH₂CN | O |
| C-3458 | Me | single bond | H | c-Pr(1-CN) | CH₂CN | Cl | CH₂CN | O |
| C-3459 | Me(R) | single bond | H | c-Pr(1-CN) | CH₂CN | Cl | CH₂CN | O |
| C-3460 | Me(S) | single bond | H | c-Pr(1-CN) | CH₂CN | Cl | CH₂CN | O |
| C-3461 | H | CH₂ | Me | c-Pr(1-CN) | CH₂CN | Cl | CH₂CN | O |
| C-3462 | Me | CH₂ | H | c-Pr(1-CN) | CH₂CN | Cl | CH₂CN | O |
| C-3463 | H | CH(Me) | H | c-Pr(1-CN) | CH₂CN | Cl | CH₂CN | O |
| C-3464 | H | CH₂CH₂ | Me | c-Pr(1-CN) | CH₂CN | Cl | CH₂CN | O |
| C-3465 | Me | CH₂CH₂ | H | c-Pr(1-CN) | CH₂CN | Cl | CH₂CN | O |
| C-3466 | H | single bond | Me | c-Pr(1-CN) | CH₂CN | Me | CH₂CN | O |
| C-3467 | Me | single bond | H | c-Pr(1-CN) | CH₂CN | Me | CH₂CN | O |
| C-3468 | Me(R) | single bond | H | c-Pr(1-CN) | CH₂CN | Me | CH₂CN | O |
| C-3469 | Me(S) | single bond | H | c-Pr(1-CN) | CH₂CN | Me | CH₂CN | O |
| C-3470 | H | CH₂ | Me | c-Pr(1-CN) | CH₂CN | Me | CH₂CN | O |

**[Table 306]**

| Compound Number | Y | W | Z | R¹ | R² | R³ | R¹⁰ | A |
|---|---|---|---|---|---|---|---|---|
| C-3471 | Me | CH₂ | H | c-Pr(1-CN) | CH₂CN | Me | CH₂CN | O |
| C-3472 | H | CH(Me) | H | c-Pr(1-CN) | CH₂CN | Me | CH₂CN | O |
| C-3473 | H | CH₂CH₂ | Me | c-Pr(1-CN) | CH₂CN | Me | CH₂CN | O |
| C-3474 | Me | CH₂CH₂ | H | c-Pr(1-CN) | CH₂CN | Me | CH₂CN | O |
| C-3475 | H | single bond | Me | c-Pr(1-CN) | CH₂CN | Cl | C(=O)Me | O |
| C-3476 | Me | single bond | H | c-Pr(1-CN) | CH₂CN | Cl | C(=O)Me | O |
| C-3477 | Me(R) | single bond | H | c-Pr(1-CN) | CH₂CN | Cl | C(=O)Me | O |
| C-3478 | Me(S) | single bond | H | c-Pr(1-CN) | CH₂CN | Cl | C(=O)Me | O |
| C-3479 | H | CH₂ | Me | c-Pr(1-CN) | CH₂CN | Cl | C(=O)Me | O |
| C-3480 | Me | CH₂ | H | c-Pr(1-CN) | CH₂CN | Cl | C(=O)Me | O |
| C-3481 | H | CH(Me) | H | c-Pr(1-CN) | CH₂CN | Cl | C(=O)Me | O |
| C-3482 | H | CH₂CH₂ | Me | c-Pr(1-CN) | CH₂CN | Cl | C(=O)Me | O |
| C-3483 | Me | CH₂CH₂ | H | c-Pr(1-CN) | CH₂CN | Cl | C(=O)Me | O |
| C-3484 | H | single bond | Me | c-Pr(1-CN) | CH₂CN | Me | C(=O)Me | O |
| C-3485 | Me | single bond | H | c-Pr(1-CN) | CH₂CN | Me | C(=O)Me | O |
| C-3486 | Me(R) | single bond | H | c-Pr(1-CN) | CH₂CN | Me | C(=O)Me | O |
| C-3487 | Me(S) | single bond | H | c-Pr(1-CN) | CH₂CN | Me | C(=O)Me | O |
| C-3488 | H | CH₂ | Me | c-Pr(1-CN) | CH₂CN | Me | C(=O)Me | O |
| C-3489 | Me | CH₂ | H | c-Pr(1-CN) | CH₂CN | Me | C(=O)Me | O |
| C-3490 | H | CH(Me) | H | c-Pr(1-CN) | CH₂CN | Me | C(=O)Me | O |
| C-3491 | H | CH₂CH₂ | Me | c-Pr(1-CN) | CH₂CN | Me | C(=O)Me | O |
| C-3492 | Me | CH₂CH₂ | H | c-Pr(1-CN) | CH₂CN | Me | C(=O)Me | O |
| C-3493 | H | single bond | Me | c-Pr(1-CN) | C(=O)Me | Cl | Me | O |
| C-3494 | Me | single bond | H | c-Pr(1-CN) | C(=O)Me | Cl | Me | O |
| C-3495 | Me(R) | single bond | H | c-Pr(1-CN) | C(=O)Me | Cl | Me | O |
| C-3496 | Me(S) | single bond | H | c-Pr(1-CN) | C(=O)Me | Cl | Me | O |
| C-3497 | H | CH₂ | Me | c-Pr(1-CN) | C(=O)Me | Cl | Me | O |
| C-3498 | Me | CH₂ | H | c-Pr(1-CN) | C(=O)Me | Cl | Me | O |
| C-3499 | H | CH(Me) | H | c-Pr(1-CN) | C(=O)Me | Cl | Me | O |
| C-3500 | H | CH₂CH₂ | Me | c-Pr(1-CN) | C(=O)Me | Cl | Me | O |
| C-3501 | Me | CH₂CH₂ | H | c-Pr(1-CN) | C(=O)Me | Cl | Me | O |
| C-3502 | H | single bond | Me | c-Pr(1-CN) | C(=O)Me | Me | Me | O |
| C-3503 | Me | single bond | H | c-Pr(1-CN) | C(=O)Me | Me | Me | O |
| C-3504 | Me(R) | single bond | H | c-Pr(1-CN) | C(=O)Me | Me | Me | O |
| C-3505 | Me(S) | single bond | H | c-Pr(1-CN) | C(=O)Me | Me | Me | O |
| C-3506 | H | CH₂ | Me | c-Pr(1-CN) | C(=O)Me | Me | Me | O |
| C-3507 | Me | CH₂ | H | c-Pr(1-CN) | C(=O)Me | Me | Me | O |
| C-3508 | H | CH(Me) | H | c-Pr(1-CN) | C(=O)Me | Me | Me | O |
| C-3509 | H | CH₂CH₂ | Me | c-Pr(1-CN) | C(=O)Me | Me | Me | O |
| C-3510 | Me | CH₂CH₂ | H | c-Pr(1-CN) | C(=O)Me | Me | Me | O |
| C-3511 | H | single bond | Me | c-Pr(1-CN) | C(=O)Me | Cl | Et | O |
| C-3512 | Me | single bond | H | c-Pr(1-CN) | C(=O)Me | Cl | Et | O |
| C-3513 | Me(R) | single bond | H | c-Pr(1-CN) | C(=O)Me | Cl | Et | O |
| C-3514 | Me(S) | single bond | H | c-Pr(1-CN) | C(=O)Me | Cl | Et | O |

**[Table 307]**

| Compound Number | Y | W | Z | R¹ | R² | R³ | R¹⁰ | A |
|---|---|---|---|---|---|---|---|---|
| C-3515 | H | CH₂ | Me | c-Pr(1-CN) | C(=O)Me | Cl | Et | O |
| C-3516 | Me | CH₂ | H | c-Pr(1-CN) | C(=O)Me | Cl | Et | O |
| C-3517 | H | CH(Me) | H | c-Pr(1-CN) | C(=O)Me | Cl | Et | O |
| C-3518 | H | CH₂CH₂ | Me | c-Pr(1-CN) | C(=O)Me | Cl | Et | O |
| C-3519 | Me | CH₂CH₂ | H | c-Pr(1-CN) | C(=O)Me | Cl | Et | O |
| C-3520 | H | single bond | Me | c-Pr(1-CN) | C(=O)Me | Me | Et | O |
| C-3521 | Me | single bond | H | c-Pr(1-CN) | C(=O)Me | Me | Et | O |
| C-3522 | Me(R) | single bond | H | c-Pr(1-CN) | C(=O)Me | Me | Et | O |
| C-3523 | Me(S) | single bond | H | c-Pr(1-CN) | C(=O)Me | Me | Et | O |
| C-3524 | H | CH₂ | Me | c-Pr(1-CN) | C(=O)Me | Me | Et | O |
| C-3525 | Me | CH₂ | H | c-Pr(1-CN) | C(=O)Me | Me | Et | O |
| C-3526 | H | CH(Me) | H | c-Pr(1-CN) | C(=O)Me | Me | Et | O |
| C-3527 | H | CH₂CH₂ | Me | c-Pr(1-CN) | C(=O)Me | Me | Et | O |
| C-3528 | Me | CH₂CH₂ | H | c-Pr(1-CN) | C(=O)Me | Me | Et | O |
| C-3529 | H | single bond | Me | c-Pr(1-CN) | C(=O)Me | Cl | CH₂CF₃ | O |
| C-3530 | Me | single bond | H | c-Pr(1-CN) | C(=O)Me | Cl | CH₂CF₃ | O |
| C-3531 | Me(R) | single bond | H | c-Pr(1-CN) | C(=O)Me | Cl | CH₂CF₃ | O |
| C-3532 | Me(S) | single bond | H | c-Pr(1-CN) | C(=O)Me | Cl | CH₂CF₃ | O |
| C-3533 | H | CH₂ | Me | c-Pr(1-CN) | C(=O)Me | Cl | CH₂CF₃ | O |
| C-3534 | Me | CH₂ | H | c-Pr(1-CN) | C(=O)Me | Cl | CH₂CF₃ | O |
| C-3535 | H | CH(Me) | H | c-Pr(1-CN) | C(=O)Me | Cl | CH₂CF₃ | O |
| C-3536 | H | CH₂CH₂ | Me | c-Pr(1-CN) | C(=O)Me | Cl | CH₂CF₃ | O |
| C-3537 | Me | CH₂CH₂ | H | c-Pr(1-CN) | C(=O)Me | Cl | CH₂CF₃ | O |
| C-3538 | H | single bond | Me | c-Pr(1-CN) | C(=O)Me | Me | CH₂CF₃ | O |
| C-3539 | Me | single bond | H | c-Pr(1-CN) | C(=O)Me | Me | CH₂CF₃ | O |
| C-3540 | Me(R) | single bond | H | c-Pr(1-CN) | C(=O)Me | Me | CH₂CF₃ | O |
| C-3541 | Me(S) | single bond | H | c-Pr(1-CN) | C(=O)Me | Me | CH₂CF₃ | O |
| C-3542 | H | CH₂ | Me | c-Pr(1-CN) | C(=O)Me | Me | CH₂CF₃ | O |
| C-3543 | Me | CH₂ | H | c-Pr(1-CN) | C(=O)Me | Me | CH₂CF₃ | O |
| C-3544 | H | CH(Me) | H | c-Pr(1-CN) | C(=O)Me | Me | CH₂CF₃ | O |
| C-3545 | H | CH₂CH₂ | Me | c-Pr(1-CN) | C(=O)Me | Me | CH₂CF₃ | O |
| C-3546 | Me | CH₂CH₂ | H | c-Pr(1-CN) | C(=O)Me | Me | CH₂CF₃ | O |
| C-3547 | H | single bond | Me | c-Pr(1-CN) | C(=O)Me | Cl | CH₂OMe | O |
| C-3548 | Me | single bond | H | c-Pr(1-CN) | C(=O)Me | Cl | CH₂OMe | O |
| C-3549 | Me(R) | single bond | H | c-Pr(1-CN) | C(=O)Me | Cl | CH₂OMe | O |
| C-3550 | Me(S) | single bond | H | c-Pr(1-CN) | C(=O)Me | Cl | CH₂OMe | O |
| C-3551 | H | CH₂ | Me | c-Pr(1-CN) | C(=O)Me | Cl | CH₂OMe | O |
| C-3552 | Me | CH₂ | H | c-Pr(1-CN) | C(=O)Me | Cl | CH₂OMe | O |
| C-3553 | H | CH(Me) | H | c-Pr(1-CN) | C(=O)Me | Cl | CH₂OMe | O |
| C-3554 | H | CH₂CH₂ | Me | c-Pr(1-CN) | C(=O)Me | Cl | CH₂OMe | O |
| C-3555 | Me | CH₂CH₂ | H | c-Pr(1-CN) | C(=O)Me | Cl | CH₂OMe | O |
| C-3556 | H | single bond | Me | c-Pr(1-CN) | C(=O)Me | Me | CH₂OMe | O |
| C-3557 | Me | single bond | H | c-Pr(1-CN) | C(=O)Me | Me | CH₂OMe | O |
| C-3558 | Me(R) | single bond | H | c-Pr(1-CN) | C(=O)Me | Me | CH₂OMe | O |

**[Table 308]**

| Compound Number | Y | W | Z | R¹ | R² | R³ | R¹⁰ | A |
|---|---|---|---|---|---|---|---|---|
| C-3559 | Me(S) | single bond | H | c-Pr(1-CN) | C(=O)Me | Me | CH₂OMe | O |
| C-3560 | H | CH₂ | Me | c-Pr(1-CN) | C(=O)Me | Me | CH₂OMe | O |
| C-3561 | Me | CH₂ | H | c-Pr(1-CN) | C(=O)Me | Me | CH₂OMe | O |
| C-3562 | H | CH(Me) | H | c-Pr(1-CN) | C(=O)Me | Me | CH₂OMe | O |
| C-3563 | H | CH₂CH₂ | Me | c-Pr(1-CN) | C(=O)Me | Me | CH₂OMe | O |
| C-3564 | Me | CH₂CH₂ | H | c-Pr(1-CN) | C(=O)Me | Me | CH₂OMe | O |
| C-3565 | H | single bond | Me | c-Pr(1-CN) | C(=O)Me | Cl | CH₂CN | O |
| C-3566 | Me | single bond | H | c-Pr(1-CN) | C(=O)Me | Cl | CH₂CN | O |
| C-3567 | Me(R) | single bond | H | c-Pr(1-CN) | C(=O)Me | Cl | CH₂CN | O |
| C-3568 | Me(S) | single bond | H | c-Pr(1-CN) | C(=O)Me | Cl | CH₂CN | O |
| C-3569 | H | CH₂ | Me | c-Pr(1-CN) | C(=O)Me | Cl | CH₂CN | O |
| C-3570 | Me | CH₂ | H | c-Pr(1-CN) | C(=O)Me | Cl | CH₂CN | O |
| C-3571 | H | CH(Me) | H | c-Pr(1-CN) | C(=O)Me | Cl | CH₂CN | O |
| C-3572 | H | CH₂CH₂ | Me | c-Pr(1-CN) | C(=O)Me | Cl | CH₂CN | O |
| C-3573 | Me | CH₂CH₂ | H | c-Pr(1-CN) | C(=O)Me | Cl | CH₂CN | O |
| C-3574 | H | single bond | Me | c-Pr(1-CN) | C(=O)Me | Me | CH₂CN | O |
| C-3575 | Me | single bond | H | c-Pr(1-CN) | C(=O)Me | Me | CH₂CN | O |
| C-3576 | Me(R) | single bond | H | c-Pr(1-CN) | C(=O)Me | Me | CH₂CN | O |
| C-3577 | Me(S) | single bond | H | c-Pr(1-CN) | C(=O)Me | Me | CH₂CN | O |
| C-3578 | H | CH₂ | Me | c-Pr(1-CN) | C(=O)Me | Me | CH₂CN | O |
| C-3579 | Me | CH₂ | H | c-Pr(1-CN) | C(=O)Me | Me | CH₂CN | O |
| C-3580 | H | CH(Me) | H | c-Pr(1-CN) | C(=O)Me | Me | CH₂CN | O |
| C-3581 | H | CH₂CH₂ | Me | c-Pr(1-CN) | C(=O)Me | Me | CH₂CN | O |
| C-3582 | Me | CH₂CH₂ | H | c-Pr(1-CN) | C(=O)Me | Me | CH₂CN | O |
| C-3583 | H | single bond | Me | c-Pr(1-CN) | C(=O)Me | Cl | C(=O)Me | O |
| C-3584 | Me | single bond | H | c-Pr(1-CN) | C(=O)Me | Cl | C(=O)Me | O |
| C-3585 | Me(R) | single bond | H | c-Pr(1-CN) | C(=O)Me | Cl | C(=O)Me | O |
| C-3586 | Me(S) | single bond | H | c-Pr(1-CN) | C(=O)Me | Cl | C(=O)Me | O |
| C-3587 | H | CH₂ | Me | c-Pr(1-CN) | C(=O)Me | Cl | C(=O)Me | O |
| C-3588 | Me | CH₂ | H | c-Pr(1-CN) | C(=O)Me | Cl | C(=O)Me | O |
| C-3589 | H | CH(Me) | H | c-Pr(1-CN) | C(=O)Me | Cl | C(=O)Me | O |
| C-3590 | H | CH₂CH₂ | Me | c-Pr(1-CN) | C(=O)Me | Cl | C(=O)Me | O |
| C-3591 | Me | CH₂CH₂ | H | c-Pr(1-CN) | C(=O)Me | Cl | C(=O)Me | O |
| C-3592 | H | single bond | Me | c-Pr(1-CN) | C(=O)Me | Me | C(=O)Me | O |
| C-3593 | Me | single bond | H | c-Pr(1-CN) | C(=O)Me | Me | C(=O)Me | O |
| C-3594 | Me(R) | single bond | H | c-Pr(1-CN) | C(=O)Me | Me | C(=O)Me | O |
| C-3595 | Me(S) | single bond | H | c-Pr(1-CN) | C(=O)Me | Me | C(=O)Me | O |
| C-3596 | H | CH₂ | Me | c-Pr(1-CN) | C(=O)Me | Me | C(=O)Me | O |
| C-3597 | Me | CH₂ | H | c-Pr(1-CN) | C(=O)Me | Me | C(=O)Me | O |
| C-3598 | H | CH(Me) | H | c-Pr(1-CN) | C(=O)Me | Me | C(=O)Me | O |
| C-3599 | H | CH₂CH₂ | Me | c-Pr(1-CN) | C(=O)Me | Me | C(=O)Me | O |
| C-3600 | Me | CH₂CH₂ | H | c-Pr(1-CN) | C(=O)Me | Me | C(=O)Me | O |
| C-3601 | H | single bond | Me | c-Pr(1-CN) | C(=O)(*i*-Pr) | Cl | Me | O |
| C-3602 | Me | single bond | H | c-Pr(1-CN) | C(=O)(*i*-Pr) | Cl | Me | O |

**[Table 309]**

| Compound Number | Y | W | Z | R¹ | R² | R³ | R¹⁰ | A |
|---|---|---|---|---|---|---|---|---|
| C-3603 | Me(R) | single bond | H | c-Pr(1-CN) | C(=O)(*i*-Pr) | Cl | Me | O |
| C-3604 | Me(S) | single bond | H | c-Pr(1-CN) | C(=O)(*i*-Pr) | Cl | Me | O |
| C-3605 | H | CH₂ | Me | c-Pr(1-CN) | C(=O)(*i*-Pr) | Cl | Me | O |
| C-3606 | Me | CH₂ | H | c-Pr(1-CN) | C(=O)(*i*-Pr) | Cl | Me | O |
| C-3607 | H | CH(Me) | H | c-Pr(1-CN) | C(=O)(*i*-Pr) | Cl | Me | O |
| C-3608 | H | CH₂CH₂ | Me | c-Pr(1-CN) | C(=O)(*i*-Pr) | Cl | Me | O |
| C-3609 | Me | CH₂CH₂ | H | c-Pr(1-CN) | C(=O)(*i*-Pr) | Cl | Me | O |
| C-3610 | H | single bond | Me | c-Pr(1-CN) | C(=O)(*i*-Pr) | Me | Me | O |
| C-3611 | Me | single bond | H | c-Pr(1-CN) | C(=O)(*i*-Pr) | Me | Me | O |
| C-3612 | Me(R) | single bond | H | c-Pr(1-CN) | C(=O)(*i*-Pr) | Me | Me | O |
| C-3613 | Me(S) | single bond | H | c-Pr(1-CN) | C(=O)(*i*-Pr) | Me | Me | O |
| C-3614 | H | CH₂ | Me | c-Pr(1-CN) | C(=O)(*i*-Pr) | Me | Me | O |
| C-3615 | Me | CH₂ | H | c-Pr(1-CN) | C(=O)(*i*-Pr) | Me | Me | O |
| C-3616 | H | CH(Me) | H | c-Pr(1-CN) | C(=O)(*i*-Pr) | Me | Me | O |
| C-3617 | H | CH₂CH₂ | Me | c-Pr(1-CN) | C(=O)(*i*-Pr) | Me | Me | O |
| C-3618 | Me | CH₂CH₂ | H | c-Pr(1-CN) | C(=O)(*i*-Pr) | Me | Me | O |
| C-3619 | H | single bond | Me | c-Pr(1-CN) | C(=O)(*i*-Pr) | Cl | Et | O |
| C-3620 | Me | single bond | H | c-Pr(1-CN) | C(=O)(*i*-Pr) | Cl | Et | O |
| C-3621 | Me(R) | single bond | H | c-Pr(1-CN) | C(=O)(*i*-Pr) | Cl | Et | O |
| C-3622 | Me(S) | single bond | H | c-Pr(1-CN) | C(=O)(*i*-Pr) | Cl | Et | O |
| C-3623 | H | CH₂ | Me | c-Pr(1-CN) | C(=O)(*i*-Pr) | Cl | Et | O |
| C-3624 | Me | CH₂ | H | c-Pr(1-CN) | C(=O)(*i*-Pr) | Cl | Et | O |
| C-3625 | H | CH(Me) | H | c-Pr(1-CN) | C(=O)(*i*-Pr) | Cl | Et | O |
| C-3626 | H | CH₂CH₂ | Me | c-Pr(1-CN) | C(=O)(*i*-Pr) | Cl | Et | O |
| C-3627 | Me | CH₂CH₂ | H | c-Pr(1-CN) | C(=O)(*i*-Pr) | Cl | Et | O |
| C-3628 | H | single bond | Me | c-Pr(1-CN) | C(=O)(*i*-Pr) | Me | Et | O |
| C-3629 | Me | single bond | H | c-Pr(1-CN) | C(=O)(*i*-Pr) | Me | Et | O |
| C-3630 | Me(R) | single bond | H | c-Pr(1-CN) | C(=O)(*i*-Pr) | Me | Et | O |
| C-3631 | Me(S) | single bond | H | c-Pr(1-CN) | C(=O)(*i*-Pr) | Me | Et | O |
| C-3632 | H | CH₂ | Me | c-Pr(1-CN) | C(=O)(*i*-Pr) | Me | Et | O |
| C-3633 | Me | CH₂ | H | c-Pr(1-CN) | C(=O)(*i*-Pr) | Me | Et | O |
| C-3634 | H | CH(Me) | H | c-Pr(1-CN) | C(=O)(*i*-Pr) | Me | Et | O |
| C-3635 | H | CH₂CH₂ | Me | c-Pr(1-CN) | C(=O)(*i*-Pr) | Me | Et | O |
| C-3636 | Me | CH₂CH₂ | H | c-Pr(1-CN) | C(=O)(*i*-Pr) | Me | Et | O |
| C-3637 | H | single bond | Me | c-Pr(1-CN) | C(=O)(*i*-Pr) | Cl | CH₂CF₃ | O |
| C-3638 | Me | single bond | H | c-Pr(1-CN) | C(=O)(*i*-Pr) | Cl | CH₂CF₃ | O |
| C-3639 | Me(R) | single bond | H | c-Pr(1-CN) | C(=O)(*i*-Pr) | Cl | CH₂CF₃ | O |
| C-3640 | Me(S) | single bond | H | c-Pr(1-CN) | C(=O)(*i*-Pr) | Cl | CH₂CF₃ | O |
| C-3641 | H | CH₂ | Me | c-Pr(1-CN) | C(=O)(*i*-Pr) | Cl | CH₂CF₃ | O |
| C-3642 | Me | CH₂ | H | c-Pr(1-CN) | C(=O)(*i*-Pr) | Cl | CH₂CF₃ | O |
| C-3643 | H | CH(Me) | H | c-Pr(1-CN) | C(=O)(*i*-Pr) | Cl | CH₂CF₃ | O |
| C-3644 | H | CH₂CH₂ | Me | c-Pr(1-CN) | C(=O)(*i*-Pr) | Cl | CH₂CF₃ | O |
| C-3645 | Me | CH₂CH₂ | H | c-Pr(1-CN) | C(=O)(*i*-Pr) | Cl | CH₂CF₃ | O |
| C-3646 | H | single bond | Me | c-Pr(1-CN) | C(=O)(*i*-Pr) | Me | CH₂CF₃ | O |

**[Table 310]**

| Compound Number | Y | W | Z | R¹ | R² | R³ | R¹⁰ | A |
|---|---|---|---|---|---|---|---|---|
| C-3647 | Me | single bond | H | c-Pr(1-CN) | C(=O)(*i*-Pr) | Me | CH₂CF₃ | O |
| C-3648 | Me(R) | single bond | H | c-Pr(1-CN) | C(=O)(*i*-Pr) | Me | CH₂CF₃ | O |
| C-3649 | Me(S) | single bond | H | c-Pr(1-CN) | C(=O)(*i*-Pr) | Me | CH₂CF₃ | O |
| C-3650 | H | CH₂ | Me | c-Pr(1-CN) | C(=O)(*i*-Pr) | Me | CH₂CF₃ | O |
| C-3651 | Me | CH₂ | H | c-Pr(1-CN) | C(=O)(*i*-Pr) | Me | CH₂CF₃ | O |
| C-3652 | H | CH(Me) | H | c-Pr(1-CN) | C(=O)(*i*-Pr) | Me | CH₂CF₃ | O |
| C-3653 | H | CH₂CH₂ | Me | c-Pr(1-CN) | C(=O)(*i*-Pr) | Me | CH₂CF₃ | O |
| C-3654 | Me | CH₂CH₂ | H | c-Pr(1-CN) | C(=O)(*i*-Pr) | Me | CH₂CF₃ | O |
| C-3655 | H | single bond | Me | c-Pr(1-CN) | C(=O)(*i*-Pr) | Cl | CH₂OMe | O |
| C-3656 | Me | single bond | H | c-Pr(1-CN) | C(=O)(*i*-Pr) | Cl | CH₂OMe | O |
| C-3657 | Me(R) | single bond | H | c-Pr(1-CN) | C(=O)(*i*-Pr) | Cl | CH₂OMe | O |
| C-3658 | Me(S) | single bond | H | c-Pr(1-CN) | C(=O)(*i*-Pr) | Cl | CH₂OMe | O |
| C-3659 | H | CH₂ | Me | c-Pr(1-CN) | C(=O)(*i*-Pr) | Cl | CH₂OMe | O |
| C-3660 | Me | CH₂ | H | c-Pr(1-CN) | C(=O)(*i*-Pr) | Cl | CH₂OMe | O |
| C-3661 | H | CH(Me) | H | c-Pr(1-CN) | C(=O)(*i*-Pr) | Cl | CH₂OMe | O |
| C-3662 | H | CH₂CH₂ | Me | c-Pr(1-CN) | C(=O)(*i*-Pr) | Cl | CH₂OMe | O |
| C-3663 | Me | CH₂CH₂ | H | c-Pr(1-CN) | C(=O)(*i*-Pr) | Cl | CH₂OMe | O |
| C-3664 | H | single bond | Me | c-Pr(1-CN) | C(=O)(*i*-Pr) | Me | CH₂OMe | O |
| C-3665 | Me | single bond | H | c-Pr(1-CN) | C(=O)(*i*-Pr) | Me | CH₂OMe | O |
| C-3666 | Me(R) | single bond | H | c-Pr(1-CN) | C(=O)(*i*-Pr) | Me | CH₂OMe | O |
| C-3667 | Me(S) | single bond | H | c-Pr(1-CN) | C(=O)(*i*-Pr) | Me | CH₂OMe | O |
| C-3668 | H | CH₂ | Me | c-Pr(1-CN) | C(=O)(*i*-Pr) | Me | CH₂OMe | O |
| C-3669 | Me | CH₂ | H | c-Pr(1-CN) | C(=O)(*i*-Pr) | Me | CH₂OMe | O |
| C-3670 | H | CH(Me) | H | c-Pr(1-CN) | C(=O)(*i*-Pr) | Me | CH₂OMe | O |
| C-3671 | H | CH₂CH₂ | Me | c-Pr(1-CN) | C(=O)(*i*-Pr) | Me | CH₂OMe | O |
| C-3672 | Me | CH₂CH₂ | H | c-Pr(1-CN) | C(=O)(*i*-Pr) | Me | CH₂OMe | O |
| C-3673 | H | single bond | Me | c-Pr(1-CN) | C(=O)(*i*-Pr) | Cl | CH₂CN | O |
| C-3674 | Me | single bond | H | c-Pr(1-CN) | C(=O)(*i*-Pr) | Cl | CH₂CN | O |
| C-3675 | Me(R) | single bond | H | c-Pr(1-CN) | C(=O)(*i*-Pr) | Cl | CH₂CN | O |
| C-3676 | Me(S) | single bond | H | c-Pr(1-CN) | C(=O)(*i*-Pr) | Cl | CH₂CN | O |
| C-3677 | H | CH₂ | Me | c-Pr(1-CN) | C(=O)(*i*-Pr) | Cl | CH₂CN | O |
| C-3678 | Me | CH₂ | H | c-Pr(1-CN) | C(=O)(*i*-Pr) | Cl | CH₂CN | O |
| C-3679 | H | CH(Me) | H | c-Pr(1-CN) | C(=O)(*i*-Pr) | Cl | CH₂CN | O |
| C-3680 | H | CH₂CH₂ | Me | c-Pr(1-CN) | C(=O)(*i*-Pr) | Cl | CH₂CN | O |
| C-3681 | Me | CH₂CH₂ | H | c-Pr(1-CN) | C(=O)(*i*-Pr) | Cl | CH₂CN | O |
| C-3682 | H | single bond | Me | c-Pr(1-CN) | C(=O)(*i*-Pr) | Me | CH₂CN | O |
| C-3683 | Me | single bond | H | c-Pr(1-CN) | C(=O)(*i*-Pr) | Me | CH₂CN | O |
| C-3684 | Me(R) | single bond | H | c-Pr(1-CN) | C(=O)(*i*-Pr) | Me | CH₂CN | O |
| C-3685 | Me(S) | single bond | H | c-Pr(1-CN) | C(=O)(*i*-Pr) | Me | CH₂CN | O |
| C-3686 | H | CH₂ | Me | c-Pr(1-CN) | C(=O)(*i*-Pr) | Me | CH₂CN | O |
| C-3687 | Me | CH₂ | H | c-Pr(1-CN) | C(=O)(*i*-Pr) | Me | CH₂CN | O |
| C-3688 | H | CH(Me) | H | c-Pr(1-CN) | C(=O)(*i*-Pr) | Me | CH₂CN | O |
| C-3689 | H | CH₂CH₂ | Me | c-Pr(1-CN) | C(=O)(*i*-Pr) | Me | CH₂CN | O |
| C-3690 | Me | CH₂CH₂ | H | c-Pr(1-CN) | C(=O)(*i*-Pr) | Me | CH₂CN | O |

**[Table 311]**

| Compound Number | Y | W | Z | R¹ | R² | R³ | R¹⁰ | A |
|---|---|---|---|---|---|---|---|---|
| C-3691 | H | single bond | Me | c-Pr(1-CN) | C(=O)(*i*-Pr) | Cl | C(=O)Me | O |
| C-3692 | Me | single bond | H | c-Pr(1-CN) | C(=O)(*i*-Pr) | Cl | C(=O)Me | O |
| C-3693 | Me(R) | single bond | H | c-Pr(1-CN) | C(=O)(*i*-Pr) | Cl | C(=O)Me | O |
| C-3694 | Me(S) | single bond | H | c-Pr(1-CN) | C(=O)(*i*-Pr) | Cl | C(=O)Me | O |
| C-3695 | H | CH₂ | Me | c-Pr(1-CN) | C(=O)(*i*-Pr) | Cl | C(=O)Me | O |
| C-3696 | Me | CH₂ | H | c-Pr(1-CN) | C(=O)(*i*-Pr) | Cl | C(=O)Me | O |
| C-3697 | H | CH(Me) | H | c-Pr(1-CN) | C(=O)(*i*-Pr) | Cl | C(=O)Me | O |
| C-3698 | H | CH₂CH₂ | Me | c-Pr(1-CN) | C(=O)(*i*-Pr) | Cl | C(=O)Me | O |
| C-3699 | Me | CH₂CH₂ | H | c-Pr(1-CN) | C(=O)(*i*-Pr) | Cl | C(=O)Me | O |
| C-3700 | H | single bond | Me | c-Pr(1-CN) | C(=O)(*i*-Pr) | Me | C(=O)Me | O |
| C-3701 | Me | single bond | H | c-Pr(1-CN) | C(=O)(*i*-Pr) | Me | C(=O)Me | O |
| C-3702 | Me(R) | single bond | H | c-Pr(1-CN) | C(=O)(*i*-Pr) | Me | C(=O)Me | O |
| C-3703 | Me(S) | single bond | H | c-Pr(1-CN) | C(=O)(*i*-Pr) | Me | C(=O)Me | O |
| C-3704 | H | CH₂ | Me | c-Pr(1-CN) | C(=O)(*i*-Pr) | Me | C(=O)Me | O |
| C-3705 | Me | CH₂ | H | c-Pr(1-CN) | C(=O)(*i*-Pr) | Me | C(=O)Me | O |
| C-3706 | H | CH(Me) | H | c-Pr(1-CN) | C(=O)(*i*-Pr) | Me | C(=O)Me | O |
| C-3707 | H | CH₂CH₂ | Me | c-Pr(1-CN) | C(=O)(*i*-Pr) | Me | C(=O)Me | O |
| C-3708 | Me | CH₂CH₂ | H | c-Pr(1-CN) | C(=O)(*i*-Pr) | Me | C(=O)Me | O |
| C-3709 | H | single bond | Me | c-Pr(1-CN) | C(=O)Ph | Cl | Me | O |
| C-3710 | Me | single bond | H | c-Pr(1-CN) | C(=O)Ph | Cl | Me | O |
| C-3711 | Me(R) | single bond | H | c-Pr(1-CN) | C(=O)Ph | Cl | Me | O |
| C-3712 | Me(S) | single bond | H | c-Pr(1-CN) | C(=O)Ph | Cl | Me | O |
| C-3713 | H | CH₂ | Me | c-Pr(1-CN) | C(=O)Ph | Cl | Me | O |
| C-3714 | Me | CH₂ | H | c-Pr(1-CN) | C(=O)Ph | Cl | Me | O |
| C-3715 | H | CH(Me) | H | c-Pr(1-CN) | C(=O)Ph | Cl | Me | O |
| C-3716 | H | CH₂CH₂ | Me | c-Pr(1-CN) | C(=O)Ph | Cl | Me | O |
| C-3717 | Me | CH₂CH₂ | H | c-Pr(1-CN) | C(=O)Ph | Cl | Me | O |
| C-3718 | H | single bond | Me | c-Pr(1-CN) | C(=O)Ph | Me | Me | O |
| C-3719 | Me | single bond | H | c-Pr(1-CN) | C(=O)Ph | Me | Me | O |
| C-3720 | Me(R) | single bond | H | c-Pr(1-CN) | C(=O)Ph | Me | Me | O |
| C-3721 | Me(S) | single bond | H | c-Pr(1-CN) | C(=O)Ph | Me | Me | O |
| C-3722 | H | CH₂ | Me | c-Pr(1-CN) | C(=O)Ph | Me | Me | O |
| C-3723 | Me | CH₂ | H | c-Pr(1-CN) | C(=O)Ph | Me | Me | O |
| C-3724 | H | CH(Me) | H | c-Pr(1-CN) | C(=O)Ph | Me | Me | O |
| C-3725 | H | CH₂CH₂ | Me | c-Pr(1-CN) | C(=O)Ph | Me | Me | O |
| C-3726 | Me | CH₂CH₂ | H | c-Pr(1-CN) | C(=O)Ph | Me | Me | O |
| C-3727 | H | single bond | Me | c-Pr(1-CN) | C(=O)Ph | Cl | Et | O |
| C-3728 | Me | single bond | H | c-Pr(1-CN) | C(=O)Ph | Cl | Et | O |
| C-3729 | Me(R) | single bond | H | c-Pr(1-CN) | C(=O)Ph | Cl | Et | O |
| C-3730 | Me(S) | single bond | H | c-Pr(1-CN) | C(=O)Ph | Cl | Et | O |
| C-3731 | H | CH₂ | Me | c-Pr(1-CN) | C(=O)Ph | Cl | Et | O |
| C-3732 | Me | CH₂ | H | c-Pr(1-CN) | C(=O)Ph | Cl | Et | O |
| C-3733 | H | CH(Me) | H | c-Pr(1-CN) | C(=O)Ph | Cl | Et | O |
| C-3734 | H | CH₂CH₂ | Me | c-Pr(1-CN) | C(=O)Ph | Cl | Et | O |

**[Table 312]**

| Compound Number | Y | W | Z | R¹ | R² | R³ | R¹⁰ | A |
|---|---|---|---|---|---|---|---|---|
| C-3735 | Me | CH₂CH₂ | H | c-Pr(1-CN) | C(=O)Ph | Cl | Et | O |
| C-3736 | H | single bond | Me | c-Pr(1-CN) | C(=O)Ph | Me | Et | O |
| C-3737 | Me | single bond | H | c-Pr(1-CN) | C(=O)Ph | Me | Et | O |
| C-3738 | Me(R) | single bond | H | c-Pr(1-CN) | C(=O)Ph | Me | Et | O |
| C-3739 | Me(S) | single bond | H | c-Pr(1-CN) | C(=O)Ph | Me | Et | O |
| C-3740 | H | CH₂ | Me | c-Pr(1-CN) | C(=O)Ph | Me | Et | O |
| C-3741 | Me | CH₂ | H | c-Pr(1-CN) | C(=O)Ph | Me | Et | O |
| C-3742 | H | CH(Me) | H | c-Pr(1-CN) | C(=O)Ph | Me | Et | O |
| C-3743 | H | CH₂CH₂ | Me | c-Pr(1-CN) | C(=O)Ph | Me | Et | O |
| C-3744 | Me | CH₂CH₂ | H | c-Pr(1-CN) | C(=O)Ph | Me | Et | O |
| C-3745 | H | single bond | Me | c-Pr(1-CN) | C(=O)Ph | Cl | CH₂CF₃ | O |
| C-3746 | Me | single bond | H | c-Pr(1-CN) | C(=O)Ph | Cl | CH₂CF₃ | O |
| C-3747 | Me(R) | single bond | H | c-Pr(1-CN) | C(=O)Ph | Cl | CH₂CF₃ | O |
| C-3748 | Me(S) | single bond | H | c-Pr(1-CN) | C(=O)Ph | Cl | CH₂CF₃ | O |
| C-3749 | H | CH₂ | Me | c-Pr(1-CN) | C(=O)Ph | Cl | CH₂CF₃ | O |
| C-3750 | Me | CH₂ | H | c-Pr(1-CN) | C(=O)Ph | Cl | CH₂CF₃ | O |
| C-3751 | H | CH(Me) | H | c-Pr(1-CN) | C(=O)Ph | Cl | CH₂CF₃ | O |
| C-3752 | H | CH₂CH₂ | Me | c-Pr(1-CN) | C(=O)Ph | Cl | CH₂CF₃ | O |
| C-3753 | Me | CH₂CH₂ | H | c-Pr(1-CN) | C(=O)Ph | Cl | CH₂CF₃ | O |
| C-3754 | H | single bond | Me | c-Pr(1-CN) | C(=O)Ph | Me | CH₂CF₃ | O |
| C-3755 | Me | single bond | H | c-Pr(1-CN) | C(=O)Ph | Me | CH₂CF₃ | O |
| C-3756 | Me(R) | single bond | H | c-Pr(1-CN) | C(=O)Ph | Me | CH₂CF₃ | O |
| C-3757 | Me(S) | single bond | H | c-Pr(1-CN) | C(=O)Ph | Me | CH₂CF₃ | O |
| C-3758 | H | CH₂ | Me | c-Pr(1-CN) | C(=O)Ph | Me | CH₂CF₃ | O |
| C-3759 | Me | CH₂ | H | c-Pr(1-CN) | C(=O)Ph | Me | CH₂CF₃ | O |
| C-3760 | H | CH(Me) | H | c-Pr(1-CN) | C(=O)Ph | Me | CH₂CF₃ | O |
| C-3761 | H | CH₂CH₂ | Me | c-Pr(1-CN) | C(=O)Ph | Me | CH₂CF₃ | O |
| C-3762 | Me | CH₂CH₂ | H | c-Pr(1-CN) | C(=O)Ph | Me | CH₂CF₃ | O |
| C-3763 | H | single bond | Me | c-Pr(1-CN) | C(=O)Ph | Cl | CH₂OMe | O |
| C-3764 | Me | single bond | H | c-Pr(1-CN) | C(=O)Ph | Cl | CH₂OMe | O |
| C-3765 | Me(R) | single bond | H | c-Pr(1-CN) | C(=O)Ph | Cl | CH₂OMe | O |
| C-3766 | Me(S) | single bond | H | c-Pr(1-CN) | C(=O)Ph | Cl | CH₂OMe | O |
| C-3767 | H | CH₂ | Me | c-Pr(1-CN) | C(=O)Ph | Cl | CH₂OMe | O |
| C-3768 | Me | CH₂ | H | c-Pr(1-CN) | C(=O)Ph | Cl | CH₂OMe | O |
| C-3769 | H | CH(Me) | H | c-Pr(1-CN) | C(=O)Ph | Cl | CH₂OMe | O |
| C-3770 | H | CH₂CH₂ | Me | c-Pr(1-CN) | C(=O)Ph | Cl | CH₂OMe | O |
| C-3771 | Me | CH₂CH₂ | H | c-Pr(1-CN) | C(=O)Ph | Cl | CH₂OMe | O |
| C-3772 | H | single bond | Me | c-Pr(1-CN) | C(=O)Ph | Me | CH₂OMe | O |
| C-3773 | Me | single bond | H | c-Pr(1-CN) | C(=O)Ph | Me | CH₂OMe | O |
| C-3774 | Me(R) | single bond | H | c-Pr(1-CN) | C(=O)Ph | Me | CH₂OMe | O |
| C-3775 | Me(S) | single bond | H | c-Pr(1-CN) | C(=O)Ph | Me | CH₂OMe | O |
| C-3776 | H | CH₂ | Me | c-Pr(1-CN) | C(=O)Ph | Me | CH₂OMe | O |
| C-3777 | Me | CH₂ | H | c-Pr(1-CN) | C(=O)Ph | Me | CH₂OMe | O |
| C-3778 | H | CH(Me) | H | c-Pr(1-CN) | C(=O)Ph | Me | CH₂OMe | O |

**[Table 313]**

| Compound Number | Y | W | Z | R¹ | R² | R³ | R¹⁰ | A |
|---|---|---|---|---|---|---|---|---|
| C-3779 | H | CH₂CH₂ | Me | c-Pr(1-CN) | C(=O)Ph | Me | CH₂OMe | O |
| C-3780 | Me | CH₂CH₂ | H | c-Pr(1-CN) | C(=O)Ph | Me | CH₂OMe | O |
| C-3781 | H | single bond | Me | c-Pr(1-CN) | C(=O)Ph | Cl | CH₂CN | O |
| C-3782 | Me | single bond | H | c-Pr(1-CN) | C(=O)Ph | Cl | CH₂CN | O |
| C-3783 | Me(R) | single bond | H | c-Pr(1-CN) | C(=O)Ph | Cl | CH₂CN | O |
| C-3784 | Me(S) | single bond | H | c-Pr(1-CN) | C(=O)Ph | Cl | CH₂CN | O |
| C-3785 | H | CH₂ | Me | c-Pr(1-CN) | C(=O)Ph | Cl | CH₂CN | O |
| C-3786 | Me | CH₂ | H | c-Pr(1-CN) | C(=O)Ph | Cl | CH₂CN | O |
| C-3787 | H | CH(Me) | H | c-Pr(1-CN) | C(=O)Ph | Cl | CH₂CN | O |
| C-3788 | H | CH₂CH₂ | Me | c-Pr(1-CN) | C(=O)Ph | Cl | CH₂CN | O |
| C-3789 | Me | CH₂CH₂ | H | c-Pr(1-CN) | C(=O)Ph | Cl | CH₂CN | O |
| C-3790 | H | single bond | Me | c-Pr(1-CN) | C(=O)Ph | Me | CH₂CN | O |
| C-3791 | Me | single bond | H | c-Pr(1-CN) | C(=O)Ph | Me | CH₂CN | O |
| C-3792 | Me(R) | single bond | H | c-Pr(1-CN) | C(=O)Ph | Me | CH₂CN | O |
| C-3793 | Me(S) | single bond | H | c-Pr(1-CN) | C(=O)Ph | Me | CH₂CN | O |
| C-3794 | H | CH₂ | Me | c-Pr(1-CN) | C(=O)Ph | Me | CH₂CN | O |
| C-3795 | Me | CH₂ | H | c-Pr(1-CN) | C(=O)Ph | Me | CH₂CN | O |
| C-3796 | H | CH(Me) | H | c-Pr(1-CN) | C(=O)Ph | Me | CH₂CN | O |
| C-3797 | H | CH₂CH₂ | Me | c-Pr(1-CN) | C(=O)Ph | Me | CH₂CN | O |
| C-3798 | Me | CH₂CH₂ | H | c-Pr(1-CN) | C(=O)Ph | Me | CH₂CN | O |
| C-3799 | H | single bond | Me | c-Pr(1-CN) | C(=O)Ph | Cl | C(=O)Me | O |
| C-3800 | Me | single bond | H | c-Pr(1-CN) | C(=O)Ph | Cl | C(=O)Me | O |
| C-3801 | Me(R) | single bond | H | c-Pr(1-CN) | C(=O)Ph | Cl | C(=O)Me | O |
| C-3802 | Me(S) | single bond | H | c-Pr(1-CN) | C(=O)Ph | Cl | C(=O)Me | O |
| C-3803 | H | CH₂ | Me | c-Pr(1-CN) | C(=O)Ph | Cl | C(=O)Me | O |
| C-3804 | Me | CH₂ | H | c-Pr(1-CN) | C(=O)Ph | Cl | C(=O)Me | O |
| C-3805 | H | CH(Me) | H | c-Pr(1-CN) | C(=O)Ph | Cl | C(=O)Me | O |
| C-3806 | H | CH₂CH₂ | Me | c-Pr(1-CN) | C(=O)Ph | Cl | C(=O)Me | O |
| C-3807 | Me | CH₂CH₂ | H | c-Pr(1-CN) | C(=O)Ph | Cl | C(=O)Me | O |
| C-3808 | H | single bond | Me | c-Pr(1-CN) | C(=O)Ph | Me | C(=O)Me | O |
| C-3809 | Me | single bond | H | c-Pr(1-CN) | C(=O)Ph | Me | C(=O)Me | O |
| C-3810 | Me(R) | single bond | H | c-Pr(1-CN) | C(=O)Ph | Me | C(=O)Me | O |
| C-3811 | Me(S) | single bond | H | c-Pr(1-CN) | C(=O)Ph | Me | C(=O)Me | O |
| C-3812 | H | CH₂ | Me | c-Pr(1-CN) | C(=O)Ph | Me | C(=O)Me | O |
| C-3813 | Me | CH₂ | H | c-Pr(1-CN) | C(=O)Ph | Me | C(=O)Me | O |
| C-3814 | H | CH(Me) | H | c-Pr(1-CN) | C(=O)Ph | Me | C(=O)Me | O |
| C-3815 | H | CH₂CH₂ | Me | c-Pr(1-CN) | C(=O)Ph | Me | C(=O)Me | O |
| C-3816 | Me | CH₂CH₂ | H | c-Pr(1-CN) | C(=O)Ph | Me | C(=O)Me | O |
| C-3817 | H | single bond | Me | c-Pr(1-CN) | C(=O)OEt | Cl | Me | O |
| C-3818 | Me | single bond | H | c-Pr(1-CN) | C(=O)OEt | Cl | Me | O |
| C-3819 | Me(R) | single bond | H | c-Pr(1-CN) | C(=O)OEt | Cl | Me | O |
| C-3820 | Me(S) | single bond | H | c-Pr(1-CN) | C(=O)OEt | Cl | Me | O |
| C-3821 | H | CH₂ | Me | c-Pr(1-CN) | C(=O)OEt | Cl | Me | O |
| C-3822 | Me | CH₂ | H | c-Pr(1-CN) | C(=O)OEt | Cl | Me | O |

**[Table 314]**

| Compound Number | Y | W | Z | R¹ | R² | R³ | R¹⁰ | A |
|---|---|---|---|---|---|---|---|---|
| C-3823 | H | CH(Me) | H | c-Pr(1-CN) | C(=O)OEt | Cl | Me | O |
| C-3824 | H | CH₂CH₂ | Me | c-Pr(1-CN) | C(=O)OEt | Cl | Me | O |
| C-3825 | Me | CH₂CH₂ | H | c-Pr(1-CN) | C(=O)OEt | Cl | Me | O |
| C-3826 | H | single bond | Me | c-Pr(1-CN) | C(=O)OEt | Me | Me | O |
| C-3827 | Me | single bond | H | c-Pr(1-CN) | C(=O)OEt | Me | Me | O |
| C-3828 | Me(R) | single bond | H | c-Pr(1-CN) | C(=O)OEt | Me | Me | O |
| C-3829 | Me(S) | single bond | H | c-Pr(1-CN) | C(=O)OEt | Me | Me | O |
| C-3830 | H | CH₂ | Me | c-Pr(1-CN) | C(=O)OEt | Me | Me | O |
| C-3831 | Me | CH₂ | H | c-Pr(1-CN) | C(=O)OEt | Me | Me | O |
| C-3832 | H | CH(Me) | H | c-Pr(1-CN) | C(=O)OEt | Me | Me | O |
| C-3833 | H | CH₂CH₂ | Me | c-Pr(1-CN) | C(=O)OEt | Me | Me | O |
| C-3834 | Me | CH₂CH₂ | H | c-Pr(1-CN) | C(=O)OEt | Me | Me | O |
| C-3835 | H | single bond | Me | c-Pr(1-CN) | C(=O)OEt | Cl | Et | O |
| C-3836 | Me | single bond | H | c-Pr(1-CN) | C(=O)OEt | Cl | Et | O |
| C-3837 | Me(R) | single bond | H | c-Pr(1-CN) | C(=O)OEt | Cl | Et | O |
| C-3838 | Me(S) | single bond | H | c-Pr(1-CN) | C(=O)OEt | Cl | Et | O |
| C-3839 | H | CH₂ | Me | c-Pr(1-CN) | C(=O)OEt | Cl | Et | O |
| C-3840 | Me | CH₂ | H | c-Pr(1-CN) | C(=O)OEt | Cl | Et | O |
| C-3841 | H | CH(Me) | H | c-Pr(1-CN) | C(=O)OEt | Cl | Et | O |
| C-3842 | H | CH₂CH₂ | Me | c-Pr(1-CN) | C(=O)OEt | Cl | Et | O |
| C-3843 | Me | CH₂CH₂ | H | c-Pr(1-CN) | C(=O)OEt | Cl | Et | O |
| C-3844 | H | single bond | Me | c-Pr(1-CN) | C(=O)OEt | Me | Et | O |
| C-3845 | Me | single bond | H | c-Pr(1-CN) | C(=O)OEt | Me | Et | O |
| C-3846 | Me(R) | single bond | H | c-Pr(1-CN) | C(=O)OEt | Me | Et | O |
| C-3847 | Me(S) | single bond | H | c-Pr(1-CN) | C(=O)OEt | Me | Et | O |
| C-3848 | H | CH₂ | Me | c-Pr(1-CN) | C(=O)OEt | Me | Et | O |
| C-3849 | Me | CH₂ | H | c-Pr(1-CN) | C(=O)OEt | Me | Et | O |
| C-3850 | H | CH(Me) | H | c-Pr(1-CN) | C(=O)OEt | Me | Et | O |
| C-3851 | H | CH₂CH₂ | Me | c-Pr(1-CN) | C(=O)OEt | Me | Et | O |
| C-3852 | Me | CH₂CH₂ | H | c-Pr(1-CN) | C(=O)OEt | Me | Et | O |
| C-3853 | H | single bond | Me | c-Pr(1-CN) | C(=O)OEt | Cl | CH₂CF₃ | O |
| C-3854 | Me | single bond | H | c-Pr(1-CN) | C(=O)OEt | Cl | CH₂CF₃ | O |
| C-3855 | Me(R) | single bond | H | c-Pr(1-CN) | C(=O)OEt | Cl | CH₂CF₃ | O |
| C-3856 | Me(S) | single bond | H | c-Pr(1-CN) | C(=O)OEt | Cl | CH₂CF₃ | O |
| C-3857 | H | CH₂ | Me | c-Pr(1-CN) | C(=O)OEt | Cl | CH₂CF₃ | O |
| C-3858 | Me | CH₂ | H | c-Pr(1-CN) | C(=O)OEt | Cl | CH₂CF₃ | O |
| C-3859 | H | CH(Me) | H | c-Pr(1-CN) | C(=O)OEt | Cl | CH₂CF₃ | O |
| C-3860 | H | CH₂CH₂ | Me | c-Pr(1-CN) | C(=O)OEt | Cl | CH₂CF₃ | O |
| C-3861 | Me | CH₂CH₂ | H | c-Pr(1-CN) | C(=O)OEt | Cl | CH₂CF₃ | O |
| C-3862 | H | single bond | Me | c-Pr(1-CN) | C(=O)OEt | Me | CH₂CF₃ | O |
| C-3863 | Me | single bond | H | c-Pr(1-CN) | C(=O)OEt | Me | CH₂CF₃ | O |
| C-3864 | Me(R) | single bond | H | c-Pr(1-CN) | C(=O)OEt | Me | CH₂CF₃ | O |
| C-3865 | Me(S) | single bond | H | c-Pr(1-CN) | C(=O)OEt | Me | CH₂CF₃ | O |
| C-3866 | H | CH₂ | Me | c-Pr(1-CN) | C(=O)OEt | Me | CH₂CF₃ | O |

**[Table 315]**

| Compound Number | Y | W | Z | R¹ | R² | R³ | R¹⁰ | A |
|---|---|---|---|---|---|---|---|---|
| C-3867 | Me | CH₂ | H | c-Pr(1-CN) | C(=O)OEt | Me | CH₂CF₃ | O |
| C-3868 | H | CH(Me) | H | c-Pr(1-CN) | C(=O)OEt | Me | CH₂CF₃ | O |
| C-3869 | H | CH₂CH₂ | Me | c-Pr(1-CN) | C(=O)OEt | Me | CH₂CF₃ | O |
| C-3870 | Me | CH₂CH₂ | H | c-Pr(1-CN) | C(=O)OEt | Me | CH₂CF₃ | O |
| C-3871 | H | single bond | Me | c-Pr(1-CN) | C(=O)OEt | Cl | CH₂OMe | O |
| C-3872 | Me | single bond | H | c-Pr(1-CN) | C(=O)OEt | Cl | CH₂OMe | O |
| C-3873 | Me(R) | single bond | H | c-Pr(1-CN) | C(=O)OEt | Cl | CH₂OMe | O |
| C-3874 | Me(S) | single bond | H | c-Pr(1-CN) | C(=O)OEt | Cl | CH₂OMe | O |
| C-3875 | H | CH₂ | Me | c-Pr(1-CN) | C(=O)OEt | Cl | CH₂OMe | O |
| C-3876 | Me | CH₂ | H | c-Pr(1-CN) | C(=O)OEt | Cl | CH₂OMe | O |
| C-3877 | H | CH(Me) | H | c-Pr(1-CN) | C(=O)OEt | Cl | CH₂OMe | O |
| C-3878 | H | CH₂CH₂ | Me | c-Pr(1-CN) | C(=O)OEt | Cl | CH₂OMe | O |
| C-3879 | Me | CH₂CH₂ | H | c-Pr(1-CN) | C(=O)OEt | Cl | CH₂OMe | O |
| C-3880 | H | single bond | Me | c-Pr(1-CN) | C(=O)OEt | Me | CH₂OMe | O |
| C-3881 | Me | single bond | H | c-Pr(1-CN) | C(=O)OEt | Me | CH₂OMe | O |
| C-3882 | Me(R) | single bond | H | c-Pr(1-CN) | C(=O)OEt | Me | CH₂OMe | O |
| C-3883 | Me(S) | single bond | H | c-Pr(1-CN) | C(=O)OEt | Me | CH₂OMe | O |
| C-3884 | H | CH₂ | Me | c-Pr(1-CN) | C(=O)OEt | Me | CH₂OMe | O |
| C-3885 | Me | CH₂ | H | c-Pr(1-CN) | C(=O)OEt | Me | CH₂OMe | O |
| C-3886 | H | CH(Me) | H | c-Pr(1-CN) | C(=O)OEt | Me | CH₂OMe | O |
| C-3887 | H | CH₂CH₂ | Me | c-Pr(1-CN) | C(=O)OEt | Me | CH₂OMe | O |
| C-3888 | Me | CH₂CH₂ | H | c-Pr(1-CN) | C(=O)OEt | Me | CH₂OMe | O |
| C-3889 | H | single bond | Me | c-Pr(1-CN) | C(=O)OEt | Cl | CH₂CN | O |
| C-3890 | Me | single bond | H | c-Pr(1-CN) | C(=O)OEt | Cl | CH₂CN | O |
| C-3891 | Me(R) | single bond | H | c-Pr(1-CN) | C(=O)OEt | Cl | CH₂CN | O |
| C-3892 | Me(S) | single bond | H | c-Pr(1-CN) | C(=O)OEt | Cl | CH₂CN | O |
| C-3893 | H | CH₂ | Me | c-Pr(1-CN) | C(=O)OEt | Cl | CH₂CN | O |
| C-3894 | Me | CH₂ | H | c-Pr(1-CN) | C(=O)OEt | Cl | CH₂CN | O |
| C-3895 | H | CH(Me) | H | c-Pr(1-CN) | C(=O)OEt | Cl | CH₂CN | O |
| C-3896 | H | CH₂CH₂ | Me | c-Pr(1-CN) | C(=O)OEt | Cl | CH₂CN | O |
| C-3897 | Me | CH₂CH₂ | H | c-Pr(1-CN) | C(=O)OEt | Cl | CH₂CN | O |
| C-3898 | H | single bond | Me | c-Pr(1-CN) | C(=O)OEt | Me | CH₂CN | O |
| C-3899 | Me | single bond | H | c-Pr(1-CN) | C(=O)OEt | Me | CH₂CN | O |
| C-3900 | Me(R) | single bond | H | c-Pr(1-CN) | C(=O)OEt | Me | CH₂CN | O |
| C-3901 | Me(S) | single bond | H | c-Pr(1-CN) | C(=O)OEt | Me | CH₂CN | O |
| C-3902 | H | CH₂ | Me | c-Pr(1-CN) | C(=O)OEt | Me | CH₂CN | O |
| C-3903 | Me | CH₂ | H | c-Pr(1-CN) | C(=O)OEt | Me | CH₂CN | O |
| C-3904 | H | CH(Me) | H | c-Pr(1-CN) | C(=O)OEt | Me | CH₂CN | O |
| C-3905 | H | CH₂CH₂ | Me | c-Pr(1-CN) | C(=O)OEt | Me | CH₂CN | O |
| C-3906 | Me | CH₂CH₂ | H | c-Pr(1-CN) | C(=O)OEt | Me | CH₂CN | O |
| C-3907 | H | single bond | Me | c-Pr(1-CN) | C(=O)OEt | Cl | C(=O)Me | O |
| C-3908 | Me | single bond | H | c-Pr(1-CN) | C(=O)OEt | Cl | C(=O)Me | O |
| C-3909 | Me(R) | single bond | H | c-Pr(1-CN) | C(=O)OEt | Cl | C(=O)Me | O |
| C-3910 | Me(S) | single bond | H | c-Pr(1-CN) | C(=O)OEt | Cl | C(=O)Me | O |

**[Table 316]**

| Compound Number | Y | W | Z | R¹ | R² | R³ | R¹⁰ | A |
|---|---|---|---|---|---|---|---|---|
| C-3911 | H | CH₂ | Me | c-Pr(1-CN) | C(=O)OEt | Cl | C(=O)Me | O |
| C-3912 | Me | CH₂ | H | c-Pr(1-CN) | C(=O)OEt | Cl | C(=O)Me | O |
| C-3913 | H | CH(Me) | H | c-Pr(1-CN) | C(=O)OEt | Cl | C(=O)Me | O |
| C-3914 | H | CH₂CH₂ | Me | c-Pr(1-CN) | C(=O)OEt | Cl | C(=O)Me | O |
| C-3915 | Me | CH₂CH₂ | H | c-Pr(1-CN) | C(=O)OEt | Cl | C(=O)Me | O |
| C-3916 | H | single bond | Me | c-Pr(1-CN) | C(=O)OEt | Me | C(=O)Me | O |
| C-3917 | Me | single bond | H | c-Pr(1-CN) | C(=O)OEt | Me | C(=O)Me | O |
| C-3918 | Me(R) | single bond | H | c-Pr(1-CN) | C(=O)OEt | Me | C(=O)Me | O |
| C-3919 | Me(S) | single bond | H | c-Pr(1-CN) | C(=O)OEt | Me | C(=O)Me | O |
| C-3920 | H | CH₂ | Me | c-Pr(1-CN) | C(=O)OEt | Me | C(=O)Me | O |
| C-3921 | Me | CH₂ | H | c-Pr(1-CN) | C(=O)OEt | Me | C(=O)Me | O |
| C-3922 | H | CH(Me) | H | c-Pr(1-CN) | C(=O)OEt | Me | C(=O)Me | O |
| C-3923 | H | CH₂CH₂ | Me | c-Pr(1-CN) | C(=O)OEt | Me | C(=O)Me | O |
| C-3924 | Me | CH₂CH₂ | H | c-Pr(1-CN) | C(=O)OEt | Me | C(=O)Me | O |
| C-3925 | H | single bond | Me | c-Pr(1-CN) | S(=O)₂Me | Cl | Me | O |
| C-3926 | Me | single bond | H | c-Pr(1-CN) | S(=O)₂Me | Cl | Me | O |
| C-3927 | Me(R) | single bond | H | c-Pr(1-CN) | S(=O)₂Me | Cl | Me | O |
| C-3928 | Me(S) | single bond | H | c-Pr(1-CN) | S(=O)₂Me | Cl | Me | O |
| C-3929 | H | CH₂ | Me | c-Pr(1-CN) | S(=O)₂Me | Cl | Me | O |
| C-3930 | Me | CH₂ | H | c-Pr(1-CN) | S(=O)₂Me | Cl | Me | O |
| C-3931 | H | CH(Me) | H | c-Pr(1-CN) | S(=O)₂Me | Cl | Me | O |
| C-3932 | H | CH₂CH₂ | Me | c-Pr(1-CN) | S(=O)₂Me | Cl | Me | O |
| C-3933 | Me | CH₂CH₂ | H | c-Pr(1-CN) | S(=O)₂Me | Cl | Me | O |
| C-3934 | H | single bond | Me | c-Pr(1-CN) | S(=O)₂Me | Me | Me | O |
| C-3935 | Me | single bond | H | c-Pr(1-CN) | S(=O)₂Me | Me | Me | O |
| C-3936 | Me(R) | single bond | H | c-Pr(1-CN) | S(=O)₂Me | Me | Me | O |
| C-3937 | Me(S) | single bond | H | c-Pr(1-CN) | S(=O)₂Me | Me | Me | O |
| C-3938 | H | CH₂ | Me | c-Pr(1-CN) | S(=O)₂Me | Me | Me | O |
| C-3939 | Me | CH₂ | H | c-Pr(1-CN) | S(=O)₂Me | Me | Me | O |
| C-3940 | H | CH(Me) | H | c-Pr(1-CN) | S(=O)₂Me | Me | Me | O |
| C-3941 | H | CH₂CH₂ | Me | c-Pr(1-CN) | S(=O)₂Me | Me | Me | O |
| C-3942 | Me | CH₂CH₂ | H | c-Pr(1-CN) | S(=O)₂Me | Me | Me | O |
| C-3943 | H | single bond | Me | c-Pr(1-CN) | S(=O)₂Me | Cl | Et | O |
| C-3944 | Me | single bond | H | c-Pr(1-CN) | S(=O)₂Me | Cl | Et | O |
| C-3945 | Me(R) | single bond | H | c-Pr(1-CN) | S(=O)₂Me | Cl | Et | O |
| C-3946 | Me(S) | single bond | H | c-Pr(1-CN) | S(=O)₂Me | Cl | Et | O |
| C-3947 | H | CH₂ | Me | c-Pr(1-CN) | S(=O)₂Me | Cl | Et | O |
| C-3948 | Me | CH₂ | H | c-Pr(1-CN) | S(=O)₂Me | Cl | Et | O |
| C-3949 | H | CH(Me) | H | c-Pr(1-CN) | S(=O)₂Me | Cl | Et | O |
| C-3950 | H | CH₂CH₂ | Me | c-Pr(1-CN) | S(=O)₂Me | Cl | Et | O |
| C-3951 | Me | CH₂CH₂ | H | c-Pr(1-CN) | S(=O)₂Me | Cl | Et | O |
| C-3952 | H | single bond | Me | c-Pr(1-CN) | S(=O)₂Me | Me | Et | O |
| C-3953 | Me | single bond | H | c-Pr(1-CN) | S(=O)₂Me | Me | Et | O |
| C-3954 | Me(R) | single bond | H | c-Pr(1-CN) | S(=O)₂Me | Me | Et | O |

**[Table 317]**

| Compound Number | Y | W | Z | R¹ | R² | R³ | R¹⁰ | A |
|---|---|---|---|---|---|---|---|---|
| C-3955 | Me(S) | single bond | H | c-Pr(1-CN) | S(=O)₂Me | Me | Et | O |
| C-3956 | H | CH₂ | Me | c-Pr(1-CN) | S(=O)₂Me | Me | Et | O |
| C-3957 | Me | CH₂ | H | c-Pr(1-CN) | S(=O)₂Me | Me | Et | O |
| C-3958 | H | CH(Me) | H | c-Pr(1-CN) | S(=O)₂Me | Me | Et | O |
| C-3959 | H | CH₂CH₂ | Me | c-Pr(1-CN) | S(=O)₂Me | Me | Et | O |
| C-3960 | Me | CH₂CH₂ | H | c-Pr(1-CN) | S(=O)₂Me | Me | Et | O |
| C-3961 | H | single bond | Me | c-Pr(1-CN) | S(=O)₂Me | Cl | CH₂CF₃ | O |
| C-3962 | Me | single bond | H | c-Pr(1-CN) | S(=O)₂Me | Cl | CH₂CF₃ | O |
| C-3963 | Me(R) | single bond | H | c-Pr(1-CN) | S(=O)₂Me | Cl | CH₂CF₃ | O |
| C-3964 | Me(S) | single bond | H | c-Pr(1-CN) | S(=O)₂Me | Cl | CH₂CF₃ | O |
| C-3965 | H | CH₂ | Me | c-Pr(1-CN) | S(=O)₂Me | Cl | CH₂CF₃ | O |
| C-3966 | Me | CH₂ | H | c-Pr(1-CN) | S(=O)₂Me | Cl | CH₂CF₃ | O |
| C-3967 | H | CH(Me) | H | c-Pr(1-CN) | S(=O)₂Me | Cl | CH₂CF₃ | O |
| C-3968 | H | CH₂CH₂ | Me | c-Pr(1-CN) | S(=O)₂Me | Cl | CH₂CF₃ | O |
| C-3969 | Me | CH₂CH₂ | H | c-Pr(1-CN) | S(=O)₂Me | Cl | CH₂CF₃ | O |
| C-3970 | H | single bond | Me | c-Pr(1-CN) | S(=O)₂Me | Me | CH₂CF₃ | O |
| C-3971 | Me | single bond | H | c-Pr(1-CN) | S(=O)₂Me | Me | CH₂CF₃ | O |
| C-3972 | Me(R) | single bond | H | c-Pr(1-CN) | S(=O)₂Me | Me | CH₂CF₃ | O |
| C-3973 | Me(S) | single bond | H | c-Pr(1-CN) | S(=O)₂Me | Me | CH₂CF₃ | O |
| C-3974 | H | CH₂ | Me | c-Pr(1-CN) | S(=O)₂Me | Me | CH₂CF₃ | O |
| C-3975 | Me | CH₂ | H | c-Pr(1-CN) | S(=O)₂Me | Me | CH₂CF₃ | O |
| C-3976 | H | CH(Me) | H | c-Pr(1-CN) | S(=O)₂Me | Me | CH₂CF₃ | O |
| C-3977 | H | CH₂CH₂ | Me | c-Pr(1-CN) | S(=O)₂Me | Me | CH₂CF₃ | O |
| C-3978 | Me | CH₂CH₂ | H | c-Pr(1-CN) | S(=O)₂Me | Me | CH₂CF₃ | O |
| C-3979 | H | single bond | Me | c-Pr(1-CN) | S(=O)₂Me | Cl | CH₂OMe | O |
| C-3980 | Me | single bond | H | c-Pr(1-CN) | S(=O)₂Me | Cl | CH₂OMe | O |
| C-3981 | Me(R) | single bond | H | c-Pr(1-CN) | S(=O)₂Me | Cl | CH₂OMe | O |
| C-3982 | Me(S) | single bond | H | c-Pr(1-CN) | S(=O)₂Me | Cl | CH₂OMe | O |
| C-3983 | H | CH₂ | Me | c-Pr(1-CN) | S(=O)₂Me | Cl | CH₂OMe | O |
| C-3984 | Me | CH₂ | H | c-Pr(1-CN) | S(=O)₂Me | Cl | CH₂OMe | O |
| C-3985 | H | CH(Me) | H | c-Pr(1-CN) | S(=O)₂Me | Cl | CH₂OMe | O |
| C-3986 | H | CH₂CH₂ | Me | c-Pr(1-CN) | S(=O)₂Me | Cl | CH₂OMe | O |
| C-3987 | Me | CH₂CH₂ | H | c-Pr(1-CN) | S(=O)₂Me | Cl | CH₂OMe | O |
| C-3988 | H | single bond | Me | c-Pr(1-CN) | S(=O)₂Me | Me | CH₂OMe | O |
| C-3989 | Me | single bond | H | c-Pr(1-CN) | S(=O)₂Me | Me | CH₂OMe | O |
| C-3990 | Me(R) | single bond | H | c-Pr(1-CN) | S(=O)₂Me | Me | CH₂OMe | O |
| C-3991 | Me(S) | single bond | H | c-Pr(1-CN) | S(=O)₂Me | Me | CH₂OMe | O |
| C-3992 | H | CH₂ | Me | c-Pr(1-CN) | S(=O)₂Me | Me | CH₂OMe | O |
| C-3993 | Me | CH₂ | H | c-Pr(1-CN) | S(=O)₂Me | Me | CH₂OMe | O |
| C-3994 | H | CH(Me) | H | c-Pr(1-CN) | S(=O)₂Me | Me | CH₂OMe | O |
| C-3995 | H | CH₂CH₂ | Me | c-Pr(1-CN) | S(=O)₂Me | Me | CH₂OMe | O |
| C-3996 | Me | CH₂CH₂ | H | c-Pr(1-CN) | S(=O)₂Me | Me | CH₂OMe | O |
| C-3997 | H | single bond | Me | c-Pr(1-CN) | S(=O)₂Me | Cl | CH₂CN | O |
| C-3998 | Me | single bond | H | c-Pr(1-CN) | S(=O)₂Me | Cl | CH₂CN | O |

**[Table 318]**

| Compound Number | Y | W | z | R¹ | R² | R³ | R¹⁰ | A |
|---|---|---|---|---|---|---|---|---|
| C-3999 | Me(R) | single bond | H | c-Pr(1-CN) | S(=O)₂Me | Cl | CH₂CN | O |
| C-4000 | Me(S) | single bond | H | c-Pr(1-CN) | S(=O)₂Me | Cl | CH₂CN | O |
| C-4001 | H | CH₂ | Me | c-Pr(1-CN) | S(=O)₂Me | Cl | CH₂CN | O |
| C-4002 | Me | CH₂ | H | c-Pr(1-CN) | S(=O)₂Me | Cl | CH₂CN | O |
| C-4003 | H | CH(Me) | H | c-Pr(1-CN) | S(=O)₂Me | Cl | CH₂CN | O |
| C-4004 | H | CH₂CH₂ | Me | c-Pr(1-CN) | S(=O)₂Me | Cl | CH₂CN | O |
| C-4005 | Me | CH₂CH₂ | H | c-Pr(1-CN) | S(=O)₂Me | Cl | CH₂CN | O |
| C-4006 | H | single bond | Me | c-Pr(1-CN) | S(=O)₂Me | Me | CH₂CN | O |
| C-4007 | Me | single bond | H | c-Pr(1-CN) | S(=O)₂Me | Me | CH₂CN | O |
| C-4008 | Me(R) | single bond | H | c-Pr(1-CN) | S(=O)₂Me | Me | CH₂CN | O |
| C-4009 | Me(S) | single bond | H | c-Pr(1-CN) | S(=O)₂Me | Me | CH₂CN | O |
| C-4010 | H | CH₂ | Me | c-Pr(1-CN) | S(=O)₂Me | Me | CH₂CN | O |
| C-4011 | Me | CH₂ | H | c-Pr(1-CN) | S(=O)₂Me | Me | CH₂CN | O |
| C-4012 | H | CH(Me) | H | c-Pr(1-CN) | S(=O)₂Me | Me | CH₂CN | O |
| C-4013 | H | CH₂CH₂ | Me | c-Pr(1-CN) | S(=O)₂Me | Me | CH₂CN | O |
| C-4014 | Me | CH₂CH₂ | H | c-Pr(1-CN) | S(=O)₂Me | Me | CH₂CN | O |
| C-4015 | H | single bond | Me | c-Pr(1-CN) | S(=O)₂Me | Cl | C(=O)Me | O |
| C-4016 | Me | single bond | H | c-Pr(1-CN) | S(=O)₂Me | Cl | C(=O)Me | O |
| C-4017 | Me(R) | single bond | H | c-Pr(1-CN) | S(=O)₂Me | Cl | C(=O)Me | O |
| C-4018 | Me(S) | single bond | H | c-Pr(1-CN) | S(=O)₂Me | Cl | C(=O)Me | O |
| C-4019 | H | CH₂ | Me | c-Pr(1-CN) | S(=O)₂Me | Cl | C(=O)Me | O |
| C-4020 | Me | CH₂ | H | c-Pr(1-CN) | S(=O)₂Me | Cl | C(=O)Me | O |
| C-4021 | H | CH(Me) | H | c-Pr(1-CN) | S(=O)₂Me | Cl | C(=O)Me | O |
| C-4022 | H | CH₂CH₂ | Me | c-Pr(1-CN) | S(=O)₂Me | Cl | C(=O)Me | O |
| C-4023 | Me | CH₂CH₂ | H | c-Pr(1-CN) | S(=O)₂Me | Cl | C(=O)Me | O |
| C-4024 | H | single bond | Me | c-Pr(1-CN) | S(=O)₂Me | Me | C(=O)Me | O |
| C-4025 | Me | single bond | H | c-Pr(1-CN) | S(=O)₂Me | Me | C(=O)Me | O |
| C-4026 | Me(R) | single bond | H | c-Pr(1-CN) | S(=O)₂Me | Me | C(=O)Me | O |
| C-4027 | Me(S) | single bond | H | c-Pr(1-CN) | S(=O)₂Me | Me | C(=O)Me | O |
| C-4028 | H | CH₂ | Me | c-Pr(1-CN) | S(=O)₂Me | Me | C(=O)Me | O |
| C-4029 | Me | CH₂ | H | c-Pr(1-CN) | S(=O)₂Me | Me | C(=O)Me | O |
| C-4030 | H | CH(Me) | H | c-Pr(1-CN) | S(=O)₂Me | Me | C(=O)Me | O |
| C-4031 | H | CH₂CH₂ | Me | c-Pr(1-CN) | S(=O)₂Me | Me | C(=O)Me | O |
| C-4032 | Me | CH₂CH₂ | H | c-Pr(1-CN) | S(=O)₂Me | Me | C(=O)Me | O |
| C-4033 | H | single bond | Me | c-Pr(1-CN) | S(=O)₂CF₃ | Cl | Me | O |
| C-4034 | Me | single bond | H | c-Pr(1-CN) | S(=O)₂CF₃ | Cl | Me | O |
| C-4035 | Me(R) | single bond | H | c-Pr(1-CN) | S(=O)₂CF₃ | Cl | Me | O |
| C-4036 | Me(S) | single bond | H | c-Pr(1-CN) | S(=O)₂CF₃ | Cl | Me | O |
| C-4037 | H | CH₂ | Me | c-Pr(1-CN) | S(=O)₂CF₃ | Cl | Me | O |
| C-4038 | Me | CH₂ | H | c-Pr(1-CN) | S(=O)₂CF₃ | Cl | Me | O |
| C-4039 | H | CH(Me) | H | c-Pr(1-CN) | S(=O)₂CF₃ | Cl | Me | O |
| C-4040 | H | CH₂CH₂ | Me | c-Pr(1-CN) | S(=O)₂CF₃ | Cl | Me | O |
| C-4041 | Me | CH₂CH₂ | H | c-Pr(1-CN) | S(=O)₂CF₃ | Cl | Me | O |
| C-4042 | H | single bond | Me | c-Pr(1-CN) | S(=O)₂CF₃ | Me | Me | O |

**[Table 319]**

| Compound Number | Y | W | Z | R¹ | R² | R³ | R¹⁰ | A |
|---|---|---|---|---|---|---|---|---|
| C-4043 | Me | single bond | H | c-Pr(1-CN) | S(=O)₂CF₃ | Me | Me | O |
| C-4044 | Me(R) | single bond | H | c-Pr(1-CN) | S(=O)₂CF₃ | Me | Me | O |
| C-4045 | Me(S) | single bond | H | c-Pr(1-CN) | S(=O)₂CF₃ | Me | Me | O |
| C-4046 | H | CH₂ | Me | c-Pr(1-CN) | S(=O)₂CF₃ | Me | Me | O |
| C-4047 | Me | CH₂ | H | c-Pr(1-CN) | S(=O)₂CF₃ | Me | Me | O |
| C-4048 | H | CH(Me) | H | c-Pr(1-CN) | S(=O)₂CF₃ | Me | Me | O |
| C-4049 | H | CH₂CH₂ | Me | c-Pr(1-CN) | S(=O)₂CF₃ | Me | Me | O |
| C-4050 | Me | CH₂CH₂ | H | c-Pr(1-CN) | S(=O)₂CF₃ | Me | Me | O |
| C-4051 | H | single bond | Me | c-Pr(1-CN) | S(=O)₂CF₃ | Cl | Et | O |
| C-4052 | Me | single bond | H | c-Pr(1-CN) | S(=O)₂CF₃ | Cl | Et | O |
| C-4053 | Me(R) | single bond | H | c-Pr(1-CN) | S(=O)₂CF₃ | Cl | Et | O |
| C-4054 | Me(S) | single bond | H | c-Pr(1-CN) | S(=O)₂CF₃ | Cl | Et | O |
| C-4055 | H | CH₂ | Me | c-Pr(1-CN) | S(=O)₂CF₃ | Cl | Et | O |
| C-4056 | Me | CH₂ | H | c-Pr(1-CN) | S(=O)₂CF₃ | Cl | Et | O |
| C-4057 | H | CH(Me) | H | c-Pr(1-CN) | S(=O)₂CF₃ | Cl | Et | O |
| C-4058 | H | CH₂CH₂ | Me | c-Pr(1-CN) | S(=O)₂CF₃ | Cl | Et | O |
| C-4059 | Me | CH₂CH₂ | H | c-Pr(1-CN) | S(=O)₂CF₃ | Cl | Et | O |
| C-4060 | H | single bond | Me | c-Pr(1-CN) | S(=O)₂CF₃ | Me | Et | O |
| C-4061 | Me | single bond | H | c-Pr(1-CN) | S(=O)₂CF₃ | Me | Et | O |
| C-4062 | Me(R) | single bond | H | c-Pr(1-CN) | S(=O)₂CF₃ | Me | Et | O |
| C-4063 | Me(S) | single bond | H | c-Pr(1-CN) | S(=O)₂CF₃ | Me | Et | O |
| C-4064 | H | CH₂ | Me | c-Pr(1-CN) | S(=O)₂CF₃ | Me | Et | O |
| C-4065 | Me | CH₂ | H | c-Pr(1-CN) | S(=O)₂CF₃ | Me | Et | O |
| C-4066 | H | CH(Me) | H | c-Pr(1-CN) | S(=O)₂CF₃ | Me | Et | O |
| C-4067 | H | CH₂CH₂ | Me | c-Pr(1-CN) | S(=O)₂CF₃ | Me | Et | O |
| C-4068 | Me | CH₂CH₂ | H | c-Pr(1-CN) | S(=O)₂CF₃ | Me | Et | O |
| C-4069 | H | single bond | Me | c-Pr(1-CN) | S(=O)₂CF₃ | Cl | CH₂CF₃ | O |
| C-4070 | Me | single bond | H | c-Pr(1-CN) | S(=O)₂CF₃ | Cl | CH₂CF₃ | O |
| C-4071 | Me(R) | single bond | H | c-Pr(1-CN) | S(=O)₂CF₃ | Cl | CH₂CF₃ | O |
| C-4072 | Me(S) | single bond | H | c-Pr(1-CN) | S(=O)₂CF₃ | Cl | CH₂CF₃ | O |
| C-4073 | H | CH₂ | Me | c-Pr(1-CN) | S(=O)₂CF₃ | Cl | CH₂CF₃ | O |
| C-4074 | Me | CH₂ | H | c-Pr(1-CN) | S(=O)₂CF₃ | Cl | CH₂CF₃ | O |
| C-4075 | H | CH(Me) | H | c-Pr(1-CN) | S(=O)₂CF₃ | Cl | CH₂CF₃ | O |
| C-4076 | H | CH₂CH₂ | Me | c-Pr(1-CN) | S(=O)₂CF₃ | Cl | CH₂CF₃ | O |
| C-4077 | Me | CH₂CH₂ | H | c-Pr(1-CN) | S(=O)₂CF₃ | Cl | CH₂CF₃ | O |
| C-4078 | H | single bond | Me | c-Pr(1-CN) | S(=O)₂CF₃ | Me | CH₂CF₃ | O |
| C-4079 | Me | single bond | H | c-Pr(1-CN) | S(=O)₂CF₃ | Me | CH₂CF₃ | O |
| C-4080 | Me(R) | single bond | H | c-Pr(1-CN) | S(=O)₂CF₃ | Me | CH₂CF₃ | O |
| C-4081 | Me(S) | single bond | H | c-Pr(1-CN) | S(=O)₂CF₃ | Me | CH₂CF₃ | O |
| C-4082 | H | CH₂ | Me | c-Pr(1-CN) | S(=O)₂CF₃ | Me | CH₂CF₃ | O |
| C-4083 | Me | CH₂ | H | c-Pr(1-CN) | S(=O)₂CF₃ | Me | CH₂CF₃ | O |
| C-4084 | H | CH(Me) | H | c-Pr(1-CN) | S(=O)₂CF₃ | Me | CH₂CF₃ | O |
| C-4085 | H | CH₂CH₂ | Me | c-Pr(1-CN) | S(=O)₂CF₃ | Me | CH₂CF₃ | O |
| C-4086 | Me | CH₂CH₂ | H | c-Pr(1-CN) | S(=O)₂CF₃ | Me | CH₂CF₃ | O |

**[Table 320]**

| Compound Number | Y | W | Z | R¹ | R² | R³ | R¹⁰ | A |
|---|---|---|---|---|---|---|---|---|
| C-4087 | H | single bond | Me | c-Pr(1-CN) | S(=O)₂CF₃ | Cl | CH₂OMe | O |
| C-4088 | Me | single bond | H | c-Pr(1-CN) | S(=O)₂CF₃ | Cl | CH₂OMe | O |
| C-4089 | Me(R) | single bond | H | c-Pr(1-CN) | S(=O)₂CF₃ | Cl | CH₂OMe | O |
| C-4090 | Me(S) | single bond | H | c-Pr(1-CN) | S(=O)₂CF₃ | Cl | CH₂OMe | O |
| C-4091 | H | CH₂ | Me | c-Pr(1-CN) | S(=O)₂CF₃ | Cl | CH₂OMe | O |
| C-4092 | Me | CH₂ | H | c-Pr(1-CN) | S(=O)₂CF₃ | Cl | CH₂OMe | O |
| C-4093 | H | CH(Me) | H | c-Pr(1-CN) | S(=O)₂CF₃ | Cl | CH₂OMe | O |
| C-4094 | H | CH₂CH₂ | Me | c-Pr(1-CN) | S(=O)₂CF₃ | Cl | CH₂OMe | O |
| C-4095 | Me | CH₂CH₂ | H | c-Pr(1-CN) | S(=O)₂CF₃ | Cl | CH₂OMe | O |
| C-4096 | H | single bond | Me | c-Pr(1-CN) | S(=O)₂CF₃ | Me | CH₂OMe | O |
| C-4097 | Me | single bond | H | c-Pr(1-CN) | S(=O)₂CF₃ | Me | CH₂OMe | O |
| C-4098 | Me(R) | single bond | H | c-Pr(1-CN) | S(=O)₂CF₃ | Me | CH₂OMe | O |
| C-4099 | Me(S) | single bond | H | c-Pr(1-CN) | S(=O)₂CF₃ | Me | CH₂OMe | O |
| C-4100 | H | CH₂ | Me | c-Pr(1-CN) | S(=O)₂CF₃ | Me | CH₂OMe | O |
| C-4101 | Me | CH₂ | H | c-Pr(1-CN) | S(=O)₂CF₃ | Me | CH₂OMe | O |
| C-4102 | H | CH(Me) | H | c-Pr(1-CN) | S(=O)₂CF₃ | Me | CH₂OMe | O |
| C-4103 | H | CH₂CH₂ | Me | c-Pr(1-CN) | S(=O)₂CF₃ | Me | CH₂OMe | O |
| C-4104 | Me | CH₂CH₂ | H | c-Pr(1-CN) | S(=O)₂CF₃ | Me | CH₂OMe | O |
| C-4105 | H | single bond | Me | c-Pr(1-CN) | S(=O)₂CF₃ | Cl | CH₂CN | O |
| C-4106 | Me | single bond | H | c-Pr(1-CN) | S(=O)₂CF₃ | Cl | CH₂CN | O |
| C-4107 | Me(R) | single bond | H | c-Pr(1-CN) | S(=O)₂CF₃ | Cl | CH₂CN | O |
| C-4108 | Me(S) | single bond | H | c-Pr(1-CN) | S(=O)₂CF₃ | Cl | CH₂CN | O |
| C-4109 | H | CH₂ | Me | c-Pr(1-CN) | S(=O)₂CF₃ | Cl | CH₂CN | O |
| C-4110 | Me | CH₂ | H | c-Pr(1-CN) | S(=O)₂CF₃ | Cl | CH₂CN | O |
| C-4111 | H | CH(Me) | H | c-Pr(1-CN) | S(=O)₂CF₃ | Cl | CH₂CN | O |
| C-4112 | H | CH₂CH₂ | Me | c-Pr(1-CN) | S(=O)₂CF₃ | Cl | CH₂CN | O |
| C-4113 | Me | CH₂CH₂ | H | c-Pr(1-CN) | S(=O)₂CF₃ | Cl | CH₂CN | O |
| C-4114 | H | single bond | Me | c-Pr(1-CN) | S(=O)₂CF₃ | Me | CH₂CN | O |
| C-4115 | Me | single bond | H | c-Pr(1-CN) | S(=O)₂CF₃ | Me | CH₂CN | O |
| C-4116 | Me(R) | single bond | H | c-Pr(1-CN) | S(=O)₂CF₃ | Me | CH₂CN | O |
| C-4117 | Me(S) | single bond | H | c-Pr(1-CN) | S(=O)₂CF₃ | Me | CH₂CN | O |
| C-4118 | H | CH₂ | Me | c-Pr(1-CN) | S(=O)₂CF₃ | Me | CH₂CN | O |
| C-4119 | Me | CH₂ | H | c-Pr(1-CN) | S(=O)₂CF₃ | Me | CH₂CN | O |
| C-4120 | H | CH(Me) | H | c-Pr(1-CN) | S(=O)₂CF₃ | Me | CH₂CN | O |
| C-4121 | H | CH₂CH₂ | Me | c-Pr(1-CN) | S(=O)₂CF₃ | Me | CH₂CN | O |
| C-4122 | Me | CH₂CH₂ | H | c-Pr(1-CN) | S(=O)₂CF₃ | Me | CH₂CN | O |
| C-4123 | H | single bond | Me | c-Pr(1-CN) | S(=O)₂CF₃ | Cl | C(=O)Me | O |
| C-4124 | Me | single bond | H | c-Pr(1-CN) | S(=O)₂CF₃ | Cl | C(=O)Me | O |
| C-4125 | Me(R) | single bond | H | c-Pr(1-CN) | S(=O)₂CF₃ | Cl | C(=O)Me | O |
| C-4126 | Me(S) | single bond | H | c-Pr(1-CN) | S(=O)₂CF₃ | Cl | C(=O)Me | O |
| C-4127 | H | CH₂ | Me | c-Pr(1-CN) | S(=O)₂CF₃ | Cl | C(=O)Me | O |
| C-4128 | Me | CH₂ | H | c-Pr(1-CN) | S(=O)₂CF₃ | Cl | C(=O)Me | O |
| C-4129 | H | CH(Me) | H | c-Pr(1-CN) | S(=O)₂CF₃ | Cl | C(=O)Me | O |
| C-4130 | H | CH₂CH₂ | Me | c-Pr(1-CN) | S(=O)₂CF₃ | Cl | C(=O)Me | O |

**[Table 321]**

| Compound Number | Y | W | z | R¹ | R² | R³ | R¹⁰ | A |
|---|---|---|---|---|---|---|---|---|
| C-4131 | Me | CH₂CH₂ | H | c-Pr(1-CN) | S(=O)₂CF₃ | Cl | C(=O)Me | O |
| C-4132 | H | single bond | Me | c-Pr(1-CN) | S(=O)₂CF₃ | Me | C(=O)Me | O |
| C-4133 | Me | single bond | H | c-Pr(1-CN) | S(=O)₂CF₃ | Me | C(=O)Me | O |
| C-4134 | Me(R) | single bond | H | c-Pr(1-CN) | S(=O)₂CF₃ | Me | C(=O)Me | O |
| C-4135 | Me(S) | single bond | H | c-Pr(1-CN) | S(=O)₂CF₃ | Me | C(=O)Me | O |
| C-4136 | H | CH₂ | Me | c-Pr(1-CN) | S(=O)₂CF₃ | Me | C(=O)Me | O |
| C-4137 | Me | CH₂ | H | c-Pr(1-CN) | S(=O)₂CF₃ | Me | C(=O)Me | O |
| C-4138 | H | CH(Me) | H | c-Pr(1-CN) | S(=O)₂CF₃ | Me | C(=O)Me | O |
| C-4139 | H | CH₂CH₂ | Me | c-Pr(1-CN) | S(=O)₂CF₃ | Me | C(=O)Me | O |
| C-4140 | Me | CH₂CH₂ | H | c-Pr(1-CN) | S(=O)₂CF₃ | Me | C(=O)Me | O |
| C-4141 | H | single bond | Me | CH₂CF₃ | Me | Cl | H | O |
| C-4142 | Me | single bond | H | CH₂CF₃ | Me | Cl | H | O |
| C-4143 | Me(R) | single bond | H | CH₂CF₃ | Me | Cl | H | O |
| C-4144 | Me(S) | single bond | H | CH₂CF₃ | Me | Cl | H | O |
| C-4145 | H | CH₂ | Me | CH₂CF₃ | Me | Cl | H | O |
| C-4146 | Me | CH₂ | H | CH₂CF₃ | Me | Cl | H | O |
| C-4147 | H | CH(Me) | H | CH₂CF₃ | Me | Cl | H | O |
| C-4148 | H | CH₂CH₂ | Me | CH₂CF₃ | Me | Cl | H | O |
| C-4149 | Me | CH₂CH₂ | H | CH₂CF₃ | Me | Cl | H | O |
| C-4150 | H | single bond | Me | Et | Me | Cl | H | O |
| C-4151 | Me | single bond | H | Et | Me | Cl | H | O |
| C-4152 | Me(R) | single bond | H | Et | Me | Cl | H | O |
| C-4153 | Me(S) | single bond | H | Et | Me | Cl | H | O |
| C-4154 | H | CH₂ | Me | Et | Me | Cl | H | O |
| C-4155 | Me | CH₂ | H | Et | Me | Cl | H | O |
| C-4156 | H | CH(Me) | H | Et | Me | Cl | H | O |
| C-4157 | H | CH₂CH₂ | Me | Et | Me | Cl | H | O |
| C-4158 | Me | CH₂CH₂ | H | Et | Me | Cl | H | O |
| C-4159 | H | single bond | Me | c-Pr | Me | Cl | H | O |
| C-4160 | Me | single bond | H | c-Pr | Me | Cl | H | O |
| C-4161 | Me(R) | single bond | H | c-Pr | Me | Cl | H | O |
| C-4162 | Me(S) | single bond | H | c-Pr | Me | Cl | H | O |
| C-4163 | H | CH₂ | Me | c-Pr | Me | Cl | H | O |
| C-4164 | Me | CH₂ | H | c-Pr | Me | Cl | H | O |
| C-4165 | H | CH(Me) | H | c-Pr | Me | Cl | H | O |
| C-4166 | H | CH₂CH₂ | Me | c-Pr | Me | Cl | H | O |
| C-4167 | Me | CH₂CH₂ | H | c-Pr | Me | Cl | H | O |
| C-4168 | H | single bond | Me | c-Pr(1-CN) | Me | Cl | H | O |
| C-4169 | Me | single bond | H | c-Pr(1-CN) | Me | Cl | H | O |
| C-4170 | Me(R) | single bond | H | c-Pr(1-CN) | Me | Cl | H | O |
| C-4171 | Me(S) | single bond | H | c-Pr(1-CN) | Me | Cl | H | O |
| C-4172 | H | CH₂ | Me | c-Pr(1-CN) | Me | Cl | H | O |
| C-4173 | Me | CH₂ | H | c-Pr(1-CN) | Me | Cl | H | O |
| C-4174 | H | CH(Me) | H | c-Pr(1-CN) | Me | Cl | H | O |

**[Table 322]**

| Compound Number | Y | W | Z | R¹ | R² | R³ | R¹⁰ | A |
|---|---|---|---|---|---|---|---|---|
| C-4175 | H | CH₂CH₂ | Me | c-Pr(1-CN) | Me | Cl | H | O |
| C-4176 | Me | CH₂CH₂ | H | c-Pr(1-CN) | Me | Cl | H | O |
| C-4177 | H | single bond | Me | CH₂CF₃ | Me | Me | H | O |
| C-4178 | Me | single bond | H | CH₂CF₃ | Me | Me | H | O |
| C-4179 | Me(R) | single bond | H | CH₂CF₃ | Me | Me | H | O |
| C-4180 | Me(S) | single bond | H | CH₂CF₃ | Me | Me | H | O |
| C-4181 | H | CH₂ | Me | CH₂CF₃ | Me | Me | H | O |
| C-4182 | Me | CH₂ | H | CH₂CF₃ | Me | Me | H | O |
| C-4183 | H | CH(Me) | H | CH₂CF₃ | Me | Me | H | O |
| C-4184 | H | CH₂CH₂ | Me | CH₂CF₃ | Me | Me | H | O |
| C-4185 | Me | CH₂CH₂ | H | CH₂CF₃ | Me | Me | H | O |
| C-4186 | H | single bond | Me | Et | Me | Me | H | O |
| C-4187 | Me | single bond | H | Et | Me | Me | H | O |
| C-4188 | Me(R) | single bond | H | Et | Me | Me | H | O |
| C-4189 | Me(S) | single bond | H | Et | Me | Me | H | O |
| C-4190 | H | CH₂ | Me | Et | Me | Me | H | O |
| C-4191 | Me | CH₂ | H | Et | Me | Me | H | O |
| C-4192 | H | CH(Me) | H | Et | Me | Me | H | O |
| C-4193 | H | CH₂CH₂ | Me | Et | Me | Me | H | O |
| C-4194 | Me | CH₂CH₂ | H | Et | Me | Me | H | O |
| C-4195 | H | single bond | Me | c-Pr | Me | Me | H | O |
| C-4196 | Me | single bond | H | c-Pr | Me | Me | H | O |
| C-4197 | Me(R) | single bond | H | c-Pr | Me | Me | H | O |
| C-4198 | Me(S) | single bond | H | c-Pr | Me | Me | H | O |
| C-4199 | H | CH₂ | Me | c-Pr | Me | Me | H | O |
| C-4200 | Me | CH₂ | H | c-Pr | Me | Me | H | O |
| C-4201 | H | CH(Me) | H | c-Pr | Me | Me | H | O |
| C-4202 | H | CH₂CH₂ | Me | c-Pr | Me | Me | H | O |
| C-4203 | Me | CH₂CH₂ | H | c-Pr | Me | Me | H | O |
| C-4204 | H | single bond | Me | c-Pr(1-CN) | Me | Me | H | O |
| C-4205 | Me | single bond | H | c-Pr(1-CN) | Me | Me | H | O |
| C-4206 | Me(R) | single bond | H | c-Pr(1-CN) | Me | Me | H | O |
| C-4207 | Me(S) | single bond | H | c-Pr(1-CN) | Me | Me | H | O |
| C-4208 | H | CH₂ | Me | c-Pr(1-CN) | Me | Me | H | O |
| C-4209 | Me | CH₂ | H | c-Pr(1-CN) | Me | Me | H | O |
| C-4210 | H | CH(Me) | H | c-Pr(1-CN) | Me | Me | H | O |
| C-4211 | H | CH₂CH₂ | Me | c-Pr(1-CN) | Me | Me | H | O |
| C-4212 | Me | CH₂CH₂ | H | c-Pr(1-CN) | Me | Me | H | O |
| C-4213 | H | single bond | Me | CH₂CF₃ | C(=O)Me | Cl | H | O |
| C-4214 | Me | single bond | H | CH₂CF₃ | C(=O)Me | Cl | H | O |
| C-4215 | Me(R) | single bond | H | CH₂CF₃ | C(=O)Me | Cl | H | O |
| C-4216 | Me(S) | single bond | H | CH₂CF₃ | C(=O)Me | Cl | H | O |
| C-4217 | H | CH₂ | Me | CH₂CF₃ | C(=O)Me | Cl | H | O |
| C-4218 | Me | CH₂ | H | CH₂CF₃ | C(=O)Me | Cl | H | O |

**[Table 323]**

| Compound Number | Y | W | Z | R¹ | R² | R³ | R¹⁰ | A |
|---|---|---|---|---|---|---|---|---|
| C-4219 | H | CH(Me) | H | CH₂CF₃ | C(=O)Me | Cl | H | O |
| C-4220 | H | CH₂CH₂ | Me | CH₂CF₃ | C(=O)Me | Cl | H | O |
| C-4221 | Me | CH₂CH₂ | H | CH₂CF₃ | C(=O)Me | Cl | H | O |
| C-4222 | H | single bond | Me | Et | C(=O)Me | Cl | H | O |
| C-4223 | Me | single bond | H | Et | C(=O)Me | Cl | H | O |
| C-4224 | Me(R) | single bond | H | Et | C(=O)Me | Cl | H | O |
| C-4225 | Me(S) | single bond | H | Et | C(=O)Me | Cl | H | O |
| C-4226 | H | CH₂ | Me | Et | C(=O)Me | Cl | H | O |
| C-4227 | Me | CH₂ | H | Et | C(=O)Me | Cl | H | O |
| C-4228 | H | CH(Me) | H | Et | C(=O)Me | Cl | H | O |
| C-4229 | H | CH₂CH₂ | Me | Et | C(=O)Me | Cl | H | O |
| C-4230 | Me | CH₂CH₂ | H | Et | C(=O)Me | Cl | H | O |
| C-4231 | H | single bond | Me | c-Pr | C(=O)Me | Cl | H | O |
| C-4232 | Me | single bond | H | c-Pr | C(=O)Me | Cl | H | O |
| C-4233 | Me(R) | single bond | H | c-Pr | C(=O)Me | Cl | H | O |
| C-4234 | Me(S) | single bond | H | c-Pr | C(=O)Me | Cl | H | O |
| C-4235 | H | CH₂ | Me | c-Pr | C(=O)Me | Cl | H | O |
| C-4236 | Me | CH₂ | H | c-Pr | C(=O)Me | Cl | H | O |
| C-4237 | H | CH(Me) | H | c-Pr | C(=O)Me | Cl | H | O |
| C-4238 | H | CH₂CH₂ | Me | c-Pr | C(=O)Me | Cl | H | O |
| C-4239 | Me | CH₂CH₂ | H | c-Pr | C(=O)Me | Cl | H | O |
| C-4240 | H | single bond | Me | c-Pr(1-CN) | C(=O)Me | Cl | H | O |
| C-4241 | Me | single bond | H | c-Pr(1-CN) | C(=O)Me | Cl | H | O |
| C-4242 | Me(R) | single bond | H | c-Pr(1-CN) | C(=O)Me | Cl | H | O |
| C-4243 | Me(S) | single bond | H | c-Pr(1-CN) | C(=O)Me | Cl | H | O |
| C-4244 | H | CH₂ | Me | c-Pr(1-CN) | C(=O)Me | Cl | H | O |
| C-4245 | Me | CH₂ | H | c-Pr(1-CN) | C(=O)Me | Cl | H | O |
| C-4246 | H | CH(Me) | H | c-Pr(1-CN) | C(=O)Me | Cl | H | O |
| C-4247 | H | CH₂CH₂ | Me | c-Pr(1-CN) | C(=O)Me | Cl | H | O |
| C-4248 | Me | CH₂CH₂ | H | c-Pr(1-CN) | C(=O)Me | Cl | H | O |
| C-4249 | H | single bond | Me | CH₂CF₃ | C(=O)Me | Me | H | O |
| C-4250 | Me | single bond | H | CH₂CF₃ | C(=O)Me | Me | H | O |
| C-4251 | Me(R) | single bond | H | CH₂CF₃ | C(=O)Me | Me | H | O |
| C-4252 | Me(S) | single bond | H | CH₂CF₃ | C(=O)Me | Me | H | O |
| C-4253 | H | CH₂ | Me | CH₂CF₃ | C(=O)Me | Me | H | O |
| C-4254 | Me | CH₂ | H | CH₂CF₃ | C(=O)Me | Me | H | O |
| C-4255 | H | CH(Me) | H | CH₂CF₃ | C(=O)Me | Me | H | O |
| C-4256 | H | CH₂CH₂ | Me | CH₂CF₃ | C(=O)Me | Me | H | O |
| C-4257 | Me | CH₂CH₂ | H | CH₂CF₃ | C(=O)Me | Me | H | O |
| C-4258 | H | single bond | Me | Et | C(=O)Me | Me | H | O |
| C-4259 | Me | single bond | H | Et | C(=O)Me | Me | H | O |
| C-4260 | Me(R) | single bond | H | Et | C(=O)Me | Me | H | O |
| C-4261 | Me(S) | single bond | H | Et | C(=O)Me | Me | H | O |
| C-4262 | H | CH₂ | Me | Et | C(=O)Me | Me | H | O |

**[Table 324]**

| Compound Number | Y | W | Z | R¹ | R² | R³ | R¹⁰ | A |
|---|---|---|---|---|---|---|---|---|
| C-4263 | Me | CH₂ | H | Et | C(=O)Me | Me | H | O |
| C-4264 | H | CH(Me) | H | Et | C(=O)Me | Me | H | O |
| C-4265 | H | CH₂CH₂ | Me | Et | C(=O)Me | Me | H | O |
| C-4266 | Me | CH₂CH₂ | H | Et | C(=O)Me | Me | H | O |
| C-4267 | H | single bond | Me | c-Pr | C(=O)Me | Me | H | O |
| C-4268 | Me | single bond | H | c-Pr | C(=O)Me | Me | H | O |
| C-4269 | Me(R) | single bond | H | c-Pr | C(=O)Me | Me | H | O |
| C-4270 | Me(S) | single bond | H | c-Pr | C(=O)Me | Me | H | O |
| C-4271 | H | CH₂ | Me | c-Pr | C(=O)Me | Me | H | O |
| C-4272 | Me | CH₂ | H | c-Pr | C(=O)Me | Me | H | O |
| C-4273 | H | CH(Me) | H | c-Pr | C(=O)Me | Me | H | O |
| C-4274 | H | CH₂CH₂ | Me | c-Pr | C(=O)Me | Me | H | O |
| C-4275 | Me | CH₂CH₂ | H | c-Pr | C(=O)Me | Me | H | O |
| C-4276 | H | single bond | Me | c-Pr(1-CN) | C(=O)Me | Me | H | O |
| C-4277 | Me | single bond | H | c-Pr(1-CN) | C(=O)Me | Me | H | O |
| C-4278 | Me(R) | single bond | H | c-Pr(1-CN) | C(=O)Me | Me | H | O |
| C-4279 | Me(S) | single bond | H | c-Pr(1-CN) | C(=O)Me | Me | H | O |
| C-4280 | H | CH₂ | Me | c-Pr(1-CN) | C(=O)Me | Me | H | O |
| C-4281 | Me | CH₂ | H | c-Pr(1-CN) | C(=O)Me | Me | H | O |
| C-4282 | H | CH(Me) | H | c-Pr(1-CN) | C(=O)Me | Me | H | O |
| C-4283 | H | CH₂CH₂ | Me | c-Pr(1-CN) | C(=O)Me | Me | H | O |
| C-4284 | Me | CH₂CH₂ | H | c-Pr(1-CN) | C(=O)Me | Me | H | O |
| C-4285 | H | single bond | Me | CH₂CF₃ | C(=O)(*i*-Pr) | Cl | H | O |
| C-4286 | Me | single bond | H | CH₂CF₃ | C(=O)(*i*-Pr) | Cl | H | O |
| C-4287 | Me(R) | single bond | H | CH₂CF₃ | C(=O)(*i*-Pr) | Cl | H | O |
| C-4288 | Me(S) | single bond | H | CH₂CF₃ | C(=O)(*i*-Pr) | Cl | H | O |
| C-4289 | H | CH₂ | Me | CH₂CF₃ | C(=O)(*i*-Pr) | Cl | H | O |
| C-4290 | Me | CH₂ | H | CH₂CF₃ | C(=O)(*i*-Pr) | Cl | H | O |
| C-4291 | H | CH(Me) | H | CH₂CF₃ | C(=O)(*i*-Pr) | Cl | H | O |
| C-4292 | H | CH₂CH₂ | Me | CH₂CF₃ | C(=O)(*i*-Pr) | Cl | H | O |
| C-4293 | Me | CH₂CH₂ | H | CH₂CF₃ | C(=O)(*i*-Pr) | Cl | H | O |
| C-4294 | H | single bond | Me | Et | C(=O)(*i*-Pr) | Cl | H | O |
| C-4295 | Me | single bond | H | Et | C(=O)(*i*-Pr) | Cl | H | O |
| C-4296 | Me(R) | single bond | H | Et | C(=O)(*i*-Pr) | Cl | H | O |
| C-4297 | Me(S) | single bond | H | Et | C(=O)(*i*-Pr) | Cl | H | O |
| C-4298 | H | CH₂ | Me | Et | C(=O)(*i*-Pr) | Cl | H | O |
| C-4299 | Me | CH₂ | H | Et | C(=O)(*i*-Pr) | Cl | H | O |
| C-4300 | H | CH(Me) | H | Et | C(=O)(*i*-Pr) | Cl | H | O |
| C-4301 | H | CH₂CH₂ | Me | Et | C(=O)(*i*-Pr) | Cl | H | O |
| C-4302 | Me | CH₂CH₂ | H | Et | C(=O)(*i*-Pr) | Cl | H | O |
| C-4303 | H | single bond | Me | c-Pr | C(=O)(*i*-Pr) | Cl | H | O |
| C-4304 | Me | single bond | H | c-Pr | C(=O)(*i*-Pr) | Cl | H | O |
| C-4305 | Me(R) | single bond | H | c-Pr | C(=O)(*i*-Pr) | Cl | H | O |
| C-4306 | Me(S) | single bond | H | c-Pr | C(=O)(*i*-Pr) | Cl | H | O |

**[Table 325]**

| Compound Number | Y | W | Z | R¹ | R² | R³ | R¹⁰ | A |
|---|---|---|---|---|---|---|---|---|
| C-4307 | H | CH₂ | Me | c-Pr | C(=O)(*i* -Pr) | Cl | H | O |
| C-4308 | Me | CH₂ | H | c-Pr | C(=O)(*i* -Pr) | Cl | H | O |
| C-4309 | H | CH(Me) | H | c-Pr | C(=O)(*i* -Pr) | Cl | H | O |
| C-4310 | H | CH₂CH₂ | Me | c-Pr | C(=O)(*i* -Pr) | Cl | H | O |
| C-4311 | Me | CH₂CH₂ | H | c-Pr | C(=O)(*i* -Pr) | Cl | H | O |
| C-4312 | H | single bond | Me | c-Pr(1-CN) | C(=O)(*i* -Pr) | Cl | H | O |
| C-4313 | Me | single bond | H | c-Pr(1-CN) | C(=O)(*i* -Pr) | Cl | H | O |
| C-4314 | Me(R) | single bond | H | c-Pr(1-CN) | C(=O)(*i* -Pr) | Cl | H | O |
| C-4315 | Me(S) | single bond | H | c-Pr(1-CN) | C(=O)(*i* -Pr) | Cl | H | O |
| C-4316 | H | CH₂ | Me | c-Pr(1-CN) | C(=O)(*i* -Pr) | Cl | H | O |
| C-4317 | Me | CH₂ | H | c-Pr(1-CN) | C(=O)(*i* -Pr) | Cl | H | O |
| C-4318 | H | CH(Me) | H | c-Pr(1-CN) | C(=O)(*i* -Pr) | Cl | H | O |
| C-4319 | H | CH₂CH₂ | Me | c-Pr(1-CN) | C(=O)(*i* -Pr) | Cl | H | O |
| C-4320 | Me | CH₂CH₂ | H | c-Pr(1-CN) | C(=O)(*i* -Pr) | Cl | H | O |
| C-4321 | H | single bond | Me | CH₂CF₃ | C(=O)(*i* -Pr) | Me | H | O |
| C-4322 | Me | single bond | H | CH₂CF₃ | C(=O)(*i* -Pr) | Me | H | O |
| C-4323 | Me(R) | single bond | H | CH₂CF₃ | C(=O)(*i* -Pr) | Me | H | O |
| C-4324 | Me(S) | single bond | H | CH₂CF₃ | C(=O)(*i* -Pr) | Me | H | O |
| C-4325 | H | CH₂ | Me | CH₂CF₃ | C(=O)(*i* -Pr) | Me | H | O |
| C-4326 | Me | CH₂ | H | CH₂CF₃ | C(=O)(*i* -Pr) | Me | H | O |
| C-4327 | H | CH(Me) | H | CH₂CF₃ | C(=O)(*i* -Pr) | Me | H | O |
| C-4328 | H | CH₂CH₂ | Me | CH₂CF₃ | C(=O)(*i* -Pr) | Me | H | O |
| C-4329 | Me | CH₂CH₂ | H | CH₂CF₃ | C(=O)(*i* -Pr) | Me | H | O |
| C-4330 | H | single bond | Me | Et | C(=O)(*i* -Pr) | Me | H | O |
| C-4331 | Me | single bond | H | Et | C(=O)(*i* -Pr) | Me | H | O |
| C-4332 | Me(R) | single bond | H | Et | C(=O)(*i* -Pr) | Me | H | O |
| C-4333 | Me(S) | single bond | H | Et | C(=O)(*i* -Pr) | Me | H | O |
| C-4334 | H | CH₂ | Me | Et | C(=O)(*i* -Pr) | Me | H | O |
| C-4335 | Me | CH₂ | H | Et | C(=O)(*i* -Pr) | Me | H | O |
| C-4336 | H | CH(Me) | H | Et | C(=O)(*i* -Pr) | Me | H | O |
| C-4337 | H | CH₂CH₂ | Me | Et | C(=O)(*i* -Pr) | Me | H | O |
| C-4338 | Me | CH₂CH₂ | H | Et | C(=O)(*i* -Pr) | Me | H | O |
| C-4339 | H | single bond | Me | c-Pr | C(=O)(*i* -Pr) | Me | H | O |
| C-4340 | Me | single bond | H | c-Pr | C(=O)(*i* -Pr) | Me | H | O |
| C-4341 | Me(R) | single bond | H | c-Pr | C(=O)(*i* -Pr) | Me | H | O |
| C-4342 | Me(S) | single bond | H | c-Pr | C(=O)(*i* -Pr) | Me | H | O |
| C-4343 | H | CH₂ | Me | c-Pr | C(=O)(*i* -Pr) | Me | H | O |
| C-4344 | Me | CH₂ | H | c-Pr | C(=O)(*i* -Pr) | Me | H | O |
| C-4345 | H | CH(Me) | H | c-Pr | C(=O)(*i* -Pr) | Me | H | O |
| C-4346 | H | CH₂CH₂ | Me | c-Pr | C(=O)(*i* -Pr) | Me | H | O |
| C-4347 | Me | CH₂CH₂ | H | c-Pr | C(=O)(*i* -Pr) | Me | H | O |
| C-4348 | H | single bond | Me | c-Pr(1-CN) | C(=O)(*i* -Pr) | Me | H | O |
| C-4349 | Me | single bond | H | c-Pr(1-CN) | C(=O)(*i* -Pr) | Me | H | O |
| C-4350 | Me(R) | single bond | H | c-Pr(1-CN) | C(=O)(*i* -Pr) | Me | H | O |

**[Table 326]**

| Compound Number | Y | W | Z | R¹ | R² | R³ | R¹⁰ | A |
|---|---|---|---|---|---|---|---|---|
| C-4351 | Me(S) | single bond | H | c-Pr(1-CN) | C(=O)(*i* -Pr) | Me | H | O |
| C-4352 | H | CH₂ | Me | c-Pr(1-CN) | C(=O)(*i* -Pr) | Me | H | O |
| C-4353 | Me | CH₂ | H | c-Pr(1-CN) | C(=O)(*i* -Pr) | Me | H | O |
| C-4354 | H | CH(Me) | H | c-Pr(1-CN) | C(=O)(*i* -Pr) | Me | H | O |
| C-4355 | H | CH₂CH₂ | Me | c-Pr(1-CN) | C(=O)(*i* -Pr) | Me | H | O |
| C-4356 | Me | CH₂CH₂ | H | c-Pr(1-CN) | C(=O)(*i* -Pr) | Me | H | O |
| C-4357 | H | single bond | Me | CH₂CF₃ | H | Br | H | O |
| C-4358 | Me | single bond | H | CH₂CF₃ | H | Br | H | O |
| C-4359 | Me(R) | single bond | H | CH₂CF₃ | H | Br | H | O |
| C-4360 | Me(S) | single bond | H | CH₂CF₃ | H | Br | H | O |
| C-4361 | H | CH₂ | Me | CH₂CF₃ | H | Br | H | O |
| C-4362 | Me | CH₂ | H | CH₂CF₃ | H | Br | H | O |
| C-4363 | H | CH(Me) | H | CH₂CF₃ | H | Br | H | O |
| C-4364 | H | CH₂CH₂ | Me | CH₂CF₃ | H | Br | H | O |
| C-4365 | Me | CH₂CH₂ | H | CH₂CF₃ | H | Br | H | O |
| C-4366 | H | single bond | Me | Et | H | Br | H | O |
| C-4367 | Me | single bond | H | Et | H | Br | H | O |
| C-4368 | Me(R) | single bond | H | Et | H | Br | H | O |
| C-4369 | Me(S) | single bond | H | Et | H | Br | H | O |
| C-4370 | H | CH₂ | Me | Et | H | Br | H | O |
| C-4371 | Me | CH₂ | H | Et | H | Br | H | O |
| C-4372 | H | CH(Me) | H | Et | H | Br | H | O |
| C-4373 | H | CH₂CH₂ | Me | Et | H | Br | H | O |
| C-4374 | Me | CH₂CH₂ | H | Et | H | Br | H | O |
| C-4375 | H | single bond | Me | c-Pr | H | Br | H | O |
| C-4376 | Me | single bond | H | c-Pr | H | Br | H | O |
| C-4377 | Me(R) | single bond | H | c-Pr | H | Br | H | O |
| C-4378 | Me(S) | single bond | H | c-Pr | H | Br | H | O |
| C-4379 | H | CH₂ | Me | c-Pr | H | Br | H | O |
| C-4380 | Me | CH₂ | H | c-Pr | H | Br | H | O |
| C-4381 | H | CH(Me) | H | c-Pr | H | Br | H | O |
| C-4382 | H | CH₂CH₂ | Me | c-Pr | H | Br | H | O |
| C-4383 | Me | CH₂CH₂ | H | c-Pr | H | Br | H | O |
| C-4384 | H | single bond | Me | c-Pr(1-CN) | H | Br | H | O |
| C-4385 | Me | single bond | H | c-Pr(1-CN) | H | Br | H | O |
| C-4386 | Me(R) | single bond | H | c-Pr(1-CN) | H | Br | H | O |
| C-4387 | Me(S) | single bond | H | c-Pr(1-CN) | H | Br | H | O |
| C-4388 | H | CH₂ | Me | c-Pr(1-CN) | H | Br | H | O |
| C-4389 | Me | CH₂ | H | c-Pr(1-CN) | H | Br | H | O |
| C-4390 | H | CH(Me) | H | c-Pr(1-CN) | H | Br | H | O |
| C-4391 | H | CH₂CH₂ | Me | c-Pr(1-CN) | H | Br | H | O |
| C-4392 | Me | CH₂CH₂ | H | c-Pr(1-CN) | H | Br | H | O |
| C-4393 | H | single bond | Me | CH₂CF₃ | H | CF₃ | H | O |
| C-4394 | Me | single bond | H | CH₂CF₃ | H | CF₃ | H | O |

**[Table 327]**

| Compound Number | Y | W | Z | R¹ | R² | R³ | R¹⁰ | A |
|---|---|---|---|---|---|---|---|---|
| C-4395 | Me(R) | single bond | H | CH₂CF₃ | H | CF₃ | H | O |
| C-4396 | Me(S) | single bond | H | CH₂CF₃ | H | CF₃ | H | O |
| C-4397 | H | CH₂ | Me | CH₂CF₃ | H | CF₃ | H | O |
| C-4398 | Me | CH₂ | H | CH₂CF₃ | H | CF₃ | H | O |
| C-4399 | H | CH(Me) | H | CH₂CF₃ | H | CF₃ | H | O |
| C-4400 | H | CH₂CH₂ | Me | CH₂CF₃ | H | CF₃ | H | O |
| C-4401 | Me | CH₂CH₂ | H | CH₂CF₃ | H | CF₃ | H | O |
| C-4402 | H | single bond | Me | Et | H | CF₃ | H | O |
| C-4403 | Me | single bond | H | Et | H | CF₃ | H | O |
| C-4404 | Me(R) | single bond | H | Et | H | CF₃ | H | O |
| C-4405 | Me(S) | single bond | H | Et | H | CF₃ | H | O |
| C-4406 | H | CH₂ | Me | Et | H | CF₃ | H | O |
| C-4407 | Me | CH₂ | H | Et | H | CF₃ | H | O |
| C-4408 | H | CH(Me) | H | Et | H | CF₃ | H | O |
| C-4409 | H | CH₂CH₂ | Me | Et | H | CF₃ | H | O |
| C-4410 | Me | CH₂CH₂ | H | Et | H | CF₃ | H | O |
| C-4411 | H | single bond | Me | c-Pr | H | CF₃ | H | O |
| C-4412 | Me | single bond | H | c-Pr | H | CF₃ | H | O |
| C-4413 | Me(R) | single bond | H | c-Pr | H | CF₃ | H | O |
| C-4414 | Me(S) | single bond | H | c-Pr | H | CF₃ | H | O |
| C-4415 | H | CH₂ | Me | c-Pr | H | CF₃ | H | O |
| C-4416 | Me | CH₂ | H | c-Pr | H | CF₃ | H | O |
| C-4417 | H | CH(Me) | H | c-Pr | H | CF₃ | H | O |
| C-4418 | H | CH₂CH₂ | Me | c-Pr | H | CF₃ | H | O |
| C-4419 | Me | CH₂CH₂ | H | c-Pr | H | CF₃ | H | O |
| C-4420 | H | single bond | Me | c-Pr(1-CN) | H | CF₃ | H | O |
| C-4421 | Me | single bond | H | c-Pr(1-CN) | H | CF₃ | H | O |
| C-4422 | Me(R) | single bond | H | c-Pr(1-CN) | H | CF₃ | H | O |
| C-4423 | Me(S) | single bond | H | c-Pr(1-CN) | H | CF₃ | H | O |
| C-4424 | H | CH₂ | Me | c-Pr(1-CN) | H | CF₃ | H | O |
| C-4425 | Me | CH₂ | H | c-Pr(1-CN) | H | CF₃ | H | O |
| C-4426 | H | CH(Me) | H | c-Pr(1-CN) | H | CF₃ | H | O |
| C-4427 | H | CH₂CH₂ | Me | c-Pr(1-CN) | H | CF₃ | H | O |
| C-4428 | Me | CH₂CH₂ | H | c-Pr(1-CN) | H | CF₃ | H | O |
| C-4429 | H | single bond | Me | CH₂CF₃ | H | OMe | H | O |
| C-4430 | Me | single bond | H | CH₂CF₃ | H | OMe | H | O |
| C-4431 | Me(R) | single bond | H | CH₂CF₃ | H | OMe | H | O |
| C-4432 | Me(S) | single bond | H | CH₂CF₃ | H | OMe | H | O |
| C-4433 | H | CH₂ | Me | CH₂CF₃ | H | OMe | H | O |
| C-4434 | Me | CH₂ | H | CH₂CF₃ | H | OMe | H | O |
| C-4435 | H | CH(Me) | H | CH₂CF₃ | H | OMe | H | O |
| C-4436 | H | CH₂CH₂ | Me | CH₂CF₃ | H | OMe | H | O |
| C-4437 | Me | CH₂CH₂ | H | CH₂CF₃ | H | OMe | H | O |
| C-4438 | H | single bond | Me | Et | H | OMe | H | O |

**[Table 328]**

| Compound Number | Y | W | Z | R¹ | R² | R³ | R¹⁰ | A |
|---|---|---|---|---|---|---|---|---|
| C-4439 | Me | single bond | H | Et | H | OMe | H | O |
| C-4440 | Me(R) | single bond | H | Et | H | OMe | H | O |
| C-4441 | Me(S) | single bond | H | Et | H | OMe | H | O |
| C-4442 | H | CH₂ | Me | Et | H | OMe | H | O |
| C-4443 | Me | CH₂ | H | Et | H | OMe | H | O |
| C-4444 | H | CH(Me) | H | Et | H | OMe | H | O |
| C-4445 | H | CH₂CH₂ | Me | Et | H | OMe | H | O |
| C-4446 | Me | CH₂CH₂ | H | Et | H | OMe | H | O |
| C-4447 | H | single bond | Me | c-Pr | H | OMe | H | O |
| C-4448 | Me | single bond | H | c-Pr | H | OMe | H | O |
| C-4449 | Me(R) | single bond | H | c-Pr | H | OMe | H | O |
| C-4450 | Me(S) | single bond | H | c-Pr | H | OMe | H | O |
| C-4451 | H | CH₂ | Me | c-Pr | H | OMe | H | O |
| C-4452 | Me | CH₂ | H | c-Pr | H | OMe | H | O |
| C-4453 | H | CH(Me) | H | c-Pr | H | OMe | H | O |
| C-4454 | H | CH₂CH₂ | Me | c-Pr | H | OMe | H | O |
| C-4455 | Me | CH₂CH₂ | H | c-Pr | H | OMe | H | O |
| C-4456 | H | single bond | Me | c-Pr(1-CN) | H | OMe | H | O |
| C-4457 | Me | single bond | H | c-Pr(1-CN) | H | OMe | H | O |
| C-4458 | Me(R) | single bond | H | c-Pr(1-CN) | H | OMe | H | O |
| C-4459 | Me(S) | single bond | H | c-Pr(1-CN) | H | OMe | H | O |
| C-4460 | H | CH₂ | Me | c-Pr(1-CN) | H | OMe | H | O |
| C-4461 | Me | CH₂ | H | c-Pr(1-CN) | H | OMe | H | O |
| C-4462 | H | CH(Me) | H | c-Pr(1-CN) | H | OMe | H | O |
| C-4463 | H | CH₂CH₂ | Me | c-Pr(1-CN) | H | OMe | H | O |
| C-4464 | Me | CH₂CH₂ | H | c-Pr(1-CN) | H | OMe | H | O |
| C-4465 | H | single bond | Me | CH₂CF₃ | H | OCF₃ | H | O |
| C-4466 | Me | single bond | H | CH₂CF₃ | H | OCF₃ | H | O |
| C-4467 | Me(R) | single bond | H | CH₂CF₃ | H | OCF₃ | H | O |
| C-4468 | Me(S) | single bond | H | CH₂CF₃ | H | OCF₃ | H | O |
| C-4469 | H | CH₂ | Me | CH₂CF₃ | H | OCF₃ | H | O |
| C-4470 | Me | CH₂ | H | CH₂CF₃ | H | OCF₃ | H | O |
| C-4471 | H | CH(Me) | H | CH₂CF₃ | H | OCF₃ | H | O |
| C-4472 | H | CH₂CH₂ | Me | CH₂CF₃ | H | OCF₃ | H | O |
| C-4473 | Me | CH₂CH₂ | H | CH₂CF₃ | H | OCF₃ | H | O |
| C-4474 | H | single bond | Me | Et | H | OCF₃ | H | O |
| C-4475 | Me | single bond | H | Et | H | OCF₃ | H | O |
| C-4476 | Me(R) | single bond | H | Et | H | OCF₃ | H | O |
| C-4477 | Me(S) | single bond | H | Et | H | OCF₃ | H | O |
| C-4478 | H | CH₂ | Me | Et | H | OCF₃ | H | O |
| C-4479 | Me | CH₂ | H | Et | H | OCF₃ | H | O |
| C-4480 | H | CH(Me) | H | Et | H | OCF₃ | H | O |
| C-4481 | H | CH₂CH₂ | Me | Et | H | OCF₃ | H | O |
| C-4482 | Me | CH₂CH₂ | H | Et | H | OCF₃ | H | O |

**[Table 329]**

| Compound Number | Y | W | Z | R¹ | R² | R³ | R¹⁰ | A |
|---|---|---|---|---|---|---|---|---|
| C-4483 | H | single bond | Me | c-Pr | H | OCF₃ | H | O |
| C-4484 | Me | single bond | H | c-Pr | H | OCF₃ | H | O |
| C-4485 | Me(R) | single bond | H | c-Pr | H | OCF₃ | H | O |
| C-4486 | Me(S) | single bond | H | c-Pr | H | OCF₃ | H | O |
| C-4487 | H | CH₂ | Me | c-Pr | H | OCF₃ | H | O |
| C-4488 | Me | CH₂ | H | c-Pr | H | OCF₃ | H | O |
| C-4489 | H | CH(Me) | H | c-Pr | H | OCF₃ | H | O |
| C-4490 | H | CH₂CH₂ | Me | c-Pr | H | OCF₃ | H | O |
| C-4491 | Me | CH₂CH₂ | H | c-Pr | H | OCF₃ | H | O |
| C-4492 | H | single bond | Me | c-Pr(1-CN) | H | OCF₃ | H | O |
| C-4493 | Me | single bond | H | c-Pr(1-CN) | H | OCF₃ | H | O |
| C-4494 | Me(R) | single bond | H | c-Pr(1-CN) | H | OCF₃ | H | O |
| C-4495 | Me(S) | single bond | H | c-Pr(1-CN) | H | OCF₃ | H | O |
| C-4496 | H | CH₂ | Me | c-Pr(1-CN) | H | OCF₃ | H | O |
| C-4497 | Me | CH₂ | H | c-Pr(1-CN) | H | OCF₃ | H | O |
| C-4498 | H | CH(Me) | H | c-Pr(1-CN) | H | OCF₃ | H | O |
| C-4499 | H | CH₂CH₂ | Me | c-Pr(1-CN) | H | OCF₃ | H | O |
| C-4500 | Me | CH₂CH₂ | H | c-Pr(1-CN) | H | OCF₃ | H | O |
| C-4501 | H | single bond | Me | CH₂CF₃ | H | Br | Me | O |
| C-4502 | Me | single bond | H | CH₂CF₃ | H | Br | Me | O |
| C-4503 | Me(R) | single bond | H | CH₂CF₃ | H | Br | Me | O |
| C-4504 | Me(S) | single bond | H | CH₂CF₃ | H | Br | Me | O |
| C-4505 | H | CH₂ | Me | CH₂CF₃ | H | Br | Me | O |
| C-4506 | Me | CH₂ | H | CH₂CF₃ | H | Br | Me | O |
| C-4507 | H | CH(Me) | H | CH₂CF₃ | H | Br | Me | O |
| C-4508 | H | CH₂CH₂ | Me | CH₂CF₃ | H | Br | Me | O |
| C-4509 | Me | CH₂CH₂ | H | CH₂CF₃ | H | Br | Me | O |
| C-4510 | H | single bond | Me | Et | H | Br | Me | O |
| C-4511 | Me | single bond | H | Et | H | Br | Me | O |
| C-4512 | Me(R) | single bond | H | Et | H | Br | Me | O |
| C-4513 | Me(S) | single bond | H | Et | H | Br | Me | O |
| C-4514 | H | CH₂ | Me | Et | H | Br | Me | O |
| C-4515 | Me | CH₂ | H | Et | H | Br | Me | O |
| C-4516 | H | CH(Me) | H | Et | H | Br | Me | O |
| C-4517 | H | CH₂CH₂ | Me | Et | H | Br | Me | O |
| C-4518 | Me | CH₂CH₂ | H | Et | H | Br | Me | O |
| C-4519 | H | single bond | Me | c-Pr | H | Br | Me | O |
| C-4520 | Me | single bond | H | c-Pr | H | Br | Me | O |
| C-4521 | Me(R) | single bond | H | c-Pr | H | Br | Me | O |
| C-4522 | Me(S) | single bond | H | c-Pr | H | Br | Me | O |
| C-4523 | H | CH₂ | Me | c-Pr | H | Br | Me | O |
| C-4524 | Me | CH₂ | H | c-Pr | H | Br | Me | O |
| C-4525 | H | CH(Me) | H | c-Pr | H | Br | Me | O |
| C-4526 | H | CH₂CH₂ | Me | c-Pr | H | Br | Me | O |

**[Table 330]**

| Compound Number | Y | W | Z | R¹ | R² | R³ | R¹⁰ | A |
|---|---|---|---|---|---|---|---|---|
| C-4527 | Me | CH₂CH₂ | H | c-Pr | H | Br | Me | O |
| C-4528 | H | single bond | Me | c-Pr(1-CN) | H | Br | Me | O |
| C-4529 | Me | single bond | H | c-Pr(1-CN) | H | Br | Me | O |
| C-4530 | Me(R) | single bond | H | c-Pr(1-CN) | H | Br | Me | O |
| C-4531 | Me(S) | single bond | H | c-Pr(1-CN) | H | Br | Me | O |
| C-4532 | H | CH₂ | Me | c-Pr(1-CN) | H | Br | Me | O |
| C-4533 | Me | CH₂ | H | c-Pr(1-CN) | H | Br | Me | O |
| C-4534 | H | CH(Me) | H | c-Pr(1-CN) | H | Br | Me | O |
| C-4535 | H | CH₂CH₂ | Me | c-Pr(1-CN) | H | Br | Me | O |
| C-4536 | Me | CH₂CH₂ | H | c-Pr(1-CN) | H | Br | Me | O |
| C-4537 | H | single bond | Me | CH₂CF₃ | H | CF₃ | Me | O |
| C-4538 | Me | single bond | H | CH₂CF₃ | H | CF₃ | Me | O |
| C-4539 | Me(R) | single bond | H | CH₂CF₃ | H | CF₃ | Me | O |
| C-4540 | Me(S) | single bond | H | CH₂CF₃ | H | CF₃ | Me | O |
| C-4541 | H | CH₂ | Me | CH₂CF₃ | H | CF₃ | Me | O |
| C-4542 | Me | CH₂ | H | CH₂CF₃ | H | CF₃ | Me | O |
| C-4543 | H | CH(Me) | H | CH₂CF₃ | H | CF₃ | Me | O |
| C-4544 | H | CH₂CH₂ | Me | CH₂CF₃ | H | CF₃ | Me | O |
| C-4545 | Me | CH₂CH₂ | H | CH₂CF₃ | H | CF₃ | Me | O |
| C-4546 | H | single bond | Me | Et | H | CF₃ | Me | O |
| C-4547 | Me | single bond | H | Et | H | CF₃ | Me | O |
| C-4548 | Me(R) | single bond | H | Et | H | CF₃ | Me | O |
| C-4549 | Me(S) | single bond | H | Et | H | CF₃ | Me | O |
| C-4550 | H | CH₂ | Me | Et | H | CF₃ | Me | O |
| C-4551 | Me | CH₂ | H | Et | H | CF₃ | Me | O |
| C-4552 | H | CH(Me) | H | Et | H | CF₃ | Me | O |
| C-4553 | H | CH₂CH₂ | Me | Et | H | CF₃ | Me | O |
| C-4554 | Me | CH₂CH₂ | H | Et | H | CF₃ | Me | O |
| C-4555 | H | single bond | Me | c-Pr | H | CF₃ | Me | O |
| C-4556 | Me | single bond | H | c-Pr | H | CF₃ | Me | O |
| C-4557 | Me(R) | single bond | H | c-Pr | H | CF₃ | Me | O |
| C-4558 | Me(S) | single bond | H | c-Pr | H | CF₃ | Me | O |
| C-4559 | H | CH₂ | Me | c-Pr | H | CF₃ | Me | O |
| C-4560 | Me | CH₂ | H | c-Pr | H | CF₃ | Me | O |
| C-4561 | H | CH(Me) | H | c-Pr | H | CF₃ | Me | O |
| C-4562 | H | CH₂CH₂ | Me | c-Pr | H | CF₃ | Me | O |
| C-4563 | Me | CH₂CH₂ | H | c-Pr | H | CF₃ | Me | O |
| C-4564 | H | single bond | Me | c-Pr(1-CN) | H | CF₃ | Me | O |
| C-4565 | Me | single bond | H | c-Pr(1-CN) | H | CF₃ | Me | O |
| C-4566 | Me(R) | single bond | H | c-Pr(1-CN) | H | CF₃ | Me | O |
| C-4567 | Me(S) | single bond | H | c-Pr(1-CN) | H | CF₃ | Me | O |
| C-4568 | H | CH₂ | Me | c-Pr(1-CN) | H | CF₃ | Me | O |
| C-4569 | Me | CH₂ | H | c-Pr(1-CN) | H | CF₃ | Me | O |
| C-4570 | H | CH(Me) | H | c-Pr(1-CN) | H | CF₃ | Me | O |

**[Table 331]**

| Compound Number | Y | W | Z | R¹ | R² | R³ | R¹⁰ | A |
|---|---|---|---|---|---|---|---|---|
| C-4571 | H | CH₂CH₂ | Me | c-Pr(1-CN) | H | CF₃ | Me | O |
| C-4572 | Me | CH₂CH₂ | H | c-Pr(1-CN) | H | CF₃ | Me | O |
| C-4573 | H | single bond | Me | CH₂CF₃ | H | OMe | Me | O |
| C-4574 | Me | single bond | H | CH₂CF₃ | H | OMe | Me | O |
| C-4575 | Me(R) | single bond | H | CH₂CF₃ | H | OMe | Me | O |
| C-4576 | Me(S) | single bond | H | CH₂CF₃ | H | OMe | Me | O |
| C-4577 | H | CH₂ | Me | CH₂CF₃ | H | OMe | Me | O |
| C-4578 | Me | CH₂ | H | CH₂CF₃ | H | OMe | Me | O |
| C-4579 | H | CH(Me) | H | CH₂CF₃ | H | OMe | Me | O |
| C-4580 | H | CH₂CH₂ | Me | CH₂CF₃ | H | OMe | Me | O |
| C-4581 | Me | CH₂CH₂ | H | CH₂CF₃ | H | OMe | Me | O |
| C-4582 | H | single bond | Me | Et | H | OMe | Me | O |
| C-4583 | Me | single bond | H | Et | H | OMe | Me | O |
| C-4584 | Me(R) | single bond | H | Et | H | OMe | Me | O |
| C-4585 | Me(S) | single bond | H | Et | H | OMe | Me | O |
| C-4586 | H | CH₂ | Me | Et | H | OMe | Me | O |
| C-4587 | Me | CH₂ | H | Et | H | OMe | Me | O |
| C-4588 | H | CH(Me) | H | Et | H | OMe | Me | O |
| C-4589 | H | CH₂CH₂ | Me | Et | H | OMe | Me | O |
| C-4590 | Me | CH₂CH₂ | H | Et | H | OMe | Me | O |
| C-4591 | H | single bond | Me | c-Pr | H | OMe | Me | O |
| C-4592 | Me | single bond | H | c-Pr | H | OMe | Me | O |
| C-4593 | Me(R) | single bond | H | c-Pr | H | OMe | Me | O |
| C-4594 | Me(S) | single bond | H | c-Pr | H | OMe | Me | O |
| C-4595 | H | CH₂ | Me | c-Pr | H | OMe | Me | O |
| C-4596 | Me | CH₂ | H | c-Pr | H | OMe | Me | O |
| C-4597 | H | CH(Me) | H | c-Pr | H | OMe | Me | O |
| C-4598 | H | CH₂CH₂ | Me | c-Pr | H | OMe | Me | O |
| C-4599 | Me | CH₂CH₂ | H | c-Pr | H | OMe | Me | O |
| C-4600 | H | single bond | Me | c-Pr(1-CN) | H | OMe | Me | O |
| C-4601 | Me | single bond | H | c-Pr(1-CN) | H | OMe | Me | O |
| C-4602 | Me(R) | single bond | H | c-Pr(1-CN) | H | OMe | Me | O |
| C-4603 | Me(S) | single bond | H | c-Pr(1-CN) | H | OMe | Me | O |
| C-4604 | H | CH₂ | Me | c-Pr(1-CN) | H | OMe | Me | O |
| C-4605 | Me | CH₂ | H | c-Pr(1-CN) | H | OMe | Me | O |
| C-4606 | H | CH(Me) | H | c-Pr(1-CN) | H | OMe | Me | O |
| C-4607 | H | CH₂CH₂ | Me | c-Pr(1-CN) | H | OMe | Me | O |
| C-4608 | Me | CH₂CH₂ | H | c-Pr(1-CN) | H | OMe | Me | O |
| C-4609 | H | single bond | Me | CH₂CF₃ | H | OCF₃ | Me | O |
| C-4610 | Me | single bond | H | CH₂CF₃ | H | OCF₃ | Me | O |
| C-4611 | Me(R) | single bond | H | CH₂CF₃ | H | OCF₃ | Me | O |
| C-4612 | Me(S) | single bond | H | CH₂CF₃ | H | OCF₃ | Me | O |
| C-4613 | H | CH₂ | Me | CH₂CF₃ | H | OCF₃ | Me | O |
| C-4614 | Me | CH₂ | H | CH₂CF₃ | H | OCF₃ | Me | O |

**[Table 332]**

| Compound Number | Y | W | Z | R¹ | R² | R³ | R¹⁰ | A |
|---|---|---|---|---|---|---|---|---|
| C-4615 | H | CH(Me) | H | CH₂CF₃ | H | OCF₃ | Me | O |
| C-4616 | H | CH₂CH₂ | Me | CH₂CF₃ | H | OCF₃ | Me | O |
| C-4617 | Me | CH₂CH₂ | H | CH₂CF₃ | H | OCF₃ | Me | O |
| C-4618 | H | single bond | Me | Et | H | OCF₃ | Me | O |
| C-4619 | Me | single bond | H | Et | H | OCF₃ | Me | O |
| C-4620 | Me(R) | single bond | H | Et | H | OCF₃ | Me | O |
| C-4621 | Me(S) | single bond | H | Et | H | OCF₃ | Me | O |
| C-4622 | H | CH₂ | Me | Et | H | OCF₃ | Me | O |
| C-4623 | Me | CH₂ | H | Et | H | OCF₃ | Me | O |
| C-4624 | H | CH(Me) | H | Et | H | OCF₃ | Me | O |
| C-4625 | H | CH₂CH₂ | Me | Et | H | OCF₃ | Me | O |
| C-4626 | Me | CH₂CH₂ | H | Et | H | OCF₃ | Me | O |
| C-4627 | H | single bond | Me | c-Pr | H | OCF₃ | Me | O |
| C-4628 | Me | single bond | H | c-Pr | H | OCF₃ | Me | O |
| C-4629 | Me(R) | single bond | H | c-Pr | H | OCF₃ | Me | O |
| C-4630 | Me(S) | single bond | H | c-Pr | H | OCF₃ | Me | O |
| C-4631 | H | CH₂ | Me | c-Pr | H | OCF₃ | Me | O |
| C-4632 | Me | CH₂ | H | c-Pr | H | OCF₃ | Me | O |
| C-4633 | H | CH(Me) | H | c-Pr | H | OCF₃ | Me | O |
| C-4634 | H | CH₂CH₂ | Me | c-Pr | H | OCF₃ | Me | O |
| C-4635 | Me | CH₂CH₂ | H | c-Pr | H | OCF₃ | Me | O |
| C-4636 | H | single bond | Me | c-Pr(1-CN) | H | OCF₃ | Me | O |
| C-4637 | Me | single bond | H | c-Pr(1-CN) | H | OCF₃ | Me | O |
| C-4638 | Me(R) | single bond | H | c-Pr(1-CN) | H | OCF₃ | Me | O |
| C-4639 | Me(S) | single bond | H | c-Pr(1-CN) | H | OCF₃ | Me | O |
| C-4640 | H | CH₂ | Me | c-Pr(1-CN) | H | OCF₃ | Me | O |
| C-4641 | Me | CH₂ | H | c-Pr(1-CN) | H | OCF₃ | Me | O |
| C-4642 | H | CH(Me) | H | c-Pr(1-CN) | H | OCF₃ | Me | O |
| C-4643 | H | CH₂CH₂ | Me | c-Pr(1-CN) | H | OCF₃ | Me | O |
| C-4644 | Me | CH₂CH₂ | H | c-Pr(1-CN) | H | OCF₃ | Me | O |
| C-4645 | H | single bond | Me | CH₂CF₃ | H | Br | CH₂OMe | O |
| C-4646 | Me | single bond | H | CH₂CF₃ | H | Br | CH₂OMe | O |
| C-4647 | Me(R) | single bond | H | CH₂CF₃ | H | Br | CH₂OMe | O |
| C-4648 | Me(S) | single bond | H | CH₂CF₃ | H | Br | CH₂OMe | O |
| C-4649 | H | CH₂ | Me | CH₂CF₃ | H | Br | CH₂OMe | O |
| C-4650 | Me | CH₂ | H | CH₂CF₃ | H | Br | CH₂OMe | O |
| C-4651 | H | CH(Me) | H | CH₂CF₃ | H | Br | CH₂OMe | O |
| C-4652 | H | CH₂CH₂ | Me | CH₂CF₃ | H | Br | CH₂OMe | O |
| C-4653 | Me | CH₂CH₂ | H | CH₂CF₃ | H | Br | CH₂OMe | O |
| C-4654 | H | single bond | Me | Et | H | Br | CH₂OMe | O |
| C-4655 | Me | single bond | H | Et | H | Br | CH₂OMe | O |
| C-4656 | Me(R) | single bond | H | Et | H | Br | CH₂OMe | O |
| C-4657 | Me(S) | single bond | H | Et | H | Br | CH₂OMe | O |
| C-4658 | H | CH₂ | Me | Et | H | Br | CH₂OMe | O |

**[Table 333]**

| Compound Number | Y | W | Z | R¹ | R² | R³ | R¹⁰ | A |
|---|---|---|---|---|---|---|---|---|
| C-4659 | Me | CH₂ | H | Et | H | Br | CH₂OMe | O |
| C-4660 | H | CH(Me) | H | Et | H | Br | CH₂OMe | O |
| C-4661 | H | CH₂CH₂ | Me | Et | H | Br | CH₂OMe | O |
| C-4662 | Me | CH₂CH₂ | H | Et | H | Br | CH₂OMe | O |
| C-4663 | H | single bond | Me | c-Pr | H | Br | CH₂OMe | O |
| C-4664 | Me | single bond | H | c-Pr | H | Br | CH₂OMe | O |
| C-4665 | Me(R) | single bond | H | c-Pr | H | Br | CH₂OMe | O |
| C-4666 | Me(S) | single bond | H | c-Pr | H | Br | CH₂OMe | O |
| C-4667 | H | CH₂ | Me | c-Pr | H | Br | CH₂OMe | O |
| C-4668 | Me | CH₂ | H | c-Pr | H | Br | CH₂OMe | O |
| C-4669 | H | CH(Me) | H | c-Pr | H | Br | CH₂OMe | O |
| C-4670 | H | CH₂CH₂ | Me | c-Pr | H | Br | CH₂OMe | O |
| C-4671 | Me | CH₂CH₂ | H | c-Pr | H | Br | CH₂OMe | O |
| C-4672 | H | single bond | Me | c-Pr(1-CN) | H | Br | CH₂OMe | O |
| C-4673 | Me | single bond | H | c-Pr(1-CN) | H | Br | CH₂OMe | O |
| C-4674 | Me(R) | single bond | H | c-Pr(1-CN) | H | Br | CH₂OMe | O |
| C-4675 | Me(S) | single bond | H | c-Pr(1-CN) | H | Br | CH₂OMe | O |
| C-4676 | H | CH₂ | Me | c-Pr(1-CN) | H | Br | CH₂OMe | O |
| C-4677 | Me | CH₂ | H | c-Pr(1-CN) | H | Br | CH₂OMe | O |
| C-4678 | H | CH(Me) | H | c-Pr(1-CN) | H | Br | CH₂OMe | O |
| C-4679 | H | CH₂CH₂ | Me | c-Pr(1-CN) | H | Br | CH₂OMe | O |
| C-4680 | Me | CH₂CH₂ | H | c-Pr(1-CN) | H | Br | CH₂OMe | O |
| C-4681 | H | single bond | Me | CH₂CF₃ | H | CF₃ | CH₂OMe | O |
| C-4682 | Me | single bond | H | CH₂CF₃ | H | CF₃ | CH₂OMe | O |
| C-4683 | Me(R) | single bond | H | CH₂CF₃ | H | CF₃ | CH₂OMe | O |
| C-4684 | Me(S) | single bond | H | CH₂CF₃ | H | CF₃ | CH₂OMe | O |
| C-4685 | H | CH₂ | Me | CH₂CF₃ | H | CF₃ | CH₂OMe | O |
| C-4686 | Me | CH₂ | H | CH₂CF₃ | H | CF₃ | CH₂OMe | O |
| C-4687 | H | CH(Me) | H | CH₂CF₃ | H | CF₃ | CH₂OMe | O |
| C-4688 | H | CH₂CH₂ | Me | CH₂CF₃ | H | CF₃ | CH₂OMe | O |
| C-4689 | Me | CH₂CH₂ | H | CH₂CF₃ | H | CF₃ | CH₂OMe | O |
| C-4690 | H | single bond | Me | Et | H | CF₃ | CH₂OMe | O |
| C-4691 | Me | single bond | H | Et | H | CF₃ | CH₂OMe | O |
| C-4692 | Me(R) | single bond | H | Et | H | CF₃ | CH₂OMe | O |
| C-4693 | Me(S) | single bond | H | Et | H | CF₃ | CH₂OMe | O |
| C-4694 | H | CH₂ | Me | Et | H | CF₃ | CH₂OMe | O |
| C-4695 | Me | CH₂ | H | Et | H | CF₃ | CH₂OMe | O |
| C-4696 | H | CH(Me) | H | Et | H | CF₃ | CH₂OMe | O |
| C-4697 | H | CH₂CH₂ | Me | Et | H | CF₃ | CH₂OMe | O |
| C-4698 | Me | CH₂CH₂ | H | Et | H | CF₃ | CH₂OMe | O |
| C-4699 | H | single bond | Me | c-Pr | H | CF₃ | CH₂OMe | O |
| C-4700 | Me | single bond | H | c-Pr | H | CF₃ | CH₂OMe | O |
| C-4701 | Me(R) | single bond | H | c-Pr | H | CF₃ | CH₂OMe | O |
| C-4702 | Me(S) | single bond | H | c-Pr | H | CF₃ | CH₂OMe | O |

**[Table 334]**

| Compound Number | Y | W | Z | R¹ | R² | R³ | R¹⁰ | A |
|---|---|---|---|---|---|---|---|---|
| C-4703 | H | CH₂ | Me | c-Pr | H | CF₃ | CH₂OMe | O |
| C-4704 | Me | CH₂ | H | c-Pr | H | CF₃ | CH₂OMe | O |
| C-4705 | H | CH(Me) | H | c-Pr | H | CF₃ | CH₂OMe | O |
| C-4706 | H | CH₂CH₂ | Me | c-Pr | H | CF₃ | CH₂OMe | O |
| C-4707 | Me | CH₂CH₂ | H | c-Pr | H | CF₃ | CH₂OMe | O |
| C-4708 | H | single bond | Me | c-Pr(1-CN) | H | CF₃ | CH₂OMe | O |
| C-4709 | Me | single bond | H | c-Pr(1-CN) | H | CF₃ | CH₂OMe | O |
| C-4710 | Me(R) | single bond | H | c-Pr(1-CN) | H | CF₃ | CH₂OMe | O |
| C-4711 | Me(S) | single bond | H | c-Pr(1-CN) | H | CF₃ | CH₂OMe | O |
| C-4712 | H | CH₂ | Me | c-Pr(1-CN) | H | CF₃ | CH₂OMe | O |
| C-4713 | Me | CH₂ | H | c-Pr(1-CN) | H | CF₃ | CH₂OMe | O |
| C-4714 | H | CH(Me) | H | c-Pr(1-CN) | H | CF₃ | CH₂OMe | O |
| C-4715 | H | CH₂CH₂ | Me | c-Pr(1-CN) | H | CF₃ | CH₂OMe | O |
| C-4716 | Me | CH₂CH₂ | H | c-Pr(1-CN) | H | CF₃ | CH₂OMe | O |
| C-4717 | H | single bond | Me | CH₂CF₃ | H | OMe | CH₂OMe | O |
| C-4718 | Me | single bond | H | CH₂CF₃ | H | OMe | CH₂OMe | O |
| C-4719 | Me(R) | single bond | H | CH₂CF₃ | H | OMe | CH₂OMe | O |
| C-4720 | Me(S) | single bond | H | CH₂CF₃ | H | OMe | CH₂OMe | O |
| C-4721 | H | CH₂ | Me | CH₂CF₃ | H | OMe | CH₂OMe | O |
| C-4722 | Me | CH₂ | H | CH₂CF₃ | H | OMe | CH₂OMe | O |
| C-4723 | H | CH(Me) | H | CH₂CF₃ | H | OMe | CH₂OMe | O |
| C-4724 | H | CH₂CH₂ | Me | CH₂CF₃ | H | OMe | CH₂OMe | O |
| C-4725 | Me | CH₂CH₂ | H | CH₂CF₃ | H | OMe | CH₂OMe | O |
| C-4726 | H | single bond | Me | Et | H | OMe | CH₂OMe | O |
| C-4727 | Me | single bond | H | Et | H | OMe | CH₂OMe | O |
| C-4728 | Me(R) | single bond | H | Et | H | OMe | CH₂OMe | O |
| C-4729 | Me(S) | single bond | H | Et | H | OMe | CH₂OMe | O |
| C-4730 | H | CH₂ | Me | Et | H | OMe | CH₂OMe | O |
| C-4731 | Me | CH₂ | H | Et | H | OMe | CH₂OMe | O |
| C-4732 | H | CH(Me) | H | Et | H | OMe | CH₂OMe | O |
| C-4733 | H | CH₂CH₂ | Me | Et | H | OMe | CH₂OMe | O |
| C-4734 | Me | CH₂CH₂ | H | Et | H | OMe | CH₂OMe | O |
| C-4735 | H | single bond | Me | c-Pr | H | OMe | CH₂OMe | O |
| C-4736 | Me | single bond | H | c-Pr | H | OMe | CH₂OMe | O |
| C-4737 | Me(R) | single bond | H | c-Pr | H | OMe | CH₂OMe | O |
| C-4738 | Me(S) | single bond | H | c-Pr | H | OMe | CH₂OMe | O |
| C-4739 | H | CH₂ | Me | c-Pr | H | OMe | CH₂OMe | O |
| C-4740 | Me | CH₂ | H | c-Pr | H | OMe | CH₂OMe | O |
| C-4741 | H | CH(Me) | H | c-Pr | H | OMe | CH₂OMe | O |
| C-4742 | H | CH₂CH₂ | Me | c-Pr | H | OMe | CH₂OMe | O |
| C-4743 | Me | CH₂CH₂ | H | c-Pr | H | OMe | CH₂OMe | O |
| C-4744 | H | single bond | Me | c-Pr(1-CN) | H | OMe | CH₂OMe | O |
| C-4745 | Me | single bond | H | c-Pr(1-CN) | H | OMe | CH₂OMe | O |
| C-4746 | Me(R) | single bond | H | c-Pr(1-CN) | H | OMe | CH₂OMe | O |

**[Table 335]**

| Compound Number | Y | W | Z | R¹ | R² | R³ | R¹⁰ | A |
|---|---|---|---|---|---|---|---|---|
| C-4747 | Me(S) | single bond | H | c-Pr(1-CN) | H | OMe | CH₂OMe | O |
| C-4748 | H | CH₂ | Me | c-Pr(1-CN) | H | OMe | CH₂OMe | O |
| C-4749 | Me | CH₂ | H | c-Pr(1-CN) | H | OMe | CH₂OMe | O |
| C-4750 | H | CH(Me) | H | c-Pr(1-CN) | H | OMe | CH₂OMe | O |
| C-4751 | H | CH₂CH₂ | Me | c-Pr(1-CN) | H | OMe | CH₂OMe | O |
| C-4752 | Me | CH₂CH₂ | H | c-Pr(1-CN) | H | OMe | CH₂OMe | O |
| C-4753 | H | single bond | Me | CH₂CF₃ | H | OCF₃ | CH₂OMe | O |
| C-4754 | Me | single bond | H | CH₂CF₃ | H | OCF₃ | CH₂OMe | O |
| C-4755 | Me(R) | single bond | H | CH₂CF₃ | H | OCF₃ | CH₂OMe | O |
| C-4756 | Me(S) | single bond | H | CH₂CF₃ | H | OCF₃ | CH₂OMe | O |
| C-4757 | H | CH₂ | Me | CH₂CF₃ | H | OCF₃ | CH₂OMe | O |
| C-4758 | Me | CH₂ | H | CH₂CF₃ | H | OCF₃ | CH₂OMe | O |
| C-4759 | H | CH(Me) | H | CH₂CF₃ | H | OCF₃ | CH₂OMe | O |
| C-4760 | H | CH₂CH₂ | Me | CH₂CF₃ | H | OCF₃ | CH₂OMe | O |
| C-4761 | Me | CH₂CH₂ | H | CH₂CF₃ | H | OCF₃ | CH₂OMe | O |
| C-4762 | H | single bond | Me | Et | H | OCF₃ | CH₂OMe | O |
| C-4763 | Me | single bond | H | Et | H | OCF₃ | CH₂OMe | O |
| C-4764 | Me(R) | single bond | H | Et | H | OCF₃ | CH₂OMe | O |
| C-4765 | Me(S) | single bond | H | Et | H | OCF₃ | CH₂OMe | O |
| C-4766 | H | CH₂ | Me | Et | H | OCF₃ | CH₂OMe | O |
| C-4767 | Me | CH₂ | H | Et | H | OCF₃ | CH₂OMe | O |
| C-4768 | H | CH(Me) | H | Et | H | OCF₃ | CH₂OMe | O |
| C-4769 | H | CH₂CH₂ | Me | Et | H | OCF₃ | CH₂OMe | O |
| C-4770 | Me | CH₂CH₂ | H | Et | H | OCF₃ | CH₂OMe | O |
| C-4771 | H | single bond | Me | c-Pr | H | OCF₃ | CH₂OMe | O |
| C-4772 | Me | single bond | H | c-Pr | H | OCF₃ | CH₂OMe | O |
| C-4773 | Me(R) | single bond | H | c-Pr | H | OCF₃ | CH₂OMe | O |
| C-4774 | Me(S) | single bond | H | c-Pr | H | OCF₃ | CH₂OMe | O |
| C-4775 | H | CH₂ | Me | c-Pr | H | OCF₃ | CH₂OMe | O |
| C-4776 | Me | CH₂ | H | c-Pr | H | OCF₃ | CH₂OMe | O |
| C-4777 | H | CH(Me) | H | c-Pr | H | OCF₃ | CH₂OMe | O |
| C-4778 | H | CH₂CH₂ | Me | c-Pr | H | OCF₃ | CH₂OMe | O |
| C-4779 | Me | CH₂CH₂ | H | c-Pr | H | OCF₃ | CH₂OMe | O |
| C-4780 | H | single bond | Me | c-Pr(1-CN) | H | OCF₃ | CH₂OMe | O |
| C-4781 | Me | single bond | H | c-Pr(1-CN) | H | OCF₃ | CH₂OMe | O |
| C-4782 | Me(R) | single bond | H | c-Pr(1-CN) | H | OCF₃ | CH₂OMe | O |
| C-4783 | Me(S) | single bond | H | c-Pr(1-CN) | H | OCF₃ | CH₂OMe | O |
| C-4784 | H | CH₂ | Me | c-Pr(1-CN) | H | OCF₃ | CH₂OMe | O |
| C-4785 | Me | CH₂ | H | c-Pr(1-CN) | H | OCF₃ | CH₂OMe | O |
| C-4786 | H | CH(Me) | H | c-Pr(1-CN) | H | OCF₃ | CH₂OMe | O |
| C-4787 | H | CH₂CH₂ | Me | c-Pr(1-CN) | H | OCF₃ | CH₂OMe | O |
| C-4788 | Me | CH₂CH₂ | H | c-Pr(1-CN) | H | OCF₃ | CH₂OMe | O |
| C-4789 | H | single bond | Me | CH₂CF₃ | H | Br | CH₂CN | O |
| C-4790 | Me | single bond | H | CH₂CF₃ | H | Br | CH₂CN | O |

**[Table 336]**

| Compound Number | Y | W | Z | R¹ | R² | R³ | R¹⁰ | A |
|---|---|---|---|---|---|---|---|---|
| C-4791 | Me(R) | single bond | H | CH₂CF₃ | H | Br | CH₂CN | O |
| C-4792 | Me(S) | single bond | H | CH₂CF₃ | H | Br | CH₂CN | O |
| C-4793 | H | CH₂ | Me | CH₂CF₃ | H | Br | CH₂CN | O |
| C-4794 | Me | CH₂ | H | CH₂CF₃ | H | Br | CH₂CN | O |
| C-4795 | H | CH(Me) | H | CH₂CF₃ | H | Br | CH₂CN | O |
| C-4796 | H | CH₂CH₂ | Me | CH₂CF₃ | H | Br | CH₂CN | O |
| C-4797 | Me | CH₂CH₂ | H | CH₂CF₃ | H | Br | CH₂CN | O |
| C-4798 | H | single bond | Me | Et | H | Br | CH₂CN | O |
| C-4799 | Me | single bond | H | Et | H | Br | CH₂CN | O |
| C-4800 | Me(R) | single bond | H | Et | H | Br | CH₂CN | O |
| C-4801 | Me(S) | single bond | H | Et | H | Br | CH₂CN | O |
| C-4802 | H | CH₂ | Me | Et | H | Br | CH₂CN | O |
| C-4803 | Me | CH₂ | H | Et | H | Br | CH₂CN | O |
| C-4804 | H | CH(Me) | H | Et | H | Br | CH₂CN | O |
| C-4805 | H | CH₂CH₂ | Me | Et | H | Br | CH₂CN | O |
| C-4806 | Me | CH₂CH₂ | H | Et | H | Br | CH₂CN | O |
| C-4807 | H | single bond | Me | c-Pr | H | Br | CH₂CN | O |
| C-4808 | Me | single bond | H | c-Pr | H | Br | CH₂CN | O |
| C-4809 | Me(R) | single bond | H | c-Pr | H | Br | CH₂CN | O |
| C-4810 | Me(S) | single bond | H | c-Pr | H | Br | CH₂CN | O |
| C-4811 | H | CH₂ | Me | c-Pr | H | Br | CH₂CN | O |
| C-4812 | Me | CH₂ | H | c-Pr | H | Br | CH₂CN | O |
| C-4813 | H | CH(Me) | H | c-Pr | H | Br | CH₂CN | O |
| C-4814 | H | CH₂CH₂ | Me | c-Pr | H | Br | CH₂CN | O |
| C-4815 | Me | CH₂CH₂ | H | c-Pr | H | Br | CH₂CN | O |
| C-4816 | H | single bond | Me | c-Pr(1-CN) | H | Br | CH₂CN | O |
| C-4817 | Me | single bond | H | c-Pr(1-CN) | H | Br | CH₂CN | O |
| C-4818 | Me(R) | single bond | H | c-Pr(1-CN) | H | Br | CH₂CN | O |
| C-4819 | Me(S) | single bond | H | c-Pr(1-CN) | H | Br | CH₂CN | O |
| C-4820 | H | CH₂ | Me | c-Pr(1-CN) | H | Br | CH₂CN | O |
| C-4821 | Me | CH₂ | H | c-Pr(1-CN) | H | Br | CH₂CN | O |
| C-4822 | H | CH(Me) | H | c-Pr(1-CN) | H | Br | CH₂CN | O |
| C-4823 | H | CH₂CH₂ | Me | c-Pr(1-CN) | H | Br | CH₂CN | O |
| C-4824 | Me | CH₂CH₂ | H | c-Pr(1-CN) | H | Br | CH₂CN | O |
| C-4825 | H | single bond | Me | CH₂CF₃ | H | CF₃ | CH₂CN | O |
| C-4826 | Me | single bond | H | CH₂CF₃ | H | CF₃ | CH₂CN | O |
| C-4827 | Me(R) | single bond | H | CH₂CF₃ | H | CF₃ | CH₂CN | O |
| C-4828 | Me(S) | single bond | H | CH₂CF₃ | H | CF₃ | CH₂CN | O |
| C-4829 | H | CH₂ | Me | CH₂CF₃ | H | CF₃ | CH₂CN | O |
| C-4830 | Me | CH₂ | H | CH₂CF₃ | H | CF₃ | CH₂CN | O |
| C-4831 | H | CH(Me) | H | CH₂CF₃ | H | CF₃ | CH₂CN | O |
| C-4832 | H | CH₂CH₂ | Me | CH₂CF₃ | H | CF₃ | CH₂CN | O |
| C-4833 | Me | CH₂CH₂ | H | CH₂CF₃ | H | CF₃ | CH₂CN | O |
| C-4834 | H | single bond | Me | Et | H | CF₃ | CH₂CN | O |

**[Table 337]**

| Compound Number | Y | W | Z | R¹ | R² | R³ | R¹⁰ | A |
|---|---|---|---|---|---|---|---|---|
| C-4835 | Me | single bond | H | Et | H | CF₃ | CH₂CN | O |
| C-4836 | Me(R) | single bond | H | Et | H | CF₃ | CH₂CN | O |
| C-4837 | Me(S) | single bond | H | Et | H | CF₃ | CH₂CN | O |
| C-4838 | H | CH₂ | Me | Et | H | CF₃ | CH₂CN | O |
| C-4839 | Me | CH₂ | H | Et | H | CF₃ | CH₂CN | O |
| C-4840 | H | CH(Me) | H | Et | H | CF₃ | CH₂CN | O |
| C-4841 | H | CH₂CH₂ | Me | Et | H | CF₃ | CH₂CN | O |
| C-4842 | Me | CH₂CH₂ | H | Et | H | CF₃ | CH₂CN | O |
| C-4843 | H | single bond | Me | c-Pr | H | CF₃ | CH₂CN | O |
| C-4844 | Me | single bond | H | c-Pr | H | CF₃ | CH₂CN | O |
| C-4845 | Me(R) | single bond | H | c-Pr | H | CF₃ | CH₂CN | O |
| C-4846 | Me(S) | single bond | H | c-Pr | H | CF₃ | CH₂CN | O |
| C-4847 | H | CH₂ | Me | c-Pr | H | CF₃ | CH₂CN | O |
| C-4848 | Me | CH₂ | H | c-Pr | H | CF₃ | CH₂CN | O |
| C-4849 | H | CH(Me) | H | c-Pr | H | CF₃ | CH₂CN | O |
| C-4850 | H | CH₂CH₂ | Me | c-Pr | H | CF₃ | CH₂CN | O |
| C-4851 | Me | CH₂CH₂ | H | c-Pr | H | CF₃ | CH₂CN | O |
| C-4852 | H | single bond | Me | c-Pr(1-CN) | H | CF₃ | CH₂CN | O |
| C-4853 | Me | single bond | H | c-Pr(1-CN) | H | CF₃ | CH₂CN | O |
| C-4854 | Me(R) | single bond | H | c-Pr(1-CN) | H | CF₃ | CH₂CN | O |
| C-4855 | Me(S) | single bond | H | c-Pr(1-CN) | H | CF₃ | CH₂CN | O |
| C-4856 | H | CH₂ | Me | c-Pr(1-CN) | H | CF₃ | CH₂CN | O |
| C-4857 | Me | CH₂ | H | c-Pr(1-CN) | H | CF₃ | CH₂CN | O |
| C-4858 | H | CH(Me) | H | c-Pr(1-CN) | H | CF₃ | CH₂CN | O |
| C-4859 | H | CH₂CH₂ | Me | c-Pr(1-CN) | H | CF₃ | CH₂CN | O |
| C-4860 | Me | CH₂CH₂ | H | c-Pr(1-CN) | H | CF₃ | CH₂CN | O |
| C-4861 | H | single bond | Me | CH₂CF₃ | H | OMe | CH₂CN | O |
| C-4862 | Me | single bond | H | CH₂CF₃ | H | OMe | CH₂CN | O |
| C-4863 | Me(R) | single bond | H | CH₂CF₃ | H | OMe | CH₂CN | O |
| C-4864 | Me(S) | single bond | H | CH₂CF₃ | H | OMe | CH₂CN | O |
| C-4865 | H | CH₂ | Me | CH₂CF₃ | H | OMe | CH₂CN | O |
| C-4866 | Me | CH₂ | H | CH₂CF₃ | H | OMe | CH₂CN | O |
| C-4867 | H | CH(Me) | H | CH₂CF₃ | H | OMe | CH₂CN | O |
| C-4868 | H | CH₂CH₂ | Me | CH₂CF₃ | H | OMe | CH₂CN | O |
| C-4869 | Me | CH₂CH₂ | H | CH₂CF₃ | H | OMe | CH₂CN | O |
| C-4870 | H | single bond | Me | Et | H | OMe | CH₂CN | O |
| C-4871 | Me | single bond | H | Et | H | OMe | CH₂CN | O |
| C-4872 | Me(R) | single bond | H | Et | H | OMe | CH₂CN | O |
| C-4873 | Me(S) | single bond | H | Et | H | OMe | CH₂CN | O |
| C-4874 | H | CH₂ | Me | Et | H | OMe | CH₂CN | O |
| C-4875 | Me | CH₂ | H | Et | H | OMe | CH₂CN | O |
| C-4876 | H | CH(Me) | H | Et | H | OMe | CH₂CN | O |
| C-4877 | H | CH₂CH₂ | Me | Et | H | OMe | CH₂CN | O |
| C-4878 | Me | CH₂CH₂ | H | Et | H | OMe | CH₂CN | O |

**[Table 338]**

| Compound Number | Y | W | Z | R¹ | R² | R³ | R¹⁰ | A |
|---|---|---|---|---|---|---|---|---|
| C-4879 | H | single bond | Me | c-Pr | H | OMe | CH₂CN | O |
| C-4880 | Me | single bond | H | c-Pr | H | OMe | CH₂CN | O |
| C-4881 | Me(R) | single bond | H | c-Pr | H | OMe | CH₂CN | O |
| C-4882 | Me(S) | single bond | H | c-Pr | H | OMe | CH₂CN | O |
| C-4883 | H | CH₂ | Me | c-Pr | H | OMe | CH₂CN | O |
| C-4884 | Me | CH₂ | H | c-Pr | H | OMe | CH₂CN | O |
| C-4885 | H | CH(Me) | H | c-Pr | H | OMe | CH₂CN | O |
| C-4886 | H | CH₂CH₂ | Me | c-Pr | H | OMe | CH₂CN | O |
| C-4887 | Me | CH₂CH₂ | H | c-Pr | H | OMe | CH₂CN | O |
| C-4888 | H | single bond | Me | c-Pr(1-CN) | H | OMe | CH₂CN | O |
| C-4889 | Me | single bond | H | c-Pr(1-CN) | H | OMe | CH₂CN | O |
| C-4890 | Me(R) | single bond | H | c-Pr(1-CN) | H | OMe | CH₂CN | O |
| C-4891 | Me(S) | single bond | H | c-Pr(1-CN) | H | OMe | CH₂CN | O |
| C-4892 | H | CH₂ | Me | c-Pr(1-CN) | H | OMe | CH₂CN | O |
| C-4893 | Me | CH₂ | H | c-Pr(1-CN) | H | OMe | CH₂CN | O |
| C-4894 | H | CH(Me) | H | c-Pr(1-CN) | H | OMe | CH₂CN | O |
| C-4895 | H | CH₂CH₂ | Me | c-Pr(1-CN) | H | OMe | CH₂CN | O |
| C-4896 | Me | CH₂CH₂ | H | c-Pr(1-CN) | H | OMe | CH₂CN | O |
| C-4897 | H | single bond | Me | CH₂CF₃ | H | OCF₃ | CH₂CN | O |
| C-4898 | Me | single bond | H | CH₂CF₃ | H | OCF₃ | CH₂CN | O |
| C-4899 | Me(R) | single bond | H | CH₂CF₃ | H | OCF₃ | CH₂CN | O |
| C-4900 | Me(S) | single bond | H | CH₂CF₃ | H | OCF₃ | CH₂CN | O |
| C-4901 | H | CH₂ | Me | CH₂CF₃ | H | OCF₃ | CH₂CN | O |
| C-4902 | Me | CH₂ | H | CH₂CF₃ | H | OCF₃ | CH₂CN | O |
| C-4903 | H | CH(Me) | H | CH₂CF₃ | H | OCF₃ | CH₂CN | O |
| C-4904 | H | CH₂CH₂ | Me | CH₂CF₃ | H | OCF₃ | CH₂CN | O |
| C-4905 | Me | CH₂CH₂ | H | CH₂CF₃ | H | OCF₃ | CH₂CN | O |
| C-4906 | H | single bond | Me | Et | H | OCF₃ | CH₂CN | O |
| C-4907 | Me | single bond | H | Et | H | OCF₃ | CH₂CN | O |
| C-4908 | Me(R) | single bond | H | Et | H | OCF₃ | CH₂CN | O |
| C-4909 | Me(S) | single bond | H | Et | H | OCF₃ | CH₂CN | O |
| C-4910 | H | CH₂ | Me | Et | H | OCF₃ | CH₂CN | O |
| C-4911 | Me | CH₂ | H | Et | H | OCF₃ | CH₂CN | O |
| C-4912 | H | CH(Me) | H | Et | H | OCF₃ | CH₂CN | O |
| C-4913 | H | CH₂CH₂ | Me | Et | H | OCF₃ | CH₂CN | O |
| C-4914 | Me | CH₂CH₂ | H | Et | H | OCF₃ | CH₂CN | O |
| C-4915 | H | single bond | Me | c-Pr | H | OCF₃ | CH₂CN | O |
| C-4916 | Me | single bond | H | c-Pr | H | OCF₃ | CH₂CN | O |
| C-4917 | Me(R) | single bond | H | c-Pr | H | OCF₃ | CH₂CN | O |
| C-4918 | Me(S) | single bond | H | c-Pr | H | OCF₃ | CH₂CN | O |
| C-4919 | H | CH₂ | Me | c-Pr | H | OCF₃ | CH₂CN | O |
| C-4920 | Me | CH₂ | H | c-Pr | H | OCF₃ | CH₂CN | O |
| C-4921 | H | CH(Me) | H | c-Pr | H | OCF₃ | CH₂CN | O |
| C-4922 | H | CH₂CH₂ | Me | c-Pr | H | OCF₃ | CH₂CN | O |

**[Table 339]**

| Compound Number | Y | W | Z | R¹ | R² | R³ | R¹⁰ | A |
|---|---|---|---|---|---|---|---|---|
| C-4923 | Me | CH₂CH₂ | H | c-Pr | H | OCF₃ | CH₂CN | O |
| C-4924 | H | single bond | Me | c-Pr(1-CN) | H | OCF₃ | CH₂CN | O |
| C-4925 | Me | single bond | H | c-Pr(1-CN) | H | OCF₃ | CH₂CN | O |
| C-4926 | Me(R) | single bond | H | c-Pr(1-CN) | H | OCF₃ | CH₂CN | O |
| C-4927 | Me(S) | single bond | H | c-Pr(1-CN) | H | OCF₃ | CH₂CN | O |
| C-4928 | H | CH₂ | Me | c-Pr(1-CN) | H | OCF₃ | CH₂CN | O |
| C-4929 | Me | CH₂ | H | c-Pr(1-CN) | H | OCF₃ | CH₂CN | O |
| C-4930 | H | CH(Me) | H | c-Pr(1-CN) | H | OCF₃ | CH₂CN | O |
| C-4931 | H | CH₂CH₂ | Me | c-Pr(1-CN) | H | OCF₃ | CH₂CN | O |
| C-4932 | Me | CH₂CH₂ | H | c-Pr(1-CN) | H | OCF₃ | CH₂CN | O |
| C-4933 | H | single bond | Me | CH₂CF₃ | Me | Br | H | O |
| C-4934 | Me | single bond | H | CH₂CF₃ | Me | Br | H | O |
| C-4935 | Me(R) | single bond | H | CH₂CF₃ | Me | Br | H | O |
| C-4936 | Me(S) | single bond | H | CH₂CF₃ | Me | Br | H | O |
| C-4937 | H | CH₂ | Me | CH₂CF₃ | Me | Br | H | O |
| C-4938 | Me | CH₂ | H | CH₂CF₃ | Me | Br | H | O |
| C-4939 | H | CH(Me) | H | CH₂CF₃ | Me | Br | H | O |
| C-4940 | H | CH₂CH₂ | Me | CH₂CF₃ | Me | Br | H | O |
| C-4941 | Me | CH₂CH₂ | H | CH₂CF₃ | Me | Br | H | O |
| C-4942 | H | single bond | Me | Et | Me | Br | H | O |
| C-4943 | Me | single bond | H | Et | Me | Br | H | O |
| C-4944 | Me(R) | single bond | H | Et | Me | Br | H | O |
| C-4945 | Me(S) | single bond | H | Et | Me | Br | H | O |
| C-4946 | H | CH₂ | Me | Et | Me | Br | H | O |
| C-4947 | Me | CH₂ | H | Et | Me | Br | H | O |
| C-4948 | H | CH(Me) | H | Et | Me | Br | H | O |
| C-4949 | H | CH₂CH₂ | Me | Et | Me | Br | H | O |
| C-4950 | Me | CH₂CH₂ | H | Et | Me | Br | H | O |
| C-4951 | H | single bond | Me | c-Pr | Me | Br | H | O |
| C-4952 | Me | single bond | H | c-Pr | Me | Br | H | O |
| C-4953 | Me(R) | single bond | H | c-Pr | Me | Br | H | O |
| C-4954 | Me(S) | single bond | H | c-Pr | Me | Br | H | O |
| C-4955 | H | CH₂ | Me | c-Pr | Me | Br | H | O |
| C-4956 | Me | CH₂ | H | c-Pr | Me | Br | H | O |
| C-4957 | H | CH(Me) | H | c-Pr | Me | Br | H | O |
| C-4958 | H | CH₂CH₂ | Me | c-Pr | Me | Br | H | O |
| C-4959 | Me | CH₂CH₂ | H | c-Pr | Me | Br | H | O |
| C-4960 | H | single bond | Me | c-Pr(1-CN) | Me | Br | H | O |
| C-4961 | Me | single bond | H | c-Pr(1-CN) | Me | Br | H | O |
| C-4962 | Me(R) | single bond | H | c-Pr(1-CN) | Me | Br | H | O |
| C-4963 | Me(S) | single bond | H | c-Pr(1-CN) | Me | Br | H | O |
| C-4964 | H | CH₂ | Me | c-Pr(1-CN) | Me | Br | H | O |
| C-4965 | Me | CH₂ | H | c-Pr(1-CN) | Me | Br | H | O |
| C-4966 | H | CH(Me) | H | c-Pr(1-CN) | Me | Br | H | O |

**[Table 340]**

| Compound Number | Y | W | Z | R¹ | R² | R³ | R¹⁰ | A |
|---|---|---|---|---|---|---|---|---|
| C-4967 | H | CH₂CH₂ | Me | c-Pr(1-CN) | Me | Br | H | O |
| C-4968 | Me | CH₂CH₂ | H | c-Pr(1-CN) | Me | Br | H | O |
| C-4969 | H | single bond | Me | CH₂CF₃ | Me | CF₃ | H | O |
| C-4970 | Me | single bond | H | CH₂CF₃ | Me | CF₃ | H | O |
| C-4971 | Me(R) | single bond | H | CH₂CF₃ | Me | CF₃ | H | O |
| C-4972 | Me(S) | single bond | H | CH₂CF₃ | Me | CF₃ | H | O |
| C-4973 | H | CH₂ | Me | CH₂CF₃ | Me | CF₃ | H | O |
| C-4974 | Me | CH₂ | H | CH₂CF₃ | Me | CF₃ | H | O |
| C-4975 | H | CH(Me) | H | CH₂CF₃ | Me | CF₃ | H | O |
| C-4976 | H | CH₂CH₂ | Me | CH₂CF₃ | Me | CF₃ | H | O |
| C-4977 | Me | CH₂CH₂ | H | CH₂CF₃ | Me | CF₃ | H | O |
| C-4978 | H | single bond | Me | Et | Me | CF₃ | H | O |
| C-4979 | Me | single bond | H | Et | Me | CF₃ | H | O |
| C-4980 | Me(R) | single bond | H | Et | Me | CF₃ | H | O |
| C-4981 | Me(S) | single bond | H | Et | Me | CF₃ | H | O |
| C-4982 | H | CH₂ | Me | Et | Me | CF₃ | H | O |
| C-4983 | Me | CH₂ | H | Et | Me | CF₃ | H | O |
| C-4984 | H | CH(Me) | H | Et | Me | CF₃ | H | O |
| C-4985 | H | CH₂CH₂ | Me | Et | Me | CF₃ | H | O |
| C-4986 | Me | CH₂CH₂ | H | Et | Me | CF₃ | H | O |
| C-4987 | H | single bond | Me | c-Pr | Me | CF₃ | H | O |
| C-4988 | Me | single bond | H | c-Pr | Me | CF₃ | H | O |
| C-4989 | Me(R) | single bond | H | c-Pr | Me | CF₃ | H | O |
| C-4990 | Me(S) | single bond | H | c-Pr | Me | CF₃ | H | O |
| C-4991 | H | CH₂ | Me | c-Pr | Me | CF₃ | H | O |
| C-4992 | Me | CH₂ | H | c-Pr | Me | CF₃ | H | O |
| C-4993 | H | CH(Me) | H | c-Pr | Me | CF₃ | H | O |
| C-4994 | H | CH₂CH₂ | Me | c-Pr | Me | CF₃ | H | O |
| C-4995 | Me | CH₂CH₂ | H | c-Pr | Me | CF₃ | H | O |
| C-4996 | H | single bond | Me | c-Pr(1-CN) | Me | CF₃ | H | O |
| C-4997 | Me | single bond | H | c-Pr(1-CN) | Me | CF₃ | H | O |
| C-4998 | Me(R) | single bond | H | c-Pr(1-CN) | Me | CF₃ | H | O |
| C-4999 | Me(S) | single bond | H | c-Pr(1-CN) | Me | CF₃ | H | O |
| C-5000 | H | CH₂ | Me | c-Pr(1-CN) | Me | CF₃ | H | O |
| C-5001 | Me | CH₂ | H | c-Pr(1-CN) | Me | CF₃ | H | O |
| C-5002 | H | CH(Me) | H | c-Pr(1-CN) | Me | CF₃ | H | O |
| C-5003 | H | CH₂CH₂ | Me | c-Pr(1-CN) | Me | CF₃ | H | O |
| C-5004 | Me | CH₂CH₂ | H | c-Pr(1-CN) | Me | CF₃ | H | O |
| C-5005 | H | single bond | Me | CH₂CF₃ | Me | OMe | H | O |
| C-5006 | Me | single bond | H | CH₂CF₃ | Me | OMe | H | O |
| C-5007 | Me(R) | single bond | H | CH₂CF₃ | Me | OMe | H | O |
| C-5008 | Me(S) | single bond | H | CH₂CF₃ | Me | OMe | H | O |
| C-5009 | H | CH₂ | Me | CH₂CF₃ | Me | OMe | H | O |
| C-5010 | Me | CH₂ | H | CH₂CF₃ | Me | OMe | H | O |

**[Table 341]**

| Compound Number | Y | W | Z | R¹ | R² | R³ | R¹⁰ | A |
|---|---|---|---|---|---|---|---|---|
| C-5011 | H | CH(Me) | H | CH₂CF₃ | Me | OMe | H | O |
| C-5012 | H | CH₂CH₂ | Me | CH₂CF₃ | Me | OMe | H | O |
| C-5013 | Me | CH₂CH₂ | H | CH₂CF₃ | Me | OMe | H | O |
| C-5014 | H | single bond | Me | Et | Me | OMe | H | O |
| C-5015 | Me | single bond | H | Et | Me | OMe | H | O |
| C-5016 | Me(R) | single bond | H | Et | Me | OMe | H | O |
| C-5017 | Me(S) | single bond | H | Et | Me | OMe | H | O |
| C-5018 | H | CH₂ | Me | Et | Me | OMe | H | O |
| C-5019 | Me | CH₂ | H | Et | Me | OMe | H | O |
| C-5020 | H | CH(Me) | H | Et | Me | OMe | H | O |
| C-5021 | H | CH₂CH₂ | Me | Et | Me | OMe | H | O |
| C-5022 | Me | CH₂CH₂ | H | Et | Me | OMe | H | O |
| C-5023 | H | single bond | Me | c-Pr | Me | OMe | H | O |
| C-5024 | Me | single bond | H | c-Pr | Me | OMe | H | O |
| C-5025 | Me(R) | single bond | H | c-Pr | Me | OMe | H | O |
| C-5026 | Me(S) | single bond | H | c-Pr | Me | OMe | H | O |
| C-5027 | H | CH₂ | Me | c-Pr | Me | OMe | H | O |
| C-5028 | Me | CH₂ | H | c-Pr | Me | OMe | H | O |
| C-5029 | H | CH(Me) | H | c-Pr | Me | OMe | H | O |
| C-5030 | H | CH₂CH₂ | Me | c-Pr | Me | OMe | H | O |
| C-5031 | Me | CH₂CH₂ | H | c-Pr | Me | OMe | H | O |
| C-5032 | H | single bond | Me | c-Pr(1-CN) | Me | OMe | H | O |
| C-5033 | Me | single bond | H | c-Pr(1-CN) | Me | OMe | H | O |
| C-5034 | Me(R) | single bond | H | c-Pr(1-CN) | Me | OMe | H | O |
| C-5035 | Me(S) | single bond | H | c-Pr(1-CN) | Me | OMe | H | O |
| C-5036 | H | CH₂ | Me | c-Pr(1-CN) | Me | OMe | H | O |
| C-5037 | Me | CH₂ | H | c-Pr(1-CN) | Me | OMe | H | O |
| C-5038 | H | CH(Me) | H | c-Pr(1-CN) | Me | OMe | H | O |
| C-5039 | H | CH₂CH₂ | Me | c-Pr(1-CN) | Me | OMe | H | O |
| C-5040 | Me | CH₂CH₂ | H | c-Pr(1-CN) | Me | OMe | H | O |
| C-5041 | H | single bond | Me | CH₂CF₃ | Me | OCF₃ | H | O |
| C-5042 | Me | single bond | H | CH₂CF₃ | Me | OCF₃ | H | O |
| C-5043 | Me(R) | single bond | H | CH₂CF₃ | Me | OCF₃ | H | O |
| C-5044 | Me(S) | single bond | H | CH₂CF₃ | Me | OCF₃ | H | O |
| C-5045 | H | CH₂ | Me | CH₂CF₃ | Me | OCF₃ | H | O |
| C-5046 | Me | CH₂ | H | CH₂CF₃ | Me | OCF₃ | H | O |
| C-5047 | H | CH(Me) | H | CH₂CF₃ | Me | OCF₃ | H | O |
| C-5048 | H | CH₂CH₂ | Me | CH₂CF₃ | Me | OCF₃ | H | O |
| C-5049 | Me | CH₂CH₂ | H | CH₂CF₃ | Me | OCF₃ | H | O |
| C-5050 | H | single bond | Me | Et | Me | OCF₃ | H | O |
| C-5051 | Me | single bond | H | Et | Me | OCF₃ | H | O |
| C-5052 | Me(R) | single bond | H | Et | Me | OCF₃ | H | O |
| C-5053 | Me(S) | single bond | H | Et | Me | OCF₃ | H | O |
| C-5054 | H | CH₂ | Me | Et | Me | OCF₃ | H | O |

**[Table 342]**

| Compound Number | Y | W | Z | R¹ | R² | R³ | R¹⁰ | A |
|---|---|---|---|---|---|---|---|---|
| C-5055 | Me | CH₂ | H | Et | Me | OCF₃ | H | O |
| C-5056 | H | CH(Me) | H | Et | Me | OCF₃ | H | O |
| C-5057 | H | CH₂CH₂ | Me | Et | Me | OCF₃ | H | O |
| C-5058 | Me | CH₂CH₂ | H | Et | Me | OCF₃ | H | O |
| C-5059 | H | single bond | Me | c-Pr | Me | OCF₃ | H | O |
| C-5060 | Me | single bond | H | c-Pr | Me | OCF₃ | H | O |
| C-5061 | Me(R) | single bond | H | c-Pr | Me | OCF₃ | H | O |
| C-5062 | Me(S) | single bond | H | c-Pr | Me | OCF₃ | H | O |
| C-5063 | H | CH₂ | Me | c-Pr | Me | OCF₃ | H | O |
| C-5064 | Me | CH₂ | H | c-Pr | Me | OCF₃ | H | O |
| C-5065 | H | CH(Me) | H | c-Pr | Me | OCF₃ | H | O |
| C-5066 | H | CH₂CH₂ | Me | c-Pr | Me | OCF₃ | H | O |
| C-5067 | Me | CH₂CH₂ | H | c-Pr | Me | OCF₃ | H | O |
| C-5068 | H | single bond | Me | c-Pr(1-CN) | Me | OCF₃ | H | O |
| C-5069 | Me | single bond | H | c-Pr(1-CN) | Me | OCF₃ | H | O |
| C-5070 | Me(R) | single bond | H | c-Pr(1-CN) | Me | OCF₃ | H | O |
| C-5071 | Me(S) | single bond | H | c-Pr(1-CN) | Me | OCF₃ | H | O |
| C-5072 | H | CH₂ | Me | c-Pr(1-CN) | Me | OCF₃ | H | O |
| C-5073 | Me | CH₂ | H | c-Pr(1-CN) | Me | OCF₃ | H | O |
| C-5074 | H | CH(Me) | H | c-Pr(1-CN) | Me | OCF₃ | H | O |
| C-5075 | H | CH₂CH₂ | Me | c-Pr(1-CN) | Me | OCF₃ | H | O |
| C-5076 | Me | CH₂CH₂ | H | c-Pr(1-CN) | Me | OCF₃ | H | O |
| C-5077 | H | single bond | Me | CH₂CF₃ | Me | Br | Me | O |
| C-5078 | Me | single bond | H | CH₂CF₃ | Me | Br | Me | O |
| C-5079 | Me(R) | single bond | H | CH₂CF₃ | Me | Br | Me | O |
| C-5080 | Me(S) | single bond | H | CH₂CF₃ | Me | Br | Me | O |
| C-5081 | H | CH₂ | Me | CH₂CF₃ | Me | Br | Me | O |
| C-5082 | Me | CH₂ | H | CH₂CF₃ | Me | Br | Me | O |
| C-5083 | H | CH(Me) | H | CH₂CF₃ | Me | Br | Me | O |
| C-5084 | H | CH₂CH₂ | Me | CH₂CF₃ | Me | Br | Me | O |
| C-5085 | Me | CH₂CH₂ | H | CH₂CF₃ | Me | Br | Me | O |
| C-5086 | H | single bond | Me | Et | Me | Br | Me | O |
| C-5087 | Me | single bond | H | Et | Me | Br | Me | O |
| C-5088 | Me(R) | single bond | H | Et | Me | Br | Me | O |
| C-5089 | Me(S) | single bond | H | Et | Me | Br | Me | O |
| C-5090 | H | CH₂ | Me | Et | Me | Br | Me | O |
| C-5091 | Me | CH₂ | H | Et | Me | Br | Me | O |
| C-5092 | H | CH(Me) | H | Et | Me | Br | Me | O |
| C-5093 | H | CH₂CH₂ | Me | Et | Me | Br | Me | O |
| C-5094 | Me | CH₂CH₂ | H | Et | Me | Br | Me | O |
| C-5095 | H | single bond | Me | c-Pr | Me | Br | Me | O |
| C-5096 | Me | single bond | H | c-Pr | Me | Br | Me | O |
| C-5097 | Me(R) | single bond | H | c-Pr | Me | Br | Me | O |
| C-5098 | Me(S) | single bond | H | c-Pr | Me | Br | Me | O |

**[Table 343]**

| Compound Number | Y | W | Z | R¹ | R² | R³ | R¹⁰ | A |
|---|---|---|---|---|---|---|---|---|
| C-5099 | H | CH₂ | Me | c-Pr | Me | Br | Me | O |
| C-5100 | Me | CH₂ | H | c-Pr | Me | Br | Me | O |
| C-5101 | H | CH(Me) | H | c-Pr | Me | Br | Me | O |
| C-5102 | H | CH₂CH₂ | Me | c-Pr | Me | Br | Me | O |
| C-5103 | Me | CH₂CH₂ | H | c-Pr | Me | Br | Me | O |
| C-5104 | H | single bond | Me | c-Pr(1-CN) | Me | Br | Me | O |
| C-5105 | Me | single bond | H | c-Pr(1-CN) | Me | Br | Me | O |
| C-5106 | Me(R) | single bond | H | c-Pr(1-CN) | Me | Br | Me | O |
| C-5107 | Me(S) | single bond | H | c-Pr(1-CN) | Me | Br | Me | O |
| C-5108 | H | CH₂ | Me | c-Pr(1-CN) | Me | Br | Me | O |
| C-5109 | Me | CH₂ | H | c-Pr(1-CN) | Me | Br | Me | O |
| C-5110 | H | CH(Me) | H | c-Pr(1-CN) | Me | Br | Me | O |
| C-5111 | H | CH₂CH₂ | Me | c-Pr(1-CN) | Me | Br | Me | O |
| C-5112 | Me | CH₂CH₂ | H | c-Pr(1-CN) | Me | Br | Me | O |
| C-5113 | H | single bond | Me | CH₂CF₃ | Me | CF₃ | Me | O |
| C-5114 | Me | single bond | H | CH₂CF₃ | Me | CF₃ | Me | O |
| C-5115 | Me(R) | single bond | H | CH₂CF₃ | Me | CF₃ | Me | O |
| C-5116 | Me(S) | single bond | H | CH₂CF₃ | Me | CF₃ | Me | O |
| C-5117 | H | CH₂ | Me | CH₂CF₃ | Me | CF₃ | Me | O |
| C-5118 | Me | CH₂ | H | CH₂CF₃ | Me | CF₃ | Me | O |
| C-5119 | H | CH(Me) | H | CH₂CF₃ | Me | CF₃ | Me | O |
| C-5120 | H | CH₂CH₂ | Me | CH₂CF₃ | Me | CF₃ | Me | O |
| C-5121 | Me | CH₂CH₂ | H | CH₂CF₃ | Me | CF₃ | Me | O |
| C-5122 | H | single bond | Me | Et | Me | CF₃ | Me | O |
| C-5123 | Me | single bond | H | Et | Me | CF₃ | Me | O |
| C-5124 | Me(R) | single bond | H | Et | Me | CF₃ | Me | O |
| C-5125 | Me(S) | single bond | H | Et | Me | CF₃ | Me | O |
| C-5126 | H | CH₂ | Me | Et | Me | CF₃ | Me | O |
| C-5127 | Me | CH₂ | H | Et | Me | CF₃ | Me | O |
| C-5128 | H | CH(Me) | H | Et | Me | CF₃ | Me | O |
| C-5129 | H | CH₂CH₂ | Me | Et | Me | CF₃ | Me | O |
| C-5130 | Me | CH₂CH₂ | H | Et | Me | CF₃ | Me | O |
| C-5131 | H | single bond | Me | c-Pr | Me | CF₃ | Me | O |
| C-5132 | Me | single bond | H | c-Pr | Me | CF₃ | Me | O |
| C-5133 | Me(R) | single bond | H | c-Pr | Me | CF₃ | Me | O |
| C-5134 | Me(S) | single bond | H | c-Pr | Me | CF₃ | Me | O |
| C-5135 | H | CH₂ | Me | c-Pr | Me | CF₃ | Me | O |
| C-5136 | Me | CH₂ | H | c-Pr | Me | CF₃ | Me | O |
| C-5137 | H | CH(Me) | H | c-Pr | Me | CF₃ | Me | O |
| C-5138 | H | CH₂CH₂ | Me | c-Pr | Me | CF₃ | Me | O |
| C-5139 | Me | CH₂CH₂ | H | c-Pr | Me | CF₃ | Me | O |
| C-5140 | H | single bond | Me | c-Pr(1-CN) | Me | CF₃ | Me | O |
| C-5141 | Me | single bond | H | c-Pr(1-CN) | Me | CF₃ | Me | O |
| C-5142 | Me(R) | single bond | H | c-Pr(1-CN) | Me | CF₃ | Me | O |

**[Table 344]**

| Compound Number | Y | W | Z | R¹ | R² | R³ | R¹⁰ | A |
|---|---|---|---|---|---|---|---|---|
| C-5143 | Me(S) | single bond | H | c-Pr(1-CN) | Me | CF₃ | Me | O |
| C-5144 | H | CH₂ | Me | c-Pr(1-CN) | Me | CF₃ | Me | O |
| C-5145 | Me | CH₂ | H | c-Pr(1-CN) | Me | CF₃ | Me | O |
| C-5146 | H | CH(Me) | H | c-Pr(1-CN) | Me | CF₃ | Me | O |
| C-5147 | H | CH₂CH₂ | Me | c-Pr(1-CN) | Me | CF₃ | Me | O |
| C-5148 | Me | CH₂CH₂ | H | c-Pr(1-CN) | Me | CF₃ | Me | O |
| C-5149 | H | single bond | Me | CH₂CF₃ | Me | OMe | Me | O |
| C-5150 | Me | single bond | H | CH₂CF₃ | Me | OMe | Me | O |
| C-5151 | Me(R) | single bond | H | CH₂CF₃ | Me | OMe | Me | O |
| C-5152 | Me(S) | single bond | H | CH₂CF₃ | Me | OMe | Me | O |
| C-5153 | H | CH₂ | Me | CH₂CF₃ | Me | OMe | Me | O |
| C-5154 | Me | CH₂ | H | CH₂CF₃ | Me | OMe | Me | O |
| C-5155 | H | CH(Me) | H | CH₂CF₃ | Me | OMe | Me | O |
| C-5156 | H | CH₂CH₂ | Me | CH₂CF₃ | Me | OMe | Me | O |
| C-5157 | Me | CH₂CH₂ | H | CH₂CF₃ | Me | OMe | Me | O |
| C-5158 | H | single bond | Me | Et | Me | OMe | Me | O |
| C-5159 | Me | single bond | H | Et | Me | OMe | Me | O |
| C-5160 | Me(R) | single bond | H | Et | Me | OMe | Me | O |
| C-5161 | Me(S) | single bond | H | Et | Me | OMe | Me | O |
| C-5162 | H | CH₂ | Me | Et | Me | OMe | Me | O |
| C-5163 | Me | CH₂ | H | Et | Me | OMe | Me | O |
| C-5164 | H | CH(Me) | H | Et | Me | OMe | Me | O |
| C-5165 | H | CH₂CH₂ | Me | Et | Me | OMe | Me | O |
| C-5166 | Me | CH₂CH₂ | H | Et | Me | OMe | Me | O |
| C-5167 | H | single bond | Me | c-Pr | Me | OMe | Me | O |
| C-5168 | Me | single bond | H | c-Pr | Me | OMe | Me | O |
| C-5169 | Me(R) | single bond | H | c-Pr | Me | OMe | Me | O |
| C-5170 | Me(S) | single bond | H | c-Pr | Me | OMe | Me | O |
| C-5171 | H | CH₂ | Me | c-Pr | Me | OMe | Me | O |
| C-5172 | Me | CH₂ | H | c-Pr | Me | OMe | Me | O |
| C-5173 | H | CH(Me) | H | c-Pr | Me | OMe | Me | O |
| C-5174 | H | CH₂CH₂ | Me | c-Pr | Me | OMe | Me | O |
| C-5175 | Me | CH₂CH₂ | H | c-Pr | Me | OMe | Me | O |
| C-5176 | H | single bond | Me | c-Pr(1-CN) | Me | OMe | Me | O |
| C-5177 | Me | single bond | H | c-Pr(1-CN) | Me | OMe | Me | O |
| C-5178 | Me(R) | single bond | H | c-Pr(1-CN) | Me | OMe | Me | O |
| C-5179 | Me(S) | single bond | H | c-Pr(1-CN) | Me | OMe | Me | O |
| C-5180 | H | CH₂ | Me | c-Pr(1-CN) | Me | OMe | Me | O |
| C-5181 | Me | CH₂ | H | c-Pr(1-CN) | Me | OMe | Me | O |
| C-5182 | H | CH(Me) | H | c-Pr(1-CN) | Me | OMe | Me | O |
| C-5183 | H | CH₂CH₂ | Me | c-Pr(1-CN) | Me | OMe | Me | O |
| C-5184 | Me | CH₂CH₂ | H | c-Pr(1-CN) | Me | OMe | Me | O |
| C-5185 | H | single bond | Me | CH₂CF₃ | Me | OCF₃ | Me | O |
| C-5186 | Me | single bond | H | CH₂CF₃ | Me | OCF₃ | Me | O |

**[Table 345]**

| Compound Number | Y | W | Z | R¹ | R² | R³ | R¹⁰ | A |
|---|---|---|---|---|---|---|---|---|
| C-5187 | Me(R) | single bond | H | CH₂CF₃ | Me | OCF₃ | Me | O |
| C-5188 | Me(S) | single bond | H | CH₂CF₃ | Me | OCF₃ | Me | O |
| C-5189 | H | CH₂ | Me | CH₂CF₃ | Me | OCF₃ | Me | O |
| C-5190 | Me | CH₂ | H | CH₂CF₃ | Me | OCF₃ | Me | O |
| C-5191 | H | CH(Me) | H | CH₂CF₃ | Me | OCF₃ | Me | O |
| C-5192 | H | CH₂CH₂ | Me | CH₂CF₃ | Me | OCF₃ | Me | O |
| C-5193 | Me | CH₂CH₂ | H | CH₂CF₃ | Me | OCF₃ | Me | O |
| C-5194 | H | single bond | Me | Et | Me | OCF₃ | Me | O |
| C-5195 | Me | single bond | H | Et | Me | OCF₃ | Me | O |
| C-5196 | Me(R) | single bond | H | Et | Me | OCF₃ | Me | O |
| C-5197 | Me(S) | single bond | H | Et | Me | OCF₃ | Me | O |
| C-5198 | H | CH₂ | Me | Et | Me | OCF₃ | Me | O |
| C-5199 | Me | CH₂ | H | Et | Me | OCF₃ | Me | O |
| C-5200 | H | CH(Me) | H | Et | Me | OCF₃ | Me | O |
| C-5201 | H | CH₂CH₂ | Me | Et | Me | OCF₃ | Me | O |
| C-5202 | Me | CH₂CH₂ | H | Et | Me | OCF₃ | Me | O |
| C-5203 | H | single bond | Me | c-Pr | Me | OCF₃ | Me | O |
| C-5204 | Me | single bond | H | c-Pr | Me | OCF₃ | Me | O |
| C-5205 | Me(R) | single bond | H | c-Pr | Me | OCF₃ | Me | O |
| C-5206 | Me(S) | single bond | H | c-Pr | Me | OCF₃ | Me | O |
| C-5207 | H | CH₂ | Me | c-Pr | Me | OCF₃ | Me | O |
| C-5208 | Me | CH₂ | H | c-Pr | Me | OCF₃ | Me | O |
| C-5209 | H | CH(Me) | H | c-Pr | Me | OCF₃ | Me | O |
| C-5210 | H | CH₂CH₂ | Me | c-Pr | Me | OCF₃ | Me | O |
| C-5211 | Me | CH₂CH₂ | H | c-Pr | Me | OCF₃ | Me | O |
| C-5212 | H | single bond | Me | c-Pr(1-CN) | Me | OCF₃ | Me | O |
| C-5213 | Me | single bond | H | c-Pr(1-CN) | Me | OCF₃ | Me | O |
| C-5214 | Me(R) | single bond | H | c-Pr(1-CN) | Me | OCF₃ | Me | O |
| C-5215 | Me(S) | single bond | H | c-Pr(1-CN) | Me | OCF₃ | Me | O |
| C-5216 | H | CH₂ | Me | c-Pr(1-CN) | Me | OCF₃ | Me | O |
| C-5217 | Me | CH₂ | H | c-Pr(1-CN) | Me | OCF₃ | Me | O |
| C-5218 | H | CH(Me) | H | c-Pr(1-CN) | Me | OCF₃ | Me | O |
| C-5219 | H | CH₂CH₂ | Me | c-Pr(1-CN) | Me | OCF₃ | Me | O |
| C-5220 | Me | CH₂CH₂ | H | c-Pr(1-CN) | Me | OCF₃ | Me | O |
| C-5221 | H | single bond | Me | CH₂CF₃ | Me | Br | CH₂OMe | O |
| C-5222 | Me | single bond | H | CH₂CF₃ | Me | Br | CH₂OMe | O |
| C-5223 | Me(R) | single bond | H | CH₂CF₃ | Me | Br | CH₂OMe | O |
| C-5224 | Me(S) | single bond | H | CH₂CF₃ | Me | Br | CH₂OMe | O |
| C-5225 | H | CH₂ | Me | CH₂CF₃ | Me | Br | CH₂OMe | O |
| C-5226 | Me | CH₂ | H | CH₂CF₃ | Me | Br | CH₂OMe | O |
| C-5227 | H | CH(Me) | H | CH₂CF₃ | Me | Br | CH₂OMe | O |
| C-5228 | H | CH₂CH₂ | Me | CH₂CF₃ | Me | Br | CH₂OMe | O |
| C-5229 | Me | CH₂CH₂ | H | CH₂CF₃ | Me | Br | CH₂OMe | O |
| C-5230 | H | single bond | Me | Et | Me | Br | CH₂OMe | O |

**[Table 346]**

| Compound Number | Y | W | Z | R¹ | R² | R³ | R¹⁰ | A |
|---|---|---|---|---|---|---|---|---|
| C-5231 | Me | single bond | H | Et | Me | Br | CH₂OMe | O |
| C-5232 | Me(R) | single bond | H | Et | Me | Br | CH₂OMe | O |
| C-5233 | Me(S) | single bond | H | Et | Me | Br | CH₂OMe | O |
| C-5234 | H | CH₂ | Me | Et | Me | Br | CH₂OMe | O |
| C-5235 | Me | CH₂ | H | Et | Me | Br | CH₂OMe | O |
| C-5236 | H | CH(Me) | H | Et | Me | Br | CH₂OMe | O |
| C-5237 | H | CH₂CH₂ | Me | Et | Me | Br | CH₂OMe | O |
| C-5238 | Me | CH₂CH₂ | H | Et | Me | Br | CH₂OMe | O |
| C-5239 | H | single bond | Me | c-Pr | Me | Br | CH₂OMe | O |
| C-5240 | Me | single bond | H | c-Pr | Me | Br | CH₂OMe | O |
| C-5241 | Me(R) | single bond | H | c-Pr | Me | Br | CH₂OMe | O |
| C-5242 | Me(S) | single bond | H | c-Pr | Me | Br | CH₂OMe | O |
| C-5243 | H | CH₂ | Me | c-Pr | Me | Br | CH₂OMe | O |
| C-5244 | Me | CH₂ | H | c-Pr | Me | Br | CH₂OMe | O |
| C-5245 | H | CH(Me) | H | c-Pr | Me | Br | CH₂OMe | O |
| C-5246 | H | CH₂CH₂ | Me | c-Pr | Me | Br | CH₂OMe | O |
| C-5247 | Me | CH₂CH₂ | H | c-Pr | Me | Br | CH₂OMe | O |
| C-5248 | H | single bond | Me | c-Pr(1-CN) | Me | Br | CH₂OMe | O |
| C-5249 | Me | single bond | H | c-Pr(1-CN) | Me | Br | CH₂OMe | O |
| C-5250 | Me(R) | single bond | H | c-Pr(1-CN) | Me | Br | CH₂OMe | O |
| C-5251 | Me(S) | single bond | H | c-Pr(1-CN) | Me | Br | CH₂OMe | O |
| C-5252 | H | CH₂ | Me | c-Pr(1-CN) | Me | Br | CH₂OMe | O |
| C-5253 | Me | CH₂ | H | c-Pr(1-CN) | Me | Br | CH₂OMe | O |
| C-5254 | H | CH(Me) | H | c-Pr(1-CN) | Me | Br | CH₂OMe | O |
| C-5255 | H | CH₂CH₂ | Me | c-Pr(1-CN) | Me | Br | CH₂OMe | O |
| C-5256 | Me | CH₂CH₂ | H | c-Pr(1-CN) | Me | Br | CH₂OMe | O |
| C-5257 | H | single bond | Me | CH₂CF₃ | Me | CF₃ | CH₂OMe | O |
| C-5258 | Me | single bond | H | CH₂CF₃ | Me | CF₃ | CH₂OMe | O |
| C-5259 | Me(R) | single bond | H | CH₂CF₃ | Me | CF₃ | CH₂OMe | O |
| C-5260 | Me(S) | single bond | H | CH₂CF₃ | Me | CF₃ | CH₂OMe | O |
| C-5261 | H | CH₂ | Me | CH₂CF₃ | Me | CF₃ | CH₂OMe | O |
| C-5262 | Me | CH₂ | H | CH₂CF₃ | Me | CF₃ | CH₂OMe | O |
| C-5263 | H | CH(Me) | H | CH₂CF₃ | Me | CF₃ | CH₂OMe | O |
| C-5264 | H | CH₂CH₂ | Me | CH₂CF₃ | Me | CF₃ | CH₂OMe | O |
| C-5265 | Me | CH₂CH₂ | H | CH₂CF₃ | Me | CF₃ | CH₂OMe | O |
| C-5266 | H | single bond | Me | Et | Me | CF₃ | CH₂OMe | O |
| C-5267 | Me | single bond | H | Et | Me | CF₃ | CH₂OMe | O |
| C-5268 | Me(R) | single bond | H | Et | Me | CF₃ | CH₂OMe | O |
| C-5269 | Me(S) | single bond | H | Et | Me | CF₃ | CH₂OMe | O |
| C-5270 | H | CH₂ | Me | Et | Me | CF₃ | CH₂OMe | O |
| C-5271 | Me | CH₂ | H | Et | Me | CF₃ | CH₂OMe | O |
| C-5272 | H | CH(Me) | H | Et | Me | CF₃ | CH₂OMe | O |
| C-5273 | H | CH₂CH₂ | Me | Et | Me | CF₃ | CH₂OMe | O |
| C-5274 | Me | CH₂CH₂ | H | Et | Me | CF₃ | CH₂OMe | O |

**[Table 347]**

| Compound Number | Y | W | Z | R¹ | R² | R³ | R¹⁰ | A |
|---|---|---|---|---|---|---|---|---|
| C-5275 | H | single bond | Me | c-Pr | Me | CF₃ | CH₂OMe | O |
| C-5276 | Me | single bond | H | c-Pr | Me | CF₃ | CH₂OMe | O |
| C-5277 | Me(R) | single bond | H | c-Pr | Me | CF₃ | CH₂OMe | O |
| C-5278 | Me(S) | single bond | H | c-Pr | Me | CF₃ | CH₂OMe | O |
| C-5279 | H | CH₂ | Me | c-Pr | Me | CF₃ | CH₂OMe | O |
| C-5280 | Me | CH₂ | H | c-Pr | Me | CF₃ | CH₂OMe | O |
| C-5281 | H | CH(Me) | H | c-Pr | Me | CF₃ | CH₂OMe | O |
| C-5282 | H | CH₂CH₂ | Me | c-Pr | Me | CF₃ | CH₂OMe | O |
| C-5283 | Me | CH₂CH₂ | H | c-Pr | Me | CF₃ | CH₂OMe | O |
| C-5284 | H | single bond | Me | c-Pr(1-CN) | Me | CF₃ | CH₂OMe | O |
| C-5285 | Me | single bond | H | c-Pr(1-CN) | Me | CF₃ | CH₂OMe | O |
| C-5286 | Me(R) | single bond | H | c-Pr(1-CN) | Me | CF₃ | CH₂OMe | O |
| C-5287 | Me(S) | single bond | H | c-Pr(1-CN) | Me | CF₃ | CH₂OMe | O |
| C-5288 | H | CH₂ | Me | c-Pr(1-CN) | Me | CF₃ | CH₂OMe | O |
| C-5289 | Me | CH₂ | H | c-Pr(1-CN) | Me | CF₃ | CH₂OMe | O |
| C-5290 | H | CH(Me) | H | c-Pr(1-CN) | Me | CF₃ | CH₂OMe | O |
| C-5291 | H | CH₂CH₂ | Me | c-Pr(1-CN) | Me | CF₃ | CH₂OMe | O |
| C-5292 | Me | CH₂CH₂ | H | c-Pr(1-CN) | Me | CF₃ | CH₂OMe | O |
| C-5293 | H | single bond | Me | CH₂CF₃ | Me | OMe | CH₂OMe | O |
| C-5294 | Me | single bond | H | CH₂CF₃ | Me | OMe | CH₂OMe | O |
| C-5295 | Me(R) | single bond | H | CH₂CF₃ | Me | OMe | CH₂OMe | O |
| C-5296 | Me(S) | single bond | H | CH₂CF₃ | Me | OMe | CH₂OMe | O |
| C-5297 | H | CH₂ | Me | CH₂CF₃ | Me | OMe | CH₂OMe | O |
| C-5298 | Me | CH₂ | H | CH₂CF₃ | Me | OMe | CH₂OMe | O |
| C-5299 | H | CH(Me) | H | CH₂CF₃ | Me | OMe | CH₂OMe | O |
| C-5300 | H | CH₂CH₂ | Me | CH₂CF₃ | Me | OMe | CH₂OMe | O |
| C-5301 | Me | CH₂CH₂ | H | CH₂CF₃ | Me | OMe | CH₂OMe | O |
| C-5302 | H | single bond | Me | Et | Me | OMe | CH₂OMe | O |
| C-5303 | Me | single bond | H | Et | Me | OMe | CH₂OMe | O |
| C-5304 | Me(R) | single bond | H | Et | Me | OMe | CH₂OMe | O |
| C-5305 | Me(S) | single bond | H | Et | Me | OMe | CH₂OMe | O |
| C-5306 | H | CH₂ | Me | Et | Me | OMe | CH₂OMe | O |
| C-5307 | Me | CH₂ | H | Et | Me | OMe | CH₂OMe | O |
| C-5308 | H | CH(Me) | H | Et | Me | OMe | CH₂OMe | O |
| C-5309 | H | CH₂CH₂ | Me | Et | Me | OMe | CH₂OMe | O |
| C-5310 | Me | CH₂CH₂ | H | Et | Me | OMe | CH₂OMe | O |
| C-5311 | H | single bond | Me | c-Pr | Me | OMe | CH₂OMe | O |
| C-5312 | Me | single bond | H | c-Pr | Me | OMe | CH₂OMe | O |
| C-5313 | Me(R) | single bond | H | c-Pr | Me | OMe | CH₂OMe | O |
| C-5314 | Me(S) | single bond | H | c-Pr | Me | OMe | CH₂OMe | O |
| C-5315 | H | CH₂ | Me | c-Pr | Me | OMe | CH₂OMe | O |
| C-5316 | Me | CH₂ | H | c-Pr | Me | OMe | CH₂OMe | O |
| C-5317 | H | CH(Me) | H | c-Pr | Me | OMe | CH₂OMe | O |
| C-5318 | H | CH₂CH₂ | Me | c-Pr | Me | OMe | CH₂OMe | O |

**[Table 348]**

| Compound Number | Y | W | Z | R¹ | R² | R³ | R¹⁰ | A |
|---|---|---|---|---|---|---|---|---|
| C-5319 | Me | CH₂CH₂ | H | c-Pr | Me | OMe | CH₂OMe | O |
| C-5320 | H | single bond | Me | c-Pr(1-CN) | Me | OMe | CH₂OMe | O |
| C-5321 | Me | single bond | H | c-Pr(1-CN) | Me | OMe | CH₂OMe | O |
| C-5322 | Me(R) | single bond | H | c-Pr(1-CN) | Me | OMe | CH₂OMe | O |
| C-5323 | Me(S) | single bond | H | c-Pr(1-CN) | Me | OMe | CH₂OMe | O |
| C-5324 | H | CH₂ | Me | c-Pr(1-CN) | Me | OMe | CH₂OMe | O |
| C-5325 | Me | CH₂ | H | c-Pr(1-CN) | Me | OMe | CH₂OMe | O |
| C-5326 | H | CH(Me) | H | c-Pr(1-CN) | Me | OMe | CH₂OMe | O |
| C-5327 | H | CH₂CH₂ | Me | c-Pr(1-CN) | Me | OMe | CH₂OMe | O |
| C-5328 | Me | CH₂CH₂ | H | c-Pr(1-CN) | Me | OMe | CH₂OMe | O |
| C-5329 | H | single bond | Me | CH₂CF₃ | Me | OCF₃ | CH₂OMe | O |
| C-5330 | Me | single bond | H | CH₂CF₃ | Me | OCF₃ | CH₂OMe | O |
| C-5331 | Me(R) | single bond | H | CH₂CF₃ | Me | OCF₃ | CH₂OMe | O |
| C-5332 | Me(S) | single bond | H | CH₂CF₃ | Me | OCF₃ | CH₂OMe | O |
| C-5333 | H | CH₂ | Me | CH₂CF₃ | Me | OCF₃ | CH₂OMe | O |
| C-5334 | Me | CH₂ | H | CH₂CF₃ | Me | OCF₃ | CH₂OMe | O |
| C-5335 | H | CH(Me) | H | CH₂CF₃ | Me | OCF₃ | CH₂OMe | O |
| C-5336 | H | CH₂CH₂ | Me | CH₂CF₃ | Me | OCF₃ | CH₂OMe | O |
| C-5337 | Me | CH₂CH₂ | H | CH₂CF₃ | Me | OCF₃ | CH₂OMe | O |
| C-5338 | H | single bond | Me | Et | Me | OCF₃ | CH₂OMe | O |
| C-5339 | Me | single bond | H | Et | Me | OCF₃ | CH₂OMe | O |
| C-5340 | Me(R) | single bond | H | Et | Me | OCF₃ | CH₂OMe | O |
| C-5341 | Me(S) | single bond | H | Et | Me | OCF₃ | CH₂OMe | O |
| C-5342 | H | CH₂ | Me | Et | Me | OCF₃ | CH₂OMe | O |
| C-5343 | Me | CH₂ | H | Et | Me | OCF₃ | CH₂OMe | O |
| C-5344 | H | CH(Me) | H | Et | Me | OCF₃ | CH₂OMe | O |
| C-5345 | H | CH₂CH₂ | Me | Et | Me | OCF₃ | CH₂OMe | O |
| C-5346 | Me | CH₂CH₂ | H | Et | Me | OCF₃ | CH₂OMe | O |
| C-5347 | H | single bond | Me | c-Pr | Me | OCF₃ | CH₂OMe | O |
| C-5348 | Me | single bond | H | c-Pr | Me | OCF₃ | CH₂OMe | O |
| C-5349 | Me(R) | single bond | H | c-Pr | Me | OCF₃ | CH₂OMe | O |
| C-5350 | Me(S) | single bond | H | c-Pr | Me | OCF₃ | CH₂OMe | O |
| C-5351 | H | CH₂ | Me | c-Pr | Me | OCF₃ | CH₂OMe | O |
| C-5352 | Me | CH₂ | H | c-Pr | Me | OCF₃ | CH₂OMe | O |
| C-5353 | H | CH(Me) | H | c-Pr | Me | OCF₃ | CH₂OMe | O |
| C-5354 | H | CH₂CH₂ | Me | c-Pr | Me | OCF₃ | CH₂OMe | O |
| C-5355 | Me | CH₂CH₂ | H | c-Pr | Me | OCF₃ | CH₂OMe | O |
| C-5356 | H | single bond | Me | c-Pr(1-CN) | Me | OCF₃ | CH₂OMe | O |
| C-5357 | Me | single bond | H | c-Pr(1-CN) | Me | OCF₃ | CH₂OMe | O |
| C-5358 | Me(R) | single bond | H | c-Pr(1-CN) | Me | OCF₃ | CH₂OMe | O |
| C-5359 | Me(S) | single bond | H | c-Pr(1-CN) | Me | OCF₃ | CH₂OMe | O |
| C-5360 | H | CH₂ | Me | c-Pr(1-CN) | Me | OCF₃ | CH₂OMe | O |
| C-5361 | Me | CH₂ | H | c-Pr(1-CN) | Me | OCF₃ | CH₂OMe | O |
| C-5362 | H | CH(Me) | H | c-Pr(1-CN) | Me | OCF₃ | CH₂OMe | O |

**[Table 349]**

| Compound Number | Y | W | Z | R¹ | R² | R³ | R¹⁰ | A |
|---|---|---|---|---|---|---|---|---|
| C-5363 | H | CH₂CH₂ | Me | c-Pr(1-CN) | Me | OCF₃ | CH₂OMe | O |
| C-5364 | Me | CH₂CH₂ | H | c-Pr(1-CN) | Me | OCF₃ | CH₂OMe | O |
| C-5365 | H | single bond | Me | CH₂CF₃ | Me | Br | CH₂CN | O |
| C-5366 | Me | single bond | H | CH₂CF₃ | Me | Br | CH₂CN | O |
| C-5367 | Me(R) | single bond | H | CH₂CF₃ | Me | Br | CH₂CN | O |
| C-5368 | Me(S) | single bond | H | CH₂CF₃ | Me | Br | CH₂CN | O |
| C-5369 | H | CH₂ | Me | CH₂CF₃ | Me | Br | CH₂CN | O |
| C-5370 | Me | CH₂ | H | CH₂CF₃ | Me | Br | CH₂CN | O |
| C-5371 | H | CH(Me) | H | CH₂CF₃ | Me | Br | CH₂CN | O |
| C-5372 | H | CH₂CH₂ | Me | CH₂CF₃ | Me | Br | CH₂CN | O |
| C-5373 | Me | CH₂CH₂ | H | CH₂CF₃ | Me | Br | CH₂CN | O |
| C-5374 | H | single bond | Me | Et | Me | Br | CH₂CN | O |
| C-5375 | Me | single bond | H | Et | Me | Br | CH₂CN | O |
| C-5376 | Me(R) | single bond | H | Et | Me | Br | CH₂CN | O |
| C-5377 | Me(S) | single bond | H | Et | Me | Br | CH₂CN | O |
| C-5378 | H | CH₂ | Me | Et | Me | Br | CH₂CN | O |
| C-5379 | Me | CH₂ | H | Et | Me | Br | CH₂CN | O |
| C-5380 | H | CH(Me) | H | Et | Me | Br | CH₂CN | O |
| C-5381 | H | CH₂CH₂ | Me | Et | Me | Br | CH₂CN | O |
| C-5382 | Me | CH₂CH₂ | H | Et | Me | Br | CH₂CN | O |
| C-5383 | H | single bond | Me | c-Pr | Me | Br | CH₂CN | O |
| C-5384 | Me | single bond | H | c-Pr | Me | Br | CH₂CN | O |
| C-5385 | Me(R) | single bond | H | c-Pr | Me | Br | CH₂CN | O |
| C-5386 | Me(S) | single bond | H | c-Pr | Me | Br | CH₂CN | O |
| C-5387 | H | CH₂ | Me | c-Pr | Me | Br | CH₂CN | O |
| C-5388 | Me | CH₂ | H | c-Pr | Me | Br | CH₂CN | O |
| C-5389 | H | CH(Me) | H | c-Pr | Me | Br | CH₂CN | O |
| C-5390 | H | CH₂CH₂ | Me | c-Pr | Me | Br | CH₂CN | O |
| C-5391 | Me | CH₂CH₂ | H | c-Pr | Me | Br | CH₂CN | O |
| C-5392 | H | single bond | Me | c-Pr(1-CN) | Me | Br | CH₂CN | O |
| C-5393 | Me | single bond | H | c-Pr(1-CN) | Me | Br | CH₂CN | O |
| C-5394 | Me(R) | single bond | H | c-Pr(1-CN) | Me | Br | CH₂CN | O |
| C-5395 | Me(S) | single bond | H | c-Pr(1-CN) | Me | Br | CH₂CN | O |
| C-5396 | H | CH₂ | Me | c-Pr(1-CN) | Me | Br | CH₂CN | O |
| C-5397 | Me | CH₂ | H | c-Pr(1-CN) | Me | Br | CH₂CN | O |
| C-5398 | H | CH(Me) | H | c-Pr(1-CN) | Me | Br | CH₂CN | O |
| C-5399 | H | CH₂CH₂ | Me | c-Pr(1-CN) | Me | Br | CH₂CN | O |
| C-5400 | Me | CH₂CH₂ | H | c-Pr(1-CN) | Me | Br | CH₂CN | O |
| C-5401 | H | single bond | Me | CH₂CF₃ | Me | CF₃ | CH₂CN | O |
| C-5402 | Me | single bond | H | CH₂CF₃ | Me | CF₃ | CH₂CN | O |
| C-5403 | Me(R) | single bond | H | CH₂CF₃ | Me | CF₃ | CH₂CN | O |
| C-5404 | Me(S) | single bond | H | CH₂CF₃ | Me | CF₃ | CH₂CN | O |
| C-5405 | H | CH₂ | Me | CH₂CF₃ | Me | CF₃ | CH₂CN | O |
| C-5406 | Me | CH₂ | H | CH₂CF₃ | Me | CF₃ | CH₂CN | O |

**[Table 350]**

| Compound Number | Y | W | Z | R¹ | R² | R³ | R¹⁰ | A |
|---|---|---|---|---|---|---|---|---|
| C-5407 | H | CH(Me) | H | CH₂CF₃ | Me | CF₃ | CH₂CN | O |
| C-5408 | H | CH₂CH₂ | Me | CH₂CF₃ | Me | CF₃ | CH₂CN | O |
| C-5409 | Me | CH₂CH₂ | H | CH₂CF₃ | Me | CF₃ | CH₂CN | O |
| C-5410 | H | single bond | Me | Et | Me | CF₃ | CH₂CN | O |
| C-5411 | Me | single bond | H | Et | Me | CF₃ | CH₂CN | O |
| C-5412 | Me(R) | single bond | H | Et | Me | CF₃ | CH₂CN | O |
| C-5413 | Me(S) | single bond | H | Et | Me | CF₃ | CH₂CN | O |
| C-5414 | H | CH₂ | Me | Et | Me | CF₃ | CH₂CN | O |
| C-5415 | Me | CH₂ | H | Et | Me | CF₃ | CH₂CN | O |
| C-5416 | H | CH(Me) | H | Et | Me | CF₃ | CH₂CN | O |
| C-5417 | H | CH₂CH₂ | Me | Et | Me | CF₃ | CH₂CN | O |
| C-5418 | Me | CH₂CH₂ | H | Et | Me | CF₃ | CH₂CN | O |
| C-5419 | H | single bond | Me | c-Pr | Me | CF₃ | CH₂CN | O |
| C-5420 | Me | single bond | H | c-Pr | Me | CF₃ | CH₂CN | O |
| C-5421 | Me(R) | single bond | H | c-Pr | Me | CF₃ | CH₂CN | O |
| C-5422 | Me(S) | single bond | H | c-Pr | Me | CF₃ | CH₂CN | O |
| C-5423 | H | CH₂ | Me | c-Pr | Me | CF₃ | CH₂CN | O |
| C-5424 | Me | CH₂ | H | c-Pr | Me | CF₃ | CH₂CN | O |
| C-5425 | H | CH(Me) | H | c-Pr | Me | CF₃ | CH₂CN | O |
| C-5426 | H | CH₂CH₂ | Me | c-Pr | Me | CF₃ | CH₂CN | O |
| C-5427 | Me | CH₂CH₂ | H | c-Pr | Me | CF₃ | CH₂CN | O |
| C-5428 | H | single bond | Me | c-Pr(1-CN) | Me | CF₃ | CH₂CN | O |
| C-5429 | Me | single bond | H | c-Pr(1-CN) | Me | CF₃ | CH₂CN | O |
| C-5430 | Me(R) | single bond | H | c-Pr(1-CN) | Me | CF₃ | CH₂CN | O |
| C-5431 | Me(S) | single bond | H | c-Pr(1-CN) | Me | CF₃ | CH₂CN | O |
| C-5432 | H | CH₂ | Me | c-Pr(1-CN) | Me | CF₃ | CH₂CN | O |
| C-5433 | Me | CH₂ | H | c-Pr(1-CN) | Me | CF₃ | CH₂CN | O |
| C-5434 | H | CH(Me) | H | c-Pr(1-CN) | Me | CF₃ | CH₂CN | O |
| C-5435 | H | CH₂CH₂ | Me | c-Pr(1-CN) | Me | CF₃ | CH₂CN | O |
| C-5436 | Me | CH₂CH₂ | H | c-Pr(1-CN) | Me | CF₃ | CH₂CN | O |
| C-5437 | H | single bond | Me | CH₂CF₃ | Me | OMe | CH₂CN | O |
| C-5438 | Me | single bond | H | CH₂CF₃ | Me | OMe | CH₂CN | O |
| C-5439 | Me(R) | single bond | H | CH₂CF₃ | Me | OMe | CH₂CN | O |
| C-5440 | Me(S) | single bond | H | CH₂CF₃ | Me | OMe | CH₂CN | O |
| C-5441 | H | CH₂ | Me | CH₂CF₃ | Me | OMe | CH₂CN | O |
| C-5442 | Me | CH₂ | H | CH₂CF₃ | Me | OMe | CH₂CN | O |
| C-5443 | H | CH(Me) | H | CH₂CF₃ | Me | OMe | CH₂CN | O |
| C-5444 | H | CH₂CH₂ | Me | CH₂CF₃ | Me | OMe | CH₂CN | O |
| C-5445 | Me | CH₂CH₂ | H | CH₂CF₃ | Me | OMe | CH₂CN | O |
| C-5446 | H | single bond | Me | Et | Me | OMe | CH₂CN | O |
| C-5447 | Me | single bond | H | Et | Me | OMe | CH₂CN | O |
| C-5448 | Me(R) | single bond | H | Et | Me | OMe | CH₂CN | O |
| C-5449 | Me(S) | single bond | H | Et | Me | OMe | CH₂CN | O |
| C-5450 | H | CH₂ | Me | Et | Me | OMe | CH₂CN | O |

**[Table 351]**

| Compound Number | Y | W | Z | R¹ | R² | R³ | R¹⁰ | A |
|---|---|---|---|---|---|---|---|---|
| C-5451 | Me | CH₂ | H | Et | Me | OMe | CH₂CN | O |
| C-5452 | H | CH(Me) | H | Et | Me | OMe | CH₂CN | O |
| C-5453 | H | CH₂CH₂ | Me | Et | Me | OMe | CH₂CN | O |
| C-5454 | Me | CH₂CH₂ | H | Et | Me | OMe | CH₂CN | O |
| C-5455 | H | single bond | Me | c-Pr | Me | OMe | CH₂CN | O |
| C-5456 | Me | single bond | H | c-Pr | Me | OMe | CH₂CN | O |
| C-5457 | Me(R) | single bond | H | c-Pr | Me | OMe | CH₂CN | O |
| C-5458 | Me(S) | single bond | H | c-Pr | Me | OMe | CH₂CN | O |
| C-5459 | H | CH₂ | Me | c-Pr | Me | OMe | CH₂CN | O |
| C-5460 | Me | CH₂ | H | c-Pr | Me | OMe | CH₂CN | O |
| C-5461 | H | CH(Me) | H | c-Pr | Me | OMe | CH₂CN | O |
| C-5462 | H | CH₂CH₂ | Me | c-Pr | Me | OMe | CH₂CN | O |
| C-5463 | Me | CH₂CH₂ | H | c-Pr | Me | OMe | CH₂CN | O |
| C-5464 | H | single bond | Me | c-Pr(1-CN) | Me | OMe | CH₂CN | O |
| C-5465 | Me | single bond | H | c-Pr(1-CN) | Me | OMe | CH₂CN | O |
| C-5466 | Me(R) | single bond | H | c-Pr(1-CN) | Me | OMe | CH₂CN | O |
| C-5467 | Me(S) | single bond | H | c-Pr(1-CN) | Me | OMe | CH₂CN | O |
| C-5468 | H | CH₂ | Me | c-Pr(1-CN) | Me | OMe | CH₂CN | O |
| C-5469 | Me | CH₂ | H | c-Pr(1-CN) | Me | OMe | CH₂CN | O |
| C-5470 | H | CH(Me) | H | c-Pr(1-CN) | Me | OMe | CH₂CN | O |
| C-5471 | H | CH₂CH₂ | Me | c-Pr(1-CN) | Me | OMe | CH₂CN | O |
| C-5472 | Me | CH₂CH₂ | H | c-Pr(1-CN) | Me | OMe | CH₂CN | O |
| C-5473 | H | single bond | Me | CH₂CF₃ | Me | OCF₃ | CH₂CN | O |
| C-5474 | Me | single bond | H | CH₂CF₃ | Me | OCF₃ | CH₂CN | O |
| C-5475 | Me(R) | single bond | H | CH₂CF₃ | Me | OCF₃ | CH₂CN | O |
| C-5476 | Me(S) | single bond | H | CH₂CF₃ | Me | OCF₃ | CH₂CN | O |
| C-5477 | H | CH₂ | Me | CH₂CF₃ | Me | OCF₃ | CH₂CN | O |
| C-5478 | Me | CH₂ | H | CH₂CF₃ | Me | OCF₃ | CH₂CN | O |
| C-5479 | H | CH(Me) | H | CH₂CF₃ | Me | OCF₃ | CH₂CN | O |
| C-5480 | H | CH₂CH₂ | Me | CH₂CF₃ | Me | OCF₃ | CH₂CN | O |
| C-5481 | Me | CH₂CH₂ | H | CH₂CF₃ | Me | OCF₃ | CH₂CN | O |
| C-5482 | H | single bond | Me | Et | Me | OCF₃ | CH₂CN | O |
| C-5483 | Me | single bond | H | Et | Me | OCF₃ | CH₂CN | O |
| C-5484 | Me(R) | single bond | H | Et | Me | OCF₃ | CH₂CN | O |
| C-5485 | Me(S) | single bond | H | Et | Me | OCF₃ | CH₂CN | O |
| C-5486 | H | CH₂ | Me | Et | Me | OCF₃ | CH₂CN | O |
| C-5487 | Me | CH₂ | H | Et | Me | OCF₃ | CH₂CN | O |
| C-5488 | H | CH(Me) | H | Et | Me | OCF₃ | CH₂CN | O |
| C-5489 | H | CH₂CH₂ | Me | Et | Me | OCF₃ | CH₂CN | O |
| C-5490 | Me | CH₂CH₂ | H | Et | Me | OCF₃ | CH₂CN | O |
| C-5491 | H | single bond | Me | c-Pr | Me | OCF₃ | CH₂CN | O |
| C-5492 | Me | single bond | H | c-Pr | Me | OCF₃ | CH₂CN | O |
| C-5493 | Me(R) | single bond | H | c-Pr | Me | OCF₃ | CH₂CN | O |
| C-5494 | Me(S) | single bond | H | c-Pr | Me | OCF₃ | CH₂CN | O |

**[Table 352]**

| Compound Number | Y | W | Z | R¹ | R² | R³ | R¹⁰ | A |
|---|---|---|---|---|---|---|---|---|
| C-5495 | H | CH₂ | Me | c-Pr | Me | OCF₃ | CH₂CN | O |
| C-5496 | Me | CH₂ | H | c-Pr | Me | OCF₃ | CH₂CN | O |
| C-5497 | H | CH(Me) | H | c-Pr | Me | OCF₃ | CH₂CN | O |
| C-5498 | H | CH₂CH₂ | Me | c-Pr | Me | OCF₃ | CH₂CN | O |
| C-5499 | Me | CH₂CH₂ | H | c-Pr | Me | OCF₃ | CH₂CN | O |
| C-5500 | H | single bond | Me | c-Pr(1-CN) | Me | OCF₃ | CH₂CN | O |
| C-5501 | Me | single bond | H | c-Pr(1-CN) | Me | OCF₃ | CH₂CN | O |
| C-5502 | Me(R) | single bond | H | c-Pr(1-CN) | Me | OCF₃ | CH₂CN | O |
| C-5503 | Me(S) | single bond | H | c-Pr(1-CN) | Me | OCF₃ | CH₂CN | O |
| C-5504 | H | CH₂ | Me | c-Pr(1-CN) | Me | OCF₃ | CH₂CN | O |
| C-5505 | Me | CH₂ | H | c-Pr(1-CN) | Me | OCF₃ | CH₂CN | O |
| C-5506 | H | CH(Me) | H | c-Pr(1-CN) | Me | OCF₃ | CH₂CN | O |
| C-5507 | H | CH₂CH₂ | Me | c-Pr(1-CN) | Me | OCF₃ | CH₂CN | O |
| C-5508 | Me | CH₂CH₂ | H | c-Pr(1-CN) | Me | OCF₃ | CH₂CN | O |
| C-5509 | H | single bond | Me | CH₂CF₃ | C(=O)Me | Br | H | O |
| C-5510 | Me | single bond | H | CH₂CF₃ | C(=O)Me | Br | H | O |
| C-5511 | Me(R) | single bond | H | CH₂CF₃ | C(=O)Me | Br | H | O |
| C-5512 | Me(S) | single bond | H | CH₂CF₃ | C(=O)Me | Br | H | O |
| C-5513 | H | CH₂ | Me | CH₂CF₃ | C(=O)Me | Br | H | O |
| C-5514 | Me | CH₂ | H | CH₂CF₃ | C(=O)Me | Br | H | O |
| C-5515 | H | CH(Me) | H | CH₂CF₃ | C(=O)Me | Br | H | O |
| C-5516 | H | CH₂CH₂ | Me | CH₂CF₃ | C(=O)Me | Br | H | O |
| C-5517 | Me | CH₂CH₂ | H | CH₂CF₃ | C(=O)Me | Br | H | O |
| C-5518 | H | single bond | Me | Et | C(=O)Me | Br | H | O |
| C-5519 | Me | single bond | H | Et | C(=O)Me | Br | H | O |
| C-5520 | Me(R) | single bond | H | Et | C(=O)Me | Br | H | O |
| C-5521 | Me(S) | single bond | H | Et | C(=O)Me | Br | H | O |
| C-5522 | H | CH₂ | Me | Et | C(=O)Me | Br | H | O |
| C-5523 | Me | CH₂ | H | Et | C(=O)Me | Br | H | O |
| C-5524 | H | CH(Me) | H | Et | C(=O)Me | Br | H | O |
| C-5525 | H | CH₂CH₂ | Me | Et | C(=O)Me | Br | H | O |
| C-5526 | Me | CH₂CH₂ | H | Et | C(=O)Me | Br | H | O |
| C-5527 | H | single bond | Me | c-Pr | C(=O)Me | Br | H | O |
| C-5528 | Me | single bond | H | c-Pr | C(=O)Me | Br | H | O |
| C-5529 | Me(R) | single bond | H | c-Pr | C(=O)Me | Br | H | O |
| C-5530 | Me(S) | single bond | H | c-Pr | C(=O)Me | Br | H | O |
| C-5531 | H | CH₂ | Me | c-Pr | C(=O)Me | Br | H | O |
| C-5532 | Me | CH₂ | H | c-Pr | C(=O)Me | Br | H | O |
| C-5533 | H | CH(Me) | H | c-Pr | C(=O)Me | Br | H | O |
| C-5534 | H | CH₂CH₂ | Me | c-Pr | C(=O)Me | Br | H | O |
| C-5535 | Me | CH₂CH₂ | H | c-Pr | C(=O)Me | Br | H | O |
| C-5536 | H | single bond | Me | c-Pr(1-CN) | C(=O)Me | Br | H | O |
| C-5537 | Me | single bond | H | c-Pr(1-CN) | C(=O)Me | Br | H | O |
| C-5538 | Me(R) | single bond | H | c-Pr(1-CN) | C(=O)Me | Br | H | O |

**[Table 353]**

| Compound Number | Y | W | Z | R¹ | R² | R³ | R¹⁰ | A |
|---|---|---|---|---|---|---|---|---|
| C-5539 | Me(S) | single bond | H | c-Pr(1-CN) | C(=O)Me | Br | H | O |
| C-5540 | H | CH₂ | Me | c-Pr(1-CN) | C(=O)Me | Br | H | O |
| C-5541 | Me | CH₂ | H | c-Pr(1-CN) | C(=O)Me | Br | H | O |
| C-5542 | H | CH(Me) | H | c-Pr(1-CN) | C(=O)Me | Br | H | O |
| C-5543 | H | CH₂CH₂ | Me | c-Pr(1-CN) | C(=O)Me | Br | H | O |
| C-5544 | Me | CH₂CH₂ | H | c-Pr(1-CN) | C(=O)Me | Br | H | O |
| C-5545 | H | single bond | Me | CH₂CF₃ | C(=O)Me | CF₃ | H | O |
| C-5546 | Me | single bond | H | CH₂CF₃ | C(=O)Me | CF₃ | H | O |
| C-5547 | Me(R) | single bond | H | CH₂CF₃ | C(=O)Me | CF₃ | H | O |
| C-5548 | Me(S) | single bond | H | CH₂CF₃ | C(=O)Me | CF₃ | H | O |
| C-5549 | H | CH₂ | Me | CH₂CF₃ | C(=O)Me | CF₃ | H | O |
| C-5550 | Me | CH₂ | H | CH₂CF₃ | C(=O)Me | CF₃ | H | O |
| C-5551 | H | CH(Me) | H | CH₂CF₃ | C(=O)Me | CF₃ | H | O |
| C-5552 | H | CH₂CH₂ | Me | CH₂CF₃ | C(=O)Me | CF₃ | H | O |
| C-5553 | Me | CH₂CH₂ | H | CH₂CF₃ | C(=O)Me | CF₃ | H | O |
| C-5554 | H | single bond | Me | Et | C(=O)Me | CF₃ | H | O |
| C-5555 | Me | single bond | H | Et | C(=O)Me | CF₃ | H | O |
| C-5556 | Me(R) | single bond | H | Et | C(=O)Me | CF₃ | H | O |
| C-5557 | Me(S) | single bond | H | Et | C(=O)Me | CF₃ | H | O |
| C-5558 | H | CH₂ | Me | Et | C(=O)Me | CF₃ | H | O |
| C-5559 | Me | CH₂ | H | Et | C(=O)Me | CF₃ | H | O |
| C-5560 | H | CH(Me) | H | Et | C(=O)Me | CF₃ | H | O |
| C-5561 | H | CH₂CH₂ | Me | Et | C(=O)Me | CF₃ | H | O |
| C-5562 | Me | CH₂CH₂ | H | Et | C(=O)Me | CF₃ | H | O |
| C-5563 | H | single bond | Me | c-Pr | C(=O)Me | CF₃ | H | O |
| C-5564 | Me | single bond | H | c-Pr | C(=O)Me | CF₃ | H | O |
| C-5565 | Me(R) | single bond | H | c-Pr | C(=O)Me | CF₃ | H | O |
| C-5566 | Me(S) | single bond | H | c-Pr | C(=O)Me | CF₃ | H | O |
| C-5567 | H | CH₂ | Me | c-Pr | C(=O)Me | CF₃ | H | O |
| C-5568 | Me | CH₂ | H | c-Pr | C(=O)Me | CF₃ | H | O |
| C-5569 | H | CH(Me) | H | c-Pr | C(=O)Me | CF₃ | H | O |
| C-5570 | H | CH₂CH₂ | Me | c-Pr | C(=O)Me | CF₃ | H | O |
| C-5571 | Me | CH₂CH₂ | H | c-Pr | C(=O)Me | CF₃ | H | O |
| C-5572 | H | single bond | Me | c-Pr(1-CN) | C(=O)Me | CF₃ | H | O |
| C-5573 | Me | single bond | H | c-Pr(1-CN) | C(=O)Me | CF₃ | H | O |
| C-5574 | Me(R) | single bond | H | c-Pr(1-CN) | C(=O)Me | CF₃ | H | O |
| C-5575 | Me(S) | single bond | H | c-Pr(1-CN) | C(=O)Me | CF₃ | H | O |
| C-5576 | H | CH₂ | Me | c-Pr(1-CN) | C(=O)Me | CF₃ | H | O |
| C-5577 | Me | CH₂ | H | c-Pr(1-CN) | C(=O)Me | CF₃ | H | O |
| C-5578 | H | CH(Me) | H | c-Pr(1-CN) | C(=O)Me | CF₃ | H | O |
| C-5579 | H | CH₂CH₂ | Me | c-Pr(1-CN) | C(=O)Me | CF₃ | H | O |
| C-5580 | Me | CH₂CH₂ | H | c-Pr(1-CN) | C(=O)Me | CF₃ | H | O |
| C-5581 | H | single bond | Me | CH₂CF₃ | C(=O)Me | OMe | H | O |
| C-5582 | Me | single bond | H | CH₂CF₃ | C(=O)Me | OMe | H | O |

**[Table 354]**

| Compound Number | Y | W | Z | R¹ | R² | R³ | R¹⁰ | A |
|---|---|---|---|---|---|---|---|---|
| C-5583 | Me(R) | single bond | H | CH₂CF₃ | C(=O)Me | OMe | H | O |
| C-5584 | Me(S) | single bond | H | CH₂CF₃ | C(=O)Me | OMe | H | O |
| C-5585 | H | CH₂ | Me | CH₂CF₃ | C(=O)Me | OMe | H | O |
| C-5586 | Me | CH₂ | H | CH₂CF₃ | C(=O)Me | OMe | H | O |
| C-5587 | H | CH(Me) | H | CH₂CF₃ | C(=O)Me | OMe | H | O |
| C-5588 | H | CH₂CH₂ | Me | CH₂CF₃ | C(=O)Me | OMe | H | O |
| C-5589 | Me | CH₂CH₂ | H | CH₂CF₃ | C(=O)Me | OMe | H | O |
| C-5590 | H | single bond | Me | Et | C(=O)Me | OMe | H | O |
| C-5591 | Me | single bond | H | Et | C(=O)Me | OMe | H | O |
| C-5592 | Me(R) | single bond | H | Et | C(=O)Me | OMe | H | O |
| C-5593 | Me(S) | single bond | H | Et | C(=O)Me | OMe | H | O |
| C-5594 | H | CH₂ | Me | Et | C(=O)Me | OMe | H | O |
| C-5595 | Me | CH₂ | H | Et | C(=O)Me | OMe | H | O |
| C-5596 | H | CH(Me) | H | Et | C(=O)Me | OMe | H | O |
| C-5597 | H | CH₂CH₂ | Me | Et | C(=O)Me | OMe | H | O |
| C-5598 | Me | CH₂CH₂ | H | Et | C(=O)Me | OMe | H | O |
| C-5599 | H | single bond | Me | c-Pr | C(=O)Me | OMe | H | O |
| C-5600 | Me | single bond | H | c-Pr | C(=O)Me | OMe | H | O |
| C-5601 | Me(R) | single bond | H | c-Pr | C(=O)Me | OMe | H | O |
| C-5602 | Me(S) | single bond | H | c-Pr | C(=O)Me | OMe | H | O |
| C-5603 | H | CH₂ | Me | c-Pr | C(=O)Me | OMe | H | O |
| C-5604 | Me | CH₂ | H | c-Pr | C(=O)Me | OMe | H | O |
| C-5605 | H | CH(Me) | H | c-Pr | C(=O)Me | OMe | H | O |
| C-5606 | H | CH₂CH₂ | Me | c-Pr | C(=O)Me | OMe | H | O |
| C-5607 | Me | CH₂CH₂ | H | c-Pr | C(=O)Me | OMe | H | O |
| C-5608 | H | single bond | Me | c-Pr(1-CN) | C(=O)Me | OMe | H | O |
| C-5609 | Me | single bond | H | c-Pr(1-CN) | C(=O)Me | OMe | H | O |
| C-5610 | Me(R) | single bond | H | c-Pr(1-CN) | C(=O)Me | OMe | H | O |
| C-5611 | Me(S) | single bond | H | c-Pr(1-CN) | C(=O)Me | OMe | H | O |
| C-5612 | H | CH₂ | Me | c-Pr(1-CN) | C(=O)Me | OMe | H | O |
| C-5613 | Me | CH₂ | H | c-Pr(1-CN) | C(=O)Me | OMe | H | O |
| C-5614 | H | CH(Me) | H | c-Pr(1-CN) | C(=O)Me | OMe | H | O |
| C-5615 | H | CH₂CH₂ | Me | c-Pr(1-CN) | C(=O)Me | OMe | H | O |
| C-5616 | Me | CH₂CH₂ | H | c-Pr(1-CN) | C(=O)Me | OMe | H | O |
| C-5617 | H | single bond | Me | CH₂CF₃ | C(=O)Me | OCF₃ | H | O |
| C-5618 | Me | single bond | H | CH₂CF₃ | C(=O)Me | OCF₃ | H | O |
| C-5619 | Me(R) | single bond | H | CH₂CF₃ | C(=O)Me | OCF₃ | H | O |
| C-5620 | Me(S) | single bond | H | CH₂CF₃ | C(=O)Me | OCF₃ | H | O |
| C-5621 | H | CH₂ | Me | CH₂CF₃ | C(=O)Me | OCF₃ | H | O |
| C-5622 | Me | CH₂ | H | CH₂CF₃ | C(=O)Me | OCF₃ | H | O |
| C-5623 | H | CH(Me) | H | CH₂CF₃ | C(=O)Me | OCF₃ | H | O |
| C-5624 | H | CH₂CH₂ | Me | CH₂CF₃ | C(=O)Me | OCF₃ | H | O |
| C-5625 | Me | CH₂CH₂ | H | CH₂CF₃ | C(=O)Me | OCF₃ | H | O |
| C-5626 | H | single bond | Me | Et | C(=O)Me | OCF₃ | H | O |

**[Table 355]**

| Compound Number | Y | W | Z | R¹ | R² | R³ | R¹⁰ | A |
|---|---|---|---|---|---|---|---|---|
| C-5627 | Me | single bond | H | Et | C(=O)Me | OCF₃ | H | O |
| C-5628 | Me(R) | single bond | H | Et | C(=O)Me | OCF₃ | H | O |
| C-5629 | Me(S) | single bond | H | Et | C(=O)Me | OCF₃ | H | O |
| C-5630 | H | CH₂ | Me | Et | C(=O)Me | OCF₃ | H | O |
| C-5631 | Me | CH₂ | H | Et | C(=O)Me | OCF₃ | H | O |
| C-5632 | H | CH(Me) | H | Et | C(=O)Me | OCF₃ | H | O |
| C-5633 | H | CH₂CH₂ | Me | Et | C(=O)Me | OCF₃ | H | O |
| C-5634 | Me | CH₂CH₂ | H | Et | C(=O)Me | OCF₃ | H | O |
| C-5635 | H | single bond | Me | c-Pr | C(=O)Me | OCF₃ | H | O |
| C-5636 | Me | single bond | H | c-Pr | C(=O)Me | OCF₃ | H | O |
| C-5637 | Me(R) | single bond | H | c-Pr | C(=O)Me | OCF₃ | H | O |
| C-5638 | Me(S) | single bond | H | c-Pr | C(=O)Me | OCF₃ | H | O |
| C-5639 | H | CH₂ | Me | c-Pr | C(=O)Me | OCF₃ | H | O |
| C-5640 | Me | CH₂ | H | c-Pr | C(=O)Me | OCF₃ | H | O |
| C-5641 | H | CH(Me) | H | c-Pr | C(=O)Me | OCF₃ | H | O |
| C-5642 | H | CH₂CH₂ | Me | c-Pr | C(=O)Me | OCF₃ | H | O |
| C-5643 | Me | CH₂CH₂ | H | c-Pr | C(=O)Me | OCF₃ | H | O |
| C-5644 | H | single bond | Me | c-Pr(1-CN) | C(=O)Me | OCF₃ | H | O |
| C-5645 | Me | single bond | H | c-Pr(1-CN) | C(=O)Me | OCF₃ | H | O |
| C-5646 | Me(R) | single bond | H | c-Pr(1-CN) | C(=O)Me | OCF₃ | H | O |
| C-5647 | Me(S) | single bond | H | c-Pr(1-CN) | C(=O)Me | OCF₃ | H | O |
| C-5648 | H | CH₂ | Me | c-Pr(1-CN) | C(=O)Me | OCF₃ | H | O |
| C-5649 | Me | CH₂ | H | c-Pr(1-CN) | C(=O)Me | OCF₃ | H | O |
| C-5650 | H | CH(Me) | H | c-Pr(1-CN) | C(=O)Me | OCF₃ | H | O |
| C-5651 | H | CH₂CH₂ | Me | c-Pr(1-CN) | C(=O)Me | OCF₃ | H | O |
| C-5652 | Me | CH₂CH₂ | H | c-Pr(1-CN) | C(=O)Me | OCF₃ | H | O |
| C-5653 | H | single bond | Me | CH₂CF₃ | C(=O)Me | Br | Me | O |
| C-5654 | Me | single bond | H | CH₂CF₃ | C(=O)Me | Br | Me | O |
| C-5655 | Me(R) | single bond | H | CH₂CF₃ | C(=O)Me | Br | Me | O |
| C-5656 | Me(S) | single bond | H | CH₂CF₃ | C(=O)Me | Br | Me | O |
| C-5657 | H | CH₂ | Me | CH₂CF₃ | C(=O)Me | Br | Me | O |
| C-5658 | Me | CH₂ | H | CH₂CF₃ | C(=O)Me | Br | Me | O |
| C-5659 | H | CH(Me) | H | CH₂CF₃ | C(=O)Me | Br | Me | O |
| C-5660 | H | CH₂CH₂ | Me | CH₂CF₃ | C(=O)Me | Br | Me | O |
| C-5661 | Me | CH₂CH₂ | H | CH₂CF₃ | C(=O)Me | Br | Me | O |
| C-5662 | H | single bond | Me | Et | C(=O)Me | Br | Me | O |
| C-5663 | Me | single bond | H | Et | C(=O)Me | Br | Me | O |
| C-5664 | Me(R) | single bond | H | Et | C(=O)Me | Br | Me | O |
| C-5665 | Me(S) | single bond | H | Et | C(=O)Me | Br | Me | O |
| C-5666 | H | CH₂ | Me | Et | C(=O)Me | Br | Me | O |
| C-5667 | Me | CH₂ | H | Et | C(=O)Me | Br | Me | O |
| C-5668 | H | CH(Me) | H | Et | C(=O)Me | Br | Me | O |
| C-5669 | H | CH₂CH₂ | Me | Et | C(=O)Me | Br | Me | O |
| C-5670 | Me | CH₂CH₂ | H | Et | C(=O)Me | Br | Me | O |

**[Table 356]**

| Compound Number | Y | W | Z | R¹ | R² | R³ | R¹⁰ | A |
|---|---|---|---|---|---|---|---|---|
| C-5671 | H | single bond | Me | c-Pr | C(=O)Me | Br | Me | O |
| C-5672 | Me | single bond | H | c-Pr | C(=O)Me | Br | Me | O |
| C-5673 | Me(R) | single bond | H | c-Pr | C(=O)Me | Br | Me | O |
| C-5674 | Me(S) | single bond | H | c-Pr | C(=O)Me | Br | Me | O |
| C-5675 | H | CH₂ | Me | c-Pr | C(=O)Me | Br | Me | O |
| C-5676 | Me | CH₂ | H | c-Pr | C(=O)Me | Br | Me | O |
| C-5677 | H | CH(Me) | H | c-Pr | C(=O)Me | Br | Me | O |
| C-5678 | H | CH₂CH₂ | Me | c-Pr | C(=O)Me | Br | Me | O |
| C-5679 | Me | CH₂CH₂ | H | c-Pr | C(=O)Me | Br | Me | O |
| C-5680 | H | single bond | Me | c-Pr(1-CN) | C(=O)Me | Br | Me | O |
| C-5681 | Me | single bond | H | c-Pr(1-CN) | C(=O)Me | Br | Me | O |
| C-5682 | Me(R) | single bond | H | c-Pr(1-CN) | C(=O)Me | Br | Me | O |
| C-5683 | Me(S) | single bond | H | c-Pr(1-CN) | C(=O)Me | Br | Me | O |
| C-5684 | H | CH₂ | Me | c-Pr(1-CN) | C(=O)Me | Br | Me | O |
| C-5685 | Me | CH₂ | H | c-Pr(1-CN) | C(=O)Me | Br | Me | O |
| C-5686 | H | CH(Me) | H | c-Pr(1-CN) | C(=O)Me | Br | Me | O |
| C-5687 | H | CH₂CH₂ | Me | c-Pr(1-CN) | C(=O)Me | Br | Me | O |
| C-5688 | Me | CH₂CH₂ | H | c-Pr(1-CN) | C(=O)Me | Br | Me | O |
| C-5689 | H | single bond | Me | CH₂CF₃ | C(=O)Me | CF₃ | Me | O |
| C-5690 | Me | single bond | H | CH₂CF₃ | C(=O)Me | CF₃ | Me | O |
| C-5691 | Me(R) | single bond | H | CH₂CF₃ | C(=O)Me | CF₃ | Me | O |
| C-5692 | Me(S) | single bond | H | CH₂CF₃ | C(=O)Me | CF₃ | Me | O |
| C-5693 | H | CH₂ | Me | CH₂CF₃ | C(=O)Me | CF₃ | Me | O |
| C-5694 | Me | CH₂ | H | CH₂CF₃ | C(=O)Me | CF₃ | Me | O |
| C-5695 | H | CH(Me) | H | CH₂CF₃ | C(=O)Me | CF₃ | Me | O |
| C-5696 | H | CH₂CH₂ | Me | CH₂CF₃ | C(=O)Me | CF₃ | Me | O |
| C-5697 | Me | CH₂CH₂ | H | CH₂CF₃ | C(=O)Me | CF₃ | Me | O |
| C-5698 | H | single bond | Me | Et | C(=O)Me | CF₃ | Me | O |
| C-5699 | Me | single bond | H | Et | C(=O)Me | CF₃ | Me | O |
| C-5700 | Me(R) | single bond | H | Et | C(=O)Me | CF₃ | Me | O |
| C-5701 | Me(S) | single bond | H | Et | C(=O)Me | CF₃ | Me | O |
| C-5702 | H | CH₂ | Me | Et | C(=O)Me | CF₃ | Me | O |
| C-5703 | Me | CH₂ | H | Et | C(=O)Me | CF₃ | Me | O |
| C-5704 | H | CH(Me) | H | Et | C(=O)Me | CF₃ | Me | O |
| C-5705 | H | CH₂CH₂ | Me | Et | C(=O)Me | CF₃ | Me | O |
| C-5706 | Me | CH₂CH₂ | H | Et | C(=O)Me | CF₃ | Me | O |
| C-5707 | H | single bond | Me | c-Pr | C(=O)Me | CF₃ | Me | O |
| C-5708 | Me | single bond | H | c-Pr | C(=O)Me | CF₃ | Me | O |
| C-5709 | Me(R) | single bond | H | c-Pr | C(=O)Me | CF₃ | Me | O |
| C-5710 | Me(S) | single bond | H | c-Pr | C(=O)Me | CF₃ | Me | O |
| C-5711 | H | CH₂ | Me | c-Pr | C(=O)Me | CF₃ | Me | O |
| C-5712 | Me | CH₂ | H | c-Pr | C(=O)Me | CF₃ | Me | O |
| C-5713 | H | CH(Me) | H | c-Pr | C(=O)Me | CF₃ | Me | O |
| C-5714 | H | CH₂CH₂ | Me | c-Pr | C(=O)Me | CF₃ | Me | O |

**[Table 357]**

| Compound Number | Y | W | Z | R¹ | R² | R³ | R¹⁰ | A |
|---|---|---|---|---|---|---|---|---|
| C-5715 | Me | CH₂CH₂ | H | c-Pr | C(=O)Me | CF₃ | Me | O |
| C-5716 | H | single bond | Me | c-Pr(1-CN) | C(=O)Me | CF₃ | Me | O |
| C-5717 | Me | single bond | H | c-Pr(1-CN) | C(=O)Me | CF₃ | Me | O |
| C-5718 | Me(R) | single bond | H | c-Pr(1-CN) | C(=O)Me | CF₃ | Me | O |
| C-5719 | Me(S) | single bond | H | c-Pr(1-CN) | C(=O)Me | CF₃ | Me | O |
| C-5720 | H | CH₂ | Me | c-Pr(1-CN) | C(=O)Me | CF₃ | Me | O |
| C-5721 | Me | CH₂ | H | c-Pr(1-CN) | C(=O)Me | CF₃ | Me | O |
| C-5722 | H | CH(Me) | H | c-Pr(1-CN) | C(=O)Me | CF₃ | Me | O |
| C-5723 | H | CH₂CH₂ | Me | c-Pr(1-CN) | C(=O)Me | CF₃ | Me | O |
| C-5724 | Me | CH₂CH₂ | H | c-Pr(1-CN) | C(=O)Me | CF₃ | Me | O |
| C-5725 | H | single bond | Me | CH₂CF₃ | C(=O)Me | OMe | Me | O |
| C-5726 | Me | single bond | H | CH₂CF₃ | C(=O)Me | OMe | Me | O |
| C-5727 | Me(R) | single bond | H | CH₂CF₃ | C(=O)Me | OMe | Me | O |
| C-5728 | Me(S) | single bond | H | CH₂CF₃ | C(=O)Me | OMe | Me | O |
| C-5729 | H | CH₂ | Me | CH₂CF₃ | C(=O)Me | OMe | Me | O |
| C-5730 | Me | CH₂ | H | CH₂CF₃ | C(=O)Me | OMe | Me | O |
| C-5731 | H | CH(Me) | H | CH₂CF₃ | C(=O)Me | OMe | Me | O |
| C-5732 | H | CH₂CH₂ | Me | CH₂CF₃ | C(=O)Me | OMe | Me | O |
| C-5733 | Me | CH₂CH₂ | H | CH₂CF₃ | C(=O)Me | OMe | Me | O |
| C-5734 | H | single bond | Me | Et | C(=O)Me | OMe | Me | O |
| C-5735 | Me | single bond | H | Et | C(=O)Me | OMe | Me | O |
| C-5736 | Me(R) | single bond | H | Et | C(=O)Me | OMe | Me | O |
| C-5737 | Me(S) | single bond | H | Et | C(=O)Me | OMe | Me | O |
| C-5738 | H | CH₂ | Me | Et | C(=O)Me | OMe | Me | O |
| C-5739 | Me | CH₂ | H | Et | C(=O)Me | OMe | Me | O |
| C-5740 | H | CH(Me) | H | Et | C(=O)Me | OMe | Me | O |
| C-5741 | H | CH₂CH₂ | Me | Et | C(=O)Me | OMe | Me | O |
| C-5742 | Me | CH₂CH₂ | H | Et | C(=O)Me | OMe | Me | O |
| C-5743 | H | single bond | Me | c-Pr | C(=O)Me | OMe | Me | O |
| C-5744 | Me | single bond | H | c-Pr | C(=O)Me | OMe | Me | O |
| C-5745 | Me(R) | single bond | H | c-Pr | C(=O)Me | OMe | Me | O |
| C-5746 | Me(S) | single bond | H | c-Pr | C(=O)Me | OMe | Me | O |
| C-5747 | H | CH₂ | Me | c-Pr | C(=O)Me | OMe | Me | O |
| C-5748 | Me | CH₂ | H | c-Pr | C(=O)Me | OMe | Me | O |
| C-5749 | H | CH(Me) | H | c-Pr | C(=O)Me | OMe | Me | O |
| C-5750 | H | CH₂CH₂ | Me | c-Pr | C(=O)Me | OMe | Me | O |
| C-5751 | Me | CH₂CH₂ | H | c-Pr | C(=O)Me | OMe | Me | O |
| C-5752 | H | single bond | Me | c-Pr(1-CN) | C(=O)Me | OMe | Me | O |
| C-5753 | Me | single bond | H | c-Pr(1-CN) | C(=O)Me | OMe | Me | O |
| C-5754 | Me(R) | single bond | H | c-Pr(1-CN) | C(=O)Me | OMe | Me | O |
| C-5755 | Me(S) | single bond | H | c-Pr(1-CN) | C(=O)Me | OMe | Me | O |
| C-5756 | H | CH₂ | Me | c-Pr(1-CN) | C(=O)Me | OMe | Me | O |
| C-5757 | Me | CH₂ | H | c-Pr(1-CN) | C(=O)Me | OMe | Me | O |
| C-5758 | H | CH(Me) | H | c-Pr(1-CN) | C(=O)Me | OMe | Me | O |

**[Table 358]**

| Compound Number | Y | W | Z | R¹ | R² | R³ | R¹⁰ | A |
|---|---|---|---|---|---|---|---|---|
| C-5759 | H | CH₂CH₂ | Me | c-Pr(1-CN) | C(=O)Me | OMe | Me | O |
| C-5760 | Me | CH₂CH₂ | H | c-Pr(1-CN) | C(=O)Me | OMe | Me | O |
| C-5761 | H | single bond | Me | CH₂CF₃ | C(=O)Me | OCF₃ | Me | O |
| C-5762 | Me | single bond | H | CH₂CF₃ | C(=O)Me | OCF₃ | Me | O |
| C-5763 | Me(R) | single bond | H | CH₂CF₃ | C(=O)Me | OCF₃ | Me | O |
| C-5764 | Me(S) | single bond | H | CH₂CF₃ | C(=O)Me | OCF₃ | Me | O |
| C-5765 | H | CH₂ | Me | CH₂CF₃ | C(=O)Me | OCF₃ | Me | O |
| C-5766 | Me | CH₂ | H | CH₂CF₃ | C(=O)Me | OCF₃ | Me | O |
| C-5767 | H | CH(Me) | H | CH₂CF₃ | C(=O)Me | OCF₃ | Me | O |
| C-5768 | H | CH₂CH₂ | Me | CH₂CF₃ | C(=O)Me | OCF₃ | Me | O |
| C-5769 | Me | CH₂CH₂ | H | CH₂CF₃ | C(=O)Me | OCF₃ | Me | O |
| C-5770 | H | single bond | Me | Et | C(=O)Me | OCF₃ | Me | O |
| C-5771 | Me | single bond | H | Et | C(=O)Me | OCF₃ | Me | O |
| C-5772 | Me(R) | single bond | H | Et | C(=O)Me | OCF₃ | Me | O |
| C-5773 | Me(S) | single bond | H | Et | C(=O)Me | OCF₃ | Me | O |
| C-5774 | H | CH₂ | Me | Et | C(=O)Me | OCF₃ | Me | O |
| C-5775 | Me | CH₂ | H | Et | C(=O)Me | OCF₃ | Me | O |
| C-5776 | H | CH(Me) | H | Et | C(=O)Me | OCF₃ | Me | O |
| C-5777 | H | CH₂CH₂ | Me | Et | C(=O)Me | OCF₃ | Me | O |
| C-5778 | Me | CH₂CH₂ | H | Et | C(=O)Me | OCF₃ | Me | O |
| C-5779 | H | single bond | Me | c-Pr | C(=O)Me | OCF₃ | Me | O |
| C-5780 | Me | single bond | H | c-Pr | C(=O)Me | OCF₃ | Me | O |
| C-5781 | Me(R) | single bond | H | c-Pr | C(=O)Me | OCF₃ | Me | O |
| C-5782 | Me(S) | single bond | H | c-Pr | C(=O)Me | OCF₃ | Me | O |
| C-5783 | H | CH₂ | Me | c-Pr | C(=O)Me | OCF₃ | Me | O |
| C-5784 | Me | CH₂ | H | c-Pr | C(=O)Me | OCF₃ | Me | O |
| C-5785 | H | CH(Me) | H | c-Pr | C(=O)Me | OCF₃ | Me | O |
| C-5786 | H | CH₂CH₂ | Me | c-Pr | C(=O)Me | OCF₃ | Me | O |
| C-5787 | Me | CH₂CH₂ | H | c-Pr | C(=O)Me | OCF₃ | Me | O |
| C-5788 | H | single bond | Me | c-Pr(1-CN) | C(=O)Me | OCF₃ | Me | O |
| C-5789 | Me | single bond | H | c-Pr(1-CN) | C(=O)Me | OCF₃ | Me | O |
| C-5790 | Me(R) | single bond | H | c-Pr(1-CN) | C(=O)Me | OCF₃ | Me | O |
| C-5791 | Me(S) | single bond | H | c-Pr(1-CN) | C(=O)Me | OCF₃ | Me | O |
| C-5792 | H | CH₂ | Me | c-Pr(1-CN) | C(=O)Me | OCF₃ | Me | O |
| C-5793 | Me | CH₂ | H | c-Pr(1-CN) | C(=O)Me | OCF₃ | Me | O |
| C-5794 | H | CH(Me) | H | c-Pr(1-CN) | C(=O)Me | OCF₃ | Me | O |
| C-5795 | H | CH₂CH₂ | Me | c-Pr(1-CN) | C(=O)Me | OCF₃ | Me | O |
| C-5796 | Me | CH₂CH₂ | H | c-Pr(1-CN) | C(=O)Me | OCF₃ | Me | O |
| C-5797 | H | single bond | Me | CH₂CF₃ | C(=O)Me | Br | CH₂OMe | O |
| C-5798 | Me | single bond | H | CH₂CF₃ | C(=O)Me | Br | CH₂OMe | O |
| C-5799 | Me(R) | single bond | H | CH₂CF₃ | C(=O)Me | Br | CH₂OMe | O |
| C-5800 | Me(S) | single bond | H | CH₂CF₃ | C(=O)Me | Br | CH₂OMe | O |
| C-5801 | H | CH₂ | Me | CH₂CF₃ | C(=O)Me | Br | CH₂OMe | O |
| C-5802 | Me | CH₂ | H | CH₂CF₃ | C(=O)Me | Br | CH₂OMe | O |

**[Table 359]**

| Compound Number | Y | W | Z | R¹ | R² | R³ | R¹⁰ | A |
|---|---|---|---|---|---|---|---|---|
| C-5803 | H | CH(Me) | H | CH₂CF₃ | C(=O)Me | Br | CH₂OMe | O |
| C-5804 | H | CH₂CH₂ | Me | CH₂CF₃ | C(=O)Me | Br | CH₂OMe | O |
| C-5805 | Me | CH₂CH₂ | H | CH₂CF₃ | C(=O)Me | Br | CH₂OMe | O |
| C-5806 | H | single bond | Me | Et | C(=O)Me | Br | CH₂OMe | O |
| C-5807 | Me | single bond | H | Et | C(=O)Me | Br | CH₂OMe | O |
| C-5808 | Me(R) | single bond | H | Et | C(=O)Me | Br | CH₂OMe | O |
| C-5809 | Me(S) | single bond | H | Et | C(=O)Me | Br | CH₂OMe | O |
| C-5810 | H | CH₂ | Me | Et | C(=O)Me | Br | CH₂OMe | O |
| C-5811 | Me | CH₂ | H | Et | C(=O)Me | Br | CH₂OMe | O |
| C-5812 | H | CH(Me) | H | Et | C(=O)Me | Br | CH₂OMe | O |
| C-5813 | H | CH₂CH₂ | Me | Et | C(=O)Me | Br | CH₂OMe | O |
| C-5814 | Me | CH₂CH₂ | H | Et | C(=O)Me | Br | CH₂OMe | O |
| C-5815 | H | single bond | Me | c-Pr | C(=O)Me | Br | CH₂OMe | O |
| C-5816 | Me | single bond | H | c-Pr | C(=O)Me | Br | CH₂OMe | O |
| C-5817 | Me(R) | single bond | H | c-Pr | C(=O)Me | Br | CH₂OMe | O |
| C-5818 | Me(S) | single bond | H | c-Pr | C(=O)Me | Br | CH₂OMe | O |
| C-5819 | H | CH₂ | Me | c-Pr | C(=O)Me | Br | CH₂OMe | O |
| C-5820 | Me | CH₂ | H | c-Pr | C(=O)Me | Br | CH₂OMe | O |
| C-5821 | H | CH(Me) | H | c-Pr | C(=O)Me | Br | CH₂OMe | O |
| C-5822 | H | CH₂CH₂ | Me | c-Pr | C(=O)Me | Br | CH₂OMe | O |
| C-5823 | Me | CH₂CH₂ | H | c-Pr | C(=O)Me | Br | CH₂OMe | O |
| C-5824 | H | single bond | Me | c-Pr(1-CN) | C(=O)Me | Br | CH₂OMe | O |
| C-5825 | Me | single bond | H | c-Pr(1-CN) | C(=O)Me | Br | CH₂OMe | O |
| C-5826 | Me(R) | single bond | H | c-Pr(1-CN) | C(=O)Me | Br | CH₂OMe | O |
| C-5827 | Me(S) | single bond | H | c-Pr(1-CN) | C(=O)Me | Br | CH₂OMe | O |
| C-5828 | H | CH₂ | Me | c-Pr(1-CN) | C(=O)Me | Br | CH₂OMe | O |
| C-5829 | Me | CH₂ | H | c-Pr(1-CN) | C(=O)Me | Br | CH₂OMe | O |
| C-5830 | H | CH(Me) | H | c-Pr(1-CN) | C(=O)Me | Br | CH₂OMe | O |
| C-5831 | H | CH₂CH₂ | Me | c-Pr(1-CN) | C(=O)Me | Br | CH₂OMe | O |
| C-5832 | Me | CH₂CH₂ | H | c-Pr(1-CN) | C(=O)Me | Br | CH₂OMe | O |
| C-5833 | H | single bond | Me | CH₂CF₃ | C(=O)Me | CF₃ | CH₂OMe | O |
| C-5834 | Me | single bond | H | CH₂CF₃ | C(=O)Me | CF₃ | CH₂OMe | O |
| C-5835 | Me(R) | single bond | H | CH₂CF₃ | C(=O)Me | CF₃ | CH₂OMe | O |
| C-5836 | Me(S) | single bond | H | CH₂CF₃ | C(=O)Me | CF₃ | CH₂OMe | O |
| C-5837 | H | CH₂ | Me | CH₂CF₃ | C(=O)Me | CF₃ | CH₂OMe | O |
| C-5838 | Me | CH₂ | H | CH₂CF₃ | C(=O)Me | CF₃ | CH₂OMe | O |
| C-5839 | H | CH(Me) | H | CH₂CF₃ | C(=O)Me | CF₃ | CH₂OMe | O |
| C-5840 | H | CH₂CH₂ | Me | CH₂CF₃ | C(=O)Me | CF₃ | CH₂OMe | O |
| C-5841 | Me | CH₂CH₂ | H | CH₂CF₃ | C(=O)Me | CF₃ | CH₂OMe | O |
| C-5842 | H | single bond | Me | Et | C(=O)Me | CF₃ | CH₂OMe | O |
| C-5843 | Me | single bond | H | Et | C(=O)Me | CF₃ | CH₂OMe | O |
| C-5844 | Me(R) | single bond | H | Et | C(=O)Me | CF₃ | CH₂OMe | O |
| C-5845 | Me(S) | single bond | H | Et | C(=O)Me | CF₃ | CH₂OMe | O |
| C-5846 | H | CH₂ | Me | Et | C(=O)Me | CF₃ | CH₂OMe | O |

**[Table 360]**

| Compound Number | Y | W | Z | R¹ | R² | R³ | R¹⁰ | A |
|---|---|---|---|---|---|---|---|---|
| C-5847 | Me | CH₂ | H | Et | C(=O)Me | CF₃ | CH₂OMe | O |
| C-5848 | H | CH(Me) | H | Et | C(=O)Me | CF₃ | CH₂OMe | O |
| C-5849 | H | CH₂CH₂ | Me | Et | C(=O)Me | CF₃ | CH₂OMe | O |
| C-5850 | Me | CH₂CH₂ | H | Et | C(=O)Me | CF₃ | CH₂OMe | O |
| C-5851 | H | single bond | Me | c-Pr | C(=O)Me | CF₃ | CH₂OMe | O |
| C-5852 | Me | single bond | H | c-Pr | C(=O)Me | CF₃ | CH₂OMe | O |
| C-5853 | Me(R) | single bond | H | c-Pr | C(=O)Me | CF₃ | CH₂OMe | O |
| C-5854 | Me(S) | single bond | H | c-Pr | C(=O)Me | CF₃ | CH₂OMe | O |
| C-5855 | H | CH₂ | Me | c-Pr | C(=O)Me | CF₃ | CH₂OMe | O |
| C-5856 | Me | CH₂ | H | c-Pr | C(=O)Me | CF₃ | CH₂OMe | O |
| C-5857 | H | CH(Me) | H | c-Pr | C(=O)Me | CF₃ | CH₂OMe | O |
| C-5858 | H | CH₂CH₂ | Me | c-Pr | C(=O)Me | CF₃ | CH₂OMe | O |
| C-5859 | Me | CH₂CH₂ | H | c-Pr | C(=O)Me | CF₃ | CH₂OMe | O |
| C-5860 | H | single bond | Me | c-Pr(1-CN) | C(=O)Me | CF₃ | CH₂OMe | O |
| C-5861 | Me | single bond | H | c-Pr(1-CN) | C(=O)Me | CF₃ | CH₂OMe | O |
| C-5862 | Me(R) | single bond | H | c-Pr(1-CN) | C(=O)Me | CF₃ | CH₂OMe | O |
| C-5863 | Me(S) | single bond | H | c-Pr(1-CN) | C(=O)Me | CF₃ | CH₂OMe | O |
| C-5864 | H | CH₂ | Me | c-Pr(1-CN) | C(=O)Me | CF₃ | CH₂OMe | O |
| C-5865 | Me | CH₂ | H | c-Pr(1-CN) | C(=O)Me | CF₃ | CH₂OMe | O |
| C-5866 | H | CH(Me) | H | c-Pr(1-CN) | C(=O)Me | CF₃ | CH₂OMe | O |
| C-5867 | H | CH₂CH₂ | Me | c-Pr(1-CN) | C(=O)Me | CF₃ | CH₂OMe | O |
| C-5868 | Me | CH₂CH₂ | H | c-Pr(1-CN) | C(=O)Me | CF₃ | CH₂OMe | O |
| C-5869 | H | single bond | Me | CH₂CF₃ | C(=O)Me | OMe | CH₂OMe | O |
| C-5870 | Me | single bond | H | CH₂CF₃ | C(=O)Me | OMe | CH₂OMe | O |
| C-5871 | Me(R) | single bond | H | CH₂CF₃ | C(=O)Me | OMe | CH₂OMe | O |
| C-5872 | Me(S) | single bond | H | CH₂CF₃ | C(=O)Me | OMe | CH₂OMe | O |
| C-5873 | H | CH₂ | Me | CH₂CF₃ | C(=O)Me | OMe | CH₂OMe | O |
| C-5874 | Me | CH₂ | H | CH₂CF₃ | C(=O)Me | OMe | CH₂OMe | O |
| C-5875 | H | CH(Me) | H | CH₂CF₃ | C(=O)Me | OMe | CH₂OMe | O |
| C-5876 | H | CH₂CH₂ | Me | CH₂CF₃ | C(=O)Me | OMe | CH₂OMe | O |
| C-5877 | Me | CH₂CH₂ | H | CH₂CF₃ | C(=O)Me | OMe | CH₂OMe | O |
| C-5878 | H | single bond | Me | Et | C(=O)Me | OMe | CH₂OMe | O |
| C-5879 | Me | single bond | H | Et | C(=O)Me | OMe | CH₂OMe | O |
| C-5880 | Me(R) | single bond | H | Et | C(=O)Me | OMe | CH₂OMe | O |
| C-5881 | Me(S) | single bond | H | Et | C(=O)Me | OMe | CH₂OMe | O |
| C-5882 | H | CH₂ | Me | Et | C(=O)Me | OMe | CH₂OMe | O |
| C-5883 | Me | CH₂ | H | Et | C(=O)Me | OMe | CH₂OMe | O |
| C-5884 | H | CH(Me) | H | Et | C(=O)Me | OMe | CH₂OMe | O |
| C-5885 | H | CH₂CH₂ | Me | Et | C(=O)Me | OMe | CH₂OMe | O |
| C-5886 | Me | CH₂CH₂ | H | Et | C(=O)Me | OMe | CH₂OMe | O |
| C-5887 | H | single bond | Me | c-Pr | C(=O)Me | OMe | CH₂OMe | O |
| C-5888 | Me | single bond | H | c-Pr | C(=O)Me | OMe | CH₂OMe | O |
| C-5889 | Me(R) | single bond | H | c-Pr | C(=O)Me | OMe | CH₂OMe | O |
| C-5890 | Me(S) | single bond | H | c-Pr | C(=O)Me | OMe | CH₂OMe | O |

**[Table 361]**

| Compound Number | Y | W | Z | R¹ | R² | R³ | R¹⁰ | A |
|---|---|---|---|---|---|---|---|---|
| C-5891 | H | CH₂ | Me | c-Pr | C(=O)Me | OMe | CH₂OMe | O |
| C-5892 | Me | CH₂ | H | c-Pr | C(=O)Me | OMe | CH₂OMe | O |
| C-5893 | H | CH(Me) | H | c-Pr | C(=O)Me | OMe | CH₂OMe | O |
| C-5894 | H | CH₂CH₂ | Me | c-Pr | C(=O)Me | OMe | CH₂OMe | O |
| C-5895 | Me | CH₂CH₂ | H | c-Pr | C(=O)Me | OMe | CH₂OMe | O |
| C-5896 | H | single bond | Me | c-Pr(1-CN) | C(=O)Me | OMe | CH₂OMe | O |
| C-5897 | Me | single bond | H | c-Pr(1-CN) | C(=O)Me | OMe | CH₂OMe | O |
| C-5898 | Me(R) | single bond | H | c-Pr(1-CN) | C(=O)Me | OMe | CH₂OMe | O |
| C-5899 | Me(S) | single bond | H | c-Pr(1-CN) | C(=O)Me | OMe | CH₂OMe | O |
| C-5900 | H | CH₂ | Me | c-Pr(1-CN) | C(=O)Me | OMe | CH₂OMe | O |
| C-5901 | Me | CH₂ | H | c-Pr(1-CN) | C(=O)Me | OMe | CH₂OMe | O |
| C-5902 | H | CH(Me) | H | c-Pr(1-CN) | C(=O)Me | OMe | CH₂OMe | O |
| C-5903 | H | CH₂CH₂ | Me | c-Pr(1-CN) | C(=O)Me | OMe | CH₂OMe | O |
| C-5904 | Me | CH₂CH₂ | H | c-Pr(1-CN) | C(=O)Me | OMe | CH₂OMe | O |
| C-5905 | H | single bond | Me | CH₂CF₃ | C(=O)Me | OCF₃ | CH₂OMe | O |
| C-5906 | Me | single bond | H | CH₂CF₃ | C(=O)Me | OCF₃ | CH₂OMe | O |
| C-5907 | Me(R) | single bond | H | CH₂CF₃ | C(=O)Me | OCF₃ | CH₂OMe | O |
| C-5908 | Me(S) | single bond | H | CH₂CF₃ | C(=O)Me | OCF₃ | CH₂OMe | O |
| C-5909 | H | CH₂ | Me | CH₂CF₃ | C(=O)Me | OCF₃ | CH₂OMe | O |
| C-5910 | Me | CH₂ | H | CH₂CF₃ | C(=O)Me | OCF₃ | CH₂OMe | O |
| C-5911 | H | CH(Me) | H | CH₂CF₃ | C(=O)Me | OCF₃ | CH₂OMe | O |
| C-5912 | H | CH₂CH₂ | Me | CH₂CF₃ | C(=O)Me | OCF₃ | CH₂OMe | O |
| C-5913 | Me | CH₂CH₂ | H | CH₂CF₃ | C(=O)Me | OCF₃ | CH₂OMe | O |
| C-5914 | H | single bond | Me | Et | C(=O)Me | OCF₃ | CH₂OMe | O |
| C-5915 | Me | single bond | H | Et | C(=O)Me | OCF₃ | CH₂OMe | O |
| C-5916 | Me(R) | single bond | H | Et | C(=O)Me | OCF₃ | CH₂OMe | O |
| C-5917 | Me(S) | single bond | H | Et | C(=O)Me | OCF₃ | CH₂OMe | O |
| C-5918 | H | CH₂ | Me | Et | C(=O)Me | OCF₃ | CH₂OMe | O |
| C-5919 | Me | CH₂ | H | Et | C(=O)Me | OCF₃ | CH₂OMe | O |
| C-5920 | H | CH(Me) | H | Et | C(=O)Me | OCF₃ | CH₂OMe | O |
| C-5921 | H | CH₂CH₂ | Me | Et | C(=O)Me | OCF₃ | CH₂OMe | O |
| C-5922 | Me | CH₂CH₂ | H | Et | C(=O)Me | OCF₃ | CH₂OMe | O |
| C-5923 | H | single bond | Me | c-Pr | C(=O)Me | OCF₃ | CH₂OMe | O |
| C-5924 | Me | single bond | H | c-Pr | C(=O)Me | OCF₃ | CH₂OMe | O |
| C-5925 | Me(R) | single bond | H | c-Pr | C(=O)Me | OCF₃ | CH₂OMe | O |
| C-5926 | Me(S) | single bond | H | c-Pr | C(=O)Me | OCF₃ | CH₂OMe | O |
| C-5927 | H | CH₂ | Me | c-Pr | C(=O)Me | OCF₃ | CH₂OMe | O |
| C-5928 | Me | CH₂ | H | c-Pr | C(=O)Me | OCF₃ | CH₂OMe | O |
| C-5929 | H | CH(Me) | H | c-Pr | C(=O)Me | OCF₃ | CH₂OMe | O |
| C-5930 | H | CH₂CH₂ | Me | c-Pr | C(=O)Me | OCF₃ | CH₂OMe | O |
| C-5931 | Me | CH₂CH₂ | H | c-Pr | C(=O)Me | OCF₃ | CH₂OMe | O |
| C-5932 | H | single bond | Me | c-Pr(1-CN) | C(=O)Me | OCF₃ | CH₂OMe | O |
| C-5933 | Me | single bond | H | c-Pr(1-CN) | C(=O)Me | OCF₃ | CH₂OMe | O |
| C-5934 | Me(R) | single bond | H | c-Pr(1-CN) | C(=O)Me | OCF₃ | CH₂OMe | O |

**[Table 362]**

| Compound Number | Y | W | Z | R¹ | R² | R³ | R¹⁰ | A |
|---|---|---|---|---|---|---|---|---|
| C-5935 | Me(S) | single bond | H | c-Pr(1-CN) | C(=O)Me | OCF₃ | CH₂OMe | O |
| C-5936 | H | CH₂ | Me | c-Pr(1-CN) | C(=O)Me | OCF₃ | CH₂OMe | O |
| C-5937 | Me | CH₂ | H | c-Pr(1-CN) | C(=O)Me | OCF₃ | CH₂OMe | O |
| C-5938 | H | CH(Me) | H | c-Pr(1-CN) | C(=O)Me | OCF₃ | CH₂OMe | O |
| C-5939 | H | CH₂CH₂ | Me | c-Pr(1-CN) | C(=O)Me | OCF₃ | CH₂OMe | O |
| C-5940 | Me | CH₂CH₂ | H | c-Pr(1-CN) | C(=O)Me | OCF₃ | CH₂OMe | O |
| C-5941 | H | single bond | Me | CH₂CF₃ | C(=O)Me | Br | CH₂CN | O |
| C-5942 | Me | single bond | H | CH₂CF₃ | C(=O)Me | Br | CH₂CN | O |
| C-5943 | Me(R) | single bond | H | CH₂CF₃ | C(=O)Me | Br | CH₂CN | O |
| C-5944 | Me(S) | single bond | H | CH₂CF₃ | C(=O)Me | Br | CH₂CN | O |
| C-5945 | H | CH₂ | Me | CH₂CF₃ | C(=O)Me | Br | CH₂CN | O |
| C-5946 | Me | CH₂ | H | CH₂CF₃ | C(=O)Me | Br | CH₂CN | O |
| C-5947 | H | CH(Me) | H | CH₂CF₃ | C(=O)Me | Br | CH₂CN | O |
| C-5948 | H | CH₂CH₂ | Me | CH₂CF₃ | C(=O)Me | Br | CH₂CN | O |
| C-5949 | Me | CH₂CH₂ | H | CH₂CF₃ | C(=O)Me | Br | CH₂CN | O |
| C-5950 | H | single bond | Me | Et | C(=O)Me | Br | CH₂CN | O |
| C-5951 | Me | single bond | H | Et | C(=O)Me | Br | CH₂CN | O |
| C-5952 | Me(R) | single bond | H | Et | C(=O)Me | Br | CH₂CN | O |
| C-5953 | Me(S) | single bond | H | Et | C(=O)Me | Br | CH₂CN | O |
| C-5954 | H | CH₂ | Me | Et | C(=O)Me | Br | CH₂CN | O |
| C-5955 | Me | CH₂ | H | Et | C(=O)Me | Br | CH₂CN | O |
| C-5956 | H | CH(Me) | H | Et | C(=O)Me | Br | CH₂CN | O |
| C-5957 | H | CH₂CH₂ | Me | Et | C(=O)Me | Br | CH₂CN | O |
| C-5958 | Me | CH₂CH₂ | H | Et | C(=O)Me | Br | CH₂CN | O |
| C-5959 | H | single bond | Me | c-Pr | C(=O)Me | Br | CH₂CN | O |
| C-5960 | Me | single bond | H | c-Pr | C(=O)Me | Br | CH₂CN | O |
| C-5961 | Me(R) | single bond | H | c-Pr | C(=O)Me | Br | CH₂CN | O |
| C-5962 | Me(S) | single bond | H | c-Pr | C(=O)Me | Br | CH₂CN | O |
| C-5963 | H | CH₂ | Me | c-Pr | C(=O)Me | Br | CH₂CN | O |
| C-5964 | Me | CH₂ | H | c-Pr | C(=O)Me | Br | CH₂CN | O |
| C-5965 | H | CH(Me) | H | c-Pr | C(=O)Me | Br | CH₂CN | O |
| C-5966 | H | CH₂CH₂ | Me | c-Pr | C(=O)Me | Br | CH₂CN | O |
| C-5967 | Me | CH₂CH₂ | H | c-Pr | C(=O)Me | Br | CH₂CN | O |
| C-5968 | H | single bond | Me | c-Pr(1-CN) | C(=O)Me | Br | CH₂CN | O |
| C-5969 | Me | single bond | H | c-Pr(1-CN) | C(=O)Me | Br | CH₂CN | O |
| C-5970 | Me(R) | single bond | H | c-Pr(1-CN) | C(=O)Me | Br | CH₂CN | O |
| C-5971 | Me(S) | single bond | H | c-Pr(1-CN) | C(=O)Me | Br | CH₂CN | O |
| C-5972 | H | CH₂ | Me | c-Pr(1-CN) | C(=O)Me | Br | CH₂CN | O |
| C-5973 | Me | CH₂ | H | c-Pr(1-CN) | C(=O)Me | Br | CH₂CN | O |
| C-5974 | H | CH(Me) | H | c-Pr(1-CN) | C(=O)Me | Br | CH₂CN | O |
| C-5975 | H | CH₂CH₂ | Me | c-Pr(1-CN) | C(=O)Me | Br | CH₂CN | O |
| C-5976 | Me | CH₂CH₂ | H | c-Pr(1-CN) | C(=O)Me | Br | CH₂CN | O |
| C-5977 | H | single bond | Me | CH₂CF₃ | C(=O)Me | CF₃ | CH₂CN | O |
| C-5978 | Me | single bond | H | CH₂CF₃ | C(=O)Me | CF₃ | CH₂CN | O |

**[Table 363]**

| Compound Number | Y | W | Z | R¹ | R² | R³ | R¹⁰ | A |
|---|---|---|---|---|---|---|---|---|
| C-5979 | Me(R) | single bond | H | CH₂CF₃ | C(=O)Me | CF₃ | CH₂CN | O |
| C-5980 | Me(S) | single bond | H | CH₂CF₃ | C(=O)Me | CF₃ | CH₂CN | O |
| C-5981 | H | CH₂ | Me | CH₂CF₃ | C(=O)Me | CF₃ | CH₂CN | O |
| C-5982 | Me | CH₂ | H | CH₂CF₃ | C(=O)Me | CF₃ | CH₂CN | O |
| C-5983 | H | CH(Me) | H | CH₂CF₃ | C(=O)Me | CF₃ | CH₂CN | O |
| C-5984 | H | CH₂CH₂ | Me | CH₂CF₃ | C(=O)Me | CF₃ | CH₂CN | O |
| C-5985 | Me | CH₂CH₂ | H | CH₂CF₃ | C(=O)Me | CF₃ | CH₂CN | O |
| C-5986 | H | single bond | Me | Et | C(=O)Me | CF₃ | CH₂CN | O |
| C-5987 | Me | single bond | H | Et | C(=O)Me | CF₃ | CH₂CN | O |
| C-5988 | Me(R) | single bond | H | Et | C(=O)Me | CF₃ | CH₂CN | O |
| C-5989 | Me(S) | single bond | H | Et | C(=O)Me | CF₃ | CH₂CN | O |
| C-5990 | H | CH₂ | Me | Et | C(=O)Me | CF₃ | CH₂CN | O |
| C-5991 | Me | CH₂ | H | Et | C(=O)Me | CF₃ | CH₂CN | O |
| C-5992 | H | CH(Me) | H | Et | C(=O)Me | CF₃ | CH₂CN | O |
| C-5993 | H | CH₂CH₂ | Me | Et | C(=O)Me | CF₃ | CH₂CN | O |
| C-5994 | Me | CH₂CH₂ | H | Et | C(=O)Me | CF₃ | CH₂CN | O |
| C-5995 | H | single bond | Me | c-Pr | C(=O)Me | CF₃ | CH₂CN | O |
| C-5996 | Me | single bond | H | c-Pr | C(=O)Me | CF₃ | CH₂CN | O |
| C-5997 | Me(R) | single bond | H | c-Pr | C(=O)Me | CF₃ | CH₂CN | O |
| C-5998 | Me(S) | single bond | H | c-Pr | C(=O)Me | CF₃ | CH₂CN | O |
| C-5999 | H | CH₂ | Me | c-Pr | C(=O)Me | CF₃ | CH₂CN | O |
| C-6000 | Me | CH₂ | H | c-Pr | C(=O)Me | CF₃ | CH₂CN | O |
| C-6001 | H | CH(Me) | H | c-Pr | C(=O)Me | CF₃ | CH₂CN | O |
| C-6002 | H | CH₂CH₂ | Me | c-Pr | C(=O)Me | CF₃ | CH₂CN | O |
| C-6003 | Me | CH₂CH₂ | H | c-Pr | C(=O)Me | CF₃ | CH₂CN | O |
| C-6004 | H | single bond | Me | c-Pr(1-CN) | C(=O)Me | CF₃ | CH₂CN | O |
| C-6005 | Me | single bond | H | c-Pr(1-CN) | C(=O)Me | CF₃ | CH₂CN | O |
| C-6006 | Me(R) | single bond | H | c-Pr(1-CN) | C(=O)Me | CF₃ | CH₂CN | O |
| C-6007 | Me(S) | single bond | H | c-Pr(1-CN) | C(=O)Me | CF₃ | CH₂CN | O |
| C-6008 | H | CH₂ | Me | c-Pr(1-CN) | C(=O)Me | CF₃ | CH₂CN | O |
| C-6009 | Me | CH₂ | H | c-Pr(1-CN) | C(=O)Me | CF₃ | CH₂CN | O |
| C-6010 | H | CH(Me) | H | c-Pr(1-CN) | C(=O)Me | CF₃ | CH₂CN | O |
| C-6011 | H | CH₂CH₂ | Me | c-Pr(1-CN) | C(=O)Me | CF₃ | CH₂CN | O |
| C-6012 | Me | CH₂CH₂ | H | c-Pr(1-CN) | C(=O)Me | CF₃ | CH₂CN | O |
| C-6013 | H | single bond | Me | CH₂CF₃ | C(=O)Me | OMe | CH₂CN | O |
| C-6014 | Me | single bond | H | CH₂CF₃ | C(=O)Me | OMe | CH₂CN | O |
| C-6015 | Me(R) | single bond | H | CH₂CF₃ | C(=O)Me | OMe | CH₂CN | O |
| C-6016 | Me(S) | single bond | H | CH₂CF₃ | C(=O)Me | OMe | CH₂CN | O |
| C-6017 | H | CH₂ | Me | CH₂CF₃ | C(=O)Me | OMe | CH₂CN | O |
| C-6018 | Me | CH₂ | H | CH₂CF₃ | C(=O)Me | OMe | CH₂CN | O |
| C-6019 | H | CH(Me) | H | CH₂CF₃ | C(=O)Me | OMe | CH₂CN | O |
| C-6020 | H | CH₂CH₂ | Me | CH₂CF₃ | C(=O)Me | OMe | CH₂CN | O |
| C-6021 | Me | CH₂CH₂ | H | CH₂CF₃ | C(=O)Me | OMe | CH₂CN | O |
| C-6022 | H | single bond | Me | Et | C(=O)Me | OMe | CH₂CN | O |

**[Table 364]**

| Compound Number | Y | W | Z | R¹ | R² | R³ | R¹⁰ | A |
|---|---|---|---|---|---|---|---|---|
| C-6023 | Me | single bond | H | Et | C(=O)Me | OMe | CH₂CN | O |
| C-6024 | Me(R) | single bond | H | Et | C(=O)Me | OMe | CH₂CN | O |
| C-6025 | Me(S) | single bond | H | Et | C(=O)Me | OMe | CH₂CN | O |
| C-6026 | H | CH₂ | Me | Et | C(=O)Me | OMe | CH₂CN | O |
| C-6027 | Me | CH₂ | H | Et | C(=O)Me | OMe | CH₂CN | O |
| C-6028 | H | CH(Me) | H | Et | C(=O)Me | OMe | CH₂CN | O |
| C-6029 | H | CH₂CH₂ | Me | Et | C(=O)Me | OMe | CH₂CN | O |
| C-6030 | Me | CH₂CH₂ | H | Et | C(=O)Me | OMe | CH₂CN | O |
| C-6031 | H | single bond | Me | c-Pr | C(=O)Me | OMe | CH₂CN | O |
| C-6032 | Me | single bond | H | c-Pr | C(=O)Me | OMe | CH₂CN | O |
| C-6033 | Me(R) | single bond | H | c-Pr | C(=O)Me | OMe | CH₂CN | O |
| C-6034 | Me(S) | single bond | H | c-Pr | C(=O)Me | OMe | CH₂CN | O |
| C-6035 | H | CH₂ | Me | c-Pr | C(=O)Me | OMe | CH₂CN | O |
| C-6036 | Me | CH₂ | H | c-Pr | C(=O)Me | OMe | CH₂CN | O |
| C-6037 | H | CH(Me) | H | c-Pr | C(=O)Me | OMe | CH₂CN | O |
| C-6038 | H | CH₂CH₂ | Me | c-Pr | C(=O)Me | OMe | CH₂CN | O |
| C-6039 | Me | CH₂CH₂ | H | c-Pr | C(=O)Me | OMe | CH₂CN | O |
| C-6040 | H | single bond | Me | c-Pr(1-CN) | C(=O)Me | OMe | CH₂CN | O |
| C-6041 | Me | single bond | H | c-Pr(1-CN) | C(=O)Me | OMe | CH₂CN | O |
| C-6042 | Me(R) | single bond | H | c-Pr(1-CN) | C(=O)Me | OMe | CH₂CN | O |
| C-6043 | Me(S) | single bond | H | c-Pr(1-CN) | C(=O)Me | OMe | CH₂CN | O |
| C-6044 | H | CH₂ | Me | c-Pr(1-CN) | C(=O)Me | OMe | CH₂CN | O |
| C-6045 | Me | CH₂ | H | c-Pr(1-CN) | C(=O)Me | OMe | CH₂CN | O |
| C-6046 | H | CH(Me) | H | c-Pr(1-CN) | C(=O)Me | OMe | CH₂CN | O |
| C-6047 | H | CH₂CH₂ | Me | c-Pr(1-CN) | C(=O)Me | OMe | CH₂CN | O |
| C-6048 | Me | CH₂CH₂ | H | c-Pr(1-CN) | C(=O)Me | OMe | CH₂CN | O |
| C-6049 | H | single bond | Me | CH₂CF₃ | C(=O)Me | OCF₃ | CH₂CN | O |
| C-6050 | Me | single bond | H | CH₂CF₃ | C(=O)Me | OCF₃ | CH₂CN | O |
| C-6051 | Me(R) | single bond | H | CH₂CF₃ | C(=O)Me | OCF₃ | CH₂CN | O |
| C-6052 | Me(S) | single bond | H | CH₂CF₃ | C(=O)Me | OCF₃ | CH₂CN | O |
| C-6053 | H | CH₂ | Me | CH₂CF₃ | C(=O)Me | OCF₃ | CH₂CN | O |
| C-6054 | Me | CH₂ | H | CH₂CF₃ | C(=O)Me | OCF₃ | CH₂CN | O |
| C-6055 | H | CH(Me) | H | CH₂CF₃ | C(=O)Me | OCF₃ | CH₂CN | O |
| C-6056 | H | CH₂CH₂ | Me | CH₂CF₃ | C(=O)Me | OCF₃ | CH₂CN | O |
| C-6057 | Me | CH₂CH₂ | H | CH₂CF₃ | C(=O)Me | OCF₃ | CH₂CN | O |
| C-6058 | H | single bond | Me | Et | C(=O)Me | OCF₃ | CH₂CN | O |
| C-6059 | Me | single bond | H | Et | C(=O)Me | OCF₃ | CH₂CN | O |
| C-6060 | Me(R) | single bond | H | Et | C(=O)Me | OCF₃ | CH₂CN | O |
| C-6061 | Me(S) | single bond | H | Et | C(=O)Me | OCF₃ | CH₂CN | O |
| C-6062 | H | CH₂ | Me | Et | C(=O)Me | OCF₃ | CH₂CN | O |
| C-6063 | Me | CH₂ | H | Et | C(=O)Me | OCF₃ | CH₂CN | O |
| C-6064 | H | CH(Me) | H | Et | C(=O)Me | OCF₃ | CH₂CN | O |
| C-6065 | H | CH₂CH₂ | Me | Et | C(=O)Me | OCF₃ | CH₂CN | O |
| C-6066 | Me | CH₂CH₂ | H | Et | C(=O)Me | OCF₃ | CH₂CN | O |

**[Table 365]**

| Compound Number | Y | W | Z | R¹ | R² | R³ | R¹⁰ | A |
|---|---|---|---|---|---|---|---|---|
| C-6067 | H | single bond | Me | c-Pr | C(=O)Me | OCF₃ | CH₂CN | O |
| C-6068 | Me | single bond | H | c-Pr | C(=O)Me | OCF₃ | CH₂CN | O |
| C-6069 | Me(R) | single bond | H | c-Pr | C(=O)Me | OCF₃ | CH₂CN | O |
| C-6070 | Me(S) | single bond | H | c-Pr | C(=O)Me | OCF₃ | CH₂CN | O |
| C-6071 | H | CH₂ | Me | c-Pr | C(=O)Me | OCF₃ | CH₂CN | O |
| C-6072 | Me | CH₂ | H | c-Pr | C(=O)Me | OCF₃ | CH₂CN | O |
| C-6073 | H | CH(Me) | H | c-Pr | C(=O)Me | OCF₃ | CH₂CN | O |
| C-6074 | H | CH₂CH₂ | Me | c-Pr | C(=O)Me | OCF₃ | CH₂CN | O |
| C-6075 | Me | CH₂CH₂ | H | c-Pr | C(=O)Me | OCF₃ | CH₂CN | O |
| C-6076 | H | single bond | Me | c-Pr(1-CN) | C(=O)Me | OCF₃ | CH₂CN | O |
| C-6077 | Me | single bond | H | c-Pr(1-CN) | C(=O)Me | OCF₃ | CH₂CN | O |
| C-6078 | Me(R) | single bond | H | c-Pr(1-CN) | C(=O)Me | OCF₃ | CH₂CN | O |
| C-6079 | Me(S) | single bond | H | c-Pr(1-CN) | C(=O)Me | OCF₃ | CH₂CN | O |
| C-6080 | H | CH₂ | Me | c-Pr(1-CN) | C(=O)Me | OCF₃ | CH₂CN | O |
| C-6081 | Me | CH₂ | H | c-Pr(1-CN) | C(=O)Me | OCF₃ | CH₂CN | O |
| C-6082 | H | CH(Me) | H | c-Pr(1-CN) | C(=O)Me | OCF₃ | CH₂CN | O |
| C-6083 | H | CH₂CH₂ | Me | c-Pr(1-CN) | C(=O)Me | OCF₃ | CH₂CN | O |
| C-6084 | Me | CH₂CH₂ | H | c-Pr(1-CN) | C(=O)Me | OCF₃ | CH₂CN | O |

**[Table 366]**

| 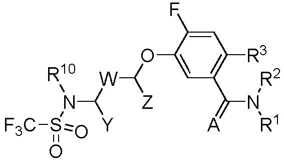 | | | | | | | | |
|---|---|---|---|---|---|---|---|---|
| Compound Number | Y | W | Z | R¹ | R² | R³ | R¹⁰ | A |
| C-6085 | H | single bond | Me | CH₂CF₃ | C(=O)OCH₂CH=CH₂ | Cl | H | O |
| C-6086 | Me | single bond | H | CH₂CF₃ | C(=O)OCH₂CH=CH₂ | Cl | H | O |
| C-6087 | Me(R) | single bond | H | CH₂CF₃ | C(=O)OCH₂CH=CH₂ | Cl | H | O |
| C-6088 | Me(S) | single bond | H | CH₂CF₃ | C(=O)OCH₂CH=CH₂ | Cl | H | O |
| C-6089 | H | CH₂ | Me | CH₂CF₃ | C(=O)OCH₂CH=CH₂ | Cl | H | O |
| C-6090 | Me | CH₂ | H | CH₂CF₃ | C(=O)OCH₂CH=CH₂ | Cl | H | O |
| C-6091 | H | CH(Me) | H | CH₂CF₃ | C(=O)OCH₂CH=CH₂ | Cl | H | O |
| C-6092 | H | CH₂CH₂ | Me | CH₂CF₃ | C(=O)OCH₂CH=CH₂ | Cl | H | O |
| C-6093 | Me | CH₂CH₂ | H | CH₂CF₃ | C(=O)OCH₂CH=CH₂ | Cl | H | O |
| C-6094 | H | single bond | Me | CH₂CF₃ | C(=O)OCH₂CH=CH₂ | Me | H | O |
| C-6095 | Me | single bond | H | CH₂CF₃ | C(=O)OCH₂CH=CH₂ | Me | H | O |
| C-6096 | Me(R) | single bond | H | CH₂CF₃ | C(=O)OCH₂CH=CH₂ | Me | H | O |
| C-6097 | Me(S) | single bond | H | CH₂CF₃ | C(=O)OCH₂CH=CH₂ | Me | H | O |
| C-6098 | H | CH₂ | Me | CH₂CF₃ | C(=O)OCH₂CH=CH₂ | Me | H | O |
| C-6099 | Me | CH₂ | H | CH₂CF₃ | C(=O)OCH₂CH=CH₂ | Me | H | O |
| C-6100 | H | CH(Me) | H | CH₂CF₃ | C(=O)OCH₂CH=CH₂ | Me | H | O |
| C-6101 | H | CH₂CH₂ | Me | CH₂CF₃ | C(=O)OCH₂CH=CH₂ | Me | H | O |
| C-6102 | Me | CH₂CH₂ | H | CH₂CF₃ | C(=O)OCH₂CH=CH₂ | Me | H | O |
| C-6103 | H | single bond | Me | CH₂CF₃ | C(=O)OCH₂CCl₃ | Cl | H | O |
| C-6104 | Me | single bond | H | CH₂CF₃ | C(=O)OCH₂CCl₃ | Cl | H | O |
| C-6105 | Me(R) | single bond | H | CH₂CF₃ | C(=O)OCH₂CCl₃ | Cl | H | O |
| C-6106 | Me(S) | single bond | H | CH₂CF₃ | C(=O)OCH₂CCl₃ | Cl | H | O |
| C-6107 | H | CH₂ | Me | CH₂CF₃ | C(=O)OCH₂CCl₃ | Cl | H | O |
| C-6108 | Me | CH₂ | H | CH₂CF₃ | C(=O)OCH₂CCl₃ | Cl | H | O |
| C-6109 | H | CH(Me) | H | CH₂CF₃ | C(=O)OCH₂CCl₃ | Cl | H | O |
| C-6110 | H | CH₂CH₂ | Me | CH₂CF₃ | C(=O)OCH₂CCl₃ | Cl | H | O |
| C-6111 | Me | CH₂CH₂ | H | CH₂CF₃ | C(=O)OCH₂CCl₃ | Cl | H | O |
| C-6112 | H | single bond | Me | CH₂CF₃ | C(=O)OCH₂CCl₃ | Me | H | O |
| C-6113 | Me | single bond | H | CH₂CF₃ | C(=O)OCH₂CCl₃ | Me | H | O |
| C-6114 | Me(R) | single bond | H | CH₂CF₃ | C(=O)OCH₂CCl₃ | Me | H | O |
| C-6115 | Me(S) | single bond | H | CH₂CF₃ | C(=O)OCH₂CCl₃ | Me | H | O |
| C-6116 | H | CH₂ | Me | CH₂CF₃ | C(=O)OCH₂CCl₃ | Me | H | O |
| C-6117 | Me | CH₂ | H | CH₂CF₃ | C(=O)OCH₂CCl₃ | Me | H | O |
| C-6118 | H | CH(Me) | H | CH₂CF₃ | C(=O)OCH₂CCl₃ | Me | H | O |
| C-6119 | H | CH₂CH₂ | Me | CH₂CF₃ | C(=O)OCH₂CCl₃ | Me | H | O |
| C-6120 | Me | CH₂CH₂ | H | CH₂CF₃ | C(=O)OCH₂CCl₃ | Me | H | O |
| C-6121 | H | single bond | Me | CH₂CF₃ | C(=O)N(Me)₂ | Cl | H | O |
| C-6122 | Me | single bond | H | CH₂CF₃ | C(=O)N(Me)₂ | Cl | H | O |

**[Table 367]**

| Compound Number | Y | W | Z | R¹ | R² | R³ | R¹⁰ | A |
|---|---|---|---|---|---|---|---|---|
| C-6123 | Me(R) | single bond | H | CH₂CF₃ | C(=O)N(Me)₂ | Cl | H | O |
| C-6124 | Me(S) | single bond | H | CH₂CF₃ | C(=O)N(Me)₂ | Cl | H | O |
| C-6125 | H | CH₂ | Me | CH₂CF₃ | C(=O)N(Me)₂ | Cl | H | O |
| C-6126 | Me | CH₂ | H | CH₂CF₃ | C(=O)N(Me)₂ | Cl | H | O |
| C-6127 | H | CH(Me) | H | CH₂CF₃ | C(=O)N(Me)₂ | Cl | H | O |
| C-6128 | H | CH₂CH₂ | Me | CH₂CF₃ | C(=O)N(Me)₂ | Cl | H | O |
| C-6129 | Me | CH₂CH₂ | H | CH₂CF₃ | C(=O)N(Me)₂ | Cl | H | O |
| C-6130 | H | single bond | Me | CH₂CF₃ | C(=O)N(Me)₂ | Me | H | O |
| C-6131 | Me | single bond | H | CH₂CF₃ | C(=O)N(Me)₂ | Me | H | O |
| C-6132 | Me(R) | single bond | H | CH₂CF₃ | C(=O)N(Me)₂ | Me | H | O |
| C-6133 | Me(S) | single bond | H | CH₂CF₃ | C(=O)N(Me)₂ | Me | H | O |
| C-6134 | H | CH₂ | Me | CH₂CF₃ | C(=O)N(Me)₂ | Me | H | O |
| C-6135 | Me | CH₂ | H | CH₂CF₃ | C(=O)N(Me)₂ | Me | H | O |
| C-6136 | H | CH(Me) | H | CH₂CF₃ | C(=O)N(Me)₂ | Me | H | O |
| C-6137 | H | CH₂CH₂ | Me | CH₂CF₃ | C(=O)N(Me)₂ | Me | H | O |
| C-6138 | Me | CH₂CH₂ | H | CH₂CF₃ | C(=O)N(Me)₂ | Me | H | O |
| C-6139 | H | single bond | Me | CH₂CF₃ | CH₂OC(=O)Me | Cl | H | O |
| C-6140 | Me | single bond | H | CH₂CF₃ | CH₂OC(=O)Me | Cl | H | O |
| C-6141 | Me(R) | single bond | H | CH₂CF₃ | CH₂OC(=O)Me | Cl | H | O |
| C-6142 | Me(S) | single bond | H | CH₂CF₃ | CH₂OC(=O)Me | Cl | H | O |
| C-6143 | H | CH₂ | Me | CH₂CF₃ | CH₂OC(=O)Me | Cl | H | O |
| C-6144 | Me | CH₂ | H | CH₂CF₃ | CH₂OC(=O)Me | Cl | H | O |
| C-6145 | H | CH(Me) | H | CH₂CF₃ | CH₂OC(=O)Me | Cl | H | O |
| C-6146 | H | CH₂CH₂ | Me | CH₂CF₃ | CH₂OC(=O)Me | Cl | H | O |
| C-6147 | Me | CH₂CH₂ | H | CH₂CF₃ | CH₂OC(=O)Me | Cl | H | O |
| C-6148 | H | single bond | Me | CH₂CF₃ | CH₂OC(=O)Me | Me | H | O |
| C-6149 | Me | single bond | H | CH₂CF₃ | CH₂OC(=O)Me | Me | H | O |
| C-6150 | Me(R) | single bond | H | CH₂CF₃ | CH₂OC(=O)Me | Me | H | O |
| C-6151 | Me(S) | single bond | H | CH₂CF₃ | CH₂OC(=O)Me | Me | H | O |
| C-6152 | H | CH₂ | Me | CH₂CF₃ | CH₂OC(=O)Me | Me | H | O |
| C-6153 | Me | CH₂ | H | CH₂CF₃ | CH₂OC(=O)Me | Me | H | O |
| C-6154 | H | CH(Me) | H | CH₂CF₃ | CH₂OC(=O)Me | Me | H | O |
| C-6155 | H | CH₂CH₂ | Me | CH₂CF₃ | CH₂OC(=O)Me | Me | H | O |
| C-6156 | Me | CH₂CH₂ | H | CH₂CF₃ | CH₂OC(=O)Me | Me | H | O |
| C-6157 | H | single bond | Me | CH₂CF₃ | CH₂OC(=O)(*i*-Pr) | Cl | H | O |
| C-6158 | Me | single bond | H | CH₂CF₃ | CH₂OC(=O)(*i*-Pr) | Cl | H | O |
| C-6159 | Me(R) | single bond | H | CH₂CF₃ | CH₂OC(=O)(*i*-Pr) | Cl | H | O |
| C-6160 | Me(S) | single bond | H | CH₂CF₃ | CH₂OC(=O)(*i*-Pr) | Cl | H | O |
| C-6161 | H | CH₂ | Me | CH₂CF₃ | CH₂OC(=O)(*i*-Pr) | Cl | H | O |
| C-6162 | Me | CH₂ | H | CH₂CF₃ | CH₂OC(=O)(*i*-Pr) | Cl | H | O |
| C-6163 | H | CH(Me) | H | CH₂CF₃ | CH₂OC(=O)(*i*-Pr) | Cl | H | O |
| C-6164 | H | CH₂CH₂ | Me | CH₂CF₃ | CH₂OC(=O)(*i*-Pr) | Cl | H | O |
| C-6165 | Me | CH₂CH₂ | H | CH₂CF₃ | CH₂OC(=O)(*i*-Pr) | Cl | H | O |
| C-6166 | H | single bond | Me | CH₂CF₃ | CH₂OC(=O)(*i*-Pr) | Me | H | O |

**[Table 368]**

| Compound Number | Y | W | Z | R¹ | R² | R³ | R¹⁰ | A |
|---|---|---|---|---|---|---|---|---|
| C-6167 | Me | single bond | H | CH₂CF₃ | CH₂OC(=O)(*i*-Pr) | Me | H | O |
| C-6168 | Me(R) | single bond | H | CH₂CF₃ | CH₂OC(=O)(*i*-Pr) | Me | H | O |
| C-6169 | Me(S) | single bond | H | CH₂CF₃ | CH₂OC(=O)(*i*-Pr) | Me | H | O |
| C-6170 | H | CH₂ | Me | CH₂CF₃ | CH₂OC(=O)(*i*-Pr) | Me | H | O |
| C-6171 | Me | CH₂ | H | CH₂CF₃ | CH₂OC(=O)(*i*-Pr) | Me | H | O |
| C-6172 | H | CH(Me) | H | CH₂CF₃ | CH₂OC(=O)(*i*-Pr) | Me | H | O |
| C-6173 | H | CH₂CH₂ | Me | CH₂CF₃ | CH₂OC(=O)(*i*-Pr) | Me | H | O |
| C-6174 | Me | CH₂CH₂ | H | CH₂CF₃ | CH₂OC(=O)(*i*-Pr) | Me | H | O |
| C-6175 | H | single bond | Me | CH₂CF₃ | CH₂OC(=O)(*t*-Bu) | Cl | H | O |
| C-6176 | Me | single bond | H | CH₂CF₃ | CH₂OC(=O)(*t*-Bu) | Cl | H | O |
| C-6177 | Me(R) | single bond | H | CH₂CF₃ | CH₂OC(=O)(*t*-Bu) | Cl | H | O |
| C-6178 | Me(S) | single bond | H | CH₂CF₃ | CH₂OC(=O)(*t*-Bu) | Cl | H | O |
| C-6179 | H | CH₂ | Me | CH₂CF₃ | CH₂OC(=O)(*t*-Bu) | Cl | H | O |
| C-6180 | Me | CH₂ | H | CH₂CF₃ | CH₂OC(=O)(*t*-Bu) | Cl | H | O |
| C-6181 | H | CH(Me) | H | CH₂CF₃ | CH₂OC(=O)(*t*-Bu) | Cl | H | O |
| C-6182 | H | CH₂CH₂ | Me | CH₂CF₃ | CH₂OC(=O)(*t*-Bu) | Cl | H | O |
| C-6183 | Me | CH₂CH₂ | H | CH₂CF₃ | CH₂OC(=O)(*t*-Bu) | Cl | H | O |
| C-6184 | H | single bond | Me | CH₂CF₃ | CH₂OC(=O)(*t*-Bu) | Me | H | O |
| C-6185 | Me | single bond | H | CH₂CF₃ | CH₂OC(=O)(*t*-Bu) | Me | H | O |
| C-6186 | Me(R) | single bond | H | CH₂CF₃ | CH₂OC(=O)(*t*-Bu) | Me | H | O |
| C-6187 | Me(S) | single bond | H | CH₂CF₃ | CH₂OC(=O)(*t*-Bu) | Me | H | O |
| C-6188 | H | CH₂ | Me | CH₂CF₃ | CH₂OC(=O)(*t*-Bu) | Me | H | O |
| C-6189 | Me | CH₂ | H | CH₂CF₃ | CH₂OC(=O)(*t*-Bu) | Me | H | O |
| C-6190 | H | CH(Me) | H | CH₂CF₃ | CH₂OC(=O)(*t*-Bu) | Me | H | O |
| C-6191 | H | CH₂CH₂ | Me | CH₂CF₃ | CH₂OC(=O)(*t*-Bu) | Me | H | O |
| C-6192 | Me | CH₂CH₂ | H | CH₂CF₃ | CH₂OC(=O)(*t*-Bu) | Me | H | O |
| C-6193 | H | single bond | Me | CH₂CF₃ | CH₂OC(=O)OEt | Cl | H | O |
| C-6194 | Me | single bond | H | CH₂CF₃ | CH₂OC(=O)OEt | Cl | H | O |
| C-6195 | Me(R) | single bond | H | CH₂CF₃ | CH₂OC(=O)OEt | Cl | H | O |
| C-6196 | Me(S) | single bond | H | CH₂CF₃ | CH₂OC(=O)OEt | Cl | H | O |
| C-6197 | H | CH₂ | Me | CH₂CF₃ | CH₂OC(=O)OEt | Cl | H | O |
| C-6198 | Me | CH₂ | H | CH₂CF₃ | CH₂OC(=O)OEt | Cl | H | O |
| C-6199 | H | CH(Me) | H | CH₂CF₃ | CH₂OC(=O)OEt | Cl | H | O |
| C-6200 | H | CH₂CH₂ | Me | CH₂CF₃ | CH₂OC(=O)OEt | Cl | H | O |
| C-6201 | Me | CH₂CH₂ | H | CH₂CF₃ | CH₂OC(=O)OEt | Cl | H | O |
| C-6202 | H | single bond | Me | CH₂CF₃ | CH₂OC(=O)OEt | Me | H | O |
| C-6203 | Me | single bond | H | CH₂CF₃ | CH₂OC(=O)OEt | Me | H | O |
| C-6204 | Me(R) | single bond | H | CH₂CF₃ | CH₂OC(=O)OEt | Me | H | O |
| C-6205 | Me(S) | single bond | H | CH₂CF₃ | CH₂OC(=O)OEt | Me | H | O |
| C-6206 | H | CH₂ | Me | CH₂CF₃ | CH₂OC(=O)OEt | Me | H | O |
| C-6207 | Me | CH₂ | H | CH₂CF₃ | CH₂OC(=O)OEt | Me | H | O |
| C-6208 | H | CH(Me) | H | CH₂CF₃ | CH₂OC(=O)OEt | Me | H | O |
| C-6209 | H | CH₂CH₂ | Me | CH₂CF₃ | CH₂OC(=O)OEt | Me | H | O |
| C-6210 | Me | CH₂CH₂ | H | CH₂CF₃ | CH₂OC(=O)OEt | Me | H | O |

**[Table 369]**

| Compound Number | Y | W | Z | R¹ | R² | R³ | R¹⁰ | A |
|---|---|---|---|---|---|---|---|---|
| C-6211 | H | single bond | Me | CH₂CF₃ | CH₂OC(=O)O(*i*-Pr) | Cl | H | O |
| C-6212 | Me | single bond | H | CH₂CF₃ | CH₂OC(=O)O(*i*-Pr) | Cl | H | O |
| C-6213 | Me(R) | single bond | H | CH₂CF₃ | CH₂OC(=O)O(*i*-Pr) | Cl | H | O |
| C-6214 | Me(S) | single bond | H | CH₂CF₃ | CH₂OC(=O)O(*i*-Pr) | Cl | H | O |
| C-6215 | H | CH₂ | Me | CH₂CF₃ | CH₂OC(=O)O(*i*-Pr) | Cl | H | O |
| C-6216 | Me | CH₂ | H | CH₂CF₃ | CH₂OC(=O)O(*i*-Pr) | Cl | H | O |
| C-6217 | H | CH(Me) | H | CH₂CF₃ | CH₂OC(=O)O(*i*-Pr) | Cl | H | O |
| C-6218 | H | CH₂CH₂ | Me | CH₂CF₃ | CH₂OC(=O)O(*i*-Pr) | Cl | H | O |
| C-6219 | Me | CH₂CH₂ | H | CH₂CF₃ | CH₂OC(=O)O(*i*-Pr) | Cl | H | O |
| C-6220 | H | single bond | Me | CH₂CF₃ | CH₂OC(=O)O(*i*-Pr) | Me | H | O |
| C-6221 | Me | single bond | H | CH₂CF₃ | CH₂OC(=O)O(*i*-Pr) | Me | H | O |
| C-6222 | Me(R) | single bond | H | CH₂CF₃ | CH₂OC(=O)O(*i*-Pr) | Me | H | O |
| C-6223 | Me(S) | single bond | H | CH₂CF₃ | CH₂OC(=O)O(*i*-Pr) | Me | H | O |
| C-6224 | H | CH₂ | Me | CH₂CF₃ | CH₂OC(=O)O(*i*-Pr) | Me | H | O |
| C-6225 | Me | CH₂ | H | CH₂CF₃ | CH₂OC(=O)O(*i*-Pr) | Me | H | O |
| C-6226 | H | CH(Me) | H | CH₂CF₃ | CH₂OC(=O)O(*i*-Pr) | Me | H | O |
| C-6227 | H | CH₂CH₂ | Me | CH₂CF₃ | CH₂OC(=O)O(*i*-Pr) | Me | H | O |
| C-6228 | Me | CH₂CH₂ | H | CH₂CF₃ | CH₂OC(=O)O(*i*-Pr) | Me | H | O |
| C-6229 | H | single bond | Me | CH₂CF₃ | CH₂N(Me)C(=O)OMe | Cl | H | O |
| C-6230 | Me | single bond | H | CH₂CF₃ | CH₂N(Me)C(=O)OMe | Cl | H | O |
| C-6231 | Me(R) | single bond | H | CH₂CF₃ | CH₂N(Me)C(=O)OMe | Cl | H | O |
| C-6232 | Me(S) | single bond | H | CH₂CF₃ | CH₂N(Me)C(=O)OMe | Cl | H | O |
| C-6233 | H | CH₂ | Me | CH₂CF₃ | CH₂N(Me)C(=O)OMe | Cl | H | O |
| C-6234 | Me | CH₂ | H | CH₂CF₃ | CH₂N(Me)C(=O)OMe | Cl | H | O |
| C-6235 | H | CH(Me) | H | CH₂CF₃ | CH₂N(Me)C(=O)OMe | Cl | H | O |
| C-6236 | H | CH₂CH₂ | Me | CH₂CF₃ | CH₂N(Me)C(=O)OMe | Cl | H | O |
| C-6237 | Me | CH₂CH₂ | H | CH₂CF₃ | CH₂N(Me)C(=O)OMe | Cl | H | O |
| C-6238 | H | single bond | Me | CH₂CF₃ | CH₂N(Me)C(=O)OMe | Me | H | O |
| C-6239 | Me | single bond | H | CH₂CF₃ | CH₂N(Me)C(=O)OMe | Me | H | O |
| C-6240 | Me(R) | single bond | H | CH₂CF₃ | CH₂N(Me)C(=O)OMe | Me | H | O |
| C-6241 | Me(S) | single bond | H | CH₂CF₃ | CH₂N(Me)C(=O)OMe | Me | H | O |
| C-6242 | H | CH₂ | Me | CH₂CF₃ | CH₂N(Me)C(=O)OMe | Me | H | O |
| C-6243 | Me | CH₂ | H | CH₂CF₃ | CH₂N(Me)C(=O)OMe | Me | H | O |
| C-6244 | H | CH(Me) | H | CH₂CF₃ | CH₂N(Me)C(=O)OMe | Me | H | O |
| C-6245 | H | CH₂CH₂ | Me | CH₂CF₃ | CH₂N(Me)C(=O)OMe | Me | H | O |
| C-6246 | Me | CH₂CH₂ | H | CH₂CF₃ | CH₂N(Me)C(=O)OMe | Me | H | O |
| C-6247 | H | single bond | Me | CH₂CF₃ | C(=O)OCH₂CH=CH₂ | Cl | Me | O |
| C-6248 | Me | single bond | H | CH₂CF₃ | C(=O)OCH₂CH=CH₂ | Cl | Me | O |
| C-6249 | Me(R) | single bond | H | CH₂CF₃ | C(=O)OCH₂CH=CH₂ | Cl | Me | O |
| C-6250 | Me(S) | single bond | H | CH₂CF₃ | C(=O)OCH₂CH=CH₂ | Cl | Me | O |
| C-6251 | H | CH₂ | Me | CH₂CF₃ | C(=O)OCH₂CH=CH₂ | Cl | Me | O |
| C-6252 | Me | CH₂ | H | CH₂CF₃ | C(=O)OCH₂CH=CH₂ | Cl | Me | O |
| C-6253 | H | CH(Me) | H | CH₂CF₃ | C(=O)OCH₂CH=CH₂ | Cl | Me | O |
| C-6254 | H | CH₂CH₂ | Me | CH₂CF₃ | C(=O)OCH₂CH=CH₂ | Cl | Me | O |

**[Table 370]**

| Compound Number | Y | W | Z | R¹ | R² | R³ | R¹⁰ | A |
|---|---|---|---|---|---|---|---|---|
| C-6255 | Me | CH₂CH₂ | H | CH₂CF₃ | C(=O)OCH₂CH=CH₂ | Cl | Me | O |
| C-6256 | H | single bond | Me | CH₂CF₃ | C(=O)OCH₂CH=CH₂ | Me | Me | O |
| C-6257 | Me | single bond | H | CH₂CF₃ | C(=O)OCH₂CH=CH₂ | Me | Me | O |
| C-6258 | Me(R) | single bond | H | CH₂CF₃ | C(=O)OCH₂CH=CH₂ | Me | Me | O |
| C-6259 | Me(S) | single bond | H | CH₂CF₃ | C(=O)OCH₂CH=CH₂ | Me | Me | O |
| C-6260 | H | CH₂ | Me | CH₂CF₃ | C(=O)OCH₂CH=CH₂ | Me | Me | O |
| C-6261 | Me | CH₂ | H | CH₂CF₃ | C(=O)OCH₂CH=CH₂ | Me | Me | O |
| C-6262 | H | CH(Me) | H | CH₂CF₃ | C(=O)OCH₂CH=CH₂ | Me | Me | O |
| C-6263 | H | CH₂CH₂ | Me | CH₂CF₃ | C(=O)OCH₂CH=CH₂ | Me | Me | O |
| C-6264 | Me | CH₂CH₂ | H | CH₂CF₃ | C(=O)OCH₂CH=CH₂ | Me | Me | O |
| C-6265 | H | single bond | Me | CH₂CF₃ | C(=O)OCH₂CCl₃ | Cl | Me | O |
| C-6266 | Me | single bond | H | CH₂CF₃ | C(=O)OCH₂CCl₃ | Cl | Me | O |
| C-6267 | Me(R) | single bond | H | CH₂CF₃ | C(=O)OCH₂CCl₃ | Cl | Me | O |
| C-6268 | Me(S) | single bond | H | CH₂CF₃ | C(=O)OCH₂CCl₃ | Cl | Me | O |
| C-6269 | H | CH₂ | Me | CH₂CF₃ | C(=O)OCH₂CCl₃ | Cl | Me | O |
| C-6270 | Me | CH₂ | H | CH₂CF₃ | C(=O)OCH₂CCl₃ | Cl | Me | O |
| C-6271 | H | CH(Me) | H | CH₂CF₃ | C(=O)OCH₂CCl₃ | Cl | Me | O |
| C-6272 | H | CH₂CH₂ | Me | CH₂CF₃ | C(=O)OCH₂CCl₃ | Cl | Me | O |
| C-6273 | Me | CH₂CH₂ | H | CH₂CF₃ | C(=O)OCH₂CCl₃ | Cl | Me | O |
| C-6274 | H | single bond | Me | CH₂CF₃ | C(=O)OCH₂CCl₃ | Me | Me | O |
| C-6275 | Me | single bond | H | CH₂CF₃ | C(=O)OCH₂CCl₃ | Me | Me | O |
| C-6276 | Me(R) | single bond | H | CH₂CF₃ | C(=O)OCH₂CCl₃ | Me | Me | O |
| C-6277 | Me(S) | single bond | H | CH₂CF₃ | C(=O)OCH₂CCl₃ | Me | Me | O |
| C-6278 | H | CH₂ | Me | CH₂CF₃ | C(=O)OCH₂CCl₃ | Me | Me | O |
| C-6279 | Me | CH₂ | H | CH₂CF₃ | C(=O)OCH₂CCl₃ | Me | Me | O |
| C-6280 | H | CH(Me) | H | CH₂CF₃ | C(=O)OCH₂CCl₃ | Me | Me | O |
| C-6281 | H | CH₂CH₂ | Me | CH₂CF₃ | C(=O)OCH₂CCl₃ | Me | Me | O |
| C-6282 | Me | CH₂CH₂ | H | CH₂CF₃ | C(=O)OCH₂CCl₃ | Me | Me | O |
| C-6283 | H | single bond | Me | CH₂CF₃ | C(=O)N(Me)₂ | Cl | Me | O |
| C-6284 | Me | single bond | H | CH₂CF₃ | C(=O)N(Me)₂ | Cl | Me | O |
| C-6285 | Me(R) | single bond | H | CH₂CF₃ | C(=O)N(Me)₂ | Cl | Me | O |
| C-6286 | Me(S) | single bond | H | CH₂CF₃ | C(=O)N(Me)₂ | Cl | Me | O |
| C-6287 | H | CH₂ | Me | CH₂CF₃ | C(=O)N(Me)₂ | Cl | Me | O |
| C-6288 | Me | CH₂ | H | CH₂CF₃ | C(=O)N(Me)₂ | Cl | Me | O |
| C-6289 | H | CH(Me) | H | CH₂CF₃ | C(=O)N(Me)₂ | Cl | Me | O |
| C-6290 | H | CH₂CH₂ | Me | CH₂CF₃ | C(=O)N(Me)₂ | Cl | Me | O |
| C-6291 | Me | CH₂CH₂ | H | CH₂CF₃ | C(=O)N(Me)₂ | Cl | Me | O |
| C-6292 | H | single bond | Me | CH₂CF₃ | C(=O)N(Me)₂ | Me | Me | O |
| C-6293 | Me | single bond | H | CH₂CF₃ | C(=O)N(Me)₂ | Me | Me | O |
| C-6294 | Me(R) | single bond | H | CH₂CF₃ | C(=O)N(Me)₂ | Me | Me | O |
| C-6295 | Me(S) | single bond | H | CH₂CF₃ | C(=O)N(Me)₂ | Me | Me | O |
| C-6296 | H | CH₂ | Me | CH₂CF₃ | C(=O)N(Me)₂ | Me | Me | O |
| C-6297 | Me | CH₂ | H | CH₂CF₃ | C(=O)N(Me)₂ | Me | Me | O |
| C-6298 | H | CH(Me) | H | CH₂CF₃ | C(=O)N(Me)₂ | Me | Me | O |

**[Table 371]**

| Compound Number | Y | W | Z | R¹ | R² | R³ | R¹⁰ | A |
|---|---|---|---|---|---|---|---|---|
| C-6299 | H | CH₂CH₂ | Me | CH₂CF₃ | C(=O)N(Me)₂ | Me | Me | O |
| C-6300 | Me | CH₂CH₂ | H | CH₂CF₃ | C(=O)N(Me)₂ | Me | Me | O |
| C-6301 | H | single bond | Me | CH₂CF₃ | CH₂OC(=O)Me | Cl | Me | O |
| C-6302 | Me | single bond | H | CH₂CF₃ | CH₂OC(=O)Me | Cl | Me | O |
| C-6303 | Me(R) | single bond | H | CH₂CF₃ | CH₂OC(=O)Me | Cl | Me | O |
| C-6304 | Me(S) | single bond | H | CH₂CF₃ | CH₂OC(=O)Me | Cl | Me | O |
| C-6305 | H | CH₂ | Me | CH₂CF₃ | CH₂OC(=O)Me | Cl | Me | O |
| C-6306 | Me | CH₂ | H | CH₂CF₃ | CH₂OC(=O)Me | Cl | Me | O |
| C-6307 | H | CH(Me) | H | CH₂CF₃ | CH₂OC(=O)Me | Cl | Me | O |
| C-6308 | H | CH₂CH₂ | Me | CH₂CF₃ | CH₂OC(=O)Me | Cl | Me | O |
| C-6309 | Me | CH₂CH₂ | H | CH₂CF₃ | CH₂OC(=O)Me | Cl | Me | O |
| C-6310 | H | single bond | Me | CH₂CF₃ | CH₂OC(=O)Me | Me | Me | O |
| C-6311 | Me | single bond | H | CH₂CF₃ | CH₂OC(=O)Me | Me | Me | O |
| C-6312 | Me(R) | single bond | H | CH₂CF₃ | CH₂OC(=O)Me | Me | Me | O |
| C-6313 | Me(S) | single bond | H | CH₂CF₃ | CH₂OC(=O)Me | Me | Me | O |
| C-6314 | H | CH₂ | Me | CH₂CF₃ | CH₂OC(=O)Me | Me | Me | O |
| C-6315 | Me | CH₂ | H | CH₂CF₃ | CH₂OC(=O)Me | Me | Me | O |
| C-6316 | H | CH(Me) | H | CH₂CF₃ | CH₂OC(=O)Me | Me | Me | O |
| C-6317 | H | CH₂CH₂ | Me | CH₂CF₃ | CH₂OC(=O)Me | Me | Me | O |
| C-6318 | Me | CH₂CH₂ | H | CH₂CF₃ | CH₂OC(=O)Me | Me | Me | O |
| C-6319 | H | single bond | Me | CH₂CF₃ | CH₂OC(=O)(*i*-Pr) | Cl | Me | O |
| C-6320 | Me | single bond | H | CH₂CF₃ | CH₂OC(=O)(*i*-Pr) | Cl | Me | O |
| C-6321 | Me(R) | single bond | H | CH₂CF₃ | CH₂OC(=O)(*i*-Pr) | Cl | Me | O |
| C-6322 | Me(S) | single bond | H | CH₂CF₃ | CH₂OC(=O)(*i*-Pr) | Cl | Me | O |
| C-6323 | H | CH₂ | Me | CH₂CF₃ | CH₂OC(=O)(*i*-Pr) | Cl | Me | O |
| C-6324 | Me | CH₂ | H | CH₂CF₃ | CH₂OC(=O)(*i*-Pr) | Cl | Me | O |
| C-6325 | H | CH(Me) | H | CH₂CF₃ | CH₂OC(=O)(*i*-Pr) | Cl | Me | O |
| C-6326 | H | CH₂CH₂ | Me | CH₂CF₃ | CH₂OC(=O)(*i*-Pr) | Cl | Me | O |
| C-6327 | Me | CH₂CH₂ | H | CH₂CF₃ | CH₂OC(=O)(*i*-Pr) | Cl | Me | O |
| C-6328 | H | single bond | Me | CH₂CF₃ | CH₂OC(=O)(*i*-Pr) | Me | Me | O |
| C-6329 | Me | single bond | H | CH₂CF₃ | CH₂OC(=O)(*i*-Pr) | Me | Me | O |
| C-6330 | Me(R) | single bond | H | CH₂CF₃ | CH₂OC(=O)(*i*-Pr) | Me | Me | O |
| C-6331 | Me(S) | single bond | H | CH₂CF₃ | CH₂OC(=O)(*i*-Pr) | Me | Me | O |
| C-6332 | H | CH₂ | Me | CH₂CF₃ | CH₂OC(=O)(*i*-Pr) | Me | Me | O |
| C-6333 | Me | CH₂ | H | CH₂CF₃ | CH₂OC(=O)(*i*-Pr) | Me | Me | O |
| C-6334 | H | CH(Me) | H | CH₂CF₃ | CH₂OC(=O)(*i*-Pr) | Me | Me | O |
| C-6335 | H | CH₂CH₂ | Me | CH₂CF₃ | CH₂OC(=O)(*i*-Pr) | Me | Me | O |
| C-6336 | Me | CH₂CH₂ | H | CH₂CF₃ | CH₂OC(=O)(*i*-Pr) | Me | Me | O |
| C-6337 | H | single bond | Me | CH₂CF₃ | CH₂OC(=O)(*t*-Bu) | Cl | Me | O |
| C-6338 | Me | single bond | H | CH₂CF₃ | CH₂OC(=O)(*t*-Bu) | Cl | Me | O |
| C-6339 | Me(R) | single bond | H | CH₂CF₃ | CH₂OC(=O)(*t*-Bu) | Cl | Me | O |
| C-6340 | Me(S) | single bond | H | CH₂CF₃ | CH₂OC(=O)(*t*-Bu) | Cl | Me | O |
| C-6341 | H | CH₂ | Me | CH₂CF₃ | CH₂OC(=O)(*t*-Bu) | Cl | Me | O |
| C-6342 | Me | CH₂ | H | CH₂CF₃ | CH₂OC(=O)(*t*-Bu) | Cl | Me | O |

**[Table 372]**

| Compound Number | Y | W | Z | R¹ | R² | R³ | R¹⁰ | A |
|---|---|---|---|---|---|---|---|---|
| C-6343 | H | CH(Me) | H | CH₂CF₃ | CH₂OC(=O)(*t*-Bu) | Cl | Me | O |
| C-6344 | H | CH₂CH₂ | Me | CH₂CF₃ | CH₂OC(=O)(*t*-Bu) | Cl | Me | O |
| C-6345 | Me | CH₂CH₂ | H | CH₂CF₃ | CH₂OC(=O)(*t*-Bu) | Cl | Me | O |
| C-6346 | H | single bond | Me | CH₂CF₃ | CH₂OC(=O)(*t*-Bu) | Me | Me | O |
| C-6347 | Me | single bond | H | CH₂CF₃ | CH₂OC(=O)(*t*-Bu) | Me | Me | O |
| C-6348 | Me(R) | single bond | H | CH₂CF₃ | CH₂OC(=O)(*t*-Bu) | Me | Me | O |
| C-6349 | Me(S) | single bond | H | CH₂CF₃ | CH₂OC(=O)(*t*-Bu) | Me | Me | O |
| C-6350 | H | CH₂ | Me | CH₂CF₃ | CH₂OC(=O)(*t*-Bu) | Me | Me | O |
| C-6351 | Me | CH₂ | H | CH₂CF₃ | CH₂OC(=O)(*t*-Bu) | Me | Me | O |
| C-6352 | H | CH(Me) | H | CH₂CF₃ | CH₂OC(=O)(*t*-Bu) | Me | Me | O |
| C-6353 | H | CH₂CH₂ | Me | CH₂CF₃ | CH₂OC(=O)(*t*-Bu) | Me | Me | O |
| C-6354 | Me | CH₂CH₂ | H | CH₂CF₃ | CH₂OC(=O)(*t*-Bu) | Me | Me | O |
| C-6355 | H | single bond | Me | CH₂CF₃ | CH₂OC(=O)OEt | Cl | Me | O |
| C-6356 | Me | single bond | H | CH₂CF₃ | CH₂OC(=O)OEt | Cl | Me | O |
| C-6357 | Me(R) | single bond | H | CH₂CF₃ | CH₂OC(=O)OEt | Cl | Me | O |
| C-6358 | Me(S) | single bond | H | CH₂CF₃ | CH₂OC(=O)OEt | Cl | Me | O |
| C-6359 | H | CH₂ | Me | CH₂CF₃ | CH₂OC(=O)OEt | Cl | Me | O |
| C-6360 | Me | CH₂ | H | CH₂CF₃ | CH₂OC(=O)OEt | Cl | Me | O |
| C-6361 | H | CH(Me) | H | CH₂CF₃ | CH₂OC(=O)OEt | Cl | Me | O |
| C-6362 | H | CH₂CH₂ | Me | CH₂CF₃ | CH₂OC(=O)OEt | Cl | Me | O |
| C-6363 | Me | CH₂CH₂ | H | CH₂CF₃ | CH₂OC(=O)OEt | Cl | Me | O |
| C-6364 | H | single bond | Me | CH₂CF₃ | CH₂OC(=O)OEt | Me | Me | O |
| C-6365 | Me | single bond | H | CH₂CF₃ | CH₂OC(=O)OEt | Me | Me | O |
| C-6366 | Me(R) | single bond | H | CH₂CF₃ | CH₂OC(=O)OEt | Me | Me | O |
| C-6367 | Me(S) | single bond | H | CH₂CF₃ | CH₂OC(=O)OEt | Me | Me | O |
| C-6368 | H | CH₂ | Me | CH₂CF₃ | CH₂OC(=O)OEt | Me | Me | O |
| C-6369 | Me | CH₂ | H | CH₂CF₃ | CH₂OC(=O)OEt | Me | Me | O |
| C-6370 | H | CH(Me) | H | CH₂CF₃ | CH₂OC(=O)OEt | Me | Me | O |
| C-6371 | H | CH₂CH₂ | Me | CH₂CF₃ | CH₂OC(=O)OEt | Me | Me | O |
| C-6372 | Me | CH₂CH₂ | H | CH₂CF₃ | CH₂OC(=O)OEt | Me | Me | O |
| C-6373 | H | single bond | Me | CH₂CF₃ | CH₂OC(=O)O(*i*-Pr) | Cl | Me | O |
| C-6374 | Me | single bond | H | CH₂CF₃ | CH₂OC(=O)O(*i*-Pr) | Cl | Me | O |
| C-6375 | Me(R) | single bond | H | CH₂CF₃ | CH₂OC(=O)O(*i*-Pr) | Cl | Me | O |
| C-6376 | Me(S) | single bond | H | CH₂CF₃ | CH₂OC(=O)O(*i*-Pr) | Cl | Me | O |
| C-6377 | H | CH₂ | Me | CH₂CF₃ | CH₂OC(=O)O(*i*-Pr) | Cl | Me | O |
| C-6378 | Me | CH₂ | H | CH₂CF₃ | CH₂OC(=O)O(*i*-Pr) | Cl | Me | O |
| C-6379 | H | CH(Me) | H | CH₂CF₃ | CH₂OC(=O)O(*i*-Pr) | Cl | Me | O |
| C-6380 | H | CH₂CH₂ | Me | CH₂CF₃ | CH₂OC(=O)O(*i*-Pr) | Cl | Me | O |
| C-6381 | Me | CH₂CH₂ | H | CH₂CF₃ | CH₂OC(=O)O(*i*-Pr) | Cl | Me | O |
| C-6382 | H | single bond | Me | CH₂CF₃ | CH₂OC(=O)O(*i*-Pr) | Me | Me | O |
| C-6383 | Me | single bond | H | CH₂CF₃ | CH₂OC(=O)O(*i*-Pr) | Me | Me | O |
| C-6384 | Me(R) | single bond | H | CH₂CF₃ | CH₂OC(=O)O(*i*-Pr) | Me | Me | O |
| C-6385 | Me(S) | single bond | H | CH₂CF₃ | CH₂OC(=O)O(*i*-Pr) | Me | Me | O |
| C-6386 | H | CH₂ | Me | CH₂CF₃ | CH₂OC(=O)O(*i*-Pr) | Me | Me | O |

**[Table 373]**

| Compound Number | Y | W | Z | R¹ | R² | R³ | R¹⁰ | A |
|---|---|---|---|---|---|---|---|---|
| C-6387 | Me | CH₂ | H | CH₂CF₃ | CH₂OC(=O)O(*i*-Pr) | Me | Me | O |
| C-6388 | H | CH(Me) | H | CH₂CF₃ | CH₂OC(=O)O(*i*-Pr) | Me | Me | O |
| C-6389 | H | CH₂CH₂ | Me | CH₂CF₃ | CH₂OC(=O)O(*i*-Pr) | Me | Me | O |
| C-6390 | Me | CH₂CH₂ | H | CH₂CF₃ | CH₂OC(=O)O(*i*-Pr) | Me | Me | O |
| C-6391 | H | single bond | Me | CH₂CF₃ | CH₂N(Me)C(=O)OMe | Cl | Me | O |
| C-6392 | Me | single bond | H | CH₂CF₃ | CH₂N(Me)C(=O)OMe | Cl | Me | O |
| C-6393 | Me(R) | single bond | H | CH₂CF₃ | CH₂N(Me)C(=O)OMe | Cl | Me | O |
| C-6394 | Me(S) | single bond | H | CH₂CF₃ | CH₂N(Me)C(=O)OMe | Cl | Me | O |
| C-6395 | H | CH₂ | Me | CH₂CF₃ | CH₂N(Me)C(=O)OMe | Cl | Me | O |
| C-6396 | Me | CH₂ | H | CH₂CF₃ | CH₂N(Me)C(=O)OMe | Cl | Me | O |
| C-6397 | H | CH(Me) | H | CH₂CF₃ | CH₂N(Me)C(=O)OMe | Cl | Me | O |
| C-6398 | H | CH₂CH₂ | Me | CH₂CF₃ | CH₂N(Me)C(=O)OMe | Cl | Me | O |
| C-6399 | Me | CH₂CH₂ | H | CH₂CF₃ | CH₂N(Me)C(=O)OMe | Cl | Me | O |
| C-6400 | H | single bond | Me | CH₂CF₃ | CH₂N(Me)C(=O)OMe | Me | Me | O |
| C-6401 | Me | single bond | H | CH₂CF₃ | CH₂N(Me)C(=O)OMe | Me | Me | O |
| C-6402 | Me(R) | single bond | H | CH₂CF₃ | CH₂N(Me)C(=O)OMe | Me | Me | O |
| C-6403 | Me(S) | single bond | H | CH₂CF₃ | CH₂N(Me)C(=O)OMe | Me | Me | O |
| C-6404 | H | CH₂ | Me | CH₂CF₃ | CH₂N(Me)C(=O)OMe | Me | Me | O |
| C-6405 | Me | CH₂ | H | CH₂CF₃ | CH₂N(Me)C(=O)OMe | Me | Me | O |
| C-6406 | H | CH(Me) | H | CH₂CF₃ | CH₂N(Me)C(=O)OMe | Me | Me | O |
| C-6407 | H | CH₂CH₂ | Me | CH₂CF₃ | CH₂N(Me)C(=O)OMe | Me | Me | O |
| C-6408 | Me | CH₂CH₂ | H | CH₂CF₃ | CH₂N(Me)C(=O)OMe | Me | Me | O |
| C-6409 | H | single bond | Me | CH₂CF₃ | C(=O)OCH₂CH=CH₂ | Cl | CH₂OMe | O |
| C-6410 | Me | single bond | H | CH₂CF₃ | C(=O)OCH₂CH=CH₂ | Cl | CH₂OMe | O |
| C-6411 | Me(R) | single bond | H | CH₂CF₃ | C(=O)OCH₂CH=CH₂ | Cl | CH₂OMe | O |
| C-6412 | Me(S) | single bond | H | CH₂CF₃ | C(=O)OCH₂CH=CH₂ | Cl | CH₂OMe | O |
| C-6413 | H | CH₂ | Me | CH₂CF₃ | C(=O)OCH₂CH=CH₂ | Cl | CH₂OMe | O |
| C-6414 | Me | CH₂ | H | CH₂CF₃ | C(=O)OCH₂CH=CH₂ | Cl | CH₂OMe | O |
| C-6415 | H | CH(Me) | H | CH₂CF₃ | C(=O)OCH₂CH=CH₂ | Cl | CH₂OMe | O |
| C-6416 | H | CH₂CH₂ | Me | CH₂CF₃ | C(=O)OCH₂CH=CH₂ | Cl | CH₂OMe | O |
| C-6417 | Me | CH₂CH₂ | H | CH₂CF₃ | C(=O)OCH₂CH=CH₂ | Cl | CH₂OMe | O |
| C-6418 | H | single bond | Me | CH₂CF₃ | C(=O)OCH₂CH=CH₂ | Me | CH₂OMe | O |
| C-6419 | Me | single bond | H | CH₂CF₃ | C(=O)OCH₂CH=CH₂ | Me | CH₂OMe | O |
| C-6420 | Me(R) | single bond | H | CH₂CF₃ | C(=O)OCH₂CH=CH₂ | Me | CH₂OMe | O |
| C-6421 | Me(S) | single bond | H | CH₂CF₃ | C(=O)OCH₂CH=CH₂ | Me | CH₂OMe | O |
| C-6422 | H | CH₂ | Me | CH₂CF₃ | C(=O)OCH₂CH=CH₂ | Me | CH₂OMe | O |
| C-6423 | Me | CH₂ | H | CH₂CF₃ | C(=O)OCH₂CH=CH₂ | Me | CH₂OMe | O |
| C-6424 | H | CH(Me) | H | CH₂CF₃ | C(=O)OCH₂CH=CH₂ | Me | CH₂OMe | O |
| C-6425 | H | CH₂CH₂ | Me | CH₂CF₃ | C(=O)OCH₂CH=CH₂ | Me | CH₂OMe | O |
| C-6426 | Me | CH₂CH₂ | H | CH₂CF₃ | C(=O)OCH₂CH=CH₂ | Me | CH₂OMe | O |
| C-6427 | H | single bond | Me | CH₂CF₃ | C(=O)OCH₂CCl₃ | Cl | CH₂OMe | O |
| C-6428 | Me | single bond | H | CH₂CF₃ | C(=O)OCH₂CCl₃ | Cl | CH₂OMe | O |
| C-6429 | Me(R) | single bond | H | CH₂CF₃ | C(=O)OCH₂CCl₃ | Cl | CH₂OMe | O |
| C-6430 | Me(S) | single bond | H | CH₂CF₃ | C(=O)OCH₂CCl₃ | Cl | CH₂OMe | O |

**[Table 374]**

| Compound Number | Y | W | Z | R¹ | R² | R³ | R¹⁰ | A |
|---|---|---|---|---|---|---|---|---|
| C-6431 | H | CH₂ | Me | CH₂CF₃ | C(=O)OCH₂CCl₃ | Cl | CH₂OMe | O |
| C-6432 | Me | CH₂ | H | CH₂CF₃ | C(=O)OCH₂CCl₃ | Cl | CH₂OMe | O |
| C-6433 | H | CH(Me) | H | CH₂CF₃ | C(=O)OCH₂CCl₃ | Cl | CH₂OMe | O |
| C-6434 | H | CH₂CH₂ | Me | CH₂CF₃ | C(=O)OCH₂CCl₃ | Cl | CH₂OMe | O |
| C-6435 | Me | CH₂CH₂ | H | CH₂CF₃ | C(=O)OCH₂CCl₃ | Cl | CH₂OMe | O |
| C-6436 | H | single bond | Me | CH₂CF₃ | C(=O)OCH₂CCl₃ | Me | CH₂OMe | O |
| C-6437 | Me | single bond | H | CH₂CF₃ | C(=O)OCH₂CCl₃ | Me | CH₂OMe | O |
| C-6438 | Me(R) | single bond | H | CH₂CF₃ | C(=O)OCH₂CCl₃ | Me | CH₂OMe | O |
| C-6439 | Me(S) | single bond | H | CH₂CF₃ | C(=O)OCH₂CCl₃ | Me | CH₂OMe | O |
| C-6440 | H | CH₂ | Me | CH₂CF₃ | C(=O)OCH₂CCl₃ | Me | CH₂OMe | O |
| C-6441 | Me | CH₂ | H | CH₂CF₃ | C(=O)OCH₂CCl₃ | Me | CH₂OMe | O |
| C-6442 | H | CH(Me) | H | CH₂CF₃ | C(=O)OCH₂CCl₃ | Me | CH₂OMe | O |
| C-6443 | H | CH₂CH₂ | Me | CH₂CF₃ | C(=O)OCH₂CCl₃ | Me | CH₂OMe | O |
| C-6444 | Me | CH₂CH₂ | H | CH₂CF₃ | C(=O)OCH₂CCl₃ | Me | CH₂OMe | O |
| C-6445 | H | single bond | Me | CH₂CF₃ | C(=O)N(Me)₂ | Cl | CH₂OMe | O |
| C-6446 | Me | single bond | H | CH₂CF₃ | C(=O)N(Me)₂ | Cl | CH₂OMe | O |
| C-6447 | Me(R) | single bond | H | CH₂CF₃ | C(=O)N(Me)₂ | Cl | CH₂OMe | O |
| C-6448 | Me(S) | single bond | H | CH₂CF₃ | C(=O)N(Me)₂ | Cl | CH₂OMe | O |
| C-6449 | H | CH₂ | Me | CH₂CF₃ | C(=O)N(Me)₂ | Cl | CH₂OMe | O |
| C-6450 | Me | CH₂ | H | CH₂CF₃ | C(=O)N(Me)₂ | Cl | CH₂OMe | O |
| C-6451 | H | CH(Me) | H | CH₂CF₃ | C(=O)N(Me)₂ | Cl | CH₂OMe | O |
| C-6452 | H | CH₂CH₂ | Me | CH₂CF₃ | C(=O)N(Me)₂ | Cl | CH₂OMe | O |
| C-6453 | Me | CH₂CH₂ | H | CH₂CF₃ | C(=O)N(Me)₂ | Cl | CH₂OMe | O |
| C-6454 | H | single bond | Me | CH₂CF₃ | C(=O)N(Me)₂ | Me | CH₂OMe | O |
| C-6455 | Me | single bond | H | CH₂CF₃ | C(=O)N(Me)₂ | Me | CH₂OMe | O |
| C-6456 | Me(R) | single bond | H | CH₂CF₃ | C(=O)N(Me)₂ | Me | CH₂OMe | O |
| C-6457 | Me(S) | single bond | H | CH₂CF₃ | C(=O)N(Me)₂ | Me | CH₂OMe | O |
| C-6458 | H | CH₂ | Me | CH₂CF₃ | C(=O)N(Me)₂ | Me | CH₂OMe | O |
| C-6459 | Me | CH₂ | H | CH₂CF₃ | C(=O)N(Me)₂ | Me | CH₂OMe | O |
| C-6460 | H | CH(Me) | H | CH₂CF₃ | C(=O)N(Me)₂ | Me | CH₂OMe | O |
| C-6461 | H | CH₂CH₂ | Me | CH₂CF₃ | C(=O)N(Me)₂ | Me | CH₂OMe | O |
| C-6462 | Me | CH₂CH₂ | H | CH₂CF₃ | C(=O)N(Me)₂ | Me | CH₂OMe | O |
| C-6463 | H | single bond | Me | CH₂CF₃ | CH₂OC(=O)Me | Cl | CH₂OMe | O |
| C-6464 | Me | single bond | H | CH₂CF₃ | CH₂OC(=O)Me | Cl | CH₂OMe | O |
| C-6465 | Me(R) | single bond | H | CH₂CF₃ | CH₂OC(=O)Me | Cl | CH₂OMe | O |
| C-6466 | Me(S) | single bond | H | CH₂CF₃ | CH₂OC(=O)Me | Cl | CH₂OMe | O |
| C-6467 | H | CH₂ | Me | CH₂CF₃ | CH₂OC(=O)Me | Cl | CH₂OMe | O |
| C-6468 | Me | CH₂ | H | CH₂CF₃ | CH₂OC(=O)Me | Cl | CH₂OMe | O |
| C-6469 | H | CH(Me) | H | CH₂CF₃ | CH₂OC(=O)Me | Cl | CH₂OMe | O |
| C-6470 | H | CH₂CH₂ | Me | CH₂CF₃ | CH₂OC(=O)Me | Cl | CH₂OMe | O |
| C-6471 | Me | CH₂CH₂ | H | CH₂CF₃ | CH₂OC(=O)Me | Cl | CH₂OMe | O |
| C-6472 | H | single bond | Me | CH₂CF₃ | CH₂OC(=O)Me | Me | CH₂OMe | O |
| C-6473 | Me | single bond | H | CH₂CF₃ | CH₂OC(=O)Me | Me | CH₂OMe | O |
| C-6474 | Me(R) | single bond | H | CH₂CF₃ | CH₂OC(=O)Me | Me | CH₂OMe | O |

**[Table 375]**

| Compound Number | Y | W | Z | R¹ | R² | R³ | R¹⁰ | A |
|---|---|---|---|---|---|---|---|---|
| C-6475 | Me(S) | single bond | H | CH₂CF₃ | CH₂OC(=O)Me | Me | CH₂OMe | O |
| C-6476 | H | CH₂ | Me | CH₂CF₃ | CH₂OC(=O)Me | Me | CH₂OMe | O |
| C-6477 | Me | CH₂ | H | CH₂CF₃ | CH₂OC(=O)Me | Me | CH₂OMe | O |
| C-6478 | H | CH(Me) | H | CH₂CF₃ | CH₂OC(=O)Me | Me | CH₂OMe | O |
| C-6479 | H | CH₂CH₂ | Me | CH₂CF₃ | CH₂OC(=O)Me | Me | CH₂OMe | O |
| C-6480 | Me | CH₂CH₂ | H | CH₂CF₃ | CH₂OC(=O)Me | Me | CH₂OMe | O |
| C-6481 | H | single bond | Me | CH₂CF₃ | CH₂OC(=O)(*i*-Pr) | Cl | CH₂OMe | O |
| C-6482 | Me | single bond | H | CH₂CF₃ | CH₂OC(=O)(*i*-Pr) | Cl | CH₂OMe | O |
| C-6483 | Me(R) | single bond | H | CH₂CF₃ | CH₂OC(=O)(*i*-Pr) | Cl | CH₂OMe | O |
| C-6484 | Me(S) | single bond | H | CH₂CF₃ | CH₂OC(=O)(*i*-Pr) | Cl | CH₂OMe | O |
| C-6485 | H | CH₂ | Me | CH₂CF₃ | CH₂OC(=O)(*i*-Pr) | Cl | CH₂OMe | O |
| C-6486 | Me | CH₂ | H | CH₂CF₃ | CH₂OC(=O)(*i*-Pr) | Cl | CH₂OMe | O |
| C-6487 | H | CH(Me) | H | CH₂CF₃ | CH₂OC(=O)(*i*-Pr) | Cl | CH₂OMe | O |
| C-6488 | H | CH₂CH₂ | Me | CH₂CF₃ | CH₂OC(=O)(*i*-Pr) | Cl | CH₂OMe | O |
| C-6489 | Me | CH₂CH₂ | H | CH₂CF₃ | CH₂OC(=O)(*i*-Pr) | Cl | CH₂OMe | O |
| C-6490 | H | single bond | Me | CH₂CF₃ | CH₂OC(=O)(*i*-Pr) | Me | CH₂OMe | O |
| C-6491 | Me | single bond | H | CH₂CF₃ | CH₂OC(=O)(*i*-Pr) | Me | CH₂OMe | O |
| C-6492 | Me(R) | single bond | H | CH₂CF₃ | CH₂OC(=O)(*i*-Pr) | Me | CH₂OMe | O |
| C-6493 | Me(S) | single bond | H | CH₂CF₃ | CH₂OC(=O)(*i*-Pr) | Me | CH₂OMe | O |
| C-6494 | H | CH₂ | Me | CH₂CF₃ | CH₂OC(=O)(*i*-Pr) | Me | CH₂OMe | O |
| C-6495 | Me | CH₂ | H | CH₂CF₃ | CH₂OC(=O)(*i*-Pr) | Me | CH₂OMe | O |
| C-6496 | H | CH(Me) | H | CH₂CF₃ | CH₂OC(=O)(*i*-Pr) | Me | CH₂OMe | O |
| C-6497 | H | CH₂CH₂ | Me | CH₂CF₃ | CH₂OC(=O)(*i*-Pr) | Me | CH₂OMe | O |
| C-6498 | Me | CH₂CH₂ | H | CH₂CF₃ | CH₂OC(=O)(*i*-Pr) | Me | CH₂OMe | O |
| C-6499 | H | single bond | Me | CH₂CF₃ | CH₂OC(=O)(*t*-Bu) | Cl | CH₂OMe | O |
| C-6500 | Me | single bond | H | CH₂CF₃ | CH₂OC(=O)(*t*-Bu) | Cl | CH₂OMe | O |
| C-6501 | Me(R) | single bond | H | CH₂CF₃ | CH₂OC(=O)(*t*-Bu) | Cl | CH₂OMe | O |
| C-6502 | Me(S) | single bond | H | CH₂CF₃ | CH₂OC(=O)(*t*-Bu) | Cl | CH₂OMe | O |
| C-6503 | H | CH₂ | Me | CH₂CF₃ | CH₂OC(=O)(*t*-Bu) | Cl | CH₂OMe | O |
| C-6504 | Me | CH₂ | H | CH₂CF₃ | CH₂OC(=O)(*t*-Bu) | Cl | CH₂OMe | O |
| C-6505 | H | CH(Me) | H | CH₂CF₃ | CH₂OC(=O)(*t*-Bu) | Cl | CH₂OMe | O |
| C-6506 | H | CH₂CH₂ | Me | CH₂CF₃ | CH₂OC(=O)(*t*-Bu) | Cl | CH₂OMe | O |
| C-6507 | Me | CH₂CH₂ | H | CH₂CF₃ | CH₂OC(=O)(*t*-Bu) | Cl | CH₂OMe | O |
| C-6508 | H | single bond | Me | CH₂CF₃ | CH₂OC(=O)(*t*-Bu) | Me | CH₂OMe | O |
| C-6509 | Me | single bond | H | CH₂CF₃ | CH₂OC(=O)(*t*-Bu) | Me | CH₂OMe | O |
| C-6510 | Me(R) | single bond | H | CH₂CF₃ | CH₂OC(=O)(*t*-Bu) | Me | CH₂OMe | O |
| C-6511 | Me(S) | single bond | H | CH₂CF₃ | CH₂OC(=O)(*t*-Bu) | Me | CH₂OMe | O |
| C-6512 | H | CH₂ | Me | CH₂CF₃ | CH₂OC(=O)(*t*-Bu) | Me | CH₂OMe | O |
| C-6513 | Me | CH₂ | H | CH₂CF₃ | CH₂OC(=O)(*t*-Bu) | Me | CH₂OMe | O |
| C-6514 | H | CH(Me) | H | CH₂CF₃ | CH₂OC(=O)(*t*-Bu) | Me | CH₂OMe | O |
| C-6515 | H | CH₂CH₂ | Me | CH₂CF₃ | CH₂OC(=O)(*t*-Bu) | Me | CH₂OMe | O |
| C-6516 | Me | CH₂CH₂ | H | CH₂CF₃ | CH₂OC(=O)(*t*-Bu) | Me | CH₂OMe | O |
| C-6517 | H | single bond | Me | CH₂CF₃ | CH₂OC(=O)OEt | Cl | CH₂OMe | O |
| C-6518 | Me | single bond | H | CH₂CF₃ | CH₂OC(=O)OEt | Cl | CH₂OMe | O |

**[Table 376]**

| Compound Number | Y | W | Z | R¹ | R² | R³ | R¹⁰ | A |
|---|---|---|---|---|---|---|---|---|
| C-6519 | Me(R) | single bond | H | CH₂CF₃ | CH₂OC(=O)OEt | Cl | CH₂OMe | O |
| C-6520 | Me(S) | single bond | H | CH₂CF₃ | CH₂OC(=O)OEt | Cl | CH₂OMe | O |
| C-6521 | H | CH₂ | Me | CH₂CF₃ | CH₂OC(=O)OEt | Cl | CH₂OMe | O |
| C-6522 | Me | CH₂ | H | CH₂CF₃ | CH₂OC(=O)OEt | Cl | CH₂OMe | O |
| C-6523 | H | CH(Me) | H | CH₂CF₃ | CH₂OC(=O)OEt | Cl | CH₂OMe | O |
| C-6524 | H | CH₂CH₂ | Me | CH₂CF₃ | CH₂OC(=O)OEt | Cl | CH₂OMe | O |
| C-6525 | Me | CH₂CH₂ | H | CH₂CF₃ | CH₂OC(=O)OEt | Cl | CH₂OMe | O |
| C-6526 | H | single bond | Me | CH₂CF₃ | CH₂OC(=O)OEt | Me | CH₂OMe | O |
| C-6527 | Me | single bond | H | CH₂CF₃ | CH₂OC(=O)OEt | Me | CH₂OMe | O |
| C-6528 | Me(R) | single bond | H | CH₂CF₃ | CH₂OC(=O)OEt | Me | CH₂OMe | O |
| C-6529 | Me(S) | single bond | H | CH₂CF₃ | CH₂OC(=O)OEt | Me | CH₂OMe | O |
| C-6530 | H | CH₂ | Me | CH₂CF₃ | CH₂OC(=O)OEt | Me | CH₂OMe | O |
| C-6531 | Me | CH₂ | H | CH₂CF₃ | CH₂OC(=O)OEt | Me | CH₂OMe | O |
| C-6532 | H | CH(Me) | H | CH₂CF₃ | CH₂OC(=O)OEt | Me | CH₂OMe | O |
| C-6533 | H | CH₂CH₂ | Me | CH₂CF₃ | CH₂OC(=O)OEt | Me | CH₂OMe | O |
| C-6534 | Me | CH₂CH₂ | H | CH₂CF₃ | CH₂OC(=O)OEt | Me | CH₂OMe | O |
| C-6535 | H | single bond | Me | CH₂CF₃ | CH₂OC(=O)O(*i* -Pr) | Cl | CH₂OMe | O |
| C-6536 | Me | single bond | H | CH₂CF₃ | CH₂OC(=O)O(*i* -Pr) | Cl | CH₂OMe | O |
| C-6537 | Me(R) | single bond | H | CH₂CF₃ | CH₂OC(=O)O(*i* -Pr) | Cl | CH₂OMe | O |
| C-6538 | Me(S) | single bond | H | CH₂CF₃ | CH₂OC(=O)O(*i* -Pr) | Cl | CH₂OMe | O |
| C-6539 | H | CH₂ | Me | CH₂CF₃ | CH₂OC(=O)O(*i* -Pr) | Cl | CH₂OMe | O |
| C-6540 | Me | CH₂ | H | CH₂CF₃ | CH₂OC(=O)O(*i* -Pr) | Cl | CH₂OMe | O |
| C-6541 | H | CH(Me) | H | CH₂CF₃ | CH₂OC(=O)O(*i* -Pr) | Cl | CH₂OMe | O |
| C-6542 | H | CH₂CH₂ | Me | CH₂CF₃ | CH₂OC(=O)O(*i* -Pr) | Cl | CH₂OMe | O |
| C-6543 | Me | CH₂CH₂ | H | CH₂CF₃ | CH₂OC(=O)O(*i* -Pr) | Cl | CH₂OMe | O |
| C-6544 | H | single bond | Me | CH₂CF₃ | CH₂OC(=O)O(*i* -Pr) | Me | CH₂OMe | O |
| C-6545 | Me | single bond | H | CH₂CF₃ | CH₂OC(=O)O(*i* -Pr) | Me | CH₂OMe | O |
| C-6546 | Me(R) | single bond | H | CH₂CF₃ | CH₂OC(=O)O(*i* -Pr) | Me | CH₂OMe | O |
| C-6547 | Me(S) | single bond | H | CH₂CF₃ | CH₂OC(=O)O(*i* -Pr) | Me | CH₂OMe | O |
| C-6548 | H | CH₂ | Me | CH₂CF₃ | CH₂OC(=O)O(*i* -Pr) | Me | CH₂OMe | O |
| C-6549 | Me | CH₂ | H | CH₂CF₃ | CH₂OC(=O)O(*i* -Pr) | Me | CH₂OMe | O |
| C-6550 | H | CH(Me) | H | CH₂CF₃ | CH₂OC(=O)O(*i* -Pr) | Me | CH₂OMe | O |
| C-6551 | H | CH₂CH₂ | Me | CH₂CF₃ | CH₂OC(=O)O(*i* -Pr) | Me | CH₂OMe | O |
| C-6552 | Me | CH₂CH₂ | H | CH₂CF₃ | CH₂OC(=O)O(*i* -Pr) | Me | CH₂OMe | O |
| C-6553 | H | single bond | Me | CH₂CF₃ | CH₂N(Me)C(=O)OMe | Cl | CH₂OMe | O |
| C-6554 | Me | single bond | H | CH₂CF₃ | CH₂N(Me)C(=O)OMe | Cl | CH₂OMe | O |
| C-6555 | Me(R) | single bond | H | CH₂CF₃ | CH₂N(Me)C(=O)OMe | Cl | CH₂OMe | O |
| C-6556 | Me(S) | single bond | H | CH₂CF₃ | CH₂N(Me)C(=O)OMe | Cl | CH₂OMe | O |
| C-6557 | H | CH₂ | Me | CH₂CF₃ | CH₂N(Me)C(=O)OMe | Cl | CH₂OMe | O |
| C-6558 | Me | CH₂ | H | CH₂CF₃ | CH₂N(Me)C(=O)OMe | Cl | CH₂OMe | O |
| C-6559 | H | CH(Me) | H | CH₂CF₃ | CH₂N(Me)C(=O)OMe | Cl | CH₂OMe | O |
| C-6560 | H | CH₂CH₂ | Me | CH₂CF₃ | CH₂N(Me)C(=O)OMe | Cl | CH₂OMe | O |
| C-6561 | Me | CH₂CH₂ | H | CH₂CF₃ | CH₂N(Me)C(=O)OMe | Cl | CH₂OMe | O |
| C-6562 | H | single bond | Me | CH₂CF₃ | CH₂N(Me)C(=O)OMe | Me | CH₂OMe | O |

**[Table 377]**

| Compound Number | Y | W | Z | R¹ | R² | R³ | R¹⁰ | A |
|---|---|---|---|---|---|---|---|---|
| C-6563 | Me | single bond | H | CH₂CF₃ | CH₂N(Me)C(=O)OMe | Me | CH₂OMe | O |
| C-6564 | Me(R) | single bond | H | CH₂CF₃ | CH₂N(Me)C(=O)OMe | Me | CH₂OMe | O |
| C-6565 | Me(S) | single bond | H | CH₂CF₃ | CH₂N(Me)C(=O)OMe | Me | CH₂OMe | O |
| C-6566 | H | CH₂ | Me | CH₂CF₃ | CH₂N(Me)C(=O)OMe | Me | CH₂OMe | O |
| C-6567 | Me | CH₂ | H | CH₂CF₃ | CH₂N(Me)C(=O)OMe | Me | CH₂OMe | O |
| C-6568 | H | CH(Me) | H | CH₂CF₃ | CH₂N(Me)C(=O)OMe | Me | CH₂OMe | O |
| C-6569 | H | CH₂CH₂ | Me | CH₂CF₃ | CH₂N(Me)C(=O)OMe | Me | CH₂OMe | O |
| C-6570 | Me | CH₂CH₂ | H | CH₂CF₃ | CH₂N(Me)C(=O)OMe | Me | CH₂OMe | O |
| C-6571 | H | single bond | Me | CH₂CF₃ | C(=O)OCH₂CH=CH₂ | Cl | CH₂CN | O |
| C-6572 | Me | single bond | H | CH₂CF₃ | C(=O)OCH₂CH=CH₂ | Cl | CH₂CN | O |
| C-6573 | Me(R) | single bond | H | CH₂CF₃ | C(=O)OCH₂CH=CH₂ | Cl | CH₂CN | O |
| C-6574 | Me(S) | single bond | H | CH₂CF₃ | C(=O)OCH₂CH=CH₂ | Cl | CH₂CN | O |
| C-6575 | H | CH₂ | Me | CH₂CF₃ | C(=O)OCH₂CH=CH₂ | Cl | CH₂CN | O |
| C-6576 | Me | CH₂ | H | CH₂CF₃ | C(=O)OCH₂CH=CH₂ | Cl | CH₂CN | O |
| C-6577 | H | CH(Me) | H | CH₂CF₃ | C(=O)OCH₂CH=CH₂ | Cl | CH₂CN | O |
| C-6578 | H | CH₂CH₂ | Me | CH₂CF₃ | C(=O)OCH₂CH=CH₂ | Cl | CH₂CN | O |
| C-6579 | Me | CH₂CH₂ | H | CH₂CF₃ | C(=O)OCH₂CH=CH₂ | Cl | CH₂CN | O |
| C-6580 | H | single bond | Me | CH₂CF₃ | C(=O)OCH₂CH=CH₂ | Me | CH₂CN | O |
| C-6581 | Me | single bond | H | CH₂CF₃ | C(=O)OCH₂CH=CH₂ | Me | CH₂CN | O |
| C-6582 | Me(R) | single bond | H | CH₂CF₃ | C(=O)OCH₂CH=CH₂ | Me | CH₂CN | O |
| C-6583 | Me(S) | single bond | H | CH₂CF₃ | C(=O)OCH₂CH=CH₂ | Me | CH₂CN | O |
| C-6584 | H | CH₂ | Me | CH₂CF₃ | C(=O)OCH₂CH=CH₂ | Me | CH₂CN | O |
| C-6585 | Me | CH₂ | H | CH₂CF₃ | C(=O)OCH₂CH=CH₂ | Me | CH₂CN | O |
| C-6586 | H | CH(Me) | H | CH₂CF₃ | C(=O)OCH₂CH=CH₂ | Me | CH₂CN | O |
| C-6587 | H | CH₂CH₂ | Me | CH₂CF₃ | C(=O)OCH₂CH=CH₂ | Me | CH₂CN | O |
| C-6588 | Me | CH₂CH₂ | H | CH₂CF₃ | C(=O)OCH₂CH=CH₂ | Me | CH₂CN | O |
| C-6589 | H | single bond | Me | CH₂CF₃ | C(=O)OCH₂CCl₃ | Cl | CH₂CN | O |
| C-6590 | Me | single bond | H | CH₂CF₃ | C(=O)OCH₂CCl₃ | Cl | CH₂CN | O |
| C-6591 | Me(R) | single bond | H | CH₂CF₃ | C(=O)OCH₂CCl₃ | Cl | CH₂CN | O |
| C-6592 | Me(S) | single bond | H | CH₂CF₃ | C(=O)OCH₂CCl₃ | Cl | CH₂CN | O |
| C-6593 | H | CH₂ | Me | CH₂CF₃ | C(=O)OCH₂CCl₃ | Cl | CH₂CN | O |
| C-6594 | Me | CH₂ | H | CH₂CF₃ | C(=O)OCH₂CCl₃ | Cl | CH₂CN | O |
| C-6595 | H | CH(Me) | H | CH₂CF₃ | C(=O)OCH₂CCl₃ | Cl | CH₂CN | O |
| C-6596 | H | CH₂CH₂ | Me | CH₂CF₃ | C(=O)OCH₂CCl₃ | Cl | CH₂CN | O |
| C-6597 | Me | CH₂CH₂ | H | CH₂CF₃ | C(=O)OCH₂CCl₃ | Cl | CH₂CN | O |
| C-6598 | H | single bond | Me | CH₂CF₃ | C(=O)OCH₂CCl₃ | Me | CH₂CN | O |
| C-6599 | Me | single bond | H | CH₂CF₃ | C(=O)OCH₂CCl₃ | Me | CH₂CN | O |
| C-6600 | Me(R) | single bond | H | CH₂CF₃ | C(=O)OCH₂CCl₃ | Me | CH₂CN | O |
| C-6601 | Me(S) | single bond | H | CH₂CF₃ | C(=O)OCH₂CCl₃ | Me | CH₂CN | O |
| C-6602 | H | CH₂ | Me | CH₂CF₃ | C(=O)OCH₂CCl₃ | Me | CH₂CN | O |
| C-6603 | Me | CH₂ | H | CH₂CF₃ | C(=O)OCH₂CCl₃ | Me | CH₂CN | O |
| C-6604 | H | CH(Me) | H | CH₂CF₃ | C(=O)OCH₂CCl₃ | Me | CH₂CN | O |
| C-6605 | H | CH₂CH₂ | Me | CH₂CF₃ | C(=O)OCH₂CCl₃ | Me | CH₂CN | O |
| C-6606 | Me | CH₂CH₂ | H | CH₂CF₃ | C(=O)OCH₂CCl₃ | Me | CH₂CN | O |

**[Table 378]**

| Compound Number | Y | W | Z | R¹ | R² | R³ | R¹⁰ | A |
|---|---|---|---|---|---|---|---|---|
| C-6607 | H | single bond | Me | CH₂CF₃ | C(=O)N(Me)₂ | Cl | CH₂CN | O |
| C-6608 | Me | single bond | H | CH₂CF₃ | C(=O)N(Me)₂ | Cl | CH₂CN | O |
| C-6609 | Me(R) | single bond | H | CH₂CF₃ | C(=O)N(Me)₂ | Cl | CH₂CN | O |
| C-6610 | Me(S) | single bond | H | CH₂CF₃ | C(=O)N(Me)₂ | Cl | CH₂CN | O |
| C-6611 | H | CH₂ | Me | CH₂CF₃ | C(=O)N(Me)₂ | Cl | CH₂CN | O |
| C-6612 | Me | CH₂ | H | CH₂CF₃ | C(=O)N(Me)₂ | Cl | CH₂CN | O |
| C-6613 | H | CH(Me) | H | CH₂CF₃ | C(=O)N(Me)₂ | Cl | CH₂CN | O |
| C-6614 | H | CH₂CH₂ | Me | CH₂CF₃ | C(=O)N(Me)₂ | Cl | CH₂CN | O |
| C-6615 | Me | CH₂CH₂ | H | CH₂CF₃ | C(=O)N(Me)₂ | Cl | CH₂CN | O |
| C-6616 | H | single bond | Me | CH₂CF₃ | C(=O)N(Me)₂ | Me | CH₂CN | O |
| C-6617 | Me | single bond | H | CH₂CF₃ | C(=O)N(Me)₂ | Me | CH₂CN | O |
| C-6618 | Me(R) | single bond | H | CH₂CF₃ | C(=O)N(Me)₂ | Me | CH₂CN | O |
| C-6619 | Me(S) | single bond | H | CH₂CF₃ | C(=O)N(Me)₂ | Me | CH₂CN | O |
| C-6620 | H | CH₂ | Me | CH₂CF₃ | C(=O)N(Me)₂ | Me | CH₂CN | O |
| C-6621 | Me | CH₂ | H | CH₂CF₃ | C(=O)N(Me)₂ | Me | CH₂CN | O |
| C-6622 | H | CH(Me) | H | CH₂CF₃ | C(=O)N(Me)₂ | Me | CH₂CN | O |
| C-6623 | H | CH₂CH₂ | Me | CH₂CF₃ | C(=O)N(Me)₂ | Me | CH₂CN | O |
| C-6624 | Me | CH₂CH₂ | H | CH₂CF₃ | C(=O)N(Me)₂ | Me | CH₂CN | O |
| C-6625 | H | single bond | Me | CH₂CF₃ | CH₂OC(=O)Me | Cl | CH₂CN | O |
| C-6626 | Me | single bond | H | CH₂CF₃ | CH₂OC(=O)Me | Cl | CH₂CN | O |
| C-6627 | Me(R) | single bond | H | CH₂CF₃ | CH₂OC(=O)Me | Cl | CH₂CN | O |
| C-6628 | Me(S) | single bond | H | CH₂CF₃ | CH₂OC(=O)Me | Cl | CH₂CN | O |
| C-6629 | H | CH₂ | Me | CH₂CF₃ | CH₂OC(=O)Me | Cl | CH₂CN | O |
| C-6630 | Me | CH₂ | H | CH₂CF₃ | CH₂OC(=O)Me | Cl | CH₂CN | O |
| C-6631 | H | CH(Me) | H | CH₂CF₃ | CH₂OC(=O)Me | Cl | CH₂CN | O |
| C-6632 | H | CH₂CH₂ | Me | CH₂CF₃ | CH₂OC(=O)Me | Cl | CH₂CN | O |
| C-6633 | Me | CH₂CH₂ | H | CH₂CF₃ | CH₂OC(=O)Me | Cl | CH₂CN | O |
| C-6634 | H | single bond | Me | CH₂CF₃ | CH₂OC(=O)Me | Me | CH₂CN | O |
| C-6635 | Me | single bond | H | CH₂CF₃ | CH₂OC(=O)Me | Me | CH₂CN | O |
| C-6636 | Me(R) | single bond | H | CH₂CF₃ | CH₂OC(=O)Me | Me | CH₂CN | O |
| C-6637 | Me(S) | single bond | H | CH₂CF₃ | CH₂OC(=O)Me | Me | CH₂CN | O |
| C-6638 | H | CH₂ | Me | CH₂CF₃ | CH₂OC(=O)Me | Me | CH₂CN | O |
| C-6639 | Me | CH₂ | H | CH₂CF₃ | CH₂OC(=O)Me | Me | CH₂CN | O |
| C-6640 | H | CH(Me) | H | CH₂CF₃ | CH₂OC(=O)Me | Me | CH₂CN | O |
| C-6641 | H | CH₂CH₂ | Me | CH₂CF₃ | CH₂OC(=O)Me | Me | CH₂CN | O |
| C-6642 | Me | CH₂CH₂ | H | CH₂CF₃ | CH₂OC(=O)Me | Me | CH₂CN | O |
| C-6643 | H | single bond | Me | CH₂CF₃ | CH₂OC(=O)(*i*-Pr) | Cl | CH₂CN | O |
| C-6644 | Me | single bond | H | CH₂CF₃ | CH₂OC(=O)(*i*-Pr) | Cl | CH₂CN | O |
| C-6645 | Me(R) | single bond | H | CH₂CF₃ | CH₂OC(=O)(*i*-Pr) | Cl | CH₂CN | O |
| C-6646 | Me(S) | single bond | H | CH₂CF₃ | CH₂OC(=O)(*i*-Pr) | Cl | CH₂CN | O |
| C-6647 | H | CH₂ | Me | CH₂CF₃ | CH₂OC(=O)(*i*-Pr) | Cl | CH₂CN | O |
| C-6648 | Me | CH₂ | H | CH₂CF₃ | CH₂OC(=O)(*i*-Pr) | Cl | CH₂CN | O |
| C-6649 | H | CH(Me) | H | CH₂CF₃ | CH₂OC(=O)(*i*-Pr) | Cl | CH₂CN | O |
| C-6650 | H | CH₂CH₂ | Me | CH₂CF₃ | CH₂OC(=O)(*i*-Pr) | Cl | CH₂CN | O |

**[Table 379]**

| Compound Number | Y | W | Z | R¹ | R² | R³ | R¹⁰ | A |
|---|---|---|---|---|---|---|---|---|
| C-6651 | Me | CH₂CH₂ | H | CH₂CF₃ | CH₂OC(=O)(*i* -Pr) | Cl | CH₂CN | O |
| C-6652 | H | single bond | Me | CH₂CF₃ | CH₂OC(=O)(*i*-Pr) | Me | CH₂CN | O |
| C-6653 | Me | single bond | H | CH₂CF₃ | CH₂OC(=O)(*i*-Pr) | Me | CH₂CN | O |
| C-6654 | Me(R) | single bond | H | CH₂CF₃ | CH₂OC(=O)(*i*-Pr) | Me | CH₂CN | O |
| C-6655 | Me(S) | single bond | H | CH₂CF₃ | CH₂OC(=O)(*i*-Pr) | Me | CH₂CN | O |
| C-6656 | H | CH₂ | Me | CH₂CF₃ | CH₂OC(=O)(*i*-Pr) | Me | CH₂CN | O |
| C-6657 | Me | CH₂ | H | CH₂CF₃ | CH₂OC(=O)(*i*-Pr) | Me | CH₂CN | O |
| C-6658 | H | CH(Me) | H | CH₂CF₃ | CH₂OC(=O)(*i*-Pr) | Me | CH₂CN | O |
| C-6659 | H | CH₂CH₂ | Me | CH₂CF₃ | CH₂OC(=O)(*i*-Pr) | Me | CH₂CN | O |
| C-6660 | Me | CH₂CH₂ | H | CH₂CF₃ | CH₂OC(=O)(*i*-Pr) | Me | CH₂CN | O |
| C-6661 | H | single bond | Me | CH₂CF₃ | CH₂OC(=O)(*t*-Bu) | Cl | CH₂CN | O |
| C-6662 | Me | single bond | H | CH₂CF₃ | CH₂OC(=O)(*t*-Bu) | Cl | CH₂CN | O |
| C-6663 | Me(R) | single bond | H | CH₂CF₃ | CH₂OC(=O)(*t*-Bu) | Cl | CH₂CN | O |
| C-6664 | Me(S) | single bond | H | CH₂CF₃ | CH₂OC(=O)(*t*-Bu) | Cl | CH₂CN | O |
| C-6665 | H | CH₂ | Me | CH₂CF₃ | CH₂OC(=O)(*t*-Bu) | Cl | CH₂CN | O |
| C-6666 | Me | CH₂ | H | CH₂CF₃ | CH₂OC(=O)(*t*-Bu) | Cl | CH₂CN | O |
| C-6667 | H | CH(Me) | H | CH₂CF₃ | CH₂OC(=O)(*t*-Bu) | Cl | CH₂CN | O |
| C-6668 | H | CH₂CH₂ | Me | CH₂CF₃ | CH₂OC(=O)(*t*-Bu) | Cl | CH₂CN | O |
| C-6669 | Me | CH₂CH₂ | H | CH₂CF₃ | CH₂OC(=O)(*t*-Bu) | Cl | CH₂CN | O |
| C-6670 | H | single bond | Me | CH₂CF₃ | CH₂OC(=O)(*t*-Bu) | Me | CH₂CN | O |
| C-6671 | Me | single bond | H | CH₂CF₃ | CH₂OC(=O)(*t*-Bu) | Me | CH₂CN | O |
| C-6672 | Me(R) | single bond | H | CH₂CF₃ | CH₂OC(=O)(*t*-Bu) | Me | CH₂CN | O |
| C-6673 | Me(S) | single bond | H | CH₂CF₃ | CH₂OC(=O)(*t*-Bu) | Me | CH₂CN | O |
| C-6674 | H | CH₂ | Me | CH₂CF₃ | CH₂OC(=O)(*t*-Bu) | Me | CH₂CN | O |
| C-6675 | Me | CH₂ | H | CH₂CF₃ | CH₂OC(=O)(*t*-Bu) | Me | CH₂CN | O |
| C-6676 | H | CH(Me) | H | CH₂CF₃ | CH₂OC(=O)(*t*-Bu) | Me | CH₂CN | O |
| C-6677 | H | CH₂CH₂ | Me | CH₂CF₃ | CH₂OC(=O)(*t*-Bu) | Me | CH₂CN | O |
| C-6678 | Me | CH₂CH₂ | H | CH₂CF₃ | CH₂OC(=O)(*t*-Bu) | Me | CH₂CN | O |
| C-6679 | H | single bond | Me | CH₂CF₃ | CH₂OC(=O)OEt | Cl | CH₂CN | O |
| C-6680 | Me | single bond | H | CH₂CF₃ | CH₂OC(=O)OEt | Cl | CH₂CN | O |
| C-6681 | Me(R) | single bond | H | CH₂CF₃ | CH₂OC(=O)OEt | Cl | CH₂CN | O |
| C-6682 | Me(S) | single bond | H | CH₂CF₃ | CH₂OC(=O)OEt | Cl | CH₂CN | O |
| C-6683 | H | CH₂ | Me | CH₂CF₃ | CH₂OC(=O)OEt | Cl | CH₂CN | O |
| C-6684 | Me | CH₂ | H | CH₂CF₃ | CH₂OC(=O)OEt | Cl | CH₂CN | O |
| C-6685 | H | CH(Me) | H | CH₂CF₃ | CH₂OC(=O)OEt | Cl | CH₂CN | O |
| C-6686 | H | CH₂CH₂ | Me | CH₂CF₃ | CH₂OC(=O)OEt | Cl | CH₂CN | O |
| C-6687 | Me | CH₂CH₂ | H | CH₂CF₃ | CH₂OC(=O)OEt | Cl | CH₂CN | O |
| C-6688 | H | single bond | Me | CH₂CF₃ | CH₂OC(=O)OEt | Me | CH₂CN | O |
| C-6689 | Me | single bond | H | CH₂CF₃ | CH₂OC(=O)OEt | Me | CH₂CN | O |
| C-6690 | Me(R) | single bond | H | CH₂CF₃ | CH₂OC(=O)OEt | Me | CH₂CN | O |
| C-6691 | Me(S) | single bond | H | CH₂CF₃ | CH₂OC(=O)OEt | Me | CH₂CN | O |
| C-6692 | H | CH₂ | Me | CH₂CF₃ | CH₂OC(=O)OEt | Me | CH₂CN | O |
| C-6693 | Me | CH₂ | H | CH₂CF₃ | CH₂OC(=O)OEt | Me | CH₂CN | O |
| C-6694 | H | CH(Me) | H | CH₂CF₃ | CH₂OC(=O)OEt | Me | CH₂CN | O |

**[Table 380]**

| Compound Number | Y | W | Z | R¹ | R² | R³ | R¹⁰ | A |
|---|---|---|---|---|---|---|---|---|
| C-6695 | H | CH₂CH₂ | Me | CH₂CF₃ | CH₂OC(=O)OEt | Me | CH₂CN | O |
| C-6696 | Me | CH₂CH₂ | H | CH₂CF₃ | CH₂OC(=O)OEt | Me | CH₂CN | O |
| C-6697 | H | single bond | Me | CH₂CF₃ | CH₂OC(=O)O(*i*-Pr) | Cl | CH₂CN | O |
| C-6698 | Me | single bond | H | CH₂CF₃ | CH₂OC(=O)O(*i*-Pr) | Cl | CH₂CN | O |
| C-6699 | Me(R) | single bond | H | CH₂CF₃ | CH₂OC(=O)O(*i*-Pr) | Cl | CH₂CN | O |
| C-6700 | Me(S) | single bond | H | CH₂CF₃ | CH₂OC(=O)O(*i*-Pr) | Cl | CH₂CN | O |
| C-6701 | H | CH₂ | Me | CH₂CF₃ | CH₂OC(=O)O(*i*-Pr) | Cl | CH₂CN | O |
| C-6702 | Me | CH₂ | H | CH₂CF₃ | CH₂OC(=O)O(*i*-Pr) | Cl | CH₂CN | O |
| C-6703 | H | CH(Me) | H | CH₂CF₃ | CH₂OC(=O)O(*i*-Pr) | Cl | CH₂CN | O |
| C-6704 | H | CH₂CH₂ | Me | CH₂CF₃ | CH₂OC(=O)O(*i*-Pr) | Cl | CH₂CN | O |
| C-6705 | Me | CH₂CH₂ | H | CH₂CF₃ | CH₂OC(=O)O(*i*-Pr) | Cl | CH₂CN | O |
| C-6706 | H | single bond | Me | CH₂CF₃ | CH₂OC(=O)O(*i*-Pr) | Me | CH₂CN | O |
| C-6707 | Me | single bond | H | CH₂CF₃ | CH₂OC(=O)O(*i*-Pr) | Me | CH₂CN | O |
| C-6708 | Me(R) | single bond | H | CH₂CF₃ | CH₂OC(=O)O(*i*-Pr) | Me | CH₂CN | O |
| C-6709 | Me(S) | single bond | H | CH₂CF₃ | CH₂OC(=O)O(*i*-Pr) | Me | CH₂CN | O |
| C-6710 | H | CH₂ | Me | CH₂CF₃ | CH₂OC(=O)O(*i*-Pr) | Me | CH₂CN | O |
| C-6711 | Me | CH₂ | H | CH₂CF₃ | CH₂OC(=O)O(*i*-Pr) | Me | CH₂CN | O |
| C-6712 | H | CH(Me) | H | CH₂CF₃ | CH₂OC(=O)O(*i*-Pr) | Me | CH₂CN | O |
| C-6713 | H | CH₂CH₂ | Me | CH₂CF₃ | CH₂OC(=O)O(*i*-Pr) | Me | CH₂CN | O |
| C-6714 | Me | CH₂CH₂ | H | CH₂CF₃ | CH₂OC(=O)O(*i*-Pr) | Me | CH₂CN | O |
| C-6715 | H | single bond | Me | CH₂CF₃ | CH₂N(Me)C(=O)OMe | Cl | CH₂CN | O |
| C-6716 | Me | single bond | H | CH₂CF₃ | CH₂N(Me)C(=O)OMe | Cl | CH₂CN | O |
| C-6717 | Me(R) | single bond | H | CH₂CF₃ | CH₂N(Me)C(=O)OMe | Cl | CH₂CN | O |
| C-6718 | Me(S) | single bond | H | CH₂CF₃ | CH₂N(Me)C(=O)OMe | Cl | CH₂CN | O |
| C-6719 | H | CH₂ | Me | CH₂CF₃ | CH₂N(Me)C(=O)OMe | Cl | CH₂CN | O |
| C-6720 | Me | CH₂ | H | CH₂CF₃ | CH₂N(Me)C(=O)OMe | Cl | CH₂CN | O |
| C-6721 | H | CH(Me) | H | CH₂CF₃ | CH₂N(Me)C(=O)OMe | Cl | CH₂CN | O |
| C-6722 | H | CH₂CH₂ | Me | CH₂CF₃ | CH₂N(Me)C(=O)OMe | Cl | CH₂CN | O |
| C-6723 | Me | CH₂CH₂ | H | CH₂CF₃ | CH₂N(Me)C(=O)OMe | Cl | CH₂CN | O |
| C-6724 | H | single bond | Me | CH₂CF₃ | CH₂N(Me)C(=O)OMe | Me | CH₂CN | O |
| C-6725 | Me | single bond | H | CH₂CF₃ | CH₂N(Me)C(=O)OMe | Me | CH₂CN | O |
| C-6726 | Me(R) | single bond | H | CH₂CF₃ | CH₂N(Me)C(=O)OMe | Me | CH₂CN | O |
| C-6727 | Me(S) | single bond | H | CH₂CF₃ | CH₂N(Me)C(=O)OMe | Me | CH₂CN | O |
| C-6728 | H | CH₂ | Me | CH₂CF₃ | CH₂N(Me)C(=O)OMe | Me | CH₂CN | O |
| C-6729 | Me | CH₂ | H | CH₂CF₃ | CH₂N(Me)C(=O)OMe | Me | CH₂CN | O |
| C-6730 | H | CH(Me) | H | CH₂CF₃ | CH₂N(Me)C(=O)OMe | Me | CH₂CN | O |
| C-6731 | H | CH₂CH₂ | Me | CH₂CF₃ | CH₂N(Me)C(=O)OMe | Me | CH₂CN | O |
| C-6732 | Me | CH₂CH₂ | H | CH₂CF₃ | CH₂N(Me)C(=O)OMe | Me | CH₂CN | O |
| C-6733 | H | single bond | Me | CH₂CF₃ | C(=O)OCH₂CH=CH₂ | Cl | C(=O)Me | O |
| C-6734 | Me | single bond | H | CH₂CF₃ | C(=O)OCH₂CH=CH₂ | Cl | C(=O)Me | O |
| C-6735 | Me(R) | single bond | H | CH₂CF₃ | C(=O)OCH₂CH=CH₂ | Cl | C(=O)Me | O |
| C-6736 | Me(S) | single bond | H | CH₂CF₃ | C(=O)OCH₂CH=CH₂ | Cl | C(=O)Me | O |
| C-6737 | H | CH₂ | Me | CH₂CF₃ | C(=O)OCH₂CH=CH₂ | Cl | C(=O)Me | O |
| C-6738 | Me | CH₂ | H | CH₂CF₃ | C(=O)OCH₂CH=CH₂ | Cl | C(=O)Me | O |

**[Table 381]**

| Compound Number | Y | W | Z | R¹ | R² | R³ | R¹⁰ | A |
|---|---|---|---|---|---|---|---|---|
| C-6739 | H | CH(Me) | H | CH₂CF₃ | C(=O)OCH₂CH=CH₂ | Cl | C(=O)Me | O |
| C-6740 | H | CH₂CH₂ | Me | CH₂CF₃ | C(=O)OCH₂CH=CH₂ | Cl | C(=O)Me | O |
| C-6741 | Me | CH₂CH₂ | H | CH₂CF₃ | C(=O)OCH₂CH=CH₂ | Cl | C(=O)Me | O |
| C-6742 | H | single bond | Me | CH₂CF₃ | C(=O)OCH₂CH=CH₂ | Me | C(=O)Me | O |
| C-6743 | Me | single bond | H | CH₂CF₃ | C(=O)OCH₂CH=CH₂ | Me | C(=O)Me | O |
| C-6744 | Me(R) | single bond | H | CH₂CF₃ | C(=O)OCH₂CH=CH₂ | Me | C(=O)Me | O |
| C-6745 | Me(S) | single bond | H | CH₂CF₃ | C(=O)OCH₂CH=CH₂ | Me | C(=O)Me | O |
| C-6746 | H | CH₂ | Me | CH₂CF₃ | C(=O)OCH₂CH=CH₂ | Me | C(=O)Me | O |
| C-6747 | Me | CH₂ | H | CH₂CF₃ | C(=O)OCH₂CH=CH₂ | Me | C(=O)Me | O |
| C-6748 | H | CH(Me) | H | CH₂CF₃ | C(=O)OCH₂CH=CH₂ | Me | C(=O)Me | O |
| C-6749 | H | CH₂CH₂ | Me | CH₂CF₃ | C(=O)OCH₂CH=CH₂ | Me | C(=O)Me | O |
| C-6750 | Me | CH₂CH₂ | H | CH₂CF₃ | C(=O)OCH₂CH=CH₂ | Me | C(=O)Me | O |
| C-6751 | H | single bond | Me | CH₂CF₃ | C(=O)OCH₂CCl₃ | Cl | C(=O)Me | O |
| C-6752 | Me | single bond | H | CH₂CF₃ | C(=O)OCH₂CCl₃ | Cl | C(=O)Me | O |
| C-6753 | Me(R) | single bond | H | CH₂CF₃ | C(=O)OCH₂CCl₃ | Cl | C(=O)Me | O |
| C-6754 | Me(S) | single bond | H | CH₂CF₃ | C(=O)OCH₂CCl₃ | Cl | C(=O)Me | O |
| C-6755 | H | CH₂ | Me | CH₂CF₃ | C(=O)OCH₂CCl₃ | Cl | C(=O)Me | O |
| C-6756 | Me | CH₂ | H | CH₂CF₃ | C(=O)OCH₂CCl₃ | Cl | C(=O)Me | O |
| C-6757 | H | CH(Me) | H | CH₂CF₃ | C(=O)OCH₂CCl₃ | Cl | C(=O)Me | O |
| C-6758 | H | CH₂CH₂ | Me | CH₂CF₃ | C(=O)OCH₂CCl₃ | Cl | C(=O)Me | O |
| C-6759 | Me | CH₂CH₂ | H | CH₂CF₃ | C(=O)OCH₂CCl₃ | Cl | C(=O)Me | O |
| C-6760 | H | single bond | Me | CH₂CF₃ | C(=O)OCH₂CCl₃ | Me | C(=O)Me | O |
| C-6761 | Me | single bond | H | CH₂CF₃ | C(=O)OCH₂CCl₃ | Me | C(=O)Me | O |
| C-6762 | Me(R) | single bond | H | CH₂CF₃ | C(=O)OCH₂CCl₃ | Me | C(=O)Me | O |
| C-6763 | Me(S) | single bond | H | CH₂CF₃ | C(=O)OCH₂CCl₃ | Me | C(=O)Me | O |
| C-6764 | H | CH₂ | Me | CH₂CF₃ | C(=O)OCH₂CCl₃ | Me | C(=O)Me | O |
| C-6765 | Me | CH₂ | H | CH₂CF₃ | C(=O)OCH₂CCl₃ | Me | C(=O)Me | O |
| C-6766 | H | CH(Me) | H | CH₂CF₃ | C(=O)OCH₂CCl₃ | Me | C(=O)Me | O |
| C-6767 | H | CH₂CH₂ | Me | CH₂CF₃ | C(=O)OCH₂CCl₃ | Me | C(=O)Me | O |
| C-6768 | Me | CH₂CH₂ | H | CH₂CF₃ | C(=O)OCH₂CCl₃ | Me | C(=O)Me | O |
| C-6769 | H | single bond | Me | CH₂CF₃ | C(=O)N(Me)₂ | Cl | C(=O)Me | O |
| C-6770 | Me | single bond | H | CH₂CF₃ | C(=O)N(Me)₂ | Cl | C(=O)Me | O |
| C-6771 | Me(R) | single bond | H | CH₂CF₃ | C(=O)N(Me)₂ | Cl | C(=O)Me | O |
| C-6772 | Me(S) | single bond | H | CH₂CF₃ | C(=O)N(Me)₂ | Cl | C(=O)Me | O |
| C-6773 | H | CH₂ | Me | CH₂CF₃ | C(=O)N(Me)₂ | Cl | C(=O)Me | O |
| C-6774 | Me | CH₂ | H | CH₂CF₃ | C(=O)N(Me)₂ | Cl | C(=O)Me | O |
| C-6775 | H | CH(Me) | H | CH₂CF₃ | C(=O)N(Me)₂ | Cl | C(=O)Me | O |
| C-6776 | H | CH₂CH₂ | Me | CH₂CF₃ | C(=O)N(Me)₂ | Cl | C(=O)Me | O |
| C-6777 | Me | CH₂CH₂ | H | CH₂CF₃ | C(=O)N(Me)₂ | Cl | C(=O)Me | O |
| C-6778 | H | single bond | Me | CH₂CF₃ | C(=O)N(Me)₂ | Me | C(=O)Me | O |
| C-6779 | Me | single bond | H | CH₂CF₃ | C(=O)N(Me)₂ | Me | C(=O)Me | O |
| C-6780 | Me(R) | single bond | H | CH₂CF₃ | C(=O)N(Me)₂ | Me | C(=O)Me | O |
| C-6781 | Me(S) | single bond | H | CH₂CF₃ | C(=O)N(Me)₂ | Me | C(=O)Me | O |
| C-6782 | H | CH₂ | Me | CH₂CF₃ | C(=O)N(Me)₂ | Me | C(=O)Me | O |

**[Table 382]**

| Compound Number | Y | W | Z | R¹ | R² | R³ | R¹⁰ | A |
|---|---|---|---|---|---|---|---|---|
| C-6783 | Me | CH₂ | H | CH₂CF₃ | C(=O)N(Me)₂ | Me | C(=O)Me | O |
| C-6784 | H | CH(Me) | H | CH₂CF₃ | C(=O)N(Me)₂ | Me | C(=O)Me | O |
| C-6785 | H | CH₂CH₂ | Me | CH₂CF₃ | C(=O)N(Me)₂ | Me | C(=O)Me | O |
| C-6786 | Me | CH₂CH₂ | H | CH₂CF₃ | C(=O)N(Me)₂ | Me | C(=O)Me | O |
| C-6787 | H | single bond | Me | CH₂CF₃ | CH₂OC(=O)Me | Cl | C(=O)Me | O |
| C-6788 | Me | single bond | H | CH₂CF₃ | CH₂OC(=O)Me | Cl | C(=O)Me | O |
| C-6789 | Me(R) | single bond | H | CH₂CF₃ | CH₂OC(=O)Me | Cl | C(=O)Me | O |
| C-6790 | Me(S) | single bond | H | CH₂CF₃ | CH₂OC(=O)Me | Cl | C(=O)Me | O |
| C-6791 | H | CH₂ | Me | CH₂CF₃ | CH₂OC(=O)Me | Cl | C(=O)Me | O |
| C-6792 | Me | CH₂ | H | CH₂CF₃ | CH₂OC(=O)Me | Cl | C(=O)Me | O |
| C-6793 | H | CH(Me) | H | CH₂CF₃ | CH₂OC(=O)Me | Cl | C(=O)Me | O |
| C-6794 | H | CH₂CH₂ | Me | CH₂CF₃ | CH₂OC(=O)Me | Cl | C(=O)Me | O |
| C-6795 | Me | CH₂CH₂ | H | CH₂CF₃ | CH₂OC(=O)Me | Cl | C(=O)Me | O |
| C-6796 | H | single bond | Me | CH₂CF₃ | CH₂OC(=O)Me | Me | C(=O)Me | O |
| C-6797 | Me | single bond | H | CH₂CF₃ | CH₂OC(=O)Me | Me | C(=O)Me | O |
| C-6798 | Me(R) | single bond | H | CH₂CF₃ | CH₂OC(=O)Me | Me | C(=O)Me | O |
| C-6799 | Me(S) | single bond | H | CH₂CF₃ | CH₂OC(=O)Me | Me | C(=O)Me | O |
| C-6800 | H | CH₂ | Me | CH₂CF₃ | CH₂OC(=O)Me | Me | C(=O)Me | O |
| C-6801 | Me | CH₂ | H | CH₂CF₃ | CH₂OC(=O)Me | Me | C(=O)Me | O |
| C-6802 | H | CH(Me) | H | CH₂CF₃ | CH₂OC(=O)Me | Me | C(=O)Me | O |
| C-6803 | H | CH₂CH₂ | Me | CH₂CF₃ | CH₂OC(=O)Me | Me | C(=O)Me | O |
| C-6804 | Me | CH₂CH₂ | H | CH₂CF₃ | CH₂OC(=O)Me | Me | C(=O)Me | O |
| C-6805 | H | single bond | Me | CH₂CF₃ | CH₂OC(=O)(*i*-Pr) | Cl | C(=O)Me | O |
| C-6806 | Me | single bond | H | CH₂CF₃ | CH₂OC(=O)(*i*-Pr) | Cl | C(=O)Me | O |
| C-6807 | Me(R) | single bond | H | CH₂CF₃ | CH₂OC(=O)(*i* -Pr) | Cl | C(=O)Me | O |
| C-6808 | Me(S) | single bond | H | CH₂CF₃ | CH₂OC(=O)(*i* -Pr) | Cl | C(=O)Me | O |
| C-6809 | H | CH₂ | Me | CH₂CF₃ | CH₂OC(=O)(*i*-Pr) | Cl | C(=O)Me | O |
| C-6810 | Me | CH₂ | H | CH₂CF₃ | CH₂OC(=O)(*i*-Pr) | Cl | C(=O)Me | O |
| C-6811 | H | CH(Me) | H | CH₂CF₃ | CH₂OC(=O)(*i* -Pr) | Cl | C(=O)Me | O |
| C-6812 | H | CH₂CH₂ | Me | CH₂CF₃ | CH₂OC(=O)(*i* -Pr) | Cl | C(=O)Me | O |
| C-6813 | Me | CH₂CH₂ | H | CH₂CF₃ | CH₂OC(=O)(*i*-Pr) | Cl | C(=O)Me | O |
| C-6814 | H | single bond | Me | CH₂CF₃ | CH₂OC(=O)(*i*-Pr) | Me | C(=O)Me | O |
| C-6815 | Me | single bond | H | CH₂CF₃ | CH₂OC(=O)(*i*-Pr) | Me | C(=O)Me | O |
| C-6816 | Me(R) | single bond | H | CH₂CF₃ | CH₂OC(=O)(*i*-Pr) | Me | C(=O)Me | O |
| C-6817 | Me(S) | single bond | H | CH₂CF₃ | CH₂OC(=O)(*i*-Pr) | Me | C(=O)Me | O |
| C-6818 | H | CH₂ | Me | CH₂CF₃ | CH₂OC(=O)(*i*-Pr) | Me | C(=O)Me | O |
| C-6819 | Me | CH₂ | H | CH₂CF₃ | CH₂OC(=O)(*i*-Pr) | Me | C(=O)Me | O |
| C-6820 | H | CH(Me) | H | CH₂CF₃ | CH₂OC(=O)(*i*-Pr) | Me | C(=O)Me | O |
| C-6821 | H | CH₂CH₂ | Me | CH₂CF₃ | CH₂OC(=O)(*i*-Pr) | Me | C(=O)Me | O |
| C-6822 | Me | CH₂CH₂ | H | CH₂CF₃ | CH₂OC(=O)(*i*-Pr) | Me | C(=O)Me | O |
| C-6823 | H | single bond | Me | CH₂CF₃ | CH₂OC(=O)(*t*-Bu) | Cl | C(=O)Me | O |
| C-6824 | Me | single bond | H | CH₂CF₃ | CH₂OC(=O)(*t*-Bu) | Cl | C(=O)Me | O |
| C-6825 | Me(R) | single bond | H | CH₂CF₃ | CH₂OC(=O)(*t*-Bu) | Cl | C(=O)Me | O |
| C-6826 | Me(S) | single bond | H | CH₂CF₃ | CH₂OC(=O)(*t*-Bu) | Cl | C(=O)Me | O |

**[Table 383]**

| Compound Number | Y | W | Z | R¹ | R² | R³ | R¹⁰ | A |
|---|---|---|---|---|---|---|---|---|
| C-6827 | H | CH₂ | Me | CH₂CF₃ | CH₂OC(=O)(*t*-Bu) | Cl | C(=O)Me | O |
| C-6828 | Me | CH₂ | H | CH₂CF₃ | CH₂OC(=O)(*t*-Bu) | Cl | C(=O)Me | O |
| C-6829 | H | CH(Me) | H | CH₂CF₃ | CH₂OC(=O)(*t*-Bu) | Cl | C(=O)Me | O |
| C-6830 | H | CH₂CH₂ | Me | CH₂CF₃ | CH₂OC(=O)(*t*-Bu) | Cl | C(=O)Me | O |
| C-6831 | Me | CH₂CH₂ | H | CH₂CF₃ | CH₂OC(=O)(*t*-Bu) | Cl | C(=O)Me | O |
| C-6832 | H | single bond | Me | CH₂CF₃ | CH₂OC(=O)(*t*-Bu) | Me | C(=O)Me | O |
| C-6833 | Me | single bond | H | CH₂CF₃ | CH₂OC(=O)(*t*-Bu) | Me | C(=O)Me | O |
| C-6834 | Me(R) | single bond | H | CH₂CF₃ | CH₂OC(=O)(*t*-Bu) | Me | C(=O)Me | O |
| C-6835 | Me(S) | single bond | H | CH₂CF₃ | CH₂OC(=O)(*t*-Bu) | Me | C(=O)Me | O |
| C-6836 | H | CH₂ | Me | CH₂CF₃ | CH₂OC(=O)(*t*-Bu) | Me | C(=O)Me | O |
| C-6837 | Me | CH₂ | H | CH₂CF₃ | CH₂OC(=O)(*t*-Bu) | Me | C(=O)Me | O |
| C-6838 | H | CH(Me) | H | CH₂CF₃ | CH₂OC(=O)(*t*-Bu) | Me | C(=O)Me | O |
| C-6839 | H | CH₂CH₂ | Me | CH₂CF₃ | CH₂OC(=O)(*t*-Bu) | Me | C(=O)Me | O |
| C-6840 | Me | CH₂CH₂ | H | CH₂CF₃ | CH₂OC(=O)(*t*-Bu) | Me | C(=O)Me | O |
| C-6841 | H | single bond | Me | CH₂CF₃ | CH₂OC(=O)OEt | Cl | C(=O)Me | O |
| C-6842 | Me | single bond | H | CH₂CF₃ | CH₂OC(=O)OEt | Cl | C(=O)Me | O |
| C-6843 | Me(R) | single bond | H | CH₂CF₃ | CH₂OC(=O)OEt | Cl | C(=O)Me | O |
| C-6844 | Me(S) | single bond | H | CH₂CF₃ | CH₂OC(=O)OEt | Cl | C(=O)Me | O |
| C-6845 | H | CH₂ | Me | CH₂CF₃ | CH₂OC(=O)OEt | Cl | C(=O)Me | O |
| C-6846 | Me | CH₂ | H | CH₂CF₃ | CH₂OC(=O)OEt | Cl | C(=O)Me | O |
| C-6847 | H | CH(Me) | H | CH₂CF₃ | CH₂OC(=O)OEt | Cl | C(=O)Me | O |
| C-6848 | H | CH₂CH₂ | Me | CH₂CF₃ | CH₂OC(=O)OEt | Cl | C(=O)Me | O |
| C-6849 | Me | CH₂CH₂ | H | CH₂CF₃ | CH₂OC(=O)OEt | Cl | C(=O)Me | O |
| C-6850 | H | single bond | Me | CH₂CF₃ | CH₂OC(=O)OEt | Me | C(=O)Me | O |
| C-6851 | Me | single bond | H | CH₂CF₃ | CH₂OC(=O)OEt | Me | C(=O)Me | O |
| C-6852 | Me(R) | single bond | H | CH₂CF₃ | CH₂OC(=O)OEt | Me | C(=O)Me | O |
| C-6853 | Me(S) | single bond | H | CH₂CF₃ | CH₂OC(=O)OEt | Me | C(=O)Me | O |
| C-6854 | H | CH₂ | Me | CH₂CF₃ | CH₂OC(=O)OEt | Me | C(=O)Me | O |
| C-6855 | Me | CH₂ | H | CH₂CF₃ | CH₂OC(=O)OEt | Me | C(=O)Me | O |
| C-6856 | H | CH(Me) | H | CH₂CF₃ | CH₂OC(=O)OEt | Me | C(=O)Me | O |
| C-6857 | H | CH₂CH₂ | Me | CH₂CF₃ | CH₂OC(=O)OEt | Me | C(=O)Me | O |
| C-6858 | Me | CH₂CH₂ | H | CH₂CF₃ | CH₂OC(=O)OEt | Me | C(=O)Me | O |
| C-6859 | H | single bond | Me | CH₂CF₃ | CH₂OC(=O)O(*i*-Pr) | Cl | C(=O)Me | O |
| C-6860 | Me | single bond | H | CH₂CF₃ | CH₂OC(=O)O(*i*-Pr) | Cl | C(=O)Me | O |
| C-6861 | Me(R) | single bond | H | CH₂CF₃ | CH₂OC(=O)O(*i*-Pr) | Cl | C(=O)Me | O |
| C-6862 | Me(S) | single bond | H | CH₂CF₃ | CH₂OC(=O)O(*i*-Pr) | Cl | C(=O)Me | O |
| C-6863 | H | CH₂ | Me | CH₂CF₃ | CH₂OC(=O)O(*i*-Pr) | Cl | C(=O)Me | O |
| C-6864 | Me | CH₂ | H | CH₂CF₃ | CH₂OC(=O)O(*i*-Pr) | Cl | C(=O)Me | O |
| C-6865 | H | CH(Me) | H | CH₂CF₃ | CH₂OC(=O)O(*i*-Pr) | Cl | C(=O)Me | O |
| C-6866 | H | CH₂CH₂ | Me | CH₂CF₃ | CH₂OC(=O)O(*i*-Pr) | Cl | C(=O)Me | O |
| C-6867 | Me | CH₂CH₂ | H | CH₂CF₃ | CH₂OC(=O)O(*i*-Pr) | Cl | C(=O)Me | O |
| C-6868 | H | single bond | Me | CH₂CF₃ | CH₂OC(=O)O(*i*-Pr) | Me | C(=O)Me | O |
| C-6869 | Me | single bond | H | CH₂CF₃ | CH₂OC(=O)O(*i*-Pr) | Me | C(=O)Me | O |
| C-6870 | Me(R) | single bond | H | CH₂CF₃ | CH₂OC(=O)O(*i*-Pr) | Me | C(=O)Me | O |

**[Table 384]**

| Compound Number | Y | W | Z | R¹ | R² | R³ | R¹⁰ | A |
|---|---|---|---|---|---|---|---|---|
| C-6871 | Me(S) | single bond | H | CH₂CF₃ | CH₂OC(=O)O(*i*-Pr) | Me | C(=O)Me | O |
| C-6872 | H | CH₂ | Me | CH₂CF₃ | CH₂OC(=O)O(*i*-Pr) | Me | C(=O)Me | O |
| C-6873 | Me | CH₂ | H | CH₂CF₃ | CH₂OC(=O)O(*i*-Pr) | Me | C(=O)Me | O |
| C-6874 | H | CH(Me) | H | CH₂CF₃ | CH₂OC(=O)O(*i*-Pr) | Me | C(=O)Me | O |
| C-6875 | H | CH₂CH₂ | Me | CH₂CF₃ | CH₂OC(=O)O(*i*-Pr) | Me | C(=O)Me | O |
| C-6876 | Me | CH₂CH₂ | H | CH₂CF₃ | CH₂OC(=O)O(*i*-Pr) | Me | C(=O)Me | O |
| C-6877 | H | single bond | Me | CH₂CF₃ | CH₂N(Me)C(=O)OMe | Cl | C(=O)Me | O |
| C-6878 | Me | single bond | H | CH₂CF₃ | CH₂N(Me)C(=O)OMe | Cl | C(=O)Me | O |
| C-6879 | Me(R) | single bond | H | CH₂CF₃ | CH₂N(Me)C(=O)OMe | Cl | C(=O)Me | O |
| C-6880 | Me(S) | single bond | H | CH₂CF₃ | CH₂N(Me)C(=O)OMe | Cl | C(=O)Me | O |
| C-6881 | H | CH₂ | Me | CH₂CF₃ | CH₂N(Me)C(=O)OMe | Cl | C(=O)Me | O |
| C-6882 | Me | CH₂ | H | CH₂CF₃ | CH₂N(Me)C(=O)OMe | Cl | C(=O)Me | O |
| C-6883 | H | CH(Me) | H | CH₂CF₃ | CH₂N(Me)C(=O)OMe | Cl | C(=O)Me | O |
| C-6884 | H | CH₂CH₂ | Me | CH₂CF₃ | CH₂N(Me)C(=O)OMe | Cl | C(=O)Me | O |
| C-6885 | Me | CH₂CH₂ | H | CH₂CF₃ | CH₂N(Me)C(=O)OMe | Cl | C(=O)Me | O |
| C-6886 | H | single bond | Me | CH₂CF₃ | CH₂N(Me)C(=O)OMe | Me | C(=O)Me | O |
| C-6887 | Me | single bond | H | CH₂CF₃ | CH₂N(Me)C(=O)OMe | Me | C(=O)Me | O |
| C-6888 | Me(R) | single bond | H | CH₂CF₃ | CH₂N(Me)C(=O)OMe | Me | C(=O)Me | O |
| C-6889 | Me(S) | single bond | H | CH₂CF₃ | CH₂N(Me)C(=O)OMe | Me | C(=O)Me | O |
| C-6890 | H | CH₂ | Me | CH₂CF₃ | CH₂N(Me)C(=O)OMe | Me | C(=O)Me | O |
| C-6891 | Me | CH₂ | H | CH₂CF₃ | CH₂N(Me)C(=O)OMe | Me | C(=O)Me | O |
| C-6892 | H | CH(Me) | H | CH₂CF₃ | CH₂N(Me)C(=O)OMe | Me | C(=O)Me | O |
| C-6893 | H | CH₂CH₂ | Me | CH₂CF₃ | CH₂N(Me)C(=O)OMe | Me | C(=O)Me | O |
| C-6894 | Me | CH₂CH₂ | H | CH₂CF₃ | CH₂N(Me)C(=O)OMe | Me | C(=O)Me | O |
| C-6895 | H | single bond | Me | Et | C(=O)OCH₂CH=CH₂ | Cl | H | O |
| C-6896 | Me | single bond | H | Et | C(=O)OCH₂CH=CH₂ | Cl | H | O |
| C-6897 | Me(R) | single bond | H | Et | C(=O)OCH₂CH=CH₂ | Cl | H | O |
| C-6898 | Me(S) | single bond | H | Et | C(=O)OCH₂CH=CH₂ | Cl | H | O |
| C-6899 | H | CH₂ | Me | Et | C(=O)OCH₂CH=CH₂ | Cl | H | O |
| C-6900 | Me | CH₂ | H | Et | C(=O)OCH₂CH=CH₂ | Cl | H | O |
| C-6901 | H | CH(Me) | H | Et | C(=O)OCH₂CH=CH₂ | Cl | H | O |
| C-6902 | H | CH₂CH₂ | Me | Et | C(=O)OCH₂CH=CH₂ | Cl | H | O |
| C-6903 | Me | CH₂CH₂ | H | Et | C(=O)OCH₂CH=CH₂ | Cl | H | O |
| C-6904 | H | single bond | Me | Et | C(=O)OCH₂CH=CH₂ | Me | H | O |
| C-6905 | Me | single bond | H | Et | C(=O)OCH₂CH=CH₂ | Me | H | O |
| C-6906 | Me(R) | single bond | H | Et | C(=O)OCH₂CH=CH₂ | Me | H | O |
| C-6907 | Me(S) | single bond | H | Et | C(=O)OCH₂CH=CH₂ | Me | H | O |
| C-6908 | H | CH₂ | Me | Et | C(=O)OCH₂CH=CH₂ | Me | H | O |
| C-6909 | Me | CH₂ | H | Et | C(=O)OCH₂CH=CH₂ | Me | H | O |
| C-6910 | H | CH(Me) | H | Et | C(=O)OCH₂CH=CH₂ | Me | H | O |
| C-6911 | H | CH₂CH₂ | Me | Et | C(=O)OCH₂CH=CH₂ | Me | H | O |
| C-6912 | Me | CH₂CH₂ | H | Et | C(=O)OCH₂CH=CH₂ | Me | H | O |
| C-6913 | H | single bond | Me | Et | C(=O)OCH₂CCl₃ | Cl | H | O |
| C-6914 | Me | single bond | H | Et | C(=O)OCH₂CCl₃ | Cl | H | O |

**[Table 385]**

| Compound Number | Y | W | Z | R¹ | R² | R³ | R¹⁰ | A |
|---|---|---|---|---|---|---|---|---|
| C-6915 | Me(R) | single bond | H | Et | C(=O)OCH₂CCl₃ | Cl | H | O |
| C-6916 | Me(S) | single bond | H | Et | C(=O)OCH₂CCl₃ | Cl | H | O |
| C-6917 | H | CH₂ | Me | Et | C(=O)OCH₂CCl₃ | Cl | H | O |
| C-6918 | Me | CH₂ | H | Et | C(=O)OCH₂CCl₃ | Cl | H | O |
| C-6919 | H | CH(Me) | H | Et | C(=O)OCH₂CCl₃ | Cl | H | O |
| C-6920 | H | CH₂CH₂ | Me | Et | C(=O)OCH₂CCl₃ | Cl | H | O |
| C-6921 | Me | CH₂CH₂ | H | Et | C(=O)OCH₂CCl₃ | Cl | H | O |
| C-6922 | H | single bond | Me | Et | C(=O)OCH₂CCl₃ | Me | H | O |
| C-6923 | Me | single bond | H | Et | C(=O)OCH₂CCl₃ | Me | H | O |
| C-6924 | Me(R) | single bond | H | Et | C(=O)OCH₂CCl₃ | Me | H | O |
| C-6925 | Me(S) | single bond | H | Et | C(=O)OCH₂CCl₃ | Me | H | O |
| C-6926 | H | CH₂ | Me | Et | C(=O)OCH₂CCl₃ | Me | H | O |
| C-6927 | Me | CH₂ | H | Et | C(=O)OCH₂CCl₃ | Me | H | O |
| C-6928 | H | CH(Me) | H | Et | C(=O)OCH₂CCl₃ | Me | H | O |
| C-6929 | H | CH₂CH₂ | Me | Et | C(=O)OCH₂CCl₃ | Me | H | O |
| C-6930 | Me | CH₂CH₂ | H | Et | C(=O)OCH₂CCl₃ | Me | H | O |
| C-6931 | H | single bond | Me | Et | C(=O)N(Me)₂ | Cl | H | O |
| C-6932 | Me | single bond | H | Et | C(=O)N(Me)₂ | Cl | H | O |
| C-6933 | Me(R) | single bond | H | Et | C(=O)N(Me)₂ | Cl | H | O |
| C-6934 | Me(S) | single bond | H | Et | C(=O)N(Me)₂ | Cl | H | O |
| C-6935 | H | CH₂ | Me | Et | C(=O)N(Me)₂ | Cl | H | O |
| C-6936 | Me | CH₂ | H | Et | C(=O)N(Me)₂ | Cl | H | O |
| C-6937 | H | CH(Me) | H | Et | C(=O)N(Me)₂ | Cl | H | O |
| C-6938 | H | CH₂CH₂ | Me | Et | C(=O)N(Me)₂ | Cl | H | O |
| C-6939 | Me | CH₂CH₂ | H | Et | C(=O)N(Me)₂ | Cl | H | O |
| C-6940 | H | single bond | Me | Et | C(=O)N(Me)₂ | Me | H | O |
| C-6941 | Me | single bond | H | Et | C(=O)N(Me)₂ | Me | H | O |
| C-6942 | Me(R) | single bond | H | Et | C(=O)N(Me)₂ | Me | H | O |
| C-6943 | Me(S) | single bond | H | Et | C(=O)N(Me)₂ | Me | H | O |
| C-6944 | H | CH₂ | Me | Et | C(=O)N(Me)₂ | Me | H | O |
| C-6945 | Me | CH₂ | H | Et | C(=O)N(Me)₂ | Me | H | O |
| C-6946 | H | CH(Me) | H | Et | C(=O)N(Me)₂ | Me | H | O |
| C-6947 | H | CH₂CH₂ | Me | Et | C(=O)N(Me)₂ | Me | H | O |
| C-6948 | Me | CH₂CH₂ | H | Et | C(=O)N(Me)₂ | Me | H | O |
| C-6949 | H | single bond | Me | Et | CH₂OC(=O)Me | Cl | H | O |
| C-6950 | Me | single bond | H | Et | CH₂OC(=O)Me | Cl | H | O |
| C-6951 | Me(R) | single bond | H | Et | CH₂OC(=O)Me | Cl | H | O |
| C-6952 | Me(S) | single bond | H | Et | CH₂OC(=O)Me | Cl | H | O |
| C-6953 | H | CH₂ | Me | Et | CH₂OC(=O)Me | Cl | H | O |
| C-6954 | Me | CH₂ | H | Et | CH₂OC(=O)Me | Cl | H | O |
| C-6955 | H | CH(Me) | H | Et | CH₂OC(=O)Me | Cl | H | O |
| C-6956 | H | CH₂CH₂ | Me | Et | CH₂OC(=O)Me | Cl | H | O |
| C-6957 | Me | CH₂CH₂ | H | Et | CH₂OC(=O)Me | Cl | H | O |
| C-6958 | H | single bond | Me | Et | CH₂OC(=O)Me | Me | H | O |

**[Table 386]**

| Compound Number | Y | W | Z | R¹ | R² | R³ | R¹⁰ | A |
|---|---|---|---|---|---|---|---|---|
| C-6959 | Me | single bond | H | Et | CH₂OC(=O)Me | Me | H | O |
| C-6960 | Me(R) | single bond | H | Et | CH₂OC(=O)Me | Me | H | O |
| C-6961 | Me(S) | single bond | H | Et | CH₂OC(=O)Me | Me | H | O |
| C-6962 | H | CH₂ | Me | Et | CH₂OC(=O)Me | Me | H | O |
| C-6963 | Me | CH₂ | H | Et | CH₂OC(=O)Me | Me | H | O |
| C-6964 | H | CH(Me) | H | Et | CH₂OC(=O)Me | Me | H | O |
| C-6965 | H | CH₂CH₂ | Me | Et | CH₂OC(=O)Me | Me | H | O |
| C-6966 | Me | CH₂CH₂ | H | Et | CH₂OC(=O)Me | Me | H | O |
| C-6967 | H | single bond | Me | Et | CH₂OC(=O)(*i*-Pr) | Cl | H | O |
| C-6968 | Me | single bond | H | Et | CH₂OC(=O)(*i*-Pr) | Cl | H | O |
| C-6969 | Me(R) | single bond | H | Et | CH₂OC(=O)(*i*-Pr) | Cl | H | O |
| C-6970 | Me(S) | single bond | H | Et | CH₂OC(=O)(*i*-Pr) | Cl | H | O |
| C-6971 | H | CH₂ | Me | Et | CH₂OC(=O)(*i*-Pr) | Cl | H | O |
| C-6972 | Me | CH₂ | H | Et | CH₂OC(=O)(*i*-Pr) | Cl | H | O |
| C-6973 | H | CH(Me) | H | Et | CH₂OC(=O)(*i*-Pr) | Cl | H | O |
| C-6974 | H | CH₂CH₂ | Me | Et | CH₂OC(=O)(*i*-Pr) | Cl | H | O |
| C-6975 | Me | CH₂CH₂ | H | Et | CH₂OC(=O)(*i*-Pr) | Cl | H | O |
| C-6976 | H | single bond | Me | Et | CH₂OC(=O)(-Pr) | Me | H | O |
| C-6977 | Me | single bond | H | Et | CH₂OC(=O)(*i*-Pr) | Me | H | O |
| C-6978 | Me(R) | single bond | H | Et | CH₂OC(=O)(*i*-Pr) | Me | H | O |
| C-6979 | Me(S) | single bond | H | Et | CH₂OC(=O)(*i*-Pr) | Me | H | O |
| C-6980 | H | CH₂ | Me | Et | CH₂OC(=O)(*i-*Pr) | Me | H | O |
| C-6981 | Me | CH₂ | H | Et | CH₂OC(=O)(*i*-Pr) | Me | H | O |
| C-6982 | H | CH(Me) | H | Et | CH₂OC(=O)(*i*-Pr) | Me | H | O |
| C-6983 | H | CH₂CH₂ | Me | Et | CH₂OC(=O)(*i*-Pr) | Me | H | O |
| C-6984 | Me | CH₂CH₂ | H | Et | CH₂OC(=O)(*i*-Pr) | Me | H | O |
| C-6985 | H | single bond | Me | Et | CH₂OC(=O)(*t*-Bu) | Cl | H | O |
| C-6986 | Me | single bond | H | Et | CH₂OC(=O)(*t*-Bu) | Cl | H | O |
| C-6987 | Me(R) | single bond | H | Et | CH₂OC(=O)(*t*-Bu) | Cl | H | O |
| C-6988 | Me(S) | single bond | H | Et | CH₂OC(=O)(*t*-Bu) | Cl | H | O |
| C-6989 | H | CH₂ | Me | Et | CH₂OC(=O)(*t*-Bu) | Cl | H | O |
| C-6990 | Me | CH₂ | H | Et | CH₂OC(=O)(*t*-Bu) | Cl | H | O |
| C-6991 | H | CH(Me) | H | Et | CH₂OC(=O)(*t*-Bu) | Cl | H | O |
| C-6992 | H | CH₂CH₂ | Me | Et | CH₂OC(=O)(*t*-Bu) | Cl | H | O |
| C-6993 | Me | CH₂CH₂ | H | Et | CH₂OC(=O)(*t*-Bu) | Cl | H | O |
| C-6994 | H | single bond | Me | Et | CH₂OC(=O)(*t*-Bu) | Me | H | O |
| C-6995 | Me | single bond | H | Et | CH₂OC(=O)(*t*-Bu) | Me | H | O |
| C-6996 | Me(R) | single bond | H | Et | CH₂OC(=O)(*t*-Bu) | Me | H | O |
| C-6997 | Me(S) | single bond | H | Et | CH₂OC(=O)(*t*-Bu) | Me | H | O |
| C-6998 | H | CH₂ | Me | Et | CH₂OC(=O)(*t*-Bu) | Me | H | O |
| C-6999 | Me | CH₂ | H | Et | CH₂OC(=O)(*t*-Bu) | Me | H | O |
| C-7000 | H | CH(Me) | H | Et | CH₂OC(=O)(*t*-Bu) | Me | H | O |
| C-7001 | H | CH₂CH₂ | Me | Et | CH₂OC(=O)(*t*-Bu) | Me | H | O |
| C-7002 | Me | CH₂CH₂ | H | Et | CH₂OC(=O)(*t*-Bu) | Me | H | O |

**[Table 387]**

| Compound Number | Y | W | Z | R¹ | R² | R³ | R¹⁰ | A |
|---|---|---|---|---|---|---|---|---|
| C-7003 | H | single bond | Me | Et | CH₂OC(=O)OEt | Cl | H | O |
| C-7004 | Me | single bond | H | Et | CH₂OC(=O)OEt | Cl | H | O |
| C-7005 | Me(R) | single bond | H | Et | CH₂OC(=O)OEt | Cl | H | O |
| C-7006 | Me(S) | single bond | H | Et | CH₂OC(=O)OEt | Cl | H | O |
| C-7007 | H | CH₂ | Me | Et | CH₂OC(=O)OEt | Cl | H | O |
| C-7008 | Me | CH₂ | H | Et | CH₂OC(=O)OEt | Cl | H | O |
| C-7009 | H | CH(Me) | H | Et | CH₂OC(=O)OEt | Cl | H | O |
| C-7010 | H | CH₂CH₂ | Me | Et | CH₂OC(=O)OEt | Cl | H | O |
| C-7011 | Me | CH₂CH₂ | H | Et | CH₂OC(=O)OEt | Cl | H | O |
| C-7012 | H | single bond | Me | Et | CH₂OC(=O)OEt | Me | H | O |
| C-7013 | Me | single bond | H | Et | CH₂OC(=O)OEt | Me | H | O |
| C-7014 | Me(R) | single bond | H | Et | CH₂OC(=O)OEt | Me | H | O |
| C-7015 | Me(S) | single bond | H | Et | CH₂OC(=O)OEt | Me | H | O |
| C-7016 | H | CH₂ | Me | Et | CH₂OC(=O)OEt | Me | H | O |
| C-7017 | Me | CH₂ | H | Et | CH₂OC(=O)OEt | Me | H | O |
| C-7018 | H | CH(Me) | H | Et | CH₂OC(=O)OEt | Me | H | O |
| C-7019 | H | CH₂CH₂ | Me | Et | CH₂OC(=O)OEt | Me | H | O |
| C-7020 | Me | CH₂CH₂ | H | Et | CH₂OC(=O)OEt | Me | H | O |
| C-7021 | H | single bond | Me | Et | CH₂OC(=O)O(*i*-Pr) | Cl | H | O |
| C-7022 | Me | single bond | H | Et | CH₂OC(=O)O(*i*-Pr) | Cl | H | O |
| C-7023 | Me(R) | single bond | H | Et | CH₂OC(=O)O(*i*-Pr) | Cl | H | O |
| C-7024 | Me(S) | single bond | H | Et | CH₂OC(=O)O(*i*-Pr) | Cl | H | O |
| C-7025 | H | CH₂ | Me | Et | CH₂OC(=O)O(*i*-Pr) | Cl | H | O |
| C-7026 | Me | CH₂ | H | Et | CH₂OC(=O)O(*i*-Pr) | Cl | H | O |
| C-7027 | H | CH(Me) | H | Et | CH₂OC(=O)O(*i*-Pr) | Cl | H | O |
| C-7028 | H | CH₂CH₂ | Me | Et | CH₂OC(=O)O(*i*-Pr) | Cl | H | O |
| C-7029 | Me | CH₂CH₂ | H | Et | CH₂OC(=O)O(*i*-Pr) | Cl | H | O |
| C-7030 | H | single bond | Me | Et | CH₂OC(=O)O(*i*-Pr) | Me | H | O |
| C-7031 | Me | single bond | H | Et | CH₂OC(=O)O(*i*-Pr) | Me | H | O |
| C-7032 | Me(R) | single bond | H | Et | CH₂OC(=O)O(*i*-Pr) | Me | H | O |
| C-7033 | Me(S) | single bond | H | Et | CH₂OC(=O)O(*i*-Pr) | Me | H | O |
| C-7034 | H | CH₂ | Me | Et | CH₂OC(=O)O(*i*-Pr) | Me | H | O |
| C-7035 | Me | CH₂ | H | Et | CH₂OC(=O)O(*i*-Pr) | Me | H | O |
| C-7036 | H | CH(Me) | H | Et | CH₂OC(=O)O(*i*-Pr) | Me | H | O |
| C-7037 | H | CH₂CH₂ | Me | Et | CH₂OC(=O)O(*i*-Pr) | Me | H | O |
| C-7038 | Me | CH₂CH₂ | H | Et | CH₂OC(=O)O(*i*-Pr) | Me | H | O |
| C-7039 | H | single bond | Me | Et | CH₂N(Me)C(=O)OMe | Cl | H | O |
| C-7040 | Me | single bond | H | Et | CH₂N(Me)C(=O)OMe | Cl | H | O |
| C-7041 | Me(R) | single bond | H | Et | CH₂N(Me)C(=O)OMe | Cl | H | O |
| C-7042 | Me(S) | single bond | H | Et | CH₂N(Me)C(=O)OMe | Cl | H | O |
| C-7043 | H | CH₂ | Me | Et | CH₂N(Me)C(=O)OMe | Cl | H | O |
| C-7044 | Me | CH₂ | H | Et | CH₂N(Me)C(=O)OMe | Cl | H | O |
| C-7045 | H | CH(Me) | H | Et | CH₂N(Me)C(=O)OMe | Cl | H | O |
| C-7046 | H | CH₂CH₂ | Me | Et | CH₂N(Me)C(=O)OMe | Cl | H | O |

**[Table 388]**

| Compound Number | Y | W | Z | R¹ | R² | R³ | R¹⁰ | A |
|---|---|---|---|---|---|---|---|---|
| C-7047 | Me | CH₂CH₂ | H | Et | CH₂N(Me)C(=O)OMe | Cl | H | O |
| C-7048 | H | single bond | Me | Et | CH₂N(Me)C(=O)OMe | Me | H | O |
| C-7049 | Me | single bond | H | Et | CH₂N(Me)C(=O)OMe | Me | H | O |
| C-7050 | Me(R) | single bond | H | Et | CH₂N(Me)C(=O)OMe | Me | H | O |
| C-7051 | Me(S) | single bond | H | Et | CH₂N(Me)C(=O)OMe | Me | H | O |
| C-7052 | H | CH₂ | Me | Et | CH₂N(Me)C(=O)OMe | Me | H | O |
| C-7053 | Me | CH₂ | H | Et | CH₂N(Me)C(=O)OMe | Me | H | O |
| C-7054 | H | CH(Me) | H | Et | CH₂N(Me)C(=O)OMe | Me | H | O |
| C-7055 | H | CH₂CH₂ | Me | Et | CH₂N(Me)C(=O)OMe | Me | H | O |
| C-7056 | Me | CH₂CH₂ | H | Et | CH₂N(Me)C(=O)OMe | Me | H | O |
| C-7057 | H | single bond | Me | Et | C(=O)OCH₂CH=CH₂ | Cl | Me | O |
| C-7058 | Me | single bond | H | Et | C(=O)OCH₂CH=CH₂ | Cl | Me | O |
| C-7059 | Me(R) | single bond | H | Et | C(=O)OCH₂CH=CH₂ | Cl | Me | O |
| C-7060 | Me(S) | single bond | H | Et | C(=O)OCH₂CH=CH₂ | Cl | Me | O |
| C-7061 | H | CH₂ | Me | Et | C(=O)OCH₂CH=CH₂ | Cl | Me | O |
| C-7062 | Me | CH₂ | H | Et | C(=O)OCH₂CH=CH₂ | Cl | Me | O |
| C-7063 | H | CH(Me) | H | Et | C(=O)OCH₂CH=CH₂ | Cl | Me | O |
| C-7064 | H | CH₂CH₂ | Me | Et | C(=O)OCH₂CH=CH₂ | Cl | Me | O |
| C-7065 | Me | CH₂CH₂ | H | Et | C(=O)OCH₂CH=CH₂ | Cl | Me | O |
| C-7066 | H | single bond | Me | Et | C(=O)OCH₂CH=CH₂ | Me | Me | O |
| C-7067 | Me | single bond | H | Et | C(=O)OCH₂CH=CH₂ | Me | Me | O |
| C-7068 | Me(R) | single bond | H | Et | C(=O)OCH₂CH=CH₂ | Me | Me | O |
| C-7069 | Me(S) | single bond | H | Et | C(=O)OCH₂CH=CH₂ | Me | Me | O |
| C-7070 | H | CH₂ | Me | Et | C(=O)OCH₂CH=CH₂ | Me | Me | O |
| C-7071 | Me | CH₂ | H | Et | C(=O)OCH₂CH=CH₂ | Me | Me | O |
| C-7072 | H | CH(Me) | H | Et | C(=O)OCH₂CH=CH₂ | Me | Me | O |
| C-7073 | H | CH₂CH₂ | Me | Et | C(=O)OCH₂CH=CH₂ | Me | Me | O |
| C-7074 | Me | CH₂CH₂ | H | Et | C(=O)OCH₂CH=CH₂ | Me | Me | O |
| C-7075 | H | single bond | Me | Et | C(=O)OCH₂CCl₃ | Cl | Me | O |
| C-7076 | Me | single bond | H | Et | C(=O)OCH₂CCl₃ | Cl | Me | O |
| C-7077 | Me(R) | single bond | H | Et | C(=O)OCH₂CCl₃ | Cl | Me | O |
| C-7078 | Me(S) | single bond | H | Et | C(=O)OCH₂CCl₃ | Cl | Me | O |
| C-7079 | H | CH₂ | Me | Et | C(=O)OCH₂CCl₃ | Cl | Me | O |
| C-7080 | Me | CH₂ | H | Et | C(=O)OCH₂CCl₃ | Cl | Me | O |
| C-7081 | H | CH(Me) | H | Et | C(=O)OCH₂CCl₃ | Cl | Me | O |
| C-7082 | H | CH₂CH₂ | Me | Et | C(=O)OCH₂CCl₃ | Cl | Me | O |
| C-7083 | Me | CH₂CH₂ | H | Et | C(=O)OCH₂CCl₃ | Cl | Me | O |
| C-7084 | H | single bond | Me | Et | C(=O)OCH₂CCl₃ | Me | Me | O |
| C-7085 | Me | single bond | H | Et | C(=O)OCH₂CCl₃ | Me | Me | O |
| C-7086 | Me(R) | single bond | H | Et | C(=O)OCH₂CCl₃ | Me | Me | O |
| C-7087 | Me(S) | single bond | H | Et | C(=O)OCH₂CCl₃ | Me | Me | O |
| C-7088 | H | CH₂ | Me | Et | C(=O)OCH₂CCl₃ | Me | Me | O |
| C-7089 | Me | CH₂ | H | Et | C(=O)OCH₂CCl₃ | Me | Me | O |
| C-7090 | H | CH(Me) | H | Et | C(=O)OCH₂CCl₃ | Me | Me | O |

**[Table 389]**

| Compound Number | Y | W | Z | R¹ | R² | R³ | R¹⁰ | A |
|---|---|---|---|---|---|---|---|---|
| C-7091 | H | CH₂CH₂ | Me | Et | C(=O)OCH₂CCl₃ | Me | Me | O |
| C-7092 | Me | CH₂CH₂ | H | Et | C(=O)OCH₂CCl₃ | Me | Me | O |
| C-7093 | H | single bond | Me | Et | C(=O)N(Me)₂ | Cl | Me | O |
| C-7094 | Me | single bond | H | Et | C(=O)N(Me)₂ | Cl | Me | O |
| C-7095 | Me(R) | single bond | H | Et | C(=O)N(Me)₂ | Cl | Me | O |
| C-7096 | Me(S) | single bond | H | Et | C(=O)N(Me)₂ | Cl | Me | O |
| C-7097 | H | CH₂ | Me | Et | C(=O)N(Me)₂ | Cl | Me | O |
| C-7098 | Me | CH₂ | H | Et | C(=O)N(Me)₂ | Cl | Me | O |
| C-7099 | H | CH(Me) | H | Et | C(=O)N(Me)₂ | Cl | Me | O |
| C-7100 | H | CH₂CH₂ | Me | Et | C(=O)N(Me)₂ | Cl | Me | O |
| C-7101 | Me | CH₂CH₂ | H | Et | C(=O)N(Me)₂ | Cl | Me | O |
| C-7102 | H | single bond | Me | Et | C(=O)N(Me)₂ | Me | Me | O |
| C-7103 | Me | single bond | H | Et | C(=O)N(Me)₂ | Me | Me | O |
| C-7104 | Me(R) | single bond | H | Et | C(=O)N(Me)₂ | Me | Me | O |
| C-7105 | Me(S) | single bond | H | Et | C(=O)N(Me)₂ | Me | Me | O |
| C-7106 | H | CH₂ | Me | Et | C(=O)N(Me)₂ | Me | Me | O |
| C-7107 | Me | CH₂ | H | Et | C(=O)N(Me)₂ | Me | Me | O |
| C-7108 | H | CH(Me) | H | Et | C(=O)N(Me)₂ | Me | Me | O |
| C-7109 | H | CH₂CH₂ | Me | Et | C(=O)N(Me)₂ | Me | Me | O |
| C-7110 | Me | CH₂CH₂ | H | Et | C(=O)N(Me)₂ | Me | Me | O |
| C-7111 | H | single bond | Me | Et | CH₂OC(=O)Me | Cl | Me | O |
| C-7112 | Me | single bond | H | Et | CH₂OC(=O)Me | Cl | Me | O |
| C-7113 | Me(R) | single bond | H | Et | CH₂OC(=O)Me | Cl | Me | O |
| C-7114 | Me(S) | single bond | H | Et | CH₂OC(=O)Me | Cl | Me | O |
| C-7115 | H | CH₂ | Me | Et | CH₂OC(=O)Me | Cl | Me | O |
| C-7116 | Me | CH₂ | H | Et | CH₂OC(=O)Me | Cl | Me | O |
| C-7117 | H | CH(Me) | H | Et | CH₂OC(=O)Me | Cl | Me | O |
| C-7118 | H | CH₂CH₂ | Me | Et | CH₂OC(=O)Me | Cl | Me | O |
| C-7119 | Me | CH₂CH₂ | H | Et | CH₂OC(=O)Me | Cl | Me | O |
| C-7120 | H | single bond | Me | Et | CH₂OC(=O)Me | Me | Me | O |
| C-7121 | Me | single bond | H | Et | CH₂OC(=O)Me | Me | Me | O |
| C-7122 | Me(R) | single bond | H | Et | CH₂OC(=O)Me | Me | Me | O |
| C-7123 | Me(S) | single bond | H | Et | CH₂OC(=O)Me | Me | Me | O |
| C-7124 | H | CH₂ | Me | Et | CH₂OC(=O)Me | Me | Me | O |
| C-7125 | Me | CH₂ | H | Et | CH₂OC(=O)Me | Me | Me | O |
| C-7126 | H | CH(Me) | H | Et | CH₂OC(=O)Me | Me | Me | O |
| C-7127 | H | CH₂CH₂ | Me | Et | CH₂OC(=O)Me | Me | Me | O |
| C-7128 | Me | CH₂CH₂ | H | Et | CH₂OC(=O)Me | Me | Me | O |
| C-7129 | H | single bond | Me | Et | CH₂OC(=O)(*i*-Pr) | Cl | Me | O |
| C-7130 | Me | single bond | H | Et | CH₂OC(=O)(*i*-Pr) | Cl | Me | O |
| C-7131 | Me(R) | single bond | H | Et | CH₂OC(=O)(*i*-Pr) | Cl | Me | O |
| C-7132 | Me(S) | single bond | H | Et | CH₂OC(=O)(*i*-Pr) | Cl | Me | O |
| C-7133 | H | CH₂ | Me | Et | CH₂OC(=O)(*i*-Pr) | Cl | Me | O |
| C-7134 | Me | CH₂ | H | Et | CH₂OC(=O)(*i*-Pr) | Cl | Me | O |

**[Table 390]**

| Compound Number | Y | W | Z | R¹ | R² | R³ | R¹⁰ | A |
|---|---|---|---|---|---|---|---|---|
| C-7135 | H | CH(Me) | H | Et | CH₂OC(=O)(*i*-Pr) | Cl | Me | O |
| C-7136 | H | CH₂CH₂ | Me | Et | CH₂OC(=O)(*i*-Pr) | Cl | Me | O |
| C-7137 | Me | CH₂CH₂ | H | Et | CH₂OC(=O)(*i*-Pr) | Cl | Me | O |
| C-7138 | H | single bond | Me | Et | CH₂OC(=O)(*i*-Pr) | Me | Me | O |
| C-7139 | Me | single bond | H | Et | CH₂OC(=O)(*i*-Pr) | Me | Me | O |
| C-7140 | Me(R) | single bond | H | Et | CH₂OC(=O)(*i*-Pr) | Me | Me | O |
| C-7141 | Me(S) | single bond | H | Et | CH₂OC(=O)(*i*-Pr) | Me | Me | O |
| C-7142 | H | CH₂ | Me | Et | CH₂OC(=O)(*i*-Pr) | Me | Me | O |
| C-7143 | Me | CH₂ | H | Et | CH₂OC(=O)(*i*-Pr) | Me | Me | O |
| C-7144 | H | CH(Me) | H | Et | CH₂OC(=O)(*i*-Pr) | Me | Me | O |
| C-7145 | H | CH₂CH₂ | Me | Et | CH₂OC(=O)(*i*-Pr) | Me | Me | O |
| C-7146 | Me | CH₂CH₂ | H | Et | CH₂OC(=O)(*i*-Pr) | Me | Me | O |
| C-7147 | H | single bond | Me | Et | CH₂OC(=O)(*t*-Bu) | Cl | Me | O |
| C-7148 | Me | single bond | H | Et | CH₂OC(=O)(*t*-Bu) | Cl | Me | O |
| C-7149 | Me(R) | single bond | H | Et | CH₂OC(=O)(*t*-Bu) | Cl | Me | O |
| C-7150 | Me(S) | single bond | H | Et | CH₂OC(=O)(*t*-Bu) | Cl | Me | O |
| C-7151 | H | CH₂ | Me | Et | CH₂OC(=O)(*t*-Bu) | Cl | Me | O |
| C-7152 | Me | CH₂ | H | Et | CH₂OC(=O)(*t*-Bu) | Cl | Me | O |
| C-7153 | H | CH(Me) | H | Et | CH₂OC(=O)(*t*-Bu) | Cl | Me | O |
| C-7154 | H | CH₂CH₂ | Me | Et | CH₂OC(=O)(*t*-Bu) | Cl | Me | O |
| C-7155 | Me | CH₂CH₂ | H | Et | CH₂OC(=O)(*t*-Bu) | Cl | Me | O |
| C-7156 | H | single bond | Me | Et | CH₂OC(=O)(*t*-Bu) | Me | Me | O |
| C-7157 | Me | single bond | H | Et | CH₂OC(=O)(*t*-Bu) | Me | Me | O |
| C-7158 | Me(R) | single bond | H | Et | CH₂OC(=O)(*t*-Bu) | Me | Me | O |
| C-7159 | Me(S) | single bond | H | Et | CH₂OC(=O)(*t*-Bu) | Me | Me | O |
| C-7160 | H | CH₂ | Me | Et | CH₂OC(=O)(*t*-Bu) | Me | Me | O |
| C-7161 | Me | CH₂ | H | Et | CH₂OC(=O)(*t*-Bu) | Me | Me | O |
| C-7162 | H | CH(Me) | H | Et | CH₂OC(=O)(*t*-Bu) | Me | Me | O |
| C-7163 | H | CH₂CH₂ | Me | Et | CH₂OC(=O)(*t*-Bu) | Me | Me | O |
| C-7164 | Me | CH₂CH₂ | H | Et | CH₂OC(=O)(*t*-Bu) | Me | Me | O |
| C-7165 | H | single bond | Me | Et | CH₂OC(=O)OEt | Cl | Me | O |
| C-7166 | Me | single bond | H | Et | CH₂OC(=O)OEt | Cl | Me | O |
| C-7167 | Me(R) | single bond | H | Et | CH₂OC(=O)OEt | Cl | Me | O |
| C-7168 | Me(S) | single bond | H | Et | CH₂OC(=O)OEt | Cl | Me | O |
| C-7169 | H | CH₂ | Me | Et | CH₂OC(=O)OEt | Cl | Me | O |
| C-7170 | Me | CH₂ | H | Et | CH₂OC(=O)OEt | Cl | Me | O |
| C-7171 | H | CH(Me) | H | Et | CH₂OC(=O)OEt | Cl | Me | O |
| C-7172 | H | CH₂CH₂ | Me | Et | CH₂OC(=O)OEt | Cl | Me | O |
| C-7173 | Me | CH₂CH₂ | H | Et | CH₂OC(=O)OEt | Cl | Me | O |
| C-7174 | H | single bond | Me | Et | CH₂OC(=O)OEt | Me | Me | O |
| C-7175 | Me | single bond | H | Et | CH₂OC(=O)OEt | Me | Me | O |
| C-7176 | Me(R) | single bond | H | Et | CH₂OC(=O)OEt | Me | Me | O |
| C-7177 | Me(S) | single bond | H | Et | CH₂OC(=O)OEt | Me | Me | O |
| C-7178 | H | CH₂ | Me | Et | CH₂OC(=O)OEt | Me | Me | O |

**[Table 391]**

| Compound Number | Y | W | Z | R¹ | R² | R³ | R¹⁰ | A |
|---|---|---|---|---|---|---|---|---|
| C-7179 | Me | CH₂ | H | Et | CH₂OC(=O)OEt | Me | Me | O |
| C-7180 | H | CH(Me) | H | Et | CH₂OC(=O)OEt | Me | Me | O |
| C-7181 | H | CH₂CH₂ | Me | Et | CH₂OC(=O)OEt | Me | Me | O |
| C-7182 | Me | CH₂CH₂ | H | Et | CH₂OC(=O)OEt | Me | Me | O |
| C-7183 | H | single bond | Me | Et | CH₂OC(=O)O(*i*-Pr) | Cl | Me | O |
| C-7184 | Me | single bond | H | Et | CH₂OC(=O)O(*i*-Pr) | Cl | Me | O |
| C-7185 | Me(R) | single bond | H | Et | CH₂OC(=O)O(*i*-Pr) | Cl | Me | O |
| C-7186 | Me(S) | single bond | H | Et | CH₂OC(=O)O(*i*-Pr) | Cl | Me | O |
| C-7187 | H | CH₂ | Me | Et | CH₂OC(=O)O(*i*-Pr) | Cl | Me | O |
| C-7188 | Me | CH₂ | H | Et | CH₂OC(=O)O(*i*-Pr) | Cl | Me | O |
| C-7189 | H | CH(Me) | H | Et | CH₂OC(=O)O(*i*-Pr) | Cl | Me | O |
| C-7190 | H | CH₂CH₂ | Me | Et | CH₂OC(=O)O(*i*-Pr) | Cl | Me | O |
| C-7191 | Me | CH₂CH₂ | H | Et | CH₂OC(=O)O(*i*-Pr) | Cl | Me | O |
| C-7192 | H | single bond | Me | Et | CH₂OC(=O)O(*i*-Pr) | Me | Me | O |
| C-7193 | Me | single bond | H | Et | CH₂OC(=O)O(*i*-Pr) | Me | Me | O |
| C-7194 | Me(R) | single bond | H | Et | CH₂OC(=O)O(*i*-Pr) | Me | Me | O |
| C-7195 | Me(S) | single bond | H | Et | CH₂OC(=O)O(*i*-Pr) | Me | Me | O |
| C-7196 | H | CH₂ | Me | Et | CH₂OC(=O)O(*i*-Pr) | Me | Me | O |
| C-7197 | Me | CH₂ | H | Et | CH₂OC(=O)O(*i*-Pr) | Me | Me | O |
| C-7198 | H | CH(Me) | H | Et | CH₂OC(=O)O(*i*-Pr) | Me | Me | O |
| C-7199 | H | CH₂CH₂ | Me | Et | CH₂OC(=O)O(*i*-Pr) | Me | Me | O |
| C-7200 | Me | CH₂CH₂ | H | Et | CH₂OC(=O)O(*i*-Pr) | Me | Me | O |
| C-7201 | H | single bond | Me | Et | CH₂N(Me)C(=O)OMe | Cl | Me | O |
| C-7202 | Me | single bond | H | Et | CH₂N(Me)C(=O)OMe | Cl | Me | O |
| C-7203 | Me(R) | single bond | H | Et | CH₂N(Me)C(=O)OMe | Cl | Me | O |
| C-7204 | Me(S) | single bond | H | Et | CH₂N(Me)C(=O)OMe | Cl | Me | O |
| C-7205 | H | CH₂ | Me | Et | CH₂N(Me)C(=O)OMe | Cl | Me | O |
| C-7206 | Me | CH₂ | H | Et | CH₂N(Me)C(=O)OMe | Cl | Me | O |
| C-7207 | H | CH(Me) | H | Et | CH₂N(Me)C(=O)OMe | Cl | Me | O |
| C-7208 | H | CH₂CH₂ | Me | Et | CH₂N(Me)C(=O)OMe | Cl | Me | O |
| C-7209 | Me | CH₂CH₂ | H | Et | CH₂N(Me)C(=O)OMe | Cl | Me | O |
| C-7210 | H | single bond | Me | Et | CH₂N(Me)C(=O)OMe | Me | Me | O |
| C-7211 | Me | single bond | H | Et | CH₂N(Me)C(=O)OMe | Me | Me | O |
| C-7212 | Me(R) | single bond | H | Et | CH₂N(Me)C(=O)OMe | Me | Me | O |
| C-7213 | Me(S) | single bond | H | Et | CH₂N(Me)C(=O)OMe | Me | Me | O |
| C-7214 | H | CH₂ | Me | Et | CH₂N(Me)C(=O)OMe | Me | Me | O |
| C-7215 | Me | CH₂ | H | Et | CH₂N(Me)C(=O)OMe | Me | Me | O |
| C-7216 | H | CH(Me) | H | Et | CH₂N(Me)C(=O)OMe | Me | Me | O |
| C-7217 | H | CH₂CH₂ | Me | Et | CH₂N(Me)C(=O)OMe | Me | Me | O |
| C-7218 | Me | CH₂CH₂ | H | Et | CH₂N(Me)C(=O)OMe | Me | Me | O |
| C-7219 | H | single bond | Me | Et | C(=O)OCH₂CH=CH₂ | Cl | CH₂OMe | O |
| C-7220 | Me | single bond | H | Et | C(=O)OCH₂CH=CH₂ | Cl | CH₂OMe | O |
| C-7221 | Me(R) | single bond | H | Et | C(=O)OCH₂CH=CH₂ | Cl | CH₂OMe | O |
| C-7222 | Me(S) | single bond | H | Et | C(=O)OCH₂CH=CH₂ | Cl | CH₂OMe | O |

**[Table 392]**

| Compound Number | Y | W | Z | R¹ | R² | R³ | R¹⁰ | A |
|---|---|---|---|---|---|---|---|---|
| C-7223 | H | CH₂ | Me | Et | C(=O)OCH₂CH=CH₂ | Cl | CH₂OMe | O |
| C-7224 | Me | CH₂ | H | Et | C(=O)OCH₂CH=CH₂ | Cl | CH₂OMe | O |
| C-7225 | H | CH(Me) | H | Et | C(=O)OCH₂CH=CH₂ | Cl | CH₂OMe | O |
| C-7226 | H | CH₂CH₂ | Me | Et | C(=O)OCH₂CH=CH₂ | Cl | CH₂OMe | O |
| C-7227 | Me | CH₂CH₂ | H | Et | C(=O)OCH₂CH=CH₂ | Cl | CH₂OMe | O |
| C-7228 | H | single bond | Me | Et | C(=O)OCH₂CH=CH₂ | Me | CH₂OMe | O |
| C-7229 | Me | single bond | H | Et | C(=O)OCH₂CH=CH₂ | Me | CH₂OMe | O |
| C-7230 | Me(R) | single bond | H | Et | C(=O)OCH₂CH=CH₂ | Me | CH₂OMe | O |
| C-7231 | Me(S) | single bond | H | Et | C(=O)OCH₂CH=CH₂ | Me | CH₂OMe | O |
| C-7232 | H | CH₂ | Me | Et | C(=O)OCH₂CH=CH₂ | Me | CH₂OMe | O |
| C-7233 | Me | CH₂ | H | Et | C(=O)OCH₂CH=CH₂ | Me | CH₂OMe | O |
| C-7234 | H | CH(Me) | H | Et | C(=O)OCH₂CH=CH₂ | Me | CH₂OMe | O |
| C-7235 | H | CH₂CH₂ | Me | Et | C(=O)OCH₂CH=CH₂ | Me | CH₂OMe | O |
| C-7236 | Me | CH₂CH₂ | H | Et | C(=O)OCH₂CH=CH₂ | Me | CH₂OMe | O |
| C-7237 | H | single bond | Me | Et | C(=O)OCH₂CCl₃ | Cl | CH₂OMe | O |
| C-7238 | Me | single bond | H | Et | C(=O)OCH₂CCl₃ | Cl | CH₂OMe | O |
| C-7239 | Me(R) | single bond | H | Et | C(=O)OCH₂CCl₃ | Cl | CH₂OMe | O |
| C-7240 | Me(S) | single bond | H | Et | C(=O)OCH₂CCl₃ | Cl | CH₂OMe | O |
| C-7241 | H | CH₂ | Me | Et | C(=O)OCH₂CCl₃ | Cl | CH₂OMe | O |
| C-7242 | Me | CH₂ | H | Et | C(=O)OCH₂CCl₃ | Cl | CH₂OMe | O |
| C-7243 | H | CH(Me) | H | Et | C(=O)OCH₂CCl₃ | Cl | CH₂OMe | O |
| C-7244 | H | CH₂CH₂ | Me | Et | C(=O)OCH₂CCl₃ | Cl | CH₂OMe | O |
| C-7245 | Me | CH₂CH₂ | H | Et | C(=O)OCH₂CCl₃ | Cl | CH₂OMe | O |
| C-7246 | H | single bond | Me | Et | C(=O)OCH₂CCl₃ | Me | CH₂OMe | O |
| C-7247 | Me | single bond | H | Et | C(=O)OCH₂CCl₃ | Me | CH₂OMe | O |
| C-7248 | Me(R) | single bond | H | Et | C(=O)OCH₂CCl₃ | Me | CH₂OMe | O |
| C-7249 | Me(S) | single bond | H | Et | C(=O)OCH₂CCl₃ | Me | CH₂OMe | O |
| C-7250 | H | CH₂ | Me | Et | C(=O)OCH₂CCl₃ | Me | CH₂OMe | O |
| C-7251 | Me | CH₂ | H | Et | C(=O)OCH₂CCl₃ | Me | CH₂OMe | O |
| C-7252 | H | CH(Me) | H | Et | C(=O)OCH₂CCl₃ | Me | CH₂OMe | O |
| C-7253 | H | CH₂CH₂ | Me | Et | C(=O)OCH₂CCl₃ | Me | CH₂OMe | O |
| C-7254 | Me | CH₂CH₂ | H | Et | C(=O)OCH₂CCl₃ | Me | CH₂OMe | O |
| C-7255 | H | single bond | Me | Et | C(=O)N(Me)₂ | Cl | CH₂OMe | O |
| C-7256 | Me | single bond | H | Et | C(=O)N(Me)₂ | Cl | CH₂OMe | O |
| C-7257 | Me(R) | single bond | H | Et | C(=O)N(Me)₂ | Cl | CH₂OMe | O |
| C-7258 | Me(S) | single bond | H | Et | C(=O)N(Me)₂ | Cl | CH₂OMe | O |
| C-7259 | H | CH₂ | Me | Et | C(=O)N(Me)₂ | Cl | CH₂OMe | O |
| C-7260 | Me | CH₂ | H | Et | C(=O)N(Me)₂ | Cl | CH₂OMe | O |
| C-7261 | H | CH(Me) | H | Et | C(=O)N(Me)₂ | Cl | CH₂OMe | O |
| C-7262 | H | CH₂CH₂ | Me | Et | C(=O)N(Me)₂ | Cl | CH₂OMe | O |
| C-7263 | Me | CH₂CH₂ | H | Et | C(=O)N(Me)₂ | Cl | CH₂OMe | O |
| C-7264 | H | single bond | Me | Et | C(=O)N(Me)₂ | Me | CH₂OMe | O |
| C-7265 | Me | single bond | H | Et | C(=O)N(Me)₂ | Me | CH₂OMe | O |
| C-7266 | Me(R) | single bond | H | Et | C(=O)N(Me)₂ | Me | CH₂OMe | O |

**[Table 393]**

| Compound Number | Y | W | Z | R¹ | R² | R³ | R¹⁰ | A |
|---|---|---|---|---|---|---|---|---|
| C-7267 | Me(S) | single bond | H | Et | C(=O)N(Me)₂ | Me | CH₂OMe | O |
| C-7268 | H | CH₂ | Me | Et | C(=O)N(Me)₂ | Me | CH₂OMe | O |
| C-7269 | Me | CH₂ | H | Et | C(=O)N(Me)₂ | Me | CH₂OMe | O |
| C-7270 | H | CH(Me) | H | Et | C(=O)N(Me)₂ | Me | CH₂OMe | O |
| C-7271 | H | CH₂CH₂ | Me | Et | C(=O)N(Me)₂ | Me | CH₂OMe | O |
| C-7272 | Me | CH₂CH₂ | H | Et | C(=O)N(Me)₂ | Me | CH₂OMe | O |
| C-7273 | H | single bond | Me | Et | CH₂OC(=O)Me | Cl | CH₂OMe | O |
| C-7274 | Me | single bond | H | Et | CH₂OC(=O)Me | Cl | CH₂OMe | O |
| C-7275 | Me(R) | single bond | H | Et | CH₂OC(=O)Me | Cl | CH₂OMe | O |
| C-7276 | Me(S) | single bond | H | Et | CH₂OC(=O)Me | Cl | CH₂OMe | O |
| C-7277 | H | CH₂ | Me | Et | CH₂OC(=O)Me | Cl | CH₂OMe | O |
| C-7278 | Me | CH₂ | H | Et | CH₂OC(=O)Me | Cl | CH₂OMe | O |
| C-7279 | H | CH(Me) | H | Et | CH₂OC(=O)Me | Cl | CH₂OMe | O |
| C-7280 | H | CH₂CH₂ | Me | Et | CH₂OC(=O)Me | Cl | CH₂OMe | O |
| C-7281 | Me | CH₂CH₂ | H | Et | CH₂OC(=O)Me | Cl | CH₂OMe | O |
| C-7282 | H | single bond | Me | Et | CH₂OC(=O)Me | Me | CH₂OMe | O |
| C-7283 | Me | single bond | H | Et | CH₂OC(=O)Me | Me | CH₂OMe | O |
| C-7284 | Me(R) | single bond | H | Et | CH₂OC(=O)Me | Me | CH₂OMe | O |
| C-7285 | Me(S) | single bond | H | Et | CH₂OC(=O)Me | Me | CH₂OMe | O |
| C-7286 | H | CH₂ | Me | Et | CH₂OC(=O)Me | Me | CH₂OMe | O |
| C-7287 | Me | CH₂ | H | Et | CH₂OC(=O)Me | Me | CH₂OMe | O |
| C-7288 | H | CH(Me) | H | Et | CH₂OC(=O)Me | Me | CH₂OMe | O |
| C-7289 | H | CH₂CH₂ | Me | Et | CH₂OC(=O)Me | Me | CH₂OMe | O |
| C-7290 | Me | CH₂CH₂ | H | Et | CH₂OC(=O)Me | Me | CH₂OMe | O |
| C-7291 | H | single bond | Me | Et | CH₂OC(=O)(*i*-Pr) | Cl | CH₂OMe | O |
| C-7292 | Me | single bond | H | Et | CH₂OC(=O)(*i*-Pr) | Cl | CH₂OMe | O |
| C-7293 | Me(R) | single bond | H | Et | CH₂OC(=O)(*i*-Pr) | Cl | CH₂OMe | O |
| C-7294 | Me(S) | single bond | H | Et | CH₂OC(=O)(*i*-Pr) | Cl | CH₂OMe | O |
| C-7295 | H | CH₂ | Me | Et | CH₂OC(=O)(*i*-Pr) | Cl | CH₂OMe | O |
| C-7296 | Me | CH₂ | H | Et | CH₂OC(=O)(*i*-Pr) | Cl | CH₂OMe | O |
| C-7297 | H | CH(Me) | H | Et | CH₂OC(=O)(*i*-Pr) | Cl | CH₂OMe | O |
| C-7298 | H | CH₂CH₂ | Me | Et | CH₂OC(=O)(*i*-Pr) | Cl | CH₂OMe | O |
| C-7299 | Me | CH₂CH₂ | H | Et | CH₂OC(=O)(*i*-Pr) | Cl | CH₂OMe | O |
| C-7300 | H | single bond | Me | Et | CH₂OC(=O)(*i*-Pr) | Me | CH₂OMe | O |
| C-7301 | Me | single bond | H | Et | CH₂OC(=O)(*i*-Pr) | Me | CH₂OMe | O |
| C-7302 | Me(R) | single bond | H | Et | CH₂OC(=O)(*i*-Pr) | Me | CH₂OMe | O |
| C-7303 | Me(S) | single bond | H | Et | CH₂OC(=O)(*i*-Pr) | Me | CH₂OMe | O |
| C-7304 | H | CH₂ | Me | Et | CH₂OC(=O)(*i*-Pr) | Me | CH₂OMe | O |
| C-7305 | Me | CH₂ | H | Et | CH₂OC(=O)(*i*-Pr) | Me | CH₂OMe | O |
| C-7306 | H | CH(Me) | H | Et | CH₂OC(=O)(*i*-Pr) | Me | CH₂OMe | O |
| C-7307 | H | CH₂CH₂ | Me | Et | CH₂OC(=O)(*i*-Pr) | Me | CH₂OMe | O |
| C-7308 | Me | CH₂CH₂ | H | Et | CH₂OC(=O)(*i*-Pr) | Me | CH₂OMe | O |
| C-7309 | H | single bond | Me | Et | CH₂OC(=O)(*t*-Bu) | Cl | CH₂OMe | O |
| C-7310 | Me | single bond | H | Et | CH₂OC(=O)(*t*-Bu) | Cl | CH₂OMe | O |

**[Table 394]**

| Compound Number | Y | W | Z | R¹ | R² | R³ | R¹⁰ | A |
|---|---|---|---|---|---|---|---|---|
| C-7311 | Me(R) | single bond | H | Et | CH₂OC(=O)(*t*-Bu) | Cl | CH₂OMe | O |
| C-7312 | Me(S) | single bond | H | Et | CH₂OC(=O)(*t*-Bu) | Cl | CH₂OMe | O |
| C-7313 | H | CH₂ | Me | Et | CH₂OC(=O)(*t*-Bu) | Cl | CH₂OMe | O |
| C-7314 | Me | CH₂ | H | Et | CH₂OC(=O)(*t*-Bu) | Cl | CH₂OMe | O |
| C-7315 | H | CH(Me) | H | Et | CH₂OC(=O)(*t*-Bu) | Cl | CH₂OMe | O |
| C-7316 | H | CH₂CH₂ | Me | Et | CH₂OC(=O)(*t*-Bu) | Cl | CH₂OMe | O |
| C-7317 | Me | CH₂CH₂ | H | Et | CH₂OC(=O)(*t*-Bu) | Cl | CH₂OMe | O |
| C-7318 | H | single bond | Me | Et | CH₂OC(=O)(*t*-Bu) | Me | CH₂OMe | O |
| C-7319 | Me | single bond | H | Et | CH₂OC(=O)(*t*-Bu) | Me | CH₂OMe | O |
| C-7320 | Me(R) | single bond | H | Et | CH₂OC(=O)(*t*-Bu) | Me | CH₂OMe | O |
| C-7321 | Me(S) | single bond | H | Et | CH₂OC(=O)(*t*-Bu) | Me | CH₂OMe | O |
| C-7322 | H | CH₂ | Me | Et | CH₂OC(=O)(*t*-Bu) | Me | CH₂OMe | O |
| C-7323 | Me | CH₂ | H | Et | CH₂OC(=O)(*t*-Bu) | Me | CH₂OMe | O |
| C-7324 | H | CH(Me) | H | Et | CH₂OC(=O)(*t*-Bu) | Me | CH₂OMe | O |
| C-7325 | H | CH₂CH₂ | Me | Et | CH₂OC(=O)(*t*-Bu) | Me | CH₂OMe | O |
| C-7326 | Me | CH₂CH₂ | H | Et | CH₂OC(=O)(*t*-Bu) | Me | CH₂OMe | O |
| C-7327 | H | single bond | Me | Et | CH₂OC(=O)OEt | Cl | CH₂OMe | O |
| C-7328 | Me | single bond | H | Et | CH₂OC(=O)OEt | Cl | CH₂OMe | O |
| C-7329 | Me(R) | single bond | H | Et | CH₂OC(=O)OEt | Cl | CH₂OMe | O |
| C-7330 | Me(S) | single bond | H | Et | CH₂OC(=O)OEt | Cl | CH₂OMe | O |
| C-7331 | H | CH₂ | Me | Et | CH₂OC(=O)OEt | Cl | CH₂OMe | O |
| C-7332 | Me | CH₂ | H | Et | CH₂OC(=O)OEt | Cl | CH₂OMe | O |
| C-7333 | H | CH(Me) | H | Et | CH₂OC(=O)OEt | Cl | CH₂OMe | O |
| C-7334 | H | CH₂CH₂ | Me | Et | CH₂OC(=O)OEt | Cl | CH₂OMe | O |
| C-7335 | Me | CH₂CH₂ | H | Et | CH₂OC(=O)OEt | Cl | CH₂OMe | O |
| C-7336 | H | single bond | Me | Et | CH₂OC(=O)OEt | Me | CH₂OMe | O |
| C-7337 | Me | single bond | H | Et | CH₂OC(=O)OEt | Me | CH₂OMe | O |
| C-7338 | Me(R) | single bond | H | Et | CH₂OC(=O)OEt | Me | CH₂OMe | O |
| C-7339 | Me(S) | single bond | H | Et | CH₂OC(=O)OEt | Me | CH₂OMe | O |
| C-7340 | H | CH₂ | Me | Et | CH₂OC(=O)OEt | Me | CH₂OMe | O |
| C-7341 | Me | CH₂ | H | Et | CH₂OC(=O)OEt | Me | CH₂OMe | O |
| C-7342 | H | CH(Me) | H | Et | CH₂OC(=O)OEt | Me | CH₂OMe | O |
| C-7343 | H | CH₂CH₂ | Me | Et | CH₂OC(=O)OEt | Me | CH₂OMe | O |
| C-7344 | Me | CH₂CH₂ | H | Et | CH₂OC(=O)OEt | Me | CH₂OMe | O |
| C-7345 | H | single bond | Me | Et | CH₂OC(=O)O(*i*-Pr) | Cl | CH₂OMe | O |
| C-7346 | Me | single bond | H | Et | CH₂OC(=O)O(*i*-Pr) | Cl | CH₂OMe | O |
| C-7347 | Me(R) | single bond | H | Et | CH₂OC(=O)O(*i*-Pr) | Cl | CH₂OMe | O |
| C-7348 | Me(S) | single bond | H | Et | CH₂OC(=O)O(*i*-Pr) | Cl | CH₂OMe | O |
| C-7349 | H | CH₂ | Me | Et | CH₂OC(=O)O(*i*-Pr) | Cl | CH₂OMe | O |
| C-7350 | Me | CH₂ | H | Et | CH₂OC(=O)O(*i*-Pr) | Cl | CH₂OMe | O |
| C-7351 | H | CH(Me) | H | Et | CH₂OC(=O)O(*i*-Pr) | Cl | CH₂OMe | O |
| C-7352 | H | CH₂CH₂ | Me | Et | CH₂OC(=O)O(*i*-Pr) | Cl | CH₂OMe | O |
| C-7353 | Me | CH₂CH₂ | H | Et | CH₂OC(=O)O(*i*-Pr) | Cl | CH₂OMe | O |
| C-7354 | H | single bond | Me | Et | CH₂OC(=O)O(*i*-Pr) | Me | CH₂OMe | O |

**[Table 395]**

| Compound Number | Y | W | Z | R¹ | R² | R³ | R¹⁰ | A |
|---|---|---|---|---|---|---|---|---|
| C-7355 | Me | single bond | H | Et | CH₂OC(=O)O(*i*-Pr) | Me | CH₂OMe | O |
| C-7356 | Me(R) | single bond | H | Et | CH₂OC(=O)O(*i*-Pr) | Me | CH₂OMe | O |
| C-7357 | Me(S) | single bond | H | Et | CH₂OC(=O)O(*i*-Pr) | Me | CH₂OMe | O |
| C-7358 | H | CH₂ | Me | Et | CH₂OC(=O)O(*i*-Pr) | Me | CH₂OMe | O |
| C-7359 | Me | CH₂ | H | Et | CH₂OC(=O)O(*i*-Pr) | Me | CH₂OMe | O |
| C-7360 | H | CH(Me) | H | Et | CH₂OC(=O)O(*i*-Pr) | Me | CH₂OMe | O |
| C-7361 | H | CH₂CH₂ | Me | Et | CH₂OC(=O)O(*i*-Pr) | Me | CH₂OMe | O |
| C-7362 | Me | CH₂CH₂ | H | Et | CH₂OC(=O)O(*i*-Pr) | Me | CH₂OMe | O |
| C-7363 | H | single bond | Me | Et | CH₂N(Me)C(=O)OMe | Cl | CH₂OMe | O |
| C-7364 | Me | single bond | H | Et | CH₂N(Me)C(=O)OMe | Cl | CH₂OMe | O |
| C-7365 | Me(R) | single bond | H | Et | CH₂N(Me)C(=O)OMe | Cl | CH₂OMe | O |
| C-7366 | Me(S) | single bond | H | Et | CH₂N(Me)C(=O)OMe | Cl | CH₂OMe | O |
| C-7367 | H | CH₂ | Me | Et | CH₂N(Me)C(=O)OMe | Cl | CH₂OMe | O |
| C-7368 | Me | CH₂ | H | Et | CH₂N(Me)C(=O)OMe | Cl | CH₂OMe | O |
| C-7369 | H | CH(Me) | H | Et | CH₂N(Me)C(=O)OMe | Cl | CH₂OMe | O |
| C-7370 | H | CH₂CH₂ | Me | Et | CH₂N(Me)C(=O)OMe | Cl | CH₂OMe | O |
| C-7371 | Me | CH₂CH₂ | H | Et | CH₂N(Me)C(=O)OMe | Cl | CH₂OMe | O |
| C-7372 | H | single bond | Me | Et | CH₂N(Me)C(=O)OMe | Me | CH₂OMe | O |
| C-7373 | Me | single bond | H | Et | CH₂N(Me)C(=O)OMe | Me | CH₂OMe | O |
| C-7374 | Me(R) | single bond | H | Et | CH₂N(Me)C(=O)OMe | Me | CH₂OMe | O |
| C-7375 | Me(S) | single bond | H | Et | CH₂N(Me)C(=O)OMe | Me | CH₂OMe | O |
| C-7376 | H | CH₂ | Me | Et | CH₂N(Me)C(=O)OMe | Me | CH₂OMe | O |
| C-7377 | Me | CH₂ | H | Et | CH₂N(Me)C(=O)OMe | Me | CH₂OMe | O |
| C-7378 | H | CH(Me) | H | Et | CH₂N(Me)C(=O)OMe | Me | CH₂OMe | O |
| C-7379 | H | CH₂CH₂ | Me | Et | CH₂N(Me)C(=O)OMe | Me | CH₂OMe | O |
| C-7380 | Me | CH₂CH₂ | H | Et | CH₂N(Me)C(=O)OMe | Me | CH₂OMe | O |
| C-7381 | H | single bond | Me | Et | C(=O)OCH₂CH=CH₂ | Cl | CH₂CN | O |
| C-7382 | Me | single bond | H | Et | C(=O)OCH₂CH=CH₂ | Cl | CH₂CN | O |
| C-7383 | Me(R) | single bond | H | Et | C(=O)OCH₂CH=CH₂ | Cl | CH₂CN | O |
| C-7384 | Me(S) | single bond | H | Et | C(=O)OCH₂CH=CH₂ | Cl | CH₂CN | O |
| C-7385 | H | CH₂ | Me | Et | C(=O)OCH₂CH=CH₂ | Cl | CH₂CN | O |
| C-7386 | Me | CH₂ | H | Et | C(=O)OCH₂CH=CH₂ | Cl | CH₂CN | O |
| C-7387 | H | CH(Me) | H | Et | C(=O)OCH₂CH=CH₂ | Cl | CH₂CN | O |
| C-7388 | H | CH₂CH₂ | Me | Et | C(=O)OCH₂CH=CH₂ | Cl | CH₂CN | O |
| C-7389 | Me | CH₂CH₂ | H | Et | C(=O)OCH₂CH=CH₂ | Cl | CH₂CN | O |
| C-7390 | H | single bond | Me | Et | C(=O)OCH₂CH=CH₂ | Me | CH₂CN | O |
| C-7391 | Me | single bond | H | Et | C(=O)OCH₂CH=CH₂ | Me | CH₂CN | O |
| C-7392 | Me(R) | single bond | H | Et | C(=O)OCH₂CH=CH₂ | Me | CH₂CN | O |
| C-7393 | Me(S) | single bond | H | Et | C(=O)OCH₂CH=CH₂ | Me | CH₂CN | O |
| C-7394 | H | CH₂ | Me | Et | C(=O)OCH₂CH=CH₂ | Me | CH₂CN | O |
| C-7395 | Me | CH₂ | H | Et | C(=O)OCH₂CH=CH₂ | Me | CH₂CN | O |
| C-7396 | H | CH(Me) | H | Et | C(=O)OCH₂CH=CH₂ | Me | CH₂CN | O |
| C-7397 | H | CH₂CH₂ | Me | Et | C(=O)OCH₂CH=CH₂ | Me | CH₂CN | O |
| C-7398 | Me | CH₂CH₂ | H | Et | C(=O)OCH₂CH=CH₂ | Me | CH₂CN | O |

**[Table 396]**

| Compound Number | Y | W | Z | R¹ | R² | R³ | R¹⁰ | A |
|---|---|---|---|---|---|---|---|---|
| C-7399 | H | single bond | Me | Et | C(=O)OCH₂CCl₃ | Cl | CH₂CN | O |
| C-7400 | Me | single bond | H | Et | C(=O)OCH₂CCl₃ | Cl | CH₂CN | O |
| C-7401 | Me(R) | single bond | H | Et | C(=O)OCH₂CCl₃ | Cl | CH₂CN | O |
| C-7402 | Me(S) | single bond | H | Et | C(=O)OCH₂CCl₃ | Cl | CH₂CN | O |
| C-7403 | H | CH₂ | Me | Et | C(=O)OCH₂CCl₃ | Cl | CH₂CN | O |
| C-7404 | Me | CH₂ | H | Et | C(=O)OCH₂CCl₃ | Cl | CH₂CN | O |
| C-7405 | H | CH(Me) | H | Et | C(=O)OCH₂CCl₃ | Cl | CH₂CN | O |
| C-7406 | H | CH₂CH₂ | Me | Et | C(=O)OCH₂CCl₃ | Cl | CH₂CN | O |
| C-7407 | Me | CH₂CH₂ | H | Et | C(=O)OCH₂CCl₃ | Cl | CH₂CN | O |
| C-7408 | H | single bond | Me | Et | C(=O)OCH₂CCl₃ | Me | CH₂CN | O |
| C-7409 | Me | single bond | H | Et | C(=O)OCH₂CCl₃ | Me | CH₂CN | O |
| C-7410 | Me(R) | single bond | H | Et | C(=O)OCH₂CCl₃ | Me | CH₂CN | O |
| C-7411 | Me(S) | single bond | H | Et | C(=O)OCH₂CCl₃ | Me | CH₂CN | O |
| C-7412 | H | CH₂ | Me | Et | C(=O)OCH₂CCl₃ | Me | CH₂CN | O |
| C-7413 | Me | CH₂ | H | Et | C(=O)OCH₂CCl₃ | Me | CH₂CN | O |
| C-7414 | H | CH(Me) | H | Et | C(=O)OCH₂CCl₃ | Me | CH₂CN | O |
| C-7415 | H | CH₂CH₂ | Me | Et | C(=O)OCH₂CCl₃ | Me | CH₂CN | O |
| C-7416 | Me | CH₂CH₂ | H | Et | C(=O)OCH₂CCl₃ | Me | CH₂CN | O |
| C-7417 | H | single bond | Me | Et | C(=O)N(Me)₂ | Cl | CH₂CN | O |
| C-7418 | Me | single bond | H | Et | C(=O)N(Me)₂ | Cl | CH₂CN | O |
| C-7419 | Me(R) | single bond | H | Et | C(=O)N(Me)₂ | Cl | CH₂CN | O |
| C-7420 | Me(S) | single bond | H | Et | C(=O)N(Me)₂ | Cl | CH₂CN | O |
| C-7421 | H | CH₂ | Me | Et | C(=O)N(Me)₂ | Cl | CH₂CN | O |
| C-7422 | Me | CH₂ | H | Et | C(=O)N(Me)₂ | Cl | CH₂CN | O |
| C-7423 | H | CH(Me) | H | Et | C(=O)N(Me)₂ | Cl | CH₂CN | O |
| C-7424 | H | CH₂CH₂ | Me | Et | C(=O)N(Me)₂ | Cl | CH₂CN | O |
| C-7425 | Me | CH₂CH₂ | H | Et | C(=O)N(Me)₂ | Cl | CH₂CN | O |
| C-7426 | H | single bond | Me | Et | C(=O)N(Me)₂ | Me | CH₂CN | O |
| C-7427 | Me | single bond | H | Et | C(=O)N(Me)₂ | Me | CH₂CN | O |
| C-7428 | Me(R) | single bond | H | Et | C(=O)N(Me)₂ | Me | CH₂CN | O |
| C-7429 | Me(S) | single bond | H | Et | C(=O)N(Me)₂ | Me | CH₂CN | O |
| C-7430 | H | CH₂ | Me | Et | C(=O)N(Me)₂ | Me | CH₂CN | O |
| C-7431 | Me | CH₂ | H | Et | C(=O)N(Me)₂ | Me | CH₂CN | O |
| C-7432 | H | CH(Me) | H | Et | C(=O)N(Me)₂ | Me | CH₂CN | O |
| C-7433 | H | CH₂CH₂ | Me | Et | C(=O)N(Me)₂ | Me | CH₂CN | O |
| C-7434 | Me | CH₂CH₂ | H | Et | C(=O)N(Me)₂ | Me | CH₂CN | O |
| C-7435 | H | single bond | Me | Et | CH₂OC(=O)Me | Cl | CH₂CN | O |
| C-7436 | Me | single bond | H | Et | CH₂OC(=O)Me | Cl | CH₂CN | O |
| C-7437 | Me(R) | single bond | H | Et | CH₂OC(=O)Me | Cl | CH₂CN | O |
| C-7438 | Me(S) | single bond | H | Et | CH₂OC(=O)Me | Cl | CH₂CN | O |
| C-7439 | H | CH₂ | Me | Et | CH₂OC(=O)Me | Cl | CH₂CN | O |
| C-7440 | Me | CH₂ | H | Et | CH₂OC(=O)Me | Cl | CH₂CN | O |
| C-7441 | H | CH(Me) | H | Et | CH₂OC(=O)Me | Cl | CH₂CN | O |
| C-7442 | H | CH₂CH₂ | Me | Et | CH₂OC(=O)Me | Cl | CH₂CN | O |

**[Table 397]**

| Compound Number | Y | W | Z | R¹ | R² | R³ | R¹⁰ | A |
|---|---|---|---|---|---|---|---|---|
| C-7443 | Me | CH₂CH₂ | H | Et | CH₂OC(=O)Me | Cl | CH₂CN | O |
| C-7444 | H | single bond | Me | Et | CH₂OC(=O)Me | Me | CH₂CN | O |
| C-7445 | Me | single bond | H | Et | CH₂OC(=O)Me | Me | CH₂CN | O |
| C-7446 | Me(R) | single bond | H | Et | CH₂OC(=O)Me | Me | CH₂CN | O |
| C-7447 | Me(S) | single bond | H | Et | CH₂OC(=O)Me | Me | CH₂CN | O |
| C-7448 | H | CH₂ | Me | Et | CH₂OC(=O)Me | Me | CH₂CN | O |
| C-7449 | Me | CH₂ | H | Et | CH₂OC(=O)Me | Me | CH₂CN | O |
| C-7450 | H | CH(Me) | H | Et | CH₂OC(=O)Me | Me | CH₂CN | O |
| C-7451 | H | CH₂CH₂ | Me | Et | CH₂OC(=O)Me | Me | CH₂CN | O |
| C-7452 | Me | CH₂CH₂ | H | Et | CH₂OC(=O)Me | Me | CH₂CN | O |
| C-7453 | H | single bond | Me | Et | CH₂OC(=O)(*i*-Pr) | Cl | CH₂CN | O |
| C-7454 | Me | single bond | H | Et | CH₂OC(=O)(*i*-Pr) | Cl | CH₂CN | O |
| C-7455 | Me(R) | single bond | H | Et | CH₂OC(=O)(*i*-Pr) | Cl | CH₂CN | O |
| C-7456 | Me(S) | single bond | H | Et | CH₂OC(=O)(*i*-Pr) | Cl | CH₂CN | O |
| C-7457 | H | CH₂ | Me | Et | CH₂OC(=O)(*i*-Pr) | Cl | CH₂CN | O |
| C-7458 | Me | CH₂ | H | Et | CH₂OC(=O)(*i*-Pr) | Cl | CH₂CN | O |
| C-7459 | H | CH(Me) | H | Et | CH₂OC(=O)(*i*-Pr) | Cl | CH₂CN | O |
| C-7460 | H | CH₂CH₂ | Me | Et | CH₂OC(=O)(*i*-Pr) | Cl | CH₂CN | O |
| C-7461 | Me | CH₂CH₂ | H | Et | CH₂OC(=O)(*i*-Pr) | Cl | CH₂CN | O |
| C-7462 | H | single bond | Me | Et | CH₂OC(=O)(*i*-Pr) | Me | CH₂CN | O |
| C-7463 | Me | single bond | H | Et | CH₂OC(=O)(*i*-Pr) | Me | CH₂CN | O |
| C-7464 | Me(R) | single bond | H | Et | CH₂OC(=O)(*i*-Pr) | Me | CH₂CN | O |
| C-7465 | Me(S) | single bond | H | Et | CH₂OC(=O)(*i*-Pr) | Me | CH₂CN | O |
| C-7466 | H | CH₂ | Me | Et | CH₂OC(=O)(*i*-Pr) | Me | CH₂CN | O |
| C-7467 | Me | CH₂ | H | Et | CH₂OC(=O)(*i*-Pr) | Me | CH₂CN | O |
| C-7468 | H | CH(Me) | H | Et | CH₂OC(=O)(*i*-Pr) | Me | CH₂CN | O |
| C-7469 | H | CH₂CH₂ | Me | Et | CH₂OC(=O)(*i*-Pr) | Me | CH₂CN | O |
| C-7470 | Me | CH₂CH₂ | H | Et | CH₂OC(=O)(*i*-Pr) | Me | CH₂CN | O |
| C-7471 | H | single bond | Me | Et | CH₂OC(=O)(*t*-Bu) | Cl | CH₂CN | O |
| C-7472 | Me | single bond | H | Et | CH₂OC(=O)(*t*-Bu) | Cl | CH₂CN | O |
| C-7473 | Me(R) | single bond | H | Et | CH₂OC(=O)(*t*-Bu) | Cl | CH₂CN | O |
| C-7474 | Me(S) | single bond | H | Et | CH₂OC(=O)(*t*-Bu) | Cl | CH₂CN | O |
| C-7475 | H | CH₂ | Me | Et | CH₂OC(=O)(*t*-Bu) | Cl | CH₂CN | O |
| C-7476 | Me | CH₂ | H | Et | CH₂OC(=O)(*t*-Bu) | Cl | CH₂CN | O |
| C-7477 | H | CH(Me) | H | Et | CH₂OC(=O)(*t*-Bu) | Cl | CH₂CN | O |
| C-7478 | H | CH₂CH₂ | Me | Et | CH₂OC(=O)(*t*-Bu) | Cl | CH₂CN | O |
| C-7479 | Me | CH₂CH₂ | H | Et | CH₂OC(=O)(*t*-Bu) | Cl | CH₂CN | O |
| C-7480 | H | single bond | Me | Et | CH₂OC(=O)(*t*-Bu) | Me | CH₂CN | O |
| C-7481 | Me | single bond | H | Et | CH₂OC(=O)(*t*-Bu) | Me | CH₂CN | O |
| C-7482 | Me(R) | single bond | H | Et | CH₂OC(=O)(*t*-Bu) | Me | CH₂CN | O |
| C-7483 | Me(S) | single bond | H | Et | CH₂OC(=O)(*t*-Bu) | Me | CH₂CN | O |
| C-7484 | H | CH₂ | Me | Et | CH₂OC(=O)(*t*-Bu) | Me | CH₂CN | O |
| C-7485 | Me | CH₂ | H | Et | CH₂OC(=O)(*t*-Bu) | Me | CH₂CN | O |
| C-7486 | H | CH(Me) | H | Et | CH₂OC(=O)(*t*-Bu) | Me | CH₂CN | O |

**[Table 398]**

| Compound Number | Y | W | Z | R¹ | R² | R³ | R¹⁰ | A |
|---|---|---|---|---|---|---|---|---|
| C-7487 | H | CH₂CH₂ | Me | Et | CH₂OC(=O)(*t*-Bu) | Me | CH₂CN | O |
| C-7488 | Me | CH₂CH₂ | H | Et | CH₂OC(=O)(*t*-Bu) | Me | CH₂CN | O |
| C-7489 | H | single bond | Me | Et | CH₂OC(=O)OEt | Cl | CH₂CN | O |
| C-7490 | Me | single bond | H | Et | CH₂OC(=O)OEt | Cl | CH₂CN | O |
| C-7491 | Me(R) | single bond | H | Et | CH₂OC(=O)OEt | Cl | CH₂CN | O |
| C-7492 | Me(S) | single bond | H | Et | CH₂OC(=O)OEt | Cl | CH₂CN | O |
| C-7493 | H | CH₂ | Me | Et | CH₂OC(=O)OEt | Cl | CH₂CN | O |
| C-7494 | Me | CH₂ | H | Et | CH₂OC(=O)OEt | Cl | CH₂CN | O |
| C-7495 | H | CH(Me) | H | Et | CH₂OC(=O)OEt | Cl | CH₂CN | O |
| C-7496 | H | CH₂CH₂ | Me | Et | CH₂OC(=O)OEt | Cl | CH₂CN | O |
| C-7497 | Me | CH₂CH₂ | H | Et | CH₂OC(=O)OEt | Cl | CH₂CN | O |
| C-7498 | H | single bond | Me | Et | CH₂OC(=O)OEt | Me | CH₂CN | O |
| C-7499 | Me | single bond | H | Et | CH₂OC(=O)OEt | Me | CH₂CN | O |
| C-7500 | Me(R) | single bond | H | Et | CH₂OC(=O)OEt | Me | CH₂CN | O |
| C-7501 | Me(S) | single bond | H | Et | CH₂OC(=O)OEt | Me | CH₂CN | O |
| C-7502 | H | CH₂ | Me | Et | CH₂OC(=O)OEt | Me | CH₂CN | O |
| C-7503 | Me | CH₂ | H | Et | CH₂OC(=O)OEt | Me | CH₂CN | O |
| C-7504 | H | CH(Me) | H | Et | CH₂OC(=O)OEt | Me | CH₂CN | O |
| C-7505 | H | CH₂CH₂ | Me | Et | CH₂OC(=O)OEt | Me | CH₂CN | O |
| C-7506 | Me | CH₂CH₂ | H | Et | CH₂OC(=O)OEt | Me | CH₂CN | O |
| C-7507 | H | single bond | Me | Et | CH₂OC(=O)O(*i*-Pr) | Cl | CH₂CN | O |
| C-7508 | Me | single bond | H | Et | CH₂OC(=O)O(*i*-Pr) | Cl | CH₂CN | O |
| C-7509 | Me(R) | single bond | H | Et | CH₂OC(=O)O(*i*-Pr) | Cl | CH₂CN | O |
| C-7510 | Me(S) | single bond | H | Et | CH₂OC(=O)O(*i*-Pr) | Cl | CH₂CN | O |
| C-7511 | H | CH₂ | Me | Et | CH₂OC(=O)O(*i*-Pr) | Cl | CH₂CN | O |
| C-7512 | Me | CH₂ | H | Et | CH₂OC(=O)O(*i*-Pr) | Cl | CH₂CN | O |
| C-7513 | H | CH(Me) | H | Et | CH₂OC(=O)O(*i*-Pr) | Cl | CH₂CN | O |
| C-7514 | H | CH₂CH₂ | Me | Et | CH₂OC(=O)O(*i*-Pr) | Cl | CH₂CN | O |
| C-7515 | Me | CH₂CH₂ | H | Et | CH₂OC(=O)O(*i*-Pr) | Cl | CH₂CN | O |
| C-7516 | H | single bond | Me | Et | CH₂OC(=O)O(*i*-Pr) | Me | CH₂CN | O |
| C-7517 | Me | single bond | H | Et | CH₂OC(=O)O(*i*-Pr) | Me | CH₂CN | O |
| C-7518 | Me(R) | single bond | H | Et | CH₂OC(=O)O(*i*-Pr) | Me | CH₂CN | O |
| C-7519 | Me(S) | single bond | H | Et | CH₂OC(=O)O(*i*-Pr) | Me | CH₂CN | O |
| C-7520 | H | CH₂ | Me | Et | CH₂OC(=O)O(*i*-Pr) | Me | CH₂CN | O |
| C-7521 | Me | CH₂ | H | Et | CH₂OC(=O)O(*i*-Pr) | Me | CH₂CN | O |
| C-7522 | H | CH(Me) | H | Et | CH₂OC(=O)O(*i*-Pr) | Me | CH₂CN | O |
| C-7523 | H | CH₂CH₂ | Me | Et | CH₂OC(=O)O(*i*-Pr) | Me | CH₂CN | O |
| C-7524 | Me | CH₂CH₂ | H | Et | CH₂OC(=O)O(*i*-Pr) | Me | CH₂CN | O |
| C-7525 | H | single bond | Me | Et | CH₂N(Me)C(=O)OMe | Cl | CH₂CN | O |
| C-7526 | Me | single bond | H | Et | CH₂N(Me)C(=O)OMe | Cl | CH₂CN | O |
| C-7527 | Me(R) | single bond | H | Et | CH₂N(Me)C(=O)OMe | Cl | CH₂CN | O |
| C-7528 | Me(S) | single bond | H | Et | CH₂N(Me)C(=O)OMe | Cl | CH₂CN | O |
| C-7529 | H | CH₂ | Me | Et | CH₂N(Me)C(=O)OMe | Cl | CH₂CN | O |
| C-7530 | Me | CH₂ | H | Et | CH₂N(Me)C(=O)OMe | Cl | CH₂CN | O |

**[Table 399]**

| Compound Number | Y | W | Z | R¹ | R² | R³ | R¹⁰ | A |
|---|---|---|---|---|---|---|---|---|
| C-7531 | H | CH(Me) | H | Et | CH₂N(Me)C(=O)OMe | Cl | CH₂CN | O |
| C-7532 | H | CH₂CH₂ | Me | Et | CH₂N(Me)C(=O)OMe | Cl | CH₂CN | O |
| C-7533 | Me | CH₂CH₂ | H | Et | CH₂N(Me)C(=O)OMe | Cl | CH₂CN | O |
| C-7534 | H | single bond | Me | Et | CH₂N(Me)C(=O)OMe | Me | CH₂CN | O |
| C-7535 | Me | single bond | H | Et | CH₂N(Me)C(=O)OMe | Me | CH₂CN | O |
| C-7536 | Me(R) | single bond | H | Et | CH₂N(Me)C(=O)OMe | Me | CH₂CN | O |
| C-7537 | Me(S) | single bond | H | Et | CH₂N(Me)C(=O)OMe | Me | CH₂CN | O |
| C-7538 | H | CH₂ | Me | Et | CH₂N(Me)C(=O)OMe | Me | CH₂CN | O |
| C-7539 | Me | CH₂ | H | Et | CH₂N(Me)C(=O)OMe | Me | CH₂CN | O |
| C-7540 | H | CH(Me) | H | Et | CH₂N(Me)C(=O)OMe | Me | CH₂CN | O |
| C-7541 | H | CH₂CH₂ | Me | Et | CH₂N(Me)C(=O)OMe | Me | CH₂CN | O |
| C-7542 | Me | CH₂CH₂ | H | Et | CH₂N(Me)C(=O)OMe | Me | CH₂CN | O |
| C-7543 | H | single bond | Me | Et | C(=O)OCH₂CH=CH₂ | Cl | C(=O)Me | O |
| C-7544 | Me | single bond | H | Et | C(=O)OCH₂CH=CH₂ | Cl | C(=O)Me | O |
| C-7545 | Me(R) | single bond | H | Et | C(=O)OCH₂CH=CH₂ | Cl | C(=O)Me | O |
| C-7546 | Me(S) | single bond | H | Et | C(=O)OCH₂CH=CH₂ | Cl | C(=O)Me | O |
| C-7547 | H | CH₂ | Me | Et | C(=O)OCH₂CH=CH₂ | Cl | C(=O)Me | O |
| C-7548 | Me | CH₂ | H | Et | C(=O)OCH₂CH=CH₂ | Cl | C(=O)Me | O |
| C-7549 | H | CH(Me) | H | Et | C(=O)OCH₂CH=CH₂ | Cl | C(=O)Me | O |
| C-7550 | H | CH₂CH₂ | Me | Et | C(=O)OCH₂CH=CH₂ | Cl | C(=O)Me | O |
| C-7551 | Me | CH₂CH₂ | H | Et | C(=O)OCH₂CH=CH₂ | Cl | C(=O)Me | O |
| C-7552 | H | single bond | Me | Et | C(=O)OCH₂CH=CH₂ | Me | C(=O)Me | O |
| C-7553 | Me | single bond | H | Et | C(=O)OCH₂CH=CH₂ | Me | C(=O)Me | O |
| C-7554 | Me(R) | single bond | H | Et | C(=O)OCH₂CH=CH₂ | Me | C(=O)Me | O |
| C-7555 | Me(S) | single bond | H | Et | C(=O)OCH₂CH=CH₂ | Me | C(=O)Me | O |
| C-7556 | H | CH₂ | Me | Et | C(=O)OCH₂CH=CH₂ | Me | C(=O)Me | O |
| C-7557 | Me | CH₂ | H | Et | C(=O)OCH₂CH=CH₂ | Me | C(=O)Me | O |
| C-7558 | H | CH(Me) | H | Et | C(=O)OCH₂CH=CH₂ | Me | C(=O)Me | O |
| C-7559 | H | CH₂CH₂ | Me | Et | C(=O)OCH₂CH=CH₂ | Me | C(=O)Me | O |
| C-7560 | Me | CH₂CH₂ | H | Et | C(=O)OCH₂CH=CH₂ | Me | C(=O)Me | O |
| C-7561 | H | single bond | Me | Et | C(=O)OCH₂CCl₃ | Cl | C(=O)Me | O |
| C-7562 | Me | single bond | H | Et | C(=O)OCH₂CCl₃ | Cl | C(=O)Me | O |
| C-7563 | Me(R) | single bond | H | Et | C(=O)OCH₂CCl₃ | Cl | C(=O)Me | O |
| C-7564 | Me(S) | single bond | H | Et | C(=O)OCH₂CCl₃ | Cl | C(=O)Me | O |
| C-7565 | H | CH₂ | Me | Et | C(=O)OCH₂CCl₃ | Cl | C(=O)Me | O |
| C-7566 | Me | CH₂ | H | Et | C(=O)OCH₂CCl₃ | Cl | C(=O)Me | O |
| C-7567 | H | CH(Me) | H | Et | C(=O)OCH₂CCl₃ | Cl | C(=O)Me | O |
| C-7568 | H | CH₂CH₂ | Me | Et | C(=O)OCH₂CCl₃ | Cl | C(=O)Me | O |
| C-7569 | Me | CH₂CH₂ | H | Et | C(=O)OCH₂CCl₃ | Cl | C(=O)Me | O |
| C-7570 | H | single bond | Me | Et | C(=O)OCH₂CCl₃ | Me | C(=O)Me | O |
| C-7571 | Me | single bond | H | Et | C(=O)OCH₂CCl₃ | Me | C(=O)Me | O |
| C-7572 | Me(R) | single bond | H | Et | C(=O)OCH₂CCl₃ | Me | C(=O)Me | O |
| C-7573 | Me(S) | single bond | H | Et | C(=O)OCH₂CCl₃ | Me | C(=O)Me | O |
| C-7574 | H | CH₂ | Me | Et | C(=O)OCH₂CCl₃ | Me | C(=O)Me | O |

**[Table 400]**

| Compound Number | Y | W | Z | R¹ | R² | R³ | R¹⁰ | A |
|---|---|---|---|---|---|---|---|---|
| C-7575 | Me | CH₂ | H | Et | C(=O)OCH₂CCl₃ | Me | C(=O)Me | O |
| C-7576 | H | CH(Me) | H | Et | C(=O)OCH₂CCl₃ | Me | C(=O)Me | O |
| C-7577 | H | CH₂CH₂ | Me | Et | C(=O)OCH₂CCl₃ | Me | C(=O)Me | O |
| C-7578 | Me | CH₂CH₂ | H | Et | C(=O)OCH₂CCl₃ | Me | C(=O)Me | O |
| C-7579 | H | single bond | Me | Et | C(=O)N(Me)₂ | Cl | C(=O)Me | O |
| C-7580 | Me | single bond | H | Et | C(=O)N(Me)₂ | Cl | C(=O)Me | O |
| C-7581 | Me(R) | single bond | H | Et | C(=O)N(Me)₂ | Cl | C(=O)Me | O |
| C-7582 | Me(S) | single bond | H | Et | C(=O)N(Me)₂ | Cl | C(=O)Me | O |
| C-7583 | H | CH₂ | Me | Et | C(=O)N(Me)₂ | Cl | C(=O)Me | O |
| C-7584 | Me | CH₂ | H | Et | C(=O)N(Me)₂ | Cl | C(=O)Me | O |
| C-7585 | H | CH(Me) | H | Et | C(=O)N(Me)₂ | Cl | C(=O)Me | O |
| C-7586 | H | CH₂CH₂ | Me | Et | C(=O)N(Me)₂ | Cl | C(=O)Me | O |
| C-7587 | Me | CH₂CH₂ | H | Et | C(=O)N(Me)₂ | Cl | C(=O)Me | O |
| C-7588 | H | single bond | Me | Et | C(=O)N(Me)₂ | Me | C(=O)Me | O |
| C-7589 | Me | single bond | H | Et | C(=O)N(Me)₂ | Me | C(=O)Me | O |
| C-7590 | Me(R) | single bond | H | Et | C(=O)N(Me)₂ | Me | C(=O)Me | O |
| C-7591 | Me(S) | single bond | H | Et | C(=O)N(Me)₂ | Me | C(=O)Me | O |
| C-7592 | H | CH₂ | Me | Et | C(=O)N(Me)₂ | Me | C(=O)Me | O |
| C-7593 | Me | CH₂ | H | Et | C(=O)N(Me)₂ | Me | C(=O)Me | O |
| C-7594 | H | CH(Me) | H | Et | C(=O)N(Me)₂ | Me | C(=O)Me | O |
| C-7595 | H | CH₂CH₂ | Me | Et | C(=O)N(Me)₂ | Me | C(=O)Me | O |
| C-7596 | Me | CH₂CH₂ | H | Et | C(=O)N(Me)₂ | Me | C(=O)Me | O |
| C-7597 | H | single bond | Me | Et | CH₂OC(=O)Me | Cl | C(=O)Me | O |
| C-7598 | Me | single bond | H | Et | CH₂OC(=O)Me | Cl | C(=O)Me | O |
| C-7599 | Me(R) | single bond | H | Et | CH₂OC(=O)Me | Cl | C(=O)Me | O |
| C-7600 | Me(S) | single bond | H | Et | CH₂OC(=O)Me | Cl | C(=O)Me | O |
| C-7601 | H | CH₂ | Me | Et | CH₂OC(=O)Me | Cl | C(=O)Me | O |
| C-7602 | Me | CH₂ | H | Et | CH₂OC(=O)Me | Cl | C(=O)Me | O |
| C-7603 | H | CH(Me) | H | Et | CH₂OC(=O)Me | Cl | C(=O)Me | O |
| C-7604 | H | CH₂CH₂ | Me | Et | CH₂OC(=O)Me | Cl | C(=O)Me | O |
| C-7605 | Me | CH₂CH₂ | H | Et | CH₂OC(=O)Me | Cl | C(=O)Me | O |
| C-7606 | H | single bond | Me | Et | CH₂OC(=O)Me | Me | C(=O)Me | O |
| C-7607 | Me | single bond | H | Et | CH₂OC(=O)Me | Me | C(=O)Me | O |
| C-7608 | Me(R) | single bond | H | Et | CH₂OC(=O)Me | Me | C(=O)Me | O |
| C-7609 | Me(S) | single bond | H | Et | CH₂OC(=O)Me | Me | C(=O)Me | O |
| C-7610 | H | CH₂ | Me | Et | CH₂OC(=O)Me | Me | C(=O)Me | O |
| C-7611 | Me | CH₂ | H | Et | CH₂OC(=O)Me | Me | C(=O)Me | O |
| C-7612 | H | CH(Me) | H | Et | CH₂OC(=O)Me | Me | C(=O)Me | O |
| C-7613 | H | CH₂CH₂ | Me | Et | CH₂OC(=O)Me | Me | C(=O)Me | O |
| C-7614 | Me | CH₂CH₂ | H | Et | CH₂OC(=O)Me | Me | C(=O)Me | O |
| C-7615 | H | single bond | Me | Et | CH₂OC(=O)(*i*-Pr) | Cl | C(=O)Me | O |
| C-7616 | Me | single bond | H | Et | CH₂OC(=O)(*i*-Pr) | Cl | C(=O)Me | O |
| C-7617 | Me(R) | single bond | H | Et | CH₂OC(=O)(*i*-Pr) | Cl | C(=O)Me | O |
| C-7618 | Me(S) | single bond | H | Et | CH₂OC(=O)(*i*-Pr) | Cl | C(=O)Me | O |

**[Table 401]**

| Compound Number | Y | W | Z | R¹ | R² | R³ | R¹⁰ | A |
|---|---|---|---|---|---|---|---|---|
| C-7619 | H | CH₂ | Me | Et | CH₂OC(=O)(*i*-Pr) | Cl | C(=O)Me | O |
| C-7620 | Me | CH₂ | H | Et | CH₂OC(=O)(*i*-Pr) | Cl | C(=O)Me | O |
| C-7621 | H | CH(Me) | H | Et | CH₂OC(=O)(*i*-Pr) | Cl | C(=O)Me | O |
| C-7622 | H | CH₂CH₂ | Me | Et | CH₂OC(=O)(*i*-Pr) | Cl | C(=O)Me | O |
| C-7623 | Me | CH₂CH₂ | H | Et | CH₂OC(=O)(*i*-Pr) | Cl | C(=O)Me | O |
| C-7624 | H | single bond | Me | Et | CH₂OC(=O)(*i*-Pr) | Me | C(=O)Me | O |
| C-7625 | Me | single bond | H | Et | CH₂OC(=O)(*i*-Pr) | Me | C(=O)Me | O |
| C-7626 | Me(R) | single bond | H | Et | CH₂OC(=O)(*i*-Pr) | Me | C(=O)Me | O |
| C-7627 | Me(S) | single bond | H | Et | CH₂OC(=O)(*i*-Pr) | Me | C(=O)Me | O |
| C-7628 | H | CH₂ | Me | Et | CH₂OC(=O)(*i*-Pr) | Me | C(=O)Me | O |
| C-7629 | Me | CH₂ | H | Et | CH₂OC(=O)(*i*-Pr) | Me | C(=O)Me | O |
| C-7630 | H | CH(Me) | H | Et | CH₂OC(=O)(*i*-Pr) | Me | C(=O)Me | O |
| C-7631 | H | CH₂CH₂ | Me | Et | CH₂OC(=O)(*i*-Pr) | Me | C(=O)Me | O |
| C-7632 | Me | CH₂CH₂ | H | Et | CH₂OC(=O)(*i*-Pr) | Me | C(=O)Me | O |
| C-7633 | H | single bond | Me | Et | CH₂OC(=O)(*t*-Bu) | Cl | C(=O)Me | O |
| C-7634 | Me | single bond | H | Et | CH₂OC(=O)(*t*-Bu) | Cl | C(=O)Me | O |
| C-7635 | Me(R) | single bond | H | Et | CH₂OC(=O)(*t*-Bu) | Cl | C(=O)Me | O |
| C-7636 | Me(S) | single bond | H | Et | CH₂OC(=O)(*t*-Bu) | Cl | C(=O)Me | O |
| C-7637 | H | CH₂ | Me | Et | CH₂OC(=O)(*t*-Bu) | Cl | C(=O)Me | O |
| C-7638 | Me | CH₂ | H | Et | CH₂OC(=O)(*t*-Bu) | Cl | C(=O)Me | O |
| C-7639 | H | CH(Me) | H | Et | CH₂OC(=O)(*t*-Bu) | Cl | C(=O)Me | O |
| C-7640 | H | CH₂CH₂ | Me | Et | CH₂OC(=O)(*t*-Bu) | Cl | C(=O)Me | O |
| C-7641 | Me | CH₂CH₂ | H | Et | CH₂OC(=O)(*t*-Bu) | Cl | C(=O)Me | O |
| C-7642 | H | single bond | Me | Et | CH₂OC(=O)(*t*-Bu) | Me | C(=O)Me | O |
| C-7643 | Me | single bond | H | Et | CH₂OC(=O)(*t*-Bu) | Me | C(=O)Me | O |
| C-7644 | Me(R) | single bond | H | Et | CH₂OC(=O)(*t*-Bu) | Me | C(=O)Me | O |
| C-7645 | Me(S) | single bond | H | Et | CH₂OC(=O)(*t*-Bu) | Me | C(=O)Me | O |
| C-7646 | H | CH₂ | Me | Et | CH₂OC(=O)(*t*-Bu) | Me | C(=O)Me | O |
| C-7647 | Me | CH₂ | H | Et | CH₂OC(=O)(*t*-Bu) | Me | C(=O)Me | O |
| C-7648 | H | CH(Me) | H | Et | CH₂OC(=O)(*t*-Bu) | Me | C(=O)Me | O |
| C-7649 | H | CH₂CH₂ | Me | Et | CH₂OC(=O)(*t*-Bu) | Me | C(=O)Me | O |
| C-7650 | Me | CH₂CH₂ | H | Et | CH₂OC(=O)(*t*-Bu) | Me | C(=O)Me | O |
| C-7651 | H | single bond | Me | Et | CH₂OC(=O)OEt | Cl | C(=O)Me | O |
| C-7652 | Me | single bond | H | Et | CH₂OC(=O)OEt | Cl | C(=O)Me | O |
| C-7653 | Me(R) | single bond | H | Et | CH₂OC(=O)OEt | Cl | C(=O)Me | O |
| C-7654 | Me(S) | single bond | H | Et | CH₂OC(=O)OEt | Cl | C(=O)Me | O |
| C-7655 | H | CH₂ | Me | Et | CH₂OC(=O)OEt | Cl | C(=O)Me | O |
| C-7656 | Me | CH₂ | H | Et | CH₂OC(=O)OEt | Cl | C(=O)Me | O |
| C-7657 | H | CH(Me) | H | Et | CH₂OC(=O)OEt | Cl | C(=O)Me | O |
| C-7658 | H | CH₂CH₂ | Me | Et | CH₂OC(=O)OEt | Cl | C(=O)Me | O |
| C-7659 | Me | CH₂CH₂ | H | Et | CH₂OC(=O)OEt | Cl | C(=O)Me | O |
| C-7660 | H | single bond | Me | Et | CH₂OC(=O)OEt | Me | C(=O)Me | O |
| C-7661 | Me | single bond | H | Et | CH₂OC(=O)OEt | Me | C(=O)Me | O |
| C-7662 | Me(R) | single bond | H | Et | CH₂OC(=O)OEt | Me | C(=O)Me | O |

**[Table 402]**

| Compound Number | Y | W | Z | R¹ | R² | R³ | R¹⁰ | A |
|---|---|---|---|---|---|---|---|---|
| C-7663 | Me(S) | single bond | H | Et | CH₂OC(=O)OEt | Me | C(=O)Me | O |
| C-7664 | H | CH₂ | Me | Et | CH₂OC(=O)OEt | Me | C(=O)Me | O |
| C-7665 | Me | CH₂ | H | Et | CH₂OC(=O)OEt | Me | C(=O)Me | O |
| C-7666 | H | CH(Me) | H | Et | CH₂OC(=O)OEt | Me | C(=O)Me | O |
| C-7667 | H | CH₂CH₂ | Me | Et | CH₂OC(=O)OEt | Me | C(=O)Me | O |
| C-7668 | Me | CH₂CH₂ | H | Et | CH₂OC(=O)OEt | Me | C(=O)Me | O |
| C-7669 | H | single bond | Me | Et | CH₂OC(=O)O(*i*-Pr) | Cl | C(=O)Me | O |
| C-7670 | Me | single bond | H | Et | CH₂OC(=O)O(*i*-Pr) | Cl | C(=O)Me | O |
| C-7671 | Me(R) | single bond | H | Et | CH₂OC(=O)O(*i*-Pr) | Cl | C(=O)Me | O |
| C-7672 | Me(S) | single bond | H | Et | CH₂OC(=O)O(*i*-Pr) | Cl | C(=O)Me | O |
| C-7673 | H | CH₂ | Me | Et | CH₂OC(=O)O(*i*-Pr) | Cl | C(=O)Me | O |
| C-7674 | Me | CH₂ | H | Et | CH₂OC(=O)O(*i*-Pr) | Cl | C(=O)Me | O |
| C-7675 | H | CH(Me) | H | Et | CH₂OC(=O)O(*i*-Pr) | Cl | C(=O)Me | O |
| C-7676 | H | CH₂CH₂ | Me | Et | CH₂OC(=O)O(*i*-Pr) | Cl | C(=O)Me | O |
| C-7677 | Me | CH₂CH₂ | H | Et | CH₂OC(=O)O(*i*-Pr) | Cl | C(=O)Me | O |
| C-7678 | H | single bond | Me | Et | CH₂OC(=O)O(*i*-Pr) | Me | C(=O)Me | O |
| C-7679 | Me | single bond | H | Et | CH₂OC(=O)O(*i*-Pr) | Me | C(=O)Me | O |
| C-7680 | Me(R) | single bond | H | Et | CH₂OC(=O)O(*i*-Pr) | Me | C(=O)Me | O |
| C-7681 | Me(S) | single bond | H | Et | CH₂OC(=O)O(*i*-Pr) | Me | C(=O)Me | O |
| C-7682 | H | CH₂ | Me | Et | CH₂OC(=O)O(*i*-Pr) | Me | C(=O)Me | O |
| C-7683 | Me | CH₂ | H | Et | CH₂OC(=O)O(*i*-Pr) | Me | C(=O)Me | O |
| C-7684 | H | CH(Me) | H | Et | CH₂OC(=O)O(*i*-Pr) | Me | C(=O)Me | O |
| C-7685 | H | CH₂CH₂ | Me | Et | CH₂OC(=O)O(*i*-Pr) | Me | C(=O)Me | O |
| C-7686 | Me | CH₂CH₂ | H | Et | CH₂OC(=O)O(*i*-Pr) | Me | C(=O)Me | O |
| C-7687 | H | single bond | Me | Et | CH₂N(Me)C(=O)OMe | Cl | C(=O)Me | O |
| C-7688 | Me | single bond | H | Et | CH₂N(Me)C(=O)OMe | Cl | C(=O)Me | O |
| C-7689 | Me(R) | single bond | H | Et | CH₂N(Me)C(=O)OMe | Cl | C(=O)Me | O |
| C-7690 | Me(S) | single bond | H | Et | CH₂N(Me)C(=O)OMe | Cl | C(=O)Me | O |
| C-7691 | H | CH₂ | Me | Et | CH₂N(Me)C(=O)OMe | Cl | C(=O)Me | O |
| C-7692 | Me | CH₂ | H | Et | CH₂N(Me)C(=O)OMe | Cl | C(=O)Me | O |
| C-7693 | H | CH(Me) | H | Et | CH₂N(Me)C(=O)OMe | Cl | C(=O)Me | O |
| C-7694 | H | CH₂CH₂ | Me | Et | CH₂N(Me)C(=O)OMe | Cl | C(=O)Me | O |
| C-7695 | Me | CH₂CH₂ | H | Et | CH₂N(Me)C(=O)OMe | Cl | C(=O)Me | O |
| C-7696 | H | single bond | Me | Et | CH₂N(Me)C(=O)OMe | Me | C(=O)Me | O |
| C-7697 | Me | single bond | H | Et | CH₂N(Me)C(=O)OMe | Me | C(=O)Me | O |
| C-7698 | Me(R) | single bond | H | Et | CH₂N(Me)C(=O)OMe | Me | C(=O)Me | O |
| C-7699 | Me(S) | single bond | H | Et | CH₂N(Me)C(=O)OMe | Me | C(=O)Me | O |
| C-7700 | H | CH₂ | Me | Et | CH₂N(Me)C(=O)OMe | Me | C(=O)Me | O |
| C-7701 | Me | CH₂ | H | Et | CH₂N(Me)C(=O)OMe | Me | C(=O)Me | O |
| C-7702 | H | CH(Me) | H | Et | CH₂N(Me)C(=O)OMe | Me | C(=O)Me | O |
| C-7703 | H | CH₂CH₂ | Me | Et | CH₂N(Me)C(=O)OMe | Me | C(=O)Me | O |
| C-7704 | Me | CH₂CH₂ | H | Et | CH₂N(Me)C(=O)OMe | Me | C(=O)Me | O |
| C-7705 | H | single bond | Me | *c*-Pr | C(=O)OCH₂CH=CH₂ | Cl | H | O |
| C-7706 | Me | single bond | H | *c*-Pr | C(=O)OCH₂CH=CH₂ | Cl | H | O |

**[Table 403]**

| Compound Number | Y | W | Z | R¹ | R² | R³ | R¹⁰ | A |
|---|---|---|---|---|---|---|---|---|
| C-7707 | Me(R) | single bond | H | *c*-Pr | C(=O)OCH₂CH=CH₂ | Cl | H | O |
| C-7708 | Me(S) | single bond | H | *c*-Pr | C(=O)OCH₂CH=CH₂ | Cl | H | O |
| C-7709 | H | CH₂ | Me | *c*-Pr | C(=O)OCH₂CH=CH₂ | Cl | H | O |
| C-7710 | Me | CH₂ | H | *c*-Pr | C(=O)OCH₂CH=CH₂ | Cl | H | O |
| C-7711 | H | CH(Me) | H | *c*-Pr | C(=O)OCH₂CH=CH₂ | Cl | H | O |
| C-7712 | H | CH₂CH₂ | Me | *c*-Pr | C(=O)OCH₂CH=CH₂ | Cl | H | O |
| C-7713 | Me | CH₂CH₂ | H | *c*-Pr | C(=O)OCH₂CH=CH₂ | Cl | H | O |
| C-7714 | H | single bond | Me | *c*-Pr | C(=O)OCH₂CH=CH₂ | Me | H | O |
| C-7715 | Me | single bond | H | *c*-Pr | C(=O)OCH₂CH=CH₂ | Me | H | O |
| C-7716 | Me(R) | single bond | H | *c*-Pr | C(=O)OCH₂CH=CH₂ | Me | H | O |
| C-7717 | Me(S) | single bond | H | *c*-Pr | C(=O)OCH₂CH=CH₂ | Me | H | O |
| C-7718 | H | CH₂ | Me | *c*-Pr | C(=O)OCH₂CH=CH₂ | Me | H | O |
| C-7719 | Me | CH₂ | H | *c*-Pr | C(=O)OCH₂CH=CH₂ | Me | H | O |
| C-7720 | H | CH(Me) | H | *c*-Pr | C(=O)OCH₂CH=CH₂ | Me | H | O |
| C-7721 | H | CH₂CH₂ | Me | *c*-Pr | C(=O)OCH₂CH=CH₂ | Me | H | O |
| C-7722 | Me | CH₂CH₂ | H | *c*-Pr | C(=O)OCH₂CH=CH₂ | Me | H | O |
| C-7723 | H | single bond | Me | *c*-Pr | C(=O)OCH₂CCl₃ | Cl | H | O |
| C-7724 | Me | single bond | H | *c*-Pr | C(=O)OCH₂CCl₃ | Cl | H | O |
| C-7725 | Me(R) | single bond | H | *c*-Pr | C(=O)OCH₂CCl₃ | Cl | H | O |
| C-7726 | Me(S) | single bond | H | *c*-Pr | C(=O)OCH₂CCl₃ | Cl | H | O |
| C-7727 | H | CH₂ | Me | *c*-Pr | C(=O)OCH₂CCl₃ | Cl | H | O |
| C-7728 | Me | CH₂ | H | *c*-Pr | C(=O)OCH₂CCl₃ | Cl | H | O |
| C-7729 | H | CH(Me) | H | *c*-Pr | C(=O)OCH₂CCl₃ | Cl | H | O |
| C-7730 | H | CH₂CH₂ | Me | *c*-Pr | C(=O)OCH₂CCl₃ | Cl | H | O |
| C-7731 | Me | CH₂CH₂ | H | *c*-Pr | C(=O)OCH₂CCl₃ | Cl | H | O |
| C-7732 | H | single bond | Me | *c*-Pr | C(=O)OCH₂CCl₃ | Me | H | O |
| C-7733 | Me | single bond | H | *c*-Pr | C(=O)OCH₂CCl₃ | Me | H | O |
| C-7734 | Me(R) | single bond | H | *c*-Pr | C(=O)OCH₂CCl₃ | Me | H | O |
| C-7735 | Me(S) | single bond | H | *c*-Pr | C(=O)OCH₂CCl₃ | Me | H | O |
| C-7736 | H | CH₂ | Me | *c*-Pr | C(=O)OCH₂CCl₃ | Me | H | O |
| C-7737 | Me | CH₂ | H | *c*-Pr | C(=O)OCH₂CCl₃ | Me | H | O |
| C-7738 | H | CH(Me) | H | *c*-Pr | C(=O)OCH₂CCl₃ | Me | H | O |
| C-7739 | H | CH₂CH₂ | Me | *c*-Pr | C(=O)OCH₂CCl₃ | Me | H | O |
| C-7740 | Me | CH₂CH₂ | H | *c*-Pr | C(=O)OCH₂CCl₃ | Me | H | O |
| C-7741 | H | single bond | Me | *c*-Pr | C(=O)N(Me)₂ | Cl | H | O |
| C-7742 | Me | single bond | H | *c*-Pr | C(=O)N(Me)₂ | Cl | H | O |
| C-7743 | Me(R) | single bond | H | *c*-Pr | C(=O)N(Me)₂ | Cl | H | O |
| C-7744 | Me(S) | single bond | H | *c*-Pr | C(=O)N(Me)₂ | Cl | H | O |
| C-7745 | H | CH₂ | Me | *c*-Pr | C(=O)N(Me)₂ | Cl | H | O |
| C-7746 | Me | CH₂ | H | *c*-Pr | C(=O)N(Me)₂ | Cl | H | O |
| C-7747 | H | CH(Me) | H | *c*-Pr | C(=O)N(Me)₂ | Cl | H | O |
| C-7748 | H | CH₂CH₂ | Me | *c*-Pr | C(=O)N(Me)₂ | Cl | H | O |
| C-7749 | Me | CH₂CH₂ | H | *c*-Pr | C(=O)N(Me)₂ | Cl | H | O |
| C-7750 | H | single bond | Me | *c*-Pr | C(=O)N(Me)₂ | Me | H | O |

**[Table 404]**

| Compound Number | Y | W | Z | R¹ | R² | R³ | R¹⁰ | A |
|---|---|---|---|---|---|---|---|---|
| C-7751 | Me | single bond | H | *c*-Pr | C(=O)N(Me)₂ | Me | H | O |
| C-7752 | Me(R) | single bond | H | *c*-Pr | C(=O)N(Me)₂ | Me | H | O |
| C-7753 | Me(S) | single bond | H | *c*-Pr | C(=O)N(Me)₂ | Me | H | O |
| C-7754 | H | CH₂ | Me | *c*-Pr | C(=O)N(Me)₂ | Me | H | O |
| C-7755 | Me | CH₂ | H | *c*-Pr | C(=O)N(Me)₂ | Me | H | O |
| C-7756 | H | CH(Me) | H | *c*-Pr | C(=O)N(Me)₂ | Me | H | O |
| C-7757 | H | CH₂CH₂ | Me | *c*-Pr | C(=O)N(Me)₂ | Me | H | O |
| C-7758 | Me | CH₂CH₂ | H | *c*-Pr | C(=O)N(Me)₂ | Me | H | O |
| C-7759 | H | single bond | Me | *c*-Pr | CH₂OC(=O)Me | Cl | H | O |
| C-7760 | Me | single bond | H | *c*-Pr | CH₂OC(=O)Me | Cl | H | O |
| C-7761 | Me(R) | single bond | H | *c*-Pr | CH₂OC(=O)Me | Cl | H | O |
| C-7762 | Me(S) | single bond | H | *c*-Pr | CH₂OC(=O)Me | Cl | H | O |
| C-7763 | H | CH₂ | Me | *c*-Pr | CH₂OC(=O)Me | Cl | H | O |
| C-7764 | Me | CH₂ | H | *c*-Pr | CH₂OC(=O)Me | Cl | H | O |
| C-7765 | H | CH(Me) | H | *c*-Pr | CH₂OC(=O)Me | Cl | H | O |
| C-7766 | H | CH₂CH₂ | Me | *c*-Pr | CH₂OC(=O)Me | Cl | H | O |
| C-7767 | Me | CH₂CH₂ | H | *c*-Pr | CH₂OC(=O)Me | Cl | H | O |
| C-7768 | H | single bond | Me | *c*-Pr | CH₂OC(=O)Me | Me | H | O |
| C-7769 | Me | single bond | H | *c*-Pr | CH₂OC(=O)Me | Me | H | O |
| C-7770 | Me(R) | single bond | H | *c*-Pr | CH₂OC(=O)Me | Me | H | O |
| C-7771 | Me(S) | single bond | H | *c*-Pr | CH₂OC(=O)Me | Me | H | O |
| C-7772 | H | CH₂ | Me | *c*-Pr | CH₂OC(=O)Me | Me | H | O |
| C-7773 | Me | CH₂ | H | *c*-Pr | CH₂OC(=O)Me | Me | H | O |
| C-7774 | H | CH(Me) | H | *c*-Pr | CH₂OC(=O)Me | Me | H | O |
| C-7775 | H | CH₂CH₂ | Me | *c*-Pr | CH₂OC(=O)Me | Me | H | O |
| C-7776 | Me | CH₂CH₂ | H | *c*-Pr | CH₂OC(=O)Me | Me | H | O |
| C-7777 | H | single bond | Me | *c*-Pr | CH₂OC(=O)(*i*-Pr) | Cl | H | O |
| C-7778 | Me | single bond | H | *c*-Pr | CH₂OC(=O)(*i*-Pr) | Cl | H | O |
| C-7779 | Me(R) | single bond | H | *c*-Pr | CH₂OC(=O)(*i*-Pr) | Cl | H | O |
| C-7780 | Me(S) | single bond | H | *c*-Pr | CH₂OC(=O)(*i*-Pr) | Cl | H | O |
| C-7781 | H | CH₂ | Me | *c*-Pr | CH₂OC(=O)(*i*-Pr) | Cl | H | O |
| C-7782 | Me | CH₂ | H | *c*-Pr | CH₂OC(=O)(*i*-Pr) | Cl | H | O |
| C-7783 | H | CH(Me) | H | *c*-Pr | CH₂OC(=O)(*i*-Pr) | Cl | H | O |
| C-7784 | H | CH₂CH₂ | Me | *c*-Pr | CH₂OC(=O)(*i*-Pr) | Cl | H | O |
| C-7785 | Me | CH₂CH₂ | H | *c*-Pr | CH₂OC(=O)(*i*-Pr) | Cl | H | O |
| C-7786 | H | single bond | Me | *c*-Pr | CH₂OC(=O)(*i*-Pr) | Me | H | O |
| C-7787 | Me | single bond | H | *c*-Pr | CH₂OC(=O)(*i*-Pr) | Me | H | O |
| C-7788 | Me(R) | single bond | H | *c*-Pr | CH₂OC(=O)(*i*-Pr) | Me | H | O |
| C-7789 | Me(S) | single bond | H | *c*-Pr | CH₂OC(=O)(*i*-Pr) | Me | H | O |
| C-7790 | H | CH₂ | Me | *c*-Pr | CH₂OC(=O)(*i*-Pr) | Me | H | O |
| C-7791 | Me | CH₂ | H | *c*-Pr | CH₂OC(=O)(*i*-Pr) | Me | H | O |
| C-7792 | H | CH(Me) | H | *c*-Pr | CH₂OC(=O)(*i*-Pr) | Me | H | O |
| C-7793 | H | CH₂CH₂ | Me | *c*-Pr | CH₂OC(=O)(*i*-Pr) | Me | H | O |
| C-7794 | Me | CH₂CH₂ | H | *c*-Pr | CH₂OC(=O)(*i*-Pr) | Me | H | O |

**[Table 405]**

| Compound Number | Y | W | Z | R¹ | R² | R³ | R¹⁰ | A |
|---|---|---|---|---|---|---|---|---|
| C-7795 | H | single bond | Me | *c*-Pr | CH₂OC(=O)(*t*-Bu) | Cl | H | O |
| C-7796 | Me | single bond | H | *c*-Pr | CH₂OC(=O)(*t*-Bu) | Cl | H | O |
| C-7797 | Me(R) | single bond | H | *c*-Pr | CH₂OC(=O)(*t*-Bu) | Cl | H | O |
| C-7798 | Me(S) | single bond | H | *c*-Pr | CH₂OC(=O)(*t*-Bu) | Cl | H | O |
| C-7799 | H | CH₂ | Me | *c*-Pr | CH₂OC(=O)(*t*-Bu) | Cl | H | O |
| C-7800 | Me | CH₂ | H | *c*-Pr | CH₂OC(=O)(*t*-Bu) | Cl | H | O |
| C-7801 | H | CH(Me) | H | *c*-Pr | CH₂OC(=O)(*t*-Bu) | Cl | H | O |
| C-7802 | H | CH₂CH₂ | Me | *c*-Pr | CH₂OC(=O)(*t*-Bu) | Cl | H | O |
| C-7803 | Me | CH₂CH₂ | H | *c*-Pr | CH₂OC(=O)(*t*-Bu) | Cl | H | O |
| C-7804 | H | single bond | Me | *c*-Pr | CH₂OC(=O)(*t*-Bu) | Me | H | O |
| C-7805 | Me | single bond | H | *c*-Pr | CH₂OC(=O)(*t*-Bu) | Me | H | O |
| C-7806 | Me(R) | single bond | H | *c*-Pr | CH₂OC(=O)(*t*-Bu) | Me | H | O |
| C-7807 | Me(S) | single bond | H | *c*-Pr | CH₂OC(=O)(*t*-Bu) | Me | H | O |
| C-7808 | H | CH₂ | Me | *c*-Pr | CH₂OC(=O)(*t*-Bu) | Me | H | O |
| C-7809 | Me | CH₂ | H | *c*-Pr | CH₂OC(=O)(*t*-Bu) | Me | H | O |
| C-7810 | H | CH(Me) | H | *c*-Pr | CH₂OC(=O)(*t*-Bu) | Me | H | O |
| C-7811 | H | CH₂CH₂ | Me | *c*-Pr | CH₂OC(=O)(*t*-Bu) | Me | H | O |
| C-7812 | Me | CH₂CH₂ | H | *c*-Pr | CH₂OC(=O)(*t*-Bu) | Me | H | O |
| C-7813 | H | single bond | Me | *c*-Pr | CH₂OC(=O)OEt | Cl | H | O |
| C-7814 | Me | single bond | H | *c*-Pr | CH₂OC(=O)OEt | Cl | H | O |
| C-7815 | Me(R) | single bond | H | *c*-Pr | CH₂OC(=O)OEt | Cl | H | O |
| C-7816 | Me(S) | single bond | H | *c*-Pr | CH₂OC(=O)OEt | Cl | H | O |
| C-7817 | H | CH₂ | Me | *c*-Pr | CH₂OC(=O)OEt | Cl | H | O |
| C-7818 | Me | CH₂ | H | *c*-Pr | CH₂OC(=O)OEt | Cl | H | O |
| C-7819 | H | CH(Me) | H | *c*-Pr | CH₂OC(=O)OEt | Cl | H | O |
| C-7820 | H | CH₂CH₂ | Me | *c*-Pr | CH₂OC(=O)OEt | Cl | H | O |
| C-7821 | Me | CH₂CH₂ | H | *c*-Pr | CH₂OC(=O)OEt | Cl | H | O |
| C-7822 | H | single bond | Me | *c*-Pr | CH₂OC(=O)OEt | Me | H | O |
| C-7823 | Me | single bond | H | *c*-Pr | CH₂OC(=O)OEt | Me | H | O |
| C-7824 | Me(R) | single bond | H | *c*-Pr | CH₂OC(=O)OEt | Me | H | O |
| C-7825 | Me(S) | single bond | H | *c*-Pr | CH₂OC(=O)OEt | Me | H | O |
| C-7826 | H | CH₂ | Me | *c*-Pr | CH₂OC(=O)OEt | Me | H | O |
| C-7827 | Me | CH₂ | H | *c*-Pr | CH₂OC(=O)OEt | Me | H | O |
| C-7828 | H | CH(Me) | H | *c*-Pr | CH₂OC(=O)OEt | Me | H | O |
| C-7829 | H | CH₂CH₂ | Me | *c*-Pr | CH₂OC(=O)OEt | Me | H | O |
| C-7830 | Me | CH₂CH₂ | H | *c*-Pr | CH₂OC(=O)OEt | Me | H | O |
| C-7831 | H | single bond | Me | *c*-Pr | CH₂OC(=O)O(*i*-Pr) | Cl | H | O |
| C-7832 | Me | single bond | H | *c*-Pr | CH₂OC(=O)O(*i*-Pr) | Cl | H | O |
| C-7833 | Me(R) | single bond | H | *c*-Pr | CH₂OC(=O)O(*i*-Pr) | Cl | H | O |
| C-7834 | Me(S) | single bond | H | *c*-Pr | CH₂OC(=O)O(*i*-Pr) | Cl | H | O |
| C-7835 | H | CH₂ | Me | *c*-Pr | CH₂OC(=O)O(*i*-Pr) | Cl | H | O |
| C-7836 | Me | CH₂ | H | *c*-Pr | CH₂OC(=O)O(*i*-Pr) | Cl | H | O |
| C-7837 | H | CH(Me) | H | *c*-Pr | CH₂OC(=O)O(*i*-Pr) | Cl | H | O |
| C-7838 | H | CH₂CH₂ | Me | *c*-Pr | CH₂OC(=O)O(*i*-Pr) | Cl | H | O |

**[Table 406]**

| Compound Number | Y | W | Z | R¹ | R² | R³ | R¹⁰ | A |
|---|---|---|---|---|---|---|---|---|
| C-7839 | Me | CH₂CH₂ | H | *c*-Pr | CH₂OC(=O)O(*i*-Pr) | Cl | H | O |
| C-7840 | H | single bond | Me | *c*-Pr | CH₂OC(=O)O(*i*-Pr) | Me | H | O |
| C-7841 | Me | single bond | H | *c*-Pr | CH₂OC(=O)O(*i*-Pr) | Me | H | O |
| C-7842 | Me(R) | single bond | H | *c*-Pr | CH₂OC(=O)O(*i*-Pr) | Me | H | O |
| C-7843 | Me(S) | single bond | H | *c*-Pr | CH₂OC(=O)O(*i*-Pr) | Me | H | O |
| C-7844 | H | CH₂ | Me | *c*-Pr | CH₂OC(=O)O(*i*-Pr) | Me | H | O |
| C-7845 | Me | CH₂ | H | *c*-Pr | CH₂OC(=O)O(*i*-Pr) | Me | H | O |
| C-7846 | H | CH(Me) | H | *c*-Pr | CH₂OC(=O)O(*i*-Pr) | Me | H | O |
| C-7847 | H | CH₂CH₂ | Me | *c*-Pr | CH₂OC(=O)O(*i*-Pr) | Me | H | O |
| C-7848 | Me | CH₂CH₂ | H | *c*-Pr | CH₂OC(=O)O(*i*-Pr) | Me | H | O |
| C-7849 | H | single bond | Me | *c*-Pr | CH₂N(Me)C(=O)OMe | Cl | H | O |
| C-7850 | Me | single bond | H | *c*-Pr | CH₂N(Me)C(=O)OMe | Cl | H | O |
| C-7851 | Me(R) | single bond | H | *c*-Pr | CH₂N(Me)C(=O)OMe | Cl | H | O |
| C-7852 | Me(S) | single bond | H | *c*-Pr | CH₂N(Me)C(=O)OMe | Cl | H | O |
| C-7853 | H | CH₂ | Me | *c*-Pr | CH₂N(Me)C(=O)OMe | Cl | H | O |
| C-7854 | Me | CH₂ | H | *c*-Pr | CH₂N(Me)C(=O)OMe | Cl | H | O |
| C-7855 | H | CH(Me) | H | *c*-Pr | CH₂N(Me)C(=O)OMe | Cl | H | O |
| C-7856 | H | CH₂CH₂ | Me | *c*-Pr | CH₂N(Me)C(=O)OMe | Cl | H | O |
| C-7857 | Me | CH₂CH₂ | H | *c*-Pr | CH₂N(Me)C(=O)OMe | Cl | H | O |
| C-7858 | H | single bond | Me | *c*-Pr | CH₂N(Me)C(=O)OMe | Me | H | O |
| C-7859 | Me | single bond | H | *c*-Pr | CH₂N(Me)C(=O)OMe | Me | H | O |
| C-7860 | Me(R) | single bond | H | *c*-Pr | CH₂N(Me)C(=O)OMe | Me | H | O |
| C-7861 | Me(S) | single bond | H | *c*-Pr | CH₂N(Me)C(=O)OMe | Me | H | O |
| C-7862 | H | CH₂ | Me | *c*-Pr | CH₂N(Me)C(=O)OMe | Me | H | O |
| C-7863 | Me | CH₂ | H | *c*-Pr | CH₂N(Me)C(=O)OMe | Me | H | O |
| C-7864 | H | CH(Me) | H | *c*-Pr | CH₂N(Me)C(=O)OMe | Me | H | O |
| C-7865 | H | CH₂CH₂ | Me | *c*-Pr | CH₂N(Me)C(=O)OMe | Me | H | O |
| C-7866 | Me | CH₂CH₂ | H | *c*-Pr | CH₂N(Me)C(=O)OMe | Me | H | O |
| C-7867 | H | single bond | Me | *c*-Pr | C(=O)OCH₂CH=CH₂ | Cl | Me | O |
| C-7868 | Me | single bond | H | *c*-Pr | C(=O)OCH₂CH=CH₂ | Cl | Me | O |
| C-7869 | Me(R) | single bond | H | *c*-Pr | C(=O)OCH₂CH=CH₂ | Cl | Me | O |
| C-7870 | Me(S) | single bond | H | *c*-Pr | C(=O)OCH₂CH=CH₂ | Cl | Me | O |
| C-7871 | H | CH₂ | Me | *c*-Pr | C(=O)OCH₂CH=CH₂ | Cl | Me | O |
| C-7872 | Me | CH₂ | H | *c*-Pr | C(=O)OCH₂CH=CH₂ | Cl | Me | O |
| C-7873 | H | CH(Me) | H | *c*-Pr | C(=O)OCH₂CH=CH₂ | Cl | Me | O |
| C-7874 | H | CH₂CH₂ | Me | *c*-Pr | C(=O)OCH₂CH=CH₂ | Cl | Me | O |
| C-7875 | Me | CH₂CH₂ | H | *c*-Pr | C(=O)OCH₂CH=CH₂ | Cl | Me | O |
| C-7876 | H | single bond | Me | *c*-Pr | C(=O)OCH₂CH=CH₂ | Me | Me | O |
| C-7877 | Me | single bond | H | *c*-Pr | C(=O)OCH₂CH=CH₂ | Me | Me | O |
| C-7878 | Me(R) | single bond | H | *c*-Pr | C(=O)OCH₂CH=CH₂ | Me | Me | O |
| C-7879 | Me(S) | single bond | H | *c*-Pr | C(=O)OCH₂CH=CH₂ | Me | Me | O |
| C-7880 | H | CH₂ | Me | *c*-Pr | C(=O)OCH₂CH=CH₂ | Me | Me | O |
| C-7881 | Me | CH₂ | H | *c*-Pr | C(=O)OCH₂CH=CH₂ | Me | Me | O |
| C-7882 | H | CH(Me) | H | *c*-Pr | C(=O)OCH₂CH=CH₂ | Me | Me | O |

**[Table 407]**

| Compound Number | Y | W | Z | R¹ | R² | R³ | R¹⁰ | A |
|---|---|---|---|---|---|---|---|---|
| C-7883 | H | CH₂CH₂ | Me | *c*-Pr | C(=O)OCH₂CH=CH₂ | Me | Me | O |
| C-7884 | Me | CH₂CH₂ | H | *c*-Pr | C(=O)OCH₂CH=CH₂ | Me | Me | O |
| C-7885 | H | single bond | Me | *c*-Pr | C(=O)OCH₂CCl₃ | Cl | Me | O |
| C-7886 | Me | single bond | H | *c*-Pr | C(=O)OCH₂CCl₃ | Cl | Me | O |
| C-7887 | Me(R) | single bond | H | *c*-Pr | C(=O)OCH₂CCl₃ | Cl | Me | O |
| C-7888 | Me(S) | single bond | H | *c*-Pr | C(=O)OCH₂CCl₃ | Cl | Me | O |
| C-7889 | H | CH₂ | Me | *c*-Pr | C(=O)OCH₂CCl₃ | Cl | Me | O |
| C-7890 | Me | CH₂ | H | *c*-Pr | C(=O)OCH₂CCl₃ | Cl | Me | O |
| C-7891 | H | CH(Me) | H | *c*-Pr | C(=O)OCH₂CCl₃ | Cl | Me | O |
| C-7892 | H | CH₂CH₂ | Me | *c*-Pr | C(=O)OCH₂CCl₃ | Cl | Me | O |
| C-7893 | Me | CH₂CH₂ | H | *c*-Pr | C(=O)OCH₂CCl₃ | Cl | Me | O |
| C-7894 | H | single bond | Me | *c*-Pr | C(=O)OCH₂CCl₃ | Me | Me | O |
| C-7895 | Me | single bond | H | *c*-Pr | C(=O)OCH₂CCl₃ | Me | Me | O |
| C-7896 | Me(R) | single bond | H | *c*-Pr | C(=O)OCH₂CCl₃ | Me | Me | O |
| C-7897 | Me(S) | single bond | H | *c*-Pr | C(=O)OCH₂CCl₃ | Me | Me | O |
| C-7898 | H | CH₂ | Me | *c*-Pr | C(=O)OCH₂CCl₃ | Me | Me | O |
| C-7899 | Me | CH₂ | H | *c*-Pr | C(=O)OCH₂CCl₃ | Me | Me | O |
| C-7900 | H | CH(Me) | H | *c*-Pr | C(=O)OCH₂CCl₃ | Me | Me | O |
| C-7901 | H | CH₂CH₂ | Me | *c*-Pr | C(=O)OCH₂CCl₃ | Me | Me | O |
| C-7902 | Me | CH₂CH₂ | H | *c*-Pr | C(=O)OCH₂CCl₃ | Me | Me | O |
| C-7903 | H | single bond | Me | *c*-Pr | C(=O)N(Me)₂ | Cl | Me | O |
| C-7904 | Me | single bond | H | *c*-Pr | C(=O)N(Me)₂ | Cl | Me | O |
| C-7905 | Me(R) | single bond | H | *c*-Pr | C(=O)N(Me)₂ | Cl | Me | O |
| C-7906 | Me(S) | single bond | H | *c*-Pr | C(=O)N(Me)₂ | Cl | Me | O |
| C-7907 | H | CH₂ | Me | *c*-Pr | C(=O)N(Me)₂ | Cl | Me | O |
| C-7908 | Me | CH₂ | H | *c*-Pr | C(=O)N(Me)₂ | Cl | Me | O |
| C-7909 | H | CH(Me) | H | *c*-Pr | C(=O)N(Me)₂ | Cl | Me | O |
| C-7910 | H | CH₂CH₂ | Me | *c*-Pr | C(=O)N(Me)₂ | Cl | Me | O |
| C-7911 | Me | CH₂CH₂ | H | *c*-Pr | C(=O)N(Me)₂ | Cl | Me | O |
| C-7912 | H | single bond | Me | *c*-Pr | C(=O)N(Me)₂ | Me | Me | O |
| C-7913 | Me | single bond | H | *c*-Pr | C(=O)N(Me)₂ | Me | Me | O |
| C-7914 | Me(R) | single bond | H | *c*-Pr | C(=O)N(Me)₂ | Me | Me | O |
| C-7915 | Me(S) | single bond | H | *c*-Pr | C(=O)N(Me)₂ | Me | Me | O |
| C-7916 | H | CH₂ | Me | *c*-Pr | C(=O)N(Me)₂ | Me | Me | O |
| C-7917 | Me | CH₂ | H | *c*-Pr | C(=O)N(Me)₂ | Me | Me | O |
| C-7918 | H | CH(Me) | H | *c*-Pr | C(=O)N(Me)₂ | Me | Me | O |
| C-7919 | H | CH₂CH₂ | Me | *c*-Pr | C(=O)N(Me)₂ | Me | Me | O |
| C-7920 | Me | CH₂CH₂ | H | *c*-Pr | C(=O)N(Me)₂ | Me | Me | O |
| C-7921 | H | single bond | Me | *c*-Pr | CH₂OC(=O)Me | Cl | Me | O |
| C-7922 | Me | single bond | H | *c*-Pr | CH₂OC(=O)Me | Cl | Me | O |
| C-7923 | Me(R) | single bond | H | *c*-Pr | CH₂OC(=O)Me | Cl | Me | O |
| C-7924 | Me(S) | single bond | H | *c*-Pr | CH₂OC(=O)Me | Cl | Me | O |
| C-7925 | H | CH₂ | Me | *c*-Pr | CH₂OC(=O)Me | Cl | Me | O |
| C-7926 | Me | CH₂ | H | *c*-Pr | CH₂OC(=O)Me | Cl | Me | O |

**[Table 408]**

| Compound Number | Y | W | Z | R¹ | R² | R³ | R¹⁰ | A |
|---|---|---|---|---|---|---|---|---|
| C-7927 | H | CH(Me) | H | *c*-Pr | CH₂OC(=O)Me | Cl | Me | O |
| C-7928 | H | CH₂CH₂ | Me | *c*-Pr | CH₂OC(=O)Me | Cl | Me | O |
| C-7929 | Me | CH₂CH₂ | H | *c*-Pr | CH₂OC(=O)Me | Cl | Me | O |
| C-7930 | H | single bond | Me | *c*-Pr | CH₂OC(=O)Me | Me | Me | O |
| C-7931 | Me | single bond | H | *c*-Pr | CH₂OC(=O)Me | Me | Me | O |
| C-7932 | Me(R) | single bond | H | *c*-Pr | CH₂OC(=O)Me | Me | Me | O |
| C-7933 | Me(S) | single bond | H | *c*-Pr | CH₂OC(=O)Me | Me | Me | O |
| C-7934 | H | CH₂ | Me | *c*-Pr | CH₂OC(=O)Me | Me | Me | O |
| C-7935 | Me | CH₂ | H | *c*-Pr | CH₂OC(=O)Me | Me | Me | O |
| C-7936 | H | CH(Me) | H | *c*-Pr | CH₂OC(=O)Me | Me | Me | O |
| C-7937 | H | CH₂CH₂ | Me | *c*-Pr | CH₂OC(=O)Me | Me | Me | O |
| C-7938 | Me | CH₂CH₂ | H | *c*-Pr | CH₂OC(=O)Me | Me | Me | O |
| C-7939 | H | single bond | Me | *c*-Pr | CH₂OC(=O)(*i* -Pr) | Cl | Me | O |
| C-7940 | Me | single bond | H | *c*-Pr | CH₂OC(=O)(*i* -Pr) | Cl | Me | O |
| C-7941 | Me(R) | single bond | H | *c*-Pr | CH₂OC(=O)(*i* -Pr) | Cl | Me | O |
| C-7942 | Me(S) | single bond | H | *c*-Pr | CH₂OC(=O)(*i* -Pr) | Cl | Me | O |
| C-7943 | H | CH₂ | Me | *c*-Pr | CH₂OC(=O)(*i* -Pr) | Cl | Me | O |
| C-7944 | Me | CH₂ | H | *c*-Pr | CH₂OC(=O)(*i*-Pr) | Cl | Me | O |
| C-7945 | H | CH(Me) | H | *c*-Pr | CH₂OC(=O)(*i*-Pr) | Cl | Me | O |
| C-7946 | H | CH₂CH₂ | Me | *c*-Pr | CH₂OC(=O)(*i* -Pr) | Cl | Me | O |
| C-7947 | Me | CH₂CH₂ | H | *c*-Pr | CH₂OC(=O)(*i* -Pr) | Cl | Me | O |
| C-7948 | H | single bond | Me | *c*-Pr | CH₂OC(=O)(*i*-Pr) | Me | Me | O |
| C-7949 | Me | single bond | H | *c*-Pr | CH₂OC(=O)(*i*-Pr) | Me | Me | O |
| C-7950 | Me(R) | single bond | H | *c*-Pr | CH₂OC(=O)(*i* -Pr) | Me | Me | O |
| C-7951 | Me(S) | single bond | H | *c*-Pr | CH₂OC(=O)(*i* -Pr) | Me | Me | O |
| C-7952 | H | CH₂ | Me | *c*-Pr | CH₂OC(=O)(*i*-Pr) | Me | Me | O |
| C-7953 | Me | CH₂ | H | *c*-Pr | CH₂OC(=O)(*i* -Pr) | Me | Me | O |
| C-7954 | H | CH(Me) | H | *c*-Pr | CH₂OC(=O)(*i* -Pr) | Me | Me | O |
| C-7955 | H | CH₂CH₂ | Me | *c*-Pr | CH₂OC(=O)(*i*-Pr) | Me | Me | O |
| C-7956 | Me | CH₂CH₂ | H | *c*-Pr | CH₂OC(=O)(*i*-Pr) | Me | Me | O |
| C-7957 | H | single bond | Me | *c*-Pr | CH₂OC(=O)(*t*-Bu) | Cl | Me | O |
| C-7958 | Me | single bond | H | *c*-Pr | CH₂OC(=O)(*t*-Bu) | Cl | Me | O |
| C-7959 | Me(R) | single bond | H | *c*-Pr | CH₂OC(=O)(*t*-Bu) | Cl | Me | O |
| C-7960 | Me(S) | single bond | H | *c*-Pr | CH₂OC(=O)(*t*-Bu) | Cl | Me | O |
| C-7961 | H | CH₂ | Me | *c*-Pr | CH₂OC(=O)(*t*-Bu) | Cl | Me | O |
| C-7962 | Me | CH₂ | H | *c*-Pr | CH₂OC(=O)(*t*-Bu) | Cl | Me | O |
| C-7963 | H | CH(Me) | H | *c*-Pr | CH₂OC(=O)(*t*-Bu) | Cl | Me | O |
| C-7964 | H | CH₂CH₂ | Me | *c*-Pr | CH₂OC(=O)(*t*-Bu) | Cl | Me | O |
| C-7965 | Me | CH₂CH₂ | H | *c*-Pr | CH₂OC(=O)(*t*-Bu) | Cl | Me | O |
| C-7966 | H | single bond | Me | *c*-Pr | CH₂OC(=O)(*t*-Bu) | Me | Me | O |
| C-7967 | Me | single bond | H | *c*-Pr | CH₂OC(=O)(*t*-Bu) | Me | Me | O |
| C-7968 | Me(R) | single bond | H | *c*-Pr | CH₂OC(=O)(*t*-Bu) | Me | Me | O |
| C-7969 | Me(S) | single bond | H | *c*-Pr | CH₂OC(=O)(*t*-Bu) | Me | Me | O |
| C-7970 | H | CH₂ | Me | *c*-Pr | CH₂OC(=O)(*t*-Bu) | Me | Me | O |

**[Table 409]**

| Compound Number | Y | W | Z | R¹ | R² | R³ | R¹⁰ | A |
|---|---|---|---|---|---|---|---|---|
| C-7971 | Me | CH₂ | H | *c*-Pr | CH₂OC(=O)(*t*-Bu) | Me | Me | O |
| C-7972 | H | CH(Me) | H | *c*-Pr | CH₂OC(=O)(*t*-Bu) | Me | Me | O |
| C-7973 | H | CH₂CH₂ | Me | *c*-Pr | CH₂OC(=O)(*t*-Bu) | Me | Me | O |
| C-7974 | Me | CH₂CH₂ | H | *c*-Pr | CH₂OC(=O)(*t*-Bu) | Me | Me | O |
| C-7975 | H | single bond | Me | *c*-Pr | CH₂OC(=O)OEt | Cl | Me | O |
| C-7976 | Me | single bond | H | *c*-Pr | CH₂OC(=O)OEt | Cl | Me | O |
| C-7977 | Me(R) | single bond | H | *c*-Pr | CH₂OC(=O)OEt | Cl | Me | O |
| C-7978 | Me(S) | single bond | H | *c*-Pr | CH₂OC(=O)OEt | Cl | Me | O |
| C-7979 | H | CH₂ | Me | *c*-Pr | CH₂OC(=O)OEt | Cl | Me | O |
| C-7980 | Me | CH₂ | H | *c*-Pr | CH₂OC(=O)OEt | Cl | Me | O |
| C-7981 | H | CH(Me) | H | *c*-Pr | CH₂OC(=O)OEt | Cl | Me | O |
| C-7982 | H | CH₂CH₂ | Me | *c*-Pr | CH₂OC(=O)OEt | Cl | Me | O |
| C-7983 | Me | CH₂CH₂ | H | *c*-Pr | CH₂OC(=O)OEt | Cl | Me | O |
| C-7984 | H | single bond | Me | *c*-Pr | CH₂OC(=O)OEt | Me | Me | O |
| C-7985 | Me | single bond | H | *c*-Pr | CH₂OC(=O)OEt | Me | Me | O |
| C-7986 | Me(R) | single bond | H | *c*-Pr | CH₂OC(=O)OEt | Me | Me | O |
| C-7987 | Me(S) | single bond | H | *c*-Pr | CH₂OC(=O)OEt | Me | Me | O |
| C-7988 | H | CH₂ | Me | *c*-Pr | CH₂OC(=O)OEt | Me | Me | O |
| C-7989 | Me | CH₂ | H | *c*-Pr | CH₂OC(=O)OEt | Me | Me | O |
| C-7990 | H | CH(Me) | H | *c*-Pr | CH₂OC(=O)OEt | Me | Me | O |
| C-7991 | H | CH₂CH₂ | Me | *c*-Pr | CH₂OC(=O)OEt | Me | Me | O |
| C-7992 | Me | CH₂CH₂ | H | *c*-Pr | CH₂OC(=O)OEt | Me | Me | O |
| C-7993 | H | single bond | Me | *c*-Pr | CH₂OC(=O)O(*i*-Pr) | Cl | Me | O |
| C-7994 | Me | single bond | H | *c*-Pr | CH₂OC(=O)O(*i*-Pr) | Cl | Me | O |
| C-7995 | Me(R) | single bond | H | *c*-Pr | CH₂OC(=O)O(*i*-Pr) | Cl | Me | O |
| C-7996 | Me(S) | single bond | H | *c*-Pr | CH₂OC(=O)O(*i*-Pr) | Cl | Me | O |
| C-7997 | H | CH₂ | Me | *c*-Pr | CH₂OC(=O)O(*i*-Pr) | Cl | Me | O |
| C-7998 | Me | CH₂ | H | *c*-Pr | CH₂OC(=O)O(*i*-Pr) | Cl | Me | O |
| C-7999 | H | CH(Me) | H | *c*-Pr | CH₂OC(=O)O(*i*-Pr) | Cl | Me | O |
| C-8000 | H | CH₂CH₂ | Me | *c*-Pr | CH₂OC(=O)O(*i*-Pr) | Cl | Me | O |
| C-8001 | Me | CH₂CH₂ | H | *c*-Pr | CH₂OC(=O)O(*i*-Pr) | Cl | Me | O |
| C-8002 | H | single bond | Me | *c*-Pr | CH₂OC(=O)O(*i*-Pr) | Me | Me | O |
| C-8003 | Me | single bond | H | *c*-Pr | CH₂OC(=O)O(*i*-Pr) | Me | Me | O |
| C-8004 | Me(R) | single bond | H | *c*-Pr | CH₂OC(=O)O(*i*-Pr) | Me | Me | O |
| C-8005 | Me(S) | single bond | H | *c*-Pr | CH₂OC(=O)O(*i*-Pr) | Me | Me | O |
| C-8006 | H | CH₂ | Me | *c*-Pr | CH₂OC(=O)O(*i*-Pr) | Me | Me | O |
| C-8007 | Me | CH₂ | H | *c*-Pr | CH₂OC(=O)O(*i*-Pr) | Me | Me | O |
| C-8008 | H | CH(Me) | H | *c*-Pr | CH₂OC(=O)O(*i*-Pr) | Me | Me | O |
| C-8009 | H | CH₂CH₂ | Me | *c*-Pr | CH₂OC(=O)O(*i*-Pr) | Me | Me | O |
| C-8010 | Me | CH₂CH₂ | H | *c*-Pr | CH₂OC(=O)O(*i*-Pr) | Me | Me | O |
| C-8011 | H | single bond | Me | *c*-Pr | CH₂N(Me)C(=O)OMe | Cl | Me | O |
| C-8012 | Me | single bond | H | *c*-Pr | CH₂N(Me)C(=O)OMe | Cl | Me | O |
| C-8013 | Me(R) | single bond | H | *c*-Pr | CH₂N(Me)C(=O)OMe | Cl | Me | O |
| C-8014 | Me(S) | single bond | H | *c*-Pr | CH₂N(Me)C(=O)OMe | Cl | Me | O |

**[Table 410]**

| Compound Number | Y | W | Z | R¹ | R² | R³ | R¹⁰ | A |
|---|---|---|---|---|---|---|---|---|
| C-8015 | H | CH₂ | Me | *c*-Pr | CH₂N(Me)C(=O)OMe | Cl | Me | O |
| C-8016 | Me | CH₂ | H | *c*-Pr | CH₂N(Me)C(=O)OMe | Cl | Me | O |
| C-8017 | H | CH(Me) | H | *c*-Pr | CH₂N(Me)C(=O)OMe | Cl | Me | O |
| C-8018 | H | CH₂CH₂ | Me | *c*-Pr | CH₂N(Me)C(=O)OMe | Cl | Me | O |
| C-8019 | Me | CH₂CH₂ | H | *c*-Pr | CH₂N(Me)C(=O)OMe | Cl | Me | O |
| C-8020 | H | single bond | Me | *c*-Pr | CH₂N(Me)C(=O)OMe | Me | Me | O |
| C-8021 | Me | single bond | H | *c*-Pr | CH₂N(Me)C(=O)OMe | Me | Me | O |
| C-8022 | Me(R) | single bond | H | *c*-Pr | CH₂N(Me)C(=O)OMe | Me | Me | O |
| C-8023 | Me(S) | single bond | H | *c*-Pr | CH₂N(Me)C(=O)OMe | Me | Me | O |
| C-8024 | H | CH₂ | Me | *c*-Pr | CH₂N(Me)C(=O)OMe | Me | Me | O |
| C-8025 | Me | CH₂ | H | *c*-Pr | CH₂N(Me)C(=O)OMe | Me | Me | O |
| C-8026 | H | CH(Me) | H | *c*-Pr | CH₂N(Me)C(=O)OMe | Me | Me | O |
| C-8027 | H | CH₂CH₂ | Me | *c*-Pr | CH₂N(Me)C(=O)OMe | Me | Me | O |
| C-8028 | Me | CH₂CH₂ | H | *c*-Pr | CH₂N(Me)C(=O)OMe | Me | Me | O |
| C-8029 | H | single bond | Me | *c*-Pr | C(=O)OCH₂CH=CH₂ | Cl | CH₂OMe | O |
| C-8030 | Me | single bond | H | *c*-Pr | C(=O)OCH₂CH=CH₂ | Cl | CH₂OMe | O |
| C-8031 | Me(R) | single bond | H | *c*-Pr | C(=O)OCH₂CH=CH₂ | Cl | CH₂OMe | O |
| C-8032 | Me(S) | single bond | H | *c*-Pr | C(=O)OCH₂CH=CH₂ | Cl | CH₂OMe | O |
| C-8033 | H | CH₂ | Me | *c*-Pr | C(=O)OCH₂CH=CH₂ | Cl | CH₂OMe | O |
| C-8034 | Me | CH₂ | H | *c*-Pr | C(=O)OCH₂CH=CH₂ | Cl | CH₂OMe | O |
| C-8035 | H | CH(Me) | H | *c*-Pr | C(=O)OCH₂CH=CH₂ | Cl | CH₂OMe | O |
| C-8036 | H | CH₂CH₂ | Me | *c*-Pr | C(=O)OCH₂CH=CH₂ | Cl | CH₂OMe | O |
| C-8037 | Me | CH₂CH₂ | H | *c*-Pr | C(=O)OCH₂CH=CH₂ | Cl | CH₂OMe | O |
| C-8038 | H | single bond | Me | *c*-Pr | C(=O)OCH₂CH=CH₂ | Me | CH₂OMe | O |
| C-8039 | Me | single bond | H | *c*-Pr | C(=O)OCH₂CH=CH₂ | Me | CH₂OMe | O |
| C-8040 | Me(R) | single bond | H | *c*-Pr | C(=O)OCH₂CH=CH₂ | Me | CH₂OMe | O |
| C-8041 | Me(S) | single bond | H | *c*-Pr | C(=O)OCH₂CH=CH₂ | Me | CH₂OMe | O |
| C-8042 | H | CH₂ | Me | *c*-Pr | C(=O)OCH₂CH=CH₂ | Me | CH₂OMe | O |
| C-8043 | Me | CH₂ | H | *c*-Pr | C(=O)OCH₂CH=CH₂ | Me | CH₂OMe | O |
| C-8044 | H | CH(Me) | H | *c*-Pr | C(=O)OCH₂CH=CH₂ | Me | CH₂OMe | O |
| C-8045 | H | CH₂CH₂ | Me | *c*-Pr | C(=O)OCH₂CH=CH₂ | Me | CH₂OMe | O |
| C-8046 | Me | CH₂CH₂ | H | *c*-Pr | C(=O)OCH₂CH=CH₂ | Me | CH₂OMe | O |
| C-8047 | H | single bond | Me | *c*-Pr | C(=O)OCH₂CCl₃ | Cl | CH₂OMe | O |
| C-8048 | Me | single bond | H | *c*-Pr | C(=O)OCH₂CCl₃ | Cl | CH₂OMe | O |
| C-8049 | Me(R) | single bond | H | *c*-Pr | C(=O)OCH₂CCl₃ | Cl | CH₂OMe | O |
| C-8050 | Me(S) | single bond | H | *c*-Pr | C(=O)OCH₂CCl₃ | Cl | CH₂OMe | O |
| C-8051 | H | CH₂ | Me | *c*-Pr | C(=O)OCH₂CCl₃ | Cl | CH₂OMe | O |
| C-8052 | Me | CH₂ | H | *c*-Pr | C(=O)OCH₂CCl₃ | Cl | CH₂OMe | O |
| C-8053 | H | CH(Me) | H | *c*-Pr | C(=O)OCH₂CCl₃ | Cl | CH₂OMe | O |
| C-8054 | H | CH₂CH₂ | Me | *c*-Pr | C(=O)OCH₂CCl₃ | Cl | CH₂OMe | O |
| C-8055 | Me | CH₂CH₂ | H | *c*-Pr | C(=O)OCH₂CCl₃ | Cl | CH₂OMe | O |
| C-8056 | H | single bond | Me | *c*-Pr | C(=O)OCH₂CCl₃ | Me | CH₂OMe | O |
| C-8057 | Me | single bond | H | *c*-Pr | C(=O)OCH₂CCl₃ | Me | CH₂OMe | O |
| C-8058 | Me(R) | single bond | H | *c*-Pr | C(=O)OCH₂CCl₃ | Me | CH₂OMe | O |

**[Table 411]**

| Compound Number | Y | W | Z | R¹ | R² | R³ | R¹⁰ | A |
|---|---|---|---|---|---|---|---|---|
| C-8059 | Me(S) | single bond | H | *c*-Pr | C(=O)OCH₂CCl₃ | Me | CH₂OMe | O |
| C-8060 | H | CH₂ | Me | *c*-Pr | C(=O)OCH₂CCl₃ | Me | CH₂OMe | O |
| C-8061 | Me | CH₂ | H | *c*-Pr | C(=O)OCH₂CCl₃ | Me | CH₂OMe | O |
| C-8062 | H | CH(Me) | H | *c*-Pr | C(=O)OCH₂CCl₃ | Me | CH₂OMe | O |
| C-8063 | H | CH₂CH₂ | Me | *c*-Pr | C(=O)OCH₂CCl₃ | Me | CH₂OMe | O |
| C-8064 | Me | CH₂CH₂ | H | *c*-Pr | C(=O)OCH₂CCl₃ | Me | CH₂OMe | O |
| C-8065 | H | single bond | Me | *c*-Pr | C(=O)N(Me)₂ | Cl | CH₂OMe | O |
| C-8066 | Me | single bond | H | *c*-Pr | C(=O)N(Me)₂ | Cl | CH₂OMe | O |
| C-8067 | Me(R) | single bond | H | *c*-Pr | C(=O)N(Me)₂ | Cl | CH₂OMe | O |
| C-8068 | Me(S) | single bond | H | *c*-Pr | C(=O)N(Me)₂ | Cl | CH₂OMe | O |
| C-8069 | H | CH₂ | Me | *c*-Pr | C(=O)N(Me)₂ | Cl | CH₂OMe | O |
| C-8070 | Me | CH₂ | H | *c*-Pr | C(=O)N(Me)₂ | Cl | CH₂OMe | O |
| C-8071 | H | CH(Me) | H | *c*-Pr | C(=O)N(Me)₂ | Cl | CH₂OMe | O |
| C-8072 | H | CH₂CH₂ | Me | *c*-Pr | C(=O)N(Me)₂ | Cl | CH₂OMe | O |
| C-8073 | Me | CH₂CH₂ | H | *c*-Pr | C(=O)N(Me)₂ | Cl | CH₂OMe | O |
| C-8074 | H | single bond | Me | *c*-Pr | C(=O)N(Me)₂ | Me | CH₂OMe | O |
| C-8075 | Me | single bond | H | *c*-Pr | C(=O)N(Me)₂ | Me | CH₂OMe | O |
| C-8076 | Me(R) | single bond | H | *c*-Pr | C(=O)N(Me)₂ | Me | CH₂OMe | O |
| C-8077 | Me(S) | single bond | H | *c*-Pr | C(=O)N(Me)₂ | Me | CH₂OMe | O |
| C-8078 | H | CH₂ | Me | *c*-Pr | C(=O)N(Me)₂ | Me | CH₂OMe | O |
| C-8079 | Me | CH₂ | H | *c*-Pr | C(=O)N(Me)₂ | Me | CH₂OMe | O |
| C-8080 | H | CH(Me) | H | *c*-Pr | C(=O)N(Me)₂ | Me | CH₂OMe | O |
| C-8081 | H | CH₂CH₂ | Me | *c*-Pr | C(=O)N(Me)₂ | Me | CH₂OMe | O |
| C-8082 | Me | CH₂CH₂ | H | *c*-Pr | C(=O)N(Me)₂ | Me | CH₂OMe | O |
| C-8083 | H | single bond | Me | *c*-Pr | CH₂OC(=O)Me | Cl | CH₂OMe | O |
| C-8084 | Me | single bond | H | *c*-Pr | CH₂OC(=O)Me | Cl | CH₂OMe | O |
| C-8085 | Me(R) | single bond | H | *c*-Pr | CH₂OC(=O)Me | Cl | CH₂OMe | O |
| C-8086 | Me(S) | single bond | H | *c*-Pr | CH₂OC(=O)Me | Cl | CH₂OMe | O |
| C-8087 | H | CH₂ | Me | *c*-Pr | CH₂OC(=O)Me | Cl | CH₂OMe | O |
| C-8088 | Me | CH₂ | H | *c*-Pr | CH₂OC(=O)Me | Cl | CH₂OMe | O |
| C-8089 | H | CH(Me) | H | *c*-Pr | CH₂OC(=O)Me | Cl | CH₂OMe | O |
| C-8090 | H | CH₂CH₂ | Me | *c*-Pr | CH₂OC(=O)Me | Cl | CH₂OMe | O |
| C-8091 | Me | CH₂CH₂ | H | *c*-Pr | CH₂OC(=O)Me | Cl | CH₂OMe | O |
| C-8092 | H | single bond | Me | *c*-Pr | CH₂OC(=O)Me | Me | CH₂OMe | O |
| C-8093 | Me | single bond | H | *c*-Pr | CH₂OC(=O)Me | Me | CH₂OMe | O |
| C-8094 | Me(R) | single bond | H | *c*-Pr | CH₂OC(=O)Me | Me | CH₂OMe | O |
| C-8095 | Me(S) | single bond | H | *c*-Pr | CH₂OC(=O)Me | Me | CH₂OMe | O |
| C-8096 | H | CH₂ | Me | *c*-Pr | CH₂OC(=O)Me | Me | CH₂OMe | O |
| C-8097 | Me | CH₂ | H | *c*-Pr | CH₂OC(=O)Me | Me | CH₂OMe | O |
| C-8098 | H | CH(Me) | H | *c*-Pr | CH₂OC(=O)Me | Me | CH₂OMe | O |
| C-8099 | H | CH₂CH₂ | Me | *c*-Pr | CH₂OC(=O)Me | Me | CH₂OMe | O |
| C-8100 | Me | CH₂CH₂ | H | *c*-Pr | CH₂OC(=O)Me | Me | CH₂OMe | O |
| C-8101 | H | single bond | Me | *c*-Pr | CH₂OC(=O)(*i*-Pr) | Cl | CH₂OMe | O |
| C-8102 | Me | single bond | H | *c*-Pr | CH₂OC(=O)(*i* -Pr) | Cl | CH₂OMe | O |

**[Table 412]**

| Compound Number | Y | W | Z | R¹ | R² | R³ | R¹⁰ | A |
|---|---|---|---|---|---|---|---|---|
| C-8103 | Me(R) | single bond | H | *c*-Pr | CH₂OC(=O)(*i*-Pr) | Cl | CH₂OMe | O |
| C-8104 | Me(S) | single bond | H | *c*-Pr | CH₂OC(=O)(*i*-Pr) | Cl | CH₂OMe | O |
| C-8105 | H | CH₂ | Me | *c*-Pr | CH₂OC(=O)(*i* -Pr) | Cl | CH₂OMe | O |
| C-8106 | Me | CH₂ | H | *c*-Pr | CH₂OC(=O)(*i* -Pr) | Cl | CH₂OMe | O |
| C-8107 | H | CH(Me) | H | *c*-Pr | CH₂OC(=O)(*i*-Pr) | Cl | CH₂OMe | O |
| C-8108 | H | CH₂CH₂ | Me | *c*-Pr | CH₂OC(=O)(*i* -Pr) | Cl | CH₂OMe | O |
| C-8109 | Me | CH₂CH₂ | H | *c*-Pr | CH₂OC(=O)(*i* -Pr) | Cl | CH₂OMe | O |
| C-8110 | H | single bond | Me | *c*-Pr | CH₂OC(=O)(*i* -Pr) | Me | CH₂OMe | O |
| C-8111 | Me | single bond | H | *c*-Pr | CH₂OC(=O)(*i*-Pr) | Me | CH₂OMe | O |
| C-8112 | Me(R) | single bond | H | *c*-Pr | CH₂OC(=O)(*i* -Pr) | Me | CH₂OMe | O |
| C-8113 | Me(S) | single bond | H | *c*-Pr | CH₂OC(=O)(*i* -Pr) | Me | CH₂OMe | O |
| C-8114 | H | CH₂ | Me | *c*-Pr | CH₂OC(=O)(*i* -Pr) | Me | CH₂OMe | O |
| C-8115 | Me | CH₂ | H | *c*-Pr | CH₂OC(=O)(*i* -Pr) | Me | CH₂OMe | O |
| C-8116 | H | CH(Me) | H | *c*-Pr | CH₂OC(=O)(*i* -Pr) | Me | CH₂OMe | O |
| C-8117 | H | CH₂CH₂ | Me | *c*-Pr | CH₂OC(=O)(*i* -Pr) | Me | CH₂OMe | O |
| C-8118 | Me | CH₂CH₂ | H | *c*-Pr | CH₂OC(=O)(*i*-Pr) | Me | CH₂OMe | O |
| C-8119 | H | single bond | Me | *c*-Pr | CH₂OC(=O)(*t*-Bu) | Cl | CH₂OMe | O |
| C-8120 | Me | single bond | H | *c*-Pr | CH₂OC(=O)(*t*-Bu) | Cl | CH₂OMe | O |
| C-8121 | Me(R) | single bond | H | *c*-Pr | CH₂OC(=O)(*t*-Bu) | Cl | CH₂OMe | O |
| C-8122 | Me(S) | single bond | H | *c*-Pr | CH₂OC(=O)(*t*-Bu) | Cl | CH₂OMe | O |
| C-8123 | H | CH₂ | Me | *c*-Pr | CH₂OC(=O)(*t*-Bu) | Cl | CH₂OMe | O |
| C-8124 | Me | CH₂ | H | *c*-Pr | CH₂OC(=O)(*t*-Bu) | Cl | CH₂OMe | O |
| C-8125 | H | CH(Me) | H | *c*-Pr | CH₂OC(=O)(*t*-Bu) | Cl | CH₂OMe | O |
| C-8126 | H | CH₂CH₂ | Me | *c*-Pr | CH₂OC(=O)(*t*-Bu) | Cl | CH₂OMe | O |
| C-8127 | Me | CH₂CH₂ | H | *c*-Pr | CH₂OC(=O)(*t*-Bu) | Cl | CH₂OMe | O |
| C-8128 | H | single bond | Me | *c*-Pr | CH₂OC(=O)(*t*-Bu) | Me | CH₂OMe | O |
| C-8129 | Me | single bond | H | *c*-Pr | CH₂OC(=O)(*t*-Bu) | Me | CH₂OMe | O |
| C-8130 | Me(R) | single bond | H | *c*-Pr | CH₂OC(=O)(*t*-Bu) | Me | CH₂OMe | O |
| C-8131 | Me(S) | single bond | H | *c*-Pr | CH₂OC(=O)(*t*-Bu) | Me | CH₂OMe | O |
| C-8132 | H | CH₂ | Me | *c*-Pr | CH₂OC(=O)(*t*-Bu) | Me | CH₂OMe | O |
| C-8133 | Me | CH₂ | H | *c*-Pr | CH₂OC(=O)(*t*-Bu) | Me | CH₂OMe | O |
| C-8134 | H | CH(Me) | H | *c*-Pr | CH₂OC(=O)(*t*-Bu) | Me | CH₂OMe | O |
| C-8135 | H | CH₂CH₂ | Me | *c*-Pr | CH₂OC(=O)(*t*-Bu) | Me | CH₂OMe | O |
| C-8136 | Me | CH₂CH₂ | H | *c*-Pr | CH₂OC(=O)(*t*-Bu) | Me | CH₂OMe | O |
| C-8137 | H | single bond | Me | *c*-Pr | CH₂OC(=O)OEt | Cl | CH₂OMe | O |
| C-8138 | Me | single bond | H | *c*-Pr | CH₂OC(=O)OEt | Cl | CH₂OMe | O |
| C-8139 | Me(R) | single bond | H | *c*-Pr | CH₂OC(=O)OEt | Cl | CH₂OMe | O |
| C-8140 | Me(S) | single bond | H | *c*-Pr | CH₂OC(=O)OEt | Cl | CH₂OMe | O |
| C-8141 | H | CH₂ | Me | *c*-Pr | CH₂OC(=O)OEt | Cl | CH₂OMe | O |
| C-8142 | Me | CH₂ | H | *c*-Pr | CH₂OC(=O)OEt | Cl | CH₂OMe | O |
| C-8143 | H | CH(Me) | H | *c*-Pr | CH₂OC(=O)OEt | Cl | CH₂OMe | O |
| C-8144 | H | CH₂CH₂ | Me | *c*-Pr | CH₂OC(=O)OEt | Cl | CH₂OMe | O |
| C-8145 | Me | CH₂CH₂ | H | *c*-Pr | CH₂OC(=O)OEt | Cl | CH₂OMe | O |
| C-8146 | H | single bond | Me | *c*-Pr | CH₂OC(=O)OEt | Me | CH₂OMe | O |

**[Table 413]**

| Compound Number | Y | W | Z | R¹ | R² | R³ | R¹⁰ | A |
|---|---|---|---|---|---|---|---|---|
| C-8147 | Me | single bond | H | *c*-Pr | CH₂OC(=O)OEt | Me | CH₂OMe | O |
| C-8148 | Me(R) | single bond | H | *c*-Pr | CH₂OC(=O)OEt | Me | CH₂OMe | O |
| C-8149 | Me(S) | single bond | H | *c*-Pr | CH₂OC(=O)OEt | Me | CH₂OMe | O |
| C-8150 | H | CH₂ | Me | *c*-Pr | CH₂OC(=O)OEt | Me | CH₂OMe | O |
| C-8151 | Me | CH₂ | H | *c*-Pr | CH₂OC(=O)OEt | Me | CH₂OMe | O |
| C-8152 | H | CH(Me) | H | *c*-Pr | CH₂OC(=O)OEt | Me | CH₂OMe | O |
| C-8153 | H | CH₂CH₂ | Me | *c*-Pr | CH₂OC(=O)OEt | Me | CH₂OMe | O |
| C-8154 | Me | CH₂CH₂ | H | *c*-Pr | CH₂OC(=O)OEt | Me | CH₂OMe | O |
| C-8155 | H | single bond | Me | *c*-Pr | CH₂OC(=O)O(*i*-Pr) | Cl | CH₂OMe | O |
| C-8156 | Me | single bond | H | *c*-Pr | CH₂OC(=O)O(*i*-Pr) | Cl | CH₂OMe | O |
| C-8157 | Me(R) | single bond | H | *c*-Pr | CH₂OC(=O)O(*i*-Pr) | Cl | CH₂OMe | O |
| C-8158 | Me(S) | single bond | H | *c*-Pr | CH₂OC(=O)O(*i*-Pr) | Cl | CH₂OMe | O |
| C-8159 | H | CH₂ | Me | *c*-Pr | CH₂OC(=O)O(*i*-Pr) | Cl | CH₂OMe | O |
| C-8160 | Me | CH₂ | H | *c*-Pr | CH₂OC(=O)O(*i*-Pr) | Cl | CH₂OMe | O |
| C-8161 | H | CH(Me) | H | *c*-Pr | CH₂OC(=O)O(*i*-Pr) | Cl | CH₂OMe | O |
| C-8162 | H | CH₂CH₂ | Me | *c*-Pr | CH₂OC(=O)O(*i*-Pr) | Cl | CH₂OMe | O |
| C-8163 | Me | CH₂CH₂ | H | *c*-Pr | CH₂OC(=O)O(*i*-Pr) | Cl | CH₂OMe | O |
| C-8164 | H | single bond | Me | *c*-Pr | CH₂OC(=O)O(*i*-Pr) | Me | CH₂OMe | O |
| C-8165 | Me | single bond | H | *c*-Pr | CH₂OC(=O)O(*i*-Pr) | Me | CH₂OMe | O |
| C-8166 | Me(R) | single bond | H | *c*-Pr | CH₂OC(=O)O(*i*-Pr) | Me | CH₂OMe | O |
| C-8167 | Me(S) | single bond | H | *c*-Pr | CH₂OC(=O)O(*i*-Pr) | Me | CH₂OMe | O |
| C-8168 | H | CH₂ | Me | *c*-Pr | CH₂OC(=O)O(*i*-Pr) | Me | CH₂OMe | O |
| C-8169 | Me | CH₂ | H | *c*-Pr | CH₂OC(=O)O(*i*-Pr) | Me | CH₂OMe | O |
| C-8170 | H | CH(Me) | H | *c*-Pr | CH₂OC(=O)O(*i*-Pr) | Me | CH₂OMe | O |
| C-8171 | H | CH₂CH₂ | Me | *c*-Pr | CH₂OC(=O)O(*i*-Pr) | Me | CH₂OMe | O |
| C-8172 | Me | CH₂CH₂ | H | *c*-Pr | CH₂OC(=O)O(*i*-Pr) | Me | CH₂OMe | O |
| C-8173 | H | single bond | Me | *c*-Pr | CH₂N(Me)C(=O)OMe | Cl | CH₂OMe | O |
| C-8174 | Me | single bond | H | *c*-Pr | CH₂N(Me)C(=O)OMe | Cl | CH₂OMe | O |
| C-8175 | Me(R) | single bond | H | *c*-Pr | CH₂N(Me)C(=O)OMe | Cl | CH₂OMe | O |
| C-8176 | Me(S) | single bond | H | *c*-Pr | CH₂N(Me)C(=O)OMe | Cl | CH₂OMe | O |
| C-8177 | H | CH₂ | Me | *c*-Pr | CH₂N(Me)C(=O)OMe | Cl | CH₂OMe | O |
| C-8178 | Me | CH₂ | H | *c*-Pr | CH₂N(Me)C(=O)OMe | Cl | CH₂OMe | O |
| C-8179 | H | CH(Me) | H | *c*-Pr | CH₂N(Me)C(=O)OMe | Cl | CH₂OMe | O |
| C-8180 | H | CH₂CH₂ | Me | *c*-Pr | CH₂N(Me)C(=O)OMe | Cl | CH₂OMe | O |
| C-8181 | Me | CH₂CH₂ | H | *c*-Pr | CH₂N(Me)C(=O)OMe | Cl | CH₂OMe | O |
| C-8182 | H | single bond | Me | *c*-Pr | CH₂N(Me)C(=O)OMe | Me | CH₂OMe | O |
| C-8183 | Me | single bond | H | *c*-Pr | CH₂N(Me)C(=O)OMe | Me | CH₂OMe | O |
| C-8184 | Me(R) | single bond | H | *c*-Pr | CH₂N(Me)C(=O)OMe | Me | CH₂OMe | O |
| C-8185 | Me(S) | single bond | H | *c*-Pr | CH₂N(Me)C(=O)OMe | Me | CH₂OMe | O |
| C-8186 | H | CH₂ | Me | *c*-Pr | CH₂N(Me)C(=O)OMe | Me | CH₂OMe | O |
| C-8187 | Me | CH₂ | H | *c*-Pr | CH₂N(Me)C(=O)OMe | Me | CH₂OMe | O |
| C-8188 | H | CH(Me) | H | *c*-Pr | CH₂N(Me)C(=O)OMe | Me | CH₂OMe | O |
| C-8189 | H | CH₂CH₂ | Me | *c*-Pr | CH₂N(Me)C(=O)OMe | Me | CH₂OMe | O |
| C-8190 | Me | CH₂CH₂ | H | *c*-Pr | CH₂N(Me)C(=O)OMe | Me | CH₂OMe | O |

**[Table 414]**

| Compound Number | Y | W | Z | R¹ | R² | R³ | R¹⁰ | A |
|---|---|---|---|---|---|---|---|---|
| C-8191 | H | single bond | Me | *c*-Pr | C(=O)OCH₂CH=CH₂ | Cl | CH₂CN | O |
| C-8192 | Me | single bond | H | *c*-Pr | C(=O)OCH₂CH=CH₂ | Cl | CH₂CN | O |
| C-8193 | Me(R) | single bond | H | *c*-Pr | C(=O)OCH₂CH=CH₂ | Cl | CH₂CN | O |
| C-8194 | Me(S) | single bond | H | *c*-Pr | C(=O)OCH₂CH=CH₂ | Cl | CH₂CN | O |
| C-8195 | H | CH₂ | Me | *c*-Pr | C(=O)OCH₂CH=CH₂ | Cl | CH₂CN | O |
| C-8196 | Me | CH₂ | H | *c*-Pr | C(=O)OCH₂CH=CH₂ | Cl | CH₂CN | O |
| C-8197 | H | CH(Me) | H | *c*-Pr | C(=O)OCH₂CH=CH₂ | Cl | CH₂CN | O |
| C-8198 | H | CH₂CH₂ | Me | *c*-Pr | C(=O)OCH₂CH=CH₂ | Cl | CH₂CN | O |
| C-8199 | Me | CH₂CH₂ | H | *c*-Pr | C(=O)OCH₂CH=CH₂ | Cl | CH₂CN | O |
| C-8200 | H | single bond | Me | *c*-Pr | C(=O)OCH₂CH=CH₂ | Me | CH₂CN | O |
| C-8201 | Me | single bond | H | *c*-Pr | C(=O)OCH₂CH=CH₂ | Me | CH₂CN | O |
| C-8202 | Me(R) | single bond | H | *c*-Pr | C(=O)OCH₂CH=CH₂ | Me | CH₂CN | O |
| C-8203 | Me(S) | single bond | H | *c*-Pr | C(=O)OCH₂CH=CH₂ | Me | CH₂CN | O |
| C-8204 | H | CH₂ | Me | *c*-Pr | C(=O)OCH₂CH=CH₂ | Me | CH₂CN | O |
| C-8205 | Me | CH₂ | H | *c*-Pr | C(=O)OCH₂CH=CH₂ | Me | CH₂CN | O |
| C-8206 | H | CH(Me) | H | *c*-Pr | C(=O)OCH₂CH=CH₂ | Me | CH₂CN | O |
| C-8207 | H | CH₂CH₂ | Me | *c*-Pr | C(=O)OCH₂CH=CH₂ | Me | CH₂CN | O |
| C-8208 | Me | CH₂CH₂ | H | *c*-Pr | C(=O)OCH₂CH=CH₂ | Me | CH₂CN | O |
| C-8209 | H | single bond | Me | *c*-Pr | C(=O)OCH₂CCl₃ | Cl | CH₂CN | O |
| C-8210 | Me | single bond | H | *c*-Pr | C(=O)OCH₂CCl₃ | Cl | CH₂CN | O |
| C-8211 | Me(R) | single bond | H | *c*-Pr | C(=O)OCH₂CCl₃ | Cl | CH₂CN | O |
| C-8212 | Me(S) | single bond | H | *c*-Pr | C(=O)OCH₂CCl₃ | Cl | CH₂CN | O |
| C-8213 | H | CH₂ | Me | *c*-Pr | C(=O)OCH₂CCl₃ | Cl | CH₂CN | O |
| C-8214 | Me | CH₂ | H | *c*-Pr | C(=O)OCH₂CCl₃ | Cl | CH₂CN | O |
| C-8215 | H | CH(Me) | H | *c*-Pr | C(=O)OCH₂CCl₃ | Cl | CH₂CN | O |
| C-8216 | H | CH₂CH₂ | Me | *c*-Pr | C(=O)OCH₂CCl₃ | Cl | CH₂CN | O |
| C-8217 | Me | CH₂CH₂ | H | *c*-Pr | C(=O)OCH₂CCl₃ | Cl | CH₂CN | O |
| C-8218 | H | single bond | Me | *c*-Pr | C(=O)OCH₂CCl₃ | Me | CH₂CN | O |
| C-8219 | Me | single bond | H | *c*-Pr | C(=O)OCH₂CCl₃ | Me | CH₂CN | O |
| C-8220 | Me(R) | single bond | H | *c*-Pr | C(=O)OCH₂CCl₃ | Me | CH₂CN | O |
| C-8221 | Me(S) | single bond | H | *c*-Pr | C(=O)OCH₂CCl₃ | Me | CH₂CN | O |
| C-8222 | H | CH₂ | Me | *c*-Pr | C(=O)OCH₂CCl₃ | Me | CH₂CN | O |
| C-8223 | Me | CH₂ | H | *c*-Pr | C(=O)OCH₂CCl₃ | Me | CH₂CN | O |
| C-8224 | H | CH(Me) | H | *c*-Pr | C(=O)OCH₂CCl₃ | Me | CH₂CN | O |
| C-8225 | H | CH₂CH₂ | Me | *c*-Pr | C(=O)OCH₂CCl₃ | Me | CH₂CN | O |
| C-8226 | Me | CH₂CH₂ | H | *c*-Pr | C(=O)OCH₂CCl₃ | Me | CH₂CN | O |
| C-8227 | H | single bond | Me | *c*-Pr | C(=O)N(Me)₂ | Cl | CH₂CN | O |
| C-8228 | Me | single bond | H | *c*-Pr | C(=O)N(Me)₂ | Cl | CH₂CN | O |
| C-8229 | Me(R) | single bond | H | *c*-Pr | C(=O)N(Me)₂ | Cl | CH₂CN | O |
| C-8230 | Me(S) | single bond | H | *c*-Pr | C(=O)N(Me)₂ | Cl | CH₂CN | O |
| C-8231 | H | CH₂ | Me | *c*-Pr | C(=O)N(Me)₂ | Cl | CH₂CN | O |
| C-8232 | Me | CH₂ | H | *c*-Pr | C(=O)N(Me)₂ | Cl | CH₂CN | O |
| C-8233 | H | CH(Me) | H | *c*-Pr | C(=O)N(Me)₂ | Cl | CH₂CN | O |
| C-8234 | H | CH₂CH₂ | Me | *c*-Pr | C(=O)N(Me)₂ | Cl | CH₂CN | O |

**[Table 415]**

| Compound Number | Y | W | Z | R¹ | R² | R³ | R¹⁰ | A |
|---|---|---|---|---|---|---|---|---|
| C-8235 | Me | CH₂CH₂ | H | *c*-Pr | C(=O)N(Me)₂ | Cl | CH₂CN | O |
| C-8236 | H | single bond | Me | *c*-Pr | C(=O)N(Me)₂ | Me | CH₂CN | O |
| C-8237 | Me | single bond | H | *c*-Pr | C(=O)N(Me)₂ | Me | CH₂CN | O |
| C-8238 | Me(R) | single bond | H | *c*-Pr | C(=O)N(Me)₂ | Me | CH₂CN | O |
| C-8239 | Me(S) | single bond | H | *c*-Pr | C(=O)N(Me)₂ | Me | CH₂CN | O |
| C-8240 | H | CH₂ | Me | *c*-Pr | C(=O)N(Me)₂ | Me | CH₂CN | O |
| C-8241 | Me | CH₂ | H | *c*-Pr | C(=O)N(Me)₂ | Me | CH₂CN | O |
| C-8242 | H | CH(Me) | H | *c*-Pr | C(=O)N(Me)₂ | Me | CH₂CN | O |
| C-8243 | H | CH₂CH₂ | Me | *c*-Pr | C(=O)N(Me)₂ | Me | CH₂CN | O |
| C-8244 | Me | CH₂CH₂ | H | *c*-Pr | C(=O)N(Me)₂ | Me | CH₂CN | O |
| C-8245 | H | single bond | Me | *c*-Pr | CH₂OC(=O)Me | Cl | CH₂CN | O |
| C-8246 | Me | single bond | H | *c*-Pr | CH₂OC(=O)Me | Cl | CH₂CN | O |
| C-8247 | Me(R) | single bond | H | *c*-Pr | CH₂OC(=O)Me | Cl | CH₂CN | O |
| C-8248 | Me(S) | single bond | H | *c*-Pr | CH₂OC(=O)Me | Cl | CH₂CN | O |
| C-8249 | H | CH₂ | Me | *c*-Pr | CH₂OC(=O)Me | Cl | CH₂CN | O |
| C-8250 | Me | CH₂ | H | *c*-Pr | CH₂OC(=O)Me | Cl | CH₂CN | O |
| C-8251 | H | CH(Me) | H | *c*-Pr | CH₂OC(=O)Me | Cl | CH₂CN | O |
| C-8252 | H | CH₂CH₂ | Me | *c*-Pr | CH₂OC(=O)Me | Cl | CH₂CN | O |
| C-8253 | Me | CH₂CH₂ | H | *c*-Pr | CH₂OC(=O)Me | Cl | CH₂CN | O |
| C-8254 | H | single bond | Me | *c*-Pr | CH₂OC(=O)Me | Me | CH₂CN | O |
| C-8255 | Me | single bond | H | *c*-Pr | CH₂OC(=O)Me | Me | CH₂CN | O |
| C-8256 | Me(R) | single bond | H | *c*-Pr | CH₂OC(=O)Me | Me | CH₂CN | O |
| C-8257 | Me(S) | single bond | H | *c*-Pr | CH₂OC(=O)Me | Me | CH₂CN | O |
| C-8258 | H | CH₂ | Me | *c*-Pr | CH₂OC(=O)Me | Me | CH₂CN | O |
| C-8259 | Me | CH₂ | H | *c*-Pr | CH₂OC(=O)Me | Me | CH₂CN | O |
| C-8260 | H | CH(Me) | H | *c*-Pr | CH₂OC(=O)Me | Me | CH₂CN | O |
| C-8261 | H | CH₂CH₂ | Me | *c*-Pr | CH₂OC(=O)Me | Me | CH₂CN | O |
| C-8262 | Me | CH₂CH₂ | H | *c*-Pr | CH₂OC(=O)Me | Me | CH₂CN | O |
| C-8263 | H | single bond | Me | *c*-Pr | CH₂OC(=O)(*i*-Pr) | Cl | CH₂CN | O |
| C-8264 | Me | single bond | H | *c*-Pr | CH₂OC(=O)(*i*-Pr) | Cl | CH₂CN | O |
| C-8265 | Me(R) | single bond | H | *c*-Pr | CH₂OC(=O)(*i*-Pr) | Cl | CH₂CN | O |
| C-8266 | Me(S) | single bond | H | *c*-Pr | CH₂OC(=O)(*i*-Pr) | Cl | CH₂CN | O |
| C-8267 | H | CH₂ | Me | *c*-Pr | CH₂OC(=O)(*i*-Pr) | Cl | CH₂CN | O |
| C-8268 | Me | CH₂ | H | *c*-Pr | CH₂OC(=O)(*i*-Pr) | Cl | CH₂CN | O |
| C-8269 | H | CH(Me) | H | *c*-Pr | CH₂OC(=O)(*i*-Pr) | Cl | CH₂CN | O |
| C-8270 | H | CH₂CH₂ | Me | *c*-Pr | CH₂OC(=O)(*i*-Pr) | Cl | CH₂CN | O |
| C-8271 | Me | CH₂CH₂ | H | *c*-Pr | CH₂OC(=O)(*i*-Pr) | Cl | CH₂CN | O |
| C-8272 | H | single bond | Me | *c*-Pr | CH₂OC(=O)(*i*-Pr) | Me | CH₂CN | O |
| C-8273 | Me | single bond | H | *c*-Pr | CH₂OC(=O)(*i*-Pr) | Me | CH₂CN | O |
| C-8274 | Me(R) | single bond | H | *c*-Pr | CH₂OC(=O)(*i*-Pr) | Me | CH₂CN | O |
| C-8275 | Me(S) | single bond | H | *c*-Pr | CH₂OC(=O)(*i*-Pr) | Me | CH₂CN | O |
| C-8276 | H | CH₂ | Me | *c*-Pr | CH₂OC(=O)(*i*-Pr) | Me | CH₂CN | O |
| C-8277 | Me | CH₂ | H | *c*-Pr | CH₂OC(=O)(*i*-Pr) | Me | CH₂CN | O |
| C-8278 | H | CH(Me) | H | *c*-Pr | CH₂OC(=O)(*i*-Pr) | Me | CH₂CN | O |

**[Table 416]**

| Compound Number | Y | W | Z | R¹ | R² | R³ | R¹⁰ | A |
|---|---|---|---|---|---|---|---|---|
| C-8279 | H | CH₂CH₂ | Me | *c*-Pr | CH₂OC(=O)(*i*-Pr) | Me | CH₂CN | O |
| C-8280 | Me | CH₂CH₂ | H | *c*-Pr | CH₂OC(=O)(*i*-Pr) | Me | CH₂CN | O |
| C-8281 | H | single bond | Me | *c*-Pr | CH₂OC(=O)(*t*-Bu) | Cl | CH₂CN | O |
| C-8282 | Me | single bond | H | *c*-Pr | CH₂OC(=O)(*t*-Bu) | Cl | CH₂CN | O |
| C-8283 | Me(R) | single bond | H | *c*-Pr | CH₂OC(=O)(*t*-Bu) | Cl | CH₂CN | O |
| C-8284 | Me(S) | single bond | H | *c*-Pr | CH₂OC(=O)(*t*-Bu) | Cl | CH₂CN | O |
| C-8285 | H | CH₂ | Me | *c*-Pr | CH₂OC(=O)(*t*-Bu) | Cl | CH₂CN | O |
| C-8286 | Me | CH₂ | H | *c*-Pr | CH₂OC(=O)(*t*-Bu) | Cl | CH₂CN | O |
| C-8287 | H | CH(Me) | H | *c*-Pr | CH₂OC(=O)(*t*-Bu) | Cl | CH₂CN | O |
| C-8288 | H | CH₂CH₂ | Me | *c*-Pr | CH₂OC(=O)(*t*-Bu) | Cl | CH₂CN | O |
| C-8289 | Me | CH₂CH₂ | H | *c*-Pr | CH₂OC(=O)(*t*-Bu) | Cl | CH₂CN | O |
| C-8290 | H | single bond | Me | *c*-Pr | CH₂OC(=O)(*t*-Bu) | Me | CH₂CN | O |
| C-8291 | Me | single bond | H | *c*-Pr | CH₂OC(=O)(*t*-Bu) | Me | CH₂CN | O |
| C-8292 | Me(R) | single bond | H | *c*-Pr | CH₂OC(=O)(*t*-Bu) | Me | CH₂CN | O |
| C-8293 | Me(S) | single bond | H | *c*-Pr | CH₂OC(=O)(*t*-Bu) | Me | CH₂CN | O |
| C-8294 | H | CH₂ | Me | *c*-Pr | CH₂OC(=O)(*t*-Bu) | Me | CH₂CN | O |
| C-8295 | Me | CH₂ | H | *c*-Pr | CH₂OC(=O)(*t*-Bu) | Me | CH₂CN | O |
| C-8296 | H | CH(Me) | H | *c*-Pr | CH₂OC(=O)(*t*-Bu) | Me | CH₂CN | O |
| C-8297 | H | CH₂CH₂ | Me | *c*-Pr | CH₂OC(=O)(*t*-Bu) | Me | CH₂CN | O |
| C-8298 | Me | CH₂CH₂ | H | *c*-Pr | CH₂OC(=O)(*t*-Bu) | Me | CH₂CN | O |
| C-8299 | H | single bond | Me | *c*-Pr | CH₂OC(=O)OEt | Cl | CH₂CN | O |
| C-8300 | Me | single bond | H | *c*-Pr | CH₂OC(=O)OEt | Cl | CH₂CN | O |
| C-8301 | Me(R) | single bond | H | *c*-Pr | CH₂OC(=O)OEt | Cl | CH₂CN | O |
| C-8302 | Me(S) | single bond | H | *c*-Pr | CH₂OC(=O)OEt | Cl | CH₂CN | O |
| C-8303 | H | CH₂ | Me | *c*-Pr | CH₂OC(=O)OEt | Cl | CH₂CN | O |
| C-8304 | Me | CH₂ | H | *c*-Pr | CH₂OC(=O)OEt | Cl | CH₂CN | O |
| C-8305 | H | CH(Me) | H | *c*-Pr | CH₂OC(=O)OEt | Cl | CH₂CN | O |
| C-8306 | H | CH₂CH₂ | Me | *c*-Pr | CH₂OC(=O)OEt | Cl | CH₂CN | O |
| C-8307 | Me | CH₂CH₂ | H | *c*-Pr | CH₂OC(=O)OEt | Cl | CH₂CN | O |
| C-8308 | H | single bond | Me | *c*-Pr | CH₂OC(=O)OEt | Me | CH₂CN | O |
| C-8309 | Me | single bond | H | *c*-Pr | CH₂OC(=O)OEt | Me | CH₂CN | O |
| C-8310 | Me(R) | single bond | H | *c*-Pr | CH₂OC(=O)OEt | Me | CH₂CN | O |
| C-8311 | Me(S) | single bond | H | *c*-Pr | CH₂OC(=O)OEt | Me | CH₂CN | O |
| C-8312 | H | CH₂ | Me | *c*-Pr | CH₂OC(=O)OEt | Me | CH₂CN | O |
| C-8313 | Me | CH₂ | H | *c*-Pr | CH₂OC(=O)OEt | Me | CH₂CN | O |
| C-8314 | H | CH(Me) | H | *c*-Pr | CH₂OC(=O)OEt | Me | CH₂CN | O |
| C-8315 | H | CH₂CH₂ | Me | *c*-Pr | CH₂OC(=O)OEt | Me | CH₂CN | O |
| C-8316 | Me | CH₂CH₂ | H | *c*-Pr | CH₂OC(=O)OEt | Me | CH₂CN | O |
| C-8317 | H | single bond | Me | *c*-Pr | CH₂OC(=O)O(*i*-Pr) | Cl | CH₂CN | O |
| C-8318 | Me | single bond | H | *c*-Pr | CH₂OC(=O)O(*i*-Pr) | Cl | CH₂CN | O |
| C-8319 | Me(R) | single bond | H | *c*-Pr | CH₂OC(=O)O(*i*-Pr) | Cl | CH₂CN | O |
| C-8320 | Me(S) | single bond | H | *c*-Pr | CH₂OC(=O)O(*i*-Pr) | Cl | CH₂CN | O |
| C-8321 | H | CH₂ | Me | *c*-Pr | CH₂OC(=O)O(*i*-Pr) | Cl | CH₂CN | O |
| C-8322 | Me | CH₂ | H | *c*-Pr | CH₂OC(=O)O(*i*-Pr) | Cl | CH₂CN | O |

**[Table 417]**

| Compound Number | Y | W | Z | R¹ | R² | R³ | R¹⁰ | A |
|---|---|---|---|---|---|---|---|---|
| C-8323 | H | CH(Me) | H | *c*-Pr | CH₂OC(=O)O(*i*-Pr) | Cl | CH₂CN | O |
| C-8324 | H | CH₂CH₂ | Me | *c*-Pr | CH₂OC(=O)O(*i*-Pr) | Cl | CH₂CN | O |
| C-8325 | Me | CH₂CH₂ | H | *c*-Pr | CH₂OC(=O)O(*i*-Pr) | Cl | CH₂CN | O |
| C-8326 | H | single bond | Me | *c*-Pr | CH₂OC(=O)O(*i*-Pr) | Me | CH₂CN | O |
| C-8327 | Me | single bond | H | *c*-Pr | CH₂OC(=O)O(*i*-Pr) | Me | CH₂CN | O |
| C-8328 | Me(R) | single bond | H | *c*-Pr | CH₂OC(=O)O(*i*-Pr) | Me | CH₂CN | O |
| C-8329 | Me(S) | single bond | H | *c*-Pr | CH₂OC(=O)O(*i*-Pr) | Me | CH₂CN | O |
| C-8330 | H | CH₂ | Me | *c*-Pr | CH₂OC(=O)O(*i*-Pr) | Me | CH₂CN | O |
| C-8331 | Me | CH₂ | H | *c*-Pr | CH₂OC(=O)O(*i*-Pr) | Me | CH₂CN | O |
| C-8332 | H | CH(Me) | H | *c*-Pr | CH₂OC(=O)O(*i*-Pr) | Me | CH₂CN | O |
| C-8333 | H | CH₂CH₂ | Me | *c*-Pr | CH₂OC(=O)O(*i*-Pr) | Me | CH₂CN | O |
| C-8334 | Me | CH₂CH₂ | H | *c*-Pr | CH₂OC(=O)O(*i*-Pr) | Me | CH₂CN | O |
| C-8335 | H | single bond | Me | *c*-Pr | CH₂N(Me)C(=O)OMe | Cl | CH₂CN | O |
| C-8336 | Me | single bond | H | *c*-Pr | CH₂N(Me)C(=O)OMe | Cl | CH₂CN | O |
| C-8337 | Me(R) | single bond | H | *c*-Pr | CH₂N(Me)C(=O)OMe | Cl | CH₂CN | O |
| C-8338 | Me(S) | single bond | H | *c*-Pr | CH₂N(Me)C(=O)OMe | Cl | CH₂CN | O |
| C-8339 | H | CH₂ | Me | *c*-Pr | CH₂N(Me)C(=O)OMe | Cl | CH₂CN | O |
| C-8340 | Me | CH₂ | H | *c*-Pr | CH₂N(Me)C(=O)OMe | Cl | CH₂CN | O |
| C-8341 | H | CH(Me) | H | *c*-Pr | CH₂N(Me)C(=O)OMe | Cl | CH₂CN | O |
| C-8342 | H | CH₂CH₂ | Me | *c*-Pr | CH₂N(Me)C(=O)OMe | Cl | CH₂CN | O |
| C-8343 | Me | CH₂CH₂ | H | *c*-Pr | CH₂N(Me)C(=O)OMe | Cl | CH₂CN | O |
| C-8344 | H | single bond | Me | *c*-Pr | CH₂N(Me)C(=O)OMe | Me | CH₂CN | O |
| C-8345 | Me | single bond | H | *c*-Pr | CH₂N(Me)C(=O)OMe | Me | CH₂CN | O |
| C-8346 | Me(R) | single bond | H | *c*-Pr | CH₂N(Me)C(=O)OMe | Me | CH₂CN | O |
| C-8347 | Me(S) | single bond | H | *c*-Pr | CH₂N(Me)C(=O)OMe | Me | CH₂CN | O |
| C-8348 | H | CH₂ | Me | *c*-Pr | CH₂N(Me)C(=O)OMe | Me | CH₂CN | O |
| C-8349 | Me | CH₂ | H | *c*-Pr | CH₂N(Me)C(=O)OMe | Me | CH₂CN | O |
| C-8350 | H | CH(Me) | H | *c*-Pr | CH₂N(Me)C(=O)OMe | Me | CH₂CN | O |
| C-8351 | H | CH₂CH₂ | Me | *c*-Pr | CH₂N(Me)C(=O)OMe | Me | CH₂CN | O |
| C-8352 | Me | CH₂CH₂ | H | *c*-Pr | CH₂N(Me)C(=O)OMe | Me | CH₂CN | O |
| C-8353 | H | single bond | Me | *c*-Pr | C(=O)OCH₂CH=CH₂ | Cl | C(=O)Me | O |
| C-8354 | Me | single bond | H | *c*-Pr | C(=O)OCH₂CH=CH₂ | Cl | C(=O)Me | O |
| C-8355 | Me(R) | single bond | H | *c*-Pr | C(=O)OCH₂CH=CH₂ | Cl | C(=O)Me | O |
| C-8356 | Me(S) | single bond | H | *c*-Pr | C(=O)OCH₂CH=CH₂ | Cl | C(=O)Me | O |
| C-8357 | H | CH₂ | Me | *c*-Pr | C(=O)OCH₂CH=CH₂ | Cl | C(=O)Me | O |
| C-8358 | Me | CH₂ | H | *c*-Pr | C(=O)OCH₂CH=CH₂ | Cl | C(=O)Me | O |
| C-8359 | H | CH(Me) | H | *c*-Pr | C(=O)OCH₂CH=CH₂ | Cl | C(=O)Me | O |
| C-8360 | H | CH₂CH₂ | Me | *c*-Pr | C(=O)OCH₂CH=CH₂ | Cl | C(=O)Me | O |
| C-8361 | Me | CH₂CH₂ | H | *c*-Pr | C(=O)OCH₂CH=CH₂ | Cl | C(=O)Me | O |
| C-8362 | H | single bond | Me | *c*-Pr | C(=O)OCH₂CH=CH₂ | Me | C(=O)Me | O |
| C-8363 | Me | single bond | H | *c*-Pr | C(=O)OCH₂CH=CH₂ | Me | C(=O)Me | O |
| C-8364 | Me(R) | single bond | H | *c*-Pr | C(=O)OCH₂CH=CH₂ | Me | C(=O)Me | O |
| C-8365 | Me(S) | single bond | H | *c*-Pr | C(=O)OCH₂CH=CH₂ | Me | C(=O)Me | O |
| C-8366 | H | CH₂ | Me | *c*-Pr | C(=O)OCH₂CH=CH₂ | Me | C(=O)Me | O |

**[Table 418]**

| Compound Number | Y | W | Z | R¹ | R² | R³ | R¹⁰ | A |
|---|---|---|---|---|---|---|---|---|
| C-8367 | Me | CH₂ | H | *c*-Pr | C(=O)OCH₂CH=CH₂ | Me | C(=O)Me | O |
| C-8368 | H | CH(Me) | H | *c*-Pr | C(=O)OCH₂CH=CH₂ | Me | C(=O)Me | O |
| C-8369 | H | CH₂CH₂ | Me | *c*-Pr | C(=O)OCH₂CH=CH₂ | Me | C(=O)Me | O |
| C-8370 | Me | CH₂CH₂ | H | *c*-Pr | C(=O)OCH₂CH=CH₂ | Me | C(=O)Me | O |
| C-8371 | H | single bond | Me | *c*-Pr | C(=O)OCH₂CCl₃ | Cl | C(=O)Me | O |
| C-8372 | Me | single bond | H | *c*-Pr | C(=O)OCH₂CCl₃ | Cl | C(=O)Me | O |
| C-8373 | Me(R) | single bond | H | *c*-Pr | C(=O)OCH₂CCl₃ | Cl | C(=O)Me | O |
| C-8374 | Me(S) | single bond | H | *c*-Pr | C(=O)OCH₂CCl₃ | Cl | C(=O)Me | O |
| C-8375 | H | CH₂ | Me | *c*-Pr | C(=O)OCH₂CCl₃ | Cl | C(=O)Me | O |
| C-8376 | Me | CH₂ | H | *c*-Pr | C(=O)OCH₂CCl₃ | Cl | C(=O)Me | O |
| C-8377 | H | CH(Me) | H | *c*-Pr | C(=O)OCH₂CCl₃ | Cl | C(=O)Me | O |
| C-8378 | H | CH₂CH₂ | Me | *c*-Pr | C(=O)OCH₂CCl₃ | Cl | C(=O)Me | O |
| C-8379 | Me | CH₂CH₂ | H | *c*-Pr | C(=O)OCH₂CCl₃ | Cl | C(=O)Me | O |
| C-8380 | H | single bond | Me | *c*-Pr | C(=O)OCH₂CCl₃ | Me | C(=O)Me | O |
| C-8381 | Me | single bond | H | *c*-Pr | C(=O)OCH₂CCl₃ | Me | C(=O)Me | O |
| C-8382 | Me(R) | single bond | H | *c*-Pr | C(=O)OCH₂CCl₃ | Me | C(=O)Me | O |
| C-8383 | Me(S) | single bond | H | *c*-Pr | C(=O)OCH₂CCl₃ | Me | C(=O)Me | O |
| C-8384 | H | CH₂ | Me | *c*-Pr | C(=O)OCH₂CCl₃ | Me | C(=O)Me | O |
| C-8385 | Me | CH₂ | H | *c*-Pr | C(=O)OCH₂CCl₃ | Me | C(=O)Me | O |
| C-8386 | H | CH(Me) | H | *c*-Pr | C(=O)OCH₂CCl₃ | Me | C(=O)Me | O |
| C-8387 | H | CH₂CH₂ | Me | *c*-Pr | C(=O)OCH₂CCl₃ | Me | C(=O)Me | O |
| C-8388 | Me | CH₂CH₂ | H | *c*-Pr | C(=O)OCH₂CCl₃ | Me | C(=O)Me | O |
| C-8389 | H | single bond | Me | *c*-Pr | C(=O)N(Me)₂ | Cl | C(=O)Me | O |
| C-8390 | Me | single bond | H | *c*-Pr | C(=O)N(Me)₂ | Cl | C(=O)Me | O |
| C-8391 | Me(R) | single bond | H | *c*-Pr | C(=O)N(Me)₂ | Cl | C(=O)Me | O |
| C-8392 | Me(S) | single bond | H | *c*-Pr | C(=O)N(Me)₂ | Cl | C(=O)Me | O |
| C-8393 | H | CH₂ | Me | *c*-Pr | C(=O)N(Me)₂ | Cl | C(=O)Me | O |
| C-8394 | Me | CH₂ | H | *c*-Pr | C(=O)N(Me)₂ | Cl | C(=O)Me | O |
| C-8395 | H | CH(Me) | H | *c*-Pr | C(=O)N(Me)₂ | Cl | C(=O)Me | O |
| C-8396 | H | CH₂CH₂ | Me | *c*-Pr | C(=O)N(Me)₂ | Cl | C(=O)Me | O |
| C-8397 | Me | CH₂CH₂ | H | *c*-Pr | C(=O)N(Me)₂ | Cl | C(=O)Me | O |
| C-8398 | H | single bond | Me | *c*-Pr | C(=O)N(Me)₂ | Me | C(=O)Me | O |
| C-8399 | Me | single bond | H | *c*-Pr | C(=O)N(Me)₂ | Me | C(=O)Me | O |
| C-8400 | Me(R) | single bond | H | *c*-Pr | C(=O)N(Me)₂ | Me | C(=O)Me | O |
| C-8401 | Me(S) | single bond | H | *c*-Pr | C(=O)N(Me)₂ | Me | C(=O)Me | O |
| C-8402 | H | CH₂ | Me | *c*-Pr | C(=O)N(Me)₂ | Me | C(=O)Me | O |
| C-8403 | Me | CH₂ | H | *c*-Pr | C(=O)N(Me)₂ | Me | C(=O)Me | O |
| C-8404 | H | CH(Me) | H | *c*-Pr | C(=O)N(Me)₂ | Me | C(=O)Me | O |
| C-8405 | H | CH₂CH₂ | Me | *c*-Pr | C(=O)N(Me)₂ | Me | C(=O)Me | O |
| C-8406 | Me | CH₂CH₂ | H | *c*-Pr | C(=O)N(Me)₂ | Me | C(=O)Me | O |
| C-8407 | H | single bond | Me | *c*-Pr | CH₂OC(=O)Me | Cl | C(=O)Me | O |
| C-8408 | Me | single bond | H | *c*-Pr | CH₂OC(=O)Me | Cl | C(=O)Me | O |
| C-8409 | Me(R) | single bond | H | *c*-Pr | CH₂OC(=O)Me | Cl | C(=O)Me | O |
| C-8410 | Me(S) | single bond | H | *c*-Pr | CH₂OC(=O)Me | Cl | C(=O)Me | O |

**[Table 419]**

| Compound Number | Y | W | Z | R¹ | R² | R³ | R¹⁰ | A |
|---|---|---|---|---|---|---|---|---|
| C-8411 | H | CH₂ | Me | *c*-Pr | CH₂OC(=O)Me | Cl | C(=O)Me | O |
| C-8412 | Me | CH₂ | H | *c*-Pr | CH₂OC(=O)Me | Cl | C(=O)Me | O |
| C-8413 | H | CH(Me) | H | *c*-Pr | CH₂OC(=O)Me | Cl | C(=O)Me | O |
| C-8414 | H | CH₂CH₂ | Me | *c*-Pr | CH₂OC(=O)Me | Cl | C(=O)Me | O |
| C-8415 | Me | CH₂CH₂ | H | *c*-Pr | CH₂OC(=O)Me | Cl | C(=O)Me | O |
| C-8416 | H | single bond | Me | *c*-Pr | CH₂OC(=O)Me | Me | C(=O)Me | O |
| C-8417 | Me | single bond | H | *c*-Pr | CH₂OC(=O)Me | Me | C(=O)Me | O |
| C-8418 | Me(R) | single bond | H | *c*-Pr | CH₂OC(=O)Me | Me | C(=O)Me | O |
| C-8419 | Me(S) | single bond | H | *c*-Pr | CH₂OC(=O)Me | Me | C(=O)Me | O |
| C-8420 | H | CH₂ | Me | *c*-Pr | CH₂OC(=O)Me | Me | C(=O)Me | O |
| C-8421 | Me | CH₂ | H | *c*-Pr | CH₂OC(=O)Me | Me | C(=O)Me | O |
| C-8422 | H | CH(Me) | H | *c*-Pr | CH₂OC(=O)Me | Me | C(=O)Me | O |
| C-8423 | H | CH₂CH₂ | Me | *c*-Pr | CH₂OC(=O)Me | Me | C(=O)Me | O |
| C-8424 | Me | CH₂CH₂ | H | *c*-Pr | CH₂OC(=O)Me | Me | C(=O)Me | O |
| C-8425 | H | single bond | Me | *c*-Pr | CH₂OC(=O)(*i*-Pr) | Cl | C(=O)Me | O |
| C-8426 | Me | single bond | H | *c*-Pr | CH₂OC(=O)(*i*-Pr) | Cl | C(=O)Me | O |
| C-8427 | Me(R) | single bond | H | *c*-Pr | CH₂OC(=O)(*i*-Pr) | Cl | C(=O)Me | O |
| C-8428 | Me(S) | single bond | H | *c*-Pr | CH₂OC(=O)(*i*-Pr) | Cl | C(=O)Me | O |
| C-8429 | H | CH₂ | Me | *c*-Pr | CH₂OC(=O)(*i*-Pr) | Cl | C(=O)Me | O |
| C-8430 | Me | CH₂ | H | *c*-Pr | CH₂OC(=O)(*i*-Pr) | Cl | C(=O)Me | O |
| C-8431 | H | CH(Me) | H | *c*-Pr | CH₂OC(=O)(*i*-Pr) | Cl | C(=O)Me | O |
| C-8432 | H | CH₂CH₂ | Me | *c*-Pr | CH₂OC(=O)(*i*-Pr) | Cl | C(=O)Me | O |
| C-8433 | Me | CH₂CH₂ | H | *c*-Pr | CH₂OC(=O)(*i*-Pr) | Cl | C(=O)Me | O |
| C-8434 | H | single bond | Me | *c*-Pr | CH₂OC(=O)(*i*-Pr) | Me | C(=O)Me | O |
| C-8435 | Me | single bond | H | *c*-Pr | CH₂OC(=O)(*i*-Pr) | Me | C(=O)Me | O |
| C-8436 | Me(R) | single bond | H | *c*-Pr | CH₂OC(=O)(*i*-Pr) | Me | C(=O)Me | O |
| C-8437 | Me(S) | single bond | H | *c*-Pr | CH₂OC(=O)(*i*-Pr) | Me | C(=O)Me | O |
| C-8438 | H | CH₂ | Me | *c*-Pr | CH₂OC(=O)(*i*-Pr) | Me | C(=O)Me | O |
| C-8439 | Me | CH₂ | H | *c*-Pr | CH₂OC(=O)(*i*-Pr) | Me | C(=O)Me | O |
| C-8440 | H | CH(Me) | H | *c*-Pr | CH₂OC(=O)(*i*-Pr) | Me | C(=O)Me | O |
| C-8441 | H | CH₂CH₂ | Me | *c*-Pr | CH₂OC(=O)(*i*-Pr) | Me | C(=O)Me | O |
| C-8442 | Me | CH₂CH₂ | H | *c*-Pr | CH₂OC(=O)(*i*-Pr) | Me | C(=O)Me | O |
| C-8443 | H | single bond | Me | *c*-Pr | CH₂OC(=O)(*t*-Bu) | Cl | C(=O)Me | O |
| C-8444 | Me | single bond | H | *c*-Pr | CH₂OC(=O)(*t*-Bu) | Cl | C(=O)Me | O |
| C-8445 | Me(R) | single bond | H | *c*-Pr | CH₂OC(=O)(*t*-Bu) | Cl | C(=O)Me | O |
| C-8446 | Me(S) | single bond | H | *c*-Pr | CH₂OC(=O)(*t*-Bu) | Cl | C(=O)Me | O |
| C-8447 | H | CH₂ | Me | *c*-Pr | CH₂OC(=O)(*t*-Bu) | Cl | C(=O)Me | O |
| C-8448 | Me | CH₂ | H | *c*-Pr | CH₂OC(=O)(*t*-Bu) | Cl | C(=O)Me | O |
| C-8449 | H | CH(Me) | H | *c*-Pr | CH₂OC(=O)(*t*-Bu) | Cl | C(=O)Me | O |
| C-8450 | H | CH₂CH₂ | Me | *c*-Pr | CH₂OC(=O)(*t*-Bu) | Cl | C(=O)Me | O |
| C-8451 | Me | CH₂CH₂ | H | *c*-Pr | CH₂OC(=O)(*t*-Bu) | Cl | C(=O)Me | O |
| C-8452 | H | single bond | Me | *c*-Pr | CH₂OC(=O)(*t*-Bu) | Me | C(=O)Me | O |
| C-8453 | Me | single bond | H | *c*-Pr | CH₂OC(=O)(*t*-Bu) | Me | C(=O)Me | O |
| C-8454 | Me(R) | single bond | H | *c*-Pr | CH₂OC(=O)(*t*-Bu) | Me | C(=O)Me | O |

**[Table 420]**

| Compound Number | Y | W | Z | R¹ | R² | R³ | R¹⁰ | A |
|---|---|---|---|---|---|---|---|---|
| C-8455 | Me(S) | single bond | H | *c*-Pr | CH₂OC(=O)(*t*-Bu) | Me | C(=O)Me | O |
| C-8456 | H | CH₂ | Me | *c*-Pr | CH₂OC(=O)(*t*-Bu) | Me | C(=O)Me | O |
| C-8457 | Me | CH₂ | H | *c*-Pr | CH₂OC(=O)(*t*-Bu) | Me | C(=O)Me | O |
| C-8458 | H | CH(Me) | H | *c*-Pr | CH₂OC(=O)(*t*-Bu) | Me | C(=O)Me | O |
| C-8459 | H | CH₂CH₂ | Me | *c*-Pr | CH₂OC(=O)(*t*-Bu) | Me | C(=O)Me | O |
| C-8460 | Me | CH₂CH₂ | H | *c*-Pr | CH₂OC(=O)(*t*-Bu) | Me | C(=O)Me | O |
| C-8461 | H | single bond | Me | *c*-Pr | CH₂OC(=O)OEt | Cl | C(=O)Me | O |
| C-8462 | Me | single bond | H | *c*-Pr | CH₂OC(=O)OEt | Cl | C(=O)Me | O |
| C-8463 | Me(R) | single bond | H | *c*-Pr | CH₂OC(=O)OEt | Cl | C(=O)Me | O |
| C-8464 | Me(S) | single bond | H | *c*-Pr | CH₂OC(=O)OEt | Cl | C(=O)Me | O |
| C-8465 | H | CH₂ | Me | *c*-Pr | CH₂OC(=O)OEt | Cl | C(=O)Me | O |
| C-8466 | Me | CH₂ | H | *c*-Pr | CH₂OC(=O)OEt | Cl | C(=O)Me | O |
| C-8467 | H | CH(Me) | H | *c*-Pr | CH₂OC(=O)OEt | Cl | C(=O)Me | O |
| C-8468 | H | CH₂CH₂ | Me | *c*-Pr | CH₂OC(=O)OEt | Cl | C(=O)Me | O |
| C-8469 | Me | CH₂CH₂ | H | *c*-Pr | CH₂OC(=O)OEt | Cl | C(=O)Me | O |
| C-8470 | H | single bond | Me | *c*-Pr | CH₂OC(=O)OEt | Me | C(=O)Me | O |
| C-8471 | Me | single bond | H | *c*-Pr | CH₂OC(=O)OEt | Me | C(=O)Me | O |
| C-8472 | Me(R) | single bond | H | *c*-Pr | CH₂OC(=O)OEt | Me | C(=O)Me | O |
| C-8473 | Me(S) | single bond | H | *c*-Pr | CH₂OC(=O)OEt | Me | C(=O)Me | O |
| C-8474 | H | CH₂ | Me | *c*-Pr | CH₂OC(=O)OEt | Me | C(=O)Me | O |
| C-8475 | Me | CH₂ | H | *c*-Pr | CH₂OC(=O)OEt | Me | C(=O)Me | O |
| C-8476 | H | CH(Me) | H | *c*-Pr | CH₂OC(=O)OEt | Me | C(=O)Me | O |
| C-8477 | H | CH₂CH₂ | Me | *c*-Pr | CH₂OC(=O)OEt | Me | C(=O)Me | O |
| C-8478 | Me | CH₂CH₂ | H | *c*-Pr | CH₂OC(=O)OEt | Me | C(=O)Me | O |
| C-8479 | H | single bond | Me | *c*-Pr | CH₂OC(=O)O(*i*-Pr) | Cl | C(=O)Me | O |
| C-8480 | Me | single bond | H | *c*-Pr | CH₂OC(=O)O(*i*-Pr) | Cl | C(=O)Me | O |
| C-8481 | Me(R) | single bond | H | *c*-Pr | CH₂OC(=O)O(*i*-Pr) | Cl | C(=O)Me | O |
| C-8482 | Me(S) | single bond | H | *c*-Pr | CH₂OC(=O)O(*i*-Pr) | Cl | C(=O)Me | O |
| C-8483 | H | CH₂ | Me | *c*-Pr | CH₂OC(=O)O(*i*-Pr) | Cl | C(=O)Me | O |
| C-8484 | Me | CH₂ | H | *c*-Pr | CH₂OC(=O)O(*i*-Pr) | Cl | C(=O)Me | O |
| C-8485 | H | CH(Me) | H | *c*-Pr | CH₂OC(=O)O(*i*-Pr) | Cl | C(=O)Me | O |
| C-8486 | H | CH₂CH₂ | Me | *c*-Pr | CH₂OC(=O)O(*i*-Pr) | Cl | C(=O)Me | O |
| C-8487 | Me | CH₂CH₂ | H | *c*-Pr | CH₂OC(=O)O(*i*-Pr) | Cl | C(=O)Me | O |
| C-8488 | H | single bond | Me | *c*-Pr | CH₂OC(=O)O(*i*-Pr) | Me | C(=O)Me | O |
| C-8489 | Me | single bond | H | *c*-Pr | CH₂OC(=O)O(*i*-Pr) | Me | C(=O)Me | O |
| C-8490 | Me(R) | single bond | H | *c*-Pr | CH₂OC(=O)O(*i*-Pr) | Me | C(=O)Me | O |
| C-8491 | Me(S) | single bond | H | *c*-Pr | CH₂OC(=O)O(*i*-Pr) | Me | C(=O)Me | O |
| C-8492 | H | CH₂ | Me | *c*-Pr | CH₂OC(=O)O(*i*-Pr) | Me | C(=O)Me | O |
| C-8493 | Me | CH₂ | H | *c*-Pr | CH₂OC(=O)O(*i*-Pr) | Me | C(=O)Me | O |
| C-8494 | H | CH(Me) | H | *c*-Pr | CH₂OC(=O)O(*i*-Pr) | Me | C(=O)Me | O |
| C-8495 | H | CH₂CH₂ | Me | *c*-Pr | CH₂OC(=O)O(*i*-Pr) | Me | C(=O)Me | O |
| C-8496 | Me | CH₂CH₂ | H | *c*-Pr | CH₂OC(=O)O(*i*-Pr) | Me | C(=O)Me | O |
| C-8497 | H | single bond | Me | *c*-Pr | CH₂N(Me)C(=O)OMe | Cl | C(=O)Me | O |
| C-8498 | Me | single bond | H | *c*-Pr | CH₂N(Me)C(=O)OMe | Cl | C(=O)Me | O |

**[Table 421]**

| Compound Number | Y | W | Z | R¹ | R² | R³ | R¹⁰ | A |
|---|---|---|---|---|---|---|---|---|
| C-8499 | Me(R) | single bond | H | *c*-Pr | CH₂N(Me)C(=O)OMe | Cl | C(=O)Me | O |
| C-8500 | Me(S) | single bond | H | *c*-Pr | CH₂N(Me)C(=O)OMe | Cl | C(=O)Me | O |
| C-8501 | H | CH₂ | Me | *c*-Pr | CH₂N(Me)C(=O)OMe | Cl | C(=O)Me | O |
| C-8502 | Me | CH₂ | H | *c*-Pr | CH₂N(Me)C(=O)OMe | Cl | C(=O)Me | O |
| C-8503 | H | CH(Me) | H | *c*-Pr | CH₂N(Me)C(=O)OMe | Cl | C(=O)Me | O |
| C-8504 | H | CH₂CH₂ | Me | *c*-Pr | CH₂N(Me)C(=O)OMe | Cl | C(=O)Me | O |
| C-8505 | Me | CH₂CH₂ | H | *c*-Pr | CH₂N(Me)C(=O)OMe | Cl | C(=O)Me | O |
| C-8506 | H | single bond | Me | *c*-Pr | CH₂N(Me)C(=O)OMe | Me | C(=O)Me | O |
| C-8507 | Me | single bond | H | *c*-Pr | CH₂N(Me)C(=O)OMe | Me | C(=O)Me | O |
| C-8508 | Me(R) | single bond | H | *c*-Pr | CH₂N(Me)C(=O)OMe | Me | C(=O)Me | O |
| C-8509 | Me(S) | single bond | H | *c*-Pr | CH₂N(Me)C(=O)OMe | Me | C(=O)Me | O |
| C-8510 | H | CH₂ | Me | *c*-Pr | CH₂N(Me)C(=O)OMe | Me | C(=O)Me | O |
| C-8511 | Me | CH₂ | H | *c*-Pr | CH₂N(Me)C(=O)OMe | Me | C(=O)Me | O |
| C-8512 | H | CH(Me) | H | *c*-Pr | CH₂N(Me)C(=O)OMe | Me | C(=O)Me | O |
| C-8513 | H | CH₂CH₂ | Me | *c*-Pr | CH₂N(Me)C(=O)OMe | Me | C(=O)Me | O |
| C-8514 | Me | CH₂CH₂ | H | *c*-Pr | CH₂N(Me)C(=O)OMe | Me | C(=O)Me | O |
| C-8515 | H | single bond | Me | *c*-Pr(1-CN) | C(=O)OCH₂CH=CH₂ | Cl | H | O |
| C-8516 | Me | single bond | H | *c*-Pr(1-CN) | C(=O)OCH₂CH=CH₂ | Cl | H | O |
| C-8517 | Me(R) | single bond | H | *c*-Pr(1-CN) | C(=O)OCH₂CH=CH₂ | Cl | H | O |
| C-8518 | Me(S) | single bond | H | *c*-Pr(1-CN) | C(=O)OCH₂CH=CH₂ | Cl | H | O |
| C-8519 | H | CH₂ | Me | *c*-Pr(1-CN) | C(=O)OCH₂CH=CH₂ | Cl | H | O |
| C-8520 | Me | CH₂ | H | *c*-Pr(1-CN) | C(=O)OCH₂CH=CH₂ | Cl | H | O |
| C-8521 | H | CH(Me) | H | *c*-Pr(1-CN) | C(=O)OCH₂CH=CH₂ | Cl | H | O |
| C-8522 | H | CH₂CH₂ | Me | *c*-Pr(1-CN) | C(=O)OCH₂CH=CH₂ | Cl | H | O |
| C-8523 | Me | CH₂CH₂ | H | *c*-Pr(1-CN) | C(=O)OCH₂CH=CH₂ | Cl | H | O |
| C-8524 | H | single bond | Me | *c*-Pr(1-CN) | C(=O)OCH₂CH=CH₂ | Me | H | O |
| C-8525 | Me | single bond | H | *c*-Pr(1-CN) | C(=O)OCH₂CH=CH₂ | Me | H | O |
| C-8526 | Me(R) | single bond | H | *c*-Pr(1-CN) | C(=O)OCH₂CH=CH₂ | Me | H | O |
| C-8527 | Me(S) | single bond | H | *c*-Pr(1-CN) | C(=O)OCH₂CH=CH₂ | Me | H | O |
| C-8528 | H | CH₂ | Me | *c*-Pr(1-CN) | C(=O)OCH₂CH=CH₂ | Me | H | O |
| C-8529 | Me | CH₂ | H | *c*-Pr(1-CN) | C(=O)OCH₂CH=CH₂ | Me | H | O |
| C-8530 | H | CH(Me) | H | *c*-Pr(1-CN) | C(=O)OCH₂CH=CH₂ | Me | H | O |
| C-8531 | H | CH₂CH₂ | Me | *c*-Pr(1-CN) | C(=O)OCH₂CH=CH₂ | Me | H | O |
| C-8532 | Me | CH₂CH₂ | H | *c*-Pr(1-CN) | C(=O)OCH₂CH=CH₂ | Me | H | O |
| C-8533 | H | single bond | Me | *c*-Pr(1-CN) | C(=O)OCH₂CCl₃ | Cl | H | O |
| C-8534 | Me | single bond | H | *c*-Pr(1-CN) | C(=O)OCH₂CCl₃ | Cl | H | O |
| C-8535 | Me(R) | single bond | H | *c*-Pr(1-CN) | C(=O)OCH₂CCl₃ | Cl | H | O |
| C-8536 | Me(S) | single bond | H | *c*-Pr(1-CN) | C(=O)OCH₂CCl₃ | Cl | H | O |
| C-8537 | H | CH₂ | Me | *c*-Pr(1-CN) | C(=O)OCH₂CCl₃ | Cl | H | O |
| C-8538 | Me | CH₂ | H | *c*-Pr(1-CN) | C(=O)OCH₂CCl₃ | Cl | H | O |
| C-8539 | H | CH(Me) | H | *c*-Pr(1-CN) | C(=O)OCH₂CCl₃ | Cl | H | O |
| C-8540 | H | CH₂CH₂ | Me | *c*-Pr(1-CN) | C(=O)OCH₂CCl₃ | Cl | H | O |
| C-8541 | Me | CH₂CH₂ | H | *c*-Pr(1-CN) | C(=O)OCH₂CCl₃ | Cl | H | O |
| C-8542 | H | single bond | Me | *c*-Pr(1-CN) | C(=O)OCH₂CCl₃ | Me | H | O |

**[Table 422]**

| Compound Number | Y | W | Z | R¹ | R² | R³ | R¹⁰ | A |
|---|---|---|---|---|---|---|---|---|
| C-8543 | Me | single bond | H | *c*-Pr(1-CN) | C(=O)OCH₂CCl₃ | Me | H | O |
| C-8544 | Me(R) | single bond | H | *c*-Pr(1-CN) | C(=O)OCH₂CCl₃ | Me | H | O |
| C-8545 | Me(S) | single bond | H | *c*-Pr(1-CN) | C(=O)OCH₂CCl₃ | Me | H | O |
| C-8546 | H | CH₂ | Me | *c*-Pr(1-CN) | C(=O)OCH₂CCl₃ | Me | H | O |
| C-8547 | Me | CH₂ | H | *c*-Pr(1-CN) | C(=O)OCH₂CCl₃ | Me | H | O |
| C-8548 | H | CH(Me) | H | *c*-Pr(1-CN) | C(=O)OCH₂CCl₃ | Me | H | O |
| C-8549 | H | CH₂CH₂ | Me | *c*-Pr(1-CN) | C(=O)OCH₂CCl₃ | Me | H | O |
| C-8550 | Me | CH₂CH₂ | H | *c*-Pr(1-CN) | C(=O)OCH₂CCl₃ | Me | H | O |
| C-8551 | H | single bond | Me | *c*-Pr(1-CN) | C(=O)N(Me)₂ | Cl | H | O |
| C-8552 | Me | single bond | H | *c*-Pr(1-CN) | C(=O)N(Me)₂ | Cl | H | O |
| C-8553 | Me(R) | single bond | H | *c*-Pr(1-CN) | C(=O)N(Me)₂ | Cl | H | O |
| C-8554 | Me(S) | single bond | H | *c*-Pr(1-CN) | C(=O)N(Me)₂ | Cl | H | O |
| C-8555 | H | CH₂ | Me | *c*-Pr(1-CN) | C(=O)N(Me)₂ | Cl | H | O |
| C-8556 | Me | CH₂ | H | *c*-Pr(1-CN) | C(=O)N(Me)₂ | Cl | H | O |
| C-8557 | H | CH(Me) | H | *c*-Pr(1-CN) | C(=O)N(Me)₂ | Cl | H | O |
| C-8558 | H | CH₂CH₂ | Me | *c*-Pr(1-CN) | C(=O)N(Me)₂ | Cl | H | O |
| C-8559 | Me | CH₂CH₂ | H | *c*-Pr(1-CN) | C(=O)N(Me)₂ | Cl | H | O |
| C-8560 | H | single bond | Me | *c*-Pr(1-CN) | C(=O)N(Me)₂ | Me | H | O |
| C-8561 | Me | single bond | H | *c*-Pr(1-CN) | C(=O)N(Me)₂ | Me | H | O |
| C-8562 | Me(R) | single bond | H | *c*-Pr(1-CN) | C(=O)N(Me)₂ | Me | H | O |
| C-8563 | Me(S) | single bond | H | *c*-Pr(1-CN) | C(=O)N(Me)₂ | Me | H | O |
| C-8564 | H | CH₂ | Me | *c*-Pr(1-CN) | C(=O)N(Me)₂ | Me | H | O |
| C-8565 | Me | CH₂ | H | *c*-Pr(1-CN) | C(=O)N(Me)₂ | Me | H | O |
| C-8566 | H | CH(Me) | H | *c*-Pr(1-CN) | C(=O)N(Me)₂ | Me | H | O |
| C-8567 | H | CH₂CH₂ | Me | *c*-Pr(1-CN) | C(=O)N(Me)₂ | Me | H | O |
| C-8568 | Me | CH₂CH₂ | H | *c*-Pr(1-CN) | C(=O)N(Me)₂ | Me | H | O |
| C-8569 | H | single bond | Me | *c*-Pr(1-CN) | CH₂OC(=O)Me | Cl | H | O |
| C-8570 | Me | single bond | H | *c*-Pr(1-CN) | CH₂OC(=O)Me | Cl | H | O |
| C-8571 | Me(R) | single bond | H | *c*-Pr(1-CN) | CH₂OC(=O)Me | Cl | H | O |
| C-8572 | Me(S) | single bond | H | *c*-Pr(1-CN) | CH₂OC(=O)Me | Cl | H | O |
| C-8573 | H | CH₂ | Me | *c*-Pr(1-CN) | CH₂OC(=O)Me | Cl | H | O |
| C-8574 | Me | CH₂ | H | *c*-Pr(1-CN) | CH₂OC(=O)Me | Cl | H | O |
| C-8575 | H | CH(Me) | H | *c*-Pr(1-CN) | CH₂OC(=O)Me | Cl | H | O |
| C-8576 | H | CH₂CH₂ | Me | *c*-Pr(1-CN) | CH₂OC(=O)Me | Cl | H | O |
| C-8577 | Me | CH₂CH₂ | H | *c*-Pr(1-CN) | CH₂OC(=O)Me | Cl | H | O |
| C-8578 | H | single bond | Me | *c*-Pr(1-CN) | CH₂OC(=O)Me | Me | H | O |
| C-8579 | Me | single bond | H | *c*-Pr(1-CN) | CH₂OC(=O)Me | Me | H | O |
| C-8580 | Me(R) | single bond | H | *c*-Pr(1-CN) | CH₂OC(=O)Me | Me | H | O |
| C-8581 | Me(S) | single bond | H | *c*-Pr(1-CN) | CH₂OC(=O)Me | Me | H | O |
| C-8582 | H | CH₂ | Me | *c*-Pr(1-CN) | CH₂OC(=O)Me | Me | H | O |
| C-8583 | Me | CH₂ | H | *c*-Pr(1-CN) | CH₂OC(=O)Me | Me | H | O |
| C-8584 | H | CH(Me) | H | *c*-Pr(1-CN) | CH₂OC(=O)Me | Me | H | O |
| C-8585 | H | CH₂CH₂ | Me | *c*-Pr(1-CN) | CH₂OC(=O)Me | Me | H | O |
| C-8586 | Me | CH₂CH₂ | H | *c*-Pr(1-CN) | CH₂OC(=O)Me | Me | H | O |

**[Table 423]**

| Compound Number | Y | W | Z | R¹ | R² | R³ | R¹⁰ | A |
|---|---|---|---|---|---|---|---|---|
| C-8587 | H | single bond | Me | *c*-Pr(1-CN) | CH₂OC(=O)(*i*-Pr) | Cl | H | O |
| C-8588 | Me | single bond | H | *c*-Pr(1-CN) | CH₂OC(=O)(*i*-Pr) | Cl | H | O |
| C-8589 | Me(R) | single bond | H | *c*-Pr(1-CN) | CH₂OC(=O)(*i*-Pr) | Cl | H | O |
| C-8590 | Me(S) | single bond | H | *c*-Pr(1-CN) | CH₂OC(=O)(*i*-Pr) | Cl | H | O |
| C-8591 | H | CH₂ | Me | *c*-Pr(1-CN) | CH₂OC(=O)(*i*-Pr) | Cl | H | O |
| C-8592 | Me | CH₂ | H | *c*-Pr(1-CN) | CH₂OC(=O)(*i*-Pr) | Cl | H | O |
| C-8593 | H | CH(Me) | H | *c*-Pr(1-CN) | CH₂OC(=O)(*i*-Pr) | Cl | H | O |
| C-8594 | H | CH₂CH₂ | Me | *c*-Pr(1-CN) | CH₂OC(=O)(*i*-Pr) | Cl | H | O |
| C-8595 | Me | CH₂CH₂ | H | *c*-Pr(1-CN) | CH₂OC(=O)(*i*-Pr) | Cl | H | O |
| C-8596 | H | single bond | Me | *c*-Pr(1-CN) | CH₂OC(=O)(*i*-Pr) | Me | H | O |
| C-8597 | Me | single bond | H | *c*-Pr(1-CN) | CH₂OC(=O)(*i*-Pr) | Me | H | O |
| C-8598 | Me(R) | single bond | H | *c*-Pr(1-CN) | CH₂OC(=O)(*i*-Pr) | Me | H | O |
| C-8599 | Me(S) | single bond | H | *c*-Pr(1-CN) | CH₂OC(=O)(*i*-Pr) | Me | H | O |
| C-8600 | H | CH₂ | Me | *c*-Pr(1-CN) | CH₂OC(=O)(*i*-Pr) | Me | H | O |
| C-8601 | Me | CH₂ | H | *c*-Pr(1-CN) | CH₂OC(=O)(*i*-Pr) | Me | H | O |
| C-8602 | H | CH(Me) | H | *c*-Pr(1-CN) | CH₂OC(=O)(*i*-Pr) | Me | H | O |
| C-8603 | H | CH₂CH₂ | Me | *c*-Pr(1-CN) | CH₂OC(=O)(*i*-Pr) | Me | H | O |
| C-8604 | Me | CH₂CH₂ | H | *c*-Pr(1-CN) | CH₂OC(=O)(*i*-Pr) | Me | H | O |
| C-8605 | H | single bond | Me | *c*-Pr(1-CN) | CH₂OC(=O)(*t*-Bu) | Cl | H | O |
| C-8606 | Me | single bond | H | *c*-Pr(1-CN) | CH₂OC(=O)(*t*-Bu) | Cl | H | O |
| C-8607 | Me(R) | single bond | H | *c*-Pr(1-CN) | CH₂OC(=O)(*t*-Bu) | Cl | H | O |
| C-8608 | Me(S) | single bond | H | *c*-Pr(1-CN) | CH₂OC(=O)(*t*-Bu) | Cl | H | O |
| C-8609 | H | CH₂ | Me | *c*-Pr(1-CN) | CH₂OC(=O)(*t*-Bu) | Cl | H | O |
| C-8610 | Me | CH₂ | H | *c*-Pr(1-CN) | CH₂OC(=O)(*t*-Bu) | Cl | H | O |
| C-8611 | H | CH(Me) | H | *c*-Pr(1-CN) | CH₂OC(=O)(*t*-Bu) | Cl | H | O |
| C-8612 | H | CH₂CH₂ | Me | *c*-Pr(1-CN) | CH₂OC(=O)(*t*-Bu) | Cl | H | O |
| C-8613 | Me | CH₂CH₂ | H | *c*-Pr(1-CN) | CH₂OC(=O)(*t*-Bu) | Cl | H | O |
| C-8614 | H | single bond | Me | *c*-Pr(1-CN) | CH₂OC(=O)(*t*-Bu) | Me | H | O |
| C-8615 | Me | single bond | H | *c*-Pr(1-CN) | CH₂OC(=O)(*t*-Bu) | Me | H | O |
| C-8616 | Me(R) | single bond | H | *c*-Pr(1-CN) | CH₂OC(=O)(*t*-Bu) | Me | H | O |
| C-8617 | Me(S) | single bond | H | *c*-Pr(1-CN) | CH₂OC(=O)(*t*-Bu) | Me | H | O |
| C-8618 | H | CH₂ | Me | *c*-Pr(1-CN) | CH₂OC(=O)(*t*-Bu) | Me | H | O |
| C-8619 | Me | CH₂ | H | *c*-Pr(1-CN) | CH₂OC(=O)(*t*-Bu) | Me | H | O |
| C-8620 | H | CH(Me) | H | *c*-Pr(1-CN) | CH₂OC(=O)(*t*-Bu) | Me | H | O |
| C-8621 | H | CH₂CH₂ | Me | *c*-Pr(1-CN) | CH₂OC(=O)(*t*-Bu) | Me | H | O |
| C-8622 | Me | CH₂CH₂ | H | *c*-Pr(1-CN) | CH₂OC(=O)(*t*-Bu) | Me | H | O |
| C-8623 | H | single bond | Me | *c*-Pr(1-CN) | CH₂OC(=O)OEt | Cl | H | O |
| C-8624 | Me | single bond | H | *c*-Pr(1-CN) | CH₂OC(=O)OEt | Cl | H | O |
| C-8625 | Me(R) | single bond | H | *c*-Pr(1-CN) | CH₂OC(=O)OEt | Cl | H | O |
| C-8626 | Me(S) | single bond | H | *c*-Pr(1-CN) | CH₂OC(=O)OEt | Cl | H | O |
| C-8627 | H | CH₂ | Me | *c*-Pr(1-CN) | CH₂OC(=O)OEt | Cl | H | O |
| C-8628 | Me | CH₂ | H | *c*-Pr(1-CN) | CH₂OC(=O)OEt | Cl | H | O |
| C-8629 | H | CH(Me) | H | *c*-Pr(1-CN) | CH₂OC(=O)OEt | Cl | H | O |
| C-8630 | H | CH₂CH₂ | Me | *c*-Pr(1-CN) | CH₂OC(=O)OEt | Cl | H | O |

**[Table 424]**

| Compound Number | Y | W | Z | R¹ | R² | R³ | R¹⁰ | A |
|---|---|---|---|---|---|---|---|---|
| C-8631 | Me | CH₂CH₂ | H | *c*-Pr(1-CN) | CH₂OC(=O)OEt | Cl | H | O |
| C-8632 | H | single bond | Me | *c*-Pr(1-CN) | CH₂OC(=O)OEt | Me | H | O |
| C-8633 | Me | single bond | H | *c*-Pr(1-CN) | CH₂OC(=O)OEt | Me | H | O |
| C-8634 | Me(R) | single bond | H | *c*-Pr(1-CN) | CH₂OC(=O)OEt | Me | H | O |
| C-8635 | Me(S) | single bond | H | *c*-Pr(1-CN) | CH₂OC(=O)OEt | Me | H | O |
| C-8636 | H | CH₂ | Me | *c*-Pr(1-CN) | CH₂OC(=O)OEt | Me | H | O |
| C-8637 | Me | CH₂ | H | *c*-Pr(1-CN) | CH₂OC(=O)OEt | Me | H | O |
| C-8638 | H | CH(Me) | H | *c*-Pr(1-CN) | CH₂OC(=O)OEt | Me | H | O |
| C-8639 | H | CH₂CH₂ | Me | *c*-Pr(1-CN) | CH₂OC(=O)OEt | Me | H | O |
| C-8640 | Me | CH₂CH₂ | H | *c*-Pr(1-CN) | CH₂OC(=O)OEt | Me | H | O |
| C-8641 | H | single bond | Me | *c*-Pr(1-CN) | CH₂OC(=O)O(*i*-Pr) | Cl | H | O |
| C-8642 | Me | single bond | H | *c*-Pr(1-CN) | CH₂OC(=O)O(*i*-Pr) | Cl | H | O |
| C-8643 | Me(R) | single bond | H | *c*-Pr(1-CN) | CH₂OC(=O)O(*i*-Pr) | Cl | H | O |
| C-8644 | Me(S) | single bond | H | *c*-Pr(1-CN) | CH₂OC(=O)O(*i*-Pr) | Cl | H | O |
| C-8645 | H | CH₂ | Me | *c*-Pr(1-CN) | CH₂OC(=O)O(*i*-Pr) | Cl | H | O |
| C-8646 | Me | CH₂ | H | *c*-Pr(1-CN) | CH₂OC(=O)O(*i*-Pr) | Cl | H | O |
| C-8647 | H | CH(Me) | H | *c*-Pr(1-CN) | CH₂OC(=O)O(*i*-Pr) | Cl | H | O |
| C-8648 | H | CH₂CH₂ | Me | *c*-Pr(1-CN) | CH₂OC(=O)O(*i*-Pr) | Cl | H | O |
| C-8649 | Me | CH₂CH₂ | H | *c*-Pr(1-CN) | CH₂OC(=O)O(*i*-Pr) | Cl | H | O |
| C-8650 | H | single bond | Me | *c*-Pr(1-CN) | CH₂OC(=O)O(*i*-Pr) | Me | H | O |
| C-8651 | Me | single bond | H | *c*-Pr(1-CN) | CH₂OC(=O)O(*i*-Pr) | Me | H | O |
| C-8652 | Me(R) | single bond | H | *c*-Pr(1-CN) | CH₂OC(=O)O(*i*-Pr) | Me | H | O |
| C-8653 | Me(S) | single bond | H | *c*-Pr(1-CN) | CH₂OC(=O)O(*i*-Pr) | Me | H | O |
| C-8654 | H | CH₂ | Me | *c*-Pr(1-CN) | CH₂OC(=O)O(*i*-Pr) | Me | H | O |
| C-8655 | Me | CH₂ | H | *c*-Pr(1-CN) | CH₂OC(=O)O(*i*-Pr) | Me | H | O |
| C-8656 | H | CH(Me) | H | *c*-Pr(1-CN) | CH₂OC(=O)O(*i*-Pr) | Me | H | O |
| C-8657 | H | CH₂CH₂ | Me | *c*-Pr(1-CN) | CH₂OC(=O)O(*i*-Pr) | Me | H | O |
| C-8658 | Me | CH₂CH₂ | H | *c*-Pr(1-CN) | CH₂OC(=O)O(*i*-Pr) | Me | H | O |
| C-8659 | H | single bond | Me | *c*-Pr(1-CN) | CH₂N(Me)C(=O)OMe | Cl | H | O |
| C-8660 | Me | single bond | H | *c*-Pr(1-CN) | CH₂N(Me)C(=O)OMe | Cl | H | O |
| C-8661 | Me(R) | single bond | H | *c*-Pr(1-CN) | CH₂N(Me)C(=O)OMe | Cl | H | O |
| C-8662 | Me(S) | single bond | H | *c*-Pr(1-CN) | CH₂N(Me)C(=O)OMe | Cl | H | O |
| C-8663 | H | CH₂ | Me | *c*-Pr(1-CN) | CH₂N(Me)C(=O)OMe | Cl | H | O |
| C-8664 | Me | CH₂ | H | *c*-Pr(1-CN) | CH₂N(Me)C(=O)OMe | Cl | H | O |
| C-8665 | H | CH(Me) | H | *c*-Pr(1-CN) | CH₂N(Me)C(=O)OMe | Cl | H | O |
| C-8666 | H | CH₂CH₂ | Me | *c*-Pr(1-CN) | CH₂N(Me)C(=O)OMe | Cl | H | O |
| C-8667 | Me | CH₂CH₂ | H | *c*-Pr(1-CN) | CH₂N(Me)C(=O)OMe | Cl | H | O |
| C-8668 | H | single bond | Me | *c*-Pr(1-CN) | CH₂N(Me)C(=O)OMe | Me | H | O |
| C-8669 | Me | single bond | H | *c*-Pr(1-CN) | CH₂N(Me)C(=O)OMe | Me | H | O |
| C-8670 | Me(R) | single bond | H | *c*-Pr(1-CN) | CH₂N(Me)C(=O)OMe | Me | H | O |
| C-8671 | Me(S) | single bond | H | *c*-Pr(1-CN) | CH₂N(Me)C(=O)OMe | Me | H | O |
| C-8672 | H | CH₂ | Me | *c*-Pr(1-CN) | CH₂N(Me)C(=O)OMe | Me | H | O |
| C-8673 | Me | CH₂ | H | *c*-Pr(1-CN) | CH₂N(Me)C(=O)OMe | Me | H | O |
| C-8674 | H | CH(Me) | H | *c*-Pr(1-CN) | CH₂N(Me)C(=O)OMe | Me | H | O |

**[Table 425]**

| Compound Number | Y | W | Z | R¹ | R² | R³ | R¹⁰ | A |
|---|---|---|---|---|---|---|---|---|
| C-8675 | H | CH₂CH₂ | Me | *c*-Pr(1-CN) | CH₂N(Me)C(=O)OMe | Me | H | O |
| C-8676 | Me | CH₂CH₂ | H | *c*-Pr(1-CN) | CH₂N(Me)C(=O)OMe | Me | H | O |
| C-8677 | H | single bond | Me | *c*-Pr(1-CN) | C(=O)OCH₂CH=CH₂ | Cl | Me | O |
| C-8678 | Me | single bond | H | *c*-Pr(1-CN) | C(=O)OCH₂CH=CH₂ | Cl | Me | O |
| C-8679 | Me(R) | single bond | H | *c*-Pr(1-CN) | C(=O)OCH₂CH=CH₂ | Cl | Me | O |
| C-8680 | Me(S) | single bond | H | *c*-Pr(1-CN) | C(=O)OCH₂CH=CH₂ | Cl | Me | O |
| C-8681 | H | CH₂ | Me | *c*-Pr(1-CN) | C(=O)OCH₂CH=CH₂ | Cl | Me | O |
| C-8682 | Me | CH₂ | H | *c*-Pr(1-CN) | C(=O)OCH₂CH=CH₂ | Cl | Me | O |
| C-8683 | H | CH(Me) | H | *c*-Pr(1-CN) | C(=O)OCH₂CH=CH₂ | Cl | Me | O |
| C-8684 | H | CH₂CH₂ | Me | *c*-Pr(1-CN) | C(=O)OCH₂CH=CH₂ | Cl | Me | O |
| C-8685 | Me | CH₂CH₂ | H | *c*-Pr(1-CN) | C(=O)OCH₂CH=CH₂ | Cl | Me | O |
| C-8686 | H | single bond | Me | *c*-Pr(1-CN) | C(=O)OCH₂CH=CH₂ | Me | Me | O |
| C-8687 | Me | single bond | H | *c*-Pr(1-CN) | C(=O)OCH₂CH=CH₂ | Me | Me | O |
| C-8688 | Me(R) | single bond | H | *c*-Pr(1-CN) | C(=O)OCH₂CH=CH₂ | Me | Me | O |
| C-8689 | Me(S) | single bond | H | *c*-Pr(1-CN) | C(=O)OCH₂CH=CH₂ | Me | Me | O |
| C-8690 | H | CH₂ | Me | *c*-Pr(1-CN) | C(=O)OCH₂CH=CH₂ | Me | Me | O |
| C-8691 | Me | CH₂ | H | *c*-Pr(1-CN) | C(=O)OCH₂CH=CH₂ | Me | Me | O |
| C-8692 | H | CH(Me) | H | *c*-Pr(1-CN) | C(=O)OCH₂CH=CH₂ | Me | Me | O |
| C-8693 | H | CH₂CH₂ | Me | *c*-Pr(1-CN) | C(=O)OCH₂CH=CH₂ | Me | Me | O |
| C-8694 | Me | CH₂CH₂ | H | *c*-Pr(1-CN) | C(=O)OCH₂CH=CH₂ | Me | Me | O |
| C-8695 | H | single bond | Me | *c*-Pr(1-CN) | C(=O)OCH₂CCl₃ | Cl | Me | O |
| C-8696 | Me | single bond | H | *c*-Pr(1-CN) | C(=O)OCH₂CCl₃ | Cl | Me | O |
| C-8697 | Me(R) | single bond | H | *c*-Pr(1-CN) | C(=O)OCH₂CCl₃ | Cl | Me | O |
| C-8698 | Me(S) | single bond | H | *c*-Pr(1-CN) | C(=O)OCH₂CCl₃ | Cl | Me | O |
| C-8699 | H | CH₂ | Me | *c*-Pr(1-CN) | C(=O)OCH₂CCl₃ | Cl | Me | O |
| C-8700 | Me | CH₂ | H | *c*-Pr(1-CN) | C(=O)OCH₂CCl₃ | Cl | Me | O |
| C-8701 | H | CH(Me) | H | *c*-Pr(1-CN) | C(=O)OCH₂CCl₃ | Cl | Me | O |
| C-8702 | H | CH₂CH₂ | Me | *c*-Pr(1-CN) | C(=O)OCH₂CCl₃ | Cl | Me | O |
| C-8703 | Me | CH₂CH₂ | H | *c*-Pr(1-CN) | C(=O)OCH₂CCl₃ | Cl | Me | O |
| C-8704 | H | single bond | Me | *c*-Pr(1-CN) | C(=O)OCH₂CCl₃ | Me | Me | O |
| C-8705 | Me | single bond | H | *c*-Pr(1-CN) | C(=O)OCH₂CCl₃ | Me | Me | O |
| C-8706 | Me(R) | single bond | H | *c*-Pr(1-CN) | C(=O)OCH₂CCl₃ | Me | Me | O |
| C-8707 | Me(S) | single bond | H | *c*-Pr(1-CN) | C(=O)OCH₂CCl₃ | Me | Me | O |
| C-8708 | H | CH₂ | Me | *c*-Pr(1-CN) | C(=O)OCH₂CCl₃ | Me | Me | O |
| C-8709 | Me | CH₂ | H | *c*-Pr(1-CN) | C(=O)OCH₂CCl₃ | Me | Me | O |
| C-8710 | H | CH(Me) | H | *c*-Pr(1-CN) | C(=O)OCH₂CCl₃ | Me | Me | O |
| C-8711 | H | CH₂CH₂ | Me | *c*-Pr(1-CN) | C(=O)OCH₂CCl₃ | Me | Me | O |
| C-8712 | Me | CH₂CH₂ | H | *c*-Pr(1-CN) | C(=O)OCH₂CCl₃ | Me | Me | O |
| C-8713 | H | single bond | Me | *c*-Pr(1-CN) | C(=O)N(Me)₂ | Cl | Me | O |
| C-8714 | Me | single bond | H | *c*-Pr(1-CN) | C(=O)N(Me)₂ | Cl | Me | O |
| C-8715 | Me(R) | single bond | H | *c*-Pr(1-CN) | C(=O)N(Me)₂ | Cl | Me | O |
| C-8716 | Me(S) | single bond | H | *c*-Pr(1-CN) | C(=O)N(Me)₂ | Cl | Me | O |
| C-8717 | H | CH₂ | Me | *c*-Pr(1-CN) | C(=O)N(Me)₂ | Cl | Me | O |
| C-8718 | Me | CH₂ | H | *c*-Pr(1-CN) | C(=O)N(Me)₂ | Cl | Me | O |

**[Table 426]**

| Compound Number | Y | W | Z | R¹ | R² | R³ | R¹⁰ | A |
|---|---|---|---|---|---|---|---|---|
| C-8719 | H | CH(Me) | H | *c*-Pr(1-CN) | C(=O)N(Me)₂ | Cl | Me | O |
| C-8720 | H | CH₂CH₂ | Me | *c*-Pr(1-CN) | C(=O)N(Me)₂ | Cl | Me | O |
| C-8721 | Me | CH₂CH₂ | H | *c*-Pr(1-CN) | C(=O)N(Me)₂ | Cl | Me | O |
| C-8722 | H | single bond | Me | *c*-Pr(1-CN) | C(=O)N(Me)₂ | Me | Me | O |
| C-8723 | Me | single bond | H | *c*-Pr(1-CN) | C(=O)N(Me)₂ | Me | Me | O |
| C-8724 | Me(R) | single bond | H | *c*-Pr(1-CN) | C(=O)N(Me)₂ | Me | Me | O |
| C-8725 | Me(S) | single bond | H | *c*-Pr(1-CN) | C(=O)N(Me)₂ | Me | Me | O |
| C-8726 | H | CH₂ | Me | *c*-Pr(1-CN) | C(=O)N(Me)₂ | Me | Me | O |
| C-8727 | Me | CH₂ | H | *c*-Pr(1-CN) | C(=O)N(Me)₂ | Me | Me | O |
| C-8728 | H | CH(Me) | H | *c*-Pr(1-CN) | C(=O)N(Me)₂ | Me | Me | O |
| C-8729 | H | CH₂CH₂ | Me | *c*-Pr(1-CN) | C(=O)N(Me)₂ | Me | Me | O |
| C-8730 | Me | CH₂CH₂ | H | *c*-Pr(1-CN) | C(=O)N(Me)₂ | Me | Me | O |
| C-8731 | H | single bond | Me | *c*-Pr(1-CN) | CH₂OC(=O)Me | Cl | Me | O |
| C-8732 | Me | single bond | H | *c*-Pr(1-CN) | CH₂OC(=O)Me | Cl | Me | O |
| C-8733 | Me(R) | single bond | H | *c*-Pr(1-CN) | CH₂OC(=O)Me | Cl | Me | O |
| C-8734 | Me(S) | single bond | H | *c*-Pr(1-CN) | CH₂OC(=O)Me | Cl | Me | O |
| C-8735 | H | CH₂ | Me | *c*-Pr(1-CN) | CH₂OC(=O)Me | Cl | Me | O |
| C-8736 | Me | CH₂ | H | *c*-Pr(1-CN) | CH₂OC(=O)Me | Cl | Me | O |
| C-8737 | H | CH(Me) | H | *c*-Pr(1-CN) | CH₂OC(=O)Me | Cl | Me | O |
| C-8738 | H | CH₂CH₂ | Me | *c*-Pr(1-CN) | CH₂OC(=O)Me | Cl | Me | O |
| C-8739 | Me | CH₂CH₂ | H | *c*-Pr(1-CN) | CH₂OC(=O)Me | Cl | Me | O |
| C-8740 | H | single bond | Me | *c*-Pr(1-CN) | CH₂OC(=O)Me | Me | Me | O |
| C-8741 | Me | single bond | H | *c*-Pr(1-CN) | CH₂OC(=O)Me | Me | Me | O |
| C-8742 | Me(R) | single bond | H | *c*-Pr(1-CN) | CH₂OC(=O)Me | Me | Me | O |
| C-8743 | Me(S) | single bond | H | *c*-Pr(1-CN) | CH₂OC(=O)Me | Me | Me | O |
| C-8744 | H | CH₂ | Me | *c*-Pr(1-CN) | CH₂OC(=O)Me | Me | Me | O |
| C-8745 | Me | CH₂ | H | *c*-Pr(1-CN) | CH₂OC(=O)Me | Me | Me | O |
| C-8746 | H | CH(Me) | H | *c*-Pr(1-CN) | CH₂OC(=O)Me | Me | Me | O |
| C-8747 | H | CH₂CH₂ | Me | *c*-Pr(1-CN) | CH₂OC(=O)Me | Me | Me | O |
| C-8748 | Me | CH₂CH₂ | H | *c*-Pr(1-CN) | CH₂OC(=O)Me | Me | Me | O |
| C-8749 | H | single bond | Me | *c*-Pr(1-CN) | CH₂OC(=O)(*i*-Pr) | Cl | Me | O |
| C-8750 | Me | single bond | H | *c*-Pr(1-CN) | CH₂OC(=O)(*i*-Pr) | Cl | Me | O |
| C-8751 | Me(R) | single bond | H | *c*-Pr(1-CN) | CH₂OC(=O)(*i*-Pr) | Cl | Me | O |
| C-8752 | Me(S) | single bond | H | *c*-Pr(1-CN) | CH₂OC(=O)(*i*-Pr) | Cl | Me | O |
| C-8753 | H | CH₂ | Me | *c*-Pr(1-CN) | CH₂OC(=O)(*i*-Pr) | Cl | Me | O |
| C-8754 | Me | CH₂ | H | *c*-Pr(1-CN) | CH₂OC(=O)(*i*-Pr) | Cl | Me | O |
| C-8755 | H | CH(Me) | H | *c*-Pr(1-CN) | CH₂OC(=O)(*i*-Pr) | Cl | Me | O |
| C-8756 | H | CH₂CH₂ | Me | *c*-Pr(1-CN) | CH₂OC(=O)(*i*-Pr) | Cl | Me | O |
| C-8757 | Me | CH₂CH₂ | H | *c*-Pr(1-CN) | CH₂OC(=O)(*i*-Pr) | Cl | Me | O |
| C-8758 | H | single bond | Me | *c*-Pr(1-CN) | CH₂OC(=O)(*i*-Pr) | Me | Me | O |
| C-8759 | Me | single bond | H | *c*-Pr(1-CN) | CH₂OC(=O)(*i*-Pr) | Me | Me | O |
| C-8760 | Me(R) | single bond | H | *c*-Pr(1-CN) | CH₂OC(=O)(*i*-Pr) | Me | Me | O |
| C-8761 | Me(S) | single bond | H | *c*-Pr(1-CN) | CH₂OC(=O)(*i*-Pr) | Me | Me | O |
| C-8762 | H | CH₂ | Me | *c*-Pr(1-CN) | CH₂OC(=O)(*i*-Pr) | Me | Me | O |

**[Table 427]**

| Compound Number | Y | W | Z | R¹ | R² | R³ | R¹⁰ | A |
|---|---|---|---|---|---|---|---|---|
| C-8763 | Me | CH₂ | H | *c*-Pr(1-CN) | CH₂OC(=O)(*i*-Pr) | Me | Me | O |
| C-8764 | H | CH(Me) | H | *c*-Pr(1-CN) | CH₂OC(=O)(*i*-Pr) | Me | Me | O |
| C-8765 | H | CH₂CH₂ | Me | *c*-Pr(1-CN) | CH₂OC(=O)(*i*-Pr) | Me | Me | O |
| C-8766 | Me | CH₂CH₂ | H | *c*-Pr(1-CN) | CH₂OC(=O)(*i*-Pr) | Me | Me | O |
| C-8767 | H | single bond | Me | *c*-Pr(1-CN) | CH₂OC(=O)(*t*-Bu) | Cl | Me | O |
| C-8768 | Me | single bond | H | *c*-Pr(1-CN) | CH₂OC(=O)(*t*-Bu) | Cl | Me | O |
| C-8769 | Me(R) | single bond | H | *c*-Pr(1-CN) | CH₂OC(=O)(*t*-Bu) | Cl | Me | O |
| C-8770 | Me(S) | single bond | H | *c*-Pr(1-CN) | CH₂OC(=O)(*t*-Bu) | Cl | Me | O |
| C-8771 | H | CH₂ | Me | *c*-Pr(1-CN) | CH₂OC(=O)(*t*-Bu) | Cl | Me | O |
| C-8772 | Me | CH₂ | H | *c*-Pr(1-CN) | CH₂OC(=O)(*t*-Bu) | Cl | Me | O |
| C-8773 | H | CH(Me) | H | *c*-Pr(1-CN) | CH₂OC(=O)(*t*-Bu) | Cl | Me | O |
| C-8774 | H | CH₂CH₂ | Me | *c*-Pr(1-CN) | CH₂OC(=O)(*t*-Bu) | Cl | Me | O |
| C-8775 | Me | CH₂CH₂ | H | *c*-Pr(1-CN) | CH₂OC(=O)(*t*-Bu) | Cl | Me | O |
| C-8776 | H | single bond | Me | *c*-Pr(1-CN) | CH₂OC(=O)(*t*-Bu) | Me | Me | O |
| C-8777 | Me | single bond | H | *c*-Pr(1-CN) | CH₂OC(=O)(*t*-Bu) | Me | Me | O |
| C-8778 | Me(R) | single bond | H | *c*-Pr(1-CN) | CH₂OC(=O)(*t*-Bu) | Me | Me | O |
| C-8779 | Me(S) | single bond | H | *c*-Pr(1-CN) | CH₂OC(=O)(*t*-Bu) | Me | Me | O |
| C-8780 | H | CH₂ | Me | *c*-Pr(1-CN) | CH₂OC(=O)(*t*-Bu) | Me | Me | O |
| C-8781 | Me | CH₂ | H | *c*-Pr(1-CN) | CH₂OC(=O)(*t*-Bu) | Me | Me | O |
| C-8782 | H | CH(Me) | H | *c*-Pr(1-CN) | CH₂OC(=O)(*t*-Bu) | Me | Me | O |
| C-8783 | H | CH₂CH₂ | Me | *c*-Pr(1-CN) | CH₂OC(=O)(*t*-Bu) | Me | Me | O |
| C-8784 | Me | CH₂CH₂ | H | *c*-Pr(1-CN) | CH₂OC(=O)(*t*-Bu) | Me | Me | O |
| C-8785 | H | single bond | Me | *c*-Pr(1-CN) | CH₂OC(=O)OEt | Cl | Me | O |
| C-8786 | Me | single bond | H | *c*-Pr(1-CN) | CH₂OC(=O)OEt | Cl | Me | O |
| C-8787 | Me(R) | single bond | H | *c*-Pr(1-CN) | CH₂OC(=O)OEt | Cl | Me | O |
| C-8788 | Me(S) | single bond | H | *c*-Pr(1-CN) | CH₂OC(=O)OEt | Cl | Me | O |
| C-8789 | H | CH₂ | Me | *c*-Pr(1-CN) | CH₂OC(=O)OEt | Cl | Me | O |
| C-8790 | Me | CH₂ | H | *c*-Pr(1-CN) | CH₂OC(=O)OEt | Cl | Me | O |
| C-8791 | H | CH(Me) | H | *c*-Pr(1-CN) | CH₂OC(=O)OEt | Cl | Me | O |
| C-8792 | H | CH₂CH₂ | Me | *c*-Pr(1-CN) | CH₂OC(=O)OEt | Cl | Me | O |
| C-8793 | Me | CH₂CH₂ | H | *c*-Pr(1-CN) | CH₂OC(=O)OEt | Cl | Me | O |
| C-8794 | H | single bond | Me | *c*-Pr(1-CN) | CH₂OC(=O)OEt | Me | Me | O |
| C-8795 | Me | single bond | H | *c*-Pr(1-CN) | CH₂OC(=O)OEt | Me | Me | O |
| C-8796 | Me(R) | single bond | H | *c*-Pr(1-CN) | CH₂OC(=O)OEt | Me | Me | O |
| C-8797 | Me(S) | single bond | H | *c*-Pr(1-CN) | CH₂OC(=O)OEt | Me | Me | O |
| C-8798 | H | CH₂ | Me | *c*-Pr(1-CN) | CH₂OC(=O)OEt | Me | Me | O |
| C-8799 | Me | CH₂ | H | *c*-Pr(1-CN) | CH₂OC(=O)OEt | Me | Me | O |
| C-8800 | H | CH(Me) | H | *c*-Pr(1-CN) | CH₂OC(=O)OEt | Me | Me | O |
| C-8801 | H | CH₂CH₂ | Me | *c*-Pr(1-CN) | CH₂OC(=O)OEt | Me | Me | O |
| C-8802 | Me | CH₂CH₂ | H | *c*-Pr(1-CN) | CH₂OC(=O)OEt | Me | Me | O |
| C-8803 | H | single bond | Me | *c*-Pr(1-CN) | CH₂OC(=O)O(*i*-Pr) | Cl | Me | O |
| C-8804 | Me | single bond | H | *c*-Pr(1-CN) | CH₂OC(=O)O(*i*-Pr) | Cl | Me | O |
| C-8805 | Me(R) | single bond | H | *c*-Pr(1-CN) | CH₂OC(=O)O(*i*-Pr) | Cl | Me | O |
| C-8806 | Me(S) | single bond | H | *c*-Pr(1-CN) | CH₂OC(=O)O(*i*-Pr) | Cl | Me | O |

**[Table 428]**

| Compound Number | Y | W | Z | R¹ | R² | R³ | R¹⁰ | A |
|---|---|---|---|---|---|---|---|---|
| C-8807 | H | CH₂ | Me | *c*-Pr(1-CN) | CH₂OC(=O)O(*i*-Pr) | Cl | Me | O |
| C-8808 | Me | CH₂ | H | *c*-Pr(1-CN) | CH₂OC(=O)O(*i*-Pr) | Cl | Me | O |
| C-8809 | H | CH(Me) | H | *c*-Pr(1-CN) | CH₂OC(=O)O(*i*-Pr) | Cl | Me | O |
| C-8810 | H | CH₂CH₂ | Me | *c*-Pr(1-CN) | CH₂OC(=O)O(*i*-Pr) | Cl | Me | O |
| C-8811 | Me | CH₂CH₂ | H | *c*-Pr(1-CN) | CH₂OC(=O)O(*i*-Pr) | Cl | Me | O |
| C-8812 | H | single bond | Me | *c*-Pr(1-CN) | CH₂OC(=O)O(*i*-Pr) | Me | Me | O |
| C-8813 | Me | single bond | H | *c*-Pr(1-CN) | CH₂OC(=O)O(*i*-Pr) | Me | Me | O |
| C-8814 | Me(R) | single bond | H | *c*-Pr(1-CN) | CH₂OC(=O)O(*i*-Pr) | Me | Me | O |
| C-8815 | Me(S) | single bond | H | *c*-Pr(1-CN) | CH₂OC(=O)O(*i*-Pr) | Me | Me | O |
| C-8816 | H | CH₂ | Me | *c*-Pr(1-CN) | CH₂OC(=O)O(*i*-Pr) | Me | Me | O |
| C-8817 | Me | CH₂ | H | *c*-Pr(1-CN) | CH₂OC(=O)O(*i*-Pr) | Me | Me | O |
| C-8818 | H | CH(Me) | H | *c*-Pr(1-CN) | CH₂OC(=O)O(*i*-Pr) | Me | Me | O |
| C-8819 | H | CH₂CH₂ | Me | *c*-Pr(1-CN) | CH₂OC(=O)O(*i*-Pr) | Me | Me | O |
| C-8820 | Me | CH₂CH₂ | H | *c*-Pr(1-CN) | CH₂OC(=O)O(*i*-Pr) | Me | Me | O |
| C-8821 | H | single bond | Me | *c*-Pr(1-CN) | CH₂N(Me)C(=O)OMe | Cl | Me | O |
| C-8822 | Me | single bond | H | *c*-Pr(1-CN) | CH₂N(Me)C(=O)OMe | Cl | Me | O |
| C-8823 | Me(R) | single bond | H | *c*-Pr(1-CN) | CH₂N(Me)C(=O)OMe | Cl | Me | O |
| C-8824 | Me(S) | single bond | H | *c*-Pr(1-CN) | CH₂N(Me)C(=O)OMe | Cl | Me | O |
| C-8825 | H | CH₂ | Me | *c*-Pr(1-CN) | CH₂N(Me)C(=O)OMe | Cl | Me | O |
| C-8826 | Me | CH₂ | H | *c*-Pr(1-CN) | CH₂N(Me)C(=O)OMe | Cl | Me | O |
| C-8827 | H | CH(Me) | H | *c*-Pr(1-CN) | CH₂N(Me)C(=O)OMe | Cl | Me | O |
| C-8828 | H | CH₂CH₂ | Me | *c*-Pr(1-CN) | CH₂N(Me)C(=O)OMe | Cl | Me | O |
| C-8829 | Me | CH₂CH₂ | H | *c*-Pr(1-CN) | CH₂N(Me)C(=O)OMe | Cl | Me | O |
| C-8830 | H | single bond | Me | *c*-Pr(1-CN) | CH₂N(Me)C(=O)OMe | Me | Me | O |
| C-8831 | Me | single bond | H | *c*-Pr(1-CN) | CH₂N(Me)C(=O)OMe | Me | Me | O |
| C-8832 | Me(R) | single bond | H | *c*-Pr(1-CN) | CH₂N(Me)C(=O)OMe | Me | Me | O |
| C-8833 | Me(S) | single bond | H | *c*-Pr(1-CN) | CH₂N(Me)C(=O)OMe | Me | Me | O |
| C-8834 | H | CH₂ | Me | *c*-Pr(1-CN) | CH₂N(Me)C(=O)OMe | Me | Me | O |
| C-8835 | Me | CH₂ | H | *c*-Pr(1-CN) | CH₂N(Me)C(=O)OMe | Me | Me | O |
| C-8836 | H | CH(Me) | H | *c*-Pr(1-CN) | CH₂N(Me)C(=O)OMe | Me | Me | O |
| C-8837 | H | CH₂CH₂ | Me | *c*-Pr(1-CN) | CH₂N(Me)C(=O)OMe | Me | Me | O |
| C-8838 | Me | CH₂CH₂ | H | *c*-Pr(1-CN) | CH₂N(Me)C(=O)OMe | Me | Me | O |
| C-8839 | H | single bond | Me | *c*-Pr(1-CN) | C(=O)OCH₂CH=CH₂ | Cl | CH₂OMe | O |
| C-8840 | Me | single bond | H | *c*-Pr(1-CN) | C(=O)OCH₂CH=CH₂ | Cl | CH₂OMe | O |
| C-8841 | Me(R) | single bond | H | *c*-Pr(1-CN) | C(=O)OCH₂CH=CH₂ | Cl | CH₂OMe | O |
| C-8842 | Me(S) | single bond | H | *c*-Pr(1-CN) | C(=O)OCH₂CH=CH₂ | Cl | CH₂OMe | O |
| C-8843 | H | CH₂ | Me | *c*-Pr(1-CN) | C(=O)OCH₂CH=CH₂ | Cl | CH₂OMe | O |
| C-8844 | Me | CH₂ | H | *c*-Pr(1-CN) | C(=O)OCH₂CH=CH₂ | Cl | CH₂OMe | O |
| C-8845 | H | CH(Me) | H | *c*-Pr(1-CN) | C(=O)OCH₂CH=CH₂ | Cl | CH₂OMe | O |
| C-8846 | H | CH₂CH₂ | Me | *c*-Pr(1-CN) | C(=O)OCH₂CH=CH₂ | Cl | CH₂OMe | O |
| C-8847 | Me | CH₂CH₂ | H | *c*-Pr(1-CN) | C(=O)OCH₂CH=CH₂ | Cl | CH₂OMe | O |
| C-8848 | H | single bond | Me | *c*-Pr(1-CN) | C(=O)OCH₂CH=CH₂ | Me | CH₂OMe | O |
| C-8849 | Me | single bond | H | *c*-Pr(1-CN) | C(=O)OCH₂CH=CH₂ | Me | CH₂OMe | O |
| C-8850 | Me(R) | single bond | H | *c*-Pr(1-CN) | C(=O)OCH₂CH=CH₂ | Me | CH₂OMe | O |

**[Table 429]**

| Compound Number | Y | W | Z | R¹ | R² | R³ | R¹⁰ | A |
|---|---|---|---|---|---|---|---|---|
| C-8851 | Me(S) | single bond | H | *c* -Pr(1-CN) | C(=O)OCH₂CH=CH₂ | Me | CH₂OMe | O |
| C-8852 | H | CH₂ | Me | *c* -Pr(1-CN) | C(=O)OCH₂CH=CH₂ | Me | CH₂OMe | O |
| C-8853 | Me | CH₂ | H | *c* -Pr(1-CN) | C(=O)OCH₂CH=CH₂ | Me | CH₂OMe | O |
| C-8854 | H | CH(Me) | H | *c* -Pr(1-CN) | C(=O)OCH₂CH=CH₂ | Me | CH₂OMe | O |
| C-8855 | H | CH₂CH₂ | Me | *c* -Pr(1-CN) | C(=O)OCH₂CH=CH₂ | Me | CH₂OMe | O |
| C-8856 | Me | CH₂CH₂ | H | *c* -Pr(1-CN) | C(=O)OCH₂CH=CH₂ | Me | CH₂OMe | O |
| C-8857 | H | single bond | Me | *c* -Pr(1-CN) | C(=O)OCH₂CCl₃ | Cl | CH₂OMe | O |
| C-8858 | Me | single bond | H | *c* -Pr(1-CN) | C(=O)OCH₂CCl₃ | Cl | CH₂OMe | O |
| C-8859 | Me(R) | single bond | H | *c* -Pr(1-CN) | C(=O)OCH₂CCl₃ | Cl | CH₂OMe | O |
| C-8860 | Me(S) | single bond | H | *c* -Pr(1-CN) | C(=O)OCH₂CCl₃ | Cl | CH₂OMe | O |
| C-8861 | H | CH₂ | Me | *c* -Pr(1-CN) | C(=O)OCH₂CCl₃ | Cl | CH₂OMe | O |
| C-8862 | Me | CH₂ | H | *c* -Pr(1-CN) | C(=O)OCH₂CCl₃ | Cl | CH₂OMe | O |
| C-8863 | H | CH(Me) | H | *c* -Pr(1-CN) | C(=O)OCH₂CCl₃ | Cl | CH₂OMe | O |
| C-8864 | H | CH₂CH₂ | Me | *c* -Pr(1-CN) | C(=O)OCH₂CCl₃ | Cl | CH₂OMe | O |
| C-8865 | Me | CH₂CH₂ | H | *c* -Pr(1-CN) | C(=O)OCH₂CCl₃ | Cl | CH₂OMe | O |
| C-8866 | H | single bond | Me | *c* -Pr(1-CN) | C(=O)OCH₂CCl₃ | Me | CH₂OMe | O |
| C-8867 | Me | single bond | H | *c* -Pr(1-CN) | C(=O)OCH₂CCl₃ | Me | CH₂OMe | O |
| C-8868 | Me(R) | single bond | H | *c* -Pr(1-CN) | C(=O)OCH₂CCl₃ | Me | CH₂OMe | O |
| C-8869 | Me(S) | single bond | H | *c* -Pr(1-CN) | C(=O)OCH₂CCl₃ | Me | CH₂OMe | O |
| C-8870 | H | CH₂ | Me | *c* -Pr(1-CN) | C(=O)OCH₂CCl₃ | Me | CH₂OMe | O |
| C-8871 | Me | CH₂ | H | *c* -Pr(1-CN) | C(=O)OCH₂CCl₃ | Me | CH₂OMe | O |
| C-8872 | H | CH(Me) | H | *c* -Pr(1-CN) | C(=O)OCH₂CCl₃ | Me | CH₂OMe | O |
| C-8873 | H | CH₂CH₂ | Me | *c* -Pr(1-CN) | C(=O)OCH₂CCl₃ | Me | CH₂OMe | O |
| C-8874 | Me | CH₂CH₂ | H | *c* -Pr(1-CN) | C(=O)OCH₂CCl₃ | Me | CH₂OMe | O |
| C-8875 | H | single bond | Me | *c* -Pr(1-CN) | C(=O)N(Me)₂ | Cl | CH₂OMe | O |
| C-8876 | Me | single bond | H | *c* -Pr(1-CN) | C(=O)N(Me)₂ | Cl | CH₂OMe | O |
| C-8877 | Me(R) | single bond | H | *c* -Pr(1-CN) | C(=O)N(Me)₂ | Cl | CH₂OMe | O |
| C-8878 | Me(S) | single bond | H | *c* -Pr(1-CN) | C(=O)N(Me)₂ | Cl | CH₂OMe | O |
| C-8879 | H | CH₂ | Me | *c* -Pr(1-CN) | C(=O)N(Me)₂ | Cl | CH₂OMe | O |
| C-8880 | Me | CH₂ | H | *c* -Pr(1-CN) | C(=O)N(Me)₂ | Cl | CH₂OMe | O |
| C-8881 | H | CH(Me) | H | *c* -Pr(1-CN) | C(=O)N(Me)₂ | Cl | CH₂OMe | O |
| C-8882 | H | CH₂CH₂ | Me | *c* -Pr(1-CN) | C(=O)N(Me)₂ | Cl | CH₂OMe | O |
| C-8883 | Me | CH₂CH₂ | H | *c* -Pr(1-CN) | C(=O)N(Me)₂ | Cl | CH₂OMe | O |
| C-8884 | H | single bond | Me | *c* -Pr(1-CN) | C(=O)N(Me)₂ | Me | CH₂OMe | O |
| C-8885 | Me | single bond | H | *c* -Pr(1-CN) | C(=O)N(Me)₂ | Me | CH₂OMe | O |
| C-8886 | Me(R) | single bond | H | *c* -Pr(1-CN) | C(=O)N(Me)₂ | Me | CH₂OMe | O |
| C-8887 | Me(S) | single bond | H | *c* -Pr(1-CN) | C(=O)N(Me)₂ | Me | CH₂OMe | O |
| C-8888 | H | CH₂ | Me | *c* -Pr(1-CN) | C(=O)N(Me)₂ | Me | CH₂OMe | O |
| C-8889 | Me | CH₂ | H | *c* -Pr(1-CN) | C(=O)N(Me)₂ | Me | CH₂OMe | O |
| C-8890 | H | CH(Me) | H | *c* -Pr(1-CN) | C(=O)N(Me)₂ | Me | CH₂OMe | O |
| C-8891 | H | CH₂CH₂ | Me | *c* -Pr(1-CN) | C(=O)N(Me)₂ | Me | CH₂OMe | O |
| C-8892 | Me | CH₂CH₂ | H | *c* -Pr(1-CN) | C(=O)N(Me)₂ | Me | CH₂OMe | O |
| C-8893 | H | single bond | Me | *c* -Pr(1-CN) | CH₂OC(=O)Me | Cl | CH₂OMe | O |
| C-8894 | Me | single bond | H | *c* -Pr(1-CN) | CH₂OC(=O)Me | Cl | CH₂OMe | O |

**[Table 430]**

| Compound Number | Y | W | Z | R¹ | R² | R³ | R¹⁰ | A |
|---|---|---|---|---|---|---|---|---|
| C-8895 | Me(R) | single bond | H | *c* -Pr(1-CN) | CH₂OC(=O)Me | Cl | CH₂OMe | O |
| C-8896 | Me(S) | single bond | H | *c* -Pr(1-CN) | CH₂OC(=O)Me | Cl | CH₂OMe | O |
| C-8897 | H | CH₂ | Me | *c* -Pr(1-CN) | CH₂OC(=O)Me | Cl | CH₂OMe | O |
| C-8898 | Me | CH₂ | H | *c* -Pr(1-CN) | CH₂OC(=O)Me | Cl | CH₂OMe | O |
| C-8899 | H | CH(Me) | H | *c* -Pr(1-CN) | CH₂OC(=O)Me | Cl | CH₂OMe | O |
| C-8900 | H | CH₂CH₂ | Me | *c* -Pr(1-CN) | CH₂OC(=O)Me | Cl | CH₂OMe | O |
| C-8901 | Me | CH₂CH₂ | H | *c* -Pr(1-CN) | CH₂OC(=O)Me | Cl | CH₂OMe | O |
| C-8902 | H | single bond | Me | *c* -Pr(1-CN) | CH₂OC(=O)Me | Me | CH₂OMe | O |
| C-8903 | Me | single bond | H | *c* -Pr(1-CN) | CH₂OC(=O)Me | Me | CH₂OMe | O |
| C-8904 | Me(R) | single bond | H | *c* -Pr(1-CN) | CH₂OC(=O)Me | Me | CH₂OMe | O |
| C-8905 | Me(S) | single bond | H | *c* -Pr(1-CN) | CH₂OC(=O)Me | Me | CH₂OMe | O |
| C-8906 | H | CH₂ | Me | *c* -Pr(1-CN) | CH₂OC(=O)Me | Me | CH₂OMe | O |
| C-8907 | Me | CH₂ | H | *c* -Pr(1-CN) | CH₂OC(=O)Me | Me | CH₂OMe | O |
| C-8908 | H | CH(Me) | H | *c* -Pr(1-CN) | CH₂OC(=O)Me | Me | CH₂OMe | O |
| C-8909 | H | CH₂CH₂ | Me | *c* -Pr(1-CN) | CH₂OC(=O)Me | Me | CH₂OMe | O |
| C-8910 | Me | CH₂CH₂ | H | *c* -Pr(1-CN) | CH₂OC(=O)Me | Me | CH₂OMe | O |
| C-8911 | H | single bond | Me | *c* -Pr(1-CN) | CH₂OC(=O)(*í* -Pr) | Cl | CH₂OMe | O |
| C-8912 | Me | single bond | H | *c* -Pr(1-CN) | CH₂OC(=O)(*í* -Pr) | Cl | CH₂OMe | O |
| C-8913 | Me(R) | single bond | H | *c* -Pr(1-CN) | CH₂OC(=O)(*í* -Pr) | Cl | CH₂OMe | O |
| C-8914 | Me(S) | single bond | H | *c* -Pr(1-CN) | CH₂OC(=O)(*í* -Pr) | Cl | CH₂OMe | O |
| C-8915 | H | CH₂ | Me | *c* -Pr(1-CN) | CH₂OC(=O)(*í* -Pr) | Cl | CH₂OMe | O |
| C-8916 | Me | CH₂ | H | *c* -Pr(1-CN) | CH₂OC(=O)(*í* -Pr) | Cl | CH₂OMe | O |
| C-8917 | H | CH(Me) | H | *c* -Pr(1-CN) | CH₂OC(=O)(*í* -Pr) | Cl | CH₂OMe | O |
| C-8918 | H | CH₂CH₂ | Me | *c* -Pr(1-CN) | CH₂OC(=O)(*í* -Pr) | Cl | CH₂OMe | O |
| C-8919 | Me | CH₂CH₂ | H | *c* -Pr(1-CN) | CH₂OC(=O)(*i* -Pr) | Cl | CH₂OMe | O |
| C-8920 | H | single bond | Me | *c* -Pr(1-CN) | CH₂OC(=O)(*í* -Pr) | Me | CH₂OMe | O |
| C-8921 | Me | single bond | H | *c* -Pr(1-CN) | CH₂OC(=O)(*i* -Pr) | Me | CH₂OMe | O |
| C-8922 | Me(R) | single bond | H | *c* -Pr(1-CN) | CH₂OC(=O)(*í* -Pr) | Me | CH₂OMe | O |
| C-8923 | Me(S) | single bond | H | *c* -Pr(1-CN) | CH₂OC(=O)(*i* -Pr) | Me | CH₂OMe | O |
| C-8924 | H | CH₂ | Me | *c* -Pr(1-CN) | CH₂OC(=O)(*í* -Pr) | Me | CH₂OMe | O |
| C-8925 | Me | CH₂ | H | *c* -Pr(1-CN) | CH₂OC(=O)(*í* -Pr) | Me | CH₂OMe | O |
| C-8926 | H | CH(Me) | H | *c* -Pr(1-CN) | CH₂OC(=O)(*í* -Pr) | Me | CH₂OMe | O |
| C-8927 | H | CH₂CH₂ | Me | *c* -Pr(1-CN) | CH₂OC(=O)(*í* -Pr) | Me | CH₂OMe | O |
| C-8928 | Me | CH₂CH₂ | H | *c* -Pr(1-CN) | CH₂OC(=O)(*í* -Pr) | Me | CH₂OMe | O |
| C-8929 | H | single bond | Me | *c* -Pr(1-CN) | CH₂OC(=O)(*t* -Bu) | Cl | CH₂OMe | O |
| C-8930 | Me | single bond | H | *c* -Pr(1-CN) | CH₂OC(=O)(*t* -Bu) | Cl | CH₂OMe | O |
| C-8931 | Me(R) | single bond | H | *c* -Pr(1-CN) | CH₂OC(=O)(*t* -Bu) | Cl | CH₂OMe | O |
| C-8932 | Me(S) | single bond | H | *c* -Pr(1-CN) | CH₂OC(=O)(*t* -Bu) | Cl | CH₂OMe | O |
| C-8933 | H | CH₂ | Me | *c* -Pr(1-CN) | CH₂OC(=O)(*t* -Bu) | Cl | CH₂OMe | O |
| C-8934 | Me | CH₂ | H | *c* -Pr(1-CN) | CH₂OC(=O)(*t* -Bu) | Cl | CH₂OMe | O |
| C-8935 | H | CH(Me) | H | *c* -Pr(1-CN) | CH₂OC(=O)(*t* -Bu) | Cl | CH₂OMe | O |
| C-8936 | H | CH₂CH₂ | Me | *c* -Pr(1-CN) | CH₂OC(=O)(*t* -Bu) | Cl | CH₂OMe | O |
| C-8937 | Me | CH₂CH₂ | H | *c* -Pr(1-CN) | CH₂OC(=O)(*t* -Bu) | Cl | CH₂OMe | O |
| C-8938 | H | single bond | Me | *c* -Pr(1-CN) | CH₂OC(=O)(*t* -Bu) | Me | CH₂OMe | O |

**[Table 431]**

| Compound Number | Y | W | Z | R¹ | R² | R³ | R¹⁰ | A |
|---|---|---|---|---|---|---|---|---|
| C-8939 | Me | single bond | H | *c* -Pr(1-CN) | CH₂OC(=O)(*t* -Bu) | Me | CH₂OMe | O |
| C-8940 | Me(R) | single bond | H | *c* -Pr(1-CN) | CH₂OC(=O)(*t* -Bu) | Me | CH₂OMe | O |
| C-8941 | Me(S) | single bond | H | *c* -Pr(1-CN) | CH₂OC(=O)(*t* -Bu) | Me | CH₂OMe | O |
| C-8942 | H | CH₂ | Me | *c* -Pr(1-CN) | CH₂OC(=O)(*t* -Bu) | Me | CH₂OMe | O |
| C-8943 | Me | CH₂ | H | *c* -Pr(1-CN) | CH₂OC(=O)(*t* -Bu) | Me | CH₂OMe | O |
| C-8944 | H | CH(Me) | H | *c* -Pr(1-CN) | CH₂OC(=O)(*t* -Bu) | Me | CH₂OMe | O |
| C-8945 | H | CH₂CH₂ | Me | *c* -Pr(1-CN) | CH₂OC(=O)(*t* -Bu) | Me | CH₂OMe | O |
| C-8946 | Me | CH₂CH₂ | H | *c* -Pr(1-CN) | CH₂OC(=O)(*t* -Bu) | Me | CH₂OMe | O |
| C-8947 | H | single bond | Me | *c* -Pr(1-CN) | CH₂OC(=O)OEt | Cl | CH₂OMe | O |
| C-8948 | Me | single bond | H | *c* -Pr(1-CN) | CH₂OC(=O)OEt | Cl | CH₂OMe | O |
| C-8949 | Me(R) | single bond | H | *c* -Pr(1-CN) | CH₂OC(=O)OEt | Cl | CH₂OMe | O |
| C-8950 | Me(S) | single bond | H | *c* -Pr(1-CN) | CH₂OC(=O)OEt | Cl | CH₂OMe | O |
| C-8951 | H | CH₂ | Me | *c* -Pr(1-CN) | CH₂OC(=O)OEt | Cl | CH₂OMe | O |
| C-8952 | Me | CH₂ | H | *c* -Pr(1-CN) | CH₂OC(=O)OEt | Cl | CH₂OMe | O |
| C-8953 | H | CH(Me) | H | *c* -Pr(1-CN) | CH₂OC(=O)OEt | Cl | CH₂OMe | O |
| C-8954 | H | CH₂CH₂ | Me | *c* -Pr(1-CN) | CH₂OC(=O)OEt | Cl | CH₂OMe | O |
| C-8955 | Me | CH₂CH₂ | H | *c* -Pr(1-CN) | CH₂OC(=O)OEt | Cl | CH₂OMe | O |
| C-8956 | H | single bond | Me | *c* -Pr(1-CN) | CH₂OC(=O)OEt | Me | CH₂OMe | O |
| C-8957 | Me | single bond | H | *c* -Pr(1-CN) | CH₂OC(=O)OEt | Me | CH₂OMe | O |
| C-8958 | Me(R) | single bond | H | *c* -Pr(1-CN) | CH₂OC(=O)OEt | Me | CH₂OMe | O |
| C-8959 | Me(S) | single bond | H | *c* -Pr(1-CN) | CH₂OC(=O)OEt | Me | CH₂OMe | O |
| C-8960 | H | CH₂ | Me | *c* -Pr(1-CN) | CH₂OC(=O)OEt | Me | CH₂OMe | O |
| C-8961 | Me | CH₂ | H | *c* -Pr(1-CN) | CH₂OC(=O)OEt | Me | CH₂OMe | O |
| C-8962 | H | CH(Me) | H | *c* -Pr(1-CN) | CH₂OC(=O)OEt | Me | CH₂OMe | O |
| C-8963 | H | CH₂CH₂ | Me | *c* -Pr(1-CN) | CH₂OC(=O)OEt | Me | CH₂OMe | O |
| C-8964 | Me | CH₂CH₂ | H | *c* -Pr(1-CN) | CH₂OC(=O)OEt | Me | CH₂OMe | O |
| C-8965 | H | single bond | Me | *c* -Pr(1-CN) | CH₂OC(=O)O(*i* -Pr) | Cl | CH₂OMe | O |
| C-8966 | Me | single bond | H | *c* -Pr(1-CN) | CH₂OC(=O)O(*i* -Pr) | Cl | CH₂OMe | O |
| C-8967 | Me(R) | single bond | H | *c* -Pr(1-CN) | CH₂OC(=O)O(*i* -Pr) | Cl | CH₂OMe | O |
| C-8968 | Me(S) | single bond | H | *c* -Pr(1-CN) | CH₂OC(=O)O(*i* -Pr) | Cl | CH₂OMe | O |
| C-8969 | H | CH₂ | Me | *c* -Pr(1-CN) | CH₂OC(=O)O(*i* -Pr) | Cl | CH₂OMe | O |
| C-8970 | Me | CH₂ | H | *c* -Pr(1-CN) | CH₂OC(=O)O(*i* -Pr) | Cl | CH₂OMe | O |
| C-8971 | H | CH(Me) | H | *c* -Pr(1-CN) | CH₂OC(=O)O(*i* -Pr) | Cl | CH₂OMe | O |
| C-8972 | H | CH₂CH₂ | Me | *c* -Pr(1-CN) | CH₂OC(=O)O(*i* -Pr) | Cl | CH₂OMe | O |
| C-8973 | Me | CH₂CH₂ | H | *c* -Pr(1-CN) | CH₂OC(=O)O(*i* -Pr) | Cl | CH₂OMe | O |
| C-8974 | H | single bond | Me | *c* -Pr(1-CN) | CH₂OC(=O)O(*i* -Pr) | Me | CH₂OMe | O |
| C-8975 | Me | single bond | H | *c* -Pr(1-CN) | CH₂OC(=O)O(*i* -Pr) | Me | CH₂OMe | O |
| C-8976 | Me(R) | single bond | H | *c* -Pr(1-CN) | CH₂OC(=O)O(*i* -Pr) | Me | CH₂OMe | O |
| C-8977 | Me(S) | single bond | H | *c* -Pr(1-CN) | CH₂OC(=O)O(*i* -Pr) | Me | CH₂OMe | O |
| C-8978 | H | CH₂ | Me | *c* -Pr(1-CN) | CH₂OC(=O)O(*i* -Pr) | Me | CH₂OMe | O |
| C-8979 | Me | CH₂ | H | *c* -Pr(1-CN) | CH₂OC(=O)O(*i* -Pr) | Me | CH₂OMe | O |
| C-8980 | H | CH(Me) | H | *c* -Pr(1-CN) | CH₂OC(=O)O(*i* -Pr) | Me | CH₂OMe | O |
| C-8981 | H | CH₂CH₂ | Me | *c* -Pr(1-CN) | CH₂OC(=O)O(*i* -Pr) | Me | CH₂OMe | O |
| C-8982 | Me | CH₂CH₂ | H | *c* -Pr(1-CN) | CH₂OC(=O)O(*i* -Pr) | Me | CH₂OMe | O |

**[Table 432]**

| Compound Number | Y | W | Z | R¹ | R² | R³ | R¹⁰ | A |
|---|---|---|---|---|---|---|---|---|
| C-8983 | H | single bond | Me | *c* -Pr(1-CN) | CH₂N(Me)C(=O)OMe | Cl | CH₂OMe | O |
| C-8984 | Me | single bond | H | *c* -Pr(1-CN) | CH₂N(Me)C(=O)OMe | Cl | CH₂OMe | O |
| C-8985 | Me(R) | single bond | H | *c* -Pr(1-CN) | CH₂N(Me)C(=O)OMe | Cl | CH₂OMe | O |
| C-8986 | Me(S) | single bond | H | *c* -Pr(1-CN) | CH₂N(Me)C(=O)OMe | Cl | CH₂OMe | O |
| C-8987 | H | CH₂ | Me | *c* -Pr(1-CN) | CH₂N(Me)C(=O)OMe | Cl | CH₂OMe | O |
| C-8988 | Me | CH₂ | H | *c* -Pr(1-CN) | CH₂N(Me)C(=O)OMe | Cl | CH₂OMe | O |
| C-8989 | H | CH(Me) | H | *c* -Pr(1-CN) | CH₂N(Me)C(=O)OMe | Cl | CH₂OMe | O |
| C-8990 | H | CH₂CH₂ | Me | *c* -Pr(1-CN) | CH₂N(Me)C(=O)OMe | Cl | CH₂OMe | O |
| C-8991 | Me | CH₂CH₂ | H | *c* -Pr(1-CN) | CH₂N(Me)C(=O)OMe | Cl | CH₂OMe | O |
| C-8992 | H | single bond | Me | *c* -Pr(1-CN) | CH₂N(Me)C(=O)OMe | Me | CH₂OMe | O |
| C-8993 | Me | single bond | H | *c* -Pr(1-CN) | CH₂N(Me)C(=O)OMe | Me | CH₂OMe | O |
| C-8994 | Me(R) | single bond | H | *c* -Pr(1-CN) | CH₂N(Me)C(=O)OMe | Me | CH₂OMe | O |
| C-8995 | Me(S) | single bond | H | *c* -Pr(1-CN) | CH₂N(Me)C(=O)OMe | Me | CH₂OMe | O |
| C-8996 | H | CH₂ | Me | *c* -Pr(1-CN) | CH₂N(Me)C(=O)OMe | Me | CH₂OMe | O |
| C-8997 | Me | CH₂ | H | *c* -Pr(1-CN) | CH₂N(Me)C(=O)OMe | Me | CH₂OMe | O |
| C-8998 | H | CH(Me) | H | *c* -Pr(1-CN) | CH₂N(Me)C(=O)OMe | Me | CH₂OMe | O |
| C-8999 | H | CH₂CH₂ | Me | *c* -Pr(1-CN) | CH₂N(Me)C(=O)OMe | Me | CH₂OMe | O |
| C-9000 | Me | CH₂CH₂ | H | *c* -Pr(1-CN) | CH₂N(Me)C(=O)OMe | Me | CH₂OMe | O |
| C-9001 | H | single bond | Me | *c* -Pr(1-CN) | C(=O)OCH₂CH=CH₂ | Cl | CH₂CN | O |
| C-9002 | Me | single bond | H | *c* -Pr(1-CN) | C(=O)OCH₂CH=CH₂ | Cl | CH₂CN | O |
| C-9003 | Me(R) | single bond | H | *c* -Pr(1-CN) | C(=O)OCH₂CH=CH₂ | Cl | CH₂CN | O |
| C-9004 | Me(S) | single bond | H | *c* -Pr(1-CN) | C(=O)OCH₂CH=CH₂ | Cl | CH₂CN | O |
| C-9005 | H | CH₂ | Me | *c* -Pr(1-CN) | C(=O)OCH₂CH=CH₂ | Cl | CH₂CN | O |
| C-9006 | Me | CH₂ | H | *c* -Pr(1-CN) | C(=O)OCH₂CH=CH₂ | Cl | CH₂CN | O |
| C-9007 | H | CH(Me) | H | *c* -Pr(1-CN) | C(=O)OCH₂CH=CH₂ | Cl | CH₂CN | O |
| C-9008 | H | CH₂CH₂ | Me | *c* -Pr(1-CN) | C(=O)OCH₂CH=CH₂ | Cl | CH₂CN | O |
| C-9009 | Me | CH₂CH₂ | H | *c* -Pr(1-CN) | C(=O)OCH₂CH=CH₂ | Cl | CH₂CN | O |
| C-9010 | H | single bond | Me | *c* -Pr(1-CN) | C(=O)OCH₂CH=CH₂ | Me | CH₂CN | O |
| C-9011 | Me | single bond | H | *c* -Pr(1-CN) | C(=O)OCH₂CH=CH₂ | Me | CH₂CN | O |
| C-9012 | Me(R) | single bond | H | *c* -Pr(1-CN) | C(=O)OCH₂CH=CH₂ | Me | CH₂CN | O |
| C-9013 | Me(S) | single bond | H | *c* -Pr(1-CN) | C(=O)OCH₂CH=CH₂ | Me | CH₂CN | O |
| C-9014 | H | CH₂ | Me | *c* -Pr(1-CN) | C(=O)OCH₂CH=CH₂ | Me | CH₂CN | O |
| C-9015 | Me | CH₂ | H | *c* -Pr(1-CN) | C(=O)OCH₂CH=CH₂ | Me | CH₂CN | O |
| C-9016 | H | CH(Me) | H | *c* -Pr(1-CN) | C(=O)OCH₂CH=CH₂ | Me | CH₂CN | O |
| C-9017 | H | CH₂CH₂ | Me | *c* -Pr(1-CN) | C(=O)OCH₂CH=CH₂ | Me | CH₂CN | O |
| C-9018 | Me | CH₂CH₂ | H | *c* -Pr(1-CN) | C(=O)OCH₂CH=CH₂ | Me | CH₂CN | O |
| C-9019 | H | single bond | Me | *c* -Pr(1-CN) | C(=O)OCH₂CCl₃ | Cl | CH₂CN | O |
| C-9020 | Me | single bond | H | *c* -Pr(1-CN) | C(=O)OCH₂CCl₃ | Cl | CH₂CN | O |
| C-9021 | Me(R) | single bond | H | *c* -Pr(1-CN) | C(=O)OCH₂CCl₃ | Cl | CH₂CN | O |
| C-9022 | Me(S) | single bond | H | *c* -Pr(1-CN) | C(=O)OCH₂CCl₃ | Cl | CH₂CN | O |
| C-9023 | H | CH₂ | Me | *c* -Pr(1-CN) | C(=O)OCH₂CCl₃ | Cl | CH₂CN | O |
| C-9024 | Me | CH₂ | H | *c* -Pr(1-CN) | C(=O)OCH₂CCl₃ | Cl | CH₂CN | O |
| C-9025 | H | CH(Me) | H | *c* -Pr(1-CN) | C(=O)OCH₂CCl₃ | Cl | CH₂CN | O |
| C-9026 | H | CH₂CH₂ | Me | *c* -Pr(1-CN) | C(=O)OCH₂CCl₃ | Cl | CH₂CN | O |

**[Table 433]**

| Compound Number | Y | W | Z | R¹ | R² | R³ | R¹⁰ | A |
|---|---|---|---|---|---|---|---|---|
| C-9027 | Me | CH₂CH₂ | H | *c* -Pr(1-CN) | C(=O)OCH₂CCl₃ | Cl | CH₂CN | O |
| C-9028 | H | single bond | Me | *c* -Pr(1-CN) | C(=O)OCH₂CCl₃ | Me | CH₂CN | O |
| C-9029 | Me | single bond | H | *c* -Pr(1-CN) | C(=O)OCH₂CCl₃ | Me | CH₂CN | O |
| C-9030 | Me(R) | single bond | H | *c* -Pr(1-CN) | C(=O)OCH₂CCl₃ | Me | CH₂CN | O |
| C-9031 | Me(S) | single bond | H | *c* -Pr(1-CN) | C(=O)OCH₂CCl₃ | Me | CH₂CN | O |
| C-9032 | H | CH₂ | Me | *c* -Pr(1-CN) | C(=O)OCH₂CCl₃ | Me | CH₂CN | O |
| C-9033 | Me | CH₂ | H | *c* -Pr(1-CN) | C(=O)OCH₂CCl₃ | Me | CH₂CN | O |
| C-9034 | H | CH(Me) | H | *c* -Pr(1-CN) | C(=O)OCH₂CCl₃ | Me | CH₂CN | O |
| C-9035 | H | CH₂CH₂ | Me | *c* -Pr(1-CN) | C(=O)OCH₂CCl₃ | Me | CH₂CN | O |
| C-9036 | Me | CH₂CH₂ | H | *c* -Pr(1-CN) | C(=O)OCH₂CCl₃ | Me | CH₂CN | O |
| C-9037 | H | single bond | Me | *c* -Pr(1-CN) | C(=O)N(Me)₂ | Cl | CH₂CN | O |
| C-9038 | Me | single bond | H | *c* -Pr(1-CN) | C(=O)N(Me)₂ | Cl | CH₂CN | O |
| C-9039 | Me(R) | single bond | H | *c* -Pr(1-CN) | C(=O)N(Me)₂ | Cl | CH₂CN | O |
| C-9040 | Me(S) | single bond | H | *c* -Pr(1-CN) | C(=O)N(Me)₂ | Cl | CH₂CN | O |
| C-9041 | H | CH₂ | Me | *c* -Pr(1-CN) | C(=O)N(Me)₂ | Cl | CH₂CN | O |
| C-9042 | Me | CH₂ | H | *c* -Pr(1-CN) | C(=O)N(Me)₂ | Cl | CH₂CN | O |
| C-9043 | H | CH(Me) | H | *c* -Pr(1-CN) | C(=O)N(Me)₂ | Cl | CH₂CN | O |
| C-9044 | H | CH₂CH₂ | Me | *c* -Pr(1-CN) | C(=O)N(Me)₂ | Cl | CH₂CN | O |
| C-9045 | Me | CH₂CH₂ | H | *c* -Pr(1-CN) | C(=O)N(Me)₂ | Cl | CH₂CN | O |
| C-9046 | H | single bond | Me | *c* -Pr(1-CN) | C(=O)N(Me)₂ | Me | CH₂CN | O |
| C-9047 | Me | single bond | H | *c* -Pr(1-CN) | C(=O)N(Me)₂ | Me | CH₂CN | O |
| C-9048 | Me(R) | single bond | H | *c* -Pr(1-CN) | C(=O)N(Me)₂ | Me | CH₂CN | O |
| C-9049 | Me(S) | single bond | H | *c* -Pr(1-CN) | C(=O)N(Me)₂ | Me | CH₂CN | O |
| C-9050 | H | CH₂ | Me | *c* -Pr(1-CN) | C(=O)N(Me)₂ | Me | CH₂CN | O |
| C-9051 | Me | CH₂ | H | *c* -Pr(1-CN) | C(=O)N(Me)₂ | Me | CH₂CN | O |
| C-9052 | H | CH(Me) | H | *c* -Pr(1-CN) | C(=O)N(Me)₂ | Me | CH₂CN | O |
| C-9053 | H | CH₂CH₂ | Me | *c* -Pr(1-CN) | C(=O)N(Me)₂ | Me | CH₂CN | O |
| C-9054 | Me | CH₂CH₂ | H | *c* -Pr(1-CN) | C(=O)N(Me)₂ | Me | CH₂CN | O |
| C-9055 | H | single bond | Me | *c* -Pr(1-CN) | CH₂OC(=O)Me | Cl | CH₂CN | O |
| C-9056 | Me | single bond | H | *c* -Pr(1-CN) | CH₂OC(=O)Me | Cl | CH₂CN | O |
| C-9057 | Me(R) | single bond | H | *c* -Pr(1-CN) | CH₂OC(=O)Me | Cl | CH₂CN | O |
| C-9058 | Me(S) | single bond | H | *c* -Pr(1-CN) | CH₂OC(=O)Me | Cl | CH₂CN | O |
| C-9059 | H | CH₂ | Me | *c* -Pr(1-CN) | CH₂OC(=O)Me | Cl | CH₂CN | O |
| C-9060 | Me | CH₂ | H | *c* -Pr(1-CN) | CH₂OC(=O)Me | Cl | CH₂CN | O |
| C-9061 | H | CH(Me) | H | *c* -Pr(1-CN) | CH₂OC(=O)Me | Cl | CH₂CN | O |
| C-9062 | H | CH₂CH₂ | Me | *c* -Pr(1-CN) | CH₂OC(=O)Me | Cl | CH₂CN | O |
| C-9063 | Me | CH₂CH₂ | H | *c* -Pr(1-CN) | CH₂OC(=O)Me | Cl | CH₂CN | O |
| C-9064 | H | single bond | Me | *c* -Pr(1-CN) | CH₂OC(=O)Me | Me | CH₂CN | O |
| C-9065 | Me | single bond | H | *c* -Pr(1-CN) | CH₂OC(=O)Me | Me | CH₂CN | O |
| C-9066 | Me(R) | single bond | H | *c* -Pr(1-CN) | CH₂OC(=O)Me | Me | CH₂CN | O |
| C-9067 | Me(S) | single bond | H | *c* -Pr(1-CN) | CH₂OC(=O)Me | Me | CH₂CN | O |
| C-9068 | H | CH₂ | Me | *c* -Pr(1-CN) | CH₂OC(=O)Me | Me | CH₂CN | O |
| C-9069 | Me | CH₂ | H | *c* -Pr(1-CN) | CH₂OC(=O)Me | Me | CH₂CN | O |
| C-9070 | H | CH(Me) | H | *c* -Pr(1-CN) | CH₂OC(=O)Me | Me | CH₂CN | O |

**[Table 434]**

| Compound Number | Y | W | Z | R¹ | R² | R³ | R¹⁰ | A |
|---|---|---|---|---|---|---|---|---|
| C-9071 | H | CH₂CH₂ | Me | *c* -Pr(1-CN) | CH₂OC(=O)Me | Me | CH₂CN | O |
| C-9072 | Me | CH₂CH₂ | H | *c* -Pr(1-CN) | CH₂OC(=O)Me | Me | CH₂CN | O |
| C-9073 | H | single bond | Me | *c* -Pr(1-CN) | CH₂OC(=O)(*í* -Pr) | Cl | CH₂CN | O |
| C-9074 | Me | single bond | H | *c* -Pr(1-CN) | CH₂OC(=O)(*í* -Pr) | Cl | CH₂CN | O |
| C-9075 | Me(R) | single bond | H | *c* -Pr(1-CN) | CH₂OC(=O)(*í* -Pr) | Cl | CH₂CN | O |
| C-9076 | Me(S) | single bond | H | *c* -Pr(1-CN) | CH₂OC(=O)(*í* -Pr) | Cl | CH₂CN | O |
| C-9077 | H | CH₂ | Me | *c* -Pr(1-CN) | CH₂OC(=O)(*í* -Pr) | Cl | CH₂CN | O |
| C-9078 | Me | CH₂ | H | *c* -Pr(1-CN) | CH₂OC(=O)(*í* -Pr) | Cl | CH₂CN | O |
| C-9079 | H | CH(Me) | H | *c* -Pr(1-CN) | CH₂OC(=O)(*í* -Pr) | Cl | CH₂CN | O |
| C-9080 | H | CH₂CH₂ | Me | *c* -Pr(1-CN) | CH₂OC(=O)(*í* -Pr) | Cl | CH₂CN | O |
| C-9081 | Me | CH₂CH₂ | H | *c* -Pr(1-CN) | CH₂OC(=O)(*í* -Pr) | Cl | CH₂CN | O |
| C-9082 | H | single bond | Me | *c* -Pr(1-CN) | CH₂OC(=O)(*í* -Pr) | Me | CH₂CN | O |
| C-9083 | Me | single bond | H | *c* -Pr(1-CN) | CH₂OC(=O)(*í* -Pr) | Me | CH₂CN | O |
| C-9084 | Me(R) | single bond | H | *c* -Pr(1-CN) | CH₂OC(=O)(*í* -Pr) | Me | CH₂CN | O |
| C-9085 | Me(S) | single bond | H | *c* -Pr(1-CN) | CH₂OC(=O)(*í* -Pr) | Me | CH₂CN | O |
| C-9086 | H | CH₂ | Me | *c* -Pr(1-CN) | CH₂OC(=O)(*i* -Pr) | Me | CH₂CN | O |
| C-9087 | Me | CH₂ | H | *c* -Pr(1-CN) | CH₂OC(=O)(*í* -Pr) | Me | CH₂CN | O |
| C-9088 | H | CH(Me) | H | *c* -Pr(1-CN) | CH₂OC(=O)(*í* -Pr) | Me | CH₂CN | O |
| C-9089 | H | CH₂CH₂ | Me | *c* -Pr(1-CN) | CH₂OC(=O)(*í* -Pr) | Me | CH₂CN | O |
| C-9090 | Me | CH₂CH₂ | H | *c* -Pr(1-CN) | CH₂OC(=O)(*í* -Pr) | Me | CH₂CN | O |
| C-9091 | H | single bond | Me | *c* -Pr(1-CN) | CH₂OC(=O)(*t* -Bu) | Cl | CH₂CN | O |
| C-9092 | Me | single bond | H | *c* -Pr(1-CN) | CH₂OC(=O)(*t* -Bu) | Cl | CH₂CN | O |
| C-9093 | Me(R) | single bond | H | *c* -Pr(1-CN) | CH₂OC(=O)(*t* -Bu) | Cl | CH₂CN | O |
| C-9094 | Me(S) | single bond | H | *c* -Pr(1-CN) | CH₂OC(=O)(*t* -Bu) | Cl | CH₂CN | O |
| C-9095 | H | CH₂ | Me | *c* -Pr(1-CN) | CH₂OC(=O)(*t* -Bu) | Cl | CH₂CN | O |
| C-9096 | Me | CH₂ | H | *c* -Pr(1-CN) | CH₂OC(=O)(*t* -Bu) | Cl | CH₂CN | O |
| C-9097 | H | CH(Me) | H | *c* -Pr(1-CN) | CH₂OC(=O)(*t* -Bu) | Cl | CH₂CN | O |
| C-9098 | H | CH₂CH₂ | Me | *c* -Pr(1-CN) | CH₂OC(=O)(*t* -Bu) | Cl | CH₂CN | O |
| C-9099 | Me | CH₂CH₂ | H | *c* -Pr(1-CN) | CH₂OC(=O)(*t* -Bu) | Cl | CH₂CN | O |
| C-9100 | H | single bond | Me | *c* -Pr(1-CN) | CH₂OC(=O)(*t* -Bu) | Me | CH₂CN | O |
| C-9101 | Me | single bond | H | *c* -Pr(1-CN) | CH₂OC(=O)(*t* -Bu) | Me | CH₂CN | O |
| C-9102 | Me(R) | single bond | H | *c* -Pr(1-CN) | CH₂OC(=O)(*t* -Bu) | Me | CH₂CN | O |
| C-9103 | Me(S) | single bond | H | *c* -Pr(1-CN) | CH₂OC(=O)(*t* -Bu) | Me | CH₂CN | O |
| C-9104 | H | CH₂ | Me | *c* -Pr(1-CN) | CH₂OC(=O)(*t* -Bu) | Me | CH₂CN | O |
| C-9105 | Me | CH₂ | H | *c* -Pr(1-CN) | CH₂OC(=O)(*t* -Bu) | Me | CH₂CN | O |
| C-9106 | H | CH(Me) | H | *c* -Pr(1-CN) | CH₂OC(=O)(*t* -Bu) | Me | CH₂CN | O |
| C-9107 | H | CH₂CH₂ | Me | *c* -Pr(1-CN) | CH₂OC(=O)(*t* -Bu) | Me | CH₂CN | O |
| C-9108 | Me | CH₂CH₂ | H | *c* -Pr(1-CN) | CH₂OC(=O)(*t* -Bu) | Me | CH₂CN | O |
| C-9109 | H | single bond | Me | *c* -Pr(1-CN) | CH₂OC(=O)OEt | Cl | CH₂CN | O |
| C-9110 | Me | single bond | H | *c* -Pr(1-CN) | CH₂OC(=O)OEt | Cl | CH₂CN | O |
| C-9111 | Me(R) | single bond | H | *c* -Pr(1-CN) | CH₂OC(=O)OEt | Cl | CH₂CN | O |
| C-9112 | Me(S) | single bond | H | *c* -Pr(1-CN) | CH₂OC(=O)OEt | Cl | CH₂CN | O |
| C-9113 | H | CH₂ | Me | *c* -Pr(1-CN) | CH₂OC(=O)OEt | Cl | CH₂CN | O |
| C-9114 | Me | CH₂ | H | *c* -Pr(1-CN) | CH₂OC(=O)OEt | Cl | CH₂CN | O |

**[Table 435]**

| Compound Number | Y | W | Z | R¹ | R² | R³ | R¹⁰ | A |
|---|---|---|---|---|---|---|---|---|
| C-9115 | H | CH(Me) | H | *c*-Pr(1-CN) | CH₂OC(=O)OEt | Cl | CH₂CN | O |
| C-9116 | H | CH₂CH₂ | Me | *c*-Pr(1-CN) | CH₂OC(=O)OEt | Cl | CH₂CN | O |
| C-9117 | Me | CH₂CH₂ | H | *c*-Pr(1-CN) | CH₂OC(=O)OEt | Cl | CH₂CN | O |
| C-9118 | H | single bond | Me | *c*-Pr(1-CN) | CH₂OC(=O)OEt | Me | CH₂CN | O |
| C-9119 | Me | single bond | H | *c*-Pr(1-CN) | CH₂OC(=O)OEt | Me | CH₂CN | O |
| C-9120 | Me(R) | single bond | H | *c*-Pr(1-CN) | CH₂OC(=O)OEt | Me | CH₂CN | O |
| C-9121 | Me(S) | single bond | H | *c*-Pr(1-CN) | CH₂OC(=O)OEt | Me | CH₂CN | O |
| C-9122 | H | CH₂ | Me | *c*-Pr(1-CN) | CH₂OC(=O)OEt | Me | CH₂CN | O |
| C-9123 | Me | CH₂ | H | *c*-Pr(1-CN) | CH₂OC(=O)OEt | Me | CH₂CN | O |
| C-9124 | H | CH(Me) | H | *c*-Pr(1-CN) | CH₂OC(=O)OEt | Me | CH₂CN | O |
| C-9125 | H | CH₂CH₂ | Me | *c*-Pr(1-CN) | CH₂OC(=O)OEt | Me | CH₂CN | O |
| C-9126 | Me | CH₂CH₂ | H | *c*-Pr(1-CN) | CH₂OC(=O)OEt | Me | CH₂CN | O |
| C-9127 | H | single bond | Me | *c*-Pr(1-CN) | CH₂OC(=O)O(*i*-Pr) | Cl | CH₂CN | O |
| C-9128 | Me | single bond | H | *c*-Pr(1-CN) | CH₂OC(=O)O(*i*-Pr) | Cl | CH₂CN | O |
| C-9129 | Me(R) | single bond | H | *c*-Pr(1-CN) | CH₂OC(=O)O(*i*-Pr) | Cl | CH₂CN | O |
| C-9130 | Me(S) | single bond | H | *c*-Pr(1-CN) | CH₂OC(=O)O(*i*-Pr) | Cl | CH₂CN | O |
| C-9131 | H | CH₂ | Me | *c*-Pr(1-CN) | CH₂OC(=O)O(*i*-Pr) | Cl | CH₂CN | O |
| C-9132 | Me | CH₂ | H | *c*-Pr(1-CN) | CH₂OC(=O)O(*i*-Pr) | Cl | CH₂CN | O |
| C-9133 | H | CH(Me) | H | *c*-Pr(1-CN) | CH₂OC(=O)O(*i*-Pr) | Cl | CH₂CN | O |
| C-9134 | H | CH₂CH₂ | Me | *c*-Pr(1-CN) | CH₂OC(=O)O(*i*-Pr) | Cl | CH₂CN | O |
| C-9135 | Me | CH₂CH₂ | H | *c*-Pr(1-CN) | CH₂OC(=O)O(*i*-Pr) | Cl | CH₂CN | O |
| C-9136 | H | single bond | Me | *c*-Pr(1-CN) | CH₂OC(=O)O(*i*-Pr) | Me | CH₂CN | O |
| C-9137 | Me | single bond | H | *c*-Pr(1-CN) | CH₂OC(=O)O(*i*-Pr) | Me | CH₂CN | O |
| C-9138 | Me(R) | single bond | H | *c*-Pr(1-CN) | CH₂OC(=O)O(*i*-Pr) | Me | CH₂CN | O |
| C-9139 | Me(S) | single bond | H | *c*-Pr(1-CN) | CH₂OC(=O)O(*i*-Pr) | Me | CH₂CN | O |
| C-9140 | H | CH₂ | Me | *c*-Pr(1-CN) | CH₂OC(=O)O(*i*-Pr) | Me | CH₂CN | O |
| C-9141 | Me | CH₂ | H | *c*-Pr(1-CN) | CH₂OC(=O)O(*i*-Pr) | Me | CH₂CN | O |
| C-9142 | H | CH(Me) | H | *c*-Pr(1-CN) | CH₂OC(=O)O(*i*-Pr) | Me | CH₂CN | O |
| C-9143 | H | CH₂CH₂ | Me | *c*-Pr(1-CN) | CH₂OC(=O)O(*i*-Pr) | Me | CH₂CN | O |
| C-9144 | Me | CH₂CH₂ | H | *c*-Pr(1-CN) | CH₂OC(=O)O(*i*-Pr) | Me | CH₂CN | O |
| C-9145 | H | single bond | Me | *c*-Pr(1-CN) | CH₂N(Me)C(=O)OMe | Cl | CH₂CN | O |
| C-9146 | Me | single bond | H | *c*-Pr(1-CN) | CH₂N(Me)C(=O)OMe | Cl | CH₂CN | O |
| C-9147 | Me(R) | single bond | H | *c*-Pr(1-CN) | CH₂N(Me)C(=O)OMe | Cl | CH₂CN | O |
| C-9148 | Me(S) | single bond | H | *c*-Pr(1-CN) | CH₂N(Me)C(=O)OMe | Cl | CH₂CN | O |
| C-9149 | H | CH₂ | Me | *c*-Pr(1-CN) | CH₂N(Me)C(=O)OMe | Cl | CH₂CN | O |
| C-9150 | Me | CH₂ | H | *c*-Pr(1-CN) | CH₂N(Me)C(=O)OMe | Cl | CH₂CN | O |
| C-9151 | H | CH(Me) | H | *c*-Pr(1-CN) | CH₂N(Me)C(=O)OMe | Cl | CH₂CN | O |
| C-9152 | H | CH₂CH₂ | Me | *c*-Pr(1-CN) | CH₂N(Me)C(=O)OMe | Cl | CH₂CN | O |
| C-9153 | Me | CH₂CH₂ | H | *c*-Pr(1-CN) | CH₂N(Me)C(=O)OMe | Cl | CH₂CN | O |
| C-9154 | H | single bond | Me | *c*-Pr(1-CN) | CH₂N(Me)C(=O)OMe | Me | CH₂CN | O |
| C-9155 | Me | single bond | H | *c*-Pr(1-CN) | CH₂N(Me)C(=O)OMe | Me | CH₂CN | O |
| C-9156 | Me(R) | single bond | H | *c*-Pr(1-CN) | CH₂N(Me)C(=O)OMe | Me | CH₂CN | O |
| C-9157 | Me(S) | single bond | H | *c*-Pr(1-CN) | CH₂N(Me)C(=O)OMe | Me | CH₂CN | O |
| C-9158 | H | CH₂ | Me | *c*-Pr(1-CN) | CH₂N(Me)C(=O)OMe | Me | CH₂CN | O |

**[Table 436]**

| Compound Number | Y | W | Z | R¹ | R² | R³ | R¹⁰ | A |
|---|---|---|---|---|---|---|---|---|
| C-9159 | Me | CH₂ | H | *c*-Pr(1-CN) | CH₂N(Me)C(=O)OMe | Me | CH₂CN | O |
| C-9160 | H | CH(Me) | H | *c*-Pr(1-CN) | CH₂N(Me)C(=O)OMe | Me | CH₂CN | O |
| C-9161 | H | CH₂CH₂ | Me | *c*-Pr(1-CN) | CH₂N(Me)C(=O)OMe | Me | CH₂CN | O |
| C-9162 | Me | CH₂CH₂ | H | *c*-Pr(1-CN) | CH₂N(Me)C(=O)OMe | Me | CH₂CN | O |
| C-9163 | H | single bond | Me | *c*-Pr(1-CN) | C(=O)OCH₂CH=CH₂ | Cl | C(=O)Me | O |
| C-9164 | Me | single bond | H | *c*-Pr(1-CN) | C(=O)OCH₂CH=CH₂ | Cl | C(=O)Me | O |
| C-9165 | Me(R) | single bond | H | *c*-Pr(1-CN) | C(=O)OCH₂CH=CH₂ | Cl | C(=O)Me | O |
| C-9166 | Me(S) | single bond | H | *c*-Pr(1-CN) | C(=O)OCH₂CH=CH₂ | Cl | C(=O)Me | O |
| C-9167 | H | CH₂ | Me | *c*-Pr(1-CN) | C(=O)OCH₂CH=CH₂ | Cl | C(=O)Me | O |
| C-9168 | Me | CH₂ | H | *c*-Pr(1-CN) | C(=O)OCH₂CH=CH₂ | Cl | C(=O)Me | O |
| C-9169 | H | CH(Me) | H | *c*-Pr(1-CN) | C(=O)OCH₂CH=CH₂ | Cl | C(=O)Me | O |
| C-9170 | H | CH₂CH₂ | Me | *c*-Pr(1-CN) | C(=O)OCH₂CH=CH₂ | Cl | C(=O)Me | O |
| C-9171 | Me | CH₂CH₂ | H | *c*-Pr(1-CN) | C(=O)OCH₂CH=CH₂ | Cl | C(=O)Me | O |
| C-9172 | H | single bond | Me | *c*-Pr(1-CN) | C(=O)OCH₂CH=CH₂ | Me | C(=O)Me | O |
| C-9173 | Me | single bond | H | *c*-Pr(1-CN) | C(=O)OCH₂CH=CH₂ | Me | C(=O)Me | O |
| C-9174 | Me(R) | single bond | H | *c*-Pr(1-CN) | C(=O)OCH₂CH=CH₂ | Me | C(=O)Me | O |
| C-9175 | Me(S) | single bond | H | *c*-Pr(1-CN) | C(=O)OCH₂CH=CH₂ | Me | C(=O)Me | O |
| C-9176 | H | CH₂ | Me | *c*-Pr(1-CN) | C(=O)OCH₂CH=CH₂ | Me | C(=O)Me | O |
| C-9177 | Me | CH₂ | H | *c*-Pr(1-CN) | C(=O)OCH₂CH=CH₂ | Me | C(=O)Me | O |
| C-9178 | H | CH(Me) | H | *c*-Pr(1-CN) | C(=O)OCH₂CH=CH₂ | Me | C(=O)Me | O |
| C-9179 | H | CH₂CH₂ | Me | *c*-Pr(1-CN) | C(=O)OCH₂CH=CH₂ | Me | C(=O)Me | O |
| C-9180 | Me | CH₂CH₂ | H | *c*-Pr(1-CN) | C(=O)OCH₂CH=CH₂ | Me | C(=O)Me | O |
| C-9181 | H | single bond | Me | *c*-Pr(1-CN) | C(=O)OCH₂CCl₃ | Cl | C(=O)Me | O |
| C-9182 | Me | single bond | H | *c*-Pr(1-CN) | C(=O)OCH₂CCl₃ | Cl | C(=O)Me | O |
| C-9183 | Me(R) | single bond | H | *c*-Pr(1-CN) | C(=O)OCH₂CCl₃ | Cl | C(=O)Me | O |
| C-9184 | Me(S) | single bond | H | *c*-Pr(1-CN) | C(=O)OCH₂CCl₃ | Cl | C(=O)Me | O |
| C-9185 | H | CH₂ | Me | *c*-Pr(1-CN) | C(=O)OCH₂CCl₃ | Cl | C(=O)Me | O |
| C-9186 | Me | CH₂ | H | *c*-Pr(1-CN) | C(=O)OCH₂CCl₃ | Cl | C(=O)Me | O |
| C-9187 | H | CH(Me) | H | *c*-Pr(1-CN) | C(=O)OCH₂CCl₃ | Cl | C(=O)Me | O |
| C-9188 | H | CH₂CH₂ | Me | *c*-Pr(1-CN) | C(=O)OCH₂CCl₃ | Cl | C(=O)Me | O |
| C-9189 | Me | CH₂CH₂ | H | *c*-Pr(1-CN) | C(=O)OCH₂CCl₃ | Cl | C(=O)Me | O |
| C-9190 | H | single bond | Me | *c*-Pr(1-CN) | C(=O)OCH₂CCl₃ | Me | C(=O)Me | O |
| C-9191 | Me | single bond | H | *c*-Pr(1-CN) | C(=O)OCH₂CCl₃ | Me | C(=O)Me | O |
| C-9192 | Me(R) | single bond | H | *c*-Pr(1-CN) | C(=O)OCH₂CCl₃ | Me | C(=O)Me | O |
| C-9193 | Me(S) | single bond | H | *c*-Pr(1-CN) | C(=O)OCH₂CCl₃ | Me | C(=O)Me | O |
| C-9194 | H | CH₂ | Me | *c*-Pr(1-CN) | C(=O)OCH₂CCl₃ | Me | C(=O)Me | O |
| C-9195 | Me | CH₂ | H | *c*-Pr(1-CN) | C(=O)OCH₂CCl₃ | Me | C(=O)Me | O |
| C-9196 | H | CH(Me) | H | *c*-Pr(1-CN) | C(=O)OCH₂CCl₃ | Me | C(=O)Me | O |
| C-9197 | H | CH₂CH₂ | Me | *c*-Pr(1-CN) | C(=O)OCH₂CCl₃ | Me | C(=O)Me | O |
| C-9198 | Me | CH₂CH₂ | H | *c*-Pr(1-CN) | C(=O)OCH₂CCl₃ | Me | C(=O)Me | O |
| C-9199 | H | single bond | Me | *c*-Pr(1-CN) | C(=O)N(Me)₂ | Cl | C(=O)Me | O |
| C-9200 | Me | single bond | H | *c*-Pr(1-CN) | C(=O)N(Me)₂ | Cl | C(=O)Me | O |
| C-9201 | Me(R) | single bond | H | *c*-Pr(1-CN) | C(=O)N(Me)₂ | Cl | C(=O)Me | O |
| C-9202 | Me(S) | single bond | H | *c*-Pr(1-CN) | C(=O)N(Me)₂ | Cl | C(=O)Me | O |

**[Table 437]**

| Compound Number | Y | W | Z | R¹ | R² | R³ | R¹⁰ | A |
|---|---|---|---|---|---|---|---|---|
| C-9203 | H | CH₂ | Me | *c*-Pr(1-CN) | C(=O)N(Me)₂ | Cl | C(=O)Me | O |
| C-9204 | Me | CH₂ | H | *c*-Pr(1-CN) | C(=O)N(Me)₂ | Cl | C(=O)Me | O |
| C-9205 | H | CH(Me) | H | *c*-Pr(1-CN) | C(=O)N(Me)₂ | Cl | C(=O)Me | O |
| C-9206 | H | CH₂CH₂ | Me | *c*-Pr(1-CN) | C(=O)N(Me)₂ | Cl | C(=O)Me | O |
| C-9207 | Me | CH₂CH₂ | H | *c*-Pr(1-CN) | C(=O)N(Me)₂ | Cl | C(=O)Me | O |
| C-9208 | H | single bond | Me | *c*-Pr(1-CN) | C(=O)N(Me)₂ | Me | C(=O)Me | O |
| C-9209 | Me | single bond | H | *c*-Pr(1-CN) | C(=O)N(Me)₂ | Me | C(=O)Me | O |
| C-9210 | Me(R) | single bond | H | *c*-Pr(1-CN) | C(=O)N(Me)₂ | Me | C(=O)Me | O |
| C-9211 | Me(S) | single bond | H | *c*-Pr(1-CN) | C(=O)N(Me)₂ | Me | C(=O)Me | O |
| C-9212 | H | CH₂ | Me | *c*-Pr(1-CN) | C(=O)N(Me)₂ | Me | C(=O)Me | O |
| C-9213 | Me | CH₂ | H | *c*-Pr(1-CN) | C(=O)N(Me)₂ | Me | C(=O)Me | O |
| C-9214 | H | CH(Me) | H | *c*-Pr(1-CN) | C(=O)N(Me)₂ | Me | C(=O)Me | O |
| C-9215 | H | CH₂CH₂ | Me | *c*-Pr(1-CN) | C(=O)N(Me)₂ | Me | C(=O)Me | O |
| C-9216 | Me | CH₂CH₂ | H | *c*-Pr(1-CN) | C(=O)N(Me)₂ | Me | C(=O)Me | O |
| C-9217 | H | single bond | Me | *c*-Pr(1-CN) | CH₂OC(=O)Me | Cl | C(=O)Me | O |
| C-9218 | Me | single bond | H | *c*-Pr(1-CN) | CH₂OC(=O)Me | Cl | C(=O)Me | O |
| C-9219 | Me(R) | single bond | H | *c*-Pr(1-CN) | CH₂OC(=O)Me | Cl | C(=O)Me | O |
| C-9220 | Me(S) | single bond | H | *c*-Pr(1-CN) | CH₂OC(=O)Me | Cl | C(=O)Me | O |
| C-9221 | H | CH₂ | Me | *c*-Pr(1-CN) | CH₂OC(=O)Me | Cl | C(=O)Me | O |
| C-9222 | Me | CH₂ | H | *c*-Pr(1-CN) | CH₂OC(=O)Me | Cl | C(=O)Me | O |
| C-9223 | H | CH(Me) | H | *c*-Pr(1-CN) | CH₂OC(=O)Me | Cl | C(=O)Me | O |
| C-9224 | H | CH₂CH₂ | Me | *c*-Pr(1-CN) | CH₂OC(=O)Me | Cl | C(=O)Me | O |
| C-9225 | Me | CH₂CH₂ | H | *c*-Pr(1-CN) | CH₂OC(=O)Me | Cl | C(=O)Me | O |
| C-9226 | H | single bond | Me | *c*-Pr(1-CN) | CH₂OC(=O)Me | Me | C(=O)Me | O |
| C-9227 | Me | single bond | H | *c*-Pr(1-CN) | CH₂OC(=O)Me | Me | C(=O)Me | O |
| C-9228 | Me(R) | single bond | H | *c*-Pr(1-CN) | CH₂OC(=O)Me | Me | C(=O)Me | O |
| C-9229 | Me(S) | single bond | H | *c*-Pr(1-CN) | CH₂OC(=O)Me | Me | C(=O)Me | O |
| C-9230 | H | CH₂ | Me | *c*-Pr(1-CN) | CH₂OC(=O)Me | Me | C(=O)Me | O |
| C-9231 | Me | CH₂ | H | *c*-Pr(1-CN) | CH₂OC(=O)Me | Me | C(=O)Me | O |
| C-9232 | H | CH(Me) | H | *c*-Pr(1-CN) | CH₂OC(=O)Me | Me | C(=O)Me | O |
| C-9233 | H | CH₂CH₂ | Me | *c*-Pr(1-CN) | CH₂OC(=O)Me | Me | C(=O)Me | O |
| C-9234 | Me | CH₂CH₂ | H | *c*-Pr(1-CN) | CH₂OC(=O)Me | Me | C(=O)Me | O |
| C-9235 | H | single bond | Me | *c*-Pr(1-CN) | CH₂OC(=O)(*i*-Pr) | Cl | C(=O)Me | O |
| C-9236 | Me | single bond | H | *c*-Pr(1-CN) | CH₂OC(=O)(*i*-Pr) | Cl | C(=O)Me | O |
| C-9237 | Me(R) | single bond | H | *c*-Pr(1-CN) | CH₂OC(=O)(*i*-Pr) | Cl | C(=O)Me | O |
| C-9238 | Me(S) | single bond | H | *c*-Pr(1-CN) | CH₂OC(=O)(*i*-Pr) | Cl | C(=O)Me | O |
| C-9239 | H | CH₂ | Me | *c*-Pr(1-CN) | CH₂OC(=O)(*i*-Pr) | Cl | C(=O)Me | O |
| C-9240 | Me | CH₂ | H | *c*-Pr(1-CN) | CH₂OC(=O)(*i*-Pr) | Cl | C(=O)Me | O |
| C-9241 | H | CH(Me) | H | *c*-Pr(1-CN) | CH₂OC(=O)(*i*-Pr) | Cl | C(=O)Me | O |
| C-9242 | H | CH₂CH₂ | Me | *c*-Pr(1-CN) | CH₂OC(=O)(*i*-Pr) | Cl | C(=O)Me | O |
| C-9243 | Me | CH₂CH₂ | H | *c*-Pr(1-CN) | CH₂OC(=O)(*i*-Pr) | Cl | C(=O)Me | O |
| C-9244 | H | single bond | Me | *c*-Pr(1-CN) | CH₂OC(=O)(*i*-Pr) | Me | C(=O)Me | O |
| C-9245 | Me | single bond | H | *c*-Pr(1-CN) | CH₂OC(=O)(*i*-Pr) | Me | C(=O)Me | O |
| C-9246 | Me(R) | single bond | H | *c*-Pr(1-CN) | CH₂OC(=O)(*i*-Pr) | Me | C(=O)Me | O |

**[Table 438]**

| Compound Number | Y | W | Z | R¹ | R² | R³ | R¹⁰ | A |
|---|---|---|---|---|---|---|---|---|
| C-9247 | Me(S) | single bond | H | *c*-Pr(1-CN) | CH₂OC(=O)(*i*-Pr) | Me | C(=O)Me | O |
| C-9248 | H | CH₂ | Me | *c*-Pr(1-CN) | CH₂OC(=O)(*i*-Pr) | Me | C(=O)Me | O |
| C-9249 | Me | CH₂ | H | *c*-Pr(1-CN) | CH₂OC(=O)(*i*-Pr) | Me | C(=O)Me | O |
| C-9250 | H | CH(Me) | H | *c*-Pr(1-CN) | CH₂OC(=O)(*i*-Pr) | Me | C(=O)Me | O |
| C-9251 | H | CH₂CH₂ | Me | *c*-Pr(1-CN) | CH₂OC(=O)(*i*-Pr) | Me | C(=O)Me | O |
| C-9252 | Me | CH₂CH₂ | H | *c*-Pr(1-CN) | CH₂OC(=O)(*i*-Pr) | Me | C(=O)Me | O |
| C-9253 | H | single bond | Me | *c*-Pr(1-CN) | CH₂OC(=O)(*t*-Bu) | Cl | C(=O)Me | O |
| C-9254 | Me | single bond | H | *c*-Pr(1-CN) | CH₂OC(=O)(*t*-Bu) | Cl | C(=O)Me | O |
| C-9255 | Me(R) | single bond | H | *c*-Pr(1-CN) | CH₂OC(=O)(*t*-Bu) | Cl | C(=O)Me | O |
| C-9256 | Me(S) | single bond | H | *c*-Pr(1-CN) | CH₂OC(=O)(*t*-Bu) | Cl | C(=O)Me | O |
| C-9257 | H | CH₂ | Me | *c*-Pr(1-CN) | CH₂OC(=O)(*t*-Bu) | Cl | C(=O)Me | O |
| C-9258 | Me | CH₂ | H | *c*-Pr(1-CN) | CH₂OC(=O)(*t*-Bu) | Cl | C(=O)Me | O |
| C-9259 | H | CH(Me) | H | *c*-Pr(1-CN) | CH₂OC(=O)(*t*-Bu) | Cl | C(=O)Me | O |
| C-9260 | H | CH₂CH₂ | Me | *c*-Pr(1-CN) | CH₂OC(=O)(*t*-Bu) | Cl | C(=O)Me | O |
| C-9261 | Me | CH₂CH₂ | H | *c*-Pr(1-CN) | CH₂OC(=O)(*t*-Bu) | Cl | C(=O)Me | O |
| C-9262 | H | single bond | Me | *c*-Pr(1-CN) | CH₂OC(=O)(*t*-Bu) | Me | C(=O)Me | O |
| C-9263 | Me | single bond | H | *c*-Pr(1-CN) | CH₂OC(=O)(*t*-Bu) | Me | C(=O)Me | O |
| C-9264 | Me(R) | single bond | H | *c*-Pr(1-CN) | CH₂OC(=O)(*t*-Bu) | Me | C(=O)Me | O |
| C-9265 | Me(S) | single bond | H | *c*-Pr(1-CN) | CH₂OC(=O)(*t*-Bu) | Me | C(=O)Me | O |
| C-9266 | H | CH₂ | Me | *c*-Pr(1-CN) | CH₂OC(=O)(*t*-Bu) | Me | C(=O)Me | O |
| C-9267 | Me | CH₂ | H | *c*-Pr(1-CN) | CH₂OC(=O)(*t*-Bu) | Me | C(=O)Me | O |
| C-9268 | H | CH(Me) | H | *c*-Pr(1-CN) | CH₂OC(=O)(*t*-Bu) | Me | C(=O)Me | O |
| C-9269 | H | CH₂CH₂ | Me | *c*-Pr(1-CN) | CH₂OC(=O)(*t*-Bu) | Me | C(=O)Me | O |
| C-9270 | Me | CH₂CH₂ | H | *c*-Pr(1-CN) | CH₂OC(=O)(*t*-Bu) | Me | C(=O)Me | O |
| C-9271 | H | single bond | Me | *c*-Pr(1-CN) | CH₂OC(=O)OEt | Cl | C(=O)Me | O |
| C-9272 | Me | single bond | H | *c*-Pr(1-CN) | CH₂OC(=O)OEt | Cl | C(=O)Me | O |
| C-9273 | Me(R) | single bond | H | *c*-Pr(1-CN) | CH₂OC(=O)OEt | Cl | C(=O)Me | O |
| C-9274 | Me(S) | single bond | H | *c*-Pr(1-CN) | CH₂OC(=O)OEt | Cl | C(=O)Me | O |
| C-9275 | H | CH₂ | Me | *c*-Pr(1-CN) | CH₂OC(=O)OEt | Cl | C(=O)Me | O |
| C-9276 | Me | CH₂ | H | *c*-Pr(1-CN) | CH₂OC(=O)OEt | Cl | C(=O)Me | O |
| C-9277 | H | CH(Me) | H | *c*-Pr(1-CN) | CH₂OC(=O)OEt | Cl | C(=O)Me | O |
| C-9278 | H | CH₂CH₂ | Me | *c*-Pr(1-CN) | CH₂OC(=O)OEt | Cl | C(=O)Me | O |
| C-9279 | Me | CH₂CH₂ | H | *c*-Pr(1-CN) | CH₂OC(=O)OEt | Cl | C(=O)Me | O |
| C-9280 | H | single bond | Me | *c*-Pr(1-CN) | CH₂OC(=O)OEt | Me | C(=O)Me | O |
| C-9281 | Me | single bond | H | *c*-Pr(1-CN) | CH₂OC(=O)OEt | Me | C(=O)Me | O |
| C-9282 | Me(R) | single bond | H | *c*-Pr(1-CN) | CH₂OC(=O)OEt | Me | C(=O)Me | O |
| C-9283 | Me(S) | single bond | H | *c*-Pr(1-CN) | CH₂OC(=O)OEt | Me | C(=O)Me | O |
| C-9284 | H | CH₂ | Me | *c*-Pr(1-CN) | CH₂OC(=O)OEt | Me | C(=O)Me | O |
| C-9285 | Me | CH₂ | H | *c*-Pr(1-CN) | CH₂OC(=O)OEt | Me | C(=O)Me | O |
| C-9286 | H | CH(Me) | H | *c*-Pr(1-CN) | CH₂OC(=O)OEt | Me | C(=O)Me | O |
| C-9287 | H | CH₂CH₂ | Me | *c*-Pr(1-CN) | CH₂OC(=O)OEt | Me | C(=O)Me | O |
| C-9288 | Me | CH₂CH₂ | H | *c*-Pr(1-CN) | CH₂OC(=O)OEt | Me | C(=O)Me | O |
| C-9289 | H | single bond | Me | *c*-Pr(1-CN) | CH₂OC(=O)O(*i*-Pr) | Cl | C(=O)Me | O |
| C-9290 | Me | single bond | H | *c*-Pr(1-CN) | CH₂OC(=O)O(*i*-Pr) | Cl | C(=O)Me | O |

**[Table 439]**

| Compound Number | Y | W | Z | R¹ | R² | R³ | R¹⁰ | A |
|---|---|---|---|---|---|---|---|---|
| C-9291 | Me(R) | single bond | H | *c*-Pr(1-CN) | CH₂OC(=O)O(*i*-Pr) | Cl | C(=O)Me | O |
| C-9292 | Me(S) | single bond | H | *c*-Pr(1-CN) | CH₂OC(=O)O(*i*-Pr) | Cl | C(=O)Me | O |
| C-9293 | H | CH₂ | Me | *c*-Pr(1-CN) | CH₂OC(=O)O(*i*-Pr) | Cl | C(=O)Me | O |
| C-9294 | Me | CH₂ | H | *c*-Pr(1-CN) | CH₂OC(=O)O(*i*-Pr) | Cl | C(=O)Me | O |
| C-9295 | H | CH(Me) | H | *c*-Pr(1-CN) | CH₂OC(=O)O(*i*-Pr) | Cl | C(=O)Me | O |
| C-9296 | H | CH₂CH₂ | Me | *c*-Pr(1-CN) | CH₂OC(=O)O(*i*-Pr) | Cl | C(=O)Me | O |
| C-9297 | Me | CH₂CH₂ | H | *c*-Pr(1-CN) | CH₂OC(=O)O(*i*-Pr) | Cl | C(=O)Me | O |
| C-9298 | H | single bond | Me | *c*-Pr(1-CN) | CH₂OC(=O)O(*i*-Pr) | Me | C(=O)Me | O |
| C-9299 | Me | single bond | H | *c*-Pr(1-CN) | CH₂OC(=O)O(*i*-Pr) | Me | C(=O)Me | O |
| C-9300 | Me(R) | single bond | H | *c*-Pr(1-CN) | CH₂OC(=O)O(*i*-Pr) | Me | C(=O)Me | O |
| C-9301 | Me(S) | single bond | H | *c*-Pr(1-CN) | CH₂OC(=O)O(*i*-Pr) | Me | C(=O)Me | O |
| C-9302 | H | CH₂ | Me | *c*-Pr(1-CN) | CH₂OC(=O)O(*i*-Pr) | Me | C(=O)Me | O |
| C-9303 | Me | CH₂ | H | *c*-Pr(1-CN) | CH₂OC(=O)O(*i*-Pr) | Me | C(=O)Me | O |
| C-9304 | H | CH(Me) | H | *c*-Pr(1-CN) | CH₂OC(=O)O(*i*-Pr) | Me | C(=O)Me | O |
| C-9305 | H | CH₂CH₂ | Me | *c*-Pr(1-CN) | CH₂OC(=O)O(*i*-Pr) | Me | C(=O)Me | O |
| C-9306 | Me | CH₂CH₂ | H | *c*-Pr(1-CN) | CH₂OC(=O)O(*i*-Pr) | Me | C(=O)Me | O |
| C-9307 | H | single bond | Me | *c*-Pr(1-CN) | CH₂N(Me)C(=O)OMe | Cl | C(=O)Me | O |
| C-9308 | Me | single bond | H | *c*-Pr(1-CN) | CH₂N(Me)C(=O)OMe | Cl | C(=O)Me | O |
| C-9309 | Me(R) | single bond | H | *c*-Pr(1-CN) | CH₂N(Me)C(=O)OMe | Cl | C(=O)Me | O |
| C-9310 | Me(S) | single bond | H | *c*-Pr(1-CN) | CH₂N(Me)C(=O)OMe | Cl | C(=O)Me | O |
| C-9311 | H | CH₂ | Me | *c*-Pr(1-CN) | CH₂N(Me)C(=O)OMe | Cl | C(=O)Me | O |
| C-9312 | Me | CH₂ | H | *c*-Pr(1-CN) | CH₂N(Me)C(=O)OMe | Cl | C(=O)Me | O |
| C-9313 | H | CH(Me) | H | *c*-Pr(1-CN) | CH₂N(Me)C(=O)OMe | Cl | C(=O)Me | O |
| C-9314 | H | CH₂CH₂ | Me | *c*-Pr(1-CN) | CH₂N(Me)C(=O)OMe | Cl | C(=O)Me | O |
| C-9315 | Me | CH₂CH₂ | H | *c*-Pr(1-CN) | CH₂N(Me)C(=O)OMe | Cl | C(=O)Me | O |
| C-9316 | H | single bond | Me | *c*-Pr(1-CN) | CH₂N(Me)C(=O)OMe | Me | C(=O)Me | O |
| C-9317 | Me | single bond | H | *c*-Pr(1-CN) | CH₂N(Me)C(=O)OMe | Me | C(=O)Me | O |
| C-9318 | Me(R) | single bond | H | *c*-Pr(1-CN) | CH₂N(Me)C(=O)OMe | Me | C(=O)Me | O |
| C-9319 | Me(S) | single bond | H | *c*-Pr(1-CN) | CH₂N(Me)C(=O)OMe | Me | C(=O)Me | O |
| C-9320 | H | CH₂ | Me | *c*-Pr(1-CN) | CH₂N(Me)C(=O)OMe | Me | C(=O)Me | O |
| C-9321 | Me | CH₂ | H | *c*-Pr(1-CN) | CH₂N(Me)C(=O)OMe | Me | C(=O)Me | O |
| C-9322 | H | CH(Me) | H | *c*-Pr(1-CN) | CH₂N(Me)C(=O)OMe | Me | C(=O)Me | O |
| C-9323 | H | CH₂CH₂ | Me | *c*-Pr(1-CN) | CH₂N(Me)C(=O)OMe | Me | C(=O)Me | O |
| C-9324 | Me | CH₂CH₂ | H | *c*-Pr(1-CN) | CH₂N(Me)C(=O)OMe | Me | C(=O)Me | O |

**[Table 440]**

| 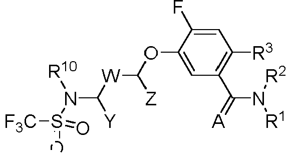 | | | | | | | | |
|---|---|---|---|---|---|---|---|---|
| Compound Number | Y | W | Z | R¹ | R² | R³ | R¹⁰ | A |
| C-9325 | H | single bond | Me | CH₂CF₃ | H | Cl | *c*-Pr | O |
| C-9326 | Me | single bond | H | CH₂CF₃ | H | Cl | *c*-Pr | O |
| C-9327 | Me(R) | single bond | H | CH₂CF₃ | H | Cl | *c*-Pr | O |
| C-9328 | Me(S) | single bond | H | CH₂CF₃ | H | Cl | *c*-Pr | O |
| C-9329 | H | single bond | Me | Et | H | Cl | *c*-Pr | O |
| C-9330 | Me | single bond | H | Et | H | Cl | *c*-Pr | O |
| C-9331 | Me(R) | single bond | H | Et | H | Cl | *c*-Pr | O |
| C-9332 | Me(S) | single bond | H | Et | H | Cl | *c*-Pr | O |
| C-9333 | H | single bond | Me | c-Pr | H | Cl | *c*-Pr | O |
| C-9334 | Me | single bond | H | c-Pr | H | Cl | *c*-Pr | O |
| C-9335 | Me(R) | single bond | H | c-Pr | H | Cl | *c*-Pr | O |
| C-9336 | Me(S) | single bond | H | c-Pr | H | Cl | *c*-Pr | O |
| C-9337 | H | single bond | Me | c-Pr(1-CN) | H | Cl | *c*-Pr | O |
| C-9338 | Me | single bond | H | c-Pr(1-CN) | H | Cl | *c*-Pr | O |
| C-9339 | Me(R) | single bond | H | c-Pr(1-CN) | H | Cl | *c*-Pr | O |
| C-9340 | Me(S) | single bond | H | c-Pr(1-CN) | H | Cl | *c*-Pr | O |
| C-9341 | H | single bond | Me | CH₂CF₃ | H | Me | *c*-Pr | O |
| C-9342 | Me | single bond | H | CH₂CF₃ | H | Me | *c*-Pr | O |
| C-9343 | Me(R) | single bond | H | CH₂CF₃ | H | Me | *c*-Pr | O |
| C-9344 | Me(S) | single bond | H | CH₂CF₃ | H | Me | *c*-Pr | O |
| C-9345 | H | single bond | Me | Et | H | Me | *c*-Pr | O |
| C-9346 | Me | single bond | H | Et | H | Me | *c*-Pr | O |
| C-9347 | Me(R) | single bond | H | Et | H | Me | *c*-Pr | O |
| C-9348 | Me(S) | single bond | H | Et | H | Me | *c*-Pr | O |
| C-9349 | H | single bond | Me | c-Pr | H | Me | *c*-Pr | O |
| C-9350 | Me | single bond | H | c-Pr | H | Me | *c*-Pr | O |
| C-9351 | Me(R) | single bond | H | c-Pr | H | Me | *c*-Pr | O |
| C-9352 | Me(S) | single bond | H | c-Pr | H | Me | *c*-Pr | O |
| C-9353 | H | single bond | Me | c-Pr(1-CN) | H | Me | *c*-Pr | O |
| C-9354 | Me | single bond | H | c-Pr(1-CN) | H | Me | *c*-Pr | O |
| C-9355 | Me(R) | single bond | H | c-Pr(1-CN) | H | Me | *c*-Pr | O |
| C-9356 | Me(S) | single bond | H | c-Pr(1-CN) | H | Me | *c*-Pr | O |
| C-9357 | H | single bond | Me | CH₂CF₃ | H | Cl | OMe | O |
| C-9358 | Me | single bond | H | CH₂CF₃ | H | Cl | OMe | O |
| C-9359 | Me(R) | single bond | H | CH₂CF₃ | H | Cl | OMe | O |
| C-9360 | Me(S) | single bond | H | CH₂CF₃ | H | Cl | OMe | O |
| C-9361 | H | single bond | Me | Et | H | Cl | OMe | O |
| C-9362 | Me | single bond | H | Et | H | Cl | OMe | O |

**[Table 441]**

| Compound Number | Y | W | Z | R¹ | R² | R³ | R¹⁰ | A |
|---|---|---|---|---|---|---|---|---|
| C-9363 | Me(R) | single bond | H | Et | H | Cl | OMe | O |
| C-9364 | Me(S) | single bond | H | Et | H | Cl | OMe | O |
| C-9365 | H | single bond | Me | c-Pr | H | Cl | OMe | O |
| C-9366 | Me | single bond | H | c-Pr | H | Cl | OMe | O |
| C-9367 | Me(R) | single bond | H | c-Pr | H | Cl | OMe | O |
| C-9368 | Me(S) | single bond | H | c-Pr | H | Cl | OMe | O |
| C-9369 | H | single bond | Me | c-Pr(1-CN) | H | Cl | OMe | O |
| C-9370 | Me | single bond | H | c-Pr(1-CN) | H | Cl | OMe | O |
| C-9371 | Me(R) | single bond | H | c-Pr(1-CN) | H | Cl | OMe | O |
| C-9372 | Me(S) | single bond | H | c-Pr(1-CN) | H | Cl | OMe | O |
| C-9373 | H | single bond | Me | CH₂CF₃ | H | Me | OMe | O |
| C-9374 | Me | single bond | H | CH₂CF₃ | H | Me | OMe | O |
| C-9375 | Me(R) | single bond | H | CH₂CF₃ | H | Me | OMe | O |
| C-9376 | Me(S) | single bond | H | CH₂CF₃ | H | Me | OMe | O |
| C-9377 | H | single bond | Me | Et | H | Me | OMe | O |
| C-9378 | Me | single bond | H | Et | H | Me | OMe | O |
| C-9379 | Me(R) | single bond | H | Et | H | Me | OMe | O |
| C-9380 | Me(S) | single bond | H | Et | H | Me | OMe | O |
| C-9381 | H | single bond | Me | c-Pr | H | Me | OMe | O |
| C-9382 | Me | single bond | H | c-Pr | H | Me | OMe | O |
| C-9383 | Me(R) | single bond | H | c-Pr | H | Me | OMe | O |
| C-9384 | Me(S) | single bond | H | c-Pr | H | Me | OMe | O |
| C-9385 | H | single bond | Me | c-Pr(1-CN) | H | Me | OMe | O |
| C-9386 | Me | single bond | H | c-Pr(1-CN) | H | Me | OMe | O |
| C-9387 | Me(R) | single bond | H | c-Pr(1-CN) | H | Me | OMe | O |
| C-9388 | Me(S) | single bond | H | c-Pr(1-CN) | H | Me | OMe | O |
| C-9389 | H | single bond | Me | CH₂CF₃ | H | Cl | C(=O)(*i*-Pr) | O |
| C-9390 | Me | single bond | H | CH₂CF₃ | H | Cl | C(=O)(*i*-Pr) | O |
| C-9391 | Me(R) | single bond | H | CH₂CF₃ | H | Cl | C(=O)(*i*-Pr) | O |
| C-9392 | Me(S) | single bond | H | CH₂CF₃ | H | Cl | C(=O)(*i*-Pr) | O |
| C-9393 | H | single bond | Me | Et | H | Cl | C(=O)(*i*-Pr) | O |
| C-9394 | Me | single bond | H | Et | H | Cl | C(=O)(*i*-Pr) | O |
| C-9395 | Me(R) | single bond | H | Et | H | Cl | C(=O)(*i*-Pr) | O |
| C-9396 | Me(S) | single bond | H | Et | H | Cl | C(=O)(*i*-Pr) | O |
| C-9397 | H | single bond | Me | c-Pr | H | Cl | C(=O)(*i*-Pr) | O |
| C-9398 | Me | single bond | H | c-Pr | H | Cl | C(=O)(*i*-Pr) | O |
| C-9399 | Me(R) | single bond | H | c-Pr | H | Cl | C(=O)(*i*-Pr) | O |
| C-9400 | Me(S) | single bond | H | c-Pr | H | Cl | C(=O)(*i*-Pᵣ) | O |
| C-9401 | H | single bond | Me | c-Pr(1-CN) | H | Cl | C(=O)(*i*-Pr) | O |
| C-9402 | Me | single bond | H | c-Pr(1-CN) | H | Cl | C(=O)(*i*-Pr) | O |
| C-9403 | Me(R) | single bond | H | c-Pr(1-CN) | H | Cl | C(=O)(*i*-Pr) | O |
| C-9404 | Me(S) | single bond | H | c-Pr(1-CN) | H | Cl | C(=O)(*i*-Pr) | O |
| C-9405 | H | single bond | Me | CH₂CF₃ | H | Me | C(=O)(*i*-Pr) | O |
| C-9406 | Me | single bond | H | CH₂CF₃ | H | Me | C(=O)(*i*-Pr) | O |

**[Table 442]**

| Compound Number | Y | W | Z | R¹ | R² | R³ | R¹⁰ | A |
|---|---|---|---|---|---|---|---|---|
| C-9407 | Me(R) | single bond | H | CH₂CF₃ | H | Me | C(=O)(*i*-Pr) | O |
| C-9408 | Me(S) | single bond | H | CH₂CF₃ | H | Me | C(=O)(*i*-Pr) | O |
| C-9409 | H | single bond | Me | Et | H | Me | C(=O)(*i*-Pr) | O |
| C-9410 | Me | single bond | H | Et | H | Me | C(=O)(*i*-Pr) | O |
| C-9411 | Me(R) | single bond | H | Et | H | Me | C(=O)(*i*-Pr) | O |
| C-9412 | Me(S) | single bond | H | Et | H | Me | C(=O)(*i*-Pr) | O |
| C-9413 | H | single bond | Me | c-Pr | H | Me | C(=O)(*i*-Pr) | O |
| C-9414 | Me | single bond | H | c-Pr | H | Me | C(=O)(*i*-Pr) | O |
| C-9415 | Me(R) | single bond | H | c-Pr | H | Me | C(=O)(*i*-Pr) | O |
| C-9416 | Me(S) | single bond | H | c-Pr | H | Me | C(=O)(*i*-Pr) | O |
| C-9417 | H | single bond | Me | c-Pr(1-CN) | H | Me | C(=O)(*i*-Pr) | O |
| C-9418 | Me | single bond | H | c-Pr(1-CN) | H | Me | C(=O)(*i*-Pr) | O |
| C-9419 | Me(R) | single bond | H | c-Pr(1-CN) | H | Me | C(=O)(*i*-Pr) | O |
| C-9420 | Me(S) | single bond | H | c-Pr(1-CN) | H | Me | C(=O)(*i*-Pr) | O |
| C-9421 | H | single bond | Me | CH₂CF₃ | H | Cl | C(=O)O(*t*-Bu) | O |
| C-9422 | Me | single bond | H | CH₂CF₃ | H | Cl | C(=O)O(*t*-Bu) | O |
| C-9423 | Me(R) | single bond | H | CH₂CF₃ | H | Cl | C(=O)O(*t*-Bu) | O |
| C-9424 | Me(S) | single bond | H | CH₂CF₃ | H | Cl | C(=O)O(*t*-Bu) | O |
| C-9425 | H | single bond | Me | Et | H | Cl | C(=O)O(*t*-Bu) | O |
| C-9426 | Me | single bond | H | Et | H | Cl | C(=O)O(*t*-Bu) | O |
| C-9427 | Me(R) | single bond | H | Et | H | Cl | C(=O)O(*t*-Bu) | O |
| C-9428 | Me(S) | single bond | H | Et | H | Cl | C(=O)O(*t*-Bu) | O |
| C-9429 | H | single bond | Me | c-Pr | H | Cl | C(=O)O(*t*-Bu) | O |
| C-9430 | Me | single bond | H | c-Pr | H | Cl | C(=O)O(*t*-Bu) | O |
| C-9431 | Me(R) | single bond | H | c-Pr | H | Cl | C(=O)O(*t*-Bu) | O |
| C-9432 | Me(S) | single bond | H | c-Pr | H | Cl | C(=O)O(*t*-Bu) | O |
| C-9433 | H | single bond | Me | c-Pr(1-CN) | H | Cl | C(=O)O(*t*-Bu) | O |
| C-9434 | Me | single bond | H | c-Pr(1-CN) | H | Cl | C(=O)O(*t*-Bu) | O |
| C-9435 | Me(R) | single bond | H | c-Pr(1-CN) | H | Cl | C(=O)O(*t*-Bu) | O |
| C-9436 | Me(S) | single bond | H | c-Pr(1-CN) | H | Cl | C(=O)O(*t*-Bu) | O |
| C-9437 | H | single bond | Me | CH₂CF₃ | H | Me | C(=O)O(*t*-Bu) | O |
| C-9438 | Me | single bond | H | CH₂CF₃ | H | Me | C(=O)O(*t*-Bu) | O |
| C-9439 | Me(R) | single bond | H | CH₂CF₃ | H | Me | C(=O)O(*t*-Bu) | O |
| C-9440 | Me(S) | single bond | H | CH₂CF₃ | H | Me | C(=O)O(*t*-Bu) | O |
| C-9441 | H | single bond | Me | Et | H | Me | C(=O)O(*t*-Bu) | O |
| C-9442 | Me | single bond | H | Et | H | Me | C(=O)O(*t*-Bu) | O |
| C-9443 | Me(R) | single bond | H | Et | H | Me | C(=O)O(*t*-Bu) | O |
| C-9444 | Me(S) | single bond | H | Et | H | Me | C(=O)O(*t*-Bu) | O |
| C-9445 | H | single bond | Me | c-Pr | H | Me | C(=O)O(*t*-Bu) | O |
| C-9446 | Me | single bond | H | c-Pr | H | Me | C(=O)O(*t*-Bu) | O |
| C-9447 | Me(R) | single bond | H | c-Pr | H | Me | C(=O)O(*t*-Bu) | O |
| C-9448 | Me(S) | single bond | H | c-Pr | H | Me | C(=O)O(*t*-Bu) | O |
| C-9449 | H | single bond | Me | c-Pr(1-CN) | H | Me | C(=O)O(*t*-Bu) | O |
| C-9450 | Me | single bond | H | c-Pr(1-CN) | H | Me | C(=O)O(*t*-Bu) | O |

**[Table 443]**

| Compound Number | Y | W | Z | R¹ | R² | R³ | R¹⁰ | A |
|---|---|---|---|---|---|---|---|---|
| C-9451 | Me(R) | single bond | H | c-Pr(1-CN) | H | Me | C(=O)O(*t*-Bu) | O |
| C-9452 | Me(S) | single bond | H | c-Pr(1-CN) | H | Me | C(=O)O(*t*-Bu) | O |
| C-9453 | H | single bond | Me | CH₂CF₃ | H | Cl | CH₂OEt | O |
| C-9454 | Me | single bond | H | CH₂CF₃ | H | Cl | CH₂OEt | O |
| C-9455 | Me(R) | single bond | H | CH₂CF₃ | H | Cl | CH₂OEt | O |
| C-9456 | Me(S) | single bond | H | CH₂CF₃ | H | Cl | CH₂OEt | O |
| C-9457 | H | single bond | Me | Et | H | Cl | CH₂OEt | O |
| C-9458 | Me | single bond | H | Et | H | Cl | CH₂OEt | O |
| C-9459 | Me(R) | single bond | H | Et | H | Cl | CH₂OEt | O |
| C-9460 | Me(S) | single bond | H | Et | H | Cl | CH₂OEt | O |
| C-9461 | H | single bond | Me | c-Pr | H | Cl | CH₂OEt | O |
| C-9462 | Me | single bond | H | c-Pr | H | Cl | CH₂OEt | O |
| C-9463 | Me(R) | single bond | H | c-Pr | H | Cl | CH₂OEt | O |
| C-9464 | Me(S) | single bond | H | c-Pr | H | Cl | CH₂OEt | O |
| C-9465 | H | single bond | Me | c-Pr(1-CN) | H | Cl | CH₂OEt | O |
| C-9466 | Me | single bond | H | c-Pr(1-CN) | H | Cl | CH₂OEt | O |
| C-9467 | Me(R) | single bond | H | c-Pr(1-CN) | H | Cl | CH₂OEt | O |
| C-9468 | Me(S) | single bond | H | c-Pr(1-CN) | H | Cl | CH₂OEt | O |
| C-9469 | H | single bond | Me | CH₂CF₃ | H | Me | CH₂OEt | O |
| C-9470 | Me | single bond | H | CH₂CF₃ | H | Me | CH₂OEt | O |
| C-9471 | Me(R) | single bond | H | CH₂CF₃ | H | Me | CH₂OEt | O |
| C-9472 | Me(S) | single bond | H | CH₂CF₃ | H | Me | CH₂OEt | O |
| C-9473 | H | single bond | Me | Et | H | Me | CH₂OEt | O |
| C-9474 | Me | single bond | H | Et | H | Me | CH₂OEt | O |
| C-9475 | Me(R) | single bond | H | Et | H | Me | CH₂OEt | O |
| C-9476 | Me(S) | single bond | H | Et | H | Me | CH₂OEt | O |
| C-9477 | H | single bond | Me | c-Pr | H | Me | CH₂OEt | O |
| C-9478 | Me | single bond | H | c-Pr | H | Me | CH₂OEt | O |
| C-9479 | Me(R) | single bond | H | c-Pr | H | Me | CH₂OEt | O |
| C-9480 | Me(S) | single bond | H | c-Pr | H | Me | CH₂OEt | O |
| C-9481 | H | single bond | Me | c-Pr(1-CN) | H | Me | CH₂OEt | O |
| C-9482 | Me | single bond | H | c-Pr(1-CN) | H | Me | CH₂OEt | O |
| C-9483 | Me(R) | single bond | H | c-Pr(1-CN) | H | Me | CH₂OEt | O |
| C-9484 | Me(S) | single bond | H | c-Pr(1-CN) | H | Me | CH₂OEt | O |
| C-9485 | H | single bond | Me | CH₂CF₃ | H | Cl | CH₂SMe | O |
| C-9486 | Me | single bond | H | CH₂CF₃ | H | Cl | CH₂SMe | O |
| C-9487 | Me(R) | single bond | H | CH₂CF₃ | H | Cl | CH₂SMe | O |
| C-9488 | Me(S) | single bond | H | CH₂CF₃ | H | Cl | CH₂SMe | O |
| C-9489 | H | single bond | Me | Et | H | Cl | CH₂SMe | O |
| C-9490 | Me | single bond | H | Et | H | Cl | CH₂SMe | O |
| C-9491 | Me(R) | single bond | H | Et | H | Cl | CH₂SMe | O |
| C-9492 | Me(S) | single bond | H | Et | H | Cl | CH₂SMe | O |
| C-9493 | H | single bond | Me | c-Pr | H | Cl | CH₂SMe | O |
| C-9494 | Me | single bond | H | c-Pr | H | Cl | CH₂SMe | O |

**[Table 444]**

| Compound Number | Y | W | z | R¹ | R² | R³ | R¹⁰ | A |
|---|---|---|---|---|---|---|---|---|
| C-9495 | Me(R) | single bond | H | c-Pr | H | Cl | CH₂SMe | O |
| C-9496 | Me(S) | single bond | H | c-Pr | H | Cl | CH₂SMe | O |
| C-9497 | H | single bond | Me | c-Pr(1-CN) | H | Cl | CH₂SMe | O |
| C-9498 | Me | single bond | H | c-Pr(1-CN) | H | Cl | CH₂SMe | O |
| C-9499 | Me(R) | single bond | H | c-Pr(1-CN) | H | Cl | CH₂SMe | O |
| C-9500 | Me(S) | single bond | H | c-Pr(1-CN) | H | Cl | CH₂SMe | O |
| C-9501 | H | single bond | Me | CH₂CF₃ | H | Me | CH₂SMe | O |
| C-9502 | Me | single bond | H | CH₂CF₃ | H | Me | CH₂SMe | O |
| C-9503 | Me(R) | single bond | H | CH₂CF₃ | H | Me | CH₂SMe | O |
| C-9504 | Me(S) | single bond | H | CH₂CF₃ | H | Me | CH₂SMe | O |
| C-9505 | H | single bond | Me | Et | H | Me | CH₂SMe | O |
| C-9506 | Me | single bond | H | Et | H | Me | CH₂SMe | O |
| C-9507 | Me(R) | single bond | H | Et | H | Me | CH₂SMe | O |
| C-9508 | Me(S) | single bond | H | Et | H | Me | CH₂SMe | O |
| C-9509 | H | single bond | Me | c-Pr | H | Me | CH₂SMe | O |
| C-9510 | Me | single bond | H | c-Pr | H | Me | CH₂SMe | O |
| C-9511 | Me(R) | single bond | H | c-Pr | H | Me | CH₂SMe | O |
| C-9512 | Me(S) | single bond | H | c-Pr | H | Me | CH₂SMe | O |
| C-9513 | H | single bond | Me | c-Pr(1-CN) | H | Me | CH₂SMe | O |
| C-9514 | Me | single bond | H | c-Pr(1-CN) | H | Me | CH₂SMe | O |
| C-9515 | Me(R) | single bond | H | c-Pr(1-CN) | H | Me | CH₂SMe | O |
| C-9516 | Me(S) | single bond | H | c-Pr(1-CN) | H | Me | CH₂SMe | O |
| C-9517 | H | single bond | Me | CH₂CF₃ | H | Cl | CH₂S(=O)₂Me | O |
| C-9518 | Me | single bond | H | CH₂CF₃ | H | Cl | CH₂S(=O)₂Me | O |
| C-9519 | Me(R) | single bond | H | CH₂CF₃ | H | Cl | CH₂S(=O)₂Me | O |
| C-9520 | Me(S) | single bond | H | CH₂CF₃ | H | Cl | CH₂S(=O)₂Me | O |
| C-9521 | H | single bond | Me | Et | H | Cl | CH₂S(=O)₂Me | O |
| C-9522 | Me | single bond | H | Et | H | Cl | CH₂S(=O)₂Me | O |
| C-9523 | Me(R) | single bond | H | Et | H | Cl | CH₂S(=O)₂Me | O |
| C-9524 | Me(S) | single bond | H | Et | H | Cl | CH₂S(=O)₂Me | O |
| C-9525 | H | single bond | Me | c-Pr | H | Cl | CH₂S(=O)₂Me | O |
| C-9526 | Me | single bond | H | c-Pr | H | Cl | CH₂S(=O)₂Me | O |
| C-9527 | Me(R) | single bond | H | c-Pr | H | Cl | CH₂S(=O)₂Me | O |
| C-9528 | Me(S) | single bond | H | c-Pr | H | Cl | CH₂S(=O)₂Me | O |
| C-9529 | H | single bond | Me | c-Pr(1-CN) | H | Cl | CH₂S(=O)₂Me | O |
| C-9530 | Me | single bond | H | c-Pr(1-CN) | H | Cl | CH₂S(=O)₂Me | O |
| C-9531 | Me(R) | single bond | H | c-Pr(1-CN) | H | Cl | CH₂S(=O)₂Me | O |
| C-9532 | Me(S) | single bond | H | c-Pr(1-CN) | H | Cl | CH₂S(=O)₂Me | O |
| C-9533 | H | single bond | Me | CH₂CF₃ | H | Me | CH₂S(=O)₂Me | O |
| C-9534 | Me | single bond | H | CH₂CF₃ | H | Me | CH₂S(=O)₂Me | O |
| C-9535 | Me(R) | single bond | H | CH₂CF₃ | H | Me | CH₂S(=O)₂Me | O |
| C-9536 | Me(S) | single bond | H | CH₂CF₃ | H | Me | CH₂S(=O)₂Me | O |
| C-9537 | H | single bond | Me | Et | H | Me | CH₂S(=O)₂Me | O |
| C-9538 | Me | single bond | H | Et | H | Me | CH₂S(=O)₂Me | O |

**[Table 445]**

| Compound Number | Y | W | Z | R¹ | R² | R³ | R¹⁰ | A |
|---|---|---|---|---|---|---|---|---|
| C-9539 | Me(R) | single bond | H | Et | H | Me | CH₂S(=O)₂Me | O |
| C-9540 | Me(S) | single bond | H | Et | H | Me | CH₂S(=O)₂Me | O |
| C-9541 | H | single bond | Me | c-Pr | H | Me | CH₂S(=O)₂Me | O |
| C-9542 | Me | single bond | H | c-Pr | H | Me | CH₂S(=O)₂Me | O |
| C-9543 | Me(R) | single bond | H | c-Pr | H | Me | CH₂S(=O)₂Me | O |
| C-9544 | Me(S) | single bond | H | c-Pr | H | Me | CH₂S(=O)₂Me | O |
| C-9545 | H | single bond | Me | c-Pr(1-CN) | H | Me | CH₂S(=O)₂Me | O |
| C-9546 | Me | single bond | H | c-Pr(1-CN) | H | Me | CH₂S(=O)₂Me | O |
| C-9547 | Me(R) | single bond | H | c-Pr(1-CN) | H | Me | CH₂S(=O)₂Me | O |
| C-9548 | Me(S) | single bond | H | c-Pr(1-CN) | H | Me | CH₂S(=O)₂Me | O |
| C-9549 | H | single bond | Me | CH₂CF₃ | H | Cl | CH₂C(=O)Me | O |
| C-9550 | Me | single bond | H | CH₂CF₃ | H | Cl | CH₂C(=O)Me | O |
| C-9551 | Me(R) | single bond | H | CH₂CF₃ | H | Cl | CH₂C(=O)Me | O |
| C-9552 | Me(S) | single bond | H | CH₂CF₃ | H | Cl | CH₂C(=O)Me | O |
| C-9553 | H | single bond | Me | Et | H | Cl | CH₂C(=O)Me | O |
| C-9554 | Me | single bond | H | Et | H | Cl | CH₂C(=O)Me | O |
| C-9555 | Me(R) | single bond | H | Et | H | Cl | CH₂C(=O)Me | O |
| C-9556 | Me(S) | single bond | H | Et | H | Cl | CH₂C(=O)Me | O |
| C-9557 | H | single bond | Me | c-Pr | H | Cl | CH₂C(=O)Me | O |
| C-9558 | Me | single bond | H | c-Pr | H | Cl | CH₂C(=O)Me | O |
| C-9559 | Me(R) | single bond | H | c-Pr | H | Cl | CH₂C(=O)Me | O |
| C-9560 | Me(S) | single bond | H | c-Pr | H | Cl | CH₂C(=O)Me | O |
| C-9561 | H | single bond | Me | c-Pr(1-CN) | H | Cl | CH₂C(=O)Me | O |
| C-9562 | Me | single bond | H | c-Pr(1-CN) | H | Cl | CH₂C(=O)Me | O |
| C-9563 | Me(R) | single bond | H | c-Pr(1-CN) | H | Cl | CH₂C(=O)Me | O |
| C-9564 | Me(S) | single bond | H | c-Pr(1-CN) | H | Cl | CH₂C(=O)Me | O |
| C-9565 | H | single bond | Me | CH₂CF₃ | H | Me | CH₂C(=O)Me | O |
| C-9566 | Me | single bond | H | CH₂CF₃ | H | Me | CH₂C(=O)Me | O |
| C-9567 | Me(R) | single bond | H | CH₂CF₃ | H | Me | CH₂C(=O)Me | O |
| C-9568 | Me(S) | single bond | H | CH₂CF₃ | H | Me | CH₂C(=O)Me | O |
| C-9569 | H | single bond | Me | Et | H | Me | CH₂C(=O)Me | O |
| C-9570 | Me | single bond | H | Et | H | Me | CH₂C(=O)Me | O |
| C-9571 | Me(R) | single bond | H | Et | H | Me | CH₂C(=O)Me | O |
| C-9572 | Me(S) | single bond | H | Et | H | Me | CH₂C(=O)Me | O |
| C-9573 | H | single bond | Me | c-Pr | H | Me | CH₂C(=O)Me | O |
| C-9574 | Me | single bond | H | c-Pr | H | Me | CH₂C(=O)Me | O |
| C-9575 | Me(R) | single bond | H | c-Pr | H | Me | CH₂C(=O)Me | O |
| C-9576 | Me(S) | single bond | H | c-Pr | H | Me | CH₂C(=O)Me | O |
| C-9577 | H | single bond | Me | c-Pr(1-CN) | H | Me | CH₂C(=O)Me | O |
| C-9578 | Me | single bond | H | c-Pr(1-CN) | H | Me | CH₂C(=O)Me | O |
| C-9579 | Me(R) | single bond | H | c-Pr(1-CN) | H | Me | CH₂C(=O)Me | O |
| C-9580 | Me(S) | single bond | H | c-Pr(1-CN) | H | Me | CH₂C(=O)Me | O |
| C-9581 | H | single bond | Me | CH₂CF₃ | H | Cl | CH₂C(=O)OMe | O |
| C-9582 | Me | single bond | H | CH₂CF₃ | H | Cl | CH₂C(=O)OMe | O |

**[Table 446]**

| Compound Number | Y | W | Z | R¹ | R² | R³ | R¹⁰ | A |
|---|---|---|---|---|---|---|---|---|
| C-9583 | Me(R) | single bond | H | CH₂CF₃ | H | Cl | CH₂C(=O)OMe | O |
| C-9584 | Me(S) | single bond | H | CH₂CF₃ | H | Cl | CH₂C(=O)OMe | O |
| C-9585 | H | single bond | Me | Et | H | Cl | CH₂C(=O)OMe | O |
| C-9586 | Me | single bond | H | Et | H | Cl | CH₂C(=O)OMe | O |
| C-9587 | Me(R) | single bond | H | Et | H | Cl | CH₂C(=O)OMe | O |
| C-9588 | Me(S) | single bond | H | Et | H | Cl | CH₂C(=O)OMe | O |
| C-9589 | H | single bond | Me | c-Pr | H | Cl | CH₂C(=O)OMe | O |
| C-9590 | Me | single bond | H | c-Pr | H | Cl | CH₂C(=O)OMe | O |
| C-9591 | Me(R) | single bond | H | c-Pr | H | Cl | CH₂C(=O)OMe | O |
| C-9592 | Me(S) | single bond | H | c-Pr | H | Cl | CH₂C(=O)OMe | O |
| C-9593 | H | single bond | Me | c-Pr(1-CN) | H | Cl | CH₂C(=O)OMe | O |
| C-9594 | Me | single bond | H | c-Pr(1-CN) | H | Cl | CH₂C(=O)OMe | O |
| C-9595 | Me(R) | single bond | H | c-Pr(1-CN) | H | Cl | CH₂C(=O)OMe | O |
| C-9596 | Me(S) | single bond | H | c-Pr(1-CN) | H | Cl | CH₂C(=O)OMe | O |
| C-9597 | H | single bond | Me | CH₂CF₃ | H | Me | CH₂C(=O)OMe | O |
| C-9598 | Me | single bond | H | CH₂CF₃ | H | Me | CH₂C(=O)OMe | O |
| C-9599 | Me(R) | single bond | H | CH₂CF₃ | H | Me | CH₂C(=O)OMe | O |
| C-9600 | Me(S) | single bond | H | CH₂CF₃ | H | Me | CH₂C(=O)OMe | O |
| C-9601 | H | single bond | Me | Et | H | Me | CH₂C(=O)OMe | O |
| C-9602 | Me | single bond | H | Et | H | Me | CH₂C(=O)OMe | O |
| C-9603 | Me(R) | single bond | H | Et | H | Me | CH₂C(=O)OMe | O |
| C-9604 | Me(S) | single bond | H | Et | H | Me | CH₂C(=O)OMe | O |
| C-9605 | H | single bond | Me | c-Pr | H | Me | CH₂C(=O)OMe | O |
| C-9606 | Me | single bond | H | c-Pr | H | Me | CH₂C(=O)OMe | O |
| C-9607 | Me(R) | single bond | H | c-Pr | H | Me | CH₂C(=O)OMe | O |
| C-9608 | Me(S) | single bond | H | c-Pr | H | Me | CH₂C(=O)OMe | O |
| C-9609 | H | single bond | Me | c-Pr(1-CN) | H | Me | CH₂C(=O)OMe | O |
| C-9610 | Me | single bond | H | c-Pr(1-CN) | H | Me | CH₂C(=O)OMe | O |
| C-9611 | Me(R) | single bond | H | c-Pr(1-CN) | H | Me | CH₂C(=O)OMe | O |
| C-9612 | Me(S) | single bond | H | c-Pr(1-CN) | H | Me | CH₂C(=O)OMe | O |
| C-9613 | H | single bond | Me | CH₂CF₃ | H | Cl | CH₂C(=O)NHMe | O |
| C-9614 | Me | single bond | H | CH₂CF₃ | H | Cl | CH₂C(=O)NHMe | O |
| C-9615 | Me(R) | single bond | H | CH₂CF₃ | H | Cl | CH₂C(=O)NHMe | O |
| C-9616 | Me(S) | single bond | H | CH₂CF₃ | H | Cl | CH₂C(=O)NHMe | O |
| C-9617 | H | single bond | Me | Et | H | Cl | CH₂C(=O)NHMe | O |
| C-9618 | Me | single bond | H | Et | H | Cl | CH₂C(=O)NHMe | O |
| C-9619 | Me(R) | single bond | H | Et | H | Cl | CH₂C(=O)NHMe | O |
| C-9620 | Me(S) | single bond | H | Et | H | Cl | CH₂C(=O)NHMe | O |
| C-9621 | H | single bond | Me | c-Pr | H | Cl | CH₂C(=O)NHMe | O |
| C-9622 | Me | single bond | H | c-Pr | H | Cl | CH₂C(=O)NHMe | O |
| C-9623 | Me(R) | single bond | H | c-Pr | H | Cl | CH₂C(=O)NHMe | O |
| C-9624 | Me(S) | single bond | H | c-Pr | H | Cl | CH₂C(=O)NHMe | O |
| C-9625 | H | single bond | Me | c-Pr(1-CN) | H | Cl | CH₂C(=O)NHMe | O |
| C-9626 | Me | single bond | H | c-Pr(1-CN) | H | Cl | CH₂C(=O)NHMe | O |

**[Table 447]**

| Compound Number | Y | W | Z | R¹ | R² | R³ | R¹⁰ | A |
|---|---|---|---|---|---|---|---|---|
| C-9627 | Me(R) | single bond | H | c-Pr(1-CN) | H | Cl | CH₂C(=O)NHMe | O |
| C-9628 | Me(S) | single bond | H | c-Pr(1-CN) | H | Cl | CH₂C(=O)NHMe | O |
| C-9629 | H | single bond | Me | CH₂CF₃ | H | Me | CH₂C(=O)NHMe | O |
| C-9630 | Me | single bond | H | CH₂CF₃ | H | Me | CH₂C(=O)NHMe | O |
| C-9631 | Me(R) | single bond | H | CH₂CF₃ | H | Me | CH₂C(=O)NHMe | O |
| C-9632 | Me(S) | single bond | H | CH₂CF₃ | H | Me | CH₂C(=O)NHMe | O |
| C-9633 | H | single bond | Me | Et | H | Me | CH₂C(=O)NHMe | O |
| C-9634 | Me | single bond | H | Et | H | Me | CH₂C(=O)NHMe | O |
| C-9635 | Me(R) | single bond | H | Et | H | Me | CH₂C(=O)NHMe | O |
| C-9636 | Me(S) | single bond | H | Et | H | Me | CH₂C(=O)NHMe | O |
| C-9637 | H | single bond | Me | c-Pr | H | Me | CH₂C(=O)NHMe | O |
| C-9638 | Me | single bond | H | c-Pr | H | Me | CH₂C(=O)NHMe | O |
| C-9639 | Me(R) | single bond | H | c-Pr | H | Me | CH₂C(=O)NHMe | O |
| C-9640 | Me(S) | single bond | H | c-Pr | H | Me | CH₂C(=O)NHMe | O |
| C-9641 | H | single bond | Me | c-Pr(1-CN) | H | Me | CH₂C(=O)NHMe | O |
| C-9642 | Me | single bond | H | c-Pr(1-CN) | H | Me | CH₂C(=O)NHMe | O |
| C-9643 | Me(R) | single bond | H | c-Pr(1-CN) | H | Me | CH₂C(=O)NHMe | O |
| C-9644 | Me(S) | single bond | H | c-Pr(1-CN) | H | Me | CH₂C(=O)NHMe | O |
| C-9645 | H | single bond | Me | CH₂CF₃ | H | Cl | CH₂OC(=O)Me | O |
| C-9646 | Me | single bond | H | CH₂CF₃ | H | Cl | CH₂OC(=O)Me | O |
| C-9647 | Me(R) | single bond | H | CH₂CF₃ | H | Cl | CH₂OC(=O)Me | O |
| C-9648 | Me(S) | single bond | H | CH₂CF₃ | H | Cl | CH₂OC(=O)Me | O |
| C-9649 | H | single bond | Me | Et | H | Cl | CH₂OC(=O)Me | O |
| C-9650 | Me | single bond | H | Et | H | Cl | CH₂OC(=O)Me | O |
| C-9651 | Me(R) | single bond | H | Et | H | Cl | CH₂OC(=O)Me | O |
| C-9652 | Me(S) | single bond | H | Et | H | Cl | CH₂OC(=O)Me | O |
| C-9653 | H | single bond | Me | c-Pr | H | Cl | CH₂OC(=O)Me | O |
| C-9654 | Me | single bond | H | c-Pr | H | Cl | CH₂OC(=O)Me | O |
| C-9655 | Me(R) | single bond | H | c-Pr | H | Cl | CH₂OC(=O)Me | O |
| C-9656 | Me(S) | single bond | H | c-Pr | H | Cl | CH₂OC(=O)Me | O |
| C-9657 | H | single bond | Me | c-Pr(1-CN) | H | Cl | CH₂OC(=O)Me | O |
| C-9658 | Me | single bond | H | c-Pr(1-CN) | H | Cl | CH₂OC(=O)Me | O |
| C-9659 | Me(R) | single bond | H | c-Pr(1-CN) | H | Cl | CH₂OC(=O)Me | O |
| C-9660 | Me(S) | single bond | H | c-Pr(1-CN) | H | Cl | CH₂OC(=O)Me | O |
| C-9661 | H | single bond | Me | CH₂CF₃ | H | Me | CH₂OC(=O)Me | O |
| C-9662 | Me | single bond | H | CH₂CF₃ | H | Me | CH₂OC(=O)Me | O |
| C-9663 | Me(R) | single bond | H | CH₂CF₃ | H | Me | CH₂OC(=O)Me | O |
| C-9664 | Me(S) | single bond | H | CH₂CF₃ | H | Me | CH₂OC(=O)Me | O |
| C-9665 | H | single bond | Me | Et | H | Me | CH₂OC(=O)Me | O |
| C-9666 | Me | single bond | H | Et | H | Me | CH₂OC(=O)Me | O |
| C-9667 | Me(R) | single bond | H | Et | H | Me | CH₂OC(=O)Me | O |
| C-9668 | Me(S) | single bond | H | Et | H | Me | CH₂OC(=O)Me | O |
| C-9669 | H | single bond | Me | c-Pr | H | Me | CH₂OC(=O)Me | O |
| C-9670 | Me | single bond | H | c-Pr | H | Me | CH₂OC(=O)Me | O |

**[Table 448]**

| Compound Number | Y | W | Z | R¹ | R² | R³ | R¹⁰ | A |
|---|---|---|---|---|---|---|---|---|
| C-9671 | Me(R) | single bond | H | c-Pr | H | Me | CH₂OC(=O)Me | O |
| C-9672 | Me(S) | single bond | H | c-Pr | H | Me | CH₂OC(=O)Me | O |
| C-9673 | H | single bond | Me | c-Pr(1-CN) | H | Me | CH₂OC(=O)Me | O |
| C-9674 | Me | single bond | H | c-Pr(1-CN) | H | Me | CH₂OC(=O)Me | O |
| C-9675 | Me(R) | single bond | H | c-Pr(1-CN) | H | Me | CH₂OC(=O)Me | O |
| C-9676 | Me(S) | single bond | H | c-Pr(1-CN) | H | Me | CH₂OC(=O)Me | O |
| C-9677 | H | single bond | Me | CH₂CF₃ | H | Cl | CH₂OC(=O)(*i*-Pr) | O |
| C-9678 | Me | single bond | H | CH₂CF₃ | H | Cl | CH₂OC(=O)(*i*-Pr) | O |
| C-9679 | Me(R) | single bond | H | CH₂CF₃ | H | Cl | CH₂OC(=O)(*i*-Pr) | O |
| C-9680 | Me(S) | single bond | H | CH₂CF₃ | H | Cl | CH₂OC(=O)(*i*-Pr) | O |
| C-9681 | H | single bond | Me | Et | H | Cl | CH₂OC(=O)(*i*-Pr) | O |
| C-9682 | Me | single bond | H | Et | H | Cl | CH₂OC(=O)(*i*-Pr) | O |
| C-9683 | Me(R) | single bond | H | Et | H | Cl | CH₂OC(=O)(*i*-Pr) | O |
| C-9684 | Me(S) | single bond | H | Et | H | Cl | CH₂OC(=O)(*i*-Pr) | O |
| C-9685 | H | single bond | Me | c-Pr | H | Cl | CH₂OC(=O)(*i*-Pr) | O |
| C-9686 | Me | single bond | H | c-Pr | H | Cl | CH₂OC(=O)(*i*-Pr) | O |
| C-9687 | Me(R) | single bond | H | c-Pr | H | Cl | CH₂OC(=O)(*i*-Pr) | O |
| C-9688 | Me(S) | single bond | H | c-Pr | H | Cl | CH₂OC(=O)(*i*-Pr) | O |
| C-9689 | H | single bond | Me | c-Pr(1-CN) | H | Cl | CH₂OC(=O)(*i*-Pr) | O |
| C-9690 | Me | single bond | H | c-Pr(1-CN) | H | Cl | CH₂OC(=O)(*i*-Pr) | O |
| C-9691 | Me(R) | single bond | H | c-Pr(1-CN) | H | Cl | CH₂OC(=O)(*i*-Pr) | O |
| C-9692 | Me(S) | single bond | H | c-Pr(1-CN) | H | Cl | CH₂OC(=O)(*i*-Pr) | O |
| C-9693 | H | single bond | Me | CH₂CF₃ | H | Me | CH₂OC(=O)(*i*-Pr) | O |
| C-9694 | Me | single bond | H | CH₂CF₃ | H | Me | CH₂OC(=O)(*i*-Pr) | O |
| C-9695 | Me(R) | single bond | H | CH₂CF₃ | H | Me | CH₂OC(=O)(*i*-Pr) | O |
| C-9696 | Me(S) | single bond | H | CH₂CF₃ | H | Me | CH₂OC(=O)(*i*-Pr) | O |
| C-9697 | H | single bond | Me | Et | H | Me | CH₂OC(=O)(*i*-Pr) | O |
| C-9698 | Me | single bond | H | Et | H | Me | CH₂OC(=O)(*i*-Pr) | O |
| C-9699 | Me(R) | single bond | H | Et | H | Me | CH₂OC(=O)(*i*-Pr) | O |
| C-9700 | Me(S) | single bond | H | Et | H | Me | CH₂OC(=O)(*i*-Pr) | O |
| C-9701 | H | single bond | Me | c-Pr | H | Me | CH₂OC(=O)(*i*-Pr) | O |
| C-9702 | Me | single bond | H | c-Pr | H | Me | CH₂OC(=O)(*i*-Pr) | O |
| C-9703 | Me(R) | single bond | H | c-Pr | H | Me | CH₂OC(=O)(*i*-Pr) | O |
| C-9704 | Me(S) | single bond | H | c-Pr | H | Me | CH₂OC(=O)(*i*-Pr) | O |
| C-9705 | H | single bond | Me | c-Pr(1-CN) | H | Me | CH₂OC(=O)(*i*-Pr) | O |
| C-9706 | Me | single bond | H | c-Pr(1-CN) | H | Me | CH₂OC(=O)(*i*-Pr) | O |
| C-9707 | Me(R) | single bond | H | c-Pr(1-CN) | H | Me | CH₂OC(=O)(*i*-Pr) | O |
| C-9708 | Me(S) | single bond | H | c-Pr(1-CN) | H | Me | CH₂OC(=O)(*i*-Pr) | O |
| C-9709 | H | single bond | Me | CH₂CF₃ | H | Cl | CH₂OC(=O)(*t*-Bu) | O |
| C-9710 | Me | single bond | H | CH₂CF₃ | H | Cl | CH₂OC(=O)(*t*-Bu) | O |
| C-9711 | Me(R) | single bond | H | CH₂CF₃ | H | Cl | CH₂OC(=O)(*t*-Bu) | O |
| C-9712 | Me(S) | single bond | H | CH₂CF₃ | H | Cl | CH₂OC(=O)(*t*-Bu) | O |
| C-9713 | H | single bond | Me | Et | H | Cl | CH₂OC(=O)(*t*-Bu) | O |
| C-9714 | Me | single bond | H | Et | H | Cl | CH₂OC(=O)(*t*-Bu) | O |

**[Table 449]**

| Compound Number | Y | W | Z | R¹ | R² | R³ | R¹⁰ | A |
|---|---|---|---|---|---|---|---|---|
| C-9715 | Me(R) | single bond | H | Et | H | Cl | CH₂OC(=O)(*t*-Bu) | O |
| C-9716 | Me(S) | single bond | H | Et | H | Cl | CH₂OC(=O)(*t*-Bu) | O |
| C-9717 | H | single bond | Me | c-Pr | H | Cl | CH₂OC(=O)(*t*-Bu) | O |
| C-9718 | Me | single bond | H | c-Pr | H | Cl | CH₂OC(=O)(*t*-Bu) | O |
| C-9719 | Me(R) | single bond | H | c-Pr | H | Cl | CH₂OC(=O)(*t*-Bu) | O |
| C-9720 | Me(S) | single bond | H | c-Pr | H | Cl | CH₂OC(=O)(*t*-Bu) | O |
| C-9721 | H | single bond | Me | c-Pr(1-CN) | H | Cl | CH₂OC(=O)(*t*-Bu) | O |
| C-9722 | Me | single bond | H | c-Pr(1-CN) | H | Cl | CH₂OC(=O)(*t*-Bu) | O |
| C-9723 | Me(R) | single bond | H | c-Pr(1-CN) | H | Cl | CH₂OC(=O)(*t*-Bu) | O |
| C-9724 | Me(S) | single bond | H | c-Pr(1-CN) | H | Cl | CH₂OC(=O)(*t*-Bu) | O |
| C-9725 | H | single bond | Me | CH₂CF₃ | H | Me | CH₂OC(=O)(*t*-Bu) | O |
| C-9726 | Me | single bond | H | CH₂CF₃ | H | Me | CH₂OC(=O)(*t*-Bu) | O |
| C-9727 | Me(R) | single bond | H | CH₂CF₃ | H | Me | CH₂OC(=O)(*t*-Bu) | O |
| C-9728 | Me(S) | single bond | H | CH₂CF₃ | H | Me | CH₂OC(=O)(*t*-Bu) | O |
| C-9729 | H | single bond | Me | Et | H | Me | CH₂OC(=O)(*t*-Bu) | O |
| C-9730 | Me | single bond | H | Et | H | Me | CH₂OC(=O)(*t*-Bu) | O |
| C-9731 | Me(R) | single bond | H | Et | H | Me | CH₂OC(=O)(*t*-Bu) | O |
| C-9732 | Me(S) | single bond | H | Et | H | Me | CH₂OC(=O)(*t*-Bu) | O |
| C-9733 | H | single bond | Me | c-Pr | H | Me | CH₂OC(=O)(*t*-Bu) | O |
| C-9734 | Me | single bond | H | c-Pr | H | Me | CH₂OC(=O)(*t*-Bu) | O |
| C-9735 | Me(R) | single bond | H | c-Pr | H | Me | CH₂OC(=O)(*t*-Bu) | O |
| C-9736 | Me(S) | single bond | H | c-Pr | H | Me | CH₂OC(=O)(*t*-Bu) | O |
| C-9737 | H | single bond | Me | c-Pr(1-CN) | H | Me | CH₂OC(=O)(*t*-Bu) | O |
| C-9738 | Me | single bond | H | c-Pr(1-CN) | H | Me | CH₂OC(=O)(*t*-Bu) | O |
| C-9739 | Me(R) | single bond | H | c-Pr(1-CN) | H | Me | CH₂OC(=O)(*t*-Bu) | O |
| C-9740 | Me(S) | single bond | H | c-Pr(1-CN) | H | Me | CH₂OC(=O)(*t*-Bu) | O |
| C-9741 | H | single bond | Me | CH₂CF₃ | H | Cl | CH₂OC(=O)OEt | O |
| C-9742 | Me | single bond | H | CH₂CF₃ | H | Cl | CH₂OC(=O)OEt | O |
| C-9743 | Me(R) | single bond | H | CH₂CF₃ | H | Cl | CH₂OC(=O)OEt | O |
| C-9744 | Me(S) | single bond | H | CH₂CF₃ | H | Cl | CH₂OC(=O)OEt | O |
| C-9745 | H | single bond | Me | Et | H | Cl | CH₂OC(=O)OEt | O |
| C-9746 | Me | single bond | H | Et | H | Cl | CH₂OC(=O)OEt | O |
| C-9747 | Me(R) | single bond | H | Et | H | Cl | CH₂OC(=O)OEt | O |
| C-9748 | Me(S) | single bond | H | Et | H | Cl | CH₂OC(=O)OEt | O |
| C-9749 | H | single bond | Me | c-Pr | H | Cl | CH₂OC(=O)OEt | O |
| C-9750 | Me | single bond | H | c-Pr | H | Cl | CH₂OC(=O)OEt | O |
| C-9751 | Me(R) | single bond | H | c-Pr | H | Cl | CH₂OC(=O)OEt | O |
| C-9752 | Me(S) | single bond | H | c-Pr | H | Cl | CH₂OC(=O)OEt | O |
| C-9753 | H | single bond | Me | c-Pr(1-CN) | H | Cl | CH₂OC(=O)OEt | O |
| C-9754 | Me | single bond | H | c-Pr(1-CN) | H | Cl | CH₂OC(=O)OEt | O |
| C-9755 | Me(R) | single bond | H | c-Pr(1-CN) | H | Cl | CH₂OC(=O)OEt | O |
| C-9756 | Me(S) | single bond | H | c-Pr(1-CN) | H | Cl | CH₂OC(=O)OEt | O |
| C-9757 | H | single bond | Me | CH₂CF₃ | H | Me | CH₂OC(=O)OEt | O |
| C-9758 | Me | single bond | H | CH₂CF₃ | H | Me | CH₂OC(=O)OEt | O |

**[Table 450]**

| Compound Number | Y | W | Z | R¹ | R² | R³ | R¹⁰ | A |
|---|---|---|---|---|---|---|---|---|
| C-9759 | Me(R) | single bond | H | CH₂CF₃ | H | Me | CH₂OC(=O)OEt | O |
| C-9760 | Me(S) | single bond | H | CH₂CF₃ | H | Me | CH₂OC(=O)OEt | O |
| C-9761 | H | single bond | Me | Et | H | Me | CH₂OC(=O)OEt | O |
| C-9762 | Me | single bond | H | Et | H | Me | CH₂OC(=O)OEt | O |
| C-9763 | Me(R) | single bond | H | Et | H | Me | CH₂OC(=O)OEt | O |
| C-9764 | Me(S) | single bond | H | Et | H | Me | CH₂OC(=O)OEt | O |
| C-9765 | H | single bond | Me | c-Pr | H | Me | CH₂OC(=O)OEt | O |
| C-9766 | Me | single bond | H | c-Pr | H | Me | CH₂OC(=O)OEt | O |
| C-9767 | Me(R) | single bond | H | c-Pr | H | Me | CH₂OC(=O)OEt | O |
| C-9768 | Me(S) | single bond | H | c-Pr | H | Me | CH₂OC(=O)OEt | O |
| C-9769 | H | single bond | Me | c-Pr(1-CN) | H | Me | CH₂OC(=O)OEt | O |
| C-9770 | Me | single bond | H | c-Pr(1-CN) | H | Me | CH₂OC(=O)OEt | O |
| C-9771 | Me(R) | single bond | H | c-Pr(1-CN) | H | Me | CH₂OC(=O)OEt | O |
| C-9772 | Me(S) | single bond | H | c-Pr(1-CN) | H | Me | CH₂OC(=O)OEt | O |
| C-9773 | H | single bond | Me | CH₂CF₃ | H | Cl | CH₂OC(=O)O(*i*-Pr) | O |
| C-9774 | Me | single bond | H | CH₂CF₃ | H | Cl | CH₂OC(=O)O(*i*-Pr) | O |
| C-9775 | Me(R) | single bond | H | CH₂CF₃ | H | Cl | CH₂OC(=O)O(*i*-Pr) | O |
| C-9776 | Me(S) | single bond | H | CH₂CF₃ | H | Cl | CH₂OC(=O)O(*i*-Pr) | O |
| C-9777 | H | single bond | Me | Et | H | Cl | CH₂OC(=O)O(*i*-Pr) | O |
| C-9778 | Me | single bond | H | Et | H | Cl | CH₂OC(=O)O(*i*-Pr) | O |
| C-9779 | Me(R) | single bond | H | Et | H | Cl | CH₂OC(=O)O(*i*-Pr) | O |
| C-9780 | Me(S) | single bond | H | Et | H | Cl | CH₂OC(=O)O(*i*-Pr) | O |
| C-9781 | H | single bond | Me | c-Pr | H | Cl | CH₂OC(=O)O(*i*-Pr) | O |
| C-9782 | Me | single bond | H | c-Pr | H | Cl | CH₂OC(=O)O(*i*-Pr) | O |
| C-9783 | Me(R) | single bond | H | c-Pr | H | Cl | CH₂OC(=O)O(*i*-Pr) | O |
| C-9784 | Me(S) | single bond | H | c-Pr | H | Cl | CH₂OC(=O)O(*i*-Pr) | O |
| C-9785 | H | single bond | Me | c-Pr(1-CN) | H | Cl | CH₂OC(=O)O(*i*-Pr) | O |
| C-9786 | Me | single bond | H | c-Pr(1-CN) | H | Cl | CH₂OC(=O)O(*i*-Pr) | O |
| C-9787 | Me(R) | single bond | H | c-Pr(1-CN) | H | Cl | CH₂OC(=O)O(*i*-Pr) | O |
| C-9788 | Me(S) | single bond | H | c-Pr(1-CN) | H | Cl | CH₂OC(=O)O(*i*-Pr) | O |
| C-9789 | H | single bond | Me | CH₂CF₃ | H | Me | CH₂OC(=O)O(*i*-Pr) | O |
| C-9790 | Me | single bond | H | CH₂CF₃ | H | Me | CH₂OC(=O)O(*i*-Pr) | O |
| C-9791 | Me(R) | single bond | H | CH₂CF₃ | H | Me | CH₂OC(=O)O(*i*-Pr) | O |
| C-9792 | Me(S) | single bond | H | CH₂CF₃ | H | Me | CH₂OC(=O)O(*i*-Pr) | O |
| C-9793 | H | single bond | Me | Et | H | Me | CH₂OC(=O)O(*i*-Pr) | O |
| C-9794 | Me | single bond | H | Et | H | Me | CH₂OC(=O)O(*i*-Pr) | O |
| C-9795 | Me(R) | single bond | H | Et | H | Me | CH₂OC(=O)O(*i*-Pr) | O |
| C-9796 | Me(S) | single bond | H | Et | H | Me | CH₂OC(=O)O(*i*-Pr) | O |
| C-9797 | H | single bond | Me | c-Pr | H | Me | CH₂OC(=O)O(*i*-Pr) | O |
| C-9798 | Me | single bond | H | c-Pr | H | Me | CH₂OC(=O)O(*i*-Pr) | O |
| C-9799 | Me(R) | single bond | H | c-Pr | H | Me | CH₂OC(=O)O(*i*-Pr) | O |
| C-9800 | Me(S) | single bond | H | c-Pr | H | Me | CH₂OC(=O)O(*i*-Pr) | O |
| C-9801 | H | single bond | Me | c-Pr(1-CN) | H | Me | CH₂OC(=O)O(*i*-Pr) | O |
| C-9802 | Me | single bond | H | c-Pr(1-CN) | H | Me | CH₂OC(=O)O(*i*-Pr) | O |

**[Table 451]**

| Compound Number | Y | W | Z | R¹ | R² | R³ | R¹⁰ | A |
|---|---|---|---|---|---|---|---|---|
| C-9803 | Me(R) | single bond | H | c-Pr(1-CN) | H | Me | CH₂OC(=O)O(*i*-Pr) | O |
| C-9804 | Me(S) | single bond | H | c-Pr(1-CN) | H | Me | CH₂OC(=O)O(*i*-Pr) | O |
| C-9805 | H | single bond | Me | CH₂CF₃ | H | Cl | CH₂N(Me)C(=O)OMe | O |
| C-9806 | Me | single bond | H | CH₂CF₃ | H | Cl | CH₂N(Me)C(=O)OMe | O |
| C-9807 | Me(R) | single bond | H | CH₂CF₃ | H | Cl | CH₂N(Me)C(=O)OMe | O |
| C-9808 | Me(S) | single bond | H | CH₂CF₃ | H | Cl | CH₂N(Me)C(=O)OMe | O |
| C-9809 | H | single bond | Me | Et | H | Cl | CH₂N(Me)C(=O)OMe | O |
| C-9810 | Me | single bond | H | Et | H | Cl | CH₂N(Me)C(=O)OMe | O |
| C-9811 | Me(R) | single bond | H | Et | H | Cl | CH₂N(Me)C(=O)OMe | O |
| C-9812 | Me(S) | single bond | H | Et | H | Cl | CH₂N(Me)C(=O)OMe | O |
| C-9813 | H | single bond | Me | c-Pr | H | Cl | CH₂N(Me)C(=O)OMe | O |
| C-9814 | Me | single bond | H | c-Pr | H | Cl | CH₂N(Me)C(=O)OMe | O |
| C-9815 | Me(R) | single bond | H | c-Pr | H | Cl | CH₂N(Me)C(=O)OMe | O |
| C-9816 | Me(S) | single bond | H | c-Pr | H | Cl | CH₂N(Me)C(=O)OMe | O |
| C-9817 | H | single bond | Me | c-Pr(1-CN) | H | Cl | CH₂N(Me)C(=O)OMe | O |
| C-9818 | Me | single bond | H | c-Pr(1-CN) | H | Cl | CH₂N(Me)C(=O)OMe | O |
| C-9819 | Me(R) | single bond | H | c-Pr(1-CN) | H | Cl | CH₂N(Me)C(=O)OMe | O |
| C-9820 | Me(S) | single bond | H | c-Pr(1-CN) | H | Cl | CH₂N(Me)C(=O)OMe | O |
| C-9821 | H | single bond | Me | CH₂CF₃ | H | Me | CH₂N(Me)C(=O)OMe | O |
| C-9822 | Me | single bond | H | CH₂CF₃ | H | Me | CH₂N(Me)C(=O)OMe | O |
| C-9823 | Me(R) | single bond | H | CH₂CF₃ | H | Me | CH₂N(Me)C(=O)OMe | O |
| C-9824 | Me(S) | single bond | H | CH₂CF₃ | H | Me | CH₂N(Me)C(=O)OMe | O |
| C-9825 | H | single bond | Me | Et | H | Me | CH₂N(Me)C(=O)OMe | O |
| C-9826 | Me | single bond | H | Et | H | Me | CH₂N(Me)C(=O)OMe | O |
| C-9827 | Me(R) | single bond | H | Et | H | Me | CH₂N(Me)C(=O)OMe | O |
| C-9828 | Me(S) | single bond | H | Et | H | Me | CH₂N(Me)C(=O)OMe | O |
| C-9829 | H | single bond | Me | c-Pr | H | Me | CH₂N(Me)C(=O)OMe | O |
| C-9830 | Me | single bond | H | c-Pr | H | Me | CH₂N(Me)C(=O)OMe | O |
| C-9831 | Me(R) | single bond | H | c-Pr | H | Me | CH₂N(Me)C(=O)OMe | O |
| C-9832 | Me(S) | single bond | H | c-Pr | H | Me | CH₂N(Me)C(=O)OMe | O |
| C-9833 | H | single bond | Me | c-Pr(1-CN) | H | Me | CH₂N(Me)C(=O)OMe | O |
| C-9834 | Me | single bond | H | c-Pr(1-CN) | H | Me | CH₂N(Me)C(=O)OMe | O |
| C-9835 | Me(R) | single bond | H | c-Pr(1-CN) | H | Me | CH₂N(Me)C(=O)OMe | O |
| C-9836 | Me(S) | single bond | H | c-Pr(1-CN) | H | Me | CH₂N(Me)C(=O)OMe | O |
| C-9837 | H | single bond | Me | CH₂CF₃ | H | Cl | CH₂OCH₂CH₂OMe | O |
| C-9838 | Me | single bond | H | CH₂CF₃ | H | Cl | CH₂OCH₂CH₂OMe | O |
| C-9839 | Me(R) | single bond | H | CH₂CF₃ | H | Cl | CH₂OCH₂CH₂OMe | O |
| C-9840 | Me(S) | single bond | H | CH₂CF₃ | H | Cl | CH₂OCH₂CH₂OMe | O |
| C-9841 | H | single bond | Me | Et | H | Cl | CH₂OCH₂CH₂OMe | O |
| C-9842 | Me | single bond | H | Et | H | Cl | CH₂OCH₂CH₂OMe | O |
| C-9843 | Me(R) | single bond | H | Et | H | Cl | CH₂OCH₂CH₂OMe | O |
| C-9844 | Me(S) | single bond | H | Et | H | Cl | CH₂OCH₂CH₂OMe | O |
| C-9845 | H | single bond | Me | c-Pr | H | Cl | CH₂OCH₂CH₂OMe | O |
| C-9846 | Me | single bond | H | c-Pr | H | Cl | CH₂OCH₂CH₂OMe | O |

**[Table 452]**

| Compound Number | Y | W | Z | R¹ | R² | R³ | R¹⁰ | A |
|---|---|---|---|---|---|---|---|---|
| C-9847 | Me(R) | single bond | H | c-Pr | H | Cl | CH₂OCH₂CH₂OMe | O |
| C-9848 | Me(S) | single bond | H | c-Pr | H | Cl | CH₂OCH₂CH₂OMe | O |
| C-9849 | H | single bond | Me | c-Pr(1-CN) | H | Cl | CH₂OCH₂CH₂OMe | O |
| C-9850 | Me | single bond | H | c-Pr(1-CN) | H | Cl | CH₂OCH₂CH₂OMe | O |
| C-9851 | Me(R) | single bond | H | c-Pr(1-CN) | H | Cl | CH₂OCH₂CH₂OMe | O |
| C-9852 | Me(S) | single bond | H | c-Pr(1-CN) | H | Cl | CH₂OCH₂CH₂OMe | O |
| C-9853 | H | single bond | Me | CH₂CF₃ | H | Me | CH₂OCH₂CH₂OMe | O |
| C-9854 | Me | single bond | H | CH₂CF₃ | H | Me | CH₂OCH₂CH₂OMe | O |
| C-9855 | Me(R) | single bond | H | CH₂CF₃ | H | Me | CH₂OCH₂CH₂OMe | O |
| C-9856 | Me(S) | single bond | H | CH₂CF₃ | H | Me | CH₂OCH₂CH₂OMe | O |
| C-9857 | H | single bond | Me | Et | H | Me | CH₂OCH₂CH₂OMe | O |
| C-9858 | Me | single bond | H | Et | H | Me | CH₂OCH₂CH₂OMe | O |
| C-9859 | Me(R) | single bond | H | Et | H | Me | CH₂OCH₂CH₂OMe | O |
| C-9860 | Me(S) | single bond | H | Et | H | Me | CH₂OCH₂CH₂OMe | O |
| C-9861 | H | single bond | Me | c-Pr | H | Me | CH₂OCH₂CH₂OMe | O |
| C-9862 | Me | single bond | H | c-Pr | H | Me | CH₂OCH₂CH₂OMe | O |
| C-9863 | Me(R) | single bond | H | c-Pr | H | Me | CH₂OCH₂CH₂OMe | O |
| C-9864 | Me(S) | single bond | H | c-Pr | H | Me | CH₂OCH₂CH₂OMe | O |
| C-9865 | H | single bond | Me | c-Pr(1-CN) | H | Me | CH₂OCH₂CH₂OMe | O |
| C-9866 | Me | single bond | H | c-Pr(1-CN) | H | Me | CH₂OCH₂CH₂OMe | O |
| C-9867 | Me(R) | single bond | H | c-Pr(1-CN) | H | Me | CH₂OCH₂CH₂OMe | O |
| C-9868 | Me(S) | single bond | H | c-Pr(1-CN) | H | Me | CH₂OCH₂CH₂OMe | O |
| C-9869 | H | single bond | Me | CH₂CF₃ | H | Cl | CH₂OCH₂CH₂CN | O |
| C-9870 | Me | single bond | H | CH₂CF₃ | H | Cl | CH₂OCH₂CH₂CN | O |
| C-9871 | Me(R) | single bond | H | CH₂CF₃ | H | Cl | CH₂OCH₂CH₂CN | O |
| C-9872 | Me(S) | single bond | H | CH₂CF₃ | H | Cl | CH₂OCH₂CH₂CN | O |
| C-9873 | H | single bond | Me | Et | H | Cl | CH₂OCH₂CH₂CN | O |
| C-9874 | Me | single bond | H | Et | H | Cl | CH₂OCH₂CH₂CN | O |
| C-9875 | Me(R) | single bond | H | Et | H | Cl | CH₂OCH₂CH₂CN | O |
| C-9876 | Me(S) | single bond | H | Et | H | Cl | CH₂OCH₂CH₂CN | O |
| C-9877 | H | single bond | Me | c-Pr | H | Cl | CH₂OCH₂CH₂CN | O |
| C-9878 | Me | single bond | H | c-Pr | H | Cl | CH₂OCH₂CH₂CN | O |
| C-9879 | Me(R) | single bond | H | c-Pr | H | Cl | CH₂OCH₂CH₂CN | O |
| C-9880 | Me(S) | single bond | H | c-Pr | H | Cl | CH₂OCH₂CH₂CN | O |
| C-9881 | H | single bond | Me | c-Pr(1-CN) | H | Cl | CH₂OCH₂CH₂CN | O |
| C-9882 | Me | single bond | H | c-Pr(1-CN) | H | Cl | CH₂OCH₂CH₂CN | O |
| C-9883 | Me(R) | single bond | H | c-Pr(1-CN) | H | Cl | CH₂OCH₂CH₂CN | O |
| C-9884 | Me(S) | single bond | H | c-Pr(1-CN) | H | Cl | CH₂OCH₂CH₂CN | O |
| C-9885 | H | single bond | Me | CH₂CF₃ | H | Me | CH₂OCH₂CH₂CN | O |
| C-9886 | Me | single bond | H | CH₂CF₃ | H | Me | CH₂OCH₂CH₂CN | O |
| C-9887 | Me(R) | single bond | H | CH₂CF₃ | H | Me | CH₂OCH₂CH₂CN | O |
| C-9888 | Me(S) | single bond | H | CH₂CF₃ | H | Me | CH₂OCH₂CH₂CN | O |
| C-9889 | H | single bond | Me | Et | H | Me | CH₂OCH₂CH₂CN | O |
| C-9890 | Me | single bond | H | Et | H | Me | CH₂OCH₂CH₂CN | O |

**[Table 453]**

| Compound Number | Y | W | Z | R¹ | R² | R³ | R¹⁰ | A |
|---|---|---|---|---|---|---|---|---|
| C-9891 | Me(R) | single bond | H | Et | H | Me | CH₂OCH₂CH₂CN | O |
| C-9892 | Me(S) | single bond | H | Et | H | Me | CH₂OCH₂CH₂CN | O |
| C-9893 | H | single bond | Me | c-Pr | H | Me | CH₂OCH₂CH₂CN | O |
| C-9894 | Me | single bond | H | c-Pr | H | Me | CH₂OCH₂CH₂CN | O |
| C-9895 | Me(R) | single bond | H | c-Pr | H | Me | CH₂OCH₂CH₂CN | O |
| C-9896 | Me(S) | single bond | H | c-Pr | H | Me | CH₂OCH₂CH₂CN | O |
| C-9897 | H | single bond | Me | c-Pr(1-CN) | H | Me | CH₂OCH₂CH₂CN | O |
| C-9898 | Me | single bond | H | c-Pr(1-CN) | H | Me | CH₂OCH₂CH₂CN | O |
| C-9899 | Me(R) | single bond | H | c-Pr(1-CN) | H | Me | CH₂OCH₂CH₂CN | O |
| C-9900 | Me(S) | single bond | H | c-Pr(1-CN) | H | Me | CH₂OCH₂CH₂CN | O |
| C-9901 | H | single bond | Me | CH₂CF₃ | H | Cl | CH₂OCH₂CH₂S(=O)₂Me | O |
| C-9902 | Me | single bond | H | CH₂CF₃ | H | Cl | CH₂OCH₂CH₂S(=O)₂Me | O |
| C-9903 | Me(R) | single bond | H | CH₂CF₃ | H | Cl | CH₂OCH₂CH₂S(=O)₂Me | O |
| C-9904 | Me(S) | single bond | H | CH₂CF₃ | H | Cl | CH₂OCH₂CH₂S(=O)₂Me | O |
| C-9905 | H | single bond | Me | Et | H | Cl | CH₂OCH₂CH₂S(=O)₂Me | O |
| C-9906 | Me | single bond | H | Et | H | Cl | CH₂OCH₂CH₂S(=O)₂Me | O |
| C-9907 | Me(R) | single bond | H | Et | H | Cl | CH₂OCH₂CH₂S(=O)₂Me | O |
| C-9908 | Me(S) | single bond | H | Et | H | Cl | CH₂OCH₂CH₂S(=O)₂Me | O |
| C-9909 | H | single bond | Me | c-Pr | H | Cl | CH₂OCH₂CH₂S(=O)₂Me | O |
| C-9910 | Me | single bond | H | c-Pr | H | Cl | CH₂OCH₂CH₂S(=O)₂Me | O |
| C-9911 | Me(R) | single bond | H | c-Pr | H | Cl | CH₂OCH₂CH₂S(=O)₂Me | O |
| C-9912 | Me(S) | single bond | H | c-Pr | H | Cl | CH₂OCH₂CH₂S(=O)₂Me | O |
| C-9913 | H | single bond | Me | c-Pr(1-CN) | H | Cl | CH₂OCH₂CH₂S(=O)₂Me | O |
| C-9914 | Me | single bond | H | c-Pr(1-CN) | H | Cl | CH₂OCH₂CH₂S(=O)₂Me | O |
| C-9915 | Me(R) | single bond | H | c-Pr(1-CN) | H | Cl | CH₂OCH₂CH₂S(=O)₂Me | O |
| C-9916 | Me(S) | single bond | H | c-Pr(1-CN) | H | Cl | CH₂OCH₂CH₂S(=O)₂Me | O |
| C-9917 | H | single bond | Me | CH₂CF₃ | H | Me | CH₂OCH₂CH₂S(=O)₂Me | O |
| C-9918 | Me | single bond | H | CH₂CF₃ | H | Me | CH₂OCH₂CH₂S(=O)₂Me | O |
| C-9919 | Me(R) | single bond | H | CH₂CF₃ | H | Me | CH₂OCH₂CH₂S(=O)₂Me | O |
| C-9920 | Me(S) | single bond | H | CH₂CF₃ | H | Me | CH₂OCH₂CH₂S(=O)₂Me | O |
| C-9921 | H | single bond | Me | Et | H | Me | CH₂OCH₂CH₂S(=O)₂Me | O |
| C-9922 | Me | single bond | H | Et | H | Me | CH₂OCH₂CH₂S(=O)₂Me | O |
| C-9923 | Me(R) | single bond | H | Et | H | Me | CH₂OCH₂CH₂S(=O)₂Me | O |
| C-9924 | Me(S) | single bond | H | Et | H | Me | CH₂OCH₂CH₂S(=O)₂Me | O |
| C-9925 | H | single bond | Me | c-Pr | H | Me | CH₂OCH₂CH₂S(=O)₂Me | O |
| C-9926 | Me | single bond | H | c-Pr | H | Me | CH₂OCH₂CH₂S(=O)₂Me | O |
| C-9927 | Me(R) | single bond | H | c-Pr | H | Me | CH₂OCH₂CH₂S(=O)₂Me | O |
| C-9928 | Me(S) | single bond | H | c-Pr | H | Me | CH₂OCH₂CH₂S(=O)₂Me | O |
| C-9929 | H | single bond | Me | c-Pr(1-CN) | H | Me | CH₂OCH₂CH₂S(=O)₂Me | O |
| C-9930 | Me | single bond | H | c-Pr(1-CN) | H | Me | CH₂OCH₂CH₂S(=O)₂Me | O |
| C-9931 | Me(R) | single bond | H | c-Pr(1-CN) | H | Me | CH₂OCH₂CH₂S(=O)₂Me | O |
| C-9932 | Me(S) | single bond | H | c-Pr(1-CN) | H | Me | CH₂OCH₂CH₂S(=O)₂Me | O |
| C-9933 | H | single bond | Me | CH₂CF₃ | H | Cl | CH₂(5-Me-1,3-dioxol-2-one-4-yl) | O |
| C-9934 | Me | single bond | H | CH₂CF₃ | H | Cl | CH₂(5-Me-1,3-dioxol-2-one-4-yl) | O |

**[Table 454]**

| Compound Number | Y | W | Z | R¹ | R² | R³ | R¹⁰ | A |
|---|---|---|---|---|---|---|---|---|
| C-9935 | Me(R) | single bond | H | CH₂CF₃ | H | Cl | CH₂(5-Me-1,3-dioxol-2-one-4-yl) | O |
| C-9936 | Me(S) | single bond | H | CH₂CF₃ | H | Cl | CH₂(5-Me-1,3-dioxol-2-one-4-yl) | O |
| C-9937 | H | single bond | Me | Et | H | Cl | CH₂(5-Me-1,3-dioxol-2-one-4-yl) | O |
| C-9938 | Me | single bond | H | Et | H | Cl | CH₂(5-Me-1,3-dioxol-2-one-4-yl) | O |
| C-9939 | Me(R) | single bond | H | Et | H | Cl | CH₂(5-Me-1,3-dioxol-2-one-4-yl) | O |
| C-9940 | Me(S) | single bond | H | Et | H | Cl | CH₂(5-Me-1,3-dioxol-2-one-4-yl) | O |
| C-9941 | H | single bond | Me | c-Pr | H | Cl | CH₂(5-Me-1,3-dioxol-2-one-4-yl) | O |
| C-9942 | Me | single bond | H | c-Pr | H | Cl | CH₂(5-Me-1,3-dioxol-2-one-4-yl) | O |
| C-9943 | Me(R) | single bond | H | c-Pr | H | Cl | CH₂(5-Me-1,3-dioxol-2-one-4-yl) | O |
| C-9944 | Me(S) | single bond | H | c-Pr | H | Cl | CH₂(5-Me-1,3-dioxol-2-one-4-yl) | O |
| C-9945 | H | single bond | Me | c-Pr(1-CN) | H | Cl | CH₂(5-Me-1,3-dioxol-2-one-4-yl) | O |
| C-9946 | Me | single bond | H | c-Pr(1-CN) | H | Cl | CH₂(5-Me-1,3-dioxol-2-one-4-yl) | O |
| C-9947 | Me(R) | single bond | H | c-Pr(1-CN) | H | Cl | CH₂(5-Me-1,3-dioxol-2-one-4-yl) | O |
| C-9948 | Me(S) | single bond | H | c-Pr(1-CN) | H | Cl | CH₂(5-Me-1,3-dioxol-2-one-4-yl) | O |
| C-9949 | H | single bond | Me | CH₂CF₃ | H | Me | CH₂(5-Me-1,3-dioxol-2-one-4-yl) | O |
| C-9950 | Me | single bond | H | CH₂CF₃ | H | Me | CH₂(5-Me-1,3-dioxol-2-one-4-yl) | O |
| C-9951 | Me(R) | single bond | H | CH₂CF₃ | H | Me | CH₂(5-Me-1,3-dioxol-2-one-4-yl) | O |
| C-9952 | Me(S) | single bond | H | CH₂CF₃ | H | Me | CH₂(5-Me-1,3-dioxol-2-one-4-yl) | O |
| C-9953 | H | single bond | Me | Et | H | Me | CH₂(5-Me-1,3-dioxol-2-one-4-yl) | O |
| C-9954 | Me | single bond | H | Et | H | Me | CH₂(5-Me-1,3-dioxol-2-one-4-yl) | O |
| C-9955 | Me(R) | single bond | H | Et | H | Me | CH₂(5-Me-1,3-dioxol-2-one-4-yl) | O |
| C-9956 | Me(S) | single bond | H | Et | H | Me | CH₂(5-Me-1,3-dioxol-2-one-4-yl) | O |
| C-9957 | H | single bond | Me | c-Pr | H | Me | CH₂(5-Me-1,3-dioxol-2-one-4-yl) | O |
| C-9958 | Me | single bond | H | c-Pr | H | Me | CH₂(5-Me-1,3-dioxol-2-one-4-yl) | O |
| C-9959 | Me(R) | single bond | H | c-Pr | H | Me | CH₂(5-Me-1,3-dioxol-2-one-4-yl) | O |
| C-9960 | Me(S) | single bond | H | c-Pr | H | Me | CH₂(5-Me-1,3-dioxol-2-one-4-yl) | O |
| C-9961 | H | single bond | Me | c-Pr(1-CN) | H | Me | CH₂(5-Me-1,3-dioxol-2-one-4-yl) | O |
| C-9962 | Me | single bond | H | c-Pr(1-CN) | H | Me | CH₂(5-Me-1,3-dioxol-2-one-4-yl) | O |
| C-9963 | Me(R) | single bond | H | c-Pr(1-CN) | H | Me | CH₂(5-Me-1,3-dioxol-2-one-4-yl) | O |
| C-9964 | Me(S) | single bond | H | c-Pr(1-CN) | H | Me | CH₂(5-Me-1,3-dioxol-2-one-4-yl) | O |
| C-9965 | H | single bond | Me | CH₂CF₃ | Me | Cl | *c*-Pr | O |
| C-9966 | Me | single bond | H | CH₂CF₃ | Me | Cl | *c*-Pr | O |
| C-9967 | Me(R) | single bond | H | CH₂CF₃ | Me | Cl | *c*-Pr | O |
| C-9968 | Me(S) | single bond | H | CH₂CF₃ | Me | Cl | *c*-Pr | O |
| C-9969 | H | single bond | Me | Et | Me | Cl | *c*-Pr | O |
| C-9970 | Me | single bond | H | Et | Me | Cl | *c*-Pr | O |
| C-9971 | Me(R) | single bond | H | Et | Me | Cl | *c*-Pr | O |
| C-9972 | Me(S) | single bond | H | Et | Me | Cl | *c*-Pr | O |
| C-9973 | H | single bond | Me | c-Pr | Me | Cl | *c*-Pr | O |
| C-9974 | Me | single bond | H | c-Pr | Me | Cl | *c*-Pr | O |
| C-9975 | Me(R) | single bond | H | c-Pr | Me | Cl | *c*-Pr | O |
| C-9976 | Me(S) | single bond | H | c-Pr | Me | Cl | *c*-Pr | O |
| C-9977 | H | single bond | Me | c-Pr(1-CN) | Me | Cl | *c*-Pr | O |
| C-9978 | Me | single bond | H | c-Pr(1-CN) | Me | Cl | *c*-Pr | O |

**[Table 455]**

| Compound Number | Y | W | Z | R¹ | R² | R³ | R¹⁰ | A |
|---|---|---|---|---|---|---|---|---|
| C-9979 | Me(R) | single bond | H | c-Pr(1-CN) | Me | Cl | *c*-Pr | O |
| C-9980 | Me(S) | single bond | H | c-Pr(1-CN) | Me | Cl | *c*-Pr | O |
| C-9981 | H | single bond | Me | CH₂CF₃ | Me | Me | *c*-Pr | O |
| C-9982 | Me | single bond | H | CH₂CF₃ | Me | Me | *c*-Pr | O |
| C-9983 | Me(R) | single bond | H | CH₂CF₃ | Me | Me | *c*-Pr | O |
| C-9984 | Me(S) | single bond | H | CH₂CF₃ | Me | Me | *c*-Pr | O |
| C-9985 | H | single bond | Me | Et | Me | Me | *c*-Pr | O |
| C-9986 | Me | single bond | H | Et | Me | Me | *c*-Pr | O |
| C-9987 | Me(R) | single bond | H | Et | Me | Me | *c*-Pr | O |
| C-9988 | Me(S) | single bond | H | Et | Me | Me | *c*-Pr | O |
| C-9989 | H | single bond | Me | c-Pr | Me | Me | *c*-Pr | O |
| C-9990 | Me | single bond | H | c-Pr | Me | Me | *c*-Pr | O |
| C-9991 | Me(R) | single bond | H | c-Pr | Me | Me | *c*-Pr | O |
| C-9992 | Me(S) | single bond | H | c-Pr | Me | Me | *c*-Pr | O |
| C-9993 | H | single bond | Me | c-Pr(1-CN) | Me | Me | *c*-Pr | O |
| C-9994 | Me | single bond | H | c-Pr(1-CN) | Me | Me | *c*-Pr | O |
| C-9995 | Me(R) | single bond | H | c-Pr(1-CN) | Me | Me | *c*-Pr | O |
| C-9996 | Me(S) | single bond | H | c-Pr(1-CN) | Me | Me | *c*-Pr | O |
| C-9997 | H | single bond | Me | CH₂CF₃ | Me | Cl | OMe | O |
| C-9998 | Me | single bond | H | CH₂CF₃ | Me | Cl | OMe | O |
| C-9999 | Me(R) | single bond | H | CH₂CF₃ | Me | Cl | OMe | O |
| C-10000 | Me(S) | single bond | H | CH₂CF₃ | Me | Cl | OMe | O |
| C-10001 | H | single bond | Me | Et | Me | Cl | OMe | O |
| C-10002 | Me | single bond | H | Et | Me | Cl | OMe | O |
| C-10003 | Me(R) | single bond | H | Et | Me | Cl | OMe | O |
| C-10004 | Me(S) | single bond | H | Et | Me | Cl | OMe | O |
| C-10005 | H | single bond | Me | c-Pr | Me | Cl | OMe | O |
| C-10006 | Me | single bond | H | c-Pr | Me | Cl | OMe | O |
| C-10007 | Me(R) | single bond | H | c-Pr | Me | Cl | OMe | O |
| C-10008 | Me(S) | single bond | H | c-Pr | Me | Cl | OMe | O |
| C-10009 | H | single bond | Me | c-Pr(1-CN) | Me | Cl | OMe | O |
| C-10010 | Me | single bond | H | c-Pr(1-CN) | Me | Cl | OMe | O |
| C-10011 | Me(R) | single bond | H | c-Pr(1-CN) | Me | Cl | OMe | O |
| C-10012 | Me(S) | single bond | H | c-Pr(1-CN) | Me | Cl | OMe | O |
| C-10013 | H | single bond | Me | CH₂CF₃ | Me | Me | OMe | O |
| C-10014 | Me | single bond | H | CH₂CF₃ | Me | Me | OMe | O |
| C-10015 | Me(R) | single bond | H | CH₂CF₃ | Me | Me | OMe | O |
| C-10016 | Me(S) | single bond | H | CH₂CF₃ | Me | Me | OMe | O |
| C-10017 | H | single bond | Me | Et | Me | Me | OMe | O |
| C-10018 | Me | single bond | H | Et | Me | Me | OMe | O |
| C-10019 | Me(R) | single bond | H | Et | Me | Me | OMe | O |
| C-10020 | Me(S) | single bond | H | Et | Me | Me | OMe | O |
| C-10021 | H | single bond | Me | c-Pr | Me | Me | OMe | O |
| C-10022 | Me | single bond | H | c-Pr | Me | Me | OMe | O |

**[Table 456]**

| Compound Number | Y | W | Z | R¹ | R² | R³ | R¹⁰ | A |
|---|---|---|---|---|---|---|---|---|
| C-10023 | Me(R) | single bond | H | c-Pr | Me | Me | OMe | O |
| C-10024 | Me(S) | single bond | H | c-Pr | Me | Me | OMe | O |
| C-10025 | H | single bond | Me | c-Pr(1-CN) | Me | Me | OMe | O |
| C-10026 | Me | single bond | H | c-Pr(1-CN) | Me | Me | OMe | O |
| C-10027 | Me(R) | single bond | H | c-Pr(1-CN) | Me | Me | OMe | O |
| C-10028 | Me(S) | single bond | H | c-Pr(1-CN) | Me | Me | OMe | O |
| C-10029 | H | single bond | Me | CH₂CF₃ | Me | Cl | C(=O)(*i*-Pr) | O |
| C-10030 | Me | single bond | H | CH₂CF₃ | Me | Cl | C(=O)(*i*-Pr) | O |
| C-10031 | Me(R) | single bond | H | CH₂CF₃ | Me | Cl | C(=O)(*i*-Pr) | O |
| C-10032 | Me(S) | single bond | H | CH₂CF₃ | Me | Cl | C(=O)(*i*-Pr) | O |
| C-10033 | H | single bond | Me | Et | Me | Cl | C(=O)(*i*-Pr) | O |
| C-10034 | Me | single bond | H | Et | Me | Cl | C(=O)(*i*-Pr) | O |
| C-10035 | Me(R) | single bond | H | Et | Me | Cl | C(=O)(*i*-Pr) | O |
| C-10036 | Me(S) | single bond | H | Et | Me | Cl | C(=O)(*i*-Pr) | O |
| C-10037 | H | single bond | Me | c-Pr | Me | Cl | C(=O)(*i*-Pr) | O |
| C-10038 | Me | single bond | H | c-Pr | Me | Cl | C(=O)(*i*-Pr) | O |
| C-10039 | Me(R) | single bond | H | c-Pr | Me | Cl | C(=O)(*i*-Pr) | O |
| C-10040 | Me(S) | single bond | H | c-Pr | Me | Cl | C(=O)(*i*-Pr) | O |
| C-10041 | H | single bond | Me | c-Pr(1-CN) | Me | Cl | C(=O)(*i*-Pr) | O |
| C-10042 | Me | single bond | H | c-Pr(1-CN) | Me | Cl | C(=O)(*i*-Pr) | O |
| C-10043 | Me(R) | single bond | H | c-Pr(1-CN) | Me | Cl | C(=O)(*i*-Pr) | O |
| C-10044 | Me(S) | single bond | H | c-Pr(1-CN) | Me | Cl | C(=O)(*i*-Pr) | O |
| C-10045 | H | single bond | Me | CH₂CF₃ | Me | Me | C(=O)(*i*-Pr) | O |
| C-10046 | Me | single bond | H | CH₂CF₃ | Me | Me | C(=O)(*i*-Pr) | O |
| C-10047 | Me(R) | single bond | H | CH₂CF₃ | Me | Me | C(=O)(*i*-Pr) | O |
| C-10048 | Me(S) | single bond | H | CH₂CF₃ | Me | Me | C(=O)(*i*-Pr) | O |
| C-10049 | H | single bond | Me | Et | Me | Me | C(=O)(*i*-Pr) | O |
| C-10050 | Me | single bond | H | Et | Me | Me | C(=O)(*i*-Pr) | O |
| C-10051 | Me(R) | single bond | H | Et | Me | Me | C(=O)(*i*-Pr) | O |
| C-10052 | Me(S) | single bond | H | Et | Me | Me | C(=O)(*i*-Pr) | O |
| C-10053 | H | single bond | Me | c-Pr | Me | Me | C(=O)(*i*-Pr) | O |
| C-10054 | Me | single bond | H | c-Pr | Me | Me | C(=O)(*i*-Pr) | O |
| C-10055 | Me(R) | single bond | H | c-Pr | Me | Me | C(=O)(*i*-Pr) | O |
| C-10056 | Me(S) | single bond | H | c-Pr | Me | Me | C(=O)(*i*-Pr) | O |
| C-10057 | H | single bond | Me | c-Pr(1-CN) | Me | Me | C(=O)(*i*-Pr) | O |
| C-10058 | Me | single bond | H | c-Pr(1-CN) | Me | Me | C(=O)(*i*-Pr) | O |
| C-10059 | Me(R) | single bond | H | c-Pr(1-CN) | Me | Me | C(=O)(*i*-Pr) | O |
| C-10060 | Me(S) | single bond | H | c-Pr(1-CN) | Me | Me | C(=O)(*i*-Pr) | O |
| C-10061 | H | single bond | Me | CH₂CF₃ | Me | Cl | C(=O)O(*t*-Bu) | O |
| C-10062 | Me | single bond | H | CH₂CF₃ | Me | Cl | C(=O)O(*t*-Bu) | O |
| C-10063 | Me(R) | single bond | H | CH₂CF₃ | Me | Cl | C(=O)O(*t*-Bu) | O |
| C-10064 | Me(S) | single bond | H | CH₂CF₃ | Me | Cl | C(=O)O(*t*-Bu) | O |
| C-10065 | H | single bond | Me | Et | Me | Cl | C(=O)O(*t*-Bu) | O |
| C-10066 | Me | single bond | H | Et | Me | Cl | C(=O)O(*t*-Bu) | O |

**[Table 457]**

| Compound Number | Y | W | Z | R¹ | R² | R³ | R¹⁰ | A |
|---|---|---|---|---|---|---|---|---|
| C-10067 | Me(R) | single bond | H | Et | Me | Cl | C(=O)O(*t*-Bu) | O |
| C-10068 | Me(S) | single bond | H | Et | Me | Cl | C(=O)O(*t*-Bu) | O |
| C-10069 | H | single bond | Me | c-Pr | Me | Cl | C(=O)O(*t*-Bu) | O |
| C-10070 | Me | single bond | H | c-Pr | Me | Cl | C(=O)O(*t*-Bu) | O |
| C-10071 | Me(R) | single bond | H | c-Pr | Me | Cl | C(=O)O(*t*-Bu) | O |
| C-10072 | Me(S) | single bond | H | c-Pr | Me | Cl | C(=O)O(*t*-Bu) | O |
| C-10073 | H | single bond | Me | c-Pr(1-CN) | Me | Cl | C(=O)O(*t*-Bu) | O |
| C-10074 | Me | single bond | H | c-Pr(1-CN) | Me | Cl | C(=O)O(*t*-Bu) | O |
| C-10075 | Me(R) | single bond | H | c-Pr(1-CN) | Me | Cl | C(=O)O(*t*-Bu) | O |
| C-10076 | Me(S) | single bond | H | c-Pr(1-CN) | Me | Cl | C(=O)O(*t*-Bu) | O |
| C-10077 | H | single bond | Me | CH₂CF₃ | Me | Me | C(=O)O(*t*-Bu) | O |
| C-10078 | Me | single bond | H | CH₂CF₃ | Me | Me | C(=O)O(*t*-Bu) | O |
| C-10079 | Me(R) | single bond | H | CH₂CF₃ | Me | Me | C(=O)O(*t*-Bu) | O |
| C-10080 | Me(S) | single bond | H | CH₂CF₃ | Me | Me | C(=O)O(*t*-Bu) | O |
| C-10081 | H | single bond | Me | Et | Me | Me | C(=O)O(*t*-Bu) | O |
| C-10082 | Me | single bond | H | Et | Me | Me | C(=O)O(*t*-Bu) | O |
| C-10083 | Me(R) | single bond | H | Et | Me | Me | C(=O)O(*t*-Bu) | O |
| C-10084 | Me(S) | single bond | H | Et | Me | Me | C(=O)O(*t*-Bu) | O |
| C-10085 | H | single bond | Me | c-Pr | Me | Me | C(=O)O(*t*-Bu) | O |
| C-10086 | Me | single bond | H | c-Pr | Me | Me | C(=O)O(*t*-Bu) | O |
| C-10087 | Me(R) | single bond | H | c-Pr | Me | Me | C(=O)O(*t*-Bu) | O |
| C-10088 | Me(S) | single bond | H | c-Pr | Me | Me | C(=O)O(*t*-Bu) | O |
| C-10089 | H | single bond | Me | c-Pr(1-CN) | Me | Me | C(=O)O(*t*-Bu) | O |
| C-10090 | Me | single bond | H | c-Pr(1-CN) | Me | Me | C(=O)O(*t*-Bu) | O |
| C-10091 | Me(R) | single bond | H | c-Pr(1-CN) | Me | Me | C(=O)O(*t*-Bu) | O |
| C-10092 | Me(S) | single bond | H | c-Pr(1-CN) | Me | Me | C(=O)O(*t*-Bu) | O |
| C-10093 | H | single bond | Me | CH₂CF₃ | Me | Cl | CH₂OEt | O |
| C-10094 | Me | single bond | H | CH₂CF₃ | Me | Cl | CH₂OEt | O |
| C-10095 | Me(R) | single bond | H | CH₂CF₃ | Me | Cl | CH₂OEt | O |
| C-10096 | Me(S) | single bond | H | CH₂CF₃ | Me | Cl | CH₂OEt | O |
| C-10097 | H | single bond | Me | Et | Me | Cl | CH₂OEt | O |
| C-10098 | Me | single bond | H | Et | Me | Cl | CH₂OEt | O |
| C-10099 | Me(R) | single bond | H | Et | Me | Cl | CH₂OEt | O |
| C-10100 | Me(S) | single bond | H | Et | Me | Cl | CH₂OEt | O |
| C-10101 | H | single bond | Me | c-Pr | Me | Cl | CH₂OEt | O |
| C-10102 | Me | single bond | H | c-Pr | Me | Cl | CH₂OEt | O |
| C-10103 | Me(R) | single bond | H | c-Pr | Me | Cl | CH₂OEt | O |
| C-10104 | Me(S) | single bond | H | c-Pr | Me | Cl | CH₂OEt | O |
| C-10105 | H | single bond | Me | c-Pr(1-CN) | Me | Cl | CH₂OEt | O |
| C-10106 | Me | single bond | H | c-Pr(1-CN) | Me | Cl | CH₂OEt | O |
| C-10107 | Me(R) | single bond | H | c-Pr(1-CN) | Me | Cl | CH₂OEt | O |
| C-10108 | Me(S) | single bond | H | c-Pr(1-CN) | Me | Cl | CH₂OEt | O |
| C-10109 | H | single bond | Me | CH₂CF₃ | Me | Me | CH₂OEt | O |
| C-10110 | Me | single bond | H | CH₂CF₃ | Me | Me | CH₂OEt | O |

**[Table 458]**

| Compound Number | Y | W | Z | R¹ | R² | R³ | R¹⁰ | A |
|---|---|---|---|---|---|---|---|---|
| C-10111 | Me(R) | single bond | H | CH₂CF₃ | Me | Me | CH₂OEt | O |
| C-10112 | Me(S) | single bond | H | CH₂CF₃ | Me | Me | CH₂OEt | O |
| C-10113 | H | single bond | Me | Et | Me | Me | CH₂OEt | O |
| C-10114 | Me | single bond | H | Et | Me | Me | CH₂OEt | O |
| C-10115 | Me(R) | single bond | H | Et | Me | Me | CH₂OEt | O |
| C-10116 | Me(S) | single bond | H | Et | Me | Me | CH₂OEt | O |
| C-10117 | H | single bond | Me | c-Pr | Me | Me | CH₂OEt | O |
| C-10118 | Me | single bond | H | c-Pr | Me | Me | CH₂OEt | O |
| C-10119 | Me(R) | single bond | H | c-Pr | Me | Me | CH₂OEt | O |
| C-10120 | Me(S) | single bond | H | c-Pr | Me | Me | CH₂OEt | O |
| C-10121 | H | single bond | Me | c-Pr(1-CN) | Me | Me | CH₂OEt | O |
| C-10122 | Me | single bond | H | c-Pr(1-CN) | Me | Me | CH₂OEt | O |
| C-10123 | Me(R) | single bond | H | c-Pr(1-CN) | Me | Me | CH₂OEt | O |
| C-10124 | Me(S) | single bond | H | c-Pr(1-CN) | Me | Me | CH₂OEt | O |
| C-10125 | H | single bond | Me | CH₂CF₃ | Me | Cl | CH₂SMe | O |
| C-10126 | Me | single bond | H | CH₂CF₃ | Me | Cl | CH₂SMe | O |
| C-10127 | Me(R) | single bond | H | CH₂CF₃ | Me | Cl | CH₂SMe | O |
| C-10128 | Me(S) | single bond | H | CH₂CF₃ | Me | Cl | CH₂SMe | O |
| C-10129 | H | single bond | Me | Et | Me | Cl | CH₂SMe | O |
| C-10130 | Me | single bond | H | Et | Me | Cl | CH₂SMe | O |
| C-10131 | Me(R) | single bond | H | Et | Me | Cl | CH₂SMe | O |
| C-10132 | Me(S) | single bond | H | Et | Me | Cl | CH₂SMe | O |
| C-10133 | H | single bond | Me | c-Pr | Me | Cl | CH₂SMe | O |
| C-10134 | Me | single bond | H | c-Pr | Me | Cl | CH₂SMe | O |
| C-10135 | Me(R) | single bond | H | c-Pr | Me | Cl | CH₂SMe | O |
| C-10136 | Me(S) | single bond | H | c-Pr | Me | Cl | CH₂SMe | O |
| C-10137 | H | single bond | Me | c-Pr(1-CN) | Me | Cl | CH₂SMe | O |
| C-10138 | Me | single bond | H | c-Pr(1-CN) | Me | Cl | CH₂SMe | O |
| C-10139 | Me(R) | single bond | H | c-Pr(1-CN) | Me | Cl | CH₂SMe | O |
| C-10140 | Me(S) | single bond | H | c-Pr(1-CN) | Me | Cl | CH₂SMe | O |
| C-10141 | H | single bond | Me | CH₂CF₃ | Me | Me | CH₂SMe | O |
| C-10142 | Me | single bond | H | CH₂CF₃ | Me | Me | CH₂SMe | O |
| C-10143 | Me(R) | single bond | H | CH₂CF₃ | Me | Me | CH₂SMe | O |
| C-10144 | Me(S) | single bond | H | CH₂CF₃ | Me | Me | CH₂SMe | O |
| C-10145 | H | single bond | Me | Et | Me | Me | CH₂SMe | O |
| C-10146 | Me | single bond | H | Et | Me | Me | CH₂SMe | O |
| C-10147 | Me(R) | single bond | H | Et | Me | Me | CH₂SMe | O |
| C-10148 | Me(S) | single bond | H | Et | Me | Me | CH₂SMe | O |
| C-10149 | H | single bond | Me | c-Pr | Me | Me | CH₂SMe | O |
| C-10150 | Me | single bond | H | c-Pr | Me | Me | CH₂SMe | O |
| C-10151 | Me(R) | single bond | H | c-Pr | Me | Me | CH₂SMe | O |
| C-10152 | Me(S) | single bond | H | c-Pr | Me | Me | CH₂SMe | O |
| C-10153 | H | single bond | Me | c-Pr(1-CN) | Me | Me | CH₂SMe | O |
| C-10154 | Me | single bond | H | c-Pr(1-CN) | Me | Me | CH₂SMe | O |

**[Table 459]**

| Compound Number | Y | W | Z | R¹ | R² | R³ | R¹⁰ | A |
|---|---|---|---|---|---|---|---|---|
| C-10155 | Me(R) | single bond | H | c-Pr(1-CN) | Me | Me | CH₂SMe | O |
| C-10156 | Me(S) | single bond | H | c-Pr(1-CN) | Me | Me | CH₂SMe | O |
| C-10157 | H | single bond | Me | CH₂CF₃ | Me | Cl | CH₂S(=O)₂Me | O |
| C-10158 | Me | single bond | H | CH₂CF₃ | Me | Cl | CH₂S(=O)₂Me | O |
| C-10159 | Me(R) | single bond | H | CH₂CF₃ | Me | Cl | CH₂S(=O)₂Me | O |
| C-10160 | Me(S) | single bond | H | CH₂CF₃ | Me | Cl | CH₂S(=O)₂Me | O |
| C-10161 | H | single bond | Me | Et | Me | Cl | CH₂S(=O)₂Me | O |
| C-10162 | Me | single bond | H | Et | Me | Cl | CH₂S(=O)₂Me | O |
| C-10163 | Me(R) | single bond | H | Et | Me | Cl | CH₂S(=O)₂Me | O |
| C-10164 | Me(S) | single bond | H | Et | Me | Cl | CH₂S(=O)₂Me | O |
| C-10165 | H | single bond | Me | c-Pr | Me | Cl | CH₂S(=O)₂Me | O |
| C-10166 | Me | single bond | H | c-Pr | Me | Cl | CH₂S(=O)₂Me | O |
| C-10167 | Me(R) | single bond | H | c-Pr | Me | Cl | CH₂S(=O)₂Me | O |
| C-10168 | Me(S) | single bond | H | c-Pr | Me | Cl | CH₂S(=O)₂Me | O |
| C-10169 | H | single bond | Me | c-Pr(1-CN) | Me | Cl | CH₂S(=O)₂Me | O |
| C-10170 | Me | single bond | H | c-Pr(1-CN) | Me | Cl | CH₂S(=O)₂Me | O |
| C-10171 | Me(R) | single bond | H | c-Pr(1-CN) | Me | Cl | CH₂S(=O)₂Me | O |
| C-10172 | Me(S) | single bond | H | c-Pr(1-CN) | Me | Cl | CH₂S(=O)₂Me | O |
| C-10173 | H | single bond | Me | CH₂CF₃ | Me | Me | CH₂S(=O)₂Me | O |
| C-10174 | Me | single bond | H | CH₂CF₃ | Me | Me | CH₂S(=O)₂Me | O |
| C-10175 | Me(R) | single bond | H | CH₂CF₃ | Me | Me | CH₂S(=O)₂Me | O |
| C-10176 | Me(S) | single bond | H | CH₂CF₃ | Me | Me | CH₂S(=O)₂Me | O |
| C-10177 | H | single bond | Me | Et | Me | Me | CH₂S(=O)₂Me | O |
| C-10178 | Me | single bond | H | Et | Me | Me | CH₂S(=O)₂Me | O |
| C-10179 | Me(R) | single bond | H | Et | Me | Me | CH₂S(=O)₂Me | O |
| C-10180 | Me(S) | single bond | H | Et | Me | Me | CH₂S(=O)₂Me | O |
| C-10181 | H | single bond | Me | c-Pr | Me | Me | CH₂S(=O)₂Me | O |
| C-10182 | Me | single bond | H | c-Pr | Me | Me | CH₂S(=O)₂Me | O |
| C-10183 | Me(R) | single bond | H | c-Pr | Me | Me | CH₂S(=O)₂Me | O |
| C-10184 | Me(S) | single bond | H | c-Pr | Me | Me | CH₂S(=O)₂Me | O |
| C-10185 | H | single bond | Me | c-Pr(1-CN) | Me | Me | CH₂S(=O)₂Me | O |
| C-10186 | Me | single bond | H | c-Pr(1-CN) | Me | Me | CH₂S(=O)₂Me | O |
| C-10187 | Me(R) | single bond | H | c-Pr(1-CN) | Me | Me | CH₂S(=O)₂Me | O |
| C-10188 | Me(S) | single bond | H | c-Pr(1-CN) | Me | Me | CH₂S(=O)₂Me | O |
| C-10189 | H | single bond | Me | CH₂CF₃ | Me | Cl | CH₂C(=O)Me | O |
| C-10190 | Me | single bond | H | CH₂CF₃ | Me | Cl | CH₂C(=O)Me | O |
| C-10191 | Me(R) | single bond | H | CH₂CF₃ | Me | Cl | CH₂C(=O)Me | O |
| C-10192 | Me(S) | single bond | H | CH₂CF₃ | Me | Cl | CH₂C(=O)Me | O |
| C-10193 | H | single bond | Me | Et | Me | Cl | CH₂C(=O)Me | O |
| C-10194 | Me | single bond | H | Et | Me | Cl | CH₂C(=O)Me | O |
| C-10195 | Me(R) | single bond | H | Et | Me | Cl | CH₂C(=O)Me | O |
| C-10196 | Me(S) | single bond | H | Et | Me | Cl | CH₂C(=O)Me | O |
| C-10197 | H | single bond | Me | c-Pr | Me | Cl | CH₂C(=O)Me | O |
| C-10198 | Me | single bond | H | c-Pr | Me | Cl | CH₂C(=O)Me | O |

**[Table 460]**

| Compound Number | Y | W | Z | R¹ | R² | R³ | R¹⁰ | A |
|---|---|---|---|---|---|---|---|---|
| C-10199 | Me(R) | single bond | H | c-Pr | Me | Cl | CH₂C(=O)Me | O |
| C-10200 | Me(S) | single bond | H | c-Pr | Me | Cl | CH₂C(=O)Me | O |
| C-10201 | H | single bond | Me | c-Pr(1-CN) | Me | Cl | CH₂C(=O)Me | O |
| C-10202 | Me | single bond | H | c-Pr(1-CN) | Me | Cl | CH₂C(=O)Me | O |
| C-10203 | Me(R) | single bond | H | c-Pr(1-CN) | Me | Cl | CH₂C(=O)Me | O |
| C-10204 | Me(S) | single bond | H | c-Pr(1-CN) | Me | Cl | CH₂C(=O)Me | O |
| C-10205 | H | single bond | Me | CH₂CF₃ | Me | Me | CH₂C(=O)Me | O |
| C-10206 | Me | single bond | H | CH₂CF₃ | Me | Me | CH₂C(=O)Me | O |
| C-10207 | Me(R) | single bond | H | CH₂CF₃ | Me | Me | CH₂C(=O)Me | O |
| C-10208 | Me(S) | single bond | H | CH₂CF₃ | Me | Me | CH₂C(=O)Me | O |
| C-10209 | H | single bond | Me | Et | Me | Me | CH₂C(=O)Me | O |
| C-10210 | Me | single bond | H | Et | Me | Me | CH₂C(=O)Me | O |
| C-10211 | Me(R) | single bond | H | Et | Me | Me | CH₂C(=O)Me | O |
| C-10212 | Me(S) | single bond | H | Et | Me | Me | CH₂C(=O)Me | O |
| C-10213 | H | single bond | Me | c-Pr | Me | Me | CH₂C(=O)Me | O |
| C-10214 | Me | single bond | H | c-Pr | Me | Me | CH₂C(=O)Me | O |
| C-10215 | Me(R) | single bond | H | c-Pr | Me | Me | CH₂C(=O)Me | O |
| C-10216 | Me(S) | single bond | H | c-Pr | Me | Me | CH₂C(=O)Me | O |
| C-10217 | H | single bond | Me | c-Pr(1-CN) | Me | Me | CH₂C(=O)Me | O |
| C-10218 | Me | single bond | H | c-Pr(1-CN) | Me | Me | CH₂C(=O)Me | O |
| C-10219 | Me(R) | single bond | H | c-Pr(1-CN) | Me | Me | CH₂C(=O)Me | O |
| C-10220 | Me(S) | single bond | H | c-Pr(1-CN) | Me | Me | CH₂C(=O)Me | O |
| C-10221 | H | single bond | Me | CH₂CF₃ | Me | Cl | CH₂C(=O)OMe | O |
| C-10222 | Me | single bond | H | CH₂CF₃ | Me | Cl | CH₂C(=O)OMe | O |
| C-10223 | Me(R) | single bond | H | CH₂CF₃ | Me | Cl | CH₂C(=O)OMe | O |
| C-10224 | Me(S) | single bond | H | CH₂CF₃ | Me | Cl | CH₂C(=O)OMe | O |
| C-10225 | H | single bond | Me | Et | Me | Cl | CH₂C(=O)OMe | O |
| C-10226 | Me | single bond | H | Et | Me | Cl | CH₂C(=O)OMe | O |
| C-10227 | Me(R) | single bond | H | Et | Me | Cl | CH₂C(=O)OMe | O |
| C-10228 | Me(S) | single bond | H | Et | Me | Cl | CH₂C(=O)OMe | O |
| C-10229 | H | single bond | Me | c-Pr | Me | Cl | CH₂C(=O)OMe | O |
| C-10230 | Me | single bond | H | c-Pr | Me | Cl | CH₂C(=O)OMe | O |
| C-10231 | Me(R) | single bond | H | c-Pr | Me | Cl | CH₂C(=O)OMe | O |
| C-10232 | Me(S) | single bond | H | c-Pr | Me | Cl | CH₂C(=O)OMe | O |
| C-10233 | H | single bond | Me | c-Pr(1-CN) | Me | Cl | CH₂C(=O)OMe | O |
| C-10234 | Me | single bond | H | c-Pr(1-CN) | Me | Cl | CH₂C(=O)OMe | O |
| C-10235 | Me(R) | single bond | H | c-Pr(1-CN) | Me | Cl | CH₂C(=O)OMe | O |
| C-10236 | Me(S) | single bond | H | c-Pr(1-CN) | Me | Cl | CH₂C(=O)OMe | O |
| C-10237 | H | single bond | Me | CH₂CF₃ | Me | Me | CH₂C(=O)OMe | O |
| C-10238 | Me | single bond | H | CH₂CF₃ | Me | Me | CH₂C(=O)OMe | O |
| C-10239 | Me(R) | single bond | H | CH₂CF₃ | Me | Me | CH₂C(=O)OMe | O |
| C-10240 | Me(S) | single bond | H | CH₂CF₃ | Me | Me | CH₂C(=O)OMe | O |
| C-10241 | H | single bond | Me | Et | Me | Me | CH₂C(=O)OMe | O |
| C-10242 | Me | single bond | H | Et | Me | Me | CH₂C(=O)OMe | O |

**[Table 461]**

| Compound Number | Y | W | Z | R¹ | R² | R³ | R¹⁰ | A |
|---|---|---|---|---|---|---|---|---|
| C-10243 | Me(R) | single bond | H | Et | Me | Me | CH₂C(=O)OMe | O |
| C-10244 | Me(S) | single bond | H | Et | Me | Me | CH₂C(=O)OMe | O |
| C-10245 | H | single bond | Me | c-Pr | Me | Me | CH₂C(=O)OMe | O |
| C-10246 | Me | single bond | H | c-Pr | Me | Me | CH₂C(=O)OMe | O |
| C-10247 | Me(R) | single bond | H | c-Pr | Me | Me | CH₂C(=O)OMe | O |
| C-10248 | Me(S) | single bond | H | c-Pr | Me | Me | CH₂C(=O)OMe | O |
| C-10249 | H | single bond | Me | c-Pr(1-CN) | Me | Me | CH₂C(=O)OMe | O |
| C-10250 | Me | single bond | H | c-Pr(1-CN) | Me | Me | CH₂C(=O)OMe | O |
| C-10251 | Me(R) | single bond | H | c-Pr(1-CN) | Me | Me | CH₂C(=O)OMe | O |
| C-10252 | Me(S) | single bond | H | c-Pr(1-CN) | Me | Me | CH₂C(=O)OMe | O |
| C-10253 | H | single bond | Me | CH₂CF₃ | Me | Cl | CH₂C(=O)NHMe | O |
| C-10254 | Me | single bond | H | CH₂CF₃ | Me | Cl | CH₂C(=O)NHMe | O |
| C-10255 | Me(R) | single bond | H | CH₂CF₃ | Me | Cl | CH₂C(=O)NHMe | O |
| C-10256 | Me(S) | single bond | H | CH₂CF₃ | Me | Cl | CH₂C(=O)NHMe | O |
| C-10257 | H | single bond | Me | Et | Me | Cl | CH₂C(=O)NHMe | O |
| C-10258 | Me | single bond | H | Et | Me | Cl | CH₂C(=O)NHMe | O |
| C-10259 | Me(R) | single bond | H | Et | Me | Cl | CH₂C(=O)NHMe | O |
| C-10260 | Me(S) | single bond | H | Et | Me | Cl | CH₂C(=O)NHMe | O |
| C-10261 | H | single bond | Me | c-Pr | Me | Cl | CH₂C(=O)NHMe | O |
| C-10262 | Me | single bond | H | c-Pr | Me | Cl | CH₂C(=O)NHMe | O |
| C-10263 | Me(R) | single bond | H | c-Pr | Me | Cl | CH₂C(=O)NHMe | O |
| C-10264 | Me(S) | single bond | H | c-Pr | Me | Cl | CH₂C(=O)NHMe | O |
| C-10265 | H | single bond | Me | c-Pr(1-CN) | Me | Cl | CH₂C(=O)NHMe | O |
| C-10266 | Me | single bond | H | c-Pr(1-CN) | Me | Cl | CH₂C(=O)NHMe | O |
| C-10267 | Me(R) | single bond | H | c-Pr(1-CN) | Me | Cl | CH₂C(=O)NHMe | O |
| C-10268 | Me(S) | single bond | H | c-Pr(1-CN) | Me | Cl | CH₂C(=O)NHMe | O |
| C-10269 | H | single bond | Me | CH₂CF₃ | Me | Me | CH₂C(=O)NHMe | O |
| C-10270 | Me | single bond | H | CH₂CF₃ | Me | Me | CH₂C(=O)NHMe | O |
| C-10271 | Me(R) | single bond | H | CH₂CF₃ | Me | Me | CH₂C(=O)NHMe | O |
| C-10272 | Me(S) | single bond | H | CH₂CF₃ | Me | Me | CH₂C(=O)NHMe | O |
| C-10273 | H | single bond | Me | Et | Me | Me | CH₂C(=O)NHMe | O |
| C-10274 | Me | single bond | H | Et | Me | Me | CH₂C(=O)NHMe | O |
| C-10275 | Me(R) | single bond | H | Et | Me | Me | CH₂C(=O)NHMe | O |
| C-10276 | Me(S) | single bond | H | Et | Me | Me | CH₂C(=O)NHMe | O |
| C-10277 | H | single bond | Me | c-Pr | Me | Me | CH₂C(=O)NHMe | O |
| C-10278 | Me | single bond | H | c-Pr | Me | Me | CH₂C(=O)NHMe | O |
| C-10279 | Me(R) | single bond | H | c-Pr | Me | Me | CH₂C(=O)NHMe | O |
| C-10280 | Me(S) | single bond | H | c-Pr | Me | Me | CH₂C(=O)NHMe | O |
| C-10281 | H | single bond | Me | c-Pr(1-CN) | Me | Me | CH₂C(=O)NHMe | O |
| C-10282 | Me | single bond | H | c-Pr(1-CN) | Me | Me | CH₂C(=O)NHMe | O |
| C-10283 | Me(R) | single bond | H | c-Pr(1-CN) | Me | Me | CH₂C(=O)NHMe | O |
| C-10284 | Me(S) | single bond | H | c-Pr(1-CN) | Me | Me | CH₂C(=O)NHMe | O |
| C-10285 | H | single bond | Me | CH₂CF₃ | Me | Cl | CH₂OC(=O)Me | O |
| C-10286 | Me | single bond | H | CH₂CF₃ | Me | Cl | CH₂OC(=O)Me | O |

**[Table 462]**

| Compound Number | Y | W | Z | R¹ | R² | R³ | R¹⁰ | A |
|---|---|---|---|---|---|---|---|---|
| C-10287 | Me(R) | single bond | H | CH₂CF₃ | Me | Cl | CH₂OC(=O)Me | O |
| C-10288 | Me(S) | single bond | H | CH₂CF₃ | Me | Cl | CH₂OC(=O)Me | O |
| C-10289 | H | single bond | Me | Et | Me | Cl | CH₂OC(=O)Me | O |
| C-10290 | Me | single bond | H | Et | Me | Cl | CH₂OC(=O)Me | O |
| C-10291 | Me(R) | single bond | H | Et | Me | Cl | CH₂OC(=O)Me | O |
| C-10292 | Me(S) | single bond | H | Et | Me | Cl | CH₂OC(=O)Me | O |
| C-10293 | H | single bond | Me | c-Pr | Me | Cl | CH₂OC(=O)Me | O |
| C-10294 | Me | single bond | H | c-Pr | Me | Cl | CH₂OC(=O)Me | O |
| C-10295 | Me(R) | single bond | H | c-Pr | Me | Cl | CH₂OC(=O)Me | O |
| C-10296 | Me(S) | single bond | H | c-Pr | Me | Cl | CH₂OC(=O)Me | O |
| C-10297 | H | single bond | Me | c-Pr(1-CN) | Me | Cl | CH₂OC(=O)Me | O |
| C-10298 | Me | single bond | H | c-Pr(1-CN) | Me | Cl | CH₂OC(=O)Me | O |
| C-10299 | Me(R) | single bond | H | c-Pr(1-CN) | Me | Cl | CH₂OC(=O)Me | O |
| C-10300 | Me(S) | single bond | H | c-Pr(1-CN) | Me | Cl | CH₂OC(=O)Me | O |
| C-10301 | H | single bond | Me | CH₂CF₃ | Me | Me | CH₂OC(=O)Me | O |
| C-10302 | Me | single bond | H | CH₂CF₃ | Me | Me | CH₂OC(=O)Me | O |
| C-10303 | Me(R) | single bond | H | CH₂CF₃ | Me | Me | CH₂OC(=O)Me | O |
| C-10304 | Me(S) | single bond | H | CH₂CF₃ | Me | Me | CH₂OC(=O)Me | O |
| C-10305 | H | single bond | Me | Et | Me | Me | CH₂OC(=O)Me | O |
| C-10306 | Me | single bond | H | Et | Me | Me | CH₂OC(=O)Me | O |
| C-10307 | Me(R) | single bond | H | Et | Me | Me | CH₂OC(=O)Me | O |
| C-10308 | Me(S) | single bond | H | Et | Me | Me | CH₂OC(=O)Me | O |
| C-10309 | H | single bond | Me | c-Pr | Me | Me | CH₂OC(=O)Me | O |
| C-10310 | Me | single bond | H | c-Pr | Me | Me | CH₂OC(=O)Me | O |
| C-10311 | Me(R) | single bond | H | c-Pr | Me | Me | CH₂OC(=O)Me | O |
| C-10312 | Me(S) | single bond | H | c-Pr | Me | Me | CH₂OC(=O)Me | O |
| C-10313 | H | single bond | Me | c-Pr(1-CN) | Me | Me | CH₂OC(=O)Me | O |
| C-10314 | Me | single bond | H | c-Pr(1-CN) | Me | Me | CH₂OC(=O)Me | O |
| C-10315 | Me(R) | single bond | H | c-Pr(1-CN) | Me | Me | CH₂OC(=O)Me | O |
| C-10316 | Me(S) | single bond | H | c-Pr(1-CN) | Me | Me | CH₂OC(=O)Me | O |
| C-10317 | H | single bond | Me | CH₂CF₃ | Me | Cl | CH₂OC(=O)(*i*-Pr) | O |
| C-10318 | Me | single bond | H | CH₂CF₃ | Me | Cl | CH₂OC(=O)(*i*-Pr) | O |
| C-10319 | Me(R) | single bond | H | CH₂CF₃ | Me | Cl | CH₂OC(=O)(*i*-Pr) | O |
| C-10320 | Me(S) | single bond | H | CH₂CF₃ | Me | Cl | CH₂OC(=O)(*i*-Pr) | O |
| C-10321 | H | single bond | Me | Et | Me | Cl | CH₂OC(=O)(*i*-Pr) | O |
| C-10322 | Me | single bond | H | Et | Me | Cl | CH₂OC(=O)(*i*-Pr) | O |
| C-10323 | Me(R) | single bond | H | Et | Me | Cl | CH₂OC(=O)(*i*-Pr) | O |
| C-10324 | Me(S) | single bond | H | Et | Me | Cl | CH₂OC(=O)(*i*-Pr) | O |
| C-10325 | H | single bond | Me | c-Pr | Me | Cl | CH₂OC(=O)(*i*-Pr) | O |
| C-10326 | Me | single bond | H | c-Pr | Me | Cl | CH₂OC(=O)(*i*-Pr) | O |
| C-10327 | Me(R) | single bond | H | c-Pr | Me | Cl | CH₂OC(=O)(*i*-Pr) | O |
| C-10328 | Me(S) | single bond | H | c-Pr | Me | Cl | CH₂OC(=O)(*i*-Pr) | O |
| C-10329 | H | single bond | Me | c-Pr(1-CN) | Me | Cl | CH₂OC(=O)(*i*-Pr) | O |
| C-10330 | Me | single bond | H | c-Pr(1-CN) | Me | Cl | CH₂OC(=O)(*i*-Pr) | O |

**[Table 463]**

| Compound Number | Y | W | Z | R¹ | R² | R³ | R¹⁰ | A |
|---|---|---|---|---|---|---|---|---|
| C-10331 | Me(R) | single bond | H | c-Pr(1-CN) | Me | Cl | CH₂OC(=O)(*i*-Pr) | O |
| C-10332 | Me(S) | single bond | H | c-Pr(1-CN) | Me | Cl | CH₂OC(=O)(*i*-Pr) | O |
| C-10333 | H | single bond | Me | CH₂CF₃ | Me | Me | CH₂OC(=O)(*i*-Pr) | O |
| C-10334 | Me | single bond | H | CH₂CF₃ | Me | Me | CH₂OC(=O)(*i*-Pr) | O |
| C-10335 | Me(R) | single bond | H | CH₂CF₃ | Me | Me | CH₂OC(=O)(*i*-Pr) | O |
| C-10336 | Me(S) | single bond | H | CH₂CF₃ | Me | Me | CH₂OC(=O)(*i*-Pr) | O |
| C-10337 | H | single bond | Me | Et | Me | Me | CH₂OC(=O)(*i*-Pr) | O |
| C-10338 | Me | single bond | H | Et | Me | Me | CH₂OC(=O)(*i*-Pr) | O |
| C-10339 | Me(R) | single bond | H | Et | Me | Me | CH₂OC(=O)(*i*-Pr) | O |
| C-10340 | Me(S) | single bond | H | Et | Me | Me | CH₂OC(=O)(*i*-Pr) | O |
| C-10341 | H | single bond | Me | c-Pr | Me | Me | CH₂OC(=O)(*i*-Pr) | O |
| C-10342 | Me | single bond | H | c-Pr | Me | Me | CH₂OC(=O)(*i*-Pr) | O |
| C-10343 | Me(R) | single bond | H | c-Pr | Me | Me | CH₂OC(=O)(*i*-Pr) | O |
| C-10344 | Me(S) | single bond | H | c-Pr | Me | Me | CH₂OC(=O)(*i*-Pr) | O |
| C-10345 | H | single bond | Me | c-Pr(1-CN) | Me | Me | CH₂OC(=O)(*i*-Pr) | O |
| C-10346 | Me | single bond | H | c-Pr(1-CN) | Me | Me | CH₂OC(=O)(*i*-Pr) | O |
| C-10347 | Me(R) | single bond | H | c-Pr(1-CN) | Me | Me | CH₂OC(=O)(*i*-Pr) | O |
| C-10348 | Me(S) | single bond | H | c-Pr(1-CN) | Me | Me | CH₂OC(=O)(*i*-Pr) | O |
| C-10349 | H | single bond | Me | CH₂CF₃ | Me | Cl | CH₂OC(=O)(*t*-Bu) | O |
| C-10350 | Me | single bond | H | CH₂CF₃ | Me | Cl | CH₂OC(=O)(*t*-Bu) | O |
| C-10351 | Me(R) | single bond | H | CH₂CF₃ | Me | Cl | CH₂OC(=O)(*t*-Bu) | O |
| C-10352 | Me(S) | single bond | H | CH₂CF₃ | Me | Cl | CH₂OC(=O)(*t*-Bu) | O |
| C-10353 | H | single bond | Me | Et | Me | Cl | CH₂OC(=O)(*t*-Bu) | O |
| C-10354 | Me | single bond | H | Et | Me | Cl | CH₂OC(=O)(*t*-Bu) | O |
| C-10355 | Me(R) | single bond | H | Et | Me | Cl | CH₂OC(=O)(*t*-Bu) | O |
| C-10356 | Me(S) | single bond | H | Et | Me | Cl | CH₂OC(=O)(*t*-Bu) | O |
| C-10357 | H | single bond | Me | c-Pr | Me | Cl | CH₂OC(=O)(*t*-Bu) | O |
| C-10358 | Me | single bond | H | c-Pr | Me | Cl | CH₂OC(=O)(*t*-Bu) | O |
| C-10359 | Me(R) | single bond | H | c-Pr | Me | Cl | CH₂OC(=O)(*t*-Bu) | O |
| C-10360 | Me(S) | single bond | H | c-Pr | Me | Cl | CH₂OC(=O)(*t*-Bu) | O |
| C-10361 | H | single bond | Me | c-Pr(1-CN) | Me | Cl | CH₂OC(=O)(*t*-Bu) | O |
| C-10362 | Me | single bond | H | c-Pr(1-CN) | Me | Cl | CH₂OC(=O)(*t*-Bu) | O |
| C-10363 | Me(R) | single bond | H | c-Pr(1-CN) | Me | Cl | CH₂OC(=O)(*t*-Bu) | O |
| C-10364 | Me(S) | single bond | H | c-Pr(1-CN) | Me | Cl | CH₂OC(=O)(*t*-Bu) | O |
| C-10365 | H | single bond | Me | CH₂CF₃ | Me | Me | CH₂OC(=O)(*t*-Bu) | O |
| C-10366 | Me | single bond | H | CH₂CF₃ | Me | Me | CH₂OC(=O)(*t*-Bu) | O |
| C-10367 | Me(R) | single bond | H | CH₂CF₃ | Me | Me | CH₂OC(=O)(*t*-Bu) | O |
| C-10368 | Me(S) | single bond | H | CH₂CF₃ | Me | Me | CH₂OC(=O)(*t*-Bu) | O |
| C-10369 | H | single bond | Me | Et | Me | Me | CH₂OC(=O)(*t*-Bu) | O |
| C-10370 | Me | single bond | H | Et | Me | Me | CH₂OC(=O)(*t*-Bu) | O |
| C-10371 | Me(R) | single bond | H | Et | Me | Me | CH₂OC(=O)(*t*-Bu) | O |
| C-10372 | Me(S) | single bond | H | Et | Me | Me | CH₂OC(=O)(*t*-Bu) | O |
| C-10373 | H | single bond | Me | c-Pr | Me | Me | CH₂OC(=O)(*t*-Bu) | O |
| C-10374 | Me | single bond | H | c-Pr | Me | Me | CH₂OC(=O)(*t*-Bu) | O |

**[Table 464]**

| Compound Number | Y | W | Z | R¹ | R² | R³ | R¹⁰ | A |
|---|---|---|---|---|---|---|---|---|
| C-10375 | Me(R) | single bond | H | c-Pr | Me | Me | CH₂OC(=O)(*t*-Bu) | O |
| C-10376 | Me(S) | single bond | H | c-Pr | Me | Me | CH₂OC(=O)(*t*-Bu) | O |
| C-10377 | H | single bond | Me | c-Pr(1-CN) | Me | Me | CH₂OC(=O)(*t*-Bu) | O |
| C-10378 | Me | single bond | H | c-Pr(1-CN) | Me | Me | CH₂OC(=O)(*t*-Bu) | O |
| C-10379 | Me(R) | single bond | H | c-Pr(1-CN) | Me | Me | CH₂OC(=O)(*t*-Bu) | O |
| C-10380 | Me(S) | single bond | H | c-Pr(1-CN) | Me | Me | CH₂OC(=O)(*t*-Bu) | O |
| C-10381 | H | single bond | Me | CH₂CF₃ | Me | Cl | CH₂OC(=O)OEt | O |
| C-10382 | Me | single bond | H | CH₂CF₃ | Me | Cl | CH₂OC(=O)OEt | O |
| C-10383 | Me(R) | single bond | H | CH₂CF₃ | Me | Cl | CH₂OC(=O)OEt | O |
| C-10384 | Me(S) | single bond | H | CH₂CF₃ | Me | Cl | CH₂OC(=O)OEt | O |
| C-10385 | H | single bond | Me | Et | Me | Cl | CH₂OC(=O)OEt | O |
| C-10386 | Me | single bond | H | Et | Me | Cl | CH₂OC(=O)OEt | O |
| C-10387 | Me(R) | single bond | H | Et | Me | Cl | CH₂OC(=O)OEt | O |
| C-10388 | Me(S) | single bond | H | Et | Me | Cl | CH₂OC(=O)OEt | O |
| C-10389 | H | single bond | Me | c-Pr | Me | Cl | CH₂OC(=O)OEt | O |
| C-10390 | Me | single bond | H | c-Pr | Me | Cl | CH₂OC(=O)OEt | O |
| C-10391 | Me(R) | single bond | H | c-Pr | Me | Cl | CH₂OC(=O)OEt | O |
| C-10392 | Me(S) | single bond | H | c-Pr | Me | Cl | CH₂OC(=O)OEt | O |
| C-10393 | H | single bond | Me | c-Pr(1-CN) | Me | Cl | CH₂OC(=O)OEt | O |
| C-10394 | Me | single bond | H | c-Pr(1-CN) | Me | Cl | CH₂OC(=O)OEt | O |
| C-10395 | Me(R) | single bond | H | c-Pr(1-CN) | Me | Cl | CH₂OC(=O)OEt | O |
| C-10396 | Me(S) | single bond | H | c-Pr(1-CN) | Me | Cl | CH₂OC(=O)OEt | O |
| C-10397 | H | single bond | Me | CH₂CF₃ | Me | Me | CH₂OC(=O)OEt | O |
| C-10398 | Me | single bond | H | CH₂CF₃ | Me | Me | CH₂OC(=O)OEt | O |
| C-10399 | Me(R) | single bond | H | CH₂CF₃ | Me | Me | CH₂OC(=O)OEt | O |
| C-10400 | Me(S) | single bond | H | CH₂CF₃ | Me | Me | CH₂OC(=O)OEt | O |
| C-10401 | H | single bond | Me | Et | Me | Me | CH₂OC(=O)OEt | O |
| C-10402 | Me | single bond | H | Et | Me | Me | CH₂OC(=O)OEt | O |
| C-10403 | Me(R) | single bond | H | Et | Me | Me | CH₂OC(=O)OEt | O |
| C-10404 | Me(S) | single bond | H | Et | Me | Me | CH₂OC(=O)OEt | O |
| C-10405 | H | single bond | Me | c-Pr | Me | Me | CH₂OC(=O)OEt | O |
| C-10406 | Me | single bond | H | c-Pr | Me | Me | CH₂OC(=O)OEt | O |
| C-10407 | Me(R) | single bond | H | c-Pr | Me | Me | CH₂OC(=O)OEt | O |
| C-10408 | Me(S) | single bond | H | c-Pr | Me | Me | CH₂OC(=O)OEt | O |
| C-10409 | H | single bond | Me | c-Pr(1-CN) | Me | Me | CH₂OC(=O)OEt | O |
| C-10410 | Me | single bond | H | c-Pr(1-CN) | Me | Me | CH₂OC(=O)OEt | O |
| C-10411 | Me(R) | single bond | H | c-Pr(1-CN) | Me | Me | CH₂OC(=O)OEt | O |
| C-10412 | Me(S) | single bond | H | c-Pr(1-CN) | Me | Me | CH₂OC(=O)OEt | O |
| C-10413 | H | single bond | Me | CH₂CF₃ | Me | Cl | CH₂OC(=O)O(*i*-Pr) | O |
| C-10414 | Me | single bond | H | CH₂CF₃ | Me | Cl | CH₂OC(=O)O(*i*-Pr) | O |
| C-10415 | Me(R) | single bond | H | CH₂CF₃ | Me | Cl | CH₂OC(=O)O(*i*-Pr) | O |
| C-10416 | Me(S) | single bond | H | CH₂CF₃ | Me | Cl | CH₂OC(=O)O(*i*-Pr) | O |
| C-10417 | H | single bond | Me | Et | Me | Cl | CH₂OC(=O)O(*i*-Pr) | O |
| C-10418 | Me | single bond | H | Et | Me | Cl | CH₂OC(=O)O(*i*-Pr) | O |

**[Table 465]**

| Compound Number | Y | W | Z | R¹ | R² | R³ | R¹⁰ | A |
|---|---|---|---|---|---|---|---|---|
| C-10419 | Me(R) | single bond | H | Et | Me | Cl | CH₂OC(=O)O(*i*-Pr) | O |
| C-10420 | Me(S) | single bond | H | Et | Me | Cl | CH₂OC(=O)O(*i*-Pr) | O |
| C-10421 | H | single bond | Me | c-Pr | Me | Cl | CH₂OC(=O)O(*i*-Pr) | O |
| C-10422 | Me | single bond | H | c-Pr | Me | Cl | CH₂OC(=O)O(*i*-Pr) | O |
| C-10423 | Me(R) | single bond | H | c-Pr | Me | Cl | CH₂OC(=O)O(*i*-Pr) | O |
| C-10424 | Me(S) | single bond | H | c-Pr | Me | Cl | CH₂OC(=O)O(*i*-Pr) | O |
| C-10425 | H | single bond | Me | c-Pr(1-CN) | Me | Cl | CH₂OC(=O)O(*i*-Pr) | O |
| C-10426 | Me | single bond | H | c-Pr(1-CN) | Me | Cl | CH₂OC(=O)O(*i*-Pr) | O |
| C-10427 | Me(R) | single bond | H | c-Pr(1-CN) | Me | Cl | CH₂OC(=O)O(*i*-Pr) | O |
| C-10428 | Me(S) | single bond | H | c-Pr(1-CN) | Me | Cl | CH₂OC(=O)O(*i*-Pr) | O |
| C-10429 | H | single bond | Me | CH₂CF₃ | Me | Me | CH₂OC(=O)O(*i*-Pr) | O |
| C-10430 | Me | single bond | H | CH₂CF₃ | Me | Me | CH₂OC(=O)O(*i*-Pr) | O |
| C-10431 | Me(R) | single bond | H | CH₂CF₃ | Me | Me | CH₂OC(=O)O(*i*-Pr) | O |
| C-10432 | Me(S) | single bond | H | CH₂CF₃ | Me | Me | CH₂OC(=O)O(*i*-Pr) | O |
| C-10433 | H | single bond | Me | Et | Me | Me | CH₂OC(=O)O(*i*-Pr) | O |
| C-10434 | Me | single bond | H | Et | Me | Me | CH₂OC(=O)O(*i*-Pr) | O |
| C-10435 | Me(R) | single bond | H | Et | Me | Me | CH₂OC(=O)O(*i*-Pr) | O |
| C-10436 | Me(S) | single bond | H | Et | Me | Me | CH₂OC(=O)O(*i*-Pr) | O |
| C-10437 | H | single bond | Me | c-Pr | Me | Me | CH₂OC(=O)O(*i*-Pr) | O |
| C-10438 | Me | single bond | H | c-Pr | Me | Me | CH₂OC(=O)O(*i*-Pr) | O |
| C-10439 | Me(R) | single bond | H | c-Pr | Me | Me | CH₂OC(=O)O(*i*-Pr) | O |
| C-10440 | Me(S) | single bond | H | c-Pr | Me | Me | CH₂OC(=O)O(*i*-Pr) | O |
| C-10441 | H | single bond | Me | c-Pr(1-CN) | Me | Me | CH₂OC(=O)O(*i*-Pr) | O |
| C-10442 | Me | single bond | H | c-Pr(1-CN) | Me | Me | CH₂OC(=O)O(*i*-Pr) | O |
| C-10443 | Me(R) | single bond | H | c-Pr(1-CN) | Me | Me | CH₂OC(=O)O(*i*-Pr) | O |
| C-10444 | Me(S) | single bond | H | c-Pr(1-CN) | Me | Me | CH₂OC(=O)O(*i*-Pr) | O |
| C-10445 | H | single bond | Me | CH₂CF₃ | Me | Cl | CH₂N(Me)C(=O)OMe | O |
| C-10446 | Me | single bond | H | CH₂CF₃ | Me | Cl | CH₂N(Me)C(=O)OMe | O |
| C-10447 | Me(R) | single bond | H | CH₂CF₃ | Me | Cl | CH₂N(Me)C(=O)OMe | O |
| C-10448 | Me(S) | single bond | H | CH₂CF₃ | Me | Cl | CH₂N(Me)C(=O)OMe | O |
| C-10449 | H | single bond | Me | Et | Me | Cl | CH₂N(Me)C(=O)OMe | O |
| C-10450 | Me | single bond | H | Et | Me | Cl | CH₂N(Me)C(=O)OMe | O |
| C-10451 | Me(R) | single bond | H | Et | Me | Cl | CH₂N(Me)C(=O)OMe | O |
| C-10452 | Me(S) | single bond | H | Et | Me | Cl | CH₂N(Me)C(=O)OMe | O |
| C-10453 | H | single bond | Me | c-Pr | Me | Cl | CH₂N(Me)C(=O)OMe | O |
| C-10454 | Me | single bond | H | c-Pr | Me | Cl | CH₂N(Me)C(=O)OMe | O |
| C-10455 | Me(R) | single bond | H | c-Pr | Me | Cl | CH₂N(Me)C(=O)OMe | O |
| C-10456 | Me(S) | single bond | H | c-Pr | Me | Cl | CH₂N(Me)C(=O)OMe | O |
| C-10457 | H | single bond | Me | c-Pr(1-CN) | Me | Cl | CH₂N(Me)C(=O)OMe | O |
| C-10458 | Me | single bond | H | c-Pr(1-CN) | Me | Cl | CH₂N(Me)C(=O)OMe | O |
| C-10459 | Me(R) | single bond | H | c-Pr(1-CN) | Me | Cl | CH₂N(Me)C(=O)OMe | O |
| C-10460 | Me(S) | single bond | H | c-Pr(1-CN) | Me | Cl | CH₂N(Me)C(=O)OMe | O |
| C-10461 | H | single bond | Me | CH₂CF₃ | Me | Me | CH₂N(Me)C(=O)OMe | O |
| C-10462 | Me | single bond | H | CH₂CF₃ | Me | Me | CH₂N(Me)C(=O)OMe | O |

**[Table 466]**

| Compound Number | Y | W | Z | R¹ | R² | R³ | R¹⁰ | A |
|---|---|---|---|---|---|---|---|---|
| C-10463 | Me(R) | single bond | H | CH₂CF₃ | Me | Me | CH₂N(Me)C(=O)OMe | O |
| C-10464 | Me(S) | single bond | H | CH₂CF₃ | Me | Me | CH₂N(Me)C(=O)OMe | O |
| C-10465 | H | single bond | Me | Et | Me | Me | CH₂N(Me)C(=O)OMe | O |
| C-10466 | Me | single bond | H | Et | Me | Me | CH₂N(Me)C(=O)OMe | O |
| C-10467 | Me(R) | single bond | H | Et | Me | Me | CH₂N(Me)C(=O)OMe | O |
| C-10468 | Me(S) | single bond | H | Et | Me | Me | CH₂N(Me)C(=O)OMe | O |
| C-10469 | H | single bond | Me | c-Pr | Me | Me | CH₂N(Me)C(=O)OMe | O |
| C-10470 | Me | single bond | H | c-Pr | Me | Me | CH₂N(Me)C(=O)OMe | O |
| C-10471 | Me(R) | single bond | H | c-Pr | Me | Me | CH₂N(Me)C(=O)OMe | O |
| C-10472 | Me(S) | single bond | H | c-Pr | Me | Me | CH₂N(Me)C(=O)OMe | O |
| C-10473 | H | single bond | Me | c-Pr(1-CN) | Me | Me | CH₂N(Me)C(=O)OMe | O |
| C-10474 | Me | single bond | H | c-Pr(1-CN) | Me | Me | CH₂N(Me)C(=O)OMe | O |
| C-10475 | Me(R) | single bond | H | c-Pr(1-CN) | Me | Me | CH₂N(Me)C(=O)OMe | O |
| C-10476 | Me(S) | single bond | H | c-Pr(1-CN) | Me | Me | CH₂N(Me)C(=O)OMe | O |
| C-10477 | H | single bond | Me | CH₂CF₃ | Me | Cl | CH₂OCH₂CH₂OMe | O |
| C-10478 | Me | single bond | H | CH₂CF₃ | Me | Cl | CH₂OCH₂CH₂OMe | O |
| C-10479 | Me(R) | single bond | H | CH₂CF₃ | Me | Cl | CH₂OCH₂CH₂OMe | O |
| C-10480 | Me(S) | single bond | H | CH₂CF₃ | Me | Cl | CH₂OCH₂CH₂OMe | O |
| C-10481 | H | single bond | Me | Et | Me | Cl | CH₂OCH₂CH₂OMe | O |
| C-10482 | Me | single bond | H | Et | Me | Cl | CH₂OCH₂CH₂OMe | O |
| C-10483 | Me(R) | single bond | H | Et | Me | Cl | CH₂OCH₂CH₂OMe | O |
| C-10484 | Me(S) | single bond | H | Et | Me | Cl | CH₂OCH₂CH₂OMe | O |
| C-10485 | H | single bond | Me | c-Pr | Me | Cl | CH₂OCH₂CH₂OMe | O |
| C-10486 | Me | single bond | H | c-Pr | Me | Cl | CH₂OCH₂CH₂OMe | O |
| C-10487 | Me(R) | single bond | H | c-Pr | Me | Cl | CH₂OCH₂CH₂OMe | O |
| C-10488 | Me(S) | single bond | H | c-Pr | Me | Cl | CH₂OCH₂CH₂OMe | O |
| C-10489 | H | single bond | Me | c-Pr(1-CN) | Me | Cl | CH₂OCH₂CH₂OMe | O |
| C-10490 | Me | single bond | H | c-Pr(1-CN) | Me | Cl | CH₂OCH₂CH₂OMe | O |
| C-10491 | Me(R) | single bond | H | c-Pr(1-CN) | Me | Cl | CH₂OCH₂CH₂OMe | O |
| C-10492 | Me(S) | single bond | H | c-Pr(1-CN) | Me | Cl | CH₂OCH₂CH₂OMe | O |
| C-10493 | H | single bond | Me | CH₂CF₃ | Me | Me | CH₂OCH₂CH₂OMe | O |
| C-10494 | Me | single bond | H | CH₂CF₃ | Me | Me | CH₂OCH₂CH₂OMe | O |
| C-10495 | Me(R) | single bond | H | CH₂CF₃ | Me | Me | CH₂OCH₂CH₂OMe | O |
| C-10496 | Me(S) | single bond | H | CH₂CF₃ | Me | Me | CH₂OCH₂CH₂OMe | O |
| C-10497 | H | single bond | Me | Et | Me | Me | CH₂OCH₂CH₂OMe | O |
| C-10498 | Me | single bond | H | Et | Me | Me | CH₂OCH₂CH₂OMe | O |
| C-10499 | Me(R) | single bond | H | Et | Me | Me | CH₂OCH₂CH₂OMe | O |
| C-10500 | Me(S) | single bond | H | Et | Me | Me | CH₂OCH₂CH₂OMe | O |
| C-10501 | H | single bond | Me | c-Pr | Me | Me | CH₂OCH₂CH₂OMe | O |
| C-10502 | Me | single bond | H | c-Pr | Me | Me | CH₂OCH₂CH₂OMe | O |
| C-10503 | Me(R) | single bond | H | c-Pr | Me | Me | CH₂OCH₂CH₂OMe | O |
| C-10504 | Me(S) | single bond | H | c-Pr | Me | Me | CH₂OCH₂CH₂OMe | O |
| C-10505 | H | single bond | Me | c-Pr(1-CN) | Me | Me | CH₂OCH₂CH₂OMe | O |
| C-10506 | Me | single bond | H | c-Pr(1-CN) | Me | Me | CH₂OCH₂CH₂OMe | O |

**[Table 467]**

| Compound Number | Y | W | Z | R¹ | R² | R³ | R¹⁰ | A |
|---|---|---|---|---|---|---|---|---|
| C-10507 | Me(R) | single bond | H | c-Pr(1-CN) | Me | Me | CH₂OCH₂CH₂OMe | O |
| C-10508 | Me(S) | single bond | H | c-Pr(1-CN) | Me | Me | CH₂OCH₂CH₂OMe | O |
| C-10509 | H | single bond | Me | CH₂CF₃ | Me | Cl | CH₂OCH₂CH₂CN | O |
| C-10510 | Me | single bond | H | CH₂CF₃ | Me | Cl | CH₂OCH₂CH₂CN | O |
| C-10511 | Me(R) | single bond | H | CH₂CF₃ | Me | Cl | CH₂OCH₂CH₂CN | O |
| C-10512 | Me(S) | single bond | H | CH₂CF₃ | Me | Cl | CH₂OCH₂CH₂CN | O |
| C-10513 | H | single bond | Me | Et | Me | Cl | CH₂OCH₂CH₂CN | O |
| C-10514 | Me | single bond | H | Et | Me | Cl | CH₂OCH₂CH₂CN | O |
| C-10515 | Me(R) | single bond | H | Et | Me | Cl | CH₂OCH₂CH₂CN | O |
| C-10516 | Me(S) | single bond | H | Et | Me | Cl | CH₂OCH₂CH₂CN | O |
| C-10517 | H | single bond | Me | c-Pr | Me | Cl | CH₂OCH₂CH₂CN | O |
| C-10518 | Me | single bond | H | c-Pr | Me | Cl | CH₂OCH₂CH₂CN | O |
| C-10519 | Me(R) | single bond | H | c-Pr | Me | Cl | CH₂OCH₂CH₂CN | O |
| C-10520 | Me(S) | single bond | H | c-Pr | Me | Cl | CH₂OCH₂CH₂CN | O |
| C-10521 | H | single bond | Me | c-Pr(1-CN) | Me | Cl | CH₂OCH₂CH₂CN | O |
| C-10522 | Me | single bond | H | c-Pr(1-CN) | Me | Cl | CH₂OCH₂CH₂CN | O |
| C-10523 | Me(R) | single bond | H | c-Pr(1-CN) | Me | Cl | CH₂OCH₂CH₂CN | O |
| C-10524 | Me(S) | single bond | H | c-Pr(1-CN) | Me | Cl | CH₂OCH₂CH₂CN | O |
| C-10525 | H | single bond | Me | CH₂CF₃ | Me | Me | CH₂OCH₂CH₂CN | O |
| C-10526 | Me | single bond | H | CH₂CF₃ | Me | Me | CH₂OCH₂CH₂CN | O |
| C-10527 | Me(R) | single bond | H | CH₂CF₃ | Me | Me | CH₂OCH₂CH₂CN | O |
| C-10528 | Me(S) | single bond | H | CH₂CF₃ | Me | Me | CH₂OCH₂CH₂CN | O |
| C-10529 | H | single bond | Me | Et | Me | Me | CH₂OCH₂CH₂CN | O |
| C-10530 | Me | single bond | H | Et | Me | Me | CH₂OCH₂CH₂CN | O |
| C-10531 | Me(R) | single bond | H | Et | Me | Me | CH₂OCH₂CH₂CN | O |
| C-10532 | Me(S) | single bond | H | Et | Me | Me | CH₂OCH₂CH₂CN | O |
| C-10533 | H | single bond | Me | c-Pr | Me | Me | CH₂OCH₂CH₂CN | O |
| C-10534 | Me | single bond | H | c-Pr | Me | Me | CH₂OCH₂CH₂CN | O |
| C-10535 | Me(R) | single bond | H | c-Pr | Me | Me | CH₂OCH₂CH₂CN | O |
| C-10536 | Me(S) | single bond | H | c-Pr | Me | Me | CH₂OCH₂CH₂CN | O |
| C-10537 | H | single bond | Me | c-Pr(1-CN) | Me | Me | CH₂OCH₂CH₂CN | O |
| C-10538 | Me | single bond | H | c-Pr(1-CN) | Me | Me | CH₂OCH₂CH₂CN | O |
| C-10539 | Me(R) | single bond | H | c-Pr(1-CN) | Me | Me | CH₂OCH₂CH₂CN | O |
| C-10540 | Me(S) | single bond | H | c-Pr(1-CN) | Me | Me | CH₂OCH₂CH₂CN | O |
| C-10541 | H | single bond | Me | CH₂CF₃ | Me | Cl | CH₂OCH₂CH₂S(=O)₂Me | O |
| C-10542 | Me | single bond | H | CH₂CF₃ | Me | Cl | CH₂OCH₂CH₂S(=O)₂Me | O |
| C-10543 | Me(R) | single bond | H | CH₂CF₃ | Me | Cl | CH₂OCH₂CH₂S(=O)₂Me | O |
| C-10544 | Me(S) | single bond | H | CH₂CF₃ | Me | Cl | CH₂OCH₂CH₂S(=O)₂Me | O |
| C-10545 | H | single bond | Me | Et | Me | Cl | CH₂OCH₂CH₂S(=O)₂Me | O |
| C-10546 | Me | single bond | H | Et | Me | Cl | CH₂OCH₂CH₂S(=O)₂Me | O |
| C-10547 | Me(R) | single bond | H | Et | Me | Cl | CH₂OCH₂CH₂S(=O)₂Me | O |
| C-10548 | Me(S) | single bond | H | Et | Me | Cl | CH₂OCH₂CH₂S(=O)₂Me | O |
| C-10549 | H | single bond | Me | c-Pr | Me | Cl | CH₂OCH₂CH₂S(=O)₂Me | O |
| C-10550 | Me | single bond | H | c-Pr | Me | Cl | CH₂OCH₂CH₂S(=O)₂Me | O |

**[Table 468]**

| Compound Number | Y | W | Z | R¹ | R² | R³ | R¹⁰ | A |
|---|---|---|---|---|---|---|---|---|
| C-10551 | Me(R) | single bond | H | c-Pr | Me | Cl | CH₂OCH₂CH₂S(=O)₂Me | O |
| C-10552 | Me(S) | single bond | H | c-Pr | Me | Cl | CH₂OCH₂CH₂S(=O)₂Me | O |
| C-10553 | H | single bond | Me | c-Pr(1-CN) | Me | Cl | CH₂OCH₂CH₂S(=O)₂Me | O |
| C-10554 | Me | single bond | H | c-Pr(1-CN) | Me | Cl | CH₂OCH₂CH₂S(=O)₂Me | O |
| C-10555 | Me(R) | single bond | H | c-Pr(1-CN) | Me | Cl | CH₂OCH₂CH₂S(=O)₂Me | O |
| C-10556 | Me(S) | single bond | H | c-Pr(1-CN) | Me | Cl | CH₂OCH₂CH₂S(=O)₂Me | O |
| C-10557 | H | single bond | Me | CH₂CF₃ | Me | Me | CH₂OCH₂CH₂S(=O)₂Me | O |
| C-10558 | Me | single bond | H | CH₂CF₃ | Me | Me | CH₂OCH₂CH₂S(=O)₂Me | O |
| C-10559 | Me(R) | single bond | H | CH₂CF₃ | Me | Me | CH₂OCH₂CH₂S(=O)₂Me | O |
| C-10560 | Me(S) | single bond | H | CH₂CF₃ | Me | Me | CH₂OCH₂CH₂S(=O)₂Me | O |
| C-10561 | H | single bond | Me | Et | Me | Me | CH₂OCH₂CH₂S(=O)₂Me | O |
| C-10562 | Me | single bond | H | Et | Me | Me | CH₂OCH₂CH₂S(=O)₂Me | O |
| C-10563 | Me(R) | single bond | H | Et | Me | Me | CH₂OCH₂CH₂S(=O)₂Me | O |
| C-10564 | Me(S) | single bond | H | Et | Me | Me | CH₂OCH₂CH₂S(=O)₂Me | O |
| C-10565 | H | single bond | Me | c-Pr | Me | Me | CH₂OCH₂CH₂S(=O)₂Me | O |
| C-10566 | Me | single bond | H | c-Pr | Me | Me | CH₂OCH₂CH₂S(=O)₂Me | O |
| C-10567 | Me(R) | single bond | H | c-Pr | Me | Me | CH₂OCH₂CH₂S(=O)₂Me | O |
| C-10568 | Me(S) | single bond | H | c-Pr | Me | Me | CH₂OCH₂CH₂S(=O)₂Me | O |
| C-10569 | H | single bond | Me | c-Pr(1-CN) | Me | Me | CH₂OCH₂CH₂S(=O)₂Me | O |
| C-10570 | Me | single bond | H | c-Pr(1-CN) | Me | Me | CH₂OCH₂CH₂S(=O)₂Me | O |
| C-10571 | Me(R) | single bond | H | c-Pr(1-CN) | Me | Me | CH₂OCH₂CH₂S(=O)₂Me | O |
| C-10572 | Me(S) | single bond | H | c-Pr(1-CN) | Me | Me | CH₂OCH₂CH₂S(=O)₂Me | O |
| C-10573 | H | single bond | Me | CH₂CF₃ | Me | Cl | CH₂(5-Me-1,3-dioxol-2-one-4-yl) | O |
| C-10574 | Me | single bond | H | CH₂CF₃ | Me | Cl | CH₂(5-Me-1,3-dioxol-2-one-4-yl) | O |
| C-10575 | Me(R) | single bond | H | CH₂CF₃ | Me | Cl | CH₂(5-Me-1,3-dioxol-2-one-4-yl) | O |
| C-10576 | Me(S) | single bond | H | CH₂CF₃ | Me | Cl | CH₂(5-Me-1,3-dioxol-2-one-4-yl) | O |
| C-10577 | H | single bond | Me | Et | Me | Cl | CH₂(5-Me-1,3-dioxol-2-one-4-yl) | O |
| C-10578 | Me | single bond | H | Et | Me | Cl | CH₂(5-Me-1,3-dioxol-2-one-4-yl) | O |
| C-10579 | Me(R) | single bond | H | Et | Me | Cl | CH₂(5-Me-1,3-dioxol-2-one-4-yl) | O |
| C-10580 | Me(S) | single bond | H | Et | Me | Cl | CH₂(5-Me-1,3-dioxol-2-one-4-yl) | O |
| C-10581 | H | single bond | Me | c-Pr | Me | Cl | CH₂(5-Me-1,3-dioxol-2-one-4-yl) | O |
| C-10582 | Me | single bond | H | c-Pr | Me | Cl | CH₂(5-Me-1,3-dioxol-2-one-4-yl) | O |
| C-10583 | Me(R) | single bond | H | c-Pr | Me | Cl | CH₂(5-Me-1,3-dioxol-2-one-4-yl) | O |
| C-10584 | Me(S) | single bond | H | c-Pr | Me | Cl | CH₂(5-Me-1,3-dioxol-2-one-4-yl) | O |
| C-10585 | H | single bond | Me | c-Pr(1-CN) | Me | Cl | CH₂(5-Me-1,3-dioxol-2-one-4-yl) | O |
| C-10586 | Me | single bond | H | c-Pr(1-CN) | Me | Cl | CH₂(5-Me-1,3-dioxol-2-one-4-yl) | O |
| C-10587 | Me(R) | single bond | H | c-Pr(1-CN) | Me | Cl | CH₂(5-Me-1,3-dioxol-2-one-4-yl) | O |
| C-10588 | Me(S) | single bond | H | c-Pr(1-CN) | Me | Cl | CH₂(5-Me-1,3-dioxol-2-one-4-yl) | O |
| C-10589 | H | single bond | Me | CH₂CF₃ | Me | Me | CH₂(5-Me-1,3-dioxol-2-one-4-yl) | O |
| C-10590 | Me | single bond | H | CH₂CF₃ | Me | Me | CH₂(5-Me-1,3-dioxol-2-one-4-yl) | O |
| C-10591 | Me(R) | single bond | H | CH₂CF₃ | Me | Me | CH₂(5-Me-1,3-dioxol-2-one-4-yl) | O |
| C-10592 | Me(S) | single bond | H | CH₂CF₃ | Me | Me | CH₂(5-Me-1,3-dioxol-2-one-4-yl) | O |
| C-10593 | H | single bond | Me | Et | Me | Me | CH₂(5-Me-1,3-dioxol-2-one-4-yl) | O |
| C-10594 | Me | single bond | H | Et | Me | Me | CH₂(5-Me-1,3-dioxol-2-one-4-yl) | O |

**[Table 469]**

| Compound Number | Y | W | Z | R¹ | R² | R³ | R¹⁰ | A |
|---|---|---|---|---|---|---|---|---|
| C-10595 | Me(R) | single bond | H | Et | Me | Me | CH₂(5-Me-1,3-dioxol-2-one-4-yl) | O |
| C-10596 | Me(S) | single bond | H | Et | Me | Me | CH₂(5-Me-1,3-dioxol-2-one-4-yl) | O |
| C-10597 | H | single bond | Me | c-Pr | Me | Me | CH₂(5-Me-1,3-dioxol-2-one-4-yl) | O |
| C-10598 | Me | single bond | H | c-Pr | Me | Me | CH₂(5-Me-1,3-dioxol-2-one-4-yl) | O |
| C-10599 | Me(R) | single bond | H | c-Pr | Me | Me | CH₂(5-Me-1,3-dioxol-2-one-4-yl) | O |
| C-10600 | Me(S) | single bond | H | c-Pr | Me | Me | CH₂(5-Me-1,3-dioxol-2-one-4-yl) | O |
| C-10601 | H | single bond | Me | c-Pr(1-CN) | Me | Me | CH₂(5-Me-1,3-dioxol-2-one-4-yl) | O |
| C-10602 | Me | single bond | H | c-Pr(1-CN) | Me | Me | CH₂(5-Me-1,3-dioxol-2-one-4-yl) | O |
| C-10603 | Me(R) | single bond | H | c-Pr(1-CN) | Me | Me | CH₂(5-Me-1,3-dioxol-2-one-4-yl) | O |
| C-10604 | Me(S) | single bond | H | c-Pr(1-CN) | Me | Me | CH₂(5-Me-1,3-dioxol-2-one-4-yl) | O |
| C-10605 | H | single bond | Me | CH₂CF₃ | C(=O)Me | Cl | *c*-Pr | O |
| C-10606 | Me | single bond | H | CH₂CF₃ | C(=O)Me | Cl | *c*-Pr | O |
| C-10607 | Me(R) | single bond | H | CH₂CF₃ | C(=O)Me | Cl | *c*-Pr | O |
| C-10608 | Me(S) | single bond | H | CH₂CF₃ | C(=O)Me | Cl | *c*-Pr | O |
| C-10609 | H | single bond | Me | Et | C(=O)Me | Cl | *c*-Pr | O |
| C-10610 | Me | single bond | H | Et | C(=O)Me | Cl | *c*-Pr | O |
| C-10611 | Me(R) | single bond | H | Et | C(=O)Me | Cl | *c*-Pr | O |
| C-10612 | Me(S) | single bond | H | Et | C(=O)Me | Cl | *c*-Pr | O |
| C-10613 | H | single bond | Me | c-Pr | C(=O)Me | Cl | *c*-Pr | O |
| C-10614 | Me | single bond | H | c-Pr | C(=O)Me | Cl | *c*-Pr | O |
| C-10615 | Me(R) | single bond | H | c-Pr | C(=O)Me | Cl | *c*-Pr | O |
| C-10616 | Me(S) | single bond | H | c-Pr | C(=O)Me | Cl | *c*-Pr | O |
| C-10617 | H | single bond | Me | c-Pr(1-CN) | C(=O)Me | Cl | *c*-Pr | O |
| C-10618 | Me | single bond | H | c-Pr(1-CN) | C(=O)Me | Cl | *c*-Pr | O |
| C-10619 | Me(R) | single bond | H | c-Pr(1-CN) | C(=O)Me | Cl | *c*-Pr | O |
| C-10620 | Me(S) | single bond | H | c-Pr(1-CN) | C(=O)Me | Cl | *c*-Pr | O |
| C-10621 | H | single bond | Me | CH₂CF₃ | C(=O)Me | Me | *c*-Pr | O |
| C-10622 | Me | single bond | H | CH₂CF₃ | C(=O)Me | Me | *c*-Pr | O |
| C-10623 | Me(R) | single bond | H | CH₂CF₃ | C(=O)Me | Me | *c*-Pr | O |
| C-10624 | Me(S) | single bond | H | CH₂CF₃ | C(=O)Me | Me | *c*-Pr | O |
| C-10625 | H | single bond | Me | Et | C(=O)Me | Me | *c*-Pr | O |
| C-10626 | Me | single bond | H | Et | C(=O)Me | Me | *c*-Pr | O |
| C-10627 | Me(R) | single bond | H | Et | C(=O)Me | Me | *c*-Pr | O |
| C-10628 | Me(S) | single bond | H | Et | C(=O)Me | Me | *c*-Pr | O |
| C-10629 | H | single bond | Me | c-Pr | C(=O)Me | Me | *c*-Pr | O |
| C-10630 | Me | single bond | H | c-Pr | C(=O)Me | Me | *c*-Pr | O |
| C-10631 | Me(R) | single bond | H | c-Pr | C(=O)Me | Me | *c*-Pr | O |
| C-10632 | Me(S) | single bond | H | c-Pr | C(=O)Me | Me | *c*-Pr | O |
| C-10633 | H | single bond | Me | c-Pr(1-CN) | C(=O)Me | Me | *c*-Pr | O |
| C-10634 | Me | single bond | H | c-Pr(1-CN) | C(=O)Me | Me | *c*-Pr | O |
| C-10635 | Me(R) | single bond | H | c-Pr(1-CN) | C(=O)Me | Me | *c*-Pr | O |
| C-10636 | Me(S) | single bond | H | c-Pr(1-CN) | C(=O)Me | Me | *c*-Pr | O |
| C-10637 | H | single bond | Me | CH₂CF₃ | C(=O)Me | Cl | OMe | O |
| C-10638 | Me | single bond | H | CH₂CF₃ | C(=O)Me | Cl | OMe | O |

**[Table 470]**

| Compound Number | Y | W | Z | R¹ | R² | R³ | R¹⁰ | A |
|---|---|---|---|---|---|---|---|---|
| C-10639 | Me(R) | single bond | H | CH₂CF₃ | C(=O)Me | Cl | OMe | O |
| C-10640 | Me(S) | single bond | H | CH₂CF₃ | C(=O)Me | Cl | OMe | O |
| C-10641 | H | single bond | Me | Et | C(=O)Me | Cl | OMe | O |
| C-10642 | Me | single bond | H | Et | C(=O)Me | Cl | OMe | O |
| C-10643 | Me(R) | single bond | H | Et | C(=O)Me | Cl | OMe | O |
| C-10644 | Me(S) | single bond | H | Et | C(=O)Me | Cl | OMe | O |
| C-10645 | H | single bond | Me | c-Pr | C(=O)Me | Cl | OMe | O |
| C-10646 | Me | single bond | H | c-Pr | C(=O)Me | Cl | OMe | O |
| C-10647 | Me(R) | single bond | H | c-Pr | C(=O)Me | Cl | OMe | O |
| C-10648 | Me(S) | single bond | H | c-Pr | C(=O)Me | Cl | OMe | O |
| C-10649 | H | single bond | Me | c-Pr(1-CN) | C(=O)Me | Cl | OMe | O |
| C-10650 | Me | single bond | H | c-Pr(1-CN) | C(=O)Me | Cl | OMe | O |
| C-10651 | Me(R) | single bond | H | c-Pr(1-CN) | C(=O)Me | Cl | OMe | O |
| C-10652 | Me(S) | single bond | H | c-Pr(1-CN) | C(=O)Me | Cl | OMe | O |
| C-10653 | H | single bond | Me | CH₂CF₃ | C(=O)Me | Me | OMe | O |
| C-10654 | Me | single bond | H | CH₂CF₃ | C(=O)Me | Me | OMe | O |
| C-10655 | Me(R) | single bond | H | CH₂CF₃ | C(=O)Me | Me | OMe | O |
| C-10656 | Me(S) | single bond | H | CH₂CF₃ | C(=O)Me | Me | OMe | O |
| C-10657 | H | single bond | Me | Et | C(=O)Me | Me | OMe | O |
| C-10658 | Me | single bond | H | Et | C(=O)Me | Me | OMe | O |
| C-10659 | Me(R) | single bond | H | Et | C(=O)Me | Me | OMe | O |
| C-10660 | Me(S) | single bond | H | Et | C(=O)Me | Me | OMe | O |
| C-10661 | H | single bond | Me | c-Pr | C(=O)Me | Me | OMe | O |
| C-10662 | Me | single bond | H | c-Pr | C(=O)Me | Me | OMe | O |
| C-10663 | Me(R) | single bond | H | c-Pr | C(=O)Me | Me | OMe | O |
| C-10664 | Me(S) | single bond | H | c-Pr | C(=O)Me | Me | OMe | O |
| C-10665 | H | single bond | Me | c-Pr(1-CN) | C(=O)Me | Me | OMe | O |
| C-10666 | Me | single bond | H | c-Pr(1-CN) | C(=O)Me | Me | OMe | O |
| C-10667 | Me(R) | single bond | H | c-Pr(1-CN) | C(=O)Me | Me | OMe | O |
| C-10668 | Me(S) | single bond | H | c-Pr(1-CN) | C(=O)Me | Me | OMe | O |
| C-10669 | H | single bond | Me | CH₂CF₃ | C(=O)Me | Cl | C(=O)(*i*-Pr) | O |
| C-10670 | Me | single bond | H | CH₂CF₃ | C(=O)Me | Cl | C(=O)(*i*-Pr) | O |
| C-10671 | Me(R) | single bond | H | CH₂CF₃ | C(=O)Me | Cl | C(=O)(*i*-Pr) | O |
| C-10672 | Me(S) | single bond | H | CH₂CF₃ | C(=O)Me | Cl | C(=O)(*i*-Pr) | O |
| C-10673 | H | single bond | Me | Et | C(=O)Me | Cl | C(=O)(*i*-Pr) | O |
| C-10674 | Me | single bond | H | Et | C(=O)Me | Cl | C(=O)(*i*-Pr) | O |
| C-10675 | Me(R) | single bond | H | Et | C(=O)Me | Cl | C(=O)(*i*-Pr) | O |
| C-10676 | Me(S) | single bond | H | Et | C(=O)Me | Cl | C(=O)(*i*-Pr) | O |
| C-10677 | H | single bond | Me | c-Pr | C(=O)Me | Cl | C(=O)(*i*-Pr) | O |
| C-10678 | Me | single bond | H | c-Pr | C(=O)Me | Cl | C(=O)(*i*-Pr) | O |
| C-10679 | Me(R) | single bond | H | c-Pr | C(=O)Me | Cl | C(=O)(*i*-Pr) | O |
| C-10680 | Me(S) | single bond | H | c-Pr | C(=O)Me | Cl | C(=O)(*i*-Pr) | O |
| C-10681 | H | single bond | Me | c-Pr(1-CN) | C(=O)Me | Cl | C(=O)(*i*-Pr) | O |
| C-10682 | Me | single bond | H | c-Pr(1-CN) | C(=O)Me | Cl | C(=O)(*i*-Pr) | O |

**[Table 471]**

| Compound Number | Y | W | Z | R¹ | R² | R³ | R¹⁰ | A |
|---|---|---|---|---|---|---|---|---|
| C-10683 | Me(R) | single bond | H | c-Pr(1-CN) | C(=O)Me | Cl | C(=O)(*i*-Pr) | O |
| C-10684 | Me(S) | single bond | H | c-Pr(1-CN) | C(=O)Me | Cl | C(=O)(*i*-Pr) | O |
| C-10685 | H | single bond | Me | CH₂CF₃ | C(=O)Me | Me | C(=O)(*i*-Pr) | O |
| C-10686 | Me | single bond | H | CH₂CF₃ | C(=O)Me | Me | C(=O)(*i*-Pr) | O |
| C-10687 | Me(R) | single bond | H | CH₂CF₃ | C(=O)Me | Me | C(=O)(*i*-Pr) | O |
| C-10688 | Me(S) | single bond | H | CH₂CF₃ | C(=O)Me | Me | C(=O)(*i*-Pr) | O |
| C-10689 | H | single bond | Me | Et | C(=O)Me | Me | C(=O)(*i*-Pr) | O |
| C-10690 | Me | single bond | H | Et | C(=O)Me | Me | C(=O)(*i*-Pr) | O |
| C-10691 | Me(R) | single bond | H | Et | C(=O)Me | Me | C(=O)(*i*-Pr) | O |
| C-10692 | Me(S) | single bond | H | Et | C(=O)Me | Me | C(=O)(*i*-Pr) | O |
| C-10693 | H | single bond | Me | c-Pr | C(=O)Me | Me | C(=O)(*i*-Pr) | O |
| C-10694 | Me | single bond | H | c-Pr | C(=O)Me | Me | C(=O)(*i*-Pr) | O |
| C-10695 | Me(R) | single bond | H | c-Pr | C(=O)Me | Me | C(=O)(*i*-Pr) | O |
| C-10696 | Me(S) | single bond | H | c-Pr | C(=O)Me | Me | C(=O)(*i*-Pr) | O |
| C-10697 | H | single bond | Me | c-Pr(1-CN) | C(=O)Me | Me | C(=O)(*i*-Pr) | O |
| C-10698 | Me | single bond | H | c-Pr(1-CN) | C(=O)Me | Me | C(=O)(*i*-Pr) | O |
| C-10699 | Me(R) | single bond | H | c-Pr(1-CN) | C(=O)Me | Me | C(=O)(*i*-Pr) | O |
| C-10700 | Me(S) | single bond | H | c-Pr(1-CN) | C(=O)Me | Me | C(=O)(*i*-Pr) | O |
| C-10701 | H | single bond | Me | CH₂CF₃ | C(=O)Me | Cl | C(=O)O(*t*-Bu) | O |
| C-10702 | Me | single bond | H | CH₂CF₃ | C(=O)Me | Cl | C(=O)O(*t*-Bu) | O |
| C-10703 | Me(R) | single bond | H | CH₂CF₃ | C(=O)Me | Cl | C(=O)O(*t*-Bu) | O |
| C-10704 | Me(S) | single bond | H | CH₂CF₃ | C(=O)Me | Cl | C(=O)O(*t*-Bu) | O |
| C-10705 | H | single bond | Me | Et | C(=O)Me | Cl | C(=O)O(*t*-Bu) | O |
| C-10706 | Me | single bond | H | Et | C(=O)Me | Cl | C(=O)O(*t*-Bu) | O |
| C-10707 | Me(R) | single bond | H | Et | C(=O)Me | Cl | C(=O)O(*t*-Bu) | O |
| C-10708 | Me(S) | single bond | H | Et | C(=O)Me | Cl | C(=O)O(*t*-Bu) | O |
| C-10709 | H | single bond | Me | c-Pr | C(=O)Me | Cl | C(=O)O(*t*-Bu) | O |
| C-10710 | Me | single bond | H | c-Pr | C(=O)Me | Cl | C(=O)O(*t*-Bu) | O |
| C-10711 | Me(R) | single bond | H | c-Pr | C(=O)Me | Cl | C(=O)O(*t*-Bu) | O |
| C-10712 | Me(S) | single bond | H | c-Pr | C(=O)Me | Cl | C(=O)O(*t*-Bu) | O |
| C-10713 | H | single bond | Me | c-Pr(1-CN) | C(=O)Me | Cl | C(=O)O(*t*-Bu) | O |
| C-10714 | Me | single bond | H | c-Pr(1-CN) | C(=O)Me | Cl | C(=O)O(*t*-Bu) | O |
| C-10715 | Me(R) | single bond | H | c-Pr(1-CN) | C(=O)Me | Cl | C(=O)O(*t*-Bu) | O |
| C-10716 | Me(S) | single bond | H | c-Pr(1-CN) | C(=O)Me | Cl | C(=O)O(*t*-Bu) | O |
| C-10717 | H | single bond | Me | CH₂CF₃ | C(=O)Me | Me | C(=O)O(*t*-Bu) | O |
| C-10718 | Me | single bond | H | CH₂CF₃ | C(=O)Me | Me | C(=O)O(*t*-Bu) | O |
| C-10719 | Me(R) | single bond | H | CH₂CF₃ | C(=O)Me | Me | C(=O)O(*t*-Bu) | O |
| C-10720 | Me(S) | single bond | H | CH₂CF₃ | C(=O)Me | Me | C(=O)O(*t*-Bu) | O |
| C-10721 | H | single bond | Me | Et | C(=O)Me | Me | C(=O)O(*t*-Bu) | O |
| C-10722 | Me | single bond | H | Et | C(=O)Me | Me | C(=O)O(*t*-Bu) | O |
| C-10723 | Me(R) | single bond | H | Et | C(=O)Me | Me | C(=O)O(*t*-Bu) | O |
| C-10724 | Me(S) | single bond | H | Et | C(=O)Me | Me | C(=O)O(*t*-Bu) | O |
| C-10725 | H | single bond | Me | c-Pr | C(=O)Me | Me | C(=O)O(*t*-Bu) | O |
| C-10726 | Me | single bond | H | c-Pr | C(=O)Me | Me | C(=O)O(*t*-Bu) | O |

**[Table 472]**

| Compound Number | Y | W | Z | R¹ | R² | R³ | R¹⁰ | A |
|---|---|---|---|---|---|---|---|---|
| C-10727 | Me(R) | single bond | H | c-Pr | C(=O)Me | Me | C(=O)O(*t*-Bu) | O |
| C-10728 | Me(S) | single bond | H | c-Pr | C(=O)Me | Me | C(=O)O(*t*-Bu) | O |
| C-10729 | H | single bond | Me | c-Pr(1-CN) | C(=O)Me | Me | C(=O)O(*t*-Bu) | O |
| C-10730 | Me | single bond | H | c-Pr(1-CN) | C(=O)Me | Me | C(=O)O(*t*-Bu) | O |
| C-10731 | Me(R) | single bond | H | c-Pr(1-CN) | C(=O)Me | Me | C(=O)O(*t*-Bu) | O |
| C-10732 | Me(S) | single bond | H | c-Pr(1-CN) | C(=O)Me | Me | C(=O)O(*t*-Bu) | O |
| C-10733 | H | single bond | Me | CH₂CF₃ | C(=O)Me | Cl | CH₂OEt | O |
| C-10734 | Me | single bond | H | CH₂CF₃ | C(=O)Me | Cl | CH₂OEt | O |
| C-10735 | Me(R) | single bond | H | CH₂CF₃ | C(=O)Me | Cl | CH₂OEt | O |
| C-10736 | Me(S) | single bond | H | CH₂CF₃ | C(=O)Me | Cl | CH₂OEt | O |
| C-10737 | H | single bond | Me | Et | C(=O)Me | Cl | CH₂OEt | O |
| C-10738 | Me | single bond | H | Et | C(=O)Me | Cl | CH₂OEt | O |
| C-10739 | Me(R) | single bond | H | Et | C(=O)Me | Cl | CH₂OEt | O |
| C-10740 | Me(S) | single bond | H | Et | C(=O)Me | Cl | CH₂OEt | O |
| C-10741 | H | single bond | Me | c-Pr | C(=O)Me | Cl | CH₂OEt | O |
| C-10742 | Me | single bond | H | c-Pr | C(=O)Me | Cl | CH₂OEt | O |
| C-10743 | Me(R) | single bond | H | c-Pr | C(=O)Me | Cl | CH₂OEt | O |
| C-10744 | Me(S) | single bond | H | c-Pr | C(=O)Me | Cl | CH₂OEt | O |
| C-10745 | H | single bond | Me | c-Pr(1-CN) | C(=O)Me | Cl | CH₂OEt | O |
| C-10746 | Me | single bond | H | c-Pr(1-CN) | C(=O)Me | Cl | CH₂OEt | O |
| C-10747 | Me(R) | single bond | H | c-Pr(1-CN) | C(=O)Me | Cl | CH₂OEt | O |
| C-10748 | Me(S) | single bond | H | c-Pr(1-CN) | C(=O)Me | Cl | CH₂OEt | O |
| C-10749 | H | single bond | Me | CH₂CF₃ | C(=O)Me | Me | CH₂OEt | O |
| C-10750 | Me | single bond | H | CH₂CF₃ | C(=O)Me | Me | CH₂OEt | O |
| C-10751 | Me(R) | single bond | H | CH₂CF₃ | C(=O)Me | Me | CH₂OEt | O |
| C-10752 | Me(S) | single bond | H | CH₂CF₃ | C(=O)Me | Me | CH₂OEt | O |
| C-10753 | H | single bond | Me | Et | C(=O)Me | Me | CH₂OEt | O |
| C-10754 | Me | single bond | H | Et | C(=O)Me | Me | CH₂OEt | O |
| C-10755 | Me(R) | single bond | H | Et | C(=O)Me | Me | CH₂OEt | O |
| C-10756 | Me(S) | single bond | H | Et | C(=O)Me | Me | CH₂OEt | O |
| C-10757 | H | single bond | Me | c-Pr | C(=O)Me | Me | CH₂OEt | O |
| C-10758 | Me | single bond | H | c-Pr | C(=O)Me | Me | CH₂OEt | O |
| C-10759 | Me(R) | single bond | H | c-Pr | C(=O)Me | Me | CH₂OEt | O |
| C-10760 | Me(S) | single bond | H | c-Pr | C(=O)Me | Me | CH₂OEt | O |
| C-10761 | H | single bond | Me | c-Pr(1-CN) | C(=O)Me | Me | CH₂OEt | O |
| C-10762 | Me | single bond | H | c-Pr(1-CN) | C(=O)Me | Me | CH₂OEt | O |
| C-10763 | Me(R) | single bond | H | c-Pr(1-CN) | C(=O)Me | Me | CH₂OEt | O |
| C-10764 | Me(S) | single bond | H | c-Pr(1-CN) | C(=O)Me | Me | CH₂OEt | O |
| C-10765 | H | single bond | Me | CH₂CF₃ | C(=O)Me | Cl | CH₂SMe | O |
| C-10766 | Me | single bond | H | CH₂CF₃ | C(=O)Me | Cl | CH₂SMe | O |
| C-10767 | Me(R) | single bond | H | CH₂CF₃ | C(=O)Me | Cl | CH₂SMe | O |
| C-10768 | Me(S) | single bond | H | CH₂CF₃ | C(=O)Me | Cl | CH₂SMe | O |
| C-10769 | H | single bond | Me | Et | C(=O)Me | Cl | CH₂SMe | O |
| C-10770 | Me | single bond | H | Et | C(=O)Me | Cl | CH₂SMe | O |

**[Table 473]**

| Compound Number | Y | W | Z | R¹ | R² | R³ | R¹⁰ | A |
|---|---|---|---|---|---|---|---|---|
| C-10771 | Me(R) | single bond | H | Et | C(=O)Me | Cl | CH₂SMe | O |
| C-10772 | Me(S) | single bond | H | Et | C(=O)Me | Cl | CH₂SMe | O |
| C-10773 | H | single bond | Me | c-Pr | C(=O)Me | Cl | CH₂SMe | O |
| C-10774 | Me | single bond | H | c-Pr | C(=O)Me | Cl | CH₂SMe | O |
| C-10775 | Me(R) | single bond | H | c-Pr | C(=O)Me | Cl | CH₂SMe | O |
| C-10776 | Me(S) | single bond | H | c-Pr | C(=O)Me | Cl | CH₂SMe | O |
| C-10777 | H | single bond | Me | c-Pr(1-CN) | C(=O)Me | Cl | CH₂SMe | O |
| C-10778 | Me | single bond | H | c-Pr(1-CN) | C(=O)Me | Cl | CH₂SMe | O |
| C-10779 | Me(R) | single bond | H | c-Pr(1-CN) | C(=O)Me | Cl | CH₂SMe | O |
| C-10780 | Me(S) | single bond | H | c-Pr(1-CN) | C(=O)Me | Cl | CH₂SMe | O |
| C-10781 | H | single bond | Me | CH₂CF₃ | C(=O)Me | Me | CH₂SMe | O |
| C-10782 | Me | single bond | H | CH₂CF₃ | C(=O)Me | Me | CH₂SMe | O |
| C-10783 | Me(R) | single bond | H | CH₂CF₃ | C(=O)Me | Me | CH₂SMe | O |
| C-10784 | Me(S) | single bond | H | CH₂CF₃ | C(=O)Me | Me | CH₂SMe | O |
| C-10785 | H | single bond | Me | Et | C(=O)Me | Me | CH₂SMe | O |
| C-10786 | Me | single bond | H | Et | C(=O)Me | Me | CH₂SMe | O |
| C-10787 | Me(R) | single bond | H | Et | C(=O)Me | Me | CH₂SMe | O |
| C-10788 | Me(S) | single bond | H | Et | C(=O)Me | Me | CH₂SMe | O |
| C-10789 | H | single bond | Me | c-Pr | C(=O)Me | Me | CH₂SMe | O |
| C-10790 | Me | single bond | H | c-Pr | C(=O)Me | Me | CH₂SMe | O |
| C-10791 | Me(R) | single bond | H | c-Pr | C(=O)Me | Me | CH₂SMe | O |
| C-10792 | Me(S) | single bond | H | c-Pr | C(=O)Me | Me | CH₂SMe | O |
| C-10793 | H | single bond | Me | c-Pr(1-CN) | C(=O)Me | Me | CH₂SMe | O |
| C-10794 | Me | single bond | H | c-Pr(1-CN) | C(=O)Me | Me | CH₂SMe | O |
| C-10795 | Me(R) | single bond | H | c-Pr(1-CN) | C(=O)Me | Me | CH₂SMe | O |
| C-10796 | Me(S) | single bond | H | c-Pr(1-CN) | C(=O)Me | Me | CH₂SMe | O |
| C-10797 | H | single bond | Me | CH₂CF₃ | C(=O)Me | Cl | CH₂S(=O)₂Me | O |
| C-10798 | Me | single bond | H | CH₂CF₃ | C(=O)Me | Cl | CH₂S(=O)₂Me | O |
| C-10799 | Me(R) | single bond | H | CH₂CF₃ | C(=O)Me | Cl | CH₂S(=O)₂Me | O |
| C-10800 | Me(S) | single bond | H | CH₂CF₃ | C(=O)Me | Cl | CH₂S(=O)₂Me | O |
| C-10801 | H | single bond | Me | Et | C(=O)Me | Cl | CH₂S(=O)₂Me | O |
| C-10802 | Me | single bond | H | Et | C(=O)Me | Cl | CH₂S(=O)₂Me | O |
| C-10803 | Me(R) | single bond | H | Et | C(=O)Me | Cl | CH₂S(=O)₂Me | O |
| C-10804 | Me(S) | single bond | H | Et | C(=O)Me | Cl | CH₂S(=O)₂Me | O |
| C-10805 | H | single bond | Me | c-Pr | C(=O)Me | Cl | CH₂S(=O)₂Me | O |
| C-10806 | Me | single bond | H | c-Pr | C(=O)Me | Cl | CH₂S(=O)₂Me | O |
| C-10807 | Me(R) | single bond | H | c-Pr | C(=O)Me | Cl | CH₂S(=O)₂Me | O |
| C-10808 | Me(S) | single bond | H | c-Pr | C(=O)Me | Cl | CH₂S(=O)₂Me | O |
| C-10809 | H | single bond | Me | c-Pr(1-CN) | C(=O)Me | Cl | CH₂S(=O)₂Me | O |
| C-10810 | Me | single bond | H | c-Pr(1-CN) | C(=O)Me | Cl | CH₂S(=O)₂Me | O |
| C-10811 | Me(R) | single bond | H | c-Pr(1-CN) | C(=O)Me | Cl | CH₂S(=O)₂Me | O |
| C-10812 | Me(S) | single bond | H | c-Pr(1-CN) | C(=O)Me | Cl | CH₂S(=O)₂Me | O |
| C-10813 | H | single bond | Me | CH₂CF₃ | C(=O)Me | Me | CH₂S(=O)₂Me | O |
| C-10814 | Me | single bond | H | CH₂CF₃ | C(=O)Me | Me | CH₂S(=O)₂Me | O |

**[Table 474]**

| Compound Number | Y | W | Z | R¹ | R² | R³ | R¹⁰ | A |
|---|---|---|---|---|---|---|---|---|
| C-10815 | Me(R) | single bond | H | CH₂CF₃ | C(=O)Me | Me | CH₂S(=O)₂Me | O |
| C-10816 | Me(S) | single bond | H | CH₂CF₃ | C(=O)Me | Me | CH₂S(=O)₂Me | O |
| C-10817 | H | single bond | Me | Et | C(=O)Me | Me | CH₂S(=O)₂Me | O |
| C-10818 | Me | single bond | H | Et | C(=O)Me | Me | CH₂S(=O)₂Me | O |
| C-10819 | Me(R) | single bond | H | Et | C(=O)Me | Me | CH₂S(=O)₂Me | O |
| C-10820 | Me(S) | single bond | H | Et | C(=O)Me | Me | CH₂S(=O)₂Me | O |
| C-10821 | H | single bond | Me | c-Pr | C(=O)Me | Me | CH₂S(=O)₂Me | O |
| C-10822 | Me | single bond | H | c-Pr | C(=O)Me | Me | CH₂S(=O)₂Me | O |
| C-10823 | Me(R) | single bond | H | c-Pr | C(=O)Me | Me | CH₂S(=O)₂Me | O |
| C-10824 | Me(S) | single bond | H | c-Pr | C(=O)Me | Me | CH₂S(=O)₂Me | O |
| C-10825 | H | single bond | Me | c-Pr(1-CN) | C(=O)Me | Me | CH₂S(=O)₂Me | O |
| C-10826 | Me | single bond | H | c-Pr(1-CN) | C(=O)Me | Me | CH₂S(=O)₂Me | O |
| C-10827 | Me(R) | single bond | H | c-Pr(1-CN) | C(=O)Me | Me | CH₂S(=O)₂Me | O |
| C-10828 | Me(S) | single bond | H | c-Pr(1-CN) | C(=O)Me | Me | CH₂S(=O)₂Me | O |
| C-10829 | H | single bond | Me | CH₂CF₃ | C(=O)Me | Cl | CH₂C(=O)Me | O |
| C-10830 | Me | single bond | H | CH₂CF₃ | C(=O)Me | Cl | CH₂C(=O)Me | O |
| C-10831 | Me(R) | single bond | H | CH₂CF₃ | C(=O)Me | Cl | CH₂C(=O)Me | O |
| C-10832 | Me(S) | single bond | H | CH₂CF₃ | C(=O)Me | Cl | CH₂C(=O)Me | O |
| C-10833 | H | single bond | Me | Et | C(=O)Me | Cl | CH₂C(=O)Me | O |
| C-10834 | Me | single bond | H | Et | C(=O)Me | Cl | CH₂C(=O)Me | O |
| C-10835 | Me(R) | single bond | H | Et | C(=O)Me | Cl | CH₂C(=O)Me | O |
| C-10836 | Me(S) | single bond | H | Et | C(=O)Me | Cl | CH₂C(=O)Me | O |
| C-10837 | H | single bond | Me | c-Pr | C(=O)Me | Cl | CH₂C(=O)Me | O |
| C-10838 | Me | single bond | H | c-Pr | C(=O)Me | Cl | CH₂C(=O)Me | O |
| C-10839 | Me(R) | single bond | H | c-Pr | C(=O)Me | Cl | CH₂C(=O)Me | O |
| C-10840 | Me(S) | single bond | H | c-Pr | C(=O)Me | Cl | CH₂C(=O)Me | O |
| C-10841 | H | single bond | Me | c-Pr(1-CN) | C(=O)Me | Cl | CH₂C(=O)Me | O |
| C-10842 | Me | single bond | H | c-Pr(1-CN) | C(=O)Me | Cl | CH₂C(=O)Me | O |
| C-10843 | Me(R) | single bond | H | c-Pr(1-CN) | C(=O)Me | Cl | CH₂C(=O)Me | O |
| C-10844 | Me(S) | single bond | H | c-Pr(1-CN) | C(=O)Me | Cl | CH₂C(=O)Me | O |
| C-10845 | H | single bond | Me | CH₂CF₃ | C(=O)Me | Me | CH₂C(=O)Me | O |
| C-10846 | Me | single bond | H | CH₂CF₃ | C(=O)Me | Me | CH₂C(=O)Me | O |
| C-10847 | Me(R) | single bond | H | CH₂CF₃ | C(=O)Me | Me | CH₂C(=O)Me | O |
| C-10848 | Me(S) | single bond | H | CH₂CF₃ | C(=O)Me | Me | CH₂C(=O)Me | O |
| C-10849 | H | single bond | Me | Et | C(=O)Me | Me | CH₂C(=O)Me | O |
| C-10850 | Me | single bond | H | Et | C(=O)Me | Me | CH₂C(=O)Me | O |
| C-10851 | Me(R) | single bond | H | Et | C(=O)Me | Me | CH₂C(=O)Me | O |
| C-10852 | Me(S) | single bond | H | Et | C(=O)Me | Me | CH₂C(=O)Me | O |
| C-10853 | H | single bond | Me | c-Pr | C(=O)Me | Me | CH₂C(=O)Me | O |
| C-10854 | Me | single bond | H | c-Pr | C(=O)Me | Me | CH₂C(=O)Me | O |
| C-10855 | Me(R) | single bond | H | c-Pr | C(=O)Me | Me | CH₂C(=O)Me | O |
| C-10856 | Me(S) | single bond | H | c-Pr | C(=O)Me | Me | CH₂C(=O)Me | O |
| C-10857 | H | single bond | Me | c-Pr(1-CN) | C(=O)Me | Me | CH₂C(=O)Me | O |
| C-10858 | Me | single bond | H | c-Pr(1-CN) | C(=O)Me | Me | CH₂C(=O)Me | O |

**[Table 475]**

| Compound Number | Y | W | Z | R¹ | R² | R³ | R¹⁰ | A |
|---|---|---|---|---|---|---|---|---|
| C-10859 | Me(R) | single bond | H | c-Pr(1-CN) | C(=O)Me | Me | CH₂C(=O)Me | O |
| C-10860 | Me(S) | single bond | H | c-Pr(1-CN) | C(=O)Me | Me | CH₂C(=O)Me | O |
| C-10861 | H | single bond | Me | CH₂CF₃ | C(=O)Me | Cl | CH₂C(=O)OMe | O |
| C-10862 | Me | single bond | H | CH₂CF₃ | C(=O)Me | Cl | CH₂C(=O)OMe | O |
| C-10863 | Me(R) | single bond | H | CH₂CF₃ | C(=O)Me | Cl | CH₂C(=O)OMe | O |
| C-10864 | Me(S) | single bond | H | CH₂CF₃ | C(=O)Me | Cl | CH₂C(=O)OMe | O |
| C-10865 | H | single bond | Me | Et | C(=O)Me | Cl | CH₂C(=O)OMe | O |
| C-10866 | Me | single bond | H | Et | C(=O)Me | Cl | CH₂C(=O)OMe | O |
| C-10867 | Me(R) | single bond | H | Et | C(=O)Me | Cl | CH₂C(=O)OMe | O |
| C-10868 | Me(S) | single bond | H | Et | C(=O)Me | Cl | CH₂C(=O)OMe | O |
| C-10869 | H | single bond | Me | c-Pr | C(=O)Me | Cl | CH₂C(=O)OMe | O |
| C-10870 | Me | single bond | H | c-Pr | C(=O)Me | Cl | CH₂C(=O)OMe | O |
| C-10871 | Me(R) | single bond | H | c-Pr | C(=O)Me | Cl | CH₂C(=O)OMe | O |
| C-10872 | Me(S) | single bond | H | c-Pr | C(=O)Me | Cl | CH₂C(=O)OMe | O |
| C-10873 | H | single bond | Me | c-Pr(1-CN) | C(=O)Me | Cl | CH₂C(=O)OMe | O |
| C-10874 | Me | single bond | H | c-Pr(1-CN) | C(=O)Me | Cl | CH₂C(=O)OMe | O |
| C-10875 | Me(R) | single bond | H | c-Pr(1-CN) | C(=O)Me | Cl | CH₂C(=O)OMe | O |
| C-10876 | Me(S) | single bond | H | c-Pr(1-CN) | C(=O)Me | Cl | CH₂C(=O)OMe | O |
| C-10877 | H | single bond | Me | CH₂CF₃ | C(=O)Me | Me | CH₂C(=O)OMe | O |
| C-10878 | Me | single bond | H | CH₂CF₃ | C(=O)Me | Me | CH₂C(=O)OMe | O |
| C-10879 | Me(R) | single bond | H | CH₂CF₃ | C(=O)Me | Me | CH₂C(=O)OMe | O |
| C-10880 | Me(S) | single bond | H | CH₂CF₃ | C(=O)Me | Me | CH₂C(=O)OMe | O |
| C-10881 | H | single bond | Me | Et | C(=O)Me | Me | CH₂C(=O)OMe | O |
| C-10882 | Me | single bond | H | Et | C(=O)Me | Me | CH₂C(=O)OMe | O |
| C-10883 | Me(R) | single bond | H | Et | C(=O)Me | Me | CH₂C(=O)OMe | O |
| C-10884 | Me(S) | single bond | H | Et | C(=O)Me | Me | CH₂C(=O)OMe | O |
| C-10885 | H | single bond | Me | c-Pr | C(=O)Me | Me | CH₂C(=O)OMe | O |
| C-10886 | Me | single bond | H | c-Pr | C(=O)Me | Me | CH₂C(=O)OMe | O |
| C-10887 | Me(R) | single bond | H | c-Pr | C(=O)Me | Me | CH₂C(=O)OMe | O |
| C-10888 | Me(S) | single bond | H | c-Pr | C(=O)Me | Me | CH₂C(=O)OMe | O |
| C-10889 | H | single bond | Me | c-Pr(1-CN) | C(=O)Me | Me | CH₂C(=O)OMe | O |
| C-10890 | Me | single bond | H | c-Pr(1-CN) | C(=O)Me | Me | CH₂C(=O)OMe | O |
| C-10891 | Me(R) | single bond | H | c-Pr(1-CN) | C(=O)Me | Me | CH₂C(=O)OMe | O |
| C-10892 | Me(S) | single bond | H | c-Pr(1-CN) | C(=O)Me | Me | CH₂C(=O)OMe | O |
| C-10893 | H | single bond | Me | CH₂CF₃ | C(=O)Me | Cl | CH₂C(=O)NHMe | O |
| C-10894 | Me | single bond | H | CH₂CF₃ | C(=O)Me | Cl | CH₂C(=O)NHMe | O |
| C-10895 | Me(R) | single bond | H | CH₂CF₃ | C(=O)Me | Cl | CH₂C(=O)NHMe | O |
| C-10896 | Me(S) | single bond | H | CH₂CF₃ | C(=O)Me | Cl | CH₂C(=O)NHMe | O |
| C-10897 | H | single bond | Me | Et | C(=O)Me | Cl | CH₂C(=O)NHMe | O |
| C-10898 | Me | single bond | H | Et | C(=O)Me | Cl | CH₂C(=O)NHMe | O |
| C-10899 | Me(R) | single bond | H | Et | C(=O)Me | Cl | CH₂C(=O)NHMe | O |
| C-10900 | Me(S) | single bond | H | Et | C(=O)Me | Cl | CH₂C(=O)NHMe | O |
| C-10901 | H | single bond | Me | c-Pr | C(=O)Me | Cl | CH₂C(=O)NHMe | O |
| C-10902 | Me | single bond | H | c-Pr | C(=O)Me | Cl | CH₂C(=O)NHMe | O |

**[Table 476]**

| Compound Number | Y | W | Z | R¹ | R² | R³ | R¹⁰ | A |
|---|---|---|---|---|---|---|---|---|
| C-10903 | Me(R) | single bond | H | c-Pr | C(=O)Me | Cl | CH₂C(=O)NHMe | O |
| C-10904 | Me(S) | single bond | H | c-Pr | C(=O)Me | Cl | CH₂C(=O)NHMe | O |
| C-10905 | H | single bond | Me | c-Pr(1-CN) | C(=O)Me | Cl | CH₂C(=O)NHMe | O |
| C-10906 | Me | single bond | H | c-Pr(1-CN) | C(=O)Me | Cl | CH₂C(=O)NHMe | O |
| C-10907 | Me(R) | single bond | H | c-Pr(1-CN) | C(=O)Me | Cl | CH₂C(=O)NHMe | O |
| C-10908 | Me(S) | single bond | H | c-Pr(1-CN) | C(=O)Me | Cl | CH₂C(=O)NHMe | O |
| C-10909 | H | single bond | Me | CH₂CF₃ | C(=O)Me | Me | CH₂C(=O)NHMe | O |
| C-10910 | Me | single bond | H | CH₂CF₃ | C(=O)Me | Me | CH₂C(=O)NHMe | O |
| C-10911 | Me(R) | single bond | H | CH₂CF₃ | C(=O)Me | Me | CH₂C(=O)NHMe | O |
| C-10912 | Me(S) | single bond | H | CH₂CF₃ | C(=O)Me | Me | CH₂C(=O)NHMe | O |
| C-10913 | H | single bond | Me | Et | C(=O)Me | Me | CH₂C(=O)NHMe | O |
| C-10914 | Me | single bond | H | Et | C(=O)Me | Me | CH₂C(=O)NHMe | O |
| C-10915 | Me(R) | single bond | H | Et | C(=O)Me | Me | CH₂C(=O)NHMe | O |
| C-10916 | Me(S) | single bond | H | Et | C(=O)Me | Me | CH₂C(=O)NHMe | O |
| C-10017 | H | single bond | Me | c-Pr | C(=O)Me | Me | CH₂C(=O)NHMe | O |
| C-10918 | Me | single bond | H | c-Pr | C(=O)Me | Me | CH₂C(=O)NHMe | O |
| C-10919 | Me(R) | single bond | H | c-Pr | C(=O)Me | Me | CH₂C(=O)NHMe | O |
| C-10920 | Me(S) | single bond | H | c-Pr | C(=O)Me | Me | CH₂C(=O)NHMe | O |
| C-10921 | H | single bond | Me | c-Pr(1-CN) | C(=O)Me | Me | CH₂C(=O)NHMe | O |
| C-10922 | Me | single bond | H | c-Pr(1-CN) | C(=O)Me | Me | CH₂C(=O)NHMe | O |
| C-10923 | Me(R) | single bond | H | c-Pr(1-CN) | C(=O)Me | Me | CH₂C(=O)NHMe | O |
| C-10924 | Me(S) | single bond | H | c-Pr(1-CN) | C(=O)Me | Me | CH₂C(=O)NHMe | O |
| C-10925 | H | single bond | Me | CH₂CF₃ | C(=O)Me | Cl | CH₂OC(=O)Me | O |
| C-10926 | Me | single bond | H | CH₂CF₃ | C(=O)Me | Cl | CH₂OC(=O)Me | O |
| C-10927 | Me(R) | single bond | H | CH₂CF₃ | C(=O)Me | Cl | CH₂OC(=O)Me | O |
| C-10928 | Me(S) | single bond | H | CH₂CF₃ | C(=O)Me | Cl | CH₂OC(=O)Me | O |
| C-10929 | H | single bond | Me | Et | C(=O)Me | Cl | CH₂OC(=O)Me | O |
| C-10930 | Me | single bond | H | Et | C(=O)Me | Cl | CH₂OC(=O)Me | O |
| C-10931 | Me(R) | single bond | H | Et | C(=O)Me | Cl | CH₂OC(=O)Me | O |
| C-10932 | Me(S) | single bond | H | Et | C(=O)Me | Cl | CH₂OC(=O)Me | O |
| C-10933 | H | single bond | Me | c-Pr | C(=O)Me | Cl | CH₂OC(=O)Me | O |
| C-10934 | Me | single bond | H | c-Pr | C(=O)Me | Cl | CH₂OC(=O)Me | O |
| C-10935 | Me(R) | single bond | H | c-Pr | C(=O)Me | Cl | CH₂OC(=O)Me | O |
| C-10936 | Me(S) | single bond | H | c-Pr | C(=O)Me | Cl | CH₂OC(=O)Me | O |
| C-10937 | H | single bond | Me | c-Pr(1-CN) | C(=O)Me | Cl | CH₂OC(=O)Me | O |
| C-10938 | Me | single bond | H | c-Pr(1-CN) | C(=O)Me | Cl | CH₂OC(=O)Me | O |
| C-10939 | Me(R) | single bond | H | c-Pr(1-CN) | C(=O)Me | Cl | CH₂OC(=O)Me | O |
| C-10940 | Me(S) | single bond | H | c-Pr(1-CN) | C(=O)Me | Cl | CH₂OC(=O)Me | O |
| C-10941 | H | single bond | Me | CH₂CF₃ | C(=O)Me | Me | CH₂OC(=O)Me | O |
| C-10942 | Me | single bond | H | CH₂CF₃ | C(=O)Me | Me | CH₂OC(=O)Me | O |
| C-10943 | Me(R) | single bond | H | CH₂CF₃ | C(=O)Me | Me | CH₂OC(=O)Me | O |
| C-10944 | Me(S) | single bond | H | CH₂CF₃ | C(=O)Me | Me | CH₂OC(=O)Me | O |
| C-10945 | H | single bond | Me | Et | C(=O)Me | Me | CH₂OC(=O)Me | O |
| C-10946 | Me | single bond | H | Et | C(=O)Me | Me | CH₂OC(=O)Me | O |

**[Table 477]**

| Compound Number | Y | W | Z | R¹ | R² | R³ | R¹⁰ | A |
|---|---|---|---|---|---|---|---|---|
| C-10047 | Me(R) | single bond | H | Et | C(=O)Me | Me | CH₂OC(=O)Me | O |
| C-10948 | Me(S) | single bond | H | Et | C(=O)Me | Me | CH₂OC(=O)Me | O |
| C-10949 | H | single bond | Me | c-Pr | C(=O)Me | Me | CH₂OC(=O)Me | O |
| C-10950 | Me | single bond | H | c-Pr | C(=O)Me | Me | CH₂OC(=O)Me | O |
| C-10951 | Me(R) | single bond | H | c-Pr | C(=O)Me | Me | CH₂OC(=O)Me | O |
| C-10952 | Me(S) | single bond | H | c-Pr | C(=O)Me | Me | CH₂OC(=O)Me | O |
| C-10953 | H | single bond | Me | c-Pr(1-CN) | C(=O)Me | Me | CH₂OC(=O)Me | O |
| C-10954 | Me | single bond | H | c-Pr(1-CN) | C(=O)Me | Me | CH₂OC(=O)Me | O |
| C-10955 | Me(R) | single bond | H | c-Pr(1-CN) | C(=O)Me | Me | CH₂OC(=O)Me | O |
| C-10956 | Me(S) | single bond | H | c-Pr(1-CN) | C(=O)Me | Me | CH₂OC(=O)Me | O |
| C-10957 | H | single bond | Me | CH₂CF₃ | C(=O)Me | Cl | CH₂OC(=O)(*i*-Pr) | O |
| C-10958 | Me | single bond | H | CH₂CF₃ | C(=O)Me | Cl | CH₂OC(=O)(*i*-Pr) | O |
| C-10959 | Me(R) | single bond | H | CH₂CF₃ | C(=O)Me | Cl | CH₂OC(=O)(*i*-Pr) | O |
| C-10960 | Me(S) | single bond | H | CH₂CF₃ | C(=O)Me | Cl | CH₂OC(=O)(*i*-Pr) | O |
| C-10961 | H | single bond | Me | Et | C(=O)Me | Cl | CH₂OC(=O)(*i*-Pr) | O |
| C-10962 | Me | single bond | H | Et | C(=O)Me | Cl | CH₂OC(=O)(*i*-Pr) | O |
| C-10963 | Me(R) | single bond | H | Et | C(=O)Me | Cl | CH₂OC(=O)(*i*-Pr) | O |
| C-10964 | Me(S) | single bond | H | Et | C(=O)Me | Cl | CH₂OC(=O)(*i*-Pr) | O |
| C-10965 | H | single bond | Me | c-Pr | C(=O)Me | Cl | CH₂OC(=O)(*i*-Pr) | O |
| C-10966 | Me | single bond | H | c-Pr | C(=O)Me | Cl | CH₂OC(=O)(*i*-Pr) | O |
| C-10967 | Me(R) | single bond | H | c-Pr | C(=O)Me | Cl | CH₂OC(=O)(*i*-Pr) | O |
| C-10968 | Me(S) | single bond | H | c-Pr | C(=O)Me | Cl | CH₂OC(=O)(*i*-Pr) | O |
| C-10969 | H | single bond | Me | c-Pr(1-CN) | C(=O)Me | Cl | CH₂OC(=O)(*i*-Pr) | O |
| C-10970 | Me | single bond | H | c-Pr(1-CN) | C(=O)Me | Cl | CH₂OC(=O)(*i*-Pr) | O |
| C-10971 | Me(R) | single bond | H | c-Pr(1-CN) | C(=O)Me | Cl | CH₂OC(=O)(*i*-Pr) | O |
| C-10972 | Me(S) | single bond | H | c-Pr(1-CN) | C(=O)Me | Cl | CH₂OC(=O)(*i*-Pr) | O |
| C-10973 | H | single bond | Me | CH₂CF₃ | C(=O)Me | Me | CH₂OC(=O)(*i*-Pr) | O |
| C-10974 | Me | single bond | H | CH₂CF₃ | C(=O)Me | Me | CH₂OC(=O)(*i*-Pr) | O |
| C-10975 | Me(R) | single bond | H | CH₂CF₃ | C(=O)Me | Me | CH₂OC(=O)(*i*-Pr) | O |
| C-10976 | Me(S) | single bond | H | CH₂CF₃ | C(=O)Me | Me | CH₂OC(=O)(*i*-Pr) | O |
| C-10977 | H | single bond | Me | Et | C(=O)Me | Me | CH₂OC(=O)(*i*-Pr) | O |
| C-10978 | Me | single bond | H | Et | C(=O)Me | Me | CH₂OC(=O)(*i*-Pr) | O |
| C-10979 | Me(R) | single bond | H | Et | C(=O)Me | Me | CH₂OC(=O)(*i*-Pr) | O |
| C-10980 | Me(S) | single bond | H | Et | C(=O)Me | Me | CH₂OC(=O)(*i*-Pr) | O |
| C-10981 | H | single bond | Me | c-Pr | C(=O)Me | Me | CH₂OC(=O)(*i*-Pr) | O |
| C-10982 | Me | single bond | H | c-Pr | C(=O)Me | Me | CH₂OC(=O)(*i*-Pr) | O |
| C-10983 | Me(R) | single bond | H | c-Pr | C(=O)Me | Me | CH₂OC(=O)(*i*-Pr) | O |
| C-10984 | Me(S) | single bond | H | c-Pr | C(=O)Me | Me | CH₂OC(=O)(*i*-Pr) | O |
| C-10985 | H | single bond | Me | c-Pr(1-CN) | C(=O)Me | Me | CH₂OC(=O)(*i*-Pr) | O |
| C-10986 | Me | single bond | H | c-Pr(1-CN) | C(=O)Me | Me | CH₂OC(=O)(*i*-Pr) | O |
| C-10987 | Me(R) | single bond | H | c-Pr(1-CN) | C(=O)Me | Me | CH₂OC(=O)(*i*-Pr) | O |
| C-10988 | Me(S) | single bond | H | c-Pr(1-CN) | C(=O)Me | Me | CH₂OC(=O)(*i*-Pr) | O |
| C-10989 | H | single bond | Me | CH₂CF₃ | C(=O)Me | Cl | CH₂OC(=O)(*t*-Bu) | O |
| C-10990 | Me | single bond | H | CH₂CF₃ | C(=O)Me | Cl | CH₂OC(=O)(*t*-Bu) | O |

**[Table 478]**

| Compound Number | Y | W | Z | R¹ | R² | R³ | R¹⁰ | A |
|---|---|---|---|---|---|---|---|---|
| C-10991 | Me(R) | single bond | H | CH₂CF₃ | C(=O)Me | Cl | CH₂OC(=O)(*t*-Bu) | O |
| C-10992 | Me(S) | single bond | H | CH₂CF₃ | C(=O)Me | Cl | CH₂OC(=O)(*t*-Bu) | O |
| C-10993 | H | single bond | Me | Et | C(=O)Me | Cl | CH₂OC(=O)(*t*-Bu) | O |
| C-10994 | Me | single bond | H | Et | C(=O)Me | Cl | CH₂OC(=O)(*t*-Bu) | O |
| C-10995 | Me(R) | single bond | H | Et | C(=O)Me | Cl | CH₂OC(=O)(*t*-Bu) | O |
| C-10996 | Me(S) | single bond | H | Et | C(=O)Me | Cl | CH₂OC(=O)(*t*-Bu) | O |
| C-10997 | H | single bond | Me | c-Pr | C(=O)Me | Cl | CH₂OC(=O)(*t*-Bu) | O |
| C-10998 | Me | single bond | H | c-Pr | C(=O)Me | Cl | CH₂OC(=O)(*t*-Bu) | O |
| C-10999 | Me(R) | single bond | H | c-Pr | C(=O)Me | Cl | CH₂OC(=O)(*t*-Bu) | O |
| C-11000 | Me(S) | single bond | H | c-Pr | C(=O)Me | Cl | CH₂OC(=O)(*t*-Bu) | O |
| C-11001 | H | single bond | Me | c-Pr(1-CN) | C(=O)Me | Cl | CH₂OC(=O)(*t*-Bu) | O |
| C-11002 | Me | single bond | H | c-Pr(1-CN) | C(=O)Me | Cl | CH₂OC(=O)(*t*-Bu) | O |
| C-11003 | Me(R) | single bond | H | c-Pr(1-CN) | C(=O)Me | Cl | CH₂OC(=O)(*t*-Bu) | O |
| C-11004 | Me(S) | single bond | H | c-Pr(1-CN) | C(=O)Me | Cl | CH₂OC(=O)(*t*-Bu) | O |
| C-11005 | H | single bond | Me | CH₂CF₃ | C(=O)Me | Me | CH₂OC(=O)(*t*-Bu) | O |
| C-11006 | Me | single bond | H | CH₂CF₃ | C(=O)Me | Me | CH₂OC(=O)(*t*-Bu) | O |
| C-11007 | Me(R) | single bond | H | CH₂CF₃ | C(=O)Me | Me | CH₂OC(=O)(*t*-Bu) | O |
| C-11008 | Me(S) | single bond | H | CH₂CF₃ | C(=O)Me | Me | CH₂OC(=O)(*t*-Bu) | O |
| C-11009 | H | single bond | Me | Et | C(=O)Me | Me | CH₂OC(=O)(*t*-Bu) | O |
| C-11010 | Me | single bond | H | Et | C(=O)Me | Me | CH₂OC(=O)(*t*-Bu) | O |
| C-11011 | Me(R) | single bond | H | Et | C(=O)Me | Me | CH₂OC(=O)(*t*-Bu) | O |
| C-11012 | Me(S) | single bond | H | Et | C(=O)Me | Me | CH₂OC(=O)(*t*-Bu) | O |
| C-11013 | H | single bond | Me | c-Pr | C(=O)Me | Me | CH₂OC(=O)(*t*-Bu) | O |
| C-11014 | Me | single bond | H | c-Pr | C(=O)Me | Me | CH₂OC(=O)(*t*-Bu) | O |
| C-11015 | Me(R) | single bond | H | c-Pr | C(=O)Me | Me | CH₂OC(=O)(*t*-Bu) | O |
| C-11016 | Me(S) | single bond | H | c-Pr | C(=O)Me | Me | CH₂OC(=O)(*t*-Bu) | O |
| C-11017 | H | single bond | Me | c-Pr(1-CN) | C(=O)Me | Me | CH₂OC(=O)(*t*-Bu) | O |
| C-11018 | Me | single bond | H | c-Pr(1-CN) | C(=O)Me | Me | CH₂OC(=O)(*t*-Bu) | O |
| C-11019 | Me(R) | single bond | H | c-Pr(1-CN) | C(=O)Me | Me | CH₂OC(=O)(*t*-Bu) | O |
| C-11020 | Me(S) | single bond | H | c-Pr(1-CN) | C(=O)Me | Me | CH₂OC(=O)(*t*-Bu) | O |
| C-11021 | H | single bond | Me | CH₂CF₃ | C(=O)Me | Cl | CH₂OC(=O)OEt | O |
| C-11022 | Me | single bond | H | CH₂CF₃ | C(=O)Me | Cl | CH₂OC(=O)OEt | O |
| C-11023 | Me(R) | single bond | H | CH₂CF₃ | C(=O)Me | Cl | CH₂OC(=O)OEt | O |
| C-11024 | Me(S) | single bond | H | CH₂CF₃ | C(=O)Me | Cl | CH₂OC(=O)OEt | O |
| C-11025 | H | single bond | Me | Et | C(=O)Me | Cl | CH₂OC(=O)OEt | O |
| C-11026 | Me | single bond | H | Et | C(=O)Me | Cl | CH₂OC(=O)OEt | O |
| C-11027 | Me(R) | single bond | H | Et | C(=O)Me | Cl | CH₂OC(=O)OEt | O |
| C-11028 | Me(S) | single bond | H | Et | C(=O)Me | Cl | CH₂OC(=O)OEt | O |
| C-11029 | H | single bond | Me | c-Pr | C(=O)Me | Cl | CH₂OC(=O)OEt | O |
| C-11030 | Me | single bond | H | c-Pr | C(=O)Me | Cl | CH₂OC(=O)OEt | O |
| C-11031 | Me(R) | single bond | H | c-Pr | C(=O)Me | Cl | CH₂OC(=O)OEt | O |
| C-11032 | Me(S) | single bond | H | c-Pr | C(=O)Me | Cl | CH₂OC(=O)OEt | O |
| C-11033 | H | single bond | Me | c-Pr(1-CN) | C(=O)Me | Cl | CH₂OC(=O)OEt | O |
| C-11034 | Me | single bond | H | c-Pr(1-CN) | C(=O)Me | Cl | CH₂OC(=O)OEt | O |

**[Table 479]**

| Compound Number | Y | W | Z | R¹ | R² | R³ | R¹⁰ | A |
|---|---|---|---|---|---|---|---|---|
| C-11035 | Me(R) | single bond | H | c-Pr(1-CN) | C(=O)Me | Cl | CH₂OC(=O)OEt | O |
| C-11036 | Me(S) | single bond | H | c-Pr(1-CN) | C(=O)Me | Cl | CH₂OC(=O)OEt | O |
| C-11037 | H | single bond | Me | CH₂CF₃ | C(=O)Me | Me | CH₂OC(=O)OEt | O |
| C-11038 | Me | single bond | H | CH₂CF₃ | C(=O)Me | Me | CH₂OC(=O)OEt | O |
| C-11039 | Me(R) | single bond | H | CH₂CF₃ | C(=O)Me | Me | CH₂OC(=O)OEt | O |
| C-11040 | Me(S) | single bond | H | CH₂CF₃ | C(=O)Me | Me | CH₂OC(=O)OEt | O |
| C-11041 | H | single bond | Me | Et | C(=O)Me | Me | CH₂OC(=O)OEt | O |
| C-11042 | Me | single bond | H | Et | C(=O)Me | Me | CH₂OC(=O)OEt | O |
| C-11043 | Me(R) | single bond | H | Et | C(=O)Me | Me | CH₂OC(=O)OEt | O |
| C-11044 | Me(S) | single bond | H | Et | C(=O)Me | Me | CH₂OC(=O)OEt | O |
| C-11045 | H | single bond | Me | c-Pr | C(=O)Me | Me | CH₂OC(=O)OEt | O |
| C-11046 | Me | single bond | H | c-Pr | C(=O)Me | Me | CH₂OC(=O)OEt | O |
| C-11047 | Me(R) | single bond | H | c-Pr | C(=O)Me | Me | CH₂OC(=O)OEt | O |
| C-11048 | Me(S) | single bond | H | c-Pr | C(=O)Me | Me | CH₂OC(=O)OEt | O |
| C-11049 | H | single bond | Me | c-Pr(1-CN) | C(=O)Me | Me | CH₂OC(=O)OEt | O |
| C-11050 | Me | single bond | H | c-Pr(1-CN) | C(=O)Me | Me | CH₂OC(=O)OEt | O |
| C-11051 | Me(R) | single bond | H | c-Pr(1-CN) | C(=O)Me | Me | CH₂OC(=O)OEt | O |
| C-11052 | Me(S) | single bond | H | c-Pr(1-CN) | C(=O)Me | Me | CH₂OC(=O)OEt | O |
| C-11053 | H | single bond | Me | CH₂CF₃ | C(=O)Me | Cl | CH₂OC(=O)O(*i*-Pr) | O |
| C-11054 | Me | single bond | H | CH₂CF₃ | C(=O)Me | Cl | CH₂OC(=O)O(*i*-Pr) | O |
| C-11055 | Me(R) | single bond | H | CH₂CF₃ | C(=O)Me | Cl | CH₂OC(=O)O(*i*-Pr) | O |
| C-11056 | Me(S) | single bond | H | CH₂CF₃ | C(=O)Me | Cl | CH₂OC(=O)O(*i*-Pr) | O |
| C-11057 | H | single bond | Me | Et | C(=O)Me | Cl | CH₂OC(=O)O(*i*-Pr) | O |
| C-11058 | Me | single bond | H | Et | C(=O)Me | Cl | CH₂OC(=O)O(*i*-Pr) | O |
| C-11059 | Me(R) | single bond | H | Et | C(=O)Me | Cl | CH₂OC(=O)O(*i*-Pr) | O |
| C-11060 | Me(S) | single bond | H | Et | C(=O)Me | Cl | CH₂OC(=O)O(*i*-Pr) | O |
| C-11061 | H | single bond | Me | c-Pr | C(=O)Me | Cl | CH₂OC(=O)O(*i*-Pr) | O |
| C-11062 | Me | single bond | H | c-Pr | C(=O)Me | Cl | CH₂OC(=O)O(*i*-Pr) | O |
| C-11063 | Me(R) | single bond | H | c-Pr | C(=O)Me | Cl | CH₂OC(=O)O(*i*-Pr) | O |
| C-11064 | Me(S) | single bond | H | c-Pr | C(=O)Me | Cl | CH₂OC(=O)O(*i*-Pr) | O |
| C-11065 | H | single bond | Me | c-Pr(1-CN) | C(=O)Me | Cl | CH₂OC(=O)O(*i*-Pr) | O |
| C-11066 | Me | single bond | H | c-Pr(1-CN) | C(=O)Me | Cl | CH₂OC(=O)O(*i*-Pr) | O |
| C-11067 | Me(R) | single bond | H | c-Pr(1-CN) | C(=O)Me | Cl | CH₂OC(=O)O(*i*-Pr) | O |
| C-11068 | Me(S) | single bond | H | c-Pr(1-CN) | C(=O)Me | Cl | CH₂OC(=O)O(*i*-Pr) | O |
| C-11069 | H | single bond | Me | CH₂CF₃ | C(=O)Me | Me | CH₂OC(=O)O(*i*-Pr) | O |
| C-11070 | Me | single bond | H | CH₂CF₃ | C(=O)Me | Me | CH₂OC(=O)O(*i*-Pr) | O |
| C-11071 | Me(R) | single bond | H | CH₂CF₃ | C(=O)Me | Me | CH₂OC(=O)O(*i*-Pr) | O |
| C-11072 | Me(S) | single bond | H | CH₂CF₃ | C(=O)Me | Me | CH₂OC(=O)O(*i*-Pr) | O |
| C-11073 | H | single bond | Me | Et | C(=O)Me | Me | CH₂OC(=O)O(*i*-Pr) | O |
| C-11074 | Me | single bond | H | Et | C(=O)Me | Me | CH₂OC(=O)O(*i*-Pr) | O |
| C-11075 | Me(R) | single bond | H | Et | C(=O)Me | Me | CH₂OC(=O)O(*i*-Pr) | O |
| C-11076 | Me(S) | single bond | H | Et | C(=O)Me | Me | CH₂OC(=O)O(*i*-Pr) | O |
| C-11077 | H | single bond | Me | c-Pr | C(=O)Me | Me | CH₂OC(=O)O(*i*-Pr) | O |
| C-11078 | Me | single bond | H | c-Pr | C(=O)Me | Me | CH₂OC(=O)O(*i*-Pr) | O |

**[Table 480]**

| Compound Number | Y | W | Z | R¹ | R² | R³ | R¹⁰ | A |
|---|---|---|---|---|---|---|---|---|
| C-11079 | Me(R) | single bond | H | c-Pr | C(=O)Me | Me | CH₂OC(=O)O(*i*-Pr) | O |
| C-11080 | Me(S) | single bond | H | c-Pr | C(=O)Me | Me | CH₂OC(=O)O(*i*-Pr) | O |
| C-11081 | H | single bond | Me | c-Pr(1-CN) | C(=O)Me | Me | CH₂OC(=O)O(*i*-Pr) | O |
| C-11082 | Me | single bond | H | c-Pr(1-CN) | C(=O)Me | Me | CH₂OC(=O)O(*i*-Pr) | O |
| C-11083 | Me(R) | single bond | H | c-Pr(1-CN) | C(=O)Me | Me | CH₂OC(=O)O(*i*-Pr) | O |
| C-11084 | Me(S) | single bond | H | c-Pr(1-CN) | C(=O)Me | Me | CH₂OC(=O)O(*i*-Pr) | O |
| C-11085 | H | single bond | Me | CH₂CF₃ | C(=O)Me | Cl | CH₂N(Me)C(=O)OMe | O |
| C-11086 | Me | single bond | H | CH₂CF₃ | C(=O)Me | Cl | CH₂N(Me)C(=O)OMe | O |
| C-11087 | Me(R) | single bond | H | CH₂CF₃ | C(=O)Me | Cl | CH₂N(Me)C(=O)OMe | O |
| C-11088 | Me(S) | single bond | H | CH₂CF₃ | C(=O)Me | Cl | CH₂N(Me)C(=O)OMe | O |
| C-11089 | H | single bond | Me | Et | C(=O)Me | Cl | CH₂N(Me)C(=O)OMe | O |
| C-11090 | Me | single bond | H | Et | C(=O)Me | Cl | CH₂N(Me)C(=O)OMe | O |
| C-11091 | Me(R) | single bond | H | Et | C(=O)Me | Cl | CH₂N(Me)C(=O)OMe | O |
| C-11092 | Me(S) | single bond | H | Et | C(=O)Me | Cl | CH₂N(Me)C(=O)OMe | O |
| C-11093 | H | single bond | Me | c-Pr | C(=O)Me | Cl | CH₂N(Me)C(=O)OMe | O |
| C-11094 | Me | single bond | H | c-Pr | C(=O)Me | Cl | CH₂N(Me)C(=O)OMe | O |
| C-11095 | Me(R) | single bond | H | c-Pr | C(=O)Me | Cl | CH₂N(Me)C(=O)OMe | O |
| C-11096 | Me(S) | single bond | H | c-Pr | C(=O)Me | Cl | CH₂N(Me)C(=O)OMe | O |
| C-11097 | H | single bond | Me | c-Pr(1-CN) | C(=O)Me | Cl | CH₂N(Me)C(=O)OMe | O |
| C-11098 | Me | single bond | H | c-Pr(1-CN) | C(=O)Me | Cl | CH₂N(Me)C(=O)OMe | O |
| C-11099 | Me(R) | single bond | H | c-Pr(1-CN) | C(=O)Me | Cl | CH₂N(Me)C(=O)OMe | O |
| C-11100 | Me(S) | single bond | H | c-Pr(1-CN) | C(=O)Me | Cl | CH₂N(Me)C(=O)OMe | O |
| C-11101 | H | single bond | Me | CH₂CF₃ | C(=O)Me | Me | CH₂N(Me)C(=O)OMe | O |
| C-11102 | Me | single bond | H | CH₂CF₃ | C(=O)Me | Me | CH₂N(Me)C(=O)OMe | O |
| C-11103 | Me(R) | single bond | H | CH₂CF₃ | C(=O)Me | Me | CH₂N(Me)C(=O)OMe | O |
| C-11104 | Me(S) | single bond | H | CH₂CF₃ | C(=O)Me | Me | CH₂N(Me)C(=O)OMe | O |
| C-11105 | H | single bond | Me | Et | C(=O)Me | Me | CH₂N(Me)C(=O)OMe | O |
| C-11106 | Me | single bond | H | Et | C(=O)Me | Me | CH₂N(Me)C(=O)OMe | O |
| C-11107 | Me(R) | single bond | H | Et | C(=O)Me | Me | CH₂N(Me)C(=O)OMe | O |
| C-11108 | Me(S) | single bond | H | Et | C(=O)Me | Me | CH₂N(Me)C(=O)OMe | O |
| C-11109 | H | single bond | Me | c-Pr | C(=O)Me | Me | CH₂N(Me)C(=O)OMe | O |
| C-11110 | Me | single bond | H | c-Pr | C(=O)Me | Me | CH₂N(Me)C(=O)OMe | O |
| C-11111 | Me(R) | single bond | H | c-Pr | C(=O)Me | Me | CH₂N(Me)C(=O)OMe | O |
| C-11112 | Me(S) | single bond | H | c-Pr | C(=O)Me | Me | CH₂N(Me)C(=O)OMe | O |
| C-11113 | H | single bond | Me | c-Pr(1-CN) | C(=O)Me | Me | CH₂N(Me)C(=O)OMe | O |
| C-11114 | Me | single bond | H | c-Pr(1-CN) | C(=O)Me | Me | CH₂N(Me)C(=O)OMe | O |
| C-11115 | Me(R) | single bond | H | c-Pr(1-CN) | C(=O)Me | Me | CH₂N(Me)C(=O)OMe | O |
| C-11116 | Me(S) | single bond | H | c-Pr(1-CN) | C(=O)Me | Me | CH₂N(Me)C(=O)OMe | O |
| C-11117 | H | single bond | Me | CH₂CF₃ | C(=O)Me | Cl | CH₂OCH₂CH₂OMe | O |
| C-11118 | Me | single bond | H | CH₂CF₃ | C(=O)Me | Cl | CH₂OCH₂CH₂OMe | O |
| C-11119 | Me(R) | single bond | H | CH₂CF₃ | C(=O)Me | Cl | CH₂OCH₂CH₂OMe | O |
| C-11120 | Me(S) | single bond | H | CH₂CF₃ | C(=O)Me | Cl | CH₂OCH₂CH₂OMe | O |
| C-11121 | H | single bond | Me | Et | C(=O)Me | Cl | CH₂OCH₂CH₂OMe | O |
| C-11122 | Me | single bond | H | Et | C(=O)Me | Cl | CH₂OCH₂CH₂OMe | O |

**[Table 481]**

| Compound Number | Y | W | Z | R¹ | R² | R³ | R¹⁰ | A |
|---|---|---|---|---|---|---|---|---|
| C-11123 | Me(R) | single bond | H | Et | C(=O)Me | Cl | CH₂OCH₂CH₂OMe | O |
| C-11124 | Me(S) | single bond | H | Et | C(=O)Me | Cl | CH₂OCH₂CH₂OMe | O |
| C-11125 | H | single bond | Me | c-Pr | C(=O)Me | Cl | CH₂OCH₂CH₂OMe | O |
| C-11126 | Me | single bond | H | c-Pr | C(=O)Me | Cl | CH₂OCH₂CH₂OMe | O |
| C-11127 | Me(R) | single bond | H | c-Pr | C(=O)Me | Cl | CH₂OCH₂CH₂OMe | O |
| C-11128 | Me(S) | single bond | H | c-Pr | C(=O)Me | Cl | CH₂OCH₂CH₂OMe | O |
| C-11129 | H | single bond | Me | c-Pr(1-CN) | C(=O)Me | Cl | CH₂OCH₂CH₂OMe | O |
| C-11130 | Me | single bond | H | c-Pr(1-CN) | C(=O)Me | Cl | CH₂OCH₂CH₂OMe | O |
| C-11131 | Me(R) | single bond | H | c-Pr(1-CN) | C(=O)Me | Cl | CH₂OCH₂CH₂OMe | O |
| C-11132 | Me(S) | single bond | H | c-Pr(1-CN) | C(=O)Me | Cl | CH₂OCH₂CH₂OMe | O |
| C-11133 | H | single bond | Me | CH₂CF₃ | C(=O)Me | Me | CH₂OCH₂CH₂OMe | O |
| C-11134 | Me | single bond | H | CH₂CF₃ | C(=O)Me | Me | CH₂OCH₂CH₂OMe | O |
| C-11135 | Me(R) | single bond | H | CH₂CF₃ | C(=O)Me | Me | CH₂OCH₂CH₂OMe | O |
| C-11136 | Me(S) | single bond | H | CH₂CF₃ | C(=O)Me | Me | CH₂OCH₂CH₂OMe | O |
| C-11137 | H | single bond | Me | Et | C(=O)Me | Me | CH₂OCH₂CH₂OMe | O |
| C-11138 | Me | single bond | H | Et | C(=O)Me | Me | CH₂OCH₂CH₂OMe | O |
| C-11139 | Me(R) | single bond | H | Et | C(=O)Me | Me | CH₂OCH₂CH₂OMe | O |
| C-11140 | Me(S) | single bond | H | Et | C(=O)Me | Me | CH₂OCH₂CH₂OMe | O |
| C-11141 | H | single bond | Me | c-Pr | C(=O)Me | Me | CH₂OCH₂CH₂OMe | O |
| C-11142 | Me | single bond | H | c-Pr | C(=O)Me | Me | CH₂OCH₂CH₂OMe | O |
| C-11143 | Me(R) | single bond | H | c-Pr | C(=O)Me | Me | CH₂OCH₂CH₂OMe | O |
| C-11144 | Me(S) | single bond | H | c-Pr | C(=O)Me | Me | CH₂OCH₂CH₂OMe | O |
| C-11145 | H | single bond | Me | c-Pr(1-CN) | C(=O)Me | Me | CH₂OCH₂CH₂OMe | O |
| C-11146 | Me | single bond | H | c-Pr(1-CN) | C(=O)Me | Me | CH₂OCH₂CH₂OMe | O |
| C-11147 | Me(R) | single bond | H | c-Pr(1-CN) | C(=O)Me | Me | CH₂OCH₂CH₂OMe | O |
| C-11148 | Me(S) | single bond | H | c-Pr(1-CN) | C(=O)Me | Me | CH₂OCH₂CH₂OMe | O |
| C-11149 | H | single bond | Me | CH₂CF₃ | C(=O)Me | Cl | CH₂OCH₂CH₂CN | O |
| C-11150 | Me | single bond | H | CH₂CF₃ | C(=O)Me | Cl | CH₂OCH₂CH₂CN | O |
| C-11151 | Me(R) | single bond | H | CH₂CF₃ | C(=O)Me | Cl | CH₂OCH₂CH₂CN | O |
| C-11152 | Me(S) | single bond | H | CH₂CF₃ | C(=O)Me | Cl | CH₂OCH₂CH₂CN | O |
| C-11153 | H | single bond | Me | Et | C(=O)Me | Cl | CH₂OCH₂CH₂CN | O |
| C-11154 | Me | single bond | H | Et | C(=O)Me | Cl | CH₂OCH₂CH₂CN | O |
| C-11155 | Me(R) | single bond | H | Et | C(=O)Me | Cl | CH₂OCH₂CH₂CN | O |
| C-11156 | Me(S) | single bond | H | Et | C(=O)Me | Cl | CH₂OCH₂CH₂CN | O |
| C-11157 | H | single bond | Me | c-Pr | C(=O)Me | Cl | CH₂OCH₂CH₂CN | O |
| C-11158 | Me | single bond | H | c-Pr | C(=O)Me | Cl | CH₂OCH₂CH₂CN | O |
| C-11159 | Me(R) | single bond | H | c-Pr | C(=O)Me | Cl | CH₂OCH₂CH₂CN | O |
| C-11160 | Me(S) | single bond | H | c-Pr | C(=O)Me | Cl | CH₂OCH₂CH₂CN | O |
| C-11161 | H | single bond | Me | c-Pr(1-CN) | C(=O)Me | Cl | CH₂OCH₂CH₂CN | O |
| C-11162 | Me | single bond | H | c-Pr(1-CN) | C(=O)Me | Cl | CH₂OCH₂CH₂CN | O |
| C-11163 | Me(R) | single bond | H | c-Pr(1-CN) | C(=O)Me | Cl | CH₂OCH₂CH₂CN | O |
| C-11164 | Me(S) | single bond | H | c-Pr(1-CN) | C(=O)Me | Cl | CH₂OCH₂CH₂CN | O |
| C-11165 | H | single bond | Me | CH₂CF₃ | C(=O)Me | Me | CH₂OCH₂CH₂CN | O |
| C-11166 | Me | single bond | H | CH₂CF₃ | C(=O)Me | Me | CH₂OCH₂CH₂CN | O |

**[Table 482]**

| Compound Number | Y | W | Z | R¹ | R² | R³ | R¹⁰ | A |
|---|---|---|---|---|---|---|---|---|
| C-11167 | Me(R) | single bond | H | CH₂CF₃ | C(=O)Me | Me | CH₂OCH₂CH₂CN | O |
| C-11168 | Me(S) | single bond | H | CH₂CF₃ | C(=O)Me | Me | CH₂OCH₂CH₂CN | O |
| C-11169 | H | single bond | Me | Et | C(=O)Me | Me | CH₂OCH₂CH₂CN | O |
| C-11170 | Me | single bond | H | Et | C(=O)Me | Me | CH₂OCH₂CH₂CN | O |
| C-11171 | Me(R) | single bond | H | Et | C(=O)Me | Me | CH₂OCH₂CH₂CN | O |
| C-11172 | Me(S) | single bond | H | Et | C(=O)Me | Me | CH₂OCH₂CH₂CN | O |
| C-11173 | H | single bond | Me | c-Pr | C(=O)Me | Me | CH₂OCH₂CH₂CN | O |
| C-11174 | Me | single bond | H | c-Pr | C(=O)Me | Me | CH₂OCH₂CH₂CN | O |
| C-11175 | Me(R) | single bond | H | c-Pr | C(=O)Me | Me | CH₂OCH₂CH₂CN | O |
| C-11176 | Me(S) | single bond | H | c-Pr | C(=O)Me | Me | CH₂OCH₂CH₂CN | O |
| C-11177 | H | single bond | Me | c-Pr(1-CN) | C(=O)Me | Me | CH₂OCH₂CH₂CN | O |
| C-11178 | Me | single bond | H | c-Pr(1-CN) | C(=O)Me | Me | CH₂OCH₂CH₂CN | O |
| C-11179 | Me(R) | single bond | H | c-Pr(1-CN) | C(=O)Me | Me | CH₂OCH₂CH₂CN | O |
| C-11180 | Me(S) | single bond | H | c-Pr(1-CN) | C(=O)Me | Me | CH₂OCH₂CH₂CN | O |
| C-11181 | H | single bond | Me | CH₂CF₃ | C(=O)Me | Cl | CH₂OCH₂CH₂S(=O)₂Me | O |
| C-11182 | Me | single bond | H | CH₂CF₃ | C(=O)Me | Cl | CH₂OCH₂CH₂S(=O)₂Me | O |
| C-11183 | Me(R) | single bond | H | CH₂CF₃ | C(=O)Me | Cl | CH₂OCH₂CH₂S(=O)₂Me | O |
| C-11184 | Me(S) | single bond | H | CH₂CF₃ | C(=O)Me | Cl | CH₂OCH₂CH₂S(=O)₂Me | O |
| C-11185 | H | single bond | Me | Et | C(=O)Me | Cl | CH₂OCH₂CH₂S(=O)₂Me | O |
| C-11186 | Me | single bond | H | Et | C(=O)Me | Cl | CH₂OCH₂CH₂S(=O)₂Me | O |
| C-11187 | Me(R) | single bond | H | Et | C(=O)Me | Cl | CH₂OCH₂CH₂S(=O)₂Me | O |
| C-11188 | Me(S) | single bond | H | Et | C(=O)Me | Cl | CH₂OCH₂CH₂S(=O)₂Me | O |
| C-11189 | H | single bond | Me | c-Pr | C(=O)Me | Cl | CH₂OCH₂CH₂S(=O)₂Me | O |
| C-11190 | Me | single bond | H | c-Pr | C(=O)Me | Cl | CH₂OCH₂CH₂S(=O)₂Me | O |
| C-11191 | Me(R) | single bond | H | c-Pr | C(=O)Me | Cl | CH₂OCH₂CH₂S(=O)₂Me | O |
| C-11192 | Me(S) | single bond | H | c-Pr | C(=O)Me | Cl | CH₂OCH₂CH₂S(=O)₂Me | O |
| C-11193 | H | single bond | Me | c-Pr(1-CN) | C(=O)Me | Cl | CH₂OCH₂CH₂S(=O)₂Me | O |
| C-11194 | Me | single bond | H | c-Pr(1-CN) | C(=O)Me | Cl | CH₂OCH₂CH₂S(=O)₂Me | O |
| C-11195 | Me(R) | single bond | H | c-Pr(1-CN) | C(=O)Me | Cl | CH₂OCH₂CH₂S(=O)₂Me | O |
| C-11196 | Me(S) | single bond | H | c-Pr(1-CN) | C(=O)Me | Cl | CH₂OCH₂CH₂S(=O)₂Me | O |
| C-11197 | H | single bond | Me | CH₂CF₃ | C(=O)Me | Me | CH₂OCH₂CH₂S(=O)₂Me | O |
| C-11198 | Me | single bond | H | CH₂CF₃ | C(=O)Me | Me | CH₂OCH₂CH₂S(=O)₂Me | O |
| C-11199 | Me(R) | single bond | H | CH₂CF₃ | C(=O)Me | Me | CH₂OCH₂CH₂S(=O)₂Me | O |
| C-11200 | Me(S) | single bond | H | CH₂CF₃ | C(=O)Me | Me | CH₂OCH₂CH₂S(=O)₂Me | O |
| C-11201 | H | single bond | Me | Et | C(=O)Me | Me | CH₂OCH₂CH₂S(=O)₂Me | O |
| C-11202 | Me | single bond | H | Et | C(=O)Me | Me | CH₂OCH₂CH₂S(=O)₂Me | O |
| C-11203 | Me(R) | single bond | H | Et | C(=O)Me | Me | CH₂OCH₂CH₂S(=O)₂Me | O |
| C-11204 | Me(S) | single bond | H | Et | C(=O)Me | Me | CH₂OCH₂CH₂S(=O)₂Me | O |
| C-11205 | H | single bond | Me | c-Pr | C(=O)Me | Me | CH₂OCH₂CH₂S(=O)₂Me | O |
| C-11206 | Me | single bond | H | c-Pr | C(=O)Me | Me | CH₂OCH₂CH₂S(=O)₂Me | O |
| C-11207 | Me(R) | single bond | H | c-Pr | C(=O)Me | Me | CH₂OCH₂CH₂S(=O)₂Me | O |
| C-11208 | Me(S) | single bond | H | c-Pr | C(=O)Me | Me | CH₂OCH₂CH₂S(=O)₂Me | O |
| C-11209 | H | single bond | Me | c-Pr(1-CN) | C(=O)Me | Me | CH₂OCH₂CH₂S(=O)₂Me | O |
| C-11210 | Me | single bond | H | c-Pr(1-CN) | C(=O)Me | Me | CH₂OCH₂CH₂S(=O)₂Me | O |

**[Table 483]**

| Compound Number | Y | W | Z | R¹ | R² | R³ | R¹⁰ | A |
|---|---|---|---|---|---|---|---|---|
| C-11211 | Me(R) | single bond | H | c-Pr(1-CN) | C(=O)Me | Me | CH₂OCH₂CH₂S(=O)₂Me | O |
| C-11212 | Me(S) | single bond | H | c-Pr(1-CN) | C(=O)Me | Me | CH₂OCH₂CH₂S(=O)₂Me | O |
| C-11213 | H | single bond | Me | CH₂CF₃ | C(=O)Me | Cl | CH₂(5-Me-1,3-dioxol-2-one-4-yl) | O |
| C-11214 | Me | single bond | H | CH₂CF₃ | C(=O)Me | Cl | CH₂(5-Me-1,3-dioxol-2-one-4-yl) | O |
| C-11215 | Me(R) | single bond | H | CH₂CF₃ | C(=O)Me | Cl | CH₂(5-Me-1,3-dioxol-2-one-4-yl) | O |
| C-11216 | Me(S) | single bond | H | CH₂CF₃ | C(=O)Me | Cl | CH₂(5-Me-1,3-dioxol-2-one-4-yl) | O |
| C-11217 | H | single bond | Me | Et | C(=O)Me | Cl | CH₂(5-Me-1,3-dioxol-2-one-4-yl) | O |
| C-11218 | Me | single bond | H | Et | C(=O)Me | Cl | CH₂(5-Me-1,3-dioxol-2-one-4-yl) | O |
| C-11219 | Me(R) | single bond | H | Et | C(=O)Me | Cl | CH₂(5-Me-1,3-dioxol-2-one-4-yl) | O |
| C-11220 | Me(S) | single bond | H | Et | C(=O)Me | Cl | CH₂(5-Me-1,3-dioxol-2-one-4-yl) | O |
| C-11221 | H | single bond | Me | c-Pr | C(=O)Me | Cl | CH₂(5-Me-1,3-dioxol-2-one-4-yl) | O |
| C-11222 | Me | single bond | H | c-Pr | C(=O)Me | Cl | CH₂(5-Me-1,3-dioxol-2-one-4-yl) | O |
| C-11223 | Me(R) | single bond | H | c-Pr | C(=O)Me | Cl | CH₂(5-Me-1,3-dioxol-2-one-4-yl) | O |
| C-11224 | Me(S) | single bond | H | c-Pr | C(=O)Me | Cl | CH₂(5-Me-1,3-dioxol-2-one-4-yl) | O |
| C-11225 | H | single bond | Me | c-Pr(1-CN) | C(=O)Me | Cl | CH₂(5-Me-1,3-dioxol-2-one-4-yl) | O |
| C-11226 | Me | single bond | H | c-Pr(1-CN) | C(=O)Me | Cl | CH₂(5-Me-1,3-dioxol-2-one-4-yl) | O |
| C-11227 | Me(R) | single bond | H | c-Pr(1-CN) | C(=O)Me | Cl | CH₂(5-Me-1,3-dioxol-2-one-4-yl) | O |
| C-11228 | Me(S) | single bond | H | c-Pr(1-CN) | C(=O)Me | Cl | CH₂(5-Me-1,3-dioxol-2-one-4-yl) | O |
| C-11229 | H | single bond | Me | CH₂CF₃ | C(=O)Me | Me | CH₂(5-Me-1,3-dioxol-2-one-4-yl) | O |
| C-11230 | Me | single bond | H | CH₂CF₃ | C(=O)Me | Me | CH₂(5-Me-1,3-dioxol-2-one-4-yl) | O |
| C-11231 | Me(R) | single bond | H | CH₂CF₃ | C(=O)Me | Me | CH₂(5-Me-1,3-dioxol-2-one-4-yl) | O |
| C-11232 | Me(S) | single bond | H | CH₂CF₃ | C(=O)Me | Me | CH₂(5-Me-1,3-dioxol-2-one-4-yl) | O |
| C-11233 | H | single bond | Me | Et | C(=O)Me | Me | CH₂(5-Me-1,3-dioxol-2-one-4-yl) | O |
| C-11234 | Me | single bond | H | Et | C(=O)Me | Me | CH₂(5-Me-1,3-dioxol-2-one-4-yl) | O |
| C-11235 | Me(R) | single bond | H | Et | C(=O)Me | Me | CH₂(5-Me-1,3-dioxol-2-one-4-yl) | O |
| C-11236 | Me(S) | single bond | H | Et | C(=O)Me | Me | CH₂(5-Me-1,3-dioxol-2-one-4-yl) | O |
| C-11237 | H | single bond | Me | c-Pr | C(=O)Me | Me | CH₂(5-Me-1,3-dioxol-2-one-4-yl) | O |
| C-11238 | Me | single bond | H | c-Pr | C(=O)Me | Me | CH₂(5-Me-1,3-dioxol-2-one-4-yl) | O |
| C-11239 | Me(R) | single bond | H | c-Pr | C(=O)Me | Me | CH₂(5-Me-1,3-dioxol-2-one-4-yl) | O |
| C-11240 | Me(S) | single bond | H | c-Pr | C(=O)Me | Me | CH₂(5-Me-1,3-dioxol-2-one-4-yl) | O |
| C-11241 | H | single bond | Me | c-Pr(1-CN) | C(=O)Me | Me | CH₂(5-Me-1,3-dioxol-2-one-4-yl) | O |
| C-11242 | Me | single bond | H | c-Pr(1-CN) | C(=O)Me | Me | CH₂(5-Me-1,3-dioxol-2-one-4-yl) | O |
| C-11243 | Me(R) | single bond | H | c-Pr(1-CN) | C(=O)Me | Me | CH₂(5-Me-1,3-dioxol-2-one-4-yl) | O |
| C-11244 | Me(S) | single bond | H | c-Pr(1-CN) | C(=O)Me | Me | CH₂(5-Me-1,3-dioxol-2-one-4-yl) | O |

**[Table 484]**

| 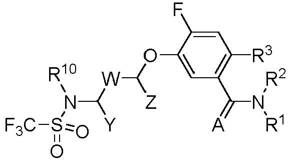 | | | | | | | | |
|---|---|---|---|---|---|---|---|---|
| Compound Number | Y | W | Z | R¹ | R² | R³ | R¹⁰ | A |
| C-11245 | H | single bond | Me | Me | H | Cl | H | O |
| C-11246 | Me | single bond | H | Me | H | Cl | H | O |
| C-11247 | Me(R) | single bond | H | Me | H | Cl | H | O |
| C-11248 | Me(S) | single bond | H | Me | H | Cl | H | O |
| C-11249 | H | single bond | Me | Me | H | Me | H | O |
| C-11250 | Me | single bond | H | Me | H | Me | H | O |
| C-11251 | Me(R) | single bond | H | Me | H | Me | H | O |
| C-11252 | Me(S) | single bond | H | Me | H | Me | H | O |
| C-11253 | H | single bond | Me | CH₂CHF₂ | H | Cl | H | O |
| C-11254 | Me | single bond | H | CH₂CHF₂ | H | Cl | H | O |
| C-11255 | Me(R) | single bond | H | CH₂CHF₂ | H | Cl | H | O |
| C-11256 | Me(S) | single bond | H | CH₂CHF₂ | H | Cl | H | O |
| C-11257 | H | single bond | Me | CH₂CHF₂ | H | Me | H | O |
| C-11258 | Me | single bond | H | CH₂CHF₂ | H | Me | H | O |
| C-11259 | Me(R) | single bond | H | CH₂CHF₂ | H | Me | H | O |
| C-11260 | Me(S) | single bond | H | CH₂CHF₂ | H | Me | H | O |
| C-11261 | H | single bond | Me | *n*-Pr | H | Cl | H | O |
| C-11262 | Me | single bond | H | *n*-Pr | H | Cl | H | O |
| C-11263 | Me(R) | single bond | H | *n*-Pr | H | Cl | H | O |
| C-11264 | Me(S) | single bond | H | *n*-Pr | H | Cl | H | O |
| C-11265 | H | single bond | Me | *n*-Pr | H | Me | H | O |
| C-11266 | Me | single bond | H | *n*-Pr | H | Me | H | O |
| C-11267 | Me(R) | single bond | H | *n*-Pr | H | Me | H | O |
| C-11268 | Me(S) | single bond | H | *n*-Pr | H | Me | H | O |
| C-11269 | H | single bond | Me | *i*-Pr | H | Cl | H | O |
| C-11270 | Me | single bond | H | *i*-Pr | H | Cl | H | O |
| C-11271 | Me(R) | single bond | H | *i-*Pr | H | Cl | H | O |
| C-11272 | Me(S) | single bond | H | *i*-Pr | H | Cl | H | O |
| C-11273 | H | single bond | Me | *i*-Pr | H | Me | H | O |
| C-11274 | Me | single bond | H | *i*-Pr | H | Me | H | O |
| C-11275 | Me(R) | single bond | H | *i*-Pr | H | Me | H | O |
| C-11276 | Me(S) | single bond | H | *i*-Pr | H | Me | H | O |
| C-11277 | H | single bond | Me | *c*-Pr(1-F) | H | Cl | H | O |
| C-11278 | Me | single bond | H | *c*-Pr(1-F) | H | Cl | H | O |
| C-11279 | Me(R) | single bond | H | *c*-Pr(1-F) | H | Cl | H | O |
| C-11280 | Me(S) | single bond | H | *c*-Pr(1-F) | H | Cl | H | O |
| C-11281 | H | single bond | Me | *c*-Pr(1-F) | H | Me | H | O |
| C-11282 | Me | single bond | H | *c*-Pr(1-F) | H | Me | H | O |

**[Table 485]**

| Compound Number | Y | W | Z | R¹ | R² | R³ | R¹⁰ | A |
|---|---|---|---|---|---|---|---|---|
| C-11283 | Me(R) | single bond | H | *c*-Pr(1-F) | H | Me | H | O |
| C-11284 | Me(S) | single bond | H | *c*-Pr(1-F) | H | Me | H | O |
| C-11285 | H | single bond | Me | *c*-Pr(2,2-F₂) | H | Cl | H | O |
| C-11286 | Me | single bond | H | *c*-Pr(2,2-F₂) | H | Cl | H | O |
| C-11287 | Me(R) | single bond | H | *c*-Pr(2,2-F₂) | H | Cl | H | O |
| C-11288 | Me(S) | single bond | H | *c*-Pr(2,2-F₂) | H | Cl | H | O |
| C-11289 | H | single bond | Me | *c*-Pr(2,2-F₂) | H | Me | H | O |
| C-11290 | Me | single bond | H | *c*-Pr(2,2-F₂) | H | Me | H | O |
| C-11291 | Me(R) | single bond | H | *c*-Pr(2,2-F₂) | H | Me | H | O |
| C-11292 | Me(S) | single bond | H | *c*-Pr(2,2-F₂) | H | Me | H | O |
| C-11293 | H | single bond | Me | *c*-Pr(1-Cl) | H | Cl | H | O |
| C-11294 | Me | single bond | H | *c*-Pr(1-Cl) | H | Cl | H | O |
| C-11295 | Me(R) | single bond | H | *c*-Pr(1-Cl) | H | Cl | H | O |
| C-11296 | Me(S) | single bond | H | *c-*Pr(1-Cl) | H | Cl | H | O |
| C-11297 | H | single bond | Me | *c*-Pr(1-Cl) | H | Me | H | O |
| C-11298 | Me | single bond | H | *c*-Pr(1-Cl) | H | Me | H | O |
| C-11299 | Me(R) | single bond | H | *c*-Pr(1-Cl) | H | Me | H | O |
| C-11300 | Me(S) | single bond | H | *c*-Pr(1-Cl) | H | Me | H | O |
| C-11301 | H | single bond | Me | *c*-Pr(1-Br) | H | Cl | H | O |
| C-11302 | Me | single bond | H | *c*-Pr(1-Br) | H | Cl | H | O |
| C-11303 | Me(R) | single bond | H | *c*-Pr(1-Br) | H | Cl | H | O |
| C-11304 | Me(S) | single bond | H | *c*-Pr(1-Br) | H | Cl | H | O |
| C-11305 | H | single bond | Me | *c*-Pr(1-Br) | H | Me | H | O |
| C-11306 | Me | single bond | H | *c*-Pr(1-Br) | H | Me | H | O |
| C-11307 | Me(R) | single bond | H | *c*-Pr(1-Br) | H | Me | H | O |
| C-11308 | Me(S) | single bond | H | *c*-Pr(1-Br) | H | Me | H | O |
| C-11309 | H | single bond | Me | *c* -Pr(1-Me) | H | Cl | H | O |
| C-11310 | Me | single bond | H | *c* -Pr(1-Me) | H | Cl | H | O |
| C-11311 | Me(R) | single bond | H | *c*-Pr(1-Me) | H | Cl | H | O |
| C-11312 | Me(S) | single bond | H | *c*-Pr(1-Me) | H | Cl | H | O |
| C-11313 | H | single bond | Me | *c*-Pr(1-Me) | H | Me | H | O |
| C-11314 | Me | single bond | H | *c*-Pr(1-Me) | H | Me | H | O |
| C-11315 | Me(R) | single bond | H | *c*-Pr(1-Me) | H | Me | H | O |
| C-11316 | Me(S) | single bond | H | *c*-Pr(1-Me) | H | Me | H | O |
| C-11317 | H | single bond | Me | *c*-Pr(1-CH₂OH) | H | Cl | H | O |
| C-11318 | Me | single bond | H | *c*-Pr(1-CH₂OH) | H | Cl | H | O |
| C-11319 | Me(R) | single bond | H | *c*-Pr(1-CH₂OH) | H | Cl | H | O |
| C-11320 | Me(S) | single bond | H | *c*-Pr(1-CH₂OH) | H | Cl | H | O |
| C-11321 | H | single bond | Me | *c*-Pr(1-CH₂OH) | H | Me | H | O |
| C-11322 | Me | single bond | H | *c*-Pr(1-CH₂OH) | H | Me | H | O |
| C-11323 | Me(R) | single bond | H | *c*-Pr(1-CH₂OH) | H | Me | H | O |
| C-11324 | Me(S) | single bond | H | *c*-Pr(1-CH₂OH) | H | Me | H | O |
| C-11325 | H | single bond | Me | *c*-Pr[1-CH₂OS(=O)₂Me] | H | Cl | H | O |
| C-11326 | Me | single bond | H | *c*-Pr[1-CH₂OS(=O)₂Me] | H | Cl | H | O |

**[Table 486]**

| Compound Number | Y | W | Z | R¹ | R² | R³ | R¹⁰ | A |
|---|---|---|---|---|---|---|---|---|
| C-11327 | Me(R) | single bond | H | *c*-Pr[1-CH₂OS(=O)₂Me] | H | Cl | H | O |
| C-11328 | Me(S) | single bond | H | *c*-Pr[1-CH₂OS(=O)₂Me] | H | Cl | H | O |
| C-11329 | H | single bond | Me | *c*-Pr[1-CH₂OS(=O)₂Me] | H | Me | H | O |
| C-11330 | Me | single bond | H | *c*-Pr[1-CH₂OS(=O)₂Me] | H | Me | H | O |
| C-11331 | Me(R) | single bond | H | *c*-Pr[1-CH₂OS(=O)₂Me] | H | Me | H | O |
| C-11332 | Me(S) | single bond | H | *c*-Pr[1-CH₂OS(=O)₂Me] | H | Me | H | O |
| C-11333 | H | single bond | Me | *c*-Pr(1-CH₂OMe) | H | Cl | H | O |
| C-11334 | Me | single bond | H | *c*-Pr(1-CH₂OMe) | H | Cl | H | O |
| C-11335 | Me(R) | single bond | H | *c*-Pr(1-CH₂OMe) | H | Cl | H | O |
| C-11336 | Me(S) | single bond | H | *c*-Pr(1-CH₂OMe) | H | Cl | H | O |
| C-11337 | H | single bond | Me | *c*-Pr(1-CH₂OMe) | H | Me | H | O |
| C-11338 | Me | single bond | H | *c*-Pr(1-CH₂OMe) | H | Me | H | O |
| C-11339 | Me(R) | single bond | H | *c*-Pr(1-CH₂OMe) | H | Me | H | O |
| C-11340 | Me(S) | single bond | H | *c*-Pr(1-CH₂OMe) | H | Me | H | O |
| C-11341 | H | single bond | Me | *c*-Pr(1-CH₂CN) | H | Cl | H | O |
| C-11342 | Me | single bond | H | *c*-Pr(1-CH₂CN) | H | Cl | H | O |
| C-11343 | Me(R) | single bond | H | *c*-Pr(1-CH₂CN) | H | Cl | H | O |
| C-11344 | Me(S) | single bond | H | *c*-Pr(1-CH₂CN) | H | Cl | H | O |
| C-11345 | H | single bond | Me | *c*-Pr(1-CH₂CN) | H | Me | H | O |
| C-11346 | Me | single bond | H | *c*-Pr(1-CH₂CN) | H | Me | H | O |
| C-11347 | Me(R) | single bond | H | *c*-Pr(1-CH₂CN) | H | Me | H | O |
| C-11348 | Me(S) | single bond | H | *c*-Pr(1-CH₂CN) | H | Me | H | O |
| C-11349 | H | single bond | Me | *c*-Pr(1-CH₂SMe) | H | Cl | H | O |
| C-11350 | Me | single bond | H | *c*-Pr(1-CH₂SMe) | H | Cl | H | O |
| C-11351 | Me(R) | single bond | H | *c*-Pr(1-CH₂SMe) | H | Cl | H | O |
| C-11352 | Me(S) | single bond | H | *c*-Pr(1-CH₂SMe) | H | Cl | H | O |
| C-11353 | H | single bond | Me | *c*-Pr(1-CH₂SMe) | H | Me | H | O |
| C-11354 | Me | single bond | H | *c*-Pr(1-CH₂SMe) | H | Me | H | O |
| C-11355 | Me(R) | single bond | H | *c*-Pr(1-CH₂SMe) | H | Me | H | O |
| C-11356 | Me(S) | single bond | H | *c*-Pr(1-CH₂SMe) | H | Me | H | O |
| C-11357 | H | single bond | Me | *c*-Pr[1-CH₂S(=O)Me] | H | Cl | H | O |
| C-11358 | Me | single bond | H | *c*-Pr[1-CH₂S(=O)Me] | H | Cl | H | O |
| C-11359 | Me(R) | single bond | H | *c*-Pr[1-CH₂S(=O)Me] | H | Cl | H | O |
| C-11360 | Me(S) | single bond | H | *c*-Pr[1-CH₂S(=O)Me] | H | Cl | H | O |
| C-11361 | H | single bond | Me | *c*-Pr[1-CH₂S(=O)Me] | H | Me | H | O |
| C-11362 | Me | single bond | H | *c*-Pr[1-CH₂S(=O)Me] | H | Me | H | O |
| C-11363 | Me(R) | single bond | H | *c*-Pr[1-CH₂S(=O)Me] | H | Me | H | O |
| C-11364 | Me(S) | single bond | H | *c*-Pr[1-CH₂S(=O)Me] | H | Me | H | O |
| C-11365 | H | single bond | Me | *c*-Pr[1-CH₂S(=O)₂Me] | H | Cl | H | O |
| C-11366 | Me | single bond | H | *c*-Pr[1-CH₂S(=O)₂Me] | H | Cl | H | O |
| C-11367 | Me(R) | single bond | H | *c*-Pr[1-CH₂S(=O)₂Me] | H | Cl | H | O |
| C-11368 | Me(S) | single bond | H | *c*-Pr[1-CH₂S(=O)₂Me] | H | Cl | H | O |
| C-11369 | H | single bond | Me | *c*-Pr[1-CH₂S(=O)₂Me] | H | Me | H | O |
| C-11370 | Me | single bond | H | *c*-Pr[1-CH₂S(=O)₂Me] | H | Me | H | O |

**[Table 487]**

| Compound Number | Y | W | Z | R¹ | R² | R³ | R¹⁰ | A |
|---|---|---|---|---|---|---|---|---|
| C-11371 | Me(R) | single bond | H | *c*-Pr[1-CH₂S(=O)₂Me] | H | Me | H | O |
| C-11372 | Me(S) | single bond | H | *c*-Pr[1-CH₂S(=O)₂Me] | H | Me | H | O |
| C-11373 | H | single bond | Me | *c*-Pr(1-CH₂F) | H | Cl | H | O |
| C-11374 | Me | single bond | H | *c*-Pr(1-CH₂F) | H | Cl | H | O |
| C-11375 | Me(R) | single bond | H | *c*-Pr(1-CH₂F) | H | Cl | H | O |
| C-11376 | Me(S) | single bond | H | *c*-Pr(1-CH₂F) | H | Cl | H | O |
| C-11377 | H | single bond | Me | *c*-Pr(1-CH₂F) | H | Me | H | O |
| C-11378 | Me | single bond | H | *c*-Pr(1-CH₂F) | H | Me | H | O |
| C-11379 | Me(R) | single bond | H | *c*-Pr(1-CH₂F) | H | Me | H | O |
| C-11380 | Me(S) | single bond | H | *c*-Pr(1-CH₂F) | H | Me | H | O |
| C-11381 | H | single bond | Me | *c*-Pr(1-CHF₂) | H | Cl | H | O |
| C-11382 | Me | single bond | H | *c*-Pr(1-CHF₂) | H | Cl | H | O |
| C-11383 | Me(R) | single bond | H | *c*-Pr(1-CHF₂) | H | Cl | H | O |
| C-11384 | Me(S) | single bond | H | *c*-Pr(1-CHF₂) | H | Cl | H | O |
| C-11385 | H | single bond | Me | *c*-Pr(1-CHF₂) | H | Me | H | O |
| C-11386 | Me | single bond | H | *c*-Pr(1-CHF₂) | H | Me | H | O |
| C-11387 | Me(R) | single bond | H | *c*-Pr(1-CHF₂) | H | Me | H | O |
| C-11388 | Me(S) | single bond | H | *c*-Pr(1-CHF₂) | H | Me | H | O |
| C-11389 | H | single bond | Me | *c*-Pr(1-CF₃) | H | Cl | H | O |
| C-11390 | Me | single bond | H | *c*-Pr(1-CF₃) | H | Cl | H | O |
| C-11391 | Me(R) | single bond | H | *c*-Pr(1-CF₃) | H | Cl | H | O |
| C-11392 | Me(S) | single bond | H | *c*-Pr(1-CF₃) | H | Cl | H | O |
| C-11393 | H | single bond | Me | *c*-Pr(1-CF₃) | H | Me | H | O |
| C-11394 | Me | single bond | H | *c*-Pr(1-CF₃) | H | Me | H | O |
| C-11395 | Me(R) | single bond | H | *c*-Pr(1-CF₃) | H | Me | H | O |
| C-11396 | Me(S) | single bond | H | *c*-Pr(1-CF₃) | H | Me | H | O |
| C-11397 | H | single bond | Me | *c*-Pr(1-C≡CH) | H | Cl | H | O |
| C-11398 | Me | single bond | H | *c*-Pr(1-C≡CH) | H | Cl | H | O |
| C-11399 | Me(R) | single bond | H | *c*-Pr(1-C≡CH) | H | Cl | H | O |
| C-11400 | Me(S) | single bond | H | *c*-Pr(1-C≡CH) | H | Cl | H | O |
| C-11401 | H | single bond | Me | *c*-Pr(1-C≡CH) | H | Me | H | O |
| C-11402 | Me | single bond | H | *c*-Pr(1-C≡CH) | H | Me | H | O |
| C-11403 | Me(R) | single bond | H | *c*-Pr(1-C≡CH) | H | Me | H | O |
| C-11404 | Me(S) | single bond | H | *c*-Pr(1-C≡CH) | H | Me | H | O |
| C-11405 | H | single bond | Me | *c*-Pr[1-C(=O)OEt] | H | Cl | H | O |
| C-11406 | Me | single bond | H | *c*-Pr[1-C(=O)OEt] | H | Cl | H | O |
| C-11407 | Me(R) | single bond | H | *c*-Pr[1-C(=O)OEt] | H | Cl | H | O |
| C-11408 | Me(S) | single bond | H | *c*-Pr[1-C(=O)OEt] | H | Cl | H | O |
| C-11409 | H | single bond | Me | *c*-Pr[1-C(=O)OEt] | H | Me | H | O |
| C-11410 | Me | single bond | H | *c*-Pr[1-C(=O)OEt] | H | Me | H | O |
| C-11411 | Me(R) | single bond | H | *c*-Pr[1-C(=O)OEt] | H | Me | H | O |
| C-11412 | Me(S) | single bond | H | *c*-Pr[1-C(=O)OEt] | H | Me | H | O |
| C-11413 | H | single bond | Me | *c*-Pr[1-C(=O)OH] | H | Cl | H | O |
| C-11414 | Me | single bond | H | *c*-Pr[1-C(=O)OH] | H | Cl | H | O |

**[Table 488]**

| Compound Number | Y | W | Z | R¹ | R² | R³ | R¹⁰ | A |
|---|---|---|---|---|---|---|---|---|
| C-11415 | Me(R) | single bond | H | *c* -Pr[1-C(=O)OH] | H | Cl | H | O |
| C-11416 | Me(S) | single bond | H | *c* -Pr[1-C(=O)OH] | H | Cl | H | O |
| C-11417 | H | single bond | Me | *c* -Pr[1-C(=O)OH] | H | Me | H | O |
| C-11418 | Me | single bond | H | *c* -Pr[1-C(=O)OH] | H | Me | H | O |
| C-11419 | Me(R) | single bond | H | *c* -Pr[1-C(=O)OH] | H | Me | H | O |
| C-11420 | Me(S) | single bond | H | *c* -Pr[1-C(=O)OH] | H | Me | H | O |
| C-11421 | H | single bond | Me | *c* -Pr[1-C(=O)NH₂ | H | Cl | H | O |
| C-11422 | Me | single bond | H | *c* -Pr[1-C(=O)NH₂ | H | Cl | H | O |
| C-11423 | Me(R) | single bond | H | *c* -Pr[1-C(=O)NH₂ | H | Cl | H | O |
| C-11424 | Me(S) | single bond | H | *c* -Pr[1-C(=O)NH₂ | H | Cl | H | O |
| C-11425 | H | single bond | Me | *c* -Pr[1-C(=O)NH₂ | H | Me | H | O |
| C-11426 | Me | single bond | H | *c* -Pr[1-C(=O)NH₂ | H | Me | H | O |
| C-11427 | Me(R) | single bond | H | *c* -Pr[1-C(=O)NH₂ | H | Me | H | O |
| C-11428 | Me(S) | single bond | H | *c* -Pr[1-C(=O)NH₂ | H | Me | H | O |
| C-11429 | H | single bond | Me | *c* -Pr(1-OMe) | H | Cl | H | O |
| C-11430 | Me | single bond | H | *c* -Pr(1-OMe) | H | Cl | H | O |
| C-11431 | Me(R) | single bond | H | *c* -Pr(1-OMe) | H | Cl | H | O |
| C-11432 | Me(S) | single bond | H | *c* -Pr(1-OMe) | H | Cl | H | O |
| C-11433 | H | single bond | Me | *c* -Pr(1-OMe) | H | Me | H | O |
| C-11434 | Me | single bond | H | *c* -Pr(1-OMe) | H | Me | H | O |
| C-11435 | Me(R) | single bond | H | *c* -Pr(1-OMe) | H | Me | H | O |
| C-11436 | Me(S) | single bond | H | *c* -Pr(1-OMe) | H | Me | H | O |
| C-11437 | H | single bond | Me | *c* -Pr(1-SMe) | H | Cl | H | O |
| C-11438 | Me | single bond | H | *c* -Pr(1-SMe) | H | Cl | H | O |
| C-11439 | Me(R) | single bond | H | *c* -Pr(1-SMe) | H | Cl | H | O |
| C-11440 | Me(S) | single bond | H | *c* -Pr(1-SMe) | H | Cl | H | O |
| C-11441 | H | single bond | Me | *c* -Pr(1-SMe) | H | Me | H | O |
| C-11442 | Me | single bond | H | *c* -Pr(1-SMe) | H | Me | H | O |
| C-11443 | Me(R) | single bond | H | *c* -Pr(1-SMe) | H | Me | H | O |
| C-11444 | Me(S) | single bond | H | *c* -Pr(1-SMe) | H | Me | H | O |
| C-11445 | H | single bond | Me | *c* -Pr[1-S(=O)Me] | H | Cl | H | O |
| C-11446 | Me | single bond | H | *c* -Pr[1-S(=O)Me] | H | Cl | H | O |
| C-11447 | Me(R) | single bond | H | *c* -Pr[1-S(=O)Me] | H | Cl | H | O |
| C-11448 | Me(S) | single bond | H | *c* -Pr[1-S(=O)Me] | H | Cl | H | O |
| C-11449 | H | single bond | Me | *c* -Pr[1-S(=O)Me] | H | Me | H | O |
| C-11450 | Me | single bond | H | *c* -Pr[1-S(=O)Me] | H | Me | H | O |
| C-11451 | Me(R) | single bond | H | *c* -Pr[1-S(=O)Me] | H | Me | H | O |
| C-11452 | Me(S) | single bond | H | *c* -Pr[1-S(=O)Me] | H | Me | H | O |
| C-11453 | H | single bond | Me | *c* -Pr[1-S(=O)₂Me] | H | Cl | H | O |
| C-11454 | Me | single bond | H | *c* -Pr[1-S(=O)₂Me] | H | Cl | H | O |
| C-11455 | Me(R) | single bond | H | *c* -Pr[1-S(=O)₂Me] | H | Cl | H | O |
| C-11456 | Me(S) | single bond | H | *c* -Pr[1-S(=O)₂Me] | H | Cl | H | O |
| C-11457 | H | single bond | Me | *c* -Pr[1-S(=O)₂Me] | H | Me | H | O |
| C-11458 | Me | single bond | H | *c* -Pr[1-S(=O)₂Me] | H | Me | H | O |

**[Table 489]**

| Compound Number | Y | W | Z | R¹ | R² | R³ | R¹⁰ | A |
|---|---|---|---|---|---|---|---|---|
| C-11459 | Me(R) | single bond | H | *c* -Pr[1-S(=O)₂Me] | H | Me | H | O |
| C-11460 | Me(S) | single bond | H | *c* -Pr[1-S(=O)₂Me] | H | Me | H | O |
| C-11461 | H | single bond | Me | *c*-Pr[1-S(=O)₂CHF₂ | H | Cl | H | O |
| C-11462 | Me | single bond | H | *c*-Pr[1-S(=O)₂CHF₂ | H | Cl | H | O |
| C-11463 | Me(R) | single bond | H | *c*-Pr[1-S(=O)₂CHF₂ | H | Cl | H | O |
| C-11464 | Me(S) | single bond | H | *c*-Pr[1-S(=O)₂CHF₂ | H | Cl | H | O |
| C-11465 | H | single bond | Me | *c*-Pr[1-S(=O)₂CHF₂ | H | Me | H | O |
| C-11466 | Me | single bond | H | *c*-Pr[1-S(=O)₂CHF₂ | H | Me | H | O |
| C-11467 | Me(R) | single bond | H | *c*-Pr[1-S(=O)₂CHF₂ | H | Me | H | O |
| C-11468 | Me(S) | single bond | H | *c*-Pr[1-S(=O)₂CHF₂ | H | Me | H | O |
| C-11469 | H | single bond | Me | *c*-Pr[1-S(=O)₂CF₃] | H | Cl | H | O |
| C-11470 | Me | single bond | H | *c*-Pr[1-S(=O)₂CF₃] | H | Cl | H | O |
| C-11471 | Me(R) | single bond | H | *c*-Pr[1-S(=O)₂CF₃] | H | Cl | H | O |
| C-11472 | Me(S) | single bond | H | *c*-Pr[1-S(=O)₂CF₃] | H | Cl | H | O |
| C-11473 | H | single bond | Me | *c*-Pr[1-S(=O)₂CF₃] | H | Me | H | O |
| C-11474 | Me | single bond | H | *c*-Pr[1-S(=O)₂CF₃] | H | Me | H | O |
| C-11475 | Me(R) | single bond | H | *c*-Pr[1-S(=O)₂CF₃] | H | Me | H | O |
| C-11476 | Me(S) | single bond | H | *c*-Pr[1-S(=O)₂CF₃ | H | Me | H | O |
| C-11477 | H | single bond | Me | *c*-Pr[1-(5,6-dihydro-1,4,2-dioxazin-3-yl)] | H | Cl | H | O |
| C-11478 | Me | single bond | H | *c*-Pr[1-(5,6-dihydro-1,4,2-dioxazin-3-yl)] | H | Cl | H | O |
| C-11479 | Me(R) | single bond | H | *c*-Pr[1-(5,6-dihydro-1,4,2-dioxazin-3-yl)] | H | Cl | H | O |
| C-11480 | Me(S) | single bond | H | *c*-Pr[1-(5,6-dihydro-1,4,2-dioxazin-3-yl)] | H | Cl | H | O |
| C-11481 | H | single bond | Me | *c*-Pr[1-(5,6-dihydro-1,4,2-dioxazin-3-yl)] | H | Me | H | O |
| C-11482 | Me | single bond | H | *c*-Pr[1-(5,6-dihydro-1,4,2-dioxazin-3-yl)] | H | Me | H | O |
| C-11483 | Me(R) | single bond | H | *c*-Pr[1-(5,6-dihydro-1,4,2-dioxazin-3-yl)] | H | Me | H | O |
| C-11484 | Me(S) | single bond | H | *c*-Pr[1-(5,6-dihydro-1,4,2-dioxazin-3-yl)] | H | Me | H | O |
| C-11485 | H | single bond | Me | *c*-Pr[1-(5-hydroxy-1,3,4-oxadiazol-2-yl)] | H | Cl | H | O |
| C-11486 | Me | single bond | H | *c*-Pr[1-(5-hydroxy-1,3,4-oxadiazol-2-yl)] | H | Cl | H | O |
| C-11487 | Me(R) | single bond | H | *c*-Pr[1-(5-hydroxy-1,3,4-oxadiazol-2-yl)] | H | Cl | H | O |
| C-11488 | Me(S) | single bond | H | *c*-Pr[1-(5-hydroxy-1,3,4-oxadiazol-2-yl)] | H | Cl | H | O |
| C-11489 | H | single bond | Me | *c*-Pr[1-(5-hydroxy-1,3,4-oxadiazol-2-yl)] | H | Me | H | O |
| C-11490 | Me | single bond | H | *c*-Pr[1-(5-hydroxy-1,3,4-oxadiazol-2-yl)] | H | Me | H | O |
| C-11491 | Me(R) | single bond | H | *c*-Pr[1-(5-hydroxy-1,3,4-oxadiazol-2-yl)] | H | Me | H | O |
| C-11492 | Me(S) | single bond | H | *c*-Pr[1-(5-hydroxy-1,3,4-oxadiazol-2-yl)] | H | Me | H | O |
| C-11493 | H | single bond | Me | *c* -Pr[1-(pyrimidin-2-yl)] | H | Cl | H | O |
| C-11494 | Me | single bond | H | *c* -Pr[1-(pyrimidin-2-yl)] | H | Cl | H | O |
| C-11495 | Me(R) | single bond | H | *c* -Pr[1-(pyrimidin-2-yl)] | H | Cl | H | O |
| C-11496 | Me(S) | single bond | H | *c* -Pr[1-(pyrimidin-2-yl)] | H | Cl | H | O |
| C-11497 | H | single bond | Me | *c* -Pr[1-(pyrimidin-2-yl)] | H | Me | H | O |
| C-11498 | Me | single bond | H | *c* -Pr[1-(pyrimidin-2-yl)] | H | Me | H | O |
| C-11499 | Me(R) | single bond | H | *c* -Pr[1-(pyrimidin-2-yl)] | H | Me | H | O |
| C-11500 | Me(S) | single bond | H | *c* -Pr[1-(pyrimidin-2-yl)] | H | Me | H | O |
| C-11501 | H | single bond | Me | *t* -Bu | H | Cl | H | O |
| C-11502 | Me | single bond | H | *t* -Bu | H | Cl | H | O |

**[Table 490]**

| Compound Number | Y | W | Z | R¹ | R² | R³ | R¹⁰ | A |
|---|---|---|---|---|---|---|---|---|
| C-11503 | Me(R) | single bond | H | *t* -Bu | H | Cl | H | O |
| C-11504 | Me(S) | single bond | H | t-Bu | H | Cl | H | O |
| C-11505 | H | single bond | Me | *t*-Bu | H | Me | H | O |
| C-11506 | Me | single bond | H | t-Bu | H | Me | H | O |
| C-11507 | Me(R) | single bond | H | t-Bu | H | Me | H | O |
| C-11508 | Me(S) | single bond | H | t-Bu | H | Me | H | O |
| C-11509 | H | single bond | Me | c-Bu | H | Cl | H | O |
| C-11510 | Me | single bond | H | c-Bu | H | Cl | H | O |
| C-11511 | Me(R) | single bond | H | c-Bu | H | Cl | H | O |
| C-11512 | Me(S) | single bond | H | c-Bu | H | Cl | H | O |
| C-11513 | H | single bond | Me | c-Bu | H | Me | H | O |
| C-11514 | Me | single bond | H | c-Bu | H | Me | H | O |
| C-11515 | Me(R) | single bond | H | *c*-Bu | H | Me | H | O |
| C-11516 | Me(S) | single bond | H | c-Bu | H | Me | H | O |
| C-11517 | H | single bond | Me | c-Bu(1-CN) | H | Cl | H | O |
| C-11518 | Me | single bond | H | c-Bu(1-CN) | H | Cl | H | O |
| C-11519 | Me(R) | single bond | H | c-Bu(1-CN) | H | Cl | H | O |
| C-11520 | Me(S) | single bond | H | c-Bu(1-CN) | H | Cl | H | O |
| C-11521 | H | single bond | Me | c-Bu(1-CN) | H | Me | H | O |
| C-11522 | Me | single bond | H | c-Bu(1-CN) | H | Me | H | O |
| C-11523 | Me(R) | single bond | H | c-Bu(1-CN) | H | Me | H | O |
| C-11524 | Me(S) | single bond | H | c-Bu(1-CN) | H | Me | H | O |
| C-11525 | H | single bond | Me | *c* -Bu(3,3-F₂) | H | Cl | H | O |
| C-11526 | Me | single bond | H | *c* -Bu(3,3-F₂) | H | Cl | H | O |
| C-11527 | Me(R) | single bond | H | *c* -Bu(3,3-F₂) | H | Cl | H | O |
| C-11528 | Me(S) | single bond | H | *c* -Bu(3,3-F₂) | H | Cl | H | O |
| C-11529 | H | single bond | Me | *c* -Bu(3,3-F₂) | H | Me | H | O |
| C-11530 | Me | single bond | H | *c* -Bu(3,3-F₂) | H | Me | H | O |
| C-11531 | Me(R) | single bond | H | *c* -Bu(3,3-F₂) | H | Me | H | O |
| C-11532 | Me(S) | single bond | H | *c* -Bu(3,3-F₂) | H | Me | H | O |
| C-11533 | H | single bond | Me | oxetan-3-yl | H | Cl | H | O |
| C-11534 | Me | single bond | H | oxetan-3-yl | H | Cl | H | O |
| C-11535 | Me(R) | single bond | H | oxetan-3-yl | H | Cl | H | O |
| C-11536 | Me(S) | single bond | H | oxetan-3-yl | H | Cl | H | O |
| C-11537 | H | single bond | Me | oxetan-3-yl | H | Me | H | O |
| C-11538 | Me | single bond | H | oxetan-3-yl | H | Me | H | O |
| C-11539 | Me(R) | single bond | H | oxetan-3-yl | H | Me | H | O |
| C-11540 | Me(S) | single bond | H | oxetan-3-yl | H | Me | H | O |
| C-11541 | H | single bond | Me | oxetan(3-CN)-3-yl | H | Cl | H | O |
| C-11542 | Me | single bond | H | oxetan(3-CN)-3-yl | H | Cl | H | O |
| C-11543 | Me(R) | single bond | H | oxetan(3-CN)-3-yl | H | Cl | H | O |
| C-11544 | Me(S) | single bond | H | oxetan(3-CN)-3-yl | H | Cl | H | O |
| C-11545 | H | single bond | Me | oxetan(3-CN)-3-yl | H | Me | H | O |
| C-11546 | Me | single bond | H | oxetan(3-CN)-3-yl | H | Me | H | O |

**[Table 491]**

| Compound Number | Y | W | Z | R¹ | R² | R³ | R¹⁰ | A |
|---|---|---|---|---|---|---|---|---|
| C-11547 | Me(R) | single bond | H | oxetan(3-CN)-3-yl | H | Me | H | O |
| C-11548 | Me(S) | single bond | H | oxetan(3-CN)-3-yl | H | Me | H | O |
| C-11549 | H | single bond | Me | azetidin[1-C(=O)Me]-3-yl | H | Cl | H | O |
| C-11550 | Me | single bond | H | azetidin[1-C(=O)Me]-3-yl | H | Cl | H | O |
| C-11551 | Me(R) | single bond | H | azetidin[1-C(=O)Me]-3-yl | H | Cl | H | O |
| C-11552 | Me(S) | single bond | H | azetidin[1-C(=O)Me]-3-yl | H | Cl | H | O |
| C-11553 | H | single bond | Me | azetidin[1-C(=O)Me]-3-yl | H | Me | H | O |
| C-11554 | Me | single bond | H | azetidin[1-C(=O)Me]-3-yl | H | Me | H | O |
| C-11555 | Me(R) | single bond | H | azetidin[1-C(=O)Me]-3-yl | H | Me | H | O |
| C-11556 | Me(S) | single bond | H | azetidin[1-C(=O)Me]-3-yl | H | Me | H | O |
| C-11557 | H | single bond | Me | azetidin[1-S(=O)₂Me]-3-yl | H | Cl | H | O |
| C-11558 | Me | single bond | H | azetidin[1-S(=O)₂Me]-3-yl | H | Cl | H | O |
| C-11559 | Me(R) | single bond | H | azetidin[1-S(=O)₂Me]-3-yl | H | Cl | H | O |
| C-11560 | Me(S) | single bond | H | azetidin[1-S(=O)₂Me]-3-yl | H | Cl | H | O |
| C-11561 | H | single bond | Me | azetidin[1-S(=O)₂Me]-3-yl | H | Me | H | O |
| C-11562 | Me | single bond | H | azetidin[1-S(=O)₂Me]-3-yl | H | Me | H | O |
| C-11563 | Me(R) | single bond | H | azetidin[1-S(=O)₂Me]-3-yl | H | Me | H | O |
| C-11564 | Me(S) | single bond | H | azetidin[1-S(=O)₂Me]-3-yl | H | Me | H | O |
| C-11565 | H | single bond | Me | azetidin[1-S(=O)₂CHF₂]-3-yl | H | Cl | H | O |
| C-11566 | Me | single bond | H | azetidin[1-S(=O)₂CHF₂]-3-yl | H | Cl | H | O |
| C-11567 | Me(R) | single bond | H | azetidin[1-S(=O)₂CHF₂]-3-yl | H | Cl | H | O |
| C-11568 | Me(S) | single bond | H | azetidin{1-S(=O)₂CHF₂]-3-yl | H | Cl | H | O |
| C-11569 | H | single bond | Me | azetidin[1-S(=O)₂CHF₂]-3-yl | H | Me | H | O |
| C-11570 | Me | single bond | H | azetidin[1-S(=O)₂CHF₂]-3-yl | H | Me | H | O |
| C-11571 | Me(R) | single bond | H | azetidin[1-S(=O)₂CHF₂]-3-yl | H | Me | H | O |
| C-11572 | Me(S) | single bond | H | azetidin[1-S(=O)₂CHF₂]-3-yl | H | Me | H | O |
| C-11573 | H | single bond | Me | c-Pen | H | Cl | H | O |
| C-11574 | Me | single bond | H | c-Pen | H | Cl | H | O |
| C-11575 | Me(R) | single bond | H | *c* -Pen | H | Cl | H | O |
| C-11576 | Me(S) | single bond | H | c-Pen | H | Cl | H | O |
| C-11577 | H | single bond | Me | c-Pen | H | Me | H | O |
| C-11578 | Me | single bond | H | c-Pen | H | Me | H | O |
| C-11579 | Me(R) | single bond | H | c-Pen | H | Me | H | O |
| C-11580 | Me(S) | single bond | H | c-Pen | H | Me | H | O |
| C-11581 | H | single bond | Me | tetrahydrofuran-3-yl | H | Cl | H | O |
| C-11582 | Me | single bond | H | tetra hydrofuran-3-yl | H | Cl | H | O |
| C-11583 | Me(R) | single bond | H | tetrahydrofuran-3-yl | H | Cl | H | O |
| C-11584 | Me(S) | single bond | H | tetrahydrofuran-3-yl | H | Cl | H | O |
| C-11585 | H | single bond | Me | tetrahydrofuran-3-yl | H | Me | H | O |
| C-11586 | Me | single bond | H | tetrahydrofuran-3-yl | H | Me | H | O |
| C-11587 | Me(R) | single bond | H | tetra hydrofuran-3-yl | H | Me | H | O |
| C-11588 | Me(S) | single bond | H | tetrahydrofuran-3-yl | H | Me | H | O |
| C-11589 | H | single bond | Me | tetrahydrothiophen-3-yl | H | Cl | H | O |
| C-11590 | Me | single bond | H | tetrahydrothiophen-3-yl | H | Cl | H | O |

**[Table 492]**

| Compound Number | Y | W | Z | R¹ | R² | R³ | R¹⁰ | A |
|---|---|---|---|---|---|---|---|---|
| C-11591 | Me(R) | single bond | H | tetrahydrothiophen-3-yl | H | Cl | H | O |
| C-11592 | Me(S) | single bond | H | tetrahydrothiophen-3-yl | H | Cl | H | O |
| C-11593 | H | single bond | Me | tetrahydrothiophen-3-yl | H | Me | H | O |
| C-11594 | Me | single bond | H | tetrahydrothiophen-3-yl | H | Me | H | O |
| C-11595 | Me(R) | single bond | H | tetrahydrothiophen-3-yl | H | Me | H | O |
| C-11596 | Me(S) | single bond | H | tetrahydrothiophen-3-yl | H | Me | H | O |
| C-11597 | H | single bond | Me | c -Pen(1-CN) | H | Cl | H | O |
| C-11598 | Me | single bond | H | c-Pen(1-CN) | H | Cl | H | O |
| C-11599 | Me(R) | single bond | H | c-Pen(1-CN) | H | Cl | H | O |
| C-11600 | Me(S) | single bond | H | *c* -Pen(1-CN) | H | Cl | H | O |
| C-11601 | H | single bond | Me | c-Pen(1-CN) | H | Me | H | O |
| C-11602 | Me | single bond | H | c-Pen(1-CN) | H | Me | H | O |
| C-11603 | Me(R) | single bond | H | c -Pen(1-CN) | H | Me | H | O |
| C-11604 | Me(S) | single bond | H | c-Pen(1-CN) | H | Me | H | O |
| C-11605 | H | single bond | Me | tetrahydrofuran(3-CN)-3-yl | H | Cl | H | O |
| C-11606 | Me | single bond | H | tetrahydrofuran(3-CN)-3-yl | H | Cl | H | O |
| C-11607 | Me(R) | single bond | H | tetrahydrofuran(3-CN)-3-yl | H | Cl | H | O |
| C-11608 | Me(S) | single bond | H | tetrahydrofuran(3-CN)-3-yl | H | Cl | H | O |
| C-11609 | H | single bond | Me | tetrahydrofuran(3-CN)-3-yl | H | Me | H | O |
| C-11610 | Me | single bond | H | tetrahydrofuran(3-CN)-3-yl | H | Me | H | O |
| C-11611 | Me(R) | single bond | H | tetrahydrofuran(3-CN)-3-yl | H | Me | H | O |
| C-11612 | Me(S) | single bond | H | tetrahydrofuran(3-CN)-3-yl | H | Me | H | O |
| C-11613 | H | single bond | Me | tetrahydrothiophen(3-CN)-3-yl | H | Cl | H | O |
| C-11614 | Me | single bond | H | tetrahydrothiophen(3-CN)-3-yl | H | Cl | H | O |
| C-11615 | Me(R) | single bond | H | tetrahydrothiophen(3-CN)-3-yl | H | Cl | H | O |
| C-11616 | Me(S) | single bond | H | tetrahydrothiophen(3-CN)-3-yl | H | Cl | H | O |
| C-11617 | H | single bond | Me | tetrahydrothiophen(3-CN)-3-yl | H | Me | H | O |
| C-11618 | Me | single bond | H | tetrahydrothiophen(3-CN)-3-yl | H | Me | H | O |
| C-11619 | Me(R) | single bond | H | tetrahydrothiophen(3-CN)-3-yl | H | Me | H | O |
| C-11620 | Me(S) | single bond | H | tetrahydrothiophen(3-CN)-3-yl | H | Me | H | O |
| C-11621 | H | single bond | Me | bicyclo[1.1.1]pentan-1-yl | H | Cl | H | O |
| C-11622 | Me | single bond | H | bicyclo[1.1.1]pentan-1-yl | H | Cl | H | O |
| C-11623 | Me(R) | single bond | H | bicyclo[1.1.1]pentan-1-yl | H | Cl | H | O |
| C-11624 | Me(S) | single bond | H | bicyclo[1.1.1]pentan-1-yl | H | Cl | H | O |
| C-11625 | H | single bond | Me | bicyclo[1.1.1]pentan-1-yl | H | Me | H | O |
| C-11626 | Me | single bond | H | bicyclo[1.1.1]pentan-1-yl | H | Me | H | O |
| C-11627 | Me(R) | single bond | H | bicyclo[1.1.1]pentan-1-yl | H | Me | H | O |
| C-11628 | Me(S) | single bond | H | bicyclo[1.1.1]pentan-1-yl | H | Me | H | O |
| C-11629 | H | single bond | Me | *c* -Hex | H | Cl | H | O |
| C-11630 | Me | single bond | H | *c* -Hex | H | Cl | H | O |
| C-11631 | Me(R) | single bond | H | *c* -Hex | H | Cl | H | O |
| C-11632 | Me(S) | single bond | H | c-Hex | H | Cl | H | O |
| C-11633 | H | single bond | Me | c-Hex | H | Me | H | O |
| C-11634 | Me | single bond | H | *c* -Hex | H | Me | H | O |

**[Table 493]**

| Compound Number | Y | W | Z | R¹ | R² | R³ | R¹⁰ | A |
|---|---|---|---|---|---|---|---|---|
| C-11635 | Me(R) | single bond | H | *c* -Hex | H | Me | H | O |
| C-11636 | Me(S) | single bond | H | *c* -Hex | H | Me | H | O |
| C-11637 | H | single bond | Me | tetrahydropyran-4-yl | H | Cl | H | O |
| C-11638 | Me | single bond | H | tetrahydropyran-4-yl | H | Cl | H | O |
| C-11639 | Me(R) | single bond | H | tetrahydropyran-4-yl | H | Cl | H | O |
| C-11640 | Me(S) | single bond | H | tetrahydropyran-4-yl | H | Cl | H | O |
| C-11641 | H | single bond | Me | tetrahydropyran-4-yl | H | Me | H | O |
| C-11642 | Me | single bond | H | tetrahydropyran-4-yl | H | Me | H | O |
| C-11643 | Me(R) | single bond | H | tetrahydropyran-4-yl | H | Me | H | O |
| C-11644 | Me(S) | single bond | H | tetrahydropyran-4-yl | H | Me | H | O |
| C-11645 | H | single bond | Me | tetrahydrothiopyran-4-yl | H | Cl | H | O |
| C-11646 | Me | single bond | H | tetrahydrothiopyran-4-yl | H | Cl | H | O |
| C-11647 | Me(R) | single bond | H | tetrahydrothiopyran-4-yl | H | Cl | H | O |
| C-11648 | Me(S) | single bond | H | tetrahydrothiopyran-4-yl | H | Cl | H | O |
| C-11649 | H | single bond | Me | tetrahydrothiopyran-4-yl | H | Me | H | O |
| C-11650 | Me | single bond | H | tetrahydrothiopyran-4-yl | H | Me | H | O |
| C-11651 | Me(R) | single bond | H | tetrahydrothiopyran-4-yl | H | Me | H | O |
| C-11652 | Me(S) | single bond | H | tetrahydrothiopyran-4-yl | H | Me | H | O |
| C-11653 | H | single bond | Me | *c* -Hex(1-CN) | H | Cl | H | O |
| C-11654 | Me | single bond | H | *c* -Hex(1-CN) | H | Cl | H | O |
| C-11655 | Me(R) | single bond | H | *c* -Hex(1-CN) | H | Cl | H | O |
| C-11656 | Me(S) | single bond | H | *c* -Hex(1-CN) | H | Cl | H | O |
| C-11657 | H | single bond | Me | *c* -Hex(1-CN) | H | Me | H | O |
| C-11658 | Me | single bond | H | *c* -Hex(1-CN) | H | Me | H | O |
| C-11659 | Me(R) | single bond | H | *c* -Hex(1-CN) | H | Me | H | O |
| C-11660 | Me(S) | single bond | H | *c* -Hex(1-CN) | H | Me | H | O |
| C-11661 | H | single bond | Me | tetrahydropyran(4-CN)-4-yl | H | Cl | H | O |
| C-11662 | Me | single bond | H | tetrahydropyran(4-CN)-4-yl | H | Cl | H | O |
| C-11663 | Me(R) | single bond | H | tetrahydropyran(4-CN)-4-yl | H | Cl | H | O |
| C-11664 | Me(S) | single bond | H | tetrahydropyran(4-CN)-4-yl | H | Cl | H | O |
| C-11665 | H | single bond | Me | tetrahydropyran(4-CN)-4-yl | H | Me | H | O |
| C-11666 | Me | single bond | H | tetrahydropyran(4-CN)-4-yl | H | Me | H | O |
| C-11667 | Me(R) | single bond | H | tetrahydropyran(4-CN)-4-yl | H | Me | H | O |
| C-11668 | Me(S) | single bond | H | tetrahydropyran(4-CN)-4-yl | H | Me | H | O |
| C-11669 | H | single bond | Me | tetrahydrothiopyran(4-CN)-4-yl | H | Cl | H | O |
| C-11670 | Me | single bond | H | tetrahydrothiopyran(4-CN)-4-yl | H | Cl | H | O |
| C-11671 | Me(R) | single bond | H | tetrahydrothiopyran(4-CN)-4-yl | H | Cl | H | O |
| C-11672 | Me(S) | single bond | H | tetrahydrothiopyran(4-CN)-4-yl | H | Cl | H | O |
| C-11673 | H | single bond | Me | tetrahydrothiopyran(4-CN)-4-yl | H | Me | H | O |
| C-11674 | Me | single bond | H | tetrahydrothiopyran(4-CN)-4-yl | H | Me | H | O |
| C-11675 | Me(R) | single bond | H | tetrahydrothiopyran(4-CN)-4-yl | H | Me | H | O |
| C-11676 | Me(S) | single bond | H | tetrahydrothiopyran(4-CN)-4-yl | H | Me | H | O |
| C-11677 | H | single bond | Me | CH₂(*c* -Pr) | H | Cl | H | O |
| C-11678 | Me | single bond | H | CH₂(*c* -Pr) | H | Cl | H | O |

**[Table 494]**

| Compound Number | Y | W | Z | R¹ | R² | R³ | R¹⁰ | A |
|---|---|---|---|---|---|---|---|---|
| C-11679 | Me(R) | single bond | H | CH₂(*c* -Pr) | H | Cl | H | O |
| C-11680 | Me(S) | single bond | H | CH₂(*c* -Pr) | H | Cl | H | O |
| C-11681 | H | single bond | Me | CH₂(*c* -Pr) | H | Me | H | O |
| C-11682 | Me | single bond | H | CH₂(*c* -Pr) | H | Me | H | O |
| C-11683 | Me(R) | single bond | H | CH₂(*c* -Pr) | H | Me | H | O |
| C-11684 | Me(S) | single bond | H | CH₂(*c* -Pr) | H | Me | H | O |
| C-11685 | H | single bond | Me | CH₂[*c* -Pr(1-CN)] | H | Cl | H | O |
| C-11686 | Me | single bond | H | CH₂[*c* -Pr(1-CN)] | H | Cl | H | O |
| C-11687 | Me(R) | single bond | H | CH₂[*c* -Pr(1-CN)] | H | Cl | H | O |
| C-11688 | Me(S) | single bond | H | CH₂[*c* -Pr(1-CN)] | H | Cl | H | O |
| C-11689 | H | single bond | Me | CH₂[*c* -Pr(1-CN)] | H | Me | H | O |
| C-11690 | Me | single bond | H | CH₂[*c* -Pr(1-CN)] | H | Me | H | O |
| C-11691 | Me(R) | single bond | H | CH₂[*c* -Pr(1-CN)] | H | Me | H | O |
| C-11692 | Me(S) | single bond | H | CH₂[*c* -Pr(1-CN)] | H | Me | H | O |
| C-11693 | H | single bond | Me | CH(Me)(*c* -Pr) | H | Cl | H | O |
| C-11694 | Me | single bond | H | CH(Me)(*c* -Pr) | H | Cl | H | O |
| C-11695 | Me(R) | single bond | H | CH(Me)(*c* -Pr) | H | Cl | H | O |
| C-11696 | Me(S) | single bond | H | CH(Me)(*c* -Pr) | H | Cl | H | O |
| C-11697 | H | single bond | Me | CH(Me)(*c* -Pr) | H | Me | H | O |
| C-11698 | Me | single bond | H | CH(Me)(*c* -Pr) | H | Me | H | O |
| C-11699 | Me(R) | single bond | H | CH(Me)(*c* -Pr) | H | Me | H | O |
| C-11700 | Me(S) | single bond | H | CH(Me)(*c* -Pr) | H | Me | H | O |
| C-11701 | H | single bond | Me | CH(Me)CH₂OMe | H | Cl | H | O |
| C-11702 | Me | single bond | H | CH(Me)CH₂OMe | H | Cl | H | O |
| C-11703 | Me(R) | single bond | H | CH(Me)CH₂OMe | H | Cl | H | O |
| C-11704 | Me(S) | single bond | H | CH(Me)CH₂OMe | H | Cl | H | O |
| C-11705 | H | single bond | Me | CH(Me)CH₂OMe | H | Me | H | O |
| C-11706 | Me | single bond | H | CH(Me)CH₂OMe | H | Me | H | O |
| C-11707 | Me(R) | single bond | H | CH(Me)CH₂OMe | H | Me | H | O |
| C-11708 | Me(S) | single bond | H | CH(Me)CH₂OMe | H | Me | H | O |
| C-11709 | H | single bond | Me | CH₂CH=CH₂ | H | Cl | H | O |
| C-11710 | Me | single bond | H | CH₂CH=CH₂ | H | Cl | H | O |
| C-11711 | Me(R) | single bond | H | CH₂CH=CH₂ | H | Cl | H | O |
| C-11712 | Me(S) | single bond | H | CH₂CH=CH₂ | H | Cl | H | O |
| C-11713 | H | single bond | Me | CH₂CH=CH₂ | H | Me | H | O |
| C-11714 | Me | single bond | H | CH₂CH=CH₂ | H | Me | H | O |
| C-11715 | Me(R) | single bond | H | CH₂CH=CH₂ | H | Me | H | O |
| C-11716 | Me(S) | single bond | H | CH₂CH=CH₂ | H | Me | H | O |
| C-11717 | H | single bond | Me | CH₂C≡CH | H | Cl | H | O |
| C-11718 | Me | single bond | H | CH₂C≡CH | H | Cl | H | O |
| C-11719 | Me(R) | single bond | H | CH₂C≡CH | H | Cl | H | O |
| C-11720 | Me(S) | single bond | H | CH₂C≡CH | H | Cl | H | O |
| C-11721 | H | single bond | Me | CH₂C≡CH | H | Me | H | O |
| C-11722 | Me | single bond | H | CH₂C≡CH | H | Me | H | O |

**[Table 495]**

| Compound Number | Y | W | Z | R¹ | R² | R³ | R¹⁰ | A |
|---|---|---|---|---|---|---|---|---|
| C-11723 | Me(R) | single bond | H | CH₂C≡CH | H | Me | H | O |
| C-11724 | Me(S) | single bond | H | CH₂C≡CH | H | Me | H | O |
| C-11725 | H | single bond | Me | CH₂CN | H | Cl | H | O |
| C-11726 | Me | single bond | H | CH₂CN | H | Cl | H | O |
| C-11727 | Me(R) | single bond | H | CH₂CN | H | Cl | H | O |
| C-11728 | Me(S) | single bond | H | CH₂CN | H | Cl | H | O |
| C-11729 | H | single bond | Me | CH₂CN | H | Me | H | O |
| C-11730 | Me | single bond | H | CH₂CN | H | Me | H | O |
| C-11731 | Me(R) | single bond | H | CH₂CN | H | Me | H | O |
| C-11732 | Me(S) | single bond | H | CH₂CN | H | Me | H | O |
| C-11733 | H | single bond | Me | CH(Me)CN | H | Cl | H | O |
| C-11734 | Me | single bond | H | CH(Me)CN | H | Cl | H | O |
| C-11735 | Me(R) | single bond | H | CH(Me)CN | H | Cl | H | O |
| C-11736 | Me(S) | single bond | H | CH(Me)CN | H | Cl | H | O |
| C-11737 | H | single bond | Me | CH(Me)CN | H | Me | H | O |
| C-11738 | Me | single bond | H | CH(Me)CN | H | Me | H | O |
| C-11739 | Me(R) | single bond | H | CH(Me)CN | H | Me | H | O |
| C-11740 | Me(S) | single bond | H | CH(Me)CN | H | Me | H | O |
| C-11741 | H | single bond | Me | C(Me)₂CN | H | Cl | H | O |
| C-11742 | Me | single bond | H | C(Me)₂CN | H | Cl | H | O |
| C-11743 | Me(R) | single bond | H | C(Me)₂CN | H | Cl | H | O |
| C-11744 | Me(S) | single bond | H | C(Me)₂CN | H | Cl | H | O |
| C-11745 | H | single bond | Me | C(Me)₂CN | H | Me | H | O |
| C-11746 | Me | single bond | H | C(Me)₂CN | H | Me | H | O |
| C-11747 | Me(R) | single bond | H | C(Me)₂CN | H | Me | H | O |
| C-11748 | Me(S) | single bond | H | C(Me)₂CN | H | Me | H | O |
| C-11749 | H | single bond | Me | C(Me)(*i* -Pr)CN | H | Cl | H | O |
| C-11750 | Me | single bond | H | C(Me)(*i* -Pr)CN | H | Cl | H | O |
| C-11751 | Me(R) | single bond | H | C(Me)(*i* -Pr)CN | H | Cl | H | O |
| C-11752 | Me(S) | single bond | H | C(Me)(*i* -Pr)CN | H | Cl | H | O |
| C-11753 | H | single bond | Me | C(Me)(*i* -Pr)CN | H | Me | H | O |
| C-11754 | Me | single bond | H | C(Me)(*i* -Pr)CN | H | Me | H | O |
| C-11755 | Me(R) | single bond | H | C(Me)(*i* -Pr)CN | H | Me | H | O |
| C-11756 | Me(S) | single bond | H | C(Me)(*i* -Pr)CN | H | Me | H | O |
| C-11757 | H | single bond | Me | OMe | H | Cl | H | O |
| C-11758 | Me | single bond | H | OMe | H | Cl | H | O |
| C-11759 | Me(R) | single bond | H | OMe | H | Cl | H | O |
| C-11760 | Me(S) | single bond | H | OMe | H | Cl | H | O |
| C-11761 | H | single bond | Me | OMe | H | Me | H | O |
| C-11762 | Me | single bond | H | OMe | H | Me | H | O |
| C-11763 | Me(R) | single bond | H | OMe | H | Me | H | O |
| C-11764 | Me(S) | single bond | H | OMe | H | Me | H | O |
| C-11765 | H | single bond | Me | CH=NOMe | H | Cl | H | O |
| C-11766 | Me | single bond | H | CH=NOMe | H | Cl | H | O |

**[Table 496]**

| Compound Number | Y | W | Z | R¹ | R² | R³ | R¹⁰ | A |
|---|---|---|---|---|---|---|---|---|
| C-11767 | Me(R) | single bond | H | CH=NOMe | H | Cl | H | O |
| C-11768 | Me(S) | single bond | H | CH=NOMe | H | Cl | H | O |
| C-11769 | H | single bond | Me | CH=NOMe | H | Me | H | O |
| C-11770 | Me | single bond | H | CH=NOMe | H | Me | H | O |
| C-11771 | Me(R) | single bond | H | CH=NOMe | H | Me | H | O |
| C-11772 | Me(S) | single bond | H | CH=NOMe | H | Me | H | O |
| C-11773 | H | single bond | Me | morpholin-4-yl | H | Cl | H | O |
| C-11774 | Me | single bond | H | morpholin-4-yl | H | Cl | H | O |
| C-11775 | Me(R) | single bond | H | morpholin-4-yl | H | Cl | H | O |
| C-11776 | Me(S) | single bond | H | morpholin-4-yl | H | Cl | H | O |
| C-11777 | H | single bond | Me | morpholin-4-yl | H | Me | H | O |
| C-11778 | Me | single bond | H | morpholin-4-yl | H | Me | H | O |
| C-11779 | Me(R) | single bond | H | morpholin-4-yl | H | Me | H | O |
| C-11780 | Me(S) | single bond | H | morpholin-4-yl | H | Me | H | O |
| C-11781 | H | single bond | Me | Ph | H | Cl | H | O |
| C-11782 | Me | single bond | H | Ph | H | Cl | H | O |
| C-11783 | Me(R) | single bond | H | Ph | H | Cl | H | O |
| C-11784 | Me(S) | single bond | H | Ph | H | Cl | H | O |
| C-11785 | H | single bond | Me | Ph | H | Me | H | O |
| C-11786 | Me | single bond | H | Ph | H | Me | H | O |
| C-11787 | Me(R) | single bond | H | Ph | H | Me | H | O |
| C-11788 | Me(S) | single bond | H | Ph | H | Me | H | O |
| C-11789 | H | single bond | Me | CH₂Ph | H | Cl | H | O |
| C-11790 | Me | single bond | H | CH₂Ph | H | Cl | H | O |
| C-11791 | Me(R) | single bond | H | CH₂Ph | H | Cl | H | O |
| C-11792 | Me(S) | single bond | H | CH₂Ph | H | Cl | H | O |
| C-11793 | H | single bond | Me | CH₂Ph | H | Me | H | O |
| C-11794 | Me | single bond | H | CH₂Ph | H | Me | H | O |
| C-11795 | Me(R) | single bond | H | CH₂Ph | H | Me | H | O |
| C-11796 | Me(S) | single bond | H | CH₂Ph | H | Me | H | O |
| C-11797 | H | single bond | Me | thiazole-2-yl | H | Cl | H | O |
| C-11798 | Me | single bond | H | thiazole-2-yl | H | Cl | H | O |
| C-11799 | Me(R) | single bond | H | thiazole-2-yl | H | Cl | H | O |
| C-11800 | Me(S) | single bond | H | thiazole-2-yl | H | Cl | H | O |
| C-11801 | H | single bond | Me | thiazole-2-yl | H | Me | H | O |
| C-11802 | Me | single bond | H | thiazole-2-yl | H | Me | H | O |
| C-11803 | Me(R) | single bond | H | thiazole-2-yl | H | Me | H | O |
| C-11804 | Me(S) | single bond | H | thiazole-2-yl | H | Me | H | O |
| C-11805 | H | single bond | Me | 1-Me-pyrazol-3-yl | H | Cl | H | O |
| C-11806 | Me | single bond | H | 1-Me-pyrazol-3-yl | H | Cl | H | O |
| C-11807 | Me(R) | single bond | H | 1-Me-pyrazol-3-yl | H | Cl | H | O |
| C-11808 | Me(S) | single bond | H | 1-Me-pyrazol-3-yl | H | Cl | H | O |
| C-11809 | H | single bond | Me | 1-Me-pyrazol-3-yl | H | Me | H | O |
| C-11810 | Me | single bond | H | 1-Me-pyrazol-3-yl | H | Me | H | O |

**[Table 497]**

| Compound Number | Y | W | Z | R¹ | R² | R³ | R¹⁰ | A |
|---|---|---|---|---|---|---|---|---|
| C-11811 | Me(R) | single bond | H | 1-Me-pyrazol-3-yl | H | Me | H | O |
| C-11812 | Me(S) | single bond | H | 1-Me-pyrazol-3-yl | H | Me | H | O |
| C-11813 | H | single bond | Me | 1-Me-pyrazol-5-yl | H | Cl | H | O |
| C-11814 | Me | single bond | H | 1-Me-pyrazol-5-yl | H | Cl | H | O |
| C-11815 | Me(R) | single bond | H | 1-Me-pyrazol-5-yl | H | Cl | H | O |
| C-11816 | Me(S) | single bond | H | 1-Me-pyrazol-5-yl | H | Cl | H | O |
| C-11817 | H | single bond | Me | 1-Me-pyrazol-5-yl | H | Me | H | O |
| C-11818 | Me | single bond | H | 1-Me-pyrazol-5-yl | H | Me | H | O |
| C-11819 | Me(R) | single bond | H | 1-Me-pyrazol-5-yl | H | Me | H | O |
| C-11820 | Me(S) | single bond | H | 1-Me-pyrazol-5-yl | H | Me | H | O |
| C-11821 | H | single bond | Me | CH₂(pyrimidin-2-yl) | H | Cl | H | O |
| C-11822 | Me | single bond | H | CH₂(pyrimidin-2-yl) | H | Cl | H | O |
| C-11823 | Me(R) | single bond | H | CH₂(pyrimidin-2-yl) | H | Cl | H | O |
| C-11824 | Me(S) | single bond | H | CH₂(pyrimidin-2-yl) | H | Cl | H | O |
| C-11825 | H | single bond | Me | CH₂(pyrimidin-2-yl) | H | Me | H | O |
| C-11826 | Me | single bond | H | CH₂(pyrimidin-2-yl) | H | Me | H | O |
| C-11827 | Me(R) | single bond | H | CH₂(pyrimidin-2-yl) | H | Me | H | O |
| C-11828 | Me(S) | single bond | H | CH₂(pyrimidin-2-yl) | H | Me | H | O |
| C-11829 | H | single bond | Me | -CH₂CH₂CH₂- | | Cl | H | O |
| C-11830 | Me | single bond | H | -CH₂CH₂CH₂- | | Cl | H | O |
| C-11831 | Me(R) | single bond | H | -CH₂CH₂CH₂- | | Cl | H | O |
| C-11832 | Me(S) | single bond | H | -CH₂CH₂CH₂- | | Cl | H | O |
| C-11833 | H | single bond | Me | -CH₂CH₂CH₂- | | Me | H | O |
| C-11834 | Me | single bond | H | -CH₂CH₂CH₂- | | Me | H | O |
| C-11835 | Me(R) | single bond | H | -CH₂CH₂CH₂- | | Me | H | O |
| C-11836 | Me(S) | single bond | H | -CH₂CH₂CH₂- | | Me | H | O |
| C-11837 | H | single bond | Me | -CH₂CH₂OCH₂CH₂- | | Cl | H | O |
| C-11838 | Me | single bond | H | -CH₂CH₂OCH₂CH₂- | | Cl | H | O |
| C-11839 | Me(R) | single bond | H | -CH₂CH₂OCH₂CH₂- | | Cl | H | O |
| C-11840 | Me(S) | single bond | H | -CH₂CH₂OCH₂CH₂- | | Cl | H | O |
| C-11841 | H | single bond | Me | -CH₂CH₂OCH₂CH₂- | | Me | H | O |
| C-11842 | Me | single bond | H | -CH₂CH₂OCH₂CH₂- | | Me | H | O |
| C-11843 | Me(R) | single bond | H | -CH₂CH₂OCH₂CH₂- | | Me | H | O |
| C-11844 | Me(S) | single bond | H | -CH₂CH₂OCH₂CH₂- | | Me | H | O |
| C-11845 | H | single bond | Me | Me | H | Cl | Me | O |
| C-11846 | Me | single bond | H | Me | H | Cl | Me | O |
| C-11847 | Me(R) | single bond | H | Me | H | Cl | Me | O |
| C-11848 | Me(S) | single bond | H | Me | H | Cl | Me | O |
| C-11849 | H | single bond | Me | Me | H | Me | Me | O |
| C-11850 | Me | single bond | H | Me | H | Me | Me | O |
| C-11851 | Me(R) | single bond | H | Me | H | Me | Me | O |
| C-11852 | Me(S) | single bond | H | Me | H | Me | Me | O |
| C-11853 | H | single bond | Me | CH₂CHF₂ | H | Cl | Me | O |
| C-11854 | Me | single bond | H | CH₂CHF₂ | H | Cl | Me | O |

**[Table 498]**

| Compound Number | Y | W | Z | R¹ | R² | R³ | R¹⁰ | A |
|---|---|---|---|---|---|---|---|---|
| C-11855 | Me(R) | single bond | H | CH₂CHF₂ | H | Cl | Me | O |
| C-11856 | Me(S) | single bond | H | CH₂CHF₂ | H | Cl | Me | O |
| C-11857 | H | single bond | Me | CH₂CHF₂ | H | Me | Me | O |
| C-11858 | Me | single bond | H | CH₂CHF₂ | H | Me | Me | O |
| C-11859 | Me(R) | single bond | H | CH₂CHF₂ | H | Me | Me | O |
| C-11860 | Me(S) | single bond | H | CH₂CHF₂ | H | Me | Me | O |
| C-11861 | H | single bond | Me | *n*-Pr | H | Cl | Me | O |
| C-11862 | Me | single bond | H | *n*-Pr | H | Cl | Me | O |
| C-11863 | Me(R) | single bond | H | *n*-Pr | H | Cl | Me | O |
| C-11864 | Me(S) | single bond | H | *n*-Pr | H | Cl | Me | O |
| C-11865 | H | single bond | Me | *n*-Pr | H | Me | Me | O |
| C-11866 | Me | single bond | H | *n*-Pr | H | Me | Me | O |
| C-11867 | Me(R) | single bond | H | *n*-Pr | H | Me | Me | O |
| C-11868 | Me(S) | single bond | H | *n*-Pr | H | Me | Me | O |
| C-11869 | H | single bond | Me | *i*-Pr | H | Cl | Me | O |
| C-11870 | Me | single bond | H | *i*-Pr | H | Cl | Me | O |
| C-11871 | Me(R) | single bond | H | *i*-Pr | H | Cl | Me | O |
| C-11872 | Me(S) | single bond | H | i-Pr | H | Cl | Me | O |
| C-11873 | H | single bond | Me | *i*-Pr | H | Me | Me | O |
| C-11874 | Me | single bond | H | *i*-Pr | H | Me | Me | O |
| C-11875 | Me(R) | single bond | H | *i*-Pr | H | Me | Me | O |
| C-11876 | Me(S) | single bond | H | *i*-Pr | H | Me | Me | O |
| C-11877 | H | single bond | Me | *c*-Pr(1-F) | H | Cl | Me | O |
| C-11878 | Me | single bond | H | *c*-Pr(1-F) | H | Cl | Me | O |
| C-11879 | Me(R) | single bond | H | *c*-Pr(1-F) | H | Cl | Me | O |
| C-11880 | Me(S) | single bond | H | *c*-Pr(1-F) | H | Cl | Me | O |
| C-11881 | H | single bond | Me | *c*-Pr(1-F) | H | Me | Me | O |
| C-11882 | Me | single bond | H | *c*-Pr(1-F) | H | Me | Me | O |
| C-11883 | Me(R) | single bond | H | *c*-Pr(1-F) | H | Me | Me | O |
| C-11884 | Me(S) | single bond | H | *c*-Pr(1-F) | H | Me | Me | O |
| C-11885 | H | single bond | Me | *c*-Pr(2,2-F₂) | H | Cl | Me | O |
| C-11886 | Me | single bond | H | *c*-Pr(2,2-F₂) | H | Cl | Me | O |
| C-11887 | Me(R) | single bond | H | *c*-Pr(2,2-F₂) | H | Cl | Me | O |
| C-11888 | Me(S) | single bond | H | *c*-Pr(2,2-F₂) | H | Cl | Me | O |
| C-11889 | H | single bond | Me | *c*-Pr(2,2-F₂) | H | Me | Me | O |
| C-11890 | Me | single bond | H | *c*-Pr(2,2-F₂) | H | Me | Me | O |
| C-11891 | Me(R) | single bond | H | *c*-Pr(2,2-F₂) | H | Me | Me | O |
| C-11892 | Me(S) | single bond | H | *c*-Pr(2,2-F₂) | H | Me | Me | O |
| C-11893 | H | single bond | Me | *c*-Pr(1-Cl) | H | Cl | Me | O |
| C-11894 | Me | single bond | H | *c*-Pr(1-Cl) | H | Cl | Me | O |
| C-11895 | Me(R) | single bond | H | *c*-Pr(1-Cl) | H | Cl | Me | O |
| C-11896 | Me(S) | single bond | H | *c*-Pr(1-Cl) | H | Cl | Me | O |
| C-11897 | H | single bond | Me | *c*-Pr(1-Cl) | H | Me | Me | O |
| C-11898 | Me | single bond | H | *c*-Pr(1-Cl) | H | Me | Me | O |

**[Table 499]**

| Compound Number | Y | W | Z | R¹ | R² | R³ | R¹⁰ | A |
|---|---|---|---|---|---|---|---|---|
| C-11899 | Me(R) | single bond | H | *c*-Pr(1-Cl) | H | Me | Me | O |
| C-11900 | Me(S) | single bond | H | *c*-Pr(1-Cl) | H | Me | Me | O |
| C-11901 | H | single bond | Me | *c*-Pr(1-Br) | H | Cl | Me | O |
| C-11902 | Me | single bond | H | *c*-Pr(1-Br) | H | Cl | Me | O |
| C-11903 | Me(R) | single bond | H | *c*-Pr(1-Br) | H | Cl | Me | O |
| C-11904 | Me(S) | single bond | H | *c*-Pr(1-Br) | H | Cl | Me | O |
| C-11905 | H | single bond | Me | *c*-Pr(1-Br) | H | Me | Me | O |
| C-11906 | Me | single bond | H | *c*-Pr(1-Br) | H | Me | Me | O |
| C-11907 | Me(R) | single bond | H | *c*-Pr(1-Br) | H | Me | Me | O |
| C-11908 | Me(S) | single bond | H | *c*-Pr(1-Br) | H | Me | Me | O |
| C-11909 | H | single bond | Me | *c*-Pr(1-Me) | H | Cl | Me | O |
| C-11910 | Me | single bond | H | *c*-Pr(1-Me) | H | Cl | Me | O |
| C-11911 | Me(R) | single bond | H | *c*-Pr(1-Me) | H | Cl | Me | O |
| C-11912 | Me(S) | single bond | H | *c*-Pr(1-Me) | H | Cl | Me | O |
| C-11913 | H | single bond | Me | *c*-Pr(1-Me) | H | Me | Me | O |
| C-11914 | Me | single bond | H | *c*-Pr(1-Me) | H | Me | Me | O |
| C-11915 | Me(R) | single bond | H | *c*-Pr(1-Me) | H | Me | Me | O |
| C-11916 | Me(S) | single bond | H | *c*-Pr(1-Me) | H | Me | Me | O |
| C-11917 | H | single bond | Me | *c*-Pr(1-CH₂OH) | H | Cl | Me | O |
| C-11918 | Me | single bond | H | *c*-Pr(1-CH₂OH) | H | Cl | Me | O |
| C-11919 | Me(R) | single bond | H | *c*-Pr(1-CH₂OH) | H | Cl | Me | O |
| C-11920 | Me(S) | single bond | H | *c*-Pr(1-CH₂OH) | H | Cl | Me | O |
| C-11921 | H | single bond | Me | *c*-Pr(1-CH₂OH) | H | Me | Me | O |
| C-11922 | Me | single bond | H | *c*-Pr(1-CH₂OH) | H | Me | Me | O |
| C-11923 | Me(R) | single bond | H | *c*-Pr(1-CH₂OH) | H | Me | Me | O |
| C-11924 | Me(S) | single bond | H | *c*-Pr(1-CH₂OH) | H | Me | Me | O |
| C-11925 | H | single bond | Me | *c*-Pr[1-CH₂OS(=O)₂Me] | H | Cl | Me | O |
| C-11926 | Me | single bond | H | *c*-Pr[1-CH₂OS(=O)₂Me] | H | Cl | Me | O |
| C-11927 | Me(R) | single bond | H | *c*-Pr[1-CH₂OS(=O)₂Me] | H | Cl | Me | O |
| C-11928 | Me(S) | single bond | H | *c*-Pr[1-CH₂OS(=O)₂Me] | H | Cl | Me | O |
| C-11929 | H | single bond | Me | *c*-Pr[1-CH₂OS(=O)₂Me] | H | Me | Me | O |
| C-11930 | Me | single bond | H | *c*-Pr[1-CH₂OS(=O)₂Me] | H | Me | Me | O |
| C-11931 | Me(R) | single bond | H | *c*-Pr[1-CH₂OS(=O)₂Me] | H | Me | Me | O |
| C-11932 | Me(S) | single bond | H | *c*-Pr[1-CH₂OS(=O)₂Me] | H | Me | Me | O |
| C-11933 | H | single bond | Me | *c*-Pr(1-CH₂OMe) | H | Cl | Me | O |
| C-11934 | Me | single bond | H | *c*-Pr(1-CH₂OMe) | H | Cl | Me | O |
| C-11935 | Me(R) | single bond | H | *c*-Pr(1-CH₂OMe) | H | Cl | Me | O |
| C-11936 | Me(S) | single bond | H | *c*-Pr(1-CH₂OMe) | H | Cl | Me | O |
| C-11937 | H | single bond | Me | *c*-Pr(1-CH₂OMe) | H | Me | Me | O |
| C-11938 | Me | single bond | H | *c*-Pr(1-CH₂OMe) | H | Me | Me | O |
| C-11939 | Me(R) | single bond | H | *c*-Pr(1-CH₂OMe) | H | Me | Me | O |
| C-11940 | Me(S) | single bond | H | *c*-Pr(1-CH₂OMe) | H | Me | Me | O |
| C-11941 | H | single bond | Me | *c*-Pr(1-CH₂CN) | H | Cl | Me | O |
| C-11942 | Me | single bond | H | *c*-Pr(1-CH₂CN) | H | Cl | Me | O |

**[Table 500]**

| Compound Number | Y | W | Z | R¹ | R² | R³ | R¹⁰ | A |
|---|---|---|---|---|---|---|---|---|
| C-11943 | Me(R) | single bond | H | *c*-Pr(1-CH₂CN) | H | Cl | Me | O |
| C-11944 | Me(S) | single bond | H | *c*-Pr(1-CH₂CN) | H | Cl | Me | O |
| C-11945 | H | single bond | Me | *c*-Pr(1-CH₂CN) | H | Me | Me | O |
| C-11946 | Me | single bond | H | *c*-Pr(1-CH₂CN) | H | Me | Me | O |
| C-11947 | Me(R) | single bond | H | *c*-Pr(1-CH₂CN) | H | Me | Me | O |
| C-11948 | Me(S) | single bond | H | *c*-Pr(1-CH₂CN) | H | Me | Me | O |
| C-11949 | H | single bond | Me | *c*-Pr(1-CH₂SMe) | H | Cl | Me | O |
| C-11950 | Me | single bond | H | *c*-Pr(1-CH₂SMe) | H | Cl | Me | O |
| C-11951 | Me(R) | single bond | H | *c*-Pr(1-CH₂SMe) | H | Cl | Me | O |
| C-11952 | Me(S) | single bond | H | *c*-Pr(1-CH₂SMe) | H | Cl | Me | O |
| C-11953 | H | single bond | Me | *c*-Pr(1-CH₂SMe) | H | Me | Me | O |
| C-11954 | Me | single bond | H | *c*-Pr(1-CH₂SMe) | H | Me | Me | O |
| C-11955 | Me(R) | single bond | H | *c*-Pr(1-CH₂SMe) | H | Me | Me | O |
| C-11956 | Me(S) | single bond | H | *c*-Pr(1-CH₂SMe) | H | Me | Me | O |
| C-11957 | H | single bond | Me | *c*-Pr[1-CH₂S(=O)Me] | H | Cl | Me | O |
| C-11958 | Me | single bond | H | *c*-Pr[1-CH₂S(=O)Me] | H | Cl | Me | O |
| C-11959 | Me(R) | single bond | H | *c*-Pr[1-CH₂S(=O)Me] | H | Cl | Me | O |
| C-11960 | Me(S) | single bond | H | *c*-Pr[1-CH₂S(=O)Me] | H | Cl | Me | O |
| C-11961 | H | single bond | Me | *c*-Pr[1-CH₂S(=O)Me] | H | Me | Me | O |
| C-11962 | Me | single bond | H | *c*-Pr[1-CH₂S(=O)Me] | H | Me | Me | O |
| C-11963 | Me(R) | single bond | H | *c*-Pr[1-CH₂S(=O)Me] | H | Me | Me | O |
| C-11964 | Me(S) | single bond | H | *c*-Pr[1-CH₂S(=O)Me] | H | Me | Me | O |
| C-11965 | H | single bond | Me | *c*-Pr[1-CH₂S(=O)₂Me] | H | Cl | Me | O |
| C-11966 | Me | single bond | H | *c*-Pr[1-CH₂S(=O)₂Me] | H | Cl | Me | O |
| C-11967 | Me(R) | single bond | H | *c*-Pr[1-CH₂S(=O)₂Me] | H | Cl | Me | O |
| C-11968 | Me(S) | single bond | H | *c*-Pr[1-CH₂S(=O)₂Me] | H | Cl | Me | O |
| C-11969 | H | single bond | Me | *c*-Pr[1-CH₂S(=O)₂Me] | H | Me | Me | O |
| C-11970 | Me | single bond | H | *c*-Pr[1-CH₂S(=O)₂Me] | H | Me | Me | O |
| C-11971 | Me(R) | single bond | H | *c*-Pr[1-CH₂S(=O)₂Me] | H | Me | Me | O |
| C-11972 | Me(S) | single bond | H | *c*-Pr[1-CH₂S(=O)₂Me] | H | Me | Me | O |
| C-11973 | H | single bond | Me | *c*-Pr(1-CH₂F) | H | Cl | Me | O |
| C-11974 | Me | single bond | H | *c*-Pr(1-CH₂F) | H | Cl | Me | O |
| C-11975 | Me(R) | single bond | H | *c*-Pr(1-CH₂F) | H | Cl | Me | O |
| C-11976 | Me(S) | single bond | H | *c*-Pr(1-CH₂F) | H | Cl | Me | O |
| C-11977 | H | single bond | Me | *c*-Pr(1-CH₂F) | H | Me | Me | O |
| C-11978 | Me | single bond | H | *c*-Pr(1-CH₂F) | H | Me | Me | O |
| C-11979 | Me(R) | single bond | H | *c*-Pr(1-CH₂F) | H | Me | Me | O |
| C-11980 | Me(S) | single bond | H | *c*-Pr(1-CH₂F) | H | Me | Me | O |
| C-11981 | H | single bond | Me | *c*-Pr(1-CHF₂) | H | Cl | Me | O |
| C-11982 | Me | single bond | H | *c*-Pr(1-CHF₂) | H | Cl | Me | O |
| C-11983 | Me(R) | single bond | H | *c*-Pr(1-CHF₂) | H | Cl | Me | O |
| C-11984 | Me(S) | single bond | H | *c*-Pr(1-CHF₂) | H | Cl | Me | O |
| C-11985 | H | single bond | Me | *c*-Pr(1-CHF₂) | H | Me | Me | O |
| C-11986 | Me | single bond | H | *c*-Pr(1-CHF₂) | H | Me | Me | O |

**[Table 501]**

| Compound Number | Y | W | Z | R¹ | R² | R³ | R¹⁰ | A |
|---|---|---|---|---|---|---|---|---|
| C-11987 | Me(R) | single bond | H | *c*-Pr(1-CHF₂) | H | Me | Me | O |
| C-11988 | Me(S) | single bond | H | *c*-Pr(1-CHF₂) | H | Me | Me | O |
| C-11989 | H | single bond | Me | *c*-Pr(1-CF₃) | H | Cl | Me | O |
| C-11990 | Me | single bond | H | *c*-Pr(1-CF₃) | H | Cl | Me | O |
| C-11991 | Me(R) | single bond | H | *c*-Pr(1-CF₃) | H | Cl | Me | O |
| C-11992 | Me(S) | single bond | H | *c*-Pr(1-CF₃) | H | Cl | Me | O |
| C-11993 | H | single bond | Me | *c*-Pr(1-CF₃) | H | Me | Me | O |
| C-11994 | Me | single bond | H | *c*-Pr(1-CF₃) | H | Me | Me | O |
| C-11995 | Me(R) | single bond | H | *c*-Pr(1-CF₃) | H | Me | Me | O |
| C-11996 | Me(S) | single bond | H | *c*-Pr(1-CF₃) | H | Me | Me | O |
| C-11997 | H | single bond | Me | *c*-Pr(1-C≡CH) | H | Cl | Me | O |
| C-11998 | Me | single bond | H | *c*-Pr(1-C≡CH) | H | Cl | Me | O |
| C-11999 | Me(R) | single bond | H | *c*-Pr(1-C≡CH) | H | Cl | Me | O |
| C-12000 | Me(S) | single bond | H | *c*-Pr(1-C≡CH) | H | Cl | Me | O |
| C-12001 | H | single bond | Me | *c*-Pr(1-C≡CH) | H | Me | Me | O |
| C-12002 | Me | single bond | H | *c*-Pr(1-C≡CH) | H | Me | Me | O |
| C-12003 | Me(R) | single bond | H | *c*-Pr(1-C≡CH) | H | Me | Me | O |
| C-12004 | Me(S) | single bond | H | *c*-Pr(1-C≡CH) | H | Me | Me | O |
| C-12005 | H | single bond | Me | *c*-Pr[1-C(=O)OEt] | H | Cl | Me | O |
| C-12006 | Me | single bond | H | *c-*Pr[1-C(=O)OEt] | H | Cl | Me | O |
| C-12007 | Me(R) | single bond | H | *c*-Pr[1-C(=O)OEt] | H | Cl | Me | O |
| C-12008 | Me(S) | single bond | H | *c*-Pr[1-C(=O)OEt] | H | Cl | Me | O |
| C-12009 | H | single bond | Me | *c*-Pr[1-C(=O)OEt] | H | Me | Me | O |
| C-12010 | Me | single bond | H | *c*-Pr[1-C(=O)OEt] | H | Me | Me | O |
| C-12011 | Me(R) | single bond | H | *c*-Pr[1-C(=O)OEt] | H | Me | Me | O |
| C-12012 | Me(S) | single bond | H | *c*-Pr[1-C(=O)OEt] | H | Me | Me | O |
| C-12013 | H | single bond | Me | *c*-Pr[1-C(=O)OH] | H | Cl | Me | O |
| C-12014 | Me | single bond | H | *c*-Pr[1-C(=O)OH] | H | Cl | Me | O |
| C-12015 | Me(R) | single bond | H | *c*-Pr[1-C(=O)OH] | H | Cl | Me | O |
| C-12016 | Me(S) | single bond | H | *c*-Pr[1-C(=O)OH] | H | Cl | Me | O |
| C-12017 | H | single bond | Me | *c*-Pr[1-C(=O)OH] | H | Me | Me | O |
| C-12018 | Me | single bond | H | *c*-Pr[1-C(=O)OH] | H | Me | Me | O |
| C-12019 | Me(R) | single bond | H | *c*-Pr[1-C(=O)OH] | H | Me | Me | O |
| C-12020 | Me(S) | single bond | H | *c*-Pr[1-C(=O)OH] | H | Me | Me | O |
| C-12021 | H | single bond | Me | *c*-Pr[1-C(=O)NH₂] | H | Cl | Me | O |
| C-12022 | Me | single bond | H | *c*-Pr[1-C(=O)NH₂] | H | Cl | Me | O |
| C-12023 | Me(R) | single bond | H | *c*-Pr[1-C(=O)NH₂] | H | Cl | Me | O |
| C-12024 | Me(S) | single bond | H | *c*-Pr[1-C(=O)NH₂] | H | Cl | Me | O |
| C-12025 | H | single bond | Me | *c*-Pr[1-C(=O)NH₂] | H | Me | Me | O |
| C-12026 | Me | single bond | H | *c*-Pr[1-C(=O)NH₂] | H | Me | Me | O |
| C-12027 | Me(R) | single bond | H | *c*-Pr[1-C(=O)NH₂] | H | Me | Me | O |
| C-12028 | Me(S) | single bond | H | *c*-Pr[1-C(=O)NH₂] | H | Me | Me | O |
| C-12029 | H | single bond | Me | *c*-Pr(1-OMe) | H | Cl | Me | O |
| C-12030 | Me | single bond | H | *c*-Pr(1-OMe) | H | Cl | Me | O |

**[Table 502]**

| Compound Number | Y | W | Z | R¹ | R² | R³ | R¹⁰ | A |
|---|---|---|---|---|---|---|---|---|
| C-12031 | Me(R) | single bond | H | *c*-Pr(1-OMe) | H | Cl | Me | O |
| C-12032 | Me(S) | single bond | H | *c*-Pr(1-OMe) | H | Cl | Me | O |
| C-12033 | H | single bond | Me | *c*-Pr(1-OMe) | H | Me | Me | O |
| C-12034 | Me | single bond | H | *c*-Pr(1-OMe) | H | Me | Me | O |
| C-12035 | Me(R) | single bond | H | *c*-Pr(1-OMe) | H | Me | Me | O |
| C-12036 | Me(S) | single bond | H | *c*-Pr(1-OMe) | H | Me | Me | O |
| C-12037 | H | single bond | Me | *c*-Pr(1-SMe) | H | Cl | Me | O |
| C-12038 | Me | single bond | H | *c*-Pr(1-SMe) | H | Cl | Me | O |
| C-12039 | Me(R) | single bond | H | *c*-Pr(1-SMe) | H | Cl | Me | O |
| C-12040 | Me(S) | single bond | H | *c*-Pr(1-SMe) | H | Cl | Me | O |
| C-12041 | H | single bond | Me | *c*-Pr(1-SMe) | H | Me | Me | O |
| C-12042 | Me | single bond | H | *c*-Pr(1-SMe) | H | Me | Me | O |
| C-12043 | Me(R) | single bond | H | *c*-Pr(1-SMe) | H | Me | Me | O |
| C-12044 | Me(S) | single bond | H | *c*-Pr(1-SMe) | H | Me | Me | O |
| C-12045 | H | single bond | Me | *c*-Pr[1-S(=O)Me] | H | Cl | Me | O |
| C-12046 | Me | single bond | H | *c*-Pr[1-S(=O)Me] | H | Cl | Me | O |
| C-12047 | Me(R) | single bond | H | *c*-Pr[1-S(=O)Me] | H | Cl | Me | O |
| C-12048 | Me(S) | single bond | H | *c*-Pr[1-S(=O)Me] | H | Cl | Me | O |
| C-12049 | H | single bond | Me | *c*-Pr[1-S(=O)Me] | H | Me | Me | O |
| C-12050 | Me | single bond | H | *c*-Pr[1-S(=O)Me] | H | Me | Me | O |
| C-12051 | Me(R) | single bond | H | *c*-Pr[1-S(=O)Me] | H | Me | Me | O |
| C-12052 | Me(S) | single bond | H | *c*-Pr[1-S(=O)Me] | H | Me | Me | O |
| C-12053 | H | single bond | Me | *c*-Pr[1-S(=O)₂Me] | H | Cl | Me | O |
| C-12054 | Me | single bond | H | *c*-Pr[1-S(=O)₂Me] | H | Cl | Me | O |
| C-12055 | Me(R) | single bond | H | *c*-Pr[1-S(=O)₂Me] | H | Cl | Me | O |
| C-12056 | Me(S) | single bond | H | *c*-Pr[1-S(=O)₂Me] | H | Cl | Me | O |
| C-12057 | H | single bond | Me | *c*-Pr[1-S(=O)₂Me] | H | Me | Me | O |
| C-12058 | Me | single bond | H | *c*-Pr[1-S(=O)₂Me] | H | Me | Me | O |
| C-12059 | Me(R) | single bond | H | *c*-Pr[1-S(=O)₂Me] | H | Me | Me | O |
| C-12060 | Me(S) | single bond | H | *c*-Pr[1-S(=O)₂Me] | H | Me | Me | O |
| C-12061 | H | single bond | Me | *c*-Pr[1-S(=O)₂CHF₂] | H | Cl | Me | O |
| C-12062 | Me | single bond | H | *c*-Pr[1-S(=O)₂CHF₂] | H | Cl | Me | O |
| C-12063 | Me(R) | single bond | H | *c*-Pr[1-S(=O)₂CHF₂] | H | Cl | Me | O |
| C-12064 | Me(S) | single bond | H | *c*-Pr[1-S(=O)₂CHF₂] | H | Cl | Me | O |
| C-12065 | H | single bond | Me | *c*-Pr[1-S(=O)₂CHF₂] | H | Me | Me | O |
| C-12066 | Me | single bond | H | *c*-Pr[1-S(=O)₂CHF₂] | H | Me | Me | O |
| C-12067 | Me(R) | single bond | H | *c*-Pr[1-S(=O)₂CHF₂] | H | Me | Me | O |
| C-12068 | Me(S) | single bond | H | *c*-Pr[1-S(=O)₂CHF₂] | H | Me | Me | O |
| C-12069 | H | single bond | Me | *c*-Pr[1-S(=O)₂CF₃] | H | Cl | Me | O |
| C-12070 | Me | single bond | H | *c*-Pr[1-S(=O)₂CF₃] | H | Cl | Me | O |
| C-12071 | Me(R) | single bond | H | *c*-Pr[1-S(=O)₂CF₃] | H | Cl | Me | O |
| C-12072 | Me(S) | single bond | H | *c*-Pr[1-S(=O)₂CF₃] | H | Cl | Me | O |
| C-12073 | H | single bond | Me | *c*-Pr[1-S(=O)₂CF₃] | H | Me | Me | O |
| C-12074 | Me | single bond | H | *c*-Pr[1-S(=O)₂CF₃] | H | Me | Me | O |

**[Table 503]**

| Compound Number | Y | W | Z | R¹ | R² | R³ | R¹⁰ | A |
|---|---|---|---|---|---|---|---|---|
| C-12075 | Me(R) | single bond | H | *c*-Pr[1-S(=O)₂CF₃] | H | Me | Me | O |
| C-12076 | Me(S) | single bond | H | *c*-Pr[1-S(=O)₂CF₃] | H | Me | Me | O |
| C-12077 | H | single bond | Me | *c*-Pr[1-(5,6-dihydro-1,4,2-dioxazin-3-yl)] | H | Cl | Me | O |
| C-12078 | Me | single bond | H | *c*-Pr[1-(5,6-dihydro-1,4,2-dioxazin-3-yl)] | H | Cl | Me | O |
| C-12079 | Me(R) | single bond | H | *c*-Pr[1-(5,6-dihydro-1,4,2-dioxazin-3-yl)] | H | Cl | Me | O |
| C-12080 | Me(S) | single bond | H | *c*-Pr[1-(5,6-dihydro-1,4,2-dioxazin-3-yl)] | H | Cl | Me | O |
| C-12081 | H | single bond | Me | *c*-Pr[1-(5,6-dihydro-1,4,2-dioxazin-3-yl)] | H | Me | Me | O |
| C-12082 | Me | single bond | H | *c*-Pr[1-(5,6-dihydro-1,4,2-dioxazin-3-yl)] | H | Me | Me | O |
| C-12083 | Me(R) | single bond | H | *c*-Pr[1-(5,6-dihydro-1,4,2-dioxazin-3-yl)] | H | Me | Me | O |
| C-12084 | Me(S) | single bond | H | *c*-Pr[1-(5,6-dihydro-1,4,2-dioxazin-3-yl)] | H | Me | Me | O |
| C-12085 | H | single bond | Me | *c*-Pr[1-(5-hydroxy-1,3,4-oxadiazol-2-yl)] | H | Cl | Me | O |
| C-12086 | Me | single bond | H | *c*-Pr[1-(5-hydroxy-1,3,4-oxadiazol-2-yl)] | H | Cl | Me | O |
| C-12087 | Me(R) | single bond | H | *c*-Pr[1-(5-hydroxy-1,3,4-oxadiazol-2-yl)] | H | Cl | Me | O |
| C-12088 | Me(S) | single bond | H | *c*-Pr[1-(5-hydroxy-1,3,4-oxadiazol-2-yl)] | H | Cl | Me | O |
| C-12089 | H | single bond | Me | *c*-Pr[1-(5-hydroxy-1,3,4-oxadiazol-2-yl)] | H | Me | Me | O |
| C-12090 | Me | single bond | H | *c*-Pr[1-(5-hydroxy-1,3,4-oxadiazol-2-yl)] | H | Me | Me | O |
| C-12091 | Me(R) | single bond | H | *c*-Pr[1-(5-hydroxy-1,3,4-oxadiazol-2-yl)] | H | Me | Me | O |
| C-12092 | Me(S) | single bond | H | *c-*Pr[1-(5-hydroxy-1,3,4-oxadiazol-2-yl)] | H | Me | Me | O |
| C-12093 | H | single bond | Me | *c*-Pr[1-(pyrimidin-2-yl)] | H | Cl | Me | O |
| C-12094 | Me | single bond | H | *c*-Pr[1-(pyrimidin-2-yl)] | H | Cl | Me | O |
| C-12095 | Me(R) | single bond | H | *c*-Pr[1-(pyrimidin-2-yl)] | H | Cl | Me | O |
| C-12096 | Me(S) | single bond | H | *c*-Pr[1-(pyrimidin-2-yl)] | H | Cl | Me | O |
| C-12097 | H | single bond | Me | *c*-Pr[1-(pyrimidin-2-yl)] | H | Me | Me | O |
| C-12098 | Me | single bond | H | *c*-Pr[1-(pyrimidin-2-yl)] | H | Me | Me | O |
| C-12099 | Me(R) | single bond | H | *c*-Pr[1-(pyrimidin-2-yl)] | H | Me | Me | O |
| C-12100 | Me(S) | single bond | H | *c*-Pr[1-(pyrimidin-2-yl)] | H | Me | Me | O |
| C-12101 | H | single bond | Me | *t*-Bu | H | Cl | Me | O |
| C-12102 | Me | single bond | H | *t*-Bu | H | Cl | Me | O |
| C-12103 | Me(R) | single bond | H | *t*-Bu | H | Cl | Me | O |
| C-12104 | Me(S) | single bond | H | *t*-Bu | H | Cl | Me | O |
| C-12105 | H | single bond | Me | *t*-Bu | H | Me | Me | O |
| C-12106 | Me | single bond | H | *t*-Bu | H | Me | Me | O |
| C-12107 | Me(R) | single bond | H | *t*-Bu | H | Me | Me | O |
| C-12108 | Me(S) | single bond | H | *t*-Bu | H | Me | Me | O |
| C-12109 | H | single bond | Me | *c*-Bu | H | Cl | Me | O |
| C-12110 | Me | single bond | H | *c*-Bu | H | Cl | Me | O |
| C-12111 | Me(R) | single bond | H | *c*-Bu | H | Cl | Me | O |
| C-12112 | Me(S) | single bond | H | *c*-Bu | H | Cl | Me | O |
| C-12113 | H | single bond | Me | *c*-Bu | H | Me | Me | O |
| C-12114 | Me | single bond | H | *c*-Bu | H | Me | Me | O |
| C-12115 | Me(R) | single bond | H | *c*-Bu | H | Me | Me | O |
| C-12116 | Me(S) | single bond | H | *c*-Bu | H | Me | Me | O |
| C-12117 | H | single bond | Me | *c*-Bu(1-CN) | H | Cl | Me | O |
| C-12118 | Me | single bond | H | *c*-Bu(1-CN) | H | Cl | Me | O |

**[Table 504]**

| Compound Number | Y | W | Z | R¹ | R² | R³ | R¹⁰ | A |
|---|---|---|---|---|---|---|---|---|
| C-12119 | Me(R) | single bond | H | *c*-Bu(1-CN) | H | Cl | Me | O |
| C-12120 | Me(S) | single bond | H | *c*-Bu(1-CN) | H | Cl | Me | O |
| C-12121 | H | single bond | Me | *c*-Bu(1-CN) | H | Me | Me | O |
| C-12122 | Me | single bond | H | *c*-Bu(1-CN) | H | Me | Me | O |
| C-12123 | Me(R) | single bond | H | *c*-Bu(1-CN) | H | Me | Me | O |
| C-12124 | Me(S) | single bond | H | *c*-Bu(1-CN) | H | Me | Me | O |
| C-12125 | H | single bond | Me | *c*-Bu(3,3-F₂) | H | Cl | Me | O |
| C-12126 | Me | single bond | H | *c*-Bu(3,3-F₂) | H | Cl | Me | O |
| C-12127 | Me(R) | single bond | H | *c*-Bu(3,3-F₂) | H | Cl | Me | O |
| C-12128 | Me(S) | single bond | H | *c*-Bu(3,3-F₂) | H | Cl | Me | O |
| C-12129 | H | single bond | Me | *c*-Bu(3,3-F₂) | H | Me | Me | O |
| C-12130 | Me | single bond | H | *c*-Bu(3,3-F₂) | H | Me | Me | O |
| C-12131 | Me(R) | single bond | H | *c*-Bu(3,3-F₂) | H | Me | Me | O |
| C-12132 | Me(S) | single bond | H | *c*-Bu(3,3-F₂) | H | Me | Me | O |
| C-12133 | H | single bond | Me | oxetan-3-yl | H | Cl | Me | O |
| C-12134 | Me | single bond | H | oxetan-3-yl | H | Cl | Me | O |
| C-12135 | Me(R) | single bond | H | oxetan-3-yl | H | Cl | Me | O |
| C-12136 | Me(S) | single bond | H | oxetan-3-yl | H | Cl | Me | O |
| C-12137 | H | single bond | Me | oxetan-3-yl | H | Me | Me | O |
| C-12138 | Me | single bond | H | oxetan-3-yl | H | Me | Me | O |
| C-12139 | Me(R) | single bond | H | oxetan-3-yl | H | Me | Me | O |
| C-12140 | Me(S) | single bond | H | oxetan-3-yl | H | Me | Me | O |
| C-12141 | H | single bond | Me | oxetan(3-CN)-3-yl | H | Cl | Me | O |
| C-12142 | Me | single bond | H | oxetan(3-CN)-3-yl | H | Cl | Me | O |
| C-12143 | Me(R) | single bond | H | oxetan(3-CN)-3-yl | H | Cl | Me | O |
| C-12144 | Me(S) | single bond | H | oxetan(3-CN)-3-yl | H | Cl | Me | O |
| C-12145 | H | single bond | Me | oxetan(3-CN)-3-yl | H | Me | Me | O |
| C-12146 | Me | single bond | H | oxetan(3-CN)-3-yl | H | Me | Me | O |
| C-12147 | Me(R) | single bond | H | oxetan(3-CN)-3-yl | H | Me | Me | O |
| C-12148 | Me(S) | single bond | H | oxetan(3-CN)-3-yl | H | Me | Me | O |
| C-12149 | H | single bond | Me | azetidin[1-C(=O)Me]-3-yl | H | Cl | Me | O |
| C-12150 | Me | single bond | H | azetidin[1-C(=O)Me]-3-yl | H | Cl | Me | O |
| C-12151 | Me(R) | single bond | H | azetidin[1-C(=O)Me]-3-yl | H | Cl | Me | O |
| C-12152 | Me(S) | single bond | H | azetidin[1-C(=O)Me]-3-yi | H | Cl | Me | O |
| C-12153 | H | single bond | Me | azetidin[1-C(=O)Me]-3-yl | H | Me | Me | O |
| C-12154 | Me | single bond | H | azetidin[1-C(=O)Me]-3-yl | H | Me | Me | O |
| C-12155 | Me(R) | single bond | H | azetidin[1-C(=O)Me]-3-yl | H | Me | Me | O |
| C-12156 | Me(S) | single bond | H | azetidin[1-C(=O)Me]-3-yl | H | Me | Me | O |
| C-12157 | H | single bond | Me | azetidin[1-S(=O)₂Me]-3-yl | H | Cl | Me | O |
| C-12158 | Me | single bond | H | azetidin[1-S(=O)₂Me]-3-yl | H | Cl | Me | O |
| C-12159 | Me(R) | single bond | H | azetidin[1-S(=O)₂Me]-3-yl | H | Cl | Me | O |
| C-12160 | Me(S) | single bond | H | azetidin[1-S(=O)₂Me]-3-yl | H | Cl | Me | O |
| C-12161 | H | single bond | Me | azetidin[1-S(=O)₂Me]-3-yl | H | Me | Me | O |
| C-12162 | Me | single bond | H | azetidin[1-S(=O)₂Me]-3-yl | H | Me | Me | O |

**[Table 505]**

| Compound Number | Y | W | Z | R¹ | R² | R³ | R¹⁰ | A |
|---|---|---|---|---|---|---|---|---|
| C-12163 | Me(R) | single bond | H | azetidin[1-S(=O)₂Me]-3-yl | H | Me | Me | O |
| C-12164 | Me(S) | single bond | H | azetidin[1-S(=O)₂Me]-3-yl | H | Me | Me | O |
| C-12165 | H | single bond | Me | azetidin[1-S(=O)₂CHF₂]-3-yl | H | Cl | Me | O |
| C-12166 | Me | single bond | H | azetidin[1-S(=O)₂CHF₂]-3-yl | H | Cl | Me | O |
| C-12167 | Me(R) | single bond | H | azetidin[1-S(=O)₂CHF₂]-3-yl | H | Cl | Me | O |
| C-12168 | Me(S) | single bond | H | azetidin[1-S(=O)₂CHF₂]-3-yl | H | Cl | Me | O |
| C-12169 | H | single bond | Me | azetidin[1-S(=O)₂CHF₂]-3-yl | H | Me | Me | O |
| C-12170 | Me | single bond | H | azetidin[1-S(=O)₂CHF₂]-3-yl | H | Me | Me | O |
| C-12171 | Me(R) | single bond | H | azetidin[1-S(=O)₂CHF₂]-3-yl | H | Me | Me | O |
| C-12172 | Me(S) | single bond | H | azetidin[1-S(=O)₂CHF₂]-3-yl | H | Me | Me | O |
| C-12173 | H | single bond | Me | *c*-Pen | H | Cl | Me | O |
| C-12174 | Me | single bond | H | *c*-Pen | H | Cl | Me | O |
| C-12175 | Me(R) | single bond | H | *c*-Pen | H | Cl | Me | O |
| C-12176 | Me(S) | single bond | H | c-Pen | H | Cl | Me | O |
| C-12177 | H | single bond | Me | *c*-Pen | H | Me | Me | O |
| C-12178 | Me | single bond | H | *c*-Pen | H | Me | Me | O |
| C-12179 | Me(R) | single bond | H | *c*-Pen | H | Me | Me | O |
| C-12180 | Me(S) | single bond | H | *c*-Pen | H | Me | Me | O |
| C-12181 | H | single bond | Me | tetrahydrofuran-3-yl | H | Cl | Me | O |
| C-12182 | Me | single bond | H | tetrahydrofuran-3-yl | H | Cl | Me | O |
| C-12183 | Me(R) | single bond | H | tetrahydrofuran-3-yl | H | Cl | Me | O |
| C-12184 | Me(S) | single bond | H | tetra hydrofuran-3-yl | H | Cl | Me | O |
| C-12185 | H | single bond | Me | tetrahydrofuran-3-yl | H | Me | Me | O |
| C-12186 | Me | single bond | H | tetrahydrofuran-3-yl | H | Me | Me | O |
| C-12187 | Me(R) | single bond | H | tetrahydrofuran-3-yl | H | Me | Me | O |
| C-12188 | Me(S) | single bond | H | tetrahydrofuran-3-yl | H | Me | Me | O |
| C-12189 | H | single bond | Me | tetrahydrothiophen-3-yl | H | Cl | Me | O |
| C-12190 | Me | single bond | H | tetrahydrothiophen-3-yl | H | Cl | Me | O |
| C-12191 | Me(R) | single bond | H | tetrahydrothiophen-3-yl | H | Cl | Me | O |
| C-12192 | Me(S) | single bond | H | tetrahydrothiophen-3-yl | H | Cl | Me | O |
| C-12193 | H | single bond | Me | tetrahydrothiophen-3-yl | H | Me | Me | O |
| C-12194 | Me | single bond | H | tetrahydrothiophen-3-yl | H | Me | Me | O |
| C-12195 | Me(R) | single bond | H | tetrahydrothiophen-3-yl | H | Me | Me | O |
| C-12196 | Me(S) | single bond | H | tetrahydrothiophen-3-yl | H | Me | Me | O |
| C-12197 | H | single bond | Me | *c*-Pen(1-CN) | H | Cl | Me | O |
| C-12198 | Me | single bond | H | *c*-Pen(1-CN) | H | Cl | Me | O |
| C-12199 | Me(R) | single bond | H | *c*-Pen(1-CN) | H | Cl | Me | O |
| C-12200 | Me(S) | single bond | H | *c*-Pen(1-CN) | H | Cl | Me | O |
| C-12201 | H | single bond | Me | *c*-Pen(1-CN) | H | Me | Me | O |
| C-12202 | Me | single bond | H | *c*-Pen(1-CN) | H | Me | Me | O |
| C-12203 | Me(R) | single bond | H | *c*-Pen(1-CN) | H | Me | Me | O |
| C-12204 | Me(S) | single bond | H | *c*-Pen(1-CN) | H | Me | Me | O |
| C-12205 | H | single bond | Me | tetrahydrofuran(3-CN)-3-yl | H | Cl | Me | O |
| C-12206 | Me | single bond | H | tetrahydrofuran(3-CN)-3-yl | H | Cl | Me | O |

**[Table 506]**

| Compound Number | Y | W | Z | R¹ | R² | R³ | R¹⁰ | A |
|---|---|---|---|---|---|---|---|---|
| C-12207 | Me(R) | single bond | H | tetrahydrofuran(3-CN)-3-yl | H | Cl | Me | O |
| C-12208 | Me(S) | single bond | H | tetrahydrofuran(3-CN)-3-yl | H | Cl | Me | O |
| C-12209 | H | single bond | Me | tetrahydrofuran(3-CN)-3-yl | H | Me | Me | O |
| C-12210 | Me | single bond | H | tetrahydrofuran(3-CN)-3-yl | H | Me | Me | O |
| C-12211 | Me(R) | single bond | H | tetrahydrofuran(3-CN)-3-yl | H | Me | Me | O |
| C-12212 | Me(S) | single bond | H | tetrahydrofuran(3-CN)-3-yl | H | Me | Me | O |
| C-12213 | H | single bond | Me | tetrahydrothiophen(3-CN)-3-yl | H | Cl | Me | O |
| C-12214 | Me | single bond | H | tetrahydrothiophen(3-CN)-3-yl | H | Cl | Me | O |
| C-12215 | Me(R) | single bond | H | tetrahydrothiophen(3-CN)-3-yl | H | Cl | Me | O |
| C-12216 | Me(S) | single bond | H | tetrahydrothiophen(3-CN)-3-yl | H | Cl | Me | O |
| C-12217 | H | single bond | Me | tetrahydrothiophen(3-CN)-3-yl | H | Me | Me | O |
| C-12218 | Me | single bond | H | tetrahydrothiophen(3-CN)-3-yl | H | Me | Me | O |
| C-12219 | Me(R) | single bond | H | tetrahydrothiophen(3-CN)-3-yl | H | Me | Me | O |
| C-12220 | Me(S) | single bond | H | tetrahydrothiophen(3-CN)-3-yl | H | Me | Me | O |
| C-12221 | H | single bond | Me | bicyclo[1.1.1]pentan-1-yl | H | Cl | Me | O |
| C-12222 | Me | single bond | H | bicyclo[1.1.1]pentan-1-yl | H | Cl | Me | O |
| C-12223 | Me(R) | single bond | H | bicyclo[1.1.1]pentan-1-yl | H | Cl | Me | O |
| C-12224 | Me(S) | single bond | H | bicyclo[1.1.1]pentan-1-yl | H | Cl | Me | O |
| C-12225 | H | single bond | Me | bicyclo[1.1.1]pentan-1-yl | H | Me | Me | O |
| C-12226 | Me | single bond | H | bicyclo[1.1.1]pentan-1-yl | H | Me | Me | O |
| C-12227 | Me(R) | single bond | H | bicyclo[1.1.1]pentan-1-yl | H | Me | Me | O |
| C-12228 | Me(S) | single bond | H | bicyclo[1.1.1]pentan-1-yl | H | Me | Me | O |
| C-12229 | H | single bond | Me | *c*-Hex | H | Cl | Me | O |
| C-12230 | Me | single bond | H | *c*-Hex | H | Cl | Me | O |
| C-12231 | Me(R) | single bond | H | *c*-Hex | H | Cl | Me | O |
| C-12232 | Me(S) | single bond | H | *c*-Hex | H | Cl | Me | O |
| C-12233 | H | single bond | Me | *c*-Hex | H | Me | Me | O |
| C-12234 | Me | single bond | H | *c*-Hex | H | Me | Me | O |
| C-12235 | Me(R) | single bond | H | *c*-Hex | H | Me | Me | O |
| C-12236 | Me(S) | single bond | H | *c*-Hex | H | Me | Me | O |
| C-12237 | H | single bond | Me | tetrahydropyran-4-yl | H | Cl | Me | O |
| C-12238 | Me | single bond | H | tetrahydropyran-4-yl | H | Cl | Me | O |
| C-12239 | Me(R) | single bond | H | tetrahydropyran-4-yl | H | Cl | Me | O |
| C-12240 | Me(S) | single bond | H | tetrahydropyran-4-yl | H | Cl | Me | O |
| C-12241 | H | single bond | Me | tetrahydropyran-4-yl | H | Me | Me | O |
| C-12242 | Me | single bond | H | tetrahydropyran-4-yl | H | Me | Me | O |
| C-12243 | Me(R) | single bond | H | tetrahydropyran-4-yl | H | Me | Me | O |
| C-12244 | Me(S) | single bond | H | tetrahydropyran-4-yl | H | Me | Me | O |
| C-12245 | H | single bond | Me | tetrahydrothiopyran-4-yl | H | Cl | Me | O |
| C-12246 | Me | single bond | H | tetrahydrothiopyran-4-yl | H | Cl | Me | O |
| C-12247 | Me(R) | single bond | H | tetrahydrothiopyran-4-yl | H | Cl | Me | O |
| C-12248 | Me(S) | single bond | H | tetrahydrothiopyran-4-yl | H | Cl | Me | O |
| C-12249 | H | single bond | Me | tetrahydrothiopyran-4-yl | H | Me | Me | O |
| C-12250 | Me | single bond | H | tetrahydrothiopyran-4-yl | H | Me | Me | O |

**[Table 507]**

| Compound Number | Y | W | Z | R¹ | R² | R³ | R¹⁰ | A |
|---|---|---|---|---|---|---|---|---|
| C-12251 | Me(R) | single bond | H | tetrahydrothiopyran-4-yl | H | Me | Me | O |
| C-12252 | Me(S) | single bond | H | tetrahydrothiopyran-4-yl | H | Me | Me | O |
| C-12253 | H | single bond | Me | *c*-Hex(1-CN) | H | Cl | Me | O |
| C-12254 | Me | single bond | H | *c*-Hex(1-CN) | H | Cl | Me | O |
| C-12255 | Me(R) | single bond | H | *c*-Hex(1-CN) | H | Cl | Me | O |
| C-12256 | Me(S) | single bond | H | *c*-Hex(1-CN) | H | Cl | Me | O |
| C-12257 | H | single bond | Me | *c*-Hex(1-CN) | H | Me | Me | O |
| C-12258 | Me | single bond | H | *c*-Hex(1-CN) | H | Me | Me | O |
| C-12259 | Me(R) | single bond | H | *c*-Hex(1-CN) | H | Me | Me | O |
| C-12260 | Me(S) | single bond | H | *c*-Hex(1-CN) | H | Me | Me | O |
| C-12261 | H | single bond | Me | tetrahydropyran(4-CN)-4-yl | H | Cl | Me | O |
| C-12262 | Me | single bond | H | tetrahydropyran(4-CN)-4-yl | H | Cl | Me | O |
| C-12263 | Me(R) | single bond | H | tetrahydropyran(4-CN)-4-yl | H | Cl | Me | O |
| C-12264 | Me(S) | single bond | H | tetrahydropyran(4-CN)-4-yl | H | Cl | Me | O |
| C-12265 | H | single bond | Me | tetrahydropyran(4-CN)-4-yl | H | Me | Me | O |
| C-12266 | Me | single bond | H | tetrahydropyran(4-CN)-4-yl | H | Me | Me | O |
| C-12267 | Me(R) | single bond | H | tetrahydropyran(4-CN)-4-yl | H | Me | Me | O |
| C-12268 | Me(S) | single bond | H | tetrahydropyran(4-CN)-4-yl | H | Me | Me | O |
| C-12269 | H | single bond | Me | tetrahydrothiopyran(4-CN)-4-yl | H | Cl | Me | O |
| C-12270 | Me | single bond | H | tetrahydrothiopyran(4-CN)-4-yl | H | Cl | Me | O |
| C-12271 | Me(R) | single bond | H | tetrahydrothiopyran(4-CN)-4-yl | H | Cl | Me | O |
| C-12272 | Me(S) | single bond | H | tetrahydrothiopyran(4-CN)-4-yl | H | Cl | Me | O |
| C-12273 | H | single bond | Me | tetrahydrothiopyran(4-CN)-4-yl | H | Me | Me | O |
| C-12274 | Me | single bond | H | tetrahydrothiopyran(4-CN)-4-yl | H | Me | Me | O |
| C-12275 | Me(R) | single bond | H | tetrahydrothiopyran(4-CN)-4-yl | H | Me | Me | O |
| C-12276 | Me(S) | single bond | H | tetrahydrothiopyran(4-CN)-4-yl | H | Me | Me | O |
| C-12277 | H | single bond | Me | CH₂(C-Pr) | H | Cl | Me | O |
| C-12278 | Me | single bond | H | CH₂(C-Pr) | H | Cl | Me | O |
| C-12279 | Me(R) | single bond | H | CH₂(C-Pr) | H | Cl | Me | O |
| C-12280 | Me(S) | single bond | H | CH₂(C-Pr) | H | Cl | Me | O |
| C-12281 | H | single bond | Me | CH₂(C-Pr) | H | Me | Me | O |
| C-12282 | Me | single bond | H | CH₂(C-Pr) | H | Me | Me | O |
| C-12283 | Me(R) | single bond | H | CH₂(C-Pr) | H | Me | Me | O |
| C-12284 | Me(S) | single bond | H | CH₂(C-Pr) | H | Me | Me | O |
| C-12285 | H | single bond | Me | CH₂[*c*-Pr(1-CN)] | H | Cl | Me | O |
| C-12286 | Me | single bond | H | CH₂[*c*-Pr(1-CN)] | H | Cl | Me | O |
| C-12287 | Me(R) | single bond | H | CH₂[*c*-Pr(1-CN)] | H | Cl | Me | O |
| C-12288 | Me(S) | single bond | H | CH₂[*c*-Pr(1-CN)] | H | Cl | Me | O |
| C-12289 | H | single bond | Me | CH₂[*c*-Pr(1-CN)] | H | Me | Me | O |
| C-12290 | Me | single bond | H | CH₂[*c*-Pr(1-CN)] | H | Me | Me | O |
| C-12291 | Me(R) | single bond | H | CH₂[*c*-Pr(1-CN)] | H | Me | Me | O |
| C-12292 | Me(S) | single bond | H | CH₂[*c*-Pr(1-CN)] | H | Me | Me | O |
| C-12293 | H | single bond | Me | CH(Me)(*c*-Pr) | H | Cl | Me | O |
| C-12294 | Me | single bond | H | CH(Me)(*c*-Pr) | H | Cl | Me | O |

**[Table 508]**

| Compound Number | Y | W | Z | R¹ | R² | R³ | R¹⁰ | A |
|---|---|---|---|---|---|---|---|---|
| C-12295 | Me(R) | single bond | H | CH(Me)(*c*-Pr) | H | Cl | Me | O |
| C-12296 | Me(S) | single bond | H | CH(Me)(*c*-Pr) | H | Cl | Me | O |
| C-12297 | H | single bond | Me | CH(Me)(*c*-Pr) | H | Me | Me | O |
| C-12298 | Me | single bond | H | CH(Me)(*c*-Pr) | H | Me | Me | O |
| C-12299 | Me(R) | single bond | H | CH(Me)(*c*-Pr) | H | Me | Me | O |
| C-12300 | Me(S) | single bond | H | CH(Me)(*c*-Pr) | H | Me | Me | O |
| C-12301 | H | single bond | Me | CH(Me)CH₂OMe | H | Cl | Me | O |
| C-12302 | Me | single bond | H | CH(Me)CH₂OMe | H | Cl | Me | O |
| C-12303 | Me(R) | single bond | H | CH(Me)CH₂OMe | H | Cl | Me | O |
| C-12304 | Me(S) | single bond | H | CH(Me)CH₂OMe | H | Cl | Me | O |
| C-12305 | H | single bond | Me | CH(Me)CH₂OMe | H | Me | Me | O |
| C-12306 | Me | single bond | H | CH(Me)CH₂OMe | H | Me | Me | O |
| C-12307 | Me(R) | single bond | H | CH(Me)CH₂OMe | H | Me | Me | O |
| C-12308 | Me(S) | single bond | H | CH(Me)CH₂OMe | H | Me | Me | O |
| C-12309 | H | single bond | Me | CH₂CH=CH₂ | H | Cl | Me | O |
| C-12310 | Me | single bond | H | CH₂CH=CH₂ | H | Cl | Me | O |
| C-12311 | Me(R) | single bond | H | CH₂CH=CH₂ | H | Cl | Me | O |
| C-12312 | Me(S) | single bond | H | CH₂CH=CH₂ | H | Cl | Me | O |
| C-12313 | H | single bond | Me | CH₂CH=CH₂ | H | Me | Me | O |
| C-12314 | Me | single bond | H | CH₂CH=CH₂ | H | Me | Me | O |
| C-12315 | Me(R) | single bond | H | CH₂CH=CH₂ | H | Me | Me | O |
| C-12316 | Me(S) | single bond | H | CH₂CH=CH₂ | H | Me | Me | O |
| C-12317 | H | single bond | Me | CH₂C≡CH | H | Cl | Me | O |
| C-12318 | Me | single bond | H | CH₂C≡CH | H | Cl | Me | O |
| C-12319 | Me(R) | single bond | H | CH₂C≡CH | H | Cl | Me | O |
| C-12320 | Me(S) | single bond | H | CH₂C≡CH | H | Cl | Me | O |
| C-12321 | H | single bond | Me | CH₂C≡CH | H | Me | Me | O |
| C-12322 | Me | single bond | H | CH₂C≡CH | H | Me | Me | O |
| C-12323 | Me(R) | single bond | H | CH₂C≡CH | H | Me | Me | O |
| C-12324 | Me(S) | single bond | H | CH₂C≡CH | H | Me | Me | O |
| C-12325 | H | single bond | Me | CH₂CN | H | Cl | Me | O |
| C-12326 | Me | single bond | H | CH₂CN | H | Cl | Me | O |
| C-12327 | Me(R) | single bond | H | CH₂CN | H | Cl | Me | O |
| C-12328 | Me(S) | single bond | H | CH₂CN | H | Cl | Me | O |
| C-12329 | H | single bond | Me | CH₂CN | H | Me | Me | O |
| C-12330 | Me | single bond | H | CH₂CN | H | Me | Me | O |
| C-12331 | Me(R) | single bond | H | CH₂CN | H | Me | Me | O |
| C-12332 | Me(S) | single bond | H | CH₂CN | H | Me | Me | O |
| C-12333 | H | single bond | Me | CH(Me)CN | H | Cl | Me | O |
| C-12334 | Me | single bond | H | CH(Me)CN | H | Cl | Me | O |
| C-12335 | Me(R) | single bond | H | CH(Me)CN | H | Cl | Me | O |
| C-12336 | Me(S) | single bond | H | CH(Me)CN | H | Cl | Me | O |
| C-12337 | H | single bond | Me | CH(Me)CN | H | Me | Me | O |
| C-12338 | Me | single bond | H | CH(Me)CN | H | Me | Me | O |

**[Table 509]**

| Compound Number | Y | W | Z | R¹ | R² | R³ | R¹⁰ | A |
|---|---|---|---|---|---|---|---|---|
| C-12339 | Me(R) | single bond | H | CH(Me)CN | H | Me | Me | O |
| C-12340 | Me(S) | single bond | H | CH(Me)CN | H | Me | Me | O |
| C-12341 | H | single bond | Me | C(Me)₂CN | H | Cl | Me | O |
| C-12342 | Me | single bond | H | C(Me)₂CN | H | Cl | Me | O |
| C-12343 | Me(R) | single bond | H | C(Me)₂CN | H | Cl | Me | O |
| C-12344 | Me(S) | single bond | H | C(Me)₂CN | H | Cl | Me | O |
| C-12345 | H | single bond | Me | C(Me)₂CN | H | Me | Me | O |
| C-12346 | Me | single bond | H | C(Me)₂CN | H | Me | Me | O |
| C-12347 | Me(R) | single bond | H | C(Me)₂CN | H | Me | Me | O |
| C-12348 | Me(S) | single bond | H | C(Me)₂CN | H | Me | Me | O |
| C-12349 | H | single bond | Me | C(Me)(*i*-Pr)CN | H | Cl | Me | O |
| C-12350 | Me | single bond | H | C(Me)(*i*-Pr)CN | H | Cl | Me | O |
| C-12351 | Me(R) | single bond | H | C(Me)(*i*-Pr)CN | H | Cl | Me | O |
| C-12352 | Me(S) | single bond | H | C(Me)(*i*-Pr)CN | H | Cl | Me | O |
| C-12353 | H | single bond | Me | C(Me)(*i*-Pr)CN | H | Me | Me | O |
| C-12354 | Me | single bond | H | C(Me)(*i*-Pr)CN | H | Me | Me | O |
| C-12355 | Me(R) | single bond | H | C(Me)(*i*-Pr)CN | H | Me | Me | O |
| C-12356 | Me(S) | single bond | H | C(Me)(*i*-Pr)CN | H | Me | Me | O |
| C-12357 | H | single bond | Me | OMe | H | Cl | Me | O |
| C-12358 | Me | single bond | H | OMe | H | Cl | Me | O |
| C-12359 | Me(R) | single bond | H | OMe | H | Cl | Me | O |
| C-12360 | Me(S) | single bond | H | OMe | H | Cl | Me | O |
| C-12361 | H | single bond | Me | OMe | H | Me | Me | O |
| C-12362 | Me | single bond | H | OMe | H | Me | Me | O |
| C-12363 | Me(R) | single bond | H | OMe | H | Me | Me | O |
| C-12364 | Me(S) | single bond | H | OMe | H | Me | Me | O |
| C-12365 | H | single bond | Me | CH=NOMe | H | Cl | Me | O |
| C-12366 | Me | single bond | H | CH=NOMe | H | Cl | Me | O |
| C-12367 | Me(R) | single bond | H | CH=NOMe | H | Cl | Me | O |
| C-12368 | Me(S) | single bond | H | CH=NOMe | H | Cl | Me | O |
| C-12369 | H | single bond | Me | CH=NOMe | H | Me | Me | O |
| C-12370 | Me | single bond | H | CH=NOMe | H | Me | Me | O |
| C-12371 | Me(R) | single bond | H | CH=NOMe | H | Me | Me | O |
| C-12372 | Me(S) | single bond | H | CH=NOMe | H | Me | Me | O |
| C-12373 | H | single bond | Me | morpholin-4-yl | H | Cl | Me | O |
| C-12374 | Me | single bond | H | morpholin-4-yl | H | Cl | Me | O |
| C-12375 | Me(R) | single bond | H | morpholin-4-yl | H | Cl | Me | O |
| C-12376 | Me(S) | single bond | H | morpholin-4-yl | H | Cl | Me | O |
| C-12377 | H | single bond | Me | morpholin-4-yl | H | Me | Me | O |
| C-12378 | Me | single bond | H | morpholin-4-yl | H | Me | Me | O |
| C-12379 | Me(R) | single bond | H | morpholin-4-yl | H | Me | Me | O |
| C-12380 | Me(S) | single bond | H | morpholin-4-yl | H | Me | Me | O |
| C-12381 | H | single bond | Me | Ph | H | Cl | Me | O |
| C-12382 | Me | single bond | H | Ph | H | Cl | Me | O |

**[Table 510]**

| Compound Number | Y | W | Z | R¹ | R² | R³ | R¹⁰ | A |
|---|---|---|---|---|---|---|---|---|
| C-12383 | Me(R) | single bond | H | Ph | H | Cl | Me | O |
| C-12384 | Me(S) | single bond | H | Ph | H | Cl | Me | O |
| C-12385 | H | single bond | Me | Ph | H | Me | Me | O |
| C-12386 | Me | single bond | H | Ph | H | Me | Me | O |
| C-12387 | Me(R) | single bond | H | Ph | H | Me | Me | O |
| C-12388 | Me(S) | single bond | H | Ph | H | Me | Me | O |
| C-12389 | H | single bond | Me | CH₂Ph | H | Cl | Me | O |
| C-12390 | Me | single bond | H | CH₂Ph | H | Cl | Me | O |
| C-12391 | Me(R) | single bond | H | CH₂Ph | H | Cl | Me | O |
| C-12392 | Me(S) | single bond | H | CH₂Ph | H | Cl | Me | O |
| C-12393 | H | single bond | Me | CH₂Ph | H | Me | Me | O |
| C-12394 | Me | single bond | H | CH₂Ph | H | Me | Me | O |
| C-12395 | Me(R) | single bond | H | CH₂Ph | H | Me | Me | O |
| C-12396 | Me(S) | single bond | H | CH₂Ph | H | Me | Me | O |
| C-12397 | H | single bond | Me | thiazole-2-yl | H | Cl | Me | O |
| C-12398 | Me | single bond | H | thiazole-2-yl | H | Cl | Me | O |
| C-12399 | Me(R) | single bond | H | thiazole-2-yl | H | Cl | Me | O |
| C-12400 | Me(S) | single bond | H | thiazole-2-yl | H | Cl | Me | O |
| C-12401 | H | single bond | Me | thiazole-2-yl | H | Me | Me | O |
| C-12402 | Me | single bond | H | thiazole-2-yl | H | Me | Me | O |
| C-12403 | Me(R) | single bond | H | thiazole-2-yl | H | Me | Me | O |
| C-12404 | Me(S) | single bond | H | thiazole-2-yl | H | Me | Me | O |
| C-12405 | H | single bond | Me | 1-Me-pyrazol-3-yl | H | Cl | Me | O |
| C-12406 | Me | single bond | H | 1-Me-pyrazol-3-yl | H | Cl | Me | O |
| C-12407 | Me(R) | single bond | H | 1-Me-pyrazol-3-yl | H | Cl | Me | O |
| C-12408 | Me(S) | single bond | H | 1-Me-pyrazol-3-yl | H | Cl | Me | O |
| C-12409 | H | single bond | Me | 1-Me-pyrazol-3-yl | H | Me | Me | O |
| C-12410 | Me | single bond | H | 1-Me-pyrazol-3-yl | H | Me | Me | O |
| C-12411 | Me(R) | single bond | H | 1-Me-pyrazol-3-yl | H | Me | Me | O |
| C-12412 | Me(S) | single bond | H | 1-Me-pyrazol-3-yl | H | Me | Me | O |
| C-12413 | H | single bond | Me | 1-Me-pyrazol-5-yl | H | Cl | Me | O |
| C-12414 | Me | single bond | H | 1-Me-pyrazol-5-yl | H | Cl | Me | O |
| C-12415 | Me(R) | single bond | H | 1-Me-pyrazol-5-yl | H | Cl | Me | O |
| C-12416 | Me(S) | single bond | H | 1-Me-pyrazol-5-yl | H | Cl | Me | O |
| C-12417 | H | single bond | Me | 1-Me-pyrazol-5-yl | H | Me | Me | O |
| C-12418 | Me | single bond | H | 1-Me-pyrazol-5-yl | H | Me | Me | O |
| C-12419 | Me(R) | single bond | H | 1-Me-pyrazol-5-yl | H | Me | Me | O |
| C-12420 | Me(S) | single bond | H | 1-Me-pyrazol-5-yl | H | Me | Me | O |
| C-12421 | H | single bond | Me | CH₂(pyrimidin-2-yl) | H | Cl | Me | O |
| C-12422 | Me | single bond | H | CH₂(pyrimidin-2-yl) | H | Cl | Me | O |
| C-12423 | Me(R) | single bond | H | CH₂(pyrimidin-2-yl) | H | Cl | Me | O |
| C-12424 | Me(S) | single bond | H | CH₂(pyrimidin-2-yl) | H | Cl | Me | O |
| C-12425 | H | single bond | Me | CH₂(pyrimidin-2-yl) | H | Me | Me | O |
| C-12426 | Me | single bond | H | CH₂(pyrimidin-2-yl) | H | Me | Me | O |

**[Table 511]**

| Compound Number | Y | W | Z | R¹ | R² | R³ | R¹⁰ | A |
|---|---|---|---|---|---|---|---|---|
| C-12427 | Me(R) | single bond | H | CH₂(pyrimidin-2-yl) | H | Me | Me | O |
| C-12428 | Me(S) | single bond | H | CH₂(pyrimidin-2-yl) | H | Me | Me | O |
| C-12429 | H | single bond | Me | -CH₂CH₂CH₂- | | Cl | Me | O |
| C-12430 | Me | single bond | H | -CH₂CH₂CH₂- | | Cl | Me | O |
| C-12431 | Me(R) | single bond | H | -CH₂CH₂CH₂- | | Cl | Me | O |
| C-12432 | Me(S) | single bond | H | -CH₂CH₂CH₂- | | Cl | Me | O |
| C-12433 | H | single bond | Me | -CH₂CH₂CH₂- | | Me | Me | O |
| C-12434 | Me | single bond | H | -CH₂CH₂CH₂- | | Me | Me | O |
| C-12435 | Me(R) | single bond | H | -CH₂CH₂CH₂- | | Me | Me | O |
| C-12436 | Me(S) | single bond | H | -CH₂CH₂CH₂- | | Me | Me | O |
| C-12437 | H | single bond | Me | -CH₂CH₂OCH₂CH₂- | | Cl | Me | O |
| C-12438 | Me | single bond | H | -CH₂CH₂OCH₂CH₂- | | Cl | Me | O |
| C-12439 | Me(R) | single bond | H | -CH₂CH₂OCH₂CH₂- | | Cl | Me | O |
| C-12440 | Me(S) | single bond | H | -CH₂CH₂OCH₂CH₂- | | Cl | Me | O |
| C-12441 | H | single bond | Me | -CH₂CH₂OCH₂CH₂- | | Me | Me | O |
| C-12442 | Me | single bond | H | -CH₂CH₂OCH₂CH₂- | | Me | Me | O |
| C-12443 | Me(R) | single bond | H | -CH₂CH₂OCH₂CH₂- | | Me | Me | O |
| C-12444 | Me(S) | single bond | H | -CH₂CH₂OCH₂CH₂- | | Me | Me | O |
| C-12445 | H | single bond | Me | Me | H | Cl | CH₂OMe | O |
| C-12446 | Me | single bond | H | Me | H | Cl | CH₂OMe | O |
| C-12447 | Me(R) | single bond | H | Me | H | Cl | CH₂OMe | O |
| C-12448 | Me(S) | single bond | H | Me | H | Cl | CH₂OMe | O |
| C-12449 | H | single bond | Me | Me | H | Me | CH₂OMe | O |
| C-12450 | Me | single bond | H | Me | H | Me | CH₂OMe | O |
| C-12451 | Me(R) | single bond | H | Me | H | Me | CH₂OMe | O |
| C-12452 | Me(S) | single bond | H | Me | H | Me | CH₂OMe | O |
| C-12453 | H | single bond | Me | CH₂CHF₂ | H | Cl | CH₂OMe | O |
| C-12454 | Me | single bond | H | CH₂CHF₂ | H | Cl | CH₂OMe | O |
| C-12455 | Me(R) | single bond | H | CH₂CHF₂ | H | Cl | CH₂OMe | O |
| C-12456 | Me(S) | single bond | H | CH₂CHF₂ | H | Cl | CH₂OMe | O |
| C-12457 | H | single bond | Me | CH₂CHF₂ | H | Me | CH₂OMe | O |
| C-12458 | Me | single bond | H | CH₂CHF₂ | H | Me | CH₂OMe | O |
| C-12459 | Me(R) | single bond | H | CH₂CHF₂ | H | Me | CH₂OMe | O |
| C-12460 | Me(S) | single bond | H | CH₂CHF₂ | H | Me | CH₂OMe | O |
| C-12461 | H | single bond | Me | *n*-Pr | H | Cl | CH₂OMe | O |
| C-12462 | Me | single bond | H | *n*-Pr | H | Cl | CH₂OMe | O |
| C-12463 | Me(R) | single bond | H | *n*-Pr | H | Cl | CH₂OMe | O |
| C-12464 | Me(S) | single bond | H | *n*-Pr | H | Cl | CH₂OMe | O |
| C-12465 | H | single bond | Me | *n*-Pr | H | Me | CH₂OMe | O |
| C-12466 | Me | single bond | H | *n*-Pr | H | Me | CH₂OMe | O |
| C-12467 | Me(R) | single bond | H | *n*-Pr | H | Me | CH₂OMe | O |
| C-12468 | Me(S) | single bond | H | *n*-Pr | H | Me | CH₂OMe | O |
| C-12469 | H | single bond | Me | *i*-Pr | H | Cl | CH₂OMe | O |
| C-12470 | Me | single bond | H | *i*-Pr | H | Cl | CH₂OMe | O |

**[Table 512]**

| Compound Number | Y | W | Z | R¹ | R² | R³ | R¹⁰ | A |
|---|---|---|---|---|---|---|---|---|
| C-12471 | Me(R) | single bond | H | *i*-Pr | H | Cl | CH₂OMe | O |
| C-12472 | Me(S) | single bond | H | *i*-Pr | H | Cl | CH₂OMe | O |
| C-12473 | H | single bond | Me | *i*-Pr | H | Me | CH₂OMe | O |
| C-12474 | Me | single bond | H | *i*-Pr | H | Me | CH₂OMe | O |
| C-12475 | Me(R) | single bond | H | *i*-Pr | H | Me | CH₂OMe | O |
| C-12476 | Me(S) | single bond | H | *i*-Pr | H | Me | CH₂OMe | O |
| C-12477 | H | single bond | Me | *c*-Pr(1-F) | H | Cl | CH₂OMe | O |
| C-12478 | Me | single bond | H | *c*-Pr(1-F) | H | Cl | CH₂OMe | O |
| C-12479 | Me(R) | single bond | H | *c*-Pr(1-F) | H | Cl | CH₂OMe | O |
| C-12480 | Me(S) | single bond | H | *c*-Pr(1-F) | H | Cl | CH₂OMe | O |
| C-12481 | H | single bond | Me | *c*-Pr(1-F) | H | Me | CH₂OMe | O |
| C-12482 | Me | single bond | H | *c*-Pr(1-F) | H | Me | CH₂OMe | O |
| C-12483 | Me(R) | single bond | H | *c*-Pr(1-F) | H | Me | CH₂OMe | O |
| C-12484 | Me(S) | single bond | H | *c*-Pr(1-F) | H | Me | CH₂OMe | O |
| C-12485 | H | single bond | Me | *c*-Pr(2,2-F₂) | H | Cl | CH₂OMe | O |
| C-12486 | Me | single bond | H | *c*-Pr(2,2-F₂) | H | Cl | CH₂OMe | O |
| C-12487 | Me(R) | single bond | H | *c*-Pr(2,2-F₂) | H | Cl | CH₂OMe | O |
| C-12488 | Me(S) | single bond | H | *c*-Pr(2,2-F₂) | H | Cl | CH₂OMe | O |
| C-12489 | H | single bond | Me | *c*-Pr(2,2-F₂) | H | Me | CH₂OMe | O |
| C-12490 | Me | single bond | H | *c*-Pr(2,2-F₂) | H | Me | CH₂OMe | O |
| C-12491 | Me(R) | single bond | H | *c*-Pr(2,2-F₂) | H | Me | CH₂OMe | O |
| C-12492 | Me(S) | single bond | H | *c*-Pr(2,2-F₂) | H | Me | CH₂OMe | O |
| C-12493 | H | single bond | Me | *c*-Pr(1-Cl) | H | Cl | CH₂OMe | O |
| C-12494 | Me | single bond | H | *c*-Pr(1-Cl) | H | Cl | CH₂OMe | O |
| C-12495 | Me(R) | single bond | H | *c*-Pr(1-Cl) | H | Cl | CH₂OMe | O |
| C-12496 | Me(S) | single bond | H | *c*-Pr(1-Cl) | H | Cl | CH₂OMe | O |
| C-12497 | H | single bond | Me | *c*-Pr(1-Cl) | H | Me | CH₂OMe | O |
| C-12498 | Me | single bond | H | *c*-Pr(1-Cl) | H | Me | CH₂OMe | O |
| C-12499 | Me(R) | single bond | H | *c*-Pr(1-Cl) | H | Me | CH₂OMe | O |
| C-12500 | Me(S) | single bond | H | *c*-Pr(1-Cl) | H | Me | CH₂OMe | O |
| C-12501 | H | single bond | Me | *c*-Pr(1-Br) | H | Cl | CH₂OMe | O |
| C-12502 | Me | single bond | H | *c*-Pr(1-Br) | H | Cl | CH₂OMe | O |
| C-12503 | Me(R) | single bond | H | *c*-Pr(1-Br) | H | Cl | CH₂OMe | O |
| C-12504 | Me(S) | single bond | H | *c*-Pr(1-Br) | H | Cl | CH₂OMe | O |
| C-12505 | H | single bond | Me | *c*-Pr(1-Br) | H | Me | CH₂OMe | O |
| C-12506 | Me | single bond | H | *c*-Pr(1-Br) | H | Me | CH₂OMe | O |
| C-12507 | Me(R) | single bond | H | *c*-Pr(1-Br) | H | Me | CH₂OMe | O |
| C-12508 | Me(S) | single bond | H | *c*-Pr(1-Br) | H | Me | CH₂OMe | O |
| C-12509 | H | single bond | Me | *c*-Pr(1-Me) | H | Cl | CH₂OMe | O |
| C-12510 | Me | single bond | H | *c*-Pr(1-Me) | H | Cl | CH₂OMe | O |
| C-12511 | Me(R) | single bond | H | *c*-Pr(1-Me) | H | Cl | CH₂OMe | O |
| C-12512 | Me(S) | single bond | H | *c*-Pr(1-Me) | H | Cl | CH₂OMe | O |
| C-12513 | H | single bond | Me | *c*-Pr(1-Me) | H | Me | CH₂OMe | O |
| C-12514 | Me | single bond | H | *c*-Pr(1-Me) | H | Me | CH₂OMe | O |

**[Table 513]**

| Compound Number | Y | W | Z | R¹ | R² | R³ | R¹⁰ | A |
|---|---|---|---|---|---|---|---|---|
| C-12515 | Me(R) | single bond | H | *c*-Pr(1-Me) | H | Me | CH₂OMe | O |
| C-12516 | Me(S) | single bond | H | *c*-Pr(1-Me) | H | Me | CH₂OMe | O |
| C-12517 | H | single bond | Me | *c*-Pr(1-CH₂OH) | H | Cl | CH₂OMe | O |
| C-12518 | Me | single bond | H | *c*-Pr(1-CH₂OH) | H | Cl | CH₂OMe | O |
| C-12519 | Me(R) | single bond | H | *c*-Pr(1-CH₂OH) | H | Cl | CH₂OMe | O |
| C-12520 | Me(S) | single bond | H | *c*-Pr(1-CH₂OH) | H | Cl | CH₂OMe | O |
| C-12521 | H | single bond | Me | *c*-Pr(1-CH₂OH) | H | Me | CH₂OMe | O |
| C-12522 | Me | single bond | H | *c*-Pr(1-CH₂OH) | H | Me | CH₂OMe | O |
| C-12523 | Me(R) | single bond | H | *c*-Pr(1-CH₂OH) | H | Me | CH₂OMe | O |
| C-12524 | Me(S) | single bond | H | *c*-Pr(1-CH₂OH) | H | Me | CH₂OMe | O |
| C-12525 | H | single bond | Me | *c*-Pr[1-CH₂OS(=O)₂Me] | H | Cl | CH₂OMe | O |
| C-12526 | Me | single bond | H | *c*-Pr[1-CH₂OS(=O)₂Me] | H | Cl | CH₂OMe | O |
| C-12527 | Me(R) | single bond | H | *c*-Pr[1-CH₂OS(=O)₂Me] | H | Cl | CH₂OMe | O |
| C-12528 | Me(S) | single bond | H | *c*-Pr[1-CH₂OS(=O)₂Me] | H | Cl | CH₂OMe | O |
| C-12529 | H | single bond | Me | *c*-Pr[1-CH₂OS(=O)₂Me] | H | Me | CH₂OMe | O |
| C-12530 | Me | single bond | H | *c*-Pr[1-CH₂OS(=O)₂Me] | H | Me | CH₂OMe | O |
| C-12531 | Me(R) | single bond | H | *c*-Pr[1-CH₂OS(=O)₂Me] | H | Me | CH₂OMe | O |
| C-12532 | Me(S) | single bond | H | *c*-Pr[1-CH₂OS(=O)₂Me] | H | Me | CH₂OMe | O |
| C-12533 | H | single bond | Me | *c*-Pr(1-CH₂OMe) | H | Cl | CH₂OMe | O |
| C-12534 | Me | single bond | H | *c*-Pr(1-CH₂OMe) | H | Cl | CH₂OMe | O |
| C-12535 | Me(R) | single bond | H | *c*-Pr(1-CH₂OMe) | H | Cl | CH₂OMe | O |
| C-12536 | Me(S) | single bond | H | *c*-Pr(1-CH₂OMe) | H | Cl | CH₂OMe | O |
| C-12537 | H | single bond | Me | *c*-Pr(1-CH₂OMe) | H | Me | CH₂OMe | O |
| C-12538 | Me | single bond | H | *c*-Pr(1-CH₂OMe) | H | Me | CH₂OMe | O |
| C-12539 | Me(R) | single bond | H | *c*-Pr(1-CH₂OMe) | H | Me | CH₂OMe | O |
| C-12540 | Me(S) | single bond | H | *c*-Pr(1-CH₂OMe) | H | Me | CH₂OMe | O |
| C-12541 | H | single bond | Me | *c*-Pr(1-CH₂CN) | H | Cl | CH₂OMe | O |
| C-12542 | Me | single bond | H | *c*-Pr(1-CH₂CN) | H | Cl | CH₂OMe | O |
| C-12543 | Me(R) | single bond | H | *c*-Pr(1-CH₂CN) | H | Cl | CH₂OMe | O |
| C-12544 | Me(S) | single bond | H | *c*-Pr(1-CH₂CN) | H | Cl | CH₂OMe | O |
| C-12545 | H | single bond | Me | *c*-Pr(1-CH₂CN) | H | Me | CH₂OMe | O |
| C-12546 | Me | single bond | H | *c*-Pr(1-CH₂CN) | H | Me | CH₂OMe | O |
| C-12547 | Me(R) | single bond | H | *c*-Pr(1-CH₂CN) | H | Me | CH₂OMe | O |
| C-12548 | Me(S) | single bond | H | *c*-Pr(1-CH₂CN) | H | Me | CH₂OMe | O |
| C-12549 | H | single bond | Me | *c*-Pr(1-CH₂SMe) | H | Cl | CH₂OMe | O |
| C-12550 | Me | single bond | H | *c*-Pr(1-CH₂SMe) | H | Cl | CH₂OMe | O |
| C-12551 | Me(R) | single bond | H | *c*-Pr(1-CH₂SMe) | H | Cl | CH₂OMe | O |
| C-12552 | Me(S) | single bond | H | *c*-Pr(1-CH₂SMe) | H | Cl | CH₂OMe | O |
| C-12553 | H | single bond | Me | *c*-Pr(1-CH₂SMe) | H | Me | CH₂OMe | O |
| C-12554 | Me | single bond | H | *c*-Pr(1-CH₂SMe) | H | Me | CH₂OMe | O |
| C-12555 | Me(R) | single bond | H | *c*-Pr(1-CH₂SMe) | H | Me | CH₂OMe | O |
| C-12556 | Me(S) | single bond | H | *c*-Pr(1-CH₂SMe) | H | Me | CH₂OMe | O |
| C-12557 | H | single bond | Me | *c*-Pr[1-CH₂S(=O)Me] | H | Cl | CH₂OMe | O |
| C-12558 | Me | single bond | H | *c*-Pr[1-CH₂S(=O)Me] | H | Cl | CH₂OMe | O |

**[Table 514]**

| Compound Number | Y | W | Z | R¹ | R² | R³ | R¹⁰ | A |
|---|---|---|---|---|---|---|---|---|
| C-12559 | Me(R) | single bond | H | *c*-Pr[1-CH₂S(=O)Me] | H | Cl | CH₂OMe | O |
| C-12560 | Me(S) | single bond | H | *c*-Pr[1-CH₂S(=O)Me] | H | Cl | CH₂OMe | O |
| C-12561 | H | single bond | Me | *c*-Pr[1-CH₂S(=O)Me] | H | Me | CH₂OMe | O |
| C-12562 | Me | single bond | H | *c*-Pr[1-CH₂S(=O)Me] | H | Me | CH₂OMe | O |
| C-12563 | Me(R) | single bond | H | *c*-Pr[1-CH₂S(=O)Me] | H | Me | CH₂OMe | O |
| C-12564 | Me(S) | single bond | H | *c*-Pr[1-CH₂S(=O)Me] | H | Me | CH₂OMe | O |
| C-12565 | H | single bond | Me | *c*-Pr[1-CH₂S(=O)₂Me] | H | Cl | CH₂OMe | O |
| C-12566 | Me | single bond | H | *c*-Pr[1-CH₂S(=O)₂Me] | H | Cl | CH₂OMe | O |
| C-12567 | Me(R) | single bond | H | *c*-Pr[1-CH₂S(=O)₂Me] | H | Cl | CH₂OMe | O |
| C-12568 | Me(S) | single bond | H | *c*-Pr[1-CH₂S(=O)₂Me] | H | Cl | CH₂OMe | O |
| C-12569 | H | single bond | Me | *c*-Pr[1-CH₂S(=O)₂Me] | H | Me | CH₂OMe | O |
| C-12570 | Me | single bond | H | *c*-Pr[1-CH₂S(=O)₂Me] | H | Me | CH₂OMe | O |
| C-12571 | Me(R) | single bond | H | *c*-Pr[1-CH₂S(=O)₂Me] | H | Me | CH₂OMe | O |
| C-12572 | Me(S) | single bond | H | *c*-Pr[1-CH₂S(=O)₂Me] | H | Me | CH₂OMe | O |
| C-12573 | H | single bond | Me | *c*-Pr(1-CH₂F) | H | Cl | CH₂OMe | O |
| C-12574 | Me | single bond | H | *c*-Pr(1-CH₂F) | H | Cl | CH₂OMe | O |
| C-12575 | Me(R) | single bond | H | *c*-Pr(1-CH₂F) | H | Cl | CH₂OMe | O |
| C-12576 | Me(S) | single bond | H | *c*-Pr(1-CH₂F) | H | Cl | CH₂OMe | O |
| C-12577 | H | single bond | Me | *c*-Pr(1-CH₂F) | H | Me | CH₂OMe | O |
| C-12578 | Me | single bond | H | *c*-Pr(1-CH₂F) | H | Me | CH₂OMe | O |
| C-12579 | Me(R) | single bond | H | *c*-Pr(1-CH₂F) | H | Me | CH₂OMe | O |
| C-12580 | Me(S) | single bond | H | *c*-Pr(1-CH₂F) | H | Me | CH₂OMe | O |
| C-12581 | H | single bond | Me | *c*-Pr(1-CHF₂) | H | Cl | CH₂OMe | O |
| C-12582 | Me | single bond | H | *c*-Pr(1-CHF₂) | H | Cl | CH₂OMe | O |
| C-12583 | Me(R) | single bond | H | *c*-Pr(1-CHF₂) | H | Cl | CH₂OMe | O |
| C-12584 | Me(S) | single bond | H | *c*-Pr(1-CHF₂) | H | Cl | CH₂OMe | O |
| C-12585 | H | single bond | Me | *c*-Pr(1-CHF₂) | H | Me | CH₂OMe | O |
| C-12586 | Me | single bond | H | *c*-Pr(1-CHF₂) | H | Me | CH₂OMe | O |
| C-12587 | Me(R) | single bond | H | *c*-Pr(1-CHF₂) | H | Me | CH₂OMe | O |
| C-12588 | Me(S) | single bond | H | *c*-Pr(1-CHF₂) | H | Me | CH₂OMe | O |
| C-12589 | H | single bond | Me | *c*-pr(1-CF₃) | H | Cl | CH₂OMe | O |
| C-12590 | Me | single bond | H | *c*-Pr(1-CF₃) | H | Cl | CH₂OMe | O |
| C-12591 | Me(R) | single bond | H | *c*-Pr(1-CF₃) | H | Cl | CH₂OMe | O |
| C-12592 | Me(S) | single bond | H | *c*-Pr(1-CF₃) | H | Cl | CH₂OMe | O |
| C-12593 | H | single bond | Me | *c*-Pr(1-CF₃) | H | Me | CH₂OMe | O |
| C-12594 | Me | single bond | H | *c*-Pr(1-CF₃) | H | Me | CH₂OMe | O |
| C-12595 | Me(R) | single bond | H | *c*-Pr(1-CF₃) | H | Me | CH₂OMe | O |
| C-12596 | Me(S) | single bond | H | *c*-Pr(1-CF₃) | H | Me | CH₂OMe | O |
| C-12597 | H | single bond | Me | *c*-Pr(1-C≡CH) | H | Cl | CH₂OMe | O |
| C-12598 | Me | single bond | H | *c*-Pr(1-C≡CH) | H | Cl | CH₂OMe | O |
| C-12599 | Me(R) | single bond | H | *c*-Pr(1-C≡CH) | H | Cl | CH₂OMe | O |
| C-12600 | Me(S) | single bond | H | *c*-Pr(1-C≡CH) | H | Cl | CH₂OMe | O |
| C-12601 | H | single bond | Me | *c*-Pr(1-C≡CH) | H | Me | CH₂OMe | O |
| C-12602 | Me | single bond | H | *c*-Pr(1-C≡CH) | H | Me | CH₂OMe | O |

**[Table 515]**

| Compound Number | Y | W | Z | R¹ | R² | R³ | R¹⁰ | A |
|---|---|---|---|---|---|---|---|---|
| C-12603 | Me(R) | single bond | H | *c*-Pr(1-C≡CH) | H | Me | CH₂OMe | O |
| C-12604 | Me(S) | single bond | H | *c*-Pr(1-C≡CH) | H | Me | CH₂OMe | O |
| C-12605 | H | single bond | Me | *c* -Pr[1-C(=O)OEt] | H | Cl | CH₂OMe | O |
| C-12606 | Me | single bond | H | *c* -Pr[1-C(=O)OEt] | H | Cl | CH₂OMe | O |
| C-12607 | Me(R) | single bond | H | *c* -Pr[1-C(=O)OEt] | H | Cl | CH₂OMe | O |
| C-12608 | Me(S) | single bond | H | *c* -Pr[1-C(=O)OEt] | H | Cl | CH₂OMe | O |
| C-12609 | H | single bond | Me | *c-*Pr[1-C(=O)OEt] | H | Me | CH₂OMe | O |
| C-12610 | Me | single bond | H | *c* -Pr[1-C(=O)OEt] | H | Me | CH₂OMe | O |
| C-12611 | Me(R) | single bond | H | *c* -Pr[1-C(=O)OEt] | H | Me | CH₂OMe | O |
| C-12612 | Me(S) | single bond | H | *c* -Pr[1-C(=O)OEt] | H | Me | CH₂OMe | O |
| C-12613 | H | single bond | Me | *c* -Pr[1-C(=O)OH] | H | Cl | CH₂OMe | O |
| C-12614 | Me | single bond | H | *c* -Pr[1-C(=O)OH] | H | Cl | CH₂OMe | O |
| C-12615 | Me(R) | single bond | H | *c* -Pr[1-C(=O)OH] | H | Cl | CH₂OMe | O |
| C-12616 | Me(S) | single bond | H | *c* -Pr[1-C(=O)OH] | H | Cl | CH₂OMe | O |
| C-12617 | H | single bond | Me | *c* -Pr[1-C(=O)OH] | H | Me | CH₂OMe | O |
| C-12618 | Me | single bond | H | *c* -Pr[1-C(=O)OH] | H | Me | CH₂OMe | O |
| C-12619 | Me(R) | single bond | H | *c* -Pr[1-C(=O)OH] | H | Me | CH₂OMe | O |
| C-12620 | Me(S) | single bond | H | *c* -Pr[1-C(=O)OH] | H | Me | CH₂OMe | O |
| C-12621 | H | single bond | Me | *c*-Pr[1-C(=O)NH₂] | H | Cl | CH₂OMe | O |
| C-12622 | Me | single bond | H | *c*-Pr[1-C(=O)NH₂] | H | Cl | CH₂OMe | O |
| C-12623 | Me(R) | single bond | H | *c*-Pr[1-C(=O)NH₂] | H | Cl | CH₂OMe | O |
| C-12624 | Me(S) | single bond | H | *c*-Pr[1-C(=O)NH₂] | H | Cl | CH₂OMe | O |
| C-12625 | H | single bond | Me | *c*-Pr[1-C(=O)NH₂] | H | Me | CH₂OMe | O |
| C-12626 | Me | single bond | H | *c*-Pr[1-C(=O)NH₂] | H | Me | CH₂OMe | O |
| C-12627 | Me(R) | single bond | H | *c*-Pr[1-C(=O)NH₂] | H | Me | CH₂OMe | O |
| C-12628 | Me(S) | single bond | H | *c*-Pr[1-C(=O)NH₂] | H | Me | CH₂OMe | O |
| C-12629 | H | single bond | Me | *c*-Pr(1-OMe) | H | Cl | CH₂OMe | O |
| C-12630 | Me | single bond | H | *c*-Pr(1-OMe) | H | Cl | CH₂OMe | O |
| C-12631 | Me(R) | single bond | H | *c*-Pr(1-OMe) | H | Cl | CH₂OMe | O |
| C-12632 | Me(S) | single bond | H | *c*-Pr(1-OMe) | H | Cl | CH₂OMe | O |
| C-12633 | H | single bond | Me | *c*-Pr(1-OMe) | H | Me | CH₂OMe | O |
| C-12634 | Me | single bond | H | *c*-Pr(1-OMe) | H | Me | CH₂OMe | O |
| C-12635 | Me(R) | single bond | H | *c*-Pr(1-OMe) | H | Me | CH₂OMe | O |
| C-12636 | Me(S) | single bond | H | *c*-Pr(1-OMe) | H | Me | CH₂OMe | O |
| C-12637 | H | single bond | Me | *c*-Pr(1-SMe) | H | Cl | CH₂OMe | O |
| C-12638 | Me | single bond | H | *c*-Pr(1-SMe) | H | Cl | CH₂OMe | O |
| C-12639 | Me(R) | single bond | H | *c*-Pr(1-SMe) | H | Cl | CH₂OMe | O |
| C-12640 | Me(S) | single bond | H | *c*-Pr(1-SMe) | H | Cl | CH₂OMe | O |
| C-12641 | H | single bond | Me | *c*-Pr(1-SMe) | H | Me | CH₂OMe | O |
| C-12642 | Me | single bond | H | *c*-Pr(1-SMe) | H | Me | CH₂OMe | O |
| C-12643 | Me(R) | single bond | H | *c*-Pr(1-SMe) | H | Me | CH₂OMe | O |
| C-12644 | Me(S) | single bond | H | *c*-Pr(1-SMe) | H | Me | CH₂OMe | O |
| C-12645 | H | single bond | Me | *c*-Pr[1-S(=O)Me] | H | Cl | CH₂OMe | O |
| C-12646 | Me | single bond | H | *c*-Pr[1-S(=O)Me] | H | Cl | CH₂OMe | O |

**[Table 516]**

| Compound Number | Y | W | Z | R¹ | R² | R³ | R¹⁰ | A |
|---|---|---|---|---|---|---|---|---|
| C-12647 | Me(R) | single bond | H | *c*-Pr[1-S(=O)Me] | H | Cl | CH₂OMe | O |
| C-12648 | Me(S) | single bond | H | *c*-Pr[1-S(=O)Me] | H | Cl | CH₂OMe | O |
| C-12649 | H | single bond | Me | *c*-Pr[1-S(=O)Me] | H | Me | CH₂OMe | O |
| C-12650 | Me | single bond | H | *c*-Pr[1-S(=O)Me] | H | Me | CH₂OMe | O |
| C-12651 | Me(R) | single bond | H | *c*-Pr[1-S(=O)Me] | H | Me | CH₂OMe | O |
| C-12652 | Me(S) | single bond | H | *c* -Pr[1-S(=O)Me] | H | Me | CH₂OMe | O |
| C-12653 | H | single bond | Me | *c*-Pr[1-S(=O)₂Me] | H | Cl | CH₂OMe | O |
| C-12654 | Me | single bond | H | *c*-Pr[1-S(=O)₂Me] | H | Cl | CH₂OMe | O |
| C-12655 | Me(R) | single bond | H | *c*-Pr[1-S(=O)₂Me] | H | Cl | CH₂OMe | O |
| C-12656 | Me(S) | single bond | H | *c-*Pr[1-S(=O)₂Me] | H | Cl | CH₂OMe | O |
| C-12657 | H | single bond | Me | *c*-Pr[1-S(=O)₂Me] | H | Me | CH₂OMe | O |
| C-12658 | Me | single bond | H | *c*-Pr[1-S(=O)₂Me] | H | Me | CH₂OMe | O |
| C-12659 | Me(R) | single bond | H | *c*-Pr[1-S(=O)₂Me] | H | Me | CH₂OMe | O |
| C-12660 | Me(S) | single bond | H | *c*-Pr[1-S(=O)₂Me] | H | Me | CH₂OMe | O |
| C-12661 | H | single bond | Me | *c*-Pr[1-S(=O)₂CHF₂] | H | Cl | CH₂OMe | O |
| C-12662 | Me | single bond | H | *c*-Pr[1-S(=O)₂CHF₂] | H | Cl | CH₂OMe | O |
| C-12663 | Me(R) | single bond | H | *c*-Pr[1-S(=O)₂CHF₂] | H | Cl | CH₂OMe | O |
| C-12664 | Me(S) | single bond | H | *c*-Pr[1-S(=O)₂CHF₂] | H | Cl | CH₂OMe | O |
| C-12665 | H | single bond | Me | *c*-Pr[1-S(=O)₂CHF₂] | H | Me | CH₂OMe | O |
| C-12666 | Me | single bond | H | *c*-Pr[1-S(=O)₂CHF₂] | H | Me | CH₂OMe | O |
| C-12667 | Me(R) | single bond | H | *c*-Pr[1-S(=O)₂CHF₂] | H | Me | CH₂OMe | O |
| C-12668 | Me(S) | single bond | H | *c*-Pr[1-S(=O)₂CHF₂] | H | Me | CH₂OMe | O |
| C-12669 | H | single bond | Me | *c*-Pr[1-S(=O)₂CF₃] | H | Cl | CH₂OMe | O |
| C-12670 | Me | single bond | H | *c*-Pr[1-S(=O)₂CF₃] | H | Cl | CH₂OMe | O |
| C-12671 | Me(R) | single bond | H | *c*-Pr[1-S(=O)₂CF₃] | H | Cl | CH₂OMe | O |
| C-12672 | Me(S) | single bond | H | *c*-Pr[1-S(=O)₂CF₃] | H | Cl | CH₂OMe | O |
| C-12673 | H | single bond | Me | *c*-Pr[1-S(=O)₂CF₃] | H | Me | CH₂OMe | O |
| C-12674 | Me | single bond | H | *c*-Pr[1-S(=O)₂CF₃] | H | Me | CH₂OMe | O |
| C-12675 | Me(R) | single bond | H | *c*-Pr[1-S(=O)₂CF₃] | H | Me | CH₂OMe | O |
| C-12676 | Me(S) | single bond | H | *c*-Pr[1-S(=O)₂CF₃] | H | Me | CH₂OMe | O |
| C-12677 | H | single bond | Me | *c*-Pr[1-(5,6-dihydro-1,4,2-dioxazin-3-yl)] | H | Cl | CH₂OMe | O |
| C-12678 | Me | single bond | H | *c*-Pr[1-(5,6-dihydro-1,4,2-dioxazin-3-yl)] | H | Cl | CH₂OMe | O |
| C-12679 | Me(R) | single bond | H | *c*-Pr[1-(5,6-dihydro-1,4,2-dioxazin-3-yl)] | H | Cl | CH₂OMe | O |
| C-12680 | Me(S) | single bond | H | *c*-Pr[1-(5,6-dihydro-1,4,2-dioxazin-3-yl)] | H | Cl | CH₂OMe | O |
| C-12681 | H | single bond | Me | *c*-Pr[1-(5,6-dihydro-1,4,2-dioxazin-3-yl)] | H | Me | CH₂OMe | O |
| C-12682 | Me | single bond | H | *c*-Pr[1-(5,6-dihydro-1,4,2-dioxazin-3-yl)] | H | Me | CH₂OMe | O |
| C-12683 | Me(R) | single bond | H | *c*-Pr[1-(5,6-dihydro-1,4,2-dioxazin-3-yl)] | H | Me | CH₂OMe | O |
| C-12684 | Me(S) | single bond | H | *c*-Pr[1-(5,6-dihydro-1,4,2-dioxazin-3-yl)] | H | Me | CH₂OMe | O |
| C-12685 | H | single bond | Me | *c*-Pr[1-(5-hydroxy-1,3,4-oxadiazol-2-yl)] | H | Cl | CH₂OMe | O |
| C-12686 | Me | single bond | H | *c*-Pr[1-(5-hydroxy-1,3,4-oxadiazol-2-yl)] | H | Cl | CH₂OMe | O |
| C-12687 | Me(R) | single bond | H | *c*-Pr[1-(5-hydroxy-1,3,4-oxadiazol-2-yl)] | H | Cl | CH₂OMe | O |
| C-12688 | Me(S) | single bond | H | *c*-Pr[1-(5-hydroxy-1,3,4-oxadiazol-2-yl)] | H | Cl | CH₂OMe | O |
| C-12689 | H | single bond | Me | *c*-Pr[1-(5-hydroxy-1,3,4-oxadiazol-2-yl)] | H | Me | CH₂OMe | O |
| C-12690 | Me | single bond | H | *c*-Pr[1-(5-hydroxy-1,3,4-oxadiazol-2-yl)] | H | Me | CH₂OMe | O |

**[Table 517]**

| Compound Number | Y | W | Z | R¹ | R² | R³ | R¹⁰ | A |
|---|---|---|---|---|---|---|---|---|
| C-12691 | Me(R) | single bond | H | *c*-Pr[1-(5-hydroxy-1,3,4-oxadiazol-2-yl)] | H | Me | CH₂OMe | O |
| C-12692 | Me(S) | single bond | H | *c*-Pr[1-(5-hydroxy-1,3,4-oxadiazol-2-yl)] | H | Me | CH₂OMe | O |
| C-12693 | H | single bond | Me | *c*-Pr[1-(pyrimidin-2-yl)] | H | Cl | CH₂OMe | O |
| C-12694 | Me | single bond | H | *c*-Pr[1-(pyrimidin-2-yl)] | H | Cl | CH₂OMe | O |
| C-12695 | Me(R) | single bond | H | *c*-Pr[1-(pyrimidin-2-yl)] | H | Cl | CH₂OMe | O |
| C-12696 | Me(S) | single bond | H | *c*-Pr[1-(pyrimidin-2-yl)] | H | Cl | CH₂OMe | O |
| C-12697 | H | single bond | Me | *c*-Pr[1-(pyrimidin-2-yl)] | H | Me | CH₂OMe | O |
| C-12698 | Me | single bond | H | *c*-Pr[1-(pyrimidin-2-yl)] | H | Me | CH₂OMe | O |
| C-12699 | Me(R) | single bond | H | *c*-Pr[1-(pyrimidin-2-yl)] | H | Me | CH₂OMe | O |
| C-12700 | Me(S) | single bond | H | *c*-Pr[1-(pyrimidin-2-yl)] | H | Me | CH₂OMe | O |
| C-12701 | H | single bond | Me | *t*-Bu | H | Cl | CH₂OMe | O |
| C-12702 | Me | single bond | H | *t*-Bu | H | Cl | CH₂OMe | O |
| C-12703 | Me(R) | single bond | H | *t*-Bu | H | Cl | CH₂OMe | O |
| C-12704 | Me(S) | single bond | H | *t*-Bu | H | Cl | CH₂OMe | O |
| C-12705 | H | single bond | Me | *t*-Bu | H | Me | CH₂OMe | O |
| C-12706 | Me | single bond | H | *t-*Bu | H | Me | CH₂OMe | O |
| C-12707 | Me(R) | single bond | H | *t*-Bu | H | Me | CH₂OMe | O |
| C-12708 | Me(S) | single bond | H | *t*-Bu | H | Me | CH₂OMe | O |
| C-12709 | H | single bond | Me | *c*-Bu | H | Cl | CH₂OMe | O |
| C-12710 | Me | single bond | H | *c*-Bu | H | Cl | CH₂OMe | O |
| C-12711 | Me(R) | single bond | H | *c*-Bu | H | Cl | CH₂OMe | O |
| C-12712 | Me(S) | single bond | H | *c*-Bu | H | Cl | CH₂OMe | O |
| C-12713 | H | single bond | Me | *c*-Bu | H | Me | CH₂OMe | O |
| C-12714 | Me | single bond | H | *c*-Bu | H | Me | CH₂OMe | O |
| C-12715 | Me(R) | single bond | H | *c*-Bu | H | Me | CH₂OMe | O |
| C-12716 | Me(S) | single bond | H | *c*-Bu | H | Me | CH₂OMe | O |
| C-12717 | H | single bond | Me | *c*-Bu(1-CN) | H | Cl | CH₂OMe | O |
| C-12718 | Me | single bond | H | *c*-Bu(1-CN) | H | Cl | CH₂OMe | O |
| C-12719 | Me(R) | single bond | H | *c*-Bu(1-CN) | H | Cl | CH₂OMe | O |
| C-12720 | Me(S) | single bond | H | *c*-Bu(1-CN) | H | Cl | CH₂OMe | O |
| C-12721 | H | single bond | Me | *c*-Bu(1-CN) | H | Me | CH₂OMe | O |
| C-12722 | Me | single bond | H | *c*-Bu(1-CN) | H | Me | CH₂OMe | O |
| C-12723 | Me(R) | single bond | H | *c*-Bu(1-CN) | H | Me | CH₂OMe | O |
| C-12724 | Me(S) | single bond | H | *c-*Bu(1-CN) | H | Me | CH₂OMe | O |
| C-12725 | H | single bond | Me | *c*-Bu(3,3-F₂) | H | Cl | CH₂OMe | O |
| C-12726 | Me | single bond | H | *c*-Bu(3,3-F₂) | H | Cl | CH₂OMe | O |
| C-12727 | Me(R) | single bond | H | *c*-Bu(3,3-F₂) | H | Cl | CH₂OMe | O |
| C-12728 | Me(S) | single bond | H | *c*-Bu(3,3-F₂) | H | Cl | CH₂OMe | O |
| C-12729 | H | single bond | Me | *c*-Bu(3,3-F₂) | H | Me | CH₂OMe | O |
| C-12730 | Me | single bond | H | *c*-Bu(3,3-F₂) | H | Me | CH₂OMe | O |
| C-12731 | Me(R) | single bond | H | *c*-Bu(3,3-F₂) | H | Me | CH₂OMe | O |
| C-12732 | Me(S) | single bond | H | *c*-Bu(3,3-F₂) | H | Me | CH₂OMe | O |
| C-12733 | H | single bond | Me | oxetan-3-yl | H | Cl | CH₂OMe | O |
| C-12734 | Me | single bond | H | oxetan-3-yl | H | Cl | CH₂OMe | O |

**[Table 518]**

| Compound Number | Y | W | Z | R¹ | R² | R³ | R¹⁰ | A |
|---|---|---|---|---|---|---|---|---|
| C-12735 | Me(R) | single bond | H | oxetan-3-yl | H | Cl | CH₂OMe | O |
| C-12736 | Me(S) | single bond | H | oxetan-3-yl | H | Cl | CH₂OMe | O |
| C-12737 | H | single bond | Me | oxetan-3-yl | H | Me | CH₂OMe | O |
| C-12738 | Me | single bond | H | oxetan-3-yl | H | Me | CH₂OMe | O |
| C-12739 | Me(R) | single bond | H | oxetan-3-yl | H | Me | CH₂OMe | O |
| C-12740 | Me(S) | single bond | H | oxetan-3-yl | H | Me | CH₂OMe | O |
| C-12741 | H | single bond | Me | oxetan(3-CN)-3-yl | H | Cl | CH₂OMe | O |
| C-12742 | Me | single bond | H | oxetan(3-CN)-3-yl | H | Cl | CH₂OMe | O |
| C-12743 | Me(R) | single bond | H | oxetan(3-CN)-3-yl | H | Cl | CH₂OMe | O |
| C-12744 | Me(S) | single bond | H | oxetan(3-CN)-3-yl | H | Cl | CH₂OMe | O |
| C-12745 | H | single bond | Me | oxetan(3-CN)-3-yl | H | Me | CH₂OMe | O |
| C-12746 | Me | single bond | H | oxetan(3-CN)-3-yl | H | Me | CH₂OMe | O |
| C-12747 | Me(R) | single bond | H | oxetan(3-CN)-3-yl | H | Me | CH₂OMe | O |
| C-12748 | Me(S) | single bond | H | oxetan(3-CN)-3-yl | H | Me | CH₂OMe | O |
| C-12749 | H | single bond | Me | azetidin[1-C(=0)Me]-3-yl | H | Cl | CH₂OMe | O |
| C-12750 | Me | single bond | H | azetidin[1-C(=O)Me]-3-yl | H | Cl | CH₂OMe | O |
| C-12751 | Me(R) | single bond | H | azetidin[1-C(=O)Me]-3-yl | H | Cl | CH₂OMe | O |
| C-12752 | Me(S) | single bond | H | azetidin[1-C(=O)Me]-3-yl | H | Cl | CH₂OMe | O |
| C-12753 | H | single bond | Me | azetidin[1-C(=O)Me]-3-yl | H | Me | CH₂OMe | O |
| C-12754 | Me | single bond | H | azetidin[1-C(=O)Me]-3-yl | H | Me | CH₂OMe | O |
| C-12755 | Me(R) | single bond | H | azetidin[1-C(=O)Me]-3-yl | H | Me | CH₂OMe | O |
| C-12756 | Me(S) | single bond | H | azetidin[1-C(=O)Me]-3-yl | H | Me | CH₂OMe | O |
| C-12757 | H | single bond | Me | azetidin[1-S(=O)₂Me]-3-yl | H | Cl | CH₂OMe | O |
| C-12758 | Me | single bond | H | azetidin[1-S(=O)₂Me]-3-yl | H | Cl | CH₂OMe | O |
| C-12759 | Me(R) | single bond | H | azetidin[1-S(=O)₂Me]-3-yl | H | Cl | CH₂OMe | O |
| C-12760 | Me(S) | single bond | H | azetidin[1-S(=O)₂Me]-3-yl | H | Cl | CH₂OMe | O |
| C-12761 | H | single bond | Me | azetidin[1-S(=O)₂Me]-3-yl | H | Me | CH₂OMe | O |
| C-12762 | Me | single bond | H | azetidin[1-S(=O)₂Me]-3-yl | H | Me | CH₂OMe | O |
| C-12763 | Me(R) | single bond | H | azetidin[1-S(=O)₂Me]-3-yl | H | Me | CH₂OMe | O |
| C-12764 | Me(S) | single bond | H | azetidin[1-S(=O)₂Me]-3-yl | H | Me | CH₂OMe | O |
| C-12765 | H | single bond | Me | azetidin[1-S(=O)₂CHF₂]-3-yl | H | Cl | CH₂OMe | O |
| C-12766 | Me | single bond | H | azetidin[1-S(=O)₂CHF₂]-3-yl | H | Cl | CH₂OMe | O |
| C-12767 | Me(R) | single bond | H | azetidin[1-S(=O)₂CHF₂]-3-yl | H | Cl | CH₂OMe | O |
| C-12768 | Me(S) | single bond | H | azetidin[1-S(=O)₂CHF₂]-3-yl | H | Cl | CH₂OMe | O |
| C-12769 | H | single bond | Me | azetidin[1-S(=O)₂CHF₂]-3-yl | H | Me | CH₂OMe | O |
| C-12770 | Me | single bond | H | azetidin[1-S(=O)₂CHF₂]-3-yl | H | Me | CH₂OMe | O |
| C-12771 | Me(R) | single bond | H | azetidin[1-S(=O)₂CHF₂]-3-yl | H | Me | CH₂OMe | O |
| C-12772 | Me(S) | single bond | H | azetidin[1-S(=O)₂CHF₂]-3-yl | H | Me | CH₂OMe | O |
| C-12773 | H | single bond | Me | *c*-Pen | H | Cl | CH₂OMe | O |
| C-12774 | Me | single bond | H | *c*-Pen | H | Cl | CH₂OMe | O |
| C-12775 | Me(R) | single bond | H | *c*-Pen | H | Cl | CH₂OMe | O |
| C-12776 | Me(S) | single bond | H | *c*-Pen | H | Cl | CH₂OMe | O |
| C-12777 | H | single bond | Me | *c*-Pen | H | Me | CH₂OMe | O |
| C-12778 | Me | single bond | H | *c*-Pen | H | Me | CH₂OMe | O |

**[Table 519]**

| Compound Number | Y | W | Z | R¹ | R² | R³ | R¹⁰ | A |
|---|---|---|---|---|---|---|---|---|
| C-12779 | Me(R) | single bond | H | *c*-Pen | H | Me | CH₂OMe | O |
| C-12780 | Me(S) | single bond | H | *c*-Pen | H | Me | CH₂OMe | O |
| C-12781 | H | single bond | Me | tetrahydrofuran-3-yl | H | Cl | CH₂OMe | O |
| C-12782 | Me | single bond | H | tetrahydrofuran-3-yl | H | Cl | CH₂OMe | O |
| C-12783 | Me(R) | single bond | H | tetrahydrofuran-3-yl | H | Cl | CH₂OMe | O |
| C-12784 | Me(S) | single bond | H | tetrahydrofuran-3-yl | H | Cl | CH₂OMe | O |
| C-12785 | H | single bond | Me | tetrahydrofuran-3-yl | H | Me | CH₂OMe | O |
| C-12786 | Me | single bond | H | tetrahydrofuran-3-yl | H | Me | CH₂OMe | O |
| C-12787 | Me(R) | single bond | H | tetrahydrofuran-3-yl | H | Me | CH₂OMe | O |
| C-12788 | Me(S) | single bond | H | tetrahydrofuran-3-yl | H | Me | CH₂OMe | O |
| C-12789 | H | single bond | Me | tetrahydrothiophen-3-yl | H | Cl | CH₂OMe | O |
| C-12790 | Me | single bond | H | tetrahydrothiophen-3-yl | H | Cl | CH₂OMe | O |
| C-12791 | Me(R) | single bond | H | tetrahydrothiophen-3-yl | H | Cl | CH₂OMe | O |
| C-12792 | Me(S) | single bond | H | tetrahydrothiophen-3-yl | H | Cl | CH₂OMe | O |
| C-12793 | H | single bond | Me | tetrahydrothiophen-3-yl | H | Me | CH₂OMe | O |
| C-12794 | Me | single bond | H | tetrahydrothiophen-3-yl | H | Me | CH₂OMe | O |
| C-12795 | Me(R) | single bond | H | tetrahydrothiophen-3-yl | H | Me | CH₂OMe | O |
| C-12796 | Me(S) | single bond | H | tetrahydrothiophen-3-yl | H | Me | CH₂OMe | O |
| C-12797 | H | single bond | Me | *c*-Pen(1-CN) | H | Cl | CH₂OMe | O |
| C-12798 | Me | single bond | H | *c*-Pen(1-CN) | H | Cl | CH₂OMe | O |
| C-12799 | Me(R) | single bond | H | *c*-Pen(1-CN) | H | Cl | CH₂OMe | O |
| C-12800 | Me(S) | single bond | H | *c*-Pen(1-CN) | H | Cl | CH₂OMe | O |
| C-12801 | H | single bond | Me | *c*-Pen(1-CN) | H | Me | CH₂OMe | O |
| C-12802 | Me | single bond | H | *c*-Pen(1-CN) | H | Me | CH₂OMe | O |
| C-12803 | Me(R) | single bond | H | *c*-Pen(1-CN) | H | Me | CH₂OMe | O |
| C-12804 | Me(S) | single bond | H | *c*-Pen(1-CN) | H | Me | CH₂OMe | O |
| C-12805 | H | single bond | Me | tetrahydrofuran(3-CN)-3-yl | H | Cl | CH₂OMe | O |
| C-12806 | Me | single bond | H | tetrahydrofuran(3-CN)-3-yl | H | Cl | CH₂OMe | O |
| C-12807 | Me(R) | single bond | H | tetrahydrofuran(3-CN)-3-yl | H | Cl | CH₂OMe | O |
| C-12808 | Me(S) | single bond | H | tetrahydrofuran(3-CN)-3-yl | H | Cl | CH₂OMe | O |
| C-12809 | H | single bond | Me | tetrahydrofuran(3-CN)-3-yl | H | Me | CH₂OMe | O |
| C-12810 | Me | single bond | H | tetrahydrofuran(3-CN)-3-yl | H | Me | CH₂OMe | O |
| C-12811 | Me(R) | single bond | H | tetrahydrofuran(3-CN)-3-yl | H | Me | CH₂OMe | O |
| C-12812 | Me(S) | single bond | H | tetrahydrofuran(3-CN)-3-yl | H | Me | CH₂OMe | O |
| C-12813 | H | single bond | Me | tetrahydrothiophen(3-CN)-3-yl | H | Cl | CH₂OMe | O |
| C-12814 | Me | single bond | H | tetrahydrothiophen(3-CN)-3-yl | H | Cl | CH₂OMe | O |
| C-12815 | Me(R) | single bond | H | tetrahydrothiophen(3-CN)-3-yl | H | Cl | CH₂OMe | O |
| C-12816 | Me(S) | single bond | H | tetrahydrothiophen(3-CN)-3-yl | H | Cl | CH₂OMe | O |
| C-12817 | H | single bond | Me | tetrahydrothiophen(3-CN)-3-yl | H | Me | CH₂OMe | O |
| C-12818 | Me | single bond | H | tetrahydrothiophen(3-CN)-3-yl | H | Me | CH₂OMe | O |
| C-12819 | Me(R) | single bond | H | tetrahydrothiophen(3-CN)-3-yl | H | Me | CH₂OMe | O |
| C-12820 | Me(S) | single bond | H | tetrahydrothiophen(3-CN)-3-yl | H | Me | CH₂OMe | O |
| C-12821 | H | single bond | Me | bicyclo[1.1.1]pentan-1-yl | H | Cl | CH₂OMe | O |
| C-12822 | Me | single bond | H | bicyclo[1.1.1]pentan-1-yl | H | Cl | CH₂OMe | O |

**[Table 520]**

| Compound Number | Y | W | Z | R¹ | R² | R³ | R¹⁰ | A |
|---|---|---|---|---|---|---|---|---|
| C-12823 | Me(R) | single bond | H | bicyclo[1.1.1]pentan-1-yl | H | Cl | CH₂OMe | O |
| C-12824 | Me(S) | single bond | H | bicyclo[1.1.1]pentan-1-yl | H | Cl | CH₂OMe | O |
| C-12825 | H | single bond | Me | bicyclo[1.1.1]pentan-1-yl | H | Me | CH₂OMe | O |
| C-12826 | Me | single bond | H | bicyclo[1.1.1]pentan-1-yl | H | Me | CH₂OMe | O |
| C-12827 | Me(R) | single bond | H | bicyclo[1.1.1]pentan-1-yl | H | Me | CH₂OMe | O |
| C-12828 | Me(S) | single bond | H | bicyclo[1.1.1]pentan-1-yl | H | Me | CH₂OMe | O |
| C-12829 | H | single bond | Me | *c*-Hex | H | Cl | CH₂OMe | O |
| C-12830 | Me | single bond | H | *c*-Hex | H | Cl | CH₂OMe | O |
| C-12831 | Me(R) | single bond | H | *c*-Hex | H | Cl | CH₂OMe | O |
| C-12832 | Me(S) | single bond | H | *c*-Hex | H | Cl | CH₂OMe | O |
| C-12833 | H | single bond | Me | *c*-Hex | H | Me | CH₂OMe | O |
| C-12834 | Me | single bond | H | *c*-Hex | H | Me | CH₂OMe | O |
| C-12835 | Me(R) | single bond | H | *c*-Hex | H | Me | CH₂OMe | O |
| C-12836 | Me(S) | single bond | H | *c*-Hex | H | Me | CH₂OMe | O |
| C-12837 | H | single bond | Me | tetrahydropyran-4-yl | H | Cl | CH₂OMe | O |
| C-12838 | Me | single bond | H | tetrahydropyran-4-yl | H | Cl | CH₂OMe | O |
| C-12839 | Me(R) | single bond | H | tetrahydropyran-4-yl | H | Cl | CH₂OMe | O |
| C-12840 | Me(S) | single bond | H | tetrahydropyran-4-yl | H | Cl | CH₂OMe | O |
| C-12841 | H | single bond | Me | tetrahydropyran-4-yl | H | Me | CH₂OMe | O |
| C-12842 | Me | single bond | H | tetrahydropyran-4-yl | H | Me | CH₂OMe | O |
| C-12843 | Me(R) | single bond | H | tetrahydropyran-4-yl | H | Me | CH₂OMe | O |
| C-12844 | Me(S) | single bond | H | tetrahydropyran-4-yl | H | Me | CH₂OMe | O |
| C-12845 | H | single bond | Me | tetrahydrothiopyran-4-yl | H | Cl | CH₂OMe | O |
| C-12846 | Me | single bond | H | tetrahydrothiopyran-4-yl | H | Cl | CH₂OMe | O |
| C-12847 | Me(R) | single bond | H | tetrahydrothiopyran-4-yl | H | Cl | CH₂OMe | O |
| C-12848 | Me(S) | single bond | H | tetrahydrothiopyran-4-yl | H | Cl | CH₂OMe | O |
| C-12849 | H | single bond | Me | tetrahydrothiopyran-4-yl | H | Me | CH₂OMe | O |
| C-12850 | Me | single bond | H | tetrahydrothiopyran-4-yl | H | Me | CH₂OMe | O |
| C-12851 | Me(R) | single bond | H | tetrahydrothiopyran-4-yl | H | Me | CH₂OMe | O |
| C-12852 | Me(S) | single bond | H | tetrahydrothiopyran-4-yl | H | Me | CH₂OMe | O |
| C-12853 | H | single bond | Me | *c*-Hex(1-CN) | H | Cl | CH₂OMe | O |
| C-12854 | Me | single bond | H | *c*-Hex(1-CN) | H | Cl | CH₂OMe | O |
| C-12855 | Me(R) | single bond | H | *c*-Hex(1-CN) | H | Cl | CH₂OMe | O |
| C-12856 | Me(S) | single bond | H | *c*-Hex(1-CN) | H | Cl | CH₂OMe | O |
| C-12857 | H | single bond | Me | *c*-Hex(1-CN) | H | Me | CH₂OMe | O |
| C-12858 | Me | single bond | H | *c*-Hex(1-CN) | H | Me | CH₂OMe | O |
| C-12859 | Me(R) | single bond | H | *c*-Hex(1-CN) | H | Me | CH₂OMe | O |
| C-12860 | Me(S) | single bond | H | *c*-Hex(1-CN) | H | Me | CH₂OMe | O |
| C-12861 | H | single bond | Me | tetrahydropyran(4-CN)-4-yl | H | Cl | CH₂OMe | O |
| C-12862 | Me | single bond | H | tetrahydropyran(4-CN)-4-yl | H | Cl | CH₂OMe | O |
| C-12863 | Me(R) | single bond | H | tetrahydropyran(4-CN)-4-yl | H | Cl | CH₂OMe | O |
| C-12864 | Me(S) | single bond | H | tetrahydropyran(4-CN)-4-yl | H | Cl | CH₂OMe | O |
| C-12865 | H | single bond | Me | tetrahydropyran(4-CN)-4-yl | H | Me | CH₂OMe | O |
| C-12866 | Me | single bond | H | tetrahydropyran(4-CN)-4-yl | H | Me | CH₂OMe | O |

**[Table 521]**

| Compound Number | Y | W | Z | R¹ | R² | R³ | R¹⁰ | A |
|---|---|---|---|---|---|---|---|---|
| C-12867 | Me(R) | single bond | H | tetrahydropyran(4-CN)-4-yl | H | Me | CH₂OMe | O |
| C-12868 | Me(S) | single bond | H | tetrahydropyran(4-CN)-4-yl | H | Me | CH₂OMe | O |
| C-12869 | H | single bond | Me | tetrahydrothiopyran(4-CN)-4-yl | H | Cl | CH₂OMe | O |
| C-12870 | Me | single bond | H | tetrahydrothiopyran(4-CN)-4-yl | H | Cl | CH₂OMe | O |
| C-12871 | Me(R) | single bond | H | tetrahydrothiopyran(4-CN)-4-yl | H | Cl | CH₂OMe | O |
| C-12872 | Me(S) | single bond | H | tetrahydrothiopyran(4-CN)-4-yl | H | Cl | CH₂OMe | O |
| C-12873 | H | single bond | Me | tetrahydrothiopyran(4-CN)-4-yl | H | Me | CH₂OMe | O |
| C-12874 | Me | single bond | H | tetrahydrothiopyran(4-CN)-4-yl | H | Me | CH₂OMe | O |
| C-12875 | Me(R) | single bond | H | tetrahydrothiopyran(4-CN)-4-yl | H | Me | CH₂OMe | O |
| C-12876 | Me(S) | single bond | H | tetrahydrothiopyran(4-CN)-4-yl | H | Me | CH₂OMe | O |
| C-12877 | H | single bond | Me | CH₂(*c*-Pr) | H | Cl | CH₂OMe | O |
| C-12878 | Me | single bond | H | CH₂(*c*-Pr) | H | Cl | CH₂OMe | O |
| C-12879 | Me(R) | single bond | H | CH₂(*c*-Pr) | H | Cl | CH₂OMe | O |
| C-12880 | Me(S) | single bond | H | CH₂(*c*-Pr) | H | Cl | CH₂OMe | O |
| C-12881 | H | single bond | Me | CH₂(*c*-Pr) | H | Me | CH₂OMe | O |
| C-12882 | Me | single bond | H | CH₂(*c*-Pr) | H | Me | CH₂OMe | O |
| C-12883 | Me(R) | single bond | H | CH₂(*c*-Pr) | H | Me | CH₂OMe | O |
| C-12884 | Me(S) | single bond | H | CH₂(*c*-Pr) | H | Me | CH₂OMe | O |
| C-12885 | H | single bond | Me | CH₂[*c*-Pr(1-CN)] | H | Cl | CH₂OMe | O |
| C-12886 | Me | single bond | H | CH₂[*c*-Pr(1-CN)] | H | Cl | CH₂OMe | O |
| C-12887 | Me(R) | single bond | H | CH₂[*c*-Pr(1-CN)] | H | Cl | CH₂OMe | O |
| C-12888 | Me(S) | single bond | H | CH₂[*c*-Pr(1-CN)] | H | Cl | CH₂OMe | O |
| C-12889 | H | single bond | Me | CH₂[*c*-Pr(1-CN)] | H | Me | CH₂OMe | O |
| C-12890 | Me | single bond | H | CH₂[*c*-Pr(1-CN)] | H | Me | CH₂OMe | O |
| C-12891 | Me(R) | single bond | H | CH₂[*c*-Pr(1-CN)] | H | Me | CH₂OMe | O |
| C-12892 | Me(S) | single bond | H | CH₂[*c*-Pr(1-CN)] | H | Me | CH₂OMe | O |
| C-12893 | H | single bond | Me | CH(Me)(*c*-Pr) | H | Cl | CH₂OMe | O |
| C-12894 | Me | single bond | H | CH(Me)(*c*-Pr) | H | Cl | CH₂OMe | O |
| C-12895 | Me(R) | single bond | H | CH(Me)(*c*-Pr) | H | Cl | CH₂OMe | O |
| C-12896 | Me(S) | single bond | H | CH(Me)(*c*-Pr) | H | Cl | CH₂OMe | O |
| C-12897 | H | single bond | Me | CH(Me)(*c*-Pr) | H | Me | CH₂OMe | O |
| C-12898 | Me | single bond | H | CH(Me)(*c*-Pr) | H | Me | CH₂OMe | O |
| C-12899 | Me(R) | single bond | H | CH(Me)(*c*-Pr) | H | Me | CH₂OMe | O |
| C-12900 | Me(S) | single bond | H | CH(Me)(*c*-Pr) | H | Me | CH₂OMe | O |
| C-12901 | H | single bond | Me | CH(Me)CH₂OMe | H | Cl | CH₂OMe | O |
| C-12902 | Me | single bond | H | CH(Me)CH₂OMe | H | Cl | CH₂OMe | O |
| C-12903 | Me(R) | single bond | H | CH(Me)CH₂OMe | H | Cl | CH₂OMe | O |
| C-12904 | Me(S) | single bond | H | CH(Me)CH₂OMe | H | Cl | CH₂OMe | O |
| C-12905 | H | single bond | Me | CH(Me)CH₂OMe | H | Me | CH₂OMe | O |
| C-12906 | Me | single bond | H | CH(Me)CH₂OMe | H | Me | CH₂OMe | O |
| C-12907 | Me(R) | single bond | H | CH(Me)CH₂OMe | H | Me | CH₂OMe | O |
| C-12908 | Me(S) | single bond | H | CH(Me)CH₂OMe | H | Me | CH₂OMe | O |
| C-12909 | H | single bond | Me | CH₂CH=CH₂ | H | Cl | CH₂OMe | O |
| C-12910 | Me | single bond | H | CH₂CH=CH₂ | H | Cl | CH₂OMe | O |

**[Table 522]**

| Compound Number | Y | W | Z | R¹ | R² | R³ | R¹⁰ | A |
|---|---|---|---|---|---|---|---|---|
| C-12911 | Me(R) | single bond | H | CH₂CH=CH₂ | H | Cl | CH₂OMe | O |
| C-12912 | Me(S) | single bond | H | CH₂CH=CH₂ | H | Cl | CH₂OMe | O |
| C-12913 | H | single bond | Me | CH₂CH=CH₂ | H | Me | CH₂OMe | O |
| C-12914 | Me | single bond | H | CH₂CH=CH₂ | H | Me | CH₂OMe | O |
| C-12915 | Me(R) | single bond | H | CH₂CH=CH₂ | H | Me | CH₂OMe | O |
| C-12916 | Me(S) | single bond | H | CH₂CH=CH₂ | H | Me | CH₂OMe | O |
| C-12917 | H | single bond | Me | CH₂C≡CH | H | Cl | CH₂OMe | O |
| C-12918 | Me | single bond | H | CH₂C≡CH | H | Cl | CH₂OMe | O |
| C-12919 | Me(R) | single bond | H | CH₂C≡CH | H | Cl | CH₂OMe | O |
| C-12920 | Me(S) | single bond | H | CH₂C≡CH | H | Cl | CH₂OMe | O |
| C-12921 | H | single bond | Me | CH₂C≡CH | H | Me | CH₂OMe | O |
| C-12922 | Me | single bond | H | CH₂C≡CH | H | Me | CH₂OMe | O |
| C-12923 | Me(R) | single bond | H | CH₂C≡CH | H | Me | CH₂OMe | O |
| C-12924 | Me(S) | single bond | H | CH₂C≡CH | H | Me | CH₂OMe | O |
| C-12925 | H | single bond | Me | CH₂CN | H | Cl | CH₂OMe | O |
| C-12926 | Me | single bond | H | CH₂CN | H | Cl | CH₂OMe | O |
| C-12927 | Me(R) | single bond | H | CH₂CN | H | Cl | CH₂OMe | O |
| C-12928 | Me(S) | single bond | H | CH₂CN | H | Cl | CH₂OMe | O |
| C-12929 | H | single bond | Me | CH₂CN | H | Me | CH₂OMe | O |
| C-12930 | Me | single bond | H | CH₂CN | H | Me | CH₂OMe | O |
| C-12931 | Me(R) | single bond | H | CH₂CN | H | Me | CH₂OMe | O |
| C-12932 | Me(S) | single bond | H | CH₂CN | H | Me | CH₂OMe | O |
| C-12933 | H | single bond | Me | CH(Me)CN | H | Cl | CH₂OMe | O |
| C-12934 | Me | single bond | H | CH(Me)CN | H | Cl | CH₂OMe | O |
| C-12935 | Me(R) | single bond | H | CH(Me)CN | H | Cl | CH₂OMe | O |
| C-12936 | Me(S) | single bond | H | CH(Me)CN | H | Cl | CH₂OMe | O |
| C-12937 | H | single bond | Me | CH(Me)CN | H | Me | CH₂OMe | O |
| C-12938 | Me | single bond | H | CH(Me)CN | H | Me | CH₂OMe | O |
| C-12939 | Me(R) | single bond | H | CH(Me)CN | H | Me | CH₂OMe | O |
| C-12940 | Me(S) | single bond | H | CH(Me)CN | H | Me | CH₂OMe | O |
| C-12941 | H | single bond | Me | C(Me)₂CN | H | Cl | CH₂OMe | O |
| C-12942 | Me | single bond | H | C(Me)₂CN | H | Cl | CH₂OMe | O |
| C-12943 | Me(R) | single bond | H | C(Me)₂CN | H | Cl | CH₂OMe | O |
| C-12944 | Me(S) | single bond | H | C(Me)₂CN | H | Cl | CH₂OMe | O |
| C-12945 | H | single bond | Me | C(Me)₂CN | H | Me | CH₂OMe | O |
| C-12946 | Me | single bond | H | C(Me)₂CN | H | Me | CH₂OMe | O |
| C-12947 | Me(R) | single bond | H | C(Me)₂CN | H | Me | CH₂OMe | O |
| C-12948 | Me(S) | single bond | H | C(Me)₂CN | H | Me | CH₂OMe | O |
| C-12949 | H | single bond | Me | C(Me)(*i*-Pr)CN | H | Cl | CH₂OMe | O |
| C-12950 | Me | single bond | H | C(Me)(*i*-Pr)CN | H | Cl | CH₂OMe | O |
| C-12951 | Me(R) | single bond | H | C(Me)(*i*-Pr)CN | H | Cl | CH₂OMe | O |
| C-12952 | Me(S) | single bond | H | C(Me)(*i*-Pr)CN | H | Cl | CH₂OMe | O |
| C-12953 | H | single bond | Me | C(Me)(*i*-Pr)CN | H | Me | CH₂OMe | O |
| C-12954 | Me | single bond | H | C(Me)(*i*-Pr)CN | H | Me | CH₂OMe | O |

**[Table 523]**

| Compound Number | Y | W | Z | R¹ | R² | R³ | R¹⁰ | A |
|---|---|---|---|---|---|---|---|---|
| C-12955 | Me(R) | single bond | H | C(Me)(*i*-Pr)CN | H | Me | CH₂OMe | O |
| C-12956 | Me(S) | single bond | H | C(Me)(*i*-Pr)CN | H | Me | CH₂OMe | O |
| C-12957 | H | single bond | Me | OMe | H | Cl | CH₂OMe | O |
| C-12958 | Me | single bond | H | OMe | H | Cl | CH₂OMe | O |
| C-12959 | Me(R) | single bond | H | OMe | H | Cl | CH₂OMe | O |
| C-12960 | Me(S) | single bond | H | OMe | H | Cl | CH₂OMe | O |
| C-12961 | H | single bond | Me | OMe | H | Me | CH₂OMe | O |
| C-12962 | Me | single bond | H | OMe | H | Me | CH₂OMe | O |
| C-12963 | Me(R) | single bond | H | OMe | H | Me | CH₂OMe | O |
| C-12964 | Me(S) | single bond | H | OMe | H | Me | CH₂OMe | O |
| C-12965 | H | single bond | Me | CH=NOMe | H | Cl | CH₂OMe | O |
| C-12966 | Me | single bond | H | CH=NOMe | H | Cl | CH₂OMe | O |
| C-12967 | Me(R) | single bond | H | CH=NOMe | H | Cl | CH₂OMe | O |
| C-12968 | Me(S) | single bond | H | CH=NOMe | H | Cl | CH₂OMe | O |
| C-12969 | H | single bond | Me | CH=NOMe | H | Me | CH₂OMe | O |
| C-12970 | Me | single bond | H | CH=NOMe | H | Me | CH₂OMe | O |
| C-12971 | Me(R) | single bond | H | CH=NOMe | H | Me | CH₂OMe | O |
| C-12972 | Me(S) | single bond | H | CH=NOMe | H | Me | CH₂OMe | O |
| C-12973 | H | single bond | Me | morpholin-4-yl | H | Cl | CH₂OMe | O |
| C-12974 | Me | single bond | H | morpholin-4-yl | H | Cl | CH₂OMe | O |
| C-12975 | Me(R) | single bond | H | morpholin-4-yl | H | Cl | CH₂OMe | O |
| C-12976 | Me(S) | single bond | H | morpholin-4-yl | H | Cl | CH₂OMe | O |
| C-12977 | H | single bond | Me | morpholin-4-yl | H | Me | CH₂OMe | O |
| C-12978 | Me | single bond | H | morpholin-4-yl | H | Me | CH₂OMe | O |
| C-12979 | Me(R) | single bond | H | morpholin-4-yl | H | Me | CH₂OMe | O |
| C-12980 | Me(S) | single bond | H | morpholin-4-yl | H | Me | CH₂OMe | O |
| C-12981 | H | single bond | Me | Ph | H | Cl | CH₂OMe | O |
| C-12982 | Me | single bond | H | Ph | H | Cl | CH₂OMe | O |
| C-12983 | Me(R) | single bond | H | Ph | H | Cl | CH₂OMe | O |
| C-12984 | Me(S) | single bond | H | Ph | H | Cl | CH₂OMe | O |
| C-12985 | H | single bond | Me | Ph | H | Me | CH₂OMe | O |
| C-12986 | Me | single bond | H | Ph | H | Me | CH₂OMe | O |
| C-12987 | Me(R) | single bond | H | Ph | H | Me | CH₂OMe | O |
| C-12988 | Me(S) | single bond | H | Ph | H | Me | CH₂OMe | O |
| C-12989 | H | single bond | Me | CH₂Ph | H | Cl | CH₂OMe | O |
| C-12990 | Me | single bond | H | CH₂Ph | H | Cl | CH₂OMe | O |
| C-12991 | Me(R) | single bond | H | CH₂Ph | H | Cl | CH₂OMe | O |
| C-12992 | Me(S) | single bond | H | CH₂Ph | H | Cl | CH₂OMe | O |
| C-12993 | H | single bond | Me | CH₂Ph | H | Me | CH₂OMe | O |
| C-12994 | Me | single bond | H | CH₂Ph | H | Me | CH₂OMe | O |
| C-12995 | Me(R) | single bond | H | CH₂Ph | H | Me | CH₂OMe | O |
| C-12996 | Me(S) | single bond | H | CH₂Ph | H | Me | CH₂OMe | O |
| C-12997 | H | single bond | Me | thiazole-2-yl | H | Cl | CH₂OMe | O |
| C-12998 | Me | single bond | H | thiazole-2-yl | H | Cl | CH₂OMe | O |

**[Table 524]**

| Compound Number | Y | W | Z | R¹ | R² | R³ | R¹⁰ | A |
|---|---|---|---|---|---|---|---|---|
| C-12999 | Me(R) | single bond | H | thiazole-2-yl | H | Cl | CH₂OMe | O |
| C-13000 | Me(S) | single bond | H | thiazole-2-yl | H | Cl | CH₂OMe | O |
| C-13001 | H | single bond | Me | thiazole-2-yl | H | Me | CH₂OMe | O |
| C-13002 | Me | single bond | H | thiazole-2-yl | H | Me | CH₂OMe | O |
| C-13003 | Me(R) | single bond | H | thiazole-2-yl | H | Me | CH₂OMe | O |
| C-13004 | Me(S) | single bond | H | thiazole-2-yl | H | Me | CH₂OMe | O |
| C-13005 | H | single bond | Me | 1-Me-pyrazol-3-yl | H | Cl | CH₂OMe | O |
| C-13006 | Me | single bond | H | 1-Me-pyrazol-3-yl | H | Cl | CH₂OMe | O |
| C-13007 | Me(R) | single bond | H | 1-Me-pyrazol-3-yl | H | Cl | CH₂OMe | O |
| C-13008 | Me(S) | single bond | H | 1-Me-pyrazol-3-yl | H | Cl | CH₂OMe | O |
| C-13009 | H | single bond | Me | 1-Me-pyrazol-3-yl | H | Me | CH₂OMe | O |
| C-13010 | Me | single bond | H | 1-Me-pyrazol-3-yl | H | Me | CH₂OMe | O |
| C-13011 | Me(R) | single bond | H | 1-Me-pyrazol-3-yl | H | Me | CH₂OMe | O |
| C-13012 | Me(S) | single bond | H | 1-Me-pyrazol-3-yl | H | Me | CH₂OMe | O |
| C-13013 | H | single bond | Me | 1-Me-pyrazol-5-yl | H | Cl | CH₂OMe | O |
| C-13014 | Me | single bond | H | 1-Me-pyrazol-5-yl | H | Cl | CH₂OMe | O |
| C-13015 | Me(R) | single bond | H | 1-Me-pyrazol-5-yl | H | Cl | CH₂OMe | O |
| C-13016 | Me(S) | single bond | H | 1-Me-pyrazol-5-yl | H | Cl | CH₂OMe | O |
| C-13017 | H | single bond | Me | 1-Me-pyrazol-5-yl | H | Me | CH₂OMe | O |
| C-13018 | Me | single bond | H | 1-Me-pyrazol-5-yl | H | Me | CH₂OMe | O |
| C-13019 | Me(R) | single bond | H | 1-Me-pyrazol-5-yl | H | Me | CH₂OMe | O |
| C-13020 | Me(S) | single bond | H | 1-Me-pyrazol-5-yl | H | Me | CH₂OMe | O |
| C-13021 | H | single bond | Me | CH₂(pyrimidin-2-yl) | H | Cl | CH₂OMe | O |
| C-13022 | Me | single bond | H | CH₂(pyrimidin-2-yl) | H | Cl | CH₂OMe | O |
| C-13023 | Me(R) | single bond | H | CH₂(pyrimidin-2-yl) | H | Cl | CH₂OMe | O |
| C-13024 | Me(S) | single bond | H | CH₂(pyrimidin-2-yl) | H | Cl | CH₂OMe | O |
| C-13025 | H | single bond | Me | CH₂(pyrimidin-2-yl) | H | Me | CH₂OMe | O |
| C-13026 | Me | single bond | H | CH₂(pyrimidin-2-yl) | H | Me | CH₂OMe | O |
| C-13027 | Me(R) | single bond | H | CH₂(pyrimidin-2-yl) | H | Me | CH₂OMe | O |
| C-13028 | Me(S) | single bond | H | CH₂(pyrimidin-2-yl) | H | Me | CH₂OMe | O |
| C-13029 | H | single bond | Me | -CH₂CH₂CH₂- | | Cl | CH₂OMe | O |
| C-13030 | Me | single bond | H | -CH₂CH₂CH₂- | | Cl | CH₂OMe | O |
| C-13031 | Me(R) | single bond | H | -CH₂CH₂CH₂- | | Cl | CH₂OMe | O |
| C-13032 | Me(S) | single bond | H | -CH₂CH₂CH₂- | | Cl | CH₂OMe | O |
| C-13033 | H | single bond | Me | -CH₂CH₂CH₂- | | Me | CH₂OMe | O |
| C-13034 | Me | single bond | H | -CH₂CH₂CH₂- | | Me | CH₂OMe | O |
| C-13035 | Me(R) | single bond | H | -CH₂CH₂CH₂- | | Me | CH₂OMe | O |
| C-13036 | Me(S) | single bond | H | -CH₂CH₂CH₂- | | Me | CH₂OMe | O |
| C-13037 | H | single bond | Me | -CH₂CH₂OCH₂CH₂- | | Cl | CH₂OMe | O |
| C-13038 | Me | single bond | H | -CH₂CH₂OCH₂CH₂- | | Cl | CH₂OMe | O |
| C-13039 | Me(R) | single bond | H | -CH₂CH₂OCH₂CH₂- | | Cl | CH₂OMe | O |
| C-13040 | Me(S) | single bond | H | -CH₂CH₂OCH₂CH₂- | | Cl | CH₂OMe | O |
| C-13041 | H | single bond | Me | -CH₂CH₂OCH₂CH₂- | | Me | CH₂OMe | O |
| C-13042 | Me | single bond | H | -CH₂CH₂OCH₂CH₂- | | Me | CH₂OMe | O |

**[Table 525]**

| Compound Number | Y | W | Z | R¹ | R² | R³ | R¹⁰ | A |
|---|---|---|---|---|---|---|---|---|
| C-13043 | Me(R) | single bond | H | -CH₂CH₂OCH₂CH₂- | | Me | CH₂OMe | O |
| C-13044 | Me(S) | single bond | H | -CH₂CH₂OCH₂CH₂- | | Me | CH₂OMe | O |
| C-13045 | H | single bond | Me | Me | H | Cl | C(=O)Me | O |
| C-13046 | Me | single bond | H | Me | H | Cl | C(=O)Me | O |
| C-13047 | Me(R) | single bond | H | Me | H | Cl | C(=O)Me | O |
| C-13048 | Me(S) | single bond | H | Me | H | Cl | C(=O)Me | O |
| C-13049 | H | single bond | Me | Me | H | Me | C(=O)Me | O |
| C-13050 | Me | single bond | H | Me | H | Me | C(=O)Me | O |
| C-13051 | Me(R) | single bond | H | Me | H | Me | C(=O)Me | O |
| C-13052 | Me(S) | single bond | H | Me | H | Me | C(=O)Me | O |
| C-13053 | H | single bond | Me | CH₂CHF₂ | H | Cl | C(=O)Me | O |
| C-13054 | Me | single bond | H | CH₂CHF₂ | H | Cl | C(=O)Me | O |
| C-13055 | Me(R) | single bond | H | CH₂CHF₂ | H | Cl | C(=O)Me | O |
| C-13056 | Me(S) | single bond | H | CH₂CHF₂ | H | Cl | C(=O)Me | O |
| C-13057 | H | single bond | Me | CH₂CHF₂ | H | Me | C(=O)Me | O |
| C-13058 | Me | single bond | H | CH₂CHF₂ | H | Me | C(=O)Me | O |
| C-13059 | Me(R) | single bond | H | CH₂CHF₂ | H | Me | C(=O)Me | O |
| C-13060 | Me(S) | single bond | H | CH₂CHF₂ | H | Me | C(=O)Me | O |
| C-13061 | H | single bond | Me | *n*-Pr | H | Cl | C(=O)Me | O |
| C-13062 | Me | single bond | H | *n*-Pr | H | Cl | C(=O)Me | O |
| C-13063 | Me(R) | single bond | H | *n*-Pr | H | Cl | C(=O)Me | O |
| C-13064 | Me(S) | single bond | H | *n*-Pr | H | Cl | C(=O)Me | O |
| C-13065 | H | single bond | Me | *n*-Pr | H | Me | C(=O)Me | O |
| C-13066 | Me | single bond | H | *n*-Pr | H | Me | C(=O)Me | O |
| C-13067 | Me(R) | single bond | H | *n*-Pr | H | Me | C(=O)Me | O |
| C-13068 | Me(S) | single bond | H | *n*-Pr | H | Me | C(=O)Me | O |
| C-13069 | H | single bond | Me | *i*-Pr | H | Cl | C(=O)Me | O |
| C-13070 | Me | single bond | H | *i*-Pr | H | Cl | C(=O)Me | O |
| C-13071 | Me(R) | single bond | H | *i*-Pr | H | Cl | C(=O)Me | O |
| C-13072 | Me(S) | single bond | H | *i*-Pr | H | Cl | C(=O)Me | O |
| C-13073 | H | single bond | Me | *i*-Pr | H | Me | C(=O)Me | O |
| C-13074 | Me | single bond | H | *i*-Pr | H | Me | C(=O)Me | O |
| C-13075 | Me(R) | single bond | H | *i*-Pr | H | Me | C(=O)Me | O |
| C-13076 | Me(S) | single bond | H | *i*-Pr | H | Me | C(=O)Me | O |
| C-13077 | H | single bond | Me | *c*-Pr(1-F) | H | Cl | C(=O)Me | O |
| C-13078 | Me | single bond | H | *c*-Pr(1-F) | H | Cl | C(=O)Me | O |
| C-13079 | Me(R) | single bond | H | *c*-Pr(1-F) | H | Cl | C(=O)Me | O |
| C-13080 | Me(S) | single bond | H | *c*-Pr(1-F) | H | Cl | C(=O)Me | O |
| C-13081 | H | single bond | Me | *c*-Pr(1-F) | H | Me | C(=O)Me | O |
| C-13082 | Me | single bond | H | *c*-Pr(1-F) | H | Me | C(=O)Me | O |
| C-13083 | Me(R) | single bond | H | *c*-Pr(1-F) | H | Me | C(=O)Me | O |
| C-13084 | Me(S) | single bond | H | *c*-Pr(1-F) | H | Me | C(=O)Me | O |
| C-13085 | H | single bond | Me | *c*-Pr(2,2-F₂) | H | Cl | C(=O)Me | O |
| C-13086 | Me | single bond | H | *c*-Pr(2,2-F₂) | H | Cl | C(=O)Me | O |

**[Table 526]**

| Compound Number | Y | W | Z | R¹ | R² | R³ | R¹⁰ | A |
|---|---|---|---|---|---|---|---|---|
| C-13087 | Me(R) | single bond | H | *c*-Pr(2,2-F₂) | H | Cl | C(=O)Me | O |
| C-13088 | Me(S) | single bond | H | *c*-Pr(2,2-F₂) | H | Cl | C(=O)Me | O |
| C-13089 | H | single bond | Me | *c*-Pr(2,2-F₂) | H | Me | C(=O)Me | O |
| C-13090 | Me | single bond | H | *c*-Pr(2,2-F₂) | H | Me | C(=O)Me | O |
| C-13091 | Me(R) | single bond | H | *c*-Pr(2,2-F₂) | H | Me | C(=O)Me | O |
| C-13092 | Me(S) | single bond | H | *c*-Pr(2,2-F₂) | H | Me | C(=O)Me | O |
| C-13093 | H | single bond | Me | *c*-Pr(1-Cl) | H | Cl | C(=O)Me | O |
| C-13094 | Me | single bond | H | *c*-Pr(1-Cl) | H | Cl | C(=O)Me | O |
| C-13095 | Me(R) | single bond | H | *c*-Pr(1-Cl) | H | Cl | C(=O)Me | O |
| C-13096 | Me(S) | single bond | H | *c*-Pr(1-Cl) | H | Cl | C(=O)Me | O |
| C-13097 | H | single bond | Me | *c*-Pr(1-Cl) | H | Me | C(=O)Me | O |
| C-13098 | Me | single bond | H | *c*-Pr(1-Cl) | H | Me | C(=O)Me | O |
| C-13099 | Me(R) | single bond | H | *c*-Pr(1-Cl) | H | Me | C(=O)Me | O |
| C-13100 | Me(S) | single bond | H | *c*-Pr(1-Cl) | H | Me | C(=O)Me | O |
| C-13101 | H | single bond | Me | *c*-Pr(1-Br) | H | Cl | C(=O)Me | O |
| C-13102 | Me | single bond | H | *c*-Pr(1-Br) | H | Cl | C(=O)Me | O |
| C-13103 | Me(R) | single bond | H | *c*-Pr(1-Br) | H | Cl | C(=O)Me | O |
| C-13104 | Me(S) | single bond | H | *c*-Pr(1-Br) | H | Cl | C(=O)Me | O |
| C-13105 | H | single bond | Me | *c*-Pr(1-Br) | H | Me | C(=O)Me | O |
| C-13106 | Me | single bond | H | *c*-Pr(1-Br) | H | Me | C(=O)Me | O |
| C-13107 | Me(R) | single bond | H | *c*-Pr(1-Br) | H | Me | C(=O)Me | O |
| C-13108 | Me(S) | single bond | H | *c*-Pr(1-Br) | H | Me | C(=O)Me | O |
| C-13109 | H | single bond | Me | *c*-Pr(1-Me) | H | Cl | C(=O)Me | O |
| C-13110 | Me | single bond | H | *c*-Pr(1-Me) | H | Cl | C(=O)Me | O |
| C-13111 | Me(R) | single bond | H | *c*-Pr(1-Me) | H | Cl | C(=O)Me | O |
| C-13112 | Me(S) | single bond | H | *c*-Pr(1-Me) | H | Cl | C(=O)Me | O |
| C-13113 | H | single bond | Me | *c*-Pr(1-Me) | H | Me | C(=O)Me | O |
| C-13114 | Me | single bond | H | *c*-Pr(1-Me) | H | Me | C(=O)Me | O |
| C-13115 | Me(R) | single bond | H | *c*-Pr(1-Me) | H | Me | C(=O)Me | O |
| C-13116 | Me(S) | single bond | H | *c*-Pr(1-Me) | H | Me | C(=O)Me | O |
| C-13117 | H | single bond | Me | *c*-Pr(1-CH₂OH) | H | Cl | C(=O)Me | O |
| C-13118 | Me | single bond | H | *c*-Pr(1-CH₂OH) | H | Cl | C(=O)Me | O |
| C-13119 | Me(R) | single bond | H | *c*-Pr(1-CH₂OH) | H | Cl | C(=O)Me | O |
| C-13120 | Me(S) | single bond | H | *c*-Pr(1-CH₂OH) | H | Cl | C(=O)Me | O |
| C-13121 | H | single bond | Me | *c*-Pr(1-CH₂OH) | H | Me | C(=O)Me | O |
| C-13122 | Me | single bond | H | *c*-Pr(1-CH₂OH) | H | Me | C(=O)Me | O |
| C-13123 | Me(R) | single bond | H | *c*-Pr(1-CH₂OH) | H | Me | C(=O)Me | O |
| C-13124 | Me(S) | single bond | H | *c*-Pr(1-CH₂OH) | H | Me | C(=O)Me | O |
| C-13125 | H | single bond | Me | *c*-Pr[1-CH₂OS(=O)₂Me] | H | Cl | C(=O)Me | O |
| C-13126 | Me | single bond | H | *c*-Pr[1-CH₂OS(=O)₂Me] | H | Cl | C(=O)Me | O |
| C-13127 | Me(R) | single bond | H | *c*-Pr[1-CH₂OS(=O)₂Me] | H | Cl | C(=O)Me | O |
| C-13128 | Me(S) | single bond | H | *c*-Pr[1-CH₂OS(=O)₂Me] | H | Cl | C(=O)Me | O |
| C-13129 | H | single bond | Me | *c*-Pr[1-CH₂OS(=O)₂Me] | H | Me | C(=O)Me | O |
| C-13130 | Me | single bond | H | *c*-Pr[1-CH₂OS(=O)₂Me] | H | Me | C(=O)Me | O |

**[Table 527]**

| Compound Number | Y | W | Z | R¹ | R² | R³ | R¹⁰ | A |
|---|---|---|---|---|---|---|---|---|
| C-13131 | Me(R) | single bond | H | *c*-Pr[1-CH₂OS(=O)₂Me] | H | Me | C(=O)Me | O |
| C-13132 | Me(S) | single bond | H | *c*-Pr[1-CH₂OS(=O)₂Me] | H | Me | C(=O)Me | O |
| C-13133 | H | single bond | Me | *c*-Pr(1-CH₂OMe) | H | Cl | C(=O)Me | O |
| C-13134 | Me | single bond | H | *c*-Pr(1-CH₂OMe) | H | Cl | C(=O)Me | O |
| C-13135 | Me(R) | single bond | H | *c*-Pr(1-CH₂OMe) | H | Cl | C(=O)Me | O |
| C-13136 | Me(S) | single bond | H | *c*-Pr(1-CH₂OMe) | H | Cl | C(=O)Me | O |
| C-13137 | H | single bond | Me | *c*-Pr(1-CH₂OMe) | H | Me | C(=O)Me | O |
| C-13138 | Me | single bond | H | *c*-Pr(1-CH₂OMe) | H | Me | C(=O)Me | O |
| C-13139 | Me(R) | single bond | H | *c*-Pr(1-CH₂OMe) | H | Me | C(=O)Me | O |
| C-13140 | Me(S) | single bond | H | *c*-Pr(1-CH₂OMe) | H | Me | C(=O)Me | O |
| C-13141 | H | single bond | Me | *c*-Pr(1-CH₂CN) | H | Cl | C(=O)Me | O |
| C-13142 | Me | single bond | H | *c*-Pr(1-CH₂CN) | H | Cl | C(=O)Me | O |
| C-13143 | Me(R) | single bond | H | *c*-Pr(1-CH₂CN) | H | Cl | C(=O)Me | O |
| C-13144 | Me(S) | single bond | H | *c*-Pr(1-CH₂CN) | H | Cl | C(=O)Me | O |
| C-13145 | H | single bond | Me | *c*-Pr(1-CH₂CN) | H | Me | C(=O)Me | O |
| C-13146 | Me | single bond | H | *c*-Pr(1-CH₂CN) | H | Me | C(=O)Me | O |
| C-13147 | Me(R) | single bond | H | *c*-Pr(1-CH₂CN) | H | Me | C(=O)Me | O |
| C-13148 | Me(S) | single bond | H | *c*-Pr(1-CH₂CN) | H | Me | C(=O)Me | O |
| C-13149 | H | single bond | Me | *c*-Pr(1-CH₂SMe) | H | Cl | C(=O)Me | O |
| C-13150 | Me | single bond | H | *c*-Pr(1-CH₂SMe) | H | Cl | C(=O)Me | O |
| C-13151 | Me(R) | single bond | H | *c*-Pr(1-CH₂SMe) | H | Cl | C(=O)Me | O |
| C-13152 | Me(S) | single bond | H | *c*-Pr(1-CH₂SMe) | H | Cl | C(=O)Me | O |
| C-13153 | H | single bond | Me | *c*-Pr(1-CH₂SMe) | H | Me | C(=O)Me | O |
| C-13154 | Me | single bond | H | *c*-Pr(1-CH₂SMe) | H | Me | C(=O)Me | O |
| C-13155 | Me(R) | single bond | H | *c*-Pr(1-CH₂SMe) | H | Me | C(=O)Me | O |
| C-13156 | Me(S) | single bond | H | *c*-Pr(1-CH₂SMe) | H | Me | C(=O)Me | O |
| C-13157 | H | single bond | Me | *c*-Pr[1-CH₂S(=O)Me] | H | Cl | C(=O)Me | O |
| C-13158 | Me | single bond | H | *c*-Pr[1-CH₂S(=O)Me] | H | Cl | C(=O)Me | O |
| C-13159 | Me(R) | single bond | H | *c*-Pr[1-CH₂S(=O)Me] | H | Cl | C(=O)Me | O |
| C-13160 | Me(S) | single bond | H | *c*-Pr[1-CH₂S(=O)Me] | H | Cl | C(=O)Me | O |
| C-13161 | H | single bond | Me | *c*-Pr[1-CH₂S(=O)Me] | H | Me | C(=O)Me | O |
| C-13162 | Me | single bond | H | *c*-Pr[1-CH₂S(=O)Me] | H | Me | C(=O)Me | O |
| C-13163 | Me(R) | single bond | H | *c*-Pr[1-CH₂S(=O)Me] | H | Me | C(=O)Me | O |
| C-13164 | Me(S) | single bond | H | *c*-Pr[1-CH₂S(=O)Me] | H | Me | C(=O)Me | O |
| C-13165 | H | single bond | Me | *c*-Pr[1-CH₂S(=O)₂Me] | H | Cl | C(=O)Me | O |
| C-13166 | Me | single bond | H | *c*-Pr[1-CH₂S(=O)₂Me] | H | Cl | C(=O)Me | O |
| C-13167 | Me(R) | single bond | H | *c*-Pr[1-CH₂S(=O)₂Me] | H | Cl | C(=O)Me | O |
| C-13168 | Me(S) | single bond | H | *c*-pr[1-CH₂S(=O)₂Me] | H | Cl | C(=O)Me | O |
| C-13169 | H | single bond | Me | *c*-Pr[1-CH₂S(=O)₂Me] | H | Me | C(=O)Me | O |
| C-13170 | Me | single bond | H | *c*-Pr[1-CH₂S(=O)₂Me] | H | Me | C(=O)Me | O |
| C-13171 | Me(R) | single bond | H | *c*-Pr[1-CH₂S(=O)₂Me] | H | Me | C(=O)Me | O |
| C-13172 | Me(S) | single bond | H | *c*-Pr[1-CH₂S(=O)₂Me] | H | Me | C(=O)Me | O |
| C-13173 | H | single bond | Me | *c*-Pr(1-CH₂F) | H | Cl | C(=O)Me | O |
| C-13174 | Me | single bond | H | *c*-Pr(1-CH₂F) | H | Cl | C(=O)Me | O |

**[Table 528]**

| Compound Number | Y | W | Z | R¹ | R² | R³ | R¹⁰ | A |
|---|---|---|---|---|---|---|---|---|
| C-13175 | Me(R) | single bond | H | *c*-Pr(1-CH₂F) | H | Cl | C(=O)Me | O |
| C-13176 | Me(S) | single bond | H | *c*-Pr(1-CH₂F) | H | Cl | C(=O)Me | O |
| C-13177 | H | single bond | Me | *c*-Pr(1-CH₂F) | H | Me | C(=O)Me | O |
| C-13178 | Me | single bond | H | *c*-Pr(1-CH₂F) | H | Me | C(=O)Me | O |
| C-13179 | Me(R) | single bond | H | *c*-Pr(1-CH₂F) | H | Me | C(=O)Me | O |
| C-13180 | Me(S) | single bond | H | *c*-Pr(1-CH₂F) | H | Me | C(=O)Me | O |
| C-13181 | H | single bond | Me | *c*-Pr(1-CHF₂) | H | Cl | C(=O)Me | O |
| C-13182 | Me | single bond | H | *c*-Pr(1-CHF₂) | H | Cl | C(=O)Me | O |
| C-13183 | Me(R) | single bond | H | *c*-Pr(1-CHF₂) | H | Cl | C(=O)Me | O |
| C-13184 | Me(S) | single bond | H | *c*-Pr(1-CHF₂) | H | Cl | C(=O)Me | O |
| C-13185 | H | single bond | Me | *c*-Pr(1-CHF₂) | H | Me | C(=O)Me | O |
| C-13186 | Me | single bond | H | *c*-Pr(1-CHF₂) | H | Me | C(=O)Me | O |
| C-13187 | Me(R) | single bond | H | *c*-Pr(1-CHF₂) | H | Me | C(=O)Me | O |
| C-13188 | Me(S) | single bond | H | *c*-Pr(1-CHF₂) | H | Me | C(=O)Me | O |
| C-13189 | H | single bond | Me | *c*-pr(1-CF₃) | H | Cl | C(=O)Me | O |
| C-13190 | Me | single bond | H | *c*-Pr(1-CF₃) | H | Cl | C(=O)Me | O |
| C-13191 | Me(R) | single bond | H | *c*-Pr(1-CF₃) | H | Cl | C(=O)Me | O |
| C-13192 | Me(S) | single bond | H | *c*-Pr(1-CF₃) | H | Cl | C(=O)Me | O |
| C-13193 | H | single bond | Me | *c*-pr(1-CF₃) | H | Me | C(=O)Me | O |
| C-13194 | Me | single bond | H | *c*-Pr(1-CF₃) | H | Me | C(=O)Me | O |
| C-13195 | Me(R) | single bond | H | *c*-Pr(1-CF₃) | H | Me | C(=O)Me | O |
| C-13196 | Me(S) | single bond | H | *c*-Pr(1-CF₃) | H | Me | C(=O)Me | O |
| C-13197 | H | single bond | Me | *c*-Pr(1-C≡CH) | H | Cl | C(=O)Me | O |
| C-13198 | Me | single bond | H | *c*-Pr(1-C≡CH) | H | Cl | C(=O)Me | O |
| C-13199 | Me(R) | single bond | H | *c*-Pr(1-C≡CH) | H | Cl | C(=O)Me | O |
| C-13200 | Me(S) | single bond | H | *c*-Pr(1-C≡CH) | H | Cl | C(=O)Me | O |
| C-13201 | H | single bond | Me | *c*-Pr(1-C≡CH) | H | Me | C(=O)Me | O |
| C-13202 | Me | single bond | H | *c*-Pr(1-C≡CH) | H | Me | C(=O)Me | O |
| C-13203 | Me(R) | single bond | H | *c*-Pr(1-C≡CH) | H | Me | C(=O)Me | O |
| C-13204 | Me(S) | single bond | H | *c*-Pr(1-C≡CH) | H | Me | C(=O)Me | O |
| C-13205 | H | single bond | Me | *c*-Pr[1-C(=O)OEt] | H | Cl | C(=O)Me | O |
| C-13206 | Me | single bond | H | *c*-Pr[1-C(=O)OEt] | H | Cl | C(=O)Me | O |
| C-13207 | Me(R) | single bond | H | *c*-Pr[1-C(=O)OEt] | H | Cl | C(=O)Me | O |
| C-13208 | Me(S) | single bond | H | *c*-Pr[1-C(=O)OEt] | H | Cl | C(=O)Me | O |
| C-13209 | H | single bond | Me | *c*-Pr[1-C(=O)OEt] | H | Me | C(=O)Me | O |
| C-13210 | Me | single bond | H | *c*-Pr[1-C(=O)OEt] | H | Me | C(=O)Me | O |
| C-13211 | Me(R) | single bond | H | *c*-Pr[1-C(=O)OEt] | H | Me | C(=O)Me | O |
| C-13212 | Me(S) | single bond | H | *c*-Pr[1-C(=O)OEt] | H | Me | C(=O)Me | O |
| C-13213 | H | single bond | Me | *c*-Pr[1-C(=O)OH] | H | Cl | C(=O)Me | O |
| C-13214 | Me | single bond | H | *c*-Pr[1-C(=O)OH] | H | Cl | C(=O)Me | O |
| C-13215 | Me(R) | single bond | H | *c*-Pr[1-C(=O)OH] | H | Cl | C(=O)Me | O |
| C-13216 | Me(S) | single bond | H | *c*-Pr[1-C(=O)OH] | H | Cl | C(=O)Me | O |
| C-13217 | H | single bond | Me | *c*-Pr[1-C(=O)OH] | H | Me | C(=O)Me | O |
| C-13218 | Me | single bond | H | *c*-Pr[1-C(=O)OH] | H | Me | C(=O)Me | O |

**[Table 529]**

| Compound Number | Y | W | Z | R¹ | R² | R³ | R¹⁰ | A |
|---|---|---|---|---|---|---|---|---|
| C-13219 | Me(R) | single bond | H | *c*-Pr[1-C(=O)OH] | H | Me | C(=O)Me | O |
| C-13220 | Me(S) | single bond | H | *c*-Pr[1-C(=O)OH] | H | Me | C(=O)Me | O |
| C-13221 | H | single bond | Me | *c*-Pr[1-C(=O)NH₂] | H | Cl | C(=O)Me | O |
| C-13222 | Me | single bond | H | *c*-Pr[1-C(=O)NH₂] | H | Cl | C(=O)Me | O |
| C-13223 | Me(R) | single bond | H | *c*-Pr[1-C(=O)NH₂] | H | Cl | C(=O)Me | O |
| C-13224 | Me(S) | single bond | H | *c*-Pr[1-C(=O)NH₂] | H | Cl | C(=O)Me | O |
| C-13225 | H | single bond | Me | *c*-Pr[1-C(=O)NH₂] | H | Me | C(=O)Me | O |
| C-13226 | Me | single bond | H | *c*-Pr[1-C(=O)NH₂] | H | Me | C(=O)Me | O |
| C-13227 | Me(R) | single bond | H | *c*-Pr[1-C(=O)NH₂] | H | Me | C(=O)Me | O |
| C-13228 | Me(S) | single bond | H | *c*-Pr[1-C(=O)NH₂] | H | Me | C(=O)Me | O |
| C-13229 | H | single bond | Me | *c*-Pr(1-OMe) | H | Cl | C(=O)Me | O |
| C-13230 | Me | single bond | H | *c*-Pr(1-OMe) | H | Cl | C(=O)Me | O |
| C-13231 | Me(R) | single bond | H | *c*-Pr(1-OMe) | H | Cl | C(=O)Me | O |
| C-13232 | Me(S) | single bond | H | *c*-Pr(1-OMe) | H | Cl | C(=O)Me | O |
| C-13233 | H | single bond | Me | *c*-Pr(1-OMe) | H | Me | C(=O)Me | O |
| C-13234 | Me | single bond | H | *c*-Pr(1-OMe) | H | Me | C(=O)Me | O |
| C-13235 | Me(R) | single bond | H | *c*-Pr(1-OMe) | H | Me | C(=O)Me | O |
| C-13236 | Me(S) | single bond | H | *c*-Pr(1-OMe) | H | Me | C(=O)Me | O |
| C-13237 | H | single bond | Me | *c*-Pr(1-SMe) | H | Cl | C(=O)Me | O |
| C-13238 | Me | single bond | H | *c*-Pr(1-SMe) | H | Cl | C(=O)Me | O |
| C-13239 | Me(R) | single bond | H | *c*-Pr(1-SMe) | H | Cl | C(=O)Me | O |
| C-13240 | Me(S) | single bond | H | *c*-Pr(1-SMe) | H | Cl | C(=O)Me | O |
| C-13241 | H | single bond | Me | *c*-Pr(1-SMe) | H | Me | C(=O)Me | O |
| C-13242 | Me | single bond | H | *c*-Pr(1-SMe) | H | Me | C(=O)Me | O |
| C-13243 | Me(R) | single bond | H | *c*-Pr(1-SMe) | H | Me | C(=O)Me | O |
| C-13244 | Me(S) | single bond | H | *c*-Pr(1-SMe) | H | Me | C(=O)Me | O |
| C-13245 | H | single bond | Me | *c*-Pr[1-S(=O)Me] | H | Cl | C(=O)Me | O |
| C-13246 | Me | single bond | H | *c*-Pr[1-S(=O)Me] | H | Cl | C(=O)Me | O |
| C-13247 | Me(R) | single bond | H | *c*-Pr[1-S(=O)Me] | H | Cl | C(=O)Me | O |
| C-13248 | Me(S) | single bond | H | *c*-Pr[1-S(=O)Me] | H | Cl | C(=O)Me | O |
| C-13249 | H | single bond | Me | *c*-Pr[1-S(=O)Me] | H | Me | C(=O)Me | O |
| C-13250 | Me | single bond | H | *c*-Pr[1-S(=O)Me] | H | Me | C(=O)Me | O |
| C-13251 | Me(R) | single bond | H | *c*-Pr[1-S(=O)Me] | H | Me | C(=O)Me | O |
| C-13252 | Me(S) | single bond | H | *c*-Pr[1-S(=O)Me] | H | Me | C(=O)Me | O |
| C-13253 | H | single bond | Me | *c*-Pr[1-S(=O)₂Me] | H | Cl | C(=O)Me | O |
| C-13254 | Me | single bond | H | *c*-Pr[1-S(=O)₂Me] | H | Cl | C(=O)Me | O |
| C-13255 | Me(R) | single bond | H | *c*-Pr[1-S(=O)₂Me] | H | Cl | C(=O)Me | O |
| C-13256 | Me(S) | single bond | H | *c*-Pr[1-S(=O)₂Me] | H | Cl | C(=O)Me | O |
| C-13257 | H | single bond | Me | *c*-Pr[1-S(=O)₂Me] | H | Me | C(=O)Me | O |
| C-13258 | Me | single bond | H | *c*-Pr[1-S(=O)₂Me] | H | Me | C(=O)Me | O |
| C-13259 | Me(R) | single bond | H | *c*-Pr[1-S(=O)₂Me] | H | Me | C(=O)Me | O |
| C-13260 | Me(S) | single bond | H | *c*-Pr[1-S(=O)₂Me] | H | Me | C(=O)Me | O |
| C-13261 | H | single bond | Me | *c*-Pr[1-S(=O)₂CHF₂] | H | Cl | C(=O)Me | O |
| C-13262 | Me | single bond | H | *c*-Pr[1-S(=O)₂CHF₂] | H | Cl | C(=O)Me | O |

**[Table 530]**

| Compound Number | Y | W | Z | R¹ | R² | R³ | R¹⁰ | A |
|---|---|---|---|---|---|---|---|---|
| C-13263 | Me(R) | single bond | H | *c*-Pr[1-S(=O)₂CHF₂] | H | Cl | C(=O)Me | O |
| C-13264 | Me(S) | single bond | H | *c*-Pr[1-S(=O)₂CHF₂] | H | Cl | C(=O)Me | O |
| C-13265 | H | single bond | Me | *c*-Pr[1-S(=O)₂CHF₂] | H | Me | C(=O)Me | O |
| C-13266 | Me | single bond | H | *c*-Pr[1-S(=O)₂CHF₂] | H | Me | C(=O)Me | O |
| C-13267 | Me(R) | single bond | H | *c*-Pr[1-S(=O)₂CHF₂] | H | Me | C(=O)Me | O |
| C-13268 | Me(S) | single bond | H | *c*-Pr[1-S(=O)₂CHF₂] | H | Me | C(=O)Me | O |
| C-13269 | H | single bond | Me | *c*-Pr[1-S(=O)₂CF₃] | H | Cl | C(=O)Me | O |
| C-13270 | Me | single bond | H | *c*-Pr[1-S(=O)₂CF₃] | H | Cl | C(=O)Me | O |
| C-13271 | Me(R) | single bond | H | *c*-Pr[1-S(=O)₂CF₃] | H | Cl | C(=O)Me | O |
| C-13272 | Me(S) | single bond | H | *c*-Pr[1-S(=O)₂CF₃] | H | Cl | C(=O)Me | O |
| C-13273 | H | single bond | Me | *c*-Pr[1-S(=O)₂CF₃] | H | Me | C(=O)Me | O |
| C-13274 | Me | single bond | H | *c*-Pr[1-S(=O)₂CF₃] | H | Me | C(=O)Me | O |
| C-13275 | Me(R) | single bond | H | *c*-Pr[1-S(=O)₂CF₃] | H | Me | C(=O)Me | O |
| C-13276 | Me(S) | single bond | H | *c*-Pr[1-S(=O)₂CF₃] | H | Me | C(=O)Me | O |
| C-13277 | H | single bond | Me | *c*-Pr[1-(5,6-dihydro-1,4,2-dioxazin-3-yl)] | H | Cl | C(=O)Me | O |
| C-13278 | Me | single bond | H | *c*-Pr[1-(5,6-dihydro-1,4,2-dioxazin-3-yl)] | H | Cl | C(=O)Me | O |
| C-13279 | Me(R) | single bond | H | *c*-Pr[1-(5,6-dihydro-1,4,2-dioxazin-3-yl)] | H | Cl | C(=O)Me | O |
| C-13280 | Me(S) | single bond | H | *c*-Pr[1-(5,6-dihydro-1,4,2-dioxazin-3-yl)] | H | Cl | C(=O)Me | O |
| C-13281 | H | single bond | Me | *c*-Pr[1-(5,6-dihydro-1,4,2-dioxazin-3-yl)] | H | Me | C(=O)Me | O |
| C-13282 | Me | single bond | H | *c*-Pr[1-(5,6-dihydro-1,4,2-dioxazin-3-yl)] | H | Me | C(=O)Me | O |
| C-13283 | Me(R) | single bond | H | *c*-Pr[1-(5,6-dihydro-1,4,2-dioxazin-3-yl)] | H | Me | C(=O)Me | O |
| C-13284 | Me(S) | single bond | H | *c*-Pr[1-(5,6-dihydro-1,4,2-dioxazin-3-yl)] | H | Me | C(=O)Me | O |
| C-13285 | H | single bond | Me | *c*-Pr[1-(5-hydroxy-1,3,4-oxadiazol-2-yl)] | H | Cl | C(=O)Me | O |
| C-13286 | Me | single bond | H | *c*-Pr[1-(5-hydroxy-1,3,4-oxadiazol-2-yl)] | H | Cl | C(=O)Me | O |
| C-13287 | Me(R) | single bond | H | *c*-Pr[1-(5-hydroxy-1,3,4-oxadiazol-2-yl)] | H | Cl | C(=O)Me | O |
| C-13288 | Me(S) | single bond | H | *c*-Pr[1-(5-hydroxy-1,3,4-oxadiazol-2-yl)] | H | Cl | C(=O)Me | O |
| C-13289 | H | single bond | Me | *c*-Pr[1-(5-hydroxy-1,3,4-oxadiazol-2-yl)] | H | Me | C(=O)Me | O |
| C-13290 | Me | single bond | H | *c*-Pr[1-(5-hydroxy-1,3,4-oxadiazol-2-yl)] | H | Me | C(=O)Me | O |
| C-13291 | Me(R) | single bond | H | *c*-Pr[1-(5-hydroxy-1,3,4-oxadiazol-2-yl)] | H | Me | C(=O)Me | O |
| C-13292 | Me(S) | single bond | H | *c*-Pr[1-(5-hydroxy-1,3,4-oxadiazol-2-yl)] | H | Me | C(=O)Me | O |
| C-13293 | H | single bond | Me | *c*-Pr[1-(pyrimidin-2-yl)] | H | Cl | C(=O)Me | O |
| C-13294 | Me | single bond | H | *c*-Pr[1-(pyrimidin-2-yl)] | H | Cl | C(=O)Me | O |
| C-13295 | Me(R) | single bond | H | *c*-Pr[1-(pyrimidin-2-yl)] | H | Cl | C(=O)Me | O |
| C-13296 | Me(S) | single bond | H | *c*-Pr[1-(pyrimidin-2-yl)] | H | Cl | C(=O)Me | O |
| C-13297 | H | single bond | Me | *c*-Pr[1-(pyrimidin-2-yl)] | H | Me | C(=O)Me | O |
| C-13298 | Me | single bond | H | *c*-Pr[1-(pyrimidin-2-yl)] | H | Me | C(=O)Me | O |
| C-13299 | Me(R) | single bond | H | *c*-Pr[1-(pyrimidin-2-yl)] | H | Me | C(=O)Me | O |
| C-13300 | Me(S) | single bond | H | *c*-Pr[1-(pyrimidin-2-yl)] | H | Me | C(=O)Me | O |
| C-13301 | H | single bond | Me | *t*-Bu | H | Cl | C(=O)Me | O |
| C-13302 | Me | single bond | H | *t*-Bu | H | Cl | C(=O)Me | O |
| C-13303 | Me(R) | single bond | H | *t*-Bu | H | Cl | C(=O)Me | O |
| C-13304 | Me(S) | single bond | H | *t*-Bu | H | Cl | C(=O)Me | O |
| C-13305 | H | single bond | Me | *t*-Bu | H | Me | C(=O)Me | O |
| C-13306 | Me | single bond | H | *t*-Bu | H | Me | C(=O)Me | O |

**[Table 531]**

| Compound Number | Y | W | Z | R¹ | R² | R³ | R¹⁰ | A |
|---|---|---|---|---|---|---|---|---|
| C-13307 | Me(R) | single bond | H | *t*-Bu | H | Me | C(=O)Me | O |
| C-13308 | Me(S) | single bond | H | *t*-Bu | H | Me | C(=O)Me | O |
| C-13309 | H | single bond | Me | *c*-Bu | H | Cl | C(=O)Me | O |
| C-13310 | Me | single bond | H | *c*-Bu | H | Cl | C(=O)Me | O |
| C-13311 | Me(R) | single bond | H | *c*-Bu | H | Cl | C(=O)Me | O |
| C-13312 | Me(S) | single bond | H | *c*-Bu | H | Cl | C(=O)Me | O |
| C-13313 | H | single bond | Me | *c*-Bu | H | Me | C(=O)Me | O |
| C-13314 | Me | single bond | H | *c*-Bu | H | Me | C(=O)Me | O |
| C-13315 | Me(R) | single bond | H | *c*-Bu | H | Me | C(=O)Me | O |
| C-13316 | Me(S) | single bond | H | *c*-Bu | H | Me | C(=O)Me | O |
| C-13317 | H | single bond | Me | *c*-Bu(1-CN) | H | Cl | C(=O)Me | O |
| C-13318 | Me | single bond | H | *c*-Bu(1-CN) | H | Cl | C(=O)Me | O |
| C-13319 | Me(R) | single bond | H | *c*-Bu(1-CN) | H | Cl | C(=O)Me | O |
| C-13320 | Me(S) | single bond | H | *c*-Bu(1-CN) | H | Cl | C(=O)Me | O |
| C-13321 | H | single bond | Me | *c*-Bu(1-CN) | H | Me | C(=O)Me | O |
| C-13322 | Me | single bond | H | *c*-Bu(1-CN) | H | Me | C(=O)Me | O |
| C-13323 | Me(R) | single bond | H | *c*-Bu(1-CN) | H | Me | C(=O)Me | O |
| C-13324 | Me(S) | single bond | H | *c*-Bu(1-CN) | H | Me | C(=O)Me | O |
| C-13325 | H | single bond | Me | *c*-Bu(3,3-F₂) | H | Cl | C(=O)Me | O |
| C-13326 | Me | single bond | H | *c*-Bu(3,3-F₂) | H | Cl | C(=O)Me | O |
| C-13327 | Me(R) | single bond | H | *c*-Bu(3,3-F₂) | H | Cl | C(=O)Me | O |
| C-13328 | Me(S) | single bond | H | *c*-Bu(3,3-F₂) | H | Cl | C(=O)Me | O |
| C-13329 | H | single bond | Me | *c*-Bu(3,3-F₂) | H | Me | C(=O)Me | O |
| C-13330 | Me | single bond | H | *c*-Bu(3,3-F₂) | H | Me | C(=O)Me | O |
| C-13331 | Me(R) | single bond | H | *c*-Bu(3,3-F₂) | H | Me | C(=O)Me | O |
| C-13332 | Me(S) | single bond | H | *c*-Bu(3,3-F₂) | H | Me | C(=O)Me | O |
| C-13333 | H | single bond | Me | oxetan-3-yl | H | Cl | C(=O)Me | O |
| C-13334 | Me | single bond | H | oxetan-3-yl | H | Cl | C(=O)Me | O |
| C-13335 | Me(R) | single bond | H | oxetan-3-yl | H | Cl | C(=O)Me | O |
| C-13336 | Me(S) | single bond | H | oxetan-3-yl | H | Cl | C(=O)Me | O |
| C-13337 | H | single bond | Me | oxetan-3-yl | H | Me | C(=O)Me | O |
| C-13338 | Me | single bond | H | oxetan-3-yl | H | Me | C(=O)Me | O |
| C-13339 | Me(R) | single bond | H | oxetan-3-yl | H | Me | C(=O)Me | O |
| C-13340 | Me(S) | single bond | H | oxetan-3-yl | H | Me | C(=O)Me | O |
| C-13341 | H | single bond | Me | oxetan(3-CN)-3-yl | H | Cl | C(=O)Me | O |
| C-13342 | Me | single bond | H | oxetan(3-CN)-3-yl | H | Cl | C(=O)Me | O |
| C-13343 | Me(R) | single bond | H | oxetan(3-CN)-3-yl | H | Cl | C(=O)Me | O |
| C-13344 | Me(S) | single bond | H | oxetan(3-CN)-3-yl | H | Cl | C(=O)Me | O |
| C-13345 | H | single bond | Me | oxetan(3-CN)-3-yl | H | Me | C(=O)Me | O |
| C-13346 | Me | single bond | H | oxetan(3-CN)-3-yl | H | Me | C(=O)Me | O |
| C-13347 | Me(R) | single bond | H | oxetan(3-CN)-3-yl | H | Me | C(=O)Me | O |
| C-13348 | Me(S) | single bond | H | oxetan(3-CN)-3-yl | H | Me | C(=O)Me | O |
| C-13349 | H | single bond | Me | azetidin[1-C(=O)Me]-3-yl | H | Cl | C(=O)Me | O |
| C-13350 | Me | single bond | H | azetidin[1-C(=O)Me]-3-yl | H | Cl | C(=O)Me | O |

**[Table 532]**

| Compound Number | Y | W | Z | R¹ | R² | R³ | R¹⁰ | A |
|---|---|---|---|---|---|---|---|---|
| C-13351 | Me(R) | single bond | H | azetidin[1-C(=O)Me]-3-yl | H | Cl | C(=O)Me | O |
| C-13352 | Me(S) | single bond | H | azetidin[1-C(=O)Me]-3-yl | H | Cl | C(=O)Me | O |
| C-13353 | H | single bond | Me | azetidin[1-C(=O)Me]-3-yl | H | Me | C(=O)Me | O |
| C-13354 | Me | single bond | H | azetidin[1-C(=O)Me]-3-yl | H | Me | C(=O)Me | O |
| C-13355 | Me(R) | single bond | H | azetidin[1-C(=O)Me]-3-yl | H | Me | C(=O)Me | O |
| C-13356 | Me(S) | single bond | H | azetidin[1-C(=O)Me]-3-yl | H | Me | C(=O)Me | O |
| C-13357 | H | single bond | Me | azetidin[1-S(=O)₂Me]-3-yl | H | Cl | C(=O)Me | O |
| C-13358 | Me | single bond | H | azetidin[1-S(=O)₂Me]-3-yl | H | Cl | C(=O)Me | O |
| C-13359 | Me(R) | single bond | H | azetidin[1-S(=O)₂Me]-3-yl | H | Cl | C(=O)Me | O |
| C-13360 | Me(S) | single bond | H | azetidin[1-S(=O)₂Me]-3-yl | H | Cl | C(=O)Me | O |
| C-13361 | H | single bond | Me | azetidin[1-S(=O)₂Me]-3-yl | H | Me | C(=O)Me | O |
| C-13362 | Me | single bond | H | azetidin[1-S(=O)₂Me]-3-yl | H | Me | C(=O)Me | O |
| C-13363 | Me(R) | single bond | H | azetidin[1-S(=O)₂Me]-3-yl | H | Me | C(=O)Me | O |
| C-13364 | Me(S) | single bond | H | azetidin[1-S(=O)₂Me]-3-yl | H | Me | C(=O)Me | O |
| C-13365 | H | single bond | Me | azetidin[1-S(=O)₂CHF₂]-3-yl | H | Cl | C(=O)Me | O |
| C-13366 | Me | single bond | H | azetidin[1-S(=O)₂CHF₂]-3-yl | H | Cl | C(=O)Me | O |
| C-13367 | Me(R) | single bond | H | azetidin[1-S(=O)₂CHF₂]-3-yl | H | Cl | C(=O)Me | O |
| C-13368 | Me(S) | single bond | H | azetidin[1-S(=O)₂CHF₂]-3-yl | H | Cl | C(=O)Me | O |
| C-13369 | H | single bond | Me | azetidin[1-S(=O)₂CHF₂]-3-yl | H | Me | C(=O)Me | O |
| C-13370 | Me | single bond | H | azetidin[1-S(=O)₂CHF₂]-3-yl | H | Me | C(=O)Me | O |
| C-13371 | Me(R) | single bond | H | azetidin[1-S(=O)₂CHF₂]-3-yl | H | Me | C(=O)Me | O |
| C-13372 | Me(S) | single bond | H | azetidin[1-S(=O)₂CHF₂]-3-yl | H | Me | C(=O)Me | O |
| C-13373 | H | single bond | Me | *c*-Pen | H | Cl | C(=O)Me | O |
| C-13374 | Me | single bond | H | *c*-Pen | H | Cl | C(=O)Me | O |
| C-13375 | Me(R) | single bond | H | *c*-Pen | H | Cl | C(=O)Me | O |
| C-13376 | Me(S) | single bond | H | *c*-Pen | H | Cl | C(=O)Me | O |
| C-13377 | H | single bond | Me | *c*-Pen | H | Me | C(=O)Me | O |
| C-13378 | Me | single bond | H | *c*-Pen | H | Me | C(=O)Me | O |
| C-13379 | Me(R) | single bond | H | *c*-Pen | H | Me | C(=O)Me | O |
| C-13380 | Me(S) | single bond | H | *c*-Pen | H | Me | C(=O)Me | O |
| C-13381 | H | single bond | Me | tetrahydrofuran-3-yl | H | Cl | C(=O)Me | O |
| C-13382 | Me | single bond | H | tetrahydrofuran-3-yl | H | Cl | C(=O)Me | O |
| C-13383 | Me(R) | single bond | H | tetra hydrofuran-3-yl | H | Cl | C(=O)Me | O |
| C-13384 | Me(S) | single bond | H | tetrahydrofuran-3-yl | H | Cl | C(=O)Me | O |
| C-13385 | H | single bond | Me | tetrahydrofuran-3-yl | H | Me | C(=O)Me | O |
| C-13386 | Me | single bond | H | tetrahydrofuran-3-yl | H | Me | C(=O)Me | O |
| C-13387 | Me(R) | single bond | H | tetrahydrofuran-3-yl | H | Me | C(=O)Me | O |
| C-13388 | Me(S) | single bond | H | tetrahydrofuran-3-yl | H | Me | C(=O)Me | O |
| C-13389 | H | single bond | Me | tetrahydrothiophen-3-yl | H | Cl | C(=O)Me | O |
| C-13390 | Me | single bond | H | tetrahydrothiophen-3-yl | H | Cl | C(=O)Me | O |
| C-13391 | Me(R) | single bond | H | tetrahydrothiophen-3-yl | H | Cl | C(=O)Me | O |
| C-13392 | Me(S) | single bond | H | tetrahydrothiophen-3-yl | H | Cl | C(=O)Me | O |
| C-13393 | H | single bond | Me | tetrahydrothiophen-3-yl | H | Me | C(=O)Me | O |
| C-13394 | Me | single bond | H | tetrahydrothiophen-3-yl | H | Me | C(=O)Me | O |

**[Table 533]**

| Compound Number | Y | W | Z | R¹ | R² | R³ | R¹⁰ | A |
|---|---|---|---|---|---|---|---|---|
| C-13395 | Me(R) | single bond | H | tetrahydrothiophen-3-yl | H | Me | C(=O)Me | O |
| C-13396 | Me(S) | single bond | H | tetrahydrothiophen-3-yl | H | Me | C(=O)Me | O |
| C-13397 | H | single bond | Me | *c*-Pen(1-CN) | H | Cl | C(=O)Me | O |
| C-13398 | Me | single bond | H | *c*-Pen(1-CN) | H | Cl | C(=O)Me | O |
| C-13399 | Me(R) | single bond | H | *c*-Pen(1-CN) | H | Cl | C(=O)Me | O |
| C-13400 | Me(S) | single bond | H | *c*-Pen(1-CN) | H | Cl | C(=O)Me | O |
| C-13401 | H | single bond | Me | *c*-Pen(1-CN) | H | Me | C(=O)Me | O |
| C-13402 | Me | single bond | H | *c*-Pen(1-CN) | H | Me | C(=O)Me | O |
| C-13403 | Me(R) | single bond | H | *c*-Pen(1-CN) | H | Me | C(=O)Me | O |
| C-13404 | Me(S) | single bond | H | *c*-Pen(1-CN) | H | Me | C(=O)Me | O |
| C-13405 | H | single bond | Me | tetrahydrofuran(3-CN)-3-yl | H | Cl | C(=O)Me | O |
| C-13406 | Me | single bond | H | tetrahydrofuran(3-CN)-3-yl | H | Cl | C(=O)Me | O |
| C-13407 | Me(R) | single bond | H | tetrahydrofuran(3-CN)-3-yl | H | Cl | C(=O)Me | O |
| C-13408 | Me(S) | single bond | H | tetrahydrofuran(3-CN)-3-yl | H | Cl | C(=O)Me | O |
| C-13409 | H | single bond | Me | tetrahydrofuran(3-CN)-3-yl | H | Me | C(=O)Me | O |
| C-13410 | Me | single bond | H | tetrahydrofuran(3-CN)-3-yl | H | Me | C(=O)Me | O |
| C-13411 | Me(R) | single bond | H | tetrahydrofuran(3-CN)-3-yl | H | Me | C(=O)Me | O |
| C-13412 | Me(S) | single bond | H | tetrahydrofuran(3-CN)-3-yl | H | Me | C(=O)Me | O |
| C-13413 | H | single bond | Me | tetrahydrothiophen(3-CN)-3-yl | H | Cl | C(=O)Me | O |
| C-13414 | Me | single bond | H | tetrahydrothiophen(3-CN)-3-yl | H | Cl | C(=O)Me | O |
| C-13415 | Me(R) | single bond | H | tetrahydrothiophen(3-CN)-3-yl | H | Cl | C(=O)Me | O |
| C-13416 | Me(S) | single bond | H | tetrahydrothiophen(3-CN)-3-yl | H | Cl | C(=O)Me | O |
| C-13417 | H | single bond | Me | tetrahydrothiophen(3-CN)-3-yl | H | Me | C(=O)Me | O |
| C-13418 | Me | single bond | H | tetrahydrothiophen(3-CN)-3-yl | H | Me | C(=O)Me | O |
| C-13419 | Me(R) | single bond | H | tetrahydrothiophen(3-CN)-3-yl | H | Me | C(=O)Me | O |
| C-13420 | Me(S) | single bond | H | tetrahydrothiophen(3-CN)-3-yl | H | Me | C(=O)Me | O |
| C-13421 | H | single bond | Me | bicyclo[1.1.1]pentan-1-yl | H | Cl | C(=O)Me | O |
| C-13422 | Me | single bond | H | bicyclo[1.1.1]pentan-1-yl | H | Cl | C(=O)Me | O |
| C-13423 | Me(R) | single bond | H | bicyclo[1.1.1]pentan-1-yl | H | Cl | C(=O)Me | O |
| C-13424 | Me(S) | single bond | H | bicyclo[1.1.1]pentan-1-yl | H | Cl | C(=O)Me | O |
| C-13425 | H | single bond | Me | bicyclo[1.1.1]pentan-1-yl | H | Me | C(=O)Me | O |
| C-13426 | Me | single bond | H | bicyclo[1.1.1]pentan-1-yl | H | Me | C(=O)Me | O |
| C-13427 | Me(R) | single bond | H | bicyclo[1.1.1]pentan-1-yl | H | Me | C(=O)Me | O |
| C-13428 | Me(S) | single bond | H | bicyclo[1.1.1]pentan-1-yl | H | Me | C(=O)Me | O |
| C-13429 | H | single bond | Me | *c*-Hex | H | Cl | C(=O)Me | O |
| C-13430 | Me | single bond | H | *c*-Hex | H | Cl | C(=O)Me | O |
| C-13431 | Me(R) | single bond | H | *c*-Hex | H | Cl | C(=O)Me | O |
| C-13432 | Me(S) | single bond | H | *c*-Hex | H | Cl | C(=O)Me | O |
| C-13433 | H | single bond | Me | *c*-Hex | H | Me | C(=O)Me | O |
| C-13434 | Me | single bond | H | *c*-Hex | H | Me | C(=O)Me | O |
| C-13435 | Me(R) | single bond | H | *c*-Hex | H | Me | C(=O)Me | O |
| C-13436 | Me(S) | single bond | H | *c*-Hex | H | Me | C(=O)Me | O |
| C-13437 | H | single bond | Me | tetrahydropyran-4-yl | H | Cl | C(=O)Me | O |
| C-13438 | Me | single bond | H | tetrahydropyran-4-yl | H | Cl | C(=O)Me | O |

**[Table 534]**

| Compound Number | Y | W | Z | R¹ | R² | R³ | R¹⁰ | A |
|---|---|---|---|---|---|---|---|---|
| C-13439 | Me(R) | single bond | H | tetrahydropyran-4-yl | H | Cl | C(=O)Me | O |
| C-13440 | Me(S) | single bond | H | tetrahydropyran-4-yl | H | Cl | C(=O)Me | O |
| C-13441 | H | single bond | Me | tetrahydropyran-4-yl | H | Me | C(=O)Me | O |
| C-13442 | Me | single bond | H | tetrahydropyran-4-yl | H | Me | C(=O)Me | O |
| C-13443 | Me(R) | single bond | H | tetrahydropyran-4-yl | H | Me | C(=O)Me | O |
| C-13444 | Me(S) | single bond | H | tetrahydropyran-4-yl | H | Me | C(=O)Me | O |
| C-13445 | H | single bond | Me | tetrahydrothiopyran-4-yl | H | Cl | C(=O)Me | O |
| C-13446 | Me | single bond | H | tetrahydrothiopyran-4-yl | H | Cl | C(=O)Me | O |
| C-13447 | Me(R) | single bond | H | tetrahydrothiopyran-4-yl | H | Cl | C(=O)Me | O |
| C-13448 | Me(S) | single bond | H | tetrahydrothiopyran-4-yl | H | Cl | C(=O)Me | O |
| C-13449 | H | single bond | Me | tetrahydrothiopyran-4-yl | H | Me | C(=O)Me | O |
| C-13450 | Me | single bond | H | tetrahydrothiopyran-4-yl | H | Me | C(=O)Me | O |
| C-13451 | Me(R) | single bond | H | tetrahydrothiopyran-4-yl | H | Me | C(=O)Me | O |
| C-13452 | Me(S) | single bond | H | tetrahydrothiopyran-4-yl | H | Me | C(=O)Me | O |
| C-13453 | H | single bond | Me | *c*-Hex(1-CN) | H | Cl | C(=O)Me | O |
| C-13454 | Me | single bond | H | *c*-Hex(1-CN) | H | Cl | C(=O)Me | O |
| C-13455 | Me(R) | single bond | H | *c*-Hex(1-CN) | H | Cl | C(=O)Me | O |
| C-13456 | Me(S) | single bond | H | *c*-Hex(1-CN) | H | Cl | C(=O)Me | O |
| C-13457 | H | single bond | Me | *c*-Hex(1-CN) | H | Me | C(=O)Me | O |
| C-13458 | Me | single bond | H | *c*-Hex(1-CN) | H | Me | C(=O)Me | O |
| C-13459 | Me(R) | single bond | H | *c*-Hex(1-CN) | H | Me | C(=O)Me | O |
| C-13460 | Me(S) | single bond | H | *c*-Hex(1-CN) | H | Me | C(=O)Me | O |
| C-13461 | H | single bond | Me | tetrahydropyran(4-CN)-4-yl | H | Cl | C(=O)Me | O |
| C-13462 | Me | single bond | H | tetrahydropyran(4-CN)-4-yl | H | Cl | C(=O)Me | O |
| C-13463 | Me(R) | single bond | H | tetrahydropyran(4-CN)-4-yl | H | Cl | C(=O)Me | O |
| C-13464 | Me(S) | single bond | H | tetrahydropyran(4-CN)-4-yl | H | Cl | C(=O)Me | O |
| C-13465 | H | single bond | Me | tetrahydropyran(4-CN)-4-yl | H | Me | C(=O)Me | O |
| C-13466 | Me | single bond | H | tetrahydropyran(4-CN)-4-yl | H | Me | C(=O)Me | O |
| C-13467 | Me(R) | single bond | H | tetrahydropyran(4-CN)-4-yl | H | Me | C(=O)Me | O |
| C-13468 | Me(S) | single bond | H | tetrahydropyran(4-CN)-4-yl | H | Me | C(=O)Me | O |
| C-13469 | H | single bond | Me | tetrahydrothiopyran(4-CN)-4-yl | H | Cl | C(=O)Me | O |
| C-13470 | Me | single bond | H | tetrahydrothiopyran(4-CN)-4-yl | H | Cl | C(=O)Me | O |
| C-13471 | Me(R) | single bond | H | tetrahydrothiopyran(4-CN)-4-yl | H | Cl | C(=O)Me | O |
| C-13472 | Me(S) | single bond | H | tetrahydrothiopyran(4-CN)-4-yl | H | Cl | C(=O)Me | O |
| C-13473 | H | single bond | Me | tetrahydrothiopyran(4-CN)-4-yl | H | Me | C(=O)Me | O |
| C-13474 | Me | single bond | H | tetrahydrothiopyran(4-CN)-4-yl | H | Me | C(=O)Me | O |
| C-13475 | Me(R) | single bond | H | tetrahydrothiopyran(4-CN)-4-yl | H | Me | C(=O)Me | O |
| C-13476 | Me(S) | single bond | H | tetrahydrothiopyran(4-CN)-4-yl | H | Me | C(=O)Me | O |
| C-13477 | H | single bond | Me | CH₂(*c*-Pr) | H | Cl | C(=O)Me | O |
| C-13478 | Me | single bond | H | CH₂(*c*-Pr) | H | Cl | C(=O)Me | O |
| C-13479 | Me(R) | single bond | H | CH₂(*c*-Pr) | H | Cl | C(=O)Me | O |
| C-13480 | Me(S) | single bond | H | CH₂(*c*-Pr) | H | Cl | C(=O)Me | O |
| C-13481 | H | single bond | Me | CH₂(*c*-Pr) | H | Me | C(=O)Me | O |
| C-13482 | Me | single bond | H | CH₂(*c*-Pr) | H | Me | C(=O)Me | O |

**[Table 535]**

| Compound Number | Y | W | Z | R¹ | R² | R³ | R¹⁰ | A |
|---|---|---|---|---|---|---|---|---|
| C-13483 | Me(R) | single bond | H | CH₂(*c*-Pr) | H | Me | C(=O)Me | O |
| C-13484 | Me(S) | single bond | H | CH₂(*c*-Pr) | H | Me | C(=O)Me | O |
| C-13485 | H | single bond | Me | CH₂[*c*-Pr(1-CN)] | H | Cl | C(=O)Me | O |
| C-13486 | Me | single bond | H | CH₂[*c*-Pr(1-CN)] | H | Cl | C(=O)Me | O |
| C-13487 | Me(R) | single bond | H | CH₂[*c*-Pr(1-CN)] | H | Cl | C(=O)Me | O |
| C-13488 | Me(S) | single bond | H | CH₂[*c*-Pr(1-CN)] | H | Cl | C(=O)Me | O |
| C-13489 | H | single bond | Me | CH₂[*c*-Pr(1-CN)] | H | Me | C(=O)Me | O |
| C-13490 | Me | single bond | H | CH₂[*c*-Pr(1-CN)] | H | Me | C(=O)Me | O |
| C-13491 | Me(R) | single bond | H | CH₂[*c*-Pr(1-CN)] | H | Me | C(=O)Me | O |
| C-13492 | Me(S) | single bond | H | CH₂[*c*-Pr(1-CN)] | H | Me | C(=O)Me | O |
| C-13493 | H | single bond | Me | CH(Me)(*c*-Pr) | H | Cl | C(=O)Me | O |
| C-13494 | Me | single bond | H | CH(Me)(*c*-Pr) | H | Cl | C(=O)Me | O |
| C-13495 | Me(R) | single bond | H | CH(Me)(*c*-Pr) | H | Cl | C(=O)Me | O |
| C-13496 | Me(S) | single bond | H | CH(Me)(*c*-Pr) | H | Cl | C(=O)Me | O |
| C-13497 | H | single bond | Me | CH(Me)(*c*-Pr) | H | Me | C(=O)Me | O |
| C-13498 | Me | single bond | H | CH(Me)(*c*-Pr) | H | Me | C(=O)Me | O |
| C-13499 | Me(R) | single bond | H | CH(Me)(*c*-Pr) | H | Me | C(=O)Me | O |
| C-13500 | Me(S) | single bond | H | CH(Me)(*c*-Pr) | H | Me | C(=O)Me | O |
| C-13501 | H | single bond | Me | CH(Me)CH₂OMe | H | Cl | C(=O)Me | O |
| C-13502 | Me | single bond | H | CH(Me)CH₂OMe | H | Cl | C(=O)Me | O |
| C-13503 | Me(R) | single bond | H | CH(Me)CH₂OMe | H | Cl | C(=O)Me | O |
| C-13504 | Me(S) | single bond | H | CH(Me)CH₂OMe | H | Cl | C(=O)Me | O |
| C-13505 | H | single bond | Me | CH(Me)CH₂OMe | H | Me | C(=O)Me | O |
| C-13506 | Me | single bond | H | CH(Me)CH₂OMe | H | Me | C(=O)Me | O |
| C-13507 | Me(R) | single bond | H | CH(Me)CH₂OMe | H | Me | C(=O)Me | O |
| C-13508 | Me(S) | single bond | H | CH(Me)CH₂OMe | H | Me | C(=O)Me | O |
| C-13509 | H | single bond | Me | CH₂CH=CH₂ | H | Cl | C(=O)Me | O |
| C-13510 | Me | single bond | H | CH₂CH=CH₂ | H | Cl | C(=O)Me | O |
| C-13511 | Me(R) | single bond | H | CH₂CH=CH₂ | H | Cl | C(=O)Me | O |
| C-13512 | Me(S) | single bond | H | CH₂CH=CH₂ | H | Cl | C(=O)Me | O |
| C-13513 | H | single bond | Me | CH₂CH=CH₂ | H | Me | C(=O)Me | O |
| C-13514 | Me | single bond | H | CH₂CH=CH₂ | H | Me | C(=O)Me | O |
| C-13515 | Me(R) | single bond | H | CH₂CH=CH₂ | H | Me | C(=O)Me | O |
| C-13516 | Me(S) | single bond | H | CH₂CH=CH₂ | H | Me | C(=O)Me | O |
| C-13517 | H | single bond | Me | CH₂C≡CH | H | Cl | C(=O)Me | O |
| C-13518 | Me | single bond | H | CH₂C≡CH | H | Cl | C(=O)Me | O |
| C-13519 | Me(R) | single bond | H | CH₂C≡CH | H | Cl | C(=O)Me | O |
| C-13520 | Me(S) | single bond | H | CH₂C≡CH | H | Cl | C(=O)Me | O |
| C-13521 | H | single bond | Me | CH₂C≡CH | H | Me | C(=O)Me | O |
| C-13522 | Me | single bond | H | CH₂C≡CH | H | Me | C(=O)Me | O |
| C-13523 | Me(R) | single bond | H | CH₂C≡CH | H | Me | C(=O)Me | O |
| C-13524 | Me(S) | single bond | H | CH₂C≡CH | H | Me | C(=O)Me | O |
| C-13525 | H | single bond | Me | CH₂CN | H | Cl | C(=O)Me | O |
| C-13526 | Me | single bond | H | CH₂CN | H | Cl | C(=O)Me | O |

**[Table 536]**

| Compound Numb | Y | W | Z | R¹ | R² | R³ | R¹⁰ | A |
|---|---|---|---|---|---|---|---|---|
| C-13527 | Me(R) | single bond | H | CH₂CN | H | Cl | C(=O)Me | O |
| C-13528 | Me(S) | single bond | H | CH₂CN | H | Cl | C(=O)Me | O |
| C-13529 | H | single bond | Me | CH₂CN | H | Me | C(=O)Me | O |
| C-13530 | Me | single bond | H | CH₂CN | H | Me | C(=O)Me | O |
| C-13531 | Me(R) | single bond | H | CH₂CN | H | Me | C(=O)Me | O |
| C-13532 | Me(S) | single bond | H | CH₂CN | H | Me | C(=O)Me | O |
| C-13533 | H | single bond | Me | CH(Me)CN | H | Cl | C(=O)Me | O |
| C-13534 | Me | single bond | H | CH(Me)CN | H | Cl | C(=O)Me | O |
| C-13535 | Me(R) | single bond | H | CH(Me)CN | H | Cl | C(=O)Me | O |
| C-13536 | Me(S) | single bond | H | CH(Me)CN | H | Cl | C(=O)Me | O |
| C-13537 | H | single bond | Me | CH(Me)CN | H | Me | C(=O)Me | O |
| C-13538 | Me | single bond | H | CH(Me)CN | H | Me | C(=O)Me | O |
| C-13539 | Me(R) | single bond | H | CH(Me)CN | H | Me | C(=O)Me | O |
| C-13540 | Me(S) | single bond | H | CH(Me)CN | H | Me | C(=O)Me | O |
| C-13541 | H | single bond | Me | C(Me)₂CN | H | Cl | C(=O)Me | O |
| C-13542 | Me | single bond | H | C(Me)₂CN | H | Cl | C(=O)Me | O |
| C-13543 | Me(R) | single bond | H | C(Me)₂CN | H | Cl | C(=O)Me | O |
| C-13544 | Me(S) | single bond | H | C(Me)₂CN | H | Cl | C(=O)Me | O |
| C-13545 | H | single bond | Me | C(Me)₂CN | H | Me | C(=O)Me | O |
| C-13546 | Me | single bond | H | C(Me)₂CN | H | Me | C(=O)Me | O |
| C-13547 | Me(R) | single bond | H | C(Me)₂CN | H | Me | C(=O)Me | O |
| C-13548 | Me(S) | single bond | H | C(Me)₂CN | H | Me | C(=O)Me | O |
| C-13549 | H | single bond | Me | C(Me)(*i*-Pr)CN | H | Cl | C(=O)Me | O |
| C-13550 | Me | single bond | H | C(Me)(*i*-Pr)CN | H | Cl | C(=O)Me | O |
| C-13551 | Me(R) | single bond | H | C(Me)(*i*-Pr)CN | H | Cl | C(=O)Me | O |
| C-13552 | Me(S) | single bond | H | C(Me)(*i*-Pr)CN | H | Cl | C(=O)Me | O |
| C-13553 | H | single bond | Me | C(Me)(*i*-Pr)CN | H | Me | C(=O)Me | O |
| C-13554 | Me | single bond | H | C(Me)(*i*-Pr)CN | H | Me | C(=O)Me | O |
| C-13555 | Me(R) | single bond | H | C(Me)(*i*-Pr)CN | H | Me | C(=O)Me | O |
| C-13556 | Me(S) | single bond | H | C(Me)(*i*-Pr)CN | H | Me | C(=O)Me | O |
| C-13557 | H | single bond | Me | OMe | H | Cl | C(=O)Me | O |
| C-13558 | Me | single bond | H | OMe | H | Cl | C(=O)Me | O |
| C-13559 | Me(R) | single bond | H | OMe | H | Cl | C(=O)Me | O |
| C-13560 | Me(S) | single bond | H | OMe | H | Cl | C(=O)Me | O |
| C-13561 | H | single bond | Me | OMe | H | Me | C(=O)Me | O |
| C-13562 | Me | single bond | H | OMe | H | Me | C(=O)Me | O |
| C-13563 | Me(R) | single bond | H | OMe | H | Me | C(=O)Me | O |
| C-13564 | Me(S) | single bond | H | OMe | H | Me | C(=O)Me | O |
| C-13565 | H | single bond | Me | CH=NOMe | H | Cl | C(=O)Me | O |
| C-13566 | Me | single bond | H | CH=NOMe | H | Cl | C(=O)Me | O |
| C-13567 | Me(R) | single bond | H | CH=NOMe | H | Cl | C(=O)Me | O |
| C-13568 | Me(S) | single bond | H | CH=NOMe | H | Cl | C(=O)Me | O |
| C-13569 | H | single bond | Me | CH=NOMe | H | Me | C(=O)Me | O |
| C-13570 | Me | single bond | H | CH=NOMe | H | Me | C(=O)Me | O |

**[Table 537]**

| Compound Number | Y | W | Z | R¹ | R² | R³ | R¹⁰ | A |
|---|---|---|---|---|---|---|---|---|
| C-13571 | Me(R) | single bond | H | CH=NOMe | H | Me | C(=O)Me | O |
| C-13572 | Me(S) | single bond | H | CH=NOMe | H | Me | C(=O)Me | O |
| C-13573 | H | single bond | Me | morpholin-4-yl | H | Cl | C(=O)Me | O |
| C-13574 | Me | single bond | H | morpholin-4-yl | H | Cl | C(=O)Me | O |
| C-13575 | Me(R) | single bond | H | morpholin-4-yl | H | Cl | C(=O)Me | O |
| C-13576 | Me(S) | single bond | H | morpholin-4-yl | H | Cl | C(=O)Me | O |
| C-13577 | H | single bond | Me | morpholin-4-yl | H | Me | C(=O)Me | O |
| C-13578 | Me | single bond | H | morpholin-4-yl | H | Me | C(=O)Me | O |
| C-13579 | Me(R) | single bond | H | morpholin-4-yl | H | Me | C(=O)Me | O |
| C-13580 | Me(S) | single bond | H | morpholin-4-yl | H | Me | C(=O)Me | O |
| C-13581 | H | single bond | Me | Ph | H | Cl | C(=O)Me | O |
| C-13582 | Me | single bond | H | Ph | H | Cl | C(=O)Me | O |
| C-13583 | Me(R) | single bond | H | Ph | H | Cl | C(=O)Me | O |
| C-13584 | Me(S) | single bond | H | Ph | H | Cl | C(=O)Me | O |
| C-13585 | H | single bond | Me | Ph | H | Me | C(=O)Me | O |
| C-13586 | Me | single bond | H | Ph | H | Me | C(=O)Me | O |
| C-13587 | Me(R) | single bond | H | Ph | H | Me | C(=O)Me | O |
| C-13588 | Me(S) | single bond | H | Ph | H | Me | C(=O)Me | O |
| C-13589 | H | single bond | Me | CH₂Ph | H | Cl | C(=O)Me | O |
| C-13590 | Me | single bond | H | CH₂Ph | H | Cl | C(=O)Me | O |
| C-13591 | Me(R) | single bond | H | CH₂Ph | H | Cl | C(=O)Me | O |
| C-13592 | Me(S) | single bond | H | CH₂Ph | H | Cl | C(=O)Me | O |
| C-13593 | H | single bond | Me | CH₂Ph | H | Me | C(=O)Me | O |
| C-13594 | Me | single bond | H | CH₂Ph | H | Me | C(=O)Me | O |
| C-13595 | Me(R) | single bond | H | CH₂Ph | H | Me | C(=O)Me | O |
| C-13596 | Me(S) | single bond | H | CH₂Ph | H | Me | C(=O)Me | O |
| C-13597 | H | single bond | Me | thiazole-2-yl | H | Cl | C(=O)Me | O |
| C-13598 | Me | single bond | H | thiazole-2-yl | H | Cl | C(=O)Me | O |
| C-13599 | Me(R) | single bond | H | thiazole-2-yl | H | Cl | C(=O)Me | O |
| C-13600 | Me(S) | single bond | H | thiazole-2-yl | H | Cl | C(=O)Me | O |
| C-13601 | H | single bond | Me | thiazole-2-yl | H | Me | C(=O)Me | O |
| C-13602 | Me | single bond | H | thiazole-2-yl | H | Me | C(=O)Me | O |
| C-13603 | Me(R) | single bond | H | thiazole-2-yl | H | Me | C(=O)Me | O |
| C-13604 | Me(S) | single bond | H | thiazole-2-yl | H | Me | C(=O)Me | O |
| C-13605 | H | single bond | Me | 1-Me-pyrazol-3-yl | H | Cl | C(=O)Me | O |
| C-13606 | Me | single bond | H | 1-Me-pyrazol-3-yl | H | Cl | C(=O)Me | O |
| C-13607 | Me(R) | single bond | H | 1-Me-pyrazol-3-yl | H | Cl | C(=O)Me | O |
| C-13608 | Me(S) | single bond | H | 1-Me-pyrazol-3-yl | H | Cl | C(=O)Me | O |
| C-13609 | H | single bond | Me | 1-Me-pyrazol-3-yl | H | Me | C(=O)Me | O |
| C-13610 | Me | single bond | H | 1-Me-pyrazol-3-yl | H | Me | C(=O)Me | O |
| C-13611 | Me(R) | single bond | H | 1-Me-pyrazol-3-yl | H | Me | C(=O)Me | O |
| C-13612 | Me(S) | single bond | H | 1-Me-pyrazol-3-yl | H | Me | C(=O)Me | O |
| C-13613 | H | single bond | Me | 1-Me-pyrazol-5-yl | H | Cl | C(=O)Me | O |
| C-13614 | Me | single bond | H | 1-Me-pyrazol-5-yl | H | Cl | C(=O)Me | O |

**[Table 538]**

| Compound Number | Y | W | Z | R¹ | R² | R³ | R¹⁰ | A |
|---|---|---|---|---|---|---|---|---|
| C-13615 | Me(R) | single bond | H | 1-Me-pyrazol-5-yl | H | Cl | C(=O)Me | O |
| C-13616 | Me(S) | single bond | H | 1-Me-pyrazol-5-yl | H | Cl | C(=O)Me | O |
| C-13617 | H | single bond | Me | 1-Me-pyrazol-5-yl | H | Me | C(=O)Me | O |
| C-13618 | Me | single bond | H | 1-Me-pyrazol-5-yl | H | Me | C(=O)Me | O |
| C-13619 | Me(R) | single bond | H | 1-Me-pyrazol-5-yl | H | Me | C(=O)Me | O |
| C-13620 | Me(S) | single bond | H | 1-Me-pyrazol-5-yl | H | Me | C(=O)Me | O |
| C-13621 | H | single bond | Me | CH₂(pyrimidin-2-yl) | H | Cl | C(=O)Me | O |
| C-13622 | Me | single bond | H | CH₂(pyrimidin-2-yl) | H | Cl | C(=O)Me | O |
| C-13623 | Me(R) | single bond | H | CH₂(pyrimidin-2-yl) | H | Cl | C(=O)Me | O |
| C-13624 | Me(S) | single bond | H | CH₂(pyrimidin-2-yl) | H | Cl | C(=O)Me | O |
| C-13625 | H | single bond | Me | CH₂(pyrimidin-2-yl) | H | Me | C(=O)Me | O |
| C-13626 | Me | single bond | H | CH₂(pyrimidin-2-yl) | H | Me | C(=O)Me | O |
| C-13627 | Me(R) | single bond | H | CH₂(pyrimidin-2-yl) | H | Me | C(=O)Me | O |
| C-13628 | Me(S) | single bond | H | CH₂(pyrimidin-2-yl) | H | Me | C(=O)Me | O |
| C-13629 | H | single bond | Me | -CH₂CH₂CH₂- | | Cl | C(=O)Me | O |
| C-13630 | Me | single bond | H | -CH₂CH₂CH₂- | | Cl | C(=O)Me | O |
| C-13631 | Me(R) | single bond | H | -CH₂CH₂CH₂- | | Cl | C(=O)Me | O |
| C-13632 | Me(S) | single bond | H | -CH₂CH₂CH₂- | | Cl | C(=O)Me | O |
| C-13633 | H | single bond | Me | -CH₂CH₂CH₂- | | Me | C(=O)Me | O |
| C-13634 | Me | single bond | H | -CH₂CH₂CH₂- | | Me | C(=O)Me | O |
| C-13635 | Me(R) | single bond | H | -CH₂CH₂CH₂- | | Me | C(=O)Me | O |
| C-13636 | Me(S) | single bond | H | -CH₂CH₂CH₂- | | Me | C(=O)Me | O |
| C-13637 | H | single bond | Me | -CH₂CH₂OCH₂CH₂- | | Cl | C(=O)Me | O |
| C-13638 | Me | single bond | H | -CH₂CH₂OCH₂CH₂- | | Cl | C(=O)Me | O |
| C-13639 | Me(R) | single bond | H | -CH₂CH₂OCH₂CH₂- | | Cl | C(=O)Me | O |
| C-13640 | Me(S) | single bond | H | -CH₂CH₂OCH₂CH₂- | | Cl | C(=O)Me | O |
| C-13641 | H | single bond | Me | -CH₂CH₂OCH₂CH₂- | | Me | C(=O)Me | O |
| C-13642 | Me | single bond | H | -CH₂CH₂OCH₂CH₂- | | Me | C(=O)Me | O |
| C-13643 | Me(R) | single bond | H | -CH₂CH₂OCH₂CH₂- | | Me | C(=O)Me | O |
| C-13644 | Me(S) | single bond | H | -CH₂CH₂OCH₂CH₂- | | Me | C(=O)Me | O |
| C-13645 | H | single bond | Me | Me | Me | Cl | H | O |
| C-13646 | Me | single bond | H | Me | Me | Cl | H | O |
| C-13647 | Me(R) | single bond | H | Me | Me | Cl | H | O |
| C-13648 | Me(S) | single bond | H | Me | Me | Cl | H | O |
| C-13649 | H | single bond | Me | Me | Me | Me | H | O |
| C-13650 | Me | single bond | H | Me | Me | Me | H | O |
| C-13651 | Me(R) | single bond | H | Me | Me | Me | H | O |
| C-13652 | Me(S) | single bond | H | Me | Me | Me | H | O |
| C-13653 | H | single bond | Me | CH₂CHF₂ | Me | Cl | H | O |
| C-13654 | Me | single bond | H | CH₂CHF₂ | Me | Cl | H | O |
| C-13655 | Me(R) | single bond | H | CH₂CHF₂ | Me | Cl | H | O |
| C-13656 | Me(S) | single bond | H | CH₂CHF₂ | Me | Cl | H | O |
| C-13657 | H | single bond | Me | CH₂CHF₂ | Me | Me | H | O |
| C-13658 | Me | single bond | H | CH₂CHF₂ | Me | Me | H | O |

**[Table 539]**

| Compound Number | Y | W | Z | R¹ | R² | R³ | R¹⁰ | A |
|---|---|---|---|---|---|---|---|---|
| C-13659 | Me(R) | single bond | H | CH₂CHF₂ | Me | Me | H | O |
| C-13660 | Me(S) | single bond | H | CH₂CHF₂ | Me | Me | H | O |
| C-13661 | H | single bond | Me | *n*-Pr | Me | Cl | H | O |
| C-13662 | Me | single bond | H | *n*-Pr | Me | Cl | H | O |
| C-13663 | Me(R) | single bond | H | *n*-Pr | Me | Cl | H | O |
| C-13664 | Me(S) | single bond | H | *n*-Pr | Me | Cl | H | O |
| C-13665 | H | single bond | Me | *n*-Pr | Me | Me | H | O |
| C-13666 | Me | single bond | H | *n*-Pr | Me | Me | H | O |
| C-13667 | Me(R) | single bond | H | *n*-Pr | Me | Me | H | O |
| C-13668 | Me(S) | single bond | H | *n*-Pr | Me | Me | H | O |
| C-13669 | H | single bond | Me | *i*-Pr | Me | Cl | H | O |
| C-13670 | Me | single bond | H | *i*-Pr | Me | Cl | H | O |
| C-13671 | Me(R) | single bond | H | *i*-Pr | Me | Cl | H | O |
| C-13672 | Me(S) | single bond | H | *i*-Pr | Me | Cl | H | O |
| C-13673 | H | single bond | Me | *i*-Pr | Me | Me | H | O |
| C-13674 | Me | single bond | H | *i*-Pr | Me | Me | H | O |
| C-13675 | Me(R) | single bond | H | *i*-Pr | Me | Me | H | O |
| C-13676 | Me(S) | single bond | H | *i*-Pr | Me | Me | H | O |
| C-13677 | H | single bond | Me | *c*-Pr(1-F) | Me | Cl | H | O |
| C-13678 | Me | single bond | H | *c*-Pr(1-F) | Me | Cl | H | O |
| C-13679 | Me(R) | single bond | H | *c*-Pr(1-F) | Me | Cl | H | O |
| C-13680 | Me(S) | single bond | H | *c*-Pr(1-F) | Me | Cl | H | O |
| C-13681 | H | single bond | Me | *c*-Pr(1-F) | Me | Me | H | O |
| C-13682 | Me | single bond | H | *c*-Pr(1-F) | Me | Me | H | O |
| C-13683 | Me(R) | single bond | H | *c*-Pr(1-F) | Me | Me | H | O |
| C-13684 | Me(S) | single bond | H | *c*-Pr(1-F) | Me | Me | H | O |
| C-13685 | H | single bond | Me | *c*-Pr(2,2-F₂) | Me | Cl | H | O |
| C-13686 | Me | single bond | H | *c*-Pr(2,2-F₂) | Me | Cl | H | O |
| C-13687 | Me(R) | single bond | H | *c*-Pr(2,2-F₂) | Me | Cl | H | O |
| C-13688 | Me(S) | single bond | H | *c*-Pr(2,2-F₂) | Me | Cl | H | O |
| C-13689 | H | single bond | Me | *c*-Pr(2,2-F₂) | Me | Me | H | O |
| C-13690 | Me | single bond | H | *c*-Pr(2,2-F₂) | Me | Me | H | O |
| C-13691 | Me(R) | single bond | H | *c*-Pr(2,2-F₂) | Me | Me | H | O |
| C-13692 | Me(S) | single bond | H | *c*-Pr(2,2-F₂) | Me | Me | H | O |
| C-13693 | H | single bond | Me | *c*-Pr(1-Cl) | Me | Cl | H | O |
| C-13694 | Me | single bond | H | *c*-Pr(1-Cl) | Me | Cl | H | O |
| C-13695 | Me(R) | single bond | H | *c*-Pr(1-Cl) | Me | Cl | H | O |
| C-13696 | Me(S) | single bond | H | *c*-Pr(1-Cl) | Me | Cl | H | O |
| C-13697 | H | single bond | Me | *c*-Pr(1-Cl) | Me | Me | H | O |
| C-13698 | Me | single bond | H | *c*-Pr(1-Cl) | Me | Me | H | O |
| C-13699 | Me(R) | single bond | H | *c*-Pr(1-Cl) | Me | Me | H | O |
| C-13700 | Me(S) | single bond | H | *c*-Pr(1-Cl) | Me | Me | H | O |
| C-13701 | H | single bond | Me | *c*-Pr(1-Br) | Me | Cl | H | O |
| C-13702 | Me | single bond | H | *c*-Pr(1-Br) | Me | Cl | H | O |

**[Table 540]**

| Compound Number | Y | W | Z | R¹ | R² | R³ | R¹⁰ | A |
|---|---|---|---|---|---|---|---|---|
| C-13703 | Me(R) | single bond | H | *c*-Pr(1-Br) | Me | Cl | H | O |
| C-13704 | Me(S) | single bond | H | *c*-Pr(1-Br) | Me | Cl | H | O |
| C-13705 | H | single bond | Me | *c*-Pr(1-Br) | Me | Me | H | O |
| C-13706 | Me | single bond | H | *c*-Pr(1-Br) | Me | Me | H | O |
| C-13707 | Me(R) | single bond | H | *c*-Pr(1-Br) | Me | Me | H | O |
| C-13708 | Me(S) | single bond | H | *c*-Pr(1-Br) | Me | Me | H | O |
| C-13709 | H | single bond | Me | *c*-Pr(1-Me) | Me | Cl | H | O |
| C-13710 | Me | single bond | H | *c*-Pr(1-Me) | Me | Cl | H | O |
| C-13711 | Me(R) | single bond | H | *c*-Pr(1-Me) | Me | Cl | H | O |
| C-13712 | Me(S) | single bond | H | *c*-Pr(1-Me) | Me | Cl | H | O |
| C-13713 | H | single bond | Me | *c*-Pr(1-Me) | Me | Me | H | O |
| C-13714 | Me | single bond | H | *c*-Pr(1-Me) | Me | Me | H | O |
| C-13715 | Me(R) | single bond | H | *c*-Pr(1-Me) | Me | Me | H | O |
| C-13716 | Me(S) | single bond | H | *c*-Pr(1-Me) | Me | Me | H | O |
| C-13717 | H | single bond | Me | *c*-Pr(1-CH₂OH) | Me | Cl | H | O |
| C-13718 | Me | single bond | H | *c*-Pr(1-CH₂OH) | Me | Cl | H | O |
| C-13719 | Me(R) | single bond | H | *c*-Pr(1-CH₂OH) | Me | Cl | H | O |
| C-13720 | Me(S) | single bond | H | *c*-Pr(1-CH₂OH) | Me | Cl | H | O |
| C-13721 | H | single bond | Me | *c*-Pr(1-CH₂OH) | Me | Me | H | O |
| C-13722 | Me | single bond | H | *c*-Pr(1-CH₂OH) | Me | Me | H | O |
| C-13723 | Me(R) | single bond | H | *c*-Pr(1-CH₂OH) | Me | Me | H | O |
| C-13724 | Me(S) | single bond | H | *c*-Pr(1-CH₂OH) | Me | Me | H | O |
| C-13725 | H | single bond | Me | *c*-Pr[1-CH₂OS(=O)₂Me] | Me | Cl | H | O |
| C-13726 | Me | single bond | H | *c*-Pr[1-CH₂OS(=O)₂Me] | Me | Cl | H | O |
| C-13727 | Me(R) | single bond | H | *c*-Pr[1-CH₂OS(=O)₂Me] | Me | Cl | H | O |
| C-13728 | Me(S) | single bond | H | *c*-Pr[1-CH₂OS(=O)₂Me] | Me | Cl | H | O |
| C-13729 | H | single bond | Me | *c*-Pr[1-CH₂OS(=O)₂Me] | Me | Me | H | O |
| C-13730 | Me | single bond | H | *c*-Pr[1-CH₂OS(=O)₂Me] | Me | Me | H | O |
| C-13731 | Me(R) | single bond | H | *c*-Pr[1-CH₂OS(=O)₂Me] | Me | Me | H | O |
| C-13732 | Me(S) | single bond | H | *c*-Pr[1-CH₂OS(=O)₂Me] | Me | Me | H | O |
| C-13733 | H | single bond | Me | *c*-Pr(1-CH₂OMe) | Me | Cl | H | O |
| C-13734 | Me | single bond | H | *c*-Pr(1-CH₂OMe) | Me | Cl | H | O |
| C-13735 | Me(R) | single bond | H | *c*-Pr(1-CH₂OMe) | Me | Cl | H | O |
| C-13736 | Me(S) | single bond | H | *c*-Pr(1-CH₂OMe) | Me | Cl | H | O |
| C-13737 | H | single bond | Me | *c*-Pr(1-CH₂OMe) | Me | Me | H | O |
| C-13738 | Me | single bond | H | *c*-Pr(1-CH₂OMe) | Me | Me | H | O |
| C-13739 | Me(R) | single bond | H | *c*-Pr(1-CH₂OMe) | Me | Me | H | O |
| C-13740 | Me(S) | single bond | H | *c*-Pr(1-CH₂OMe) | Me | Me | H | O |
| C-13741 | H | single bond | Me | *c*-Pr(1-CH₂CN) | Me | Cl | H | O |
| C-13742 | Me | single bond | H | *c*-Pr(1-CH₂CN) | Me | Cl | H | O |
| C-13743 | Me(R) | single bond | H | *c*-Pr(1-CH₂CN) | Me | Cl | H | O |
| C-13744 | Me(S) | single bond | H | *c*-Pr(1-CH₂CN) | Me | Cl | H | O |
| C-13745 | H | single bond | Me | *c*-Pr(1-CH₂CN) | Me | Me | H | O |
| C-13746 | Me | single bond | H | *c*-Pr(1-CH₂CN) | Me | Me | H | O |

**[Table 541]**

| Compound Number | Y | W | Z | R¹ | R² | R³ | R¹⁰ | A |
|---|---|---|---|---|---|---|---|---|
| C-13747 | Me(R) | single bond | H | *c*-Pr(1-CH₂CN) | Me | Me | H | O |
| C-13748 | Me(S) | single bond | H | *c*-Pr(1-CH₂CN) | Me | Me | H | O |
| C-13749 | H | single bond | Me | *c*-Pr(1-CH₂SMe) | Me | Cl | H | O |
| C-13750 | Me | single bond | H | *c*-Pr(1-CH₂SMe) | Me | Cl | H | O |
| C-13751 | Me(R) | single bond | H | *c*-Pr(1-CH₂SMe) | Me | Cl | H | O |
| C-13752 | Me(S) | single bond | H | *c*-Pr(1-CH₂SMe) | Me | Cl | H | O |
| C-13753 | H | single bond | Me | *c*-Pr(1-CH₂SMe) | Me | Me | H | O |
| C-13754 | Me | single bond | H | *c*-Pr(1-CH₂SMe) | Me | Me | H | O |
| C-13755 | Me(R) | single bond | H | *c*-Pr(1-CH₂SMe) | Me | Me | H | O |
| C-13756 | Me(S) | single bond | H | *c*-Pr(1-CH₂SMe) | Me | Me | H | O |
| C-13757 | H | single bond | Me | *c*-Pr[1-CH₂S(=O)Me] | Me | Cl | H | O |
| C-13758 | Me | single bond | H | *c*-Pr[1-CH₂S(=O)Me] | Me | Cl | H | O |
| C-13759 | Me(R) | single bond | H | *c*-Pr[1-CH₂S(=O)Me] | Me | Cl | H | O |
| C-13760 | Me(S) | single bond | H | *c*-Pr[1-CH₂S(=O)Me] | Me | Cl | H | O |
| C-13761 | H | single bond | Me | *c*-Pr[1-CH₂S(=O)Me] | Me | Me | H | O |
| C-13762 | Me | single bond | H | *c*-Pr[1-CH₂S(=O)Me] | Me | Me | H | O |
| C-13763 | Me(R) | single bond | H | *c*-Pr[1-CH₂S(=O)Me] | Me | Me | H | O |
| C-13764 | Me(S) | single bond | H | *c*-Pr[1-CH₂S(=O)Me] | Me | Me | H | O |
| C-13765 | H | single bond | Me | *c*-Pr[1-CH₂S(=O)₂Me] | Me | Cl | H | O |
| C-13766 | Me | single bond | H | *c*-Pr[1-CH₂S(=O)₂Me] | Me | Cl | H | O |
| C-13767 | Me(R) | single bond | H | *c*-Pr[1-CH₂S(=O)₂Me] | Me | Cl | H | O |
| C-13768 | Me(S) | single bond | H | *c*-Pr[1-CH₂S(=O)₂Me] | Me | Cl | H | O |
| C-13769 | H | single bond | Me | *c*-Pr[1-CH₂S(=O)₂Me] | Me | Me | H | O |
| C-13770 | Me | single bond | H | *c*-Pr[1-CH₂S(=O)₂Me] | Me | Me | H | O |
| C-13771 | Me(R) | single bond | H | *c*-Pr[1-CH₂S(=O)₂Me] | Me | Me | H | O |
| C-13772 | Me(S) | single bond | H | *c*-Pr[1-CH₂S(=O)₂Me] | Me | Me | H | O |
| C-13773 | H | single bond | Me | *c*-Pr(1-CH₂F) | Me | Cl | H | O |
| C-13774 | Me | single bond | H | *c*-Pr(1-CH₂F) | Me | Cl | H | O |
| C-13775 | Me(R) | single bond | H | *c*-Pr(1-CH₂F) | Me | Cl | H | O |
| C-13776 | Me(S) | single bond | H | *c*-Pr(1-CH₂F) | Me | Cl | H | O |
| C-13777 | H | single bond | Me | *c*-Pr(1-CH₂F) | Me | Me | H | O |
| C-13778 | Me | single bond | H | *c*-Pr(1-CH₂F) | Me | Me | H | O |
| C-13779 | Me(R) | single bond | H | *c*-Pr(1-CH₂F) | Me | Me | H | O |
| C-13780 | Me(S) | single bond | H | *c*-Pr(1-CH₂F) | Me | Me | H | O |
| C-13781 | H | single bond | Me | *c*-Pr(1-CHF₂) | Me | Cl | H | O |
| C-13782 | Me | single bond | H | *c*-Pr(1-CHF₂) | Me | Cl | H | O |
| C-13783 | Me(R) | single bond | H | *c*-Pr(1-CHF₂) | Me | Cl | H | O |
| C-13784 | Me(S) | single bond | H | *c*-Pr(1-CHF₂) | Me | Cl | H | O |
| C-13785 | H | single bond | Me | *c*-Pr(1-CHF₂) | Me | Me | H | O |
| C-13786 | Me | single bond | H | *c*-Pr(1-CHF₂) | Me | Me | H | O |
| C-13787 | Me(R) | single bond | H | *c*-Pr(1-CHF₂) | Me | Me | H | O |
| C-13788 | Me(S) | single bond | H | *c*-Pr(1-CHF₂) | Me | Me | H | O |
| C-13789 | H | single bond | Me | *c*-Pr(1-CF₃) | Me | Cl | H | O |
| C-13790 | Me | single bond | H | *c*-Pr(1-CF₃) | Me | Cl | H | O |

**[Table 542]**

| Compound Number | Y | W | Z | R¹ | R² | R³ | R¹⁰ | A |
|---|---|---|---|---|---|---|---|---|
| C-13791 | Me(R) | single bond | H | *c*-Pr(1-CF₃) | Me | Cl | H | O |
| C-13792 | Me(S) | single bond | H | *c*-Pr(1-CF₃) | Me | Cl | H | O |
| C-13793 | H | single bond | Me | *c*-Pr(1-CF₃) | Me | Me | H | O |
| C-13794 | Me | single bond | H | *c*-Pr(1-CF₃) | Me | Me | H | O |
| C-13795 | Me(R) | single bond | H | *c*-Pr(1-CF₃) | Me | Me | H | O |
| C-13796 | Me(S) | single bond | H | *c*-Pr(1-CF₃) | Me | Me | H | O |
| C-13797 | H | single bond | Me | *c*-Pr(1-C≡CH) | Me | Cl | H | O |
| C-13798 | Me | single bond | H | *c*-Pr(1-C≡CH) | Me | Cl | H | O |
| C-13799 | Me(R) | single bond | H | *c*-Pr(1-C≡CH) | Me | Cl | H | O |
| C-13800 | Me(S) | single bond | H | *c*-Pr(1-C≡CH) | Me | Cl | H | O |
| C-13801 | H | single bond | Me | *c*-Pr(1-C≡CH) | Me | Me | H | O |
| C-13802 | Me | single bond | H | *c*-Pr(1-C≡CH) | Me | Me | H | O |
| C-13803 | Me(R) | single bond | H | *c*-Pr(1-C≡CH) | Me | Me | H | O |
| C-13804 | Me(S) | single bond | H | *c*-Pr(1-C≡CH) | Me | Me | H | O |
| C-13805 | H | single bond | Me | *c*-Pr[1-C(=O)OEt] | Me | Cl | H | O |
| C-13806 | Me | single bond | H | *c*-Pr[1-C(=O)OEt] | Me | Cl | H | O |
| C-13807 | Me(R) | single bond | H | *c*-Pr[1-C(=O)OEt] | Me | Cl | H | O |
| C-13808 | Me(S) | single bond | H | *c*-Pr[1-C(=O)OEt] | Me | Cl | H | O |
| C-13809 | H | single bond | Me | *c*-Pr[1-C(=O)OEt] | Me | Me | H | O |
| C-13810 | Me | single bond | H | *c*-Pr[1-C(=O)OEt] | Me | Me | H | O |
| C-13811 | Me(R) | single bond | H | *c*-Pr[1-C(=O)OEt] | Me | Me | H | O |
| C-13812 | Me(S) | single bond | H | *c*-Pr[1-C(=O)OEt] | Me | Me | H | O |
| C-13813 | H | single bond | Me | *c*-Pr[1-C(=O)OH] | Me | Cl | H | O |
| C-13814 | Me | single bond | H | *c*-Pr[1-C(=O)OH] | Me | Cl | H | O |
| C-13815 | Me(R) | single bond | H | *c*-Pr[1-C(=O)OH] | Me | Cl | H | O |
| C-13816 | Me(S) | single bond | H | *c*-Pr[1-C(=O)OH] | Me | Cl | H | O |
| C-13817 | H | single bond | Me | *c*-Pr[1-C(=O)OH] | Me | Me | H | O |
| C-13818 | Me | single bond | H | *c*-Pr[1-C(=O)OH] | Me | Me | H | O |
| C-13819 | Me(R) | single bond | H | *c*-Pr[1-C(=O)OH] | Me | Me | H | O |
| C-13820 | Me(S) | single bond | H | *c*-Pr[1-C(=O)OH] | Me | Me | H | O |
| C-13821 | H | single bond | Me | *c*-Pr[1-C(=O)NH₂] | Me | Cl | H | O |
| C-13822 | Me | single bond | H | *c*-Pr[1-C(=O)NH₂] | Me | Cl | H | O |
| C-13823 | Me(R) | single bond | H | *c*-Pr[1-C(=O)NH₂] | Me | Cl | H | O |
| C-13824 | Me(S) | single bond | H | *c*-Pr[1-C(=O)NH₂] | Me | Cl | H | O |
| C-13825 | H | single bond | Me | *c*-Pr[1-C(=O)NH₂] | Me | Me | H | O |
| C-13826 | Me | single bond | H | *c*-Pr[1-C(=O)NH₂] | Me | Me | H | O |
| C-13827 | Me(R) | single bond | H | *c*-Pr[1-C(=O)NH₂] | Me | Me | H | O |
| C-13828 | Me(S) | single bond | H | *c*-Pr[1-C(=O)NH₂] | Me | Me | H | O |
| C-13829 | H | single bond | Me | *c*-Pr(1-OMe) | Me | Cl | H | O |
| C-13830 | Me | single bond | H | *c*-Pr(1-OMe) | Me | Cl | H | O |
| C-13831 | Me(R) | single bond | H | *c*-Pr(1-OMe) | Me | Cl | H | O |
| C-13832 | Me(S) | single bond | H | *c*-Pr(1-OMe) | Me | Cl | H | O |
| C-13833 | H | single bond | Me | *c*-Pr(1-OMe) | Me | Me | H | O |
| C-13834 | Me | single bond | H | *c*-Pr(1-OMe) | Me | Me | H | O |

**[Table 543]**

| Compound Number | Y | W | Z | R¹ | R² | R³ | R¹⁰ | A |
|---|---|---|---|---|---|---|---|---|
| C-13835 | Me(R) | single bond | H | *c*-Pr(1-OMe) | Me | Me | H | O |
| C-13836 | Me(S) | single bond | H | *c*-Pr(1-OMe) | Me | Me | H | O |
| C-13837 | H | single bond | Me | *c*-Pr(1-SMe) | Me | Cl | H | O |
| C-13838 | Me | single bond | H | *c*-Pr(1-SMe) | Me | Cl | H | O |
| C-13839 | Me(R) | single bond | H | *c*-Pr(1-SMe) | Me | Cl | H | O |
| C-13840 | Me(S) | single bond | H | *c*-Pr(1-SMe) | Me | Cl | H | O |
| C-13841 | H | single bond | Me | *c*-Pr(1-SMe) | Me | Me | H | O |
| C-13842 | Me | single bond | H | *c*-Pr(1-SMe) | Me | Me | H | O |
| C-13843 | Me(R) | single bond | H | *c*-Pr(1-SMe) | Me | Me | H | O |
| C-13844 | Me(S) | single bond | H | *c*-Pr(1-SMe) | Me | Me | H | O |
| C-13845 | H | single bond | Me | *c*-Pr[1-S(=O)Me] | Me | Cl | H | O |
| C-13846 | Me | single bond | H | *c*-Pr[1-S(=O)Me] | Me | Cl | H | O |
| C-13847 | Me(R) | single bond | H | *c*-Pr[1-S(=O)Me] | Me | Cl | H | O |
| C-13848 | Me(S) | single bond | H | *c*-Pr[1-S(=O)Me] | Me | Cl | H | O |
| C-13849 | H | single bond | Me | *c*-Pr[1-S(=O)Me] | Me | Me | H | O |
| C-13850 | Me | single bond | H | *c*-Pr[1-S(=O)Me] | Me | Me | H | O |
| C-13851 | Me(R) | single bond | H | *c*-Pr[1-S(=O)Me] | Me | Me | H | O |
| C-13852 | Me(S) | single bond | H | *c*-Pr[1-S(=O)Me] | Me | Me | H | O |
| C-13853 | H | single bond | Me | *c*-Pr[1-S(=O)₂Me] | Me | Cl | H | O |
| C-13854 | Me | single bond | H | *c*-Pr[1-S(=O)₂Me] | Me | Cl | H | O |
| C-13855 | Me(R) | single bond | H | *c*-Pr[1-S(=O)₂Me] | Me | Cl | H | O |
| C-13856 | Me(S) | single bond | H | *c*-Pr[1-S(=O)₂Me] | Me | Cl | H | O |
| C-13857 | H | single bond | Me | *c*-Pr[1-S(=O)₂Me] | Me | Me | H | O |
| C-13858 | Me | single bond | H | *c*-Pr[1-S(=O)₂Me] | Me | Me | H | O |
| C-13859 | Me(R) | single bond | H | *c*-Pr[1-S(=O)₂Me] | Me | Me | H | O |
| C-13860 | Me(S) | single bond | H | *c*-Pr[1-S(=O)₂Me] | Me | Me | H | O |
| C-13861 | H | single bond | Me | *c*-Pr[1-S(=O)₂CHF₂] | Me | Cl | H | O |
| C-13862 | Me | single bond | H | *c*-Pr[1-S(=O)₂CHF₂] | Me | Cl | H | O |
| C-13863 | Me(R) | single bond | H | *c*-Pr[1-S(=O)₂CHF₂] | Me | Cl | H | O |
| C-13864 | Me(S) | single bond | H | *c*-Pr[1-S(=O)₂CHF₂] | Me | Cl | H | O |
| C-13865 | H | single bond | Me | *c*-Pr[1-S(=O)₂CHF₂] | Me | Me | H | O |
| C-13866 | Me | single bond | H | *c*-Pr[1-S(=O)₂CHF₂] | Me | Me | H | O |
| C-13867 | Me(R) | single bond | H | *c*-Pr[1-S(=O)₂CHF₂] | Me | Me | H | O |
| C-13868 | Me(S) | single bond | H | *c*-Pr[1-S(=O)₂CHF₂] | Me | Me | H | O |
| C-13869 | H | single bond | Me | *c*-Pr[1-S(=O)₂CF₃] | Me | Cl | H | O |
| C-13870 | Me | single bond | H | *c*-Pr[1-S(=O)₂CF₃] | Me | Cl | H | O |
| C-13871 | Me(R) | single bond | H | *c*-Pr[1-S(=O)₂CF₃] | Me | Cl | H | O |
| C-13872 | Me(S) | single bond | H | *c*-Pr[1-S(=O)₂CF₃] | Me | Cl | H | O |
| C-13873 | H | single bond | Me | *c*-Pr[1-S(=O)₂CF₃] | Me | Me | H | O |
| C-13874 | Me | single bond | H | *c*-Pr[1-S(=O)₂CF₃] | Me | Me | H | O |
| C-13875 | Me(R) | single bond | H | *c*-Pr[1-S(=O)₂CF₃] | Me | Me | H | O |
| C-13876 | Me(S) | single bond | H | *c*-Pr[1-S(=O)₂CF₃] | Me | Me | H | O |
| C-13877 | H | single bond | Me | *c*-Pr[1-(5,6-dihydro-1,4,2-dioxazin-3-yl)] | Me | Cl | H | O |
| C-13878 | Me | single bond | H | *c*-Pr[1-(5,6-dihydro-1,4,2-dioxazin-3-yl)] | Me | Cl | H | O |

**[Table 544]**

| Compound Number | Y | W | z | R¹ | R² | R³ | R¹⁰ | A |
|---|---|---|---|---|---|---|---|---|
| C-13879 | Me(R) | single bond | H | *c*-Pr[1-(5,6-dihydro-1,4,2-dioxazin-3-yl)] | Me | Cl | H | O |
| C-13880 | Me(S) | single bond | H | *c*-Pr[1-(5,6-dihydro-1,4,2-dioxazin-3-yl)] | Me | Cl | H | O |
| C-13881 | H | single bond | Me | *c*-Pr[1-(5,6-dihydro-1,4,2-dioxazin-3-yl)] | Me | Me | H | O |
| C-13882 | Me | single bond | H | *c*-Pr[1-(5,6-dihydro-1,4,2-dioxazin-3-yl)] | Me | Me | H | O |
| C-13883 | Me(R) | single bond | H | *c*-Pr[1-(5,6-dihydro-1,4,2-dioxazin-3-yl)] | Me | Me | H | O |
| C-13884 | Me(S) | single bond | H | *c*-Pr[1-(5,6-dihydro-1,4,2-dioxazin-3-yl)] | Me | Me | H | O |
| C-13885 | H | single bond | Me | *c*-Pr[1-(5-hydroxy-1,3,4-oxadiazol-2-yl)] | Me | Cl | H | O |
| C-13886 | Me | single bond | H | *c*-Pr[1-(5-hydroxy-1,3,4-oxadiazol-2-yl)] | Me | Cl | H | O |
| C-13887 | Me(R) | single bond | H | *c*-Pr[1-(5-hydroxy-1,3,4-oxadiazol-2-yl)] | Me | Cl | H | O |
| C-13888 | Me(S) | single bond | H | *c*-Pr[1-(5-hydroxy-1,3,4-oxadiazol-2-yl)] | Me | Cl | H | O |
| C-13889 | H | single bond | Me | *c*-Pr[1-(5-hydroxy-1,3,4-oxadiazol-2-yl)] | Me | Me | H | O |
| C-13890 | Me | single bond | H | *c*-Pr[1-(5-hydroxy-1,3,4-oxadiazol-2-yl)] | Me | Me | H | O |
| C-13891 | Me(R) | single bond | H | *c*-Pr[1-(5-hydroxy-1,3,4-oxadiazol-2-yl)] | Me | Me | H | O |
| C-13892 | Me(S) | single bond | H | *c*-Pr[1-(5-hydroxy-1,3,4-oxadiazol-2-yl)] | Me | Me | H | O |
| C-13893 | H | single bond | Me | *c*-Pr[1-(pyrimidin-2-yl)] | Me | Cl | H | O |
| C-13894 | Me | single bond | H | *c*-Pr[1-(pyrimidin-2-yl)] | Me | Cl | H | O |
| C-13895 | Me(R) | single bond | H | *c*-Pr[1-(pyrimidin-2-yl)] | Me | Cl | H | O |
| C-13896 | Me(S) | single bond | H | *c*-Pr[1-(pyrimidin-2-yl)] | Me | Cl | H | O |
| C-13897 | H | single bond | Me | *c*-Pr[1-(pyrimidin-2-yl)] | Me | Me | H | O |
| C-13898 | Me | single bond | H | *c*-Pr[1-(pyrimidin-2-yl)] | Me | Me | H | O |
| C-13899 | Me(R) | single bond | H | *c*-Pr[1-(pyrimidin-2-yl)] | Me | Me | H | O |
| C-13900 | Me(S) | single bond | H | *c*-Pr[1-(pyrimidin-2-yl)] | Me | Me | H | O |
| C-13901 | H | single bond | Me | *t*-Bu | Me | Cl | H | O |
| C-13902 | Me | single bond | H | *t*-Bu | Me | Cl | H | O |
| C-13903 | Me(R) | single bond | H | *t*-Bu | Me | Cl | H | O |
| C-13904 | Me(S) | single bond | H | *t*-Bu | Me | Cl | H | O |
| C-13905 | H | single bond | Me | *t*-Bu | Me | Me | H | O |
| C-13906 | Me | single bond | H | *t*-Bu | Me | Me | H | O |
| C-13907 | Me(R) | single bond | H | *t*-Bu | Me | Me | H | O |
| C-13908 | Me(S) | single bond | H | *t*-Bu | Me | Me | H | O |
| C-13909 | H | single bond | Me | *c*-Bu | Me | Cl | H | O |
| C-13910 | Me | single bond | H | *c*-Bu | Me | Cl | H | O |
| C-13911 | Me(R) | single bond | H | *c*-Bu | Me | Cl | H | O |
| C-13912 | Me(S) | single bond | H | *c*-Bu | Me | Cl | H | O |
| C-13913 | H | single bond | Me | *c*-Bu | Me | Me | H | O |
| C-13914 | Me | single bond | H | *c*-Bu | Me | Me | H | O |
| C-13915 | Me(R) | single bond | H | *c*-Bu | Me | Me | H | O |
| C-13916 | Me(S) | single bond | H | *c*-Bu | Me | Me | H | O |
| C-13917 | H | single bond | Me | *c*-Bu(1-CN) | Me | Cl | H | O |
| C-13918 | Me | single bond | H | *c*-Bu(1-CN) | Me | Cl | H | O |
| C-13919 | Me(R) | single bond | H | *c*-Bu(1-CN) | Me | Cl | H | O |
| C-13920 | Me(S) | single bond | H | *c*-Bu(1-CN) | Me | Cl | H | O |
| C-13921 | H | single bond | Me | *c*-Bu(1-CN) | Me | Me | H | O |
| C-13922 | Me | single bond | H | *c*-Bu(1-CN) | Me | Me | H | O |

**[Table 545]**

| Compound Number | Y | W | Z | R¹ | R² | R³ | R¹⁰ | A |
|---|---|---|---|---|---|---|---|---|
| C-13923 | Me(R) | single bond | H | *c*-Bu(1-CN) | Me | Me | H | O |
| C-13924 | Me(S) | single bond | H | *c*-Bu(1-CN) | Me | Me | H | O |
| C-13925 | H | single bond | Me | *c*-Bu(3,3-F₂) | Me | Cl | H | O |
| C-13926 | Me | single bond | H | *c*-Bu(3,3-F₂) | Me | Cl | H | O |
| C-13927 | Me(R) | single bond | H | *c*-Bu(3,3-F₂) | Me | Cl | H | O |
| C-13928 | Me(S) | single bond | H | *c*-Bu(3,3-F₂) | Me | Cl | H | O |
| C-13929 | H | single bond | Me | *c*-Bu(3,3-F₂) | Me | Me | H | O |
| C-13930 | Me | single bond | H | *c*-Bu(3,3-F₂) | Me | Me | H | O |
| C-13931 | Me(R) | single bond | H | *c*-Bu(3,3-F₂) | Me | Me | H | O |
| C-13932 | Me(S) | single bond | H | *c*-Bu(3,3-F₂) | Me | Me | H | O |
| C-13933 | H | single bond | Me | oxetan-3-yl | Me | Cl | H | O |
| C-13934 | Me | single bond | H | oxetan-3-yl | Me | Cl | H | O |
| C-13935 | Me(R) | single bond | H | oxetan-3-yl | Me | Cl | H | O |
| C-13936 | Me(S) | single bond | H | oxetan-3-yl | Me | Cl | H | O |
| C-13937 | H | single bond | Me | oxetan-3-yl | Me | Me | H | O |
| C-13938 | Me | single bond | H | oxetan-3-yl | Me | Me | H | O |
| C-13939 | Me(R) | single bond | H | oxetan-3-yl | Me | Me | H | O |
| C-13940 | Me(S) | single bond | H | oxetan-3-yl | Me | Me | H | O |
| C-13941 | H | single bond | Me | oxetan(3-CN)-3-yl | Me | Cl | H | O |
| C-13942 | Me | single bond | H | oxetan(3-CN)-3-yl | Me | Cl | H | O |
| C-13943 | Me(R) | single bond | H | oxetan(3-CN)-3-yl | Me | Cl | H | O |
| C-13944 | Me(S) | single bond | H | oxetan(3-CN)-3-yl | Me | Cl | H | O |
| C-13945 | H | single bond | Me | oxetan(3-CN)-3-yl | Me | Me | H | O |
| C-13946 | Me | single bond | H | oxetan(3-CN)-3-yl | Me | Me | H | O |
| C-13947 | Me(R) | single bond | H | oxetan(3-CN)-3-yl | Me | Me | H | O |
| C-13948 | Me(S) | single bond | H | oxetan(3-CN)-3-yl | Me | Me | H | O |
| C-13949 | H | single bond | Me | azetidin[1-C(=O)Me]-3-yl | Me | Cl | H | O |
| C-13950 | Me | single bond | H | azetidin[1-C(=O)Me]-3-yl | Me | Cl | H | O |
| C-13951 | Me(R) | single bond | H | azetidin[1-C(=O)Me]-3-yl | Me | Cl | H | O |
| C-13952 | Me(S) | single bond | H | azetidin[1-C(=O)Me]-3-yl | Me | Cl | H | O |
| C-13953 | H | single bond | Me | azetidin[1-C(=O)Me]-3-yl | Me | Me | H | O |
| C-13954 | Me | single bond | H | azetidin[1-C(=O)Me]-3-yl | Me | Me | H | O |
| C-13955 | Me(R) | single bond | H | azetidin[1-C(=O)Me]-3-yl | Me | Me | H | O |
| C-13956 | Me(S) | single bond | H | azetidin[1-C(=O)Me]-3-yl | Me | Me | H | O |
| C-13957 | H | single bond | Me | azetidin[1-S(=O)₂Me]-3-yl | Me | Cl | H | O |
| C-13958 | Me | single bond | H | azetidin[1-S(=O)₂Me]-3-yl | Me | Cl | H | O |
| C-13959 | Me(R) | single bond | H | azetidin[1-S(=O)₂Me]-3-yl | Me | Cl | H | O |
| C-13960 | Me(S) | single bond | H | azetidin[1-S(=O)₂Me]-3-yl | Me | Cl | H | O |
| C-13961 | H | single bond | Me | azetidin[1-S(=O)₂Me]-3-yl | Me | Me | H | O |
| C-13962 | Me | single bond | H | azetidin[1-S(=O)₂Me]-3-yl | Me | Me | H | O |
| C-13963 | Me(R) | single bond | H | azetidin[1-S(=O)₂Me]-3-yl | Me | Me | H | O |
| C-13964 | Me(S) | single bond | H | azetidin[1-S(=O)₂Me]-3-yl | Me | Me | H | O |
| C-13965 | H | single bond | Me | azetidin[1-S(=O)₂CHF₂]-3-yl | Me | Cl | H | O |
| C-13966 | Me | single bond | H | azetidin[1-S(=O)₂CHF₂]-3-yl | Me | Cl | H | O |

**[Table 546]**

| Compound Number | Y | W | Z | R¹ | R² | R³ | R¹⁰ | A . |
|---|---|---|---|---|---|---|---|---|
| C-13967 | Me(R) | single bond | H | azetidin[1-S(=O)₂CHF₂]-3-yl | Me | Cl | H | O |
| C-13968 | Me(S) | single bond | H | azetidin[1-S(=O)₂CHF₂]-3-yl | Me | Cl | H | O |
| C-13969 | H | single bond | Me | azetidin[1-S(=O)₂CHF₂]-3-yl | Me | Me | H | O |
| C-13970 | Me | single bond | H | azetidin[1-S(=O)₂CHF₂]-3-yl | Me | Me | H | O |
| C-13971 | Me(R) | single bond | H | azetidin[1-S(=O)₂CHF₂]-3-yl | Me | Me | H | O |
| C-13972 | Me(S) | single bond | H | azetidin[1-S(=O)₂CHF₂]-3-yl | Me | Me | H | O |
| C-13973 | H | single bond | Me | *c*-Pen | Me | Cl | H | O |
| C-13974 | Me | single bond | H | *c*-Pen | Me | Cl | H | O |
| C-13975 | Me(R) | single bond | H | *c*-Pen | Me | Cl | H | O |
| C-13976 | Me(S) | single bond | H | *c*-Pen | Me | Cl | H | O |
| C-13977 | H | single bond | Me | *c*-Pen | Me | Me | H | O |
| C-13978 | Me | single bond | H | *c*-Pen | Me | Me | H | O |
| C-13979 | Me(R) | single bond | H | *c*-Pen | Me | Me | H | O |
| C-13980 | Me(S) | single bond | H | *c*-Pen | Me | Me | H | O |
| C-13981 | H | single bond | Me | tetrahydrofuran-3-yl | Me | Cl | H | O |
| C-13982 | Me | single bond | H | tetrahydrofuran-3-yl | Me | Cl | H | O |
| C-13983 | Me(R) | single bond | H | tetrahydrofuran-3-yl | Me | Cl | H | O |
| C-13984 | Me(S) | single bond | H | tetrahydrofuran-3-yl | Me | Cl | H | O |
| C-13985 | H | single bond | Me | tetrahydrofuran-3-yl | Me | Me | H | O |
| C-13986 | Me | single bond | H | tetrahydrofuran-3-yl | Me | Me | H | O |
| C-13987 | Me(R) | single bond | H | tetrahydrofuran-3-yl | Me | Me | H | O |
| C-13988 | Me(S) | single bond | H | tetra hydrofuran-3-yl | Me | Me | H | O |
| C-13989 | H | single bond | Me | tetrahydrothiophen-3-yl | Me | Cl | H | O |
| C-13990 | Me | single bond | H | tetrahydrothiophen-3-yl | Me | Cl | H | O |
| C-13991 | Me(R) | single bond | H | tetrahydrothiophen-3-yl | Me | Cl | H | O |
| C-13992 | Me(S) | single bond | H | tetrahydrothiophen-3-yl | Me | Cl | H | O |
| C-13993 | H | single bond | Me | tetrahydrothiophen-3-yl | Me | Me | H | O |
| C-13994 | Me | single bond | H | tetrahydrothiophen-3-yl | Me | Me | H | O |
| C-13995 | Me(R) | single bond | H | tetrahydrothiophen-3-yl | Me | Me | H | O |
| C-13996 | Me(S) | single bond | H | tetrahydrothiophen-3-yl | Me | Me | H | O |
| C-13997 | H | single bond | Me | *c*-Pen(1-CN) | Me | Cl | H | O |
| C-13998 | Me | single bond | H | *c*-Pen(1-CN) | Me | Cl | H | O |
| C-13999 | Me(R) | single bond | H | *c*-Pen(1-CN) | Me | Cl | H | O |
| C-14000 | Me(S) | single bond | H | *c*-Pen(1-CN) | Me | Cl | H | O |
| C-14001 | H | single bond | Me | *c*-Pen(1-CN) | Me | Me | H | O |
| C-14002 | Me | single bond | H | *c*-Pen(1-CN) | Me | Me | H | O |
| C-14003 | Me(R) | single bond | H | *c*-Pen(1-CN) | Me | Me | H | O |
| C-14004 | Me(S) | single bond | H | *c*-Pen(1-CN) | Me | Me | H | O |
| C-14005 | H | single bond | Me | tetrahydrofuran(3-CN)-3-yl | Me | Cl | H | O |
| C-14006 | Me | single bond | H | tetrahydrofuran(3-CN)-3-yl | Me | Cl | H | O |
| C-14007 | Me(R) | single bond | H | tetrahydrofuran(3-CN)-3-yl | Me | Cl | H | O |
| C-14008 | Me(S) | single bond | H | tetrahydrofuran(3-CN)-3-yl | Me | Cl | H | O |
| C-14009 | H | single bond | Me | tetrahydrofuran(3-CN)-3-yl | Me | Me | H | O |
| C-14010 | Me | single bond | H | tetrahydrofuran(3-CN)-3-yl | Me | Me | H | O |

**[Table 547]**

| Compound Number | Y | W | Z | R¹ | R² | R³ | R¹⁰ | A |
|---|---|---|---|---|---|---|---|---|
| C-14011 | Me(R) | single bond | H | tetrahydrofuran(3-CN)-3-yl | Me | Me | H | O |
| C-14012 | Me(S) | single bond | H | tetrahydrofuran(3-CN)-3-yl | Me | Me | H | O |
| C-14013 | H | single bond | Me | tetrahydrothiophen(3-CN)-3-yl | Me | Cl | H | O |
| C-14014 | Me | single bond | H | tetrahydrothiophen(3-CN)-3-yl | Me | Cl | H | O |
| C-14015 | Me(R) | single bond | H | tetrahydrothiophen(3-CN)-3-yl | Me | Cl | H | O |
| C-14016 | Me(S) | single bond | H | tetrahydrothiophen(3-CN)-3-yl | Me | Cl | H | O |
| C-14017 | H | single bond | Me | tetrahydrothiophen(3-CN)-3-yl | Me | Me | H | O |
| C-14018 | Me | single bond | H | tetrahydrothiophen(3-CN)-3-yl | Me | Me | H | O |
| C-14019 | Me(R) | single bond | H | tetrahydrothiophen(3-CN)-3-yl | Me | Me | H | O |
| C-14020 | Me(S) | single bond | H | tetrahydrothiophen(3-CN)-3-yl | Me | Me | H | O |
| C-14021 | H | single bond | Me | bicyclo[1.1.1]pentan-1-yl | Me | Cl | H | O |
| C-14022 | Me | single bond | H | bicyclo[1.1.1]pentan-1-yl | Me | Cl | H | O |
| C-14023 | Me(R) | single bond | H | bicyclo[1.1.1]pentan-1-yl | Me | Cl | H | O |
| C-14024 | Me(S) | single bond | H | bicyclo[1.1.1]pentan-1-yl | Me | Cl | H | O |
| C-14025 | H | single bond | Me | bicyclo[1.1.1]pentan-1-yl | Me | Me | H | O |
| C-14026 | Me | single bond | H | bicyclo[1.1.1]pentan-1-yl | Me | Me | H | O |
| C-14027 | Me(R) | single bond | H | bicyclo[1.1.1]pentan-1-yl | Me | Me | H | O |
| C-14028 | Me(S) | single bond | H | bicyclo[1.1.1]pentan-1-yl | Me | Me | H | O |
| C-14029 | H | single bond | Me | *c*-Hex | Me | Cl | H | O |
| C-14030 | Me | single bond | H | *c*-Hex | Me | Cl | H | O |
| C-14031 | Me(R) | single bond | H | *c*-Hex | Me | Cl | H | O |
| C-14032 | Me(S) | single bond | H | *c*-Hex | Me | Cl | H | O |
| C-14033 | H | single bond | Me | *c*-Hex | Me | Me | H | O |
| C-14034 | Me | single bond | H | *c*-Hex | Me | Me | H | O |
| C-14035 | Me(R) | single bond | H | *c*-Hex | Me | Me | H | O |
| C-14036 | Me(S) | single bond | H | *c*-Hex | Me | Me | H | O |
| C-14037 | H | single bond | Me | tetrahydropyran-4-yl | Me | Cl | H | O |
| C-14038 | Me | single bond | H | tetrahydropyran-4-yl | Me | Cl | H | O |
| C-14039 | Me(R) | single bond | H | tetrahydropyran-4-yl | Me | Cl | H | O |
| C-14040 | Me(S) | single bond | H | tetrahydropyran-4-yl | Me | Cl | H | O |
| C-14041 | H | single bond | Me | tetrahydropyran-4-yl | Me | Me | H | O |
| C-14042 | Me | single bond | H | tetrahydropyran-4-yl | Me | Me | H | O |
| C-14043 | Me(R) | single bond | H | tetrahydropyran-4-yl | Me | Me | H | O |
| C-14044 | Me(S) | single bond | H | tetrahydropyran-4-yl | Me | Me | H | O |
| C-14045 | H | single bond | Me | tetrahydrothiopyran-4-yl | Me | Cl | H | O |
| C-14046 | Me | single bond | H | tetrahydrothiopyran-4-yl | Me | Cl | H | O |
| C-14047 | Me(R) | single bond | H | tetrahydrothiopyran-4-yl | Me | Cl | H | O |
| C-14048 | Me(S) | single bond | H | tetrahydrothiopyran-4-yl | Me | Cl | H | O |
| C-14049 | H | single bond | Me | tetrahydrothiopyran-4-yl | Me | Me | H | O |
| C-14050 | Me | single bond | H | tetrahydrothiopyran-4-yl | Me | Me | H | O |
| C-14051 | Me(R) | single bond | H | tetrahydrothiopyran-4-yl | Me | Me | H | O |
| C-14052 | Me(S) | single bond | H | tetrahydrothiopyran-4-yl | Me | Me | H | O |
| C-14053 | H | single bond | Me | *c*-Hex(1-CN) | Me | Cl | H | O |
| C-14054 | Me | single bond | H | *c*-Hex(1-CN) | Me | Cl | H | O |

**[Table 548]**

| Compound Number | Y | W | Z | R¹ | R² | R³ | R¹⁰ | A |
|---|---|---|---|---|---|---|---|---|
| C-14055 | Me(R) | single bond | H | *c*-Hex(1-CN) | Me | Cl | H | O |
| C-14056 | Me(S) | single bond | H | *c*-Hex(1-CN) | Me | Cl | H | O |
| C-14057 | H | single bond | Me | *c*-Hex(1-CN) | Me | Me | H | O |
| C-14058 | Me | single bond | H | *c*-Hex(1-CN) | Me | Me | H | O |
| C-14059 | Me(R) | single bond | H | *c*-Hex(1-CN) | Me | Me | H | O |
| C-14060 | Me(S) | single bond | H | *c*-Hex(1-CN) | Me | Me | H | O |
| C-14061 | H | single bond | Me | tetrahydropyran(4-CN)-4-yl | Me | Cl | H | O |
| C-14062 | Me | single bond | H | tetrahydropyran(4-CN)-4-yl | Me | Cl | H | O |
| C-14063 | Me(R) | single bond | H | tetrahydropyran(4-CN)-4-yl | Me | Cl | H | O |
| C-14064 | Me(S) | single bond | H | tetrahydropyran(4-CN)-4-yl | Me | Cl | H | O |
| C-14065 | H | single bond | Me | tetrahydropyran(4-CN)-4-yl | Me | Me | H | O |
| C-14066 | Me | single bond | H | tetrahydropyran(4-CN)-4-yl | Me | Me | H | O |
| C-14067 | Me(R) | single bond | H | tetrahydropyran(4-CN)-4-yl | Me | Me | H | O |
| C-14068 | Me(S) | single bond | H | tetrahydropyran(4-CN)-4-yl | Me | Me | H | O |
| C-14069 | H | single bond | Me | tetrahydrothiopyran(4-CN)-4-yl | Me | Cl | H | O |
| C-14070 | Me | single bond | H | tetrahydrothiopyran(4-CN)-4-yl | Me | Cl | H | O |
| C-14071 | Me(R) | single bond | H | tetrahydrothiopyran(4-CN)-4-yl | Me | Cl | H | O |
| C-14072 | Me(S) | single bond | H | tetrahydrothiopyran(4-CN)-4-yl | Me | Cl | H | O |
| C-14073 | H | single bond | Me | tetrahydrothiopyran(4-CN)-4-yl | Me | Me | H | O |
| C-14074 | Me | single bond | H | tetrahydrothiopyran(4-CN)-4-yl | Me | Me | H | O |
| C-14075 | Me(R) | single bond | H | tetrahydrothiopyran(4-CN)-4-yl | Me | Me | H | O |
| C-14076 | Me(S) | single bond | H | tetrahydrothiopyran(4-CN)-4-yl | Me | Me | H | O |
| C-14077 | H | single bond | Me | CH₂(C-Pr) | Me | Cl | H | O |
| C-14078 | Me | single bond | H | CH₂(C-Pr) | Me | Cl | H | O |
| C-14079 | Me(R) | single bond | H | CH₂(C-Pr) | Me | Cl | H | O |
| C-14080 | Me(S) | single bond | H | CH₂(C-Pr) | Me | Cl | H | O |
| C-14081 | H | single bond | Me | CH₂(C-Pr) | Me | Me | H | O |
| C-14082 | Me | single bond | H | CH₂(C-Pr) | Me | Me | H | O |
| C-14083 | Me(R) | single bond | H | CH₂(C-Pr) | Me | Me | H | O |
| C-14084 | Me(S) | single bond | H | CH₂(C-Pr) | Me | Me | H | O |
| C-14085 | H | single bond | Me | CH₂[*c*-Pr(1-CN)] | Me | Cl | H | O |
| C-14086 | Me | single bond | H | CH₂[*c*-Pr(1-CN)] | Me | Cl | H | O |
| C-14087 | Me(R) | single bond | H | CH₂[*c*-Pr(1-CN)] | Me | Cl | H | O |
| C-14088 | Me(S) | single bond | H | CH₂[*c*-Pr(1-CN)] | Me | Cl | H | O |
| C-14089 | H | single bond | Me | CH₂[*c*-Pr(1-CN)] | Me | Me | H | O |
| C-14090 | Me | single bond | H | CH₂[*c*-Pr(1-CN)] | Me | Me | H | O |
| C-14091 | Me(R) | single bond | H | CH₂[*c*-Pr(1-CN)] | Me | Me | H | O |
| C-14092 | Me(S) | single bond | H | CH₂[*c*-Pr(1-CN)] | Me | Me | H | O |
| C-14093 | H | single bond | Me | CH(Me)(*c*-Pr) | Me | Cl | H | O |
| C-14094 | Me | single bond | H | CH(Me)(*c*-Pr) | Me | Cl | H | O |
| C-14095 | Me(R) | single bond | H | CH(Me)(*c*-Pr) | Me | Cl | H | O |
| C-14096 | Me(S) | single bond | H | CH(Me)(*c*-Pr) | Me | Cl | H | O |
| C-14097 | H | single bond | Me | CH(Me)(*c*-Pr) | Me | Me | H | O |
| C-14098 | Me | single bond | H | CH(Me)(*c*-Pr) | Me | Me | H | O |

**[Table 549]**

| Compound Number | Y | W | Z | R¹ | R² | R³ | R¹⁰ | A |
|---|---|---|---|---|---|---|---|---|
| C-14099 | Me(R) | single bond | H | CH(Me)(*c*-Pr) | Me | Me | H | O |
| C-14100 | Me(S) | single bond | H | CH(Me)(*c*-Pr) | Me | Me | H | O |
| C-14101 | H | single bond | Me | CH(Me)CH₂OMe | Me | Cl | H | O |
| C-14102 | Me | single bond | H | CH(Me)CH₂OMe | Me | Cl | H | O |
| C-14103 | Me(R) | single bond | H | CH(Me)CH₂OMe | Me | Cl | H | O |
| C-14104 | Me(S) | single bond | H | CH(Me)CH₂OMe | Me | Cl | H | O |
| C-14105 | H | single bond | Me | CH(Me)CH₂OMe | Me | Me | H | O |
| C-14106 | Me | single bond | H | CH(Me)CH₂OMe | Me | Me | H | O |
| C-14107 | Me(R) | single bond | H | CH(Me)CH₂OMe | Me | Me | H | O |
| C-14108 | Me(S) | single bond | H | CH(Me)CH₂OMe | Me | Me | H | O |
| C-14109 | H | single bond | Me | CH₂CH=CH₂ | Me | Cl | H | O |
| C-14110 | Me | single bond | H | CH₂CH=CH₂ | Me | Cl | H | O |
| C-14111 | Me(R) | single bond | H | CH₂CH=CH₂ | Me | Cl | H | O |
| C-14112 | Me(S) | single bond | H | CH₂CH=CH₂ | Me | Cl | H | O |
| C-14113 | H | single bond | Me | CH₂CH=CH₂ | Me | Me | H | O |
| C-14114 | Me | single bond | H | CH₂CH=CH₂ | Me | Me | H | O |
| C-14115 | Me(R) | single bond | H | CH₂CH=CH₂ | Me | Me | H | O |
| C-14116 | Me(S) | single bond | H | CH₂CH=CH₂ | Me | Me | H | O |
| C-14117 | H | single bond | Me | CH₂C≡CH | Me | Cl | H | O |
| C-14118 | Me | single bond | H | CH₂C≡CH | Me | Cl | H | O |
| C-14119 | Me(R) | single bond | H | CH₂C≡CH | Me | Cl | H | O |
| C-14120 | Me(S) | single bond | H | CH₂C≡CH | Me | Cl | H | O |
| C-14121 | H | single bond | Me | CH₂C≡CH | Me | Me | H | O |
| C-14122 | Me | single bond | H | CH₂C≡CH | Me | Me | H | O |
| C-14123 | Me(R) | single bond | H | CH₂C≡CH | Me | Me | H | O |
| C-14124 | Me(S) | single bond | H | CH₂C≡CH | Me | Me | H | O |
| C-14125 | H | single bond | Me | CH₂CN | Me | Cl | H | O |
| C-14126 | Me | single bond | H | CH₂CN | Me | Cl | H | O |
| C-14127 | Me(R) | single bond | H | CH₂CN | Me | Cl | H | O |
| C-14128 | Me(S) | single bond | H | CH₂CN | Me | Cl | H | O |
| C-14129 | H | single bond | Me | CH₂CN | Me | Me | H | O |
| C-14130 | Me | single bond | H | CH₂CN | Me | Me | H | O |
| C-14131 | Me(R) | single bond | H | CH₂CN | Me | Me | H | O |
| C-14132 | Me(S) | single bond | H | CH₂CN | Me | Me | H | O |
| C-14133 | H | single bond | Me | CH(Me)CN | Me | Cl | H | O |
| C-14134 | Me | single bond | H | CH(Me)CN | Me | Cl | H | O |
| C-14135 | Me(R) | single bond | H | CH(Me)CN | Me | Cl | H | O |
| C-14136 | Me(S) | single bond | H | CH(Me)CN | Me | Cl | H | O |
| C-14137 | H | single bond | Me | CH(Me)CN | Me | Me | H | O |
| C-14138 | Me | single bond | H | CH(Me)CN | Me | Me | H | O |
| C-14139 | Me(R) | single bond | H | CH(Me)CN | Me | Me | H | O |
| C-14140 | Me(S) | single bond | H | CH(Me)CN | Me | Me | H | O |
| C-14141 | H | single bond | Me | C(Me)₂CN | Me | Cl | H | O |
| C-14142 | Me | single bond | H | C(Me)₂CN | Me | Cl | H | O |

**[Table 550]**

| Compound Number | Y | W | Z | R¹ | R² | R³ | R¹⁰ | A |
|---|---|---|---|---|---|---|---|---|
| C-14143 | Me(R) | single bond | H | C(Me)₂CN | Me | Cl | H | O |
| C-14144 | Me(S) | single bond | H | C(Me)₂CN | Me | Cl | H | O |
| C-14145 | H | single bond | Me | C(Me)₂CN | Me | Me | H | O |
| C-14146 | Me | single bond | H | C(Me)₂CN | Me | Me | H | O |
| C-14147 | Me(R) | single bond | H | C(Me)₂CN | Me | Me | H | O |
| C-14148 | Me(S) | single bond | H | C(Me)₂CN | Me | Me | H | O |
| C-14149 | H | single bond | Me | C(Me)(*i*-Pr)CN | Me | Cl | H | O |
| C-14150 | Me | single bond | H | C(Me)(*i*-Pr)CN | Me | Cl | H | O |
| C-14151 | Me(R) | single bond | H | C(Me)(*i*-Pr)CN | Me | Cl | H | O |
| C-14152 | Me(S) | single bond | H | C(Me)(*i*-Pr)CN | Me | Cl | H | O |
| C-14153 | H | single bond | Me | C(Me)(*i*-Pr)CN | Me | Me | H | O |
| C-14154 | Me | single bond | H | C(Me)(*i*-Pr)CN | Me | Me | H | O |
| C-14155 | Me(R) | single bond | H | C(Me)(*i*-Pr)CN | Me | Me | H | O |
| C-14156 | Me(S) | single bond | H | C(Me)(*i*-Pr)CN | Me | Me | H | O |
| C-14157 | H | single bond | Me | OMe | Me | Cl | H | O |
| C-14158 | Me | single bond | H | OMe | Me | Cl | H | O |
| C-14159 | Me(R) | single bond | H | OMe | Me | Cl | H | O |
| C-14160 | Me(S) | single bond | H | OMe | Me | Cl | H | O |
| C-14161 | H | single bond | Me | OMe | Me | Me | H | O |
| C-14162 | Me | single bond | H | OMe | Me | Me | H | O |
| C-14163 | Me(R) | single bond | H | OMe | Me | Me | H | O |
| C-14164 | Me(S) | single bond | H | OMe | Me | Me | H | O |
| C-14165 | H | single bond | Me | CH=NOMe | Me | Cl | H | O |
| C-14166 | Me | single bond | H | CH=NOMe | Me | Cl | H | O |
| C-14167 | Me(R) | single bond | H | CH=NOMe | Me | Cl | H | O |
| C-14168 | Me(S) | single bond | H | CH=NOMe | Me | Cl | H | O |
| C-14169 | H | single bond | Me | CH=NOMe | Me | Me | H | O |
| C-14170 | Me | single bond | H | CH=NOMe | Me | Me | H | O |
| C-14171 | Me(R) | single bond | H | CH=NOMe | Me | Me | H | O |
| C-14172 | Me(S) | single bond | H | CH=NOMe | Me | Me | H | O |
| C-14173 | H | single bond | Me | morpholin-4-yl | Me | Cl | H | O |
| C-14174 | Me | single bond | H | morpholin-4-yl | Me | Cl | H | O |
| C-14175 | Me(R) | single bond | H | morpholin-4-yl | Me | Cl | H | O |
| C-14176 | Me(S) | single bond | H | morpholin-4-yl | Me | Cl | H | O |
| C-14177 | H | single bond | Me | morpholin-4-yl | Me | Me | H | O |
| C-14178 | Me | single bond | H | morpholin-4-yl | Me | Me | H | O |
| C-14179 | Me(R) | single bond | H | morpholin-4-yl | Me | Me | H | O |
| C-14180 | Me(S) | single bond | H | morpholin-4-yl | Me | Me | H | O |
| C-14181 | H | single bond | Me | Ph | Me | Cl | H | O |
| C-14182 | Me | single bond | H | Ph | Me | Cl | H | O |
| C-14183 | Me(R) | single bond | H | Ph | Me | Cl | H | O |
| C-14184 | Me(S) | single bond | H | Ph | Me | Cl | H | O |
| C-14185 | H | single bond | Me | Ph | Me | Me | H | O |
| C-14186 | Me | single bond | H | Ph | Me | Me | H | O |

**[Table 551]**

| Compound Number | Y | W | Z | R¹ | R² | R³ | R¹⁰ | A |
|---|---|---|---|---|---|---|---|---|
| C-14187 | Me(R) | single bond | H | Ph | Me | Me | H | O |
| C-14188 | Me(S) | single bond | H | Ph | Me | Me | H | O |
| C-14189 | H | single bond | Me | CH₂Ph | Me | Cl | H | O |
| C-14190 | Me | single bond | H | CH₂Ph | Me | Cl | H | O |
| C-14191 | Me(R) | single bond | H | CH₂Ph | Me | Cl | H | O |
| C-14192 | Me(S) | single bond | H | CH₂Ph | Me | Cl | H | O |
| C-14193 | H | single bond | Me | CH₂Ph | Me | Me | H | O |
| C-14194 | Me | single bond | H | CH₂Ph | Me | Me | H | O |
| C-14195 | Me(R) | single bond | H | CH₂Ph | Me | Me | H | O |
| C-14196 | Me(S) | single bond | H | CH₂Ph | Me | Me | H | O |
| C-14197 | H | single bond | Me | thiazole-2-yl | Me | Cl | H | O |
| C-14198 | Me | single bond | H | thiazole-2-yl | Me | Cl | H | O |
| C-14199 | Me(R) | single bond | H | thiazole-2-yl | Me | Cl | H | O |
| C-14200 | Me(S) | single bond | H | thiazole-2-yl | Me | Cl | H | O |
| C-14201 | H | single bond | Me | thiazole-2-yl | Me | Me | H | O |
| C-14202 | Me | single bond | H | thiazole-2-yl | Me | Me | H | O |
| C-14203 | Me(R) | single bond | H | thiazole-2-yl | Me | Me | H | O |
| C-14204 | Me(S) | single bond | H | thiazole-2-yl | Me | Me | H | O |
| C-14205 | H | single bond | Me | 1-Me-pyrazol-3-yl | Me | Cl | H | O |
| C-14206 | Me | single bond | H | 1-Me-pyrazol-3-yl | Me | Cl | H | O |
| C-14207 | Me(R) | single bond | H | 1-Me-pyrazol-3-yl | Me | Cl | H | O |
| C-14208 | Me(S) | single bond | H | 1-Me-pyrazol-3-yl | Me | Cl | H | O |
| C-14209 | H | single bond | Me | 1-Me-pyrazol-3-yl | Me | Me | H | O |
| C-14210 | Me | single bond | H | 1-Me-pyrazol-3-yl | Me | Me | H | O |
| C-14211 | Me(R) | single bond | H | 1-Me-pyrazol-3-yl | Me | Me | H | O |
| C-14212 | Me(S) | single bond | H | 1-Me-pyrazol-3-yl | Me | Me | H | O |
| C-14213 | H | single bond | Me | 1-Me-pyrazol-5-yl | Me | Cl | H | O |
| C-14214 | Me | single bond | H | 1-Me-pyrazol-5-yl | Me | Cl | H | O |
| C-14215 | Me(R) | single bond | H | 1-Me-pyrazol-5-yl | Me | Cl | H | O |
| C-14216 | Me(S) | single bond | H | 1-Me-pyrazol-5-yl | Me | Cl | H | O |
| C-14217 | H | single bond | Me | 1-Me-pyrazol-5-yl | Me | Me | H | O |
| C-14218 | Me | single bond | H | 1-Me-pyrazol-5-yl | Me | Me | H | O |
| C-14219 | Me(R) | single bond | H | 1-Me-pyrazol-5-yl | Me | Me | H | O |
| C-14220 | Me(S) | single bond | H | 1-Me-pyrazol-5-yl | Me | Me | H | O |
| C-14221 | H | single bond | Me | CH₂(pyrimidin-2-yl) | Me | Cl | H | O |
| C-14222 | Me | single bond | H | CH₂(pyrimidin-2-yl) | Me | Cl | H | O |
| C-14223 | Me(R) | single bond | H | CH₂(pyrimidin-2-yl) | Me | Cl | H | O |
| C-14224 | Me(S) | single bond | H | CH₂(pyrimidin-2-yl) | Me | Cl | H | O |
| C-14225 | H | single bond | Me | CH₂(pyrimidin-2-yl) | Me | Me | H | O |
| C-14226 | Me | single bond | H | CH₂(pyrimidin-2-yl) | Me | Me | H | O |
| C-14227 | Me(R) | single bond | H | CH₂(pyrimidin-2-yl) | Me | Me | H | O |
| C-14228 | Me(S) | single bond | H | CH₂(pyrimidin-2-yl) | Me | Me | H | O |
| C-14229 | H | single bond | Me | Me | Me | Cl | Me | O |
| C-14230 | Me | single bond | H | Me | Me | Cl | Me | O |

**[Table 552]**

| Compound Number | Y | W | Z | R¹ | R² | R³ | R¹⁰ | A |
|---|---|---|---|---|---|---|---|---|
| C-14231 | Me(R) | single bond | H | Me | Me | Cl | Me | O |
| C-14232 | Me(S) | single bond | H | Me | Me | Cl | Me | O |
| C-14233 | H | single bond | Me | Me | Me | Me | Me | O |
| C-14234 | Me | single bond | H | Me | Me | Me | Me | O |
| C-14235 | Me(R) | single bond | H | Me | Me | Me | Me | O |
| C-14236 | Me(S) | single bond | H | Me | Me | Me | Me | O |
| C-14237 | H | single bond | Me | CH₂CHF₂ | Me | Cl | Me | O |
| C-14238 | Me | single bond | H | CH₂CHF₂ | Me | Cl | Me | O |
| C-14239 | Me(R) | single bond | H | CH₂CHF₂ | Me | Cl | Me | O |
| C-14240 | Me(S) | single bond | H | CH₂CHF₂ | Me | Cl | Me | O |
| C-14241 | H | single bond | Me | CH₂CHF₂ | Me | Me | Me | O |
| C-14242 | Me | single bond | H | CH₂CHF₂ | Me | Me | Me | O |
| C-14243 | Me(R) | single bond | H | CH₂CHF₂ | Me | Me | Me | O |
| C-14244 | Me(S) | single bond | H | CH₂CHF₂ | Me | Me | Me | O |
| C-14245 | H | single bond | Me | *n*-Pr | Me | Cl | Me | O |
| C-14246 | Me | single bond | H | *n*-Pr | Me | Cl | Me | O |
| C-14247 | Me(R) | single bond | H | *n*-Pr | Me | Cl | Me | O |
| C-14248 | Me(S) | single bond | H | *n*-Pr | Me | Cl | Me | O |
| C-14249 | H | single bond | Me | *n*-Pr | Me | Me | Me | O |
| C-14250 | Me | single bond | H | *n*-Pr | Me | Me | Me | O |
| C-14251 | Me(R) | single bond | H | *n*-Pr | Me | Me | Me | O |
| C-14252 | Me(S) | single bond | *H* | *n*-Pr | Me | Me | Me | O |
| C-14253 | H | single bond | Me | *i*-Pr | Me | Cl | Me | O |
| C-14254 | Me | single bond | H | *i*-Pr | Me | Cl | Me | O |
| C-14255 | Me(R) | single bond | H | *i*-Pr | Me | Cl | Me | O |
| C-14256 | Me(S) | single bond | H | *i*-Pr | Me | Cl | Me | O |
| C-14257 | H | single bond | Me | *i*-Pr | Me | Me | Me | O |
| C-14258 | Me | single bond | H | *i*-Pr | Me | Me | Me | O |
| C-14259 | Me(R) | single bond | H | *i*-Pr | Me | Me | Me | O |
| C-14260 | Me(S) | single bond | H | *i*-Pr | Me | Me | Me | O |
| C-14261 | H | single bond | Me | *c*-Pr(1-F) | Me | Cl | Me | O |
| C-14262 | Me | single bond | H | *c*-Pr(1-F) | Me | Cl | Me | O |
| C-14263 | Me(R) | single bond | H | *c*-Pr(1-F) | Me | Cl | Me | O |
| C-14264 | Me(S) | single bond | H | *c*-Pr(1-F) | Me | Cl | Me | O |
| C-14265 | H | single bond | Me | *c*-Pr(1-F) | Me | Me | Me | O |
| C-14266 | Me | single bond | H | *c*-Pr(1-F) | Me | Me | Me | O |
| C-14267 | Me(R) | single bond | H | *c*-Pr(1-F) | Me | Me | Me | O |
| C-14268 | Me(S) | single bond | H | *c*-Pr(1-F) | Me | Me | Me | O |
| C-14269 | H | single bond | Me | *c*-Pr(2,2-F₂) | Me | Cl | Me | O |
| C-14270 | Me | single bond | H | *c*-Pr(2,2-F₂) | Me | Cl | Me | O |
| C-14271 | Me(R) | single bond | H | *c*-Pr(2,2-F₂) | Me | Cl | Me | O |
| C-14272 | Me(S) | single bond | H | *c*-Pr(2,2-F₂) | Me | Cl | Me | O |
| C-14273 | H | single bond | Me | *c*-Pr(2,2-F₂) | Me | Me | Me | O |
| C-14274 | Me | single bond | H | *c*-Pr(2,2-F₂) | Me | Me | Me | O |

**[Table 553]**

| Compound Number | Y | W | Z | R¹ | R² | R³ | R¹⁰ | A |
|---|---|---|---|---|---|---|---|---|
| C-14275 | Me(R) | single bond | H | *c*-Pr(2,2-F₂) | Me | Me | Me | O |
| C-14276 | Me(S) | single bond | H | *c*-Pr(2,2-F₂) | Me | Me | Me | O |
| C-14277 | H | single bond | Me | *c*-Pr(1-Cl) | Me | Cl | Me | O |
| C-14278 | Me | single bond | H | *c*-Pr(1-Cl) | Me | Cl | Me | O |
| C-14279 | Me(R) | single bond | H | *c*-Pr(1-Cl) | Me | Cl | Me | O |
| C-14280 | Me(S) | single bond | H | *c*-Pr(1-Cl) | Me | Cl | Me | O |
| C-14281 | H | single bond | Me | *c*-Pr(1-Cl) | Me | Me | Me | O |
| C-14282 | Me | single bond | H | *c*-Pr(1-Cl) | Me | Me | Me | O |
| C-14283 | Me(R) | single bond | H | *c*-Pr(1-Cl) | Me | Me | Me | O |
| C-14284 | Me(S) | single bond | H | *c*-Pr(1-Cl) | Me | Me | Me | O |
| C-14285 | H | single bond | Me | *c*-Pr(1-Br) | Me | Cl | Me | O |
| C-14286 | Me | single bond | H | *c*-Pr(1-Br) | Me | Cl | Me | O |
| C-14287 | Me(R) | single bond | H | *c*-Pr(1-Br) | Me | Cl | Me | O |
| C-14288 | Me(S) | single bond | H | *c*-Pr(1-Br) | Me | Cl | Me | O |
| C-14289 | H | single bond | Me | *c*-Pr(1-Br) | Me | Me | Me | O |
| C-14290 | Me | single bond | H | *c*-Pr(1-Br) | Me | Me | Me | O |
| C-14291 | Me(R) | single bond | H | *c*-Pr(1-Br) | Me | Me | Me | O |
| C-14292 | Me(S) | single bond | H | *c*-Pr(1-Br) | Me | Me | Me | O |
| C-14293 | H | single bond | Me | *c*-Pr(1-Me) | Me | Cl | Me | O |
| C-14294 | Me | single bond | H | *c*-Pr(1-Me) | Me | Cl | Me | O |
| C-14295 | Me(R) | single bond | H | *c*-Pr(1-Me) | Me | Cl | Me | O |
| C-14296 | Me(S) | single bond | H | *c*-Pr(1-Me) | Me | Cl | Me | O |
| C-14297 | H | single bond | Me | *c*-Pr(1-Me) | Me | Me | Me | O |
| C-14298 | Me | single bond | H | *c*-Pr(1-Me) | Me | Me | Me | O |
| C-14299 | Me(R) | single bond | H | *c*-Pr(1-Me) | Me | Me | Me | O |
| C-14300 | Me(S) | single bond | H | *c*-Pr(1-Me) | Me | Me | Me | O |
| C-14301 | H | single bond | Me | *c*-Pr(1-CH₂OH) | Me | Cl | Me | O |
| C-14302 | Me | single bond | H | *c*-Pr(1-CH₂OH) | Me | Cl | Me | O |
| C-14303 | Me(R) | single bond | H | *c*-Pr(1-CH₂OH) | Me | Cl | Me | O |
| C-14304 | Me(S) | single bond | H | *c*-Pr(1-CH₂OH) | Me | Cl | Me | O |
| C-14305 | H | single bond | Me | *c*-Pr(1-CH₂OH) | Me | Me | Me | O |
| C-14306 | Me | single bond | H | *c*-Pr(1-CH₂OH) | Me | Me | Me | O |
| C-14307 | Me(R) | single bond | H | *c*-Pr(1-CH₂OH) | Me | Me | Me | O |
| C-14308 | Me(S) | single bond | H | *c*-Pr(1-CH₂OH) | Me | Me | Me | O |
| C-14309 | H | single bond | Me | *c*-Pr[1-CH₂OS(=O)₂Me] | Me | Cl | Me | O |
| C-14310 | Me | single bond | H | *c*-Pr[1-CH₂OS(=O)₂Me] | Me | Cl | Me | O |
| C-14311 | Me(R) | single bond | H | *c*-Pr[1-CH₂OS(=O)₂Me] | Me | Cl | Me | O |
| C-14312 | Me(S) | single bond | H | *c*-Pr[1-CH₂OS(=O)₂Me] | Me | Cl | Me | O |
| C-14313 | H | single bond | Me | *c*-Pr[1-CH₂OS(=O)₂Me] | Me | Me | Me | O |
| C-14314 | Me | single bond | H | *c*-Pr[1-CH₂OS(=O)₂Me] | Me | Me | Me | O |
| C-14315 | Me(R) | single bond | H | *c*-Pr[1-CH₂OS(=O)₂Me] | Me | Me | Me | O |
| C-14316 | Me(S) | single bond | H | *c*-Pr[1-CH₂OS(=O)₂Me] | Me | Me | Me | O |
| C-14317 | H | single bond | Me | *c*-Pr(1-CH₂OMe) | Me | Cl | Me | O |
| C-14318 | Me | single bond | H | *c*-Pr(1-CH₂OMe) | Me | Cl | Me | O |

**[Table 554]**

| Compound Number | Y | W | Z | R¹ | R² | R³ | R¹⁰ | A |
|---|---|---|---|---|---|---|---|---|
| C-14319 | Me(R) | single bond | H | *c*-Pr(1-CH₂OMe) | Me | Cl | Me | O |
| C-14320 | Me(S) | single bond | H | *c*-Pr(1-CH₂OMe) | Me | Cl | Me | O |
| C-14321 | H | single bond | Me | *c*-Pr(1-CH₂OMe) | Me | Me | Me | O |
| C-14322 | Me | single bond | H | *c*-Pr(1-CH₂OMe) | Me | Me | Me | O |
| C-14323 | Me(R) | single bond | H | *c*-Pr(1-CH₂OMe) | Me | Me | Me | O |
| C-14324 | Me(S) | single bond | H | *c*-Pr(1-CH₂OMe) | Me | Me | Me | O |
| C-14325 | H | single bond | Me | *c*-Pr(1-CH₂CN) | Me | Cl | Me | O |
| C-14326 | Me | single bond | H | *c*-Pr(1-CH₂CN) | Me | Cl | Me | O |
| C-14327 | Me(R) | single bond | H | *c*-Pr(1-CH₂CN) | Me | Cl | Me | O |
| C-14328 | Me(S) | single bond | H | *c*-Pr(1-CH₂CN) | Me | Cl | Me | O |
| C-14329 | H | single bond | Me | *c*-Pr(1-CH₂CN) | Me | Me | Me | O |
| C-14330 | Me | single bond | H | *c*-Pr(1-CH₂CN) | Me | Me | Me | O |
| C-14331 | Me(R) | single bond | H | *c*-Pr(1-CH₂CN) | Me | Me | Me | O |
| C-14332 | Me(S) | single bond | H | *c*-Pr(1-CH₂CN) | Me | Me | Me | O |
| C-14333 | H | single bond | Me | *c*-Pr(1-CH₂SMe) | Me | Cl | Me | O |
| C-14334 | Me | single bond | H | *c*-Pr(1-CH₂SMe) | Me | Cl | Me | O |
| C-14335 | Me(R) | single bond | H | *c*-Pr(1-CH₂SMe) | Me | Cl | Me | O |
| C-14336 | Me(S) | single bond | H | *c*-Pr(1-CH₂SMe) | Me | Cl | Me | O |
| C-14337 | H | single bond | Me | *c*-Pr(1-CH₂SMe) | Me | Me | Me | O |
| C-14338 | Me | single bond | H | *c*-Pr(1-CH₂SMe) | Me | Me | Me | O |
| C-14339 | Me(R) | single bond | H | *c*-Pr(1-CH₂SMe) | Me | Me | Me | O |
| C-14340 | Me(S) | single bond | H | *c*-Pr(1-CH₂SMe) | Me | Me | Me | O |
| C-14341 | H | single bond | Me | *c*-Pr[1-CH₂S(=O)Me] | Me | Cl | Me | O |
| C-14342 | Me | single bond | H | *c*-Pr[1-CH₂S(=O)Me] | Me | Cl | Me | O |
| C-14343 | Me(R) | single bond | H | *c*-Pr[1-CH₂S(=O)Me] | Me | Cl | Me | O |
| C-14344 | Me(S) | single bond | H | *c*-Pr[1-CH₂S(=O)Me] | Me | Cl | Me | O |
| C-14345 | H | single bond | Me | *c*-Pr[1-CH₂S(=O)Me] | Me | Me | Me | O |
| C-14346 | Me | single bond | H | *c*-Pr[1-CH₂S(=O)Me] | Me | Me | Me | O |
| C-14347 | Me(R) | single bond | H | *c*-Pr[1-CH₂S(=O)Me] | Me | Me | Me | O |
| C-14348 | Me(S) | single bond | H | *c*-Pr[1-CH₂S(=O)Me] | Me | Me | Me | O |
| C-14349 | H | single bond | Me | *c*-Pr[1-CH₂S(=O)₂Me] | Me | Cl | Me | O |
| C-14350 | Me | single bond | H | *c*-Pr[1-CH₂S(=O)₂Me] | Me | Cl | Me | O |
| C-14351 | Me(R) | single bond | H | *c*-Pr[1-CH₂S(=O)₂Me] | Me | Cl | Me | O |
| C-14352 | Me(S) | single bond | H | *c*-Pr[1-CH₂S(=O)₂Me] | Me | Cl | Me | O |
| C-14353 | H | single bond | Me | *c*-Pr[1-CH₂S(=O)₂Me] | Me | Me | Me | O |
| C-14354 | Me | single bond | H | *c*-Pr[1-CH₂S(=O)₂Me] | Me | Me | Me | O |
| C-14355 | Me(R) | single bond | H | *c*-Pr[1-CH₂S(=O)₂Me] | Me | Me | Me | O |
| C-14356 | Me(S) | single bond | H | *c*-Pr[1-CH₂S(=O)₂Me] | Me | Me | Me | O |
| C-14357 | H | single bond | Me | *c*-Pr(1-CH₂F) | Me | Cl | Me | O |
| C-14358 | Me | single bond | H | *c*-Pr(1-CH₂F) | Me | Cl | Me | O |
| C-14359 | Me(R) | single bond | H | *c*-Pr(1-CH₂F) | Me | Cl | Me | O |
| C-14360 | Me(S) | single bond | H | *c*-Pr(1-CH₂F) | Me | Cl | Me | O |
| C-14361 | H | single bond | Me | *c*-Pr(1-CH₂F) | Me | Me | Me | O |
| C-14362 | Me | single bond | H | *c*-Pr(1-CH₂F) | Me | Me | Me | O |

**[Table 555]**

| Compound Number | Y | W | Z | R¹ | R² | R³ | R¹⁰ | A |
|---|---|---|---|---|---|---|---|---|
| C-14363 | Me(R) | single bond | H | *c*-Pr(1-CH₂F) | Me | Me | Me | O |
| C-14364 | Me(S) | single bond | H | *c*-Pr(1-CH₂F) | Me | Me | Me | O |
| C-14365 | H | single bond | Me | *c*-Pr(1-CHF₂) | Me | Cl | Me | O |
| C-14366 | Me | single bond | H | *c*-Pr(1-CHF₂) | Me | Cl | Me | O |
| C-14367 | Me(R) | single bond | H | *c*-Pr(1-CHF₂) | Me | Cl | Me | O |
| C-14368 | Me(S) | single bond | H | *c*-Pr(1-CHF₂) | Me | Cl | Me | O |
| C-14369 | H | single bond | Me | *c*-Pr(1-CHF₂) | Me | Me | Me | O |
| C-14370 | Me | single bond | H | *c*-Pr(1-CHF₂) | Me | Me | Me | O |
| C-14371 | Me(R) | single bond | H | *c*-Pr(1-CHF₂) | Me | Me | Me | O |
| C-14372 | Me(S) | single bond | H | *c*-Pr(1-CHF₂) | Me | Me | Me | O |
| C-14373 | H | single bond | Me | *c*-Pr(1-CF₃) | Me | Cl | Me | O |
| C-14374 | Me | single bond | H | *c*-Pr(1-CF₃) | Me | Cl | Me | O |
| C-14375 | Me(R) | single bond | H | *c*-Pr(1-CF₃) | Me | Cl | Me | O |
| C-14376 | Me(S) | single bond | H | *c*-Pr(1-CF₃) | Me | Cl | Me | O |
| C-14377 | H | single bond | Me | *c*-Pr(1-CF₃) | Me | Me | Me | O |
| C-14378 | Me | single bond | H | *c*-Pr(1-CF₃) | Me | Me | Me | O |
| C-14379 | Me(R) | single bond | H | *c*-Pr(1-CF₃) | Me | Me | Me | O |
| C-14380 | Me(S) | single bond | H | *c*-Pr(1-CF₃) | Me | Me | Me | O |
| C-14381 | H | single bond | Me | *c*-Pr(1-C≡H) | Me | Cl | Me | O |
| C-14382 | Me | single bond | H | *c*-Pr(1-C≡H) | Me | Cl | Me | O |
| C-14383 | Me(R) | single bond | H | *c*-pr(1-C≡CH) | Me | Cl | Me | O |
| C-14384 | Me(S) | single bond | H | *c*-pr(1-C≡CH) | Me | Cl | Me | O |
| C-14385 | H | single bond | Me | *c*-Pr(1-C≡H) | Me | Me | Me | O |
| C-14386 | Me | single bond | H | *c*-Pr(1-C≡H) | Me | Me | Me | O |
| C-14387 | Me(R) | single bond | H | *c*-Pr(1-C≡H) | Me | Me | Me | O |
| C-14388 | Me(S) | single bond | H | *c*-Pr(1-C≡H) | Me | Me | Me | O |
| C-14389 | H | single bond | Me | *c*-Pr[1-C(=O)OEt] | Me | Cl | Me | O |
| C-14390 | Me | single bond | H | *c*-Pr[1-C(=O)OEt] | Me | Cl | Me | O |
| C-14391 | Me(R) | single bond | H | *c*-Pr[1-C(=O)OEt] | Me | Cl | Me | O |
| C-14392 | Me(S) | single bond | H | *c*-Pr[1-C(=O)OEt] | Me | Cl | Me | O |
| C-14393 | H | single bond | Me | *c*-Pr[1-C(=O)OEt] | Me | Me | Me | O |
| C-14394 | Me | single bond | H | *c*-Pr[1-C(=O)OEt] | Me | Me | Me | O |
| C-14395 | Me(R) | single bond | H | *c*-Pr[1-C(=O)OEt] | Me | Me | Me | O |
| C-14396 | Me(S) | single bond | H | *c*-Pr[1-C(=O)OEt] | Me | Me | Me | O |
| C-14397 | H | single bond | Me | *c*-Pr[1-C(=O)OH] | Me | Cl | Me | O |
| C-14398 | Me | single bond | H | *c*-Pr[1-C(=O)OH] | Me | Cl | Me | O |
| C-14399 | Me(R) | single bond | H | *c*-Pr[1-C(=O)OH] | Me | Cl | Me | O |
| C-14400 | Me(S) | single bond | H | *c*-Pr[1-C(=O)OH] | Me | Cl | Me | O |
| C-14401 | H | single bond | Me | *c*-Pr[1-C(=O)OH] | Me | Me | Me | O |
| C-14402 | Me | single bond | H | *c*-Pr[1-C(=O)OH] | Me | Me | Me | O |
| C-14403 | Me(R) | single bond | H | *c*-Pr[1-C(=O)OH] | Me | Me | Me | O |
| C-14404 | Me(S) | single bond | H | *c*-Pr[1-C(=O)OH] | Me | Me | Me | O |
| C-14405 | H | single bond | Me | *c*-Pr[1-C(=O)NH₂] | Me | Cl | Me | O |
| C-14406 | Me | single bond | H | *c*-Pr[1-C(=O)NH₂] | Me | Cl | Me | O |

**[Table 556]**

| Compound Number | Y | W | Z | R¹ | R² | R³ | R¹⁰ | A |
|---|---|---|---|---|---|---|---|---|
| C-14407 | Me(R) | single bond | H | *c*-Pr[1-C(=O)NH₂] | Me | Cl | Me | O |
| C-14408 | Me(S) | single bond | H | *c*-Pr[1-C(=O)NH₂] | Me | Cl | Me | O |
| C-14409 | H | single bond | Me | *c*-Pr[1-C(=O)NH₂] | Me | Me | Me | O |
| C-14410 | Me | single bond | H | *c*-Pr[1-C(=O)NH₂ | Me | Me | Me | O |
| C-14411 | Me(R) | single bond | H | *c*-Pr[1-C(=O)NH₂ | Me | Me | Me | O |
| C-14412 | Me(S) | single bond | H | *c*-Pr[1-C(=O)NH₂] | Me | Me | Me | O |
| C-14413 | H | single bond | Me | *c*-Pr(1-OMe) | Me | Cl | Me | O |
| C-14414 | Me | single bond | H | *c*-Pr(1-OMe) | Me | Cl | Me | O |
| C-14415 | Me(R) | single bond | H | *c*-Pr(1-OMe) | Me | Cl | Me | O |
| C-14416 | Me(S) | single bond | H | *c*-Pr(1-OMe) | Me | Cl | Me | O |
| C-14417 | H | single bond | Me | *c*-Pr(1-OMe) | Me | Me | Me | O |
| C-14418 | Me | single bond | H | *c*-Pr(1-OMe) | Me | Me | Me | O |
| C-14419 | Me(R) | single bond | H | *c*-Pr(1-OMe) | Me | Me | Me | O |
| C-14420 | Me(S) | single bond | H | *c*-Pr(1-OMe) | Me | Me | Me | O |
| C-14421 | H | single bond | Me | *c*-Pr(1-SMe) | Me | Cl | Me | O |
| C-14422 | Me | single bond | H | *c*-Pr(1-SMe) | Me | Cl | Me | O |
| C-14423 | Me(R) | single bond | H | *c*-Pr(1-SMe) | Me | Cl | Me | O |
| C-14424 | Me(S) | single bond | H | *c*-Pr(1-SMe) | Me | Cl | Me | O |
| C-14425 | H | single bond | Me | *c*-Pr(1-SMe) | Me | Me | Me | O |
| C-14426 | Me | single bond | H | *c*-Pr(1-SMe) | Me | Me | Me | O |
| C-14427 | Me(R) | single bond | H | *c*-Pr(1-SMe) | Me | Me | Me | O |
| C-14428 | Me(S) | single bond | H | *c*-Pr(1-SMe) | Me | Me | Me | O |
| C-14429 | H | single bond | Me | *c*-Pr[1-S(=O)Me] | Me | Cl | Me | O |
| C-14430 | Me | single bond | H | *c*-Pr[1-S(=O)Me] | Me | Cl | Me | O |
| C-14431 | Me(R) | single bond | H | *c*-Pr[1-S(=O)Me] | Me | Cl | Me | O |
| C-14432 | Me(S) | single bond | H | *c*-Pr[1-S(=O)Me] | Me | Cl | Me | O |
| C-14433 | H | single bond | Me | *c*-Pr[1-S(=O)Me] | Me | Me | Me | O |
| C-14434 | Me | single bond | H | *c*-Pr[1-S(=O)Me] | Me | Me | Me | O |
| C-14435 | Me(R) | single bond | H | *c*-Pr[1-S(=O)Me] | Me | Me | Me | O |
| C-14436 | Me(S) | single bond | H | *c*-Pr[1-S(=O)Me] | Me | Me | Me | O |
| C-14437 | H | single bond | Me | *c*-Pr[1-S(=O)₂Me] | Me | Cl | Me | O |
| C-14438 | Me | single bond | H | *c*-Pr[1-S(=O)₂Me] | Me | Cl | Me | O |
| C-14439 | Me(R) | single bond | H | *c*-Pr[1-S(=O)₂Me] | Me | Cl | Me | O |
| C-14440 | Me(S) | single bond | H | *c*-Pr[1-S(=O)₂Me] | Me | Cl | Me | O |
| C-14441 | H | single bond | Me | *c*-Pr[1-S(=O)₂Me] | Me | Me | Me | O |
| C-14442 | Me | single bond | H | *c*-Pr[1-S(=O)₂Me] | Me | Me | Me | O |
| C-14443 | Me(R) | single bond | H | *c*-Pr[1-S(=O)₂Me] | Me | Me | Me | O |
| C-14444 | Me(S) | single bond | H | *c*-Pr[1-S(=O)₂Me] | Me | Me | Me | O |
| C-14445 | H | single bond | Me | *c*-Pr[1-S(=O)₂CHF₂ | Me | Cl | Me | O |
| C-14446 | Me | single bond | H | *c*-Pr[1-S(=O)₂CHF₂ | Me | Cl | Me | O |
| C-14447 | Me(R) | single bond | H | *c*-Pr[1-S(=O)₂CHF₂ | Me | Cl | Me | O |
| C-14448 | Me(S) | single bond | H | *c*-Pr[1-S(=O)₂CHF₂ | Me | Cl | Me | O |
| C-14449 | H | single bond | Me | *c*-Pr[1-S(=O)₂CHF₂ | Me | Me | Me | O |
| C-14450 | Me | single bond | H | *c*-Pr[1-S(=O)₂CHF₂ | Me | Me | Me | O |

**[Table 557]**

| Compound Number | Y | W | Z | R¹ | R² | R³ | R¹⁰ | A |
|---|---|---|---|---|---|---|---|---|
| C-14451 | Me(R) | single bond | H | *c*-Pr[1-S(=O)₂CHF₂ | Me | Me | Me | O |
| C-14452 | Me(S) | single bond | H | *c*-Pr[1-S(=O)₂CHF₂ | Me | Me | Me | O |
| C-14453 | H | single bond | Me | *c*-Pr[1-S(=O)₂CF₃ | Me | Cl | Me | O |
| C-14454 | Me | single bond | H | *c*-Pr[1-S(=O)₂CF₃ | Me | Cl | Me | O |
| C-14455 | Me(R) | single bond | H | *c*-Pr[1-S(=O)₂CF₃] | Me | Cl | Me | O |
| C-14456 | Me(S) | single bond | H | *c*-Pr[1-S(=O)₂CF₃] | Me | Cl | Me | O |
| C-14457 | H | single bond | Me | *c*-Pr[1-S(=O)₂CF₃ | Me | Me | Me | O |
| C-14458 | Me | single bond | H | *c*-Pr[1-S(=O)₂CF₃] | Me | Me | Me | O |
| C-14459 | Me(R) | single bond | H | *c*-Pr[1-S(=O)₂CF₃] | Me | Me | Me | O |
| C-14460 | Me(S) | single bond | H | *c*-Pr[1-S(=O)₂CF₃ | Me | Me | Me | O |
| C-14461 | H | single bond | Me | *c*-Pr[1-(5,6-dihydro-1,4,2-dioxazin-3-yl)] | Me | Cl | Me | O |
| C-14462 | Me | single bond | H | *c*-Pr[1-(5,6-dihydro-1,4,2-dioxazin-3-yl)] | Me | Cl | Me | O |
| C-14463 | Me(R) | single bond | H | *c*-Pr[1-(5,6-dihydro-1,4,2-dioxazin-3-yl)] | Me | Cl | Me | O |
| C-14464 | Me(S) | single bond | H | *c*-Pr[1-(5,6-dihydro-1,4,2-dioxazin-3-yl)] | Me | Cl | Me | O |
| C-14465 | H | single bond | Me | *c*-Pr[1-(5,6-dihydro-1,4,2-dioxazin-3-yl)] | Me | Me | Me | O |
| C-14466 | Me | single bond | H | *c*-Pr[1-(5,6-dihydro-1,4,2-dioxazin-3-yl)] | Me | Me | Me | O |
| C-14467 | Me(R) | single bond | H | *c*-Pr[1-(5,6-dihydro-1,4,2-dioxazin-3-yl)] | Me | Me | Me | O |
| C-14468 | Me(S) | single bond | H | *c*-Pr[1-(5,6-dihydro-1,4,2-dioxazin-3-yl)] | Me | Me | Me | O |
| C-14469 | H | single bond | Me | *c*-Pr[1-(5-hydroxy-1,3,4-oxadiazol-2-yl)] | Me | Cl | Me | O |
| C-14470 | Me | single bond | H | *c*-Pr[1-(5-hydroxy-1,3,4-oxadiazol-2-yl)] | Me | Cl | Me | O |
| C-14471 | Me(R) | single bond | H | *c*-Pr[1-(5-hydroxy-1,3,4-oxadiazol-2-yl)] | Me | Cl | Me | O |
| C-14472 | Me(S) | single bond | H | *c*-Pr[1-(5-hydroxy-1,3,4-oxadiazol-2-yl)] | Me | Cl | Me | O |
| C-14473 | H | single bond | Me | *c*-Pr[1-(5-hydroxy-1,3,4-oxadiazol-2-yl)] | Me | Me | Me | O |
| C-14474 | Me | single bond | H | *c*-Pr[1-(5-hydroxy-1,3,4-oxadiazol-2-yl)] | Me | Me | Me | O |
| C-14475 | Me(R) | single bond | H | *c*-Pr[1-(5-hydroxy-1,3,4-oxadiazol-2-yl)] | Me | Me | Me | O |
| C-14476 | Me(S) | single bond | H | *c*-Pr[1-(5-hydroxy-1,3,4-oxadiazol-2-yl)] | Me | Me | Me | O |
| C-14477 | H | single bond | Me | *c*-Pr[1-(pyrimidin-2-yl)] | Me | Cl | Me | O |
| C-14478 | Me | single bond | H | *c*-Pr[1-(pyrimidin-2-yl)] | Me | Cl | Me | O |
| C-14479 | Me(R) | single bond | H | *c*-Pr[1-(pyrimidin-2-yl)] | Me | Cl | Me | O |
| C-14480 | Me(S) | single bond | H | *c*-Pr[1-(pyrimidin-2-yl)] | Me | Cl | Me | O |
| C-14481 | H | single bond | Me | *c*-Pr[1-(pyrimidin-2-yl)] | Me | Me | Me | O |
| C-14482 | Me | single bond | H | *c*-Pr[1-(pyrimidin-2-yl)] | Me | Me | Me | O |
| C-14483 | Me(R) | single bond | H | *c*-Pr[1-(pyrimidin-2-yl)] | Me | Me | Me | O |
| C-14484 | Me(S) | single bond | H | *c*-Pr[1-(pyrimidin-2-yl)] | Me | Me | Me | O |
| C-14485 | H | single bond | Me | *t*-Bu | Me | Cl | Me | O |
| C-14486 | Me | single bond | H | *t*-Bu | Me | Cl | Me | O |
| C-14487 | Me(R) | single bond | H | *t*-Bu | Me | Cl | Me | O |
| C-14488 | Me(S) | single bond | H | *t*-Bu | Me | Cl | Me | O |
| C-14489 | H | single bond | Me | *t*-Bu | Me | Me | Me | O |
| C-14490 | Me | single bond | H | *t*-Bu | Me | Me | Me | O |
| C-14491 | Me(R) | single bond | H | *t*-Bu | Me | Me | Me | O |
| C-14492 | Me(S) | single bond | H | *t*-Bu | Me | Me | Me | O |
| C-14493 | H | single bond | Me | *c*-Bu | Me | Cl | Me | O |
| C-14494 | Me | single bond | H | *c*-Bu | Me | Cl | Me | O |

**[Table 558]**

| Compound Number | Y | W | Z | R¹ | R² | R³ | R¹⁰ | A |
|---|---|---|---|---|---|---|---|---|
| C-14495 | Me(R) | single bond | H | *c*-Bu | Me | Cl | Me | O |
| C-14496 | Me(S) | single bond | H | *c*-Bu | Me | Cl | Me | O |
| C-14497 | H | single bond | Me | *c*-Bu | Me | Me | Me | O |
| C-14498 | Me | single bond | H | *c*-Bu | Me | Me | Me | O |
| C-14499 | Me(R) | single bond | H | *c*-Bu | Me | Me | Me | O |
| C-14500 | Me(S) | single bond | H | *c*-Bu | Me | Me | Me | O |
| C-14501 | H | single bond | Me | *c*-Bu(1-CN) | Me | Cl | Me | O |
| C-14502 | Me | single bond | H | *c*-Bu(1-CN) | Me | Cl | Me | O |
| C-14503 | Me(R) | single bond | H | *c*-Bu(1-CN) | Me | Cl | Me | O |
| C-14504 | Me(S) | single bond | H | *c*-Bu(1-CN) | Me | Cl | Me | O |
| C-14505 | H | single bond | Me | *c*-Bu(1-CN) | Me | Me | Me | O |
| C-14506 | Me | single bond | H | *c*-Bu(1-CN) | Me | Me | Me | O |
| C-14507 | Me(R) | single bond | H | *c*-Bu(1-CN) | Me | Me | Me | O |
| C-14508 | Me(S) | single bond | H | *c*-Bu(1-CN) | Me | Me | Me | O |
| C-14509 | H | single bond | Me | *c*-Bu(3,3-F₂) | Me | Cl | Me | O |
| C-14510 | Me | single bond | H | *c*-Bu(3,3-F₂) | Me | Cl | Me | O |
| C-14511 | Me(R) | single bond | H | *c*-Bu(3,3-F₂) | Me | Cl | Me | O |
| C-14512 | Me(S) | single bond | H | *c*-Bu(3,3-F₂) | Me | Cl | Me | O |
| C-14513 | H | single bond | Me | *c*-Bu(3,3-F₂) | Me | Me | Me | O |
| C-14514 | Me | single bond | H | *c*-Bu(3,3-F₂) | Me | Me | Me | O |
| C-14515 | Me(R) | single bond | H | *c*-Bu(3,3-F₂) | Me | Me | Me | O |
| C-14516 | Me(S) | single bond | H | *c*-Bu(3,3-F₂) | Me | Me | Me | O |
| C-14517 | H | single bond | Me | oxetan-3-yl | Me | Cl | Me | O |
| C-14518 | Me | single bond | H | oxetan-3-yl | Me | Cl | Me | O |
| C-14519 | Me(R) | single bond | H | oxetan-3-yl | Me | Cl | Me | O |
| C-14520 | Me(S) | single bond | H | oxetan-3-yl | Me | Cl | Me | O |
| C-14521 | H | single bond | Me | oxetan-3-yl | Me | Me | Me | O |
| C-14522 | Me | single bond | H | oxetan-3-yl | Me | Me | Me | O |
| C-14523 | Me(R) | single bond | H | oxetan-3-yl | Me | Me | Me | O |
| C-14524 | Me(S) | single bond | H | oxetan-3-yl | Me | Me | Me | O |
| C-14525 | H | single bond | Me | oxetan(3-CN)-3-yl | Me | Cl | Me | O |
| C-14526 | Me | single bond | H | oxetan(3-CN)-3-yl | Me | Cl | Me | O |
| C-14527 | Me(R) | single bond | H | oxetan(3-CN)-3-yl | Me | Cl | Me | O |
| C-14528 | Me(S) | single bond | H | oxetan(3-CN)-3-yl | Me | Cl | Me | O |
| C-14529 | H | single bond | Me | oxetan(3-CN)-3-yl | Me | Me | Me | O |
| C-14530 | Me | single bond | H | oxetan(3-CN)-3-yl | Me | Me | Me | O |
| C-14531 | Me(R) | single bond | H | oxetan(3-CN)-3-yl | Me | Me | Me | O |
| C-14532 | Me(S) | single bond | H | oxetan(3-CN)-3-yl | Me | Me | Me | O |
| C-14533 | H | single bond | Me | azetidin[1-C(=O)Me]-3-yl | Me | Cl | Me | O |
| C-14534 | Me | single bond | H | azetidin[1-C(=O)Me]-3-yl | Me | Cl | Me | O |
| C-14535 | Me(R) | single bond | H | azetidin[1-C(=O)Me]-3-yl | Me | Cl | Me | O |
| C-14536 | Me(S) | single bond | H | azetidin[1-C(=O)Me]-3-yl | Me | Cl | Me | O |
| C-14537 | H | single bond | Me | azetidin[1-C(=O)Me]-3-yl | Me | Me | Me | O |
| C-14538 | Me | single bond | H | azetidin[1-C(=O)Me]-3-yl | Me | Me | Me | O |

**[Table 559]**

| Compound Number | Y | W | Z | R¹ | R² | R³ | R¹⁰ | A |
|---|---|---|---|---|---|---|---|---|
| C-14539 | Me(R) | single bond | H | azetidin[1-C(=O)Me]-3-yl | Me | Me | Me | O |
| C-14540 | Me(S) | single bond | H | azetidin[1-C(=O)Me]-3-yl | Me | Me | Me | O |
| C-14541 | H | single bond | Me | azetidin[1-S(=O)₂Me]-3-yl | Me | Cl | Me | O |
| C-14542 | Me | single bond | H | azetidin[1-S(=O)₂Me]-3-yl | Me | Cl | Me | O |
| C-14543 | Me(R) | single bond | H | azetidin[1-S(=O)₂Me]-3-yl | Me | Cl | Me | O |
| C-14544 | Me(S) | single bond | H | azetidin[1-S(=O)₂Me]-3-yl | Me | Cl | Me | O |
| C-14545 | H | single bond | Me | azetidin[1-S(=O)₂Me]-3-yl | Me | Me | Me | O |
| C-14546 | Me | single bond | H | azetidin[1-S(=O)₂Me]-3-yl | Me | Me | Me | O |
| C-14547 | Me(R) | single bond | H | azetidin[1-S(=O)₂Me]-3-yl | Me | Me | Me | O |
| C-14548 | Me(S) | single bond | H | azetidin[1-S(=O)₂Me]-3-yl | Me | Me | Me | O |
| C-14549 | H | single bond | Me | azetidin[1-S(=O)₂CHF₂]-3-yl | Me | Cl | Me | O |
| C-14550 | Me | single bond | H | azetidin[1-S(=O)₂CHF₂]-3-yl | Me | Cl | Me | O |
| C-14551 | Me(R) | single bond | H | azetidin[1-S(=O)₂CHF₂]-3-yl | Me | Cl | Me | O |
| C-14552 | Me(S) | single bond | H | azetidin[1-S(=O)₂CHF₂]-3-yl | Me | Cl | Me | O |
| C-14553 | H | single bond | Me | azetidin[1-S(=O)₂CHF₂]-3-yl | Me | Me | Me | O |
| C-14554 | Me | single bond | H | azetidin[1-S(=O)₂CHF₂]-3-yl | Me | Me | Me | O |
| C-14555 | Me(R) | single bond | H | azetidin[1-S(=O)₂CHF₂]-3-yl | Me | Me | Me | O |
| C-14556 | Me(S) | single bond | H | azetidin[1-S(=O)₂CHF₂]-3-yl | Me | Me | Me | O |
| C-14557 | H | single bond | Me | *c*-Pen | Me | Cl | Me | O |
| C-14558 | Me | single bond | H | *c*-Pen | Me | Cl | Me | O |
| C-14559 | Me(R) | single bond | H | *c*-Pen | Me | Cl | Me | O |
| C-14560 | Me(S) | single bond | H | *c*-Pen | Me | Cl | Me | O |
| C-14561 | H | single bond | Me | *c*-Pen | Me | Me | Me | O |
| C-14562 | Me | single bond | H | *c*-Pen | Me | Me | Me | O |
| C-14563 | Me(R) | single bond | H | *c*-Pen | Me | Me | Me | O |
| C-14564 | Me(S) | single bond | H | *c*-Pen | Me | Me | Me | O |
| C-14565 | H | single bond | Me | tetrahydrofuran-3-yl | Me | Cl | Me | O |
| C-14566 | Me | single bond | H | tetrahydrofuran-3-yl | Me | Cl | Me | O |
| C-14567 | Me(R) | single bond | H | tetrahydrofuran-3-yl | Me | Cl | Me | O |
| C-14568 | Me(S) | single bond | H | tetrahydrofuran-3-yl | Me | Cl | Me | O |
| C-14569 | H | single bond | Me | tetra hydrofuran-3-yl | Me | Me | Me | O |
| C-14570 | Me | single bond | H | tetrahydrofuran-3-yl | Me | Me | Me | O |
| C-14571 | Me(R) | single bond | H | tetra hydrofuran-3-yl | Me | Me | Me | O |
| C-14572 | Me(S) | single bond | H | tetrahydrofuran-3-yl | Me | Me | Me | O |
| C-14573 | H | single bond | Me | tetrahydrothiophen-3-yl | Me | Cl | Me | O |
| C-14574 | Me | single bond | H | tetrahydrothiophen-3-yl | Me | Cl | Me | O |
| C-14575 | Me(R) | single bond | H | tetrahydrothiophen-3-yl | Me | Cl | Me | O |
| C-14576 | Me(S) | single bond | H | tetrahydrothiophen-3-yl | Me | Cl | Me | O |
| C-14577 | H | single bond | Me | tetrahydrothiophen-3-yl | Me | Me | Me | O |
| C-14578 | Me | single bond | H | tetrahydrothiophen-3-yl | Me | Me | Me | O |
| C-14579 | Me(R) | single bond | H | tetrahydrothiophen-3-yl | Me | Me | Me | O |
| C-14580 | Me(S) | single bond | H | tetrahydrothiophen-3-yl | Me | Me | Me | O |
| C-14581 | H | single bond | Me | *c*-Pen(1-CN) | Me | Cl | Me | O |
| C-14582 | Me | single bond | H | *c*-Pen(1-CN) | Me | Cl | Me | O |

**[Table 560]**

| Compound Number | Y | W | Z | R¹ | R² | R³ | R¹⁰ | A |
|---|---|---|---|---|---|---|---|---|
| C-14583 | Me(R) | single bond | H | c-Pen(1-CN) | Me | Cl | Me | O |
| C-14584 | Me(S) | single bond | H | c-Pen(1-CN) | Me | Cl | Me | O |
| C-14585 | H | single bond | Me | c-Pen(1-CN) | Me | Me | Me | O |
| C-14586 | Me | single bond | H | c-Pen(1-CN) | Me | Me | Me | O |
| C-14587 | Me(R) | single bond | H | c-Pen(1-CN) | Me | Me | Me | O |
| C-14588 | Me(S) | single bond | H | c-Pen(1-CN) | Me | Me | Me | O |
| C-14589 | H | single bond | Me | tetrahydrofuran(3-CN)-3-yl | Me | Cl | Me | O |
| C-14590 | Me | single bond | H | tetrahydrofuran(3-CN)-3-yl | Me | Cl | Me | O |
| C-14591 | Me(R) | single bond | H | tetrahydrofuran(3-CN)-3-yl | Me | Cl | Me | O |
| C-14592 | Me(S) | single bond | H | tetrahydrofuran(3-CN)-3-yl | Me | Cl | Me | O |
| C-14593 | H | single bond | Me | tetrahydrofuran(3-CN)-3-yl | Me | Me | Me | O |
| C-14594 | Me | single bond | H | tetrahydrofuran(3-CN)-3-yl | Me | Me | Me | O |
| C-14595 | Me(R) | single bond | H | tetrahydrofuran(3-CN)-3-yl | Me | Me | Me | O |
| C-14596 | Me(S) | single bond | H | tetrahydrofuran(3-CN)-3-yl | Me | Me | Me | O |
| C-14597 | H | single bond | Me | tetrahydrothiophen(3-CN)-3-yl | Me | Cl | Me | O |
| C-14598 | Me | single bond | H | tetrahydrothiophen(3-CN)-3-yl | Me | Cl | Me | O |
| C-14599 | Me(R) | single bond | H | tetrahydrothiophen(3-CN)-3-yl | Me | Cl | Me | O |
| C-14600 | Me(S) | single bond | H | tetrahydrothiophen(3-CN)-3-yl | Me | Cl | Me | O |
| C-14601 | H | single bond | Me | tetrahydrothiophen(3-CN)-3-yl | Me | Me | Me | O |
| C-14602 | Me | single bond | H | tetrahydrothiophen(3-CN)-3-yl | Me | Me | Me | O |
| C-14603 | Me(R) | single bond | H | tetrahydrothiophen(3-CN)-3-yl | Me | Me | Me | O |
| C-14604 | Me(S) | single bond | H | tetrahydrothiophen(3-CN)-3-yl | Me | Me | Me | O |
| C-14605 | H | single bond | Me | bicyclo[1.1.1]pentan-1-yl | Me | Cl | Me | O |
| C-14606 | Me | single bond | H | bicyclo[1.1.1]pentan-1-yl | Me | Cl | Me | O |
| C-14607 | Me(R) | single bond | H | bicyclo[1.1.1]pentan-1-yl | Me | Cl | Me | O |
| C-14608 | Me(S) | single bond | H | bicyclo[1.1.1]pentan-1-yl | Me | Cl | Me | O |
| C-14609 | H | single bond | Me | bicyclo[1.1.1]pentan-1-yl | Me | Me | Me | O |
| C-14610 | Me | single bond | H | bicyclo[1.1.1]pentan-1-yl | Me | Me | Me | O |
| C-14611 | Me(R) | single bond | H | bicyclo[1.1.1]pentan-1-yl | Me | Me | Me | O |
| C-14612 | Me(S) | single bond | H | bicyclo[1.1.1]pentan-1-yl | Me | Me | Me | O |
| C-14613 | H | single bond | Me | *c*-Hex | Me | Cl | Me | O |
| C-14614 | Me | single bond | H | *c*-Hex | Me | Cl | Me | O |
| C-14615 | Me(R) | single bond | H | *c*-Hex | Me | Cl | Me | O |
| C-14616 | Me(S) | single bond | H | *c*-Hex | Me | Cl | Me | O |
| C-14617 | H | single bond | Me | *c*-Hex | Me | Me | Me | O |
| C-14618 | Me | single bond | H | *c*-Hex | Me | Me | Me | O |
| C-14619 | Me(R) | single bond | H | *c*-Hex | Me | Me | Me | O |
| C-14620 | Me(S) | single bond | H | c-Hex | Me | Me | Me | O |
| C-14621 | H | single bond | Me | tetrahydropyran-4-yl | Me | Cl | Me | O |
| C-14622 | Me | single bond | H | tetrahydropyran-4-yl | Me | Cl | Me | O |
| C-14623 | Me(R) | single bond | H | tetrahydropyran-4-yl | Me | Cl | Me | O |
| C-14624 | Me(S) | single bond | H | tetrahydropyran-4-yl | Me | Cl | Me | O |
| C-14625 | H | single bond | Me | tetrahydropyran-4-yl | Me | Me | Me | O |
| C-14626 | Me | single bond | H | tetrahydropyran-4-yl | Me | Me | Me | O |

**[Table 561]**

| Compound Number | Y | W | Z | R¹ | R² | R³ | R¹⁰ | A |
|---|---|---|---|---|---|---|---|---|
| C-14627 | Me(R) | single bond | H | tetrahydropyran-4-yl | Me | Me | Me | O |
| C-14628 | Me(S) | single bond | H | tetrahydropyran-4-yl | Me | Me | Me | O |
| C-14629 | H | single bond | Me | tetrahydrothiopyran-4-yl | Me | Cl | Me | O |
| C-14630 | Me | single bond | H | tetrahydrothiopyran-4-yl | Me | Cl | Me | O |
| C-14631 | Me(R) | single bond | H | tetrahydrothiopyran-4-yl | Me | Cl | Me | O |
| C-14632 | Me(S) | single bond | H | tetrahydrothiopyran-4-yl | Me | Cl | Me | O |
| C-14633 | H | single bond | Me | tetrahydrothiopyran-4-yl | Me | Me | Me | O |
| C-14634 | Me | single bond | H | tetrahydrothiopyran-4-yl | Me | Me | Me | O |
| C-14635 | Me(R) | single bond | H | tetrahydrothiopyran-4-yl | Me | Me | Me | O |
| C-14636 | Me(S) | single bond | H | tetrahydrothiopyran-4-yl | Me | Me | Me | O |
| C-14637 | H | single bond | Me | c-Hex(1-CN) | Me | Cl | Me | O |
| C-14638 | Me | single bond | H | *c*-Hex(1-CN) | Me | Cl | Me | O |
| C-14639 | Me(R) | single bond | H | *c*-Hex(1-CN) | Me | Cl | Me | O |
| C-14640 | Me(S) | single bond | H | *c*-Hex(1-CN) | Me | Cl | Me | O |
| C-14641 | H | single bond | Me | *c*-Hex(1-CN) | Me | Me | Me | O |
| C-14642 | Me | single bond | H | *c*-Hex(1-CN) | Me | Me | Me | O |
| C-14643 | Me(R) | single bond | H | *c*-Hex(1-CN) | Me | Me | Me | O |
| C-14644 | Me(S) | single bond | H | *c*-Hex(1-CN) | Me | Me | Me | O |
| C-14645 | H | single bond | Me | tetrahydropyran(4-CN)-4-yl | Me | Cl | Me | O |
| C-14646 | Me | single bond | H | tetrahydropyran(4-CN)-4-yl | Me | Cl | Me | O |
| C-14647 | Me(R) | single bond | H | tetrahydropyran(4-CN)-4-yl | Me | Cl | Me | O |
| C-14648 | Me(S) | single bond | H | tetrahydropyran(4-CN)-4-yl | Me | Cl | Me | O |
| C-14649 | H | single bond | Me | tetrahydropyran(4-CN)-4-yl | Me | Me | Me | O |
| C-14650 | Me | single bond | H | tetrahydropyran(4-CN)-4-yl | Me | Me | Me | O |
| C-14651 | Me(R) | single bond | H | tetrahydropyran(4-CN)-4-yl | Me | Me | Me | O |
| C-14652 | Me(S) | single bond | H | tetrahydropyran(4-CN)-4-yl | Me | Me | Me | O |
| C-14653 | H | single bond | Me | tetrahydrothiopyran(4-CN)-4-yl | Me | Cl | Me | O |
| C-14654 | Me | single bond | H | tetrahydrothiopyran(4-CN)-4-yl | Me | Cl | Me | O |
| C-14655 | Me(R) | single bond | H | tetrahydrothiopyran(4-CN)-4-yl | Me | Cl | Me | O |
| C-14656 | Me(S) | single bond | H | tetrahydrothiopyran(4-CN)-4-yl | Me | Cl | Me | O |
| C-14657 | H | single bond | Me | tetrahydrothiopyran(4-CN)-4-yl | Me | Me | Me | O |
| C-14658 | Me | single bond | H | tetrahydrothiopyran(4-CN)-4-yl | Me | Me | Me | O |
| C-14659 | Me(R) | single bond | H | tetrahydrothiopyran(4-CN)-4-yl | Me | Me | Me | O |
| C-14660 | Me(S) | single bond | H | tetrahydrothiopyran(4-CN)-4-yl | Me | Me | Me | O |
| C-14661 | H | single bond | Me | CH₂(*c*-Pr) | Me | Cl | Me | O |
| C-14662 | Me | single bond | H | CH₂(*c*-Pr) | Me | Cl | Me | O |
| C-14663 | Me(R) | single bond | H | CH₂(*c*-Pr) | Me | Cl | Me | O |
| C-14664 | Me(S) | single bond | H | CH₂(*c*-Pr) | Me | Cl | Me | O |
| C-14665 | H | single bond | Me | CH₂(*c*-Pr) | Me | Me | Me | O |
| C-14666 | Me | single bond | H | CH₂(*c*-Pr) | Me | Me | Me | O |
| C-14667 | Me(R) | single bond | H | CH₂(*c*-Pr) | Me | Me | Me | O |
| C-14668 | Me(S) | single bond | H | CH₂(*c*-Pr) | Me | Me | Me | O |
| C-14669 | H | single bond | Me | CH₂[*c*-Pr(1-CN)] | Me | Cl | Me | O |
| C-14670 | Me | single bond | H | CH₂[*c*-Pr(1-CN)] | Me | Cl | Me | O |

**[Table 562]**

| Compound Number | Y | W | Z | R¹ | R² | R³ | R¹⁰ | A |
|---|---|---|---|---|---|---|---|---|
| C-14671 | Me(R) | single bond | H | CH₃[*c*-Pr(1-CN)] | Me | Cl | Me | O |
| C-14672 | Me(S) | single bond | H | CH₃[*c*-Pr(1-CN)] | Me | Cl | Me | O |
| C-14673 | H | single bond | Me | CH₂[*c*-Pr(1-CN)] | Me | Me | Me | O |
| C-14674 | Me | single bond | H | CH₃[*c*-Pr(1-CN)] | Me | Me | Me | O |
| C-14675 | Me(R) | single bond | H | CH₃[*c*-Pr(1-CN)] | Me | Me | Me | O |
| C-14676 | Me(S) | single bond | H | CH₂[*c*-Pr(1-CN)] | Me | Me | Me | O |
| C-14677 | H | single bond | Me | CH(Me)(*c*-Pr) | Me | Cl | Me | O |
| C-14678 | Me | single bond | H | CH(Me)(*c*-Pr) | Me | Cl | Me | O |
| C-14679 | Me(R) | single bond | H | CH(Me)(*c*-Pr) | Me | Cl | Me | O |
| C-14680 | Me(S) | single bond | H | CH(Me)(*c*-Pr) | Me | Cl | Me | O |
| C-14681 | H | single bond | Me | CH(Me)(*c*-Pr) | Me | Me | Me | O |
| C-14682 | Me | single bond | H | CH(Me)(*c*-Pr) | Me | Me | Me | O |
| C-14683 | Me(R) | single bond | H | CH(Me)(*c*-Pr) | Me | Me | Me | O |
| C-14684 | Me(S) | single bond | H | CH(Me)(*c*-Pr) | Me | Me | Me | O |
| C-14685 | H | single bond | Me | CH(Me)CH₂OMe | Me | Cl | Me | O |
| C-14686 | Me | single bond | H | CH(Me)CH₂OMe | Me | Cl | Me | O |
| C-14687 | Me(R) | single bond | H | CH(Me)CH₂OMe | Me | Cl | Me | O |
| C-14688 | Me(S) | single bond | H | CH(Me)CH₂OMe | Me | Cl | Me | O |
| C-14689 | H | single bond | Me | CH(Me)CH₂OMe | Me | Me | Me | O |
| C-14690 | Me | single bond | H | CH(Me)CH₂OMe | Me | Me | Me | O |
| C-14691 | Me(R) | single bond | H | CH(Me)CH₂OMe | Me | Me | Me | O |
| C-14692 | Me(S) | single bond | H | CH(Me)CH₂OMe | Me | Me | Me | O |
| C-14693 | H | single bond | Me | CH₂CH=CH₂ | Me | Cl | Me | O |
| C-14694 | Me | single bond | H | CH₂CH=CH₂ | Me | Cl | Me | O |
| C-14695 | Me(R) | single bond | H | CH₂CH=CH₂ | Me | Cl | Me | O |
| C-14696 | Me(S) | single bond | H | CH₂CH=CH₂ | Me | Cl | Me | O |
| C-14697 | H | single bond | Me | CH₂CH=CH₂ | Me | Me | Me | O |
| C-14698 | Me | single bond | H | CH₂CH=CH₂ | Me | Me | Me | O |
| C-14699 | Me(R) | single bond | H | CH₂CH=CH₂ | Me | Me | Me | O |
| C-14700 | Me(S) | single bond | H | CH₂CH=CH₂ | Me | Me | Me | O |
| C-14701 | H | single bond | Me | CH₂C≡CH | Me | Cl | Me | O |
| C-14702 | Me | single bond | H | CH₂C≡CH | Me | Cl | Me | O |
| C-14703 | Me(R) | single bond | H | CH₂C≡CH | Me | Cl | Me | O |
| C-14704 | Me(S) | single bond | H | CH₂C≡CH | Me | Cl | Me | O |
| C-14705 | H | single bond | Me | CH₂C≡CH | Me | Me | Me | O |
| C-14706 | Me | single bond | H | CH₂C≡CH | Me | Me | Me | O |
| C-14707 | Me(R) | single bond | H | CH₂C≡CH | Me | Me | Me | O |
| C-14708 | Me(S) | single bond | H | CH₂C≡CH | Me | Me | Me | O |
| C-14709 | H | single bond | Me | CH₂CN | Me | Cl | Me | O |
| C-14710 | Me | single bond | H | CH₂CN | Me | Cl | Me | O |
| C-14711 | Me(R) | single bond | H | CH₂CN | Me | Cl | Me | O |
| C-14712 | Me(S) | single bond | H | CH₂CN | Me | Cl | Me | O |
| C-14713 | H | single bond | Me | CH₂CN | Me | Me | Me | O |
| C-14714 | Me | single bond | H | CH₂CN | Me | Me | Me | O |

**[Table 563]**

| Compound Number | Y | W | Z | R¹ | R² | R³ | R¹⁰ | A |
|---|---|---|---|---|---|---|---|---|
| C-14715 | Me(R) | single bond | H | CH₂CN | Me | Me | Me | O |
| C-14716 | Me(S) | single bond | H | CH₂CN | Me | Me | Me | O |
| C-14717 | H | single bond | Me | CH(Me)CN | Me | Cl | Me | O |
| C-14718 | Me | single bond | H | CH(Me)CN | Me | Cl | Me | O |
| C-14719 | Me(R) | single bond | H | CH(Me)CN | Me | Cl | Me | O |
| C-14720 | Me(S) | single bond | H | CH(Me)CN | Me | Cl | Me | O |
| C-14721 | H | single bond | Me | CH(Me)CN | Me | Me | Me | O |
| C-14722 | Me | single bond | H | CH(Me)CN | Me | Me | Me | O |
| C-14723 | Me(R) | single bond | H | CH(Me)CN | Me | Me | Me | O |
| C-14724 | Me(S) | single bond | H | CH(Me)CN | Me | Me | Me | O |
| C-14725 | H | single bond | Me | C(Me)₂CN | Me | Cl | Me | O |
| C-14726 | Me | single bond | H | C(Me)₂CN | Me | Cl | Me | O |
| C-14727 | Me(R) | single bond | H | C(Me)₂CN | Me | Cl | Me | O |
| C-14728 | Me(S) | single bond | H | C(Me)₂CN | Me | Cl | Me | O |
| C-14729 | H | single bond | Me | C(Me)₂CN | Me | Me | Me | O |
| C-14730 | Me | single bond | H | C(Me)₂CN | Me | Me | Me | O |
| C-14731 | Me(R) | single bond | H | C(Me)₂CN | Me | Me | Me | O |
| C-14732 | Me(S) | single bond | H | C(Me)₂CN | Me | Me | Me | O |
| C-14733 | H | single bond | Me | C(Me)(*i*-Pr)CN | Me | Cl | Me | O |
| C-14734 | Me | single bond | H | C(Me)(*i*-Pr)CN | Me | Cl | Me | O |
| C-14735 | Me(R) | single bond | H | C(Me)(*i*-Pr)CN | Me | Cl | Me | O |
| C-14736 | Me(S) | single bond | H | C(Me)(*i*-Pr)CN | Me | Cl | Me | O |
| C-14737 | H | single bond | Me | C(Me)(*i*-Pr)CN | Me | Me | Me | O |
| C-14738 | Me | single bond | H | C(Me)(*i*-Pr)CN | Me | Me | Me | O |
| C-14739 | Me(R) | single bond | H | C(Me)(*i*-Pr)CN | Me | Me | Me | O |
| C-14740 | Me(S) | single bond | H | C(Me)(*i*-Pr)CN | Me | Me | Me | O |
| C-14741 | H | single bond | Me | OMe | Me | Cl | Me | O |
| C-14742 | Me | single bond | H | OMe | Me | Cl | Me | O |
| C-14743 | Me(R) | single bond | H | OMe | Me | Cl | Me | O |
| C-14744 | Me(S) | single bond | H | OMe | Me | Cl | Me | O |
| C-14745 | H | single bond | Me | OMe | Me | Me | Me | O |
| C-14746 | Me | single bond | H | OMe | Me | Me | Me | O |
| C-14747 | Me(R) | single bond | H | OMe | Me | Me | Me | O |
| C-14748 | Me(S) | single bond | H | OMe | Me | Me | Me | O |
| C-14749 | H | single bond | Me | CH=NOMe | Me | Cl | Me | O |
| C-14750 | Me | single bond | H | CH=NOMe | Me | Cl | Me | O |
| C-14751 | Me(R) | single bond | H | CH=NOMe | Me | Cl | Me | O |
| C-14752 | Me(S) | single bond | H | CH=NOMe | Me | Cl | Me | O |
| C-14753 | H | single bond | Me | CH=NOMe | Me | Me | Me | O |
| C-14754 | Me | single bond | H | CH=NOMe | Me | Me | Me | O |
| C-14755 | Me(R) | single bond | H | CH=NOMe | Me | Me | Me | O |
| C-14756 | Me(S) | single bond | H | CH=NOMe | Me | Me | Me | O |
| C-14757 | H | single bond | Me | morpholin-4-yl | Me | Cl | Me | O |
| C-14758 | Me | single bond | H | morpholin-4-yl | Me | Cl | Me | O |

**[Table 564]**

| Compound Number | Y | W | Z | R¹ | R² | R³ | R¹⁰ | A |
|---|---|---|---|---|---|---|---|---|
| C-14759 | Me(R) | single bond | H | morpholin-4-yl | Me | Cl | Me | O |
| C-14760 | Me(S) | single bond | H | morpholin-4-yl | Me | Cl | Me | O |
| C-14761 | H | single bond | Me | morpholin-4-yl | Me | Me | Me | O |
| C-14762 | Me | single bond | H | morpholin-4-yl | Me | Me | Me | O |
| C-14763 | Me(R) | single bond | H | morpholin-4-yl | Me | Me | Me | O |
| C-14764 | Me(S) | single bond | H | morpholin-4-yl | Me | Me | Me | O |
| C-14765 | H | single bond | Me | Ph | Me | Cl | Me | O |
| C-14766 | Me | single bond | H | Ph | Me | Cl | Me | O |
| C-14767 | Me(R) | single bond | H | Ph | Me | Cl | Me | O |
| C-14768 | Me(S) | single bond | H | Ph | Me | Cl | Me | O |
| C-14769 | H | single bond | Me | Ph | Me | Me | Me | O |
| C-14770 | Me | single bond | H | Ph | Me | Me | Me | O |
| C-14771 | Me(R) | single bond | H | Ph | Me | Me | Me | O |
| C-14772 | Me(S) | single bond | H | Ph | Me | Me | Me | O |
| C-14773 | H | single bond | Me | CH₂Ph | Me | Cl | Me | O |
| C-14774 | Me | single bond | H | CH₂Ph | Me | Cl | Me | O |
| C-14775 | Me(R) | single bond | H | CH₂Ph | Me | Cl | Me | O |
| C-14776 | Me(S) | single bond | H | CH₂Ph | Me | Cl | Me | O |
| C-14777 | H | single bond | Me | CH₂Ph | Me | Me | Me | O |
| C-14778 | Me | single bond | H | CH₂Ph | Me | Me | Me | O |
| C-14779 | Me(R) | single bond | H | CH₂Ph | Me | Me | Me | O |
| C-14780 | Me(S) | single bond | H | CH₂Ph | Me | Me | Me | O |
| C-14781 | H | single bond | Me | thiazole-2-yl | Me | Cl | Me | O |
| C-14782 | Me | single bond | H | thiazole-2-yl | Me | Cl | Me | O |
| C-14783 | Me(R) | single bond | H | thiazole-2-yl | Me | Cl | Me | O |
| C-14784 | Me(S) | single bond | H | thiazole-2-yl | Me | Cl | Me | O |
| C-14785 | H | single bond | Me | thiazole-2-yl | Me | Me | Me | O |
| C-14786 | Me | single bond | H | thiazole-2-yl | Me | Me | Me | O |
| C-14787 | Me(R) | single bond | H | thiazole-2-yl | Me | Me | Me | O |
| C-14788 | Me(S) | single bond | H | thiazole-2-yl | Me | Me | Me | O |
| C-14789 | H | single bond | Me | 1-Me-pyrazol-3-yl | Me | Cl | Me | O |
| C-14790 | Me | single bond | H | 1-Me-pyrazol-3-yl | Me | Cl | Me | O |
| C-14791 | Me(R) | single bond | H | 1-Me-pyrazol-3-yl | Me | Cl | Me | O |
| C-14792 | Me(S) | single bond | H | 1-Me-pyrazol-3-yl | Me | Cl | Me | O |
| C-14793 | H | single bond | Me | 1-Me-pyrazol-3-yl | Me | Me | Me | O |
| C-14794 | Me | single bond | H | 1-Me-pyrazol-3-yl | Me | Me | Me | O |
| C-14795 | Me(R) | single bond | H | 1-Me-pyrazol-3-yl | Me | Me | Me | O |
| C-14796 | Me(S) | single bond | H | 1-Me-pyrazol-3-yl | Me | Me | Me | O |
| C-14797 | H | single bond | Me | 1-Me-pyrazol-5-yl | Me | Cl | Me | O |
| C-14798 | Me | single bond | H | 1-Me-pyrazol-5-yl | Me | Cl | Me | O |
| C-14799 | Me(R) | single bond | H | 1-Me-pyrazol-5-yl | Me | Cl | Me | O |
| C-14800 | Me(S) | single bond | H | 1-Me-pyrazol-5-yl | Me | Cl | Me | O |
| C-14801 | H | single bond | Me | 1-Me-pyrazol-5-yl | Me | Me | Me | O |
| C-14802 | Me | single bond | H | 1-Me-pyrazol-5-yl | Me | Me | Me | O |

**[Table 565]**

| Compound Number | Y | W | Z | R¹ | R² | R³ | R¹⁰ | A |
|---|---|---|---|---|---|---|---|---|
| C-14803 | Me(R) | single bond | H | 1-Me-pyrazol-5-yl | Me | Me | Me | O |
| C-14804 | Me(S) | single bond | H | 1-Me-pyrazol-5-yl | Me | Me | Me | O |
| C-14805 | H | single bond | Me | CH₂(pyrimidin-2-yl) | Me | Cl | Me | O |
| C-14806 | Me | single bond | H | CH₂(pyrimidin-2-yl) | Me | Cl | Me | O |
| C-14807 | Me(R) | single bond | H | CH₂(pyrimidin-2-yl) | Me | Cl | Me | O |
| C-14808 | Me(S) | single bond | H | CH₂(pyrimidin-2-yl) | Me | Cl | Me | O |
| C-14809 | H | single bond | Me | CH₂(pyrimidin-2-yl) | Me | Me | Me | O |
| C-14810 | Me | single bond | H | CH₂(pyrimidin-2-yl) | Me | Me | Me | O |
| C-14811 | Me(R) | single bond | H | CH₂(pyrimidin-2-yl) | Me | Me | Me | O |
| C-14812 | Me(S) | single bond | H | CH₂(pyrimidin-2-yl) | Me | Me | Me | O |
| C-14813 | H | single bond | Me | Me | Me | Cl | CH₂OMe | O |
| C-14814 | Me | single bond | H | Me | Me | Cl | CH₂OMe | O |
| C-14815 | Me(R) | single bond | H | Me | Me | Cl | CH₂OMe | O |
| C-14816 | Me(S) | single bond | H | Me | Me | Cl | CH₂OMe | O |
| C-14817 | H | single bond | Me | Me | Me | Me | CH₂OMe | O |
| C-14818 | Me | single bond | H | Me | Me | Me | CH₂OMe | O |
| C-14819 | Me(R) | single bond | H | Me | Me | Me | CH₂OMe | O |
| C-14820 | Me(S) | single bond | H | Me | Me | Me | CH₂OMe | O |
| C-14821 | H | single bond | Me | CH₂CHF₂ | Me | Cl | CH₂OMe | O |
| C-14822 | Me | single bond | H | CH₂CHF₂ | Me | Cl | CH₂OMe | O |
| C-14823 | Me(R) | single bond | H | CH₂CHF₂ | Me | Cl | CH₂OMe | O |
| C-14824 | Me(S) | single bond | H | CH₂CHF₂ | Me | Cl | CH₂OMe | O |
| C-14825 | H | single bond | Me | CH₂CHF₂ | Me | Me | CH₂OMe | O |
| C-14826 | Me | single bond | H | CH₂CHF₂ | Me | Me | CH₂OMe | O |
| C-14827 | Me(R) | single bond | H | CH₂CHF₂ | Me | Me | CH₂OMe | O |
| C-14828 | Me(S) | single bond | H | CH₂CHF₂ | Me | Me | CH₂OMe | O |
| C-14829 | H | single bond | Me | *n*-Pr | Me | Cl | CH₂OMe | O |
| C-14830 | Me | single bond | H | *n*-Pr | Me | Cl | CH₂OMe | O |
| C-14831 | Me(R) | single bond | H | *n*-Pr | Me | Cl | CH₂OMe | O |
| C-14832 | Me(S) | single bond | H | *n*-Pr | Me | Cl | CH₂OMe | O |
| C-14833 | H | single bond | Me | *n*-Pr | Me | Me | CH₂OMe | O |
| C-14834 | Me | single bond | H | *n*-Pr | Me | Me | CH₂OMe | O |
| C-14835 | Me(R) | single bond | H | *n*-Pr | Me | Me | CH₂OMe | O |
| C-14836 | Me(S) | single bond | H | *n*-Pr | Me | Me | CH₂OMe | O |
| C-14837 | H | single bond | Me | *i*-Pr | Me | Cl | CH₂OMe | O |
| C-14838 | Me | single bond | H | *i*-Pr | Me | Cl | CH₂OMe | O |
| C-14839 | Me(R) | single bond | H | *i*-Pr | Me | Cl | CH₂OMe | O |
| C-14840 | Me(S) | single bond | H | *i*-Pr | Me | Cl | CH₂OMe | O |
| C-14841 | H | single bond | Me | *i*-Pr | Me | Me | CH₂OMe | O |
| C-14842 | Me | single bond | H | *i*-Pr | Me | Me | CH₂OMe | O |
| C-14843 | Me(R) | single bond | H | *i*-Pr | Me | Me | CH₂OMe | O |
| C-14844 | Me(S) | single bond | H | *i*-Pr | Me | Me | CH₂OMe | O |
| C-14845 | H | single bond | Me | *c*-Pr(1-F) | Me | Cl | CH₂OMe | O |
| C-14846 | Me | single bond | H | *c*-Pr(1-F) | Me | Cl | CH₂OMe | O |

**[Table 566]**

| Compound Number | Y | W | Z | R¹ | R² | R³ | R¹⁰ | A |
|---|---|---|---|---|---|---|---|---|
| C-14847 | Me(R) | single bond | H | *c*-Pr(1-F) | Me | Cl | CH₂OMe | O |
| C-14848 | Me(S) | single bond | H | *c*-Pr(1-F) | Me | Cl | CH₂OMe | O |
| C-14849 | H | single bond | Me | *c*-Pr(1-F) | Me | Me | CH₂OMe | O |
| C-14850 | Me | single bond | H | *c*-Pr(1-F) | Me | Me | CH₂OMe | O |
| C-14851 | Me(R) | single bond | H | *c*-Pr(1-F) | Me | Me | CH₂OMe | O |
| C-14852 | Me(S) | single bond | H | *c*-Pr(1-F) | Me | Me | CH₂OMe | O |
| C-14853 | H | single bond | Me | *c*-Pr(2,2-F₂) | Me | Cl | CH₂OMe | O |
| C-14854 | Me | single bond | H | *c*-Pr(2,2-F₂) | Me | Cl | CH₂OMe | O |
| C-14855 | Me(R) | single bond | H | *c*-Pr(2,2-F₂) | Me | Cl | CH₂OMe | O |
| C-14856 | Me(S) | single bond | H | *c*-Pr(2,2-F₂) | Me | Cl | CH₂OMe | O |
| C-14857 | H | single bond | Me | *c*-Pr(2,2-F₂) | Me | Me | CH₂OMe | O |
| C-14858 | Me | single bond | H | *c*-Pr(2,2-F₂) | Me | Me | CH₂OMe | O |
| C-14859 | Me(R) | single bond | H | *c*-Pr(2,2-F₂) | Me | Me | CH₂OMe | O |
| C-14860 | Me(S) | single bond | H | *c*-Pr(2,2-F₂) | Me | Me | CH₂OMe | O |
| C-14861 | H | single bond | Me | *c*-Pr(1-Cl) | Me | Cl | CH₂OMe | O |
| C-14862 | Me | single bond | H | *c*-Pr(1-Cl) | Me | Cl | CH₂OMe | O |
| C-14863 | Me(R) | single bond | H | *c*-Pr(1-Cl) | Me | Cl | CH₂OMe | O |
| C-14864 | Me(S) | single bond | H | *c*-Pr(1-Cl) | Me | Cl | CH₂OMe | O |
| C-14865 | H | single bond | Me | *c*-Pr(1-Cl) | Me | Me | CH₂OMe | O |
| C-14866 | Me | single bond | H | *c*-Pr(1-Cl) | Me | Me | CH₂OMe | O |
| C-14867 | Me(R) | single bond | H | *c*-Pr(1-Cl) | Me | Me | CH₂OMe | O |
| C-14868 | Me(S) | single bond | H | *c*-Pr(1-Cl) | Me | Me | CH₂OMe | O |
| C-14869 | H | single bond | Me | *c*-Pr(1-Br) | Me | Cl | CH₂OMe | O |
| C-14870 | Me | single bond | H | *c*-Pr(1-Br) | Me | Cl | CH₂OMe | O |
| C-14871 | Me(R) | single bond | H | *c*-Pr(1-Br) | Me | Cl | CH₂OMe | O |
| C-14872 | Me(S) | single bond | H | *c*-Pr(1-Br) | Me | Cl | CH₂OMe | O |
| C-14873 | H | single bond | Me | *c*-Pr(1-Br) | Me | Me | CH₂OMe | O |
| C-14874 | Me | single bond | H | *c*-Pr(1-Br) | Me | Me | CH₂OMe | O |
| C-14875 | Me(R) | single bond | H | *c*-Pr(1-Br) | Me | Me | CH₂OMe | O |
| C-14876 | Me(S) | single bond | H | *c*-Pr(1-Br) | Me | Me | CH₂OMe | O |
| C-14877 | H | single bond | Me | *c*-Pr(1-Me) | Me | Cl | CH₂OMe | O |
| C-14878 | Me | single bond | H | *c*-Pr(1-Me) | Me | Cl | CH₂OMe | O |
| C-14879 | Me(R) | single bond | H | *c*-Pr(1-Me) | Me | Cl | CH₂OMe | O |
| C-14880 | Me(S) | single bond | H | *c*-Pr(1-Me) | Me | Cl | CH₂OMe | O |
| C-14881 | H | single bond | Me | *c*-Pr(1-Me) | Me | Me | CH₂OMe | O |
| C-14882 | Me | single bond | H | *c*-Pr(1-Me) | Me | Me | CH₂OMe | O |
| C-14883 | Me(R) | single bond | H | *c*-Pr(1-Me) | Me | Me | CH₂OMe | O |
| C-14884 | Me(S) | single bond | H | *c*-Pr(1-Me) | Me | Me | CH₂OMe | O |
| C-14885 | H | single bond | Me | *c*-Pr(1-CH₂OH) | Me | Cl | CH₂OMe | O |
| C-14886 | Me | single bond | H | *c*-Pr(1-CH₂OH) | Me | Cl | CH₂OMe | O |
| C-14887 | Me(R) | single bond | H | *c*-Pr(1-CH₂OH) | Me | Cl | CH₂OMe | O |
| C-14888 | Me(S) | single bond | H | *c*-Pr(1-CH₂OH) | Me | Cl | CH₂OMe | O |
| C-14889 | H | single bond | Me | *c*-Pr(1-CH₂OH) | Me | Me | CH₂OMe | O |
| C-14890 | Me | single bond | H | *c*-Pr(1-CH₂OH) | Me | Me | CH₂OMe | O |

**[Table 567]**

| Compound Number | Y | W | Z | R¹ | R² | R³ | R¹⁰ | A |
|---|---|---|---|---|---|---|---|---|
| C-14891 | Me(R) | single bond | H | *c*-Pr(1-CH₂OH) | Me | Me | CH₂OMe | O |
| C-14892 | Me(S) | single bond | H | *c*-Pr(1-CH₂OH) | Me | Me | CH₂OMe | O |
| C-14893 | H | single bond | Me | *c*-Pr[1-CH₂OS(=O)₂Me] | Me | Cl | CH₂OMe | O |
| C-14894 | Me | single bond | H | *c*-Pr[1-CH₂OS(=O)₂Me] | Me | Cl | CH₂OMe | O |
| C-14895 | Me(R) | single bond | H | *c*-Pr[1-CH₂OS(=O)₂Me] | Me | Cl | CH₂OMe | O |
| C-14896 | Me(S) | single bond | H | *c*-Pr[1-CH₂OS(=O)₂Me] | Me | Cl | CH₂OMe | O |
| C-14897 | H | single bond | Me | *c*-Pr[1-CH₂OS(=O)₂Me] | Me | Me | CH₂OMe | O |
| C-14898 | Me | single bond | H | *c*-Pr[1-CH₂OS(=O)₂Me] | Me | Me | CH₂OMe | O |
| C-14899 | Me(R) | single bond | H | *c*-Pr[1-CH₂OS(=O)₂Me] | Me | Me | CH₂OMe | O |
| C-14900 | Me(S) | single bond | H | *c*-Pr[1-CH₂OS(=O)₂Me] | Me | Me | CH₂OMe | O |
| C-14901 | H | single bond | Me | *c*-Pr(1-CH₂OMe) | Me | Cl | CH₂OMe | O |
| C-14902 | Me | single bond | H | *c*-Pr(1-CH₂OMe) | Me | Cl | CH₂OMe | O |
| C-14903 | Me(R) | single bond | H | *c*-Pr(1-CH₂OMe) | Me | Cl | CH₂OMe | O |
| C-14904 | Me(S) | single bond | H | *c*-Pr(1-CH₂OMe) | Me | Cl | CH₂OMe | O |
| C-14905 | H | single bond | Me | *c*-Pr(1-CH₂OMe) | Me | Me | CH₂OMe | O |
| C-14906 | Me | single bond | H | *c*-Pr(1-CH₂OMe) | Me | Me | CH₂OMe | O |
| C-14907 | Me(R) | single bond | H | *c*-Pr(1-CH₂OMe) | Me | Me | CH₂OMe | O |
| C-14908 | Me(S) | single bond | H | *c*-Pr(1-CH₂OMe) | Me | Me | CH₂OMe | O |
| C-14909 | H | single bond | Me | *c*-Pr(1-CH₂CN) | Me | Cl | CH₂OMe | O |
| C-14910 | Me | single bond | H | *c*-Pr(1-CH₂CN) | Me | Cl | CH₂OMe | O |
| C-14911 | Me(R) | single bond | H | *c*-Pr(1-CH₂CN) | Me | Cl | CH₂OMe | O |
| C-14912 | Me(S) | single bond | H | *c*-Pr(1-CH₂CN) | Me | Cl | CH₂OMe | O |
| C-14913 | H | single bond | Me | *c*-Pr(1-CH₂CN) | Me | Me | CH₂OMe | O |
| C-14914 | Me | single bond | H | *c*-Pr(1-CH₂CN) | Me | Me | CH₂OMe | O |
| C-14915 | Me(R) | single bond | H | *c*-Pr(1-CH₂CN) | Me | Me | CH₂OMe | O |
| C-14916 | Me(S) | single bond | H | *c*-Pr(1-CH₂CN) | Me | Me | CH₂OMe | O |
| C-14917 | H | single bond | Me | *c*-Pr(1-CH₂SMe) | Me | Cl | CH₂OMe | O |
| C-14918 | Me | single bond | H | *c*-Pr(1-CH₂SMe) | Me | Cl | CH₂OMe | O |
| C-14919 | Me(R) | single bond | H | *c*-Pr(1-CH₂SMe) | Me | Cl | CH₂OMe | O |
| C-14920 | Me(S) | single bond | H | *c*-Pr(1-CH₂SMe) | Me | Cl | CH₂OMe | O |
| C-14921 | H | single bond | Me | *c*-Pr(1-CH₂SMe) | Me | Me | CH₂OMe | O |
| C-14922 | Me | single bond | H | *c*-Pr(1-CH₂SMe) | Me | Me | CH₂OMe | O |
| C-14923 | Me(R) | single bond | H | *c*-Pr(1-CH₂SMe) | Me | Me | CH₂OMe | O |
| C-14924 | Me(S) | single bond | H | *c*-Pr(1-CH₂SMe) | Me | Me | CH₂OMe | O |
| C-14925 | H | single bond | Me | *c*-Pr[1-CH₂S(=O)Me] | Me | Cl | CH₂OMe | O |
| C-14926 | Me | single bond | H | *c*-Pr[1-CH₂S(=O)Me] | Me | Cl | CH₂OMe | O |
| C-14927 | Me(R) | single bond | H | *c*-Pr[1-CH₂S(=O)Me] | Me | Cl | CH₂OMe | O |
| C-14928 | Me(S) | single bond | H | *c*-Pr[1-CH₂S(=O)Me] | Me | Cl | CH₂OMe | O |
| C-14929 | H | single bond | Me | *c*-Pr[1-CH₂S(=O)Me] | Me | Me | CH₂OMe | O |
| C-14930 | Me | single bond | H | *c*-Pr[1-CH₂S(=O)Me] | Me | Me | CH₂OMe | O |
| C-14931 | Me(R) | single bond | H | *c*-Pr[1-CH₂S(=O)Me] | Me | Me | CH₂OMe | O |
| C-14932 | Me(S) | single bond | H | *c*-Pr[1-CH₂S(=O)Me] | Me | Me | CH₂OMe | O |
| C-14933 | H | single bond | Me | *c*-Pr[1-CH₂S(=O)₂Me] | Me | Cl | CH₂OMe | O |
| C-14934 | Me | single bond | H | *c*-Pr[1-CH₂S(=O)₂Me] | Me | Cl | CH₂OMe | O |

**[Table 568]**

| Compound Number | Y | W | Z | R¹ | R² | R³ | R¹⁰ | A |
|---|---|---|---|---|---|---|---|---|
| C-14935 | Me(R) | single bond | H | *c*-Pr[1-CH₂S(=O)₂Me] | Me | Cl | CH₂OMe | O |
| C-14936 | Me(S) | single bond | H | *c*-Pr[1-CH₂S(=O)₂Me] | Me | Cl | CH₂OMe | O |
| C-14937 | H | single bond | Me | *c*-Pr[1-CH₂S(=O)₂Me] | Me | Me | CH₂OMe | O |
| C-14938 | Me | single bond | H | *c*-Pr[1-CH₂S(=O)₂Me] | Me | Me | CH₂OMe | O |
| C-14939 | Me(R) | single bond | H | *c*-Pr[1-CH₂S(=O)₂Me] | Me | Me | CH₂OMe | O |
| C-14940 | Me(S) | single bond | H | *c*-Pr[1-CH₂S(=O)₂Me] | Me | Me | CH₂OMe | O |
| C-14941 | H | single bond | Me | *c*-Pr(1-CH₂F) | Me | Cl | CH₂OMe | O |
| C-14942 | Me | single bond | H | *c*-Pr(1-CH₂F) | Me | Cl | CH₂OMe | O |
| C-14943 | Me(R) | single bond | H | *c*-Pr(1-CH₂F) | Me | Cl | CH₂OMe | O |
| C-14944 | Me(S) | single bond | H | *c*-Pr(1-CH₂F) | Me | Cl | CH₂OMe | O |
| C-14945 | H | single bond | Me | *c*-Pr(1-CH₂F) | Me | Me | CH₂OMe | O |
| C-14946 | Me | single bond | H | *c*-Pr(1-CH₂F) | Me | Me | CH₂OMe | O |
| C-14947 | Me(R) | single bond | H | *c*-Pr(1-CH₂F) | Me | Me | CH₂OMe | O |
| C-14948 | Me(S) | single bond | H | *c*-Pr(1-CH₂F) | Me | Me | CH₂OMe | O |
| C-14949 | H | single bond | Me | *c*-Pr(1-CHF₂) | Me | Cl | CH₂OMe | O |
| C-14950 | Me | single bond | H | *c*-Pr(1-CHF₂) | Me | Cl | CH₂OMe | O |
| C-14951 | Me(R) | single bond | H | *c*-Pr(1-CHF₂) | Me | Cl | CH₂OMe | O |
| C-14952 | Me(S) | single bond | H | *c*-Pr(1-CHF₂) | Me | Cl | CH₂OMe | O |
| C-14953 | H | single bond | Me | *c*-Pr(1-CHF₂) | Me | Me | CH₂OMe | O |
| C-14954 | Me | single bond | H | *c*-Pr(1-CHF₂) | Me | Me | CH₂OMe | O |
| C-14955 | Me(R) | single bond | H | *c*-Pr(1-CHF₂) | Me | Me | CH₂OMe | O |
| C-14956 | Me(S) | single bond | H | *c*-Pr(1-CHF₂) | Me | Me | CH₂OMe | O |
| C-14957 | H | single bond | Me | *c*-Pr(1-CF₃) | Me | Cl | CH₂OMe | O |
| C-14958 | Me | single bond | H | *c*-Pr(1-CF₃) | Me | Cl | CH₂OMe | O |
| C-14959 | Me(R) | single bond | H | *c*-Pr(1-CF₃) | Me | Cl | CH₂OMe | O |
| C-14960 | Me(S) | single bond | H | *c*-Pr(1-CF₃) | Me | Cl | CH₂OMe | O |
| C-14961 | H | single bond | Me | *c*-Pr(1-CF₃) | Me | Me | CH₂OMe | O |
| C-14962 | Me | single bond | H | *c*-Pr(1-CF₃) | Me | Me | CH₂OMe | O |
| C-14963 | Me(R) | single bond | H | *c*-Pr(1-CF₃) | Me | Me | CH₂OMe | O |
| C-14964 | Me(S) | single bond | H | *c*-Pr(1-CF₃) | Me | Me | CH₂OMe | O |
| C-14965 | H | single bond | Me | *c*-Pr(1-C≡CH) | Me | Cl | CH₂OMe | O |
| C-14966 | Me | single bond | H | *c*-Pr(1-C≡CH) | Me | Cl | CH₂OMe | O |
| C-14967 | Me(R) | single bond | H | *c*-Pr(1-C≡CH) | Me | Cl | CH₂OMe | O |
| C-14968 | Me(S) | single bond | H | *c*-Pr(1-C≡CH) | Me | Cl | CH₂OMe | O |
| C-14969 | H | single bond | Me | *c*-Pr(1-C≡CH) | Me | Me | CH₂OMe | O |
| C-14970 | Me | single bond | H | *c*-Pr(1-C≡CH) | Me | Me | CH₂OMe | O |
| C-14971 | Me(R) | single bond | H | *c*-Pr(1-C≡CH) | Me | Me | CH₂OMe | O |
| C-14972 | Me(S) | single bond | H | *c*-Pr(1-C≡CH) | Me | Me | CH₂OMe | O |
| C-14973 | H | single bond | Me | *c*-Pr[1-C(=O)OEt] | Me | Cl | CH₂OMe | O |
| C-14974 | Me | single bond | H | *c*-Pr[1-C(=O)OEt] | Me | Cl | CH₂OMe | O |
| C-14975 | Me(R) | single bond | H | *c*-Pr[1-C(=O)OEt] | Me | Cl | CH₂OMe | O |
| C-14976 | Me(S) | single bond | H | *c*-Pr[1-C(=O)OEt] | Me | Cl | CH₂OMe | O |
| C-14977 | H | single bond | Me | *c*-Pr[1-C(=O)OEt] | Me | Me | CH₂OMe | O |
| C-14978 | Me | single bond | H | *c*-Pr[1-C(=O)OEt] | Me | Me | CH₂OMe | O |

**[Table 569]**

| Compound Number | Y | W | Z | R¹ | R² | R³ | R¹⁰ | A |
|---|---|---|---|---|---|---|---|---|
| C-14979 | Me(R) | single bond | H | *c*-Pr[1-C(=O)OEt] | Me | Me | CH₂OMe | O |
| C-14980 | Me(S) | single bond | H | *c-*Pr[1-C(=O)OEt] | Me | Me | CH₂OMe | O |
| C-14981 | H | single bond | Me | *c*-Pr[1-C(=O)OH] | Me | Cl | CH₂OMe | O |
| C-14982 | Me | single bond | H | *c*-Pr[1-C(=O)OH] | Me | Cl | CH₂OMe | O |
| C-14983 | Me(R) | single bond | H | *c*-Pr[1-C(=O)OH] | Me | Cl | CH₂OMe | O |
| C-14984 | Me(S) | single bond | H | *c*-Pr[1-C(=O)OH] | Me | Cl | CH₂OMe | O |
| C-14985 | H | single bond | Me | *c*-Pr[1-C(=O)OH] | Me | Me | CH₂OMe | O |
| C-14986 | Me | single bond | H | *c*-Pr[1-C(=O)OH] | Me | Me | CH₂OMe | O |
| C-14987 | Me(R) | single bond | H | *c*-Pr[1-C(=O)OH] | Me | Me | CH₂OMe | O |
| C-14988 | Me(S) | single bond | H | *c*-Pr[1-C(=O)OH] | Me | Me | CH₂OMe | O |
| C-14989 | H | single bond | Me | *c*-Pr[1-C(=O)NH₂] | Me | Cl | CH₂OMe | O |
| C-14990 | Me | single bond | H | *c*-Pr[1-C(=O)NH₂] | Me | Cl | CH₂OMe | O |
| C-14991 | Me(R) | single bond | H | *c*-Pr[1-C(=O)NH₂] | Me | Cl | CH₂OMe | O |
| C-14992 | Me(S) | single bond | H | *c*-Pr[1-C(=O)NH₂] | Me | Cl | CH₂OMe | O |
| C-14993 | H | single bond | Me | *c*-Pr[1-C(=O)NH₂] | Me | Me | CH₂OMe | O |
| C-14994 | Me | single bond | H | *c*-Pr[1-C(=O)NH₂] | Me | Me | CH₂OMe | O |
| C-14995 | Me(R) | single bond | H | *c*-Pr[1-C(=O)NH₂] | Me | Me | CH₂OMe | O |
| C-14996 | Me(S) | single bond | H | *c*-Pr[1-C(=O)NH₂] | Me | Me | CH₂OMe | O |
| C-14997 | H | single bond | Me | *c*-Pr(1-OMe) | Me | Cl | CH₂OMe | O |
| C-14998 | Me | single bond | H | *c*-Pr(1-OMe) | Me | Cl | CH₂OMe | O |
| C-14999 | Me(R) | single bond | H | *c*-Pr(1-OMe) | Me | Cl | CH₂OMe | O |
| C-15000 | Me(S) | single bond | H | *c*-Pr(1-OMe) | Me | Cl | CH₂OMe | O |
| C-15001 | H | single bond | Me | *c*-Pr(1-OMe) | Me | Me | CH₂OMe | O |
| C-15002 | Me | single bond | H | *c*-Pr(1-OMe) | Me | Me | CH₂OMe | O |
| C-15003 | Me(R) | single bond | H | *c*-Pr(1-OMe) | Me | Me | CH₂OMe | O |
| C-15004 | Me(S) | single bond | H | *c*-Pr(1-OMe) | Me | Me | CH₂OMe | O |
| C-15005 | H | single bond | Me | *c*-Pr(1-SMe) | Me | Cl | CH₂OMe | O |
| C-15006 | Me | single bond | H | *c*-Pr(1-SMe) | Me | Cl | CH₂OMe | O |
| C-15007 | Me(R) | single bond | H | *c*-Pr(1-SMe) | Me | Cl | CH₂OMe | O |
| C-15008 | Me(S) | single bond | H | *c*-Pr(1-SMe) | Me | Cl | CH₂OMe | O |
| C-15009 | H | single bond | Me | *c*-Pr(1-SMe) | Me | Me | CH₂OMe | O |
| C-15010 | Me | single bond | H | *c*-Pr(1-SMe) | Me | Me | CH₂OMe | O |
| C-15011 | Me(R) | single bond | H | *c*-Pr(1-SMe) | Me | Me | CH₂OMe | O |
| C-15012 | Me(S) | single bond | H | *c*-Pr(1-SMe) | Me | Me | CH₂OMe | O |
| C-15013 | H | single bond | Me | *c*-Pr[1-S(=O)Me] | Me | Cl | CH₂OMe | O |
| C-15014 | Me | single bond | H | *c*-Pr[1-S(=O)Me] | Me | Cl | CH₂OMe | O |
| C-15015 | Me(R) | single bond | H | *c*-Pr[1-S(=O)Me] | Me | Cl | CH₂OMe | O |
| C-15016 | Me(S) | single bond | H | *c*-Pr[1-S(=O)Me] | Me | Cl | CH₂OMe | O |
| C-15017 | H | single bond | Me | *c*-Pr[1-S(=O)Me] | Me | Me | CH₂OMe | O |
| C-15018 | Me | single bond | H | *c*-Pr[1-S(=O)Me] | Me | Me | CH₂OMe | O |
| C-15019 | Me(R) | single bond | H | *c*-Pr[1-S(=O)Me] | Me | Me | CH₂OMe | O |
| C-15020 | Me(S) | single bond | H | *c*-Pr[1-S(=O)Me] | Me | Me | CH₂OMe | O |
| C-15021 | H | single bond | Me | *c*-Pr[1-S(=O)₂Me] | Me | Cl | CH₂OMe | O |
| C-15022 | Me | single bond | H | *c*-Pr[1-S(=O)₂Me] | Me | Cl | CH₂OMe | O |

**[Table 570]**

| Compound Number | Y | W | Z | R¹ | R² | R³ | R¹⁰ | A |
|---|---|---|---|---|---|---|---|---|
| C-15023 | Me(R) | single bond | H | *c*-Pr[1-S(=O)₂Me] | Me | Cl | CH₂OMe | O |
| C-15024 | Me(S) | single bond | H | *c*-Pr[1-S(=O)₂Me] | Me | Cl | CH₂OMe | O |
| C-15025 | H | single bond | Me | *c*-Pr[1-S(=O)₂Me] | Me | Me | CH₂OMe | O |
| C-15026 | Me | single bond | H | *c*-Pr[1-S(=O)₂Me] | Me | Me | CH₂OMe | O |
| C-15027 | Me(R) | single bond | H | *c*-Pr[1-S(=O)₂Me] | Me | Me | CH₂OMe | O |
| C-15028 | Me(S) | single bond | H | *c*-Pr[1-S(=O)₂Me] | Me | Me | CH₂OMe | O |
| C-15029 | H | single bond | Me | *c*-Pr[1-S(=O)₂CHF₂] | Me | Cl | CH₂OMe | O |
| C-15030 | Me | single bond | H | *c*-Pr[1-S(=O)₂CHF₂] | Me | Cl | CH₂OMe | O |
| C-15031 | Me(R) | single bond | H | *c*-Pr[1-S(=O)₂CHF₂] | Me | Cl | CH₂OMe | O |
| C-15032 | Me(S) | single bond | H | *c*-Pr[1-S(=O)₂CHF₂] | Me | Cl | CH₂OMe | O |
| C-15033 | H | single bond | Me | *c*-Pr[1-S(=O)₂CHF₂] | Me | Me | CH₂OMe | O |
| C-15034 | Me | single bond | H | *c*-Pr[1-S(=O)₂CHF₂] | Me | Me | CH₂OMe | O |
| C-15035 | Me(R) | single bond | H | *c*-Pr[1-S(=O)₂CHF₂] | Me | Me | CH₂OMe | O |
| C-15036 | Me(S) | single bond | H | *c*-Pr[1-S(=O)₂CHF₂] | Me | Me | CH₂OMe | O |
| C-15037 | H | single bond | Me | *c*-Pr[1-S(=O)₂CF₃] | Me | Cl | CH₂OMe | O |
| C-15038 | Me | single bond | H | *c*-Pr[1-S(=O)₂CF₃] | Me | Cl | CH₂OMe | O |
| C-15039 | Me(R) | single bond | H | *c*-Pr[1-S(=O)₂CF₃] | Me | Cl | CH₂OMe | O |
| C-15040 | Me(S) | single bond | H | *c*-Pr[1-S(=O)₂CF₃] | Me | Cl | CH₂OMe | O |
| C-15041 | H | single bond | Me | *c*-Pr[1-S(=O)₂CF₃] | Me | Me | CH₂OMe | O |
| C-15042 | Me | single bond | H | *c*-Pr[1-S(=O)₂CF₃] | Me | Me | CH₂OMe | O |
| C-15043 | Me(R) | single bond | H | *c*-Pr[1-S(=O)₂CF₃] | Me | Me | CH₂OMe | O |
| C-15044 | Me(S) | single bond | H | *c*-Pr[1-S(=O)₂CF₃] | Me | Me | CH₂OMe | O |
| C-15045 | H | single bond | Me | *c*-Pr[1-(5,6-dihydro-1,4,2-dioxazin-3-yl)] | Me | Cl | CH₂OMe | O |
| C-15046 | Me | single bond | H | *c*-Pr[1-(5,6-dihydro-1,4,2-dioxazin-3-yl)] | Me | Cl | CH₂OMe | O |
| C-15047 | Me(R) | single bond | H | *c*-Pr[1-(5,6-dihydro-1,4,2-dioxazin-3-yl)] | Me | Cl | CH₂OMe | O |
| C-15048 | Me(S) | single bond | H | *c*-Pr[1-(5,6-dihydro-1,4,2-dioxazin-3-yl)] | Me | Cl | CH₂OMe | O |
| C-15049 | H | single bond | Me | *c*-Pr[1-(5,6-dihydro-1,4,2-dioxazin-3-yl)] | Me | Me | CH₂OMe | O |
| C-15050 | Me | single bond | H | *c*-Pr[1-(5,6-dihydro-1,4,2-dioxazin-3-yl)] | Me | Me | CH₂OMe | O |
| C-15051 | Me(R) | single bond | H | *c*-Pr[1-(5,6-dihydro-1,4,2-dioxazin-3-yl)] | Me | Me | CH₂OMe | O |
| C-15052 | Me(S) | single bond | H | *c*-Pr[1-(5,6-dihydro-1,4,2-dioxazin-3-yl)] | Me | Me | CH₂OMe | O |
| C-15053 | H | single bond | Me | *c*-Pr[1-(5-hydroxy-1,3,4-oxadiazol-2-yl)] | Me | Cl | CH₂OMe | O |
| C-15054 | Me | single bond | H | *c*-Pr[1-(5-hydroxy-1,3,4-oxadiazol-2-yl)] | Me | Cl | CH₂OMe | O |
| C-15055 | Me(R) | single bond | H | *c*-Pr[1-(5-hydroxy-1,3,4-oxadiazol-2-yl)] | Me | Cl | CH₂OMe | O |
| C-15056 | Me(S) | single bond | H | *c*-Pr[1-(5-hydroxy-1,3,4-oxadiazol-2-yl)] | Me | Cl | CH₂OMe | O |
| C-15057 | H | single bond | Me | *c*-Pr[1-(5-hydroxy-1,3,4-oxadiazol-2-yl)] | Me | Me | CH₂OMe | O |
| C-15058 | Me | single bond | H | *c*-Pr[1-(5-hydroxy-1,3,4-oxadiazol-2-yl)] | Me | Me | CH₂OMe | O |
| C-15059 | Me(R) | single bond | H | *c*-Pr[1-(5-hydroxy-1,3,4-oxadiazol-2-yl)] | Me | Me | CH₂OMe | O |
| C-15060 | Me(S) | single bond | H | *c*-Pr[1-(5-hydroxy-1,3,4-oxadiazol-2-yl)] | Me | Me | CH₂OMe | O |
| C-15061 | H | single bond | Me | *c*-Pr[1-(pyrimidin-2-yl)] | Me | Cl | CH₂OMe | O |
| C-15062 | Me | single bond | H | *c*-Pr[1-(pyrimidin-2-yl)] | Me | Cl | CH₂OMe | O |
| C-15063 | Me(R) | single bond | H | *c*-Pr[1-(pyrimidin-2-yl)] | Me | Cl | CH₂OMe | O |
| C-15064 | Me(S) | single bond | H | *c*-Pr[1-(pyrimidin-2-yl)] | Me | Cl | CH₂OMe | O |
| C-15065 | H | single bond | Me | *c*-Pr[1-(pyrimidin-2-yl)] | Me | Me | CH₂OMe | O |
| C-15066 | Me | single bond | H | *c*-Pr[1-(pyrimidin-2-yl)] | Me | Me | CH₂OMe | O |

**[Table 571]**

| Compound Number | Y | W | Z | R¹ | R² | R³ | R¹⁰ | A |
|---|---|---|---|---|---|---|---|---|
| C-15067 | Me(R) | single bond | H | *c*-Pr[1-(pyrimidin-2-yl)] | Me | Me | CH₂OMe | O |
| C-15068 | Me(S) | single bond | H | *c*-Pr[1-(pyrimidin-2-yl)] | Me | Me | CH₂OMe | O |
| C-15069 | H | single bond | Me | *t*-Bu | Me | Cl | CH₂OMe | O |
| C-15070 | Me | single bond | H | *t*-Bu | Me | Cl | CH₂OMe | O |
| C-15071 | Me(R) | single bond | H | *t*-Bu | Me | Cl | CH₂OMe | O |
| C-15072 | Me(S) | single bond | H | *t*-Bu | Me | Cl | CH₂OMe | O |
| C-15073 | H | single bond | Me | *t*-Bu | Me | Me | CH₂OMe | O |
| C-15074 | Me | single bond | H | *t*-Bu | Me | Me | CH₂OMe | O |
| C-15075 | Me(R) | single bond | H | *t*-Bu | Me | Me | CH₂OMe | O |
| C-15076 | Me(S) | single bond | H | *t*-Bu | Me | Me | CH₂OMe | O |
| C-15077 | H | single bond | Me | *c*-Bu | Me | Cl | CH₂OMe | O |
| C-15078 | Me | single bond | H | *c*-Bu | Me | Cl | CH₂OMe | O |
| C-15079 | Me(R) | single bond | H | *c*-Bu | Me | Cl | CH₂OMe | O |
| C-15080 | Me(S) | single bond | H | *c*-Bu | Me | Cl | CH₂OMe | O |
| C-15081 | H | single bond | Me | *c*-Bu | Me | Me | CH₂OMe | O |
| C-15082 | Me | single bond | H | *c*-Bu | Me | Me | CH₂OMe | O |
| C-15083 | Me(R) | single bond | H | *c*-Bu | Me | Me | CH₂OMe | O |
| C-15084 | Me(S) | single bond | H | *c*-Bu | Me | Me | CH₂OMe | O |
| C-15085 | H | single bond | Me | *c*-Bu(1-CN) | Me | Cl | CH₂OMe | O |
| C-15086 | Me | single bond | H | *c*-Bu(1-CN) | Me | Cl | CH₂OMe | O |
| C-15087 | Me(R) | single bond | H | *c*-Bu(1-CN) | Me | Cl | CH₂OMe | O |
| C-15088 | Me(S) | single bond | H | *c*-Bu(1-CN) | Me | Cl | CH₂OMe | O |
| C-15089 | H | single bond | Me | *c*-Bu(1-CN) | Me | Me | CH₂OMe | O |
| C-15090 | Me | single bond | H | *c*-Bu(1-CN) | Me | Me | CH₂OMe | O |
| C-15091 | Me(R) | single bond | H | *c*-Bu(1-CN) | Me | Me | CH₂OMe | O |
| C-15092 | Me(S) | single bond | H | *c*-Bu(1-CN) | Me | Me | CH₂OMe | O |
| C-15093 | H | single bond | Me | *c*-Bu(3,3-F₂) | Me | Cl | CH₂OMe | O |
| C-15094 | Me | single bond | H | *c*-Bu(3,3-F₂) | Me | Cl | CH₂OMe | O |
| C-15095 | Me(R) | single bond | H | *c*-Bu(3,3-F₂) | Me | Cl | CH₂OMe | O |
| C-15096 | Me(S) | single bond | H | *c*-Bu(3,3-F₂) | Me | Cl | CH₂OMe | O |
| C-15097 | H | single bond | Me | *c*-Bu(3,3-F₂) | Me | Me | CH₂OMe | O |
| C-15098 | Me | single bond | H | *c*-Bu(3,3-F₂) | Me | Me | CH₂OMe | O |
| C-15099 | Me(R) | single bond | H | *c*-Bu(3,3-F₂) | Me | Me | CH₂OMe | O |
| C-15100 | Me(S) | single bond | H | *c*-Bu(3,3-F₂) | Me | Me | CH₂OMe | O |
| C-15101 | H | single bond | Me | oxetan-3-yl | Me | Cl | CH₂OMe | O |
| C-15102 | Me | single bond | H | oxetan-3-yl | Me | Cl | CH₂OMe | O |
| C-15103 | Me(R) | single bond | H | oxetan-3-yl | Me | Cl | CH₂OMe | O |
| C-15104 | Me(S) | single bond | H | oxetan-3-yl | Me | Cl | CH₂OMe | O |
| C-15105 | H | single bond | Me | oxetan-3-yl | Me | Me | CH₂OMe | O |
| C-15106 | Me | single bond | H | oxetan-3-yl | Me | Me | CH₂OMe | O |
| C-15107 | Me(R) | single bond | H | oxetan-3-yl | Me | Me | CH₂OMe | O |
| C-15108 | Me(S) | single bond | H | oxetan-3-yl | Me | Me | CH₂OMe | O |
| C-15109 | H | single bond | Me | oxetan(3-CN)-3-yl | Me | Cl | CH₂OMe | O |
| C-15110 | Me | single bond | H | oxetan(3-CN)-3-yl | Me | Cl | CH₂OMe | O |

**[Table 572]**

| Compound Number | Y | W | Z | R¹ | R² | R³ | R¹⁰ | A |
|---|---|---|---|---|---|---|---|---|
| C-15111 | Me(R) | single bond | H | oxetan(3-CN)-3-yl | Me | Cl | CH₂OMe | O |
| C-15112 | Me(S) | single bond | H | oxetan(3-CN)-3-yl | Me | Cl | CH₂OMe | O |
| C-15113 | H | single bond | Me | oxetan(3-CN)-3-yl | Me | Me | CH₂OMe | O |
| C-15114 | Me | single bond | H | oxetan(3-CN)-3-yl | Me | Me | CH₂OMe | O |
| C-15115 | Me(R) | single bond | H | oxetan(3-CN)-3-yl | Me | Me | CH₂OMe | O |
| C-15116 | Me(S) | single bond | H | oxetan(3-CN)-3-yl | Me | Me | CH₂OMe | O |
| C-15117 | H | single bond | Me | azetidin[1-C(=O)Me]-3-yl | Me | Cl | CH₂OMe | O |
| C-15118 | Me | single bond | H | azetidin[1-C(=O)Me]-3-yl | Me | Cl | CH₂OMe | O |
| C-15119 | Me(R) | single bond | H | azetidin[1-C(=O)Me]-3-yl | Me | Cl | CH₂OMe | O |
| C-15120 | Me(S) | single bond | H | azetidin[1-C(=O)Me]-3-yl | Me | Cl | CH₂OMe | O |
| C-15121 | H | single bond | Me | azetidin[1-C(=O)Me]-3-yl | Me | Me | CH₂OMe | O |
| C-15122 | Me | single bond | H | azetidin[1-C(=O)Me]-3-yl | Me | Me | CH₂OMe | O |
| C-15123 | Me(R) | single bond | H | azetidin[1-C(=O)Me]-3-yl | Me | Me | CH₂OMe | O |
| C-15124 | Me(S) | single bond | H | azetidin[1-C(=O)Me]-3-yl | Me | Me | CH₂OMe | O |
| C-15125 | H | single bond | Me | azetidin[1-S(=O)₂Me]-3-yl | Me | Cl | CH₂OMe | O |
| C-15126 | Me | single bond | H | azetidin[1-S(=O)₂Me]-3-yl | Me | Cl | CH₂OMe | O |
| C-15127 | Me(R) | single bond | H | azetidin[1-S(=O)₂Me]-3-yl | Me | Cl | CH₂OMe | O |
| C-15128 | Me(S) | single bond | H | azetidin[1-S(=O)₂Me]-3-yl | Me | Cl | CH₂OMe | O |
| C-15129 | H | single bond | Me | azetidin[1-S(=O)₂Me]-3-yl | Me | Me | CH₂OMe | O |
| C-15130 | Me | single bond | H | azetidin[1-S(=O)₂Me]-3-yl | Me | Me | CH₂OMe | O |
| C-15131 | Me(R) | single bond | H | azetidin[1-S(=O)₂Me]-3-yl | Me | Me | CH₂OMe | O |
| C-15132 | Me(S) | single bond | H | azetidin[1-S(=O)₂Me]-3-yl | Me | Me | CH₂OMe | O |
| C-15133 | H | single bond | Me | azetidin[1-S(=O)₂CHF₂]-3-yl | Me | Cl | CH₂OMe | O |
| C-15134 | Me | single bond | H | azetidin[1-S(=O)₂CHF₂]-3-yl | Me | Cl | CH₂OMe | O |
| C-15135 | Me(R) | single bond | H | azetidin[1-S(=O)₂CHF₂]-3-yl | Me | Cl | CH₂OMe | O |
| C-15136 | Me(S) | single bond | H | azetidin[1-S(=O)₂CHF₂]-3-yl | Me | Cl | CH₂OMe | O |
| C-15137 | H | single bond | Me | azetidin[1-S(=O)₂CHF₂]-3-yl | Me | Me | CH₂OMe | O |
| C-15138 | Me | single bond | H | azetidin[1-S(=O)₂CHF₂]-3-yl | Me | Me | CH₂OMe | O |
| C-15139 | Me(R) | single bond | H | azetidin[1-S(=O)₂CHF₂]-3-yl | Me | Me | CH₂OMe | O |
| C-15140 | Me(S) | single bond | H | azetidin[1-S(=O)₂CHF₂]-3-yl | Me | Me | CH₂OMe | O |
| C-15141 | H | single bond | Me | *c*-Pen | Me | Cl | CH₂OMe | O |
| C-15142 | Me | single bond | H | *c*-Pen | Me | Cl | CH₂OMe | O |
| C-15143 | Me(R) | single bond | H | *c*-Pen | Me | Cl | CH₂OMe | O |
| C-15144 | Me(S) | single bond | H | *c*-Pen | Me | Cl | CH₂OMe | O |
| C-15145 | H | single bond | Me | *c*-Pen | Me | Me | CH₂OMe | O |
| C-15146 | Me | single bond | H | *c*-Pen | Me | Me | CH₂OMe | O |
| C-15147 | Me(R) | single bond | H | *c*-Pen | Me | Me | CH₂OMe | O |
| C-15148 | Me(S) | single bond | H | *c*-Pen | Me | Me | CH₂OMe | O |
| C-15149 | H | single bond | Me | tetrahydrofuran-3-yl | Me | Cl | CH₂OMe | O |
| C-15150 | Me | single bond | H | tetrahydrofuran-3-yl | Me | Cl | CH₂OMe | O |
| C-15151 | Me(R) | single bond | H | tetrahydrofuran-3-yl | Me | Cl | CH₂OMe | O |
| C-15152 | Me(S) | single bond | H | tetrahydrofuran-3-yl | Me | Cl | CH₂OMe | O |
| C-15153 | H | single bond | Me | tetrahydrofuran-3-yl | Me | Me | CH₂OMe | O |
| C-15154 | Me | single bond | H | tetrahydrofuran-3-yl | Me | Me | CH₂OMe | O |

**[Table 573]**

| Compound Number | Y | W | Z | R¹ | R² | R³ | R¹⁰ | A |
|---|---|---|---|---|---|---|---|---|
| C-15155 | Me(R) | single bond | H | tetrahydrofuran-3-yl | Me | Me | CH₂OMe | O |
| C-15156 | Me(S) | single bond | H | tetrahydrofuran-3-yl | Me | Me | CH₂OMe | O |
| C-15157 | H | single bond | Me | tetrahydrothiophen-3-yl | Me | Cl | CH₂OMe | O |
| C-15158 | Me | single bond | H | tetrahydrothiophen-3-yl | Me | Cl | CH₂OMe | O |
| C-15159 | Me(R) | single bond | H | tetrahydrothiophen-3-yl | Me | Cl | CH₂OMe | O |
| C-15160 | Me(S) | single bond | H | tetrahydrothiophen-3-yl | Me | Cl | CH₂OMe | O |
| C-15161 | H | single bond | Me | tetrahydrothiophen-3-yl | Me | Me | CH₂OMe | O |
| C-15162 | Me | single bond | H | tetrahydrothiophen-3-yl | Me | Me | CH₂OMe | O |
| C-15163 | Me(R) | single bond | H | tetrahydrothiophen-3-yl | Me | Me | CH₂OMe | O |
| C-15164 | Me(S) | single bond | H | tetrahydrothiophen-3-yl | Me | Me | CH₂OMe | O |
| C-15165 | H | single bond | Me | *c*-Pen(1-CN) | Me | Cl | CH₂OMe | O |
| C-15166 | Me | single bond | H | *c*-Pen(1-CN) | Me | Cl | CH₂OMe | O |
| C-15167 | Me(R) | single bond | H | *c-*Pen(1-CN) | Me | Cl | CH₂OMe | O |
| C-15168 | Me(S) | single bond | H | *c*-Pen(1-CN) | Me | Cl | CH₂OMe | O |
| C-15169 | H | single bond | Me | *c*-Pen(1-CN) | Me | Me | CH₂OMe | O |
| C-15170 | Me | single bond | H | *c*-Pen(1-CN) | Me | Me | CH₂OMe | O |
| C-15171 | Me(R) | single bond | H | *c*-Pen(1-CN) | Me | Me | CH₂OMe | O |
| C-15172 | Me(S) | single bond | H | *c*-Pen(1-CN) | Me | Me | CH₂OMe | O |
| C-15173 | H | single bond | Me | tetrahydrofuran(3-CN)-3-yl | Me | Cl | CH₂OMe | O |
| C-15174 | Me | single bond | H | tetrahydrofuran(3-CN)-3-yl | Me | Cl | CH₂OMe | O |
| C-15175 | Me(R) | single bond | H | tetrahydrofuran(3-CN)-3-yl | Me | Cl | CH₂OMe | O |
| C-15176 | Me(S) | single bond | H | tetrahydrofuran(3-CN)-3-yl | Me | Cl | CH₂OMe | O |
| C-15177 | H | single bond | Me | tetrahydrofuran(3-CN)-3-yl | Me | Me | CH₂OMe | O |
| C-15178 | Me | single bond | H | tetrahydrofuran(3-CN)-3-yl | Me | Me | CH₂OMe | O |
| C-15179 | Me(R) | single bond | H | tetrahydrofuran(3-CN)-3-yl | Me | Me | CH₂OMe | O |
| C-15180 | Me(S) | single bond | H | tetrahydrofuran(3-CN)-3-yl | Me | Me | CH₂OMe | O |
| C-15181 | H | single bond | Me | tetrahydrothiophen(3-CN)-3-yl | Me | Cl | CH₂OMe | O |
| C-15182 | Me | single bond | H | tetrahydrothiophen(3-CN)-3-yl | Me | Cl | CH₂OMe | O |
| C-15183 | Me(R) | single bond | H | tetrahydrothiophen(3-CN)-3-yl | Me | Cl | CH₂OMe | O |
| C-15184 | Me(S) | single bond | H | tetrahydrothiophen(3-CN)-3-yl | Me | Cl | CH₂OMe | O |
| C-15185 | H | single bond | Me | tetrahydrothiophen(3-CN)-3-yl | Me | Me | CH₂OMe | O |
| C-15186 | Me | single bond | H | tetrahydrothiophen(3-CN)-3-yl | Me | Me | CH₂OMe | O |
| C-15187 | Me(R) | single bond | H | tetrahydrothiophen(3-CN)-3-yl | Me | Me | CH₂OMe | O |
| C-15188 | Me(S) | single bond | H | tetrahydrothiophen(3-CN)-3-yl | Me | Me | CH₂OMe | O |
| C-15189 | H | single bond | Me | bicyclo[1.1.1]pentan-1-yl | Me | Cl | CH₂OMe | O |
| C-15190 | Me | single bond | H | bicyclo[1.1.1]pentan-1-yl | Me | Cl | CH₂OMe | O |
| C-15191 | Me(R) | single bond | H | bicyclo[1.1.1]pentan-1-yl | Me | Cl | CH₂OMe | O |
| C-15192 | Me(S) | single bond | H | bicyclo[1.1.1]pentan-1-yl | Me | Cl | CH₂OMe | O |
| C-15193 | H | single bond | Me | bicyclo[1.1.1]pentan-1-yl | Me | Me | CH₂OMe | O |
| C-15194 | Me | single bond | H | bicyclo[1.1.1]pentan-1-yl | Me | Me | CH₂OMe | O |
| C-15195 | Me(R) | single bond | H | bicyclo[1.1.1]pentan-1-yl | Me | Me | CH₂OMe | O |
| C-15196 | Me(S) | single bond | H | bicyclo[1.1.1]pentan-1-yl | Me | Me | CH₂OMe | O |
| C-15197 | H | single bond | Me | *c*-Hex | Me | Cl | CH₂OMe | O |
| C-15198 | Me | single bond | H | *c*-Hex | Me | Cl | CH₂OMe | O |

**[Table 574]**

| Compound Number | Y | W | Z | R¹ | R² | R³ | R¹⁰ | A |
|---|---|---|---|---|---|---|---|---|
| C-15199 | Me(R) | single bond | H | *c*-Hex | Me | Cl | CH₂OMe | O |
| C-15200 | Me(S) | single bond | H | *c*-Hex | Me | Cl | CH₂OMe | O |
| C-15201 | H | single bond | Me | *c*-Hex | Me | Me | CH₂OMe | O |
| C-15202 | Me | single bond | H | *c*-Hex | Me | Me | CH₂OMe | O |
| C-15203 | Me(R) | single bond | H | *c*-Hex | Me | Me | CH₂OMe | O |
| C-15204 | Me(S) | single bond | H | *c*-Hex | Me | Me | CH₂OMe | O |
| C-15205 | H | single bond | Me | tetrahydropyran-4-yl | Me | Cl | CH₂OMe | O |
| C-15206 | Me | single bond | H | tetrahydropyran-4-yl | Me | Cl | CH₂OMe | O |
| C-15207 | Me(R) | single bond | H | tetrahydropyran-4-yl | Me | Cl | CH₂OMe | O |
| C-15208 | Me(S) | single bond | H | tetrahydropyran-4-yl | Me | Cl | CH₂OMe | O |
| C-15209 | H | single bond | Me | tetrahydropyran-4-yl | Me | Me | CH₂OMe | O |
| C-15210 | Me | single bond | H | tetrahydropyran-4-yl | Me | Me | CH₂OMe | O |
| C-15211 | Me(R) | single bond | H | tetrahydropyran-4-yl | Me | Me | CH₂OMe | O |
| C-15212 | Me(S) | single bond | H | tetrahydropyran-4-yl | Me | Me | CH₂OMe | O |
| C-15213 | H | single bond | Me | tetrahydrothiopyran-4-yl | Me | Cl | CH₂OMe | O |
| C-15214 | Me | single bond | H | tetrahydrothiopyran-4-yl | Me | Cl | CH₂OMe | O |
| C-15215 | Me(R) | single bond | H | tetrahydrothiopyran-4-yl | Me | Cl | CH₂OMe | O |
| C-15216 | Me(S) | single bond | H | tetrahydrothiopyran-4-yl | Me | Cl | CH₂OMe | O |
| C-15217 | H | single bond | Me | tetrahydrothiopyran-4-yl | Me | Me | CH₂OMe | O |
| C-15218 | Me | single bond | H | tetrahydrothiopyran-4-yl | Me | Me | CH₂OMe | O |
| C-15219 | Me(R) | single bond | H | tetrahydrothiopyran-4-yl | Me | Me | CH₂OMe | O |
| C-15220 | Me(S) | single bond | H | tetrahydrothiopyran-4-yl | Me | Me | CH₂OMe | O |
| C-15221 | H | single bond | Me | *c*-Hex(1-CN) | Me | Cl | CH₂OMe | O |
| C-15222 | Me | single bond | H | *c*-Hex(1-CN) | Me | Cl | CH₂OMe | O |
| C-15223 | Me(R) | single bond | H | *c*-Hex(1-CN) | Me | Cl | CH₂OMe | O |
| C-15224 | Me(S) | single bond | H | *c*-Hex(1-CN) | Me | Cl | CH₂OMe | O |
| C-15225 | H | single bond | Me | *c*-Hex(1-CN) | Me | Me | CH₂OMe | O |
| C-15226 | Me | single bond | H | *c*-Hex(1-CN) | Me | Me | CH₂OMe | O |
| C-15227 | Me(R) | single bond | H | *c*-Hex(1-CN) | Me | Me | CH₂OMe | O |
| C-15228 | Me(S) | single bond | H | *c*-Hex(1-CN) | Me | Me | CH₂OMe | O |
| C-15229 | H | single bond | Me | tetrahydropyran(4-CN)-4-yl | Me | Cl | CH₂OMe | O |
| C-15230 | Me | single bond | H | tetrahydropyran(4-CN)-4-yl | Me | Cl | CH₂OMe | O |
| C-15231 | Me(R) | single bond | H | tetrahydropyran(4-CN)-4-yl | Me | Cl | CH₂OMe | O |
| C-15232 | Me(S) | single bond | H | tetrahydropyran(4-CN)-4-yl | Me | Cl | CH₂OMe | O |
| C-15233 | H | single bond | Me | tetrahydropyran(4-CN)-4-yl | Me | Me | CH₂OMe | O |
| C-15234 | Me | single bond | H | tetrahydropyran(4-CN)-4-yl | Me | Me | CH₂OMe | O |
| C-15235 | Me(R) | single bond | H | tetrahydropyran(4-CN)-4-yl | Me | Me | CH₂OMe | O |
| C-15236 | Me(S) | single bond | H | tetrahydropyran(4-CN)-4-yl | Me | Me | CH₂OMe | O |
| C-15237 | H | single bond | Me | tetrahydrothiopyran(4-CN)-4-yl | Me | Cl | CH₂OMe | O |
| C-15238 | Me | single bond | H | tetrahydrothiopyran(4-CN)-4-yl | Me | Cl | CH₂OMe | O |
| C-15239 | Me(R) | single bond | H | tetrahydrothiopyran(4-CN)-4-yl | Me | Cl | CH₂OMe | O |
| C-15240 | Me(S) | single bond | H | tetrahydrothiopyran(4-CN)-4-yl | Me | Cl | CH₂OMe | O |
| C-15241 | H | single bond | Me | tetrahydrothiopyran(4-CN)-4-yl | Me | Me | CH₂OMe | O |
| C-15242 | Me | single bond | H | tetrahydrothiopyran(4-CN)-4-yl | Me | Me | CH₂OMe | O |

**[Table 575]**

| Compound Number | Y | W | Z | R¹ | R² | R³ | R¹⁰ | A |
|---|---|---|---|---|---|---|---|---|
| C-15243 | Me(R) | single bond | H | tetrahydrothiopyran(4-CN)-4-yl | Me | Me | CH₂OMe | O |
| C-15244 | Me(S) | single bond | H | tetrahydrothiopyran(4-CN)-4-yl | Me | Me | CH₂OMe | O |
| C-15245 | H | single bond | Me | CH₂(*c*-Pr) | Me | Cl | CH₂OMe | O |
| C-15246 | Me | single bond | H | CH₂(*c*-Pr) | Me | Cl | CH₂OMe | O |
| C-15247 | Me(R) | single bond | H | CH₂(*c*-Pr) | Me | Cl | CH₂OMe | O |
| C-15248 | Me(S) | single bond | H | CH₂(*c*-Pr) | Me | Cl | CH₂OMe | O |
| C-15249 | H | single bond | Me | CH₂(*c*-Pr) | Me | Me | CH₂OMe | O |
| C-15250 | Me | single bond | H | CH₂(*c*-Pr) | Me | Me | CH₂OMe | O |
| C-15251 | Me(R) | single bond | H | CH₂(*c*-Pr) | Me | Me | CH₂OMe | O |
| C-15252 | Me(S) | single bond | H | CH₂(*c*-Pr) | Me | Me | CH₂OMe | O |
| C-15253 | H | single bond | Me | CH₂[*c*-Pr(1-CN)] | Me | Cl | CH₂OMe | O |
| C-15254 | Me | single bond | H | CH₂[*c*-Pr(1-CN)] | Me | Cl | CH₂OMe | O |
| C-15255 | Me(R) | single bond | H | CH₂[*c*-Pr(1-CN)] | Me | Cl | CH₂OMe | O |
| C-15256 | Me(S) | single bond | H | CH₂[*c*-Pr(1-CN)] | Me | Cl | CH₂OMe | O |
| C-15257 | H | single bond | Me | CH₂[*c*-Pr(1-CN)] | Me | Me | CH₂OMe | O |
| C-15258 | Me | single bond | H | CH₂[*c*-Pr(1-CN)] | Me | Me | CH₂OMe | O |
| C-15259 | Me(R) | single bond | H | CH₂[*c*-Pr(1-CN)] | Me | Me | CH₂OMe | O |
| C-15260 | Me(S) | single bond | H | CH₂[*c*-Pr(1-CN)] | Me | Me | CH₂OMe | O |
| C-15261 | H | single bond | Me | CH(Me)(*c*-Pr) | Me | Cl | CH₂OMe | O |
| C-15262 | Me | single bond | H | CH(Me)(*c*-Pr) | Me | Cl | CH₂OMe | O |
| C-15263 | Me(R) | single bond | H | CH(Me)(*c*-Pr) | Me | Cl | CH₂OMe | O |
| C-15264 | Me(S) | single bond | H | CH(Me)(*c*-Pr) | Me | Cl | CH₂OMe | O |
| C-15265 | H | single bond | Me | CH(Me)(*c*-Pr) | Me | Me | CH₂OMe | O |
| C-15266 | Me | single bond | H | CH(Me)(*c*-Pr) | Me | Me | CH₂OMe | O |
| C-15267 | Me(R) | single bond | H | CH(Me)(*c*-Pr) | Me | Me | CH₂OMe | O |
| C-15268 | Me(S) | single bond | H | CH(Me)(*c*-Pr) | Me | Me | CH₂OMe | O |
| C-15269 | H | single bond | Me | CH(Me)CH₂OMe | Me | Cl | CH₂OMe | O |
| C-15270 | Me | single bond | H | CH(Me)CH₂OMe | Me | Cl | CH₂OMe | O |
| C-15271 | Me(R) | single bond | H | CH(Me)CH₂OMe | Me | Cl | CH₂OMe | O |
| C-15272 | Me(S) | single bond | H | CH(Me)CH₂OMe | Me | Cl | CH₂OMe | O |
| C-15273 | H | single bond | Me | CH(Me)CH₂OMe | Me | Me | CH₂OMe | O |
| C-15274 | Me | single bond | H | CH(Me)CH₂OMe | Me | Me | CH₂OMe | O |
| C-15275 | Me(R) | single bond | H | CH(Me)CH₂OMe | Me | Me | CH₂OMe | O |
| C-15276 | Me(S) | single bond | H | CH(Me)CH₂OMe | Me | Me | CH₂OMe | O |
| C-15277 | H | single bond | Me | CH₂CH=CH₂ | Me | Cl | CH₂OMe | O |
| C-15278 | Me | single bond | H | CH₂CH=CH₂ | Me | Cl | CH₂OMe | O |
| C-15279 | Me(R) | single bond | H | CH₂CH=CH₂ | Me | Cl | CH₂OMe | O |
| C-15280 | Me(S) | single bond | H | CH₂CH=CH₂ | Me | Cl | CH₂OMe | O |
| C-15281 | H | single bond | Me | CH₂CH=CH₂ | Me | Me | CH₂OMe | O |
| C-15282 | Me | single bond | H | CH₂CH=CH₂ | Me | Me | CH₂OMe | O |
| C-15283 | Me(R) | single bond | H | CH₂CH=CH₂ | Me | Me | CH₂OMe | O |
| C-15284 | Me(S) | single bond | H | CH₂CH=CH₂ | Me | Me | CH₂OMe | O |
| C-15285 | H | single bond | Me | CH₂C≡CH | Me | Cl | CH₂OMe | O |
| C-15286 | Me | single bond | H | CH₂C≡CH | Me | Cl | CH₂OMe | O |

**[Table 576]**

| Compound Number | Y | W | Z | R¹ | R² | R³ | R¹⁰ | A |
|---|---|---|---|---|---|---|---|---|
| C-15287 | Me(R) | single bond | H | CH₂C≡CH | Me | Cl | CH₂OMe | O |
| C-15288 | Me(S) | single bond | H | CH₂C≡CH | Me | Cl | CH₂OMe | O |
| C-15289 | H | single bond | Me | CH₂C≡CH | Me | Me | CH₂OMe | O |
| C-15290 | Me | single bond | H | CH₂C≡CH | Me | Me | CH₂OMe | O |
| C-15291 | Me(R) | single bond | H | CH₂C≡CH | Me | Me | CH₂OMe | O |
| C-15292 | Me(S) | single bond | H | CH₂C≡CH | Me | Me | CH₂OMe | O |
| C-15293 | H | single bond | Me | CH₂CN | Me | Cl | CH₂OMe | O |
| C-15294 | Me | single bond | H | CH₂CN | Me | Cl | CH₂OMe | O |
| C-15295 | Me(R) | single bond | H | CH₂CN | Me | Cl | CH₂OMe | O |
| C-15296 | Me(S) | single bond | H | CH₂CN | Me | Cl | CH₂OMe | O |
| C-15297 | H | single bond | Me | CH₂CN | Me | Me | CH₂OMe | O |
| C-15298 | Me | single bond | H | CH₂CN | Me | Me | CH₂OMe | O |
| C-15299 | Me(R) | single bond | H | CH₂CN | Me | Me | CH₂OMe | O |
| C-15300 | Me(S) | single bond | H | CH₂CN | Me | Me | CH₂OMe | O |
| C-15301 | H | single bond | Me | CH(Me)CN | Me | Cl | CH₂OMe | O |
| C-15302 | Me | single bond | H | CH(Me)CN | Me | Cl | CH₂OMe | O |
| C-15303 | Me(R) | single bond | H | CH(Me)CN | Me | Cl | CH₂OMe | O |
| C-15304 | Me(S) | single bond | H | CH(Me)CN | Me | Cl | CH₂OMe | O |
| C-15305 | H | single bond | Me | CH(Me)CN | Me | Me | CH₂OMe | O |
| C-15306 | Me | single bond | H | CH(Me)CN | Me | Me | CH₂OMe | O |
| C-15307 | Me(R) | single bond | H | CH(Me)CN | Me | Me | CH₂OMe | O |
| C-15308 | Me(S) | single bond | H | CH(Me)CN | Me | Me | CH₂OMe | O |
| C-15309 | H | single bond | Me | C(Me)₂CN | Me | Cl | CH₂OMe | O |
| C-15310 | Me | single bond | H | C(Me)₂CN | Me | Cl | CH₂OMe | O |
| C-15311 | Me(R) | single bond | H | C(Me)₂CN | Me | Cl | CH₂OMe | O |
| C-15312 | Me(S) | single bond | H | C(Me)₂CN | Me | Cl | CH₂OMe | O |
| C-15313 | H | single bond | Me | C(Me)₂CN | Me | Me | CH₂OMe | O |
| C-15314 | Me | single bond | H | C(Me)₂CN | Me | Me | CH₂OMe | O |
| C-15315 | Me(R) | single bond | H | C(Me)₂CN | Me | Me | CH₂OMe | O |
| C-15316 | Me(S) | single bond | H | C(Me)₂CN | Me | Me | CH₂OMe | O |
| C-15317 | H | single bond | Me | C(Me)(*i*-Pr)CN | Me | Cl | CH₂OMe | O |
| C-15318 | Me | single bond | H | C(Me)(*i*-Pr)CN | Me | Cl | CH₂OMe | O |
| C-15319 | Me(R) | single bond | H | C(Me)(*i*-Pr)CN | Me | Cl | CH₂OMe | O |
| C-15320 | Me(S) | single bond | H | C(Me)(*i*-Pr)CN | Me | Cl | CH₂OMe | O |
| C-15321 | H | single bond | Me | C(Me)(*i*-Pr)CN | Me | Me | CH₂OMe | O |
| C-15322 | Me | single bond | H | C(Me)(*i*-Pr)CN | Me | Me | CH₂OMe | O |
| C-15323 | Me(R) | single bond | H | C(Me)(*i*-Pr)CN | Me | Me | CH₂OMe | O |
| C-15324 | Me(S) | single bond | H | C(Me)(*i*-Pr)CN | Me | Me | CH₂OMe | O |
| C-15325 | H | single bond | Me | OMe | Me | Cl | CH₂OMe | O |
| C-15326 | Me | single bond | H | OMe | Me | Cl | CH₂OMe | O |
| C-15327 | Me(R) | single bond | H | OMe | Me | Cl | CH₂OMe | O |
| C-15328 | Me(S) | single bond | H | OMe | Me | Cl | CH₂OMe | O |
| C-15329 | H | single bond | Me | OMe | Me | Me | CH₂OMe | O |
| C-15330 | Me | single bond | H | OMe | Me | Me | CH₂OMe | O |

**[Table 577]**

| Compound Number | Y | W | Z | R¹ | R² | R³ | R¹⁰ | A |
|---|---|---|---|---|---|---|---|---|
| C-15331 | Me(R) | single bond | H | OMe | Me | Me | CH₂OMe | O |
| C-15332 | Me(S) | single bond | H | OMe | Me | Me | CH₂OMe | O |
| C-15333 | H | single bond | Me | CH=NOMe | Me | Cl | CH₂OMe | O |
| C-15334 | Me | single bond | H | CH=NOMe | Me | Cl | CH₂OMe | O |
| C-15335 | Me(R) | single bond | H | CH=NOMe | Me | Cl | CH₂OMe | O |
| C-15336 | Me(S) | single bond | H | CH=NOMe | Me | Cl | CH₂OMe | O |
| C-15337 | H | single bond | Me | CH=NOMe | Me | Me | CH₂OMe | O |
| C-15338 | Me | single bond | H | CH=NOMe | Me | Me | CH₂OMe | O |
| C-15339 | Me(R) | single bond | H | CH=NOMe | Me | Me | CH₂OMe | O |
| C-15340 | Me(S) | single bond | H | CH=NOMe | Me | Me | CH₂OMe | O |
| C-15341 | H | single bond | Me | morpholin-4-yl | Me | Cl | CH₂OMe | O |
| C-15342 | Me | single bond | H | morpholin-4-yl | Me | Cl | CH₂OMe | O |
| C-15343 | Me(R) | single bond | H | morpholin-4-yl | Me | Cl | CH₂OMe | O |
| C-15344 | Me(S) | single bond | H | morpholin-4-yl | Me | Cl | CH₂OMe | O |
| C-15345 | H | single bond | Me | morpholin-4-yl | Me | Me | CH₂OMe | O |
| C-15346 | Me | single bond | H | morpholin-4-yl | Me | Me | CH₂OMe | O |
| C-15347 | Me(R) | single bond | H | morpholin-4-yl | Me | Me | CH₂OMe | O |
| C-15348 | Me(S) | single bond | H | morpholin-4-yl | Me | Me | CH₂OMe | O |
| C-15349 | H | single bond | Me | Ph | Me | Cl | CH₂OMe | O |
| C-15350 | Me | single bond | H | Ph | Me | Cl | CH₂OMe | O |
| C-15351 | Me(R) | single bond | H | Ph | Me | Cl | CH₂OMe | O |
| C-15352 | Me(S) | single bond | H | Ph | Me | Cl | CH₂OMe | O |
| C-15353 | H | single bond | Me | Ph | Me | Me | CH₂OMe | O |
| C-15354 | Me | single bond | H | Ph | Me | Me | CH₂OMe | O |
| C-15355 | Me(R) | single bond | H | Ph | Me | Me | CH₂OMe | O |
| C-15356 | Me(S) | single bond | H | Ph | Me | Me | CH₂OMe | O |
| C-15357 | H | single bond | Me | CH₂Ph | Me | Cl | CH₂OMe | O |
| C-15358 | Me | single bond | H | CH₂Ph | Me | Cl | CH₂OMe | O |
| C-15359 | Me(R) | single bond | H | CH₂Ph | Me | Cl | CH₂OMe | O |
| C-15360 | Me(S) | single bond | H | CH₂Ph | Me | Cl | CH₂OMe | O |
| C-15361 | H | single bond | Me | CH₂Ph | Me | Me | CH₂OMe | O |
| C-15362 | Me | single bond | H | CH₂Ph | Me | Me | CH₂OMe | O |
| C-15363 | Me(R) | single bond | H | CH₂Ph | Me | Me | CH₂OMe | O |
| C-15364 | Me(S) | single bond | H | CH₂Ph | Me | Me | CH₂OMe | O |
| C-15365 | H | single bond | Me | thiazole-2-yl | Me | Cl | CH₂OMe | O |
| C-15366 | Me | single bond | H | thiazole-2-yl | Me | Cl | CH₂OMe | O |
| C-15367 | Me(R) | single bond | H | thiazole-2-yl | Me | Cl | CH₂OMe | O |
| C-15368 | Me(S) | single bond | H | thiazole-2-yl | Me | Cl | CH₂OMe | O |
| C-15369 | H | single bond | Me | thiazole-2-yl | Me | Me | CH₂OMe | O |
| C-15370 | Me | single bond | H | thiazole-2-yl | Me | Me | CH₂OMe | O |
| C-15371 | Me(R) | single bond | H | thiazole-2-yl | Me | Me | CH₂OMe | O |
| C-15372 | Me(S) | single bond | H | thiazole-2-yl | Me | Me | CH₂OMe | O |
| C-15373 | H | single bond | Me | 1-Me-pyrazol-3-yl | Me | Cl | CH₂OMe | O |
| C-15374 | Me | single bond | H | 1-Me-pyrazol-3-yl | Me | Cl | CH₂OMe | O |

**[Table 578]**

| Compound Number | Y | W | Z | R¹ | R² | R³ | R¹⁰ | A |
|---|---|---|---|---|---|---|---|---|
| C-15375 | Me(R) | single bond | H | 1-Me-pyrazol-3-yl | Me | Cl | CH₂OMe | O |
| C-15376 | Me(S) | single bond | H | 1-Me-pyrazol-3-yl | Me | Cl | CH₂OMe | O |
| C-15377 | H | single bond | Me | 1-Me-pyrazol-3-yl | Me | Me | CH₂OMe | O |
| C-15378 | Me | single bond | H | 1-Me-pyrazol-3-yl | Me | Me | CH₂OMe | O |
| C-15379 | Me(R) | single bond | H | 1-Me-pyrazol-3-yl | Me | Me | CH₂OMe | O |
| C-15380 | Me(S) | single bond | H | 1-Me-pyrazol-3-yl | Me | Me | CH₂OMe | O |
| C-15381 | H | single bond | Me | 1-Me-pyrazol-5-yl | Me | Cl | CH₂OMe | O |
| C-15382 | Me | single bond | H | 1-Me-pyrazol-5-yl | Me | Cl | CH₂OMe | O |
| C-15383 | Me(R) | single bond | H | 1-Me-pyrazol-5-yl | Me | Cl | CH₂OMe | O |
| C-15384 | Me(S) | single bond | H | 1-Me-pyrazol-5-yl | Me | Cl | CH₂OMe | O |
| C-15385 | H | single bond | Me | 1-Me-pyrazol-5-yl | Me | Me | CH₂OMe | O |
| C-15386 | Me | single bond | H | 1-Me-pyrazol-5-yl | Me | Me | CH₂OMe | O |
| C-15387 | Me(R) | single bond | H | 1-Me-pyrazol-5-yl | Me | Me | CH₂OMe | O |
| C-15388 | Me(S) | single bond | H | 1-Me-pyrazol-5-yl | Me | Me | CH₂OMe | O |
| C-15389 | H | single bond | Me | CH₂(pyrimidin-2-yl) | Me | Cl | CH₂OMe | O |
| C-15390 | Me | single bond | H | CH₂(pyrimidin-2-yl) | Me | Cl | CH₂OMe | O |
| C-15391 | Me(R) | single bond | H | CH₂(pyrimidin-2-yl) | Me | Cl | CH₂OMe | O |
| C-15392 | Me(S) | single bond | H | CH₂(pyrimidin-2-yl) | Me | Cl | CH₂OMe | O |
| C-15393 | H | single bond | Me | CH₂(pyrimidin-2-yl) | Me | Me | CH₂OMe | O |
| C-15394 | Me | single bond | H | CH₂(pyrimidin-2-yl) | Me | Me | CH₂OMe | O |
| C-15395 | Me(R) | single bond | H | CH₂(pyrimidin-2-yl) | Me | Me | CH₂OMe | O |
| C-15396 | Me(S) | single bond | H | CH₂(pyrimidin-2-yl) | Me | Me | CH₂OMe | O |
| C-15397 | H | single bond | Me | Me | Me | Cl | C(=O)Me | O |
| C-15398 | Me | single bond | H | Me | Me | Cl | C(=O)Me | O |
| C-15399 | Me(R) | single bond | H | Me | Me | Cl | C(=O)Me | O |
| C-15400 | Me(S) | single bond | H | Me | Me | Cl | C(=O)Me | O |
| C-15401 | H | single bond | Me | Me | Me | Me | C(=O)Me | O |
| C-15402 | Me | single bond | H | Me | Me | Me | C(=O)Me | O |
| C-15403 | Me(R) | single bond | H | Me | Me | Me | C(=O)Me | O |
| C-15404 | Me(S) | single bond | H | Me | Me | Me | C(=O)Me | O |
| C-15405 | H | single bond | Me | CH₂CHF₂ | Me | Cl | C(=O)Me | O |
| C-15406 | Me | single bond | H | CH₂CHF₂ | Me | Cl | C(=O)Me | O |
| C-15407 | Me(R) | single bond | H | CH₂CHF₂ | Me | Cl | C(=O)Me | O |
| C-15408 | Me(S) | single bond | H | CH₂CHF₂ | Me | Cl | C(=O)Me | O |
| C-15409 | H | single bond | Me | CH₂CHF₂ | Me | Me | C(=O)Me | O |
| C-15410 | Me | single bond | H | CH₂CHF₂ | Me | Me | C(=O)Me | O |
| C-15411 | Me(R) | single bond | H | CH₂CHF₂ | Me | Me | C(=O)Me | O |
| C-15412 | Me(S) | single bond | H | CH₂CHF₂ | Me | Me | C(=O)Me | O |
| C-15413 | H | single bond | Me | *n*-Pr | Me | Cl | C(=O)Me | O |
| C-15414 | Me | single bond | H | *n*-Pr | Me | Cl | C(=O)Me | O |
| C-15415 | Me(R) | single bond | H | *n*-Pr | Me | Cl | C(=O)Me | O |
| C-15416 | Me(S) | single bond | H | *n*-Pr | Me | Cl | C(=O)Me | O |
| C-15417 | H | single bond | Me | *n*-Pr | Me | Me | C(=O)Me | O |
| C-15418 | Me | single bond | H | *n*-Pr | Me | Me | C(=O)Me | O |

**[Table 579]**

| Compound Number | Y | W | Z | R¹ | R² | R³ | R¹⁰ | A |
|---|---|---|---|---|---|---|---|---|
| C-15419 | Me(R) | single bond | H | *n* -Pr | Me | Me | C(=O)Me | O |
| C-15420 | Me(S) | single bond | H | *n* -Pr | Me | Me | C(=O)Me | O |
| C-15421 | H | single bond | Me | *i* -Pr | Me | Cl | C(=O)Me | O |
| C-15422 | Me | single bond | H | *i* -Pr | Me | Cl | C(=O)Me | O |
| C-15423 | Me(R) | single bond | H | *i* -Pr | Me | Cl | C(=O)Me | O |
| C-15424 | Me(S) | single bond | H | *i* -Pr | Me | Cl | C(=O)Me | O |
| C-15425 | H | single bond | Me | *i* -Pr | Me | Me | C(=O)Me | O |
| C-15426 | Me | single bond | H | *i* -Pr | Me | Me | C(=O)Me | O |
| C-15427 | Me(R) | single bond | H | *i* -Pr | Me | Me | C(=O)Me | O |
| C-15428 | Me(S) | single bond | H | *i* -Pr | Me | Me | C(=O)Me | O |
| C-15429 | H | single bond | Me | *c* -Pr(1-F) | Me | Cl | C(=O)Me | O |
| C-15430 | Me | single bond | H | *c* -Pr(1-F) | Me | Cl | C(=O)Me | O |
| C-15431 | Me(R) | single bond | H | *c* -Pr(1-F) | Me | Cl | C(=O)Me | O |
| C-15432 | Me(S) | single bond | H | *c* -Pr(1-F) | Me | Cl | C(=O)Me | O |
| C-15433 | H | single bond | Me | *c* -Pr(1-F) | Me | Me | C(=O)Me | O |
| C-15434 | Me | single bond | H | *c* -Pr(1-F) | Me | Me | C(=O)Me | O |
| C-15435 | Me(R) | single bond | H | *c* -Pr(1-F) | Me | Me | C(=O)Me | O |
| C-15436 | Me(S) | single bond | H | *c* -Pr(1-F) | Me | Me | C(=O)Me | O |
| C-15437 | H | single bond | Me | *c* -Pr(2,2-F₂) | Me | Cl | C(=O)Me | O |
| C-15438 | Me | single bond | H | *c* -Pr(2,2-F₂) | Me | Cl | C(=O)Me | O |
| C-15439 | Me(R) | single bond | H | *c* -Pr(2,2-F₂) | Me | Cl | C(=O)Me | O |
| C-15440 | Me(S) | single bond | H | *c* -Pr(2,2-F₂) | Me | Cl | C(=O)Me | O |
| C-15441 | H | single bond | Me | *c* -Pr(2,2-F₂) | Me | Me | C(=O)Me | O |
| C-15442 | Me | single bond | H | *c* -Pr(2,2-F₂) | Me | Me | C(=O)Me | O |
| C-15443 | Me(R) | single bond | H | *c* -Pr(2,2-F₂) | Me | Me | C(=O)Me | O |
| C-15444 | Me(S) | single bond | H | *c* -Pr(2,2-F₂) | Me | Me | C(=O)Me | O |
| C-15445 | H | single bond | Me | *c* -Pr(1-Cl) | Me | Cl | C(=O)Me | O |
| C-15446 | Me | single bond | H | *c* -Pr(1-Cl) | Me | Cl | C(=O)Me | O |
| C-15447 | Me(R) | single bond | H | *c* -Pr(1-Cl) | Me | Cl | C(=O)Me | O |
| C-15448 | Me(S) | single bond | H | *c* -Pr(1-Cl) | Me | Cl | C(=O)Me | O |
| C-15449 | H | single bond | Me | *c* -Pr(1-Cl) | Me | Me | C(=O)Me | O |
| C-15450 | Me | single bond | H | *c* -Pr(1-Cl) | Me | Me | C(=O)Me | O |
| C-15451 | Me(R) | single bond | H | *c* -Pr(1-Cl) | Me | Me | C(=O)Me | O |
| C-15452 | Me(S) | single bond | H | *c* -Pr(1-Cl) | Me | Me | C(=O)Me | O |
| C-15453 | H | single bond | Me | *c* -Pr(1-Br) | Me | Cl | C(=O)Me | O |
| C-15454 | Me | single bond | H | *c* -Pr(1-Br) | Me | Cl | C(=O)Me | O |
| C-15455 | Me(R) | single bond | H | *c* -Pr(1-Br) | Me | Cl | C(=O)Me | O |
| C-15456 | Me(S) | single bond | H | *c* -Pr(1-Br) | Me | Cl | C(=O)Me | O |
| C-15457 | H | single bond | Me | *c* -Pr(1-Br) | Me | Me | C(=O)Me | O |
| C-15458 | Me | single bond | H | *c* -Pr(1-Br) | Me | Me | C(=O)Me | O |
| C-15459 | Me(R) | single bond | H | *c* -Pr(1-Br) | Me | Me | C(=O)Me | O |
| C-15460 | Me(S) | single bond | H | *c* -Pr(1-Br) | Me | Me | C(=O)Me | O |
| C-15461 | H | single bond | Me | *c* -Pr(1-Me) | Me | Cl | C(=O)Me | O |
| C-15462 | Me | single bond | H | *c* -Pr(1-Me) | Me | Cl | C(=O)Me | O |

**[Table 580]**

| Compound Number | Y | W | Z | R¹ | R² | R³ | R¹⁰ | A |
|---|---|---|---|---|---|---|---|---|
| C-15463 | Me(R) | single bond | H | *c* -Pr(1-Me) | Me | Cl | C(=O)Me | O |
| C-15464 | Me(S) | single bond | H | *c* -Pr(1-Me) | Me | Cl | C(=O)Me | O |
| C-15465 | H | single bond | Me | *c* -Pr(1-Me) | Me | Me | C(=O)Me | O |
| C-15466 | Me | single bond | H | *c* -Pr(1-Me) | Me | Me | C(=O)Me | O |
| C-15467 | Me(R) | single bond | H | *c* -Pr(1-Me) | Me | Me | C(=O)Me | O |
| C-15468 | Me(S) | single bond | H | *c* -Pr(1-Me) | Me | Me | C(=O)Me | O |
| C-15469 | H | single bond | Me | *c* -Pr(1-CH₂OH) | Me | Cl | C(=O)Me | O |
| C-15470 | Me | single bond | H | *c* -Pr(1-CH₂OH) | Me | Cl | C(=O)Me | O |
| C-15471 | Me(R) | single bond | H | *c* -Pr(1-CH₂OH) | Me | Cl | C(=O)Me | O |
| C-15472 | Me(S) | single bond | H | *c* -Pr(1-CH₂OH) | Me | Cl | C(=O)Me | O |
| C-15473 | H | single bond | Me | *c* -Pr(1-CH₂OH) | Me | Me | C(=O)Me | O |
| C-15474 | Me | single bond | H | *c* -Pr(1-CH₂OH) | Me | Me | C(=O)Me | O |
| C-15475 | Me(R) | single bond | H | *c* -Pr(1-CH₂OH) | Me | Me | C(=O)Me | O |
| C-15476 | Me(S) | single bond | H | *c* -Pr(1-CH₂OH) | Me | Me | C(=O)Me | O |
| C-15477 | H | single bond | Me | *c* -Pr[1-CH₂OS(=O)₂Me] | Me | Cl | C(=O)Me | O |
| C-15478 | Me | single bond | H | *c* -Pr[1-CH₂OS(=O)₂Me] | Me | Cl | C(=O)Me | O |
| C-15479 | Me(R) | single bond | H | *c* -Pr[1-CH₂OS(=O)₂Me] | Me | Cl | C(=O)Me | O |
| C-15480 | Me(S) | single bond | H | *c* -Pr[1-CH₂OS(=O)₂Me] | Me | Cl | C(=O)Me | O |
| C-15481 | H | single bond | Me | *c* -Pr[1-CH₂OS(=O)₂Me] | Me | Me | C(=O)Me | O |
| C-15482 | Me | single bond | H | *c* -Pr[1-CH₂OS(=O)₂Me] | Me | Me | C(=O)Me | O |
| C-15483 | Me(R) | single bond | H | *c* -Pr[1-CH₂OS(=O)₂Me] | Me | Me | C(=O)Me | O |
| C-15484 | Me(S) | single bond | H | *c* -Pr[1-CH₂OS(=O)₂Me] | Me | Me | C(=O)Me | O |
| C-15485 | H | single bond | Me | *c* -Pr(1-CH₂OMe) | Me | Cl | C(=O)Me | O |
| C-15486 | Me | single bond | H | *c* -Pr(1-CH₂OMe) | Me | Cl | C(=O)Me | O |
| C-15487 | Me(R) | single bond | H | *c* -Pr(1-CH₂OMe) | Me | Cl | C(=O)Me | O |
| C-15488 | Me(S) | single bond | H | *c* -Pr(1-CH₂OMe) | Me | Cl | C(=O)Me | O |
| C-15489 | H | single bond | Me | *c* -Pr(1-CH₂OMe) | Me | Me | C(=O)Me | O |
| C-15490 | Me | single bond | H | *c* -Pr(1-CH₂OMe) | Me | Me | C(=O)Me | O |
| C-15491 | Me(R) | single bond | H | *c* -Pr(1-CH₂OMe) | Me | Me | C(=O)Me | O |
| C-15492 | Me(S) | single bond | H | *c* -Pr(1-CH₂OMe) | Me | Me | C(=O)Me | O |
| C-15493 | H | single bond | Me | *c* -Pr(1-CH₂CN) | Me | Cl | C(=O)Me | O |
| C-15494 | Me | single bond | H | *c* -Pr(1-CH₂CN) | Me | Cl | C(=O)Me | O |
| C-15495 | Me(R) | single bond | H | *c* -Pr(1-CH₂CN) | Me | Cl | C(=O)Me | O |
| C-15496 | Me(S) | single bond | H | *c* -Pr(1-CH₂CN) | Me | Cl | C(=O)Me | O |
| C-15497 | H | single bond | Me | *c* -Pr(1-CH₂CN) | Me | Me | C(=O)Me | O |
| C-15498 | Me | single bond | H | *c* -Pr(1-CH₂CN) | Me | Me | C(=O)Me | O |
| C-15499 | Me(R) | single bond | H | *c* -Pr(1-CH₂CN) | Me | Me | C(=O)Me | O |
| C-15500 | Me(S) | single bond | H | *c* -Pr(1-CH₂CN) | Me | Me | C(=O)Me | O |
| C-15501 | H | single bond | Me | *c* -Pr(1-CH₂SMe) | Me | Cl | C(=O)Me | O |
| C-15502 | Me | single bond | H | *c* -Pr(1-CH₂SMe) | Me | Cl | C(=O)Me | O |
| C-15503 | Me(R) | single bond | H | *c* -Pr(1-CH₂SMe) | Me | Cl | C(=O)Me | O |
| C-15504 | Me(S) | single bond | H | *c* -Pr(1-CH₂SMe) | Me | Cl | C(=O)Me | O |
| C-15505 | H | single bond | Me | *c* -Pr(1-CH₂SMe) | Me | Me | C(=O)Me | O |
| C-15506 | Me | single bond | H | *c* -Pr(1-CH₂SMe) | Me | Me | C(=O)Me | O |

**[Table 581]**

| Compound Number | Y | W | Z | R¹ | R² | R³ | R¹⁰ | A |
|---|---|---|---|---|---|---|---|---|
| C-15507 | Me(R) | single bond | H | *c* -Pr(1-CH₂SMe) | Me | Me | C(=O)Me | O |
| C-15508 | Me(S) | single bond | H | *c* -Pr(1-CH₂SMe) | Me | Me | C(=O)Me | O |
| C-15509 | H | single bond | Me | *c* -Pr[1-CH₂S(=O)Me] | Me | Cl | C(=O)Me | O |
| C-15510 | Me | single bond | H | *c* -Pr[1-CH₂S(=O)Me] | Me | Cl | C(=O)Me | O |
| C-15511 | Me(R) | single bond | H | *c* -Pr[1-CH₂S(=O)Me] | Me | Cl | C(=O)Me | O |
| C-15512 | Me(S) | single bond | H | *c* -Pr[1-CH₂S(=O)Me] | Me | Cl | C(=O)Me | O |
| C-15513 | H | single bond | Me | *c* -Pr[1-CH₂S(=O)Me] | Me | Me | C(=O)Me | O |
| C-15514 | Me | single bond | H | *c* -Pr[1-CH₂S(=O)Me] | Me | Me | C(=O)Me | O |
| C-15515 | Me(R) | single bond | H | *c* -Pr[1-CH₂S(=O)Me] | Me | Me | C(=O)Me | O |
| C-15516 | Me(S) | single bond | H | *c* -Pr[1-CH₂S(=O)Me] | Me | Me | C(=O)Me | O |
| C-15517 | H | single bond | Me | *c* -Pr[1-CH₂S(=O)₂Me] | Me | Cl | C(=O)Me | O |
| C-15518 | Me | single bond | H | *c* -Pr[1-CH₂S(=O)₂Me] | Me | Cl | C(=O)Me | O |
| C-15519 | Me(R) | single bond | H | *c* -Pr[1-CH₂S(=O)₂Me] | Me | Cl | C(=O)Me | O |
| C-15520 | Me(S) | single bond | H | *c* -Pr[1-CH₂S(=O)₂Me] | Me | Cl | C(=O)Me | O |
| C-15521 | H | single bond | Me | *c* -Pr[1-CH₂S(=O)₂Me] | Me | Me | C(=O)Me | O |
| C-15522 | Me | single bond | H | *c* -Pr[1-CH₂S(=O)₂Me] | Me | Me | C(=O)Me | O |
| C-15523 | Me(R) | single bond | H | *c* -Pr[1-CH₂S(=O)₂Me] | Me | Me | C(=O)Me | O |
| C-15524 | Me(S) | single bond | H | *c* -Pr[1-CH₂S(=O)₂Me] | Me | Me | C(=O)Me | O |
| C-15525 | H | single bond | Me | *c* -Pr(1-CH₂F) | Me | Cl | C(=O)Me | O |
| C-15526 | Me | single bond | H | *c* -Pr(1-CH₂F) | Me | Cl | C(=O)Me | O |
| C-15527 | Me(R) | single bond | H | *c* -Pr(1-CH₂F) | Me | Cl | C(=O)Me | O |
| C-15528 | Me(S) | single bond | H | *c* -Pr(1-CH₂F) | Me | Cl | C(=O)Me | O |
| C-15529 | H | single bond | Me | *c* -Pr(1-CH₂F) | Me | Me | C(=O)Me | O |
| C-15530 | Me | single bond | H | *c* -Pr(1-CH₂F) | Me | Me | C(=O)Me | O |
| C-15531 | Me(R) | single bond | H | *c* -Pr(1-CH₂F) | Me | Me | C(=O)Me | O |
| C-15532 | Me(S) | single bond | H | *c* -Pr(1-CH₂F) | Me | Me | C(=O)Me | O |
| C-15533 | H | single bond | Me | *c* -Pr(1-CHF₂) | Me | Cl | C(=O)Me | O |
| C-15534 | Me | single bond | H | *c* -Pr(1-CHF₂) | Me | Cl | C(=O)Me | O |
| C-15535 | Me(R) | single bond | H | *c* -Pr(1-CHF₂) | Me | Cl | C(=O)Me | O |
| C-15536 | Me(S) | single bond | H | *c* -Pr(1-CHF₂) | Me | Cl | C(=O)Me | O |
| C-15537 | H | single bond | Me | *c* -Pr(1-CHF₂) | Me | Me | C(=O)Me | O |
| C-15538 | Me | single bond | H | *c* -Pr(1-CHF₂) | Me | Me | C(=O)Me | O |
| C-15539 | Me(R) | single bond | H | *c* -Pr(1-CHF₂) | Me | Me | C(=O)Me | O |
| C-15540 | Me(S) | single bond | H | *c* -Pr(1-CHF₂) | Me | Me | C(=O)Me | O |
| C-15541 | H | single bond | Me | *c* -Pr(1-CF₃) | Me | Cl | C(=O)Me | O |
| C-15542 | Me | single bond | H | *c* -Pr(1-CF₃) | Me | Cl | C(=O)Me | O |
| C-15543 | Me(R) | single bond | H | *c* -Pr(1-CF₃) | Me | Cl | C(=O)Me | O |
| C-15544 | Me(S) | single bond | H | *c* -Pr(1-CF₃) | Me | Cl | C(=O)Me | O |
| C-15545 | H | single bond | Me | *c* -Pr(1-CF₃) | Me | Me | C(=O)Me | O |
| C-15546 | Me | single bond | H | *c* -Pr(1-CF₃) | Me | Me | C(=O)Me | O |
| C-15547 | Me(R) | single bond | H | *c* -Pr(1-CF₃) | Me | Me | C(=O)Me | O |
| C-15548 | Me(S) | single bond | H | *c* -Pr(1-CF₃) | Me | Me | C(=O)Me | O |
| C-15549 | H | single bond | Me | *c* -Pr(1-C≡CH) | Me | Cl | C(=O)Me | O |
| C-15550 | Me | single bond | H | *c* -Pr(1-C≡CH) | Me | Cl | C(=O)Me | O |

**[Table 582]**

| Compound Number | Y | W | Z | R¹ | R² | R³ | R¹⁰ | A |
|---|---|---|---|---|---|---|---|---|
| C-15551 | Me(R) | single bond | H | *c* -pr(1-C≡CH) | Me | Cl | C(=O)Me | O |
| C-15552 | Me(S) | single bond | H | *c* -pr(1-C≡CH) | Me | Cl | C(=O)Me | O |
| C-15553 | H | single bond | Me | *c* -Pr(1-C≡CH) | Me | Me | C(=O)Me | O |
| C-15554 | Me | single bond | H | *c* -Pr(1-C≡CH) | Me | Me | C(=O)Me | O |
| C-15555 | Me(R) | single bond | H | *c* -Pr(1-C≡CH) | Me | Me | C(=O)Me | O |
| C-15556 | Me(S) | single bond | H | *c* -Pr(1-C≡CH) | Me | Me | C(=O)Me | O |
| C-15557 | H | single bond | Me | *c* -Pr[1-C(=O)OEt] | Me | Cl | C(=O)Me | O |
| C-15558 | Me | single bond | H | *c* -Pr[1-C(=O)OEt] | Me | Cl | C(=O)Me | O |
| C-15559 | Me(R) | single bond | H | *c* -Pr[1-C(=O)OEt] | Me | Cl | C(=O)Me | O |
| C-15560 | Me(S) | single bond | H | *c* -Pr[1-C(=O)OEt] | Me | Cl | C(=O)Me | O |
| C-15561 | H | single bond | Me | *c* -Pr[1-C(=O)OEt] | Me | Me | C(=O)Me | O |
| C-15562 | Me | single bond | H | *c* -Pr[1-C(=O)OEt] | Me | Me | C(=O)Me | O |
| C-15563 | Me(R) | single bond | H | *c* -Pr[1-C(=O)OEt] | Me | Me | C(=O)Me | O |
| C-15564 | Me(S) | single bond | H | *c* -Pr[1-C(=O)OEt] | Me | Me | C(=O)Me | O |
| C-15565 | H | single bond | Me | *c* -Pr[1-C(=O)OH] | Me | Cl | C(=O)Me | O |
| C-15566 | Me | single bond | H | *c* -Pr[1-C(=O)OH] | Me | Cl | C(=O)Me | O |
| C-15567 | Me(R) | single bond | H | *c* -Pr[1-C(=O)OH] | Me | Cl | C(=O)Me | O |
| C-15568 | Me(S) | single bond | H | *c* -Pr[1-C(=O)OH] | Me | Cl | C(=O)Me | O |
| C-15569 | H | single bond | Me | *c* -Pr[1-C(=O)OH] | Me | Me | C(=O)Me | O |
| C-15570 | Me | single bond | H | *c* -Pr[1-C(=O)OH] | Me | Me | C(=O)Me | O |
| C-15571 | Me(R) | single bond | H | *c* -Pr[1-C(=O)OH] | Me | Me | C(=O)Me | O |
| C-15572 | Me(S) | single bond | H | *c* -Pr[1-C(=O)OH] | Me | Me | C(=O)Me | O |
| C-15573 | H | single bond | Me | *c* -Pr[1-C(=O)NH₂] | Me | Cl | C(=O)Me | O |
| C-15574 | Me | single bond | H | *c* -Pr[1-C(=O)NH₂] | Me | Cl | C(=O)Me | O |
| C-15575 | Me(R) | single bond | H | *c* -Pr[1-C(=O)NH₂] | Me | Cl | C(=O)Me | O |
| C-15576 | Me(S) | single bond | H | *c* -Pr[1-C(=O)NH₂] | Me | Cl | C(=O)Me | O |
| C-15577 | H | single bond | Me | *c* -Pr[1-C(=O)NH₂] | Me | Me | C(=O)Me | O |
| C-15578 | Me | single bond | H | *c* -Pr[1-C(=O)NH₂] | Me | Me | C(=O)Me | O |
| C-15579 | Me(R) | single bond | H | *c* -Pr[1-C(=O)NH₂] | Me | Me | C(=O)Me | O |
| C-15580 | Me(S) | single bond | H | *c* -Pr[1-C(=O)NH₂] | Me | Me | C(=O)Me | O |
| C-15581 | H | single bond | Me | *c* -Pr(1-OMe) | Me | Cl | C(=O)Me | O |
| C-15582 | Me | single bond | H | *c* -Pr(1-OMe) | Me | Cl | C(=O)Me | O |
| C-15583 | Me(R) | single bond | H | *c* -Pr(1-OMe) | Me | Cl | C(=O)Me | O |
| C-15584 | Me(S) | single bond | H | *c* -Pr(1-OMe) | Me | Cl | C(=O)Me | O |
| C-15585 | H | single bond | Me | *c* -Pr(1-OMe) | Me | Me | C(=O)Me | O |
| C-15586 | Me | single bond | H | *c* -Pr(1-OMe) | Me | Me | C(=O)Me | O |
| C-15587 | Me(R) | single bond | H | *c* -Pr(1-OMe) | Me | Me | C(=O)Me | O |
| C-15588 | Me(S) | single bond | H | *c* -Pr(1-OMe) | Me | Me | C(=O)Me | O |
| C-15589 | H | single bond | Me | *c* -Pr(1-SMe) | Me | Cl | C(=O)Me | O |
| C-15590 | Me | single bond | H | *c* -Pr(1-SMe) | Me | Cl | C(=O)Me | O |
| C-15591 | Me(R) | single bond | H | *c* -Pr(1-SMe) | Me | Cl | C(=O)Me | O |
| C-15592 | Me(S) | single bond | H | *c* -Pr(1-SMe) | Me | Cl | C(=O)Me | O |
| C-15593 | H | single bond | Me | *c* -Pr(1-SMe) | Me | Me | C(=O)Me | O |
| C-15594 | Me | single bond | H | *c* -Pr(1-SMe) | Me | Me | C(=O)Me | O |

**[Table 583]**

| Compound Number | Y | W | Z | R¹ | R² | R³ | R¹⁰ | A |
|---|---|---|---|---|---|---|---|---|
| C-15595 | Me(R) | single bond | H | *c*-Pr(1-SMe) | Me | Me | C(=O)Me | O |
| C-15596 | Me(S) | single bond | H | *c*-Pr(1-SMe) | Me | Me | C(=O)Me | O |
| C-15597 | H | single bond | Me | *c*-Pr[1-S(=O)Me] | Me | Cl | C(=O)Me | O |
| C-15598 | Me | single bond | H | *c*-Pr[1-S(=O)Me] | Me | Cl | C(=O)Me | O |
| C-15599 | Me(R) | single bond | H | *c*-Pr[1-S(=O)Me] | Me | Cl | C(=O)Me | O |
| C-15600 | Me(S) | single bond | H | *c*-Pr[1-S(=O)Me] | Me | Cl | C(=O)Me | O |
| C-15601 | H | single bond | Me | *c*-Pr[1-S(=O)Me] | Me | Me | C(=O)Me | O |
| C-15602 | Me | single bond | H | *c*-Pr[1-S(=O)Me] | Me | Me | C(=O)Me | O |
| C-15603 | Me(R) | single bond | H | *c*-Pr[1-S(=O)Me] | Me | Me | C(=O)Me | O |
| C-15604 | Me(S) | single bond | H | *c*-Pr[1-S(=O)Me] | Me | Me | C(=O)Me | O |
| C-15605 | H | single bond | Me | *c*-Pr[1-S(=O)₂Me] | Me | Cl | C(=O)Me | O |
| C-15606 | Me | single bond | H | *c*-Pr[1-S(=O)₂Me] | Me | Cl | C(=O)Me | O |
| C-15607 | Me(R) | single bond | H | *c*-Pr[1-S(=O)₂Me] | Me | Cl | C(=O)Me | O |
| C-15608 | Me(S) | single bond | H | *c*-Pr[1-S(=O)₂Me] | Me | Cl | C(=O)Me | O |
| C-15609 | H | single bond | Me | *c*-Pr[1-S(=O)₂Me] | Me | Me | C(=O)Me | O |
| C-15610 | Me | single bond | H | *c*-Pr[1-S(=O)₂Me] | Me | Me | C(=O)Me | O |
| C-15611 | Me(R) | single bond | H | *c*-Pr[1-S(=O)₂Me] | Me | Me | C(=O)Me | O |
| C-15612 | Me(S) | single bond | H | *c*-Pr[1-S(=O)₂Me] | Me | Me | C(=O)Me | O |
| C-15613 | H | single bond | Me | *c*-Pr[1-S(=O)₂CHF₂] | Me | Cl | C(=O)Me | O |
| C-15614 | Me | single bond | H | *c-*Pr[1-S(=O)₂CHF₂] | Me | Cl | C(=O)Me | O |
| C-15615 | Me(R) | single bond | H | *c*-Pr[1-S(=O)₂CHF₂] | Me | Cl | C(=O)Me | O |
| C-15616 | Me(S) | single bond | H | *c*-Pr[1-S(=O)₂CHF₂] | Me | Cl | C(=O)Me | O |
| C-15617 | H | single bond | Me | *c*-Pr[1-S(=O)₂CHF₂] | Me | Me | C(=O)Me | O |
| C-15618 | Me | single bond | H | *c*-Pr[1-S(=O)₂CHF₂] | Me | Me | C(=O)Me | O |
| C-15619 | Me(R) | single bond | H | *c*-Pr[1-S(=O)₂CHF₂] | Me | Me | C(=O)Me | O |
| C-15620 | Me(S) | single bond | H | *c*-Pr[1-S(=O)₂CHF₂] | Me | Me | C(=O)Me | O |
| C-15621 | H | single bond | Me | *c*-Pr[1-S(=O)₂CF₃] | Me | Cl | C(=O)Me | O |
| C-15622 | Me | single bond | H | *c*-Pr[1-S(=O)₂CF₃] | Me | Cl | C(=O)Me | O |
| C-15623 | Me(R) | single bond | H | *c*-Pr[1-S(=O)₂CF₃] | Me | Cl | C(=O)Me | O |
| C-15624 | Me(S) | single bond | H | *c*-Pr[1-S(=O)₂CF₃] | Me | Cl | C(=O)Me | O |
| C-15625 | H | single bond | Me | *c*-Pr[1-S(=O)₂CF₃] | Me | Me | C(=O)Me | O |
| C-15626 | Me | single bond | H | *c*-Pr[1-S(=O)₂CF₃] | Me | Me | C(=O)Me | O |
| C-15627 | Me(R) | single bond | H | *c*-Pr[1-S(=O)₂CF₃] | Me | Me | C(=O)Me | O |
| C-15628 | Me(S) | single bond | H | *c*-Pr(1-S(=O)₂CF₃] | Me | Me | C(=O)Me | O |
| C-15629 | H | single bond | Me | *c*-Pr[1-(5,6-dihydro-1,4,2-dioxazin-3-yl)] | Me | Cl | C(=O)Me | O |
| C-15630 | Me | single bond | H | *c*-Pr[1-(5,6-dihydro-1,4,2-dioxazin-3-yl)] | Me | Cl | C(=O)Me | O |
| C-15631 | Me(R) | single bond | H | *c*-Pr[1-(5,6-dihydro-1,4,2-dioxazin-3-yl)] | Me | Cl | C(=O)Me | O |
| C-15632 | Me(S) | single bond | H | *c*-Pr[1-(5,6-dihydro-1,4,2-dioxazin-3-yl)] | Me | Cl | C(=O)Me | O |
| C-15633 | H | single bond | Me | *c*-Pr[1-(5,6-dihydro-1,4,2-dioxazin-3-yl)] | Me | Me | C(=O)Me | O |
| C-15634 | Me | single bond | H | *c*-Pr[1-(5,6-dihydro-1,4,2-dioxazin-3-yl)] | Me | Me | C(=O)Me | O |
| C-15635 | Me(R) | single bond | H | *c*-Pr[1-(5,6-dihydro-1,4,2-dioxazin-3-yl)] | Me | Me | C(=O)Me | O |
| C-15636 | Me(S) | single bond | H | *c*-Pr[1-(5,6-dihydro-1,4,2-dioxazin-3-yl)] | Me | Me | C(=O)Me | O |
| C-15637 | H | single bond | Me | *c*-Pr[1-(5-hydroxy-1 ,3,4-oxadiazol-2-yl)] | Me | Cl | C(=O)Me | O |
| C-15638 | Me | single bond | H | *c*-Pr[1-(5-hydroxy-1,3,4-oxadiazol-2-yl)] | Me | Cl | C(=O)Me | O |

**[Table 584]**

| Compound Number | Y | W | Z | R¹ | R² | R³ | R¹⁰ | A |
|---|---|---|---|---|---|---|---|---|
| C-15639 | Me(R) | single bond | H | *c*-Pr[1-(5-hydroxy-1,3,4-oxadiazol-2-yl)] | Me | Cl | C(=O)Me | O |
| C-15640 | Me(S) | single bond | H | *c-*Pr[1-(5-hydroxy-1,3,4-oxadiazol-2-yl)] | Me | Cl | C(=O)Me | O |
| C-15641 | H | single bond | Me | *c*-Pr[1-(5-hydroxy-1,3,4-oxadiazol-2-yl)] | Me | Me | C(=O)Me | O |
| C-15642 | Me | single bond | H | *c*-Pr[1-(5-hydroxy-1,3,4-oxadiazol-2-yl)] | Me | Me | C(=O)Me | O |
| C-15643 | Me(R) | single bond | H | *c*-Pr[1-(5-hydroxy-1,3,4-oxadiazol-2-yl)] | Me | Me | C(=O)Me | O |
| C-15644 | Me(S) | single bond | H | *c*-Pr[1-(5-hydroxy-1,3,4-oxadiazol-2-yl)] | Me | Me | C(=O)Me | O |
| C-15645 | H | single bond | Me | *c*-Pr[1-(pyrimidin-2-yl)] | Me | Cl | C(=O)Me | O |
| C-15646 | Me | single bond | H | *c*-Pr[1-(pyrimidin-2-yl)] | Me | Cl | C(=O)Me | O |
| C-15647 | Me(R) | single bond | H | *c*-Pr[1-(pyrimidin-2-yl)] | Me | Cl | C(=O)Me | O |
| C-15648 | Me(S) | single bond | H | *c*-Pr[1-(pyrimidin-2-yl)] | Me | Cl | C(=O)Me | O |
| C-15649 | H | single bond | Me | *c*-Pr[1-(pyrimidin-2-yl)] | Me | Me | C(=O)Me | O |
| C-15650 | Me | single bond | H | *c*-Pr[1-(pyrimidin-2-yl)] | Me | Me | C(=O)Me | O |
| C-15651 | Me(R) | single bond | H | *c*-Pr[1-(pyrimidin-2-yl)] | Me | Me | C(=O)Me | O |
| C-15652 | Me(S) | single bond | H | *c*-Pr[1-(pyrimidin-2-yl)] | Me | Me | C(=O)Me | O |
| C-15653 | H | single bond | Me | *t*-Bu | Me | Cl | C(=O)Me | O |
| C-15654 | Me | single bond | H | *t*-Bu | Me | Cl | C(=O)Me | O |
| C-15655 | Me(R) | single bond | H | *t*-Bu | Me | Cl | C(=O)Me | O |
| C-15656 | Me(S) | single bond | H | *t*-Bu | Me | Cl | C(=O)Me | O |
| C-15657 | H | single bond | Me | *t*-Bu | Me | Me | C(=O)Me | O |
| C-15658 | Me | single bond | H | *t*-Bu | Me | Me | C(=O)Me | O |
| C-15659 | Me(R) | single bond | H | *t*-Bu | Me | Me | C(=O)Me | O |
| C-15660 | Me(S) | single bond | H | *t*-Bu | Me | Me | C(=O)Me | O |
| C-15661 | H | single bond | Me | *c*-Bu | Me | Cl | C(=O)Me | O |
| C-15662 | Me | single bond | H | *c*-Bu | Me | Cl | C(=O)Me | O |
| C-15663 | Me(R) | single bond | H | *c*-Bu | Me | Cl | C(=O)Me | O |
| C-15664 | Me(S) | single bond | H | *c*-Bu | Me | Cl | C(=O)Me | O |
| C-15665 | H | single bond | Me | *c*-Bu | Me | Me | C(=O)Me | O |
| C-15666 | Me | single bond | H | *c*-Bu | Me | Me | C(=O)Me | O |
| C-15667 | Me(R) | single bond | H | *c*-Bu | Me | Me | C(=O)Me | O |
| C-15668 | Me(S) | single bond | H | *c*-Bu | Me | Me | C(=O)Me | O |
| C-15669 | H | single bond | Me | *c*-Bu(1-CN) | Me | Cl | C(=O)Me | O |
| C-15670 | Me | single bond | H | *c*-Bu(1-CN) | Me | Cl | C(=O)Me | O |
| C-15671 | Me(R) | single bond | H | *c*-Bu(1-CN) | Me | Cl | C(=O)Me | O |
| C-15672 | Me(S) | single bond | H | *c*-Bu(1-CN) | Me | Cl | C(=O)Me | O |
| C-15673 | H | single bond | Me | *c*-Bu(1-CN) | Me | Me | C(=O)Me | O |
| C-15674 | Me | single bond | H | *c*-Bu(1-CN) | Me | Me | C(=O)Me | O |
| C-15675 | Me(R) | single bond | H | *c*-Bu(1-CN) | Me | Me | C(=O)Me | O |
| C-15676 | Me(S) | single bond | H | *c*-Bu(1-CN) | Me | Me | C(=O)Me | O |
| C-15677 | H | single bond | Me | *c*-Bu(3,3-F₂) | Me | Cl | C(=O)Me | O |
| C-15678 | Me | single bond | H | *c*-Bu(3,3-F₂) | Me | Cl | C(=O)Me | O |
| C-15679 | Me(R) | single bond | H | *c*-Bu(3,3-F₂) | Me | Cl | C(=O)Me | O |
| C-15680 | Me(S) | single bond | H | *c*-Bu(3,3-F₂) | Me | Cl | C(=O)Me | O |
| C-15681 | H | single bond | Me | *c*-Bu(3,3-F₂) | Me | Me | C(=O)Me | O |
| C-15682 | Me | single bond | H | *c*-Bu(3,3-F₂) | Me | Me | C(=O)Me | O |

**[Table 585]**

| Compound Number | Y | W | Z | R¹ | R² | R³ | R¹⁰ | A |
|---|---|---|---|---|---|---|---|---|
| C-15683 | Me(R) | single bond | H | *c*-Bu(3,3-F₂) | Me | Me | C(=O)Me | O |
| C-15684 | Me(S) | single bond | H | *c*-Bu(3,3-F₂) | Me | Me | C(=O)Me | O |
| C-15685 | H | single bond | Me | oxetan-3-yl | Me | Cl | C(=O)Me | O |
| C-15686 | Me | single bond | H | oxetan-3-yl | Me | Cl | C(=O)Me | O |
| C-15687 | Me(R) | single bond | H | oxetan-3-yl | Me | Cl | C(=O)Me | O |
| C-15688 | Me(S) | single bond | H | oxetan-3-yl | Me | Cl | C(=O)Me | O |
| C-15689 | H | single bond | Me | oxetan-3-yl | Me | Me | C(=O)Me | O |
| C-15690 | Me | single bond | H | oxetan-3-yl | Me | Me | C(=O)Me | O |
| C-15691 | Me(R) | single bond | H | oxetan-3-yl | Me | Me | C(=O)Me | O |
| C-15692 | Me(S) | single bond | H | oxetan-3-yl | Me | Me | C(=O)Me | O |
| C-15693 | H | single bond | Me | oxetan(3-CN)-3-yl | Me | Cl | C(=O)Me | O |
| C-15694 | Me | single bond | H | oxetan(3-CN)-3-yl | Me | Cl | C(=O)Me | O |
| C-15695 | Me(R) | single bond | H | oxetan(3-CN)-3-yl | Me | Cl | C(=O)Me | O |
| C-15696 | Me(S) | single bond | H | oxetan(3-CN)-3-yl | Me | Cl | C(=O)Me | O |
| C-15697 | H | single bond | Me | oxetan(3-CN)-3-yl | Me | Me | C(=O)Me | O |
| C-15698 | Me | single bond | H | oxetan(3-CN)-3-yl | Me | Me | C(=O)Me | O |
| C-15699 | Me(R) | single bond | H | oxetan(3-CN)-3-yl | Me | Me | C(=O)Me | O |
| C-15700 | Me(S) | single bond | H | oxetan(3-CN)-3-yl | Me | Me | C(=O)Me | O |
| C-15701 | H | single bond | Me | azetidin[1-C(=O)Me]-3-yl | Me | Cl | C(=O)Me | O |
| C-15702 | Me | single bond | H | azetidin[1-C(=O)Me]-3-yl | Me | Cl | C(=O)Me | O |
| C-15703 | Me(R) | single bond | H | azetidin[1-C(=O)Me]-3-yl | Me | Cl | C(=O)Me | O |
| C-15704 | Me(S) | single bond | H | azetidin[1-C(=O)Me]-3-yl | Me | Cl | C(=O)Me | O |
| C-15705 | H | single bond | Me | azetidin[1-C(=O)Me]-3-yl | Me | Me | C(=O)Me | O |
| C-15706 | Me | single bond | H | azetidin[1-C(=O)Me]-3-yl | Me | Me | C(=O)Me | O |
| C-15707 | Me(R) | single bond | H | azetidin[1-C(=O)Me]-3-yl | Me | Me | C(=O)Me | O |
| C-15708 | Me(S) | single bond | H | azetidin[1-C(=O)Me]-3-yl | Me | Me | C(=O)Me | O |
| C-15709 | H | single bond | Me | azetidin[1-S(=O)₂Me]-3-yl | Me | Cl | C(=O)Me | O |
| C-15710 | Me | single bond | H | azetidin[1-S(=O)₂Me]-3-yl | Me | Cl | C(=O)Me | O |
| C-15711 | Me(R) | single bond | H | azetidin[1-S(=O)₂Me]-3-yl | Me | Cl | C(=O)Me | O |
| C-15712 | Me(S) | single bond | H | azetidin[1-S(=O)₂Me]-3-yl | Me | Cl | C(=O)Me | O |
| C-15713 | H | single bond | Me | azetidin[1-S(=O)₂Me]-3-yl | Me | Me | C(=O)Me | O |
| C-15714 | Me | single bond | H | azetidin[1-S(=O)₂Me]-3-yl | Me | Me | C(=O)Me | O |
| C-15715 | Me(R) | single bond | H | azetidin[1-S(=O)₂Me]-3-yl | Me | Me | C(=O)Me | O |
| C-15716 | Me(S) | single bond | H | azetidin[1-S(=O)₂Me]-3-yl | Me | Me | C(=O)Me | O |
| C-15717 | H | single bond | Me | azetidin[1-S(=O)₂CHF₂]-3-yl | Me | Cl | C(=O)Me | O |
| C-15718 | Me | single bond | H | azetidin[1-S(=O)₂CHF₂]-3-yl | Me | Cl | C(=O)Me | O |
| C-15719 | Me(R) | single bond | H | azetidin[1-S(=O)₂CHF₂]-3-yl | Me | Cl | C(=O)Me | O |
| C-15720 | Me(S) | single bond | H | azetidin[1-S(=O)₂CHF₂]-3-yl | Me | Cl | C(=O)Me | O |
| C-15721 | H | single bond | Me | azetidin[1-S(=O)₂CHF₂]-3-yl | Me | Me | C(=O)Me | O |
| C-15722 | Me | single bond | H | azetidin[1-S(=O)₂CHF₂]-3-yl | Me | Me | C(=O)Me | O |
| C-15723 | Me(R) | single bond | H | azetidin[1-S(=O)₂CHF₂]-3-yl | Me | Me | C(=O)Me | O |
| C-15724 | Me(S) | single bond | H | azetidin[1-S(=O)₂CHF₂]-3-yl | Me | Me | C(=O)Me | O |
| C-15725 | H | single bond | Me | *c*-Pen | Me | Cl | C(=O)Me | O |
| C-15726 | Me | single bond | H | *c*-Pen | Me | Cl | C(=O)Me | O |

**[Table 586]**

| Compound Number | Y | W | Z | R¹ | R² | R³ | R¹⁰ | A |
|---|---|---|---|---|---|---|---|---|
| C-15727 | Me(R) | single bond | H | *c*-Pen | Me | Cl | C(=O)Me | O |
| C-15728 | Me(S) | single bond | H | *c*-Pen | Me | Cl | C(=O)Me | O |
| C-15729 | H | single bond | Me | *c*-Pen | Me | Me | C(=O)Me | O |
| C-15730 | Me | single bond | H | *c*-Pen | Me | Me | C(=O)Me | O |
| C-15731 | Me(R) | single bond | H | *c*-Pen | Me | Me | C(=O)Me | O |
| C-15732 | Me(S) | single bond | H | *c*-Pen | Me | Me | C(=O)Me | O |
| C-15733 | H | single bond | Me | tetrahydrofuran-3-yl | Me | Cl | C(=O)Me | O |
| C-15734 | Me | single bond | H | tetrahydrofuran-3-yl | Me | Cl | C(=O)Me | O |
| C-15735 | Me(R) | single bond | H | tetrahydrofuran-3-yl | Me | Cl | C(=O)Me | O |
| C-15736 | Me(S) | single bond | H | tetrahydrofuran-3-yl | Me | Cl | C(=O)Me | O |
| C-15737 | H | single bond | Me | tetrahydrofuran-3-yl | Me | Me | C(=O)Me | O |
| C-15738 | Me | single bond | H | tetrahydrofuran-3-yl | Me | Me | C(=O)Me | O |
| C-15739 | Me(R) | single bond | H | tetrahydrofuran-3-yl | Me | Me | C(=O)Me | O |
| C-15740 | Me(S) | single bond | H | tetrahydrofuran-3-yl | Me | Me | C(=O)Me | O |
| C-15741 | H | single bond | Me | tetrahydrothiophen-3-yl | Me | Cl | C(=O)Me | O |
| C-15742 | Me | single bond | H | tetrahydrothiophen-3-yl | Me | Cl | C(=O)Me | O |
| C-15743 | Me(R) | single bond | H | tetrahydrothiophen-3-yl | Me | Cl | C(=O)Me | O |
| C-15744 | Me(S) | single bond | H | tetrahydrothiophen-3-yl | Me | Cl | C(=O)Me | O |
| C-15745 | H | single bond | Me | tetrahydrothiophen-3-yl | Me | Me | C(=O)Me | O |
| C-15746 | Me | single bond | H | tetrahydrothiophen-3-yl | Me | Me | C(=O)Me | O |
| C-15747 | Me(R) | single bond | H | tetrahydrothiophen-3-yl | Me | Me | C(=O)Me | O |
| C-15748 | Me(S) | single bond | H | tetrahydrothiophen-3-yl | Me | Me | C(=O)Me | O |
| C-15749 | H | single bond | Me | *c*-Pen(1-CN) | Me | Cl | C(=O)Me | O |
| C-15750 | Me | single bond | H | *c*-Pen(1-CN) | Me | Cl | C(=O)Me | O |
| C-15751 | Me(R) | single bond | H | *c*-Pen(1-CN) | Me | Cl | C(=O)Me | O |
| C-15752 | Me(S) | single bond | H | *c*-Pen(1-CN) | Me | Cl | C(=O)Me | O |
| C-15753 | H | single bond | Me | *c*-Pen(1-CN) | Me | Me | C(=O)Me | O |
| C-15754 | Me | single bond | H | *c*-Pen(1-CN) | Me | Me | C(=O)Me | O |
| C-15755 | Me(R) | single bond | H | *c*-Pen(1-CN) | Me | Me | C(=O)Me | O |
| C-15756 | Me(S) | single bond | H | *c*-Pen(1-CN) | Me | Me | C(=O)Me | O |
| C-15757 | H | single bond | Me | tetrahydrofuran(3-CN)-3-yl | Me | Cl | C(=O)Me | O |
| C-15758 | Me | single bond | H | tetrahydrofuran(3-CN)-3-yl | Me | Cl | C(=O)Me | O |
| C-15759 | Me(R) | single bond | H | tetrahydrofuran(3-CN)-3-yl | Me | Cl | C(=O)Me | O |
| C-15760 | Me(S) | single bond | H | tetrahydrofuran(3-CN)-3-yl | Me | Cl | C(=O)Me | O |
| C-15761 | H | single bond | Me | tetrahydrofuran(3-CN)-3-yl | Me | Me | C(=O)Me | O |
| C-15762 | Me | single bond | H | tetrahydrofuran(3-CN)-3-yl | Me | Me | C(=O)Me | O |
| C-15763 | Me(R) | single bond | H | tetrahydrofuran(3-CN)-3-yl | Me | Me | C(=O)Me | O |
| C-15764 | Me(S) | single bond | H | tetrahydrofuran(3-CN)-3-yl | Me | Me | C(=O)Me | O |
| C-15765 | H | single bond | Me | tetrahydrothiophen(3-CN)-3-yl | Me | Cl | C(=O)Me | O |
| C-15766 | Me | single bond | H | tetrahydrothiophen(3-CN)-3-yl | Me | Cl | C(=O)Me | O |
| C-15767 | Me(R) | single bond | H | tetrahydrothiophen(3-CN)-3-yl | Me | Cl | C(=O)Me | O |
| C-15768 | Me(S) | single bond | H | tetrahydrothiophen(3-CN)-3-yl | Me | Cl | C(=O)Me | O |
| C-15769 | H | single bond | Me | tetrahydrothiophen(3-CN)-3-yl | Me | Me | C(=O)Me | O |
| C-15770 | Me | single bond | H | tetrahydrothiophen(3-CN)-3-yl | Me | Me | C(=O)Me | O |

**[Table 587]**

| Compound Number | Y | W | Z | R¹ | R² | R³ | R¹⁰ | A |
|---|---|---|---|---|---|---|---|---|
| C-15771 | Me(R) | single bond | H | tetrahydrothiophen(3-CN)-3-yl | Me | Me | C(=O)Me | O |
| C-15772 | Me(S) | single bond | H | tetrahydrothiophen(3-CN)-3-yl | Me | Me | C(=O)Me | O |
| C-15773 | H | single bond | Me | bicyclo[111]pentan-1-yl | Me | Cl | C(=O)Me | O |
| C-15774 | Me | single bond | H | bicyclo[1.1.1]pentan-1-yl | Me | Cl | C(=O)Me | O |
| C-15775 | Me(R) | single bond | H | bicyclo[1.1.1]pentan-1-yl | Me | Cl | C(=O)Me | O |
| C-15776 | Me(S) | single bond | H | bicyclo[1.1.1]pentan-1-yl | Me | Cl | C(=O)Me | O |
| C-15777 | H | single bond | Me | bicyclo[1.1.1]pentan-1-yl | Me | Me | C(=O)Me | O |
| C-15778 | Me | single bond | H | bicyclo[1.1.1]pentan-1-yl | Me | Me | C(=O)Me | O |
| C-15779 | Me(R) | single bond | H | bicyclo[1.1.1]pentan-1-yl | Me | Me | C(=O)Me | O |
| C-15780 | Me(S) | single bond | H | bicyclo[1.1.1]pentan-1-yl | Me | Me | C(=O)Me | O |
| C-15781 | H | single bond | Me | *c*-Hex | Me | Cl | C(=O)Me | O |
| C-15782 | Me | single bond | H | *c*-Hex | Me | Cl | C(=O)Me | O |
| C-15783 | Me(R) | single bond | H | *c*-Hex | Me | Cl | C(=O)Me | O |
| C-15784 | Me(S) | single bond | H | *c*-Hex | Me | Cl | C(=O)Me | O |
| C-15785 | H | single bond | Me | *c*-Hex | Me | Me | C(=O)Me | O |
| C-15786 | Me | single bond | H | *c*-Hex | Me | Me | C(=O)Me | O |
| C-15787 | Me(R) | single bond | H | *c*-Hex | Me | Me | C(=O)Me | O |
| C-15788 | Me(S) | single bond | H | *c*-Hex | Me | Me | C(=O)Me | O |
| C-15789 | H | single bond | Me | tetrahydropyran-4-yl | Me | Cl | C(=O)Me | O |
| C-15790 | Me | single bond | H | tetrahydropyran-4-yl | Me | Cl | C(=O)Me | O |
| C-15791 | Me(R) | single bond | H | tetrahydropyran-4-yl | Me | Cl | C(=O)Me | O |
| C-15792 | Me(S) | single bond | H | tetrahydropyran-4-yl | Me | Cl | C(=O)Me | O |
| C-15793 | H | single bond | Me | tetrahydropyran-4-yl | Me | Me | C(=O)Me | O |
| C-15794 | Me | single bond | H | tetrahydropyran-4-yl | Me | Me | C(=O)Me | O |
| C-15795 | Me(R) | single bond | H | tetrahydropyran-4-yl | Me | Me | C(=O)Me | O |
| C-15796 | Me(S) | single bond | H | tetrahydropyran-4-yl | Me | Me | C(=O)Me | O |
| C-15797 | H | single bond | Me | tetrahydrothiopyran-4-yl | Me | Cl | C(=O)Me | O |
| C-15798 | Me | single bond | H | tetrahydrothiopyran-4-yl | Me | Cl | C(=O)Me | O |
| C-15799 | Me(R) | single bond | H | tetrahydrothiopyran-4-yl | Me | Cl | C(=O)Me | O |
| C-15800 | Me(S) | single bond | H | tetrahydrothiopyran-4-yl | Me | Cl | C(=O)Me | O |
| C-15801 | H | single bond | Me | tetrahydrothiopyran-4-yl | Me | Me | C(=O)Me | O |
| C-15802 | Me | single bond | H | tetrahydrothiopyran-4-yl | Me | Me | C(=O)Me | O |
| C-15803 | Me(R) | single bond | H | tetrahydrothiopyran-4-yl | Me | Me | C(=O)Me | O |
| C-15804 | Me(S) | single bond | H | tetrahydrothiopyran-4-yl | Me | Me | C(=O)Me | O |
| C-15805 | H | single bond | Me | *c*-Hex(1-CN) | Me | Cl | C(=O)Me | O |
| C-15806 | Me | single bond | H | *c*-Hex(1-CN) | Me | Cl | C(=O)Me | O |
| C-15807 | Me(R) | single bond | H | *c*-Hex(1-CN) | Me | Cl | C(=O)Me | O |
| C-15808 | Me(S) | single bond | H | *c*-Hex(1-CN) | Me | Cl | C(=O)Me | O |
| C-15809 | H | single bond | Me | *c*-Hex(1-CN) | Me | Me | C(=O)Me | O |
| C-15810 | Me | single bond | H | *c*-Hex(1-CN) | Me | Me | C(=O)Me | O |
| C-15811 | Me(R) | single bond | H | *c*-Hex(1-CN) | Me | Me | C(=O)Me | O |
| C-15812 | Me(S) | single bond | H | *c*-Hex(1-CN) | Me | Me | C(=O)Me | O |
| C-15813 | H | single bond | Me | tetrahydropyran(4-CN)-4-yl | Me | Cl | C(=O)Me | O |
| C-15814 | Me | single bond | H | tetrahydropyran(4-CN)-4-yl | Me | Cl | C(=O)Me | O |

**[Table 588]**

| Compound Number | Y | W | Z | R¹ | R² | R³ | R¹⁰ | A |
|---|---|---|---|---|---|---|---|---|
| C-15815 | Me(R) | single bond | H | tetrahydropyran(4-CN)-4-yl | Me | Cl | C(=O)Me | O |
| C-15816 | Me(S) | single bond | H | tetrahydropyran(4-CN)-4-yl | Me | Cl | C(=O)Me | O |
| C-15817 | H | single bond | Me | tetrahydropyran(4-CN)-4-yl | Me | Me | C(=O)Me | O |
| C-15818 | Me | single bond | H | tetrahydropyran(4-CN)-4-yl | Me | Me | C(=O)Me | O |
| C-15819 | Me(R) | single bond | H | tetrahydropyran(4-CN)-4-yl | Me | Me | C(=O)Me | O |
| C-15820 | Me(S) | single bond | H | tetrahydropyran(4-CN)-4-yl | Me | Me | C(=O)Me | O |
| C-15821 | H | single bond | Me | tetrahydrothiopyran(4-CN)-4-yl | Me | Cl | C(=O)Me | O |
| C-15822 | Me | single bond | H | tetrahydrothiopyran(4-CN)-4-yl | Me | Cl | C(=O)Me | O |
| C-15823 | Me(R) | single bond | H | tetrahydrothiopyran(4-CN)-4-yl | Me | Cl | C(=O)Me | O |
| C-15824 | Me(S) | single bond | H | tetrahydrothiopyran(4-CN)-4-yl | Me | Cl | C(=O)Me | O |
| C-15825 | H | single bond | Me | tetrahydrothiopyran(4-CN)-4-yl | Me | Me | C(=O)Me | O |
| C-15826 | Me | single bond | H | tetrahydrothiopyran(4-CN)-4-yl | Me | Me | C(=O)Me | O |
| C-15827 | Me(R) | single bond | H | tetrahydrothiopyran(4-CN)-4-yl | Me | Me | C(=O)Me | O |
| C-15828 | Me(S) | single bond | H | tetrahydrothiopyran(4-CN)-4-yl | Me | Me | C(=O)Me | O |
| C-15829 | H | single bond | Me | CH₂(*c*-Pr) | Me | Cl | C(=O)Me | O |
| C-15830 | Me | single bond | H | CH₂(*c*-Pr) | Me | Cl | C(=O)Me | O |
| C-15831 | Me(R) | single bond | H | CH₂(*c*-Pr) | Me | Cl | C(=O)Me | O |
| C-15832 | Me(S) | single bond | H | CH₂(*c*-Pr) | Me | Cl | C(=O)Me | O |
| C-15833 | H | single bond | Me | CH₂(*c*-Pr) | Me | Me | C(=O)Me | O |
| C-15834 | Me | single bond | H | CH₂(*c*-Pr) | Me | Me | C(=O)Me | O |
| C-15835 | Me(R) | single bond | H | CH₂(*c*-Pr) | Me | Me | C(=O)Me | O |
| C-15836 | Me(S) | single bond | H | CH₂(*c*-Pr) | Me | Me | C(=O)Me | O |
| C-15837 | H | single bond | Me | CH₂[*c*-Pr(1-CN)] | Me | Cl | C(=O)Me | O |
| C-15838 | Me | single bond | H | CH₂[*c*-Pr(1-CN)] | Me | Cl | C(=O)Me | O |
| C-15839 | Me(R) | single bond | H | CH₂[*c*-Pr(1-CN)] | Me | Cl | C(=O)Me | O |
| C-15840 | Me(S) | single bond | H | CH₂[*c*-Pr(1-CN)] | Me | Cl | C(=O)Me | O |
| C-15841 | H | single bond | Me | CH₂[*c*-Pr(1-CN)] | Me | Me | C(=O)Me | O |
| C-15842 | Me | single bond | H | CH₂[*c*-Pr(1-CN)] | Me | Me | C(=O)Me | O |
| C-15843 | Me(R) | single bond | H | CH₂[*c*-Pr(1-CN)] | Me | Me | C(=O)Me | O |
| C-15844 | Me(S) | single bond | H | CH₂[*c*-Pr(1-CN)] | Me | Me | C(=O)Me | O |
| C-15845 | H | single bond | Me | CH(Me)(*c*-Pr) | Me | Cl | C(=O)Me | O |
| C-15846 | Me | single bond | H | CH(Me)(*c*-Pr) | Me | Cl | C(=O)Me | O |
| C-15847 | Me(R) | single bond | H | CH(Me)(*c*-Pr) | Me | Cl | C(=O)Me | O |
| C-15848 | Me(S) | single bond | H | CH(Me)(*c*-Pr) | Me | Cl | C(=O)Me | O |
| C-15849 | H | single bond | Me | CH(Me)(*c*-Pr) | Me | Me | C(=O)Me | O |
| C-15850 | Me | single bond | H | CH(Me)(*c*-Pr) | Me | Me | C(=O)Me | O |
| C-15851 | Me(R) | single bond | H | CH(Me)(*c*-Pr) | Me | Me | C(=O)Me | O |
| C-15852 | Me(S) | single bond | H | CH(Me)(*c*-Pr) | Me | Me | C(=O)Me | O |
| C-15853 | H | single bond | Me | CH(Me)CH₂OMe | Me | Cl | C(=O)Me | O |
| C-15854 | Me | single bond | H | CH(Me)CH₂OMe | Me | Cl | C(=O)Me | O |
| C-15855 | Me(R) | single bond | H | CH(Me)CH₂OMe | Me | Cl | C(=O)Me | O |
| C-15856 | Me(S) | single bond | H | CH(Me)CH₂OMe | Me | Cl | C(=O)Me | O |
| C-15857 | H | single bond | Me | CH(Me)CH₂OMe | Me | Me | C(=O)Me | O |
| C-15858 | Me | single bond | H | CH(Me)CH₂OMe | Me | Me | C(=O)Me | O |

**[Table 589]**

| Compound Number | Y | W | Z | R¹ | R² | R³ | R¹⁰ | A |
|---|---|---|---|---|---|---|---|---|
| C-15859 | Me(R) | single bond | H | CH(Me)CH₂OMe | Me | Me | C(=O)Me | O |
| C-15860 | Me(S) | single bond | H | CH(Me)CH₂OMe | Me | Me | C(=O)Me | O |
| C-15861 | H | single bond | Me | CH₂CH=CH₂ | Me | Cl | C(=O)Me | O |
| C-15862 | Me | single bond | H | CH₂CH=CH₂ | Me | Cl | C(=O)Me | O |
| C-15863 | Me(R) | single bond | H | CH₂CH=CH₂ | Me | Cl | C(=O)Me | O |
| C-15864 | Me(S) | single bond | H | CH₂CH=CH₂ | Me | Cl | C(=O)Me | O |
| C-15865 | H | single bond | Me | CH₂CH=CH₂ | Me | Me | C(=O)Me | O |
| C-15866 | Me | single bond | H | CH₂CH=CH₂ | Me | Me | C(=O)Me | O |
| C-15867 | Me(R) | single bond | H | CH₂CH=CH₂ | Me | Me | C(=O)Me | O |
| C-15868 | Me(S) | single bond | H | CH₂CH=CH₂ | Me | Me | C(=O)Me | O |
| C-15869 | H | single bond | Me | CH₂C≡CH | Me | Cl | C(=O)Me | O |
| C-15870 | Me | single bond | H | CH₂C≡CH | Me | Cl | C(=O)Me | O |
| C-15871 | Me(R) | single bond | H | CH₂C≡CH | Me | Cl | C(=O)Me | O |
| C-15872 | Me(S) | single bond | H | CH₂C≡CH | Me | Cl | C(=O)Me | O |
| C-15873 | H | single bond | Me | CH₂C≡CH | Me | Me | C(=O)Me | O |
| C-15874 | Me | single bond | H | CH₂C≡CH | Me | Me | C(=O)Me | O |
| C-15875 | Me(R) | single bond | H | CH₂C≡CH | Me | Me | C(=O)Me | O |
| C-15876 | Me(S) | single bond | H | CH₂C≡CH | Me | Me | C(=O)Me | O |
| C-15877 | H | single bond | Me | CH₂CN | Me | Cl | C(=O)Me | O |
| C-15878 | Me | single bond | H | CH₂CN | Me | Cl | C(=O)Me | O |
| C-15879 | Me(R) | single bond | H | CH₂CN | Me | Cl | C(=O)Me | O |
| C-15880 | Me(S) | single bond | H | CH₂CN | Me | Cl | C(=O)Me | O |
| C-15881 | H | single bond | Me | CH₂CN | Me | Me | C(=O)Me | O |
| C-15882 | Me | single bond | H | CH₂CN | Me | Me | C(=O)Me | O |
| C-15883 | Me(R) | single bond | H | CH₂CN | Me | Me | C(=O)Me | O |
| C-15884 | Me(S) | single bond | H | CH₂CN | Me | Me | C(=O)Me | O |
| C-15885 | H | single bond | Me | CH(Me)CN | Me | Cl | C(=O)Me | O |
| C-15886 | Me | single bond | H | CH(Me)CN | Me | Cl | C(=O)Me | O |
| C-15887 | Me(R) | single bond | H | CH(Me)CN | Me | Cl | C(=O)Me | O |
| C-15888 | Me(S) | single bond | H | CH(Me)CN | Me | Cl | C(=O)Me | O |
| C-15889 | H | single bond | Me | CH(Me)CN | Me | Me | C(=O)Me | O |
| C-15890 | Me | single bond | H | CH(Me)CN | Me | Me | C(=O)Me | O |
| C-15891 | Me(R) | single bond | H | CH(Me)CN | Me | Me | C(=O)Me | O |
| C-15892 | Me(S) | single bond | H | CH(Me)CN | Me | Me | C(=O)Me | O |
| C-15893 | H | single bond | Me | C(Me)₂CN | Me | Cl | C(=O)Me | O |
| C-15894 | Me | single bond | H | C(Me)₂CN | Me | Cl | C(=O)Me | O |
| C-15895 | Me(R) | single bond | H | C(Me)₂CN | Me | Cl | C(=O)Me | O |
| C-15896 | Me(S) | single bond | H | C(Me)₂CN | Me | Cl | C(=O)Me | O |
| C-15897 | H | single bond | Me | C(Me)₂CN | Me | Me | C(=O)Me | O |
| C-15898 | Me | single bond | H | C(Me)₂CN | Me | Me | C(=O)Me | O |
| C-15899 | Me(R) | single bond | H | C(Me)₂CN | Me | Me | C(=O)Me | O |
| C-15900 | Me(S) | single bond | H | C(Me)₂CN | Me | Me | C(=O)Me | O |
| C-15901 | H | single bond | Me | C(Me)(*i*-Pr)CN | Me | Cl | C(=O)Me | O |
| C-15902 | Me | single bond | H | C(Me)(*i*-Pr)CN | Me | Cl | C(=O)Me | O |

**[Table 590]**

| Compound Number | Y | W | Z | R¹ | R² | R³ | R¹⁰ | A |
|---|---|---|---|---|---|---|---|---|
| C-15903 | Me(R) | single bond | H | C(Me)(*i*-Pr)CN | Me | Cl | C(=O)Me | O |
| C-15904 | Me(S) | single bond | H | C(Me)(*i*-Pr)CN | Me | Cl | C(=O)Me | O |
| C-15905 | H | single bond | Me | C(Me)(*i*-Pr)CN | Me | Me | C(=O)Me | O |
| C-15906 | Me | single bond | H | C(Me)(*i*-Pr)CN | Me | Me | C(=O)Me | O |
| C-15907 | Me(R) | single bond | H | C(Me)(*i*-Pr)CN | Me | Me | C(=O)Me | O |
| C-15908 | Me(S) | single bond | H | C(Me)(*i*-Pr)CN | Me | Me | C(=O)Me | O |
| C-15909 | H | single bond | Me | OMe | Me | Cl | C(=O)Me | O |
| C-15910 | Me | single bond | H | OMe | Me | Cl | C(=O)Me | O |
| C-15911 | Me(R) | single bond | H | OMe | Me | Cl | C(=O)Me | O |
| C-15912 | Me(S) | single bond | H | OMe | Me | Cl | C(=O)Me | O |
| C-15913 | H | single bond | Me | OMe | Me | Me | C(=O)Me | O |
| C-15914 | Me | single bond | H | OMe | Me | Me | C(=O)Me | O |
| C-15915 | Me(R) | single bond | H | OMe | Me | Me | C(=O)Me | O |
| C-15916 | Me(S) | single bond | H | OMe | Me | Me | C(=O)Me | O |
| C-15917 | H | single bond | Me | CH=NOMe | Me | Cl | C(=O)Me | O |
| C-15918 | Me | single bond | H | CH=NOMe | Me | Cl | C(=O)Me | O |
| C-15919 | Me(R) | single bond | H | CH=NOMe | Me | Cl | C(=O)Me | O |
| C-15920 | Me(S) | single bond | H | CH=NOMe | Me | Cl | C(=O)Me | O |
| C-15921 | H | single bond | Me | CH=NOMe | Me | Me | C(=O)Me | O |
| C-15922 | Me | single bond | H | CH=NOMe | Me | Me | C(=O)Me | O |
| C-15923 | Me(R) | single bond | H | CH=NOMe | Me | Me | C(=O)Me | O |
| C-15924 | Me(S) | single bond | H | CH=NOMe | Me | Me | C(=O)Me | O |
| C-15925 | H | single bond | Me | morpholin-4-yl | Me | Cl | C(=O)Me | O |
| C-15926 | Me | single bond | H | morpholin-4-yl | Me | Cl | C(=O)Me | O |
| C-15927 | Me(R) | single bond | H | morpholin-4-yl | Me | Cl | C(=O)Me | O |
| C-15928 | Me(S) | single bond | H | morpholin-4-yl | Me | Cl | C(=O)Me | O |
| C-15929 | H | single bond | Me | morpholin-4-yl | Me | Me | C(=O)Me | O |
| C-15930 | Me | single bond | H | morpholin-4-yl | Me | Me | C(=O)Me | O |
| C-15931 | Me(R) | single bond | H | morpholin-4-yl | Me | Me | C(=O)Me | O |
| C-15932 | Me(S) | single bond | H | morpholin-4-yl | Me | Me | C(=O)Me | O |
| C-15933 | H | single bond | Me | Ph | Me | Cl | C(=O)Me | O |
| C-15934 | Me | single bond | H | Ph | Me | Cl | C(=O)Me | O |
| C-15935 | Me(R) | single bond | H | Ph | Me | Cl | C(=O)Me | O |
| C-15936 | Me(S) | single bond | H | Ph | Me | Cl | C(=O)Me | O |
| C-15937 | H | single bond | Me | Ph | Me | Me | C(=O)Me | O |
| C-15938 | Me | single bond | H | Ph | Me | Me | C(=O)Me | O |
| C-15939 | Me(R) | single bond | H | Ph | Me | Me | C(=O)Me | O |
| C-15940 | Me(S) | single bond | H | Ph | Me | Me | C(=O)Me | O |
| C-15941 | H | single bond | Me | CH₂Ph | Me | Cl | C(=O)Me | O |
| C-15942 | Me | single bond | H | CH₂Ph | Me | Cl | C(=O)Me | O |
| C-15943 | Me(R) | single bond | H | CH₂Ph | Me | Cl | C(=O)Me | O |
| C-15944 | Me(S) | single bond | H | CH₂Ph | Me | Cl | C(=O)Me | O |
| C-15945 | H | single bond | Me | CH₂Ph | Me | Me | C(=O)Me | O |
| C-15946 | Me | single bond | H | CH₂Ph | Me | Me | C(=O)Me | O |

**[Table 591]**

| Compound Number | Y | W | Z | R¹ | R² | R³ | R¹⁰ | A |
|---|---|---|---|---|---|---|---|---|
| C-15947 | Me(R) | single bond | H | CH₂Ph | Me | Me | C(=O)Me | O |
| C-15948 | Me(S) | single bond | H | CH₂Ph | Me | Me | C(=O)Me | O |
| C-15949 | H | single bond | Me | thiazole-2-yl | Me | Cl | C(=O)Me | O |
| C-15950 | Me | single bond | H | thiazole-2-yl | Me | Cl | C(=O)Me | O |
| C-15951 | Me(R) | single bond | H | thiazole-2-yl | Me | Cl | C(=O)Me | O |
| C-15952 | Me(S) | single bond | H | thiazole-2-yl | Me | Cl | C(=O)Me | O |
| C-15953 | H | single bond | Me | thiazole-2-yl | Me | Me | C(=O)Me | O |
| C-15954 | Me | single bond | H | thiazole-2-yl | Me | Me | C(=O)Me | O |
| C-15955 | Me(R) | single bond | H | thiazole-2-yl | Me | Me | C(=O)Me | O |
| C-15956 | Me(S) | single bond | H | thiazole-2-yl | Me | Me | C(=O)Me | O |
| C-15957 | H | single bond | Me | 1-Me-pyrazol-3-yl | Me | Cl | C(=O)Me | O |
| C-15958 | Me | single bond | H | 1-Me-pyrazol-3-yl | Me | Cl | C(=O)Me | O |
| C-15959 | Me(R) | single bond | H | 1-Me-pyrazol-3-yl | Me | Cl | C(=O)Me | O |
| C-15960 | Me(S) | single bond | H | 1-Me-pyrazol-3-yl | Me | Cl | C(=O)Me | O |
| C-15961 | H | single bond | Me | 1-Me-pyrazol-3-yl | Me | Me | C(=O)Me | O |
| C-15962 | Me | single bond | H | 1-Me-pyrazol-3-yl | Me | Me | C(=O)Me | O |
| C-15963 | Me(R) | single bond | H | 1-Me-pyrazol-3-yl | Me | Me | C(=O)Me | O |
| C-15964 | Me(S) | single bond | H | 1-Me-pyrazol-3-yl | Me | Me | C(=O)Me | O |
| C-15965 | H | single bond | Me | 1-Me-pyrazol-5-yl | Me | Cl | C(=O)Me | O |
| C-15966 | Me | single bond | H | 1-Me-pyrazol-5-yl | Me | Cl | C(=O)Me | O |
| C-15967 | Me(R) | single bond | H | 1-Me-pyrazol-5-yl | Me | Cl | C(=O)Me | O |
| C-15968 | Me(S) | single bond | H | 1-Me-pyrazol-5-yl | Me | Cl | C(=O)Me | O |
| C-15969 | H | single bond | Me | 1-Me-pyrazol-5-yl | Me | Me | C(=O)Me | O |
| C-15970 | Me | single bond | H | 1-Me-pyrazol-5-yl | Me | Me | C(=O)Me | O |
| C-15971 | Me(R) | single bond | H | 1-Me-pyrazol-5-yl | Me | Me | C(=O)Me | O |
| C-15972 | Me(S) | single bond | H | 1-Me-pyrazol-5-yl | Me | Me | C(=O)Me | O |
| C-15973 | H | single bond | Me | CH₂(pyrimidin-2-yl) | Me | Cl | C(=O)Me | O |
| C-15974 | Me | single bond | H | CH₂(pyrimidin-2-yl) | Me | Cl | C(=O)Me | O |
| C-15975 | Me(R) | single bond | H | CH₂(pyrimidin-2-yl) | Me | Cl | C(=O)Me | O |
| C-15976 | Me(S) | single bond | H | CH₂(pyrimidin-2-yl) | Me | Cl | C(=O)Me | O |
| C-15977 | H | single bond | Me | CH₂(pyrimidin-2-yl) | Me | Me | C(=O)Me | O |
| C-15978 | Me | single bond | H | CH₂(pyrimidin-2-yl) | Me | Me | C(=O)Me | O |
| C-15979 | Me(R) | single bond | H | CH₂(pyrimidin-2-yl) | Me | Me | C(=O)Me | O |
| C-15980 | Me(S) | single bond | H | CH₂(pyrimidin-2-yl) | Me | Me | C(=O)Me | O |
| C-15981 | H | single bond | Me | Me | C(=O)Me | Cl | H | O |
| C-15982 | Me | single bond | H | Me | C(=O)Me | Cl | H | O |
| C-15983 | Me(R) | single bond | H | Me | C(=O)Me | Cl | H | O |
| C-15984 | Me(S) | single bond | H | Me | C(=O)Me | Cl | H | O |
| C-15985 | H | single bond | Me | Me | C(=O)Me | Me | H | O |
| C-15986 | Me | single bond | H | Me | C(=O)Me | Me | H | O |
| C-15987 | Me(R) | single bond | H | Me | C(=O)Me | Me | H | O |
| C-15988 | Me(S) | single bond | H | Me | C(=O)Me | Me | H | O |
| C-15989 | H | single bond | Me | CH₂CHF₂ | C(=O)Me | Cl | H | O |
| C-15990 | Me | single bond | H | CH₂CHF₂ | C(=O)Me | Cl | H | O |

**[Table 592]**

| Compound Number | Y | W | Z | R¹ | R² | R³ | R¹⁰ | A |
|---|---|---|---|---|---|---|---|---|
| C-15991 | Me(R) | single bond | H | CH₂CHF₂ | C(=O)Me | Cl | H | O |
| C-15992 | Me(S) | single bond | H | CH₂CHF₂ | C(=O)Me | Cl | H | O |
| C-15993 | H | single bond | Me | CH₂CHF₂ | C(=O)Me | Me | H | O |
| C-15994 | Me | single bond | H | CH₂CHF₂ | C(=O)Me | Me | H | O |
| C-15995 | Me(R) | single bond | H | CH₂CHF₂ | C(=O)Me | Me | H | O |
| C-15996 | Me(S) | single bond | H | CH₂CHF₂ | C(=O)Me | Me | H | O |
| C-15997 | H | single bond | Me | *n*-Pr | C(=O)Me | Cl | H | O |
| C-15998 | Me | single bond | H | *n*-Pr | C(=O)Me | Cl | H | O |
| C-15999 | Me(R) | single bond | H | *n*-Pr | C(=O)Me | Cl | H | O |
| C-16000 | Me(S) | single bond | H | *n*-Pr | C(=O)Me | Cl | H | O |
| C-16001 | H | single bond | Me | *n*-Pr | C(=O)Me | Me | H | O |
| C-16002 | Me | single bond | H | *n*-Pr | C(=O)Me | Me | H | O |
| C-16003 | Me(R) | single bond | H | *n*-Pr | C(=O)Me | Me | H | O |
| C-16004 | Me(S) | single bond | H | *n*-Pr | C(=O)Me | Me | H | O |
| C-16005 | H | single bond | Me | *i*-Pr | C(=O)Me | Cl | H | O |
| C-16006 | Me | single bond | H | *i*-Pr | C(=O)Me | Cl | H | O |
| C-16007 | Me(R) | single bond | H | *i*-Pr | C(=O)Me | Cl | H | O |
| C-16008 | Me(S) | single bond | H | *i*-Pr | C(=O)Me | Cl | H | O |
| C-16009 | H | single bond | Me | *i*-Pr | C(=O)Me | Me | H | O |
| C-16010 | Me | single bond | H | *i*-Pr | C(=O)Me | Me | H | O |
| C-16011 | Me(R) | single bond | H | *i*-Pr | C(=O)Me | Me | H | O |
| C-16012 | Me(S) | single bond | H | *i*-Pr | C(=O)Me | Me | H | O |
| C-16013 | H | single bond | Me | *c*-Pr(1-F) | C(=O)Me | Cl | H | O |
| C-16014 | Me | single bond | H | *c*-Pr(1-F) | C(=O)Me | Cl | H | O |
| C-16015 | Me(R) | single bond | H | *c*-Pr(1-F) | C(=O)Me | Cl | H | O |
| C-16016 | Me(S) | single bond | H | *c*-Pr(1-F) | C(=O)Me | Cl | H | O |
| C-16017 | H | single bond | Me | *c*-Pr(1-F) | C(=O)Me | Me | H | O |
| C-16018 | Me | single bond | H | *c*-Pr(1-F) | C(=O)Me | Me | H | O |
| C-16019 | Me(R) | single bond | H | *c*-Pr(1-F) | C(=O)Me | Me | H | O |
| C-16020 | Me(S) | single bond | H | *c*-Pr(1-F) | C(=O)Me | Me | H | O |
| C-16021 | H | single bond | Me | *c*-Pr(2,2-F₂) | C(=O)Me | Cl | H | O |
| C-16022 | Me | single bond | H | *c*-Pr(2,2-F₂) | C(=O)Me | Cl | H | O |
| C-16023 | Me(R) | single bond | H | *c*-Pr(2,2-F₂) | C(=O)Me | Cl | H | O |
| C-16024 | Me(S) | single bond | H | *c*-Pr(2,2-F₂) | C(=O)Me | Cl | H | O |
| C-16025 | H | single bond | Me | *c*-Pr(2,2-F₂) | C(=O)Me | Me | H | O |
| C-16026 | Me | single bond | H | *c*-Pr(2,2-F₂) | C(=O)Me | Me | H | O |
| C-16027 | Me(R) | single bond | H | *c*-Pr(2,2-F₂) | C(=O)Me | Me | H | O |
| C-16028 | Me(S) | single bond | H | *c*-Pr(2,2-F₂) | C(=O)Me | Me | H | O |
| C-16029 | H | single bond | Me | *c*-Pr(1-Cl) | C(=O)Me | Cl | H | O |
| C-16030 | Me | single bond | H | *c*-Pr(1-Cl) | C(=O)Me | Cl | H | O |
| C-16031 | Me(R) | single bond | H | *c*-Pr(1-Cl) | C(=O)Me | Cl | H | O |
| C-16032 | Me(S) | single bond | H | *c*-Pr(1-Cl) | C(=O)Me | Cl | H | O |
| C-16033 | H | single bond | Me | *c*-Pr(1-Cl) | C(=O)Me | Me | H | O |
| C-16034 | Me | single bond | H | *c*-Pr(1-Cl) | C(=O)Me | Me | H | O |

**[Table 593]**

| Compound Number | Y | W | Z | R¹ | R² | R³ | R¹⁰ | A |
|---|---|---|---|---|---|---|---|---|
| C-16035 | Me(R) | single bond | H | *c*-Pr(1-Cl) | C(=O)Me | Me | H | O |
| C-16036 | Me(S) | single bond | H | *c*-Pr(1-Cl) | C(=O)Me | Me | H | O |
| C-16037 | H | single bond | Me | *c*-Pr(1-Br) | C(=O)Me | Cl | H | O |
| C-16038 | Me | single bond | H | *c*-Pr(1-Br) | C(=O)Me | Cl | H | O |
| C-16039 | Me(R) | single bond | H | *c*-Pr(1-Br) | C(=O)Me | Cl | H | O |
| C-16040 | Me(S) | single bond | H | *c*-Pr(1-Br) | C(=O)Me | Cl | H | O |
| C-16041 | H | single bond | Me | *c*-Pr(1-Br) | C(=O)Me | Me | H | O |
| C-16042 | Me | single bond | H | *c*-Pr(1-Br) | C(=O)Me | Me | H | O |
| C-16043 | Me(R) | single bond | H | *c*-Pr(1-Br) | C(=O)Me | Me | H | O |
| C-16044 | Me(S) | single bond | H | *c*-Pr(1-Br) | C(=O)Me | Me | H | O |
| C-16045 | H | single bond | Me | *c*-Pr(1-Me) | C(=O)Me | Cl | H | O |
| C-16046 | Me | single bond | H | *c*-Pr(1-Me) | C(=O)Me | Cl | H | O |
| C-16047 | Me(R) | single bond | H | *c*-Pr(1-Me) | C(=O)Me | Cl | H | O |
| C-16048 | Me(S) | single bond | H | *c*-Pr(1-Me) | C(=O)Me | Cl | H | O |
| C-16049 | H | single bond | Me | *c*-Pr(1-Me) | C(=O)Me | Me | H | O |
| C-16050 | Me | single bond | H | *c*-Pr(1-Me) | C(=O)Me | Me | H | O |
| C-16051 | Me(R) | single bond | H | *c*-Pr(1-Me) | C(=O)Me | Me | H | O |
| C-16052 | Me(S) | single bond | H | *c*-Pr(1-Me) | C(=O)Me | Me | H | O |
| C-16053 | H | single bond | Me | *c*-Pr(1-CH₂OH) | C(=O)Me | Cl | H | O |
| C-16054 | Me | single bond | H | *c*-Pr(1-CH₂OH) | C(=O)Me | Cl | H | O |
| C-16055 | Me(R) | single bond | H | *c*-Pr(1-CH₂OH) | C(=O)Me | Cl | H | O |
| C-16056 | Me(S) | single bond | H | *c*-Pr(1-CH₂OH) | C(=O)Me | Cl | H | O |
| C-16057 | H | single bond | Me | *c*-Pr(1-CH₂OH) | C(=O)Me | Me | H | O |
| C-16058 | Me | single bond | H | *c*-Pr(1-CH₂OH) | C(=O)Me | Me | H | O |
| C-16059 | Me(R) | single bond | H | *c*-Pr(1-CH₂OH) | C(=O)Me | Me | H | O |
| C-16060 | Me(S) | single bond | H | *c*-Pr(1-CH₂OH) | C(=O)Me | Me | H | O |
| C-16061 | H | single bond | Me | *c*-Pr[1-CH₂OS(=O)₂Me] | C(=O)Me | Cl | H | O |
| C-16062 | Me | single bond | H | *c*-Pr[1-CH₂OS(=O)₂Me] | C(=O)Me | Cl | H | O |
| C-16063 | Me(R) | single bond | H | *c*-Pr[1-CH₂OS(=O)₂Me] | C(=O)Me | Cl | H | O |
| C-16064 | Me(S) | single bond | H | *c*-Pr[1-CH₂OS(=O)₂Me] | C(=O)Me | Cl | H | O |
| C-16065 | H | single bond | Me | *c*-Pr[1-CH₂OS(=O)₂Me] | C(=O)Me | Me | H | O |
| C-16066 | Me | single bond | H | *c*-Pr[1-CH₂OS(=O)₂Me] | C(=O)Me | Me | H | O |
| C-16067 | Me(R) | single bond | H | *c*-Pr[1-CH₂OS(=O)₂Me] | C(=O)Me | Me | H | O |
| C-16068 | Me(S) | single bond | H | *c*-Pr[1-CH₂OS(=O)₂Me] | C(=O)Me | Me | H | O |
| C-16069 | H | single bond | Me | *c*-Pr(1-CH₂OMe) | C(=O)Me | Cl | H | O |
| C-16070 | Me | single bond | H | *c*-Pr(1-CH₂OMe) | C(=O)Me | Cl | H | O |
| C-16071 | Me(R) | single bond | H | *c*-Pr(1-CH₂OMe) | C(=O)Me | Cl | H | O |
| C-16072 | Me(S) | single bond | H | *c*-Pr(1-CH₂OMe) | C(=O)Me | Cl | H | O |
| C-16073 | H | single bond | Me | *c*-Pr(1-CH₂OMe) | C(=O)Me | Me | H | O |
| C-16074 | Me | single bond | H | *c*-Pr(1-CH₂OMe) | C(=O)Me | Me | H | O |
| C-16075 | Me(R) | single bond | H | *c*-Pr(1-CH₂OMe) | C(=O)Me | Me | H | O |
| C-16076 | Me(S) | single bond | H | *c*-Pr(1-CH₂OMe) | C(=O)Me | Me | H | O |
| C-16077 | H | single bond | Me | *c*-Pr(1-CH₂CN) | C(=O)Me | Cl | H | O |
| C-16078 | Me | single bond | H | *c*-Pr(1-CH₂CN) | C(=O)Me | Cl | H | O |

**[Table 594]**

| Compound Number | Y | w | z | R¹ | R² | R³ | R¹⁰ | A |
|---|---|---|---|---|---|---|---|---|
| C-16079 | Me(R) | single bond | H | *c*-Pr(1-CH₂CN) | C(=O)Me | Cl | H | O |
| C-16080 | Me(S) | single bond | H | *c*-Pr(1-CH₂CN) | C(=O)Me | Cl | H | O |
| C-16081 | H | single bond | Me | *c*-Pr(1-CH₂CN) | C(=O)Me | Me | H | O |
| C-16082 | Me | single bond | H | *c*-Pr(1-CH₂CN) | C(=O)Me | Me | H | O |
| C-16083 | Me(R) | single bond | H | *c*-Pr(1-CH₂CN) | C(=O)Me | Me | H | O |
| C-16084 | Me(S) | single bond | H | *c*-Pr(1-CH₂CN) | C(=O)Me | Me | H | O |
| C-16085 | H | single bond | Me | *c*-Pr(1-CH₂SMe) | C(=O)Me | Cl | H | O |
| C-16086 | Me | single bond | H | *c*-Pr(1-CH₂SMe) | C(=O)Me | Cl | H | O |
| C-16087 | Me(R) | single bond | H | *c*-Pr(1-CH₂SMe) | C(=O)Me | Cl | H | O |
| C-16088 | Me(S) | single bond | H | *c*-Pr(1-CH₂SMe) | C(=O)Me | Cl | H | O |
| C-16089 | H | single bond | Me | *c*-Pr(1-CH₂SMe) | C(=O)Me | Me | H | O |
| C-16090 | Me | single bond | H | *c*-Pr(1-CH₂SMe) | C(=O)Me | Me | H | O |
| C-16091 | Me(R) | single bond | H | *c*-Pr(1-CH₂SMe) | C(=O)Me | Me | H | O |
| C-16092 | Me(S) | single bond | H | *c*-Pr(1-CH₂SMe) | C(=O)Me | Me | H | O |
| C-16093 | H | single bond | Me | *c*-Pr[1-CH₂S(=O)Me] | C(=O)Me | Cl | H | O |
| C-16094 | Me | single bond | H | *c*-Pr[1-CH₂S(=O)Me] | C(=O)Me | Cl | H | O |
| C-16095 | Me(R) | single bond | H | *c*-Pr[1-CH₂S(=O)Me] | C(=O)Me | Cl | H | O |
| C-16096 | Me(S) | single bond | H | *c*-Pr[1-CH₂S(=O)Me] | C(=O)Me | Cl | H | O |
| C-16097 | H | single bond | Me | *c*-Pr[1-CH₂S(=O)Me] | C(=O)Me | Me | H | O |
| C-16098 | Me | single bond | H | *c*-Pr[1-CH₂S(=O)Me] | C(=O)Me | Me | H | O |
| C-16099 | Me(R) | single bond | H | *c*-Pr[1-CH₂S(=O)Me] | C(=O)Me | Me | H | O |
| C-16100 | Me(S) | single bond | H | *c*-Pr[1-CH₂S(=O)Me] | C(=O)Me | Me | H | O |
| C-16101 | H | single bond | Me | *c*-Pr[1-CH₂S(=O)₂Me] | C(=O)Me | Cl | H | O |
| C-16102 | Me | single bond | H | *c*-Pr[1-CH₂S(=O)₂Me] | C(=O)Me | Cl | H | O |
| C-16103 | Me(R) | single bond | H | *c*-Pr[1-CH₂S(=O)₂Me] | C(=O)Me | Cl | H | O |
| C-16104 | Me(S) | single bond | H | *c*-Pr[1-CH₂S(=O)₂Me] | C(=O)Me | Cl | H | O |
| C-16105 | H | single bond | Me | *c*-Pr[1-CH₂S(=O)₂Me] | C(=O)Me | Me | H | O |
| C-16106 | Me | single bond | H | *c*-Pr[1-CH₂S(=O)₂Me] | C(=O)Me | Me | H | O |
| C-16107 | Me(R) | single bond | H | *c*-Pr[1-CH₂S(=O)₂Me] | C(=O)Me | Me | H | O |
| C-16108 | Me(S) | single bond | H | *c*-Pr[1-CH₂S(=O)₂Me] | C(=O)Me | Me | H | O |
| C-16109 | H | single bond | Me | *c*-Pr(1-CH₂F) | C(=O)Me | Cl | H | O |
| C-16110 | Me | single bond | H | *c*-Pr(1-CH₂F) | C(=O)Me | Cl | H | O |
| C-16111 | Me(R) | single bond | H | *c*-Pr(1-CH₂F) | C(=O)Me | Cl | H | O |
| C-16112 | Me(S) | single bond | H | *c*-Pr(1-CH₂F) | C(=O)Me | Cl | H | O |
| C-16113 | H | single bond | Me | *c*-Pr(1-CH₂F) | C(=O)Me | Me | H | O |
| C-16114 | Me | single bond | H | *c*-Pr(1-CH₂F) | C(=O)Me | Me | H | O |
| C-16115 | Me(R) | single bond | H | *c*-Pr(1-CH₂F) | C(=O)Me | Me | H | O |
| C-16116 | Me(S) | single bond | H | *c*-Pr(1-CH₂F) | C(=O)Me | Me | H | O |
| C-16117 | H | single bond | Me | *c*-Pr(1-CHF₂) | C(=O)Me | Cl | H | O |
| C-16118 | Me | single bond | H | *c*-Pr(1-CHF₂) | C(=O)Me | Cl | H | O |
| C-16119 | Me(R) | single bond | H | *c*-Pr(1-CHF₂) | C(=O)Me | Cl | H | O |
| C-16120 | Me(S) | single bond | H | *c*-Pr(1-CHF₂) | C(=O)Me | Cl | H | O |
| C-16121 | H | single bond | Me | *c*-Pr(1-CHF₂) | C(=O)Me | Me | H | O |
| C-16122 | Me | single bond | H | *c*-Pr(1-CHF₂) | C(=O)Me | Me | H | O |

**[Table 595]**

| Compound Number | Y | W | Z | R¹ | R² | R³ | R¹⁰ | A |
|---|---|---|---|---|---|---|---|---|
| C-16123 | Me(R) | single bond | H | *c*-Pr(1-CHF₂) | C(=O)Me | Me | H | O |
| C-16124 | Me(S) | single bond | H | *c*-Pr(1-CHF₂) | C(=O)Me | Me | H | O |
| C-16125 | H | single bond | Me | *c*-Pr(1-CF₃) | C(=O)Me | Cl | H | O |
| C-16126 | Me | single bond | H | *c*-Pr(1-CF₃) | C(=O)Me | Cl | H | O |
| C-16127 | Me(R) | single bond | H | *c*-Pr(1-CF₃) | C(=O)Me | Cl | H | O |
| C-16128 | Me(S) | single bond | H | *c*-Pr(1-CF₃) | C(=O)Me | Cl | H | O |
| C-16129 | H | single bond | Me | *c*-Pr(1-CF₃) | C(=O)Me | Me | H | O |
| C-16130 | Me | single bond | H | *c*-Pr(1-CF₃) | C(=O)Me | Me | H | O |
| C-16131 | Me(R) | single bond | H | *c*-Pr(1-CF₃) | C(=O)Me | Me | H | O |
| C-16132 | Me(S) | single bond | H | *c*-Pr(1-CF₃) | C(=O)Me | Me | H | O |
| C-16133 | H | single bond | Me | *c*-Pr(1-C≡CH) | C(=O)Me | Cl | H | O |
| C-16134 | Me | single bond | H | *c*-Pr(1-C≡CH) | C(=O)Me | Cl | H | O |
| C-16135 | Me(R) | single bond | H | *c*-Pr(1-C≡CH) | C(=O)Me | Cl | H | O |
| C-16136 | Me(S) | single bond | H | *c*-Pr(1-C≡CH) | C(=O)Me | Cl | H | O |
| C-16137 | H | single bond | Me | *c*-Pr(1-C≡CH) | C(=O)Me | Me | H | O |
| C-16138 | Me | single bond | H | *c*-Pr(1-C≡CH) | C(=O)Me | Me | H | O |
| C-16139 | Me(R) | single bond | H | *c*-Pr(1-C≡CH) | C(=O)Me | Me | H | O |
| C-16140 | Me(S) | single bond | H | *c*-Pr(1-C≡CH) | C(=O)Me | Me | H | O |
| C-16141 | H | single bond | Me | c -Pr[1-C(=O)OEt] | C(=O)Me | Cl | H | O |
| C-16142 | Me | single bond | H | c -Pr[1-C(=O)OEt] | C(=O)Me | Cl | H | O |
| C-16143 | Me(R) | single bond | H | c -Pr[1-C(=O)OEt] | C(=O)Me | Cl | H | O |
| C-16144 | Me(S) | single bond | H | c -Pr[1-C(=O)OEt] | C(=O)Me | Cl | H | O |
| C-16145 | H | single bond | Me | *c*-Pr[1-C(=O)OEt] | C(=O)Me | Me | H | O |
| C-16146 | Me | single bond | H | c -Pr[1-C(=O)OEt] | C(=O)Me | Me | H | O |
| C-16147 | Me(R) | single bond | H | c -Pr[1-C(=O)OEt] | C(=O)Me | Me | H | O |
| C-16148 | Me(S) | single bond | H | c -Pr[1-C(=O)OEt] | C(=O)Me | Me | H | O |
| C-16149 | H | single bond | Me | c -Pr[1-C(=O)OH] | C(=O)Me | Cl | H | O |
| C-16150 | Me | single bond | H | c -Pr[1-C(=O)OH] | C(=O)Me | Cl | H | O |
| C-16151 | Me(R) | single bond | H | c -Pr[1-C(=O)OH] | C(=O)Me | Cl | H | O |
| C-16152 | Me(S) | single bond | H | c -Pr[1-C(=O)OH] | C(=O)Me | Cl | H | O |
| C-16153 | H | single bond | Me | c -Pr[1-C(=O)OH] | C(=O)Me | Me | H | O |
| C-16154 | Me | single bond | H | c -Pr[1-C(=O)OH] | C(=O)Me | Me | H | O |
| C-16155 | Me(R) | single bond | H | c -Pr[1-C(=O)OH] | C(=O)Me | Me | H | O |
| C-16156 | Me(S) | single bond | H | c -Pr[1-C(=O)OH] | C(=O)Me | Me | H | O |
| C-16157 | H | single bond | Me | *c*-Pr[1-C(=O)NH₂] | C(=O)Me | Cl | H | O |
| C-16158 | Me | single bond | H | *c*-Pr[1-C(=O)NH₂] | C(=O)Me | Cl | H | O |
| C-16159 | Me(R) | single bond | H | *c*-Pr[1-C(=O)NH₂] | C(=O)Me | Cl | H | O |
| C-16160 | Me(S) | single bond | H | *c*-Pr[1-C(=O)NH₂] | C(=O)Me | Cl | H | O |
| C-16161 | H | single bond | Me | *c*-Pr[1-C(=O)NH₂] | C(=O)Me | Me | H | O |
| C-16162 | Me | single bond | H | *c*-Pr[1-C(=O)NH₂] | C(=O)Me | Me | H | O |
| C-16163 | Me(R) | single bond | H | *c*-Pr[1-C(=O)NH₂] | C(=O)Me | Me | H | O |
| C-16164 | Me(S) | single bond | H | *c*-Pr[1-C(=O)NH₂] | C(=O)Me | Me | H | O |
| C-16165 | H | single bond | Me | *c*-Pr(1-OMe) | C(=O)Me | Cl | H | O |
| C-16166 | Me | single bond | H | *c*-Pr(1-OMe) | C(=O)Me | Cl | H | O |

**[Table 596]**

| Compound Number | Y | W | Z | R¹ | R² | R³ | R¹⁰ | A |
|---|---|---|---|---|---|---|---|---|
| C-16167 | Me(R) | single bond | H | *c*-Pr(1-OMe) | C(=O)Me | Cl | H | O |
| C-16168 | Me(S) | single bond | H | *c*-Pr(1-OMe) | C(=O)Me | Cl | H | O |
| C-16169 | H | single bond | Me | *c*-Pr(1-OMe) | C(=O)Me | Me | H | O |
| C-16170 | Me | single bond | H | *c*-Pr(1-OMe) | C(=O)Me | Me | H | O |
| C-16171 | Me(R) | single bond | H | *c*-Pr(1-OMe) | C(=O)Me | Me | H | O |
| C-16172 | Me(S) | single bond | H | *c*-Pr(1-OMe) | C(=O)Me | Me | H | O |
| C-16173 | H | single bond | Me | *c*-Pr(1-SMe) | C(=O)Me | Cl | H | O |
| C-16174 | Me | single bond | H | *c*-Pr(1-SMe) | C(=O)Me | Cl | H | O |
| C-16175 | Me(R) | single bond | H | *c*-Pr(1-SMe) | C(=O)Me | Cl | H | O |
| C-16176 | Me(S) | single bond | H | *c*-Pr(1-SMe) | C(=O)Me | Cl | H | O |
| C-16177 | H | single bond | Me | *c*-Pr(1-SMe) | C(=O)Me | Me | H | O |
| C-16178 | Me | single bond | H | *c*-Pr(1-SMe) | C(=O)Me | Me | H | O |
| C-16179 | Me(R) | single bond | H | *c*-Pr(1-SMe) | C(=O)Me | Me | H | O |
| C-16180 | Me(S) | single bond | H | *c*-Pr(1-SMe) | C(=O)Me | Me | H | O |
| C-16181 | H | single bond | Me | c -Pr[1-S(=O)Me] | C(=O)Me | Cl | H | O |
| C-16182 | Me | single bond | H | c -Pr[1-S(=O)Me] | C(=O)Me | Cl | H | O |
| C-16183 | Me(R) | single bond | H | c -Pr[1-S(=O)Me] | C(=O)Me | Cl | H | O |
| C-16184 | Me(S) | single bond | H | c -Pr[1-S(=O)Me] | C(=O)Me | Cl | H | O |
| C-16185 | H | single bond | Me | c -Pr[1-S(=O)Me] | C(=O)Me | Me | H | O |
| C-16186 | Me | single bond | H | c -Pr[1-S(=O)Me] | C(=O)Me | Me | H | O |
| C-16187 | Me(R) | single bond | H | c -Pr[1-S(=O)Me] | C(=O)Me | Me | H | O |
| C-16188 | Me(S) | single bond | H | c -Pr[1-S(=O)Me] | C(=O)Me | Me | H | O |
| C-16189 | H | single bond | Me | *c*-Pr[1-S(=O)₂Me] | C(=O)Me | Cl | H | O |
| C-16190 | Me | single bond | H | *c*-Pr[1-S(=O)₂Me] | C(=O)Me | Cl | H | O |
| C-16191 | Me(R) | single bond | H | *c*-Pr[1-S(=O)₂Me] | C(=O)Me | Cl | H | O |
| C-16192 | Me(S) | single bond | H | *c*-Pr[1-S(=O)₂Me] | C(=O)Me | Cl | H | O |
| C-16193 | H | single bond | Me | *c*-Pr[1-S(=O)₂Me] | C(=O)Me | Me | H | O |
| C-16194 | Me | single bond | H | *c*-Pr[1-S(=O)₂Me] | C(=O)Me | Me | H | O |
| C-16195 | Me(R) | single bond | H | *c*-Pr[1-S(=O)₂Me] | C(=O)Me | Me | H | O |
| C-16196 | Me(S) | single bond | H | *c*-Pr[1-S(=O)₂Me] | C(=O)Me | Me | H | O |
| C-16197 | H | single bond | Me | *c*-Pr[1-S(=O)₂CHF₂] | C(=O)Me | Cl | H | O |
| C-16198 | Me | single bond | H | *c*-Pr[1-S(=O)₂CHF₂] | C(=O)Me | Cl | H | O |
| C-16199 | Me(R) | single bond | H | *c*-Pr[1-S(=O)₂CHF₂] | C(=O)Me | Cl | H | O |
| C-16200 | Me(S) | single bond | H | *c*-Pr[1-S(=O)₂CHF₂] | C(=O)Me | Cl | H | O |
| C-16201 | H | single bond | Me | *c*-Pr[1-S(=O)₂CHF₂] | C(=O)Me | Me | H | O |
| C-16202 | Me | single bond | H | *c*-Pr[1-S(=O)₂CHF₂] | C(=O)Me | Me | H | O |
| C-16203 | Me(R) | single bond | H | *c*-Pr[1-S(=O)₂CHF₂] | C(=O)Me | Me | H | O |
| C-16204 | Me(S) | single bond | H | *c*-Pr[1-S(=O)₂CHF₂] | C(=O)Me | Me | H | O |
| C-16205 | H | single bond | Me | *c*-Pr[1-S(=O)₂CF₃] | C(=O)Me | Cl | H | O |
| C-16206 | Me | single bond | H | *c*-Pr[1-S(=O)₂CF₃] | C(=O)Me | Cl | H | O |
| C-16207 | Me(R) | single bond | H | *c*-Pr[1-S(=O)₂CF₃] | C(=O)Me | Cl | H | O |
| C-16208 | Me(S) | single bond | H | *c*-Pr[1-S(=O)₂CF₃] | C(=O)Me | Cl | H | O |
| C-16209 | H | single bond | Me | *c*-Pr[1-S(=O)₂CF₃] | C(=O)Me | Me | H | O |
| C-16210 | Me | single bond | H | *c*-Pr[1-S(=O)₂CF₃] | C(=O)Me | Me | H | O |

**[Table 597]**

| Compound Number | Y | W | Z | R¹ | R² | R³ | R¹⁰ | A |
|---|---|---|---|---|---|---|---|---|
| C-16211 | Me(R) | single bond | H | *c*-Pr[1-S(=O)₂CF₃] | C(=O)Me | Me | H | O |
| C-16212 | Me(S) | single bond | H | *c*-Pr[1-S(=O)₂CF₃] | C(=O)Me | Me | H | O |
| C-16213 | H | single bond | Me | *c*-Pr[1-(5,6-dihydro-1,4,2-dioxazin-3-yl)] | C(=O)Me | Cl | H | O |
| C-16214 | Me | single bond | H | *c*-Pr[1-(5,6-dihydro-1,4,2-dioxazin-3-yl)] | C(=O)Me | Cl | H | O |
| C-16215 | Me(R) | single bond | H | *c*-Pr[1-(5,6-dihydro-1,4,2-dioxazin-3-yl)] | C(=O)Me | Cl | H | O |
| C-16216 | Me(S) | single bond | H | c-Pr[1-(5,6-dihydro-1,4,2-dioxazin-3-yl)] | C(=O)Me | Cl | H | O |
| C-16217 | H | single bond | Me | *c*-Pr[1-(5,6-dihydro-1,4,2-dioxazin-3-yl)] | C(=O)Me | Me | H | O |
| C-16218 | Me | single bond | H | *c*-Pr[1-(5,6-dihydro-1,4,2-dioxazin-3-yl)] | C(=O)Me | Me | H | O |
| C-16219 | Me(R) | single bond | H | *c*-Pr[1-(5,6-dihydro-1,4,2-dioxazin-3-yl)] | C(=O)Me | Me | H | O |
| C-16220 | Me(S) | single bond | H | *c*-Pr[1-(5,6-dihydro-1,4,2-dioxazin-3-yl)] | C(=O)Me | Me | H | O |
| C-16221 | H | single bond | Me | *c*-Pr[1-(5-hydroxy-1,3,4-oxadiazol-2-yl)] | C(=O)Me | Cl | H | O |
| C-16222 | Me | single bond | H | *c*-Pr[1-(5-hydroxy-1,3,4-oxadiazol-2-yl)] | C(=O)Me | Cl | H | O |
| C-16223 | Me(R) | single bond | H | *c*-Pr[1-(5-hydroxy-1,3,4-oxadiazol-2-yl)] | C(=O)Me | Cl | H | O |
| C-16224 | Me(S) | single bond | H | *c*-Pr[1-(5-hydroxy-1,3,4-oxadiazol-2-yl)] | C(=O)Me | Cl | H | O |
| C-16225 | H | single bond | Me | *c*-Pr[1-(5-hydroxy-1,3,4-oxadiazol-2-yl)] | C(=O)Me | Me | H | O |
| C-16226 | Me | single bond | H | *c*-Pr[1-(5-hydroxy-1,3,4-oxadiazol-2-yl)] | C(=O)Me | Me | H | O |
| C-16227 | Me(R) | single bond | H | *c*-Pr[1-(5-hydroxy-1,3,4-oxadiazol-2-yl)] | C(=O)Me | Me | H | O |
| C-16228 | Me(S) | single bond | H | *c*-Pr[1-(5-hydroxy-1,3,4-oxadiazol-2-yl)] | C(=O)Me | Me | H | O |
| C-16229 | H | single bond | Me | c -Pr[1-(pyrimidin-2-yl)] | C(=O)Me | Cl | H | O |
| C-16230 | Me | single bond | H | c -Pr[1-(pyrimidin-2-yl)] | C(=O)Me | Cl | H | O |
| C-16231 | Me(R) | single bond | H | c -Pr[1-(pyrimidin-2-yl)] | C(=O)Me | Cl | H | O |
| C-16232 | Me(S) | single bond | H | c -Pr[1-(pyrimidin-2-yl)] | C(=O)Me | Cl | H | O |
| C-16233 | H | single bond | Me | c -Pr[1-(pyrimidin-2-yl)] | C(=O)Me | Me | H | O |
| C-16234 | Me | single bond | H | c -Pr[1-(pyrimidin-2-yl)] | C(=O)Me | Me | H | O |
| C-16235 | Me(R) | single bond | H | c -Pr[1-(pyrimidin-2-yl)] | C(=O)Me | Me | H | O |
| C-16236 | Me(S) | single bond | H | c -Pr[1-(pyrimidin-2-yl)] | C(=O)Me | Me | H | O |
| C-16237 | H | single bond | Me | t-Bu | C(=O)Me | Cl | H | O |
| C-16238 | Me | single bond | H | t-Bu | C(=O)Me | Cl | H | O |
| C-16239 | Me(R) | single bond | H | t-Bu | C(=O)Me | Cl | H | O |
| C-16240 | Me(S) | single bond | H | t-Bu | C(=O)Me | Cl | H | O |
| C-16241 | H | single bond | Me | t-Bu | C(=O)Me | Me | H | O |
| C-16242 | Me | single bond | H | t-Bu | C(=O)Me | Me | H | O |
| C-16243 | Me(R) | single bond | H | t-Bu | C(=O)Me | Me | H | O |
| C-16244 | Me(S) | single bond | H | t-Bu | C(=O)Me | Me | H | O |
| C-16245 | H | single bond | Me | c-Bu | C(=O)Me | Cl | H | O |
| C-16246 | Me | single bond | H | c-Bu | C(=O)Me | Cl | H | O |
| C-16247 | Me(R) | single bond | H | c-Bu | C(=O)Me | Cl | H | O |
| C-16248 | Me(S) | single bond | H | c-Bu | C(=O)Me | Cl | H | O |
| C-16249 | H | single bond | Me | c-Bu | C(=O)Me | Me | H | O |
| C-16250 | Me | single bond | H | c-Bu | C(=O)Me | Me | H | O |
| C-16251 | Me(R) | single bond | H | c-Bu | C(=O)Me | Me | H | O |
| C-16252 | Me(S) | single bond | H | c-Bu | C(=O)Me | Me | H | O |
| C-16253 | H | single bond | Me | c-Bu(1-CN) | C(=O)Me | Cl | H | O |
| C-16254 | Me | single bond | H | *c-*Bu(1-CN) | C(=O)Me | Cl | H | O |

**[Table 598]**

| Compound Number | Y | W | Z | R¹ | R² | R³ | R¹⁰ | A |
|---|---|---|---|---|---|---|---|---|
| C-16255 | Me(R) | single bond | H | c-Bu(1-CN) | C(=O)Me | Cl | H | O |
| C-16256 | Me(S) | single bond | H | c-Bu(1-CN) | C(=O)Me | Cl | H | O |
| C-16257 | H | single bond | Me | c-Bu(1-CN) | C(=O)Me | Me | H | O |
| C-16258 | Me | single bond | H | c-Bu(1-CN) | C(=O)Me | Me | H | O |
| C-16259 | Me(R) | single bond | H | c-Bu(1-CN) | C(=O)Me | Me | H | O |
| C-16260 | Me(S) | single bond | H | *c*-Bu(1-CN) | C(=O)Me | Me | H | O |
| C-16261 | H | single bond | Me | *c*-Bu(3,3-F₂) | C(=O)Me | Cl | H | O |
| C-16262 | Me | single bond | H | *c*-Bu(3,3-F₂) | C(=O)Me | Cl | H | O |
| C-16263 | Me(R) | single bond | H | *c*-Bu(3,3-F₂) | C(=O)Me | Cl | H | O |
| C-16264 | Me(S) | single bond | H | *c*-Bu(3,3-F₂) | C(=O)Me | Cl | H | O |
| C-16265 | H | single bond | Me | *c*-Bu(3,3-F₂) | C(=O)Me | Me | H | O |
| C-16266 | Me | single bond | H | *c*-Bu(3,3-F₂) | C(=O)Me | Me | H | O |
| C-16267 | Me(R) | single bond | H | *c*-Bu(3,3-F₂) | C(=O)Me | Me | H | O |
| C-16268 | Me(S) | single bond | H | *c*-Bu(3,3-F₂) | C(=O)Me | Me | H | O |
| C-16269 | H | single bond | Me | oxetan-3-yl | C(=O)Me | Cl | H | O |
| C-16270 | Me | single bond | H | oxetan-3-yl | C(=O)Me | Cl | H | O |
| C-16271 | Me(R) | single bond | H | oxetan-3-yl | C(=O)Me | Cl | H | O |
| C-16272 | Me(S) | single bond | H | oxetan-3-yl | C(=O)Me | Cl | H | O |
| C-16273 | H | single bond | Me | oxetan-3-yl | C(=O)Me | Me | H | O |
| C-16274 | Me | single bond | H | oxetan-3-yl | C(=O)Me | Me | H | O |
| C-16275 | Me(R) | single bond | H | oxetan-3-yl | C(=O)Me | Me | H | O |
| C-16276 | Me(S) | single bond | H | oxetan-3-yl | C(=O)Me | Me | H | O |
| C-16277 | H | single bond | Me | oxetan(3-CN)-3-yl | C(=O)Me | Cl | H | O |
| C-16278 | Me | single bond | H | oxetan(3-CN)-3-yl | C(=O)Me | Cl | H | O |
| C-16279 | Me(R) | single bond | H | oxetan(3-CN)-3-yl | C(=O)Me | Cl | H | O |
| C-16280 | Me(S) | single bond | H | oxetan(3-CN)-3-yl | C(=O)Me | Cl | H | O |
| C-16281 | H | single bond | Me | oxetan(3-CN)-3-yl | C(=O)Me | Me | H | O |
| C-16282 | Me | single bond | H | oxetan(3-CN)-3-yl | C(=O)Me | Me | H | O |
| C-16283 | Me(R) | single bond | H | oxetan(3-CN)-3-yl | C(=O)Me | Me | H | O |
| C-16284 | Me(S) | single bond | H | oxetan(3-CN)-3-yl | C(=O)Me | Me | H | O |
| C-16285 | H | single bond | Me | azetidin[1-C(=O)Me]-3-yl | C(=O)Me | Cl | H | O |
| C-16286 | Me | single bond | H | azetidin[1-C(=O)Me]-3-yl | C(=O)Me | Cl | H | O |
| C-16287 | Me(R) | single bond | H | azetidin[1-C(=O)Me]-3-yl | C(=O)Me | Cl | H | O |
| C-16288 | Me(S) | single bond | H | azetidin[1-C(=O)Me]-3-yl | C(=O)Me | Cl | H | O |
| C-16289 | H | single bond | Me | azetidin[1-C(=O)Me]-3-yl | C(=O)Me | Me | H | O |
| C-16290 | Me | single bond | H | azetidin[1-C(=O)Me]-3-yl | C(=O)Me | Me | H | O |
| C-16291 | Me(R) | single bond | H | azetidin[1-C(=O)Me]-3-yl | C(=O)Me | Me | H | O |
| C-16292 | Me(S) | single bond | H | azetidin[1-C(=O)Me]-3-yl | C(=O)Me | Me | H | O |
| C-16293 | H | single bond | Me | azetidin[1-S(=O)₂Me]-3-yl | C(=O)Me | Cl | H | O |
| C-16294 | Me | single bond | H | azetidin[1-S(=O)₂Me]-3-yl | C(=O)Me | Cl | H | O |
| C-16295 | Me(R) | single bond | H | azetidin[1-S(=O)₂Me]-3-yl | C(=O)Me | Cl | H | O |
| C-16296 | Me(S) | single bond | H | azetidin[1-S(=O)₂Me]-3-yl | C(=O)Me | Cl | H | O |
| C-16297 | H | single bond | Me | azetidin[1-S(=O)₂Me]-3-yl | C(=O)Me | Me | H | O |
| C-16298 | Me | single bond | H | azetidin[1-S(=O)₂Me]-3-yl | C(=O)Me | Me | H | O |

**[Table 599]**

| Compound Number | Y | W | Z | R¹ | R² | R³ | R¹⁰ | A . |
|---|---|---|---|---|---|---|---|---|
| C-16299 | Me(R) | single bond | H | azetidin[1-S(=O)₂Me]-3-yl | C(=O)Me | Me | H | O |
| C-16300 | Me(S) | single bond | H | azetidin[1-S(=O)₂Me]-3-yl | C(=O)Me | Me | H | O |
| C-16301 | H | single bond | Me | azetidin[1-S(=O)₂CHF₂]-3-yl | C(=O)Me | Cl | H | O |
| C-16302 | Me | single bond | H | azetidin[1-S(=O)₂CHF₂]-3-yl | C(=O)Me | Cl | H | O |
| C-16303 | Me(R) | single bond | H | azetidin[1-S(=O)₂CHF₂]-3-yl | C(=O)Me | Cl | H | O |
| C-16304 | Me(S) | single bond | H | azetidin[1-S(=O)₂CHF₂]-3-yl | C(=O)Me | Cl | H | O |
| C-16305 | H | single bond | Me | azetidin[1-S(=O)₂CHF₂]-3-yl | C(=O)Me | Me | H | O |
| C-16306 | Me | single bond | H | azetidin[1-S(=O)₂CHF₂]-3-yl | C(=O)Me | Me | H | O |
| C-16307 | Me(R) | single bond | H | azetidin[1-S(=O)₂CHF₂]-3-yl | C(=O)Me | Me | H | O |
| C-16308 | Me(S) | single bond | H | azetidin[1-S(=O)₂CHF₂]-3-yl | C(=O)Me | Me | H | O |
| C-16309 | H | single bond | Me | c-Pen | C(=O)Me | Cl | H | O |
| C-16310 | Me | single bond | H | *c*-Pen | C(=O)Me | Cl | H | O |
| C-16311 | Me(R) | single bond | H | *c*-Pen | C(=O)Me | Cl | H | O |
| C-16312 | Me(S) | single bond | H | c-Pen | C(=O)Me | Cl | H | O |
| C-16313 | H | single bond | Me | *c*-Pen | C(=O)Me | Me | H | O |
| C-16314 | Me | single bond | H | *c*-Pen | C(=O)Me | Me | H | O |
| C-16315 | Me(R) | single bond | H | *c*-Pen | C(=O)Me | Me | H | O |
| C-16316 | Me(S) | single bond | H | *c*-Pen | C(=O)Me | Me | H | O |
| C-16317 | H | single bond | Me | tetrahydrofuran-3-yl | C(=O)Me | Cl | H | O |
| C-16318 | Me | single bond | H | tetrahydrofuran-3-yl | C(=O)Me | Cl | H | O |
| C-16319 | Me(R) | single bond | H | tetrahydrofuran-3-yl | C(=O)Me | Cl | H | O |
| C-16320 | Me(S) | single bond | H | tetra hydrofuran-3-yl | C(=O)Me | Cl | H | O |
| C-16321 | H | single bond | Me | tetrahydrofuran-3-yl | C(=O)Me | Me | H | O |
| C-16322 | Me | single bond | H | tetrahydrofuran-3-yl | C(=O)Me | Me | H | O |
| C-16323 | Me(R) | single bond | H | tetrahydrofuran-3-yl | C(=O)Me | Me | H | O |
| C-16324 | Me(S) | single bond | H | tetrahydrofuran-3-yl | C(=O)Me | Me | H | O |
| C-16325 | H | single bond | Me | tetrahydrothiophen-3-yl | C(=O)Me | Cl | H | O |
| C-16326 | Me | single bond | H | tetrahydrothiophen-3-yl | C(=O)Me | Cl | H | O |
| C-16327 | Me(R) | single bond | H | tetrahydrothiophen-3-yl | C(=O)Me | Cl | H | O |
| C-16328 | Me(S) | single bond | H | tetrahydrothiophen-3-yl | C(=O)Me | Cl | H | O |
| C-16329 | H | single bond | Me | tetrahydrothiophen-3-yl | C(=O)Me | Me | H | O |
| C-16330 | Me | single bond | H | tetrahydrothiophen-3-yl | C(=O)Me | Me | H | O |
| C-16331 | Me(R) | single bond | H | tetrahydrothiophen-3-yl | C(=O)Me | Me | H | O |
| C-16332 | Me(S) | single bond | H | tetrahydrothiophen-3-yl | C(=O)Me | Me | H | O |
| C-16333 | H | single bond | Me | c-Pen(1-CN) | C(=O)Me | Cl | H | O |
| C-16334 | Me | single bond | H | c-Pen(1-CN) | C(=O)Me | Cl | H | O |
| C-16335 | Me(R) | single bond | H | c -Pen(1-CN) | C(=O)Me | Cl | H | O |
| C-16336 | Me(S) | single bond | H | *c*-Pen(1-CN) | C(=O)Me | Cl | H | O |
| C-16337 | H | single bond | Me | c-Pen(1-CN) | C(=O)Me | Me | H | O |
| C-16338 | Me | single bond | H | *c*-Pen(1-CN) | C(=O)Me | Me | H | O |
| C-16339 | Me(R) | single bond | H | c-Pen(1-CN) | C(=O)Me | Me | H | O |
| C-16340 | Me(S) | single bond | H | *c*-Pen(1-CN) | C(=O)Me | Me | H | O |
| C-16341 | H | single bond | Me | tetrahydrofuran(3-CN)-3-yl | C(=O)Me | Cl | H | O |
| C-16342 | Me | single bond | H | tetrahydrofuran(3-CN)-3-yl | C(=O)Me | Cl | H | O |

**[Table 600]**

| Compound Number | Y | W | Z | R¹ | R² | R³ | R¹⁰ | A |
|---|---|---|---|---|---|---|---|---|
| C-16343 | Me(R) | single bond | H | tetrahydrofuran(3-CN)-3-yl | C(=O)Me | Cl | H | O |
| C-16344 | Me(S) | single bond | H | tetrahydrofuran(3-CN)-3-yl | C(=O)Me | Cl | H | O |
| C-16345 | H | single bond | Me | tetrahydrofuran(3-CN)-3-yl | C(=O)Me | Me | H | O |
| C-16346 | Me | single bond | H | tetrahydrofuran(3-CN)-3-yl | C(=O)Me | Me | H | O |
| C-16347 | Me(R) | single bond | H | tetrahydrofuran(3-CN)-3-yl | C(=O)Me | Me | H | O |
| C-16348 | Me(S) | single bond | H | tetrahydrofuran(3-CN)-3-yl | C(=O)Me | Me | H | O |
| C-16349 | H | single bond | Me | tetrahydrothiophen(3-CN)-3-yl | C(=O)Me | Cl | H | O |
| C-16350 | Me | single bond | H | tetrahydrothiophen(3-CN)-3-yl | C(=O)Me | Cl | H | O |
| C-16351 | Me(R) | single bond | H | tetrahydrothiophen(3-CN)-3-yl | C(=O)Me | Cl | H | O |
| C-16352 | Me(S) | single bond | H | tetrahydrothiophen(3-CN)-3-yl | C(=O)Me | Cl | H | O |
| C-16353 | H | single bond | Me | tetrahydrothiophen(3-CN)-3-yl | C(=O)Me | Me | H | O |
| C-16354 | Me | single bond | H | tetrahydrothiophen(3-CN)-3-yl | C(=O)Me | Me | H | O |
| C-16355 | Me(R) | single bond | H | tetrahydrothiophen(3-CN)-3-yl | C(=O)Me | Me | H | O |
| C-16356 | Me(S) | single bond | H | tetrahydrothiophen(3-CN)-3-yl | C(=O)Me | Me | H | O |
| C-16357 | H | single bond | Me | bicyclo[1.1.1]pentan-1-yl | C(=O)Me | Cl | H | O |
| C-16358 | Me | single bond | H | bicyclo[1.1.1]pentan-1-yl | C(=O)Me | Cl | H | O |
| C-16359 | Me(R) | single bond | H | bicyclo[1.1.1]pentan-1-yl | C(=O)Me | Cl | H | O |
| C-16360 | Me(S) | single bond | H | bicyclo[1.1.1]pentan-1-yl | C(=O)Me | Cl | H | O |
| C-16361 | H | single bond | Me | bicyclo[1.1.1]pentan-1-yl | C(=O)Me | Me | H | O |
| C-16362 | Me | single bond | H | bicyclo[1.1.1]pentan-1-yl | C(=O)Me | Me | H | O |
| C-16363 | Me(R) | single bond | H | bicyclo[1.1.1]pentan-1-yl | C(=O)Me | Me | H | O |
| C-16364 | Me(S) | single bond | H | bicyclo[1.1.1]pentan-1-yl | C(=O)Me | Me | H | O |
| C-16365 | H | single bond | Me | *c*-Hex | C(=O)Me | Cl | H | O |
| C-16366 | Me | single bond | H | *c*-Hex | C(=O)Me | Cl | H | O |
| C-16367 | Me(R) | single bond | H | *c*-Hex | C(=O)Me | Cl | H | O |
| C-16368 | Me(S) | single bond | H | *c*-Hex | C(=O)Me | Cl | H | O |
| C-16369 | H | single bond | Me | *c*-Hex | C(=O)Me | Me | H | O |
| C-16370 | Me | single bond | H | *c*-Hex | C(=O)Me | Me | H | O |
| C-16371 | Me(R) | single bond | H | *c*-Hex | C(=O)Me | Me | H | O |
| C-16372 | Me(S) | single bond | H | c-Hex | C(=O)Me | Me | H | O |
| C-16373 | H | single bond | Me | tetrahydropyran-4-yl | C(=O)Me | Cl | H | O |
| C-16374 | Me | single bond | H | tetrahydropyran-4-yl | C(=O)Me | Cl | H | O |
| C-16375 | Me(R) | single bond | H | tetrahydropyran-4-yl | C(=O)Me | Cl | H | O |
| C-16376 | Me(S) | single bond | H | tetrahydropyran-4-yl | C(=O)Me | Cl | H | O |
| C-16377 | H | single bond | Me | tetrahydropyran-4-yl | C(=O)Me | Me | H | O |
| C-16378 | Me | single bond | H | tetrahydropyran-4-yl | C(=O)Me | Me | H | O |
| C-16379 | Me(R) | single bond | H | tetrahydropyran-4-yl | C(=O)Me | Me | H | O |
| C-16380 | Me(S) | single bond | H | tetrahydropyran-4-yl | C(=O)Me | Me | H | O |
| C-16381 | H | single bond | Me | tetrahydrothiopyran-4-yl | C(=O)Me | Cl | H | O |
| C-16382 | Me | single bond | H | tetrahydrothiopyran-4-yl | C(=O)Me | Cl | H | O |
| C-16383 | Me(R) | single bond | H | tetrahydrothiopyran-4-yl | C(=O)Me | Cl | H | O |
| C-16384 | Me(S) | single bond | H | tetrahydrothiopyran-4-yl | C(=O)Me | Cl | H | O |
| C-16385 | H | single bond | Me | tetrahydrothiopyran-4-yl | C(=O)Me | Me | H | O |
| C-16386 | Me | single bond | H | tetrahydrothiopyran-4-yl | C(=O)Me | Me | H | O |

**[Table 601]**

| Compound Number | Y | W | Z | R¹ | R² | R³ | R¹⁰ | A |
|---|---|---|---|---|---|---|---|---|
| C-16387 | Me(R) | single bond | H | tetrahydrothiopyran-4-yl | C(=O)Me | Me | H | O |
| C-16388 | Me(S) | single bond | H | tetrahydrothiopyran-4-yl | C(=O)Me | Me | H | O |
| C-16389 | H | single bond | Me | *c*-Hex(1-CN) | C(=O)Me | Cl | H | O |
| C-16390 | Me | single bond | H | *c*-Hex(1-CN) | C(=O)Me | Cl | H | O |
| C-16391 | Me(R) | single bond | H | *c*-Hex(1-CN) | C(=O)Me | Cl | H | O |
| C-16392 | Me(S) | single bond | H | *c*-Hex(1-CN) | C(=O)Me | Cl | H | O |
| C-16393 | H | single bond | Me | *c*-Hex(1-CN) | C(=O)Me | Me | H | O |
| C-16394 | Me | single bond | H | *c*-Hex(1-CN) | C(=O)Me | Me | H | O |
| C-16395 | Me(R) | single bond | H | *c*-Hex(1-CN) | C(=O)Me | Me | H | O |
| C-16396 | Me(S) | single bond | H | *c*-Hex(1-CN) | C(=O)Me | Me | H | O |
| C-16397 | H | single bond | Me | tetrahydropyran(4-CN)-4-yl | C(=O)Me | Cl | H | O |
| C-16398 | Me | single bond | H | tetrahydropyran(4-CN)-4-yl | C(=O)Me | Cl | H | O |
| C-16399 | Me(R) | single bond | H | tetrahydropyran(4-CN)-4-yl | C(=O)Me | Cl | H | O |
| C-16400 | Me(S) | single bond | H | tetrahydropyran(4-CN)-4-yl | C(=O)Me | Cl | H | O |
| C-16401 | H | single bond | Me | tetrahydropyran(4-CN)-4-yl | C(=O)Me | Me | H | O |
| C-16402 | Me | single bond | H | tetrahydropyran(4-CN)-4-yl | C(=O)Me | Me | H | O |
| C-16403 | Me(R) | single bond | H | tetrahydropyran(4-CN)-4-yl | C(=O)Me | Me | H | O |
| C-16404 | Me(S) | single bond | H | tetrahydropyran(4-CN)-4-yl | C(=O)Me | Me | H | O |
| C-16405 | H | single bond | Me | tetrahydrothiopyran(4-CN)-4-yl | C(=O)Me | Cl | H | O |
| C-16406 | Me | single bond | H | tetrahydrothiopyran(4-CN)-4-yl | C(=O)Me | Cl | H | O |
| C-16407 | Me(R) | single bond | H | tetrahydrothiopyran(4-CN)-4-yl | C(=O)Me | Cl | H | O |
| C-16408 | Me(S) | single bond | H | tetrahydrothiopyran(4-CN)-4-yl | C(=O)Me | Cl | H | O |
| C-16409 | H | single bond | Me | tetrahydrothiopyran(4-CN)-4-yl | C(=O)Me | Me | H | O |
| C-16410 | Me | single bond | H | tetrahydrothiopyran(4-CN)-4-yl | C(=O)Me | Me | H | O |
| C-16411 | Me(R) | single bond | H | tetrahydrothiopyran(4-CN)-4-yl | C(=O)Me | Me | H | O |
| C-16412 | Me(S) | single bond | H | tetrahydrothiopyran(4-CN)-4-yl | C(=O)Me | Me | H | O |
| C-16413 | H | single bond | Me | CH₂(*c*-Pr) | C(=O)Me | Cl | H | O |
| C-16414 | Me | single bond | H | CH₂(*c*-Pr) | C(=O)Me | Cl | H | O |
| C-16415 | Me(R) | single bond | H | CH₂(*c*-Pr) | C(=O)Me | Cl | H | O |
| C-16416 | Me(S) | single bond | H | CH₂(*c*-Pr) | C(=O)Me | Cl | H | O |
| C-16417 | H | single bond | Me | CH₂(*c*-Pr) | C(=O)Me | Me | H | O |
| C-16418 | Me | single bond | H | CH₂(*c*-Pr) | C(=O)Me | Me | H | O |
| C-16419 | Me(R) | single bond | H | CH₂(*c*-Pr) | C(=O)Me | Me | H | O |
| C-16420 | Me(S) | single bond | H | CH₂(*c*-Pr) | C(=O)Me | Me | H | O |
| C-16421 | H | single bond | Me | CH₂[*c*-Pr(1-CN)] | C(=O)Me | Cl | H | O |
| C-16422 | Me | single bond | H | CH₂[*c*-Pr(1-CN)] | C(=O)Me | Cl | H | O |
| C-16423 | Me(R) | single bond | H | CH₂[*c*-Pr(1-CN)] | C(=O)Me | Cl | H | O |
| C-16424 | Me(S) | single bond | H | CH₂[*c*-Pr(1-CN)] | C(=O)Me | Cl | H | O |
| C-16425 | H | single bond | Me | CH₂[*c*-Pr(1-CN)] | C(=O)Me | Me | H | O |
| C-16426 | Me | *single bond* | H | CH₂[*c*-Pr(1-CN)] | C(=O)Me | Me | H | O |
| C-16427 | Me(R) | single bond | H | CH₂[*c*-Pr(1-CN)] | C(=O)Me | Me | H | O |
| C-16428 | Me(S) | single bond | H | CH₂[*c*-Pr(1-CN)] | C(=O)Me | Me | H | O |
| C-16429 | H | single bond | Me | CH(Me)(*c*-Pr) | C(=O)Me | Cl | H | O |
| C-16430 | Me | single bond | H | CH(Me)(*c*-Pr) | C(=O)Me | Cl | H | O |

**[Table 602]**

| Compound Number | Y | W | Z | R¹ | R² | R³ | R¹⁰ | A |
|---|---|---|---|---|---|---|---|---|
| C-16431 | Me(R) | single bond | H | CH(Me)(*c*-Pr) | C(=O)Me | Cl | H | O |
| C-16432 | Me(S) | single bond | H | CH(Me)(*c*-Pr) | C(=O)Me | Cl | H | O |
| C-16433 | H | single bond | Me | CH(Me)(*c*-Pr) | C(=O)Me | Me | H | O |
| C-16434 | Me | single bond | H | CH(Me)(*c*-Pr) | C(=O)Me | Me | H | O |
| C-16435 | Me(R) | single bond | H | CH(Me)(*c*-Pr) | C(=O)Me | Me | H | O |
| C-16436 | Me(S) | single bond | H | CH(Me)(*c*-Pr) | C(=O)Me | Me | H | O |
| C-16437 | H | single bond | Me | CH(Me)CH₂OMe | C(=O)Me | Cl | H | O |
| C-16438 | Me | single bond | H | CH(Me)CH₂OMe | C(=O)Me | Cl | H | O |
| C-16439 | Me(R) | single bond | H | CH(Me)CH₂OMe | C(=O)Me | Cl | H | O |
| C-16440 | Me(S) | single bond | H | CH(Me)CH₂OMe | C(=O)Me | Cl | H | O |
| C-16441 | H | single bond | Me | CH(Me)CH₂OMe | C(=O)Me | Me | H | O |
| C-16442 | Me | single bond | H | CH(Me)CH₂OMe | C(=O)Me | Me | H | O |
| C-16443 | Me(R) | single bond | H | CH(Me)CH₂OMe | C(=O)Me | Me | H | O |
| C-16444 | Me(S) | single bond | H | CH(Me)CH₂OMe | C(=O)Me | Me | H | O |
| C-16445 | H | single bond | Me | CH₂CH=CH₂ | C(=O)Me | Cl | H | O |
| C-16446 | Me | single bond | H | CH₂CH=CH₂ | C(=O)Me | Cl | H | O |
| C-16447 | Me(R) | single bond | H | CH₂CH=CH₂ | C(=O)Me | Cl | H | O |
| C-16448 | Me(S) | single bond | H | CH₂CH=CH₂ | C(=O)Me | Cl | H | O |
| C-16449 | H | single bond | Me | CH₂CH=CH₂ | C(=O)Me | Me | H | O |
| C-16450 | Me | single bond | H | CH₂CH=CH₂ | C(=O)Me | Me | H | O |
| C-16451 | Me(R) | single bond | H | CH₂CH=CH₂ | C(=O)Me | Me | H | O |
| C-16452 | Me(S) | single bond | H | CH₂CH=CH₂ | C(=O)Me | Me | H | O |
| C-16453 | H | single bond | Me | CH₂C≡CH | C(=O)Me | Cl | H | O |
| C-16454 | Me | single bond | H | CH₂C≡CH | C(=O)Me | Cl | H | O |
| C-16455 | Me(R) | single bond | H | CH₂C≡CH | C(=O)Me | Cl | H | O |
| C-16456 | Me(S) | single bond | H | CH₂C≡CH | C(=O)Me | Cl | H | O |
| C-16457 | H | single bond | Me | CH₂C≡CH | C(=O)Me | Me | H | O |
| C-16458 | Me | single bond | H | CH₂C≡CH | C(=O)Me | Me | H | O |
| C-16459 | Me(R) | single bond | H | CH₂C≡CH | C(=O)Me | Me | H | O |
| C-16460 | Me(S) | single bond | H | CH₂C≡CH | C(=O)Me | Me | H | O |
| C-16461 | H | single bond | Me | CH₂CN | C(=O)Me | Cl | H | O |
| C-16462 | Me | single bond | H | CH₂CN | C(=O)Me | Cl | H | O |
| C-16463 | Me(R) | single bond | H | CH₂CN | C(=O)Me | Cl | H | O |
| C-16464 | Me(S) | single bond | H | CH₂CN | C(=O)Me | Cl | H | O |
| C-16465 | H | single bond | Me | CH₂CN | C(=O)Me | Me | H | O |
| C-16466 | Me | single bond | H | CH₂CN | C(=O)Me | Me | H | O |
| C-16467 | Me(R) | single bond | H | CH₂CN | C(=O)Me | Me | H | O |
| C-16468 | Me(S) | single bond | H | CH₂CN | C(=O)Me | Me | H | O |
| C-16469 | H | single bond | Me | CH(Me)CN | C(=O)Me | Cl | H | O |
| C-16470 | Me | single bond | H | CH(Me)CN | C(=O)Me | Cl | H | O |
| C-16471 | Me(R) | single bond | H | CH(Me)CN | C(=O)Me | Cl | H | O |
| C-16472 | Me(S) | single bond | H | CH(Me)CN | C(=O)Me | Cl | H | O |
| C-16473 | H | single bond | Me | CH(Me)CN | C(=O)Me | Me | H | O |
| C-16474 | Me | single bond | H | CH(Me)CN | C(=O)Me | Me | H | O |

**[Table 603]**

| Compound Number | Y | W | Z | R¹ | R² | R³ | R¹⁰ | A |
|---|---|---|---|---|---|---|---|---|
| C-16475 | Me(R) | single bond | H | CH(Me)CN | C(=O)Me | Me | H | O |
| C-16476 | Me(S) | single bond | H | CH(Me)CN | C(=O)Me | Me | H | O |
| C-16477 | H | single bond | Me | C(Me)₂CN | C(=O)Me | Cl | H | O |
| C-16478 | Me | single bond | H | C(Me)₂CN | C(=O)Me | Cl | H | O |
| C-16479 | Me(R) | single bond | H | C(Me)₂CN | C(=O)Me | Cl | H | O |
| C-16480 | Me(S) | single bond | H | C(Me)₂CN | C(=O)Me | Cl | H | O |
| C-16481 | H | single bond | Me | C(Me)₂CN | C(=O)Me | Me | H | O |
| C-16482 | Me | single bond | H | C(Me)₂CN | C(=O)Me | Me | H | O |
| C-16483 | Me(R) | single bond | H | C(Me)₂CN | C(=O)Me | Me | H | O |
| C-16484 | Me(S) | single bond | H | C(Me)₂CN | C(=O)Me | Me | H | O |
| C-16485 | H | single bond | Me | C(Me)(*i*-Pr)CN | C(=O)Me | Cl | H | O |
| C-16486 | Me | single bond | H | C(Me)(*i*-Pr)CN | C(=O)Me | Cl | H | O |
| C-16487 | Me(R) | single bond | H | C(Me)(*i*-Pr)CN | C(=O)Me | Cl | H | O |
| C-16488 | Me(S) | single bond | H | C(Me)(*i*-Pr)CN | C(=O)Me | Cl | H | O |
| C-16489 | H | single bond | Me | C(Me)(*i*-Pr)CN | C(=O)Me | Me | H | O |
| C-16490 | Me | single bond | H | C(Me)(*i*-Pr)CN | C(=O)Me | Me | H | O |
| C-16491 | Me(R) | single bond | H | C(Me)(*i*-Pr)CN | C(=O)Me | Me | H | O |
| C-16492 | Me(S) | single bond | H | C(Me)(*i*-Pr)CN | C(=O)Me | Me | H | O |
| C-16493 | H | single bond | Me | OMe | C(=O)Me | Cl | H | O |
| C-16494 | Me | single bond | H | OMe | C(=O)Me | Cl | H | O |
| C-16495 | Me(R) | single bond | H | OMe | C(=O)Me | Cl | H | O |
| C-16496 | Me(S) | single bond | H | OMe | C(=O)Me | Cl | H | O |
| C-16497 | H | single bond | Me | OMe | C(=O)Me | Me | H | O |
| C-16498 | Me | single bond | H | OMe | C(=O)Me | Me | H | O |
| C-16499 | Me(R) | single bond | H | OMe | C(=O)Me | Me | H | O |
| C-16500 | Me(S) | single bond | H | OMe | C(=O)Me | Me | H | O |
| C-16501 | H | single bond | Me | CH=NOMe | C(=O)Me | Cl | H | O |
| C-16502 | Me | single bond | H | CH=NOMe | C(=O)Me | Cl | H | O |
| C-16503 | Me(R) | single bond | H | CH=NOMe | C(=O)Me | Cl | H | O |
| C-16504 | Me(S) | single bond | H | CH=NOMe | C(=O)Me | Cl | H | O |
| C-16505 | H | single bond | Me | CH=NOMe | C(=O)Me | Me | H | O |
| C-16506 | Me | single bond | H | CH=NOMe | C(=O)Me | Me | H | O |
| C-16507 | Me(R) | single bond | H | CH=NOMe | C(=O)Me | Me | H | O |
| C-16508 | Me(S) | single bond | H | CH=NOMe | C(=O)Me | Me | H | O |
| C-16509 | H | single bond | Me | morpholin-4-yl | C(=O)Me | Cl | H | O |
| C-16510 | Me | single bond | H | morpholin-4-yl | C(=O)Me | Cl | H | O |
| C-16511 | Me(R) | single bond | H | morpholin-4-yl | C(=O)Me | Cl | H | O |
| C-16512 | Me(S) | single bond | H | morpholin-4-yl | C(=O)Me | Cl | H | O |
| C-16513 | H | single bond | Me | morpholin-4-yl | C(=O)Me | Me | H | O |
| C-16514 | Me | single bond | H | morpholin-4-yl | C(=O)Me | Me | H | O |
| C-16515 | Me(R) | single bond | H | morpholin-4-yl | C(=O)Me | Me | H | O |
| C-16516 | Me(S) | single bond | H | morpholin-4-yl | C(=O)Me | Me | H | O |
| C-16517 | H | single bond | Me | Ph | C(=O)Me | Cl | H | O |
| C-16518 | Me | single bond | H | Ph | C(=O)Me | Cl | H | O |

**[Table 604]**

| Compound Number | Y | W | Z | R¹ | R² | R³ | R¹⁰ | A |
|---|---|---|---|---|---|---|---|---|
| C-16519 | Me(R) | single bond | H | Ph | C(=O)Me | Cl | H | O |
| C-16520 | Me(S) | single bond | H | Ph | C(=O)Me | Cl | H | O |
| C-16521 | H | single bond | Me | Ph | C(=O)Me | Me | H | O |
| C-16522 | Me | single bond | H | Ph | C(=O)Me | Me | H | O |
| C-16523 | Me(R) | single bond | H | Ph | C(=O)Me | Me | H | O |
| C-16524 | Me(S) | single bond | H | Ph | C(=O)Me | Me | H | O |
| C-16525 | H | single bond | Me | CH₂Ph | C(=O)Me | Cl | H | O |
| C-16526 | Me | single bond | H | CH₂Ph | C(=O)Me | Cl | H | O |
| C-16527 | Me(R) | single bond | H | CH₂Ph | C(=O)Me | Cl | H | O |
| C-16528 | Me(S) | single bond | H | CH₂Ph | C(=O)Me | Cl | H | O |
| C-16529 | H | single bond | Me | CH₂Ph | C(=O)Me | Me | H | O |
| C-16530 | Me | single bond | H | CH₂Ph | C(=O)Me | Me | H | O |
| C-16531 | Me(R) | single bond | H | CH₂Ph | C(=O)Me | Me | H | O |
| C-16532 | Me(S) | single bond | H | CH₂Ph | C(=O)Me | Me | H | O |
| C-16533 | H | single bond | Me | thiazole-2-yl | C(=O)Me | Cl | H | O |
| C-16534 | Me | single bond | H | thiazole-2-yl | C(=O)Me | Cl | H | O |
| C-16535 | Me(R) | single bond | H | thiazole-2-yl | C(=O)Me | Cl | H | O |
| C-16536 | Me(S) | single bond | H | thiazole-2-yl | C(=O)Me | Cl | H | O |
| C-16537 | H | single bond | Me | thiazole-2-yl | C(=O)Me | Me | H | O |
| C-16538 | Me | single bond | H | thiazole-2-yl | C(=O)Me | Me | H | O |
| C-16539 | Me(R) | single bond | H | thiazole-2-yl | C(=O)Me | Me | H | O |
| C-16540 | Me(S) | single bond | H | thiazole-2-yl | C(=O)Me | Me | H | O |
| C-16541 | H | single bond | Me | 1-Me-pyrazol-3-yl | C(=O)Me | Cl | H | O |
| C-16542 | Me | single bond | H | 1-Me-pyrazol-3-yl | C(=O)Me | Cl | H | O |
| C-16543 | Me(R) | single bond | H | 1-Me-pyrazol-3-yl | C(=O)Me | Cl | H | O |
| C-16544 | Me(S) | single bond | H | 1-Me-pyrazol-3-yl | C(=O)Me | Cl | H | O |
| C-16545 | H | single bond | Me | 1-Me-pyrazol-3-yl | C(=O)Me | Me | H | O |
| C-16546 | Me | single bond | H | 1-Me-pyrazol-3-yl | C(=O)Me | Me | H | O |
| C-16547 | Me(R) | single bond | H | 1-Me-pyrazol-3-yl | C(=O)Me | Me | H | O |
| C-16548 | Me(S) | single bond | H | 1-Me-pyrazol-3-yl | C(=O)Me | Me | H | O |
| C-16549 | H | single bond | Me | 1-Me-pyrazol-5-yl | C(=O)Me | Cl | H | O |
| C-16550 | Me | single bond | H | 1-Me-pyrazol-5-yl | C(=O)Me | Cl | H | O |
| C-16551 | Me(R) | single bond | H | 1-Me-pyrazol-5-yl | C(=O)Me | Cl | H | O |
| C-16552 | Me(S) | single bond | H | 1-Me-pyrazol-5-yl | C(=O)Me | Cl | H | O |
| C-16553 | H | single bond | Me | 1-Me-pyrazol-5-yl | C(=O)Me | Me | H | O |
| C-16554 | Me | single bond | H | 1-Me-pyrazol-5-yl | C(=O)Me | Me | H | O |
| C-16555 | Me(R) | single bond | H | 1-Me-pyrazol-5-yl | C(=O)Me | Me | H | O |
| C-16556 | Me(S) | single bond | H | 1-Me-pyrazol-5-yl | C(=O)Me | Me | H | O |
| C-16557 | H | single bond | Me | CH₂(pyrimidin-2-yl) | C(=O)Me | Cl | H | O |
| C-16558 | Me | single bond | H | CH₂(pyrimidin-2-yl) | C(=O)Me | Cl | H | O |
| C-16559 | Me(R) | single bond | H | CH₂(pyrimidin-2-yl) | C(=O)Me | Cl | H | O |
| C-16560 | Me(S) | single bond | H | CH₂(pyrimidin-2-yl) | C(=O)Me | Cl | H | O |
| C-16561 | H | single bond | Me | CH₂(pyrimidin-2-yl) | C(=O)Me | Me | H | O |
| C-16562 | Me | single bond | H | CH₂(pyrimidin-2-yl) | C(=O)Me | Me | H | O |

**[Table 605]**

| Compound Number | Y | W | Z | R¹ | R² | R³ | R¹⁰ | A |
|---|---|---|---|---|---|---|---|---|
| C-16563 | Me(R) | single bond | H | CH₂(pyrimidin-2-yl) | C(=O)Me | Me | H | O |
| C-16564 | Me(S) | single bond | H | CH₂(pyrimidin-2-yl) | C(=O)Me | Me | H | O |
| C-16565 | H | single bond | Me | Me | C(=O)Me | Cl | Me | O |
| C-16566 | Me | single bond | H | Me | C(=O)Me | Cl | Me | O |
| C-16567 | Me(R) | single bond | H | Me | C(=O)Me | Cl | Me | O |
| C-16568 | Me(S) | single bond | H | Me | C(=O)Me | Cl | Me | O |
| C-16569 | H | single bond | Me | Me | C(=O)Me | Me | Me | O |
| C-16570 | Me | single bond | H | Me | C(=O)Me | Me | Me | O |
| C-16571 | Me(R) | single bond | H | Me | C(=O)Me | Me | Me | O |
| C-16572 | Me(S) | single bond | H | Me | C(=O)Me | Me | Me | O |
| C-16573 | H | single bond | Me | CH₂CHF₂ | C(=O)Me | Cl | Me | O |
| C-16574 | Me | single bond | H | CH₂CHF₂ | C(=O)Me | Cl | Me | O |
| C-16575 | Me(R) | single bond | H | CH₂CHF₂ | C(=O)Me | Cl | Me | O |
| C-16576 | Me(S) | single bond | H | CH₂CHF₂ | C(=O)Me | Cl | Me | O |
| C-16577 | H | single bond | Me | CH₂CHF₂ | C(=O)Me | Me | Me | O |
| C-16578 | Me | single bond | H | CH₂CHF₂ | C(=O)Me | Me | Me | O |
| C-16579 | Me(R) | single bond | H | CH₂CHF₂ | C(=O)Me | Me | Me | O |
| C-16580 | Me(S) | single bond | H | CH₂CHF₂ | C(=O)Me | Me | Me | O |
| C-16581 | H | single bond | Me | *n*-Pr | C(=O)Me | Cl | Me | O |
| C-16582 | Me | single bond | H | *n*-Pr | C(=O)Me | Cl | Me | O |
| C-16583 | Me(R) | single bond | H | *n*-Pr | C(=O)Me | Cl | Me | O |
| C-16584 | Me(S) | single bond | H | *n*-Pr | C(=O)Me | Cl | Me | O |
| C-16585 | H | single bond | Me | *n*-Pr | C(=O)Me | Me | Me | O |
| C-16586 | Me | single bond | H | *n*-Pr | C(=O)Me | Me | Me | O |
| C-16587 | Me(R) | single bond | H | *n*-Pr | C(=O)Me | Me | Me | O |
| C-16588 | Me(S) | single bond | H | *n*-Pr | C(=O)Me | Me | Me | O |
| C-16589 | H | single bond | Me | *i-*Pr | C(=O)Me | Cl | Me | O |
| C-16590 | Me | single bond | H | *i*-Pr | C(=O)Me | Cl | Me | O |
| C-16591 | Me(R) | single bond | H | *i*-Pr | C(=O)Me | Cl | Me | O |
| C-16592 | Me(S) | single bond | H | *i*-Pr | C(=O)Me | Cl | Me | O |
| C-16593 | H | single bond | Me | *i*-Pr | C(=O)Me | Me | Me | O |
| C-16594 | Me | single bond | H | *i*-Pr | C(=O)Me | Me | Me | O |
| C-16595 | Me(R) | single bond | H | *i*-Pr | C(=O)Me | Me | Me | O |
| C-16596 | Me(S) | single bond | H | *i*-Pr | C(=O)Me | Me | Me | O |
| C-16597 | H | single bond | Me | *c*-Pr(1-F) | C(=O)Me | Cl | Me | O |
| C-16598 | Me | single bond | H | *c*-Pr(1-F) | C(=O)Me | Cl | Me | O |
| C-16599 | Me(R) | single bond | H | *c*-Pr(1-F) | C(=O)Me | Cl | Me | O |
| C-16600 | Me(S) | single bond | H | *c*-Pr(1-F) | C(=O)Me | Cl | Me | O |
| C-16601 | H | single bond | Me | *c*-Pr(1-F) | C(=O)Me | Me | Me | O |
| C-16602 | Me | single bond | H | *c*-Pr(1-F) | C(=O)Me | Me | Me | O |
| C-16603 | Me(R) | single bond | H | *c*-Pr(1-F) | C(=O)Me | Me | Me | O |
| C-16604 | Me(S) | single bond | H | *c*-Pr(1-F) | C(=O)Me | Me | Me | O |
| C-16605 | H | single bond | Me | *c-*Pr(2,2-F₂) | C(=O)Me | Cl | Me | O |
| C-16606 | Me | single bond | H | *c*-Pr(2,2-F₂) | C(=O)Me | Cl | Me | O |

**[Table 606]**

| Compound Number | Y | W | Z | R¹ | R² | R³ | R¹⁰ | A |
|---|---|---|---|---|---|---|---|---|
| C-16607 | Me(R) | single bond | H | *c*-Pr(2,2-F₂) | C(=O)Me | Cl | Me | O |
| C-16608 | Me(S) | single bond | H | *c*-Pr(2,2-F₂) | C(=O)Me | Cl | Me | O |
| C-16609 | H | single bond | Me | *c*-Pr(2,2-F₂) | C(=O)Me | Me | Me | O |
| C-16610 | Me | single bond | H | *c*-Pr(2,2-F₂) | C(=O)Me | Me | Me | O |
| C-16611 | Me(R) | single bond | H | *c*-Pr(2,2-F₂) | C(=O)Me | Me | Me | O |
| C-16612 | Me(S) | single bond | H | *c*-Pr(2,2-F₂) | C(=O)Me | Me | Me | O |
| C-16613 | H | single bond | Me | *c*-Pr(1-Cl) | C(=O)Me | Cl | Me | O |
| C-16614 | Me | single bond | H | *c*-Pr(1-Cl) | C(=O)Me | Cl | Me | O |
| C-16615 | Me(R) | single bond | H | *c*-Pr(1-Cl) | C(=O)Me | Cl | Me | O |
| C-16616 | Me(S) | single bond | H | *c*-Pr(1-Cl) | C(=O)Me | Cl | Me | O |
| C-16617 | H | single bond | Me | *c*-Pr(1-Cl) | C(=O)Me | Me | Me | O |
| C-16618 | Me | single bond | H | *c*-Pr(1-Cl) | C(=O)Me | Me | Me | O |
| C-16619 | Me(R) | single bond | H | *c*-Pr(1-Cl) | C(=O)Me | Me | Me | O |
| C-16620 | Me(S) | single bond | H | *c*-Pr(1-Cl) | C(=O)Me | Me | Me | O |
| C-16621 | H | single bond | Me | *c*-Pr(1-Br) | C(=O)Me | Cl | Me | O |
| C-16622 | Me | single bond | H | *c*-Pr(1-Br) | C(=O)Me | Cl | Me | O |
| C-16623 | Me(R) | single bond | H | *c*-Pr(1-Br) | C(=O)Me | Cl | Me | O |
| C-16624 | Me(S) | single bond | H | *c*-Pr(1-Br) | C(=O)Me | Cl | Me | O |
| C-16625 | H | single bond | Me | *c*-Pr(1-Br) | C(=O)Me | Me | Me | O |
| C-16626 | Me | single bond | H | *c*-Pr(1-Br) | C(=O)Me | Me | Me | O |
| C-16627 | Me(R) | single bond | H | *c*-Pr(1-Br) | C(=O)Me | Me | Me | O |
| C-16628 | Me(S) | single bond | H | *c*-Pr(1-Br) | C(=O)Me | Me | Me | O |
| C-16629 | H | single bond | Me | *c*-Pr(1-Me) | C(=O)Me | Cl | Me | O |
| C-16630 | Me | single bond | H | *c*-Pr(1-Me) | C(=O)Me | Cl | Me | O |
| C-16631 | Me(R) | single bond | H | *c*-Pr(1-Me) | C(=O)Me | Cl | Me | O |
| C-16632 | Me(S) | single bond | H | *c*-Pr(1-Me) | C(=O)Me | Cl | Me | O |
| C-16633 | H | single bond | Me | *c*-Pr(1-Me) | C(=O)Me | Me | Me | O |
| C-16634 | Me | single bond | H | *c*-Pr(1-Me) | C(=O)Me | Me | Me | O |
| C-16635 | Me(R) | single bond | H | *c*-Pr(1-Me) | C(=O)Me | Me | Me | O |
| C-16636 | Me(S) | single bond | H | *c*-Pr(1-Me) | C(=O)Me | Me | Me | O |
| C-16637 | H | single bond | Me | *c*-Pr(1-CH₂OH) | C(=O)Me | Cl | Me | O |
| C-16638 | Me | single bond | H | *c*-Pr(1-CH₂OH) | C(=O)Me | Cl | Me | O |
| C-16639 | Me(R) | single bond | H | *c*-Pr(1-CH₂OH) | C(=O)Me | Cl | Me | O |
| C-16640 | Me(S) | single bond | H | *c*-Pr(1-CH₂OH) | C(=O)Me | Cl | Me | O |
| C-16641 | H | single bond | Me | *c*-Pr(1-CH₂OH) | C(=O)Me | Me | Me | O |
| C-16642 | Me | single bond | H | *c*-Pr(1-CH₂OH) | C(=O)Me | Me | Me | O |
| C-16643 | Me(R) | single bond | H | *c*-Pr(1-CH₂OH) | C(=O)Me | Me | Me | O |
| C-16644 | Me(S) | single bond | H | *c*-Pr(1-CH₂OH) | C(=O)Me | Me | Me | O |
| C-16645 | H | single bond | Me | *c*-Pr[1-CH₂OS(=O)₂Me] | C(=O)Me | Cl | Me | O |
| C-16646 | Me | single bond | H | *c*-Pr[1-CH₂OS(=O)₂Me] | C(=O)Me | Cl | Me | O |
| C-16647 | Me(R) | single bond | H | *c*-Pr[1-CH₂OS(=O)₂Me] | C(=O)Me | Cl | Me | ○ |
| C-16648 | Me(S) | single bond | H | *c*-Pr[1-CH₂OS(=O)₂Me] | C(=O)Me | Cl | Me | O |
| C-16649 | H | single bond | Me | *c*-Pr[1-CH₂OS(=O)₂Me] | C(=O)Me | Me | Me | O |
| C-16650 | Me | single bond | H | *c-*Pr[1-CH₂OS(=O)₂Me] | C(=O)Me | Me | Me | O |

**[Table 607]**

| Compound Number | Y | W | Z | R¹ | R² | R³ | R¹⁰ | A |
|---|---|---|---|---|---|---|---|---|
| C-16651 | Me(R) | single bond | H | *c*-Pr[1-CH₂OS(=O)₂Me] | C(=O)Me | Me | Me | O |
| C-16652 | Me(S) | single bond | H | *c*-Pr[1-CH₂OS(=O)₂Me] | C(=O)Me | Me | Me | O |
| C-16653 | H | single bond | Me | *c*-Pr(1-CH₂OMe) | C(=O)Me | Cl | Me | O |
| C-16654 | Me | single bond | H | *c*-Pr(1-CH₂OMe) | C(=O)Me | Cl | Me | O |
| C-16655 | Me(R) | single bond | H | *c*-Pr(1-CH₂OMe) | C(=O)Me | Cl | Me | O |
| C-16656 | Me(S) | single bond | H | *c*-Pr(1-CH₂OMe) | C(=O)Me | Cl | Me | O |
| C-16657 | H | single bond | Me | *c*-Pr(1-CH₂OMe) | C(=O)Me | Me | Me | O |
| C-16658 | Me | single bond | H | *c*-Pr(1-CH₂OMe) | C(=O)Me | Me | Me | O |
| C-16659 | Me(R) | single bond | H | *c*-Pr(1-CH₂OMe) | C(=O)Me | Me | Me | O |
| C-16660 | Me(S) | single bond | H | *c*-Pr(1-CH₂OMe) | C(=O)Me | Me | Me | O |
| C-16661 | H | single bond | Me | *c*-Pr(1-CH₂CN) | C(=O)Me | Cl | Me | O |
| C-16662 | Me | single bond | H | *c-*Pr(1-CH₂CN) | C(=O)Me | Cl | Me | O |
| C-16663 | Me(R) | single bond | H | *c*-Pr(1-CH₂CN) | C(=O)Me | Cl | Me | O |
| C-16664 | Me(S) | single bond | H | *c*-Pr(1-CH₂CN) | C(=O)Me | Cl | Me | O |
| C-16665 | H | single bond | Me | *c*-Pr(1-CH₂CN) | C(=O)Me | Me | Me | O |
| C-16666 | Me | single bond | H | *c*-Pr(1-CH₂CN) | C(=O)Me | Me | Me | O |
| C-16667 | Me(R) | single bond | H | *c*-Pr(1-CH₂CN) | C(=O)Me | Me | Me | O |
| C-16668 | Me(S) | single bond | H | *c*-Pr(1-CH₂CN) | C(=O)Me | Me | Me | O |
| C-16669 | H | single bond | Me | *c*-Pr(1-CH₂SMe) | C(=O)Me | Cl | Me | O |
| C-16670 | Me | single bond | H | *c*-Pr(1-CH₂SMe) | C(=O)Me | Cl | Me | O |
| C-16671 | Me(R) | single bond | H | *c*-Pr(1-CH₂SMe) | C(=O)Me | Cl | Me | O |
| C-16672 | Me(S) | single bond | H | *c*-Pr(1-CH₂SMe) | C(=O)Me | Cl | Me | O |
| C-16673 | H | single bond | Me | *c*-Pr(1-CH₂SMe) | C(=O)Me | Me | Me | O |
| C-16674 | Me | single bond | H | *c*-Pr(1-CH₂SMe) | C(=O)Me | Me | Me | O |
| C-16675 | Me(R) | single bond | H | *c*-Pr(1-CH₂SMe) | C(=O)Me | Me | Me | O |
| C-16676 | Me(S) | single bond | H | *c*-Pr(1-CH₂SMe) | C(=O)Me | Me | Me | O |
| C-16677 | H | single bond | Me | *c*-Pr[1-CH₂S(=O)Me] | C(=O)Me | Cl | Me | O |
| C-16678 | Me | single bond | H | *c* -Pr[1-CH₂S(=O)Me] | C(=O)Me | Cl | Me | O |
| C-16679 | Me(R) | single bond | H | *c* -Pr[1-CH₂S(=O)Me] | C(=O)Me | Cl | Me | O |
| C-16680 | Me(S) | single bond | H | *c* -Pr[1-CH₂S(=O)Me] | C(=O)Me | Cl | Me | O |
| C-16681 | H | single bond | Me | *c* -Pr[1-CH₂S(=O)Me] | C(=O)Me | Me | Me | O |
| C-16682 | Me | single bond | H | *c* -Pr[1-CH₂S(=O)Me] | C(=O)Me | Me | Me | O |
| C-16683 | Me(R) | single bond | H | *c* -Pr[1-CH₂S(=O)Me] | C(=O)Me | Me | Me | O |
| C-16684 | Me(S) | single bond | H | *c* -Pr[1-CH₂S(=O)Me] | C(=O)Me | Me | Me | O |
| C-16685 | H | single bond | Me | *c* -Pr[1-CH₂S(=O)₂Me] | C(=O)Me | Cl | Me | O |
| C-16686 | Me | single bond | H | *c* -Pr[1-CH₂S(=O)₂Me] | C(=O)Me | Cl | Me | O |
| C-16687 | Me(R) | single bond | H | *c* -Pr[1-CH₂S(=O)₂Me] | C(=O)Me | Cl | Me | O |
| C-16688 | Me(S) | single bond | H | *c* -Pr[1-CH₂S(=O)₂Me] | C(=O)Me | Cl | Me | O |
| C-16689 | H | single bond | Me | *c* -Pr[1-CH₂S(=O)₂Me] | C(=O)Me | Me | Me | O |
| C-16690 | Me | single bond | H | *c* -Pr[1-CH₂S(=O)₂Me] | C(=O)Me | Me | Me | O |
| C-16691 | Me(R) | single bond | H | *c* -Pr[1-CH₂S(=O)₂Me] | C(=O)Me | Me | Me | O |
| C-16692 | Me(S) | single bond | H | *c* -Pr[1-CH₂S(=O)₂Me] | C(=O)Me | Me | Me | O |
| C-16693 | H | single bond | Me | *c* -Pr(1-CH₂F) | C(=O)Me | Cl | Me | O |
| C-16694 | Me | single bond | H | *c* -Pr(1-CH₂F) | C(=O)Me | Cl | Me | O |

**[Table 608]**

| Compound Number | Y | W | Z | R¹ | R² | R³ | R¹⁰ | A |
|---|---|---|---|---|---|---|---|---|
| C-16695 | Me(R) | single bond | H | *c* -Pr(1-CH₂F) | C(=O)Me | Cl | Me | O |
| C-16696 | Me(S) | single bond | H | *c* -Pr(1-CH₂F) | C(=O)Me | Cl | Me | O |
| C-16697 | H | single bond | Me | *c* -Pr(1-CH₂F) | C(=O)Me | Me | Me | O |
| C-16698 | Me | single bond | H | *c* -Pr(1-CH₂F) | C(=O)Me | Me | Me | O |
| C-16699 | Me(R) | single bond | H | *c* -Pr(1-CH₂F) | C(=O)Me | Me | Me | O |
| C-16700 | Me(S) | single bond | H | *c* -Pr(1-CH₂F) | C(=O)Me | Me | Me | O |
| C-16701 | H | single bond | Me | *c* -Pr(1-CHF₂) | C(=O)Me | Cl | Me | O |
| C-16702 | Me | single bond | H | *c* -Pr(1-CHF₂) | C(=O)Me | Cl | Me | O |
| C-16703 | Me(R) | single bond | H | *c* -Pr(1-CHF₂) | C(=O)Me | Cl | Me | O |
| C-16704 | Me(S) | single bond | H | *c* -Pr(1-CHF₂) | C(=O)Me | Cl | Me | O |
| C-16705 | H | single bond | Me | *c* -Pr(1-CHF₂) | C(=O)Me | Me | Me | O |
| C-16706 | Me | single bond | H | *c* -Pr(1-CHF₂) | C(=O)Me | Me | Me | O |
| C-16707 | Me(R) | single bond | H | *c* -Pr(1-CHF₂) | C(=O)Me | Me | Me | O |
| C-16708 | Me(S) | single bond | H | *c* -Pr(1-CHF₂) | C(=O)Me | Me | Me | O |
| C-16709 | H | single bond | Me | *c* -Pr(1-CF₃) | C(=O)Me | Cl | Me | O |
| C-16710 | Me | single bond | H | *c* -Pr(1-CF₃) | C(=O)Me | Cl | Me | O |
| C-16711 | Me(R) | single bond | H | *c* -Pr(1-CF₃) | C(=O)Me | Cl | Me | O |
| C-16712 | Me(S) | single bond | H | *c* -Pr(1-CF₃) | C(=O)Me | Cl | Me | O |
| C-16713 | H | single bond | Me | *c* -Pr(1-CF₃) | C(=O)Me | Me | Me | O |
| C-16714 | Me | single bond | H | *c* -Pr(1-CF₃) | C(=O)Me | Me | Me | O |
| C-16715 | Me(R) | single bond | H | *c* -Pr(1-CF₃) | C(=O)Me | Me | Me | O |
| C-16716 | Me(S) | single bond | H | *c* -Pr(1-CF₃) | C(=O)Me | Me | Me | O |
| C-16717 | H | single bond | Me | *c* -Pr(1-C≡CH) | C(=O)Me | Cl | Me | O |
| C-16718 | Me | single bond | H | *c* -Pr(1-C≡CH) | C(=O)Me | Cl | Me | O |
| C-16719 | Me(R) | single bond | H | *c* -Pr(1-C≡CH) | C(=O)Me | Cl | Me | O |
| C-16720 | Me(S) | single bond | H | *c* -Pr(1-C≡CH) | C(=O)Me | Cl | Me | O |
| C-16721 | H | single bond | Me | *c* -Pr(1-C≡CH) | C(=O)Me | Me | Me | O |
| C-16722 | Me | single bond | H | *c* -Pr(1-C≡CH) | C(=O)Me | Me | Me | O |
| C-16723 | Me(R) | single bond | H | *c* -Pr(1-C≡CH) | C(=O)Me | Me | Me | O |
| C-16724 | Me(S) | single bond | H | *c* -Pr(1-C≡CH) | C(=O)Me | Me | Me | O |
| C-16725 | H | single bond | Me | *c* -Pr[1-C(=O)OEt] | C(=O)Me | Cl | Me | O |
| C-16726 | Me | single bond | H | *c* -Pr[1-C(=O)OEt] | C(=O)Me | Cl | Me | O |
| C-16727 | Me(R) | single bond | H | *c* -Pr[1-C(=O)OEt] | C(=O)Me | Cl | Me | O |
| C-16728 | Me(S) | single bond | H | *c* -Pr[1-C(=O)OEt] | C(=O)Me | Cl | Me | O |
| C-16729 | H | single bond | Me | *c* -Pr[1-C(=O)OEt] | C(=O)Me | Me | Me | O |
| C-16730 | Me | single bond | H | *c* -Pr[1-C(=O)OEt] | C(=O)Me | Me | Me | O |
| C-16731 | Me(R) | single bond | H | *c* -Pr[1-C(=O)OEt] | C(=O)Me | Me | Me | O |
| C-16732 | Me(S) | single bond | H | *c* -Pr[1-C(=O)OEt] | C(=O)Me | Me | Me | O |
| C-16733 | H | single bond | Me | *c* -Pr[1-C(=O)OH] | C(=O)Me | Cl | Me | O |
| C-16734 | Me | single bond | H | *c* -Pr[1-C(=O)OH] | C(=O)Me | Cl | Me | O |
| C-16735 | Me(R) | single bond | H | *c* -Pr[1-C(=O)OH] | C(=O)Me | Cl | Me | O |
| C-16736 | Me(S) | single bond | H | *c* -Pr[1-C(=O)OH] | C(=O)Me | Cl | Me | O |
| C-16737 | H | single bond | Me | c -Pr[1-C(=0)OH] | C(=O)Me | Me | Me | O |
| C-16738 | Me | single bond | H | *c* -Pr[1-C(=O)OH] | C(=O)Me | Me | Me | O |

**[Table 609]**

| Compound Number | Y | W | Z | R¹ | R² | R³ | R¹⁰ | A |
|---|---|---|---|---|---|---|---|---|
| C-16739 | Me(R) | single bond | H | *c* -Pr[1-C(=O)OH] | C(=O)Me | Me | Me | O |
| C-16740 | Me(S) | single bond | H | *c* -Pr[1-C(=O)OH] | C(=O)Me | Me | Me | O |
| C-16741 | H | single bond | Me | *c* -Pr[1-C(=O)NH₂] | C(=O)Me | Cl | Me | O |
| C-16742 | Me | single bond | H | *c* -Pr[1-C(=O)NH₂] | C(=O)Me | Cl | Me | O |
| C-16743 | Me(R) | single bond | H | *c* -Pr[1-C(=O)NH₂] | C(=O)Me | Cl | Me | O |
| C-16744 | Me(S) | single bond | H | *c* -Pr[1-C(=O)NH₂] | C(=O)Me | Cl | Me | O |
| C-16745 | H | single bond | Me | *c* -Pr[1-C(=O)NH₂] | C(=O)Me | Me | Me | O |
| C-16746 | Me | single bond | H | *c* -Pr[1-C(=O)NH₂] | C(=O)Me | Me | Me | O |
| C-16747 | Me(R) | single bond | H | *c* -Pr[1-C(=O)NH₂] | C(=O)Me | Me | Me | O |
| C-16748 | Me(S) | single bond | H | *c* -Pr[1-C(=O)NH₂] | C(=O)Me | Me | Me | O |
| C-16749 | H | single bond | Me | *c* -Pr(1-OMe) | C(=O)Me | Cl | Me | O |
| C-16750 | Me | single bond | H | *c* -Pr(1-OMe) | C(=O)Me | Cl | Me | O |
| C-16751 | Me(R) | single bond | H | *c* -Pr(1-OMe) | C(=O)Me | Cl | Me | O |
| C-16752 | Me(S) | single bond | H | *c* -Pr(1-OMe) | C(=O)Me | Cl | Me | O |
| C-16753 | H | single bond | Me | *c* -Pr(1-OMe) | C(=O)Me | Me | Me | O |
| C-16754 | Me | single bond | H | *c* -Pr(1-OMe) | C(=O)Me | Me | Me | O |
| C-16755 | Me(R) | single bond | H | *c* -Pr(1-OMe) | C(=O)Me | Me | Me | O |
| C-16756 | Me(S) | single bond | H | *c* -Pr(1-OMe) | C(=O)Me | Me | Me | O |
| C-16757 | H | single bond | Me | *c* -Pr(1-SMe) | C(=O)Me | Cl | Me | O |
| C-16758 | Me | single bond | H | *c* -Pr(1-SMe) | C(=O)Me | Cl | Me | O |
| C-16759 | Me(R) | single bond | H | *c* -Pr(1-SMe) | C(=O)Me | Cl | Me | O |
| C-16760 | Me(S) | single bond | H | *c* -Pr(1-SMe) | C(=O)Me | Cl | Me | O |
| C-16761 | H | single bond | Me | *c* -Pr(1-SMe) | C(=O)Me | Me | Me | O |
| C-16762 | Me | single bond | H | *c* -Pr(1-SMe) | C(=O)Me | Me | Me | O |
| C-16763 | Me(R) | single bond | H | *c* -Pr(1-SMe) | C(=O)Me | Me | Me | O |
| C-16764 | Me(S) | single bond | H | *c* -Pr(1-SMe) | C(=O)Me | Me | Me | O |
| C-16765 | H | single bond | Me | *c* -Pr[1-S(=O)Me] | C(=O)Me | Cl | Me | O |
| C-16766 | Me | single bond | H | *c* -Pr[1-S(=O)Me] | C(=O)Me | Cl | Me | O |
| C-16767 | Me(R) | single bond | H | *c* -Pr[1-S(=O)Me] | C(=O)Me | Cl | Me | O |
| C-16768 | Me(S) | single bond | H | *c* -Pr[1-S(=O)Me] | C(=O)Me | Cl | Me | O |
| C-16769 | H | single bond | Me | *c* -Pr[1-S(=O)Me] | C(=O)Me | Me | Me | O |
| C-16770 | Me | single bond | H | *c* -Pr[1-S(=O)Me] | C(=O)Me | Me | Me | O |
| C-16771 | Me(R) | single bond | H | *c* -Pr[1-S(=O)Me] | C(=O)Me | Me | Me | O |
| C-16772 | Me(S) | single bond | H | *c* -Pr[1-S(=O)Me] | C(=O)Me | Me | Me | O |
| C-16773 | H | single bond | Me | *c* -Pr[1-S(=O)₂Me] | C(=O)Me | Cl | Me | O |
| C-16774 | Me | single bond | H | *c* -Pr[1-S(=O)₂Me] | C(=O)Me | Cl | Me | O |
| C-16775 | Me(R) | single bond | H | *c* -Pr[1-S(=O)₂Me] | C(=O)Me | Cl | Me | O |
| C-16776 | Me(S) | single bond | H | *c* -Pr[1-S(=O)₂Me] | C(=O)Me | Cl | Me | O |
| C-16777 | H | single bond | Me | *c* -Pr[1-S(=O)₂Me] | C(=O)Me | Me | Me | O |
| C-16778 | Me | single bond | H | *c* -Pr[1-S(=O)₂Me] | C(=O)Me | Me | Me | O |
| C-16779 | Me(R) | single bond | H | *c* -Pr[1-S(=O)₂Me] | C(=O)Me | Me | Me | O |
| C-16780 | Me(S) | single bond | H | *c* -Pr[1-S(=O)₂Me] | C(=O)Me | Me | Me | O |
| C-16781 | H | single bond | Me | *c* -Pr[1-S(=O)₂CHF₂] | C(=O)Me | Cl | Me | O |
| C-16782 | Me | single bond | H | *c* -Pr[1-S(=O)₂CHF₂ | C(=O)Me | Cl | Me | O |

**[Table 610]**

| Compound Number | Y | W | Z | R¹ | R² | R³ | R¹⁰ | A |
|---|---|---|---|---|---|---|---|---|
| C-16783 | Me(R) | single bond | H | *c*-Pr[1-S(=O)₂CHF₂] | C(=O)Me | Cl | Me | O |
| C-16784 | Me(S) | single bond | H | *c*-Pr[1-S(=O)₂CHF₂] | C(=O)Me | Cl | Me | O |
| C-16785 | H | single bond | Me | *c*-Pr[1-S(=O)₂CHF₂] | C(=O)Me | Me | Me | O |
| C-16786 | Me | single bond | H | *c*-Pr[1-S(=O)₂CHF₂] | C(=O)Me | Me | Me | O |
| C-16787 | Me(R) | single bond | H | *c*-Pr[1-S(=O)₂CHF₂] | C(=O)Me | Me | Me | O |
| C-16788 | Me(S) | single bond | H | *c*-Pr[1-S(=O)₂CHF₂ | C(=O)Me | Me | Me | O |
| C-16789 | H | single bond | Me | *c*-Pr[1-S(=O)₂CF₃] | C(=O)Me | Cl | Me | O |
| C-16790 | Me | single bond | H | *c*-Pr[1-S(=O)₂CF₃] | C(=O)Me | Cl | Me | O |
| C-16791 | Me(R) | single bond | H | *c*-Pr[1-S(=O)₂CF₃] | C(=O)Me | Cl | Me | O |
| C-16792 | Me(S) | single bond | H | *c*-Pr[1-S(=O)₂CF₃] | C(=O)Me | Cl | Me | O |
| C-16793 | H | single bond | Me | *c*-Pr[1-S(=O)₂CF₃] | C(=O)Me | Me | Me | O |
| C-16794 | Me | single bond | H | *c*-Pr[1-S(=O)₂CF₃] | C(=O)Me | Me | Me | O |
| C-16795 | Me(R) | single bond | H | *c*-Pr[1-S(=O)₂CF₃] | C(=O)Me | Me | Me | O |
| C-16796 | Me(S) | single bond | H | *c*-Pr[1-S(=O)₂CF₃] | C(=O)Me | Me | Me | O |
| C-16797 | H | single bond | Me | *c*-Pr[1-(5,6-dihydro-1,4,2-dioxazin-3-yl)] | C(=O)Me | Cl | Me | O |
| C-16798 | Me | single bond | H | *c*-Pr[1-(5,6-dihydro-1,4,2-dioxazin-3-yl)] | C(=O)Me | Cl | Me | O |
| C-16799 | Me(R) | single bond | H | *c*-Pr[1-(5,6-dihydro-1,4,2-dioxazin-3-yl)] | C(=O)Me | Cl | Me | O |
| C-16800 | Me(S) | single bond | H | *c*-Pr[1-(5,6-dihydro-1,4,2-dioxazin-3-yl)] | C(=O)Me | Cl | Me | O |
| C-16801 | H | single bond | Me | *c*-Pr[1-(5,6-dihydro-1,4,2-dioxazin-3-yl)] | C(=O)Me | Me | Me | O |
| C-16802 | Me | single bond | H | *c*-Pr[1-(5,6-dihydro-1,4,2-dioxazin-3-yl)] | C(=O)Me | Me | Me | O |
| C-16803 | Me(R) | single bond | H | *c*-Pr[1-(5,6-dihydro-1,4,2-dioxazin-3-yl)] | C(=O)Me | Me | Me | O |
| C-16804 | Me(S) | single bond | H | *c*-Pr[1-(5,6-dihydro-1,4,2-dioxazin-3-yl)] | C(=O)Me | Me | Me | O |
| C-16805 | H | single bond | Me | *c*-Pr[1-(5-hydroxy-1,3,4-oxadiazol-2-yl)] | C(=O)Me | Cl | Me | O |
| C-16806 | Me | single bond | H | *c*-Pr[1-(5-hydroxy-1,3,4-oxadiazol-2-yl)] | C(=O)Me | Cl | Me | O |
| C-16807 | Me(R) | single bond | H | *c*-Pr[1-(5-hydroxy-1,3,4-oxadiazol-2-yl)] | C(=O)Me | Cl | Me | O |
| C-16808 | Me(S) | single bond | H | *c*-Pr[1-(5-hydroxy-1,3,4-oxadiazol-2-yl)] | C(=O)Me | Cl | Me | O |
| C-16809 | H | single bond | Me | *c*-Pr[1-(5-hydroxy-1,3,4-oxadiazol-2-yl)] | C(=O)Me | Me | Me | O |
| C-16810 | Me | single bond | H | *c*-Pr[1-(5-hydroxy-1,3,4-oxadiazol-2-yl)] | C(=O)Me | Me | Me | O |
| C-16811 | Me(R) | single bond | H | *c*-Pr[1-(5-hydroxy-1,3,4-oxadiazol-2-yl)] | C(=O)Me | Me | Me | O |
| C-16812 | Me(S) | single bond | H | *c*-Pr[1-(5-hydroxy-1,3,4-oxadiazol-2-yl)] | C(=O)Me | Me | Me | O |
| C-16813 | H | single bond | Me | *c*-Pr[1-(pyrimidin-2-yl)] | C(=O)Me | Cl | Me | O |
| C-16814 | Me | single bond | H | *c*-Pr[1-(pyrimidin-2-yl)] | C(=O)Me | Cl | Me | O |
| C-16815 | Me(R) | single bond | H | *c*-Pr[1-(pyrimidin-2-yl)] | C(=O)Me | Cl | Me | O |
| C-16816 | Me(S) | single bond | H | *c*-Pr[1-(pyrimidin-2-yl)] | C(=O)Me | Cl | Me | O |
| C-16817 | H | single bond | Me | *c*-Pr[1-(pyrimidin-2-yl)] | C(=O)Me | Me | Me | O |
| C-16818 | Me | single bond | H | *c*-Pr[1-(pyrimidin-2-yl)] | C(=O)Me | Me | Me | O |
| C-16819 | Me(R) | single bond | H | *c*-Pr[1-(pyrimidin-2-yl)] | C(=O)Me | Me | Me | O |
| C-16820 | Me(S) | single bond | H | *c*-Pr[1-(pyrimidin-2-yl)] | C(=O)Me | Me | Me | O |
| C-16821 | H | single bond | Me | *t*-Bu | C(=O)Me | Cl | Me | O |
| C-16822 | Me | single bond | H | *t*-Bu | C(=O)Me | Cl | Me | O |
| C-16823 | Me(R) | single bond | H | *t*-Bu | C(=O)Me | Cl | Me | O |
| C-16824 | Me(S) | single bond | H | *t*-Bu | C(=O)Me | Cl | Me | O |
| C-16825 | H | single bond | Me | *t*-Bu | C(=O)Me | Me | Me | O |
| C-16826 | Me | single bond | H | *t*-Bu | C(=O)Me | Me | Me | O |

**[Table 611]**

| Compound Number | Y | W | Z | R¹ | R² | R³ | R¹⁰ | A |
|---|---|---|---|---|---|---|---|---|
| C-16827 | Me(R) | single bond | H | *t*-Bu | C(=O)Me | Me | Me | O |
| C-16828 | Me(S) | single bond | H | *t*-Bu | C(=O)Me | Me | Me | O |
| C-16829 | H | single bond | Me | *c*-Bu | C(=O)Me | Cl | Me | O |
| C-16830 | Me | single bond | H | *c*-Bu | C(=O)Me | Cl | Me | O |
| C-16831 | Me(R) | single bond | H | *c*-Bu | C(=O)Me | Cl | Me | O |
| C-16832 | Me(S) | single bond | H | c-Bu | C(=O)Me | Cl | Me | O |
| C-16833 | H | single bond | Me | *c*-Bu | C(=O)Me | Me | Me | O |
| C-16834 | Me | single bond | H | *c*-Bu | C(=O)Me | Me | Me | O |
| C-16835 | Me(R) | single bond | H | *c*-Bu | C(=O)Me | Me | Me | O |
| C-16836 | Me(S) | single bond | H | *c*-Bu | C(=O)Me | Me | Me | O |
| C-16837 | H | single bond | Me | *c*-Bu(1-CN) | C(=O)Me | Cl | Me | O |
| C-16838 | Me | single bond | H | *c*-Bu(1-CN) | C(=O)Me | Cl | Me | O |
| C-16839 | Me(R) | single bond | H | *c*-Bu(1-CN) | C(=O)Me | Cl | Me | O |
| C-16840 | Me(S) | single bond | H | *c*-Bu(1-CN) | C(=O)Me | Cl | Me | O |
| C-16841 | H | single bond | Me | *c*-Bu(1-CN) | C(=O)Me | Me | Me | O |
| C-16842 | Me | single bond | H | *c*-Bu(1-CN) | C(=O)Me | Me | Me | O |
| C-16843 | Me(R) | single bond | H | *c*-Bu(1-CN) | C(=O)Me | Me | Me | O |
| C-16844 | Me(S) | single bond | H | *c*-Bu(1-CN) | C(=O)Me | Me | Me | O |
| C-16845 | H | single bond | Me | *c*-Bu(3,3-F₂) | C(=O)Me | Cl | Me | O |
| C-16846 | Me | single bond | H | *c*-Bu(3,3-F₂) | C(=O)Me | Cl | Me | O |
| C-16847 | Me(R) | single bond | H | *c*-Bu(3,3-F₂) | C(=O)Me | Cl | Me | O |
| C-16848 | Me(S) | single bond | H | *c*-Bu(3,3-F₂) | C(=O)Me | Cl | Me | O |
| C-16849 | H | single bond | Me | *c*-Bu(3,3-F₂) | C(=O)Me | Me | Me | O |
| C-16850 | Me | single bond | H | *c*-Bu(3,3-F₂) | C(=O)Me | Me | Me | O |
| C-16851 | Me(R) | single bond | H | *c*-Bu(3,3-F₂) | C(=O)Me | Me | Me | O |
| C-16852 | Me(S) | single bond | H | *c*-Bu(3,3-F₂) | C(=O)Me | Me | Me | O |
| C-16853 | H | single bond | Me | oxetan-3-yl | C(=O)Me | Cl | Me | O |
| C-16854 | Me | single bond | H | oxetan-3-yl | C(=O)Me | Cl | Me | O |
| C-16855 | Me(R) | single bond | H | oxetan-3-yl | C(=O)Me | Cl | Me | O |
| C-16856 | Me(S) | single bond | H | oxetan-3-yl | C(=O)Me | Cl | Me | O |
| C-16857 | H | single bond | Me | oxetan-3-yl | C(=O)Me | Me | Me | O |
| C-16858 | Me | single bond | H | oxetan-3-yl | C(=O)Me | Me | Me | O |
| C-16859 | Me(R) | single bond | H | oxetan-3-yl | C(=O)Me | Me | Me | O |
| C-16860 | Me(S) | single bond | H | oxetan-3-yl | C(=O)Me | Me | Me | O |
| C-16861 | H | single bond | Me | oxetan(3-CN)-3-yl | C(=O)Me | Cl | Me | O |
| C-16862 | Me | single bond | H | oxetan(3-CN)-3-yl | C(=O)Me | Cl | Me | O |
| C-16863 | Me(R) | single bond | H | oxetan(3-CN)-3-yl | C(=O)Me | Cl | Me | O |
| C-16864 | Me(S) | single bond | H | oxetan(3-CN)-3-yl | C(=O)Me | Cl | Me | O |
| C-16865 | H | single bond | Me | oxetan(3-CN)-3-yl | C(=O)Me | Me | Me | O |
| C-16866 | Me | single bond | H | oxetan(3-CN)-3-yl | C(=O)Me | Me | Me | O |
| C-16867 | Me(R) | single bond | H | oxetan(3-CN)-3-yl | C(=O)Me | Me | Me | O |
| C-16868 | Me(S) | single bond | H | oxetan(3-CN)-3-yl | C(=O)Me | Me | Me | O |
| C-16869 | H | single bond | Me | azetidin[1-C(=O)Me]-3-yl | C(=O)Me | Cl | Me | O |
| C-16870 | Me | single bond | H | azetidin[1-C(=O)Me]-3-yl | C(=O)Me | Cl | Me | O |

**[Table 612]**

| Compound Number | Y | W | Z | R¹ | R² | R³ | R¹⁰ | A |
|---|---|---|---|---|---|---|---|---|
| C-16871 | Me(R) | single bond | H | azetidin[1-C(=O)Me]-3-yl | C(=O)Me | Cl | Me | O |
| C-16872 | Me(S) | single bond | H | azetidin[1-C(=O)Me]-3-yl | C(=O)Me | Cl | Me | O |
| C-16873 | H | single bond | Me | azetidin[1-C(=O)Me]-3-yl | C(=O)Me | Me | Me | O |
| C-16874 | Me | single bond | H | azetidin[1-C(=O)Me]-3-yl | C(=O)Me | Me | Me | O |
| C-16875 | Me(R) | single bond | H | azetidin[1-C(=O)Me]-3-yl | C(=O)Me | Me | Me | O |
| C-16876 | Me(S) | single bond | H | azetidin[1-C(=O)Me]-3-yl | C(=O)Me | Me | Me | O |
| C-16877 | H | single bond | Me | azetidin[1-S(=O)₂Me]-3-yl | C(=O)Me | Cl | Me | O |
| C-16878 | Me | single bond | H | azetidin[1-S(=O)₂Me]-3-yl | C(=O)Me | Cl | Me | O |
| C-16879 | Me(R) | single bond | H | azetidin[1-S(=O)₂Me]-3-yl | C(=O)Me | Cl | Me | O |
| C-16880 | Me(S) | single bond | H | azetidin[1-S(=O)₂Me]-3-yl | C(=O)Me | Cl | Me | O |
| C-16881 | H | single bond | Me | azetidin[1-S(=O)₂Me]-3-yl | *C*(=O)Me | Me | Me | O |
| C-16882 | Me | single bond | H | azetidin[1-S(=O)₂Me]-3-yl | C(=O)Me | Me | Me | O |
| C-16883 | Me(R) | single bond | H | azetidin[1-S(=O)₂Me]-3-yl | C(=O)Me | Me | Me | O |
| C-16884 | Me(S) | single bond | H | azetidin[1-S(=O)₂Me]-3-yl | C(=O)Me | Me | Me | O |
| C-16885 | H | single bond | Me | azetidin[1-S(=O)₂CHF₂]-3-yl | C(=O)Me | Cl | Me | O |
| C-16886 | Me | single bond | H | azetidin[1-S(=O)₂CHF₂]-3-yl | C(=O)Me | Cl | Me | O |
| C-16887 | Me(R) | single bond | H | azetidin[1-S(=O)₂CHF₂]-3-yl | C(=O)Me | Cl | Me | O |
| C-16888 | Me(S) | single bond | H | azetidin[1-S(=O)₂CHF₂]-3-yl | C(=O)Me | Cl | Me | O |
| C-16889 | H | single bond | Me | azetidin[1-S(=O)₂CHF₂]-3-yl | C(=O)Me | Me | Me | O |
| C-16890 | Me | single bond | H | azetidin[1-S(=O)₂CHF₂]-3-yl | C(=O)Me | Me | Me | O |
| C-16891 | Me(R) | single bond | H | azetidin[1-S(=O)₂CHF₂]-3-yl | C(=O)Me | Me | Me | O |
| C-16892 | Me(S) | single bond | H | azetidin[1-S(=O)₂CHF₂]-3-yl | C(=O)Me | Me | Me | O |
| C-16893 | H | single bond | Me | *c*-Pen | C(=O)Me | Cl | Me | O |
| C-16894 | Me | single bond | H | *c*-Pen | C(=O)Me | Cl | Me | O |
| C-16895 | Me(R) | single bond | H | *c*-Pen | C(=O)Me | Cl | Me | O |
| C-16896 | Me(S) | single bond | H | *c*-Pen | C(=O)Me | Cl | Me | O |
| C-16897 | H | single bond | Me | *c*-Pen | C(=O)Me | Me | Me | O |
| C-16898 | Me | single bond | H | *c*-Pen | C(=O)Me | Me | Me | O |
| C-16899 | Me(R) | single bond | H | *c*-Pen | C(=O)Me | Me | Me | O |
| C-16900 | Me(S) | single bond | H | *c*-Pen | C(=O)Me | Me | Me | O |
| C-16901 | H | single bond | Me | tetra hydrofuran-3-yl | C(=O)Me | Cl | Me | O |
| C-16902 | Me | single bond | H | tetrahydrofuran-3-yl | C(=O)Me | Cl | Me | O |
| C-16903 | Me(R) | single bond | H | tetra hydrofuran-3-yl | C(=O)Me | Cl | Me | O |
| C-16904 | Me(S) | single bond | H | tetrahydrofuran-3-yl | C(=O)Me | Cl | Me | O |
| C-16905 | H | single bond | Me | tetrahydrofuran-3-yl | C(=O)Me | Me | Me | O |
| C-16906 | Me | single bond | H | tetrahydrofuran-3-yl | C(=O)Me | Me | Me | O |
| C-16907 | Me(R) | single bond | H | tetrahydrofuran-3-yl | C(=O)Me | Me | Me | O |
| C-16908 | Me(S) | single bond | H | tetrahydrofuran-3-yl | C(=O)Me | Me | Me | O |
| C-16909 | H | single bond | Me | tetrahydrothiophen-3-yl | C(=O)Me | Cl | Me | O |
| C-16910 | Me | single bond | H | tetrahydrothiophen-3-yl | C(=O)Me | Cl | Me | O |
| C-16911 | Me(R) | single bond | H | tetrahydrothiophen-3-yl | C(=O)Me | Cl | Me | O |
| C-16912 | Me(S) | single bond | H | tetrahydrothiophen-3-yl | C(=O)Me | Cl | Me | O |
| C-16913 | H | single bond | Me | tetrahydrothiophen-3-yl | C(=O)Me | Me | Me | O |
| C-16914 | Me | single bond | H | tetrahydrothiophen-3-yl | C(=O)Me | Me | Me | O |

**[Table 613]**

| Compound Number | Y | W | Z | R¹ | R² | R³ | R¹⁰ | A |
|---|---|---|---|---|---|---|---|---|
| C-16915 | Me(R) | single bond | H | tetrahydrothiophen-3-yl | C(=O)Me | Me | Me | O |
| C-16916 | Me(S) | single bond | H | tetrahydrothiophen-3-yl | C(=O)Me | Me | Me | O |
| C-16917 | H | single bond | Me | *c*-Pen(1-CN) | C(=O)Me | Cl | Me | O |
| C-16918 | Me | single bond | H | *c*-Pen(1-CN) | C(=O)Me | Cl | Me | O |
| C-16919 | Me(R) | single bond | H | *c*-Pen(1-CN) | C(=O)Me | Cl | Me | O |
| C-16920 | Me(S) | single bond | H | *c*-Pen(1-CN) | C(=O)Me | Cl | Me | O |
| C-16921 | H | single bond | Me | *c*-Pen(1-CN) | C(=O)Me | Me | Me | O |
| C-16922 | Me | single bond | H | *c*-Pen(1-CN) | C(=O)Me | Me | Me | O |
| C-16923 | Me(R) | single bond | H | *c*-Pen(1-CN) | C(=O)Me | Me | Me | O |
| C-16924 | Me(S) | single bond | H | *c*-Pen(1-CN) | C(=O)Me | Me | Me | O |
| C-16925 | H | single bond | Me | tetrahydrofuran(3-CN)-3-yl | C(=O)Me | Cl | Me | O |
| C-16926 | Me | single bond | H | tetrahydrofuran(3-CN)-3-yl | C(=O)Me | Cl | Me | O |
| C-16927 | Me(R) | single bond | H | tetrahydrofuran(3-CN)-3-yl | C(=O)Me | Cl | Me | O |
| C-16928 | Me(S) | single bond | H | tetrahydrofuran(3-CN)-3-yl | C(=O)Me | Cl | Me | O |
| C-16929 | H | single bond | Me | tetrahydrofuran(3-CN)-3-yl | C(=O)Me | Me | Me | O |
| C-16930 | Me | single bond | H | tetrahydrofuran(3-CN)-3-yl | C(=O)Me | Me | Me | O |
| C-16931 | Me(R) | single bond | H | tetrahydrofuran(3-CN)-3-yl | C(=O)Me | Me | Me | O |
| C-16932 | Me(S) | single bond | H | tetrahydrofuran(3-CN)-3-yl | C(=O)Me | Me | Me | O |
| C-16933 | H | single bond | Me | tetrahydrothiophen(3-CN)-3-yl | C(=O)Me | Cl | Me | O |
| C-16934 | Me | single bond | H | tetrahydrothiophen(3-CN)-3-yl | C(=O)Me | Cl | Me | O |
| C-16935 | Me(R) | single bond | H | tetrahydrothiophen(3-CN)-3-yl | C(=O)Me | Cl | Me | O |
| C-16936 | Me(S) | single bond | H | tetrahydrothiophen(3-CN)-3-yl | C(=O)Me | Cl | Me | O |
| C-16937 | H | single bond | Me | tetrahydrothiophen(3-CN)-3-yl | C(=O)Me | Me | Me | O |
| C-16938 | Me | single bond | H | tetrahydrothiophen(3-CN)-3-yl | C(=O)Me | Me | Me | O |
| C-16939 | Me(R) | single bond | H | tetrahydrothiophen(3-CN)-3-yl | C(=O)Me | Me | Me | O |
| C-16940 | Me(S) | single bond | H | tetrahydrothiophen(3-CN)-3-yl | C(=O)Me | Me | Me | O |
| C-16941 | H | single bond | Me | bicyclo[1.1.1]pentan-1-yl | C(=O)Me | Cl | Me | O |
| C-16942 | Me | single bond | H | bicyclo[1.1.1]pentan-1-yl | C(=O)Me | Cl | Me | O |
| C-16943 | Me(R) | single bond | H | bicyclo[1.1.1]pentan-1-yl | C(=O)Me | Cl | Me | O |
| C-16944 | Me(S) | single bond | H | bicyclo[1.1.1]pentan-1-yl | C(=O)Me | Cl | Me | O |
| C-16945 | H | single bond | Me | bicyclo[1.1.1]pentan-1-yl | C(=O)Me | Me | Me | O |
| C-16946 | Me | single bond | H | bicyclo[1.1.1]pentan-1-yl | C(=O)Me | Me | Me | O |
| C-16947 | Me(R) | single bond | H | bicyclo[1.1.1]pentan-1-yl | C(=O)Me | Me | Me | O |
| C-16948 | Me(S) | single bond | H | bicyclo[1.1.1]pentan-1-yl | C(=O)Me | Me | Me | O |
| C-16949 | H | single bond | Me | *c*-Hex | C(=O)Me | Cl | Me | O |
| C-16950 | Me | single bond | H | *c*-Hex | C(=O)Me | Cl | Me | O |
| C-16951 | Me(R) | single bond | H | *c*-Hex | C(=O)Me | Cl | Me | O |
| C-16952 | Me(S) | single bond | H | *c*-Hex | C(=O)Me | Cl | Me | O |
| C-16953 | H | single bond | Me | c-Hex | C(=O)Me | Me | Me | O |
| C-16954 | Me | single bond | H | *c*-Hex | C(=O)Me | Me | Me | O |
| C-16955 | Me(R) | single bond | H | *c*-Hex | C(=O)Me | Me | Me | O |
| C-16956 | Me(S) | single bond | H | *c*-Hex | C(=O)Me | Me | Me | O |
| C-16957 | H | single bond | Me | tetrahydropyran-4-yl | C(=O)Me | Cl | Me | O |
| C-16958 | Me | single bond | H | tetrahydropyran-4-yl | C(=O)Me | Cl | Me | O |

**[Table 614]**

| Compound Number | Y | W | Z | R¹ | R² | R³ | R¹⁰ | A |
|---|---|---|---|---|---|---|---|---|
| C-16959 | Me(R) | single bond | H | tetrahydropyran-4-yl | C(=O)Me | Cl | Me | O |
| C-16960 | Me(S) | single bond | H | tetrahydropyran-4-yl | C(=O)Me | Cl | Me | O |
| C-16961 | H | single bond | Me | tetrahydropyran-4-yl | C(=O)Me | Me | Me | O |
| C-16962 | Me | single bond | H | tetrahydropyran-4-yl | C(=O)Me | Me | Me | O |
| C-16963 | Me(R) | single bond | H | tetrahydropyran-4-yl | C(=O)Me | Me | Me | O |
| C-16964 | Me(S) | single bond | H | tetrahydropyran-4-yl | C(=O)Me | Me | Me | O |
| C-16965 | H | single bond | Me | tetrahydrothiopyran-4-yl | C(=O)Me | Cl | Me | O |
| C-16966 | Me | single bond | H | tetrahydrothiopyran-4-yl | C(=O)Me | Cl | Me | O |
| C-16967 | Me(R) | single bond | H | tetrahydrothiopyran-4-yl | C(=O)Me | Cl | Me | O |
| C-16968 | Me(S) | single bond | H | tetrahydrothiopyran-4-yl | C(=O)Me | Cl | Me | O |
| C-16969 | H | single bond | Me | tetrahydrothiopyran-4-yl | C(=O)Me | Me | Me | O |
| C-16970 | Me | single bond | H | tetrahydrothiopyran-4-yl | C(=O)Me | Me | Me | O |
| C-16971 | Me(R) | single bond | H | tetrahydrothiopyran-4-yl | C(=O)Me | Me | Me | O |
| C-16972 | Me(S) | single bond | H | tetrahydrothiopyran-4-yl | C(=O)Me | Me | Me | O |
| C-16973 | H | single bond | Me | *c*-Hex(1-CN) | C(=O)Me | Cl | Me | O |
| C-16974 | Me | single bond | H | *c*-Hex(1-CN) | C(=O)Me | Cl | Me | O |
| C-16975 | Me(R) | single bond | H | *c*-Hex(1-CN) | C(=O)Me | Cl | Me | O |
| C-16976 | Me(S) | single bond | H | *c*-Hex(1-CN) | C(=O)Me | Cl | Me | O |
| C-16977 | H | single bond | Me | *c*-Hex(1-CN) | C(=O)Me | Me | Me | O |
| C-16978 | Me | single bond | H | *c*-Hex(1-CN) | C(=O)Me | Me | Me | O |
| C-16979 | Me(R) | single bond | H | *c*-Hex(1-CN) | C(=O)Me | Me | Me | O |
| C-16980 | Me(S) | single bond | H | *c*-Hex(1-CN) | C(=O)Me | Me | Me | O |
| C-16981 | H | single bond | Me | tetrahydropyran(4-CN)-4-yl | C(=O)Me | Cl | Me | O |
| C-16982 | Me | single bond | H | tetrahydropyran(4-CN)-4-yl | C(=O)Me | Cl | Me | O |
| C-16983 | Me(R) | single bond | H | tetrahydropyran(4-CN)-4-yl | C(=O)Me | Cl | Me | O |
| C-16984 | Me(S) | single bond | H | tetrahydropyran(4-CN)-4-yl | C(=O)Me | Cl | Me | O |
| C-16985 | H | single bond | Me | tetrahydropyran(4-CN)-4-yl | C(=O)Me | Me | Me | O |
| C-16986 | Me | single bond | H | tetrahydropyran(4-CN)-4-yl | C(=O)Me | Me | Me | O |
| C-16987 | Me(R) | single bond | H | tetrahydropyran(4-CN)-4-yl | C(=O)Me | Me | Me | O |
| C-16988 | Me(S) | single bond | H | tetrahydropyran(4-CN)-4-yl | C(=O)Me | Me | Me | O |
| C-16989 | H | single bond | Me | tetrahydrothiopyran(4-CN)-4-yl | C(=O)Me | Cl | Me | O |
| C-16990 | Me | single bond | H | tetrahydrothiopyran(4-CN)-4-yl | C(=O)Me | Cl | Me | O |
| C-16991 | Me(R) | single bond | H | tetrahydrothiopyran(4-CN)-4-yl | C(=O)Me | Cl | Me | O |
| C-16992 | Me(S) | single bond | H | tetrahydrothiopyran(4-CN)-4-yl | C(=O)Me | Cl | Me | O |
| C-16993 | H | single bond | Me | tetrahydrothiopyran(4-CN)-4-yl | C(=O)Me | Me | Me | O |
| C-16984 | Me | single bond | H | tetrahydrothiopyran(4-CN)-4-yl | C(=O)Me | Me | Me | O |
| C-16995 | Me(R) | single bond | H | tetrahydrothiopyran(4-CN)-4-yl | C(=O)Me | Me | Me | O |
| C-16996 | Me(S) | single bond | H | tetrahydrothiopyran(4-CN)-4-yl | C(=O)Me | Me | Me | O |
| C-16997 | H | single bond | Me | CH₂(*c*-Pr) | C(=O)Me | Cl | Me | O |
| C-16998 | Me | single bond | H | CH₂(*c*-Pr) | C(=O)Me | Cl | Me | O |
| C-16999 | Me(R) | single bond | H | CH₂(*c*-Pr) | C(=O)Me | Cl | Me | O |
| C-17000 | Me(S) | single bond | H | CH₂(*c*-Pr) | C(=O)Me | Cl | Me | O |
| C-17001 | H | single bond | Me | CH₂(*c*-Pr) | C(=O)Me | Me | Me | O |
| C-17002 | Me | single bond | H | CH₂(*c* -Pr) | C(=O)Me | Me | Me | O |

**[Table 615]**

| Compound Number | Y | W | Z | R¹ | R² | R³ | R¹⁰ | A |
|---|---|---|---|---|---|---|---|---|
| C-17003 | Me(R) | single bond | H | CH₂(*c*-Pr) | C(=O)Me | Me | Me | O |
| C-17004 | Me(S) | single bond | H | CH₂(*c*-Pr) | C(=O)Me | Me | Me | O |
| C-17005 | H | single bond | Me | CH₂[*c*-Pr(1-CN)] | C(=O)Me | Cl | Me | O |
| C-17006 | Me | single bond | H | CH₂[*c*-Pr(1-CN)] | C(=O)Me | Cl | Me | O |
| C-17007 | Me(R) | single bond | H | CH₂[*c*-Pr(1-CN)] | C(=O)Me | Cl | Me | O |
| C-17008 | Me(S) | single bond | H | CH₂[*c*-Pr(1-CN)] | C(=O)Me | Cl | Me | O |
| C-17009 | H | single bond | Me | CH₂[*c*-Pr(1-CN)] | C(=O)Me | Me | Me | O |
| C-17010 | Me | single bond | H | CH₂[*c*-Pr(1-CN)] | C(=O)Me | Me | Me | O |
| C-17011 | Me(R) | single bond | H | CH₂[*c*-Pr(1-CN)] | C(=O)Me | Me | Me | O |
| C-17012 | Me(S) | single bond | H | CH₂[*c*-Pr(1-CN)] | C(=O)Me | Me | Me | O |
| C-17013 | H | single bond | Me | CH(Me)(*c*-Pr) | C(=O)Me | Cl | Me | O |
| C-17014 | Me | single bond | H | CH(Me)(*c*-Pr) | C(=O)Me | Cl | Me | O |
| C-17015 | Me(R) | single bond | H | CH(Me)(*c*-Pr) | C(=O)Me | Cl | Me | O |
| C-17016 | Me(S) | single bond | H | CH(Me)(*c*-Pr) | C(=O)Me | Cl | Me | O |
| C-17017 | H | single bond | Me | CH(Me)(*c*-Pr) | C(=O)Me | Me | Me | O |
| C-17018 | Me | single bond | H | CH(Me)(*c*-Pr) | C(=O)Me | Me | Me | O |
| C-17019 | Me(R) | single bond | H | CH(Me)(*c*-Pr) | C(=O)Me | Me | Me | O |
| C-17020 | Me(S) | single bond | H | CH(Me)(*c*-Pr) | C(=O)Me | Me | Me | O |
| C-17021 | H | single bond | Me | CH(Me)CH₂OMe | C(=O)Me | Cl | Me | O |
| C-17022 | Me | single bond | H | CH(Me)CH₂OMe | C(=O)Me | Cl | Me | O |
| C-17023 | Me(R) | single bond | H | CH(Me)CH₂OMe | C(=O)Me | Cl | Me | O |
| C-17024 | Me(S) | single bond | H | CH(Me)CH₂OMe | C(=O)Me | Cl | Me | O |
| C-17025 | H | single bond | Me | CH(Me)CH₂OMe | C(=O)Me | Me | Me | O |
| C-17026 | Me | single bond | H | CH(Me)CH₂OMe | C(=O)Me | Me | Me | O |
| C-17027 | Me(R) | single bond | H | CH(Me)CH₂OMe | C(=O)Me | Me | Me | O |
| C-17028 | Me(S) | single bond | H | CH(Me)CH₂OMe | C(=O)Me | Me | Me | O |
| C-17029 | H | single bond | Me | CH₂CH=CH₂ | C(=O)Me | Cl | Me | O |
| C-17030 | Me | single bond | H | CH₂CH=CH₂ | C(=O)Me | Cl | Me | O |
| C-17031 | Me(R) | single bond | H | CH₂CH=CH₂ | C(=O)Me | Cl | Me | O |
| C-17032 | Me(S) | single bond | H | CH₂CH=CH₂ | C(=O)Me | Cl | Me | O |
| C-17033 | H | single bond | Me | CH₂CH=CH₂ | C(=O)Me | Me | Me | O |
| C-17034 | Me | single bond | H | CH₂CH=CH₂ | C(=O)Me | Me | Me | O |
| C-17035 | Me(R) | single bond | H | CH₂CH=CH₂ | C(=O)Me | Me | Me | O |
| C-17036 | Me(S) | single bond | H | CH₂CH=CH₂ | C(=O)Me | Me | Me | O |
| C-17037 | H | single bond | Me | CH₂C≡CH | C(=O)Me | Cl | Me | O |
| C-17038 | Me | single bond | H | CH₂C≡CH | C(=O)Me | Cl | Me | O |
| C-17039 | Me(R) | single bond | H | CH₂C≡CH | C(=O)Me | Cl | Me | O |
| C-17040 | Me(S) | single bond | H | CH₂C≡CH | C(=O)Me | Cl | Me | O |
| C-17041 | H | single bond | Me | CH₂C≡CH | C(=O)Me | Me | Me | O |
| C-17042 | Me | single bond | H | CH₂C≡CH | C(=O)Me | Me | Me | O |
| C-17043 | Me(R) | single bond | H | CH₂C≡CH | C(=O)Me | Me | Me | O |
| C-17044 | Me(S) | single bond | H | CH₂C≡CH | C(=O)Me | Me | Me | O |
| C-17045 | H | single bond | Me | CH₂CN | C(=O)Me | Cl | Me | O |
| C-17046 | Me | single bond | H | CH₂CN | C(=O)Me | Cl | Me | O |

**[Table 616]**

| Compound Number | Y | W | Z | R¹ | R² | R³ | R¹⁰ | A |
|---|---|---|---|---|---|---|---|---|
| C-17047 | Me(R) | single bond | H | CH₂CN | C(=O)Me | Cl | Me | O |
| C-17048 | Me(S) | single bond | H | CH₂CN | C(=O)Me | Cl | Me | O |
| C-17049 | H | single bond | Me | CH₂CN | C(=O)Me | Me | Me | O |
| C-17050 | Me | single bond | H | CH₂CN | C(=O)Me | Me | Me | O |
| C-17051 | Me(R) | single bond | H | CH₂CN | C(=O)Me | Me | Me | O |
| C-17052 | Me(S) | single bond | H | CH₂CN | C(=O)Me | Me | Me | O |
| C-17053 | H | single bond | Me | CH(Me)CN | C(=O)Me | Cl | Me | O |
| C-17054 | Me | single bond | H | CH(Me)CN | C(=O)Me | Cl | Me | O |
| C-17055 | Me(R) | single bond | H | CH(Me)CN | C(=O)Me | Cl | Me | O |
| C-17056 | Me(S) | single bond | H | CH(Me)CN | C(=O)Me | Cl | Me | O |
| C-17057 | H | single bond | Me | CH(Me)CN | C(=O)Me | Me | Me | O |
| C-17058 | Me | single bond | H | CH(Me)CN | C(=O)Me | Me | Me | O |
| C-17059 | Me(R) | single bond | H | CH(Me)CN | C(=O)Me | Me | Me | O |
| C-17060 | Me(S) | single bond | H | CH(Me)CN | C(=O)Me | Me | Me | O |
| C-17061 | H | single bond | Me | C(Me)₂CN | C(=O)Me | Cl | Me | O |
| C-17062 | Me | single bond | H | C(Me)₂CN | C(=O)Me | Cl | Me | O |
| C-17063 | Me(R) | single bond | H | C(Me)₂CN | C(=O)Me | Cl | Me | O |
| C-17064 | Me(S) | single bond | H | C(Me)₂CN | C(=O)Me | Cl | Me | O |
| C-17065 | H | single bond | Me | C(Me)₂CN | C(=O)Me | Me | Me | O |
| C-17066 | Me | single bond | H | C(Me)₂CN | C(=O)Me | Me | Me | O |
| C-17067 | Me(R) | single bond | H | C(Me)₂CN | C(=O)Me | Me | Me | O |
| C-17068 | Me(S) | single bond | H | C(Me)₂CN | C(=O)Me | Me | Me | O |
| C-17069 | H | single bond | Me | C(Me)(*i*-Pr)CN | C(=O)Me | Cl | Me | O |
| C-17070 | Me | single bond | H | C(Me)(*i*-Pr)CN | C(=O)Me | Cl | Me | O |
| C-17071 | Me(R) | single bond | H | C(Me)(*i*-Pr)CN | C(=O)Me | Cl | Me | O |
| C-17072 | Me(S) | single bond | H | C(Me)(*i*-Pr)CN | C(=O)Me | Cl | Me | O |
| C-17073 | H | single bond | Me | C(Me)(*i*-Pr)CN | C(=O)Me | Me | Me | O |
| C-17074 | Me | single bond | H | C(Me)(*i*-Pr)CN | C(=O)Me | Me | Me | O |
| C-17075 | Me(R) | single bond | H | C(Me)(*i*-Pr)CN | C(=O)Me | Me | Me | O |
| C-17076 | Me(S) | single bond | H | C(Me)(*i*-Pr)CN | C(=O)Me | Me | Me | O |
| C-17077 | H | single bond | Me | OMe | C(=O)Me | Cl | Me | O |
| C-17078 | Me | single bond | H | OMe | C(=O)Me | Cl | Me | O |
| C-17079 | Me(R) | single bond | H | OMe | C(=O)Me | Cl | Me | O |
| C-17080 | Me(S) | single bond | H | OMe | C(=O)Me | Cl | Me | O |
| C-17081 | H | single bond | Me | OMe | C(=O)Me | Me | Me | O |
| C-17082 | Me | single bond | H | OMe | C(=O)Me | Me | Me | O |
| C-17083 | Me(R) | single bond | H | OMe | C(=O)Me | Me | Me | O |
| C-17084 | Me(S) | single bond | H | OMe | C(=O)Me | Me | Me | O |
| C-17085 | H | single bond | Me | CH=NOMe | C(=O)Me | Cl | Me | O |
| C-17086 | Me | single bond | H | CH=NOMe | C(=O)Me | Cl | Me | O |
| C-17087 | Me(R) | single bond | H | CH=NOMe | C(=O)Me | Cl | Me | O |
| C-17088 | Me(S) | single bond | H | CH=NOMe | C(=O)Me | Cl | Me | O |
| C-17089 | H | single bond | Me | CH=NOMe | C(=O)Me | Me | Me | O |
| C-17090 | Me | single bond | H | CH=NOMe | C(=O)Me | Me | Me | O |

**[Table 617]**

| Compound Number | Y | W | Z | R¹ | R² | R³ | R¹⁰ | A |
|---|---|---|---|---|---|---|---|---|
| C-17091 | Me(R) | single bond | H | CH=NOMe | C(=O)Me | Me | Me | O |
| C-17092 | Me(S) | single bond | H | CH=NOMe | C(=O)Me | Me | Me | O |
| C-17093 | H | single bond | Me | morpholin-4-yl | C(=O)Me | Cl | Me | O |
| C-17094 | Me | single bond | H | morpholin-4-yl | C(=O)Me | Cl | Me | O |
| C-17095 | Me(R) | single bond | H | morpholin-4-yl | C(=O)Me | Cl | Me | O |
| C-17096 | Me(S) | single bond | H | morpholin-4-yl | C(=O)Me | Cl | Me | O |
| C-17097 | H | single bond | Me | morpholin-4-yl | C(=O)Me | Me | Me | O |
| C-17098 | Me | single bond | H | morpholin-4-yl | C(=O)Me | Me | Me | O |
| C-17099 | Me(R) | single bond | H | morpholin-4-yl | C(=O)Me | Me | Me | O |
| C-17100 | Me(S) | single bond | H | morpholin-4-yl | C(=O)Me | Me | Me | O |
| C-17101 | H | single bond | Me | Ph | C(=O)Me | Cl | Me | O |
| C-17102 | Me | single bond | H | Ph | C(=O)Me | Cl | Me | O |
| C-17103 | Me(R) | single bond | H | Ph | C(=O)Me | Cl | Me | O |
| C-17104 | Me(S) | single bond | H | Ph | C(=O)Me | Cl | Me | O |
| C-17105 | H | single bond | Me | Ph | C(=O)Me | Me | Me | O |
| C-17106 | Me | single bond | H | Ph | C(=O)Me | Me | Me | O |
| C-17107 | Me(R) | single bond | H | Ph | C(=O)Me | Me | Me | O |
| C-17108 | Me(S) | single bond | H | Ph | C(=O)Me | Me | Me | O |
| C-17109 | H | single bond | Me | CH₂Ph | C(=O)Me | Cl | Me | O |
| C-17110 | Me | single bond | H | CH₂Ph | C(=O)Me | Cl | Me | O |
| C-17111 | Me(R) | single bond | H | CH₂Ph | C(=O)Me | Cl | Me | O |
| C-17112 | Me(S) | single bond | H | CH₂Ph | C(=O)Me | Cl | Me | O |
| C-17113 | H | single bond | Me | CH₂Ph | C(=O)Me | Me | Me | O |
| C-17114 | Me | single bond | H | CH₂Ph | C(=O)Me | Me | Me | O |
| C-17115 | Me(R) | single bond | H | CH₂Ph | C(=O)Me | Me | Me | O |
| C-17116 | Me(S) | single bond | H | CH₂Ph | C(=O)Me | Me | Me | O |
| C-17117 | H | single bond | Me | thiazole-2-yl | C(=O)Me | Cl | Me | O |
| C-17118 | Me | single bond | H | thiazole-2-yl | C(=O)Me | Cl | Me | O |
| C-17119 | Me(R) | single bond | H | thiazole-2-yl | C(=O)Me | Cl | Me | O |
| C-17120 | Me(S) | single bond | H | thiazole-2-yl | C(=O)Me | Cl | Me | O |
| C-17121 | H | single bond | Me | thiazole-2-yl | C(=O)Me | Me | Me | O |
| C-17122 | Me | single bond | H | thiazole-2-yl | C(=O)Me | Me | Me | O |
| C-17123 | Me(R) | single bond | H | thiazole-2-yl | C(=O)Me | Me | Me | O |
| C-17124 | Me(S) | single bond | H | thiazole-2-yl | C(=O)Me | Me | Me | O |
| C-17125 | H | single bond | Me | 1-Me-pyrazol-3-yl | C(=O)Me | Cl | Me | O |
| C-17126 | Me | single bond | H | 1-Me-pyrazol-3-yl | C(=O)Me | Cl | Me | O |
| C-17127 | Me(R) | single bond | H | 1-Me-pyrazol-3-yl | C(=O)Me | Cl | Me | O |
| C-17128 | Me(S) | single bond | H | 1-Me-pyrazol-3-yl | C(=O)Me | Cl | Me | O |
| C-17129 | H | single bond | Me | 1-Me-pyrazol-3-yl | C(=O)Me | Me | Me | O |
| C-17130 | Me | single bond | H | 1-Me-pyrazol-3-yl | C(=O)Me | Me | Me | O |
| C-17131 | Me(R) | single bond | H | 1-Me-pyrazol-3-yl | C(=O)Me | Me | Me | O |
| C-17132 | Me(S) | single bond | H | 1-Me-pyrazol-3-yl | C(=O)Me | Me | Me | O |
| C-17133 | H | single bond | Me | 1-Me-pyrazol-5-yl | C(=O)Me | Cl | Me | O |
| C-17134 | Me | single bond | H | 1-Me-pyrazol-5-yl | C(=O)Me | Cl | Me | O |

**[Table 618]**

| Compound Number | Y | W | Z | R¹ | R² | R³ | R¹⁰ | A |
|---|---|---|---|---|---|---|---|---|
| C-17135 | Me(R) | single bond | H | 1-Me-pyrazol-5-yl | C(=O)Me | Cl | Me | O |
| C-17136 | Me(S) | single bond | H | 1-Me-pyrazol-5-yl | C(=O)Me | Cl | Me | O |
| C-17137 | H | single bond | Me | 1-Me-pyrazol-5-yl | C(=O)Me | Me | Me | O |
| C-17138 | Me | single bond | H | 1-Me-pyrazol-5-yl | C(=O)Me | Me | Me | O |
| C-17139 | Me(R) | single bond | H | 1-Me-pyrazol-5-yl | C(=O)Me | Me | Me | O |
| C-17140 | Me(S) | single bond | H | 1-Me-pyrazol-5-yl | C(=O)Me | Me | Me | O |
| C-17141 | H | single bond | Me | CH₂(pyrimidin-2-yl) | C(=O)Me | Cl | Me | O |
| C-17142 | Me | single bond | H | CH₂(pyrimidin-2-yl) | C(=O)Me | Cl | Me | O |
| C-17143 | Me(R) | single bond | H | CH₂(pyrimidin-2-yl) | C(=O)Me | Cl | Me | O |
| C-17144 | Me(S) | single bond | H | CH₂(pyrimidin-2-yl) | C(=O)Me | Cl | Me | O |
| C-17145 | H | single bond | Me | CH₂(pyrimidin-2-yl) | C(=O)Me | Me | Me | O |
| C-17146 | Me | single bond | H | CH₂(pyrimidin-2-yl) | C(=O)Me | Me | Me | O |
| C-17147 | Me(R) | single bond | H | CH₂(pyrimidin-2-yl) | C(=O)Me | Me | Me | O |
| C-17148 | Me(S) | single bond | H | CH₂(pyrimidin-2-yl) | C(=O)Me | Me | Me | O |
| C-17149 | H | single bond | Me | Me | C(=O)Me | Cl | CH₂OMe | O |
| C-17150 | Me | single bond | H | Me | C(=O)Me | Cl | CH₂OMe | O |
| C-17151 | Me(R) | single bond | H | Me | C(=O)Me | Cl | CH₂OMe | O |
| C-17152 | Me(S) | single bond | H | Me | C(=O)Me | Cl | CH₂OMe | O |
| C-17153 | H | single bond | Me | Me | C(=O)Me | Me | CH₂OMe | O |
| C-17154 | Me | single bond | H | Me | C(=O)Me | Me | CH₂OMe | O |
| C-17155 | Me(R) | single bond | H | Me | C(=O)Me | Me | CH₂OMe | O |
| C-17156 | Me(S) | single bond | H | Me | C(=O)Me | Me | CH₂OMe | O |
| C-17157 | H | single bond | Me | CH₂CHF₂ | C(=O)Me | Cl | CH₂OMe | O |
| C-17158 | Me | single bond | H | CH₂CHF₂ | C(=O)Me | Cl | CH₂OMe | O |
| C-17159 | Me(R) | single bond | H | CH₂CHF₂ | C(=O)Me | Cl | CH₂OMe | O |
| C-17160 | Me(S) | single bond | H | CH₂CHF₂ | C(=O)Me | Cl | CH₂OMe | O |
| C-17161 | H | single bond | Me | CH₂CHF₂ | C(=O)Me | Me | CH₂OMe | O |
| C-17162 | Me | single bond | H | CH₂CHF₂ | C(=O)Me | Me | CH₂OMe | O |
| C-17163 | Me(R) | single bond | H | CH₂CHF₂ | C(=O)Me | Me | CH₂OMe | O |
| C-17164 | Me(S) | single bond | H | CH₂CHF₂ | C(=O)Me | Me | CH₂OMe | O |
| C-17165 | H | single bond | Me | *n*-Pr | C(=O)Me | Cl | CH₂OMe | O |
| C-17166 | Me | single bond | H | *n*-Pr | C(=O)Me | Cl | CH₂OMe | O |
| C-17167 | Me(R) | single bond | H | *n*-Pr | C(=O)Me | Cl | CH₂OMe | O |
| C-17168 | Me(S) | single bond | H | *n*-Pr | C(=O)Me | Cl | CH₂OMe | O |
| C-17169 | H | single bond | Me | *n*-Pr | C(=O)Me | Me | CH₂OMe | O |
| C-17170 | Me | single bond | H | *n*-Pr | C(=O)Me | Me | CH₂OMe | O |
| C-17171 | Me(R) | single bond | H | *n*-Pr | C(=O)Me | Me | CH₂OMe | O |
| C-17172 | Me(S) | single bond | H | *n*-Pr | C(=O)Me | Me | CH₂OMe | O |
| C-17173 | H | single bond | Me | *i*-Pr | C(=O)Me | Cl | CH₂OMe | O |
| C-17174 | Me | single bond | H | *i*-Pr | C(=O)Me | Cl | CH₂OMe | O |
| C-17175 | Me(R) | single bond | H | *i*-Pr | C(=O)Me | Cl | CH₂OMe | O |
| C-17176 | Me(S) | single bond | H | *i*-Pr | C(=O)Me | Cl | CH₂OMe | O |
| C-17177 | H | single bond | Me | *i*-Pr | C(=O)Me | Me | CH₂OMe | O |
| C-17178 | Me | single bond | H | *i*-Pr | C(=O)Me | Me | CH₂OMe | O |

**[Table 619]**

| Compound Number | Y | W | Z | R¹ | R² | R³ | R¹⁰ | A |
|---|---|---|---|---|---|---|---|---|
| C-17179 | Me(R) | single bond | H | *i*-Pr | C(=O)Me | Me | CH₂OMe | O |
| C-17180 | Me(S) | single bond | H | *i*-Pr | C(=O)Me | Me | CH₂OMe | O |
| C-17181 | H | single bond | Me | *c*-Pr(1-F) | C(=O)Me | Cl | CH₂OMe | O |
| C-17182 | Me | single bond | H | *c*-Pr(1-F) | C(=O)Me | Cl | CH₂OMe | O |
| C-17183 | Me(R) | single bond | H | *c*-Pr(1-F) | C(=O)Me | Cl | CH₂OMe | O |
| C-17184 | Me(S) | single bond | H | *c*-Pr(1-F) | C(=O)Me | Cl | CH₂OMe | O |
| C-17185 | H | single bond | Me | *c*-Pr(1-F) | C(=O)Me | Me | CH₂OMe | O |
| C-17186 | Me | single bond | H | *c*-Pr(1-F) | C(=O)Me | Me | CH₂OMe | O |
| C-17187 | Me(R) | single bond | H | *c*-Pr(1-F) | C(=O)Me | Me | CH₂OMe | O |
| C-17188 | Me(S) | single bond | H | *c*-Pr(1-F) | C(=O)Me | Me | CH₂OMe | O |
| C-17189 | H | single bond | Me | *c*-Pr(2,2-F₂) | C(=O)Me | Cl | CH₂OMe | O |
| C-17190 | Me | single bond | H | *c*-Pr(2,2-F₂) | C(=O)Me | Cl | CH₂OMe | O |
| C-17191 | Me(R) | single bond | H | *c*-Pr(2,2-F₂) | C(=O)Me | Cl | CH₂OMe | O |
| C-17192 | Me(S) | single bond | H | *c*-Pr(2,2-F₂) | C(=O)Me | Cl | CH₂OMe | O |
| C-17193 | H | single bond | Me | *c*-Pr(2,2-F₂) | C(=O)Me | Me | CH₂OMe | O |
| C-17194 | Me | single bond | H | *c*-Pr(2,2-F₂) | C(=O)Me | Me | CH₂OMe | O |
| C-17195 | Me(R) | single bond | H | *c*-Pr(2,2-F₂) | C(=O)Me | Me | CH₂OMe | O |
| C-17196 | Me(S) | single bond | H | *c*-Pr(2,2-F₂) | C(=O)Me | Me | CH₂OMe | O |
| C-17197 | H | single bond | Me | *c*-Pr(1-Cl) | C(=O)Me | Cl | CH₂OMe | O |
| C-17198 | Me | single bond | H | *c*-Pr(1-Cl) | C(=O)Me | Cl | CH₂OMe | O |
| C-17199 | Me(R) | single bond | H | *c*-Pr(1-Cl) | C(=O)Me | Cl | CH₂OMe | O |
| C-17200 | Me(S) | single bond | H | *c*-Pr(1-Cl) | C(=O)Me | Cl | CH₂OMe | O |
| C-17201 | H | single bond | Me | *c*-Pr(1-Cl) | C(=O)Me | Me | CH₂OMe | O |
| C-17202 | Me | single bond | H | *c*-Pr(1-Cl) | C(=O)Me | Me | CH₂OMe | O |
| C-17203 | Me(R) | single bond | H | *c*-Pr(1-Cl) | C(=O)Me | Me | CH₂OMe | O |
| C-17204 | Me(S) | single bond | H | *c*-Pr(1-Cl) | C(=O)Me | Me | CH₂OMe | O |
| C-17205 | H | single bond | Me | *c*-Pr(1-Br) | C(=O)Me | Cl | CH₂OMe | O |
| C-17206 | Me | single bond | H | *c*-Pr(1-Br) | C(=O)Me | Cl | CH₂OMe | O |
| C-17207 | Me(R) | single bond | H | *c*-Pr(1-Br) | C(=O)Me | Cl | CH₂OMe | O |
| C-17208 | Me(S) | single bond | H | *c*-Pr(1-Br) | C(=O)Me | Cl | CH₂OMe | O |
| C-17209 | H | single bond | Me | *c*-Pr(1-Br) | C(=O)Me | Me | CH₂OMe | O |
| C-17210 | Me | single bond | H | *c*-Pr(1-Br) | C(=O)Me | Me | CH₂OMe | O |
| C-17211 | Me(R) | single bond | H | *c*-Pr(1-Br) | C(=O)Me | Me | CH₂OMe | O |
| C-17212 | Me(S) | single bond | H | *c*-Pr(1-Br) | C(=O)Me | Me | CH₂OMe | O |
| C-17213 | H | single bond | Me | *c*-Pr(1-Me) | C(=O)Me | Cl | CH₂OMe | O |
| C-17214 | Me | single bond | H | *c*-Pr(1-Me) | C(=O)Me | Cl | CH₂OMe | O |
| C-17215 | Me(R) | single bond | H | *c*-Pr(1-Me) | C(=O)Me | Cl | CH₂OMe | O |
| C-17216 | Me(S) | single bond | H | *c*-Pr(1-Me) | C(=O)Me | Cl | CH₂OMe | O |
| C-17217 | H | single bond | Me | *c*-Pr(1-Me) | C(=O)Me | Me | CH₂OMe | O |
| C-17218 | Me | single bond | H | *c*-Pr(1-Me) | C(=O)Me | Me | CH₂OMe | O |
| C-17219 | Me(R) | single bond | H | *c*-Pr(1-Me) | C(=O)Me | Me | CH₂OMe | O |
| C-17220 | Me(S) | single bond | H | *c*-Pr(1-Me) | C(=O)Me | Me | CH₂OMe | O |
| C-17221 | H | single bond | Me | *c*-Pr(1-CH₂OH) | C(=O)Me | Cl | CH₂OMe | O |
| C-17222 | Me | single bond | H | *c*-Pr(1-CH₂OH) | C(=O)Me | Cl | CH₂OMe | O |

**[Table 620]**

| Compound Number | Y | W | Z | R¹ | R² | R³ | R¹⁰ | A |
|---|---|---|---|---|---|---|---|---|
| C-17223 | Me(R) | single bond | H | *c*-Pr(1-CH₂OH) | C(=O)Me | Cl | CH₂OMe | O |
| C-17224 | Me(S) | single bond | H | *c*-Pr(1-CH₂OH) | C(=O)Me | Cl | CH₂OMe | O |
| C-17225 | H | single bond | Me | *c*-Pr(1-CH₂OH) | C(=O)Me | Me | CH₂OMe | O |
| C-17226 | Me | single bond | H | *c*-Pr(1-CH₂OH) | C(=O)Me | Me | CH₂OMe | O |
| C-17227 | Me(R) | single bond | H | *c*-Pr(1-CH₂OH) | C(=O)Me | Me | CH₂OMe | O |
| C-17228 | Me(S) | single bond | H | *c*-Pr(1-CH₂OH) | C(=O)Me | Me | CH₂OMe | O |
| C-17229 | H | single bond | Me | *c*-Pr[1-CH₂₀S(=O)₂Me] | C(=O)Me | Cl | CH₂OMe | O |
| C-17230 | Me | single bond | H | *c*-Pr[1-CH₂₀S(=O)₂Me] | C(=O)Me | Cl | CH₂OMe | O |
| C-17231 | Me(R) | single bond | H | *c*-Pr[1-CH₂₀S(=O)₂Me] | C(=O)Me | Cl | CH₂OMe | O |
| C-17232 | Me(S) | single bond | H | *c*-Pr[1-CH₂₀S(=O)₂Me] | C(=O)Me | Cl | CH₂OMe | O |
| C-17233 | H | single bond | Me | *c*-Pr[1-CH₂₀S(=O)₂Me] | C(=O)Me | Me | CH₂OMe | O |
| C-17234 | Me | single bond | H | *c*-Pr[1-CH₂₀S(=O)₂Me] | C(=O)Me | Me | CH₂OMe | O |
| C-17235 | Me(R) | single bond | H | *c*-Pr[1-CH₂₀S(=O)₂Me] | C(=O)Me | Me | CH₂OMe | O |
| C-17236 | Me(S) | single bond | H | *c*-Pr[1-CH₂OS(=O)₂Me] | C(=O)Me | Me | CH₂OMe | O |
| C-17237 | H | single bond | Me | *c*-Pr(1-CH₂OMe) | C(=O)Me | Cl | CH₂OMe | O |
| C-17238 | Me | single bond | H | *c*-Pr(1-CH₂OMe) | C(=O)Me | Cl | CH₂OMe | O |
| C-17239 | Me(R) | single bond | H | *c*-Pr(1-CH₂OMe) | C(=O)Me | Cl | CH₂OMe | O |
| C-17240 | Me(S) | single bond | H | *c*-Pr(1-CH₂0Me) | C(=O)Me | Cl | CH₂OMe | O |
| C-17241 | H | single bond | Me | *c*-Pr(1-CH₂OMe) | C(=O)Me | Me | CH₂OMe | O |
| C-17242 | Me | single bond | H | *c*-Pr(1-CH₂OMe) | C(=O)Me | Me | CH₂OMe | O |
| C-17243 | Me(R) | single bond | H | *c*-Pr(1-CH₂OMe) | C(=O)Me | Me | CH₂OMe | O |
| C-17244 | Me(S) | single bond | H | *c*-Pr(1-CH₂OMe) | C(=O)Me | Me | CH₂OMe | O |
| C-17245 | H | single bond | Me | *c*-Pr(1-CH₂CN) | C(=O)Me | Cl | CH₂OMe | O |
| C-17246 | Me | single bond | H | *c*-Pr(1-CH₂CN) | C(=O)Me | Cl | CH₂OMe | O |
| C-17247 | Me(R) | single bond | H | *c*-Pr(1-CH₂CN) | C(=O)Me | Cl | CH₂OMe | O |
| C-17248 | Me(S) | single bond | H | *c*-Pr(1-CH₂CN) | C(=O)Me | Cl | CH₂OMe | O |
| C-17249 | H | single bond | Me | *c*-Pr(1-CH₂CN) | C(=O)Me | Me | CH₂OMe | O |
| C-17250 | Me | single bond | H | *c*-Pr(1-CH₂CN) | C(=O)Me | Me | CH₂OMe | O |
| C-17251 | Me(R) | single bond | H | *c*-Pr(1-CH₂CN) | C(=O)Me | Me | CH₂OMe | O |
| C-17252 | Me(S) | single bond | H | *c*-Pr(1-CH₂CN) | C(=O)Me | Me | CH₂OMe | O |
| C-17253 | H | single bond | Me | *c*-Pr(1-CH₂SMe) | C(=O)Me | Cl | CH₂OMe | O |
| C-17254 | Me | single bond | H | *c*-Pr(1-CH₂SMe) | C(=O)Me | Cl | CH₂OMe | O |
| C-17255 | Me(R) | single bond | H | *c*-Pr(1-CH₂SMe) | C(=O)Me | Cl | CH₂OMe | O |
| C-17256 | Me(S) | single bond | H | *c*-Pr(1-CH₂SMe) | C(=O)Me | Cl | CH₂OMe | O |
| C-17257 | H | single bond | Me | *c*-Pr(1-CH₂SMe) | C(=O)Me | Me | CH₂OMe | O |
| C-17258 | Me | single bond | H | *c*-Pr(1-CH₂SMe) | C(=O)Me | Me | CH₂OMe | O |
| C-17259 | Me(R) | single bond | H | *c*-Pr(1-CH₂SMe) | C(=O)Me | Me | CH₂OMe | O |
| C-17260 | Me(S) | single bond | H | *c*-Pr(1-CH₂SMe) | C(=O)Me | Me | CH₂OMe | O |
| C-17261 | H | single bond | Me | *c*-Pr[1-CH₂S(=O)Me] | C(=O)Me | Cl | CH₂OMe | O |
| C-17262 | Me | single bond | H | c-Pr[1-CH₂S(=O)Me] | C(=O)Me | Cl | CH₂OMe | O |
| C-17263 | Me(R) | single bond | H | *c*-Pr[1-CH₂S(=O)Me] | C(=O)Me | Cl | CH₂OMe | O |
| C-17264 | Me(S) | single bond | H | *c*-Pr[1-CH₂S(=O)Me] | C(=O)Me | Cl | CH₂OMe | O |
| C-17265 | H | single bond | Me | *c*-Pr[1-CH₂S(=O)Me] | C(=O)Me | Me | CH₂OMe | O |
| C-17266 | Me | single bond | H | *c*-Pr[1-CH₂S(=O)Me] | C(=O)Me | Me | CH₂OMe | O |

**[Table 621]**

| Compound Number | Y | W | Z | R¹ | R² | R³ | R¹⁰ | A |
|---|---|---|---|---|---|---|---|---|
| C-17267 | Me(R) | single bond | H | *c*-Pr[1-CH₂S(=O)Me] | C(=O)Me | Me | CH₂OMe | O |
| C-17268 | Me(S) | single bond | H | *c*-Pr[1-CH₂S(=O)Me] | C(=O)Me | Me | CH₂OMe | O |
| C-17269 | H | single bond | Me | *c*-Pr[1-CH₂S(=O)₂Me] | C(=O)Me | Cl | CH₂OMe | O |
| C-17270 | Me | single bond | H | *c*-Pr[1-CH₂S(=O)₂Me] | C(=O)Me | Cl | CH₂OMe | O |
| C-17271 | Me(R) | single bond | H | *c*-Pr[1-CH₂S(=O)₂Me] | C(=O)Me | Cl | CH₂OMe | O |
| C-17272 | Me(S) | single bond | H | *c*-Pr[1-CH₂S(=O)₂Me] | C(=O)Me | Cl | CH₂OMe | O |
| C-17273 | H | single bond | Me | *c*-Pr[1-CH₂S(=O)₂Me] | C(=O)Me | Me | CH₂OMe | O |
| C-17274 | Me | single bond | H | *c*-Pr[1-CH₂S(=O)₂Me] | C(=O)Me | Me | CH₂OMe | O |
| C-17275 | Me(R) | single bond | H | *c*-Pr[1-CH₂S(=O)₂Me] | C(=O)Me | Me | CH₂OMe | O |
| C-17276 | Me(S) | single bond | H | *c*-Pr[1-CH₂S(=O)₂Me] | C(=O)Me | Me | CH₂OMe | O |
| C-17277 | H | single bond | Me | *c*-Pr(1-CH₂F) | C(=O)Me | Cl | CH₂OMe | O |
| C-17278 | Me | single bond | H | *c*-Pr(1-CH₂F) | C(=O)Me | Cl | CH₂OMe | O |
| C-17279 | Me(R) | single bond | H | *c*-Pr(1-CH₂F) | C(=O)Me | Cl | CH₂OMe | O |
| C-17280 | Me(S) | single bond | H | *c*-Pr(1-CH₂F) | C(=O)Me | Cl | CH₂OMe | O |
| C-17281 | H | single bond | Me | *c*-Pr(1-CH₂F) | C(=O)Me | Me | CH₂OMe | O |
| C-17282 | Me | single bond | H | *c*-Pr(1-CH₂F) | C(=O)Me | Me | CH₂OMe | O |
| C-17283 | Me(R) | single bond | H | *c*-Pr(1-CH₂F) | C(=O)Me | Me | CH₂OMe | O |
| C-17284 | Me(S) | single bond | H | *c*-Pr(1-CH₂F) | C(=O)Me | Me | CH₂OMe | O |
| C-17285 | H | single bond | Me | *c*-Pr(1-CHF₂) | C(=O)Me | Cl | CH₂OMe | O |
| C-17286 | Me | single bond | H | *c*-Pr(1-CHF₂) | C(=O)Me | Cl | CH₂OMe | O |
| C-17287 | Me(R) | single bond | H | *c*-Pr(1-CHF₂) | C(=O)Me | Cl | CH₂OMe | O |
| C-17288 | Me(S) | single bond | H | *c*-Pr(1-CHF₂) | C(=O)Me | Cl | CH₂OMe | O |
| C-17289 | H | single bond | Me | *c*-Pr(1-CHF₂) | C(=O)Me | Me | CH₂OMe | O |
| C-17290 | Me | single bond | H | *c*-Pr(1-CHF₂) | C(=O)Me | Me | CH₂OMe | O |
| C-17291 | Me(R) | single bond | H | *c*-Pr(1-CHF₂) | C(=O)Me | Me | CH₂OMe | O |
| C-17292 | Me(S) | single bond | H | *c*-Pr(1-CHF₂) | C(=O)Me | Me | CH₂OMe | O |
| C-17293 | H | single bond | Me | *c*-Pr(1-CF₃) | C(=O)Me | Cl | CH₂OMe | O |
| C-17294 | Me | single bond | H | *c*-Pr(1-CF₃) | C(=O)Me | Cl | CH₂OMe | O |
| C-17295 | Me(R) | single bond | H | *c*-Pr(1-CF₃) | C(=O)Me | Cl | CH₂OMe | O |
| C-17296 | Me(S) | single bond | H | *c*-Pr(1-CF₃) | C(=O)Me | Cl | CH₂OMe | O |
| C-17297 | H | single bond | Me | *c*-Pr(1-CF₃) | C(=O)Me | Me | CH₂OMe | O |
| C-17298 | Me | single bond | H | *c*-Pr(1-CF₃) | C(=O)Me | Me | CH₂OMe | O |
| C-17299 | Me(R) | single bond | H | *c*-Pr(1-CF₃) | C(=O)Me | Me | CH₂OMe | O |
| C-17300 | Me(S) | single bond | H | *c*-Pr(1-CF₃) | C(=O)Me | Me | CH₂OMe | O |
| C-17301 | H | single bond | Me | *c*-Pr(1-C≡CH) | C(=O)Me | Cl | CH₂OMe | O |
| C-17302 | Me | single bond | H | *c*-Pr(1-C≡CH) | C(=O)Me | Cl | CH₂OMe | O |
| C-17303 | Me(R) | single bond | H | *c*-Pr(1-C≡CH) | C(=O)Me | Cl | CH₂OMe | O |
| C-17304 | Me(S) | single bond | H | *c*-Pr(1-C≡CH) | C(=O)Me | Cl | CH₂OMe | O |
| C-17305 | H | single bond | Me | *c*-Pr(1-C≡CH) | C(=O)Me | Me | CH₂OMe | O |
| C-17306 | Me | single bond | H | *c*-Pr(1-C≡CH) | C(=O)Me | Me | CH₂OMe | O |
| C-17307 | Me(R) | single bond | H | *c*-Pr(1-C≡CH) | C(=O)Me | Me | CH₂OMe | O |
| C-17308 | Me(S) | single bond | H | *c*-Pr(1-C≡CH) | C(=O)Me | Me | CH₂OMe | O |
| C-17309 | H | single bond | Me | *c*-pr[1-C(=O)OEt] | C(=O)Me | Cl | CH₂OMe | O |
| C-17310 | Me | single bond | H | *c*-pr[1-C(=O)OEt] | C(=O)Me | Cl | CH₂OMe | O |

**[Table 622]**

| Compound Number | Y | W | Z | R¹ | R² | R³ | R¹⁰ | A |
|---|---|---|---|---|---|---|---|---|
| C-17311 | Me(R) | single bond | H | *c*-pr[1-C(=O)OEt] | C(=O)Me | Cl | CH₂OMe | O |
| C-17312 | Me(S) | single bond | H | *c*-pr[1-C(=O)OEt] | C(=O)Me | Cl | CH₂OMe | O |
| C-17313 | H | single bond | Me | *c*-pr[1-C(=O)OEt] | C(=O)Me | Me | CH₂OMe | O |
| C-17314 | Me | single bond | H | *c*-pr[1-C(=O)OEt] | C(=O)Me | Me | CH₂OMe | O |
| C-17315 | Me(R) | single bond | H | *c*-pr[1-C(=O)OEt] | C(=O)Me | Me | CH₂OMe | O |
| C-17316 | Me(S) | single bond | H | *c*-pr[1-C(=O)OEt] | C(=O)Me | Me | CH₂OMe | O |
| C-17317 | H | single bond | Me | *c*-Pr[1-C(=O)OH] | C(=O)Me | Cl | CH₂OMe | O |
| C-17318 | Me | single bond | H | *c*-Pr[1-C(=O)OH] | C(=O)Me | Cl | CH₂OMe | O |
| C-17319 | Me(R) | single bond | H | *c*-Pr[1-C(=O)OH] | C(=O)Me | Cl | CH₂OMe | O |
| C-17320 | Me(S) | single bond | H | *c*-Pr[1-C(=O)OH] | C(=O)Me | Cl | CH₂OMe | O |
| C-17321 | H | single bond | Me | *c* -Pr[1-C(=0)OH] | C(=O)Me | Me | CH₂OMe | O |
| C-17322 | Me | single bond | H | *c*-Pr[1-C(=O)OH] | C(=O)Me | Me | CH₂OMe | O |
| C-17323 | Me(R) | single bond | H | *c*-Pr[1-C(=O)OH] | C(=O)Me | Me | CH₂OMe | O |
| C-17324 | Me(S) | single bond | H | *c*-Pr[1-C(=O)OH] | C(=O)Me | Me | CH₂OMe | O |
| C-17325 | H | single bond | Me | *c*-Pr[1-C(=O)NH₂] | C(=O)Me | Cl | CH₂OMe | O |
| C-17326 | Me | single bond | H | *c*-Pr[1-C(=O)NH₂] | C(=O)Me | Cl | CH₂OMe | O |
| C-17327 | Me(R) | single bond | H | *c*-Pr[1-C(=O)NH₂] | C(=O)Me | Cl | CH₂OMe | O |
| C-17328 | Me(S) | single bond | H | *c*-Pr[1-C(=O)NH₂] | C(=O)Me | Cl | CH₂OMe | O |
| C-17329 | H | single bond | Me | *c*-Pr[1-C(=O)NH₂] | C(=O)Me | Me | CH₂OMe | O |
| C-17330 | Me | single bond | H | *c*-Pr[1-C(=O)NH₂] | C(=O)Me | Me | CH₂OMe | O |
| C-17331 | Me(R) | single bond | H | *c*-Pr[1-C(=O)NH₂] | C(=O)Me | Me | CH₂OMe | O |
| C-17332 | Me(S) | single bond | H | *c*-Pr[1-C(=O)NH₂] | C(=O)Me | Me | CH₂OMe | O |
| C-17333 | H | single bond | Me | *c*-Pr(1-OMe) | C(=O)Me | Cl | CH₂OMe | O |
| C-17334 | Me | single bond | H | *c*-Pr(1-OMe) | C(=O)Me | Cl | CH₂OMe | O |
| C-17335 | Me(R) | single bond | H | *c*-Pr(1-OMe) | C(=O)Me | Cl | CH₂OMe | O |
| C-17336 | Me(S) | single bond | H | *c*-Pr(1-OMe) | C(=O)Me | Cl | CH₂OMe | O |
| C-17337 | H | single bond | Me | *c*-Pr(1-OMe) | C(=O)Me | Me | CH₂OMe | O |
| C-17338 | Me | single bond | H | *c*-Pr(1-OMe) | C(=O)Me | Me | CH₂OMe | O |
| C-17339 | Me(R) | single bond | H | *c*-Pr(1-OMe) | C(=O)Me | Me | CH₂OMe | O |
| C-17340 | Me(S) | single bond | H | *c*-Pr(1-OMe) | C(=O)Me | Me | CH₂OMe | O |
| C-17341 | H | single bond | Me | *c*-Pr(1-SMe) | C(=O)Me | Cl | CH₂OMe | O |
| C-17342 | Me | single bond | H | *c*-Pr(1-SMe) | C(=O)Me | Cl | CH₂OMe | O |
| C-17343 | Me(R) | single bond | H | *c*-Pr(1-SMe) | C(=O)Me | Cl | CH₂OMe | O |
| C-17344 | Me(S) | single bond | H | *c*-Pr(1-SMe) | C(=O)Me | Cl | CH₂OMe | O |
| C-17345 | H | single bond | Me | *c*-Pr(1-SMe) | C(=O)Me | Me | CH₂OMe | O |
| C-17346 | Me | single bond | H | *c*-Pr(1-SMe) | C(=O)Me | Me | CH₂OMe | O |
| C-17347 | Me(R) | single bond | H | *c*-Pr(1-SMe) | C(=O)Me | Me | CH₂OMe | O |
| C-17348 | Me(S) | single bond | H | *c*-Pr(1-SMe) | C(=O)Me | Me | CH₂OMe | O |
| C-17349 | H | single bond | Me | *c*-Pr[1-S(=O)Me] | C(=O)Me | Cl | CH₂OMe | O |
| C-17350 | Me | single bond | H | *c*-Pr[1-S(=O)Me] | C(=O)Me | Cl | CH₂OMe | O |
| C-17351 | Me(R) | single bond | H | *c*-Pr[1-S(=O)Me] | C(=O)Me | Cl | CH₂OMe | O |
| C-17352 | Me(S) | single bond | H | *c*-Pr[1-S(=O)Me] | C(=O)Me | Cl | CH₂OMe | O |
| C-17353 | H | single bond | Me | *c*-Pr[1-S(=O)Me] | C(=O)Me | Me | CH₂OMe | O |
| C-17354 | Me | single bond | H | *c*-Pr[1-S(=O)Me] | C(=O)Me | Me | CH₂OMe | O |

**[Table 623]**

| Compound Number | Y | W | Z | R¹ | R² | R³ | R¹⁰ | A |
|---|---|---|---|---|---|---|---|---|
| C-17355 | Me(R) | single bond | H | *c*-Pr[1-S(=O)Me] | C(=O)Me | Me | CH₂OMe | O |
| C-17356 | Me(S) | single bond | H | *c*-Pr[1-S(=O)Me] | C(=O)Me | Me | CH₂OMe | O |
| C-17357 | H | single bond | Me | *c*-Pr[1-S(=O)₂Me] | C(=O)Me | Cl | CH₂OMe | O |
| C-17358 | Me | single bond | H | *c*-Pr[1-S(=O)₂Me] | C(=O)Me | Cl | CH₂OMe | O |
| C-17359 | Me(R) | single bond | H | *c*-Pr[1-S(=O)₂Me] | C(=O)Me | Cl | CH₂OMe | O |
| C-17360 | Me(S) | single bond | H | *c*-Pr[1-S(=O)₂Me] | C(=O)Me | Cl | CH₂OMe | O |
| C-17361 | H | single bond | Me | *c*-Pr[1-S(=O)₂Me] | C(=O)Me | Me | CH₂OMe | O |
| C-17362 | Me | single bond | H | *c*-Pr[1-S(=O)₂Me] | C(=O)Me | Me | CH₂OMe | O |
| C-17363 | Me(R) | single bond | H | *c*-Pr[1-S(=O)₂Me] | C(=O)Me | Me | CH₂OMe | O |
| C-17364 | Me(S) | single bond | H | *c*-Pr[1-S(=O)₂Me] | C(=O)Me | Me | CH₂OMe | O |
| C-17365 | H | single bond | Me | *c*-Pr[1-S(=O)₂CHF₂] | C(=O)Me | Cl | CH₂OMe | O |
| C-17366 | Me | single bond | H | *c*-Pr[1-S(=O)₂CHF₂] | C(=O)Me | Cl | CH₂OMe | O |
| C-17367 | Me(R) | single bond | H | *c*-Pr[1-S(=O)₂CHF₂] | C(=O)Me | Cl | CH₂OMe | O |
| C-17368 | Me(S) | single bond | H | *c*-Pr[1-S(=O)₂CHF₂] | C(=O)Me | Cl | CH₂OMe | O |
| C-17369 | H | single bond | Me | *c*-Pr[1-S(=O)₂CHF₂] | C(=O)Me | Me | CH₂OMe | O |
| C-17370 | Me | single bond | H | *c*-Pr[1-S(=O)₂CHF₂] | C(=O)Me | Me | CH₂OMe | O |
| C-17371 | Me(R) | single bond | H | *c*-Pr[1-S(=O)₂CHF₂] | C(=O)Me | Me | CH₂OMe | O |
| C-17372 | Me(S) | single bond | H | *c*-Pr[1-S(=O)₂CHF₂] | C(=O)Me | Me | CH₂OMe | O |
| C-17373 | H | single bond | Me | *c*-Pr[1-S(=O)₂CF₃] | C(=O)Me | Cl | CH₂OMe | O |
| C-17374 | Me | single bond | H | *c*-Pr[1-S(=O)₂CF₃] | C(=O)Me | Cl | CH₂OMe | O |
| C-17375 | Me(R) | single bond | H | *c*-Pr[1-S(=O)₂CF₃] | C(=O)Me | Cl | CH₂OMe | O |
| C-17376 | Me(S) | single bond | H | *c*-Pr[1-S(=O)₂CF₃] | C(=O)Me | Cl | CH₂OMe | O |
| C-17377 | H | single bond | Me | *c*-Pr[1-S(=O)₂CF₃] | C(=O)Me | Me | CH₂OMe | O |
| C-17378 | Me | single bond | H | *c*-Pr[1-S(=O)₂CF₃] | C(=O)Me | Me | CH₂OMe | O |
| C-17379 | Me(R) | single bond | H | *c*-Pr[1-S(=O)₂CF₃] | C(=O)Me | Me | CH₂OMe | O |
| C-17380 | Me(S) | single bond | H | *c*-Pr[1-S(=O)₂CF₃] | C(=O)Me | Me | CH₂OMe | O |
| C-17381 | H | single bond | Me | *c*-Pr[1-(5,6-dihydro-1,4,2-dioxazin-3-yl)] | C(=O)Me | Cl | CH₂OMe | O |
| C-17382 | Me | single bond | H | *c*-Pr[1-(5,6-dihydro-1,4,2-dioxazin-3-yl)] | C(=O)Me | Cl | CH₂OMe | O |
| C-17383 | Me(R) | single bond | H | *c*-Pr[1-(5,6-dihydro-1,4,2-dioxazin-3-yl)] | C(=O)Me | Cl | CH₂OMe | O |
| C-17384 | Me(S) | single bond | H | *c*-Pr[1-(5,6-dihydro-1,4,2-dioxazin-3-yl)] | C(=O)Me | Cl | CH₂OMe | O |
| C-17385 | H | single bond | Me | *c*-Pr[1-(5,6-dihydro-1,4,2-dioxazin-3-yl)] | C(=O)Me | Me | CH₂OMe | O |
| C-17386 | Me | single bond | H | *c*-Pr[1-(5,6-dihydro-1,4,2-dioxazin-3-yl)] | C(=O)Me | Me | CH₂OMe | O |
| C-17387 | Me(R) | single bond | H | *c*-Pr[1-(5,6-dihydro-1,4,2-dioxazin-3-yl)] | C(=O)Me | Me | CH₂OMe | O |
| C-17388 | Me(S) | single bond | H | *c*-Pr[1-(5,6-dihydro-1,4,2-dioxazin-3-yl)] | C(=O)Me | Me | CH₂OMe | O |
| C-17389 | H | single bond | Me | *c*-Pr[1-(5-hydroxy-1,3,4-oxadiazol-2-yl)] | C(=O)Me | Cl | CH₂OMe | O |
| C-17390 | Me | single bond | H | *c*-Pr[1-(5-hydroxy-1,3,4-oxadiazol-2-yl)] | C(=O)Me | Cl | CH₂OMe | O |
| C-17391 | Me(R) | single bond | H | *c*-Pr[1-(5-hydroxy-1,3,4-oxadiazol-2-yl)] | C(=O)Me | Cl | CH₂OMe | O |
| C-17392 | Me(S) | single bond | H | *c*-Pr[1-(5-hydroxy-1,3,4-oxadiazol-2-yl)] | C(=O)Me | Cl | CH₂OMe | O |
| C-17393 | H | single bond | Me | *c*-Pr[1-(5-hydroxy-1,3,4-oxadiazol-2-yl)] | C(=O)Me | Me | CH₂OMe | O |
| C-17394 | Me | single bond | H | *c*-Pr[1-(5-hydroxy-1,3,4-oxadiazol-2-yl)] | C(=O)Me | Me | CH₂OMe | O |
| C-17395 | Me(R) | single bond | H | *c*-Pr[1-(5-hydroxy-1,3,4-oxadiazol-2-yl)] | C(=O)Me | Me | CH₂OMe | O |
| C-17396 | Me(S) | single bond | H | *c*-Pr[1-(5-hydroxy-1,3,4-oxadiazol-2-yl)] | C(=O)Me | Me | CH₂OMe | O |
| C-17397 | H | single bond | Me | *c* -Pr[1-(pyrimidin-2-yl)] | C(=O)Me | Cl | CH₂OMe | O |
| C-17398 | Me | single bond | H | *c*-Pr[1-(pyrimidin-2-yl)] | C(=O)Me | Cl | CH₂OMe | O |

**[Table 624]**

| Compound Number | Y | W | Z | R¹ | R² | R³ | R¹⁰ | A |
|---|---|---|---|---|---|---|---|---|
| C-17399 | Me(R) | single bond | H | *c*-Pr[1-(pyrimidin-2-yl)] | C(=O)Me | Cl | CH₂OMe | O |
| C-17400 | Me(S) | single bond | H | *c*-Pr[1-(pyrimidin-2-yl)] | C(=O)Me | Cl | CH₂OMe | O |
| C-17401 | H | single bond | Me | *c*-Pr[1-(pyrimidin-2-yl)] | C(=O)Me | Me | CH₂OMe | O |
| C-17402 | Me | single bond | H | *c*-Pr[1-(pyrimidin-2-yl)] | C(=O)Me | Me | CH₂OMe | O |
| C-17403 | Me(R) | single bond | H | *c*-Pr[1-(pyrimidin-2-yl)] | C(=O)Me | Me | CH₂OMe | O |
| C-17404 | Me(S) | single bond | H | *c*-Pr[1-(pyrimidin-2-yl)] | C(=O)Me | Me | CH₂OMe | O |
| C-17405 | H | single bond | Me | *t-*Bu | C(=O)Me | Cl | CH₂OMe | O |
| C-17406 | Me | single bond | H | *t*-Bu | C(=O)Me | Cl | CH₂OMe | O |
| C-17407 | Me(R) | single bond | H | *t*-Bu | C(=O)Me | Cl | CH₂OMe | O |
| C-17408 | Me(S) | single bond | H | *t*-Bu | C(=O)Me | Cl | CH₂OMe | O |
| C-17409 | H | single bond | Me | *t*-Bu | C(=O)Me | Me | CH₂OMe | O |
| C-17410 | Me | single bond | H | *t*-Bu | C(=O)Me | Me | CH₂OMe | O |
| C-17411 | Me(R) | single bond | H | *t*-Bu | C(=O)Me | Me | CH₂OMe | O |
| C-17412 | Me(S) | single bond | H | *t*-Bu | C(=O)Me | Me | CH₂OMe | O |
| C-17413 | H | single bond | Me | *c*-Bu | C(=O)Me | Cl | CH₂OMe | O |
| C-17414 | Me | single bond | H | *c*-Bu | C(=O)Me | Cl | CH₂OMe | O |
| C-17415 | Me(R) | single bond | H | *c*-Bu | C(=O)Me | Cl | CH₂OMe | O |
| C-17416 | Me(S) | single bond | H | *c*-Bu | C(=O)Me | Cl | CH₂OMe | O |
| C-17417 | H | single bond | Me | *c*-Bu | C(=O)Me | Me | CH₂OMe | O |
| C-17418 | Me | single bond | H | *c*-Bu | C(=O)Me | Me | CH₂OMe | O |
| C-17419 | Me(R) | single bond | H | c-Bu | C(=O)Me | Me | CH₂OMe | O |
| C-17420 | Me(S) | single bond | H | *c*-Bu | C(=O)Me | Me | CH₂OMe | O |
| C-17421 | H | single bond | Me | *c*-Bu(1-CN) | C(=O)Me | Cl | CH₂OMe | O |
| C-17422 | Me | single bond | H | *c*-Bu(1-CN) | C(=O)Me | Cl | CH₂OMe | O |
| C-17423 | Me(R) | single bond | H | *c*-Bu(1-CN) | C(=O)Me | Cl | CH₂OMe | O |
| C-17424 | Me(S) | single bond | H | *c*-Bu(1-CN) | C(=O)Me | Cl | CH₂OMe | O |
| C-17425 | H | single bond | Me | *c*-Bu(1-CN) | C(=O)Me | Me | CH₂OMe | O |
| C-17426 | Me | single bond | H | *c*-Bu(1-CN) | C(=O)Me | Me | CH₂OMe | O |
| C-17427 | Me(R) | single bond | H | *c*-Bu(1-CN) | C(=O)Me | Me | CH₂OMe | O |
| C-17428 | Me(S) | single bond | H | *c*-Bu(1-CN) | C(=O)Me | Me | CH₂OMe | O |
| C-17429 | H | single bond | Me | *c*-Bu(3,3-F₂) | C(=O)Me | Cl | CH₂OMe | O |
| C-17430 | Me | single bond | H | *c*-Bu(3,3-F₂) | C(=O)Me | Cl | CH₂OMe | O |
| C-17431 | Me(R) | single bond | H | *c*-Bu(3,3-F₂) | C(=O)Me | Cl | CH₂OMe | O |
| C-17432 | Me(S) | single bond | H | *c*-Bu(3,3-F₂) | C(=O)Me | Cl | CH₂OMe | O |
| C-17433 | H | single bond | Me | *c*-Bu(3,3-F₂) | C(=O)Me | Me | CH₂OMe | O |
| C-17434 | Me | single bond | H | *c*-Bu(3,3-F₂) | C(=O)Me | Me | CH₂OMe | O |
| C-17435 | Me(R) | single bond | H | *c*-Bu(3,3-F₂) | C(=O)Me | Me | CH₂OMe | O |
| C-17436 | Me(S) | single bond | H | *c*-Bu(3,3-F₂) | C(=O)Me | Me | CH₂OMe | O |
| C-17437 | H | single bond | Me | oxetan-3-yl | C(=O)Me | Cl | CH₂OMe | O |
| C-17438 | Me | single bond | H | oxetan-3-yl | C(=O)Me | Cl | CH₂OMe | O |
| C-17439 | Me(R) | single bond | H | oxetan-3-yl | C(=O)Me | Cl | CH₂OMe | O |
| C-17440 | Me(S) | single bond | H | oxetan-3-yl | C(=O)Me | Cl | CH₂OMe | O |
| C-17441 | H | single bond | Me | oxetan-3-yl | C(=O)Me | Me | CH₂OMe | O |
| C-17442 | Me | single bond | H | oxetan-3-yl | C(=O)Me | Me | CH₂OMe | O |

**[Table 625]**

| Compound Number | Y | W | Z | R¹ | R² | R³ | R¹⁰ | A |
|---|---|---|---|---|---|---|---|---|
| C-17443 | Me(R) | single bond | H | oxetan-3-yl | C(=O)Me | Me | CH₂OMe | O |
| C-17444 | Me(S) | single bond | H | oxetan-3-yl | C(=O)Me | Me | CH₂OMe | O |
| C-17445 | H | single bond | Me | oxetan(3-CN)-3-yl | C(=O)Me | Cl | CH₂OMe | O |
| C-17446 | Me | single bond | H | oxetan(3-CN)-3-yl | C(=O)Me | Cl | CH₂OMe | O |
| C-17447 | Me(R) | single bond | H | oxetan(3-CN)-3-yl | C(=O)Me | Cl | CH₂OMe | O |
| C-17448 | Me(S) | single bond | H | oxetan(3-CN)-3-yl | C(=O)Me | Cl | CH₂OMe | O |
| C-17449 | H | single bond | Me | oxetan(3-CN)-3-yl | C(=O)Me | Me | CH₂OMe | O |
| C-17450 | Me | single bond | H | oxetan(3-CN)-3-yl | C(=O)Me | Me | CH₂OMe | O |
| C-17451 | Me(R) | single bond | H | oxetan(3-CN)-3-yl | C(=O)Me | Me | CH₂OMe | O |
| C-17452 | Me(S) | single bond | H | oxetan(3-CN)-3-yl | C(=O)Me | Me | CH₂OMe | O |
| C-17453 | H | single bond | Me | azetidin[1-C(=O)Me]-3-yl | C(=O)Me | Cl | CH₂OMe | O |
| C-17454 | Me | single bond | H | azetidin[1-C(=O)Me]-3-yl | C(=O)Me | Cl | CH₂OMe | O |
| C-17455 | Me(R) | single bond | H | azetidin[1-C(=O)Me]-3-yl | C(=O)Me | Cl | CH₂OMe | O |
| C-17456 | Me(S) | single bond | H | azetidin[1-C(=O)Me]-3-yl | C(=O)Me | Cl | CH₂OMe | O |
| C-17457 | H | single bond | Me | azetidin[1-C(=O)Me]-3-yl | C(=O)Me | Me | CH₂OMe | O |
| C-17458 | Me | single bond | H | azetidin[1-C(=O)Me]-3-yl | C(=O)Me | Me | CH₂OMe | O |
| C-17459 | Me(R) | single bond | H | azetidin[1-C(=O)Me]-3-yl | C(=O)Me | Me | CH₂OMe | O |
| C-17460 | Me(S) | single bond | H | azetidin[1-C(=O)Me]-3-yl | C(=O)Me | Me | CH₂OMe | O |
| C-17461 | H | single bond | Me | azetidin[1-S(=O)₂Me]-3-yl | C(=O)Me | Cl | CH₂OMe | O |
| C-17462 | Me | single bond | H | azetidin[1-S(=O)₂Me]-3-yl | C(=O)Me | Cl | CH₂OMe | O |
| C-17463 | Me(R) | single bond | H | azetidin[1-S(=O)₂Me]-3-yl | C(=O)Me | Cl | CH₂OMe | O |
| C-17464 | Me(S) | single bond | H | azetidin[1-S(=O)₂Me]-3-yl | C(=O)Me | Cl | CH₂OMe | O |
| C-17465 | H | single bond | Me | azetidin[1-S(=O)₂Me]-3-yl | C(=O)Me | Me | CH₂OMe | O |
| C-17466 | Me | single bond | H | azetidin[1-S(=O)₂Me]-3-yl | C(=O)Me | Me | CH₂OMe | O |
| C-17467 | Me(R) | single bond | H | azetidin[1-S(=O)₂Me]-3-yl | C(=O)Me | Me | CH₂OMe | O |
| C-17468 | Me(S) | single bond | H | azetidin[1-S(=O)₂Me]-3-yl | C(=O)Me | Me | CH₂OMe | O |
| C-17469 | H | single bond | Me | azetidin[1-S(=O)₂CHF₂]-3-yl | C(=O)Me | Cl | CH₂OMe | O |
| C-17470 | Me | single bond | H | azetidin[1-S(=O)₂CHF₂]-3-yl | C(=O)Me | Cl | CH₂OMe | O |
| C-17471 | Me(R) | single bond | H | azetidin[1-S(=O)₂CHF₂]-3-yl | C(=O)Me | Cl | CH₂OMe | O |
| C-17472 | Me(S) | single bond | H | azetidin[1-S(=O)₂CHF₂]-3-yl | C(=O)Me | Cl | CH₂OMe | O |
| C-17473 | H | single bond | Me | azetidin[1-S(=O)₂CHF₂]-3-yl | C(=O)Me | Me | CH₂OMe | O |
| C-17474 | Me | single bond | H | azetidin[1-S(=O)₂CHF₂]-3-yl | C(=O)Me | Me | CH₂OMe | O |
| C-17475 | Me(R) | single bond | H | azetidin[1-S(=O)₂CHF₂]-3-yl | C(=O)Me | Me | CH₂OMe | O |
| C-17476 | Me(S) | single bond | H | azetidin[1-S(=O)₂CHF₂]-3-yl | C(=O)Me | Me | CH₂OMe | O |
| C-17477 | H | single bond | Me | *c*-Pen | C(=O)Me | Cl | CH₂OMe | O |
| C-17478 | Me | single bond | H | *c*-Pen | C(=O)Me | Cl | CH₂OMe | O |
| C-17479 | Me(R) | single bond | H | *c*-Pen | C(=O)Me | Cl | CH₂OMe | O |
| C-17480 | Me(S) | single bond | H | *c*-Pen | C(=O)Me | Cl | CH₂OMe | O |
| C-17481 | H | single bond | Me | *c*-Pen | C(=O)Me | Me | CH₂OMe | O |
| C-17482 | Me | single bond | H | *c*-Pen | C(=O)Me | Me | CH₂OMe | O |
| C-17483 | Me(R) | single bond | H | *c*-Pen | C(=O)Me | Me | CH₂OMe | O |
| C-17484 | Me(S) | single bond | H | *c*-Pen | C(=O)Me | Me | CH₂OMe | O |
| C-17485 | H | single bond | Me | tetrahydrofuran-3-yl | C(=O)Me | Cl | CH₂OMe | O |
| C-17486 | Me | single bond | H | tetrahydrofuran-3-yl | C(=O)Me | Cl | CH₂OMe | O |

**[Table 626]**

| Compound Number | Y | W | Z | R¹ | R² | R³ | R¹⁰ | A |
|---|---|---|---|---|---|---|---|---|
| C-17487 | Me(R) | single bond | H | tetrahydrofuran-3-yl | C(=O)Me | Cl | CH₂OMe | O |
| C-17488 | Me(S) | single bond | H | tetrahydrofuran-3-yl | C(=O)Me | Cl | CH₂OMe | O |
| C-17489 | H | single bond | Me | tetrahydrofuran-3-yl | C(=O)Me | Me | CH₂OMe | O |
| C-17490 | Me | single bond | H | tetrahydrofuran-3-yl | C(=O)Me | Me | CH₂OMe | O |
| C-17491 | Me(R) | single bond | H | tetra hydrofuran-3-yl | C(=O)Me | Me | CH₂OMe | O |
| C-17492 | Me(S) | single bond | H | tetrahydrofuran-3-yl | C(=O)Me | Me | CH₂OMe | O |
| C-17493 | H | single bond | Me | tetrahydrothiophen-3-yl | C(=O)Me | Cl | CH₂OMe | O |
| C-17494 | Me | single bond | H | tetrahydrothiophen-3-yl | C(=O)Me | Cl | CH₂OMe | O |
| C-17495 | Me(R) | single bond | H | tetrahydrothiophen-3-yl | C(=O)Me | Cl | CH₂OMe | O |
| C-17496 | Me(S) | single bond | H | tetrahydrothiophen-3-yl | C(=O)Me | Cl | CH₂OMe | O |
| C-17497 | H | single bond | Me | tetrahydrothiophen-3-yl | C(=O)Me | Me | CH₂OMe | O |
| C-17498 | Me | single bond | H | tetrahydrothiophen-3-yl | C(=O)Me | Me | CH₂OMe | O |
| C-17499 | Me(R) | single bond | H | tetrahydrothiophen-3-yl | C(=O)Me | Me | CH₂OMe | O |
| C-17500 | Me(S) | single bond | H | tetrahydrothiophen-3-yl | C(=O)Me | Me | CH₂OMe | O |
| C-17501 | H | single bond | Me | *c*-Pen(1-CN) | C(=O)Me | Cl | CH₂OMe | O |
| C-17502 | Me | single bond | H | *c*-Pen(1-CN) | C(=O)Me | Cl | CH₂OMe | O |
| C-17503 | Me(R) | single bond | H | *c*-Pen(1-CN) | C(=O)Me | Cl | CH₂OMe | O |
| C-17504 | Me(S) | single bond | H | *c*-Pen(1-CN) | C(=O)Me | Cl | CH₂OMe | O |
| C-17505 | H | single bond | Me | *c*-Pen(1-CN) | C(=O)Me | Me | CH₂OMe | O |
| C-17506 | Me | single bond | H | *c*-Pen(1-CN) | C(=O)Me | Me | CH₂OMe | O |
| C-17507 | Me(R) | single bond | H | *c*-Pen(1-CN) | C(=O)Me | Me | CH₂OMe | O |
| C-17508 | Me(S) | single bond | H | *c*-Pen(1-CN) | C(=O)Me | Me | CH₂OMe | O |
| C-17509 | H | single bond | Me | tetrahydrofuran(3-CN)-3-yl | C(=O)Me | Cl | CH₂OMe | O |
| C-17510 | Me | single bond | H | tetrahydrofuran(3-CN)-3-yl | C(=O)Me | Cl | CH₂OMe | O |
| C-17511 | Me(R) | single bond | H | tetrahydrofuran(3-CN)-3-yl | C(=O)Me | Cl | CH₂OMe | O |
| C-17512 | Me(S) | single bond | H | tetrahydrofuran(3-CN)-3-yl | C(=O)Me | Cl | CH₂OMe | O |
| C-17513 | H | single bond | Me | tetrahydrofuran(3-CN)-3-yl | C(=O)Me | Me | CH₂OMe | O |
| C-17514 | Me | single bond | H | tetrahydrofuran(3-CN)-3-yl | C(=O)Me | Me | CH₂OMe | O |
| C-17515 | Me(R) | single bond | H | tetrahydrofuran(3-CN)-3-yl | C(=O)Me | Me | CH₂OMe | O |
| C-17516 | Me(S) | single bond | H | tetrahydrofuran(3-CN)-3-yl | C(=O)Me | Me | CH₂OMe | O |
| C-17517 | H | single bond | Me | tetrahydrothiophen(3-CN)-3-yl | C(=O)Me | Cl | CH₂OMe | O |
| C-17518 | Me | single bond | H | tetrahydrothiophen(3-CN)-3-yl | C(=O)Me | Cl | CH₂OMe | O |
| C-17519 | Me(R) | single bond | H | tetrahydrothiophen(3-CN)-3-yl | C(=O)Me | Cl | CH₂OMe | O |
| C-17520 | Me(S) | single bond | H | tetrahydrothiophen(3-CN)-3-yl | C(=O)Me | Cl | CH₂OMe | O |
| C-17521 | H | single bond | Me | tetrahydrothiophen(3-CN)-3-yl | C(=O)Me | Me | CH₂OMe | O |
| C-17522 | Me | single bond | H | tetrahydrothiophen(3-CN)-3-yl | C(=O)Me | Me | CH₂OMe | O |
| C-17523 | Me(R) | single bond | H | tetrahydrothiophen(3-CN)-3-yl | C(=O)Me | Me | CH₂OMe | O |
| C-17524 | Me(S) | single bond | H | tetrahydrothiophen(3-CN)-3-yl | C(=O)Me | Me | CH₂OMe | O |
| C-17525 | H | single bond | Me | bicyclo[1.1.1]pentan-1-yl | C(=O)Me | Cl | CH₂OMe | O |
| C-17526 | Me | single bond | H | bicyclo[1.1.1]pentan-1-yl | C(=O)Me | Cl | CH₂OMe | O |
| C-17527 | Me(R) | single bond | H | bicyclo[1.1.1]pentan-1-yl | C(=O)Me | Cl | CH₂OMe | O |
| C-17528 | Me(S) | single bond | H | bicyclo[1.1.1]pentan-1-yl | C(=O)Me | Cl | CH₂OMe | O |
| C-17529 | H | single bond | Me | bicyclo[1.1.1]pentan-1-yl | C(=O)Me | Me | CH₂OMe | O |
| C-17530 | Me | single bond | H | bicyclo[1.1.1]pentan-1-yl | C(=O)Me | Me | CH₂OMe | O |

**[Table 627]**

| Compound Number | Y | W | Z | R¹ | R² | R³ | R¹⁰ | A |
|---|---|---|---|---|---|---|---|---|
| C-17531 | Me(R) | single bond | H | bicyclo[1.1.1]pentan-1-yl | C(=O)Me | Me | CH₂OMe | O |
| C-17532 | Me(S) | single bond | H | bicyclo[1.1.1]pentan-1-yl | C(=O)Me | Me | CH₂OMe | O |
| C-17533 | H | single bond | Me | *c*-Hex | C(=O)Me | Cl | CH₂OMe | O |
| C-17534 | Me | single bond | H | *c*-Hex | C(=O)Me | Cl | CH₂OMe | O |
| C-17535 | Me(R) | single bond | H | *c*-Hex | C(=O)Me | Cl | CH₂OMe | O |
| C-17536 | Me(S) | single bond | H | *c*-Hex | C(=O)Me | Cl | CH₂OMe | O |
| C-17537 | H | single bond | Me | *c*-Hex | C(=O)Me | Me | CH₂OMe | O |
| C-17538 | Me | single bond | H | *c-h*ex | C(=O)Me | Me | CH₂OMe | O |
| C-17539 | Me(R) | single bond | H | *c*-Hex | C(=O)Me | Me | CH₂OMe | O |
| C-17540 | Me(S) | single bond | H | *c*-Hex | C(=O)Me | Me | CH₂OMe | O |
| C-17541 | H | single bond | Me | tetrahydropyran-4-yl | C(=O)Me | Cl | CH₂OMe | O |
| C-17542 | Me | single bond | H | tetrahydropyran-4-yl | C(=O)Me | Cl | CH₂OMe | O |
| C-17543 | Me(R) | single bond | H | tetrahydropyran-4-yl | C(=O)Me | Cl | CH₂OMe | O |
| C-17544 | Me(S) | single bond | H | tetrahydropyran-4-yl | C(=O)Me | Cl | CH₂OMe | O |
| C-17545 | H | single bond | Me | tetrahydropyran-4-yl | C(=O)Me | Me | CH₂OMe | O |
| C-17546 | Me | single bond | H | tetrahydropyran-4-yl | C(=O)Me | Me | CH₂OMe | O |
| C-17547 | Me(R) | single bond | H | tetrahydropyran-4-yl | C(=O)Me | Me | CH₂OMe | O |
| C-17548 | Me(S) | single bond | H | tetrahydropyran-4-yl | C(=O)Me | Me | CH₂OMe | O |
| C-17549 | H | single bond | Me | tetrahydrothiopyran-4-yl | C(=O)Me | Cl | CH₂OMe | O |
| C-17550 | Me | single bond | H | tetrahydrothiopyran-4-yl | C(=O)Me | Cl | CH₂OMe | O |
| C-17551 | Me(R) | single bond | H | tetrahydrothiopyran-4-yl | C(=O)Me | Cl | CH₂OMe | O |
| C-17552 | Me(S) | single bond | H | tetrahydrothiopyran-4-yl | C(=O)Me | Cl | CH₂OMe | O |
| C-17553 | H | single bond | Me | tetrahydrothiopyran-4-yl | C(=O)Me | Me | CH₂OMe | O |
| C-17554 | Me | single bond | H | tetrahydrothiopyran-4-yl | C(=O)Me | Me | CH₂OMe | O |
| C-17555 | Me(R) | single bond | H | tetrahydrothiopyran-4-yl | C(=O)Me | Me | CH₂OMe | O |
| C-17556 | Me(S) | single bond | H | tetrahydrothiopyran-4-yl | C(=O)Me | Me | CH₂OMe | O |
| C-17557 | H | single bond | Me | *c*-Hex(1-CN) | C(=O)Me | Cl | CH₂OMe | O |
| C-17558 | Me | single bond | H | *c*-Hex(1-CN) | C(=O)Me | Cl | CH₂OMe | O |
| C-17559 | Me(R) | single bond | H | *c*-Hex(1-CN) | C(=O)Me | Cl | CH₂OMe | O |
| C-17560 | Me(S) | single bond | H | *c*-Hex(1-CN) | C(=O)Me | Cl | CH₂OMe | O |
| C-17561 | H | single bond | Me | c*-*Hex(1-CN) | C(=O)Me | Me | CH₂OMe | O |
| C-17562 | Me | single bond | H | *c*-Hex(1-CN) | C(=O)Me | Me | CH₂OMe | O |
| C-17563 | Me(R) | single bond | H | *c*-Hex(1-CN) | C(=O)Me | Me | CH₂OMe | O |
| C-17564 | Me(S) | single bond | H | *c*-Hex(1-CN) | C(=O)Me | Me | CH₂OMe | O |
| C-17565 | H | single bond | Me | tetrahydropyran(4-CN)-4-yl | C(=O)Me | Cl | CH₂OMe | O |
| C-17566 | Me | single bond | H | tetrahydropyran(4-CN)-4-yl | C(=O)Me | Cl | CH₂OMe | O |
| C-17567 | Me(R) | single bond | H | tetrahydropyran(4-CN)-4-yl | C(=O)Me | Cl | CH₂OMe | O |
| C-17568 | Me(S) | single bond | H | tetrahydropyran(4-CN)-4-yl | C(=O)Me | Cl | CH₂OMe | O |
| C-17569 | H | single bond | Me | tetrahydropyran(4-CN)-4-yl | C(=O)Me | Me | CH₂OMe | O |
| C-17570 | Me | single bond | H | tetrahydropyran(4-CN)-4-yl | C(=O)Me | Me | CH₂OMe | O |
| C-17571 | Me(R) | single bond | H | tetrahydropyran(4-CN)-4-yl | C(=O)Me | Me | CH₂OMe | O |
| C-17572 | Me(S) | single bond | H | tetrahydropyran(4-CN)-4-yl | C(=O)Me | Me | CH₂OMe | O |
| C-17573 | H | single bond | Me | tetrahydrothiopyran(4-CN)-4-yl | C(=O)Me | Cl | CH₂OMe | O |
| C-17574 | Me | single bond | H | tetrahydrothiopyran(4-CN)-4-yl | C(=O)Me | Cl | CH₂OMe | O |

**[Table 628]**

| Compound Number | Y | W | Z | R¹ | R² | R³ | R¹⁰ | A |
|---|---|---|---|---|---|---|---|---|
| C-17575 | Me(R) | single bond | H | tetrahydrothiopyran(4-CN)-4-yl | C(=O)Me | Cl | CH₂OMe | O |
| C-17576 | Me(S) | single bond | H | tetrahydrothiopyran(4-CN)-4-yl | C(=O)Me | Cl | CH₂OMe | O |
| C-17577 | H | single bond | Me | tetrahydrothiopyran(4-CN)-4-yl | C(=O)Me | Me | CH₂OMe | O |
| C-17578 | Me | single bond | H | tetrahydrothiopyran(4-CN)-4-yl | C(=O)Me | Me | CH₂OMe | O |
| C-17579 | Me(R) | single bond | H | tetrahydrothiopyran(4-CN)-4-yl | C(=O)Me | Me | CH₂OMe | O |
| C-17580 | Me(S) | single bond | H | tetrahydrothiopyran(4-CN)-4-yl | C(=O)Me | Me | CH₂OMe | O |
| C-17581 | H | single bond | Me | CH₂(*c*-Pr) | C(=O)Me | Cl | CH₂OMe | O |
| C-17582 | Me | single bond | H | CH₂(*c*-Pr) | C(=O)Me | Cl | CH₂OMe | O |
| C-17583 | Me(R) | single bond | H | CH₂(*c*-Pr) | C(=O)Me | Cl | CH₂OMe | O |
| C-17584 | Me(S) | single bond | H | CH₂(*c*-Pr) | C(=O)Me | Cl | CH₂OMe | O |
| C-17585 | H | single bond | Me | CH₂(*c*-Pr) | C(=O)Me | Me | CH₂OMe | O |
| C-17586 | Me | single bond | H | CH₂(*c*-Pr) | C(=O)Me | Me | CH₂OMe | O |
| C-17587 | Me(R) | single bond | H | CH₂(*c*-Pr) | C(=O)Me | Me | CH₂OMe | O |
| C-17588 | Me(S) | single bond | H | CH₂(*c*-Pr) | C(=O)Me | Me | CH₂OMe | O |
| C-17589 | H | single bond | Me | CH₂[*c*-Pr(1-CN)] | C(=O)Me | Cl | CH₂OMe | O |
| C-17590 | Me | single bond | H | CH₂[*c*-Pr(1-CN)] | C(=O)Me | Cl | CH₂OMe | O |
| C-17591 | Me(R) | single bond | H | CH₂[*c*-Pr(1-CN)] | C(=O)Me | Cl | CH₂OMe | O |
| C-17592 | Me(S) | single bond | H | CH₂[*c* -Pr(1-CN)] | C(=O)Me | Cl | CH₂OMe | O |
| C-17593 | H | single bond | Me | CH₂[*c*-Pr(1-CN)] | C(=O)Me | Me | CH₂OMe | O |
| C-17594 | Me | single bond | H | CH₂[*c*-Pr(1-CN)] | C(=O)Me | Me | CH₂OMe | O |
| C-17595 | Me(R) | single bond | H | CH₂[*c*-Pr(1-CN)] | C(=O)Me | Me | CH₂OMe | O |
| C-17596 | Me(S) | single bond | H | CH₂[*c*-Pr(1-CN)] | C(=O)Me | Me | CH₂OMe | O |
| C-17597 | H | single bond | Me | CH(Me)(*c*-Pr) | C(=O)Me | Cl | CH₂OMe | O |
| C-17598 | Me | single bond | H | CH(Me)(*c*-Pr) | C(=O)Me | Cl | CH₂OMe | O |
| C-17599 | Me(R) | single bond | H | CH(Me)(*c*-Pr) | C(=O)Me | Cl | CH₂OMe | O |
| C-17600 | Me(S) | single bond | H | CH(Me)(*c*-Pr) | C(=O)Me | Cl | CH₂OMe | O |
| C-17601 | H | single bond | Me | CH(Me)(*c*-Pr) | C(=O)Me | Me | CH₂OMe | O |
| C-17602 | Me | single bond | H | CH(Me)(*c*-Pr) | C(=O)Me | Me | CH₂OMe | O |
| C-17603 | Me(R) | single bond | H | CH(Me)(*c*-Pr) | C(=O)Me | Me | CH₂OMe | O |
| C-17604 | Me(S) | single bond | H | CH(Me)(*c* -Pr) | C(=O)Me | Me | CH₂OMe | O |
| C-17605 | H | single bond | Me | CH(Me)CH₂OMe | C(=O)Me | Cl | CH₂OMe | O |
| C-17606 | Me | single bond | H | CH(Me)CH₂OMe | C(=O)Me | Cl | CH₂OMe | O |
| C-17607 | Me(R) | single bond | H | CH(Me)CH₂OMe | C(=O)Me | Cl | CH₂OMe | O |
| C-17608 | Me(S) | single bond | H | CH(Me)CH₂OMe | C(=O)Me | Cl | CH₂OMe | O |
| C-17609 | H | single bond | Me | CH(Me)CH₂OMe | C(=O)Me | Me | CH₂OMe | O |
| C-17610 | Me | single bond | H | CH(Me)CH₂OMe | C(=O)Me | Me | CH₂OMe | O |
| C-17611 | Me(R) | single bond | H | CH(Me)CH₂OMe | C(=O)Me | Me | CH₂OMe | O |
| C-17612 | Me(S) | single bond | H | CH(Me)CH₂OMe | C(=O)Me | Me | CH₂OMe | O |
| C-17613 | H | single bond | Me | CH₂CH=CH₂ | C(=O)Me | Cl | CH₂OMe | O |
| C-17614 | Me | single bond | H | CH₂CH=CH₂ | C(=O)Me | Cl | CH₂OMe | O |
| C-17615 | Me(R) | single bond | H | CH₂CH=CH₂ | C(=O)Me | Cl | CH₂OMe | O |
| C-17616 | Me(S) | single bond | H | CH₂CH=CH₂ | C(=O)Me | Cl | CH₂OMe | O |
| C-17617 | H | single bond | Me | CH₂CH=CH₂ | C(=O)Me | Me | CH₂OMe | O |
| C-17618 | Me | single bond | H | CH₂CH=CH₂ | C(=O)Me | Me | CH₂OMe | O |

**[Table 629]**

| Compound Number | Y | W | Z | R¹ | R² | R³ | R¹⁰ | A |
|---|---|---|---|---|---|---|---|---|
| C-17619 | Me(R) | single bond | H | CH₂CH=CH₂ | C(=O)Me | Me | CH₂OMe | O |
| C-17620 | Me(S) | single bond | H | CH₂CH=CH₂ | C(=O)Me | Me | CH₂OMe | O |
| C-17621 | H | single bond | Me | CH₂C≡CH | C(=O)Me | Cl | CH₂OMe | O |
| C-17622 | Me | single bond | H | CH₂C≡CH | C(=O)Me | Cl | CH₂OMe | O |
| C-17623 | Me(R) | single bond | H | CH₂C≡CH | C(=O)Me | Cl | CH₂OMe | O |
| C-17624 | Me(S) | single bond | H | CH₂C≡CH | C(=O)Me | Cl | CH₂OMe | O |
| C-17625 | H | single bond | Me | CH₂C≡CH | C(=O)Me | Me | CH₂OMe | O |
| C-17626 | Me | single bond | H | CH₂C≡CH | C(=O)Me | Me | CH₂OMe | O |
| C-17627 | Me(R) | single bond | H | CH₂C≡CH | C(=O)Me | Me | CH₂OMe | O |
| C-17628 | Me(S) | single bond | H | CH₂C≡CH | C(=O)Me | Me | CH₂OMe | O |
| C-17629 | H | single bond | Me | CH₂CN | C(=O)Me | Cl | CH₂OMe | O |
| C-17630 | Me | single bond | H | CH₂CN | C(=O)Me | Cl | CH₂OMe | O |
| C-17631 | Me(R) | single bond | H | CH₂CN | C(=O)Me | Cl | CH₂OMe | O |
| C-17632 | Me(S) | single bond | H | CH₂CN | C(=O)Me | Cl | CH₂OMe | O |
| C-17633 | H | single bond | Me | CH₂CN | C(=O)Me | Me | CH₂OMe | O |
| C-17634 | Me | single bond | H | CH₂CN | C(=O)Me | Me | CH₂OMe | O |
| C-17635 | Me(R) | single bond | H | CH₂CN | C(=O)Me | Me | CH₂OMe | O |
| C-17636 | Me(S) | single bond | H | CH₂CN | C(=O)Me | Me | CH₂OMe | O |
| C-17637 | H | single bond | Me | CH(Me)CN | C(=O)Me | Cl | CH₂OMe | O |
| C-17638 | Me | single bond | H | CH(Me)CN | C(=O)Me | Cl | CH₂OMe | O |
| C-17639 | Me(R) | single bond | H | CH(Me)CN | C(=O)Me | Cl | CH₂OMe | O |
| C-17640 | Me(S) | single bond | H | CH(Me)CN | C(=O)Me | Cl | CH₂OMe | O |
| C-17641 | H | single bond | Me | CH(Me)CN | C(=O)Me | Me | CH₂OMe | O |
| C-17642 | Me | single bond | H | CH(Me)CN | C(=O)Me | Me | CH₂OMe | O |
| C-17643 | Me(R) | single bond | H | CH(Me)CN | C(=O)Me | Me | CH₂OMe | O |
| C-17644 | Me(S) | single bond | H | CH(Me)CN | C(=O)Me | Me | CH₂OMe | O |
| C-17645 | H | single bond | Me | C(Me)₂CN | C(=O)Me | Cl | CH₂OMe | O |
| C-17646 | Me | single bond | H | C(Me)₂CN | C(=O)Me | Cl | CH₂OMe | O |
| C-17647 | Me(R) | single bond | H | C(Me)₂CN | C(=O)Me | Cl | CH₂OMe | O |
| C-17648 | Me(S) | single bond | H | C(Me)₂CN | C(=O)Me | Cl | CH₂OMe | O |
| C-17649 | H | single bond | Me | C(Me)₂CN | C(=O)Me | Me | CH₂OMe | O |
| C-17650 | Me | single bond | H | C(Me)₂CN | C(=O)Me | Me | CH₂OMe | O |
| C-17651 | Me(R) | single bond | H | C(Me)₂CN | C(=O)Me | Me | CH₂OMe | O |
| C-17652 | Me(S) | single bond | H | C(Me)₂CN | C(=O)Me | Me | CH₂OMe | O |
| C-17653 | H | single bond | Me | C(Me)(*i*-Pr)CN | C(=O)Me | Cl | CH₂OMe | O |
| C-17654 | Me | single bond | H | C(Me)(*i*-Pr)CN | C(=O)Me | Cl | CH₂OMe | O |
| C-17655 | Me(R) | single bond | H | C(Me)(*i*-Pr)CN | C(=O)Me | Cl | CH₂OMe | O |
| C-17656 | Me(S) | single bond | H | C(Me)(*i*-Pr)CN | C(=O)Me | Cl | CH₂OMe | O |
| C-17657 | H | single bond | Me | C(Me)(*i*-Pr)CN | C(=O)Me | Me | CH₂OMe | O |
| C-17658 | Me | single bond | H | C(Me)(*i*-Pr)CN | C(=O)Me | Me | CH₂OMe | O |
| C-17659 | Me(R) | single bond | H | C(Me)(*i*-Pr)CN | C(=O)Me | Me | CH₂OMe | O |
| C-17660 | Me(S) | single bond | H | C(Me)(*i*-Pr)CN | C(=O)Me | Me | CH₂OMe | O |
| C-17661 | H | single bond | Me | OMe | C(=O)Me | Cl | CH₂OMe | O |
| C-17662 | Me | single bond | H | OMe | C(=O)Me | Cl | CH₂OMe | O |

**[Table 630]**

| Compound Number | Y | W | Z | R¹ | R² | R³ | R¹⁰ | A |
|---|---|---|---|---|---|---|---|---|
| C-17663 | Me(R) | single bond | H | OMe | C(=O)Me | Cl | CH₂OMe | O |
| C-17664 | Me(S) | single bond | H | OMe | C(=O)Me | Cl | CH₂OMe | O |
| C-17665 | H | single bond | Me | OMe | C(=O)Me | Me | CH₂OMe | O |
| C-17666 | Me | single bond | H | OMe | C(=O)Me | Me | CH₂OMe | O |
| C-17667 | Me(R) | single bond | H | OMe | C(=O)Me | Me | CH₂OMe | O |
| C-17668 | Me(S) | single bond | H | OMe | C(=O)Me | Me | CH₂OMe | O |
| C-17669 | H | single bond | Me | CH=NOMe | C(=O)Me | Cl | CH₂OMe | O |
| C-17670 | Me | single bond | H | CH=NOMe | C(=O)Me | Cl | CH₂OMe | O |
| C-17671 | Me(R) | single bond | H | CH=NOMe | C(=O)Me | Cl | CH₂OMe | O |
| C-17672 | Me(S) | single bond | H | CH=NOMe | C(=O)Me | Cl | CH₂OMe | O |
| C-17673 | H | single bond | Me | CH=NOMe | C(=O)Me | Me | CH₂OMe | O |
| C-17674 | Me | single bond | H | CH=NOMe | C(=O)Me | Me | CH₂OMe | O |
| C-17675 | Me(R) | single bond | H | CH=NOMe | C(=O)Me | Me | CH₂OMe | O |
| C-17676 | Me(S) | single bond | H | CH=NOMe | C(=O)Me | Me | CH₂OMe | O |
| C-17677 | H | single bond | Me | morpholin-4-yl | C(=O)Me | Cl | CH₂OMe | O |
| C-17678 | Me | single bond | H | morpholin-4-yl | C(=O)Me | Cl | CH₂OMe | O |
| C-17679 | Me(R) | single bond | H | morpholin-4-yl | C(=O)Me | Cl | CH₂OMe | O |
| C-17680 | Me(S) | single bond | H | morpholin-4-yl | C(=O)Me | Cl | CH₂OMe | O |
| C-17681 | H | single bond | Me | morpholin-4-yl | C(=O)Me | Me | CH₂OMe | O |
| C-17682 | Me | single bond | H | morpholin-4-yl | C(=O)Me | Me | CH₂OMe | O |
| C-17683 | Me(R) | single bond | H | morpholin-4-yl | C(=O)Me | Me | CH₂OMe | O |
| C-17684 | Me(S) | single bond | H | morpholin-4-yl | C(=O)Me | Me | CH₂OMe | O |
| C-17685 | H | single bond | Me | Ph | C(=O)Me | Cl | CH₂OMe | O |
| C-17686 | Me | single bond | H | Ph | C(=O)Me | Cl | CH₂OMe | O |
| C-17687 | Me(R) | single bond | H | Ph | C(=O)Me | Cl | CH₂OMe | O |
| C-17688 | Me(S) | single bond | H | Ph | C(=O)Me | Cl | CH₂OMe | O |
| C-17689 | H | single bond | Me | Ph | C(=O)Me | Me | CH₂OMe | O |
| C-17690 | Me | single bond | H | Ph | C(=O)Me | Me | CH₂OMe | O |
| C-17691 | Me(R) | single bond | H | Ph | C(=O)Me | Me | CH₂OMe | O |
| C-17692 | Me(S) | single bond | H | Ph | C(=O)Me | Me | CH₂OMe | O |
| C-17693 | H | single bond | Me | CH₂Ph | C(=O)Me | Cl | CH₂OMe | O |
| C-17694 | Me | single bond | H | CH₂Ph | C(=O)Me | Cl | CH₂OMe | O |
| C-17695 | Me(R) | single bond | H | CH₂Ph | C(=O)Me | Cl | CH₂OMe | O |
| C-17696 | Me(S) | single bond | H | CH₂Ph | C(=O)Me | Cl | CH₂OMe | O |
| C-17697 | H | single bond | Me | CH₂Ph | C(=O)Me | Me | CH₂OMe | O |
| C-17698 | Me | single bond | H | CH₂Ph | C(=O)Me | Me | CH₂OMe | O |
| C-17699 | Me(R) | single bond | H | CH₂Ph | C(=O)Me | Me | CH₂OMe | O |
| C-17700 | Me(S) | single bond | H | CH₂Ph | C(=O)Me | Me | CH₂OMe | O |
| C-17701 | H | single bond | Me | thiazole-2-yl | C(=O)Me | Cl | CH₂OMe | O |
| C-17702 | Me | single bond | H | thiazole-2-yl | C(=O)Me | Cl | CH₂OMe | O |
| C-17703 | Me(R) | single bond | H | thiazole-2-yl | C(=O)Me | Cl | CH₂OMe | O |
| C-17704 | Me(S) | single bond | H | thiazole-2-yl | C(=O)Me | Cl | CH₂OMe | O |
| C-17705 | H | single bond | Me | thiazole-2-yl | C(=O)Me | Me | CH₂OMe | O |
| C-17706 | Me | single bond | H | thiazole-2-yl | C(=O)Me | Me | CH₂OMe | O |

**[Table 631]**

| Compound Number | Y | W | Z | R¹ | R² | R³ | R¹⁰ | A |
|---|---|---|---|---|---|---|---|---|
| C-17707 | Me(R) | single bond | H | thiazole-2-yl | C(=O)Me | Me | CH₂OMe | O |
| C-17708 | Me(S) | single bond | H | thiazole-2-yl | C(=O)Me | Me | CH₂OMe | O |
| C-17709 | H | single bond | Me | 1-Me-pyrazol-3-yl | C(=O)Me | Cl | CH₂OMe | O |
| C-17710 | Me | single bond | H | 1-Me-pyrazol-3-yl | C(=O)Me | Cl | CH₂OMe | O |
| C-17711 | Me(R) | single bond | H | 1-Me-pyrazol-3-yl | C(=O)Me | Cl | CH₂OMe | O |
| C-17712 | Me(S) | single bond | H | 1-Me-pyrazol-3-yl | C(=O)Me | Cl | CH₂OMe | O |
| C-17713 | H | single bond | Me | 1-Me-pyrazol-3-yl | C(=O)Me | Me | CH₂OMe | O |
| C-17714 | Me | single bond | H | 1-Me-pyrazol-3-yl | C(=O)Me | Me | CH₂OMe | O |
| C-17715 | Me(R) | single bond | H | 1-Me-pyrazol-3-yl | C(=O)Me | Me | CH₂OMe | O |
| C-17716 | Me(S) | single bond | H | 1-Me-pyrazol-3-yl | C(=O)Me | Me | CH₂OMe | O |
| C-17717 | H | single bond | Me | 1-Me-pyrazol-5-yl | C(=O)Me | Cl | CH₂OMe | O |
| C-17718 | Me | single bond | H | 1-Me-pyrazol-5-yl | C(=O)Me | Cl | CH₂OMe | O |
| C-17719 | Me(R) | single bond | H | 1-Me-pyrazol-5-yl | C(=O)Me | Cl | CH₂OMe | O |
| C-17720 | Me(S) | single bond | H | 1-Me-pyrazol-5-yl | C(=O)Me | Cl | CH₂OMe | O |
| C-17721 | H | single bond | Me | 1-Me-pyrazol-5-yl | C(=O)Me | Me | CH₂OMe | O |
| C-17722 | Me | single bond | H | 1-Me-pyrazol-5-yl | C(=O)Me | Me | CH₂OMe | O |
| C-17723 | Me(R) | single bond | H | 1-Me-pyrazol-5-yl | C(=O)Me | Me | CH₂OMe | O |
| C-17724 | Me(S) | single bond | H | 1-Me-pyrazol-5-yl | C(=O)Me | Me | CH₂OMe | O |
| C-17725 | H | single bond | Me | CH₂(pyrimidin-2-yl) | C(=O)Me | Cl | CH₂OMe | O |
| C-17726 | Me | single bond | H | CH₂(pyrimidin-2-yl) | C(=O)Me | Cl | CH₂OMe | O |
| C-17727 | Me(R) | single bond | H | CH₂(pyrimidin-2-yl) | C(=O)Me | Cl | CH₂OMe | O |
| C-17728 | Me(S) | single bond | H | CH₂(pyrimidin-2-yl) | C(=O)Me | Cl | CH₂OMe | O |
| C-17729 | H | single bond | Me | CH₂(pyrimidin-2-yl) | C(=O)Me | Me | CH₂OMe | O |
| C-17730 | Me | single bond | H | CH₂(pyrimidin-2-yl) | C(=O)Me | Me | CH₂OMe | O |
| C-17731 | Me(R) | single bond | H | CH₂(pyrimidin-2-yl) | C(=O)Me | Me | CH₂OMe | O |
| C-17732 | Me(S) | single bond | H | CH₂(pyrimidin-2-yl) | C(=O)Me | Me | CH₂OMe | O |
| C-17733 | H | single bond | Me | Me | C(=O)Me | Cl | C(=O)Me | O |
| C-17734 | Me | single bond | H | Me | C(=O)Me | Cl | C(=O)Me | O |
| C-17735 | Me(R) | single bond | H | Me | C(=O)Me | Cl | C(=O)Me | O |
| C-17736 | Me(S) | single bond | H | Me | C(=O)Me | Cl | C(=O)Me | O |
| C-17737 | H | single bond | Me | Me | C(=O)Me | Me | C(=O)Me | O |
| C-17738 | Me | single bond | H | Me | C(=O)Me | Me | C(=O)Me | O |
| C-17739 | Me(R) | single bond | H | Me | C(=O)Me | Me | C(=O)Me | O |
| C-17740 | Me(S) | single bond | H | Me | C(=O)Me | Me | C(=O)Me | O |
| C-17741 | H | single bond | Me | CH₂CHF₂ | C(=O)Me | Cl | C(=O)Me | O |
| C-17742 | Me | single bond | H | CH₂CHF₂ | C(=O)Me | Cl | C(=O)Me | O |
| C-17743 | Me(R) | single bond | H | CH₂CHF₂ | C(=O)Me | Cl | C(=O)Me | O |
| C-17744 | Me(S) | single bond | H | CH₂CHF₂ | C(=O)Me | Cl | C(=O)Me | O |
| C-17745 | H | single bond | Me | CH₂CHF₂ | C(=O)Me | Me | C(=O)Me | O |
| C-17746 | Me | single bond | H | CH₂CHF₂ | C(=O)Me | Me | C(=O)Me | O |
| C-17747 | Me(R) | single bond | H | CH₂CHF₂ | C(=O)Me | Me | C(=O)Me | O |
| C-17748 | Me(S) | single bond | H | CH₂CHF₂ | C(=O)Me | Me | C(=O)Me | O |
| C-17749 | H | single bond | Me | *n*-Pr | C(=O)Me | Cl | C(=O)Me | O |
| C-17750 | Me | single bond | H | *n*-Pr | C(=O)Me | Cl | C(=O)Me | O |

**[Table 632]**

| Compound Number | Y | W | Z | R¹ | R² | R³ | R¹⁰ | A |
|---|---|---|---|---|---|---|---|---|
| C-17751 | Me(R) | single bond | H | *n*-Pr | C(=O)Me | Cl | C(=O)Me | O |
| C-17752 | Me(S) | single bond | H | *n*-Pr | C(=O)Me | Cl | C(=O)Me | O |
| C-17753 | H | single bond | Me | *n*-Pr | C(=O)Me | Me | C(=O)Me | O |
| C-17754 | Me | single bond | H | *n*-Pr | C(=O)Me | Me | C(=O)Me | O |
| C-17755 | Me(R) | single bond | H | *n*-Pr | C(=O)Me | Me | C(=O)Me | O |
| C-17756 | Me(S) | single bond | H | *n*-Pr | C(=O)Me | Me | C(=O)Me | O |
| C-17757 | H | single bond | Me | *i*-Pr | C(=O)Me | Cl | C(=O)Me | O |
| C-17758 | Me | single bond | H | *i*-Pr | C(=O)Me | Cl | C(=O)Me | O |
| C-17759 | Me(R) | single bond | H | *i*-Pr | C(=O)Me | Cl | C(=O)Me | O |
| C-17760 | Me(S) | single bond | H | *i*-Pr | C(=O)Me | Cl | C(=O)Me | O |
| C-17761 | H | single bond | Me | *i*-Pr | C(=O)Me | Me | C(=O)Me | O |
| C-17762 | Me | single bond | H | *i*-Pr | C(=O)Me | Me | C(=O)Me | O |
| C-17763 | Me(R) | single bond | H | *i*-Pr | C(=O)Me | Me | C(=O)Me | O |
| C-17764 | Me(S) | single bond | H | *i*-Pr | C(=O)Me | Me | C(=O)Me | O |
| C-17765 | H | single bond | Me | *c*-Pr(1-F) | C(=O)Me | Cl | C(=O)Me | O |
| C-17766 | Me | single bond | H | *c*-Pr(1-F) | C(=O)Me | Cl | C(=O)Me | O |
| C-17767 | Me(R) | single bond | H | *c*-Pr(1-F) | C(=O)Me | Cl | C(=O)Me | O |
| C-17768 | Me(S) | single bond | H | *c*-Pr(1-F) | C(=O)Me | Cl | C(=O)Me | O |
| C-17769 | H | single bond | Me | *c*-Pr(1-F) | C(=O)Me | Me | C(=O)Me | O |
| C-17770 | Me | single bond | H | *c*-Pr(1-F) | C(=O)Me | Me | C(=O)Me | O |
| C-17771 | Me(R) | single bond | H | *c*-Pr(1-F) | C(=O)Me | Me | C(=O)Me | O |
| C-17772 | Me(S) | single bond | H | *c*-Pr(1-F) | C(=O)Me | Me | C(=O)Me | O |
| C-17773 | H | single bond | Me | *c*-Pr(2,2-F₂) | C(=O)Me | Cl | C(=O)Me | O |
| C-17774 | Me | single bond | H | *c*-Pr(2,2-F₂) | C(=O)Me | Cl | C(=O)Me | O |
| C-17775 | Me(R) | single bond | H | *c*-Pr(2,2-F₂) | C(=O)Me | Cl | C(=O)Me | O |
| C-17776 | Me(S) | single bond | H | *c*-Pr(2,2-F₂) | C(=O)Me | Cl | C(=O)Me | O |
| C-17777 | H | single bond | Me | *c*-Pr(2,2-F₂) | C(=O)Me | Me | C(=O)Me | O |
| C-17778 | Me | single bond | H | *c*-Pr(2,2-F₂) | C(=O)Me | Me | C(=O)Me | O |
| C-17779 | Me(R) | single bond | H | *c*-Pr(2,2-F₂) | C(=O)Me | Me | C(=O)Me | O |
| C-17780 | Me(S) | single bond | H | *c*-Pr(2,2-F₂) | C(=O)Me | Me | C(=O)Me | O |
| C-17781 | H | single bond | Me | *c*-Pr(1-Cl) | C(=O)Me | Cl | C(=O)Me | O |
| C-17782 | Me | single bond | H | *c*-Pr(1-Cl) | C(=O)Me | Cl | C(=O)Me | O |
| C-17783 | Me(R) | single bond | H | *c*-Pr(1-Cl) | C(=O)Me | Cl | C(=O)Me | O |
| C-17784 | Me(S) | single bond | H | *c*-Pr(1-Cl) | C(=O)Me | Cl | C(=O)Me | O |
| C-17785 | H | single bond | Me | *c*-Pr(1-Cl) | C(=O)Me | Me | C(=O)Me | O |
| C-17786 | Me | single bond | H | *c*-Pr(1-Cl) | C(=O)Me | Me | C(=O)Me | O |
| C-17787 | Me(R) | single bond | H | *c*-Pr(1-Cl) | C(=O)Me | Me | C(=O)Me | O |
| C-17788 | Me(S) | single bond | H | *c*-pr(1-Cl) | C(=O)Me | Me | C(=O)Me | O |
| C-17789 | H | single bond | Me | *c*-Pr(1-Br) | C(=O)Me | Cl | C(=O)Me | O |
| C-17790 | Me | single bond | H | *c*-Pr(1-Br) | C(=O)Me | Cl | C(=O)Me | O |
| C-17791 | Me(R) | single bond | H | *c*-Pr(1-Br) | C(=O)Me | Cl | C(=O)Me | O |
| C-17792 | Me(S) | single bond | H | *c*-Pr(1-Br) | C(=O)Me | Cl | C(=O)Me | O |
| C-17793 | H | single bond | Me | *c*-Pr(1-Br) | C(=O)Me | Me | C(=O)Me | O |
| C-17794 | Me | single bond | H | *c*-Pr(1-Br) | C(=O)Me | Me | C(=O)Me | O |

**[Table 633]**

| Compound Number | Y | W | Z | R¹ | R² | R³ | R¹⁰ | A |
|---|---|---|---|---|---|---|---|---|
| C-17795 | Me(R) | single bond | H | *c*-Pr(1-Br) | C(=O)Me | Me | C(=O)Me | O |
| C-17796 | Me(S) | single bond | H | *c*-Pr(1-Br) | C(=O)Me | Me | C(=O)Me | O |
| C-17797 | H | single bond | Me | *c*-Pr(1-Me) | C(=O)Me | Cl | C(=O)Me | O |
| C-17798 | Me | single bond | H | *c*-Pr(1-Me) | C(=O)Me | Cl | C(=O)Me | O |
| C-17799 | Me(R) | single bond | H | *c*-Pr(1-Me) | C(=O)Me | Cl | C(=O)Me | O |
| C-17800 | Me(S) | single bond | H | *c*-Pr(1-Me) | C(=O)Me | Cl | C(=O)Me | O |
| C-17801 | H | single bond | Me | *c*-Pr(1-Me) | C(=O)Me | Me | C(=O)Me | O |
| C-17802 | Me | single bond | H | *c*-Pr(1-Me) | C(=O)Me | Me | C(=O)Me | O |
| C-17803 | Me(R) | single bond | H | *c*-Pr(1-Me) | C(=O)Me | Me | C(=O)Me | O |
| C-17804 | Me(S) | single bond | H | *c*-Pr(1-Me) | C(=O)Me | Me | C(=O)Me | O |
| C-17805 | H | single bond | Me | *c*-Pr(1-CH₂OH) | C(=O)Me | Cl | C(=O)Me | O |
| C-17806 | Me | single bond | H | *c*-Pr(1-CH₂OH) | C(=O)Me | Cl | C(=O)Me | O |
| C-17807 | Me(R) | single bond | H | *c*-Pr(1-CH₂OH) | C(=O)Me | Cl | C(=O)Me | O |
| C-17808 | Me(S) | single bond | H | *c*-Pr(1-CH₂OH) | C(=O)Me | Cl | C(=O)Me | O |
| C-17809 | H | single bond | Me | *c*-Pr(1-CH₂OH) | C(=O)Me | Me | C(=O)Me | O |
| C-17810 | Me | single bond | H | *c*-Pr(1-CH₂OH) | C(=O)Me | Me | C(=O)Me | O |
| C-17811 | Me(R) | single bond | H | *c*-Pr(1-CH₂OH) | C(=O)Me | Me | C(=O)Me | O |
| C-17812 | Me(S) | single bond | H | *c*-Pr(1-CH₂OH) | C(=O)Me | Me | C(=O)Me | O |
| C-17813 | H | single bond | Me | *c*-Pr[1-CH₂OS(=O)₂Me] | C(=O)Me | Cl | C(=O)Me | O |
| C-17814 | Me | single bond | H | *c*-Pr[1-CH₂OS(=O)₂Me] | C(=O)Me | Cl | C(=O)Me | O |
| C-17815 | Me(R) | single bond | H | *c*-Pr[1-CH₂OS(=O)₂Me] | C(=O)Me | Cl | C(=O)Me | O |
| C-17816 | Me(S) | single bond | H | *c*-Pr[1-CH₂OS(=O)₂Me] | C(=O)Me | Cl | C(=O)Me | O |
| C-17817 | H | single bond | Me | *c*-Pr[1-CH₂OS(=O)₂Me] | C(=O)Me | Me | C(=O)Me | O |
| C-17818 | Me | single bond | H | *c*-Pr[1-CH₂OS(=O)₂Me] | C(=O)Me | Me | C(=O)Me | O |
| C-17819 | Me(R) | single bond | H | *c*-Pr[1-CH₂OS(=O)₂Me] | C(=O)Me | Me | C(=O)Me | O |
| C-17820 | Me(S) | single bond | H | *c*-Pr[1-CH₂OS(=O)₂Me] | C(=O)Me | Me | C(=O)Me | O |
| C-17821 | H | single bond | Me | *c*-Pr(1-CH₂OMe) | C(=O)Me | Cl | C(=O)Me | O |
| C-17822 | Me | single bond | H | *c*-Pr(1-CH₂OMe) | C(=O)Me | Cl | C(=O)Me | O |
| C-17823 | Me(R) | single bond | H | *c*-Pr(1-CH₂OMe) | C(=O)Me | Cl | C(=O)Me | O |
| C-17824 | Me(S) | single bond | H | *c*-Pr(1-CH₂OMe) | C(=O)Me | Cl | C(=O)Me | O |
| C-17825 | H | single bond | Me | *c*-Pr(1-CH₂OMe) | C(=O)Me | Me | C(=O)Me | O |
| C-17826 | Me | single bond | H | *c*-Pr(1-CH₂OMe) | C(=O)Me | Me | C(=O)Me | O |
| C-17827 | Me(R) | single bond | H | *c*-Pr(1-CH₂OMe) | C(=O)Me | Me | C(=O)Me | O |
| C-17828 | Me(S) | single bond | H | *c*-Pr(1-CH₂OMe) | C(=O)Me | Me | C(=O)Me | O |
| C-17829 | H | single bond | Me | *c*-Pr(1-CH₂CN) | C(=O)Me | Cl | C(=O)Me | O |
| C-17830 | Me | single bond | H | *c*-Pr(1-CH₂CN) | C(=O)Me | Cl | C(=O)Me | O |
| C-17831 | Me(R) | single bond | H | *c*-Pr(1-CH₂CN) | C(=O)Me | Cl | C(=O)Me | O |
| C-17832 | Me(S) | single bond | H | *c*-Pr(1-CH₂CN) | C(=O)Me | Cl | C(=O)Me | O |
| C-17833 | H | single bond | Me | *c*-Pr(1-CH₂CN) | C(=O)Me | Me | C(=O)Me | O |
| C-17834 | Me | single bond | H | *c*-Pr(1-CH₂CN) | C(=O)Me | Me | C(=O)Me | O |
| C-17835 | Me(R) | single bond | H | *c*-Pr(1-CH₂CN) | C(=O)Me | Me | C(=O)Me | O |
| C-17836 | Me(S) | single bond | H | *c*-Pr(1-CH₂CN) | C(=O)Me | Me | C(=O)Me | O |
| C-17837 | H | single bond | Me | *c*-Pr(1-CH₂SMe) | C(=O)Me | Cl | C(=O)Me | O |
| C-17838 | Me | single bond | H | *c*-Pr(1-CH₂SMe) | C(=O)Me | Cl | C(=O)Me | O |

**[Table 634]**

| Compound Number | Y | W | Z | R¹ | R² | R³ | R¹⁰ | A |
|---|---|---|---|---|---|---|---|---|
| C-17839 | Me(R) | single bond | H | *c*-Pr(1-CH₂SMe) | C(=O)Me | Cl | C(=O)Me | O |
| C-17840 | Me(S) | single bond | H | *c*-Pr(1-CH₂SMe) | C(=O)Me | Cl | C(=O)Me | O |
| C-17841 | H | single bond | Me | *c*-Pr(1-CH₂SMe) | C(=O)Me | Me | C(=O)Me | O |
| C-17842 | Me | single bond | H | *c*-Pr(1-CH₂SMe) | C(=O)Me | Me | C(=O)Me | O |
| C-17843 | Me(R) | single bond | H | *c*-Pr(1-CH₂SMe) | C(=O)Me | Me | C(=O)Me | O |
| C-17844 | Me(S) | single bond | H | *c*-Pr(1-CH₂SMe) | C(=O)Me | Me | C(=O)Me | O |
| C-17845 | H | single bond | Me | *c*-Pr[1-CH₂S(=O)Me] | C(=O)Me | Cl | C(=O)Me | O |
| C-17846 | Me | single bond | H | *c*-Pr[1-CH₂S(=O)Me] | C(=O)Me | Cl | C(=O)Me | O |
| C-17847 | Me(R) | single bond | H | *c*-Pr[1-CH₂S(=O)Me] | C(=O)Me | Cl | C(=O)Me | O |
| C-17848 | Me(S) | single bond | H | *c*-Pr[1-CH₂S(=O)Me] | C(=O)Me | Cl | C(=O)Me | O |
| C-17849 | H | single bond | Me | *c*-Pr[1-CH₂S(=O)Me] | C(=O)Me | Me | C(=O)Me | O |
| C-17850 | Me | single bond | H | *c*-Pr[1-CH₂S(=O)Me] | C(=O)Me | Me | C(=O)Me | O |
| C-17851 | Me(R) | single bond | H | *c*-Pr[1-CH₂S(=O)Me] | C(=O)Me | Me | C(=O)Me | O |
| C-17852 | Me(S) | single bond | H | *c*-Pr[1-CH₂S(=O)Me] | C(=O)Me | Me | C(=O)Me | O |
| C-17853 | H | single bond | Me | *c*-Pr[1-CH₂S(=O)₂Me] | C(=O)Me | Cl | C(=O)Me | O |
| C-17854 | Me | single bond | H | *c*-Pr[1-CH₂S(=O)₂Me] | C(=O)Me | Cl | C(=O)Me | O |
| C-17855 | Me(R) | single bond | H | *c*-Pr[1-CH₂S(=O)₂Me] | C(=O)Me | Cl | C(=O)Me | O |
| C-17856 | Me(S) | single bond | H | *c*-Pr[1-CH₂S(=O)₂Me] | C(=O)Me | Cl | C(=O)Me | O |
| C-17857 | H | single bond | Me | *c*-Pr[1-CH₂S(=O)₂Me] | C(=O)Me | Me | C(=O)Me | O |
| C-17858 | Me | single bond | H | *c*-Pr[1-CH₂S(=O)₂Me] | C(=O)Me | Me | C(=O)Me | O |
| C-17859 | Me(R) | single bond | H | *c*-Pr[1-CH₂S(=O)₂Me] | C(=O)Me | Me | C(=O)Me | O |
| C-17860 | Me(S) | single bond | H | *c*-Pr[1-CH₂S(=O)₂Me] | C(=O)Me | Me | C(=O)Me | O |
| C-17861 | H | single bond | Me | *c*-Pr(1-CH₂F) | C(=O)Me | Cl | C(=O)Me | O |
| C-17862 | Me | single bond | H | *c*-Pr(1-CH₂F) | C(=O)Me | Cl | C(=O)Me | O |
| C-17863 | Me(R) | single bond | H | *c*-Pr(1-CH₂F) | C(=O)Me | Cl | C(=O)Me | O |
| C-17864 | Me(S) | single bond | H | *c*-Pr(1-CH₂F) | C(=O)Me | Cl | C(=O)Me | O |
| C-17865 | H | single bond | Me | *c*-Pr(1-CH₂F) | C(=O)Me | Me | C(=O)Me | O |
| C-17866 | Me | single bond | H | *c*-Pr(1-CH₂F) | C(=O)Me | Me | C(=O)Me | O |
| C-17867 | Me(R) | single bond | H | *c*-Pr(1-CH₂F) | C(=O)Me | Me | C(=O)Me | O |
| C-17868 | Me(S) | single bond | H | *c*-Pr(1-CH₂F) | C(=O)Me | Me | C(=O)Me | O |
| C-17869 | H | single bond | Me | *c*-Pr(1-CHF₂) | C(=O)Me | Cl | C(=O)Me | O |
| C-17870 | Me | single bond | H | *c*-Pr(1-CHF₂) | C(=O)Me | Cl | C(=O)Me | O |
| C-17871 | Me(R) | single bond | H | *c*-Pr(1-CHF₂) | C(=O)Me | Cl | C(=O)Me | O |
| C-17872 | Me(S) | single bond | H | *c*-Pr(1-CHF₂) | C(=O)Me | Cl | C(=O)Me | O |
| C-17873 | H | single bond | Me | *c*-Pr(1-CHF₂) | C(=O)Me | Me | C(=O)Me | O |
| C-17874 | Me | single bond | H | *c*-Pr(1-CHF₂) | C(=O)Me | Me | C(=O)Me | O |
| C-17875 | Me(R) | single bond | H | *c*-Pr(1-CHF₂) | C(=O)Me | Me | C(=O)Me | O |
| C-17876 | Me(S) | single bond | H | *c*-Pr(1-CHF₂) | C(=O)Me | Me | C(=O)Me | O |
| C-17877 | H | single bond | Me | *c*-Pr(1-CF₃) | C(=O)Me | Cl | C(=O)Me | O |
| C-17878 | Me | single bond | H | *c*-Pr(1-CF₃) | C(=O)Me | Cl | C(=O)Me | O |
| C-17879 | Me(R) | single bond | H | *c*-Pr(1-CF₃) | C(=O)Me | Cl | C(=O)Me | O |
| C-17880 | Me(S) | single bond | H | *c*-Pr(1-CF₃) | C(=O)Me | Cl | C(=O)Me | O |
| C-17881 | H | single bond | Me | *c*-Pr(1-CF₃) | C(=O)Me | Me | C(=O)Me | O |
| C-17882 | Me | single bond | H | *c*-Pr(1-CF₃) | C(=O)Me | Me | C(=O)Me | O |

**[Table 635]**

| Compound Number | Y | W | Z | R¹ | R² | R³ | R¹⁰ | A |
|---|---|---|---|---|---|---|---|---|
| C-17883 | Me(R) | single bond | H | *c*-Pr(1-CF₃) | C(=O)Me | Me | C(=O)Me | O |
| C-17884 | Me(S) | single bond | H | *c*-Pr(1-CF₃) | C(=O)Me | Me | C(=O)Me | O |
| C-17885 | H | single bond | Me | *c*-Pr(1-C≡CH) | C(=O)Me | Cl | C(=O)Me | O |
| C-17886 | Me | single bond | H | *c*-Pr(1-C≡CH) | C(=O)Me | Cl | C(=O)Me | O |
| C-17887 | Me(R) | single bond | H | *c*-Pr(1-C≡CH) | C(=O)Me | Cl | C(=O)Me | O |
| C-17888 | Me(S) | single bond | H | *c*-Pr(1-C≡CH) | C(=O)Me | Cl | C(=O)Me | O |
| C-17889 | H | single bond | Me | *c*-Pr(1-C≡CH) | C(=O)Me | Me | C(=O)Me | O |
| C-17890 | Me | single bond | H | *c*-Pr(1-C≡CH) | C(=O)Me | Me | C(=O)Me | O |
| C-17891 | Me(R) | single bond | H | *c*-Pr(1-C≡CH) | C(=O)Me | Me | C(=O)Me | O |
| C-17892 | Me(S) | single bond | H | *c*-Pr(1-C≡CH) | C(=O)Me | Me | C(=O)Me | O |
| C-17893 | H | single bond | Me | *c*-Pr[1-C(=O)OEt] | C(=O)Me | Cl | C(=O)Me | O |
| C-17894 | Me | single bond | H | *c*-Pr[1-C(=O)OEt] | C(=O)Me | Cl | C(=O)Me | O |
| C-17895 | Me(R) | single bond | H | *c*-Pr[1-C(=O)OEt] | C(=O)Me | Cl | C(=O)Me | O |
| C-17896 | Me(S) | single bond | H | *c*-Pr[1-C(=O)OEt] | C(=O)Me | Cl | C(=O)Me | O |
| C-17897 | H | single bond | Me | *c*-Pr[1-C(=O)OEt] | C(=O)Me | Me | C(=O)Me | O |
| C-17898 | Me | single bond | H | *c*-Pr[1-C(=O)OEt] | C(=O)Me | Me | C(=O)Me | O |
| C-17899 | Me(R) | single bond | H | *c*-Pr[1-C(=O)OEt] | C(=O)Me | Me | C(=O)Me | O |
| C-17900 | Me(S) | single bond | H | *c*-Pr[1-C(=O)OEt] | C(=O)Me | Me | C(=O)Me | O |
| C-17901 | H | single bond | Me | *c*-Pr[1-C(=O)OH] | C(=O)Me | Cl | C(=O)Me | O |
| C-17902 | Me | single bond | H | *c*-Pr[1-C(=O)OH] | C(=O)Me | Cl | C(=O)Me | O |
| C-17903 | Me(R) | single bond | H | *c*-Pr[1-C(=O)OH] | C(=O)Me | Cl | C(=O)Me | O |
| C-17904 | Me(S) | single bond | H | *c*-Pr[1-C(=O)OH] | C(=O)Me | Cl | C(=O)Me | O |
| C-17905 | H | single bond | Me | *c*-Pr[1-C(=O)OH] | C(=O)Me | Me | C(=O)Me | O |
| C-17906 | Me | single bond | H | *c*-Pr[1-C(=O)OH] | C(=O)Me | Me | C(=O)Me | O |
| C-17907 | Me(R) | single bond | H | *c*-Pr[1-C(=O)OH] | C(=O)Me | Me | C(=O)Me | O |
| C-17908 | Me(S) | single bond | H | *c*-Pr[1-C(=O)OH] | C(=O)Me | Me | C(=O)Me | O |
| C-17909 | H | single bond | Me | *c*-Pr[1-C(=O)NH₂] | C(=O)Me | Cl | C(=O)Me | O |
| C-17910 | Me | single bond | H | *c*-Pr[1-C(=O)NH₂] | C(=O)Me | Cl | C(=O)Me | O |
| C-17911 | Me(R) | single bond | H | *c*-Pr[1-C(=O)NH₂] | C(=O)Me | Cl | C(=O)Me | O |
| C-17912 | Me(S) | single bond | H | *c*-Pr[1-C(=O)NH₂] | C(=O)Me | Cl | C(=O)Me | O |
| C-17913 | H | single bond | Me | *c*-Pr[1-C(=O)NH₂] | C(=O)Me | Me | C(=O)Me | O |
| C-17914 | Me | single bond | H | *c*-Pr[1-C(=O)NH₂] | C(=O)Me | Me | C(=O)Me | O |
| C-17915 | Me(R) | single bond | H | *c*-Pr[1-C(=O)NH₂] | C(=O)Me | Me | C(=O)Me | O |
| C-17916 | Me(S) | single bond | H | *c*-Pr[1-C(=O)NH₂] | C(=O)Me | Me | C(=O)Me | O |
| C-17917 | H | single bond | Me | *c*-Pr(1-OMe) | C(=O)Me | Cl | C(=O)Me | O |
| C-17918 | Me | single bond | H | *c*-Pr(1-OMe) | C(=O)Me | Cl | C(=O)Me | O |
| C-17919 | Me(R) | single bond | H | *c*-Pr(1-OMe) | C(=O)Me | Cl | C(=O)Me | O |
| C-17920 | Me(S) | single bond | H | *c*-Pr(1-OMe) | C(=O)Me | Cl | C(=O)Me | O |
| C-17921 | H | single bond | Me | *c*-Pr(1-OMe) | C(=O)Me | Me | C(=O)Me | O |
| C-17922 | Me | single bond | H | *c*-Pr(1-OMe) | C(=O)Me | Me | C(=O)Me | O |
| C-17923 | Me(R) | single bond | H | *c*-Pr(1-OMe) | C(=O)Me | Me | C(=O)Me | O |
| C-17924 | Me(S) | single bond | H | *c*-Pr(1-OMe) | C(=O)Me | Me | C(=O)Me | O |
| C-17925 | H | single bond | Me | *c*-Pr(1-SMe) | C(=O)Me | Cl | C(=O)Me | O |
| C-17926 | Me | single bond | H | *c*-Pr(1-SMe) | C(=O)Me | Cl | C(=O)Me | O |

**[Table 636]**

| Compound Number | Y | W | Z | R¹ | R² | R³ | R¹⁰ | A |
|---|---|---|---|---|---|---|---|---|
| C-17927 | Me(R) | single bond | H | *c*-Pr(1-SMe) | C(=O)Me | Cl | C(=O)Me | O |
| C-17928 | Me(S) | single bond | H | *c*-Pr(1-SMe) | C(=O)Me | Cl | C(=O)Me | O |
| C-17929 | H | single bond | Me | *c*-Pr(1-SMe) | C(=O)Me | Me | C(=O)Me | O |
| C-17930 | Me | single bond | H | *c*-Pr(1-SMe) | C(=O)Me | Me | C(=O)Me | O |
| C-17931 | Me(R) | single bond | H | *c*-Pr(1-SMe) | C(=O)Me | Me | C(=O)Me | O |
| C-17932 | Me(S) | single bond | H | *c*-Pr(1-SMe) | C(=O)Me | Me | C(=O)Me | O |
| C-17933 | H | single bond | Me | *c*-Pr[1-S(=O)Me] | C(=O)Me | Cl | C(=O)Me | O |
| C-17934 | Me | single bond | H | *c*-Pr[1-S(=O)Me] | C(=O)Me | Cl | C(=O)Me | O |
| C-17935 | Me(R) | single bond | H | *c*-Pr[1-S(=O)Me] | C(=O)Me | Cl | C(=O)Me | O |
| C-17936 | Me(S) | single bond | H | *c* -Pr[1-S(=O)Me] | C(=O)Me | Cl | C(=O)Me | O |
| C-17937 | H | single bond | Me | *c*-Pr[1-S(=O)Me] | C(=O)Me | Me | C(=O)Me | O |
| C-17938 | Me | single bond | H | *c*-Pr[1-S(=O)Me] | C(=O)Me | Me | C(=O)Me | O |
| C-17939 | Me(R) | single bond | H | *c* -Pr[1-S(=O)Me] | C(=O)Me | Me | C(=O)Me | O |
| C-17940 | Me(S) | single bond | H | *c* -Pr[1-S(=O)Me] | C(=O)Me | Me | C(=O)Me | O |
| C-17941 | H | single bond | Me | *c*-Pr[1-S(=O)₂Me] | C(=O)Me | Cl | C(=O)Me | O |
| C-17942 | Me | single bond | H | *c*-Pr[1-S(=O)₂Me] | C(=O)Me | Cl | C(=O)Me | O |
| C-17943 | Me(R) | single bond | H | *c*-Pr[1-S(=O)₂Me] | C(=O)Me | Cl | C(=O)Me | O |
| C-17944 | Me(S) | single bond | H | *c*-Pr[1-S(=O)₂Me] | C(=O)Me | Cl | C(=O)Me | O |
| C-17945 | H | single bond | Me | *c*-Pr[1-S(=O)₂Me] | C(=O)Me | Me | C(=O)Me | O |
| C-17946 | Me | single bond | H | *c*-Pr[1-S(=O)₂Me] | C(=O)Me | Me | C(=O)Me | O |
| C-17947 | Me(R) | single bond | H | *c*-Pr[1-S(=O)₂Me] | C(=O)Me | Me | C(=O)Me | O |
| C-17948 | Me(S) | single bond | H | *c*-Pr[1-S(=O)₂Me] | C(=O)Me | Me | C(=O)Me | O |
| C-17949 | H | single bond | Me | *c*-Pr[1-S(=O)₂CHF₂] | C(=O)Me | Cl | C(=O)Me | O |
| C-17950 | Me | single bond | H | *c*-Pr[1-S(=O)₂CHF₂] | C(=O)Me | Cl | C(=O)Me | O |
| C-17951 | Me(R) | single bond | H | *c*-Pr[1-S(=O)₂CHF₂] | C(=O)Me | Cl | C(=O)Me | O |
| C-17952 | Me(S) | single bond | H | *c*-Pr[1-S(=O)₂CHF₂] | C(=O)Me | Cl | C(=O)Me | O |
| C-17953 | H | single bond | Me | *c*-Pr[1-S(=O)₂CHF₂] | C(=O)Me | Me | C(=O)Me | O |
| C-17954 | Me | single bond | H | *c*-Pr[1-S(=O)₂CHF₂] | C(=O)Me | Me | C(=O)Me | O |
| C-17955 | Me(R) | single bond | H | *c*-Pr[1-S(=O)₂CHF₂] | C(=O)Me | Me | C(=O)Me | O |
| C-17956 | Me(S) | single bond | H | *c*-Pr[1-S(=O)₂CHF₂] | C(=O)Me | Me | C(=O)Me | O |
| C-17957 | H | single bond | Me | *c*-Pr[1-S(=O)₂CF₃] | C(=O)Me | Cl | C(=O)Me | O |
| C-17958 | Me | single bond | H | *c*-Pr[1-S(=O)₂CF₃] | C(=O)Me | Cl | C(=O)Me | O |
| C-17959 | Me(R) | single bond | H | *c*-Pr[1-S(=O)₂CF₃] | C(=O)Me | Cl | C(=O)Me | O |
| C-17960 | Me(S) | single bond | H | *c*-Pr[1-S(=O)₂CF₃] | C(=O)Me | Cl | C(=O)Me | O |
| C-17961 | H | single bond | Me | *c*-Pr[1-S(=O)₂CF₃] | C(=O)Me | Me | C(=O)Me | O |
| C-17962 | Me | single bond | H | *c*-Pr[1-S(=O)₂CF₃] | C(=O)Me | Me | C(=O)Me | O |
| C-17963 | Me(R) | single bond | H | *c*-Pr[1-S(=O)₂CF₃] | C(=O)Me | Me | C(=O)Me | O |
| C-17964 | Me(S) | single bond | H | *c*-Pr[1-S(=O)₂CF₃] | C(=O)Me | Me | C(=O)Me | O |
| C-17965 | H | single bond | Me | *c*-Pr[1-(5,6-dihydro-1,4,2-dioxazin-3-yl)] | C(=O)Me | Cl | C(=O)Me | O |
| C-17966 | Me | single bond | H | *c*-Pr[1-(5,6-dihydro-1,4,2-dioxazin-3-yl)] | C(=O)Me | Cl | C(=O)Me | O |
| C-17967 | Me(R) | single bond | H | *c*-Pr[1-(5,6-dihydro-1,4,2-dioxazin-3-yl)] | C(=O)Me | Cl | C(=O)Me | O |
| C-17968 | Me(S) | single bond | H | *c*-Pr[1-(5,6-dihydro-1,4,2-dioxazin-3-yl)] | C(=O)Me | Cl | C(=O)Me | O |
| C-17969 | H | single bond | Me | *c*-Pr[1-(5,6-dihydro-1,4,2-dioxazin-3-yl)] | C(=O)Me | Me | C(=O)Me | O |
| C-17970 | Me | single bond | H | *c*-Pr[1-(5,6-dihydro-1,4,2-dioxazin-3-yl)] | C(=O)Me | Me | C(=O)Me | O |

**[Table 637]**

| Compound Number | Y | W | Z | R¹ | R² | R³ | R¹⁰ | A |
|---|---|---|---|---|---|---|---|---|
| C-17971 | Me(R) | single bond | H | *c*-Pr[1-(5,6-dihydro-1,4,2-dioxazin-3-yl)] | C(=O)Me | Me | C(=O)Me | O |
| C-17972 | Me(S) | single bond | H | *c*-Pr[1-(5,6-dihydro-1,4,2-dioxazin-3-yl)] | C(=O)Me | Me | C(=O)Me | O |
| C-17973 | H | single bond | Me | *c*-Pr[1-(5-hydroxy-1,3,4-oxadiazol-2-yl)] | C(=O)Me | Cl | C(=O)Me | O |
| C-17974 | Me | single bond | H | *c*-Pr[1-(5-hydroxy-1,3,4-oxadiazol-2-yl)] | C(=O)Me | Cl | C(=O)Me | O |
| C-17975 | Me(R) | single bond | H | *c*-Pr[1-(5-hydroxy-1,3,4-oxadiazol-2-yl)] | C(=O)Me | Cl | C(=O)Me | O |
| C-17976 | Me(S) | single bond | H | *c*-Pr[1-(5-hydroxy-1,3,4-oxadiazol-2-yl)] | C(=O)Me | Cl | C(=O)Me | O |
| C-17977 | H | single bond | Me | *c*-Pr[1-(5-hydroxy-1,3,4-oxadiazol-2-yl)] | C(=O)Me | Me | C(=O)Me | O |
| C-17978 | Me | single bond | H | *c*-Pr[1-(5-hydroxy-1,3,4-oxadiazol-2-yl)] | C(=O)Me | Me | C(=O)Me | O |
| C-17979 | Me(R) | single bond | H | *c*-Pr[1-(5-hydroxy-1,3,4-oxadiazol-2-yl)] | C(=O)Me | Me | C(=O)Me | O |
| C-17980 | Me(S) | single bond | H | *c*-Pr[1-(5-hydroxy-1,3,4-oxadiazol-2-yl)] | C(=O)Me | Me | C(=O)Me | O |
| C-17981 | H | single bond | Me | *c* -Pr[1-(pyrimidin-2-yl)] | C(=O)Me | Cl | C(=O)Me | O |
| C-17982 | Me | single bond | H | *c* -Pr[1-(pyrimidin-2-yl)] | C(=O)Me | Cl | C(=O)Me | O |
| C-17983 | Me(R) | single bond | H | *c*-Pr[1-(pyrimidin-2-yl)] | C(=O)Me | Cl | C(=O)Me | O |
| C-17984 | Me(S) | single bond | H | *c*-Pr[1-(pyrimidin-2-yl)] | C(=O)Me | Cl | C(=O)Me | O |
| C-17985 | H | single bond | Me | *c*-Pr[1-(pyrimidin-2-yl)] | C(=O)Me | Me | C(=O)Me | O |
| C-17986 | Me | single bond | H | *c*-Pr[1-(pyrimidin-2-yl)] | C(=O)Me | Me | C(=O)Me | O |
| C-17987 | Me(R) | single bond | H | *c*-Pr[1-(pyrimidin-2-yl)] | C(=O)Me | Me | C(=O)Me | O |
| C-17988 | Me(S) | single bond | H | *c*-Pr[1-(pyrimidin-2-yl)] | C(=O)Me | Me | C(=O)Me | O |
| C-17989 | H | single bond | Me | *t*-Bu | C(=O)Me | Cl | C(=O)Me | O |
| C-17990 | Me | single bond | H | *t*-Bu | C(=O)Me | Cl | C(=O)Me | O |
| C-17991 | Me(R) | single bond | H | *t*-Bu | C(=O)Me | Cl | C(=O)Me | O |
| C-17992 | Me(S) | single bond | H | *t*-Bu | C(=O)Me | Cl | C(=O)Me | O |
| C-17993 | H | single bond | Me | *t*-Bu | C(=O)Me | Me | C(=O)Me | O |
| C-17994 | Me | single bond | H | *t*-Bu | C(=O)Me | Me | C(=O)Me | O |
| C-17995 | Me(R) | single bond | H | *t*-Bu | C(=O)Me | Me | C(=O)Me | O |
| C-17996 | Me(S) | single bond | H | *t*-Bu | C(=O)Me | Me | C(=O)Me | O |
| C-17997 | H | single bond | Me | *c*-Bu | C(=O)Me | Cl | C(=O)Me | O |
| C-17998 | Me | single bond | H | *c*-Bu | C(=O)Me | Cl | C(=O)Me | O |
| C-17999 | Me(R) | single bond | H | *c*-Bu | C(=O)Me | Cl | C(=O)Me | O |
| C-18000 | Me(S) | single bond | H | *c*-Bu | C(=O)Me | Cl | C(=O)Me | O |
| C-18001 | H | single bond | Me | *c*-Bu | C(=O)Me | Me | C(=O)Me | O |
| C-18002 | Me | single bond | H | *c*-Bu | C(=O)Me | Me | C(=O)Me | O |
| C-18003 | Me(R) | single bond | H | *c*-Bu | C(=O)Me | Me | C(=O)Me | O |
| C-18004 | Me(S) | single bond | H | *c*-Bu | C(=O)Me | Me | C(=O)Me | O |
| C-18005 | H | single bond | Me | *c*-Bu(1-CN) | C(=O)Me | Cl | C(=O)Me | O |
| C-18006 | Me | single bond | H | *c*-Bu(1-CN) | C(=O)Me | Cl | C(=O)Me | O |
| C-18007 | Me(R) | single bond | H | *c*-Bu(1-CN) | C(=O)Me | Cl | C(=O)Me | O |
| C-18008 | Me(S) | single bond | H | *c*-Bu(1-CN) | C(=O)Me | Cl | C(=O)Me | O |
| C-18009 | H | single bond | Me | *c*-Bu(1-CN) | C(=O)Me | Me | C(=O)Me | O |
| C-18010 | Me | single bond | H | *c*-Bu(1-CN) | C(=O)Me | Me | C(=O)Me | O |
| C-18011 | Me(R) | single bond | H | *c*-Bu(1-CN) | C(=O)Me | Me | C(=O)Me | O |
| C-18012 | Me(S) | single bond | H | *c*-Bu(1-CN) | C(=O)Me | Me | C(=O)Me | O |
| C-18013 | H | single bond | Me | *c*-Bu(3,3-F₂) | C(=O)Me | Cl | C(=O)Me | O |
| C-18014 | Me | single bond | H | *c*-Bu(3,3-F₂) | C(=O)Me | Cl | C(=O)Me | O |

**[Table 638]**

| Compound Number | Y | W | Z | R¹ | R² | R³ | R¹⁰ | A |
|---|---|---|---|---|---|---|---|---|
| C-18015 | Me(R) | single bond | H | *c*-Bu(3,3-F₂) | C(=O)Me | Cl | C(=O)Me | O |
| C-18016 | Me(S) | single bond | H | *c*-Bu(3,3-F₂) | C(=O)Me | Cl | C(=O)Me | O |
| C-18017 | H | single bond | Me | *c*-Bu(3,3-F₂) | C(=O)Me | Me | C(=O)Me | O |
| C-18018 | Me | single bond | H | *c*-Bu(3,3-F₂) | C(=O)Me | Me | C(=O)Me | O |
| C-18019 | Me(R) | single bond | H | *c*-Bu(3,3-F₂) | C(=O)Me | Me | C(=O)Me | O |
| C-18020 | Me(S) | single bond | H | *c*-Bu(3,3-F₂) | C(=O)Me | Me | C(=O)Me | O |
| C-18021 | H | single bond | Me | oxetan-3-yl | C(=O)Me | Cl | C(=O)Me | O |
| C-18022 | Me | single bond | H | oxetan-3-yl | C(=O)Me | Cl | C(=O)Me | O |
| C-18023 | Me(R) | single bond | H | oxetan-3-yl | C(=O)Me | Cl | C(=O)Me | O |
| C-18024 | Me(S) | single bond | H | oxetan-3-yl | C(=O)Me | Cl | C(=O)Me | O |
| C-18025 | H | single bond | Me | oxetan-3-yl | C(=O)Me | Me | C(=O)Me | O |
| C-18026 | Me | single bond | H | oxetan-3-yl | C(=O)Me | Me | C(=O)Me | O |
| C-18027 | Me(R) | single bond | H | oxetan-3-yl | C(=O)Me | Me | C(=O)Me | O |
| C-18028 | Me(S) | single bond | H | oxetan-3-yl | C(=O)Me | Me | C(=O)Me | O |
| C-18029 | H | single bond | Me | oxetan(3-CN)-3-yl | C(=O)Me | Cl | C(=O)Me | O |
| C-18030 | Me | single bond | H | oxetan(3-CN)-3-yl | C(=O)Me | Cl | C(=O)Me | O |
| C-18031 | Me(R) | single bond | H | oxetan(3-CN)-3-yl | C(=O)Me | Cl | C(=O)Me | O |
| C-18032 | Me(S) | single bond | H | oxetan(3-CN)-3-yl | C(=O)Me | Cl | C(=O)Me | O |
| C-18033 | H | single bond | Me | oxetan(3-CN)-3-yl | C(=O)Me | Me | C(=O)Me | O |
| C-18034 | Me | single bond | H | oxetan(3-CN)-3-yl | C(=O)Me | Me | C(=O)Me | O |
| C-18035 | Me(R) | single bond | H | oxetan(3-CN)-3-yl | C(=O)Me | Me | C(=O)Me | O |
| C-18036 | Me(S) | single bond | H | oxetan(3-CN)-3-yl | C(=O)Me | Me | C(=O)Me | O |
| C-18037 | H | single bond | Me | azetidin[1-C(=O)Me]-3-yl | C(=O)Me | Cl | C(=O)Me | O |
| C-18038 | Me | single bond | H | azetidin[1-C(=O)Me]-3-yl | C(=O)Me | Cl | C(=O)Me | O |
| C-18039 | Me(R) | single bond | H | azetidin[1-C(=O)Me]-3-yl | C(=O)Me | Cl | C(=O)Me | O |
| C-18040 | Me(S) | single bond | H | azetidin[1-C(=O)Me]-3-yl | C(=O)Me | Cl | C(=O)Me | O |
| C-18041 | H | single bond | Me | azetidin[1-C(=O)Me]-3-yl | C(=O)Me | Me | C(=O)Me | O |
| C-18042 | Me | single bond | H | azetidin[1-C(=O)Me]-3-yl | C(=O)Me | Me | C(=O)Me | O |
| C-18043 | Me(R) | single bond | H | azetidin[1-C(=O)Me]-3-yl | C(=O)Me | Me | C(=O)Me | O |
| C-18044 | Me(S) | single bond | H | azetidin[1-C(=O)Me]-3-yl | C(=O)Me | Me | C(=O)Me | O |
| C-18045 | H | single bond | Me | azetidin[1-S(=O)₂Me]-3-yl | C(=O)Me | Cl | C(=O)Me | O |
| C-18046 | Me | single bond | H | azetidin[1-S(=O)₂Me]-3-yl | C(=O)Me | Cl | C(=O)Me | O |
| C-18047 | Me(R) | single bond | H | azetidin[1-S(=O)₂Me]-3-yl | C(=O)Me | Cl | C(=O)Me | O |
| C-18048 | Me(S) | single bond | H | azetidin[1-S(=O)₂Me]-3-yl | C(=O)Me | Cl | C(=O)Me | O |
| C-18049 | H | single bond | Me | azetidin[1-S(=O)₂Me]-3-yl | C(=O)Me | Me | C(=O)Me | O |
| C-18050 | Me | single bond | H | azetidin[1-S(=O)₂Me]-3-yl | C(=O)Me | Me | C(=O)Me | O |
| C-18051 | Me(R) | single bond | H | azetidin[1-S(=O)₂Me]-3-yl | C(=O)Me | Me | C(=O)Me | O |
| C-18052 | Me(S) | single bond | H | azetidin[1-S(=O)₂Me]-3-yl | C(=O)Me | Me | C(=O)Me | O |
| C-18053 | H | single bond | Me | azetidin[1-S(=O)₂CHF₂]-3-yl | C(=O)Me | Cl | C(=O)Me | O |
| C-18054 | Me | single bond | H | azetidin[1-S(=O)₂CHF₂]-3-yl | C(=O)Me | Cl | C(=O)Me | O |
| C-18055 | Me(R) | single bond | H | azetidin[1-S(=O)₂CHF₂]-3-yl | C(=O)Me | Cl | C(=O)Me | O |
| C-18056 | Me(S) | single bond | H | azetidin[1-S(=O)₂CHF₂]-3-yl | C(=O)Me | Cl | C(=O)Me | O |
| C-18057 | H | single bond | Me | azetidin[1-S(=O)₂CHF₂]-3-yl | C(=O)Me | Me | C(=O)Me | O |
| C-18058 | Me | single bond | H | azetidin[1-S(=O)₂CHF₂]-3-yl | C(=O)Me | Me | C(=O)Me | O |

**[Table 639]**

| Compound Number | Y | W | Z | R¹ | R² | R³ | R¹⁰ | A |
|---|---|---|---|---|---|---|---|---|
| C-18059 | Me(R) | single bond | H | azetidin[1-S(=O)₂CHF₂]-3-yl | C(=O)Me | Me | C(=O)Me | O |
| C-18060 | Me(S) | single bond | H | azetidin[1-S(=O)₂CHF₂]-3-yl | C(=O)Me | Me | C(=O)Me | O |
| C-18061 | H | single bond | Me | *c*-Pen | C(=O)Me | Cl | C(=O)Me | O |
| C-18062 | Me | single bond | H | *c*-Pen | C(=O)Me | Cl | C(=O)Me | O |
| C-18063 | Me(R) | single bond | H | *c*-Pen | C(=O)Me | Cl | C(=O)Me | O |
| C-18064 | Me(S) | single bond | H | *c*-Pen | C(=O)Me | Cl | C(=O)Me | O |
| C-18065 | H | single bond | Me | *c*-Pen | C(=O)Me | Me | C(=O)Me | O |
| C-18066 | Me | single bond | H | *c*-Pen | C(=O)Me | Me | C(=O)Me | O |
| C-18067 | Me(R) | single bond | H | *c*-Pen | C(=O)Me | Me | C(=O)Me | O |
| C-18068 | Me(S) | single bond | H | *c*-Pen | C(=O)Me | Me | C(=O)Me | O |
| C-18069 | H | single bond | Me | tetrahydrofuran-3-yl | C(=O)Me | Cl | C(=O)Me | O |
| C-18070 | Me | single bond | H | tetrahydrofuran-3-yl | C(=O)Me | Cl | C(=O)Me | O |
| C-18071 | Me(R) | single bond | H | tetrahydrofuran-3-yl | C(=O)Me | Cl | C(=O)Me | O |
| C-18072 | Me(S) | single bond | H | tetrahydrofuran-3-yl | C(=O)Me | Cl | C(=O)Me | O |
| C-18073 | H | single bond | Me | tetrahydrofuran-3-yl | C(=O)Me | Me | C(=O)Me | O |
| C-18074 | Me | single bond | H | tetrahydrofuran-3-yl | C(=O)Me | Me | C(=O)Me | O |
| C-18075 | Me(R) | single bond | H | tetrahydrofuran-3-yl | C(=O)Me | Me | C(=O)Me | O |
| C-18076 | Me(S) | single bond | H | tetrahydrofuran-3-yl | C(=O)Me | Me | C(=O)Me | O |
| C-18077 | H | single bond | Me | tetrahydrothiophen-3-yl | C(=O)Me | Cl | C(=O)Me | O |
| C-18078 | Me | single bond | H | tetrahydrothiophen-3-yl | C(=O)Me | Cl | C(=O)Me | O |
| C-18079 | Me(R) | single bond | H | tetrahydrothiophen-3-yl | C(=O)Me | Cl | C(=O)Me | O |
| C-18080 | Me(S) | single bond | H | tetrahydrothiophen-3-yl | C(=O)Me | Cl | C(=O)Me | O |
| C-18081 | H | single bond | Me | tetrahydrothiophen-3-yl | C(=O)Me | Me | C(=O)Me | O |
| C-18082 | Me | single bond | H | tetrahydrothiophen-3-yl | C(=O)Me | Me | C(=O)Me | O |
| C-18083 | Me(R) | single bond | H | tetrahydrothiophen-3-yl | C(=O)Me | Me | C(=O)Me | O |
| C-18084 | Me(S) | single bond | H | tetrahydrothiophen-3-yl | C(=O)Me | Me | C(=O)Me | O |
| C-18085 | H | single bond | Me | *c*-Pen(1-CN) | C(=O)Me | Cl | C(=O)Me | O |
| C-18086 | Me | single bond | H | *c*-Pen(1-CN) | C(=O)Me | Cl | C(=O)Me | O |
| C-18087 | Me(R) | single bond | H | *c*-Pen(1-CN) | C(=O)Me | Cl | C(=O)Me | O |
| C-18088 | Me(S) | single bond | H | *c*-Pen(1-CN) | C(=O)Me | Cl | C(=O)Me | O |
| C-18089 | H | single bond | Me | *c*-Pen(1-CN) | C(=O)Me | Me | C(=O)Me | O |
| C-18090 | Me | single bond | H | *c*-Pen(1-CN) | C(=O)Me | Me | C(=O)Me | O |
| C-18091 | Me(R) | single bond | H | *c*-Pen(1-CN) | C(=O)Me | Me | C(=O)Me | O |
| C-18092 | Me(S) | single bond | H | *c*-Pen(1-CN) | C(=O)Me | Me | C(=O)Me | O |
| C-18093 | H | single bond | Me | tetrahydrofuran(3-CN)-3-yl | C(=O)Me | Cl | C(=O)Me | O |
| C-18094 | Me | single bond | H | tetrahydrofuran(3-CN)-3-yl | C(=O)Me | Cl | C(=O)Me | O |
| C-18095 | Me(R) | single bond | H | tetrahydrofuran(3-CN)-3-yl | C(=O)Me | Cl | C(=O)Me | O |
| C-18096 | Me(S) | single bond | H | tetrahydrofuran(3-CN)-3-yl | C(=O)Me | Cl | C(=O)Me | O |
| C-18097 | H | single bond | Me | tetrahydrofuran(3-CN)-3-yl | C(=O)Me | Me | C(=O)Me | O |
| C-18098 | Me | single bond | H | tetrahydrofuran(3-CN)-3-yl | C(=O)Me | Me | C(=O)Me | O |
| C-18099 | Me(R) | single bond | H | tetrahydrofuran(3-CN)-3-yl | C(=O)Me | Me | C(=O)Me | O |
| C-18100 | Me(S) | single bond | H | tetrahydrofuran(3-CN)-3-yl | C(=O)Me | Me | C(=O)Me | O |
| C-18101 | H | single bond | Me | tetrahydrothiophen(3-CN)-3-yl | C(=O)Me | Cl | C(=O)Me | O |
| C-18102 | Me | single bond | H | tetrahydrothiophen(3-CN)-3-yl | C(=O)Me | Cl | C(=O)Me | O |

**[Table 640]**

| Compound Number | Y | W | Z | R¹ | R² | R³ | R¹⁰ | A |
|---|---|---|---|---|---|---|---|---|
| C-18103 | Me(R) | single bond | H | tetrahydrothiophen(3-CN)-3-yl | C(=O)Me | Cl | C(=O)Me | O |
| C-18104 | Me(S) | single bond | H | tetrahydrothiophen(3-CN)-3-yl | C(=O)Me | Cl | C(=O)Me | O |
| C-18105 | H | single bond | Me | tetrahydrothiophen(3-CN)-3-yl | C(=O)Me | Me | C(=O)Me | O |
| C-18106 | Me | single bond | H | tetrahydrothiophen(3-CN)-3-yl | C(=O)Me | Me | C(=O)Me | O |
| C-18107 | Me(R) | single bond | H | tetrahydrothiophen(3-CN)-3-yl | C(=O)Me | Me | C(=O)Me | O |
| C-18108 | Me(S) | single bond | H | tetrahydrothiophen(3-CN)-3-yl | C(=O)Me | Me | C(=O)Me | O |
| C-18109 | H | single bond | Me | bicyclo[1.1.1]pentan-1-yl | C(=O)Me | Cl | C(=O)Me | O |
| C-18110 | Me | single bond | H | bicyclo[1.1.1]pentan-1-yl | C(=O)Me | Cl | C(=O)Me | O |
| C-18111 | Me(R) | single bond | H | bicyclo[1.1.1]pentan-1-yl | C(=O)Me | Cl | C(=O)Me | O |
| C-18112 | Me(S) | single bond | H | bicyclo[1.1.1]pentan-1-yl | C(=O)Me | Cl | C(=O)Me | O |
| C-18113 | H | single bond | Me | bicyclo[1.1.1]pentan-1-yl | C(=O)Me | Me | C(=O)Me | O |
| C-18114 | Me | single bond | H | bicyclo[1.1.1]pentan-1-yl | C(=O)Me | Me | C(=O)Me | O |
| C-18115 | Me(R) | single bond | H | bicyclo[1.1.1]pentan-1-yl | C(=O)Me | Me | C(=O)Me | O |
| C-18116 | Me(S) | single bond | H | bicyclo[1.1.1]pentan-1-yl | C(=O)Me | Me | C(=O)Me | O |
| C-18117 | H | single bond | Me | *c*-Hex | C(=O)Me | Cl | C(=O)Me | O |
| C-18118 | Me | single bond | H | *c*-Hex | C(=O)Me | Cl | C(=O)Me | O |
| C-18119 | Me(R) | single bond | H | *c*-Hex | C(=O)Me | Cl | C(=O)Me | O |
| C-18120 | Me(S) | single bond | H | *c*-Hex | C(=O)Me | Cl | C(=O)Me | O |
| C-18121 | H | single bond | Me | *c*-Hex | C(=O)Me | Me | C(=O)Me | O |
| C-18122 | Me | single bond | H | *c*-Hex | C(=O)Me | Me | C(=O)Me | O |
| C-18123 | Me(R) | single bond | H | *c*-Hex | C(=O)Me | Me | C(=O)Me | O |
| C-18124 | Me(S) | single bond | H | *c*-Hex | C(=O)Me | Me | C(=O)Me | O |
| C-18125 | H | single bond | Me | tetrahydropyran-4-yl | C(=O)Me | Cl | C(=O)Me | O |
| C-18126 | Me | single bond | H | tetrahydropyran-4-yl | C(=O)Me | Cl | C(=O)Me | O |
| C-18127 | Me(R) | single bond | H | tetrahydropyran-4-yl | C(=O)Me | Cl | C(=O)Me | O |
| C-18128 | Me(S) | single bond | H | tetrahydropyran-4-yl | C(=O)Me | Cl | C(=O)Me | O |
| C-18129 | H | single bond | Me | tetrahydropyran-4-yl | C(=O)Me | Me | C(=O)Me | O |
| C-18130 | Me | single bond | H | tetrahydropyran-4-yl | C(=O)Me | Me | C(=O)Me | O |
| C-18131 | Me(R) | single bond | H | tetrahydropyran-4-yl | C(=O)Me | Me | C(=O)Me | O |
| C-18132 | Me(S) | single bond | H | tetrahydropyran-4-yl | C(=O)Me | Me | C(=O)Me | O |
| C-18133 | H | single bond | Me | tetrahydrothiopyran-4-yl | C(=O)Me | Cl | C(=O)Me | O |
| C-18134 | Me | single bond | H | tetrahydrothiopyran-4-yl | C(=O)Me | Cl | C(=O)Me | O |
| C-18135 | Me(R) | single bond | H | tetrahydrothiopyran-4-yl | C(=O)Me | Cl | C(=O)Me | O |
| C-18136 | Me(S) | single bond | H | tetrahydrothiopyran-4-yl | C(=O)Me | Cl | C(=O)Me | O |
| C-18137 | H | single bond | Me | tetrahydrothiopyran-4-yl | C(=O)Me | Me | C(=O)Me | O |
| C-18138 | Me | single bond | H | tetrahydrothiopyran-4-yl | C(=O)Me | Me | C(=O)Me | O |
| C-18139 | Me(R) | single bond | H | tetrahydrothiopyran-4-yl | C(=O)Me | Me | C(=O)Me | O |
| C-18140 | Me(S) | single bond | H | tetrahydrothiopyran-4-yl | C(=O)Me | Me | C(=O)Me | O |
| C-18141 | H | single bond | Me | *c*-Hex(1-CN) | C(=O)Me | Cl | C(=O)Me | O |
| C-18142 | Me | single bond | H | *c*-Hex(1-CN) | C(=O)Me | Cl | C(=O)Me | O |
| C-18143 | Me(R) | single bond | H | *c*-Hex(1-CN) | C(=O)Me | Cl | C(=O)Me | O |
| C-18144 | Me(S) | single bond | H | *c*-Hex(1-CN) | C(=O)Me | Cl | C(=O)Me | O |
| C-18145 | H | single bond | Me | *c*-Hex(1-CN) | C(=O)Me | Me | C(=O)Me | O |
| C-18146 | Me | single bond | H | *c*-Hex(1-CN) | C(=O)Me | Me | C(=O)Me | O |

**[Table 641]**

| Compound Number | Y | W | Z | R¹ | R² | R³ | R¹⁰ | A |
|---|---|---|---|---|---|---|---|---|
| C-18147 | Me(R) | single bond | H | *c*-Hex(1-CN) | C(=O)Me | Me | C(=O)Me | O |
| C-18148 | Me(S) | single bond | H | *c*-Hex(1-CN) | C(=O)Me | Me | C(=O)Me | O |
| C-18149 | H | single bond | Me | tetrahydropyran(4-CN)-4-yl | C(=O)Me | Cl | C(=O)Me | O |
| C-18150 | Me | single bond | H | tetrahydropyran(4-CN)-4-yl | C(=O)Me | Cl | C(=O)Me | O |
| C-18151 | Me(R) | single bond | H | tetrahydropyran(4-CN)-4-yl | C(=O)Me | Cl | C(=O)Me | O |
| C-18152 | Me(S) | single bond | H | tetrahydropyran(4-CN)-4-yl | C(=O)Me | Cl | C(=O)Me | O |
| C-18153 | H | single bond | Me | tetrahydropyran(4-CN)-4-yl | C(=O)Me | Me | C(=O)Me | O |
| C-18154 | Me | single bond | H | tetrahydropyran(4-CN)-4-yl | C(=O)Me | Me | C(=O)Me | O |
| C-18155 | Me(R) | single bond | H | tetrahydropyran(4-CN)-4-yl | C(=O)Me | Me | C(=O)Me | O |
| C-18156 | Me(S) | single bond | H | tetrahydropyran(4-CN)-4-yl | C(=O)Me | Me | C(=O)Me | O |
| C-18157 | H | single bond | Me | tetrahydrothiopyran(4-CN)-4-yl | C(=O)Me | Cl | C(=O)Me | O |
| C-18158 | Me | single bond | H | tetrahydrothiopyran(4-CN)-4-yl | C(=O)Me | Cl | C(=O)Me | O |
| C-18159 | Me(R) | single bond | H | tetrahydrothiopyran(4-CN)-4-yl | C(=O)Me | Cl | C(=O)Me | O |
| C-18160 | Me(S) | single bond | H | tetrahydrothiopyran(4-CN)-4-yl | C(=O)Me | Cl | C(=O)Me | O |
| C-18161 | H | single bond | Me | tetrahydrothiopyran(4-CN)-4-yl | C(=O)Me | Me | C(=O)Me | O |
| C-18162 | Me | single bond | H | tetrahydrothiopyran(4-CN)-4-yl | C(=O)Me | Me | C(=O)Me | O |
| C-18163 | Me(R) | single bond | H | tetrahydrothiopyran(4-CN)-4-yl | C(=O)Me | Me | C(=O)Me | O |
| C-18164 | Me(S) | single bond | H | tetrahydrothiopyran(4-CN)-4-yl | C(=O)Me | Me | C(=O)Me | O |
| C-18165 | H | single bond | Me | CH₂(*c*-Pr) | C(=O)Me | Cl | C(=O)Me | O |
| C-18166 | Me | single bond | H | CH₂(*c*-Pr) | C(=O)Me | Cl | C(=O)Me | O |
| C-18167 | Me(R) | single bond | H | CH₂(*c*-Pr) | C(=O)Me | Cl | C(=O)Me | O |
| C-18168 | Me(S) | single bond | H | CH₂(*c*-Pr) | C(=O)Me | Cl | C(=O)Me | O |
| C-18169 | H | single bond | Me | CH₂(*c*-Pr) | C(=O)Me | Me | C(=O)Me | O |
| C-18170 | Me | single bond | H | CH₂(*c*-Pr) | C(=O)Me | Me | C(=O)Me | O |
| C-18171 | Me(R) | single bond | H | CH₂(*c*-Pr) | C(=O)Me | Me | C(=O)Me | O |
| C-18172 | Me(S) | single bond | H | CH₂(*c*-Pr) | C(=O)Me | Me | C(=O)Me | O |
| C-18173 | H | single bond | Me | CH₂[*c*-Pr(1-CN)] | C(=O)Me | Cl | C(=O)Me | O |
| C-18174 | Me | single bond | H | CH₂[*c*-Pr(1-CN)] | C(=O)Me | Cl | C(=O)Me | O |
| C-18175 | Me(R) | single bond | H | CH₂[*c*-Pr(1-CN)] | C(=O)Me | Cl | C(=O)Me | O |
| C-18176 | Me(S) | single bond | H | CH₂[*c*-Pr(1-CN)] | C(=O)Me | Cl | C(=O)Me | O |
| C-18177 | H | single bond | Me | CH₂[*c*-Pr(1-CN)] | C(=O)Me | Me | C(=O)Me | O |
| C-18178 | Me | single bond | H | CH₂[*c*-Pr(1-CN)] | C(=O)Me | Me | C(=O)Me | O |
| C-18179 | Me(R) | single bond | H | CH₂[*c*-Pr(1-CN)] | C(=O)Me | Me | C(=O)Me | O |
| C-18180 | Me(S) | single bond | H | CH₂[*c*-Pr(1-CN)] | C(=O)Me | Me | C(=O)Me | O |
| C-18181 | H | single bond | Me | CH(Me)(*c*-Pr) | C(=O)Me | Cl | C(=O)Me | O |
| C-18182 | Me | single bond | H | CH(Me)(*c*-Pr) | C(=O)Me | Cl | C(=O)Me | O |
| C-18183 | Me(R) | single bond | H | CH(Me)(*c*-Pr) | C(=O)Me | Cl | C(=O)Me | O |
| C-18184 | Me(S) | single bond | H | CH(Me)(*c*-Pr) | C(=O)Me | Cl | C(=O)Me | O |
| C-18185 | H | single bond | Me | CH(Me)(*c*-Pr) | C(=O)Me | Me | C(=O)Me | O |
| C-18186 | Me | single bond | H | CH(Me)(*c*-Pr) | C(=O)Me | Me | C(=O)Me | O |
| C-18187 | Me(R) | single bond | H | CH(Me)(*c*-Pr) | C(=O)Me | Me | C(=O)Me | O |
| C-18188 | Me(S) | single bond | H | CH(Me)(*c*-Pr) | C(=O)Me | Me | C(=O)Me | O |
| C-18189 | H | single bond | Me | CH(Me)CH₂OMe | C(=O)Me | Cl | C(=O)Me | O |
| C-18190 | Me | single bond | H | CH(Me)CH₂OMe | C(=O)Me | Cl | C(=O)Me | O |

**[Table 642]**

| Compound Number | Y | W | Z | R¹ | R² | R³ | R¹⁰ | A |
|---|---|---|---|---|---|---|---|---|
| C-18191 | Me(R) | single bond | H | CH(Me)CH₂OMe | C(=O)Me | Cl | C(=O)Me | O |
| C-18192 | Me(S) | single bond | H | CH(Me)CH₂OMe | C(=O)Me | Cl | C(=O)Me | O |
| C-18193 | H | single bond | Me | CH(Me)CH₂OMe | C(=O)Me | Me | C(=O)Me | O |
| C-18194 | Me | single bond | H | CH(Me)CH₂OMe | C(=O)Me | Me | C(=O)Me | O |
| C-18195 | Me(R) | single bond | H | CH(Me)CH₂OMe | C(=O)Me | Me | C(=O)Me | O |
| C-18196 | Me(S) | single bond | H | CH(Me)CH₂OMe | C(=O)Me | Me | C(=O)Me | O |
| C-18197 | H | single bond | Me | CH₂CH=CH₂ | C(=O)Me | Cl | C(=O)Me | O |
| C-18198 | Me | single bond | H | CH₂CH=CH₂ | C(=O)Me | Cl | C(=O)Me | O |
| C-18199 | Me(R) | single bond | H | CH₂CH=CH₂ | C(=O)Me | Cl | C(=O)Me | O |
| C-18200 | Me(S) | single bond | H | CH₂CH=CH₂ | C(=O)Me | Cl | C(=O)Me | O |
| C-18201 | H | single bond | Me | CH₂CH=CH₂ | C(=O)Me | Me | C(=O)Me | O |
| C-18202 | Me | single bond | H | CH₂CH=CH₂ | C(=O)Me | Me | C(=O)Me | O |
| C-18203 | Me(R) | single bond | H | CH₂CH=CH₂ | C(=O)Me | Me | C(=O)Me | O |
| C-18204 | Me(S) | single bond | H | CH₂CH=CH₂ | C(=O)Me | Me | C(=O)Me | O |
| C-18205 | H | single bond | Me | CH₂C≡CH | C(=O)Me | Cl | C(=O)Me | O |
| C-18206 | Me | single bond | H | CH₂C≡CH | C(=O)Me | Cl | C(=O)Me | O |
| C-18207 | Me(R) | single bond | H | CH₂C≡CH | C(=O)Me | Cl | C(=O)Me | O |
| C-18208 | Me(S) | single bond | H | CH₂C≡CH | C(=O)Me | Cl | C(=O)Me | O |
| C-18209 | H | single bond | Me | CH₂C≡CH | C(=O)Me | Me | C(=O)Me | O |
| C-18210 | Me | single bond | H | CH₂C≡CH | C(=O)Me | Me | C(=O)Me | O |
| C-18211 | Me(R) | single bond | H | CH₂C≡CH | C(=O)Me | Me | C(=O)Me | O |
| C-18212 | Me(S) | single bond | H | CH₂C≡CH | C(=O)Me | Me | C(=O)Me | O |
| C-18213 | H | single bond | Me | CH₂CN | C(=O)Me | Cl | C(=O)Me | O |
| C-18214 | Me | single bond | H | CH₂CN | C(=O)Me | Cl | C(=O)Me | O |
| C-18215 | Me(R) | single bond | H | CH₂CN | C(=O)Me | Cl | C(=O)Me | O |
| C-18216 | Me(S) | single bond | H | CH₂CN | C(=O)Me | Cl | C(=O)Me | O |
| C-18217 | H | single bond | Me | CH₂CN | C(=O)Me | Me | C(=O)Me | O |
| C-18218 | Me | single bond | H | CH₂CN | C(=O)Me | Me | C(=O)Me | O |
| C-18219 | Me(R) | single bond | H | CH₂CN | C(=O)Me | Me | C(=O)Me | O |
| C-18220 | Me(S) | single bond | H | CH₂CN | C(=O)Me | Me | C(=O)Me | O |
| C-18221 | H | single bond | Me | CH(Me)CN | C(=O)Me | Cl | C(=O)Me | O |
| C-18222 | Me | single bond | H | CH(Me)CN | C(=O)Me | Cl | C(=O)Me | O |
| C-18223 | Me(R) | single bond | H | CH(Me)CN | C(=O)Me | Cl | C(=O)Me | O |
| C-18224 | Me(S) | single bond | H | CH(Me)CN | C(=O)Me | Cl | C(=O)Me | O |
| C-18225 | H | single bond | Me | CH(Me)CN | C(=O)Me | Me | C(=O)Me | O |
| C-18226 | Me | single bond | H | CH(Me)CN | C(=O)Me | Me | C(=O)Me | O |
| C-18227 | Me(R) | single bond | H | CH(Me)CN | C(=O)Me | Me | C(=O)Me | O |
| C-18228 | Me(S) | single bond | H | CH(Me)CN | C(=O)Me | Me | C(=O)Me | O |
| C-18229 | H | single bond | Me | C(Me)₂CN | C(=O)Me | Cl | C(=O)Me | O |
| C-18230 | Me | single bond | H | C(Me)₂CN | C(=O)Me | Cl | C(=O)Me | O |
| C-18231 | Me(R) | single bond | H | C(Me)₂CN | C(=O)Me | Cl | C(=O)Me | O |
| C-18232 | Me(S) | single bond | H | C(Me)₂CN | C(=O)Me | Cl | C(=O)Me | O |
| C-18233 | H | single bond | Me | C(Me)₂CN | C(=O)Me | Me | C(=O)Me | O |
| C-18234 | Me | single bond | H | C(Me)₂CN | C(=O)Me | Me | C(=O)Me | O |

**[Table 643]**

| Compound Number | Y | W | Z | R¹ | R² | R³ | R¹⁰ | A |
|---|---|---|---|---|---|---|---|---|
| C-18235 | Me(R) | single bond | H | C(Me)₂CN | C(=O)Me | Me | C(=O)Me | O |
| C-18236 | Me(S) | single bond | H | C(Me)₂CN | C(=O)Me | Me | C(=O)Me | O |
| C-18237 | H | single bond | Me | C(Me)(*i*-Pr)CN | C(=O)Me | Cl | C(=O)Me | O |
| C-18238 | Me | single bond | H | C(Me)(*i*-Pr)CN | C(=O)Me | Cl | C(=O)Me | O |
| C-18239 | Me(R) | single bond | H | C(Me)(*i*-Pr)CN | C(=O)Me | Cl | C(=O)Me | O |
| C-18240 | Me(S) | single bond | H | C(Me)(*i*-Pr)CN | C(=O)Me | Cl | C(=O)Me | O |
| C-18241 | H | single bond | Me | C(Me)(*i*-Pr)CN | C(=O)Me | Me | C(=O)Me | O |
| C-18242 | Me | single bond | H | C(Me)(*i*-Pr)CN | C(=O)Me | Me | C(=O)Me | O |
| C-18243 | Me(R) | single bond | H | C(Me)(*i*-Pr)CN | C(=O)Me | Me | C(=O)Me | O |
| C-18244 | Me(S) | single bond | H | C(Me)(*i*-Pr)CN | C(=O)Me | Me | C(=O)Me | O |
| C-18245 | H | single bond | Me | OMe | C(=O)Me | Cl | C(=O)Me | O |
| C-18246 | Me | single bond | H | OMe | C(=O)Me | Cl | C(=O)Me | O |
| C-18247 | Me(R) | single bond | H | OMe | C(=O)Me | Cl | C(=O)Me | O |
| C-18248 | Me(S) | single bond | H | OMe | C(=O)Me | Cl | C(=O)Me | O |
| C-18249 | H | single bond | Me | OMe | C(=O)Me | Me | C(=O)Me | O |
| C-18250 | Me | single bond | H | OMe | C(=O)Me | Me | C(=O)Me | O |
| C-18251 | Me(R) | single bond | H | OMe | C(=O)Me | Me | C(=O)Me | O |
| C-18252 | Me(S) | single bond | H | OMe | C(=O)Me | Me | C(=O)Me | O |
| C-18253 | H | single bond | Me | CH=NOMe | C(=O)Me | Cl | C(=O)Me | O |
| C-18254 | Me | single bond | H | CH=NOMe | C(=O)Me | Cl | C(=O)Me | O |
| C-18255 | Me(R) | single bond | H | CH=NOMe | C(=O)Me | Cl | C(=O)Me | O |
| C-18256 | Me(S) | single bond | H | CH=NOMe | C(=O)Me | Cl | C(=O)Me | O |
| C-18257 | H | single bond | Me | CH=NOMe | C(=O)Me | Me | C(=O)Me | O |
| C-18258 | Me | single bond | H | CH=NOMe | C(=O)Me | Me | C(=O)Me | O |
| C-18259 | Me(R) | single bond | H | CH=NOMe | C(=O)Me | Me | C(=O)Me | O |
| C-18260 | Me(S) | single bond | H | CH=NOMe | C(=O)Me | Me | C(=O)Me | O |
| C-18261 | H | single bond | Me | morpholin-4-yl | C(=O)Me | Cl | C(=O)Me | O |
| C-18262 | Me | single bond | H | morpholin-4-yl | C(=O)Me | Cl | C(=O)Me | O |
| C-18263 | Me(R) | single bond | H | morpholin-4-yl | C(=O)Me | Cl | C(=O)Me | O |
| C-18264 | Me(S) | single bond | H | morpholin-4-yl | C(=O)Me | Cl | C(=O)Me | O |
| C-18265 | H | single bond | Me | morpholin-4-yl | C(=O)Me | Me | C(=O)Me | O |
| C-18266 | Me | single bond | H | morpholin-4-yl | C(=O)Me | Me | C(=O)Me | O |
| C-18267 | Me(R) | single bond | H | morpholin-4-yl | C(=O)Me | Me | C(=O)Me | O |
| C-18268 | Me(S) | single bond | H | morpholin-4-yl | C(=O)Me | Me | C(=O)Me | O |
| C-18269 | H | single bond | Me | Ph | C(=O)Me | Cl | C(=O)Me | O |
| C-18270 | Me | single bond | H | Ph | C(=O)Me | Cl | C(=O)Me | O |
| C-18271 | Me(R) | single bond | H | Ph | C(=O)Me | Cl | C(=O)Me | O |
| C-18272 | Me(S) | single bond | H | Ph | C(=O)Me | Cl | C(=O)Me | O |
| C-18273 | H | single bond | Me | Ph | C(=O)Me | Me | C(=O)Me | O |
| C-18274 | Me | single bond | H | Ph | C(=O)Me | Me | C(=O)Me | O |
| C-18275 | Me(R) | single bond | H | Ph | C(=O)Me | Me | C(=O)Me | O |
| C-18276 | Me(S) | single bond | H | Ph | C(=O)Me | Me | C(=O)Me | O |
| C-18277 | H | single bond | Me | CH₂Ph | C(=O)Me | Cl | C(=O)Me | O |
| C-18278 | Me | single bond | H | CH₂Ph | C(=O)Me | Cl | C(=O)Me | O |

**[Table 644]**

| Compound Number | Y | W | Z | R¹ | R² | R³ | R¹⁰ | A |
|---|---|---|---|---|---|---|---|---|
| C-18279 | Me(R) | single bond | H | CH₂Ph | C(=O)Me | Cl | C(=O)Me | O |
| C-18280 | Me(S) | single bond | H | CH₂Ph | C(=O)Me | Cl | C(=O)Me | O |
| C-18281 | H | single bond | Me | CH₂Ph | C(=O)Me | Me | C(=O)Me | O |
| C-18282 | Me | single bond | H | CH₂Ph | C(=O)Me | Me | C(=O)Me | O |
| C-18283 | Me(R) | single bond | H | CH₂Ph | C(=O)Me | Me | C(=O)Me | O |
| C-18284 | Me(S) | single bond | H | CH₂Ph | C(=O)Me | Me | C(=O)Me | O |
| C-18285 | H | single bond | Me | thiazole-2-yl | C(=O)Me | Cl | C(=O)Me | O |
| C-18286 | Me | single bond | H | thiazole-2-yl | C(=O)Me | Cl | C(=O)Me | O |
| C-18287 | Me(R) | single bond | H | thiazole-2-yl | C(=O)Me | Cl | C(=O)Me | O |
| C-18288 | Me(S) | single bond | H | thiazole-2-yl | C(=O)Me | Cl | C(=O)Me | O |
| C-18289 | H | single bond | Me | thiazole-2-yl | C(=O)Me | Me | C(=O)Me | O |
| C-18290 | Me | single bond | H | thiazole-2-yl | C(=O)Me | Me | C(=O)Me | O |
| C-18291 | Me(R) | single bond | H | thiazole-2-yl | C(=O)Me | Me | C(=O)Me | O |
| C-18292 | Me(S) | single bond | H | thiazole-2-yl | C(=O)Me | Me | C(=O)Me | O |
| C-18293 | H | single bond | Me | 1-Me-pyrazol-3-yl | C(=O)Me | Cl | C(=O)Me | O |
| C-18294 | Me | single bond | H | 1-Me-pyrazol-3-yl | C(=O)Me | Cl | C(=O)Me | O |
| C-18295 | Me(R) | single bond | H | 1-Me-pyrazol-3-yl | C(=O)Me | Cl | C(=O)Me | O |
| C-18296 | Me(S) | single bond | H | 1-Me-pyrazol-3-yl | C(=O)Me | Cl | C(=O)Me | O |
| C-18297 | H | single bond | Me | 1-Me-pyrazol-3-yl | C(=O)Me | Me | C(=O)Me | O |
| C-18298 | Me | single bond | H | 1-Me-pyrazol-3-yl | C(=O)Me | Me | C(=O)Me | O |
| C-18299 | Me(R) | single bond | H | 1-Me-pyrazol-3-yl | C(=O)Me | Me | C(=O)Me | O |
| C-18300 | Me(S) | single bond | H | 1-Me-pyrazol-3-yl | C(=O)Me | Me | C(=O)Me | O |
| C-18301 | H | single bond | Me | 1-Me-pyrazol-5-yl | C(=O)Me | Cl | C(=O)Me | O |
| C-18302 | Me | single bond | H | 1-Me-pyrazol-5-yl | C(=O)Me | Cl | C(=O)Me | O |
| C-18303 | Me(R) | single bond | H | 1-Me-pyrazol-5-yl | C(=O)Me | Cl | C(=O)Me | O |
| C-18304 | Me(S) | single bond | H | 1-Me-pyrazol-5-yl | C(=O)Me | Cl | C(=O)Me | O |
| C-18305 | H | single bond | Me | 1-Me-pyrazol-5-yl | C(=O)Me | Me | C(=O)Me | O |
| C-18306 | Me | single bond | H | 1-Me-pyrazol-5-yl | C(=O)Me | Me | C(=O)Me | O |
| C-18307 | Me(R) | single bond | H | 1-Me-pyrazol-5-yl | C(=O)Me | Me | C(=O)Me | O |
| C-18308 | Me(S) | single bond | H | 1-Me-pyrazol-5-yl | C(=O)Me | Me | C(=O)Me | O |
| C-18309 | H | single bond | Me | CH₂(pyrimidin-2-yl) | C(=O)Me | Cl | C(=O)Me | O |
| C-18310 | Me | single bond | H | CH₂(pyrimidin-2-yl) | C(=O)Me | Cl | C(=O)Me | O |
| C-18311 | Me(R) | single bond | H | CH₂(pyrimidin-2-yl) | C(=O)Me | Cl | C(=O)Me | O |
| C-18312 | Me(S) | single bond | H | CH₂(pyrimidin-2-yl) | C(=O)Me | Cl | C(=O)Me | O |
| C-18313 | H | single bond | Me | CH₂(pyrimidin-2-yl) | C(=O)Me | Me | C(=O)Me | O |
| C-18314 | Me | single bond | H | CH₂(pyrimidin-2-yl) | C(=O)Me | Me | C(=O)Me | O |
| C-18315 | Me(R) | single bond | H | CH₂(pyrimidin-2-yl) | C(=O)Me | Me | C(=O)Me | O |
| C-18316 | Me(S) | single bond | H | CH₂(pyrimidin-2-yl) | C(=O)Me | Me | C(=O)Me | O |

**[Table 645]**

| 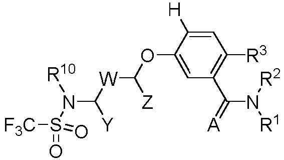 | | | | | | | | |
|---|---|---|---|---|---|---|---|---|
| Compound Number | Y | W | Z | R¹ | R² | R³ | R¹⁰ | A |
| D-0001 | H | single bond | Me | CH₂CF₃ | H | Cl | H | O |
| D-0002 | H | single bond | Et | CH₂CF₃ | H | Cl | H | O |
| D-0003 | H | single bond | *i*-Pr | CH₂CF₃ | H | Cl | H | O |
| D-0004 | Me | single bond | H | CH₂CF₃ | H | Cl | H | O |
| D-0005 | Me(R) | single bond | H | CH₂CF₃ | H | Cl | H | O |
| D-0006 | Me(S) | single bond | H | CH₂CF₃ | H | Cl | H | O |
| D-0007 | Me | single bond | H | CH₂CF₃ | H | Cl | H | S |
| D-0008 | Me(R) | single bond | H | CH₂CF₃ | H | Cl | H | S |
| D-0009 | Me(S) | single bond | H | CH₂CF₃ | H | Cl | H | S |
| D-0010 | Et | single bond | H | CH₂CF₃ | H | Cl | H | O |
| D-0011 | *i*-Pr | single bond | H | CH₂CF₃ | H | Cl | H | O |
| D-0012 | CF₃ | single bond | H | CH₂CF₃ | H | Cl | H | O |
| D-0013 | Me | single bond | Me | CH₂CF₃ | H | Cl | H | O |
| D-0014 | H | CH₂ | Me | CH₂CF₃ | H | Cl | H | O |
| D-0015 | Me | CH₂ | H | CH₂CF₃ | H | Cl | H | O |
| D-0016 | Me | CH₂ | Me | CH₂CF₃ | H | Cl | H | O |
| D-0017 | H | CH(Me) | H | CH₂CF₃ | H | Cl | H | O |
| D-0018 | H | CH(Me) | Me | CH₂CF₃ | H | Cl | H | O |
| D-0019 | Me | CH(Me) | H | CH₂CF₃ | H | Cl | H | O |
| D-0020 | Me | CH(Me) | Me | CH₂CF₃ | H | Cl | H | O |
| D-0021 | H | CH₂CH₂ | Me | CH₂CF₃ | H | Cl | H | O |
| D-0022 | Me | CH₂CH₂ | H | CH₂CF₃ | H | Cl | H | O |
| D-0023 | Me | CH₂CH₂ | Me | CH₂CF₃ | H | Cl | H | O |
| D-0024 | H | CH₂CH(Me) | H | CH₂CF₃ | H | Cl | H | O |
| D-0025 | H | CH₂CH(Me) | Me | CH₂CF₃ | H | Cl | H | O |
| D-0026 | Me | CH₂CH(Me) | H | CH₂CF₃ | H | Cl | H | O |
| D-0027 | Me | CH₂CH(Me) | Me | CH₂CF₃ | H | Cl | H | O |
| D-0028 | H | CH(Me)CH₂ | H | CH₂CF₃ | H | Cl | H | O |
| D-0029 | H | CH(Me)CH₂ | Me | CH₂CF₃ | H | Cl | H | O |
| D-0030 | Me | CH(Me)CH₂ | H | CH₂CF₃ | H | Cl | H | O |
| D-0031 | Me | CH(Me)CH₂ | Me | CH₂CF₃ | H | Cl | H | O |
| D-0032 | H | single bond | Me | CH₂CF₃ | H | Me | H | O |
| D-0033 | H | single bond | Et | CH₂CF₃ | H | Me | H | O |
| D-0034 | H | single bond | *i*-Pr | CH₂CF₃ | H | Me | H | O |
| D-0035 | Me | single bond | H | CH₂CF₃ | H | Me | H | O |
| D-0036 | Me(R) | single bond | H | CH₂CF₃ | H | Me | H | O |
| D-0037 | Me(S) | single bond | H | CH₂CF₃ | H | Me | H | O |
| D-0038 | Me | single bond | H | CH₂CF₃ | H | Me | H | S |

**[Table 646]**

| Compound Number | Y | W | Z | R¹ | R² | R³ | R¹⁰ | A |
|---|---|---|---|---|---|---|---|---|
| 0-0039 | Me(R) | single bond | H | CH₂CF₃ | H | Me | H | S |
| 0-0040 | Me(S) | single bond | H | CH₂CF₃ | H | Me | H | S |
| D-0041 | Et | single bond | H | CH₂CF₃ | H | Me | H | O |
| D-0042 | *i*-Pr | single bond | H | CH₂CF₃ | H | Me | H | O |
| D-0043 | CF₃ | single bond | H | CH₂CF₃ | H | Me | H | O |
| D-0044 | Me | single bond | Me | CH₂CF₃ | H | Me | H | O |
| D-0045 | H | CH₂ | Me | CH₂CF₃ | H | Me | H | O |
| D-0046 | Me | CH₂ | H | CH₂CF₃ | H | Me | H | O |
| D-0047 | Me | CH₂ | Me | CH₂CF₃ | H | Me | H | O |
| D-0048 | H | CH(Me) | H | CH₂CF₃ | H | Me | H | O |
| D-0049 | H | CH(Me) | Me | CH₂CF₃ | H | Me | H | O |
| D-0050 | Me | CH(Me) | H | CH₂CF₃ | H | Me | H | O |
| D-0051 | Me | CH(Me) | Me | CH₂CF₃ | H | Me | H | O |
| D-0052 | H | CH₂CH₂ | Me | CH₂CF₃ | H | Me | H | O |
| D-0053 | Me | CH₂CH₂ | H | CH₂CF₃ | H | Me | H | O |
| D-0054 | Me | CH₂CH₂ | Me | CH₂CF₃ | H | Me | H | O |
| D-0055 | H | CH₂CH(Me) | H | CH₂CF₃ | H | Me | H | O |
| D-0056 | H | CH₂CH(Me) | Me | CH₂CF₃ | H | Me | H | O |
| D-0057 | Me | CH₂CH(Me) | H | CH₂CF₃ | H | Me | H | O |
| D-0058 | Me | CH₂CH(Me) | Me | CH₂CF₃ | H | Me | H | O |
| D-0059 | H | CH(Me)CH₂ | H | CH₂CF₃ | H | Me | H | O |
| D-0060 | H | CH(Me)CH₂ | Me | CH₂CF₃ | H | Me | H | O |
| D-0061 | Me | CH(Me)CH₂ | H | CH₂CF₃ | H | Me | H | O |
| D-0062 | Me | CH(Me)CH₂ | Me | CH₂CF₃ | H | Me | H | O |
| D-0063 | H | single bond | Me | Et | H | Cl | H | O |
| D-0064 | H | single bond | Et | Et | H | Cl | H | O |
| D-0065 | H | single bond | *i*-Pr | Et | H | Cl | H | O |
| D-0066 | Me | single bond | H | Et | H | Cl | H | O |
| D-0067 | Me(R) | single bond | H | Et | H | Cl | H | O |
| D-0068 | Me(S) | single bond | H | Et | H | Cl | H | O |
| D-0069 | Me | single bond | H | Et | H | Cl | H | S |
| D-0070 | Me(R) | single bond | H | Et | H | Cl | H | S |
| D-0071 | Me(S) | single bond | H | Et | H | Cl | H | S |
| D-0072 | Et | single bond | H | Et | H | Cl | H | O |
| D-0073 | *i*-Pr | single bond | H | Et | H | Cl | H | O |
| D-0074 | CF₃ | single bond | H | Et | H | Cl | H | O |
| D-0075 | Me | single bond | Me | Et | H | Cl | H | O |
| D-0076 | H | CH₂ | Me | Et | H | Cl | H | O |
| D-0077 | Me | CH₂ | H | Et | H | Cl | H | O |
| D-0078 | Me | CH₂ | Me | Et | H | Cl | H | O |
| D-0079 | H | CH(Me) | H | Et | H | Cl | H | O |
| D-0080 | H | CH(Me) | Me | Et | H | Cl | H | O |
| D-0081 | Me | CH(Me) | H | Et | H | Cl | H | O |
| D-0082 | Me | CH(Me) | Me | Et | H | Cl | H | O |

**[Table 647]**

| Compound Number | Y | W | Z | R¹ | R² | R³ | R¹⁰ | A |
|---|---|---|---|---|---|---|---|---|
| 0-0083 | H | CH₂CH₂ | Me | Et | H | Cl | H | O |
| D-0084 | Me | CH₂CH₂ | H | Et | H | Cl | H | O |
| D-0085 | Me | CH₂CH₂ | Me | Et | H | Cl | H | O |
| D-0086 | H | CH₂CH(Me) | H | Et | H | Cl | H | O |
| D-0087 | H | CH₂CH(Me) | Me | Et | H | Cl | H | O |
| D-0088 | Me | CH₂CH(Me) | H | Et | H | Cl | H | O |
| D-0089 | Me | CH₂CH(Me) | Me | Et | H | Cl | H | O |
| D-0090 | H | CH(Me)CH₂ | H | Et | H | Cl | H | O |
| D-0091 | H | CH(Me)CH₂ | Me | Et | H | Cl | H | O |
| D-0092 | Me | CH(Me)CH₂ | H | Et | H | Cl | H | O |
| D-0093 | Me | CH(Me)CH₂ | Me | Et | H | Cl | H | O |
| D-0094 | H | single bond | Me | Et | H | Me | H | O |
| D-0095 | H | single bond | Et | Et | H | Me | H | O |
| D-0096 | H | single bond | *i*-Pr | Et | H | Me | H | O |
| D-0097 | Me | single bond | H | Et | H | Me | H | O |
| D-0098 | Me(R) | single bond | H | Et | H | Me | H | O |
| D-0099 | Me(S) | single bond | H | Et | H | Me | H | O |
| D-0100 | Me | single bond | H | Et | H | Me | H | S |
| D-0101 | Me(R) | single bond | H | Et | H | Me | H | S |
| D-0102 | Me(S) | single bond | H | Et | H | Me | H | S |
| D-0103 | Et | single bond | H | Et | H | Me | H | O |
| D-0104 | *i*-Pr | single bond | H | Et | H | Me | H | O |
| D-0105 | CF₃ | single bond | H | Et | H | Me | H | O |
| D-0106 | Me | single bond | Me | Et | H | Me | H | O |
| D-0107 | H | CH₂ | Me | Et | H | Me | H | O |
| D-0108 | Me | CH₂ | H | Et | H | Me | H | O |
| D-0109 | Me | CH₂ | Me | Et | H | Me | H | O |
| D-0110 | H | CH(Me) | H | Et | H | Me | H | O |
| D-0111 | H | CH(Me) | Me | Et | H | Me | H | O |
| D-0112 | Me | CH(Me) | H | Et | H | Me | H | O |
| D-0113 | Me | CH(Me) | Me | Et | H | Me | H | O |
| D-0114 | H | CH₂CH₂ | Me | Et | H | Me | H | O |
| D-0115 | Me | CH₂CH₂ | H | Et | H | Me | H | O |
| D-0116 | Me | CH₂CH₂ | Me | Et | H | Me | H | O |
| D-0117 | H | CH₂CH(Me) | H | Et | H | Me | H | O |
| D-0118 | H | CH₂CH(Me) | Me | Et | H | Me | H | O |
| D-0119 | Me | CH₂CH(Me) | H | Et | H | Me | H | O |
| D-0120 | Me | CH₂CH(Me) | Me | Et | H | Me | H | O |
| D-0121 | H | CH(Me)CH₂ | H | Et | H | Me | H | O |
| D-0122 | H | CH(Me)CH₂ | Me | Et | H | Me | H | O |
| D-0123 | Me | CH(Me)CH₂ | H | Et | H | Me | H | O |
| D-0124 | Me | CH(Me)CH₂ | Me | Et | H | Me | H | O |
| D-0125 | H | single bond | Me | *c*-Pr | H | Cl | H | O |
| D-0126 | H | single bond | Et | *c*-Pr | H | Cl | H | O |

**[Table 648]**

| Compound Number | Y | W | Z | R¹ | R² | R³ | R¹⁰ | A |
|---|---|---|---|---|---|---|---|---|
| D-0127 | H | single bond | *i*-Pr | *c*-Pr | H | Cl | H | O |
| D-0128 | Me | single bond | H | *c*-Pr | H | Cl | H | O |
| D-0129 | Me(R) | single bond | H | *c*-Pr | H | Cl | H | O |
| D-0130 | Me(S) | single bond | H | *c*-Pr | H | Cl | H | O |
| D-0131 | Me | single bond | H | *c*-Pr | H | Cl | H | S |
| D-0132 | Me(R) | single bond | H | *c*-Pr | H | Cl | H | S |
| D-0133 | Me(S) | single bond | H | *c*-Pr | H | Cl | H | S |
| D-0134 | Et | single bond | H | *c*-Pr | H | Cl | H | O |
| D-0135 | *i*-Pr | single bond | H | *c*-Pr | H | Cl | H | O |
| D-0136 | CF₃ | single bond | H | *c*-Pr | H | Cl | H | O |
| D-0137 | Me | single bond | Me | *c*-Pr | H | Cl | H | O |
| D-0138 | H | CH₂ | Me | *c*-Pr | H | Cl | H | O |
| D-0139 | Me | CH₂ | H | *c*-Pr | H | Cl | H | O |
| D-0140 | Me | CH₂ | Me | *c*-Pr | H | Cl | H | O |
| D-0141 | H | CH(Me) | H | *c*-Pr | H | Cl | H | O |
| D-0142 | H | CH(Me) | Me | *c*-Pr | H | Cl | H | O |
| D-0143 | Me | CH(Me) | H | *c*-Pr | H | Cl | H | O |
| D-0144 | Me | CH(Me) | Me | c-Pr | H | Cl | H | O |
| D-0145 | H | CH₂CH₂ | Me | *c*-Pr | H | Cl | H | O |
| D-0146 | Me | CH₂CH₂ | H | *c*-Pr | H | Cl | H | O |
| D-0147 | Me | CH₂CH₂ | Me | *c*-Pr | H | Cl | H | O |
| D-0148 | H | CH₂CH(Me) | H | *c*-Pr | H | Cl | H | O |
| D-0149 | H | CH₂CH(Me) | Me | *c*-Pr | H | Cl | H | O |
| D-0150 | Me | CH₂CH(Me) | H | *c*-Pr | H | Cl | H | O |
| D-0151 | Me | CH₂CH(Me) | Me | *c*-Pr | H | Cl | H | O |
| D-0152 | H | CH(Me)CH₂ | H | *c*-Pr | H | Cl | H | O |
| D-0153 | H | CH(Me)CH₂ | Me | *c*-Pr | H | Cl | H | O |
| D-0154 | Me | CH(Me)CH₂ | H | *c*-Pr | H | Cl | H | O |
| D-0155 | Me | CH(Me)CH₂ | Me | *c*-Pr | H | Cl | H | O |
| D-0156 | H | single bond | Me | *c*-Pr | H | Me | H | O |
| D-0157 | H | single bond | Et | *c*-Pr | H | Me | H | O |
| D-0158 | H | single bond | *i*-Pr | *c*-Pr | H | Me | H | O |
| D-0159 | Me | single bond | H | *c*-Pr | H | Me | H | O |
| D-0160 | Me(R) | single bond | H | *c*-Pr | H | Me | H | O |
| D-0161 | Me(S) | single bond | H | *c*-Pr | H | Me | H | O |
| D-0162 | Me | single bond | H | *c*-Pr | H | Me | H | S |
| D-0163 | Me(R) | single bond | H | *c*-Pr | H | Me | H | S |
| D-0164 | Me(S) | single bond | H | *c*-Pr | H | Me | H | S |
| D-0165 | Et | single bond | H | *c*-Pr | H | Me | H | O |
| D-0166 | *i*-Pr | single bond | H | *c*-Pr | H | Me | H | O |
| D-0167 | CF₃ | single bond | H | *c*-Pr | H | Me | H | O |
| D-0168 | Me | single bond | Me | *c*-Pr | H | Me | H | O |
| D-0169 | H | CH₂ | Me | *c*-Pr | H | Me | H | O |
| D-0170 | Me | CH₂ | H | *c*-Pr | H | Me | H | O |

**[Table 649]**

| Compound Number | Y | W | Z | R¹ | R² | R³ | R¹⁰ | A |
|---|---|---|---|---|---|---|---|---|
| D-0171 | Me | CH₂ | Me | *c*-Pr | H | Me | H | O |
| D-0172 | H | CH(Me) | H | *c*-Pr | H | Me | H | O |
| D-0173 | H | CH(Me) | Me | *c*-Pr | H | Me | H | O |
| D-0174 | Me | CH(Me) | H | *c*-Pr | H | Me | H | O |
| D-0175 | Me | CH(Me) | Me | *c*-Pr | H | Me | H | O |
| D-0176 | H | CH₂CH₂ | Me | *c*-Pr | H | Me | H | O |
| D-0177 | Me | CH₂CH₂ | H | *c*-Pr | H | Me | H | O |
| D-0178 | Me | CH₂CH₂ | Me | *c*-Pr | H | Me | H | O |
| D-0179 | H | CH₂CH(Me) | H | *c*-Pr | H | Me | H | O |
| D-0180 | H | CH₂CH(Me) | Me | *c*-Pr | H | Me | H | O |
| D-0181 | Me | CH₂CH(Me) | H | *c*-Pr | H | Me | H | O |
| D-0182 | Me | CH₂CH(Me) | Me | *c*-Pr | H | Me | H | O |
| D-0183 | H | CH(Me)CH₂ | H | *c*-Pr | H | Me | H | O |
| D-0184 | H | CH(Me)CH₂ | Me | *c*-Pr | H | Me | H | O |
| D-0185 | Me | CH(Me)CH₂ | H | *c*-Pr | H | Me | H | O |
| D-0186 | Me | CH(Me)CH₂ | Me | *c*-Pr | H | Me | H | O |
| D-0187 | H | single bond | Me | *c*-Pr | H | Cl | Me | O |
| D-0188 | H | single bond | Et | c-Pr | H | Cl | Me | O |
| D-0189 | H | single bond | *i*-Pr | *c*-Pr | H | Cl | Me | O |
| D-0190 | Me | single bond | H | *c*-Pr | H | Cl | Me | O |
| D-0191 | Me (R) | single bond | H | *c*-Pr | H | Cl | Me | O |
| D-0192 | Me (S) | single bond | H | *c*-Pr | H | Cl | Me | O |
| D-0193 | Me | single bond | H | *c*-Pr | H | Cl | Me | S |
| D-0194 | Me(R) | single bond | H | *c*-Pr | H | Cl | Me | S |
| D-0195 | Me(S) | single bond | H | *c*-Pr | H | Cl | Me | S |
| D-0196 | Et | single bond | H | *c*-Pr | H | Cl | Me | O |
| D-0197 | *i*-Pr | single bond | H | *c*-Pr | H | Cl | Me | O |
| D-0198 | CF₃ | single bond | H | *c*-Pr | H | Cl | Me | O |
| D-0199 | Me | single bond | Me | *c*-Pr | H | Cl | Me | O |
| D-0200 | H | CH₂ | Me | *c*-Pr | H | Cl | Me | O |
| D-0201 | Me | CH₂ | H | *c*-Pr | H | Cl | Me | O |
| D-0202 | Me | CH₂ | Me | *c*-Pr | H | Cl | Me | O |
| D-0203 | H | CH(Me) | H | *c*-Pr | H | Cl | Me | O |
| D-0204 | H | CH(Me) | Me | *c*-Pr | H | Cl | Me | O |
| D-0205 | Me | CH(Me) | H | *c*-Pr | H | Cl | Me | O |
| D-0206 | Me | CH(Me) | Me | *c*-Pr | H | Cl | Me | O |
| D-0207 | H | CH₂CH₂ | Me | *c*-Pr | H | Cl | Me | O |
| D-0208 | Me | CH₂CH₂ | H | c-Pr | H | Cl | Me | O |
| D-0209 | Me | CH₂CH₂ | Me | *c*-Pr | H | Cl | Me | O |
| D-0210 | H | CH₂CH(Me) | H | *c*-Pr | H | Cl | Me | O |
| D-0211 | H | CH₂CH(Me) | Me | *c*-Pr | H | Cl | Me | O |
| D-0212 | Me | CH₂CH(Me) | H | *c*-Pr | H | Cl | Me | O |
| D-0213 | Me | CH₂CH(Me) | Me | *c*-Pr | H | Cl | Me | O |
| D-0214 | H | CH(Me)CH₂ | H | *c*-Pr | H | Cl | Me | O |

**[Table 650]**

| Compound Number | Y | W | Z | R¹ | R² | R³ | R¹⁰ | A |
|---|---|---|---|---|---|---|---|---|
| D-0215 | H | CH(Me)CH₂ | Me | *c*-Pr | H | Cl | Me | O |
| D-0216 | Me | CH(Me)CH₂ | H | *c*-Pr | H | Cl | Me | O |
| D-0217 | Me | CH(Me)CH₂ | Me | *c*-Pr | H | Cl | Me | O |
| D-0218 | H | single bond | Me | *c*-Pr | H | Me | Me | O |
| D-0219 | H | single bond | Et | *c*-Pr | H | Me | Me | O |
| D-0220 | H | single bond | *i*-Pr | *c*-Pr | H | Me | Me | O |
| D-0221 | Me | single bond | H | *c*-Pr | H | Me | Me | O |
| D-0222 | Me(R) | single bond | H | *c*-Pr | H | Me | Me | O |
| D-0223 | Me(S) | single bond | H | *c*-Pr | H | Me | Me | O |
| D-0224 | Me | single bond | H | *c*-Pr | H | Me | Me | S |
| D-0225 | Me(R) | single bond | H | *c*-Pr | H | Me | Me | S |
| D-0226 | Me(S) | single bond | H | *c*-Pr | H | Me | Me | S |
| D-0227 | Et | single bond | H | *c*-Pr | H | Me | Me | O |
| D-0228 | *i*-Pr | single bond | H | *c*-Pr | H | Me | Me | O |
| D-0229 | CF₃ | single bond | H | *c*-Pr | H | Me | Me | O |
| D-0230 | Me | single bond | Me | *c*-Pr | H | Me | Me | O |
| D-0231 | H | CH₂ | Me | *c*-Pr | H | Me | Me | O |
| D-0232 | Me | CH₂ | H | *c*-Pr | H | Me | Me | O |
| D-0233 | Me | CH₂ | Me | *c*-Pr | H | Me | Me | O |
| D-0234 | H | CH(Me) | H | *c*-Pr | H | Me | Me | O |
| D-0235 | H | CH(Me) | Me | *c*-Pr | H | Me | Me | O |
| D-0236 | Me | CH(Me) | H | *c*-Pr | H | Me | Me | O |
| D-0237 | Me | CH(Me) | Me | *c*-Pr | H | Me | Me | O |
| 0-0238 | H | CH₂CH₂ | Me | *c*-Pr | H | Me | Me | O |
| D-0239 | Me | CH₂CH₂ | H | *c*-Pr | H | Me | Me | O |
| D-0240 | Me | CH₂CH₂ | Me | *c*-Pr | H | Me | Me | O |
| D-0241 | H | CH₂CH(Me) | H | *c*-Pr | H | Me | Me | O |
| D-0242 | H | CH₂CH(Me) | Me | *c*-Pr | H | Me | Me | O |
| D-0243 | Me | CH₂CH(Me) | H | *c*-Pr | H | Me | Me | O |
| D-0244 | Me | CH₂CH(Me) | Me | *c*-Pr | H | Me | Me | O |
| 0-0245 | H | CH(Me)CH₂ | H | *c*-Pr | H | Me | Me | O |
| D-0246 | H | CH(Me)CH₂ | Me | *c*-Pr | H | Me | Me | O |
| D-0247 | Me | CH(Me)CH₂ | H | *c*-Pr | H | Me | Me | O |
| D-0248 | Me | CH(Me)CH₂ | Me | *c*-Pr | H | Me | Me | O |
| D-0249 | H | single bond | Me | *c*-Pr | H | Cl | Me | O |
| D-0250 | H | single bond | Et | *c*-Pr | H | Cl | Me | O |
| D-0251 | H | single bond | *i*-Pr | *c*-Pr | H | Cl | Me | O |
| D-0252 | Me | single bond | H | *c*-Pr | H | Cl | Me | O |
| D-0253 | Me(R) | single bond | H | *c*-Pr | H | Cl | Me | O |
| D-0254 | Me(S) | single bond | H | *c*-Pr | H | Cl | Me | O |
| D-0255 | Me | single bond | H | *c*-Pr | H | Cl | Me | S |
| D-0256 | Me(R) | single bond | H | *c*-Pr | H | Cl | Me | |
| D-0257 | Me(S) | single bond | H | *c*-Pr | H | Cl | Me | S |
| D-0258 | Et | single bond | H | *c*-Pr | H | Cl | Me | O |

**[Table 651]**

| Compound Number | Y | W | Z | R¹ | R² | R³ | R¹⁰ | A |
|---|---|---|---|---|---|---|---|---|
| D-0259 | *i*-Pr | single bond | H | *c*-Pr | H | Cl | Me | O |
| D-0260 | CF₃ | single bond | H | *c*-Pr | H | Cl | Me | O |
| D-0261 | Me | single bond | Me | *c*-Pr | H | Cl | Me | O |
| D-0262 | H | CH₂ | Me | *c*-Pr | H | Cl | Me | O |
| D-0263 | Me | CH₂ | H | *c*-Pr | H | Cl | Me | O |
| D-0264 | Me | CH₂ | Me | *c*-Pr | H | Cl | Me | O |
| D-0265 | H | CH(Me) | H | *c*-Pr | H | Cl | Me | O |
| D-0266 | H | CH(Me) | Me | *c*-Pr | H | Cl | Me | O |
| D-0267 | Me | CH(Me) | H | *c*-Pr | H | Cl | Me | O |
| D-0268 | Me | CH(Me) | Me | *c*-Pr | H | Cl | Me | O |
| 0-0269 | H | CH₂CH₂ | Me | *c*-Pr | H | Cl | Me | O |
| D-0270 | Me | CH₂CH₂ | H | *c*-Pr | H | Cl | Me | O |
| D-0271 | Me | CH₂CH₂ | Me | *c*-Pr | H | Cl | Me | O |
| D-0272 | H | CH₂CH(Me) | H | *c*-Pr | H | Cl | Me | O |
| D-0273 | H | CH₂CH(Me) | Me | *c*-Pr | H | Cl | Me | O |
| 0-0274 | Me | CH₂CH(Me) | H | *c*-Pr | H | Cl | Me | O |
| D-0275 | Me | CH₂CH(Me) | Me | *c*-Pr | H | Cl | Me | O |
| D-0276 | H | CH(Me)CH₂ | H | c-Pr | H | Cl | Me | O |
| D-0277 | H | CH(Me)CH₂ | Me | *c*-Pr | H | Cl | Me | O |
| D-0278 | Me | CH(Me)CH₂ | H | *c*-Pr | H | Cl | Me | O |
| D-0279 | Me | CH(Me)CH₂ | Me | *c*-Pr | H | Cl | Me | O |
| D-0280 | H | single bond | Me | *c*-Pr | H | Me | Me | O |
| D-0281 | H | single bond | Et | *c*-Pr | H | Me | Me | O |
| D-0282 | H | single bond | *i*-Pr | *c*-Pr | H | Me | Me | O |
| D-0283 | Me | single bond | H | *c*-Pr | H | Me | Me | O |
| D-0284 | Me(R) | single bond | H | *c*-Pr | H | Me | Me | O |
| D-0285 | Me(S) | single bond | H | *c*-Pr | H | Me | Me | O |
| D-0286 | Me | single bond | H | *c*-Pr | H | Me | Me | S |
| D-0287 | Me(R) | single bond | H | *c*-Pr | H | Me | Me | S |
| D-0288 | Me(S) | single bond | H | *c*-Pr | H | Me | Me | S |
| D-0289 | Et | single bond | H | *c*-Pr | H | Me | Me | O |
| D-0290 | *i*-Pr | single bond | H | *c*-Pr | H | Me | Me | O |
| D-0291 | CF₃ | single bond | H | *c*-Pr | H | Me | Me | O |
| D-0292 | Me | single bond | Me | *c*-Pr | H | Me | Me | O |
| D-0293 | H | CH₂ | Me | *c*-Pr | H | Me | Me | O |
| D-0294 | Me | CH₂ | H | *c*-Pr | H | Me | Me | O |
| D-0295 | Me | CH₂ | Me | *c*-Pr | H | Me | Me | O |
| D-0296 | H | CH(Me) | H | c-Pr | H | Me | Me | O |
| D-0297 | H | CH(Me) | Me | *c*-Pr | H | Me | Me | O |
| D-0298 | Me | CH(Me) | H | *c*-Pr | H | Me | Me | O |
| D-0299 | Me | CH(Me) | Me | *c*-Pr | H | Me | Me | O |
| D-0300 | H | CH₂CH₂ | Me | *c*-Pr | H | Me | Me | O |
| D-0301 | Me | CH₂CH₂ | H | *c*-Pr | H | Me | Me | O |
| D-0302 | Me | CH₂CH₂ | Me | *c*-Pr | H | Me | Me | O |

**[Table 652]**

| Compound Number | Y | W | Z | R¹ | R² | R³ | R¹⁰ | A |
|---|---|---|---|---|---|---|---|---|
| D-0303 | H | CH₂CH(Me) | H | *c*-Pr | H | Me | Me | O |
| 0-0304 | H | CH₂CH(Me) | Me | *c*-Pr | H | Me | Me | O |
| 0-0305 | Me | CH₂CH(Me) | H | *c*-Pr | H | Me | Me | O |
| D-0306 | Me | CH₂CH(Me) | Me | *c*-Pr | H | Me | Me | O |
| 0-0307 | H | CH(Me)CH₂ | H | *c*-Pr | H | Me | Me | O |
| 0-0308 | H | CH(Me)CH₂ | Me | *c*-Pr | H | Me | Me | O |
| D-0309 | Me | CH(Me)CH₂ | H | *c*-Pr | H | Me | Me | O |
| D-0310 | Me | CH(Me)CH₂ | Me | *c*-Pr | H | Me | Me | O |
| D-0311 | H | single bond | Me | *c*-Pr | H | Cl | Me | O |
| D-0312 | H | single bond | Et | *c*-Pr | H | Cl | Me | O |
| D-0313 | H | single bond | *i*-Pr | *c*-Pr | H | Cl | Me | O |
| D-0314 | Me | single bond | H | *c*-Pr | H | Cl | Me | O |
| D-0315 | Me(R) | single bond | H | *c*-Pr | H | Cl | Me | O |
| D-0316 | Me(S) | single bond | H | *c*-Pr | H | Cl | Me | O |
| D-0317 | Me | single bond | H | *c*-Pr | H | Cl | Me | S |
| D-0318 | Me(R) | single bond | H | *c*-Pr | H | Cl | Me | S |
| D-0319 | Me(S) | single bond | H | *c*-Pr | H | Cl | Me | S |
| D-0320 | Et | single bond | H | c-Pr | H | Cl | Me | O |
| D-0321 | *i*-Pr | single bond | H | *c*-Pr | H | Cl | Me | O |
| D-0322 | CF₃ | single bond | H | *c*-Pr | H | Cl | Me | O |
| D-0323 | Me | single bond | Me | *c*-Pr | H | Cl | Me | O |
| D-0324 | H | CH₂ | Me | *c*-Pr | H | Cl | Me | O |
| D-0325 | Me | CH₂ | H | *c*-Pr | H | Cl | Me | O |
| D-0326 | Me | CH₂ | Me | *c*-Pr | H | Cl | Me | O |
| D-0327 | H | CH(Me) | H | *c*-Pr | H | Cl | Me | O |
| D-0328 | H | CH(Me) | Me | *c*-Pr | H | Cl | Me | O |
| D-0329 | Me | CH(Me) | H | *c*-Pr | H | Cl | Me | O |
| D-0330 | Me | CH(Me) | Me | *c*-Pr | H | Cl | Me | O |
| D-0331 | H | CH₂CH₂ | Me | *c*-Pr | H | Cl | Me | O |
| D-0332 | Me | CH₂CH₂ | H | *c*-Pr | H | Cl | Me | O |
| D-0333 | Me | CH₂CH₂ | Me | *c*-Pr | H | Cl | Me | O |
| D-0334 | H | CH₂CH(Me) | H | *c*-Pr | H | Cl | Me | O |
| D-0335 | H | CH₂CH(Me) | Me | *c*-Pr | H | Cl | Me | O |
| D-0336 | Me | CH₂CH(Me) | H | *c*-Pr | H | Cl | Me | O |
| D-0337 | Me | CH₂CH(Me) | Me | *c*-Pr | H | Cl | Me | O |
| D-0338 | H | CH(Me)CH₂ | H | *c*-Pr | H | Cl | Me | O |
| D-0339 | H | CH(Me)CH₂ | Me | *c*-Pr | H | Cl | Me | O |
| D-0340 | Me | CH(Me)CH₂ | H | c-Pr | H | Cl | Me | O |
| D-0341 | Me | CH(Me)CH₂ | Me | *c*-Pr | H | Cl | Me | O |
| D-0342 | H | single bond | Me | *c*-Pr | H | Me | Me | O |
| D-0343 | H | single bond | Et | *c*-Pr | H | Me | Me | O |
| D-0344 | H | single bond | *i*-Pr | *c*-Pr | H | Me | Me | O |
| D-0345 | Me | single bond | H | *c*-Pr | H | Me | Me | O |
| D-0346 | Me(R) | single bond | H | *c*-Pr | H | Me | Me | O |

**[Table 653]**

| Compound Number | Y | W | Z | R¹ | R² | R³ | R¹⁰ | A |
|---|---|---|---|---|---|---|---|---|
| D-0347 | Me(S) | single bond | H | *c*-Pr | H | Me | Me | O |
| D-0348 | Me | single bond | H | *c*-Pr | H | Me | Me | S |
| D-0349 | Me(R) | single bond | H | *c*-Pr | H | Me | Me | S |
| D-0350 | Me(S) | single bond | H | *c*-Pr | H | Me | Me | S |
| D-0351 | Et | single bond | H | *c*-Pr | H | Me | Me | O |
| D-0352 | *i*-Pr | single bond | H | *c*-Pr | H | Me | Me | O |
| D-0353 | CF₃ | single bond | H | *c*-Pr | H | Me | Me | O |
| D-0354 | Me | single bond | Me | *c*-Pr | H | Me | Me | O |
| 0-0355 | H | CH₂ | Me | *c*-Pr | H | Me | Me | O |
| D-0356 | Me | CH₂ | H | *c*-Pr | H | Me | Me | O |
| D-0357 | Me | CH₂ | Me | *c*-Pr | H | Me | Me | O |
| D-0358 | H | CH(Me) | H | *c*-Pr | H | Me | Me | O |
| 0-0359 | H | CH(Me) | Me | *c*-Pr | H | Me | Me | O |
| D-0360 | Me | CH(Me) | H | *c*-Pr | H | Me | Me | O |
| D-0361 | Me | CH(Me) | Me | *c*-Pr | H | Me | Me | O |
| D-0362 | H | CH₂CH₂ | Me | *c*-Pr | H | Me | Me | O |
| D-0363 | Me | CH₂CH₂ | H | *c*-Pr | H | Me | Me | O |
| D-0364 | Me | CH₂CH₂ | Me | *c*-Pr | H | Me | Me | O |
| D-0365 | H | CH₂CH(Me) | H | *c*-Pr | H | Me | Me | O |
| D-0366 | H | CH₂CH(Me) | Me | *c*-Pr | H | Me | Me | O |
| D-0367 | Me | CH₂CH(Me) | H | *c*-Pr | H | Me | Me | O |
| D-0368 | Me | CH₂CH(Me) | Me | *c*-Pr | H | Me | Me | O |
| D-0369 | H | CH(Me)CH₂ | H | *c*-Pr | H | Me | Me | O |
| D-0370 | H | CH(Me)CH₂ | Me | *c*-Pr | H | Me | Me | O |
| D-0371 | Me | CH(Me)CH₂ | H | *c*-Pr | H | Me | Me | O |
| D-0372 | Me | CH(Me)CH₂ | Me | *c*-Pr | H | Me | Me | O |
| D-0373 | H | single bond | Me | *c*-Pr | H | Cl | Et | O |
| D-0374 | Me | single bond | H | *c*-Pr | H | Cl | Et | O |
| D-0375 | Me(R) | single bond | H | *c*-Pr | H | Cl | Et | O |
| D-0376 | Me(S) | single bond | H | *c*-Pr | H | Cl | Et | O |
| D-0377 | Me | single bond | H | *c*-Pr | H | Cl | Et | S |
| D-0378 | Me(R) | single bond | H | *c*-Pr | H | Cl | Et | S |
| D-0379 | Me(S) | single bond | H | *c*-Pr | H | Cl | Et | S |
| D-0380 | Me | single bond | Me | *c*-Pr | H | Cl | Et | O |
| D-0381 | H | CH₂ | Me | *c*-Pr | H | Cl | Et | O |
| D-0382 | Me | CH₂ | H | *c*-Pr | H | Cl | Et | O |
| D-0383 | Me | CH₂ | Me | *c*-Pr | H | Cl | Et | O |
| D-0384 | H | CH(Me) | H | *c*-Pr | H | Cl | Et | O |
| D-0385 | H | CH(Me) | Me | *c*-Pr | H | Cl | Et | O |
| D-0386 | Me | CH(Me) | H | *c*-Pr | H | Cl | Et | O |
| D-0387 | Me | CH(Me) | Me | *c*-Pr | H | Cl | Et | O |
| D-0388 | H | CH₂CH₂ | Me | *c*-Pr | H | Cl | Et | O |
| D-0389 | Me | CH₂CH₂ | H | *c*-Pr | H | Cl | Et | O |
| D-0390 | Me | CH₂CH₂ | Me | *c*-Pr | H | Cl | Et | O |

**[Table 654]**

| Compound Number | Y | W | Z | R¹ | R² | R³ | R¹⁰ | A |
|---|---|---|---|---|---|---|---|---|
| D-0391 | H | single bond | Me | *c*-Pr | H | Me | Et | O |
| D-0392 | Me | single bond | H | *c*-Pr | H | Me | Et | O |
| D-0393 | Me(R) | single bond | H | *c*-Pr | H | Me | Et | O |
| D-0394 | Me(S) | single bond | H | *c*-Pr | H | Me | Et | O |
| D-0395 | Me | single bond | H | *c*-Pr | H | Me | Et | S |
| D-0396 | Me(R) | single bond | H | *c*-Pr | H | Me | Et | S |
| D-0397 | Me(S) | single bond | H | *c*-Pr | H | Me | Et | S |
| D-0398 | Me | single bond | Me | *c*-Pr | H | Me | Et | O |
| D-0399 | H | CH₂ | Me | *c*-Pr | H | Me | Et | O |
| D-0400 | Me | CH₂ | H | *c*-Pr | H | Me | Et | O |
| D-0401 | Me | CH₂ | Me | *c*-Pr | H | Me | Et | O |
| D-0402 | H | CH(Me) | H | *c*-Pr | H | Me | Et | O |
| D-0403 | H | CH(Me) | Me | *c*-Pr | H | Me | Et | O |
| D-0404 | Me | CH(Me) | H | *c*-Pr | H | Me | Et | O |
| D-0405 | Me | CH(Me) | Me | *c*-Pr | H | Me | Et | O |
| D-0406 | H | CH₂CH₂ | Me | *c*-Pr | H | Me | Et | O |
| D-0407 | Me | CH₂CH₂ | H | *c*-Pr | H | Me | Et | O |
| D-0408 | Me | CH₂CH₂ | Me | *c*-Pr | H | Me | Et | O |
| D-0409 | H | single bond | Me | *c*-Pr | H | Cl | Et | O |
| D-0410 | Me | single bond | H | *c*-Pr | H | Cl | Et | O |
| D-0411 | Me(R) | single bond | H | *c*-Pr | H | Cl | Et | O |
| D-0412 | Me(S) | single bond | H | *c*-Pr | H | Cl | Et | O |
| D-0413 | Me | single bond | H | *c*-Pr | H | Cl | Et | S |
| D-0414 | Me(R) | single bond | H | *c*-Pr | H | Cl | Et | S |
| D-0415 | Me(S) | single bond | H | *c*-Pr | H | Cl | Et | S |
| D-0416 | Me | single bond | Me | *c*-Pr | H | Cl | Et | O |
| D-0417 | H | CH₂ | Me | *c*-Pr | H | Cl | Et | O |
| D-0418 | Me | CH₂ | H | *c*-Pr | H | Cl | Et | O |
| D-0419 | Me | CH₂ | Me | *c*-Pr | H | Cl | Et | O |
| D-0420 | H | CH(Me) | H | *c*-Pr | H | Cl | Et | O |
| D-0421 | H | CH(Me) | Me | *c*-Pr | H | Cl | Et | O |
| D-0422 | Me | CH(Me) | H | *c*-Pr | H | Cl | Et | O |
| D-0423 | Me | CH(Me) | Me | *c*-Pr | H | Cl | Et | O |
| D-0424 | H | CH₂CH₂ | Me | *c*-Pr | H | Cl | Et | O |
| D-0425 | Me | CH₂CH₂ | H | *c*-Pr | H | Cl | Et | O |
| D-0426 | Me | CH₂CH₂ | Me | *c*-Pr | H | Cl | Et | O |
| D-0427 | H | single bond | Me | *c*-Pr | H | Me | Et | O |
| D-0428 | Me | single bond | H | *c*-Pr | H | Me | Et | O |
| D-0429 | Me(R) | single bond | H | *c*-Pr | H | Me | Et | O |
| D-0430 | Me(S) | single bond | H | *c*-Pr | H | Me | Et | O |
| D-0431 | Me | single bond | H | *c*-Pr | H | Me | Et | S |
| D-0432 | Me(R) | single bond | H | *c*-Pr | H | Me | Et | S |
| D-0433 | Me(S) | single bond | H | *c*-Pr | H | Me | Et | S |
| D-0434 | Me | single bond | Me | *c*-Pr | H | Me | Et | O |

**[Table 655]**

| Compound Number | Y | W | Z | R¹ | R² | R³ | R¹⁰ | A |
|---|---|---|---|---|---|---|---|---|
| D-0435 | H | CH₂ | Me | *c*-Pr | H | Me | Et | O |
| D-0436 | Me | CH₂ | H | *c*-Pr | H | Me | Et | O |
| D-0437 | Me | CH₂ | Me | *c*-Pr | H | Me | Et | O |
| D-0438 | H | CH(Me) | H | *c*-Pr | H | Me | Et | O |
| D-0439 | H | CH(Me) | Me | *c*-Pr | H | Me | Et | O |
| D-0440 | Me | CH(Me) | H | *c*-Pr | H | Me | Et | O |
| D-0441 | Me | CH(Me) | Me | *c*-Pr | H | Me | Et | O |
| D-0442 | H | CH₂CH₂ | Me | *c*-Pr | H | Me | Et | O |
| D-0443 | Me | CH₂CH₂ | H | *c*-Pr | H | Me | Et | O |
| D-0444 | Me | CH₂CH₂ | Me | *c*-Pr | H | Me | Et | O |
| D-0445 | H | single bond | Me | *c*-Pr | H | Cl | Et | O |
| D-0446 | Me | single bond | H | *c*-Pr | H | Cl | Et | O |
| D-0447 | Me(R) | single bond | H | *c*-Pr | H | Cl | Et | O |
| D-0448 | Me(S) | single bond | H | *c*-Pr | H | Cl | Et | O |
| D-0449 | Me | single bond | H | *c*-Pr | H | Cl | Et | S |
| D-0450 | Me(R) | single bond | H | *c*-Pr | H | Cl | Et | S |
| D-0451 | Me(S) | single bond | H | *c*-Pr | H | Cl | Et | S |
| D-0452 | Me | single bond | Me | *c*-Pr | H | Cl | Et | O |
| D-0453 | H | CH₂ | Me | *c*-Pr | H | Cl | Et | O |
| D-0454 | Me | CH₂ | H | *c*-Pr | H | Cl | Et | O |
| D-0455 | Me | CH₂ | Me | *c*-Pr | H | Cl | Et | O |
| D-0456 | H | CH(Me) | H | *c*-Pr | H | Cl | Et | O |
| D-0457 | H | CH(Me) | Me | *c*-Pr | H | Cl | Et | O |
| D-0458 | Me | CH(Me) | H | *c*-Pr | H | Cl | Et | O |
| D-0459 | Me | CH(Me) | Me | *c*-Pr | H | Cl | Et | O |
| D-0460 | H | CH₂CH₂ | Me | *c*-Pr | H | Cl | Et | O |
| D-0461 | Me | CH₂CH₂ | H | *c*-Pr | H | Cl | Et | O |
| D-0462 | Me | CH₂CH₂ | Me | *c*-Pr | H | Cl | Et | O |
| D-0463 | H | single bond | Me | *c*-Pr | H | Me | Et | O |
| D-0464 | Me | single bond | H | *c*-Pr | H | Me | Et | O |
| D-0465 | Me(R) | single bond | H | *c*-Pr | H | Me | Et | O |
| D-0466 | Me(S) | single bond | H | *c*-Pr | H | Me | Et | O |
| D-0467 | Me | single bond | H | *c*-Pr | H | Me | Et | S |
| D-0468 | Me(R) | single bond | H | *c*-Pr | H | Me | Et | S |
| D-0469 | Me(S) | single bond | H | *c*-Pr | H | Me | Et | S |
| D-0470 | Me | single bond | Me | *c*-Pr | H | Me | Et | O |
| D-0471 | H | CH₂ | Me | *c*-Pr | H | Me | Et | O |
| D-0472 | Me | CH₂ | H | *c*-Pr | H | Me | Et | O |
| D-0473 | Me | CH₂ | Me | *c*-Pr | H | Me | Et | O |
| D-0474 | H | CH(Me) | H | *c*-Pr | H | Me | Et | O |
| D-0475 | H | CH(Me) | Me | *c*-Pr | H | Me | Et | O |
| D-0476 | Me | CH(Me) | H | *c*-Pr | H | Me | Et | O |
| D-0477 | Me | CH(Me) | Me | *c*-Pr | H | Me | Et | O |
| D-0478 | H | CH₂CH₂ | Me | *c*-Pr | H | Me | Et | O |

**[Table 656]**

| Compound Number | Y | W | Z | R¹ | R² | R³ | R¹⁰ | A |
|---|---|---|---|---|---|---|---|---|
| D-0479 | Me | CH₂CH₂ | H | *c*-Pr | H | Me | Et | O |
| D-0480 | Me | CH₂CH₂ | Me | *c*-Pr | H | Me | Et | O |
| D-0481 | H | single bond | Me | *c*-Pr | H | Cl | CH₂CF₃ | O |
| D-0482 | Me | single bond | H | *c*-Pr | H | Cl | CH₂CF₃ | O |
| D-0483 | Me(R) | single bond | H | *c*-Pr | H | Cl | CH₂CF₃ | O |
| D-0484 | Me(S) | single bond | H | *c*-Pr | H | Cl | CH₂CF₃ | O |
| D-0485 | Me | single bond | H | *c*-Pr | H | Cl | CH₂CF₃ | S |
| D-0486 | Me(R) | single bond | H | *c*-Pr | H | Cl | CH₂CF₃ | S |
| D-0487 | Me(S) | single bond | H | *c*-Pr | H | Cl | CH₂CF₃ | S |
| D-0488 | Me | single bond | Me | *c*-Pr | H | Cl | CH₂CF₃ | O |
| D-0489 | H | CH₂ | Me | *c*-Pr | H | Cl | CH₂CF₃ | O |
| D-0490 | Me | CH₂ | H | *c*-Pr | H | Cl | CH₂CF₃ | O |
| D-0491 | Me | CH₂ | Me | *c*-Pr | H | Cl | CH₂CF₃ | O |
| D-0492 | H | CH(Me) | H | *c*-Pr | H | Cl | CH₂CF₃ | O |
| D-0493 | H | CH(Me) | Me | *c*-Pr | H | Cl | CH₂CF₃ | O |
| D-0494 | Me | CH(Me) | H | *c*-Pr | H | Cl | CH₂CF₃ | O |
| D-0495 | Me | CH(Me) | Me | *c*-Pr | H | Cl | CH₂CF₃ | O |
| D-0496 | H | CH₂CH₂ | Me | *c*-Pr | H | Cl | CH₂CF₃ | O |
| D-0497 | Me | CH₂CH₂ | H | *c*-Pr | H | Cl | CH₂CF₃ | O |
| D-0498 | Me | CH₂CH₂ | Me | *c*-Pr | H | Cl | CH₂CF₃ | O |
| D-0499 | H | single bond | Me | *c*-Pr | H | Me | CH₂CF₃ | O |
| D-0500 | Me | single bond | H | *c*-Pr | H | Me | CH₂CF₃ | O |
| D-0501 | Me(R) | single bond | H | *c*-Pr | H | Me | CH₂CF₃ | O |
| D-0502 | Me(S) | single bond | H | *c*-Pr | H | Me | CH₂CF₃ | O |
| D-0503 | Me | single bond | H | *c*-Pr | H | Me | CH₂CF₃ | S |
| D-0504 | Me(R) | single bond | H | *c*-Pr | H | Me | CH₂CF₃ | S |
| D-0505 | Me(S) | single bond | H | *c*-Pr | H | Me | CH₂CF₃ | S |
| D-0506 | Me | single bond | Me | *c*-Pr | H | Me | CH₂CF₃ | O |
| D-0507 | H | CH₂ | Me | *c*-Pr | H | Me | CH₂CF₃ | O |
| D-0508 | Me | CH₂ | H | *c*-Pr | H | Me | CH₂CF₃ | O |
| D-0509 | Me | CH₂ | Me | *c*-Pr | H | Me | CH₂CF₃ | O |
| D-0510 | H | CH(Me) | H | *c*-Pr | H | Me | CH₂CF₃ | O |
| D-0511 | H | CH(Me) | Me | *c*-Pr | H | Me | CH₂CF₃ | O |
| D-0512 | Me | CH(Me) | H | *c*-Pr | H | Me | CH₂CF₃ | O |
| D-0513 | Me | CH(Me) | Me | *c*-Pr | H | Me | CH₂CF₃ | O |
| D-0514 | H | CH₂CH₂ | Me | *c*-Pr | H | Me | CH₂CF₃ | O |
| D-0515 | Me | CH₂CH₂ | H | *c*-Pr | H | Me | CH₂CF₃ | O |
| D-0516 | Me | CH₂CH₂ | Me | *c*-Pr | H | Me | CH₂CF₃ | O |
| D-0517 | H | single bond | Me | *c*-Pr | H | Cl | CH₂CF₃ | O |
| D-0518 | Me | single bond | H | *c*-Pr | H | Cl | CH₂CF₃ | O |
| D-0519 | Me(R) | single bond | H | *c*-Pr | H | Cl | CH₂CF₃ | O |
| D-0520 | Me(S) | single bond | H | *c*-Pr | H | Cl | CH₂CF₃ | O |
| D-0521 | Me | single bond | H | *c*-Pr | H | Cl | CH₂CF₃ | S |
| D-0522 | Me(R) | single bond | H | *c*-Pr | H | Cl | CH₂CF₃ | S |

**[Table 657]**

| Compound Number | Y | W | Z | R¹ | R² | R³ | R¹⁰ | A |
|---|---|---|---|---|---|---|---|---|
| D-0523 | Me(S) | single bond | H | *c*-Pr | H | Cl | CH₂CF₃ | S |
| D-0524 | Me | single bond | Me | *c*-Pr | H | Cl | CH₂CF₃ | O |
| D-0525 | H | CH₂ | Me | *c*-Pr | H | Cl | CH₂CF₃ | O |
| D-0526 | Me | CH₂ | H | *c*-Pr | H | Cl | CH₂CF₃ | O |
| D-0527 | Me | CH₂ | Me | *c*-Pr | H | Cl | CH₂CF₃ | O |
| D-0528 | H | CH(Me) | H | *c*-Pr | H | Cl | CH₂CF₃ | O |
| D-0529 | H | CH(Me) | Me | *c*-Pr | H | Cl | CH₂CF₃ | O |
| D-0530 | Me | CH(Me) | H | *c*-Pr | H | Cl | CH₂CF₃ | O |
| D-0531 | Me | CH(Me) | Me | *c*-Pr | H | Cl | CH₂CF₃ | O |
| D-0532 | H | CH₂CH₂ | Me | *c*-Pr | H | Cl | CH₂CF₃ | O |
| D-0533 | Me | CH₂CH₂ | H | *c*-Pr | H | Cl | CH₂CF₃ | O |
| D-0534 | Me | CH₂CH₂ | Me | *c*-Pr | H | Cl | CH₂CF₃ | O |
| D-0535 | H | single bond | Me | *c*-Pr | H | Me | CH₂CF₃ | O |
| D-0536 | Me | single bond | H | *c*-Pr | H | Me | CH₂CF₃ | O |
| D-0537 | Me(R) | single bond | H | *c*-Pr | H | Me | CH₂CF₃ | O |
| D-0538 | Me(S) | single bond | H | *c*-Pr | H | Me | CH₂CF₃ | O |
| D-0539 | Me | single bond | H | *c*-Pr | H | Me | CH₂CF₃ | S |
| D-0540 | Me(R) | single bond | H | *c*-Pr | H | Me | CH₂CF₃ | S |
| D-0541 | Me(S) | single bond | H | *c*-Pr | H | Me | CH₂CF₃ | S |
| D-0542 | Me | single bond | Me | *c*-Pr | H | Me | CH₂CF₃ | O |
| D-0543 | H | CH₂ | Me | *c*-Pr | H | Me | CH₂CF₃ | O |
| D-0544 | Me | CH₂ | H | *c*-Pr | H | Me | CH₂CF₃ | O |
| D-0545 | Me | CH₂ | Me | *c*-Pr | H | Me | CH₂CF₃ | O |
| D-0546 | H | CH(Me) | H | *c*-Pr | H | Me | CH₂CF₃ | O |
| D-0547 | H | CH(Me) | Me | *c*-Pr | H | Me | CH₂CF₃ | O |
| D-0548 | Me | CH(Me) | H | *c*-Pr | H | Me | CH₂CF₃ | O |
| D-0549 | Me | CH(Me) | Me | *c*-Pr | H | Me | CH₂CF₃ | O |
| D-0550 | H | CH₂CH₂ | Me | *c*-Pr | H | Me | CH₂CF₃ | O |
| D-0551 | Me | CH₂CH₂ | H | *c*-Pr | H | Me | CH₂CF₃ | O |
| D-0552 | Me | CH₂CH₂ | Me | *c*-Pr | H | Me | CH₂CF₃ | O |
| D-0553 | H | single bond | Me | *c*-Pr | H | Cl | CH₂CF₃ | O |
| D-0554 | Me | single bond | H | *c*-Pr | H | Cl | CH₂CF₃ | O |
| D-0555 | Me(R) | single bond | H | *c*-Pr | H | Cl | CH₂CF₃ | O |
| D-0556 | Me(S) | single bond | H | *c*-Pr | H | Cl | CH₂CF₃ | O |
| D-0557 | Me | single bond | H | *c*-Pr | H | Cl | CH₂CF₃ | S |
| D-0558 | Me(R) | single bond | H | *c*-Pr | H | Cl | CH₂CF₃ | S |
| D-0559 | Me(S) | single bond | H | *c*-Pr | H | Cl | CH₂CF₃ | S |
| D-0560 | Me | single bond | Me | *c*-Pr | H | Cl | CH₂CF₃ | O |
| D-0561 | H | CH₂ | Me | *c*-Pr | H | Cl | CH₂CF₃ | O |
| D-0562 | Me | CH₂ | H | *c*-Pr | H | Cl | CH₂CF₃ | O |
| D-0563 | Me | CH₂ | Me | *c*-Pr | H | Cl | CH₂CF₃ | O |
| D-0564 | H | CH(Me) | H | *c*-Pr | H | Cl | CH₂CF₃ | O |
| D-0565 | H | CH(Me) | Me | *c*-Pr | H | Cl | CH₂CF₃ | O |
| D-0566 | Me | CH(Me) | H | *c*-Pr | H | Cl | CH₂CF₃ | O |

**[Table 658]**

| Compound Number | Y | W | Z | R¹ | R² | R³ | R¹⁰ | A |
|---|---|---|---|---|---|---|---|---|
| D-0567 | Me | CH(Me) | Me | *c*-Pr | H | Cl | CH₂CF₃ | O |
| D-0568 | H | CH₂CH₂ | Me | *c*-Pr | H | Cl | CH₂CF₃ | O |
| D-0569 | Me | CH₂CH₂ | H | *c*-Pr | H | Cl | CH₂CF₃ | O |
| D-0570 | Me | CH₂CH₂ | Me | *c*-Pr | H | Cl | CH₂CF₃ | O |
| D-0571 | H | single bond | Me | *c*-Pr | H | Me | CH₂CF₃ | O |
| D-0572 | Me | single bond | H | *c*-Pr | H | Me | CH₂CF₃ | O |
| D-0573 | Me(R) | single bond | H | *c*-Pr | H | Me | CH₂CF₃ | O |
| D-0574 | Me(S) | single bond | H | *c*-Pr | H | Me | CH₂CF₃ | O |
| D-0575 | Me | single bond | H | *c*-Pr | H | Me | CH₂CF₃ | S |
| D-0576 | Me(R) | single bond | H | *c*-Pr | H | Me | CH₂CF₃ | S |
| D-0577 | Me(S) | single bond | H | *c*-Pr | H | Me | CH₂CF₃ | S |
| D-0578 | Me | single bond | Me | *c*-Pr | H | Me | CH₂CF₃ | O |
| D-0579 | H | CH₂ | Me | *c*-Pr | H | Me | CH₂CF₃ | O |
| D-0580 | Me | CH₂ | H | *c*-Pr | H | Me | CH₂CF₃ | O |
| D-0581 | Me | CH₂ | Me | *c*-Pr | H | Me | CH₂CF₃ | O |
| D-0582 | H | CH(Me) | H | *c*-Pr | H | Me | CH₂CF₃ | O |
| D-0583 | H | CH(Me) | Me | *c*-Pr | H | Me | CH₂CF₃ | O |
| D-0584 | Me | CH(Me) | H | *c*-Pr | H | Me | CH₂CF₃ | O |
| D-0585 | Me | CH(Me) | Me | *c*-Pr | H | Me | CH₂CF₃ | O |
| D-0586 | H | CH₂CH₂ | Me | *c*-Pr | H | Me | CH₂CF₃ | O |
| D-0587 | Me | CH₂CH₂ | H | *c*-Pr | H | Me | CH₂CF₃ | O |
| D-0588 | Me | CH₂CH₂ | Me | *c*-Pr | H | Me | CH₂CF₃ | O |
| D-0589 | H | single bond | Me | *c*-Pr | H | Cl | CH₂OMe | O |
| D-0590 | Me | single bond | H | *c*-Pr | H | Cl | CH₂OMe | O |
| D-0591 | Me(R) | single bond | H | *c*-Pr | H | Cl | CH₂OMe | O |
| D-0592 | Me(S) | single bond | H | *c*-Pr | H | Cl | CH₂OMe | O |
| D-0593 | Me | single bond | H | *c*-Pr | H | Cl | CH₂OMe | S |
| D-0594 | Me(R) | single bond | H | *c*-Pr | H | Cl | CH₂OMe | S |
| D-0595 | Me(S) | single bond | H | *c*-Pr | H | Cl | CH₂OMe | S |
| D-0596 | Me | single bond | Me | *c*-Pr | H | Cl | CH₂OMe | O |
| D-0597 | H | CH₂ | Me | *c*-Pr | H | Cl | CH₂OMe | O |
| D-0598 | Me | CH₂ | H | *c*-Pr | H | Cl | CH₂OMe | O |
| D-0599 | Me | CH₂ | Me | *c*-Pr | H | Cl | CH₂OMe | O |
| D-0600 | H | CH(Me) | H | *c*-Pr | H | Cl | CH₂OMe | O |
| D-0601 | H | CH(Me) | Me | *c*-Pr | H | Cl | CH₂OMe | O |
| D-0602 | Me | CH(Me) | H | *c*-Pr | H | Cl | CH₂OMe | O |
| D-0603 | Me | CH(Me) | Me | *c*-Pr | H | Cl | CH₂OMe | O |
| D-0604 | H | CH₂CH₂ | Me | *c*-Pr | H | Cl | CH₂OMe | O |
| D-0605 | Me | CH₂CH₂ | H | *c*-Pr | H | Cl | CH₂OMe | O |
| D-0606 | Me | CH₂CH₂ | Me | *c*-Pr | H | Cl | CH₂OMe | O |
| D-0607 | H | single bond | Me | *c*-Pr | H | Me | CH₂OMe | O |
| D-0608 | Me | single bond | H | *c*-Pr | H | Me | CH₂OMe | O |
| D-0609 | Me(R) | single bond | H | *c*-Pr | H | Me | CH₂OMe | O |
| D-0610 | Me(S) | single bond | H | *c*-Pr | H | Me | CH₂OMe | O |

**[Table 659]**

| Compound Number | Y | W | Z | R¹ | R² | R³ | R¹⁰ | A |
|---|---|---|---|---|---|---|---|---|
| D-0611 | Me | single bond | H | *c*-Pr | H | Me | CH₂OMe | S |
| D-0612 | Me(R) | single bond | H | *c*-Pr | H | Me | CH₂OMe | S |
| D-0613 | Me(S) | single bond | H | *c*-Pr | H | Me | CH₂OMe | S |
| D-0614 | Me | single bond | Me | *c*-Pr | H | Me | CH₂OMe | O |
| D-0615 | H | CH₂ | Me | *c*-Pr | H | Me | CH₂OMe | O |
| D-0616 | Me | CH₂ | H | *c*-Pr | H | Me | CH₂OMe | O |
| D-0617 | Me | CH₂ | Me | *c*-Pr | H | Me | CH₂OMe | O |
| D-0618 | H | CH(Me) | H | *c*-Pr | H | Me | CH₂OMe | O |
| D-0619 | H | CH(Me) | Me | *c*-Pr | H | Me | CH₂OMe | O |
| D-0620 | Me | CH(Me) | H | *c*-Pr | H | Me | CH₂OMe | O |
| D-0621 | Me | CH(Me) | Me | *c*-Pr | H | Me | CH₂OMe | O |
| D-0622 | H | CH₂CH₂ | Me | *c*-Pr | H | Me | CH₂OMe | O |
| D-0623 | Me | CH₂CH₂ | H | *c*-Pr | H | Me | CH₂OMe | O |
| D-0624 | Me | CH₂CH₂ | Me | *c*-Pr | H | Me | CH₂OMe | O |
| D-0625 | H | single bond | Me | *c*-Pr | H | Cl | CH₂OMe | O |
| D-0626 | Me | single bond | H | *c*-Pr | H | Cl | CH₂OMe | O |
| D-0627 | Me(R) | single bond | H | *c*-Pr | H | Cl | CH₂OMe | O |
| D-0628 | Me(S) | single bond | H | *c*-Pr | H | Cl | CH₂OMe | O |
| D-0629 | Me | single bond | H | *c*-Pr | H | Cl | CH₂OMe | S |
| D-0630 | Me(R) | single bond | H | *c*-Pr | H | Cl | CH₂OMe | S |
| D-0631 | Me(S) | single bond | H | *c*-Pr | H | Cl | CH₂OMe | S |
| D-0632 | Me | single bond | Me | *c*-Pr | H | Cl | CH₂OMe | O |
| D-0633 | H | CH₂ | Me | *c*-Pr | H | Cl | CH₂OMe | O |
| D-0634 | Me | CH₂ | H | *c*-Pr | H | Cl | CH₂OMe | O |
| D-0635 | Me | CH₂ | Me | *c*-Pr | H | Cl | CH₂OMe | O |
| D-0636 | H | CH(Me) | H | *c*-Pr | H | Cl | CH₂OMe | O |
| D-0637 | H | CH(Me) | Me | *c*-Pr | H | Cl | CH₂OMe | O |
| D-0638 | Me | CH(Me) | H | *c*-Pr | H | Cl | CH₂OMe | O |
| D-0639 | Me | CH(Me) | Me | *c*-Pr | H | Cl | CH₂OMe | O |
| D-0640 | H | CH₂CH₂ | Me | *c*-Pr | H | Cl | CH₂OMe | O |
| D-0641 | Me | CH₂CH₂ | H | *c*-Pr | H | Cl | CH₂OMe | O |
| D-0642 | Me | CH₂CH₂ | Me | *c*-Pr | H | Cl | CH₂OMe | O |
| D-0643 | H | single bond | Me | *c*-Pr | H | Me | CH₂OMe | O |
| D-0644 | Me | single bond | H | *c*-Pr | H | Me | CH₂OMe | O |
| D-0645 | Me(R) | single bond | H | *c*-Pr | H | Me | CH₂OMe | O |
| D-0646 | Me(S) | single bond | H | *c*-Pr | H | Me | CH₂OMe | O |
| D-0647 | Me | single bond | H | *c*-Pr | H | Me | CH₂OMe | S |
| D-0648 | Me(R) | single bond | H | *c*-Pr | H | Me | CH₂OMe | S |
| D-0649 | Me(S) | single bond | H | *c*-Pr | H | Me | CH₂OMe | S |
| D-0650 | Me | single bond | Me | *c*-Pr | H | Me | CH₂OMe | O |
| D-0651 | H | CH₂ | Me | *c*-Pr | H | Me | CH₂OMe | O |
| D-0652 | Me | CH₂ | H | *c*-Pr | H | Me | CH₂OMe | O |
| D-0653 | Me | CH₂ | Me | *c*-Pr | H | Me | CH₂OMe | O |
| D-0654 | H | CH(Me) | H | *c*-Pr | H | Me | CH₂OMe | O |

**[Table 660]**

| Compound Number | Y | W | Z | R¹ | R² | R³ | R¹⁰ | A |
|---|---|---|---|---|---|---|---|---|
| D-0655 | H | CH(Me) | Me | *c*-Pr | H | Me | CH₂OMe | O |
| D-0656 | Me | CH(Me) | H | *c*-Pr | H | Me | CH₂OMe | O |
| D-0657 | Me | CH(Me) | Me | *c*-Pr | H | Me | CH₂OMe | O |
| D-0658 | H | CH₂CH₂ | Me | *c*-Pr | H | Me | CH₂OMe | O |
| D-0659 | Me | CH₂CH₂ | H | *c*-Pr | H | Me | CH₂OMe | O |
| D-0660 | Me | CH₂CH₂ | Me | *c*-Pr | H | Me | CH₂OMe | O |
| D-0661 | H | single bond | Me | *c*-Pr | H | Cl | CH₂OMe | O |
| D-0662 | Me | single bond | H | *c*-Pr | H | Cl | CH₂OMe | O |
| D-0663 | Me(R) | single bond | H | *c*-Pr | H | Cl | CH₂OMe | O |
| D-0664 | Me(S) | single bond | H | *c*-Pr | H | Cl | CH₂OMe | O |
| D-0665 | Me | single bond | H | *c*-Pr | H | Cl | CH₂OMe | S |
| D-0666 | Me(R) | single bond | H | *c*-Pr | H | Cl | CH₂OMe | S |
| D-0667 | Me(S) | single bond | H | *c*-Pr | H | Cl | CH₂OMe | S |
| D-0668 | Me | single bond | Me | *c*-Pr | H | Cl | CH₂OMe | O |
| D-0669 | H | CH₂ | Me | *c*-Pr | H | Cl | CH₂OMe | O |
| D-0670 | Me | CH₂ | H | *c*-Pr | H | Cl | CH₂OMe | O |
| D-0671 | Me | CH₂ | Me | *c*-Pr | H | Cl | CH₂OMe | O |
| D-0672 | H | CH(Me) | H | *c*-Pr | H | Cl | CH₂OMe | O |
| D-0673 | H | CH(Me) | Me | *c*-Pr | H | Cl | CH₂OMe | O |
| D-0674 | Me | CH(Me) | H | *c*-Pr | H | Cl | CH₂OMe | O |
| D-0675 | Me | CH(Me) | Me | *c*-Pr | H | Cl | CH₂OMe | O |
| D-0676 | H | CH₂CH₂ | Me | *c*-Pr | H | Cl | CH₂OMe | O |
| D-0677 | Me | CH₂CH₂ | H | *c*-Pr | H | Cl | CH₂OMe | O |
| D-0678 | Me | CH₂CH₂ | Me | *c*-Pr | H | Cl | CH₂OMe | O |
| D-0679 | H | single bond | Me | *c*-Pr | H | Me | CH₂OMe | O |
| D-0680 | Me | single bond | H | *c*-Pr | H | Me | CH₂OMe | O |
| D-0681 | Me(R) | single bond | H | *c*-Pr | H | Me | CH₂OMe | O |
| D-0682 | Me(S) | single bond | H | *c*-Pr | H | Me | CH₂OMe | O |
| D-0683 | Me | single bond | H | *c*-Pr | H | Me | CH₂OMe | S |
| D-0684 | Me(R) | single bond | H | *c*-Pr | H | Me | CH₂OMe | S |
| D-0685 | Me(S) | single bond | H | *c*-Pr | H | Me | CH₂OMe | S |
| D-0686 | Me | single bond | Me | *c*-Pr | H | Me | CH₂OMe | O |
| D-0687 | H | CH₂ | Me | *c*-Pr | H | Me | CH₂OMe | O |
| D-0688 | Me | CH₂ | H | *c*-Pr | H | Me | CH₂OMe | O |
| D-0689 | Me | CH₂ | Me | *c*-Pr | H | Me | CH₂OMe | O |
| D-0690 | H | CH(Me) | H | *c*-Pr | H | Me | CH₂OMe | O |
| D-0691 | H | CH(Me) | Me | *c*-Pr | H | Me | CH₂OMe | O |
| D-0692 | Me | CH(Me) | H | *c*-Pr | H | Me | CH₂OMe | O |
| D-0693 | Me | CH(Me) | Me | *c*-Pr | H | Me | CH₂OMe | O |
| D-0694 | H | CH₂CH₂ | Me | *c*-Pr | H | Me | CH₂OMe | O |
| D-0695 | Me | CH₂CH₂ | H | *c*-Pr | H | Me | CH₂OMe | O |
| D-0696 | Me | CH₂CH₂ | Me | *c*-Pr | H | Me | CH₂OMe | O |
| D-0697 | H | single bond | Me | *c*-Pr | H | Cl | CH₂CN | O |
| D-0698 | Me | single bond | H | *c*-Pr | H | Cl | CH₂CN | O |

**[Table 661]**

| Compound Number | Y | W | Z | R¹ | R² | R³ | R¹⁰ | A |
|---|---|---|---|---|---|---|---|---|
| D-0699 | Me(R) | single bond | H | *c*-Pr | H | Cl | CH₂CN | O |
| D-0700 | Me(S) | single bond | H | *c*-Pr | H | Cl | CH₂CN | O |
| D-0701 | Me | single bond | H | *c*-Pr | H | Cl | CH₂CN | S |
| D-0702 | Me(R) | single bond | H | *c*-Pr | H | Cl | CH₂CN | S |
| D-0703 | Me(S) | single bond | H | *c*-Pr | H | Cl | CH₂CN | S |
| D-0704 | Me | single bond | Me | *c*-Pr | H | Cl | CH₂CN | O |
| 0-0705 | H | CH₂ | Me | *c*-Pr | H | Cl | CH₂CN | O |
| D-0706 | Me | CH₂ | H | *c*-Pr | H | Cl | CH₂CN | O |
| D-0707 | Me | CH₂ | Me | *c*-Pr | H | Cl | CH₂CN | O |
| D-0708 | H | CH(Me) | H | *c*-Pr | H | Cl | CH₂CN | O |
| 0-0709 | H | CH(Me) | Me | *c*-Pr | H | Cl | CH₂CN | O |
| D-0710 | Me | CH(Me) | H | *c*-Pr | H | Cl | CH₂CN | O |
| D-0711 | Me | CH(Me) | Me | *c*-Pr | H | Cl | CH₂CN | O |
| D-0712 | H | CH₂CH₂ | Me | *c*-Pr | H | Cl | CH₂CN | O |
| D-0713 | Me | CH₂CH₂ | H | *c*-Pr | H | Cl | CH₂CN | O |
| D-0714 | Me | CH₂CH₂ | Me | *c*-Pr | H | Cl | CH₂CN | O |
| D-0715 | H | single bond | Me | *c*-Pr | H | Me | CH₂CN | O |
| D-0716 | Me | single bond | H | *c*-Pr | H | Me | CH₂CN | O |
| D-0717 | Me(R) | single bond | H | *c*-Pr | H | Me | CH₂CN | O |
| D-0718 | Me(S) | single bond | H | *c*-Pr | H | Me | CH₂CN | O |
| D-0719 | Me | single bond | H | *c*-Pr | H | Me | CH₂CN | S |
| D-0720 | Me(R) | single bond | H | *c*-Pr | H | Me | CH₂CN | S |
| D-0721 | Me(S) | single bond | H | *c*-Pr | H | Me | CH₂CN | S |
| D-0722 | Me | single bond | Me | *c*-Pr | H | Me | CH₂CN | O |
| D-0723 | H | CH₂ | Me | *c*-Pr | H | Me | CH₂CN | O |
| D-0724 | Me | CH₂ | H | *c*-Pr | H | Me | CH₂CN | O |
| D-0725 | Me | CH₂ | Me | *c*-Pr | H | Me | CH₂CN | O |
| D-0726 | H | CH(Me) | H | *c*-Pr | H | Me | CH₂CN | O |
| D-0727 | H | CH(Me) | Me | *c*-Pr | H | Me | CH₂CN | O |
| D-0728 | Me | CH(Me) | H | *c*-Pr | H | Me | CH₂CN | O |
| D-0729 | Me | CH(Me) | Me | *c*-Pr | H | Me | CH₂CN | O |
| D-0730 | H | CH₂CH₂ | Me | *c*-Pr | H | Me | CH₂CN | O |
| D-0731 | Me | CH₂CH₂ | H | *c*-Pr | H | Me | CH₂CN | O |
| D-0732 | Me | CH₂CH₂ | Me | *c*-Pr | H | Me | CH₂CN | O |
| D-0733 | H | single bond | Me | *c*-Pr | H | Cl | CH₂CN | O |
| D-0734 | Me | single bond | H | *c*-Pr | H | Cl | CH₂CN | O |
| D-0735 | Me(R) | single bond | H | *c*-Pr | H | Cl | CH₂CN | O |
| D-0736 | Me(S) | single bond | H | *c*-Pr | H | Cl | CH₂CN | O |
| D-0737 | Me | single bond | H | *c*-Pr | H | Cl | CH₂CN | S |
| D-0738 | Me(R) | single bond | H | *c*-Pr | H | Cl | CH₂CN | S |
| D-0739 | Me(S) | single bond | H | *c*-Pr | H | Cl | CH₂CN | S |
| D-0740 | Me | single bond | Me | *c*-Pr | H | Cl | CH₂CN | O |
| D-0741 | H | CH₂ | Me | *c*-Pr | H | Cl | CH₂CN | O |
| D-0742 | Me | CH₂ | H | *c*-Pr | H | Cl | CH₂CN | O |

**[Table 662]**

| Compound Number | Y | W | Z | R¹ | R² | R³ | R¹⁰ | A |
|---|---|---|---|---|---|---|---|---|
| D-0743 | Me | CH₂ | Me | *c*-Pr | H | Cl | CH₂CN | O |
| D-0744 | H | CH(Me) | H | *c*-Pr | H | Cl | CH₂CN | O |
| D-0745 | H | CH(Me) | Me | *c*-Pr | H | Cl | CH₂CN | O |
| D-0746 | Me | CH(Me) | H | *c*-Pr | H | Cl | CH₂CN | O |
| D-0747 | Me | CH(Me) | Me | *c*-Pr | H | Cl | CH₂CN | O |
| D-0748 | H | CH₂CH₂ | Me | *c*-Pr | H | Cl | CH₂CN | O |
| D-0749 | Me | CH₂CH₂ | H | *c*-Pr | H | Cl | CH₂CN | O |
| D-0750 | Me | CH₂CH₂ | Me | *c*-Pr | H | Cl | CH₂CN | O |
| D-0751 | H | single bond | Me | *c*-Pr | H | Me | CH₂CN | O |
| D-0752 | Me | single bond | H | *c*-Pr | H | Me | CH₂CN | O |
| D-0753 | Me(R) | single bond | H | *c*-Pr | H | Me | CH₂CN | O |
| D-0754 | Me(S) | single bond | H | *c*-Pr | H | Me | CH₂CN | O |
| D-0755 | Me | single bond | H | *c*-Pr | H | Me | CH₂CN | S |
| D-0756 | Me(R) | single bond | H | *c*-Pr | H | Me | CH₂CN | S |
| D-0757 | Me(S) | single bond | H | *c*-Pr | H | Me | CH₂CN | S |
| D-0758 | Me | single bond | Me | *c*-Pr | H | Me | CH₂CN | O |
| D-0759 | H | CH₂ | Me | *c*-Pr | H | Me | CH₂CN | O |
| D-0760 | Me | CH₂ | H | *c*-Pr | H | Me | CH₂CN | O |
| D-0761 | Me | CH₂ | Me | *c*-Pr | H | Me | CH₂CN | O |
| D-0762 | H | CH(Me) | H | *c*-Pr | H | Me | CH₂CN | O |
| D-0763 | H | CH(Me) | Me | *c*-Pr | H | Me | CH₂CN | O |
| D-0764 | Me | CH(Me) | H | *c*-Pr | H | Me | CH₂CN | O |
| D-0765 | Me | CH(Me) | Me | *c*-Pr | H | Me | CH₂CN | O |
| D-0766 | H | CH₂CH₂ | Me | *c*-Pr | H | Me | CH₂CN | O |
| D-0767 | Me | CH₂CH₂ | H | *c*-Pr | H | Me | CH₂CN | O |
| D-0768 | Me | CH₂CH₂ | Me | *c*-Pr | H | Me | CH₂CN | O |
| D-0769 | H | single bond | Me | *c*-Pr | H | Cl | CH₂CN | O |
| D-0770 | Me | single bond | H | *c*-Pr | H | Cl | CH₂CN | O |
| D-0771 | Me(R) | single bond | H | *c*-Pr | H | Cl | CH₂CN | O |
| D-0772 | Me(S) | single bond | H | *c*-Pr | H | Cl | CH₂CN | O |
| D-0773 | Me | single bond | H | *c*-Pr | H | Cl | CH₂CN | S |
| D-0774 | Me(R) | single bond | H | *c*-Pr | H | Cl | CH₂CN | S |
| D-0775 | Me(S) | single bond | H | *c*-Pr | H | Cl | CH₂CN | S |
| D-0776 | Me | single bond | Me | *c*-Pr | H | Cl | CH₂CN | O |
| D-0777 | H | CH₂ | Me | *c*-Pr | H | Cl | CH₂CN | O |
| D-0778 | Me | CH₂ | H | *c*-Pr | H | Cl | CH₂CN | O |
| D-0779 | Me | CH₂ | Me | *c*-Pr | H | Cl | CH₂CN | O |
| D-0780 | H | CH(Me) | H | *c*-Pr | H | Cl | CH₂CN | O |
| D-0781 | H | CH(Me) | Me | *c*-Pr | H | Cl | CH₂CN | O |
| D-0782 | Me | CH(Me) | H | *c*-Pr | H | Cl | CH₂CN | O |
| D-0783 | Me | CH(Me) | Me | *c*-Pr | H | Cl | CH₂CN | O |
| D-0784 | H | CH₂CH₂ | Me | *c*-Pr | H | Cl | CH₂CN | O |
| D-0785 | Me | CH₂CH₂ | H | *c*-Pr | H | Cl | CH₂CN | O |
| D-0786 | Me | CH₂CH₂ | Me | *c*-Pr | H | Cl | CH₂CN | O |

**[Table 663]**

| Compound Number | Y | W | Z | R¹ | R² | R³ | R¹⁰ | A |
|---|---|---|---|---|---|---|---|---|
| D-0787 | H | single bond | Me | *c*-Pr | H | Me | CH₂CN | O |
| D-0788 | Me | single bond | H | *c*-Pr | H | Me | CH₂CN | O |
| D-0789 | Me(R) | single bond | H | *c*-Pr | H | Me | CH₂CN | O |
| D-0790 | Me(S) | single bond | H | *c*-Pr | H | Me | CH₂CN | O |
| D-0791 | Me | single bond | H | *c*-Pr | H | Me | CH₂CN | S |
| D-0792 | Me(R) | single bond | H | *c*-Pr | H | Me | CH₂CN | S |
| D-0793 | Me(S) | single bond | H | *c*-Pr | H | Me | CH₂CN | S |
| D-0794 | Me | single bond | Me | *c*-Pr | H | Me | CH₂CN | O |
| D-0795 | H | CH₂ | Me | *c*-Pr | H | Me | CH₂CN | O |
| D-0796 | Me | CH₂ | H | *c*-Pr | H | Me | CH₂CN | O |
| D-0797 | Me | CH₂ | Me | *c*-Pr | H | Me | CH₂CN | O |
| D-0798 | H | CH(Me) | H | *c*-Pr | H | Me | CH₂CN | O |
| D-0799 | H | CH(Me) | Me | *c*-Pr | H | Me | CH₂CN | O |
| D-0800 | Me | CH(Me) | H | *c*-Pr | H | Me | CH₂CN | O |
| D-0801 | Me | CH(Me) | Me | *c*-Pr | H | Me | CH₂CN | O |
| 0-0802 | H | CH₂CH₂ | Me | *c*-Pr | H | Me | CH₂CN | O |
| D-0803 | Me | CH₂CH₂ | H | *c*-Pr | H | Me | CH₂CN | O |
| D-0804 | Me | CH₂CH₂ | Me | *c*-Pr | H | Me | CH₂CN | O |
| D-0805 | H | single bond | Me | *c*-Pr | H | Cl | C(=O)Me | O |
| D-0806 | Me | single bond | H | *c*-Pr | H | Cl | C(=O)Me | O |
| D-0807 | Me(R) | single bond | H | *c*-Pr | H | Cl | C(=O)Me | O |
| D-0808 | Me(S) | single bond | H | *c*-Pr | H | Cl | C(=O)Me | O |
| D-0809 | Me | single bond | H | *c*-Pr | H | Cl | C(=O)Me | S |
| D-0810 | Me(R) | single bond | H | *c*-Pr | H | Cl | C(=O)Me | S |
| D-0811 | Me(S) | single bond | H | *c*-Pr | H | Cl | C(=O)Me | S |
| D-0812 | Me | single bond | Me | *c*-Pr | H | Cl | C(=O)Me | O |
| D-0813 | H | CH₂ | Me | *c*-Pr | H | Cl | C(=O)Me | O |
| D-0814 | Me | CH₂ | H | *c*-Pr | H | Cl | C(=O)Me | O |
| D-0815 | Me | CH₂ | Me | *c*-Pr | H | Cl | C(=O)Me | O |
| D-0816 | H | CH(Me) | H | *c*-Pr | H | Cl | C(=O)Me | O |
| D-0817 | H | CH(Me) | Me | *c*-Pr | H | Cl | C(=O)Me | O |
| D-0818 | Me | CH(Me) | H | *c*-Pr | H | Cl | C(=O)Me | O |
| D-0819 | Me | CH(Me) | Me | *c*-Pr | H | Cl | C(=O)Me | O |
| D-0820 | H | CH₂CH₂ | Me | *c*-Pr | H | Cl | C(=O)Me | O |
| D-0821 | Me | CH₂CH₂ | H | *c*-Pr | H | Cl | C(=O)Me | O |
| D-0822 | Me | CH₂CH₂ | Me | *c*-Pr | H | Cl | C(=O)Me | O |
| D-0823 | H | single bond | Me | *c*-Pr | H | Me | C(=O)Me | O |
| D-0824 | Me | single bond | H | *c*-Pr | H | Me | C(=O)Me | O |
| D-0825 | Me(R) | single bond | H | *c*-Pr | H | Me | C(=O)Me | O |
| D-0826 | Me(S) | single bond | H | *c*-Pr | H | Me | C(=O)Me | O |
| D-0827 | Me | single bond | H | *c*-Pr | H | Me | C(=O)Me | S |
| D-0828 | Me(R) | single bond | H | *c*-Pr | H | Me | C(=O)Me | S |
| D-0829 | Me(S) | single bond | H | *c*-Pr | H | Me | C(=O)Me | S |
| D-0830 | Me | single bond | Me | *c*-Pr | H | Me | C(=O)Me | o |

**[Table 664]**

| Compound Number | Y | W | Z | R¹ | R² | R³ | R¹⁰ | A |
|---|---|---|---|---|---|---|---|---|
| D-0831 | H | CH₂ | Me | *c*-Pr | H | Me | C(=O)Me | O |
| D-0832 | Me | CH₂ | H | *c*-Pr | H | Me | C(=O)Me | O |
| D-0833 | Me | CH₂ | Me | *c*-Pr | H | Me | C(=O)Me | O |
| D-0834 | H | CH(Me) | H | *c-*Pr | H | Me | C(=O)Me | O |
| D-0835 | H | CH(Me) | Me | *c*-Pr | H | Me | C(=O)Me | O |
| D-0836 | Me | CH(Me) | H | *c*-Pr | H | Me | C(=O)Me | O |
| D-0837 | Me | CH(Me) | Me | *c*-Pr | H | Me | C(=O)Me | O |
| D-0838 | H | CH₂CH₂ | Me | *c*-Pr | H | Me | C(=O)Me | O |
| D-0839 | Me | CH₂CH₂ | H | *c*-Pr | H | Me | C(=O)Me | O |
| D-0840 | Me | CH₂CH₂ | Me | *c*-Pr | H | Me | C(=O)Me | O |
| D-0841 | H | single bond | Me | *c*-Pr | H | Cl | C(=O)Me | O |
| D-0842 | Me | single bond | H | *c*-Pr | H | Cl | C(=O)Me | O |
| D-0843 | Me(R) | single bond | H | *c*-Pr | H | Cl | C(=O)Me | O |
| D-0844 | Me(S) | single bond | H | *c*-Pr | H | Cl | C(=O)Me | O |
| D-0845 | Me | single bond | H | *c*-Pr | H | Cl | C(=O)Me | S |
| D-0846 | Me(R) | single bond | H | *c*-Pr | H | Cl | C(=O)Me | S |
| D-0847 | Me(S) | single bond | H | *c*-Pr | H | Cl | C(=O)Me | S |
| D-0848 | Me | single bond | Me | *c*-Pr | H | Cl | C(=O)Me | O |
| D-0849 | H | CH₂ | Me | *c*-Pr | H | Cl | C(=O)Me | O |
| D-0850 | Me | CH₂ | H | *c*-Pr | H | Cl | C(=O)Me | O |
| D-0851 | Me | CH₂ | Me | *c*-Pr | H | Cl | C(=O)Me | O |
| D-0852 | H | CH(Me) | H | *c*-Pr | H | Cl | C(=O)Me | O |
| D-0853 | H | CH(Me) | Me | *c*-Pr | H | Cl | C(=O)Me | O |
| D-0854 | Me | CH(Me) | H | *c*-Pr | H | Cl | C(=O)Me | O |
| D-0855 | Me | CH(Me) | Me | *c*-Pr | H | Cl | C(=O)Me | O |
| D-0856 | H | CH₂CH₂ | Me | *c*-Pr | H | Cl | C(=O)Me | O |
| D-0857 | Me | CH₂CH₂ | H | *c*-Pr | H | Cl | C(=O)Me | O |
| D-0858 | Me | CH₂CH₂ | Me | *c*-Pr | H | Cl | C(=O)Me | O |
| D-0859 | H | single bond | Me | *c*-Pr | H | Me | C(=O)Me | O |
| D-0860 | Me | single bond | H | *c*-Pr | H | Me | C(=O)Me | O |
| D-0861 | Me(R) | single bond | H | *c*-Pr | H | Me | C(=O)Me | O |
| D-0862 | Me(S) | single bond | H | *c*-Pr | H | Me | C(=O)Me | O |
| D-0863 | Me | single bond | H | *c*-Pr | H | Me | C(=O)Me | S |
| D-0864 | Me(R) | single bond | H | *c*-Pr | H | Me | C(=O)Me | S |
| D-0865 | Me(S) | single bond | H | *c*-Pr | H | Me | C(=O)Me | S |
| D-0866 | Me | single bond | Me | *c*-Pr | H | Me | C(=O)Me | O |
| D-0867 | H | CH₂ | Me | *c*-Pr | H | Me | C(=O)Me | O |
| D-0868 | Me | CH₂ | H | *c*-Pr | H | Me | C(=O)Me | O |
| D-0869 | Me | CH₂ | Me | *c*-Pr | H | Me | C(=O)Me | O |
| D-0870 | H | CH(Me) | H | *c*-Pr | H | Me | C(=O)Me | O |
| D-0871 | H | CH(Me) | Me | *c*-Pr | H | Me | C(=O)Me | O |
| D-0872 | Me | CH(Me) | H | *c*-Pr | H | Me | C(=O)Me | O |
| D-0873 | Me | CH(Me) | Me | *c*-Pr | H | Me | C(=O)Me | O |
| D-0874 | H | CH₂CH₂ | Me | *c*-Pr | H | Me | C(=O)Me | O |

**[Table 665]**

| Compound Number | Y | W | Z | R¹ | R² | R³ | R¹⁰ | A |
|---|---|---|---|---|---|---|---|---|
| D-0875 | Me | CH₂CH₂ | H | *c*-Pr | H | Me | C(=O)Me | O |
| D-0876 | Me | CH₂CH₂ | Me | *c*-Pr | H | Me | C(=O)Me | O |
| D-0877 | H | single bond | Me | *c*-Pr | H | Cl | C(=O)Me | O |
| D-0878 | Me | single bond | H | *c*-Pr | H | Cl | C(=O)Me | O |
| D-0879 | Me(R) | single bond | H | *c*-Pr | H | Cl | C(=O)Me | O |
| D-0880 | Me(S) | single bond | H | *c*-Pr | H | Cl | C(=O)Me | O |
| D-0881 | Me | single bond | H | *c*-Pr | H | Cl | C(=O)Me | S |
| D-0882 | Me(R) | single bond | H | *c*-Pr | H | Cl | C(=O)Me | S |
| D-0883 | Me(S) | single bond | H | *c*-Pr | H | Cl | C(=O)Me | S |
| D-0884 | Me | single bond | Me | *c*-Pr | H | Cl | C(=O)Me | O |
| D-0885 | H | CH₂ | Me | *c*-Pr | H | Cl | C(=O)Me | O |
| D-0886 | Me | CH₂ | H | *c*-Pr | H | Cl | C(=O)Me | O |
| D-0887 | Me | CH₂ | Me | *c*-Pr | H | Cl | C(=O)Me | O |
| D-0888 | H | CH(Me) | H | *c*-Pr | H | Cl | C(=O)Me | O |
| D-0889 | H | CH(Me) | Me | *c*-Pr | H | Cl | C(=O)Me | O |
| D-0890 | Me | CH(Me) | H | *c*-Pr | H | Cl | C(=O)Me | O |
| D-0891 | Me | CH(Me) | Me | *c*-Pr | H | Cl | C(=O)Me | O |
| D-0892 | H | CH₂CH₂ | Me | *c*-Pr | H | Cl | C(=O)Me | O |
| D-0893 | Me | CH₂CH₂ | H | *c*-Pr | H | Cl | C(=O)Me | O |
| D-0894 | Me | CH₂CH₂ | Me | *c*-Pr | H | Cl | C(=O)Me | O |
| D-0895 | H | single bond | Me | *c*-Pr | H | Me | C(=O)Me | O |
| D-0896 | Me | single bond | H | *c*-Pr | H | Me | C(=O)Me | O |
| D-0897 | Me(R) | single bond | H | *c*-Pr | H | Me | C(=O)Me | O |
| D-0898 | Me(S) | single bond | H | *c*-Pr | H | Me | C(=O)Me | O |
| D-0899 | Me | single bond | H | *c-*Pr | H | Me | C(=O)Me | S |
| D-0900 | Me(R) | single bond | H | *c*-Pr | H | Me | C(=O)Me | S |
| D-0901 | Me(S) | single bond | H | *c*-Pr | H | Me | C(=O)Me | S |
| D-0902 | Me | single bond | Me | *c*-Pr | H | Me | C(=O)Me | O |
| D-0903 | H | CH₂ | Me | *c*-Pr | H | Me | C(=O)Me | O |
| D-0904 | Me | CH₂ | H | *c*-Pr | H | Me | C(=O)Me | O |
| D-0905 | Me | CH₂ | Me | *c*-Pr | H | Me | C(=O)Me | O |
| D-0906 | H | CH(Me) | H | *c*-Pr | H | Me | C(=O)Me | O |
| D-0907 | H | CH(Me) | Me | *c*-Pr | H | Me | C(=O)Me | O |
| D-0908 | Me | CH(Me) | H | *c*-Pr | H | Me | C(=O)Me | O |
| D-0909 | Me | CH(Me) | Me | *c*-Pr | H | Me | C(=O)Me | O |
| D-0910 | H | CH₂CH₂ | Me | *c*-Pr | H | Me | C(=O)Me | O |
| D-0911 | Me | CH₂CH₂ | H | *c*-Pr | H | Me | C(=O)Me | O |
| D-0912 | Me | CH₂CH₂ | Me | *c*-Pr | H | Me | C(=O)Me | O |
| D-0913 | H | single bond | Me | *c*-Pr | H | Cl | C(=O)OEt | O |
| D-0914 | Me | single bond | H | *c*-Pr | H | Cl | C(=O)OEt | O |
| D-0915 | Me(R) | single bond | H | *c*-Pr | H | Cl | C(=O)OEt | O |
| D-0916 | Me(S) | single bond | H | *c*-Pr | H | Cl | C(=O)OEt | O |
| D-0917 | Me | single bond | H | *c*-Pr | H | Cl | C(=O)OEt | S |
| D-0918 | Me(R) | single bond | H | *c*-Pr | H | Cl | C(=O)OEt | S |

**[Table 666]**

| Compound Number | Y | W | Z | R¹ | R² | R³ | R¹⁰ | A |
|---|---|---|---|---|---|---|---|---|
| D-0919 | Me(S) | single bond | H | *c*-Pr | H | Cl | C(=O)OEt | S |
| D-0920 | Me | single bond | Me | *c*-Pr | H | Cl | C(=O)OEt | O |
| D-0921 | H | CH₂ | Me | *c*-Pr | H | Cl | C(=O)OEt | O |
| D-0922 | Me | CH₂ | H | *c*-Pr | H | Cl | C(=O)OEt | O |
| D-0923 | Me | CH₂ | Me | *c*-Pr | H | Cl | C(=O)OEt | O |
| D-0924 | H | CH(Me) | H | *c*-Pr | H | Cl | C(=O)OEt | O |
| D-0925 | H | CH(Me) | Me | *c*-Pr | H | Cl | C(=O)OEt | O |
| D-0926 | Me | CH(Me) | H | *c*-Pr | H | Cl | C(=O)OEt | O |
| D-0927 | Me | CH(Me) | Me | *c*-Pr | H | Cl | C(=O)OEt | O |
| D-0928 | H | CH₂CH₂ | Me | *c*-Pr | H | Cl | C(=O)OEt | O |
| D-0929 | Me | CH₂CH₂ | H | *c*-Pr | H | Cl | C(=O)OEt | O |
| D-0930 | Me | CH₂CH₂ | Me | *c*-Pr | H | Cl | C(=O)OEt | O |
| D-0931 | H | single bond | Me | *c*-Pr | H | Me | C(=O)OEt | O |
| D-0932 | Me | single bond | H | *c*-Pr | H | Me | C(=O)OEt | O |
| D-0933 | Me(R) | single bond | H | *c*-Pr | H | Me | C(=O)OEt | O |
| D-0934 | Me(S) | single bond | H | *c*-Pr | H | Me | C(=O)OEt | O |
| D-0935 | Me | single bond | H | *c*-Pr | H | Me | C(=O)OEt | S |
| D-0936 | Me(R) | single bond | H | *c*-Pr | H | Me | C(=O)OEt | S |
| D-0937 | Me(S) | single bond | H | *c*-Pr | H | Me | C(=O)OEt | S |
| D-0938 | Me | single bond | Me | *c*-Pr | H | Me | C(=O)OEt | O |
| D-0939 | H | CH₂ | Me | *c*-Pr | H | Me | C(=O)OEt | O |
| D-0940 | Me | CH₂ | H | *c*-Pr | H | Me | C(=O)OEt | O |
| D-0941 | Me | CH₂ | Me | *c*-Pr | H | Me | C(=O)OEt | O |
| D-0942 | H | CH(Me) | H | *c*-Pr | H | Me | C(=O)OEt | O |
| D-0943 | H | CH(Me) | Me | *c*-Pr | H | Me | C(=O)OEt | O |
| D-0944 | Me | CH(Me) | H | *c*-Pr | H | Me | C(=O)OEt | O |
| D-0945 | Me | CH(Me) | Me | *c*-Pr | H | Me | C(=O)OEt | O |
| D-0946 | H | CH₂CH₂ | Me | *c*-Pr | H | Me | C(=O)OEt | O |
| D-0947 | Me | CH₂CH₂ | H | *c*-Pr | H | Me | C(=O)OEt | O |
| D-0948 | Me | CH₂CH₂ | Me | *c*-Pr | H | Me | C(=O)OEt | O |
| D-0949 | H | single bond | Me | *c*-Pr | H | Cl | C(=O)OEt | O |
| D-0950 | Me | single bond | H | *c*-Pr | H | Cl | C(=O)OEt | O |
| D-0951 | Me(R) | single bond | H | *c*-Pr | H | Cl | C(=O)OEt | O |
| D-0952 | Me(S) | single bond | H | *c*-Pr | H | Cl | C(=O)OEt | O |
| D-0953 | Me | single bond | H | *c*-Pr | H | Cl | C(=O)OEt | S |
| D-0954 | Me(R) | single bond | H | *c*-Pr | H | Cl | C(=O)OEt | S |
| D-0955 | Me(S) | single bond | H | *c*-Pr | H | Cl | C(=O)OEt | S |
| D-0956 | Me | single bond | Me | *c*-Pr | H | Cl | C(=O)OEt | O |
| D-0957 | H | CH₂ | Me | *c*-Pr | H | Cl | C(=O)OEt | O |
| D-0958 | Me | CH₂ | H | *c*-Pr | H | Cl | C(=O)OEt | O |
| D-0959 | Me | CH₂ | Me | *c*-Pr | H | Cl | C(=O)OEt | O |
| D-0960 | H | CH(Me) | H | *c*-Pr | H | Cl | C(=O)OEt | O |
| D-0961 | H | CH(Me) | Me | *c*-Pr | H | Cl | C(=O)OEt | O |
| D-0962 | Me | CH(Me) | H | *c*-Pr | H | Cl | C(=O)OEt | O |

**[Table 667]**

| Compound Number | Y | W | Z | R¹ | R² | R³ | R¹⁰ | A |
|---|---|---|---|---|---|---|---|---|
| D-0963 | Me | CH(Me) | Me | *c*-Pr | H | Cl | C(=O)OEt | O |
| D-0964 | H | CH₂CH₂ | Me | *c*-Pr | H | Cl | C(=O)OEt | O |
| D-0965 | Me | CH₂CH₂ | H | *c*-Pr | H | Cl | C(=O)OEt | O |
| D-0966 | Me | CH₂CH₂ | Me | *c*-Pr | H | Cl | C(=O)OEt | O |
| D-0967 | H | single bond | Me | *c*-Pr | H | Me | C(=O)OEt | O |
| D-0968 | Me | single bond | H | *c*-Pr | H | Me | C(=O)OEt | O |
| D-0969 | Me(R) | single bond | H | *c*-Pr | H | Me | C(=O)OEt | O |
| D-0970 | Me(S) | single bond | H | *c*-Pr | H | Me | C(=O)OEt | O |
| D-0971 | Me | single bond | H | *c*-Pr | H | Me | C(=O)OEt | S |
| D-0972 | Me(R) | single bond | H | *c*-Pr | H | Me | C(=O)OEt | S |
| D-0973 | Me(S) | single bond | H | *c*-Pr | H | Me | C(=O)OEt | S |
| D-0974 | Me | single bond | Me | *c*-Pr | H | Me | C(=O)OEt | O |
| D-0975 | H | CH₂ | Me | *c*-Pr | H | Me | C(=O)OEt | O |
| D-0976 | Me | CH₂ | H | *c*-Pr | H | Me | C(=O)OEt | O |
| D-0977 | Me | CH₂ | Me | *c*-Pr | H | Me | C(=O)OEt | O |
| D-0978 | H | CH(Me) | H | *c*-Pr | H | Me | C(=O)OEt | O |
| D-0979 | H | CH(Me) | Me | *c*-Pr | H | Me | C(=O)OEt | O |
| D-0980 | Me | CH(Me) | H | *c*-Pr | H | Me | C(=O)OEt | O |
| D-0981 | Me | CH(Me) | Me | *c*-Pr | H | Me | C(=O)OEt | O |
| D-0982 | H | CH₂CH₂ | Me | *c*-Pr | H | Me | C(=O)OEt | O |
| D-0983 | Me | CH₂CH₂ | H | *c*-Pr | H | Me | C(=O)OEt | O |
| D-0984 | Me | CH₂CH₂ | Me | *c*-Pr | H | Me | C(=O)OEt | O |
| D-0985 | H | single bond | Me | *c*-Pr | H | Cl | C(=O)OEt | O |
| D-0986 | Me | single bond | H | *c*-Pr | H | Cl | C(=O)OEt | O |
| D-0987 | Me(R) | single bond | H | *c*-Pr | H | Cl | C(=O)OEt | O |
| D-0988 | Me(S) | single bond | H | *c*-Pr | H | Cl | C(=O)OEt | O |
| D-0989 | Me | single bond | H | *c*-Pr | H | Cl | C(=O)OEt | S |
| D-0990 | Me(R) | single bond | H | *c*-Pr | H | Cl | C(=O)OEt | S |
| D-0991 | Me(S) | single bond | H | *c*-Pr | H | Cl | C(=O)OEt | S |
| D-0992 | Me | single bond | Me | *c*-Pr | H | Cl | C(=O)OEt | O |
| D-0993 | H | CH₂ | Me | *c*-Pr | H | Cl | C(=O)OEt | O |
| D-0994 | Me | CH₂ | H | *c*-Pr | H | Cl | C(=O)OEt | O |
| D-0995 | Me | CH₂ | Me | *c*-Pr | H | Cl | C(=O)OEt | O |
| D-0996 | H | CH(Me) | H | *c*-Pr | H | Cl | C(=O)OEt | O |
| D-0997 | H | CH(Me) | Me | *c*-Pr | H | Cl | C(=O)OEt | O |
| D-0998 | Me | CH(Me) | H | *c*-Pr | H | Cl | C(=O)OEt | O |
| D-0999 | Me | CH(Me) | Me | *c*-Pr | H | Cl | C(=O)OEt | O |
| D-1000 | H | CH₂CH₂ | Me | c-Pr | H | Cl | C(=O)OEt | O |
| D-1001 | Me | CH₂CH₂ | H | *c*-Pr | H | Cl | C(=O)OEt | O |
| D-1002 | Me | CH₂CH₂ | Me | *c*-Pr | H | Cl | C(=O)OEt | O |
| D-1003 | H | single bond | Me | *c*-Pr | H | Me | C(=O)OEt | O |
| D-1004 | Me | single bond | H | *c*-Pr | H | Me | C(=O)OEt | O |
| D-1005 | Me(R) | single bond | H | *c*-Pr | H | Me | C(=O)OEt | O |
| D-1006 | Me(S) | single bond | H | *c*-Pr | H | Me | C(=O)OEt | O |

**[Table 668]**

| Compound Number | Y | W | Z | R¹ | R² | R³ | R¹⁰ | A |
|---|---|---|---|---|---|---|---|---|
| D-1007 | Me | single bond | H | *c*-Pr | H | Me | C(=O)OEt | S |
| D-1008 | Me(R) | single bond | H | *c*-Pr | H | Me | C(=O)OEt | S |
| D-1009 | Me(S) | single bond | H | *c*-Pr | H | Me | C(=O)OEt | S |
| D-1010 | Me | single bond | Me | *c*-Pr | H | Me | C(=O)OEt | O |
| D-1011 | H | CH₂ | Me | *c*-Pr | H | Me | C(=O)OEt | O |
| D-1012 | Me | CH₂ | H | *c*-Pr | H | Me | C(=O)OEt | O |
| D-1013 | Me | CH₂ | Me | *c*-Pr | H | Me | C(=O)OEt | O |
| D-1014 | H | CH(Me) | H | *c*-Pr | H | Me | C(=O)OEt | O |
| D-1015 | H | CH(Me) | Me | *c*-Pr | H | Me | C(=O)OEt | O |
| D-1016 | Me | CH(Me) | H | *c*-Pr | H | Me | C(=O)OEt | O |
| D-1017 | Me | CH(Me) | Me | *c*-Pr | H | Me | C(=O)OEt | O |
| D-1018 | H | CH₂CH₂ | Me | *c*-Pr | H | Me | C(=O)OEt | O |
| D-1019 | Me | CH₂CH₂ | H | *c*-Pr | H | Me | C(=O)OEt | O |
| D-1020 | Me | CH₂CH₂ | Me | *c*-Pr | H | Me | C(=O)OEt | O |
| D-1021 | H | single bond | Me | *c*-Pr | Me | Cl | Me | O |
| D-1022 | H | single bond | Et | *c*-Pr | Me | Cl | Me | O |
| D-1023 | Me | single bond | H | *c*-Pr | Me | Cl | Me | O |
| D-1024 | Me(R) | single bond | H | *c*-Pr | Me | Cl | Me | O |
| D-1025 | Me(S) | single bond | H | *c*-Pr | Me | Cl | Me | O |
| D-1026 | H | CH₂ | Me | *c*-Pr | Me | Cl | Me | O |
| D-1027 | Me | CH₂ | H | *c*-Pr | Me | Cl | Me | O |
| D-1028 | H | CH(Me) | H | *c*-Pr | Me | Cl | Me | O |
| D-1029 | H | CH₂CH₂ | Me | *c*-Pr | Me | Cl | Me | O |
| D-1030 | Me | CH₂CH₂ | H | *c*-Pr | Me | Cl | Me | O |
| D-1031 | H | single bond | Me | *c*-Pr | Me | Me | Me | O |
| D-1032 | Me | single bond | H | *c*-Pr | Me | Me | Me | O |
| D-1033 | Me(R) | single bond | H | *c*-Pr | Me | Me | Me | O |
| D-1034 | Me(S) | single bond | H | *c*-Pr | Me | Me | Me | O |
| D-1035 | H | CH₂ | Me | *c*-Pr | Me | Me | Me | O |
| D-1036 | Me | CH₂ | H | *c*-Pr | Me | Me | Me | O |
| D-1037 | H | CH(Me) | H | *c*-Pr | Me | Me | Me | O |
| D-1038 | H | CH₂CH₂ | Me | *c*-Pr | Me | Me | Me | O |
| D-1039 | Me | CH₂CH₂ | H | *c*-Pr | Me | Me | Me | O |
| D-1040 | H | single bond | Me | *c*-Pr | Me | Cl | Et | O |
| D-1041 | Me | single bond | H | *c*-Pr | Me | Cl | Et | O |
| D-1042 | Me(R) | single bond | H | *c*-Pr | Me | Cl | Et | O |
| D-1043 | Me(S) | single bond | H | *c*-Pr | Me | Cl | Et | O |
| D-1044 | H | CH₂ | Me | *c*-Pr | Me | Cl | Et | O |
| D-1045 | Me | CH₂ | H | *c*-Pr | Me | Cl | Et | O |
| D-1046 | H | CH(Me) | H | *c*-Pr | Me | Cl | Et | O |
| D-1047 | H | CH₂CH₂ | Me | *c*-Pr | Me | Cl | Et | O |
| D-1048 | Me | CH₂CH₂ | H | *c*-Pr | Me | Cl | Et | O |
| D-1049 | H | single bond | Me | *c*-Pr | Me | Me | Et | O |
| D-1050 | Me | single bond | H | *c*-Pr | Me | Me | Et | O |

**[Table 669]**

| Compound Number | Y | W | Z | R¹ | R² | R³ | R¹⁰ | A |
|---|---|---|---|---|---|---|---|---|
| D-1051 | Me(R) | single bond | H | *c*-Pr | Me | Me | Et | O |
| D-1052 | Me(S) | single bond | H | *c*-Pr | Me | Me | Et | O |
| D-1053 | H | CH₂ | Me | *c*-Pr | Me | Me | Et | O |
| D-1054 | Me | CH₂ | H | *c*-Pr | Me | Me | Et | O |
| D-1055 | H | CH(Me) | H | *c*-Pr | Me | Me | Et | O |
| D-1056 | H | CH₂CH₂ | Me | *c*-Pr | Me | Me | Et | O |
| D-1057 | Me | CH₂CH₂ | H | *c*-Pr | Me | Me | Et | O |
| D-1058 | H | single bond | Me | *c*-Pr | Me | Cl | CH₂CF₃ | O |
| D-1059 | Me | single bond | H | *c*-Pr | Me | Cl | CH₂CF₃ | O |
| D-1060 | Me(R) | single bond | H | *c*-Pr | Me | Cl | CH₂CF₃ | O |
| D-1061 | Me(S) | single bond | H | *c*-Pr | Me | Cl | CH₂CF₃ | O |
| D-1062 | H | CH₂ | Me | *c*-Pr | Me | Cl | CH₂CF₃ | O |
| D-1063 | Me | CH₂ | H | *c*-Pr | Me | Cl | CH₂CF₃ | O |
| D-1064 | H | CH(Me) | H | *c*-Pr | Me | Cl | CH₂CF₃ | O |
| D-1065 | H | CH₂CH₂ | Me | *c*-Pr | Me | Cl | CH₂CF₃ | O |
| D-1066 | Me | CH₂CH₂ | H | *c*-Pr | Me | Cl | CH₂CF₃ | O |
| D-1067 | H | single bond | Me | *c*-Pr | Me | Me | CH₂CF₃ | O |
| D-1068 | Me | single bond | H | *c*-Pr | Me | Me | CH₂CF₃ | O |
| D-1069 | Me(R) | single bond | H | *c*-Pr | Me | Me | CH₂CF₃ | O |
| D-1070 | Me(S) | single bond | H | *c*-Pr | Me | Me | CH₂CF₃ | O |
| D-1071 | H | CH₂ | Me | *c*-Pr | Me | Me | CH₂CF₃ | O |
| D-1072 | Me | CH₂ | H | *c*-Pr | Me | Me | CH₂CF₃ | O |
| D-1073 | H | CH(Me) | H | *c*-Pr | Me | Me | CH₂CF₃ | O |
| D-1074 | H | CH₂CH₂ | Me | *c*-Pr | Me | Me | CH₂CF₃ | O |
| D-1075 | Me | CH₂CH₂ | H | *c*-Pr | Me | Me | CH₂CF₃ | O |
| D-1076 | H | single bond | Me | *c*-Pr | Me | Cl | CH₂OMe | O |
| D-1077 | Me | single bond | H | *c*-Pr | Me | Cl | CH₂OMe | O |
| D-1078 | Me(R) | single bond | H | *c*-Pr | Me | Cl | CH₂OMe | O |
| D-1079 | Me(S) | single bond | H | *c*-Pr | Me | Cl | CH₂OMe | O |
| D-1080 | H | CH₂ | Me | *c*-Pr | Me | Cl | CH₂OMe | O |
| D-1081 | Me | CH₂ | H | *c*-Pr | Me | Cl | CH₂OMe | O |
| D-1082 | H | CH(Me) | H | *c*-Pr | Me | Cl | CH₂OMe | O |
| D-1083 | H | CH₂CH₂ | Me | *c*-Pr | Me | Cl | CH₂OMe | O |
| D-1084 | Me | CH₂CH₂ | H | *c*-Pr | Me | Cl | CH₂OMe | O |
| D-1085 | H | single bond | Me | *c*-Pr | Me | Me | CH₂OMe | O |
| D-1086 | Me | single bond | H | *c*-Pr | Me | Me | CH₂OMe | O |
| D-1087 | Me(R) | single bond | H | *c*-Pr | Me | Me | CH₂OMe | O |
| D-1088 | Me(S) | single bond | H | *c*-Pr | Me | Me | CH₂OMe | O |
| D-1089 | H | CH₂ | Me | *c*-Pr | Me | Me | CH₂OMe | O |
| D-1090 | Me | CH₂ | H | *c*-Pr | Me | Me | CH₂OMe | O |
| D-1091 | H | CH(Me) | H | *c*-Pr | Me | Me | CH₂OMe | O |
| D-1092 | H | CH₂CH₂ | Me | *c*-Pr | Me | Me | CH₂OMe | O |
| D-1093 | Me | CH₂CH₂ | H | *c*-Pr | Me | Me | CH₂OMe | O |
| D-1094 | H | single bond | Me | *c*-Pr | Me | Cl | CH₂CN | O |

**[Table 670]**

| Compound Number | Y | W | Z | R¹ | R² | R³ | R¹⁰ | A |
|---|---|---|---|---|---|---|---|---|
| D-1095 | Me | single bond | H | *c*-Pr | Me | Cl | CH₂CN | O |
| D-1096 | Me(R) | single bond | H | *c*-Pr | Me | Cl | CH₂CN | O |
| D-1097 | Me(S) | single bond | H | *c*-Pr | Me | Cl | CH₂CN | O |
| D-1098 | H | CH₂ | Me | *c*-Pr | Me | Cl | CH₂CN | O |
| D-1099 | Me | CH₂ | H | *c*-Pr | Me | Cl | CH₂CN | O |
| D-1100 | H | CH(Me) | H | *c*-Pr | Me | Cl | CH₂CN | O |
| D-1101 | H | CH₂CH₂ | Me | *c*-Pr | Me | Cl | CH₂CN | O |
| D-1102 | Me | CH₂CH₂ | H | *c*-Pr | Me | Cl | CH₂CN | O |
| D-1103 | H | single bond | Me | *c*-Pr | Me | Me | CH₂CN | O |
| D-1104 | Me | single bond | H | *c*-Pr | Me | Me | CH₂CN | O |
| D-1105 | Me(R) | single bond | H | *c*-Pr | Me | Me | CH₂CN | O |
| D-1106 | Me(S) | single bond | H | *c*-Pr | Me | Me | CH₂CN | O |
| D-1107 | H | CH₂ | Me | *c*-Pr | Me | Me | CH₂CN | O |
| D-1108 | Me | CH₂ | H | *c*-Pr | Me | Me | CH₂CN | O |
| D-1109 | H | CH(Me) | H | *c*-Pr | Me | Me | CH₂CN | O |
| D-1110 | H | CH₂CH₂ | Me | *c*-Pr | Me | Me | CH₂CN | O |
| D-1111 | Me | CH₂CH₂ | H | *c*-Pr | Me | Me | CH₂CN | O |
| D-1112 | H | single bond | Me | *c*-Pr | Me | Cl | C(=O)Me | O |
| D-1113 | Me | single bond | H | *c*-Pr | Me | Cl | C(=O)Me | O |
| D-1114 | Me(R) | single bond | H | *c*-Pr | Me | Cl | C(=O)Me | O |
| D-1115 | Me(S) | single bond | H | *c*-Pr | Me | Cl | C(=O)Me | O |
| D-1116 | H | CH₂ | Me | *c*-Pr | Me | Cl | C(=O)Me | O |
| D-1117 | Me | CH₂ | H | *c*-Pr | Me | Cl | C(=O)Me | O |
| D-1118 | H | CH(Me) | H | *c*-Pr | Me | Cl | C(=O)Me | O |
| D-1119 | H | CH₂CH₂ | Me | *c*-Pr | Me | Cl | C(=O)Me | O |
| D-1120 | Me | CH₂CH₂ | H | *c*-Pr | Me | Cl | C(=O)Me | O |
| D-1121 | H | single bond | Me | *c*-Pr | Me | Me | C(=O)Me | O |
| D-1122 | Me | single bond | H | *c*-Pr | Me | Me | C(=O)Me | O |
| D-1123 | Me(R) | single bond | H | *c*-Pr | Me | Me | C(=O)Me | O |
| D-1124 | Me(S) | single bond | H | *c*-Pr | Me | Me | C(=O)Me | O |
| D-1125 | H | CH₂ | Me | *c*-Pr | Me | Me | C(=O)Me | O |
| D-1126 | Me | CH₂ | H | *c*-Pr | Me | Me | C(=O)Me | O |
| D-1127 | H | CH(Me) | H | *c*-Pr | Me | Me | C(=O)Me | O |
| D-1128 | H | CH₂CH₂ | Me | *c*-Pr | Me | Me | C(=O)Me | O |
| D-1129 | Me | CH₂CH₂ | H | *c*-Pr | Me | Me | C(=O)Me | O |
| D-1130 | H | single bond | Me | *c*-Pr | CH₂OMe | Cl | Me | O |
| D-1131 | H | single bond | Et | *c*-Pr | CH₂OMe | Cl | Me | O |
| D-1132 | Me | single bond | H | *c*-Pr | CH₂OMe | Cl | Me | O |
| D-1133 | Me(R) | single bond | H | *c*-Pr | CH₂OMe | Cl | Me | O |
| D-1134 | Me(S) | single bond | H | *c*-Pr | CH₂OMe | Cl | Me | O |
| D-1135 | H | CH₂ | Me | *c*-Pr | CH₂OMe | Cl | Me | O |
| D-1136 | Me | CH₂ | H | *c*-Pr | CH₂OMe | Cl | Me | O |
| D-1137 | H | CH(Me) | H | *c*-Pr | CH₂OMe | Cl | Me | O |
| D-1138 | H | CH₂CH₂ | Me | *c*-Pr | CH₂OMe | Cl | Me | O |

**[ Table 671]**

| Compound Number | Y | W | Z | R¹ | R² | R³ | R¹⁰ | A |
|---|---|---|---|---|---|---|---|---|
| D-1139 | Me | CH₂CH₂ | H | *c*-Pr | CH₂OMe | Cl | Me | O |
| D-1140 | H | single bond | Me | *c*-Pr | CH₂OMe | Me | Me | O |
| D-1141 | Me | single bond | H | *c*-Pr | CH₂OMe | Me | Me | O |
| D-1142 | Me(R) | single bond | H | *c*-Pr | CH₂OMe | Me | Me | O |
| D-1143 | Me(S) | single bond | H | *c*-Pr | CH₂OMe | Me | Me | O |
| D-1144 | H | CH₂ | Me | *c*-Pr | CH₂OMe | Me | Me | O |
| D-1145 | Me | CH₂ | H | *c*-Pr | CH₂OMe | Me | Me | O |
| D-1146 | H | CH(Me) | H | *c*-Pr | CH₂OMe | Me | Me | O |
| D-1147 | H | CH₂CH₂ | Me | *c*-Pr | CH₂OMe | Me | Me | O |
| D-1148 | Me | CH₂CH₂ | H | *c*-Pr | CH₂OMe | Me | Me | O |
| D-1149 | H | single bond | Me | *c*-Pr | CH₂OMe | Cl | Et | O |
| D-1150 | Me | single bond | H | *c*-Pr | CH₂OMe | Cl | Et | O |
| D-1151 | Me(R) | single bond | H | *c*-Pr | CH₂OMe | Cl | Et | O |
| D-1152 | Me(S) | single bond | H | *c*-Pr | CH₂OMe | Cl | Et | O |
| D-1153 | H | CH₂ | Me | *c*-Pr | CH₂OMe | Cl | Et | O |
| D-1154 | Me | CH₂ | H | *c*-Pr | CH₂OMe | Cl | Et | O |
| D-1155 | H | CH(Me) | H | *c*-Pr | CH₂OMe | Cl | Et | O |
| D-1156 | H | CH₂CH₂ | Me | *c*-Pr | CH₂OMe | Cl | Et | O |
| D-1157 | Me | CH₂CH₂ | H | *c*-Pr | CH₂OMe | Cl | Et | O |
| D-1158 | H | single bond | Me | *c*-Pr | CH₂OMe | Me | Et | O |
| D-1159 | Me | single bond | H | *c*-Pr | CH₂OMe | Me | Et | O |
| D-1160 | Me(R) | single bond | H | *c*-Pr | CH₂OMe | Me | Et | O |
| D-1161 | Me(S) | single bond | H | *c*-Pr | CH₂OMe | Me | Et | O |
| D-1162 | H | CH₂ | Me | *c*-Pr | CH₂OMe | Me | Et | O |
| D-1163 | Me | CH₂ | H | *c*-Pr | CH₂OMe | Me | Et | O |
| D-1164 | H | CH(Me) | H | *c*-Pr | CH₂OMe | Me | Et | O |
| D-1165 | H | CH₂CH₂ | Me | *c*-Pr | CH₂OMe | Me | Et | O |
| D-1166 | Me | CH₂CH₂ | H | *c*-Pr | CH₂OMe | Me | Et | O |
| D-1167 | H | single bond | Me | *c*-Pr | CH₂OMe | Cl | CH₂CF₃ | O |
| D-1168 | Me | single bond | H | *c*-Pr | CH₂OMe | Cl | CH₂CF₃ | O |
| D-1169 | Me(R) | single bond | H | *c*-Pr | CH₂OMe | Cl | CH₂CF₃ | O |
| D-1170 | Me(S) | single bond | H | *c*-Pr | CH₂OMe | Cl | CH₂CF₃ | O |
| D-1171 | H | CH₂ | Me | *c*-Pr | CH₂OMe | Cl | CH₂CF₃ | O |
| D-1172 | Me | CH₂ | H | *c*-Pr | CH₂OMe | Cl | CH₂CF₃ | O |
| D-1173 | H | CH(Me) | H | *c*-Pr | CH₂OMe | Cl | CH₂CF₃ | O |
| D-1174 | H | CH₂CH₂ | Me | *c*-Pr | CH₂OMe | Cl | CH₂CF₃ | O |
| D-1175 | Me | CH₂CH₂ | H | *c*-Pr | CH₂OMe | Cl | CH₂CF₃ | O |
| D-1176 | H | single bond | Me | *c*-Pr | CH₂OMe | Me | CH₂CF₃ | O |
| D-1177 | Me | single bond | H | *c*-Pr | CH₂OMe | Me | CH₂CF₃ | O |
| D-1178 | Me(R) | single bond | H | *c*-Pr | CH₂OMe | Me | CH₂CF₃ | O |
| D-1179 | Me(S) | single bond | H | *c*-Pr | CH₂OMe | Me | CH₂CF₃ | O |
| D-1180 | H | CH₂ | Me | *c*-Pr | CH₂OMe | Me | CH₂CF₃ | O |
| D-1181 | Me | CH₂ | H | *c*-Pr | CH₂OMe | Me | CH₂CF₃ | O |
| D-1182 | H | CH(Me) | H | *c*-Pr | CH₂OMe | Me | CH₂CF₃ | O |

**[Table 672]**

| Compound Number | Y | W | Z | R¹ | R² | R³ | R¹⁰ | A |
|---|---|---|---|---|---|---|---|---|
| D-1183 | H | CH₂CH₂ | Me | *c*-Pr | CH₂OMe | Me | CH₂CF₃ | O |
| D-1184 | Me | CH₂CH₂ | H | *c*-Pr | CH₂OMe | Me | CH₂CF₃ | O |
| D-1185 | H | single bond | Me | *c*-Pr | CH₂OMe | Cl | CH₂OMe | O |
| D-1186 | Me | single bond | H | *c*-Pr | CH₂OMe | Cl | CH₂OMe | O |
| D-1187 | Me(R) | single bond | H | *c*-Pr | CH₂OMe | Cl | CH₂OMe | O |
| D-1188 | Me(S) | single bond | H | *c*-Pr | CH₂OMe | Cl | CH₂OMe | O |
| D-1189 | H | CH₂ | Me | *c*-Pr | CH₂OMe | Cl | CH₂OMe | O |
| D-1190 | Me | CH₂ | H | *c*-Pr | CH₂OMe | Cl | CH₂OMe | O |
| D-1191 | H | CH(Me) | H | *c*-Pr | CH₂OMe | Cl | CH₂OMe | O |
| D-1192 | H | CH₂CH₂ | Me | *c*-Pr | CH₂OMe | Cl | CH₂OMe | O |
| D-1193 | Me | CH₂CH₂ | H | *c*-Pr | CH₂OMe | Cl | CH₂OMe | O |
| D-1194 | H | single bond | Me | *c*-Pr | CH₂OMe | Me | CH₂OMe | O |
| D-1195 | Me | single bond | H | *c*-Pr | CH₂OMe | Me | CH₂OMe | O |
| D-1196 | Me(R) | single bond | H | *c*-Pr | CH₂OMe | Me | CH₂OMe | O |
| D-1197 | Me(S) | single bond | H | *c*-Pr | CH₂OMe | Me | CH₂OMe | O |
| D-1198 | H | CH₂ | Me | *c*-Pr | CH₂OMe | Me | CH₂OMe | O |
| D-1199 | Me | CH₂ | H | *c*-Pr | CH₂OMe | Me | CH₂OMe | O |
| D-1200 | H | CH(Me) | H | *c*-Pr | CH₂OMe | Me | CH₂OMe | O |
| D-1201 | H | CH₂CH₂ | Me | *c*-Pr | CH₂OMe | Me | CH₂OMe | O |
| D-1202 | Me | CH₂CH₂ | H | *c*-Pr | CH₂OMe | Me | CH₂OMe | O |
| D-1203 | H | single bond | Me | *c*-Pr | CH₂OMe | Cl | CH₂CN | O |
| D-1204 | Me | single bond | H | *c*-Pr | CH₂OMe | Cl | CH₂CN | O |
| D-1205 | Me(R) | single bond | H | *c*-Pr | CH₂OMe | Cl | CH₂CN | O |
| D-1206 | Me(S) | single bond | H | *c*-Pr | CH₂OMe | Cl | CH₂CN | O |
| D-1207 | H | CH₂ | Me | *c*-Pr | CH₂OMe | Cl | CH₂CN | O |
| D-1208 | Me | CH₂ | H | *c*-Pr | CH₂OMe | Cl | CH₂CN | O |
| D-1209 | H | CH(Me) | H | *c*-Pr | CH₂OMe | Cl | CH₂CN | O |
| D-1210 | H | CH₂CH₂ | Me | *c*-Pr | CH₂OMe | Cl | CH₂CN | O |
| D-1211 | Me | CH₂CH₂ | H | *c*-Pr | CH₂OMe | Cl | CH₂CN | O |
| D-1212 | H | single bond | Me | *c*-Pr | CH₂OMe | Me | CH₂CN | O |
| D-1213 | Me | single bond | H | *c*-Pr | CH₂OMe | Me | CH₂CN | O |
| D-1214 | Me(R) | single bond | H | *c*-Pr | CH₂OMe | Me | CH₂CN | O |
| D-1215 | Me(S) | single bond | H | *c*-Pr | CH₂OMe | Me | CH₂CN | O |
| D-1216 | H | CH₂ | Me | *c*-Pr | CH₂OMe | Me | CH₂CN | O |
| D-1217 | Me | CH₂ | H | *c*-Pr | CH₂OMe | Me | CH₂CN | O |
| D-1218 | H | CH(Me) | H | *c*-Pr | CH₂OMe | Me | CH₂CN | O |
| D-1219 | H | CH₂CH₂ | Me | *c*-Pr | CH₂OMe | Me | CH₂CN | O |
| D-1220 | Me | CH₂CH₂ | H | *c*-Pr | CH₂OMe | Me | CH₂CN | O |
| D-1221 | H | single bond | Me | *c*-Pr | CH₂OMe | Cl | C(=O)Me | O |
| D-1222 | Me | single bond | H | *c*-Pr | CH₂OMe | Cl | C(=O)Me | O |
| D-1223 | Me(R) | single bond | H | *c*-Pr | CH₂OMe | Cl | C(=O)Me | O |
| D-1224 | Me(S) | single bond | H | *c*-Pr | CH₂OMe | Cl | C(=O)Me | O |
| D-1225 | H | CH₂ | Me | *c*-Pr | CH₂OMe | Cl | C(=O)Me | O |
| D-1226 | Me | CH₂ | H | *c*-Pr | CH₂OMe | Cl | C(=O)Me | O |

**[Table 673]**

| Compound Number | Y | W | Z | R¹ | R² | R³ | R¹⁰ | A |
|---|---|---|---|---|---|---|---|---|
| D-1227 | H | CH(Me) | H | *c*-Pr | CH₂OMe | Cl | C(=O)Me | O |
| D-1228 | H | CH₂CH₂ | Me | *c*-Pr | CH₂OMe | Cl | C(=O)Me | O |
| D-1229 | Me | CH₂CH₂ | H | *c*-Pr | CH₂OMe | Cl | C(=O)Me | O |
| D-1230 | H | single bond | Me | *c*-Pr | CH₂OMe | Me | C(=O)Me | O |
| D-1231 | Me | single bond | H | *c*-Pr | CH₂OMe | Me | C(=O)Me | O |
| D-1232 | Me(R) | single bond | H | *c*-Pr | CH₂OMe | Me | C(=O)Me | O |
| D-1233 | Me(S) | single bond | H | *c*-Pr | CH₂OMe | Me | C(=O)Me | O |
| D-1234 | H | CH₂ | Me | *c*-Pr | CH₂OMe | Me | C(=O)Me | O |
| D-1235 | Me | CH₂ | H | *c*-Pr | CH₂OMe | Me | C(=O)Me | O |
| D-1236 | H | CH(Me) | H | *c*-Pr | CH₂OMe | Me | C(=O)Me | O |
| D-1237 | H | CH₂CH₂ | Me | *c*-Pr | CH₂OMe | Me | C(=O)Me | O |
| D-1238 | Me | CH₂CH₂ | H | *c*-Pr | CH₂OMe | Me | C(=O)Me | O |
| D-1239 | H | single bond | Me | *c*-Pr | CH₂CN | Cl | Me | O |
| D-1240 | H | single bond | Et | *c*-Pr | CH₂CN | Cl | Me | O |
| D-1241 | Me | single bond | H | *c*-Pr | CH₂CN | Cl | Me | O |
| D-1242 | Me(R) | single bond | H | *c*-Pr | CH₂CN | Cl | Me | O |
| D-1243 | Me(S) | single bond | H | *c*-Pr | CH₂CN | Cl | Me | O |
| D-1244 | H | CH₂ | Me | *c*-Pr | CH₂CN | Cl | Me | O |
| D-1245 | Me | CH₂ | H | *c*-Pr | CH₂CN | Cl | Me | O |
| D-1246 | H | CH(Me) | H | *c*-Pr | CH₂CN | Cl | Me | O |
| D-1247 | H | CH₂CH₂ | Me | *c*-Pr | CH₂CN | Cl | Me | O |
| D-1248 | Me | CH₂CH₂ | H | *c*-Pr | CH₂CN | Cl | Me | O |
| D-1249 | H | single bond | Me | *c*-Pr | CH₂CN | Me | Me | O |
| D-1250 | Me | single bond | H | *c*-Pr | CH₂CN | Me | Me | O |
| D-1251 | Me(R) | single bond | H | *c*-Pr | CH₂CN | Me | Me | O |
| D-1252 | Me(S) | single bond | H | *c*-Pr | CH₂CN | Me | Me | O |
| D-1253 | H | CH₂ | Me | *c*-Pr | CH₂CN | Me | Me | O |
| D-1254 | Me | CH₂ | H | *c*-Pr | CH₂CN | Me | Me | O |
| D-1255 | H | CH(Me) | H | *c*-Pr | CH₂CN | Me | Me | O |
| D-1256 | H | CH₂CH₂ | Me | *c*-Pr | CH₂CN | Me | Me | O |
| D-1257 | Me | CH₂CH₂ | H | *c*-Pr | CH₂CN | Me | Me | O |
| D-1258 | H | single bond | Me | *c*-Pr | CH₂CN | Cl | Et | O |
| D-1259 | Me | single bond | H | *c*-Pr | CH₂CN | Cl | Et | O |
| D-1260 | Me(R) | single bond | H | *c*-Pr | CH₂CN | Cl | Et | O |
| D-1261 | Me(S) | single bond | H | *c*-Pr | CH₂CN | Cl | Et | O |
| D-1262 | H | CH₂ | Me | *c*-Pr | CH₂CN | Cl | Et | O |
| D-1263 | Me | CH₂ | H | *c*-Pr | CH₂CN | Cl | Et | O |
| D-1264 | H | CH(Me) | H | *c*-Pr | CH₂CN | Cl | Et | O |
| D-1265 | H | CH₂CH₂ | Me | *c*-Pr | CH₂CN | Cl | Et | O |
| D-1266 | Me | CH₂CH₂ | H | *c*-Pr | CH₂CN | Cl | Et | O |
| D-1267 | H | single bond | Me | *c*-Pr | CH₂CN | Me | Et | O |
| D-1268 | Me | single bond | H | *c*-Pr | CH₂CN | Me | Et | O |
| D-1269 | Me(R) | single bond | H | *c*-Pr | CH₂CN | Me | Et | O |
| D-1270 | Me(S) | single bond | H | *c*-Pr | CH₂CN | Me | Et | O |

**[Table 674]**

| Compound Number | Y | W | Z | R¹ | R² | R³ | R¹⁰ | A |
|---|---|---|---|---|---|---|---|---|
| D-1271 | H | CH₂ | Me | *c*-Pr | CH₂CN | Me | Et | O |
| D-1272 | Me | CH₂ | H | *c*-Pr | CH₂CN | Me | Et | O |
| D-1273 | H | CH(Me) | H | *c*-Pr | CH₂CN | Me | Et | O |
| D-1274 | H | CH₂CH₂ | Me | *c*-Pr | CH₂CN | Me | Et | O |
| D-1275 | Me | CH₂CH₂ | H | *c*-Pr | CH₂CN | Me | Et | O |
| D-1276 | H | single bond | Me | *c*-Pr | CH₂CN | Cl | CH₂CF₃ | O |
| D-1277 | Me | single bond | H | *c*-Pr | CH₂CN | Cl | CH₂CF₃ | O |
| D-1278 | Me(R) | single bond | H | *c*-Pr | CH₂CN | Cl | CH₂CF₃ | O |
| D-1279 | Me(S) | single bond | H | *c*-Pr | CH₂CN | Cl | CH₂CF₃ | O |
| D-1280 | H | CH₂ | Me | *c*-Pr | CH₂CN | Cl | CH₂CF₃ | O |
| D-1281 | Me | CH₂ | H | *c*-Pr | CH₂CN | Cl | CH₂CF₃ | O |
| D-1282 | H | CH(Me) | H | *c*-Pr | CH₂CN | Cl | CH₂CF₃ | O |
| D-1283 | H | CH₂CH₂ | Me | *c*-Pr | CH₂CN | Cl | CH₂CF₃ | O |
| D-1284 | Me | CH₂CH₂ | H | *c*-Pr | CH₂CN | Cl | CH₂CF₃ | O |
| D-1285 | H | single bond | Me | *c*-Pr | CH₂CN | Me | CH₂CF₃ | O |
| D-1286 | Me | single bond | H | *c*-Pr | CH₂CN | Me | CH₂CF₃ | O |
| D-1287 | Me(R) | single bond | H | *c*-Pr | CH₂CN | Me | CH₂CF₃ | O |
| D-1288 | Me(S) | single bond | H | c-Pr | CH₂CN | Me | CH₂CF₃ | O |
| D-1289 | H | CH₂ | Me | *c*-Pr | CH₂CN | Me | CH₂CF₃ | O |
| D-1290 | Me | CH₂ | H | *c*-Pr | CH₂CN | Me | CH₂CF₃ | O |
| D-1291 | H | CH(Me) | H | *c*-Pr | CH₂CN | Me | CH₂CF₃ | O |
| D-1292 | H | CH₂CH₂ | Me | *c*-Pr | CH₂CN | Me | CH₂CF₃ | O |
| D-1293 | Me | CH₂CH₂ | H | *c*-Pr | CH₂CN | Me | CH₂CF₃ | O |
| D-1294 | H | single bond | Me | *c*-Pr | CH₂CN | Cl | CH₂OMe | O |
| D-1295 | Me | single bond | H | *c*-Pr | CH₂CN | Cl | CH₂OMe | O |
| D-1296 | Me(R) | single bond | H | *c*-Pr | CH₂CN | Cl | CH₂OMe | O |
| D-1297 | Me(S) | single bond | H | *c*-Pr | CH₂CN | Cl | CH₂OMe | O |
| D-1298 | H | CH₂ | Me | *c*-Pr | CH₂CN | Cl | CH₂OMe | O |
| D-1299 | Me | CH₂ | H | *c*-Pr | CH₂CN | Cl | CH₂OMe | O |
| D-1300 | H | CH(Me) | H | *c*-Pr | CH₂CN | Cl | CH₂OMe | O |
| D-1301 | H | CH₂CH₂ | Me | *c*-Pr | CH₂CN | Cl | CH₂OMe | O |
| D-1302 | Me | CH₂CH₂ | H | *c*-Pr | CH₂CN | Cl | CH₂OMe | O |
| D-1303 | H | single bond | Me | *c*-Pr | CH₂CN | Me | CH₂OMe | O |
| D-1304 | Me | single bond | H | *c*-Pr | CH₂CN | Me | CH₂OMe | O |
| D-1305 | Me(R) | single bond | H | *c*-Pr | CH₂CN | Me | CH₂OMe | O |
| D-1306 | Me(S) | single bond | H | *c*-Pr | CH₂CN | Me | CH₂OMe | O |
| D-1307 | H | CH₂ | Me | *c*-Pr | CH₂CN | Me | CH₂OMe | O |
| D-1308 | Me | CH₂ | H | c-Pr | CH₂CN | Me | CH₂OMe | O |
| D-1309 | H | CH(Me) | H | *c*-Pr | CH₂CN | Me | CH₂OMe | O |
| D-1310 | H | CH₂CH₂ | Me | *c*-Pr | CH₂CN | Me | CH₂OMe | O |
| D-1311 | Me | CH₂CH₂ | H | *c*-Pr | CH₂CN | Me | CH₂OMe | O |
| D-1312 | H | single bond | Me | *c*-Pr | CH₂CN | Cl | CH₂CN | O |
| D-1313 | Me | single bond | H | *c*-Pr | CH₂CN | Cl | CH₂CN | O |
| D-1314 | Me(R) | single bond | H | *c*-Pr | CH₂CN | Cl | CH₂CN | O |

**[Table 675]**

| Compound Number | Y | W | Z | R¹ | R² | R³ | R¹⁰ | A |
|---|---|---|---|---|---|---|---|---|
| D-1315 | Me(S) | single bond | H | *c*-Pr | CH₂CN | Cl | CH₂CN | O |
| D-1316 | H | CH₂ | Me | *c*-Pr | CH₂CN | Cl | CH₂CN | O |
| D-1317 | Me | CH₂ | H | *c*-Pr | CH₂CN | Cl | CH₂CN | O |
| D-1318 | H | CH(Me) | H | *c*-Pr | CH₂CN | Cl | CH₂CN | O |
| D-1319 | H | CH₂CH₂ | Me | *c*-Pr | CH₂CN | Cl | CH₂CN | O |
| D-1320 | Me | CH₂CH₂ | H | *c*-Pr | CH₂CN | Cl | CH₂CN | O |
| D-1321 | H | single bond | Me | *c*-Pr | CH₂CN | Me | CH₂CN | O |
| D-1322 | Me | single bond | H | *c*-Pr | CH₂CN | Me | CH₂CN | O |
| D-1323 | Me(R) | single bond | H | *c*-Pr | CH₂CN | Me | CH₂CN | O |
| D-1324 | Me(S) | single bond | H | *c*-Pr | CH₂CN | Me | CH₂CN | O |
| D-1325 | H | CH₂ | Me | *c*-Pr | CH₂CN | Me | CH₂CN | O |
| D-1326 | Me | CH₂ | H | *c*-Pr | CH₂CN | Me | CH₂CN | O |
| D-1327 | H | CH(Me) | H | *c*-Pr | CH₂CN | Me | CH₂CN | O |
| D-1328 | H | CH₂CH₂ | Me | *c*-Pr | CH₂CN | Me | CH₂CN | O |
| D-1329 | Me | CH₂CH₂ | H | *c*-Pr | CH₂CN | Me | CH₂CN | O |
| D-1330 | H | single bond | Me | *c*-Pr | CH₂CN | Cl | C(=O)Me | O |
| D-1331 | Me | single bond | H | *c*-Pr | CH₂CN | Cl | C(=O)Me | O |
| D-1332 | Me(R) | single bond | H | c-Pr | CH₂CN | Cl | C(=O)Me | O |
| D-1333 | Me(S) | single bond | H | *c*-Pr | CH₂CN | Cl | C(=O)Me | O |
| D-1334 | H | CH₂ | Me | *c*-Pr | CH₂CN | Cl | C(=O)Me | O |
| D-1335 | Me | CH₂ | H | *c*-Pr | CH₂CN | Cl | C(=O)Me | O |
| D-1336 | H | CH(Me) | H | *c*-Pr | CH₂CN | Cl | C(=O)Me | O |
| D-1337 | H | CH₂CH₂ | Me | *c*-Pr | CH₂CN | Cl | C(=O)Me | O |
| D-1338 | Me | CH₂CH₂ | H | *c*-Pr | CH₂CN | Cl | C(=O)Me | O |
| D-1339 | H | single bond | Me | *c*-Pr | CH₂CN | Me | C(=O)Me | O |
| D-1340 | Me | single bond | H | *c*-Pr | CH₂CN | Me | C(=O)Me | O |
| D-1341 | Me(R) | single bond | H | *c*-Pr | CH₂CN | Me | C(=O)Me | O |
| D-1342 | Me(S) | single bond | H | *c*-Pr | CH₂CN | Me | C(=O)Me | O |
| D-1343 | H | CH₂ | Me | *c*-Pr | CH₂CN | Me | C(=O)Me | O |
| D-1344 | Me | CH₂ | H | *c*-Pr | CH₂CN | Me | C(=O)Me | O |
| D-1345 | H | CH(Me) | H | *c*-Pr | CH₂CN | Me | C(=O)Me | O |
| D-1346 | H | CH₂CH₂ | Me | *c*-Pr | CH₂CN | Me | C(=O)Me | O |
| D-1347 | Me | CH₂CH₂ | H | *c*-Pr | CH₂CN | Me | C(=O)Me | O |
| D-1348 | H | single bond | Me | *c*-Pr | C(=O)Me | Cl | Me | O |
| D-1349 | Me | single bond | H | *c*-Pr | C(=O)Me | Cl | Me | O |
| D-1350 | Me(R) | single bond | H | *c*-Pr | C(=O)Me | Cl | Me | O |
| D-1351 | Me(S) | single bond | H | *c*-Pr | C(=O)Me | Cl | Me | O |
| D-1352 | H | CH₂ | Me | c-Pr | C(=O)Me | Cl | Me | O |
| D-1353 | Me | CH₂ | H | *c*-Pr | C(=O)Me | Cl | Me | O |
| D-1354 | H | CH(Me) | H | *c*-Pr | C(=O)Me | Cl | Me | O |
| D-1355 | H | CH₂CH₂ | Me | *c*-Pr | C(=O)Me | Cl | Me | O |
| D-1356 | Me | CH₂CH₂ | H | *c*-Pr | C(=O)Me | Cl | Me | O |
| D-1357 | H | single bond | Me | *c*-Pr | C(=O)Me | Me | Me | O |
| D-1358 | Me | single bond | H | *c*-Pr | C(=O)Me | Me | Me | O |

**[Table 676]**

| Compound Number | Y | W | Z | R¹ | R² | R³ | R¹⁰ | A |
|---|---|---|---|---|---|---|---|---|
| D-1359 | Me(R) | single bond | H | *c*-Pr | C(=O)Me | Me | Me | O |
| D-1360 | Me(S) | single bond | H | *c*-Pr | C(=O)Me | Me | Me | O |
| D-1361 | H | CH₂ | Me | *c*-Pr | C(=O)Me | Me | Me | O |
| D-1362 | Me | CH₂ | H | *c*-Pr | C(=O)Me | Me | Me | O |
| D-1363 | H | CH(Me) | H | *c*-Pr | C(=O)Me | Me | Me | O |
| D-1364 | H | CH₂CH₂ | Me | *c*-Pr | C(=O)Me | Me | Me | O |
| D-1365 | Me | CH₂CH₂ | H | *c*-Pr | C(=O)Me | Me | Me | O |
| D-1366 | H | single bond | Me | *c*-Pr | C(=O)Me | Cl | Et | O |
| D-1367 | Me | single bond | H | *c*-Pr | C(=O)Me | Cl | Et | O |
| D-1368 | Me(R) | single bond | H | *c*-Pr | C(=O)Me | Cl | Et | O |
| D-1369 | Me(S) | single bond | H | *c*-Pr | C(=O)Me | Cl | Et | O |
| D-1370 | H | CH₂ | Me | *c*-Pr | C(=O)Me | Cl | Et | O |
| D-1371 | Me | CH₂ | H | *c*-Pr | C(=O)Me | Cl | Et | O |
| D-1372 | H | CH(Me) | H | *c*-Pr | C(=O)Me | Cl | Et | O |
| D-1373 | H | CH₂CH₂ | Me | *c*-Pr | C(=O)Me | Cl | Et | O |
| D-1374 | Me | CH₂CH₂ | H | *c*-Pr | C(=O)Me | Cl | Et | O |
| D-1375 | H | single bond | Me | *c*-Pr | C(=O)Me | Me | Et | O |
| D-1376 | Me | single bond | H | c-Pr | C(=O)Me | Me | Et | O |
| D-1377 | Me(R) | single bond | H | *c*-Pr | C(=O)Me | Me | Et | O |
| D-1378 | Me(S) | single bond | H | *c*-Pr | C(=O)Me | Me | Et | O |
| D-1379 | H | CH₂ | Me | *c*-Pr | C(=O)Me | Me | Et | O |
| D-1380 | Me | CH₂ | H | *c*-Pr | C(=O)Me | Me | Et | O |
| D-1381 | H | CH(Me) | H | *c*-Pr | C(=O)Me | Me | Et | O |
| D-1382 | H | CH₂CH₂ | Me | *c*-Pr | C(=O)Me | Me | Et | O |
| D-1383 | Me | CH₂CH₂ | H | *c*-Pr | C(=O)Me | Me | Et | O |
| D-1384 | H | single bond | Me | *c*-Pr | C(=O)Me | Cl | CH₂CF₃ | O |
| D-1385 | Me | single bond | H | *c*-Pr | C(=O)Me | Cl | CH₂CF₃ | O |
| D-1386 | Me(R) | single bond | H | *c*-Pr | C(=O)Me | Cl | CH₂CF₃ | O |
| D-1387 | Me(S) | single bond | H | *c*-Pr | C(=O)Me | Cl | CH₂CF₃ | O |
| D-1388 | H | CH₂ | Me | *c*-Pr | C(=O)Me | Cl | CH₂CF₃ | O |
| D-1389 | Me | CH₂ | H | *c*-Pr | C(=O)Me | Cl | CH₂CF₃ | O |
| D-1390 | H | CH(Me) | H | *c*-Pr | C(=O)Me | Cl | CH₂CF₃ | O |
| D-1391 | H | CH₂CH₂ | Me | *c*-Pr | C(=O)Me | Cl | CH₂CF₃ | O |
| D-1392 | Me | CH₂CH₂ | H | *c*-Pr | C(=O)Me | Cl | CH₂CF₃ | O |
| D-1393 | H | single bond | Me | *c*-Pr | C(=O)Me | Me | CH₂CF₃ | O |
| D-1394 | Me | single bond | H | *c*-Pr | C(=O)Me | Me | CH₂CF₃ | O |
| D-1395 | Me(R) | single bond | H | *c*-Pr | C(=O)Me | Me | CH₂CF₃ | O |
| D-1396 | Me(S) | single bond | H | c-Pr | C(=O)Me | Me | CH₂CF₃ | O |
| D-1397 | H | CH₂ | Me | *c*-Pr | C(=O)Me | Me | CH₂CF₃ | O |
| D-1398 | Me | CH₂ | H | *c*-Pr | C(=O)Me | Me | CH₂CF₃ | O |
| D-1399 | H | CH(Me) | H | *c*-Pr | C(=O)Me | Me | CH₂CF₃ | O |
| D-1400 | H | CH₂CH₂ | Me | *c*-Pr | C(=O)Me | Me | CH₂CF₃ | O |
| D-1401 | Me | CH₂CH₂ | H | *c*-Pr | C(=O)Me | Me | CH₂CF₃ | O |
| D-1402 | H | single bond | Me | *c*-Pr | C(=O)Me | Cl | CH₂OMe | O |

**[Table 677]**

| Compound Number | Y | W | Z | R¹ | R² | R³ | R¹⁰ | A |
|---|---|---|---|---|---|---|---|---|
| D-1403 | Me | single bond | H | *c*-Pr | C(=O)Me | Cl | CH₂OMe | O |
| D-1404 | Me(R) | single bond | H | *c*-Pr | C(=O)Me | Cl | CH₂OMe | O |
| D-1405 | Me(S) | single bond | H | *c*-Pr | C(=O)Me | Cl | CH₂OMe | O |
| D-1406 | H | CH₂ | Me | *c*-Pr | C(=O)Me | Cl | CH₂OMe | O |
| D-1407 | Me | CH₂ | H | *c*-Pr | C(=O)Me | Cl | CH₂OMe | O |
| D-1408 | H | CH(Me) | H | *c*-Pr | C(=O)Me | Cl | CH₂OMe | O |
| D-1409 | H | CH₂CH₂ | Me | *c*-Pr | C(=O)Me | Cl | CH₂OMe | O |
| D-1410 | Me | CH₂CH₂ | H | *c*-Pr | C(=O)Me | Cl | CH₂OMe | O |
| D-1411 | H | single bond | Me | *c*-Pr | C(=O)Me | Me | CH₂OMe | O |
| D-1412 | Me | single bond | H | *c*-Pr | C(=O)Me | Me | CH₂OMe | O |
| D-1413 | Me(R) | single bond | H | *c*-Pr | C(=O)Me | Me | CH₂OMe | O |
| D-1414 | Me(S) | single bond | H | *c*-Pr | C(=O)Me | Me | CH₂OMe | O |
| D-1415 | H | CH₂ | Me | *c*-Pr | C(=O)Me | Me | CH₂OMe | O |
| D-1416 | Me | CH₂ | H | *c*-Pr | C(=O)Me | Me | CH₂OMe | O |
| D-1417 | H | CH(Me) | H | *c*-Pr | C(=O)Me | Me | CH₂OMe | O |
| D-1418 | H | CH₂CH₂ | Me | *c*-Pr | C(=O)Me | Me | CH₂OMe | O |
| D-1419 | Me | CH₂CH₂ | H | *c*-Pr | C(=O)Me | Me | CH₂OMe | O |
| D-1420 | H | single bond | Me | c-Pr | C(=O)Me | Cl | CH₂CN | O |
| D-1421 | Me | single bond | H | *c*-Pr | C(=O)Me | Cl | CH₂CN | O |
| D-1422 | Me(R) | single bond | H | *c*-Pr | C(=O)Me | Cl | CH₂CN | O |
| D-1423 | Me(S) | single bond | H | *c*-Pr | C(=O)Me | Cl | CH₂CN | O |
| D-1424 | H | CH₂ | Me | *c*-Pr | C(=O)Me | Cl | CH₂CN | O |
| D-1425 | Me | CH₂ | H | *c*-Pr | C(=O)Me | Cl | CH₂CN | O |
| D-1426 | H | CH(Me) | H | *c*-Pr | C(=O)Me | Cl | CH₂CN | O |
| D-1427 | H | CH₂CH₂ | Me | *c*-Pr | C(=O)Me | Cl | CH₂CN | O |
| D-1428 | Me | CH₂CH₂ | H | *c*-Pr | C(=O)Me | Cl | CH₂CN | O |
| D-1429 | H | single bond | Me | *c*-Pr | C(=O)Me | Me | CH₂CN | O |
| D-1430 | Me | single bond | H | *c*-Pr | C(=O)Me | Me | CH₂CN | O |
| D-1431 | Me(R) | single bond | H | *c*-Pr | C(=O)Me | Me | CH₂CN | O |
| D-1432 | Me(S) | single bond | H | *c*-Pr | C(=O)Me | Me | CH₂CN | O |
| D-1433 | H | CH₂ | Me | *c*-Pr | C(=O)Me | Me | CH₂CN | O |
| D-1434 | Me | CH₂ | H | *c*-Pr | C(=O)Me | Me | CH₂CN | O |
| D-1435 | H | CH(Me) | H | *c*-Pr | C(=O)Me | Me | CH₂CN | O |
| D-1436 | H | CH₂CH₂ | Me | *c*-Pr | C(=O)Me | Me | CH₂CN | O |
| D-1437 | Me | CH₂CH₂ | H | *c*-Pr | C(=O)Me | Me | CH₂CN | O |
| D-1438 | H | single bond | Me | *c*-Pr | C(=O)Me | Cl | C(=O)Me | O |
| D-1439 | Me | single bond | H | *c*-Pr | C(=O)Me | Cl | C(=O)Me | O |
| D-1440 | Me(R) | single bond | H | c-Pr | C(=O)Me | Cl | C(=O)Me | O |
| D-1441 | Me(S) | single bond | H | *c*-Pr | C(=O)Me | Cl | C(=O)Me | O |
| D-1442 | H | CH₂ | Me | *c*-Pr | C(=O)Me | Cl | C(=O)Me | O |
| D-1443 | Me | CH₂ | H | *c*-Pr | C(=O)Me | Cl | C(=O)Me | O |
| D-1444 | H | CH(Me) | H | *c*-Pr | C(=O)Me | Cl | C(=O)Me | O |
| D-1445 | H | CH₂CH₂ | Me | *c*-Pr | C(=O)Me | Cl | C(=O)Me | O |
| D-1446 | Me | CH₂CH₂ | H | *c*-Pr | C(=O)Me | Cl | C(=O)Me | O |

**[Table 678]**

| Compound Number | Y | W | Z | R¹ | R² | R³ | R¹⁰ | A |
|---|---|---|---|---|---|---|---|---|
| D-1447 | H | single bond | Me | *c*-Pr | C(=O)Me | Me | C(=O)Me | O |
| D-1448 | Me | single bond | H | *c*-Pr | C(=O)Me | Me | C(=O)Me | O |
| D-1449 | Me(R) | single bond | H | *c*-Pr | C(=O)Me | Me | C(=O)Me | O |
| D-1450 | Me(S) | single bond | H | *c*-Pr | C(=O)Me | Me | C(=O)Me | O |
| D-1451 | H | CH₂ | Me | *c*-Pr | C(=O)Me | Me | C(=O)Me | O |
| D-1452 | Me | CH₂ | H | *c*-Pr | C(=O)Me | Me | C(=O)Me | O |
| D-1453 | H | CH(Me) | H | *c*-Pr | C(=O)Me | Me | C(=O)Me | O |
| D-1454 | H | CH₂CH₂ | Me | *c*-Pr | C(=O)Me | Me | C(=O)Me | O |
| D-1455 | Me | CH₂CH₂ | H | *c*-Pr | C(=O)Me | Me | C(=O)Me | O |
| D-1456 | H | single bond | Me | *c*-Pr | C(=O)(*i*-Pr) | Cl | Me | O |
| D-1457 | Me | single bond | H | *c*-Pr | C(=O)(*i*-Pr) | Cl | Me | O |
| D-1458 | Me(R) | single bond | H | *c*-Pr | C(=O)(*i*-Pr) | Cl | Me | O |
| D-1459 | Me(S) | single bond | H | *c*-Pr | C(=O)(*i*-Pr) | Cl | Me | O |
| D-1460 | H | CH₂ | Me | *c*-Pr | C(=O)(*i*-Pr) | Cl | Me | O |
| D-1461 | Me | CH₂ | H | *c*-Pr | C(=O)(*i*-Pr) | Cl | Me | O |
| D-1462 | H | CH(Me) | H | *c*-Pr | C(=O)(*i*-Pr) | Cl | Me | O |
| D-1463 | H | CH₂CH₂ | Me | *c*-Pr | C(=O)(*i*-Pr) | Cl | Me | O |
| D-1464 | Me | CH₂CH₂ | H | c-Pr | C(=O)(*i*-Pr) | Cl | Me | O |
| D-1465 | H | single bond | Me | *c*-Pr | C(=O)(*i*-Pr) | Me | Me | O |
| D-1466 | Me | single bond | H | *c*-Pr | C(=O)(*i*-Pr) | Me | Me | O |
| D-1467 | Me(R) | single bond | H | *c*-Pr | C(=O)(*i*-Pr) | Me | Me | O |
| D-1468 | Me(S) | single bond | H | *c*-Pr | C(=O)(*i*-Pr) | Me | Me | O |
| D-1469 | H | CH₂ | Me | *c*-Pr | C(=O)(*i*-Pr) | Me | Me | O |
| D-1470 | Me | CH₂ | H | *c*-Pr | C(=O)(*i*-Pr) | Me | Me | O |
| D-1471 | H | CH(Me) | H | *c*-Pr | C(=O)(*i*-Pr) | Me | Me | O |
| D-1472 | H | CH₂CH₂ | Me | *c*-Pr | C(=O)(*i*-Pr) | Me | Me | O |
| D-1473 | Me | CH₂CH₂ | H | *c*-Pr | C(=O)(*i*-Pr) | Me | Me | O |
| D-1474 | H | single bond | Me | *c*-Pr | C(=O)(*i*-Pr) | Cl | Et | O |
| D-1475 | Me | single bond | H | *c*-Pr | C(=O)(*i*-Pr) | Cl | Et | O |
| D-1476 | Me(R) | single bond | H | *c*-Pr | C(=O)(*i*-Pr) | Cl | Et | O |
| D-1477 | Me(S) | single bond | H | *c*-Pr | C(=O)(*i*-Pr) | Cl | Et | O |
| D-1478 | H | CH₂ | Me | *c*-Pr | C(=O)(*i*-Pr) | Cl | Et | O |
| D-1479 | Me | CH₂ | H | *c*-Pr | C(=O)(*i*-Pr) | Cl | Et | O |
| D-1480 | H | CH(Me) | H | *c*-Pr | C(=O)(*i*-Pr) | Cl | Et | O |
| D-1481 | H | CH₂CH₂ | Me | *c*-Pr | C(=O)(*i*-Pr) | Cl | Et | O |
| D-1482 | Me | CH₂CH₂ | H | *c*-Pr | C(=O)(*i*-Pr) | Cl | Et | O |
| D-1483 | H | single bond | Me | *c*-Pr | C(=O)(*i*-Pr) | Me | Et | O |
| D-1484 | Me | single bond | H | c-Pr | C(=O)(*i*-Pr) | Me | Et | O |
| D-1485 | Me(R) | single bond | H | *c*-Pr | C(=O)(*i*-Pr) | Me | Et | O |
| D-1486 | Me(S) | single bond | H | *c*-Pr | C(=O)(*i*-Pr) | Me | Et | O |
| D-1487 | H | CH₂ | Me | *c*-Pr | C(=O)(*i*-Pr) | Me | Et | O |
| D-1488 | Me | CH₂ | H | *c*-Pr | C(=O)(*i*-Pr) | Me | Et | O |
| D-1489 | H | CH(Me) | H | *c*-Pr | C(=O)(*i*-Pr) | Me | Et | O |
| D-1490 | H | CH₂CH₂ | Me | *c*-Pr | C(=O)(*i*-Pr) | Me | Et | O |

**[Table 679]**

| Compound Number | Y | W | Z | R¹ | R² | R³ | R¹⁰ | A |
|---|---|---|---|---|---|---|---|---|
| D-1491 | Me | CH₂CH₂ | H | *c*-Pr | C(=O)(*i*-Pr) | Me | Et | O |
| D-1492 | H | single bond | Me | *c*-Pr | C(=O)(*i*-Pr) | Cl | CH₂CF₃ | O |
| D-1493 | Me | single bond | H | *c*-Pr | C(=O)(*i*-Pr) | Cl | CH₂CF₃ | O |
| D-1494 | Me(R) | single bond | H | *c*-Pr | C(=O)(*i*-Pr) | Cl | CH₂CF₃ | O |
| D-1495 | Me(S) | single bond | H | *c*-Pr | C(=O)(*i*-Pr) | Cl | CH₂CF₃ | O |
| D-1496 | H | CH₂ | Me | *c*-Pr | C(=O)(*i*-Pr) | Cl | CH₂CF₃ | O |
| D-1497 | Me | CH₂ | H | *c*-Pr | C(=O)(*i*-Pr) | Cl | CH₂CF₃ | O |
| D-1498 | H | CH(Me) | H | *c*-Pr | C(=O)(*i*-Pr) | Cl | CH₂CF₃ | O |
| D-1499 | H | CH₂CH₂ | Me | *c*-Pr | C(=O)(*i*-Pr) | Cl | CH₂CF₃ | O |
| D-1500 | Me | CH₂CH₂ | H | *c*-Pr | C(=O)(*i*-Pr) | Cl | CH₂CF₃ | O |
| D-1501 | H | single bond | Me | *c*-Pr | C(=O)(*i*-Pr) | Me | CH₂CF₃ | O |
| D-1502 | Me | single bond | H | *c*-Pr | C(=O)(*i*-Pr) | Me | CH₂CF₃ | O |
| D-1503 | Me(R) | single bond | H | *c*-Pr | C(=O)(*i-*Pr) | Me | CH₂CF₃ | O |
| D-1504 | Me(S) | single bond | H | *c*-Pr | C(=O)(*i*-Pr) | Me | CH₂CF₃ | O |
| D-1505 | H | CH₂ | Me | *c*-Pr | C(=O)(*i*-Pr) | Me | CH₂CF₃ | O |
| D-1506 | Me | CH₂ | H | *c*-Pr | C(=O)(*i*-Pr) | Me | CH₂CF₃ | O |
| D-1507 | H | CH(Me) | H | *c*-Pr | C(=O)(*i*-Pr) | Me | CH₂CF₃ | O |
| D-1508 | H | CH₂CH₂ | Me | c-Pr | C(=O)(*i*-Pr) | Me | CH₂CF₃ | O |
| D-1509 | Me | CH₂CH₂ | H | *c*-Pr | C(=O)(*i*-Pr) | Me | CH₂CF₃ | O |
| D-1510 | H | single bond | Me | *c*-Pr | C(=O)(*i*-Pr) | Cl | CH₂OMe | O |
| D-1511 | Me | single bond | H | *c*-Pr | C(=O)(*i*-Pr) | Cl | CH₂OMe | O |
| D-1512 | Me(R) | single bond | H | *c*-Pr | C(=O)(*i*-Pr) | Cl | CH₂OMe | O |
| D-1513 | Me(S) | single bond | H | *c*-Pr | C(=O)(*i*-Pr) | Cl | CH₂OMe | O |
| D-1514 | H | CH₂ | Me | *c*-Pr | C(=O)(*i*-Pr) | Cl | CH₂OMe | O |
| D-1515 | Me | CH₂ | H | *c*-Pr | C(=O)(*i*-Pr) | Cl | CH₂OMe | O |
| D-1516 | H | CH(Me) | H | *c*-Pr | C(=O)(*i*-Pr) | Cl | CH₂OMe | O |
| D-1517 | H | CH₂CH₂ | Me | *c*-Pr | C(=O)(*i*-Pr) | Cl | CH₂OMe | O |
| D-1518 | Me | CH₂CH₂ | H | *c*-Pr | C(=O)(*i*-Pr) | Cl | CH₂OMe | O |
| D-1519 | H | single bond | Me | *c*-Pr | C(=O)(*i*-Pr) | Me | CH₂OMe | O |
| D-1520 | Me | single bond | H | *c*-Pr | C(=O)(*i*-Pr) | Me | CH₂OMe | O |
| D-1521 | Me(R) | single bond | H | *c*-Pr | C(=O)(*i*-Pr) | Me | CH₂OMe | O |
| D-1522 | Me(S) | single bond | H | *c*-Pr | C(=O)(*i*-Pr) | Me | CH₂OMe | O |
| D-1523 | H | CH₂ | Me | *c*-Pr | C(=O)(*i*-Pr) | Me | CH₂OMe | O |
| D-1524 | Me | CH₂ | H | *c*-Pr | C(=O)(*i*-Pr) | Me | CH₂OMe | O |
| D-1525 | H | CH(Me) | H | *c*-Pr | C(=O)(*i*-Pr) | Me | CH₂OMe | O |
| D-1526 | H | CH₂CH₂ | Me | *c*-Pr | C(=O)(*i*-Pr) | Me | CH₂OMe | O |
| D-1527 | Me | CH₂CH₂ | H | *c*-Pr | C(=O)(*i*-Pr) | Me | CH₂OMe | O |
| D-1528 | H | single bond | Me | c-Pr | C(=O)(*i*-Pr) | Cl | CH₂CN | O |
| D-1529 | Me | single bond | H | *c*-Pr | C(=O)(*i*-Pr) | Cl | CH₂CN | O |
| D-1530 | Me(R) | single bond | H | *c*-Pr | C(=O)(*i*-Pr) | Cl | CH₂CN | O |
| D-1531 | Me(S) | single bond | H | *c*-Pr | C(=O)(*i*-Pr) | Cl | CH₂CN | O |
| D-1532 | H | CH₂ | Me | *c*-Pr | C(=O)(*i*-Pr) | Cl | CH₂CN | O |
| D-1533 | Me | CH₂ | H | *c*-Pr | C(=O)(*i*-Pr) | Cl | CH₂CN | O |
| D-1534 | H | CH(Me) | H | *c*-Pr | C(=O)(*i-*Pr) | Cl | CH₂CN | O |

**[Table 680]**

| Compound Number | Y | w | Z | R¹ | R² | R³ | R¹⁰ | A |
|---|---|---|---|---|---|---|---|---|
| D-1535 | H | CH₂CH₂ | Me | *c*-Pr | C(=O)(*i*-Pr) | Cl | CH₂CN | O |
| D-1536 | Me | CH₂CH₂ | H | *c*-Pr | C(=O)(*i*-Pr) | Cl | CH₂CN | O |
| D-1537 | H | single bond | Me | *c*-Pr | C(=O)(*i*-Pr) | Me | CH₂CN | O |
| D-1538 | Me | single bond | H | *c*-Pr | C(=O)(*i*-Pr) | Me | CH₂CN | O |
| D-1539 | Me(R) | single bond | H | *c*-Pr | C(=O)(*i*-Pr) | Me | CH₂CN | O |
| D-1540 | Me(S) | single bond | H | *c*-Pr | C(=O)(*i*-Pr) | Me | CH₂CN | O |
| D-1541 | H | CH₂ | Me | *c*-Pr | C(=O)(*i*-Pr) | Me | CH₂CN | O |
| D-1542 | Me | CH₂ | H | *c*-Pr | C(=O)(*i*-Pr) | Me | CH₂CN | O |
| D-1543 | H | CH(Me) | H | *c*-Pr | C(=O)(*i*-Pr) | Me | CH₂CN | O |
| D-1544 | H | CH₂CH₂ | Me | *c*-Pr | C(=O)(*i*-Pr) | Me | CH₂CN | O |
| D-1545 | Me | CH₂CH₂ | H | *c*-Pr | C(=O)(*i*-Pr) | Me | CH₂CN | O |
| D-1546 | H | single bond | Me | *c*-Pr | C(=O)(*i*-Pr) | Cl | C(=O)Me | O |
| D-1547 | Me | single bond | H | *c*-Pr | C(=O)(*i*-Pr) | Cl | C(=O)Me | O |
| D-1548 | Me(R) | single bond | H | *c-*Pr | C(=O)(*i*-Pr) | Cl | C(=O)Me | O |
| D-1549 | Me(S) | single bond | H | *c*-Pr | C(=O)(*i*-Pr) | Cl | C(=O)Me | O |
| D-1550 | H | CH₂ | Me | *c*-Pr | C(=O)(*i*-Pr) | Cl | C(=O)Me | O |
| D-1551 | Me | CH₂ | H | *c*-Pr | C(=O)(*i*-Pr) | Cl | C(=O)Me | O |
| D-1552 | H | CH(Me) | H | *c*-Pr | C(=O)(*i*-Pr) | Cl | C(=O)Me | O |
| D-1553 | H | CH₂CH₂ | Me | *c*-Pr | C(=O)(*i*-Pr) | Cl | C(=O)Me | O |
| D-1554 | Me | CH₂CH₂ | H | *c*-Pr | C(=O)(*i*-Pr) | Cl | C(=O)Me | O |
| D-1555 | H | single bond | Me | *c*-Pr | C(=O)(*i*-Pr) | Me | C(=O)Me | O |
| D-1556 | Me | single bond | H | *c*-Pr | C(=O)(*i*-Pr) | Me | C(=O)Me | O |
| D-1557 | Me(R) | single bond | H | *c*-Pr | C(=O)(*i*-Pr) | Me | C(=O)Me | O |
| D-1558 | Me(S) | single bond | H | *c*-Pr | C(=O)(*i*-Pr) | Me | C(=O)Me | O |
| D-1559 | H | CH₂ | Me | *c*-Pr | C(=O)(*i*-Pr) | Me | C(=O)Me | O |
| D-1560 | Me | CH₂ | H | *c*-Pr | C(=O)(*i*-Pr) | Me | C(=O)Me | O |
| D-1561 | H | CH(Me) | H | *c*-Pr | C(=O)(*i*-Pr) | Me | C(=O)Me | O |
| D-1562 | H | CH₂CH₂ | Me | *c*-Pr | C(=O)(*i*-Pr) | Me | C(=O)Me | O |
| D-1563 | Me | CH₂CH₂ | H | *c*-Pr | C(=O)(*i*-Pr) | Me | C(=O)Me | O |
| D-1564 | H | single bond | Me | *c*-Pr | C(=O)Ph | Cl | Me | O |
| D-1565 | Me | single bond | H | *c*-Pr | C(=O)Ph | Cl | Me | O |
| D-1566 | Me(R) | single bond | H | *c*-Pr | C(=O)Ph | Cl | Me | O |
| D-1567 | Me(S) | single bond | H | *c*-Pr | C(=O)Ph | Cl | Me | O |
| D-1568 | H | CH₂ | Me | *c*-Pr | C(=O)Ph | Cl | Me | O |
| D-1569 | Me | CH₂ | H | *c*-Pr | C(=O)Ph | Cl | Me | O |
| D-1570 | H | CH(Me) | H | *c*-Pr | C(=O)Ph | Cl | Me | O |
| D-1571 | H | CH₂CH₂ | Me | *c*-Pr | C(=O)Ph | Cl | Me | O |
| D-1572 | Me | CH₂CH₂ | H | *c*-Pr | C(=O)Ph | Cl | Me | O |
| D-1573 | H | single bond | Me | *c*-Pr | C(=O)Ph | Me | Me | O |
| D-1574 | Me | single bond | H | *c*-Pr | C(=O)Ph | Me | Me | O |
| D-1575 | Me(R) | single bond | H | *c*-Pr | C(=O)Ph | Me | Me | O |
| D-1576 | Me(S) | single bond | H | *c*-Pr | C(=O)Ph | Me | Me | O |
| D-1577 | H | CH₂ | Me | *c*-Pr | C(=O)Ph | Me | Me | O |
| D-1578 | Me | CH₂ | H | *c*-Pr | C(=O)Ph | Me | Me | O |

**[Table 681]**

| Compound Number | Y | W | Z | R¹ | R² | R³ | R¹⁰ | A |
|---|---|---|---|---|---|---|---|---|
| D-1579 | H | CH(Me) | H | *c*-Pr | C(=O)Ph | Me | Me | O |
| D-1580 | H | CH₂CH₂ | Me | *c*-Pr | C(=O)Ph | Me | Me | O |
| D-1581 | Me | CH₂CH₂ | H | *c*-Pr | C(=O)Ph | Me | Me | O |
| D-1582 | H | single bond | Me | *c*-Pr | C(=O)Ph | Cl | Et | O |
| D-1583 | Me | single bond | H | *c*-Pr | C(=O)Ph | Cl | Et | O |
| D-1584 | Me(R) | single bond | H | *c*-Pr | C(=O)Ph | Cl | Et | O |
| D-1585 | Me(S) | single bond | H | *c*-Pr | C(=O)Ph | Cl | Et | O |
| D-1586 | H | CH₂ | Me | *c*-Pr | C(=O)Ph | Cl | Et | O |
| D-1587 | Me | CH₂ | H | *c*-Pr | C(=O)Ph | Cl | Et | O |
| D-1588 | H | CH(Me) | H | *c*-Pr | C(=O)Ph | Cl | Et | O |
| D-1589 | H | CH₂CH₂ | Me | *c*-Pr | C(=O)Ph | Cl | Et | O |
| D-1590 | Me | CH₂CH₂ | H | *c*-Pr | C(=O)Ph | Cl | Et | O |
| D-1591 | H | single bond | Me | *c*-Pr | C(=O)Ph | Me | Et | O |
| D-1592 | Me | single bond | H | *c*-Pr | C(=O)Ph | Me | Et | O |
| D-1593 | Me(R) | single bond | H | *c*-Pr | C(=O)Ph | Me | Et | O |
| D-1594 | Me(S) | single bond | H | *c*-Pr | C(=O)Ph | Me | Et | O |
| D-1595 | H | CH₂ | Me | *c*-Pr | C(=O)Ph | Me | Et | O |
| D-1596 | Me | CH₂ | H | *c*-Pr | C(=O)Ph | Me | Et | O |
| D-1597 | H | CH(Me) | H | *c*-Pr | C(=O)Ph | Me | Et | O |
| D-1598 | H | CH₂CH₂ | Me | *c*-Pr | C(=O)Ph | Me | Et | O |
| D-1599 | Me | CH₂CH₂ | H | *c*-Pr | C(=O)Ph | Me | Et | O |
| D-1600 | H | single bond | Me | *c*-Pr | C(=O)Ph | Cl | CH₂CF₃ | O |
| D-1601 | Me | single bond | H | *c*-Pr | C(=O)Ph | Cl | CH₂CF₃ | O |
| D-1602 | Me(R) | single bond | H | *c*-Pr | C(=O)Ph | Cl | CH₂CF₃ | O |
| D-1603 | Me(S) | single bond | H | *c*-Pr | C(=O)Ph | Cl | CH₂CF₃ | O |
| D-1604 | H | CH₂ | Me | *c*-Pr | C(=O)Ph | Cl | CH₂CF₃ | O |
| D-1605 | Me | CH₂ | H | *c*-Pr | C(=O)Ph | Cl | CH₂CF₃ | O |
| D-1606 | H | CH(Me) | H | *c*-Pr | C(=O)Ph | Cl | CH₂CF₃ | O |
| D-1607 | H | CH₂CH₂ | Me | *c*-Pr | C(=O)Ph | Cl | CH₂CF₃ | O |
| D-1608 | Me | CH₂CH₂ | H | *c*-Pr | C(=O)Ph | Cl | CH₂CF₃ | O |
| D-1609 | H | single bond | Me | *c*-Pr | C(=O)Ph | Me | CH₂CF₃ | O |
| D-1610 | Me | single bond | H | *c*-Pr | C(=O)Ph | Me | CH₂CF₃ | O |
| D-1611 | Me(R) | single bond | H | *c*-Pr | C(=O)Ph | Me | CH₂CF₃ | O |
| D-1612 | Me(S) | single bond | H | *c*-Pr | C(=O)Ph | Me | CH₂CF₃ | O |
| D-1613 | H | CH₂ | Me | *c*-Pr | C(=O)Ph | Me | CH₂CF₃ | O |
| D-1614 | Me | CH₂ | H | *c*-Pr | C(=O)Ph | Me | CH₂CF₃ | O |
| D-1615 | H | CH(Me) | H | *c*-Pr | C(=O)Ph | Me | CH₂CF₃ | O |
| D-1616 | H | CH₂CH₂ | Me | *c*-Pr | C(=O)Ph | Me | CH₂CF₃ | O |
| D-1617 | Me | CH₂CH₂ | H | *c*-Pr | C(=O)Ph | Me | CH₂CF₃ | O |
| D-1618 | H | single bond | Me | *c*-Pr | C(=O)Ph | Cl | CH₂OMe | O |
| D-1619 | Me | single bond | H | *c*-Pr | C(=O)Ph | Cl | CH₂OMe | O |
| D-1620 | Me(R) | single bond | H | *c*-Pr | C(=O)Ph | Cl | CH₂OMe | O |
| D-1621 | Me(S) | single bond | H | *c*-Pr | C(=O)Ph | Cl | CH₂OMe | O |
| D-1622 | H | CH₂ | Me | *c*-Pr | C(=O)Ph | Cl | CH₂OMe | O |

**[Table 682]**

| Compound Number | Y | W | Z | R¹ | R² | R³ | R¹⁰ | A |
|---|---|---|---|---|---|---|---|---|
| D-1623 | Me | CH₂ | H | *c*-Pr | C(=O)Ph | Cl | CH₂OMe | O |
| D-1624 | H | CH(Me) | H | *c*-Pr | C(=O)Ph | Cl | CH₂OMe | O |
| D-1625 | H | CH₂CH₂ | Me | *c*-Pr | C(=O)Ph | Cl | CH₂OMe | O |
| D-1626 | Me | CH₂CH₂ | H | *c*-Pr | C(=O)Ph | Cl | CH₂OMe | O |
| D-1627 | H | single bond | Me | *c*-Pr | C(=O)Ph | Me | CH₂OMe | O |
| D-1628 | Me | single bond | H | *c*-Pr | C(=O)Ph | Me | CH₂OMe | O |
| D-1629 | Me(R) | single bond | H | *c*-Pr | C(=O)Ph | Me | CH₂OMe | O |
| D-1630 | Me(S) | single bond | H | *c*-Pr | C(=O)Ph | Me | CH₂OMe | O |
| D-1631 | H | CH₂ | Me | *c*-Pr | C(=O)Ph | Me | CH₂OMe | O |
| D-1632 | Me | CH₂ | H | *c*-Pr | C(=O)Ph | Me | CH₂OMe | O |
| D-1633 | H | CH(Me) | H | *c*-Pr | C(=O)Ph | Me | CH₂OMe | O |
| D-1634 | H | CH₂CH₂ | Me | *c*-Pr | C(=O)Ph | Me | CH₂OMe | O |
| D-1635 | Me | CH₂CH₂ | H | *c*-Pr | C(=O)Ph | Me | CH₂OMe | O |
| D-1636 | H | single bond | Me | *c*-Pr | C(=O)Ph | Cl | CH₂CN | O |
| D-1637 | Me | single bond | H | *c*-Pr | C(=O)Ph | Cl | CH₂CN | O |
| D-1638 | Me(R) | single bond | H | *c-*Pr | C(=O)Ph | Cl | CH₂CN | O |
| D-1639 | Me(S) | single bond | H | *c*-Pr | C(=O)Ph | Cl | CH₂CN | O |
| D-1640 | H | CH₂ | Me | *c*-Pr | C(=O)Ph | Cl | CH₂CN | O |
| D-1641 | Me | CH₂ | H | *c*-Pr | C(=O)Ph | Cl | CH₂CN | O |
| D-1642 | H | CH(Me) | H | *c*-Pr | C(=O)Ph | Cl | CH₂CN | O |
| D-1643 | H | CH₂CH₂ | Me | *c*-Pr | C(=O)Ph | Cl | CH₂CN | O |
| D-1644 | Me | CH₂CH₂ | H | *c*-Pr | C(=O)Ph | Cl | CH₂CN | O |
| D-1645 | H | single bond | Me | *c*-Pr | C(=O)Ph | Me | CH₂CN | O |
| D-1646 | Me | single bond | H | *c*-Pr | C(=O)Ph | Me | CH₂CN | O |
| D-1647 | Me(R) | single bond | H | *c*-Pr | C(=O)Ph | Me | CH₂CN | O |
| D-1648 | Me(S) | single bond | H | *c*-Pr | C(=O)Ph | Me | CH₂CN | O |
| D-1649 | H | CH₂ | Me | *c*-Pr | C(=O)Ph | Me | CH₂CN | O |
| D-1650 | Me | CH₂ | H | *c*-Pr | C(=O)Ph | Me | CH₂CN | O |
| D-1651 | H | CH(Me) | H | *c*-Pr | C(=O)Ph | Me | CH₂CN | O |
| D-1652 | H | CH₂CH₂ | Me | *c*-Pr | C(=O)Ph | Me | CH₂CN | O |
| D-1653 | Me | CH₂CH₂ | H | *c*-Pr | C(=O)Ph | Me | CH₂CN | O |
| D-1654 | H | single bond | Me | *c*-Pr | C(=O)Ph | Cl | C(=O)Me | O |
| D-1655 | Me | single bond | H | *c*-Pr | C(=O)Ph | Cl | C(=O)Me | O |
| D-1656 | Me(R) | single bond | H | *c*-Pr | C(=O)Ph | Cl | C(=O)Me | O |
| D-1657 | Me(S) | single bond | H | *c*-Pr | C(=O)Ph | Cl | C(=O)Me | O |
| D-1658 | H | CH₂ | Me | *c*-Pr | C(=O)Ph | Cl | C(=O)Me | O |
| D-1659 | Me | CH₂ | H | *c*-Pr | C(=O)Ph | Cl | C(=O)Me | O |
| D-1660 | H | CH(Me) | H | *c*-Pr | C(=O)Ph | Cl | C(=O)Me | O |
| D-1661 | H | CH₂CH₂ | Me | *c*-Pr | C(=O)Ph | Cl | C(=O)Me | O |
| D-1662 | Me | CH₂CH₂ | H | *c*-Pr | C(=O)Ph | Cl | C(=O)Me | O |
| D-1663 | H | single bond | Me | *c*-Pr | C(=O)Ph | Me | C(=O)Me | O |
| D-1664 | Me | single bond | H | *c*-Pr | C(=O)Ph | Me | C(=O)Me | O |
| D-1665 | Me(R) | single bond | H | *c*-Pr | C(=O)Ph | Me | C(=O)Me | O |
| D-1666 | Me(S) | single bond | H | *c*-Pr | C(=O)Ph | Me | C(=O)Me | O |

**[Table 683]**

| Compound Number | Y | W | Z | R¹ | R² | R³ | R¹⁰ | A |
|---|---|---|---|---|---|---|---|---|
| D-1667 | H | CH₂ | Me | *c*-Pr | C(=O)Ph | Me | C(=O)Me | O |
| D-1668 | Me | CH₂ | H | *c*-Pr | C(=O)Ph | Me | C(=O)Me | O |
| D-1669 | H | CH(Me) | H | *c*-Pr | C(=O)Ph | Me | C(=O)Me | O |
| D-1670 | H | CH₂CH₂ | Me | *c*-Pr | C(=O)Ph | Me | C(=O)Me | O |
| D-1671 | Me | CH₂CH₂ | H | *c*-Pr | C(=O)Ph | Me | C(=O)Me | O |
| D-1672 | H | single bond | Me | *c*-Pr | C(=O)OEt | Cl | Me | O |
| D-1673 | H | single bond | Et | *c*-Pr | C(=O)OEt | Cl | Me | O |
| D-1674 | Me | single bond | H | *c*-Pr | C(=O)OEt | Cl | Me | O |
| D-1675 | Me(R) | single bond | H | *c*-Pr | C(=O)OEt | Cl | Me | O |
| D-1676 | Me(S) | single bond | H | *c*-Pr | C(=O)OEt | Cl | Me | O |
| D-1677 | H | CH₂ | Me | *c*-Pr | C(=O)OEt | Cl | Me | O |
| D-1678 | Me | CH₂ | H | *c*-Pr | C(=O)OEt | Cl | Me | O |
| D-1679 | H | CH(Me) | H | *c*-Pr | C(=O)OEt | Cl | Me | O |
| D-1680 | H | CH₂CH₂ | Me | *c*-Pr | C(=O)OEt | Cl | Me | O |
| D-1681 | Me | CH₂CH₂ | H | *c*-Pr | C(=O)OEt | Cl | Me | O |
| D-1682 | H | single bond | Me | *c*-Pr | C(=O)OEt | Me | Me | O |
| D-1683 | Me | single bond | H | *c*-Pr | C(=O)OEt | Me | Me | O |
| D-1684 | Me(R) | single bond | H | *c*-Pr | C(=O)OEt | Me | Me | O |
| D-1685 | Me(S) | single bond | H | *c*-Pr | C(=O)OEt | Me | Me | O |
| D-1686 | H | CH₂ | Me | *c*-Pr | C(=O)OEt | Me | Me | O |
| D-1687 | Me | CH₂ | H | *c*-Pr | C(=O)OEt | Me | Me | O |
| D-1688 | H | CH(Me) | H | *c*-Pr | C(=O)OEt | Me | Me | O |
| D-1689 | H | CH₂CH₂ | Me | *c*-Pr | C(=O)OEt | Me | Me | O |
| D-1690 | Me | CH₂CH₂ | H | *c*-Pr | C(=O)OEt | Me | Me | O |
| D-1691 | H | single bond | Me | *c*-Pr | C(=O)OEt | Cl | Et | O |
| D-1692 | Me | single bond | H | *c*-Pr | C(=O)OEt | Cl | Et | O |
| D-1693 | Me(R) | single bond | H | *c*-Pr | C(=O)OEt | Cl | Et | O |
| D-1694 | Me(S) | single bond | H | *c*-Pr | C(=O)OEt | Cl | Et | O |
| D-1695 | H | CH₂ | Me | *c*-Pr | C(=O)OEt | Cl | Et | O |
| D-1696 | Me | CH₂ | H | *c*-Pr | C(=O)OEt | Cl | Et | O |
| D-1697 | H | CH(Me) | H | *c*-Pr | C(=O)OEt | Cl | Et | O |
| D-1698 | H | CH₂CH₂ | Me | *c*-Pr | C(=O)OEt | Cl | Et | O |
| D-1699 | Me | CH₂CH₂ | H | *c*-Pr | C(=O)OEt | Cl | Et | O |
| D-1700 | H | single bond | Me | *c*-Pr | C(=O)OEt | Me | Et | O |
| D-1701 | Me | single bond | H | *c*-Pr | C(=O)OEt | Me | Et | O |
| D-1702 | Me(R) | single bond | H | *c*-Pr | C(=O)OEt | Me | Et | O |
| D-1703 | Me(S) | single bond | H | *c*-Pr | C(=O)OEt | Me | Et | O |
| D-1704 | H | CH₂ | Me | *c*-Pr | C(=O)OEt | Me | Et | O |
| D-1705 | Me | CH₂ | H | *c*-Pr | C(=O)OEt | Me | Et | O |
| D-1706 | H | CH(Me) | H | *c*-Pr | C(=O)OEt | Me | Et | O |
| D-1707 | H | CH₂CH₂ | Me | *c*-Pr | C(=O)OEt | Me | Et | O |
| D-1708 | Me | CH₂CH₂ | H | *c*-Pr | C(=O)OEt | Me | Et | O |
| D-1709 | H | single bond | Me | *c-*Pr | C(=O)OEt | Cl | CH₂CF₃ | O |
| D-1710 | Me | single bond | H | *c*-Pr | C(=O)OEt | Cl | CH₂CF₃ | O |

**[Table 684]**

| Compound Number | Y | W | Z | R¹ | R² | R³ | R¹⁰ | A |
|---|---|---|---|---|---|---|---|---|
| D-1711 | Me(R) | single bond | H | *c*-Pr | C(=O)OEt | Cl | CH₂CF₃ | O |
| D-1712 | Me(S) | single bond | H | *c*-Pr | C(=O)OEt | Cl | CH₂CF₃ | O |
| D-1713 | H | CH₂ | Me | *c*-Pr | C(=O)OEt | Cl | CH₂CF₃ | O |
| D-1714 | Me | CH₂ | H | *c*-Pr | C(=O)OEt | Cl | CH₂CF₃ | O |
| D-1715 | H | CH(Me) | H | *c*-Pr | C(=O)OEt | Cl | CH₂CF₃ | O |
| D-1716 | H | CH₂CH₂ | Me | *c*-Pr | C(=O)OEt | Cl | CH₂CF₃ | O |
| D-1717 | Me | CH₂CH₂ | H | *c*-Pr | C(=O)OEt | Cl | CH₂CF₃ | O |
| D-1718 | H | single bond | Me | *c*-Pr | C(=O)OEt | Me | CH₂CF₃ | O |
| D-1719 | Me | single bond | H | *c*-Pr | C(=O)OEt | Me | CH₂CF₃ | O |
| D-1720 | Me(R) | single bond | H | *c*-Pr | C(=O)OEt | Me | CH₂CF₃ | O |
| D-1721 | Me(S) | single bond | H | *c*-Pr | C(=O)OEt | Me | CH₂CF₃ | O |
| D-1722 | H | CH₂ | Me | *c*-Pr | C(=O)OEt | Me | CH₂CF₃ | O |
| D-1723 | Me | CH₂ | H | *c*-Pr | C(=O)OEt | Me | CH₂CF₃ | O |
| D-1724 | H | CH(Me) | H | *c*-Pr | C(=O)OEt | Me | CH₂CF₃ | O |
| D-1725 | H | CH₂CH₂ | Me | *c*-Pr | C(=O)OEt | Me | CH₂CF₃ | O |
| D-1726 | Me | CH₂CH₂ | H | *c*-Pr | C(=O)OEt | Me | CH₂CF₃ | O |
| D-1727 | H | single bond | Me | *c*-Pr | C(=O)OEt | Cl | CH₂OMe | O |
| D-1728 | Me | single bond | H | *c*-Pr | C(=O)OEt | Cl | CH₂OMe | O |
| D-1729 | Me(R) | single bond | H | *c*-Pr | C(=O)OEt | Cl | CH₂OMe | O |
| D-1730 | Me(S) | single bond | H | *c*-Pr | C(=O)OEt | Cl | CH₂OMe | O |
| D-1731 | H | CH₂ | Me | *c*-Pr | C(=O)OEt | Cl | CH₂OMe | O |
| D-1732 | Me | CH₂ | H | *c*-Pr | C(=O)OEt | Cl | CH₂OMe | O |
| D-1733 | H | CH(Me) | H | *c*-Pr | C(=O)OEt | Cl | CH₂OMe | O |
| D-1734 | H | CH₂CH₂ | Me | *c*-Pr | C(=O)OEt | Cl | CH₂OMe | O |
| D-1735 | Me | CH₂CH₂ | H | *c*-Pr | C(=O)OEt | Cl | CH₂OMe | O |
| D-1736 | H | single bond | Me | *c*-Pr | C(=O)OEt | Me | CH₂OMe | O |
| D-1737 | Me | single bond | H | *c*-Pr | C(=O)OEt | Me | CH₂OMe | O |
| D-1738 | Me(R) | single bond | H | *c*-Pr | C(=O)OEt | Me | CH₂OMe | O |
| D-1739 | Me(S) | single bond | H | *c*-Pr | C(=O)OEt | Me | CH₂OMe | O |
| D-1740 | H | CH₂ | Me | *c*-Pr | C(=O)OEt | Me | CH₂OMe | O |
| D-1741 | Me | CH₂ | H | *c*-Pr | C(=O)OEt | Me | CH₂OMe | O |
| D-1742 | H | CH(Me) | H | *c*-Pr | C(=O)OEt | Me | CH₂OMe | O |
| D-1743 | H | CH₂CH₂ | Me | *c*-Pr | C(=O)OEt | Me | CH₂OMe | O |
| D-1744 | Me | CH₂CH₂ | H | *c*-Pr | C(=O)OEt | Me | CH₂OMe | O |
| D-1745 | H | single bond | Me | *c*-Pr | C(=O)OEt | Cl | CH₂CN | O |
| D-1746 | Me | single bond | H | *c*-Pr | C(=O)OEt | Cl | CH₂CN | O |
| D-1747 | Me(R) | single bond | H | *c*-Pr | C(=O)OEt | Cl | CH₂CN | O |
| D-1748 | Me(S) | single bond | H | *c*-Pr | C(=O)OEt | Cl | CH₂CN | O |
| D-1749 | H | CH₂ | Me | *c-*Pr | C(=O)OEt | Cl | CH₂CN | O |
| D-1750 | Me | CH₂ | H | *c*-Pr | C(=O)OEt | Cl | CH₂CN | O |
| D-1751 | H | CH(Me) | H | *c*-Pr | C(=O)OEt | Cl | CH₂CN | O |
| D-1752 | H | CH₂CH₂ | Me | *c*-Pr | C(=O)OEt | Cl | CH₂CN | O |
| D-1753 | Me | CH₂CH₂ | H | *c*-Pr | C(=O)OEt | Cl | CH₂CN | O |
| D-1754 | H | single bond | Me | *c*-Pr | C(=O)OEt | Me | CH₂CN | O |

**[Table 685]**

| Compound Number | Y | W | Z | R¹ | R² | R³ | R¹⁰ | A |
|---|---|---|---|---|---|---|---|---|
| D-1755 | Me | single bond | H | *c*-Pr | C(=O)OEt | Me | CH₂CN | O |
| D-1756 | Me(R) | single bond | H | *c*-Pr | C(=O)OEt | Me | CH₂CN | O |
| D-1757 | Me(S) | single bond | H | *c*-Pr | C(=O)OEt | Me | CH₂CN | O |
| D-1758 | H | CH₂ | Me | *c*-Pr | C(=O)OEt | Me | CH₂CN | O |
| D-1759 | Me | CH₂ | H | *c*-Pr | C(=O)OEt | Me | CH₂CN | O |
| D-1760 | H | CH(Me) | H | *c*-Pr | C(=O)OEt | Me | CH₂CN | O |
| D-1761 | H | CH₂CH₂ | Me | *c*-Pr | C(=O)OEt | Me | CH₂CN | O |
| D-1762 | Me | CH₂CH₂ | H | *c*-Pr | C(=O)OEt | Me | CH₂CN | O |
| D-1763 | H | single bond | Me | *c*-Pr | C(=O)OEt | Cl | C(=O)Me | O |
| D-1764 | Me | single bond | H | *c*-Pr | C(=O)OEt | Cl | C(=O)Me | O |
| D-1765 | Me(R) | single bond | H | *c*-Pr | C(=O)OEt | Cl | C(=O)Me | O |
| D-1766 | Me(S) | single bond | H | *c*-Pr | C(=O)OEt | Cl | C(=O)Me | O |
| D-1767 | H | CH₂ | Me | *c*-Pr | C(=O)OEt | Cl | C(=O)Me | O |
| D-1768 | Me | CH₂ | H | *c*-Pr | C(=O)OEt | Cl | C(=O)Me | O |
| D-1769 | H | CH(Me) | H | *c*-Pr | C(=O)OEt | Cl | C(=O)Me | O |
| D-1770 | H | CH₂CH₂ | Me | *c*-Pr | C(=O)OEt | Cl | C(=O)Me | O |
| D-1771 | Me | CH₂CH₂ | H | *c*-Pr | C(=O)OEt | Cl | C(=O)Me | O |
| D-1772 | H | single bond | Me | *c*-Pr | C(=O)OEt | Me | C(=O)Me | O |
| D-1773 | Me | single bond | H | *c*-Pr | C(=O)OEt | Me | C(=O)Me | O |
| D-1774 | Me(R) | single bond | H | *c*-Pr | C(=O)OEt | Me | C(=O)Me | O |
| D-1775 | Me(S) | single bond | H | *c*-Pr | C(=O)OEt | Me | C(=O)Me | O |
| D-1776 | H | CH₂ | Me | *c*-Pr | C(=O)OEt | Me | C(=O)Me | O |
| D-1777 | Me | CH₂ | H | *c*-Pr | C(=O)OEt | Me | C(=O)Me | O |
| D-1778 | H | CH(Me) | H | *c*-Pr | C(=O)OEt | Me | C(=O)Me | O |
| D-1779 | H | CH₂CH₂ | Me | *c*-Pr | C(=O)OEt | Me | C(=O)Me | O |
| D-1780 | Me | CH₂CH₂ | H | *c*-Pr | C(=O)OEt | Me | C(=O)Me | O |
| D-1781 | H | single bond | Me | *c*-Pr | S(=O)₂Me | Cl | Me | O |
| D-1782 | H | single bond | Et | *c*-Pr | S(=O)₂Me | Cl | Me | O |
| D-1783 | Me | single bond | H | *c*-Pr | S(=O)₂Me | Cl | Me | O |
| D-1784 | Me(R) | single bond | H | *c*-Pr | S(=O)₂Me | Cl | Me | O |
| D-1785 | Me(S) | single bond | H | *c*-Pr | S(=O)₂Me | Cl | Me | O |
| D-1786 | H | CH₂ | Me | *c*-Pr | S(=O)₂Me | Cl | Me | O |
| D-1787 | Me | CH₂ | H | *c*-Pr | S(=O)₂Me | Cl | Me | O |
| D-1788 | H | CH(Me) | H | *c*-Pr | S(=O)₂Me | Cl | Me | O |
| D-1789 | H | CH₂CH₂ | Me | *c*-Pr | S(=O)₂Me | Cl | Me | O |
| D-1790 | Me | CH₂CH₂ | H | *c*-Pr | S(=O)₂Me | Cl | Me | O |
| D-1791 | H | single bond | Me | *c*-Pr | S(=O)₂Me | Me | Me | O |
| D-1792 | Me | single bond | H | *c*-Pr | S(=O)₂Me | Me | Me | O |
| D-1793 | Me(R) | single bond | H | *c*-Pr | S(=O)₂Me | Me | Me | O |
| D-1794 | Me(S) | single bond | H | *c*-Pr | S(=O)₂Me | Me | Me | O |
| D-1795 | H | CH₂ | Me | *c*-Pr | S(=O)₂Me | Me | Me | O |
| D-1796 | Me | CH₂ | H | *c*-Pr | S(=O)₂Me | Me | Me | O |
| D-1797 | H | CH(Me) | H | *c*-Pr | S(=O)₂Me | Me | Me | O |
| D-1798 | H | CH₂CH₂ | Me | *c*-Pr | S(=O)₂Me | Me | Me | O |

**[Table 686]**

| Compound Number | Y | W | Z | R¹ | R² | R³ | R¹⁰ | A |
|---|---|---|---|---|---|---|---|---|
| D-1799 | Me | CH₂CH₂ | H | *c*-Pr | S(=O)₂Me | Me | Me | O |
| D-1800 | H | single bond | Me | *c*-Pr | S(=O)₂Me | Cl | Et | O |
| D-1801 | Me | single bond | H | *c*-Pr | S(=O)₂Me | Cl | Et | O |
| D-1802 | Me(R) | single bond | H | *c*-Pr | S(=O)₂Me | Cl | Et | O |
| D-1803 | Me(S) | single bond | H | *c*-Pr | S(=O)₂Me | Cl | Et | O |
| D-1804 | H | CH₂ | Me | *c*-Pr | S(=O)₂Me | Cl | Et | O |
| D-1805 | Me | CH₂ | H | *c*-Pr | S(=O)₂Me | Cl | Et | O |
| D-1806 | H | CH(Me) | H | *c*-Pr | S(=O)₂Me | Cl | Et | O |
| D-1807 | H | CH₂CH₂ | Me | *c*-Pr | S(=O)₂Me | Cl | Et | O |
| D-1808 | Me | CH₂CH₂ | H | *c*-Pr | S(=O)₂Me | Cl | Et | O |
| D-1809 | H | single bond | Me | *c*-Pr | S(=O)₂Me | Me | Et | O |
| D-1810 | Me | single bond | H | *c*-Pr | S(=O)₂Me | Me | Et | O |
| D-1811 | Me(R) | single bond | H | *c*-Pr | S(=O)₂Me | Me | Et | O |
| D-1812 | Me(S) | single bond | H | *c*-Pr | S(=O)₂Me | Me | Et | O |
| D-1813 | H | CH₂ | Me | *c*-Pr | S(=O)₂Me | Me | Et | O |
| D-1814 | Me | CH₂ | H | *c*-Pr | S(=O)₂Me | Me | Et | O |
| D-1815 | H | CH(Me) | H | *c*-Pr | S(=O)₂Me | Me | Et | O |
| D-1816 | H | CH₂CH₂ | Me | c-Pr | S(=O)₂Me | Me | Et | O |
| D-1817 | Me | CH₂CH₂ | H | *c*-Pr | S(=O)₂Me | Me | Et | O |
| D-1818 | H | single bond | Me | *c*-Pr | S(=O)₂Me | Cl | CH₂CF₃ | O |
| D-1819 | Me | single bond | H | *c*-Pr | S(=O)₂Me | Cl | CH₂CF₃ | O |
| D-1820 | Me(R) | single bond | H | *c*-Pr | S(=O)₂Me | Cl | CH₂CF₃ | O |
| D-1821 | Me(S) | single bond | H | *c*-Pr | S(=O)₂Me | Cl | CH₂CF₃ | O |
| D-1822 | H | CH₂ | Me | *c*-Pr | S(=O)₂Me | Cl | CH₂CF₃ | O |
| D-1823 | Me | CH₂ | H | *c*-Pr | S(=O)₂Me | Cl | CH₂CF₃ | O |
| D-1824 | H | CH(Me) | H | *c*-Pr | S(=O)₂Me | Cl | CH₂CF₃ | O |
| D-1825 | H | CH₂CH₂ | Me | *c*-Pr | S(=O)₂Me | Cl | CH₂CF₃ | O |
| D-1826 | Me | CH₂CH₂ | H | *c*-Pr | S(=O)₂Me | Cl | CH₂CF₃ | O |
| D-1827 | H | single bond | Me | *c*-Pr | S(=O)₂Me | Me | CH₂CF₃ | O |
| D-1828 | Me | single bond | H | *c*-Pr | S(=O)₂Me | Me | CH₂CF₃ | O |
| D-1829 | Me(R) | single bond | H | *c*-Pr | S(=O)₂Me | Me | CH₂CF₃ | O |
| D-1830 | Me(S) | single bond | H | *c*-Pr | S(=O)₂Me | Me | CH₂CF₃ | O |
| D-1831 | H | CH₂ | Me | *c*-Pr | S(=O)₂Me | Me | CH₂CF₃ | O |
| D-1832 | Me | CH₂ | H | *c*-Pr | S(=O)₂Me | Me | CH₂CF₃ | O |
| D-1833 | H | CH(Me) | H | *c*-Pr | S(=O)₂Me | Me | CH₂CF₃ | O |
| D-1834 | H | CH₂CH₂ | Me | *c*-Pr | S(=O)₂Me | Me | CH₂CF₃ | O |
| D-1835 | Me | CH₂CH₂ | H | *c*-Pr | S(=O)₂Me | Me | CH₂CF₃ | O |
| D-1836 | H | single bond | Me | *c*-Pr | S(=O)₂Me | Cl | CH₂OMe | O |
| D-1837 | Me | single bond | H | *c*-Pr | S(=O)₂Me | Cl | CH₂OMe | O |
| D-1838 | Me(R) | single bond | H | *c*-Pr | S(=O)₂Me | Cl | CH₂OMe | O |
| D-1839 | Me(S) | single bond | H | *c*-Pr | S(=O)₂Me | Cl | CH₂OMe | O |
| D-1840 | H | CH₂ | Me | *c*-Pr | S(=O)₂Me | Cl | CH₂OMe | O |
| D-1841 | Me | CH₂ | H | *c*-Pr | S(=O)₂Me | Cl | CH₂OMe | O |
| D-1842 | H | CH(Me) | H | *c*-Pr | S(=O)₂Me | Cl | CH₂OMe | O |

**[Table 687]**

| Compound Number | Y | W | Z | R¹ | R² | R³ | R¹⁰ | A |
|---|---|---|---|---|---|---|---|---|
| D-1843 | H | CH₂CH₂ | Me | *c*-Pr | S(=O)₂Me | Cl | CH₂OMe | O |
| D-1844 | Me | CH₂CH₂ | H | *c*-Pr | S(=O)₂Me | Cl | CH₂OMe | O |
| D-1845 | H | single bond | Me | *c*-Pr | S(=O)₂Me | Me | CH₂OMe | O |
| D-1846 | Me | single bond | H | *c*-Pr | S(=O)₂Me | Me | CH₂OMe | O |
| D-1847 | Me(R) | single bond | H | *c*-Pr | S(=O)₂Me | Me | CH₂OMe | O |
| D-1848 | Me(S) | single bond | H | *c*-Pr | S(=O)₂Me | Me | CH₂OMe | O |
| D-1849 | H | CH₂ | Me | *c*-Pr | S(=O)₂Me | Me | CH₂OMe | O |
| D-1850 | Me | CH₂ | H | *c*-Pr | S(=O)₂Me | Me | CH₂OMe | O |
| D-1851 | H | CH(Me) | H | *c*-Pr | S(=O)₂Me | Me | CH₂OMe | O |
| D-1852 | H | CH₂CH₂ | Me | *c*-Pr | S(=O)₂Me | Me | CH₂OMe | O |
| D-1853 | Me | CH₂CH₂ | H | *c*-Pr | S(=O)₂Me | Me | CH₂OMe | O |
| D-1854 | H | single bond | Me | *c*-Pr | S(=O)₂Me | Cl | CH₂CN | O |
| D-1855 | Me | single bond | H | *c*-Pr | S(=O)₂Me | Cl | CH₂CN | O |
| D-1856 | Me(R) | single bond | H | *c*-Pr | S(=O)₂Me | Cl | CH₂CN | O |
| D-1857 | Me(S) | single bond | H | *c*-Pr | S(=O)₂Me | Cl | CH₂CN | O |
| D-1858 | H | CH₂ | Me | *c*-Pr | S(=O)₂Me | Cl | CH₂CN | O |
| D-1859 | Me | CH₂ | H | *c*-Pr | S(=O)₂Me | Cl | CH₂CN | O |
| D-1860 | H | CH(Me) | H | *c*-Pr | S(=O)₂Me | Cl | CH₂CN | O |
| D-1861 | H | CH₂CH₂ | Me | *c*-Pr | S(=O)₂Me | Cl | CH₂CN | O |
| D-1862 | Me | CH₂CH₂ | H | *c*-Pr | S(=O)₂Me | Cl | CH₂CN | O |
| D-1863 | H | single bond | Me | *c*-Pr | S(=O)₂Me | Me | CH₂CN | O |
| D-1864 | Me | single bond | H | *c*-Pr | S(=O)₂Me | Me | CH₂CN | O |
| D-1865 | Me(R) | single bond | H | *c*-Pr | S(=O)₂Me | Me | CH₂CN | O |
| D-1866 | Me(S) | single bond | H | *c*-Pr | S(=O)₂Me | Me | CH₂CN | O |
| D-1867 | H | CH₂ | Me | *c*-Pr | S(=O)₂Me | Me | CH₂CN | O |
| D-1868 | Me | CH₂ | H | *c*-Pr | S(=O)₂Me | Me | CH₂CN | O |
| D-1869 | H | CH(Me) | H | *c*-Pr | S(=O)₂Me | Me | CH₂CN | O |
| D-1870 | H | CH₂CH₂ | Me | *c*-Pr | S(=O)₂Me | Me | CH₂CN | O |
| D-1871 | Me | CH₂CH₂ | H | *c*-Pr | S(=O)₂Me | Me | CH₂CN | O |
| D-1872 | H | single bond | Me | *c*-Pr | S(=O)₂Me | Cl | C(=O)Me | O |
| D-1873 | Me | single bond | H | *c*-Pr | S(=O)₂Me | Cl | C(=O)Me | O |
| D-1874 | Me(R) | single bond | H | *c*-Pr | S(=O)₂Me | Cl | C(=O)Me | O |
| D-1875 | Me(S) | single bond | H | *c*-Pr | S(=O)₂Me | Cl | C(=O)Me | O |
| D-1876 | H | CH₂ | Me | *c*-Pr | S(=O)₂Me | Cl | C(=O)Me | O |
| D-1877 | Me | CH₂ | H | *c*-Pr | S(=O)₂Me | Cl | C(=O)Me | O |
| D-1878 | H | CH(Me) | H | *c*-Pr | S(=O)₂Me | Cl | C(=O)Me | O |
| D-1879 | H | CH₂CH₂ | Me | *c*-Pr | S(=O)₂Me | Cl | C(=O)Me | O |
| D-1880 | Me | CH₂CH₂ | H | *c*-Pr | S(=O)₂Me | Cl | C(=O)Me | O |
| D-1881 | H | single bond | Me | *c*-Pr | S(=O)₂Me | Me | C(=O)Me | O |
| D-1882 | Me | single bond | H | *c*-Pr | S(=O)₂Me | Me | C(=O)Me | O |
| D-1883 | Me(R) | single bond | H | *c*-Pr | S(=O)₂Me | Me | C(=O)Me | O |
| D-1884 | Me(S) | single bond | H | *c*-Pr | S(=O)₂Me | Me | C(=O)Me | O |
| D-1885 | H | CH₂ | Me | *c*-Pr | S(=O)₂Me | Me | C(=O)Me | O |
| D-1886 | Me | CH₂ | H | *c*-Pr | S(=O)₂Me | Me | C(=O)Me | O |

**[Table 688]**

| Compound Number | Y | W | Z | R¹ | R² | R³ | R¹⁰ | A |
|---|---|---|---|---|---|---|---|---|
| D-1887 | H | CH(Me) | H | *c*-Pr | S(=O)₂Me | Me | C(=O)Me | O |
| D-1888 | H | CH₂CH₂ | Me | *c*-Pr | S(=O)₂Me | Me | C(=O)Me | O |
| D-1889 | Me | CH₂CH₂ | H | *c*-Pr | S(=O)₂Me | Me | C(=O)Me | O |
| D-1890 | H | single bond | Me | *c*-Pr | S(=O)₂CF₃ | Cl | Me | O |
| D-1891 | H | single bond | Et | *c*-Pr | S(=O)₂CF₃ | Cl | Me | O |
| D-1892 | Me | single bond | H | *c*-Pr | S(=O)₂CF₃ | Cl | Me | O |
| D-1893 | Me(R) | single bond | H | *c*-Pr | S(=O)₂CF₃ | Cl | Me | O |
| D-1894 | Me(S) | single bond | H | *c*-Pr | S(=O)₂CF₃ | Cl | Me | O |
| D-1895 | H | CH₂ | Me | *c*-Pr | S(=O)₂CF₃ | Cl | Me | O |
| D-1896 | Me | CH₂ | H | *c*-Pr | S(=O)₂CF₃ | Cl | Me | O |
| D-1897 | H | CH(Me) | H | *c*-Pr | S(=O)₂CF₃ | Cl | Me | O |
| D-1898 | H | CH₂CH₂ | Me | *c*-Pr | S(=O)₂CF₃ | Cl | Me | O |
| D-1899 | Me | CH₂CH₂ | H | *c*-Pr | S(=O)₂CF₃ | Cl | Me | O |
| D-1900 | H | single bond | Me | *c*-Pr | S(=O)₂CF₃ | Me | Me | O |
| D-1901 | Me | single bond | H | *c*-Pr | S(=O)₂CF₃ | Me | Me | O |
| D-1902 | Me(R) | single bond | H | *c*-Pr | S(=O)₂CF₃ | Me | Me | O |
| D-1903 | Me(S) | single bond | H | *c*-Pr | S(=O)₂CF₃ | Me | Me | O |
| D-1904 | H | CH₂ | Me | *c*-Pr | S(=O)₂CF₃ | Me | Me | O |
| D-1905 | Me | CH₂ | H | *c*-Pr | S(=O)₂CF₃ | Me | Me | O |
| D-1906 | H | CH(Me) | H | *c*-Pr | S(=O)₂CF₃ | Me | Me | O |
| D-1907 | H | CH₂CH₂ | Me | *c*-Pr | S(=O)₂CF₃ | Me | Me | O |
| D-1908 | Me | CH₂CH₂ | H | *c*-Pr | S(=O)₂CF₃ | Me | Me | O |
| D-1909 | H | single bond | Me | *c*-Pr | S(=O)₂CF₃ | Cl | Et | O |
| D-1910 | Me | single bond | H | *c*-Pr | S(=O)₂CF₃ | Cl | Et | O |
| D-1911 | Me(R) | single bond | H | *c*-Pr | S(=O)₂CF₃ | Cl | Et | O |
| D-1912 | Me(S) | single bond | H | *c*-Pr | S(=O)₂CF₃ | Cl | Et | O |
| D-1913 | H | CH₂ | Me | *c*-Pr | S(=O)₂CF₃ | Cl | Et | O |
| D-1914 | Me | CH₂ | H | *c*-Pr | S(=O)₂CF₃ | Cl | Et | O |
| D-1915 | H | CH(Me) | H | *c*-Pr | S(=O)₂CF₃ | Cl | Et | O |
| D-1916 | H | CH₂CH₂ | Me | *c*-Pr | S(=O)₂CF₃ | Cl | Et | O |
| D-1917 | Me | CH₂CH₂ | H | *c*-Pr | S(=O)₂CF₃ | Cl | Et | O |
| D-1918 | H | single bond | Me | *c*-Pr | S(=O)₂CF₃ | Me | Et | O |
| D-1919 | Me | single bond | H | *c*-Pr | S(=O)₂CF₃ | Me | Et | O |
| D-1920 | Me(R) | single bond | H | *c*-Pr | S(=O)₂CF₃ | Me | Et | O |
| D-1921 | Me(S) | single bond | H | *c*-Pr | S(=O)₂CF₃ | Me | Et | O |
| D-1922 | H | CH₂ | Me | *c*-Pr | S(=O)₂CF₃ | Me | Et | O |
| D-1923 | Me | CH₂ | H | *c*-Pr | S(=O)₂CF₃ | Me | Et | O |
| D-1924 | H | CH(Me) | H | c-Pr | S(=O)₂CF₃ | Me | Et | O |
| D-1925 | H | CH₂CH₂ | Me | *c*-Pr | S(=O)₂CF₃ | Me | Et | O |
| D-1926 | Me | CH₂CH₂ | H | *c*-Pr | S(=O)₂CF₃ | Me | Et | O |
| D-1927 | H | single bond | Me | *c*-Pr | S(=O)₂CF₃ | Cl | CH₂CF₃ | O |
| D-1928 | Me | single bond | H | *c*-Pr | S(=O)₂CF₃ | Cl | CH₂CF₃ | O |
| D-1929 | Me(R) | single bond | H | *c*-Pr | S(=O)₂CF₃ | Cl | CH₂CF₃ | O |
| D-1930 | Me(S) | single bond | H | *c*-Pr | S(=O)₂CF₃ | Cl | CH₂CF₃ | O |

**[Table 689]**

| Compound Number | Y | W | Z | R¹ | R² | R³ | R¹⁰ | A |
|---|---|---|---|---|---|---|---|---|
| D-1931 | H | CH₂ | Me | *c*-Pr | S(=O)₂CF₃ | Cl | CH₂CF₃ | O |
| D-1932 | Me | CH₂ | H | *c*-Pr | S(=O)₂CF₃ | Cl | CH₂CF₃ | O |
| D-1933 | H | CH(Me) | H | *c*-Pr | S(=O)₂CF₃ | Cl | CH₂CF₃ | O |
| D-1934 | H | CH₂CH₂ | Me | *c*-Pr | S(=O)₂CF₃ | Cl | CH₂CF₃ | O |
| D-1935 | Me | CH₂CH₂ | H | *c*-Pr | S(=O)₂CF₃ | Cl | CH₂CF₃ | O |
| D-1936 | H | single bond | Me | *c*-Pr | S(=O)₂CF₃ | Me | CH₂CF₃ | O |
| D-1937 | Me | single bond | H | *c*-Pr | S(=O)₂CF₃ | Me | CH₂CF₃ | O |
| D-1938 | Me(R) | single bond | H | *c*-Pr | S(=O)₂CF₃ | Me | CH₂CF₃ | O |
| D-1939 | Me(S) | single bond | H | *c*-Pr | S(=O)₂CF₃ | Me | CH₂CF₃ | O |
| D-1940 | H | CH₂ | Me | *c*-Pr | S(=O)₂CF₃ | Me | CH₂CF₃ | O |
| D-1941 | Me | CH₂ | H | *c*-Pr | S(=O)₂CF₃ | Me | CH₂CF₃ | O |
| D-1942 | H | CH(Me) | H | *c*-Pr | S(=O)₂CF₃ | Me | CH₂CF₃ | O |
| D-1943 | H | CH₂CH₂ | Me | *c*-Pr | S(=O)₂CF₃ | Me | CH₂CF₃ | O |
| D-1944 | Me | CH₂CH₂ | H | *c*-Pr | S(=O)₂CF₃ | Me | CH₂CF₃ | O |
| D-1945 | H | single bond | Me | *c*-Pr | S(=O)₂CF₃ | Cl | CH₂OMe | O |
| D-1946 | Me | single bond | H | *c*-Pr | S(=O)₂CF₃ | Cl | CH₂OMe | O |
| D-1947 | Me(R) | single bond | H | *c*-Pr | S(=O)₂CF₃ | Cl | CH₂OMe | O |
| D-1948 | Me(S) | single bond | H | *c*-Pr | S(=O)₂CF₃ | Cl | CH₂OMe | O |
| D-1949 | H | CH₂ | Me | *c*-Pr | S(=O)₂CF₃ | Cl | CH₂OMe | O |
| D-1950 | Me | CH₂ | H | *c*-Pr | S(=O)₂CF₃ | Cl | CH₂OMe | O |
| D-1951 | H | CH(Me) | H | *c*-Pr | S(=O)₂CF₃ | Cl | CH₂OMe | O |
| D-1952 | H | CH₂CH₂ | Me | *c*-Pr | S(=O)₂CF₃ | Cl | CH₂OMe | O |
| D-1953 | Me | CH₂CH₂ | H | *c*-Pr | S(=O)₂CF₃ | Cl | CH₂OMe | O |
| D-1954 | H | single bond | Me | *c*-Pr | S(=O)₂CF₃ | Me | CH₂OMe | O |
| D-1955 | Me | single bond | H | *c*-Pr | S(=O)₂CF₃ | Me | CH₂OMe | O |
| D-1956 | Me(R) | single bond | H | *c*-Pr | S(=O)₂CF₃ | Me | CH₂OMe | O |
| D-1957 | Me(S) | single bond | H | *c*-Pr | S(=O)₂CF₃ | Me | CH₂OMe | O |
| D-1958 | H | CH₂ | Me | *c*-Pr | S(=O)₂CF₃ | Me | CH₂OMe | O |
| D-1959 | Me | CH₂ | H | *c*-Pr | S(=O)₂CF₃ | Me | CH₂OMe | O |
| D-1960 | H | CH(Me) | H | *c*-Pr | S(=O)₂CF₃ | Me | CH₂OMe | O |
| D-1961 | H | CH₂CH₂ | Me | *c*-Pr | S(=O)₂CF₃ | Me | CH₂OMe | O |
| D-1962 | Me | CH₂CH₂ | H | *c*-Pr | S(=O)₂CF₃ | Me | CH₂OMe | O |
| D-1963 | H | single bond | Me | *c*-Pr | S(=O)₂CF₃ | Cl | CH₂CN | O |
| D-1964 | Me | single bond | H | *c*-Pr | S(=O)₂CF₃ | Cl | CH₂CN | O |
| D-1965 | Me(R) | single bond | H | *c*-Pr | S(=O)₂CF₃ | Cl | CH₂CN | O |
| D-1966 | Me(S) | single bond | H | *c*-Pr | S(=O)₂CF₃ | Cl | CH₂CN | O |
| D-1967 | H | CH₂ | Me | *c*-Pr | S(=O)₂CF₃ | Cl | CH₂CN | O |
| D-1968 | Me | CH₂ | H | *c*-Pr | S(=O)₂CF₃ | Cl | CH₂CN | O |
| D-1969 | H | CH(Me) | H | *c*-Pr | S(=O)₂CF₃ | Cl | CH₂CN | O |
| D-1970 | H | CH₂CH₂ | Me | *c*-Pr | S(=O)₂CF₃ | Cl | CH₂CN | O |
| D-1971 | Me | CH₂CH₂ | H | *c*-Pr | S(=O)₂CF₃ | Cl | CH₂CN | O |
| D-1972 | H | single bond | Me | *c*-Pr | S(=O)₂CF₃ | Me | CH₂CN | O |
| D-1973 | Me | single bond | H | *c*-Pr | S(=O)₂CF₃ | Me | CH₂CN | O |
| D-1974 | Me(R) | single bond | H | *c*-Pr | S(=O)₂CF₃ | Me | CH₂CN | O |

**[Table 690]**

| Compound Number | Y | W | Z | R¹ | R² | R³ | R¹⁰ | A |
|---|---|---|---|---|---|---|---|---|
| D-1975 | Me(S) | single bond | H | *c*-Pr | S(=O)₂CF₃ | Me | CH₂CN | O |
| D-1976 | H | CH₂ | Me | *c*-Pr | S(=O)₂CF₃ | Me | CH₂CN | O |
| D-1977 | Me | CH₂ | H | *c*-Pr | S(=O)₂CF₃ | Me | CH₂CN | O |
| D-1978 | H | CH(Me) | H | *c*-Pr | S(=O)₂CF₃ | Me | CH₂CN | O |
| D-1979 | H | CH₂CH₂ | Me | *c*-Pr | S(=O)₂CF₃ | Me | CH₂CN | O |
| D-1980 | Me | CH₂CH₂ | H | *c*-Pr | S(=O)₂CF₃ | Me | CH₂CN | O |
| D-1981 | H | single bond | Me | *c*-Pr | S(=O)₂CF₃ | Cl | C(=O)Me | O |
| D-1982 | Me | single bond | H | *c*-Pr | S(=O)₂CF₃ | Cl | C(=O)Me | O |
| D-1983 | Me(R) | single bond | H | *c*-Pr | S(=O)₂CF₃ | Cl | C(=O)Me | O |
| D-1984 | Me(S) | single bond | H | *c*-Pr | S(=O)₂CF₃ | Cl | C(=O)Me | O |
| D-1985 | H | CH₂ | Me | *c*-Pr | S(=O)₂CF₃ | Cl | C(=O)Me | O |
| D-1986 | Me | CH₂ | H | *c*-Pr | S(=O)₂CF₃ | Cl | C(=O)Me | O |
| D-1987 | H | CH(Me) | H | *c*-Pr | S(=O)₂CF₃ | Cl | C(=O)Me | O |
| D-1988 | H | CH₂CH₂ | Me | *c*-Pr | S(=O)₂CF₃ | Cl | C(=O)Me | O |
| D-1989 | Me | CH₂CH₂ | H | *c*-Pr | S(=O)₂CF₃ | Cl | C(=O)Me | O |
| D-1990 | H | single bond | Me | *c*-Pr | S(=O)₂CF₃ | Me | C(=O)Me | O |
| D-1991 | Me | single bond | H | *c*-Pr | S(=O)₂CF₃ | Me | C(=O)Me | O |
| D-1992 | Me(R) | single bond | H | *c*-Pr | S(=O)₂CF₃ | Me | C(=O)Me | O |
| D-1993 | Me(S) | single bond | H | *c*-Pr | S(=O)₂CF₃ | Me | C(=O)Me | O |
| D-1994 | H | CH₂ | Me | *c*-Pr | S(=O)₂CF₃ | Me | C(=O)Me | O |
| D-1995 | Me | CH₂ | H | *c*-Pr | S(=O)₂CF₃ | Me | C(=O)Me | O |
| D-1996 | H | CH(Me) | H | *c*-Pr | S(=O)₂CF₃ | Me | C(=O)Me | O |
| D-1997 | H | CH₂CH₂ | Me | *c*-Pr | S(=O)₂CF₃ | Me | C(=O)Me | O |
| D-1998 | Me | CH₂CH₂ | H | *c*-Pr | S(=O)₂CF₃ | Me | C(=O)Me | O |
| D-1999 | H | single bond | Me | *c*-Pr | Me | Cl | Me | O |
| D-2000 | H | single bond | Et | *c*-Pr | Me | Cl | Me | O |
| D-2001 | Me | single bond | H | *c*-Pr | Me | Cl | Me | O |
| D-2002 | Me(R) | single bond | H | *c*-Pr | Me | Cl | Me | O |
| D-2003 | Me(S) | single bond | H | *c*-Pr | Me | Cl | Me | O |
| D-2004 | H | CH₂ | Me | *c*-Pr | Me | Cl | Me | O |
| D-2005 | Me | CH₂ | H | *c*-Pr | Me | Cl | Me | O |
| D-2006 | H | CH(Me) | H | *c*-Pr | Me | Cl | Me | O |
| D-2007 | H | CH₂CH₂ | Me | *c*-Pr | Me | Cl | Me | O |
| D-2008 | Me | CH₂CH₂ | H | *c*-Pr | Me | Cl | Me | O |
| D-2009 | H | single bond | Me | *c*-Pr | Me | Me | Me | O |
| D-2010 | Me | single bond | H | *c*-Pr | Me | Me | Me | O |
| D-2011 | Me(R) | single bond | H | *c*-Pr | Me | Me | Me | O |
| D-2012 | Me(S) | single bond | H | *c*-Pr | Me | Me | Me | O |
| D-2013 | H | CH₂ | Me | *c*-Pr | Me | Me | Me | O |
| D-2014 | Me | CH₂ | H | *c*-Pr | Me | Me | Me | O |
| D-2015 | H | CH(Me) | H | *c*-Pr | Me | Me | Me | O |
| D-2016 | H | CH₂CH₂ | Me | *c*-Pr | Me | Me | Me | O |
| D-2017 | Me | CH₂CH₂ | H | *c*-Pr | Me | Me | Me | O |
| D-2018 | H | single bond | Me | *c*-Pr | Me | Cl | Et | O |

**[Table 691]**

| Compound Number | Y | W | Z | R¹ | R² | R³ | R¹⁰ | A |
|---|---|---|---|---|---|---|---|---|
| D-2019 | Me | single bond | H | *c*-Pr | Me | Cl | Et | O |
| D-2020 | Me(R) | single bond | H | *c*-Pr | Me | Cl | Et | O |
| D-2021 | Me(S) | single bond | H | *c*-Pr | Me | Cl | Et | O |
| D-2022 | H | CH₂ | Me | *c*-Pr | Me | Cl | Et | O |
| D-2023 | Me | CH₂ | H | *c*-Pr | Me | Cl | Et | O |
| D-2024 | H | CH(Me) | H | *c*-Pr | Me | Cl | Et | O |
| D-2025 | H | CH₂CH₂ | Me | *c*-Pr | Me | Cl | Et | O |
| D-2026 | Me | CH₂CH₂ | H | *c*-Pr | Me | Cl | Et | O |
| D-2027 | H | single bond | Me | *c*-Pr | Me | Me | Et | O |
| D-2028 | Me | single bond | H | *c*-Pr | Me | Me | Et | O |
| D-2029 | Me(R) | single bond | H | *c*-Pr | Me | Me | Et | O |
| D-2030 | Me(S) | single bond | H | *c*-Pr | Me | Me | Et | O |
| D-2031 | H | CH₂ | Me | *c*-Pr | Me | Me | Et | O |
| D-2032 | Me | CH₂ | H | *c*-Pr | Me | Me | Et | O |
| D-2033 | H | CH(Me) | H | *c*-Pr | Me | Me | Et | O |
| D-2034 | H | CH₂CH₂ | Me | *c*-Pr | Me | Me | Et | O |
| D-2035 | Me | CH₂CH₂ | H | *c*-Pr | Me | Me | Et | O |
| D-2036 | H | single bond | Me | *c*-Pr | Me | Cl | CH₂CF₃ | O |
| D-2037 | Me | single bond | H | *c*-Pr | Me | Cl | CH₂CF₃ | O |
| D-2038 | Me(R) | single bond | H | *c*-Pr | Me | Cl | CH₂CF₃ | O |
| D-2039 | Me(S) | single bond | H | *c*-Pr | Me | Cl | CH₂CF₃ | O |
| D-2040 | H | CH₂ | Me | *c*-Pr | Me | Cl | CH₂CF₃ | O |
| D-2041 | Me | CH₂ | H | *c*-Pr | Me | Cl | CH₂CF₃ | O |
| D-2042 | H | CH(Me) | H | *c*-Pr | Me | Cl | CH₂CF₃ | O |
| D-2043 | H | CH₂CH₂ | Me | *c*-Pr | Me | Cl | CH₂CF₃ | O |
| D-2044 | Me | CH₂CH₂ | H | *c*-Pr | Me | Cl | CH₂CF₃ | O |
| D-2045 | H | single bond | Me | *c*-Pr | Me | Me | CH₂CF₃ | O |
| D-2046 | Me | single bond | H | *c*-Pr | Me | Me | CH₂CF₃ | O |
| D-2047 | Me(R) | single bond | H | *c*-Pr | Me | Me | CH₂CF₃ | O |
| D-2048 | Me(S) | single bond | H | *c*-Pr | Me | Me | CH₂CF₃ | O |
| D-2049 | H | CH₂ | Me | *c*-Pr | Me | Me | CH₂CF₃ | O |
| D-2050 | Me | CH₂ | H | *c*-Pr | Me | Me | CH₂CF₃ | O |
| D-2051 | H | CH(Me) | H | *c*-Pr | Me | Me | CH₂CF₃ | O |
| D-2052 | H | CH₂CH₂ | Me | *c*-Pr | Me | Me | CH₂CF₃ | O |
| D-2053 | Me | CH₂CH₂ | H | *c*-Pr | Me | Me | CH₂CF₃ | O |
| D-2054 | H | single bond | Me | *c*-Pr | Me | Cl | CH₂OMe | O |
| D-2055 | Me | single bond | H | *c*-Pr | Me | Cl | CH₂OMe | O |
| D-2056 | Me(R) | single bond | H | *c*-Pr | Me | Cl | CH₂OMe | O |
| D-2057 | Me(S) | single bond | H | *c*-Pr | Me | Cl | CH₂OMe | O |
| D-2058 | H | CH₂ | Me | *c*-Pr | Me | Cl | CH₂OMe | O |
| D-2059 | Me | CH₂ | H | *c*-Pr | Me | Cl | CH₂OMe | O |
| D-2060 | H | CH(Me) | H | *c*-Pr | Me | Cl | CH₂OMe | O |
| D-2061 | H | CH₂CH₂ | Me | *c*-Pr | Me | Cl | CH₂OMe | O |
| D-2062 | Me | CH₂CH₂ | H | *c*-Pr | Me | Cl | CH₂OMe | O |

**[Table 692]**

| Compound Number | Y | W | Z | R¹ | R² | R³ | R¹⁰ | A |
|---|---|---|---|---|---|---|---|---|
| D-2063 | H | single bond | Me | *c*-Pr | Me | Me | CH₂OMe | O |
| D-2064 | Me | single bond | H | *c*-Pr | Me | Me | CH₂OMe | O |
| D-2065 | Me(R) | single bond | H | *c*-Pr | Me | Me | CH₂OMe | O |
| D-2066 | Me(S) | single bond | H | *c*-Pr | Me | Me | CH₂OMe | O |
| D-2067 | H | CH₂ | Me | *c*-Pr | Me | Me | CH₂OMe | O |
| D-2068 | Me | CH₂ | H | *c*-Pr | Me | Me | CH₂OMe | O |
| D-2069 | H | CH(Me) | H | *c*-Pr | Me | Me | CH₂OMe | O |
| D-2070 | H | CH₂CH₂ | Me | *c*-Pr | Me | Me | CH₂OMe | O |
| D-2071 | Me | CH₂CH₂ | H | *c*-Pr | Me | Me | CH₂OMe | O |
| D-2072 | H | single bond | Me | *c*-Pr | Me | Cl | CH₂CN | O |
| D-2073 | Me | single bond | H | *c*-Pr | Me | Cl | CH₂CN | O |
| D-2074 | Me(R) | single bond | H | *c*-Pr | Me | Cl | CH₂CN | O |
| D-2075 | Me(S) | single bond | H | *c*-Pr | Me | Cl | CH₂CN | O |
| 0-2076 | H | CH₂ | Me | *c*-Pr | Me | Cl | CH₂CN | O |
| D-2077 | Me | CH₂ | H | *c*-Pr | Me | Cl | CH₂CN | O |
| D-2078 | H | CH(Me) | H | *c*-Pr | Me | Cl | CH₂CN | O |
| 0-2079 | H | CH₂CH₂ | Me | *c*-Pr | Me | Cl | CH₂CN | O |
| D-2080 | Me | CH₂CH₂ | H | *c*-Pr | Me | Cl | CH₂CN | O |
| D-2081 | H | single bond | Me | *c*-Pr | Me | Me | CH₂CN | O |
| D-2082 | Me | single bond | H | *c*-Pr | Me | Me | CH₂CN | O |
| 0-2083 | Me(R) | single bond | H | *c*-Pr | Me | Me | CH₂CN | O |
| D-2084 | Me(S) | single bond | H | *c*-Pr | Me | Me | CH₂CN | O |
| D-2085 | H | CH₂ | Me | *c*-Pr | Me | Me | CH₂CN | O |
| D-2086 | Me | CH₂ | H | *c*-Pr | Me | Me | CH₂CN | O |
| D-2087 | H | CH(Me) | H | *c*-Pr | Me | Me | CH₂CN | O |
| 0-2088 | H | CH₂CH₂ | Me | *c*-Pr | Me | Me | CH₂CN | O |
| D-2089 | Me | CH₂CH₂ | H | *c*-Pr | Me | Me | CH₂CN | O |
| D-2090 | H | single bond | Me | *c*-Pr | Me | Cl | C(=O)Me | O |
| D-2091 | Me | single bond | H | *c*-Pr | Me | Cl | C(=O)Me | O |
| D-2092 | Me(R) | single bond | H | *c*-Pr | Me | Cl | C(=O)Me | O |
| D-2093 | Me(S) | single bond | H | *c*-Pr | Me | Cl | C(=O)Me | O |
| D-2094 | H | CH₂ | Me | *c*-Pr | Me | Cl | C(=O)Me | O |
| D-2095 | Me | CH₂ | H | *c*-Pr | Me | Cl | C(=O)Me | O |
| D-2096 | H | CH(Me) | H | *c*-Pr | Me | Cl | C(=O)Me | O |
| D-2097 | H | CH₂CH₂ | Me | *c*-Pr | Me | Cl | C(=O)Me | O |
| D-2098 | Me | CH₂CH₂ | H | *c*-Pr | Me | Cl | C(=O)Me | O |
| D-2099 | H | single bond | Me | *c*-Pr | Me | Me | C(=O)Me | O |
| D-2100 | Me | single bond | H | *c*-Pr | Me | Me | C(=O)Me | O |
| D-2101 | Me(R) | single bond | H | *c*-Pr | Me | Me | C(=O)Me | O |
| D-2102 | Me(S) | single bond | H | *c*-Pr | Me | Me | C(=O)Me | O |
| D-2103 | H | CH₂ | Me | *c*-Pr | Me | Me | C(=O)Me | O |
| D-2104 | Me | CH₂ | H | *c*-Pr | Me | Me | C(=O)Me | O |
| D-2105 | H | CH(Me) | H | *c*-Pr | Me | Me | C(=O)Me | O |
| D-2106 | H | CH₂CH₂ | Me | *c*-Pr | Me | Me | C(=O)Me | O |

**[Table 693]**

| Compound Number | Y | W | Z | R¹ | R² | R³ | R¹⁰ | A |
|---|---|---|---|---|---|---|---|---|
| D-2107 | Me | CH₂CH₂ | H | *c*-Pr | Me | Me | C(=O)Me | O |
| D-2108 | H | single bond | Me | *c*-Pr | CH₂OMe | Cl | Me | O |
| D-2109 | H | single bond | Et | *c*-Pr | CH₂OMe | Cl | Me | O |
| D-2110 | Me | single bond | H | *c*-Pr | CH₂OMe | Cl | Me | O |
| D-2111 | Me(R) | single bond | H | *c*-Pr | CH₂OMe | Cl | Me | O |
| D-2112 | Me(S) | single bond | H | *c*-Pr | CH₂OMe | Cl | Me | O |
| D-2113 | H | CH₂ | Me | *c*-Pr | CH₂OMe | Cl | Me | O |
| D-2114 | Me | CH₂ | H | *c*-Pr | CH₂OMe | Cl | Me | O |
| D-2115 | H | CH(Me) | H | *c*-Pr | CH₂OMe | Cl | Me | O |
| D-2116 | H | CH₂CH₂ | Me | *c*-Pr | CH₂OMe | Cl | Me | O |
| D-2117 | Me | CH₂CH₂ | H | *c*-Pr | CH₂OMe | Cl | Me | O |
| D-2118 | H | single bond | Me | *c*-Pr | CH₂OMe | Me | Me | O |
| D-2119 | Me | single bond | H | *c*-Pr | CH₂OMe | Me | Me | O |
| D-2120 | Me(R) | single bond | H | *c*-Pr | CH₂OMe | Me | Me | O |
| D-2121 | Me(S) | single bond | H | *c*-Pr | CH₂OMe | Me | Me | O |
| D-2122 | H | CH₂ | Me | *c*-Pr | CH₂OMe | Me | Me | O |
| D-2123 | Me | CH₂ | H | *c*-Pr | CH₂OMe | Me | Me | O |
| D-2124 | H | CH(Me) | H | *c*-Pr | CH₂OMe | Me | Me | O |
| D-2125 | H | CH₂CH₂ | Me | *c*-Pr | CH₂OMe | Me | Me | O |
| D-2126 | Me | CH₂CH₂ | H | *c*-Pr | CH₂OMe | Me | Me | O |
| D-2127 | H | single bond | Me | *c*-Pr | CH₂OMe | Cl | Et | O |
| D-2128 | Me | single bond | H | *c*-Pr | CH₂OMe | Cl | Et | O |
| D-2129 | Me(R) | single bond | H | *c*-Pr | CH₂OMe | Cl | Et | O |
| D-2130 | Me(S) | single bond | H | *c*-Pr | CH₂OMe | Cl | Et | O |
| D-2131 | H | CH₂ | Me | *c*-Pr | CH₂OMe | Cl | Et | O |
| D-2132 | Me | CH₂ | H | *c*-Pr | CH₂OMe | Cl | Et | O |
| D-2133 | H | CH(Me) | H | *c*-Pr | CH₂OMe | Cl | Et | O |
| D-2134 | H | CH₂CH₂ | Me | *c*-Pr | CH₂OMe | Cl | Et | O |
| D-2135 | Me | CH₂CH₂ | H | *c*-Pr | CH₂OMe | Cl | Et | O |
| D-2136 | H | single bond | Me | *c*-Pr | CH₂OMe | Me | Et | O |
| D-2137 | Me | single bond | H | *c*-Pr | CH₂OMe | Me | Et | O |
| D-2138 | Me(R) | single bond | H | *c*-Pr | CH₂OMe | Me | Et | O |
| D-2139 | Me(S) | single bond | H | *c*-Pr | CH₂OMe | Me | Et | O |
| D-2140 | H | CH₂ | Me | *c*-Pr | CH₂OMe | Me | Et | O |
| D-2141 | Me | CH₂ | H | *c*-Pr | CH₂OMe | Me | Et | O |
| D-2142 | H | CH(Me) | H | *c*-Pr | CH₂OMe | Me | Et | O |
| D-2143 | H | CH₂CH₂ | Me | *c*-Pr | CH₂OMe | Me | Et | O |
| D-2144 | Me | CH₂CH₂ | H | *c*-Pr | CH₂OMe | Me | Et | O |
| D-2145 | H | single bond | Me | *c*-Pr | CH₂OMe | Cl | CH₂CF₃ | O |
| D-2146 | Me | single bond | H | *c*-Pr | CH₂OMe | Cl | CH₂CF₃ | O |
| D-2147 | Me(R) | single bond | H | *c*-Pr | CH₂OMe | Cl | CH₂CF₃ | O |
| D-2148 | Me(S) | single bond | H | *c*-Pr | CH₂OMe | Cl | CH₂CF₃ | O |
| D-2149 | H | CH₂ | Me | *c*-Pr | CH₂OMe | Cl | CH₂CF₃ | O |
| D-2150 | Me | CH₂ | H | *c*-Pr | CH₂OMe | Cl | CH₂CF₃ | O |

**[Table 694]**

| Compound Number | Y | W | z | R¹ | R² | R³ | R¹⁰ | A |
|---|---|---|---|---|---|---|---|---|
| D-2151 | H | CH(Me) | H | *c*-Pr | CH₂OMe | Cl | CH₂CF₃ | O |
| D-2152 | H | CH₂CH₂ | Me | *c*-Pr | CH₂OMe | Cl | CH₂CF₃ | O |
| D-2153 | Me | CH₂CH₂ | H | *c*-Pr | CH₂OMe | Cl | CH₂CF₃ | O |
| D-2154 | H | single bond | Me | *c*-Pr | CH₂OMe | Me | CH₂CF₃ | O |
| D-2155 | Me | single bond | H | *c*-Pr | CH₂OMe | Me | CH₂CF₃ | O |
| D-2156 | Me(R) | single bond | H | *c*-Pr | CH₂OMe | Me | CH₂CF₃ | O |
| D-2157 | Me(S) | single bond | H | *c*-Pr | CH₂OMe | Me | CH₂CF₃ | O |
| D-2158 | H | CH₂ | Me | *c*-Pr | CH₂OMe | Me | CH₂CF₃ | O |
| D-2159 | Me | CH₂ | H | *c*-Pr | CH₂OMe | Me | CH₂CF₃ | O |
| D-2160 | H | CH(Me) | H | *c*-Pr | CH₂OMe | Me | CH₂CF₃ | O |
| D-2161 | H | CH₂CH₂ | Me | *c*-Pr | CH₂OMe | Me | CH₂CF₃ | O |
| D-2162 | Me | CH₂CH₂ | H | *c*-Pr | CH₂OMe | Me | CH₂CF₃ | O |
| D-2163 | H | single bond | Me | *c*-Pr | CH₂OMe | Cl | CH₂OMe | O |
| D-2164 | Me | single bond | H | *c*-Pr | CH₂OMe | Cl | CH₂OMe | O |
| D-2165 | Me(R) | single bond | H | *c*-Pr | CH₂OMe | Cl | CH₂OMe | O |
| D-2166 | Me(S) | single bond | H | *c*-Pr | CH₂OMe | Cl | CH₂OMe | O |
| D-2167 | H | CH₂ | Me | *c*-Pr | CH₂OMe | Cl | CH₂OMe | O |
| D-2168 | Me | CH₂ | H | *c*-Pr | CH₂OMe | Cl | CH₂OMe | O |
| D-2169 | H | CH(Me) | H | *c*-Pr | CH₂OMe | Cl | CH₂OMe | O |
| D-2170 | H | CH₂CH₂ | Me | *c*-Pr | CH₂OMe | Cl | CH₂OMe | O |
| D-2171 | Me | CH₂CH₂ | H | *c*-Pr | CH₂OMe | Cl | CH₂OMe | O |
| D-2172 | H | single bond | Me | *c*-Pr | CH₂OMe | Me | CH₂OMe | O |
| D-2173 | Me | single bond | H | *c*-Pr | CH₂OMe | Me | CH₂OMe | O |
| D-2174 | Me(R) | single bond | H | *c*-Pr | CH₂OMe | Me | CH₂OMe | O |
| D-2175 | Me(S) | single bond | H | *c*-Pr | CH₂OMe | Me | CH₂OMe | O |
| D-2176 | H | CH₂ | Me | *c*-Pr | CH₂OMe | Me | CH₂OMe | O |
| D-2177 | Me | CH₂ | H | *c*-Pr | CH₂OMe | Me | CH₂OMe | O |
| D-2178 | H | CH(Me) | H | *c*-Pr | CH₂OMe | Me | CH₂OMe | O |
| D-2179 | H | CH₂CH₂ | Me | *c*-Pr | CH₂OMe | Me | CH₂OMe | O |
| D-2180 | Me | CH₂CH₂ | H | *c*-Pr | CH₂OMe | Me | CH₂OMe | O |
| D-2181 | H | single bond | Me | *c*-Pr | CH₂OMe | Cl | CH₂CN | O |
| D-2182 | Me | single bond | H | *c*-Pr | CH₂OMe | Cl | CH₂CN | O |
| D-2183 | Me(R) | single bond | H | *c*-Pr | CH₂OMe | Cl | CH₂CN | O |
| D-2184 | Me(S) | single bond | H | *c*-Pr | CH₂OMe | Cl | CH₂CN | O |
| D-2185 | H | CH₂ | Me | *c*-Pr | CH₂OMe | Cl | CH₂CN | O |
| D-2186 | Me | CH₂ | H | *c*-Pr | CH₂OMe | Cl | CH₂CN | O |
| D-2187 | H | CH(Me) | H | *c*-Pr | CH₂OMe | Cl | CH₂CN | O |
| D-2188 | H | CH₂CH₂ | Me | *c*-Pr | CH₂OMe | Cl | CH₂CN | O |
| D-2189 | Me | CH₂CH₂ | H | *c*-Pr | CH₂OMe | Cl | CH₂CN | O |
| D-2190 | H | single bond | Me | *c*-Pr | CH₂OMe | Me | CH₂CN | O |
| D-2191 | Me | single bond | H | *c*-Pr | CH₂OMe | Me | CH₂CN | O |
| D-2192 | Me(R) | single bond | H | *c*-Pr | CH₂OMe | Me | CH₂CN | O |
| D-2193 | Me(S) | single bond | H | *c*-Pr | CH₂OMe | Me | CH₂CN | O |
| D-2194 | H | CH₂ | Me | *c*-Pr | CH₂OMe | Me | CH₂CN | O |

**[Table 695]**

| Compound Number | Y | W | Z | R¹ | R² | R³ | R¹⁰ | A |
|---|---|---|---|---|---|---|---|---|
| D-2195 | Me | CH₂ | H | *c*-Pr | CH₂OMe | Me | CH₂CN | O |
| D-2196 | H | CH(Me) | H | *c*-Pr | CH₂OMe | Me | CH₂CN | O |
| D-2197 | H | CH₂CH₂ | Me | *c*-Pr | CH₂OMe | Me | CH₂CN | O |
| D-2198 | Me | CH₂CH₂ | H | *c*-Pr | CH₂OMe | Me | CH₂CN | O |
| D-2199 | H | single bond | Me | *c*-Pr | CH₂OMe | Cl | C(=O)Me | O |
| D-2200 | Me | single bond | H | *c*-Pr | CH₂OMe | Cl | C(=O)Me | O |
| D-2201 | Me(R) | single bond | H | *c*-Pr | CH₂OMe | Cl | C(=O)Me | O |
| D-2202 | Me(S) | single bond | H | *c*-Pr | CH₂OMe | Cl | C(=O)Me | O |
| D-2203 | H | CH₂ | Me | *c*-Pr | CH₂OMe | Cl | C(=O)Me | O |
| D-2204 | Me | CH₂ | H | *c*-Pr | CH₂OMe | Cl | C(=O)Me | O |
| D-2205 | H | CH(Me) | H | *c*-Pr | CH₂OMe | Cl | C(=O)Me | O |
| D-2206 | H | CH₂CH₂ | Me | *c*-Pr | CH₂OMe | Cl | C(=O)Me | O |
| D-2207 | Me | CH₂CH₂ | H | *c*-Pr | CH₂OMe | Cl | C(=O)Me | O |
| D-2208 | H | single bond | Me | *c*-Pr | CH₂OMe | Me | C(=O)Me | O |
| D-2209 | Me | single bond | H | *c*-Pr | CH₂OMe | Me | C(=O)Me | O |
| D-2210 | Me(R) | single bond | H | *c*-Pr | CH₂OMe | Me | C(=O)Me | O |
| D-2211 | Me(S) | single bond | H | *c*-Pr | CH₂OMe | Me | C(=O)Me | O |
| D-2212 | H | CH₂ | Me | *c*-Pr | CH₂OMe | Me | C(=O)Me | O |
| D-2213 | Me | CH₂ | H | *c*-Pr | CH₂OMe | Me | C(=O)Me | O |
| D-2214 | H | CH(Me) | H | *c*-Pr | CH₂OMe | Me | C(=O)Me | O |
| D-2215 | H | CH₂CH₂ | Me | *c*-Pr | CH₂OMe | Me | C(=O)Me | O |
| D-2216 | Me | CH₂CH₂ | H | *c*-Pr | CH₂OMe | Me | C(=O)Me | O |
| D-2217 | H | single bond | Me | *c*-Pr | CH₂CN | Cl | Me | O |
| D-2218 | H | single bond | Et | *c*-Pr | CH₂CN | Cl | Me | O |
| D-2219 | Me | single bond | H | *c*-Pr | CH₂CN | Cl | Me | O |
| D-2220 | Me(R) | single bond | H | *c*-Pr | CH₂CN | Cl | Me | O |
| D-2221 | Me(S) | single bond | H | *c*-Pr | CH₂CN | Cl | Me | O |
| D-2222 | H | CH₂ | Me | *c*-Pr | CH₂CN | Cl | Me | O |
| D-2223 | Me | CH₂ | H | *c*-Pr | CH₂CN | Cl | Me | O |
| D-2224 | H | CH(Me) | H | *c*-Pr | CH₂CN | Cl | Me | O |
| D-2225 | H | CH₂CH₂ | Me | *c*-Pr | CH₂CN | Cl | Me | O |
| D-2226 | Me | CH₂CH₂ | H | *c*-Pr | CH₂CN | Cl | Me | O |
| D-2227 | H | single bond | Me | *c*-Pr | CH₂CN | Me | Me | O |
| D-2228 | Me | single bond | H | *c*-Pr | CH₂CN | Me | Me | O |
| D-2229 | Me(R) | single bond | H | *c*-Pr | CH₂CN | Me | Me | O |
| D-2230 | Me(S) | single bond | H | *c*-Pr | CH₂CN | Me | Me | O |
| D-2231 | H | CH₂ | Me | *c*-Pr | CH₂CN | Me | Me | O |
| D-2232 | Me | CH₂ | H | *c*-Pr | CH₂CN | Me | Me | O |
| D-2233 | H | CH(Me) | H | *c*-Pr | CH₂CN | Me | Me | O |
| D-2234 | H | CH₂CH₂ | Me | *c*-Pr | CH₂CN | Me | Me | O |
| D-2235 | Me | CH₂CH₂ | H | *c*-Pr | CH₂CN | Me | Me | O |
| D-2236 | H | single bond | Me | *c*-Pr | CH₂CN | Cl | Et | O |
| D-2237 | Me | single bond | H | *c*-Pr | CH₂CN | Cl | Et | O |
| D-2238 | Me(R) | single bond | H | *c*-Pr | CH₂CN | Cl | Et | O |

**[Table 696]**

| Compound Number | Y | W | Z | R¹ | R² | R³ | R¹⁰ | A |
|---|---|---|---|---|---|---|---|---|
| D-2239 | Me(S) | single bond | H | *c*-Pr | CH₂CN | Cl | Et | O |
| D-2240 | H | CH₂ | Me | *c*-Pr | CH₂CN | Cl | Et | O |
| D-2241 | Me | CH₂ | H | *c*-Pr | CH₂CN | Cl | Et | O |
| D-2242 | H | CH(Me) | H | *c*-Pr | CH₂CN | Cl | Et | O |
| D-2243 | H | CH₂CH₂ | Me | *c*-Pr | CH₂CN | Cl | Et | O |
| D-2244 | Me | CH₂CH₂ | H | *c*-Pr | CH₂CN | Cl | Et | O |
| D-2245 | H | single bond | Me | *c*-Pr | CH₂CN | Me | Et | O |
| D-2246 | Me | single bond | H | *c*-Pr | CH₂CN | Me | Et | O |
| D-2247 | Me(R) | single bond | H | *c*-Pr | CH₂CN | Me | Et | O |
| D-2248 | Me(S) | single bond | H | *c*-Pr | CH₂CN | Me | Et | O |
| D-2249 | H | CH₂ | Me | *c*-Pr | CH₂CN | Me | Et | O |
| D-2250 | Me | CH₂ | H | *c*-Pr | CH₂CN | Me | Et | O |
| D-2251 | H | CH(Me) | H | *c*-Pr | CH₂CN | Me | Et | O |
| D-2252 | H | CH₂CH₂ | Me | *c*-Pr | CH₂CN | Me | Et | O |
| D-2253 | Me | CH₂CH₂ | H | *c*-Pr | CH₂CN | Me | Et | O |
| D-2254 | H | single bond | Me | *c*-Pr | CH₂CN | Cl | CH₂CF₃ | O |
| D-2255 | Me | single bond | H | *c*-Pr | CH₂CN | Cl | CH₂CF₃ | O |
| D-2256 | Me(R) | single bond | H | *c*-Pr | CH₂CN | Cl | CH₂CF₃ | O |
| D-2257 | Me(S) | single bond | H | *c*-Pr | CH₂CN | Cl | CH₂CF₃ | O |
| D-2258 | H | CH₂ | Me | *c*-Pr | CH₂CN | Cl | CH₂CF₃ | O |
| D-2259 | Me | CH₂ | H | *c*-Pr | CH₂CN | Cl | CH₂CF₃ | O |
| D-2260 | H | CH(Me) | H | *c*-Pr | CH₂CN | Cl | CH₂CF₃ | O |
| D-2261 | H | CH₂CH₂ | Me | *c*-Pr | CH₂CN | Cl | CH₂CF₃ | O |
| D-2262 | Me | CH₂CH₂ | H | *c*-Pr | CH₂CN | Cl | CH₂CF₃ | O |
| D-2263 | H | single bond | Me | *c*-Pr | CH₂CN | Me | CH₂CF₃ | O |
| D-2264 | Me | single bond | H | *c*-Pr | CH₂CN | Me | CH₂CF₃ | O |
| D-2265 | Me(R) | single bond | H | *c*-Pr | CH₂CN | Me | CH₂CF₃ | O |
| D-2266 | Me(S) | single bond | H | *c*-Pr | CH₂CN | Me | CH₂CF₃ | O |
| D-2267 | H | CH₂ | Me | *c*-Pr | CH₂CN | Me | CH₂CF₃ | O |
| D-2268 | Me | CH₂ | H | *c*-Pr | CH₂CN | Me | CH₂CF₃ | O |
| D-2269 | H | CH(Me) | H | *c*-Pr | CH₂CN | Me | CH₂CF₃ | O |
| D-2270 | H | CH₂CH₂ | Me | *c*-Pr | CH₂CN | Me | CH₂CF₃ | O |
| D-2271 | Me | CH₂CH₂ | H | *c*-Pr | CH₂CN | Me | CH₂CF₃ | O |
| D-2272 | H | single bond | Me | *c*-Pr | CH₂CN | Cl | CH₂OMe | O |
| D-2273 | Me | single bond | H | *c*-Pr | CH₂CN | Cl | CH₂OMe | O |
| D-2274 | Me(R) | single bond | H | *c*-Pr | CH₂CN | Cl | CH₂OMe | O |
| D-2275 | Me(S) | single bond | H | *c*-Pr | CH₂CN | Cl | CH₂OMe | O |
| D-2276 | H | CH₂ | Me | *c*-Pr | CH₂CN | Cl | CH₂OMe | O |
| D-2277 | Me | CH₂ | H | *c*-Pr | CH₂CN | Cl | CH₂OMe | O |
| D-2278 | H | CH(Me) | H | *c*-Pr | CH₂CN | Cl | CH₂OMe | O |
| D-2279 | H | CH₂CH₂ | Me | *c*-Pr | CH₂CN | Cl | CH₂OMe | O |
| D-2280 | Me | CH₂CH₂ | H | *c*-Pr | CH₂CN | Cl | CH₂OMe | O |
| D-2281 | H | single bond | Me | *c*-Pr | CH₂CN | Me | CH₂OMe | O |
| D-2282 | Me | single bond | H | *c*-Pr | CH₂CN | Me | CH₂OMe | O |

**[Table 697]**

| Compound Number | Y | W | Z | R¹ | R² | R³ | R¹⁰ | A |
|---|---|---|---|---|---|---|---|---|
| D-2283 | Me(R) | single bond | H | *c*-Pr | CH₂CN | Me | CH₂OMe | O |
| D-2284 | Me(S) | single bond | H | *c*-Pr | CH₂CN | Me | CH₂OMe | O |
| D-2285 | H | CH₂ | Me | *c*-Pr | CH₂CN | Me | CH₂OMe | O |
| D-2286 | Me | CH₂ | H | *c*-Pr | CH₂CN | Me | CH₂OMe | O |
| D-2287 | H | CH(Me) | H | *c*-Pr | CH₂CN | Me | CH₂OMe | O |
| D-2288 | H | CH₂CH₂ | Me | *c*-Pr | CH₂CN | Me | CH₂OMe | O |
| D-2289 | Me | CH₂CH₂ | H | *c*-Pr | CH₂CN | Me | CH₂OMe | O |
| D-2290 | H | single bond | Me | *c*-Pr | CH₂CN | Cl | CH₂CN | O |
| D-2291 | Me | single bond | H | *c*-Pr | CH₂CN | Cl | CH₂CN | O |
| D-2292 | Me(R) | single bond | H | *c*-Pr | CH₂CN | Cl | CH₂CN | O |
| D-2293 | Me(S) | single bond | H | *c*-Pr | CH₂CN | Cl | CH₂CN | O |
| D-2294 | H | CH₂ | Me | *c*-Pr | CH₂CN | Cl | CH₂CN | O |
| D-2295 | Me | CH₂ | H | *c*-Pr | CH₂CN | Cl | CH₂CN | O |
| D-2296 | H | CH(Me) | H | *c*-Pr | CH₂CN | Cl | CH₂CN | O |
| D-2297 | H | CH₂CH₂ | Me | *c*-Pr | CH₂CN | Cl | CH₂CN | O |
| D-2298 | Me | CH₂CH₂ | H | *c*-Pr | CH₂CN | Cl | CH₂CN | O |
| D-2299 | H | single bond | Me | *c*-Pr | CH₂CN | Me | CH₂CN | O |
| D-2300 | Me | single bond | H | c-Pr | CH₂CN | Me | CH₂CN | O |
| D-2301 | Me(R) | single bond | H | *c*-Pr | CH₂CN | Me | CH₂CN | O |
| D-2302 | Me(S) | single bond | H | *c*-Pr | CH₂CN | Me | CH₂CN | O |
| D-2303 | H | CH₂ | Me | *c*-Pr | CH₂CN | Me | CH₂CN | O |
| D-2304 | Me | CH₂ | H | *c*-Pr | CH₂CN | Me | CH₂CN | O |
| D-2305 | H | CH(Me) | H | *c*-Pr | CH₂CN | Me | CH₂CN | O |
| D-2306 | H | CH₂CH₂ | Me | *c*-Pr | CH₂CN | Me | CH₂CN | O |
| D-2307 | Me | CH₂CH₂ | H | *c*-Pr | CH₂CN | Me | CH₂CN | O |
| D-2308 | H | single bond | Me | *c*-Pr | CH₂CN | Cl | C(=O)Me | O |
| D-2309 | Me | single bond | H | *c*-Pr | CH₂CN | Cl | C(=O)Me | O |
| D-2310 | Me(R) | single bond | H | *c*-Pr | CH₂CN | Cl | C(=O)Me | O |
| D-2311 | Me(S) | single bond | H | *c*-Pr | CH₂CN | Cl | C(=O)Me | O |
| D-2312 | H | CH₂ | Me | *c*-Pr | CH₂CN | Cl | C(=O)Me | O |
| D-2313 | Me | CH₂ | H | *c*-Pr | CH₂CN | Cl | C(=O)Me | O |
| D-2314 | H | CH(Me) | H | *c*-Pr | CH₂CN | Cl | C(=O)Me | O |
| D-2315 | H | CH₂CH₂ | Me | *c*-Pr | CH₂CN | Cl | C(=O)Me | O |
| D-2316 | Me | CH₂CH₂ | H | *c*-Pr | CH₂CN | Cl | C(=O)Me | O |
| D-2317 | H | single bond | Me | *c*-Pr | CH₂CN | Me | C(=O)Me | O |
| D-2318 | Me | single bond | H | *c*-Pr | CH₂CN | Me | C(=O)Me | O |
| D-2319 | Me(R) | single bond | H | *c*-Pr | CH₂CN | Me | C(=O)Me | O |
| D-2320 | Me(S) | single bond | H | c-Pr | CH₂CN | Me | C(=O)Me | O |
| D-2321 | H | CH₂ | Me | *c*-Pr | CH₂CN | Me | C(=O)Me | O |
| D-2322 | Me | CH₂ | H | *c*-Pr | CH₂CN | Me | C(=O)Me | O |
| D-2323 | H | CH(Me) | H | *c*-Pr | CH₂CN | Me | C(=O)Me | O |
| D-2324 | H | CH₂CH₂ | Me | *c*-Pr | CH₂CN | Me | C(=O)Me | O |
| D-2325 | Me | CH₂CH₂ | H | *c*-Pr | CH₂CN | Me | C(=O)Me | O |
| D-2326 | H | single bond | Me | *c*-Pr | C(=O)Me | Cl | Me | O |

**[Table 698]**

| Compound Number | Y | W | Z | R¹ | R² | R³ | R¹⁰ | A |
|---|---|---|---|---|---|---|---|---|
| D-2327 | Me | single bond | H | *c*-Pr | C(=O)Me | Cl | Me | O |
| D-2328 | Me(R) | single bond | H | *c*-Pr | C(=O)Me | Cl | Me | O |
| D-2329 | Me(S) | single bond | H | *c*-Pr | C(=O)Me | Cl | Me | O |
| D-2330 | H | CH₂ | Me | *c*-Pr | C(=O)Me | Cl | Me | O |
| D-2331 | Me | CH₂ | H | *c*-Pr | C(=O)Me | Cl | Me | O |
| D-2332 | H | CH(Me) | H | *c*-Pr | C(=O)Me | Cl | Me | O |
| D-2333 | H | CH₂CH₂ | Me | *c*-Pr | C(=O)Me | Cl | Me | O |
| D-2334 | Me | CH₂CH₂ | H | *c*-Pr | C(=O)Me | Cl | Me | O |
| D-2335 | H | single bond | Me | *c*-Pr | C(=O)Me | Me | Me | O |
| D-2336 | Me | single bond | H | *c*-Pr | C(=O)Me | Me | Me | O |
| D-2337 | Me(R) | single bond | H | *c*-Pr | C(=O)Me | Me | Me | O |
| D-2338 | Me(S) | single bond | H | *c*-Pr | C(=O)Me | Me | Me | O |
| D-2339 | H | CH₂ | Me | *c*-Pr | C(=O)Me | Me | Me | O |
| D-2340 | Me | CH₂ | H | *c*-Pr | C(=O)Me | Me | Me | O |
| D-2341 | H | CH(Me) | H | *c*-Pr | C(=O)Me | Me | Me | O |
| D-2342 | H | CH₂CH₂ | Me | *c*-Pr | C(=O)Me | Me | Me | O |
| D-2343 | Me | CH₂CH₂ | H | *c*-Pr | C(=O)Me | Me | Me | O |
| D-2344 | H | single bond | Me | *c*-Pr | C(=O)Me | Cl | Et | O |
| D-2345 | Me | single bond | H | *c*-Pr | C(=O)Me | Cl | Et | O |
| D-2346 | Me(R) | single bond | H | *c*-Pr | C(=O)Me | Cl | Et | O |
| D-2347 | Me(S) | single bond | H | *c*-Pr | C(=O)Me | Cl | Et | O |
| D-2348 | H | CH₂ | Me | *c*-Pr | C(=O)Me | Cl | Et | O |
| D-2349 | Me | CH₂ | H | *c*-Pr | C(=O)Me | Cl | Et | O |
| D-2350 | H | CH(Me) | H | *c*-Pr | C(=O)Me | Cl | Et | O |
| D-2351 | H | CH₂CH₂ | Me | *c*-Pr | C(=O)Me | Cl | Et | O |
| D-2352 | Me | CH₂CH₂ | H | *c*-Pr | C(=O)Me | Cl | Et | O |
| D-2353 | H | single bond | Me | *c*-Pr | C(=O)Me | Me | Et | O |
| D-2354 | Me | single bond | H | *c*-Pr | C(=O)Me | Me | Et | O |
| D-2355 | Me(R) | single bond | H | *c*-Pr | C(=O)Me | Me | Et | O |
| D-2356 | Me(S) | single bond | H | *c*-Pr | C(=O)Me | Me | Et | O |
| D-2357 | H | CH₂ | Me | *c*-Pr | C(=O)Me | Me | Et | O |
| D-2358 | Me | CH₂ | H | *c*-Pr | C(=O)Me | Me | Et | O |
| D-2359 | H | CH(Me) | H | *c*-Pr | C(=O)Me | Me | Et | O |
| D-2360 | H | CH₂CH₂ | Me | *c*-Pr | C(=O)Me | Me | Et | O |
| D-2361 | Me | CH₂CH₂ | H | *c*-Pr | C(=O)Me | Me | Et | O |
| D-2362 | H | single bond | Me | *c*-Pr | C(=O)Me | Cl | CH₂CF₃ | O |
| D-2363 | Me | single bond | H | *c*-Pr | C(=O)Me | Cl | CH₂CF₃ | O |
| D-2364 | Me(R) | single bond | H | *c*-Pr | C(=O)Me | Cl | CH₂CF₃ | O |
| D-2365 | Me(S) | single bond | H | *c*-Pr | C(=O)Me | Cl | CH₂CF₃ | O |
| D-2366 | H | CH₂ | Me | *c*-Pr | C(=O)Me | Cl | CH₂CF₃ | O |
| D-2367 | Me | CH₂ | H | *c*-Pr | C(=O)Me | Cl | CH₂CF₃ | O |
| D-2368 | H | CH(Me) | H | *c*-Pr | C(=O)Me | Cl | CH₂CF₃ | O |
| D-2369 | H | CH₂CH₂ | Me | *c*-Pr | C(=O)Me | Cl | CH₂CF₃ | O |
| D-2370 | Me | CH₂CH₂ | H | *c*-Pr | C(=O)Me | Cl | CH₂CF₃ | O |

**[Table 699]**

| Compound Number | Y | W | Z | R¹ | R² | R³ | R¹⁰ | A |
|---|---|---|---|---|---|---|---|---|
| D-2371 | H | single bond | Me | *c*-Pr | C(=O)Me | Me | CH₂CF₃ | O |
| D-2372 | Me | single bond | H | *c*-Pr | C(=O)Me | Me | CH₂CF₃ | O |
| D-2373 | Me(R) | single bond | H | *c*-Pr | C(=O)Me | Me | CH₂CF₃ | O |
| D-2374 | Me(S) | single bond | H | *c*-Pr | C(=O)Me | Me | CH₂CF₃ | O |
| D-2375 | H | CH₂ | Me | *c*-Pr | C(=O)Me | Me | CH₂CF₃ | O |
| D-2376 | Me | CH₂ | H | *c*-Pr | C(=O)Me | Me | CH₂CF₃ | O |
| D-2377 | H | CH(Me) | H | *c*-Pr | C(=O)Me | Me | CH₂CF₃ | O |
| D-2378 | H | CH₂CH₂ | Me | *c*-Pr | C(=O)Me | Me | CH₂CF₃ | O |
| D-2379 | Me | CH₂CH₂ | H | *c*-Pr | C(=O)Me | Me | CH₂CF₃ | O |
| D-2380 | H | single bond | Me | *c*-Pr | C(=O)Me | Cl | CH₂OMe | O |
| D-2381 | Me | single bond | H | *c*-Pr | C(=O)Me | Cl | CH₂OMe | O |
| D-2382 | Me(R) | single bond | H | *c*-Pr | C(=O)Me | Cl | CH₂OMe | O |
| D-2383 | Me(S) | single bond | H | *c*-Pr | C(=O)Me | Cl | CH₂OMe | O |
| D-2384 | H | CH₂ | Me | *c*-Pr | C(=O)Me | Cl | CH₂OMe | O |
| D-2385 | Me | CH₂ | H | *c*-Pr | C(=O)Me | Cl | CH₂OMe | O |
| D-2386 | H | CH(Me) | H | *c*-Pr | C(=O)Me | Cl | CH₂OMe | O |
| D-2387 | H | CH₂CH₂ | Me | *c*-Pr | C(=O)Me | Cl | CH₂OMe | O |
| D-2388 | Me | CH₂CH₂ | H | *c*-Pr | C(=O)Me | Cl | CH₂OMe | O |
| D-2389 | H | single bond | Me | *c*-Pr | C(=O)Me | Me | CH₂OMe | O |
| D-2390 | Me | single bond | H | *c*-Pr | C(=O)Me | Me | CH₂OMe | O |
| D-2391 | Me(R) | single bond | H | *c*-Pr | C(=O)Me | Me | CH₂OMe | O |
| D-2392 | Me(S) | single bond | H | *c*-Pr | C(=O)Me | Me | CH₂OMe | O |
| D-2393 | H | CH₂ | Me | *c*-Pr | C(=O)Me | Me | CH₂OMe | O |
| D-2394 | Me | CH₂ | H | *c*-Pr | C(=O)Me | Me | CH₂OMe | O |
| D-2395 | H | CH(Me) | H | *c*-Pr | C(=O)Me | Me | CH₂OMe | O |
| D-2396 | H | CH₂CH₂ | Me | *c*-Pr | C(=O)Me | Me | CH₂OMe | O |
| D-2397 | Me | CH₂CH₂ | H | *c*-Pr | C(=O)Me | Me | CH₂OMe | O |
| D-2398 | H | single bond | Me | *c*-Pr | C(=O)Me | Cl | CH₂CN | O |
| D-2399 | Me | single bond | H | *c*-Pr | C(=O)Me | Cl | CH₂CN | O |
| D-2400 | Me(R) | single bond | H | *c*-Pr | C(=O)Me | Cl | CH₂CN | O |
| D-2401 | Me(S) | single bond | H | *c*-Pr | C(=O)Me | Cl | CH₂CN | O |
| D-2402 | H | CH₂ | Me | *c*-Pr | C(=O)Me | Cl | CH₂CN | O |
| D-2403 | Me | CH₂ | H | *c*-Pr | C(=O)Me | Cl | CH₂CN | O |
| D-2404 | H | CH(Me) | H | *c*-Pr | C(=O)Me | Cl | CH₂CN | O |
| D-2405 | H | CH₂CH₂ | Me | *c*-Pr | C(=O)Me | Cl | CH₂CN | O |
| D-2406 | Me | CH₂CH₂ | H | *c*-Pr | C(=O)Me | Cl | CH₂CN | O |
| D-2407 | H | single bond | Me | *c*-Pr | C(=O)Me | Me | CH₂CN | O |
| D-2408 | Me | single bond | H | *c*-Pr | C(=O)Me | Me | CH₂CN | O |
| D-2409 | Me(R) | single bond | H | *c*-Pr | C(=O)Me | Me | CH₂CN | O |
| D-2410 | Me(S) | single bond | H | *c*-Pr | C(=O)Me | Me | CH₂CN | O |
| D-2411 | H | CH₂ | Me | *c*-Pr | C(=O)Me | Me | CH₂CN | O |
| D-2412 | Me | CH₂ | H | *c*-Pr | C(=O)Me | Me | CH₂CN | O |
| D-2413 | H | CH(Me) | H | *c*-Pr | C(=O)Me | Me | CH₂CN | O |
| D-2414 | H | CH₂CH₂ | Me | *c*-Pr | C(=O)Me | Me | CH₂CN | O |

**[Table 700]**

| Compound Number | Y | W | Z | R¹ | R² | R³ | R¹⁰ | A |
|---|---|---|---|---|---|---|---|---|
| D-2415 | Me | CH₂CH₂ | H | *c*-Pr | C(=O)Me | Me | CH₂CN | O |
| D-2416 | H | single bond | Me | *c*-Pr | C(=O)Me | Cl | C(=O)Me | O |
| D-2417 | Me | single bond | H | *c*-Pr | C(=O)Me | Cl | C(=O)Me | O |
| D-2418 | Me(R) | single bond | H | *c*-Pr | C(=O)Me | Cl | C(=O)Me | O |
| D-2419 | Me(S) | single bond | H | *c*-Pr | C(=O)Me | Cl | C(=O)Me | O |
| D-2420 | H | CH₂ | Me | *c*-Pr | C(=O)Me | Cl | C(=O)Me | O |
| D-2421 | Me | CH₂ | H | *c*-Pr | C(=O)Me | Cl | C(=O)Me | O |
| D-2422 | H | CH(Me) | H | *c*-Pr | C(=O)Me | Cl | C(=O)Me | O |
| D-2423 | H | CH₂CH₂ | Me | *c*-Pr | C(=O)Me | Cl | C(=O)Me | O |
| D-2424 | Me | CH₂CH₂ | H | *c*-Pr | C(=O)Me | Cl | C(=O)Me | O |
| D-2425 | H | single bond | Me | *c*-Pr | C(=O)Me | Me | C(=O)Me | O |
| D-2426 | Me | single bond | H | *c*-Pr | C(=O)Me | Me | C(=O)Me | O |
| D-2427 | Me(R) | single bond | H | *c*-Pr | C(=O)Me | Me | C(=O)Me | O |
| D-2428 | Me(S) | single bond | H | *c*-Pr | C(=O)Me | Me | C(=O)Me | O |
| D-2429 | H | CH₂ | Me | *c*-Pr | C(=O)Me | Me | C(=O)Me | O |
| D-2430 | Me | CH₂ | H | *c*-Pr | C(=O)Me | Me | C(=O)Me | O |
| D-2431 | H | CH(Me) | H | *c*-Pr | C(=O)Me | Me | C(=O)Me | O |
| D-2432 | H | CH₂CH₂ | Me | *c*-Pr | C(=O)Me | Me | C(=O)Me | O |
| D-2433 | Me | CH₂CH₂ | H | *c*-Pr | C(=O)Me | Me | C(=O)Me | O |
| D-2434 | H | single bond | Me | *c*-Pr | C(=O)(*i*-Pr) | Cl | Me | O |
| D-2435 | Me | single bond | H | *c*-Pr | C(=O)(*i*-Pr) | Cl | Me | O |
| D-2436 | Me(R) | single bond | H | *c*-Pr | C(=O)(*i*-Pr) | Cl | Me | O |
| D-2437 | Me(S) | single bond | H | *c*-Pr | C(=O)(*i*-Pr) | Cl | Me | O |
| D-2438 | H | CH₂ | Me | *c*-Pr | C(=O)(*i*-Pr) | Cl | Me | O |
| D-2439 | Me | CH₂ | H | *c*-Pr | C(=O)(*i*-Pr) | Cl | Me | O |
| D-2440 | H | CH(Me) | H | *c*-Pr | C(=O)(*i*-Pr) | Cl | Me | O |
| D-2441 | H | CH₂CH₂ | Me | *c*-Pr | C(=O)(i-Pr) | Cl | Me | O |
| D-2442 | Me | CH₂CH₂ | H | *c*-Pr | C(=O)(*i*-Pr) | Cl | Me | O |
| D-2443 | H | single bond | Me | *c*-Pr | C(=O)(*i*-Pr) | Me | Me | O |
| D-2444 | Me | single bond | H | *c*-Pr | C(=O)(*i*-Pr) | Me | Me | O |
| D-2445 | Me(R) | single bond | H | *c*-Pr | C(=O)(*i*-Pr) | Me | Me | O |
| D-2446 | Me(S) | single bond | H | *c*-Pr | C(=O)(*i*-Pr) | Me | Me | O |
| D-2447 | H | CH₂ | Me | *c*-Pr | C(=O)(*i*-Pr) | Me | Me | O |
| D-2448 | Me | CH₂ | H | *c*-Pr | C(=O)(*i*-Pr) | Me | Me | O |
| D-2449 | H | CH(Me) | H | *c*-Pr | C(=O)(*i*-Pr) | Me | Me | O |
| D-2450 | H | CH₂CH₂ | Me | *c*-Pr | C(=O)(*i*-Pr) | Me | Me | O |
| D-2451 | Me | CH₂CH₂ | H | *c*-Pr | C(=O)(*i*-Pr) | Me | Me | O |
| D-2452 | H | single bond | Me | *c*-Pr | C(=O)(*i*-Pr) | Cl | Et | O |
| D-2453 | Me | single bond | H | *c*-Pr | C(=O)(*i*-Pr) | Cl | Et | O |
| D-2454 | Me(R) | single bond | H | *c*-Pr | C(=O)(*i*-Pr) | Cl | Et | O |
| D-2455 | Me(S) | single bond | H | *c*-Pr | C(=O)(*i*-Pr) | Cl | Et | O |
| D-2456 | H | CH₂ | Me | *c*-Pr | C(=O)(*i*-Pr) | Cl | Et | O |
| D-2457 | Me | CH₂ | H | *c*-Pr | C(=O)(*i*-Pr) | Cl | Et | O |
| D-2458 | H | CH(Me) | H | *c*-Pr | C(=O)(*i*-Pr) | Cl | Et | O |

**[Table 701]**

| Compound Number | Y | W | Z | R¹ | R² | R³ | R¹⁰ | A |
|---|---|---|---|---|---|---|---|---|
| D-2459 | H | CH₂CH₂ | Me | *c*-Pr | C(=O)(*i*-Pr) | Cl | Et | O |
| D-2460 | Me | CH₂CH₂ | H | *c*-Pr | C(=O)(*i*-Pr) | Cl | Et | O |
| D-2461 | H | single bond | Me | *c*-Pr | C(=O)(*i*-Pr) | Me | Et | O |
| D-2462 | Me | single bond | H | *c*-Pr | C(=O)(*i*-Pr) | Me | Et | O |
| D-2463 | Me(R) | single bond | H | *c*-Pr | C(=O)(*i*-Pr) | Me | Et | O |
| D-2464 | Me(S) | single bond | H | *c*-Pr | C(=O)(*i*-Pr) | Me | Et | O |
| D-2465 | H | CH₂ | Me | *c*-Pr | C(=O)(*i*-Pr) | Me | Et | O |
| D-2466 | Me | CH₂ | H | *c*-Pr | C(=O)(*i*-Pr) | Me | Et | O |
| D-2467 | H | CH(Me) | H | *c*-Pr | C(=O)(*i*-Pr) | Me | Et | O |
| D-2468 | H | CH₂CH₂ | Me | *c*-Pr | C(=O)(*i*-Pr) | Me | Et | O |
| D-2469 | Me | CH₂CH₂ | H | *c*-Pr | C(=O)(*i*-Pr) | Me | Et | O |
| D-2470 | H | single bond | Me | *c*-Pr | C(=O)(*i*-Pr) | Cl | CH₂CF₃ | O |
| D-2471 | Me | single bond | H | *c*-Pr | C(=O)(*i*-Pr) | Cl | CH₂CF₃ | O |
| D-2472 | Me(R) | single bond | H | *c*-Pr | C(=O)(*i*-Pr) | Cl | CH₂CF₃ | O |
| D-2473 | Me(S) | single bond | H | *c*-Pr | C(=O)(*i*-Pr) | Cl | CH₂CF₃ | O |
| D-2474 | H | CH₂ | Me | *c*-Pr | C(=O)(*i*-Pr) | Cl | CH₂CF₃ | O |
| D-2475 | Me | CH₂ | H | *c*-Pr | C(=O)(*i*-Pr) | Cl | CH₂CF₃ | O |
| D-2476 | H | CH(Me) | H | *c*-Pr | C(=O)(*i*-Pr) | Cl | CH₂CF₃ | O |
| D-2477 | H | CH₂CH₂ | Me | *c*-Pr | C(=O)(*i*-Pr) | Cl | CH₂CF₃ | O |
| D-2478 | Me | CH₂CH₂ | H | *c*-Pr | C(=O)(*i*-Pr) | Cl | CH₂CF₃ | O |
| D-2479 | H | single bond | Me | *c*-Pr | C(=O)(*i*-Pr) | Me | CH₂CF₃ | O |
| D-2480 | Me | single bond | H | *c*-Pr | C(=O)(*i*-Pr) | Me | CH₂CF₃ | O |
| D-2481 | Me(R) | single bond | H | *c*-Pr | C(=O)(*i*-Pr) | Me | CH₂CF₃ | O |
| D-2482 | Me(S) | single bond | H | *c*-Pr | C(=O)(*i*-Pr) | Me | CH₂CF₃ | O |
| D-2483 | H | CH₂ | Me | *c*-Pr | C(=O)(*i*-Pr) | Me | CH₂CF₃ | O |
| D-2484 | Me | CH₂ | H | *c*-Pr | C(=O)(*i*-Pr) | Me | CH₂CF₃ | O |
| D-2485 | H | CH(Me) | H | *c*-Pr | C(=O)(*i*-Pr) | Me | CH₂CF₃ | O |
| D-2486 | H | CH₂CH₂ | Me | *c*-Pr | C(=O)(*i*-Pr) | Me | CH₂CF₃ | O |
| D-2487 | Me | CH₂CH₂ | H | *c*-Pr | C(=O)(*i*-Pr) | Me | CH₂CF₃ | O |
| D-2488 | H | single bond | Me | *c*-Pr | C(=O)(*i*-Pr) | Cl | CH₂OMe | O |
| D-2489 | Me | single bond | H | *c*-Pr | C(=O)(*i*-Pr) | Cl | CH₂OMe | O |
| D-2490 | Me(R) | single bond | H | *c*-Pr | C(=O)(*i*-Pr) | Cl | CH₂OMe | O |
| D-2491 | Me(S) | single bond | H | *c*-Pr | C(=O)(*i*-Pr) | Cl | CH₂OMe | O |
| D-2492 | H | CH₂ | Me | *c*-Pr | C(=O)(*i*-Pr) | Cl | CH₂OMe | O |
| D-2493 | Me | CH₂ | H | *c*-Pr | C(=O)(*i*-Pr) | Cl | CH₂OMe | O |
| D-2494 | H | CH(Me) | H | *c*-Pr | C(=O)(*i*-Pr) | Cl | CH₂OMe | O |
| D-2495 | H | CH₂CH₂ | Me | *c*-Pr | C(=O)(*i*-Pr) | Cl | CH₂OMe | O |
| D-2496 | Me | CH₂CH₂ | H | *c*-Pr | C(=O)(*i*-Pr) | Cl | CH₂OMe | O |
| D-2497 | H | single bond | Me | *c*-Pr | C(=O)(*i*-Pr) | Me | CH₂OMe | O |
| D-2498 | Me | single bond | H | *c*-Pr | C(=O)(*i*-Pr) | Me | CH₂OMe | O |
| D-2499 | Me(R) | single bond | H | *c*-Pr | C(=O)(*i*-Pr) | Me | CH₂OMe | O |
| D-2500 | Me(S) | single bond | H | *c*-Pr | C(=O)(*i*-Pr) | Me | CH₂OMe | O |
| D-2501 | H | CH₂ | Me | *c*-Pr | C(=O)(*i*-Pr) | Me | CH₂OMe | O |
| D-2502 | Me | CH₂ | H | *c*-Pr | C(=O)(*i*-Pr) | Me | CH₂OMe | O |

**[Table 702]**

| Compound Number | Y | W | Z | R¹ | R² | R³ | R¹⁰ | A |
|---|---|---|---|---|---|---|---|---|
| D-2503 | H | CH(Me) | H | *c*-Pr | C(=O)(*i*-Pr) | Me | CH₂OMe | O |
| D-2504 | H | CH₂CH₂ | Me | *c*-Pr | C(=O)(*i*-Pr) | Me | CH₂OMe | O |
| D-2505 | Me | CH₂CH₂ | H | *c*-Pr | C(=O)(*i*-Pr) | Me | CH₂OMe | O |
| D-2506 | H | single bond | Me | *c*-Pr | C(=O)(*i*-Pr) | Cl | CH₂CN | O |
| D-2507 | Me | single bond | H | *c*-Pr | C(=O)(*i*-Pr) | Cl | CH₂CN | O |
| D-2508 | Me(R) | single bond | H | *c*-Pr | C(=O)(*i*-Pr) | Cl | CH₂CN | O |
| D-2509 | Me(S) | single bond | H | *c*-Pr | C(=O)(*i*-Pr) | Cl | CH₂CN | O |
| D-2510 | H | CH₂ | Me | *c*-Pr | C(=O)(*i*-Pr) | Cl | CH₂CN | O |
| D-2511 | Me | CH₂ | H | *c*-Pr | C(=O)(*i*-Pr) | Cl | CH₂CN | O |
| D-2512 | H | CH(Me) | H | *c*-Pr | C(=O)(*i*-Pr) | Cl | CH₂CN | O |
| D-2513 | H | CH₂CH₂ | Me | *c*-Pr | C(=O)(*i*-Pr) | Cl | CH₂CN | O |
| D-2514 | Me | CH₂CH₂ | H | *c*-Pr | C(=O)(*i*-Pr) | Cl | CH₂CN | O |
| D-2515 | H | single bond | Me | *c*-Pr | C(=O)(*i*-Pr) | Me | CH₂CN | O |
| D-2516 | Me | single bond | H | *c*-Pr | C(=O)(*i*-Pr) | Me | CH₂CN | O |
| D-2517 | Me(R) | single bond | H | *c*-Pr | C(=O)(*i*-Pr) | Me | CH₂CN | O |
| D-2518 | Me(S) | single bond | H | *c*-Pr | C(=O)(*i*-Pr) | Me | CH₂CN | O |
| D-2519 | H | CH₂ | Me | *c*-Pr | C(=O)(*i*-Pr) | Me | CH₂CN | O |
| D-2520 | Me | CH₂ | H | *c*-Pr | C(=O)(*i*-Pr) | Me | CH₂CN | O |
| D-2521 | H | CH(Me) | H | *c*-Pr | C(=O)(*i*-Pr) | Me | CH₂CN | O |
| D-2522 | H | CH₂CH₂ | Me | *c*-Pr | C(=O)(*i*-Pr) | Me | CH₂CN | O |
| D-2523 | Me | CH₂CH₂ | H | *c*-Pr | C(=O)(*i*-Pr) | Me | CH₂CN | O |
| D-2524 | H | single bond | Me | *c*-Pr | C(=O)(*i*-Pr) | Cl | C(=O)Me | O |
| D-2525 | Me | single bond | H | *c*-Pr | C(=O)(*i*-Pr) | Cl | C(=O)Me | O |
| D-2526 | Me(R) | single bond | H | *c*-Pr | C(=O)(*i*-Pr) | Cl | C(=O)Me | O |
| D-2527 | Me(S) | single bond | H | *c*-Pr | C(=O)(*i*-Pr) | Cl | C(=O)Me | O |
| D-2528 | H | CH₂ | Me | *c*-Pr | C(=O)(*i*-Pr) | Cl | C(=O)Me | O |
| D-2529 | Me | CH₂ | H | *c*-Pr | C(=O)(*i*-Pr) | Cl | C(=O)Me | O |
| D-2530 | H | CH(Me) | H | *c*-Pr | C(=O)(*i*-Pr) | Cl | C(=O)Me | O |
| D-2531 | H | CH₂CH₂ | Me | *c*-Pr | C(=O)(*i*-Pr) | Cl | C(=O)Me | O |
| D-2532 | Me | CH₂CH₂ | H | *c*-Pr | C(=O)(*i*-Pr) | Cl | C(=O)Me | O |
| D-2533 | H | single bond | Me | *c*-Pr | C(=O)(*i*-Pr) | Me | C(=O)Me | O |
| D-2534 | Me | single bond | H | *c*-Pr | C(=O)(*i*-Pr) | Me | C(=O)Me | O |
| D-2535 | Me(R) | single bond | H | *c*-Pr | C(=O)(*i*-Pr) | Me | C(=O)Me | O |
| D-2536 | Me(S) | single bond | H | *c*-Pr | C(=O)(*i*-Pr) | Me | C(=O)Me | O |
| D-2537 | H | CH₂ | Me | *c*-Pr | C(=O)(*i*-Pr) | Me | C(=O)Me | O |
| D-2538 | Me | CH₂ | H | *c*-Pr | C(=O)(*i*-Pr) | Me | C(=O)Me | O |
| D-2539 | H | CH(Me) | H | *c*-Pr | C(=O)(*i*-Pr) | Me | C(=O)Me | O |
| D-2540 | H | CH₂CH₂ | Me | *c*-Pr | C(=O)(*i*-Pr) | Me | C(=O)Me | O |
| D-2541 | Me | CH₂CH₂ | H | *c*-Pr | C(=O)(*i*-Pr) | Me | C(=O)Me | O |
| D-2542 | H | single bond | Me | *c*-Pr | C(=O)Ph | Cl | Me | O |
| D-2543 | Me | single bond | H | *c*-Pr | C(=O)Ph | Cl | Me | O |
| D-2544 | Me(R) | single bond | H | *c*-Pr | C(=O)Ph | Cl | Me | O |
| D-2545 | Me(S) | single bond | H | *c*-Pr | C(=O)Ph | Cl | Me | O |
| D-2546 | H | CH₂ | Me | *c*-Pr | C(=O)Ph | Cl | Me | O |

**[Table 703]**

| Compound Number | Y | W | Z | R¹ | R² | R³ | R¹⁰ | A |
|---|---|---|---|---|---|---|---|---|
| D-2547 | Me | CH₂ | H | *c*-Pr | C(=O)Ph | Cl | Me | O |
| D-2548 | H | CH(Me) | H | *c*-Pr | C(=O)Ph | Cl | Me | O |
| D-2549 | H | CH₂CH₂ | Me | *c*-Pr | C(=O)Ph | Cl | Me | O |
| D-2550 | Me | CH₂CH₂ | H | *c*-Pr | C(=O)Ph | Cl | Me | O |
| D-2551 | H | single bond | Me | *c*-Pr | C(=O)Ph | Me | Me | O |
| D-2552 | Me | single bond | H | *c*-Pr | C(=O)Ph | Me | Me | O |
| D-2553 | Me(R) | single bond | H | *c*-Pr | C(=O)Ph | Me | Me | O |
| D-2554 | Me(S) | single bond | H | *c*-Pr | C(=O)Ph | Me | Me | O |
| D-2555 | H | CH₂ | Me | *c*-Pr | C(=O)Ph | Me | Me | O |
| D-2556 | Me | CH₂ | H | *c*-Pr | C(=O)Ph | Me | Me | O |
| D-2557 | H | CH(Me) | H | *c*-Pr | C(=O)Ph | Me | Me | O |
| D-2558 | H | CH₂CH₂ | Me | *c*-Pr | C(=O)Ph | Me | Me | O |
| D-2559 | Me | CH₂CH₂ | H | *c*-Pr | C(=O)Ph | Me | Me | O |
| D-2560 | H | single bond | Me | *c*-Pr | C(=O)Ph | Cl | Et | O |
| D-2561 | Me | single bond | H | *c*-Pr | C(=O)Ph | Cl | Et | O |
| D-2562 | Me(R) | single bond | H | *c*-Pr | C(=O)Ph | Cl | Et | O |
| D-2563 | Me(S) | single bond | H | *c*-Pr | C(=O)Ph | Cl | Et | O |
| D-2564 | H | CH₂ | Me | *c*-Pr | C(=O)Ph | Cl | Et | O |
| D-2565 | Me | CH₂ | H | *c*-Pr | C(=O)Ph | Cl | Et | O |
| D-2566 | H | CH(Me) | H | *c*-Pr | C(=O)Ph | Cl | Et | O |
| D-2567 | H | CH₂CH₂ | Me | *c*-Pr | C(=O)Ph | Cl | Et | O |
| D-2568 | Me | CH₂CH₂ | H | *c*-Pr | C(=O)Ph | Cl | Et | O |
| D-2569 | H | single bond | Me | *c*-Pr | C(=O)Ph | Me | Et | O |
| D-2570 | Me | single bond | H | *c*-Pr | C(=O)Ph | Me | Et | O |
| D-2571 | Me(R) | single bond | H | *c*-Pr | C(=O)Ph | Me | Et | O |
| D-2572 | Me(S) | single bond | H | *c*-Pr | C(=O)Ph | Me | Et | O |
| D-2573 | H | CH₂ | Me | *c*-Pr | C(=O)Ph | Me | Et | O |
| D-2574 | Me | CH₂ | H | *c*-Pr | C(=O)Ph | Me | Et | O |
| D-2575 | H | CH(Me) | H | *c*-Pr | C(=O)Ph | Me | Et | O |
| D-2576 | H | CH₂CH₂ | Me | *c*-Pr | C(=O)Ph | Me | Et | O |
| D-2577 | Me | CH₂CH₂ | H | *c*-Pr | C(=O)Ph | Me | Et | O |
| D-2578 | H | single bond | Me | *c*-Pr | C(=O)Ph | Cl | CH₂CF₃ | O |
| D-2579 | Me | single bond | H | *c*-Pr | C(=O)Ph | Cl | CH₂CF₃ | O |
| D-2580 | Me(R) | single bond | H | *c*-Pr | C(=O)Ph | Cl | CH₂CF₃ | O |
| D-2581 | Me(S) | single bond | H | *c*-Pr | C(=O)Ph | Cl | CH₂CF₃ | O |
| D-2582 | H | CH₂ | Me | *c*-Pr | C(=O)Ph | Cl | CH₂CF₃ | O |
| D-2583 | Me | CH₂ | H | *c*-Pr | C(=O)Ph | Cl | CH₂CF₃ | O |
| D-2584 | H | CH(Me) | H | *c*-Pr | C(=O)Ph | Cl | CH₂CF₃ | O |
| D-2585 | H | CH₂CH₂ | Me | *c*-Pr | C(=O)Ph | Cl | CH₂CF₃ | O |
| D-2586 | Me | CH₂CH₂ | H | *c*-Pr | C(=O)Ph | Cl | CH₂CF₃ | O |
| D-2587 | H | single bond | Me | *c*-Pr | C(=O)Ph | Me | CH₂CF₃ | O |
| D-2588 | Me | single bond | H | *c*-Pr | C(=O)Ph | Me | CH₂CF₃ | O |
| D-2589 | Me(R) | single bond | H | *c*-Pr | C(=O)Ph | Me | CH₂CF₃ | O |
| D-2590 | Me(S) | single bond | H | *c*-Pr | C(=O)Ph | Me | CH₂CF₃ | O |

**[Table 704]**

| Compound Number | Y | W | Z | R¹ | R² | R³ | R¹⁰ | A |
|---|---|---|---|---|---|---|---|---|
| D-2591 | H | CH₂ | Me | *c*-Pr | C(=O)Ph | Me | CH₂CF₃ | O |
| D-2592 | Me | CH₂ | H | *c*-Pr | C(=O)Ph | Me | CH₂CF₃ | O |
| D-2593 | H | CH(Me) | H | *c*-Pr | C(=O)Ph | Me | CH₂CF₃ | O |
| D-2594 | H | CH₂CH₂ | Me | *c*-Pr | C(=O)Ph | Me | CH₂CF₃ | O |
| D-2595 | Me | CH₂CH₂ | H | *c*-Pr | C(=O)Ph | Me | CH₂CF₃ | O |
| D-2596 | H | single bond | Me | *c*-Pr | C(=O)Ph | Cl | CH₂OMe | O |
| D-2597 | Me | single bond | H | *c*-Pr | C(=O)Ph | Cl | CH₂OMe | O |
| D-2598 | Me(R) | single bond | H | *c*-Pr | C(=O)Ph | Cl | CH₂OMe | O |
| D-2599 | Me(S) | single bond | H | *c*-Pr | C(=O)Ph | Cl | CH₂OMe | O |
| D-2600 | H | CH₂ | Me | *c*-Pr | C(=O)Ph | Cl | CH₂OMe | O |
| D-2601 | Me | CH₂ | H | *c*-Pr | C(=O)Ph | Cl | CH₂OMe | O |
| D-2602 | H | CH(Me) | H | *c*-Pr | C(=O)Ph | Cl | CH₂OMe | O |
| D-2603 | H | CH₂CH₂ | Me | *c*-Pr | C(=O)Ph | Cl | CH₂OMe | O |
| D-2604 | Me | CH₂CH₂ | H | *c*-Pr | C(=O)Ph | Cl | CH₂OMe | O |
| D-2605 | H | single bond | Me | *c*-Pr | C(=O)Ph | Me | CH₂OMe | O |
| D-2606 | Me | single bond | H | *c*-Pr | C(=O)Ph | Me | CH₂OMe | O |
| D-2607 | Me(R) | single bond | H | *c*-Pr | C(=O)Ph | Me | CH₂OMe | O |
| D-2608 | Me(S) | single bond | H | *c*-Pr | C(=O)Ph | Me | CH₂OMe | O |
| D-2609 | H | CH₂ | Me | *c*-Pr | C(=O)Ph | Me | CH₂OMe | O |
| D-2610 | Me | CH₂ | H | *c*-Pr | C(=O)Ph | Me | CH₂OMe | O |
| D-2611 | H | CH(Me) | H | *c*-Pr | C(=O)Ph | Me | CH₂OMe | O |
| D-2612 | H | CH₂CH₂ | Me | *c*-Pr | C(=O)Ph | Me | CH₂OMe | O |
| D-2613 | Me | CH₂CH₂ | H | *c*-Pr | C(=O)Ph | Me | CH₂OMe | O |
| D-2614 | H | single bond | Me | *c*-Pr | C(=O)Ph | Cl | CH₂CN | O |
| D-2615 | Me | single bond | H | *c*-Pr | C(=O)Ph | Cl | CH₂CN | O |
| D-2616 | Me(R) | single bond | H | *c*-Pr | C(=O)Ph | Cl | CH₂CN | O |
| D-2617 | Me(S) | single bond | H | *c*-Pr | C(=O)Ph | Cl | CH₂CN | O |
| D-2618 | H | CH₂ | Me | *c*-Pr | C(=O)Ph | Cl | CH₂CN | O |
| D-2619 | Me | CH₂ | H | *c*-Pr | C(=O)Ph | Cl | CH₂CN | O |
| D-2620 | H | CH(Me) | H | *c*-Pr | C(=O)Ph | Cl | CH₂CN | O |
| D-2621 | H | CH₂CH₂ | Me | *c*-Pr | C(=O)Ph | Cl | CH₂CN | O |
| D-2622 | Me | CH₂CH₂ | H | *c*-Pr | C(=O)Ph | Cl | CH₂CN | O |
| D-2623 | H | single bond | Me | *c*-Pr | C(=O)Ph | Me | CH₂CN | O |
| D-2624 | Me | single bond | H | *c*-Pr | C(=O)Ph | Me | CH₂CN | O |
| D-2625 | Me(R) | single bond | H | *c*-Pr | C(=O)Ph | Me | CH₂CN | O |
| D-2626 | Me(S) | single bond | H | *c*-Pr | C(=O)Ph | Me | CH₂CN | O |
| D-2627 | H | CH₂ | Me | *c*-Pr | C(=O)Ph | Me | CH₂CN | O |
| D-2628 | Me | CH₂ | H | *c*-Pr | C(=O)Ph | Me | CH₂CN | O |
| D-2629 | H | CH(Me) | H | *c*-Pr | C(=O)Ph | Me | CH₂CN | O |
| D-2630 | H | CH₂CH₂ | Me | *c*-Pr | C(=O)Ph | Me | CH₂CN | O |
| D-2631 | Me | CH₂CH₂ | H | *c*-Pr | C(=O)Ph | Me | CH₂CN | O |
| D-2632 | H | single bond | Me | *c*-Pr | C(=O)Ph | Cl | C(=O)Me | O |
| D-2633 | Me | single bond | H | *c*-Pr | C(=O)Ph | Cl | C(=O)Me | O |
| D-2634 | Me(R) | single bond | H | *c*-Pr | C(=O)Ph | Cl | C(=O)Me | O |

**[Table 705]**

| Compound Number | Y | W | Z | R¹ | R² | R³ | R¹⁰ | A |
|---|---|---|---|---|---|---|---|---|
| D-2635 | Me(S) | single bond | H | *c*-Pr | C(=O)Ph | Cl | C(=O)Me | O |
| D-2636 | H | CH₂ | Me | *c*-Pr | C(=O)Ph | Cl | C(=O)Me | O |
| D-2637 | Me | CH₂ | H | *c*-Pr | C(=O)Ph | Cl | C(=O)Me | O |
| D-2638 | H | CH(Me) | H | *c*-Pr | C(=O)Ph | Cl | C(=O)Me | O |
| D-2639 | H | CH₂CH₂ | Me | *c*-Pr | C(=O)Ph | Cl | C(=O)Me | O |
| D-2640 | Me | CH₂CH₂ | H | *c*-Pr | C(=O)Ph | Cl | C(=O)Me | O |
| D-2641 | H | single bond | Me | *c*-Pr | C(=O)Ph | Me | C(=O)Me | O |
| D-2642 | Me | single bond | H | *c*-Pr | C(=O)Ph | Me | C(=O)Me | O |
| D-2643 | Me(R) | single bond | H | *c*-Pr | C(=O)Ph | Me | C(=O)Me | O |
| D-2644 | Me(S) | single bond | H | *c*-Pr | C(=O)Ph | Me | C(=O)Me | O |
| D-2645 | H | CH₂ | Me | *c*-Pr | C(=O)Ph | Me | C(=O)Me | O |
| D-2646 | Me | CH₂ | H | *c*-Pr | C(=O)Ph | Me | C(=O)Me | O |
| D-2647 | H | CH(Me) | H | *c*-Pr | C(=O)Ph | Me | C(=O)Me | O |
| D-2648 | H | CH₂CH₂ | Me | *c*-Pr | C(=O)Ph | Me | C(=O)Me | O |
| D-2649 | Me | CH₂CH₂ | H | *c*-Pr | C(=O)Ph | Me | C(=O)Me | O |
| D-2650 | H | single bond | Me | *c*-Pr | C(=O)OEt | Cl | Me | O |
| D-2651 | H | single bond | Et | *c*-Pr | C(=O)OEt | Cl | Me | O |
| D-2652 | Me | single bond | H | *c*-Pr | C(=O)OEt | Cl | Me | O |
| D-2653 | Me(R) | single bond | H | *c*-Pr | C(=O)OEt | Cl | Me | O |
| D-2654 | Me(S) | single bond | H | *c*-Pr | C(=O)OEt | Cl | Me | O |
| D-2655 | H | CH₂ | Me | *c*-Pr | C(=O)OEt | Cl | Me | O |
| D-2656 | Me | CH₂ | H | *c*-Pr | C(=O)OEt | Cl | Me | O |
| D-2657 | H | CH(Me) | H | *c*-Pr | C(=O)OEt | Cl | Me | O |
| D-2658 | H | CH₂CH₂ | Me | *c*-Pr | C(=O)OEt | Cl | Me | O |
| D-2659 | Me | CH₂CH₂ | H | *c*-Pr | C(=O)OEt | Cl | Me | O |
| D-2660 | H | single bond | Me | *c*-Pr | C(=O)OEt | Me | Me | O |
| D-2661 | Me | single bond | H | *c*-Pr | C(=O)OEt | Me | Me | O |
| D-2662 | Me(R) | single bond | H | *c*-Pr | C(=O)OEt | Me | Me | O |
| D-2663 | Me(S) | single bond | H | *c*-Pr | C(=O)OEt | Me | Me | O |
| D-2664 | H | CH₂ | Me | *c*-Pr | C(=O)OEt | Me | Me | O |
| D-2665 | Me | CH₂ | H | *c*-Pr | C(=O)OEt | Me | Me | O |
| D-2666 | H | CH(Me) | H | *c*-Pr | C(=O)OEt | Me | Me | O |
| D-2667 | H | CH₂CH₂ | Me | *c*-Pr | C(=O)OEt | Me | Me | O |
| D-2668 | Me | CH₂CH₂ | H | *c*-Pr | C(=O)OEt | Me | Me | O |
| D-2669 | H | single bond | Me | *c*-Pr | C(=O)OEt | Cl | Et | O |
| D-2670 | Me | single bond | H | *c*-Pr | C(=O)OEt | Cl | Et | O |
| D-2671 | Me(R) | single bond | H | *c*-Pr | C(=O)OEt | Cl | Et | O |
| D-2672 | Me(S) | single bond | H | *c*-Pr | C(=O)OEt | Cl | Et | O |
| D-2673 | H | CH₂ | Me | *c*-Pr | C(=O)OEt | Cl | Et | O |
| D-2674 | Me | CH₂ | H | *c*-Pr | C(=O)OEt | Cl | Et | O |
| D-2675 | H | CH(Me) | H | *c*-Pr | C(=O)OEt | Cl | Et | O |
| D-2676 | H | CH₂CH₂ | Me | *c*-Pr | C(=O)OEt | Cl | Et | O |
| D-2677 | Me | CH₂CH₂ | H | *c*-Pr | C(=O)OEt | Cl | Et | O |
| D-2678 | H | single bond | Me | *c*-Pr | C(=O)OEt | Me | Et | O |

**[Table 706]**

| Compound Number | Y | W | Z | R¹ | R² | R³ | R¹⁰ | A |
|---|---|---|---|---|---|---|---|---|
| D-2679 | Me | single bond | H | *c*-Pr | C(=O)OEt | Me | Et | O |
| D-2680 | Me(R) | single bond | H | *c*-Pr | C(=O)OEt | Me | Et | O |
| D-2681 | Me(S) | single bond | H | *c*-Pr | C(=O)OEt | Me | Et | O |
| D-2682 | H | CH₂ | Me | *c*-Pr | C(=O)OEt | Me | Et | O |
| D-2683 | Me | CH₂ | H | *c*-Pr | C(=O)OEt | Me | Et | O |
| D-2684 | H | CH(Me) | H | *c*-Pr | C(=O)OEt | Me | Et | O |
| D-2685 | H | CH₂CH₂ | Me | *c*-Pr | C(=O)OEt | Me | Et | O |
| D-2686 | Me | CH₂CH₂ | H | *c*-Pr | C(=O)OEt | Me | Et | O |
| D-2687 | H | single bond | Me | *c*-Pr | C(=O)OEt | Cl | CH₂CF₃ | O |
| D-2688 | Me | single bond | H | *c*-Pr | C(=O)OEt | Cl | CH₂CF₃ | O |
| D-2689 | Me(R) | single bond | H | *c*-Pr | C(=O)OEt | Cl | CH₂CF₃ | O |
| D-2690 | Me(S) | single bond | H | *c*-Pr | C(=O)OEt | Cl | CH₂CF₃ | O |
| D-2691 | H | CH₂ | Me | *c*-Pr | C(=O)OEt | Cl | CH₂CF₃ | O |
| D-2692 | Me | CH₂ | H | *c*-Pr | C(=O)OEt | Cl | CH₂CF₃ | O |
| D-2693 | H | CH(Me) | H | *c*-Pr | C(=O)OEt | Cl | CH₂CF₃ | O |
| D-2694 | H | CH₂CH₂ | Me | *c*-Pr | C(=O)OEt | Cl | CH₂CF₃ | O |
| D-2695 | Me | CH₂CH₂ | H | *c*-Pr | C(=O)OEt | Cl | CH₂CF₃ | O |
| D-2696 | H | single bond | Me | *c*-Pr | C(=O)OEt | Me | CH₂CF₃ | O |
| D-2697 | Me | single bond | H | *c*-Pr | C(=O)OEt | Me | CH₂CF₃ | O |
| D-2698 | Me(R) | single bond | H | *c*-Pr | C(=O)OEt | Me | CH₂CF₃ | O |
| D-2699 | Me(S) | single bond | H | *c*-Pr | C(=O)OEt | Me | CH₂CF₃ | O |
| D-2700 | H | CH₂ | Me | *c*-Pr | C(=O)OEt | Me | CH₂CF₃ | O |
| D-2701 | Me | CH₂ | H | *c*-Pr | C(=O)OEt | Me | CH₂CF₃ | O |
| D-2702 | H | CH(Me) | H | *c*-Pr | C(=O)OEt | Me | CH₂CF₃ | O |
| D-2703 | H | CH₂CH₂ | Me | *c*-Pr | C(=O)OEt | Me | CH₂CF₃ | O |
| D-2704 | Me | CH₂CH₂ | H | *c*-Pr | C(=O)OEt | Me | CH₂CF₃ | O |
| D-2705 | H | single bond | Me | *c*-Pr | C(=O)OEt | Cl | CH₂OMe | O |
| D-2706 | Me | single bond | H | *c*-Pr | C(=O)OEt | Cl | CH₂OMe | O |
| D-2707 | Me(R) | single bond | H | *c*-Pr | C(=O)OEt | Cl | CH₂OMe | O |
| D-2708 | Me(S) | single bond | H | *c*-Pr | C(=O)OEt | Cl | CH₂OMe | O |
| D-2709 | H | CH₂ | Me | *c*-Pr | C(=O)OEt | Cl | CH₂OMe | O |
| D-2710 | Me | CH₂ | H | *c*-Pr | C(=O)OEt | Cl | CH₂OMe | O |
| D-2711 | H | CH(Me) | H | *c*-Pr | C(=O)OEt | Cl | CH₂OMe | O |
| D-2712 | H | CH₂CH₂ | Me | *c*-Pr | C(=O)OEt | Cl | CH₂OMe | O |
| D-2713 | Me | CH₂CH₂ | H | *c*-Pr | C(=O)OEt | Cl | CH₂OMe | O |
| D-2714 | H | single bond | Me | *c*-Pr | C(=O)OEt | Me | CH₂OMe | O |
| D-2715 | Me | single bond | H | *c*-Pr | C(=O)OEt | Me | CH₂OMe | O |
| D-2716 | Me(R) | single bond | H | *c*-Pr | C(=O)OEt | Me | CH₂OMe | O |
| D-2717 | Me(S) | single bond | H | *c*-Pr | C(=O)OEt | Me | CH₂OMe | O |
| D-2718 | H | CH₂ | Me | *c*-Pr | C(=O)OEt | Me | CH₂OMe | O |
| D-2719 | Me | CH₂ | H | *c*-Pr | C(=O)OEt | Me | CH₂OMe | O |
| D-2720 | H | CH(Me) | H | *c*-Pr | C(=O)OEt | Me | CH₂OMe | O |
| D-2721 | H | CH₂CH₂ | Me | *c*-Pr | C(=O)OEt | Me | CH₂OMe | O |
| D-2722 | Me | CH₂CH₂ | H | *c*-Pr | C(=O)OEt | Me | CH₂OMe | O |

**[Table 707]**

| Compound Number | Y | W | Z | R¹ | R² | R³ | R¹⁰ | A |
|---|---|---|---|---|---|---|---|---|
| D-2723 | H | single bond | Me | *c*-Pr | C(=O)OEt | Cl | CH₂CN | O |
| D-2724 | Me | single bond | H | *c*-Pr | C(=O)OEt | Cl | CH₂CN | O |
| D-2725 | Me(R) | single bond | H | *c*-Pr | C(=O)OEt | Cl | CH₂CN | O |
| D-2726 | Me(S) | single bond | H | *c*-Pr | C(=O)OEt | Cl | CH₂CN | O |
| D-2727 | H | CH₂ | Me | *c*-Pr | C(=O)OEt | Cl | CH₂CN | O |
| D-2728 | Me | CH₂ | H | *c*-Pr | C(=O)OEt | Cl | CH₂CN | O |
| D-2729 | H | CH(Me) | H | *c*-Pr | C(=O)OEt | Cl | CH₂CN | O |
| D-2730 | H | CH₂CH₂ | Me | *c*-Pr | C(=O)OEt | Cl | CH₂CN | O |
| D-2731 | Me | CH₂CH₂ | H | *c*-Pr | C(=O)OEt | Cl | CH₂CN | O |
| D-2732 | H | single bond | Me | *c*-Pr | C(=O)OEt | Me | CH₂CN | O |
| D-2733 | Me | single bond | H | *c*-Pr | C(=O)OEt | Me | CH₂CN | O |
| D-2734 | Me(R) | single bond | H | *c*-Pr | C(=O)OEt | Me | CH₂CN | O |
| D-2735 | Me(S) | single bond | H | *c*-Pr | C(=O)OEt | Me | CH₂CN | O |
| D-2736 | H | CH₂ | Me | *c*-Pr | C(=O)OEt | Me | CH₂CN | O |
| D-2737 | Me | CH₂ | H | *c*-Pr | C(=O)OEt | Me | CH₂CN | O |
| D-2738 | H | CH(Me) | H | *c*-Pr | C(=O)OEt | Me | CH₂CN | O |
| D-2739 | H | CH₂CH₂ | Me | *c*-Pr | C(=O)OEt | Me | CH₂CN | O |
| D-2740 | Me | CH₂CH₂ | H | *c*-Pr | C(=O)OEt | Me | CH₂CN | O |
| D-2741 | H | single bond | Me | *c*-Pr | C(=O)OEt | Cl | C(=O)Me | O |
| D-2742 | Me | single bond | H | *c*-Pr | C(=O)OEt | Cl | C(=O)Me | O |
| D-2743 | Me(R) | single bond | H | *c*-Pr | C(=O)OEt | Cl | C(=O)Me | O |
| D-2744 | Me(S) | single bond | H | *c*-Pr | C(=O)OEt | Cl | C(=O)Me | O |
| D-2745 | H | CH₂ | Me | *c*-Pr | C(=O)OEt | Cl | C(=O)Me | O |
| D-2746 | Me | CH₂ | H | *c*-Pr | C(=O)OEt | Cl | C(=O)Me | O |
| D-2747 | H | CH(Me) | H | *c*-Pr | C(=O)OEt | Cl | C(=O)Me | O |
| D-2748 | H | CH₂CH₂ | Me | *c*-Pr | C(=O)OEt | Cl | C(=O)Me | O |
| D-2749 | Me | CH₂CH₂ | H | *c*-Pr | C(=O)OEt | Cl | C(=O)Me | O |
| D-2750 | H | single bond | Me | *c*-Pr | C(=O)OEt | Me | C(=O)Me | O |
| D-2751 | Me | single bond | H | *c*-Pr | C(=O)OEt | Me | C(=O)Me | O |
| D-2752 | Me(R) | single bond | H | *c*-Pr | C(=O)OEt | Me | C(=O)Me | O |
| D-2753 | Me(S) | single bond | H | *c*-Pr | C(=O)OEt | Me | C(=O)Me | O |
| D-2754 | H | CH₂ | Me | *c*-Pr | C(=O)OEt | Me | C(=O)Me | O |
| D-2755 | Me | CH₂ | H | *c*-Pr | C(=O)OEt | Me | C(=O)Me | O |
| D-2756 | H | CH(Me) | H | *c*-Pr | C(=O)OEt | Me | C(=O)Me | O |
| D-2757 | H | CH₂CH₂ | Me | *c*-Pr | C(=O)OEt | Me | C(=O)Me | O |
| D-2758 | Me | CH₂CH₂ | H | *c*-Pr | C(=O)OEt | Me | C(=O)Me | O |
| D-2759 | H | single bond | Me | *c*-Pr | S(=O)₂Me | Cl | Me | O |
| D-2760 | H | single bond | Et | *c*-Pr | S(=O)₂Me | Cl | Me | O |
| D-2761 | Me | single bond | H | *c*-Pr | S(=O)₂Me | Cl | Me | O |
| D-2762 | Me(R) | single bond | H | *c*-Pr | S(=O)₂Me | Cl | Me | O |
| D-2763 | Me(S) | single bond | H | *c*-Pr | S(=O)₂Me | Cl | Me | O |
| D-2764 | H | CH₂ | Me | *c*-Pr | S(=O)₂Me | Cl | Me | O |
| D-2765 | Me | CH₂ | H | *c*-Pr | S(=O)₂Me | Cl | Me | O |
| D-2766 | H | CH(Me) | H | *c*-Pr | S(=O)₂Me | Cl | Me | O |

**[Table 708]**

| Compound Number | Y | W | Z | R¹ | R² | R³ | R¹⁰ | A |
|---|---|---|---|---|---|---|---|---|
| D-2767 | H | CH₂CH₂ | Me | *c*-Pr | S(=O)₂Me | Cl | Me | O |
| D-2768 | Me | CH₂CH₂ | H | *c*-Pr | S(=O)₂Me | Cl | Me | O |
| D-2769 | H | single bond | Me | *c*-Pr | S(=O)₂Me | Me | Me | O |
| D-2770 | Me | single bond | H | *c*-Pr | S(=O)₂Me | Me | Me | O |
| D-2771 | Me(R) | single bond | H | *c*-Pr | S(=O)₂Me | Me | Me | O |
| D-2772 | Me(S) | single bond | H | *c*-Pr | S(=O)₂Me | Me | Me | O |
| D-2773 | H | CH₂ | Me | *c*-Pr | S(=O)₂Me | Me | Me | O |
| D-2774 | Me | CH₂ | H | *c*-Pr | S(=O)₂Me | Me | Me | O |
| D-2775 | H | CH(Me) | H | *c*-Pr | S(=O)₂Me | Me | Me | O |
| D-2776 | H | CH₂CH₂ | Me | *c*-Pr | S(=O)₂Me | Me | Me | O |
| D-2777 | Me | CH₂CH₂ | H | *c*-Pr | S(=O)₂Me | Me | Me | O |
| D-2778 | H | single bond | Me | *c*-Pr | S(=O)₂Me | Cl | Et | O |
| D-2779 | Me | single bond | H | *c*-Pr | S(=O)₂Me | Cl | Et | O |
| D-2780 | Me(R) | single bond | H | *c*-Pr | S(=O)₂Me | Cl | Et | O |
| D-2781 | Me(S) | single bond | H | *c*-Pr | S(=O)₂Me | Cl | Et | O |
| D-2782 | H | CH₂ | Me | *c*-Pr | S(=O)₂Me | Cl | Et | O |
| D-2783 | Me | CH₂ | H | *c*-Pr | S(=O)₂Me | Cl | Et | O |
| D-2784 | H | CH(Me) | H | *c*-Pr | S(=O)₂Me | Cl | Et | O |
| D-2785 | H | CH₂CH₂ | Me | *c*-Pr | S(=O)₂Me | Cl | Et | O |
| D-2786 | Me | CH₂CH₂ | H | *c*-Pr | S(=O)₂Me | Cl | Et | O |
| D-2787 | H | single bond | Me | *c*-Pr | S(=O)₂Me | Me | Et | O |
| D-2788 | Me | single bond | H | *c*-Pr | S(=O)₂Me | Me | Et | O |
| D-2789 | Me(R) | single bond | H | *c*-Pr | S(=O)₂Me | Me | Et | O |
| D-2790 | Me(S) | single bond | H | *c*-Pr | S(=O)₂Me | Me | Et | O |
| D-2791 | H | CH₂ | Me | *c*-Pr | S(=O)₂Me | Me | Et | O |
| D-2792 | Me | CH₂ | H | *c*-Pr | S(=O)₂Me | Me | Et | O |
| D-2793 | H | CH(Me) | H | *c*-Pr | S(=O)₂Me | Me | Et | O |
| D-2794 | H | CH₂CH₂ | Me | *c*-Pr | S(=O)₂Me | Me | Et | O |
| D-2795 | Me | CH₂CH₂ | H | *c*-Pr | S(=O)₂Me | Me | Et | O |
| D-2796 | H | single bond | Me | *c*-Pr | S(=O)₂Me | Cl | CH₂CF₃ | O |
| D-2797 | Me | single bond | H | *c*-Pr | S(=O)₂Me | Cl | CH₂CF₃ | O |
| D-2798 | Me(R) | single bond | H | *c*-Pr | S(=O)₂Me | Cl | CH₂CF₃ | O |
| D-2799 | Me(S) | single bond | H | *c*-Pr | S(=O)₂Me | Cl | CH₂CF₃ | O |
| D-2800 | H | CH₂ | Me | *c*-Pr | S(=O)₂Me | Cl | CH₂CF₃ | O |
| D-2801 | Me | CH₂ | H | *c*-Pr | S(=O)₂Me | Cl | CH₂CF₃ | O |
| D-2802 | H | CH(Me) | H | *c*-Pr | S(=O)₂Me | Cl | CH₂CF₃ | O |
| D-2803 | H | CH₂CH₂ | Me | *c*-Pr | S(=O)₂Me | Cl | CH₂CF₃ | O |
| D-2804 | Me | CH₂CH₂ | H | *c*-Pr | S(=O)₂Me | Cl | CH₂CF₃ | O |
| D-2805 | H | single bond | Me | *c*-Pr | S(=O)₂Me | Me | CH₂CF₃ | O |
| D-2806 | Me | single bond | H | *c*-Pr | S(=O)₂Me | Me | CH₂CF₃ | O |
| D-2807 | Me(R) | single bond | H | *c*-Pr | S(=O)₂Me | Me | CH₂CF₃ | O |
| D-2808 | Me(S) | single bond | H | *c*-Pr | S(=O)₂Me | Me | CH₂CF₃ | O |
| D-2809 | H | CH₂ | Me | *c*-Pr | S(=O)₂Me | Me | CH₂CF₃ | O |
| D-2810 | Me | CH₂ | H | *c*-Pr | S(=O)₂Me | Me | CH₂CF₃ | O |

**[Table 709]**

| Compound Number | Y | W | Z | R¹ | R² | R³ | R¹⁰ | A |
|---|---|---|---|---|---|---|---|---|
| D-2811 | H | CH(Me) | H | *c*-Pr | S(=O)₂Me | Me | CH₂CF₃ | O |
| D-2812 | H | CH₂CH₂ | Me | *c*-Pr | S(=O)₂Me | Me | CH₂CF₃ | O |
| D-2813 | Me | CH₂CH₂ | H | *c*-Pr | S(=O)₂Me | Me | CH₂CF₃ | O |
| D-2814 | H | single bond | Me | *c*-Pr | S(=O)₂Me | Cl | CH₂OMe | O |
| D-2815 | Me | single bond | H | *c*-Pr | S(=O)₂Me | Cl | CH₂OMe | O |
| D-2816 | Me(R) | single bond | H | *c*-Pr | S(=O)₂Me | Cl | CH₂OMe | O |
| D-2817 | Me(S) | single bond | H | *c*-Pr | S(=O)₂Me | Cl | CH₂OMe | O |
| D-2818 | H | CH₂ | Me | *c*-Pr | S(=O)₂Me | Cl | CH₂OMe | O |
| D-2819 | Me | CH₂ | H | *c*-Pr | S(=O)₂Me | Cl | CH₂OMe | O |
| D-2820 | H | CH(Me) | H | *c*-Pr | S(=O)₂Me | Cl | CH₂OMe | O |
| D-2821 | H | CH₂CH₂ | Me | *c*-Pr | S(=O)₂Me | Cl | CH₂OMe | O |
| D-2822 | Me | CH₂CH₂ | H | *c*-Pr | S(=O)₂Me | Cl | CH₂OMe | O |
| D-2823 | H | single bond | Me | *c*-Pr | S(=O)₂Me | Me | CH₂OMe | O |
| D-2824 | Me | single bond | H | *c*-Pr | S(=O)₂Me | Me | CH₂OMe | O |
| D-2825 | Me(R) | single bond | H | *c*-Pr | S(=O)₂Me | Me | CH₂OMe | O |
| D-2826 | Me(S) | single bond | H | *c*-Pr | S(=O)₂Me | Me | CH₂OMe | O |
| D-2827 | H | CH₂ | Me | *c*-Pr | S(=O)₂Me | Me | CH₂OMe | O |
| D-2828 | Me | CH₂ | H | *c*-Pr | S(=O)₂Me | Me | CH₂OMe | O |
| D-2829 | H | CH(Me) | H | *c*-Pr | S(=O)₂Me | Me | CH₂OMe | O |
| D-2830 | H | CH₂CH₂ | Me | *c*-Pr | S(=O)₂Me | Me | CH₂OMe | O |
| D-2831 | Me | CH₂CH₂ | H | *c*-Pr | S(=O)₂Me | Me | CH₂OMe | O |
| D-2832 | H | single bond | Me | *c*-Pr | S(=O)₂Me | Cl | CH₂CN | O |
| D-2833 | Me | single bond | H | *c*-Pr | S(=O)₂Me | Cl | CH₂CN | O |
| D-2834 | Me(R) | single bond | H | *c*-Pr | S(=O)₂Me | Cl | CH₂CN | O |
| D-2835 | Me(S) | single bond | H | *c*-Pr | S(=O)₂Me | Cl | CH₂CN | O |
| D-2836 | H | CH₂ | Me | *c*-Pr | S(=O)₂Me | Cl | CH₂CN | O |
| D-2837 | Me | CH₂ | H | *c*-Pr | S(=O)₂Me | Cl | CH₂CN | O |
| D-2838 | H | CH(Me) | H | *c*-Pr | S(=O)₂Me | Cl | CH₂CN | O |
| D-2839 | H | CH₂CH₂ | Me | *c*-Pr | S(=O)₂Me | Cl | CH₂CN | O |
| D-2840 | Me | CH₂CH₂ | H | *c*-Pr | S(=O)₂Me | Cl | CH₂CN | O |
| D-2841 | H | single bond | Me | *c*-Pr | S(=O)₂Me | Me | CH₂CN | O |
| D-2842 | Me | single bond | H | *c*-Pr | S(=O)₂Me | Me | CH₂CN | O |
| D-2843 | Me(R) | single bond | H | *c*-Pr | S(=O)₂Me | Me | CH₂CN | O |
| D-2844 | Me(S) | single bond | H | *c*-Pr | S(=O)₂Me | Me | CH₂CN | O |
| D-2845 | H | CH₂ | Me | *c*-Pr | S(=O)₂Me | Me | CH₂CN | O |
| D-2846 | Me | CH₂ | H | *c*-Pr | S(=O)₂Me | Me | CH₂CN | O |
| D-2847 | H | CH(Me) | H | *c*-Pr | S(=O)₂Me | Me | CH₂CN | O |
| D-2848 | H | CH₂CH₂ | Me | *c*-Pr | S(=O)₂Me | Me | CH₂CN | O |
| D-2849 | Me | CH₂CH₂ | H | *c*-Pr | S(=O)₂Me | Me | CH₂CN | O |
| D-2850 | H | single bond | Me | *c*-Pr | S(=O)₂Me | Cl | C(=O)Me | O |
| D-2851 | Me | single bond | H | *c*-Pr | S(=O)₂Me | Cl | C(=O)Me | O |
| D-2852 | Me(R) | single bond | H | *c*-Pr | S(=O)₂Me | Cl | C(=O)Me | O |
| D-2853 | Me(S) | single bond | H | *c*-Pr | S(=O)₂Me | Cl | C(=O)Me | O |
| D-2854 | H | CH₂ | Me | *c*-Pr | S(=O)₂Me | Cl | C(=O)Me | O |

**[Table 710]**

| Compound Number | Y | W | Z | R¹ | R² | R³ | R¹⁰ | A |
|---|---|---|---|---|---|---|---|---|
| D-2855 | Me | CH₂ | H | *c*-Pr | S(=O)₂Me | Cl | C(=O)Me | O |
| D-2856 | H | CH(Me) | H | *c*-Pr | S(=O)₂Me | Cl | C(=O)Me | O |
| D-2857 | H | CH₂CH₂ | Me | *c*-Pr | S(=O)₂Me | Cl | C(=O)Me | O |
| D-2858 | Me | CH₂CH₂ | H | *c*-Pr | S(=O)₂Me | Cl | C(=O)Me | O |
| D-2859 | H | single bond | Me | *c*-Pr | S(=O)₂Me | Me | C(=O)Me | O |
| D-2860 | Me | single bond | H | *c*-Pr | S(=O)₂Me | Me | C(=O)Me | O |
| D-2861 | Me(R) | single bond | H | *c*-Pr | S(=O)₂Me | Me | C(=O)Me | O |
| D-2862 | Me(S) | single bond | H | *c*-Pr | S(=O)₂Me | Me | C(=O)Me | O |
| D-2863 | H | CH₂ | Me | *c*-Pr | S(=O)₂Me | Me | C(=O)Me | O |
| D-2864 | Me | CH₂ | H | *c*-Pr | S(=O)₂Me | Me | C(=O)Me | O |
| D-2865 | H | CH(Me) | H | *c*-Pr | S(=O)₂Me | Me | C(=O)Me | O |
| D-2866 | H | CH₂CH₂ | Me | *c*-Pr | S(=O)₂Me | Me | C(=O)Me | O |
| D-2867 | Me | CH₂CH₂ | H | *c*-Pr | S(=O)₂Me | Me | C(=O)Me | O |
| D-2868 | H | single bond | Me | *c*-Pr | S(=O)₂CF₃ | Cl | Me | O |
| D-2869 | H | single bond | Et | *c*-Pr | S(=O)₂CF₃ | Cl | Me | O |
| D-2870 | Me | single bond | H | *c*-Pr | S(=O)₂CF₃ | Cl | Me | O |
| D-2871 | Me(R) | single bond | H | *c*-Pr | S(=O)₂CF₃ | Cl | Me | O |
| D-2872 | Me(S) | single bond | H | *c*-Pr | S(=O)₂CF₃ | Cl | Me | O |
| D-2873 | H | CH₂ | Me | *c*-Pr | S(=O)₂CF₃ | Cl | Me | O |
| D-2874 | Me | CH₂ | H | *c*-Pr | S(=O)₂CF₃ | Cl | Me | O |
| D-2875 | H | CH(Me) | H | *c*-Pr | S(=O)₂CF₃ | Cl | Me | O |
| D-2876 | H | CH₂CH₂ | Me | *c*-Pr | S(=O)₂CF₃ | Cl | Me | O |
| D-2877 | Me | CH₂CH₂ | H | *c*-Pr | S(=O)₂CF₃ | Cl | Me | O |
| D-2878 | H | single bond | Me | *c*-Pr | S(=O)₂CF₃ | Me | Me | O |
| D-2879 | Me | single bond | H | *c*-Pr | S(=O)₂CF₃ | Me | Me | O |
| D-2880 | Me(R) | single bond | H | *c*-Pr | S(=O)₂CF₃ | Me | Me | O |
| D-2881 | Me(S) | single bond | H | *c*-Pr | S(=O)₂CF₃ | Me | Me | O |
| D-2882 | H | CH₂ | Me | *c*-Pr | S(=O)₂CF₃ | Me | Me | O |
| D-2883 | Me | CH₂ | H | *c*-Pr | S(=O)₂CF₃ | Me | Me | O |
| D-2884 | H | CH(Me) | H | *c*-Pr | S(=O)₂CF₃ | Me | Me | O |
| D-2885 | H | CH₂CH₂ | Me | *c*-Pr | S(=O)₂CF₃ | Me | Me | O |
| D-2886 | Me | CH₂CH₂ | H | *c*-Pr | S(=O)₂CF₃ | Me | Me | O |
| D-2887 | H | single bond | Me | *c*-Pr | S(=O)₂CF₃ | Cl | Et | O |
| D-2888 | Me | single bond | H | *c*-Pr | S(=O)₂CF₃ | Cl | Et | O |
| D-2889 | Me(R) | single bond | H | *c*-Pr | S(=O)₂CF₃ | Cl | Et | O |
| D-2890 | Me(S) | single bond | H | *c*-Pr | S(=O)₂CF₃ | Cl | Et | O |
| D-2891 | H | CH₂ | Me | *c*-Pr | S(=O)₂CF₃ | Cl | Et | O |
| D-2892 | Me | CH₂ | H | *c*-Pr | S(=O)₂CF₃ | Cl | Et | O |
| D-2893 | H | CH(Me) | H | *c*-Pr | S(=O)₂CF₃ | Cl | Et | O |
| D-2894 | H | CH₂CH₂ | Me | *c*-Pr | S(=O)₂CF₃ | Cl | Et | O |
| D-2895 | Me | CH₂CH₂ | H | *c*-Pr | S(=O)₂CF₃ | Cl | Et | O |
| D-2896 | H | single bond | Me | *c*-Pr | S(=O)₂CF₃ | Me | Et | O |
| D-2897 | Me | single bond | H | *c*-Pr | S(=O)₂CF₃ | Me | Et | O |
| D-2898 | Me(R) | single bond | H | *c*-Pr | S(=O)₂CF₃ | Me | Et | O |

**[Table 711]**

| Compound Number | Y | W | Z | R¹ | R² | R³ | R¹⁰ | A |
|---|---|---|---|---|---|---|---|---|
| D-2899 | Me(S) | single bond | H | *c*-Pr | S(=O)₂CF₃ | Me | Et | O |
| D-2900 | H | CH₂ | Me | *c*-Pr | S(=O)₂CF₃ | Me | Et | O |
| D-2901 | Me | CH₂ | H | *c*-Pr | S(=O)₂CF₃ | Me | Et | O |
| D-2902 | H | CH(Me) | H | *c*-Pr | S(=O)₂CF₃ | Me | Et | O |
| D-2903 | H | CH₂CH₂ | Me | *c*-Pr | S(=O)₂CF₃ | Me | Et | O |
| D-2904 | Me | CH₂CH₂ | H | *c*-Pr | S(=O)₂CF₃ | Me | Et | O |
| D-2905 | H | single bond | Me | *c*-Pr | S(=O)₂CF₃ | Cl | CH₂CF₃ | O |
| D-2906 | Me | single bond | H | *c*-Pr | S(=O)₂CF₃ | Cl | CH₂CF₃ | O |
| D-2907 | Me(R) | single bond | H | *c*-Pr | S(=O)₂CF₃ | Cl | CH₂CF₃ | O |
| D-2908 | Me(S) | single bond | H | *c*-Pr | S(=O)₂CF₃ | Cl | CH₂CF₃ | O |
| D-2909 | H | CH₂ | Me | *c*-Pr | S(=O)₂CF₃ | Cl | CH₂CF₃ | O |
| D-2910 | Me | CH₂ | H | *c*-Pr | S(=O)₂CF₃ | Cl | CH₂CF₃ | O |
| D-2911 | H | CH(Me) | H | *c*-Pr | S(=O)₂CF₃ | Cl | CH₂CF₃ | O |
| D-2912 | H | CH₂CH₂ | Me | *c*-Pr | S(=O)₂CF₃ | Cl | CH₂CF₃ | O |
| D-2913 | Me | CH₂CH₂ | H | *c*-Pr | S(=O)₂CF₃ | Cl | CH₂CF₃ | O |
| D-2914 | H | single bond | Me | *c*-Pr | S(=O)₂CF₃ | Me | CH₂CF₃ | O |
| D-2915 | Me | single bond | H | *c*-Pr | S(=O)₂CF₃ | Me | CH₂CF₃ | O |
| D-2916 | Me(R) | single bond | H | *c*-Pr | S(=O)₂CF₃ | Me | CH₂CF₃ | O |
| D-2917 | Me(S) | single bond | H | *c*-Pr | S(=O)₂CF₃ | Me | CH₂CF₃ | O |
| D-2918 | H | CH₂ | Me | *c*-Pr | S(=O)₂CF₃ | Me | CH₂CF₃ | O |
| D-2919 | Me | CH₂ | H | *c*-Pr | S(=O)₂CF₃ | Me | CH₂CF₃ | O |
| D-2920 | H | CH(Me) | H | *c*-Pr | S(=O)₂CF₃ | Me | CH₂CF₃ | O |
| D-2921 | H | CH₂CH₂ | Me | *c*-Pr | S(=O)₂CF₃ | Me | CH₂CF₃ | O |
| D-2922 | Me | CH₂CH₂ | H | *c*-Pr | S(=O)₂CF₃ | Me | CH₂CF₃ | O |
| D-2923 | H | single bond | Me | *c*-Pr | S(=O)₂CF₃ | Cl | CH₂OMe | O |
| D-2924 | Me | single bond | H | *c*-Pr | S(=O)₂CF₃ | Cl | CH₂OMe | O |
| D-2925 | Me(R) | single bond | H | *c*-Pr | S(=O)₂CF₃ | Cl | CH₂OMe | O |
| D-2926 | Me(S) | single bond | H | *c*-Pr | S(=O)₂CF₃ | Cl | CH₂OMe | O |
| D-2927 | H | CH₂ | Me | *c*-Pr | S(=O)₂CF₃ | Cl | CH₂OMe | O |
| D-2928 | Me | CH₂ | H | *c*-Pr | S(=O)₂CF₃ | Cl | CH₂OMe | O |
| D-2929 | H | CH(Me) | H | *c*-Pr | S(=O)₂CF₃ | Cl | CH₂OMe | O |
| D-2930 | H | CH₂CH₂ | Me | *c*-Pr | S(=O)₂CF₃ | Cl | CH₂OMe | O |
| D-2931 | Me | CH₂CH₂ | H | *c*-Pr | S(=O)₂CF₃ | Cl | CH₂OMe | O |
| D-2932 | H | single bond | Me | *c*-Pr | S(=O)₂CF₃ | Me | CH₂OMe | O |
| D-2933 | Me | single bond | H | *c*-Pr | S(=O)₂CF₃ | Me | CH₂OMe | O |
| D-2934 | Me(R) | single bond | H | *c*-Pr | S(=O)₂CF₃ | Me | CH₂OMe | O |
| D-2935 | Me(S) | single bond | H | *c*-Pr | S(=O)₂CF₃ | Me | CH₂OMe | O |
| D-2936 | H | CH₂ | Me | c-Pr | S(=O)₂CF₃ | Me | CH₂OMe | O |
| D-2937 | Me | CH₂ | H | *c*-Pr | S(=O)₂CF₃ | Me | CH₂OMe | O |
| D-2938 | H | CH(Me) | H | *c*-Pr | S(=O)₂CF₃ | Me | CH₂OMe | O |
| D-2939 | H | CH₂CH₂ | Me | *c*-Pr | S(=O)₂CF₃ | Me | CH₂OMe | O |
| D-2940 | Me | CH₂CH₂ | H | *c*-Pr | S(=O)₂CF₃ | Me | CH₂OMe | O |
| D-2941 | H | single bond | Me | *c*-Pr | S(=O)₂CF₃ | Cl | CH₂CN | O |
| D-2942 | Me | single bond | H | *c*-Pr | S(=O)₂CF₃ | Cl | CH₂CN | O |

**[Table 712]**

| Compound Number | Y | W | Z | R¹ | R² | R³ | R¹⁰ | A |
|---|---|---|---|---|---|---|---|---|
| D-2943 | Me(R) | single bond | H | *c*-Pr | S(=O)₂CF₃ | Cl | CH₂CN | O |
| D-2944 | Me(S) | single bond | H | *c*-Pr | S(=O)₂CF₃ | Cl | CH₂CN | O |
| D-2945 | H | CH₂ | Me | *c*-Pr | S(=O)₂CF₃ | Cl | CH₂CN | O |
| D-2946 | Me | CH₂ | H | *c*-Pr | S(=O)₂CF₃ | Cl | CH₂CN | O |
| D-2947 | H | CH(Me) | H | *c*-Pr | S(=O)₂CF₃ | Cl | CH₂CN | O |
| D-2948 | H | CH₂CH₂ | Me | *c*-Pr | S(=O)₂CF₃ | Cl | CH₂CN | O |
| D-2949 | Me | CH₂CH₂ | H | *c*-Pr | S(=O)₂CF₃ | Cl | CH₂CN | O |
| D-2950 | H | single bond | Me | *c*-Pr | S(=O)₂CF₃ | Me | CH₂CN | O |
| D-2951 | Me | single bond | H | *c*-Pr | S(=O)₂CF₃ | Me | CH₂CN | O |
| D-2952 | Me(R) | single bond | H | *c*-Pr | S(=O)₂CF₃ | Me | CH₂CN | O |
| D-2953 | Me(S) | single bond | H | *c*-Pr | S(=O)₂CF₃ | Me | CH₂CN | O |
| D-2954 | H | CH₂ | Me | *c*-Pr | S(=O)₂CF₃ | Me | CH₂CN | O |
| D-2955 | Me | CH₂ | H | *c*-Pr | S(=O)₂CF₃ | Me | CH₂CN | O |
| D-2956 | H | CH(Me) | H | *c*-Pr | S(=O)₂CF₃ | Me | CH₂CN | O |
| D-2957 | H | CH₂CH₂ | Me | *c*-Pr | S(=O)₂CF₃ | Me | CH₂CN | O |
| D-2958 | Me | CH₂CH₂ | H | *c*-Pr | S(=O)₂CF₃ | Me | CH₂CN | O |
| D-2959 | H | single bond | Me | *c*-Pr | S(=O)₂CF₃ | Cl | C(=O)Me | O |
| D-2960 | Me | single bond | H | *c*-Pr | S(=O)₂CF₃ | Cl | C(=O)Me | O |
| D-2961 | Me(R) | single bond | H | *c*-Pr | S(=O)₂CF₃ | Cl | C(=O)Me | O |
| D-2962 | Me(S) | single bond | H | *c*-Pr | S(=O)₂CF₃ | Cl | C(=O)Me | O |
| D-2963 | H | CH₂ | Me | *c*-Pr | S(=O)₂CF₃ | Cl | C(=O)Me | O |
| D-2964 | Me | CH₂ | H | *c*-Pr | S(=O)₂CF₃ | Cl | C(=O)Me | O |
| D-2965 | H | CH(Me) | H | *c*-Pr | S(=O)₂CF₃ | Cl | C(=O)Me | O |
| D-2966 | H | CH₂CH₂ | Me | *c*-Pr | S(=O)₂CF₃ | Cl | C(=O)Me | O |
| D-2967 | Me | CH₂CH₂ | H | *c*-Pr | S(=O)₂CF₃ | Cl | C(=O)Me | O |
| D-2968 | H | single bond | Me | *c*-Pr | S(=O)₂CF₃ | Me | C(=O)Me | O |
| D-2969 | Me | single bond | H | *c*-Pr | S(=O)₂CF₃ | Me | C(=O)Me | O |
| D-2970 | Me(R) | single bond | H | *c*-Pr | S(=O)₂CF₃ | Me | C(=O)Me | O |
| D-2971 | Me(S) | single bond | H | *c*-Pr | S(=O)₂CF₃ | Me | C(=O)Me | O |
| D-2972 | H | CH₂ | Me | *c*-Pr | S(=O)₂CF₃ | Me | C(=O)Me | O |
| D-2973 | Me | CH₂ | H | *c*-Pr | S(=O)₂CF₃ | Me | C(=O)Me | O |
| D-2974 | H | CH(Me) | H | *c*-Pr | S(=O)₂CF₃ | Me | C(=O)Me | O |
| D-2975 | H | CH₂CH₂ | Me | *c*-Pr | S(=O)₂CF₃ | Me | C(=O)Me | O |
| D-2976 | Me | CH₂CH₂ | H | *c*-Pr | S(=O)₂CF₃ | Me | C(=O)Me | O |
| D-2977 | H | single bond | Me | *c*-Pr | H | Cl | H | O |
| D-2978 | Me | single bond | H | *c*-Pr | H | Cl | H | O |
| D-2979 | Me(R) | single bond | H | *c*-Pr | H | Cl | H | O |
| D-2980 | Me(S) | single bond | H | *c*-Pr | H | Cl | H | O |
| D-2981 | Me | single bond | H | *c*-Pr | H | Cl | H | S |
| D-2982 | Me(R) | single bond | H | *c*-Pr | H | Cl | H | S |
| D-2983 | Me(S) | single bond | H | *c*-Pr | H | Cl | H | S |
| D-2984 | H | CH₂ | Me | *c*-Pr | H | Cl | H | O |
| D-2985 | Me | CH₂ | H | *c*-Pr | H | Cl | H | O |
| D-2986 | H | CH(Me) | H | *c*-Pr | H | Cl | H | O |

**[Table 713]**

| Compound Number | Y | W | Z | R¹ | R² | R³ | R¹⁰ | A |
|---|---|---|---|---|---|---|---|---|
| D-2987 | H | CH₂CH₂ | Me | *c*-Pr | H | Cl | H | O |
| D-2988 | Me | CH₂CH₂ | H | *c*-Pr | H | Cl | H | O |
| D-2989 | H | single bond | Me | *c*-Pr | H | Me | H | O |
| D-2990 | Me | single bond | H | *c*-Pr | H | Me | H | O |
| D-2991 | Me(R) | single bond | H | *c*-Pr | H | Me | H | O |
| D-2992 | Me(S) | single bond | H | *c*-Pr | H | Me | H | O |
| D-2993 | Me | single bond | H | *c*-Pr | H | Me | H | S |
| D-2994 | Me(R) | single bond | H | *c*-Pr | H | Me | H | S |
| D-2995 | Me(S) | single bond | H | *c*-Pr | H | Me | H | S |
| D-2996 | H | CH₂ | Me | *c*-Pr | H | Me | H | O |
| D-2997 | Me | CH₂ | H | *c*-Pr | H | Me | H | O |
| D-2998 | H | CH(Me) | H | *c*-Pr | H | Me | H | O |
| D-2999 | H | CH₂CH₂ | Me | *c*-Pr | H | Me | H | O |
| D-3000 | Me | CH₂CH₂ | H | *c*-Pr | H | Me | H | O |
| D-3001 | H | single bond | Me | *c*-Pr | H | Cl | Me | O |
| D-3002 | Me | single bond | H | *c*-Pr | H | Cl | Me | O |
| D-3003 | Me(R) | single bond | H | *c*-Pr | H | Cl | Me | O |
| D-3004 | Me(S) | single bond | H | *c*-Pr | H | Cl | Me | O |
| D-3005 | Me | single bond | H | *c*-Pr | H | Cl | Me | S |
| D-3006 | Me(R) | single bond | H | *c*-Pr | H | Cl | Me | S |
| D-3007 | Me(S) | single bond | H | *c*-Pr | H | Cl | Me | S |
| D-3008 | H | CH₂ | Me | *c*-Pr | H | Cl | Me | O |
| D-3009 | Me | CH₂ | H | *c*-Pr | H | Cl | Me | O |
| D-3010 | H | CH(Me) | H | *c*-Pr | H | Cl | Me | O |
| D-3011 | H | CH₂CH₂ | Me | *c*-Pr | H | Cl | Me | O |
| D-3012 | Me | CH₂CH₂ | H | *c*-Pr | H | Cl | Me | O |
| D-3013 | H | single bond | Me | *c*-Pr | H | Me | Me | O |
| D-3014 | Me | single bond | H | *c*-Pr | H | Me | Me | O |
| D-3015 | Me(R) | single bond | H | *c*-Pr | H | Me | Me | O |
| D-3016 | Me(S) | single bond | H | *c*-Pr | H | Me | Me | O |
| D-3017 | Me | single bond | H | *c*-Pr | H | Me | Me | S |
| D-3018 | Me(R) | single bond | H | *c*-Pr | H | Me | Me | S |
| D-3019 | Me(S) | single bond | H | *c*-Pr | H | Me | Me | S |
| D-3020 | H | CH₂ | Me | *c*-Pr | H | Me | Me | O |
| D-3021 | Me | CH₂ | H | *c*-Pr | H | Me | Me | O |
| D-3022 | H | CH(Me) | H | *c*-Pr | H | Me | Me | O |
| D-3023 | H | CH₂CH₂ | Me | *c*-Pr | H | Me | Me | O |
| D-3024 | Me | CH₂CH₂ | H | *c*-Pr | H | Me | Me | O |
| D-3025 | H | single bond | Me | *c*-Pr | H | Cl | Et | O |
| D-3026 | Me | single bond | H | *c*-Pr | H | Cl | Et | O |
| D-3027 | Me(R) | single bond | H | *c*-Pr | H | Cl | Et | O |
| D-3028 | Me(S) | single bond | H | *c*-Pr | H | Cl | Et | O |
| D-3029 | Me | single bond | H | *c*-Pr | H | Cl | Et | S |
| D-3030 | Me(R) | single bond | H | *c*-Pr | H | Cl | Et | S |

**[Table 714]**

| Compound Number | Y | W | Z | R¹ | R² | R³ | R¹⁰ | A |
|---|---|---|---|---|---|---|---|---|
| D-3031 | Me(S) | single bond | H | *c*-Pr | H | Cl | Et | S |
| D-3032 | H | CH₂ | Me | *c*-Pr | H | Cl | Et | O |
| D-3033 | Me | CH₂ | H | *c*-Pr | H | Cl | Et | O |
| D-3034 | H | CH(Me) | H | *c*-Pr | H | Cl | Et | O |
| D-3035 | H | CH₂CH₂ | Me | *c*-Pr | H | Cl | Et | O |
| D-3036 | Me | CH₂CH₂ | H | *c*-Pr | H | Cl | Et | O |
| D-3037 | H | single bond | Me | *c*-Pr | H | Me | Et | O |
| D-3038 | Me | single bond | H | *c*-Pr | H | Me | Et | O |
| D-3039 | Me(R) | single bond | H | *c*-Pr | H | Me | Et | O |
| D-3040 | Me(S) | single bond | H | *c*-Pr | H | Me | Et | O |
| D-3041 | Me | single bond | H | *c*-Pr | H | Me | Et | S |
| D-3042 | Me(R) | single bond | H | *c*-Pr | H | Me | Et | S |
| D-3043 | Me(S) | single bond | H | *c*-Pr | H | Me | Et | S |
| D-3044 | H | CH₂ | Me | *c*-Pr | H | Me | Et | O |
| D-3045 | Me | CH₂ | H | *c*-Pr | H | Me | Et | O |
| D-3046 | H | CH(Me) | H | *c*-Pr | H | Me | Et | O |
| D-3047 | H | CH₂CH₂ | Me | *c*-Pr | H | Me | Et | O |
| D-3048 | Me | CH₂CH₂ | H | *c*-Pr | H | Me | Et | O |
| D-3049 | H | single bond | Me | *c*-Pr | H | Cl | CH₂CF₃ | O |
| D-3050 | Me | single bond | H | *c*-Pr | H | Cl | CH₂CF₃ | O |
| D-3051 | Me(R) | single bond | H | *c*-Pr | H | Cl | CH₂CF₃ | O |
| D-3052 | Me(S) | single bond | H | *c*-Pr | H | Cl | CH₂CF₃ | O |
| D-3053 | Me | single bond | H | *c*-Pr | H | Cl | CH₂CF₃ | S |
| D-3054 | Me(R) | single bond | H | *c*-Pr | H | Cl | CH₂CF₃ | S |
| D-3055 | Me(S) | single bond | H | *c*-Pr | H | Cl | CH₂CF₃ | S |
| D-3056 | H | CH₂ | Me | *c*-Pr | H | Cl | CH₂CF₃ | O |
| D-3057 | Me | CH₂ | H | *c*-Pr | H | Cl | CH₂CF₃ | O |
| D-3058 | H | CH(Me) | H | *c*-Pr | H | Cl | CH₂CF₃ | O |
| D-3059 | H | CH₂CH₂ | Me | *c*-Pr | H | Cl | CH₂CF₃ | O |
| D-3060 | Me | CH₂CH₂ | H | *c*-Pr | H | Cl | CH₂CF₃ | O |
| D-3061 | H | single bond | Me | *c*-Pr | H | Me | CH₂CF₃ | O |
| D-3062 | Me | single bond | H | *c*-Pr | H | Me | CH₂CF₃ | O |
| D-3063 | Me(R) | single bond | H | *c*-Pr | H | Me | CH₂CF₃ | O |
| D-3064 | Me(S) | single bond | H | *c*-Pr | H | Me | CH₂CF₃ | O |
| D-3065 | Me | single bond | H | *c*-Pr | H | Me | CH₂CF₃ | S |
| D-3066 | Me(R) | single bond | H | *c*-Pr | H | Me | CH₂CF₃ | S |
| D-3067 | Me(S) | single bond | H | *c*-Pr | H | Me | CH₂CF₃ | S |
| D-3068 | H | CH₂ | Me | *c*-Pr | H | Me | CH₂CF₃ | O |
| D-3069 | Me | CH₂ | H | *c*-Pr | H | Me | CH₂CF₃ | O |
| D-3070 | H | CH(Me) | H | *c*-Pr | H | Me | CH₂CF₃ | O |
| D-3071 | H | CH₂CH₂ | Me | *c*-Pr | H | Me | CH₂CF₃ | O |
| D-3072 | Me | CH₂CH₂ | H | *c*-Pr | H | Me | CH₂CF₃ | O |
| D-3073 | H | single bond | Me | *c*-Pr | H | Cl | CH₂OMe | O |
| D-3074 | Me | single bond | H | *c*-Pr | H | Cl | CH₂OMe | O |

**[Table 715]**

| Compound Number | Y | W | Z | R¹ | R² | R³ | R¹⁰ | A |
|---|---|---|---|---|---|---|---|---|
| D-3075 | Me(R) | single bond | H | *c*-Pr | H | Cl | CH₂OMe | O |
| D-3076 | Me(S) | single bond | H | *c*-Pr | H | Cl | CH₂OMe | O |
| D-3077 | Me | single bond | H | *c*-Pr | H | Cl | CH₂OMe | S |
| D-3078 | Me(R) | single bond | H | *c*-Pr | H | Cl | CH₂OMe | S |
| D-3079 | Me(S) | single bond | H | *c*-Pr | H | Cl | CH₂OMe | S |
| D-3080 | H | CH₂ | Me | *c*-Pr | H | Cl | CH₂OMe | O |
| D-3081 | Me | CH₂ | H | *c*-Pr | H | Cl | CH₂OMe | O |
| D-3082 | H | CH(Me) | H | *c*-Pr | H | Cl | CH₂OMe | O |
| D-3083 | H | CH₂CH₂ | Me | *c*-Pr | H | Cl | CH₂OMe | O |
| D-3084 | Me | CH₂CH₂ | H | *c*-Pr | H | Cl | CH₂OMe | O |
| D-3085 | H | single bond | Me | *c*-Pr | H | Me | CH₂OMe | O |
| D-3086 | Me | single bond | H | *c*-Pr | H | Me | CH₂OMe | O |
| D-3087 | Me(R) | single bond | H | *c*-Pr | H | Me | CH₂OMe | O |
| D-3088 | Me(S) | single bond | H | *c*-Pr | H | Me | CH₂OMe | O |
| D-3089 | Me | single bond | H | *c*-Pr | H | Me | CH₂OMe | S |
| D-3090 | Me(R) | single bond | H | *c*-Pr | H | Me | CH₂OMe | S |
| D-3091 | Me(S) | single bond | H | *c*-Pr | H | Me | CH₂OMe | S |
| D-3092 | H | CH₂ | Me | *c*-Pr | H | Me | CH₂OMe | O |
| D-3093 | Me | CH₂ | H | *c*-Pr | H | Me | CH₂OMe | O |
| D-3094 | H | CH(Me) | H | *c*-Pr | H | Me | CH₂OMe | O |
| D-3095 | H | CH₂CH₂ | Me | *c*-Pr | H | Me | CH₂OMe | O |
| D-3096 | Me | CH₂CH₂ | H | *c*-Pr | H | Me | CH₂OMe | O |
| D-3097 | H | single bond | Me | *c*-Pr | H | Cl | CH₂CN | O |
| D-3098 | Me | single bond | H | *c*-Pr | H | Cl | CH₂CN | O |
| D-3099 | Me(R) | single bond | H | *c*-Pr | H | Cl | CH₂CN | O |
| D-3100 | Me(S) | single bond | H | *c*-Pr | H | Cl | CH₂CN | O |
| D-3101 | Me | single bond | H | *c*-Pr | H | Cl | CH₂CN | S |
| D-3102 | Me(R) | single bond | H | *c*-Pr | H | Cl | CH₂CN | S |
| D-3103 | Me(S) | single bond | H | *c*-Pr | H | Cl | CH₂CN | S |
| D-3104 | H | CH₂ | Me | *c*-Pr | H | Cl | CH₂CN | O |
| D-3105 | Me | CH₂ | H | *c*-Pr | H | Cl | CH₂CN | O |
| D-3106 | H | CH(Me) | H | *c*-Pr | H | Cl | CH₂CN | O |
| D-3107 | H | CH₂CH₂ | Me | *c*-Pr | H | Cl | CH₂CN | O |
| D-3108 | Me | CH₂CH₂ | H | *c*-Pr | H | Cl | CH₂CN | O |
| D-3109 | H | single bond | Me | *c*-Pr | H | Me | CH₂CN | O |
| D-3110 | Me | single bond | H | *c*-Pr | H | Me | CH₂CN | O |
| D-3111 | Me(R) | single bond | H | *c*-Pr | H | Me | CH₂CN | O |
| D-3112 | Me(S) | single bond | H | *c*-Pr | H | Me | CH₂CN | O |
| D-3113 | Me | single bond | H | *c*-Pr | H | Me | CH₂CN | S |
| D-3114 | Me(R) | single bond | H | *c*-Pr | H | Me | CH₂CN | S |
| D-3115 | Me(S) | single bond | H | *c*-Pr | H | Me | CH₂CN | S |
| D-3116 | H | CH₂ | Me | *c*-Pr | H | Me | CH₂CN | O |
| D-3117 | Me | CH₂ | H | *c*-Pr | H | Me | CH₂CN | O |
| D-3118 | H | CH(Me) | H | *c*-Pr | H | Me | CH₂CN | O |

**[Table 716]**

| Compound Number | Y | W | Z | R¹ | R² | R³ | R¹⁰ | A |
|---|---|---|---|---|---|---|---|---|
| D-3119 | H | CH₂CH₂ | Me | *c*-Pr | H | Me | CH₂CN | O |
| D-3120 | Me | CH₂CH₂ | H | *c*-Pr | H | Me | CH₂CN | O |
| D-3121 | H | single bond | Me | *c*-Pr | H | Cl | C(=O)Me | O |
| D-3122 | Me | single bond | H | *c*-Pr | H | Cl | C(=O)Me | O |
| D-3123 | Me(R) | single bond | H | *c*-Pr | H | Cl | C(=O)Me | O |
| D-3124 | Me(S) | single bond | H | *c*-Pr | H | Cl | C(=O)Me | O |
| D-3125 | Me | single bond | H | *c*-Pr | H | Cl | C(=O)Me | S |
| D-3126 | Me(R) | single bond | H | *c*-Pr | H | Cl | C(=O)Me | S |
| D-3127 | Me(S) | single bond | H | *c*-Pr | H | Cl | C(=O)Me | S |
| D-3128 | H | CH₂ | Me | *c*-Pr | H | Cl | C(=O)Me | O |
| D-3129 | Me | CH₂ | H | *c*-Pr | H | Cl | C(=O)Me | O |
| D-3130 | H | CH(Me) | H | *c*-Pr | H | Cl | C(=O)Me | O |
| D-3131 | H | CH₂CH₂ | Me | *c*-Pr | H | Cl | C(=O)Me | O |
| D-3132 | Me | CH₂CH₂ | H | *c*-Pr | H | Cl | C(=O)Me | O |
| D-3133 | H | single bond | Me | *c*-Pr | H | Me | C(=O)Me | O |
| D-3134 | Me | single bond | H | *c*-Pr | H | Me | C(=O)Me | O |
| D-3135 | Me(R) | single bond | H | *c*-Pr | H | Me | C(=O)Me | O |
| D-3136 | Me(S) | single bond | H | *c*-Pr | H | Me | C(=O)Me | O |
| D-3137 | Me | single bond | H | *c*-Pr | H | Me | C(=O)Me | S |
| D-3138 | Me(R) | single bond | H | *c*-Pr | H | Me | C(=O)Me | S |
| D-3139 | Me(S) | single bond | H | *c*-Pr | H | Me | C(=O)Me | S |
| D-3140 | H | CH₂ | Me | *c*-Pr | H | Me | C(=O)Me | O |
| D-3141 | Me | CH₂ | H | *c*-Pr | H | Me | C(=O)Me | O |
| D-3142 | H | CH(Me) | H | *c*-Pr | H | Me | C(=O)Me | O |
| D-3143 | H | CH₂CH₂ | Me | *c*-Pr | H | Me | C(=O)Me | O |
| D-3144 | Me | CH₂CH₂ | H | *c*-Pr | H | Me | C(=O)Me | O |
| D-3145 | H | single bond | Me | *c*-Pr | H | Cl | C(=O)OEt | O |
| D-3146 | Me | single bond | H | *c*-Pr | H | Cl | C(=O)OEt | O |
| 0-3147 | Me(R) | single bond | H | *c*-Pr | H | Cl | C(=O)OEt | O |
| D-3148 | Me(S) | single bond | H | *c*-Pr | H | Cl | C(=O)OEt | O |
| D-3149 | Me | single bond | H | *c*-Pr | H | Cl | C(=O)OEt | S |
| D-3150 | Me(R) | single bond | H | *c*-Pr | H | Cl | C(=O)OEt | S |
| D-3151 | Me(S) | single bond | H | *c*-Pr | H | Cl | C(=O)OEt | S |
| D-3152 | H | CH₂ | Me | *c*-Pr | H | Cl | C(=O)OEt | O |
| D-3153 | Me | CH₂ | H | *c*-Pr | H | Cl | C(=O)OEt | O |
| D-3154 | H | CH(Me) | H | *c*-Pr | H | Cl | C(=O)OEt | O |
| D-3155 | H | CH₂CH₂ | Me | *c*-Pr | H | Cl | C(=O)OEt | O |
| D-3156 | Me | CH₂CH₂ | H | *c*-Pr | H | Cl | C(=O)OEt | O |
| D-3157 | H | single bond | Me | *c*-Pr | H | Me | C(=O)OEt | O |
| D-3158 | Me | single bond | H | *c*-Pr | H | Me | C(=O)OEt | O |
| D-3159 | Me(R) | single bond | H | *c*-Pr | H | Me | C(=O)OEt | O |
| D-3160 | Me(S) | single bond | H | *c*-Pr | H | Me | C(=O)OEt | O |
| D-3161 | Me | single bond | H | *c*-Pr | H | Me | C(=O)OEt | S |
| D-3162 | Me(R) | single bond | H | *c*-Pr | H | Me | C(=O)OEt | S |

**[Table 717]**

| Compound Number | Y | W | Z | R¹ | R² | R³ | R¹⁰ | A |
|---|---|---|---|---|---|---|---|---|
| D-3163 | Me(S) | single bond | H | *c*-Pr | H | Me | C(=O)OEt | S |
| D-3164 | H | CH₂ | Me | *c*-Pr | H | Me | C(=O)OEt | O |
| D-3165 | Me | CH₂ | H | *c*-Pr | H | Me | C(=O)OEt | O |
| D-3166 | H | CH(Me) | H | *c*-Pr | H | Me | C(=O)OEt | O |
| D-3167 | H | CH₂CH₂ | Me | *c*-Pr | H | Me | C(=O)OEt | O |
| D-3168 | Me | CH₂CH₂ | H | *c*-Pr | H | Me | C(=O)OEt | O |
| D-3169 | H | single bond | Me | *c*-Pr | Me | Cl | Me | O |
| D-3170 | Me | single bond | H | *c*-Pr | Me | Cl | Me | O |
| D-3171 | Me(R) | single bond | H | *c*-Pr | Me | Cl | Me | O |
| D-3172 | Me(S) | single bond | H | *c*-Pr | Me | Cl | Me | O |
| D-3173 | H | CH₂ | Me | *c*-Pr | Me | Cl | Me | O |
| D-3174 | Me | CH₂ | H | *c*-Pr | Me | Cl | Me | O |
| D-3175 | H | CH(Me) | H | *c*-Pr | Me | Cl | Me | O |
| D-3176 | H | CH₂CH₂ | Me | *c*-Pr | Me | Cl | Me | O |
| D-3177 | Me | CH₂CH₂ | H | *c*-Pr | Me | Cl | Me | O |
| D-3178 | H | single bond | Me | *c*-Pr | Me | Me | Me | O |
| D-3179 | Me | single bond | H | *c*-Pr | Me | Me | Me | O |
| D-3180 | Me(R) | single bond | H | *c*-Pr | Me | Me | Me | O |
| D-3181 | Me(S) | single bond | H | *c*-Pr | Me | Me | Me | O |
| D-3182 | H | CH₂ | Me | *c*-Pr | Me | Me | Me | O |
| D-3183 | Me | CH₂ | H | *c*-Pr | Me | Me | Me | O |
| D-3184 | H | CH(Me) | H | *c*-Pr | Me | Me | Me | O |
| D-3185 | H | CH₂CH₂ | Me | *c*-Pr | Me | Me | Me | O |
| D-3186 | Me | CH₂CH₂ | H | *c*-Pr | Me | Me | Me | O |
| D-3187 | H | single bond | Me | *c*-Pr | Me | Cl | Et | O |
| D-3188 | Me | single bond | H | *c*-Pr | Me | Cl | Et | O |
| D-3189 | Me(R) | single bond | H | *c*-Pr | Me | Cl | Et | O |
| D-3190 | Me(S) | single bond | H | *c*-Pr | Me | Cl | Et | O |
| D-3191 | H | CH₂ | Me | *c*-Pr | Me | Cl | Et | O |
| D-3192 | Me | CH₂ | H | *c*-Pr | Me | Cl | Et | O |
| D-3193 | H | CH(Me) | H | *c*-Pr | Me | Cl | Et | O |
| D-3194 | H | CH₂CH₂ | Me | *c*-Pr | Me | Cl | Et | O |
| D-3195 | Me | CH₂CH₂ | H | *c*-Pr | Me | Cl | Et | O |
| D-3196 | H | single bond | Me | *c*-Pr | Me | Me | Et | O |
| D-3197 | Me | single bond | H | *c*-Pr | Me | Me | Et | O |
| D-3198 | Me(R) | single bond | H | *c*-Pr | Me | Me | Et | O |
| D-3199 | Me(S) | single bond | H | *c*-Pr | Me | Me | Et | O |
| D-3200 | H | CH₂ | Me | *c*-Pr | Me | Me | Et | O |
| D-3201 | Me | CH₂ | H | *c*-Pr | Me | Me | Et | O |
| D-3202 | H | CH(Me) | H | *c*-Pr | Me | Me | Et | O |
| D-3203 | H | CH₂CH₂ | Me | *c*-Pr | Me | Me | Et | O |
| D-3204 | Me | CH₂CH₂ | H | *c*-Pr | Me | Me | Et | O |
| D-3205 | H | single bond | Me | *c*-Pr | Me | Cl | CH₂CF₃ | O |
| D-3206 | Me | single bond | H | *c*-Pr | Me | Cl | CH₂CF₃ | O |

**[Table 718]**

| Compound Number | Y | W | Z | R¹ | R² | R³ | R¹⁰ | A |
|---|---|---|---|---|---|---|---|---|
| D-3207 | Me(R) | single bond | H | *c*-Pr | Me | Cl | CH₂CF₃ | O |
| D-3208 | Me(S) | single bond | H | *c*-Pr | Me | Cl | CH₂CF₃ | O |
| D-3209 | H | CH₂ | Me | *c*-Pr | Me | Cl | CH₂CF₃ | O |
| D-3210 | Me | CH₂ | H | *c*-Pr | Me | Cl | CH₂CF₃ | O |
| D-3211 | H | CH(Me) | H | *c*-Pr | Me | Cl | CH₂CF₃ | O |
| D-3212 | H | CH₂CH₂ | Me | *c*-Pr | Me | Cl | CH₂CF₃ | O |
| D-3213 | Me | CH₂CH₂ | H | *c*-Pr | Me | Cl | CH₂CF₃ | O |
| D-3214 | H | single bond | Me | *c*-Pr | Me | Me | CH₂CF₃ | O |
| D-3215 | Me | single bond | H | *c*-Pr | Me | Me | CH₂CF₃ | O |
| D-3216 | Me(R) | single bond | H | *c*-Pr | Me | Me | CH₂CF₃ | O |
| D-3217 | Me(S) | single bond | H | *c*-Pr | Me | Me | CH₂CF₃ | O |
| D-3218 | H | CH₂ | Me | *c*-Pr | Me | Me | CH₂CF₃ | O |
| D-3219 | Me | CH₂ | H | *c*-Pr | Me | Me | CH₂CF₃ | O |
| D-3220 | H | CH(Me) | H | *c*-Pr | Me | Me | CH₂CF₃ | O |
| D-3221 | H | CH₂CH₂ | Me | *c*-Pr | Me | Me | CH₂CF₃ | O |
| D-3222 | Me | CH₂CH₂ | H | *c*-Pr | Me | Me | CH₂CF₃ | O |
| D-3223 | H | single bond | Me | *c*-Pr | Me | Cl | CH₂OMe | O |
| D-3224 | Me | single bond | H | *c*-Pr | Me | Cl | CH₂OMe | O |
| D-3225 | Me(R) | single bond | H | *c*-Pr | Me | Cl | CH₂OMe | O |
| D-3226 | Me(S) | single bond | H | *c*-Pr | Me | Cl | CH₂OMe | O |
| D-3227 | H | CH₂ | Me | *c*-Pr | Me | Cl | CH₂OMe | O |
| D-3228 | Me | CH₂ | H | *c*-Pr | Me | Cl | CH₂OMe | O |
| D-3229 | H | CH(Me) | H | *c*-Pr | Me | Cl | CH₂OMe | O |
| D-3230 | H | CH₂CH₂ | Me | *c*-Pr | Me | Cl | CH₂OMe | O |
| D-3231 | Me | CH₂CH₂ | H | *c*-Pr | Me | Cl | CH₂OMe | O |
| D-3232 | H | single bond | Me | *c*-Pr | Me | Me | CH₂OMe | O |
| D-3233 | Me | single bond | H | *c*-Pr | Me | Me | CH₂OMe | O |
| D-3234 | Me(R) | single bond | H | *c*-Pr | Me | Me | CH₂OMe | O |
| D-3235 | Me(S) | single bond | H | *c*-Pr | Me | Me | CH₂OMe | O |
| D-3236 | H | CH₂ | Me | *c*-Pr | Me | Me | CH₂OMe | O |
| D-3237 | Me | CH₂ | H | *c*-Pr | Me | Me | CH₂OMe | O |
| D-3238 | H | CH(Me) | H | *c*-Pr | Me | Me | CH₂OMe | O |
| D-3239 | H | CH₂CH₂ | Me | *c*-Pr | Me | Me | CH₂OMe | O |
| D-3240 | Me | CH₂CH₂ | H | *c*-Pr | Me | Me | CH₂OMe | O |
| D-3241 | H | single bond | Me | *c*-Pr | Me | Cl | CH₂CN | O |
| D-3242 | Me | single bond | H | *c*-Pr | Me | Cl | CH₂CN | O |
| D-3243 | Me(R) | single bond | H | *c*-Pr | Me | Cl | CH₂CN | O |
| D-3244 | Me(S) | single bond | H | *c*-Pr | Me | Cl | CH₂CN | O |
| D-3245 | H | CH₂ | Me | *c*-Pr | Me | Cl | CH₂CN | O |
| D-3246 | Me | CH₂ | H | *c*-Pr | Me | Cl | CH₂CN | O |
| D-3247 | H | CH(Me) | H | *c*-Pr | Me | Cl | CH₂CN | O |
| D-3248 | H | CH₂CH₂ | Me | *c*-Pr | Me | Cl | CH₂CN | O |
| D-3249 | Me | CH₂CH₂ | H | *c*-Pr | Me | Cl | CH₂CN | O |
| D-3250 | H | single bond | Me | *c*-Pr | Me | Me | CH₂CN | O |

**[Table 719]**

| Compound Number | Y | W | Z | R¹ | R² | R³ | R¹⁰ | A |
|---|---|---|---|---|---|---|---|---|
| D-3251 | Me | single bond | H | *c*-Pr | Me | Me | CH₂CN | O |
| D-3252 | Me(R) | single bond | H | *c*-Pr | Me | Me | CH₂CN | O |
| D-3253 | Me(S) | single bond | H | *c*-Pr | Me | Me | CH₂CN | O |
| D-3254 | H | CH₂ | Me | *c*-Pr | Me | Me | CH₂CN | O |
| D-3255 | Me | CH₂ | H | *c*-Pr | Me | Me | CH₂CN | O |
| D-3256 | H | CH(Me) | H | *c*-Pr | Me | Me | CH₂CN | O |
| D-3257 | H | CH₂CH₂ | Me | *c*-Pr | Me | Me | CH₂CN | O |
| D-3258 | Me | CH₂CH₂ | H | *c*-Pr | Me | Me | CH₂CN | O |
| D-3259 | H | single bond | Me | *c*-Pr | Me | Cl | C(=O)Me | O |
| D-3260 | Me | single bond | H | *c*-Pr | Me | Cl | C(=O)Me | O |
| D-3261 | Me(R) | single bond | H | *c*-Pr | Me | Cl | C(=O)Me | O |
| D-3262 | Me(S) | single bond | H | *c*-Pr | Me | Cl | C(=O)Me | O |
| D-3263 | H | CH₂ | Me | *c*-Pr | Me | Cl | C(=O)Me | O |
| D-3264 | Me | CH₂ | H | *c*-Pr | Me | Cl | C(=O)Me | O |
| D-3265 | H | CH(Me) | H | *c*-Pr | Me | Cl | C(=O)Me | O |
| D-3266 | H | CH₂CH₂ | Me | *c*-Pr | Me | Cl | C(=O)Me | O |
| D-3267 | Me | CH₂CH₂ | H | *c*-Pr | Me | Cl | C(=O)Me | O |
| D-3268 | H | single bond | Me | *c*-Pr | Me | Me | C(=O)Me | O |
| D-3269 | Me | single bond | H | *c*-Pr | Me | Me | C(=O)Me | O |
| D-3270 | Me(R) | single bond | H | *c*-Pr | Me | Me | C(=O)Me | O |
| D-3271 | Me(S) | single bond | H | *c*-Pr | Me | Me | C(=O)Me | O |
| D-3272 | H | CH₂ | Me | *c*-Pr | Me | Me | C(=O)Me | O |
| D-3273 | Me | CH₂ | H | *c*-Pr | Me | Me | C(=O)Me | O |
| D-3274 | H | CH(Me) | H | *c*-Pr | Me | Me | C(=O)Me | O |
| D-3275 | H | CH₂CH₂ | Me | *c*-Pr | Me | Me | C(=O)Me | O |
| D-3276 | Me | CH₂CH₂ | H | *c*-Pr | Me | Me | C(=O)Me | O |
| D-3277 | H | single bond | Me | *c*-Pr | CH₂OMe | Cl | Me | O |
| D-3278 | Me | single bond | H | *c*-Pr | CH₂OMe | Cl | Me | O |
| D-3279 | Me(R) | single bond | H | *c*-Pr | CH₂OMe | Cl | Me | O |
| D-3280 | Me(S) | single bond | H | *c*-Pr | CH₂OMe | Cl | Me | O |
| D-3281 | H | CH₂ | Me | *c*-Pr | CH₂OMe | Cl | Me | O |
| D-3282 | Me | CH₂ | H | *c*-Pr | CH₂OMe | Cl | Me | O |
| D-3283 | H | CH(Me) | H | *c*-Pr | CH₂OMe | Cl | Me | O |
| D-3284 | H | CH₂CH₂ | Me | *c*-Pr | CH₂OMe | Cl | Me | O |
| D-3285 | Me | CH₂CH₂ | H | *c*-Pr | CH₂OMe | Cl | Me | O |
| D-3286 | H | single bond | Me | *c*-Pr | CH₂OMe | Me | Me | O |
| D-3287 | Me | single bond | H | *c*-Pr | CH₂OMe | Me | Me | O |
| D-3288 | Me(R) | single bond | H | *c*-Pr | CH₂OMe | Me | Me | O |
| D-3289 | Me(S) | single bond | H | *c*-Pr | CH₂OMe | Me | Me | O |
| D-3290 | H | CH₂ | Me | *c*-Pr | CH₂OMe | Me | Me | O |
| D-3291 | Me | CH₂ | H | *c*-Pr | CH₂OMe | Me | Me | O |
| D-3292 | H | CH(Me) | H | *c*-Pr | CH₂OMe | Me | Me | O |
| D-3293 | H | CH₂CH₂ | Me | *c*-Pr | CH₂OMe | Me | Me | O |
| D-3294 | Me | CH₂CH₂ | H | *c*-Pr | CH₂OMe | Me | Me | O |

**[Table 720]**

| Compound Number | Y | W | Z | R¹ | R² | R³ | R¹⁰ | A |
|---|---|---|---|---|---|---|---|---|
| D-3295 | H | single bond | Me | *c*-Pr | CH₂OMe | Cl | Et | O |
| D-3296 | Me | single bond | H | *c*-Pr | CH₂OMe | Cl | Et | O |
| D-3297 | Me(R) | single bond | H | *c*-Pr | CH₂OMe | Cl | Et | O |
| D-3298 | Me(S) | single bond | H | *c*-Pr | CH₂OMe | Cl | Et | O |
| D-3299 | H | CH₂ | Me | *c*-Pr | CH₂OMe | Cl | Et | O |
| D-3300 | Me | CH₂ | H | *c*-Pr | CH₂OMe | Cl | Et | O |
| D-3301 | H | CH(Me) | H | *c*-Pr | CH₂OMe | Cl | Et | O |
| D-3302 | H | CH₂CH₂ | Me | *c*-Pr | CH₂OMe | Cl | Et | O |
| D-3303 | Me | CH₂CH₂ | H | *c*-Pr | CH₂OMe | Cl | Et | O |
| D-3304 | H | single bond | Me | *c*-Pr | CH₂OMe | Me | Et | O |
| D-3305 | Me | single bond | H | *c*-Pr | CH₂OMe | Me | Et | O |
| D-3306 | Me(R) | single bond | H | *c*-Pr | CH₂OMe | Me | Et | O |
| D-3307 | Me(S) | single bond | H | *c*-Pr | CH₂OMe | Me | Et | O |
| D-3308 | H | CH₂ | Me | *c*-Pr | CH₂OMe | Me | Et | O |
| D-3309 | Me | CH₂ | H | *c*-Pr | CH₂OMe | Me | Et | O |
| D-3310 | H | CH(Me) | H | *c*-Pr | CH₂OMe | Me | Et | O |
| D-3311 | H | CH₂CH₂ | Me | *c*-Pr | CH₂OMe | Me | Et | O |
| D-3312 | Me | CH₂CH₂ | H | *c*-Pr | CH₂OMe | Me | Et | O |
| D-3313 | H | single bond | Me | *c*-Pr | CH₂OMe | Cl | CH₂CF₃ | O |
| D-3314 | Me | single bond | H | *c*-Pr | CH₂OMe | Cl | CH₂CF₃ | O |
| D-3315 | Me(R) | single bond | H | *c*-Pr | CH₂OMe | Cl | CH₂CF₃ | O |
| D-3316 | Me(S) | single bond | H | *c*-Pr | CH₂OMe | Cl | CH₂CF₃ | O |
| D-3317 | H | CH₂ | Me | *c*-Pr | CH₂OMe | Cl | CH₂CF₃ | O |
| D-3318 | Me | CH₂ | H | *c*-Pr | CH₂OMe | Cl | CH₂CF₃ | O |
| D-3319 | H | CH(Me) | H | *c*-Pr | CH₂OMe | Cl | CH₂CF₃ | O |
| D-3320 | H | CH₂CH₂ | Me | *c*-Pr | CH₂OMe | Cl | CH₂CF₃ | O |
| D-3321 | Me | CH₂CH₂ | H | *c*-Pr | CH₂OMe | Cl | CH₂CF₃ | O |
| D-3322 | H | single bond | Me | *c*-Pr | CH₂OMe | Me | CH₂CF₃ | O |
| D-3323 | Me | single bond | H | *c*-Pr | CH₂OMe | Me | CH₂CF₃ | O |
| D-3324 | Me(R) | single bond | H | *c*-Pr | CH₂OMe | Me | CH₂CF₃ | O |
| D-3325 | Me(S) | single bond | H | *c*-Pr | CH₂OMe | Me | CH₂CF₃ | O |
| D-3326 | H | CH₂ | Me | *c*-Pr | CH₂OMe | Me | CH₂CF₃ | O |
| D-3327 | Me | CH₂ | H | *c*-Pr | CH₂OMe | Me | CH₂CF₃ | O |
| D-3328 | H | CH(Me) | H | *c*-Pr | CH₂OMe | Me | CH₂CF₃ | O |
| D-3329 | H | CH₂CH₂ | Me | *c*-Pr | CH₂OMe | Me | CH₂CF₃ | O |
| D-3330 | Me | CH₂CH₂ | H | *c*-Pr | CH₂OMe | Me | CH₂CF₃ | O |
| D-3331 | H | single bond | Me | *c*-Pr | CH₂OMe | Cl | CH₂OMe | O |
| D-3332 | Me | single bond | H | *c*-Pr | CH₂OMe | Cl | CH₂OMe | O |
| D-3333 | Me(R) | single bond | H | *c*-Pr | CH₂OMe | Cl | CH₂OMe | O |
| D-3334 | Me(S) | single bond | H | *c*-Pr | CH₂OMe | Cl | CH₂OMe | O |
| D-3335 | H | CH₂ | Me | *c*-Pr | CH₂OMe | Cl | CH₂OMe | O |
| D-3336 | Me | CH₂ | H | *c*-Pr | CH₂OMe | Cl | CH₂OMe | O |
| D-3337 | H | CH(Me) | H | *c*-Pr | CH₂OMe | Cl | CH₂OMe | O |
| D-3338 | H | CH₂CH₂ | Me | *c*-Pr | CH₂OMe | Cl | CH₂OMe | O |

**[Table 721]**

| Compound Number | Y | W | Z | R¹ | R² | R³ | R¹⁰ | A |
|---|---|---|---|---|---|---|---|---|
| D-3339 | Me | CH₂CH₂ | H | *c*-Pr | CH₂OMe | Cl | CH₂OMe | O |
| D-3340 | H | single bond | Me | *c*-Pr | CH₂OMe | Me | CH₂OMe | O |
| D-3341 | Me | single bond | H | *c*-Pr | CH₂OMe | Me | CH₂OMe | O |
| D-3342 | Me(R) | single bond | H | *c*-Pr | CH₂OMe | Me | CH₂OMe | O |
| D-3343 | Me(S) | single bond | H | *c*-Pr | CH₂OMe | Me | CH₂OMe | O |
| D-3344 | H | CH₂ | Me | *c*-Pr | CH₂OMe | Me | CH₂OMe | O |
| D-3345 | Me | CH₂ | H | *c*-Pr | CH₂OMe | Me | CH₂OMe | O |
| D-3346 | H | CH(Me) | H | *c*-Pr | CH₂OMe | Me | CH₂OMe | O |
| D-3347 | H | CH₂CH₂ | Me | *c*-Pr | CH₂OMe | Me | CH₂OMe | O |
| D-3348 | Me | CH₂CH₂ | H | *c*-Pr | CH₂OMe | Me | CH₂OMe | O |
| D-3349 | H | single bond | Me | *c*-Pr | CH₂OMe | Cl | CH₂CN | O |
| D-3350 | Me | single bond | H | *c*-Pr | CH₂OMe | Cl | CH₂CN | O |
| D-3351 | Me(R) | single bond | H | *c*-Pr | CH₂OMe | Cl | CH₂CN | O |
| D-3352 | Me(S) | single bond | H | *c*-Pr | CH₂OMe | Cl | CH₂CN | O |
| D-3353 | H | CH₂ | Me | *c*-Pr | CH₂OMe | Cl | CH₂CN | O |
| D-3354 | Me | CH₂ | H | *c*-Pr | CH₂OMe | Cl | CH₂CN | O |
| D-3355 | H | CH(Me) | H | *c*-Pr | CH₂OMe | Cl | CH₂CN | O |
| D-3356 | H | CH₂CH₂ | Me | *c*-Pr | CH₂OMe | Cl | CH₂CN | O |
| D-3357 | Me | CH₂CH₂ | H | *c*-Pr | CH₂OMe | Cl | CH₂CN | O |
| D-3358 | H | single bond | Me | *c*-Pr | CH₂OMe | Me | CH₂CN | O |
| D-3359 | Me | single bond | H | *c*-Pr | CH₂OMe | Me | CH₂CN | O |
| D-3360 | Me(R) | single bond | H | *c*-Pr | CH₂OMe | Me | CH₂CN | O |
| D-3361 | Me(S) | single bond | H | *c*-Pr | CH₂OMe | Me | CH₂CN | O |
| D-3362 | H | CH₂ | Me | *c*-Pr | CH₂OMe | Me | CH₂CN | O |
| D-3363 | Me | CH₂ | H | *c*-Pr | CH₂OMe | Me | CH₂CN | O |
| D-3364 | H | CH(Me) | H | *c*-Pr | CH₂OMe | Me | CH₂CN | O |
| D-3365 | H | CH₂CH₂ | Me | *c*-Pr | CH₂OMe | Me | CH₂CN | O |
| D-3366 | Me | CH₂CH₂ | H | *c*-Pr | CH₂OMe | Me | CH₂CN | O |
| D-3367 | H | single bond | Me | *c*-Pr | CH₂OMe | Cl | C(=O)Me | O |
| D-3368 | Me | single bond | H | *c*-Pr | CH₂OMe | Cl | C(=O)Me | O |
| D-3369 | Me(R) | single bond | H | *c*-Pr | CH₂OMe | Cl | C(=O)Me | O |
| D-3370 | Me(S) | single bond | H | *c*-Pr | CH₂OMe | Cl | C(=O)Me | O |
| D-3371 | H | CH₂ | Me | *c*-Pr | CH₂OMe | Cl | C(=O)Me | O |
| D-3372 | Me | CH₂ | H | *c*-Pr | CH₂OMe | Cl | C(=O)Me | O |
| D-3373 | H | CH(Me) | H | *c*-Pr | CH₂OMe | Cl | C(=O)Me | O |
| D-3374 | H | CH₂CH₂ | Me | *c*-Pr | CH₂OMe | Cl | C(=O)Me | O |
| D-3375 | Me | CH₂CH₂ | H | *c*-Pr | CH₂OMe | Cl | C(=O)Me | O |
| D-3376 | H | single bond | Me | *c*-Pr | CH₂OMe | Me | C(=O)Me | O |
| D-3377 | Me | single bond | H | *c*-Pr | CH₂OMe | Me | C(=O)Me | O |
| D-3378 | Me(R) | single bond | H | *c*-Pr | CH₂OMe | Me | C(=O)Me | O |
| D-3379 | Me(S) | single bond | H | *c*-Pr | CH₂OMe | Me | C(=O)Me | O |
| D-3380 | H | CH₂ | Me | *c*-Pr | CH₂OMe | Me | C(=O)Me | O |
| D-3381 | Me | CH₂ | H | *c*-Pr | CH₂OMe | Me | C(=O)Me | O |
| D-3382 | H | CH(Me) | H | *c*-Pr | CH₂OMe | Me | C(=O)Me | O |

**[Table 722]**

| Compound Number | Y | W | Z | R¹ | R² | R³ | R¹⁰ | A |
|---|---|---|---|---|---|---|---|---|
| D-3383 | H | CH₂CH₂ | Me | *c*-Pr | CH₂OMe | Me | C(=O)Me | O |
| D-3384 | Me | CH₂CH₂ | H | *c*-Pr | CH₂OMe | Me | C(=O)Me | O |
| D-3385 | H | single bond | Me | *c*-Pr | CH₂CN | Cl | Me | O |
| D-3386 | Me | single bond | H | *c*-Pr | CH₂CN | Cl | Me | O |
| D-3387 | Me(R) | single bond | H | *c*-Pr | CH₂CN | Cl | Me | O |
| D-3388 | Me(S) | single bond | H | *c*-Pr | CH₂CN | Cl | Me | O |
| D-3389 | H | CH₂ | Me | *c*-Pr | CH₂CN | Cl | Me | O |
| D-3390 | Me | CH₂ | H | *c*-Pr | CH₂CN | Cl | Me | O |
| D-3391 | H | CH(Me) | H | *c*-Pr | CH₂CN | Cl | Me | O |
| D-3392 | H | CH₂CH₂ | Me | *c*-Pr | CH₂CN | Cl | Me | O |
| D-3393 | Me | CH₂CH₂ | H | *c*-Pr | CH₂CN | Cl | Me | O |
| D-3394 | H | single bond | Me | *c*-Pr | CH₂CN | Me | Me | O |
| D-3395 | Me | single bond | H | *c*-Pr | CH₂CN | Me | Me | O |
| D-3396 | Me(R) | single bond | H | *c*-Pr | CH₂CN | Me | Me | O |
| D-3397 | Me(S) | single bond | H | *c*-Pr | CH₂CN | Me | Me | O |
| D-3398 | H | CH₂ | Me | *c*-Pr | CH₂CN | Me | Me | O |
| D-3399 | Me | CH₂ | H | *c*-Pr | CH₂CN | Me | Me | O |
| D-3400 | H | CH(Me) | H | *c*-Pr | CH₂CN | Me | Me | O |
| D-3401 | H | CH₂CH₂ | Me | *c*-Pr | CH₂CN | Me | Me | O |
| D-3402 | Me | CH₂CH₂ | H | *c*-Pr | CH₂CN | Me | Me | O |
| D-3403 | H | single bond | Me | *c*-Pr | CH₂CN | Cl | Et | O |
| D-3404 | Me | single bond | H | *c*-Pr | CH₂CN | Cl | Et | O |
| D-3405 | Me(R) | single bond | H | *c*-Pr | CH₂CN | Cl | Et | O |
| D-3406 | Me(S) | single bond | H | *c*-Pr | CH₂CN | Cl | Et | O |
| D-3407 | H | CH₂ | Me | *c*-Pr | CH₂CN | Cl | Et | O |
| D-3408 | Me | CH₂ | H | *c*-Pr | CH₂CN | Cl | Et | O |
| D-3409 | H | CH(Me) | H | *c*-Pr | CH₂CN | Cl | Et | O |
| D-3410 | H | CH₂CH₂ | Me | *c*-Pr | CH₂CN | Cl | Et | O |
| D-3411 | Me | CH₂CH₂ | H | *c*-Pr | CH₂CN | Cl | Et | O |
| D-3412 | H | single bond | Me | *c*-Pr | CH₂CN | Me | Et | O |
| D-3413 | Me | single bond | H | *c*-Pr | CH₂CN | Me | Et | O |
| D-3414 | Me(R) | single bond | H | *c*-Pr | CH₂CN | Me | Et | O |
| D-3415 | Me(S) | single bond | H | *c*-Pr | CH₂CN | Me | Et | O |
| D-3416 | H | CH₂ | Me | *c*-Pr | CH₂CN | Me | Et | O |
| D-3417 | Me | CH₂ | H | *c*-Pr | CH₂CN | Me | Et | O |
| D-3418 | H | CH(Me) | H | *c*-Pr | CH₂CN | Me | Et | O |
| D-3419 | H | CH₂CH₂ | Me | *c*-Pr | CH₂CN | Me | Et | O |
| D-3420 | Me | CH₂CH₂ | H | *c*-Pr | CH₂CN | Me | Et | O |
| D-3421 | H | single bond | Me | *c*-Pr | CH₂CN | Cl | CH₂CF₃ | O |
| D-3422 | Me | single bond | H | *c*-Pr | CH₂CN | Cl | CH₂CF₃ | O |
| D-3423 | Me(R) | single bond | H | *c*-Pr | CH₂CN | Cl | CH₂CF₃ | O |
| D-3424 | Me(S) | single bond | H | *c*-Pr | CH₂CN | Cl | CH₂CF₃ | O |
| D-3425 | H | CH₂ | Me | *c*-Pr | CH₂CN | Cl | CH₂CF₃ | O |
| D-3426 | Me | CH₂ | H | *c*-Pr | CH₂CN | Cl | CH₂CF₃ | O |

**[Table 723]**

| Compound Number | Y | W | Z | R¹ | R² | R³ | R¹⁰ | A |
|---|---|---|---|---|---|---|---|---|
| D-3427 | H | CH(Me) | H | *c*-Pr | CH₂CN | Cl | CH₂CF₃ | O |
| D-3428 | H | CH₂CH₂ | Me | *c*-Pr | CH₂CN | Cl | CH₂CF₃ | O |
| D-3429 | Me | CH₂CH₂ | H | *c*-Pr | CH₂CN | Cl | CH₂CF₃ | O |
| D-3430 | H | single bond | Me | *c*-Pr | CH₂CN | Me | CH₂CF₃ | O |
| D-3431 | Me | single bond | H | *c*-Pr | CH₂CN | Me | CH₂CF₃ | O |
| D-3432 | Me(R) | single bond | H | *c*-Pr | CH₂CN | Me | CH₂CF₃ | O |
| D-3433 | Me(S) | single bond | H | *c*-Pr | CH₂CN | Me | CH₂CF₃ | O |
| D-3434 | H | CH₂ | Me | *c*-Pr | CH₂CN | Me | CH₂CF₃ | O |
| D-3435 | Me | CH₂ | H | *c*-Pr | CH₂CN | Me | CH₂CF₃ | O |
| D-3436 | H | CH(Me) | H | *c*-Pr | CH₂CN | Me | CH₂CF₃ | O |
| D-3437 | H | CH₂CH₂ | Me | *c*-Pr | CH₂CN | Me | CH₂CF₃ | O |
| D-3438 | Me | CH₂CH₂ | H | *c*-Pr | CH₂CN | Me | CH₂CF₃ | O |
| D-3439 | H | single bond | Me | *c*-Pr | CH₂CN | Cl | CH₂OMe | O |
| D-3440 | Me | single bond | H | *c*-Pr | CH₂CN | Cl | CH₂OMe | O |
| D-3441 | Me(R) | single bond | H | *c*-Pr | CH₂CN | Cl | CH₂OMe | O |
| D-3442 | Me(S) | single bond | H | *c*-Pr | CH₂CN | Cl | CH₂OMe | O |
| D-3443 | H | CH₂ | Me | *c*-Pr | CH₂CN | Cl | CH₂OMe | O |
| D-3444 | Me | CH₂ | H | *c*-Pr | CH₂CN | Cl | CH₂OMe | O |
| D-3445 | H | CH(Me) | H | *c*-Pr | CH₂CN | Cl | CH₂OMe | O |
| D-3446 | H | CH₂CH₂ | Me | *c*-Pr | CH₂CN | Cl | CH₂OMe | O |
| D-3447 | Me | CH₂CH₂ | H | *c*-Pr | CH₂CN | Cl | CH₂OMe | O |
| D-3448 | H | single bond | Me | *c*-Pr | CH₂CN | Me | CH₂OMe | O |
| D-3449 | Me | single bond | H | *c*-Pr | CH₂CN | Me | CH₂OMe | O |
| D-3450 | Me(R) | single bond | H | *c*-Pr | CH₂CN | Me | CH₂OMe | O |
| D-3451 | Me(S) | single bond | H | *c*-Pr | CH₂CN | Me | CH₂OMe | O |
| D-3452 | H | CH₂ | Me | *c*-Pr | CH₂CN | Me | CH₂OMe | O |
| D-3453 | Me | CH₂ | H | *c*-Pr | CH₂CN | Me | CH₂OMe | O |
| D-3454 | H | CH(Me) | H | *c*-Pr | CH₂CN | Me | CH₂OMe | O |
| D-3455 | H | CH₂CH₂ | Me | *c*-Pr | CH₂CN | Me | CH₂OMe | O |
| D-3456 | Me | CH₂CH₂ | H | *c*-Pr | CH₂CN | Me | CH₂OMe | O |
| D-3457 | H | single bond | Me | *c*-Pr | CH₂CN | Cl | CH₂CN | O |
| D-3458 | Me | single bond | H | *c*-Pr | CH₂CN | Cl | CH₂CN | O |
| D-3459 | Me(R) | single bond | H | *c*-Pr | CH₂CN | Cl | CH₂CN | O |
| D-3460 | Me(S) | single bond | H | *c*-Pr | CH₂CN | Cl | CH₂CN | O |
| D-3461 | H | CH₂ | Me | *c*-Pr | CH₂CN | Cl | CH₂CN | O |
| D-3462 | Me | CH₂ | H | *c*-Pr | CH₂CN | Cl | CH₂CN | O |
| D-3463 | H | CH(Me) | H | *c*-Pr | CH₂CN | Cl | CH₂CN | O |
| D-3464 | H | CH₂CH₂ | Me | *c*-Pr | CH₂CN | Cl | CH₂CN | O |
| D-3465 | Me | CH₂CH₂ | H | *c*-Pr | CH₂CN | Cl | CH₂CN | O |
| D-3466 | H | single bond | Me | *c*-Pr | CH₂CN | Me | CH₂CN | O |
| D-3467 | Me | single bond | H | *c*-Pr | CH₂CN | Me | CH₂CN | O |
| D-3468 | Me(R) | single bond | H | *c*-Pr | CH₂CN | Me | CH₂CN | O |
| D-3469 | Me(S) | single bond | H | *c*-Pr | CH₂CN | Me | CH₂CN | O |
| D-3470 | H | CH₂ | Me | *c*-Pr | CH₂CN | Me | CH₂CN | O |

**[Table 724]**

| Compound Number | Y | W | Z | R¹ | R² | R³ | R¹⁰ | A |
|---|---|---|---|---|---|---|---|---|
| D-3471 | Me | CH₂ | H | *c*-Pr | CH₂CN | Me | CH₂CN | O |
| D-3472 | H | CH(Me) | H | *c*-Pr | CH₂CN | Me | CH₂CN | O |
| D-3473 | H | CH₂CH₂ | Me | *c*-Pr | CH₂CN | Me | CH₂CN | O |
| D-3474 | Me | CH₂CH₂ | H | *c*-Pr | CH₂CN | Me | CH₂CN | O |
| D-3475 | H | single bond | Me | *c*-Pr | CH₂CN | Cl | C(=O)Me | O |
| D-3476 | Me | single bond | H | *c*-Pr | CH₂CN | Cl | C(=O)Me | O |
| D-3477 | Me(R) | single bond | H | *c*-Pr | CH₂CN | Cl | C(=O)Me | O |
| D-3478 | Me(S) | single bond | H | *c*-Pr | CH₂CN | Cl | C(=O)Me | O |
| D-3479 | H | CH₂ | Me | *c*-Pr | CH₂CN | Cl | C(=O)Me | O |
| D-3480 | Me | CH₂ | H | *c*-Pr | CH₂CN | Cl | C(=O)Me | O |
| D-3481 | H | CH(Me) | H | *c*-Pr | CH₂CN | Cl | C(=O)Me | O |
| D-3482 | H | CH₂CH₂ | Me | *c*-Pr | CH₂CN | Cl | C(=O)Me | O |
| D-3483 | Me | CH₂CH₂ | H | *c*-Pr | CH₂CN | Cl | C(=O)Me | O |
| D-3484 | H | single bond | Me | *c*-Pr | CH₂CN | Me | C(=O)Me | O |
| D-3485 | Me | single bond | H | *c*-Pr | CH₂CN | Me | C(=O)Me | O |
| D-3486 | Me(R) | single bond | H | *c*-Pr | CH₂CN | Me | C(=O)Me | O |
| D-3487 | Me(S) | single bond | H | *c*-Pr | CH₂CN | Me | C(=O)Me | O |
| D-3488 | H | CH₂ | Me | *c*-Pr | CH₂CN | Me | C(=O)Me | O |
| D-3489 | Me | CH₂ | H | *c*-Pr | CH₂CN | Me | C(=O)Me | O |
| D-3490 | H | CH(Me) | H | *c*-Pr | CH₂CN | Me | C(=O)Me | O |
| D-3491 | H | CH₂CH₂ | Me | *c*-Pr | CH₂CN | Me | C(=O)Me | O |
| D-3492 | Me | CH₂CH₂ | H | *c*-Pr | CH₂CN | Me | C(=O)Me | O |
| D-3493 | H | single bond | Me | *c*-Pr | C(=O)Me | Cl | Me | O |
| D-3494 | Me | single bond | H | *c*-Pr | C(=O)Me | Cl | Me | O |
| D-3495 | Me(R) | single bond | H | *c*-Pr | C(=O)Me | Cl | Me | O |
| D-3496 | Me(S) | single bond | H | *c*-Pr | C(=O)Me | Cl | Me | O |
| D-3497 | H | CH₂ | Me | *c*-Pr | C(=O)Me | Cl | Me | O |
| D-3498 | Me | CH₂ | H | *c*-Pr | C(=O)Me | Cl | Me | O |
| D-3499 | H | CH(Me) | H | *c*-Pr | C(=O)Me | Cl | Me | O |
| D-3500 | H | CH₂CH₂ | Me | *c*-Pr | C(=O)Me | Cl | Me | O |
| D-3501 | Me | CH₂CH₂ | H | *c*-Pr | C(=O)Me | Cl | Me | O |
| D-3502 | H | single bond | Me | *c*-Pr | C(=O)Me | Me | Me | O |
| D-3503 | Me | single bond | H | *c*-Pr | C(=O)Me | Me | Me | O |
| D-3504 | Me(R) | single bond | H | *c*-Pr | C(=O)Me | Me | Me | O |
| D-3505 | Me(S) | single bond | H | *c*-Pr | C(=O)Me | Me | Me | O |
| D-3506 | H | CH₂ | Me | *c*-Pr | C(=O)Me | Me | Me | O |
| D-3507 | Me | CH₂ | H | *c*-Pr | C(=O)Me | Me | Me | O |
| D-3508 | H | CH(Me) | H | *c*-Pr | C(=O)Me | Me | Me | O |
| D-3509 | H | CH₂CH₂ | Me | *c*-Pr | C(=O)Me | Me | Me | O |
| D-3510 | Me | CH₂CH₂ | H | *c*-Pr | C(=O)Me | Me | Me | O |
| D-3511 | H | single bond | Me | *c*-Pr | C(=O)Me | Cl | Et | O |
| D-3512 | Me | single bond | H | *c*-Pr | C(=O)Me | Cl | Et | O |
| D-3513 | Me(R) | single bond | H | *c*-Pr | C(=O)Me | Cl | Et | O |
| D-3514 | Me(S) | single bond | H | *c*-Pr | C(=O)Me | Cl | Et | O |

**[Table 725]**

| Compound Number | Y | W | Z | R¹ | R² | R³ | R¹⁰ | A |
|---|---|---|---|---|---|---|---|---|
| D-3515 | H | CH₂ | Me | *c*-Pr | C(=O)Me | Cl | Et | O |
| D-3516 | Me | CH₂ | H | *c*-Pr | C(=O)Me | Cl | Et | O |
| D-3517 | H | CH(Me) | H | *c*-Pr | C(=O)Me | Cl | Et | O |
| D-3518 | H | CH₂CH₂ | Me | *c*-Pr | C(=O)Me | Cl | Et | O |
| D-3519 | Me | CH₂CH₂ | H | *c*-Pr | C(=O)Me | Cl | Et | O |
| D-3520 | H | single bond | Me | *c*-Pr | C(=O)Me | Me | Et | O |
| D-3521 | Me | single bond | H | *c*-Pr | C(=O)Me | Me | Et | O |
| D-3522 | Me(R) | single bond | H | *c*-Pr | C(=O)Me | Me | Et | O |
| D-3523 | Me(S) | single bond | H | *c*-Pr | C(=O)Me | Me | Et | O |
| D-3524 | H | CH₂ | Me | *c*-Pr | C(=O)Me | Me | Et | O |
| D-3525 | Me | CH₂ | H | *c*-Pr | C(=O)Me | Me | Et | O |
| D-3526 | H | CH(Me) | H | *c*-Pr | C(=O)Me | Me | Et | O |
| D-3527 | H | CH₂CH₂ | Me | *c*-Pr | C(=O)Me | Me | Et | O |
| D-3528 | Me | CH₂CH₂ | H | *c*-Pr | C(=O)Me | Me | Et | O |
| D-3529 | H | single bond | Me | *c*-Pr | C(=O)Me | Cl | CH₂CF₃ | O |
| D-3530 | Me | single bond | H | *c*-Pr | C(=O)Me | Cl | CH₂CF₃ | O |
| D-3531 | Me(R) | single bond | H | *c*-Pr | C(=O)Me | Cl | CH₂CF₃ | O |
| D-3532 | Me(S) | single bond | H | *c*-Pr | C(=O)Me | Cl | CH₂CF₃ | O |
| D-3533 | H | CH₂ | Me | *c*-Pr | C(=O)Me | Cl | CH₂CF₃ | O |
| D-3534 | Me | CH₂ | H | *c*-Pr | C(=O)Me | Cl | CH₂CF₃ | O |
| D-3535 | H | CH(Me) | H | *c*-Pr | C(=O)Me | Cl | CH₂CF₃ | O |
| D-3536 | H | CH₂CH₂ | Me | *c*-Pr | C(=O)Me | Cl | CH₂CF₃ | O |
| D-3537 | Me | CH₂CH₂ | H | *c*-Pr | C(=O)Me | Cl | CH₂CF₃ | O |
| D-3538 | H | single bond | Me | *c*-Pr | C(=O)Me | Me | CH₂CF₃ | O |
| D-3539 | Me | single bond | H | *c*-Pr | C(=O)Me | Me | CH₂CF₃ | O |
| D-3540 | Me(R) | single bond | H | *c*-Pr | C(=O)Me | Me | CH₂CF₃ | O |
| D-3541 | Me(S) | single bond | H | *c*-Pr | C(=O)Me | Me | CH₂CF₃ | O |
| D-3542 | H | CH₂ | Me | *c*-Pr | C(=O)Me | Me | CH₂CF₃ | O |
| D-3543 | Me | CH₂ | H | *c*-Pr | C(=O)Me | Me | CH₂CF₃ | O |
| D-3544 | H | CH(Me) | H | *c*-Pr | C(=O)Me | Me | CH₂CF₃ | O |
| D-3545 | H | CH₂CH₂ | Me | *c*-Pr | C(=O)Me | Me | CH₂CF₃ | O |
| D-3546 | Me | CH₂CH₂ | H | *c*-Pr | C(=O)Me | Me | CH₂CF₃ | O |
| D-3547 | H | single bond | Me | *c*-Pr | C(=O)Me | Cl | CH₂OMe | O |
| D-3548 | Me | single bond | H | *c*-Pr | C(=O)Me | Cl | CH₂OMe | O |
| D-3549 | Me(R) | single bond | H | *c*-Pr | C(=O)Me | Cl | CH₂OMe | O |
| D-3550 | Me(S) | single bond | H | *c*-Pr | C(=O)Me | Cl | CH₂OMe | O |
| D-3551 | H | CH₂ | Me | *c*-Pr | C(=O)Me | Cl | CH₂OMe | O |
| D-3552 | Me | CH₂ | H | *c*-Pr | C(=O)Me | Cl | CH₂OMe | O |
| D-3553 | H | CH(Me) | H | *c*-Pr | C(=O)Me | Cl | CH₂OMe | O |
| D-3554 | H | CH₂CH₂ | Me | *c*-Pr | C(=O)Me | Cl | CH₂OMe | O |
| D-3555 | Me | CH₂CH₂ | H | *c*-Pr | C(=O)Me | Cl | CH₂OMe | O |
| D-3556 | H | single bond | Me | *c*-Pr | C(=O)Me | Me | CH₂OMe | O |
| D-3557 | Me | single bond | H | *c*-Pr | C(=O)Me | Me | CH₂OMe | O |
| D-3558 | Me(R) | single bond | H | *c*-Pr | C(=O)Me | Me | CH₂OMe | O |

**[Table 726]**

| Compound Number | Y | W | Z | R¹ | R² | R³ | R¹⁰ | A |
|---|---|---|---|---|---|---|---|---|
| D-3559 | Me(S) | single bond | H | *c*-Pr | C(=O)Me | Me | CH₂OMe | O |
| D-3560 | H | CH₂ | Me | *c*-Pr | C(=O)Me | Me | CH₂OMe | O |
| D-3561 | Me | CH₂ | H | *c*-Pr | C(=O)Me | Me | CH₂OMe | O |
| D-3562 | H | CH(Me) | H | *c*-Pr | C(=O)Me | Me | CH₂OMe | O |
| D-3563 | H | CH₂CH₂ | Me | *c*-Pr | C(=O)Me | Me | CH₂OMe | O |
| D-3564 | Me | CH₂CH₂ | H | *c*-Pr | C(=O)Me | Me | CH₂OMe | O |
| D-3565 | H | single bond | Me | *c*-Pr | C(=O)Me | Cl | CH₂CN | O |
| D-3566 | Me | single bond | H | *c*-Pr | C(=O)Me | Cl | CH₂CN | O |
| D-3567 | Me(R) | single bond | H | *c*-Pr | C(=O)Me | Cl | CH₂CN | O |
| D-3568 | Me(S) | single bond | H | *c*-Pr | C(=O)Me | Cl | CH₂CN | O |
| D-3569 | H | CH₂ | Me | *c*-Pr | C(=O)Me | Cl | CH₂CN | O |
| D-3570 | Me | CH₂ | H | *c*-Pr | C(=O)Me | Cl | CH₂CN | O |
| D-3571 | H | CH(Me) | H | *c*-Pr | C(=O)Me | Cl | CH₂CN | O |
| D-3572 | H | CH₂CH₂ | Me | *c*-Pr | C(=O)Me | Cl | CH₂CN | O |
| D-3573 | Me | CH₂CH₂ | H | *c*-Pr | C(=O)Me | Cl | CH₂CN | O |
| D-3574 | H | single bond | Me | *c*-Pr | C(=O)Me | Me | CH₂CN | O |
| D-3575 | Me | single bond | H | *c*-Pr | C(=O)Me | Me | CH₂CN | O |
| D-3576 | Me(R) | single bond | H | *c*-Pr | C(=O)Me | Me | CH₂CN | O |
| D-3577 | Me(S) | single bond | H | *c*-Pr | C(=O)Me | Me | CH₂CN | O |
| D-3578 | H | CH₂ | Me | *c*-Pr | C(=O)Me | Me | CH₂CN | O |
| D-3579 | Me | CH₂ | H | *c*-Pr | C(=O)Me | Me | CH₂CN | O |
| D-3580 | H | CH(Me) | H | *c*-Pr | C(=O)Me | Me | CH₂CN | O |
| D-3581 | H | CH₂CH₂ | Me | *c*-Pr | C(=O)Me | Me | CH₂CN | O |
| D-3582 | Me | CH₂CH₂ | H | *c*-Pr | C(=O)Me | Me | CH₂CN | O |
| D-3583 | H | single bond | Me | *c*-Pr | C(=O)Me | Cl | C(=O)Me | O |
| D-3584 | Me | single bond | H | *c*-Pr | C(=O)Me | Cl | C(=O)Me | O |
| D-3585 | Me(R) | single bond | H | *c*-Pr | C(=O)Me | Cl | C(=O)Me | O |
| D-3586 | Me(S) | single bond | H | *c*-Pr | C(=O)Me | Cl | C(=O)Me | O |
| D-3587 | H | CH₂ | Me | *c*-Pr | C(=O)Me | Cl | C(=O)Me | O |
| D-3588 | Me | CH₂ | H | *c*-Pr | C(=O)Me | Cl | C(=O)Me | O |
| D-3589 | H | CH(Me) | H | *c*-Pr | C(=O)Me | Cl | C(=O)Me | O |
| D-3590 | H | CH₂CH₂ | Me | *c*-Pr | C(=O)Me | Cl | C(=O)Me | O |
| D-3591 | Me | CH₂CH₂ | H | *c*-Pr | C(=O)Me | Cl | C(=O)Me | O |
| D-3592 | H | single bond | Me | *c*-Pr | C(=O)Me | Me | C(=O)Me | O |
| D-3593 | Me | single bond | H | *c*-Pr | C(=O)Me | Me | C(=O)Me | O |
| D-3594 | Me(R) | single bond | H | *c*-Pr | C(=O)Me | Me | C(=O)Me | O |
| D-3595 | Me(S) | single bond | H | *c*-Pr | C(=O)Me | Me | C(=O)Me | O |
| D-3596 | H | CH₂ | Me | *c*-Pr | C(=O)Me | Me | C(=O)Me | O |
| D-3597 | Me | CH₂ | H | *c*-Pr | C(=O)Me | Me | C(=O)Me | O |
| D-3598 | H | CH(Me) | H | *c*-Pr | C(=O)Me | Me | C(=O)Me | O |
| D-3599 | H | CH₂CH₂ | Me | *c*-Pr | C(=O)Me | Me | C(=O)Me | O |
| D-3600 | Me | CH₂CH₂ | H | *c*-Pr | C(=O)Me | Me | C(=O)Me | O |
| D-3601 | H | single bond | Me | *c*-Pr | C(=O)(*i*-Pr) | Cl | Me | O |
| D-3602 | Me | single bond | H | *c*-Pr | C(=O)(*i*-Pr) | Cl | Me | O |

**[Table 727]**

| Compound Number | Y | W | Z | R¹ | R² | R³ | R¹⁰ | A |
|---|---|---|---|---|---|---|---|---|
| D-3603 | Me(R) | single bond | H | *c*-Pr | C(=O)(*i*-Pr) | Cl | Me | O |
| D-3604 | Me(S) | single bond | H | *c*-Pr | C(=O)(*i*-Pr) | Cl | Me | O |
| D-3605 | H | CH₂ | Me | *c*-Pr | C(=O)(*i*-Pr) | Cl | Me | O |
| D-3606 | Me | CH₂ | H | *c*-Pr | C(=O)(*i*-Pr) | Cl | Me | O |
| D-3607 | H | CH(Me) | H | *c*-Pr | C(=O)(*i*-Pr) | Cl | Me | O |
| D-3608 | H | CH₂CH₂ | Me | *c*-Pr | C(=O)(*i*-Pr) | Cl | Me | O |
| D-3609 | Me | CH₂CH₂ | H | *c*-Pr | C(=O)(*i*-Pr) | Cl | Me | O |
| D-3610 | H | single bond | Me | *c*-Pr | C(=O)(*i*-Pr) | Me | Me | O |
| D-3611 | Me | single bond | H | *c*-Pr | C(=O)(*i*-Pr) | Me | Me | O |
| D-3612 | Me(R) | single bond | H | *c*-Pr | C(=O)(*i*-Pr) | Me | Me | O |
| D-3613 | Me(S) | single bond | H | *c*-Pr | C(=O)(*i*-Pr) | Me | Me | O |
| D-3614 | H | CH₂ | Me | *c*-Pr | C(=O)(*i*-Pr) | Me | Me | O |
| D-3615 | Me | CH₂ | H | *c*-Pr | C(=O)(*i*-Pr) | Me | Me | O |
| D-3616 | H | CH(Me) | H | *c*-Pr | C(=O)(*i*-Pr) | Me | Me | O |
| D-3617 | H | CH₂CH₂ | Me | *c*-Pr | C(=O)(*i*-Pr) | Me | Me | O |
| D-3618 | Me | CH₂CH₂ | H | *c*-Pr | C(=O)(*i*-Pr) | Me | Me | O |
| D-3619 | H | single bond | Me | *c*-Pr | C(=O)(*i*-Pr) | Cl | Et | O |
| D-3620 | Me | single bond | H | *c*-Pr | C(=O)(*i*-Pr) | Cl | Et | O |
| D-3621 | Me(R) | single bond | H | *c*-Pr | C(=O)(*i*-Pr) | Cl | Et | O |
| D-3622 | Me(S) | single bond | H | *c*-Pr | C(=O)(*i*-Pr) | Cl | Et | O |
| D-3623 | H | CH₂ | Me | *c*-Pr | C(=O)(*i*-Pr) | Cl | Et | O |
| D-3624 | Me | CH₂ | H | *c*-Pr | C(=O)(*i*-Pr) | Cl | Et | O |
| D-3625 | H | CH(Me) | H | *c*-Pr | C(=O)(*i*-Pr) | Cl | Et | O |
| D-3626 | H | CH₂CH₂ | Me | *c*-Pr | C(=O)(*i*-Pr) | Cl | Et | O |
| D-3627 | Me | CH₂CH₂ | H | *c*-Pr | C(=O)(*i*-Pr) | Cl | Et | O |
| D-3628 | H | single bond | Me | *c*-Pr | C(=O)(*i*-Pr) | Me | Et | O |
| D-3629 | Me | single bond | H | *c*-Pr | C(=O)(*i*-Pr) | Me | Et | O |
| D-3630 | Me(R) | single bond | H | *c*-Pr | C(=O)(*i*-Pr) | Me | Et | O |
| D-3631 | Me(S) | single bond | H | *c*-Pr | C(=O)(*i*-Pr) | Me | Et | O |
| D-3632 | H | CH₂ | Me | *c*-Pr | C(=O)(*i*-Pr) | Me | Et | O |
| D-3633 | Me | CH₂ | H | *c*-Pr | C(=O)(*i*-Pr) | Me | Et | O |
| D-3634 | H | CH(Me) | H | *c*-Pr | C(=O)(*i*-Pr) | Me | Et | O |
| D-3635 | H | CH₂CH₂ | Me | *c*-Pr | C(=O)(*i*-Pr) | Me | Et | O |
| D-3636 | Me | CH₂CH₂ | H | *c*-Pr | C(=O)(*i*-Pr) | Me | Et | O |
| D-3637 | H | single bond | Me | *c*-Pr | C(=O)(*i*-Pr) | Cl | CH₂CF₃ | O |
| D-3638 | Me | single bond | H | *c*-Pr | C(=O)(*i*-Pr) | Cl | CH₂CF₃ | O |
| D-3639 | Me(R) | single bond | H | *c*-Pr | C(=O)(*i*-Pr) | Cl | CH₂CF₃ | O |
| D-3640 | Me(S) | single bond | H | *c*-Pr | C(=O)(*i*-Pr) | Cl | CH₂CF₃ | O |
| D-3641 | H | CH₂ | Me | *c*-Pr | C(=O)(*i*-Pr) | Cl | CH₂CF₃ | O |
| D-3642 | Me | CH₂ | H | *c*-Pr | C(=O)(*i*-Pr) | Cl | CH₂CF₃ | O |
| D-3643 | H | CH(Me) | H | *c*-Pr | C(=O)(*i*-Pr) | Cl | CH₂CF₃ | O |
| D-3644 | H | CH₂CH₂ | Me | *c*-Pr | C(=O)(*i*-Pr) | Cl | CH₂CF₃ | O |
| D-3645 | Me | CH₂CH₂ | H | *c*-Pr | C(=O)(*i*-Pr) | Cl | CH₂CF₃ | O |
| D-3646 | H | single bond | Me | *c*-Pr | C(=O)(*i*-Pr) | Me | CH₂CF₃ | O |

**[Table 728]**

| Compound Number | Y | W | Z | R¹ | R² | R³ | R¹⁰ | A |
|---|---|---|---|---|---|---|---|---|
| D-3647 | Me | single bond | H | *c*-Pr | C(=O)(*i*-Pr) | Me | CH₂CF₃ | O |
| D-3648 | Me(R) | single bond | H | *c*-Pr | C(=O)(*i*-Pr) | Me | CH₂CF₃ | O |
| D-3649 | Me(S) | single bond | H | *c*-Pr | C(=O)(*i*-Pr) | Me | CH₂CF₃ | O |
| D-3650 | H | CH₂ | Me | *c*-Pr | C(=O)(*i*-Pr) | Me | CH₂CF₃ | O |
| D-3651 | Me | CH₂ | H | *c*-Pr | C(=O)(*i*-Pr) | Me | CH₂CF₃ | O |
| D-3652 | H | CH(Me) | H | *c*-Pr | C(=O)(*i*-Pr) | Me | CH₂CF₃ | O |
| D-3653 | H | CH₂CH₂ | Me | *c*-Pr | C(=O)(*i*-Pr) | Me | CH₂CF₃ | O |
| D-3654 | Me | CH₂CH₂ | H | *c*-Pr | C(=O)(*i*-Pr) | Me | CH₂CF₃ | O |
| D-3655 | H | single bond | Me | *c*-Pr | C(=O)(*i*-Pr) | Cl | CH₂OMe | O |
| D-3656 | Me | single bond | H | *c*-Pr | C(=O)(*i*-Pr) | Cl | CH₂OMe | O |
| D-3657 | Me(R) | single bond | H | *c*-Pr | C(=O)(*i*-Pr) | Cl | CH₂OMe | O |
| D-3658 | Me(S) | single bond | H | *c*-Pr | C(=O)(*i*-Pr) | Cl | CH₂OMe | O |
| D-3659 | H | CH₂ | Me | *c*-Pr | C(=O)(*i*-Pr) | Cl | CH₂OMe | O |
| D-3660 | Me | CH₂ | H | *c*-Pr | C(=O)(*i*-Pr) | Cl | CH₂OMe | O |
| D-3661 | H | CH(Me) | H | *c*-Pr | C(=O)(*i*-Pr) | Cl | CH₂OMe | O |
| D-3662 | H | CH₂CH₂ | Me | *c*-Pr | C(=O)(*i*-Pr) | Cl | CH₂OMe | O |
| D-3663 | Me | CH₂CH₂ | H | *c*-Pr | C(=O)(*i*-Pr) | Cl | CH₂OMe | O |
| D-3664 | H | single bond | Me | *c*-Pr | C(=O)(*i*-Pr) | Me | CH₂OMe | O |
| D-3665 | Me | single bond | H | *c*-Pr | C(=O)(*i*-Pr) | Me | CH₂OMe | O |
| D-3666 | Me(R) | single bond | H | *c*-Pr | C(=O)(*i*-Pr) | Me | CH₂OMe | O |
| D-3667 | Me(S) | single bond | H | *c*-Pr | C(=O)(*i*-Pr) | Me | CH₂OMe | O |
| D-3668 | H | CH₂ | Me | *c*-Pr | C(=O)(*i*-Pr) | Me | CH₂OMe | O |
| D-3669 | Me | CH₂ | H | *c*-Pr | C(=O)(*i*-Pr) | Me | CH₂OMe | O |
| D-3670 | H | CH(Me) | H | *c*-Pr | C(=O)(*i*-Pr) | Me | CH₂OMe | O |
| D-3671 | H | CH₂CH₂ | Me | *c*-Pr | C(=O)(*i*-Pr) | Me | CH₂OMe | O |
| D-3672 | Me | CH₂CH₂ | H | *c*-Pr | C(=O)(*i*-Pr) | Me | CH₂OMe | O |
| D-3673 | H | single bond | Me | *c*-Pr | C(=O)(*i*-Pr) | Cl | CH₂CN | O |
| D-3674 | Me | single bond | H | *c*-Pr | C(=O)(*i*-Pr) | Cl | CH₂CN | O |
| D-3675 | Me(R) | single bond | H | *c*-Pr | C(=O)(*i*-Pr) | Cl | CH₂CN | O |
| D-3676 | Me(S) | single bond | H | *c*-Pr | C(=O)(*i*-Pr) | Cl | CH₂CN | O |
| D-3677 | H | CH₂ | Me | *c*-Pr | C(=O)(*i*-Pr) | Cl | CH₂CN | O |
| D-3678 | Me | CH₂ | H | *c*-Pr | C(=O)(*i*-Pr) | Cl | CH₂CN | O |
| D-3679 | H | CH(Me) | H | *c*-Pr | C(=O)(*i*-Pr) | Cl | CH₂CN | O |
| D-3680 | H | CH₂CH₂ | Me | *c-*Pr | C(=O)(*i*-Pr) | Cl | CH₂CN | O |
| D-3681 | Me | CH₂CH₂ | H | *c*-Pr | C(=O)(*i*-Pr) | Cl | CH₂CN | O |
| D-3682 | H | single bond | Me | *c*-Pr | C(=O)(*i*-Pr) | Me | CH₂CN | O |
| D-3683 | Me | single bond | H | *c*-Pr | C(=O)(*i*-Pr) | Me | CH₂CN | O |
| D-3684 | Me(R) | single bond | H | *c*-Pr | C(=O)(*i*-Pr) | Me | CH₂CN | O |
| D-3685 | Me(S) | single bond | H | *c*-Pr | C(=O)(*i*-Pr) | Me | CH₂CN | O |
| D-3686 | H | CH₂ | Me | *c*-Pr | C(=O)(*i*-Pr) | Me | CH₂CN | O |
| D-3687 | Me | CH₂ | H | *c*-Pr | C(=O)(*i*-Pr) | Me | CH₂CN | O |
| D-3688 | H | CH(Me) | H | *c*-Pr | C(=O)(*i*-Pr) | Me | CH₂CN | O |
| D-3689 | H | CH₂CH₂ | Me | *c*-Pr | C(=O)(*i*-Pr) | Me | CH₂CN | O |
| D-3690 | Me | CH₂CH₂ | H | *c*-Pr | C(=O)(*i* -Pr) | Me | CH₂CN | O |

**[Table 729]**

| Compound Number | Y | W | Z | R¹ | R² | R³ | R¹⁰ | A |
|---|---|---|---|---|---|---|---|---|
| D-3691 | H | single bond | Me | *c*-Pr | C(=O)(*i*-Pr) | Cl | C(=O)Me | O |
| D-3692 | Me | single bond | H | *c*-Pr | C(=O)(*i*-Pr) | Cl | C(=O)Me | O |
| D-3693 | Me(R) | single bond | H | *c*-Pr | C(=O)(*i*-Pr) | Cl | C(=O)Me | O |
| D-3694 | Me(S) | single bond | H | *c*-Pr | C(=O)(*i*-Pr) | Cl | C(=O)Me | O |
| D-3695 | H | CH₂ | Me | *c*-Pr | C(=O)(*i*-Pr) | Cl | C(=O)Me | O |
| D-3696 | Me | CH₂ | H | *c*-Pr | C(=O)(*i*-Pr) | Cl | C(=O)Me | O |
| D-3697 | H | CH(Me) | H | *c*-Pr | C(=O)(*i*-Pr) | Cl | C(=O)Me | O |
| D-3698 | H | CH₂CH₂ | Me | *c*-Pr | C(=O)(*i*-Pr) | Cl | C(=O)Me | O |
| D-3699 | Me | CH₂CH₂ | H | *c*-Pr | C(=O)(*i*-Pr) | Cl | C(=O)Me | O |
| D-3700 | H | single bond | Me | *c*-Pr | C(=O)(*i*-Pr) | Me | C(=O)Me | O |
| D-3701 | Me | single bond | H | *c*-Pr | C(=O)(*i*-Pr) | Me | C(=O)Me | O |
| D-3702 | Me(R) | single bond | H | *c*-Pr | C(=O)(*i*-Pr) | Me | C(=O)Me | O |
| D-3703 | Me(S) | single bond | H | *c*-Pr | C(=O)(*i*-Pr) | Me | C(=O)Me | O |
| D-3704 | H | CH₂ | Me | *c*-Pr | C(=O)(*i*-Pr) | Me | C(=O)Me | O |
| D-3705 | Me | CH₂ | H | *c*-Pr | C(=O)(*i*-Pr) | Me | C(=O)Me | O |
| D-3706 | H | CH(Me) | H | *c*-Pr | C(=O)(*i*-Pr) | Me | C(=O)Me | O |
| D-3707 | H | CH₂CH₂ | Me | *c*-Pr | C(=O)(*i*-Pr) | Me | C(=O)Me | O |
| D-3708 | Me | CH₂CH₂ | H | *c*-Pr | C(=O)(*i*-Pr) | Me | C(=O)Me | O |
| D-3709 | H | single bond | Me | *c*-Pr | C(=O)Ph | Cl | Me | O |
| D-3710 | Me | single bond | H | *c*-Pr | C(=O)Ph | Cl | Me | O |
| D-3711 | Me(R) | single bond | H | *c*-Pr | C(=O)Ph | Cl | Me | O |
| D-3712 | Me(S) | single bond | H | *c*-Pr | C(=O)Ph | Cl | Me | O |
| D-3713 | H | CH₂ | Me | *c*-Pr | C(=O)Ph | Cl | Me | O |
| D-3714 | Me | CH₂ | H | *c*-Pr | C(=O)Ph | Cl | Me | O |
| D-3715 | H | CH(Me) | H | *c*-Pr | C(=O)Ph | Cl | Me | O |
| D-3716 | H | CH₂CH₂ | Me | *c*-Pr | C(=O)Ph | Cl | Me | O |
| D-3717 | Me | CH₂CH₂ | H | *c*-Pr | C(=O)Ph | Cl | Me | O |
| D-3718 | H | single bond | Me | *c*-Pr | C(=O)Ph | Me | Me | O |
| D-3719 | Me | single bond | H | *c*-Pr | C(=O)Ph | Me | Me | O |
| D-3720 | Me(R) | single bond | H | *c*-Pr | C(=O)Ph | Me | Me | O |
| D-3721 | Me(S) | single bond | H | *c*-Pr | C(=O)Ph | Me | Me | O |
| D-3722 | H | CH₂ | Me | *c*-Pr | C(=O)Ph | Me | Me | O |
| D-3723 | Me | CH₂ | H | *c*-Pr | C(=O)Ph | Me | Me | O |
| D-3724 | H | CH(Me) | H | *c*-Pr | C(=O)Ph | Me | Me | O |
| D-3725 | H | CH₂CH₂ | Me | *c*-Pr | C(=O)Ph | Me | Me | O |
| D-3726 | Me | CH₂CH₂ | H | *c*-Pr | C(=O)Ph | Me | Me | O |
| D-3727 | H | single bond | Me | *c*-Pr | C(=O)Ph | Cl | Et | O |
| D-3728 | Me | single bond | H | *c*-Pr | C(=O)Ph | Cl | Et | O |
| D-3729 | Me(R) | single bond | H | *c*-Pr | C(=O)Ph | Cl | Et | O |
| D-3730 | Me(S) | single bond | H | *c*-Pr | C(=O)Ph | Cl | Et | O |
| D-3731 | H | CH₂ | Me | *c*-Pr | C(=O)Ph | Cl | Et | O |
| D-3732 | Me | CH₂ | H | *c*-Pr | C(=O)Ph | Cl | Et | O |
| D-3733 | H | CH(Me) | H | *c*-Pr | C(=O)Ph | Cl | Et | O |
| D-3734 | H | CH₂CH₂ | Me | *c*-Pr | C(=O)Ph | Cl | Et | O |

**[Table 730]**

| Compound Number | Y | W | Z | R¹ | R² | R³ | R¹⁰ | A |
|---|---|---|---|---|---|---|---|---|
| D-3735 | Me | CH₂CH₂ | H | *c*-Pr | C(=O)Ph | Cl | Et | O |
| D-3736 | H | single bond | Me | *c*-Pr | C(=O)Ph | Me | Et | O |
| D-3737 | Me | single bond | H | *c*-Pr | C(=O)Ph | Me | Et | O |
| D-3738 | Me(R) | single bond | H | *c*-Pr | C(=O)Ph | Me | Et | O |
| D-3739 | Me(S) | single bond | H | *c*-Pr | C(=O)Ph | Me | Et | O |
| D-3740 | H | CH₂ | Me | *c*-Pr | C(=O)Ph | Me | Et | O |
| D-3741 | Me | CH₂ | H | *c*-Pr | C(=O)Ph | Me | Et | O |
| D-3742 | H | CH(Me) | H | *c*-Pr | C(=O)Ph | Me | Et | O |
| D-3743 | H | CH₂CH₂ | Me | *c*-Pr | C(=O)Ph | Me | Et | O |
| D-3744 | Me | CH₂CH₂ | H | *c*-Pr | C(=O)Ph | Me | Et | O |
| D-3745 | H | single bond | Me | *c*-Pr | C(=O)Ph | Cl | CH₂CF₃ | O |
| D-3746 | Me | single bond | H | *c*-Pr | C(=O)Ph | Cl | CH₂CF₃ | O |
| D-3747 | Me(R) | single bond | H | *c*-Pr | C(=O)Ph | Cl | CH₂CF₃ | O |
| D-3748 | Me(S) | single bond | H | *c*-Pr | C(=O)Ph | Cl | CH₂CF₃ | O |
| D-3749 | H | CH₂ | Me | *c*-Pr | C(=O)Ph | Cl | CH₂CF₃ | O |
| D-3750 | Me | CH₂ | H | *c*-Pr | C(=O)Ph | Cl | CH₂CF₃ | O |
| D-3751 | H | CH(Me) | H | *c*-Pr | C(=O)Ph | Cl | CH₂CF₃ | O |
| D-3752 | H | CH₂CH₂ | Me | *c*-Pr | C(=O)Ph | Cl | CH₂CF₃ | O |
| D-3753 | Me | CH₂CH₂ | H | *c*-Pr | C(=O)Ph | Cl | CH₂CF₃ | O |
| D-3754 | H | single bond | Me | *c*-Pr | C(=O)Ph | Me | CH₂CF₃ | O |
| D-3755 | Me | single bond | H | *c*-Pr | C(=O)Ph | Me | CH₂CF₃ | O |
| D-3756 | Me(R) | single bond | H | *c*-Pr | C(=O)Ph | Me | CH₂CF₃ | O |
| D-3757 | Me(S) | single bond | H | *c*-Pr | C(=O)Ph | Me | CH₂CF₃ | O |
| D-3758 | H | CH₂ | Me | *c*-Pr | C(=O)Ph | Me | CH₂CF₃ | O |
| D-3759 | Me | CH₂ | H | *c*-Pr | C(=O)Ph | Me | CH₂CF₃ | O |
| D-3760 | H | CH(Me) | H | *c*-Pr | C(=O)Ph | Me | CH₂CF₃ | O |
| D-3761 | H | CH₂CH₂ | Me | *c*-Pr | C(=O)Ph | Me | CH₂CF₃ | O |
| D-3762 | Me | CH₂CH₂ | H | *c*-Pr | C(=O)Ph | Me | CH₂CF₃ | O |
| D-3763 | H | single bond | Me | *c*-Pr | C(=O)Ph | Cl | CH₂OMe | O |
| D-3764 | Me | single bond | H | *c*-Pr | C(=O)Ph | Cl | CH₂OMe | O |
| D-3765 | Me(R) | single bond | H | *c*-Pr | C(=O)Ph | Cl | CH₂OMe | O |
| D-3766 | Me(S) | single bond | H | *c*-Pr | C(=O)Ph | Cl | CH₂OMe | O |
| D-3767 | H | CH₂ | Me | *c*-Pr | C(=O)Ph | Cl | CH₂OMe | O |
| D-3768 | Me | CH₂ | H | *c*-Pr | C(=O)Ph | Cl | CH₂OMe | O |
| D-3769 | H | CH(Me) | H | *c*-Pr | C(=O)Ph | Cl | CH₂OMe | O |
| D-3770 | H | CH₂CH₂ | Me | *c*-Pr | C(=O)Ph | Cl | CH₂OMe | O |
| D-3771 | Me | CH₂CH₂ | H | *c*-Pr | C(=O)Ph | Cl | CH₂OMe | O |
| D-3772 | H | single bond | Me | *c*-Pr | C(=O)Ph | Me | CH₂OMe | O |
| D-3773 | Me | single bond | H | *c*-Pr | C(=O)Ph | Me | CH₂OMe | O |
| D-3774 | Me(R) | single bond | H | *c*-Pr | C(=O)Ph | Me | CH₂OMe | O |
| D-3775 | Me(S) | single bond | H | *c*-Pr | C(=O)Ph | Me | CH₂OMe | O |
| D-3776 | H | CH₂ | Me | *c*-Pr | C(=O)Ph | Me | CH₂OMe | O |
| D-3777 | Me | CH₂ | H | *c*-Pr | C(=O)Ph | Me | CH₂OMe | O |
| D-3778 | H | CH(Me) | H | *c*-Pr | C(=O)Ph | Me | CH₂OMe | O |

**[Table 731]**

| Compound Number | Y | W | Z | R¹ | R² | R³ | R¹⁰ | A |
|---|---|---|---|---|---|---|---|---|
| D-3779 | H | CH₂CH₂ | Me | *c*-Pr | C(=O)Ph | Me | CH₂OMe | O |
| D-3780 | Me | CH₂CH₂ | H | *c*-Pr | C(=O)Ph | Me | CH₂OMe | O |
| D-3781 | H | single bond | Me | *c*-Pr | C(=O)Ph | Cl | CH₂CN | O |
| D-3782 | Me | single bond | H | *c*-Pr | C(=O)Ph | Cl | CH₂CN | O |
| D-3783 | Me(R) | single bond | H | *c*-Pr | C(=O)Ph | Cl | CH₂CN | O |
| D-3784 | Me(S) | single bond | H | *c*-Pr | C(=O)Ph | Cl | CH₂CN | O |
| D-3785 | H | CH₂ | Me | *c*-Pr | C(=O)Ph | Cl | CH₂CN | O |
| D-3786 | Me | CH₂ | H | *c*-Pr | C(=O)Ph | Cl | CH₂CN | O |
| D-3787 | H | CH(Me) | H | *c*-Pr | C(=O)Ph | Cl | CH₂CN | O |
| D-3788 | H | CH₂CH₂ | Me | *c*-Pr | C(=O)Ph | Cl | CH₂CN | O |
| D-3789 | Me | CH₂CH₂ | H | *c*-Pr | C(=O)Ph | Cl | CH₂CN | O |
| D-3790 | H | single bond | Me | *c*-Pr | C(=O)Ph | Me | CH₂CN | O |
| D-3791 | Me | single bond | H | *c*-Pr | C(=O)Ph | Me | CH₂CN | O |
| D-3792 | Me(R) | single bond | H | *c*-Pr | C(=O)Ph | Me | CH₂CN | O |
| D-3793 | Me(S) | single bond | H | *c*-Pr | C(=O)Ph | Me | CH₂CN | O |
| D-3794 | H | CH₂ | Me | *c*-Pr | C(=O)Ph | Me | CH₂CN | O |
| D-3795 | Me | CH₂ | H | *c*-Pr | C(=O)Ph | Me | CH₂CN | O |
| D-3796 | H | CH(Me) | H | *c*-Pr | C(=O)Ph | Me | CH₂CN | O |
| D-3797 | H | CH₂CH₂ | Me | *c*-Pr | C(=O)Ph | Me | CH₂CN | O |
| D-3798 | Me | CH₂CH₂ | H | *c*-Pr | C(=O)Ph | Me | CH₂CN | O |
| D-3799 | H | single bond | Me | *c*-Pr | C(=O)Ph | Cl | C(=O)Me | O |
| D-3800 | Me | single bond | H | *c*-Pr | C(=O)Ph | Cl | C(=O)Me | O |
| D-3801 | Me(R) | single bond | H | *c*-Pr | C(=O)Ph | Cl | C(=O)Me | O |
| D-3802 | Me(S) | single bond | H | *c*-Pr | C(=O)Ph | Cl | C(=O)Me | O |
| D-3803 | H | CH₂ | Me | *c*-Pr | C(=O)Ph | Cl | C(=O)Me | O |
| D-3804 | Me | CH₂ | H | *c*-Pr | C(=O)Ph | Cl | C(=O)Me | O |
| D-3805 | H | CH(Me) | H | *c*-Pr | C(=O)Ph | Cl | C(=O)Me | O |
| D-3806 | H | CH₂CH₂ | Me | *c*-Pr | C(=O)Ph | Cl | C(=O)Me | O |
| D-3807 | Me | CH₂CH₂ | H | *c*-Pr | C(=O)Ph | Cl | C(=O)Me | O |
| D-3808 | H | single bond | Me | *c*-Pr | C(=O)Ph | Me | C(=O)Me | O |
| D-3809 | Me | single bond | H | *c*-Pr | C(=O)Ph | Me | C(=O)Me | O |
| D-3810 | Me(R) | single bond | H | *c*-Pr | C(=O)Ph | Me | C(=O)Me | O |
| D-3811 | Me(S) | single bond | H | *c*-Pr | C(=O)Ph | Me | C(=O)Me | O |
| D-3812 | H | CH₂ | Me | *c*-Pr | C(=O)Ph | Me | C(=O)Me | O |
| D-3813 | Me | CH₂ | H | *c*-Pr | C(=O)Ph | Me | C(=O)Me | O |
| D-3814 | H | CH(Me) | H | *c*-Pr | C(=O)Ph | Me | C(=O)Me | O |
| D-3815 | H | CH₂CH₂ | Me | *c*-Pr | C(=O)Ph | Me | C(=O)Me | O |
| D-3816 | Me | CH₂CH₂ | H | c-Pr | C(=O)Ph | Me | C(=O)Me | O |
| D-3817 | H | single bond | Me | *c*-Pr | C(=O)OEt | Cl | Me | O |
| D-3818 | Me | single bond | H | *c*-Pr | C(=O)OEt | Cl | Me | O |
| D-3819 | Me(R) | single bond | H | *c*-Pr | C(=O)OEt | Cl | Me | O |
| D-3820 | Me(S) | single bond | H | *c*-Pr | C(=O)OEt | Cl | Me | O |
| D-3821 | H | CH₂ | Me | *c*-Pr | C(=O)OEt | Cl | Me | O |
| D-3822 | Me | CH₂ | H | *c*-Pr | C(=O)OEt | Cl | Me | O |

**[Table 732]**

| Compound Number | Y | W | Z | R¹ | R² | R³ | R¹⁰ | A |
|---|---|---|---|---|---|---|---|---|
| D-3823 | H | CH(Me) | H | *c*-Pr | C(=O)OEt | Cl | Me | O |
| D-3824 | H | CH₂CH₂ | Me | *c*-Pr | C(=O)OEt | Cl | Me | O |
| D-3825 | Me | CH₂CH₂ | H | *c*-Pr | C(=O)OEt | Cl | Me | O |
| D-3826 | H | single bond | Me | *c*-Pr | C(=O)OEt | Me | Me | O |
| D-3827 | Me | single bond | H | *c*-Pr | C(=O)OEt | Me | Me | O |
| D-3828 | Me(R) | single bond | H | *c*-Pr | C(=O)OEt | Me | Me | O |
| D-3829 | Me(S) | single bond | H | *c*-Pr | C(=O)OEt | Me | Me | O |
| D-3830 | H | CH₂ | Me | *c*-Pr | C(=O)OEt | Me | Me | O |
| D-3831 | Me | CH₂ | H | *c*-Pr | C(=O)OEt | Me | Me | O |
| D-3832 | H | CH(Me) | H | *c*-Pr | C(=O)OEt | Me | Me | O |
| D-3833 | H | CH₂CH₂ | Me | *c*-Pr | C(=O)OEt | Me | Me | O |
| D-3834 | Me | CH₂CH₂ | H | *c*-Pr | C(=O)OEt | Me | Me | O |
| D-3835 | H | single bond | Me | *c*-Pr | C(=O)OEt | Cl | Et | O |
| D-3836 | Me | single bond | H | *c*-Pr | C(=O)OEt | Cl | Et | O |
| D-3837 | Me(R) | single bond | H | *c*-Pr | C(=O)OEt | Cl | Et | O |
| D-3838 | Me(S) | single bond | H | *c*-Pr | C(=O)OEt | Cl | Et | O |
| D-3839 | H | CH₂ | Me | *c*-Pr | C(=O)OEt | Cl | Et | O |
| D-3840 | Me | CH₂ | H | *c*-Pr | C(=O)OEt | Cl | Et | O |
| D-3841 | H | CH(Me) | H | *c*-Pr | C(=O)OEt | Cl | Et | O |
| D-3842 | H | CH₂CH₂ | Me | *c*-Pr | C(=O)OEt | Cl | Et | O |
| D-3843 | Me | CH₂CH₂ | H | *c*-Pr | C(=O)OEt | Cl | Et | O |
| D-3844 | H | single bond | Me | *c*-Pr | C(=O)OEt | Me | Et | O |
| D-3845 | Me | single bond | H | *c*-Pr | C(=O)OEt | Me | Et | O |
| D-3846 | Me(R) | single bond | H | *c*-Pr | C(=O)OEt | Me | Et | O |
| D-3847 | Me(S) | single bond | H | *c*-Pr | C(=O)OEt | Me | Et | O |
| D-3848 | H | CH₂ | Me | *c*-Pr | C(=O)OEt | Me | Et | O |
| D-3849 | Me | CH₂ | H | *c*-Pr | C(=O)OEt | Me | Et | O |
| D-3850 | H | CH(Me) | H | *c*-Pr | C(=O)OEt | Me | Et | O |
| D-3851 | H | CH₂CH₂ | Me | *c*-Pr | C(=O)OEt | Me | Et | O |
| D-3852 | Me | CH₂CH₂ | H | *c*-Pr | C(=O)OEt | Me | Et | O |
| D-3853 | H | single bond | Me | *c*-Pr | C(=O)OEt | Cl | CH₂CF₃ | O |
| D-3854 | Me | single bond | H | *c*-Pr | C(=O)OEt | Cl | CH₂CF₃ | O |
| D-3855 | Me(R) | single bond | H | *c*-Pr | C(=O)OEt | Cl | CH₂CF₃ | O |
| D-3856 | Me(S) | single bond | H | *c*-Pr | C(=O)OEt | Cl | CH₂CF₃ | O |
| D-3857 | H | CH₂ | Me | *c*-Pr | C(=O)OEt | Cl | CH₂CF₃ | O |
| D-3858 | Me | CH₂ | H | *c*-Pr | C(=O)OEt | Cl | CH₂CF₃ | O |
| D-3859 | H | CH(Me) | H | *c*-Pr | C(=O)OEt | Cl | CH₂CF₃ | O |
| D-3860 | H | CH₂CH₂ | Me | c-Pr | C(=O)OEt | Cl | CH₂CF₃ | O |
| D-3861 | Me | CH₂CH₂ | H | *c*-Pr | C(=O)OEt | Cl | CH₂CF₃ | O |
| D-3862 | H | single bond | Me | *c*-Pr | C(=O)OEt | Me | CH₂CF₃ | O |
| D-3863 | Me | single bond | H | *c*-Pr | C(=O)OEt | Me | CH₂CF₃ | O |
| D-3864 | Me(R) | single bond | H | *c*-Pr | C(=O)OEt | Me | CH₂CF₃ | O |
| D-3865 | Me(S) | single bond | H | *c*-Pr | C(=O)OEt | Me | CH₂CF₃ | O |
| D-3866 | H | CH₂ | Me | *c*-Pr | C(=O)OEt | Me | CH₂CF₃ | O |

**[Table 733]**

| Compound Number | Y | W | Z | R¹ | R² | R³ | R¹⁰ | A |
|---|---|---|---|---|---|---|---|---|
| D-3867 | Me | CH₂ | H | *c*-Pr | C(=O)OEt | Me | CH₂CF₃ | O |
| D-3868 | H | CH(Me) | H | *c*-Pr | C(=O)OEt | Me | CH₂CF₃ | O |
| D-3869 | H | CH₂CH₂ | Me | *c*-Pr | C(=O)OEt | Me | CH₂CF₃ | O |
| D-3870 | Me | CH₂CH₂ | H | *c*-Pr | C(=O)OEt | Me | CH₂CF₃ | O |
| D-3871 | H | single bond | Me | *c*-Pr | C(=O)OEt | Cl | CH₂OMe | O |
| D-3872 | Me | single bond | H | *c*-Pr | C(=O)OEt | Cl | CH₂OMe | O |
| D-3873 | Me(R) | single bond | H | *c*-Pr | C(=O)OEt | Cl | CH₂OMe | O |
| D-3874 | Me(S) | single bond | H | *c*-Pr | C(=O)OEt | Cl | CH₂OMe | O |
| D-3875 | H | CH₂ | Me | *c*-Pr | C(=O)OEt | Cl | CH₂OMe | O |
| D-3876 | Me | CH₂ | H | *c*-Pr | C(=O)OEt | Cl | CH₂OMe | O |
| D-3877 | H | CH(Me) | H | *c*-Pr | C(=O)OEt | Cl | CH₂OMe | O |
| D-3878 | H | CH₂CH₂ | Me | *c*-Pr | C(=O)OEt | Cl | CH₂OMe | O |
| D-3879 | Me | CH₂CH₂ | H | *c*-Pr | C(=O)OEt | Cl | CH₂OMe | O |
| D-3880 | H | single bond | Me | *c*-Pr | C(=O)OEt | Me | CH₂OMe | O |
| D-3881 | Me | single bond | H | *c*-Pr | C(=O)OEt | Me | CH₂OMe | O |
| D-3882 | Me(R) | single bond | H | *c*-Pr | C(=O)OEt | Me | CH₂OMe | O |
| D-3883 | Me(S) | single bond | H | *c*-Pr | C(=O)OEt | Me | CH₂OMe | O |
| D-3884 | H | CH₂ | Me | c-Pr | C(=O)OEt | Me | CH₂OMe | O |
| D-3885 | Me | CH₂ | H | *c*-Pr | C(=O)OEt | Me | CH₂OMe | O |
| D-3886 | H | CH(Me) | H | *c*-Pr | C(=O)OEt | Me | CH₂OMe | O |
| D-3887 | H | CH₂CH₂ | Me | *c*-Pr | C(=O)OEt | Me | CH₂OMe | O |
| D-3888 | Me | CH₂CH₂ | H | *c*-Pr | C(=O)OEt | Me | CH₂OMe | O |
| D-3889 | H | single bond | Me | *c*-Pr | C(=O)OEt | Cl | CH₂CN | O |
| D-3890 | Me | single bond | H | *c*-Pr | C(=O)OEt | Cl | CH₂CN | O |
| D-3891 | Me(R) | single bond | H | *c*-Pr | C(=O)OEt | Cl | CH₂CN | O |
| D-3892 | Me(S) | single bond | H | *c*-Pr | C(=O)OEt | Cl | CH₂CN | O |
| D-3893 | H | CH₂ | Me | *c*-Pr | C(=O)OEt | Cl | CH₂CN | O |
| D-3894 | Me | CH₂ | H | *c*-Pr | C(=O)OEt | Cl | CH₂CN | O |
| D-3895 | H | CH(Me) | H | *c*-Pr | C(=O)OEt | Cl | CH₂CN | O |
| D-3896 | H | CH₂CH₂ | Me | *c*-Pr | C(=O)OEt | Cl | CH₂CN | O |
| D-3897 | Me | CH₂CH₂ | H | *c*-Pr | C(=O)OEt | Cl | CH₂CN | O |
| D-3898 | H | single bond | Me | *c*-Pr | C(=O)OEt | Me | CH₂CN | O |
| D-3899 | Me | single bond | H | *c*-Pr | C(=O)OEt | Me | CH₂CN | O |
| D-3900 | Me(R) | single bond | H | *c*-Pr | C(=O)OEt | Me | CH₂CN | O |
| D-3901 | Me(S) | single bond | H | *c*-Pr | C(=O)OEt | Me | CH₂CN | O |
| D-3902 | H | CH₂ | Me | *c*-Pr | C(=O)OEt | Me | CH₂CN | O |
| D-3903 | Me | CH₂ | H | *c*-Pr | C(=O)OEt | Me | CH₂CN | O |
| D-3904 | H | CH(Me) | H | c-Pr | C(=O)OEt | Me | CH₂CN | O |
| D-3905 | H | CH₂CH₂ | Me | *c*-Pr | C(=O)OEt | Me | CH₂CN | O |
| D-3906 | Me | CH₂CH₂ | H | *c*-Pr | C(=O)OEt | Me | CH₂CN | O |
| D-3907 | H | single bond | Me | *c*-Pr | C(=O)OEt | Cl | C(=O)Me | O |
| D-3908 | Me | single bond | H | *c*-Pr | C(=O)OEt | Cl | C(=O)Me | O |
| D-3909 | Me(R) | single bond | H | *c*-Pr | C(=O)OEt | Cl | C(=O)Me | O |
| D-3910 | Me(S) | single bond | H | *c*-Pr | C(=O)OEt | Cl | C(=O)Me | O |

**[Table 734]**

| Compound Number | Y | W | Z | R¹ | R² | R³ | R¹⁰ | A |
|---|---|---|---|---|---|---|---|---|
| D-3911 | H | CH₂ | Me | *c*-Pr | C(=O)OEt | Cl | C(=O)Me | O |
| D-3912 | Me | CH₂ | H | *c*-Pr | C(=O)OEt | Cl | C(=O)Me | O |
| D-3913 | H | CH(Me) | H | *c*-Pr | C(=O)OEt | Cl | C(=O)Me | O |
| D-3914 | H | CH₂CH₂ | Me | *c*-Pr | C(=O)OEt | Cl | C(=O)Me | O |
| D-3915 | Me | CH₂CH₂ | H | *c*-Pr | C(=O)OEt | Cl | C(=O)Me | O |
| D-3916 | H | single bond | Me | *c*-Pr | C(=O)OEt | Me | C(=O)Me | O |
| D-3917 | Me | single bond | H | *c*-Pr | C(=O)OEt | Me | C(=O)Me | O |
| D-3918 | Me(R) | single bond | H | *c*-Pr | C(=O)OEt | Me | C(=O)Me | O |
| D-3919 | Me(S) | single bond | H | *c*-Pr | C(=O)OEt | Me | C(=O)Me | O |
| D-3920 | H | CH₂ | Me | *c*-Pr | C(=O)OEt | Me | C(=O)Me | O |
| D-3921 | Me | CH₂ | H | *c*-Pr | C(=O)OEt | Me | C(=O)Me | O |
| D-3922 | H | CH(Me) | H | *c*-Pr | C(=O)OEt | Me | C(=O)Me | O |
| D-3923 | H | CH₂CH₂ | Me | *c*-Pr | C(=O)OEt | Me | C(=O)Me | O |
| D-3924 | Me | CH₂CH₂ | H | *c*-Pr | C(=O)OEt | Me | C(=O)Me | O |
| D-3925 | H | single bond | Me | *c*-Pr | S(=O)₂Me | Cl | Me | O |
| D-3926 | Me | single bond | H | *c*-Pr | S(=O)₂Me | Cl | Me | O |
| D-3927 | Me(R) | single bond | H | *c*-Pr | S(=O)₂Me | Cl | Me | O |
| D-3928 | Me(S) | single bond | H | c-Pr | S(=O)₂Me | Cl | Me | O |
| D-3929 | H | CH₂ | Me | *c*-Pr | S(=O)₂Me | Cl | Me | O |
| D-3930 | Me | CH₂ | H | *c*-Pr | S(=O)₂Me | Cl | Me | O |
| D-3931 | H | CH(Me) | H | *c*-Pr | S(=O)₂Me | Cl | Me | O |
| D-3932 | H | CH₂CH₂ | Me | *c*-Pr | S(=O)₂Me | Cl | Me | O |
| D-3933 | Me | CH₂CH₂ | H | *c*-Pr | S(=O)₂Me | Cl | Me | O |
| D-3934 | H | single bond | Me | *c*-Pr | S(=O)₂Me | Me | Me | O |
| D-3935 | Me | single bond | H | *c*-Pr | S(=O)₂Me | Me | Me | O |
| D-3936 | Me(R) | single bond | H | *c*-Pr | S(=O)₂Me | Me | Me | O |
| D-3937 | Me(S) | single bond | H | *c*-Pr | S(=O)₂Me | Me | Me | O |
| D-3938 | H | CH₂ | Me | *c*-Pr | S(=O)₂Me | Me | Me | O |
| D-3939 | Me | CH₂ | H | *c*-Pr | S(=O)₂Me | Me | Me | O |
| D-3940 | H | CH(Me) | H | *c*-Pr | S(=O)₂Me | Me | Me | O |
| D-3941 | H | CH₂CH₂ | Me | *c*-Pr | S(=O)₂Me | Me | Me | O |
| D-3942 | Me | CH₂CH₂ | H | *c*-Pr | S(=O)₂Me | Me | Me | O |
| D-3943 | H | single bond | Me | *c*-Pr | S(=O)₂Me | Cl | Et | O |
| D-3944 | Me | single bond | H | *c*-Pr | S(=O)₂Me | Cl | Et | O |
| D-3945 | Me(R) | single bond | H | *c*-Pr | S(=O)₂Me | Cl | Et | O |
| D-3946 | Me(S) | single bond | H | *c*-Pr | S(=O)₂Me | Cl | Et | O |
| D-3947 | H | CH₂ | Me | *c*-Pr | S(=O)₂Me | Cl | Et | O |
| D-3948 | Me | CH₂ | H | *c*-Pr | S(=O)₂Me | Cl | Et | O |
| D-3949 | H | CH(Me) | H | *c*-Pr | S(=O)₂Me | Cl | Et | O |
| D-3950 | H | CH₂CH₂ | Me | *c*-Pr | S(=O)₂Me | Cl | Et | O |
| D-3951 | Me | CH₂CH₂ | H | *c*-Pr | S(=O)₂Me | Cl | Et | O |
| D-3952 | H | single bond | Me | *c*-Pr | S(=O)₂Me | Me | Et | O |
| D-3953 | Me | single bond | H | *c*-Pr | S(=O)₂Me | Me | Et | O |
| D-3954 | Me(R) | single bond | H | *c*-Pr | S(=O)₂Me | Me | Et | O |

**[Table 735]**

| Compound Number | Y | W | Z | R¹ | R² | R³ | R¹⁰ | A |
|---|---|---|---|---|---|---|---|---|
| D-3955 | Me(S) | single bond | H | *c*-Pr | S(=O)₂Me | Me | Et | O |
| D-3956 | H | CH₂ | Me | *c*-Pr | S(=O)₂Me | Me | Et | O |
| D-3957 | Me | CH₂ | H | *c*-Pr | S(=O)₂Me | Me | Et | O |
| D-3958 | H | CH(Me) | H | *c*-Pr | S(=O)₂Me | Me | Et | O |
| D-3959 | H | CH₂CH₂ | Me | *c*-Pr | S(=O)₂Me | Me | Et | O |
| D-3960 | Me | CH₂CH₂ | H | *c*-Pr | S(=O)₂Me | Me | Et | O |
| D-3961 | H | single bond | Me | *c*-Pr | S(=O)₂Me | Cl | CH₂CF₃ | O |
| D-3962 | Me | single bond | H | *c*-Pr | S(=O)₂Me | Cl | CH₂CF₃ | O |
| D-3963 | Me(R) | single bond | H | *c*-Pr | S(=O)₂Me | Cl | CH₂CF₃ | O |
| D-3964 | Me(S) | single bond | H | *c*-Pr | S(=O)₂Me | Cl | CH₂CF₃ | O |
| D-3965 | H | CH₂ | Me | *c*-Pr | S(=O)₂Me | Cl | CH₂CF₃ | O |
| D-3966 | Me | CH₂ | H | *c*-Pr | S(=O)₂Me | Cl | CH₂CF₃ | O |
| D-3967 | H | CH(Me) | H | *c*-Pr | S(=O)₂Me | Cl | CH₂CF₃ | O |
| D-3968 | H | CH₂CH₂ | Me | *c*-Pr | S(=O)₂Me | Cl | CH₂CF₃ | O |
| D-3969 | Me | CH₂CH₂ | H | *c*-Pr | S(=O)₂Me | Cl | CH₂CF₃ | O |
| D-3970 | H | single bond | Me | *c*-Pr | S(=O)₂Me | Me | CH₂CF₃ | O |
| D-3971 | Me | single bond | H | *c*-Pr | S(=O)₂Me | Me | CH₂CF₃ | O |
| D-3972 | Me(R) | single bond | H | c-Pr | S(=O)₂Me | Me | CH₂CF₃ | O |
| D-3973 | Me(S) | single bond | H | *c*-Pr | S(=O)₂Me | Me | CH₂CF₃ | O |
| D-3974 | H | CH₂ | Me | *c*-Pr | S(=O)₂Me | Me | CH₂CF₃ | O |
| D-3975 | Me | CH₂ | H | *c*-Pr | S(=O)₂Me | Me | CH₂CF₃ | O |
| D-3976 | H | CH(Me) | H | *c*-Pr | S(=O)₂Me | Me | CH₂CF₃ | O |
| D-3977 | H | CH₂CH₂ | Me | *c*-Pr | S(=O)₂Me | Me | CH₂CF₃ | O |
| D-3978 | Me | CH₂CH₂ | H | *c*-Pr | S(=O)₂Me | Me | CH₂CF₃ | O |
| D-3979 | H | single bond | Me | *c*-Pr | S(=O)₂Me | Cl | CH₂OMe | O |
| D-3980 | Me | single bond | H | *c*-Pr | S(=O)₂Me | Cl | CH₂OMe | O |
| D-3981 | Me(R) | single bond | H | *c*-Pr | S(=O)₂Me | Cl | CH₂OMe | O |
| D-3982 | Me(S) | single bond | H | *c*-Pr | S(=O)₂Me | Cl | CH₂OMe | O |
| D-3983 | H | CH₂ | Me | *c*-Pr | S(=O)₂Me | Cl | CH₂OMe | O |
| D-3984 | Me | CH₂ | H | *c*-Pr | S(=O)₂Me | Cl | CH₂OMe | O |
| D-3985 | H | CH(Me) | H | *c*-Pr | S(=O)₂Me | Cl | CH₂OMe | O |
| D-3986 | H | CH₂CH₂ | Me | *c*-Pr | S(=O)₂Me | Cl | CH₂OMe | O |
| D-3987 | Me | CH₂CH₂ | H | *c*-Pr | S(=O)₂Me | Cl | CH₂OMe | O |
| D-3988 | H | single bond | Me | *c*-Pr | S(=O)₂Me | Me | CH₂OMe | O |
| D-3989 | Me | single bond | H | *c*-Pr | S(=O)₂Me | Me | CH₂OMe | O |
| D-3990 | Me(R) | single bond | H | *c*-Pr | S(=O)₂Me | Me | CH₂OMe | O |
| D-3991 | Me(S) | single bond | H | *c*-Pr | S(=O)₂Me | Me | CH₂OMe | O |
| D-3992 | H | CH₂ | Me | c-Pr | S(=O)₂Me | Me | CH₂OMe | O |
| D-3993 | Me | CH₂ | H | *c*-Pr | S(=O)₂Me | Me | CH₂OMe | O |
| D-3994 | H | CH(Me) | H | *c*-Pr | S(=O)₂Me | Me | CH₂OMe | O |
| D-3995 | H | CH₂CH₂ | Me | *c*-Pr | S(=O)₂Me | Me | CH₂OMe | O |
| D-3996 | Me | CH₂CH₂ | H | *c*-Pr | S(=O)₂Me | Me | CH₂OMe | O |
| D-3997 | H | single bond | Me | *c*-Pr | S(=O)₂Me | Cl | CH₂CN | O |
| D-3998 | Me | single bond | H | *c*-Pr | S(=O)₂Me | Cl | CH₂CN | O |

**[Table 736]**

| Compound Number | Y | W | Z | R¹ | R² | R³ | R¹⁰ | A |
|---|---|---|---|---|---|---|---|---|
| D-3999 | Me(R) | single bond | H | *c*-Pr | S(=O)₂Me | Cl | CH₂CN | O |
| D-4000 | Me(S) | single bond | H | *c*-Pr | S(=O)₂Me | Cl | CH₂CN | O |
| D-4001 | H | CH₂ | Me | *c*-Pr | S(=O)₂Me | Cl | CH₂CN | O |
| D-4002 | Me | CH₂ | H | *c*-Pr | S(=O)₂Me | Cl | CH₂CN | O |
| D-4003 | H | CH(Me) | H | *c*-Pr | S(=O)₂Me | Cl | CH₂CN | O |
| D-4004 | H | CH₂CH₂ | Me | *c*-Pr | S(=O)₂Me | Cl | CH₂CN | O |
| D-4005 | Me | CH₂CH₂ | H | *c*-Pr | S(=O)₂Me | Cl | CH₂CN | O |
| D-4006 | H | single bond | Me | *c*-Pr | S(=O)₂Me | Me | CH₂CN | O |
| D-4007 | Me | single bond | H | *c*-Pr | S(=O)₂Me | Me | CH₂CN | O |
| D-4008 | Me(R) | single bond | H | *c*-Pr | S(=O)₂Me | Me | CH₂CN | O |
| D-4009 | Me(S) | single bond | H | *c*-Pr | S(=O)₂Me | Me | CH₂CN | O |
| D-4010 | H | CH₂ | Me | *c*-Pr | S(=O)₂Me | Me | CH₂CN | O |
| D-4011 | Me | CH₂ | H | *c*-Pr | S(=O)₂Me | Me | CH₂CN | O |
| D-4012 | H | CH(Me) | H | *c*-Pr | S(=O)₂Me | Me | CH₂CN | O |
| D-4013 | H | CH₂CH₂ | Me | *c*-Pr | S(=O)₂Me | Me | CH₂CN | O |
| D-4014 | Me | CH₂CH₂ | H | *c*-Pr | S(=O)₂Me | Me | CH₂CN | O |
| D-4015 | H | single bond | Me | *c*-Pr | S(=O)₂Me | Cl | C(=O)Me | O |
| D-4016 | Me | single bond | H | c-Pr | S(=O)₂Me | Cl | C(=O)Me | O |
| D-4017 | Me(R) | single bond | H | *c*-Pr | S(=O)₂Me | Cl | C(=O)Me | O |
| D-4018 | Me(S) | single bond | H | *c*-Pr | S(=O)₂Me | Cl | C(=O)Me | O |
| D-4019 | H | CH₂ | Me | *c*-Pr | S(=O)₂Me | Cl | C(=O)Me | O |
| D-4020 | Me | CH₂ | H | *c*-Pr | S(=O)₂Me | Cl | C(=O)Me | O |
| D-4021 | H | CH(Me) | H | *c*-Pr | S(=O)₂Me | Cl | C(=O)Me | O |
| D-4022 | H | CH₂CH₂ | Me | *c*-Pr | S(=O)₂Me | Cl | C(=O)Me | O |
| D-4023 | Me | CH₂CH₂ | H | *c*-Pr | S(=O)₂Me | Cl | C(=O)Me | O |
| D-4024 | H | single bond | Me | *c*-Pr | S(=O)₂Me | Me | C(=O)Me | O |
| D-4025 | Me | single bond | H | *c*-Pr | S(=O)₂Me | Me | C(=O)Me | O |
| D-4026 | Me(R) | single bond | H | *c*-Pr | S(=O)₂Me | Me | C(=O)Me | O |
| D-4027 | Me(S) | single bond | H | *c*-Pr | S(=O)₂Me | Me | C(=O)Me | O |
| D-4028 | H | CH₂ | Me | *c*-Pr | S(=O)₂Me | Me | C(=O)Me | O |
| D-4029 | Me | CH₂ | H | *c*-Pr | S(=O)₂Me | Me | C(=O)Me | O |
| D-4030 | H | CH(Me) | H | *c*-Pr | S(=O)₂Me | Me | C(=O)Me | O |
| D-4031 | H | CH₂CH₂ | Me | *c*-Pr | S(=O)₂Me | Me | C(=O)Me | O |
| D-4032 | Me | CH₂CH₂ | H | *c*-Pr | S(=O)₂Me | Me | C(=O)Me | O |
| D-4033 | H | single bond | Me | *c*-Pr | S(=O)₂CF₃ | Cl | Me | O |
| D-4034 | Me | single bond | H | *c*-Pr | S(=O)₂CF₃ | Cl | Me | O |
| D-4035 | Me(R) | single bond | H | *c*-Pr | S(=O)₂CF₃ | Cl | Me | O |
| D-4036 | Me(S) | single bond | H | c-Pr | S(=O)₂CF₃ | Cl | Me | O |
| D-4037 | H | CH₂ | Me | *c*-Pr | S(=O)₂CF₃ | Cl | Me | O |
| D-4038 | Me | CH₂ | H | *c*-Pr | S(=O)₂CF₃ | Cl | Me | O |
| D-4039 | H | CH(Me) | H | *c*-Pr | S(=O)₂CF₃ | Cl | Me | O |
| D-4040 | H | CH₂CH₂ | Me | *c*-Pr | S(=O)₂CF₃ | Cl | Me | O |
| D-4041 | Me | CH₂CH₂ | H | *c*-Pr | S(=O)₂CF₃ | Cl | Me | O |
| D-4042 | H | single bond | Me | *c*-Pr | S(=O)₂CF₃ | Me | Me | O |

**[Table 737]**

| Compound Number | Y | W | Z | R¹ | R² | R³ | R¹⁰ | A |
|---|---|---|---|---|---|---|---|---|
| D-4043 | Me | single bond | H | *c*-Pr | S(=O)₂CF₃ | Me | Me | O |
| D-4044 | Me(R) | single bond | H | *c*-Pr | S(=O)₂CF₃ | Me | Me | O |
| D-4045 | Me(S) | single bond | H | *c*-Pr | S(=O)₂CF₃ | Me | Me | O |
| D-4046 | H | CH₂ | Me | *c*-Pr | S(=O)₂CF₃ | Me | Me | O |
| D-4047 | Me | CH₂ | H | *c*-Pr | S(=O)₂CF₃ | Me | Me | O |
| D-4048 | H | CH(Me) | H | *c*-Pr | S(=O)₂CF₃ | Me | Me | O |
| D-4049 | H | CH₂CH₂ | Me | *c*-Pr | S(=O)₂CF₃ | Me | Me | O |
| D-4050 | Me | CH₂CH₂ | H | *c*-Pr | S(=O)₂CF₃ | Me | Me | O |
| D-4051 | H | single bond | Me | *c*-Pr | S(=O)₂CF₃ | Cl | Et | O |
| D-4052 | Me | single bond | H | *c*-Pr | S(=O)₂CF₃ | Cl | Et | O |
| D-4053 | Me(R) | single bond | H | *c*-Pr | S(=O)₂CF₃ | Cl | Et | O |
| D-4054 | Me(S) | single bond | H | *c*-Pr | S(=O)₂CF₃ | Cl | Et | O |
| D-4055 | H | CH₂ | Me | *c*-Pr | S(=O)₂CF₃ | Cl | Et | O |
| D-4056 | Me | CH₂ | H | *c*-Pr | S(=O)₂CF₃ | Cl | Et | O |
| D-4057 | H | CH(Me) | H | *c*-Pr | S(=O)₂CF₃ | Cl | Et | O |
| D-4058 | H | CH₂CH₂ | Me | *c*-Pr | S(=O)₂CF₃ | Cl | Et | O |
| D-4059 | Me | CH₂CH₂ | H | *c*-Pr | S(=O)₂CF₃ | Cl | Et | O |
| D-4060 | H | single bond | Me | *c*-Pr | S(=O)₂CF₃ | Me | Et | O |
| D-4061 | Me | single bond | H | *c*-Pr | S(=O)₂CF₃ | Me | Et | O |
| D-4062 | Me(R) | single bond | H | *c*-Pr | S(=O)₂CF₃ | Me | Et | O |
| D-4063 | Me(S) | single bond | H | *c*-Pr | S(=O)₂CF₃ | Me | Et | O |
| D-4064 | H | CH₂ | Me | *c*-Pr | S(=O)₂CF₃ | Me | Et | O |
| D-4065 | Me | CH₂ | H | *c*-Pr | S(=O)₂CF₃ | Me | Et | O |
| D-4066 | H | CH(Me) | H | *c*-Pr | S(=O)₂CF₃ | Me | Et | O |
| D-4067 | H | CH₂CH₂ | Me | *c*-Pr | S(=O)₂CF₃ | Me | Et | O |
| D-4068 | Me | CH₂CH₂ | H | *c*-Pr | S(=O)₂CF₃ | Me | Et | O |
| D-4069 | H | single bond | Me | *c*-Pr | S(=O)₂CF₃ | Cl | CH₂CF₃ | O |
| D-4070 | Me | single bond | H | *c*-Pr | S(=O)₂CF₃ | Cl | CH₂CF₃ | O |
| D-4071 | Me(R) | single bond | H | *c*-Pr | S(=O)₂CF₃ | Cl | CH₂CF₃ | O |
| D-4072 | Me(S) | single bond | H | *c*-Pr | S(=O)₂CF₃ | Cl | CH₂CF₃ | O |
| D-4073 | H | CH₂ | Me | *c*-Pr | S(=O)₂CF₃ | Cl | CH₂CF₃ | O |
| D-4074 | Me | CH₂ | H | *c*-Pr | S(=O)₂CF₃ | Cl | CH₂CF₃ | O |
| D-4075 | H | CH(Me) | H | *c*-Pr | S(=O)₂CF₃ | Cl | CH₂CF₃ | O |
| D-4076 | H | CH₂CH₂ | Me | *c*-Pr | S(=O)₂CF₃ | Cl | CH₂CF₃ | O |
| D-4077 | Me | CH₂CH₂ | H | *c*-Pr | S(=O)₂CF₃ | Cl | CH₂CF₃ | O |
| D-4078 | H | single bond | Me | *c*-Pr | S(=O)₂CF₃ | Me | CH₂CF₃ | O |
| D-4079 | Me | single bond | H | *c*-Pr | S(=O)₂CF₃ | Me | CH₂CF₃ | O |
| D-4080 | Me(R) | single bond | H | *c*-Pr | S(=O)₂CF₃ | Me | CH₂CF₃ | O |
| D-4081 | Me(S) | single bond | H | *c*-Pr | S(=O)₂CF₃ | Me | CH₂CF₃ | O |
| D-4082 | H | CH₂ | Me | *c*-Pr | S(=O)₂CF₃ | Me | CH₂CF₃ | O |
| D-4083 | Me | CH₂ | H | *c*-Pr | S(=O)₂CF₃ | Me | CH₂CF₃ | O |
| D-4084 | H | CH(Me) | H | *c*-Pr | S(=O)₂CF₃ | Me | CH₂CF₃ | O |
| D-4085 | H | CH₂CH₂ | Me | *c*-Pr | S(=O)₂CF₃ | Me | CH₂CF₃ | O |
| D-4086 | Me | CH₂CH₂ | H | *c*-Pr | S(=O)₂CF₃ | Me | CH₂CF₃ | O |

**[Table 738]**

| Compound Number | Y | W | Z | R¹ | R² | R³ | R¹⁰ | A |
|---|---|---|---|---|---|---|---|---|
| D-4087 | H | single bond | Me | *c*-Pr | S(=O)₂CF₃ | Cl | CH₂OMe | O |
| D-4088 | Me | single bond | H | *c*-Pr | S(=O)₂CF₃ | Cl | CH₂OMe | O |
| D-4089 | Me(R) | single bond | H | *c*-Pr | S(=O)₂CF₃ | Cl | CH₂OMe | O |
| D-4090 | Me(S) | single bond | H | *c*-Pr | S(=O)₂CF₃ | Cl | CH₂OMe | O |
| D-4091 | H | CH₂ | Me | *c*-Pr | S(=O)₂CF₃ | Cl | CH₂OMe | O |
| D-4092 | Me | CH₂ | H | *c*-Pr | S(=O)₂CF₃ | Cl | CH₂OMe | O |
| D-4093 | H | CH(Me) | H | *c*-Pr | S(=O)₂CF₃ | Cl | CH₂OMe | O |
| D-4094 | H | CH₂CH₂ | Me | *c*-Pr | S(=O)₂CF₃ | Cl | CH₂OMe | O |
| D-4095 | Me | CH₂CH₂ | H | *c*-Pr | S(=O)₂CF₃ | Cl | CH₂OMe | O |
| D-4096 | H | single bond | Me | *c*-Pr | S(=O)₂CF₃ | Me | CH₂OMe | O |
| D-4097 | Me | single bond | H | *c*-Pr | S(=O)₂CF₃ | Me | CH₂OMe | O |
| D-4098 | Me(R) | single bond | H | *c*-Pr | S(=O)₂CF₃ | Me | CH₂OMe | O |
| D-4099 | Me(S) | single bond | H | *c*-Pr | S(=O)₂CF₃ | Me | CH₂OMe | O |
| D-4100 | H | CH₂ | Me | *c*-Pr | S(=O)₂CF₃ | Me | CH₂OMe | O |
| D-4101 | Me | CH₂ | H | *c*-Pr | S(=O)₂CF₃ | Me | CH₂OMe | O |
| D-4102 | H | CH(Me) | H | *c*-Pr | S(=O)₂CF₃ | Me | CH₂OMe | O |
| D-4103 | H | CH₂CH₂ | Me | *c*-Pr | S(=O)₂CF₃ | Me | CH₂OMe | O |
| D-4104 | Me | CH₂CH₂ | H | *c*-Pr | S(=O)₂CF₃ | Me | CH₂OMe | O |
| D-4105 | H | single bond | Me | *c*-Pr | S(=O)₂CF₃ | Cl | CH₂CN | O |
| D-4106 | Me | single bond | H | *c*-Pr | S(=O)₂CF₃ | Cl | CH₂CN | O |
| D-4107 | Me(R) | single bond | H | *c*-Pr | S(=O)₂CF₃ | Cl | CH₂CN | O |
| D-4108 | Me(S) | single bond | H | *c*-Pr | S(=O)₂CF₃ | Cl | CH₂CN | O |
| D-4109 | H | CH₂ | Me | *c*-Pr | S(=O)₂CF₃ | Cl | CH₂CN | O |
| D-4110 | Me | CH₂ | H | *c*-Pr | S(=O)₂CF₃ | Cl | CH₂CN | O |
| D-4111 | H | CH(Me) | H | *c*-Pr | S(=O)₂CF₃ | Cl | CH₂CN | O |
| D-4112 | H | CH₂CH₂ | Me | *c*-Pr | S(=O)₂CF₃ | Cl | CH₂CN | O |
| D-4113 | Me | CH₂CH₂ | H | *c*-Pr | S(=O)₂CF₃ | Cl | CH₂CN | O |
| D-4114 | H | single bond | Me | *c*-Pr | S(=O)₂CF₃ | Me | CH₂CN | O |
| D-4115 | Me | single bond | H | *c*-Pr | S(=O)₂CF₃ | Me | CH₂CN | O |
| D-4116 | Me(R) | single bond | H | *c*-Pr | S(=O)₂CF₃ | Me | CH₂CN | O |
| D-4117 | Me(S) | single bond | H | *c*-Pr | S(=O)₂CF₃ | Me | CH₂CN | O |
| D-4118 | H | CH₂ | Me | *c*-Pr | S(=O)₂CF₃ | Me | CH₂CN | O |
| D-4119 | Me | CH₂ | H | *c*-Pr | S(=O)₂CF₃ | Me | CH₂CN | O |
| D-4120 | H | CH(Me) | H | *c*-Pr | S(=O)₂CF₃ | Me | CH₂CN | O |
| D-4121 | H | CH₂CH₂ | Me | *c*-Pr | S(=O)₂CF₃ | Me | CH₂CN | O |
| D-4122 | Me | CH₂CH₂ | H | *c*-Pr | S(=O)₂CF₃ | Me | CH₂CN | O |
| D-4123 | H | single bond | Me | *c*-Pr | S(=O)₂CF₃ | Cl | C(=O)Me | O |
| D-4124 | Me | single bond | H | c-Pr | S(=O)₂CF₃ | Cl | C(=O)Me | O |
| D-4125 | Me(R) | single bond | H | *c*-Pr | S(=O)₂CF₃ | Cl | C(=O)Me | O |
| D-4126 | Me(S) | single bond | H | *c*-Pr | S(=O)₂CF₃ | Cl | C(=O)Me | O |
| D-4127 | H | CH₂ | Me | *c*-Pr | S(=O)₂CF₃ | Cl | C(=O)Me | O |
| D-4128 | Me | CH₂ | H | *c*-Pr | S(=O)₂CF₃ | Cl | C(=O)Me | O |
| D-4129 | H | CH(Me) | H | *c*-Pr | S(=O)₂CF₃ | Cl | C(=O)Me | O |
| D-4130 | H | CH₂CH₂ | Me | *c*-Pr | S(=O)₂CF₃ | Cl | C(=O)Me | O |

**[Table 739]**

| Compound Number | Y | W | Z | R¹ | R² | R³ | R¹⁰ | A |
|---|---|---|---|---|---|---|---|---|
| D-4131 | Me | CH₂CH₂ | H | *c*-Pr | S(=O)₂CF₃ | Cl | C(=O)Me | O |
| D-4132 | H | single bond | Me | *c*-Pr | S(=O)₂CF₃ | Me | C(=O)Me | O |
| D-4133 | Me | single bond | H | *c*-Pr | S(=O)₂CF₃ | Me | C(=O)Me | O |
| D-4134 | Me(R) | single bond | H | *c*-Pr | S(=O)₂CF₃ | Me | C(=O)Me | O |
| D-4135 | Me(S) | single bond | H | *c*-Pr | S(=O)₂CF₃ | Me | C(=O)Me | O |
| D-4136 | H | CH₂ | Me | *c*-Pr | S(=O)₂CF₃ | Me | C(=O)Me | O |
| D-4137 | Me | CH₂ | H | *c*-Pr | S(=O)₂CF₃ | Me | C(=O)Me | O |
| D-4138 | H | CH(Me) | H | *c*-Pr | S(=O)₂CF₃ | Me | C(=O)Me | O |
| D-4139 | H | CH₂CH₂ | Me | *c*-Pr | S(=O)₂CF₃ | Me | C(=O)Me | O |
| D-4140 | Me | CH₂CH₂ | H | *c*-Pr | S(=O)₂CF₃ | Me | C(=O)Me | O |
| D-4141 | H | single bond | Me | *c*-Pr | Me | Cl | H | O |
| D-4142 | Me | single bond | H | *c*-Pr | Me | Cl | H | O |
| D-4143 | Me(R) | single bond | H | *c*-Pr | Me | Cl | H | O |
| D-4144 | Me(S) | single bond | H | *c*-Pr | Me | Cl | H | O |
| D-4145 | H | CH₂ | Me | *c*-Pr | Me | Cl | H | O |
| D-4146 | Me | CH₂ | H | *c*-Pr | Me | Cl | H | O |
| D-4147 | H | CH(Me) | H | *c*-Pr | Me | Cl | H | O |
| D-4148 | H | CH₂CH₂ | Me | *c*-Pr | Me | Cl | H | O |
| D-4149 | Me | CH₂CH₂ | H | *c*-Pr | Me | Cl | H | O |
| D-4150 | H | single bond | Me | *c*-Pr | Me | Cl | H | O |
| D-4151 | Me | single bond | H | *c*-Pr | Me | Cl | H | O |
| D-4152 | Me(R) | single bond | H | *c*-Pr | Me | Cl | H | O |
| D-4153 | Me(S) | single bond | H | *c*-Pr | Me | Cl | H | O |
| D-4154 | H | CH₂ | Me | *c*-Pr | Me | Cl | H | O |
| D-4155 | Me | CH₂ | H | *c*-Pr | Me | Cl | H | O |
| D-4156 | H | CH(Me) | H | *c*-Pr | Me | Cl | H | O |
| D-4157 | H | CH₂CH₂ | Me | *c*-Pr | Me | Cl | H | O |
| D-4158 | Me | CH₂CH₂ | H | *c*-Pr | Me | Cl | H | O |
| D-4159 | H | single bond | Me | *c*-Pr | Me | Cl | H | O |
| D-4160 | Me | single bond | H | *c*-Pr | Me | Cl | H | O |
| D-4161 | Me(R) | single bond | H | *c*-Pr | Me | Cl | H | O |
| D-4162 | Me(S) | single bond | H | *c*-Pr | Me | Cl | H | O |
| D-4163 | H | CH₂ | Me | *c*-Pr | Me | Cl | H | O |
| D-4164 | Me | CH₂ | H | *c*-Pr | Me | Cl | H | O |
| D-4165 | H | CH(Me) | H | *c*-Pr | Me | Cl | H | O |
| D-4166 | H | CH₂CH₂ | Me | *c*-Pr | Me | Cl | H | O |
| D-4167 | Me | CH₂CH₂ | H | *c*-Pr | Me | Cl | H | O |
| D-4168 | H | single bond | Me | *c*-Pr | Me | Cl | H | O |
| D-4169 | Me | single bond | H | *c*-Pr | Me | Cl | H | O |
| D-4170 | Me(R) | single bond | H | *c*-Pr | Me | Cl | H | O |
| D-4171 | Me(S) | single bond | H | *c*-Pr | Me | Cl | H | O |
| D-4172 | H | CH₂ | Me | *c*-Pr | Me | Cl | H | O |
| D-4173 | Me | CH₂ | H | *c*-Pr | Me | Cl | H | O |
| D-4174 | H | CH(Me) | H | *c*-Pr | Me | Cl | H | O |

**[Table 740]**

| Compound Number | Y | W | Z | R¹ | R² | R³ | R¹⁰ | A |
|---|---|---|---|---|---|---|---|---|
| D-4175 | H | CH₂CH₂ | Me | *c*-Pr | Me | Cl | H | O |
| D-4176 | Me | CH₂CH₂ | H | *c*-Pr | Me | Cl | H | O |
| D-4177 | H | single bond | Me | *c*-Pr | Me | Me | H | O |
| D-4178 | Me | single bond | H | *c*-Pr | Me | Me | H | O |
| D-4179 | Me(R) | single bond | H | *c*-Pr | Me | Me | H | O |
| D-4180 | Me(S) | single bond | H | *c*-Pr | Me | Me | H | O |
| D-4181 | H | CH₂ | Me | *c*-Pr | Me | Me | H | O |
| D-4182 | Me | CH₂ | H | *c*-Pr | Me | Me | H | O |
| D-4183 | H | CH(Me) | H | *c*-Pr | Me | Me | H | O |
| D-4184 | H | CH₂CH₂ | Me | *c*-Pr | Me | Me | H | O |
| D-4185 | Me | CH₂CH₂ | H | *c*-Pr | Me | Me | H | O |
| D-4186 | H | single bond | Me | *c*-Pr | Me | Me | H | O |
| D-4187 | Me | single bond | H | *c*-Pr | Me | Me | H | O |
| D-4188 | Me(R) | single bond | H | *c*-Pr | Me | Me | H | O |
| D-4189 | Me(S) | single bond | H | *c*-Pr | Me | Me | H | O |
| D-4190 | H | CH₂ | Me | *c*-Pr | Me | Me | H | O |
| D-4191 | Me | CH₂ | H | *c*-Pr | Me | Me | H | O |
| D-4192 | H | CH(Me) | H | *c*-Pr | Me | Me | H | O |
| D-4193 | H | CH₂CH₂ | Me | *c*-Pr | Me | Me | H | O |
| D-4194 | Me | CH₂CH₂ | H | *c*-Pr | Me | Me | H | O |
| D-4195 | H | single bond | Me | *c*-Pr | Me | Me | H | O |
| D-4196 | Me | single bond | H | *c*-Pr | Me | Me | H | O |
| D-4197 | Me(R) | single bond | H | *c*-Pr | Me | Me | H | O |
| D-4198 | Me(S) | single bond | H | *c*-Pr | Me | Me | H | O |
| D-4199 | H | CH₂ | Me | *c*-Pr | Me | Me | H | O |
| D-4200 | Me | CH₂ | H | *c*-Pr | Me | Me | H | O |
| D-4201 | H | CH(Me) | H | *c*-Pr | Me | Me | H | O |
| D-4202 | H | CH₂CH₂ | Me | *c*-Pr | Me | Me | H | O |
| D-4203 | Me | CH₂CH₂ | H | *c*-Pr | Me | Me | H | O |
| D-4204 | H | single bond | Me | *c*-Pr | Me | Me | H | O |
| D-4205 | Me | single bond | H | *c*-Pr | Me | Me | H | O |
| D-4206 | Me(R) | single bond | H | *c*-Pr | Me | Me | H | O |
| D-4207 | Me(S) | single bond | H | *c*-Pr | Me | Me | H | O |
| D-4208 | H | CH₂ | Me | *c*-Pr | Me | Me | H | O |
| D-4209 | Me | CH₂ | H | *c*-Pr | Me | Me | H | O |
| D-4210 | H | CH(Me) | H | *c*-Pr | Me | Me | H | O |
| D-4211 | H | CH₂CH₂ | Me | *c*-Pr | Me | Me | H | O |
| D-4212 | Me | CH₂CH₂ | H | *c*-Pr | Me | Me | H | O |
| D-4213 | H | single bond | Me | *c*-Pr | C(=O)Me | Cl | H | O |
| D-4214 | Me | single bond | H | *c*-Pr | C(=O)Me | Cl | H | O |
| D-4215 | Me(R) | single bond | H | *c*-Pr | C(=O)Me | Cl | H | O |
| D-4216 | Me(S) | single bond | H | *c*-Pr | C(=O)Me | Cl | H | O |
| D-4217 | H | CH₂ | Me | *c*-Pr | C(=O)Me | Cl | H | O |
| D-4218 | Me | CH₂ | H | *c*-Pr | C(=O)Me | Cl | H | O |

**[Table 741]**

| Compound Number | Y | W | Z | R¹ | R² | R³ | R¹⁰ | A |
|---|---|---|---|---|---|---|---|---|
| D-4219 | H | CH(Me) | H | *c*-Pr | C(=O)Me | Cl | H | O |
| D-4220 | H | CH₂CH₂ | Me | *c*-Pr | C(=O)Me | Cl | H | O |
| D-4221 | Me | CH₂CH₂ | H | *c*-Pr | C(=O)Me | Cl | H | O |
| D-4222 | H | single bond | Me | *c*-Pr | C(=O)Me | Cl | H | O |
| D-4223 | Me | single bond | H | *c*-Pr | C(=O)Me | Cl | H | O |
| D-4224 | Me(R) | single bond | H | *c*-Pr | C(=O)Me | Cl | H | O |
| D-4225 | Me(S) | single bond | H | *c*-Pr | C(=O)Me | Cl | H | O |
| D-4226 | H | CH₂ | Me | *c*-Pr | C(=O)Me | Cl | H | O |
| D-4227 | Me | CH₂ | H | *c*-Pr | C(=O)Me | Cl | H | O |
| D-4228 | H | CH(Me) | H | *c*-Pr | C(=O)Me | Cl | H | O |
| D-4229 | H | CH₂CH₂ | Me | *c*-Pr | C(=O)Me | Cl | H | O |
| D-4230 | Me | CH₂CH₂ | H | *c*-Pr | C(=O)Me | Cl | H | O |
| D-4231 | H | single bond | Me | *c*-Pr | C(=O)Me | Cl | H | O |
| D-4232 | Me | single bond | H | *c*-Pr | C(=O)Me | Cl | H | O |
| D-4233 | Me(R) | single bond | H | *c*-Pr | C(=O)Me | Cl | H | O |
| D-4234 | Me(S) | single bond | H | *c*-Pr | C(=O)Me | Cl | H | O |
| D-4235 | H | CH₂ | Me | *c*-Pr | C(=O)Me | Cl | H | O |
| D-4236 | Me | CH₂ | H | c-Pr | C(=O)Me | Cl | H | O |
| D-4237 | H | CH(Me) | H | *c*-Pr | C(=O)Me | Cl | H | O |
| D-4238 | H | CH₂CH₂ | Me | *c*-Pr | C(=O)Me | Cl | H | O |
| D-4239 | Me | CH₂CH₂ | H | *c*-Pr | C(=O)Me | Cl | H | O |
| D-4240 | H | single bond | Me | *c*-Pr | C(=O)Me | Cl | H | O |
| D-4241 | Me | single bond | H | *c*-Pr | C(=O)Me | Cl | H | O |
| D-4242 | Me(R) | single bond | H | *c*-Pr | C(=O)Me | Cl | H | O |
| D-4243 | Me(S) | single bond | H | *c*-Pr | C(=O)Me | Cl | H | O |
| D-4244 | H | CH₂ | Me | *c*-Pr | C(=O)Me | Cl | H | O |
| D-4245 | Me | CH₂ | H | *c*-Pr | C(=O)Me | Cl | H | O |
| D-4246 | H | CH(Me) | H | *c*-Pr | C(=O)Me | Cl | H | O |
| D-4247 | H | CH₂CH₂ | Me | *c*-Pr | C(=O)Me | Cl | H | O |
| D-4248 | Me | CH₂CH₂ | H | *c*-Pr | C(=O)Me | Cl | H | O |
| D-4249 | H | single bond | Me | *c*-Pr | C(=O)Me | Me | H | O |
| D-4250 | Me | single bond | H | *c*-Pr | C(=O)Me | Me | H | O |
| D-4251 | Me(R) | single bond | H | *c*-Pr | C(=O)Me | Me | H | O |
| D-4252 | Me(S) | single bond | H | *c*-Pr | C(=O)Me | Me | H | O |
| D-4253 | H | CH₂ | Me | *c*-Pr | C(=O)Me | Me | H | O |
| D-4254 | Me | CH₂ | H | *c*-Pr | C(=O)Me | Me | H | O |
| D-4255 | H | CH(Me) | H | *c*-Pr | C(=O)Me | Me | H | O |
| D-4256 | H | CH₂CH₂ | Me | *c*-Pr | C(=O)Me | Me | H | O |
| D-4257 | Me | CH₂CH₂ | H | *c*-Pr | C(=O)Me | Me | H | O |
| D-4258 | H | single bond | Me | *c*-Pr | C(=O)Me | Me | H | O |
| D-4259 | Me | single bond | H | *c*-Pr | C(=O)Me | Me | H | O |
| D-4260 | Me(R) | single bond | H | *c*-Pr | C(=O)Me | Me | H | O |
| D-4261 | Me(S) | single bond | H | *c*-Pr | C(=O)Me | Me | H | O |
| D-4262 | H | CH₂ | Me | *c*-Pr | C(=O)Me | Me | H | O |

**[Table 742]**

| Compound Number | Y | W | Z | R¹ | R² | R³ | R¹⁰ | A |
|---|---|---|---|---|---|---|---|---|
| D-4263 | Me | CH₂ | H | *c*-Pr | C(=O)Me | Me | H | O |
| D-4264 | H | CH(Me) | H | *c*-Pr | C(=O)Me | Me | H | O |
| D-4265 | H | CH₂CH₂ | Me | *c*-Pr | C(=O)Me | Me | H | O |
| D-4266 | Me | CH₂CH₂ | H | *c*-Pr | C(=O)Me | Me | H | O |
| D-4267 | H | single bond | Me | *c*-Pr | C(=O)Me | Me | H | O |
| D-4268 | Me | single bond | H | *c*-Pr | C(=O)Me | Me | H | O |
| D-4269 | Me(R) | single bond | H | *c*-Pr | C(=O)Me | Me | H | O |
| D-4270 | Me(S) | single bond | H | *c*-Pr | C(=O)Me | Me | H | O |
| D-4271 | H | CH₂ | Me | *c*-Pr | C(=O)Me | Me | H | O |
| D-4272 | Me | CH₂ | H | *c*-Pr | C(=O)Me | Me | H | O |
| D-4273 | H | CH(Me) | H | *c*-Pr | C(=O)Me | Me | H | O |
| D-4274 | H | CH₂CH₂ | Me | *c*-Pr | C(=O)Me | Me | H | O |
| D-4275 | Me | CH₂CH₂ | H | *c*-Pr | C(=O)Me | Me | H | O |
| D-4276 | H | single bond | Me | *c*-Pr | C(=O)Me | Me | H | O |
| D-4277 | Me | single bond | H | *c*-Pr | C(=O)Me | Me | H | O |
| D-4278 | Me(R) | single bond | H | *c*-Pr | C(=O)Me | Me | H | O |
| D-4279 | Me(S) | single bond | H | *c*-Pr | C(=O)Me | Me | H | O |
| D-4280 | H | CH₂ | Me | *c*-Pr | C(=O)Me | Me | H | O |
| D-4281 | Me | CH₂ | H | *c*-Pr | C(=O)Me | Me | H | O |
| D-4282 | H | CH(Me) | H | *c*-Pr | C(=O)Me | Me | H | O |
| D-4283 | H | CH₂CH₂ | Me | *c*-Pr | C(=O)Me | Me | H | O |
| D-4284 | Me | CH₂CH₂ | H | *c*-Pr | C(=O)Me | Me | H | O |
| D-4285 | H | single bond | Me | *c*-Pr | C(=O)(*i*-Pr) | Cl | H | O |
| D-4286 | Me | single bond | H | *c*-Pr | C(=O)(*i*-Pr) | Cl | H | O |
| D-4287 | Me(R) | single bond | H | *c*-Pr | C(=O)(*i*-Pr) | Cl | H | O |
| D-4288 | Me(S) | single bond | H | *c*-Pr | C(=O)(*i*-Pr) | Cl | H | O |
| D-4289 | H | CH₂ | Me | *c*-Pr | C(=O)(*i*-Pr) | Cl | H | O |
| D-4290 | Me | CH₂ | H | *c*-Pr | C(=O)(*i*-Pr) | Cl | H | O |
| D-4291 | H | CH(Me) | H | *c*-Pr | C(=O)(*i*-Pr) | Cl | H | O |
| D-4292 | H | CH₂CH₂ | Me | *c*-Pr | C(=O)(*i*-Pr) | Cl | H | O |
| D-4293 | Me | CH₂CH₂ | H | *c*-Pr | C(=O)(*i*-Pr) | Cl | H | O |
| D-4294 | H | single bond | Me | *c*-Pr | C(=O)(*i*-Pr) | Cl | H | O |
| D-4295 | Me | single bond | H | *c*-Pr | C(=O)(*i*-Pr) | Cl | H | O |
| D-4296 | Me(R) | single bond | H | *c*-Pr | C(=O)(*i*-Pr) | Cl | H | O |
| D-4297 | Me(S) | single bond | H | *c*-Pr | C(=O)(*i*-Pr) | Cl | H | O |
| D-4298 | H | CH₂ | Me | *c*-Pr | C(=O)(*i*-Pr) | Cl | H | O |
| D-4299 | Me | CH₂ | H | *c*-Pr | C(=O)(*i*-Pr) | Cl | H | O |
| D-4300 | H | CH(Me) | H | *c*-Pr | C(=O)(*i*-Pr) | Cl | H | O |
| D-4301 | H | CH₂CH₂ | Me | *c*-Pr | C(=O)(*i*-Pr) | Cl | H | O |
| D-4302 | Me | CH₂CH₂ | H | *c*-Pr | C(=O)(*i*-Pr) | Cl | H | O |
| D-4303 | H | single bond | Me | *c*-Pr | C(=O)(*i*-Pr) | Cl | H | O |
| D-4304 | Me | single bond | H | *c*-Pr | C(=O)(*i*-Pr) | Cl | H | O |
| D-4305 | Me(R) | single bond | H | *c*-Pr | C(=O)(*i*-Pr) | Cl | H | O |
| D-4306 | Me(S) | single bond | H | *c*-Pr | C(=O)(*i*-Pr) | Cl | H | O |

**[Table 743]**

| Compound Number | Y | W | Z | R¹ | R² | R³ | R¹⁰ | A |
|---|---|---|---|---|---|---|---|---|
| D-4307 | H | CH₂ | Me | *c*-Pr | C(=O)(*i*-Pr) | Cl | H | O |
| D-4308 | Me | CH₂ | H | *c*-Pr | C(=O)(*i*-Pr) | Cl | H | O |
| D-4309 | H | CH(Me) | H | *c*-Pr | C(=O)(*i*-Pr) | Cl | H | O |
| D-4310 | H | CH₂CH₂ | Me | *c*-Pr | C(=O)(*i*-Pr) | Cl | H | O |
| D-4311 | Me | CH₂CH₂ | H | *c*-Pr | C(=O)(*i*-Pr) | Cl | H | O |
| D-4312 | H | single bond | Me | *c*-Pr | C(=O)(*i*-Pr) | Cl | H | O |
| D-4313 | Me | single bond | H | *c*-Pr | C(=O)(*i*-Pr) | Cl | H | O |
| D-4314 | Me(R) | single bond | H | *c*-Pr | C(=O)(*i*-Pr) | Cl | H | O |
| D-4315 | Me(S) | single bond | H | *c*-Pr | C(=O)(*i*-Pr) | Cl | H | O |
| D-4316 | H | CH₂ | Me | *c*-Pr | C(=O)(*i*-Pr) | Cl | H | O |
| D-4317 | Me | CH₂ | H | *c*-Pr | C(=O)(*i*-Pr) | Cl | H | O |
| D-4318 | H | CH(Me) | H | *c*-Pr | C(=O)(*i*-Pr) | Cl | H | O |
| D-4319 | H | CH₂CH₂ | Me | *c*-Pr | C(=O)(*i*-Pr) | Cl | H | O |
| D-4320 | Me | CH₂CH₂ | H | *c*-Pr | C(=O)(*i*-Pr) | Cl | H | O |
| D-4321 | H | single bond | Me | *c*-Pr | C(=O)(*i*-Pr) | Me | H | O |
| D-4322 | Me | single bond | H | *c*-Pr | C(=O)(*i*-Pr) | Me | H | O |
| D-4323 | Me(R) | single bond | H | *c*-Pr | C(=O)(*i*-Pr) | Me | H | O |
| D-4324 | Me(S) | single bond | H | *c*-Pr | C(=O)(*i*-Pr) | Me | H | O |
| D-4325 | H | CH₂ | Me | *c*-Pr | C(=O)(*i*-Pr) | Me | H | O |
| D-4326 | Me | CH₂ | H | *c*-Pr | C(=O)(*i*-Pr) | Me | H | O |
| D-4327 | H | CH(Me) | H | *c*-Pr | C(=O)(*i*-Pr) | Me | H | O |
| D-4328 | H | CH₂CH₂ | Me | *c*-Pr | C(=O)(*i*-Pr) | Me | H | O |
| D-4329 | Me | CH₂CH₂ | H | *c*-Pr | C(=O)(*i*-Pr) | Me | H | O |
| D-4330 | H | single bond | Me | *c*-Pr | C(=O)(*i*-Pr) | Me | H | O |
| D-4331 | Me | single bond | H | *c*-Pr | C(=O)(*i*-Pr) | Me | H | O |
| D-4332 | Me(R) | single bond | H | *c*-Pr | C(=O)(*i*-Pr) | Me | H | O |
| D-4333 | Me(S) | single bond | H | *c*-Pr | C(=O)(*i*-Pr) | Me | H | O |
| D-4334 | H | CH₂ | Me | *c*-Pr | C(=O)(*i*-Pr) | Me | H | O |
| D-4335 | Me | CH₂ | H | *c*-Pr | C(=O)(*i*-Pr) | Me | H | O |
| D-4336 | H | CH(Me) | H | *c*-Pr | C(=O)(*i*-Pr) | Me | H | O |
| D-4337 | H | CH₂CH₂ | Me | *c*-Pr | C(=O)(*i*-Pr) | Me | H | O |
| D-4338 | Me | CH₂CH₂ | H | *c*-Pr | C(=O)(*i*-Pr) | Me | H | O |
| D-4339 | H | single bond | Me | *c*-Pr | C(=O)(*i*-Pr) | Me | H | O |
| D-4340 | Me | single bond | H | *c*-Pr | C(=O)(*i*-Pr) | Me | H | O |
| D-4341 | Me(R) | single bond | H | *c*-Pr | C(=O)(*i*-Pr) | Me | H | O |
| D-4342 | Me(S) | single bond | H | *c*-Pr | C(=O)(*i*-Pr) | Me | H | O |
| D-4343 | H | CH₂ | Me | *c*-Pr | C(=O)(*i*-Pr) | Me | H | O |
| D-4344 | Me | CH₂ | H | *c*-Pr | C(=O)(*i*-Pr) | Me | H | O |
| D-4345 | H | CH(Me) | H | *c*-Pr | C(=O)(*i*-Pr) | Me | H | O |
| D-4346 | H | CH₂CH₂ | Me | *c*-Pr | C(=O)(*i*-Pr) | Me | H | O |
| D-4347 | Me | CH₂CH₂ | H | *c*-Pr | C(=O)(*i*-Pr) | Me | H | O |
| D-4348 | H | single bond | Me | *c*-Pr | C(=O)(*i*-Pr) | Me | H | O |
| D-4349 | Me | single bond | H | *c*-Pr | C(=O)(*i*-Pr) | Me | H | O |
| D-4350 | Me(R) | single bond | H | *c*-Pr | C(=O)(*i*-Pr) | Me | H | O |

**[Table 744]**

| Compound Number | Y | W | Z | R¹ | R² | R³ | R¹⁰ | A |
|---|---|---|---|---|---|---|---|---|
| D-4351 | Me(S) | single bond | H | *c*-Pr | C(=O)(*i*-Pr) | Me | H | O |
| D-4352 | H | CH₂ | Me | *c*-Pr | C(=O)(*i*-Pr) | Me | H | O |
| D-4353 | Me | CH₂ | H | *c*-Pr | C(=O)(*i*-Pr) | Me | H | O |
| D-4354 | H | CH(Me) | H | *c*-Pr | C(=O)(*i*-Pr) | Me | H | O |
| D-4355 | H | CH₂CH₂ | Me | *c*-Pr | C(=O)(*i*-Pr) | Me | H | O |
| D-4356 | Me | CH₂CH₂ | H | *c*-Pr | C(=O)(*i*-Pr) | Me | H | O |
| D-4357 | H | single bond | Me | *c*-Pr | H | Br | H | O |
| D-4358 | Me | single bond | H | *c*-Pr | H | Br | H | O |
| D-4359 | Me(R) | single bond | H | *c*-Pr | H | Br | H | O |
| D-4360 | Me(S) | single bond | H | *c*-Pr | H | Br | H | O |
| D-4361 | H | CH₂ | Me | *c*-Pr | H | Br | H | O |
| D-4362 | Me | CH₂ | H | *c*-Pr | H | Br | H | O |
| D-4363 | H | CH(Me) | H | *c*-Pr | H | Br | H | O |
| D-4364 | H | CH₂CH₂ | Me | *c*-Pr | H | Br | H | O |
| D-4365 | Me | CH₂CH₂ | H | *c*-Pr | H | Br | H | O |
| D-4366 | H | single bond | Me | *c*-Pr | H | Br | H | O |
| D-4367 | Me | single bond | H | *c*-Pr | H | Br | H | O |
| D-4368 | Me(R) | single bond | H | *c*-Pr | H | Br | H | O |
| D-4369 | Me(S) | single bond | H | *c*-Pr | H | Br | H | O |
| D-4370 | H | CH₂ | Me | *c*-Pr | H | Br | H | O |
| D-4371 | Me | CH₂ | H | *c*-Pr | H | Br | H | O |
| D-4372 | H | CH(Me) | H | *c*-Pr | H | Br | H | O |
| D-4373 | H | CH₂CH₂ | Me | *c*-Pr | H | Br | H | O |
| D-4374 | Me | CH₂CH₂ | H | *c*-Pr | H | Br | H | O |
| D-4375 | H | single bond | Me | *c*-Pr | H | Br | H | O |
| D-4376 | Me | single bond | H | *c*-Pr | H | Br | H | O |
| D-4377 | Me(R) | single bond | H | *c*-Pr | H | Br | H | O |
| D-4378 | Me(S) | single bond | H | *c*-Pr | H | Br | H | O |
| D-4379 | H | CH₂ | Me | *c*-Pr | H | Br | H | O |
| D-4380 | Me | CH₂ | H | *c*-Pr | H | Br | H | O |
| D-4381 | H | CH(Me) | H | *c*-Pr | H | Br | H | O |
| D-4382 | H | CH₂CH₂ | Me | *c*-Pr | H | Br | H | O |
| D-4383 | Me | CH₂CH₂ | H | *c*-Pr | H | Br | H | O |
| D-4384 | H | single bond | Me | *c*-Pr | H | Br | H | O |
| D-4385 | Me | single bond | H | *c*-Pr | H | Br | H | O |
| D-4386 | Me(R) | single bond | H | *c*-Pr | H | Br | H | O |
| D-4387 | Me(S) | single bond | H | *c*-Pr | H | Br | H | O |
| D-4388 | H | CH₂ | Me | *c*-Pr | H | Br | H | O |
| D-4389 | Me | CH₂ | H | *c*-Pr | H | Br | H | O |
| D-4390 | H | CH(Me) | H | *c*-Pr | H | Br | H | O |
| D-4391 | H | CH₂CH₂ | Me | *c*-Pr | H | Br | H | O |
| D-4392 | Me | CH₂CH₂ | H | *c*-Pr | H | Br | H | O |
| D-4393 | H | single bond | Me | *c*-Pr | H | CF₃ | H | O |
| D-4394 | Me | single bond | H | *c*-Pr | H | CF₃ | H | O |

**[Table 745]**

| Compound Number | Y | W | Z | R¹ | R² | R³ | R¹⁰ | A |
|---|---|---|---|---|---|---|---|---|
| D-4395 | Me(R) | single bond | H | *c*-Pr | H | CF₃ | H | O |
| D-4396 | Me(S) | single bond | H | *c*-Pr | H | CF₃ | H | O |
| D-4397 | H | CH₂ | Me | *c*-Pr | H | CF₃ | H | O |
| D-4398 | Me | CH₂ | H | *c*-Pr | H | CF₃ | H | O |
| D-4399 | H | CH(Me) | H | *c*-Pr | H | CF₃ | H | O |
| D-4400 | H | CH₂CH₂ | Me | *c*-Pr | H | CF₃ | H | O |
| D-4401 | Me | CH₂CH₂ | H | *c*-Pr | H | CF₃ | H | O |
| D-4402 | H | single bond | Me | *c*-Pr | H | CF₃ | H | O |
| D-4403 | Me | single bond | H | *c*-Pr | H | CF₃ | H | O |
| D-4404 | Me(R) | single bond | H | *c*-Pr | H | CF₃ | H | O |
| D-4405 | Me(S) | single bond | H | *c*-Pr | H | CF₃ | H | O |
| D-4406 | H | CH₂ | Me | *c*-Pr | H | CF₃ | H | O |
| D-4407 | Me | CH₂ | H | *c*-Pr | H | CF₃ | H | O |
| D-4408 | H | CH(Me) | H | *c*-Pr | H | CF₃ | H | O |
| D-4409 | H | CH₂CH₂ | Me | *c*-Pr | H | CF₃ | H | O |
| D-4410 | Me | CH₂CH₂ | H | *c*-Pr | H | CF₃ | H | O |
| D-4411 | H | single bond | Me | *c*-Pr | H | CF₃ | H | O |
| D-4412 | Me | single bond | H | *c*-Pr | H | CF₃ | H | O |
| D-4413 | Me(R) | single bond | H | *c*-Pr | H | CF₃ | H | O |
| D-4414 | Me(S) | single bond | H | *c*-Pr | H | CF₃ | H | O |
| D-4415 | H | CH₂ | Me | *c*-Pr | H | CF₃ | H | O |
| D-4416 | Me | CH₂ | H | *c*-Pr | H | CF₃ | H | O |
| D-4417 | H | CH(Me) | H | *c*-Pr | H | CF₃ | H | O |
| D-4418 | H | CH₂CH₂ | Me | *c*-Pr | H | CF₃ | H | O |
| D-4419 | Me | CH₂CH₂ | H | *c*-Pr | H | CF₃ | H | O |
| D-4420 | H | single bond | Me | *c*-Pr | H | CF₃ | H | O |
| D-4421 | Me | single bond | H | *c*-Pr | H | CF₃ | H | O |
| D-4422 | Me(R) | single bond | H | *c*-Pr | H | CF₃ | H | O |
| D-4423 | Me(S) | single bond | H | *c*-Pr | H | CF₃ | H | O |
| D-4424 | H | CH₂ | Me | *c*-Pr | H | CF₃ | H | O |
| D-4425 | Me | CH₂ | H | *c*-Pr | H | CF₃ | H | O |
| D-4426 | H | CH(Me) | H | *c*-Pr | H | CF₃ | H | O |
| D-4427 | H | CH₂CH₂ | Me | *c*-Pr | H | CF₃ | H | O |
| D-4428 | Me | CH₂CH₂ | H | *c*-Pr | H | CF₃ | H | O |
| D-4429 | H | single bond | Me | *c*-Pr | H | OMe | H | O |
| D-4430 | Me | single bond | H | *c*-Pr | H | OMe | H | O |
| D-4431 | Me(R) | single bond | H | *c*-Pr | H | OMe | H | O |
| D-4432 | Me(S) | single bond | H | *c*-Pr | H | OMe | H | O |
| D-4433 | H | CH₂ | Me | *c*-Pr | H | OMe | H | O |
| D-4434 | Me | CH₂ | H | *c*-Pr | H | OMe | H | O |
| D-4435 | H | CH(Me) | H | *c*-Pr | H | OMe | H | O |
| D-4436 | H | CH₂CH₂ | Me | *c*-Pr | H | OMe | H | O |
| D-4437 | Me | CH₂CH₂ | H | *c*-Pr | H | OMe | H | O |
| D-4438 | H | single bond | Me | *c*-Pr | H | OMe | H | O |

**[Table 746]**

| Compound Number | Y | W | Z | R¹ | R² | R³ | R¹⁰ | A |
|---|---|---|---|---|---|---|---|---|
| D-4439 | Me | single bond | H | *c*-Pr | H | OMe | H | O |
| D-4440 | Me(R) | single bond | H | *c*-Pr | H | OMe | H | O |
| D-4441 | Me(S) | single bond | H | *c*-Pr | H | OMe | H | O |
| D-4442 | H | CH₂ | Me | *c*-Pr | H | OMe | H | O |
| D-4443 | Me | CH₂ | H | *c*-Pr | H | OMe | H | O |
| D-4444 | H | CH(Me) | H | *c*-Pr | H | OMe | H | O |
| D-4445 | H | CH₂CH₂ | Me | *c*-Pr | H | OMe | H | O |
| D-4446 | Me | CH₂CH₂ | H | *c*-Pr | H | OMe | H | O |
| D-4447 | H | single bond | Me | *c*-Pr | H | OMe | H | O |
| D-4448 | Me | single bond | H | *c*-Pr | H | OMe | H | O |
| D-4449 | Me(R) | single bond | H | *c*-Pr | H | OMe | H | O |
| D-4450 | Me(S) | single bond | H | *c*-Pr | H | OMe | H | O |
| D-4451 | H | CH₂ | Me | *c*-Pr | H | OMe | H | O |
| D-4452 | Me | CH₂ | H | *c*-Pr | H | OMe | H | O |
| D-4453 | H | CH(Me) | H | *c*-Pr | H | OMe | H | O |
| D-4454 | H | CH₂CH₂ | Me | *c*-Pr | H | OMe | H | O |
| D-4455 | Me | CH₂CH₂ | H | *c*-Pr | H | OMe | H | O |
| D-4456 | H | single bond | Me | *c*-Pr | H | OMe | H | O |
| D-4457 | Me | single bond | H | *c*-Pr | H | OMe | H | O |
| D-4458 | Me(R) | single bond | H | *c*-Pr | H | OMe | H | O |
| D-4459 | Me(S) | single bond | H | *c*-Pr | H | OMe | H | O |
| D-4460 | H | CH₂ | Me | *c*-Pr | H | OMe | H | O |
| D-4461 | Me | CH₂ | H | *c*-Pr | H | OMe | H | O |
| D-4462 | H | CH(Me) | H | *c*-Pr | H | OMe | H | O |
| D-4463 | H | CH₂CH₂ | Me | *c*-Pr | H | OMe | H | O |
| D-4464 | Me | CH₂CH₂ | H | *c*-Pr | H | OMe | H | O |
| D-4465 | H | single bond | Me | *c*-Pr | H | OCF₃ | H | O |
| D-4466 | Me | single bond | H | *c*-Pr | H | OCF₃ | H | O |
| D-4467 | Me(R) | single bond | H | *c*-Pr | H | OCF₃ | H | O |
| D-4468 | Me(S) | single bond | H | *c*-Pr | H | OCF₃ | H | O |
| D-4469 | H | CH₂ | Me | *c*-Pr | H | OCF₃ | H | O |
| D-4470 | Me | CH₂ | H | *c*-Pr | H | OCF₃ | H | O |
| D-4471 | H | CH(Me) | H | *c*-Pr | H | OCF₃ | H | O |
| D-4472 | H | CH₂CH₂ | Me | *c*-Pr | H | OCF₃ | H | O |
| D-4473 | Me | CH₂CH₂ | H | *c*-Pr | H | OCF₃ | H | O |
| D-4474 | H | single bond | Me | *c*-Pr | H | OCF₃ | H | O |
| D-4475 | Me | single bond | H | *c*-Pr | H | OCF₃ | H | O |
| D-4476 | Me(R) | single bond | H | *c*-Pr | H | OCF₃ | H | O |
| D-4477 | Me(S) | single bond | H | *c*-Pr | H | OCF₃ | H | O |
| D-4478 | H | CH₂ | Me | *c*-Pr | H | OCF₃ | H | O |
| D-4479 | Me | CH₂ | H | *c*-Pr | H | OCF₃ | H | O |
| D-4480 | H | CH(Me) | H | *c*-Pr | H | OCF₃ | H | O |
| D-4481 | H | CH₂CH₂ | Me | *c*-Pr | H | OCF₃ | H | O |
| D-4482 | Me | CH₂CH₂ | H | *c*-Pr | H | OCF₃ | H | O |

**[Table 747]**

| Compound Number | Y | W | Z | R¹ | R² | R³ | R¹⁰ | A |
|---|---|---|---|---|---|---|---|---|
| D-4483 | H | single bond | Me | *c*-Pr | H | OCF₃ | H | O |
| D-4484 | Me | single bond | H | *c*-Pr | H | OCF₃ | H | O |
| D-4485 | Me(R) | single bond | H | *c*-Pr | H | OCF₃ | H | O |
| D-4486 | Me(S) | single bond | H | *c*-Pr | H | OCF₃ | H | O |
| D-4487 | H | CH₂ | Me | *c*-Pr | H | OCF₃ | H | O |
| D-4488 | Me | CH₂ | H | *c*-Pr | H | OCF₃ | H | O |
| D-4489 | H | CH(Me) | H | *c*-Pr | H | OCF₃ | H | O |
| D-4490 | H | CH₂CH₂ | Me | *c*-Pr | H | OCF₃ | H | O |
| D-4491 | Me | CH₂CH₂ | H | *c*-Pr | H | OCF₃ | H | O |
| D-4492 | H | single bond | Me | *c*-Pr | H | OCF₃ | H | O |
| D-4493 | Me | single bond | H | *c*-Pr | H | OCF₃ | H | O |
| D-4494 | Me(R) | single bond | H | *c*-Pr | H | OCF₃ | H | O |
| D-4495 | Me(S) | single bond | H | *c*-Pr | H | OCF₃ | H | O |
| D-4496 | H | CH₂ | Me | *c*-Pr | H | OCF₃ | H | O |
| D-4497 | Me | CH₂ | H | *c*-Pr | H | OCF₃ | H | O |
| D-4498 | H | CH(Me) | H | *c*-Pr | H | OCF₃ | H | O |
| D-4499 | H | CH₂CH₂ | Me | *c*-Pr | H | OCF₃ | H | O |
| D-4500 | Me | CH₂CH₂ | H | *c*-Pr | H | OCF₃ | H | O |
| D-4501 | H | single bond | Me | *c*-Pr | H | Br | Me | O |
| D-4502 | Me | single bond | H | *c*-Pr | H | Br | Me | O |
| D-4503 | Me(R) | single bond | H | *c*-Pr | H | Br | Me | O |
| D-4504 | Me(S) | single bond | H | *c*-Pr | H | Br | Me | O |
| D-4505 | H | CH₂ | Me | *c*-Pr | H | Br | Me | O |
| D-4506 | Me | CH₂ | H | *c*-Pr | H | Br | Me | O |
| D-4507 | H | CH(Me) | H | *c*-Pr | H | Br | Me | O |
| D-4508 | H | CH₂CH₂ | Me | *c*-Pr | H | Br | Me | O |
| D-4509 | Me | CH₂CH₂ | H | *c*-Pr | H | Br | Me | O |
| D-4510 | H | single bond | Me | *c*-Pr | H | Br | Me | O |
| D-4511 | Me | single bond | H | *c*-Pr | H | Br | Me | O |
| D-4512 | Me(R) | single bond | H | *c*-Pr | H | Br | Me | O |
| D-4513 | Me(S) | single bond | H | *c*-Pr | H | Br | Me | O |
| D-4514 | H | CH₂ | Me | *c*-Pr | H | Br | Me | O |
| D-4515 | Me | CH₂ | H | *c*-Pr | H | Br | Me | O |
| D-4516 | H | CH(Me) | H | *c*-Pr | H | Br | Me | O |
| D-4517 | H | CH₂CH₂ | Me | *c*-Pr | H | Br | Me | O |
| D-4518 | Me | CH₂CH₂ | H | *c*-Pr | H | Br | Me | O |
| D-4519 | H | single bond | Me | *c*-Pr | H | Br | Me | O |
| D-4520 | Me | single bond | H | *c*-Pr | H | Br | Me | O |
| D-4521 | Me(R) | single bond | H | *c*-Pr | H | Br | Me | O |
| D-4522 | Me(S) | single bond | H | *c*-Pr | H | Br | Me | O |
| D-4523 | H | CH₂ | Me | *c*-Pr | H | Br | Me | O |
| D-4524 | Me | CH₂ | H | *c*-Pr | H | Br | Me | O |
| D-4525 | H | CH(Me) | H | *c*-Pr | H | Br | Me | O |
| D-4526 | H | CH₂CH₂ | Me | *c*-Pr | H | Br | Me | O |

**[Table 748]**

| Compound Number | Y | W | Z | R¹ | R² | R³ | R¹⁰ | A |
|---|---|---|---|---|---|---|---|---|
| D-4527 | Me | CH₂CH₂ | H | *c*-Pr | H | Br | Me | O |
| D-4528 | H | single bond | Me | *c*-Pr | H | Br | Me | O |
| D-4529 | Me | single bond | H | *c*-Pr | H | Br | Me | O |
| D-4530 | Me(R) | single bond | H | *c*-Pr | H | Br | Me | O |
| D-4531 | Me(S) | single bond | H | *c*-Pr | H | Br | Me | O |
| D-4532 | H | CH₂ | Me | *c*-Pr | H | Br | Me | O |
| D-4533 | Me | CH₂ | H | *c*-Pr | H | Br | Me | O |
| D-4534 | H | CH(Me) | H | *c*-Pr | H | Br | Me | O |
| D-4535 | H | CH₂CH₂ | Me | *c*-Pr | H | Br | Me | O |
| D-4536 | Me | CH₂CH₂ | H | *c*-Pr | H | Br | Me | O |
| D-4537 | H | single bond | Me | *c*-Pr | H | CF₃ | Me | O |
| D-4538 | Me | single bond | H | *c*-Pr | H | CF₃ | Me | O |
| D-4539 | Me(R) | single bond | H | *c*-Pr | H | CF₃ | Me | O |
| D-4540 | Me(S) | single bond | H | *c*-Pr | H | CF₃ | Me | O |
| D-4541 | H | CH₂ | Me | *c*-Pr | H | CF₃ | Me | O |
| D-4542 | Me | CH₂ | H | *c*-Pr | H | CF₃ | Me | O |
| D-4543 | H | CH(Me) | H | *c*-Pr | H | CF₃ | Me | O |
| D-4544 | H | CH₂CH₂ | Me | *c*-Pr | H | CF₃ | Me | O |
| D-4545 | Me | CH₂CH₂ | H | *c*-Pr | H | CF₃ | Me | O |
| D-4546 | H | single bond | Me | *c*-Pr | H | CF₃ | Me | O |
| D-4547 | Me | single bond | H | *c*-Pr | H | CF₃ | Me | O |
| D-4548 | Me(R) | single bond | H | *c*-Pr | H | CF₃ | Me | O |
| D-4549 | Me(S) | single bond | H | *c*-Pr | H | CF₃ | Me | O |
| D-4550 | H | CH₂ | Me | *c*-Pr | H | CF₃ | Me | O |
| D-4551 | Me | CH₂ | H | *c*-Pr | H | CF₃ | Me | O |
| D-4552 | H | CH(Me) | H | *c*-Pr | H | CF₃ | Me | O |
| D-4553 | H | CH₂CH₂ | Me | *c*-Pr | H | CF₃ | Me | O |
| D-4554 | Me | CH₂CH₂ | H | *c*-Pr | H | CF₃ | Me | O |
| D-4555 | H | single bond | Me | *c*-Pr | H | CF₃ | Me | O |
| D-4556 | Me | single bond | H | *c*-Pr | H | CF₃ | Me | O |
| D-4557 | Me(R) | single bond | H | *c*-Pr | H | CF₃ | Me | O |
| D-4558 | Me(S) | single bond | H | *c*-Pr | H | CF₃ | Me | O |
| D-4559 | H | CH₂ | Me | *c*-Pr | H | CF₃ | Me | O |
| D-4560 | Me | CH₂ | H | *c*-Pr | H | CF₃ | Me | O |
| D-4561 | H | CH(Me) | H | *c*-Pr | H | CF₃ | Me | O |
| D-4562 | H | CH₂CH₂ | Me | *c*-Pr | H | CF₃ | Me | O |
| D-4563 | Me | CH₂CH₂ | H | *c*-Pr | H | CF₃ | Me | O |
| D-4564 | H | single bond | Me | *c*-Pr | H | CF₃ | Me | O |
| D-4565 | Me | single bond | H | *c*-Pr | H | CF₃ | Me | O |
| D-4566 | Me(R) | single bond | H | *c*-Pr | H | CF₃ | Me | O |
| D-4567 | Me(S) | single bond | H | *c*-Pr | H | CF₃ | Me | O |
| D-4568 | H | CH₂ | Me | *c*-Pr | H | CF₃ | Me | O |
| D-4569 | Me | CH₂ | H | *c*-Pr | H | CF₃ | Me | O |
| D-4570 | H | CH(Me) | H | *c*-Pr | H | CF₃ | Me | O |

**[Table 749]**

| Compound Number | Y | W | Z | R¹ | R² | R³ | R¹⁰ | A |
|---|---|---|---|---|---|---|---|---|
| D-4571 | H | CH₂CH₂ | Me | *c*-Pr | H | CF₃ | Me | O |
| D-4572 | Me | CH₂CH₂ | H | *c*-Pr | H | CF₃ | Me | O |
| D-4573 | H | single bond | Me | *c*-Pr | H | OMe | Me | O |
| D-4574 | Me | single bond | H | *c*-Pr | H | OMe | Me | O |
| D-4575 | Me(R) | single bond | H | *c*-Pr | H | OMe | Me | O |
| D-4576 | Me(S) | single bond | H | *c*-Pr | H | OMe | Me | O |
| D-4577 | H | CH₂ | Me | *c*-Pr | H | OMe | Me | O |
| D-4578 | Me | CH₂ | H | *c*-Pr | H | OMe | Me | O |
| D-4579 | H | CH(Me) | H | *c*-Pr | H | OMe | Me | O |
| D-4580 | H | CH₂CH₂ | Me | *c*-Pr | H | OMe | Me | O |
| D-4581 | Me | CH₂CH₂ | H | *c*-Pr | H | OMe | Me | O |
| D-4582 | H | single bond | Me | *c*-Pr | H | OMe | Me | O |
| D-4583 | Me | single bond | H | *c*-Pr | H | OMe | Me | O |
| D-4584 | Me(R) | single bond | H | *c*-Pr | H | OMe | Me | O |
| D-4585 | Me(S) | single bond | H | *c*-Pr | H | OMe | Me | O |
| D-4586 | H | CH₂ | Me | *c*-Pr | H | OMe | Me | O |
| D-4587 | Me | CH₂ | H | *c*-Pr | H | OMe | Me | O |
| D-4588 | H | CH(Me) | H | *c*-Pr | H | OMe | Me | O |
| D-4589 | H | CH₂CH₂ | Me | *c*-Pr | H | OMe | Me | O |
| D-4590 | Me | CH₂CH₂ | H | *c*-Pr | H | OMe | Me | O |
| D-4591 | H | single bond | Me | *c*-Pr | H | OMe | Me | O |
| D-4592 | Me | single bond | H | *c*-Pr | H | OMe | Me | O |
| D-4593 | Me(R) | single bond | H | *c*-Pr | H | OMe | Me | O |
| D-4594 | Me(S) | single bond | H | *c*-Pr | H | OMe | Me | O |
| D-4595 | H | CH₂ | Me | *c*-Pr | H | OMe | Me | O |
| D-4596 | Me | CH₂ | H | *c*-Pr | H | OMe | Me | O |
| D-4597 | H | CH(Me) | H | *c*-Pr | H | OMe | Me | O |
| D-4598 | H | CH₂CH₂ | Me | *c*-Pr | H | OMe | Me | O |
| D-4599 | Me | CH₂CH₂ | H | *c*-Pr | H | OMe | Me | O |
| D-4600 | H | single bond | Me | *c*-Pr | H | OMe | Me | O |
| D-4601 | Me | single bond | H | *c*-Pr | H | OMe | Me | O |
| D-4602 | Me(R) | single bond | H | *c*-Pr | H | OMe | Me | O |
| D-4603 | Me(S) | single bond | H | *c*-Pr | H | OMe | Me | O |
| D-4604 | H | CH₂ | Me | *c*-Pr | H | OMe | Me | O |
| D-4605 | Me | CH₂ | H | *c*-Pr | H | OMe | Me | O |
| D-4606 | H | CH(Me) | H | *c*-Pr | H | OMe | Me | O |
| D-4607 | H | CH₂CH₂ | Me | *c*-Pr | H | OMe | Me | O |
| D-4608 | Me | CH₂CH₂ | H | *c*-Pr | H | OMe | Me | O |
| D-4609 | H | single bond | Me | *c*-Pr | H | OCF₃ | Me | O |
| D-4610 | Me | single bond | H | *c*-Pr | H | OCF₃ | Me | O |
| D-4611 | Me(R) | single bond | H | *c*-Pr | H | OCF₃ | Me | O |
| D-4612 | Me(S) | single bond | H | *c*-Pr | H | OCF₃ | Me | O |
| D-4613 | H | CH₂ | Me | *c*-Pr | H | OCF₃ | Me | O |
| D-4614 | Me | CH₂ | H | *c*-Pr | H | OCF₃ | Me | O |

**[Table 750]**

| Compound Number | Y | W | Z | R¹ | R² | R³ | R¹⁰ | A |
|---|---|---|---|---|---|---|---|---|
| D-4615 | H | CH(Me) | H | *c*-Pr | H | OCF₃ | Me | O |
| D-4616 | H | CH₂CH₂ | Me | *c*-Pr | H | OCF₃ | Me | O |
| D-4617 | Me | CH₂CH₂ | H | *c*-Pr | H | OCF₃ | Me | O |
| D-4618 | H | single bond | Me | *c*-Pr | H | OCF₃ | Me | O |
| D-4619 | Me | single bond | H | *c*-Pr | H | OCF₃ | Me | O |
| D-4620 | Me(R) | single bond | H | *c*-Pr | H | OCF₃ | Me | O |
| D-4621 | Me(S) | single bond | H | *c*-Pr | H | OCF₃ | Me | O |
| D-4622 | H | CH₂ | Me | *c*-Pr | H | OCF₃ | Me | O |
| D-4623 | Me | CH₂ | H | *c*-Pr | H | OCF₃ | Me | O |
| D-4624 | H | CH(Me) | H | *c*-Pr | H | OCF₃ | Me | O |
| D-4625 | H | CH₂CH₂ | Me | *c*-Pr | H | OCF₃ | Me | O |
| D-4626 | Me | CH₂CH₂ | H | *c*-Pr | H | OCF₃ | Me | O |
| D-4627 | H | single bond | Me | *c*-Pr | H | OCF₃ | Me | O |
| D-4628 | Me | single bond | H | *c*-Pr | H | OCF₃ | Me | O |
| D-4629 | Me(R) | single bond | H | *c*-Pr | H | OCF₃ | Me | O |
| D-4630 | Me(S) | single bond | H | *c*-Pr | H | OCF₃ | Me | O |
| D-4631 | H | CH₂ | Me | *c*-Pr | H | OCF₃ | Me | O |
| D-4632 | Me | CH₂ | H | *c*-Pr | H | OCF₃ | Me | O |
| D-4633 | H | CH(Me) | H | *c*-Pr | H | OCF₃ | Me | O |
| D-4634 | H | CH₂CH₂ | Me | *c*-Pr | H | OCF₃ | Me | O |
| D-4635 | Me | CH₂CH₂ | H | *c*-Pr | H | OCF₃ | Me | O |
| D-4636 | H | single bond | Me | *c*-Pr | H | OCF₃ | Me | O |
| D-4637 | Me | single bond | H | *c*-Pr | H | OCF₃ | Me | O |
| D-4638 | Me(R) | single bond | H | *c*-Pr | H | OCF₃ | Me | O |
| D-4639 | Me(S) | single bond | H | *c*-Pr | H | OCF₃ | Me | O |
| D-4640 | H | CH₂ | Me | *c*-Pr | H | OCF₃ | Me | O |
| D-4641 | Me | CH₂ | H | *c*-Pr | H | OCF₃ | Me | O |
| D-4642 | H | CH(Me) | H | *c*-Pr | H | OCF₃ | Me | O |
| D-4643 | H | CH₂CH₂ | Me | *c*-Pr | H | OCF₃ | Me | O |
| D-4644 | Me | CH₂CH₂ | H | *c*-Pr | H | OCF₃ | Me | O |
| D-4645 | H | single bond | Me | *c*-Pr | H | Br | CH₂OMe | O |
| D-4646 | Me | single bond | H | *c*-Pr | H | Br | CH₂OMe | O |
| D-4647 | Me(R) | single bond | H | *c*-Pr | H | Br | CH₂OMe | O |
| D-4648 | Me(S) | single bond | H | *c*-Pr | H | Br | CH₂OMe | O |
| D-4649 | H | CH₂ | Me | *c*-Pr | H | Br | CH₂OMe | O |
| D-4650 | Me | CH₂ | H | *c*-Pr | H | Br | CH₂OMe | O |
| D-4651 | H | CH(Me) | H | *c*-Pr | H | Br | CH₂OMe | O |
| D-4652 | H | CH₂CH₂ | Me | *c*-Pr | H | Br | CH₂OMe | O |
| D-4653 | Me | CH₂CH₂ | H | *c*-Pr | H | Br | CH₂OMe | O |
| D-4654 | H | single bond | Me | *c*-Pr | H | Br | CH₂OMe | O |
| D-4655 | Me | single bond | H | *c*-Pr | H | Br | CH₂OMe | O |
| D-4656 | Me(R) | single bond | H | *c*-Pr | H | Br | CH₂OMe | O |
| D-4657 | Me(S) | single bond | H | *c*-Pr | H | Br | CH₂OMe | O |
| D-4658 | H | CH₂ | Me | *c*-Pr | H | Br | CH₂OMe | O |

**[Table 751]**

| Compound Number | Y | W | Z | R¹ | R² | R³ | R¹⁰ | A |
|---|---|---|---|---|---|---|---|---|
| D-4659 | Me | CH₂ | H | *c*-Pr | H | Br | CH₂OMe | O |
| D-4660 | H | CH(Me) | H | *c*-Pr | H | Br | CH₂OMe | O |
| D-4661 | H | CH₂CH₂ | Me | *c*-Pr | H | Br | CH₂OMe | O |
| D-4662 | Me | CH₂CH₂ | H | *c*-Pr | H | Br | CH₂OMe | O |
| D-4663 | H | single bond | Me | *c*-Pr | H | Br | CH₂OMe | O |
| D-4664 | Me | single bond | H | *c*-Pr | H | Br | CH₂OMe | O |
| D-4665 | Me(R) | single bond | H | *c*-Pr | H | Br | CH₂OMe | O |
| D-4666 | Me(S) | single bond | H | *c*-Pr | H | Br | CH₂OMe | O |
| D-4667 | H | CH₂ | Me | *c*-Pr | H | Br | CH₂OMe | O |
| D-4668 | Me | CH₂ | H | *c*-Pr | H | Br | CH₂OMe | O |
| D-4669 | H | CH(Me) | H | *c*-Pr | H | Br | CH₂OMe | O |
| D-4670 | H | CH₂CH₂ | Me | *c*-Pr | H | Br | CH₂OMe | O |
| D-4671 | Me | CH₂CH₂ | H | *c*-Pr | H | Br | CH₂OMe | O |
| D-4672 | H | single bond | Me | *c*-Pr | H | Br | CH₂OMe | O |
| D-4673 | Me | single bond | H | *c*-Pr | H | Br | CH₂OMe | O |
| D-4674 | Me(R) | single bond | H | *c*-Pr | H | Br | CH₂OMe | O |
| D-4675 | Me(S) | single bond | H | *c*-Pr | H | Br | CH₂OMe | O |
| D-4676 | H | CH₂ | Me | *c*-Pr | H | Br | CH₂OMe | O |
| D-4677 | Me | CH₂ | H | *c*-Pr | H | Br | CH₂OMe | O |
| D-4678 | H | CH(Me) | H | *c*-Pr | H | Br | CH₂OMe | O |
| D-4679 | H | CH₂CH₂ | Me | *c*-Pr | H | Br | CH₂OMe | O |
| D-4680 | Me | CH₂CH₂ | H | *c*-Pr | H | Br | CH₂OMe | O |
| D-4681 | H | single bond | Me | *c*-Pr | H | CF₃ | CH₂OMe | O |
| D-4682 | Me | single bond | H | *c*-Pr | H | CF₃ | CH₂OMe | O |
| D-4683 | Me(R) | single bond | H | *c*-Pr | H | CF₃ | CH₂OMe | O |
| D-4684 | Me(S) | single bond | H | *c*-Pr | H | CF₃ | CH₂OMe | O |
| D-4685 | H | CH₂ | Me | *c*-Pr | H | CF₃ | CH₂OMe | 0 |
| D-4686 | Me | CH₂ | H | *c*-Pr | H | CF₃ | CH₂OMe | O |
| D-4687 | H | CH(Me) | H | *c*-Pr | H | CF₃ | CH₂OMe | O |
| D-4688 | H | CH₂CH₂ | Me | *c*-Pr | H | CF₃ | CH₂OMe | O |
| D-4689 | Me | CH₂CH₂ | H | *c*-Pr | H | CF₃ | CH₂OMe | O |
| D-4690 | H | single bond | Me | *c*-Pr | H | CF₃ | CH₂OMe | O |
| D-4691 | Me | single bond | H | *c*-Pr | H | CF₃ | CH₂OMe | O |
| D-4692 | Me(R) | single bond | H | *c*-Pr | H | CF₃ | CH₂OMe | O |
| D-4693 | Me(S) | single bond | H | *c*-Pr | H | CF₃ | CH₂OMe | O |
| D-4694 | H | CH₂ | Me | *c*-Pr | H | CF₃ | CH₂OMe | O |
| D-4695 | Me | CH₂ | H | *c*-Pr | H | CF₃ | CH₂OMe | O |
| D-4696 | H | CH(Me) | H | *c*-Pr | H | CF₃ | CH₂OMe | O |
| D-4697 | H | CH₂CH₂ | Me | *c*-Pr | H | CF₃ | CH₂OMe | O |
| D-4698 | Me | CH₂CH₂ | H | *c*-Pr | H | CF₃ | CH₂OMe | O |
| D-4699 | H | single bond | Me | *c*-Pr | H | CF₃ | CH₂OMe | O |
| D-4700 | Me | single bond | H | *c*-Pr | H | CF₃ | CH₂OMe | O |
| D-4701 | Me(R) | single bond | H | *c*-Pr | H | CF₃ | CH₂OMe | O |
| D-4702 | Me(S) | single bond | H | *c*-Pr | H | CF₃ | CH₂OMe | O |

**[Table 752]**

| Compound Number | Y | W | Z | R¹ | R² | R³ | R¹⁰ | A |
|---|---|---|---|---|---|---|---|---|
| D-4703 | H | CH₂ | Me | *c* -Pr | H | CF₃ | CH₂OMe | O |
| D-4704 | Me | CH₂ | H | *c* -Pr | H | CF₃ | CH₂OMe | O |
| D-4705 | H | CH(Me) | H | *c* -Pr | H | CF₃ | CH₂OMe | O |
| D-4706 | H | CH₂CH₂ | Me | *c* -Pr | H | CF₃ | CH₂OMe | O |
| D-4707 | Me | CH₂CH₂ | H | *c* -Pr | H | CF₃ | CH₂OMe | O |
| D-4708 | H | single bond | Me | *c* -Pr | H | CF₃ | CH₂OMe | O |
| D-4709 | Me | single bond | H | *c* -Pr | H | CF₃ | CH₂OMe | O |
| D-4710 | Me(R) | single bond | H | *c* -Pr | H | CF₃ | CH₂OMe | O |
| D-4711 | Me(S) | single bond | H | *c* -Pr | H | CF₃ | CH₂OMe | O |
| D-4712 | H | CH₂ | Me | *c* -Pr | H | CF₃ | CH₂OMe | O |
| D-4713 | Me | CH₂ | H | *c* -Pr | H | CF₃ | CH₂OMe | O |
| D-4714 | H | CH(Me) | H | *c* -Pr | H | CF₃ | CH₂OMe | O |
| D-4715 | H | CH₂CH₂ | Me | *c* -Pr | H | CF₃ | CH₂OMe | O |
| D-4716 | Me | CH₂CH₂ | H | *c* -Pr | H | CF₃ | CH₂OMe | O |
| D-4717 | H | single bond | Me | *c* -Pr | H | OMe | CH₂OMe | O |
| D-4718 | Me | single bond | H | *c* -Pr | H | OMe | CH₂OMe | O |
| D-4719 | Me(R) | single bond | H | *c* -Pr | H | OMe | CH₂OMe | O |
| D-4720 | Me(S) | single bond | H | *c* -Pr | H | OMe | CH₂OMe | O |
| D-4721 | H | CH₂ | Me | *c* -Pr | H | OMe | CH₂OMe | O |
| D-4722 | Me | CH₂ | H | *c* -Pr | H | OMe | CH₂OMe | O |
| D-4723 | H | CH(Me) | H | *c* -Pr | H | OMe | CH₂OMe | O |
| D-4724 | H | CH₂CH₂ | Me | *c* -Pr | H | OMe | CH₂OMe | O |
| D-4725 | Me | CH₂CH₂ | H | *c* -Pr | H | OMe | CH₂OMe | O |
| D-4726 | H | single bond | Me | *c* -Pr | H | OMe | CH₂OMe | O |
| D-4727 | Me | single bond | H | *c* -Pr | H | OMe | CH₂OMe | O |
| D-4728 | Me(R) | single bond | H | *c* -Pr | H | OMe | CH₂OMe | O |
| D-4729 | Me(S) | single bond | H | *c* -Pr | H | OMe | CH₂OMe | O |
| D-4730 | H | CH₂ | Me | *c* -Pr | H | OMe | CH₂OMe | O |
| D-4731 | Me | CH₂ | H | *c* -Pr | H | OMe | CH₂OMe | O |
| D-4732 | H | CH(Me) | H | *c* -Pr | H | OMe | CH₂OMe | O |
| D-4733 | H | CH₂CH₂ | Me | *c* -Pr | H | OMe | CH₂OMe | O |
| D-4734 | Me | CH₂CH₂ | H | *c* -Pr | H | OMe | CH₂OMe | O |
| D-4735 | H | single bond | Me | *c* -Pr | H | OMe | CH₂OMe | O |
| D-4736 | Me | single bond | H | *c* -Pr | H | OMe | CH₂OMe | O |
| D-4737 | Me(R) | single bond | H | *c* -Pr | H | OMe | CH₂OMe | O |
| D-4738 | Me(S) | single bond | H | *c* -Pr | H | OMe | CH₂OMe | O |
| D-4739 | H | CH₂ | Me | *c* -Pr | H | OMe | CH₂OMe | O |
| D-4740 | Me | CH₂ | H | *c* -Pr | H | OMe | CH₂OMe | O |
| D-4741 | H | CH(Me) | H | *c* -Pr | H | OMe | CH₂OMe | O |
| D-4742 | H | CH₂CH₂ | Me | *c* -Pr | H | OMe | CH₂OMe | O |
| D-4743 | Me | CH₂CH₂ | H | *c* -Pr | H | OMe | CH₂OMe | O |
| D-4744 | H | single bond | Me | *c* -Pr | H | OMe | CH₂OMe | O |
| D-4745 | Me | single bond | H | *c* -Pr | H | OMe | CH₂OMe | O |
| D-4746 | Me(R) | single bond | H | *c* -Pr | H | OMe | CH₂OMe | O |

**[Table 753]**

| Compound Number | Y | W | Z | R¹ | R² | R³ | R¹⁰ | A |
|---|---|---|---|---|---|---|---|---|
| D-4747 | Me(S) | single bond | H | *c* -Pr | H | OMe | CH₂OMe | O |
| D-4748 | H | CH₂ | Me | *c* -Pr | H | OMe | CH₂OMe | O |
| D-4749 | Me | CH₂ | H | *c* -Pr | H | OMe | CH₂OMe | O |
| D-4750 | H | CH(Me) | H | *c* -Pr | H | OMe | CH₂OMe | O |
| D-4751 | H | CH₂CH₂ | Me | *c* -Pr | H | OMe | CH₂OMe | O |
| D-4752 | Me | CH₂CH₂ | H | *c* -Pr | H | OMe | CH₂OMe | O |
| D-4753 | H | single bond | Me | *c* -Pr | H | OCF₃ | CH₂OMe | O |
| D-4754 | Me | single bond | H | *c* -Pr | H | OCF₃ | CH₂OMe | O |
| D-4755 | Me(R) | single bond | H | *c* -Pr | H | OCF₃ | CH₂OMe | O |
| D-4756 | Me(S) | single bond | H | *c* -Pr | H | OCF₃ | CH₂OMe | O |
| D-4757 | H | CH₂ | Me | *c* -Pr | H | OCF₃ | CH₂OMe | O |
| D-4758 | Me | CH₂ | H | *c* -Pr | H | OCF₃ | CH₂OMe | O |
| D-4759 | H | CH(Me) | H | *c* -Pr | H | OCF₃ | CH₂OMe | O |
| D-4760 | H | CH₂CH₂ | Me | *c* -Pr | H | OCF₃ | CH₂OMe | O |
| D-4761 | Me | CH₂CH₂ | H | *c* -Pr | H | OCF₃ | CH₂OMe | O |
| D-4762 | H | single bond | Me | *c* -Pr | H | OCF₃ | CH₂OMe | O |
| D-4763 | Me | single bond | H | *c* -Pr | H | OCF₃ | CH₂OMe | O |
| D-4764 | Me(R) | single bond | H | *c* -Pr | H | OCF₃ | CH₂OMe | O |
| D-4765 | Me(S) | single bond | H | *c* -Pr | H | OCF₃ | CH₂OMe | O |
| D-4766 | H | CH₂ | Me | *c* -Pr | H | OCF₃ | CH₂OMe | O |
| D-4767 | Me | CH₂ | H | *c* -Pr | H | OCF₃ | CH₂OMe | O |
| D-4768 | H | CH(Me) | H | *c* -Pr | H | OCF₃ | CH₂OMe | O |
| D-4769 | H | CH₂CH₂ | Me | *c* -Pr | H | OCF₃ | CH₂OMe | O |
| D-4770 | Me | CH₂CH₂ | H | *c* -Pr | H | OCF₃ | CH₂OMe | O |
| D-4771 | H | single bond | Me | *c* -Pr | H | OCF₃ | CH₂OMe | O |
| D-4772 | Me | single bond | H | *c* -Pr | H | OCF₃ | CH₂OMe | O |
| D-4773 | Me(R) | single bond | H | *c* -Pr | H | OCF₃ | CH₂OMe | O |
| D-4774 | Me(S) | single bond | H | *c* -Pr | H | OCF₃ | CH₂OMe | O |
| D-4775 | H | CH₂ | Me | *c* -Pr | H | OCF₃ | CH₂OMe | O |
| D-4776 | Me | CH₂ | H | *c* -Pr | H | OCF₃ | CH₂OMe | O |
| D-4777 | H | CH(Me) | H | *c* -Pr | H | OCF₃ | CH₂OMe | O |
| D-4778 | H | CH₂CH₂ | Me | *c* -Pr | H | OCF₃ | CH₂OMe | O |
| D-4779 | Me | CH₂CH₂ | H | *c* -Pr | H | OCF₃ | CH₂OMe | O |
| D-4780 | H | single bond | Me | *c* -Pr | H | OCF₃ | CH₂OMe | O |
| D-4781 | Me | single bond | H | *c* -Pr | H | OCF₃ | CH₂OMe | O |
| D-4782 | Me(R) | single bond | H | *c* -Pr | H | OCF₃ | CH₂OMe | O |
| D-4783 | Me(S) | single bond | H | *c* -Pr | H | OCF₃ | CH₂OMe | O |
| D-4784 | H | CH₂ | Me | *c* -Pr | H | OCF₃ | CH₂OMe | O |
| D-4785 | Me | CH₂ | H | *c* -Pr | H | OCF₃ | CH₂OMe | O |
| D-4786 | H | CH(Me) | H | *c* -Pr | H | OCF₃ | CH₂OMe | O |
| D-4787 | H | CH₂CH₂ | Me | *c* -Pr | H | OCF₃ | CH₂OMe | O |
| D-4788 | Me | CH₂CH₂ | H | *c* -Pr | H | OCF₃ | CH₂OMe | O |
| D-4789 | H | single bond | Me | *c* -Pr | H | Br | CH₂CN | O |
| D-4790 | Me | single bond | H | *c* -Pr | H | Br | CH₂CN | O |

**[Table 754]**

| Compound Number | Y | W | Z | R¹ | R² | R³ | R¹⁰ | A |
|---|---|---|---|---|---|---|---|---|
| D-4791 | Me(R) | single bond | H | *c* -Pr | H | Br | CH₂CN | O |
| D-4792 | Me(S) | single bond | H | *c* -Pr | H | Br | CH₂CN | O |
| D-4793 | H | CH₂ | Me | *c* -Pr | H | Br | CH₂CN | O |
| D-4794 | Me | CH₂ | H | *c* -Pr | H | Br | CH₂CN | O |
| D-4795 | H | CH(Me) | H | *c* -Pr | H | Br | CH₂CN | O |
| D-4796 | H | CH₂CH₂ | Me | *c* -Pr | H | Br | CH₂CN | O |
| D-4797 | Me | CH₂CH₂ | H | *c* -Pr | H | Br | CH₂CN | O |
| D-4798 | H | single bond | Me | *c* -Pr | H | Br | CH₂CN | O |
| D-4799 | Me | single bond | H | *c* -Pr | H | Br | CH₂CN | O |
| D-4800 | Me(R) | single bond | H | *c* -Pr | H | Br | CH₂CN | O |
| D-4801 | Me(S) | single bond | H | *c* -Pr | H | Br | CH₂CN | O |
| D-4802 | H | CH₂ | Me | *c* -Pr | H | Br | CH₂CN | O |
| D-4803 | Me | CH₂ | H | *c* -Pr | H | Br | CH₂CN | O |
| D-4804 | H | CH(Me) | H | *c* -Pr | H | Br | CH₂CN | O |
| D-4805 | H | CH₂CH₂ | Me | *c* -Pr | H | Br | CH₂CN | O |
| D-4806 | Me | CH₂CH₂ | H | *c* -Pr | H | Br | CH₂CN | O |
| D-4807 | H | single bond | Me | *c* -Pr | H | Br | CH₂CN | O |
| D-4808 | Me | single bond | H | *c* -Pr | H | Br | CH₂CN | O |
| D-4809 | Me(R) | single bond | H | *c* -Pr | H | Br | CH₂CN | O |
| D-4810 | Me(S) | single bond | H | *c* -Pr | H | Br | CH₂CN | O |
| D-4811 | H | CH₂ | Me | *c* -Pr | H | Br | CH₂CN | O |
| D-4812 | Me | CH₂ | H | *c* -Pr | H | Br | CH₂CN | O |
| D-4813 | H | CH(Me) | H | *c* -Pr | H | Br | CH₂CN | O |
| D-4814 | H | CH₂CH₂ | Me | *c* -Pr | H | Br | CH₂CN | O |
| D-4815 | Me | CH₂CH₂ | H | *c* -Pr | H | Br | CH₂CN | O |
| D-4816 | H | single bond | Me | *c* -Pr | H | Br | CH₂CN | O |
| D-4817 | Me | single bond | H | *c* -Pr | H | Br | CH₂CN | O |
| D-4818 | Me(R) | single bond | H | *c* -Pr | H | Br | CH₂CN | O |
| D-4819 | Me(S) | single bond | H | *c* -Pr | H | Br | CH₂CN | O |
| D-4820 | H | CH₂ | Me | *c* -Pr | H | Br | CH₂CN | O |
| D-4821 | Me | CH₂ | H | *c* -Pr | H | Br | CH₂CN | O |
| D-4822 | H | CH(Me) | H | *c* -Pr | H | Br | CH₂CN | O |
| D-4823 | H | CH₂CH₂ | Me | *c* -Pr | H | Br | CH₂CN | O |
| D-4824 | Me | CH₂CH₂ | H | *c* -Pr | H | Br | CH₂CN | O |
| D-4825 | H | single bond | Me | *c* -Pr | H | CF₃ | CH₂CN | O |
| D-4826 | Me | single bond | H | *c* -Pr | H | CF₃ | CH₂CN | O |
| D-4827 | Me(R) | single bond | H | *c* -Pr | H | CF₃ | CH₂CN | O |
| D-4828 | Me(S) | single bond | H | *c* -Pr | H | CF₃ | CH₂CN | O |
| D-4829 | H | CH₂ | Me | *c* -Pr | H | CF₃ | CH₂CN | O |
| D-4830 | Me | CH₂ | H | *c* -Pr | H | CF₃ | CH₂CN | O |
| D-4831 | H | CH(Me) | H | *c* -Pr | H | CF₃ | CH₂CN | O |
| D-4832 | H | CH₂CH₂ | Me | *c* -Pr | H | CF₃ | CH₂CN | O |
| D-4833 | Me | CH₂CH₂ | H | *c* -Pr | H | CF₃ | CH₂CN | O |
| D-4834 | H | single bond | Me | *c* -Pr | H | CF₃ | CH₂CN | O |

**[Table 755]**

| Compound Number | Y | W | Z | R¹ | R² | R³ | R¹⁰ | A |
|---|---|---|---|---|---|---|---|---|
| D-4835 | Me | single bond | H | *c* -Pr | H | CF₃ | CH₂CN | O |
| D-4836 | Me(R) | single bond | H | *c* -Pr | H | CF₃ | CH₂CN | O |
| D-4837 | Me(S) | single bond | H | *c* -Pr | H | CF₃ | CH₂CN | O |
| D-4838 | H | CH₂ | Me | *c* -Pr | H | CF₃ | CH₂CN | O |
| D-4839 | Me | CH₂ | H | *c* -Pr | H | CF₃ | CH₂CN | O |
| D-4840 | H | CH(Me) | H | *c* -Pr | H | CF₃ | CH₂CN | O |
| D-4841 | H | CH₂CH₂ | Me | *c* -Pr | H | CF₃ | CH₂CN | O |
| D-4842 | Me | CH₂CH₂ | H | *c* -Pr | H | CF₃ | CH₂CN | O |
| D-4843 | H | single bond | Me | *c* -Pr | H | CF₃ | CH₂CN | O |
| D-4844 | Me | single bond | H | *c* -Pr | H | CF₃ | CH₂CN | O |
| D-4845 | Me(R) | single bond | H | *c* -Pr | H | CF₃ | CH₂CN | O |
| D-4846 | Me(S) | single bond | H | *c* -Pr | H | CF₃ | CH₂CN | O |
| D-4847 | H | CH₂ | Me | *c* -Pr | H | CF₃ | CH₂CN | O |
| D-4848 | Me | CH₂ | H | *c* -Pr | H | CF₃ | CH₂CN | O |
| D-4849 | H | CH(Me) | H | *c* -Pr | H | CF₃ | CH₂CN | O |
| D-4850 | H | CH₂CH₂ | Me | *c* -Pr | H | CF₃ | CH₂CN | O |
| D-4851 | Me | CH₂CH₂ | H | *c* -Pr | H | CF₃ | CH₂CN | O |
| D-4852 | H | single bond | Me | *c* -Pr | H | CF₃ | CH₂CN | O |
| D-4853 | Me | single bond | H | *c* -Pr | H | CF₃ | CH₂CN | O |
| D-4854 | Me(R) | single bond | H | *c* -Pr | H | CF₃ | CH₂CN | O |
| D-4855 | Me(S) | single bond | H | *c* -Pr | H | CF₃ | CH₂CN | O |
| D-4856 | H | CH₂ | Me | *c* -Pr | H | CF₃ | CH₂CN | O |
| D-4857 | Me | CH₂ | H | *c* -Pr | H | CF₃ | CH₂CN | O |
| D-4858 | H | CH(Me) | H | *c* -Pr | H | CF₃ | CH₂CN | O |
| D-4859 | H | CH₂CH₂ | Me | *c* -Pr | H | CF₃ | CH₂CN | O |
| D-4860 | Me | CH₂CH₂ | H | *c* -Pr | H | CF₃ | CH₂CN | O |
| D-4861 | H | single bond | Me | *c* -Pr | H | OMe | CH₂CN | O |
| D-4862 | Me | single bond | H | *c* -Pr | H | OMe | CH₂CN | O |
| D-4863 | Me(R) | single bond | H | *c* -Pr | H | OMe | CH₂CN | O |
| D-4864 | Me(S) | single bond | H | *c* -Pr | H | OMe | CH₂CN | O |
| D-4865 | H | CH₂ | Me | *c* -Pr | H | OMe | CH₂CN | O |
| D-4866 | Me | CH₂ | H | *c* -Pr | H | OMe | CH₂CN | O |
| D-4867 | H | CH(Me) | H | *c* -Pr | H | OMe | CH₂CN | O |
| D-4868 | H | CH₂CH₂ | Me | *c* -Pr | H | OMe | CH₂CN | O |
| D-4869 | Me | CH₂CH₂ | H | *c* -Pr | H | OMe | CH₂CN | O |
| D-4870 | H | single bond | Me | *c* -Pr | H | OMe | CH₂CN | O |
| D-4871 | Me | single bond | H | *c* -Pr | H | OMe | CH₂CN | O |
| D-4872 | Me(R) | single bond | H | *c* -Pr | H | OMe | CH₂CN | O |
| D-4873 | Me(S) | single bond | H | *c* -Pr | H | OMe | CH₂CN | O |
| D-4874 | H | CH₂ | Me | *c* -Pr | H | OMe | CH₂CN | O |
| D-4875 | Me | CH₂ | H | *c* -Pr | H | OMe | CH₂CN | O |
| D-4876 | H | CH(Me) | H | *c* -Pr | H | OMe | CH₂CN | O |
| D-4877 | H | CH₂CH₂ | Me | *c* -Pr | H | OMe | CH₂CN | O |
| D-4878 | Me | CH₂CH₂ | H | *c* -Pr | H | OMe | CH₂CN | O |

**[Table 756]**

| Compound Number | Y | W | Z | R¹ | R² | R³ | R¹⁰ | A |
|---|---|---|---|---|---|---|---|---|
| D-4879 | H | single bond | Me | *c* -Pr | H | OMe | CH₂CN | O |
| D-4880 | Me | single bond | H | *c* -Pr | H | OMe | CH₂CN | O |
| D-4881 | Me(R) | single bond | H | *c* -Pr | H | OMe | CH₂CN | O |
| D-4882 | Me(S) | single bond | H | *c* -Pr | H | OMe | CH₂CN | O |
| D-4883 | H | CH₂ | Me | *c* -Pr | H | OMe | CH₂CN | O |
| D-4884 | Me | CH₂ | H | *c* -Pr | H | OMe | CH₂CN | O |
| D-4885 | H | CH(Me) | H | *c* -Pr | H | OMe | CH₂CN | O |
| D-4886 | H | CH₂CH₂ | Me | *c* -Pr | H | OMe | CH₂CN | O |
| D-4887 | Me | CH₂CH₂ | H | *c* -Pr | H | OMe | CH₂CN | O |
| D-4888 | H | single bond | Me | *c* -Pr | H | OMe | CH₂CN | O |
| D-4889 | Me | single bond | H | *c* -Pr | H | OMe | CH₂CN | O |
| D-4890 | Me(R) | single bond | H | *c* -Pr | H | OMe | CH₂CN | O |
| D-4891 | Me(S) | single bond | H | *c* -Pr | H | OMe | CH₂CN | O |
| D-4892 | H | CH₂ | Me | *c* -Pr | H | OMe | CH₂CN | O |
| D-4893 | Me | CH₂ | H | *c* -Pr | H | OMe | CH₂CN | O |
| D-4894 | H | CH(Me) | H | *c* -Pr | H | OMe | CH₂CN | O |
| D-4895 | H | CH₂CH₂ | Me | *c* -Pr | H | OMe | CH₂CN | O |
| D-4896 | Me | CH₂CH₂ | H | *c* -Pr | H | OMe | CH₂CN | O |
| D-4897 | H | single bond | Me | *c* -Pr | H | OCF₃ | CH₂CN | O |
| D-4898 | Me | single bond | H | *c* -Pr | H | OCF₃ | CH₂CN | O |
| D-4899 | Me(R) | single bond | H | *c* -Pr | H | OCF₃ | CH₂CN | O |
| D-4900 | Me(S) | single bond | H | *c* -Pr | H | OCF₃ | CH₂CN | O |
| D-4901 | H | CH₂ | Me | *c* -Pr | H | OCF₃ | CH₂CN | O |
| D-4902 | Me | CH₂ | H | *c* -Pr | H | OCF₃ | CH₂CN | O |
| D-4903 | H | CH(Me) | H | *c* -Pr | H | OCF₃ | CH₂CN | O |
| D-4904 | H | CH₂CH₂ | Me | *c* -Pr | H | OCF₃ | CH₂CN | O |
| D-4905 | Me | CH₂CH₂ | H | *c* -Pr | H | OCF₃ | CH₂CN | O |
| D-4906 | H | single bond | Me | *c* -Pr | H | OCF₃ | CH₂CN | O |
| D-4907 | Me | single bond | H | *c* -Pr | H | OCF₃ | CH₂CN | O |
| D-4908 | Me(R) | single bond | H | *c* -Pr | H | OCF₃ | CH₂CN | O |
| D-4909 | Me(S) | single bond | H | *c* -Pr | H | OCF₃ | CH₂CN | O |
| D-4910 | H | CH₂ | Me | *c* -Pr | H | OCF₃ | CH₂CN | O |
| D-4911 | Me | CH₂ | H | *c* -Pr | H | OCF₃ | CH₂CN | O |
| D-4912 | H | CH(Me) | H | *c* -Pr | H | OCF₃ | CH₂CN | O |
| D-4913 | H | CH₂CH₂ | Me | *c* -Pr | H | OCF₃ | CH₂CN | O |
| D-4914 | Me | CH₂CH₂ | H | *c* -Pr | H | OCF₃ | CH₂CN | O |
| D-4915 | H | single bond | Me | *c* -Pr | H | OCF₃ | CH₂CN | O |
| D-4916 | Me | single bond | H | *c* -Pr | H | OCF₃ | CH₂CN | O |
| D-4917 | Me(R) | single bond | H | *c* -Pr | H | OCF₃ | CH₂CN | O |
| D-4918 | Me(S) | single bond | H | *c* -Pr | H | OCF₃ | CH₂CN | O |
| D-4919 | H | CH₂ | Me | *c* -Pr | H | OCF₃ | CH₂CN | O |
| D-4920 | Me | CH₂ | H | *c* -Pr | H | OCF₃ | CH₂CN | O |
| D-4921 | H | CH(Me) | H | *c* -Pr | H | OCF₃ | CH₂CN | O |
| D-4922 | H | CH₂CH₂ | Me | *c* -Pr | H | OCF₃ | CH₂CN | O |

**[Table 757]**

| Compound Number | Y | W | Z | R¹ | R² | R³ | R¹⁰ | A |
|---|---|---|---|---|---|---|---|---|
| D-4923 | Me | CH₂CH₂ | H | *c* -Pr | H | OCF₃ | CH₂CN | O |
| D-4924 | H | single bond | Me | *c* -Pr | H | OCF₃ | CH₂CN | O |
| D-4925 | Me | single bond | H | *c* -Pr | H | OCF₃ | CH₂CN | O |
| D-4926 | Me(R) | single bond | H | *c* -Pr | H | OCF₃ | CH₂CN | O |
| D-4927 | Me(S) | single bond | H | *c* -Pr | H | OCF₃ | CH₂CN | O |
| D-4928 | H | CH₂ | Me | *c* -Pr | H | OCF₃ | CH₂CN | O |
| D-4929 | Me | CH₂ | H | *c* -Pr | H | OCF₃ | CH₂CN | O |
| D-4930 | H | CH(Me) | H | *c* -Pr | H | OCF₃ | CH₂CN | O |
| D-4931 | H | CH₂CH₂ | Me | *c* -Pr | H | OCF₃ | CH₂CN | O |
| D-4932 | Me | CH₂CH₂ | H | *c* -Pr | H | OCF₃ | CH₂CN | O |
| D-4933 | H | single bond | Me | *c* -Pr | Me | Br | H | O |
| D-4934 | Me | single bond | H | *c* -Pr | Me | Br | H | O |
| D-4935 | Me(R) | single bond | H | *c* -Pr | Me | Br | H | O |
| D-4936 | Me(S) | single bond | H | *c* -Pr | Me | Br | H | O |
| D-4937 | H | CH₂ | Me | *c* -Pr | Me | Br | H | O |
| D-4938 | Me | CH₂ | H | *c* -Pr | Me | Br | H | O |
| D-4939 | H | CH(Me) | H | *c* -Pr | Me | Br | H | O |
| D-4940 | H | CH₂CH₂ | Me | *c* -Pr | Me | Br | H | O |
| D-4941 | Me | CH₂CH₂ | H | *c* -Pr | Me | Br | H | O |
| D-4942 | H | single bond | Me | *c* -Pr | Me | Br | H | O |
| D-4943 | Me | single bond | H | *c* -Pr | Me | Br | H | O |
| D-4944 | Me(R) | single bond | H | *c* -Pr | Me | Br | H | O |
| D-4945 | Me(S) | single bond | H | *c* -Pr | Me | Br | H | O |
| D-4946 | H | CH₂ | Me | *c* -Pr | Me | Br | H | O |
| D-4947 | Me | CH₂ | H | *c* -Pr | Me | Br | H | O |
| D-4948 | H | CH(Me) | H | *c* -Pr | Me | Br | H | O |
| D-4949 | H | CH₂CH₂ | Me | *c* -Pr | Me | Br | H | O |
| D-4950 | Me | CH₂CH₂ | H | *c* -Pr | Me | Br | H | O |
| D-4951 | H | single bond | Me | *c* -Pr | Me | Br | H | O |
| D-4952 | Me | single bond | H | *c* -Pr | Me | Br | H | O |
| D-4953 | Me(R) | single bond | H | *c* -Pr | Me | Br | H | O |
| D-4954 | Me(S) | single bond | H | *c* -Pr | Me | Br | H | O |
| D-4955 | H | CH₂ | Me | *c* -Pr | Me | Br | H | O |
| D-4956 | Me | CH₂ | H | *c* -Pr | Me | Br | H | O |
| D-4957 | H | CH(Me) | H | *c* -Pr | Me | Br | H | O |
| D-4958 | H | CH₂CH₂ | Me | *c* -Pr | Me | Br | H | O |
| D-4959 | Me | CH₂CH₂ | H | *c* -Pr | Me | Br | H | O |
| D-4960 | H | single bond | Me | *c* -Pr | Me | Br | H | O |
| D-4961 | Me | single bond | H | *c* -Pr | Me | Br | H | O |
| D-4962 | Me(R) | single bond | H | *c* -Pr | Me | Br | H | O |
| D-4963 | Me(S) | single bond | H | *c* -Pr | Me | Br | H | O |
| D-4964 | H | CH₂ | Me | *c* -Pr | Me | Br | H | O |
| D-4965 | Me | CH₂ | H | *c* -Pr | Me | Br | H | O |
| D-4966 | H | CH(Me) | H | *c* -Pr | Me | Br | H | O |

**[Table 758]**

| Compound Number | Y | W | Z | R¹ | R² | R³ | R¹⁰ | A |
|---|---|---|---|---|---|---|---|---|
| D-4967 | H | CH₂CH₂ | Me | *c*-Pr | Me | Br | H | O |
| D-4968 | Me | CH₂CH₂ | H | *c*-Pr | Me | Br | H | O |
| D-4969 | H | single bond | Me | *c*-Pr | Me | CF₃ | H | O |
| D-4970 | Me | single bond | H | *c*-Pr | Me | CF₃ | H | O |
| D-4971 | Me(R) | single bond | H | *c*-Pr | Me | CF₃ | H | O |
| D-4972 | Me(S) | single bond | H | *c*-Pr | Me | CF₃ | H | O |
| D-4973 | H | CH₂ | Me | *c*-Pr | Me | CF₃ | H | O |
| D-4974 | Me | CH₂ | H | *c*-Pr | Me | CF₃ | H | O |
| D-4975 | H | CH(Me) | H | *c*-Pr | Me | CF₃ | H | O |
| D-4976 | H | CH₂CH₂ | Me | *c*-Pr | Me | CF₃ | H | O |
| D-4977 | Me | CH₂CH₂ | H | *c*-Pr | Me | CF₃ | H | O |
| D-4978 | H | single bond | Me | *c*-Pr | Me | CF₃ | H | O |
| D-4979 | Me | single bond | H | *c*-Pr | Me | CF₃ | H | O |
| D-4980 | Me(R) | single bond | H | *c*-Pr | Me | CF₃ | H | O |
| D-4981 | Me(S) | single bond | H | *c*-Pr | Me | CF₃ | H | O |
| D-4982 | H | CH₂ | Me | *c*-Pr | Me | CF₃ | H | O |
| D-4983 | Me | CH₂ | H | *c*-Pr | Me | CF₃ | H | O |
| D-4984 | H | CH(Me) | H | *c*-Pr | Me | CF₃ | H | O |
| D-4985 | H | CH₂CH₂ | Me | *c*-Pr | Me | CF₃ | H | O |
| D-4986 | Me | CH₂CH₂ | H | *c*-Pr | Me | CF₃ | H | O |
| D-4987 | H | single bond | Me | *c*-Pr | Me | CF₃ | H | O |
| D-4988 | Me | single bond | H | *c*-Pr | Me | CF₃ | H | O |
| D-4989 | Me(R) | single bond | H | *c*-Pr | Me | CF₃ | H | O |
| D-4990 | Me(S) | single bond | H | *c*-Pr | Me | CF₃ | H | O |
| D-4991 | H | CH₂ | Me | *c*-Pr | Me | CF₃ | H | O |
| D-4992 | Me | CH₂ | H | *c*-Pr | Me | CF₃ | H | O |
| D-4993 | H | CH(Me) | H | *c*-Pr | Me | CF₃ | H | O |
| D-4994 | H | CH₂CH₂ | Me | *c*-Pr | Me | CF₃ | H | O |
| D-4995 | Me | CH₂CH₂ | H | *c*-Pr | Me | CF₃ | H | O |
| D-4996 | H | single bond | Me | *c*-Pr | Me | CF₃ | H | O |
| D-4997 | Me | single bond | H | *c*-Pr | Me | CF₃ | H | O |
| D-4998 | Me(R) | single bond | H | *c*-Pr | Me | CF₃ | H | O |
| D-4999 | Me(S) | single bond | H | *c*-Pr | Me | CF₃ | H | O |
| D-5000 | H | CH₂ | Me | *c*-Pr | Me | CF₃ | H | O |
| D-5001 | Me | CH₂ | H | *c*-Pr | Me | CF₃ | H | O |
| D-5002 | H | CH(Me) | H | *c*-Pr | Me | CF₃ | H | O |
| D-5003 | H | CH₂CH₂ | Me | *c*-Pr | Me | CF₃ | H | O |
| D-5004 | Me | CH₂CH₂ | H | *c*-Pr | Me | CF₃ | H | O |
| D-5005 | H | single bond | Me | *c*-Pr | Me | OMe | H | O |
| D-5006 | Me | single bond | H | *c*-Pr | Me | OMe | H | O |
| D-5007 | Me(R) | single bond | H | *c*-Pr | Me | OMe | H | O |
| D-5008 | Me(S) | single bond | H | *c*-Pr | Me | OMe | H | O |
| D-5009 | H | CH₂ | Me | *c*-Pr | Me | OMe | H | O |
| D-5010 | Me | CH₂ | H | *c*-Pr | Me | OMe | H | O |

**[Table 759]**

| Compound Number | Y | W | Z | R¹ | R² | R³ | R¹⁰ | A |
|---|---|---|---|---|---|---|---|---|
| D-5011 | H | CH(Me) | H | *c*-Pr | Me | OMe | H | O |
| D-5012 | H | CH₂CH₂ | Me | *c*-Pr | Me | OMe | H | O |
| D-5013 | Me | CH₂CH₂ | H | *c*-Pr | Me | OMe | H | O |
| D-5014 | H | single bond | Me | *c*-Pr | Me | OMe | H | O |
| D-5015 | Me | single bond | H | *c*-Pr | Me | OMe | H | O |
| D-5016 | Me(R) | single bond | H | *c*-Pr | Me | OMe | H | O |
| D-5017 | Me(S) | single bond | H | *c*-Pr | Me | OMe | H | O |
| D-5018 | H | CH₂ | Me | *c*-Pr | Me | OMe | H | O |
| D-5019 | Me | CH₂ | H | *c*-Pr | Me | OMe | H | O |
| D-5020 | H | CH(Me) | H | *c*-Pr | Me | OMe | H | O |
| D-5021 | H | CH₂CH₂ | Me | *c*-Pr | Me | OMe | H | O |
| D-5022 | Me | CH₂CH₂ | H | *c*-Pr | Me | OMe | H | O |
| D-5023 | H | single bond | Me | *c*-Pr | Me | OMe | H | O |
| D-5024 | Me | single bond | H | *c*-Pr | Me | OMe | H | O |
| D-5025 | Me(R) | single bond | H | *c*-Pr | Me | OMe | H | O |
| D-5026 | Me(S) | single bond | H | *c*-Pr | Me | OMe | H | O |
| D-5027 | H | CH₂ | Me | *c*-Pr | Me | OMe | H | O |
| D-5028 | Me | CH₂ | H | *c*-Pr | Me | OMe | H | O |
| D-5029 | H | CH(Me) | H | *c*-Pr | Me | OMe | H | O |
| D-5030 | H | CH₂CH₂ | Me | *c*-Pr | Me | OMe | H | O |
| D-5031 | Me | CH₂CH₂ | H | *c*-Pr | Me | OMe | H | O |
| D-5032 | H | single bond | Me | *c*-Pr | Me | OMe | H | O |
| D-5033 | Me | single bond | H | *c*-Pr | Me | OMe | H | O |
| D-5034 | Me(R) | single bond | H | *c*-Pr | Me | OMe | H | O |
| D-5035 | Me(S) | single bond | H | *c*-Pr | Me | OMe | H | O |
| D-5036 | H | CH₂ | Me | *c*-Pr | Me | OMe | H | O |
| D-5037 | Me | CH₂ | H | *c*-Pr | Me | OMe | H | O |
| D-5038 | H | CH(Me) | H | *c*-Pr | Me | OMe | H | O |
| D-5039 | H | CH₂CH₂ | Me | *c*-Pr | Me | OMe | H | O |
| D-5040 | Me | CH₂CH₂ | H | *c*-Pr | Me | OMe | H | O |
| D-5041 | H | single bond | Me | *c*-Pr | Me | OCF₃ | H | O |
| D-5042 | Me | single bond | H | *c*-Pr | Me | OCF₃ | H | O |
| D-5043 | Me(R) | single bond | H | *c*-Pr | Me | OCF₃ | H | O |
| D-5044 | Me(S) | single bond | H | *c*-Pr | Me | OCF₃ | H | O |
| D-5045 | H | CH₂ | Me | *c*-Pr | Me | OCF₃ | H | O |
| D-5046 | Me | CH₂ | H | *c*-Pr | Me | OCF₃ | H | O |
| D-5047 | H | CH(Me) | H | *c*-Pr | Me | OCF₃ | H | O |
| D-5048 | H | CH₂CH₂ | Me | *c*-Pr | Me | OCF₃ | H | O |
| D-5049 | Me | CH₂CH₂ | H | *c*-Pr | Me | OCF₃ | H | O |
| D-5050 | H | single bond | Me | *c*-Pr | Me | OCF₃ | H | O |
| D-5051 | Me | single bond | H | *c*-Pr | Me | OCF₃ | H | O |
| D-5052 | Me(R) | single bond | H | *c*-Pr | Me | OCF₃ | H | O |
| D-5053 | Me(S) | single bond | H | *c*-Pr | Me | OCF₃ | H | O |
| D-5054 | H | CH₂ | Me | *c*-Pr | Me | OCF₃ | H | O |

**[Table 760]**

| Compound Number | Y | W | Z | R¹ | R² | R³ | R¹⁰ | A |
|---|---|---|---|---|---|---|---|---|
| D-5055 | Me | CH₂ | H | *c*-Pr | Me | OCF₃ | H | O |
| D-5056 | H | CH(Me) | H | *c*-Pr | Me | OCF₃ | H | O |
| D-5057 | H | CH₂CH₂ | Me | *c*-Pr | Me | OCF₃ | H | O |
| D-5058 | Me | CH₂CH₂ | H | *c*-Pr | Me | OCF₃ | H | O |
| D-5059 | H | single bond | Me | *c*-Pr | Me | OCF₃ | H | O |
| D-5060 | Me | single bond | H | *c*-Pr | Me | OCF₃ | H | O |
| D-5061 | Me(R) | single bond | H | *c*-Pr | Me | OCF₃ | H | O |
| D-5062 | Me(S) | single bond | H | *c*-Pr | Me | OCF₃ | H | O |
| D-5063 | H | CH₂ | Me | *c*-Pr | Me | OCF₃ | H | O |
| D-5064 | Me | CH₂ | H | *c*-Pr | Me | OCF₃ | H | O |
| D-5065 | H | CH(Me) | H | *c*-Pr | Me | OCF₃ | H | O |
| D-5066 | H | CH₂CH₂ | Me | *c*-Pr | Me | OCF₃ | H | O |
| D-5067 | Me | CH₂CH₂ | H | *c*-Pr | Me | OCF₃ | H | O |
| D-5068 | H | single bond | Me | *c*-Pr | Me | OCF₃ | H | O |
| D-5069 | Me | single bond | H | *c*-Pr | Me | OCF₃ | H | O |
| D-5070 | Me(R) | single bond | H | *c*-Pr | Me | OCF₃ | H | O |
| D-5071 | Me(S) | single bond | H | *c*-Pr | Me | OCF₃ | H | O |
| D-5072 | H | CH₂ | Me | *c*-Pr | Me | OCF₃ | H | O |
| D-5073 | Me | CH₂ | H | *c*-Pr | Me | OCF₃ | H | O |
| D-5074 | H | CH(Me) | H | *c*-Pr | Me | OCF₃ | H | O |
| D-5075 | H | CH₂CH₂ | Me | *c*-Pr | Me | OCF₃ | H | O |
| D-5076 | Me | CH₂CH₂ | H | *c*-Pr | Me | OCF₃ | H | O |
| D-5077 | H | single bond | Me | *c*-Pr | Me | Br | Me | O |
| D-5078 | Me | single bond | H | *c*-Pr | Me | Br | Me | O |
| D-5079 | Me(R) | single bond | H | *c*-Pr | Me | Br | Me | O |
| D-5080 | Me(S) | single bond | H | *c*-Pr | Me | Br | Me | O |
| D-5081 | H | CH₂ | Me | *c*-Pr | Me | Br | Me | O |
| D-5082 | Me | CH₂ | H | *c*-Pr | Me | Br | Me | O |
| D-5083 | H | CH(Me) | H | *c*-Pr | Me | Br | Me | O |
| D-5084 | H | CH₂CH₂ | Me | *c*-Pr | Me | Br | Me | O |
| D-5085 | Me | CH₂CH₂ | H | *c*-Pr | Me | Br | Me | O |
| D-5086 | H | single bond | Me | *c*-Pr | Me | Br | Me | O |
| D-5087 | Me | single bond | H | *c*-Pr | Me | Br | Me | O |
| D-5088 | Me(R) | single bond | H | *c*-Pr | Me | Br | Me | O |
| D-5089 | Me(S) | single bond | H | *c*-Pr | Me | Br | Me | O |
| D-5090 | H | CH₂ | Me | *c*-Pr | Me | Br | Me | O |
| D-5091 | Me | CH₂ | H | *c*-Pr | Me | Br | Me | O |
| D-5092 | H | CH(Me) | H | *c*-Pr | Me | Br | Me | O |
| D-5093 | H | CH₂CH₂ | Me | *c*-Pr | Me | Br | Me | O |
| D-5094 | Me | CH₂CH₂ | H | *c*-Pr | Me | Br | Me | O |
| D-5095 | H | single bond | Me | *c*-Pr | Me | Br | Me | O |
| D-5096 | Me | single bond | H | *c*-Pr | Me | Br | Me | O |
| D-5097 | Me(R) | single bond | H | *c*-Pr | Me | Br | Me | O |
| D-5098 | Me(S) | single bond | H | *c*-Pr | Me | Br | Me | O |

**[Table 761]**

| Compound Number | Y | W | Z | R¹ | R² | R³ | R¹⁰ | A |
|---|---|---|---|---|---|---|---|---|
| D-5099 | H | CH₂ | Me | *c*-Pr | Me | Br | Me | O |
| D-5100 | Me | CH₂ | H | *c*-Pr | Me | Br | Me | O |
| D-5101 | H | CH(Me) | H | *c*-Pr | Me | Br | Me | O |
| D-5102 | H | CH₂CH₂ | Me | *c*-Pr | Me | Br | Me | O |
| D-5103 | Me | CH₂CH₂ | H | *c*-Pr | Me | Br | Me | O |
| D-5104 | H | single bond | Me | *c*-Pr | Me | Br | Me | O |
| D-5105 | Me | single bond | H | *c*-Pr | Me | Br | Me | O |
| D-5106 | Me(R) | single bond | H | *c*-Pr | Me | Br | Me | O |
| D-5107 | Me(S) | single bond | H | *c*-Pr | Me | Br | Me | O |
| D-5108 | H | CH₂ | Me | *c*-Pr | Me | Br | Me | O |
| D-5109 | Me | CH₂ | H | *c*-Pr | Me | Br | Me | O |
| D-5110 | H | CH(Me) | H | *c*-Pr | Me | Br | Me | O |
| D-5111 | H | CH₂CH₂ | Me | *c*-Pr | Me | Br | Me | O |
| D-5112 | Me | CH₂CH₂ | H | *c*-Pr | Me | Br | Me | O |
| D-5113 | H | single bond | Me | *c*-Pr | Me | CF₃ | Me | O |
| D-5114 | Me | single bond | H | *c*-Pr | Me | CF₃ | Me | O |
| D-5115 | Me(R) | single bond | H | *c*-Pr | Me | CF₃ | Me | O |
| D-5116 | Me(S) | single bond | H | *c*-Pr | Me | CF₃ | Me | O |
| D-5117 | H | CH₂ | Me | *c*-Pr | Me | CF₃ | Me | O |
| D-5118 | Me | CH₂ | H | *c*-Pr | Me | CF₃ | Me | O |
| D-5119 | H | CH(Me) | H | *c*-Pr | Me | CF₃ | Me | O |
| D-5120 | H | CH₂CH₂ | Me | *c*-Pr | Me | CF₃ | Me | O |
| D-5121 | Me | CH₂CH₂ | H | *c*-Pr | Me | CF₃ | Me | O |
| D-5122 | H | single bond | Me | *c*-Pr | Me | CF₃ | Me | O |
| D-5123 | Me | single bond | H | *c*-Pr | Me | CF₃ | Me | O |
| D-5124 | Me(R) | single bond | H | *c*-Pr | Me | CF₃ | Me | O |
| D-5125 | Me(S) | single bond | H | *c*-Pr | Me | CF₃ | Me | O |
| D-5126 | H | CH₂ | Me | *c*-Pr | Me | CF₃ | Me | O |
| D-5127 | Me | CH₂ | H | *c*-Pr | Me | CF₃ | Me | O |
| D-5128 | H | CH(Me) | H | *c*-Pr | Me | CF₃ | Me | O |
| D-5129 | H | CH₂CH₂ | Me | *c*-Pr | Me | CF₃ | Me | O |
| D-5130 | Me | CH₂CH₂ | H | *c*-Pr | Me | CF₃ | Me | O |
| D-5131 | H | single bond | Me | *c*-Pr | Me | CF₃ | Me | O |
| D-5132 | Me | single bond | H | *c*-Pr | Me | CF₃ | Me | O |
| D-5133 | Me(R) | single bond | H | *c*-Pr | Me | CF₃ | Me | O |
| D-5134 | Me(S) | single bond | H | *c*-Pr | Me | CF₃ | Me | O |
| D-5135 | H | CH₂ | Me | *c*-Pr | Me | CF₃ | Me | O |
| D-5136 | Me | CH₂ | H | *c*-Pr | Me | CF₃ | Me | O |
| D-5137 | H | CH(Me) | H | *c*-Pr | Me | CF₃ | Me | O |
| D-5138 | H | CH₂CH₂ | Me | *c*-Pr | Me | CF₃ | Me | O |
| D-5139 | Me | CH₂CH₂ | H | *c*-Pr | Me | CF₃ | Me | O |
| D-5140 | H | single bond | Me | *c*-Pr | Me | CF₃ | Me | O |
| D-5141 | Me | single bond | H | *c*-Pr | Me | CF₃ | Me | O |
| D-5142 | Me(R) | single bond | H | *c*-Pr | Me | CF₃ | Me | O |

**[Table 762]**

| Compound Number | Y | W | Z | R¹ | R² | R³ | R¹⁰ | A |
|---|---|---|---|---|---|---|---|---|
| D-5143 | Me(S) | single bond | H | *c*-Pr | Me | CF₃ | Me | O |
| D-5144 | H | CH₂ | Me | *c*-Pr | Me | CF₃ | Me | O |
| D-5145 | Me | CH₂ | H | *c*-Pr | Me | CF₃ | Me | O |
| D-5146 | H | CH(Me) | H | *c*-Pr | Me | CF₃ | Me | O |
| D-5147 | H | CH₂CH₂ | Me | *c*-Pr | Me | CF₃ | Me | O |
| D-5148 | Me | CH₂CH₂ | H | *c*-Pr | Me | CF₃ | Me | O |
| D-5149 | H | single bond | Me | *c*-Pr | Me | OMe | Me | O |
| D-5150 | Me | single bond | H | *c*-Pr | Me | OMe | Me | O |
| D-5151 | Me(R) | single bond | H | *c*-Pr | Me | OMe | Me | O |
| D-5152 | Me(S) | single bond | H | *c*-Pr | Me | OMe | Me | O |
| D-5153 | H | CH₂ | Me | *c*-Pr | Me | OMe | Me | O |
| D-5154 | Me | CH₂ | H | *c*-Pr | Me | OMe | Me | O |
| D-5155 | H | CH(Me) | H | *c*-Pr | Me | OMe | Me | O |
| D-5156 | H | CH₂CH₂ | Me | *c*-Pr | Me | OMe | Me | O |
| D-5157 | Me | CH₂CH₂ | H | *c*-Pr | Me | OMe | Me | O |
| D-5158 | H | single bond | Me | *c*-Pr | Me | OMe | Me | O |
| D-5159 | Me | single bond | H | *c*-Pr | Me | OMe | Me | O |
| D-5160 | Me(R) | single bond | H | *c*-Pr | Me | OMe | Me | O |
| D-5161 | Me(S) | single bond | H | *c*-Pr | Me | OMe | Me | O |
| D-5162 | H | CH₂ | Me | *c*-Pr | Me | OMe | Me | O |
| D-5163 | Me | CH₂ | H | *c*-Pr | Me | OMe | Me | O |
| D-5164 | H | CH(Me) | H | *c*-Pr | Me | OMe | Me | O |
| D-5165 | H | CH₂CH₂ | Me | *c*-Pr | Me | OMe | Me | O |
| D-5166 | Me | CH₂CH₂ | H | *c*-Pr | Me | OMe | Me | O |
| D-5167 | H | single bond | Me | *c*-Pr | Me | OMe | Me | O |
| D-5168 | Me | single bond | H | *c*-Pr | Me | OMe | Me | O |
| D-5169 | Me(R) | single bond | H | *c*-Pr | Me | OMe | Me | O |
| D-5170 | Me(S) | single bond | H | *c*-Pr | Me | OMe | Me | O |
| D-5171 | H | CH₂ | Me | *c*-Pr | Me | OMe | Me | O |
| D-5172 | Me | CH₂ | H | *c*-Pr | Me | OMe | Me | O |
| D-5173 | H | CH(Me) | H | *c*-Pr | Me | OMe | Me | O |
| D-5174 | H | CH₂CH₂ | Me | *c*-Pr | Me | OMe | Me | O |
| D-5175 | Me | CH₂CH₂ | H | *c*-Pr | Me | OMe | Me | O |
| D-5176 | H | single bond | Me | *c*-Pr | Me | OMe | Me | O |
| D-5177 | Me | single bond | H | *c*-Pr | Me | OMe | Me | O |
| D-5178 | Me(R) | single bond | H | *c*-Pr | Me | OMe | Me | O |
| D-5179 | Me(S) | single bond | H | *c*-Pr | Me | OMe | Me | O |
| D-5180 | H | CH₂ | Me | *c*-Pr | Me | OMe | Me | O |
| D-5181 | Me | CH₂ | H | *c*-Pr | Me | OMe | Me | O |
| D-5182 | H | CH(Me) | H | *c*-Pr | Me | OMe | Me | O |
| D-5183 | H | CH₂CH₂ | Me | *c*-Pr | Me | OMe | Me | O |
| D-5184 | Me | CH₂CH₂ | H | *c*-Pr | Me | OMe | Me | O |
| D-5185 | H | single bond | Me | *c*-Pr | Me | OCF₃ | Me | O |
| D-5186 | Me | single bond | H | *c*-Pr | Me | OCF₃ | Me | O |

**[Table 763]**

| Compound Number | Y | W | Z | R¹ | R² | R³ | R¹⁰ | A |
|---|---|---|---|---|---|---|---|---|
| D-5187 | Me(R) | single bond | H | *c*-Pr | Me | OCF₃ | Me | O |
| D-5188 | Me(S) | single bond | H | *c*-Pr | Me | OCF₃ | Me | O |
| D-5189 | H | CH₂ | Me | *c*-Pr | Me | OCF₃ | Me | O |
| D-5190 | Me | CH₂ | H | *c*-Pr | Me | OCF₃ | Me | O |
| D-5191 | H | CH(Me) | H | *c*-Pr | Me | OCF₃ | Me | O |
| D-5192 | H | CH₂CH₂ | Me | *c*-Pr | Me | OCF₃ | Me | O |
| D-5193 | Me | CH₂CH₂ | H | *c*-Pr | Me | OCF₃ | Me | O |
| D-5194 | H | single bond | Me | *c*-Pr | Me | OCF₃ | Me | O |
| D-5195 | Me | single bond | H | *c*-Pr | Me | OCF₃ | Me | O |
| D-5196 | Me(R) | single bond | H | *c*-Pr | Me | OCF₃ | Me | O |
| D-5197 | Me(S) | single bond | H | *c*-Pr | Me | OCF₃ | Me | O |
| D-5198 | H | CH₂ | Me | *c*-Pr | Me | OCF₃ | Me | O |
| D-5199 | Me | CH₂ | H | *c*-Pr | Me | OCF₃ | Me | O |
| D-5200 | H | CH(Me) | H | *c*-Pr | Me | OCF₃ | Me | O |
| D-5201 | H | CH₂CH₂ | Me | *c*-Pr | Me | OCF₃ | Me | O |
| D-5202 | Me | CH₂CH₂ | H | *c*-Pr | Me | OCF₃ | Me | O |
| D-5203 | H | single bond | Me | *c*-Pr | Me | OCF₃ | Me | O |
| D-5204 | Me | single bond | H | *c*-Pr | Me | OCF₃ | Me | O |
| D-5205 | Me(R) | single bond | H | *c*-Pr | Me | OCF₃ | Me | O |
| D-5206 | Me(S) | single bond | H | *c*-Pr | Me | OCF₃ | Me | O |
| D-5207 | H | CH₂ | Me | *c*-Pr | Me | OCF₃ | Me | O |
| D-5208 | Me | CH₂ | H | *c*-Pr | Me | OCF₃ | Me | O |
| D-5209 | H | CH(Me) | H | *c*-Pr | Me | OCF₃ | Me | O |
| D-5210 | H | CH₂CH₂ | Me | *c*-Pr | Me | OCF₃ | Me | O |
| D-5211 | Me | CH₂CH₂ | H | *c*-Pr | Me | OCF₃ | Me | O |
| D-5212 | H | single bond | Me | *c*-Pr | Me | OCF₃ | Me | O |
| D-5213 | Me | single bond | H | *c*-Pr | Me | OCF₃ | Me | O |
| D-5214 | Me(R) | single bond | H | *c*-Pr | Me | OCF₃ | Me | O |
| D-5215 | Me(S) | single bond | H | *c*-Pr | Me | OCF₃ | Me | O |
| D-5216 | H | CH₂ | Me | *c*-Pr | Me | OCF₃ | Me | O |
| D-5217 | Me | CH₂ | H | *c*-Pr | Me | OCF₃ | Me | O |
| D-5218 | H | CH(Me) | H | *c*-Pr | Me | OCF₃ | Me | O |
| D-5219 | H | CH₂CH₂ | Me | *c*-Pr | Me | OCF₃ | Me | O |
| D-5220 | Me | CH₂CH₂ | H | *c*-Pr | Me | OCF₃ | Me | O |
| D-5221 | H | single bond | Me | *c*-Pr | Me | Br | CH₂OMe | O |
| D-5222 | Me | single bond | H | *c*-Pr | Me | Br | CH₂OMe | O |
| D-5223 | Me(R) | single bond | H | *c*-Pr | Me | Br | CH₂OMe | O |
| D-5224 | Me(S) | single bond | H | *c*-Pr | Me | Br | CH₂OMe | O |
| D-5225 | H | CH₂ | Me | *c*-Pr | Me | Br | CH₂OMe | O |
| D-5226 | Me | CH₂ | H | *c*-Pr | Me | Br | CH₂OMe | O |
| D-5227 | H | CH(Me) | H | *c*-Pr | Me | Br | CH₂OMe | O |
| D-5228 | H | CH₂CH₂ | Me | *c*-Pr | Me | Br | CH₂OMe | O |
| D-5229 | Me | CH₂CH₂ | H | *c*-Pr | Me | Br | CH₂OMe | O |
| D-5230 | H | single bond | Me | *c*-Pr | Me | Br | CH₂OMe | O |

**[Table 764]**

| Compound Number | Y | W | Z | R¹ | R² | R³ | R¹⁰ | A |
|---|---|---|---|---|---|---|---|---|
| D-5231 | Me | single bond | H | *c*-Pr | Me | Br | CH₂OMe | O |
| D-5232 | Me(R) | single bond | H | *c*-Pr | Me | Br | CH₂OMe | O |
| D-5233 | Me(S) | single bond | H | *c*-Pr | Me | Br | CH₂OMe | O |
| D-5234 | H | CH₂ | Me | *c*-Pr | Me | Br | CH₂OMe | O |
| D-5235 | Me | CH₂ | H | *c*-Pr | Me | Br | CH₂OMe | O |
| D-5236 | H | CH(Me) | H | *c*-Pr | Me | Br | CH₂OMe | O |
| D-5237 | H | CH₂CH₂ | Me | *c*-Pr | Me | Br | CH₂OMe | O |
| D-5238 | Me | CH₂CH₂ | H | *c*-Pr | Me | Br | CH₂OMe | O |
| D-5239 | H | single bond | Me | *c*-Pr | Me | Br | CH₂OMe | O |
| D-5240 | Me | single bond | H | *c*-Pr | Me | Br | CH₂OMe | O |
| D-5241 | Me(R) | single bond | H | *c*-Pr | Me | Br | CH₂OMe | O |
| D-5242 | Me(S) | single bond | H | *c*-Pr | Me | Br | CH₂OMe | O |
| D-5243 | H | CH₂ | Me | *c*-Pr | Me | Br | CH₂OMe | O |
| D-5244 | Me | CH₂ | H | *c*-Pr | Me | Br | CH₂OMe | O |
| D-5245 | H | CH(Me) | H | *c*-Pr | Me | Br | CH₂OMe | O |
| D-5246 | H | CH₂CH₂ | Me | *c*-Pr | Me | Br | CH₂OMe | O |
| D-5247 | Me | CH₂CH₂ | H | *c*-Pr | Me | Br | CH₂OMe | O |
| D-5248 | H | single bond | Me | *c*-Pr | Me | Br | CH₂OMe | O |
| D-5249 | Me | single bond | H | *c*-Pr | Me | Br | CH₂OMe | O |
| D-5250 | Me(R) | single bond | H | *c*-Pr | Me | Br | CH₂OMe | O |
| D-5251 | Me(S) | single bond | H | *c*-Pr | Me | Br | CH₂OMe | O |
| D-5252 | H | CH₂ | Me | *c*-Pr | Me | Br | CH₂OMe | O |
| D-5253 | Me | CH₂ | H | *c*-Pr | Me | Br | CH₂OMe | O |
| D-5254 | H | CH(Me) | H | *c*-Pr | Me | Br | CH₂OMe | O |
| D-5255 | H | CH₂CH₂ | Me | *c*-Pr | Me | Br | CH₂OMe | O |
| D-5256 | Me | CH₂CH₂ | H | *c*-Pr | Me | Br | CH₂OMe | O |
| D-5257 | H | single bond | Me | *c*-Pr | Me | CF₃ | CH₂OMe | O |
| D-5258 | Me | single bond | H | *c*-Pr | Me | CF₃ | CH₂OMe | O |
| D-5259 | Me(R) | single bond | H | *c*-Pr | Me | CF₃ | CH₂OMe | O |
| D-5260 | Me(S) | single bond | H | *c*-Pr | Me | CF₃ | CH₂OMe | O |
| D-5261 | H | CH₂ | Me | *c*-Pr | Me | CF₃ | CH₂OMe | O |
| D-5262 | Me | CH₂ | H | *c*-Pr | Me | CF₃ | CH₂OMe | O |
| D-5263 | H | CH(Me) | H | *c*-Pr | Me | CF₃ | CH₂OMe | O |
| D-5264 | H | CH₂CH₂ | Me | *c*-Pr | Me | CF₃ | CH₂OMe | O |
| D-5265 | Me | CH₂CH₂ | H | *c*-Pr | Me | CF₃ | CH₂OMe | O |
| D-5266 | H | single bond | Me | *c*-Pr | Me | CF₃ | CH₂OMe | O |
| D-5267 | Me | single bond | H | *c*-Pr | Me | CF₃ | CH₂OMe | O |
| D-5268 | Me(R) | single bond | H | *c*-Pr | Me | CF₃ | CH₂OMe | O |
| D-5269 | Me(S) | single bond | H | *c*-Pr | Me | CF₃ | CH₂OMe | O |
| D-5270 | H | CH₂ | Me | *c*-Pr | Me | CF₃ | CH₂OMe | O |
| D-5271 | Me | CH₂ | H | *c*-Pr | Me | CF₃ | CH₂OMe | O |
| D-5272 | H | CH(Me) | H | *c*-Pr | Me | CF₃ | CH₂OMe | O |
| D-5273 | H | CH₂CH₂ | Me | *c*-Pr | Me | CF₃ | CH₂OMe | O |
| D-5274 | Me | CH₂CH₂ | H | *c*-Pr | Me | CF₃ | CH₂OMe | O |

**[Table 765]**

| Compound Number | Y | W | Z | R¹ | R² | R³ | R¹⁰ | A |
|---|---|---|---|---|---|---|---|---|
| D-5275 | H | single bond | Me | *c*-Pr | Me | CF₃ | CH₂OMe | O |
| D-5276 | Me | single bond | H | *c*-Pr | Me | CF₃ | CH₂OMe | O |
| D-5277 | Me(R) | single bond | H | *c*-Pr | Me | CF₃ | CH₂OMe | O |
| D-5278 | Me(S) | single bond | H | *c*-Pr | Me | CF₃ | CH₂OMe | O |
| D-5279 | H | CH₂ | Me | *c*-Pr | Me | CF₃ | CH₂OMe | O |
| D-5280 | Me | CH₂ | H | *c*-Pr | Me | CF₃ | CH₂OMe | O |
| D-5281 | H | CH(Me) | H | *c*-Pr | Me | CF₃ | CH₂OMe | O |
| D-5282 | H | CH₂CH₂ | Me | *c*-Pr | Me | CF₃ | CH₂OMe | O |
| D-5283 | Me | CH₂CH₂ | H | *c*-Pr | Me | CF₃ | CH₂OMe | O |
| D-5284 | H | single bond | Me | *c*-Pr | Me | CF₃ | CH₂OMe | O |
| D-5285 | Me | single bond | H | *c*-Pr | Me | CF₃ | CH₂OMe | O |
| D-5286 | Me(R) | single bond | H | *c*-Pr | Me | CF₃ | CH₂OMe | O |
| D-5287 | Me(S) | single bond | H | *c*-Pr | Me | CF₃ | CH₂OMe | O |
| D-5288 | H | CH₂ | Me | *c*-Pr | Me | CF₃ | CH₂OMe | O |
| D-5289 | Me | CH₂ | H | *c*-Pr | Me | CF₃ | CH₂OMe | O |
| D-5290 | H | CH(Me) | H | *c*-Pr | Me | CF₃ | CH₂OMe | O |
| D-5291 | H | CH₂CH₂ | Me | *c*-Pr | Me | CF₃ | CH₂OMe | O |
| D-5292 | Me | CH₂CH₂ | H | *c*-Pr | Me | CF₃ | CH₂OMe | O |
| D-5293 | H | single bond | Me | *c*-Pr | Me | OMe | CH₂OMe | O |
| D-5294 | Me | single bond | H | *c*-Pr | Me | OMe | CH₂OMe | O |
| D-5295 | Me(R) | single bond | H | *c*-Pr | Me | OMe | CH₂OMe | O |
| D-5296 | Me(S) | single bond | H | *c*-Pr | Me | OMe | CH₂OMe | O |
| D-5297 | H | CH₂ | Me | *c*-Pr | Me | OMe | CH₂OMe | O |
| D-5298 | Me | CH₂ | H | *c*-Pr | Me | OMe | CH₂OMe | O |
| D-5299 | H | CH(Me) | H | *c*-Pr | Me | OMe | CH₂OMe | O |
| D-5300 | H | CH₂CH₂ | Me | *c*-Pr | Me | OMe | CH₂OMe | O |
| D-5301 | Me | CH₂CH₂ | H | *c*-Pr | Me | OMe | CH₂OMe | O |
| D-5302 | H | single bond | Me | *c*-Pr | Me | OMe | CH₂OMe | O |
| D-5303 | Me | single bond | H | *c*-Pr | Me | OMe | CH₂OMe | O |
| D-5304 | Me(R) | single bond | H | *c*-Pr | Me | OMe | CH₂OMe | O |
| D-5305 | Me(S) | single bond | H | *c*-Pr | Me | OMe | CH₂OMe | O |
| D-5306 | H | CH₂ | Me | *c*-Pr | Me | OMe | CH₂OMe | O |
| D-5307 | Me | CH₂ | H | *c*-Pr | Me | OMe | CH₂OMe | O |
| D-5308 | H | CH(Me) | H | *c*-Pr | Me | OMe | CH₂OMe | O |
| D-5309 | H | CH₂CH₂ | Me | *c*-Pr | Me | OMe | CH₂OMe | O |
| D-5310 | Me | CH₂CH₂ | H | *c*-Pr | Me | OMe | CH₂OMe | O |
| D-5311 | H | single bond | Me | *c*-Pr | Me | OMe | CH₂OMe | O |
| D-5312 | Me | single bond | H | *c*-Pr | Me | OMe | CH₂OMe | O |
| D-5313 | Me(R) | single bond | H | *c*-Pr | Me | OMe | CH₂OMe | O |
| D-5314 | Me(S) | single bond | H | *c*-Pr | Me | OMe | CH₂OMe | O |
| D-5315 | H | CH₂ | Me | *c*-Pr | Me | OMe | CH₂OMe | O |
| D-5316 | Me | CH₂ | H | *c*-Pr | Me | OMe | CH₂OMe | O |
| D-5317 | H | CH(Me) | H | *c*-Pr | Me | OMe | CH₂OMe | O |
| D-5318 | H | CH₂CH₂ | Me | *c*-Pr | Me | OMe | CH₂OMe | O |

**[Table 766]**

| Compound Number | Y | W | Z | R¹ | R² | R³ | R¹⁰ | A |
|---|---|---|---|---|---|---|---|---|
| D-5319 | Me | CH₂CH₂ | H | *c*-Pr | Me | OMe | CH₂OMe | O |
| D-5320 | H | single bond | Me | *c*-Pr | Me | OMe | CH₂OMe | O |
| D-5321 | Me | single bond | H | *c*-Pr | Me | OMe | CH₂OMe | O |
| D-5322 | Me(R) | single bond | H | *c*-Pr | Me | OMe | CH₂OMe | O |
| D-5323 | Me(S) | single bond | H | *c*-Pr | Me | OMe | CH₂OMe | O |
| D-5324 | H | CH₂ | Me | *c*-Pr | Me | OMe | CH₂OMe | O |
| D-5325 | Me | CH₂ | H | *c*-Pr | Me | OMe | CH₂OMe | O |
| D-5326 | H | CH(Me) | H | *c*-Pr | Me | OMe | CH₂OMe | O |
| D-5327 | H | CH₂CH₂ | Me | *c*-Pr | Me | OMe | CH₂OMe | O |
| D-5328 | Me | CH₂CH₂ | H | *c*-Pr | Me | OMe | CH₂OMe | O |
| D-5329 | H | single bond | Me | *c*-Pr | Me | OCF₃ | CH₂OMe | O |
| D-5330 | Me | single bond | H | *c*-Pr | Me | OCF₃ | CH₂OMe | O |
| D-5331 | Me(R) | single bond | H | *c*-Pr | Me | OCF₃ | CH₂OMe | O |
| D-5332 | Me(S) | single bond | H | *c*-Pr | Me | OCF₃ | CH₂OMe | O |
| D-5333 | H | CH₂ | Me | *c*-Pr | Me | OCF₃ | CH₂OMe | O |
| D-5334 | Me | CH₂ | H | *c*-Pr | Me | OCF₃ | CH₂OMe | O |
| D-5335 | H | CH(Me) | H | *c*-Pr | Me | OCF₃ | CH₂OMe | O |
| D-5336 | H | CH₂CH₂ | Me | *c*-Pr | Me | OCF₃ | CH₂OMe | O |
| D-5337 | Me | CH₂CH₂ | H | *c*-Pr | Me | OCF₃ | CH₂OMe | O |
| D-5338 | H | single bond | Me | *c*-Pr | Me | OCF₃ | CH₂OMe | O |
| D-5339 | Me | single bond | H | *c*-Pr | Me | OCF₃ | CH₂OMe | O |
| D-5340 | Me(R) | single bond | H | *c*-Pr | Me | OCF₃ | CH₂OMe | O |
| D-5341 | Me(S) | single bond | H | *c*-Pr | Me | OCF₃ | CH₂OMe | O |
| D-5342 | H | CH₂ | Me | *c*-Pr | Me | OCF₃ | CH₂OMe | O |
| D-5343 | Me | CH₂ | H | *c*-Pr | Me | OCF₃ | CH₂OMe | O |
| D-5344 | H | CH(Me) | H | *c*-Pr | Me | OCF₃ | CH₂OMe | O |
| D-5345 | H | CH₂CH₂ | Me | *c*-Pr | Me | OCF₃ | CH₂OMe | O |
| D-5346 | Me | CH₂CH₂ | H | *c*-Pr | Me | OCF₃ | CH₂OMe | O |
| D-5347 | H | single bond | Me | *c*-Pr | Me | OCF₃ | CH₂OMe | O |
| D-5348 | Me | single bond | H | *c*-Pr | Me | OCF₃ | CH₂OMe | O |
| D-5349 | Me(R) | single bond | H | *c*-Pr | Me | OCF₃ | CH₂OMe | O |
| D-5350 | Me(S) | single bond | H | *c*-Pr | Me | OCF₃ | CH₂OMe | O |
| D-5351 | H | CH₂ | Me | *c*-Pr | Me | OCF₃ | CH₂OMe | O |
| D-5352 | Me | CH₂ | H | *c*-Pr | Me | OCF₃ | CH₂OMe | O |
| D-5353 | H | CH(Me) | H | *c*-Pr | Me | OCF₃ | CH₂OMe | O |
| D-5354 | H | CH₂CH₂ | Me | *c*-Pr | Me | OCF₃ | CH₂OMe | O |
| D-5355 | Me | CH₂CH₂ | H | *c*-Pr | Me | OCF₃ | CH₂OMe | O |
| D-5356 | H | single bond | Me | *c*-Pr | Me | OCF₃ | CH₂OMe | O |
| D-5357 | Me | single bond | H | *c*-Pr | Me | OCF₃ | CH₂OMe | O |
| D-5358 | Me(R) | single bond | H | *c*-Pr | Me | OCF₃ | CH₂OMe | O |
| D-5359 | Me(S) | single bond | H | *c*-Pr | Me | OCF₃ | CH₂OMe | O |
| D-5360 | H | CH₂ | Me | *c*-Pr | Me | OCF₃ | CH₂OMe | O |
| D-5361 | Me | CH₂ | H | *c*-Pr | Me | OCF₃ | CH₂OMe | O |
| D-5362 | H | CH(Me) | H | *c*-Pr | Me | OCF₃ | CH₂OMe | O |

**[Table 767]**

| Compound Number | Y | W | Z | R¹ | R² | R³ | R¹⁰ | A |
|---|---|---|---|---|---|---|---|---|
| D-5363 | H | CH₂CH₂ | Me | *c*-Pr | Me | OCF₃ | CH₂OMe | O |
| D-5364 | Me | CH₂CH₂ | H | *c*-Pr | Me | OCF₃ | CH₂OMe | O |
| D-5365 | H | single bond | Me | *c*-Pr | Me | Br | CH₂CN | O |
| D-5366 | Me | single bond | H | *c*-Pr | Me | Br | CH₂CN | O |
| D-5367 | Me(R) | single bond | H | *c*-Pr | Me | Br | CH₂CN | O |
| D-5368 | Me(S) | single bond | H | *c*-Pr | Me | Br | CH₂CN | O |
| D-5369 | H | CH₂ | Me | *c*-Pr | Me | Br | CH₂CN | O |
| D-5370 | Me | CH₂ | H | *c*-Pr | Me | Br | CH₂CN | O |
| D-5371 | H | CH(Me) | H | *c*-Pr | Me | Br | CH₂CN | O |
| D-5372 | H | CH₂CH₂ | Me | *c*-Pr | Me | Br | CH₂CN | O |
| D-5373 | Me | CH₂CH₂ | H | *c*-Pr | Me | Br | CH₂CN | O |
| D-5374 | H | single bond | Me | *c*-Pr | Me | Br | CH₂CN | O |
| D-5375 | Me | single bond | H | *c*-Pr | Me | Br | CH₂CN | O |
| D-5376 | Me(R) | single bond | H | *c*-Pr | Me | Br | CH₂CN | O |
| D-5377 | Me(S) | single bond | H | *c*-Pr | Me | Br | CH₂CN | O |
| D-5378 | H | CH₂ | Me | *c*-Pr | Me | Br | CH₂CN | O |
| D-5379 | Me | CH₂ | H | *c*-Pr | Me | Br | CH₂CN | O |
| D-5380 | H | CH(Me) | H | *c*-Pr | Me | Br | CH₂CN | O |
| D-5381 | H | CH₂CH₂ | Me | *c*-Pr | Me | Br | CH₂CN | O |
| D-5382 | Me | CH₂CH₂ | H | *c*-Pr | Me | Br | CH₂CN | O |
| D-5383 | H | single bond | Me | *c*-Pr | Me | Br | CH₂CN | O |
| D-5384 | Me | single bond | H | *c*-Pr | Me | Br | CH₂CN | O |
| D-5385 | Me(R) | single bond | H | *c*-Pr | Me | Br | CH₂CN | O |
| D-5386 | Me(S) | single bond | H | *c*-Pr | Me | Br | CH₂CN | O |
| D-5387 | H | CH₂ | Me | *c*-Pr | Me | Br | CH₂CN | O |
| D-5388 | Me | CH₂ | H | *c*-Pr | Me | Br | CH₂CN | O |
| D-5389 | H | CH(Me) | H | *c*-Pr | Me | Br | CH₂CN | O |
| D-5390 | H | CH₂CH₂ | Me | *c*-Pr | Me | Br | CH₂CN | O |
| D-5391 | Me | CH₂CH₂ | H | *c*-Pr | Me | Br | CH₂CN | O |
| D-5392 | H | single bond | Me | *c*-Pr | Me | Br | CH₂CN | O |
| D-5393 | Me | single bond | H | *c*-Pr | Me | Br | CH₂CN | O |
| D-5394 | Me(R) | single bond | H | *c*-Pr | Me | Br | CH₂CN | O |
| D-5395 | Me(S) | single bond | H | *c*-Pr | Me | Br | CH₂CN | O |
| D-5396 | H | CH₂ | Me | *c*-Pr | Me | Br | CH₂CN | O |
| D-5397 | Me | CH₂ | H | *c*-Pr | Me | Br | CH₂CN | O |
| D-5398 | H | CH(Me) | H | *c*-Pr | Me | Br | CH₂CN | O |
| D-5399 | H | CH₂CH₂ | Me | *c*-Pr | Me | Br | CH₂CN | O |
| D-5400 | Me | CH₂CH₂ | H | *c*-Pr | Me | Br | CH₂CN | O |
| D-5401 | H | single bond | Me | *c*-Pr | Me | CF₃ | CH₂CN | O |
| D-5402 | Me | single bond | H | *c*-Pr | Me | CF₃ | CH₂CN | O |
| D-5403 | Me(R) | single bond | H | *c*-Pr | Me | CF₃ | CH₂CN | O |
| D-5404 | Me(S) | single bond | H | *c*-Pr | Me | CF₃ | CH₂CN | O |
| D-5405 | H | CH₂ | Me | *c*-Pr | Me | CF₃ | CH₂CN | O |
| D-5406 | Me | CH₂ | H | *c*-Pr | Me | CF₃ | CH₂CN | O |

**[Table 768]**

| Compound Number | Y | W | Z | R¹ | R² | R³ | R¹⁰ | A |
|---|---|---|---|---|---|---|---|---|
| D-5407 | H | CH(Me) | H | *c*-Pr | Me | CF₃ | CH₂CN | O |
| D-5408 | H | CH₂CH₂ | Me | *c*-Pr | Me | CF₃ | CH₂CN | O |
| D-5409 | Me | CH₂CH₂ | H | *c*-Pr | Me | CF₃ | CH₂CN | O |
| D-5410 | H | single bond | Me | *c*-Pr | Me | CF₃ | CH₂CN | O |
| D-5411 | Me | single bond | H | *c*-Pr | Me | CF₃ | CH₂CN | O |
| D-5412 | Me(R) | single bond | H | *c*-Pr | Me | CF₃ | CH₂CN | O |
| D-5413 | Me(S) | single bond | H | *c*-Pr | Me | CF₃ | CH₂CN | O |
| D-5414 | H | CH₂ | Me | *c*-Pr | Me | CF₃ | CH₂CN | O |
| D-5415 | Me | CH₂ | H | *c*-Pr | Me | CF₃ | CH₂CN | O |
| D-5416 | H | CH(Me) | H | *c*-Pr | Me | CF₃ | CH₂CN | O |
| D-5417 | H | CH₂CH₂ | Me | *c*-Pr | Me | CF₃ | CH₂CN | O |
| D-5418 | Me | CH₂CH₂ | H | *c*-Pr | Me | CF₃ | CH₂CN | O |
| D-5419 | H | single bond | Me | *c*-Pr | Me | CF₃ | CH₂CN | O |
| D-5420 | Me | single bond | H | *c*-Pr | Me | CF₃ | CH₂CN | O |
| D-5421 | Me(R) | single bond | H | *c*-Pr | Me | CF₃ | CH₂CN | O |
| D-5422 | Me(S) | single bond | H | *c*-Pr | Me | CF₃ | CH₂CN | O |
| D-5423 | H | CH₂ | Me | *c*-Pr | Me | CF₃ | CH₂CN | O |
| D-5424 | Me | CH₂ | H | *c*-Pr | Me | CF₃ | CH₂CN | O |
| D-5425 | H | CH(Me) | H | *c*-Pr | Me | CF₃ | CH₂CN | O |
| D-5426 | H | CH₂CH₂ | Me | *c*-Pr | Me | CF₃ | CH₂CN | O |
| D-5427 | Me | CH₂CH₂ | H | *c*-Pr | Me | CF₃ | CH₂CN | O |
| D-5428 | H | single bond | Me | *c*-Pr | Me | CF₃ | CH₂CN | O |
| D-5429 | Me | single bond | H | *c*-Pr | Me | CF₃ | CH₂CN | O |
| D-5430 | Me(R) | single bond | H | *c*-Pr | Me | CF₃ | CH₂CN | O |
| D-5431 | Me(S) | single bond | H | *c*-Pr | Me | CF₃ | CH₂CN | O |
| D-5432 | H | CH₂ | Me | *c*-Pr | Me | CF₃ | CH₂CN | O |
| D-5433 | Me | CH₂ | H | *c*-Pr | Me | CF₃ | CH₂CN | O |
| D-5434 | H | CH(Me) | H | *c*-Pr | Me | CF₃ | CH₂CN | O |
| D-5435 | H | CH₂CH₂ | Me | *c*-Pr | Me | CF₃ | CH₂CN | O |
| D-5436 | Me | CH₂CH₂ | H | *c*-Pr | Me | CF₃ | CH₂CN | O |
| D-5437 | H | single bond | Me | *c*-Pr | Me | OMe | CH₂CN | O |
| D-5438 | Me | single bond | H | *c*-Pr | Me | OMe | CH₂CN | O |
| D-5439 | Me(R) | single bond | H | *c*-Pr | Me | OMe | CH₂CN | O |
| D-5440 | Me(S) | single bond | H | *c*-Pr | Me | OMe | CH₂CN | O |
| D-5441 | H | CH₂ | Me | *c*-Pr | Me | OMe | CH₂CN | O |
| D-5442 | Me | CH₂ | H | *c*-Pr | Me | OMe | CH₂CN | O |
| D-5443 | H | CH(Me) | H | *c*-Pr | Me | OMe | CH₂CN | O |
| D-5444 | H | CH₂CH₂ | Me | *c*-Pr | Me | OMe | CH₂CN | O |
| D-5445 | Me | CH₂CH₂ | H | *c*-Pr | Me | OMe | CH₂CN | O |
| D-5446 | H | single bond | Me | *c*-Pr | Me | OMe | CH₂CN | O |
| D-5447 | Me | single bond | H | *c*-Pr | Me | OMe | CH₂CN | O |
| D-5448 | Me(R) | single bond | H | *c*-Pr | Me | OMe | CH₂CN | O |
| D-5449 | Me(S) | single bond | H | *c*-Pr | Me | OMe | CH₂CN | O |
| D-5450 | H | CH₂ | Me | *c*-Pr | Me | OMe | CH₂CN | O |

**[Table 769]**

| Compound Number | Y | W | Z | R¹ | R² | R³ | R¹⁰ | A |
|---|---|---|---|---|---|---|---|---|
| D-5451 | Me | CH₂ | H | *c*-Pr | Me | OMe | CH₂CN | O |
| D-5452 | H | CH(Me) | H | *c*-Pr | Me | OMe | CH₂CN | O |
| D-5453 | H | CH₂CH₂ | Me | *c*-Pr | Me | OMe | CH₂CN | O |
| D-5454 | Me | CH₂CH₂ | H | *c*-Pr | Me | OMe | CH₂CN | O |
| D-5455 | H | single bond | Me | *c*-Pr | Me | OMe | CH₂CN | O |
| D-5456 | Me | single bond | H | *c*-Pr | Me | OMe | CH₂CN | O |
| D-5457 | Me(R) | single bond | H | *c*-Pr | Me | OMe | CH₂CN | O |
| D-5458 | Me(S) | single bond | H | *c*-Pr | Me | OMe | CH₂CN | O |
| D-5459 | H | CH₂ | Me | *c*-Pr | Me | OMe | CH₂CN | O |
| D-5460 | Me | CH₂ | H | *c*-Pr | Me | OMe | CH₂CN | O |
| D-5461 | H | CH(Me) | H | *c*-Pr | Me | OMe | CH₂CN | O |
| D-5462 | H | CH₂CH₂ | Me | *c*-Pr | Me | OMe | CH₂CN | O |
| D-5463 | Me | CH₂CH₂ | H | *c*-Pr | Me | OMe | CH₂CN | O |
| D-5464 | H | single bond | Me | *c*-Pr | Me | OMe | CH₂CN | O |
| D-5465 | Me | single bond | H | *c*-Pr | Me | OMe | CH₂CN | O |
| D-5466 | Me(R) | single bond | H | *c*-Pr | Me | OMe | CH₂CN | O |
| D-5467 | Me(S) | single bond | H | *c*-Pr | Me | OMe | CH₂CN | O |
| D-5468 | H | CH₂ | Me | *c*-Pr | Me | OMe | CH₂CN | O |
| D-5469 | Me | CH₂ | H | *c*-Pr | Me | OMe | CH₂CN | O |
| D-5470 | H | CH(Me) | H | *c*-Pr | Me | OMe | CH₂CN | O |
| D-5471 | H | CH₂CH₂ | Me | *c*-Pr | Me | OMe | CH₂CN | O |
| D-5472 | Me | CH₂CH₂ | H | *c*-Pr | Me | OMe | CH₂CN | O |
| D-5473 | H | single bond | Me | *c*-Pr | Me | OCF₃ | CH₂CN | O |
| D-5474 | Me | single bond | H | *c*-Pr | Me | OCF₃ | CH₂CN | O |
| D-5475 | Me(R) | single bond | H | *c*-Pr | Me | OCF₃ | CH₂CN | O |
| D-5476 | Me(S) | single bond | H | *c*-Pr | Me | OCF₃ | CH₂CN | O |
| D-5477 | H | CH₂ | Me | *c*-Pr | Me | OCF₃ | CH₂CN | O |
| D-5478 | Me | CH₂ | H | *c*-Pr | Me | OCF₃ | CH₂CN | O |
| D-5479 | H | CH(Me) | H | *c*-Pr | Me | OCF₃ | CH₂CN | O |
| D-5480 | H | CH₂CH₂ | Me | *c*-Pr | Me | OCF₃ | CH₂CN | O |
| D-5481 | Me | CH₂CH₂ | H | *c*-Pr | Me | OCF₃ | CH₂CN | O |
| D-5482 | H | single bond | Me | *c*-Pr | Me | OCF₃ | CH₂CN | O |
| D-5483 | Me | single bond | H | *c*-Pr | Me | OCF₃ | CH₂CN | O |
| D-5484 | Me(R) | single bond | H | *c*-Pr | Me | OCF₃ | CH₂CN | O |
| D-5485 | Me(S) | single bond | H | *c*-Pr | Me | OCF₃ | CH₂CN | O |
| D-5486 | H | CH₂ | Me | *c*-Pr | Me | OCF₃ | CH₂CN | O |
| D-5487 | Me | CH₂ | H | *c*-Pr | Me | OCF₃ | CH₂CN | O |
| D-5488 | H | CH(Me) | H | *c*-Pr | Me | OCF₃ | CH₂CN | O |
| D-5489 | H | CH₂CH₂ | Me | *c*-Pr | Me | OCF₃ | CH₂CN | O |
| D-5490 | Me | CH₂CH₂ | H | *c*-Pr | Me | OCF₃ | CH₂CN | O |
| D-5491 | H | single bond | Me | *c*-Pr | Me | OCF₃ | CH₂CN | O |
| D-5492 | Me | single bond | H | *c*-Pr | Me | OCF₃ | CH₂CN | O |
| D-5493 | Me(R) | single bond | H | *c*-Pr | Me | OCF₃ | CH₂CN | O |
| D-5494 | Me(S) | single bond | H | *c*-Pr | Me | OCF₃ | CH₂CN | O |

**[Table 770]**

| Compound Number | Y | W | Z | R¹ | R² | R³ | R¹⁰ | A |
|---|---|---|---|---|---|---|---|---|
| D-5495 | H | CH₂ | Me | *c*-Pr | Me | OCF₃ | CH₂CN | O |
| D-5496 | Me | CH₂ | H | *c*-Pr | Me | OCF₃ | CH₂CN | O |
| D-5497 | H | CH(Me) | H | *c*-Pr | Me | OCF₃ | CH₂CN | O |
| D-5498 | H | CH₂CH₂ | Me | *c*-Pr | Me | OCF₃ | CH₂CN | O |
| D-5499 | Me | CH₂CH₂ | H | *c*-Pr | Me | OCF₃ | CH₂CN | O |
| D-5500 | H | single bond | Me | *c*-Pr | Me | OCF₃ | CH₂CN | O |
| D-5501 | Me | single bond | H | *c*-Pr | Me | OCF₃ | CH₂CN | O |
| D-5502 | Me(R) | single bond | H | *c*-Pr | Me | OCF₃ | CH₂CN | O |
| D-5503 | Me(S) | single bond | H | *c*-Pr | Me | OCF₃ | CH₂CN | O |
| D-5504 | H | CH₂ | Me | *c*-Pr | Me | OCF₃ | CH₂CN | O |
| D-5505 | Me | CH₂ | H | *c*-Pr | Me | OCF₃ | CH₂CN | O |
| D-5506 | H | CH(Me) | H | *c*-Pr | Me | OCF₃ | CH₂CN | O |
| D-5507 | H | CH₂CH₂ | Me | *c*-Pr | Me | OCF₃ | CH₂CN | O |
| D-5508 | Me | CH₂CH₂ | H | *c*-Pr | Me | OCF₃ | CH₂CN | O |
| D-5509 | H | single bond | Me | *c*-Pr | C(=O)Me | Br | H | O |
| D-5510 | Me | single bond | H | *c*-Pr | C(=O)Me | Br | H | O |
| D-5511 | Me(R) | single bond | H | *c*-Pr | C(=O)Me | Br | H | O |
| D-5512 | Me(S) | single bond | H | *c*-Pr | C(=O)Me | Br | H | O |
| D-5513 | H | CH₂ | Me | *c*-Pr | C(=O)Me | Br | H | O |
| D-5514 | Me | CH₂ | H | *c*-Pr | C(=O)Me | Br | H | O |
| D-5515 | H | CH(Me) | H | *c*-Pr | C(=O)Me | Br | H | O |
| D-5516 | H | CH₂CH₂ | Me | *c*-Pr | C(=O)Me | Br | H | O |
| D-5517 | Me | CH₂CH₂ | H | *c*-Pr | C(=O)Me | Br | H | O |
| D-5518 | H | single bond | Me | *c*-Pr | C(=O)Me | Br | H | O |
| D-5519 | Me | single bond | H | *c*-Pr | C(=O)Me | Br | H | O |
| D-5520 | Me(R) | single bond | H | *c*-Pr | C(=O)Me | Br | H | O |
| D-5521 | Me(S) | single bond | H | *c*-Pr | C(=O)Me | Br | H | O |
| D-5522 | H | CH₂ | Me | *c*-Pr | C(=O)Me | Br | H | O |
| D-5523 | Me | CH₂ | H | *c*-Pr | C(=O)Me | Br | H | O |
| D-5524 | H | CH(Me) | H | *c*-Pr | C(=O)Me | Br | H | O |
| D-5525 | H | CH₂CH₂ | Me | *c*-Pr | C(=O)Me | Br | H | O |
| D-5526 | Me | CH₂CH₂ | H | *c*-Pr | C(=O)Me | Br | H | O |
| D-5527 | H | single bond | Me | *c*-Pr | C(=O)Me | Br | H | O |
| D-5528 | Me | single bond | H | *c*-Pr | C(=O)Me | Br | H | O |
| D-5529 | Me(R) | single bond | H | *c*-Pr | C(=O)Me | Br | H | O |
| D-5530 | Me(S) | single bond | H | *c*-Pr | C(=O)Me | Br | H | O |
| D-5531 | H | CH₂ | Me | *c*-Pr | C(=O)Me | Br | H | O |
| D-5532 | Me | CH₂ | H | *c*-Pr | C(=O)Me | Br | H | O |
| D-5533 | H | CH(Me) | H | *c*-Pr | C(=O)Me | Br | H | O |
| D-5534 | H | CH₂CH₂ | Me | *c*-Pr | C(=O)Me | Br | H | O |
| D-5535 | Me | CH₂CH₂ | H | *c*-Pr | C(=O)Me | Br | H | O |
| D-5536 | H | single bond | Me | *c*-Pr | C(=O)Me | Br | H | O |
| D-5537 | Me | single bond | H | *c*-Pr | C(=O)Me | Br | H | O |
| D-5538 | Me(R) | single bond | H | *c*-Pr | C(=O)Me | Br | H | O |

**[Table 771]**

| Compound Number | Y | W | Z | R¹ | R² | R³ | R¹⁰ | A |
|---|---|---|---|---|---|---|---|---|
| D-5539 | Me(S) | single bond | H | *c*-Pr | C(=O)Me | Br | H | O |
| D-5540 | H | CH₂ | Me | *c*-Pr | C(=O)Me | Br | H | O |
| D-5541 | Me | CH₂ | H | *c*-Pr | C(=O)Me | Br | H | O |
| D-5542 | H | CH(Me) | H | *c*-Pr | C(=O)Me | Br | H | O |
| D-5543 | H | CH₂CH₂ | Me | *c*-Pr | C(=O)Me | Br | H | O |
| D-5544 | Me | CH₂CH₂ | H | *c*-Pr | C(=O)Me | Br | H | O |
| D-5545 | H | single bond | Me | *c*-Pr | C(=O)Me | CF₃ | H | O |
| D-5546 | Me | single bond | H | *c*-Pr | C(=O)Me | CF₃ | H | O |
| D-5547 | Me(R) | single bond | H | *c*-Pr | C(=O)Me | CF₃ | H | O |
| D-5548 | Me(S) | single bond | H | *c*-Pr | C(=O)Me | CF₃ | H | O |
| D-5549 | H | CH₂ | Me | *c*-Pr | C(=O)Me | CF₃ | H | O |
| D-5550 | Me | CH₂ | H | *c*-Pr | C(=O)Me | CF₃ | H | O |
| D-5551 | H | CH(Me) | H | *c*-Pr | C(=O)Me | CF₃ | H | O |
| D-5552 | H | CH₂CH₂ | Me | *c*-Pr | C(=O)Me | CF₃ | H | O |
| D-5553 | Me | CH₂CH₂ | H | *c*-Pr | C(=O)Me | CF₃ | H | O |
| D-5554 | H | single bond | Me | *c*-Pr | C(=O)Me | CF₃ | H | O |
| D-5555 | Me | single bond | H | *c*-Pr | C(=O)Me | CF₃ | H | O |
| D-5556 | Me(R) | single bond | H | *c*-Pr | C(=O)Me | CF₃ | H | O |
| D-5557 | Me(S) | single bond | H | *c*-Pr | C(=O)Me | CF₃ | H | O |
| D-5558 | H | CH₂ | Me | *c*-Pr | C(=O)Me | CF₃ | H | O |
| D-5559 | Me | CH₂ | H | *c*-Pr | C(=O)Me | CF₃ | H | O |
| D-5560 | H | CH(Me) | H | *c*-Pr | C(=O)Me | CF₃ | H | O |
| D-5561 | H | CH₂CH₂ | Me | *c*-Pr | C(=O)Me | CF₃ | H | O |
| D-5562 | Me | CH₂CH₂ | H | *c*-Pr | C(=O)Me | CF₃ | H | O |
| D-5563 | H | single bond | Me | *c*-Pr | C(=O)Me | CF₃ | H | O |
| D-5564 | Me | single bond | H | *c*-Pr | C(=O)Me | CF₃ | H | O |
| D-5565 | Me(R) | single bond | H | *c*-Pr | C(=O)Me | CF₃ | H | O |
| D-5566 | Me(S) | single bond | H | *c*-Pr | C(=O)Me | CF₃ | H | O |
| D-5567 | H | CH₂ | Me | *c*-Pr | C(=O)Me | CF₃ | H | O |
| D-5568 | Me | CH₂ | H | *c*-Pr | C(=O)Me | CF₃ | H | O |
| D-5569 | H | CH(Me) | H | *c*-Pr | C(=O)Me | CF₃ | H | O |
| D-5570 | H | CH₂CH₂ | Me | *c*-Pr | C(=O)Me | CF₃ | H | O |
| D-5571 | Me | CH₂CH₂ | H | *c*-Pr | C(=O)Me | CF₃ | H | O |
| D-5572 | H | single bond | Me | *c*-Pr | C(=O)Me | CF₃ | H | O |
| D-5573 | Me | single bond | H | *c*-Pr | C(=O)Me | CF₃ | H | O |
| D-5574 | Me(R) | single bond | H | *c*-Pr | C(=O)Me | CF₃ | H | O |
| D-5575 | Me(S) | single bond | H | *c*-Pr | C(=O)Me | CF₃ | H | O |
| D-5576 | H | CH₂ | Me | *c*-Pr | C(=O)Me | CF₃ | H | O |
| D-5577 | Me | CH₂ | H | *c*-Pr | C(=O)Me | CF₃ | H | O |
| D-5578 | H | CH(Me) | H | *c*-Pr | C(=O)Me | CF₃ | H | O |
| D-5579 | H | CH₂CH₂ | Me | *c*-Pr | C(=O)Me | CF₃ | H | O |
| D-5580 | Me | CH₂CH₂ | H | *c*-Pr | C(=O)Me | CF₃ | H | O |
| D-5581 | H | single bond | Me | *c*-Pr | C(=O)Me | OMe | H | O |
| D-5582 | Me | single bond | H | *c*-Pr | C(=O)Me | OMe | H | O |

**[Table 772]**

| Compound Number | Y | W | Z | R¹ | R² | R³ | R¹⁰ | A |
|---|---|---|---|---|---|---|---|---|
| D-5583 | Me(R) | single bond | H | *c*-Pr | C(=O)Me | OMe | H | O |
| D-5584 | Me(S) | single bond | H | *c*-Pr | C(=O)Me | OMe | H | O |
| D-5585 | H | CH₂ | Me | *c*-Pr | C(=O)Me | OMe | H | O |
| D-5586 | Me | CH₂ | H | *c*-Pr | C(=O)Me | OMe | H | O |
| D-5587 | H | CH(Me) | H | *c*-Pr | C(=O)Me | OMe | H | O |
| D-5588 | H | CH₂CH₂ | Me | *c*-Pr | C(=O)Me | OMe | H | O |
| D-5589 | Me | CH₂CH₂ | H | *c*-Pr | C(=O)Me | OMe | H | O |
| D-5590 | H | single bond | Me | *c*-Pr | C(=O)Me | OMe | H | O |
| D-5591 | Me | single bond | H | *c*-Pr | C(=O)Me | OMe | H | O |
| D-5592 | Me(R) | single bond | H | *c*-Pr | C(=O)Me | OMe | H | O |
| D-5593 | Me(S) | single bond | H | *c*-Pr | C(=O)Me | OMe | H | O |
| D-5594 | H | CH₂ | Me | *c*-Pr | C(=O)Me | OMe | H | O |
| D-5595 | Me | CH₂ | H | *c*-Pr | C(=O)Me | OMe | H | O |
| D-5596 | H | CH(Me) | H | *c*-Pr | C(=O)Me | OMe | H | O |
| D-5597 | H | CH₂CH₂ | Me | *c*-Pr | C(=O)Me | OMe | H | O |
| D-5598 | Me | CH₂CH₂ | H | *c*-Pr | C(=O)Me | OMe | H | O |
| D-5599 | H | single bond | Me | *c*-Pr | C(=O)Me | OMe | H | O |
| D-5600 | Me | single bond | H | *c*-Pr | C(=O)Me | OMe | H | O |
| D-5601 | Me(R) | single bond | H | *c*-Pr | C(=O)Me | OMe | H | O |
| D-5602 | Me(S) | single bond | H | *c*-Pr | C(=O)Me | OMe | H | O |
| D-5603 | H | CH₂ | Me | *c*-Pr | C(=O)Me | OMe | H | O |
| D-5604 | Me | CH₂ | H | *c*-Pr | C(=O)Me | OMe | H | O |
| D-5605 | H | CH(Me) | H | *c*-Pr | C(=O)Me | OMe | H | O |
| D-5606 | H | CH₂CH₂ | Me | *c*-Pr | C(=O)Me | OMe | H | O |
| D-5607 | Me | CH₂CH₂ | H | *c*-Pr | C(=O)Me | OMe | H | O |
| D-5608 | H | single bond | Me | *c*-Pr | C(=O)Me | OMe | H | O |
| D-5609 | Me | single bond | H | *c*-Pr | C(=O)Me | OMe | H | O |
| D-5610 | Me(R) | single bond | H | *c*-Pr | C(=O)Me | OMe | H | O |
| D-5611 | Me(S) | single bond | H | *c*-Pr | C(=O)Me | OMe | H | O |
| D-5612 | H | CH₂ | Me | *c*-Pr | C(=O)Me | OMe | H | O |
| D-5613 | Me | CH₂ | H | *c*-Pr | C(=O)Me | OMe | H | O |
| D-5614 | H | CH(Me) | H | *c*-Pr | C(=O)Me | OMe | H | O |
| D-5615 | H | CH₂CH₂ | Me | *c*-Pr | C(=O)Me | OMe | H | O |
| D-5616 | Me | CH₂CH₂ | H | *c*-Pr | C(=O)Me | OMe | H | O |
| D-5617 | H | single bond | Me | *c*-Pr | C(=O)Me | OCF₃ | H | O |
| D-5618 | Me | single bond | H | *c*-Pr | C(=O)Me | OCF₃ | H | O |
| D-5619 | Me(R) | single bond | H | *c*-Pr | C(=O)Me | OCF₃ | H | O |
| D-5620 | Me(S) | single bond | H | *c*-Pr | C(=O)Me | OCF₃ | H | O |
| D-5621 | H | CH₂ | Me | *c*-Pr | C(=O)Me | OCF₃ | H | O |
| D-5622 | Me | CH₂ | H | *c*-Pr | C(=O)Me | OCF₃ | H | O |
| D-5623 | H | CH(Me) | H | *c*-Pr | C(=O)Me | OCF₃ | H | O |
| D-5624 | H | CH₂CH₂ | Me | *c*-Pr | C(=O)Me | OCF₃ | H | O |
| D-5625 | Me | CH₂CH₂ | H | *c*-Pr | C(=O)Me | OCF₃ | H | O |
| D-5626 | H | single bond | Me | *c*-Pr | C(=O)Me | OCF₃ | H | O |

**[Table 773]**

| Compound Number | Y | W | Z | R¹ | R² | R³ | R¹⁰ | A |
|---|---|---|---|---|---|---|---|---|
| D-5627 | Me | single bond | H | *c*-Pr | C(=O)Me | OCF₃ | H | O |
| D-5628 | Me(R) | single bond | H | *c*-Pr | C(=O)Me | OCF₃ | H | O |
| D-5629 | Me(S) | single bond | H | *c*-Pr | C(=O)Me | OCF₃ | H | O |
| D-5630 | H | CH₂ | Me | *c*-Pr | C(=O)Me | OCF₃ | H | O |
| D-5631 | Me | CH₂ | H | *c*-Pr | C(=O)Me | OCF₃ | H | O |
| D-5632 | H | CH(Me) | H | *c*-Pr | C(=O)Me | OCF₃ | H | O |
| D-5633 | H | CH₂CH₂ | Me | *c*-Pr | C(=O)Me | OCF₃ | H | O |
| D-5634 | Me | CH₂CH₂ | H | *c*-Pr | C(=O)Me | OCF₃ | H | O |
| D-5635 | H | single bond | Me | *c*-Pr | C(=O)Me | OCF₃ | H | O |
| D-5636 | Me | single bond | H | *c*-Pr | C(=O)Me | OCF₃ | H | O |
| D-5637 | Me(R) | single bond | H | *c*-Pr | C(=O)Me | OCF₃ | H | O |
| D-5638 | Me(S) | single bond | H | *c*-Pr | C(=O)Me | OCF₃ | H | O |
| D-5639 | H | CH₂ | Me | c-Pr | C(=O)Me | OCF₃ | H | O |
| D-5640 | Me | CH₂ | H | *c*-Pr | C(=O)Me | OCF₃ | H | O |
| D-5641 | H | CH(Me) | H | *c*-Pr | C(=O)Me | OCF₃ | H | O |
| D-5642 | H | CH₂CH₂ | Me | *c*-Pr | C(=O)Me | OCF₃ | H | O |
| D-5643 | Me | CH₂CH₂ | H | *c*-Pr | C(=O)Me | OCF₃ | H | O |
| D-5644 | H | single bond | Me | *c*-Pr | C(=O)Me | OCF₃ | H | O |
| D-5645 | Me | single bond | H | *c*-Pr | C(=O)Me | OCF₃ | H | O |
| D-5646 | Me(R) | single bond | H | *c*-Pr | C(=O)Me | OCF₃ | H | O |
| D-5647 | Me(S) | single bond | H | *c*-Pr | C(=O)Me | OCF₃ | H | O |
| D-5648 | H | CH₂ | Me | *c*-Pr | C(=O)Me | OCF₃ | H | O |
| D-5649 | Me | CH₂ | H | *c*-Pr | C(=O)Me | OCF₃ | H | O |
| D-5650 | H | CH(Me) | H | *c*-Pr | C(=O)Me | OCF₃ | H | O |
| D-5651 | H | CH₂CH₂ | Me | *c*-Pr | C(=O)Me | OCF₃ | H | O |
| D-5652 | Me | CH₂CH₂ | H | *c*-Pr | C(=O)Me | OCF₃ | H | O |
| D-5653 | H | single bond | Me | *c*-Pr | C(=O)Me | Br | Me | O |
| D-5654 | Me | single bond | H | *c*-Pr | C(=O)Me | Br | Me | O |
| D-5655 | Me(R) | single bond | H | *c*-Pr | C(=O)Me | Br | Me | O |
| D-5656 | Me(S) | single bond | H | *c*-Pr | C(=O)Me | Br | Me | O |
| D-5657 | H | CH₂ | Me | *c*-Pr | C(=O)Me | Br | Me | O |
| D-5658 | Me | CH₂ | H | *c*-Pr | C(=O)Me | Br | Me | O |
| D-5659 | H | CH(Me) | H | *c*-Pr | C(=O)Me | Br | Me | O |
| D-5660 | H | CH₂CH₂ | Me | *c*-Pr | C(=O)Me | Br | Me | O |
| D-5661 | Me | CH₂CH₂ | H | *c*-Pr | C(=O)Me | Br | Me | O |
| D-5662 | H | single bond | Me | *c*-Pr | C(=O)Me | Br | Me | O |
| D-5663 | Me | single bond | H | *c*-Pr | C(=O)Me | Br | Me | O |
| D-5664 | Me(R) | single bond | H | c-Pr | C(=O)Me | Br | Me | O |
| D-5665 | Me(S) | single bond | H | *c*-Pr | C(=O)Me | Br | Me | O |
| D-5666 | H | CH₂ | Me | *c*-Pr | C(=O)Me | Br | Me | O |
| D-5667 | Me | CH₂ | H | *c*-Pr | C(=O)Me | Br | Me | O |
| D-5668 | H | CH(Me) | H | *c*-Pr | C(=O)Me | Br | Me | O |
| D-5669 | H | CH₂CH₂ | Me | *c*-Pr | C(=O)Me | Br | Me | O |
| D-5670 | Me | CH₂CH₂ | H | *c*-Pr | C(=O)Me | Br | Me | O |

**[Table 774]**

| Compound Number | Y | W | Z | R¹ | R² | R³ | R¹⁰ | A |
|---|---|---|---|---|---|---|---|---|
| D-5671 | H | single bond | Me | *c*-Pr | C(=O)Me | Br | Me | O |
| D-5672 | Me | single bond | H | *c*-Pr | C(=O)Me | Br | Me | O |
| D-5673 | Me(R) | single bond | H | *c*-Pr | C(=O)Me | Br | Me | O |
| D-5674 | Me(S) | single bond | H | *c*-Pr | C(=O)Me | Br | Me | O |
| D-5675 | H | CH₂ | Me | *c*-Pr | C(=O)Me | Br | Me | O |
| D-5676 | Me | CH₂ | H | *c*-Pr | C(=O)Me | Br | Me | O |
| D-5677 | H | CH(Me) | H | *c*-Pr | C(=O)Me | Br | Me | O |
| D-5678 | H | CH₂CH₂ | Me | *c*-Pr | C(=O)Me | Br | Me | O |
| D-5679 | Me | CH₂CH₂ | H | *c*-Pr | C(=O)Me | Br | Me | O |
| D-5680 | H | single bond | Me | *c*-Pr | C(=O)Me | Br | Me | O |
| D-5681 | Me | single bond | H | *c*-Pr | C(=O)Me | Br | Me | O |
| D-5682 | Me(R) | single bond | H | *c*-Pr | C(=O)Me | Br | Me | O |
| D-5683 | Me(S) | single bond | H | *c*-Pr | C(=O)Me | Br | Me | O |
| D-5684 | H | CH₂ | Me | *c*-Pr | C(=O)Me | Br | Me | O |
| D-5685 | Me | CH₂ | H | *c*-Pr | C(=O)Me | Br | Me | O |
| D-5686 | H | CH(Me) | H | *c*-Pr | C(=O)Me | Br | Me | O |
| D-5687 | H | CH₂CH₂ | Me | *c*-Pr | C(=O)Me | Br | Me | O |
| D-5688 | Me | CH₂CH₂ | H | *c*-Pr | C(=O)Me | Br | Me | O |
| D-5689 | H | single bond | Me | *c*-Pr | C(=O)Me | CF₃ | Me | O |
| D-5690 | Me | single bond | H | *c*-Pr | C(=O)Me | CF₃ | Me | O |
| D-5691 | Me(R) | single bond | H | *c*-Pr | C(=O)Me | CF₃ | Me | O |
| D-5692 | Me(S) | single bond | H | *c*-Pr | C(=O)Me | CF₃ | Me | O |
| D-5693 | H | CH₂ | Me | *c*-Pr | C(=O)Me | CF₃ | Me | O |
| D-5694 | Me | CH₂ | H | *c*-Pr | C(=O)Me | CF₃ | Me | O |
| D-5695 | H | CH(Me) | H | *c*-Pr | C(=O)Me | CF₃ | Me | O |
| D-5696 | H | CH₂CH₂ | Me | *c*-Pr | C(=O)Me | CF₃ | Me | O |
| D-5697 | Me | CH₂CH₂ | H | *c*-Pr | C(=O)Me | CF₃ | Me | O |
| D-5698 | H | single bond | Me | *c*-Pr | C(=O)Me | CF₃ | Me | O |
| D-5699 | Me | single bond | H | *c*-Pr | C(=O)Me | CF₃ | Me | O |
| D-5700 | Me(R) | single bond | H | *c*-Pr | C(=O)Me | CF₃ | Me | O |
| D-5701 | Me(S) | single bond | H | *c*-Pr | C(=O)Me | CF₃ | Me | O |
| D-5702 | H | CH₂ | Me | *c*-Pr | C(=O)Me | CF₃ | Me | O |
| D-5703 | Me | CH₂ | H | *c*-Pr | C(=O)Me | CF₃ | Me | O |
| D-5704 | H | CH(Me) | H | *c*-Pr | C(=O)Me | CF₃ | Me | O |
| D-5705 | H | CH₂CH₂ | Me | *c*-Pr | C(=O)Me | CF₃ | Me | O |
| D-5706 | Me | CH₂CH₂ | H | *c*-Pr | C(=O)Me | CF₃ | Me | O |
| D-5707 | H | single bond | Me | *c*-Pr | C(=O)Me | CF₃ | Me | O |
| D-5708 | Me | single bond | H | *c*-Pr | C(=O)Me | CF₃ | Me | O |
| D-5709 | Me(R) | single bond | H | *c*-Pr | C(=O)Me | CF₃ | Me | O |
| D-5710 | Me(S) | single bond | H | *c*-Pr | C(=O)Me | CF₃ | Me | O |
| D-5711 | H | CH₂ | Me | *c*-Pr | C(=O)Me | CF₃ | Me | O |
| D-5712 | Me | CH₂ | H | *c*-Pr | C(=O)Me | CF₃ | Me | O |
| D-5713 | H | CH(Me) | H | *c*-Pr | C(=O)Me | CF₃ | Me | O |
| D-5714 | H | CH₂CH₂ | Me | *c*-Pr | C(=O)Me | CF₃ | Me | O |

**[Table 775]**

| Compound Number | Y | W | Z | R¹ | R² | R³ | R¹⁰ | A |
|---|---|---|---|---|---|---|---|---|
| D-5715 | Me | CH₂CH₂ | H | *c*-Pr | C(=O)Me | CF₃ | Me | O |
| D-5716 | H | single bond | Me | *c*-Pr | C(=O)Me | CF₃ | Me | O |
| D-5717 | Me | single bond | H | *c*-Pr | C(=O)Me | CF₃ | Me | O |
| D-5718 | Me(R) | single bond | H | *c*-Pr | C(=O)Me | CF₃ | Me | O |
| D-5719 | Me(S) | single bond | H | *c*-Pr | C(=O)Me | CF₃ | Me | O |
| D-5720 | H | CH₂ | Me | *c*-Pr | C(=O)Me | CF₃ | Me | O |
| D-5721 | Me | CH₂ | H | *c*-Pr | C(=O)Me | CF₃ | Me | O |
| D-5722 | H | CH(Me) | H | *c*-Pr | C(=O)Me | CF₃ | Me | O |
| D-5723 | H | CH₂CH₂ | Me | *c*-Pr | C(=O)Me | CF₃ | Me | O |
| D-5724 | Me | CH₂CH₂ | H | *c*-Pr | C(=O)Me | CF₃ | Me | O |
| D-5725 | H | single bond | Me | *c*-Pr | C(=O)Me | OMe | Me | O |
| D-5726 | Me | single bond | H | *c*-Pr | C(=O)Me | OMe | Me | O |
| D-5727 | Me(R) | single bond | H | *c*-Pr | C(=O)Me | OMe | Me | O |
| D-5728 | Me(S) | single bond | H | *c*-Pr | C(=O)Me | OMe | Me | O |
| D-5729 | H | CH₂ | Me | *c*-Pr | C(=O)Me | OMe | Me | O |
| D-5730 | Me | CH₂ | H | *c*-Pr | C(=O)Me | OMe | Me | O |
| D-5731 | H | CH(Me) | H | *c*-Pr | C(=O)Me | OMe | Me | O |
| D-5732 | H | CH₂CH₂ | Me | *c*-Pr | C(=O)Me | OMe | Me | O |
| D-5733 | Me | CH₂CH₂ | H | *c*-Pr | C(=O)Me | OMe | Me | O |
| D-5734 | H | single bond | Me | *c*-Pr | C(=O)Me | OMe | Me | O |
| D-5735 | Me | single bond | H | *c*-Pr | C(=O)Me | OMe | Me | O |
| D-5736 | Me(R) | single bond | H | *c*-Pr | C(=O)Me | OMe | Me | O |
| D-5737 | Me(S) | single bond | H | *c*-Pr | C(=O)Me | OMe | Me | O |
| D-5738 | H | CH₂ | Me | *c*-Pr | C(=O)Me | OMe | Me | O |
| D-5739 | Me | CH₂ | H | *c*-Pr | C(=O)Me | OMe | Me | O |
| D-5740 | H | CH(Me) | H | *c*-Pr | C(=O)Me | OMe | Me | O |
| D-5741 | H | CH₂CH₂ | Me | *c*-Pr | C(=O)Me | OMe | Me | O |
| D-5742 | Me | CH₂CH₂ | H | *c*-Pr | C(=O)Me | OMe | Me | O |
| D-5743 | H | single bond | Me | *c*-Pr | C(=O)Me | OMe | Me | O |
| D-5744 | Me | single bond | H | *c*-Pr | C(=O)Me | OMe | Me | O |
| D-5745 | Me(R) | single bond | H | *c*-Pr | C(=O)Me | OMe | Me | O |
| D-5746 | Me(S) | single bond | H | *c*-Pr | C(=O)Me | OMe | Me | O |
| D-5747 | H | CH₂ | Me | *c*-Pr | C(=O)Me | OMe | Me | O |
| D-5748 | Me | CH₂ | H | *c*-Pr | C(=O)Me | OMe | Me | O |
| D-5749 | H | CH(Me) | H | *c*-Pr | C(=O)Me | OMe | Me | O |
| D-5750 | H | CH₂CH₂ | Me | *c*-Pr | C(=O)Me | OMe | Me | O |
| D-5751 | Me | CH₂CH₂ | H | *c*-Pr | C(=O)Me | OMe | Me | O |
| D-5752 | H | single bond | Me | *c*-Pr | C(=O)Me | OMe | Me | O |
| D-5753 | Me | single bond | H | *c*-Pr | C(=O)Me | OMe | Me | O |
| D-5754 | Me(R) | single bond | H | *c*-Pr | C(=O)Me | OMe | Me | O |
| D-5755 | Me(S) | single bond | H | *c*-Pr | C(=O)Me | OMe | Me | O |
| D-5756 | H | CH₂ | Me | *c*-Pr | C(=O)Me | OMe | Me | O |
| D-5757 | Me | CH₂ | H | *c*-Pr | C(=O)Me | OMe | Me | O |
| D-5758 | H | CH(Me) | H | *c*-Pr | C(=O)Me | OMe | Me | O |

**[Table 776]**

| Compound Number | Y | W | Z | R¹ | R² | R³ | R¹⁰ | A |
|---|---|---|---|---|---|---|---|---|
| D-5759 | H | CH₂CH₂ | Me | *c*-Pr | C(=O)Me | OMe | Me | O |
| D-5760 | Me | CH₂CH₂ | H | *c*-Pr | C(=O)Me | OMe | Me | O |
| D-5761 | H | single bond | Me | *c*-Pr | C(=O)Me | OCF₃ | Me | O |
| D-5762 | Me | single bond | H | *c*-Pr | C(=O)Me | OCF₃ | Me | O |
| D-5763 | Me(R) | single bond | H | *c*-Pr | C(=O)Me | OCF₃ | Me | O |
| D-5764 | Me(S) | single bond | H | *c*-Pr | C(=O)Me | OCF₃ | Me | O |
| D-5765 | H | CH₂ | Me | *c*-Pr | C(=O)Me | OCF₃ | Me | O |
| D-5766 | Me | CH₂ | H | *c*-Pr | C(=O)Me | OCF₃ | Me | O |
| D-5767 | H | CH(Me) | H | *c*-Pr | C(=O)Me | OCF₃ | Me | O |
| D-5768 | H | CH₂CH₂ | Me | *c*-Pr | C(=O)Me | OCF₃ | Me | O |
| D-5769 | Me | CH₂CH₂ | H | *c*-Pr | C(=O)Me | OCF₃ | Me | O |
| D-5770 | H | single bond | Me | *c*-Pr | C(=O)Me | OCF₃ | Me | O |
| D-5771 | Me | single bond | H | *c*-Pr | C(=O)Me | OCF₃ | Me | O |
| D-5772 | Me(R) | single bond | H | *c*-Pr | C(=O)Me | OCF₃ | Me | O |
| D-5773 | Me(S) | single bond | H | *c*-Pr | C(=O)Me | OCF₃ | Me | O |
| D-5774 | H | CH₂ | Me | *c*-Pr | C(=O)Me | OCF₃ | Me | O |
| D-5775 | Me | CH₂ | H | *c*-Pr | C(=O)Me | OCF₃ | Me | O |
| D-5776 | H | CH(Me) | H | *c*-Pr | C(=O)Me | OCF₃ | Me | O |
| D-5777 | H | CH₂CH₂ | Me | *c*-Pr | C(=O)Me | OCF₃ | Me | O |
| D-5778 | Me | CH₂CH₂ | H | *c*-Pr | C(=O)Me | OCF₃ | Me | O |
| D-5779 | H | single bond | Me | *c*-Pr | C(=O)Me | OCF₃ | Me | O |
| D-5780 | Me | single bond | H | *c*-Pr | C(=O)Me | OCF₃ | Me | O |
| D-5781 | Me(R) | single bond | H | *c*-Pr | C(=O)Me | OCF₃ | Me | O |
| D-5782 | Me(S) | single bond | H | *c*-Pr | C(=O)Me | OCF₃ | Me | O |
| D-5783 | H | CH₂ | Me | *c*-Pr | C(=O)Me | OCF₃ | Me | O |
| D-5784 | Me | CH₂ | H | *c*-Pr | C(=O)Me | OCF₃ | Me | O |
| D-5785 | H | CH(Me) | H | *c*-Pr | C(=O)Me | OCF₃ | Me | O |
| D-5786 | H | CH₂CH₂ | Me | *c*-Pr | C(=O)Me | OCF₃ | Me | O |
| D-5787 | Me | CH₂CH₂ | H | *c*-Pr | C(=O)Me | OCF₃ | Me | O |
| D-5788 | H | single bond | Me | *c*-Pr | C(=O)Me | OCF₃ | Me | O |
| D-5789 | Me | single bond | H | *c*-Pr | C(=O)Me | OCF₃ | Me | O |
| D-5790 | Me(R) | single bond | H | *c*-Pr | C(=O)Me | OCF₃ | Me | O |
| D-5791 | Me(S) | single bond | H | *c*-Pr | C(=O)Me | OCF₃ | Me | O |
| D-5792 | H | CH₂ | Me | *c*-Pr | C(=O)Me | OCF₃ | Me | O |
| D-5793 | Me | CH₂ | H | *c*-Pr | C(=O)Me | OCF₃ | Me | O |
| D-5794 | H | CH(Me) | H | *c*-Pr | C(=O)Me | OCF₃ | Me | O |
| D-5795 | H | CH₂CH₂ | Me | *c*-Pr | C(=O)Me | OCF₃ | Me | O |
| D-5796 | Me | CH₂CH₂ | H | *c*-Pr | C(=O)Me | OCF₃ | Me | O |
| D-5797 | H | single bond | Me | *c*-Pr | C(=O)Me | Br | CH₂OMe | O |
| D-5798 | Me | single bond | H | *c*-Pr | C(=O)Me | Br | CH₂OMe | O |
| D-5799 | Me(R) | single bond | H | *c*-Pr | C(=O)Me | Br | CH₂OMe | O |
| D-5800 | Me(S) | single bond | H | *c*-Pr | C(=O)Me | Br | CH₂OMe | O |
| D-5801 | H | CH₂ | Me | *c*-Pr | C(=O)Me | Br | CH₂OMe | O |
| D-5802 | Me | CH₂ | H | *c*-Pr | C(=O)Me | Br | CH₂OMe | O |

**[Table 777]**

| Compound Number | Y | W | Z | R¹ | R² | R³ | R¹⁰ | A |
|---|---|---|---|---|---|---|---|---|
| D-5803 | H | CH(Me) | H | *c*-Pr | C(=O)Me | Br | CH₂OMe | O |
| D-5804 | H | CH₂CH₂ | Me | *c*-Pr | C(=O)Me | Br | CH₂OMe | O |
| D-5805 | Me | CH₂CH₂ | H | *c*-Pr | C(=O)Me | Br | CH₂OMe | O |
| D-5806 | H | single bond | Me | *c*-Pr | C(=O)Me | Br | CH₂OMe | O |
| D-5807 | Me | single bond | H | *c*-Pr | C(=O)Me | Br | CH₂OMe | O |
| D-5808 | Me(R) | single bond | H | *c*-Pr | C(=O)Me | Br | CH₂OMe | O |
| D-5809 | Me(S) | single bond | H | *c*-Pr | C(=O)Me | Br | CH₂OMe | O |
| D-5810 | H | CH₂ | Me | *c*-Pr | C(=O)Me | Br | CH₂OMe | O |
| D-5811 | Me | CH₂ | H | *c*-Pr | C(=O)Me | Br | CH₂OMe | O |
| D-5812 | H | CH(Me) | H | *c*-Pr | C(=O)Me | Br | CH₂OMe | O |
| D-5813 | H | CH₂CH₂ | Me | *c*-Pr | C(=O)Me | Br | CH₂OMe | O |
| D-5814 | Me | CH₂CH₂ | H | *c*-Pr | C(=O)Me | Br | CH₂OMe | O |
| D-5815 | H | single bond | Me | *c*-Pr | C(=O)Me | Br | CH₂OMe | O |
| D-5816 | Me | single bond | H | *c*-Pr | C(=O)Me | Br | CH₂OMe | O |
| D-5817 | Me(R) | single bond | H | *c*-Pr | C(=O)Me | Br | CH₂OMe | O |
| D-5818 | Me(S) | single bond | H | *c*-Pr | C(=O)Me | Br | CH₂OMe | O |
| D-5819 | H | CH₂ | Me | *c*-Pr | C(=O)Me | Br | CH₂OMe | O |
| D-5820 | Me | CH₂ | H | *c*-Pr | C(=O)Me | Br | CH₂OMe | O |
| D-5821 | H | CH(Me) | H | *c*-Pr | C(=O)Me | Br | CH₂OMe | O |
| D-5822 | H | CH₂CH₂ | Me | *c*-Pr | C(=O)Me | Br | CH₂OMe | O |
| D-5823 | Me | CH₂CH₂ | H | *c*-Pr | C(=O)Me | Br | CH₂OMe | O |
| D-5824 | H | single bond | Me | *c*-Pr | C(=O)Me | Br | CH₂OMe | O |
| D-5825 | Me | single bond | H | *c*-Pr | C(=O)Me | Br | CH₂OMe | O |
| D-5826 | Me(R) | single bond | H | *c*-Pr | C(=O)Me | Br | CH₂OMe | O |
| D-5827 | Me(S) | single bond | H | *c*-Pr | C(=O)Me | Br | CH₂OMe | O |
| D-5828 | H | CH₂ | Me | *c*-Pr | C(=O)Me | Br | CH₂OMe | O |
| D-5829 | Me | CH₂ | H | *c*-Pr | C(=O)Me | Br | CH₂OMe | O |
| D-5830 | H | CH(Me) | H | *c*-Pr | C(=O)Me | Br | CH₂OMe | O |
| D-5831 | H | CH₂CH₂ | Me | *c*-Pr | C(=O)Me | Br | CH₂OMe | O |
| D-5832 | Me | CH₂CH₂ | H | *c*-Pr | C(=O)Me | Br | CH₂OMe | O |
| D-5833 | H | single bond | Me | *c*-Pr | C(=O)Me | CF₃ | CH₂OMe | O |
| D-5834 | Me | single bond | H | *c*-Pr | C(=O)Me | CF₃ | CH₂OMe | O |
| D-5835 | Me(R) | single bond | H | *c*-Pr | C(=O)Me | CF₃ | CH₂OMe | O |
| D-5836 | Me(S) | single bond | H | *c*-Pr | C(=O)Me | CF₃ | CH₂OMe | O |
| D-5837 | H | CH₂ | Me | *c*-Pr | C(=O)Me | CF₃ | CH₂OMe | O |
| D-5838 | Me | CH₂ | H | *c*-Pr | C(=O)Me | CF₃ | CH₂OMe | O |
| D-5839 | H | CH(Me) | H | *c*-Pr | C(=O)Me | CF₃ | CH₂OMe | O |
| D-5840 | H | CH₂CH₂ | Me | *c*-Pr | C(=O)Me | CF₃ | CH₂OMe | O |
| D-5841 | Me | CH₂CH₂ | H | *c*-Pr | C(=O)Me | CF₃ | CH₂OMe | O |
| D-5842 | H | single bond | Me | *c*-Pr | C(=O)Me | CF₃ | CH₂OMe | O |
| D-5843 | Me | single bond | H | *c*-Pr | C(=O)Me | CF₃ | CH₂OMe | O |
| D-5844 | Me(R) | single bond | H | *c*-Pr | C(=O)Me | CF₃ | CH₂OMe | O |
| D-5845 | Me(S) | single bond | H | *c*-Pr | C(=O)Me | CF₃ | CH₂OMe | O |
| D-5846 | H | CH₂ | Me | *c*-Pr | C(=O)Me | CF₃ | CH₂OMe | O |

**[Table 778]**

| Compound Number | Y | W | Z | R¹ | R² | R³ | R¹⁰ | A |
|---|---|---|---|---|---|---|---|---|
| D-5847 | Me | CH₂ | H | *c*-Pr | C(=O)Me | CF₃ | CH₂OMe | O |
| D-5848 | H | CH(Me) | H | *c*-Pr | C(=O)Me | CF₃ | CH₂OMe | O |
| D-5849 | H | CH₂CH₂ | Me | *c*-Pr | C(=O)Me | CF₃ | CH₂OMe | O |
| D-5850 | Me | CH₂CH₂ | H | *c*-Pr | C(=O)Me | CF₃ | CH₂OMe | O |
| D-5851 | H | single bond | Me | *c*-Pr | C(=O)Me | CF₃ | CH₂OMe | O |
| D-5852 | Me | single bond | H | c-Pr | C(=O)Me | CF₃ | CH₂OMe | O |
| D-5853 | Me(R) | single bond | H | *c*-Pr | C(=O)Me | CF₃ | CH₂OMe | O |
| D-5854 | Me(S) | single bond | H | *c*-Pr | C(=O)Me | CF₃ | CH₂OMe | O |
| D-5855 | H | CH₂ | Me | *c*-Pr | C(=O)Me | CF₃ | CH₂OMe | O |
| D-5856 | Me | CH₂ | H | *c*-Pr | C(=O)Me | CF₃ | CH₂OMe | O |
| D-5857 | H | CH(Me) | H | *c*-Pr | C(=O)Me | CF₃ | CH₂OMe | O |
| D-5858 | H | CH₂CH₂ | Me | *c*-Pr | C(=O)Me | CF₃ | CH₂OMe | O |
| D-5859 | Me | CH₂CH₂ | H | *c*-Pr | C(=O)Me | CF₃ | CH₂OMe | O |
| D-5860 | H | single bond | Me | *c*-Pr | C(=O)Me | CF₃ | CH₂OMe | O |
| D-5861 | Me | single bond | H | *c*-Pr | C(=O)Me | CF₃ | CH₂OMe | O |
| D-5862 | Me(R) | single bond | H | *c*-Pr | C(=O)Me | CF₃ | CH₂OMe | O |
| D-5863 | Me(S) | single bond | H | *c*-Pr | C(=O)Me | CF₃ | CH₂OMe | O |
| D-5864 | H | CH₂ | Me | *c*-Pr | C(=O)Me | CF₃ | CH₂OMe | O |
| D-5865 | Me | CH₂ | H | *c*-Pr | C(=O)Me | CF₃ | CH₂OMe | O |
| D-5866 | H | CH(Me) | H | *c*-Pr | C(=O)Me | CF₃ | CH₂OMe | O |
| D-5867 | H | CH₂CH₂ | Me | *c*-Pr | C(=O)Me | CF₃ | CH₂OMe | O |
| D-5868 | Me | CH₂CH₂ | H | *c*-Pr | C(=O)Me | CF₃ | CH₂OMe | O |
| D-5869 | H | single bond | Me | *c*-Pr | C(=O)Me | OMe | CH₂OMe | O |
| D-5870 | Me | single bond | H | *c*-Pr | C(=O)Me | OMe | CH₂OMe | O |
| D-5871 | Me(R) | single bond | H | *c*-Pr | C(=O)Me | OMe | CH₂OMe | O |
| D-5872 | Me(S) | single bond | H | *c*-Pr | C(=O)Me | OMe | CH₂OMe | O |
| D-5873 | H | CH₂ | Me | *c*-Pr | C(=O)Me | OMe | CH₂OMe | O |
| D-5874 | Me | CH₂ | H | *c*-Pr | C(=O)Me | OMe | CH₂OMe | O |
| D-5875 | H | CH(Me) | H | *c*-Pr | C(=O)Me | OMe | CH₂OMe | O |
| D-5876 | H | CH₂CH₂ | Me | *c*-Pr | C(=O)Me | OMe | CH₂OMe | O |
| D-5877 | Me | CH₂CH₂ | H | *c*-Pr | C(=O)Me | OMe | CH₂OMe | O |
| D-5878 | H | single bond | Me | *c*-Pr | C(=O)Me | OMe | CH₂OMe | O |
| D-5879 | Me | single bond | H | *c*-Pr | C(=O)Me | OMe | CH₂OMe | O |
| D-5880 | Me(R) | single bond | H | *c*-Pr | C(=O)Me | OMe | CH₂OMe | O |
| D-5881 | Me(S) | single bond | H | *c*-Pr | C(=O)Me | OMe | CH₂OMe | O |
| D-5882 | H | CH₂ | Me | *c*-Pr | C(=O)Me | OMe | CH₂OMe | O |
| D-5883 | Me | CH₂ | H | *c*-Pr | C(=O)Me | OMe | CH₂OMe | O |
| D-5884 | H | CH(Me) | H | *c*-Pr | C(=O)Me | OMe | CH₂OMe | O |
| D-5885 | H | CH₂CH₂ | Me | *c*-Pr | C(=O)Me | OMe | CH₂OMe | O |
| D-5886 | Me | CH₂CH₂ | H | *c*-Pr | C(=O)Me | OMe | CH₂OMe | O |
| D-5887 | H | single bond | Me | *c*-Pr | C(=O)Me | OMe | CH₂OMe | O |
| D-5888 | Me | single bond | H | *c*-Pr | C(=O)Me | OMe | CH₂OMe | O |
| D-5889 | Me(R) | single bond | H | *c*-Pr | C(=O)Me | OMe | CH₂OMe | O |
| D-5890 | Me(S) | single bond | H | *c*-Pr | C(=O)Me | OMe | CH₂OMe | O |

**[Table 779]**

| Compound Number | Y | W | Z | R¹ | R² | R³ | R¹⁰ | A |
|---|---|---|---|---|---|---|---|---|
| D-5891 | H | CH₂ | Me | *c*-Pr | C(=O)Me | OMe | CH₂OMe | O |
| D-5892 | Me | CH₂ | H | *c*-Pr | C(=O)Me | OMe | CH₂OMe | O |
| D-5893 | H | CH(Me) | H | *c*-Pr | C(=O)Me | OMe | CH₂OMe | O |
| D-5894 | H | CH₂CH₂ | Me | *c*-Pr | C(=O)Me | OMe | CH₂OMe | O |
| D-5895 | Me | CH₂CH₂ | H | *c*-Pr | C(=O)Me | OMe | CH₂OMe | O |
| D-5896 | H | single bond | Me | *c*-Pr | C(=O)Me | OMe | CH₂OMe | O |
| D-5897 | Me | single bond | H | *c*-Pr | C(=O)Me | OMe | CH₂OMe | O |
| D-5898 | Me(R) | single bond | H | *c*-Pr | C(=O)Me | OMe | CH₂OMe | O |
| D-5899 | Me(S) | single bond | H | *c*-Pr | C(=O)Me | OMe | CH₂OMe | O |
| D-5900 | H | CH₂ | Me | *c*-Pr | C(=O)Me | OMe | CH₂OMe | O |
| D-5901 | Me | CH₂ | H | *c*-Pr | C(=O)Me | OMe | CH₂OMe | O |
| D-5902 | H | CH(Me) | H | *c*-Pr | C(=O)Me | OMe | CH₂OMe | O |
| D-5903 | H | CH₂CH₂ | Me | *c*-Pr | C(=O)Me | OMe | CH₂OMe | O |
| D-5904 | Me | CH₂CH₂ | H | *c*-Pr | C(=O)Me | OMe | CH₂OMe | O |
| D-5905 | H | single bond | Me | *c*-Pr | C(=O)Me | OCF₃ | CH₂OMe | O |
| D-5906 | Me | single bond | H | *c*-Pr | C(=O)Me | OCF₃ | CH₂OMe | O |
| D-5907 | Me(R) | single bond | H | *c*-Pr | C(=O)Me | OCF₃ | CH₂OMe | O |
| D-5908 | Me(S) | single bond | H | *c*-Pr | C(=O)Me | OCF₃ | CH₂OMe | O |
| D-5909 | H | CH₂ | Me | *c*-Pr | C(=O)Me | OCF₃ | CH₂OMe | O |
| D-5910 | Me | CH₂ | H | *c*-Pr | C(=O)Me | OCF₃ | CH₂OMe | O |
| D-5911 | H | CH(Me) | H | *c*-Pr | C(=O)Me | OCF₃ | CH₂OMe | O |
| D-5912 | H | CH₂CH₂ | Me | *c*-Pr | C(=O)Me | OCF₃ | CH₂OMe | O |
| D-5913 | Me | CH₂CH₂ | H | *c*-Pr | C(=O)Me | OCF₃ | CH₂OMe | O |
| D-5914 | H | single bond | Me | *c*-Pr | C(=O)Me | OCF₃ | CH₂OMe | O |
| D-5915 | Me | single bond | H | *c*-Pr | C(=O)Me | OCF₃ | CH₂OMe | O |
| D-5916 | Me(R) | single bond | H | *c*-Pr | C(=O)Me | OCF₃ | CH₂OMe | O |
| D-5917 | Me(S) | single bond | H | *c*-Pr | C(=O)Me | OCF₃ | CH₂OMe | O |
| D-5918 | H | CH₂ | Me | *c*-Pr | C(=O)Me | OCF₃ | CH₂OMe | O |
| D-5919 | Me | CH₂ | H | *c*-Pr | C(=O)Me | OCF₃ | CH₂OMe | O |
| D-5920 | H | CH(Me) | H | *c*-Pr | C(=O)Me | OCF₃ | CH₂OMe | O |
| D-5921 | H | CH₂CH₂ | Me | *c*-Pr | C(=O)Me | OCF₃ | CH₂OMe | O |
| D-5922 | Me | CH₂CH₂ | H | *c*-Pr | C(=O)Me | OCF₃ | CH₂OMe | O |
| D-5923 | H | single bond | Me | *c*-Pr | C(=O)Me | OCF₃ | CH₂OMe | O |
| D-5924 | Me | single bond | H | *c*-Pr | C(=O)Me | OCF₃ | CH₂OMe | O |
| D-5925 | Me(R) | single bond | H | *c*-Pr | C(=O)Me | OCF₃ | CH₂OMe | O |
| D-5926 | Me(S) | single bond | H | *c*-Pr | C(=O)Me | OCF₃ | CH₂OMe | O |
| D-5927 | H | CH₂ | Me | *c*-Pr | C(=O)Me | OCF₃ | CH₂OMe | O |
| D-5928 | Me | CH₂ | H | c-Pr | C(=O)Me | OCF₃ | CH₂OMe | O |
| D-5929 | H | CH(Me) | H | *c*-Pr | C(=O)Me | OCF₃ | CH₂OMe | O |
| D-5930 | H | CH₂CH₂ | Me | *c*-Pr | C(=O)Me | OCF₃ | CH₂OMe | O |
| D-5931 | Me | CH₂CH₂ | H | *c*-Pr | C(=O)Me | OCF₃ | CH₂OMe | O |
| D-5932 | H | single bond | Me | *c*-Pr | C(=O)Me | OCF₃ | CH₂OMe | O |
| D-5933 | Me | single bond | H | *c*-Pr | C(=O)Me | OCF₃ | CH₂OMe | O |
| D-5934 | Me(R) | single bond | H | *c*-Pr | C(=O)Me | OCF₃ | CH₂OMe | O |

**[Table 780]**

| Compound Number | Y | W | Z | R¹ | R² | R³ | R¹⁰ | A |
|---|---|---|---|---|---|---|---|---|
| D-5935 | Me(S) | single bond | H | *c*-Pr | C(=O)Me | OCF₃ | CH₂OMe | O |
| D-5936 | H | CH₂ | Me | *c*-Pr | C(=O)Me | OCF₃ | CH₂OMe | O |
| D-5937 | Me | CH₂ | H | *c*-Pr | C(=O)Me | OCF₃ | CH₂OMe | O |
| D-5938 | H | CH(Me) | H | *c*-Pr | C(=O)Me | OCF₃ | CH₂OMe | O |
| D-5939 | H | CH₂CH₂ | Me | *c*-Pr | C(=O)Me | OCF₃ | CH₂OMe | O |
| D-5940 | Me | CH₂CH₂ | H | *c*-Pr | C(=O)Me | OCF₃ | CH₂OMe | O |
| D-5941 | H | single bond | Me | *c*-Pr | C(=O)Me | Br | CH₂CN | O |
| D-5942 | Me | single bond | H | *c*-Pr | C(=O)Me | Br | CH₂CN | O |
| D-5943 | Me(R) | single bond | H | *c*-Pr | C(=O)Me | Br | CH₂CN | O |
| D-5944 | Me(S) | single bond | H | *c*-Pr | C(=O)Me | Br | CH₂CN | O |
| D-5945 | H | CH₂ | Me | *c*-Pr | C(=O)Me | Br | CH₂CN | O |
| D-5946 | Me | CH₂ | H | *c*-Pr | C(=O)Me | Br | CH₂CN | O |
| D-5947 | H | CH(Me) | H | *c*-Pr | C(=O)Me | Br | CH₂CN | O |
| D-5948 | H | CH₂CH₂ | Me | *c*-Pr | C(=O)Me | Br | CH₂CN | O |
| D-5949 | Me | CH₂CH₂ | H | *c*-Pr | C(=O)Me | Br | CH₂CN | O |
| D-5950 | H | single bond | Me | *c*-Pr | C(=O)Me | Br | CH₂CN | O |
| D-5951 | Me | single bond | H | *c*-Pr | C(=O)Me | Br | CH₂CN | O |
| D-5952 | Me(R) | single bond | H | *c*-Pr | C(=O)Me | Br | CH₂CN | O |
| D-5953 | Me(S) | single bond | H | *c*-Pr | C(=O)Me | Br | CH₂CN | O |
| D-5954 | H | CH₂ | Me | *c*-Pr | C(=O)Me | Br | CH₂CN | O |
| D-5955 | Me | CH₂ | H | *c*-Pr | C(=O)Me | Br | CH₂CN | O |
| D-5956 | H | CH(Me) | H | *c*-Pr | C(=O)Me | Br | CH₂CN | O |
| D-5957 | H | CH₂CH₂ | Me | *c*-Pr | C(=O)Me | Br | CH₂CN | O |
| D-5958 | Me | CH₂CH₂ | H | *c*-Pr | C(=O)Me | Br | CH₂CN | O |
| D-5959 | H | single bond | Me | *c*-Pr | C(=O)Me | Br | CH₂CN | O |
| D-5960 | Me | single bond | H | *c*-Pr | C(=O)Me | Br | CH₂CN | O |
| D-5961 | Me(R) | single bond | H | *c*-Pr | C(=O)Me | Br | CH₂CN | O |
| D-5962 | Me(S) | single bond | H | *c*-Pr | C(=O)Me | Br | CH₂CN | O |
| D-5963 | H | CH₂ | Me | *c*-Pr | C(=O)Me | Br | CH₂CN | O |
| D-5964 | Me | CH₂ | H | *c*-Pr | C(=O)Me | Br | CH₂CN | O |
| D-5965 | H | CH(Me) | H | *c*-Pr | C(=O)Me | Br | CH₂CN | O |
| D-5966 | H | CH₂CH₂ | Me | *c*-Pr | C(=O)Me | Br | CH₂CN | O |
| D-5967 | Me | CH₂CH₂ | H | *c*-Pr | C(=O)Me | Br | CH₂CN | O |
| D-5968 | H | single bond | Me | *c*-Pr | C(=O)Me | Br | CH₂CN | O |
| D-5969 | Me | single bond | H | *c*-Pr | C(=O)Me | Br | CH₂CN | O |
| D-5970 | Me(R) | single bond | H | *c*-Pr | C(=O)Me | Br | CH₂CN | O |
| D-5971 | Me(S) | single bond | H | *c*-Pr | C(=O)Me | Br | CH₂CN | O |
| D-5972 | H | CH₂ | Me | *c*-Pr | C(=O)Me | Br | CH₂CN | O |
| D-5973 | Me | CH₂ | H | *c*-Pr | C(=O)Me | Br | CH₂CN | O |
| D-5974 | H | CH(Me) | H | *c*-Pr | C(=O)Me | Br | CH₂CN | O |
| D-5975 | H | CH₂CH₂ | Me | *c*-Pr | C(=O)Me | Br | CH₂CN | O |
| D-5976 | Me | CH₂CH₂ | H | *c*-Pr | C(=O)Me | Br | CH₂CN | O |
| D-5977 | H | single bond | Me | *c*-Pr | C(=O)Me | CF₃ | CH₂CN | O |
| D-5978 | Me | single bond | H | *c*-Pr | C(=O)Me | CF₃ | CH₂CN | O |

**[Table 781]**

| Compound Number | Y | W | Z | R¹ | R² | R³ | R¹⁰ | A |
|---|---|---|---|---|---|---|---|---|
| D-5979 | Me(R) | single bond | H | *c*-Pr | C(=O)Me | CF₃ | CH₂CN | O |
| D-5980 | Me(S) | single bond | H | *c*-Pr | C(=O)Me | CF₃ | CH₂CN | O |
| D-5981 | H | CH₂ | Me | *c*-Pr | C(=O)Me | CF₃ | CH₂CN | O |
| D-5982 | Me | CH₂ | H | *c*-Pr | C(=O)Me | CF₃ | CH₂CN | O |
| D-5983 | H | CH(Me) | H | *c*-Pr | C(=O)Me | CF₃ | CH₂CN | O |
| D-5984 | H | CH₂CH₂ | Me | *c*-Pr | C(=O)Me | CF₃ | CH₂CN | O |
| D-5985 | Me | CH₂CH₂ | H | *c*-Pr | C(=O)Me | CF₃ | CH₂CN | O |
| D-5986 | H | single bond | Me | *c*-Pr | C(=O)Me | CF₃ | CH₂CN | O |
| D-5987 | Me | single bond | H | *c*-Pr | C(=O)Me | CF₃ | CH₂CN | O |
| D-5988 | Me(R) | single bond | H | *c*-Pr | C(=O)Me | CF₃ | CH₂CN | O |
| D-5989 | Me(S) | single bond | H | *c*-Pr | C(=O)Me | CF₃ | CH₂CN | O |
| D-5990 | H | CH₂ | Me | *c*-Pr | C(=O)Me | CF₃ | CH₂CN | O |
| D-5991 | Me | CH₂ | H | *c*-Pr | C(=O)Me | CF₃ | CH₂CN | O |
| D-5992 | H | CH(Me) | H | *c*-Pr | C(=O)Me | CF₃ | CH₂CN | O |
| D-5993 | H | CH₂CH₂ | Me | *c*-Pr | C(=O)Me | CF₃ | CH₂CN | O |
| D-5994 | Me | CH₂CH₂ | H | *c*-Pr | C(=O)Me | CF₃ | CH₂CN | O |
| D-5995 | H | single bond | Me | *c*-Pr | C(=O)Me | CF₃ | CH₂CN | O |
| D-5996 | Me | single bond | H | *c*-Pr | C(=O)Me | CF₃ | CH₂CN | O |
| D-5997 | Me(R) | single bond | H | *c*-Pr | C(=O)Me | CF₃ | CH₂CN | O |
| D-5998 | Me(S) | single bond | H | *c*-Pr | C(=O)Me | CF₃ | CH₂CN | O |
| D-5999 | H | CH₂ | Me | *c*-Pr | C(=O)Me | CF₃ | CH₂CN | O |
| D-6000 | Me | CH₂ | H | *c*-Pr | C(=O)Me | CF₃ | CH₂CN | O |
| D-6001 | H | CH(Me) | H | *c*-Pr | C(=O)Me | CF₃ | CH₂CN | O |
| D-6002 | H | CH₂CH₂ | Me | *c*-Pr | C(=O)Me | CF₃ | CH₂CN | O |
| D-6003 | Me | CH₂CH₂ | H | *c*-Pr | C(=O)Me | CF₃ | CH₂CN | O |
| D-6004 | H | single bond | Me | *c*-Pr | C(=O)Me | CF₃ | CH₂CN | O |
| D-6005 | Me | single bond | H | *c*-Pr | C(=O)Me | CF₃ | CH₂CN | O |
| D-6006 | Me(R) | single bond | H | *c*-Pr | C(=O)Me | CF₃ | CH₂CN | O |
| D-6007 | Me(S) | single bond | H | *c*-Pr | C(=O)Me | CF₃ | CH₂CN | O |
| D-6008 | H | CH₂ | Me | *c*-Pr | C(=O)Me | CF₃ | CH₂CN | O |
| D-6009 | Me | CH₂ | H | *c*-Pr | C(=O)Me | CF₃ | CH₂CN | O |
| D-6010 | H | CH(Me) | H | *c*-Pr | C(=O)Me | CF₃ | CH₂CN | O |
| D-6011 | H | CH₂CH₂ | Me | *c*-Pr | C(=O)Me | CF₃ | CH₂CN | O |
| D-6012 | Me | CH₂CH₂ | H | *c*-Pr | C(=O)Me | CF₃ | CH₂CN | O |
| D-6013 | H | single bond | Me | *c*-Pr | C(=O)Me | OMe | CH₂CN | O |
| D-6014 | Me | single bond | H | *c*-Pr | C(=O)Me | OMe | CH₂CN | O |
| D-6015 | Me(R) | single bond | H | *c*-Pr | C(=O)Me | OMe | CH₂CN | O |
| D-6016 | Me(S) | single bond | H | *c*-Pr | C(=O)Me | OMe | CH₂CN | O |
| D-6017 | H | CH₂ | Me | *c*-Pr | C(=O)Me | OMe | CH₂CN | O |
| D-6018 | Me | CH₂ | H | *c*-Pr | C(=O)Me | OMe | CH₂CN | O |
| D-6019 | H | CH(Me) | H | *c*-Pr | C(=O)Me | OMe | CH₂CN | O |
| D-6020 | H | CH₂CH₂ | Me | *c*-Pr | C(=O)Me | OMe | CH₂CN | O |
| D-6021 | Me | CH₂CH₂ | H | *c*-Pr | C(=O)Me | OMe | CH₂CN | O |
| D-6022 | H | single bond | Me | *c*-Pr | C(=O)Me | OMe | CH₂CN | O |

**[Table 782]**

| Compound Number | Y | W | Z | R¹ | R² | R³ | R¹⁰ | A |
|---|---|---|---|---|---|---|---|---|
| D-6023 | Me | single bond | H | *c*-Pr | C(=O)Me | OMe | CH₂CN | O |
| D-6024 | Me(R) | single bond | H | *c*-Pr | C(=O)Me | OMe | CH₂CN | O |
| D-6025 | Me(S) | single bond | H | *c*-Pr | C(=O)Me | OMe | CH₂CN | O |
| D-6026 | H | CH₂ | Me | *c*-Pr | C(=O)Me | OMe | CH₂CN | O |
| D-6027 | Me | CH₂ | H | *c*-Pr | C(=O)Me | OMe | CH₂CN | O |
| D-6028 | H | CH(Me) | H | *c*-Pr | C(=O)Me | OMe | CH₂CN | O |
| D-6029 | H | CH₂CH₂ | Me | *c*-Pr | C(=O)Me | OMe | CH₂CN | O |
| D-6030 | Me | CH₂CH₂ | H | *c*-Pr | C(=O)Me | OMe | CH₂CN | O |
| D-6031 | H | single bond | Me | *c*-Pr | C(=O)Me | OMe | CH₂CN | O |
| D-6032 | Me | single bond | H | *c*-Pr | C(=O)Me | OMe | CH₂CN | O |
| D-6033 | Me(R) | single bond | H | *c*-Pr | C(=O)Me | OMe | CH₂CN | O |
| D-6034 | Me(S) | single bond | H | *c*-Pr | C(=O)Me | OMe | CH₂CN | O |
| D-6035 | H | CH₂ | Me | *c*-Pr | C(=O)Me | OMe | CH₂CN | O |
| D-6036 | Me | CH₂ | H | *c*-Pr | C(=O)Me | OMe | CH₂CN | O |
| D-6037 | H | CH(Me) | H | *c*-Pr | C(=O)Me | OMe | CH₂CN | O |
| D-6038 | H | CH₂CH₂ | Me | *c*-Pr | C(=O)Me | OMe | CH₂CN | O |
| D-6039 | Me | CH₂CH₂ | H | *c*-Pr | C(=O)Me | OMe | CH₂CN | O |
| D-6040 | H | single bond | Me | *c*-Pr | C(=O)Me | OMe | CH₂CN | O |
| D-6041 | Me | single bond | H | *c*-Pr | C(=O)Me | OMe | CH₂CN | O |
| D-6042 | Me(R) | single bond | H | *c*-Pr | C(=O)Me | OMe | CH₂CN | O |
| D-6043 | Me(S) | single bond | H | *c*-Pr | C(=O)Me | OMe | CH₂CN | O |
| D-6044 | H | CH₂ | Me | *c*-Pr | C(=O)Me | OMe | CH₂CN | O |
| D-6045 | Me | CH₂ | H | *c*-Pr | C(=O)Me | OMe | CH₂CN | O |
| D-6046 | H | CH(Me) | H | *c*-Pr | C(=O)Me | OMe | CH₂CN | O |
| D-6047 | H | CH₂CH₂ | Me | *c*-Pr | C(=O)Me | OMe | CH₂CN | O |
| D-6048 | Me | CH₂CH₂ | H | *c*-Pr | C(=O)Me | OMe | CH₂CN | O |
| D-6049 | H | single bond | Me | *c*-Pr | C(=O)Me | OCF₃ | CH₂CN | O |
| D-6050 | Me | single bond | H | *c*-Pr | C(=O)Me | OCF₃ | CH₂CN | O |
| D-6051 | Me(R) | single bond | H | *c*-Pr | C(=O)Me | OCF₃ | CH₂CN | O |
| D-6052 | Me(S) | single bond | H | *c*-Pr | C(=O)Me | OCF₃ | CH₂CN | O |
| D-6053 | H | CH₂ | Me | *c*-Pr | C(=O)Me | OCF₃ | CH₂CN | O |
| D-6054 | Me | CH₂ | H | *c*-Pr | C(=O)Me | OCF₃ | CH₂CN | O |
| D-6055 | H | CH(Me) | H | *c*-Pr | C(=O)Me | OCF₃ | CH₂CN | O |
| D-6056 | H | CH₂CH₂ | Me | *c*-Pr | C(=O)Me | OCF₃ | CH₂CN | O |
| D-6057 | Me | CH₂CH₂ | H | *c*-Pr | C(=O)Me | OCF₃ | CH₂CN | O |
| D-6058 | H | single bond | Me | *c*-Pr | C(=O)Me | OCF₃ | CH₂CN | O |
| D-6059 | Me | single bond | H | *c*-Pr | C(=O)Me | OCF₃ | CH₂CN | O |
| D-6060 | Me(R) | single bond | H | *c*-Pr | C(=O)Me | OCF₃ | CH₂CN | O |
| D-6061 | Me(S) | single bond | H | *c*-Pr | C(=O)Me | OCF₃ | CH₂CN | O |
| D-6062 | H | CH₂ | Me | *c*-Pr | C(=O)Me | OCF₃ | CH₂CN | O |
| D-6063 | Me | CH₂ | H | *c*-Pr | C(=O)Me | OCF₃ | CH₂CN | O |
| D-6064 | H | CH(Me) | H | *c*-Pr | C(=O)Me | OCF₃ | CH₂CN | O |
| D-6065 | H | CH₂CH₂ | Me | *c*-Pr | C(=O)Me | OCF₃ | CH₂CN | O |
| D-6066 | Me | CH₂CH₂ | H | *c*-Pr | C(=O)Me | OCF₃ | CH₂CN | O |

**[Table 783]**

| Compound Number | Y | W | Z | R¹ | R² | R³ | R¹⁰ | A |
|---|---|---|---|---|---|---|---|---|
| D-6067 | H | single bond | Me | *c*-Pr | C(=O)Me | OCF₃ | CH₂CN | O |
| D-6068 | Me | single bond | H | *c*-Pr | C(=O)Me | OCF₃ | CH₂CN | O |
| D-6069 | Me(R) | single bond | H | *c*-Pr | C(=O)Me | OCF₃ | CH₂CN | O |
| D-6070 | Me(S) | single bond | H | *c*-Pr | C(=O)Me | OCF₃ | CH₂CN | O |
| D-6071 | H | CH₂ | Me | *c*-Pr | C(=O)Me | OCF₃ | CH₂CN | O |
| D-6072 | Me | CH₂ | H | *c*-Pr | C(=O)Me | OCF₃ | CH₂CN | O |
| D-6073 | H | CH(Me) | H | *c*-Pr | C(=O)Me | OCF₃ | CH₂CN | O |
| D-6074 | H | CH₂CH₂ | Me | *c*-Pr | C(=O)Me | OCF₃ | CH₂CN | O |
| D-6075 | Me | CH₂CH₂ | H | *c*-Pr | C(=O)Me | OCF₃ | CH₂CN | O |
| D-6076 | H | single bond | Me | *c*-Pr(1-CN) | C(=O)Me | OCF₃ | CH₂CN | O |
| D-6077 | Me | single bond | H | *c*-Pr(1-CN) | C(=O)Me | OCF₃ | CH₂CN | O |
| D-6078 | Me(R) | single bond | H | *c*-Pr(1-CN) | C(=O)Me | OCF₃ | CH₂CN | O |
| D-6079 | Me(S) | single bond | H | *c*-Pr(1-CN) | C(=O)Me | OCF₃ | CH₂CN | O |
| D-6080 | H | CH₂ | Me | *c*-Pr(1-CN) | C(=O)Me | OCF₃ | CH₂CN | O |
| D-6081 | Me | CH₂ | H | *c*-Pr(1-CN) | C(=O)Me | OCF₃ | CH₂CN | O |
| D-6082 | H | CH(Me) | H | *c*-Pr(1-CN) | C(=O)Me | OCF₃ | CH₂CN | O |
| D-6083 | H | CH₂CH₂ | Me | *c*-Pr(1-CN) | C(=O)Me | OCF₃ | CH₂CN | O |
| D-6084 | Me | CH₂CH₂ | H | *c*-Pr(1-CN) | C(=O)Me | OCF₃ | CH₂CN | O |

**[Table 784]**

| 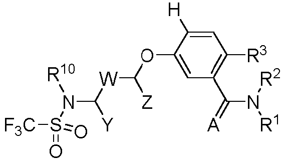 | | | | | | | | |
|---|---|---|---|---|---|---|---|---|
| Compound Number | Y | W | Z | R¹ | R² | R³ | R¹⁰ | A |
| D-6085 | H | single bond | Me | CH₂CF₃ | C(=O)OCH₂CH=CH₂ | Cl | H | O |
| D-6086 | Me | single bond | H | CH₂CF₃ | C(=O)OCH₂CH=CH₂ | Cl | H | O |
| D-6087 | Me(R) | single bond | H | CH₂CF₃ | C(=O)OCH₂CH=CH₂ | Cl | H | O |
| D-6088 | Me(S) | single bond | H | CH₂CF₃ | C(=O)OCH₂CH=CH₂ | Cl | H | O |
| D-6089 | H | CH₂ | Me | CH₂CF₃ | C(=O)OCH₂CH=CH₂ | Cl | H | O |
| D-6090 | Me | CH₂ | H | CH₂CF₃ | C(=O)OCH₂CH=CH₂ | Cl | H | O |
| D-6091 | H | CH(Me) | H | CH₂CF₃ | C(=O)OCH₂CH=CH₂ | Cl | H | O |
| D-6092 | H | CH₂CH₂ | Me | CH₂CF₃ | C(=O)OCH₂CH=CH₂ | Cl | H | O |
| D-6093 | Me | CH₂CH₂ | H | CH₂CF₃ | C(=O)OCH₂CH=CH₂ | Cl | H | O |
| D-6094 | H | single bond | Me | CH₂CF₃ | C(=O)OCH₂CH=CH₂ | Me | H | O |
| D-6095 | Me | single bond | H | CH₂CF₃ | C(=O)OCH₂CH=CH₂ | Me | H | O |
| D-6096 | Me(R) | single bond | H | CH₂CF₃ | C(=O)OCH₂CH=CH₂ | Me | H | O |
| D-6097 | Me(S) | single bond | H | CH₂CF₃ | C(=O)OCH₂CH=CH₂ | Me | H | O |
| D-6098 | H | CH₂ | Me | CH₂CF₃ | C(=O)OCH₂CH=CH₂ | Me | H | O |
| D-6099 | Me | CH₂ | H | CH₂CF₃ | C(=O)OCH₂CH=CH₂ | Me | H | O |
| D-6100 | H | CH(Me) | H | CH₂CF₃ | C(=O)OCH₂CH=CH₂ | Me | H | O |
| D-6101 | H | CH₂CH₂ | Me | CH₂CF₃ | C(=O)OCH₂CH=CH₂ | Me | H | O |
| D-6102 | Me | CH₂CH₂ | H | CH₂CF₃ | C(=O)OCH₂CH=CH₂ | Me | H | O |
| D-6103 | H | single bond | Me | CH₂CF₃ | C(=O)OCH₂CCl₃ | Cl | H | O |
| D-6104 | Me | single bond | H | CH₂CF₃ | C(=O)OCH₂CCl₃ | Cl | H | O |
| D-6105 | Me(R) | single bond | H | CH₂CF₃ | C(=O)OCH₂CCl₃ | Cl | H | O |
| D-6106 | Me(S) | single bond | H | CH₂CF₃ | C(=O)OCH₂CCl₃ | Cl | H | O |
| D-6107 | H | CH₂ | Me | CH₂CF₃ | C(=O)OCH₂CCl₃ | Cl | H | O |
| D-6108 | Me | CH₂ | H | CH₂CF₃ | C(=O)OCH₂CCl₃ | Cl | H | O |
| D-6109 | H | CH(Me) | H | CH₂CF₃ | C(=O)OCH₂CCl₃ | Cl | H | O |
| D-6110 | H | CH₂CH₂ | Me | CH₂CF₃ | C(=O)OCH₂CCl₃ | Cl | H | O |
| D-6111 | Me | CH₂CH₂ | H | CH₂CF₃ | C(=O)OCH₂CCl₃ | Cl | H | O |
| D-6112 | H | single bond | Me | CH₂CF₃ | C(=O)OCH₂CCl₃ | Me | H | O |
| D-6113 | Me | single bond | H | CH₂CF₃ | C(=O)OCH₂CCl₃ | Me | H | O |
| D-6114 | Me(R) | single bond | H | CH₂CF₃ | C(=O)OCH₂CCl₃ | Me | H | O |
| D-6115 | Me(S) | single bond | H | CH₂CF₃ | C(=O)OCH₂CCl₃ | Me | H | O |
| D-6116 | H | CH₂ | Me | CH₂CF₃ | C(=O)OCH₂CCl₃ | Me | H | O |
| D-6117 | Me | CH₂ | H | CH₂CF₃ | C(=O)OCH₂CCl₃ | Me | H | O |
| D-6118 | H | CH(Me) | H | CH₂CF₃ | C(=O)OCH₂CCl₃ | Me | H | O |
| D-6119 | H | CH₂CH₂ | Me | CH₂CF₃ | C(=O)OCH₂CCl₃ | Me | H | O |
| D-6120 | Me | CH₂CH₂ | H | CH₂CF₃ | C(=O)OCH₂CCl₃ | Me | H | O |
| D-6121 | H | single bond | Me | CH₂CF₃ | C(=O)N(Me)₂ | Cl | H | O |
| D-6122 | Me | single bond | H | CH₂CF₃ | C(=O)N(Me)₂ | Cl | H | O |

**[Table 785]**

| Compound Number | Y | W | Z | R¹ | R² | R³ | R¹⁰ | A |
|---|---|---|---|---|---|---|---|---|
| D-6123 | Me(R) | single bond | H | CH₂CF₃ | C(=O)N(Me)₂ | Cl | H | O |
| D-6124 | Me(S) | single bond | H | CH₂CF₃ | C(=O)N(Me)₂ | Cl | H | O |
| D-6125 | H | CH₂ | Me | CH₂CF₃ | C(=O)N(Me)₂ | Cl | H | O |
| D-6126 | Me | CH₂ | H | CH₂CF₃ | C(=O)N(Me)₂ | Cl | H | O |
| D-6127 | H | CH(Me) | H | CH₂CF₃ | C(=O)N(Me)₂ | Cl | H | O |
| D-6128 | H | CH₂CH₂ | Me | CH₂CF₃ | C(=O)N(Me)₂ | Cl | H | O |
| D-6129 | Me | CH₂CH₂ | H | CH₂CF₃ | C(=O)N(Me)₂ | Cl | H | O |
| D-6130 | H | single bond | Me | CH₂CF₃ | C(=O)N(Me)₂ | Me | H | O |
| D-6131 | Me | single bond | H | CH₂CF₃ | C(=O)N(Me)₂ | Me | H | O |
| D-6132 | Me(R) | single bond | H | CH₂CF₃ | C(=O)N(Me)₂ | Me | H | O |
| D-6133 | Me(S) | single bond | H | CH₂CF₃ | C(=O)N(Me)₂ | Me | H | O |
| D-6134 | H | CH₂ | Me | CH₂CF₃ | C(=O)N(Me)₂ | Me | H | O |
| D-6135 | Me | CH₂ | H | CH₂CF₃ | C(=O)N(Me)₂ | Me | H | O |
| D-6136 | H | CH(Me) | H | CH₂CF₃ | C(=O)N(Me)₂ | Me | H | O |
| D-6137 | H | CH₂CH₂ | Me | CH₂CF₃ | C(=O)N(Me)₂ | Me | H | O |
| D-6138 | Me | CH₂CH₂ | H | CH₂CF₃ | C(=O)N(Me)₂ | Me | H | O |
| D-6139 | H | single bond | Me | CH₂CF₃ | CH₂OC(=O)Me | Cl | H | O |
| D-6140 | Me | single bond | H | CH₂CF₃ | CH₂OC(=O)Me | Cl | H | O |
| D-6141 | Me(R) | single bond | H | CH₂CF₃ | CH₂OC(=O)Me | Cl | H | O |
| D-6142 | Me(S) | single bond | H | CH₂CF₃ | CH₂OC(=O)Me | Cl | H | O |
| D-6143 | H | CH₂ | Me | CH₂CF₃ | CH₂OC(=O)Me | Cl | H | O |
| D-6144 | Me | CH₂ | H | CH₂CF₃ | CH₂OC(=O)Me | Cl | H | O |
| D-6145 | H | CH(Me) | H | CH₂CF₃ | CH₂OC(=O)Me | Cl | H | O |
| D-6146 | H | CH₂CH₂ | Me | CH₂CF₃ | CH₂OC(=O)Me | Cl | H | O |
| D-6147 | Me | CH₂CH₂ | H | CH₂CF₃ | CH₂OC(=O)Me | Cl | H | O |
| D-6148 | H | single bond | Me | CH₂CF₃ | CH₂OC(=O)Me | Me | H | O |
| D-6149 | Me | single bond | H | CH₂CF₃ | CH₂OC(=O)Me | Me | H | O |
| D-6150 | Me(R) | single bond | H | CH₂CF₃ | CH₂OC(=O)Me | Me | H | O |
| D-6151 | Me(S) | single bond | H | CH₂CF₃ | CH₂OC(=O)Me | Me | H | O |
| D-6152 | H | CH₂ | Me | CH₂CF₃ | CH₂OC(=O)Me | Me | H | O |
| D-6153 | Me | CH₂ | H | CH₂CF₃ | CH₂OC(=O)Me | Me | H | O |
| D-6154 | H | CH(Me) | H | CH₂CF₃ | CH₂OC(=O)Me | Me | H | O |
| D-6155 | H | CH₂CH₂ | Me | CH₂CF₃ | CH₂OC(=O)Me | Me | H | O |
| D-6156 | Me | CH₂CH₂ | H | CH₂CF₃ | CH₂OC(=O)Me | Me | H | O |
| D-6157 | H | single bond | Me | CH₂CF₃ | CH₂OC(=O)(*i* -Pr) | Cl | H | O |
| D-6158 | Me | single bond | H | CH₂CF₃ | CH₂OC(=O)(*i* -Pr) | Cl | H | O |
| D-6159 | Me(R) | single bond | H | CH₂CF₃ | CH₂OC(=O)(*i* -Pr) | Cl | H | O |
| D-6160 | Me(S) | single bond | H | CH₂CF₃ | CH₂OC(=O)(*i* -Pr) | Cl | H | O |
| D-6161 | H | CH₂ | Me | CH₂CF₃ | CH₂OC(=O)(*i* -Pr) | Cl | H | O |
| D-6162 | Me | CH₂ | H | CH₂CF₃ | CH₂OC(=O)(*i* -Pr) | Cl | H | O |
| D-6163 | H | CH(Me) | H | CH₂CF₃ | CH₂OC(=O)(*i* -Pr) | Cl | H | O |
| D-6164 | H | CH₂CH₂ | Me | CH₂CF₃ | CH₂OC(=O)(*i* -Pr) | Cl | H | O |
| D-6165 | Me | CH₂CH₂ | H | CH₂CF₃ | CH₂OC(=O)(*i* -Pr) | Cl | H | O |
| D-6166 | H | single bond | Me | CH₂CF₃ | CH₂OC(=O)(*i* -Pr) | Me | H | O |

**[Table 786]**

| Compound Number | Y | W | Z | R¹ | R² | R³ | R¹⁰ | A |
|---|---|---|---|---|---|---|---|---|
| D-6167 | Me | single bond | H | CH₂CF₃ | CH₂OC(=O)(*i*-Pr) | Me | H | O |
| D-6168 | Me(R) | single bond | H | CH₂CF₃ | CH₂OC(=O)(*i*-Pr) | Me | H | O |
| D-6169 | Me(S) | single bond | H | CH₂CF₃ | CH₂OC(=O)(*i*-Pr) | Me | H | O |
| D-6170 | H | CH₂ | Me | CH₂CF₃ | CH₂OC(=O)(*i*-Pr) | Me | H | O |
| D-6171 | Me | CH₂ | H | CH₂CF₃ | CH₂OC(=O)(*i*-Pr) | Me | H | O |
| D-6172 | H | CH(Me) | H | CH₂CF₃ | CH₂OC(=O)(*i*-Pr) | Me | H | O |
| D-6173 | H | CH₂CH₂ | Me | CH₂CF₃ | CH₂OC(=O)(*i*-Pr) | Me | H | O |
| D-6174 | Me | CH₂CH₂ | H | CH₂CF₃ | CH₂OC(=O)(*i*-Pr) | Me | H | O |
| D-6175 | H | single bond | Me | CH₂CF₃ | CH₂OC(=O)(*t*-Bu) | Cl | H | O |
| D-6176 | Me | single bond | H | CH₂CF₃ | CH₂OC(=O)(*t*-Bu) | Cl | H | O |
| D-6177 | Me(R) | single bond | H | CH₂CF₃ | CH₂OC(=O)(*t*-Bu) | Cl | H | O |
| D-6178 | Me(S) | single bond | H | CH₂CF₃ | CH₂OC(=O)(*t*-Bu) | Cl | H | O |
| D-6179 | H | CH₂ | Me | CH₂CF₃ | CH₂OC(=O)(*t*-Bu) | Cl | H | O |
| D-6180 | Me | CH₂ | H | CH₂CF₃ | CH₂OC(=O)(*t*-Bu) | Cl | H | O |
| D-6181 | H | CH(Me) | H | CH₂CF₃ | CH₂OC(=O)(*t*-Bu) | Cl | H | O |
| D-6182 | H | CH₂CH₂ | Me | CH₂CF₃ | CH₂OC(=O)(*t*-Bu) | Cl | H | O |
| D-6183 | Me | CH₂CH₂ | H | CH₂CF₃ | CH₂OC(=O)(*t*-Bu) | Cl | H | O |
| D-6184 | H | single bond | Me | CH₂CF₃ | CH₂OC(=O)(*t*-Bu) | Me | H | O |
| D-6185 | Me | single bond | H | CH₂CF₃ | CH₂OC(=O)(*t*-Bu) | Me | H | O |
| D-6186 | Me(R) | single bond | H | CH₂CF₃ | CH₂OC(=O)(*t*-Bu) | Me | H | O |
| D-6187 | Me(S) | single bond | H | CH₂CF₃ | CH₂OC(=O)(*t*-Bu) | Me | H | O |
| D-6188 | H | CH₂ | Me | CH₂CF₃ | CH₂OC(=O)(*t*-Bu) | Me | H | O |
| D-6189 | Me | CH₂ | H | CH₂CF₃ | CH₂OC(=O)(*t*-Bu) | Me | H | O |
| D-6190 | H | CH(Me) | H | CH₂CF₃ | CH₂OC(=O)(*t*-Bu) | Me | H | O |
| D-6191 | H | CH₂CH₂ | Me | CH₂CF₃ | CH₂OC(=O)(*t*-Bu) | Me | H | O |
| D-6192 | Me | CH₂CH₂ | H | CH₂CF₃ | CH₂OC(=O)(*t*-Bu) | Me | H | O |
| D-6193 | H | single bond | Me | CH₂CF₃ | CH₂OC(=O)OEt | Cl | H | O |
| D-6194 | Me | single bond | H | CH₂CF₃ | CH₂OC(=O)OEt | Cl | H | O |
| D-6195 | Me(R) | single bond | H | CH₂CF₃ | CH₂OC(=O)OEt | Cl | H | O |
| D-6196 | Me(S) | single bond | H | CH₂CF₃ | CH₂OC(=O)OEt | Cl | H | O |
| D-6197 | H | CH₂ | Me | CH₂CF₃ | CH₂OC(=O)OEt | Cl | H | O |
| D-6198 | Me | CH₂ | H | CH₂CF₃ | CH₂OC(=O)OEt | Cl | H | O |
| D-6199 | H | CH(Me) | H | CH₂CF₃ | CH₂OC(=O)OEt | Cl | H | O |
| D-6200 | H | CH₂CH₂ | Me | CH₂CF₃ | CH₂OC(=O)OEt | Cl | H | O |
| D-6201 | Me | CH₂CH₂ | H | CH₂CF₃ | CH₂OC(=O)OEt | Cl | H | O |
| D-6202 | H | single bond | Me | CH₂CF₃ | CH₂OC(=O)OEt | Me | H | O |
| D-6203 | Me | single bond | H | CH₂CF₃ | CH₂OC(=O)OEt | Me | H | O |
| D-6204 | Me(R) | single bond | H | CH₂CF₃ | CH₂OC(=O)OEt | Me | H | O |
| D-6205 | Me(S) | single bond | H | CH₂CF₃ | CH₂OC(=O)OEt | Me | H | O |
| D-6206 | H | CH₂ | Me | CH₂CF₃ | CH₂OC(=O)OEt | Me | H | O |
| D-6207 | Me | CH₂ | H | CH₂CF₃ | CH₂OC(=O)OEt | Me | H | O |
| D-6208 | H | CH(Me) | H | CH₂CF₃ | CH₂OC(=O)OEt | Me | H | O |
| D-6209 | H | CH₂CH₂ | Me | CH₂CF₃ | CH₂OC(=O)OEt | Me | H | O |
| D-6210 | Me | CH₂CH₂ | H | CH₂CF₃ | CH₂OC(=O)OEt | Me | H | O |

**[Table 787]**

| Compound Number | Y | W | Z | R¹ | R² | R³ | R¹⁰ | A |
|---|---|---|---|---|---|---|---|---|
| D-6211 | H | single bond | Me | CH₂CF₃ | CH₂OC(=O)O(*i* -Pr) | Cl | H | O |
| D-6212 | Me | single bond | H | CH₂CF₃ | CH₂OC(=O)O(*i* -Pr) | Cl | H | O |
| D-6213 | Me(R) | single bond | H | CH₂CF₃ | CH₂OC(=O)O(*i* -Pr) | Cl | H | O |
| D-6214 | Me(S) | single bond | H | CH₂CF₃ | CH₂OC(=O)O(*i* -Pr) | Cl | H | O |
| D-6215 | H | CH₂ | Me | CH₂CF₃ | CH₂OC(=O)O(*i* -Pr) | Cl | H | O |
| D-6216 | Me | CH₂ | H | CH₂CF₃ | CH₂OC(=O)O(*i* -Pr) | Cl | H | O |
| D-6217 | H | CH(Me) | H | CH₂CF₃ | CH₂OC(=O)O(*i* -Pr) | Cl | H | O |
| D-6218 | H | CH₂CH₂ | Me | CH₂CF₃ | CH₂OC(=O)O(*i* -Pr) | Cl | H | O |
| D-6219 | Me | CH₂CH₂ | H | CH₂CF₃ | CH₂OC(=O)O(*i* -Pr) | Cl | H | O |
| D-6220 | H | single bond | Me | CH₂CF₃ | CH₂OC(=O)O(*i* -Pr) | Me | H | O |
| D-6221 | Me | single bond | H | CH₂CF₃ | CH₂OC(=O)O(*i* -Pr) | Me | H | O |
| D-6222 | Me(R) | single bond | H | CH₂CF₃ | CH₂OC(=O)O(*i* -Pr) | Me | H | O |
| D-6223 | Me(S) | single bond | H | CH₂CF₃ | CH₂OC(=O)O(*i* -Pr) | Me | H | O |
| D-6224 | H | CH₂ | Me | CH₂CF₃ | CH₂OC(=O)O(*i* -Pr) | Me | H | O |
| D-6225 | Me | CH₂ | H | CH₂CF₃ | CH₂OC(=O)O(*i* -Pr) | Me | H | O |
| D-6226 | H | CH(Me) | H | CH₂CF₃ | CH₂OC(=O)O(*i* -Pr) | Me | H | O |
| D-6227 | H | CH₂CH₂ | Me | CH₂CF₃ | CH₂OC(=O)O(*i* -Pr) | Me | H | O |
| D-6228 | Me | CH₂CH₂ | H | CH₂CF₃ | CH₂OC(=O)O(*i* -Pr) | Me | H | O |
| D-6229 | H | single bond | Me | CH₂CF₃ | CH₂N(Me)C(=O)OMe | Cl | H | O |
| D-6230 | Me | single bond | H | CH₂CF₃ | CH₂N(Me)C(=O)OMe | Cl | H | O |
| D-6231 | Me(R) | single bond | H | CH₂CF₃ | CH₂N(Me)C(=O)OMe | Cl | H | O |
| D-6232 | Me(S) | single bond | H | CH₂CF₃ | CH₂N(Me)C(=O)OMe | Cl | H | O |
| D-6233 | H | CH₂ | Me | CH₂CF₃ | CH₂N(Me)C(=O)OMe | Cl | H | O |
| D-6234 | Me | CH₂ | H | CH₂CF₃ | CH₂N(Me)C(=O)OMe | Cl | H | O |
| D-6235 | H | CH(Me) | H | CH₂CF₃ | CH₂N(Me)C(=O)OMe | Cl | H | O |
| D-6236 | H | CH₂CH₂ | Me | CH₂CF₃ | CH₂N(Me)C(=O)OMe | Cl | H | O |
| D-6237 | Me | CH₂CH₂ | H | CH₂CF₃ | CH₂N(Me)C(=O)OMe | Cl | H | O |
| D-6238 | H | single bond | Me | CH₂CF₃ | CH₂N(Me)C(=O)OMe | Me | H | O |
| D-6239 | Me | single bond | H | CH₂CF₃ | CH₂N(Me)C(=O)OMe | Me | H | O |
| D-6240 | Me(R) | single bond | H | CH₂CF₃ | CH₂N(Me)C(=O)OMe | Me | H | O |
| D-6241 | Me(S) | single bond | H | CH₂CF₃ | CH₂N(Me)C(=O)OMe | Me | H | O |
| D-6242 | H | CH₂ | Me | CH₂CF₃ | CH₂N(Me)C(=O)OMe | Me | H | O |
| D-6243 | Me | CH₂ | H | CH₂CF₃ | CH₂N(Me)C(=O)OMe | Me | H | O |
| D-6244 | H | CH(Me) | H | CH₂CF₃ | CH₂N(Me)C(=O)OMe | Me | H | O |
| D-6245 | H | CH₂CH₂ | Me | CH₂CF₃ | CH₂N(Me)C(=O)OMe | Me | H | O |
| D-6246 | Me | CH₂CH₂ | H | CH₂CF₃ | CH₂N(Me)C(=O)OMe | Me | H | O |
| D-6247 | H | single bond | Me | CH₂CF₃ | C(=O)OCH₂CH=CH₂ | Cl | Me | O |
| D-6248 | Me | single bond | H | CH₂CF₃ | C(=O)OCH₂CH=CH₂ | Cl | Me | O |
| D-6249 | Me(R) | single bond | H | CH₂CF₃ | C(=O)OCH₂CH=CH₂ | Cl | Me | O |
| D-6250 | Me(S) | single bond | H | CH₂CF₃ | C(=O)OCH₂CH=CH₂ | Cl | Me | O |
| D-6251 | H | CH₂ | Me | CH₂CF₃ | C(=O)OCH₂CH=CH₂ | Cl | Me | O |
| D-6252 | Me | CH₂ | H | CH₂CF₃ | C(=O)OCH₂CH=CH₂ | Cl | Me | O |
| D-6253 | H | CH(Me) | H | CH₂CF₃ | C(=O)OCH₂CH=CH₂ | Cl | Me | O |
| D-6254 | H | CH₂CH₂ | Me | CH₂CF₃ | C(=O)OCH₂CH=CH₂ | Cl | Me | O |

**[Table 788]**

| Compound Number | Y | W | Z | R¹ | R² | R³ | R¹⁰ | A |
|---|---|---|---|---|---|---|---|---|
| D-6255 | Me | CH₂CH₂ | H | CH₂CF₃ | C(=O)OCH₂CH=CH₂ | Cl | Me | O |
| D-6256 | H | single bond | Me | CH₂CF₃ | C(=O)OCH₂CH=CH₂ | Me | Me | O |
| D-6257 | Me | single bond | H | CH₂CF₃ | C(=O)OCH₂CH=CH₂ | Me | Me | O |
| D-6258 | Me(R) | single bond | H | CH₂CF₃ | C(=O)OCH₂CH=CH₂ | Me | Me | O |
| D-6259 | Me(S) | single bond | H | CH₂CF₃ | C(=O)OCH₂CH=CH₂ | Me | Me | O |
| D-6260 | H | CH₂ | Me | CH₂CF₃ | C(=O)OCH₂CH=CH₂ | Me | Me | O |
| D-6261 | Me | CH₂ | H | CH₂CF₃ | C(=O)OCH₂CH=CH₂ | Me | Me | O |
| D-6262 | H | CH(Me) | H | CH₂CF₃ | C(=O)OCH₂CH=CH₂ | Me | Me | O |
| D-6263 | H | CH₂CH₂ | Me | CH₂CF₃ | C(=O)OCH₂CH=CH₂ | Me | Me | O |
| D-6264 | Me | CH₂CH₂ | H | CH₂CF₃ | C(=O)OCH₂CH=CH₂ | Me | Me | O |
| D-6265 | H | single bond | Me | CH₂CF₃ | C(=O)OCH₂CCl₃ | Cl | Me | O |
| D-6266 | Me | single bond | H | CH₂CF₃ | C(=O)OCH₂CCl₃ | Cl | Me | O |
| D-6267 | Me(R) | single bond | H | CH₂CF₃ | C(=O)OCH₂CCl₃ | Cl | Me | O |
| D-6268 | Me(S) | single bond | H | CH₂CF₃ | C(=O)OCH₂CCl₃ | Cl | Me | O |
| D-6269 | H | CH₂ | Me | CH₂CF₃ | C(=O)OCH₂CCl₃ | Cl | Me | O |
| D-6270 | Me | CH₂ | H | CH₂CF₃ | C(=O)OCH₂CCl₃ | Cl | Me | O |
| D-6271 | H | CH(Me) | H | CH₂CF₃ | C(=O)OCH₂CCl₃ | Cl | Me | O |
| D-6272 | H | CH₂CH₂ | Me | CH₂CF₃ | C(=O)OCH₂CCl₃ | Cl | Me | O |
| D-6273 | Me | CH₂CH₂ | H | CH₂CF₃ | C(=O)OCH₂CCl₃ | Cl | Me | O |
| D-6274 | H | single bond | Me | CH₂CF₃ | C(=O)OCH₂CCl₃ | Me | Me | O |
| D-6275 | Me | single bond | H | CH₂CF₃ | C(=O)OCH₂CCl₃ | Me | Me | O |
| D-6276 | Me(R) | single bond | H | CH₂CF₃ | C(=O)OCH₂CCl₃ | Me | Me | O |
| D-6277 | Me(S) | single bond | H | CH₂CF₃ | C(=O)OCH₂CCl₃ | Me | Me | O |
| D-6278 | H | CH₂ | Me | CH₂CF₃ | C(=O)OCH₂CCl₃ | Me | Me | O |
| D-6279 | Me | CH₂ | H | CH₂CF₃ | C(=O)OCH₂CCl₃ | Me | Me | O |
| D-6280 | H | CH(Me) | H | CH₂CF₃ | C(=O)OCH₂CCl₃ | Me | Me | O |
| D-6281 | H | CH₂CH₂ | Me | CH₂CF₃ | C(=O)OCH₂CCl₃ | Me | Me | O |
| D-6282 | Me | CH₂CH₂ | H | CH₂CF₃ | C(=O)OCH₂CCl₃ | Me | Me | O |
| D-6283 | H | single bond | Me | CH₂CF₃ | C(=O)N(Me)₂ | Cl | Me | O |
| D-6284 | Me | single bond | H | CH₂CF₃ | C(=O)N(Me)₂ | Cl | Me | O |
| D-6285 | Me(R) | single bond | H | CH₂CF₃ | C(=O)N(Me)₂ | Cl | Me | O |
| D-6286 | Me(S) | single bond | H | CH₂CF₃ | C(=O)N(Me)₂ | Cl | Me | O |
| D-6287 | H | CH₂ | Me | CH₂CF₃ | C(=O)N(Me)₂ | Cl | Me | O |
| D-6288 | Me | CH₂ | H | CH₂CF₃ | C(=O)N(Me)₂ | Cl | Me | O |
| D-6289 | H | CH(Me) | H | CH₂CF₃ | C(=O)N(Me)₂ | Cl | Me | O |
| D-6290 | H | CH₂CH₂ | Me | CH₂CF₃ | C(=O)N(Me)₂ | Cl | Me | O |
| D-6291 | Me | CH₂CH₂ | H | CH₂CF₃ | C(=O)N(Me)₂ | Cl | Me | O |
| D-6292 | H | single bond | Me | CH₂CF₃ | C(=O)N(Me)₂ | Me | Me | O |
| D-6293 | Me | single bond | H | CH₂CF₃ | C(=O)N(Me)₂ | Me | Me | O |
| D-6294 | Me(R) | single bond | H | CH₂CF₃ | C(=O)N(Me)₂ | Me | Me | O |
| D-6295 | Me(S) | single bond | H | CH₂CF₃ | C(=O)N(Me)₂ | Me | Me | O |
| D-6296 | H | CH₂ | Me | CH₂CF₃ | C(=O)N(Me)₂ | Me | Me | O |
| D-6297 | Me | CH₂ | H | CH₂CF₃ | C(=O)N(Me)₂ | Me | Me | O |
| D-6298 | H | CH(Me) | H | CH₂CF₃ | C(=O)N(Me)₂ | Me | Me | O |

**[Table 789]**

| Compound Number | Y | W | Z | R¹ | R² | R³ | R¹⁰ | A |
|---|---|---|---|---|---|---|---|---|
| D-6299 | H | CH₂CH₂ | Me | CH₂CF₃ | C(=O)N(Me)₂ | Me | Me | O |
| D-6300 | Me | CH₂CH₂ | H | CH₂CF₃ | C(=O)N(Me)₂ | Me | Me | O |
| D-6301 | H | single bond | Me | CH₂CF₃ | CH₂OC(=O)Me | Cl | Me | O |
| D-6302 | Me | single bond | H | CH₂CF₃ | CH₂OC(=O)Me | Cl | Me | O |
| D-6303 | Me(R) | single bond | H | CH₂CF₃ | CH₂OC(=O)Me | Cl | Me | O |
| D-6304 | Me(S) | single bond | H | CH₂CF₃ | CH₂OC(=O)Me | Cl | Me | O |
| D-6305 | H | CH₂ | Me | CH₂CF₃ | CH₂OC(=O)Me | Cl | Me | O |
| D-6306 | Me | CH₂ | H | CH₂CF₃ | CH₂OC(=O)Me | Cl | Me | O |
| D-6307 | H | CH(Me) | H | CH₂CF₃ | CH₂OC(=O)Me | Cl | Me | O |
| D-6308 | H | CH₂CH₂ | Me | CH₂CF₃ | CH₂OC(=O)Me | Cl | Me | O |
| D-6309 | Me | CH₂CH₂ | H | CH₂CF₃ | CH₂OC(=O)Me | Cl | Me | O |
| D-6310 | H | single bond | Me | CH₂CF₃ | CH₂OC(=O)Me | Me | Me | O |
| D-6311 | Me | single bond | H | CH₂CF₃ | CH₂OC(=O)Me | Me | Me | O |
| D-6312 | Me(R) | single bond | H | CH₂CF₃ | CH₂OC(=O)Me | Me | Me | O |
| D-6313 | Me(S) | single bond | H | CH₂CF₃ | CH₂OC(=O)Me | Me | Me | O |
| D-6314 | H | CH₂ | Me | CH₂CF₃ | CH₂OC(=O)Me | Me | Me | O |
| D-6315 | Me | CH₂ | H | CH₂CF₃ | CH₂OC(=O)Me | Me | Me | O |
| D-6316 | H | CH(Me) | H | CH₂CF₃ | CH₂OC(=O)Me | Me | Me | O |
| D-6317 | H | CH₂CH₂ | Me | CH₂CF₃ | CH₂OC(=O)Me | Me | Me | O |
| D-6318 | Me | CH₂CH₂ | H | CH₂CF₃ | CH₂OC(=O)Me | Me | Me | O |
| D-6319 | H | single bond | Me | CH₂CF₃ | CH₂OC(=O)(*i* -Pr) | Cl | Me | O |
| D-6320 | Me | single bond | H | CH₂CF₃ | CH₂OC(=O)(*i* -Pr) | Cl | Me | O |
| D-6321 | Me(R) | single bond | H | CH₂CF₃ | CH₂OC(=O)(*i* -Pr) | Cl | Me | O |
| D-6322 | Me(S) | single bond | H | CH₂CF₃ | CH₂OC(=O)(*i* -Pr) | Cl | Me | O |
| D-6323 | H | CH₂ | Me | CH₂CF₃ | CH₂OC(=O)(*i* -Pr) | Cl | Me | O |
| D-6324 | Me | CH₂ | H | CH₂CF₃ | CH₂OC(=O)(*i* -Pr) | Cl | Me | O |
| D-6325 | H | CH(Me) | H | CH₂CF₃ | CH₂OC(=O)(*i* -Pr) | Cl | Me | O |
| D-6326 | H | CH₂CH₂ | Me | CH₂CF₃ | CH₂OC(=O)(*i* -Pr) | Cl | Me | O |
| D-6327 | Me | CH₂CH₂ | H | CH₂CF₃ | CH₂OC(=O)(*i* -Pr) | Cl | Me | O |
| D-6328 | H | single bond | Me | CH₂CF₃ | CH₂OC(=O)(*i* -Pr) | Me | Me | O |
| D-6329 | Me | single bond | H | CH₂CF₃ | CH₂OC(=O)(*i* -Pr) | Me | Me | O |
| D-6330 | Me(R) | single bond | H | CH₂CF₃ | CH₂OC(=O)(*i* -Pr) | Me | Me | O |
| D-6331 | Me(S) | single bond | H | CH₂CF₃ | CH₂OC(=O)(*i* -Pr) | Me | Me | O |
| D-6332 | H | CH₂ | Me | CH₂CF₃ | CH₂OC(=O)(*i* -Pr) | Me | Me | O |
| D-6333 | Me | CH₂ | H | CH₂CF₃ | CH₂OC(=O)(*i* -Pr) | Me | Me | O |
| D-6334 | H | CH(Me) | H | CH₂CF₃ | CH₂OC(=O)(*i* -Pr) | Me | Me | O |
| D-6335 | H | CH₂CH₂ | Me | CH₂CF₃ | CH₂OC(=O)(*i* -Pr) | Me | Me | O |
| D-6336 | Me | CH₂CH₂ | H | CH₂CF₃ | CH₂OC(=O)(*i* -Pr) | Me | Me | O |
| D-6337 | H | single bond | Me | CH₂CF₃ | CH₂OC(=O)(*t* -Bu) | Cl | Me | O |
| D-6338 | Me | single bond | H | CH₂CF₃ | CH₂OC(=O)(*t* -Bu) | Cl | Me | O |
| D-6339 | Me(R) | single bond | H | CH₂CF₃ | CH₂OC(=O)(*t* -Bu) | Cl | Me | O |
| D-6340 | Me(S) | single bond | H | CH₂CF₃ | CH₂OC(=O)(*t* -Bu) | Cl | Me | O |
| D-6341 | H | CH₂ | Me | CH₂CF₃ | CH₂OC(=O)(*t* -Bu) | Cl | Me | O |
| D-6342 | Me | CH₂ | H | CH₂CF₃ | CH₂OC(=O)(*t* -Bu) | Cl | Me | O |

**[Table 790]**

| Compound Number | Y | W | Z | R¹ | R² | R³ | R¹⁰ | A |
|---|---|---|---|---|---|---|---|---|
| D-6343 | H | CH(Me) | H | CH₂CF₃ | CH₂OC(=O)(*t* -Bu) | Cl | Me | O |
| D-6344 | H | CH₂CH₂ | Me | CH₂CF₃ | CH₂OC(=O)(*t* -Bu) | Cl | Me | O |
| D-6345 | Me | CH₂CH₂ | H | CH₂CF₃ | CH₂OC(=O)(*t* -Bu) | Cl | Me | O |
| D-6346 | H | single bond | Me | CH₂CF₃ | CH₂OC(=O)(*t* -Bu) | Me | Me | O |
| D-6347 | Me | single bond | H | CH₂CF₃ | CH₂OC(=O)(*t* -Bu) | Me | Me | O |
| D-6348 | Me(R) | single bond | H | CH₂CF₃ | CH₂OC(=O)(*t* -Bu) | Me | Me | O |
| D-6349 | Me(S) | single bond | H | CH₂CF₃ | CH₂OC(=O)(*t* -Bu) | Me | Me | O |
| D-6350 | H | CH₂ | Me | CH₂CF₃ | CH₂OC(=O)(*t* -Bu) | Me | Me | O |
| D-6351 | Me | CH₂ | H | CH₂CF₃ | CH₂OC(=O)(*t* -Bu) | Me | Me | O |
| D-6352 | H | CH(Me) | H | CH₂CF₃ | CH₂OC(=O)(*t* -Bu) | Me | Me | O |
| D-6353 | H | CH₂CH₂ | Me | CH₂CF₃ | CH₂OC(=O)(*t* -Bu) | Me | Me | O |
| D-6354 | Me | CH₂CH₂ | H | CH₂CF₃ | CH₂OC(=O)(*t* -Bu) | Me | Me | O |
| D-6355 | H | single bond | Me | CH₂CF₃ | CH₂OC(=O)OEt | Cl | Me | O |
| D-6356 | Me | single bond | H | CH₂CF₃ | CH₂OC(=O)OEt | Cl | Me | O |
| D-6357 | Me(R) | single bond | H | CH₂CF₃ | CH₂OC(=O)OEt | Cl | Me | O |
| D-6358 | Me(S) | single bond | H | CH₂CF₃ | CH₂OC(=O)OEt | Cl | Me | O |
| D-6359 | H | CH₂ | Me | CH₂CF₃ | CH₂OC(=O)OEt | Cl | Me | O |
| D-6360 | Me | CH₂ | H | CH₂CF₃ | CH₂OC(=O)OEt | Cl | Me | O |
| D-6361 | H | CH(Me) | H | CH₂CF₃ | CH₂OC(=O)OEt | Cl | Me | O |
| D-6362 | H | CH₂CH₂ | Me | CH₂CF₃ | CH₂OC(=O)OEt | Cl | Me | O |
| D-6363 | Me | CH₂CH₂ | H | CH₂CF₃ | CH₂OC(=O)OEt | Cl | Me | O |
| D-6364 | H | single bond | Me | CH₂CF₃ | CH₂OC(=O)OEt | Me | Me | O |
| D-6365 | Me | single bond | H | CH₂CF₃ | CH₂OC(=O)OEt | Me | Me | O |
| D-6366 | Me(R) | single bond | H | CH₂CF₃ | CH₂OC(=O)OEt | Me | Me | O |
| D-6367 | Me(S) | single bond | H | CH₂CF₃ | CH₂OC(=O)OEt | Me | Me | O |
| D-6368 | H | CH₂ | Me | CH₂CF₃ | CH₂OC(=O)OEt | Me | Me | O |
| D-6369 | Me | CH₂ | H | CH₂CF₃ | CH₂OC(=O)OEt | Me | Me | O |
| D-6370 | H | CH(Me) | H | CH₂CF₃ | CH₂OC(=O)OEt | Me | Me | O |
| D-6371 | H | CH₂CH₂ | Me | CH₂CF₃ | CH₂OC(=O)OEt | Me | Me | O |
| D-6372 | Me | CH₂CH₂ | H | CH₂CF₃ | CH₂OC(=O)OEt | Me | Me | O |
| D-6373 | H | single bond | Me | CH₂CF₃ | CH₂OC(=O)O(*i* -Pr) | Cl | Me | O |
| D-6374 | Me | single bond | H | CH₂CF₃ | CH₂OC(=O)O(*i* -Pr) | Cl | Me | O |
| D-6375 | Me(R) | single bond | H | CH₂CF₃ | CH₂OC(=O)O(*i* -Pr) | Cl | Me | O |
| D-6376 | Me(S) | single bond | H | CH₂CF₃ | CH₂OC(=O)O(*i* -Pr) | Cl | Me | O |
| D-6377 | H | CH₂ | Me | CH₂CF₃ | CH₂OC(=O)O(*i* -Pr) | Cl | Me | O |
| D-6378 | Me | CH₂ | H | CH₂CF₃ | CH₂OC(=O)O(*i* -Pr) | Cl | Me | O |
| D-6379 | H | CH(Me) | H | CH₂CF₃ | CH₂OC(=O)O(*i* -Pr) | Cl | Me | O |
| D-6380 | H | CH₂CH₂ | Me | CH₂CF₃ | CH₂OC(=O)O(*i* -Pr) | Cl | Me | O |
| D-6381 | Me | CH₂CH₂ | H | CH₂CF₃ | CH₂OC(=O)O(*i* -Pr) | Cl | Me | O |
| D-6382 | H | single bond | Me | CH₂CF₃ | CH₂OC(=O)O(*i* -Pr) | Me | Me | O |
| D-6383 | Me | single bond | H | CH₂CF₃ | CH₂OC(=O)O(*i* -Pr) | Me | Me | O |
| D-6384 | Me(R) | single bond | H | CH₂CF₃ | CH₂OC(=O)O(*i* -Pr) | Me | Me | O |
| D-6385 | Me(S) | single bond | H | CH₂CF₃ | CH₂OC(=O)O(*i* -Pr) | Me | Me | O |
| D-6386 | H | CH₂ | Me | CH₂CF₃ | CH₂OC(=O)O(*i* -Pr) | Me | Me | O |

**[Table 791]**

| Compound Number | Y | W | Z | R¹ | R² | R³ | R¹⁰ | A |
|---|---|---|---|---|---|---|---|---|
| D-6387 | Me | CH₂ | H | CH₂CF₃ | CH₂OC(=O)O(*i*-Pr) | Me | Me | O |
| D-6388 | H | CH(Me) | H | CH₂CF₃ | CH₂OC(=O)O(*i*-Pr) | Me | Me | O |
| D-6389 | H | CH₂CH₂ | Me | CH₂CF₃ | CH₂OC(=O)O(*i*-Pr) | Me | Me | O |
| D-6390 | Me | CH₂CH₂ | H | CH₂CF₃ | CH₂OC(=O)O(*i*-Pr) | Me | Me | O |
| D-6391 | H | single bond | Me | CH₂CF₃ | CH₂N(Me)C(=O)OMe | Cl | Me | O |
| D-6392 | Me | single bond | H | CH₂CF₃ | CH₂N(Me)C(=O)OMe | Cl | Me | O |
| D-6393 | Me(R) | single bond | H | CH₂CF₃ | CH₂N(Me)C(=O)OMe | Cl | Me | O |
| D-6394 | Me(S) | single bond | H | CH₂CF₃ | CH₂N(Me)C(=O)OMe | Cl | Me | O |
| D-6395 | H | CH₂ | Me | CH₂CF₃ | CH₂N(Me)C(=O)OMe | Cl | Me | O |
| D-6396 | Me | CH₂ | H | CH₂CF₃ | CH₂N(Me)C(=O)OMe | Cl | Me | O |
| D-6397 | H | CH(Me) | H | CH₂CF₃ | CH₂N(Me)C(=O)OMe | Cl | Me | O |
| D-6398 | H | CH₂CH₂ | Me | CH₂CF₃ | CH₂N(Me)C(=O)OMe | Cl | Me | O |
| D-6399 | Me | CH₂CH₂ | H | CH₂CF₃ | CH₂N(Me)C(=O)OMe | Cl | Me | O |
| D-6400 | H | single bond | Me | CH₂CF₃ | CH₂N(Me)C(=O)OMe | Me | Me | O |
| D-6401 | Me | single bond | H | CH₂CF₃ | CH₂N(Me)C(=O)OMe | Me | Me | O |
| D-6402 | Me(R) | single bond | H | CH₂CF₃ | CH₂N(Me)C(=O)OMe | Me | Me | O |
| D-6403 | Me(S) | single bond | H | CH₂CF₃ | CH₂N(Me)C(=O)OMe | Me | Me | O |
| D-6404 | H | CH₂ | Me | CH₂CF₃ | CH₂N(Me)C(=O)OMe | Me | Me | O |
| D-6405 | Me | CH₂ | H | CH₂CF₃ | CH₂N(Me)C(=O)OMe | Me | Me | O |
| D-6406 | H | CH(Me) | H | CH₂CF₃ | CH₂N(Me)C(=O)OMe | Me | Me | O |
| D-6407 | H | CH₂CH₂ | Me | CH₂CF₃ | CH₂N(Me)C(=O)OMe | Me | Me | O |
| D-6408 | Me | CH₂CH₂ | H | CH₂CF₃ | CH₂N(Me)C(=O)OMe | Me | Me | O |
| D-6409 | H | single bond | Me | CH₂CF₃ | C(=O)OCH₂CH=CH₂ | Cl | CH₂OMe | O |
| D-6410 | Me | single bond | H | CH₂CF₃ | C(=O)OCH₂CH=CH₂ | Cl | CH₂OMe | O |
| D-6411 | Me(R) | single bond | H | CH₂CF₃ | C(=O)OCH₂CH=CH₂ | Cl | CH₂OMe | O |
| D-6412 | Me(S) | single bond | H | CH₂CF₃ | C(=O)OCH₂CH=CH₂ | Cl | CH₂OMe | O |
| D-6413 | H | CH₂ | Me | CH₂CF₃ | C(=O)OCH₂CH=CH₂ | Cl | CH₂OMe | O |
| D-6414 | Me | CH₂ | H | CH₂CF₃ | C(=O)OCH₂CH=CH₂ | Cl | CH₂OMe | O |
| D-6415 | H | CH(Me) | H | CH₂CF₃ | C(=O)OCH₂CH=CH₂ | Cl | CH₂OMe | O |
| D-6416 | H | CH₂CH₂ | Me | CH₂CF₃ | C(=O)OCH₂CH=CH₂ | Cl | CH₂OMe | O |
| D-6417 | Me | CH₂CH₂ | H | CH₂CF₃ | C(=O)OCH₂CH=CH₂ | Cl | CH₂OMe | O |
| D-6418 | H | single bond | Me | CH₂CF₃ | C(=O)OCH₂CH=CH₂ | Me | CH₂OMe | O |
| D-6419 | Me | single bond | H | CH₂CF₃ | C(=O)OCH₂CH=CH₂ | Me | CH₂OMe | O |
| D-6420 | Me(R) | single bond | H | CH₂CF₃ | C(=O)OCH₂CH=CH₂ | Me | CH₂OMe | O |
| D-6421 | Me(S) | single bond | H | CH₂CF₃ | C(=O)OCH₂CH=CH₂ | Me | CH₂OMe | O |
| D-6422 | H | CH₂ | Me | CH₂CF₃ | C(=O)OCH₂CH=CH₂ | Me | CH₂OMe | O |
| D-6423 | Me | CH₂ | H | CH₂CF₃ | C(=O)OCH₂CH=CH₂ | Me | CH₂OMe | O |
| D-6424 | H | CH(Me) | H | CH₂CF₃ | C(=O)OCH₂CH=CH₂ | Me | CH₂OMe | O |
| D-6425 | H | CH₂CH₂ | Me | CH₂CF₃ | C(=O)OCH₂CH=CH₂ | Me | CH₂OMe | O |
| D-6426 | Me | CH₂CH₂ | H | CH₂CF₃ | C(=O)OCH₂CH=CH₂ | Me | CH₂OMe | O |
| D-6427 | H | single bond | Me | CH₂CF₃ | C(=O)OCH₂CCl₃ | Cl | CH₂OMe | O |
| D-6428 | Me | single bond | H | CH₂CF₃ | C(=O)OCH₂CCl₃ | Cl | CH₂OMe | O |
| D-6429 | Me(R) | single bond | H | CH₂CF₃ | C(=O)OCH₂CCl₃ | Cl | CH₂OMe | O |
| D-6430 | Me(S) | single bond | H | CH₂CF₃ | C(=O)OCH₂CCl₃ | Cl | CH₂OMe | O |

**[Table 792]**

| Compound Number | Y | W | Z | R¹ | R² | R³ | R¹⁰ | A |
|---|---|---|---|---|---|---|---|---|
| D-6431 | H | CH₂ | Me | CH₂CF₃ | C(=O)OCH₂CCl₃ | Cl | CH₂OMe | O |
| D-6432 | Me | CH₂ | H | CH₂CF₃ | C(=O)OCH₂CCl₃ | Cl | CH₂OMe | O |
| D-6433 | H | CH(Me) | H | CH₂CF₃ | C(=O)OCH₂CCl₃ | Cl | CH₂OMe | O |
| D-6434 | H | CH₂CH₂ | Me | CH₂CF₃ | C(=O)OCH₂CCl₃ | Cl | CH₂OMe | O |
| D-6435 | Me | CH₂CH₂ | H | CH₂CF₃ | C(=O)OCH₂CCl₃ | Cl | CH₂OMe | O |
| D-6436 | H | single bond | Me | CH₂CF₃ | C(=O)OCH₂CCl₃ | Me | CH₂OMe | O |
| D-6437 | Me | single bond | H | CH₂CF₃ | C(=O)OCH₂CCl₃ | Me | CH₂OMe | O |
| D-6438 | Me(R) | single bond | H | CH₂CF₃ | C(=O)OCH₂CCl₃ | Me | CH₂OMe | O |
| D-6439 | Me(S) | single bond | H | CH₂CF₃ | C(=O)OCH₂CCl₃ | Me | CH₂OMe | O |
| D-6440 | H | CH₂ | Me | CH₂CF₃ | C(=O)OCH₂CCl₃ | Me | CH₂OMe | O |
| D-6441 | Me | CH₂ | H | CH₂CF₃ | C(=O)OCH₂CCl₃ | Me | CH₂OMe | O |
| D-6442 | H | CH(Me) | H | CH₂CF₃ | C(=O)OCH₂CCl₃ | Me | CH₂OMe | O |
| D-6443 | H | CH₂CH₂ | Me | CH₂CF₃ | C(=O)OCH₂CCl₃ | Me | CH₂OMe | O |
| D-6444 | Me | CH₂CH₂ | H | CH₂CF₃ | C(=O)OCH₂CCl₃ | Me | CH₂OMe | O |
| D-6445 | H | single bond | Me | CH₂CF₃ | C(=O)N(Me)₂ | Cl | CH₂OMe | O |
| D-6446 | Me | single bond | H | CH₂CF₃ | C(=O)N(Me)₂ | Cl | CH₂OMe | O |
| D-6447 | Me(R) | single bond | H | CH₂CF₃ | C(=O)N(Me)₂ | Cl | CH₂OMe | O |
| D-6448 | Me(S) | single bond | H | CH₂CF₃ | C(=O)N(Me)₂ | Cl | CH₂OMe | O |
| D-6449 | H | CH₂ | Me | CH₂CF₃ | C(=O)N(Me)₂ | Cl | CH₂OMe | O |
| D-6450 | Me | CH₂ | H | CH₂CF₃ | C(=O)N(Me)₂ | Cl | CH₂OMe | O |
| D-6451 | H | CH(Me) | H | CH₂CF₃ | C(=O)N(Me)₂ | Cl | CH₂OMe | O |
| D-6452 | H | CH₂CH₂ | Me | CH₂CF₃ | C(=O)N(Me)₂ | Cl | CH₂OMe | O |
| D-6453 | Me | CH₂CH₂ | H | CH₂CF₃ | C(=O)N(Me)₂ | Cl | CH₂OMe | O |
| D-6454 | H | single bond | Me | CH₂CF₃ | C(=O)N(Me)₂ | Me | CH₂OMe | O |
| D-6455 | Me | single bond | H | CH₂CF₃ | C(=O)N(Me)₂ | Me | CH₂OMe | O |
| D-6456 | Me(R) | single bond | H | CH₂CF₃ | C(=O)N(Me)₂ | Me | CH₂OMe | O |
| D-6457 | Me(S) | single bond | H | CH₂CF₃ | C(=O)N(Me)₂ | Me | CH₂OMe | O |
| D-6458 | H | CH₂ | Me | CH₂CF₃ | C(=O)N(Me)₂ | Me | CH₂OMe | O |
| D-6459 | Me | CH₂ | H | CH₂CF₃ | C(=O)N(Me)₂ | Me | CH₂OMe | O |
| D-6460 | H | CH(Me) | H | CH₂CF₃ | C(=O)N(Me)₂ | Me | CH₂OMe | O |
| D-6461 | H | CH₂CH₂ | Me | CH₂CF₃ | C(=O)N(Me)₂ | Me | CH₂OMe | O |
| D-6462 | Me | CH₂CH₂ | H | CH₂CF₃ | C(=O)N(Me)₂ | Me | CH₂OMe | O |
| D-6463 | H | single bond | Me | CH₂CF₃ | CH₂OC(=O)Me | Cl | CH₂OMe | O |
| D-6464 | Me | single bond | H | CH₂CF₃ | CH₂OC(=O)Me | Cl | CH₂OMe | O |
| D-6465 | Me(R) | single bond | H | CH₂CF₃ | CH₂OC(=O)Me | Cl | CH₂OMe | O |
| D-6466 | Me(S) | single bond | H | CH₂CF₃ | CH₂OC(=O)Me | Cl | CH₂OMe | O |
| D-6467 | H | CH₂ | Me | CH₂CF₃ | CH₂OC(=O)Me | Cl | CH₂OMe | O |
| D-6468 | Me | CH₂ | H | CH₂CF₃ | CH₂OC(=O)Me | Cl | CH₂OMe | O |
| D-6469 | H | CH(Me) | H | CH₂CF₃ | CH₂OC(=O)Me | Cl | CH₂OMe | O |
| D-6470 | H | CH₂CH₂ | Me | CH₂CF₃ | CH₂OC(=O)Me | Cl | CH₂OMe | O |
| D-6471 | Me | CH₂CH₂ | H | CH₂CF₃ | CH₂OC(=O)Me | Cl | CH₂OMe | O |
| D-6472 | H | single bond | Me | CH₂CF₃ | CH₂OC(=O)Me | Me | CH₂OMe | O |
| D-6473 | Me | single bond | H | CH₂CF₃ | CH₂OC(=O)Me | Me | CH₂OMe | O |
| D-6474 | Me(R) | single bond | H | CH₂CF₃ | CH₂OC(=O)Me | Me | CH₂OMe | O |

**[Table 793]**

| Compound Number | Y | W | Z | R¹ | R² | R³ | R¹⁰ | A |
|---|---|---|---|---|---|---|---|---|
| D-6475 | Me(S) | single bond | H | CH₂CF₃ | CH₂OC(=O)Me | Me | CH₂OMe | O |
| D-6476 | H | CH₂ | Me | CH₂CF₃ | CH₂OC(=O)Me | Me | CH₂OMe | O |
| D-6477 | Me | CH₂ | H | CH₂CF₃ | CH₂OC(=O)Me | Me | CH₂OMe | O |
| D-6478 | H | CH(Me) | H | CH₂CF₃ | CH₂OC(=O)Me | Me | CH₂OMe | O |
| D-6479 | H | CH₂CH₂ | Me | CH₂CF₃ | CH₂OC(=O)Me | Me | CH₂OMe | O |
| D-6480 | Me | CH₂CH₂ | H | CH₂CF₃ | CH₂OC(=O)Me | Me | CH₂OMe | O |
| D-6481 | H | single bond | Me | CH₂CF₃ | CH₂OC(=O)(*i*-Pr) | Cl | CH₂OMe | O |
| D-6482 | Me | single bond | H | CH₂CF₃ | CH₂OC(=O)(*i*-Pr) | Cl | CH₂OMe | O |
| D-6483 | Me(R) | single bond | H | CH₂CF₃ | CH₂OC(=O)(*i*-Pr) | Cl | CH₂OMe | O |
| D-6484 | Me(S) | single bond | H | CH₂CF₃ | CH₂OC(=O)(*i*-Pr) | Cl | CH₂OMe | O |
| D-6485 | H | CH₂ | Me | CH₂CF₃ | CH₂OC(=O)(*i*-Pr) | Cl | CH₂OMe | O |
| D-6486 | Me | CH₂ | H | CH₂CF₃ | CH₂OC(=O)(*i*-Pr) | Cl | CH₂OMe | O |
| D-6487 | H | CH(Me) | H | CH₂CF₃ | CH₂OC(=O)(*i*-Pr) | Cl | CH₂OMe | O |
| D-6488 | H | CH₂CH₂ | Me | CH₂CF₃ | CH₂OC(=O)(*i*-Pr) | Cl | CH₂OMe | O |
| D-6489 | Me | CH₂CH₂ | H | CH₂CF₃ | CH₂OC(=O)(*i*-Pr) | Cl | CH₂OMe | O |
| D-6490 | H | single bond | Me | CH₂CF₃ | CH₂OC(=O)(*i*-Pr) | Me | CH₂OMe | O |
| D-6491 | Me | single bond | H | CH₂CF₃ | CH₂OC(=O)(*i*-Pr) | Me | CH₂OMe | O |
| D-6492 | Me(R) | single bond | H | CH₂CF₃ | CH₂OC(=O)(*i*-Pr) | Me | CH₂OMe | O |
| D-6493 | Me(S) | single bond | H | CH₂CF₃ | CH₂OC(=O)(*i*-Pr) | Me | CH₂OMe | O |
| D-6494 | H | CH₂ | Me | CH₂CF₃ | CH₂OC(=O)(*i*-Pr) | Me | CH₂OMe | O |
| D-6495 | Me | CH₂ | H | CH₂CF₃ | CH₂OC(=O)(*i*-Pr) | Me | CH₂OMe | O |
| D-6496 | H | CH(Me) | H | CH₂CF₃ | CH₂OC(=O)(*i*-Pr) | Me | CH₂OMe | O |
| D-6497 | H | CH₂CH₂ | Me | CH₂CF₃ | CH₂OC(=O)(*i*-Pr) | Me | CH₂OMe | O |
| D-6498 | Me | CH₂CH₂ | H | CH₂CF₃ | CH₂OC(=O)(*i*-Pr) | Me | CH₂OMe | O |
| D-6499 | H | single bond | Me | CH₂CF₃ | CH₂OC(=O)(*t*-Bu) | Cl | CH₂OMe | O |
| D-6500 | Me | single bond | H | CH₂CF₃ | CH₂OC(=O)(*t*-Bu) | Cl | CH₂OMe | O |
| D-6501 | Me(R) | single bond | H | CH₂CF₃ | CH₂OC(=O)(*t*-Bu) | Cl | CH₂OMe | O |
| D-6502 | Me(S) | single bond | H | CH₂CF₃ | CH₂OC(=O)(*t*-Bu) | Cl | CH₂OMe | O |
| D-6503 | H | CH₂ | Me | CH₂CF₃ | CH₂OC(=O)(*t*-Bu) | Cl | CH₂OMe | O |
| D-6504 | Me | CH₂ | H | CH₂CF₃ | CH₂OC(=O)(*t*-Bu) | Cl | CH₂OMe | O |
| D-6505 | H | CH(Me) | H | CH₂CF₃ | CH₂OC(=O)(*t*-Bu) | Cl | CH₂OMe | O |
| D-6506 | H | CH₂CH₂ | Me | CH₂CF₃ | CH₂OC(=O)(*t*-Bu) | Cl | CH₂OMe | O |
| D-6507 | Me | CH₂CH₂ | H | CH₂CF₃ | CH₂OC(=O)(*t*-Bu) | Cl | CH₂OMe | O |
| D-6508 | H | single bond | Me | CH₂CF₃ | CH₂OC(=O)(*t*-Bu) | Me | CH₂OMe | O |
| D-6509 | Me | single bond | H | CH₂CF₃ | CH₂OC(=O)(*t*-Bu) | Me | CH₂OMe | O |
| D-6510 | Me(R) | single bond | H | CH₂CF₃ | CH₂OC(=O)(*t*-Bu) | Me | CH₂OMe | O |
| D-6511 | Me(S) | single bond | H | CH₂CF₃ | CH₂OC(=O)(*t*-Bu) | Me | CH₂OMe | O |
| D-6512 | H | CH₂ | Me | CH₂CF₃ | CH₂OC(=O)(*t*-Bu) | Me | CH₂OMe | O |
| D-6513 | Me | CH₂ | H | CH₂CF₃ | CH₂OC(=O)(*t*-Bu) | Me | CH₂OMe | O |
| D-6514 | H | CH(Me) | H | CH₂CF₃ | CH₂OC(=O)(*t*-Bu) | Me | CH₂OMe | O |
| D-6515 | H | CH₂CH₂ | Me | CH₂CF₃ | CH₂OC(=O)(*t*-Bu) | Me | CH₂OMe | O |
| D-6516 | Me | CH₂CH₂ | H | CH₂CF₃ | CH₂OC(=O)(*t*-Bu) | Me | CH₂OMe | O |
| D-6517 | H | single bond | Me | CH₂CF₃ | CH₂OC(=O)OEt | Cl | CH₂OMe | O |
| D-6518 | Me | single bond | H | CH₂CF₃ | CH₂OC(=O)OEt | Cl | CH₂OMe | O |

**[Table 794]**

| Compound Number | Y | W | Z | R¹ | R² | R³ | R¹⁰ | A |
|---|---|---|---|---|---|---|---|---|
| D-6519 | Me(R) | single bond | H | CH₂CF₃ | CH₂OC(=O)OEt | Cl | CH₂OMe | O |
| D-6520 | Me(S) | single bond | H | CH₂CF₃ | CH₂OC(=O)OEt | Cl | CH₂OMe | O |
| D-6521 | H | CH₂ | Me | CH₂CF₃ | CH₂OC(=O)OEt | Cl | CH₂OMe | O |
| D-6522 | Me | CH₂ | H | CH₂CF₃ | CH₂OC(=O)OEt | Cl | CH₂OMe | O |
| D-6523 | H | CH(Me) | H | CH₂CF₃ | CH₂OC(=O)OEt | Cl | CH₂OMe | O |
| D-6524 | H | CH₂CH₂ | Me | CH₂CF₃ | CH₂OC(=O)OEt | Cl | CH₂OMe | O |
| D-6525 | Me | CH₂CH₂ | H | CH₂CF₃ | CH₂OC(=O)OEt | Cl | CH₂OMe | O |
| D-6526 | H | single bond | Me | CH₂CF₃ | CH₂OC(=O)OEt | Me | CH₂OMe | O |
| D-6527 | Me | single bond | H | CH₂CF₃ | CH₂OC(=O)OEt | Me | CH₂OMe | O |
| D-6528 | Me(R) | single bond | H | CH₂CF₃ | CH₂OC(=O)OEt | Me | CH₂OMe | O |
| D-6529 | Me(S) | single bond | H | CH₂CF₃ | CH₂OC(=O)OEt | Me | CH₂OMe | O |
| D-6530 | H | CH₂ | Me | CH₂CF₃ | CH₂OC(=O)OEt | Me | CH₂OMe | O |
| D-6531 | Me | CH₂ | H | CH₂CF₃ | CH₂OC(=O)OEt | Me | CH₂OMe | O |
| D-6532 | H | CH(Me) | H | CH₂CF₃ | CH₂OC(=O)OEt | Me | CH₂OMe | O |
| D-6533 | H | CH₂CH₂ | Me | CH₂CF₃ | CH₂OC(=O)OEt | Me | CH₂OMe | O |
| D-6534 | Me | CH₂CH₂ | H | CH₂CF₃ | CH₂OC(=O)OEt | Me | CH₂OMe | O |
| D-6535 | H | single bond | Me | CH₂CF₃ | CH₂OC(=O)O(*i*-Pr) | Cl | CH₂OMe | O |
| D-6536 | Me | single bond | H | CH₂CF₃ | CH₂OC(=O)O(*i*-Pr) | Cl | CH₂OMe | O |
| D-6537 | Me(R) | single bond | H | CH₂CF₃ | CH₂OC(=O)O(*i*-Pr) | Cl | CH₂OMe | O |
| D-6538 | Me(S) | single bond | H | CH₂CF₃ | CH₂OC(=O)O(*i*-Pr) | Cl | CH₂OMe | O |
| D-6539 | H | CH₂ | Me | CH₂CF₃ | CH₂OC(=O)O(*i*-Pr) | Cl | CH₂OMe | O |
| D-6540 | Me | CH₂ | H | CH₂CF₃ | CH₂OC(=O)O(*i*-Pr) | Cl | CH₂OMe | O |
| D-6541 | H | CH(Me) | H | CH₂CF₃ | CH₂OC(=O)O(*i*-Pr) | Cl | CH₂OMe | O |
| D-6542 | H | CH₂CH₂ | Me | CH₂CF₃ | CH₂OC(=O)O(*i*-Pr) | Cl | CH₂OMe | O |
| D-6543 | Me | CH₂CH₂ | H | CH₂CF₃ | CH₂OC(=O)O(*i*-Pr) | Cl | CH₂OMe | O |
| D-6544 | H | single bond | Me | CH₂CF₃ | CH₂OC(=O)O(*i*-Pr) | Me | CH₂OMe | O |
| D-6545 | Me | single bond | H | CH₂CF₃ | CH₂OC(=O)O(*i*-Pr) | Me | CH₂OMe | O |
| D-6546 | Me(R) | single bond | H | CH₂CF₃ | CH₂OC(=O)O(*i*-Pr) | Me | CH₂OMe | O |
| D-6547 | Me(S) | single bond | H | CH₂CF₃ | CH₂OC(=O)O(*i*-Pr) | Me | CH₂OMe | O |
| D-6548 | H | CH₂ | Me | CH₂CF₃ | CH₂OC(=O)O(*i*-Pr) | Me | CH₂OMe | O |
| D-6549 | Me | CH₂ | H | CH₂CF₃ | CH₂OC(=O)O(*i*-Pr) | Me | CH₂OMe | O |
| D-6550 | H | CH(Me) | H | CH₂CF₃ | CH₂OC(=O)O(*i*-Pr) | Me | CH₂OMe | O |
| D-6551 | H | CH₂CH₂ | Me | CH₂CF₃ | CH₂OC(=O)O(*i*-Pr) | Me | CH₂OMe | O |
| D-6552 | Me | CH₂CH₂ | H | CH₂CF₃ | CH₂OC(=O)O(*i*-Pr) | Me | CH₂OMe | O |
| D-6553 | H | single bond | Me | CH₂CF₃ | CH₂N(Me)C(=O)OMe | Cl | CH₂OMe | O |
| D-6554 | Me | single bond | H | CH₂CF₃ | CH₂N(Me)C(=O)OMe | Cl | CH₂OMe | O |
| D-6555 | Me(R) | single bond | H | CH₂CF₃ | CH₂N(Me)C(=O)OMe | Cl | CH₂OMe | O |
| D-6556 | Me(S) | single bond | H | CH₂CF₃ | CH₂N(Me)C(=O)OMe | Cl | CH₂OMe | O |
| D-6557 | H | CH₂ | Me | CH₂CF₃ | CH₂N(Me)C(=O)OMe | Cl | CH₂OMe | O |
| D-6558 | Me | CH₂ | H | CH₂CF₃ | CH₂N(Me)C(=O)OMe | Cl | CH₂OMe | O |
| D-6559 | H | CH(Me) | H | CH₂CF₃ | CH₂N(Me)C(=O)OMe | Cl | CH₂OMe | O |
| D-6560 | H | CH₂CH₂ | Me | CH₂CF₃ | CH₂N(Me)C(=O)OMe | Cl | CH₂OMe | O |
| D-6561 | Me | CH₂CH₂ | H | CH₂CF₃ | CH₂N(Me)C(=O)OMe | Cl | CH₂OMe | O |
| D-6562 | H | single bond | Me | CH₂CF₃ | CH₂N(Me)C(=O)OMe | Me | CH₂OMe | O |

**[Table 795]**

| Compound Number | Y | W | Z | R¹ | R² | R³ | R¹⁰ | A |
|---|---|---|---|---|---|---|---|---|
| D-6563 | Me | single bond | H | CH₂CF₃ | CH₂N(Me)C(=O)OMe | Me | CH₂OMe | O |
| D-6564 | Me(R) | single bond | H | CH₂CF₃ | CH₂N(Me)C(=O)OMe | Me | CH₂OMe | O |
| D-6565 | Me(S) | single bond | H | CH₂CF₃ | CH₂N(Me)C(=O)OMe | Me | CH₂OMe | O |
| D-6566 | H | CH₂ | Me | CH₂CF₃ | CH₂N(Me)C(=O)OMe | Me | CH₂OMe | O |
| D-6567 | Me | CH₂ | H | CH₂CF₃ | CH₂N(Me)C(=O)OMe | Me | CH₂OMe | O |
| D-6568 | H | CH(Me) | H | CH₂CF₃ | CH₂N(Me)C(=O)OMe | Me | CH₂OMe | O |
| D-6569 | H | CH₂CH₂ | Me | CH₂CF₃ | CH₂N(Me)C(=O)OMe | Me | CH₂OMe | O |
| D-6570 | Me | CH₂CH₂ | H | CH₂CF₃ | CH₂N(Me)C(=O)OMe | Me | CH₂OMe | O |
| D-6571 | H | single bond | Me | CH₂CF₃ | C(=O)OCH₂CH=CH₂ | Cl | CH₂CN | O |
| D-6572 | Me | single bond | H | CH₂CF₃ | C(=O)OCH₂CH=CH₂ | Cl | CH₂CN | O |
| D-6573 | Me(R) | single bond | H | CH₂CF₃ | C(=O)OCH₂CH=CH₂ | Cl | CH₂CN | O |
| D-6574 | Me(S) | single bond | H | CH₂CF₃ | C(=O)OCH₂CH=CH₂ | Cl | CH₂CN | O |
| D-6575 | H | CH₂ | Me | CH₂CF₃ | C(=O)OCH₂CH=CH₂ | Cl | CH₂CN | O |
| D-6576 | Me | CH₂ | H | CH₂CF₃ | C(=O)OCH₂CH=CH₂ | Cl | CH₂CN | O |
| D-6577 | H | CH(Me) | H | CH₂CF₃ | C(=O)OCH₂CH=CH₂ | Cl | CH₂CN | O |
| D-6578 | H | CH₂CH₂ | Me | CH₂CF₃ | C(=O)OCH₂CH=CH₂ | Cl | CH₂CN | O |
| D-6579 | Me | CH₂CH₂ | H | CH₂CF₃ | C(=O)OCH₂CH=CH₂ | Cl | CH₂CN | O |
| D-6580 | H | single bond | Me | CH₂CF₃ | C(=O)OCH₂CH=CH₂ | Me | CH₂CN | O |
| D-6581 | Me | single bond | H | CH₂CF₃ | C(=O)OCH₂CH=CH₂ | Me | CH₂CN | O |
| D-6582 | Me(R) | single bond | H | CH₂CF₃ | C(=O)OCH₂CH=CH₂ | Me | CH₂CN | O |
| D-6583 | Me(S) | single bond | H | CH₂CF₃ | C(=O)OCH₂CH=CH₂ | Me | CH₂CN | O |
| D-6584 | H | CH₂ | Me | CH₂CF₃ | C(=O)OCH₂CH=CH₂ | Me | CH₂CN | O |
| D-6585 | Me | CH₂ | H | CH₂CF₃ | C(=O)OCH₂CH=CH₂ | Me | CH₂CN | O |
| D-6586 | H | CH(Me) | H | CH₂CF₃ | C(=O)OCH₂CH=CH₂ | Me | CH₂CN | O |
| D-6587 | H | CH₂CH₂ | Me | CH₂CF₃ | C(=O)OCH₂CH=CH₂ | Me | CH₂CN | O |
| D-6588 | Me | CH₂CH₂ | H | CH₂CF₃ | C(=O)OCH₂CH=CH₂ | Me | CH₂CN | O |
| D-6589 | H | single bond | Me | CH₂CF₃ | C(=O)OCH₂CCl₃ | Cl | CH₂CN | O |
| D-6590 | Me | single bond | H | CH₂CF₃ | C(=O)OCH₂CCl₃ | Cl | CH₂CN | O |
| D-6591 | Me(R) | single bond | H | CH₂CF₃ | C(=O)OCH₂CCl₃ | Cl | CH₂CN | O |
| D-6592 | Me(S) | single bond | H | CH₂CF₃ | C(=O)OCH₂CCl₃ | Cl | CH₂CN | O |
| D-6593 | H | CH₂ | Me | CH₂CF₃ | C(=O)OCH₂CCl₃ | Cl | CH₂CN | O |
| D-6594 | Me | CH₂ | H | CH₂CF₃ | C(=O)OCH₂CCl₃ | Cl | CH₂CN | O |
| D-6595 | H | CH(Me) | H | CH₂CF₃ | C(=O)OCH₂CCl₃ | Cl | CH₂CN | O |
| D-6596 | H | CH₂CH₂ | Me | CH₂CF₃ | C(=O)OCH₂CCl₃ | Cl | CH₂CN | O |
| D-6597 | Me | CH₂CH₂ | H | CH₂CF₃ | C(=O)OCH₂CCl₃ | Cl | CH₂CN | O |
| D-6598 | H | single bond | Me | CH₂CF₃ | C(=O)OCH₂CCl₃ | Me | CH₂CN | O |
| D-6599 | Me | single bond | H | CH₂CF₃ | C(=O)OCH₂CCl₃ | Me | CH₂CN | O |
| D-6600 | Me(R) | single bond | H | CH₂CF₃ | C(=O)OCH₂CCl₃ | Me | CH₂CN | O |
| D-6601 | Me(S) | single bond | H | CH₂CF₃ | C(=O)OCH₂CCl₃ | Me | CH₂CN | O |
| D-6602 | H | CH₂ | Me | CH₂CF₃ | C(=O)OCH₂CCl₃ | Me | CH₂CN | O |
| D-6603 | Me | CH₂ | H | CH₂CF₃ | C(=O)OCH₂CCl₃ | Me | CH₂CN | O |
| D-6604 | H | CH(Me) | H | CH₂CF₃ | C(=O)OCH₂CCl₃ | Me | CH₂CN | O |
| D-6605 | H | CH₂CH₂ | Me | CH₂CF₃ | C(=O)OCH₂CCl₃ | Me | CH₂CN | O |
| D-6606 | Me | CH₂CH₂ | H | CH₂CF₃ | C(=O)OCH₂CCl₃ | Me | CH₂CN | O |

**[Table 796]**

| Compound Number | Y | W | Z | R¹ | R² | R³ | R¹⁰ | A |
|---|---|---|---|---|---|---|---|---|
| D-6607 | H | single bond | Me | CH₂CF₃ | C(=O)N(Me)₂ | Cl | CH₂CN | O |
| D-6608 | Me | single bond | H | CH₂CF₃ | C(=O)N(Me)₂ | Cl | CH₂CN | O |
| D-6609 | Me(R) | single bond | H | CH₂CF₃ | C(=O)N(Me)₂ | Cl | CH₂CN | O |
| D-6610 | Me(S) | single bond | H | CH₂CF₃ | C(=O)N(Me)₂ | Cl | CH₂CN | O |
| D-6611 | H | CH₂ | Me | CH₂CF₃ | C(=O)N(Me)₂ | Cl | CH₂CN | O |
| D-6612 | Me | CH₂ | H | CH₂CF₃ | C(=O)N(Me)₂ | Cl | CH₂CN | O |
| D-6613 | H | CH(Me) | H | CH₂CF₃ | C(=O)N(Me)₂ | Cl | CH₂CN | O |
| D-6614 | H | CH₂CH₂ | Me | CH₂CF₃ | C(=O)N(Me)₂ | Cl | CH₂CN | O |
| D-6615 | Me | CH₂CH₂ | H | CH₂CF₃ | C(=O)N(Me)₂ | Cl | CH₂CN | O |
| D-6616 | H | single bond | Me | CH₂CF₃ | C(=O)N(Me)₂ | Me | CH₂CN | O |
| D-6617 | Me | single bond | H | CH₂CF₃ | C(=O)N(Me)₂ | Me | CH₂CN | O |
| D-6618 | Me(R) | single bond | H | CH₂CF₃ | C(=O)N(Me)₂ | Me | CH₂CN | O |
| D-6619 | Me(S) | single bond | H | CH₂CF₃ | C(=O)N(Me)₂ | Me | CH₂CN | O |
| D-6620 | H | CH₂ | Me | CH₂CF₃ | C(=O)N(Me)₂ | Me | CH₂CN | O |
| D-6621 | Me | CH₂ | H | CH₂CF₃ | C(=O)N(Me)₂ | Me | CH₂CN | O |
| D-6622 | H | CH(Me) | H | CH₂CF₃ | C(=O)N(Me)₂ | Me | CH₂CN | O |
| D-6623 | H | CH₂CH₂ | Me | CH₂CF₃ | C(=O)N(Me)₂ | Me | CH₂CN | O |
| D-6624 | Me | CH₂CH₂ | H | CH₂CF₃ | C(=O)N(Me)₂ | Me | CH₂CN | O |
| D-6625 | H | single bond | Me | CH₂CF₃ | CH₂OC(=O)Me | Cl | CH₂CN | O |
| D-6626 | Me | single bond | H | CH₂CF₃ | CH₂OC(=O)Me | Cl | CH₂CN | O |
| D-6627 | Me(R) | single bond | H | CH₂CF₃ | CH₂OC(=O)Me | Cl | CH₂CN | O |
| D-6628 | Me(S) | single bond | H | CH₂CF₃ | CH₂OC(=O)Me | Cl | CH₂CN | O |
| D-6629 | H | CH₂ | Me | CH₂CF₃ | CH₂OC(=O)Me | Cl | CH₂CN | O |
| D-6630 | Me | CH₂ | H | CH₂CF₃ | CH₂OC(=O)Me | Cl | CH₂CN | O |
| D-6631 | H | CH(Me) | H | CH₂CF₃ | CH₂OC(=O)Me | Cl | CH₂CN | O |
| D-6632 | H | CH₂CH₂ | Me | CH₂CF₃ | CH₂OC(=O)Me | Cl | CH₂CN | O |
| D-6633 | Me | CH₂CH₂ | H | CH₂CF₃ | CH₂OC(=O)Me | Cl | CH₂CN | O |
| D-6634 | H | single bond | Me | CH₂CF₃ | CH₂OC(=O)Me | Me | CH₂CN | O |
| D-6635 | Me | single bond | H | CH₂CF₃ | CH₂OC(=O)Me | Me | CH₂CN | O |
| D-6636 | Me(R) | single bond | H | CH₂CF₃ | CH₂OC(=O)Me | Me | CH₂CN | O |
| D-6637 | Me(S) | single bond | H | CH₂CF₃ | CH₂OC(=O)Me | Me | CH₂CN | O |
| D-6638 | H | CH₂ | Me | CH₂CF₃ | CH₂OC(=O)Me | Me | CH₂CN | O |
| D-6639 | Me | CH₂ | H | CH₂CF₃ | CH₂OC(=O)Me | Me | CH₂CN | O |
| D-6640 | H | CH(Me) | H | CH₂CF₃ | CH₂OC(=O)Me | Me | CH₂CN | O |
| D-6641 | H | CH₂CH₂ | Me | CH₂CF₃ | CH₂OC(=O)Me | Me | CH₂CN | O |
| D-6642 | Me | CH₂CH₂ | H | CH₂CF₃ | CH₂OC(=O)Me | Me | CH₂CN | O |
| D-6643 | H | single bond | Me | CH₂CF₃ | CH₂OC(=O)(*i*-Pr) | Cl | CH₂CN | O |
| D-6644 | Me | single bond | H | CH₂CF₃ | CH₂OC(=O)(*i*-Pr) | Cl | CH₂CN | O |
| D-6645 | Me(R) | single bond | H | CH₂CF₃ | CH₂OC(=O)(*i*-Pr) | Cl | CH₂CN | O |
| D-6646 | Me(S) | single bond | H | CH₂CF₃ | CH₂OC(=O)(*i*-Pr) | Cl | CH₂CN | O |
| D-6647 | H | CH₂ | Me | CH₂CF₃ | CH₂OC(=O)(*i*-Pr) | Cl | CH₂CN | O |
| D-6648 | Me | CH₂ | H | CH₂CF₃ | CH₂OC(=O)(*i*-Pr) | Cl | CH₂CN | O |
| D-6649 | H | CH(Me) | H | CH₂CF₃ | CH₂OC(=O)(*i*-Pr) | Cl | CH₂CN | O |
| D-6650 | H | CH₂CH₂ | Me | CH₂CF₃ | CH₂OC(=O)(*i*-Pr) | Cl | CH₂CN | O |

**[Table 797]**

| Compound Number | Y | W | Z | R¹ | R² | R³ | R¹⁰ | A |
|---|---|---|---|---|---|---|---|---|
| D-6651 | Me | CH₂CH₂ | H | CH₂CF₃ | CH₂OC(=O)(*i*-Pr) | Cl | CH₂CN | O |
| D-6652 | H | single bond | Me | CH₂CF₃ | CH₂OC(=O)(*i*-Pr) | Me | CH₂CN | O |
| D-6653 | Me | single bond | H | CH₂CF₃ | CH₂OC(=O)(*i*-Pr) | Me | CH₂CN | O |
| D-6654 | Me(R) | single bond | H | CH₂CF₃ | CH₂OC(=O)(*i*-Pr) | Me | CH₂CN | O |
| D-6655 | Me(S) | single bond | H | CH₂CF₃ | CH₂OC(=O)(*i*-Pr) | Me | CH₂CN | O |
| D-6656 | H | CH₂ | Me | CH₂CF₃ | CH₂OC(=O)(*i*-Pr) | Me | CH₂CN | O |
| D-6657 | Me | CH₂ | H | CH₂CF₃ | CH₂OC(=O)(*i*-Pr) | Me | CH₂CN | O |
| D-6658 | H | CH(Me) | H | CH₂CF₃ | CH₂OC(=O)(*i*-Pr) | Me | CH₂CN | O |
| D-6659 | H | CH₂CH₂ | Me | CH₂CF₃ | CH₂OC(=O)(*i*-Pr) | Me | CH₂CN | O |
| D-6660 | Me | CH₂CH₂ | H | CH₂CF₃ | CH₂OC(=O)(*i*-Pr) | Me | CH₂CN | O |
| D-6661 | H | single bond | Me | CH₂CF₃ | CH₂OC(=O)(*t*-Bu) | Cl | CH₂CN | O |
| D-6662 | Me | single bond | H | CH₂CF₃ | CH₂OC(=O)(*t*-Bu) | Cl | CH₂CN | O |
| D-6663 | Me(R) | single bond | H | CH₂CF₃ | CH₂OC(=O)(*t*-Bu) | Cl | CH₂CN | O |
| D-6664 | Me(S) | single bond | H | CH₂CF₃ | CH₂OC(=O)(*t*-Bu) | Cl | CH₂CN | O |
| D-6665 | H | CH₂ | Me | CH₂CF₃ | CH₂OC(=O)(*t*-Bu) | Cl | CH₂CN | O |
| D-6666 | Me | CH₂ | H | CH₂CF₃ | CH₂OC(=O)(*t*-Bu) | Cl | CH₂CN | O |
| D-6667 | H | CH(Me) | H | CH₂CF₃ | CH₂OC(=O)(*t*-Bu) | Cl | CH₂CN | O |
| D-6668 | H | CH₂CH₂ | Me | CH₂CF₃ | CH₂OC(=O)(*t*-Bu) | Cl | CH₂CN | O |
| D-6669 | Me | CH₂CH₂ | H | CH₂CF₃ | CH₂OC(=O)(*t*-Bu) | Cl | CH₂CN | O |
| D-6670 | H | single bond | Me | CH₂CF₃ | CH₂OC(=O)(*t*-Bu) | Me | CH₂CN | O |
| D-6671 | Me | single bond | H | CH₂CF₃ | CH₂OC(=O)(*t*-Bu) | Me | CH₂CN | O |
| D-6672 | Me(R) | single bond | H | CH₂CF₃ | CH₂OC(=O)(*t*-Bu) | Me | CH₂CN | O |
| D-6673 | Me(S) | single bond | H | CH₂CF₃ | CH₂OC(=O)(*t*-Bu) | Me | CH₂CN | O |
| D-6674 | H | CH₂ | Me | CH₂CF₃ | CH₂OC(=O)(*t*-Bu) | Me | CH₂CN | O |
| D-6675 | Me | CH₂ | H | CH₂CF₃ | CH₂OC(=O)(*t*-Bu) | Me | CH₂CN | O |
| D-6676 | H | CH(Me) | H | CH₂CF₃ | CH₂OC(=O)(*t*-Bu) | Me | CH₂CN | O |
| D-6677 | H | CH₂CH₂ | Me | CH₂CF₃ | CH₂OC(=O)(*t*-Bu) | Me | CH₂CN | O |
| D-6678 | Me | CH₂CH₂ | H | CH₂CF₃ | CH₂OC(=O)(*t*-Bu) | Me | CH₂CN | O |
| D-6679 | H | single bond | Me | CH₂CF₃ | CH₂OC(=O)OEt | Cl | CH₂CN | O |
| D-6680 | Me | single bond | H | CH₂CF₃ | CH₂OC(=O)OEt | Cl | CH₂CN | O |
| D-6681 | Me(R) | single bond | H | CH₂CF₃ | CH₂OC(=O)OEt | Cl | CH₂CN | O |
| D-6682 | Me(S) | single bond | H | CH₂CF₃ | CH₂OC(=O)OEt | Cl | CH₂CN | O |
| D-6683 | H | CH₂ | Me | CH₂CF₃ | CH₂OC(=O)OEt | Cl | CH₂CN | O |
| D-6684 | Me | CH₂ | H | CH₂CF₃ | CH₂OC(=O)OEt | Cl | CH₂CN | O |
| D-6685 | H | CH(Me) | H | CH₂CF₃ | CH₂OC(=O)OEt | Cl | CH₂CN | O |
| D-6686 | H | CH₂CH₂ | Me | CH₂CF₃ | CH₂OC(=O)OEt | Cl | CH₂CN | O |
| D-6687 | Me | CH₂CH₂ | H | CH₂CF₃ | CH₂OC(=O)OEt | Cl | CH₂CN | O |
| D-6688 | H | single bond | Me | CH₂CF₃ | CH₂OC(=O)OEt | Me | CH₂CN | O |
| D-6689 | Me | single bond | H | CH₂CF₃ | CH₂OC(=O)OEt | Me | CH₂CN | O |
| D-6690 | Me(R) | single bond | H | CH₂CF₃ | CH₂OC(=O)OEt | Me | CH₂CN | O |
| D-6691 | Me(S) | single bond | H | CH₂CF₃ | CH₂OC(=O)OEt | Me | CH₂CN | O |
| D-6692 | H | CH₂ | Me | CH₂CF₃ | CH₂OC(=O)OEt | Me | CH₂CN | O |
| D-6693 | Me | CH₂ | H | CH₂CF₃ | CH₂OC(=O)OEt | Me | CH₂CN | O |
| D-6694 | H | CH(Me) | H | CH₂CF₃ | CH₂OC(=O)OEt | Me | CH₂CN | O |

**[Table 798]**

| Compound Number | Y | W | Z | R¹ | R² | R³ | R¹⁰ | A |
|---|---|---|---|---|---|---|---|---|
| D-6695 | H | CH₂CH₂ | Me | CH₂CF₃ | CH₂OC(=O)OEt | Me | CH₂CN | O |
| D-6696 | Me | CH₂CH₂ | H | CH₂CF₃ | CH₂OC(=O)OEt | Me | CH₂CN | O |
| D-6697 | H | single bond | Me | CH₂CF₃ | CH₂OC(=O)O(*i*-Pr) | Cl | CH₂CN | O |
| D-6698 | Me | single bond | H | CH₂CF₃ | CH₂OC(=O)O(*i*-Pr) | Cl | CH₂CN | O |
| D-6699 | Me(R) | single bond | H | CH₂CF₃ | CH₂OC(=O)O(*i*-Pr) | Cl | CH₂CN | O |
| D-6700 | Me(S) | single bond | H | CH₂CF₃ | CH₂OC(=O)O(*i*-Pr) | Cl | CH₂CN | O |
| D-6701 | H | CH₂ | Me | CH₂CF₃ | CH₂OC(=O)O(*i*-Pr) | Cl | CH₂CN | O |
| D-6702 | Me | CH₂ | H | CH₂CF₃ | CH₂OC(=O)O(*i*-Pr) | Cl | CH₂CN | O |
| D-6703 | H | CH(Me) | H | CH₂CF₃ | CH₂OC(=O)O(*i*-Pr) | Cl | CH₂CN | O |
| D-6704 | H | CH₂CH₂ | Me | CH₂CF₃ | CH₂OC(=O)O(*i*-Pr) | Cl | CH₂CN | O |
| D-6705 | Me | CH₂CH₂ | H | CH₂CF₃ | CH₂OC(=O)O(*i*-Pr) | Cl | CH₂CN | O |
| D-6706 | H | single bond | Me | CH₂CF₃ | CH₂OC(=O)O(*i*-Pr) | Me | CH₂CN | O |
| D-6707 | Me | single bond | H | CH₂CF₃ | CH₂OC(=O)O(*i*-Pr) | Me | CH₂CN | O |
| D-6708 | Me(R) | single bond | H | CH₂CF₃ | CH₂OC(=O)O(*i*-Pr) | Me | CH₂CN | O |
| D-6709 | Me(S) | single bond | H | CH₂CF₃ | CH₂OC(=O)O(*i*-Pr) | Me | CH₂CN | O |
| D-6710 | H | CH₂ | Me | CH₂CF₃ | CH₂OC(=O)O(*i*-Pr) | Me | CH₂CN | O |
| D-6711 | Me | CH₂ | H | CH₂CF₃ | CH₂OC(=O)O(*i*-Pr) | Me | CH₂CN | O |
| D-6712 | H | CH(Me) | H | CH₂CF₃ | CH₂OC(=O)O(*i*-Pr) | Me | CH₂CN | O |
| D-6713 | H | CH₂CH₂ | Me | CH₂CF₃ | CH₂OC(=O)O(*i*-Pr) | Me | CH₂CN | O |
| D-6714 | Me | CH₂CH₂ | H | CH₂CF₃ | CH₂OC(=O)O(*i*-Pr) | Me | CH₂CN | O |
| D-6715 | H | single bond | Me | CH₂CF₃ | CH₂N(Me)C(=O)OMe | Cl | CH₂CN | O |
| D-6716 | Me | single bond | H | CH₂CF₃ | CH₂N(Me)C(=O)OMe | Cl | CH₂CN | O |
| D-6717 | Me(R) | single bond | H | CH₂CF₃ | CH₂N(Me)C(=O)OMe | Cl | CH₂CN | O |
| D-6718 | Me(S) | single bond | H | CH₂CF₃ | CH₂N(Me)C(=O)OMe | Cl | CH₂CN | O |
| D-6719 | H | CH₂ | Me | CH₂CF₃ | CH₂N(Me)C(=O)OMe | Cl | CH₂CN | O |
| D-6720 | Me | CH₂ | H | CH₂CF₃ | CH₂N(Me)C(=O)OMe | Cl | CH₂CN | O |
| D-6721 | H | CH(Me) | H | CH₂CF₃ | CH₂N(Me)C(=O)OMe | Cl | CH₂CN | O |
| D-6722 | H | CH₂CH₂ | Me | CH₂CF₃ | CH₂N(Me)C(=O)OMe | Cl | CH₂CN | O |
| D-6723 | Me | CH₂CH₂ | H | CH₂CF₃ | CH₂N(Me)C(=O)OMe | Cl | CH₂CN | O |
| D-6724 | H | single bond | Me | CH₂CF₃ | CH₂N(Me)C(=O)OMe | Me | CH₂CN | O |
| D-6725 | Me | single bond | H | CH₂CF₃ | CH₂N(Me)C(=O)OMe | Me | CH₂CN | O |
| D-6726 | Me(R) | single bond | H | CH₂CF₃ | CH₂N(Me)C(=O)OMe | Me | CH₂CN | O |
| D-6727 | Me(S) | single bond | H | CH₂CF₃ | CH₂N(Me)C(=O)OMe | Me | CH₂CN | O |
| D-6728 | H | CH₂ | Me | CH₂CF₃ | CH₂N(Me)C(=O)OMe | Me | CH₂CN | O |
| D-6729 | Me | CH₂ | H | CH₂CF₃ | CH₂N(Me)C(=O)OMe | Me | CH₂CN | O |
| D-6730 | H | CH(Me) | H | CH₂CF₃ | CH₂N(Me)C(=O)OMe | Me | CH₂CN | O |
| D-6731 | H | CH₂CH₂ | Me | CH₂CF₃ | CH₂N(Me)C(=O)OMe | Me | CH₂CN | O |
| D-6732 | Me | CH₂CH₂ | H | CH₂CF₃ | CH₂N(Me)C(=O)OMe | Me | CH₂CN | O |
| D-6733 | H | single bond | Me | CH₂CF₃ | C(=O)OCH₂CH=CH₂ | Cl | C(=O)Me | O |
| D-6734 | Me | single bond | H | CH₂CF₃ | C(=O)OCH₂CH=CH₂ | Cl | C(=O)Me | O |
| D-6735 | Me(R) | single bond | H | CH₂CF₃ | C(=O)OCH₂CH=CH₂ | Cl | C(=O)Me | O |
| D-6736 | Me(S) | single bond | H | CH₂CF₃ | C(=O)OCH₂CH=CH₂ | Cl | C(=O)Me | O |
| D-6737 | H | CH₂ | Me | CH₂CF₃ | C(=O)OCH₂CH=CH₂ | Cl | C(=O)Me | O |
| D-6738 | Me | CH₂ | H | CH₂CF₃ | C(=O)OCH₂CH=CH₂ | Cl | C(=O)Me | O |

**[Table 799]**

| Compound Number | Y | W | Z | R¹ | R² | R³ | R¹⁰ | A |
|---|---|---|---|---|---|---|---|---|
| D-6739 | H | CH(Me) | H | CH₂CF₃ | C(=O)OCH₂CH=CH₂ | Cl | C(=O)Me | O |
| D-6740 | H | CH₂CH₂ | Me | CH₂CF₃ | C(=O)OCH₂CH=CH₂ | Cl | C(=O)Me | O |
| D-6741 | Me | CH₂CH₂ | H | CH₂CF₃ | C(=O)OCH₂CH=CH₂ | Cl | C(=O)Me | O |
| D-6742 | H | single bond | Me | CH₂CF₃ | C(=O)OCH₂CH=CH₂ | Me | C(=O)Me | O |
| D-6743 | Me | single bond | H | CH₂CF₃ | C(=O)OCH₂CH=CH₂ | Me | C(=O)Me | O |
| D-6744 | Me(R) | single bond | H | CH₂CF₃ | C(=O)OCH₂CH=CH₂ | Me | C(=O)Me | O |
| D-6745 | Me(S) | single bond | H | CH₂CF₃ | C(=O)OCH₂CH=CH₂ | Me | C(=O)Me | O |
| D-6746 | H | CH₂ | Me | CH₂CF₃ | C(=O)OCH₂CH=CH₂ | Me | C(=O)Me | O |
| D-6747 | Me | CH₂ | H | CH₂CF₃ | C(=O)OCH₂CH=CH₂ | Me | C(=O)Me | O |
| D-6748 | H | CH(Me) | H | CH₂CF₃ | C(=O)OCH₂CH=CH₂ | Me | C(=O)Me | O |
| D-6749 | H | CH₂CH₂ | Me | CH₂CF₃ | C(=O)OCH₂CH=CH₂ | Me | C(=O)Me | O |
| D-6750 | Me | CH₂CH₂ | H | CH₂CF₃ | C(=O)OCH₂CH=CH₂ | Me | C(=O)Me | O |
| D-6751 | H | single bond | Me | CH₂CF₃ | C(=O)OCH₂CCl₃ | Cl | C(=O)Me | O |
| D-6752 | Me | single bond | H | CH₂CF₃ | C(=O)OCH₂CCl₃ | Cl | C(=O)Me | O |
| D-6753 | Me(R) | single bond | H | CH₂CF₃ | C(=O)OCH₂CCl₃ | Cl | C(=O)Me | O |
| D-6754 | Me(S) | single bond | H | CH₂CF₃ | C(=O)OCH₂CCl₃ | Cl | C(=O)Me | O |
| D-6755 | H | CH₂ | Me | CH₂CF₃ | C(=O)OCH₂CCl₃ | Cl | C(=O)Me | O |
| D-6756 | Me | CH₂ | H | CH₂CF₃ | C(=O)OCH₂CCl₃ | Cl | C(=O)Me | O |
| D-6757 | H | CH(Me) | H | CH₂CF₃ | C(=O)OCH₂CCl₃ | Cl | C(=O)Me | O |
| D-6758 | H | CH₂CH₂ | Me | CH₂CF₃ | C(=O)OCH₂CCl₃ | Cl | C(=O)Me | O |
| D-6759 | Me | CH₂CH₂ | H | CH₂CF₃ | C(=O)OCH₂CCl₃ | Cl | C(=O)Me | O |
| D-6760 | H | single bond | Me | CH₂CF₃ | C(=O)OCH₂CCl₃ | Me | C(=O)Me | O |
| D-6761 | Me | single bond | H | CH₂CF₃ | C(=O)OCH₂CCl₃ | Me | C(=O)Me | O |
| D-6762 | Me(R) | single bond | H | CH₂CF₃ | C(=O)OCH₂CCl₃ | Me | C(=O)Me | O |
| D-6763 | Me(S) | single bond | H | CH₂CF₃ | C(=O)OCH₂CCl₃ | Me | C(=O)Me | O |
| D-6764 | H | CH₂ | Me | CH₂CF₃ | C(=O)OCH₂CCl₃ | Me | C(=O)Me | O |
| D-6765 | Me | CH₂ | H | CH₂CF₃ | C(=O)OCH₂CCl₃ | Me | C(=O)Me | O |
| D-6766 | H | CH(Me) | H | CH₂CF₃ | C(=O)OCH₂CCl₃ | Me | C(=O)Me | O |
| D-6767 | H | CH₂CH₂ | Me | CH₂CF₃ | C(=O)OCH₂CCl₃ | Me | C(=O)Me | O |
| D-6768 | Me | CH₂CH₂ | H | CH₂CF₃ | C(=O)OCH₂CCl₃ | Me | C(=O)Me | O |
| D-6769 | H | single bond | Me | CH₂CF₃ | C(=O)N(Me)₂ | Cl | C(=O)Me | O |
| D-6770 | Me | single bond | H | CH₂CF₃ | C(=O)N(Me)₂ | Cl | C(=O)Me | O |
| D-6771 | Me(R) | single bond | H | CH₂CF₃ | C(=O)N(Me)₂ | Cl | C(=O)Me | O |
| D-6772 | Me(S) | single bond | H | CH₂CF₃ | C(=O)N(Me)₂ | Cl | C(=O)Me | O |
| D-6773 | H | CH₂ | Me | CH₂CF₃ | C(=O)N(Me)₂ | Cl | C(=O)Me | O |
| D-6774 | Me | CH₂ | H | CH₂CF₃ | C(=O)N(Me)₂ | Cl | C(=O)Me | O |
| D-6775 | H | CH(Me) | H | CH₂CF₃ | C(=O)N(Me)₂ | Cl | C(=O)Me | O |
| D-6776 | H | CH₂CH₂ | Me | CH₂CF₃ | C(=O)N(Me)₂ | Cl | C(=O)Me | O |
| D-6777 | Me | CH₂CH₂ | H | CH₂CF₃ | C(=O)N(Me)₂ | Cl | C(=O)Me | O |
| D-6778 | H | single bond | Me | CH₂CF₃ | C(=O)N(Me)₂ | Me | C(=O)Me | O |
| D-6779 | Me | single bond | H | CH₂CF₃ | C(=O)N(Me)₂ | Me | C(=O)Me | O |
| D-6780 | Me(R) | single bond | H | CH₂CF₃ | C(=O)N(Me)₂ | Me | C(=O)Me | O |
| D-6781 | Me(S) | single bond | H | CH₂CF₃ | C(=O)N(Me)₂ | Me | C(=O)Me | O |
| D-6782 | H | CH₂ | Me | CH₂CF₃ | C(=O)N(Me)₂ | Me | C(=O)Me | O |

**[Table 800]**

| Compound Number | Y | W | Z | R¹ | R² | R³ | R¹⁰ | A |
|---|---|---|---|---|---|---|---|---|
| D-6783 | Me | CH₂ | H | CH₂CF₃ | C(=O)N(Me)₂ | Me | C(=O)Me | O |
| D-6784 | H | CH(Me) | H | CH₂CF₃ | C(=O)N(Me)₂ | Me | C(=O)Me | O |
| D-6785 | H | CH₂CH₂ | Me | CH₂CF₃ | C(=O)N(Me)₂ | Me | C(=O)Me | O |
| D-6786 | Me | CH₂CH₂ | H | CH₂CF₃ | C(=O)N(Me)₂ | Me | C(=O)Me | O |
| D-6787 | H | single bond | Me | CH₂CF₃ | CH₂OC(=O)Me | Cl | C(=O)Me | O |
| D-6788 | Me | single bond | H | CH₂CF₃ | CH₂OC(=O)Me | Cl | C(=O)Me | O |
| D-6789 | Me(R) | single bond | H | CH₂CF₃ | CH₂OC(=O)Me | Cl | C(=O)Me | O |
| D-6790 | Me(S) | single bond | H | CH₂CF₃ | CH₂OC(=O)Me | Cl | C(=O)Me | O |
| D-6791 | H | CH₂ | Me | CH₂CF₃ | CH₂OC(=O)Me | Cl | C(=O)Me | O |
| D-6792 | Me | CH₂ | H | CH₂CF₃ | CH₂OC(=O)Me | Cl | C(=O)Me | O |
| D-6793 | H | CH(Me) | H | CH₂CF₃ | CH₂OC(=O)Me | Cl | C(=O)Me | O |
| D-6794 | H | CH₂CH₂ | Me | CH₂CF₃ | CH₂OC(=O)Me | Cl | C(=O)Me | O |
| D-6795 | Me | CH₂CH₂ | H | CH₂CF₃ | CH₂OC(=O)Me | Cl | C(=O)Me | O |
| D-6796 | H | single bond | Me | CH₂CF₃ | CH₂OC(=O)Me | Me | C(=O)Me | O |
| D-6797 | Me | single bond | H | CH₂CF₃ | CH₂OC(=O)Me | Me | C(=O)Me | O |
| D-6798 | Me(R) | single bond | H | CH₂CF₃ | CH₂OC(=O)Me | Me | C(=O)Me | O |
| D-6799 | Me(S) | single bond | H | CH₂CF₃ | CH₂OC(=O)Me | Me | C(=O)Me | O |
| D-6800 | H | CH₂ | Me | CH₂CF₃ | CH₂OC(=O)Me | Me | C(=O)Me | O |
| D-6801 | Me | CH₂ | H | CH₂CF₃ | CH₂OC(=O)Me | Me | C(=O)Me | O |
| D-6802 | H | CH(Me) | H | CH₂CF₃ | CH₂OC(=O)Me | Me | C(=O)Me | O |
| D-6803 | H | CH₂CH₂ | Me | CH₂CF₃ | CH₂OC(=O)Me | Me | C(=O)Me | O |
| D-6804 | Me | CH₂CH₂ | H | CH₂CF₃ | CH₂OC(=O)Me | Me | C(=O)Me | O |
| D-6805 | H | single bond | Me | CH₂CF₃ | CH₂OC(=O)(*i*-Pr) | Cl | C(=O)Me | O |
| D-6806 | Me | single bond | H | CH₂CF₃ | CH₂OC(=O)(*i*-Pr) | Cl | C(=O)Me | O |
| D-6807 | Me(R) | single bond | H | CH₂CF₃ | CH₂OC(=O)(*i*-Pr) | Cl | C(=O)Me | O |
| D-6808 | Me(S) | single bond | H | CH₂CF₃ | CH₂OC(=O)(*i*-Pr) | Cl | C(=O)Me | O |
| D-6809 | H | CH₂ | Me | CH₂CF₃ | CH₂OC(=O)(*i*-Pr) | Cl | C(=O)Me | O |
| D-6810 | Me | CH₂ | H | CH₂CF₃ | CH₂OC(=O)(*i*-Pr) | Cl | C(=O)Me | O |
| D-6811 | H | CH(Me) | H | CH₂CF₃ | CH₂OC(=O)(*i*-Pr) | Cl | C(=O)Me | O |
| D-6812 | H | CH₂CH₂ | Me | CH₂CF₃ | CH₂OC(=O)(*i*-Pr) | Cl | C(=O)Me | O |
| D-6813 | Me | CH₂CH₂ | H | CH₂CF₃ | CH₂OC(=O)(*i*-Pr) | Cl | C(=O)Me | O |
| D-6814 | H | single bond | Me | CH₂CF₃ | CH₂OC(=O)(*i*-Pr) | Me | C(=O)Me | O |
| D-6815 | Me | single bond | H | CH₂CF₃ | CH₂OC(=O)(*i*-Pr) | Me | C(=O)Me | O |
| D-6816 | Me(R) | single bond | H | CH₂CF₃ | CH₂OC(=O)(*i*-Pr) | Me | C(=O)Me | O |
| D-6817 | Me(S) | single bond | H | CH₂CF₃ | CH₂OC(=O)(*i*-Pr) | Me | C(=O)Me | O |
| D-6818 | H | CH₂ | Me | CH₂CF₃ | CH₂OC(=O)(*i*-Pr) | Me | C(=O)Me | O |
| D-6819 | Me | CH₂ | H | CH₂CF₃ | CH₂OC(=O)(*i*-Pr) | Me | C(=O)Me | O |
| D-6820 | H | CH(Me) | H | CH₂CF₃ | CH₂OC(=O)(*i*-Pr) | Me | C(=O)Me | O |
| D-6821 | H | CH₂CH₂ | Me | CH₂CF₃ | CH₂OC(=O)(*i*-Pr) | Me | C(=O)Me | O |
| D-6822 | Me | CH₂CH₂ | H | CH₂CF₃ | CH₂OC(=O)(*i*-Pr) | Me | C(=O)Me | O |
| D-6823 | H | single bond | Me | CH₂CF₃ | CH₂OC(=O)(*t*-Bu) | Cl | C(=O)Me | O |
| D-6824 | Me | single bond | H | CH₂CF₃ | CH₂OC(=O)(*t*-Bu) | Cl | C(=O)Me | O |
| D-6825 | Me(R) | single bond | H | CH₂CF₃ | CH₂OC(=O)(*t*-Bu) | Cl | C(=O)Me | O |
| D-6826 | Me(S) | single bond | H | CH₂CF₃ | CH₂OC(=O)(*t*-Bu) | Cl | C(=O)Me | O |

**[Table 801]**

| Compound Number | Y | W | Z | R¹ | R² | R³ | R¹⁰ | A |
|---|---|---|---|---|---|---|---|---|
| D-6827 | H | CH₂ | Me | CH₂CF₃ | CH₂OC(=O)(*t*-Bu) | Cl | C(=O)Me | O |
| D-6828 | Me | CH₂ | H | CH₂CF₃ | CH₂OC(=O)(*t*-Bu) | Cl | C(=O)Me | O |
| D-6829 | H | CH(Me) | H | CH₂CF₃ | CH₂OC(=O)(*t*-Bu) | Cl | C(=O)Me | O |
| D-6830 | H | CH₂CH₂ | Me | CH₂CF₃ | CH₂OC(=O)(*t*-Bu) | Cl | C(=O)Me | O |
| D-6831 | Me | CH₂CH₂ | H | CH₂CF₃ | CH₂OC(=O)(*t*-Bu) | Cl | C(=O)Me | O |
| D-6832 | H | single bond | Me | CH₂CF₃ | CH₂OC(=O)(*t*-Bu) | Me | C(=O)Me | O |
| D-6833 | Me | single bond | H | CH₂CF₃ | CH₂OC(=O)(*t*-Bu) | Me | C(=O)Me | O |
| D-6834 | Me(R) | single bond | H | CH₂CF₃ | CH₂OC(=O)(*t*-Bu) | Me | C(=O)Me | O |
| D-6835 | Me(S) | single bond | H | CH₂CF₃ | CH₂OC(=O)(*t*-Bu) | Me | C(=O)Me | O |
| D-6836 | H | CH₂ | Me | CH₂CF₃ | CH₂OC(=O)(*t*-Bu) | Me | C(=O)Me | O |
| D-6837 | Me | CH₂ | H | CH₂CF₃ | CH₂OC(=O)(*t*-Bu) | Me | C(=O)Me | O |
| D-6838 | H | CH(Me) | H | CH₂CF₃ | CH₂OC(=O)(*t*-Bu) | Me | C(=O)Me | O |
| D-6839 | H | CH₂CH₂ | Me | CH₂CF₃ | CH₂OC(=O)(*t*-Bu) | Me | C(=O)Me | O |
| D-6840 | Me | CH₂CH₂ | H | CH₂CF₃ | CH₂OC(=O)(*t*-Bu) | Me | C(=O)Me | O |
| D-6841 | H | single bond | Me | CH₂CF₃ | CH₂OC(=O)OEt | Cl | C(=O)Me | O |
| D-6842 | Me | single bond | H | CH₂CF₃ | CH₂OC(=O)OEt | Cl | C(=O)Me | O |
| D-6843 | Me(R) | single bond | H | CH₂CF₃ | CH₂OC(=O)OEt | Cl | C(=O)Me | O |
| D-6844 | Me(S) | single bond | H | CH₂CF₃ | CH₂OC(=O)OEt | Cl | C(=O)Me | O |
| D-6845 | H | CH₂ | Me | CH₂CF₃ | CH₂OC(=O)OEt | Cl | C(=O)Me | O |
| D-6846 | Me | CH₂ | H | CH₂CF₃ | CH₂OC(=O)OEt | Cl | C(=O)Me | O |
| D-6847 | H | CH(Me) | H | CH₂CF₃ | CH₂OC(=O)OEt | Cl | C(=O)Me | O |
| D-6848 | H | CH₂CH₂ | Me | CH₂CF₃ | CH₂OC(=O)OEt | Cl | C(=O)Me | O |
| D-6849 | Me | CH₂CH₂ | H | CH₂CF₃ | CH₂OC(=O)OEt | Cl | C(=O)Me | O |
| D-6850 | H | single bond | Me | CH₂CF₃ | CH₂OC(=O)OEt | Me | C(=O)Me | O |
| D-6851 | Me | single bond | H | CH₂CF₃ | CH₂OC(=O)OEt | Me | C(=O)Me | O |
| D-6852 | Me(R) | single bond | H | CH₂CF₃ | CH₂OC(=O)OEt | Me | C(=O)Me | O |
| D-6853 | Me(S) | single bond | H | CH₂CF₃ | CH₂OC(=O)OEt | Me | C(=O)Me | O |
| D-6854 | H | CH₂ | Me | CH₂CF₃ | CH₂OC(=O)OEt | Me | C(=O)Me | O |
| D-6855 | Me | CH₂ | H | CH₂CF₃ | CH₂OC(=O)OEt | Me | C(=O)Me | O |
| D-6856 | H | CH(Me) | H | CH₂CF₃ | CH₂OC(=O)OEt | Me | C(=O)Me | O |
| D-6857 | H | CH₂CH₂ | Me | CH₂CF₃ | CH₂OC(=O)OEt | Me | C(=O)Me | O |
| D-6858 | Me | CH₂CH₂ | H | CH₂CF₃ | CH₂OC(=O)OEt | Me | C(=O)Me | O |
| D-6859 | H | single bond | Me | CH₂CF₃ | CH₂OC(=O)O(*i*-Pr) | Cl | C(=O)Me | O |
| D-6860 | Me | single bond | H | CH₂CF₃ | CH₂OC(=O)O(*i*-Pr) | Cl | C(=O)Me | O |
| D-6861 | Me(R) | single bond | H | CH₂CF₃ | CH₂OC(=O)O(*i*-Pr) | Cl | C(=O)Me | O |
| D-6862 | Me(S) | single bond | H | CH₂CF₃ | CH₂OC(=O)O(*i*-Pr) | Cl | C(=O)Me | O |
| D-6863 | H | CH₂ | Me | CH₂CF₃ | CH₂OC(=O)O(*i*-Pr) | Cl | C(=O)Me | O |
| D-6864 | Me | CH₂ | H | CH₂CF₃ | CH₂OC(=O)O(*i*-Pr) | Cl | C(=O)Me | O |
| D-6865 | H | CH(Me) | H | CH₂CF₃ | CH₂OC(=O)O(*i*-Pr) | Cl | C(=O)Me | O |
| D-6866 | H | CH₂CH₂ | Me | CH₂CF₃ | CH₂OC(=O)O(*i*-Pr) | Cl | C(=O)Me | O |
| D-6867 | Me | CH₂CH₂ | H | CH₂CF₃ | CH₂OC(=O)O(*i*-Pr) | Cl | C(=O)Me | O |
| D-6868 | H | single bond | Me | CH₂CF₃ | CH₂OC(=O)O(*i*-Pr) | Me | C(=O)Me | O |
| D-6869 | Me | single bond | H | CH₂CF₃ | CH₂OC(=O)O(*i*-Pr) | Me | C(=O)Me | O |
| D-6870 | Me(R) | single bond | H | CH₂CF₃ | CH₂OC(=O)O(*i*-Pr) | Me | C(=O)Me | O |

**[Table 802]**

| Compound Number | Y | W | Z | R¹ | R² | R³ | R¹⁰ | A |
|---|---|---|---|---|---|---|---|---|
| D-6871 | Me(S) | single bond | H | CH₂CF₃ | CH₂OC(=O)O(*i*-Pr) | Me | C(=O)Me | O |
| D-6872 | H | CH₂ | Me | CH₂CF₃ | CH₂OC(=O)O(*i*-Pr) | Me | C(=O)Me | O |
| D-6873 | Me | CH₂ | H | CH₂CF₃ | CH₂OC(=O)O(*i*-Pr) | Me | C(=O)Me | O |
| D-6874 | H | CH(Me) | H | CH₂CF₃ | CH₂OC(=O)O(*i*-Pr) | Me | C(=O)Me | O |
| D-6875 | H | CH₂CH₂ | Me | CH₂CF₃ | CH₂OC(=O)O(*i*-Pr) | Me | C(=O)Me | O |
| D-6876 | Me | CH₂CH₂ | H | CH₂CF₃ | CH₂OC(=O)O(*i*-Pr) | Me | C(=O)Me | O |
| D-6877 | H | single bond | Me | CH₂CF₃ | CH₂N(Me)C(=O)OMe | Cl | C(=O)Me | O |
| D-6878 | Me | single bond | H | CH₂CF₃ | CH₂N(Me)C(=O)OMe | Cl | C(=O)Me | O |
| D-6879 | Me(R) | single bond | H | CH₂CF₃ | CH₂N(Me)C(=O)OMe | Cl | C(=O)Me | O |
| D-6880 | Me(S) | single bond | H | CH₂CF₃ | CH₂N(Me)C(=O)OMe | Cl | C(=O)Me | O |
| D-6881 | H | CH₂ | Me | CH₂CF₃ | CH₂N(Me)C(=O)OMe | Cl | C(=O)Me | O |
| D-6882 | Me | CH₂ | H | CH₂CF₃ | CH₂N(Me)C(=O)OMe | Cl | C(=O)Me | O |
| D-6883 | H | CH(Me) | H | CH₂CF₃ | CH₂N(Me)C(=O)OMe | Cl | C(=O)Me | O |
| D-6884 | H | CH₂CH₂ | Me | CH₂CF₃ | CH₂N(Me)C(=O)OMe | Cl | C(=O)Me | O |
| D-6885 | Me | CH₂CH₂ | H | CH₂CF₃ | CH₂N(Me)C(=O)OMe | Cl | C(=O)Me | O |
| D-6886 | H | single bond | Me | CH₂CF₃ | CH₂N(Me)C(=O)OMe | Me | C(=O)Me | O |
| D-6887 | Me | single bond | H | CH₂CF₃ | CH₂N(Me)C(=O)OMe | Me | C(=O)Me | O |
| D-6888 | Me(R) | single bond | H | CH₂CF₃ | CH₂N(Me)C(=O)OMe | Me | C(=O)Me | O |
| D-6889 | Me(S) | single bond | H | CH₂CF₃ | CH₂N(Me)C(=O)OMe | Me | C(=O)Me | O |
| D-6890 | H | CH₂ | Me | CH₂CF₃ | CH₂N(Me)C(=O)OMe | Me | C(=O)Me | O |
| D-6891 | Me | CH₂ | H | CH₂CF₃ | CH₂N(Me)C(=O)OMe | Me | C(=O)Me | O |
| D-6892 | H | CH(Me) | H | CH₂CF₃ | CH₂N(Me)C(=O)OMe | Me | C(=O)Me | O |
| D-6893 | H | CH₂CH₂ | Me | CH₂CF₃ | CH₂N(Me)C(=O)OMe | Me | C(=O)Me | O |
| D-6894 | Me | CH₂CH₂ | H | CH₂CF₃ | CH₂N(Me)C(=O)OMe | Me | C(=O)Me | O |
| D-6895 | H | single bond | Me | Et | C(=O)OCH₂CH=CH₂ | Cl | H | O |
| D-6896 | Me | single bond | H | Et | C(=O)OCH₂CH=CH₂ | Cl | H | O |
| D-6897 | Me(R) | single bond | H | Et | C(=O)OCH₂CH=CH₂ | Cl | H | O |
| D-6898 | Me(S) | single bond | H | Et | C(=O)OCH₂CH=CH₂ | Cl | H | O |
| D-6899 | H | CH₂ | Me | Et | C(=O)OCH₂CH=CH₂ | Cl | H | O |
| D-6900 | Me | CH₂ | H | Et | C(=O)OCH₂CH=CH₂ | Cl | H | O |
| D-6901 | H | CH(Me) | H | Et | C(=O)OCH₂CH=CH₂ | Cl | H | O |
| D-6902 | H | CH₂CH₂ | Me | Et | C(=O)OCH₂CH=CH₂ | Cl | H | O |
| D-6903 | Me | CH₂CH₂ | H | Et | C(=O)OCH₂CH=CH₂ | Cl | H | O |
| D-6904 | H | single bond | Me | Et | C(=O)OCH₂CH=CH₂ | Me | H | O |
| D-6905 | Me | single bond | H | Et | C(=O)OCH₂CH=CH₂ | Me | H | O |
| D-6906 | Me(R) | single bond | H | Et | C(=O)OCH₂CH=CH₂ | Me | H | O |
| D-6907 | Me(S) | single bond | H | Et | C(=O)OCH₂CH=CH₂ | Me | H | O |
| D-6908 | H | CH₂ | Me | Et | C(=O)OCH₂CH=CH₂ | Me | H | O |
| D-6909 | Me | CH₂ | H | Et | C(=O)OCH₂CH=CH₂ | Me | H | O |
| D-6910 | H | CH(Me) | H | Et | C(=O)OCH₂CH=CH₂ | Me | H | O |
| D-6911 | H | CH₂CH₂ | Me | Et | C(=O)OCH₂CH=CH₂ | Me | H | O |
| D-6912 | Me | CH₂CH₂ | H | Et | C(=O)OCH₂CH=CH₂ | Me | H | O |
| D-6913 | H | single bond | Me | Et | C(=O)OCH₂CCl₃ | Cl | H | O |
| D-6914 | Me | single bond | H | Et | C(=O)OCH₂CCl₃ | Cl | H | O |

**[Table 803]**

| Compound Number | Y | W | z | R¹ | R² | R³ | R¹⁰ | A |
|---|---|---|---|---|---|---|---|---|
| D-6915 | Me(R) | single bond | H | Et | C(=O)OCH₂CCl₃ | Cl | H | O |
| D-6916 | Me(S) | single bond | H | Et | C(=O)OCH₂CCl₃ | Cl | H | O |
| D-6917 | H | CH₂ | Me | Et | C(=O)OCH₂CCl₃ | Cl | H | O |
| D-6918 | Me | CH₂ | H | Et | C(=O)OCH₂CCl₃ | Cl | H | O |
| D-6919 | H | CH(Me) | H | Et | C(=O)OCH₂CCl₃ | Cl | H | O |
| D-6920 | H | CH₂CH₂ | Me | Et | C(=O)OCH₂CCl₃ | Cl | H | O |
| D-6921 | Me | CH₂CH₂ | H | Et | C(=O)OCH₂CCl₃ | Cl | H | O |
| D-6922 | H | single bond | Me | Et | C(=O)OCH₂CCl₃ | Me | H | O |
| D-6923 | Me | single bond | H | Et | C(=O)OCH₂CCl₃ | Me | H | O |
| D-6924 | Me(R) | single bond | H | Et | C(=O)OCH₂CCl₃ | Me | H | O |
| D-6925 | Me(S) | single bond | H | Et | C(=O)OCH₂CCl₃ | Me | H | O |
| D-6926 | H | CH₂ | Me | Et | C(=O)OCH₂CCl₃ | Me | H | O |
| D-6927 | Me | CH₂ | H | Et | C(=O)OCH₂CCl₃ | Me | H | O |
| D-6928 | H | CH(Me) | H | Et | C(=O)OCH₂CCl₃ | Me | H | O |
| D-6929 | H | CH₂CH₂ | Me | Et | C(=O)OCH₂CCl₃ | Me | H | O |
| D-6930 | Me | CH₂CH₂ | H | Et | C(=O)OCH₂CCl₃ | Me | H | O |
| D-6931 | H | single bond | Me | Et | C(=O)N(Me)₂ | Cl | H | O |
| D-6932 | Me | single bond | H | Et | C(=O)N(Me)₂ | Cl | H | O |
| D-6933 | Me(R) | single bond | H | Et | C(=O)N(Me)₂ | Cl | H | O |
| D-6934 | Me(S) | single bond | H | Et | C(=O)N(Me)₂ | Cl | H | O |
| D-6935 | H | CH₂ | Me | Et | C(=O)N(Me)₂ | Cl | H | O |
| D-6936 | Me | CH₂ | H | Et | C(=O)N(Me)₂ | Cl | H | O |
| D-6937 | H | CH(Me) | H | Et | C(=O)N(Me)₂ | Cl | H | O |
| D-6938 | H | CH₂CH₂ | Me | Et | C(=O)N(Me)₂ | Cl | H | O |
| D-6939 | Me | CH₂CH₂ | H | Et | C(=O)N(Me)₂ | Cl | H | O |
| D-6940 | H | single bond | Me | Et | C(=O)N(Me)₂ | Me | H | O |
| D-6941 | Me | single bond | H | Et | C(=O)N(Me)₂ | Me | H | O |
| D-6942 | Me(R) | single bond | H | Et | C(=O)N(Me)₂ | Me | H | O |
| D-6943 | Me(S) | single bond | H | Et | C(=O)N(Me)₂ | Me | H | O |
| D-6944 | H | CH₂ | Me | Et | C(=O)N(Me)₂ | Me | H | O |
| D-6945 | Me | CH₂ | H | Et | C(=O)N(Me)₂ | Me | H | O |
| D-6946 | H | CH(Me) | H | Et | C(=O)N(Me)₂ | Me | H | O |
| D-6947 | H | CH₂CH₂ | Me | Et | C(=O)N(Me)₂ | Me | H | O |
| D-6948 | Me | CH₂CH₂ | H | Et | C(=O)N(Me)₂ | Me | H | O |
| D-6949 | H | single bond | Me | Et | CH₂OC(=O)Me | Cl | H | O |
| D-6950 | Me | single bond | H | Et | CH₂OC(=O)Me | Cl | H | O |
| D-6951 | Me(R) | single bond | H | Et | CH₂OC(=O)Me | Cl | H | O |
| D-6952 | Me(S) | single bond | H | Et | CH₂OC(=O)Me | Cl | H | O |
| D-6953 | H | CH₂ | Me | Et | CH₂OC(=O)Me | Cl | H | O |
| D-6954 | Me | CH₂ | H | Et | CH₂OC(=O)Me | Cl | H | O |
| D-6955 | H | CH(Me) | H | Et | CH₂OC(=O)Me | Cl | H | O |
| D-6956 | H | CH₂CH₂ | Me | Et | CH₂OC(=O)Me | Cl | H | O |
| D-6957 | Me | CH₂CH₂ | H | Et | CH₂OC(=O)Me | Cl | H | O |
| D-6958 | H | single bond | Me | Et | CH₂OC(=O)Me | Me | H | O |

**[Table 804]**

| Compound Number | Y | W | z | R¹ | R² | R³ | R¹⁰ | A |
|---|---|---|---|---|---|---|---|---|
| D-6959 | Me | single bond | H | Et | CH₂OC(=O)Me | Me | H | O |
| D-6960 | Me(R) | single bond | H | Et | CH₂OC(=O)Me | Me | H | O |
| D-6961 | Me(S) | single bond | H | Et | CH₂OC(=O)Me | Me | H | O |
| D-6962 | H | CH₂ | Me | Et | CH₂OC(=O)Me | Me | H | O |
| D-6963 | Me | CH₂ | H | Et | CH₂OC(=O)Me | Me | H | O |
| D-6964 | H | CH(Me) | H | Et | CH₂OC(=O)Me | Me | H | O |
| D-6965 | H | CH₂CH₂ | Me | Et | CH₂OC(=O)Me | Me | H | O |
| D-6966 | Me | CH₂CH₂ | H | Et | CH₂OC(=O)Me | Me | H | O |
| D-6967 | H | single bond | Me | Et | CH₂OC(=O)(*i*-Pr) | Cl | H | O |
| D-6968 | Me | single bond | H | Et | CH₂OC(=O)(*i*-Pr) | Cl | H | O |
| D-6969 | Me(R) | single bond | H | Et | CH₂OC(=O)(*i*-Pr) | Cl | H | O |
| D-6970 | Me(S) | single bond | H | Et | CH₂OC(=O)(*i*-Pr) | Cl | H | O |
| D-6971 | H | CH₂ | Me | Et | CH₂OC(=O)(*i*-Pr) | Cl | H | O |
| D-6972 | Me | CH₂ | H | Et | CH₂OC(=O)(*i*-Pr) | Cl | H | O |
| D-6973 | H | CH(Me) | H | Et | CH₂OC(=O)(*i*-Pr) | Cl | H | O |
| D-6974 | H | CH₂CH₂ | Me | Et | CH₂OC(=O)(*i*-Pr) | Cl | H | O |
| D-6975 | Me | CH₂CH₂ | H | Et | CH₂OC(=O)(*i*-Pr) | Cl | H | O |
| D-6976 | H | single bond | Me | Et | CH₂OC(=O)(*i*-Pr) | Me | H | O |
| D-6977 | Me | single bond | H | Et | CH₂OC(=O)(*i*-Pr) | Me | H | O |
| D-6978 | Me(R) | single bond | H | Et | CH₂OC(=O)(*i*-Pr) | Me | H | O |
| D-6979 | Me(S) | single bond | H | Et | CH₂OC(=O)(*i*-Pr) | Me | H | O |
| D-6980 | H | CH₂ | Me | Et | CH₂OC(=O)(*i*-Pr) | Me | H | O |
| D-6981 | Me | CH₂ | H | Et | CH₂OC(=O)(*i*-Pr) | Me | H | O |
| D-6982 | H | CH(Me) | H | Et | CH₂OC(=O)(*i*-Pr) | Me | H | O |
| D-6983 | H | CH₂CH₂ | Me | Et | CH₂OC(=O)(*i*-Pr) | Me | H | O |
| D-6984 | Me | CH₂CH₂ | H | Et | CH₂OC(=O)(*i*-Pr) | Me | H | O |
| D-6985 | H | single bond | Me | Et | CH₂OC(=O)(*t*-Bu) | Cl | H | O |
| D-6986 | Me | single bond | H | Et | CH₂OC(=O)(*t*-Bu) | Cl | H | O |
| D-6987 | Me(R) | single bond | H | Et | CH₂OC(=O)(*t*-Bu) | Cl | H | O |
| D-6988 | Me(S) | single bond | H | Et | CH₂OC(=O)(*t*-Bu) | Cl | H | O |
| D-6989 | H | CH₂ | Me | Et | CH₂OC(=O)(*t*-Bu) | Cl | H | O |
| D-6990 | Me | CH₂ | H | Et | CH₂OC(=O)(*t*-Bu) | Cl | H | O |
| D-6991 | H | CH(Me) | H | Et | CH₂OC(=O)(*t*-Bu) | Cl | H | O |
| D-6992 | H | CH₂CH₂ | Me | Et | CH₂OC(=O)(*t*-Bu) | Cl | H | O |
| D-6993 | Me | CH₂CH₂ | H | Et | CH₂OC(=O)(*t*-Bu) | Cl | H | O |
| D-6994 | H | single bond | Me | Et | CH₂OC(=O)(*t*-Bu) | Me | H | O |
| D-6995 | Me | single bond | H | Et | CH₂OC(=O)(*t*-Bu) | Me | H | O |
| D-6996 | Me(R) | single bond | H | Et | CH₂OC(=O)(*t*-Bu) | Me | H | O |
| D-6997 | Me(S) | single bond | H | Et | CH₂OC(=O)(*t*-Bu) | Me | H | O |
| D-6998 | H | CH₂ | Me | Et | CH₂OC(=O)(*t*-Bu) | Me | H | O |
| D-6999 | Me | CH₂ | H | Et | CH₂OC(=O)(*t*-Bu) | Me | H | O |
| D-7000 | H | CH(Me) | H | Et | CH₂OC(=O)(*t*-Bu) | Me | H | O |
| D-7001 | H | CH₂CH₂ | Me | Et | CH₂OC(=O)(*t*-Bu) | Me | H | O |
| D-7002 | Me | CH₂CH₂ | H | Et | CH₂OC(=O)(*t*-Bu) | Me | H | O |

**[Table 805]**

| Compound Number | Y | W | z | R¹ | R² | R³ | R¹⁰ | A |
|---|---|---|---|---|---|---|---|---|
| D-7003 | H | single bond | Me | Et | CH₂OC(=O)OEt | Cl | H | O |
| D-7004 | Me | single bond | H | Et | CH₂OC(=O)OEt | Cl | H | O |
| D-7005 | Me(R) | single bond | H | Et | CH₂OC(=O)OEt | Cl | H | O |
| D-7006 | Me(S) | single bond | H | Et | CH₂OC(=O)OEt | Cl | H | O |
| D-7007 | H | CH₂ | Me | Et | CH₂OC(=O)OEt | Cl | H | O |
| D-7008 | Me | CH₂ | H | Et | CH₂OC(=O)OEt | Cl | H | O |
| D-7009 | H | CH(Me) | H | Et | CH₂OC(=O)OEt | Cl | H | O |
| D-7010 | H | CH₂CH₂ | Me | Et | CH₂OC(=O)OEt | Cl | H | O |
| D-7011 | Me | CH₂CH₂ | H | Et | CH₂OC(=O)OEt | Cl | H | O |
| D-7012 | H | single bond | Me | Et | CH₂OC(=O)OEt | Me | H | O |
| D-7013 | Me | single bond | H | Et | CH₂OC(=O)OEt | Me | H | O |
| D-7014 | Me(R) | single bond | H | Et | CH₂OC(=O)OEt | Me | H | O |
| D-7015 | Me(S) | single bond | H | Et | CH₂OC(=O)OEt | Me | H | O |
| D-7016 | H | CH₂ | Me | Et | CH₂OC(=O)OEt | Me | H | O |
| D-7017 | Me | CH₂ | H | Et | CH₂OC(=O)OEt | Me | H | O |
| D-7018 | H | CH(Me) | H | Et | CH₂OC(=O)OEt | Me | H | O |
| D-7019 | H | CH₂CH₂ | Me | Et | CH₂OC(=O)OEt | Me | H | O |
| D-7020 | Me | CH₂CH₂ | H | Et | CH₂OC(=O)OEt | Me | H | O |
| D-7021 | H | single bond | Me | Et | CH₂OC(=O)O(*i*-Pr) | Cl | H | O |
| D-7022 | Me | single bond | H | Et | CH₂OC(=O)O(*i*-Pr) | Cl | H | O |
| D-7023 | Me(R) | single bond | H | Et | CH₂OC(=O)O(*i*-Pr) | Cl | H | O |
| D-7024 | Me(S) | single bond | H | Et | CH₂OC(=O)O(*i*-Pr) | Cl | H | O |
| D-7025 | H | CH₂ | Me | Et | CH₂OC(=O)O(*i*-Pr) | Cl | H | O |
| D-7026 | Me | CH₂ | H | Et | CH₂OC(=O)O(*i*-Pr) | Cl | H | O |
| D-7027 | H | CH(Me) | H | Et | CH₂OC(=O)O(*i*-Pr) | Cl | H | O |
| D-7028 | H | CH₂CH₂ | Me | Et | CH₂OC(=O)O(*i*-Pr) | Cl | H | O |
| D-7029 | Me | CH₂CH₂ | H | Et | CH₂OC(=O)O(*i*-Pr) | Cl | H | O |
| D-7030 | H | single bond | Me | Et | CH₂OC(=O)O(*i*-Pr) | Me | H | O |
| D-7031 | Me | single bond | H | Et | CH₂OC(=O)O(*i*-Pr) | Me | H | O |
| D-7032 | Me(R) | single bond | H | Et | CH₂OC(=O)O(*i*-Pr) | Me | H | O |
| D-7033 | Me(S) | single bond | H | Et | CH₂OC(=O)O(*i*-Pr) | Me | H | O |
| D-7034 | H | CH₂ | Me | Et | CH₂OC(=O)O(*i*-Pr) | Me | H | O |
| D-7035 | Me | CH₂ | H | Et | CH₂OC(=O)O(*i*-Pr) | Me | H | O |
| D-7036 | H | CH(Me) | H | Et | CH₂OC(=O)O(*i*-Pr) | Me | H | O |
| D-7037 | H | CH₂CH₂ | Me | Et | CH₂OC(=O)O(*i*-Pr) | Me | H | O |
| D-7038 | Me | CH₂CH₂ | H | Et | CH₂OC(=O)O(*i*-Pr) | Me | H | O |
| D-7039 | H | single bond | Me | Et | CH₂N(Me)C(=O)OMe | Cl | H | O |
| D-7040 | Me | single bond | H | Et | CH₂N(Me)C(=O)OMe | Cl | H | O |
| D-7041 | Me(R) | single bond | H | Et | CH₂N(Me)C(=O)OMe | Cl | H | O |
| D-7042 | Me(S) | single bond | H | Et | CH₂N(Me)C(=O)OMe | Cl | H | O |
| D-7043 | H | CH₂ | Me | Et | CH₂N(Me)C(=O)OMe | Cl | H | O |
| D-7044 | Me | CH₂ | H | Et | CH₂N(Me)C(=O)OMe | Cl | H | O |
| D-7045 | H | CH(Me) | H | Et | CH₂N(Me)C(=O)OMe | Cl | H | O |
| D-7046 | H | CH₂CH₂ | Me | Et | CH₂N(Me)C(=O)OMe | Cl | H | O |

**[Table 806]**

| Compound Number | Y | W | z | R¹ | R² | R³ | R¹⁰ | A |
|---|---|---|---|---|---|---|---|---|
| D-7047 | Me | CH₂CH₂ | H | Et | CH₂N(Me)C(=O)OMe | Cl | H | O |
| D-7048 | H | single bond | Me | Et | CH₂N(Me)C(=O)OMe | Me | H | O |
| D-7049 | Me | single bond | H | Et | CH₂N(Me)C(=O)OMe | Me | H | O |
| D-7050 | Me(R) | single bond | H | Et | CH₂N(Me)C(=O)OMe | Me | H | O |
| D-7051 | Me(S) | single bond | H | Et | CH₂N(Me)C(=O)OMe | Me | H | O |
| D-7052 | H | CH₂ | Me | Et | CH₂N(Me)C(=O)OMe | Me | H | O |
| D-7053 | Me | CH₂ | H | Et | CH₂N(Me)C(=O)OMe | Me | H | O |
| D-7054 | H | CH(Me) | H | Et | CH₂N(Me)C(=O)OMe | Me | H | O |
| D-7055 | H | CH₂CH₂ | Me | Et | CH₂N(Me)C(=O)OMe | Me | H | O |
| D-7056 | Me | CH₂CH₂ | H | Et | CH₂N(Me)C(=O)OMe | Me | H | O |
| D-7057 | H | single bond | Me | Et | C(=O)OCH₂CH=CH₂ | Cl | Me | O |
| D-7058 | Me | single bond | H | Et | C(=O)OCH₂CH=CH₂ | Cl | Me | O |
| D-7059 | Me(R) | single bond | H | Et | C(=O)OCH₂CH=CH₂ | Cl | Me | O |
| D-7060 | Me(S) | single bond | H | Et | C(=O)OCH₂CH=CH₂ | Cl | Me | O |
| D-7061 | H | CH₂ | Me | Et | C(=O)OCH₂CH=CH₂ | Cl | Me | O |
| D-7062 | Me | CH₂ | H | Et | C(=O)OCH₂CH=CH₂ | Cl | Me | O |
| D-7063 | H | CH(Me) | H | Et | C(=O)OCH₂CH=CH₂ | Cl | Me | O |
| D-7064 | H | CH₂CH₂ | Me | Et | C(=O)OCH₂CH=CH₂ | Cl | Me | O |
| D-7065 | Me | CH₂CH₂ | H | Et | C(=O)OCH₂CH=CH₂ | Cl | Me | O |
| D-7066 | H | single bond | Me | Et | C(=O)OCH₂CH=CH₂ | Me | Me | O |
| D-7067 | Me | single bond | H | Et | C(=O)OCH₂CH=CH₂ | Me | Me | O |
| D-7068 | Me(R) | single bond | H | Et | C(=O)OCH₂CH=CH₂ | Me | Me | O |
| D-7069 | Me(S) | single bond | H | Et | C(=O)OCH₂CH=CH₂ | Me | Me | O |
| D-7070 | H | CH₂ | Me | Et | C(=O)OCH₂CH=CH₂ | Me | Me | O |
| D-7071 | Me | CH₂ | H | Et | C(=O)OCH₂CH=CH₂ | Me | Me | O |
| D-7072 | H | CH(Me) | H | Et | C(=O)OCH₂CH=CH₂ | Me | Me | O |
| D-7073 | H | CH₂CH₂ | Me | Et | C(=O)OCH₂CH=CH₂ | Me | Me | O |
| D-7074 | Me | CH₂CH₂ | H | Et | C(=O)OCH₂CH=CH₂ | Me | Me | O |
| D-7075 | H | single bond | Me | Et | C(=O)OCH₂CCl₃ | Cl | Me | O |
| D-7076 | Me | single bond | H | Et | C(=O)OCH₂CCl₃ | Cl | Me | O |
| D-7077 | Me(R) | single bond | H | Et | C(=O)OCH₂CCl₃ | Cl | Me | O |
| D-7078 | Me(S) | single bond | H | Et | C(=O)OCH₂CCl₃ | Cl | Me | O |
| D-7079 | H | CH₂ | Me | Et | C(=O)OCH₂CCl₃ | Cl | Me | O |
| D-7080 | Me | CH₂ | H | Et | C(=O)OCH₂CCl₃ | Cl | Me | O |
| D-7081 | H | CH(Me) | H | Et | C(=O)OCH₂CCl₃ | Cl | Me | O |
| D-7082 | H | CH₂CH₂ | Me | Et | C(=O)OCH₂CCl₃ | Cl | Me | O |
| D-7083 | Me | CH₂CH₂ | H | Et | C(=O)OCH₂CCl₃ | Cl | Me | O |
| D-7084 | H | single bond | Me | Et | C(=O)OCH₂CCl₃ | Me | Me | O |
| D-7085 | Me | single bond | H | Et | C(=O)OCH₂CCl₃ | Me | Me | O |
| 0-7086 | Me(R) | single bond | H | Et | C(=O)OCH₂CCl₃ | Me | Me | O |
| D-7087 | Me(S) | single bond | H | Et | C(=O)OCH₂CCl₃ | Me | Me | O |
| 0-7088 | H | CH₂ | Me | Et | C(=O)OCH₂CCl₃ | Me | Me | O |
| D-7089 | Me | CH₂ | H | Et | C(=O)OCH₂CCl₃ | Me | Me | O |
| D-7090 | H | CH(Me) | H | Et | C(=O)OCH₂CCl₃ | Me | Me | O |

**[Table 807]**

| Compound Number | Y | W | z | R¹ | R² | R³ | R¹⁰ | A |
|---|---|---|---|---|---|---|---|---|
| D-7091 | H | CH₂CH₂ | Me | Et | C(=O)OCH₂CCl₃ | Me | Me | O |
| D-7092 | Me | CH₂CH₂ | H | Et | C(=O)OCH₂CCl₃ | Me | Me | O |
| D-7093 | H | single bond | Me | Et | C(=O)N(Me)₂ | Cl | Me | O |
| D-7094 | Me | single bond | H | Et | C(=O)N(Me)₂ | Cl | Me | O |
| D-7095 | Me(R) | single bond | H | Et | C(=O)N(Me)₂ | Cl | Me | O |
| D-7096 | Me(S) | single bond | H | Et | C(=O)N(Me)₂ | Cl | Me | O |
| D-7097 | H | CH₂ | Me | Et | C(=O)N(Me)₂ | Cl | Me | O |
| D-7098 | Me | CH₂ | H | Et | C(=O)N(Me)₂ | Cl | Me | O |
| D-7099 | H | CH(Me) | H | Et | C(=O)N(Me)₂ | Cl | Me | O |
| D-7100 | H | CH₂CH₂ | Me | Et | C(=O)N(Me)₂ | Cl | Me | O |
| D-7101 | Me | CH₂CH₂ | H | Et | C(=O)N(Me)₂ | Cl | Me | O |
| D-7102 | H | single bond | Me | Et | C(=O)N(Me)₂ | Me | Me | O |
| D-7103 | Me | single bond | H | Et | C(=O)N(Me)₂ | Me | Me | O |
| D-7104 | Me(R) | single bond | H | Et | C(=O)N(Me)₂ | Me | Me | O |
| D-7105 | Me(S) | single bond | H | Et | C(=O)N(Me)₂ | Me | Me | O |
| D-7106 | H | CH₂ | Me | Et | C(=O)N(Me)₂ | Me | Me | O |
| D-7107 | Me | CH₂ | H | Et | C(=O)N(Me)₂ | Me | Me | O |
| D-7108 | H | CH(Me) | H | Et | C(=O)N(Me)₂ | Me | Me | O |
| D-7109 | H | CH₂CH₂ | Me | Et | C(=O)N(Me)₂ | Me | Me | O |
| D-7110 | Me | CH₂CH₂ | H | Et | C(=O)N(Me)₂ | Me | Me | O |
| D-7111 | H | single bond | Me | Et | CH₂OC(=O)Me | Cl | Me | O |
| D-7112 | Me | single bond | H | Et | CH₂OC(=O)Me | Cl | Me | O |
| D-7113 | Me(R) | single bond | H | Et | CH₂OC(=O)Me | Cl | Me | O |
| D-7114 | Me(S) | single bond | H | Et | CH₂OC(=O)Me | Cl | Me | O |
| D-7115 | H | CH₂ | Me | Et | CH₂OC(=O)Me | Cl | Me | O |
| D-7116 | Me | CH₂ | H | Et | CH₂OC(=O)Me | Cl | Me | O |
| D-7117 | H | CH(Me) | H | Et | CH₂OC(=O)Me | Cl | Me | O |
| D-7118 | H | CH₂CH₂ | Me | Et | CH₂OC(=O)Me | Cl | Me | O |
| D-7119 | Me | CH₂CH₂ | H | Et | CH₂OC(=O)Me | Cl | Me | O |
| D-7120 | H | single bond | Me | Et | CH₂OC(=O)Me | Me | Me | O |
| D-7121 | Me | single bond | H | Et | CH₂OC(=O)Me | Me | Me | O |
| D-7122 | Me(R) | single bond | H | Et | CH₂OC(=O)Me | Me | Me | O |
| D-7123 | Me(S) | single bond | H | Et | CH₂OC(=O)Me | Me | Me | O |
| D-7124 | H | CH₂ | Me | Et | CH₂OC(=O)Me | Me | Me | O |
| D-7125 | Me | CH₂ | H | Et | CH₂OC(=O)Me | Me | Me | O |
| D-7126 | H | CH(Me) | H | Et | CH₂OC(=O)Me | Me | Me | O |
| D-7127 | H | CH₂CH₂ | Me | Et | CH₂OC(=O)Me | Me | Me | O |
| D-7128 | Me | CH₂CH₂ | H | Et | CH₂OC(=O)Me | Me | Me | O |
| D-7129 | H | single bond | Me | Et | CH₂OC(=O)(*i*-Pr) | Cl | Me | O |
| D-7130 | Me | single bond | H | Et | CH₂OC(=O)(*i*-Pr) | Cl | Me | O |
| D-7131 | Me(R) | single bond | H | Et | CH₂OC(=O)(*i*-Pr) | Cl | Me | O |
| D-7132 | Me(S) | single bond | H | Et | CH₂OC(=O)(*i*-Pr) | Cl | Me | O |
| D-7133 | H | CH₂ | Me | Et | CH₂OC(=O)(*i*-Pr) | Cl | Me | O |
| D-7134 | Me | CH₂ | H | Et | CH₂OC(=O)(*i*-Pr) | Cl | Me | O |

**[Table 808]**

| Compound Number | Y | W | Z | R¹ | R² | R³ | R¹⁰ | A |
|---|---|---|---|---|---|---|---|---|
| D-7135 | H | CH(Me) | H | Et | CH₂OC(=O)(*i*-Pr) | Cl | Me | O |
| D-7136 | H | CH₂CH₂ | Me | Et | CH₂OC(=O)(*i*-Pr) | Cl | Me | O |
| D-7137 | Me | CH₂CH₂ | H | Et | CH₂OC(=O)(*i*-Pr) | Cl | Me | O |
| D-7138 | H | single bond | Me | Et | CH₂OC(=O)(*i*-Pr) | Me | Me | O |
| D-7139 | Me | single bond | H | Et | CH₂OC(=O)(*i*-Pr) | Me | Me | O |
| D-7140 | Me(R) | single bond | H | Et | CH₂OC(=O)(*i*-Pr) | Me | Me | O |
| D-7141 | Me(S) | single bond | H | Et | CH₂OC(=O)(*i*-Pr) | Me | Me | O |
| D-7142 | H | CH₂ | Me | Et | CH₂OC(=O)(*i*-Pr) | Me | Me | O |
| D-7143 | Me | CH₂ | H | Et | CH₂OC(=O)(*i*-Pr) | Me | Me | O |
| D-7144 | H | CH(Me) | H | Et | CH₂OC(=O)(*i*-Pr) | Me | Me | O |
| D-7145 | H | CH₂CH₂ | Me | Et | CH₂OC(=O)(*i*-Pr) | Me | Me | O |
| D-7146 | Me | CH₂CH₂ | H | Et | CH₂OC(=O)(*i*-Pr) | Me | Me | O |
| D-7147 | H | single bond | Me | Et | CH₂OC(=O)(*t*-Bu) | Cl | Me | O |
| D-7148 | Me | single bond | H | Et | CH₂OC(=O)(*t*-Bu) | Cl | Me | O |
| D-7149 | Me(R) | single bond | H | Et | CH₂OC(=O)(*t*-Bu) | Cl | Me | O |
| D-7150 | Me(S) | single bond | H | Et | CH₂OC(=O)(*t*-Bu) | Cl | Me | O |
| D-7151 | H | CH₂ | Me | Et | CH₂OC(=O)(*t*-Bu) | Cl | Me | O |
| D-7152 | Me | CH₂ | H | Et | CH₂OC(=O)(*t*-Bu) | Cl | Me | O |
| D-7153 | H | CH(Me) | H | Et | CH₂OC(=O)(*t*-Bu) | Cl | Me | O |
| D-7154 | H | CH₂CH₂ | Me | Et | CH₂OC(=O)(*t*-Bu) | Cl | Me | O |
| D-7155 | Me | CH₂CH₂ | H | Et | CH₂OC(=O)(*t*-Bu) | Cl | Me | O |
| D-7156 | H | single bond | Me | Et | CH₂OC(=O)(*t*-Bu) | Me | Me | O |
| D-7157 | Me | single bond | H | Et | CH₂OC(=O)(*t*-Bu) | Me | Me | O |
| D-7158 | Me(R) | single bond | H | Et | CH₂OC(=O)(*t*-Bu) | Me | Me | O |
| D-7159 | Me(S) | single bond | H | Et | CH₂OC(=O)(*t*-Bu) | Me | Me | O |
| D-7160 | H | CH₂ | Me | Et | CH₂OC(=O)(*t*-Bu) | Me | Me | O |
| D-7161 | Me | CH₂ | H | Et | CH₂OC(=O)(*t*-Bu) | Me | Me | O |
| D-7162 | H | CH(Me) | H | Et | CH₂OC(=O)(*t*-Bu) | Me | Me | O |
| D-7163 | H | CH₂CH₂ | Me | Et | CH₂OC(=O)(*t*-Bu) | Me | Me | O |
| D-7164 | Me | CH₂CH₂ | H | Et | CH₂OC(=O)(*t*-Bu) | Me | Me | O |
| D-7165 | H | single bond | Me | Et | CH₂OC(=O)OEt | Cl | Me | O |
| D-7166 | Me | single bond | H | Et | CH₂OC(=O)OEt | Cl | Me | O |
| D-7167 | Me(R) | single bond | H | Et | CH₂OC(=O)OEt | Cl | Me | O |
| D-7168 | Me(S) | single bond | H | Et | CH₂OC(=O)OEt | Cl | Me | O |
| D-7169 | H | CH₂ | Me | Et | CH₂OC(=O)OEt | Cl | Me | O |
| D-7170 | Me | CH₂ | H | Et | CH₂OC(=O)OEt | Cl | Me | O |
| D-7171 | H | CH(Me) | H | Et | CH₂OC(=O)OEt | Cl | Me | O |
| D-7172 | H | CH₂CH₂ | Me | Et | CH₂OC(=O)OEt | Cl | Me | O |
| D-7173 | Me | CH₂CH₂ | H | Et | CH₂OC(=O)OEt | Cl | Me | O |
| D-7174 | H | single bond | Me | Et | CH₂OC(=O)OEt | Me | Me | O |
| D-7175 | Me | single bond | H | Et | CH₂OC(=O)OEt | Me | Me | O |
| D-7176 | Me(R) | single bond | H | Et | CH₂OC(=O)OEt | Me | Me | O |
| D-7177 | Me(S) | single bond | H | Et | CH₂OC(=O)OEt | Me | Me | O |
| D-7178 | H | CH₂ | Me | Et | CH₂OC(=O)OEt | Me | Me | O |

**[Table 809]**

| Compound Number | Y | W | Z | R¹ | R² | R³ | R¹⁰ | A |
|---|---|---|---|---|---|---|---|---|
| D-7179 | Me | CH₂ | H | Et | CH₂OC(=O)OEt | Me | Me | O |
| D-7180 | H | CH(Me) | H | Et | CH₂OC(=O)OEt | Me | Me | O |
| D-7181 | H | CH₂CH₂ | Me | Et | CH₂OC(=O)OEt | Me | Me | O |
| D-7182 | Me | CH₂CH₂ | H | Et | CH₂OC(=O)OEt | Me | Me | O |
| D-7183 | H | single bond | Me | Et | CH₂OC(=O)O(*i*-Pr) | Cl | Me | O |
| D-7184 | Me | single bond | H | Et | CH₂OC(=O)O(*i*-Pr) | Cl | Me | O |
| D-7185 | Me(R) | single bond | H | Et | CH₂OC(=O)O(*i*-Pr) | Cl | Me | O |
| D-7186 | Me(S) | single bond | H | Et | CH₂OC(=O)O(*i*-Pr) | Cl | Me | O |
| D-7187 | H | CH₂ | Me | Et | CH₂OC(=O)O(*i*-Pr) | Cl | Me | O |
| D-7188 | Me | CH₂ | H | Et | CH₂OC(=O)O(*i*-Pr) | Cl | Me | O |
| D-7189 | H | CH(Me) | H | Et | CH₂OC(=O)O(*i*-Pr) | Cl | Me | O |
| D-7190 | H | CH₂CH₂ | Me | Et | CH₂OC(=O)O(*i*-Pr) | Cl | Me | O |
| D-7191 | Me | CH₂CH₂ | H | Et | CH₂OC(=O)O(*i*-Pr) | Cl | Me | O |
| D-7192 | H | single bond | Me | Et | CH₂OC(=O)O(*i*-Pr) | Me | Me | O |
| D-7193 | Me | single bond | H | Et | CH₂OC(=O)O(*i*-Pr) | Me | Me | O |
| D-7194 | Me(R) | single bond | H | Et | CH₂OC(=O)O(*i*-Pr) | Me | Me | O |
| D-7195 | Me(S) | single bond | H | Et | CH₂OC(=O)O(*i*-Pr) | Me | Me | O |
| D-7196 | H | CH₂ | Me | Et | CH₂OC(=O)O(*i*-Pr) | Me | Me | O |
| D-7197 | Me | CH₂ | H | Et | CH₂OC(=O)O(*i*-Pr) | Me | Me | O |
| D-7198 | H | CH(Me) | H | Et | CH₂OC(=O)O(*i*-Pr) | Me | Me | O |
| D-7199 | H | CH₂CH₂ | Me | Et | CH₂OC(=O)O(*i*-Pr) | Me | Me | O |
| D-7200 | Me | CH₂CH₂ | H | Et | CH₂OC(=O)O(*i*-Pr) | Me | Me | O |
| D-7201 | H | single bond | Me | Et | CH₂N(Me)C(=O)OMe | Cl | Me | O |
| D-7202 | Me | single bond | H | Et | CH₂N(Me)C(=O)OMe | Cl | Me | O |
| D-7203 | Me(R) | single bond | H | Et | CH₂N(Me)C(=O)OMe | Cl | Me | O |
| D-7204 | Me(S) | single bond | H | Et | CH₂N(Me)C(=O)OMe | Cl | Me | O |
| 0-7205 | H | CH₂ | Me | Et | CH₂N(Me)C(=O)OMe | Cl | Me | O |
| D-7206 | Me | CH₂ | H | Et | CH₂N(Me)C(=O)OMe | Cl | Me | O |
| D-7207 | H | CH(Me) | H | Et | CH₂N(Me)C(=O)OMe | Cl | Me | O |
| D-7208 | H | CH₂CH₂ | Me | Et | CH₂N(Me)C(=O)OMe | Cl | Me | O |
| D-7209 | Me | CH₂CH₂ | H | Et | CH₂N(Me)C(=O)OMe | Cl | Me | O |
| D-7210 | H | single bond | Me | Et | CH₂N(Me)C(=O)OMe | Me | Me | O |
| D-7211 | Me | single bond | H | Et | CH₂N(Me)C(=O)OMe | Me | Me | O |
| D-7212 | Me(R) | single bond | H | Et | CH₂N(Me)C(=O)OMe | Me | Me | O |
| D-7213 | Me(S) | single bond | H | Et | CH₂N(Me)C(=O)OMe | Me | Me | O |
| D-7214 | H | CH₂ | Me | Et | CH₂N(Me)C(=O)OMe | Me | Me | O |
| D-7215 | Me | CH₂ | H | Et | CH₂N(Me)C(=O)OMe | Me | Me | O |
| D-7216 | H | CH(Me) | H | Et | CH₂N(Me)C(=O)OMe | Me | Me | O |
| D-7217 | H | CH₂CH₂ | Me | Et | CH₂N(Me)C(=O)OMe | Me | Me | O |
| D-7218 | Me | CH₂CH₂ | H | Et | CH₂N(Me)C(=O)OMe | Me | Me | O |
| D-7219 | H | single bond | Me | Et | C(=O)OCH₂CH=CH₂ | Cl | CH₂OMe | O |
| D-7220 | Me | single bond | H | Et | C(=O)OCH₂CH=CH₂ | Cl | CH₂OMe | O |
| D-7221 | Me(R) | single bond | H | Et | C(=O)OCH₂CH=CH₂ | Cl | CH₂OMe | O |
| D-7222 | Me(S) | single bond | H | Et | C(=O)OCH₂CH=CH₂ | Cl | CH₂OMe | O |

**[Table 810]**

| Compound Number | Y | W | Z | R¹ | R² | R³ | R¹⁰ | A |
|---|---|---|---|---|---|---|---|---|
| D-7223 | H | CH₂ | Me | Et | C(=O)OCH₂CH=CH₂ | Cl | CH₂OMe | O |
| D-7224 | Me | CH₂ | H | Et | C(=O)OCH₂CH=CH₂ | Cl | CH₂OMe | O |
| D-7225 | H | CH(Me) | H | Et | C(=O)OCH₂CH=CH₂ | Cl | CH₂OMe | O |
| D-7226 | H | CH₂CH₂ | Me | Et | C(=O)OCH₂CH=CH₂ | Cl | CH₂OMe | O |
| D-7227 | Me | CH₂CH₂ | H | Et | C(=O)OCH₂CH=CH₂ | Cl | CH₂OMe | O |
| D-7228 | H | single bond | Me | Et | C(=O)OCH₂CH=CH₂ | Me | CH₂OMe | O |
| D-7229 | Me | single bond | H | Et | C(=O)OCH₂CH=CH₂ | Me | CH₂OMe | O |
| D-7230 | Me(R) | single bond | H | Et | C(=O)OCH₂CH=CH₂ | Me | CH₂OMe | O |
| D-7231 | Me(S) | single bond | H | Et | C(=O)OCH₂CH=CH₂ | Me | CH₂OMe | O |
| D-7232 | H | CH₂ | Me | Et | C(=O)OCH₂CH=CH₂ | Me | CH₂OMe | O |
| D-7233 | Me | CH₂ | H | Et | C(=O)OCH₂CH=CH₂ | Me | CH₂OMe | O |
| D-7234 | H | CH(Me) | H | Et | C(=O)OCH₂CH=CH₂ | Me | CH₂OMe | O |
| D-7235 | H | CH₂CH₂ | Me | Et | C(=O)OCH₂CH=CH₂ | Me | CH₂OMe | O |
| D-7236 | Me | CH₂CH₂ | H | Et | C(=O)OCH₂CH=CH₂ | Me | CH₂OMe | O |
| D-7237 | H | single bond | Me | Et | C(=O)OCH₂CCl₃ | Cl | CH₂OMe | O |
| D-7238 | Me | single bond | H | Et | C(=O)OCH₂CCl₃ | Cl | CH₂OMe | O |
| D-7239 | Me(R) | single bond | H | Et | C(=O)OCH₂CCl₃ | Cl | CH₂OMe | O |
| D-7240 | Me(S) | single bond | H | Et | C(=O)OCH₂CCl₃ | Cl | CH₂OMe | O |
| D-7241 | H | CH₂ | Me | Et | C(=O)OCH₂CCl₃ | Cl | CH₂OMe | O |
| D-7242 | Me | CH₂ | H | Et | C(=O)OCH₂CCl₃ | Cl | CH₂OMe | O |
| D-7243 | H | CH(Me) | H | Et | C(=O)OCH₂CCl₃ | Cl | CH₂OMe | O |
| D-7244 | H | CH₂CH₂ | Me | Et | C(=O)OCH₂CCl₃ | Cl | CH₂OMe | O |
| D-7245 | Me | CH₂CH₂ | H | Et | C(=O)OCH₂CCl₃ | Cl | CH₂OMe | O |
| D-7246 | H | single bond | Me | Et | C(=O)OCH₂CCl₃ | Me | CH₂OMe | O |
| D-7247 | Me | single bond | H | Et | C(=O)OCH₂CCl₃ | Me | CH₂OMe | O |
| D-7248 | Me(R) | single bond | H | Et | C(=O)OCH₂CCl₃ | Me | CH₂OMe | O |
| D-7249 | Me(S) | single bond | H | Et | C(=O)OCH₂CCl₃ | Me | CH₂OMe | O |
| D-7250 | H | CH₂ | Me | Et | C(=O)OCH₂CCl₃ | Me | CH₂OMe | O |
| D-7251 | Me | CH₂ | H | Et | C(=O)OCH₂CCl₃ | Me | CH₂OMe | O |
| D-7252 | H | CH(Me) | H | Et | C(=O)OCH₂CCl₃ | Me | CH₂OMe | O |
| D-7253 | H | CH₂CH₂ | Me | Et | C(=O)OCH₂CCl₃ | Me | CH₂OMe | O |
| D-7254 | Me | CH₂CH₂ | H | Et | C(=O)OCH₂CCl₃ | Me | CH₂OMe | O |
| D-7255 | H | single bond | Me | Et | C(=O)N(Me)₂ | Cl | CH₂OMe | O |
| D-7256 | Me | single bond | H | Et | C(=O)N(Me)₂ | Cl | CH₂OMe | O |
| D-7257 | Me(R) | single bond | H | Et | C(=O)N(Me)₂ | Cl | CH₂OMe | O |
| D-7258 | Me(S) | single bond | H | Et | C(=O)N(Me)₂ | Cl | CH₂OMe | O |
| D-7259 | H | CH₂ | Me | Et | C(=O)N(Me)₂ | Cl | CH₂OMe | O |
| D-7260 | Me | CH₂ | H | Et | C(=O)N(Me)₂ | Cl | CH₂OMe | O |
| D-7261 | H | CH(Me) | H | Et | C(=O)N(Me)₂ | Cl | CH₂OMe | O |
| D-7262 | H | CH₂CH₂ | Me | Et | C(=O)N(Me)₂ | Cl | CH₂OMe | O |
| D-7263 | Me | CH₂CH₂ | H | Et | C(=O)N(Me)₂ | Cl | CH₂OMe | O |
| D-7264 | H | single bond | Me | Et | C(=O)N(Me)₂ | Me | CH₂OMe | O |
| D-7265 | Me | single bond | H | Et | C(=O)N(Me)₂ | Me | CH₂OMe | O |
| D-7266 | Me(R) | single bond | H | Et | C(=O)N(Me)₂ | Me | CH₂OMe | O |

**[Table 811]**

| Compound Number | Y | W | Z | R¹ | R² | R³ | R¹⁰ | A |
|---|---|---|---|---|---|---|---|---|
| D-7267 | Me(S) | single bond | H | Et | C(=O)N(Me)₂ | Me | CH₂OMe | O |
| D-7268 | H | CH₂ | Me | Et | C(=O)N(Me)₂ | Me | CH₂OMe | O |
| D-7269 | Me | CH₂ | H | Et | C(=O)N(Me)₂ | Me | CH₂OMe | O |
| D-7270 | H | CH(Me) | H | Et | C(=O)N(Me)₂ | Me | CH₂OMe | O |
| D-7271 | H | CH₂CH₂ | Me | Et | C(=O)N(Me)₂ | Me | CH₂OMe | O |
| D-7272 | Me | CH₂CH₂ | H | Et | C(=O)N(Me)₂ | Me | CH₂OMe | O |
| D-7273 | H | single bond | Me | Et | CH₂OC(=O)Me | Cl | CH₂OMe | O |
| D-7274 | Me | single bond | H | Et | CH₂OC(=O)Me | Cl | CH₂OMe | O |
| D-7275 | Me(R) | single bond | H | Et | CH₂OC(=O)Me | Cl | CH₂OMe | O |
| D-7276 | Me(S) | single bond | H | Et | CH₂OC(=O)Me | Cl | CH₂OMe | O |
| D-7277 | H | CH₂ | Me | Et | CH₂OC(=O)Me | Cl | CH₂OMe | O |
| D-7278 | Me | CH₂ | H | Et | CH₂OC(=O)Me | Cl | CH₂OMe | O |
| D-7279 | H | CH(Me) | H | Et | CH₂OC(=O)Me | Cl | CH₂OMe | O |
| D-7280 | H | CH₂CH₂ | Me | Et | CH₂OC(=O)Me | Cl | CH₂OMe | O |
| D-7281 | Me | CH₂CH₂ | H | Et | CH₂OC(=O)Me | Cl | CH₂OMe | O |
| D-7282 | H | single bond | Me | Et | CH₂OC(=O)Me | Me | CH₂OMe | O |
| D-7283 | Me | single bond | H | Et | CH₂OC(=O)Me | Me | CH₂OMe | O |
| D-7284 | Me(R) | single bond | H | Et | CH₂OC(=O)Me | Me | CH₂OMe | O |
| D-7285 | Me(S) | single bond | H | Et | CH₂OC(=O)Me | Me | CH₂OMe | O |
| D-7286 | H | CH₂ | Me | Et | CH₂OC(=O)Me | Me | CH₂OMe | O |
| D-7287 | Me | CH₂ | H | Et | CH₂OC(=O)Me | Me | CH₂OMe | O |
| D-7288 | H | CH(Me) | H | Et | CH₂OC(=O)Me | Me | CH₂OMe | O |
| D-7289 | H | CH₂CH₂ | Me | Et | CH₂OC(=O)Me | Me | CH₂OMe | O |
| D-7290 | Me | CH₂CH₂ | H | Et | CH₂OC(=O)Me | Me | CH₂OMe | O |
| D-7291 | H | single bond | Me | Et | CH₂OC(=O)(*i*-Pr) | Cl | CH₂OMe | O |
| D-7292 | Me | single bond | H | Et | CH₂OC(=O)(*i*-Pr) | Cl | CH₂OMe | O |
| D-7293 | Me(R) | single bond | H | Et | CH₂OC(=O)(*i*-Pr) | Cl | CH₂OMe | O |
| D-7294 | Me(S) | single bond | H | Et | CH₂OC(=O)(*i*-Pr) | Cl | CH₂OMe | O |
| D-7295 | H | CH₂ | Me | Et | CH₂OC(=O)(*i*-Pr) | Cl | CH₂OMe | O |
| D-7296 | Me | CH₂ | H | Et | CH₂OC(=O)(*i*-Pr) | Cl | CH₂OMe | O |
| D-7297 | H | CH(Me) | H | Et | CH₂OC(=O)(*i*-Pr) | Cl | CH₂OMe | O |
| D-7298 | H | CH₂CH₂ | Me | Et | CH₂OC(=O)(*i*-Pr) | Cl | CH₂OMe | O |
| D-7299 | Me | CH₂CH₂ | H | Et | CH₂OC(=O)(*i*-Pr) | Cl | CH₂OMe | O |
| D-7300 | H | single bond | Me | Et | CH₂OC(=O)(*i*-Pr) | Me | CH₂OMe | O |
| D-7301 | Me | single bond | H | Et | CH₂OC(=O)(*i*-Pr) | Me | CH₂OMe | O |
| D-7302 | Me(R) | single bond | H | Et | CH₂OC(=O)(*i*-Pr) | Me | CH₂OMe | O |
| D-7303 | Me(S) | single bond | H | Et | CH₂OC(=O)(*i*-Pr) | Me | CH₂OMe | O |
| D-7304 | H | CH₂ | Me | Et | CH₂OC(=O)(*i*-Pr) | Me | CH₂OMe | O |
| D-7305 | Me | CH₂ | H | Et | CH₂OC(=O)(*i*-Pr) | Me | CH₂OMe | O |
| D-7306 | H | CH(Me) | H | Et | CH₂OC(=O)(*i*-Pr) | Me | CH₂OMe | O |
| D-7307 | H | CH₂CH₂ | Me | Et | CH₂OC(=O)(*i*-Pr) | Me | CH₂OMe | O |
| D-7308 | Me | CH₂CH₂ | H | Et | CH₂OC(=O)(*i*-Pr) | Me | CH₂OMe | O |
| 0-7309 | H | single bond | Me | Et | CH₂OC(=O)(*t*-Bu) | Cl | CH₂OMe | O |
| D-7310 | Me | single bond | H | Et | CH₂OC(=O)(*t*-Bu) | Cl | CH₂OMe | O |

**[Table 812]**

| Compound Number | Y | W | Z | R¹ | R² | R³ | R¹⁰ | A |
|---|---|---|---|---|---|---|---|---|
| D-7311 | Me(R) | single bond | H | Et | CH₂OC(=O)(*t*-Bu) | Cl | CH₂OMe | O |
| D-7312 | Me(S) | single bond | H | Et | CH₂OC(=O)(*t*-Bu) | Cl | CH₂OMe | O |
| D-7313 | H | CH₂ | Me | Et | CH₂OC(=O)(*t*-Bu) | Cl | CH₂OMe | O |
| D-7314 | Me | CH₂ | H | Et | CH₂OC(=O)(*t*-Bu) | Cl | CH₂OMe | O |
| D-7315 | H | CH(Me) | H | Et | CH₂OC(=O)(*t*-Bu) | Cl | CH₂OMe | O |
| D-7316 | H | CH₂CH₂ | Me | Et | CH₂OC(=O)(*t*-Bu) | Cl | CH₂OMe | O |
| D-7317 | Me | CH₂CH₂ | H | Et | CH₂OC(=O)(*t*-Bu) | Cl | CH₂OMe | O |
| D-7318 | H | single bond | Me | Et | CH₂OC(=O)(*t*-Bu) | Me | CH₂OMe | O |
| D-7319 | Me | single bond | H | Et | CH₂OC(=O)(*t*-Bu) | Me | CH₂OMe | O |
| D-7320 | Me(R) | single bond | H | Et | CH₂OC(=O)(*t*-Bu) | Me | CH₂OMe | O |
| D-7321 | Me(S) | single bond | H | Et | CH₂OC(=O)(*t*-Bu) | Me | CH₂OMe | O |
| D-7322 | H | CH₂ | Me | Et | CH₂OC(=O)(*t*-Bu) | Me | CH₂OMe | O |
| D-7323 | Me | CH₂ | H | Et | CH₂OC(=O)(*t*-Bu) | Me | CH₂OMe | O |
| D-7324 | H | CH(Me) | H | Et | CH₂OC(=O)(*t*-Bu) | Me | CH₂OMe | O |
| D-7325 | H | CH₂CH₂ | Me | Et | CH₂OC(=O)(*t*-Bu) | Me | CH₂OMe | O |
| D-7326 | Me | CH₂CH₂ | H | Et | CH₂OC(=O)(*t*-Bu) | Me | CH₂OMe | O |
| D-7327 | H | single bond | Me | Et | CH₂OC(=O)OEt | Cl | CH₂OMe | O |
| D-7328 | Me | single bond | H | Et | CH₂OC(=O)OEt | Cl | CH₂OMe | O |
| D-7329 | Me(R) | single bond | H | Et | CH₂OC(=O)OEt | Cl | CH₂OMe | O |
| D-7330 | Me(S) | single bond | H | Et | CH₂OC(=O)OEt | Cl | CH₂OMe | O |
| D-7331 | H | CH₂ | Me | Et | CH₂OC(=O)OEt | Cl | CH₂OMe | O |
| D-7332 | Me | CH₂ | H | Et | CH₂OC(=O)OEt | Cl | CH₂OMe | O |
| D-7333 | H | CH(Me) | H | Et | CH₂OC(=O)OEt | Cl | CH₂OMe | O |
| D-7334 | H | CH₂CH₂ | Me | Et | CH₂OC(=O)OEt | Cl | CH₂OMe | O |
| D-7335 | Me | CH₂CH₂ | H | Et | CH₂OC(=O)OEt | Cl | CH₂OMe | O |
| D-7336 | H | single bond | Me | Et | CH₂OC(=O)OEt | Me | CH₂OMe | O |
| D-7337 | Me | single bond | H | Et | CH₂OC(=O)OEt | Me | CH₂OMe | O |
| D-7338 | Me(R) | single bond | H | Et | CH₂OC(=O)OEt | Me | CH₂OMe | O |
| D-7339 | Me(S) | single bond | H | Et | CH₂OC(=O)OEt | Me | CH₂OMe | O |
| D-7340 | H | CH₂ | Me | Et | CH₂OC(=O)OEt | Me | CH₂OMe | O |
| D-7341 | Me | CH₂ | H | Et | CH₂OC(=O)OEt | Me | CH₂OMe | O |
| D-7342 | H | CH(Me) | H | Et | CH₂OC(=O)OEt | Me | CH₂OMe | O |
| D-7343 | H | CH₂CH₂ | Me | Et | CH₂OC(=O)OEt | Me | CH₂OMe | O |
| D-7344 | Me | CH₂CH₂ | H | Et | CH₂OC(=O)OEt | Me | CH₂OMe | O |
| D-7345 | H | single bond | Me | Et | CH₂OC(=O)O(*i*-Pr) | Cl | CH₂OMe | O |
| D-7346 | Me | single bond | H | Et | CH₂OC(=O)O(*i*-Pr) | Cl | CH₂OMe | O |
| D-7347 | Me(R) | single bond | H | Et | CH₂OC(=O)O(*i*-Pr) | Cl | CH₂OMe | O |
| D-7348 | Me(S) | single bond | H | Et | CH₂OC(=O)O(*i*-Pr) | Cl | CH₂OMe | O |
| D-7349 | H | CH₂ | Me | Et | CH₂OC(=O)O(*i*-Pr) | Cl | CH₂OMe | O |
| D-7350 | Me | CH₂ | H | Et | CH₂OC(=O)O(*i*-Pr) | Cl | CH₂OMe | O |
| D-7351 | H | CH(Me) | H | Et | CH₂OC(=O)O(*i*-Pr) | Cl | CH₂OMe | O |
| D-7352 | H | CH₂CH₂ | Me | Et | CH₂OC(=O)O(*i*-Pr) | Cl | CH₂OMe | O |
| D-7353 | Me | CH₂CH₂ | H | Et | CH₂OC(=O)O(*i*-Pr) | Cl | CH₂OMe | O |
| D-7354 | H | single bond | Me | Et | CH₂OC(=O)O(*i*-Pr) | Me | CH₂OMe | O |

**[Table 813]**

| Compound Number | Y | W | Z | R¹ | R² | R³ | R¹⁰ | A |
|---|---|---|---|---|---|---|---|---|
| D-7355 | Me | single bond | H | Et | CH₂OC(=O)O(*i*-Pr) | Me | CH₂OMe | O |
| D-7356 | Me(R) | single bond | H | Et | CH₂OC(=O)O(*i*-Pr) | Me | CH₂OMe | O |
| D-7357 | Me(S) | single bond | H | Et | CH₂OC(=O)O(*i*-Pr) | Me | CH₂OMe | O |
| D-7358 | H | CH₂ | Me | Et | CH₂OC(=O)O(*i*-Pr) | Me | CH₂OMe | O |
| D-7359 | Me | CH₂ | H | Et | CH₂OC(=O)O(*i*-Pr) | Me | CH₂OMe | O |
| D-7360 | H | CH(Me) | H | Et | CH₂OC(=O)O(*i*-Pr) | Me | CH₂OMe | O |
| D-7361 | H | CH₂CH₂ | Me | Et | CH₂OC(=O)O(*i*-Pr) | Me | CH₂OMe | O |
| D-7362 | Me | CH₂CH₂ | H | Et | CH₂OC(=O)O(*i*-Pr) | Me | CH₂OMe | O |
| D-7363 | H | single bond | Me | Et | CH₂N(Me)C(=O)OMe | Cl | CH₂OMe | O |
| D-7364 | Me | single bond | H | Et | CH₂N(Me)C(=O)OMe | Cl | CH₂OMe | O |
| D-7365 | Me(R) | single bond | H | Et | CH₂N(Me)C(=O)OMe | Cl | CH₂OMe | O |
| D-7366 | Me(S) | single bond | H | Et | CH₂N(Me)C(=O)OMe | Cl | CH₂OMe | O |
| D-7367 | H | CH₂ | Me | Et | CH₂N(Me)C(=O)OMe | Cl | CH₂OMe | O |
| D-7368 | Me | CH₂ | H | Et | CH₂N(Me)C(=O)OMe | Cl | CH₂OMe | O |
| D-7369 | H | CH(Me) | H | Et | CH₂N(Me)C(=O)OMe | Cl | CH₂OMe | O |
| D-7370 | H | CH₂CH₂ | Me | Et | CH₂N(Me)C(=O)OMe | Cl | CH₂OMe | O |
| D-7371 | Me | CH₂CH₂ | H | Et | CH₂N(Me)C(=O)OMe | Cl | CH₂OMe | O |
| D-7372 | H | single bond | Me | Et | CH₂N(Me)C(=O)OMe | Me | CH₂OMe | O |
| D-7373 | Me | single bond | H | Et | CH₂N(Me)C(=O)OMe | Me | CH₂OMe | O |
| D-7374 | Me(R) | single bond | H | Et | CH₂N(Me)C(=O)OMe | Me | CH₂OMe | O |
| D-7375 | Me(S) | single bond | H | Et | CH₂N(Me)C(=O)OMe | Me | CH₂OMe | O |
| D-7376 | H | CH₂ | Me | Et | CH₂N(Me)C(=O)OMe | Me | CH₂OMe | O |
| D-7377 | Me | CH₂ | H | Et | CH₂N(Me)C(=O)OMe | Me | CH₂OMe | O |
| D-7378 | H | CH(Me) | H | Et | CH₂N(Me)C(=O)OMe | Me | CH₂OMe | O |
| D-7379 | H | CH₂CH₂ | Me | Et | CH₂N(Me)C(=O)OMe | Me | CH₂OMe | O |
| D-7380 | Me | CH₂CH₂ | H | Et | CH₂N(Me)C(=O)OMe | Me | CH₂OMe | O |
| D-7381 | H | single bond | Me | Et | C(=O)OCH₂CH=CH₂ | Cl | CH₂CN | O |
| D-7382 | Me | single bond | H | Et | C(=O)OCH₂CH=CH₂ | Cl | CH₂CN | O |
| D-7383 | Me(R) | single bond | H | Et | C(=O)OCH₂CH=CH₂ | Cl | CH₂CN | O |
| D-7384 | Me(S) | single bond | H | Et | C(=O)OCH₂CH=CH₂ | Cl | CH₂CN | O |
| D-7385 | H | CH₂ | Me | Et | C(=O)OCH₂CH=CH₂ | Cl | CH₂CN | O |
| D-7386 | Me | CH₂ | H | Et | C(=O)OCH₂CH=CH₂ | Cl | CH₂CN | O |
| D-7387 | H | CH(Me) | H | Et | C(=O)OCH₂CH=CH₂ | Cl | CH₂CN | O |
| D-7388 | H | CH₂CH₂ | Me | Et | C(=O)OCH₂CH=CH₂ | Cl | CH₂CN | O |
| D-7389 | Me | CH₂CH₂ | H | Et | C(=O)OCH₂CH=CH₂ | Cl | CH₂CN | O |
| D-7390 | H | single bond | Me | Et | C(=O)OCH₂CH=CH₂ | Me | CH₂CN | O |
| D-7391 | Me | single bond | H | Et | C(=O)OCH₂CH=CH₂ | Me | CH₂CN | O |
| D-7392 | Me(R) | single bond | H | Et | C(=O)OCH₂CH=CH₂ | Me | CH₂CN | O |
| D-7393 | Me(S) | single bond | H | Et | C(=O)OCH₂CH=CH₂ | Me | CH₂CN | O |
| D-7394 | H | CH₂ | Me | Et | C(=O)OCH₂CH=CH₂ | Me | CH₂CN | O |
| D-7395 | Me | CH₂ | H | Et | C(=O)OCH₂CH=CH₂ | Me | CH₂CN | O |
| D-7396 | H | CH(Me) | H | Et | C(=O)OCH₂CH=CH₂ | Me | CH₂CN | O |
| D-7397 | H | CH₂CH₂ | Me | Et | C(=O)OCH₂CH=CH₂ | Me | CH₂CN | O |
| D-7398 | Me | CH₂CH₂ | H | Et | C(=O)OCH₂CH=CH₂ | Me | CH₂CN | O |

**[Table 814]**

| Compound Number | Y | W | z | R¹ | R² | R³ | R¹⁰ | A |
|---|---|---|---|---|---|---|---|---|
| D-7399 | H | single bond | Me | Et | C(=O)OCH₂CCl₃ | Cl | CH₂CN | O |
| D-7400 | Me | single bond | H | Et | C(=O)OCH₂CCl₃ | Cl | CH₂CN | O |
| D-7401 | Me(R) | single bond | H | Et | C(=O)OCH₂CCl₃ | Cl | CH₂CN | O |
| D-7402 | Me(S) | single bond | H | Et | C(=O)OCH₂CCl₃ | Cl | CH₂CN | O |
| D-7403 | H | CH₂ | Me | Et | C(=O)OCH₂CCl₃ | Cl | CH₂CN | O |
| D-7404 | Me | CH₂ | H | Et | C(=O)OCH₂CCl₃ | Cl | CH₂CN | O |
| D-7405 | H | CH(Me) | H | Et | C(=O)OCH₂CCl₃ | Cl | CH₂CN | O |
| D-7406 | H | CH₂CH₂ | Me | Et | C(=O)OCH₂CCl₃ | Cl | CH₂CN | O |
| D-7407 | Me | CH₂CH₂ | H | Et | C(=O)OCH₂CCl₃ | Cl | CH₂CN | O |
| 0-7408 | H | single bond | Me | Et | C(=O)OCH₂CCl₃ | Me | CH₂CN | O |
| D-7409 | Me | single bond | H | Et | C(=O)OCH₂CCl₃ | Me | CH₂CN | O |
| D-7410 | Me(R) | single bond | H | Et | C(=O)OCH₂CCl₃ | Me | CH₂CN | O |
| D-7411 | Me(S) | single bond | H | Et | C(=O)OCH₂CCl₃ | Me | CH₂CN | O |
| D-7412 | H | CH₂ | Me | Et | C(=O)OCH₂CCl₃ | Me | CH₂CN | O |
| D-7413 | Me | CH₂ | H | Et | C(=O)OCH₂CCl₃ | Me | CH₂CN | O |
| D-7414 | H | CH(Me) | H | Et | C(=O)OCH₂CCl₃ | Me | CH₂CN | O |
| D-7415 | H | CH₂CH₂ | Me | Et | C(=O)OCH₂CCl₃ | Me | CH₂CN | O |
| D-7416 | Me | CH₂CH₂ | H | Et | C(=O)OCH₂CCl₃ | Me | CH₂CN | O |
| D-7417 | H | single bond | Me | Et | C(=O)N(Me)₂ | Cl | CH₂CN | O |
| D-7418 | Me | single bond | H | Et | C(=O)N(Me)₂ | Cl | CH₂CN | O |
| D-7419 | Me(R) | single bond | H | Et | C(=O)N(Me)₂ | Cl | CH₂CN | O |
| D-7420 | Me(S) | single bond | H | Et | C(=O)N(Me)₂ | Cl | CH₂CN | O |
| D-7421 | H | CH₂ | Me | Et | C(=O)N(Me)₂ | Cl | CH₂CN | O |
| D-7422 | Me | CH₂ | H | Et | C(=O)N(Me)₂ | Cl | CH₂CN | O |
| D-7423 | H | CH(Me) | H | Et | C(=O)N(Me)₂ | Cl | CH₂CN | O |
| D-7424 | H | CH₂CH₂ | Me | Et | C(=O)N(Me)₂ | Cl | CH₂CN | O |
| D-7425 | Me | CH₂CH₂ | H | Et | C(=O)N(Me)₂ | Cl | CH₂CN | O |
| D-7426 | H | single bond | Me | Et | C(=O)N(Me)₂ | Me | CH₂CN | O |
| D-7427 | Me | single bond | H | Et | C(=O)N(Me)₂ | Me | CH₂CN | O |
| D-7428 | Me(R) | single bond | H | Et | C(=O)N(Me)₂ | Me | CH₂CN | O |
| D-7429 | Me(S) | single bond | H | Et | C(=O)N(Me)₂ | Me | CH₂CN | O |
| D-7430 | H | CH₂ | Me | Et | C(=O)N(Me)₂ | Me | CH₂CN | O |
| D-7431 | Me | CH₂ | H | Et | C(=O)N(Me)₂ | Me | CH₂CN | O |
| D-7432 | H | CH(Me) | H | Et | C(=O)N(Me)₂ | Me | CH₂CN | O |
| D-7433 | H | CH₂CH₂ | Me | Et | C(=O)N(Me)₂ | Me | CH₂CN | O |
| D-7434 | Me | CH₂CH₂ | H | Et | C(=O)N(Me)₂ | Me | CH₂CN | O |
| D-7435 | H | single bond | Me | Et | CH₂OC(=O)Me | Cl | CH₂CN | O |
| D-7436 | Me | single bond | H | Et | CH₂OC(=O)Me | Cl | CH₂CN | O |
| D-7437 | Me(R) | single bond | H | Et | CH₂OC(=O)Me | Cl | CH₂CN | O |
| D-7438 | Me(S) | single bond | H | Et | CH₂OC(=O)Me | Cl | CH₂CN | O |
| D-7439 | H | CH₂ | Me | Et | CH₂OC(=O)Me | Cl | CH₂CN | O |
| D-7440 | Me | CH₂ | H | Et | CH₂OC(=O)Me | Cl | CH₂CN | O |
| D-7441 | H | CH(Me) | H | Et | CH₂OC(=O)Me | Cl | CH₂CN | O |
| D-7442 | H | CH₂CH₂ | Me | Et | CH₂OC(=O)Me | Cl | CH₂CN | O |

**[Table 815]**

| Compound Number | Y | W | z | R¹ | R² | R³ | R¹⁰ | A |
|---|---|---|---|---|---|---|---|---|
| D-7443 | Me | CH₂CH₂ | H | Et | CH₂OC(=O)Me | Cl | CH₂CN | O |
| D-7444 | H | single bond | Me | Et | CH₂OC(=O)Me | Me | CH₂CN | O |
| D-7445 | Me | single bond | H | Et | CH₂OC(=O)Me | Me | CH₂CN | O |
| D-7446 | Me(R) | single bond | H | Et | CH₂OC(=O)Me | Me | CH₂CN | O |
| D-7447 | Me(S) | single bond | H | Et | CH₂OC(=O)Me | Me | CH₂CN | O |
| D-7448 | H | CH₂ | Me | Et | CH₂OC(=O)Me | Me | CH₂CN | O |
| D-7449 | Me | CH₂ | H | Et | CH₂OC(=O)Me | Me | CH₂CN | O |
| D-7450 | H | CH(Me) | H | Et | CH₂OC(=O)Me | Me | CH₂CN | O |
| D-7451 | H | CH₂CH₂ | Me | Et | CH₂OC(=O)Me | Me | CH₂CN | O |
| D-7452 | Me | CH₂CH₂ | H | Et | CH₂OC(=O)Me | Me | CH₂CN | O |
| D-7453 | H | single bond | Me | Et | CH₂OC(=O)(*i*-Pr) | Cl | CH₂CN | O |
| D-7454 | Me | single bond | H | Et | CH₂OC(=O)(*i* -Pr) | Cl | CH₂CN | O |
| D-7455 | Me(R) | single bond | H | Et | CH₂OC(=O)(*i*-Pr) | Cl | CH₂CN | O |
| D-7456 | Me(S) | single bond | H | Et | CH₂OC(=O)(*i*-Pr) | Cl | CH₂CN | O |
| D-7457 | H | CH₂ | Me | Et | CH₂OC(=O)(*i*-Pr) | Cl | CH₂CN | O |
| D-7458 | Me | CH₂ | H | Et | CH₂OC(=O)(*i*-Pr) | Cl | CH₂CN | O |
| D-7459 | H | CH(Me) | H | Et | CH₂OC(=O)(*i*-Pr) | Cl | CH₂CN | O |
| D-7460 | H | CH₂CH₂ | Me | Et | CH₂OC(=O)(*i*-Pr) | Cl | CH₂CN | O |
| D-7461 | Me | CH₂CH₂ | H | Et | CH₂OC(=O)(*i*-Pr) | Cl | CH₂CN | O |
| D-7462 | H | single bond | Me | Et | CH₂OC(=O)(*i*-Pr) | Me | CH₂CN | O |
| D-7463 | Me | single bond | H | Et | CH₂OC(=O)(*i*-Pr) | Me | CH₂CN | O |
| D-7464 | Me(R) | single bond | H | Et | CH₂OC(=O)(i -Pr) | Me | CH₂CN | O |
| D-7465 | Me(S) | single bond | H | Et | CH₂OC(=O)(i -Pr) | Me | CH₂CN | O |
| D-7466 | H | CH₂ | Me | Et | CH₂OC(=O)(*i*-Pr) | Me | CH₂CN | O |
| D-7467 | Me | CH₂ | H | Et | CH₂OC(=O)(*i*-Pr) | Me | CH₂CN | O |
| D-7468 | H | CH(Me) | H | Et | CH₂OC(=O)(*i*-Pr) | Me | CH₂CN | O |
| D-7469 | H | CH₂CH₂ | Me | Et | CH₂OC(=O)(*i*-Pr) | Me | CH₂CN | O |
| D-7470 | Me | CH₂CH₂ | H | Et | CH₂OC(=O)(*i*-Pr) | Me | CH₂CN | O |
| D-7471 | H | single bond | Me | Et | CH₂OC(=O)(*t*-Bu) | Cl | CH₂CN | O |
| D-7472 | Me | single bond | H | Et | CH₂OC(=O)(*t*-Bu) | Cl | CH₂CN | O |
| D-7473 | Me(R) | single bond | H | Et | CH₂OC(=O)(*t*-Bu) | Cl | CH₂CN | O |
| D-7474 | Me(S) | single bond | H | Et | CH₂OC(=O)(*t*-Bu) | Cl | CH₂CN | O |
| D-7475 | H | CH₂ | Me | Et | CH₂OC(=O)(*t*-Bu) | Cl | CH₂CN | O |
| D-7476 | Me | CH₂ | H | Et | CH₂OC(=O)(*t*-Bu) | Cl | CH₂CN | O |
| D-7477 | H | CH(Me) | H | Et | CH₂OC(=O)(*t*-Bu) | Cl | CH₂CN | O |
| D-7478 | H | CH₂CH₂ | Me | Et | CH₂OC(=O)(*t*-Bu) | Cl | CH₂CN | O |
| D-7479 | Me | CH₂CH₂ | H | Et | CH₂OC(=O)(*t*-Bu) | Cl | CH₂CN | O |
| D-7480 | H | single bond | Me | Et | CH₂OC(=O)(*t*-Bu) | Me | CH₂CN | O |
| D-7481 | Me | single bond | H | Et | CH₂OC(=O)(*t*-Bu) | Me | CH₂CN | O |
| D-7482 | Me(R) | single bond | H | Et | CH₂OC(=O)(*t*-Bu) | Me | CH₂CN | O |
| D-7483 | Me(S) | single bond | H | Et | CH₂OC(=O)(*t*-Bu) | Me | CH₂CN | O |
| D-7484 | H | CH₂ | Me | Et | CH₂OC(=O)(*t*-Bu) | Me | CH₂CN | O |
| D-7485 | Me | CH₂ | H | Et | CH₂OC(=O)(*t*-Bu) | Me | CH₂CN | O |
| D-7486 | H | CH(Me) | H | Et | CH₂OC(=O)(*t*-Bu) | Me | CH₂CN | O |

**[Table 816]**

| Compound Number | Y | W | z | R¹ | R² | R³ | R¹⁰ | A |
|---|---|---|---|---|---|---|---|---|
| D-7487 | H | CH₂CH₂ | Me | Et | CH₂OC(=O)(*t*-Bu) | Me | CH₂CN | O |
| D-7488 | Me | CH₂CH₂ | H | Et | CH₂OC(=O)(*t*-Bu) | Me | CH₂CN | O |
| D-7489 | H | single bond | Me | Et | CH₂OC(=O)OEt | Cl | CH₂CN | O |
| D-7490 | Me | single bond | H | Et | CH₂OC(=O)OEt | Cl | CH₂CN | O |
| D-7491 | Me(R) | single bond | H | Et | CH₂OC(=O)OEt | Cl | CH₂CN | O |
| D-7492 | Me(S) | single bond | H | Et | CH₂OC(=O)OEt | Cl | CH₂CN | O |
| D-7493 | H | CH₂ | Me | Et | CH₂OC(=O)OEt | Cl | CH₂CN | O |
| D-7494 | Me | CH₂ | H | Et | CH₂OC(=O)OEt | Cl | CH₂CN | O |
| D-7495 | H | CH(Me) | H | Et | CH₂OC(=O)OEt | Cl | CH₂CN | O |
| D-7496 | H | CH₂CH₂ | Me | Et | CH₂OC(=O)OEt | Cl | CH₂CN | O |
| D-7497 | Me | CH₂CH₂ | H | Et | CH₂OC(=O)OEt | Cl | CH₂CN | O |
| D-7498 | H | single bond | Me | Et | CH₂OC(=O)OEt | Me | CH₂CN | O |
| D-7499 | Me | single bond | H | Et | CH₂OC(=O)OEt | Me | CH₂CN | O |
| D-7500 | Me(R) | single bond | H | Et | CH₂OC(=O)OEt | Me | CH₂CN | O |
| D-7501 | Me(S) | single bond | H | Et | CH₂OC(=O)OEt | Me | CH₂CN | O |
| 0-7502 | H | CH₂ | Me | Et | CH₂OC(=O)OEt | Me | CH₂CN | O |
| D-7503 | Me | CH₂ | H | Et | CH₂OC(=O)OEt | Me | CH₂CN | O |
| D-7504 | H | CH(Me) | H | Et | CH₂OC(=O)OEt | Me | CH₂CN | O |
| 0-7505 | H | CH₂CH₂ | Me | Et | CH₂OC(=O)OEt | Me | CH₂CN | O |
| D-7506 | Me | CH₂CH₂ | H | Et | CH₂OC(=O)OEt | Me | CH₂CN | O |
| D-7507 | H | single bond | Me | Et | CH₂OC(=O)O(*i*-Pr) | Cl | CH₂CN | O |
| D-7508 | Me | single bond | H | Et | CH₂OC(=O)O(*i*-Pr) | Cl | CH₂CN | O |
| 0-7509 | Me(R) | single bond | H | Et | CH₂OC(=O)O(*i*-Pr) | Cl | CH₂CN | O |
| D-7510 | Me(S) | single bond | H | Et | CH₂OC(=O)O(*i*-Pr) | Cl | CH₂CN | O |
| D-7511 | H | CH₂ | Me | Et | CH₂OC(=O)O(*i*-Pr) | Cl | CH₂CN | O |
| D-7512 | Me | CH₂ | H | Et | CH₂OC(=O)O(*i*-Pr) | Cl | CH₂CN | O |
| D-7513 | H | CH(Me) | H | Et | CH₂OC(=O)O(*i*-Pr) | Cl | CH₂CN | O |
| D-7514 | H | CH₂CH₂ | Me | Et | CH₂OC(=O)O(*i*-Pr) | Cl | CH₂CN | O |
| D-7515 | Me | CH₂CH₂ | H | Et | CH₂OC(=O)O(*i*-Pr) | Cl | CH₂CN | O |
| D-7516 | H | single bond | Me | Et | CH₂OC(=O)O(*i*-Pr) | Me | CH₂CN | O |
| D-7517 | Me | single bond | H | Et | CH₂OC(=O)O(*i*-Pr) | Me | CH₂CN | O |
| D-7518 | Me(R) | single bond | H | Et | CH₂OC(=O)O(*i*-Pr) | Me | CH₂CN | O |
| D-7519 | Me(S) | single bond | H | Et | CH₂OC(=O)O(*i*-Pr) | Me | CH₂CN | O |
| D-7520 | H | CH₂ | Me | Et | CH₂OC(=O)O(*i*-Pr) | Me | CH₂CN | O |
| D-7521 | Me | CH₂ | H | Et | CH₂OC(=O)O(*i*-Pr) | Me | CH₂CN | O |
| D-7522 | H | CH(Me) | H | Et | CH₂OC(=O)O(*i*-Pr) | Me | CH₂CN | O |
| D-7523 | H | CH₂CH₂ | Me | Et | CH₂OC(=O)O(*i*-Pr) | Me | CH₂CN | O |
| D-7524 | Me | CH₂CH₂ | H | Et | CH₂OC(=O)O(*i*-Pr) | Me | CH₂CN | O |
| D-7525 | H | single bond | Me | Et | CH₂N(Me)C(=O)OMe | Cl | CH₂CN | O |
| D-7526 | Me | single bond | H | Et | CH₂N(Me)C(=O)OMe | Cl | CH₂CN | O |
| D-7527 | Me(R) | single bond | H | Et | CH₂N(Me)C(=O)OMe | Cl | CH₂CN | O |
| D-7528 | Me(S) | single bond | H | Et | CH₂N(Me)C(=O)OMe | Cl | CH₂CN | O |
| D-7529 | H | CH₂ | Me | Et | CH₂N(Me)C(=O)OMe | Cl | CH₂CN | O |
| D-7530 | Me | CH₂ | H | Et | CH₂N(Me)C(=O)OMe | Cl | CH₂CN | O |

**[Table 817]**

| Compound Number | Y | W | z | R¹ | R² | R³ | R¹⁰ | A |
|---|---|---|---|---|---|---|---|---|
| D-7531 | H | CH(Me) | H | Et | CH₂N(Me)C(=O)OMe | Cl | CH₂CN | O |
| D-7532 | H | CH₂CH₂ | Me | Et | CH₂N(Me)C(=O)OMe | Cl | CH₂CN | O |
| D-7533 | Me | CH₂CH₂ | H | Et | CH₂N(Me)C(=O)OMe | Cl | CH₂CN | O |
| D-7534 | H | single bond | Me | Et | CH₂N(Me)C(=O)OMe | Me | CH₂CN | O |
| D-7535 | Me | single bond | H | Et | CH₂N(Me)C(=O)OMe | Me | CH₂CN | O |
| D-7536 | Me(R) | single bond | H | Et | CH₂N(Me)C(=O)OMe | Me | CH₂CN | O |
| D-7537 | Me(S) | single bond | H | Et | CH₂N(Me)C(=O)OMe | Me | CH₂CN | O |
| D-7538 | H | CH₂ | Me | Et | CH₂N(Me)C(=O)OMe | Me | CH₂CN | O |
| D-7539 | Me | CH₂ | H | Et | CH₂N(Me)C(=O)OMe | Me | CH₂CN | O |
| D-7540 | H | CH(Me) | H | Et | CH₂N(Me)C(=O)OMe | Me | CH₂CN | O |
| D-7541 | H | CH₂CH₂ | Me | Et | CH₂N(Me)C(=O)OMe | Me | CH₂CN | O |
| D-7542 | Me | CH₂CH₂ | H | Et | CH₂N(Me)C(=O)OMe | Me | CH₂CN | O |
| D-7543 | H | single bond | Me | Et | C(=O)OCH₂CH=CH₂ | Cl | C(=O)Me | O |
| D-7544 | Me | single bond | H | Et | C(=O)OCH₂CH=CH₂ | Cl | C(=O)Me | O |
| D-7545 | Me(R) | single bond | H | Et | C(=O)OCH₂CH=CH₂ | Cl | C(=O)Me | O |
| D-7546 | Me(S) | single bond | H | Et | C(=O)OCH₂CH=CH₂ | Cl | C(=O)Me | O |
| D-7547 | H | CH₂ | Me | Et | C(=O)OCH₂CH=CH₂ | Cl | C(=O)Me | O |
| D-7548 | Me | CH₂ | H | Et | C(=O)OCH₂CH=CH₂ | Cl | C(=O)Me | O |
| D-7549 | H | CH(Me) | H | Et | C(=O)OCH₂CH=CH₂ | Cl | C(=O)Me | O |
| D-7550 | H | CH₂CH₂ | Me | Et | C(=O)OCH₂CH=CH₂ | Cl | C(=O)Me | O |
| D-7551 | Me | CH₂CH₂ | H | Et | C(=O)OCH₂CH=CH₂ | Cl | C(=O)Me | O |
| D-7552 | H | single bond | Me | Et | C(=O)OCH₂CH=CH₂ | Me | C(=O)Me | O |
| D-7553 | Me | single bond | H | Et | C(=O)OCH₂CH=CH₂ | Me | C(=O)Me | O |
| D-7554 | Me(R) | single bond | H | Et | C(=O)OCH₂CH=CH₂ | Me | C(=O)Me | O |
| D-7555 | Me(S) | single bond | H | Et | C(=O)OCH₂CH=CH₂ | Me | C(=O)Me | O |
| D-7556 | H | CH₂ | Me | Et | C(=O)OCH₂CH=CH₂ | Me | C(=O)Me | O |
| D-7557 | Me | CH₂ | H | Et | C(=O)OCH₂CH=CH₂ | Me | C(=O)Me | O |
| D-7558 | H | CH(Me) | H | Et | C(=O)OCH₂CH=CH₂ | Me | C(=O)Me | O |
| D-7559 | H | CH₂CH₂ | Me | Et | C(=O)OCH₂CH=CH₂ | Me | C(=O)Me | O |
| D-7560 | Me | CH₂CH₂ | H | Et | C(=O)OCH₂CH=CH₂ | Me | C(=O)Me | O |
| D-7561 | H | single bond | Me | Et | C(=O)OCH₂CCl₃ | Cl | C(=O)Me | O |
| D-7562 | Me | single bond | H | Et | C(=O)OCH₂CCl₃ | Cl | C(=O)Me | O |
| D-7563 | Me(R) | single bond | H | Et | C(=O)OCH₂CCl₃ | Cl | C(=O)Me | O |
| D-7564 | Me(S) | single bond | H | Et | C(=O)OCH₂CCl₃ | Cl | C(=O)Me | O |
| D-7565 | H | CH₂ | Me | Et | C(=O)OCH₂CCl₃ | Cl | C(=O)Me | O |
| D-7566 | Me | CH₂ | H | Et | C(=O)OCH₂CCl₃ | Cl | C(=O)Me | O |
| D-7567 | H | CH(Me) | H | Et | C(=O)OCH₂CCl₃ | Cl | C(=O)Me | O |
| D-7568 | H | CH₂CH₂ | Me | Et | C(=O)OCH₂CCl₃ | Cl | C(=O)Me | O |
| D-7569 | Me | CH₂CH₂ | H | Et | C(=O)OCH₂CCl₃ | Cl | C(=O)Me | O |
| D-7570 | H | single bond | Me | Et | C(=O)OCH₂CCl₃ | Me | C(=O)Me | O |
| D-7571 | Me | single bond | H | Et | C(=O)OCH₂CCl₃ | Me | C(=O)Me | O |
| D-7572 | Me(R) | single bond | H | Et | C(=O)OCH₂CCl₃ | Me | C(=O)Me | O |
| D-7573 | Me(S) | single bond | H | Et | C(=O)OCH₂CCl₃ | Me | C(=O)Me | O |
| D-7574 | H | CH₂ | Me | Et | C(=O)OCH₂CCl₃ | Me | C(=O)Me | O |

**[Table 818]**

| Compound Number | Y | W | Z | R¹ | R² | R³ | R¹⁰ | A |
|---|---|---|---|---|---|---|---|---|
| D-7575 | Me | CH₂ | H | Et | C(=O)OCH₂CCl₃ | Me | C(=O)Me | O |
| D-7576 | H | CH(Me) | H | Et | C(=O)OCH₂CCl₃ | Me | C(=O)Me | O |
| D-7577 | H | CH₂CH₂ | Me | Et | C(=O)OCH₂CCl₃ | Me | C(=O)Me | O |
| D-7578 | Me | CH₂CH₂ | H | Et | C(=O)OCH₂CCl₃ | Me | C(=O)Me | O |
| D-7579 | H | single bond | Me | Et | C(=O)N(Me)₂ | Cl | C(=O)Me | O |
| D-7580 | Me | single bond | H | Et | C(=O)N(Me)₂ | Cl | C(=O)Me | O |
| D-7581 | Me(R) | single bond | H | Et | C(=O)N(Me)₂ | Cl | C(=O)Me | O |
| D-7582 | Me(S) | single bond | H | Et | C(=O)N(Me)₂ | Cl | C(=O)Me | O |
| D-7583 | H | CH₂ | Me | Et | C(=O)N(Me)₂ | Cl | C(=O)Me | O |
| D-7584 | Me | CH₂ | H | Et | C(=O)N(Me)₂ | Cl | C(=O)Me | O |
| D-7585 | H | CH(Me) | H | Et | C(=O)N(Me)₂ | Cl | C(=O)Me | O |
| D-7586 | H | CH₂CH₂ | Me | Et | C(=O)N(Me)₂ | Cl | C(=O)Me | O |
| D-7587 | Me | CH₂CH₂ | H | Et | C(=O)N(Me)₂ | Cl | C(=O)Me | O |
| D-7588 | H | single bond | Me | Et | C(=O)N(Me)₂ | Me | C(=O)Me | O |
| D-7589 | Me | single bond | H | Et | C(=O)N(Me)₂ | Me | C(=O)Me | O |
| D-7590 | Me(R) | single bond | H | Et | C(=O)N(Me)₂ | Me | C(=O)Me | O |
| D-7591 | Me(S) | single bond | H | Et | C(=O)N(Me)₂ | Me | C(=O)Me | O |
| D-7592 | H | CH₂ | Me | Et | C(=O)N(Me)₂ | Me | C(=O)Me | O |
| D-7593 | Me | CH₂ | H | Et | C(=O)N(Me)₂ | Me | C(=O)Me | O |
| D-7594 | H | CH(Me) | H | Et | C(=O)N(Me)₂ | Me | C(=O)Me | O |
| D-7595 | H | CH₂CH₂ | Me | Et | C(=O)N(Me)₂ | Me | C(=O)Me | O |
| D-7596 | Me | CH₂CH₂ | H | Et | C(=O)N(Me)₂ | Me | C(=O)Me | O |
| D-7597 | H | single bond | Me | Et | CH₂OC(=O)Me | Cl | C(=O)Me | O |
| D-7598 | Me | single bond | H | Et | CH₂OC(=O)Me | Cl | C(=O)Me | O |
| D-7599 | Me(R) | single bond | H | Et | CH₂OC(=O)Me | Cl | C(=O)Me | O |
| D-7600 | Me(S) | single bond | H | Et | CH₂OC(=O)Me | Cl | C(=O)Me | O |
| D-7601 | H | CH₂ | Me | Et | CH₂OC(=O)Me | Cl | C(=O)Me | O |
| D-7602 | Me | CH₂ | H | Et | CH₂OC(=O)Me | Cl | C(=O)Me | O |
| D-7603 | H | CH(Me) | H | Et | CH₂OC(=O)Me | Cl | C(=O)Me | O |
| D-7604 | H | CH₂CH₂ | Me | Et | CH₂OC(=O)Me | Cl | C(=O)Me | O |
| D-7605 | Me | CH₂CH₂ | H | Et | CH₂OC(=O)Me | Cl | C(=O)Me | O |
| D-7606 | H | single bond | Me | Et | CH₂OC(=O)Me | Me | C(=O)Me | O |
| D-7607 | Me | single bond | H | Et | CH₂OC(=O)Me | Me | C(=O)Me | O |
| D-7608 | Me(R) | single bond | H | Et | CH₂OC(=O)Me | Me | C(=O)Me | O |
| D-7609 | Me(S) | single bond | H | Et | CH₂OC(=O)Me | Me | C(=O)Me | O |
| D-7610 | H | CH₂ | Me | Et | CH₂OC(=O)Me | Me | C(=O)Me | O |
| D-7611 | Me | CH₂ | H | Et | CH₂OC(=O)Me | Me | C(=O)Me | O |
| D-7612 | H | CH(Me) | H | Et | CH₂OC(=O)Me | Me | C(=O)Me | O |
| D-7613 | H | CH₂CH₂ | Me | Et | CH₂OC(=O)Me | Me | C(=O)Me | O |
| D-7614 | Me | CH₂CH₂ | H | Et | CH₂OC(=O)Me | Me | C(=O)Me | O |
| D-7615 | H | single bond | Me | Et | CH₂OC(=O)(*i*-Pr) | Cl | C(=O)Me | O |
| D-7616 | Me | single bond | H | Et | CH₂OC(=O)(*i*-Pr) | Cl | C(=O)Me | O |
| D-7617 | Me(R) | single bond | H | Et | CH₂OC(=O)(*i*-Pr) | Cl | C(=O)Me | O |
| D-7618 | Me(S) | single bond | H | Et | CH₂OC(=O)(*i*-Pr) | Cl | C(=O)Me | O |

**[Table 819]**

| Compound Number | Y | W | z | R¹ | R² | R³ | R¹⁰ | A |
|---|---|---|---|---|---|---|---|---|
| D-7619 | H | CH₂ | Me | Et | CH₂OC(=O)(*i*-Pr) | Cl | C(=O)Me | O |
| D-7620 | Me | CH₂ | H | Et | CH₂OC(=O)(*i*-Pr) | Cl | C(=O)Me | O |
| D-7621 | H | CH(Me) | H | Et | CH₂OC(=O)(*i*-Pr) | Cl | C(=O)Me | O |
| D-7622 | H | CH₂CH₂ | Me | Et | CH₂OC(=O)(*i*-Pr) | Cl | C(=O)Me | O |
| D-7623 | Me | CH₂CH₂ | H | Et | CH₂OC(=O)(*i*-Pr) | Cl | C(=O)Me | O |
| D-7624 | H | single bond | Me | Et | CH₂OC(=O)(*i*-Pr) | Me | C(=O)Me | O |
| D-7625 | Me | single bond | H | Et | CH₂OC(=O)(*i*-Pr) | Me | C(=O)Me | O |
| D-7626 | Me(R) | single bond | H | Et | CH₂OC(=O)(*i*-Pr) | Me | C(=O)Me | O |
| D-7627 | Me(S) | single bond | H | Et | CH₂OC(=O)(*i*-Pr) | Me | C(=O)Me | O |
| D-7628 | H | CH₂ | Me | Et | CH₂OC(=O)(*i*-Pr) | Me | C(=O)Me | O |
| D-7629 | Me | CH₂ | H | Et | CH₂OC(=O)(*i*-Pr) | Me | C(=O)Me | O |
| D-7630 | H | CH(Me) | H | Et | CH₂OC(=O)(*i*-Pr) | Me | C(=O)Me | O |
| D-7631 | H | CH₂CH₂ | Me | Et | CH₂OC(=O)(*i*-Pr) | Me | C(=O)Me | O |
| D-7632 | Me | CH₂CH₂ | H | Et | CH₂OC(=O)(*i*-Pr) | Me | C(=O)Me | O |
| D-7633 | H | single bond | Me | Et | CH₂OC(=O)(*t*-Bu) | Cl | C(=O)Me | O |
| D-7634 | Me | single bond | H | Et | CH₂OC(=O)(*t*-Bu) | Cl | C(=O)Me | O |
| D-7635 | Me(R) | single bond | H | Et | CH₂OC(=O)(*t*-Bu) | Cl | C(=O)Me | O |
| D-7636 | Me(S) | single bond | H | Et | CH₂OC(=O)(*t*-Bu) | Cl | C(=O)Me | O |
| D-7637 | H | CH₂ | Me | Et | CH₂OC(=O)(*t*-Bu) | Cl | C(=O)Me | O |
| D-7638 | Me | CH₂ | H | Et | CH₂OC(=O)(*t*-Bu) | Cl | C(=O)Me | O |
| D-7639 | H | CH(Me) | H | Et | CH₂OC(=O)(*t*-Bu) | Cl | C(=O)Me | O |
| D-7640 | H | CH₂CH₂ | Me | Et | CH₂OC(=O)(*t*-Bu) | Cl | C(=O)Me | O |
| D-7641 | Me | CH₂CH₂ | H | Et | CH₂OC(=O)(*t*-Bu) | Cl | C(=O)Me | O |
| D-7642 | H | single bond | Me | Et | CH₂OC(=O)(*t*-Bu) | Me | C(=O)Me | O |
| D-7643 | Me | single bond | H | Et | CH₂OC(=O)(*t*-Bu) | Me | C(=O)Me | O |
| D-7644 | Me(R) | single bond | H | Et | CH₂OC(=O)(*t*-Bu) | Me | C(=O)Me | O |
| D-7645 | Me(S) | single bond | H | Et | CH₂OC(=O)(*t*-Bu) | Me | C(=O)Me | O |
| D-7646 | H | CH₂ | Me | Et | CH₂OC(=O)(*t*-Bu) | Me | C(=O)Me | O |
| D-7647 | Me | CH₂ | H | Et | CH₂OC(=O)(*t*-Bu) | Me | C(=O)Me | O |
| D-7648 | H | CH(Me) | H | Et | CH₂OC(=O)(*t*-Bu) | Me | C(=O)Me | O |
| D-7649 | H | CH₂CH₂ | Me | Et | CH₂OC(=O)(*t*-Bu) | Me | C(=O)Me | O |
| D-7650 | Me | CH₂CH₂ | H | Et | CH₂OC(=O)(*t*-Bu) | Me | C(=O)Me | O |
| D-7651 | H | single bond | Me | Et | CH₂OC(=O)OEt | Cl | C(=O)Me | O |
| D-7652 | Me | single bond | H | Et | CH₂OC(=O)OEt | Cl | C(=O)Me | O |
| D-7653 | Me(R) | single bond | H | Et | CH₂OC(=O)OEt | Cl | C(=O)Me | O |
| D-7654 | Me(S) | single bond | H | Et | CH₂OC(=O)OEt | Cl | C(=O)Me | O |
| D-7655 | H | CH₂ | Me | Et | CH₂OC(=O)OEt | Cl | C(=O)Me | O |
| D-7656 | Me | CH₂ | H | Et | CH₂OC(=O)OEt | Cl | C(=O)Me | O |
| D-7657 | H | CH(Me) | H | Et | CH₂OC(=O)OEt | Cl | C(=O)Me | O |
| D-7658 | H | CH₂CH₂ | Me | Et | CH₂OC(=O)OEt | Cl | C(=O)Me | O |
| D-7659 | Me | CH₂CH₂ | H | Et | CH₂OC(=O)OEt | Cl | C(=O)Me | O |
| D-7660 | H | single bond | Me | Et | CH₂OC(=O)OEt | Me | C(=O)Me | O |
| D-7661 | Me | single bond | H | Et | CH₂OC(=O)OEt | Me | C(=O)Me | O |
| D-7662 | Me(R) | single bond | H | Et | CH₂OC(=O)OEt | Me | C(=O)Me | O |

**[Table 820]**

| Compound Number | Y | W | z | R¹ | R² | R³ | R¹⁰ | A |
|---|---|---|---|---|---|---|---|---|
| D-7663 | Me(S) | single bond | H | Et | CH₂OC(=O)OEt | Me | C(=O)Me | O |
| D-7664 | H | CH₂ | Me | Et | CH₂OC(=O)OEt | Me | C(=O)Me | O |
| D-7665 | Me | CH₂ | H | Et | CH₂OC(=O)OEt | Me | C(=O)Me | O |
| D-7666 | H | CH(Me) | H | Et | CH₂OC(=O)OEt | Me | C(=O)Me | O |
| D-7667 | H | CH₂CH₂ | Me | Et | CH₂OC(=O)OEt | Me | C(=O)Me | O |
| D-7668 | Me | CH₂CH₂ | H | Et | CH₂OC(=O)OEt | Me | C(=O)Me | O |
| D-7669 | H | single bond | Me | Et | CH₂OC(=O)O(*i*-Pr) | Cl | C(=O)Me | O |
| D-7670 | Me | single bond | H | Et | CH₂OC(=O)O(*i*-Pr) | Cl | C(=O)Me | O |
| D-7671 | Me(R) | single bond | H | Et | CH₂OC(=O)O(*i*-Pr) | Cl | C(=O)Me | O |
| D-7672 | Me(S) | single bond | H | Et | CH₂OC(=O)O(*i*-Pr) | Cl | C(=O)Me | O |
| D-7673 | H | CH₂ | Me | Et | CH₂OC(=O)O(*i*-Pr) | Cl | C(=O)Me | O |
| D-7674 | Me | CH₂ | H | Et | CH₂OC(=O)O(*i*-Pr) | Cl | C(=O)Me | O |
| D-7675 | H | CH(Me) | H | Et | CH₂OC(=O)O(*i*-Pr) | Cl | C(=O)Me | O |
| D-7676 | H | CH₂CH₂ | Me | Et | CH₂OC(=O)O(*i*-Pr) | Cl | C(=O)Me | O |
| D-7677 | Me | CH₂CH₂ | H | Et | CH₂OC(=O)O(*i*-Pr) | Cl | C(=O)Me | O |
| D-7678 | H | single bond | Me | Et | CH₂OC(=O)O(*i*-Pr) | Me | C(=O)Me | O |
| D-7679 | Me | single bond | H | Et | CH₂OC(=O)O(*i*-Pr) | Me | C(=O)Me | O |
| D-7680 | Me(R) | single bond | H | Et | CH₂OC(=O)O(*i*-Pr) | Me | C(=O)Me | O |
| D-7681 | Me(S) | single bond | H | Et | CH₂OC(=O)O(*i*-Pr) | Me | C(=O)Me | O |
| D-7682 | H | CH₂ | Me | Et | CH₂OC(=O)O(*i*-Pr) | Me | C(=O)Me | O |
| D-7683 | Me | CH₂ | H | Et | CH₂OC(=O)O(*i*-Pr) | Me | C(=O)Me | O |
| D-7684 | H | CH(Me) | H | Et | CH₂OC(=O)O(*i*-Pr) | Me | C(=O)Me | O |
| D-7685 | H | CH₂CH₂ | Me | Et | CH₂OC(=O)O(*i*-Pr) | Me | C(=O)Me | O |
| D-7686 | Me | CH₂CH₂ | H | Et | CH₂OC(=O)O(*i*-Pr) | Me | C(=O)Me | O |
| D-7687 | H | single bond | Me | Et | CH₂N(Me)C(=O)OMe | Cl | C(=O)Me | O |
| D-7688 | Me | single bond | H | Et | CH₂N(Me)C(=O)OMe | Cl | C(=O)Me | O |
| D-7689 | Me(R) | single bond | H | Et | CH₂N(Me)C(=O)OMe | Cl | C(=O)Me | O |
| D-7690 | Me(S) | single bond | H | Et | CH₂N(Me)C(=O)OMe | Cl | C(=O)Me | O |
| D-7691 | H | CH₂ | Me | Et | CH₂N(Me)C(=O)OMe | Cl | C(=O)Me | O |
| D-7692 | Me | CH₂ | H | Et | CH₂N(Me)C(=O)OMe | Cl | C(=O)Me | O |
| D-7693 | H | CH(Me) | H | Et | CH₂N(Me)C(=O)OMe | Cl | C(=O)Me | O |
| D-7694 | H | CH₂CH₂ | Me | Et | CH₂N(Me)C(=O)OMe | Cl | C(=O)Me | O |
| D-7695 | Me | CH₂CH₂ | H | Et | CH₂N(Me)C(=O)OMe | Cl | C(=O)Me | O |
| D-7696 | H | single bond | Me | Et | CH₂N(Me)C(=O)OMe | Me | C(=O)Me | O |
| D-7697 | Me | single bond | H | Et | CH₂N(Me)C(=O)OMe | Me | C(=O)Me | O |
| D-7698 | Me(R) | single bond | H | Et | CH₂N(Me)C(=O)OMe | Me | C(=O)Me | O |
| D-7699 | Me(S) | single bond | H | Et | CH₂N(Me)C(=O)OMe | Me | C(=O)Me | O |
| D-7700 | H | CH₂ | Me | Et | CH₂N(Me)C(=O)OMe | Me | C(=O)Me | O |
| D-7701 | Me | CH₂ | H | Et | CH₂N(Me)C(=O)OMe | Me | C(=O)Me | O |
| D-7702 | H | CH(Me) | H | Et | CH₂N(Me)C(=O)OMe | Me | C(=O)Me | O |
| D-7703 | H | CH₂CH₂ | Me | Et | CH₂N(Me)C(=O)OMe | Me | C(=O)Me | O |
| D-7704 | Me | CH₂CH₂ | H | Et | CH₂N(Me)C(=O)OMe | Me | C(=O)Me | O |
| D-7705 | H | single bond | Me | *c*-Pr | C(=O)OCH₂CH=CH₂ | Cl | H | O |
| D-7706 | Me | single bond | H | *c*-Pr | C(=O)OCH₂CH=CH₂ | Cl | H | O |

**[Table 821]**

| Compound Number | Y | W | z | R¹ | R² | R³ | R¹⁰ | A |
|---|---|---|---|---|---|---|---|---|
| D-7707 | Me(R) | single bond | H | *c*-Pr | C(=O)OCH₂CH=CH₂ | Cl | H | O |
| D-7708 | Me(S) | single bond | H | *c*-Pr | C(=O)OCH₂CH=CH₂ | Cl | H | O |
| 0-7709 | H | CH₂ | Me | *c*-Pr | C(=O)OCH₂CH=CH₂ | Cl | H | O |
| D-7710 | Me | CH₂ | H | *c*-Pr | C(=O)OCH₂CH=CH₂ | Cl | H | O |
| D-7711 | H | CH(Me) | H | *c*-Pr | C(=O)OCH₂CH=CH₂ | Cl | H | O |
| D-7712 | H | CH₂CH₂ | Me | *c*-Pr | C(=O)OCH₂CH=CH₂ | Cl | H | O |
| D-7713 | Me | CH₂CH₂ | H | *c*-Pr | C(=O)OCH₂CH=CH₂ | Cl | H | O |
| D-7714 | H | single bond | Me | *c*-Pr | C(=O)OCH₂CH=CH₂ | Me | H | O |
| D-7715 | Me | single bond | H | *c*-Pr | C(=O)OCH₂CH=CH₂ | Me | H | O |
| D-7716 | Me(R) | single bond | H | *c*-Pr | C(=O)OCH₂CH=CH₂ | Me | H | O |
| D-7717 | Me(S) | single bond | H | *c*-Pr | C(=O)OCH₂CH=CH₂ | Me | H | O |
| D-7718 | H | CH₂ | Me | *c*-Pr | C(=O)OCH₂CH=CH₂ | Me | H | O |
| D-7719 | Me | CH₂ | H | *c*-Pr | C(=O)OCH₂CH=CH₂ | Me | H | O |
| D-7720 | H | CH(Me) | H | *c*-Pr | C(=O)OCH₂CH=CH₂ | Me | H | O |
| D-7721 | H | CH₂CH₂ | Me | *c*-Pr | C(=O)OCH₂CH=CH₂ | Me | H | O |
| D-7722 | Me | CH₂CH₂ | H | *c*-Pr | C(=O)OCH₂CH=CH₂ | Me | H | O |
| D-7723 | H | single bond | Me | *c*-Pr | C(=O)OCH₂CCl₃ | Cl | H | O |
| D-7724 | Me | single bond | H | *c*-Pr | C(=O)OCH₂CCl₃ | Cl | H | O |
| D-7725 | Me(R) | single bond | H | *c*-Pr | C(=O)OCH₂CCl₃ | Cl | H | O |
| D-7726 | Me(S) | single bond | H | *c*-Pr | C(=O)OCH₂CCl₃ | Cl | H | O |
| D-7727 | H | CH₂ | Me | *c*-Pr | C(=O)OCH₂CCl₃ | Cl | H | O |
| D-7728 | Me | CH₂ | H | *c*-Pr | C(=O)OCH₂CCl₃ | Cl | H | O |
| D-7729 | H | CH(Me) | H | *c*-Pr | C(=O)OCH₂CCl₃ | Cl | H | O |
| D-7730 | H | CH₂CH₂ | Me | *c*-Pr | C(=O)OCH₂CCl₃ | Cl | H | O |
| D-7731 | Me | CH₂CH₂ | H | *c*-Pr | C(=O)OCH₂CCl₃ | Cl | H | O |
| D-7732 | H | single bond | Me | *c*-Pr | C(=O)OCH₂CCl₃ | Me | H | O |
| D-7733 | Me | single bond | H | *c*-Pr | C(=O)OCH₂CCl₃ | Me | H | O |
| D-7734 | Me(R) | single bond | H | *c*-Pr | C(=O)OCH₂CCl₃ | Me | H | O |
| D-7735 | Me(S) | single bond | H | *c*-Pr | C(=O)OCH₂CCl₃ | Me | H | O |
| D-7736 | H | CH₂ | Me | *c*-Pr | C(=O)OCH₂CCl₃ | Me | H | O |
| D-7737 | Me | CH₂ | H | *c*-Pr | C(=O)OCH₂CCl₃ | Me | H | O |
| D-7738 | H | CH(Me) | H | *c*-Pr | C(=O)OCH₂CCl₃ | Me | H | O |
| D-7739 | H | CH₂CH₂ | Me | *c*-Pr | C(=O)OCH₂CCl₃ | Me | H | O |
| D-7740 | Me | CH₂CH₂ | H | *c*-Pr | C(=O)OCH₂CCl₃ | Me | H | O |
| D-7741 | H | single bond | Me | *c*-Pr | C(=O)N(Me)₂ | Cl | H | O |
| D-7742 | Me | single bond | H | *c*-Pr | C(=O)N(Me)₂ | Cl | H | O |
| D-7743 | Me(R) | single bond | H | *c*-Pr | C(=O)N(Me)₂ | Cl | H | O |
| D-7744 | Me(S) | single bond | H | *c*-Pr | C(=O)N(Me)₂ | Cl | H | O |
| D-7745 | H | CH₂ | Me | *c*-Pr | C(=O)N(Me)₂ | Cl | H | O |
| D-7746 | Me | CH₂ | H | *c*-Pr | C(=O)N(Me)₂ | Cl | H | O |
| D-7747 | H | CH(Me) | H | *c*-Pr | C(=O)N(Me)₂ | Cl | H | O |
| D-7748 | H | CH₂CH₂ | Me | *c*-Pr | C(=O)N(Me)₂ | Cl | H | O |
| D-7749 | Me | CH₂CH₂ | H | *c*-Pr | C(=O)N(Me)₂ | Cl | H | O |
| D-7750 | H | single bond | Me | *c*-Pr | C(=O)N(Me)₂ | Me | H | O |

**[Table 822]**

| Compound Number | Y | W | z | R¹ | R² | R³ | R¹⁰ | A |
|---|---|---|---|---|---|---|---|---|
| D-7751 | Me | single bond | H | *c*-Pr | C(=O)N(Me)₂ | Me | H | O |
| D-7752 | Me(R) | single bond | H | *c*-Pr | C(=O)N(Me)₂ | Me | H | O |
| D-7753 | Me(S) | single bond | H | *c*-Pr | C(=O)N(Me)₂ | Me | H | O |
| D-7754 | H | CH₂ | Me | *c*-Pr | C(=O)N(Me)₂ | Me | H | O |
| D-7755 | Me | CH₂ | H | *c*-Pr | C(=O)N(Me)₂ | Me | H | O |
| D-7756 | H | CH(Me) | H | *c*-Pr | C(=O)N(Me)₂ | Me | H | O |
| D-7757 | H | CH₂CH₂ | Me | *c*-Pr | C(=O)N(Me)₂ | Me | H | O |
| D-7758 | Me | CH₂CH₂ | H | *c*-Pr | C(=O)N(Me)₂ | Me | H | O |
| D-7759 | H | single bond | Me | *c*-Pr | CH₂OC(=O)Me | Cl | H | O |
| D-7760 | Me | single bond | H | *c*-Pr | CH₂OC(=O)Me | Cl | H | O |
| D-7761 | Me(R) | single bond | H | *c*-Pr | CH₂OC(=O)Me | Cl | H | O |
| D-7762 | Me(S) | single bond | H | *c*-Pr | CH₂OC(=O)Me | Cl | H | O |
| D-7763 | H | CH₂ | Me | *c*-Pr | CH₂OC(=O)Me | Cl | H | O |
| D-7764 | Me | CH₂ | H | *c*-Pr | CH₂OC(=O)Me | Cl | H | O |
| D-7765 | H | CH(Me) | H | *c*-Pr | CH₂OC(=O)Me | Cl | H | O |
| D-7766 | H | CH₂CH₂ | Me | *c*-Pr | CH₂OC(=O)Me | Cl | H | O |
| D-7767 | Me | CH₂CH₂ | H | *c*-Pr | CH₂OC(=O)Me | Cl | H | O |
| D-7768 | H | single bond | Me | *c*-Pr | CH₂OC(=O)Me | Me | H | O |
| D-7769 | Me | single bond | H | *c*-Pr | CH₂OC(=O)Me | Me | H | O |
| D-7770 | Me(R) | single bond | H | *c*-Pr | CH₂OC(=O)Me | Me | H | O |
| D-7771 | Me(S) | single bond | H | *c*-Pr | CH₂OC(=O)Me | Me | H | O |
| D-7772 | H | CH₂ | Me | *c*-Pr | CH₂OC(=O)Me | Me | H | O |
| D-7773 | Me | CH₂ | H | *c*-Pr | CH₂OC(=O)Me | Me | H | O |
| D-7774 | H | CH(Me) | H | *c*-Pr | CH₂OC(=O)Me | Me | H | O |
| D-7775 | H | CH₂CH₂ | Me | *c*-Pr | CH₂OC(=O)Me | Me | H | O |
| D-7776 | Me | CH₂CH₂ | H | *c*-Pr | CH₂OC(=O)Me | Me | H | O |
| D-7777 | H | single bond | Me | *c*-Pr | CH₂OC(=O)(*i*-Pr) | Cl | H | O |
| D-7778 | Me | single bond | H | *c*-Pr | CH₂OC(=O)(*i*-Pr) | Cl | H | O |
| D-7779 | Me(R) | single bond | H | *c*-Pr | CH₂OC(=O)(*i*-Pr) | Cl | H | O |
| D-7780 | Me(S) | single bond | H | *c*-Pr | CH₂OC(=O)(*i*-Pr) | Cl | H | O |
| D-7781 | H | CH₂ | Me | *c*-Pr | CH₂OC(=O)(*i*-Pr) | Cl | H | O |
| D-7782 | Me | CH₂ | H | *c*-Pr | CH₂OC(=O)(*i*-Pr) | Cl | H | O |
| D-7783 | H | CH(Me) | H | *c*-Pr | CH₂OC(=O)(*i*-Pr) | Cl | H | O |
| D-7784 | H | CH₂CH₂ | Me | *c*-Pr | CH₂OC(=O)(*i*-Pr) | Cl | H | O |
| D-7785 | Me | CH₂CH₂ | H | *c*-Pr | CH₂OC(=O)(*i*-Pr) | Cl | H | O |
| D-7786 | H | single bond | Me | *c*-Pr | CH₂OC(=O)(*i*-Pr) | Me | H | O |
| D-7787 | Me | single bond | H | *c*-Pr | CH₂OC(=O)(*i*-Pr) | Me | H | O |
| D-7788 | Me(R) | single bond | H | *c*-Pr | CH₂OC(=O)(*i*-Pr) | Me | H | O |
| D-7789 | Me(S) | single bond | H | *c*-Pr | CH₂OC(=O)(*i*-Pr) | Me | H | O |
| D-7790 | H | CH₂ | Me | *c*-Pr | CH₂OC(=O)(*i*-Pr) | Me | H | O |
| D-7791 | Me | CH₂ | H | *c*-Pr | CH₂OC(=O)(*i*-Pr) | Me | H | O |
| D-7792 | H | CH(Me) | H | *c*-Pr | CH₂OC(=O)(*i*-Pr) | Me | H | O |
| D-7793 | H | CH₂CH₂ | Me | *c*-Pr | CH₂OC(=O)(*i*-Pr) | Me | H | O |
| D-7794 | Me | CH₂CH₂ | H | *c*-Pr | CH₂OC(=O)(*i*-Pr) | Me | H | O |

**[Table 823]**

| Compound Number | Y | W | z | R¹ | R² | R³ | R¹⁰ | A |
|---|---|---|---|---|---|---|---|---|
| D-7795 | H | single bond | Me | *c*-Pr | CH₂OC(=O)(*t*-Bu) | Cl | H | O |
| D-7796 | Me | single bond | H | *c*-Pr | CH₂OC(=O)(*t*-Bu) | Cl | H | O |
| D-7797 | Me(R) | single bond | H | *c*-Pr | CH₂OC(=O)(*t*-Bu) | Cl | H | O |
| D-7798 | Me(S) | single bond | H | *c*-Pr | CH₂OC(=O)(*t*-Bu) | Cl | H | O |
| D-7799 | H | CH₂ | Me | *c*-Pr | CH₂OC(=O)(*t*-Bu) | Cl | H | O |
| D-7800 | Me | CH₂ | H | *c*-Pr | CH₂OC(=O)(*t*-Bu) | Cl | H | O |
| D-7801 | H | CH(Me) | H | *c*-Pr | CH₂OC(=O)(*t*-Bu) | Cl | H | O |
| D-7802 | H | CH₂CH₂ | Me | *c*-Pr | CH₂OC(=O)(*t*-Bu) | Cl | H | O |
| D-7803 | Me | CH₂CH₂ | H | *c*-Pr | CH₂OC(=O)(*t*-Bu) | Cl | H | O |
| D-7804 | H | single bond | Me | *c*-Pr | CH₂OC(=O)(*t*-Bu) | Me | H | O |
| D-7805 | Me | single bond | H | *c*-Pr | CH₂OC(=O)(*t*-Bu) | Me | H | O |
| D-7806 | Me(R) | single bond | H | *c*-Pr | CH₂OC(=O)(*t*-Bu) | Me | H | O |
| D-7807 | Me(S) | single bond | H | *c*-Pr | CH₂OC(=O)(*t*-Bu) | Me | H | O |
| D-7808 | H | CH₂ | Me | *c*-Pr | CH₂OC(=O)(*t*-Bu) | Me | H | O |
| D-7809 | Me | CH₂ | H | *c*-Pr | CH₂OC(=O)(*t*-Bu) | Me | H | O |
| D-7810 | H | CH(Me) | H | *c*-Pr | CH₂OC(=O)(*t*-Bu) | Me | H | O |
| D-7811 | H | CH₂CH₂ | Me | *c*-Pr | CH₂OC(=O)(*t*-Bu) | Me | H | O |
| D-7812 | Me | CH₂CH₂ | H | *c*-Pr | CH₂OC(=O)(*t*-Bu) | Me | H | O |
| D-7813 | H | single bond | Me | *c*-Pr | CH₂OC(=O)OEt | Cl | H | O |
| D-7814 | Me | single bond | H | *c*-Pr | CH₂OC(=O)OEt | Cl | H | O |
| D-7815 | Me(R) | single bond | H | *c*-Pr | CH₂OC(=O)OEt | Cl | H | O |
| D-7816 | Me(S) | single bond | H | *c*-Pr | CH₂OC(=O)OEt | Cl | H | O |
| D-7817 | H | CH₂ | Me | *c*-Pr | CH₂OC(=O)OEt | Cl | H | O |
| D-7818 | Me | CH₂ | H | *c*-Pr | CH₂OC(=O)OEt | Cl | H | O |
| D-7819 | H | CH(Me) | H | *c*-Pr | CH₂OC(=O)OEt | Cl | H | O |
| D-7820 | H | CH₂CH₂ | Me | *c*-Pr | CH₂OC(=O)OEt | Cl | H | O |
| D-7821 | Me | CH₂CH₂ | H | *c*-Pr | CH₂OC(=O)OEt | Cl | H | O |
| D-7822 | H | single bond | Me | *c*-Pr | CH₂OC(=O)OEt | Me | H | O |
| D-7823 | Me | single bond | H | *c*-Pr | CH₂OC(=O)OEt | Me | H | O |
| D-7824 | Me(R) | single bond | H | *c*-Pr | CH₂OC(=O)OEt | Me | H | O |
| D-7825 | Me(S) | single bond | H | *c*-Pr | CH₂OC(=O)OEt | Me | H | O |
| D-7826 | H | CH₂ | Me | *c*-Pr | CH₂OC(=O)OEt | Me | H | O |
| D-7827 | Me | CH₂ | H | *c*-Pr | CH₂OC(=O)OEt | Me | H | O |
| D-7828 | H | CH(Me) | H | *c*-Pr | CH₂OC(=O)OEt | Me | H | O |
| D-7829 | H | CH₂CH₂ | Me | *c*-Pr | CH₂OC(=O)OEt | Me | H | O |
| D-7830 | Me | CH₂CH₂ | H | *c*-Pr | CH₂OC(=O)OEt | Me | H | O |
| D-7831 | H | single bond | Me | *c*-Pr | CH₂OC(=O)O(*i*-Pr) | Cl | H | O |
| D-7832 | Me | single bond | H | *c*-Pr | CH₂OC(=O)O(*i*-Pr) | Cl | H | O |
| D-7833 | Me(R) | single bond | H | *c*-Pr | CH₂OC(=O)O(*i*-Pr) | Cl | H | O |
| D-7834 | Me(S) | single bond | H | *c*-Pr | CH₂OC(=O)O(*i*-Pr) | Cl | H | O |
| D-7835 | H | CH₂ | Me | *c*-Pr | CH₂OC(=O)O(*i*-Pr) | Cl | H | O |
| D-7836 | Me | CH₂ | H | *c*-Pr | CH₂OC(=O)O(*i*-Pr) | Cl | H | O |
| D-7837 | H | CH(Me) | H | *c*-Pr | CH₂OC(=O)O(*i*-Pr) | Cl | H | O |
| D-7838 | H | CH₂CH₂ | Me | *c*-Pr | CH₂OC(=O)O(*i*-Pr) | Cl | H | O |

**[Table 824]**

| Compound Number | Y | W | z | R¹ | R² | R³ | R¹⁰ | A |
|---|---|---|---|---|---|---|---|---|
| D-7839 | Me | CH₂CH₂ | H | *c*-Pr | CH₂OC(=O)O(*i*-Pr) | Cl | H | O |
| D-7840 | H | single bond | Me | *c*-Pr | CH₂OC(=O)O(*i*-Pr) | Me | H | O |
| D-7841 | Me | single bond | H | *c*-Pr | CH₂OC(=O)O(*i*-Pr) | Me | H | O |
| D-7842 | Me(R) | single bond | H | *c*-Pr | CH₂OC(=O)O(*i*-Pr) | Me | H | O |
| D-7843 | Me(S) | single bond | H | *c*-Pr | CH₂OC(=O)O(*i*-Pr) | Me | H | O |
| D-7844 | H | CH₂ | Me | *c*-Pr | CH₂OC(=O)O(*i*-Pr) | Me | H | O |
| D-7845 | Me | CH₂ | H | *c*-Pr | CH₂OC(=O)O(*i*-Pr) | Me | H | O |
| D-7846 | H | CH(Me) | H | *c*-Pr | CH₂OC(=O)O(*i*-Pr) | Me | H | O |
| D-7847 | H | CH₂CH₂ | Me | *c*-Pr | CH₂OC(=O)O(*i*-Pr) | Me | H | O |
| D-7848 | Me | CH₂CH₂ | H | *c*-Pr | CH₂OC(=O)O(*i*-Pr) | Me | H | O |
| D-7849 | H | single bond | Me | *c*-Pr | CH₂N(Me)C(=O)OMe | Cl | H | O |
| D-7850 | Me | single bond | H | *c*-Pr | CH₂N(Me)C(=O)OMe | Cl | H | O |
| D-7851 | Me(R) | single bond | H | *c*-Pr | CH₂N(Me)C(=O)OMe | Cl | H | O |
| D-7852 | Me(S) | single bond | H | *c*-Pr | CH₂N(Me)C(=O)OMe | Cl | H | O |
| D-7853 | H | CH₂ | Me | *c*-Pr | CH₂N(Me)C(=O)OMe | Cl | H | O |
| D-7854 | Me | CH₂ | H | *c*-Pr | CH₂N(Me)C(=O)OMe | Cl | H | O |
| D-7855 | H | CH(Me) | H | *c*-Pr | CH₂N(Me)C(=O)OMe | Cl | H | O |
| D-7856 | H | CH₂CH₂ | Me | *c*-Pr | CH₂N(Me)C(=O)OMe | Cl | H | O |
| D-7857 | Me | CH₂CH₂ | H | *c*-Pr | CH₂N(Me)C(=O)OMe | Cl | H | O |
| D-7858 | H | single bond | Me | *c*-Pr | CH₂N(Me)C(=O)OMe | Me | H | O |
| D-7859 | Me | single bond | H | *c*-Pr | CH₂N(Me)C(=O)OMe | Me | H | O |
| D-7860 | Me(R) | single bond | H | *c*-Pr | CH₂N(Me)C(=O)OMe | Me | H | O |
| D-7861 | Me(S) | single bond | H | *c*-Pr | CH₂N(Me)C(=O)OMe | Me | H | O |
| D-7862 | H | CH₂ | Me | *c*-Pr | CH₂N(Me)C(=O)OMe | Me | H | O |
| D-7863 | Me | CH₂ | H | *c*-Pr | CH₂N(Me)C(=O)OMe | Me | H | O |
| D-7864 | H | CH(Me) | H | *c*-Pr | CH₂N(Me)C(=O)OMe | Me | H | O |
| D-7865 | H | CH₂CH₂ | Me | *c*-Pr | CH₂N(Me)C(=O)OMe | Me | H | O |
| D-7866 | Me | CH₂CH₂ | H | *c*-Pr | CH₂N(Me)C(=O)OMe | Me | H | O |
| D-7867 | H | single bond | Me | *c*-Pr | C(=O)OCH₂CH=CH₂ | Cl | Me | O |
| D-7868 | Me | single bond | H | *c*-Pr | C(=O)OCH₂CH=CH₂ | Cl | Me | O |
| D-7869 | Me(R) | single bond | H | *c*-Pr | C(=O)OCH₂CH=CH₂ | Cl | Me | O |
| D-7870 | Me(S) | single bond | H | *c*-Pr | C(=O)OCH₂CH=CH₂ | Cl | Me | O |
| D-7871 | H | CH₂ | Me | *c*-Pr | C(=O)OCH₂CH=CH₂ | Cl | Me | O |
| D-7872 | Me | CH₂ | H | *c*-Pr | C(=O)OCH₂CH=CH₂ | Cl | Me | O |
| D-7873 | H | CH(Me) | H | *c*-Pr | C(=O)OCH₂CH=CH₂ | Cl | Me | O |
| D-7874 | H | CH₂CH₂ | Me | *c*-Pr | C(=O)OCH₂CH=CH₂ | Cl | Me | O |
| D-7875 | Me | CH₂CH₂ | H | *c*-Pr | C(=O)OCH₂CH=CH₂ | Cl | Me | O |
| D-7876 | H | single bond | Me | *c*-Pr | C(=O)OCH₂CH=CH₂ | Me | Me | O |
| D-7877 | Me | single bond | H | *c*-Pr | C(=O)OCH₂CH=CH₂ | Me | Me | O |
| D-7878 | Me(R) | single bond | H | *c*-Pr | C(=O)OCH₂CH=CH₂ | Me | Me | O |
| D-7879 | Me(S) | single bond | H | *c*-Pr | C(=O)OCH₂CH=CH₂ | Me | Me | O |
| D-7880 | H | CH₂ | Me | *c*-Pr | C(=O)OCH₂CH=CH₂ | Me | Me | O |
| D-7881 | Me | CH₂ | H | *c*-Pr | C(=O)OCH₂CH=CH₂ | Me | Me | O |
| D-7882 | H | CH(Me) | H | *c*-Pr | C(=O)OCH₂CH=CH₂ | Me | Me | O |

**[Table 825]**

| Compound Number | Y | W | z | R¹ | R² | R³ | R¹⁰ | A |
|---|---|---|---|---|---|---|---|---|
| D-7883 | H | CH₂CH₂ | Me | *c*-Pr | C(=O)OCH₂CH=CH₂ | Me | Me | O |
| D-7884 | Me | CH₂CH₂ | H | *c*-Pr | C(=O)OCH₂CH=CH₂ | Me | Me | O |
| D-7885 | H | single bond | Me | *c*-Pr | C(=O)OCH₂CCl₃ | Cl | Me | O |
| D-7886 | Me | single bond | H | *c*-Pr | C(=O)OCH₂CCl₃ | Cl | Me | O |
| D-7887 | Me(R) | single bond | H | *c*-Pr | C(=O)OCH₂CCl₃ | Cl | Me | O |
| D-7888 | Me(S) | single bond | H | *c*-Pr | C(=O)OCH₂CCl₃ | Cl | Me | O |
| D-7889 | H | CH₂ | Me | *c*-Pr | C(=O)OCH₂CCl₃ | Cl | Me | O |
| D-7890 | Me | CH₂ | H | *c*-Pr | C(=O)OCH₂CCl₃ | Cl | Me | O |
| D-7891 | H | CH(Me) | H | *c*-Pr | C(=O)OCH₂CCl₃ | Cl | Me | O |
| D-7892 | H | CH₂CH₂ | Me | *c*-Pr | C(=O)OCH₂CCl₃ | Cl | Me | O |
| D-7893 | Me | CH₂CH₂ | H | *c*-Pr | C(=O)OCH₂CCl₃ | Cl | Me | O |
| D-7894 | H | single bond | Me | *c*-Pr | C(=O)OCH₂CCl₃ | Me | Me | O |
| D-7895 | Me | single bond | H | *c*-Pr | C(=O)OCH₂CCl₃ | Me | Me | O |
| D-7896 | Me(R) | single bond | H | *c*-Pr | C(=O)OCH₂CCl₃ | Me | Me | O |
| D-7897 | Me(S) | single bond | H | *c*-Pr | C(=O)OCH₂CCl₃ | Me | Me | O |
| D-7898 | H | CH₂ | Me | *c*-Pr | C(=O)OCH₂CCl₃ | Me | Me | O |
| D-7899 | Me | CH₂ | H | *c*-Pr | C(=O)OCH₂CCl₃ | Me | Me | O |
| D-7900 | H | CH(Me) | H | *c*-Pr | C(=O)OCH₂CCl₃ | Me | Me | O |
| D-7901 | H | CH₂CH₂ | Me | *c*-Pr | C(=O)OCH₂CCl₃ | Me | Me | O |
| D-7902 | Me | CH₂CH₂ | H | *c*-Pr | C(=O)OCH₂CCl₃ | Me | Me | O |
| D-7903 | H | single bond | Me | *c*-Pr | C(=O)N(Me)₂ | Cl | Me | O |
| D-7904 | Me | single bond | H | *c*-Pr | C(=O)N(Me)₂ | Cl | Me | O |
| D-7905 | Me(R) | single bond | H | *c*-Pr | C(=O)N(Me)₂ | Cl | Me | O |
| D-7906 | Me(S) | single bond | H | *c*-Pr | C(=O)N(Me)₂ | Cl | Me | O |
| D-7907 | H | CH₂ | Me | *c*-Pr | C(=O)N(Me)₂ | Cl | Me | O |
| D-7908 | Me | CH₂ | H | *c*-Pr | C(=O)N(Me)₂ | Cl | Me | O |
| D-7909 | H | CH(Me) | H | *c*-Pr | C(=O)N(Me)₂ | Cl | Me | O |
| D-7910 | H | CH₂CH₂ | Me | *c*-Pr | C(=O)N(Me)₂ | Cl | Me | O |
| D-7911 | Me | CH₂CH₂ | H | *c*-Pr | C(=O)N(Me)₂ | Cl | Me | O |
| D-7912 | H | single bond | Me | *c*-Pr | C(=O)N(Me)₂ | Me | Me | O |
| D-7913 | Me | single bond | H | *c*-Pr | C(=O)N(Me)₂ | Me | Me | O |
| D-7914 | Me(R) | single bond | H | *c*-Pr | C(=O)N(Me)₂ | Me | Me | O |
| D-7915 | Me(S) | single bond | H | *c*-Pr | C(=O)N(Me)₂ | Me | Me | O |
| D-7916 | H | CH₂ | Me | *c*-Pr | C(=O)N(Me)₂ | Me | Me | O |
| D-7917 | Me | CH₂ | H | *c*-Pr | C(=O)N(Me)₂ | Me | Me | O |
| D-7918 | H | CH(Me) | H | *c*-Pr | C(=O)N(Me)₂ | Me | Me | O |
| D-7919 | H | CH₂CH₂ | Me | *c*-Pr | C(=O)N(Me)₂ | Me | Me | O |
| D-7920 | Me | CH₂CH₂ | H | *c*-Pr | C(=O)N(Me)₂ | Me | Me | O |
| D-7921 | H | single bond | Me | *c*-Pr | CH₂OC(=O)Me | Cl | Me | O |
| D-7922 | Me | single bond | H | *c*-Pr | CH₂OC(=O)Me | Cl | Me | O |
| D-7923 | Me(R) | single bond | H | *c*-Pr | CH₂OC(=O)Me | Cl | Me | O |
| D-7924 | Me(S) | single bond | H | *c*-Pr | CH₂OC(=O)Me | Cl | Me | O |
| D-7925 | H | CH₂ | Me | *c*-Pr | CH₂OC(=O)Me | Cl | Me | O |
| D-7926 | Me | CH₂ | H | *c*-Pr | CH₂OC(=O)Me | Cl | Me | O |

**[Table 826]**

| Compound Number | Y | W | z | R¹ | R² | R³ | R¹⁰ | A |
|---|---|---|---|---|---|---|---|---|
| D-7927 | H | CH(Me) | H | *c*-Pr | CH₂OC(=O)Me | Cl | Me | O |
| D-7928 | H | CH₂CH₂ | Me | *c*-Pr | CH₂OC(=O)Me | Cl | Me | O |
| D-7929 | Me | CH₂CH₂ | H | *c*-Pr | CH₂OC(=O)Me | Cl | Me | O |
| D-7930 | H | single bond | Me | *c*-Pr | CH₂OC(=O)Me | Me | Me | O |
| D-7931 | Me | single bond | H | *c*-Pr | CH₂OC(=O)Me | Me | Me | O |
| D-7932 | Me(R) | single bond | H | *c*-Pr | CH₂OC(=O)Me | Me | Me | O |
| D-7933 | Me(S) | single bond | H | *c*-Pr | CH₂OC(=O)Me | Me | Me | O |
| D-7934 | H | CH₂ | Me | *c*-Pr | CH₂OC(=O)Me | Me | Me | O |
| D-7935 | Me | CH₂ | H | *c*-Pr | CH₂OC(=O)Me | Me | Me | O |
| D-7936 | H | CH(Me) | H | *c*-Pr | CH₂OC(=O)Me | Me | Me | O |
| D-7937 | H | CH₂CH₂ | Me | *c*-Pr | CH₂OC(=O)Me | Me | Me | O |
| D-7938 | Me | CH₂CH₂ | H | *c*-Pr | CH₂OC(=O)Me | Me | Me | O |
| D-7939 | H | single bond | Me | *c*-Pr | CH₂OC(=O)(*i*-Pr) | Cl | Me | O |
| D-7940 | Me | single bond | H | *c*-Pr | CH₂OC(=O)(*i*-Pr) | Cl | Me | O |
| D-7941 | Me(R) | single bond | H | *c*-Pr | CH₂OC(=O)(*i*-Pr) | Cl | Me | O |
| D-7942 | Me(S) | single bond | H | *c*-Pr | CH₂OC(=O)(*i*-Pr) | Cl | Me | O |
| D-7943 | H | CH₂ | Me | *c*-Pr | CH₂OC(=O)(*i*-Pr) | Cl | Me | O |
| D-7944 | Me | CH₂ | H | *c*-Pr | CH₂OC(=O)(*i*-Pr) | Cl | Me | O |
| D-7945 | H | CH(Me) | H | *c*-Pr | CH₂OC(=O)(*i*-Pr) | Cl | Me | O |
| D-7946 | H | CH₂CH₂ | Me | *c*-Pr | CH₂OC(=O)(*i*-Pr) | Cl | Me | O |
| D-7947 | Me | CH₂CH₂ | H | *c*-Pr | CH₂OC(=O)(*i*-Pr) | Cl | Me | O |
| D-7948 | H | single bond | Me | *c*-Pr | CH₂OC(=O)(*i*-Pr) | Me | Me | O |
| D-7949 | Me | single bond | H | *c*-Pr | CH₂OC(=O)(*i*-Pr) | Me | Me | O |
| D-7950 | Me(R) | single bond | H | *c*-Pr | CH₂OC(=O)(*i*-Pr) | Me | Me | O |
| D-7951 | Me(S) | single bond | H | *c*-Pr | CH₂OC(=O)(*i*-Pr) | Me | Me | O |
| D-7952 | H | CH₂ | Me | *c*-Pr | CH₂OC(=O)(*i*-Pr) | Me | Me | O |
| D-7953 | Me | CH₂ | H | *c*-Pr | CH₂OC(=O)(*i*-Pr) | Me | Me | O |
| D-7954 | H | CH(Me) | H | *c*-Pr | CH₂OC(=O)(*i*-Pr) | Me | Me | O |
| D-7955 | H | CH₂CH₂ | Me | *c*-Pr | CH₂OC(=O)(*i*-Pr) | Me | Me | O |
| D-7956 | Me | CH₂CH₂ | H | *c*-Pr | CH₂OC(=O)(*i*-Pr) | Me | Me | O |
| D-7957 | H | single bond | Me | *c*-Pr | CH₂OC(=O)(*t*-Bu) | Cl | Me | O |
| D-7958 | Me | single bond | H | *c*-Pr | CH₂OC(=O)(*t*-Bu) | Cl | Me | O |
| D-7959 | Me(R) | single bond | H | *c*-Pr | CH₂OC(=O)(*t*-Bu) | Cl | Me | O |
| D-7960 | Me(S) | single bond | H | *c*-Pr | CH₂OC(=O)(*t*-Bu) | Cl | Me | O |
| D-7961 | H | CH₂ | Me | *c*-Pr | CH₂OC(=O)(*t*-Bu) | Cl | Me | O |
| D-7962 | Me | CH₂ | H | *c*-Pr | CH₂OC(=O)(*t*-Bu) | Cl | Me | O |
| D-7963 | H | CH(Me) | H | *c*-Pr | CH₂OC(=O)(*t*-Bu) | Cl | Me | O |
| D-7964 | H | CH₂CH₂ | Me | *c*-Pr | CH₂OC(=O)(*t*-Bu) | Cl | Me | O |
| D-7965 | Me | CH₂CH₂ | H | *c*-Pr | CH₂OC(=O)(*t*-Bu) | Cl | Me | O |
| D-7966 | H | single bond | Me | *c*-Pr | CH₂OC(=O)(*t*-Bu) | Me | Me | O |
| D-7967 | Me | single bond | H | *c*-Pr | CH₂OC(=O)(*t*-Bu) | Me | Me | O |
| D-7968 | Me(R) | single bond | H | *c*-Pr | CH₂OC(=O)(*t*-Bu) | Me | Me | O |
| D-7969 | Me(S) | single bond | H | *c*-Pr | CH₂OC(=O)(*t*-Bu) | Me | Me | O |
| D-7970 | H | CH₂ | Me | *c*-Pr | CH₂OC(=O)(*t*-Bu) | Me | Me | O |

**[Table 827]**

| Compound Number | Y | W | z | R¹ | R² | R³ | R¹⁰ | A |
|---|---|---|---|---|---|---|---|---|
| D-7971 | Me | CH₂ | H | *c*-Pr | CH₂OC(=O)(*t*-Bu) | Me | Me | O |
| D-7972 | H | CH(Me) | H | *c*-Pr | CH₂OC(=O)(*t*-Bu) | Me | Me | O |
| D-7973 | H | CH₂CH₂ | Me | *c*-Pr | CH₂OC(=O)(*t*-Bu) | Me | Me | O |
| D-7974 | Me | CH₂CH₂ | H | *c*-Pr | CH₂OC(=O)(*t*-Bu) | Me | Me | O |
| D-7975 | H | single bond | Me | *c*-Pr | CH₂OC(=O)OEt | Cl | Me | O |
| D-7976 | Me | single bond | H | *c*-Pr | CH₂OC(=O)OEt | Cl | Me | O |
| D-7977 | Me(R) | single bond | H | *c*-Pr | CH₂OC(=O)OEt | Cl | Me | O |
| D-7978 | Me(S) | single bond | H | *c*-Pr | CH₂OC(=O)OEt | Cl | Me | O |
| D-7979 | H | CH₂ | Me | *c*-Pr | CH₂OC(=O)OEt | Cl | Me | O |
| D-7980 | Me | CH₂ | H | *c*-Pr | CH₂OC(=O)OEt | Cl | Me | O |
| D-7981 | H | CH(Me) | H | *c*-Pr | CH₂OC(=O)OEt | Cl | Me | O |
| D-7982 | H | CH₂CH₂ | Me | *c*-Pr | CH₂OC(=O)OEt | Cl | Me | O |
| D-7983 | Me | CH₂CH₂ | H | *c*-Pr | CH₂OC(=O)OEt | Cl | Me | O |
| D-7984 | H | single bond | Me | *c*-Pr | CH₂OC(=O)OEt | Me | Me | O |
| D-7985 | Me | single bond | H | *c*-Pr | CH₂OC(=O)OEt | Me | Me | O |
| D-7986 | Me(R) | single bond | H | *c*-Pr | CH₂OC(=O)OEt | Me | Me | O |
| D-7987 | Me(S) | single bond | H | *c*-Pr | CH₂OC(=O)OEt | Me | Me | O |
| D-7988 | H | CH₂ | Me | *c*-Pr | CH₂OC(=O)OEt | Me | Me | O |
| D-7989 | Me | CH₂ | H | *c*-Pr | CH₂OC(=O)OEt | Me | Me | O |
| D-7990 | H | CH(Me) | H | *c*-Pr | CH₂OC(=O)OEt | Me | Me | O |
| D-7991 | H | CH₂CH₂ | Me | *c*-Pr | CH₂OC(=O)OEt | Me | Me | O |
| D-7992 | Me | CH₂CH₂ | H | *c*-Pr | CH₂OC(=O)OEt | Me | Me | O |
| D-7993 | H | single bond | Me | *c*-Pr | CH₂OC(=O)O(*i*-Pr) | Cl | Me | O |
| D-7994 | Me | single bond | H | *c*-Pr | CH₂OC(=O)O(*i*-Pr) | Cl | Me | O |
| D-7995 | Me(R) | single bond | H | *c*-Pr | CH₂OC(=O)O(*i*-Pr) | Cl | Me | O |
| D-7996 | Me(S) | single bond | H | *c*-Pr | CH₂OC(=O)O(*i*-Pr) | Cl | Me | O |
| D-7997 | H | CH₂ | Me | *c*-Pr | CH₂OC(=O)O(*i*-Pr) | Cl | Me | O |
| D-7998 | Me | CH₂ | H | *c*-Pr | CH₂OC(=O)O(*i*-Pr) | Cl | Me | O |
| D-7999 | H | CH(Me) | H | *c*-Pr | CH₂OC(=O)O(*i*-Pr) | Cl | Me | O |
| D-8000 | H | CH₂CH₂ | Me | *c*-Pr | CH₂OC(=O)O(*i*-Pr) | Cl | Me | O |
| D-8001 | Me | CH₂CH₂ | H | *c*-Pr | CH₂OC(=O)O(*i*-Pr) | Cl | Me | O |
| D-8002 | H | single bond | Me | *c*-Pr | CH₂OC(=O)O(*i*-Pr) | Me | Me | O |
| D-8003 | Me | single bond | H | *c*-Pr | CH₂OC(=O)O(*i*-Pr) | Me | Me | O |
| D-8004 | Me(R) | single bond | H | *c*-Pr | CH₂OC(=O)O(*i*-Pr) | Me | Me | O |
| D-8005 | Me(S) | single bond | H | *c*-Pr | CH₂OC(=O)O(*i*-Pr) | Me | Me | O |
| D-8006 | H | CH₂ | Me | *c*-Pr | CH₂OC(=O)O(*i*-Pr) | Me | Me | O |
| D-8007 | Me | CH₂ | H | *c*-Pr | CH₂OC(=O)O(*i*-Pr) | Me | Me | O |
| D-8008 | H | CH(Me) | H | *c*-Pr | CH₂OC(=O)O(*i*-Pr) | Me | Me | O |
| D-8009 | H | CH₂CH₂ | Me | *c*-Pr | CH₂OC(=O)O(*i*-Pr) | Me | Me | O |
| D-8010 | Me | CH₂CH₂ | H | *c*-Pr | CH₂OC(=O)O(*i*-Pr) | Me | Me | O |
| D-8011 | H | single bond | Me | *c*-Pr | CH₂N(Me)C(=O)OMe | Cl | Me | O |
| D-8012 | Me | single bond | H | *c*-Pr | CH₂N(Me)C(=O)OMe | Cl | Me | O |
| D-8013 | Me(R) | single bond | H | *c*-Pr | CH₂N(Me)C(=O)OMe | Cl | Me | O |
| D-8014 | Me(S) | single bond | H | *c*-Pr | CH₂N(Me)C(=O)OMe | Cl | Me | O |

**[Table 828]**

| Compound Number | Y | W | z | R¹ | R² | R³ | R¹⁰ | A |
|---|---|---|---|---|---|---|---|---|
| D-8015 | H | CH₂ | Me | *c*-Pr | CH₂N(Me)C(=O)OMe | Cl | Me | O |
| D-8016 | Me | CH₂ | H | *c*-Pr | CH₂N(Me)C(=O)OMe | Cl | Me | O |
| D-8017 | H | CH(Me) | H | *c*-Pr | CH₂N(Me)C(=O)OMe | Cl | Me | O |
| 0-8018 | H | CH₂CH₂ | Me | *c*-Pr | CH₂N(Me)C(=O)OMe | Cl | Me | O |
| D-8019 | Me | CH₂CH₂ | H | *c*-Pr | CH₂N(Me)C(=O)OMe | Cl | Me | O |
| D-8020 | H | single bond | Me | *c*-Pr | CH₂N(Me)C(=O)OMe | Me | Me | O |
| D-8021 | Me | single bond | H | *c*-Pr | CH₂N(Me)C(=O)OMe | Me | Me | O |
| D-8022 | Me(R) | single bond | H | *c*-Pr | CH₂N(Me)C(=O)OMe | Me | Me | O |
| D-8023 | Me(S) | single bond | H | *c*-Pr | CH₂N(Me)C(=O)OMe | Me | Me | O |
| D-8024 | H | CH₂ | Me | *c*-Pr | CH₂N(Me)C(=O)OMe | Me | Me | O |
| D-8025 | Me | CH₂ | H | *c*-Pr | CH₂N(Me)C(=O)OMe | Me | Me | O |
| 0-8026 | H | CH(Me) | H | *c*-Pr | CH₂N(Me)C(=O)OMe | Me | Me | O |
| D-8027 | H | CH₂CH₂ | Me | *c*-Pr | CH₂N(Me)C(=O)OMe | Me | Me | O |
| D-8028 | Me | CH₂CH₂ | H | *c*-Pr | CH₂N(Me)C(=O)OMe | Me | Me | O |
| 0-8029 | H | single bond | Me | *c*-Pr | C(=O)OCH₂CH=CH₂ | Cl | CH₂OMe | O |
| D-8030 | Me | single bond | H | *c*-Pr | C(=O)OCH₂CH=CH₂ | Cl | CH₂OMe | O |
| D-8031 | Me(R) | single bond | H | *c*-Pr | C(=O)OCH₂CH=CH₂ | Cl | CH₂OMe | O |
| D-8032 | Me(S) | single bond | H | *c*-Pr | C(=O)OCH₂CH=CH₂ | Cl | CH₂OMe | O |
| D-8033 | H | CH₂ | Me | *c*-Pr | C(=O)OCH₂CH=CH₂ | Cl | CH₂OMe | O |
| D-8034 | Me | CH₂ | H | *c*-Pr | C(=O)OCH₂CH=CH₂ | Cl | CH₂OMe | O |
| D-8035 | H | CH(Me) | H | *c*-Pr | C(=O)OCH₂CH=CH₂ | Cl | CH₂OMe | O |
| D-8036 | H | CH₂CH₂ | Me | *c*-Pr | C(=O)OCH₂CH=CH₂ | Cl | CH₂OMe | O |
| D-8037 | Me | CH₂CH₂ | H | *c*-Pr | C(=O)OCH₂CH=CH₂ | Cl | CH₂OMe | O |
| 0-8038 | H | single bond | Me | *c*-Pr | C(=O)OCH₂CH=CH₂ | Me | CH₂OMe | O |
| D-8039 | Me | single bond | H | *c*-Pr | C(=O)OCH₂CH=CH₂ | Me | CH₂OMe | O |
| D-8040 | Me(R) | single bond | H | *c*-Pr | C(=O)OCH₂CH=CH₂ | Me | CH₂OMe | O |
| D-8041 | Me(S) | single bond | H | *c*-Pr | C(=O)OCH₂CH=CH₂ | Me | CH₂OMe | O |
| D-8042 | H | CH₂ | Me | *c*-Pr | C(=O)OCH₂CH=CH₂ | Me | CH₂OMe | O |
| D-8043 | Me | CH₂ | H | *c*-Pr | C(=O)OCH₂CH=CH₂ | Me | CH₂OMe | O |
| D-8044 | H | CH(Me) | H | *c*-Pr | C(=O)OCH₂CH=CH₂ | Me | CH₂OMe | O |
| 0-8045 | H | CH₂CH₂ | Me | *c*-Pr | C(=O)OCH₂CH=CH₂ | Me | CH₂OMe | O |
| D-8046 | Me | CH₂CH₂ | H | *c*-Pr | C(=O)OCH₂CH=CH₂ | Me | CH₂OMe | O |
| D-8047 | H | single bond | Me | *c*-Pr | C(=O)OCH₂CCl₃ | Cl | CH₂OMe | O |
| D-8048 | Me | single bond | H | *c*-Pr | C(=O)OCH₂CCl₃ | Cl | CH₂OMe | O |
| D-8049 | Me(R) | single bond | H | *c*-Pr | C(=O)OCH₂CCl₃ | Cl | CH₂OMe | O |
| D-8050 | Me(S) | single bond | H | *c*-Pr | C(=O)OCH₂CCl₃ | Cl | CH₂OMe | O |
| 0-8051 | H | CH₂ | Me | *c*-Pr | C(=O)OCH₂CCl₃ | Cl | CH₂OMe | O |
| D-8052 | Me | CH₂ | H | *c*-Pr | C(=O)OCH₂CCl₃ | Cl | CH₂OMe | O |
| D-8053 | H | CH(Me) | H | *c*-Pr | C(=O)OCH₂CCl₃ | Cl | CH₂OMe | O |
| 0-8054 | H | CH₂CH₂ | Me | *c*-Pr | C(=O)OCH₂CCl₃ | Cl | CH₂OMe | O |
| D-8055 | Me | CH₂CH₂ | H | *c*-Pr | C(=O)OCH₂CCl₃ | Cl | CH₂OMe | O |
| 0-8056 | H | single bond | Me | *c*-Pr | C(=O)OCH₂CCl₃ | Me | CH₂OMe | O |
| D-8057 | Me | single bond | H | *c*-Pr | C(=O)OCH₂CCl₃ | Me | CH₂OMe | O |
| D-8058 | Me(R) | single bond | H | *c*-Pr | C(=O)OCH₂CCl₃ | Me | CH₂OMe | O |

**[Table 829]**

| Compound Number | Y | W | z | R¹ | R² | R³ | R¹⁰ | A |
|---|---|---|---|---|---|---|---|---|
| D-8059 | Me(S) | single bond | H | *c*-Pr | C(=O)OCH₂CCl₃ | Me | CH₂OMe | O |
| D-8060 | H | CH₂ | Me | *c*-Pr | C(=O)OCH₂CCl₃ | Me | CH₂OMe | O |
| D-8061 | Me | CH₂ | H | *c*-Pr | C(=O)OCH₂CCl₃ | Me | CH₂OMe | O |
| D-8062 | H | CH(Me) | H | *c*-Pr | C(=O)OCH₂CCl₃ | Me | CH₂OMe | O |
| D-8063 | H | CH₂CH₂ | Me | *c*-Pr | C(=O)OCH₂CCl₃ | Me | CH₂OMe | O |
| D-8064 | Me | CH₂CH₂ | H | *c*-Pr | C(=O)OCH₂CCl₃ | Me | CH₂OMe | O |
| D-8065 | H | single bond | Me | *c*-Pr | C(=O)N(Me)₂ | Cl | CH₂OMe | O |
| D-8066 | Me | single bond | H | *c*-Pr | C(=O)N(Me)₂ | Cl | CH₂OMe | O |
| D-8067 | Me(R) | single bond | H | *c*-Pr | C(=O)N(Me)₂ | Cl | CH₂OMe | O |
| D-8068 | Me(S) | single bond | H | *c*-Pr | C(=O)N(Me)₂ | Cl | CH₂OMe | O |
| D-8069 | H | CH₂ | Me | *c*-Pr | C(=O)N(Me)₂ | Cl | CH₂OMe | O |
| D-8070 | Me | CH₂ | H | *c*-Pr | C(=O)N(Me)₂ | Cl | CH₂OMe | O |
| D-8071 | H | CH(Me) | H | *c*-Pr | C(=O)N(Me)₂ | Cl | CH₂OMe | O |
| D-8072 | H | CH₂CH₂ | Me | *c*-Pr | C(=O)N(Me)₂ | Cl | CH₂OMe | O |
| D-8073 | Me | CH₂CH₂ | H | *c*-Pr | C(=O)N(Me)₂ | Cl | CH₂OMe | O |
| D-8074 | H | single bond | Me | *c*-Pr | C(=O)N(Me)₂ | Me | CH₂OMe | O |
| D-8075 | Me | single bond | H | *c*-Pr | C(=O)N(Me)₂ | Me | CH₂OMe | O |
| D-8076 | Me(R) | single bond | H | *c*-Pr | C(=O)N(Me)₂ | Me | CH₂OMe | O |
| D-8077 | Me(S) | single bond | H | *c*-Pr | C(=O)N(Me)₂ | Me | CH₂OMe | O |
| D-8078 | H | CH₂ | Me | *c*-Pr | C(=O)N(Me)₂ | Me | CH₂OMe | O |
| D-8079 | Me | CH₂ | H | *c*-Pr | C(=O)N(Me)₂ | Me | CH₂OMe | O |
| D-8080 | H | CH(Me) | H | *c*-Pr | C(=O)N(Me)₂ | Me | CH₂OMe | O |
| D-8081 | H | CH₂CH₂ | Me | *c*-Pr | C(=O)N(Me)₂ | Me | CH₂OMe | O |
| D-8082 | Me | CH₂CH₂ | H | *c*-Pr | C(=O)N(Me)₂ | Me | CH₂OMe | O |
| D-8083 | H | single bond | Me | *c*-Pr | CH₂OC(=O)Me | Cl | CH₂OMe | O |
| D-8084 | Me | single bond | H | *c*-Pr | CH₂OC(=O)Me | Cl | CH₂OMe | O |
| D-8085 | Me(R) | single bond | H | *c*-Pr | CH₂OC(=O)Me | Cl | CH₂OMe | O |
| D-8086 | Me(S) | single bond | H | *c*-Pr | CH₂OC(=O)Me | Cl | CH₂OMe | O |
| D-8087 | H | CH₂ | Me | *c*-Pr | CH₂OC(=O)Me | Cl | CH₂OMe | O |
| D-8088 | Me | CH₂ | H | *c*-Pr | CH₂OC(=O)Me | Cl | CH₂OMe | O |
| D-8089 | H | CH(Me) | H | *c*-Pr | CH₂OC(=O)Me | Cl | CH₂OMe | O |
| D-8090 | H | CH₂CH₂ | Me | *c*-Pr | CH₂OC(=O)Me | Cl | CH₂OMe | O |
| D-8091 | Me | CH₂CH₂ | H | *c*-Pr | CH₂OC(=O)Me | Cl | CH₂OMe | O |
| D-8092 | H | single bond | Me | *c*-Pr | CH₂OC(=O)Me | Me | CH₂OMe | O |
| D-8093 | Me | single bond | H | *c*-Pr | CH₂OC(=O)Me | Me | CH₂OMe | O |
| D-8094 | Me(R) | single bond | H | *c*-Pr | CH₂OC(=O)Me | Me | CH₂OMe | O |
| D-8095 | Me(S) | single bond | H | *c*-Pr | CH₂OC(=O)Me | Me | CH₂OMe | O |
| D-8096 | H | CH₂ | Me | *c*-Pr | CH₂OC(=O)Me | Me | CH₂OMe | O |
| D-8097 | Me | CH₂ | H | *c*-Pr | CH₂OC(=O)Me | Me | CH₂OMe | O |
| D-8098 | H | CH(Me) | H | *c*-Pr | CH₂OC(=O)Me | Me | CH₂OMe | O |
| D-8099 | H | CH₂CH₂ | Me | *c*-Pr | CH₂OC(=O)Me | Me | CH₂OMe | O |
| D-8100 | Me | CH₂CH₂ | H | *c*-Pr | CH₂OC(=O)Me | Me | CH₂OMe | O |
| D-8101 | H | single bond | Me | *c*-Pr | CH₂OC (=O)(*i*-Pr) | Cl | CH₂OMe | O |
| D-8102 | Me | single bond | H | *c*-Pr | CH₂OC(=O)(*i*-Pr) | Cl | CH₂OMe | O |

**[Table 830]**

| Compound Number | Y | W | z | R¹ | R² | R³ | R¹⁰ | A |
|---|---|---|---|---|---|---|---|---|
| D-8103 | Me(R) | single bond | H | *c*-Pr | CH₂OC (=O)(*i*-Pr) | Cl | CH₂OMe | O |
| D-8104 | Me(S) | single bond | H | *c*-Pr | CH₂OC (=O)(*i*-Pr) | Cl | CH₂OMe | O |
| D-8105 | H | CH₂ | Me | *c*-Pr | CH₂OC(=O)(*i*-Pr) | Cl | CH₂OMe | O |
| D-8106 | Me | CH₂ | H | *c*-Pr | CH₂OC(=O)(*i*-Pr) | Cl | CH₂OMe | O |
| D-8107 | H | CH(Me) | H | *c*-Pr | CH₂OC(=O)(*i*-Pr) | Cl | CH₂OMe | O |
| D-8108 | H | CH₂CH₂ | Me | *c*-Pr | CH₂OC(=O)(*i*-Pr) | Cl | CH₂OMe | O |
| D-8109 | Me | CH₂CH₂ | H | *c*-Pr | CH₂OC(=O)(*i*-Pr) | Cl | CH₂OMe | O |
| D-8110 | H | single bond | Me | *c*-Pr | CH₂OC (=O)(*i*-Pr) | Me | CH₂OMe | O |
| D-8111 | Me | single bond | H | *c*-Pr | CH₂OC (=O)(*i*-Pr) | Me | CH₂OMe | O |
| D-8112 | Me(R) | single bond | H | *c*-Pr | CH₂OC(=O)(*i*-Pr) | Me | CH₂OMe | O |
| D-8113 | Me(S) | single bond | H | *c*-Pr | CH₂OC(=O)(*i*-Pr) | Me | CH₂OMe | O |
| D-8114 | H | CH₂ | Me | *c*-Pr | CH₂OC (=O)(*i*-Pr) | Me | CH₂OMe | O |
| D-8115 | Me | CH₂ | H | *c*-Pr | CH₂OC(=O)(*i*-Pr) | Me | CH₂OMe | O |
| D-8116 | H | CH(Me) | H | *c*-Pr | CH₂OC(=O)(*i*-Pr) | Me | CH₂OMe | O |
| D-8117 | H | CH₂CH₂ | Me | *c*-Pr | CH₂OC(=O)(*i*-Pr) | Me | CH₂OMe | O |
| D-8118 | Me | CH₂CH₂ | H | *c*-Pr | CH₂OC(=O)(*i*-Pr) | Me | CH₂OMe | O |
| D-8119 | H | single bond | Me | *c*-Pr | CH₂OC(=O)(*t*-Bu) | Cl | CH₂OMe | O |
| D-8120 | Me | single bond | H | *c*-Pr | CH₂OC(=O)(*t*-Bu) | Cl | CH₂OMe | O |
| D-8121 | Me(R) | single bond | H | *c*-Pr | CH₂OC(=O)(*t*-Bu) | Cl | CH₂OMe | O |
| D-8122 | Me(S) | single bond | H | *c*-Pr | CH₂OC(=O)(*t*-Bu) | Cl | CH₂OMe | O |
| D-8123 | H | CH₂ | Me | *c*-Pr | CH₂OC(=O)(*t*-Bu) | Cl | CH₂OMe | O |
| D-8124 | Me | CH₂ | H | *c*-Pr | CH₂OC(=O)(*t*-Bu) | Cl | CH₂OMe | O |
| D-8125 | H | CH(Me) | H | *c*-Pr | CH₂OC(=O)(*t*-Bu) | Cl | CH₂OMe | O |
| D-8126 | H | CH₂CH₂ | Me | *c*-Pr | CH₂OC(=O)(*t*-Bu) | Cl | CH₂OMe | O |
| D-8127 | Me | CH₂CH₂ | H | *c*-Pr | CH₂OC(=O)(*t*-Bu) | Cl | CH₂OMe | O |
| D-8128 | H | single bond | Me | *c*-Pr | CH₂OC(=O)(*t*-Bu) | Me | CH₂OMe | O |
| D-8129 | Me | single bond | H | *c*-Pr | CH₂OC(=O)(*t*-Bu) | Me | CH₂OMe | O |
| D-8130 | Me(R) | single bond | H | *c*-Pr | CH₂OC(=O)(*t*-Bu) | Me | CH₂OMe | O |
| D-8131 | Me(S) | single bond | H | *c*-Pr | CH₂OC(=O)(*t*-Bu) | Me | CH₂OMe | O |
| D-8132 | H | CH₂ | Me | *c*-Pr | CH₂OC(=O)(*t*-Bu) | Me | CH₂OMe | O |
| D-8133 | Me | CH₂ | H | *c*-Pr | CH₂OC(=O)(*t*-Bu) | Me | CH₂OMe | O |
| D-8134 | H | CH(Me) | H | *c*-Pr | CH₂OC(=O)(*t*-Bu) | Me | CH₂OMe | O |
| D-8135 | H | CH₂CH₂ | Me | *c*-Pr | CH₂OC(=O)(*t*-Bu) | Me | CH₂OMe | O |
| D-8136 | Me | CH₂CH₂ | H | *c*-Pr | CH₂OC(=O)(*t*-Bu) | Me | CH₂OMe | O |
| D-8137 | H | single bond | Me | *c*-Pr | CH₂OC(=O)OEt | Cl | CH₂OMe | O |
| D-8138 | Me | single bond | H | *c*-Pr | CH₂OC(=O)OEt | Cl | CH₂OMe | O |
| D-8139 | Me(R) | single bond | H | *c*-Pr | CH₂OC(=O)OEt | Cl | CH₂OMe | O |
| D-8140 | Me(S) | single bond | H | *c*-Pr | CH₂OC(=O)OEt | Cl | CH₂OMe | O |
| D-8141 | H | CH₂ | Me | *c*-Pr | CH₂OC(=O)OEt | Cl | CH₂OMe | O |
| D-8142 | Me | CH₂ | H | *c*-Pr | CH₂OC(=O)OEt | Cl | CH₂OMe | O |
| D-8143 | H | CH(Me) | H | *c*-Pr | CH₂OC(=O)OEt | Cl | CH₂OMe | O |
| D-8144 | H | CH₂CH₂ | Me | *c*-Pr | CH₂OC(=O)OEt | Cl | CH₂OMe | O |
| D-8145 | Me | CH₂CH₂ | H | *c*-Pr | CH₂OC(=O)OEt | Cl | CH₂OMe | O |
| D-8146 | H | single bond | Me | *c*-Pr | CH₂OC(=O)OEt | Me | CH₂OMe | O |

**[Table 831]**

| Compound Number | Y | W | z | R¹ | R² | R³ | R¹⁰ | A |
|---|---|---|---|---|---|---|---|---|
| D-8147 | Me | single bond | H | *c*-Pr | CH₂OC(=O)OEt | Me | CH₂OMe | O |
| D-8148 | Me(R) | single bond | H | *c*-Pr | CH₂OC(=O)OEt | Me | CH₂OMe | O |
| D-8149 | Me(S) | single bond | H | *c*-Pr | CH₂OC(=O)OEt | Me | CH₂OMe | O |
| D-8150 | H | CH₂ | Me | *c*-Pr | CH₂OC(=O)OEt | Me | CH₂OMe | O |
| D-8151 | Me | CH₂ | H | *c*-Pr | CH₂OC(=O)OEt | Me | CH₂OMe | O |
| D-8152 | H | CH(Me) | H | *c*-Pr | CH₂OC(=O)OEt | Me | CH₂OMe | O |
| D-8153 | H | CH₂CH₂ | Me | *c*-Pr | CH₂OC(=O)OEt | Me | CH₂OMe | O |
| D-8154 | Me | CH₂CH₂ | H | *c*-Pr | CH₂OC(=O)OEt | Me | CH₂OMe | O |
| D-8155 | H | single bond | Me | *c*-Pr | CH₂OC(=O)O(*i*-Pr) | Cl | CH₂OMe | O |
| D-8156 | Me | single bond | H | *c*-Pr | CH₂OC(=O)O(*i*-Pr) | Cl | CH₂OMe | O |
| D-8157 | Me(R) | single bond | H | *c*-Pr | CH₂OC(=O)O(*i*-Pr) | Cl | CH₂OMe | O |
| D-8158 | Me(S) | single bond | H | *c*-Pr | CH₂OC(=O)O(*i*-Pr) | Cl | CH₂OMe | O |
| D-8159 | H | CH₂ | Me | *c*-Pr | CH₂OC(=O)O(*i*-Pr) | Cl | CH₂OMe | O |
| D-8160 | Me | CH₂ | H | *c*-Pr | CH₂OC(=O)O(*i*-Pr) | Cl | CH₂OMe | O |
| D-8161 | H | CH(Me) | H | *c*-Pr | CH₂OC(=O)O(*i*-Pr) | Cl | CH₂OMe | O |
| D-8162 | H | CH₂CH₂ | Me | *c*-Pr | CH₂OC(=O)O(*i*-Pr) | Cl | CH₂OMe | O |
| D-8163 | Me | CH₂CH₂ | H | *c*-Pr | CH₂OC(=O)O(*i*-Pr) | Cl | CH₂OMe | O |
| D-8164 | H | single bond | Me | *c*-Pr | CH₂OC(=O)O(*i*-Pr) | Me | CH₂OMe | O |
| D-8165 | Me | single bond | H | *c*-Pr | CH₂OC(=O)O(*i*-Pr) | Me | CH₂OMe | O |
| D-8166 | Me(R) | single bond | H | *c*-Pr | CH₂OC(=O)O(*i*-Pr) | Me | CH₂OMe | O |
| D-8167 | Me(S) | single bond | H | *c*-Pr | CH₂OC(=O)O(*i*-Pr) | Me | CH₂OMe | O |
| D-8168 | H | CH₂ | Me | *c*-Pr | CH₂OC(=O)O(*i*-Pr) | Me | CH₂OMe | O |
| D-8169 | Me | CH₂ | H | *c*-Pr | CH₂OC(=O)O(*i*-Pr) | Me | CH₂OMe | O |
| D-8170 | H | CH(Me) | H | *c*-Pr | CH₂OC(=O)O(*i*-Pr) | Me | CH₂OMe | O |
| D-8171 | H | CH₂CH₂ | Me | *c*-Pr | CH₂OC(=O)O(*i*-Pr) | Me | CH₂OMe | O |
| D-8172 | Me | CH₂CH₂ | H | *c*-Pr | CH₂OC(=O)O(*i*-Pr) | Me | CH₂OMe | O |
| D-8173 | H | single bond | Me | *c*-Pr | CH₂N(Me)C(=O)OMe | Cl | CH₂OMe | O |
| D-8174 | Me | single bond | H | *c*-Pr | CH₂N(Me)C(=O)OMe | Cl | CH₂OMe | O |
| D-8175 | Me(R) | single bond | H | *c*-Pr | CH₂N(Me)C(=O)OMe | Cl | CH₂OMe | O |
| D-8176 | Me(S) | single bond | H | *c*-Pr | CH₂N(Me)C(=O)OMe | Cl | CH₂OMe | O |
| D-8177 | H | CH₂ | Me | *c*-Pr | CH₂N(Me)C(=O)OMe | Cl | CH₂OMe | O |
| D-8178 | Me | CH₂ | H | *c*-Pr | CH₂N(Me)C(=O)OMe | Cl | CH₂OMe | O |
| D-8179 | H | CH(Me) | H | *c*-Pr | CH₂N(Me)C(=O)OMe | Cl | CH₂OMe | O |
| D-8180 | H | CH₂CH₂ | Me | *c*-Pr | CH₂N(Me)C(=O)OMe | Cl | CH₂OMe | O |
| D-8181 | Me | CH₂CH₂ | H | *c*-Pr | CH₂N(Me)C(=O)OMe | Cl | CH₂OMe | O |
| D-8182 | H | single bond | Me | *c*-Pr | CH₂N(Me)C(=O)OMe | Me | CH₂OMe | O |
| D-8183 | Me | single bond | H | *c*-Pr | CH₂N(Me)C(=O)OMe | Me | CH₂OMe | O |
| D-8184 | Me(R) | single bond | H | *c*-Pr | CH₂N(Me)C(=O)OMe | Me | CH₂OMe | O |
| D-8185 | Me(S) | single bond | H | *c*-Pr | CH₂N(Me)C(=O)OMe | Me | CH₂OMe | O |
| D-8186 | H | CH₂ | Me | *c*-Pr | CH₂N(Me)C(=O)OMe | Me | CH₂OMe | O |
| D-8187 | Me | CH₂ | H | *c*-Pr | CH₂N(Me)C(=O)OMe | Me | CH₂OMe | O |
| D-8188 | H | CH(Me) | H | *c*-Pr | CH₂N(Me)C(=O)OMe | Me | CH₂OMe | O |
| D-8189 | H | CH₂CH₂ | Me | *c*-Pr | CH₂N(Me)C(=O)OMe | Me | CH₂OMe | O |
| D-8190 | Me | CH₂CH₂ | H | *c*-Pr | CH₂N(Me)C(=O)OMe | Me | CH₂OMe | O |

**[Table 832]**

| Compound Number | Y | W | z | R¹ | R² | R³ | R¹⁰ | A |
|---|---|---|---|---|---|---|---|---|
| D-8191 | H | single bond | Me | *c*-Pr | C(=O)OCH₂CH=CH₂ | Cl | CH₂CN | O |
| D-8192 | Me | single bond | H | *c*-Pr | C(=O)OCH₂CH=CH₂ | Cl | CH₂CN | O |
| D-8193 | Me(R) | single bond | H | *c*-Pr | C(=O)OCH₂CH=CH₂ | Cl | CH₂CN | O |
| D-8194 | Me(S) | single bond | H | *c*-Pr | C(=O)OCH₂CH=CH₂ | Cl | CH₂CN | O |
| D-8195 | H | CH₂ | Me | *c*-Pr | C(=O)OCH₂CH=CH₂ | Cl | CH₂CN | O |
| D-8196 | Me | CH₂ | H | *c*-Pr | C(=O)OCH₂CH=CH₂ | Cl | CH₂CN | O |
| D-8197 | H | CH(Me) | H | *c*-Pr | C(=O)OCH₂CH=CH₂ | Cl | CH₂CN | O |
| D-8198 | H | CH₂CH₂ | Me | *c*-Pr | C(=O)OCH₂CH=CH₂ | Cl | CH₂CN | O |
| D-8199 | Me | CH₂CH₂ | H | *c*-Pr | C(=O)OCH₂CH=CH₂ | Cl | CH₂CN | O |
| D-8200 | H | single bond | Me | *c*-Pr | C(=O)OCH₂CH=CH₂ | Me | CH₂CN | O |
| D-8201 | Me | single bond | H | *c*-Pr | C(=O)OCH₂CH=CH₂ | Me | CH₂CN | O |
| D-8202 | Me(R) | single bond | H | *c*-Pr | C(=O)OCH₂CH=CH₂ | Me | CH₂CN | O |
| D-8203 | Me(S) | single bond | H | *c*-Pr | C(=O)OCH₂CH=CH₂ | Me | CH₂CN | O |
| D-8204 | H | CH₂ | Me | *c*-Pr | C(=O)OCH₂CH=CH₂ | Me | CH₂CN | O |
| D-8205 | Me | CH₂ | H | *c*-Pr | C(=O)OCH₂CH=CH₂ | Me | CH₂CN | O |
| D-8206 | H | CH(Me) | H | *c*-Pr | C(=O)OCH₂CH=CH₂ | Me | CH₂CN | O |
| D-8207 | H | CH₂CH₂ | Me | *c*-Pr | C(=O)OCH₂CH=CH₂ | Me | CH₂CN | O |
| D-8208 | Me | CH₂CH₂ | H | *c*-Pr | C(=O)OCH₂CH=CH₂ | Me | CH₂CN | O |
| D-8209 | H | single bond | Me | *c*-Pr | C(=O)OCH₂CCl₃ | Cl | CH₂CN | O |
| D-8210 | Me | single bond | H | *c*-Pr | C(=O)OCH₂CCl₃ | Cl | CH₂CN | O |
| D-8211 | Me(R) | single bond | H | *c*-Pr | C(=O)OCH₂CCl₃ | Cl | CH₂CN | O |
| D-8212 | Me(S) | single bond | H | *c*-Pr | C(=O)OCH₂CCl₃ | Cl | CH₂CN | O |
| D-8213 | H | CH₂ | Me | *c*-Pr | C(=O)OCH₂CCl₃ | Cl | CH₂CN | O |
| D-8214 | Me | CH₂ | H | *c*-Pr | C(=O)OCH₂CCl₃ | Cl | CH₂CN | O |
| D-8215 | H | CH(Me) | H | *c*-Pr | C(=O)OCH₂CCl₃ | Cl | CH₂CN | O |
| D-8216 | H | CH₂CH₂ | Me | *c*-Pr | C(=O)OCH₂CCl₃ | Cl | CH₂CN | O |
| D-8217 | Me | CH₂CH₂ | H | *c*-Pr | C(=O)OCH₂CCl₃ | Cl | CH₂CN | O |
| D-8218 | H | single bond | Me | *c*-Pr | C(=O)OCH₂CCl₃ | Me | CH₂CN | O |
| D-8219 | Me | single bond | H | *c*-Pr | C(=O)OCH₂CCl₃ | Me | CH₂CN | O |
| D-8220 | Me(R) | single bond | H | *c*-Pr | C(=O)OCH₂CCl₃ | Me | CH₂CN | O |
| D-8221 | Me(S) | single bond | H | *c*-Pr | C(=O)OCH₂CCl₃ | Me | CH₂CN | O |
| D-8222 | H | CH₂ | Me | *c*-Pr | C(=O)OCH₂CCl₃ | Me | CH₂CN | O |
| D-8223 | Me | CH₂ | H | *c*-Pr | C(=O)OCH₂CCl₃ | Me | CH₂CN | O |
| D-8224 | H | CH(Me) | H | *c*-Pr | C(=O)OCH₂CCl₃ | Me | CH₂CN | O |
| D-8225 | H | CH₂CH₂ | Me | *c*-Pr | C(=O)OCH₂CCl₃ | Me | CH₂CN | O |
| D-8226 | Me | CH₂CH₂ | H | *c*-Pr | C(=O)OCH₂CCl₃ | Me | CH₂CN | O |
| D-8227 | H | single bond | Me | *c*-Pr | C(=O)N(Me)₂ | Cl | CH₂CN | O |
| D-8228 | Me | single bond | H | *c*-Pr | C(=O)N(Me)₂ | Cl | CH₂CN | O |
| D-8229 | Me(R) | single bond | H | *c*-Pr | C(=O)N(Me)₂ | Cl | CH₂CN | O |
| D-8230 | Me(S) | single bond | H | *c*-Pr | C(=O)N(Me)₂ | Cl | CH₂CN | O |
| D-8231 | H | CH₂ | Me | *c*-Pr | C(=O)N(Me)₂ | Cl | CH₂CN | O |
| D-8232 | Me | CH₂ | H | *c*-Pr | C(=O)N(Me)₂ | Cl | CH₂CN | O |
| D-8233 | H | CH(Me) | H | *c*-Pr | C(=O)N(Me)₂ | Cl | CH₂CN | O |
| D-8234 | H | CH₂CH₂ | Me | *c*-Pr | C(=O)N(Me)₂ | Cl | CH₂CN | O |

**[Table 833]**

| Compound Number | Y | W | z | R¹ | R² | R³ | R¹⁰ | A |
|---|---|---|---|---|---|---|---|---|
| D-8235 | Me | CH₂CH₂ | H | *c*-Pr | C(=O)N(Me)₂ | Cl | CH₂CN | O |
| D-8236 | H | single bond | Me | *c*-Pr | C(=O)N(Me)₂ | Me | CH₂CN | O |
| D-8237 | Me | single bond | H | *c*-Pr | C(=O)N(Me)₂ | Me | CH₂CN | O |
| D-8238 | Me(R) | single bond | H | *c*-Pr | C(=O)N(Me)₂ | Me | CH₂CN | O |
| D-8239 | Me(S) | single bond | H | *c*-Pr | C(=O)N(Me)₂ | Me | CH₂CN | O |
| D-8240 | H | CH₂ | Me | *c*-Pr | C(=O)N(Me)₂ | Me | CH₂CN | O |
| D-8241 | Me | CH₂ | H | *c*-Pr | C(=O)N(Me)₂ | Me | CH₂CN | O |
| D-8242 | H | CH(Me) | H | *c*-Pr | C(=O)N(Me)₂ | Me | CH₂CN | O |
| D-8243 | H | CH₂CH₂ | Me | *c*-Pr | C(=O)N(Me)₂ | Me | CH₂CN | O |
| D-8244 | Me | CH₂CH₂ | H | *c*-Pr | C(=O)N(Me)₂ | Me | CH₂CN | O |
| D-8245 | H | single bond | Me | *c*-Pr | CH₂OC(=O)Me | Cl | CH₂CN | O |
| D-8246 | Me | single bond | H | *c*-Pr | CH₂OC(=O)Me | Cl | CH₂CN | O |
| D-8247 | Me(R) | single bond | H | *c*-Pr | CH₂OC(=O)Me | Cl | CH₂CN | O |
| D-8248 | Me(S) | single bond | H | *c*-Pr | CH₂OC(=O)Me | Cl | CH₂CN | O |
| D-8249 | H | CH₂ | Me | *c*-Pr | CH₂OC(=O)Me | Cl | CH₂CN | O |
| D-8250 | Me | CH₂ | H | *c*-Pr | CH₂OC(=O)Me | Cl | CH₂CN | O |
| D-8251 | H | CH(Me) | H | *c*-Pr | CH₂OC(=O)Me | Cl | CH₂CN | O |
| D-8252 | H | CH₂CH₂ | Me | *c*-Pr | CH₂OC(=O)Me | Cl | CH₂CN | O |
| D-8253 | Me | CH₂CH₂ | H | *c*-Pr | CH₂OC(=O)Me | Cl | CH₂CN | O |
| D-8254 | H | single bond | Me | *c*-Pr | CH₂OC(=O)Me | Me | CH₂CN | O |
| D-8255 | Me | single bond | H | *c*-Pr | CH₂OC(=O)Me | Me | CH₂CN | O |
| D-8256 | Me(R) | single bond | H | *c*-Pr | CH₂OC(=O)Me | Me | CH₂CN | O |
| D-8257 | Me(S) | single bond | H | *c*-Pr | CH₂OC(=O)Me | Me | CH₂CN | O |
| D-8258 | H | CH₂ | Me | *c*-Pr | CH₂OC(=O)Me | Me | CH₂CN | O |
| D-8259 | Me | CH₂ | H | *c*-Pr | CH₂OC(=O)Me | Me | CH₂CN | O |
| D-8260 | H | CH(Me) | H | *c*-Pr | CH₂OC(=O)Me | Me | CH₂CN | O |
| D-8261 | H | CH₂CH₂ | Me | *c*-Pr | CH₂OC(=O)Me | Me | CH₂CN | O |
| D-8262 | Me | CH₂CH₂ | H | *c*-Pr | CH₂OC(=O)Me | Me | CH₂CN | O |
| D-8263 | H | single bond | Me | *c*-Pr | CH₂OC (=O)(*i*-Pr) | Cl | CH₂CN | O |
| D-8264 | Me | single bond | H | *c*-Pr | CH₂OC (=O)(*i*-Pr) | Cl | CH₂CN | O |
| D-8265 | Me(R) | single bond | H | *c*-Pr | CH₂OC(=O)(*i*-Pr) | Cl | CH₂CN | O |
| D-8266 | Me(S) | single bond | H | *c*-Pr | CH₂OC (=O)(*i*-Pr) | Cl | CH₂CN | O |
| D-8267 | H | CH₂ | Me | *c*-Pr | CH₂OC (=O)(*i*-Pr) | Cl | CH₂CN | O |
| D-8268 | Me | CH₂ | H | *c*-Pr | CH₂OC(=O)(*i*-Pr) | Cl | CH₂CN | O |
| D-8269 | H | CH(Me) | H | *c*-Pr | CH₂OC(=O)(*i*-Pr) | Cl | CH₂CN | O |
| D-8270 | H | CH₂CH₂ | Me | *c*-Pr | CH₂OC (=O)(*i*-Pr) | Cl | CH₂CN | O |
| D-8271 | Me | CH₂CH₂ | H | *c*-Pr | CH₂OC (=O)(*i*-Pr) | Cl | CH₂CN | O |
| D-8272 | H | single bond | Me | *c*-Pr | CH₂OC(=O)(*i*-Pr) | Me | CH₂CN | O |
| D-8273 | Me | single bond | H | *c*-Pr | CH₂OC (=O)(*i*-Pr) | Me | CH₂CN | O |
| D-8274 | Me(R) | single bond | H | *c*-Pr | CH₂OC (=O)(*i*-Pr) | Me | CH₂CN | O |
| D-8275 | Me(S) | single bond | H | *c*-Pr | CH₂OC(=O)(*i*-Pr) | Me | CH₂CN | O |
| D-8276 | H | CH₂ | Me | *c*-Pr | CH₂OC(=O)(*i*-Pr) | Me | CH₂CN | O |
| D-8277 | Me | CH₂ | H | *c*-Pr | CH₂OC (=O)(*i*-Pr) | Me | CH₂CN | O |
| D-8278 | H | CH(Me) | H | *c*-Pr | CH₂OC(=O)(*i*-Pr) | Me | CH₂CN | O |

**[Table 834]**

| Compound Number | Y | W | Z | R¹ | R² | R³ | R¹⁰ | A |
|---|---|---|---|---|---|---|---|---|
| D-8279 | H | CH₂CH₂ | Me | *c*-Pr | CH₂OC (=O)(*i*-Pr) | Me | CH₂CN | O |
| D-8280 | Me | CH₂CH₂ | H | *c*-Pr | CH₂OC (=O)(*i*-Pr) | Me | CH₂CN | O |
| D-8281 | H | single bond | Me | *c*-Pr | CH₂OC(=O)(*t*-Bu) | Cl | CH₂CN | O |
| D-8282 | Me | single bond | H | *c*-Pr | CH₂OC(=O)(*t*-Bu) | Cl | CH₂CN | O |
| D-8283 | Me(R) | single bond | H | *c*-Pr | CH₂OC(=O)(*t*-Bu) | Cl | CH₂CN | O |
| D-8284 | Me(S) | single bond | H | *c*-Pr | CH₂OC(=O)(*t*-Bu) | Cl | CH₂CN | O |
| D-8285 | H | CH₂ | Me | *c*-Pr | CH₂OC(=O)(*t*-Bu) | Cl | CH₂CN | O |
| D-8286 | Me | CH₂ | H | *c*-Pr | CH₂OC(=O)(*t*-Bu) | Cl | CH₂CN | O |
| D-8287 | H | CH(Me) | H | *c*-Pr | CH₂OC(=O)(*t*-Bu) | Cl | CH₂CN | O |
| D-8288 | H | CH₂CH₂ | Me | *c*-Pr | CH₂OC(=O)(*t*-Bu) | Cl | CH₂CN | O |
| D-8289 | Me | CH₂CH₂ | H | *c*-Pr | CH₂OC(=O)(*t*-Bu) | Cl | CH₂CN | O |
| D-8290 | H | single bond | Me | *c*-Pr | CH₂OC(=O)(*t*-Bu) | Me | CH₂CN | O |
| D-8291 | Me | single bond | H | *c*-Pr | CH₂OC(=O)(*t*-Bu) | Me | CH₂CN | O |
| D-8292 | Me(R) | single bond | H | *c*-Pr | CH₂OC(=O)(*t*-Bu) | Me | CH₂CN | O |
| D-8293 | Me(S) | single bond | H | *c*-Pr | CH₂OC(=O)(*t*-Bu) | Me | CH₂CN | O |
| D-8294 | H | CH₂ | Me | *c*-Pr | CH₂OC(=O)(*t*-Bu) | Me | CH₂CN | O |
| D-8295 | Me | CH₂ | H | *c*-Pr | CH₂OC(=O)(*t*-Bu) | Me | CH₂CN | O |
| D-8296 | H | CH(Me) | H | *c*-Pr | CH₂OC(=O)(*t*-Bu) | Me | CH₂CN | O |
| D-8297 | H | CH₂CH₂ | Me | *c*-Pr | CH₂OC(=O)(*t*-Bu) | Me | CH₂CN | O |
| D-8298 | Me | CH₂CH₂ | H | *c*-Pr | CH₂OC(=O)(*t*-Bu) | Me | CH₂CN | O |
| D-8299 | H | single bond | Me | *c*-Pr | CH₂OC(=O)OEt | Cl | CH₂CN | O |
| D-8300 | Me | single bond | H | *c*-Pr | CH₂OC(=O)OEt | Cl | CH₂CN | O |
| D-8301 | Me(R) | single bond | H | *c*-Pr | CH₂OC(=O)OEt | Cl | CH₂CN | O |
| D-8302 | Me(S) | single bond | H | *c*-Pr | CH₂OC(=O)OEt | Cl | CH₂CN | O |
| D-8303 | H | CH₂ | Me | *c*-Pr | CH₂OC(=O)OEt | Cl | CH₂CN | O |
| D-8304 | Me | CH₂ | H | *c*-Pr | CH₂OC(=O)OEt | Cl | CH₂CN | O |
| D-8305 | H | CH(Me) | H | *c*-Pr | CH₂OC(=O)OEt | Cl | CH₂CN | O |
| D-8306 | H | CH₂CH₂ | Me | *c*-Pr | CH₂OC(=O)OEt | Cl | CH₂CN | O |
| D-8307 | Me | CH₂CH₂ | H | *c*-Pr | CH₂OC(=O)OEt | Cl | CH₂CN | O |
| D-8308 | H | single bond | Me | *c*-Pr | CH₂OC(=O)OEt | Me | CH₂CN | O |
| D-8309 | Me | single bond | H | *c*-Pr | CH₂OC(=O)OEt | Me | CH₂CN | O |
| D-8310 | Me(R) | single bond | H | *c*-Pr | CH₂OC(=O)OEt | Me | CH₂CN | O |
| D-8311 | Me(S) | single bond | H | *c*-Pr | CH₂OC(=O)OEt | Me | CH₂CN | O |
| D-8312 | H | CH₂ | Me | *c*-Pr | CH₂OC(=O)OEt | Me | CH₂CN | O |
| D-8313 | Me | CH₂ | H | *c*-Pr | CH₂OC(=O)OEt | Me | CH₂CN | O |
| D-8314 | H | CH(Me) | H | *c*-Pr | CH₂OC(=O)OEt | Me | CH₂CN | O |
| D-8315 | H | CH₂CH₂ | Me | *c*-Pr | CH₂OC(=O)OEt | Me | CH₂CN | O |
| D-8316 | Me | CH₂CH₂ | H | *c*-Pr | CH₂OC(=O)OEt | Me | CH₂CN | O |
| D-8317 | H | single bond | Me | *c*-Pr | CH₂OC(=O)O(*i*-Pr) | Cl | CH₂CN | O |
| D-8318 | Me | single bond | H | *c*-Pr | CH₂OC(=O)O(*i*-Pr) | Cl | CH₂CN | O |
| D-8319 | Me(R) | single bond | H | *c*-Pr | CH₂OC(=O)O(*i*-Pr) | Cl | CH₂CN | O |
| D-8320 | Me(S) | single bond | H | *c*-Pr | CH₂OC(=O)O(*i*-Pr) | Cl | CH₂CN | O |
| D-8321 | H | CH₂ | Me | *c*-Pr | CH₂OC(=O)O(*i*-Pr) | Cl | CH₂CN | O |
| D-8322 | Me | CH₂ | H | *c*-Pr | CH₂OC(=O)O(*i*-Pr) | Cl | CH₂CN | O |

**[Table 835]**

| Compound Number | Y | W | Z | R¹ | R² | R³ | R¹⁰ | A |
|---|---|---|---|---|---|---|---|---|
| D-8323 | H | CH(Me) | H | *c*-Pr | CH₂OC(=O)O(*i*-Pr) | Cl | CH₂CN | O |
| D-8324 | H | CH₂CH₂ | Me | *c*-Pr | CH₂OC(=O)O(*i*-Pr) | Cl | CH₂CN | O |
| D-8325 | Me | CH₂CH₂ | H | *c*-Pr | CH₂OC(=O)O(*i*-Pr) | Cl | CH₂CN | O |
| D-8326 | H | single bond | Me | *c*-Pr | CH₂OC(=O)O(*i*-Pr) | Me | CH₂CN | O |
| D-8327 | Me | single bond | H | *c*-Pr | CH₂OC(=O)O(*i*-Pr) | Me | CH₂CN | O |
| D-8328 | Me(R) | single bond | H | *c*-Pr | CH₂OC(=O)O(*i*-Pr) | Me | CH₂CN | O |
| D-8329 | Me(S) | single bond | H | *c*-Pr | CH₂OC(=O)O(*i*-Pr) | Me | CH₂CN | O |
| D-8330 | H | CH₂ | Me | *c*-Pr | CH₂OC(=O)O(*i*-Pr) | Me | CH₂CN | O |
| D-8331 | Me | CH₂ | H | *c*-Pr | CH₂OC(=O)O(*i*-Pr) | Me | CH₂CN | O |
| D-8332 | H | CH(Me) | H | *c*-Pr | CH₂OC(=O)O(*i*-Pr) | Me | CH₂CN | O |
| D-8333 | H | CH₂CH₂ | Me | *c*-Pr | CH₂OC(=O)O(*i*-Pr) | Me | CH₂CN | O |
| D-8334 | Me | CH₂CH₂ | H | *c*-Pr | CH₂OC(=O)O(*i*-Pr) | Me | CH₂CN | O |
| D-8335 | H | single bond | Me | *c*-Pr | CH₂N(Me)C(=O)OMe | Cl | CH₂CN | O |
| D-8336 | Me | single bond | H | *c*-Pr | CH₂N(Me)C(=O)OMe | Cl | CH₂CN | O |
| D-8337 | Me(R) | single bond | H | *c*-Pr | CH₂N(Me)C(=O)OMe | Cl | CH₂CN | O |
| D-8338 | Me(S) | single bond | H | *c*-Pr | CH₂N(Me)C(=O)OMe | Cl | CH₂CN | O |
| D-8339 | H | CH₂ | Me | *c*-Pr | CH₂N(Me)C(=O)OMe | Cl | CH₂CN | O |
| D-8340 | Me | CH₂ | H | *c*-Pr | CH₂N(Me)C(=O)OMe | Cl | CH₂CN | O |
| D-8341 | H | CH(Me) | H | *c*-Pr | CH₂N(Me)C(=O)OMe | Cl | CH₂CN | O |
| D-8342 | H | CH₂CH₂ | Me | *c*-Pr | CH₂N(Me)C(=O)OMe | Cl | CH₂CN | O |
| D-8343 | Me | CH₂CH₂ | H | *c*-Pr | CH₂N(Me)C(=O)OMe | Cl | CH₂CN | O |
| D-8344 | H | single bond | Me | *c*-Pr | CH₂N(Me)C(=O)OMe | Me | CH₂CN | O |
| D-8345 | Me | single bond | H | *c*-Pr | CH₂N(Me)C(=O)OMe | Me | CH₂CN | O |
| D-8346 | Me(R) | single bond | H | *c*-Pr | CH₂N(Me)C(=O)OMe | Me | CH₂CN | O |
| D-8347 | Me(S) | single bond | H | *c*-Pr | CH₂N(Me)C(=O)OMe | Me | CH₂CN | O |
| D-8348 | H | CH₂ | Me | *c*-Pr | CH₂N(Me)C(=O)OMe | Me | CH₂CN | O |
| D-8349 | Me | CH₂ | H | *c*-Pr | CH₂N(Me)C(=O)OMe | Me | CH₂CN | O |
| D-8350 | H | CH(Me) | H | *c*-Pr | CH₂N(Me)C(=O)OMe | Me | CH₂CN | O |
| D-8351 | H | CH₂CH₂ | Me | *c*-Pr | CH₂N(Me)C(=O)OMe | Me | CH₂CN | O |
| D-8352 | Me | CH₂CH₂ | H | *c*-Pr | CH₂N(Me)C(=O)OMe | Me | CH₂CN | O |
| D-8353 | H | single bond | Me | *c*-Pr | C(=O)OCH₂CH=CH₂ | Cl | C(=O)Me | O |
| D-8354 | Me | single bond | H | *c*-Pr | C(=O)OCH₂CH=CH₂ | Cl | C(=O)Me | O |
| D-8355 | Me(R) | single bond | H | *c*-Pr | C(=O)OCH₂CH=CH₂ | Cl | C(=O)Me | O |
| D-8356 | Me(S) | single bond | H | *c*-Pr | C(=O)OCH₂CH=CH₂ | Cl | C(=O)Me | O |
| D-8357 | H | CH₂ | Me | *c*-Pr | C(=O)OCH₂CH=CH₂ | Cl | C(=O)Me | O |
| D-8358 | Me | CH₂ | H | *c*-Pr | C(=O)OCH₂CH=CH₂ | Cl | C(=O)Me | O |
| D-8359 | H | CH(Me) | H | *c*-Pr | C(=O)OCH₂CH=CH₂ | Cl | C(=O)Me | O |
| D-8360 | H | CH₂CH₂ | Me | *c*-Pr | C(=O)OCH₂CH=CH₂ | Cl | C(=O)Me | O |
| D-8361 | Me | CH₂CH₂ | H | *c*-Pr | C(=O)OCH₂CH=CH₂ | Cl | C(=O)Me | O |
| D-8362 | H | single bond | Me | *c*-Pr | C(=O)OCH₂CH=CH₂ | Me | C(=O)Me | O |
| D-8363 | Me | single bond | H | *c*-Pr | C(=O)OCH₂CH=CH₂ | Me | C(=O)Me | O |
| D-8364 | Me(R) | single bond | H | *c*-Pr | C(=O)OCH₂CH=CH₂ | Me | C(=O)Me | O |
| D-8365 | Me(S) | single bond | H | *c*-Pr | C(=O)OCH₂CH=CH₂ | Me | C(=O)Me | O |
| D-8366 | H | CH₂ | Me | *c*-Pr | C(=O)OCH₂CH=CH₂ | Me | C(=O)Me | O |

**[Table 836]**

| Compound Number | Y | W | Z | R¹ | R² | R³ | R¹⁰ | A |
|---|---|---|---|---|---|---|---|---|
| D-8367 | Me | CH₂ | H | *c*-Pr | C(=O)OCH₂CH=CH₂ | Me | C(=O)Me | O |
| D-8368 | H | CH(Me) | H | *c*-Pr | C(=O)OCH₂CH=CH₂ | Me | C(=O)Me | O |
| D-8369 | H | CH₂CH₂ | Me | *c*-Pr | C(=O)OCH₂CH=CH₂ | Me | C(=O)Me | O |
| D-8370 | Me | CH₂CH₂ | H | *c*-Pr | C(=O)OCH₂CH=CH₂ | Me | C(=O)Me | O |
| D-8371 | H | single bond | Me | *c*-Pr | C(=O)OCH₂CCl₃ | Cl | C(=O)Me | O |
| D-8372 | Me | single bond | H | *c*-Pr | C(=O)OCH₂CCl₃ | Cl | C(=O)Me | O |
| D-8373 | Me(R) | single bond | H | *c*-Pr | C(=O)OCH₂CCl₃ | Cl | C(=O)Me | O |
| D-8374 | Me(S) | single bond | H | *c*-Pr | C(=O)OCH₂CCl₃ | Cl | C(=O)Me | O |
| D-8375 | H | CH₂ | Me | *c*-Pr | C(=O)OCH₂CCl₃ | Cl | C(=O)Me | O |
| D-8376 | Me | CH₂ | H | *c*-Pr | C(=O)OCH₂CCl₃ | Cl | C(=O)Me | O |
| D-8377 | H | CH(Me) | H | *c*-Pr | C(=O)OCH₂CCl₃ | Cl | C(=O)Me | O |
| D-8378 | H | CH₂CH₂ | Me | *c*-Pr | C(=O)OCH₂CCl₃ | Cl | C(=O)Me | O |
| D-8379 | Me | CH₂CH₂ | H | *c*-Pr | C(=O)OCH₂CCl₃ | Cl | C(=O)Me | O |
| D-8380 | H | single bond | Me | *c*-Pr | C(=O)OCH₂CCl₃ | Me | C(=O)Me | O |
| D-8381 | Me | single bond | H | *c*-Pr | C(=O)OCH₂CCl₃ | Me | C(=O)Me | O |
| D-8382 | Me(R) | single bond | H | *c*-Pr | C(=O)OCH₂CCl₃ | Me | C(=O)Me | O |
| D-8383 | Me(S) | single bond | H | *c*-Pr | C(=O)OCH₂CCl₃ | Me | C(=O)Me | O |
| D-8384 | H | CH₂ | Me | *c*-Pr | C(=O)OCH₂CCl₃ | Me | C(=O)Me | O |
| D-8385 | Me | CH₂ | H | *c*-Pr | C(=O)OCH₂CCl₃ | Me | C(=O)Me | O |
| D-8386 | H | CH(Me) | H | *c*-Pr | C(=O)OCH₂CCl₃ | Me | C(=O)Me | O |
| D-8387 | H | CH₂CH₂ | Me | *c*-Pr | C(=O)OCH₂CCl₃ | Me | C(=O)Me | O |
| D-8388 | Me | CH₂CH₂ | H | *c*-Pr | C(=O)OCH₂CCl₃ | Me | C(=O)Me | O |
| D-8389 | H | single bond | Me | *c*-Pr | C(=O)N(Me)₂ | Cl | C(=O)Me | O |
| D-8390 | Me | single bond | H | *c*-Pr | C(=O)N(Me)₂ | Cl | C(=O)Me | O |
| D-8391 | Me(R) | single bond | H | *c*-Pr | C(=O)N(Me)₂ | Cl | C(=O)Me | O |
| D-8392 | Me(S) | single bond | H | *c*-Pr | C(=O)N(Me)₂ | Cl | C(=O)Me | O |
| D-8393 | H | CH₂ | Me | *c*-Pr | C(=O)N(Me)₂ | Cl | C(=O)Me | O |
| D-8394 | Me | CH₂ | H | *c*-Pr | C(=O)N(Me)₂ | Cl | C(=O)Me | O |
| D-8395 | H | CH(Me) | H | *c*-Pr | C(=O)N(Me)₂ | Cl | C(=O)Me | O |
| D-8396 | H | CH₂CH₂ | Me | *c*-Pr | C(=O)N(Me)₂ | Cl | C(=O)Me | O |
| D-8397 | Me | CH₂CH₂ | H | *c*-Pr | C(=O)N(Me)₂ | Cl | C(=O)Me | O |
| D-8398 | H | single bond | Me | *c*-Pr | C(=O)N(Me)₂ | Me | C(=O)Me | O |
| D-8399 | Me | single bond | H | *c*-Pr | C(=O)N(Me)₂ | Me | C(=O)Me | O |
| D-8400 | Me(R) | single bond | H | *c*-Pr | C(=O)N(Me)₂ | Me | C(=O)Me | O |
| D-8401 | Me(S) | single bond | H | *c*-Pr | C(=O)N(Me)₂ | Me | C(=O)Me | O |
| D-8402 | H | CH₂ | Me | *c*-Pr | C(=O)N(Me)₂ | Me | C(=O)Me | O |
| D-8403 | Me | CH₂ | H | *c*-Pr | C(=O)N(Me)₂ | Me | C(=O)Me | O |
| D-8404 | H | CH(Me) | H | *c*-Pr | C(=O)N(Me)₂ | Me | C(=O)Me | O |
| D-8405 | H | CH₂CH₂ | Me | *c*-Pr | C(=O)N(Me)₂ | Me | C(=O)Me | O |
| D-8406 | Me | CH₂CH₂ | H | *c*-Pr | C(=O)N(Me)₂ | Me | C(=O)Me | O |
| D-8407 | H | single bond | Me | *c*-Pr | CH₂OC(=O)Me | Cl | C(=O)Me | O |
| D-8408 | Me | single bond | H | *c*-Pr | CH₂OC(=O)Me | Cl | C(=O)Me | O |
| D-8409 | Me(R) | single bond | H | *c*-Pr | CH₂OC(=O)Me | Cl | C(=O)Me | O |
| D-8410 | Me(S) | single bond | H | *c*-Pr | CH₂OC(=O)Me | Cl | C(=O)Me | O |

**[Table 837]**

| Compound Number | Y | W | Z | R¹ | R² | R³ | R¹⁰ | A |
|---|---|---|---|---|---|---|---|---|
| D-8411 | H | CH₂ | Me | *c*-Pr | CH₂OC(=O)Me | Cl | C(=O)Me | O |
| D-8412 | Me | CH₂ | H | *c*-Pr | CH₂OC(=O)Me | Cl | C(=O)Me | O |
| D-8413 | H | CH(Me) | H | *c*-Pr | CH₂OC(=O)Me | Cl | C(=O)Me | O |
| D-8414 | H | CH₂CH₂ | Me | *c*-Pr | CH₂OC(=O)Me | Cl | C(=O)Me | O |
| D-8415 | Me | CH₂CH₂ | H | *c*-Pr | CH₂OC(=O)Me | Cl | C(=O)Me | O |
| D-8416 | H | single bond | Me | *c*-Pr | CH₂OC(=O)Me | Me | C(=O)Me | O |
| D-8417 | Me | single bond | H | *c*-Pr | CH₂OC(=O)Me | Me | C(=O)Me | O |
| D-8418 | Me(R) | single bond | H | *c*-Pr | CH₂OC(=O)Me | Me | C(=O)Me | O |
| D-8419 | Me(S) | single bond | H | *c*-Pr | CH₂OC(=O)Me | Me | C(=O)Me | O |
| D-8420 | H | CH₂ | Me | *c*-Pr | CH₂OC(=O)Me | Me | C(=O)Me | O |
| D-8421 | Me | CH₂ | H | *c*-Pr | CH₂OC(=O)Me | Me | C(=O)Me | O |
| D-8422 | H | CH(Me) | H | *c*-Pr | CH₂OC(=O)Me | Me | C(=O)Me | O |
| D-8423 | H | CH₂CH₂ | Me | *c*-Pr | CH₂OC(=O)Me | Me | C(=O)Me | O |
| D-8424 | Me | CH₂CH₂ | H | *c*-Pr | CH₂OC(=O)Me | Me | C(=O)Me | O |
| D-8425 | H | single bond | Me | *c*-Pr | CH₂OC(=O)(*i*-Pr) | Cl | C(=O)Me | O |
| D-8426 | Me | single bond | H | *c*-Pr | CH₂OC(=O)(*i*-Pr) | Cl | C(=O)Me | O |
| D-8427 | Me(R) | single bond | H | *c*-Pr | CH₂OC(=O)(*i*-Pr) | Cl | C(=O)Me | O |
| D-8428 | Me(S) | single bond | H | *c*-Pr | CH₂OC(=O)(*i*-Pr) | Cl | C(=O)Me | O |
| D-8429 | H | CH₂ | Me | *c*-Pr | CH₂OC(=O)(*i*-Pr) | Cl | C(=O)Me | O |
| D-8430 | Me | CH₂ | H | *c*-Pr | CH₂OC(=O)(*i*-Pr) | Cl | C(=O)Me | O |
| D-8431 | H | CH(Me) | H | *c*-Pr | CH₂OC(=O)(*i*-Pr) | Cl | C(=O)Me | O |
| D-8432 | H | CH₂CH₂ | Me | *c*-Pr | CH₂OC(=O)(*i*-Pr) | Cl | C(=O)Me | O |
| D-8433 | Me | CH₂CH₂ | H | *c*-Pr | CH₂OC(=O)(*i*-Pr) | Cl | C(=O)Me | O |
| D-8434 | H | single bond | Me | *c*-Pr | CH₂OC(=O)(*i*-Pr) | Me | C(=O)Me | O |
| D-8435 | Me | single bond | H | *c*-Pr | CH₂OC(=O)(*i*-Pr) | Me | C(=O)Me | O |
| D-8436 | Me(R) | single bond | H | *c*-Pr | CH₂OC(=O)(*i*-Pr) | Me | C(=O)Me | O |
| D-8437 | Me(S) | single bond | H | *c*-Pr | CH₂OC(=O)(*i*-Pr) | Me | C(=O)Me | O |
| D-8438 | H | CH₂ | Me | *c*-Pr | CH₂OC(=O)(*i*-Pr) | Me | C(=O)Me | O |
| D-8439 | Me | CH₂ | H | *c*-Pr | CH₂OC(=O)(*i*-Pr) | Me | C(=O)Me | O |
| D-8440 | H | CH(Me) | H | *c*-Pr | CH₂OC(=O)(*i*-Pr) | Me | C(=O)Me | O |
| D-8441 | H | CH₂CH₂ | Me | *c*-Pr | CH₂OC(=O)(*i*-Pr) | Me | C(=O)Me | O |
| D-8442 | Me | CH₂CH₂ | H | *c*-Pr | CH₂OC(=O)(*i*-Pr) | Me | C(=O)Me | O |
| D-8443 | H | single bond | Me | *c*-Pr | CH₂OC(=O)(*t*-Bu) | Cl | C(=O)Me | O |
| D-8444 | Me | single bond | H | *c*-Pr | CH₂OC(=O)(*t*-Bu) | Cl | C(=O)Me | O |
| D-8445 | Me(R) | single bond | H | *c*-Pr | CH₂OC(=O)(*t*-Bu) | Cl | C(=O)Me | O |
| D-8446 | Me(S) | single bond | H | *c*-Pr | CH₂OC(=O)(*t*-Bu) | Cl | C(=O)Me | O |
| D-8447 | H | CH₂ | Me | *c*-Pr | CH₂OC(=O)(*t*-Bu) | Cl | C(=O)Me | O |
| D-8448 | Me | CH₂ | H | *c*-Pr | CH₂OC(=O)(*t*-Bu) | Cl | C(=O)Me | O |
| D-8449 | H | CH(Me) | H | *c*-Pr | CH₂OC(=O)(*t*-Bu) | Cl | C(=O)Me | O |
| D-8450 | H | CH₂CH₂ | Me | *c*-Pr | CH₂OC(=O)(*t*-Bu) | Cl | C(=O)Me | O |
| D-8451 | Me | CH₂CH₂ | H | *c*-Pr | CH₂OC(=O)(*t*-Bu) | Cl | C(=O)Me | O |
| D-8452 | H | single bond | Me | *c*-Pr | CH₂OC(=O)(*t*-Bu) | Me | C(=O)Me | O |
| D-8453 | Me | single bond | H | *c*-Pr | CH₂OC(=O)(*t*-Bu) | Me | C(=O)Me | O |
| D-8454 | Me(R) | single bond | H | *c*-Pr | CH₂OC(=O)(*t*-Bu) | Me | C(=O)Me | O |

**[Table 838]**

| Compound Number | Y | W | Z | R¹ | R² | R³ | R¹⁰ | A |
|---|---|---|---|---|---|---|---|---|
| D-8455 | Me(S) | single bond | H | *c*-Pr | CH₂OC(=O)(*t*-Bu) | Me | C(=O)Me | O |
| D-8456 | H | CH₂ | Me | *c*-Pr | CH₂OC(=O)(*t*-Bu) | Me | C(=O)Me | O |
| D-8457 | Me | CH₂ | H | *c*-Pr | CH₂OC(=O)(*t*-Bu) | Me | C(=O)Me | O |
| D-8458 | H | CH(Me) | H | *c*-Pr | CH₂OC(=O)(*t*-Bu) | Me | C(=O)Me | O |
| D-8459 | H | CH₂CH₂ | Me | *c*-Pr | CH₂OC(=O)(*t*-Bu) | Me | C(=O)Me | O |
| D-8460 | Me | CH₂CH₂ | H | *c*-Pr | CH₂OC(=O)(*t*-Bu) | Me | C(=O)Me | O |
| D-8461 | H | single bond | Me | *c*-Pr | CH₂OC(=O)OEt | Cl | C(=O)Me | O |
| D-8462 | Me | single bond | H | *c*-Pr | CH₂OC(=O)OEt | Cl | C(=O)Me | O |
| D-8463 | Me(R) | single bond | H | *c*-Pr | CH₂OC(=O)OEt | Cl | C(=O)Me | O |
| D-8464 | Me(S) | single bond | H | *c*-Pr | CH₂OC(=O)OEt | Cl | C(=O)Me | O |
| D-8465 | H | CH₂ | Me | *c*-Pr | CH₂OC(=O)OEt | Cl | C(=O)Me | O |
| D-8466 | Me | CH₂ | H | *c*-Pr | CH₂OC(=O)OEt | Cl | C(=O)Me | O |
| D-8467 | H | CH(Me) | H | *c*-Pr | CH₂OC(=O)OEt | Cl | C(=O)Me | O |
| D-8468 | H | CH₂CH₂ | Me | *c*-Pr | CH₂OC(=O)OEt | Cl | C(=O)Me | O |
| D-8469 | Me | CH₂CH₂ | H | *c*-Pr | CH₂OC(=O)OEt | Cl | C(=O)Me | O |
| D-8470 | H | single bond | Me | *c*-Pr | CH₂OC(=O)OEt | Me | C(=O)Me | O |
| D-8471 | Me | single bond | H | *c*-Pr | CH₂OC(=O)OEt | Me | C(=O)Me | O |
| D-8472 | Me(R) | single bond | H | *c*-Pr | CH₂OC(=O)OEt | Me | C(=O)Me | O |
| D-8473 | Me(S) | single bond | H | *c*-Pr | CH₂OC(=O)OEt | Me | C(=O)Me | O |
| D-8474 | H | CH₂ | Me | *c*-Pr | CH₂OC(=O)OEt | Me | C(=O)Me | O |
| D-8475 | Me | CH₂ | H | *c*-Pr | CH₂OC(=O)OEt | Me | C(=O)Me | O |
| D-8476 | H | CH(Me) | H | *c*-Pr | CH₂OC(=O)OEt | Me | C(=O)Me | O |
| D-8477 | H | CH₂CH₂ | Me | *c*-Pr | CH₂OC(=O)OEt | Me | C(=O)Me | O |
| D-8478 | Me | CH₂CH₂ | H | *c*-Pr | CH₂OC(=O)OEt | Me | C(=O)Me | O |
| D-8479 | H | single bond | Me | *c*-Pr | CH₂OC(=O)O(*i*-Pr) | Cl | C(=O)Me | O |
| D-8480 | Me | single bond | H | *c*-Pr | CH₂OC(=O)O(*i*-Pr) | Cl | C(=O)Me | O |
| D-8481 | Me(R) | single bond | H | *c*-Pr | CH₂OC(=O)O(*i*-Pr) | Cl | C(=O)Me | O |
| D-8482 | Me(S) | single bond | H | *c*-Pr | CH₂OC(=O)O(*i*-Pr) | Cl | C(=O)Me | O |
| D-8483 | H | CH₂ | Me | *c*-Pr | CH₂OC(=O)O(*i*-Pr) | Cl | C(=O)Me | O |
| D-8484 | Me | CH₂ | H | *c*-Pr | CH₂OC(=O)O(*i*-Pr) | Cl | C(=O)Me | O |
| D-8485 | H | CH(Me) | H | *c*-Pr | CH₂OC(=O)O(*i*-Pr) | Cl | C(=O)Me | O |
| D-8486 | H | CH₂CH₂ | Me | *c*-Pr | CH₂OC(=O)O(*i*-Pr) | Cl | C(=O)Me | O |
| D-8487 | Me | CH₂CH₂ | H | *c*-Pr | CH₂OC(=O)O(*i*-Pr) | Cl | C(=O)Me | O |
| D-8488 | H | single bond | Me | *c*-Pr | CH₂OC(=O)O(*i*-Pr) | Me | C(=O)Me | O |
| D-8489 | Me | single bond | H | *c*-Pr | CH₂OC(=O)O(*i*-Pr) | Me | C(=O)Me | O |
| D-8490 | Me(R) | single bond | H | *c*-Pr | CH₂OC(=O)O(*i*-Pr) | Me | C(=O)Me | O |
| D-8491 | Me(S) | single bond | H | *c*-Pr | CH₂OC(=O)O(*i*-Pr) | Me | C(=O)Me | O |
| D-8492 | H | CH₂ | Me | *c*-Pr | CH₂OC(=O)O(*i*-Pr) | Me | C(=O)Me | O |
| D-8493 | Me | CH₂ | H | *c*-Pr | CH₂OC(=O)O(*i*-Pr) | Me | C(=O)Me | O |
| D-8494 | H | CH(Me) | H | *c*-Pr | CH₂OC(=O)O(*i*-Pr) | Me | C(=O)Me | O |
| D-8495 | H | CH₂CH₂ | Me | *c*-Pr | CH₂OC(=O)O(*i*-Pr) | Me | C(=O)Me | O |
| D-8496 | Me | CH₂CH₂ | H | *c*-Pr | CH₂OC(=O)O(*i*-Pr) | Me | C(=O)Me | O |
| D-8497 | H | single bond | Me | *c*-Pr | CH₂N(Me)C(=O)OMe | Cl | C(=O)Me | O |
| D-8498 | Me | single bond | H | *c*-Pr | CH₂N(Me)C(=O)OMe | Cl | C(=O)Me | O |

**[Table 839]**

| Compound Number | Y | W | Z | R¹ | R² | R³ | R¹⁰ | A |
|---|---|---|---|---|---|---|---|---|
| D-8499 | Me(R) | single bond | H | *c*-Pr | CH₂N(Me)C(=O)OMe | Cl | C(=O)Me | O |
| D-8500 | Me(S) | single bond | H | *c*-Pr | CH₂N(Me)C(=O)OMe | Cl | C(=O)Me | O |
| D-8501 | H | CH₂ | Me | *c*-Pr | CH₂N(Me)C(=O)OMe | Cl | C(=O)Me | O |
| D-8502 | Me | CH₂ | H | *c*-Pr | CH₂N(Me)C(=O)OMe | Cl | C(=O)Me | O |
| D-8503 | H | CH(Me) | H | *c*-Pr | CH₂N(Me)C(=O)OMe | Cl | C(=O)Me | O |
| D-8504 | H | CH₂CH₂ | Me | *c*-Pr | CH₂N(Me)C(=O)OMe | Cl | C(=O)Me | O |
| D-8505 | Me | CH₂CH₂ | H | *c*-Pr | CH₂N(Me)C(=O)OMe | Cl | C(=O)Me | O |
| D-8506 | H | single bond | Me | *c*-Pr | CH₂N(Me)C(=O)OMe | Me | C(=O)Me | O |
| D-8507 | Me | single bond | H | *c*-Pr | CH₂N(Me)C(=O)OMe | Me | C(=O)Me | O |
| D-8508 | Me(R) | single bond | H | *c*-Pr | CH₂N(Me)C(=O)OMe | Me | C(=O)Me | O |
| D-8509 | Me(S) | single bond | H | *c*-Pr | CH₂N(Me)C(=O)OMe | Me | C(=O)Me | O |
| D-8510 | H | CH₂ | Me | *c*-Pr | CH₂N(Me)C(=O)OMe | Me | C(=O)Me | O |
| D-8511 | Me | CH₂ | H | *c*-Pr | CH₂N(Me)C(=O)OMe | Me | C(=O)Me | O |
| D-8512 | H | CH(Me) | H | *c*-Pr | CH₂N(Me)C(=O)OMe | Me | C(=O)Me | O |
| D-8513 | H | CH₂CH₂ | Me | *c*-Pr | CH₂N(Me)C(=O)OMe | Me | C(=O)Me | O |
| D-8514 | Me | CH₂CH₂ | H | *c*-Pr | CH₂N(Me)C(=O)OMe | Me | C(=O)Me | O |
| D-8515 | H | single bond | Me | *c*-Pr(1-CN) | C(=O)OCH₂CH=CH₂ | Cl | H | O |
| D-8516 | Me | single bond | H | *c*-Pr(1-CN) | C(=O)OCH₂CH=CH₂ | Cl | H | O |
| D-8517 | Me(R) | single bond | H | *c*-Pr(1-CN) | C(=O)OCH₂CH=CH₂ | Cl | H | O |
| D-8518 | Me(S) | single bond | H | *c*-Pr(1-CN) | C(=O)OCH₂CH=CH₂ | Cl | H | O |
| D-8519 | H | CH₂ | Me | *c*-Pr(1-CN) | C(=O)OCH₂CH=CH₂ | Cl | H | O |
| D-8520 | Me | CH₂ | H | *c*-Pr(1-CN) | C(=O)OCH₂CH=CH₂ | Cl | H | O |
| D-8521 | H | CH(Me) | H | *c*-Pr(1-CN) | C(=O)OCH₂CH=CH₂ | Cl | H | O |
| D-8522 | H | CH₂CH₂ | Me | *c*-Pr(1-CN) | C(=O)OCH₂CH=CH₂ | Cl | H | O |
| D-8523 | Me | CH₂CH₂ | H | *c*-Pr(1-CN) | C(=O)OCH₂CH=CH₂ | Cl | H | O |
| D-8524 | H | single bond | Me | *c*-Pr(1-CN) | C(=O)OCH₂CH=CH₂ | Me | H | O |
| D-8525 | Me | single bond | H | *c*-Pr(1-CN) | C(=O)OCH₂CH=CH₂ | Me | H | O |
| D-8526 | Me(R) | single bond | H | *c*-Pr(1-CN) | C(=O)OCH₂CH=CH₂ | Me | H | O |
| D-8527 | Me(S) | single bond | H | *c*-Pr(1-CN) | C(=O)OCH₂CH=CH₂ | Me | H | O |
| D-8528 | H | CH₂ | Me | *c*-Pr(1-CN) | C(=O)OCH₂CH=CH₂ | Me | H | O |
| D-8529 | Me | CH₂ | H | *c*-Pr(1-CN) | C(=O)OCH₂CH=CH₂ | Me | H | O |
| D-8530 | H | CH(Me) | H | *c*-Pr(1-CN) | C(=O)OCH₂CH=CH₂ | Me | H | O |
| D-8531 | H | CH₂CH₂ | Me | *c*-Pr(1-CN) | C(=O)OCH₂CH=CH₂ | Me | H | O |
| D-8532 | Me | CH₂CH₂ | H | *c*-Pr(1-CN) | C(=O)OCH₂CH=CH₂ | Me | H | O |
| D-8533 | H | single bond | Me | *c*-Pr(1-CN) | C(=O)OCH₂CCl₃ | Cl | H | O |
| D-8534 | Me | single bond | H | *c*-Pr(1-CN) | C(=O)OCH₂CCl₃ | Cl | H | O |
| D-8535 | Me(R) | single bond | H | *c*-Pr(1-CN) | C(=O)OCH₂CCl₃ | Cl | H | O |
| D-8536 | Me(S) | single bond | H | *c*-Pr(1-CN) | C(=O)OCH₂CCl₃ | Cl | H | O |
| D-8537 | H | CH₂ | Me | *c*-Pr(1-CN) | C(=O)OCH₂CCl₃ | Cl | H | O |
| D-8538 | Me | CH₂ | H | *c*-Pr(1-CN) | C(=O)OCH₂CCl₃ | Cl | H | O |
| D-8539 | H | CH(Me) | H | *c*-Pr(1-CN) | C(=O)OCH₂CCl₃ | Cl | H | O |
| D-8540 | H | CH₂CH₂ | Me | *c*-Pr(1-CN) | C(=O)OCH₂CCl₃ | Cl | H | O |
| D-8541 | Me | CH₂CH₂ | H | *c*-Pr(1-CN) | C(=O)OCH₂CCl₃ | Cl | H | O |
| D-8542 | H | single bond | Me | *c*-Pr(1-CN) | C(=O)OCH₂CCl₃ | Me | H | O |

**[Table 840]**

| Compound Number | Y | W | Z | R¹ | R² | R³ | R¹⁰ | A |
|---|---|---|---|---|---|---|---|---|
| D-8543 | Me | single bond | H | *c*-Pr(1-CN) | C(=O)OCH₂CCl₃ | Me | H | O |
| D-8544 | Me(R) | single bond | H | *c*-Pr(1-CN) | C(=O)OCH₂CCl₃ | Me | H | O |
| D-8545 | Me(S) | single bond | H | *c*-Pr(1-CN) | C(=O)OCH₂CCl₃ | Me | H | O |
| D-8546 | H | CH₂ | Me | *c*-Pr(1-CN) | C(=O)OCH₂CCl₃ | Me | H | O |
| D-8547 | Me | CH₂ | H | *c*-Pr(1-CN) | C(=O)OCH₂CCl₃ | Me | H | O |
| D-8548 | H | CH(Me) | H | *c*-Pr(1-CN) | C(=O)OCH₂CCl₃ | Me | H | O |
| D-8549 | H | CH₂CH₂ | Me | *c*-Pr(1-CN) | C(=O)OCH₂CCl₃ | Me | H | O |
| D-8550 | Me | CH₂CH₂ | H | *c*-Pr(1-CN) | C(=O)OCH₂CCl₃ | Me | H | O |
| D-8551 | H | single bond | Me | *c*-Pr(1-CN) | C(=O)N(Me)₂ | Cl | H | O |
| D-8552 | Me | single bond | H | *c*-Pr(1-CN) | C(=O)N(Me)₂ | Cl | H | O |
| D-8553 | Me(R) | single bond | H | *c*-Pr(1-CN) | C(=O)N(Me)₂ | Cl | H | O |
| D-8554 | Me(S) | single bond | H | *c*-Pr(1-CN) | C(=O)N(Me)₂ | Cl | H | O |
| D-8555 | H | CH₂ | Me | *c*-Pr(1-CN) | C(=O)N(Me)₂ | Cl | H | O |
| D-8556 | Me | CH₂ | H | *c*-Pr(1-CN) | C(=O)N(Me)₂ | Cl | H | O |
| D-8557 | H | CH(Me) | H | *c*-Pr(1-CN) | C(=O)N(Me)₂ | Cl | H | O |
| D-8558 | H | CH₂CH₂ | Me | *c*-Pr(1-CN) | C(=O)N(Me)₂ | Cl | H | O |
| D-8559 | Me | CH₂CH₂ | H | *c*-Pr(1-CN) | C(=O)N(Me)₂ | Cl | H | O |
| D-8560 | H | single bond | Me | *c*-Pr(1-CN) | C(=O)N(Me)₂ | Me | H | O |
| D-8561 | Me | single bond | H | *c*-Pr(1-CN) | C(=O)N(Me)₂ | Me | H | O |
| D-8562 | Me(R) | single bond | H | *c*-Pr(1-CN) | C(=O)N(Me)₂ | Me | H | O |
| D-8563 | Me(S) | single bond | H | *c*-Pr(1-CN) | C(=O)N(Me)₂ | Me | H | O |
| D-8564 | H | CH₂ | Me | *c*-Pr(1-CN) | C(=O)N(Me)₂ | Me | H | O |
| D-8565 | Me | CH₂ | H | *c*-Pr(1-CN) | C(=O)N(Me)₂ | Me | H | O |
| D-8566 | H | CH(Me) | H | *c*-Pr(1-CN) | C(=O)N(Me)₂ | Me | H | O |
| D-8567 | H | CH₂CH₂ | Me | *c*-Pr(1-CN) | C(=O)N(Me)₂ | Me | H | O |
| D-8568 | Me | CH₂CH₂ | H | *c*-Pr(1-CN) | C(=O)N(Me)₂ | Me | H | O |
| D-8569 | H | single bond | Me | *c*-Pr(1-CN) | CH₂OC(=O)Me | Cl | H | O |
| D-8570 | Me | single bond | H | *c*-Pr(1-CN) | CH₂OC(=O)Me | Cl | H | O |
| D-8571 | Me(R) | single bond | H | *c*-Pr(1-CN) | CH₂OC(=O)Me | Cl | H | O |
| D-8572 | Me(S) | single bond | H | *c*-Pr(1-CN) | CH₂OC(=O)Me | Cl | H | O |
| D-8573 | H | CH₂ | Me | *c*-Pr(1-CN) | CH₂OC(=O)Me | Cl | H | O |
| D-8574 | Me | CH₂ | H | *c*-Pr(1-CN) | CH₂OC(=O)Me | Cl | H | O |
| D-8575 | H | CH(Me) | H | *c*-Pr(1-CN) | CH₂OC(=O)Me | Cl | H | O |
| D-8576 | H | CH₂CH₂ | Me | *c*-Pr(1-CN) | CH₂OC(=O)Me | Cl | H | O |
| D-8577 | Me | CH₂CH₂ | H | *c*-Pr(1-CN) | CH₂OC(=O)Me | Cl | H | O |
| D-8578 | H | single bond | Me | *c*-pr(1-CN) | CH₂OC(=O)Me | Me | H | O |
| D-8579 | Me | single bond | H | *c*-Pr(1-CN) | CH₂OC(=O)Me | Me | H | O |
| D-8580 | Me(R) | single bond | H | *c*-Pr(1-CN) | CH₂OC(=O)Me | Me | H | O |
| D-8581 | Me(S) | single bond | H | *c*-Pr(1-CN) | CH₂OC(=O)Me | Me | H | O |
| D-8582 | H | CH₂ | Me | *c*-Pr(1-CN) | CH₂OC(=O)Me | Me | H | O |
| D-8583 | Me | CH₂ | H | *c*-Pr(1-CN) | CH₂OC(=O)Me | Me | H | O |
| D-8584 | H | CH(Me) | H | *c*-Pr(1-CN) | CH₂OC(=O)Me | Me | H | O |
| D-8585 | H | CH₂CH₂ | Me | *c*-Pr(1-CN) | CH₂OC(=O)Me | Me | H | O |
| D-8586 | Me | CH₂CH₂ | H | *c*-Pr(1-CN) | CH₂OC(=O)Me | Me | H | O |

**[Table 841]**

| Compound Number | Y | W | Z | R¹ | R² | R³ | R¹⁰ | A |
|---|---|---|---|---|---|---|---|---|
| D-8587 | H | single bond | Me | *c*-Pr(1-CN) | CH₂OC(=O)(*i*-Pr) | Cl | H | O |
| D-8588 | Me | single bond | H | *c*-Pr(1-CN) | CH₂OC(=O)(*i*-Pr) | Cl | H | O |
| D-8589 | Me(R) | single bond | H | *c*-Pr(1-CN) | CH₂OC(=O)(*i*-Pr) | Cl | H | O |
| D-8590 | Me(S) | single bond | H | *c*-Pr(1-CN) | CH₂OC(=O)(*i*-Pr) | Cl | H | O |
| D-8591 | H | CH₂ | Me | *c*-Pr(1-CN) | CH₂OC(=O)(*i*-Pr) | Cl | H | O |
| D-8592 | Me | CH₂ | H | *c*-Pr(1-CN) | CH₂OC(=O)(*i*-Pr) | Cl | H | O |
| D-8593 | H | CH(Me) | H | *c*-Pr(1-CN) | CH₂OC(=O)(*i*-Pr) | Cl | H | O |
| D-8594 | H | CH₂CH₂ | Me | *c*-Pr(1-CN) | CH₂OC(=O)(*i*-Pr) | Cl | H | O |
| D-8595 | Me | CH₂CH₂ | H | *c*-Pr(1-CN) | CH₂OC(=O)(*i*-Pr) | Cl | H | O |
| D-8596 | H | single bond | Me | *c*-Pr(1-CN) | CH₂OC(=O)(*i*-Pr) | Me | H | O |
| D-8597 | Me | single bond | H | *c*-Pr(1-CN) | CH₂OC(=O)(*i*-Pr) | Me | H | O |
| D-8598 | Me(R) | single bond | H | *c*-Pr(1-CN) | CH₂OC(=O)(*i*-Pr) | Me | H | O |
| D-8599 | Me(S) | single bond | H | *c*-Pr(1-CN) | CH₂OC(=O)(*i*-Pr) | Me | H | O |
| D-8600 | H | CH₂ | Me | *c*-Pr(1-CN) | CH₂OC(=O)(*i*-Pr) | Me | H | O |
| D-8601 | Me | CH₂ | H | *c*-Pr(1-CN) | CH₂OC(=O)(*i*-Pr) | Me | H | O |
| D-8602 | H | CH(Me) | H | *c*-Pr(1-CN) | CH₂OC(=O)(*i*-Pr) | Me | H | O |
| D-8603 | H | CH₂CH₂ | Me | *c*-Pr(1-CN) | CH₂OC(=O)(*i*-Pr) | Me | H | O |
| D-8604 | Me | CH₂CH₂ | H | *c*-Pr(1-CN) | CH₂OC(=O)(*i*-Pr) | Me | H | O |
| D-8605 | H | single bond | Me | *c*-Pr(1-CN) | CH₂OC(=O)(*t*-Bu) | Cl | H | O |
| D-8606 | Me | single bond | H | *c*-Pr(1-CN) | CH₂OC(=O)(*t*-Bu) | Cl | H | O |
| D-8607 | Me(R) | single bond | H | *c*-Pr(1-CN) | CH₂OC(=O)(*t*-Bu) | Cl | H | O |
| D-8608 | Me(S) | single bond | H | *c*-Pr(1-CN) | CH₂OC(=O)(*t*-Bu) | Cl | H | O |
| D-8609 | H | CH₂ | Me | *c*-Pr(1-CN) | CHOC(=O)(*t*-Bu) | Cl | H | O |
| D-8610 | Me | CH₂ | H | *c*-Pr(1-CN) | CH₂OC(=O)(*t*-Bu) | Cl | H | O |
| D-8611 | H | CH(Me) | H | *c*-Pr(1-CN) | CH₂OC(=O)(*t*-Bu) | Cl | H | O |
| D-8612 | H | CH₂CH₂ | Me | *c*-Pr(1-CN) | CHOC(=O)(*t*-Bu) | Cl | H | O |
| D-8613 | Me | CH₂CH₂ | H | *c*-Pr(1-CN) | CH₂OC(=O)(*t*-Bu) | Cl | H | O |
| D-8614 | H | single bond | Me | *c*-Pr(1-CN) | CH₂OC(=O)(*t*-Bu) | Me | H | O |
| D-8615 | Me | single bond | H | *c*-Pr(1-CN) | CHOC(=O)(*t*-Bu) | Me | H | O |
| D-8616 | Me(R) | single bond | H | *c*-Pr(1-CN) | CHOC(=O)(*t*-Bu) | Me | H | O |
| D-8617 | Me(S) | single bond | H | *c*-Pr(1-CN) | CH₂OC(=O)(*t*-Bu) | Me | H | O |
| D-8618 | H | CH₂ | Me | *c*-Pr(1-CN) | CHOC(=O)(*t*-Bu) | Me | H | O |
| D-8619 | Me | CH₂ | H | *c*-Pr(1-CN) | CHOC(=O)(*t*-Bu) | Me | H | O |
| D-8620 | H | CH(Me) | H | *c*-Pr(1-CN) | CH₂OC(=O)(*t*-Bu) | Me | H | O |
| D-8621 | H | CH₂CH₂ | Me | *c*-Pr(1-CN) | CH₂OC(=O)(*t*-Bu) | Me | H | O |
| D-8622 | Me | CH₂CH₂ | H | *c*-Pr(1-CN) | CHOC(=O)(*t*-Bu) | Me | H | O |
| D-8623 | H | single bond | Me | *c*-Pr(1-CN) | CH₂OC(=O)OEt | Cl | H | O |
| D-8624 | Me | single bond | H | *c*-Pr(1-CN) | CH₂OC(=O)OEt | Cl | H | O |
| D-8625 | Me(R) | single bond | H | *c*-Pr(1-CN) | CH₂OC(=O)OEt | Cl | H | O |
| D-8626 | Me(S) | single bond | H | *c*-Pr(1-CN) | CH₂OC(=O)OEt | Cl | H | O |
| D-8627 | H | CH₂ | Me | *c*-Pr(1-CN) | CH₂OC(=O)OEt | Cl | H | O |
| D-8628 | Me | CH₂ | H | *c*-Pr(1-CN) | CH₂OC(=O)OEt | Cl | H | O |
| D-8629 | H | CH(Me) | H | *c*-Pr(1-CN) | CH₂OC(=O)OEt | Cl | H | O |
| D-8630 | H | CH₂CH₂ | Me | *c*-Pr(1-CN) | CH₂OC(=O)OEt | Cl | H | O |

**[Table 842]**

| Compound Number | Y | W | Z | R¹ | R² | R³ | R¹⁰ | A |
|---|---|---|---|---|---|---|---|---|
| D-8631 | Me | CH₂CH₂ | H | *c*-Pr(1-CN) | CH₂OC(=O)OEt | Cl | H | O |
| D-8632 | H | single bond | Me | *c*-Pr(1-CN) | CH₂OC(=O)OEt | Me | H | O |
| D-8633 | Me | single bond | H | *c*-Pr(1-CN) | CH₂OC(=O)OEt | Me | H | O |
| D-8634 | Me(R) | single bond | H | *c*-Pr(1-CN) | CH₂OC(=O)OEt | Me | H | O |
| D-8635 | Me(S) | single bond | H | *c*-Pr(1-CN) | CH₂OC(=O)OEt | Me | H | O |
| D-8636 | H | CH₂ | Me | *c*-Pr(1-CN) | CH₂OC(=O)OEt | Me | H | O |
| D-8637 | Me | CH₂ | H | *c*-Pr(1-CN) | CH₂OC(=O)OEt | Me | H | O |
| D-8638 | H | CH(Me) | H | *c*-Pr(1-CN) | CH₂OC(=O)OEt | Me | H | O |
| D-8639 | H | CH₂CH₂ | Me | *c*-Pr(1-CN) | CH₂OC(=O)OEt | Me | H | O |
| D-8640 | Me | CH₂CH₂ | H | *c*-Pr(1-CN) | CH₂OC(=O)OEt | Me | H | O |
| D-8641 | H | single bond | Me | *c*-Pr(1-CN) | CH₂OC(=O)O(*i*-Pr) | Cl | H | O |
| D-8642 | Me | single bond | H | *c*-Pr(1-CN) | CH₂OC(=O)O(*i*-Pr) | Cl | H | O |
| D-8643 | Me(R) | single bond | H | *c*-Pr(1-CN) | CH₂OC(=O)O(*i*-Pr) | Cl | H | O |
| D-8644 | Me(S) | single bond | H | *c*-Pr(1-CN) | CH₂OC(=O)O(*i*-Pr) | Cl | H | O |
| D-8645 | H | CH₂ | Me | *c*-Pr(1-CN) | CH₂OC(=O)O(*i*-Pr) | Cl | H | O |
| D-8646 | Me | CH₂ | H | *c*-Pr(1-CN) | CH₂OC(=O)O(*i*-Pr) | Cl | H | O |
| D-8647 | H | CH(Me) | H | *c*-Pr(1-CN) | CH₂OC(=O)O(*i*-Pr) | Cl | H | O |
| D-8648 | H | CH₂CH₂ | Me | *c*-Pr(1-CN) | CH₂OC(=O)O(*i*-Pr) | Cl | H | O |
| D-8649 | Me | CH₂CH₂ | H | *c*-Pr(1-CN) | CH₂OC(=O)O(*i*-Pr) | Cl | H | O |
| D-8650 | H | single bond | Me | *c*-Pr(1-CN) | CH₂OC(=O)O(*i*-Pr) | Me | H | O |
| D-8651 | Me | single bond | H | *c*-Pr(1-CN) | CH₂OC(=O)O(*i*-Pr) | Me | H | O |
| D-8652 | Me(R) | single bond | H | *c*-Pr(1-CN) | CH₂OC(=O)O(*i*-Pr) | Me | H | O |
| D-8653 | Me(S) | single bond | H | *c*-Pr(1-CN) | CH₂OC(=O)O(*i*-Pr) | Me | H | O |
| D-8654 | H | CH₂ | Me | *c*-Pr(1-CN) | CH₂OC(=O)O(*i*-Pr) | Me | H | O |
| D-8655 | Me | CH₂ | H | *c*-Pr(1-CN) | CH₂OC(=O)O(*i*-Pr) | Me | H | O |
| D-8656 | H | CH(Me) | H | *c*-Pr(1-CN) | CH₂OC(=O)O(*i*-Pr) | Me | H | O |
| D-8657 | H | CH₂CH₂ | Me | *c*-Pr(1-CN) | CH₂OC(=O)O(*i*-Pr) | Me | H | O |
| D-8658 | Me | CH₂CH₂ | H | *c*-Pr(1-CN) | CH₂OC(=O)O(*i*-Pr) | Me | H | O |
| D-8659 | H | single bond | Me | *c*-Pr(1-CN) | CH₂N(Me)C(=O)OMe | Cl | H | O |
| D-8660 | Me | single bond | H | *c*-Pr(1-CN) | CH₂N(Me)C(=O)OMe | Cl | H | O |
| D-8661 | Me(R) | single bond | H | *c*-Pr(1-CN) | CH₂N(Me)C(=O)OMe | Cl | H | O |
| D-8662 | Me(S) | single bond | H | *c*-Pr(1-CN) | CH₂N(Me)C(=O)OMe | Cl | H | O |
| D-8663 | H | CH₂ | Me | *c*-Pr(1-CN) | CH₂N(Me)C(=O)OMe | Cl | H | O |
| D-8664 | Me | CH₂ | H | *c*-Pr(1-CN) | CH₂N(Me)C(=O)OMe | Cl | H | O |
| D-8665 | H | CH(Me) | H | *c*-Pr(1-CN) | CH₂N(Me)C(=O)OMe | Cl | H | O |
| D-8666 | H | CH₂CH₂ | Me | *c*-Pr(1-CN) | CH₂N(Me)C(=O)OMe | Cl | H | O |
| D-8667 | Me | CH₂CH₂ | H | *c*-Pr(1-CN) | CH₂N(Me)C(=O)OMe | Cl | H | O |
| D-8668 | H | single bond | Me | *c*-Pr(1-CN) | CH₂N(Me)C(=O)OMe | Me | H | O |
| D-8669 | Me | single bond | H | *c*-Pr(1-CN) | CH₂N(Me)C(=O)OMe | Me | H | O |
| D-8670 | Me(R) | single bond | H | *c*-Pr(1-CN) | CH₂N(Me)C(=O)OMe | Me | H | O |
| D-8671 | Me(S) | single bond | H | *c*-Pr(1-CN) | CH₂N(Me)C(=O)OMe | Me | H | O |
| D-8672 | H | CH₂ | Me | *c*-Pr(1-CN) | CH₂N(Me)C(=O)OMe | Me | H | O |
| D-8673 | Me | CH₂ | H | *c*-Pr(1-CN) | CH₂N(Me)C(=O)OMe | Me | H | O |
| D-8674 | H | CH(Me) | H | *c*-Pr(1-CN) | CH₂N(Me)C(=O)OMe | Me | H | O |

**[Table 843]**

| Compound Number | Y | W | z | R¹ | R² | R³ | R¹⁰ | A |
|---|---|---|---|---|---|---|---|---|
| D-8675 | H | CH₂CH₂ | Me | *c*-Pr(1-CN) | CH₂N(Me)C(=O)OMe | Me | H | O |
| D-8676 | Me | CH₂CH₂ | H | *c*-Pr(1-CN) | CH₂N(Me)C(=O)OMe | Me | H | O |
| D-8677 | H | single bond | Me | *c*-Pr(1-CN) | C(=O)OCH₂CH=CH₂ | Cl | Me | O |
| D-8678 | Me | single bond | H | *c*-Pr(1-CN) | C(=O)OCH₂CH=CH₂ | Cl | Me | O |
| D-8679 | Me(R) | single bond | H | *c*-Pr(1-CN) | C(=O)OCH₂CH=CH₂ | Cl | Me | O |
| D-8680 | Me(S) | single bond | H | *c*-Pr(1-CN) | C(=O)OCH₂CH=CH₂ | Cl | Me | O |
| D-8681 | H | CH₂ | Me | *c* -Pr(1-CN) | C(=O)OCH₂CH=CH₂ | Cl | Me | O |
| D-8682 | Me | CH₂ | H | *c* -Pr(1-CN) | C(=O)OCH₂CH=CH₂ | Cl | Me | O |
| D-8683 | H | CH(Me) | H | *c* -Pr(1-CN) | C(=O)OCH₂CH=CH₂ | Cl | Me | O |
| D-8684 | H | CH₂CH₂ | Me | *c* -Pr(1-CN) | C(=O)OCH₂CH=CH₂ | Cl | Me | O |
| D-8685 | Me | CH₂CH₂ | H | *c* -Pr(1-CN) | C(=O)OCH₂CH=CH₂ | Cl | Me | O |
| D-8686 | H | single bond | Me | *c* -Pr(1-CN) | C(=O)OCH₂CH=CH₂ | Me | Me | O |
| D-8687 | Me | single bond | H | *c*-Pr(1-CN) | C(=O)OCH₂CH=CH₂ | Me | Me | O |
| D-8688 | Me(R) | single bond | H | *c*-Pr(1-CN) | C(=O)OCH₂CH=CH₂ | Me | Me | O |
| D-8689 | Me(S) | single bond | H | *c*-Pr(1-CN) | C(=O)OCH₂CH=CH₂ | Me | Me | O |
| 0-8690 | H | CH₂ | Me | *c*-Pr(1-CN) | C(=O)OCH₂CH=CH₂ | Me | Me | O |
| D-8691 | Me | CH₂ | H | *c*-Pr(1-CN) | C(=O)OCH₂CH=CH₂ | Me | Me | O |
| D-8692 | H | CH(Me) | H | *c*-Pr(1-CN) | C(=O)OCH₂CH=CH₂ | Me | Me | O |
| D-8693 | H | CH₂CH₂ | Me | *c*-Pr(1-CN) | C(=O)OCH₂CH=CH₂ | Me | Me | O |
| D-8694 | Me | CH₂CH₂ | H | *c*-Pr(1-CN) | C(=O)OCH₂CH=CH₂ | Me | Me | O |
| D-8695 | H | single bond | Me | *c*-Pr(1-CN) | C(=O)OCH₂CCl₃ | Cl | Me | O |
| D-8696 | Me | single bond | H | *c*-Pr(1-CN) | C(=O)OCH₂CCl₃ | Cl | Me | O |
| D-8697 | Me(R) | single bond | H | *c*-Pr(1-CN) | C(=O)OCH₂CCl₃ | Cl | Me | O |
| D-8698 | Me(S) | single bond | H | *c*-Pr(1-CN) | C(=O)OCH₂CCl₃ | Cl | Me | O |
| D-8699 | H | CH₂ | Me | *c*-Pr(1-CN) | C(=O)OCH₂CCl₃ | Cl | Me | O |
| D-8700 | Me | CH₂ | H | *c*-Pr(1-CN) | C(=O)OCH₂CCl₃ | Cl | Me | O |
| D-8701 | H | CH(Me) | H | *c*-Pr(1-CN) | C(=O)OCH₂CCl₃ | Cl | Me | O |
| D-8702 | H | CH₂CH₂ | Me | *c*-Pr(1-CN) | C(=O)OCH₂CCl₃ | Cl | Me | O |
| D-8703 | Me | CH₂CH₂ | H | *c*-Pr(1-CN) | C(=O)OCH₂CCl₃ | Cl | Me | O |
| D-8704 | H | single bond | Me | *c*-Pr(1-CN) | C(=O)OCH₂CCl₃ | Me | Me | O |
| D-8705 | Me | single bond | H | *c*-Pr(1-CN) | C(=O)OCH₂CCl₃ | Me | Me | O |
| D-8706 | Me(R) | single bond | H | *c*-Pr(1-CN) | C(=O)OCH₂CCl₃ | Me | Me | O |
| D-8707 | Me(S) | single bond | H | *c*-Pr(1-CN) | C(=O)OCH₂CCl₃ | Me | Me | O |
| D-8708 | H | CH₂ | Me | *c*-Pr(1-CN) | C(=O)OCH₂CCl₃ | Me | Me | O |
| D-8709 | Me | CH₂ | H | *c*-Pr(1-CN) | C(=O)OCH₂CCl₃ | Me | Me | O |
| D-8710 | H | CH(Me) | H | *c*-Pr(1-CN) | C(=O)OCH₂CCl₃ | Me | Me | O |
| D-8711 | H | CH₂CH₂ | Me | *c*-Pr(1-CN) | C(=O)OCH₂CCl₃ | Me | Me | O |
| D-8712 | Me | CH₂CH₂ | H | *c*-Pr(1-CN) | C(=O)OCH₂CCl₃ | Me | Me | O |
| D-8713 | H | single bond | Me | *c*-Pr(1-CN) | C(=O)N(Me)₂ | Cl | Me | O |
| D-8714 | Me | single bond | H | *c*-Pr(1-CN) | C(=O)N(Me)₂ | Cl | Me | O |
| D-8715 | Me(R) | single bond | H | *c*-Pr(1-CN) | C(=O)N(Me)₂ | Cl | Me | O |
| D-8716 | Me(S) | single bond | H | *c*-Pr(1-CN) | C(=O)N(Me)₂ | Cl | Me | O |
| D-8717 | H | CH₂ | Me | *c*-Pr(1-CN) | C(=O)N(Me)₂ | Cl | Me | O |
| D-8718 | Me | CH₂ | H | *c*-Pr(1-CN) | C(=O)N(Me)₂ | Cl | Me | O |

**[Table 844]**

| Compound Number | Y | W | Z | R¹ | R² | R³ | R¹⁰ | A |
|---|---|---|---|---|---|---|---|---|
| D-8719 | H | CH(Me) | H | *c* -Pr(1-CN) | C(=O)N(Me)₂ | Cl | Me | O |
| D-8720 | H | CH₂CH₂ | Me | *c* -Pr(1-CN) | C(=O)N(Me)₂ | Cl | Me | O |
| D-8721 | Me | CH₂CH₂ | H | *c* -Pr(1-CN) | C(=O)N(Me)₂ | Cl | Me | O |
| D-8722 | H | single bond | Me | *c* -Pr(1-CN) | C(=O)N(Me)₂ | Me | Me | O |
| D-8723 | Me | single bond | H | *c* -Pr(1-CN) | C(=O)N(Me)₂ | Me | Me | O |
| D-8724 | Me(R) | single bond | H | *c*-Pr(1-CN) | C(=O)N(Me)₂ | Me | Me | O |
| D-8725 | Me(S) | single bond | H | *c* -Pr(1-CN) | C(=O)N(Me)₂ | Me | Me | O |
| D-8726 | H | CH₂ | Me | *c* -Pr(1-CN) | C(=O)N(Me)₂ | Me | Me | O |
| D-8727 | Me | CH₂ | H | *c* -Pr(1-CN) | C(=O)N(Me)₂ | Me | Me | O |
| D-8728 | H | CH(Me) | H | *c* -Pr(1-CN) | C(=O)N(Me)₂ | Me | Me | O |
| D-8729 | H | CH₂CH₂ | Me | *c* -Pr(1-CN) | C(=O)N(Me)₂ | Me | Me | O |
| D-8730 | Me | CH₂CH₂ | H | *c* -Pr(1-CN) | C(=O)N(Me)₂ | Me | Me | O |
| D-8731 | H | single bond | Me | *c* -Pr(1-CN) | CH₂OC(=O)Me | Cl | Me | O |
| D-8732 | Me | single bond | H | *c* -Pr(1-CN) | CH₂OC(=O)Me | Cl | Me | O |
| D-8733 | Me(R) | single bond | H | *c* -Pr(1-CN) | CH₂OC(=O)Me | Cl | Me | O |
| D-8734 | Me(S) | single bond | H | *c* -Pr(1-CN) | CH₂OC(=O)Me | Cl | Me | O |
| D-8735 | H | CH₂ | Me | *c* -Pr(1-CN) | CH₂OC(=O)Me | Cl | Me | O |
| D-8736 | Me | CH₂ | H | *c* -Pr(1-CN) | CH₂OC(=O)Me | Cl | Me | O |
| D-8737 | H | CH(Me) | H | *c* -Pr(1-CN) | CH₂OC(=O)Me | Cl | Me | O |
| D-8738 | H | CH₂CH₂ | Me | *c* -Pr(1-CN) | CH₂OC(=O)Me | Cl | Me | O |
| D-8739 | Me | CH₂CH₂ | H | *c* -Pr(1-CN) | CH₂OC(=O)Me | Cl | Me | O |
| D-8740 | H | single bond | Me | *c*-Pr(1-CN) | CH₂OC(=O)Me | Me | Me | O |
| D-8741 | Me | single bond | H | *c* -Pr(1-CN) | CH₂OC(=O)Me | Me | Me | O |
| D-8742 | Me(R) | single bond | H | *c* -Pr(1-CN) | CH₂OC(=O)Me | Me | Me | O |
| D-8743 | Me(S) | single bond | H | *c* -Pr(1-CN) | CH₂OC(=O)Me | Me | Me | O |
| D-8744 | H | CH₂ | Me | *c* -Pr(1-CN) | CH₂OC(=O)Me | Me | Me | O |
| D-8745 | Me | CH₂ | H | *c* -Pr(1-CN) | CH₂OC(=O)Me | Me | Me | O |
| D-8746 | H | CH(Me) | H | *c* -Pr(1-CN) | CH₂OC(=O)Me | Me | Me | O |
| D-8747 | H | CH₂CH₂ | Me | *c* -Pr(1-CN) | CH₂OC(=O)Me | Me | Me | O |
| D-8748 | Me | CH₂CH₂ | H | *c* -Pr(1-CN) | CH₂OC(=O)Me | Me | Me | O |
| D-8749 | H | single bond | Me | *c* -Pr(1-CN) | CH₂OC(=O)(*i*-Pr) | Cl | Me | O |
| D-8750 | Me | single bond | H | *c* -Pr(1-CN) | CH₂OC (=O)(i -Pr) | Cl | Me | O |
| D-8751 | Me(R) | single bond | H | *c* -Pr(1-CN) | CH₂OC (=O)(i -Pr) | Cl | Me | O |
| D-8752 | Me(S) | single bond | H | *c* -Pr(1-CN) | CH₂OC(=O)(*i*-Pr) | Cl | Me | O |
| D-8753 | H | CH₂ | Me | *c* -Pr(1-CN) | CH₂OC(=O)(*i*-Pr) | Cl | Me | O |
| D-8754 | Me | CH₂ | H | *c* -Pr(1-CN) | CH₂OC (=O)(i -Pr) | Cl | Me | O |
| D-8755 | H | CH(Me) | H | *c* -Pr(1-CN) | CH₂OC (=O)(i -Pr) | Cl | Me | O |
| D-8756 | H | CH₂CH₂ | Me | *c* -Pr(1-CN) | CH₂OC(=O)(*i*-Pr) | Cl | Me | O |
| D-8757 | Me | CH₂CH₂ | H | *c* -Pr(1-CN) | CH₂OC (=O)(i -Pr) | Cl | Me | O |
| D-8758 | H | single bond | Me | *c* -Pr(1-CN) | CH₂OC (=O)(i -Pr) | Me | Me | O |
| D-8759 | Me | single bond | H | *c* -Pr(1-CN) | CH₂OC(=O)(*i*-Pr) | Me | Me | O |
| D-8760 | Me(R) | single bond | H | *c* -Pr(1-CN) | CH₂OC(=O)(*i*-Pr) | Me | Me | O |
| D-8761 | Me(S) | single bond | H | *c* -Pr(1-CN) | CH₂OC (=O)(i -Pr) | Me | Me | O |
| D-8762 | H | CH₂ | Me | *c* -Pr(1-CN) | CH₂OC(=O)(*i*-Pr) | Me | Me | O |

**[Table 845]**

| Compound Number | Y | W | Z | R¹ | R² | R³ | R¹⁰ | A |
|---|---|---|---|---|---|---|---|---|
| D-8763 | Me | CH₂ | H | *c* -Pr(1-CN) | CH₂OC (=O)(i -Pr) | Me | Me | O |
| D-8764 | H | CH(Me) | H | *c* -Pr(1-CN) | CH₂OC (=O)(i -Pr) | Me | Me | O |
| D-8765 | H | CH₂CH₂ | Me | *c* -Pr(1-CN) | CH₂OC(=O)(*i*-Pr) | Me | Me | O |
| D-8766 | Me | CH₂CH₂ | H | *c* -Pr(1-CN) | CH₂OC(=O)(*i*-Pr) | Me | Me | O |
| D-8767 | H | single bond | Me | *c* -Pr(1-CN) | CH₂OC(=O)(*t*-Bu) | Cl | Me | O |
| D-8768 | Me | single bond | H | *c*-Pr(1-CN) | CH₂OC(=O)(*t*-Bu) | Cl | Me | O |
| D-8769 | Me(R) | single bond | H | *c* -Pr(1-CN) | CH₂OC(=O)(*t*-Bu) | Cl | Me | O |
| D-8770 | Me(S) | single bond | H | *c* -Pr(1-CN) | CH₂OC(=O)(*t*-Bu) | Cl | Me | O |
| D-8771 | H | CH₂ | Me | *c* -Pr(1-CN) | CH₂OC(=O)(*t*-Bu) | Cl | Me | O |
| D-8772 | Me | CH₂ | H | *c* -Pr(1-CN) | CH₂OC(=O)(*t*-Bu) | Cl | Me | O |
| D-8773 | H | CH(Me) | H | *c* -Pr(1-CN) | CH₂OC(=O)(*t*-Bu) | Cl | Me | O |
| D-8774 | H | CH₂CH₂ | Me | *c* -Pr(1-CN) | CH₂OC(=O)(*t*-Bu) | Cl | Me | O |
| D-8775 | Me | CH₂CH₂ | H | *c*-Pr(1-CN) | CH₂OC(=O)(*t*-Bu) | Cl | Me | O |
| D-8776 | H | single bond | Me | *c* -Pr(1-CN) | CH₂OC(=O)(*t*-Bu) | Me | Me | O |
| D-8777 | Me | single bond | H | *c* -Pr(1-CN) | CH₂OC(=O)(*t*-Bu) | Me | Me | O |
| D-8778 | Me(R) | single bond | H | *c* -Pr(1-CN) | CH₂OC(=O)(*t*-Bu) | Me | Me | O |
| D-8779 | Me(S) | single bond | H | *c* -Pr(1-CN) | CH₂OC(=O)(*t*-Bu) | Me | Me | O |
| D-8780 | H | CH₂ | Me | *c* -Pr(1-CN) | CH₂OC(=O)(*t*-Bu) | Me | Me | O |
| D-8781 | Me | CH₂ | H | *c* -Pr(1-CN) | CH₂OC(=O)(*t*-Bu) | Me | Me | O |
| D-8782 | H | CH(Me) | H | *c* -Pr(1-CN) | CH₂OC(=O)(*t*-Bu) | Me | Me | O |
| D-8783 | H | CH₂CH₂ | Me | *c* -Pr(1-CN) | CH₂OC(=O)(*t*-Bu) | Me | Me | O |
| D-8784 | Me | CH₂CH₂ | H | *c* -Pr(1-CN) | CH₂OC(=O)(*t*-Bu) | Me | Me | O |
| D-8785 | H | single bond | Me | *c* -Pr(1-CN) | CH₂OC(=O)OEt | Cl | Me | O |
| D-8786 | Me | single bond | H | *c* -Pr(1-CN) | CH₂OC(=O)OEt | Cl | Me | O |
| D-8787 | Me(R) | single bond | H | *c* -Pr(1-CN) | CH₂OC(=O)OEt | Cl | Me | O |
| D-8788 | Me(S) | single bond | H | *c* -Pr(1-CN) | CH₂OC(=O)OEt | Cl | Me | O |
| D-8789 | H | CH₂ | Me | *c* -Pr(1-CN) | CH₂OC(=O)OEt | Cl | Me | O |
| D-8790 | Me | CH₂ | H | *c* -Pr(1-CN) | CH₂OC(=O)OEt | Cl | Me | O |
| D-8791 | H | CH(Me) | H | *c* -Pr(1-CN) | CH₂OC(=O)OEt | Cl | Me | O |
| D-8792 | H | CH₂CH₂ | Me | *c* -Pr(1-CN) | CH₂OC(=O)OEt | Cl | Me | O |
| D-8793 | Me | CH₂CH₂ | H | *c* -Pr(1-CN) | CH₂OC(=O)OEt | Cl | Me | O |
| D-8794 | H | single bond | Me | *c* -Pr(1-CN) | CH₂OC(=O)OEt | Me | Me | O |
| D-8795 | Me | single bond | H | *c* -Pr(1-CN) | CH₂OC(=O)OEt | Me | Me | O |
| D-8796 | Me(R) | single bond | H | *c* -Pr(1-CN) | CH₂OC(=O)OEt | Me | Me | O |
| D-8797 | Me(S) | single bond | H | *c* -Pr(1-CN) | CH₂OC(=O)OEt | Me | Me | O |
| D-8798 | H | CH₂ | Me | *c* -Pr(1-CN) | CH₂OC(=O)OEt | Me | Me | O |
| D-8799 | Me | CH₂ | H | *c* -Pr(1-CN) | CH₂OC(=O)OEt | Me | Me | O |
| D-8800 | H | CH(Me) | H | *c* -Pr(1-CN) | CH₂OC(=O)OEt | Me | Me | O |
| D-8801 | H | CH₂CH₂ | Me | *c* -Pr(1-CN) | CH₂OC(=O)OEt | Me | Me | O |
| D-8802 | Me | CH₂CH₂ | H | *c* -Pr(1-CN) | CH₂OC(=O)OEt | Me | Me | O |
| D-8803 | H | single bond | Me | *c* -Pr(1-CN) | CH₂OC(=O)O(*i*-Pr) | Cl | Me | O |
| D-8804 | Me | single bond | H | *c* -Pr(1-CN) | CH₂OC(=O)O(*i*-Pr) | Cl | Me | O |
| D-8805 | Me(R) | single bond | H | *c* -Pr(1-CN) | CH₂OC(=O)O(*i*-Pr) | Cl | Me | O |
| D-8806 | Me(S) | single bond | H | *c* -Pr(1-CN) | CH₂OC(=O)O(*i*-Pr) | Cl | Me | O |

**[Table 846]**

| Compound Number | Y | W | Z | R¹ | R² | R³ | R¹⁰ | A |
|---|---|---|---|---|---|---|---|---|
| D-8807 | H | CH₂ | Me | *c* -Pr(1-CN) | CH₂OC(=O)O(*i*-Pr) | Cl | Me | O |
| D-8808 | Me | CH₂ | H | *c* -Pr(1-CN) | CH₂OC(=O)O(*i*-Pr) | Cl | Me | O |
| D-8809 | H | CH(Me) | H | *c* -Pr(1-CN) | CH₂OC(=O)O(*i*-Pr) | Cl | Me | O |
| D-8810 | H | CH₂CH₂ | Me | *c* -Pr(1-CN) | CH₂OC(=O)O(*i*-Pr) | Cl | Me | O |
| D-8811 | Me | CH₂CH₂ | H | *c* -Pr(1-CN) | CH₂OC(=O)O(*i*-Pr) | Cl | Me | O |
| D-8812 | H | single bond | Me | *c* -Pr(1-CN) | CH₂OC(=O)O(*i*-Pr) | Me | Me | O |
| D-8813 | Me | single bond | H | *c* -Pr(1-CN) | CH₂OC(=O)O(*i*-Pr) | Me | Me | O |
| D-8814 | Me(R) | single bond | H | *c* -Pr(1-CN) | CH₂OC(=O)O(*i*-Pr) | Me | Me | O |
| D-8815 | Me(S) | single bond | H | *c* -Pr(1-CN) | CH₂OC(=O)O(*i*-Pr) | Me | Me | O |
| D-8816 | H | CH₂ | Me | *c* -Pr(1-CN) | CH₂OC(=O)O(*i*-Pr) | Me | Me | O |
| D-8817 | Me | CH₂ | H | *c* -Pr(1-CN) | CH₂OC(=O)O(*i*-Pr) | Me | Me | O |
| D-8818 | H | CH(Me) | H | *c* -Pr(1-CN) | CH₂OC(=O)O(*i*-Pr) | Me | Me | O |
| D-8819 | H | CH₂CH₂ | Me | *c* -Pr(1-CN) | CH₂OC(=O)O(*i*-Pr) | Me | Me | O |
| D-8820 | Me | CH₂CH₂ | H | *c* -Pr(1-CN) | CH₂OC(=O)O(*i*-Pr) | Me | Me | O |
| D-8821 | H | single bond | Me | *c*-Pr(1-CN) | CH₂N(Me)C(=O)OMe | Cl | Me | O |
| D-8822 | Me | single bond | H | *c* -Pr(1-CN) | CH₂N(Me)C(=O)OMe | Cl | Me | O |
| D-8823 | Me(R) | single bond | H | *c* -Pr(1-CN) | CH₂N(Me)C(=O)OMe | Cl | Me | O |
| D-8824 | Me(S) | single bond | H | *c* -Pr(1-CN) | CH₂N(Me)C(=O)OMe | Cl | Me | O |
| D-8825 | H | CH₂ | Me | *c* -Pr(1-CN) | CH₂N(Me)C(=O)OMe | Cl | Me | O |
| D-8826 | Me | CH₂ | H | *c* -Pr(1-CN) | CH₂N(Me)C(=O)OMe | Cl | Me | O |
| D-8827 | H | CH(Me) | H | *c* -Pr(1-CN) | CH₂N(Me)C(=O)OMe | Cl | Me | O |
| D-8828 | H | CH₂CH₂ | Me | *c* -Pr(1-CN) | CH₂N(Me)C(=O)OMe | Cl | Me | O |
| D-8829 | Me | CH₂CH₂ | H | *c* -Pr(1-CN) | CH₂N(Me)C(=O)OMe | Cl | Me | O |
| D-8830 | H | single bond | Me | *c*-Pr(1-CN) | CH₂N(Me)C(=O)OMe | Me | Me | O |
| D-8831 | Me | single bond | H | *c* -Pr(1-CN) | CH₂N(Me)C(=O)OMe | Me | Me | O |
| D-8832 | Me(R) | single bond | H | *c* -Pr(1-CN) | CH₂N(Me)C(=O)OMe | Me | Me | O |
| D-8833 | Me(S) | single bond | H | *c* -Pr(1-CN) | CH₂N(Me)C(=O)OMe | Me | Me | O |
| D-8834 | H | CH₂ | Me | *c* -Pr(1-CN) | CH₂N(Me)C(=O)OMe | Me | Me | O |
| D-8835 | Me | CH₂ | H | *c* -Pr(1-CN) | CH₂N(Me)C(=O)OMe | Me | Me | O |
| D-8836 | H | CH(Me) | H | *c* -Pr(1-CN) | CH₂N(Me)C(=O)OMe | Me | Me | O |
| D-8837 | H | CH₂CH₂ | Me | *c* -Pr(1-CN) | CH₂N(Me)C(=O)OMe | Me | Me | O |
| D-8838 | Me | CH₂CH₂ | H | *c* -Pr(1-CN) | CH₂N(Me)C(=O)OMe | Me | Me | O |
| D-8839 | H | single bond | Me | *c* -Pr(1-CN) | C(=O)OCH₂CH=CH₂ | Cl | CH₂OMe | O |
| D-8840 | Me | single bond | H | *c* -Pr(1-CN) | C(=O)OCH₂CH=CH₂ | Cl | CH₂OMe | O |
| D-8841 | Me(R) | single bond | H | *c* -Pr(1-CN) | C(=O)OCH₂CH=CH₂ | Cl | CH₂OMe | O |
| D-8842 | Me(S) | single bond | H | *c* -Pr(1-CN) | C(=O)OCH₂CH=CH₂ | Cl | CH₂OMe | O |
| D-8843 | H | CH₂ | Me | *c* -Pr(1-CN) | C(=O)OCH₂CH=CH₂ | Cl | CH₂OMe | O |
| D-8844 | Me | CH₂ | H | *c* -Pr(1-CN) | C(=O)OCH₂CH=CH₂ | Cl | CH₂OMe | O |
| D-8845 | H | CH(Me) | H | *c* -Pr(1-CN) | C(=O)OCH₂CH=CH₂ | Cl | CH₂OMe | O |
| D-8846 | H | CH₂CH₂ | Me | *c* -Pr(1-CN) | C(=O)OCH₂CH=CH₂ | Cl | CH₂OMe | O |
| D-8847 | Me | CH₂CH₂ | H | *c* -Pr(1-CN) | C(=O)OCH₂CH=CH₂ | Cl | CH₂OMe | O |
| D-8848 | H | single bond | Me | *c* -Pr(1-CN) | C(=O)OCH₂CH=CH₂ | Me | CH₂OMe | O |
| D-8849 | Me | single bond | H | *c* -Pr(1-CN) | C(=O)OCH₂CH=CH₂ | Me | CH₂OMe | O |
| D-8850 | Me(R) | single bond | H | *c*-Pr(1-CN) | C(=O)OCH₂CH=CH₂ | Me | CH₂OMe | O |

**[Table 847]**

| Compound Number | Y | W | Z | R¹ | R² | R³ | R¹⁰ | A |
|---|---|---|---|---|---|---|---|---|
| D-8851 | Me(S) | single bond | H | *c* -Pr(1-CN) | C(=O)OCH₂CH=CH₂ | Me | CH₂OMe | O |
| D-8852 | H | CH₂ | Me | *c* -Pr(1-CN) | C(=O)OCH₂CH=CH₂ | Me | CH₂OMe | O |
| D-8853 | Me | CH₂ | H | *c* -Pr(1-CN) | C(=O)OCH₂CH=CH₂ | Me | CH₂OMe | O |
| D-8854 | H | CH(Me) | H | *c* -Pr(1-CN) | C(=O)OCH₂CH=CH₂ | Me | CH₂OMe | O |
| D-8855 | H | CH₂CH₂ | Me | *c* -Pr(1-CN) | C(=O)OCH₂CH=CH₂ | Me | CH₂OMe | O |
| D-8856 | Me | CH₂CH₂ | H | *c*-Pr(1-CN) | C(=O)OCH₂CH=CH₂ | Me | CH₂OMe | O |
| D-8857 | H | single bond | Me | *c* -Pr(1-CN) | C(=O)OCH₂CCl₃ | Cl | CH₂OMe | O |
| D-8858 | Me | single bond | H | *c* -Pr(1-CN) | C(=O)OCH₂CCl₃ | Cl | CH₂OMe | O |
| D-8859 | Me(R) | single bond | H | *c* -Pr(1-CN) | C(=O)OCH₂CCl₃ | Cl | CH₂OMe | O |
| D-8860 | Me(S) | single bond | H | *c* -Pr(1-CN) | C(=O)OCH₂CCl₃ | Cl | CH₂OMe | O |
| D-8861 | H | CH₂ | Me | *c* -Pr(1-CN) | C(=O)OCH₂CCl₃ | Cl | CH₂OMe | O |
| D-8862 | Me | CH₂ | H | *c* -Pr(1-CN) | C(=O)OCH₂CCl₃ | Cl | CH₂OMe | O |
| D-8863 | H | CH(Me) | H | *c*-Pr(1-CN) | C(=O)OCH₂CCl₃ | Cl | CH₂OMe | O |
| D-8864 | H | CH₂CH₂ | Me | *c* -Pr(1-CN) | C(=O)OCH₂CCl₃ | Cl | CH₂OMe | O |
| D-8865 | Me | CH₂CH₂ | H | *c* -Pr(1-CN) | C(=O)OCH₂CCl₃ | Cl | CH₂OMe | O |
| D-8866 | H | single bond | Me | *c* -Pr(1-CN) | C(=O)OCH₂CCl₃ | Me | CH₂OMe | O |
| D-8867 | Me | single bond | H | *c* -Pr(1-CN) | C(=O)OCH₂CCl₃ | Me | CH₂OMe | O |
| D-8868 | Me(R) | single bond | H | *c* -Pr(1-CN) | C(=O)OCH₂CCl₃ | Me | CH₂OMe | O |
| D-8869 | Me(S) | single bond | H | *c* -Pr(1-CN) | C(=O)OCH₂CCl₃ | Me | CH₂OMe | O |
| D-8870 | H | CH₂ | Me | *c* -Pr(1-CN) | C(=O)OCH₂CCl₃ | Me | CH₂OMe | O |
| D-8871 | Me | CH₂ | H | *c* -Pr(1-CN) | C(=O)OCH₂CCl₃ | Me | CH₂OMe | O |
| D-8872 | H | CH(Me) | H | *c* -Pr(1-CN) | C(=O)OCH₂CCl₃ | Me | CH₂OMe | O |
| D-8873 | H | CH₂CH₂ | Me | *c* -Pr(1-CN) | C(=O)OCH₂CCl₃ | Me | CH₂OMe | O |
| D-8874 | Me | CH₂CH₂ | H | *c* -Pr(1-CN) | C(=O)OCH₂CCl₃ | Me | CH₂OMe | O |
| D-8875 | H | single bond | Me | *c* -Pr(1-CN) | C(=O)N(Me)₂ | Cl | CH₂OMe | O |
| D-8876 | Me | single bond | H | *c* -Pr(1-CN) | C(=O)N(Me)₂ | Cl | CH₂OMe | O |
| D-8877 | Me(R) | single bond | H | *c* -Pr(1-CN) | C(=O)N(Me)₂ | Cl | CH₂OMe | O |
| D-8878 | Me(S) | single bond | H | *c* -Pr(1-CN) | C(=O)N(Me)₂ | Cl | CH₂OMe | O |
| D-8879 | H | CH₂ | Me | *c* -Pr(1-CN) | C(=O)N(Me)₂ | Cl | CH₂OMe | O |
| D-8880 | Me | CH₂ | H | *c* -Pr(1-CN) | C(=O)N(Me)₂ | Cl | CH₂OMe | O |
| D-8881 | H | CH(Me) | H | *c* -Pr(1-CN) | C(=O)N(Me)₂ | Cl | CH₂OMe | O |
| D-8882 | H | CH₂CH₂ | Me | *c* -Pr(1-CN) | C(=O)N(Me)₂ | Cl | CH₂OMe | O |
| D-8883 | Me | CH₂CH₂ | H | *c* -Pr(1-CN) | C(=O)N(Me)₂ | Cl | CH₂OMe | O |
| D-8884 | H | single bond | Me | *c* -Pr(1-CN) | C(=O)N(Me)₂ | Me | CH₂OMe | O |
| D-8885 | Me | single bond | H | *c* -Pr(1-CN) | C(=O)N(Me)₂ | Me | CH₂OMe | O |
| D-8886 | Me(R) | single bond | H | *c* -Pr(1-CN) | C(=O)N(Me)₂ | Me | CH₂OMe | O |
| D-8887 | Me(S) | single bond | H | *c* -Pr(1-CN) | C(=O)N(Me)₂ | Me | CH₂OMe | O |
| D-8888 | H | CH₂ | Me | *c* -Pr(1-CN) | C(=O)N(Me)₂ | Me | CH₂OMe | O |
| D-8889 | Me | CH₂ | H | *c* -Pr(1-CN) | C(=O)N(Me)₂ | Me | CH₂OMe | O |
| D-8890 | H | CH(Me) | H | *c* -Pr(1-CN) | C(=O)N(Me)₂ | Me | CH₂OMe | O |
| D-8891 | H | CH₂CH₂ | Me | *c* -Pr(1-CN) | C(=O)N(Me)₂ | Me | CH₂OMe | O |
| D-8892 | Me | CH₂CH₂ | H | *c* -Pr(1-CN) | C(=O)N(Me)₂ | Me | CH₂OMe | O |
| D-8893 | H | single bond | Me | *c* -Pr(1-CN) | CH₂OC(=O)Me | Cl | CH₂OMe | O |
| D-8894 | Me | single bond | H | *c* -Pr(1-CN) | CH₂OC(=O)Me | Cl | CH₂OMe | O |

**[Table 848]**

| Compound Number | Y | W | Z | R¹ | R² | R³ | R¹⁰ | A |
|---|---|---|---|---|---|---|---|---|
| D-8895 | Me(R) | single bond | H | *c* -Pr(1-CN) | CH₂OC(=O)Me | Cl | CH₂OMe | O |
| D-8896 | Me(S) | single bond | H | *c* -Pr(1-CN) | CH₂OC(=O)Me | Cl | CH₂OMe | O |
| D-8897 | H | CH₂ | Me | *c* -Pr(1-CN) | CH₂OC(=O)Me | Cl | CH₂OMe | O |
| D-8898 | Me | CH₂ | H | *c* -Pr(1-CN) | CH₂OC(=O)Me | Cl | CH₂OMe | O |
| D-8899 | H | CH(Me) | H | *c* -Pr(1-CN) | CH₂OC(=O)Me | Cl | CH₂OMe | O |
| D-8900 | H | CH₂CH₂ | Me | *c* -Pr(1-CN) | CH₂OC(=O)Me | Cl | CH₂OMe | O |
| D-8901 | Me | CH₂CH₂ | H | *c* -Pr(1-CN) | CH₂OC(=O)Me | Cl | CH₂OMe | O |
| D-8902 | H | single bond | Me | *c*-Pr(1-CN) | CH₂OC(=O)Me | Me | CH₂OMe | O |
| D-8903 | Me | single bond | H | *c* -Pr(1-CN) | CH₂OC(=O)Me | Me | CH₂OMe | O |
| D-8904 | Me(R) | single bond | H | *c* -Pr(1-CN) | CH₂OC(=O)Me | Me | CH₂OMe | O |
| D-8905 | Me(S) | single bond | H | *c* -Pr(1-CN) | CH₂OC(=O)Me | Me | CH₂OMe | O |
| D-8906 | H | CH₂ | Me | *c* -Pr(1-CN) | CH₂OC(=O)Me | Me | CH₂OMe | O |
| D-8907 | Me | CH₂ | H | *c* -Pr(1-CN) | CH₂OC(=O)Me | Me | CH₂OMe | O |
| D-8908 | H | CH(Me) | H | *c* -Pr(1-CN) | CH₂OC(=O)Me | Me | CH₂OMe | O |
| D-8909 | H | CH₂CH₂ | Me | *c* -Pr(1-CN) | CH₂OC(=O)Me | Me | CH₂OMe | O |
| D-8910 | Me | CH₂CH₂ | H | *c* -Pr(1-CN) | CH₂OC(=O)Me | Me | CH₂OMe | O |
| D-8911 | H | single bond | Me | *c* -Pr(1-CN) | CH₂OC(=O)(*i*-Pr) | Cl | CH₂OMe | O |
| D-8912 | Me | single bond | H | *c* -Pr(1-CN) | CH₂OC(=O)(*i*-Pr) | Cl | CH₂OMe | O |
| D-8913 | Me(R) | single bond | H | *c* -Pr(1-CN) | CH₂OC(=O)(*i*-Pr) | Cl | CH₂OMe | O |
| D-8914 | Me(S) | single bond | H | *c* -Pr(1-CN) | CH₂OC(=O)(*i*-Pr) | Cl | CH₂OMe | O |
| D-8915 | H | CH₂ | Me | *c* -Pr(1-CN) | CH₂OC(=O)(*i*-Pr) | Cl | CH₂OMe | O |
| D-8916 | Me | CH₂ | H | *c* -Pr(1-CN) | CH₂OC (=O)(i -Pr) | Cl | CH₂OMe | O |
| D-8917 | H | CH(Me) | H | *c* -Pr(1-CN) | CH₂OC (=O)(i -Pr) | Cl | CH₂OMe | O |
| D-8918 | H | CH₂CH₂ | Me | *c* -Pr(1-CN) | CH₂OC (=O)(/ -Pr) | Cl | CH₂OMe | O |
| D-8919 | Me | CH₂CH₂ | H | *c* -Pr(1-CN) | CH₂OC (=O)(/ -Pr) | Cl | CH₂OMe | O |
| D-8920 | H | single bond | Me | *c* -Pr(1-CN) | CH₂OC (=O)(i -Pr) | Me | CH₂OMe | O |
| D-8921 | Me | single bond | H | *c* -Pr(1-CN) | CH₂OC(=O)(*i*-Pr) | Me | CH₂OMe | O |
| D-8922 | Me(R) | single bond | H | *c* -Pr(1-CN) | CH₂OC(=O)(*i*-Pr) | Me | CH₂OMe | O |
| D-8923 | Me(S) | single bond | H | *c* -Pr(1-CN) | CH₂OC (=O)(i -Pr) | Me | CH₂OMe | O |
| D-8924 | H | CH₂ | Me | *c* -Pr(1-CN) | CH₂OC (=O)(i -Pr) | Me | CH₂OMe | O |
| D-8925 | Me | CH₂ | H | *c* -Pr(1-CN) | CH₂OC(=O)(*i*-Pr) | Me | CH₂OMe | O |
| D-8926 | H | CH(Me) | H | *c* -Pr(1-CN) | CH₂OC (=O)(*i*-Pr) | Me | CH₂OMe | O |
| D-8927 | H | CH₂CH₂ | Me | *c* -Pr(1-CN) | CH₂OC (=O)(i -Pr) | Me | CH₂OMe | O |
| D-8928 | Me | CH₂CH₂ | H | *c* -Pr(1-CN) | CH₂OC(=O)(*i*-Pr) | Me | CH₂OMe | O |
| D-8929 | H | single bond | Me | *c* -Pr(1-CN) | CH₂OC(=O)(*t*-Bu) | Cl | CH₂OMe | O |
| D-8930 | Me | single bond | H | *c* -Pr(1-CN) | CH₂OC(=O)(*t*-Bu) | Cl | CH₂OMe | O |
| D-8931 | Me(R) | single bond | H | *c* -Pr(1-CN) | CH₂OC(=O)(*t*-Bu) | Cl | CH₂OMe | O |
| D-8932 | Me(S) | single bond | H | *c* -Pr(1-CN) | CH₂OC(=O)(*t*-Bu) | Cl | CH₂OMe | O |
| D-8933 | H | CH₂ | Me | *c* -Pr(1-CN) | CH₂OC(=O)(*t*-Bu) | Cl | CH₂OMe | O |
| D-8934 | Me | CH₂ | H | *c* -Pr(1-CN) | CH₂OC(=O)(*t*-Bu) | Cl | CH₂OMe | O |
| D-8935 | H | CH(Me) | H | *c* -Pr(1-CN) | CH₂OC(=O)(*t*-Bu) | Cl | CH₂OMe | O |
| D-8936 | H | CH₂CH₂ | Me | *c* -Pr(1-CN) | CH₂OC(=O)(*t*-Bu) | Cl | CH₂OMe | O |
| D-8937 | Me | CH₂CH₂ | H | *c* -Pr(1-CN) | CH₂OC(=O)(*t*-Bu) | Cl | CH₂OMe | O |
| D-8938 | H | single bond | Me | *c*-Pr(1-CN) | CH₂OC(=O)(*t*-Bu) | Me | CH₂OMe | O |

**[Table 849]**

| Compound Number | Y | W | Z | R¹ | R² | R³ | R¹⁰ | A |
|---|---|---|---|---|---|---|---|---|
| D-8939 | Me | single bond | H | *c* -Pr(1-CN) | CH₂OC(=O)(*t*-Bu) | Me | CH₂OMe | O |
| D-8940 | Me(R) | single bond | H | *c* -Pr(1-CN) | CH₂OC(=O)(*t*-Bu) | Me | CH₂OMe | O |
| D-8941 | Me(S) | single bond | H | *c* -Pr(1-CN) | CH₂OC(=O)(*t*-Bu) | Me | CH₂OMe | O |
| D-8942 | H | CH₂ | Me | *c* -Pr(1-CN) | CH₂OC(=O)(*t*-Bu) | Me | CH₂OMe | O |
| D-8943 | Me | CH₂ | H | *c* -Pr(1-CN) | CH₂OC(=O)(*t*-Bu) | Me | CH₂OMe | O |
| D-8944 | H | CH(Me) | H | *c*-Pr(1-CN) | CH₂OC(=O)(*t*-Bu) | Me | CH₂OMe | O |
| D-8945 | H | CH₂CH₂ | Me | *c* -Pr(1-CN) | CH₂OC(=O)(*t*-Bu) | Me | CH₂OMe | O |
| D-8946 | Me | CH₂CH₂ | H | *c* -Pr(1-CN) | CH₂OC(=O)(*t*-Bu) | Me | CH₂OMe | O |
| D-8947 | H | single bond | Me | *c* -Pr(1-CN) | CH₂OC(=O)OEt | Cl | CH₂OMe | O |
| D-8948 | Me | single bond | H | *c* -Pr(1-CN) | CH₂OC(=O)OEt | Cl | CH₂OMe | O |
| D-8949 | Me(R) | single bond | H | *c* -Pr(1-CN) | CH₂OC(=O)OEt | Cl | CH₂OMe | O |
| D-8950 | Me(S) | single bond | H | *c* -Pr(1-CN) | CH₂OC(=O)OEt | Cl | CH₂OMe | O |
| D-8951 | H | CH₂ | Me | *c* -Pr(1-CN) | CH₂OC(=O)OEt | Cl | CH₂OMe | O |
| D-8952 | Me | CH₂ | H | *c* -Pr(1-CN) | CH₂OC(=O)OEt | Cl | CH₂OMe | O |
| D-8953 | H | CH(Me) | H | *c* -Pr(1-CN) | CH₂OC(=O)OEt | Cl | CH₂OMe | O |
| D-8954 | H | CH₂CH₂ | Me | *c* -Pr(1-CN) | CH₂OC(=O)OEt | Cl | CH₂OMe | O |
| D-8955 | Me | CH₂CH₂ | H | *c* -Pr(1-CN) | CH₂OC(=O)OEt | Cl | CH₂OMe | O |
| D-8956 | H | single bond | Me | *c* -Pr(1-CN) | CH₂OC(=O)OEt | Me | CH₂OMe | O |
| D-8957 | Me | single bond | H | *c* -Pr(1-CN) | CH₂OC(=O)OEt | Me | CH₂OMe | O |
| D-8958 | Me(R) | single bond | H | *c* -Pr(1-CN) | CH₂OC(=O)OEt | Me | CH₂OMe | O |
| D-8959 | Me(S) | single bond | H | *c* -Pr(1-CN) | CH₂OC(=O)OEt | Me | CH₂OMe | O |
| D-8960 | H | CH₂ | Me | *c* -Pr(1-CN) | CH₂OC(=O)OEt | Me | CH₂OMe | O |
| D-8961 | Me | CH₂ | H | *c* -Pr(1-CN) | CH₂OC(=O)OEt | Me | CH₂OMe | O |
| D-8962 | H | CH(Me) | H | *c* -Pr(1-CN) | CH₂OC(=O)OEt | Me | CH₂OMe | O |
| D-8963 | H | CH₂CH₂ | Me | *c* -Pr(1-CN) | CH₂OC(=O)OEt | Me | CH₂OMe | O |
| D-8964 | Me | CH₂CH₂ | H | *c* -Pr(1-CN) | CH₂OC(=O)OEt | Me | CH₂OMe | O |
| D-8965 | H | single bond | Me | *c* -Pr(1-CN) | CH₂OC(=O)O(*i*-Pr) | Cl | CH₂OMe | O |
| D-8966 | Me | single bond | H | *c* -Pr(1-CN) | CH₂OC(=O)O(*i*-Pr) | Cl | CH₂OMe | O |
| D-8967 | Me(R) | single bond | H | *c* -Pr(1-CN) | CH₂OC(=O)O(*i*-Pr) | Cl | CH₂OMe | O |
| D-8968 | Me(S) | single bond | H | *c* -Pr(1-CN) | CH₂OC(=O)O(*i*-Pr) | Cl | CH₂OMe | O |
| D-8969 | H | CH₂ | Me | *c* -Pr(1-CN) | CH₂OC(=O)O(*i*-Pr) | Cl | CH₂OMe | O |
| D-8970 | Me | CH₂ | H | *c* -Pr(1-CN) | CH₂OC(=O)O(*i*-Pr) | Cl | CH₂OMe | O |
| D-8971 | H | CH(Me) | H | *c* -Pr(1-CN) | CH₂OC(=O)O(*i*-Pr) | Cl | CH₂OMe | O |
| D-8972 | H | CH₂CH₂ | Me | *c* -Pr(1-CN) | CH₂OC(=O)O(*i*-Pr) | Cl | CH₂OMe | O |
| D-8973 | Me | CH₂CH₂ | H | *c* -Pr(1-CN) | CH₂OC(=O)O(*i*-Pr) | Cl | CH₂OMe | O |
| D-8974 | H | single bond | Me | *c* -Pr(1-CN) | CH₂OC(=O)O(*i*-Pr) | Me | CH₂OMe | O |
| D-8975 | Me | single bond | H | *c* -Pr(1-CN) | CH₂OC(=O)O(*i*-Pr) | Me | CH₂OMe | O |
| D-8976 | Me(R) | single bond | H | *c* -Pr(1-CN) | CH₂OC(=O)O(*i*-Pr) | Me | CH₂OMe | O |
| D-8977 | Me(S) | single bond | H | *c* -Pr(1-CN) | CH₂OC(=O)O(*i*-Pr) | Me | CH₂OMe | O |
| D-8978 | H | CH₂ | Me | *c* -Pr(1-CN) | CH₂OC(=O)O(*i*-Pr) | Me | CH₂OMe | O |
| D-8979 | Me | CH₂ | H | *c* -Pr(1-CN) | CH₂OC(=O)O(*i*-Pr) | Me | CH₂OMe | O |
| D-8980 | H | CH(Me) | H | *c* -Pr(1-CN) | CH₂OC(=O)O(*i*-Pr) | Me | CH₂OMe | O |
| D-8981 | H | CH₂CH₂ | Me | *c* -Pr(1-CN) | CH₂OC(=O)O(*i*-Pr) | Me | CH₂OMe | O |
| D-8982 | Me | CH₂CH₂ | H | *c* -Pr(1-CN) | CH₂OC(=O)O(*i*-Pr) | Me | CH₂OMe | O |

**[Table 850]**

| Compound Number | Y | W | Z | R¹ | R² | R³ | R¹⁰ | A |
|---|---|---|---|---|---|---|---|---|
| D-8983 | H | single bond | Me | *c*-Pr(1-CN) | CH₂N(Me)C(=O)OMe | Cl | CH₂OMe | O |
| D-8984 | Me | single bond | H | *c*-Pr(1-CN) | CH₂N(Me)C(=O)OMe | Cl | CH₂OMe | O |
| D-8985 | Me(R) | single bond | H | *c*-Pr(1-CN) | CH₂N(Me)C(=O)OMe | Cl | CH₂OMe | O |
| D-8986 | Me(S) | single bond | H | *c*-Pr(1-CN) | CH₂N(Me)C(=O)OMe | Cl | CH₂OMe | O |
| D-8987 | H | CH₂ | Me | *c*-Pr(1-CN) | CH₂N(Me)C(=O)OMe | Cl | CH₂OMe | O |
| D-8988 | Me | CH₂ | H | *c*-Pr(1-CN) | CH₂N(Me)C(=O)OMe | Cl | CH₂OMe | O |
| D-8989 | H | CH(Me) | H | *c*-Pr(1-CN) | CH₂N(Me)C(=O)OMe | Cl | CH₂OMe | O |
| D-8990 | H | CH₂CH₂ | Me | *c*-Pr(1-CN) | CH₂N(Me)C(=O)OMe | Cl | CH₂OMe | O |
| D-8991 | Me | CH₂CH₂ | H | *c*-Pr(1-CN) | CH₂N(Me)C(=O)OMe | Cl | CH₂OMe | O |
| D-8992 | H | single bond | Me | *c*-pr(1-CN) | CH₂N(Me)C(=O)OMe | Me | CH₂OMe | O |
| D-8993 | Me | single bond | H | *c*-Pr(1-CN) | CH₂N(Me)C(=O)OMe | Me | CH₂OMe | O |
| D-8994 | Me(R) | single bond | H | *c*-Pr(1-CN) | CH₂N(Me)C(=O)OMe | Me | CH₂OMe | O |
| D-8995 | Me(S) | single bond | H | *c*-Pr(1-CN) | CH₂N(Me)C(=O)OMe | Me | CH₂OMe | O |
| D-8996 | H | CH₂ | Me | *c*-Pr(1-CN) | CH₂N(Me)C(=O)OMe | Me | CH₂OMe | O |
| D-8997 | Me | CH₂ | H | *c*-Pr(1-CN) | CH₂N(Me)C(=O)OMe | Me | CH₂OMe | O |
| D-8998 | H | CH(Me) | H | *c*-Pr(1-CN) | CH₂N(Me)C(=O)OMe | Me | CH₂OMe | O |
| D-8999 | H | CH₂CH₂ | Me | *c*-Pr(1-CN) | CH₂N(Me)C(=O)OMe | Me | CH₂OMe | O |
| D-9000 | Me | CH₂CH₂ | H | *c*-Pr(1-CN) | CH₂N(Me)C(=O)OMe | Me | CH₂OMe | O |
| D-9001 | H | single bond | Me | *c*-Pr(1-CN) | C(=O)OCH₂CH=CH₂ | Cl | CH₂CN | O |
| D-9002 | Me | single bond | H | *c*-Pr(1-CN) | C(=O)OCH₂CH=CH₂ | Cl | CH₂CN | O |
| D-9003 | Me(R) | single bond | H | *c*-Pr(1-CN) | C(=O)OCH₂CH=CH₂ | Cl | CH₂CN | O |
| D-9004 | Me(S) | single bond | H | *c*-Pr(1-CN) | C(=O)OCH₂CH=CH₂ | Cl | CH₂CN | O |
| D-9005 | H | CH₂ | Me | *c*-Pr(1-CN) | C(=O)OCH₂CH=CH₂ | Cl | CH₂CN | O |
| D-9006 | Me | CH₂ | H | *c*-Pr(1-CN) | C(=O)OCH₂CH=CH₂ | Cl | CH₂CN | O |
| D-9007 | H | CH(Me) | H | *c*-Pr(1-CN) | C(=O)OCH₂CH=CH₂ | Cl | CH₂CN | O |
| D-9008 | H | CH₂CH₂ | Me | *c*-Pr(1-CN) | C(=O)OCH₂CH=CH₂ | Cl | CH₂CN | O |
| D-9009 | Me | CH₂CH₂ | H | *c*-Pr(1-CN) | C(=O)OCH₂CH=CH₂ | Cl | CH₂CN | O |
| D-9010 | H | single bond | Me | *c*-Pr(1-CN) | C(=O)OCH₂CH=CH₂ | Me | CH₂CN | O |
| D-9011 | Me | single bond | H | *c*-Pr(1-CN) | C(=O)OCH₂CH=CH₂ | Me | CH₂CN | O |
| D-9012 | Me(R) | single bond | H | *c*-Pr(1-CN) | C(=O)OCH₂CH=CH₂ | Me | CH₂CN | O |
| D-9013 | Me(S) | single bond | H | *c*-Pr(1-CN) | C(=O)OCH₂CH=CH₂ | Me | CH₂CN | O |
| D-9014 | H | CH₂ | Me | *c*-Pr(1-CN) | C(=O)OCH₂CH=CH₂ | Me | CH₂CN | O |
| D-9015 | Me | CH₂ | H | *c*-Pr(1-CN) | C(=O)OCH₂CH=CH₂ | Me | CH₂CN | O |
| D-9016 | H | CH(Me) | H | *c*-Pr(1-CN) | C(=O)OCH₂CH=CH₂ | Me | CH₂CN | O |
| D-9017 | H | CH₂CH₂ | Me | *c*-Pr(1-CN) | C(=O)OCH₂CH=CH₂ | Me | CH₂CN | O |
| D-9018 | Me | CH₂CH₂ | H | *c*-Pr(1-CN) | C(=O)OCH₂CH=CH₂ | Me | CH₂CN | O |
| D-9019 | H | single bond | Me | *c*-Pr(1-CN) | C(=O)OCH₂CCl₃ | Cl | CH₂CN | O |
| D-9020 | Me | single bond | H | *c*-Pr(1-CN) | C(=O)OCH₂CCl₃ | Cl | CH₂CN | O |
| D-9021 | Me(R) | single bond | H | *c*-Pr(1-CN) | C(=O)OCH₂CCl₃ | Cl | CH₂CN | O |
| D-9022 | Me(S) | single bond | H | *c*-Pr(1-CN) | C(=O)OCH₂CCl₃ | Cl | CH₂CN | O |
| D-9023 | H | CH₂ | Me | *c*-Pr(1-CN) | C(=O)OCH₂CCl₃ | Cl | CH₂CN | O |
| D-9024 | Me | CH₂ | H | *c*-Pr(1-CN) | C(=O)OCH₂CCl₃ | Cl | CH₂CN | O |
| D-9025 | H | CH(Me) | H | *c*-Pr(1-CN) | C(=O)OCH₂CCl₃ | Cl | CH₂CN | O |
| D-9026 | H | CH₂CH₂ | Me | *c*-Pr(1-CN) | C(=O)OCH₂CCl₃ | Cl | CH₂CN | O |

**[Table 851]**

| Compound Number | Y | W | Z | R¹ | R² | R³ | R¹⁰ | A |
|---|---|---|---|---|---|---|---|---|
| D-9027 | Me | CH₂CH₂ | H | *c* -Pr(1-CN) | C(=O)OCH₂CCl₃ | Cl | CH₂CN | O |
| D-9028 | H | single bond | Me | *c* -Pr(1-CN) | C(=O)OCH₂CCl₃ | Me | CH₂CN | O |
| D-9029 | Me | single bond | H | *c* -Pr(1-CN) | C(=O)OCH₂CCl₃ | Me | CH₂CN | O |
| D-9030 | Me(R) | single bond | H | *c* -Pr(1-CN) | C(=O)OCH₂CCl₃ | Me | CH₂CN | O |
| D-9031 | Me(S) | single bond | H | *c* -Pr(1-CN) | C(=O)OCH₂CCl₃ | Me | CH₂CN | O |
| D-9032 | H | CH₂ | Me | *c* -Pr(1-CN) | C(=O)OCH₂CCl₃ | Me | CH₂CN | O |
| D-9033 | Me | CH₂ | H | *c* -Pr(1-CN) | C(=O)OCH₂CCl₃ | Me | CH₂CN | O |
| D-9034 | H | CH(Me) | H | *c* -Pr(1-CN) | C(=O)OCH₂CCl₃ | Me | CH₂CN | O |
| D-9035 | H | CH₂CH₂ | Me | *c* -Pr(1-CN) | C(=O)OCH₂CCl₃ | Me | CH₂CN | O |
| D-9036 | Me | CH₂CH₂ | H | *c* -Pr(1-CN) | C(=O)OCH₂CCl₃ | Me | CH₂CN | O |
| D-9037 | H | single bond | Me | *c* -Pr(1-CN) | C(=O)N(Me)₂ | Cl | CH₂CN | O |
| D-9038 | Me | single bond | H | *c* -Pr(1-CN) | C(=O)N(Me)₂ | Cl | CH₂CN | O |
| D-9039 | Me(R) | single bond | H | *c*-Pr(1-CN) | C(=O)N(Me)₂ | Cl | CH₂CN | O |
| D-9040 | Me(S) | single bond | H | *c* -Pr(1-CN) | C(=O)N(Me)₂ | Cl | CH₂CN | O |
| D-9041 | H | CH₂ | Me | *c* -Pr(1-CN) | C(=O)N(Me)₂ | Cl | CH₂CN | O |
| D-9042 | Me | CH₂ | H | *c* -Pr(1-CN) | C(=O)N(Me)₂ | Cl | CH₂CN | O |
| D-9043 | H | CH(Me) | H | *c* -Pr(1-CN) | C(=O)N(Me)₂ | Cl | CH₂CN | O |
| D-9044 | H | CH₂CH₂ | Me | *c* -Pr(1-CN) | C(=O)N(Me)₂ | Cl | CH₂CN | O |
| D-9045 | Me | CH₂CH₂ | H | *c* -Pr(1-CN) | C(=O)N(Me)₂ | Cl | CH₂CN | O |
| D-9046 | H | single bond | Me | *c* -Pr(1-CN) | C(=O)N(Me)₂ | Me | CH₂CN | O |
| D-9047 | Me | single bond | H | *c* -Pr(1-CN) | C(=O)N(Me)₂ | Me | CH₂CN | O |
| D-9048 | Me(R) | single bond | H | *c* -Pr(1-CN) | C(=O)N(Me)₂ | Me | CH₂CN | O |
| D-9049 | Me(S) | single bond | H | *c* -Pr(1-CN) | C(=O)N(Me)₂ | Me | CH₂CN | O |
| D-9050 | H | CH₂ | Me | *c* -Pr(1-CN) | C(=O)N(Me)₂ | Me | CH₂CN | O |
| D-9051 | Me | CH₂ | H | *c* -Pr(1-CN) | C(=O)N(Me)₂ | Me | CH₂CN | O |
| D-9052 | H | CH(Me) | H | *c* -Pr(1-CN) | C(=O)N(Me)₂ | Me | CH₂CN | O |
| D-9053 | H | CH₂CH₂ | Me | *c* -Pr(1-CN) | C(=O)N(Me)₂ | Me | CH₂CN | O |
| D-9054 | Me | CH₂CH₂ | H | *c* -Pr(1-CN) | C(=O)N(Me)₂ | Me | CH₂CN | O |
| D-9055 | H | single bond | Me | *c* -Pr(1-CN) | CH₂OC(=O)Me | Cl | CH₂CN | O |
| D-9056 | Me | single bond | H | *c* -Pr(1-CN) | CH₂OC(=O)Me | Cl | CH₂CN | O |
| D-9057 | Me(R) | single bond | H | *c* -Pr(1-CN) | CH₂OC(=O)Me | Cl | CH₂CN | O |
| D-9058 | Me(S) | single bond | H | *c* -Pr(1-CN) | CH₂OC(=O)Me | Cl | CH₂CN | O |
| D-9059 | H | CH₂ | Me | *c* -Pr(1-CN) | CH₂OC(=O)Me | Cl | CH₂CN | O |
| D-9060 | Me | CH₂ | H | *c* -Pr(1-CN) | CH₂OC(=O)Me | Cl | CH₂CN | O |
| D-9061 | H | CH(Me) | H | *c* -Pr(1-CN) | CH₂OC(=O)Me | Cl | CH₂CN | O |
| D-9062 | H | CH₂CH₂ | Me | *c* -Pr(1-CN) | CH₂OC(=O)Me | Cl | CH₂CN | O |
| D-9063 | Me | CH₂CH₂ | H | *c* -Pr(1-CN) | CH₂OC(=O)Me | Cl | CH₂CN | O |
| D-9064 | H | single bond | Me | *c* -Pr(1-CN) | CH₂OC(=O)Me | Me | CH₂CN | O |
| D-9065 | Me | single bond | H | *c* -Pr(1-CN) | CH₂OC(=O)Me | Me | CH₂CN | O |
| D-9066 | Me(R) | single bond | H | *c* -Pr(1-CN) | CH₂OC(=O)Me | Me | CH₂CN | O |
| D-9067 | Me(S) | single bond | H | *c* -Pr(1-CN) | CH₂OC(=O)Me | Me | CH₂CN | O |
| D-9068 | H | CH₂ | Me | *c* -Pr(1-CN) | CH₂OC(=O)Me | Me | CH₂CN | O |
| D-9069 | Me | CH₂ | H | *c* -Pr(1-CN) | CH₂OC(=O)Me | Me | CH₂CN | O |
| D-9070 | H | CH(Me) | H | *c* -Pr(1-CN) | CH₂OC(=O)Me | Me | CH₂CN | O |

**[Table 852]**

| Compound Number | Y | W | Z | R¹ | R² | R³ | R¹⁰ | A |
|---|---|---|---|---|---|---|---|---|
| D-9071 | H | CH₂CH₂ | Me | *c* -Pr(1-CN) | CH₂OC(=O)Me | Me | CH₂CN | O |
| D-9072 | Me | CH₂CH₂ | H | *c* -Pr(1-CN) | CH₂OC(=O)Me | Me | CH₂CN | O |
| D-9073 | H | single bond | Me | *c* -Pr(1-CN) | CH₂OC(=O)(*i* -Pr) | Cl | CH₂CN | O |
| D-9074 | Me | single bond | H | *c* -Pr(1-CN) | CH₂OC(=O)(*i* -Pr) | Cl | CH₂CN | O |
| D-9075 | Me(R) | single bond | H | *c* -Pr(1-CN) | CH₂OC(=O)(*i* -Pr) | Cl | CH₂CN | O |
| D-9076 | Me(S) | single bond | H | *c*-Pr(1-CN) | CH₂OC(=O)(*i* -Pr) | Cl | CH₂CN | O |
| D-9077 | H | CH₂ | Me | *c* -Pr(1-CN) | CH₂OC(=O)(*i* -Pr) | Cl | CH₂CN | O |
| D-9078 | Me | CH₂ | H | *c* -Pr(1-CN) | CH₂OC(=O)(*i* -Pr) | Cl | CH₂CN | O |
| D-9079 | H | CH(Me) | H | *c* -Pr(1-CN) | CH₂OC(=O)(*i* -Pr) | Cl | CH₂CN | O |
| D-9080 | H | CH₂CH₂ | Me | *c* -Pr(1-CN) | CH₂OC(=O)(*i* -Pr) | Cl | CH₂CN | O |
| D-9081 | Me | CH₂CH₂ | H | *c* -Pr(1-CN) | CH₂OC(=O)(*i* -Pr) | Cl | CH₂CN | O |
| D-9082 | H | single bond | Me | *c* -Pr(1-CN) | CH₂OC(=O)(*i* -Pr) | Me | CH₂CN | O |
| D-9083 | Me | single bond | H | *c*-Pr(1-CN) | CH₂OC(=O)(*i*-Pr) | Me | CH₂CN | O |
| D-9084 | Me(R) | single bond | H | *c* -Pr(1-CN) | CH₂OC(=O)(*i*-Pr) | Me | CH₂CN | O |
| D-9085 | Me(S) | single bond | H | *c* -Pr(1-CN) | CH₂OC(=O)(*i* -Pr) | Me | CH₂CN | O |
| D-9086 | H | CH₂ | Me | *c* -Pr(1-CN) | CH₂OC(=O)(*i* -Pr) | Me | CH₂CN | O |
| D-9087 | Me | CH₂ | H | *c* -Pr(1-CN) | CH₂OC(=O)(*i*-Pr) | Me | CH₂CN | O |
| D-9088 | H | CH(Me) | H | *c* -Pr(1-CN) | CH₂OC(=O)(*i*-Pr) | Me | CH₂CN | O |
| D-9089 | H | CH₂CH₂ | Me | *c* -Pr(1-CN) | CH₂OC(=O)(*i* -Pr) | Me | CH₂CN | O |
| D-9090 | Me | CH₂CH₂ | H | *c* -Pr(1-CN) | CH₂OC(=O)(*i*-Pr) | Me | CH₂CN | O |
| D-9091 | H | single bond | Me | *c* -Pr(1-CN) | CH₂OC(=O)(*t*-Bu) | Cl | CH₂CN | O |
| D-9092 | Me | single bond | H | *c* -Pr(1-CN) | CH₂OC(=O)(*t*-Bu) | Cl | CH₂CN | O |
| D-9093 | Me(R) | single bond | H | *c* -Pr(1-CN) | CH₂OC(=O)(*t*-Bu) | Cl | CH₂CN | O |
| D-9094 | Me(S) | single bond | H | *c* -Pr(1-CN) | CH₂OC(=O)(*t*-Bu) | Cl | CH₂CN | O |
| D-9095 | H | CH₂ | Me | *c* -Pr(1-CN) | CH₂OC(=O)(*t*-Bu) | Cl | CH₂CN | O |
| D-9096 | Me | CH₂ | H | *c* -Pr(1-CN) | CH₂OC(=O)(*t*-Bu) | Cl | CH₂CN | O |
| D-9097 | H | CH(Me) | H | *c* -Pr(1-CN) | CH₂OC(=O)(*t*-Bu) | Cl | CH₂CN | O |
| D-9098 | H | CH₂CH₂ | Me | *c* -Pr(1-CN) | CH₂OC(=O)(*t*-Bu) | Cl | CH₂CN | O |
| D-9099 | Me | CH₂CH₂ | H | *c* -Pr(1-CN) | CH₂OC(=O)(*t*-Bu) | Cl | CH₂CN | O |
| D-9100 | H | single bond | Me | *c* -Pr(1-CN) | CH₂OC(=O)(*t*-Bu) | Me | CH₂CN | O |
| D-9101 | Me | single bond | H | *c* -Pr(1-CN) | CH₂OC(=O)(*t*-Bu) | Me | CH₂CN | O |
| D-9102 | Me(R) | single bond | H | *c* -Pr(1-CN) | CH₂OC(=O)(*t*-Bu) | Me | CH₂CN | O |
| D-9103 | Me(S) | single bond | H | *c* -Pr(1-CN) | CH₂OC(=O)(*t*-Bu) | Me | CH₂CN | O |
| D-9104 | H | CH₂ | Me | *c* -Pr(1-CN) | CH₂OC(=O)(*t*-Bu) | Me | CH₂CN | O |
| D-9105 | Me | CH₂ | H | *c* -Pr(1-CN) | CH₂OC(=O)(*t*-Bu) | Me | CH₂CN | O |
| D-9106 | H | CH(Me) | H | *c* -Pr(1-CN) | CH₂OC(=O)(*t*-Bu) | Me | CH₂CN | O |
| D-9107 | H | CH₂CH₂ | Me | *c* -Pr(1-CN) | CH₂OC(=O)(*t*-Bu) | Me | CH₂CN | O |
| D-9108 | Me | CH₂CH₂ | H | *c* -Pr(1-CN) | CH₂OC(=O)(*t*-Bu) | Me | CH₂CN | O |
| D-9109 | H | single bond | Me | *c* -Pr(1-CN) | CH₂OC(=O)OEt | Cl | CH₂CN | O |
| D-9110 | Me | single bond | H | *c* -Pr(1-CN) | CH₂OC(=O)OEt | Cl | CH₂CN | O |
| D-9111 | Me(R) | single bond | H | *c* -Pr(1-CN) | CH₂OC(=O)OEt | Cl | CH₂CN | O |
| D-9112 | Me(S) | single bond | H | *c* -Pr(1-CN) | CH₂OC(=O)OEt | Cl | CH₂CN | O |
| D-9113 | H | CH₂ | Me | *c* -Pr(1-CN) | CH₂OC(=O)OEt | Cl | CH₂CN | O |
| D-9114 | Me | CH₂ | H | *c* -Pr(1-CN) | CH₂OC(=O)OEt | Cl | CH₂CN | O |

**[Table 853]**

| Compound Number | Y | W | Z | R¹ | R² | R³ | R¹⁰ | A |
|---|---|---|---|---|---|---|---|---|
| D-9115 | H | CH(Me) | H | *c* -Pr(1-CN) | CH₂OC(=O)OEt | Cl | CH₂CN | O |
| D-9116 | H | CH₂CH₂ | Me | *c* -Pr(1-CN) | CH₂OC(=O)OEt | Cl | CH₂CN | O |
| D-9117 | Me | CH₂CH₂ | H | *c* -Pr(1-CN) | CH₂OC(=O)OEt | Cl | CH₂CN | O |
| D-9118 | H | single bond | Me | *c* -Pr(1-CN) | CH₂OC(=O)OEt | Me | CH₂CN | O |
| D-9119 | Me | single bond | H | *c* -Pr(1-CN) | CH₂OC(=O)OEt | Me | CH₂CN | O |
| D-9120 | Me(R) | single bond | H | *c*-Pr(1-CN) | CH₂OC(=O)OEt | Me | CH₂CN | O |
| D-9121 | Me(S) | single bond | H | *c* -Pr(1-CN) | CH₂OC(=O)OEt | Me | CH₂CN | O |
| D-9122 | H | CH₂ | Me | *c* -Pr(1-CN) | CH₂OC(=O)OEt | Me | CH₂CN | O |
| D-9123 | Me | CH₂ | H | *c* -Pr(1-CN) | CH₂OC(=O)OEt | Me | CH₂CN | O |
| D-9124 | H | CH(Me) | H | *c* -Pr(1-CN) | CH₂OC(=O)OEt | Me | CH₂CN | O |
| D-9125 | H | CH₂CH₂ | Me | *c* -Pr(1-CN) | CH₂OC(=O)OEt | Me | CH₂CN | O |
| D-9126 | Me | CH₂CH₂ | H | *c* -Pr(1-CN) | CH₂OC(=O)OEt | Me | CH₂CN | O |
| D-9127 | H | single bond | Me | *c* -Pr(1-CN) | CH₂OC(=O)O(*i*-Pr) | Cl | CH₂CN | O |
| D-9128 | Me | single bond | H | *c* -Pr(1-CN) | CH₂OC(=O)O(*i* -Pr) | Cl | CH₂CN | O |
| D-9129 | Me(R) | single bond | H | *c* -Pr(1-CN) | CH₂OC(=O)O(*i* -Pr) | Cl | CH₂CN | O |
| D-9130 | Me(S) | single bond | H | *c* -Pr(1-CN) | CH₂OC(=O)O(*i* -Pr) | Cl | CH₂CN | O |
| D-9131 | H | CH₂ | Me | *c* -Pr(1-CN) | CH₂OC(=O)O(*i* -Pr) | Cl | CH₂CN | O |
| D-9132 | Me | CH₂ | H | *c* -Pr(1-CN) | CH₂OC(=O)O(*i* -Pr) | Cl | CH₂CN | O |
| D-9133 | H | CH(Me) | H | *c* -Pr(1-CN) | CH₂OC(=O)O(*i* -Pr) | Cl | CH₂CN | O |
| D-9134 | H | CH₂CH₂ | Me | *c* -Pr(1-CN) | CH₂OC(=O)O(*i* -Pr) | Cl | CH₂CN | O |
| D-9135 | Me | CH₂CH₂ | H | *c* -Pr(1-CN) | CH₂OC(=O)O(*i* -Pr) | Cl | CH₂CN | O |
| D-9136 | H | single bond | Me | *c* -Pr(1-CN) | CH₂OC(=O)O(*i* -Pr) | Me | CH₂CN | O |
| D-9137 | Me | single bond | H | *c* -Pr(1-CN) | CH₂OC(=O)O(*i* -Pr) | Me | CH₂CN | O |
| D-9138 | Me(R) | single bond | H | *c* -Pr(1-CN) | CH₂OC(=O)O(*i* -Pr) | Me | CH₂CN | O |
| D-9139 | Me(S) | single bond | H | *c* -Pr(1-CN) | CH₂OC(=O)O(*i* -Pr) | Me | CH₂CN | O |
| D-9140 | H | CH₂ | Me | *c* -Pr(1-CN) | CH₂OC(=O)O(*i* -Pr) | Me | CH₂CN | O |
| D-9141 | Me | CH₂ | H | *c* -Pr(1-CN) | CH₂OC(=O)O(*i* -Pr) | Me | CH₂CN | O |
| D-9142 | H | CH(Me) | H | *c* -Pr(1-CN) | CH₂OC(=O)O(*i* -Pr) | Me | CH₂CN | O |
| D-9143 | H | CH₂CH₂ | Me | *c* -Pr(1-CN) | CH₂OC(=O)O(*i* -Pr) | Me | CH₂CN | O |
| D-9144 | Me | CH₂CH₂ | H | *c* -Pr(1-CN) | CH₂OC(=O)O(*i* -Pr) | Me | CH₂CN | O |
| D-9145 | H | single bond | Me | *c*-pr(1-CN) | CH₂N(Me)C(=O)OMe | Cl | CH₂CN | O |
| D-9146 | Me | single bond | H | *c* -Pr(1-CN) | CH₂N(Me)C(=O)OMe | Cl | CH₂CN | O |
| D-9147 | Me(R) | single bond | H | *c* -Pr(1-CN) | CH₂N(Me)C(=O)OMe | Cl | CH₂CN | O |
| D-9148 | Me(S) | single bond | H | *c* -Pr(1-CN) | CH₂N(Me)C(=O)OMe | Cl | CH₂CN | O |
| D-9149 | H | CH₂ | Me | *c* -Pr(1-CN) | CH₂N(Me)C(=O)OMe | Cl | CH₂CN | O |
| D-9150 | Me | CH₂ | H | *c* -Pr(1-CN) | CH₂N(Me)C(=O)OMe | Cl | CH₂CN | O |
| D-9151 | H | CH(Me) | H | *c* -Pr(1-CN) | CH₂N(Me)C(=O)OMe | Cl | CH₂CN | O |
| D-9152 | H | CH₂CH₂ | Me | *c* -Pr(1-CN) | CH₂N(Me)C(=O)OMe | Cl | CH₂CN | O |
| D-9153 | Me | CH₂CH₂ | H | *c* -Pr(1-CN) | CH₂N(Me)C(=O)OMe | Cl | CH₂CN | O |
| D-9154 | H | single bond | Me | *c*-Pr(1-CN) | CH₂N(Me)C(=O)OMe | Me | CH₂CN | O |
| D-9155 | Me | single bond | H | *c* -Pr(1-CN) | CH₂N(Me)C(=O)OMe | Me | CH₂CN | O |
| D-9156 | Me(R) | single bond | H | *c* -Pr(1-CN) | CH₂N(Me)C(=O)OMe | Me | CH₂CN | O |
| D-9157 | Me(S) | single bond | H | *c* -Pr(1-CN) | CH₂N(Me)C(=O)OMe | Me | CH₂CN | O |
| D-9158 | H | CH₂ | Me | *c* -Pr(1-CN) | CH₂N(Me)C(=O)OMe | Me | CH₂CN | O |

**[Table 854]**

| Compound Number | Y | W | Z | R¹ | R² | R³ | R¹⁰ | A |
|---|---|---|---|---|---|---|---|---|
| D-9159 | Me | CH₂ | H | *c* -Pr(1-CN) | CH₂N(Me)C(=O)OMe | Me | CH₂CN | O |
| D-9160 | H | CH(Me) | H | *c* -Pr(1-CN) | CH₂N(Me)C(=O)OMe | Me | CH₂CN | O |
| D-9161 | H | CH₂CH₂ | Me | *c* -Pr(1-CN) | CH₂N(Me)C(=O)OMe | Me | CH₂CN | O |
| D-9162 | Me | CH₂CH₂ | H | *c* -Pr(1-CN) | CH₂N(Me)C(=O)OMe | Me | CH₂CN | O |
| D-9163 | H | single bond | Me | *c* -Pr(1-CN) | C(=O)OCH₂CH=CH₂ | Cl | C(=O)Me | O |
| D-9164 | Me | single bond | H | *c* -Pr(1-CN) | C(=O)OCH₂CH=CH₂ | Cl | C(=O)Me | O |
| D-9165 | Me(R) | single bond | H | *c* -Pr(1-CN) | C(=O)OCH₂CH=CH₂ | Cl | C(=O)Me | O |
| D-9166 | Me(S) | single bond | H | *c* -Pr(1-CN) | C(=O)OCH₂CH=CH₂ | Cl | C(=O)Me | O |
| D-9167 | H | CH₂ | Me | *c* -Pr(1-CN) | C(=O)OCH₂CH=CH₂ | Cl | C(=O)Me | O |
| D-9168 | Me | CH₂ | H | *c* -Pr(1-CN) | C(=O)OCH₂CH=CH₂ | Cl | C(=O)Me | O |
| D-9169 | H | CH(Me) | H | *c* -Pr(1-CN) | C(=O)OCH₂CH=CH₂ | Cl | C(=O)Me | O |
| D-9170 | H | CH₂CH₂ | Me | *c* -Pr(1-CN) | C(=O)OCH₂CH=CH₂ | Cl | C(=O)Me | O |
| D-9171 | Me | CH₂CH₂ | H | *c*-Pr(1-CN) | C(=O)OCH₂CH=CH₂ | Cl | C(=O)Me | O |
| D-9172 | H | single bond | Me | *c* -Pr(1-CN) | C(=O)OCH₂CH=CH₂ | Me | C(=O)Me | O |
| D-9173 | Me | single bond | H | *c* -Pr(1-CN) | C(=O)OCH₂CH=CH₂ | Me | C(=O)Me | O |
| D-9174 | Me(R) | single bond | H | *c* -Pr(1-CN) | C(=O)OCH₂CH=CH₂ | Me | C(=O)Me | O |
| D-9175 | Me(S) | single bond | H | *c* -Pr(1-CN) | C(=O)OCH₂CH=CH₂ | Me | C(=O)Me | O |
| D-9176 | H | CH₂ | Me | *c* -Pr(1-CN) | C(=O)OCH₂CH=CH₂ | Me | C(=O)Me | O |
| D-9177 | Me | CH₂ | H | *c* -Pr(1-CN) | C(=O)OCH₂CH=CH₂ | Me | C(=O)Me | O |
| D-9178 | H | CH(Me) | H | *c* -Pr(1-CN) | C(=O)OCH₂CH=CH₂ | Me | C(=O)Me | O |
| D-9179 | H | CH₂CH₂ | Me | *c* -Pr(1-CN) | C(=O)OCH₂CH=CH₂ | Me | C(=O)Me | O |
| D-9180 | Me | CH₂CH₂ | H | *c* -Pr(1-CN) | C(=O)OCH₂CH=CH₂ | Me | C(=O)Me | O |
| D-9181 | H | single bond | Me | *c* -Pr(1-CN) | C(=O)OCH₂CCl₃ | Cl | C(=O)Me | O |
| D-9182 | Me | single bond | H | *c* -Pr(1-CN) | C(=O)OCH₂CCl₃ | Cl | C(=O)Me | O |
| D-9183 | Me(R) | single bond | H | *c* -Pr(1-CN) | C(=O)OCH₂CCl₃ | Cl | C(=O)Me | O |
| D-9184 | Me(S) | single bond | H | *c* -Pr(1-CN) | C(=O)OCH₂CCl₃ | Cl | C(=O)Me | O |
| D-9185 | H | CH₂ | Me | *c* -Pr(1-CN) | C(=O)OCH₂CCl₃ | Cl | C(=O)Me | O |
| D-9186 | Me | CH₂ | H | *c* -Pr(1-CN) | C(=O)OCH₂CCl₃ | Cl | C(=O)Me | O |
| D-9187 | H | CH(Me) | H | *c* -Pr(1-CN) | C(=O)OCH₂CCl₃ | Cl | C(=O)Me | O |
| D-9188 | H | CH₂CH₂ | Me | *c* -Pr(1-CN) | C(=O)OCH₂CCl₃ | Cl | C(=O)Me | O |
| D-9189 | Me | CH₂CH₂ | H | *c* -Pr(1-CN) | C(=O)OCH₂CCl₃ | Cl | C(=O)Me | O |
| D-9190 | H | single bond | Me | *c* -Pr(1-CN) | C(=O)OCH₂CCl₃ | Me | C(=O)Me | O |
| D-9191 | Me | single bond | H | *c* -Pr(1-CN) | C(=O)OCH₂CCl₃ | Me | C(=O)Me | O |
| D-9192 | Me(R) | single bond | H | *c* -Pr(1-CN) | C(=O)OCH₂CCl₃ | Me | C(=O)Me | O |
| D-9193 | Me(S) | single bond | H | *c* -Pr(1-CN) | C(=O)OCH₂CCl₃ | Me | C(=O)Me | O |
| D-9194 | H | CH₂ | Me | *c* -Pr(1-CN) | C(=O)OCH₂CCl₃ | Me | C(=O)Me | O |
| D-9195 | Me | CH₂ | H | *c* -Pr(1-CN) | C(=O)OCH₂CCl₃ | Me | C(=O)Me | O |
| D-9196 | H | CH(Me) | H | *c* -Pr(1-CN) | C(=O)OCH₂CCl₃ | Me | C(=O)Me | O |
| D-9197 | H | CH₂CH₂ | Me | *c* -Pr(1-CN) | C(=O)OCH₂CCl₃ | Me | C(=O)Me | O |
| D-9198 | Me | CH₂CH₂ | H | *c* -Pr(1-CN) | C(=O)OCH₂CCl₃ | Me | C(=O)Me | O |
| D-9199 | H | single bond | Me | *c* -Pr(1-CN) | C(=O)N(Me)₂ | Cl | C(=O)Me | O |
| D-9200 | Me | single bond | H | *c* -Pr(1-CN) | C(=O)N(Me)₂ | Cl | C(=O)Me | O |
| D-9201 | Me(R) | single bond | H | *c* -Pr(1-CN) | C(=O)N(Me)₂ | Cl | C(=O)Me | O |
| D-9202 | Me(S) | single bond | H | *c*-Pr(1-CN) | C(=O)N(Me)₂ | Cl | C(=O)Me | O |

**[Table 855]**

| Compound Number | Y | W | Z | R¹ | R² | R³ | R¹⁰ | A |
|---|---|---|---|---|---|---|---|---|
| D-9203 | H | CH₂ | Me | *c* -Pr(1-CN) | C(=O)N(Me)₂ | Cl | C(=O)Me | O |
| D-9204 | Me | CH₂ | H | *c* -Pr(1-CN) | C(=O)N(Me)₂ | Cl | C(=O)Me | O |
| D-9205 | H | CH(Me) | H | *c* -Pr(1-CN) | C(=O)N(Me)₂ | Cl | C(=O)Me | O |
| D-9206 | H | CH₂CH₂ | Me | *c* -Pr(1-CN) | C(=O)N(Me)₂ | Cl | C(=O)Me | O |
| D-9207 | Me | CH₂CH₂ | H | *c* -Pr(1-CN) | C(=O)N(Me)₂ | Cl | C(=O)Me | O |
| D-9208 | H | single bond | Me | *c* -Pr(1-CN) | C(=O)N(Me)₂ | Me | C(=O)Me | O |
| D-9209 | Me | single bond | H | *c* -Pr(1-CN) | C(=O)N(Me)₂ | Me | C(=O)Me | O |
| D-9210 | Me(R) | single bond | H | *c* -Pr(1-CN) | C(=O)N(Me)₂ | Me | C(=O)Me | O |
| D-9211 | Me(S) | single bond | H | *c* -Pr(1-CN) | C(=O)N(Me)₂ | Me | C(=O)Me | O |
| D-9212 | H | CH₂ | Me | *c* -Pr(1-CN) | C(=O)N(Me)₂ | Me | C(=O)Me | O |
| D-9213 | Me | CH₂ | H | *c* -Pr(1-CN) | C(=O)N(Me)₂ | Me | C(=O)Me | O |
| D-9214 | H | CH(Me) | H | *c* -Pr(1-CN) | C(=O)N(Me)₂ | Me | C(=O)Me | O |
| D-9215 | H | CH₂CH₂ | Me | *c* -Pr(1-CN) | C(=O)N(Me)₂ | Me | C(=O)Me | O |
| D-9216 | Me | CH₂CH₂ | H | *c* -Pr(1-CN) | C(=O)N(Me)₂ | Me | C(=O)Me | O |
| D-9217 | H | single bond | Me | *c* -Pr(1-CN) | CH₂OC(=O)Me | Cl | C(=O)Me | O |
| D-9218 | Me | single bond | H | *c* -Pr(1-CN) | CH₂OC(=O)Me | Cl | C(=O)Me | O |
| D-9219 | Me(R) | single bond | H | *c* -Pr(1-CN) | CH₂OC(=O)Me | Cl | C(=O)Me | O |
| D-9220 | Me(S) | single bond | H | *c* -Pr(1-CN) | CH₂OC(=O)Me | Cl | C(=O)Me | O |
| D-9221 | H | CH₂ | Me | *c* -Pr(1-CN) | CH₂OC(=O)Me | Cl | C(=O)Me | O |
| D-9222 | Me | CH₂ | H | *c* -Pr(1-CN) | CH₂OC(=O)Me | Cl | C(=O)Me | O |
| D-9223 | H | CH(Me) | H | *c* -Pr(1-CN) | CH₂OC(=O)Me | Cl | C(=O)Me | O |
| D-9224 | H | CH₂CH₂ | Me | *c* -Pr(1-CN) | CH₂OC(=O)Me | Cl | C(=O)Me | O |
| D-9225 | Me | CH₂CH₂ | H | *c* -Pr(1-CN) | CH₂OC(=O)Me | Cl | C(=O)Me | O |
| D-9226 | H | single bond | Me | *c*-Pr(1-CN) | CH₂OC(=O)Me | Me | C(=O)Me | O |
| D-9227 | Me | single bond | H | *c* -Pr(1-CN) | CH₂OC(=O)Me | Me | C(=O)Me | O |
| D-9228 | Me(R) | single bond | H | *c* -Pr(1-CN) | CH₂OC(=O)Me | Me | C(=O)Me | O |
| D-9229 | Me(S) | single bond | H | *c* -Pr(1-CN) | CH₂OC(=O)Me | Me | C(=O)Me | O |
| D-9230 | H | CH₂ | Me | *c* -Pr(1-CN) | CH₂OC(=O)Me | Me | C(=O)Me | O |
| D-9231 | Me | CH₂ | H | *c* -Pr(1-CN) | CH₂OC(=O)Me | Me | C(=O)Me | O |
| D-9232 | H | CH(Me) | H | *c* -Pr(1-CN) | CH₂OC(=O)Me | Me | C(=O)Me | O |
| D-9233 | H | CH₂CH₂ | Me | *c* -Pr(1-CN) | CH₂OC(=O)Me | Me | C(=O)Me | O |
| D-9234 | Me | CH₂CH₂ | H | *c* -Pr(1-CN) | CH₂OC(=O)Me | Me | C(=O)Me | O |
| D-9235 | H | single bond | Me | *c* -Pr(1-CN) | CH₂OC(=O)(*i* -Pr) | Cl | C(=O)Me | O |
| D-9236 | Me | single bond | H | *c* -Pr(1-CN) | CH₂OC(=O)(*i* -Pr) | Cl | C(=O)Me | O |
| D-9237 | Me(R) | single bond | H | *c* -Pr(1-CN) | CH₂OC(=O)(*i* -Pr) | Cl | C(=O)Me | O |
| D-9238 | Me(S) | single bond | H | *c* -Pr(1-CN) | CH₂OC(=O)(*i* -Pr) | Cl | C(=O)Me | O |
| D-9239 | H | CH₂ | Me | *c* -Pr(1-CN) | CH₂OC(=O)(*i* -Pr) | Cl | C(=O)Me | O |
| D-9240 | Me | CH₂ | H | *c* -Pr(1-CN) | CH₂OC(=O)(*i*-Pr) | Cl | C(=O)Me | O |
| D-9241 | H | CH(Me) | H | *c* -Pr(1-CN) | CH₂OC(=O)(*i* -Pr) | Cl | C(=O)Me | O |
| D-9242 | H | CH₂CH₂ | Me | *c* -Pr(1-CN) | CH₂OC(=O)(*i* -Pr) | Cl | C(=O)Me | O |
| D-9243 | Me | CH₂CH₂ | H | *c* -Pr(1-CN) | CH₂OC(=O)(*i* -Pr) | Cl | C(=O)Me | O |
| D-9244 | H | single bond | Me | *c* -Pr(1-CN) | CH₂OC(=O)(*i* -Pr) | Me | C(=O)Me | O |
| D-9245 | Me | single bond | H | *c* -Pr(1-CN) | CH₂OC(=O)(*i* -Pr) | Me | C(=O)Me | O |
| D-9246 | Me(R) | single bond | H | *c*-Pr(1-CN) | CH₂OC(=O)(*i* -Pr) | Me | C(=O)Me | O |

**[Table 856]**

| Compound Number | Y | W | Z | R¹ | R² | R³ | R¹⁰ | A |
|---|---|---|---|---|---|---|---|---|
| D-9247 | Me(S) | single bond | H | *c* -Pr(1-CN) | CH₂OC(=O)(*i* -Pr) | Me | C(=O)Me | O |
| D-9248 | H | CH₂ | Me | *c* -Pr(1-CN) | CH₂OC(=O)(*i* -Pr) | Me | C(=O)Me | O |
| D-9249 | Me | CH₂ | H | *c* -Pr(1-CN) | CH₂OC(=O)(*i*-Pr) | Me | C(=O)Me | O |
| D-9250 | H | CH(Me) | H | *c* -Pr(1-CN) | CH₂OC(=O)(*i*-Pr) | Me | C(=O)Me | O |
| D-9251 | H | CH₂CH₂ | Me | *c* -Pr(1-CN) | CH₂OC (=O)(i -Pr) | Me | C(=O)Me | O |
| D-9252 | Me | CH₂CH₂ | H | *c*-Pr(1-CN) | CH₂OC(=O)(*i*-Pr) | Me | C(=O)Me | O |
| D-9253 | H | single bond | Me | *c* -Pr(1-CN) | CH₂OC(=O)(*t*-Bu) | Cl | C(=O)Me | O |
| D-9254 | Me | single bond | H | *c* -Pr(1-CN) | CH₂OC(=O)(*t*-Bu) | Cl | C(=O)Me | O |
| D-9255 | Me(R) | single bond | H | *c* -Pr(1-CN) | CH₂OC(=O)(*t*-Bu) | Cl | C(=O)Me | O |
| D-9256 | Me(S) | single bond | H | *c* -Pr(1-CN) | CH₂OC(=O)(*t*-Bu) | Cl | C(=O)Me | O |
| D-9257 | H | CH₂ | Me | *c* -Pr(1-CN) | CH₂OC(=O)(*t*-Bu) | Cl | C(=O)Me | O |
| D-9258 | Me | CH₂ | H | *c* -Pr(1-CN) | CH₂OC(=O)(*t*-Bu) | Cl | C(=O)Me | O |
| D-9259 | H | CH(Me) | H | *c*-Pr(1-CN) | CH₂OC(=O)(*t*-Bu) | Cl | C(=O)Me | O |
| D-9260 | H | CH₂CH₂ | Me | *c* -Pr(1-CN) | CH₂OC(=O)(*t*-Bu) | Cl | C(=O)Me | O |
| D-9261 | Me | CH₂CH₂ | H | *c* -Pr(1-CN) | CH₂OC(=O)(*t*-Bu) | Cl | C(=O)Me | O |
| D-9262 | H | single bond | Me | *c* -Pr(1-CN) | CH₂OC(=O)(*t*-Bu) | Me | C(=O)Me | O |
| D-9263 | Me | single bond | H | *c* -Pr(1-CN) | CH₂OC(=O)(*t*-Bu) | Me | C(=O)Me | O |
| D-9264 | Me(R) | single bond | H | *c* -Pr(1-CN) | CH₂OC(=O)(*t*-Bu) | Me | C(=O)Me | O |
| D-9265 | Me(S) | single bond | H | *c* -Pr(1-CN) | CH₂OC(=O)(*t*-Bu) | Me | C(=O)Me | O |
| D-9266 | H | CH₂ | Me | *c* -Pr(1-CN) | CH₂OC(=O)(*t*-Bu) | Me | C(=O)Me | O |
| D-9267 | Me | CH₂ | H | *c* -Pr(1-CN) | CH₂OC(=O)(*t*-Bu) | Me | C(=O)Me | O |
| D-9268 | H | CH(Me) | H | *c* -Pr(1-CN) | CH₂OC(=O)(*t*-Bu) | Me | C(=O)Me | O |
| D-9269 | H | CH₂CH₂ | Me | *c* -Pr(1-CN) | CH₂OC(=O)(*t*-Bu) | Me | C(=O)Me | O |
| D-9270 | Me | CH₂CH₂ | H | *c* -Pr(1-CN) | CH₂OC(=O)(*t*-Bu) | Me | C(=O)Me | O |
| D-9271 | H | single bond | Me | *c* -Pr(1-CN) | CH₂OC(=O)OEt | Cl | C(=O)Me | O |
| D-9272 | Me | single bond | H | *c* -Pr(1-CN) | CH₂OC(=O)OEt | Cl | C(=O)Me | O |
| D-9273 | Me(R) | single bond | H | *c* -Pr(1-CN) | CH₂OC(=O)OEt | Cl | C(=O)Me | O |
| D-9274 | Me(S) | single bond | H | *c* -Pr(1-CN) | CH₂OC(=O)OEt | Cl | C(=O)Me | O |
| D-9275 | H | CH₂ | Me | *c* -Pr(1-CN) | CH₂OC(=O)OEt | Cl | C(=O)Me | O |
| D-9276 | Me | CH₂ | H | *c* -Pr(1-CN) | CH₂OC(=O)OEt | Cl | C(=O)Me | O |
| D-9277 | H | CH(Me) | H | *c* -Pr(1-CN) | CH₂OC(=O)OEt | Cl | C(=O)Me | O |
| D-9278 | H | CH₂CH₂ | Me | *c* -Pr(1-CN) | CH₂OC(=O)OEt | Cl | C(=O)Me | O |
| D-9279 | Me | CH₂CH₂ | H | *c* -Pr(1-CN) | CH₂OC(=O)OEt | Cl | C(=O)Me | O |
| D-9280 | H | single bond | Me | *c* -Pr(1-CN) | CH₂OC(=O)OEt | Me | C(=O)Me | O |
| D-9281 | Me | single bond | H | *c* -Pr(1-CN) | CH₂OC(=O)OEt | Me | C(=O)Me | O |
| D-9282 | Me(R) | single bond | H | *c* -Pr(1-CN) | CH₂OC(=O)OEt | Me | C(=O)Me | O |
| D-9283 | Me(S) | single bond | H | *c* -Pr(1-CN) | CH₂OC(=O)OEt | Me | C(=O)Me | O |
| D-9284 | H | CH₂ | Me | *c* -Pr(1-CN) | CH₂OC(=O)OEt | Me | C(=O)Me | O |
| D-9285 | Me | CH₂ | H | *c* -Pr(1-CN) | CH₂OC(=O)OEt | Me | C(=O)Me | O |
| D-9286 | H | CH(Me) | H | *c* -Pr(1-CN) | CH₂OC(=O)OEt | Me | C(=O)Me | O |
| D-9287 | H | CH₂CH₂ | Me | *c* -Pr(1-CN) | CH₂OC(=O)OEt | Me | C(=O)Me | O |
| D-9288 | Me | CH₂CH₂ | H | *c* -Pr(1-CN) | CH₂OC(=O)OEt | Me | C(=O)Me | O |
| D-9289 | H | single bond | Me | *c* -Pr(1-CN) | CH₂OC(=O)O(*i* -Pr) | Cl | C(=O)Me | O |
| D-9290 | Me | single bond | H | *c* -Pr(1-CN) | CH₂OC(=O)O(*i*-Pr) | Cl | C(=O)Me | O |

**[Table 857]**

| Compound Number | Y | W | Z | R¹ | R² | R³ | R¹⁰ | A |
|---|---|---|---|---|---|---|---|---|
| D-9291 | Me(R) | single bond | H | *c*-Pr(1-CN) | CH₂OC(=O)O(*i* -Pr) | Cl | C(=O)Me | O |
| D-9292 | Me(S) | single bond | H | *c*-Pr(1-CN) | CH₂OC(=O)O(*i* -Pr) | Cl | C(=O)Me | O |
| D-9293 | H | CH₂ | Me | *c*-Pr(1-CN) | CH₂OC(=O)O(*i* -Pr) | Cl | C(=O)Me | O |
| D-9294 | Me | CH₂ | H | *c*-Pr(1-CN) | CH₂OC(=O)O(*i* -Pr) | Cl | C(=O)Me | O |
| D-9295 | H | CH(Me) | H | *c*-Pr(1-CN) | CH₂OC(=O)O(*i* -Pr) | Cl | C(=O)Me | O |
| D-9296 | H | CH₂CH₂ | Me | *c*-Pr(1-CN) | CH₂OC(=O)O(*i*-Pr) | Cl | C(=O)Me | O |
| D-9297 | Me | CH₂CH₂ | H | *c*-Pr(1-CN) | CH₂OC(=O)O(*i*-Pr) | Cl | C(=O)Me | O |
| D-9298 | H | single bond | Me | *c*-Pr(1-CN) | CH₂OC(=O)O(*i* -Pr) | Me | C(=O)Me | O |
| D-9299 | Me | single bond | H | *c*-Pr(1-CN) | CH₂OC(=O)O(*i*-Pr) | Me | C(=O)Me | O |
| D-9300 | Me(R) | single bond | H | *c* -Pr(1-CN) | CH₂OC(=O)O(*i*-Pr) | Me | C(=O)Me | O |
| D-9301 | Me(S) | single bond | H | *c* -Pr(1-CN) | CH₂OC(=O)O(*i* -Pr) | Me | C(=O)Me | O |
| D-9302 | H | CH₂ | Me | *c* -Pr(1-CN) | CH₂OC(=O)O(*i*-Pr) | Me | C(=O)Me | O |
| D-9303 | Me | CH₂ | H | *c* -Pr(1-CN) | CH₂OC(=O)O(*i*-Pr) | Me | C(=O)Me | O |
| D-9304 | H | CH(Me) | H | *c* -Pr(1-CN) | CH₂OC(=O)O(*i* -Pr) | Me | C(=O)Me | O |
| D-9305 | H | CH₂CH₂ | Me | *c*-Pr(1-CN) | CH₂OC(=O)O(*i*-Pr) | Me | C(=O)Me | O |
| D-9306 | Me | CH₂CH₂ | H | *c*-Pr(1-CN) | CH₂OC(=O)O(*i* -Pr) | Me | C(=O)Me | O |
| D-9307 | H | single bond | Me | *c*-Pr(1-CN) | CH₂N(Me)C(=O)OMe | Cl | C(=O)Me | O |
| D-9308 | Me | single bond | H | *c*-Pr(1-CN) | CH₂N(Me)C(=O)OMe | Cl | C(=O)Me | O |
| D-9309 | Me(R) | single bond | H | *c*-Pr(1-CN) | CH₂N(Me)C(=O)OMe | Cl | C(=O)Me | O |
| D-9310 | Me(S) | single bond | H | *c* -Pr(1-CN) | CH₂N(Me)C(=O)OMe | Cl | C(=O)Me | O |
| D-9311 | H | CH₂ | Me | *c* -Pr(1-CN) | CH₂N(Me)C(=O)OMe | Cl | C(=O)Me | O |
| D-9312 | Me | CH₂ | H | *c* -Pr(1-CN) | CH₂N(Me)C(=O)OMe | Cl | C(=O)Me | O |
| D-9313 | H | CH(Me) | H | *c* -Pr(1-CN) | CH₂N(Me)C(=O)OMe | Cl | C(=O)Me | O |
| D-9314 | H | CH₂CH₂ | Me | *c* -Pr(1-CN) | CH₂N(Me)C(=O)OMe | Cl | C(=O)Me | O |
| D-9315 | Me | CH₂CH₂ | H | *c* -Pr(1-CN) | CH₂N(Me)C(=O)OMe | Cl | C(=O)Me | O |
| D-9316 | H | single bond | Me | *c* -Pr(1-CN) | CH₂N(Me)C(=O)OMe | Me | C(=O)Me | O |
| D-9317 | Me | single bond | H | *c* -Pr(1-CN) | CH₂N(Me)C(=O)OMe | Me | C(=O)Me | O |
| D-9318 | Me(R) | single bond | H | *c* -Pr(1-CN) | CH₂N(Me)C(=O)OMe | Me | C(=O)Me | O |
| D-9319 | Me(S) | single bond | H | *c* -Pr(1-CN) | CH₂N(Me)C(=O)OMe | Me | C(=O)Me | O |
| D-9320 | H | CH₂ | Me | *c* -Pr(1-CN) | CH₂N(Me)C(=O)OMe | Me | C(=O)Me | O |
| D-9321 | Me | CH₂ | H | *c* -Pr(1-CN) | CH₂N(Me)C(=O)OMe | Me | C(=O)Me | O |
| D-9322 | H | CH(Me) | H | *c* -Pr(1-CN) | CH₂N(Me)C(=O)OMe | Me | C(=O)Me | O |
| D-9323 | H | CH₂CH₂ | Me | *c* -Pr(1-CN) | CH₂N(Me)C(=O)OMe | Me | C(=O)Me | O |
| D-9324 | Me | CH₂CH₂ | H | *c* -Pr(1-CN) | CH₂N(Me)C(=O)OMe | Me | C(=O)Me | O |

**[Table 858]**

| 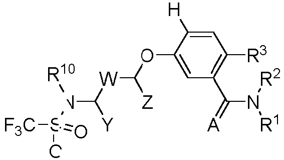 | | | | | | | | |
|---|---|---|---|---|---|---|---|---|
| Compound Number | Y | W | Z | R¹ | R² | R³ | R¹⁰ | A |
| D-9325 | H | single bond | Me | CH₂CF₃ | H | Cl | *c*-Pr | O |
| D-9326 | Me | single bond | H | CH₂CF₃ | H | Cl | *c*-Pr | O |
| D-9327 | Me(R) | single bond | H | CH₂CF₃ | H | Cl | *c*-Pr | O |
| D-9328 | Me(S) | single bond | H | CH₂CF₃ | H | Cl | *c*-Pr | O |
| D-9329 | H | single bond | Me | Et | H | Cl | *c*-Pr | O |
| D-9330 | Me | single bond | H | Et | H | Cl | *c*-Pr | O |
| D-9331 | Me(R) | single bond | H | Et | H | Cl | *c*-Pr | O |
| D-9332 | Me(S) | single bond | H | Et | H | Cl | *c*-Pr | O |
| D-9333 | H | single bond | Me | c-Pr | H | Cl | *c*-Pr | O |
| D-9334 | Me | single bond | H | c-Pr | H | Cl | *c*-Pr | O |
| D-9335 | Me(R) | single bond | H | c-Pr | H | Cl | *c*-Pr | O |
| D-9336 | Me(S) | single bond | H | c-Pr | H | Cl | *c*-Pr | O |
| D-9337 | H | single bond | Me | *c*-Pr(1-CN) | H | Cl | *c*-Pr | O |
| D-9338 | Me | single bond | H | *c*-Pr(1-CN) | H | Cl | *c*-Pr | O |
| D-9339 | Me(R) | single bond | H | *c*-Pr(1-CN) | H | Cl | *c*-Pr | O |
| D-9340 | Me(S) | single bond | H | *c*-Pr(1-CN) | H | Cl | *c*-Pr | O |
| D-9341 | H | single bond | Me | CH₂CF₃ | H | Me | *c*-Pr | O |
| D-9342 | Me | single bond | H | CH₂CF₃ | H | Me | *c*-Pr | O |
| D-9343 | Me(R) | single bond | H | CH₂CF₃ | H | Me | *c*-Pr | O |
| D-9344 | Me(S) | single bond | H | CH₂CF₃ | H | Me | *c*-Pr | O |
| D-9345 | H | single bond | Me | Et | H | Me | *c*-Pr | O |
| D-9346 | Me | single bond | H | Et | H | Me | *c*-Pr | O |
| D-9347 | Me(R) | single bond | H | Et | H | Me | *c*-Pr | O |
| D-9348 | Me(S) | single bond | H | Et | H | Me | *c*-Pr | O |
| D-9349 | H | single bond | Me | *c*-Pr | H | Me | *c*-Pr | O |
| D-9350 | Me | single bond | H | *c*-Pr | H | Me | *c*-Pr | O |
| D-9351 | Me(R) | single bond | H | *c*-Pr | H | Me | *c*-Pr | O |
| D-9352 | Me(S) | single bond | H | *c*-Pr | H | Me | *c*-Pr | O |
| D-9353 | H | single bond | Me | *c-*Pr(1-CN) | H | Me | *c*-Pr | O |
| D-9354 | Me | single bond | H | *c-*Pr(1-CN) | H | Me | c-Pr | O |
| D-9355 | Me(R) | single bond | H | *c-*Pr(1-CN) | H | Me | *c*-Pr | O |
| D-9356 | Me(S) | single bond | H | *c*-Pr(1-CN) | H | Me | *c*-Pr | O |
| D-9357 | H | single bond | Me | CH₂CF₃ | H | Cl | OMe | O |
| D-9358 | Me | single bond | H | CH₂CF₃ | H | Cl | OMe | O |
| D-9359 | Me(R) | single bond | H | CH₂CF₃ | H | Cl | OMe | O |
| D-9360 | Me(S) | single bond | H | CH₂CF₃ | H | Cl | OMe | O |
| D-9361 | H | single bond | Me | Et | H | Cl | OMe | O |
| D-9362 | Me | single bond | H | Et | H | Cl | OMe | O |

**[Table 859]**

| Compound Number | Y | W | Z | R¹ | R² | R³ | R¹⁰ | A |
|---|---|---|---|---|---|---|---|---|
| D-9363 | Me(R) | single bond | H | Et | H | Cl | OMe | O |
| D-9364 | Me(S) | single bond | H | Et | H | Cl | OMe | O |
| D-9365 | H | single bond | Me | *c*-Pr | H | Cl | OMe | O |
| D-9366 | Me | single bond | H | *c*-Pr | H | Cl | OMe | O |
| D-9367 | Me(R) | single bond | H | *c*-Pr | H | Cl | OMe | O |
| D-9368 | Me(S) | single bond | H | *c*-Pr | H | Cl | OMe | O |
| D-9369 | H | single bond | Me | *c*-Pr(1-CN) | H | Cl | OMe | O |
| D-9370 | Me | single bond | H | *c*-Pr(1-CN) | H | Cl | OMe | O |
| D-9371 | Me(R) | single bond | H | *c*-Pr(1-CN) | H | Cl | OMe | O |
| D-9372 | Me(S) | single bond | H | *c*-Pr(1-CN) | H | Cl | OMe | O |
| D-9373 | H | single bond | Me | CH₂CF₃ | H | Me | OMe | O |
| D-9374 | Me | single bond | H | CH₂CF₃ | H | Me | OMe | O |
| D-9375 | Me(R) | single bond | H | CH₂CF₃ | H | Me | OMe | O |
| D-9376 | Me(S) | single bond | H | CH₂CF₃ | H | Me | OMe | O |
| D-9377 | H | single bond | Me | Et | H | Me | OMe | O |
| D-9378 | Me | single bond | H | Et | H | Me | OMe | O |
| D-9379 | Me(R) | single bond | H | Et | H | Me | OMe | O |
| D-9380 | Me(S) | single bond | H | Et | H | Me | OMe | O |
| D-9381 | H | single bond | Me | *c*-Pr | H | Me | OMe | O |
| D-9382 | Me | single bond | H | *c*-Pr | H | Me | OMe | O |
| D-9383 | Me(R) | single bond | H | *c*-Pr | H | Me | OMe | O |
| D-9384 | Me(S) | single bond | H | *c*-Pr | H | Me | OMe | O |
| D-9385 | H | single bond | Me | *c*-Pr(1-CN) | H | Me | OMe | O |
| D-9386 | Me | single bond | H | *c*-Pr(1-CN) | H | Me | OMe | O |
| D-9387 | Me(R) | single bond | H | *c*-Pr(1-CN) | H | Me | OMe | O |
| D-9388 | Me(S) | single bond | H | *c*-Pr(1-CN) | H | Me | OMe | O |
| D-9389 | H | single bond | Me | CH₂CF₃ | H | Cl | C(=O)(*i* -Pr) | O |
| D-9390 | Me | single bond | H | CH₂CF₃ | H | Cl | C(=O)(*i*-Pr) | O |
| D-9391 | Me(R) | single bond | H | CH₂CF₃ | H | Cl | C(=O)(*i*-Pr) | O |
| D-9392 | Me(S) | single bond | H | CH₂CF₃ | H | Cl | C(=O)(*i*-Pr) | O |
| D-9393 | H | single bond | Me | Et | H | Cl | C(=O)(*i*-Pr) | O |
| D-9394 | Me | single bond | H | Et | H | Cl | C(=O)(*i*-Pr) | O |
| D-9395 | Me(R) | single bond | H | Et | H | Cl | C(=O)(*i*-Pr) | O |
| D-9396 | Me(S) | single bond | H | Et | H | Cl | C(=O)(*i*-Pr) | O |
| D-9397 | H | single bond | Me | *c*-Pr | H | Cl | C(=O)(*i*-Pr) | O |
| D-9398 | Me | single bond | H | *c*-Pr | H | Cl | C(=O)(*i*-Pr) | O |
| D-9399 | Me(R) | single bond | H | *c*-Pr | H | Cl | C(=O)(*i*-Pr) | O |
| D-9400 | Me(S) | single bond | H | *c*-Pr | H | Cl | C(=O)(*i*-Pr) | O |
| D-9401 | H | single bond | Me | *c*-Pr(1-CN) | H | Cl | C(=O)(*i*-Pr) | O |
| D-9402 | Me | single bond | H | *c*-Pr(1-CN) | H | Cl | C(=O)(*i*-Pr) | O |
| D-9403 | Me(R) | single bond | H | *c*-Pr(1-CN) | H | Cl | C(=O)(*i*-Pr) | O |
| D-9404 | Me(S) | single bond | H | *c*-Pr(1-CN) | H | Cl | C(=O)(*i*-Pr) | O |
| D-9405 | H | single bond | Me | CH₂CF₃ | H | Me | C(=O)(*i*-Pr) | O |
| D-9406 | Me | single bond | H | CH₂CF₃ | H | Me | C(=O)(*i*-Pr) | O |

**[Table 860]**

| Compound Number | Y | W | Z | R¹ | R² | R³ | R¹⁰ | A |
|---|---|---|---|---|---|---|---|---|
| D-9407 | Me(R) | single bond | H | CH₂CF₃ | H | Me | C(=O)(*i*-Pr) | O |
| D-9408 | Me(S) | single bond | H | CH₂CF₃ | H | Me | C(=O)(*i*-Pr) | O |
| D-9409 | H | single bond | Me | Et | H | Me | C(=O)(*i*-Pr) | O |
| D-9410 | Me | single bond | H | Et | H | Me | C(=O)(*i*-Pr) | O |
| D-9411 | Me(R) | single bond | H | Et | H | Me | C(=O)(*i*-Pr) | O |
| D-9412 | Me(S) | single bond | H | Et | H | Me | C(=O)(*i*-Pr) | O |
| D-9413 | H | single bond | Me | *c*-Pr | H | Me | C(=O)(*i*-Pr) | O |
| D-9414 | Me | single bond | H | *c*-Pr | H | Me | C(=O)(*i*-Pr) | O |
| D-9415 | Me(R) | single bond | H | *c*-Pr | H | Me | C(=O)(*i*-Pr) | O |
| D-9416 | Me(S) | single bond | H | *c*-Pr | H | Me | C(=O)(*i*-Pr) | O |
| D-9417 | H | single bond | Me | *c*-Pr(1-CN) | H | Me | C(=O)(*i*-Pr) | O |
| D-9418 | Me | single bond | H | *c*-Pr(1-CN) | H | Me | C(=O)(*i*-Pr) | O |
| D-9419 | Me(R) | single bond | H | *c-*Pr(1-CN) | H | Me | C(=O)(*i*-Pr) | O |
| D-9420 | Me(S) | single bond | H | *c*-Pr(1-CN) | H | Me | C(=O)(*i*-Pr) | O |
| D-9421 | H | single bond | Me | CH₂CF₃ | H | Cl | C(=O)O(*t*-Bu) | O |
| D-9422 | Me | single bond | H | CH₂CF₃ | H | Cl | C(=O)O(*t*-Bu) | O |
| D-9423 | Me(R) | single bond | H | CH₂CF₃ | H | Cl | C(=O)O(*t*-Bu) | O |
| D-9424 | Me(S) | single bond | H | CH₂CF₃ | H | Cl | C(=O)O(*t*-Bu) | O |
| D-9425 | H | single bond | Me | Et | H | Cl | C(=O)O(*t*-Bu) | O |
| D-9426 | Me | single bond | H | Et | H | Cl | C(=O)O(*t*-Bu) | O |
| D-9427 | Me(R) | single bond | H | Et | H | Cl | C(=O)O(*t*-Bu) | O |
| D-9428 | Me(S) | single bond | H | Et | H | Cl | C(=O)O(*t*-Bu) | O |
| D-9429 | H | single bond | Me | *c*-Pr | H | Cl | C(=O)O(*t*-Bu) | O |
| D-9430 | Me | single bond | H | *c*-Pr | H | Cl | C(=O)O(*t*-Bu) | O |
| D-9431 | Me(R) | single bond | H | *c*-Pr | H | Cl | C(=O)O(*t*-Bu) | O |
| D-9432 | Me(S) | single bond | H | *c*-Pr | H | Cl | C(=O)O(*t*-Bu) | O |
| D-9433 | H | single bond | Me | *c*-Pr(1-CN) | H | Cl | C(=O)O(*t*-Bu) | O |
| D-9434 | Me | single bond | H | *c*-Pr(1-CN) | H | Cl | C(=O)O(*t*-Bu) | O |
| D-9435 | Me(R) | single bond | H | *c*-Pr(1-CN) | H | Cl | C(=O)O(*t*-Bu) | O |
| D-9436 | Me(S) | single bond | H | *c*-Pr(1-CN) | H | Cl | C(=O)O(*t*-Bu) | O |
| D-9437 | H | single bond | Me | CH₂CF₃ | H | Me | C(=O)O(*t*-Bu) | O |
| D-9438 | Me | single bond | H | CH₂CF₃ | H | Me | C(=O)O(*t*-Bu) | O |
| D-9439 | Me(R) | single bond | H | CH₂CF₃ | H | Me | C(=O)O(*t*-Bu) | O |
| D-9440 | Me(S) | single bond | H | CH₂CF₃ | H | Me | C(=O)O(*t*-Bu) | O |
| D-9441 | H | single bond | Me | Et | H | Me | C(=O)O(*t*-Bu) | O |
| D-9442 | Me | single bond | H | Et | H | Me | C(=O)O(*t*-Bu) | O |
| D-9443 | Me(R) | single bond | H | Et | H | Me | C(=O)O(*t*-Bu) | O |
| D-9444 | Me(S) | single bond | H | Et | H | Me | C(=O)O(*t*-Bu) | O |
| D-9445 | H | single bond | Me | *c*-Pr | H | Me | C(=O)O(*t*-Bu) | O |
| D-9446 | Me | single bond | H | *c*-Pr | H | Me | C(=O)O(*t*-Bu) | O |
| D-9447 | Me(R) | single bond | H | *c*-Pr | H | Me | C(=O)O(*t*-Bu) | O |
| D-9448 | Me(S) | single bond | H | *c*-Pr | H | Me | C(=O)O(*t*-Bu) | O |
| D-9449 | H | single bond | Me | *c*-Pr(1-CN) | H | Me | C(=O)O(*t*-Bu) | O |
| D-9450 | Me | single bond | H | *c-*Pr(1-CN) | H | Me | C(=O)O(*t*-Bu) | O |

**[Table 861]**

| Compound Number | Y | W | Z | R¹ | R² | R³ | R¹⁰ | A |
|---|---|---|---|---|---|---|---|---|
| D-9451 | Me(R) | single bond | H | *c*-Pr(1-CN) | H | Me | C(=O)O(*t*-Bu) | O |
| D-9452 | Me(S) | single bond | H | *c*-Pr(1-CN) | H | Me | C(=O)O(*t*-Bu) | O |
| D-9453 | H | single bond | Me | CH₂CF₃ | H | Cl | CH₂OEt | O |
| D-9454 | Me | single bond | H | CH₂CF₃ | H | Cl | CH₂OEt | O |
| D-9455 | Me(R) | single bond | H | CH₂CF₃ | H | Cl | CH₂OEt | O |
| D-9456 | Me(S) | single bond | H | CH₂CF₃ | H | Cl | CH₂OEt | O |
| D-9457 | H | single bond | Me | Et | H | Cl | CH₂OEt | O |
| D-9458 | Me | single bond | H | Et | H | Cl | CH₂OEt | O |
| D-9459 | Me(R) | single bond | H | Et | H | Cl | CH₂OEt | O |
| D-9460 | Me(S) | single bond | H | Et | H | Cl | CH₂OEt | O |
| D-9461 | H | single bond | Me | c-Pr | H | Cl | CH₂OEt | O |
| D-9462 | Me | single bond | H | c-Pr | H | Cl | CH₂OEt | O |
| D-9463 | Me(R) | single bond | H | c-Pr | H | Cl | CH₂OEt | O |
| D-9464 | Me(S) | single bond | H | c-Pr | H | Cl | CH₂OEt | O |
| D-9465 | H | single bond | Me | *c*-Pr(1-CN) | H | Cl | CH₂OEt | O |
| D-9466 | Me | single bond | H | *c*-Pr(1-CN) | H | Cl | CH₂OEt | O |
| D-9467 | Me(R) | single bond | H | *c*-Pr(1-CN) | H | Cl | CH₂OEt | O |
| D-9468 | Me(S) | single bond | H | *c*-Pr(1-CN) | H | Cl | CH₂OEt | O |
| D-9469 | H | single bond | Me | CH₂CF₃ | H | Me | CH₂OEt | O |
| D-9470 | Me | single bond | H | CH₂CF₃ | H | Me | CH₂OEt | O |
| D-9471 | Me(R) | single bond | H | CH₂CF₃ | H | Me | CH₂OEt | O |
| D-9472 | Me(S) | single bond | H | CH₂CF₃ | H | Me | CH₂OEt | O |
| D-9473 | H | single bond | Me | Et | H | Me | CH₂OEt | O |
| D-9474 | Me | single bond | H | Et | H | Me | CH₂OEt | O |
| D-9475 | Me(R) | single bond | H | Et | H | Me | CH₂OEt | O |
| D-9476 | Me(S) | single bond | H | Et | H | Me | CH₂OEt | O |
| D-9477 | H | single bond | Me | *c*-Pr | H | Me | CH₂OEt | O |
| D-9478 | Me | single bond | H | *c*-Pr | H | Me | CH₂OEt | O |
| D-9479 | Me(R) | single bond | H | *c*-Pr | H | Me | CH₂OEt | O |
| D-9480 | Me(S) | single bond | H | *c*-Pr | H | Me | CH₂OEt | O |
| D-9481 | H | single bond | Me | *c*-Pr(1-CN) | H | Me | CH₂OEt | O |
| D-9482 | Me | single bond | H | *c*-Pr(1-CN) | H | Me | CH₂OEt | O |
| D-9483 | Me(R) | single bond | H | *c*-Pr(1-CN) | H | Me | CH₂OEt | O |
| D-9484 | Me(S) | single bond | H | *c*-Pr(1-CN) | H | Me | CH₂OEt | O |
| D-9485 | H | single bond | Me | CH₂CF₃ | H | Cl | CH₂SMe | O |
| D-9486 | Me | single bond | H | CH₂CF₃ | H | Cl | CH₂SMe | O |
| D-9487 | Me(R) | single bond | H | CH₂CF₃ | H | Cl | CH₂SMe | O |
| D-9488 | Me(S) | single bond | H | CH₂CF₃ | H | Cl | CH₂SMe | O |
| D-9489 | H | single bond | Me | Et | H | Cl | CH₂SMe | O |
| D-9490 | Me | single bond | H | Et | H | Cl | CH₂SMe | O |
| D-9491 | Me(R) | single bond | H | Et | H | Cl | CH₂SMe | O |
| D-9492 | Me(S) | single bond | H | Et | H | Cl | CH₂SMe | O |
| D-9493 | H | single bond | Me | *c*-Pr | H | Cl | CH₂SMe | O |
| D-9494 | Me | single bond | H | *c*-Pr | H | Cl | CH₂SMe | O |

**[Table 862]**

| Compound Number | Y | W | Z | R¹ | R² | R³ | R¹⁰ | A |
|---|---|---|---|---|---|---|---|---|
| D-9495 | Me(R) | single bond | H | *c*-Pr | H | Cl | CH₂SMe | O |
| D-9496 | Me(S) | single bond | H | *c*-Pr | H | Cl | CH₂SMe | O |
| D-9497 | H | single bond | Me | *c*-Pr(1-CN) | H | Cl | CH₂SMe | O |
| D-9498 | Me | single bond | H | *c*-Pr(1-CN) | H | Cl | CH₂SMe | O |
| D-9499 | Me(R) | single bond | H | *c*-Pr(1-CN) | H | Cl | CH₂SMe | O |
| D-9500 | Me(S) | single bond | H | *c-*Pr(1-CN) | H | Cl | CH₂SMe | O |
| D-9501 | H | single bond | Me | CH₂CF₃ | H | Me | CH₂SMe | O |
| D-9502 | Me | single bond | H | CH₂CF₃ | H | Me | CH₂SMe | O |
| D-9503 | Me(R) | single bond | H | CH₂CF₃ | H | Me | CH₂SMe | O |
| D-9504 | Me(S) | single bond | H | CH₂CF₃ | H | Me | CH₂SMe | O |
| D-9505 | H | single bond | Me | Et | H | Me | CH₂SMe | O |
| D-9506 | Me | single bond | H | Et | H | Me | CH₂SMe | O |
| D-9507 | Me(R) | single bond | H | Et | H | Me | CH₂SMe | O |
| D-9508 | Me(S) | single bond | H | Et | H | Me | CH₂SMe | O |
| D-9509 | H | single bond | Me | c-Pr | H | Me | CH₂SMe | O |
| D-9510 | Me | single bond | H | c-Pr | H | Me | CH₂SMe | O |
| D-9511 | Me(R) | single bond | H | c-Pr | H | Me | CH₂SMe | O |
| D-9512 | Me(S) | single bond | H | c-Pr | H | Me | CH₂SMe | O |
| D-9513 | H | single bond | Me | *c*-Pr(1-CN) | H | Me | CH₂SMe | O |
| D-9514 | Me | single bond | H | *c*-Pr(1-CN) | H | Me | CH₂SMe | O |
| D-9515 | Me(R) | single bond | H | *c*-Pr(1-CN) | H | Me | CH₂SMe | O |
| D-9516 | Me(S) | single bond | H | *c*-Pr(1-CN) | H | Me | CH₂SMe | O |
| D-9517 | H | single bond | Me | CH₂CF₃ | H | Cl | CH₂S(=O)₂Me | O |
| D-9518 | Me | single bond | H | CH₂CF₃ | H | Cl | CH₂S(=O)₂Me | O |
| D-9519 | Me(R) | single bond | H | CH₂CF₃ | H | Cl | CH₂S(=O)₂Me | O |
| D-9520 | Me(S) | single bond | H | CH₂CF₃ | H | Cl | CH₂S(=O)₂Me | O |
| D-9521 | H | single bond | Me | Et | H | Cl | CH₂S(=O)₂Me | O |
| D-9522 | Me | single bond | H | Et | H | Cl | CH₂S(=O)₂Me | O |
| D-9523 | Me(R) | single bond | H | Et | H | Cl | CH₂S(=O)₂Me | O |
| D-9524 | Me(S) | single bond | H | Et | H | Cl | CH₂S(=O)₂Me | O |
| D-9525 | H | single bond | Me | *c* -Pr | H | Cl | CH₂S(=O)₂Me | O |
| D-9526 | Me | single bond | H | *c* -Pr | H | Cl | CH₂S(=O)₂Me | O |
| D-9527 | Me(R) | single bond | H | *c* -Pr | H | Cl | CH₂S(=O)₂Me | O |
| D-9528 | Me(S) | single bond | H | *c* -Pr | H | Cl | CH₂S(=O)₂Me | O |
| D-9529 | H | single bond | Me | *c*-Pr(1-CN) | H | Cl | CH₂S(=O)₂Me | O |
| D-9530 | Me | single bond | H | *c*-Pr(1-CN) | H | Cl | CH₂S(=O)₂Me | O |
| D-9531 | Me(R) | single bond | H | *c* -Pr(1-CN) | H | Cl | CH₂S(=O)₂Me | O |
| D-9532 | Me(S) | single bond | H | *c-*Pr(1-CN) | H | Cl | CH₂S(=O)₂Me | O |
| D-9533 | H | single bond | Me | CH₂CF₃ | H | Me | CH₂S(=O)₂Me | O |
| D-9534 | Me | single bond | H | CH₂CF₃ | H | Me | CH₂S(=O)₂Me | O |
| D-9535 | Me(R) | single bond | H | CH₂CF₃ | H | Me | CH₂S(=O)₂Me | O |
| D-9536 | Me(S) | single bond | H | CH₂CF₃ | H | Me | CH₂S(=O)₂Me | O |
| D-9537 | H | single bond | Me | Et | H | Me | CH₂S(=O)₂Me | O |
| D-9538 | Me | single bond | H | Et | H | Me | CH₂S(=O)₂Me | O |

**[Table 863]**

| Compound Number | Y | W | Z | R¹ | R² | R³ | R¹⁰ | A |
|---|---|---|---|---|---|---|---|---|
| D-9539 | Me(R) | single bond | H | Et | H | Me | CH₂S(=O)₂Me | O |
| D-9540 | Me(S) | single bond | H | Et | H | Me | CH₂S(=O)₂Me | O |
| D-9541 | H | single bond | Me | *c*-Pr | H | Me | CH₂S(=O)₂Me | O |
| D-9542 | Me | single bond | H | *c*-Pr | H | Me | CH₂S(=O)₂Me | O |
| D-9543 | Me(R) | single bond | H | *c*-Pr | H | Me | CH₂S(=O)₂Me | O |
| D-9544 | Me(S) | single bond | H | *c* Pr | H | Me | CH₂S(=O)₂Me | O |
| D-9545 | H | single bond | Me | *c*-Pr(1-CN) | H | Me | CH₂S(=O)₂Me | O |
| D-9546 | Me | single bond | H | *c*-Pr(1-CN) | H | Me | CH₂S(=O)₂Me | O |
| D-9547 | Me(R) | single bond | H | *c*-Pr(1-CN) | H | Me | CH₂S(=O)₂Me | O |
| D-9548 | Me(S) | single bond | H | *c*-Pr(1-CN) | H | Me | CH₂S(=O)₂Me | O |
| D-9549 | H | single bond | Me | CH₂CF₃ | H | Cl | CH₂C(=O)Me | O |
| D-9550 | Me | single bond | H | CH₂CF₃ | H | Cl | CH₂C(=O)Me | O |
| D-9551 | Me(R) | single bond | H | CH₂CF₃ | H | Cl | CH₂C(=O)Me | O |
| D-9552 | Me(S) | single bond | H | CH₂CF₃ | H | Cl | CH₂C(=O)Me | O |
| D-9553 | H | single bond | Me | Et | H | Cl | CH₂C(=O)Me | O |
| D-9554 | Me | single bond | H | Et | H | Cl | CH₂C(=O)Me | O |
| D-9555 | Me(R) | single bond | H | Et | H | Cl | CH₂C(=O)Me | O |
| D-9556 | Me(S) | single bond | H | Et | H | Cl | CH₂C(=O)Me | O |
| D-9557 | H | single bond | Me | *c*-Pr | H | Cl | CH₂C(=O)Me | O |
| D-9558 | Me | single bond | H | *c*-Pr | H | Cl | CH₂C(=O)Me | O |
| D-9559 | Me(R) | single bond | H | *c*-Pr | H | Cl | CH₂C(=O)Me | O |
| D-9560 | Me(S) | single bond | H | *c*-Pr | H | Cl | CH₂C(=O)Me | O |
| D-9561 | H | single bond | Me | *c*-Pr(1-CN) | H | Cl | CH₂C(=O)Me | O |
| D-9562 | Me | single bond | H | *c*-Pr(1-CN) | H | Cl | CH₂C(=O)Me | O |
| D-9563 | Me(R) | single bond | H | *c*-Pr(1-CN) | H | Cl | CH₂C(=O)Me | O |
| D-9564 | Me(S) | single bond | H | *c*-Pr(1-CN) | H | Cl | CH₂C(=O)Me | O |
| D-9565 | H | single bond | Me | CH₂CF₃ | H | Me | CH₂C(=O)Me | O |
| D-9566 | Me | single bond | H | CH₂CF₃ | H | Me | CH₂C(=O)Me | O |
| D-9567 | Me(R) | single bond | H | CH₂CF₃ | H | Me | CH₂C(=O)Me | O |
| D-9568 | Me(S) | single bond | H | CH₂CF₃ | H | Me | CH₂C(=O)Me | O |
| D-9569 | H | single bond | Me | Et | H | Me | CH₂C(=O)Me | O |
| D-9570 | Me | single bond | H | Et | H | Me | CH₂C(=O)Me | O |
| D-9571 | Me(R) | single bond | H | Et | H | Me | CH₂C(=O)Me | O |
| D-9572 | Me(S) | single bond | H | Et | H | Me | CH₂C(=O)Me | O |
| D-9573 | H | single bond | Me | *c*-Pr | H | Me | CH₂C(=O)Me | O |
| D-9574 | Me | single bond | H | *c*-Pr | H | Me | CH₂C(=O)Me | O |
| D-9575 | Me(R) | single bond | H | *c*-Pr | H | Me | CH₂C(=O)Me | O |
| D-9576 | Me(S) | single bond | H | *c*-Pr | H | Me | CH₂C(=O)Me | O |
| D-9577 | H | single bond | Me | *c*-Pr(1-CN) | H | Me | CH₂C(=O)Me | O |
| D-9578 | Me | single bond | H | *c*-Pr(1-CN) | H | Me | CH₂C(=O)Me | O |
| D-9579 | Me(R) | single bond | H | *c*-Pr(1-CN) | H | Me | CH₂C(=O)Me | O |
| D-9580 | Me(S) | single bond | H | *c*-Pr(1-CN) | H | Me | CH₂C(=O)Me | O |
| D-9581 | H | single bond | Me | CH₂CF₃ | H | Cl | CH₂C(=O)OMe | O |
| D-9582 | Me | single bond | H | CH₂CF₃ | H | Cl | CH₂C(=O)OMe | O |

**[Table 864]**

| Compound Number | Y | W | Z | R¹ | R² | R³ | R¹⁰ | A |
|---|---|---|---|---|---|---|---|---|
| D-9583 | Me(R) | single bond | H | CH₂CF₃ | H | Cl | CH₂C(=O)OMe | O |
| D-9584 | Me(S) | single bond | H | CH₂CF₃ | H | Cl | CH₂C(=O)OMe | O |
| D-9585 | H | single bond | Me | Et | H | Cl | CH₂C(=O)OMe | O |
| D-9586 | Me | single bond | H | Et | H | Cl | CH₂C(=O)OMe | O |
| D-9587 | Me(R) | single bond | H | Et | H | Cl | CH₂C(=O)OMe | O |
| D-9588 | Me(S) | single bond | H | Et | H | Cl | CH₂C(=O)OMe | O |
| D-9589 | H | single bond | Me | *c*-Pr | H | Cl | CH₂C(=O)OMe | O |
| D-9590 | Me | single bond | H | *c*-Pr | H | Cl | CH₂C(=O)OMe | O |
| D-9591 | Me(R) | single bond | H | *c*-Pr | H | Cl | CH₂C(=O)OMe | O |
| D-9592 | Me(S) | single bond | H | *c*-Pr | H | Cl | CH₂C(=O)OMe | O |
| D-9593 | H | single bond | Me | *c*-Pr(1-CN) | H | Cl | CH₂C(=O)OMe | O |
| D-9594 | Me | single bond | H | *c*-Pr(1-CN) | H | Cl | CH₂C(=O)OMe | O |
| D-9595 | Me(R) | single bond | H | *c-*Pr(1-CN) | H | Cl | CH₂C(=O)OMe | O |
| D-9596 | Me(S) | single bond | H | *c*-Pr(1-CN) | H | Cl | CH₂C(=O)OMe | O |
| D-9597 | H | single bond | Me | CH₂CF₃ | H | Me | CH₂C(=O)OMe | O |
| D-9598 | Me | single bond | H | CH₂CF₃ | H | Me | CH₂C(=O)OMe | O |
| D-9599 | Me(R) | single bond | H | CH₂CF₃ | H | Me | CH₂C(=O)OMe | O |
| D-9600 | Me(S) | single bond | H | CH₂CF₃ | H | Me | CH₂C(=O)OMe | O |
| D-9601 | H | single bond | Me | Et | H | Me | CH₂C(=O)OMe | O |
| D-9602 | Me | single bond | H | Et | H | Me | CH₂C(=O)OMe | O |
| D-9603 | Me(R) | single bond | H | Et | H | Me | CH₂C(=O)OMe | O |
| D-9604 | Me(S) | single bond | H | Et | H | Me | CH₂C(=O)OMe | O |
| D-9605 | H | single bond | Me | *c*-Pr | H | Me | CH₂C(=O)OMe | O |
| D-9606 | Me | single bond | H | *c*-Pr | H | Me | CH₂C(=O)OMe | O |
| D-9607 | Me(R) | single bond | H | *c*-Pr | H | Me | CH₂C(=O)OMe | O |
| D-9608 | Me(S) | single bond | H | *c*-Pr | H | Me | CH₂C(=O)OMe | O |
| D-9609 | H | single bond | Me | *c*-Pr(1-CN) | H | Me | CH₂C(=O)OMe | O |
| D-9610 | Me | single bond | H | *c*-Pr(1-CN) | H | Me | CH₂C(=O)OMe | O |
| D-9611 | Me(R) | single bond | H | *c*-Pr(1-CN) | H | Me | CH₂C(=O)OMe | O |
| D-9612 | Me(S) | single bond | H | *c*-Pr(1-CN) | H | Me | CH₂C(=O)OMe | O |
| D-9613 | H | single bond | Me | CH₂CF₃ | H | Cl | CH₂C(=O)NHMe | O |
| D-9614 | Me | single bond | H | CH₂CF₃ | H | Cl | CH₂C(=O)NHMe | O |
| D-9615 | Me(R) | single bond | H | CH₂CF₃ | H | Cl | CH₂C(=O)NHMe | O |
| D-9616 | Me(S) | single bond | H | CH₂CF₃ | H | Cl | CH₂C(=O)NHMe | O |
| D-9617 | H | single bond | Me | Et | H | Cl | CH₂C(=O)NHMe | O |
| D-9618 | Me | single bond | H | Et | H | Cl | CH₂C(=O)NHMe | O |
| D-9619 | Me(R) | single bond | H | Et | H | Cl | CH₂C(=O)NHMe | O |
| D-9620 | Me(S) | single bond | H | Et | H | Cl | CH₂C(=O)NHMe | O |
| D-9621 | H | single bond | Me | *c*-Pr | H | Cl | CH₂C(=O)NHMe | O |
| D-9622 | Me | single bond | H | *c*-Pr | H | Cl | CH₂C(=O)NHMe | O |
| D-9623 | Me(R) | single bond | H | *c*-Pr | H | Cl | CH₂C(=O)NHMe | O |
| D-9624 | Me(S) | single bond | H | *c*-Pr | H | Cl | CH₂C(=O)NHMe | O |
| D-9625 | H | single bond | Me | *c*-Pr(1-CN) | H | Cl | CH₂C(=O)NHMe | O |
| D-9626 | Me | single bond | H | *c-*Pr(1-CN) | H | Cl | CH₂C(=O)NHMe | O |

**[Table 865]**

| Compound Number | Y | W | Z | R¹ | R² | R³ | R¹⁰ | A |
|---|---|---|---|---|---|---|---|---|
| D-9627 | Me(R) | single bond | H | *c*-Pr(1-CN) | H | Cl | CH₂C(=O)NHMe | O |
| D-9628 | Me(S) | single bond | H | *c*-Pr(1-CN) | H | Cl | CH₂C(=O)NHMe | O |
| D-9629 | H | single bond | Me | CH₂CF₃ | H | Me | CH₂C(=O)NHMe | O |
| D-9630 | Me | single bond | H | CH₂CF₃ | H | Me | CH₂C(=O)NHMe | O |
| D-9631 | Me(R) | single bond | H | CH₂CF₃ | H | Me | CH₂C(=O)NHMe | O |
| D-9632 | Me(S) | single bond | H | CH₂CF₃ | H | Me | CH₂C(=O)NHMe | O |
| D-9633 | H | single bond | Me | Et | H | Me | CH₂C(=O)NHMe | O |
| D-9634 | Me | single bond | H | Et | H | Me | CH₂C(=O)NHMe | O |
| D-9635 | Me(R) | single bond | H | Et | H | Me | CH₂C(=O)NHMe | O |
| D-9636 | Me(S) | single bond | H | Et | H | Me | CH₂C(=O)NHMe | O |
| D-9637 | H | single bond | Me | *c*-Pr | H | Me | CH₂C(=O)NHMe | O |
| D-9638 | Me | single bond | H | *c*-Pr | H | Me | CH₂C(=O)NHMe | O |
| D-9639 | Me(R) | single bond | H | *c*-Pr | H | Me | CH₂C(=O)NHMe | O |
| D-9640 | Me(S) | single bond | H | *c*-Pr | H | Me | CH₂C(=O)NHMe | O |
| D-9641 | H | single bond | Me | *c*-Pr(1-CN) | H | Me | CH₂C(=O)NHMe | O |
| D-9642 | Me | single bond | H | *c*-Pr(1-CN) | H | Me | CH₂C(=O)NHMe | O |
| D-9643 | Me(R) | single bond | H | *c*-Pr(1-CN) | H | Me | CH₂C(=O)NHMe | O |
| D-9644 | Me(S) | single bond | H | *c*-Pr(1-CN) | H | Me | CH₂C(=O)NHMe | O |
| D-9645 | H | single bond | Me | CH₂CF₃ | H | Cl | CH₂OC(=O)Me | O |
| D-9646 | Me | single bond | H | CH₂CF₃ | H | Cl | CH₂OC(=O)Me | O |
| D-9647 | Me(R) | single bond | H | CH₂CF₃ | H | Cl | CH₂OC(=O)Me | O |
| D-9648 | Me(S) | single bond | H | CH₂CF₃ | H | Cl | CH₂OC(=O)Me | O |
| D-9649 | H | single bond | Me | Et | H | Cl | CH₂OC(=O)Me | O |
| D-9650 | Me | single bond | H | Et | H | Cl | CH₂OC(=O)Me | O |
| D-9651 | Me(R) | single bond | H | Et | H | Cl | CH₂OC(=O)Me | O |
| D-9652 | Me(S) | single bond | H | Et | H | Cl | CH₂OC(=O)Me | O |
| D-9653 | H | single bond | Me | *c*-Pr | H | Cl | CH₂OC(=O)Me | O |
| D-9654 | Me | single bond | H | *c*-Pr | H | Cl | CH₂OC(=O)Me | O |
| D-9655 | Me(R) | single bond | H | *c*-Pr | H | Cl | CH₂OC(=O)Me | O |
| D-9656 | Me(S) | single bond | H | *c*-Pr | H | Cl | CH₂OC(=O)Me | O |
| D-9657 | H | single bond | Me | *c*-Pr(1-CN) | H | Cl | CH₂OC(=O)Me | O |
| D-9658 | Me | single bond | H | *c*-Pr(1-CN) | H | Cl | CH₂OC(=O)Me | O |
| D-9659 | Me(R) | single bond | H | *c*-Pr(1-CN) | H | Cl | CH₂OC(=O)Me | O |
| D-9660 | Me(S) | single bond | H | *c*-Pr(1-CN) | H | Cl | CH₂OC(=O)Me | O |
| D-9661 | H | single bond | Me | CH₂CF₃ | H | Me | CH₂OC(=O)Me | O |
| D-9662 | Me | single bond | H | CH₂CF₃ | H | Me | CH₂OC(=O)Me | O |
| D-9663 | Me(R) | single bond | H | CH₂CF₃ | H | Me | CH₂OC(=O)Me | O |
| D-9664 | Me(S) | single bond | H | CH₂CF₃ | H | Me | CH₂OC(=O)Me | O |
| D-9665 | H | single bond | Me | Et | H | Me | CH₂OC(=O)Me | O |
| D-9666 | Me | single bond | H | Et | H | Me | CH₂OC(=O)Me | O |
| D-9667 | Me(R) | single bond | H | Et | H | Me | CH₂OC(=O)Me | O |
| D-9668 | Me(S) | single bond | H | Et | H | Me | CH₂OC(=O)Me | O |
| D-9669 | H | single bond | Me | *c*-Pr | H | Me | CH₂OC(=O)Me | O |
| D-9670 | Me | single bond | H | *c*-Pr | H | Me | CH₂OC(=O)Me | O |

**[Table 866]**

| Compound Number | Y | W | Z | R¹ | R² | R³ | R¹⁰ | A |
|---|---|---|---|---|---|---|---|---|
| D-9671 | Me(R) | single bond | H | *c*-Pr | H | Me | CH₂OC(=O)Me | O |
| D-9672 | Me(S) | single bond | H | *c*-Pr | H | Me | CH₂OC(=O)Me | O |
| D-9673 | H | single bond | Me | *c*-Pr(1-CN) | H | Me | CH₂OC(=O)Me | O |
| D-9674 | Me | single bond | H | *c*-Pr(1-CN) | H | Me | CH₂OC(=O)Me | O |
| D-9675 | Me(R) | single bond | H | *c*-Pr(1-CN) | H | Me | CH₂OC(=O)Me | O |
| D-9676 | Me(S) | single bond | H | *c*-Pr(1-CN) | H | Me | CH₂OC(=O)Me | O |
| D-9677 | H | single bond | Me | CH₂CF₃ | H | Cl | CH₂OC(=O)(*i*-Pr) | O |
| D-9678 | Me | single bond | H | CH₂CF₃ | H | Cl | CH₂OC(=O)(*i*-Pr) | O |
| D-9679 | Me(R) | single bond | H | CH₂CF₃ | H | Cl | CH₂OC(=O)(*i*-Pr) | O |
| D-9680 | Me(S) | single bond | H | CH₂CF₃ | H | Cl | CH₂OC(=O)(*i*-Pr) | O |
| D-9681 | H | single bond | Me | Et | H | Cl | CH₂OC(=O)(*i*-Pr) | O |
| D-9682 | Me | single bond | H | Et | H | Cl | CH₂OC (=O)(*i*-Pr) | O |
| D-9683 | Me(R) | single bond | H | Et | H | Cl | CH₂OC(=O)(*i*-Pr) | O |
| D-9684 | Me(S) | single bond | H | Et | H | Cl | CH₂OC(=O)(*i*-Pr) | O |
| D-9685 | H | single bond | Me | *c*-Pr | H | Cl | CH₂OC(=O)(*i*-Pr) | O |
| D-9686 | Me | single bond | H | *c*-Pr | H | Cl | CH₂OC(=O)(*i*-Pr) | O |
| D-9687 | Me(R) | single bond | H | *c*-Pr | H | Cl | CH₂OC(=O)(*i*-Pr) | O |
| D-9688 | Me(S) | single bond | H | *c*-Pr | H | Cl | CH₂OC(=O)(*i*-Pr) | O |
| D-9689 | H | single bond | Me | *c*-Pr(1-CN) | H | Cl | CH₂OC(=O)(*i*-Pr) | O |
| D-9690 | Me | single bond | H | *c*-Pr(1-CN) | H | Cl | CH₂OC(=O)(*i*-Pr) | O |
| D-9691 | Me(R) | single bond | H | *c*-Pr(1-CN) | H | Cl | CH₂OC(=O)(*i*-Pr) | O |
| D-9692 | Me(S) | single bond | H | *c*-Pr(1-CN) | H | Cl | CH₂OC(=O)(*i*-Pr) | O |
| D-9693 | H | single bond | Me | CH₂CF₃ | H | Me | CH₂OC(=O)(*i*-Pr) | O |
| D-9694 | Me | single bond | H | CH₂CF₃ | H | Me | CH₂OC(=O)(*i*-Pr) | O |
| D-9695 | Me(R) | single bond | H | CH₂CF₃ | H | Me | CH₂OC(=O)(*i*-Pr) | O |
| D-9696 | Me(S) | single bond | H | CH₂CF₃ | H | Me | CH₂OC(=O)(*i*-Pr) | O |
| D-9697 | H | single bond | Me | Et | H | Me | CH₂OC(=O)(*i*-Pr) | O |
| D-9698 | Me | single bond | H | Et | H | Me | CH₂OC(=O)(*i*-Pr) | O |
| D-9699 | Me(R) | single bond | H | Et | H | Me | CH₂OC(=O)(*i*-Pr) | O |
| D-9700 | Me(S) | single bond | H | Et | H | Me | CH₂OC(=O)(*i*-Pr) | O |
| D-9701 | H | single bond | Me | *c*-Pr | H | Me | CH₂OC(=O)(*i*-Pr) | O |
| D-9702 | Me | single bond | H | *c*-Pr | H | Me | CH₂OC(=O)(*i*-Pr) | O |
| D-9703 | Me(R) | single bond | H | *c*-Pr | H | Me | CH₂OC(=O)(*i*-Pr) | O |
| D-9704 | Me(S) | single bond | H | *c*-Pr | H | Me | CH₂OC(=O)(*i*-Pr) | O |
| D-9705 | H | single bond | Me | *c*-Pr(1-CN) | H | Me | CH₂OC(=O)(*i*-Pr) | O |
| D-9706 | Me | single bond | H | *c*-Pr(1-CN) | H | Me | CH₂OC(=O)(*i*-Pr) | O |
| D-9707 | Me(R) | single bond | H | *c*-Pr(1-CN) | H | Me | CH₂OC(=O)(*i*-Pr) | O |
| D-9708 | Me(S) | single bond | H | *c*-Pr(1-CN) | H | Me | CH₂OC(=O)(*i*-Pr) | O |
| D-9709 | H | single bond | Me | CH₂CF₃ | H | Cl | CH₂OC(=O)(*t*-Bu) | O |
| D-9710 | Me | single bond | H | CH₂CF₃ | H | Cl | CH₂OC(=O)(*t*-Bu) | O |
| D-9711 | Me(R) | single bond | H | CH₂CF₃ | H | Cl | CH₂OC(=O)(*t*-Bu) | O |
| D-9712 | Me(S) | single bond | H | CH₂CF₃ | H | Cl | CH₂OC(=O)(*t*-Bu) | O |
| D-9713 | H | single bond | Me | Et | H | Cl | CH₂OC(=O)(*t*-Bu) | O |
| D-9714 | Me | single bond | H | Et | H | Cl | CH₂OC(=O)(*t*-Bu) | O |

**[Table 867]**

| Compound Number | Y | W | Z | R¹ | R² | R³ | R¹⁰ | A |
|---|---|---|---|---|---|---|---|---|
| D-9715 | Me(R) | single bond | H | Et | H | Cl | CH₂OC(=O)(*t*-Bu) | O |
| D-9716 | Me(S) | single bond | H | Et | H | Cl | CH₂OC(=O)(*t*-Bu) | O |
| D-9717 | H | single bond | Me | *c*-Pr | H | Cl | CH₂OC(=O)(*t*-Bu) | O |
| D-9718 | Me | single bond | H | *c*-Pr | H | Cl | CH₂OC(=O)(*t*-Bu) | O |
| D-9719 | Me(R) | single bond | H | *c*-Pr | H | Cl | CH₂OC(=O)(*t*-Bu) | O |
| D-9720 | Me(S) | single bond | H | *c*-Pr | H | Cl | CH₂OC(=O)(*t*-Bu) | O |
| D-9721 | H | single bond | Me | *c*-Pr(1-CN) | H | Cl | CH₂OC(=O)(*t*-Bu) | O |
| D-9722 | Me | single bond | H | *c*-Pr(1-CN) | H | Cl | CH₂OC(=O)(*t*-Bu) | O |
| D-9723 | Me(R) | single bond | H | *c*-Pr(1-CN) | H | Cl | CH₂OC(=O)(*t*-Bu) | O |
| D-9724 | Me(S) | single bond | H | *c*-Pr(1-CN) | H | Cl | CH₂OC(=O)(*t*-Bu) | O |
| D-9725 | H | single bond | Me | CH₂CF₃ | H | Me | CH₂OC(=O)(*t*-Bu) | O |
| D-9726 | Me | single bond | H | CH₂CF₃ | H | Me | CH₂OC(=O)(*t*-Bu) | O |
| D-9727 | Me(R) | single bond | H | CH₂CF₃ | H | Me | CH₂OC(=O)(*t*-Bu) | O |
| D-9728 | Me(S) | single bond | H | CH₂CF₃ | H | Me | CH₂OC(=O)(*t*-Bu) | O |
| D-9729 | H | single bond | Me | Et | H | Me | CH₂OC(=O)(*t*-Bu) | O |
| D-9730 | Me | single bond | H | Et | H | Me | CH₂OC(=O)(*t*-Bu) | O |
| D-9731 | Me(R) | single bond | H | Et | H | Me | CH₂OC(=O)(*t*-Bu) | O |
| D-9732 | Me(S) | single bond | H | Et | H | Me | CH₂OC(=O)(*t*-Bu) | O |
| D-9733 | H | single bond | Me | *c*-Pr | H | Me | CH₂OC(=O)(*t*-Bu) | O |
| D-9734 | Me | single bond | H | *c*-Pr | H | Me | CH₂OC(=O)(*t*-Bu) | O |
| D-9735 | Me(R) | single bond | H | *c*-Pr | H | Me | CH₂OC(=O)(*t*-Bu) | O |
| D-9736 | Me(S) | single bond | H | *c*-Pr | H | Me | CH₂OC(=O)(*t*-Bu) | O |
| D-9737 | H | single bond | Me | *c*-Pr(1-CN) | H | Me | CH₂OC(=O)(*t*-Bu) | O |
| D-9738 | Me | single bond | H | *c*-Pr(1-CN) | H | Me | CH₂OC(=O)(*t*-Bu) | O |
| D-9739 | Me(R) | single bond | H | *c*-Pr(1-CN) | H | Me | CH₂OC(=O)(*t*-Bu) | O |
| D-9740 | Me(S) | single bond | H | *c*-Pr(1-CN) | H | Me | CH₂OC(=O)(*t*-Bu) | O |
| D-9741 | H | single bond | Me | CH₂CF₃ | H | Cl | CH₂OC(=O)OEt | O |
| D-9742 | Me | single bond | H | CH₂CF₃ | H | Cl | CH₂OC(=O)OEt | O |
| D-9743 | Me(R) | single bond | H | CH₂CF₃ | H | Cl | CH₂OC(=O)OEt | O |
| D-9744 | Me(S) | single bond | H | CH₂CF₃ | H | Cl | CH₂OC(=O)OEt | O |
| D-9745 | H | single bond | Me | Et | H | Cl | CH₂OC(=O)OEt | O |
| D-9746 | Me | single bond | H | Et | H | Cl | CH₂OC(=O)OEt | O |
| D-9747 | Me(R) | single bond | H | Et | H | Cl | CH₂OC(=O)OEt | O |
| D-9748 | Me(S) | single bond | H | Et | H | Cl | CH₂OC(=O)OEt | O |
| D-9749 | H | single bond | Me | *c*-Pr | H | Cl | CH₂OC(=O)OEt | O |
| D-9750 | Me | single bond | H | *c*-Pr | H | Cl | CH₂OC(=O)OEt | O |
| D-9751 | Me(R) | single bond | H | *c*-Pr | H | Cl | CH₂OC(=O)OEt | O |
| D-9752 | Me(S) | single bond | H | *c*-Pr | H | Cl | CH₂OC(=O)OEt | O |
| D-9753 | H | single bond | Me | *c*-Pr(1-CN) | H | Cl | CH₂OC(=O)OEt | O |
| D-9754 | Me | single bond | H | *c*-Pr(1-CN) | H | Cl | CH₂OC(=O)OEt | O |
| D-9755 | Me(R) | single bond | H | *c*-Pr(1-CN) | H | Cl | CH₂OC(=O)OEt | O |
| D-9756 | Me(S) | single bond | H | *c*-Pr(1-CN) | H | Cl | CH₂OC(=O)OEt | O |
| D-9757 | H | single bond | Me | CH₂CF₃ | H | Me | CH₂OC(=O)OEt | O |
| D-9758 | Me | single bond | H | CH₂CF₃ | H | Me | CH₂OC(=O)OEt | O |

**[Table 868]**

| Compound Number | Y | W | Z | R¹ | R² | R³ | R¹⁰ | A |
|---|---|---|---|---|---|---|---|---|
| D-9759 | Me(R) | single bond | H | CH₂CF₃ | H | Me | CH₂OC(=O)OEt | O |
| D-9760 | Me(S) | single bond | H | CH₂CF₃ | H | Me | CH₂OC(=O)OEt | O |
| D-9761 | H | single bond | Me | Et | H | Me | CH₂OC(=O)OEt | O |
| D-9762 | Me | single bond | H | Et | H | Me | CH₂OC(=O)OEt | O |
| D-9763 | Me(R) | single bond | H | Et | H | Me | CH₂OC(=O)OEt | O |
| D-9764 | Me(S) | single bond | H | Et | H | Me | CH₂OC(=O)OEt | O |
| D-9765 | H | single bond | Me | *c*-Pr | H | Me | CH₂OC(=O)OEt | O |
| D-9766 | Me | single bond | H | *c*-Pr | H | Me | CH₂OC(=O)OEt | O |
| D-9767 | Me(R) | single bond | H | *c*-Pr | H | Me | CH₂OC(=O)OEt | O |
| D-9768 | Me(S) | single bond | H | *c*-Pr | H | Me | CH₂OC(=O)OEt | O |
| D-9769 | H | single bond | Me | *c*-Pr(1-CN) | H | Me | CH₂OC(=O)OEt | O |
| D-9770 | Me | single bond | H | *c*-Pr(1-CN) | H | Me | CH₂OC(=O)OEt | O |
| D-9771 | Me(R) | single bond | H | *c*-Pr(1-CN) | H | Me | CH₂OC(=O)OEt | O |
| D-9772 | Me(S) | single bond | H | *c*-Pr(1-CN) | H | Me | CH₂OC(=O)OEt | O |
| D-9773 | H | single bond | Me | CH₂CF₃ | H | Cl | CH₂OC(=O)O(*i*-Pr) | O |
| D-9774 | Me | single bond | H | CH₂CF₃ | H | Cl | CH₂OC(=O)O(*i*-Pr) | O |
| D-9775 | Me(R) | single bond | H | CH₂CF₃ | H | Cl | CH₂OC(=O)O(*i*-Pr) | O |
| D-9776 | Me(S) | single bond | H | CH₂CF₃ | H | Cl | CH₂OC(=O)O(*i*-Pr) | O |
| D-9777 | H | single bond | Me | Et | H | Cl | CH₂OC(=O)O(*i*-Pr) | O |
| D-9778 | Me | single bond | H | Et | H | Cl | CH₂OC(=O)O(*i*-Pr) | O |
| D-9779 | Me(R) | single bond | H | Et | H | Cl | CH₂OC(=O)O(*i*-Pr) | O |
| D-9780 | Me(S) | single bond | H | Et | H | Cl | CH₂OC(=O)O(*i*-Pr) | O |
| D-9781 | H | single bond | Me | *c*-Pr | H | Cl | CH₂OC(=O)O(*i*-Pr) | O |
| D-9782 | Me | single bond | H | *c*-Pr | H | Cl | CH₂OC(=O)O(*i*-Pr) | O |
| D-9783 | Me(R) | single bond | H | *c*-Pr | H | Cl | CH₂OC(=O)O(*i*-Pr) | O |
| D-9784 | Me(S) | single bond | H | *c*-Pr | H | Cl | CH₂OC(=O)O(*i*-Pr) | O |
| D-9785 | H | single bond | Me | *c*-Pr(1-CN) | H | Cl | CH₂OC(=O)O(*i*-Pr) | O |
| D-9786 | Me | single bond | H | *c*-Pr(1-CN) | H | Cl | CH₂OC(=O)O(*i*-Pr) | O |
| D-9787 | Me(R) | single bond | H | *c*-Pr(1-CN) | H | Cl | CH₂OC(=O)O(*i*-Pr) | O |
| D-9788 | Me(S) | single bond | H | *c*-Pr(1-CN) | H | Cl | CH₂OC(=O)O(*i*-Pr) | O |
| D-9789 | H | single bond | Me | CH₂CF₃ | H | Me | CH₂OC(=O)O(*i*-Pr) | O |
| D-9790 | Me | single bond | H | CH₂CF₃ | H | Me | CH₂OC(=O)O(*i*-Pr) | O |
| D-9791 | Me(R) | single bond | H | CH₂CF₃ | H | Me | CH₂OC(=O)O(*i*-Pr) | O |
| D-9792 | Me(S) | single bond | H | CH₂CF₃ | H | Me | CH₂OC(=O)O(*i*-Pr) | O |
| D-9793 | H | single bond | Me | Et | H | Me | CH₂OC(=O)O(*i*-Pr) | O |
| D-9794 | Me | single bond | H | Et | H | Me | CH₂OC(=O)O(*i*-Pr) | O |
| D-9795 | Me(R) | single bond | H | Et | H | Me | CH₂OC(=O)O(*i*-Pr) | O |
| D-9796 | Me(S) | single bond | H | Et | H | Me | CH₂OC(=O)O(*i*-Pr) | O |
| D-9797 | H | single bond | Me | *c*-Pr | H | Me | CH₂OC(=O)O(*i*-Pr) | O |
| D-9798 | Me | single bond | H | *c*-Pr | H | Me | CH₂OC(=O)O(*i*-Pr) | O |
| D-9799 | Me(R) | single bond | H | *c*-Pr | H | Me | CH₂OC(=O)O(*i*-Pr) | O |
| D-9800 | Me(S) | single bond | H | *c*-Pr | H | Me | CH₂OC(=O)O(*i*-Pr) | O |
| D-9801 | H | single bond | Me | *c*-Pr(1-CN) | H | Me | CH₂OC(=O)O(*i*-Pr) | O |
| D-9802 | Me | single bond | H | *c*-Pr(1-CN) | H | Me | CH₂OC(=O)O(*i*-Pr) | O |

**[Table 869]**

| Compound Number | Y | W | Z | R¹ | R² | R³ | R¹⁰ | A |
|---|---|---|---|---|---|---|---|---|
| D-9803 | Me(R) | single bond | H | *c*-Pr(1-CN) | H | Me | CH₂OC(=O)O(*i*-Pr) | O |
| D-9804 | Me(S) | single bond | H | *c*-Pr(1-CN) | H | Me | CH₂OC(=O)O(*i*-Pr) | O |
| D-9805 | H | single bond | Me | CH₂CF₃ | H | Cl | CH₂N(Me)C(=O)OMe | O |
| D-9806 | Me | single bond | H | CH₂CF₃ | H | Cl | CH₂N(Me)C(=O)OMe | O |
| D-9807 | Me(R) | single bond | H | CH₂CF₃ | H | Cl | CH₂N(Me)C(=O)OMe | O |
| D-9808 | Me(S) | single bond | H | CH₂CF₃ | H | Cl | CH₂N(Me)C(=O)OMe | O |
| D-9809 | H | single bond | Me | Et | H | Cl | CH₂N(Me)C(=O)OMe | O |
| D-9810 | Me | single bond | H | Et | H | Cl | CH₂N(Me)C(=O)OMe | O |
| D-9811 | Me(R) | single bond | H | Et | H | Cl | CH₂N(Me)C(=O)OMe | O |
| D-9812 | Me(S) | single bond | H | Et | H | Cl | CH₂N(Me)C(=O)OMe | O |
| D-9813 | H | single bond | Me | *c*-Pr | H | Cl | CH₂N(Me)C(=O)OMe | O |
| D-9814 | Me | single bond | H | *c*-Pr | H | Cl | CH₂N(Me)C(=O)OMe | O |
| D-9815 | Me(R) | single bond | H | *c*-Pr | H | Cl | CH₂N(Me)C(=O)OMe | O |
| D-9816 | Me(S) | single bond | H | *c*-Pr | H | Cl | CH₂N(Me)C(=O)OMe | O |
| D-9817 | H | single bond | Me | *c*-Pr(1-CN) | H | Cl | CH₂N(Me)C(=O)OMe | O |
| D-9818 | Me | single bond | H | *c*-Pr(1-CN) | H | Cl | CH₂N(Me)C(=O)OMe | O |
| D-9819 | Me(R) | single bond | H | *c*-Pr(1-CN) | H | Cl | CH₂N(Me)C(=O)OMe | O |
| D-9820 | Me(S) | single bond | H | *c*-Pr(1-CN) | H | Cl | CH₂N(Me)C(=O)OMe | O |
| D-9821 | H | single bond | Me | CH₂CF₃ | H | Me | CH₂N(Me)C(=O)OMe | O |
| D-9822 | Me | single bond | H | CH₂CF₃ | H | Me | CH₂N(Me)C(=O)OMe | O |
| D-9823 | Me(R) | single bond | H | CH₂CF₃ | H | Me | CH₂N(Me)C(=O)OMe | O |
| D-9824 | Me(S) | single bond | H | CH₂CF₃ | H | Me | CH₂N(Me)C(=O)OMe | O |
| D-9825 | H | single bond | Me | Et | H | Me | CH₂N(Me)C(=O)OMe | O |
| D-9826 | Me | single bond | H | Et | H | Me | CH₂N(Me)C(=O)OMe | O |
| D-9827 | Me(R) | single bond | H | Et | H | Me | CH₂N(Me)C(=O)OMe | O |
| D-9828 | Me(S) | single bond | H | Et | H | Me | CH₂N(Me)C(=O)OMe | O |
| D-9829 | H | single bond | Me | *c*-Pr | H | Me | CH₂N(Me)C(=O)OMe | O |
| D-9830 | Me | single bond | H | *c*-Pr | H | Me | CH₂N(Me)C(=O)OMe | O |
| D-9831 | Me(R) | single bond | H | *c*-Pr | H | Me | CH₂N(Me)C(=O)OMe | O |
| D-9832 | Me(S) | single bond | H | *c*-Pr | H | Me | CH₂N(Me)C(=O)OMe | O |
| D-9833 | H | single bond | Me | *c*-Pr(1-CN) | H | Me | CH₂N(Me)C(=O)OMe | O |
| D-9834 | Me | single bond | H | *c*-Pr(1-CN) | H | Me | CH₂N(Me)C(=O)OMe | O |
| D-9835 | Me(R) | single bond | H | *c*-Pr(1-CN) | H | Me | CH₂N(Me)C(=O)OMe | O |
| D-9836 | Me(S) | single bond | H | *c*-Pr(1-CN) | H | Me | CH₂N(Me)C(=O)OMe | O |
| D-9837 | H | single bond | Me | CH₂CF₃ | H | Cl | CH₂OCH₂CH₂OMe | O |
| D-9838 | Me | single bond | H | CH₂CF₃ | H | Cl | CH₂OCH₂CH₂OMe | O |
| D-9839 | Me(R) | single bond | H | CH₂CF₃ | H | Cl | CH₂OCH₂CH₂OMe | O |
| D-9840 | Me(S) | single bond | H | CH₂CF₃ | H | Cl | CH₂OCH₂CH₂OMe | O |
| D-9841 | H | single bond | Me | Et | H | Cl | CH₂OCH₂CH₂OMe | O |
| D-9842 | Me | single bond | H | Et | H | Cl | CH₂OCH₂CH₂OMe | O |
| D-9843 | Me(R) | single bond | H | Et | H | Cl | CH₂OCH₂CH₂OMe | O |
| D-9844 | Me(S) | single bond | H | Et | H | Cl | CH₂OCH₂CH₂OMe | O |
| D-9845 | H | single bond | Me | *c*-Pr | H | Cl | CH₂OCH₂CH₂OMe | O |
| D-9846 | Me | single bond | H | *c*-Pr | H | Cl | CH₂OCH₂CH₂OMe | O |

**[Table 870]**

| Compound Number | Y | W | Z | R¹ | R² | R³ | R¹⁰ | A |
|---|---|---|---|---|---|---|---|---|
| D-9847 | Me(R) | single bond | H | *c*-Pr | H | Cl | CH₂OCH₂CH₂OMe | O |
| D-9848 | Me(S) | single bond | H | *c*-Pr | H | Cl | CH₂OCH₂CH₂OMe | O |
| D-9849 | H | single bond | Me | *c*-Pr(1-CN) | H | Cl | CH₂OCH₂CH₂OMe | O |
| D-9850 | Me | single bond | H | *c*-Pr(1-CN) | H | Cl | CH₂OCH₂CH₂OMe | O |
| D-9851 | Me(R) | single bond | H | *c*-Pr(1-CN) | H | Cl | CH₂OCH₂CH₂OMe | O |
| D-9852 | Me(S) | single bond | H | *c*-Pr(1-CN) | H | Cl | CH₂OCH₂CH₂OMe | O |
| D-9853 | H | single bond | Me | CH₂CF₃ | H | Me | CH₂OCH₂CH₂OMe | O |
| D-9854 | Me | single bond | H | CH₂CF₃ | H | Me | CH₂OCH₂CH₂OMe | O |
| D-9855 | Me(R) | single bond | H | CH₂CF₃ | H | Me | CH₂OCH₂CH₂OMe | O |
| D-9856 | Me(S) | single bond | H | CH₂CF₃ | H | Me | CH₂OCH₂CH₂OMe | O |
| D-9857 | H | single bond | Me | Et | H | Me | CH₂OCH₂CH₂OMe | O |
| D-9858 | Me | single bond | H | Et | H | Me | CH₂OCH₂CH₂OMe | O |
| D-9859 | Me(R) | single bond | H | Et | H | Me | CH₂OCH₂CH₂OMe | O |
| D-9860 | Me(S) | single bond | H | Et | H | Me | CH₂OCH₂CH₂OMe | O |
| D-9861 | H | single bond | Me | *c*-Pr | H | Me | CH₂OCH₂CH₂OMe | O |
| D-9862 | Me | single bond | H | *c*-Pr | H | Me | CH₂OCH₂CH₂OMe | O |
| D-9863 | Me(R) | single bond | H | *c*-Pr | H | Me | CH₂OCH₂CH₂OMe | O |
| D-9864 | Me(S) | single bond | H | *c*-Pr | H | Me | CH₂OCH₂CH₂OMe | O |
| D-9865 | H | single bond | Me | *c*-Pr(1-CN) | H | Me | CH₂OCH₂CH₂OMe | O |
| D-9866 | Me | single bond | H | *c*-Pr(1-CN) | H | Me | CH₂OCH₂CH₂OMe | O |
| D-9867 | Me(R) | single bond | H | *c*-Pr(1-CN) | H | Me | CH₂OCH₂CH₂OMe | O |
| D-9868 | Me(S) | single bond | H | *c*-Pr(1-CN) | H | Me | CH₂OCH₂CH₂OMe | O |
| D-9869 | H | single bond | Me | CH₂CF₃ | H | Cl | CH₂OCH₂CH₂CN | O |
| D-9870 | Me | single bond | H | CH₂CF₃ | H | Cl | CH₂OCH₂CH₂CN | O |
| D-9871 | Me(R) | single bond | H | CH₂CF₃ | H | Cl | CH₂OCH₂CH₂CN | O |
| D-9872 | Me(S) | single bond | H | CH₂CF₃ | H | Cl | CH₂OCH₂CH₂CN | O |
| D-9873 | H | single bond | Me | Et | H | Cl | CH₂OCH₂CH₂CN | O |
| D-9874 | Me | single bond | H | Et | H | Cl | CH₂OCH₂CH₂CN | O |
| D-9875 | Me(R) | single bond | H | Et | H | Cl | CH₂OCH₂CH₂CN | O |
| D-9876 | Me(S) | single bond | H | Et | H | Cl | CH₂OCH₂CH₂CN | O |
| D-9877 | H | single bond | Me | *c*-Pr | H | Cl | CH₂OCH₂CH₂CN | O |
| D-9878 | Me | single bond | H | *c*-Pr | H | Cl | CH₂OCH₂CH₂CN | O |
| D-9879 | Me(R) | single bond | H | *c*-Pr | H | Cl | CH₂OCH₂CH₂CN | O |
| D-9880 | Me(S) | single bond | H | *c*-Pr | H | Cl | CH₂OCH₂CH₂CN | O |
| D-9881 | H | single bond | Me | *c*-Pr(1-CN) | H | Cl | CH₂OCH₂CH₂CN | O |
| D-9882 | Me | single bond | H | *c*-Pr(1-CN) | H | Cl | CH₂OCH₂CH₂CN | O |
| D-9883 | Me(R) | single bond | H | *c*-Pr(1-CN) | H | Cl | CH₂OCH₂CH₂CN | O |
| D-9884 | Me(S) | single bond | H | *c*-Pr(1-CN) | H | Cl | CH₂OCH₂CH₂CN | O |
| D-9885 | H | single bond | Me | CH₂CF₃ | H | Me | CH₂OCH₂CH₂CN | O |
| D-9886 | Me | single bond | H | CH₂CF₃ | H | Me | CH₂OCH₂CH₂CN | O |
| D-9887 | Me(R) | single bond | H | CH₂CF₃ | H | Me | CH₂OCH₂CH₂CN | O |
| D-9888 | Me(S) | single bond | H | CH₂CF₃ | H | Me | CH₂OCH₂CH₂CN | O |
| D-9889 | H | single bond | Me | Et | H | Me | CH₂OCH₂CH₂CN | O |
| D-9890 | Me | single bond | H | Et | H | Me | CH₂OCH₂CH₂CN | O |

**[Table 871]**

| Compound Number | Y | W | Z | R¹ | R² | R³ | R¹⁰ | A |
|---|---|---|---|---|---|---|---|---|
| D-9891 | Me(R) | single bond | H | Et | H | Me | CH₂OCH₂CH₂CN | O |
| D-9892 | Me(S) | single bond | H | Et | H | Me | CH₂OCH₂CH₂CN | O |
| D-9893 | H | single bond | Me | *c*-Pr | H | Me | CH₂OCH₂CH₂CN | O |
| D-9894 | Me | single bond | H | *c*-Pr | H | Me | CH₂OCH₂CH₂CN | O |
| D-9895 | Me(R) | single bond | H | *c*-Pr | H | Me | CH₂OCH₂CH₂CN | O |
| D-9896 | Me(S) | single bond | H | *c*-Pr | H | Me | CH₂OCH₂CH₂CN | O |
| D-9897 | H | single bond | Me | *c*-Pr(1-CN) | H | Me | CH₂OCH₂CH₂CN | O |
| D-9898 | Me | single bond | H | *c*-Pr(1-CN) | H | Me | CH₂OCH₂CH₂CN | O |
| D-9899 | Me(R) | single bond | H | *c*-Pr(1-CN) | H | Me | CH₂OCH₂CH₂CN | O |
| D-9900 | Me(S) | single bond | H | *c*-Pr(1-CN) | H | Me | CH₂OCH₂CH₂CN | O |
| D-9901 | H | single bond | Me | CH₂CF₃ | H | Cl | CH₂OCH₂CH₂S(=O)₂Me | O |
| D-9902 | Me | single bond | H | CH₂CF₃ | H | Cl | CH₂OCH₂CH₂S(=O)₂Me | O |
| D-9903 | Me(R) | single bond | H | CH₂CF₃ | H | Cl | CH₂OCH₂CH₂S(=O)₂Me | O |
| D-9904 | Me(S) | single bond | H | CH₂CF₃ | H | Cl | CH₂OCH₂CH₂S(=O)₂Me | O |
| D-9905 | H | single bond | Me | Et | H | Cl | CH₂OCH₂CH₂S(=O)₂Me | O |
| D-9906 | Me | single bond | H | Et | H | Cl | CH₂OCH₂CH₂S(=O)₂Me | O |
| D-9907 | Me(R) | single bond | H | Et | H | Cl | CH₂OCH₂CH₂S(=O)₂Me | O |
| D-9908 | Me(S) | single bond | H | Et | H | Cl | CH₂OCH₂CH₂S(=O)₂Me | O |
| D-9909 | H | single bond | Me | *c*-Pr | H | Cl | CH₂OCH₂CH₂S(=O)₂Me | O |
| D-9910 | Me | single bond | H | *c*-Pr | H | Cl | CH₂OCH₂CH₂S(=O)₂Me | O |
| D-9911 | Me(R) | single bond | H | *c*-Pr | H | Cl | CH₂OCH₂CH₂S(=O)₂Me | O |
| D-9912 | Me(S) | single bond | H | *c*-Pr | H | Cl | CH₂OCH₂CH₂S(=O)₂Me | O |
| D-9913 | H | single bond | Me | *c*-Pr(1-CN) | H | Cl | CH₂OCH₂CH₂S(=O)₂Me | O |
| D-9914 | Me | single bond | H | *c*-Pr(1-CN) | H | Cl | CH₂OCH₂CH₂S(=O)₂Me | O |
| D-9915 | Me(R) | single bond | H | *c*-Pr(1-CN) | H | Cl | CH₂OCH₂CH₂S(=O)₂Me | O |
| D-9916 | Me(S) | single bond | H | *c*-Pr(1-CN) | H | Cl | CH₂OCH₂CH₂S(=O)₂Me | O |
| D-9917 | H | single bond | Me | CH₂CF₃ | H | Me | CH₂OCH₂CH₂S(=O)₂Me | O |
| D-9918 | Me | single bond | H | CH₂CF₃ | H | Me | CH₂OCH₂CH₂S(=O)₂Me | O |
| D-9919 | Me(R) | single bond | H | CH₂CF₃ | H | Me | CH₂OCH₂CH₂S(=O)₂Me | O |
| D-9920 | Me(S) | single bond | H | CH₂CF₃ | H | Me | CH₂OCH₂CH₂S(=O)₂Me | O |
| D-9921 | H | single bond | Me | Et | H | Me | CH₂OCH₂CH₂S(=O)₂Me | O |
| D-9922 | Me | single bond | H | Et | H | Me | CH₂OCH₂CH₂S(=O)₂Me | O |
| D-9923 | Me(R) | single bond | H | Et | H | Me | CH₂OCH₂CH₂S(=O)₂Me | O |
| D-9924 | Me(S) | single bond | H | Et | H | Me | CH₂OCH₂CH₂S(=O)₂Me | O |
| D-9925 | H | single bond | Me | *c*-Pr | H | Me | CH₂OCH₂CH₂S(=O)₂Me | O |
| D-9926 | Me | single bond | H | *c*-Pr | H | Me | CH₂OCH₂CH₂S(=O)₂Me | O |
| D-9927 | Me(R) | single bond | H | *c*-Pr | H | Me | CH₂OCH₂CH₂S(=O)₂Me | O |
| D-9928 | Me(S) | single bond | H | *c*-Pr | H | Me | CH₂OCH₂CH₂S(=O)₂Me | O |
| D-9929 | H | single bond | Me | *c*-Pr(1-CN) | H | Me | CH₂OCH₂CH₂S(=O)₂Me | O |
| D-9930 | Me | single bond | H | *c*-Pr(1-CN) | H | Me | CH₂OCH₂CH₂S(=O)₂Me | O |
| D-9931 | Me(R) | single bond | H | *c*-Pr(1-CN) | H | Me | CH₂OCH₂CH₂S(=O)₂Me | O |
| D-9932 | Me(S) | single bond | H | *c*-Pr(1-CN) | H | Me | CH₂OCH₂CH₂S(=O)₂Me | O |
| D-9933 | H | single bond | Me | CH₂CF₃ | H | Cl | CH₂(5-Me-1,3-dioxol-2-one-4-yl) | O |
| D-9934 | Me | single bond | H | CH₂CF₃ | H | Cl | CH₂(5-Me-1,3-dioxol-2-one-4-yl) | O |

**[Table 872]**

| Compound Number | Y | W | Z | R¹ | R² | R³ | R¹⁰ | A |
|---|---|---|---|---|---|---|---|---|
| D-9935 | Me(R) | single bond | H | CH₂CF₃ | H | Cl | CH₂(5-Me-1,3-dioxol-2-one-4-yl) | O |
| D-9936 | Me(S) | single bond | H | CH₂CF₃ | H | Cl | CH₂(5-Me-1,3-dioxol-2-one-4-yl) | O |
| D-9937 | H | single bond | Me | Et | H | Cl | CH₂(5-Me-1,3-dioxol-2-one-4-yl) | O |
| D-9938 | Me | single bond | H | Et | H | Cl | CH₂(5-Me-1,3-dioxol-2-one-4-yl) | O |
| D-9939 | Me(R) | single bond | H | Et | H | Cl | CH₂(5-Me-1,3-dioxol-2-one-4-yl) | O |
| D-9940 | Me(S) | single bond | H | Et | H | Cl | CH₂(5-Me-1,3-dioxol-2-one-4-yl) | O |
| D-9941 | H | single bond | Me | *c*-Pr | H | Cl | CH₂(5-Me-1,3-dioxol-2-one-4-yl) | O |
| D-9942 | Me | single bond | H | *c*-Pr | H | Cl | CH₂(5-Me-1,3-dioxol-2-one-4-yl) | O |
| D-9943 | Me(R) | single bond | H | *c*-Pr | H | Cl | CH₂(5-Me-1,3-dioxol-2-one-4-yl) | O |
| D-9944 | Me(S) | single bond | H | *c*-Pr | H | Cl | CH₂(5-Me-1,3-dioxol-2-one-4-yl) | O |
| D-9945 | H | single bond | Me | *c*-Pr(1-CN) | H | Cl | CH₂(5-Me-1,3-dioxol-2-one-4-yl) | O |
| D-9946 | Me | single bond | H | *c*-Pr(1-CN) | H | Cl | CH₂(5-Me-1,3-dioxol-2-one-4-yl) | O |
| D-9947 | Me(R) | single bond | H | *c*-Pr(1-CN) | H | Cl | CH₂(5-Me-1,3-dioxol-2-one-4-yl) | O |
| D-9948 | Me(S) | single bond | H | *c*-Pr(1-CN) | H | Cl | CH₂(5-Me-1,3-dioxol-2-one-4-yl) | O |
| D-9949 | H | single bond | Me | CH₂CF₃ | H | Me | CH₂(5-Me-1,3-dioxol-2-one-4-yl) | O |
| D-9950 | Me | single bond | H | CH₂CF₃ | H | Me | CH₂(5-Me-1,3-dioxol-2-one-4-yl) | O |
| D-9951 | Me(R) | single bond | H | CH₂CF₃ | H | Me | CH₂(5-Me-1,3-dioxol-2-one-4-yl) | O |
| D-9952 | Me(S) | single bond | H | CH₂CF₃ | H | Me | CH₂(5-Me-1,3-dioxol-2-one-4-yl) | O |
| D-9953 | H | single bond | Me | Et | H | Me | CH₂(5-Me-1,3-dioxol-2-one-4-yl) | O |
| D-9954 | Me | single bond | H | Et | H | Me | CH₂(5-Me-1,3-dioxol-2-one-4-yl) | O |
| D-9955 | Me(R) | single bond | H | Et | H | Me | CH₂(5-Me-1,3-dioxol-2-one-4-yl) | O |
| D-9956 | Me(S) | single bond | H | Et | H | Me | CH₂(5-Me-1,3-dioxol-2-one-4-yl) | O |
| D-9957 | H | single bond | Me | *c*-Pr | H | Me | CH₂(5-Me-1,3-dioxol-2-one-4-yl) | O |
| D-9958 | Me | single bond | H | *c*-Pr | H | Me | CH₂(5-Me-1,3-dioxol-2-one-4-yl) | O |
| D-9959 | Me(R) | single bond | H | *c*-Pr | H | Me | CH₂(5-Me-1,3-dioxol-2-one-4-yl) | O |
| D-9960 | Me(S) | single bond | H | *c*-Pr | H | Me | CH₂(5-Me-1,3-dioxol-2-one-4-yl) | O |
| D-9961 | H | single bond | Me | *c*-Pr(1-CN) | H | Me | CH₂(5-Me-1,3-dioxol-2-one-4-yl) | O |
| D-9962 | Me | single bond | H | *c*-Pr(1-CN) | H | Me | CH₂(5-Me-1,3-dioxol-2-one-4-yl) | O |
| D-9963 | Me(R) | single bond | H | *c*-Pr(1-CN) | H | Me | CH₂(5-Me-1,3-dioxol-2-one-4-yl) | O |
| D-9964 | Me(S) | single bond | H | *c*-Pr(1-CN) | H | Me | CH₂(5-Me-1,3-dioxol-2-one-4-yl) | O |
| D-9965 | H | single bond | Me | CH₂CF₃ | Me | Cl | *c*-Pr | O |
| D-9966 | Me | single bond | H | CH₂CF₃ | Me | Cl | *c*-Pr | O |
| D-9967 | Me(R) | single bond | H | CH₂CF₃ | Me | Cl | *c*-Pr | O |
| D-9968 | Me(S) | single bond | H | CH₂CF₃ | Me | Cl | *c*-Pr | O |
| D-9969 | H | single bond | Me | Et | Me | Cl | *c*-Pr | O |
| D-9970 | Me | single bond | H | Et | Me | Cl | *c*-Pr | O |
| D-9971 | Me(R) | single bond | H | Et | Me | Cl | *c*-Pr | O |
| D-9972 | Me(S) | single bond | H | Et | Me | Cl | *c*-Pr | O |
| D-9973 | H | single bond | Me | *c*-Pr | Me | Cl | *c*-Pr | O |
| D-9974 | Me | single bond | H | *c*-Pr | Me | Cl | *c*-Pr | O |
| D-9975 | Me(R) | single bond | H | *c*-Pr | Me | Cl | *c*-Pr | O |
| D-9976 | Me(S) | single bond | H | *c*-Pr | Me | Cl | *c*-Pr | O |
| D-9977 | H | single bond | Me | *c*-Pr(1-CN) | Me | Cl | *c*-Pr | O |
| D-9978 | Me | single bond | H | *c*-Pr(1-CN) | Me | Cl | *c*-Pr | O |

**[Table 873]**

| Compound Number | Y | W | Z | R¹ | R² | R³ | R¹⁰ | A |
|---|---|---|---|---|---|---|---|---|
| D-9979 | Me(R) | single bond | H | *c*-Pr(1-CN) | Me | Cl | *c*-Pr | O |
| D-9980 | Me(S) | single bond | H | *c*-Pr(1-CN) | Me | Cl | *c*-Pr | O |
| D-9981 | H | single bond | Me | CH₂CF₃ | Me | Me | *c*-Pr | O |
| D-9982 | Me | single bond | H | CH₂CF₃ | Me | Me | *c*-Pr | O |
| D-9983 | Me(R) | single bond | H | CH₂CF₃ | Me | Me | *c*-Pr | O |
| D-9984 | Me(S) | single bond | H | CH₂CF₃ | Me | Me | *c*-Pr | O |
| D-9985 | H | single bond | Me | Et | Me | Me | *c*-Pr | O |
| D-9986 | Me | single bond | H | Et | Me | Me | *c*-Pr | O |
| D-9987 | Me(R) | single bond | H | Et | Me | Me | *c*-Pr | O |
| D-9988 | Me(S) | single bond | H | Et | Me | Me | *c*-Pr | O |
| D-9989 | H | single bond | Me | *c*-Pr | Me | Me | *c*-Pr | O |
| D-9990 | Me | single bond | H | *c*-Pr | Me | Me | *c*-Pr | O |
| D-9991 | Me(R) | single bond | H | *c*-Pr | Me | Me | *c*-Pr | O |
| D-9992 | Me(S) | single bond | H | *c*-Pr | Me | Me | *c*-Pr | O |
| D-9993 | H | single bond | Me | *c*-Pr(1-CN) | Me | Me | *c*-Pr | O |
| D-9994 | Me | single bond | H | *c*-Pr(1-CN) | Me | Me | *c*-Pr | O |
| D-9995 | Me(R) | single bond | H | *c*-Pr(1-CN) | Me | Me | *c*-Pr | O |
| D-9996 | Me(S) | single bond | H | *c*-Pr(1-CN) | Me | Me | *c*-Pr | O |
| D-9997 | H | single bond | Me | CH₂CF₃ | Me | Cl | OMe | O |
| D-9998 | Me | single bond | H | CH₂CF₃ | Me | Cl | OMe | O |
| D-9999 | Me(R) | single bond | H | CH₂CF₃ | Me | Cl | OMe | O |
| D-10000 | Me(S) | single bond | H | CH₂CF₃ | Me | Cl | OMe | O |
| D-10001 | H | single bond | Me | Et | Me | Cl | OMe | O |
| D-10002 | Me | single bond | H | Et | Me | Cl | OMe | O |
| D-10003 | Me(R) | single bond | H | Et | Me | Cl | OMe | O |
| D-10004 | Me(S) | single bond | H | Et | Me | Cl | OMe | O |
| D-10005 | H | single bond | Me | *c*-Pr | Me | Cl | OMe | O |
| D-10006 | Me | single bond | H | *c*-Pr | Me | Cl | OMe | O |
| D-10007 | Me(R) | single bond | H | *c*-Pr | Me | Cl | OMe | O |
| D-10008 | Me(S) | single bond | H | *c*-Pr | Me | Cl | OMe | O |
| D-10009 | H | single bond | Me | *c*-Pr(1-CN) | Me | Cl | OMe | O |
| D-10010 | Me | single bond | H | *c*-Pr(1-CN) | Me | Cl | OMe | O |
| D-10011 | Me(R) | single bond | H | *c*-Pr(1-CN) | Me | Cl | OMe | O |
| D-10012 | Me(S) | single bond | H | *c*-Pr(1-CN) | Me | Cl | OMe | O |
| D-10013 | H | single bond | Me | CH₂CF₃ | Me | Me | OMe | O |
| D-10014 | Me | single bond | H | CH₂CF₃ | Me | Me | OMe | O |
| D-10015 | Me(R) | single bond | H | CH₂CF₃ | Me | Me | OMe | O |
| D-10016 | Me(S) | single bond | H | CH₂CF₃ | Me | Me | OMe | O |
| D-10017 | H | single bond | Me | Et | Me | Me | OMe | O |
| D-10018 | Me | single bond | H | Et | Me | Me | OMe | O |
| D-10019 | Me(R) | single bond | H | Et | Me | Me | OMe | O |
| D-10020 | Me(S) | single bond | H | Et | Me | Me | OMe | O |
| D-10021 | H | single bond | Me | *c*-Pr | Me | Me | OMe | O |
| D-10022 | Me | single bond | H | *c*-Pr | Me | Me | OMe | O |

**[Table 874]**

| Compound Number | Y | W | Z | R¹ | R² | R³ | R¹⁰ | A |
|---|---|---|---|---|---|---|---|---|
| D-10023 | Me(R) | single bond | H | *c*-Pr | Me | Me | OMe | O |
| D-10024 | Me(S) | single bond | H | *c*-Pr | Me | Me | OMe | O |
| D-10025 | H | single bond | Me | *c*-Pr(1-CN) | Me | Me | OMe | O |
| D-10026 | Me | single bond | H | *c*-Pr(1-CN) | Me | Me | OMe | O |
| D-10027 | Me(R) | single bond | H | *c*-Pr(1-CN) | Me | Me | OMe | O |
| D-10028 | Me(S) | single bond | H | *c*-Pr(1-CN) | Me | Me | OMe | O |
| D-10029 | H | single bond | Me | CH₂CF₃ | Me | Cl | C(=O)(*i*-Pr) | O |
| D-10030 | Me | single bond | H | CH₂CF₃ | Me | Cl | C(=O)(*i*-Pr) | O |
| D-10031 | Me(R) | single bond | H | CH₂CF₃ | Me | Cl | C(=O)(*i*-Pr) | O |
| D-10032 | Me(S) | single bond | H | CH₂CF₃ | Me | Cl | C(=O)(*i*-Pr) | O |
| D-10033 | H | single bond | Me | Et | Me | Cl | C(=O)(*i*-Pr) | O |
| D-10034 | Me | single bond | H | Et | Me | Cl | C(=O)(*i*-Pr) | O |
| D-10035 | Me(R) | single bond | H | Et | Me | Cl | C(=O)(*i*-Pr) | O |
| D-10036 | Me(S) | single bond | H | Et | Me | Cl | C(=O)(*i*-Pr) | O |
| D-10037 | H | single bond | Me | *c*-Pr | Me | Cl | C(=O)(*i*-Pr) | O |
| D-10038 | Me | single bond | H | *c*-Pr | Me | Cl | C(=O)(*i*-Pr) | O |
| D-10039 | Me(R) | single bond | H | *c*-Pr | Me | Cl | C(=O)(*i*-Pr) | O |
| D-10040 | Me(S) | single bond | H | *c*-Pr | Me | Cl | C(=O)(*i*-Pr) | O |
| D-10041 | H | single bond | Me | *c*-Pr(1-CN) | Me | Cl | C(=O)(*i*-Pr) | O |
| D-10042 | Me | single bond | H | *c*-Pr(1-CN) | Me | Cl | C(=O)(*i*-Pr) | O |
| D-10043 | Me(R) | single bond | H | *c*-Pr(1-CN) | Me | Cl | C(=O)(*i*-Pr) | O |
| D-10044 | Me(S) | single bond | H | *c*-Pr(1-CN) | Me | Cl | C(=O)(*i*-Pr) | O |
| D-10045 | H | single bond | Me | CH₂CF₃ | Me | Me | C(=O)(*i*-Pr) | O |
| D-10046 | Me | single bond | H | CH₂CF₃ | Me | Me | C(=O)(*i*-Pr) | O |
| D-10047 | Me(R) | single bond | H | CH₂CF₃ | Me | Me | C(=O)(*i*-Pr) | O |
| D-10048 | Me(S) | single bond | H | CH₂CF₃ | Me | Me | C(=O)(*i*-Pr) | O |
| D-10049 | H | single bond | Me | Et | Me | Me | C(=O)(*i*-Pr) | O |
| D-10050 | Me | single bond | H | Et | Me | Me | C(=O)(*i*-Pr) | O |
| D-10051 | Me(R) | single bond | H | Et | Me | Me | C(=O)(*i*-Pr) | O |
| D-10052 | Me(S) | single bond | H | Et | Me | Me | C(=O)(*i*-Pr) | O |
| D-10053 | H | single bond | Me | *c*-Pr | Me | Me | C(=O)(*i*-Pr) | O |
| D-10054 | Me | single bond | H | *c*-Pr | Me | Me | C(=O)(*i*-Pr) | O |
| D-10055 | Me(R) | single bond | H | *c*-Pr | Me | Me | C(=O)(*i*-Pr) | O |
| D-10056 | Me(S) | single bond | H | *c*-Pr | Me | Me | C(=O)(*i*-Pr) | O |
| D-10057 | H | single bond | Me | *c*-Pr(1-CN) | Me | Me | C(=O)(*i*-Pr) | O |
| D-10058 | Me | single bond | H | *c*-Pr(1-CN) | Me | Me | C(=O)(*i*-Pr) | O |
| D-10059 | Me(R) | single bond | H | *c*-Pr(1-CN) | Me | Me | C(=O)(*i*-Pr) | O |
| D-10060 | Me(S) | single bond | H | *c*-Pr(1-CN) | Me | Me | C(=O)(*i*-Pr) | O |
| D-10061 | H | single bond | Me | CH₂CF₃ | Me | Cl | C(=O)O(*t*-Bu) | O |
| D-10062 | Me | single bond | H | CH₂CF₃ | Me | Cl | C(=O)O(*t*-Bu) | O |
| D-10063 | Me(R) | single bond | H | CH₂CF₃ | Me | Cl | C(=O)O(*t*-Bu) | O |
| D-10064 | Me(S) | single bond | H | CH₂CF₃ | Me | Cl | C(=O)O(*t*-Bu) | O |
| D-10065 | H | single bond | Me | Et | Me | Cl | C(=O)O(*t*-Bu) | O |
| D-10066 | Me | single bond | H | Et | Me | Cl | C(=O)O(*t*-Bu) | O |

**[Table 875]**

| Compound Number | Y | W | Z | ¹ | R² | R³ | R¹⁰ | A |
|---|---|---|---|---|---|---|---|---|
| D-10067 | Me(R) | single bond | H | Et | Me | Cl | C(=O)O(*t*-Bu) | O |
| D-10068 | Me(S) | single bond | H | Et | Me | Cl | C(=O)O(*t*-Bu) | O |
| D-10069 | H | single bond | Me | *c*-Pr | Me | Cl | C(=O)O(*t*-Bu) | O |
| D-10070 | Me | single bond | H | *c*-Pr | Me | Cl | C(=O)O(*t*-Bu) | O |
| D-10071 | Me(R) | single bond | H | *c*-Pr | Me | Cl | C(=O)O(*t*-Bu) | O |
| D-10072 | Me(S) | single bond | H | *c*-Pr | Me | Cl | C(=O)O(*t*-Bu) | O |
| D-10073 | H | single bond | Me | *c*-Pr(1-CN) | Me | Cl | C(=O)O(*t*-Bu) | O |
| D-10074 | Me | single bond | H | *c*-Pr(1-CN) | Me | Cl | C(=O)O(*t*-Bu) | O |
| D-10075 | Me(R) | single bond | H | *c*-Pr(1-CN) | Me | Cl | C(=O)O(*t*-Bu) | O |
| D-10076 | Me(S) | single bond | H | *c*-Pr(1-CN) | Me | Cl | C(=O)O(*t*-Bu) | O |
| D-10077 | H | single bond | Me | CH₂CF₃ | Me | Me | C(=O)O(*t*-Bu) | O |
| D-10078 | Me | single bond | H | CH₂CF₃ | Me | Me | C(=O)O(*t*-Bu) | O |
| D-10079 | Me(R) | single bond | H | CH₂CF₃ | Me | Me | C(=O)O(*t*-Bu) | O |
| D-10080 | Me(S) | single bond | H | CH₂CF₃ | Me | Me | C(=O)O(*t*-Bu) | O |
| D-10081 | H | single bond | Me | Et | Me | Me | C(=O)O(*t*-Bu) | O |
| D-10082 | Me | single bond | H | Et | Me | Me | C(=O)O(*t*-Bu) | O |
| D-10083 | Me(R) | single bond | H | Et | Me | Me | C(=O)O(*t*-Bu) | O |
| D-10084 | Me(S) | single bond | H | Et | Me | Me | C(=O)O(*t*-Bu) | O |
| D-10085 | H | single bond | Me | *c*-Pr | Me | Me | C(=O)O(*t*-Bu) | O |
| D-10086 | Me | single bond | H | *c*-Pr | Me | Me | C(=O)O(*t*-Bu) | O |
| D-10087 | Me(R) | single bond | H | *c*-Pr | Me | Me | C(=O)O(*t*-Bu) | O |
| D-10088 | Me(S) | single bond | H | *c*-Pr | Me | Me | C(=O)O(*t*-Bu) | O |
| D-10089 | H | single bond | Me | *c*-Pr(1-CN) | Me | Me | C(=O)O(*t*-Bu) | O |
| D-10090 | Me | single bond | H | *c*-Pr(1-CN) | Me | Me | C(=O)O(*t*-Bu) | O |
| D-10091 | Me(R) | single bond | H | *c*-Pr(1-CN) | Me | Me | C(=O)O(*t*-Bu) | O |
| D-10092 | Me(S) | single bond | H | *c*-Pr(1-CN) | Me | Me | C(=O)O(*t*-Bu) | O |
| D-10093 | H | single bond | Me | CH₂CF₃ | Me | Cl | CH₂OEt | O |
| D-10094 | Me | single bond | H | CH₂CF₃ | Me | Cl | CH₂OEt | O |
| D-10095 | Me(R) | single bond | H | CH₂CF₃ | Me | Cl | CH₂OEt | O |
| D-10096 | Me(S) | single bond | H | CH₂CF₃ | Me | Cl | CH₂OEt | O |
| D-10097 | H | single bond | Me | Et | Me | Cl | CH₂OEt | O |
| D-10098 | Me | single bond | H | Et | Me | Cl | CH₂OEt | O |
| D-10099 | Me(R) | single bond | H | Et | Me | Cl | CH₂OEt | O |
| D-10100 | Me(S) | single bond | H | Et | Me | Cl | CH₂OEt | O |
| D-10101 | H | single bond | Me | *c*-Pr | Me | Cl | CH₂OEt | O |
| D-10102 | Me | single bond | H | *c*-Pr | Me | Cl | CH₂OEt | O |
| D-10103 | Me(R) | single bond | H | *c*-Pr | Me | Cl | CH₂OEt | O |
| D-10104 | Me(S) | single bond | H | *c*-Pr | Me | Cl | CH₂OEt | O |
| D-10105 | H | single bond | Me | *c*-Pr(1-CN) | Me | Cl | CH₂OEt | O |
| D-10106 | Me | single bond | H | *c*-Pr(1-CN) | Me | Cl | CH₂OEt | O |
| D-10107 | Me(R) | single bond | H | *c*-Pr(1-CN) | Me | Cl | CH₂OEt | O |
| D-10108 | Me(S) | single bond | H | *c*-Pr(1-CN) | Me | Cl | CH₂OEt | O |
| D-10109 | H | single bond | Me | CH₂CF₃ | Me | Me | CH₂OEt | O |
| D-10110 | Me | single bond | H | CH₂CF₃ | Me | Me | CH₂OEt | O |

**[Table 876]**

| Compound Number | Y | W | Z | R¹ | R² | R³ | R¹⁰ | A |
|---|---|---|---|---|---|---|---|---|
| D-10111 | Me(R) | single bond | H | CH₂CF₃ | Me | Me | CH₂OEt | O |
| D-10112 | Me(S) | single bond | H | CH₂CF₃ | Me | Me | CH₂OEt | O |
| D-10113 | H | single bond | Me | Et | Me | Me | CH₂OEt | O |
| D-10114 | Me | single bond | H | Et | Me | Me | CH₂OEt | O |
| D-10115 | Me(R) | single bond | H | Et | Me | Me | CH₂OEt | O |
| D-10116 | Me(S) | single bond | H | Et | Me | Me | CH₂OEt | O |
| D-10117 | H | single bond | Me | *c*-Pr | Me | Me | CH₂OEt | O |
| D-10118 | Me | single bond | H | *c*-Pr | Me | Me | CH₂OEt | O |
| D-10119 | Me(R) | single bond | H | *c*-Pr | Me | Me | CH₂OEt | O |
| D-10120 | Me(S) | single bond | H | *c*-Pr | Me | Me | CH₂OEt | O |
| D-10121 | H | single bond | Me | *c*-Pr(1-CN) | Me | Me | CH₂OEt | O |
| D-10122 | Me | single bond | H | *c*-Pr(1-CN) | Me | Me | CH₂OEt | O |
| D-10123 | Me(R) | single bond | H | *c*-Pr(1-CN) | Me | Me | CH₂OEt | O |
| D-10124 | Me(S) | single bond | H | *c*-Pr(1-CN) | Me | Me | CH₂OEt | O |
| D-10125 | H | single bond | Me | CH₂CF₃ | Me | Cl | CH₂SMe | O |
| D-10126 | Me | single bond | H | CH₂CF₃ | Me | Cl | CH₂SMe | O |
| D-10127 | Me(R) | single bond | H | CH₂CF₃ | Me | Cl | CH₂SMe | O |
| D-10128 | Me(S) | single bond | H | CH₂CF₃ | Me | Cl | CH₂SMe | O |
| D-10129 | H | single bond | Me | Et | Me | Cl | CH₂SMe | O |
| D-10130 | Me | single bond | H | Et | Me | Cl | CH₂SMe | O |
| D-10131 | Me(R) | single bond | H | Et | Me | Cl | CH₂SMe | O |
| D-10132 | Me(S) | single bond | H | Et | Me | Cl | CH₂SMe | O |
| D-10133 | H | single bond | Me | *c*-Pr | Me | Cl | CH₂SMe | O |
| D-10134 | Me | single bond | H | *c*-Pr | Me | Cl | CH₂SMe | O |
| D-10135 | Me(R) | single bond | H | *c*-Pr | Me | Cl | CH₂SMe | O |
| D-10136 | Me(S) | single bond | H | *c*-Pr | Me | Cl | CH₂SMe | O |
| D-10137 | H | single bond | Me | *c*-Pr(1-CN) | Me | Cl | CH₂SMe | O |
| D-10138 | Me | single bond | H | *c*-Pr(1-CN) | Me | Cl | CH₂SMe | O |
| D-10139 | Me(R) | single bond | H | *c*-Pr(1-CN) | Me | Cl | CH₂SMe | O |
| D-10140 | Me(S) | single bond | H | *c*-Pr(1-CN) | Me | Cl | CH₂SMe | O |
| D-10141 | H | single bond | Me | CH₂CF₃ | Me | Me | CH₂SMe | O |
| D-10142 | Me | single bond | H | CH₂CF₃ | Me | Me | CH₂SMe | O |
| D-10143 | Me(R) | single bond | H | CH₂CF₃ | Me | Me | CH₂SMe | O |
| D-10144 | Me(S) | single bond | H | CH₂CF₃ | Me | Me | CH₂SMe | O |
| D-10145 | H | single bond | Me | Et | Me | Me | CH₂SMe | O |
| D-10146 | Me | single bond | H | Et | Me | Me | CH₂SMe | O |
| D-10147 | Me(R) | single bond | H | Et | Me | Me | CH₂SMe | O |
| D-10148 | Me(S) | single bond | H | Et | Me | Me | CH₂SMe | O |
| D-10149 | H | single bond | Me | *c*-Pr | Me | Me | CH₂SMe | O |
| D-10150 | Me | single bond | H | *c*-Pr | Me | Me | CH₂SMe | O |
| D-10151 | Me(R) | single bond | H | *c*-Pr | Me | Me | CH₂SMe | O |
| D-10152 | Me(S) | single bond | H | *c*-Pr | Me | Me | CH₂SMe | O |
| D-10153 | H | single bond | Me | *c*-Pr(1-CN) | Me | Me | CH₂SMe | O |
| D-10154 | Me | single bond | H | *c*-Pr(1-CN) | Me | Me | CH₂SMe | O |

**[Table 877]**

| Compound Number | Y | W | Z | R¹ | R² | R³ | R¹⁰ | A |
|---|---|---|---|---|---|---|---|---|
| D-10155 | Me(R) | single bond | H | *c*-Pr(1-CN) | Me | Me | CH₂SMe | O |
| D-10156 | Me(S) | single bond | H | *c*-Pr(1-CN) | Me | Me | CH₂SMe | O |
| D-10157 | H | single bond | Me | CH₂CF₃ | Me | Cl | CH₂S(=O)₂Me | O |
| D-10158 | Me | single bond | H | CH₂CF₃ | Me | Cl | CH₂S(=O)₂Me | O |
| D-10159 | Me(R) | single bond | H | CH₂CF₃ | Me | Cl | CH₂S(=O)₂Me | O |
| D-10160 | Me(S) | single bond | H | CH₂CF₃ | Me | Cl | CH₂S(=O)₂Me | O |
| D-10161 | H | single bond | Me | Et | Me | Cl | CH₂S(=O)₂Me | O |
| D-10162 | Me | single bond | H | Et | Me | Cl | CH₂S(=O)₂Me | O |
| D-10163 | Me(R) | single bond | H | Et | Me | Cl | CH₂S(=O)₂Me | O |
| D-10164 | Me(S) | single bond | H | Et | Me | Cl | CH₂S(=O)₂Me | O |
| D-10165 | H | single bond | Me | *c*-Pr | Me | Cl | CH₂S(=O)₂Me | O |
| D-10166 | Me | single bond | H | *c*-Pr | Me | Cl | CH₂S(=O)₂Me | O |
| D-10167 | Me(R) | single bond | H | *c*-Pr | Me | Cl | CH₂S(=O)₂Me | O |
| D-10168 | Me(S) | single bond | H | *c*-Pr | Me | Cl | CH₂S(=O)₂Me | O |
| D-10169 | H | single bond | Me | *c*-Pr(1-CN) | Me | Cl | CH₂S(=O)₂Me | O |
| D-10170 | Me | single bond | H | *c*-Pr(1-CN) | Me | Cl | CH₂S(=O)₂Me | O |
| D-10171 | Me(R) | single bond | H | *c*-Pr(1-CN) | Me | Cl | CH₂S(=O)₂Me | O |
| D-10172 | Me(S) | single bond | H | *c*-Pr(1-CN) | Me | Cl | CH₂S(=O)₂Me | O |
| D-10173 | H | single bond | Me | CH₂CF₃ | Me | Me | CH₂S(=O)₂Me | O |
| D-10174 | Me | single bond | H | CH₂CF₃ | Me | Me | CH₂S(=O)₂Me | O |
| D-10175 | Me(R) | single bond | H | CH₂CF₃ | Me | Me | CH₂S(=O)₂Me | O |
| D-10176 | Me(S) | single bond | H | CH₂CF₃ | Me | Me | CH₂S(=O)₂Me | O |
| D-10177 | H | single bond | Me | Et | Me | Me | CH₂S(=O)₂Me | O |
| D-10178 | Me | single bond | H | Et | Me | Me | CH₂S(=O)₂Me | O |
| D-10179 | Me(R) | single bond | H | Et | Me | Me | CH₂S(=O)₂Me | O |
| D-10180 | Me(S) | single bond | H | Et | Me | Me | CH₂S(=O)₂Me | O |
| D-10181 | H | single bond | Me | *c*-Pr | Me | Me | CH₂S(=O)₂Me | O |
| D-10182 | Me | single bond | H | *c*-Pr | Me | Me | CH₂S(=O)₂Me | O |
| D-10183 | Me(R) | single bond | H | *c*-Pr | Me | Me | CH₂S(=O)₂Me | O |
| D-10184 | Me(S) | single bond | H | *c*-Pr | Me | Me | CH₂S(=O)₂Me | O |
| D-10185 | H | single bond | Me | *c*-Pr(1-CN) | Me | Me | CH₂S(=O)₂Me | O |
| D-10186 | Me | single bond | H | *c*-Pr(1-CN) | Me | Me | CH₂S(=O)₂Me | O |
| D-10187 | Me(R) | single bond | H | *c*-Pr(1-CN) | Me | Me | CH₂S(=O)₂Me | O |
| D-10188 | Me(S) | single bond | H | *c*-Pr(1-CN) | Me | Me | CH₂S(=O)₂Me | O |
| D-10189 | H | single bond | Me | CH₂CF₃ | Me | Cl | CH₂C(=O)Me | O |
| D-10190 | Me | single bond | H | CH₂CF₃ | Me | Cl | CH₂C(=O)Me | O |
| D-10191 | Me(R) | single bond | H | CH₂CF₃ | Me | Cl | CH₂C(=O)Me | O |
| D-10192 | Me(S) | single bond | H | CH₂CF₃ | Me | Cl | CH₂C(=O)Me | O |
| D-10193 | H | single bond | Me | Et | Me | Cl | CH₂C(=O)Me | O |
| D-10194 | Me | single bond | H | Et | Me | Cl | CH₂C(=O)Me | O |
| D-10195 | Me(R) | single bond | H | Et | Me | Cl | CH₂C(=O)Me | O |
| D-10196 | Me(S) | single bond | H | Et | Me | Cl | CH₂C(=O)Me | O |
| D-10197 | H | single bond | Me | *c*-Pr | Me | Cl | CH₂C(=O)Me | O |
| D-10198 | Me | single bond | H | *c*-Pr | Me | Cl | CH₂C(=O)Me | O |

**[Table 878]**

| Compound Number | Y | W | Z | R¹ | R² | R³ | R¹⁰ | A |
|---|---|---|---|---|---|---|---|---|
| D-10199 | Me(R) | single bond | H | *c*-Pr | Me | Cl | CH₂C(=O)Me | O |
| D-10200 | Me(S) | single bond | H | *c*-Pr | Me | Cl | CH₂C(=O)Me | O |
| D-10201 | H | single bond | Me | *c*-Pr(1-CN) | Me | Cl | CH₂C(=O)Me | O |
| D-10202 | Me | single bond | H | *c*-Pr(1-CN) | Me | Cl | CH₂C(=O)Me | O |
| D-10203 | Me(R) | single bond | H | *c*-Pr(1-CN) | Me | Cl | CH₂C(=O)Me | O |
| D-10204 | Me(S) | single bond | H | *c*-Pr(1-CN) | Me | Cl | CH₂C(=O)Me | O |
| D-10205 | H | single bond | Me | CH₂CF₃ | Me | Me | CH₂C(=O)Me | O |
| D-10206 | Me | single bond | H | CH₂CF₃ | Me | Me | CH₂C(=O)Me | O |
| D-10207 | Me(R) | single bond | H | CH₂CF₃ | Me | Me | CH₂C(=O)Me | O |
| D-10208 | Me(S) | single bond | H | CH₂CF₃ | Me | Me | CH₂C(=O)Me | O |
| D-10209 | H | single bond | Me | Et | Me | Me | CH₂C(=O)Me | O |
| D-10210 | Me | single bond | H | Et | Me | Me | CH₂C(=O)Me | O |
| D-10211 | Me(R) | single bond | H | Et | Me | Me | CH₂C(=O)Me | O |
| D-10212 | Me(S) | single bond | H | Et | Me | Me | CH₂C(=O)Me | O |
| D-10213 | H | single bond | Me | *c*-Pr | Me | Me | CH₂C(=O)Me | O |
| D-10214 | Me | single bond | H | *c*-Pr | Me | Me | CH₂C(=O)Me | O |
| D-10215 | Me(R) | single bond | H | *c*-Pr | Me | Me | CH₂C(=O)Me | O |
| D-10216 | Me(S) | single bond | H | *c*-Pr | Me | Me | CH₂C(=O)Me | O |
| D-10217 | H | single bond | Me | *c*-Pr(1-CN) | Me | Me | CH₂C(=O)Me | O |
| D-10218 | Me | single bond | H | *c*-Pr(1-CN) | Me | Me | CH₂C(=O)Me | O |
| D-10219 | Me(R) | single bond | H | *c*-Pr(1-CN) | Me | Me | CH₂C(=O)Me | O |
| D-10220 | Me(S) | single bond | H | *c*-Pr(1-CN) | Me | Me | CH₂C(=O)Me | O |
| D-10221 | H | single bond | Me | CH₂CF₃ | Me | Cl | CH₂C(=O)OMe | O |
| D-10222 | Me | single bond | H | CH₂CF₃ | Me | Cl | CH₂C(=O)OMe | O |
| D-10223 | Me(R) | single bond | H | CH₂CF₃ | Me | Cl | CH₂C(=O)OMe | O |
| D-10224 | Me(S) | single bond | H | CH₂CF₃ | Me | Cl | CH₂C(=O)OMe | O |
| D-10225 | H | single bond | Me | Et | Me | Cl | CH₂C(=O)OMe | O |
| D-10226 | Me | single bond | H | Et | Me | Cl | CH₂C(=O)OMe | O |
| D-10227 | Me(R) | single bond | H | Et | Me | Cl | CH₂C(=O)OMe | O |
| D-10228 | Me(S) | single bond | H | Et | Me | Cl | CH₂C(=O)OMe | O |
| D-10229 | H | single bond | Me | *c*-Pr | Me | Cl | CH₂C(=O)OMe | O |
| D-10230 | Me | single bond | H | *c*-Pr | Me | Cl | CH₂C(=O)OMe | O |
| D-10231 | Me(R) | single bond | H | *c*-Pr | Me | Cl | CH₂C(=O)OMe | O |
| D-10232 | Me(S) | single bond | H | *c*-Pr | Me | Cl | CH₂C(=O)OMe | O |
| D-10233 | H | single bond | Me | *c*-Pr(1-CN) | Me | Cl | CH₂C(=O)OMe | O |
| D-10234 | Me | single bond | H | *c*-Pr(1-CN) | Me | Cl | CH₂C(=O)OMe | O |
| D-10235 | Me(R) | single bond | H | *c*-Pr(1-CN) | Me | Cl | CH₂C(=O)OMe | O |
| D-10236 | Me(S) | single bond | H | *c*-Pr(1-CN) | Me | Cl | CH₂C(=O)OMe | O |
| D-10237 | H | single bond | Me | CH₂CF₃ | Me | Me | CH₂C(=O)OMe | O |
| D-10238 | Me | single bond | H | CH₂CF₃ | Me | Me | CH₂C(=O)OMe | O |
| D-10239 | Me(R) | single bond | H | CH₂CF₃ | Me | Me | CH₂C(=O)OMe | O |
| D-10240 | Me(S) | single bond | H | CH₂CF₃ | Me | Me | CH₂C(=O)OMe | O |
| D-10241 | H | single bond | Me | Et | Me | Me | CH₂C(=O)OMe | O |
| D-10242 | Me | single bond | H | Et | Me | Me | CH₂C(=O)OMe | O |

**[Table 879]**

| Compound Number | Y | W | Z | R¹ | R² | R³ | R¹⁰ | A |
|---|---|---|---|---|---|---|---|---|
| D-10243 | Me(R) | single bond | H | Et | Me | Me | CH₂C(=O)OMe | O |
| D-10244 | Me(S) | single bond | H | Et | Me | Me | CH₂C(=O)OMe | O |
| D-10245 | H | single bond | Me | *c*-Pr | Me | Me | CH₂C(=O)OMe | O |
| D-10246 | Me | single bond | H | *c*-Pr | Me | Me | CH₂C(=O)OMe | O |
| D-10247 | Me(R) | single bond | H | *c*-Pr | Me | Me | CH₂C(=O)OMe | O |
| D-10248 | Me(S) | single bond | H | *c*-Pr | Me | Me | CH₂C(=O)OMe | O |
| D-10249 | H | single bond | Me | *c*-Pr(1-CN) | Me | Me | CH₂C(=O)OMe | O |
| D-10250 | Me | single bond | H | *c*-Pr(1-CN) | Me | Me | CH₂C(=O)OMe | O |
| D-10251 | Me(R) | single bond | H | *c*-Pr(1-CN) | Me | Me | CH₂C(=O)OMe | O |
| D-10252 | Me(S) | single bond | H | *c*-Pr(1-CN) | Me | Me | CH₂C(=O)OMe | O |
| D-10253 | H | single bond | Me | CH₂CF₃ | Me | Cl | CH₂C(=O)NHMe | O |
| D-10254 | Me | single bond | H | CH₂CF₃ | Me | Cl | CH₂C(=O)NHMe | O |
| D-10255 | Me(R) | single bond | H | CH₂CF₃ | Me | Cl | CH₂C(=O)NHMe | O |
| D-10256 | Me(S) | single bond | H | CH₂CF₃ | Me | Cl | CH₂C(=O)NHMe | O |
| D-10257 | H | single bond | Me | Et | Me | Cl | CH₂C(=O)NHMe | O |
| D-10258 | Me | single bond | H | Et | Me | Cl | CH₂C(=O)NHMe | O |
| D-10259 | Me(R) | single bond | H | Et | Me | Cl | CH₂C(=O)NHMe | O |
| D-10260 | Me(S) | single bond | H | Et | Me | Cl | CH₂C(=O)NHMe | O |
| D-10261 | H | single bond | Me | *c*-Pr | Me | Cl | CH₂C(=O)NHMe | O |
| D-10262 | Me | single bond | H | *c*-Pr | Me | Cl | CH₂C(=O)NHMe | O |
| D-10263 | Me(R) | single bond | H | *c*-Pr | Me | Cl | CH₂C(=O)NHMe | O |
| D-10264 | Me(S) | single bond | H | *c*-Pr | Me | Cl | CH₂C(=O)NHMe | O |
| D-10265 | H | single bond | Me | *c*-Pr(1-CN) | Me | Cl | CH₂C(=O)NHMe | O |
| D-10266 | Me | single bond | H | *c*-Pr(1-CN) | Me | Cl | CH₂C(=O)NHMe | O |
| D-10267 | Me(R) | single bond | H | *c*-Pr(1-CN) | Me | Cl | CH₂C(=O)NHMe | O |
| D-10268 | Me(S) | single bond | H | *c*-Pr(1-CN) | Me | Cl | CH₂C(=O)NHMe | O |
| D-10269 | H | single bond | Me | CH₂CF₃ | Me | Me | CH₂C(=O)NHMe | O |
| D-10270 | Me | single bond | H | CH₂CF₃ | Me | Me | CH₂C(=O)NHMe | O |
| D-10271 | Me(R) | single bond | H | CH₂CF₃ | Me | Me | CH₂C(=O)NHMe | O |
| D-10272 | Me(S) | single bond | H | CH₂CF₃ | Me | Me | CH₂C(=O)NHMe | O |
| D-10273 | H | single bond | Me | Et | Me | Me | CH₂C(=O)NHMe | O |
| D-10274 | Me | single bond | H | Et | Me | Me | CH₂C(=O)NHMe | O |
| D-10275 | Me(R) | single bond | H | Et | Me | Me | CH₂C(=O)NHMe | O |
| D-10276 | Me(S) | single bond | H | Et | Me | Me | CH₂C(=O)NHMe | O |
| D-10277 | H | single bond | Me | *c*-Pr | Me | Me | CH₂C(=O)NHMe | O |
| D-10278 | Me | single bond | H | *c*-Pr | Me | Me | CH₂C(=O)NHMe | O |
| D-10279 | Me(R) | single bond | H | *c*-Pr | Me | Me | CH₂C(=O)NHMe | O |
| D-10280 | Me(S) | single bond | H | *c*-Pr | Me | Me | CH₂C(=O)NHMe | O |
| D-10281 | H | single bond | Me | *c*-Pr(1-CN) | Me | Me | CH₂C(=O)NHMe | O |
| D-10282 | Me | single bond | H | *c*-Pr(1-CN) | Me | Me | CH₂C(=O)NHMe | O |
| D-10283 | Me(R) | single bond | H | *c*-Pr(1-CN) | Me | Me | CH₂C(=O)NHMe | O |
| D-10284 | Me(S) | single bond | H | *c*-Pr(1-CN) | Me | Me | CH₂C(=O)NHMe | O |
| D-10285 | H | single bond | Me | CH₂CF₃ | Me | Cl | CH₂OC(=O)Me | O |
| D-10286 | Me | single bond | H | CH₂CF₃ | Me | Cl | CH₂OC(=O)Me | O |

**[Table 880]**

| Compound Number | Y | W | Z | R¹ | R² | R³ | R¹⁰ | A |
|---|---|---|---|---|---|---|---|---|
| D-10287 | Me(R) | single bond | H | CH₂CF₃ | Me | Cl | CH₂OC(=O)Me | O |
| D-10288 | Me(S) | single bond | H | CH₂CF₃ | Me | Cl | CH₂OC(=O)Me | O |
| D-10289 | H | single bond | Me | Et | Me | Cl | CH₂OC(=O)Me | O |
| D-10290 | Me | single bond | H | Et | Me | Cl | CH₂OC(=O)Me | O |
| D-10291 | Me(R) | single bond | H | Et | Me | Cl | CH₂OC(=O)Me | O |
| D-10292 | Me(S) | single bond | H | Et | Me | Cl | CH₂OC(=O)Me | O |
| D-10293 | H | single bond | Me | *c*-Pr | Me | Cl | CH₂OC(=O)Me | O |
| D-10294 | Me | single bond | H | *c*-Pr | Me | Cl | CH₂OC(=O)Me | O |
| D-10295 | Me(R) | single bond | H | *c*-Pr | Me | Cl | CH₂OC(=O)Me | O |
| D-10296 | Me(S) | single bond | H | *c*-Pr | Me | Cl | CH₂OC(=O)Me | O |
| D-10297 | H | single bond | Me | *c*-Pr(1-CN) | Me | Cl | CH₂OC(=O)Me | O |
| D-10298 | Me | single bond | H | *c*-Pr(1-CN) | Me | Cl | CH₂OC(=O)Me | O |
| D-10299 | Me(R) | single bond | H | *c*-Pr(1-CN) | Me | Cl | CH₂OC(=O)Me | O |
| D-10300 | Me(S) | single bond | H | *c*-Pr(1-CN) | Me | Cl | CH₂OC(=O)Me | O |
| D-10301 | H | single bond | Me | CH₂CF₃ | Me | Me | CH₂OC(=O)Me | O |
| D-10302 | Me | single bond | H | CH₂CF₃ | Me | Me | CH₂OC(=O)Me | O |
| D-10303 | Me(R) | single bond | H | CH₂CF₃ | Me | Me | CH₂OC(=O)Me | O |
| D-10304 | Me(S) | single bond | H | CH₂CF₃ | Me | Me | CH₂OC(=O)Me | O |
| D-10305 | H | single bond | Me | Et | Me | Me | CH₂OC(=O)Me | O |
| D-10306 | Me | single bond | H | Et | Me | Me | CH₂OC(=O)Me | O |
| D-10307 | Me(R) | single bond | H | Et | Me | Me | CH₂OC(=O)Me | O |
| D-10308 | Me(S) | single bond | H | Et | Me | Me | CH₂OC(=O)Me | O |
| D-10309 | H | single bond | Me | *c*-Pr | Me | Me | CH₂OC(=O)Me | O |
| D-10310 | Me | single bond | H | *c*-Pr | Me | Me | CH₂OC(=O)Me | O |
| D-10311 | Me(R) | single bond | H | *c*-Pr | Me | Me | CH₂OC(=O)Me | O |
| D-10312 | Me(S) | single bond | H | *c*-Pr | Me | Me | CH₂OC(=O)Me | O |
| D-10313 | H | single bond | Me | *c*-Pr(1-CN) | Me | Me | CH₂OC(=O)Me | O |
| D-10314 | Me | single bond | H | *c*-Pr(1-CN) | Me | Me | CH₂OC(=O)Me | O |
| D-10315 | Me(R) | single bond | H | *c*-Pr(1-CN) | Me | Me | CH₂OC(=O)Me | O |
| D-10316 | Me(S) | single bond | H | *c*-Pr(1-CN) | Me | Me | CH₂OC(=O)Me | O |
| D-10317 | H | single bond | Me | CH₂CF₃ | Me | Cl | CH₂OC(=O)(*i*-Pr) | O |
| D-10318 | Me | single bond | H | CH₂CF₃ | Me | Cl | CH₂OC(=O)(*i* -Pr) | O |
| D-10319 | Me(R) | single bond | H | CH₂CF₃ | Me | Cl | CH₂OC(=O)(i -Pr) | O |
| D-10320 | Me(S) | single bond | H | CH₂CF₃ | Me | Cl | CH₂OC(=O)(*i*-Pr) | O |
| D-10321 | H | single bond | Me | Et | Me | Cl | CH₂OC(=O)(*i*-Pr) | O |
| D-10322 | Me | single bond | H | Et | Me | Cl | CH₂OC(=O)(*i*-Pr) | O |
| D-10323 | Me(R) | single bond | H | Et | Me | Cl | CH₂OC(=O)(*i*-Pr) | O |
| D-10324 | Me(S) | single bond | H | Et | Me | Cl | CH₂OC(=O)(*i*-Pr) | O |
| D-10325 | H | single bond | Me | *c*-Pr | Me | Cl | CH₂OC(=O)(*i*-Pr) | O |
| D-10326 | Me | single bond | H | *c*-Pr | Me | Cl | CH₂OC(=O)(*i*-Pr) | O |
| D-10327 | Me(R) | single bond | H | *c*-Pr | Me | Cl | CH₂OC(=O)(*i*-Pr) | O |
| D-10328 | Me(S) | single bond | H | *c*-Pr | Me | Cl | CH₂OC(=O)(*i*-Pr) | O |
| D-10329 | H | single bond | Me | *c*-Pr(1-CN) | Me | Cl | CH₂OC(=O)(*i*-Pr) | O |
| D-10330 | Me | single bond | H | *c*-Pr(1-CN) | Me | Cl | CH₂OC(=O)(*i*-Pr) | O |

**[Table 881]**

| Compound Number | Y | W | Z | R¹ | R² | R³ | R¹⁰ | A |
|---|---|---|---|---|---|---|---|---|
| D-10331 | Me(R) | single bond | H | *c*-Pr(1-CN) | Me | Cl | CH₂OC(=O)(*i*-Pr) | O |
| D-10332 | Me(S) | single bond | H | *c*-Pr(1-CN) | Me | Cl | CH₂OC(=O)(*i*-Pr) | O |
| D-10333 | H | single bond | Me | CH₂CF₃ | Me | Me | CH₂OC(=O)(*i*-Pr) | O |
| D-10334 | Me | single bond | H | CH₂CF₃ | Me | Me | CH₂OC(=O)(*i*-Pr) | O |
| D-10335 | Me(R) | single bond | H | CH₂CF₃ | Me | Me | CH₂OC(=O)(*i*-Pr) | O |
| D-10336 | Me(S) | single bond | H | CH₂CF₃ | Me | Me | CH₂OC(=O)(*i*-Pr) | O |
| D-10337 | H | single bond | Me | Et | Me | Me | CH₂OC(=O)(*i*-Pr) | O |
| D-10338 | Me | single bond | H | Et | Me | Me | CH₂OC(=O)(*i*-Pr) | O |
| D-10339 | Me(R) | single bond | H | Et | Me | Me | CH₂OC(=O)(*i*-Pr) | O |
| D-10340 | Me(S) | single bond | H | Et | Me | Me | CH₂OC(=O)(*i*-Pr) | O |
| D-10341 | H | single bond | Me | *c*-Pr | Me | Me | CH₂OC(=O)(*i*-Pr) | O |
| D-10342 | Me | single bond | H | *c*-Pr | Me | Me | CH₂OC(=O)(*i-*Pr) | O |
| D-10343 | Me(R) | single bond | H | *c*-Pr | Me | Me | CH₂OC(=O)(*i*-Pr) | O |
| D-10344 | Me(S) | single bond | H | *c*-Pr | Me | Me | CH₂OC(=O)(*i*-Pr) | O |
| D-10345 | H | single bond | Me | *c*-Pr(1-CN) | Me | Me | CH₂OC(=O)(*i*-Pr) | O |
| D-10346 | Me | single bond | H | *c*-Pr(1-CN) | Me | Me | CH₂OC(=O)(*i*-Pr) | O |
| D-10347 | Me(R) | single bond | H | *c*-Pr(1-CN) | Me | Me | CH₂OC(=O)(*i*-Pr) | O |
| D-10348 | Me(S) | single bond | H | *c*-Pr(1-CN) | Me | Me | CH₂OC(=O)(*i*-Pr) | O |
| D-10349 | H | single bond | Me | CH₂CF₃ | Me | Cl | CH₂OC(=O)(*t*-Bu) | O |
| D-10350 | Me | single bond | H | CH₂CF₃ | Me | Cl | CH₂OC(=O)(*t*-Bu) | O |
| D-10351 | Me(R) | single bond | H | CH₂CF₃ | Me | Cl | CH₂OC(=O)(*t*-Bu) | O |
| D-10352 | Me(S) | single bond | H | CH₂CF₃ | Me | Cl | CH₂OC(=O)(*t*-Bu) | O |
| D-10353 | H | single bond | Me | Et | Me | Cl | CH₂OC(=O)(*t*-Bu) | O |
| D-10354 | Me | single bond | H | Et | Me | Cl | CH₂OC(=O)(*t*-Bu) | O |
| D-10355 | Me(R) | single bond | H | Et | Me | Cl | CH₂OC(=O)(*t*-Bu) | O |
| D-10356 | Me(S) | single bond | H | Et | Me | Cl | CH₂OC(=O)(*t*-Bu) | O |
| D-10357 | H | single bond | Me | *c*-Pr | Me | Cl | CH₂OC(=O)(*t*-Bu) | O |
| D-10358 | Me | single bond | H | *c*-Pr | Me | Cl | CH₂OC(=O)(*t*-Bu) | O |
| D-10359 | Me(R) | single bond | H | *c*-Pr | Me | Cl | CH₂OC(=O)(*t*-Bu) | O |
| D-10360 | Me(S) | single bond | H | *c*-Pr | Me | Cl | CH₂OC(=O)(*t*-Bu) | O |
| D-10361 | H | single bond | Me | *c*-Pr(1-CN) | Me | Cl | CH₂OC(=O)(*t*-Bu) | O |
| D-10362 | Me | single bond | H | *c*-Pr(1-CN) | Me | Cl | CH₂OC(=O)(*t*-Bu) | O |
| D-10363 | Me(R) | single bond | H | *c*-Pr(1-CN) | Me | Cl | CH₂OC(=O)(*t*-Bu) | O |
| D-10364 | Me(S) | single bond | H | *c*-Pr(1-CN) | Me | Cl | CH₂OC(=O)(*t*-Bu) | O |
| D-10365 | H | single bond | Me | CH₂CF₃ | Me | Me | CH₂OC(=O)(*t*-Bu) | O |
| D-10366 | Me | single bond | H | CH₂CF₃ | Me | Me | CH₂OC(=O)(*t*-Bu) | O |
| D-10367 | Me(R) | single bond | H | CH₂CF₃ | Me | Me | CH₂OC(=O)(*t*-Bu) | O |
| D-10368 | Me(S) | single bond | H | CH₂CF₃ | Me | Me | CH₂OC(=O)(*t*-Bu) | O |
| D-10369 | H | single bond | Me | Et | Me | Me | CH₂OC(=O)(*t*-Bu) | O |
| D-10370 | Me | single bond | H | Et | Me | Me | CH₂OC(=O)(*t*-Bu) | O |
| D-10371 | Me(R) | single bond | H | Et | Me | Me | CH₂OC(=O)(*t*-Bu) | O |
| D-10372 | Me(S) | single bond | H | Et | Me | Me | CH₂OC(=O)(*t*-Bu) | O |
| D-10373 | H | single bond | Me | *c*-Pr | Me | Me | CH₂OC(=O)(*t*-Bu) | O |
| D-10374 | Me | single bond | H | *c*-Pr | Me | Me | CH₂OC(=O)(*t*-Bu) | O |

**[Table 882]**

| Compound Number | Y | W | Z | R¹ | R² | R³ | R¹⁰ | A |
|---|---|---|---|---|---|---|---|---|
| D-10375 | Me(R) | single bond | H | *c*-Pr | Me | Me | CH₂OC(=O)(*t*-Bu) | O |
| D-10376 | Me(S) | single bond | H | *c*-Pr | Me | Me | CH₂OC(=O)(*t*-Bu) | O |
| D-10377 | H | single bond | Me | *c*-Pr(1-CN) | Me | Me | CH₂OC(=O)(*t*-Bu) | O |
| D-10378 | Me | single bond | H | *c*-Pr(1-CN) | Me | Me | CH₂OC(=O)(*t*-Bu) | O |
| D-10379 | Me(R) | single bond | H | *c*-Pr(1-CN) | Me | Me | CH₂OC(=O)(*t*-Bu) | O |
| D-10380 | Me(S) | single bond | H | *c*-Pr(1-CN) | Me | Me | CH₂OC(=O)(*t*-Bu) | O |
| D-10381 | H | single bond | Me | CH₂CF₃ | Me | Cl | CH₂OC(=O)OEt | O |
| D-10382 | Me | single bond | H | CH₂CF₃ | Me | Cl | CH₂OC(=O)OEt | O |
| D-10383 | Me(R) | single bond | H | CH₂CF₃ | Me | Cl | CH₂OC(=O)OEt | O |
| D-10384 | Me(S) | single bond | H | CH₂CF₃ | Me | Cl | CH₂OC(=O)OEt | O |
| D-10385 | H | single bond | Me | Et | Me | Cl | CH₂OC(=O)OEt | O |
| D-10386 | Me | single bond | H | Et | Me | Cl | CH₂OC(=O)OEt | O |
| D-10387 | Me(R) | single bond | H | Et | Me | Cl | CH₂OC(=O)OEt | O |
| D-10388 | Me(S) | single bond | H | Et | Me | Cl | CH₂OC(=O)OEt | O |
| D-10389 | H | single bond | Me | *c*-Pr | Me | Cl | CH₂OC(=O)OEt | O |
| D-10390 | Me | single bond | H | *c*-Pr | Me | Cl | CH₂OC(=O)OEt | O |
| D-10391 | Me(R) | single bond | H | *c*-Pr | Me | Cl | CH₂OC(=O)OEt | O |
| D-10392 | Me(S) | single bond | H | *c*-Pr | Me | Cl | CH₂OC(=O)OEt | O |
| D-10393 | H | single bond | Me | *c*-Pr(1-CN) | Me | Cl | CH₂OC(=O)OEt | O |
| D-10394 | Me | single bond | H | *c*-Pr(1-CN) | Me | Cl | CH₂OC(=O)OEt | O |
| D-10395 | Me(R) | single bond | H | *c*-Pr(1-CN) | Me | Cl | CH₂OC(=O)OEt | O |
| D-10396 | Me(S) | single bond | H | *c*-Pr(1-CN) | Me | Cl | CH₂OC(=O)OEt | O |
| D-10397 | H | single bond | Me | CH₂CF₃ | Me | Me | CH₂OC(=O)OEt | O |
| D-10398 | Me | single bond | H | CH₂CF₃ | Me | Me | CH₂OC(=O)OEt | O |
| D-10399 | Me(R) | single bond | H | CH₂CF₃ | Me | Me | CH₂OC(=O)OEt | O |
| D-10400 | Me(S) | single bond | H | CH₂CF₃ | Me | Me | CH₂OC(=O)OEt | O |
| D-10401 | H | single bond | Me | Et | Me | Me | CH₂OC(=O)OEt | O |
| D-10402 | Me | single bond | H | Et | Me | Me | CH₂OC(=O)OEt | O |
| D-10403 | Me(R) | single bond | H | Et | Me | Me | CH₂OC(=O)OEt | O |
| D-10404 | Me(S) | single bond | H | Et | Me | Me | CH₂OC(=O)OEt | O |
| D-10405 | H | single bond | Me | *c*-Pr | Me | Me | CH₂OC(=O)OEt | O |
| D-10406 | Me | single bond | H | *c*-Pr | Me | Me | CH₂OC(=O)OEt | O |
| D-10407 | Me(R) | single bond | H | *c*-Pr | Me | Me | CH₂OC(=O)OEt | O |
| D-10408 | Me(S) | single bond | H | *c*-Pr | Me | Me | CH₂OC(=O)OEt | O |
| D-10409 | H | single bond | Me | *c*-Pr(1-CN) | Me | Me | CH₂OC(=O)OEt | O |
| D-10410 | Me | single bond | H | *c*-Pr(1-CN) | Me | Me | CH₂OC(=O)OEt | O |
| D-10411 | Me(R) | single bond | H | *c*-Pr(1-CN) | Me | Me | CH₂OC(=O)OEt | O |
| D-10412 | Me(S) | single bond | H | *c*-Pr(1-CN) | Me | Me | CH₂OC(=O)OEt | O |
| D-10413 | H | single bond | Me | CH₂CF₃ | Me | Cl | CH₂OC(=O)O(*i*-Pr) | O |
| D-10414 | Me | single bond | H | CH₂CF₃ | Me | Cl | CH₂OC(=O)O(*i*-Pr) | O |
| D-10415 | Me(R) | single bond | H | CH₂CF₃ | Me | Cl | CH₂OC(=O)O(*i*-Pr) | O |
| D-10416 | Me(S) | single bond | H | CH₂CF₃ | Me | Cl | CH₂OC(=O)O(*i*-Pr) | O |
| D-10417 | H | single bond | Me | Et | Me | Cl | CH₂OC(=O)O(*i*-Pr) | O |
| D-10418 | Me | single bond | H | Et | Me | Cl | CH₂OC(=O)O(*i*-Pr) | O |

**[Table 883]**

| Compound Number | Y | W | Z | R¹ | R² | R³ | R¹⁰ | A |
|---|---|---|---|---|---|---|---|---|
| D-10419 | Me(R) | single bond | H | Et | Me | Cl | CH₂OC(=O)O(*i*-Pr) | O |
| D-10420 | Me(S) | single bond | H | Et | Me | Cl | CH₂OC(=O)O(*i*-Pr) | O |
| D-10421 | H | single bond | Me | *c*-Pr | Me | Cl | CH₂OC(=O)O(*i*-Pr) | O |
| D-10422 | Me | single bond | H | *c*-Pr | Me | Cl | CH₂OC(=O)O(*i*-Pr) | O |
| D-10423 | Me(R) | single bond | H | *c*-Pr | Me | Cl | CH₂OC(=O)O(*i*-Pr) | O |
| D-10424 | Me(S) | single bond | H | *c*-Pr | Me | Cl | CH₂OC(=O)O(*i*-Pr) | O |
| D-10425 | H | single bond | Me | *c*-Pr(1-CN) | Me | Cl | CH₂OC(=O)O(*i*-Pr) | O |
| D-10426 | Me | single bond | H | *c*-Pr(1-CN) | Me | Cl | CH₂OC(=O)O(*i*-Pr) | O |
| D-10427 | Me(R) | single bond | H | *c*-Pr(1-CN) | Me | Cl | CH₂OC(=O)O(*i*-Pr) | O |
| D-10428 | Me(S) | single bond | H | *c*-Pr(1-CN) | Me | Cl | CH₂OC(=O)O(*i*-Pr) | O |
| D-10429 | H | single bond | Me | CH₂CF₃ | Me | Me | CH₂OC(=O)O(*i*-Pr) | O |
| D-10430 | Me | single bond | H | CH₂CF₃ | Me | Me | CH₂OC(=O)O(*i*-Pr) | O |
| D-10431 | Me(R) | single bond | H | CH₂CF₃ | Me | Me | CH₂OC(=O)O(*i*-Pr) | O |
| D-10432 | Me(S) | single bond | H | CH₂CF₃ | Me | Me | CH₂OC(=O)O(*i*-Pr) | O |
| D-10433 | H | single bond | Me | Et | Me | Me | CH₂OC(=O)O(*i*-Pr) | O |
| D-10434 | Me | single bond | H | Et | Me | Me | CH₂OC(=O)O(*i*-Pr) | O |
| D-10435 | Me(R) | single bond | H | Et | Me | Me | CH₂OC(=O)O(*i*-Pr) | O |
| D-10436 | Me(S) | single bond | H | Et | Me | Me | CH₂OC(=O)O(*i*-Pr) | O |
| D-10437 | H | single bond | Me | *c*-Pr | Me | Me | CH₂OC(=O)O(*i*-Pr) | O |
| D-10438 | Me | single bond | H | *c*-Pr | Me | Me | CH₂OC(=O)O(*i*-Pr) | O |
| D-10439 | Me(R) | single bond | H | *c*-Pr | Me | Me | CH₂OC(=O)O(*i*-Pr) | O |
| D-10440 | Me(S) | single bond | H | *c*-Pr | Me | Me | CH₂OC(=O)O(*i*-Pr) | O |
| D-10441 | H | single bond | Me | *c*-Pr(1-CN) | Me | Me | CH₂OC(=O)O(*i*-Pr) | O |
| D-10442 | Me | single bond | H | *c*-Pr(1-CN) | Me | Me | CH₂OC(=O)O(*i*-Pr) | O |
| D-10443 | Me(R) | single bond | H | *c*-Pr(1-CN) | Me | Me | CH₂OC(=O)O(*i*-Pr) | O |
| D-10444 | Me(S) | single bond | H | *c*-Pr(1-CN) | Me | Me | CH₂OC(=O)O(*i*-Pr) | O |
| D-10445 | H | single bond | Me | CH₂CF₃ | Me | Cl | CH₂N(Me)C(=O)OMe | O |
| D-10446 | Me | single bond | H | CH₂CF₃ | Me | Cl | CH₂N(Me)C(=O)OMe | O |
| D-10447 | Me(R) | single bond | H | CH₂CF₃ | Me | Cl | CH₂N(Me)C(=O)OMe | O |
| D-10448 | Me(S) | single bond | H | CH₂CF₃ | Me | Cl | CH₂N(Me)C(=O)OMe | O |
| D-10449 | H | single bond | Me | Et | Me | Cl | CH₂N(Me)C(=O)OMe | O |
| D-10450 | Me | single bond | H | Et | Me | Cl | CH₂N(Me)C(=O)OMe | O |
| D-10451 | Me(R) | single bond | H | Et | Me | Cl | CH₂N(Me)C(=O)OMe | O |
| D-10452 | Me(S) | single bond | H | Et | Me | Cl | CH₂N(Me)C(=O)OMe | O |
| D-10453 | H | single bond | Me | *c*-Pr | Me | Cl | CH₂N(Me)C(=O)OMe | O |
| D-10454 | Me | single bond | H | *c*-Pr | Me | Cl | CH₂N(Me)C(=O)OMe | O |
| D-10455 | Me(R) | single bond | H | *c*-Pr | Me | Cl | CH₂N(Me)C(=O)OMe | O |
| D-10456 | Me(S) | single bond | H | *c*-Pr | Me | Cl | CH₂N(Me)C(=O)OMe | O |
| D-10457 | H | single bond | Me | *c*-Pr(1-CN) | Me | Cl | CH₂N(Me)C(=O)OMe | O |
| D-10458 | Me | single bond | H | *c*-Pr(1-CN) | Me | Cl | CH₂N(Me)C(=O)OMe | O |
| D-10459 | Me(R) | single bond | H | *c*-Pr(1-CN) | Me | Cl | CH₂N(Me)C(=O)OMe | O |
| D-10460 | Me(S) | single bond | H | *c*-Pr(1-CN) | Me | Cl | CH₂N(Me)C(=O)OMe | O |
| D-10461 | H | single bond | Me | CH₂CF₃ | Me | Me | CH₂N(Me)C(=O)OMe | O |
| D-10462 | Me | single bond | H | CH₂CF₃ | Me | Me | CH₂N(Me)C(=O)OMe | O |

**[Table 884]**

| Compound Number | Y | W | Z | R¹ | R² | R³ | R¹⁰ | A |
|---|---|---|---|---|---|---|---|---|
| D-10463 | Me(R) | single bond | H | CH₂CF₃ | Me | Me | CH₂N(Me)C(=O)OMe | O |
| D-10464 | Me(S) | single bond | H | CH₂CF₃ | Me | Me | CH₂N(Me)C(=O)OMe | O |
| D-10465 | H | single bond | Me | Et | Me | Me | CH₂N(Me)C(=O)OMe | O |
| D-10466 | Me | single bond | H | Et | Me | Me | CH₂N(Me)C(=O)OMe | O |
| D-10467 | Me(R) | single bond | H | Et | Me | Me | CH₂N(Me)C(=O)OMe | O |
| D-10468 | Me(S) | single bond | H | Et | Me | Me | CH₂N(Me)C(=O)OMe | O |
| D-10469 | H | single bond | Me | *c*-Pr | Me | Me | CH₂N(Me)C(=O)OMe | O |
| D-10470 | Me | single bond | H | *c*-Pr | Me | Me | CH₂N(Me)C(=O)OMe | O |
| D-10471 | Me(R) | single bond | H | *c*-Pr | Me | Me | CH₂N(Me)C(=O)OMe | O |
| D-10472 | Me(S) | single bond | H | *c*-Pr | Me | Me | CH₂N(Me)C(=O)OMe | O |
| D-10473 | H | single bond | Me | *c*-Pr(1-CN) | Me | Me | CH₂N(Me)C(=O)OMe | O |
| D-10474 | Me | single bond | H | *c*-Pr(1-CN) | Me | Me | CH₂N(Me)C(=O)OMe | O |
| D-10475 | Me(R) | single bond | H | *c*-Pr(1-CN) | Me | Me | CH₂N(Me)C(=O)OMe | O |
| D-10476 | Me(S) | single bond | H | *c*-Pr(1-CN) | Me | Me | CH₂N(Me)C(=O)OMe | O |
| D-10477 | H | single bond | Me | CH₂CF₃ | Me | Cl | CH₂OCH₂CH₂OMe | O |
| D-10478 | Me | single bond | H | CH₂CF₃ | Me | Cl | CH₂OCH₂CH₂OMe | O |
| D-10479 | Me(R) | single bond | H | CH₂CF₃ | Me | Cl | CH₂OCH₂CH₂OMe | O |
| D-10480 | Me(S) | single bond | H | CH₂CF₃ | Me | Cl | CH₂OCH₂CH₂OMe | O |
| D-10481 | H | single bond | Me | Et | Me | Cl | CH₂OCH₂CH₂OMe | O |
| D-10482 | Me | single bond | H | Et | Me | Cl | CH₂OCH₂CH₂OMe | O |
| D-10483 | Me(R) | single bond | H | Et | Me | Cl | CH₂OCH₂CH₂OMe | O |
| D-10484 | Me(S) | single bond | H | Et | Me | Cl | CH₂OCH₂CH₂OMe | O |
| D-10485 | H | single bond | Me | *c*-Pr | Me | Cl | CH₂OCH₂CH₂OMe | O |
| D-10486 | Me | single bond | H | *c*-Pr | Me | Cl | CH₂OCH₂CH₂OMe | O |
| D-10487 | Me(R) | single bond | H | *c*-Pr | Me | Cl | CH₂OCH₂CH₂OMe | O |
| D-10488 | Me(S) | single bond | H | *c*-Pr | Me | Cl | CH₂OCH₂CH₂OMe | O |
| D-10489 | H | single bond | Me | *c*-Pr(1-CN) | Me | Cl | CH₂OCH₂CH₂OMe | O |
| D-10490 | Me | single bond | H | *c*-Pr(1-CN) | Me | Cl | CH₂OCH₂CH₂OMe | O |
| D-10491 | Me(R) | single bond | H | *c*-Pr(1-CN) | Me | Cl | CH₂OCH₂CH₂OMe | O |
| D-10492 | Me(S) | single bond | H | *c*-Pr(1-CN) | Me | Cl | CH₂OCH₂CH₂OMe | O |
| D-10493 | H | single bond | Me | CH₂CF₃ | Me | Me | CH₂OCH₂CH₂OMe | O |
| D-10494 | Me | single bond | H | CH₂CF₃ | Me | Me | CH₂OCH₂CH₂OMe | O |
| D-10495 | Me(R) | single bond | H | CH₂CF₃ | Me | Me | CH₂OCH₂CH₂OMe | O |
| D-10496 | Me(S) | single bond | H | CH₂CF₃ | Me | Me | CH₂OCH₂CH₂OMe | O |
| D-10497 | H | single bond | Me | Et | Me | Me | CH₂OCH₂CH₂OMe | O |
| D-10498 | Me | single bond | H | Et | Me | Me | CH₂OCH₂CH₂OMe | O |
| D-10499 | Me(R) | single bond | H | Et | Me | Me | CH₂OCH₂CH₂OMe | O |
| D-10500 | Me(S) | single bond | H | Et | Me | Me | CH₂OCH₂CH₂OMe | O |
| D-10501 | H | single bond | Me | *c*-Pr | Me | Me | CH₂OCH₂CH₂OMe | O |
| D-10502 | Me | single bond | H | *c*-Pr | Me | Me | CH₂OCH₂CH₂OMe | O |
| D-10503 | Me(R) | single bond | H | *c*-Pr | Me | Me | CH₂OCH₂CH₂OMe | O |
| D-10504 | Me(S) | single bond | H | *c*-Pr | Me | Me | CH₂OCH₂CH₂OMe | O |
| D-10505 | H | single bond | Me | *c*-Pr(1-CN) | Me | Me | CH₂OCH₂CH₂OMe | O |
| D-10506 | Me | single bond | H | *c*-Pr(1-CN) | Me | Me | CH₂OCH₂CH₂OMe | O |

**[Table 885]**

| Compound Number | Y | W | Z | R¹ | R² | R³ | R¹⁰ | A |
|---|---|---|---|---|---|---|---|---|
| D-10507 | Me(R) | single bond | H | *c*-Pr(1-CN) | Me | Me | CH₂OCH₂CH₂OMe | O |
| D-10508 | Me(S) | single bond | H | *c*-Pr(1-CN) | Me | Me | CH₂OCH₂CH₂OMe | O |
| D-10509 | H | single bond | Me | CH₂CF₃ | Me | Cl | CH₂OCH₂CH₂CN | O |
| D-10510 | Me | single bond | H | CH₂CF₃ | Me | Cl | CH₂OCH₂CH₂CN | O |
| D-10511 | Me(R) | single bond | H | CH₂CF₃ | Me | Cl | CH₂OCH₂CH₂CN | O |
| D-10512 | Me(S) | single bond | H | CH₂CF₃ | Me | Cl | CH₂OCH₂CH₂CN | O |
| D-10513 | H | single bond | Me | Et | Me | Cl | CH₂OCH₂CH₂CN | O |
| D-10514 | Me | single bond | H | Et | Me | Cl | CH₂OCH₂CH₂CN | O |
| D-10515 | Me(R) | single bond | H | Et | Me | Cl | CH₂OCH₂CH₂CN | O |
| D-10516 | Me(S) | single bond | H | Et | Me | Cl | CH₂OCH₂CH₂CN | O |
| D-10517 | H | single bond | Me | *c*-Pr | Me | Cl | CH₂OCH₂CH₂CN | O |
| D-10518 | Me | single bond | H | *c*-Pr | Me | Cl | CH₂OCH₂CH₂CN | O |
| D-10519 | Me(R) | single bond | H | *c*-Pr | Me | Cl | CH₂OCH₂CH₂CN | O |
| D-10520 | Me(S) | single bond | H | *c*-Pr | Me | Cl | CH₂OCH₂CH₂CN | O |
| D-10521 | H | single bond | Me | *c*-Pr(1-CN) | Me | Cl | CH₂OCH₂CH₂CN | O |
| D-10522 | Me | single bond | H | *c*-Pr(1-CN) | Me | Cl | CH₂OCH₂CH₂CN | O |
| D-10523 | Me(R) | single bond | H | *c*-Pr(1-CN) | Me | Cl | CH₂OCH₂CH₂CN | O |
| D-10524 | Me(S) | single bond | H | *c*-Pr(1-CN) | Me | Cl | CH₂OCH₂CH₂CN | O |
| D-10525 | H | single bond | Me | CH₂CF₃ | Me | Me | CH₂OCH₂CH₂CN | O |
| D-10526 | Me | single bond | H | CH₂CF₃ | Me | Me | CH₂OCH₂CH₂CN | O |
| D-10527 | Me(R) | single bond | H | CH₂CF₃ | Me | Me | CH₂OCH₂CH₂CN | O |
| D-10528 | Me(S) | single bond | H | CH₂CF₃ | Me | Me | CH₂OCH₂CH₂CN | O |
| 0-10529 | H | single bond | Me | Et | Me | Me | CH₂OCH₂CH₂CN | O |
| D-10530 | Me | single bond | H | Et | Me | Me | CH₂OCH₂CH₂CN | O |
| D-10531 | Me(R) | single bond | H | Et | Me | Me | CH₂OCH₂CH₂CN | O |
| D-10532 | Me(S) | single bond | H | Et | Me | Me | CH₂OCH₂CH₂CN | O |
| D-10533 | H | single bond | Me | *c*-Pr | Me | Me | CH₂OCH₂CH₂CN | O |
| D-10534 | Me | single bond | H | *c*-Pr | Me | Me | CH₂OCH₂CH₂CN | O |
| D-10535 | Me(R) | single bond | H | *c*-Pr | Me | Me | CH₂OCH₂CH₂CN | O |
| D-10536 | Me(S) | single bond | H | *c*-Pr | Me | Me | CH₂OCH₂CH₂CN | O |
| D-10537 | H | single bond | Me | *c*-Pr(1-CN) | Me | Me | CH₂OCH₂CH₂CN | O |
| D-10538 | Me | single bond | H | *c*-Pr(1-CN) | Me | Me | CH₂OCH₂CH₂CN | O |
| D-10539 | Me(R) | single bond | H | *c*-Pr(1-CN) | Me | Me | CH₂OCH₂CH₂CN | O |
| D-10540 | Me(S) | single bond | H | *c*-Pr(1-CN) | Me | Me | CH₂OCH₂CH₂CN | O |
| D-10541 | H | single bond | Me | CH₂CF₃ | Me | Cl | CH₂OCH₂CH₂S(=O)₂Me | O |
| D-10542 | Me | single bond | H | CH₂CF₃ | Me | Cl | CH₂OCH₂CH₂S(=O)₂Me | O |
| D-10543 | Me(R) | single bond | H | CH₂CF₃ | Me | Cl | CH₂OCH₂CH₂S(=O)₂Me | O |
| D-10544 | Me(S) | single bond | H | CH₂CF₃ | Me | Cl | CH₂OCH₂CH₂S(=O)₂Me | O |
| D-10545 | H | single bond | Me | Et | Me | Cl | CH₂OCH₂CH₂S(=O)₂Me | O |
| D-10546 | Me | single bond | H | Et | Me | Cl | CH₂OCH₂CH₂S(=O)₂Me | O |
| D-10547 | Me(R) | single bond | H | Et | Me | Cl | CH₂OCH₂CH₂S(=O)₂Me | O |
| D-10548 | Me(S) | single bond | H | Et | Me | Cl | CH₂OCH₂CH₂S(=O)₂Me | O |
| D-10549 | H | single bond | Me | *c*-Pr | Me | Cl | CH₂OCH₂CH₂S(=O)₂Me | O |
| D-10550 | Me | single bond | H | *c*-Pr | Me | Cl | CH₂OCH₂CH₂S(=O)₂Me | O |

**[Table 886]**

| Compound Number | Y | W | Z | R¹ | R² | R³ | R¹⁰ | A |
|---|---|---|---|---|---|---|---|---|
| D-10551 | Me(R) | single bond | H | *c*-Pr | Me | Cl | CH₂OCH₂CH₂S(=O)₂Me | O |
| D-10552 | Me(S) | single bond | H | *c*-Pr | Me | Cl | CH₂OCH₂CH₂S(=O)₂Me | O |
| D-10553 | H | single bond | Me | *c*-Pr(1-CN) | Me | Cl | CH₂OCH₂CH₂S(=O)₂Me | O |
| D-10554 | Me | single bond | H | *c*-Pr(1-CN) | Me | Cl | CH₂OCH₂CH₂S(=O)₂Me | O |
| D-10555 | Me(R) | single bond | H | *c*-Pr(1-CN) | Me | Cl | CH₂OCH₂CH₂S(=O)₂Me | O |
| D-10556 | Me(S) | single bond | H | *c*-Pr(1-CN) | Me | Cl | CH₂OCH₂CH₂S(=O)₂Me | O |
| D-10557 | H | single bond | Me | CH₂CF₃ | Me | Me | CH₂OCH₂CH₂S(=O)₂Me | O |
| D-10558 | Me | single bond | H | CH₂CF₃ | Me | Me | CH₂OCH₂CH₂S(=O)₂Me | O |
| D-10559 | Me(R) | single bond | H | CH₂CF₃ | Me | Me | CH₂OCH₂CH₂S(=O)₂Me | O |
| D-10560 | Me(S) | single bond | H | CH₂CF₃ | Me | Me | CH₂OCH₂CH₂S(=O)₂Me | O |
| D-10561 | H | single bond | Me | Et | Me | Me | CH₂OCH₂CH₂S(=O)₂Me | O |
| D-10562 | Me | single bond | H | Et | Me | Me | CH₂OCH₂CH₂S(=O)₂Me | O |
| D-10563 | Me(R) | single bond | H | Et | Me | Me | CH₂OCH₂CH₂S(=O)₂Me | O |
| D-10564 | Me(S) | single bond | H | Et | Me | Me | CH₂OCH₂CH₂S(=O)₂Me | O |
| D-10565 | H | single bond | Me | *c*-Pr | Me | Me | CH₂OCH₂CH₂S(=O)₂Me | O |
| D-10566 | Me | single bond | H | *c*-Pr | Me | Me | CH₂OCH₂CH₂S(=O)₂Me | O |
| D-10567 | Me(R) | single bond | H | *c*-Pr | Me | Me | CH₂OCH₂CH₂S(=O)₂Me | O |
| D-10568 | Me(S) | single bond | H | *c*-Pr | Me | Me | CH₂OCH₂CH₂S(=O)₂Me | O |
| D-10569 | H | single bond | Me | *c*-Pr(1-CN) | Me | Me | CH₂OCH₂CH₂S(=O)₂Me | O |
| D-10570 | Me | single bond | H | *c*-Pr(1-CN) | Me | Me | CH₂OCH₂CH₂S(=O)₂Me | O |
| D-10571 | Me(R) | single bond | H | *c*-Pr(1-CN) | Me | Me | CH₂OCH₂CH₂S(=O)₂Me | O |
| D-10572 | Me(S) | single bond | H | *c*-Pr(1-CN) | Me | Me | CH₂OCH₂CH₂S(=O)₂Me | O |
| D-10573 | H | single bond | Me | CH₂CF₃ | Me | Cl | CH₂(5-Me-1,3-dioxol-2-one-4-yl) | O |
| D-10574 | Me | single bond | H | CH₂CF₃ | Me | Cl | CH₂(5-Me-1,3-dioxol-2-one-4-yl) | O |
| D-10575 | Me(R) | single bond | H | CH₂CF₃ | Me | Cl | CH₂(5-Me-1,3-dioxol-2-one-4-yl) | O |
| D-10576 | Me(S) | single bond | H | CH₂CF₃ | Me | Cl | CH₂(5-Me-1,3-dioxol-2-one-4-yl) | O |
| D-10577 | H | single bond | Me | Et | Me | Cl | CH₂(5-Me-1,3-dioxol-2-one-4-yl) | O |
| D-10578 | Me | single bond | H | Et | Me | Cl | CH₂(5-Me-1,3-dioxol-2-one-4-yl) | O |
| D-10579 | Me(R) | single bond | H | Et | Me | Cl | CH₂(5-Me-1,3-dioxol-2-one-4-yl) | O |
| D-10580 | Me(S) | single bond | H | Et | Me | Cl | CH₂(5-Me-1,3-dioxol-2-one-4-yl) | O |
| D-10581 | H | single bond | Me | *c*-Pr | Me | Cl | CH₂(5-Me-1,3-dioxol-2-one-4-yl) | O |
| D-10582 | Me | single bond | H | *c*-Pr | Me | Cl | CH₂(5-Me-1,3-dioxol-2-one-4-yl) | O |
| D-10583 | Me(R) | single bond | H | *c*-Pr | Me | Cl | CH₂(5-Me-1,3-dioxol-2-one-4-yl) | O |
| D-10584 | Me(S) | single bond | H | *c*-Pr | Me | Cl | CH₂(5-Me-1,3-dioxol-2-one-4-yl) | O |
| D-10585 | H | single bond | Me | *c*-Pr(1-CN) | Me | Cl | CH₂(5-Me-1,3-dioxol-2-one-4-yl) | O |
| D-10586 | Me | single bond | H | *c*-Pr(1-CN) | Me | Cl | CH₂(5-Me-1,3-dioxol-2-one-4-yl) | O |
| D-10587 | Me(R) | single bond | H | *c*-Pr(1-CN) | Me | Cl | CH₂(5-Me-1,3-dioxol-2-one-4-yl) | O |
| D-10588 | Me(S) | single bond | H | *c*-Pr(1-CN) | Me | Cl | CH₂(5-Me-1,3-dioxol-2-one-4-yl) | O |
| D-10589 | H | single bond | Me | CH₂CF₃ | Me | Me | CH₂(5-Me-1,3-dioxol-2-one-4-yl) | O |
| D-10590 | Me | single bond | H | CH₂CF₃ | Me | Me | CH₂(5-Me-1,3-dioxol-2-one-4-yl) | O |
| D-10591 | Me(R) | single bond | H | CH₂CF₃ | Me | Me | CH₂(5-Me-1,3-dioxol-2-one-4-yl) | O |
| D-10592 | Me(S) | single bond | H | CH₂CF₃ | Me | Me | CH₂(5-Me-1,3-dioxol-2-one-4-yl) | O |
| D-10593 | H | single bond | Me | Et | Me | Me | CH₂(5-Me-1,3-dioxol-2-one-4-yl) | O |
| D-10594 | Me | single bond | H | Et | Me | Me | CH₂(5-Me-1,3-dioxol-2-one-4-yl) | O |

**[Table 887]**

| Compound Number | Y | W | Z | R¹ | R² | R³ | R¹⁰ | A |
|---|---|---|---|---|---|---|---|---|
| D-10595 | Me(R) | single bond | H | Et | Me | Me | CH₂(5-Me-1,3-dioxol-2-one-4-yl) | O |
| D-10596 | Me(S) | single bond | H | Et | Me | Me | CH₂(5-Me-1,3-dioxol-2-one-4-yl) | O |
| D-10597 | H | single bond | Me | *c-*Pr | Me | Me | CH₂(5-Me-1,3-dioxol-2-one-4-yl) | O |
| D-10598 | Me | single bond | H | *c*-Pr | Me | Me | CH₂(5-Me-1,3-dioxol-2-one-4-yl) | O |
| D-10599 | Me(R) | single bond | H | *c*-Pr | Me | Me | CH₂(5-Me-1,3-dioxol-2-one-4-yl) | O |
| D-10600 | Me(S) | single bond | H | *c*-Pr | Me | Me | CH₂(5-Me-1,3-dioxol-2-one-4-yl) | O |
| D-10601 | H | single bond | Me | *c*-Pr(1-CN) | Me | Me | CH₂(5-Me-1,3-dioxol-2-one-4-yl) | O |
| D-10602 | Me | single bond | H | *c*-Pr(1-CN) | Me | Me | CH₂(5-Me-1,3-dioxol-2-one-4-yl) | O |
| D-10603 | Me(R) | single bond | H | *c*-Pr(1-CN) | Me | Me | CH₂(5-Me-1,3-dioxol-2-one-4-yl) | O |
| D-10604 | Me(S) | single bond | H | *c*-Pr(1-CN) | Me | Me | CH₂(5-Me-1,3-dioxol-2-one-4-yl) | O |
| D-10605 | H | single bond | Me | CH₂CF₃ | C(=O)Me | Cl | *c*-Pr | O |
| D-10606 | Me | single bond | H | CH₂CF₃ | C(=O)Me | Cl | *c*-Pr | O |
| D-10607 | Me(R) | single bond | H | CH₂CF₃ | C(=O)Me | Cl | *c*-Pr | O |
| D-10608 | Me(S) | single bond | H | CH₂CF₃ | C(=O)Me | Cl | *c*-Pr | O |
| D-10609 | H | single bond | Me | Et | C(=O)Me | Cl | *c*-Pr | O |
| D-10610 | Me | single bond | H | Et | C(=O)Me | Cl | *c*-Pr | O |
| D-10611 | Me(R) | single bond | H | Et | C(=O)Me | Cl | *c*-Pr | O |
| D-10612 | Me(S) | single bond | H | Et | C(=O)Me | Cl | *c*-Pr | O |
| D-10613 | H | single bond | Me | *c*-Pr | C(=O)Me | Cl | *c*-Pr | O |
| D-10614 | Me | single bond | H | *c*-Pr | C(=O)Me | Cl | *c*-Pr | O |
| D-10615 | Me(R) | single bond | H | *c*-Pr | C(=O)Me | Cl | *c*-Pr | O |
| D-10616 | Me(S) | single bond | H | *c*-Pr | C(=O)Me | Cl | *c*-Pr | O |
| D-10617 | H | single bond | Me | *c*-Pr(1-CN) | C(=O)Me | Cl | *c*-Pr | O |
| D-10618 | Me | single bond | H | *c*-Pr(1-CN) | C(=O)Me | Cl | *c*-Pr | O |
| D-10619 | Me(R) | single bond | H | *c*-Pr(1-CN) | C(=O)Me | Cl | *c*-Pr | O |
| D-10620 | Me(S) | single bond | H | *c*-Pr(1-CN) | C(=O)Me | Cl | *c*-Pr | O |
| D-10621 | H | single bond | Me | CH₂CF₃ | C(=O)Me | Me | *c*-Pr | O |
| D-10622 | Me | single bond | H | CH₂CF₃ | C(=O)Me | Me | *c*-Pr | O |
| D-10623 | Me(R) | single bond | H | CH₂CF₃ | C(=O)Me | Me | *c*-Pr | O |
| D-10624 | Me(S) | single bond | H | CH₂CF₃ | C(=O)Me | Me | *c*-Pr | O |
| D-10625 | H | single bond | Me | Et | C(=O)Me | Me | *c*-Pr | O |
| D-10626 | Me | single bond | H | Et | C(=O)Me | Me | *c*-Pr | O |
| D-10627 | Me(R) | single bond | H | Et | C(=O)Me | Me | *c*-Pr | O |
| D-10628 | Me(S) | single bond | H | Et | C(=O)Me | Me | *c*-Pr | O |
| 0-10629 | H | single bond | Me | *c*-Pr | C(=O)Me | Me | *c*-Pr | O |
| D-10630 | Me | single bond | H | *c*-Pr | C(=O)Me | Me | *c*-Pr | O |
| D-10631 | Me(R) | single bond | H | *c*-Pr | C(=O)Me | Me | *c-*Pr | O |
| D-10632 | Me(S) | single bond | H | *c*-Pr | C(=O)Me | Me | *c-*Pr | O |
| D-10633 | H | single bond | Me | *c*-Pr(1-CN) | C(=O)Me | Me | *c*-Pr | O |
| D-10634 | Me | single bond | H | *c*-Pr(1-CN) | C(=O)Me | Me | *c*-Pr | O |
| D-10635 | Me(R) | single bond | H | *c*-Pr(1-CN) | C(=O)Me | Me | *c*-Pr | O |
| D-10636 | Me(S) | single bond | H | *c*-Pr(1-CN) | C(=O)Me | Me | *c*-Pr | O |
| D-10637 | H | single bond | Me | CH₂CF₃ | C(=O)Me | Cl | OMe | O |
| D-10638 | Me | single bond | H | CH₂CF₃ | C(=O)Me | Cl | OMe | O |

**[Table 888]**

| Compound Number | Y | W | Z | R¹ | R² | R³ | R¹⁰ | A |
|---|---|---|---|---|---|---|---|---|
| D-10639 | Me(R) | single bond | H | CH₂CF₃ | C(=O)Me | Cl | OMe | O |
| D-10640 | Me(S) | single bond | H | CH₂CF₃ | C(=O)Me | Cl | OMe | O |
| D-10641 | H | single bond | Me | Et | C(=O)Me | Cl | OMe | O |
| D-10642 | Me | single bond | H | Et | C(=O)Me | Cl | OMe | O |
| D-10643 | Me(R) | single bond | H | Et | C(=O)Me | Cl | OMe | O |
| D-10644 | Me(S) | single bond | H | Et | C(=O)Me | Cl | OMe | O |
| D-10645 | H | single bond | Me | *c*-Pr | C(=O)Me | Cl | OMe | O |
| D-10646 | Me | single bond | H | *c*-Pr | C(=O)Me | Cl | OMe | O |
| D-10647 | Me(R) | single bond | H | *c*-Pr | C(=O)Me | Cl | OMe | O |
| D-10648 | Me(S) | single bond | H | *c*-Pr | C(=O)Me | Cl | OMe | O |
| D-10649 | H | single bond | Me | *c*-Pr(1-CN) | C(=O)Me | Cl | OMe | O |
| D-10650 | Me | single bond | H | *c*-Pr(1-CN) | C(=O)Me | Cl | OMe | O |
| D-10651 | Me(R) | single bond | H | *c*-Pr(1-CN) | C(=O)Me | Cl | OMe | O |
| D-10652 | Me(S) | single bond | H | *c*-Pr(1-CN) | C(=O)Me | Cl | OMe | O |
| D-10653 | H | single bond | Me | CH₂CF₃ | C(=O)Me | Me | OMe | O |
| D-10654 | Me | single bond | H | CH₂CF₃ | C(=O)Me | Me | OMe | O |
| D-10655 | Me(R) | single bond | H | CH₂CF₃ | C(=O)Me | Me | OMe | O |
| D-10656 | Me(S) | single bond | H | CH₂CF₃ | C(=O)Me | Me | OMe | O |
| D-10657 | H | single bond | Me | Et | C(=O)Me | Me | OMe | O |
| D-10658 | Me | single bond | H | Et | C(=O)Me | Me | OMe | O |
| D-10659 | Me(R) | single bond | H | Et | C(=O)Me | Me | OMe | O |
| D-10660 | Me(S) | single bond | H | Et | C(=O)Me | Me | OMe | O |
| D-10661 | H | single bond | Me | *c*-Pr | C(=O)Me | Me | OMe | O |
| D-10662 | Me | single bond | H | *c*-Pr | C(=O)Me | Me | OMe | O |
| D-10663 | Me(R) | single bond | H | *c*-Pr | C(=O)Me | Me | OMe | O |
| D-10664 | Me(S) | single bond | H | *c*-Pr | C(=O)Me | Me | OMe | O |
| D-10665 | H | single bond | Me | *c*-Pr(1-CN) | C(=O)Me | Me | OMe | O |
| D-10666 | Me | single bond | H | *c*-Pr(1-CN) | C(=O)Me | Me | OMe | O |
| D-10667 | Me(R) | single bond | H | *c*-Pr(1-CN) | C(=O)Me | Me | OMe | O |
| D-10668 | Me(S) | single bond | H | *c*-Pr(1-CN) | C(=O)Me | Me | OMe | O |
| D-10669 | H | single bond | Me | CH₂CF₃ | C(=O)Me | Cl | C(=O)(*i*-Pr) | O |
| D-10670 | Me | single bond | H | CH₂CF₃ | C(=O)Me | Cl | C(=O)(*i*-Pr) | O |
| D-10671 | Me(R) | single bond | H | CH₂CF₃ | C(=O)Me | Cl | C(=O)(*i*-Pr) | O |
| D-10672 | Me(S) | single bond | H | CH₂CF₃ | C(=O)Me | Cl | C(=O)(*i*-Pr) | O |
| D-10673 | H | single bond | Me | Et | C(=O)Me | Cl | C(=O)(*i*-Pr) | O |
| D-10674 | Me | single bond | H | Et | C(=O)Me | Cl | C(=O)(*i*-Pr) | O |
| D-10675 | Me(R) | single bond | H | Et | C(=O)Me | Cl | C(=O)(*i*-Pr) | O |
| D-10676 | Me(S) | single bond | H | Et | C(=O)Me | Cl | C(=O)(*i*-Pr) | O |
| D-10677 | H | single bond | Me | *c*-Pr | C(=O)Me | Cl | C(=O)(*i*-Pr) | O |
| D-10678 | Me | single bond | H | *c*-Pr | C(=O)Me | Cl | C(=O)(*i*-Pr) | O |
| D-10679 | Me(R) | single bond | H | *c*-Pr | C(=O)Me | Cl | C(=O)(*i*-Pr) | O |
| D-10680 | Me(S) | single bond | H | *c*-Pr | C(=O)Me | Cl | C(=O)(*i*-Pr) | O |
| D-10681 | H | single bond | Me | *c*-Pr(1-CN) | C(=O)Me | Cl | C(=O)(*i*-Pr) | O |
| D-10682 | Me | single bond | H | *c*-Pr(1-CN) | C(=O)Me | Cl | C(=O)(*i*-Pr) | O |

**[Table 889]**

| Compound Number | Y | W | Z | R¹ | R² | R³ | R¹⁰ | A |
|---|---|---|---|---|---|---|---|---|
| D-10683 | Me(R) | single bond | H | *c*-Pr(1-CN) | C(=O)Me | Cl | C(=O)(*i*-Pr) | O |
| D-10684 | Me(S) | single bond | H | *c*-Pr(1-CN) | C(=O)Me | Cl | C(=O)(*i*-Pr) | O |
| D-10685 | H | single bond | Me | CH₂CF₃ | C(=O)Me | Me | C(=O)(*i*-Pr) | O |
| D-10686 | Me | single bond | H | CH₂CF₃ | C(=O)Me | Me | C(=O)(*i*-Pr) | O |
| D-10687 | Me(R) | single bond | H | CH₂CF₃ | C(=O)Me | Me | C(=O)(*i*-Pr) | O |
| D-10688 | Me(S) | single bond | H | CH₂CF₃ | C(=O)Me | Me | C(=O)(*i*-Pr) | O |
| D-10689 | H | single bond | Me | Et | C(=O)Me | Me | C(=O)(*i*-Pr) | O |
| D-10690 | Me | single bond | H | Et | C(=O)Me | Me | C(=O)(*i*-Pr) | O |
| D-10691 | Me(R) | single bond | H | Et | C(=O)Me | Me | C(=O)(*i*-Pr) | O |
| D-10692 | Me(S) | single bond | H | Et | C(=O)Me | Me | C(=O)(*i*-Pr) | O |
| D-10693 | H | single bond | Me | *c*-Pr | C(=O)Me | Me | C(=O)(*i*-Pr) | O |
| D-10694 | Me | single bond | H | *c*-Pr | C(=O)Me | Me | C(=O)(*i*-Pr) | O |
| D-10695 | Me(R) | single bond | H | *c*-Pr | C(=O)Me | Me | C(=O)(*i*-Pr) | O |
| D-10696 | Me(S) | single bond | H | *c*-Pr | C(=O)Me | Me | C(=O)(*i*-Pr) | O |
| D-10697 | H | single bond | Me | *c*-Pr(1-CN) | C(=O)Me | Me | C(=O)(*i*-Pr) | O |
| D-10698 | Me | single bond | H | *c*-Pr(1-CN) | C(=O)Me | Me | C(=O)(*i*-Pr) | O |
| D-10699 | Me(R) | single bond | H | *c*-Pr(1-CN) | C(=O)Me | Me | C(=O)(*i*-Pr) | O |
| D-10700 | Me(S) | single bond | H | *c*-Pr(1-CN) | C(=O)Me | Me | C(=O)(*i*-Pr) | O |
| D-10701 | H | single bond | Me | CH₂CF₃ | C(=O)Me | Cl | C(=O)O(*t*-Bu) | O |
| D-10702 | Me | single bond | H | CH₂CF₃ | C(=O)Me | Cl | C(=O)O(*t*-Bu) | O |
| D-10703 | Me(R) | single bond | H | CH₂CF₃ | C(=O)Me | Cl | C(=O)O(*t*-Bu) | O |
| D-10704 | Me(S) | single bond | H | CH₂CF₃ | C(=O)Me | Cl | C(=O)O(*t*-Bu) | O |
| D-10705 | H | single bond | Me | Et | C(=O)Me | Cl | C(=O)O(*t*-Bu) | O |
| D-10706 | Me | single bond | H | Et | C(=O)Me | Cl | C(=O)O(*t*-Bu) | O |
| D-10707 | Me(R) | single bond | H | Et | C(=O)Me | Cl | C(=O)O(*t*-Bu) | O |
| D-10708 | Me(S) | single bond | H | Et | C(=O)Me | Cl | C(=O)O(*t*-Bu) | O |
| D-10709 | H | single bond | Me | *c*-Pr | C(=O)Me | Cl | C(=O)O(*t*-Bu) | O |
| D-10710 | Me | single bond | H | *c*-Pr | C(=O)Me | Cl | C(=O)O(*t*-Bu) | O |
| D-10711 | Me(R) | single bond | H | *c*-Pr | C(=O)Me | Cl | C(=O)O(*t*-Bu) | O |
| D-10712 | Me(S) | single bond | H | *c*-Pr | C(=O)Me | Cl | C(=O)O(*t*-Bu) | O |
| D-10713 | H | single bond | Me | *c*-Pr(1-CN) | C(=O)Me | Cl | C(=O)O(*t*-Bu) | O |
| D-10714 | Me | single bond | H | *c*-Pr(1-CN) | C(=O)Me | Cl | C(=O)O(*t*-Bu) | O |
| D-10715 | Me(R) | single bond | H | *c*-Pr(1-CN) | C(=O)Me | Cl | C(=O)O(*t*-Bu) | O |
| D-10716 | Me(S) | single bond | H | *c*-Pr(1-CN) | C(=O)Me | Cl | C(=O)O(*t*-Bu) | O |
| D-10717 | H | single bond | Me | CH₂CF₃ | C(=O)Me | Me | C(=O)O(*t*-Bu) | O |
| D-10718 | Me | single bond | H | CH₂CF₃ | C(=O)Me | Me | C(=O)O(*t*-Bu) | O |
| D-10719 | Me(R) | single bond | H | CH₂CF₃ | C(=O)Me | Me | C(=O)O(*t*-Bu) | O |
| D-10720 | Me(S) | single bond | H | CH₂CF₃ | C(=O)Me | Me | C(=O)O(*t*-Bu) | O |
| D-10721 | H | single bond | Me | Et | C(=O)Me | Me | C(=O)O(*t*-Bu) | O |
| D-10722 | Me | single bond | H | Et | C(=O)Me | Me | C(=O)O(*t*-Bu) | O |
| D-10723 | Me(R) | single bond | H | Et | C(=O)Me | Me | C(=O)O(*t*-Bu) | O |
| D-10724 | Me(S) | single bond | H | Et | C(=O)Me | Me | C(=O)O(*t*-Bu) | O |
| D-10725 | H | single bond | Me | *c*-Pr | C(=O)Me | Me | C(=O)O(*t*-Bu) | O |
| D-10726 | Me | single bond | H | *c*-Pr | C(=O)Me | Me | C(=O)O(*t*-Bu) | O |

**[Table 890]**

| Compound Number | Y | W | Z | R¹ | R² | R³ | R¹⁰ | A |
|---|---|---|---|---|---|---|---|---|
| D-10727 | Me(R) | single bond | H | *c*-Pr | C(=O)Me | Me | C(=O)O(*t*-Bu) | O |
| D-10728 | Me(S) | single bond | H | *c*-Pr | C(=O)Me | Me | C(=O)O(*t*-Bu) | O |
| 0-10729 | H | single bond | Me | *c*-Pr(1-CN) | C(=O)Me | Me | C(=O)O(*t*-Bu) | O |
| D-10730 | Me | single bond | H | *c*-Pr(1-CN) | C(=O)Me | Me | C(=O)O(*t*-Bu) | O |
| D-10731 | Me(R) | single bond | H | *c*-Pr(1-CN) | C(=O)Me | Me | C(=O)O(*t*-Bu) | O |
| D-10732 | Me(S) | single bond | H | *c*-Pr(1-CN) | C(=O)Me | Me | C(=O)O(*t*-Bu) | O |
| D-10733 | H | single bond | Me | CH₂CF₃ | C(=O)Me | Cl | CH₂OEt | O |
| D-10734 | Me | single bond | H | CH₂CF₃ | C(=O)Me | Cl | CH₂OEt | O |
| D-10735 | Me(R) | single bond | H | CH₂CF₃ | C(=O)Me | Cl | CH₂OEt | O |
| D-10736 | Me(S) | single bond | H | CH₂CF₃ | C(=O)Me | Cl | CH₂OEt | O |
| D-10737 | H | single bond | Me | Et | C(=O)Me | Cl | CH₂OEt | O |
| D-10738 | Me | single bond | H | Et | C(=O)Me | Cl | CH₂OEt | O |
| D-10739 | Me(R) | single bond | H | Et | C(=O)Me | Cl | CH₂OEt | O |
| D-10740 | Me(S) | single bond | H | Et | C(=O)Me | Cl | CH₂OEt | O |
| D-10741 | H | single bond | Me | *c*-Pr | C(=O)Me | Cl | CH₂OEt | O |
| D-10742 | Me | single bond | H | *c*-Pr | C(=O)Me | Cl | CH₂OEt | O |
| D-10743 | Me(R) | single bond | H | *c*-Pr | C(=O)Me | Cl | CH₂OEt | O |
| D-10744 | Me(S) | single bond | H | *c*-Pr | C(=O)Me | Cl | CH₂OEt | O |
| D-10745 | H | single bond | Me | *c*-Pr(1-CN) | C(=O)Me | Cl | CH₂OEt | O |
| D-10746 | Me | single bond | H | *c*-Pr(1-CN) | C(=O)Me | Cl | CH₂OEt | O |
| D-10747 | Me(R) | single bond | H | *c*-Pr(1-CN) | C(=O)Me | Cl | CH₂OEt | O |
| D-10748 | Me(S) | single bond | H | *c*-Pr(1-CN) | C(=O)Me | Cl | CH₂OEt | O |
| D-10749 | H | single bond | Me | CH₂CF₃ | C(=O)Me | Me | CH₂OEt | O |
| D-10750 | Me | single bond | H | CH₂CF₃ | C(=O)Me | Me | CH₂OEt | O |
| D-10751 | Me(R) | single bond | H | CH₂CF₃ | C(=O)Me | Me | CH₂OEt | O |
| D-10752 | Me(S) | single bond | H | CH₂CF₃ | C(=O)Me | Me | CH₂OEt | O |
| D-10753 | H | single bond | Me | Et | C(=O)Me | Me | CH₂OEt | O |
| D-10754 | Me | single bond | H | Et | C(=O)Me | Me | CH₂OEt | O |
| D-10755 | Me(R) | single bond | H | Et | C(=O)Me | Me | CH₂OEt | O |
| D-10756 | Me(S) | single bond | H | Et | C(=O)Me | Me | CH₂OEt | O |
| D-10757 | H | single bond | Me | *c*-Pr | C(=O)Me | Me | CH₂OEt | O |
| D-10758 | Me | single bond | H | *c*-Pr | C(=O)Me | Me | CH₂OEt | O |
| D-10759 | Me(R) | single bond | H | *c*-Pr | C(=O)Me | Me | CH₂OEt | O |
| D-10760 | Me(S) | single bond | H | *c*-Pr | C(=O)Me | Me | CH₂OEt | O |
| D-10761 | H | single bond | Me | *c*-Pr(1-CN) | C(=O)Me | Me | CH₂OEt | O |
| D-10762 | Me | single bond | H | *c*-Pr(1-CN) | C(=O)Me | Me | CH₂OEt | O |
| D-10763 | Me(R) | single bond | H | *c*-Pr(1-CN) | C(=O)Me | Me | CH₂OEt | O |
| D-10764 | Me(S) | single bond | H | *c*-Pr(1-CN) | C(=O)Me | Me | CH₂OEt | O |
| D-10765 | H | single bond | Me | CH₂CF₃ | C(=O)Me | Cl | CH₂SMe | O |
| D-10766 | Me | single bond | H | CH₂CF₃ | C(=O)Me | Cl | CH₂SMe | O |
| D-10767 | Me(R) | single bond | H | CH₂CF₃ | C(=O)Me | Cl | CH₂SMe | O |
| D-10768 | Me(S) | single bond | H | CH₂CF₃ | C(=O)Me | Cl | CH₂SMe | O |
| D-10769 | H | single bond | Me | Et | C(=O)Me | Cl | CH₂SMe | O |
| D-10770 | Me | single bond | H | Et | C(=O)Me | Cl | CH₂SMe | O |

**[Table 891]**

| Compound Number | Y | W | Z | R¹ | R² | R³ | R¹⁰ | A |
|---|---|---|---|---|---|---|---|---|
| D-10771 | Me(R) | single bond | H | Et | C(=O)Me | Cl | CH₂SMe | O |
| D-10772 | Me(S) | single bond | H | Et | C(=O)Me | Cl | CH₂SMe | O |
| D-10773 | H | single bond | Me | *c*-Pr | C(=O)Me | Cl | CH₂SMe | O |
| D-10774 | Me | single bond | H | *c*-Pr | C(=O)Me | Cl | CH₂SMe | O |
| D-10775 | Me(R) | single bond | H | *c*-Pr | C(=O)Me | Cl | CH₂SMe | O |
| D-10776 | Me(S) | single bond | H | *c*-Pr | C(=O)Me | Cl | CH₂SMe | O |
| D-10777 | H | single bond | Me | *c*-Pr(1-CN) | C(=O)Me | Cl | CH₂SMe | O |
| D-10778 | Me | single bond | H | *c*-Pr(1-CN) | C(=O)Me | Cl | CH₂SMe | O |
| D-10779 | Me(R) | single bond | H | *c*-Pr(1-CN) | C(=O)Me | Cl | CH₂SMe | O |
| D-10780 | Me(S) | single bond | H | *c*-Pr(1-CN) | C(=O)Me | Cl | CH₂SMe | O |
| D-10781 | H | single bond | Me | CH₂CF₃ | C(=O)Me | Me | CH₂SMe | O |
| D-10782 | Me | single bond | H | CH₂CF₃ | C(=O)Me | Me | CH₂SMe | O |
| D-10783 | Me(R) | single bond | H | CH₂CF₃ | C(=O)Me | Me | CH₂SMe | O |
| D-10784 | Me(S) | single bond | H | CH₂CF₃ | C(=O)Me | Me | CH₂SMe | O |
| D-10785 | H | single bond | Me | Et | C(=O)Me | Me | CH₂SMe | O |
| D-10786 | Me | single bond | H | Et | C(=O)Me | Me | CH₂SMe | O |
| D-10787 | Me(R) | single bond | H | Et | C(=O)Me | Me | CH₂SMe | O |
| D-10788 | Me(S) | single bond | H | Et | C(=O)Me | Me | CH₂SMe | O |
| D-10789 | H | single bond | Me | *c*-Pr | C(=O)Me | Me | CH₂SMe | O |
| D-10790 | Me | single bond | H | *c*-Pr | C(=O)Me | Me | CH₂SMe | O |
| D-10791 | Me(R) | single bond | H | *c*-Pr | C(=O)Me | Me | CH₂SMe | O |
| D-10792 | Me(S) | single bond | H | *c*-Pr | C(=O)Me | Me | CH₂SMe | O |
| D-10793 | H | single bond | Me | *c*-Pr(1-CN) | C(=O)Me | Me | CH₂SMe | O |
| D-10794 | Me | single bond | H | *c*-Pr(1-CN) | C(=O)Me | Me | CH₂SMe | O |
| D-10795 | Me(R) | single bond | H | *c*-Pr(1-CN) | C(=O)Me | Me | CH₂SMe | O |
| D-10796 | Me(S) | single bond | H | *c*-Pr(1-CN) | C(=O)Me | Me | CH₂SMe | O |
| D-10797 | H | single bond | Me | CH₂CF₃ | C(=O)Me | Cl | CH₂S(=O)₂Me | O |
| D-10798 | Me | single bond | H | CH₂CF₃ | C(=O)Me | Cl | CH₂S(=O)₂Me | O |
| D-10799 | Me(R) | single bond | H | CH₂CF₃ | C(=O)Me | Cl | CH₂S(=O)₂Me | O |
| D-10800 | Me(S) | single bond | H | CH₂CF₃ | C(=O)Me | Cl | CH₂S(=O)₂Me | O |
| D-10801 | H | single bond | Me | Et | C(=O)Me | Cl | CH₂S(=O)₂Me | O |
| D-10802 | Me | single bond | H | Et | C(=O)Me | Cl | CH₂S(=O)₂Me | O |
| D-10803 | Me(R) | single bond | H | Et | C(=O)Me | Cl | CH₂S(=O)₂Me | O |
| D-10804 | Me(S) | single bond | H | Et | C(=O)Me | Cl | CH₂S(=O)₂Me | O |
| D-10805 | H | single bond | Me | *c*-Pr | C(=O)Me | Cl | CH₂S(=O)₂Me | O |
| D-10806 | Me | single bond | H | *c*-Pr | C(=O)Me | Cl | CH₂S(=O)₂Me | O |
| D-10807 | Me(R) | single bond | H | *c*-Pr | C(=O)Me | Cl | CH₂S(=O)₂Me | O |
| D-10808 | Me(S) | single bond | H | *c*-Pr | C(=O)Me | Cl | CH₂S(=O)₂Me | O |
| D-10809 | H | single bond | Me | *c*-Pr(1-CN) | C(=O)Me | Cl | CH₂S(=O)₂Me | O |
| D-10810 | Me | single bond | H | *c*-Pr(1-CN) | C(=O)Me | Cl | CH₂S(=O)₂Me | O |
| D-10811 | Me(R) | single bond | H | *c*-Pr(1-CN) | C(=O)Me | Cl | CH₂S(=O)₂Me | O |
| D-10812 | Me(S) | single bond | H | *c*-Pr(1-CN) | C(=O)Me | Cl | CH₂S(=O)₂Me | O |
| D-10813 | H | single bond | Me | CH₂CF₃ | C(=O)Me | Me | CH₂S(=O)₂Me | O |
| D-10814 | Me | single bond | H | CH₂CF₃ | C(=O)Me | Me | CH₂S(=O)₂Me | O |

**[Table 892]**

| Compound Number | Y | W | Z | R¹ | R² | R³ | R¹⁰ | A |
|---|---|---|---|---|---|---|---|---|
| D-10815 | Me(R) | single bond | H | CH₂CF₃ | C(=O)Me | Me | CH₂S(=O)₂Me | O |
| D-10816 | Me(S) | single bond | H | CH₂CF₃ | C(=O)Me | Me | CH₂S(=O)₂Me | O |
| D-10817 | H | single bond | Me | Et | C(=O)Me | Me | CH₂S(=O)₂Me | O |
| D-10818 | Me | single bond | H | Et | C(=O)Me | Me | CH₂S(=O)₂Me | O |
| D-10819 | Me(R) | single bond | H | Et | C(=O)Me | Me | CH₂S(=O)₂Me | O |
| D-10820 | Me(S) | single bond | H | Et | C(=O)Me | Me | CH₂S(=O)₂Me | O |
| D-10821 | H | single bond | Me | *c*-Pr | C(=O)Me | Me | CH₂S(=O)₂Me | O |
| D-10822 | Me | single bond | H | *c*-Pr | C(=O)Me | Me | CH₂S(=O)₂Me | O |
| 0-10823 | Me(R) | single bond | H | *c*-Pr | C(=O)Me | Me | CH₂S(=O)₂Me | O |
| D-10824 | Me(S) | single bond | H | *c*-Pr | C(=O)Me | Me | CH₂S(=O)₂Me | O |
| D-10825 | H | single bond | Me | *c*-Pr(1-CN) | C(=O)Me | Me | CH₂S(=O)₂Me | O |
| D-10826 | Me | single bond | H | *c*-Pr(1-CN) | C(=O)Me | Me | CH₂S(=O)₂Me | O |
| D-10827 | Me(R) | single bond | H | *c*-Pr(1-CN) | C(=O)Me | Me | CH₂S(=O)₂Me | O |
| D-10828 | Me(S) | single bond | H | *c*-Pr(1-CN) | C(=O)Me | Me | CH₂S(=O)₂Me | O |
| 0-10829 | H | single bond | Me | CH₂CF₃ | C(=O)Me | Cl | CH₂C(=O)Me | O |
| D-10830 | Me | single bond | H | CH₂CF₃ | C(=O)Me | Cl | CH₂C(=O)Me | O |
| D-10831 | Me(R) | single bond | H | CH₂CF₃ | C(=O)Me | Cl | CH₂C(=O)Me | O |
| D-10832 | Me(S) | single bond | H | CH₂CF₃ | C(=O)Me | Cl | CH₂C(=O)Me | O |
| D-10833 | H | single bond | Me | Et | C(=O)Me | Cl | CH₂C(=O)Me | O |
| D-10834 | Me | single bond | H | Et | C(=O)Me | Cl | CH₂C(=O)Me | O |
| D-10835 | Me(R) | single bond | H | Et | C(=O)Me | Cl | CH₂C(=O)Me | O |
| D-10836 | Me(S) | single bond | H | Et | C(=O)Me | Cl | CH₂C(=O)Me | O |
| D-10837 | H | single bond | Me | *c*-Pr | C(=O)Me | Cl | CH₂C(=O)Me | O |
| D-10838 | Me | single bond | H | *c*-Pr | C(=O)Me | Cl | CH₂C(=O)Me | O |
| D-10839 | Me(R) | single bond | H | *c*-Pr | C(=O)Me | Cl | CH₂C(=O)Me | O |
| D-10840 | Me(S) | single bond | H | *c*-Pr | C(=O)Me | Cl | CH₂C(=O)Me | O |
| D-10841 | H | single bond | Me | *c*-Pr(1-CN) | C(=O)Me | Cl | CH₂C(=O)Me | O |
| D-10842 | Me | single bond | H | *c*-Pr(1-CN) | C(=O)Me | Cl | CH₂C(=O)Me | O |
| D-10843 | Me(R) | single bond | H | *c*-Pr(1-CN) | C(=O)Me | Cl | CH₂C(=O)Me | O |
| D-10844 | Me(S) | single bond | H | *c*-Pr(1-CN) | C(=O)Me | Cl | CH₂C(=O)Me | O |
| D-10845 | H | single bond | Me | CH₂CF₃ | C(=O)Me | Me | CH₂C(=O)Me | O |
| D-10846 | Me | single bond | H | CH₂CF₃ | C(=O)Me | Me | CH₂C(=O)Me | O |
| D-10847 | Me(R) | single bond | H | CH₂CF₃ | C(=O)Me | Me | CH₂C(=O)Me | O |
| D-10848 | Me(S) | single bond | H | CH₂CF₃ | C(=O)Me | Me | CH₂C(=O)Me | O |
| D-10849 | H | single bond | Me | Et | C(=O)Me | Me | CH₂C(=O)Me | O |
| D-10850 | Me | single bond | H | Et | C(=O)Me | Me | CH₂C(=O)Me | O |
| D-10851 | Me(R) | single bond | H | Et | C(=O)Me | Me | CH₂C(=O)Me | O |
| D-10852 | Me(S) | single bond | H | Et | C(=O)Me | Me | CH₂C(=O)Me | O |
| D-10853 | H | single bond | Me | *c*-Pr | C(=O)Me | Me | CH₂C(=O)Me | O |
| D-10854 | Me | single bond | H | *c*-Pr | C(=O)Me | Me | CH₂C(=O)Me | O |
| D-10855 | Me(R) | single bond | H | *c*-Pr | C(=O)Me | Me | CH₂C(=O)Me | O |
| D-10856 | Me(S) | single bond | H | *c*-Pr | C(=O)Me | Me | CH₂C(=O)Me | O |
| D-10857 | H | single bond | Me | *c*-Pr(1-CN) | C(=O)Me | Me | CH₂C(=O)Me | O |
| D-10858 | Me | single bond | H | *c*-Pr(1-CN) | C(=O)Me | Me | CH₂C(=O)Me | O |

**[Table 893]**

| Compound Number | Y | W | Z | R¹ | R² | R³ | R¹⁰ | A |
|---|---|---|---|---|---|---|---|---|
| D-10859 | Me(R) | single bond | H | *c*-Pr(1-CN) | C(=O)Me | Me | CH₂C(=O)Me | O |
| D-10860 | Me(S) | single bond | H | *c*-Pr(1-CN) | C(=O)Me | Me | CH₂C(=O)Me | O |
| D-10861 | H | single bond | Me | CH₂CF₃ | C(=O)Me | Cl | CH₂C(=O)OMe | O |
| 0-10862 | Me | single bond | H | CH₂CF₃ | C(=O)Me | Cl | CH₂C(=O)OMe | O |
| D-10863 | Me(R) | single bond | H | CH₂CF₃ | C(=O)Me | Cl | CH₂C(=O)OMe | O |
| D-10864 | Me(S) | single bond | H | CH₂CF₃ | C(=O)Me | Cl | CH₂C(=O)OMe | O |
| D-10865 | H | single bond | Me | Et | C(=O)Me | Cl | CH₂C(=O)OMe | O |
| D-10866 | Me | single bond | H | Et | C(=O)Me | Cl | CH₂C(=O)OMe | O |
| D-10867 | Me(R) | single bond | H | Et | C(=O)Me | Cl | CH₂C(=O)OMe | O |
| D-10868 | Me(S) | single bond | H | Et | C(=O)Me | Cl | CH₂C(=O)OMe | O |
| D-10869 | H | single bond | Me | *c*-Pr | C(=O)Me | Cl | CH₂C(=O)OMe | O |
| D-10870 | Me | single bond | H | *c*-Pr | C(=O)Me | Cl | CH₂C(=O)OMe | O |
| D-10871 | Me(R) | single bond | H | *c*-Pr | C(=O)Me | Cl | CH₂C(=O)OMe | O |
| D-10872 | Me(S) | single bond | H | *c*-Pr | C(=O)Me | Cl | CH₂C(=O)OMe | O |
| D-10873 | H | single bond | Me | *c*-Pr(1-CN) | C(=O)Me | Cl | CH₂C(=O)OMe | O |
| D-10874 | Me | single bond | H | *c*-Pr(1-CN) | C(=O)Me | Cl | CH₂C(=O)OMe | O |
| D-10875 | Me(R) | single bond | H | *c*-Pr(1-CN) | C(=O)Me | Cl | CH₂C(=O)OMe | O |
| D-10876 | Me(S) | single bond | H | *c*-Pr(1-CN) | C(=O)Me | Cl | CH₂C(=O)OMe | O |
| D-10877 | H | single bond | Me | CH₂CF₃ | C(=O)Me | Me | CH₂C(=O)OMe | O |
| D-10878 | Me | single bond | H | CH₂CF₃ | C(=O)Me | Me | CH₂C(=O)OMe | O |
| D-10879 | Me(R) | single bond | H | CH₂CF₃ | C(=O)Me | Me | CH₂C(=O)OMe | O |
| D-10880 | Me(S) | single bond | H | CH₂CF₃ | C(=O)Me | Me | CH₂C(=O)OMe | O |
| D-10881 | H | single bond | Me | Et | C(=O)Me | Me | CH₂C(=O)OMe | O |
| D-10882 | Me | single bond | H | Et | C(=O)Me | Me | CH₂C(=O)OMe | O |
| D-10883 | Me(R) | single bond | H | Et | C(=O)Me | Me | CH₂C(=O)OMe | O |
| D-10884 | Me(S) | single bond | H | Et | C(=O)Me | Me | CH₂C(=O)OMe | O |
| D-10885 | H | single bond | Me | *c*-Pr | C(=O)Me | Me | CH₂C(=O)OMe | O |
| D-10886 | Me | single bond | H | *c*-Pr | C(=O)Me | Me | CH₂C(=O)OMe | O |
| D-10887 | Me(R) | single bond | H | *c*-Pr | C(=O)Me | Me | CH₂C(=O)OMe | O |
| D-10888 | Me(S) | single bond | H | *c*-Pr | C(=O)Me | Me | CH₂C(=O)OMe | O |
| D-10889 | H | single bond | Me | *c*-Pr(1-CN) | C(=O)Me | Me | CH₂C(=O)OMe | O |
| D-10890 | Me | single bond | H | *c*-Pr(1-CN) | C(=O)Me | Me | CH₂C(=O)OMe | O |
| D-10891 | Me(R) | single bond | H | *c*-Pr(1-CN) | C(=O)Me | Me | CH₂C(=O)OMe | O |
| D-10892 | Me(S) | single bond | H | *c*-Pr(1-CN) | C(=O)Me | Me | CH₂C(=O)OMe | O |
| D-10893 | H | single bond | Me | CH₂CF₃ | C(=O)Me | Cl | CH₂C(=O)NHMe | O |
| D-10894 | Me | single bond | H | CH₂CF₃ | C(=O)Me | Cl | CH₂C(=O)NHMe | O |
| D-10895 | Me(R) | single bond | H | CH₂CF₃ | C(=O)Me | Cl | CH₂C(=O)NHMe | O |
| D-10896 | Me(S) | single bond | H | CH₂CF₃ | C(=O)Me | Cl | CH₂C(=O)NHMe | O |
| D-10897 | H | single bond | Me | Et | C(=O)Me | Cl | CH₂C(=O)NHMe | O |
| D-10898 | Me | single bond | H | Et | C(=O)Me | Cl | CH₂C(=O)NHMe | O |
| D-10899 | Me(R) | single bond | H | Et | C(=O)Me | Cl | CH₂C(=O)NHMe | O |
| D-10900 | Me(S) | single bond | H | Et | C(=O)Me | Cl | CH₂C(=O)NHMe | O |
| D-10901 | H | single bond | Me | *c*-Pr | C(=O)Me | Cl | CH₂C(=O)NHMe | O |
| D-10902 | Me | single bond | H | *c*-Pr | C(=O)Me | Cl | CH₂C(=O)NHMe | O |

**[Table 894]**

| Compound Number | Y | W | Z | R¹ | R² | R³ | R¹⁰ | A |
|---|---|---|---|---|---|---|---|---|
| D-10903 | Me(R) | single bond | H | *c*-Pr | C(=O)Me | Cl | CH₂C(=O)NHMe | O |
| D-10904 | Me(S) | single bond | H | *c*-Pr | C(=O)Me | Cl | CH₂C(=O)NHMe | O |
| D-10905 | H | single bond | Me | *c*-Pr(1-CN) | C(=O)Me | Cl | CH₂C(=O)NHMe | O |
| D-10906 | Me | single bond | H | *c*-Pr(1-CN) | C(=O)Me | Cl | CH₂C(=O)NHMe | O |
| D-10907 | Me(R) | single bond | H | *c*-Pr(1-CN) | C(=O)Me | Cl | CH₂C(=O)NHMe | O |
| D-10908 | Me(S) | single bond | H | *c*-Pr(1-CN) | C(=O)Me | Cl | CH₂C(=O)NHMe | O |
| D-10909 | H | single bond | Me | CH₂CF₃ | C(=O)Me | Me | CH₂C(=O)NHMe | O |
| D-10910 | Me | single bond | H | CH₂CF₃ | C(=O)Me | Me | CH₂C(=O)NHMe | O |
| D-10911 | Me(R) | single bond | H | CH₂CF₃ | C(=O)Me | Me | CH₂C(=O)NHMe | O |
| D-10912 | Me(S) | single bond | H | CH₂CF₃ | C(=O)Me | Me | CH₂C(=O)NHMe | O |
| D-10913 | H | single bond | Me | Et | C(=O)Me | Me | CH₂C(=O)NHMe | O |
| D-10914 | Me | single bond | H | Et | C(=O)Me | Me | CH₂C(=O)NHMe | O |
| D-10915 | Me(R) | single bond | H | Et | C(=O)Me | Me | CH₂C(=O)NHMe | O |
| D-10916 | Me(S) | single bond | H | Et | C(=O)Me | Me | CH₂C(=O)NHMe | O |
| 0-10917 | H | single bond | Me | *c*-Pr | C(=O)Me | Me | CH₂C(=O)NHMe | O |
| D-10918 | Me | single bond | H | *c*-Pr | C(=O)Me | Me | CH₂C(=O)NHMe | O |
| D-10919 | Me(R) | single bond | H | *c*-Pr | C(=O)Me | Me | CH₂C(=O)NHMe | O |
| D-10920 | Me(S) | single bond | H | *c*-Pr | C(=O)Me | Me | CH₂C(=O)NHMe | O |
| D-10921 | H | single bond | Me | *c*-Pr(1-CN) | C(=O)Me | Me | CH₂C(=O)NHMe | O |
| D-10922 | Me | single bond | H | *c*-Pr(1-CN) | C(=O)Me | Me | CH₂C(=O)NHMe | O |
| D-10923 | Me(R) | single bond | H | *c*-Pr(1-CN) | C(=O)Me | Me | CH₂C(=O)NHMe | O |
| D-10924 | Me(S) | single bond | H | *c*-Pr(1-CN) | C(=O)Me | Me | CH₂C(=O)NHMe | O |
| D-10925 | H | single bond | Me | CH₂CF₃ | C(=O)Me | Cl | CH₂OC(=O)Me | O |
| D-10926 | Me | single bond | H | CH₂CF₃ | C(=O)Me | Cl | CH₂OC(=O)Me | O |
| D-10927 | Me(R) | single bond | H | CH₂CF₃ | C(=O)Me | Cl | CH₂OC(=O)Me | O |
| D-10928 | Me(S) | single bond | H | CH₂CF₃ | C(=O)Me | Cl | CH₂OC(=O)Me | O |
| D-10929 | H | single bond | Me | Et | C(=O)Me | Cl | CH₂OC(=O)Me | O |
| D-10930 | Me | single bond | H | Et | C(=O)Me | Cl | CH₂OC(=O)Me | O |
| D-10931 | Me(R) | single bond | H | Et | C(=O)Me | Cl | CH₂OC(=O)Me | O |
| D-10932 | Me(S) | single bond | H | Et | C(=O)Me | Cl | CH₂OC(=O)Me | O |
| D-10933 | H | single bond | Me | *c*-Pr | C(=O)Me | Cl | CH₂OC(=O)Me | O |
| D-10934 | Me | single bond | H | *c*-Pr | C(=O)Me | Cl | CH₂OC(=O)Me | O |
| D-10935 | Me(R) | single bond | H | *c*-Pr | C(=O)Me | Cl | CH₂OC(=O)Me | O |
| D-10936 | Me(S) | single bond | H | *c*-Pr | C(=O)Me | Cl | CH₂OC(=O)Me | O |
| 0-10937 | H | single bond | Me | *c*-Pr(1-CN) | C(=O)Me | Cl | CH₂OC(=O)Me | O |
| D-10938 | Me | single bond | H | *c*-Pr(1-CN) | C(=O)Me | Cl | CH₂OC(=O)Me | O |
| D-10939 | Me(R) | single bond | H | *c*-Pr(1-CN) | C(=O)Me | Cl | CH₂OC(=O)Me | O |
| D-10940 | Me(S) | single bond | H | *c*-Pr(1-CN) | C(=O)Me | Cl | CH₂OC(=O)Me | O |
| D-10941 | H | single bond | Me | CH₂CF₃ | C(=O)Me | Me | CH₂OC(=O)Me | O |
| D-10942 | Me | single bond | H | CH₂CF₃ | C(=O)Me | Me | CH₂OC(=O)Me | O |
| D-10943 | Me(R) | single bond | H | CH₂CF₃ | C(=O)Me | Me | CH₂OC(=O)Me | O |
| D-10944 | Me(S) | single bond | H | CH₂CF₃ | C(=O)Me | Me | CH₂OC(=O)Me | O |
| D-10945 | H | single bond | Me | Et | C(=O)Me | Me | CH₂OC(=O)Me | O |
| D-10946 | Me | single bond | H | Et | C(=O)Me | Me | CH₂OC(=O)Me | O |

**[Table 895]**

| Compound Number | Y | W | Z | R¹ | R² | R³ | R¹⁰ | A |
|---|---|---|---|---|---|---|---|---|
| D-10947 | Me(R) | single bond | H | Et | C(=O)Me | Me | CH₂OC(=O)Me | O |
| D-10948 | Me(S) | single bond | H | Et | C(=O)Me | Me | CH₂OC(=O)Me | O |
| D-10949 | H | single bond | Me | *c*-Pr | C(=O)Me | Me | CH₂OC(=O)Me | O |
| D-10950 | Me | single bond | H | *c*-Pr | C(=O)Me | Me | CH₂OC(=O)Me | O |
| D-10951 | Me(R) | single bond | H | *c*-Pr | C(=O)Me | Me | CH₂OC(=O)Me | O |
| D-10952 | Me(S) | single bond | H | *c*-Pr | C(=O)Me | Me | CH₂OC(=O)Me | O |
| D-10953 | H | single bond | Me | *c*-Pr(1-CN) | C(=O)Me | Me | CH₂OC(=O)Me | O |
| D-10954 | Me | single bond | H | *c*-Pr(1-CN) | C(=O)Me | Me | CH₂OC(=O)Me | O |
| D-10955 | Me(R) | single bond | H | *c*-Pr(1-CN) | C(=O)Me | Me | CH₂OC(=O)Me | O |
| D-10956 | Me(S) | single bond | H | *c*-Pr(1-CN) | C(=O)Me | Me | CH₂OC(=O)Me | O |
| 0-10957 | H | single bond | Me | CH₂CF₃ | C(=O)Me | Cl | CH₂OC(=O)(*i*-Pr) | O |
| 0-10958 | Me | single bond | H | CH₂CF₃ | C(=O)Me | Cl | CH₂OC(=O)(*i*-Pr) | O |
| D-10959 | Me(R) | single bond | H | CH₂CF₃ | C(=O)Me | Cl | CH₂OC(=O)(*i*-Pr) | O |
| D-10960 | Me(S) | single bond | H | CH₂CF₃ | C(=O)Me | Cl | CH₂OC(=O)(*i*-Pr) | O |
| D-10961 | H | single bond | Me | Et | C(=O)Me | Cl | CH₂OC(=O)(*i*-Pr) | O |
| D-10962 | Me | single bond | H | Et | C(=O)Me | Cl | CH₂OC(=O)(*i*-Pr) | O |
| D-10963 | Me(R) | single bond | H | Et | C(=O)Me | Cl | CH₂OC(=O)(*i*-Pr) | O |
| D-10964 | Me(S) | single bond | H | Et | C(=O)Me | Cl | CH₂OC(=O)(*i*-Pr) | O |
| D-10965 | H | single bond | Me | *c*-Pr | C(=O)Me | Cl | CH₂OC(=O)(*i*-Pr) | O |
| D-10966 | Me | single bond | H | *c*-Pr | C(=O)Me | Cl | CH₂OC(=O)(*i*-Pr) | O |
| D-10967 | Me(R) | single bond | H | *c*-Pr | C(=O)Me | Cl | CH₂OC(=O)(*i*-Pr) | O |
| D-10968 | Me(S) | single bond | H | *c*-Pr | C(=O)Me | Cl | CH₂OC(=O)(*i*-Pr) | O |
| 0-10969 | H | single bond | Me | *c*-Pr(1-CN) | C(=O)Me | Cl | CH₂OC(=O)(*i*-Pr) | O |
| D-10970 | Me | single bond | H | *c*-Pr(1-CN) | C(=O)Me | Cl | CH₂OC(=O)(*i*-Pr) | O |
| D-10971 | Me(R) | single bond | H | *c*-Pr(1-CN) | C(=O)Me | Cl | CH₂OC(=O)(*i*-Pr) | O |
| D-10972 | Me(S) | single bond | H | *c*-Pr(1-CN) | C(=O)Me | Cl | CH₂OC(=O)(*i*-Pr) | O |
| D-10973 | H | single bond | Me | CH₂CF₃ | C(=O)Me | Me | CH₂OC(=O)(*i*-Pr) | O |
| D-10974 | Me | single bond | H | CH₂CF₃ | C(=O)Me | Me | CH₂OC(=O)(*i*-Pr) | O |
| D-10975 | Me(R) | single bond | H | CH₂CF₃ | C(=O)Me | Me | CH₂OC(=O)(*i*-Pr) | O |
| D-10976 | Me(S) | single bond | H | CH₂CF₃ | C(=O)Me | Me | CH₂OC(=O)(*i*-Pr) | O |
| 0-10977 | H | single bond | Me | Et | C(=O)Me | Me | CH₂OC(=O)(*i*-Pr) | O |
| D-10978 | Me | single bond | H | Et | C(=O)Me | Me | CH₂OC(=O)(*i*-Pr) | O |
| D-10979 | Me(R) | single bond | H | Et | C(=O)Me | Me | CH₂OC(=O)(*i*-Pr) | O |
| D-10980 | Me(S) | single bond | H | Et | C(=O)Me | Me | CH₂OC(=O)(*i*-Pr) | O |
| D-10981 | H | single bond | Me | *c*-Pr | C(=O)Me | Me | CH₂OC(=O)(*i*-Pr) | O |
| D-10982 | Me | single bond | H | *c*-Pr | C(=O)Me | Me | CH₂OC(=O)(*i*-Pr) | O |
| D-10983 | Me(R) | single bond | H | *c*-Pr | C(=O)Me | Me | CH₂OC(=O)(*i*-Pr) | O |
| D-10984 | Me(S) | single bond | H | *c*-Pr | C(=O)Me | Me | CH₂OC(=O)(*i*-Pr) | O |
| D-10985 | H | single bond | Me | *c*-Pr(1-CN) | C(=O)Me | Me | CH₂OC(=O)(*i*-Pr) | O |
| D-10986 | Me | single bond | H | *c*-Pr(1-CN) | C(=O)Me | Me | CH₂OC(=O)(*i*-Pr) | O |
| D-10987 | Me(R) | single bond | H | *c*-Pr(1-CN) | C(=O)Me | Me | CH₂OC(=O)(*i*-Pr) | O |
| D-10988 | Me(S) | single bond | H | *c*-Pr(1-CN) | C(=O)Me | Me | CH₂OC(=O)(*i*-Pr) | O |
| D-10989 | H | single bond | Me | CH₂CF₃ | C(=O)Me | Cl | CH₂OC(=O)(*t*-Bu) | O |
| D-10990 | Me | single bond | H | CH₂CF₃ | C(=O)Me | Cl | CH₂OC(=O)(*t*-Bu) | O |

**[Table 896]**

| Compound Number | Y | W | Z | R¹ | R² | R³ | R¹⁰ | A |
|---|---|---|---|---|---|---|---|---|
| D-10991 | Me(R) | single bond | H | CH₂CF₃ | C(=O)Me | Cl | CH₂OC(=O)(*t*-Bu) | O |
| D-10992 | Me(S) | single bond | H | CH₂CF₃ | C(=O)Me | Cl | CH₂OC(=O)(*t*-Bu) | O |
| D-10993 | H | single bond | Me | Et | C(=O)Me | Cl | CH₂OC(=O)(*t*-Bu) | O |
| D-10994 | Me | single bond | H | Et | C(=O)Me | Cl | CH₂OC(=O)(*t*-Bu) | O |
| D-10995 | Me(R) | single bond | H | Et | C(=O)Me | Cl | CH₂OC(=O)(*t*-Bu) | O |
| D-10996 | Me(S) | single bond | H | Et | C(=O)Me | Cl | CH₂OC(=O)(*t*-Bu) | O |
| D-10997 | H | single bond | Me | *c*-Pr | C(=O)Me | Cl | CH₂OC(=O)(*t*-Bu) | O |
| D-10998 | Me | single bond | H | *c*-Pr | C(=O)Me | Cl | CH₂OC(=O)(*t*-Bu) | O |
| D-10999 | Me(R) | single bond | H | *c*-Pr | C(=O)Me | Cl | CH₂OC(=O)(*t*-Bu) | O |
| D-11000 | Me(S) | single bond | H | *c*-Pr | C(=O)Me | Cl | CH₂OC(=O)(*t*-Bu) | O |
| D-11001 | H | single bond | Me | *c*-Pr(1-CN) | C(=O)Me | Cl | CH₂OC(=O)(*t*-Bu) | O |
| D-11002 | Me | single bond | H | *c*-Pr(1-CN) | C(=O)Me | Cl | CH₂OC(=O)(*t*-Bu) | O |
| D-11003 | Me(R) | single bond | H | *c*-Pr(1-CN) | C(=O)Me | Cl | CH₂OC(=O)(*t*-Bu) | O |
| D-11004 | Me(S) | single bond | H | *c*-Pr(1-CN) | C(=O)Me | Cl | CH₂OC(=O)(*t*-Bu) | O |
| D-11005 | H | single bond | Me | CH₂CF₃ | C(=O)Me | Me | CH₂OC(=O)(*t*-Bu) | O |
| D-11006 | Me | single bond | H | CH₂CF₃ | C(=O)Me | Me | CH₂OC(=O)(*t*-Bu) | O |
| D-11007 | Me(R) | single bond | H | CH₂CF₃ | C(=O)Me | Me | CH₂OC(=O)(*t*-Bu) | O |
| D-11008 | Me(S) | single bond | H | CH₂CF₃ | C(=O)Me | Me | CH₂OC(=O)(*t*-Bu) | O |
| D-11009 | H | single bond | Me | Et | C(=O)Me | Me | CH₂OC(=O)(*t*-Bu) | O |
| D-11010 | Me | single bond | H | Et | C(=O)Me | Me | CH₂OC(=O)(*t*-Bu) | O |
| D-11011 | Me(R) | single bond | H | Et | C(=O)Me | Me | CH₂OC(=O)(*t*-Bu) | O |
| D-11012 | Me(S) | single bond | H | Et | C(=O)Me | Me | CH₂OC(=O)(*t*-Bu) | O |
| D-11013 | H | single bond | Me | *c*-Pr | C(=O)Me | Me | CH₂OC(=O)(*t*-Bu) | O |
| D-11014 | Me | single bond | H | *c*-Pr | C(=O)Me | Me | CH₂OC(=O)(*t*-Bu) | O |
| D-11015 | Me(R) | single bond | H | *c*-Pr | C(=O)Me | Me | CH₂OC(=O)(*t*-Bu) | O |
| D-11016 | Me(S) | single bond | H | *c*-Pr | C(=O)Me | Me | CH₂OC(=O)(*t*-Bu) | O |
| D-11017 | H | single bond | Me | *c*-Pr(1-CN) | C(=O)Me | Me | CH₂OC(=O)(*t*-Bu) | O |
| D-11018 | Me | single bond | H | *c*-Pr(1-CN) | C(=O)Me | Me | CH₂OC(=O)(*t*-Bu) | O |
| D-11019 | Me(R) | single bond | H | *c*-Pr(1-CN) | C(=O)Me | Me | CH₂OC(=O)(*t*-Bu) | O |
| D-11020 | Me(S) | single bond | H | *c*-Pr(1-CN) | C(=O)Me | Me | CH₂OC(=O)(*t*-Bu) | O |
| D-11021 | H | single bond | Me | CH₂CF₃ | C(=O)Me | Cl | CH₂OC(=O)OEt | O |
| D-11022 | Me | single bond | H | CH₂CF₃ | C(=O)Me | Cl | CH₂OC(=O)OEt | O |
| D-11023 | Me(R) | single bond | H | CH₂CF₃ | C(=O)Me | Cl | CH₂OC(=O)OEt | O |
| D-11024 | Me(S) | single bond | H | CH₂CF₃ | C(=O)Me | Cl | CH₂OC(=O)OEt | O |
| D-11025 | H | single bond | Me | Et | C(=O)Me | Cl | CH₂OC(=O)OEt | O |
| D-11026 | Me | single bond | H | Et | C(=O)Me | Cl | CH₂OC(=O)OEt | O |
| D-11027 | Me(R) | single bond | H | Et | C(=O)Me | Cl | CH₂OC(=O)OEt | O |
| D-11028 | Me(S) | single bond | H | Et | C(=O)Me | Cl | CH₂OC(=O)OEt | O |
| D-11029 | H | single bond | Me | *c*-Pr | C(=O)Me | Cl | CH₂OC(=O)OEt | O |
| D-11030 | Me | single bond | H | *c*-Pr | C(=O)Me | Cl | CH₂OC(=O)OEt | O |
| D-11031 | Me(R) | single bond | H | *c*-Pr | C(=O)Me | Cl | CH₂OC(=O)OEt | O |
| D-11032 | Me(S) | single bond | H | *c*-Pr | C(=O)Me | Cl | CH₂OC(=O)OEt | O |
| D-11033 | H | single bond | Me | *c*-Pr(1-CN) | C(=O)Me | Cl | CH₂OC(=O)OEt | O |
| D-11034 | Me | single bond | H | *c*-Pr(1-CN) | C(=O)Me | Cl | CH₂OC(=O)OEt | O |

**[Table 897]**

| Compound Number | Y | w | Z | R¹ | R² | R³ | R¹⁰ | A |
|---|---|---|---|---|---|---|---|---|
| D-11035 | Me(R) | single bond | H | *c*-Pr(1-CN) | C(=O)Me | Cl | CH₂OC(=O)OEt | O |
| D-11036 | Me(S) | single bond | H | *c*-Pr(1-CN) | C(=O)Me | Cl | CH₂OC(=O)OEt | O |
| D-11037 | H | single bond | Me | CH₂CF₃ | C(=O)Me | Me | CH₂OC(=O)OEt | O |
| D-11038 | Me | single bond | H | CH₂CF₃ | C(=O)Me | Me | CH₂OC(=O)OEt | O |
| D-11039 | Me(R) | single bond | H | CH₂CF₃ | C(=O)Me | Me | CH₂OC(=O)OEt | O |
| D-11040 | Me(S) | single bond | H | CH₂CF₃ | C(=O)Me | Me | CH₂OC(=O)OEt | O |
| D-11041 | H | single bond | Me | Et | C(=O)Me | Me | CH₂OC(=O)OEt | O |
| D-11042 | Me | single bond | H | Et | C(=O)Me | Me | CH₂OC(=O)OEt | O |
| D-11043 | Me(R) | single bond | H | Et | C(=O)Me | Me | CH₂OC(=O)OEt | O |
| D-11044 | Me(S) | single bond | H | Et | C(=O)Me | Me | CH₂OC(=O)OEt | O |
| D-11045 | H | single bond | Me | *c*-Pr | C(=O)Me | Me | CH₂OC(=O)OEt | O |
| D-11046 | Me | single bond | H | *c*-Pr | C(=O)Me | Me | CH₂OC(=O)OEt | O |
| D-11047 | Me(R) | single bond | H | *c*-Pr | C(=O)Me | Me | CH₂OC(=O)OEt | O |
| D-11048 | Me(S) | single bond | H | *c*-Pr | C(=O)Me | Me | CH₂OC(=O)OEt | O |
| D-11049 | H | single bond | Me | *c*-Pr(1-CN) | C(=O)Me | Me | CH₂OC(=O)OEt | O |
| D-11050 | Me | single bond | H | *c*-Pr(1-CN) | C(=O)Me | Me | CH₂OC(=O)OEt | O |
| D-11051 | Me(R) | single bond | H | *c*-Pr(1-CN) | C(=O)Me | Me | CH₂OC(=O)OEt | O |
| D-11052 | Me(S) | single bond | H | *c*-Pr(1-CN) | C(=O)Me | Me | CH₂OC(=O)OEt | O |
| D-11053 | H | single bond | Me | CH₂CF₃ | C(=O)Me | Cl | CH₂OC(=O)O(*i*-Pr) | O |
| D-11054 | Me | single bond | H | CH₂CF₃ | C(=O)Me | Cl | CH₂OC(=O)O(*i*-Pr) | O |
| D-11055 | Me(R) | single bond | H | CH₂CF₃ | C(=O)Me | Cl | CH₂OC(=O)O(*i*-Pr) | O |
| D-11056 | Me(S) | single bond | H | CH₂CF₃ | C(=O)Me | Cl | CH₂OC(=O)O(*i*-Pr) | O |
| D-11057 | H | single bond | Me | Et | C(=O)Me | Cl | CH₂OC(=O)O(*i*-Pr) | O |
| D-11058 | Me | single bond | H | Et | C(=O)Me | Cl | CH₂OC(=O)O(*i*-Pr) | O |
| D-11059 | Me(R) | single bond | H | Et | C(=O)Me | Cl | CH₂OC(=O)O(*i*-Pr) | O |
| 0-11060 | Me(S) | single bond | H | Et | C(=O)Me | Cl | CH₂OC(=O)O(*i*-Pr) | O |
| D-11061 | H | single bond | Me | *c*-Pr | C(=O)Me | Cl | CH₂OC(=O)O(*i*-Pr) | O |
| D-11062 | Me | single bond | H | *c*-Pr | C(=O)Me | Cl | CH₂OC(=O)O(*i*-Pr) | O |
| D-11063 | Me(R) | single bond | H | *c*-Pr | C(=O)Me | Cl | CH₂OC(=O)O(*i*-Pr) | O |
| D-11064 | Me(S) | single bond | H | *c*-Pr | C(=O)Me | Cl | CH₂OC(=O)O(*i*-Pr) | O |
| D-1 1065 | H | single bond | Me | *c*-Pr(1-CN) | C(=O)Me | Cl | CH₂OC(=O)O(*i*-Pr) | O |
| D-11066 | Me | single bond | H | *c*-Pr(1-CN) | C(=O)Me | Cl | CH₂OC(=O)O(*i*-Pr) | O |
| D-11067 | Me(R) | single bond | H | *c*-Pr(1-CN) | C(=O)Me | Cl | CH₂OC(=O)O(*i*-Pr) | O |
| D-11068 | Me(S) | single bond | H | *c*-Pr(1-CN) | C(=O)Me | Cl | CH₂OC(=O)O(*i*-Pr) | O |
| D-11069 | H | single bond | Me | CH₂CF₃ | C(=O)Me | Me | CH₂OC(=O)O(*i*-Pr) | O |
| D-11070 | Me | single bond | H | CH₂CF₃ | C(=O)Me | Me | CH₂OC(=O)O(*i*-Pr) | O |
| D-11071 | Me(R) | single bond | H | CH₂CF₃ | C(=O)Me | Me | CH₂OC(=O)O(*i*-Pr) | O |
| D-11072 | Me(S) | single bond | H | CH₂CF₃ | C(=O)Me | Me | CH₂OC(=O)O(*i*-Pr) | O |
| D-11073 | H | single bond | Me | Et | C(=O)Me | Me | CH₂OC(=O)O(*i*-Pr) | O |
| D-11074 | Me | single bond | H | Et | C(=O)Me | Me | CH₂OC(=O)O(*i*-Pr) | O |
| D-11075 | Me(R) | single bond | H | Et | C(=O)Me | Me | CH₂OC(=O)O(*i*-Pr) | O |
| D-11076 | Me(S) | single bond | H | Et | C(=O)Me | Me | CH₂OC(=O)O(*i*-Pr) | O |
| D-11077 | H | single bond | Me | *c*-Pr | C(=O)Me | Me | CH₂OC(=O)O(*i*-Pr) | O |
| D-11078 | Me | single bond | H | *c*-Pr | C(=O)Me | Me | CH₂OC(=O)O(*i*-Pr) | O |

**[Table 898]**

| Compound Number | Y | W | Z | R¹ | R² | R³ | R¹⁰ | A |
|---|---|---|---|---|---|---|---|---|
| D-11079 | Me(R) | single bond | H | *c*-Pr | C(=O)Me | Me | CH₂OC(=O)O(*i*-Pr) | O |
| D-11080 | Me(S) | single bond | H | *c*-Pr | C(=O)Me | Me | CH₂OC(=O)O(*i*-Pr) | O |
| D-11081 | H | single bond | Me | *c*-Pr(1-CN) | C(=O)Me | Me | CH₂OC(=O)O(*i*-Pr) | O |
| D-11082 | Me | single bond | H | *c*-Pr(1-CN) | C(=O)Me | Me | CH₂OC(=O)O(*i*-Pr) | O |
| D-11083 | Me(R) | single bond | H | *c*-Pr(1-CN) | C(=O)Me | Me | CH₂OC(=O)O(*i*-Pr) | O |
| D-11084 | Me(S) | single bond | H | *c*-Pr(1-CN) | C(=O)Me | Me | CH₂OC(=O)O(*i*-Pr) | O |
| D-11085 | H | single bond | Me | CH₂CF₃ | C(=O)Me | Cl | CH₂N(Me)C(=O)OMe | O |
| D-11086 | Me | single bond | H | CH₂CF₃ | C(=O)Me | Cl | CH₂N(Me)C(=O)OMe | O |
| D-11087 | Me(R) | single bond | H | CH₂CF₃ | C(=O)Me | Cl | CH₂N(Me)C(=O)OMe | O |
| D-11088 | Me(S) | single bond | H | CH₂CF₃ | C(=O)Me | Cl | CH₂N(Me)C(=O)OMe | O |
| D-11089 | H | single bond | Me | Et | C(=O)Me | Cl | CH₂N(Me)C(=O)OMe | O |
| D-11090 | Me | single bond | H | Et | C(=O)Me | Cl | CH₂N(Me)C(=O)OMe | O |
| D-11091 | Me(R) | single bond | H | Et | C(=O)Me | Cl | CH₂N(Me)C(=O)OMe | O |
| D-11092 | Me(S) | single bond | H | Et | C(=O)Me | Cl | CH₂N(Me)C(=O)OMe | O |
| D-11093 | H | single bond | Me | *c*-Pr | C(=O)Me | Cl | CH₂N(Me)C(=O)OMe | O |
| D-11094 | Me | single bond | H | *c*-Pr | C(=O)Me | Cl | CH₂N(Me)C(=O)OMe | O |
| D-11095 | Me(R) | single bond | H | *c*-Pr | C(=O)Me | Cl | CH₂N(Me)C(=O)OMe | O |
| D-11096 | Me(S) | single bond | H | *c*-Pr | C(=O)Me | Cl | CH₂N(Me)C(=O)OMe | O |
| D-11097 | H | single bond | Me | *c*-Pr(1-CN) | C(=O)Me | Cl | CH₂N(Me)C(=O)OMe | O |
| D-11098 | Me | single bond | H | *c*-Pr(1-CN) | C(=O)Me | Cl | CH₂N(Me)C(=O)OMe | O |
| D-11099 | Me(R) | single bond | H | *c*-Pr(1-CN) | C(=O)Me | Cl | CH₂N(Me)C(=O)OMe | O |
| D-11100 | Me(S) | single bond | H | *c*-Pr(1-CN) | C(=O)Me | Cl | CH₂N(Me)C(=O)OMe | O |
| D-11101 | H | single bond | Me | CH₂CF₃ | C(=O)Me | Me | CH₂N(Me)C(=O)OMe | O |
| D-11102 | Me | single bond | H | CH₂CF₃ | C(=O)Me | Me | CH₂N(Me)C(=O)OMe | O |
| D-11103 | Me(R) | single bond | H | CH₂CF₃ | C(=O)Me | Me | CH₂N(Me)C(=O)OMe | O |
| D-11104 | Me(S) | single bond | H | CH₂CF₃ | C(=O)Me | Me | CH₂N(Me)C(=O)OMe | O |
| D-11105 | H | single bond | Me | Et | C(=O)Me | Me | CH₂N(Me)C(=O)OMe | O |
| D-11106 | Me | single bond | H | Et | C(=O)Me | Me | CH₂N(Me)C(=O)OMe | O |
| D-11107 | Me(R) | single bond | H | Et | C(=O)Me | Me | CH₂N(Me)C(=O)OMe | O |
| D-11108 | Me(S) | single bond | H | Et | C(=O)Me | Me | CH₂N(Me)C(=O)OMe | O |
| D-11109 | H | single bond | Me | *c*-Pr | C(=O)Me | Me | CH₂N(Me)C(=O)OMe | O |
| D-11110 | Me | single bond | H | *c*-Pr | C(=O)Me | Me | CH₂N(Me)C(=O)OMe | O |
| D-11111 | Me(R) | single bond | H | *c*-Pr | C(=O)Me | Me | CH₂N(Me)C(=O)OMe | O |
| D-11112 | Me(S) | single bond | H | *c*-Pr | C(=O)Me | Me | CH₂N(Me)C(=O)OMe | O |
| D-11113 | H | single bond | Me | *c*-Pr(1-CN) | C(=O)Me | Me | CH₂N(Me)C(=O)OMe | O |
| D-11114 | Me | single bond | H | *c*-Pr(1-CN) | C(=O)Me | Me | CH₂N(Me)C(=O)OMe | O |
| D-11115 | Me(R) | single bond | H | *c*-Pr(1-CN) | C(=O)Me | Me | CH₂N(Me)C(=O)OMe | O |
| D-11116 | Me(S) | single bond | H | *c*-Pr(1-CN) | C(=O)Me | Me | CH₂N(Me)C(=O)OMe | O |
| D-11117 | H | single bond | Me | CH₂CF₃ | C(=O)Me | Cl | CH₂OCH₂CH₂OMe | O |
| D-11118 | Me | single bond | H | CH₂CF₃ | C(=O)Me | Cl | CH₂OCH₂CH₂OMe | O |
| D-11119 | Me(R) | single bond | H | CH₂CF₃ | C(=O)Me | Cl | CH₂OCH₂CH₂OMe | O |
| D-11120 | Me(S) | single bond | H | CH₂CF₃ | C(=O)Me | Cl | CH₂OCH₂CH₂OMe | O |
| D-11121 | H | single bond | Me | Et | C(=O)Me | Cl | CH₂OCH₂CH₂OMe | O |
| D-11122 | Me | single bond | H | Et | C(=O)Me | Cl | CH₂OCH₂CH₂OMe | O |

**[Table 899]**

| Compound Number | Y | W | Z | R¹ | R² | R³ | R¹⁰ | A |
|---|---|---|---|---|---|---|---|---|
| D-11123 | Me(R) | single bond | H | Et | C(=O)Me | Cl | CH₂OCH₂CH₂OMe | O |
| D-11124 | Me(S) | single bond | H | Et | C(=O)Me | Cl | CH₂OCH₂CH₂OMe | O |
| D-11125 | H | single bond | Me | *c*-Pr | C(=O)Me | Cl | CH₂OCH₂CH₂OMe | O |
| D-11126 | Me | single bond | H | *c*-Pr | C(=O)Me | Cl | CH₂OCH₂CH₂OMe | O |
| D-11127 | Me(R) | single bond | H | *c*-Pr | C(=O)Me | Cl | CH₂OCH₂CH₂OMe | O |
| D-11128 | Me(S) | single bond | H | *c*-Pr | C(=O)Me | Cl | CH₂OCH₂CH₂OMe | O |
| D-11129 | H | single bond | Me | *c*-Pr(1-CN) | C(=O)Me | Cl | CH₂OCH₂CH₂OMe | O |
| D-11130 | Me | single bond | H | *c*-Pr(1-CN) | C(=O)Me | Cl | CH₂OCH₂CH₂OMe | O |
| D-11131 | Me(R) | single bond | H | *c*-Pr(1-CN) | C(=O)Me | Cl | CH₂OCH₂CH₂OMe | O |
| D-11132 | Me(S) | single bond | H | *c*-Pr(1-CN) | C(=O)Me | Cl | CH₂OCH₂CH₂OMe | O |
| D-11133 | H | single bond | Me | CH₂CF₃ | C(=O)Me | Me | CH₂OCH₂CH₂OMe | O |
| D-11134 | Me | single bond | H | CH₂CF₃ | C(=O)Me | Me | CH₂OCH₂CH₂OMe | O |
| D-11135 | Me(R) | single bond | H | CH₂CF₃ | C(=O)Me | Me | CH₂OCH₂CH₂OMe | O |
| D-11136 | Me(S) | single bond | H | CH₂CF₃ | C(=O)Me | Me | CH₂OCH₂CH₂OMe | O |
| D-11137 | H | single bond | Me | Et | C(=O)Me | Me | CH₂OCH₂CH₂OMe | O |
| D-11138 | Me | single bond | H | Et | C(=O)Me | Me | CH₂OCH₂CH₂OMe | O |
| D-11139 | Me(R) | single bond | H | Et | C(=O)Me | Me | CH₂OCH₂CH₂OMe | O |
| D-11140 | Me(S) | single bond | H | Et | C(=O)Me | Me | CH₂OCH₂CH₂OMe | O |
| D-11141 | H | single bond | Me | *c*-Pr | C(=O)Me | Me | CH₂OCH₂CH₂OMe | O |
| D-11142 | Me | single bond | H | *c*-Pr | C(=O)Me | Me | CH₂OCH₂CH₂OMe | O |
| D-11143 | Me(R) | single bond | H | *c*-Pr | C(=O)Me | Me | CH₂OCH₂CH₂OMe | O |
| D-11144 | Me(S) | single bond | H | *c*-Pr | C(=O)Me | Me | CH₂OCH₂CH₂OMe | O |
| D-11145 | H | single bond | Me | *c*-Pr(1-CN) | C(=O)Me | Me | CH₂OCH₂CH₂OMe | O |
| D-11146 | Me | single bond | H | *c*-Pr(1-CN) | C(=O)Me | Me | CH₂OCH₂CH₂OMe | O |
| D-11147 | Me(R) | single bond | H | *c*-Pr(1-CN) | C(=O)Me | Me | CH₂OCH₂CH₂OMe | O |
| D-11148 | Me(S) | single bond | H | *c*-Pr(1-CN) | C(=O)Me | Me | CH₂OCH₂CH₂OMe | O |
| D-11149 | H | single bond | Me | CH₂CF₃ | C(=O)Me | Cl | CH₂OCH₂CH₂CN | O |
| D-11150 | Me | single bond | H | CH₂CF₃ | C(=O)Me | Cl | CH₂OCH₂CH₂CN | O |
| D-11151 | Me(R) | single bond | H | CH₂CF₃ | C(=O)Me | Cl | CH₂OCH₂CH₂CN | O |
| D-11152 | Me(S) | single bond | H | CH₂CF₃ | C(=O)Me | Cl | CH₂OCH₂CH₂CN | O |
| D-11153 | H | single bond | Me | Et | C(=O)Me | Cl | CH₂OCH₂CH₂CN | O |
| D-11154 | Me | single bond | H | Et | C(=O)Me | Cl | CH₂OCH₂CH₂CN | O |
| D-11155 | Me(R) | single bond | H | Et | C(=O)Me | Cl | CH₂OCH₂CH₂CN | O |
| D-11156 | Me(S) | single bond | H | Et | C(=O)Me | Cl | CH₂OCH₂CH₂CN | O |
| D-11157 | H | single bond | Me | *c*-Pr | C(=O)Me | Cl | CH₂OCH₂CH₂CN | O |
| D-11158 | Me | single bond | H | *c*-Pr | C(=O)Me | Cl | CH₂OCH₂CH₂CN | O |
| D-11159 | Me(R) | single bond | H | *c*-Pr | C(=O)Me | Cl | CH₂OCH₂CH₂CN | O |
| D-11160 | Me(S) | single bond | H | *c*-Pr | C(=O)Me | Cl | CH₂OCH₂CH₂CN | O |
| D-11161 | H | single bond | Me | *c*-Pr(1-CN) | C(=O)Me | Cl | CH₂OCH₂CH₂CN | O |
| D-11162 | Me | single bond | H | *c*-Pr(1-CN) | C(=O)Me | Cl | CH₂OCH₂CH₂CN | O |
| D-11163 | Me(R) | single bond | H | *c*-Pr(1-CN) | C(=O)Me | Cl | CH₂OCH₂CH₂CN | O |
| D-11164 | Me(S) | single bond | H | *c*-Pr(1-CN) | C(=O)Me | Cl | CH₂OCH₂CH₂CN | O |
| D-1 1165 | H | single bond | Me | CH₂CF₃ | C(=O)Me | Me | CH₂OCH₂CH₂CN | O |
| D-11166 | Me | single bond | H | CH₂CF₃ | C(=O)Me | Me | CH₂OCH₂CH₂CN | O |

**[Table 900]**

| Compound Number | Y | W | Z | R¹ | R² | R³ | R¹⁰ | A |
|---|---|---|---|---|---|---|---|---|
| D-11167 | Me(R) | single bond | H | CH₂CF₃ | C(=O)Me | Me | CH₂OCH₂CH₂CN | O |
| D-11168 | Me(S) | single bond | H | CH₂CF₃ | C(=O)Me | Me | CH₂OCH₂CH₂CN | O |
| D-11169 | H | single bond | Me | Et | C(=O)Me | Me | CH₂OCH₂CH₂CN | O |
| D-11170 | Me | single bond | H | Et | C(=O)Me | Me | CH₂OCH₂CH₂CN | O |
| D-11171 | Me(R) | single bond | H | Et | C(=O)Me | Me | CH₂OCH₂CH₂CN | O |
| D-11172 | Me(S) | single bond | H | Et | C(=O)Me | Me | CH₂OCH₂CH₂CN | O |
| D-11173 | H | single bond | Me | *c*-Pr | C(=O)Me | Me | CH₂OCH₂CH₂CN | O |
| D-11174 | Me | single bond | H | *c*-Pr | C(=O)Me | Me | CH₂OCH₂CH₂CN | O |
| D-11175 | Me(R) | single bond | H | *c*-Pr | C(=O)Me | Me | CH₂OCH₂CH₂CN | O |
| D-11176 | Me(S) | single bond | H | *c*-Pr | C(=O)Me | Me | CH₂OCH₂CH₂CN | O |
| D-11177 | H | single bond | Me | *c*-Pr(1-CN) | C(=O)Me | Me | CH₂OCH₂CH₂CN | O |
| D-11178 | Me | single bond | H | *c*-Pr(1-CN) | C(=O)Me | Me | CH₂OCH₂CH₂CN | O |
| D-11179 | Me(R) | single bond | H | *c*-Pr(1-CN) | C(=O)Me | Me | CH₂OCH₂CH₂CN | O |
| D-11180 | Me(S) | single bond | H | *c*-Pr(1-CN) | C(=O)Me | Me | CH₂OCH₂CH₂CN | O |
| D-11181 | H | single bond | Me | CH₂CF₃ | C(=O)Me | Cl | CH₂OCH₂CH₂S(=O)₂Me | O |
| D-11182 | Me | single bond | H | CH₂CF₃ | C(=O)Me | Cl | CH₂OCH₂CH₂S(=O)₂Me | O |
| D-11183 | Me(R) | single bond | H | CH₂CF₃ | C(=O)Me | Cl | CH₂OCH₂CH₂S(=O)₂Me | O |
| D-11184 | Me(S) | single bond | H | CH₂CF₃ | C(=O)Me | Cl | CH₂OCH₂CH₂S(=O)₂Me | O |
| D-11185 | H | single bond | Me | Et | C(=O)Me | Cl | CH₂OCH₂CH₂S(=O)₂Me | O |
| D-11186 | Me | single bond | H | Et | C(=O)Me | Cl | CH₂OCH₂CH₂S(=O)₂Me | O |
| D-11187 | Me(R) | single bond | H | Et | C(=O)Me | Cl | CH₂OCH₂CH₂S(=O)₂Me | O |
| D-11188 | Me(S) | single bond | H | Et | C(=O)Me | Cl | CH₂OCH₂CH₂S(=O)₂Me | O |
| D-11189 | H | single bond | Me | *c*-Pr | C(=O)Me | Cl | CH₂OCH₂CH₂S(=O)₂Me | O |
| D-11190 | Me | single bond | H | *c*-Pr | C(=O)Me | Cl | CH₂OCH₂CH₂S(=O)₂Me | O |
| D-11191 | Me(R) | single bond | H | *c*-Pr | C(=O)Me | Cl | CH₂OCH₂CH₂S(=O)₂Me | O |
| D-11192 | Me(S) | single bond | H | *c*-Pr | C(=O)Me | Cl | CH₂OCH₂CH₂S(=O)₂Me | O |
| D-11193 | H | single bond | Me | *c*-Pr(1-CN) | C(=O)Me | Cl | CH₂OCH₂CH₂S(=O)₂Me | O |
| D-11194 | Me | single bond | H | *c*-Pr(1-CN) | C(=O)Me | Cl | CH₂OCH₂CH₂S(=O)₂Me | O |
| D-11195 | Me(R) | single bond | H | *c*-Pr(1-CN) | C(=O)Me | Cl | CH₂OCH₂CH₂S(=O)₂Me | O |
| D-11196 | Me(S) | single bond | H | *c*-Pr(1-CN) | C(=O)Me | Cl | CH₂OCH₂CH₂S(=O)₂Me | O |
| D-11197 | H | single bond | Me | CH₂CF₃ | C(=O)Me | Me | CH₂OCH₂CH₂S(=O)₂Me | O |
| D-11198 | Me | single bond | H | CH₂CF₃ | C(=O)Me | Me | CH₂OCH₂CH₂S(=O)₂Me | O |
| D-11199 | Me(R) | single bond | H | CH₂CF₃ | C(=O)Me | Me | CH₂OCH₂CH₂S(=O)₂Me | O |
| D-11200 | Me(S) | single bond | H | CH₂CF₃ | C(=O)Me | Me | CH₂OCH₂CH₂S(=O)₂Me | O |
| D-11201 | H | single bond | Me | Et | C(=O)Me | Me | CH₂OCH₂CH₂S(=O)₂Me | O |
| D-11202 | Me | single bond | H | Et | C(=O)Me | Me | CH₂OCH₂CH₂S(=O)₂Me | O |
| D-11203 | Me(R) | single bond | H | Et | C(=O)Me | Me | CH₂OCH₂CH₂S(=O)₂Me | O |
| D-11204 | Me(S) | single bond | H | Et | C(=O)Me | Me | CH₂OCH₂CH₂S(=O)₂Me | O |
| D-11205 | H | single bond | Me | *c*-Pr | C(=O)Me | Me | CH₂OCH₂CH₂S(=O)₂Me | O |
| D-11206 | Me | single bond | H | *c*-Pr | C(=O)Me | Me | CH₂OCH₂CH₂S(=O)₂Me | O |
| D-11207 | Me(R) | single bond | H | *c*-Pr | C(=O)Me | Me | CH₂OCH₂CH₂S(=O)₂Me | O |
| D-11208 | Me(S) | single bond | H | *c*-Pr | C(=O)Me | Me | CH₂OCH₂CH₂S(=O)₂Me | O |
| D-11209 | H | single bond | Me | *c*-Pr(1-CN) | C(=O)Me | Me | CH₂OCH₂CH₂S(=O)₂Me | O |
| D-11210 | Me | single bond | H | *c*-Pr(1-CN) | C(=O)Me | Me | CH₂OCH₂CH₂S(=O)₂Me | O |

**[Table 901]**

| Compound Number | Y | W | Z | R¹ | R² | R³ | R¹⁰ | A |
|---|---|---|---|---|---|---|---|---|
| D-11211 | Me(R) | single bond | H | *c*-Pr(1-CN) | C(=O)Me | Me | CH₂OCH₂CH₂S(=O)₂Me | O |
| D-11212 | Me(S) | single bond | H | *c*-Pr(1-CN) | C(=O)Me | Me | CH₂OCH₂CH₂S(=O)₂Me | O |
| D-11213 | H | single bond | Me | CH₂CF₃ | C(=O)Me | Cl | CH₂(5-Me-1,3-dioxol-2-one-4-yl) | O |
| D-11214 | Me | single bond | H | CH₂CF₃ | C(=O)Me | Cl | CH₂(5-Me-1,3-dioxol-2-one-4-yl) | O |
| D-11215 | Me(R) | single bond | H | CH₂CF₃ | C(=O)Me | Cl | CH₂(5-Me-1,3-dioxol-2-one-4-yl) | O |
| D-11216 | Me(S) | single bond | H | CH₂CF₃ | C(=O)Me | Cl | CH₂(5-Me-1,3-dioxol-2-one-4-yl) | O |
| D-11217 | H | single bond | Me | Et | C(=O)Me | Cl | CH₂(5-Me-1,3-dioxol-2-one-4-yl) | O |
| D-11218 | Me | single bond | H | Et | C(=O)Me | Cl | CH₂(5-Me-1,3-dioxol-2-one-4-yl) | O |
| D-11219 | Me(R) | single bond | H | Et | C(=O)Me | Cl | CH₂(5-Me-1,3-dioxol-2-one-4-yl) | O |
| D-11220 | Me(S) | single bond | H | Et | C(=O)Me | Cl | CH₂(5-Me-1,3-dioxol-2-one-4-yl) | O |
| D-11221 | H | single bond | Me | *c*-Pr | C(=O)Me | Cl | CH₂(5-Me-1,3-dioxol-2-one-4-yl) | O |
| D-11222 | Me | single bond | H | *c*-Pr | C(=O)Me | Cl | CH₂(5-Me-1,3-dioxol-2-one-4-yl) | O |
| D-11223 | Me(R) | single bond | H | *c*-Pr | C(=O)Me | Cl | CH₂(5-Me-1,3-dioxol-2-one-4-yl) | O |
| D-11224 | Me(S) | single bond | H | *c*-Pr | C(=O)Me | Cl | CH₂(5-Me-1,3-dioxol-2-one-4-yl) | O |
| D-11225 | H | single bond | Me | *c*-Pr(1-CN) | C(=O)Me | Cl | CH₂(5-Me-1,3-dioxol-2-one-4-yl) | O |
| D-11226 | Me | single bond | H | *c*-Pr(1-CN) | C(=O)Me | Cl | CH₂(5-Me-1,3-dioxol-2-one-4-yl) | O |
| D-11227 | Me(R) | single bond | H | *c*-Pr(1-CN) | C(=O)Me | Cl | CH₂(5-Me-1,3-dioxol-2-one-4-yl) | O |
| D-11228 | Me(S) | single bond | H | *c*-Pr(1-CN) | C(=O)Me | Cl | CH₂(5-Me-1,3-dioxol-2-one-4-yl) | O |
| D-11229 | H | single bond | Me | CH₂CF₃ | C(=O)Me | Me | CH₂(5-Me-1,3-dioxol-2-one-4-yl) | O |
| D-11230 | Me | single bond | H | CH₂CF₃ | C(=O)Me | Me | CH₂(5-Me-1,3-dioxol-2-one-4-yl) | O |
| D-11231 | Me(R) | single bond | H | CH₂CF₃ | C(=O)Me | Me | CH₂(5-Me-1,3-dioxol-2-one-4-yl) | O |
| D-11232 | Me(S) | single bond | H | CH₂CF₃ | C(=O)Me | Me | CH₂(5-Me-1,3-dioxol-2-one-4-yl) | O |
| D-11233 | H | single bond | Me | Et | C(=O)Me | Me | CH₂(5-Me-1,3-dioxol-2-one-4-yl) | O |
| D-11234 | Me | single bond | H | Et | C(=O)Me | Me | CH₂(5-Me-1,3-dioxol-2-one-4-yl) | O |
| D-11235 | Me(R) | single bond | H | Et | C(=O)Me | Me | CH₂(5-Me-1,3-dioxol-2-one-4-yl) | O |
| D-11236 | Me(S) | single bond | H | Et | C(=O)Me | Me | CH₂(5-Me-1,3-dioxol-2-one-4-yl) | O |
| D-11237 | H | single bond | Me | *c*-Pr | C(=O)Me | Me | CH₂(5-Me-1,3-dioxol-2-one-4-yl) | O |
| D-11238 | Me | single bond | H | *c*-Pr | C(=O)Me | Me | CH₂(5-Me-1,3-dioxol-2-one-4-yl) | O |
| D-11239 | Me(R) | single bond | H | *c*-Pr | C(=O)Me | Me | CH₂(5-Me-1,3-dioxol-2-one-4-yl) | O |
| D-11240 | Me(S) | single bond | H | *c*-Pr | C(=O)Me | Me | CH₂(5-Me-1,3-dioxol-2-one-4-yl) | O |
| D-11241 | H | single bond | Me | *c*-Pr(1-CN) | C(=O)Me | Me | CH₂(5-Me-1,3-dioxol-2-one-4-yl) | O |
| D-11242 | Me | single bond | H | *c*-Pr(1-CN) | C(=O)Me | Me | CH₂(5-Me-1,3-dioxol-2-one-4-yl) | O |
| D-11243 | Me(R) | single bond | H | *c*-Pr(1-CN) | C(=O)Me | Me | CH₂(5-Me-1,3-dioxol-2-one-4-yl) | O |
| D-11244 | Me(S) | single bond | H | *c*-Pr(1-CN) | C(=O)Me | Me | CH₂(5-Me-1,3-dioxol-2-one-4-yl) | O |

**[Table 902]**

| 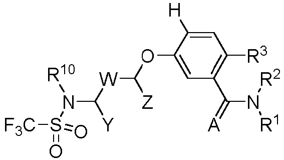 | | | | | | | | |
|---|---|---|---|---|---|---|---|---|
| Compound Number | Y | W | Z | R¹ | R² | R³ | R¹⁰ | A |
| D-11245 | H | single bond | Me | Me | H | Cl | H | O |
| D-11246 | Me | single bond | H | Me | H | Cl | H | O |
| D-11247 | Me(R) | single bond | H | Me | H | Cl | H | O |
| D-11248 | Me(S) | single bond | H | Me | H | Cl | H | O |
| D-11249 | H | single bond | Me | Me | H | Me | H | O |
| D-11250 | Me | single bond | H | Me | H | Me | H | O |
| D-11251 | Me(R) | single bond | H | Me | H | Me | H | O |
| D-11252 | Me(S) | single bond | H | Me | H | Me | H | O |
| D-11253 | H | single bond | Me | CH₂CHF₂ | H | Cl | H | O |
| D-11254 | Me | single bond | H | CH₂CHF₂ | H | Cl | H | O |
| D-11255 | Me(R) | single bond | H | CH₂CHF₂ | H | Cl | H | O |
| D-11256 | Me(S) | single bond | H | CH₂CHF₂ | H | Cl | H | O |
| D-11257 | H | single bond | Me | CH₂CHF₂ | H | Me | H | O |
| D-11258 | Me | single bond | H | CH₂CHF₂ | H | Me | H | O |
| D-11259 | Me(R) | single bond | H | CH₂CHF₂ | H | Me | H | O |
| D-11260 | Me(S) | single bond | H | CH₂CHF₂ | H | Me | H | O |
| D-11261 | H | single bond | Me | *n*-Pr | H | Cl | H | O |
| D-11262 | Me | single bond | H | *n*-Pr | H | Cl | H | O |
| D-11263 | Me(R) | single bond | H | *n*-Pr | H | Cl | H | O |
| D-11264 | Me(S) | single bond | H | *n*-Pr | H | Cl | H | O |
| D-11265 | H | single bond | Me | *n*-Pr | H | Me | H | O |
| D-11266 | Me | single bond | H | *n*-Pr | H | Me | H | O |
| D-11267 | Me(R) | single bond | H | *n*-Pr | H | Me | H | O |
| D-11268 | Me(S) | single bond | H | *n*-Pr | H | Me | H | O |
| D-11269 | H | single bond | Me | *i*-Pr | H | Cl | H | O |
| D-11270 | Me | single bond | H | *i*-Pr | H | Cl | H | O |
| D-11271 | Me(R) | single bond | H | *i*-Pr | H | Cl | H | O |
| D-11272 | Me(S) | single bond | H | *i*-Pr | H | Cl | H | O |
| D-11273 | H | single bond | Me | *i*-Pr | H | Me | H | O |
| D-11274 | Me | single bond | H | *i*-Pr | H | Me | H | O |
| D-11275 | Me(R) | single bond | H | *i*-Pr | H | Me | H | O |
| D-11276 | Me(S) | single bond | H | *i*-Pr | H | Me | H | O |
| D-11277 | H | single bond | Me | *c*-Pr(1-F) | H | Cl | H | O |
| D-11278 | Me | single bond | H | *c*-Pr(1-F) | H | Cl | H | O |
| D-11279 | Me(R) | single bond | H | *c*-Pr(1-F) | H | Cl | H | O |
| D-11280 | Me(S) | single bond | H | *c*-Pr(1-F) | H | Cl | H | O |
| D-11281 | H | single bond | Me | *c*-Pr(1-F) | H | Me | H | O |
| D-11282 | Me | single bond | H | *c*-Pr(1-F) | H | Me | H | O |

**[Table 903]**

| Compound Number | Y | W | Z | R¹ | R² | R³ | R¹⁰ | A |
|---|---|---|---|---|---|---|---|---|
| D-11283 | Me(R) | single bond | H | *c*-Pr(1-F) | H | Me | H | O |
| D-11284 | Me(S) | single bond | H | *c*-Pr(1-F) | H | Me | H | O |
| D-11285 | H | single bond | Me | *c*-Pr(2,2-F₂) | H | Cl | H | O |
| D-11286 | Me | single bond | H | *c*-Pr(2,2-F₂) | H | Cl | H | O |
| D-11287 | Me(R) | single bond | H | *c*-Pr(2,2-F₂) | H | Cl | H | O |
| D-11288 | Me(S) | single bond | H | *c*-Pr(2,2-F₂) | H | Cl | H | O |
| D-11289 | H | single bond | Me | *c*-Pr(2,2-F₂) | H | Me | H | O |
| D-11290 | Me | single bond | H | *c*-Pr(2,2-F₂) | H | Me | H | O |
| D-11291 | Me(R) | single bond | H | *c*-Pr(2,2-F₂) | H | Me | H | O |
| D-11292 | Me(S) | single bond | H | *c*-Pr(2,2-F₂) | H | Me | H | O |
| D-11293 | H | single bond | Me | *c*-Pr(1-Cl) | H | Cl | H | O |
| D-11294 | Me | single bond | H | *c*-Pr(1-Cl) | H | Cl | H | O |
| D-11295 | Me(R) | single bond | H | *c*-Pr(1-Cl) | H | Cl | H | O |
| D-11296 | Me(S) | single bond | H | *c*-Pr(1-Cl) | H | Cl | H | O |
| D-11297 | H | single bond | Me | *c*-Pr(1-Cl) | H | Me | H | O |
| D-11298 | Me | single bond | H | *c*-Pr(1-Cl) | H | Me | H | O |
| D-11299 | Me(R) | single bond | H | *c*-Pr(1-Cl) | H | Me | H | O |
| D-11300 | Me(S) | single bond | H | *c*-Pr(1-Cl) | H | Me | H | O |
| D-11301 | H | single bond | Me | *c*-Pr(1-Br) | H | Cl | H | O |
| D-11302 | Me | single bond | H | *c*-Pr(1-Br) | H | Cl | H | O |
| D-11303 | Me(R) | single bond | H | *c*-Pr(1-Br) | H | Cl | H | O |
| D-11304 | Me(S) | single bond | H | *c*-Pr(1-Br) | H | Cl | H | O |
| D-11305 | H | single bond | Me | *c*-Pr(1-Br) | H | Me | H | O |
| D-11306 | Me | single bond | H | *c*-Pr(1-Br) | H | Me | H | O |
| D-11307 | Me(R) | single bond | H | *c*-Pr(1-Br) | H | Me | H | O |
| D-11308 | Me(S) | single bond | H | *c*-Pr(1-Br) | H | Me | H | O |
| D-11309 | H | single bond | Me | *c*-Pr(1-Me) | H | Cl | H | O |
| D-11310 | Me | single bond | H | *c*-Pr(1-Me) | H | Cl | H | O |
| D-11311 | Me(R) | single bond | H | *c*-Pr(1-Me) | H | Cl | H | O |
| D-11312 | Me(S) | single bond | H | *c*-Pr(1-Me) | H | Cl | H | O |
| D-11313 | H | single bond | Me | *c*-Pr(1-Me) | H | Me | H | O |
| D-11314 | Me | single bond | H | *c*-Pr(1-Me) | H | Me | H | O |
| D-11315 | Me(R) | single bond | H | *c*-Pr(1-Me) | H | Me | H | O |
| D-11316 | Me(S) | single bond | H | *c*-Pr(1-Me) | H | Me | H | O |
| D-11317 | H | single bond | Me | *c*-Pr(1-CH₂OH) | H | Cl | H | O |
| D-11318 | Me | single bond | H | *c*-Pr(1-CH₂OH) | H | Cl | H | O |
| D-11319 | Me(R) | single bond | H | *c*-Pr(1-CH₂OH) | H | Cl | H | O |
| D-11320 | Me(S) | single bond | H | *c*-Pr(1-CH₂OH) | H | Cl | H | O |
| D-11321 | H | single bond | Me | *c*-Pr(1-CH₂OH) | H | Me | H | O |
| D-11322 | Me | single bond | H | *c*-Pr(1-CH₂OH) | H | Me | H | O |
| D-11323 | Me(R) | single bond | H | *c*-Pr(1-CH₂OH) | H | Me | H | O |
| D-11324 | Me(S) | single bond | H | *c*-Pr(1-CH₂OH) | H | Me | H | O |
| D-11325 | H | single bond | Me | *c*-Pr[1-CH₂OS(=O)₂Me] | H | Cl | H | O |
| D-11326 | Me | single bond | H | *c*-Pr[1-CH₂OS(=O)₂Me] | H | Cl | H | O |

**[Table 904]**

| Compound Number | Y | W | Z | R¹ | R² | R³ | R¹⁰ | A |
|---|---|---|---|---|---|---|---|---|
| D-11327 | Me(R) | single bond | H | *c*-Pr[1-CH₂OS(=O)₂Me] | H | Cl | H | O |
| D-11328 | Me(S) | single bond | H | *c*-Pr[1-CH₂OS(=O)₂Me] | H | Cl | H | O |
| D-11329 | H | single bond | Me | *c*-Pr[1-CH₂OS(=O)₂Me] | H | Me | H | O |
| D-11330 | Me | single bond | H | *c*-Pr[1-CH₂OS(=O)₂Me] | H | Me | H | O |
| D-11331 | Me(R) | single bond | H | *c*-Pr[1-CH₂OS(=O)₂Me] | H | Me | H | O |
| D-11332 | Me(S) | single bond | H | *c*-Pr[1-CH₂OS(=O)₂Me] | H | Me | H | O |
| D-11333 | H | single bond | Me | *c*-Pr(1-CH₂OMe) | H | Cl | H | O |
| D-11334 | Me | single bond | H | *c*-Pr(1-CH₂OMe) | H | Cl | H | O |
| D-11335 | Me(R) | single bond | H | *c*-Pr(1-CH₂OMe) | H | Cl | H | O |
| D-11336 | Me(S) | single bond | H | *c*-Pr(1-CH₂OMe) | H | Cl | H | O |
| D-11337 | H | single bond | Me | *c*-Pr(1-CH₂OMe) | H | Me | H | O |
| D-11338 | Me | single bond | H | *c*-Pr(1-CH₂OMe) | H | Me | H | O |
| D-11339 | Me(R) | single bond | H | *c*-Pr(1-CH₂OMe) | H | Me | H | O |
| D-11340 | Me(S) | single bond | H | *c*-Pr(1-CH₂OMe) | H | Me | H | O |
| D-11341 | H | single bond | Me | *c*-Pr(1-CH₂OCN) | H | Cl | H | O |
| D-11342 | Me | single bond | H | *c*-Pr(1-CH₂OCN) | H | Cl | H | O |
| D-11343 | Me(R) | single bond | H | *c*-Pr(1-CH₂OCN) | H | Cl | H | O |
| D-11344 | Me(S) | single bond | H | *c*-Pr(1-CH₂OCN) | H | Cl | H | O |
| D-11345 | H | single bond | Me | *c*-Pr(1-CH₂OCN) | H | Me | H | O |
| D-11346 | Me | single bond | H | *c*-Pr(1-CH₂OCN) | H | Me | H | O |
| D-11347 | Me(R) | single bond | H | *c*-Pr(1-CH₂OCN) | H | Me | H | O |
| D-11348 | Me(S) | single bond | H | *c*-Pr(1-CH₂OCN) | H | Me | H | O |
| D-11349 | H | single bond | Me | *c*-Pr(1-CH₂SMe) | H | Cl | H | O |
| D-11350 | Me | single bond | H | *c*-Pr(1-CH₂SMe) | H | Cl | H | O |
| D-11351 | Me(R) | single bond | H | *c*-Pr(1-CH₂SMe) | H | Cl | H | O |
| D-11352 | Me(S) | single bond | H | *c*-Pr(1-CH₂SMe) | H | Cl | H | O |
| D-11353 | H | single bond | Me | *c*-Pr(1-CH₂SMe) | H | Me | H | O |
| D-11354 | Me | single bond | H | *c*-Pr(1-CH₂SMe) | H | Me | H | O |
| D-11355 | Me(R) | single bond | H | *c*-Pr(1-CH₂SMe) | H | Me | H | O |
| D-11356 | Me(S) | single bond | H | *c*-Pr(1-CH₂SMe) | H | Me | H | O |
| D-11357 | H | single bond | Me | *c*-Pr[1-CH₂S(=O)Me] | H | Cl | H | O |
| D-11358 | Me | single bond | H | *c*-Pr[1-CH₂S(=O)Me] | H | Cl | H | O |
| D-11359 | Me(R) | single bond | H | *c*-Pr[1-CH₂S(=O)Me] | H | Cl | H | O |
| D-11360 | Me(S) | single bond | H | *c*-Pr[1-CH₂S(=O)Me] | H | Cl | H | O |
| D-11361 | H | single bond | Me | *c*-Pr[1-CH₂S(=O)Me] | H | Me | H | O |
| D-11362 | Me | single bond | H | *c*-Pr[1-CH₂S(=O)Me] | H | Me | H | O |
| D-11363 | Me(R) | single bond | H | *c*-Pr[1-CH₂S(=O)Me] | H | Me | H | O |
| D-11364 | Me(S) | single bond | H | *c*-Pr[1-CH₂S(=O)Me] | H | Me | H | O |
| D-11365 | H | single bond | Me | *c*-Pr[1-CH₂S(=O)₂Me] | H | Cl | H | O |
| D-11366 | Me | single bond | H | *c*-Pr[1-CH₂S(=O)₂Me] | H | Cl | H | O |
| D-11367 | Me(R) | single bond | H | *c*-Pr[1-CH₂S(=O)₂Me] | H | Cl | H | O |
| D-11368 | Me(S) | single bond | H | *c*-Pr[1-CH₂S(=O)₂Me] | H | Cl | H | O |
| D-11369 | H | single bond | Me | *c*-Pr[1-CH₂S(=O)₂Me] | H | Me | H | O |
| D-11370 | Me | single bond | H | *c*-Pr[1-CH₂S(=O)₂Me] | H | Me | H | O |

**[Table 905]**

| Compound Number | Y | W | Z | R¹ | R² | R³ | R¹⁰ | A |
|---|---|---|---|---|---|---|---|---|
| D-11371 | Me(R) | single bond | H | *c*-Pr[1-CH₂S(=O)₂Me] | H | Me | H | O |
| D-11372 | Me(S) | single bond | H | *c*-Pr[1-CH₂S(=O)₂Me] | H | Me | H | O |
| D-11373 | H | single bond | Me | *c*-Pr(1-CH₂F) | H | Cl | H | O |
| D-11374 | Me | single bond | H | *c*-Pr(1-CH₂F) | H | Cl | H | O |
| D-11375 | Me(R) | single bond | H | *c*-Pr(1-CH₂F) | H | Cl | H | O |
| D-11376 | Me(S) | single bond | H | *c*-Pr(1-CH₂F) | H | Cl | H | O |
| D-11377 | H | single bond | Me | *c*-Pr(1-CH₂F) | H | Me | H | O |
| D-11378 | Me | single bond | H | *c*-Pr(1-CH₂F) | H | Me | H | O |
| D-11379 | Me(R) | single bond | H | *c*-Pr(1-CH₂F) | H | Me | H | O |
| D-11380 | Me(S) | single bond | H | *c*-Pr(1-CH₂F) | H | Me | H | O |
| D-11381 | H | single bond | Me | *c*-Pr(1-CHF₂) | H | Cl | H | O |
| D-11382 | Me | single bond | H | *c*-Pr(1-CHF₂) | H | Cl | H | O |
| D-11383 | Me(R) | single bond | H | *c*-Pr(1-CHF₂) | H | Cl | H | O |
| D-11384 | Me(S) | single bond | H | *c*-Pr(1-CHF₂) | H | Cl | H | O |
| D-11385 | H | single bond | Me | *c*-Pr(1-CHF₂) | H | Me | H | O |
| D-11386 | Me | single bond | H | *c*-Pr(1-CHF₂) | H | Me | H | O |
| D-11387 | Me(R) | single bond | H | *c*-Pr(1-CHF₂) | H | Me | H | O |
| D-11388 | Me(S) | single bond | H | *c*-Pr(1-CHF₂) | H | Me | H | O |
| D-11389 | H | single bond | Me | *c*-Pr(1-CF₃) | H | Cl | H | O |
| D-11390 | Me | single bond | H | *c*-Pr(1-CF₃) | H | Cl | H | O |
| D-11391 | Me(R) | single bond | H | *c*-Pr(1-CF₃) | H | Cl | H | O |
| D-11392 | Me(S) | single bond | H | *c*-Pr(1-CF₃) | H | Cl | H | O |
| D-11393 | H | single bond | Me | *c*-Pr(1-CF₃) | H | Me | H | O |
| D-11394 | Me | single bond | H | *c*-Pr(1-CF₃) | H | Me | H | O |
| D-11395 | Me(R) | single bond | H | *c*-Pr(1-CF₃) | H | Me | H | O |
| D-11396 | Me(S) | single bond | H | *c*-Pr(1-CF₃) | H | Me | H | O |
| D-11397 | H | single bond | Me | *c*-Pr(1-C≡CH) | H | Cl | H | O |
| D-11398 | Me | single bond | H | *c*-Pr(1-C≡CH) | H | Cl | H | O |
| D-11399 | Me(R) | single bond | H | *c*-Pr(1-C≡CH) | H | Cl | H | O |
| D-11400 | Me(S) | single bond | H | *c*-Pr(1-C≡CH) | H | Cl | H | O |
| D-11401 | H | single bond | Me | *c*-Pr(1-C≡CH) | H | Me | H | O |
| D-11402 | Me | single bond | H | *c*-Pr(1-C≡CH) | H | Me | H | O |
| D-11403 | Me(R) | single bond | H | *c*-Pr(1-C≡CH) | H | Me | H | O |
| D-11404 | Me(S) | single bond | H | *c*-Pr(1-C≡CH) | H | Me | H | O |
| D-11405 | H | single bond | Me | *c*-Pr[1-C(=O)OEt] | H | Cl | H | O |
| D-11406 | Me | single bond | H | *c*-Pr[1-C(=O)OEt] | H | Cl | H | O |
| D-11407 | Me(R) | single bond | H | *c*-Pr[1-C(=O)OEt] | H | Cl | H | O |
| D-11408 | Me(S) | single bond | H | *c*-Pr[1-C(=O)OEt] | H | Cl | H | O |
| D-11409 | H | single bond | Me | *c*-Pr[1-C(=O)OEt] | H | Me | H | O |
| D-11410 | Me | single bond | H | *c*-Pr[1-C(=O)OEt] | H | Me | H | O |
| D-11411 | Me(R) | single bond | H | *c*-Pr[1-C(=O)OEt] | H | Me | H | O |
| D-11412 | Me(S) | single bond | H | *c*-Pr[1-C(=O)OEt] | H | Me | H | O |
| D-11413 | H | single bond | Me | *c*-Pr[1-C(=O)OH] | H | Cl | H | O |
| D-11414 | Me | single bond | H | *c*-Pr[1-C(=O)OH] | H | Cl | H | O |

**[Table 906]**

| Compound Number | Y | W | Z | R¹ | R² | R³ | R¹⁰ | A |
|---|---|---|---|---|---|---|---|---|
| D-11415 | Me(R) | single bond | H | *c*-Pr[1-C(=O)OH] | H | Cl | H | O |
| D-11416 | Me(S) | single bond | H | *c*-Pr[1-C(=O)OH] | H | Cl | H | O |
| D-11417 | H | single bond | Me | *c*-Pr[1-C(=O)OH] | H | Me | H | O |
| D-11418 | Me | single bond | H | *c*-Pr[1-C(=O)OH] | H | Me | H | O |
| D-11419 | Me(R) | single bond | H | *c*-Pr[1-C(=O)OH] | H | Me | H | O |
| D-11420 | Me(S) | single bond | H | *c*-Pr[1-C(=O)OH] | H | Me | H | O |
| D-11421 | H | single bond | Me | *c*-Pr[1-C(=O)NH₂] | H | Cl | H | O |
| D-11422 | Me | single bond | H | *c*-Pr[1-C(=O)NH₂] | H | Cl | H | O |
| D-11423 | Me(R) | single bond | H | *c*-Pr[1-C(=O)NH₂] | H | Cl | H | O |
| D-11424 | Me(S) | single bond | H | *c*-Pr[1-C(=O)NH₂] | H | Cl | H | O |
| D-11425 | H | single bond | Me | *c*-Pr[1-C(=O)NH₂] | H | Me | H | O |
| D-11426 | Me | single bond | H | *c*-Pr[1-C(=O)NH₂] | H | Me | H | O |
| D-11427 | Me(R) | single bond | H | *c*-Pr[1-C(=O)NH₂] | H | Me | H | O |
| D-11428 | Me(S) | single bond | H | *c*-Pr[1-C(=O)NH₂] | H | Me | H | O |
| D-11429 | H | single bond | Me | *c*-Pr(1-OMe) | H | Cl | H | O |
| D-11430 | Me | single bond | H | *c*-Pr(1-OMe) | H | Cl | H | O |
| D-11431 | Me(R) | single bond | H | *c*-Pr(1-OMe) | H | Cl | H | O |
| D-11432 | Me(S) | single bond | H | *c*-Pr(1-OMe) | H | Cl | H | O |
| D-11433 | H | single bond | Me | *c*-Pr(1-OMe) | H | Me | H | O |
| D-11434 | Me | single bond | H | *c*-Pr(1-OMe) | H | Me | H | O |
| D-11435 | Me(R) | single bond | H | *c*-Pr(1-OMe) | H | Me | H | O |
| D-11436 | Me(S) | single bond | H | *c*-Pr(1-OMe) | H | Me | H | O |
| D-11437 | H | single bond | Me | *c*-Pr(1-SMe) | H | Cl | H | O |
| D-11438 | Me | single bond | H | *c*-Pr(1-SMe) | H | Cl | H | O |
| D-11439 | Me(R) | single bond | H | *c*-Pr(1-SMe) | H | Cl | H | O |
| D-11440 | Me(S) | single bond | H | *c*-Pr(1-SMe) | H | Cl | H | O |
| D-11441 | H | single bond | Me | *c*-Pr(1-SMe) | H | Me | H | O |
| D-11442 | Me | single bond | H | *c*-Pr(1-SMe) | H | Me | H | O |
| D-11443 | Me(R) | single bond | H | *c*-Pr(1-SMe) | H | Me | H | O |
| D-11444 | Me(S) | single bond | H | *c*-Pr(1-SMe) | H | Me | H | O |
| D-11445 | H | single bond | Me | *c*-Pr[1-S(=O)Me] | H | Cl | H | O |
| D-11446 | Me | single bond | H | *c*-Pr[1-S(=O)Me] | H | Cl | H | O |
| D-11447 | Me(R) | single bond | H | *c*-Pr[1-S(=O)Me] | H | Cl | H | O |
| D-11448 | Me(S) | single bond | H | *c*-Pr[1-S(=O)Me] | H | Cl | H | O |
| D-11449 | H | single bond | Me | *c*-Pr[1-S(=O)Me] | H | Me | H | O |
| D-11450 | Me | single bond | H | *c*-Pr[1-S(=O)Me] | H | Me | H | O |
| D-11451 | Me(R) | single bond | H | *c*-Pr[1-S(=O)Me] | H | Me | H | O |
| D-11452 | Me(S) | single bond | H | *c*-Pr[1-S(=O)Me] | H | Me | H | O |
| D-11453 | H | single bond | Me | *c*-Pr[1-S(=O)₂Me] | H | Cl | H | O |
| D-11454 | Me | single bond | H | *c*-Pr[1-S(=O)₂Me] | H | Cl | H | O |
| D-11455 | Me(R) | single bond | H | *c*-Pr[1-S(=O)₂Me] | H | Cl | H | O |
| D-11456 | Me(S) | single bond | H | *c*-Pr[1-S(=O)₂Me] | H | Cl | H | O |
| D-11457 | H | single bond | Me | *c*-Pr[1-S(=O)₂Me] | H | Me | H | O |
| D-11458 | Me | single bond | H | *c*-Pr[1-S(=O)₂Me] | H | Me | H | O |

**[Table 907]**

| Compound Number | Y | W | Z | R¹ | R² | R³ | R¹⁰ | A |
|---|---|---|---|---|---|---|---|---|
| D-11459 | Me(R) | single bond | H | *c*-Pr[1-S(=O)₂Me] | H | Me | H | O |
| D-11460 | Me(S) | single bond | H | *c*-Pr[1-S(=O)₂Me] | H | Me | H | O |
| D-11461 | H | single bond | Me | *c*-Pr[1-S(=O)₂CHF₂] | H | Cl | H | O |
| D-11462 | Me | single bond | H | *c*-Pr[1-S(=O)₂CHF₂] | H | Cl | H | O |
| D-11463 | Me(R) | single bond | H | *c*-Pr[1-S(=O)₂CHF₂] | H | Cl | H | O |
| D-11464 | Me(S) | single bond | H | *c*-Pr[1-S(=O)₂CHF₂] | H | Cl | H | O |
| D-11465 | H | single bond | Me | *c*-Pr[1-S(=O)₂CHF₂] | H | Me | H | O |
| D-11466 | Me | single bond | H | *c*-Pr[1-S(=O)₂CHF₂] | H | Me | H | O |
| D-11467 | Me(R) | single bond | H | *c*-Pr[1-S(=O)₂CHF₂] | H | Me | H | O |
| D-11468 | Me(S) | single bond | H | *c*-Pr[1-S(=O)₂CHF₂] | H | Me | H | O |
| D-11469 | H | single bond | Me | *c*-Pr[1-S(=O)₂CF₃] | H | Cl | H | O |
| D-11470 | Me | single bond | H | *c*-Pr[1-S(=O)₂CF₃] | H | Cl | H | O |
| D-11471 | Me(R) | single bond | H | *c*-Pr[1-S(=O)₂CF₃] | H | Cl | H | O |
| D-11472 | Me(S) | single bond | H | *c*-Pr[1-S(=O)₂CF₃] | H | Cl | H | O |
| D-11473 | H | single bond | Me | *c*-Pr[1-S(=O)₂CF₃] | H | Me | H | O |
| D-11474 | Me | single bond | H | *c*-Pr[1-S(=O)₂CF₃] | H | Me | H | O |
| D-11475 | Me(R) | single bond | H | *c*-Pr[1-S(=O)₂CF₃] | H | Me | H | O |
| D-11476 | Me(S) | single bond | H | *c*-Pr[1-S(=O)₂CF₃] | H | Me | H | O |
| D-11477 | H | single bond | Me | *c*-Pr[1-(5,6-dihydro-1,4,2-dioxazin-3-yl)] | H | Cl | H | O |
| D-11478 | Me | single bond | H | *c*-Pr[1-(5,6-dihydro-1 ,4,2-dioxazin-3-yl)] | H | Cl | H | O |
| D-11479 | Me(R) | single bond | H | *c*-Pr[1-(5,6-dihydro-1 ,4,2-dioxazin-3-yl)] | H | Cl | H | O |
| D-11480 | Me(S) | single bond | H | *c*-Pr[1-(5,6-dihydro-1 ,4,2-dioxazin-3-yl)] | H | Cl | H | O |
| D-11481 | H | single bond | Me | *c*-Pr[1-(5,6-dihydro-1 ,4,2-dioxazin-3-yl)] | H | Me | H | O |
| D-11482 | Me | single bond | H | *c*-Pr[1-(5,6-dihydro-1 ,4,2-dioxazin-3-yl)] | H | Me | H | O |
| D-11483 | Me(R) | single bond | H | *c*-Pr[1-(5,6-dihydro-1 ,4,2-dioxazin-3-yl)] | H | Me | H | O |
| D-11484 | Me(S) | single bond | H | *c*-Pr[1-(5,6-dihydro-1 ,4,2-dioxazin-3-yl)] | H | Me | H | O |
| D-11485 | H | single bond | Me | *c*-Pr[1-(5-hydroxy-1,3,4-oxadiazol-2-yl)] | H | Cl | H | O |
| D-11486 | Me | single bond | H | *c*-Pr[1-(5-hydroxy-1,3,4-oxadiazol-2-yl)] | H | Cl | H | O |
| D-11487 | Me(R) | single bond | H | *c*-Pr[1-(5-hydroxy-1,3,4-oxadiazol-2-yl)] | H | Cl | H | O |
| D-11488 | Me(S) | single bond | H | *c*-Pr[1-(5-hydroxy-1,3,4-oxadiazol-2-yl)] | H | Cl | H | O |
| D-11489 | H | single bond | Me | *c*-Pr[1-(5-hydroxy-1,3,4-oxadiazol-2-yl)] | H | Me | H | O |
| D-11490 | Me | single bond | H | *c*-Pr[1-(5-hydroxy-1,3,4-oxadiazol-2-yl)] | H | Me | H | O |
| D-11491 | Me(R) | single bond | H | *c*-Pr[1-(5-hydroxy-1,3,4-oxadiazol-2-yl)] | H | Me | H | O |
| D-11492 | Me(S) | single bond | H | *c*-Pr[1-(5-hydroxy-1,3,4-oxadiazol-2-yl)] | H | Me | H | O |
| D-11493 | H | single bond | Me | *c*-Pr[1-(pyri midin-2-yl)] | H | Cl | H | O |
| D-11494 | Me | single bond | H | *c*-Pr[1-(pyri midin-2-yl)] | H | Cl | H | O |
| D-11495 | Me(R) | single bond | H | *c*-Pr[1-(pyri midin-2-yl)] | H | Cl | H | O |
| D-11496 | Me(S) | single bond | H | *c*-Pr[1-(pyri midin-2-yl)] | H | Cl | H | O |
| D-11497 | H | single bond | Me | *c*-Pr[1-(pyri midin-2-yl)] | H | Me | H | O |
| D-11498 | Me | single bond | H | *c*-Pr[1-(pyri midin-2-yl)] | H | Me | H | O |
| D-11499 | Me(R) | single bond | H | *c*-Pr[1-(pyri midin-2-yl)] | H | Me | H | O |
| D-11500 | Me(S) | single bond | H | *c*-Pr[1-(pyri midin-2-yl)] | H | Me | H | O |
| D-11501 | H | single bond | Me | *t*-Bu | H | Cl | H | O |
| D-11502 | Me | single bond | H | *t*-Bu | H | Cl | H | O |

**[Table 908]**

| Compound Number | Y | W | Z | R¹ | R² | R³ | R¹⁰ | A |
|---|---|---|---|---|---|---|---|---|
| D-11503 | Me(R) | single bond | H | *t*-Bu | H | Cl | H | O |
| D-11504 | Me(S) | single bond | H | *t*-Bu | H | Cl | H | O |
| D-11505 | H | single bond | Me | *t*-Bu | H | Me | H | O |
| D-11506 | Me | single bond | H | *t*-Bu | H | Me | H | O |
| D-11507 | Me(R) | single bond | H | *t*-Bu | H | Me | H | O |
| D-11508 | Me(S) | single bond | H | *t*-Bu | H | Me | H | O |
| D-11509 | H | single bond | Me | *c*-Bu | H | Cl | H | O |
| D-11510 | Me | single bond | H | *c*-Bu | H | Cl | H | O |
| D-11511 | Me(R) | single bond | H | *c*-Bu | H | Cl | H | O |
| D-11512 | Me(S) | single bond | H | *c*-Bu | H | Cl | H | O |
| D-11513 | H | single bond | Me | *c*-Bu | H | Me | H | O |
| D-11514 | Me | single bond | H | *c*-Bu | H | Me | H | O |
| D-11515 | Me(R) | single bond | H | *c*-Bu | H | Me | H | O |
| D-11516 | Me(S) | single bond | H | *c*-Bu | H | Me | H | O |
| D-11517 | H | single bond | Me | *c*-Bu(1-CN) | H | Cl | H | O |
| D-11518 | Me | single bond | H | *c*-Bu(1-CN) | H | Cl | H | O |
| D-11519 | Me(R) | single bond | H | *c*-Bu(1-CN) | H | Cl | H | O |
| D-11520 | Me(S) | single bond | H | *c*-Bu(1-CN) | H | Cl | H | O |
| D-11521 | H | single bond | Me | *c*-Bu(1-CN) | H | Me | H | O |
| D-11522 | Me | single bond | H | *c*-Bu(1-CN) | H | Me | H | O |
| D-11523 | Me(R) | single bond | H | *c*-Bu(1-CN) | H | Me | H | O |
| D-11524 | Me(S) | single bond | H | *c*-Bu(1-CN) | H | Me | H | O |
| D-11525 | H | single bond | Me | *c*-Bu(3,3-F₂) | H | Cl | H | O |
| D-11526 | Me | single bond | H | *c*-Bu(3,3-F₂) | H | Cl | H | O |
| D-11527 | Me(R) | single bond | H | *c*-Bu(3,3-F₂) | H | Cl | H | O |
| D-11528 | Me(S) | single bond | H | *c*-Bu(3,3-F₂) | H | Cl | H | O |
| D-11529 | H | single bond | Me | *c*-Bu(3,3-F₂) | H | Me | H | O |
| D-11530 | Me | single bond | H | *c*-Bu(3,3-F₂) | H | Me | H | O |
| D-11531 | Me(R) | single bond | H | *c*-Bu(3,3-F₂) | H | Me | H | O |
| D-11532 | Me(S) | single bond | H | *c*-Bu(3,3-F₂) | H | Me | H | O |
| D-11533 | H | single bond | Me | oxetan-3-yl | H | Cl | H | O |
| D-11534 | Me | single bond | H | oxetan-3-yl | H | Cl | H | O |
| D-11535 | Me(R) | single bond | H | oxetan-3-yl | H | Cl | H | O |
| D-11536 | Me(S) | single bond | H | oxetan-3-yl | H | Cl | H | O |
| D-11537 | H | single bond | Me | oxetan-3-yl | H | Me | H | O |
| D-11538 | Me | single bond | H | oxetan-3-yl | H | Me | H | O |
| D-11539 | Me(R) | single bond | H | oxetan-3-yl | H | Me | H | O |
| D-11540 | Me(S) | single bond | H | oxetan-3-yl | H | Me | H | O |
| D-11541 | H | single bond | Me | oxetan(3-CN)-3-yl | H | Cl | H | O |
| D-11542 | Me | single bond | H | oxetan(3-CN)-3-yl | H | Cl | H | O |
| D-11543 | Me(R) | single bond | H | oxetan(3-CN)-3-yl | H | Cl | H | O |
| D-11544 | Me(S) | single bond | H | oxetan(3-CN)-3-yl | H | Cl | H | O |
| D-11545 | H | single bond | Me | oxetan(3-CN)-3-yl | H | Me | H | O |
| D-11546 | Me | single bond | H | oxetan(3-CN)-3-yl | H | Me | H | O |

**[Table 909]**

| Compound Number | Y | W | Z | R¹ | R² | R³ | R¹⁰ | A |
|---|---|---|---|---|---|---|---|---|
| D-11547 | Me(R) | single bond | H | oxetan(3-CN)-3-yl | H | Me | H | O |
| D-11548 | Me(S) | single bond | H | oxetan(3-CN)-3-yl | H | Me | H | O |
| D-11549 | H | single bond | Me | azetidin[1-C(=0)Me]-3-yl | H | Cl | H | O |
| D-11550 | Me | single bond | H | azetidin[1-C(=O)Me]-3-yl | H | Cl | H | O |
| D-11551 | Me(R) | single bond | H | azetidin[1-C(=0)Me]-3-yl | H | Cl | H | O |
| D-11552 | Me(S) | single bond | H | azetidin[1-C(=0)Me]-3-yl | H | Cl | H | O |
| D-11553 | H | single bond | Me | azetidin[1-C(=O)Me]-3-yl | H | Me | H | O |
| D-11554 | Me | single bond | H | azetidin[1-C(=0)Me]-3-yl | H | Me | H | O |
| D-11555 | Me(R) | single bond | H | azetidin[1-C(=0)Me]-3-yl | H | Me | H | O |
| D-11556 | Me(S) | single bond | H | azetidin[1-C(=O)Me]-3-yl | H | Me | H | O |
| D-11557 | H | single bond | Me | azetidin[1-S(=O)₂Me]-3-yl | H | Cl | H | O |
| D-11558 | Me | single bond | H | azetidin[1-S(=O)₂Me]-3-yl | H | Cl | H | O |
| D-11559 | Me(R) | single bond | H | azetidin[1-S(=O)₂Me]-3-yl | H | Cl | H | O |
| D-11560 | Me(S) | single bond | H | azetidin[1-S(=O)₂Me]-3-yl | H | Cl | H | O |
| D-11561 | H | single bond | Me | azetidin[1-S(=O)₂Me]-3-yl | H | Me | H | O |
| D-11562 | Me | single bond | H | azetidin[1-S(=O)₂Me]-3-yl | H | Me | H | O |
| D-11563 | Me(R) | single bond | H | azetidin[1-S(=O)₂Me]-3-yl | H | Me | H | O |
| D-11564 | Me(S) | single bond | H | azetidin[1-S(=O)₂Me]-3-yl | H | Me | H | O |
| D-11565 | H | single bond | Me | azetidin[1-S(=O)₂CHF₂]-3-yl | H | Cl | H | O |
| D-11566 | Me | single bond | H | azetidin[1-S(=O)₂CHF₂]-3-yl | H | Cl | H | O |
| D-11567 | Me(R) | single bond | H | azetidin[1-S(=O)₂CHF₂]-3-yl | H | Cl | H | O |
| D-11568 | Me(S) | single bond | H | azetidin[1-S(=O)₂CHF₂]-3-yl | H | Cl | H | O |
| D-11569 | H | single bond | Me | azetidin[1-S(=O)₂CHF₂]-3-yl | H | Me | H | O |
| D-11570 | Me | single bond | H | azetidin[1-S(=O)₂CHF₂]-3-yl | H | Me | H | O |
| D-11571 | Me(R) | single bond | H | azetidin[1-S(=O)₂CHF₂]-3-yl | H | Me | H | O |
| D-11572 | Me(S) | single bond | H | azetidin[1-S(=O)₂CHF₂]-3-yl | H | Me | H | O |
| D-11573 | H | single bond | Me | *c*-Pen | H | Cl | H | O |
| D-11574 | Me | single bond | H | *c*-Pen | H | Cl | H | O |
| D-11575 | Me(R) | single bond | H | *c*-Pen | H | Cl | H | O |
| D-11576 | Me(S) | single bond | H | *c*-Pen | H | Cl | H | O |
| D-11577 | H | single bond | Me | *c*-Pen | H | Me | H | O |
| D-11578 | Me | single bond | H | *c*-Pen | H | Me | H | O |
| D-11579 | Me(R) | single bond | H | *c*-Pen | H | Me | H | O |
| D-11580 | Me(S) | single bond | H | *c*-Pen | H | Me | H | O |
| D-11581 | H | single bond | Me | tetrahydrofuran-3-yl | H | Cl | H | O |
| D-11582 | Me | single bond | H | tetrahydrofuran-3-yl | H | Cl | H | O |
| D-11583 | Me(R) | single bond | H | tetrahydrofuran-3-yl | H | Cl | H | O |
| D-11584 | Me(S) | single bond | H | tetrahydrofuran-3-yl | H | Cl | H | O |
| D-11585 | H | single bond | Me | tetrahydrofuran-3-yl | H | Me | H | O |
| D-11586 | Me | single bond | H | tetrahydrofuran-3-yl | H | Me | H | O |
| D-11587 | Me(R) | single bond | H | tetrahydrofuran-3-yl | H | Me | H | O |
| D-11588 | Me(S) | single bond | H | tetrahydrofuran-3-yl | H | Me | H | O |
| D-11589 | H | single bond | Me | tetrahydrothiophen-3-yl | H | Cl | H | O |
| D-11590 | Me | single bond | H | tetrahydrothiophen-3-yl | H | Cl | H | O |

**[Table 910]**

| Compound Number | Y | W | Z | R¹ | R² | R³ | R¹⁰ | A |
|---|---|---|---|---|---|---|---|---|
| D-11591 | Me(R) | single bond | H | tetrahydrothiophen-3-yl | H | Cl | H | O |
| D-11592 | Me(S) | single bond | H | tetrahydrothiophen-3-yl | H | Cl | H | O |
| D-11593 | H | single bond | Me | tetrahydrothiophen-3-yl | H | Me | H | O |
| D-11594 | Me | single bond | H | tetrahydrothiophen-3-yl | H | Me | H | O |
| D-11595 | Me(R) | single bond | H | tetrahydrothiophen-3-yl | H | Me | H | O |
| D-11596 | Me(S) | single bond | H | tetrahydrothiophen-3-yl | H | Me | H | O |
| D-11597 | H | single bond | Me | *c*-Pen(1-CN) | H | Cl | H | O |
| D-11598 | Me | single bond | H | *c*-Pen(1-CN) | H | Cl | H | O |
| D-11599 | Me(R) | single bond | H | *c*-Pen(1-CN) | H | Cl | H | O |
| D-11600 | Me(S) | single bond | H | *c*-Pen(1-CN) | H | Cl | H | O |
| D-11601 | H | single bond | Me | *c*-Pen(1-CN) | H | Me | H | O |
| D-11602 | Me | single bond | H | *c*-Pen(1-CN) | H | Me | H | O |
| D-11603 | Me(R) | single bond | H | *c-*Pen(1-CN) | H | Me | H | O |
| D-11604 | Me(S) | single bond | H | *c-*Pen(1-CN) | H | Me | H | O |
| D-11605 | H | single bond | Me | tetrahydrofuran(3-CN)-3-yl | H | Cl | H | O |
| D-11606 | Me | single bond | H | tetrahydrofuran(3-CN)-3-yl | H | Cl | H | O |
| D-11607 | Me(R) | single bond | H | tetrahydrofuran(3-CN)-3-yl | H | Cl | H | O |
| D-11608 | Me(S) | single bond | H | tetrahydrofuran(3-CN)-3-yl | H | Cl | H | O |
| D-11609 | H | single bond | Me | tetrahydrofuran(3-CN)-3-yl | H | Me | H | O |
| D-11610 | Me | single bond | H | tetrahydrofuran(3-CN)-3-yl | H | Me | H | O |
| D-11611 | Me(R) | single bond | H | tetrahydrofuran(3-CN)-3-yl | H | Me | H | O |
| D-11612 | Me(S) | single bond | H | tetrahydrofuran(3-CN)-3-yl | H | Me | H | O |
| D-11613 | H | single bond | Me | tetrahydrothiophen(3-CN)-3-yl | H | Cl | H | O |
| D-11614 | Me | single bond | H | tetrahydrothiophen(3-CN)-3-yl | H | Cl | H | O |
| D-11615 | Me(R) | single bond | H | tetrahydrothiophen(3-CN)-3-yl | H | Cl | H | O |
| D-11616 | Me(S) | single bond | H | tetrahydrothiophen(3-CN)-3-yl | H | Cl | H | O |
| D-11617 | H | single bond | Me | tetrahydrothiophen(3-CN)-3-yl | H | Me | H | O |
| D-11618 | Me | single bond | H | tetrahydrothiophen(3-CN)-3-yl | H | Me | H | O |
| D-11619 | Me(R) | single bond | H | tetrahydrothiophen(3-CN)-3-yl | H | Me | H | O |
| D-11620 | Me(S) | single bond | H | tetrahydrothiophen(3-CN)-3-yl | H | Me | H | O |
| D-11621 | H | single bond | Me | bicyclo[1.1.1]pertan-1-yl | H | Cl | H | O |
| D-11622 | Me | single bond | H | bicyclo[1.1.1]pertan-1-yl | H | Cl | H | O |
| D-11623 | Me(R) | single bond | H | bicyclo[1.1.1]pertan-1-yl | H | Cl | H | O |
| D-11624 | Me(S) | single bond | H | bicyclo[1.1.1]pertan-1-yl | H | Cl | H | O |
| D-11625 | H | single bond | Me | bicyclo[1.1.1]pertan-1-yl | H | Me | H | O |
| D-11626 | Me | single bond | H | bicyclo[1.1.1]pertan-1-yl | H | Me | H | O |
| D-11627 | Me(R) | single bond | H | bicyclo[1.1.1]pentan-1-yl | H | Me | H | O |
| D-11628 | Me(S) | single bond | H | bicyclo[1.1.1]pertan-1-yl | H | Me | H | O |
| D-11629 | H | single bond | Me | *c*-Hex | H | Cl | H | O |
| D-11630 | Me | single bond | H | *c -Hex* | H | Cl | H | O |
| D-11631 | Me(R) | single bond | H | *c*-Hex | H | Cl | H | O |
| D-11632 | Me(S) | single bond | H | *c*-Hex | H | Cl | H | O |
| D-11633 | H | single bond | Me | *c*-Hex | H | Me | H | O |
| D-11634 | Me | single bond | H | *c*-Hex | H | Me | H | O |

**[Table 911]**

| Compound Number | Y | W | Z | R¹ | R² | R³ | R¹⁰ | A |
|---|---|---|---|---|---|---|---|---|
| D-11635 | Me(R) | single bond | H | *c*-Hex | H | Me | H | O |
| D-11636 | Me(S) | single bond | H | *c*-Hex | H | Me | H | O |
| D-11637 | H | single bond | Me | tetrahydropyran-4-yl | H | Cl | H | O |
| D-11638 | Me | single bond | H | tetrahydropyran-4-yl | H | Cl | H | O |
| D-11639 | Me(R) | single bond | H | tetrahydropyran-4-yl | H | Cl | H | O |
| D-11640 | Me(S) | single bond | H | tetrahydropyran-4-yl | H | Cl | H | O |
| D-11641 | H | single bond | Me | tetrahydropyran-4-yl | H | Me | H | O |
| D-11642 | Me | single bond | H | tetrahydropyran-4-yl | H | Me | H | O |
| D-11643 | Me(R) | single bond | H | tetrahydropyran-4-yl | H | Me | H | O |
| D-11644 | Me(S) | single bond | H | tetrahydropyran-4-yl | H | Me | H | O |
| D-11645 | H | single bond | Me | tetrahydrothiopyran-4-yl | H | Cl | H | O |
| D-11646 | Me | single bond | H | tetrahydrothiopyran-4-yl | H | Cl | H | O |
| D-11647 | Me(R) | single bond | H | tetrahydrothiopyran-4-yl | H | Cl | H | O |
| D-11648 | Me(S) | single bond | H | tetrahydrothiopyran-4-yl | H | Cl | H | O |
| D-11649 | H | single bond | Me | tetrahydrothiopyran-4-yl | H | Me | H | O |
| D-11650 | Me | single bond | H | tetrahydrothiopyran-4-yl | H | Me | H | O |
| D-11651 | Me(R) | single bond | H | tetrahydrothiopyran-4-yl | H | Me | H | O |
| D-11652 | Me(S) | single bond | H | tetrahydrothiopyran-4-yl | H | Me | H | O |
| D-11653 | H | single bond | Me | *c*-Hex(1-CN) | H | Cl | H | O |
| D-11654 | Me | single bond | H | *c*-Hex(1-CN) | H | Cl | H | O |
| D-11655 | Me(R) | single bond | H | *c*-Hex(1-CN) | H | Cl | H | O |
| D-11656 | Me(S) | single bond | H | *c*-Hex(1-CN) | H | Cl | H | O |
| D-11657 | H | single bond | Me | *c*-Hex(1-CN) | H | Me | H | O |
| D-11658 | Me | single bond | H | *c*-Hex(1-CN) | H | Me | H | O |
| D-11659 | Me(R) | single bond | H | *c*-Hex(1-CN) | H | Me | H | O |
| D-11660 | Me(S) | single bond | H | *c*-Hex(1-CN) | H | Me | H | O |
| D-11661 | H | single bond | Me | tetrahydropyran(4-CN)-4-yl | H | Cl | H | O |
| D-11662 | Me | single bond | H | tetrahydropyran(4-CN)-4-yl | H | Cl | H | O |
| D-11663 | Me(R) | single bond | H | tetrahydropyran(4-CN)-4-yl | H | Cl | H | O |
| D-11664 | Me(S) | single bond | H | tetrahydropyran(4-CN)-4-yl | H | Cl | H | O |
| D-11665 | H | single bond | Me | tetrahydropyran(4-CN)-4-yl | H | Me | H | O |
| D-11666 | Me | single bond | H | tetrahydropyran(4-CN)-4-yl | H | Me | H | O |
| D-11667 | Me(R) | single bond | H | tetrahydropyran(4-CN)-4-yl | H | Me | H | O |
| D-11668 | Me(S) | single bond | H | tetrahydropyran(4-CN)-4-yl | H | Me | H | O |
| D-11669 | H | single bond | Me | tetrahydrothiopyran(4-CN)-4-yl | H | Cl | H | O |
| D-11670 | Me | single bond | H | tetrahydrothiopyran(4-CN)-4-yl | H | Cl | H | O |
| D-11671 | Me(R) | single bond | H | tetrahydrothiopyran(4-CN)-4-yl | H | Cl | H | O |
| D-11672 | Me(S) | single bond | H | tetrahydrothiopyran(4-CN)-4-yl | H | Cl | H | O |
| D-11673 | H | single bond | Me | tetrahydrothiopyran(4-CN)-4-yl | H | Me | H | O |
| D-11674 | Me | single bond | H | tetrahydrothiopyran(4-CN)-4-yl | H | Me | H | O |
| D-11675 | Me(R) | single bond | H | tetrahydrothiopyran(4-CN)-4-yl | H | Me | H | O |
| D-11676 | Me(S) | single bond | H | tetrahydrothiopyran(4-CN)-4-yl | H | Me | H | O |
| D-11677 | H | single bond | Me | CH₂(*c*-Pr) | H | Cl | H | O |
| D-11678 | Me | single bond | H | CH₂(*c*-Pr) | H | Cl | H | O |

**[Table 912]**

| Compound Number | Y | W | Z | R¹ | R² | R³ | R¹⁰ | A |
|---|---|---|---|---|---|---|---|---|
| D-11679 | Me(R) | single bond | H | CH₂(*c*-Pr) | H | Cl | H | O |
| D-11680 | Me(S) | single bond | H | CH₂(*c*-Pr) | H | Cl | H | O |
| D-11681 | H | single bond | Me | CH₂(*c*-Pr) | H | Me | H | O |
| D-11682 | Me | single bond | H | CH₂(*c*-Pr) | H | Me | H | O |
| D-11683 | Me(R) | single bond | H | CH₂(*c*-Pr) | H | Me | H | O |
| D-11684 | Me(S) | single bond | H | CH₂(*c*-Pr) | H | Me | H | O |
| D-11685 | H | single bond | Me | CH₂[*c*-Pr(1-CN)] | H | Cl | H | O |
| D-11686 | Me | single bond | H | CH₂[*c*-Pr(1-CN)] | H | Cl | H | O |
| D-11687 | Me(R) | single bond | H | CH₂[*c*-Pr(1-CN)] | H | Cl | H | O |
| D-11688 | Me(S) | single bond | H | CH₂[*c*-Pr(1-CN)] | H | Cl | H | O |
| D-11689 | H | single bond | Me | CH₂[*c*-Pr(1-CN)] | H | Me | H | O |
| D-11690 | Me | single bond | H | CH₂[*c*-Pr(1-CN)] | H | Me | H | O |
| D-11691 | Me(R) | single bond | H | CH₂[*c*-Pr(1-CN)] | H | Me | H | O |
| D-11692 | Me(S) | single bond | H | CH₂[*c*-Pr(1-CN)] | H | Me | H | O |
| D-11693 | H | single bond | Me | CH(Me)(*c*-Pr) | H | Cl | H | O |
| D-11694 | Me | single bond | H | CH(Me)(*c*-Pr) | H | Cl | H | O |
| D-11695 | Me(R) | single bond | H | CH(Me)(*c*-Pr) | H | Cl | H | O |
| D-11696 | Me(S) | single bond | H | CH(Me)(*c*-Pr) | H | Cl | H | O |
| D-11697 | H | single bond | Me | CH(Me)(*c*-Pr) | H | Me | H | O |
| D-11698 | Me | single bond | H | CH(Me)(*c*-Pr) | H | Me | H | O |
| D-11699 | Me(R) | single bond | H | CH(Me)(*c*-Pr) | H | Me | H | O |
| D-11700 | Me(S) | single bond | H | CH(Me)(*c*-Pr) | H | Me | H | O |
| D-11701 | H | single bond | Me | CH(Me)CH₂OMe | H | Cl | H | O |
| D-11702 | Me | single bond | H | CH(Me)CH₂OMe | H | Cl | H | O |
| D-11703 | Me(R) | single bond | H | CH(Me)CH₂OMe | H | Cl | H | O |
| D-11704 | Me(S) | single bond | H | CH(Me)CH₂OMe | H | Cl | H | O |
| D-11705 | H | single bond | Me | CH(Me)CH₂OMe | H | Me | H | O |
| D-11706 | Me | single bond | H | CH(Me)CH₂OMe | H | Me | H | O |
| D-11707 | Me(R) | single bond | H | CH(Me)CH₂OMe | H | Me | H | O |
| D-11708 | Me(S) | single bond | H | CH(Me)CH₂OMe | H | Me | H | O |
| D-11709 | H | single bond | Me | CH₂CH=CH₂ | H | Cl | H | O |
| D-11710 | Me | single bond | H | CH₂CH=CH₂ | H | Cl | H | O |
| D-11711 | Me(R) | single bond | H | CH₂CH=CH₂ | H | Cl | H | O |
| D-11712 | Me(S) | single bond | H | CH₂CH=CH₂ | H | Cl | H | O |
| D-11713 | H | single bond | Me | CH₂CH=CH₂ | H | Me | H | O |
| D-11714 | Me | single bond | H | CH₂CH=CH₂ | H | Me | H | O |
| D-11715 | Me(R) | single bond | H | CH₂CH=CH₂ | H | Me | H | O |
| D-11716 | Me(S) | single bond | H | CH₂CH=CH₂ | H | Me | H | O |
| D-11717 | H | single bond | Me | CH₂C≡CH | H | Cl | H | O |
| D-11718 | Me | single bond | H | CH₂C≡CH | H | Cl | H | O |
| D-11719 | Me(R) | single bond | H | CH₂C≡CH | H | Cl | H | O |
| D-11720 | Me(S) | single bond | H | CH₂C≡CH | H | Cl | H | O |
| D-11721 | H | single bond | Me | CH₂C≡CH | H | Me | H | O |
| D-11722 | Me | single bond | H | CH₂C≡CH | H | Me | H | O |

**[Table 913]**

| Compound Number | Y | W | Z | R¹ | R² | R³ | R¹⁰ | A |
|---|---|---|---|---|---|---|---|---|
| D-11723 | Me(R) | single bond | H | CH₂C≡CH | H | Me | H | O |
| D-11724 | Me(S) | single bond | H | CH₂C≡CH | H | Me | H | O |
| D-11725 | H | single bond | Me | CH₂CN | H | Cl | H | O |
| D-11726 | Me | single bond | H | CH₂CN | H | Cl | H | O |
| D-11727 | Me(R) | single bond | H | CH₂CN | H | Cl | H | O |
| D-11728 | Me(S) | single bond | H | CH₂CN | H | Cl | H | O |
| D-11729 | H | single bond | Me | CH₂CN | H | Me | H | O |
| D-11730 | Me | single bond | H | CH₂CN | H | Me | H | O |
| D-11731 | Me(R) | single bond | H | CH₂CN | H | Me | H | O |
| D-11732 | Me(S) | single bond | H | CH₂CN | H | Me | H | O |
| D-11733 | H | single bond | Me | CH(Me)CN | H | Cl | H | O |
| D-11734 | Me | single bond | H | CH(Me)CN | H | Cl | H | O |
| D-11735 | Me(R) | single bond | H | CH(Me)CN | H | Cl | H | O |
| D-11736 | Me(S) | single bond | H | CH(Me)CN | H | Cl | H | O |
| D-11737 | H | single bond | Me | CH(Me)CN | H | Me | H | O |
| D-11738 | Me | single bond | H | CH(Me)CN | H | Me | H | O |
| D-11739 | Me(R) | single bond | H | CH(Me)CN | H | Me | H | O |
| D-11740 | Me(S) | single bond | H | CH(Me)CN | H | Me | H | O |
| D-11741 | H | single bond | Me | C(Me)₂CN | H | Cl | H | O |
| D-11742 | Me | single bond | H | C(Me)₂CN | H | Cl | H | O |
| D-11743 | Me(R) | single bond | H | C(Me)₂CN | H | Cl | H | O |
| D-11744 | Me(S) | single bond | H | C(Me)₂CN | H | Cl | H | O |
| D-11745 | H | single bond | Me | C(Me)₂CN | H | Me | H | O |
| D-11746 | Me | single bond | H | C(Me)₂CN | H | Me | H | O |
| D-11747 | Me(R) | single bond | H | C(Me)₂CN | H | Me | H | O |
| D-11748 | Me(S) | single bond | H | C(Me)₂CN | H | Me | H | O |
| D-11749 | H | single bond | Me | C(Me)(*i*-Pr)CN | H | Cl | H | O |
| D-11750 | Me | single bond | H | C(Me)(*i*-Pr)CN | H | Cl | H | O |
| D-11751 | Me(R) | single bond | H | C(Me)(*i*-Pr)CN | H | Cl | H | O |
| D-11752 | Me(S) | single bond | H | C(Me)(*i*-Pr)CN | H | Cl | H | O |
| D-11753 | H | single bond | Me | C(Me)(*i*-Pr)CN | H | Me | H | O |
| D-11754 | Me | single bond | H | C(Me)(*i*-Pr)CN | H | Me | H | O |
| D-11755 | Me(R) | single bond | H | C(Me)(*i*-Pr)CN | H | Me | H | O |
| D-11756 | Me(S) | single bond | H | C(Me)(*i*-Pr)CN | H | Me | H | O |
| D-11757 | H | single bond | Me | OMe | H | Cl | H | O |
| D-11758 | Me | single bond | H | OMe | H | Cl | H | O |
| D-11759 | Me(R) | single bond | H | OMe | H | Cl | H | O |
| D-11760 | Me(S) | single bond | H | OMe | H | Cl | H | O |
| D-11761 | H | single bond | Me | OMe | H | Me | H | O |
| D-11762 | Me | single bond | H | OMe | H | Me | H | O |
| D-11763 | Me(R) | single bond | H | OMe | H | Me | H | O |
| D-11764 | Me(S) | single bond | H | OMe | H | Me | H | O |
| D-11765 | H | single bond | Me | CH=NOMe | H | Cl | H | O |
| D-11766 | Me | single bond | H | CH=NOMe | H | Cl | H | O |

**[Table 914]**

| Compound Number | Y | W | Z | R¹ | R² | R³ | R¹⁰ | A |
|---|---|---|---|---|---|---|---|---|
| D-11767 | Me(R) | single bond | H | CH=NOMe | H | Cl | H | O |
| D-11768 | Me(S) | single bond | H | CH=NOMe | H | Cl | H | O |
| D-11769 | H | single bond | Me | CH=NOMe | H | Me | H | O |
| D-11770 | Me | single bond | H | CH=NOMe | H | Me | H | O |
| D-11771 | Me(R) | single bond | H | CH=NOMe | H | Me | H | O |
| D-11772 | Me(S) | single bond | H | CH=NOMe | H | Me | H | O |
| D-11773 | H | single bond | Me | morpholin-4-yl | H | Cl | H | O |
| D-11774 | Me | single bond | H | morpholin-4-yl | H | Cl | H | O |
| D-11775 | Me(R) | single bond | H | morpholin-4-yl | H | Cl | H | O |
| D-11776 | Me(S) | single bond | H | morpholin-4-yl | H | Cl | H | O |
| D-11777 | H | single bond | Me | morpholin-4-yl | H | Me | H | O |
| D-11778 | Me | single bond | H | morpholin-4-yl | H | Me | H | O |
| D-11779 | Me(R) | single bond | H | morpholin-4-yl | H | Me | H | O |
| D-11780 | Me(S) | single bond | H | morpholin-4-yl | H | Me | H | O |
| D-11781 | H | single bond | Me | Ph | H | Cl | H | O |
| D-11782 | Me | single bond | H | Ph | H | Cl | H | O |
| D-11783 | Me(R) | single bond | H | Ph | H | Cl | H | O |
| D-11784 | Me(S) | single bond | H | Ph | H | Cl | H | O |
| D-11785 | H | single bond | Me | Ph | H | Me | H | O |
| D-11786 | Me | single bond | H | Ph | H | Me | H | O |
| D-11787 | Me(R) | single bond | H | Ph | H | Me | H | O |
| D-11788 | Me(S) | single bond | H | Ph | H | Me | H | O |
| D-11789 | H | single bond | Me | CH₂Ph | H | Cl | H | O |
| D-11790 | Me | single bond | H | CH₂Ph | H | Cl | H | O |
| D-11791 | Me(R) | single bond | H | CH₂Ph | H | Cl | H | O |
| D-11792 | Me(S) | single bond | H | CH₂Ph | H | Cl | H | O |
| D-11793 | H | single bond | Me | CH₂Ph | H | Me | H | O |
| D-11794 | Me | single bond | H | CH₂Ph | H | Me | H | O |
| D-11795 | Me(R) | single bond | H | CH₂Ph | H | Me | H | O |
| D-11796 | Me(S) | single bond | H | CH₂Ph | H | Me | H | O |
| D-11797 | H | single bond | Me | thiazole-2-yl | H | Cl | H | O |
| D-11798 | Me | single bond | H | thiazole-2-yl | H | Cl | H | O |
| D-11799 | Me(R) | single bond | H | thiazole-2-yl | H | Cl | H | O |
| D-11800 | Me(S) | single bond | H | thiazole-2-yl | H | Cl | H | O |
| D-11801 | H | single bond | Me | thiazole-2-yl | H | Me | H | O |
| D-11802 | Me | single bond | H | thiazole-2-yl | H | Me | H | O |
| D-11803 | Me(R) | single bond | H | thiazole-2-yl | H | Me | H | O |
| D-11804 | Me(S) | single bond | H | thiazole-2-yl | H | Me | H | O |
| D-11805 | H | single bond | Me | 1-Me-pyrazol-3-yl | H | Cl | H | O |
| D-11806 | Me | single bond | H | 1-Me-pyrazol-3-yl | H | Cl | H | O |
| D-11807 | Me(R) | single bond | H | 1-Me-pyrazol-3-yl | H | Cl | H | O |
| D-11808 | Me(S) | single bond | H | 1-Me-pyrazol-3-yl | H | Cl | H | O |
| D-11809 | H | single bond | Me | 1-Me-pyrazol-3-yl | H | Me | H | O |
| D-11810 | Me | single bond | H | 1-Me-pyrazol-3-yl | H | Me | H | O |

**[Table 915]**

| Compound Number | Y | W | Z | R¹ | R² | R³ | R¹⁰ | A |
|---|---|---|---|---|---|---|---|---|
| D-11811 | Me(R) | single bond | H | 1-Me-pyrazol-3-yl | H | Me | H | O |
| D-11812 | Me(S) | single bond | H | 1-Me-pyrazol-3-yl | H | Me | H | O |
| D-11813 | H | single bond | Me | 1-Me-pyrazol-5-yl | H | Cl | H | O |
| D-11814 | Me | single bond | H | 1-Me-pyrazol-5-yl | H | Cl | H | O |
| D-11815 | Me(R) | single bond | H | 1-Me-pyrazol-5-yl | H | Cl | H | O |
| D-11816 | Me(S) | single bond | H | 1-Me-pyrazol-5-yl | H | Cl | H | O |
| D-11817 | H | single bond | Me | 1-Me-pyrazol-5-yl | H | Me | H | O |
| D-11818 | Me | single bond | H | 1-Me-pyrazol-5-yl | H | Me | H | O |
| D-11819 | Me(R) | single bond | H | 1-Me-pyrazol-5-yl | H | Me | H | O |
| D-11820 | Me(S) | single bond | H | 1-Me-pyrazol-5-yl | H | Me | H | O |
| D-11821 | H | single bond | Me | CH₂(pyrimidin-2-yl) | H | Cl | H | O |
| D-11822 | Me | single bond | H | CH₂(pyrimidin-2-yl) | H | Cl | H | O |
| D-11823 | Me(R) | single bond | H | CH₂(pyrimidin-2-yl) | H | Cl | H | O |
| D-11824 | Me(S) | single bond | H | CH₂(pyrimidin-2-yl) | H | Cl | H | O |
| D-11825 | H | single bond | Me | CH₂(pyrimidin-2-yl) | H | Me | H | O |
| D-11826 | Me | single bond | H | CH₂(pyrimidin-2-yl) | H | Me | H | O |
| D-11827 | Me(R) | single bond | H | CH₂(pyrimidin-2-yl) | H | Me | H | O |
| D-11828 | Me(S) | single bond | H | CH₂(pyrimidin-2-yl) | H | Me | H | O |
| D-11829 | H | single bond | Me | -CH₂CH₂CH₂- | | Cl | H | O |
| D-11830 | Me | single bond | H | -CH₂CH₂CH₂- | | Cl | H | O |
| D-11831 | Me(R) | single bond | H | -CH₂CH₂CH₂- | | Cl | H | O |
| D-11832 | Me(S) | single bond | H | -CH₂CH₂CH₂- | | Cl | H | O |
| D-11833 | H | single bond | Me | -CH₂CH₂CH₂- | | Me | H | O |
| D-11834 | Me | single bond | H | -CH₂CH₂CH₂- | | Me | H | O |
| D-11835 | Me(R) | single bond | H | -CH₂CH₂CH₂- | | Me | H | O |
| D-11836 | Me(S) | single bond | H | -CH₂CH₂CH₂- | | Me | H | O |
| D-11837 | H | single bond | Me | -CH₂CH₂OCH₂CH₂- | | Cl | H | O |
| D-11838 | Me | single bond | H | -CH₂CH₂OCH₂CH₂- | | Cl | H | O |
| D-11839 | Me(R) | single bond | H | -CH₂CH₂OCH₂CH₂- | | Cl | H | O |
| D-11840 | Me(S) | single bond | H | -CH₂CH₂OCH₂CH₂- | | Cl | H | O |
| D-11841 | H | single bond | Me | -CH₂CH₂OCH₂CH₂- | | Me | H | O |
| D-11842 | Me | single bond | H | -CH₂CH₂OCH₂CH₂- | | Me | H | O |
| D-11843 | Me(R) | single bond | H | -CH₂CH₂OCH₂CH₂- | | Me | H | O |
| D-11844 | Me(S) | single bond | H | -CH₂CH₂OCH₂CH₂- | | Me | H | O |
| D-11845 | H | single bond | Me | Me | H | Cl | Me | O |
| D-11846 | Me | single bond | H | Me | H | Cl | Me | O |
| D-11847 | Me(R) | single bond | H | Me | H | Cl | Me | O |
| D-11848 | Me(S) | single bond | H | Me | H | Cl | Me | O |
| D-11849 | H | single bond | Me | Me | H | Me | Me | O |
| D-11850 | Me | single bond | H | Me | H | Me | Me | O |
| D-11851 | Me(R) | single bond | H | Me | H | Me | Me | O |
| D-11852 | Me(S) | single bond | H | Me | H | Me | Me | O |
| D-11853 | H | single bond | Me | CH₂CHF₂ | H | Cl | Me | O |
| D-11854 | Me | single bond | H | CH₂CHF₂ | H | Cl | Me | O |

**[Table 916]**

| Compound Number | Y | W | Z | R¹ | R² | R³ | R¹⁰ | A |
|---|---|---|---|---|---|---|---|---|
| D-11855 | Me(R) | single bond | H | CH₂CHF₂ | H | Cl | Me | O |
| D-11856 | Me(S) | single bond | H | CH₂CHF₂ | H | Cl | Me | O |
| D-11857 | H | single bond | Me | CH₂CHF₂ | H | Me | Me | O |
| D-11858 | Me | single bond | H | CH₂CHF₂ | H | Me | Me | O |
| D-11859 | Me(R) | single bond | H | CH₂CHF₂ | H | Me | Me | O |
| D-11860 | Me(S) | single bond | H | CH₂CHF₂ | H | Me | Me | O |
| D-11861 | H | single bond | Me | *n*-Pr | H | Cl | Me | O |
| D-11862 | Me | single bond | H | *n*-Pr | H | Cl | Me | O |
| D-11863 | Me(R) | single bond | H | *n*-Pr | H | Cl | Me | O |
| D-11864 | Me(S) | single bond | H | *n*-Pr | H | Cl | Me | O |
| D-11865 | H | single bond | Me | *n*-Pr | H | Me | Me | O |
| D-11866 | Me | single bond | H | *n*-Pr | H | Me | Me | O |
| D-11867 | Me(R) | single bond | H | *n*-Pr | H | Me | Me | O |
| D-11868 | Me(S) | single bond | H | *n*-Pr | H | Me | Me | O |
| D-11869 | H | single bond | Me | *i*-Pr | H | Cl | Me | O |
| D-11870 | Me | single bond | H | *i*-Pr | H | Cl | Me | O |
| D-11871 | Me(R) | single bond | H | *i*-Pr | H | Cl | Me | O |
| D-11872 | Me(S) | single bond | H | *i*-Pr | H | Cl | Me | O |
| D-11873 | H | single bond | Me | *i*-Pr | H | Me | Me | O |
| D-11874 | Me | single bond | H | *i*-Pr | H | Me | Me | O |
| D-11875 | Me(R) | single bond | H | *i*-Pr | H | Me | Me | O |
| D-11876 | Me(S) | single bond | H | *i*-Pr | H | Me | Me | O |
| D-11877 | H | single bond | Me | *c*-Pr(1-F) | H | Cl | Me | O |
| D-11878 | Me | single bond | H | *c*-Pr(1-F) | H | Cl | Me | O |
| D-11879 | Me(R) | single bond | H | *c*-Pr(1-F) | H | Cl | Me | O |
| D-11880 | Me(S) | single bond | H | *c*-Pr(1-F) | H | Cl | Me | O |
| D-11881 | H | single bond | Me | *c*-Pr(1-F) | H | Me | Me | O |
| D-11882 | Me | single bond | H | *c*-Pr(1-F) | H | Me | Me | O |
| D-11883 | Me(R) | single bond | H | *c*-Pr(1-F) | H | Me | Me | O |
| D-11884 | Me(S) | single bond | H | *c*-Pr(1-F) | H | Me | Me | O |
| D-11885 | H | single bond | Me | *c*-Pr(2,2-F₂) | H | Cl | Me | O |
| D-11886 | Me | single bond | H | *c*-Pr(2,2-F₂) | H | Cl | Me | O |
| D-11887 | Me(R) | single bond | H | *c*-Pr(2,2-F₂) | H | Cl | Me | O |
| D-11888 | Me(S) | single bond | H | *c*-Pr(2,2-F₂) | H | Cl | Me | O |
| D-11889 | H | single bond | Me | *c*-Pr(2,2-F₂) | H | Me | Me | O |
| D-11890 | Me | single bond | H | *c*-Pr(2,2-F₂) | H | Me | Me | O |
| D-11891 | Me(R) | single bond | H | *c*-Pr(2,2-F₂) | H | Me | Me | O |
| D-11892 | Me(S) | single bond | H | *c*-Pr(2,2-F₂) | H | Me | Me | O |
| D-11893 | H | single bond | Me | *c*-Pr(1-Cl) | H | Cl | Me | O |
| D-11894 | Me | single bond | H | *c*-Pr(1-Cl) | H | Cl | Me | O |
| D-11895 | Me(R) | single bond | H | *c*-Pr(1-Cl) | H | Cl | Me | O |
| D-11896 | Me(S) | single bond | H | *c*-Pr(1-Cl) | H | Cl | Me | O |
| D-11897 | H | single bond | Me | *c*-Pr(1-Cl) | H | Me | Me | O |
| D-11898 | Me | single bond | H | *c*-Pr(1-Cl) | H | Me | Me | O |

**[Table 917]**

| Compound Number | Y | W | Z | R¹ | R² | R³ | R¹⁰ | A |
|---|---|---|---|---|---|---|---|---|
| D-11899 | Me(R) | single bond | H | *c*-Pr(1-Cl) | H | Me | Me | O |
| D-11900 | Me(S) | single bond | H | *c*-Pr(1-Cl) | H | Me | Me | O |
| D-11901 | H | single bond | Me | *c*-Pr(1-Br) | H | Cl | Me | O |
| D-11902 | Me | single bond | H | *c*-Pr(1-Br) | H | Cl | Me | O |
| D-11903 | Me(R) | single bond | H | *c*-Pr(1-Br) | H | Cl | Me | O |
| D-11904 | Me(S) | single bond | H | *c*-Pr(1-Br) | H | Cl | Me | O |
| D-11905 | H | single bond | Me | *c*-Pr(1-Br) | H | Me | Me | O |
| D-11906 | Me | single bond | H | *c*-Pr(1-Br) | H | Me | Me | O |
| D-11907 | Me(R) | single bond | H | *c*-Pr(1-Br) | H | Me | Me | O |
| D-11908 | Me(S) | single bond | H | *c*-Pr(1-Br) | H | Me | Me | O |
| D-11909 | H | single bond | Me | *c*-Pr(1-Me) | H | Cl | Me | O |
| D-11910 | Me | single bond | H | *c*-Pr(1-Me) | H | Cl | Me | O |
| D-11911 | Me(R) | single bond | H | *c*-Pr(1-Me) | H | Cl | Me | O |
| D-11912 | Me(S) | single bond | H | *c*-Pr(1-Me) | H | Cl | Me | O |
| D-11913 | H | single bond | Me | *c*-Pr(1-Me) | H | Me | Me | O |
| D-11914 | Me | single bond | H | *c*-Pr(1-Me) | H | Me | Me | O |
| D-11915 | Me(R) | single bond | H | *c*-Pr(1-Me) | H | Me | Me | O |
| D-11916 | Me(S) | single bond | H | *c*-Pr(1-Me) | H | Me | Me | O |
| D-11917 | H | single bond | Me | *c*-Pr(1-CH₂OH) | H | Cl | Me | O |
| D-11918 | Me | single bond | H | *c*-Pr(1-CH₂OH) | H | Cl | Me | O |
| D-11919 | Me(R) | single bond | H | *c*-Pr(1-CH₂OH) | H | Cl | Me | O |
| D-11920 | Me(S) | single bond | H | *c*-Pr(1-CH₂OH) | H | Cl | Me | O |
| D-11921 | H | single bond | Me | *c*-Pr(1-CH₂OH) | H | Me | Me | O |
| D-11922 | Me | single bond | H | *c*-Pr(1-CH₂OH) | H | Me | Me | O |
| D-11923 | Me(R) | single bond | H | *c*-Pr(1-CH₂OH) | H | Me | Me | O |
| D-11924 | Me(S) | single bond | H | *c*-Pr(1-CH₂OH) | H | Me | Me | O |
| D-11925 | H | single bond | Me | *c*-Pr[1-CH₂OS(=O)₂Me] | H | Cl | Me | O |
| D-11926 | Me | single bond | H | *c*-Pr[1-CH₂OS(=O)₂Me] | H | Cl | Me | O |
| D-11927 | Me(R) | single bond | H | *c*-Pr[1-CH₂OS(=O)₂Me] | H | Cl | Me | O |
| D-11928 | Me(S) | single bond | H | *c*-Pr[1-CH₂OS(=O)₂Me] | H | Cl | Me | O |
| D-11929 | H | single bond | Me | *c*-Pr[1-CH₂OS(=O)₂Me] | H | Me | Me | O |
| D-11930 | Me | single bond | H | *c*-Pr[1-CH₂OS(=O)₂Me] | H | Me | Me | O |
| D-11931 | Me(R) | single bond | H | *c*-Pr[1-CH₂OS(=O)₂Me] | H | Me | Me | O |
| D-11932 | Me(S) | single bond | H | *c*-Pr[1-CH₂OS(=O)₂Me] | H | Me | Me | O |
| D-11933 | H | single bond | Me | *c*-Pr(1-CH₂OMe) | H | Cl | Me | O |
| D-11934 | Me | single bond | H | *c*-Pr(1-CH₂OMe) | H | Cl | Me | O |
| D-11935 | Me(R) | single bond | H | *c*-Pr(1-CH₂OMe) | H | Cl | Me | O |
| D-11936 | Me(S) | single bond | H | *c*-Pr(1-CH₂OMe) | H | Cl | Me | O |
| D-11937 | H | single bond | Me | *c*-Pr(1-CH₂OMe) | H | Me | Me | O |
| D-11938 | Me | single bond | H | *c*-Pr(1-CH₂OMe) | H | Me | Me | O |
| D-11939 | Me(R) | single bond | H | *c*-Pr(1-CH₂OMe) | H | Me | Me | O |
| D-11940 | Me(S) | single bond | H | *c*-Pr(1-CH₂OMe) | H | Me | Me | O |
| D-11941 | H | single bond | Me | *c*-Pr(1-CH₂OCN) | H | Cl | Me | O |
| D-11942 | Me | single bond | H | *c*-Pr(1-CH₂OCN) | H | Cl | Me | O |

**[Table 918]**

| Compound Number | Y | W | Z | R¹ | R² | R³ | R¹⁰ | A |
|---|---|---|---|---|---|---|---|---|
| D-11943 | Me(R) | single bond | H | *c*-Pr(1-CH₂OCN) | H | Cl | Me | O |
| D-11944 | Me(S) | single bond | H | *c*-Pr(1-CH₂OCN) | H | Cl | Me | O |
| D-11945 | H | single bond | Me | *c*-Pr(1-CH₂OCN) | H | Me | Me | O |
| D-11946 | Me | single bond | H | *c*-Pr(1-CH₂OCN) | H | Me | Me | O |
| D-11947 | Me(R) | single bond | H | *c*-Pr(1-CH₂OCN) | H | Me | Me | O |
| D-11948 | Me(S) | single bond | H | *c*-Pr(1-CH₂OCN) | H | Me | Me | O |
| D-11949 | H | single bond | Me | *c*-Pr(1-CH₂SMe) | H | Cl | Me | O |
| D-11950 | Me | single bond | H | *c*-Pr(1-CH₂SMe) | H | Cl | Me | O |
| D-11951 | Me(R) | single bond | H | *c*-Pr(1-CH₂SMe) | H | Cl | Me | O |
| D-11952 | Me(S) | single bond | H | *c*-Pr(1-CH₂SMe) | H | Cl | Me | O |
| D-11953 | H | single bond | Me | *c*-Pr(1-CH₂SMe) | H | Me | Me | O |
| D-11954 | Me | single bond | H | *c*-Pr(1-CH₂SMe) | H | Me | Me | O |
| D-11955 | Me(R) | single bond | H | *c*-Pr(1-CH₂SMe) | H | Me | Me | O |
| D-11956 | Me(S) | single bond | H | *c*-Pr(1-CH₂SMe) | H | Me | Me | O |
| D-11957 | H | single bond | Me | *c*-Pr[1-CH₂S(=O)Me] | H | Cl | Me | O |
| D-11958 | Me | single bond | H | *c*-Pr[1-CH₂S(=O)Me] | H | Cl | Me | O |
| D-11959 | Me(R) | single bond | H | *c*-Pr[1-CH₂S(=O)Me] | H | Cl | Me | O |
| D-11960 | Me(S) | single bond | H | *c*-Pr[1-CH₂S(=O)Me] | H | Cl | Me | O |
| D-11961 | H | single bond | Me | *c*-Pr[1-CH₂S(=O)Me] | H | Me | Me | O |
| D-11962 | Me | single bond | H | *c*-Pr[1-CH₂S(=O)Me] | H | Me | Me | O |
| D-11963 | Me(R) | single bond | H | *c*-Pr[1-CH₂S(=O)Me] | H | Me | Me | O |
| D-11964 | Me(S) | single bond | H | *c*-Pr[1-CH₂S(=O)Me] | H | Me | Me | O |
| D-11965 | H | single bond | Me | *c*-Pr[1-CH₂S(=O)₂Me] | H | Cl | Me | O |
| D-11966 | Me | single bond | H | *c*-Pr[1-CH₂S(=O)₂Me] | H | Cl | Me | O |
| D-11967 | Me(R) | single bond | H | *c*-Pr[1-CH₂S(=O)₂Me] | H | Cl | Me | O |
| D-11968 | Me(S) | single bond | H | *c*-Pr[1-CH₂S(=O)₂Me] | H | Cl | Me | O |
| D-11969 | H | single bond | Me | *c*-Pr[1-CH₂S(=O)₂Me] | H | Me | Me | O |
| D-11970 | Me | single bond | H | *c*-Pr[1-CH₂S(=O)₂Me] | H | Me | Me | O |
| D-11971 | Me(R) | single bond | H | *c*-Pr[1-CH₂S(=O)₂Me] | H | Me | Me | O |
| D-11972 | Me(S) | single bond | H | *c*-Pr[1-CH₂S(=O)₂Me] | H | Me | Me | O |
| D-11973 | H | single bond | Me | *c*-Pr(1-CH₂F) | H | Cl | Me | O |
| D-11974 | Me | single bond | H | *c*-Pr(1-CH₂F) | H | Cl | Me | O |
| D-11975 | Me(R) | single bond | H | *c*-Pr(1-CH₂F) | H | Cl | Me | O |
| D-11976 | Me(S) | single bond | H | *c*-Pr(1-CH₂F) | H | Cl | Me | O |
| D-11977 | H | single bond | Me | *c*-Pr(1-CH₂F) | H | Me | Me | O |
| D-11978 | Me | single bond | H | *c*-Pr(1-CH₂F) | H | Me | Me | O |
| D-11979 | Me(R) | single bond | H | *c*-Pr(1-CH₂F) | H | Me | Me | O |
| D-11980 | Me(S) | single bond | H | *c*-Pr(1-CH₂F) | H | Me | Me | O |
| D-11981 | H | single bond | Me | *c*-Pr(1-CHF₂) | H | Cl | Me | O |
| D-11982 | Me | single bond | H | *c*-Pr(1-CHF₂) | H | Cl | Me | O |
| D-11983 | Me(R) | single bond | H | *c*-Pr(1-CHF₂) | H | Cl | Me | O |
| D-11984 | Me(S) | single bond | H | *c*-Pr(1-CHF₂) | H | Cl | Me | O |
| D-11985 | H | single bond | Me | *c*-Pr(1-CHF₂) | H | Me | Me | O |
| D-11986 | Me | single bond | H | *c*-Pr(1-CHF₂) | H | Me | Me | O |

**[Table 919]**

| Compound Number | Y | W | Z | R¹ | R² | R³ | R¹⁰ | A |
|---|---|---|---|---|---|---|---|---|
| D-11987 | Me(R) | single bond | H | *c*-Pr(1-CHF₂) | H | Me | Me | O |
| D-11988 | Me(S) | single bond | H | *c*-Pr(1-CHF₂) | H | Me | Me | O |
| D-11989 | H | single bond | Me | *c*-Pr(1-CF₃) | H | Cl | Me | O |
| D-11990 | Me | single bond | H | *c*-Pr(1-CF₃) | H | Cl | Me | O |
| D-11991 | Me(R) | single bond | H | *c*-Pr(1-CF₃) | H | Cl | Me | O |
| D-11992 | Me(S) | single bond | H | *c*-Pr(1-CF₃) | H | Cl | Me | O |
| D-11993 | H | single bond | Me | *c*-Pr(1-CF₃) | H | Me | Me | O |
| D-11994 | Me | single bond | H | *c*-Pr(1-CF₃) | H | Me | Me | O |
| D-11995 | Me(R) | single bond | H | *c*-Pr(1-CF₃) | H | Me | Me | O |
| D-11996 | Me(S) | single bond | H | *c*-Pr(1-CF₃) | H | Me | Me | O |
| D-11997 | H | single bond | Me | *c*-Pr(1-C≡CH) | H | Cl | Me | O |
| D-11998 | Me | single bond | H | *c*-Pr(1-C≡CH) | H | Cl | Me | O |
| D-11999 | Me(R) | single bond | H | *c*-Pr(1-C≡CH) | H | Cl | Me | O |
| D-12000 | Me(S) | single bond | H | *c*-Pr(1-C≡CH) | H | Cl | Me | O |
| D-12001 | H | single bond | Me | *c*-Pr(1-C≡CH) | H | Me | Me | O |
| D-12002 | Me | single bond | H | *c*-Pr(1-C≡CH) | H | Me | Me | O |
| D-12003 | Me(R) | single bond | H | *c*-Pr(1-C≡CH) | H | Me | Me | O |
| D-12004 | Me(S) | single bond | H | *c*-Pr(1-C≡CH) | H | Me | Me | O |
| D-12005 | H | single bond | Me | *c*-Pr[1-C(=O)OEt] | H | Cl | Me | O |
| D-12006 | Me | single bond | H | *c*-Pr[1-C(=O)OEt] | H | Cl | Me | O |
| D-12007 | Me(R) | single bond | H | *c*-Pr[1-C(=O)OEt] | H | Cl | Me | O |
| D-12008 | Me(S) | single bond | H | *c*-Pr[1-C(=O)OEt] | H | Cl | Me | O |
| D-12009 | H | single bond | Me | *c*-Pr[1-C(=O)OEt] | H | Me | Me | O |
| D-12010 | Me | single bond | H | *c*-Pr[1-C(=O)OEt] | H | Me | Me | O |
| D-12011 | Me(R) | single bond | H | *c*-Pr[1-C(=O)OEt] | H | Me | Me | O |
| D-12012 | Me(S) | single bond | H | *c*-Pr[1-C(=O)OEt] | H | Me | Me | O |
| D-12013 | H | single bond | Me | *c*-Pr[1-C(=O)OH] | H | Cl | Me | O |
| D-12014 | Me | single bond | H | *c*-Pr[1-C(=O)OH] | H | Cl | Me | O |
| D-12015 | Me(R) | single bond | H | *c*-Pr[1-C(=O)OH] | H | Cl | Me | O |
| D-12016 | Me(S) | single bond | H | *c*-Pr[1-C(=O)OH] | H | Cl | Me | O |
| D-12017 | H | single bond | Me | *c*-Pr[1-C(=O)OH] | H | Me | Me | O |
| D-12018 | Me | single bond | H | *c*-Pr[1-C(=O)OH] | H | Me | Me | O |
| D-12019 | Me(R) | single bond | H | *c*-Pr[1-C(=O)OH] | H | Me | Me | O |
| D-12020 | Me(S) | single bond | H | *c*-Pr[1-C(=O)OH] | H | Me | Me | O |
| D-12021 | H | single bond | Me | *c*-Pr[1-C(=O)NH₂] | H | Cl | Me | O |
| D-12022 | Me | single bond | H | *c*-Pr[1-C(=O)NH₂] | H | Cl | Me | O |
| D-12023 | Me(R) | single bond | H | *c*-Pr[1-C(=O)NH₂] | H | Cl | Me | O |
| D-12024 | Me(S) | single bond | H | *c*-Pr[1-C(=O)NH₂] | H | Cl | Me | O |
| D-12025 | H | single bond | Me | *c*-Pr[1-C(=O)NH₂] | H | Me | Me | O |
| D-12026 | Me | single bond | H | *c*-Pr[1-C(=O)NH₂] | H | Me | Me | O |
| D-12027 | Me(R) | single bond | H | *c*-Pr[1-C(=O)NH₂] | H | Me | Me | O |
| D-12028 | Me(S) | single bond | H | *c*-Pr[1-C(=O)NH₂] | H | Me | Me | O |
| D-12029 | H | single bond | Me | *c*-Pr(1-OMe) | H | Cl | Me | O |
| D-12030 | Me | single bond | H | *c*-Pr(1-OMe) | H | Cl | Me | O |

**[Table 920]**

| Compound Number | Y | W | Z | R¹ | R² | R³ | R¹⁰ | A |
|---|---|---|---|---|---|---|---|---|
| D-12031 | Me(R) | single bond | H | *c*-Pr(1-OMe) | H | Cl | Me | O |
| D-12032 | Me(S) | single bond | H | *c*-Pr(1-OMe) | H | Cl | Me | O |
| D-12033 | H | single bond | Me | *c*-Pr(1-OMe) | H | Me | Me | O |
| D-12034 | Me | single bond | H | *c*-Pr(1-OMe) | H | Me | Me | O |
| D-12035 | Me(R) | single bond | H | *c*-Pr(1-OMe) | H | Me | Me | O |
| D-12036 | Me(S) | single bond | H | *c*-Pr(1-OMe) | H | Me | Me | O |
| D-12037 | H | single bond | Me | c-Pr(1-SMe) | H | Cl | Me | O |
| D-12038 | Me | single bond | H | c-Pr(1-SMe) | H | Cl | Me | O |
| D-12039 | Me(R) | single bond | H | c-Pr(1-SMe) | H | Cl | Me | O |
| D-12040 | Me(S) | single bond | H | c-Pr(1-SMe) | H | Cl | Me | O |
| D-12041 | H | single bond | Me | c-Pr(1-SMe) | H | Me | Me | O |
| D-12042 | Me | single bond | H | c-Pr(1-SMe) | H | Me | Me | O |
| D-12043 | Me(R) | single bond | H | c-Pr(1-SMe) | H | Me | Me | O |
| D-12044 | Me(S) | single bond | H | c-Pr(1-SMe) | H | Me | Me | O |
| D-12045 | H | single bond | Me | *c*-Pr[1-S(=O)Me] | H | Cl | Me | O |
| D-12046 | Me | single bond | H | *c*-Pr[1-S(=O)Me] | H | Cl | Me | O |
| D-12047 | Me(R) | single bond | H | *c*-Pr[1-S(=O)Me] | H | Cl | Me | O |
| D-12048 | Me(S) | single bond | H | *c*-Pr[1-S(=O)Me] | H | Cl | Me | O |
| D-12049 | H | single bond | Me | *c*-Pr[1-S(=O)Me] | H | Me | Me | O |
| D-12050 | Me | single bond | H | *c*-Pr[1-S(=O)Me] | H | Me | Me | O |
| D-12051 | Me(R) | single bond | H | *c*-Pr[1-S(=O)Me] | H | Me | Me | O |
| D-12052 | Me(S) | single bond | H | *c*-Pr[1-S(=O)Me] | H | Me | Me | O |
| D-12053 | H | single bond | Me | *c*-Pr[1-S(=O)₂Me] | H | Cl | Me | O |
| D-12054 | Me | single bond | H | *c*-Pr[1-S(=O)₂Me] | H | Cl | Me | O |
| D-12055 | Me(R) | single bond | H | *c*-Pr[1-S(=O)₂Me] | H | Cl | Me | O |
| D-12056 | Me(S) | single bond | H | *c*-Pr[1-S(=O)₂Me] | H | Cl | Me | O |
| D-12057 | H | single bond | Me | *c*-Pr[1-S(=O)₂Me] | H | Me | Me | O |
| D-12058 | Me | single bond | H | *c*-Pr[1-S(=O)₂Me] | H | Me | Me | O |
| D-12059 | Me(R) | single bond | H | *c*-Pr[1-S(=O)₂Me] | H | Me | Me | O |
| D-12060 | Me(S) | single bond | H | *c*-Pr[1-S(=O)₂Me] | H | Me | Me | O |
| D-12061 | H | single bond | Me | *c*-Pr[1-S(=O)₂CHF₂] | H | Cl | Me | O |
| D-12062 | Me | single bond | H | *c*-Pr[1-S(=O)₂CHF₂] | H | Cl | Me | O |
| D-12063 | Me(R) | single bond | H | *c*-Pr[1-S(=O)₂CHF₂] | H | Cl | Me | O |
| D-12064 | Me(S) | single bond | H | *c*-Pr[1-S(=O)₂CHF₂] | H | Cl | Me | O |
| D-12065 | H | single bond | Me | *c*-Pr[1-S(=O)₂CHF₂] | H | Me | Me | O |
| D-12066 | Me | single bond | H | *c*-Pr[1-S(=O)₂CHF₂] | H | Me | Me | O |
| D-12067 | Me(R) | single bond | H | *c*-Pr[1-S(=O)₂CHF₂] | H | Me | Me | O |
| D-12068 | Me(S) | single bond | H | *c*-Pr[1-S(=O)₂CHF₂] | H | Me | Me | O |
| D-12069 | H | single bond | Me | *c*-Pr[1-S(=O)₂CF₃] | H | Cl | Me | O |
| D-12070 | Me | single bond | H | *c*-Pr[1-S(=O)₂CF₃] | H | Cl | Me | O |
| D-12071 | Me(R) | single bond | H | *c*-Pr[1-S(=O)₂CF₃] | H | Cl | Me | O |
| D-12072 | Me(S) | single bond | H | *c*-Pr[1-S(=O)₂CF₃] | H | Cl | Me | O |
| D-12073 | H | single bond | Me | *c*-Pr[1-S(=O)₂CF₃] | H | Me | Me | O |
| D-12074 | Me | single bond | H | *c*-Pr[1-S(=O)₂CF₃] | H | Me | Me | O |

**[Table 921]**

| Compound Number | Y | W | Z | R¹ | R² | R³ | R¹⁰ | A |
|---|---|---|---|---|---|---|---|---|
| D-12075 | Me(R) | single bond | H | *c*-Pr[1-S(=O)₂CF₃] | H | Me | Me | O |
| D-12076 | Me(S) | single bond | H | *c*-Pr[1-S(=O)₂CF₃] | H | Me | Me | O |
| 0-12077 | H | single bond | Me | c -Pr[1-(5,6-dihydro-1 ,4,2-dioxazin-3-yl)] | H | Cl | Me | O |
| D-12078 | Me | single bond | H | c -Pr[1-(5,6-dihydro-1 ,4,2-dioxazin-3-yl)] | H | Cl | Me | O |
| D-12079 | Me(R) | single bond | H | c -Pr[1-(5,6-dihydro-1 ,4,2-dioxazin-3-yl)] | H | Cl | Me | O |
| D-12080 | Me(S) | single bond | H | c -Pr[1-(5,6-dihydro-1 ,4,2-dioxazin-3-yl)] | H | Cl | Me | O |
| D-12081 | H | single bond | Me | c -Pr[1-(5,6-dihydro-1 ,4,2-dioxazin-3-yl)] | H | Me | Me | O |
| D-12082 | Me | single bond | H | c -Pr[1-(5,6-dihydro-1 ,4,2-dioxazin-3-yl)] | H | Me | Me | O |
| D-12083 | Me(R) | single bond | H | c -Pr[1-(5,6-dihydro-1 ,4,2-dioxazin-3-yl)] | H | Me | Me | O |
| D-12084 | Me(S) | single bond | H | c -Pr[1-(5,6-dihydro-1 ,4,2-dioxazin-3-yl)] | H | Me | Me | O |
| D-12085 | H | single bond | Me | c -Pr[1-(5-hydroxy-1,3,4-oxadiazol-2-yl)] | H | Cl | Me | O |
| D-12086 | Me | single bond | H | c -Pr[1-(5-hydroxy-1,3,4-oxadiazol-2-yl)] | H | Cl | Me | O |
| D-12087 | Me(R) | single bond | H | c -Pr[1-(5-hydroxy-1,3,4-oxadiazol-2-yl)] | H | Cl | Me | O |
| 0-12088 | Me(S) | single bond | H | c -Pr[1-(5-hydroxy-1,3,4-oxadiazol-2-yl)] | H | Cl | Me | O |
| D-12089 | H | single bond | Me | c -Pr[1-(5-hydroxy-1,3,4-oxadiazol-2-yl)] | H | Me | Me | O |
| D-12090 | Me | single bond | H | c -Pr[1-(5-hydroxy-1,3,4-oxadiazol-2-yl)] | H | Me | Me | O |
| D-12091 | Me(R) | single bond | H | c -Pr[1-(5-hydroxy-1,3,4-oxadiazol-2-yl)] | H | Me | Me | O |
| D-12092 | Me(S) | single bond | H | *c*-Pr[1-(5-hydroxy-1,3,4-oxadiazol-2-yl)] | H | Me | Me | O |
| D-12093 | H | single bond | Me | c -Pr[1-(pyri midin-2-yl)] | H | Cl | Me | O |
| D-12094 | Me | single bond | H | c -Pr[1-(pyri midin-2-yl)] | H | Cl | Me | O |
| D-12095 | Me(R) | single bond | H | c -Pr[1-(pyri midin-2-yl)] | H | Cl | Me | O |
| D-12096 | Me(S) | single bond | H | c -Pr[1-(pyri midin-2-yl)] | H | Cl | Me | O |
| 0-12097 | H | single bond | Me | c -Pr[1-(pyri midin-2-yl)] | H | Me | Me | O |
| D-12098 | Me | single bond | H | c -Pr[1-(pyri midin-2-yl)] | H | Me | Me | O |
| D-12099 | Me(R) | single bond | H | c -Pr[1-(pyri midin-2-yl)] | H | Me | Me | O |
| D-12100 | Me(S) | single bond | H | c -Pr[1-(pyri midin-2-yl)] | H | Me | Me | O |
| D-12101 | H | single bond | Me | t-Bu | H | Cl | Me | O |
| D-12102 | Me | single bond | H | t-Bu | H | Cl | Me | O |
| D-12103 | Me(R) | single bond | H | t-Bu | H | Cl | Me | O |
| D-12104 | Me(S) | single bond | H | t-Bu | H | Cl | Me | O |
| D-12105 | H | single bond | Me | t-Bu | H | Me | Me | O |
| D-12106 | Me | single bond | H | t-Bu | H | Me | Me | O |
| D-12107 | Me(R) | single bond | H | t-Bu | H | Me | Me | O |
| D-12108 | Me(S) | single bond | H | t-Bu | H | Me | Me | O |
| D-12109 | H | single bond | Me | *c*-Bu | H | Cl | Me | O |
| D-12110 | Me | single bond | H | *c*-Bu | H | Cl | Me | O |
| D-12111 | Me(R) | single bond | H | *c*-Bu | H | Cl | Me | O |
| D-12112 | Me(S) | single bond | H | c-Bu | H | Cl | Me | O |
| D-12113 | H | single bond | Me | *c*-Bu | H | Me | Me | O |
| D-12114 | Me | single bond | H | *c*-Bu | H | Me | Me | O |
| D-12115 | Me(R) | single bond | H | *c*-Bu | H | Me | Me | O |
| D-12116 | Me(S) | single bond | H | *c*-Bu | H | Me | Me | O |
| D-12117 | H | single bond | Me | c-Bu(1-CN) | H | Cl | Me | O |
| D-12118 | Me | single bond | H | *c*-Bu(1-CN) | H | Cl | Me | O |

**[Table 922]**

| Compound Number | Y | W | Z | R¹ | R² | R³ | R¹⁰ | A |
|---|---|---|---|---|---|---|---|---|
| D-12119 | Me(R) | single bond | H | c-Bu(1-CN) | H | Cl | Me | O |
| D-12120 | Me(S) | single bond | H | c-Bu(1-CN) | H | Cl | Me | O |
| D-12121 | H | single bond | Me | c-Bu(1-CN) | H | Me | Me | O |
| D-12122 | Me | single bond | H | c-Bu(1-CN) | H | Me | Me | O |
| D-12123 | Me(R) | single bond | H | c-Bu(1-CN) | H | Me | Me | O |
| D-12124 | Me(S) | single bond | H | *c*-Bu(1-CN) | H | Me | Me | O |
| D-12125 | H | single bond | Me | *c*-Bu(3,3-F₂) | H | Cl | Me | O |
| D-12126 | Me | single bond | H | *c*-Bu(3,3-F₂) | H | Cl | Me | O |
| D-12127 | Me(R) | single bond | H | *c*-Bu(3,3-F₂) | H | Cl | Me | O |
| D-12128 | Me(S) | single bond | H | *c*-Bu(3,3-F₂) | H | Cl | Me | O |
| D-12129 | H | single bond | Me | *c*-Bu(3,3-F₂) | H | Me | Me | O |
| D-12130 | Me | single bond | H | *c*-Bu(3,3-F₂) | H | Me | Me | O |
| D-12131 | Me(R) | single bond | H | *c*-Bu(3,3-F₂) | H | Me | Me | O |
| D-12132 | Me(S) | single bond | H | *c*-Bu(3,3-F₂) | H | Me | Me | O |
| D-12133 | H | single bond | Me | oxetan-3-yl | H | Cl | Me | O |
| D-12134 | Me | single bond | H | oxetan-3-yl | H | Cl | Me | O |
| D-12135 | Me(R) | single bond | H | oxetan-3-yl | H | Cl | Me | O |
| D-12136 | Me(S) | single bond | H | oxetan-3-yl | H | Cl | Me | O |
| D-12137 | H | single bond | Me | oxetan-3-yl | H | Me | Me | O |
| D-12138 | Me | single bond | H | oxetan-3-yl | H | Me | Me | O |
| D-12139 | Me(R) | single bond | H | oxetan-3-yl | H | Me | Me | O |
| D-12140 | Me(S) | single bond | H | oxetan-3-yl | H | Me | Me | O |
| D-12141 | H | single bond | Me | oxetan(3-CN)-3-yl | H | Cl | Me | O |
| D-12142 | Me | single bond | H | oxetan(3-CN)-3-yl | H | Cl | Me | O |
| D-12143 | Me(R) | single bond | H | oxetan(3-CN)-3-yl | H | Cl | Me | O |
| D-12144 | Me(S) | single bond | H | oxetan(3-CN)-3-yl | H | Cl | Me | O |
| D-12145 | H | single bond | Me | oxetan(3-CN)-3-yl | H | Me | Me | O |
| D-12146 | Me | single bond | H | oxetan(3-CN)-3-yl | H | Me | Me | O |
| D-12147 | Me(R) | single bond | H | oxetan(3-CN)-3-yl | H | Me | Me | O |
| D-12148 | Me(S) | single bond | H | oxetan(3-CN)-3-yl | H | Me | Me | O |
| D-12149 | H | single bond | Me | azetidin[1-C(=O)Me]-3-yl | H | Cl | Me | O |
| D-12150 | Me | single bond | H | azetidin[1-C(=O)Me]-3-yl | H | Cl | Me | O |
| D-12151 | Me(R) | single bond | H | azetidin[1-C(=O)Me]-3-yl | H | Cl | Me | O |
| D-12152 | Me(S) | single bond | H | azetidin[1-C(=O)Me]-3-yl | H | Cl | Me | O |
| D-12153 | H | single bond | Me | azetidin[1-C(=O)Me]-3-yl | H | Me | Me | O |
| D-12154 | Me | single bond | H | azetidin[1-C(=O)Me]-3-yl | H | Me | Me | O |
| D-12155 | Me(R) | single bond | H | azetidin[1-C(=O)Me]-3-yl | H | Me | Me | O |
| D-12156 | Me(S) | single bond | H | azetidin[1-C(=O)Me]-3-yl | H | Me | Me | O |
| D-12157 | H | single bond | Me | azetidin[1-S(=O)₂Me]-3-yl | H | Cl | Me | O |
| D-12158 | Me | single bond | H | azetidin[1-S(=O)₂Me]-3-yl | H | Cl | Me | O |
| D-12159 | Me(R) | single bond | H | azetidin[1-S(=O)₂Me]-3-yl | H | Cl | Me | O |
| D-12160 | Me(S) | single bond | H | azetidin[1-S(=O)₂Me]-3-yl | H | Cl | Me | O |
| D-12161 | H | single bond | Me | azetidin[1-S(=O)₂Me]-3-yl | H | Me | Me | O |
| D-12162 | Me | single bond | H | azetidin[1-S(=O)₂Me]-3-yl | H | Me | Me | O |

**[Table 923]**

| Compound Number | Y | W | Z | R¹ | R² | R³ | R¹⁰ | A |
|---|---|---|---|---|---|---|---|---|
| D-12163 | Me(R) | single bond | H | azetidin[1-S(=O)₂Me]-3-yl | H | Me | Me | O |
| D-12164 | Me(S) | single bond | H | azetidin[1-S(=O)₂Me]-3-yl | H | Me | Me | O |
| D-12165 | H | single bond | Me | azetidin[1-S(=O)₂CHF₂]-3-yl | H | Cl | Me | O |
| D-12166 | Me | single bond | H | azetidin[1-S(=O)₂CHF₂]-3-yl | H | Cl | Me | O |
| D-12167 | Me(R) | single bond | H | azetidin[1-S(=O)₂CHF₂]-3-yl | H | Cl | Me | O |
| D-12168 | Me(S) | single bond | H | azetidin[1-S(=O)₂CHF₂]-3-yl | H | Cl | Me | O |
| D-12169 | H | single bond | Me | azetidin[1-S(=O)₂CHF₂]-3-yl | H | Me | Me | O |
| D-12170 | Me | single bond | H | azetidin[1-S(=O)₂CHF₂]-3-yl | H | Me | Me | O |
| D-12171 | Me(R) | single bond | H | azetidin[1-S(=O)₂CHF₂]-3-yl | H | Me | Me | O |
| D-12172 | Me(S) | single bond | H | azetidin[1-S(=O)₂CHF₂]-3-yl | H | Me | Me | O |
| D-12173 | H | *single* bond | Me | *c*-Pen | H | Cl | Me | O |
| D-12174 | Me | single bond | H | *c*-Pen | H | Cl | Me | O |
| D-12175 | Me(R) | single bond | H | *c*-Pen | H | Cl | Me | O |
| D-12176 | Me(S) | single bond | H | *c*-Pen | H | Cl | Me | O |
| D-12177 | H | single bond | Me | *c*-Pen | H | Me | Me | O |
| D-12178 | Me | single bond | H | c-Pen | H | Me | Me | O |
| D-12179 | Me(R) | single bond | H | *c*-Pen | H | Me | Me | O |
| D-12180 | Me(S) | single bond | H | c-Pen | H | Me | Me | O |
| D-12181 | H | single bond | Me | tetrahydrofuran-3-yl | H | Cl | Me | O |
| D-12182 | Me | single bond | H | tetrahydrofuran-3-yl | H | Cl | Me | O |
| D-12183 | Me(R) | single bond | H | tetrahydrofuran-3-yl | H | Cl | Me | O |
| D-12184 | Me(S) | single bond | H | tetrahydrofuran-3-yl | H | Cl | Me | O |
| D-12185 | H | single bond | Me | tetrahydrofuran-3-yl | H | Me | Me | O |
| D-12186 | Me | single bond | H | tetrahydrofuran-3-yl | H | Me | Me | O |
| D-12187 | Me(R) | single bond | H | tetrahydrofuran-3-yl | H | Me | Me | O |
| D-12188 | Me(S) | single bond | H | tetrahydrofuran-3-yl | H | Me | Me | O |
| D-12189 | H | single bond | Me | tetrahydrothiophen-3-yl | H | Cl | Me | O |
| D-12190 | Me | single bond | H | tetrahydrothiophen-3-yl | H | Cl | Me | O |
| D-12191 | Me(R) | single bond | H | tetrahydrothiophen-3-yl | H | Cl | Me | O |
| D-12192 | Me(S) | single bond | H | tetrahydrothiophen-3-yl | H | Cl | Me | O |
| D-12193 | H | single bond | Me | tetrahydrothiophen-3-yl | H | Me | Me | O |
| D-12194 | Me | single bond | H | tetrahydrothiophen-3-yl | H | Me | Me | O |
| D-12195 | Me(R) | single bond | H | tetrahydrothiophen-3-yl | H | Me | Me | O |
| D-12196 | Me(S) | single bond | H | tetrahydrothiophen-3-yl | H | Me | Me | O |
| D-12197 | H | single bond | Me | *c*-Pen(1-CN) | H | Cl | Me | O |
| D-12198 | Me | single bond | H | *c*-Pen(1-CN) | H | Cl | Me | O |
| D-12199 | Me(R) | single bond | H | c-Pen(1-CN) | H | Cl | Me | O |
| D-12200 | Me(S) | single bond | H | c-Pen(1-CN) | H | Cl | Me | O |
| D-12201 | H | single bond | Me | *c*-Pen(1-CN) | H | Me | Me | O |
| D-12202 | Me | *single bond* | H | *c-Pen(1-CN)* | H | Me | Me | O |
| D-12203 | Me(R) | single bond | H | c-Pen(1-CN) | H | Me | Me | O |
| D-12204 | Me(S) | single bond | H | c-Pen(1-CN) | H | Me | Me | O |
| D-12205 | H | single bond | Me | tetrahydrofuran(3-CN)-3-yl | H | Cl | Me | O |
| D-12206 | Me | single bond | H | tetrahydrofuran(3-CN)-3-yl | H | Cl | Me | O |

**[Table 924]**

| Compound Number | Y | W | Z | R¹ | R² | R³ | R¹⁰ | A |
|---|---|---|---|---|---|---|---|---|
| D-12207 | Me(R) | single bond | H | tetrahydrofuran(3-CN)-3-yl | H | Cl | Me | O |
| D-12208 | Me(S) | single bond | H | tetrahydrofuran(3-CN)-3-yl | H | Cl | Me | O |
| D-12209 | H | single bond | Me | tetrahydrofuran(3-CN)-3-yl | H | Me | Me | O |
| D-12210 | Me | single bond | H | tetrahydrofuran(3-CN)-3-yl | H | Me | Me | O |
| D-12211 | Me(R) | single bond | H | tetrahydrofuran(3-CN)-3-yl | H | Me | Me | O |
| D-12212 | Me(S) | single bond | H | tetrahydrofuran(3-CN)-3-yl | H | Me | Me | O |
| D-12213 | H | single bond | Me | tetrahydrothiophen(3-CN)-3-yl | H | Cl | Me | O |
| D-12214 | Me | single bond | H | tetrahydrothiophen(3-CN)-3-yl | H | Cl | Me | O |
| D-12215 | Me(R) | single bond | H | tetrahydrothiophen(3-CN)-3-yl | H | Cl | Me | O |
| D-12216 | Me(S) | single bond | H | tetrahydrothiophen(3-CN)-3-yl | H | Cl | Me | O |
| D-12217 | H | single bond | Me | tetrahydrothiophen(3-CN)-3-yl | H | Me | Me | O |
| D-12218 | Me | single bond | H | tetrahydrothiophen(3-CN)-3-yl | H | Me | Me | O |
| D-12219 | Me(R) | single bond | H | tetrahydrothiophen(3-CN)-3-yl | H | Me | Me | O |
| D-12220 | Me(S) | single bond | H | tetrahydrothiophen(3-CN)-3-yl | H | Me | Me | O |
| D-12221 | H | single bond | Me | bicyclo[1.1.1]pertan-1-yl | H | Cl | Me | O |
| D-12222 | Me | single bond | H | bicyclo[1.1.1]pentan-1-yl | H | Cl | Me | O |
| D-12223 | Me(R) | single bond | H | bicyclo[1.1.1]pertan-1-yl | H | Cl | Me | O |
| D-12224 | Me(S) | single bond | H | bicyclo[1.1.1]pertan-1-yl | H | Cl | Me | O |
| D-12225 | H | single bond | Me | bicyclo[1.1.1]pentan-1-yl | H | Me | Me | O |
| D-12226 | Me | single bond | H | bicyclo[1.1.1]pertan-1-yl | H | Me | Me | O |
| D-12227 | Me(R) | single bond | H | bicyclo[1.1.1]pertan-1-yl | H | Me | Me | O |
| D-12228 | Me(S) | single bond | H | bicyclo[1.1.1]pentan-1-yl | H | Me | Me | O |
| D-12229 | H | single bond | Me | *c*-Hex | H | Cl | Me | O |
| D-12230 | Me | single bond | H | c -Hex | H | Cl | Me | O |
| D-12231 | Me(R) | single bond | H | *c*-Hex | H | Cl | Me | O |
| D-12232 | Me(S) | single bond | H | *c*-Hex | H | Cl | Me | O |
| D-12233 | H | single bond | Me | c -Hex | H | Me | Me | O |
| D-12234 | Me | single bond | H | *c*-Hex | H | Me | Me | O |
| D-12235 | Me(R) | single bond | H | *c*-Hex | H | Me | Me | O |
| D-12236 | Me(S) | single bond | H | *c*-Hex | H | Me | Me | O |
| D-12237 | H | single bond | Me | tetrahydropyran-4-yl | H | Cl | Me | O |
| D-12238 | Me | single bond | H | tetrahydropyran-4-yl | H | Cl | Me | O |
| D-12239 | Me(R) | single bond | H | tetrahydropyran-4-yl | H | Cl | Me | O |
| D-12240 | Me(S) | single bond | H | tetrahydropyran-4-yl | H | Cl | Me | O |
| D-12241 | H | single bond | Me | tetrahydropyran-4-yl | H | Me | Me | O |
| D-12242 | Me | single bond | H | tetrahydropyran-4-yl | H | Me | Me | O |
| D-12243 | Me(R) | single bond | H | tetrahydropyran-4-yl | H | Me | Me | O |
| D-12244 | Me(S) | single bond | H | tetrahydropyran-4-yl | H | Me | Me | O |
| D-12245 | H | single bond | Me | tetrahydrothiopyran-4-yl | H | Cl | Me | O |
| D-12246 | Me | single bond | H | tetrahydrothiopyran-4-yl | H | Cl | Me | O |
| D-12247 | Me(R) | single bond | H | tetrahydrothiopyran-4-yl | H | Cl | Me | O |
| D-12248 | Me(S) | single bond | H | tetrahydrothiopyran-4-yl | H | Cl | Me | O |
| D-12249 | H | single bond | Me | tetrahydrothiopyran-4-yl | H | Me | Me | O |
| D-12250 | Me | single bond | H | tetrahydrothiopyran-4-yl | H | Me | Me | O |

**[Table 925]**

| Compound Number | Y | W | Z | R¹ | R² | R³ | R¹⁰ | A |
|---|---|---|---|---|---|---|---|---|
| D-12251 | Me(R) | single bond | H | tetrahydrothiopyran-4-yl | H | Me | Me | O |
| D-12252 | Me(S) | single bond | H | tetrahydrothiopyran-4-yl | H | Me | Me | O |
| D-12253 | H | single bond | Me | *c-*Hex(1-CN) | H | Cl | Me | O |
| D-12254 | Me | single bond | H | *c-*Hex(1-CN) | H | Cl | Me | O |
| D-12255 | Me(R) | single bond | H | *c-*Hex(1-CN) | H | Cl | Me | O |
| D-12256 | Me(S) | single bond | H | *c-*Hex(1-CN) | H | Cl | Me | O |
| D-12257 | H | single bond | Me | *c-*Hex(1-CN) | H | Me | Me | O |
| D-12258 | Me | single bond | H | *c-*Hex(1-CN) | H | Me | Me | O |
| D-12259 | Me(R) | single bond | H | *c-*Hex(1-CN) | H | Me | Me | O |
| D-12260 | Me(S) | single bond | H | *c-*Hex(1-CN) | H | Me | Me | O |
| D-12261 | H | single bond | Me | tetrahydropyran(4-CN)-4-yl | H | Cl | Me | O |
| D-12262 | Me | single bond | H | tetrahydropyran(4-CN)-4-yl | H | Cl | Me | O |
| D-12263 | Me(R) | single bond | H | tetrahydropyran(4-CN)-4-yl | H | Cl | Me | O |
| D-12264 | Me(S) | single bond | H | tetrahydropyran(4-CN)-4-yl | H | Cl | Me | O |
| D-12265 | H | single bond | Me | tetrahydropyran(4-CN)-4-yl | H | Me | Me | O |
| D-12266 | Me | single bond | H | tetrahydropyran(4-CN)-4-yl | H | Me | Me | O |
| D-12267 | Me(R) | single bond | H | tetrahydropyran(4-CN)-4-yl | H | Me | Me | O |
| D-12268 | Me(S) | single bond | H | tetrahydropyran(4-CN)-4-yl | H | Me | Me | O |
| D-12269 | H | single bond | Me | tetrahydrothiopyran(4-CN)-4-yl | H | Cl | Me | O |
| D-12270 | Me | single bond | H | tetrahydrothiopyran(4-CN)-4-yl | H | Cl | Me | O |
| D-12271 | Me(R) | single bond | H | tetrahydrothiopyran(4-CN)-4-yl | H | Cl | Me | O |
| D-12272 | Me(S) | single bond | H | tetrahydrothiopyran(4-CN)-4-yl | H | Cl | Me | O |
| D-12273 | H | single bond | Me | tetrahydrothiopyran(4-CN)-4-yl | H | Me | Me | O |
| D-12274 | Me | single bond | H | tetrahydrothiopyran(4-CN)-4-yl | H | Me | Me | O |
| D-12275 | Me(R) | single bond | H | tetrahydrothiopyran(4-CN)-4-yl | H | Me | Me | O |
| D-12276 | Me(S) | single bond | H | tetrahydrothiopyran(4-CN)-4-yl | H | Me | Me | O |
| D-12277 | H | single bond | Me | CH₂(*c*-Pr) | H | Cl | Me | O |
| D-12278 | Me | single bond | H | CH₂(*c*-Pr) | H | Cl | Me | O |
| D-12279 | Me(R) | single bond | H | CH₂(*c*-Pr) | H | Cl | Me | O |
| D-12280 | Me(S) | single bond | H | CH₂(*c*-Pr) | H | Cl | Me | O |
| D-12281 | H | single bond | Me | CH₂(*c*-Pr) | H | Me | Me | O |
| D-12282 | Me | single bond | H | CH₂(*c*-Pr) | H | Me | Me | O |
| D-12283 | Me(R) | single bond | H | CH₂(*c*-Pr) | H | Me | Me | O |
| D-12284 | Me(S) | single bond | H | CH₂(*c*-Pr) | H | Me | Me | O |
| D-12285 | H | single bond | Me | CH₂[*c*-Pr(1-CN)] | H | Cl | Me | O |
| D-12286 | Me | single bond | H | CH₂[*c*-Pr(1-CN)] | H | Cl | Me | O |
| D-12287 | Me(R) | single bond | H | CH₂[*c*-Pr(1-CN)] | H | Cl | Me | O |
| D-12288 | Me(S) | single bond | H | CH₂[*c*-Pr(1-CN)] | H | Cl | Me | O |
| D-12289 | H | single bond | Me | CH₂[*c*-Pr(1-CN)] | H | Me | Me | O |
| D-12290 | Me | single bond | H | CH₂[*c*-Pr(1-CN)] | H | Me | Me | O |
| D-12291 | Me(R) | single bond | H | CH₂[*c*-Pr(1-CN)] | H | Me | Me | O |
| D-12292 | Me(S) | single bond | H | CH₂[*c*-Pr(1-CN)] | H | Me | Me | O |
| D-12293 | H | single bond | Me | CH(Me)(*c*-Pr) | H | Cl | Me | O |
| D-12294 | Me | single bond | H | CH(Me)(*c*-Pr) | H | Cl | Me | O |

**[Table 926]**

| Compound Number | Y | W | Z | R¹ | R² | R³ | R¹⁰ | A |
|---|---|---|---|---|---|---|---|---|
| D-12295 | Me(R) | single bond | H | CH(Me)(*c*-Pr) | H | Cl | Me | O |
| D-12296 | Me(S) | single bond | H | CH(Me)(*c*-Pr) | H | Cl | Me | O |
| D-12297 | H | single bond | Me | CH(Me)(*c*-Pr) | H | Me | Me | O |
| D-12298 | Me | single bond | H | CH(Me)(*c*-Pr) | H | Me | Me | O |
| D-12299 | Me(R) | single bond | H | CH(Me)(*c*-Pr) | H | Me | Me | O |
| D-12300 | Me(S) | single bond | H | CH(Me)(*c*-Pr) | H | Me | Me | O |
| D-12301 | H | single bond | Me | CH(Me)CH₂OMe | H | Cl | Me | O |
| D-12302 | Me | single bond | H | CH(Me)CH₂OMe | H | Cl | Me | O |
| D-12303 | Me(R) | single bond | H | CH(Me)CH₂OMe | H | Cl | Me | O |
| D-12304 | Me(S) | single bond | H | CH(Me)CH₂OMe | H | Cl | Me | O |
| D-12305 | H | single bond | Me | CH(Me)CH₂OMe | H | Me | Me | O |
| D-12306 | Me | single bond | H | CH(Me)CH₂OMe | H | Me | Me | O |
| D-12307 | Me(R) | single bond | H | CH(Me)CH₂OMe | H | Me | Me | O |
| D-12308 | Me(S) | single bond | H | CH(Me)CH₂OMe | H | Me | Me | O |
| D-12309 | H | single bond | Me | CH₂CH=CH₂ | H | Cl | Me | O |
| D-12310 | Me | single bond | H | CH₂CH=CH₂ | H | Cl | Me | O |
| D-12311 | Me(R) | single bond | H | CH₂CH=CH₂ | H | Cl | Me | O |
| D-12312 | Me(S) | single bond | H | CH₂CH=CH₂ | H | Cl | Me | O |
| D-12313 | H | single bond | Me | CH₂CH=CH₂ | H | Me | Me | O |
| D-12314 | Me | single bond | H | CH₂CH=CH₂ | H | Me | Me | O |
| D-12315 | Me(R) | single bond | H | CH₂CH=CH₂ | H | Me | Me | O |
| D-12316 | Me(S) | single bond | H | CH₂CH=CH₂ | H | Me | Me | O |
| D-12317 | H | single bond | Me | CH₂C≡CH | H | Cl | Me | O |
| D-12318 | Me | single bond | H | CH₂C≡CH | H | Cl | Me | O |
| D-12319 | Me(R) | single bond | H | CH₂C≡CH | H | Cl | Me | O |
| D-12320 | Me(S) | single bond | H | CH₂C≡CH | H | Cl | Me | O |
| D-12321 | H | single bond | Me | CH₂C≡CH | H | Me | Me | O |
| D-12322 | Me | single bond | H | CH₂C≡CH | H | Me | Me | O |
| D-12323 | Me(R) | single bond | H | CH₂C≡CH | H | Me | Me | O |
| D-12324 | Me(S) | single bond | H | CH₂C≡CH | H | Me | Me | O |
| D-12325 | H | single bond | Me | CH₂CN | H | Cl | Me | O |
| D-12326 | Me | single bond | H | CH₂CN | H | Cl | Me | O |
| D-12327 | Me(R) | single bond | H | CH₂CN | H | Cl | Me | O |
| D-12328 | Me(S) | single bond | H | CH₂CN | H | Cl | Me | O |
| D-12329 | H | single bond | Me | CH₂CN | H | Me | Me | O |
| D-12330 | Me | single bond | H | CH₂CN | H | Me | Me | O |
| D-12331 | Me(R) | single bond | H | CH₂CN | H | Me | Me | O |
| D-12332 | Me(S) | single bond | H | CH₂CN | H | Me | Me | O |
| D-12333 | H | single bond | Me | CH(Me)CN | H | Cl | Me | O |
| D-12334 | Me | single bond | H | CH(Me)CN | H | Cl | Me | O |
| D-12335 | Me(R) | single bond | H | CH(Me)CN | H | Cl | Me | O |
| D-12336 | Me(S) | single bond | H | CH(Me)CN | H | Cl | Me | O |
| D-12337 | H | single bond | Me | CH(Me)CN | H | Me | Me | O |
| D-12338 | Me | single bond | H | CH(Me)CN | H | Me | Me | O |

**[Table 927]**

| Compound Number | Y | W | Z | R¹ | R² | R³ | R¹⁰ | A |
|---|---|---|---|---|---|---|---|---|
| D-12339 | Me(R) | single bond | H | CH(Me)CN | H | Me | Me | O |
| D-12340 | Me(S) | single bond | H | CH(Me)CN | H | Me | Me | O |
| D-12341 | H | single bond | Me | C(Me)₂CN | H | Cl | Me | O |
| D-12342 | Me | single bond | H | C(Me)₂CN | H | Cl | Me | O |
| D-12343 | Me(R) | single bond | H | C(Me)₂CN | H | Cl | Me | O |
| D-12344 | Me(S) | single bond | H | C(Me)₂CN | H | Cl | Me | O |
| D-12345 | H | single bond | Me | C(Me)₂CN | H | Me | Me | O |
| D-12346 | Me | single bond | H | C(Me)₂CN | H | Me | Me | O |
| D-12347 | Me(R) | single bond | H | C(Me)₂CN | H | Me | Me | O |
| D-12348 | Me(S) | single bond | H | C(Me)₂CN | H | Me | Me | O |
| D-12349 | H | single bond | Me | C(Me)(*í* -Pr)CN | H | Cl | Me | O |
| D-12350 | Me | single bond | H | C(Me)(*í* -Pr)CN | H | Cl | Me | O |
| D-12351 | Me(R) | single bond | H | C(Me)(*í* -Pr)CN | H | Cl | Me | O |
| D-12352 | Me(S) | single bond | H | C(Me)(*í* -Pr)CN | H | Cl | Me | O |
| D-12353 | H | single bond | Me | C(Me)(*í* -Pr)CN | H | Me | Me | O |
| D-12354 | Me | single bond | H | C(Me)(*í* -Pr)CN | H | Me | Me | O |
| D-12355 | Me(R) | single bond | H | C(Me)(*í* -Pr)CN | H | Me | Me | O |
| D-12356 | Me(S) | single bond | H | C(Me)(*í* -Pr)CN | H | Me | Me | O |
| D-12357 | H | single bond | Me | OMe | H | Cl | Me | O |
| D-12358 | Me | single bond | H | OMe | H | Cl | Me | O |
| D-12359 | Me(R) | single bond | H | OMe | H | Cl | Me | O |
| D-12360 | Me(S) | single bond | H | OMe | H | Cl | Me | O |
| D-12361 | H | single bond | Me | OMe | H | Me | Me | O |
| D-12362 | Me | single bond | H | OMe | H | Me | Me | O |
| D-12363 | Me(R) | single bond | H | OMe | H | Me | Me | O |
| D-12364 | Me(S) | single bond | H | OMe | H | Me | Me | O |
| D-12365 | H | single bond | Me | CH=NOMe | H | Cl | Me | O |
| D-12366 | Me | single bond | H | CH=NOMe | H | Cl | Me | O |
| D-12367 | Me(R) | single bond | H | CH=NOMe | H | Cl | Me | O |
| D-12368 | Me(S) | single bond | H | CH=NOMe | H | Cl | Me | O |
| D-12369 | H | single bond | Me | CH=NOMe | H | Me | Me | O |
| D-12370 | Me | single bond | H | CH=NOMe | H | Me | Me | O |
| D-12371 | Me(R) | single bond | H | CH=NOMe | H | Me | Me | O |
| D-12372 | Me(S) | single bond | H | CH=NOMe | H | Me | Me | O |
| D-12373 | H | single bond | Me | morpholin-4-yl | H | Cl | Me | O |
| D-12374 | Me | single bond | H | morpholin-4-yl | H | Cl | Me | O |
| D-12375 | Me(R) | single bond | H | morpholin-4-yl | H | Cl | Me | O |
| D-12376 | Me(S) | single bond | H | morpholin-4-yl | H | Cl | Me | O |
| D-12377 | H | single bond | Me | morpholin-4-yl | H | Me | Me | O |
| D-12378 | Me | single bond | H | morpholin-4-yl | H | Me | Me | O |
| D-12379 | Me(R) | single bond | H | morpholin-4-yl | H | Me | Me | O |
| D-12380 | Me(S) | single bond | H | morpholin-4-yl | H | Me | Me | O |
| D-12381 | H | single bond | Me | Ph | H | Cl | Me | O |
| D-12382 | Me | single bond | H | Ph | H | Cl | Me | O |

**[Table 928]**

| Compound Number | Y | W | Z | R¹ | R² | R³ | R¹⁰ | A |
|---|---|---|---|---|---|---|---|---|
| D-12383 | Me(R) | single bond | H | Ph | H | Cl | Me | O |
| D-12384 | Me(S) | single bond | H | Ph | H | Cl | Me | O |
| D-12385 | H | single bond | Me | Ph | H | Me | Me | O |
| D-12386 | Me | single bond | H | Ph | H | Me | Me | O |
| D-12387 | Me(R) | single bond | H | Ph | H | Me | Me | O |
| D-12388 | Me(S) | single bond | H | Ph | H | Me | Me | O |
| D-12389 | H | single bond | Me | CH₂Ph | H | Cl | Me | O |
| D-12390 | Me | single bond | H | CH₂Ph | H | Cl | Me | O |
| D-12391 | Me(R) | single bond | H | CH₂Ph | H | Cl | Me | O |
| D-12392 | Me(S) | single bond | H | CH₂Ph | H | Cl | Me | O |
| D-12393 | H | single bond | Me | CH₂Ph | H | Me | Me | O |
| D-12394 | Me | single bond | H | CH₂Ph | H | Me | Me | O |
| D-12395 | Me(R) | single bond | H | CH₂Ph | H | Me | Me | O |
| D-12396 | Me(S) | single bond | H | CH₂Ph | H | Me | Me | O |
| D-12397 | H | single bond | Me | thiazole-2-yl | H | Cl | Me | O |
| D-12398 | Me | single bond | H | thiazole-2-yl | H | Cl | Me | O |
| D-12399 | Me(R) | single bond | H | thiazole-2-yl | H | Cl | Me | O |
| D-12400 | Me(S) | single bond | H | thiazole-2-yl | H | Cl | Me | O |
| D-12401 | H | single bond | Me | thiazole-2-yl | H | Me | Me | O |
| D-12402 | Me | single bond | H | thiazole-2-yl | H | Me | Me | O |
| D-12403 | Me(R) | single bond | H | thiazole-2-yl | H | Me | Me | O |
| D-12404 | Me(S) | single bond | H | thiazole-2-yl | H | Me | Me | O |
| D-12405 | H | single bond | Me | 1-Me-pyrazol-3-yl | H | Cl | Me | O |
| D-12406 | Me | single bond | H | 1-Me-pyrazol-3-yl | H | Cl | Me | O |
| D-12407 | Me(R) | single bond | H | 1-Me-pyrazol-3-yl | H | Cl | Me | O |
| D-12408 | Me(S) | single bond | H | 1-Me-pyrazol-3-yl | H | Cl | Me | O |
| D-12409 | H | single bond | Me | 1-Me-pyrazol-3-yl | H | Me | Me | O |
| D-12410 | Me | single bond | H | 1-Me-pyrazol-3-yl | H | Me | Me | O |
| D-12411 | Me(R) | single bond | H | 1-Me-pyrazol-3-yl | H | Me | Me | O |
| D-12412 | Me(S) | single bond | H | 1-Me-pyrazol-3-yl | H | Me | Me | O |
| D-12413 | H | single bond | Me | 1-Me-pyrazol-5-yl | H | Cl | Me | O |
| D-12414 | Me | single bond | H | 1-Me-pyrazol-5-yl | H | Cl | Me | O |
| D-12415 | Me(R) | single bond | H | 1-Me-pyrazol-5-yl | H | Cl | Me | O |
| D-12416 | Me(S) | single bond | H | 1-Me-pyrazol-5-yl | H | Cl | Me | O |
| D-12417 | H | single bond | Me | 1-Me-pyrazol-5-yl | H | Me | Me | O |
| D-12418 | Me | single bond | H | 1-Me-pyrazol-5-yl | H | Me | Me | O |
| D-12419 | Me(R) | single bond | H | 1-Me-pyrazol-5-yl | H | Me | Me | O |
| D-12420 | Me(S) | single bond | H | 1-Me-pyrazol-5-yl | H | Me | Me | O |
| D-12421 | H | single bond | Me | CH₂(pyrimidin-2-yl) | H | Cl | Me | O |
| D-12422 | Me | single bond | H | CH₂(pyrimidin-2-yl) | H | Cl | Me | O |
| D-12423 | Me(R) | single bond | H | CH₂(pyrimidin-2-yl) | H | Cl | Me | O |
| D-12424 | Me(S) | single bond | H | CH₂(pyrimidin-2-yl) | H | Cl | Me | O |
| D-12425 | H | single bond | Me | CH₂(pyrimidin-2-yl) | H | Me | Me | O |
| D-12426 | Me | single bond | H | CH₂(pyrimidin-2-yl) | H | Me | Me | O |

**[Table 929]**

| Compound Number | Y | W | Z | R¹ | R² | R³ | R¹⁰ | A |
|---|---|---|---|---|---|---|---|---|
| D-12427 | Me(R) | single bond | H | CH₂(pyrimidin-2-yl) | H | Me | Me | O |
| D-12428 | Me(S) | single bond | H | CH₂(pyrimidin-2-yl) | H | Me | Me | O |
| D-12429 | H | single bond | Me | -CH₂CH₂CH₂- | | Cl | Me | O |
| D-12430 | Me | single bond | H | -CH₂CH₂CH₂- | | Cl | Me | O |
| D-12431 | Me(R) | single bond | H | -CH₂CH₂CH₂- | | Cl | Me | O |
| D-12432 | Me(S) | single bond | H | -CH₂CH₂CH₂- | | Cl | Me | O |
| D-12433 | H | single bond | Me | -CH₂CH₂CH₂- | | Me | Me | O |
| D-12434 | Me | single bond | H | -CH₂CH₂CH₂- | | Me | Me | O |
| D-12435 | Me(R) | single bond | H | -CH₂CH₂CH₂- | | Me | Me | O |
| D-12436 | Me(S) | single bond | H | -CH₂CH₂CH₂- | | Me | Me | O |
| D-12437 | H | single bond | Me | -CH₂CH₂OCH₂CH₂- | | Cl | Me | O |
| D-12438 | Me | single bond | H | -CH₂CH₂OCH₂CH₂- | | Cl | Me | O |
| D-12439 | Me(R) | single bond | H | -CH₂CH₂OCH₂CH₂- | | Cl | Me | O |
| D-12440 | Me(S) | single bond | H | -CH₂CH₂OCH₂CH₂- | | Cl | Me | O |
| D-12441 | H | single bond | Me | -CH₂CH₂OCH₂CH₂- | | Me | Me | O |
| D-12442 | Me | single bond | H | -CH₂CH₂OCH₂CH₂- | | Me | Me | O |
| D-12443 | Me(R) | single bond | H | -CH₂CH₂OCH₂CH₂- | | Me | Me | O |
| D-12444 | Me(S) | single bond | H | -CH₂CH₂OCH₂CH₂- | | Me | Me | O |
| D-12445 | H | single bond | Me | Me | H | Cl | CH₂OMe | O |
| D-12446 | Me | single bond | H | Me | H | Cl | CH₂OMe | O |
| D-12447 | Me(R) | single bond | H | Me | H | Cl | CH₂OMe | O |
| D-12448 | Me(S) | single bond | H | Me | H | Cl | CH₂OMe | O |
| D-12449 | H | single bond | Me | Me | H | Me | CH₂OMe | O |
| D-12450 | Me | single bond | H | Me | H | Me | CH₂OMe | O |
| D-12451 | Me(R) | single bond | H | Me | H | Me | CH₂OMe | O |
| D-12452 | Me(S) | single bond | H | Me | H | Me | CH₂OMe | O |
| D-12453 | H | single bond | Me | CH₂CHF₂ | H | Cl | CH₂OMe | O |
| D-12454 | Me | single bond | H | CH₂CHF₂ | H | Cl | CH₂OMe | O |
| D-12455 | Me(R) | single bond | H | CH₂CHF₂ | H | Cl | CH₂OMe | O |
| D-12456 | Me(S) | single bond | H | CH₂CHF₂ | H | Cl | CH₂OMe | O |
| D-12457 | H | single bond | Me | CH₂CHF₂ | H | Me | CH₂OMe | O |
| D-12458 | Me | single bond | H | CH₂CHF₂ | H | Me | CH₂OMe | O |
| D-12459 | Me(R) | single bond | H | CH₂CHF₂ | H | Me | CH₂OMe | O |
| D-12460 | Me(S) | single bond | H | CH₂CHF₂ | H | Me | CH₂OMe | O |
| D-12461 | H | single bond | Me | *n* -Pr | H | Cl | CH₂OMe | O |
| D-12462 | Me | single bond | H | *n* -Pr | H | Cl | CH₂OMe | O |
| D-12463 | Me(R) | single bond | H | *n* -Pr | H | Cl | CH₂OMe | O |
| D-12464 | Me(S) | single bond | H | *n* -Pr | H | Cl | CH₂OMe | O |
| D-12465 | H | single bond | Me | *n* -Pr | H | Me | CH₂OMe | O |
| D-12466 | Me | single bond | H | *n* -Pr | H | Me | CH₂OMe | O |
| D-12467 | Me(R) | single bond | H | *n* -Pr | H | Me | CH₂OMe | O |
| D-12468 | Me(S) | single bond | H | *n* -Pr | H | Me | CH₂OMe | O |
| D-12469 | H | single bond | Me | *í* -Pr | H | Cl | CH₂OMe | O |
| D-12470 | Me | single bond | H | *i* -Pr | H | Cl | CH₂OMe | O |

**[Table 930]**

| Compound Number | Y | W | Z | R¹ | R² | R³ | R¹⁰ | A |
|---|---|---|---|---|---|---|---|---|
| D-12471 | Me(R) | single bond | H | *í* -Pr | H | Cl | CH₂OMe | O |
| D-12472 | Me(S) | single bond | H | *í* -Pr | H | Cl | CH₂OMe | O |
| D-12473 | H | single bond | Me | *í* -Pr | H | Me | CH₂OMe | O |
| D-12474 | Me | single bond | H | *í* -Pr | H | Me | CH₂OMe | O |
| D-12475 | Me(R) | single bond | H | *í* -Pr | H | Me | CH₂OMe | O |
| D-12476 | Me(S) | single bond | H | i-Pr | H | Me | CH₂OMe | O |
| D-12477 | H | single bond | Me | *c* -Pr(1-F) | H | Cl | CH₂OMe | O |
| D-12478 | Me | single bond | H | *c* -Pr(1-F) | H | Cl | CH₂OMe | O |
| D-12479 | Me(R) | single bond | H | *c* -Pr(1-F) | H | Cl | CH₂OMe | O |
| D-12480 | Me(S) | single bond | H | *c* -Pr(1-F) | H | Cl | CH₂OMe | O |
| D-12481 | H | single bond | Me | *c* -Pr(1-F) | H | Me | CH₂OMe | O |
| D-12482 | Me | single bond | H | *c* -Pr(1-F) | H | Me | CH₂OMe | O |
| D-12483 | Me(R) | single bond | H | *c* -Pr(1-F) | H | Me | CH₂OMe | O |
| D-12484 | Me(S) | single bond | H | *c* -Pr(1-F) | H | Me | CH₂OMe | O |
| D-12485 | H | single bond | Me | *c* -Pr(2,2-F₂) | H | Cl | CH₂OMe | O |
| D-12486 | Me | single bond | H | *c* -Pr(2,2-F₂) | H | Cl | CH₂OMe | O |
| D-12487 | Me(R) | single bond | H | *c* -Pr(2,2-F₂) | H | Cl | CH₂OMe | O |
| D-12488 | Me(S) | single bond | H | *c* -Pr(2,2-F₂) | H | Cl | CH₂OMe | O |
| D-12489 | H | single bond | Me | *c* -Pr(2,2-F₂) | H | Me | CH₂OMe | O |
| D-12490 | Me | single bond | H | *c* -Pr(2,2-F₂) | H | Me | CH₂OMe | O |
| D-12491 | Me(R) | single bond | H | *c* -Pr(2,2-F₂) | H | Me | CH₂OMe | O |
| D-12492 | Me(S) | single bond | H | *c* -Pr(2,2-F₂) | H | Me | CH₂OMe | O |
| D-12493 | H | single bond | Me | *c* -Pr(1-Cl) | H | Cl | CH₂OMe | O |
| D-12494 | Me | single bond | H | *c* -Pr(1-Cl) | H | Cl | CH₂OMe | O |
| D-12495 | Me(R) | single bond | H | *c* -Pr(1-Cl) | H | Cl | CH₂OMe | O |
| D-12496 | Me(S) | single bond | H | *c* -Pr(1-Cl) | H | Cl | CH₂OMe | O |
| D-12497 | H | single bond | Me | *c* -Pr(1-Cl) | H | Me | CH₂OMe | O |
| D-12498 | Me | single bond | H | *c* -Pr(1-Cl) | H | Me | CH₂OMe | O |
| D-12499 | Me(R) | single bond | H | *c* -Pr(1-Cl) | H | Me | CH₂OMe | O |
| D-12500 | Me(S) | single bond | H | *c* -Pr(1-Cl) | H | Me | CH₂OMe | O |
| D-12501 | H | single bond | Me | *c* -Pr(1-Br) | H | Cl | CH₂OMe | O |
| D-12502 | Me | single bond | H | c -Pr(1-Br) | H | Cl | CH₂OMe | O |
| D-12503 | Me(R) | single bond | H | c -Pr(1-Br) | H | Cl | CH₂OMe | O |
| D-12504 | Me(S) | single bond | H | c -Pr(1-Br) | H | Cl | CH₂OMe | O |
| D-12505 | H | single bond | Me | *c* -Pr(1-Br) | H | Me | CH₂OMe | O |
| D-12506 | Me | single bond | H | *c* -Pr(1-Br) | H | Me | CH₂OMe | O |
| D-12507 | Me(R) | single bond | H | *c* -Pr(1-Br) | H | Me | CH₂OMe | O |
| D-12508 | Me(S) | single bond | H | *c* -Pr(1-Br) | H | Me | CH₂OMe | O |
| D-12509 | H | single bond | Me | *c* -Pr(1-Me) | H | Cl | CH₂OMe | O |
| D-12510 | Me | single bond | H | *c* -Pr(1-Me) | H | Cl | CH₂OMe | O |
| D-12511 | Me(R) | single bond | H | *c* -Pr(1-Me) | H | Cl | CH₂OMe | O |
| D-12512 | Me(S) | single bond | H | *c* -Pr(1-Me) | H | Cl | CH₂OMe | O |
| D-12513 | H | single bond | Me | *c* -Pr(1-Me) | H | Me | CH₂OMe | O |
| D-12514 | Me | single bond | H | *c* -Pr(1-Me) | H | Me | CH₂OMe | O |

**[Table 931]**

| Compound Number | Y | W | Z | R¹ | R² | R³ | R¹⁰ | A |
|---|---|---|---|---|---|---|---|---|
| D-12515 | Me(R) | single bond | H | *c* -Pr(1-Me) | H | Me | CH₂OMe | O |
| D-12516 | Me(S) | single bond | H | *c* -Pr(1-Me) | H | Me | CH₂OMe | O |
| D-12517 | H | single bond | Me | *c* -Pr(1-CH₂OH) | H | Cl | CH₂OMe | O |
| D-12518 | Me | single bond | H | *c* -Pr(1-CH₂OH) | H | Cl | CH₂OMe | O |
| D-12519 | Me(R) | single bond | H | *c* -Pr(1-CH₂OH) | H | Cl | CH₂OMe | O |
| D-12520 | Me(S) | single bond | H | *c* -Pr(1-CH₂OH) | H | Cl | CH₂OMe | O |
| D-12521 | H | single bond | Me | *c* -Pr(1-CH₂OH) | H | Me | CH₂OMe | O |
| D-12522 | Me | single bond | H | *c* -Pr(1-CH₂OH) | H | Me | CH₂OMe | O |
| D-12523 | Me(R) | single bond | H | *c* -Pr(1-CH₂OH) | H | Me | CH₂OMe | O |
| D-12524 | Me(S) | single bond | H | *c* -Pr(1-CH₂OH) | H | Me | CH₂OMe | O |
| D-12525 | H | single bond | Me | *c* -Pr[1-CH₂OS(=O)₂Me] | H | Cl | CH₂OMe | O |
| D-12526 | Me | single bond | H | *c* -Pr[1-CH₂OS(=O)₂Me] | H | Cl | CH₂OMe | O |
| D-12527 | Me(R) | single bond | H | *c* -Pr[1-CH₂OS(=O)₂Me] | H | Cl | CH₂OMe | O |
| D-12528 | Me(S) | single bond | H | *c* -Pr[1-CH₂OS(=O)₂Me] | H | Cl | CH₂OMe | O |
| D-12529 | H | single bond | Me | *c* -Pr[1-CH₂OS(=O)₂Me] | H | Me | CH₂OMe | O |
| D-12530 | Me | single bond | H | *c* -Pr[1-CH₂OS(=O)₂Me] | H | Me | CH₂OMe | O |
| D-12531 | Me(R) | single bond | H | *c* -Pr[1-CH₂OS(=O)₂Me] | H | Me | CH₂OMe | O |
| D-12532 | Me(S) | single bond | H | *c* -Pr[1-CH₂OS(=O)₂Me] | H | Me | CH₂OMe | O |
| D-12533 | H | single bond | Me | *c* -Pr(1-CH₂OMe) | H | Cl | CH₂OMe | O |
| D-12534 | Me | single bond | H | *c* -Pr(1-CH₂OMe) | H | Cl | CH₂OMe | O |
| D-12535 | Me(R) | single bond | H | *c* -Pr(1-CH₂OMe) | H | Cl | CH₂OMe | O |
| D-12536 | Me(S) | single bond | H | *c* -Pr(1-CH₂OMe) | H | Cl | CH₂OMe | O |
| D-12537 | H | single bond | Me | *c* -Pr(1-CH₂OMe) | H | Me | CH₂OMe | O |
| D-12538 | Me | single bond | H | *c* -Pr(1-CH₂OMe) | H | Me | CH₂OMe | O |
| D-12539 | Me(R) | single bond | H | *c* -Pr(1-CH₂OMe) | H | Me | CH₂OMe | O |
| D-12540 | Me(S) | single bond | H | *c* -Pr(1-CH₂OMe) | H | Me | CH₂OMe | O |
| D-12541 | H | single bond | Me | *c* -Pr(1-CH₂OCN) | H | Cl | CH₂OMe | O |
| D-12542 | Me | single bond | H | *c* -Pr(1-CH₂OCN) | H | Cl | CH₂OMe | O |
| D-12543 | Me(R) | single bond | H | *c* -Pr(1-CH₂OCN) | H | Cl | CH₂OMe | O |
| D-12544 | Me(S) | single bond | H | *c* -Pr(1-CH₂OCN) | H | Cl | CH₂OMe | O |
| D-12545 | H | single bond | Me | *c* -Pr(1-CH₂OCN) | H | Me | CH₂OMe | O |
| D-12546 | Me | single bond | H | *c* -Pr(1-CH₂OCN) | H | Me | CH₂OMe | O |
| D-12547 | Me(R) | single bond | H | *c* -Pr(1-CH₂OCN) | H | Me | CH₂OMe | O |
| D-12548 | Me(S) | single bond | H | *c* -Pr(1-CH₂OCN) | H | Me | CH₂OMe | O |
| D-12549 | H | single bond | Me | *c* -Pr(1-CH₂SMe) | H | Cl | CH₂OMe | O |
| D-12550 | Me | single bond | H | *c* -Pr(1-CH₂SMe) | H | Cl | CH₂OMe | O |
| D-12551 | Me(R) | single bond | H | *c* -Pr(1-CH₂SMe) | H | Cl | CH₂OMe | O |
| D-12552 | Me(S) | single bond | H | *c* -Pr(1-CH₂SMe) | H | Cl | CH₂OMe | O |
| D-12553 | H | single bond | Me | *c* -Pr(1-CH₂SMe) | H | Me | CH₂OMe | O |
| D-12554 | Me | single bond | H | *c* -Pr(1-CH₂SMe) | H | Me | CH₂OMe | O |
| D-12555 | Me(R) | single bond | H | *c* -Pr(1-CH₂SMe) | H | Me | CH₂OMe | O |
| D-12556 | Me(S) | single bond | H | *c* -Pr(1-CH₂SMe) | H | Me | CH₂OMe | O |
| D-12557 | H | single bond | Me | *c* -Pr[1-CH₂S(=O)Me] | H | Cl | CH₂OMe | O |
| D-12558 | Me | single bond | H | *c* -Pr[1-CH₂S(=O)Me] | H | Cl | CH₂OMe | O |

**[Table 932]**

| Compound Number | Y | W | Z | R¹ | R² | R³ | R¹⁰ | A |
|---|---|---|---|---|---|---|---|---|
| D-12559 | Me(R) | single bond | H | *c* -Pr[1-CH₂S(=O)Me] | H | Cl | CH₂OMe | O |
| D-12560 | Me(S) | single bond | H | *c* -Pr[1-CH₂S(=O)Me] | H | Cl | CH₂OMe | O |
| D-12561 | H | single bond | Me | *c* -Pr[1-CH₂S(=O)Me] | H | Me | CH₂OMe | O |
| D-12562 | Me | single bond | H | *c* -Pr[1-CH₂S(=O)Me] | H | Me | CH₂OMe | O |
| D-12563 | Me(R) | single bond | H | *c* -Pr[1-CH₂S(=O)Me] | H | Me | CH₂OMe | O |
| D-12564 | Me(S) | single bond | H | *c* -Pr[1-CH₂S(=O)Me] | H | Me | CH₂OMe | O |
| D-12565 | H | single bond | Me | c -Pr[1-C H₂S(=O)₂Me] | H | Cl | CH₂OMe | O |
| D-12566 | Me | single bond | H | c -Pr[1-C H₂S(=O)₂Me] | H | Cl | CH₂OMe | O |
| D-12567 | Me(R) | single bond | H | c -Pr[1-C H₂S(=O)₂Me] | H | Cl | CH₂OMe | O |
| D-12568 | Me(S) | single bond | H | c -Pr[1-C H₂S(=O)₂Me] | H | Cl | CH₂OMe | O |
| D-12569 | H | single bond | Me | c -Pr[1-C H₂S(=O)₂Me] | H | Me | CH₂OMe | O |
| D-12570 | Me | single bond | H | c -Pr[1-C H₂S(=O)₂Me] | H | Me | CH₂OMe | O |
| D-12571 | Me(R) | single bond | H | c -Pr[1-C H₂S(=O)₂Me] | H | Me | CH₂OMe | O |
| D-12572 | Me(S) | single bond | H | c -Pr[1-C H₂S(=O)₂Me] | H | Me | CH₂OMe | O |
| D-12573 | H | single bond | Me | *c* -Pr(1-CH₂F) | H | Cl | CH₂OMe | O |
| D-12574 | Me | single bond | H | *c* -Pr(1-CH₂F) | H | Cl | CH₂OMe | O |
| D-12575 | Me(R) | single bond | H | *c* -Pr(1-CH₂F) | H | Cl | CH₂OMe | O |
| D-12576 | Me(S) | single bond | H | *c* -Pr(1-CH₂F) | H | Cl | CH₂OMe | O |
| D-12577 | H | single bond | Me | *c* -Pr(1-CH₂F) | H | Me | CH₂OMe | O |
| D-12578 | Me | single bond | H | *c* -Pr(1-CH₂F) | H | Me | CH₂OMe | O |
| D-12579 | Me(R) | single bond | H | *c* -Pr(1-CH₂F) | H | Me | CH₂OMe | O |
| D-12580 | Me(S) | single bond | H | *c* -Pr(1-CH₂F) | H | Me | CH₂OMe | O |
| D-12581 | H | single bond | Me | *c* -Pr(1-CHF₂) | H | Cl | CH₂OMe | O |
| D-12582 | Me | single bond | H | *c* -Pr(1-CHF₂) | H | Cl | CH₂OMe | O |
| D-12583 | Me(R) | single bond | H | *c* -Pr(1-CHF₂) | H | Cl | CH₂OMe | O |
| D-12584 | Me(S) | single bond | H | *c* -Pr(1-CHF₂) | H | Cl | CH₂OMe | O |
| D-12585 | H | single bond | Me | *c* -Pr(1-CHF₂) | H | Me | CH₂OMe | O |
| D-12586 | Me | single bond | H | *c* -Pr(1-CHF₂) | H | Me | CH₂OMe | O |
| D-12587 | Me(R) | single bond | H | *c* -Pr(1-CHF₂) | H | Me | CH₂OMe | O |
| D-12588 | Me(S) | single bond | H | *c* -Pr(1-CHF₂) | H | Me | CH₂OMe | O |
| D-12589 | H | single bond | Me | *c* -Pr(1-CF₃) | H | Cl | CH₂OMe | O |
| D-12590 | Me | single bond | H | *c* -Pr(1-CF₃) | H | Cl | CH₂OMe | O |
| D-12591 | Me(R) | single bond | H | *c* -Pr(1-CF₃) | H | Cl | CH₂OMe | O |
| D-12592 | Me(S) | single bond | H | *c* -Pr(1-CF₃) | H | Cl | CH₂OMe | O |
| D-12593 | H | single bond | Me | *c* -Pr(1-CF₃) | H | Me | CH₂OMe | O |
| D-12594 | Me | single bond | H | *c* -Pr(1-CF₃) | H | Me | CH₂OMe | O |
| D-12595 | Me(R) | single bond | H | *c* -Pr(1-CF₃) | H | Me | CH₂OMe | O |
| D-12596 | Me(S) | single bond | H | *c* -Pr(1-CF₃) | H | Me | CH₂OMe | O |
| D-12597 | H | single bond | Me | *c* -Pr(1-C≡CH) | H | Cl | CH₂OMe | O |
| D-12598 | Me | single bond | H | *c* -Pr(1-C≡CH) | H | Cl | CH₂OMe | O |
| D-12599 | Me(R) | single bond | H | *c* -Pr(1-C≡CH) | H | Cl | CH₂OMe | O |
| D-12600 | Me(S) | single bond | H | *c* -Pr(1-C≡CH) | H | Cl | CH₂OMe | O |
| D-12601 | H | single bond | Me | *c* -Pr(1-C≡CH) | H | Me | CH₂OMe | O |
| D-12602 | Me | single bond | H | *c* -Pr(1-C≡CH) | H | Me | CH₂OMe | O |

**[Table 933]**

| Compound Number | Y | W | Z | R¹ | R² | R³ | R¹⁰ | A |
|---|---|---|---|---|---|---|---|---|
| D-12603 | Me(R) | single bond | H | *c* -Pr(1-C≡CH) | H | Me | CH₂OMe | O |
| D-12604 | Me(S) | single bond | H | *c* -Pr(1-C≡CH) | H | Me | CH₂OMe | O |
| D-12605 | H | single bond | Me | *c* -Pr[1-C(=O)OEt] | H | Cl | CH₂OMe | O |
| D-12606 | Me | single bond | H | *c* -Pr[1-C(=O)OEt] | H | Cl | CH₂OMe | O |
| D-12607 | Me(R) | single bond | H | *c* -Pr[1-C(=O)OEt] | H | Cl | CH₂OMe | O |
| D-12608 | Me(S) | single bond | H | *c* -Pr{1-C(=O)OEt] | H | Cl | CH₂OMe | O |
| D-12609 | H | single bond | Me | *c* -Pr[1-C(=O)OEt] | H | Me | CH₂OMe | O |
| D-12610 | Me | single bond | H | *c* -Pr[1-C(=O)OEt] | H | Me | CH₂OMe | O |
| D-12611 | Me(R) | single bond | H | *c* -Pr[1-C(=O)OEt] | H | Me | CH₂OMe | O |
| D-12612 | Me(S) | single bond | H | *c* -Pr[1-C(=O)OEt] | H | Me | CH₂OMe | O |
| D-12613 | H | single bond | Me | *c* -Pr[1-C(=O)OH] | H | Cl | CH₂OMe | O |
| D-12614 | Me | single bond | H | *c* -Pr[1-C(=O)OH] | H | Cl | CH₂OMe | O |
| D-12615 | Me(R) | single bond | H | *c* -Pr[1-C(=O)OH] | H | Cl | CH₂OMe | O |
| D-12616 | Me(S) | single bond | H | *c* -Pr[1-C(=O)OH] | H | Cl | CH₂OMe | O |
| D-12617 | H | single bond | Me | *c* -Pr[1-C(=O)OH] | H | Me | CH₂OMe | O |
| D-12618 | Me | single bond | H | *c* -Pr[1-C(=O)OH] | H | Me | CH₂OMe | O |
| D-12619 | Me(R) | single bond | H | *c* -Pr[1-C(=O)OH] | H | Me | CH₂OMe | O |
| D-12620 | Me(S) | single bond | H | *c* -Pr[1-C(=O)OH] | H | Me | CH₂OMe | O |
| D-12621 | H | single bond | Me | *c* -Pr[1-C(=O)NH₂] | H | Cl | CH₂OMe | O |
| D-12622 | Me | single bond | H | *c* -Pr[1-C(=O)NH₂] | H | Cl | CH₂OMe | O |
| D-12623 | Me(R) | single bond | H | *c* -Pr[1-C(=O)NH₂] | H | Cl | CH₂OMe | O |
| D-12624 | Me(S) | single bond | H | *c* -Pr[1-C(=O)NH₂] | H | Cl | CH₂OMe | O |
| D-12625 | H | single bond | Me | *c* -Pr[1-C(=O)NH₂] | H | Me | CH₂OMe | O |
| D-12626 | Me | single bond | H | *c* -Pr[1-C(=O)NH₂] | H | Me | CH₂OMe | O |
| D-12627 | Me(R) | single bond | H | *c* -Pr[1-C(=O)NH₂] | H | Me | CH₂OMe | O |
| D-12628 | Me(S) | single bond | H | *c* -Pr[1-C(=O)NH₂] | H | Me | CH₂OMe | O |
| D-12629 | H | single bond | Me | *c* -Pr(1-OMe) | H | Cl | CH₂OMe | O |
| D-12630 | Me | single bond | H | *c* -Pr(1-OMe) | H | Cl | CH₂OMe | O |
| D-12631 | Me(R) | single bond | H | *c* -Pr(1-OMe) | H | Cl | CH₂OMe | O |
| D-12632 | Me(S) | single bond | H | *c* -Pr(1-OMe) | H | Cl | CH₂OMe | O |
| D-12633 | H | single bond | Me | *c* -Pr(1-OMe) | H | Me | CH₂OMe | O |
| D-12634 | Me | single bond | H | *c* -Pr(1-OMe) | H | Me | CH₂OMe | O |
| D-12635 | Me(R) | single bond | H | *c* -Pr(1-OMe) | H | Me | CH₂OMe | O |
| D-12636 | Me(S) | single bond | H | *c* -Pr(1-OMe) | H | Me | CH₂OMe | O |
| D-12637 | H | single bond | Me | *c* -Pr(1-SMe) | H | Cl | CH₂OMe | O |
| D-12638 | Me | single bond | H | c-Pr(1-SMe) | H | Cl | CH₂OMe | O |
| D-12639 | Me(R) | single bond | H | *c* -Pr(1-SMe) | H | Cl | CH₂OMe | O |
| D-12640 | Me(S) | single bond | H | *c* -Pr(1-SMe) | H | Cl | CH₂OMe | O |
| D-12641 | H | single bond | Me | c-Pr(1-SMe) | H | Me | CH₂OMe | O |
| D-12642 | Me | single bond | H | c-Pr(1-SMe) | H | Me | CH₂OMe | O |
| D-12643 | Me(R) | single bond | H | c-Pr(1-SMe) | H | Me | CH₂OMe | O |
| D-12644 | Me(S) | single bond | H | c-Pr(1-SMe) | H | Me | CH₂OMe | O |
| D-12645 | H | single bond | Me | *c* -Pr[1-S(=O)Me] | H | Cl | CH₂OMe | O |
| D-12646 | Me | single bond | H | *c* -Pr[1-S(=O)Me] | H | Cl | CH₂OMe | O |

**[Table 934]**

| Compound Number | Y | W | Z | R¹ | R² | R³ | R¹⁰ | A |
|---|---|---|---|---|---|---|---|---|
| D-12647 | Me(R) | single bond | H | *c* -Pr[1-S(=O)Me] | H | Cl | CH₂OMe | O |
| D-12648 | Me(S) | single bond | H | *c* -Pr[1-S(=O)Me] | H | Cl | CH₂OMe | O |
| D-12649 | H | single bond | Me | c -Pr[1-S(=O)Me] | H | Me | CH₂OMe | O |
| D-12650 | Me | single bond | H | *c* -Pr[1-S(=O)Me] | H | Me | CH₂OMe | O |
| D-12651 | Me(R) | single bond | H | *c* -Pr[1-S(=O)Me] | H | Me | CH₂OMe | O |
| D-12652 | Me(S) | single bond | H | *c* -Pr[1-S(=O)Me) | H | Me | CH₂OMe | O |
| D-12653 | H | single bond | Me | *c* -Pr[1-S(=O)₂Me] | H | Cl | CH₂OMe | O |
| D-12654 | Me | single bond | H | *c* -Pr[1-S(=O)₂Me] | H | Cl | CH₂OMe | O |
| D-12655 | Me(R) | single bond | H | *c* -Pr[1-S(=O)₂Me] | H | Cl | CH₂OMe | O |
| D-12656 | Me(S) | single bond | H | *c* -Pr[1-S(=O)₂Me] | H | Cl | CH₂OMe | O |
| D-12657 | H | single bond | Me | *c* -Pr[1-S(=O)₂Me] | H | Me | CH₂OMe | O |
| D-12658 | Me | single bond | H | *c* -Pr[1-S(=O)₂Me] | H | Me | CH₂OMe | O |
| D-12659 | Me(R) | single bond | H | *c* -Pr[1-S(=O)₂Me] | H | Me | CH₂OMe | O |
| D-12660 | Me(S) | single bond | H | *c* -Pr[1-S(=O)₂Me] | H | Me | CH₂OMe | O |
| D-12661 | H | single bond | Me | *c* -Pr[1-S(=O)₂CHF₂] | H | Cl | CH₂OMe | O |
| D-12662 | Me | single bond | H | *c* -Pr[1-S(=O)₂CHF₂] | H | Cl | CH₂OMe | O |
| D-12663 | Me(R) | single bond | H | *c* -Pr[1-S(=O)₂CHF₂] | H | Cl | CH₂OMe | O |
| D-12664 | Me(S) | single bond | H | *c* -Pr[1-S(=O)₂CHF₂] | H | Cl | CH₂OMe | O |
| D-12665 | H | single bond | Me | *c* -Pr[1-S(=O)₂CHF₂] | H | Me | CH₂OMe | O |
| D-12666 | Me | single bond | H | *c* -Pr[1-S(=O)₂CHF₂] | H | Me | CH₂OMe | O |
| D-12667 | Me(R) | single bond | H | *c* -Pr[1-S(=O)₂CHF₂] | H | Me | CH₂OMe | O |
| D-12668 | Me(S) | single bond | H | *c* -Pr[1-S(=O)₂CHF₂] | H | Me | CH₂OMe | O |
| D-12669 | H | single bond | Me | *c* -Pr[1-S(=O)₂CF₃] | H | Cl | CH₂OMe | O |
| D-12670 | Me | single bond | H | *c* -Pr[1-S(=O)₂CF₃] | H | Cl | CH₂OMe | O |
| D-12671 | Me(R) | single bond | H | *c* -Pr[1-S(=O)₂CF₃] | H | Cl | CH₂OMe | O |
| D-12672 | Me(S) | single bond | H | *c* -Pr[1-S(=O)₂CF₃] | H | Cl | CH₂OMe | O |
| D-12673 | H | single bond | Me | *c* -Pr[1-S(=O)₂CF₃] | H | Me | CH₂OMe | O |
| D-12674 | Me | single bond | H | *c -*Pr[1-S(=O)₂CF₃] | H | Me | CH₂OMe | O |
| D-12675 | Me(R) | single bond | H | *c* -Pr[1-S(=O)₂CF₃] | H | Me | CH₂OMe | O |
| D-12676 | Me(S) | single bond | H | *c* -Pr[1-S(=O)₂CF₃] | H | Me | CH₂OMe | O |
| D-12677 | H | single bond | Me | *c* -Pr[1-(5,6-dihydro-1,4,2-dioxazin-3-yl)] | H | Cl | CH₂OMe | O |
| D-12678 | Me | single bond | H | *c* -Pr[1-(5,6-dihydro-1,4,2-dioxazin-3-yl)] | H | Cl | CH₂OMe | O |
| D-12679 | Me(R) | single bond | H | *c* -Pr[1-(5,6-dihydro-1,4,2-dioxazin-3-yl)] | H | Cl | CH₂OMe | O |
| D-12680 | Me(S) | single bond | H | *c* -Pr[1-(5,6-dihydro-1,4,2-dioxazin-3-yl)] | H | Cl | CH₂OMe | O |
| D-12681 | H | single bond | Me | *c* -Pr[1-(5,6-dihydro-1,4,2-dioxazin-3-yl)] | H | Me | CH₂OMe | O |
| D-12682 | Me | single bond | H | *c* -Pr[1-(5,6-dihydro-1,4,2-dioxazin-3-yl)] | H | Me | CH₂OMe | O |
| D-12683 | Me(R) | single bond | H | *c* -Pr[1-(5,6-dihydro-1,4,2-dioxazin-3-yl)] | H | Me | CH₂OMe | O |
| D-12684 | Me(S) | single bond | H | *c* -Pr[1-(5,6-dihydro-1,4,2-dioxazin-3-yl)] | H | Me | CH₂OMe | O |
| D-12685 | H | single bond | Me | *c* -Pr[1-(5-hydroxy-1,3,4-oxadiazol-2-yl)] | H | Cl | CH₂OMe | O |
| D-12686 | Me | single bond | H | c -Pr[1-(5-hydroxy-1,3,4-oxadiazol-2-yl)] | H | Cl | CH₂OMe | O |
| D-12687 | Me(R) | single bond | H | c -Pr[1-(5-hydroxy-1,3,4-oxadiazol-2-yl)] | H | Cl | CH₂OMe | O |
| D-12688 | Me(S) | single bond | H | c -Pr[1-(5-hydroxy-1,3,4-oxadiazol-2-yl)] | H | Cl | CH₂OMe | O |
| D-12689 | H | single bond | Me | c -Pr[1-(5-hydroxy-1,3,4-oxadiazol-2-yl)] | H | Me | CH₂OMe | O |
| D-12690 | Me | single bond | H | c -Pr[1-(5-hydroxy-1,3,4-oxadiazol-2-yl)] | H | Me | CH₂OMe | O |

**[Table 935]**

| Compound Number | Y | W | Z | R¹ | R² | R³ | R¹⁰ | A |
|---|---|---|---|---|---|---|---|---|
| D-12691 | Me(R) | single bond | H | *c* -Pr[1-(5-hydroxy-1,3,4-oxadiazol-2-yl)] | H | Me | CH₂OMe | O |
| D-12692 | Me(S) | single bond | H | *c* -Pr[1-(5-hydroxy-1,3,4-oxadiazol-2-yl)] | H | Me | CH₂OMe | O |
| D-12693 | H | single bond | Me | c -Pr[1-(pyri midin-2-yl)] | H | Cl | CH₂OMe | O |
| D-12694 | Me | single bond | H | c -Pr[1-(pyri midin-2-yl)] | H | Cl | CH₂OMe | O |
| D-12695 | Me(R) | single bond | H | c -Pr[1-(pyri midin-2-yl)] | H | Cl | CH₂OMe | O |
| D-12696 | Me(S) | single bond | H | c -Pr[1-(pyri midin-2-yl)] | H | Cl | CH₂OMe | O |
| D-12697 | H | single bond | Me | c -Pr[1-(pyri midin-2-yl)] | H | Me | CH₂OMe | O |
| D-12698 | Me | single bond | H | c -Pr[1-(pyri midin-2-yl)] | H | Me | CH₂OMe | O |
| D-12699 | Me(R) | single bond | H | c -Pr[1-(pyri midin-2-yl)] | H | Me | CH₂OMe | O |
| D-12700 | Me(S) | single bond | H | c -Pr[1-(pyri midin-2-yl)] | H | Me | CH₂OMe | O |
| D-12701 | H | single bond | Me | t-Bu | H | Cl | CH₂OMe | O |
| D-12702 | Me | single bond | H | t-Bu | H | Cl | CH₂OMe | O |
| D-12703 | Me(R) | single bond | H | t-Bu | H | Cl | CH₂OMe | O |
| D-12704 | Me(S) | single bond | H | t-Bu | H | Cl | CH₂OMe | O |
| D-12705 | H | single bond | Me | t-Bu | H | Me | CH₂OMe | O |
| 0-12706 | Me | single bond | H | t-Bu | H | Me | CH₂OMe | O |
| D-12707 | Me(R) | single bond | H | t-Bu | H | Me | CH₂OMe | O |
| D-12708 | Me(S) | single bond | H | t-Bu | H | Me | CH₂OMe | O |
| D-12709 | H | single bond | Me | *c* -Bu | H | Cl | CH₂OMe | O |
| D-12710 | Me | single bond | H | *c* -Bu | H | Cl | CH₂OMe | O |
| D-12711 | Me(R) | single bond | H | *c* -Bu | H | Cl | CH₂OMe | O |
| D-12712 | Me(S) | single bond | H | *c* -Bu | H | Cl | CH₂OMe | O |
| D-12713 | H | single bond | Me | *c* -Bu | H | Me | CH₂OMe | O |
| D-12714 | Me | single bond | H | *c* -Bu | H | Me | CH₂OMe | O |
| D-12715 | Me(R) | single bond | H | *c* -Bu | H | Me | CH₂OMe | O |
| D-12716 | Me(S) | single bond | H | *c* -Bu | H | Me | CH₂OMe | O |
| D-12717 | H | single bond | Me | c-Bu(1-CN) | H | Cl | CH₂OMe | O |
| D-12718 | Me | single bond | H | c-Bu(1-CN) | H | Cl | CH₂OMe | O |
| D-12719 | Me(R) | single bond | H | c-Bu(1-CN) | H | Cl | CH₂OMe | O |
| D-12720 | Me(S) | single bond | H | c-Bu(1-CN) | H | Cl | CH₂OMe | O |
| D-12721 | H | single bond | Me | c-Bu(1-CN) | H | Me | CH₂OMe | O |
| D-12722 | Me | single bond | H | c-Bu(1-CN) | H | Me | CH₂OMe | O |
| D-12723 | Me(R) | single bond | H | c-Bu(1-CN) | H | Me | CH₂OMe | O |
| D-12724 | Me(S) | single bond | H | c-Bu(1-CN) | H | Me | CH₂OMe | O |
| D-12725 | H | single bond | Me | *c* -Bu(3,3-F₂) | H | Cl | CH₂OMe | O |
| D-12726 | Me | single bond | H | *c* -Bu(3,3-F₂) | H | Cl | CH₂OMe | O |
| D-12727 | Me(R) | single bond | H | *c* -Bu(3,3-F₂) | H | Cl | CH₂OMe | O |
| D-12728 | Me(S) | single bond | H | *c* -Bu(3,3-F₂) | H | Cl | CH₂OMe | O |
| D-12729 | H | single bond | Me | *c* -Bu(3,3-F₂) | H | Me | CH₂OMe | O |
| D-12730 | Me | single bond | H | *c* -Bu(3,3-F₂) | H | Me | CH₂OMe | O |
| D-12731 | Me(R) | single bond | H | *c* -Bu(3,3-F₂) | H | Me | CH₂OMe | O |
| D-12732 | Me(S) | single bond | H | *c* -Bu(3,3-F₂) | H | Me | CH₂OMe | O |
| D-12733 | H | single bond | Me | oxetan-3-yl | H | Cl | CH₂OMe | O |
| D-12734 | Me | single bond | H | oxetan-3-yl | H | Cl | CH₂OMe | O |

**[Table 936]**

| Compound Number | Y | W | Z | R¹ | R² | R³ | R¹⁰ | A |
|---|---|---|---|---|---|---|---|---|
| D-12735 | Me(R) | single bond | H | oxetan-3-yl | H | Cl | CH₂OMe | O |
| D-12736 | Me(S) | single bond | H | oxetan-3-yl | H | Cl | CH₂OMe | O |
| D-12737 | H | single bond | Me | oxetan-3-yl | H | Me | CH₂OMe | O |
| D-12738 | Me | single bond | H | oxetan-3-yl | H | Me | CH₂OMe | O |
| D-12739 | Me(R) | single bond | H | oxetan-3-yl | H | Me | CH₂OMe | O |
| D-12740 | Me(S) | single bond | H | oxetan-3-yl | H | Me | CH₂OMe | O |
| D-12741 | H | single bond | Me | oxetan(3-CN)-3-yl | H | Cl | CH₂OMe | O |
| D-12742 | Me | single bond | H | oxetan(3-CN)-3-yl | H | Cl | CH₂OMe | O |
| D-12743 | Me(R) | single bond | H | oxetan(3-CN)-3-yl | H | Cl | CH₂OMe | O |
| D-12744 | Me(S) | single bond | H | oxetan(3-CN)-3-yl | H | Cl | CH₂OMe | O |
| D-12745 | H | single bond | Me | oxetan(3-CN)-3-yl | H | Me | CH₂OMe | O |
| D-12746 | Me | single bond | H | oxetan(3-CN)-3-yl | H | Me | CH₂OMe | O |
| D-12747 | Me(R) | single bond | H | oxetan(3-CN)-3-yl | H | Me | CH₂OMe | O |
| D-12748 | Me(S) | single bond | H | oxetan(3-CN)-3-yl | H | Me | CH₂OMe | O |
| D-12749 | H | single bond | Me | azetidin[1-C(=O)Me]-3-yl | H | Cl | CH₂OMe | O |
| D-12750 | Me | single bond | H | azetidin[1-C(=O)Me]-3-yl | H | Cl | CH₂OMe | O |
| D-12751 | Me(R) | single bond | H | azetidin[1-C(=O)Me]-3-yl | H | Cl | CH₂OMe | O |
| D-12752 | Me(S) | single bond | H | azetidin[1-C(=O)Me]-3-yl | H | Cl | CH₂OMe | O |
| D-12753 | H | single bond | Me | azetidin[1-C(=0)Me]-3-yl | H | Me | CH₂OMe | O |
| D-12754 | Me | single bond | H | azetidin[1-C(=O)Me]-3-yl | H | Me | CH₂OMe | O |
| D-12755 | Me(R) | single bond | H | azetidin[1-C(=0)Me]-3-yl | H | Me | CH₂OMe | O |
| D-12756 | Me(S) | single bond | H | azetidin[1-C(=O)Me]-3-yl | H | Me | CH₂OMe | O |
| D-12757 | H | single bond | Me | azetidin[1-S(=O)₂Me]-3-yl | H | Cl | CH₂OMe | O |
| D-12758 | Me | single bond | H | azetidin[1-S(=O)₂Me]-3-yl | H | Cl | CH₂OMe | O |
| D-12759 | Me(R) | single bond | H | azetidin[1-S(=O)₂Me]-3-yl | H | Cl | CH₂OMe | O |
| D-12760 | Me(S) | single bond | H | azetidin[1-S(=O)₂Me]-3-yl | H | Cl | CH₂OMe | O |
| D-12761 | H | single bond | Me | azetidin[1-S(=O)₂Me]-3-yl | H | Me | CH₂OMe | O |
| D-12762 | Me | single bond | H | azetidin[1-S(=O)₂Me]-3-yl | H | Me | CH₂OMe | O |
| D-12763 | Me(R) | single bond | H | azetidin[1-S(=O)₂Me]-3-yl | H | Me | CH₂OMe | O |
| D-12764 | Me(S) | single bond | H | azetidin[1-S(=O)₂Me]-3-yl | H | Me | CH₂OMe | O |
| D-12765 | H | single bond | Me | azetidin[1-S(=O)₂CHF₂]-3-yl | H | Cl | CH₂OMe | O |
| D-12766 | Me | single bond | H | azetidin[1-S(=O)₂CHF₂]-3-yl | H | Cl | CH₂OMe | O |
| D-12767 | Me(R) | single bond | H | azetidin[1-S(=O)₂CHF₂]-3-yl | H | Cl | CH₂OMe | O |
| D-12768 | Me(S) | single bond | H | azetidin[1-S(=O)₂CHF₂]-3-yl | H | Cl | CH₂OMe | O |
| D-12769 | H | single bond | Me | azetidin[1-S(=O)₂CHF₂]-3-yl | H | Me | CH₂OMe | O |
| D-12770 | Me | single bond | H | azetidin[1-S(=O)₂CHF₂]-3-yl | H | Me | CH₂OMe | O |
| D-12771 | Me(R) | single bond | H | azetidin[1-S(=O)₂CHF₂]-3-yl | H | Me | CH₂OMe | O |
| D-12772 | Me(S) | single bond | H | azetidin[1-S(=O)₂CHF₂]-3-yl | H | Me | CH₂OMe | O |
| D-12773 | H | single bond | Me | *c*-Pen | H | Cl | CH₂OMe | O |
| D-12774 | Me | single bond | H | *c*-Pen | H | Cl | CH₂OMe | O |
| D-12775 | Me(R) | single bond | H | *c*-Pen | H | Cl | CH₂OMe | O |
| D-12776 | Me(S) | single bond | H | *c*-Pen | H | Cl | CH₂OMe | O |
| D-12777 | H | single bond | Me | *c*-Pen | H | Me | CH₂OMe | O |
| D-12778 | Me | single bond | H | *c-*Pen | H | Me | CH₂OMe | O |

**[Table 937]**

| Compound Number | Y | W | Z | R¹ | R² | R³ | R¹⁰ | A |
|---|---|---|---|---|---|---|---|---|
| D-12779 | Me(R) | single bond | H | *c*-Pen | H | Me | CH₂OMe | O |
| D-12780 | Me(S) | single bond | H | *c*-Pen | H | Me | CH₂OMe | O |
| D-12781 | H | single bond | Me | tetrahydrofuran-3-yl | H | Cl | CH₂OMe | O |
| D-12782 | Me | single bond | H | tetrahydrofuran-3-yl | H | Cl | CH₂OMe | O |
| D-12783 | Me(R) | single bond | H | tetrahydrofuran-3-yl | H | Cl | CH₂OMe | O |
| D-12784 | Me(S) | single bond | H | tetrahydrofuran-3-yl | H | Cl | CH₂OMe | O |
| D-12785 | H | single bond | Me | tetrahydrofuran-3-yl | H | Me | CH₂OMe | O |
| D-12786 | Me | single bond | H | tetrahydrofuran-3-yl | H | Me | CH₂OMe | O |
| D-12787 | Me(R) | single bond | H | tetrahydrofuran-3-yl | H | Me | CH₂OMe | O |
| D-12788 | Me(S) | single bond | H | tetrahydrofuran-3-yl | H | Me | CH₂OMe | O |
| D-12789 | H | single bond | Me | tetrahydrothiophen-3-yl | H | Cl | CH₂OMe | O |
| D-12790 | Me | single bond | H | tetrahydrothiophen-3-yl | H | Cl | CH₂OMe | O |
| D-12791 | Me(R) | single bond | H | tetrahydrothiophen-3-yl | H | Cl | CH₂OMe | O |
| D-12792 | Me(S) | single bond | H | tetrahydrothiophen-3-yl | H | Cl | CH₂OMe | O |
| D-12793 | H | single bond | Me | tetrahydrothiophen-3-yl | H | Me | CH₂OMe | O |
| D-12794 | Me | single bond | H | tetrahydrothiophen-3-yl | H | Me | CH₂OMe | O |
| D-12795 | Me(R) | single bond | H | tetrahydrothiophen-3-yl | H | Me | CH₂OMe | O |
| D-12796 | Me(S) | single bond | H | tetrahydrothiophen-3-yl | H | Me | CH₂OMe | O |
| D-12797 | H | single bond | Me | *c*-Pen(1-CN) | H | Cl | CH₂OMe | O |
| D-12798 | Me | single bond | H | c-Pen(1-CN) | H | Cl | CH₂OMe | O |
| D-12799 | Me(R) | single bond | H | c-Pen(1-CN) | H | Cl | CH₂OMe | O |
| D-12800 | Me(S) | single bond | H | c-Pen(1-CN) | H | Cl | CH₂OMe | O |
| D-12801 | H | single bond | Me | c-Pen(1-CN) | H | Me | CH₂OMe | O |
| D-12802 | Me | single bond | H | c-Pen(1-CN) | H | Me | CH₂OMe | O |
| D-12803 | Me(R) | single bond | H | c-Pen(1-CN) | H | Me | CH₂OMe | O |
| D-12804 | Me(S) | single bond | H | c-Pen(1-CN) | H | Me | CH₂OMe | O |
| D-12805 | H | single bond | Me | tetrahydrofuran(3-CN)-3-yl | H | Cl | CH₂OMe | O |
| D-12806 | Me | single bond | H | tetrahydrofuran(3-CN)-3-yl | H | Cl | CH₂OMe | O |
| D-12807 | Me(R) | single bond | H | tetrahydrofuran(3-CN)-3-yl | H | Cl | CH₂OMe | O |
| D-12808 | Me(S) | single bond | H | tetrahydrofuran(3-CN)-3-yl | H | Cl | CH₂OMe | O |
| D-12809 | H | single bond | Me | tetrahydrofuran(3-CN)-3-yl | H | Me | CH₂OMe | O |
| D-12810 | Me | single bond | H | tetrahydrofuran(3-CN)-3-yl | H | Me | CH₂OMe | O |
| D-12811 | Me(R) | single bond | H | tetrahydrofuran(3-CN)-3-yl | H | Me | CH₂OMe | O |
| D-12812 | Me(S) | single bond | H | tetrahydrofuran(3-CN)-3-yl | H | Me | CH₂OMe | O |
| D-12813 | H | single bond | Me | tetrahydrothiophen(3-CN)-3-yl | H | Cl | CH₂OMe | O |
| D-12814 | Me | single bond | H | tetrahydrothiophen(3-CN)-3-yl | H | Cl | CH₂OMe | O |
| D-12815 | Me(R) | single bond | H | tetrahydrothiophen(3-CN)-3-yl | H | Cl | CH₂OMe | O |
| D-12816 | Me(S) | single bond | H | tetrahydrothiophen(3-CN)-3-yl | H | Cl | CH₂OMe | O |
| D-12817 | H | single bond | Me | tetrahydrothiophen(3-CN)-3-yl | H | Me | CH₂OMe | O |
| D-12818 | Me | single bond | H | tetrahydrothiophen(3-CN)-3-yl | H | Me | CH₂OMe | O |
| D-12819 | Me(R) | single bond | H | tetrahydrothiophen(3-CN)-3-yl | H | Me | CH₂OMe | O |
| D-12820 | Me(S) | single bond | H | tetrahydrothiophen(3-CN)-3-yl | H | Me | CH₂OMe | O |
| D-12821 | H | single bond | Me | bicyclo[1.1.1]pentan-1-yl | H | Cl | CH₂OMe | O |
| D-12822 | Me | single bond | H | bicyclo[1.1.1]pentan-1-yl | H | Cl | CH₂OMe | O |

**[Table 938]**

| Compound Number | Y | W | Z | R¹ | R² | R³ | R¹⁰ | A |
|---|---|---|---|---|---|---|---|---|
| D-12823 | Me(R) | single bond | H | bicyclo[1.1.1]pentan-1-yl | H | Cl | CH₂OMe | O |
| D-12824 | Me(S) | single bond | H | bicyclo[1.1.1]pentan-1-yl | H | Cl | CH₂OMe | O |
| D-12825 | H | single bond | Me | bicyclo[1.1.1]pentan-1-yl | H | Me | CH₂OMe | O |
| D-12826 | Me | single bond | H | bicyclo[1.1.1]pentan-1-yl | H | Me | CH₂OMe | O |
| D-12827 | Me(R) | single bond | H | bicyclo[1.1.1]pentan-1-yl | H | Me | CH₂OMe | O |
| D-12828 | Me(S) | single bond | H | bicyclo[1.1.1]pentan-1-yl | H | Me | CH₂OMe | O |
| D-12829 | H | single bond | Me | *c*-Hex | H | Cl | CH₂OMe | O |
| D-12830 | Me | single bond | H | *c*-Hex | H | Cl | CH₂OMe | O |
| D-12831 | Me(R) | single bond | H | *c*-Hex | H | Cl | CH₂OMe | O |
| D-12832 | Me(S) | single bond | H | *c*-Hex | H | Cl | CH₂OMe | O |
| D-12833 | H | single bond | Me | *c*-Hex | H | Me | CH₂OMe | O |
| D-12834 | Me | single bond | H | *c*-Hex | H | Me | CH₂OMe | O |
| D-12835 | Me(R) | single bond | H | *c*-Hex | H | Me | CH₂OMe | O |
| D-12836 | Me(S) | single bond | H | *c*-Hex | H | Me | CH₂OMe | O |
| D-12837 | H | single bond | Me | tetrahydropyran-4-yl | H | Cl | CH₂OMe | O |
| D-12838 | Me | single bond | H | tetrahydropyran-4-yl | H | Cl | CH₂OMe | O |
| D-12839 | Me(R) | single bond | H | tetrahydropyran-4-yl | H | Cl | CH₂OMe | O |
| D-12840 | Me(S) | single bond | H | tetrahydropyran-4-yl | H | Cl | CH₂OMe | O |
| D-12841 | H | single bond | Me | tetrahydropyran-4-yl | H | Me | CH₂OMe | O |
| D-12842 | Me | single bond | H | tetrahydropyran-4-yl | H | Me | CH₂OMe | O |
| D-12843 | Me(R) | single bond | H | tetrahydropyran-4-yl | H | Me | CH₂OMe | O |
| D-12844 | Me(S) | single bond | H | tetrahydropyran-4-yl | H | Me | CH₂OMe | O |
| D-12845 | H | single bond | Me | tetrahydrothiopyran-4-yl | H | Cl | CH₂OMe | O |
| D-12846 | Me | single bond | H | tetrahydrothiopyran-4-yl | H | Cl | CH₂OMe | O |
| D-12847 | Me(R) | single bond | H | tetrahydrothiopyran-4-yl | H | Cl | CH₂OMe | O |
| D-12848 | Me(S) | single bond | H | tetrahydrothiopyran-4-yl | H | Cl | CH₂OMe | O |
| D-12849 | H | single bond | Me | tetrahydrothiopyran-4-yl | H | Me | CH₂OMe | O |
| D-12850 | Me | single bond | H | tetrahydrothiopyran-4-yl | H | Me | CH₂OMe | O |
| D-12851 | Me(R) | single bond | H | tetrahydrothiopyran-4-yl | H | Me | CH₂OMe | O |
| D-12852 | Me(S) | single bond | H | tetrahydrothiopyran-4-yl | H | Me | CH₂OMe | O |
| D-12853 | H | single bond | Me | *c*-Hex(1-CN) | H | Cl | CH₂OMe | O |
| D-12854 | Me | single bond | H | *c*-Hex(1-CN) | H | Cl | CH₂OMe | O |
| D-12855 | Me(R) | single bond | H | *c*-Hex(1-CN) | H | Cl | CH₂OMe | O |
| D-12856 | Me(S) | single bond | H | *c*-Hex(1-CN) | H | Cl | CH₂OMe | O |
| D-12857 | H | single bond | Me | *c*-Hex(1-CN) | H | Me | CH₂OMe | O |
| D-12858 | Me | single bond | H | *c*-Hex(1-CN) | H | Me | CH₂OMe | O |
| D-12859 | Me(R) | single bond | H | *c*-Hex(1-CN) | H | Me | CH₂OMe | O |
| D-12860 | Me(S) | single bond | H | *c*-Hex(1-CN) | H | Me | CH₂OMe | O |
| D-12861 | H | single bond | Me | tetrahydropyran(4-CN)-4-yl | H | Cl | CH₂OMe | O |
| D-12862 | Me | single bond | H | tetrahydropyran(4-CN)-4-yl | H | Cl | CH₂OMe | O |
| D-12863 | Me(R) | single bond | H | tetrahydropyran(4-CN)-4-yl | H | Cl | CH₂OMe | O |
| D-12864 | Me(S) | single bond | H | tetrahydropyran(4-CN)-4-yl | H | Cl | CH₂OMe | O |
| D-12865 | H | single bond | Me | tetrahydropyran(4-CN)-4-yl | H | Me | CH₂OMe | O |
| D-12866 | Me | single bond | H | tetrahydropyran(4-CN)-4-yl | H | Me | CH₂OMe | O |

**[Table 939]**

| Compound Number | Y | W | Z | R¹ | R² | R³ | R¹⁰ | A |
|---|---|---|---|---|---|---|---|---|
| D-12867 | Me(R) | single bond | H | tetrahydropyran(4-CN)-4-yl | H | Me | CH₂OMe | O |
| D-12868 | Me(S) | single bond | H | tetrahydropyran(4-CN)-4-yl | H | Me | CH₂OMe | O |
| D-12869 | H | single bond | Me | tetrahydrothiopyran(4-CN)-4-yl | H | Cl | CH₂OMe | O |
| D-12870 | Me | single bond | H | tetrahydrothiopyran(4-CN)-4-yl | H | Cl | CH₂OMe | O |
| D-12871 | Me(R) | single bond | H | tetrahydrothiopyran(4-CN)-4-yl | H | Cl | CH₂OMe | O |
| D-12872 | Me(S) | single bond | H | tetrahydrothiopyran(4-CN)-4-yl | H | Cl | CH₂OMe | O |
| D-12873 | H | single bond | Me | tetrahydrothiopyran(4-CN)-4-yl | H | Me | CH₂OMe | O |
| D-12874 | Me | single bond | H | tetrahydrothiopyran(4-CN)-4-yl | H | Me | CH₂OMe | O |
| D-12875 | Me(R) | single bond | H | tetrahydrothiopyran(4-CN)-4-yl | H | Me | CH₂OMe | O |
| D-12876 | Me(S) | single bond | H | tetrahydrothiopyran(4-CN)-4-yl | H | Me | CH₂OMe | O |
| D-12877 | H | single bond | Me | CH₂(*c*-Pr) | H | Cl | CH₂OMe | O |
| D-12878 | Me | single bond | H | CH₂(*c*-Pr) | H | Cl | CH₂OMe | O |
| D-12879 | Me(R) | single bond | H | CH₂(*c*-Pr) | H | Cl | CH₂OMe | O |
| D-12880 | Me(S) | single bond | H | CH₂(*c*-Pr) | H | Cl | CH₂OMe | O |
| D-12881 | H | single bond | Me | CH₂(*c*-Pr) | H | Me | CH₂OMe | O |
| D-12882 | Me | single bond | H | CH₂(*c*-Pr) | H | Me | CH₂OMe | O |
| D-12883 | Me(R) | single bond | H | CH₂(*c*-Pr) | H | Me | CH₂OMe | O |
| D-12884 | Me(S) | single bond | H | CH₂(*c*-Pr) | H | Me | CH₂OMe | O |
| D-12885 | H | single bond | Me | CH₂[*c*-Pr(1-CN)] | H | Cl | CH₂OMe | O |
| D-12886 | Me | single bond | H | CH₂[*c*-Pr(1-CN)] | H | Cl | CH₂OMe | O |
| D-12887 | Me(R) | single bond | H | CH₂[*c*-Pr(1-CN)] | H | Cl | CH₂OMe | O |
| D-12888 | Me(S) | single bond | H | CH₂[*c*-Pr(1-CN)] | H | Cl | CH₂OMe | O |
| D-12889 | H | single bond | Me | CH₂[*c*-Pr(1-CN)] | H | Me | CH₂OMe | O |
| D-12890 | Me | single bond | H | CH₂[*c*-Pr(1-CN)] | H | Me | CH₂OMe | O |
| D-12891 | Me(R) | single bond | H | CH₂[*c*-Pr(1-CN)] | H | Me | CH₂OMe | O |
| D-12892 | Me(S) | single bond | H | CH₂[*c*-Pr(1-CN)] | H | Me | CH₂OMe | O |
| D-12893 | H | single bond | Me | CH(Me)(*c*-Pr) | H | Cl | CH₂OMe | O |
| D-12894 | Me | single bond | H | CH(Me)(*c*-Pr) | H | Cl | CH₂OMe | O |
| D-12895 | Me(R) | single bond | H | CH(Me)(*c*-Pr) | H | Cl | CH₂OMe | O |
| D-12896 | Me(S) | single bond | H | CH(Me)(*c*-Pr) | H | Cl | CH₂OMe | O |
| D-12897 | H | single bond | Me | CH(Me)(*c*-Pr) | H | Me | CH₂OMe | O |
| D-12898 | Me | single bond | H | CH(Me)(*c*-Pr) | H | Me | CH₂OMe | O |
| D-12899 | Me(R) | single bond | H | CH(Me)(*c*-Pr) | H | Me | CH₂OMe | O |
| D-12900 | Me(S) | single bond | H | CH(Me)(*c*-Pr) | H | Me | CH₂OMe | O |
| D-12901 | H | single bond | Me | CH(Me)CH₂OMe | H | Cl | CH₂OMe | O |
| D-12902 | Me | single bond | H | CH(Me)CH₂OMe | H | Cl | CH₂OMe | O |
| D-12903 | Me(R) | single bond | H | CH(Me)CH₂OMe | H | Cl | CH₂OMe | O |
| D-12904 | Me(S) | single bond | H | CH(Me)CH₂OMe | H | Cl | CH₂OMe | O |
| 0-12905 | H | single bond | Me | CH(Me)CH₂OMe | H | Me | CH₂OMe | O |
| 0-12906 | Me | single bond | H | CH(Me)CH₂OMe | H | Me | CH₂OMe | O |
| D-12907 | Me(R) | single bond | H | CH(Me)CH₂OMe | H | Me | CH₂OMe | O |
| 0-12908 | Me(S) | single bond | H | CH(Me)CH₂OMe | H | Me | CH₂OMe | O |
| D-12909 | H | single bond | Me | CH₂CH=CH₂ | H | Cl | CH₂OMe | O |
| D-12910 | Me | single bond | H | CH₂CH=CH₂ | H | Cl | CH₂OMe | O |

**[Table 940]**

| Compound Number | Y | W | Z | R¹ | R² | R³ | R¹⁰ | A |
|---|---|---|---|---|---|---|---|---|
| D-12911 | Me(R) | single bond | H | CH₂CH=CH₂ | H | Cl | CH₂OMe | O |
| D-12912 | Me(S) | single bond | H | CH₂CH=CH₂ | H | Cl | CH₂OMe | O |
| D-12913 | H | single bond | Me | CH₂CH=CH₂ | H | Me | CH₂OMe | O |
| D-12914 | Me | single bond | H | CH₂CH=CH₂ | H | Me | CH₂OMe | O |
| D-12915 | Me(R) | single bond | H | CH₂CH=CH₂ | H | Me | CH₂OMe | O |
| D-12916 | Me(S) | single bond | H | CH₂CH=CH₂ | H | Me | CH₂OMe | O |
| D-12917 | H | single bond | Me | CH₂C≡CH | H | Cl | CH₂OMe | O |
| D-12918 | Me | single bond | H | CH₂C≡CH | H | Cl | CH₂OMe | O |
| D-12919 | Me(R) | single bond | H | CH₂C≡CH | H | Cl | CH₂OMe | O |
| D-12920 | Me(S) | single bond | H | CH₂C≡CH | H | Cl | CH₂OMe | O |
| D-12921 | H | single bond | Me | CH₂C≡CH | H | Me | CH₂OMe | O |
| D-12922 | Me | single bond | H | CH₂C≡CH | H | Me | CH₂OMe | O |
| D-12923 | Me(R) | single bond | H | CH₂C≡CH | H | Me | CH₂OMe | O |
| D-12924 | Me(S) | single bond | H | CH₂C≡CH | H | Me | CH₂OMe | O |
| D-12925 | H | single bond | Me | CH₂CN | H | Cl | CH₂OMe | O |
| D-12926 | Me | single bond | H | CH₂CN | H | Cl | CH₂OMe | O |
| D-12927 | Me(R) | single bond | H | CH₂CN | H | Cl | CH₂OMe | O |
| D-12928 | Me(S) | single bond | H | CH₂CN | H | Cl | CH₂OMe | O |
| D-12929 | H | single bond | Me | CH₂CN | H | Me | CH₂OMe | O |
| D-12930 | Me | single bond | H | CH₂CN | H | Me | CH₂OMe | O |
| D-12931 | Me(R) | single bond | H | CH₂CN | H | Me | CH₂OMe | O |
| D-12932 | Me(S) | single bond | H | CH₂CN | H | Me | CH₂OMe | O |
| D-12933 | H | single bond | Me | CH(Me)CN | H | Cl | CH₂OMe | O |
| D-12934 | Me | single bond | H | CH(Me)CN | H | Cl | CH₂OMe | O |
| D-12935 | Me(R) | single bond | H | CH(Me)CN | H | Cl | CH₂OMe | O |
| D-12936 | Me(S) | single bond | H | CH(Me)CN | H | Cl | CH₂OMe | O |
| D-12937 | H | single bond | Me | CH(Me)CN | H | Me | CH₂OMe | O |
| D-12938 | Me | single bond | H | CH(Me)CN | H | Me | CH₂OMe | O |
| D-12939 | Me(R) | single bond | H | CH(Me)CN | H | Me | CH₂OMe | O |
| D-12940 | Me(S) | single bond | H | CH(Me)CN | H | Me | CH₂OMe | O |
| D-12941 | H | single bond | Me | C(Me)₂CN | H | Cl | CH₂OMe | O |
| D-12942 | Me | single bond | H | C(Me)₂CN | H | Cl | CH₂OMe | O |
| D-12943 | Me(R) | single bond | H | C(Me)₂CN | H | Cl | CH₂OMe | O |
| D-12944 | Me(S) | single bond | H | C(Me)₂CN | H | Cl | CH₂OMe | O |
| D-12945 | H | single bond | Me | C(Me)₂CN | H | Me | CH₂OMe | O |
| D-12946 | Me | single bond | H | C(Me)₂CN | H | Me | CH₂OMe | O |
| D-12947 | Me(R) | single bond | H | C(Me)₂CN | H | Me | CH₂OMe | O |
| D-12948 | Me(S) | single bond | H | C(Me)₂CN | H | Me | CH₂OMe | O |
| D-12949 | H | single bond | Me | C(Me)(*i*-Pr)CN | H | Cl | CH₂OMe | O |
| D-12950 | Me | single bond | H | C(Me)(*i*-Pr)CN | H | Cl | CH₂OMe | O |
| D-12951 | Me(R) | single bond | H | C(Me)(*i*-Pr)CN | H | Cl | CH₂OMe | O |
| D-12952 | Me(S) | single bond | H | C(Me)(*i*-Pr)CN | H | Cl | CH₂OMe | O |
| D-12953 | H | single bond | Me | C(Me)(*i*-Pr)CN | H | Me | CH₂OMe | O |
| D-12954 | Me | single bond | H | C(Me)(*i*-Pr)CN | H | Me | CH₂OMe | O |

**[Table 941]**

| Compound Number | Y | W | Z | R¹ | R² | R³ | R¹⁰ | A |
|---|---|---|---|---|---|---|---|---|
| D-12955 | Me(R) | single bond | H | C(Me)(*i*-Pr)CN | H | Me | CH₂OMe | O |
| D-12956 | Me(S) | single bond | H | C(Me)(*i*-Pr)CN | H | Me | CH₂OMe | O |
| D-12957 | H | single bond | Me | OMe | H | Cl | CH₂OMe | O |
| D-12958 | Me | single bond | H | OMe | H | Cl | CH₂OMe | O |
| D-12959 | Me(R) | single bond | H | OMe | H | Cl | CH₂OMe | O |
| D-12960 | Me(S) | single bond | H | OMe | H | Cl | CH₂OMe | O |
| D-12961 | H | single bond | Me | OMe | H | Me | CH₂OMe | O |
| D-12962 | Me | single bond | H | OMe | H | Me | CH₂OMe | O |
| D-12963 | Me(R) | single bond | H | OMe | H | Me | CH₂OMe | O |
| D-12964 | Me(S) | single bond | H | OMe | H | Me | CH₂OMe | O |
| D-12965 | H | single bond | Me | CH=NOMe | H | Cl | CH₂OMe | O |
| D-12966 | Me | single bond | H | CH=NOMe | H | Cl | CH₂OMe | O |
| D-12967 | Me(R) | single bond | H | CH=NOMe | H | Cl | CH₂OMe | O |
| D-12968 | Me(S) | single bond | H | CH=NOMe | H | Cl | CH₂OMe | O |
| D-12969 | H | single bond | Me | CH=NOMe | H | Me | CH₂OMe | O |
| D-12970 | Me | single bond | H | CH=NOMe | H | Me | CH₂OMe | O |
| D-12971 | Me(R) | single bond | H | CH=NOMe | H | Me | CH₂OMe | O |
| D-12972 | Me(S) | single bond | H | CH=NOMe | H | Me | CH₂OMe | O |
| D-12973 | H | single bond | Me | morpholin-4-yl | H | Cl | CH₂OMe | O |
| D-12974 | Me | single bond | H | morpholin-4-yl | H | Cl | CH₂OMe | O |
| D-12975 | Me(R) | single bond | H | morpholin-4-yl | H | Cl | CH₂OMe | O |
| D-12976 | Me(S) | single bond | H | morpholin-4-yl | H | Cl | CH₂OMe | O |
| D-12977 | H | single bond | Me | morpholin-4-yl | H | Me | CH₂OMe | O |
| D-12978 | Me | single bond | H | morpholin-4-yl | H | Me | CH₂OMe | O |
| D-12979 | Me(R) | single bond | H | morpholin-4-yl | H | Me | CH₂OMe | O |
| D-12980 | Me(S) | single bond | H | morpholin-4-yl | H | Me | CH₂OMe | O |
| D-12981 | H | single bond | Me | Ph | H | Cl | CH₂OMe | O |
| D-12982 | Me | single bond | H | Ph | H | Cl | CH₂OMe | O |
| D-12983 | Me(R) | single bond | H | Ph | H | Cl | CH₂OMe | O |
| D-12984 | Me(S) | single bond | H | Ph | H | Cl | CH₂OMe | O |
| D-12985 | H | single bond | Me | Ph | H | Me | CH₂OMe | O |
| D-12986 | Me | single bond | H | Ph | H | Me | CH₂OMe | O |
| D-12987 | Me(R) | single bond | H | Ph | H | Me | CH₂OMe | O |
| 0-12988 | Me(S) | single bond | H | Ph | H | Me | CH₂OMe | O |
| D-12989 | H | single bond | Me | CH₂Ph | H | Cl | CH₂OMe | O |
| D-12990 | Me | single bond | H | CH₂Ph | H | Cl | CH₂OMe | O |
| D-12991 | Me(R) | single bond | H | CH₂Ph | H | Cl | CH₂OMe | O |
| D-12992 | Me(S) | single bond | H | CH₂Ph | H | Cl | CH₂OMe | O |
| D-12993 | H | single bond | Me | CH₂Ph | H | Me | CH₂OMe | O |
| D-12994 | Me | single bond | H | CH₂Ph | H | Me | CH₂OMe | O |
| D-12995 | Me(R) | single bond | H | CH₂Ph | H | Me | CH₂OMe | O |
| D-12996 | Me(S) | single bond | H | CH₂Ph | H | Me | CH₂OMe | O |
| D-12997 | H | single bond | Me | thiazole-2-yl | H | Cl | CH₂OMe | O |
| D-12998 | Me | single bond | H | thiazole-2-yl | H | Cl | CH₂OMe | O |

**[Table 942]**

| Compound Number | Y | W | Z | R¹ | R² | R³ | R¹⁰ | A |
|---|---|---|---|---|---|---|---|---|
| D-12999 | Me(R) | single bond | H | thiazole-2-yl | H | Cl | CH₂OMe | O |
| D-13000 | Me(S) | single bond | H | thiazole-2-yl | H | Cl | CH₂OMe | O |
| D-13001 | H | single bond | Me | thiazole-2-yl | H | Me | CH₂OMe | O |
| D-13002 | Me | single bond | H | thiazole-2-yl | H | Me | CH₂OMe | O |
| D-13003 | Me(R) | single bond | H | thiazole-2-yl | H | Me | CH₂OMe | O |
| D-13004 | Me(S) | single bond | H | thiazole-2-yl | H | Me | CH₂OMe | O |
| D-13005 | H | single bond | Me | 1-Me-pyrazol-3-yl | H | Cl | CH₂OMe | O |
| D-13006 | Me | single bond | H | 1-Me-pyrazol-3-yl | H | Cl | CH₂OMe | O |
| D-13007 | Me(R) | single bond | H | 1-Me-pyrazol-3-yl | H | Cl | CH₂OMe | O |
| D-13008 | Me(S) | single bond | H | 1-Me-pyrazol-3-yl | H | Cl | CH₂OMe | O |
| D-13009 | H | single bond | Me | 1-Me-pyrazol-3-yl | H | Me | CH₂OMe | O |
| D-13010 | Me | single bond | H | 1-Me-pyrazol-3-yl | H | Me | CH₂OMe | O |
| D-13011 | Me(R) | single bond | H | 1-Me-pyrazol-3-yl | H | Me | CH₂OMe | O |
| D-13012 | Me(S) | single bond | H | 1-Me-pyrazol-3-yl | H | Me | CH₂OMe | O |
| D-13013 | H | single bond | Me | 1-Me-pyrazol-5-yl | H | Cl | CH₂OMe | O |
| D-13014 | Me | single bond | H | 1-Me-pyrazol-5-yl | H | Cl | CH₂OMe | O |
| D-13015 | Me(R) | single bond | H | 1-Me-pyrazol-5-yl | H | Cl | CH₂OMe | O |
| D-13016 | Me(S) | single bond | H | 1-Me-pyrazol-5-yl | H | Cl | CH₂OMe | O |
| D-13017 | H | single bond | Me | 1-Me-pyrazol-5-yl | H | Me | CH₂OMe | O |
| D-13018 | Me | single bond | H | 1-Me-pyrazol-5-yl | H | Me | CH₂OMe | O |
| D-13019 | Me(R) | single bond | H | 1-Me-pyrazol-5-yl | H | Me | CH₂OMe | O |
| D-13020 | Me(S) | single bond | H | 1-Me-pyrazol-5-yl | H | Me | CH₂OMe | O |
| D-13021 | H | single bond | Me | CH₂(pyrimidin-2-yl) | H | Cl | CH₂OMe | O |
| D-13022 | Me | single bond | H | CH₂(pyrimidin-2-yl) | H | Cl | CH₂OMe | O |
| D-13023 | Me(R) | single bond | H | CH₂(pyrimidin-2-yl) | H | Cl | CH₂OMe | O |
| D-13024 | Me(S) | single bond | H | CH₂(pyrimidin-2-yl) | H | Cl | CH₂OMe | O |
| D-13025 | H | single bond | Me | CH₂(pyrimidin-2-yl) | H | Me | CH₂OMe | O |
| D-13026 | Me | single bond | H | CH₂(pyrimidin-2-yl) | H | Me | CH₂OMe | O |
| D-13027 | Me(R) | single bond | H | CH₂(pyrimidin-2-yl) | H | Me | CH₂OMe | O |
| D-13028 | Me(S) | single bond | H | CH₂(pyrimidin-2-yl) | H | Me | CH₂OMe | O |
| D-13029 | H | single bond | Me | -CH₂CH₂CH₂- | | Cl | CH₂OMe | O |
| D-13030 | Me | single bond | H | -CH₂CH₂CH₂- | | Cl | CH₂OMe | O |
| D-13031 | Me(R) | single bond | H | -CH₂CH₂CH₂- | | Cl | CH₂OMe | O |
| D-13032 | Me(S) | single bond | H | -CH₂CH₂CH₂- | | Cl | CH₂OMe | O |
| D-13033 | H | single bond | Me | -CH₂CH₂CH₂- | | Me | CH₂OMe | O |
| D-13034 | Me | single bond | H | -CH₂CH₂CH₂- | | Me | CH₂OMe | O |
| D-13035 | Me(R) | single bond | H | -CH₂CH₂CH₂- | | Me | CH₂OMe | O |
| D-13036 | Me(S) | single bond | H | -CH₂CH₂CH₂- | | Me | CH₂OMe | O |
| D-13037 | H | single bond | Me | -CH₂CH₂OCH₂CH₂- | | Cl | CH₂OMe | O |
| D-13038 | Me | single bond | H | -CH₂CH₂OCH₂CH₂- | | Cl | CH₂OMe | O |
| D-13039 | Me(R) | single bond | H | -CH₂CH₂OCH₂CH₂- | | Cl | CH₂OMe | O |
| D-13040 | Me(S) | single bond | H | -CH₂CH₂OCH₂CH₂- | | Cl | CH₂OMe | O |
| D-13041 | H | single bond | Me | -CH₂CH₂OCH₂CH₂- | | Me | CH₂OMe | O |
| D-13042 | Me | single bond | H | -CH₂CH₂OCH₂CH₂- | | Me | CH₂OMe | O |

**[Table 943]**

| Compound Number | Y | W | Z | R¹ | R² | R³ | R¹⁰ | A |
|---|---|---|---|---|---|---|---|---|
| D-13043 | Me(R) | single bond | H | -CH₂CH₂OCH₂CH₂- | | Me | CH₂OMe | O |
| D-13044 | Me(S) | single bond | H | -CH₂CH₂OCH₂CH₂- | | Me | CH₂OMe | O |
| D-13045 | H | single bond | Me | Me | H | Cl | C(=O)Me | O |
| D-13046 | Me | single bond | H | Me | H | Cl | C(=O)Me | O |
| D-13047 | Me(R) | single bond | H | Me | H | Cl | C(=O)Me | O |
| D-13048 | Me(S) | single bond | H | Me | H | Cl | C(=O)Me | O |
| D-13049 | H | single bond | Me | Me | H | Me | C(=O)Me | O |
| D-13050 | Me | single bond | H | Me | H | Me | C(=O)Me | O |
| D-13051 | Me(R) | single bond | H | Me | H | Me | C(=O)Me | O |
| D-13052 | Me(S) | single bond | H | Me | H | Me | C(=O)Me | O |
| D-13053 | H | single bond | Me | CH₂CHF₂ | H | Cl | C(=O)Me | O |
| D-13054 | Me | single bond | H | CH₂CHF₂ | H | Cl | C(=O)Me | O |
| D-13055 | Me(R) | single bond | H | CH₂CHF₂ | H | Cl | C(=O)Me | O |
| D-13056 | Me(S) | single bond | H | CH₂CHF₂ | H | Cl | C(=O)Me | O |
| D-13057 | H | single bond | Me | CH₂CHF₂ | H | Me | C(=O)Me | O |
| D-13058 | Me | single bond | H | CH₂CHF₂ | H | Me | C(=O)Me | O |
| D-13059 | Me(R) | single bond | H | CH₂CHF₂ | H | Me | C(=O)Me | O |
| D-13060 | Me(S) | single bond | H | CH₂CHF₂ | H | Me | C(=O)Me | O |
| D-13061 | H | single bond | Me | *n*-Pr | H | Cl | C(=O)Me | O |
| D-13062 | Me | single bond | H | *n*-Pr | H | Cl | C(=O)Me | O |
| D-13063 | Me(R) | single bond | H | *n*-Pr | H | Cl | C(=O)Me | O |
| D-13064 | Me(S) | single bond | H | *n*-Pr | H | Cl | C(=O)Me | O |
| D-13065 | H | single bond | Me | *n*-Pr | H | Me | C(=O)Me | O |
| D-13066 | Me | single bond | H | *n*-Pr | H | Me | C(=O)Me | O |
| D-13067 | Me(R) | single bond | H | *n*-Pr | H | Me | C(=O)Me | O |
| D-13068 | Me(S) | single bond | H | *n*-Pr | H | Me | C(=O)Me | O |
| D-13069 | H | single bond | Me | *i*-Pr | H | Cl | C(=O)Me | O |
| D-13070 | Me | single bond | H | *i*-Pr | H | Cl | C(=O)Me | O |
| D-13071 | Me(R) | single bond | H | *i*-Pr | H | Cl | C(=O)Me | O |
| D-13072 | Me(S) | single bond | H | *i*-Pr | H | Cl | C(=O)Me | O |
| D-13073 | H | single bond | Me | *i*-Pr | H | Me | C(=O)Me | O |
| D-13074 | Me | single bond | H | *i*-Pr | H | Me | C(=O)Me | O |
| D-13075 | Me(R) | single bond | H | *i*-Pr | H | Me | C(=O)Me | O |
| D-13076 | Me(S) | single bond | H | *i*-Pr | H | Me | C(=O)Me | O |
| D-13077 | H | single bond | Me | *c*-Pr(1-F) | H | Cl | C(=O)Me | O |
| D-13078 | Me | single bond | H | *c*-Pr(1-F) | H | Cl | C(=O)Me | O |
| D-13079 | Me(R) | single bond | H | *c*-Pr(1-F) | H | Cl | C(=O)Me | O |
| D-13080 | Me(S) | single bond | H | *c*-Pr(1-F) | H | Cl | C(=O)Me | O |
| D-13081 | H | single bond | Me | *c*-Pr(1-F) | H | Me | C(=O)Me | O |
| D-13082 | Me | single bond | H | *c*-Pr(1-F) | H | Me | C(=O)Me | O |
| D-13083 | Me(R) | single bond | H | *c*-Pr(1-F) | H | Me | C(=O)Me | O |
| D-13084 | Me(S) | single bond | H | *c*-Pr(1-F) | H | Me | C(=O)Me | O |
| D-13085 | H | single bond | Me | *c*-Pr(2,2-F₂) | H | Cl | C(=O)Me | O |
| D-13086 | Me | single bond | H | *c*-Pr(2,2-F₂) | H | Cl | C(=O)Me | O |

**[Table 944]**

| Compound Number | Y | W | Z | R¹ | R² | R³ | R¹⁰ | A |
|---|---|---|---|---|---|---|---|---|
| D-13087 | Me(R) | single bond | H | *c*-Pr(2,2-F₂) | H | Cl | C(=O)Me | O |
| D-13088 | Me(S) | single bond | H | *c*-Pr(2,2-F₂) | H | Cl | C(=O)Me | O |
| D-13089 | H | single bond | Me | *c*-Pr(2,2-F₂) | H | Me | C(=O)Me | O |
| D-13090 | Me | single bond | H | *c*-Pr(2,2-F₂) | H | Me | C(=O)Me | O |
| D-13091 | Me(R) | single bond | H | *c*-Pr(2,2-F₂) | H | Me | C(=O)Me | O |
| D-13092 | Me(S) | single bond | H | *c*-Pr(2,2-F₂) | H | Me | C(=O)Me | O |
| D-13093 | H | single bond | Me | *c*-Pr(1-Cl) | H | Cl | C(=O)Me | O |
| D-13094 | Me | single bond | H | *c*-Pr(1-Cl) | H | Cl | C(=O)Me | O |
| D-13095 | Me(R) | single bond | H | *c*-Pr(1-Cl) | H | Cl | C(=O)Me | O |
| 0-13096 | Me(S) | single bond | H | *c*-Pr(1-Cl) | H | Cl | C(=O)Me | O |
| D-13097 | H | single bond | Me | *c*-Pr(1-Cl) | H | Me | C(=O)Me | O |
| D-13098 | Me | single bond | H | *c*-Pr(1-Cl) | H | Me | C(=O)Me | O |
| D-13099 | Me(R) | single bond | H | *c*-Pr(1-Cl) | H | Me | C(=O)Me | O |
| D-13100 | Me(S) | single bond | H | *c*-Pr(1-Cl) | H | Me | C(=O)Me | O |
| D-13101 | H | single bond | Me | *c*-Pr(1-Br) | H | Cl | C(=O)Me | O |
| D-13102 | Me | single bond | H | *c*-Pr(1-Br) | H | Cl | C(=O)Me | O |
| D-13103 | Me(R) | single bond | H | *c*-Pr(1-Br) | H | Cl | C(=O)Me | O |
| D-13104 | Me(S) | single bond | H | *c*-Pr(1-Br) | H | Cl | C(=O)Me | O |
| D-13105 | H | single bond | Me | *c*-Pr(1-Br) | H | Me | C(=O)Me | O |
| D-13106 | Me | single bond | H | *c*-Pr(1-Br) | H | Me | C(=O)Me | O |
| D-13107 | Me(R) | single bond | H | *c*-Pr(1-Br) | H | Me | C(=O)Me | O |
| D-13108 | Me(S) | single bond | H | *c*-Pr(1-Br) | H | Me | C(=O)Me | O |
| D-13109 | H | single bond | Me | *c*-Pr(1-Me) | H | Cl | C(=O)Me | O |
| D-13110 | Me | single bond | H | *c*-Pr(1-Me) | H | Cl | C(=O)Me | O |
| D-13111 | Me(R) | single bond | H | *c*-Pr(1-Me) | H | Cl | C(=O)Me | O |
| D-13112 | Me(S) | single bond | H | *c*-Pr(1-Me) | H | Cl | C(=O)Me | O |
| D-13113 | H | single bond | Me | *c*-Pr(1-Me) | H | Me | C(=O)Me | O |
| D-13114 | Me | single bond | H | *c*-Pr(1-Me) | H | Me | C(=O)Me | O |
| D-13115 | Me(R) | single bond | H | *c*-Pr(1-Me) | H | Me | C(=O)Me | O |
| D-13116 | Me(S) | single bond | H | *c*-Pr(1-Me) | H | Me | C(=O)Me | O |
| D-13117 | H | single bond | Me | *c*-Pr(1-CH₂OH) | H | Cl | C(=O)Me | O |
| D-13118 | Me | single bond | H | *c*-Pr(1-CH₂OH) | H | Cl | C(=O)Me | O |
| D-13119 | Me(R) | single bond | H | *c*-Pr(1-CH₂OH) | H | Cl | C(=O)Me | O |
| D-13120 | Me(S) | single bond | H | *c*-Pr(1-CH₂OH) | H | Cl | C(=O)Me | O |
| D-13121 | H | single bond | Me | *c*-Pr(1-CH₂OH) | H | Me | C(=O)Me | O |
| D-13122 | Me | single bond | H | *c*-Pr(1-CH₂OH) | H | Me | C(=O)Me | O |
| D-13123 | Me(R) | single bond | H | *c*-Pr(1-CH₂OH) | H | Me | C(=O)Me | O |
| D-13124 | Me(S) | single bond | H | *c*-Pr(1-CH₂OH) | H | Me | C(=O)Me | O |
| D-13125 | H | single bond | Me | *c*-Pr[1-CH₂OS(=O)₂Me] | H | Cl | C(=O)Me | O |
| D-13126 | Me | single bond | H | *c*-Pr[1-CH₂OS(=O)₂Me] | H | Cl | C(=O)Me | O |
| D-13127 | Me(R) | single bond | H | *c*-Pr[1-CH₂OS(=O)₂Me] | H | Cl | C(=O)Me | O |
| D-13128 | Me(S) | single bond | H | *c*-Pr[1-CH₂OS(=O)₂Me] | H | Cl | C(=O)Me | O |
| D-13129 | H | single bond | Me | *c*-Pr[1-CH₂OS(=O)₂Me] | H | Me | C(=O)Me | O |
| D-13130 | Me | single bond | H | *c*-Pr[1-CH₂OS(=O)₂Me] | H | Me | C(=O)Me | O |

**[Table 945]**

| Compound Number | Y | W | Z | R¹ | R² | R³ | R¹⁰ | A |
|---|---|---|---|---|---|---|---|---|
| D-13131 | Me(R) | single bond | H | *c*-Pr[1-CH₂OS(=O)₂Me] | H | Me | C(=O)Me | O |
| D-13132 | Me(S) | single bond | H | *c*-Pr[1-CH₂OS(=O)₂Me] | H | Me | C(=O)Me | O |
| D-13133 | H | single bond | Me | *c*-Pr(1-CH₂OMe) | H | Cl | C(=O)Me | O |
| D-13134 | Me | single bond | H | *c*-Pr(1-CH₂OMe) | H | Cl | C(=O)Me | O |
| D-13135 | Me(R) | single bond | H | *c*-Pr(1-CH₂OMe) | H | Cl | C(=O)Me | O |
| D-13136 | Me(S) | single bond | H | *c*-Pr(1-CH₂OMe) | H | Cl | C(=O)Me | O |
| D-13137 | H | single bond | Me | *c*-Pr(1-CH₂OMe) | H | Me | C(=O)Me | O |
| D-13138 | Me | single bond | H | *c*-Pr(1-CH₂OMe) | H | Me | C(=O)Me | O |
| D-13139 | Me(R) | single bond | H | *c*-Pr(1-CH₂OMe) | H | Me | C(=O)Me | O |
| D-13140 | Me(S) | single bond | H | *c*-Pr(1-CH₂OMe) | H | Me | C(=O)Me | O |
| D-13141 | H | single bond | Me | *c*-Pr(1-CH₂OCN) | H | Cl | C(=O)Me | O |
| D-13142 | Me | single bond | H | *c*-Pr(1-CH₂OCN) | H | Cl | C(=O)Me | O |
| D-13143 | Me(R) | single bond | H | *c*-Pr(1-CH₂OCN) | H | Cl | C(=O)Me | O |
| D-13144 | Me(S) | single bond | H | *c*-Pr(1-CH₂OCN) | H | Cl | C(=O)Me | O |
| D-13145 | H | single bond | Me | *c*-Pr(1-CH₂OCN) | H | Me | C(=O)Me | O |
| D-13146 | Me | single bond | H | *c*-Pr(1-CH₂OCN) | H | Me | C(=O)Me | O |
| D-13147 | Me(R) | single bond | H | *c*-Pr(1-CH₂OCN) | H | Me | C(=O)Me | O |
| D-13148 | Me(S) | single bond | H | *c*-Pr(1-CH₂OCN) | H | Me | C(=O)Me | O |
| D-13149 | H | single bond | Me | *c*-Pr(1-CH₂SMe) | H | Cl | C(=O)Me | O |
| D-13150 | Me | single bond | H | *c*-Pr(1-CH₂SMe) | H | Cl | C(=O)Me | O |
| D-13151 | Me(R) | single bond | H | *c*-Pr(1-CH₂SMe) | H | Cl | C(=O)Me | O |
| D-13152 | Me(S) | single bond | H | *c*-Pr(1-CH₂SMe) | H | Cl | C(=O)Me | O |
| D-13153 | H | single bond | Me | *c*-Pr(1-CH₂SMe) | H | Me | C(=O)Me | O |
| D-13154 | Me | single bond | H | *c*-Pr(1-CH₂SMe) | H | Me | C(=O)Me | O |
| D-13155 | Me(R) | single bond | H | *c*-Pr(1-CH₂SMe) | H | Me | C(=O)Me | O |
| D-13156 | Me(S) | single bond | H | *c*-Pr(1-CH₂SMe) | H | Me | C(=O)Me | O |
| D-13157 | H | single bond | Me | *c*-Pr[1-CH₂S(=O)Me] | H | Cl | C(=O)Me | O |
| D-13158 | Me | single bond | H | *c*-Pr[1-CH₂S(=O)Me] | H | Cl | C(=O)Me | O |
| D-13159 | Me(R) | single bond | H | *c*-Pr[1-CH₂S(=O)Me] | H | Cl | C(=O)Me | O |
| D-13160 | Me(S) | single bond | H | *c*-Pr[1-CH₂S(=O)Me] | H | Cl | C(=O)Me | O |
| D-13161 | H | single bond | Me | *c*-Pr[1-CH₂S(=O)Me] | H | Me | C(=O)Me | O |
| D-13162 | Me | single bond | H | *c*-Pr[1-CH₂S(=O)Me] | H | Me | C(=O)Me | O |
| D-13163 | Me(R) | single bond | H | *c*-Pr[1-CH₂S(=O)Me] | H | Me | C(=O)Me | O |
| D-13164 | Me(S) | single bond | H | *c*-Pr[1-CH₂S(=O)Me] | H | Me | C(=O)Me | O |
| D-13165 | H | single bond | Me | *c*-Pr[1-CH₂S(=O)₂Me] | H | Cl | C(=O)Me | O |
| D-13166 | Me | single bond | H | *c*-Pr[1-CH₂S(=O)₂Me] | H | Cl | C(=O)Me | O |
| D-13167 | Me(R) | single bond | H | *c*-Pr[1-CH₂S(=O)₂Me] | H | Cl | C(=O)Me | O |
| D-13168 | Me(S) | single bond | H | *c*-Pr[1-CH₂S(=O)₂Me] | H | Cl | C(=O)Me | O |
| D-13169 | H | single bond | Me | *c*-Pr[1-CH₂S(=O)₂Me] | H | Me | C(=O)Me | O |
| D-13170 | Me | single bond | H | *c*-Pr[1-CH₂S(=O)₂Me] | H | Me | C(=O)Me | O |
| D-13171 | Me(R) | single bond | H | *c*-Pr[1-CH₂S(=O)₂Me] | H | Me | C(=O)Me | O |
| D-13172 | Me(S) | single bond | H | *c*-Pr[1-CH₂S(=O)₂Me] | H | Me | C(=O)Me | O |
| D-13173 | H | single bond | Me | *c*-Pr(1-CH₂F) | H | Cl | C(=O)Me | O |
| D-13174 | Me | single bond | H | *c*-Pr(1-CH₂F) | H | Cl | C(=O)Me | O |

**[Table 946]**

| Compound Number | Y | W | Z | R¹ | R² | R³ | R¹⁰ | A |
|---|---|---|---|---|---|---|---|---|
| D-13175 | Me(R) | single bond | H | *c*-Pr(1-CH₂F) | H | Cl | C(=O)Me | O |
| D-13176 | Me(S) | single bond | H | *c*-Pr(1-CH₂F) | H | Cl | C(=O)Me | O |
| D-13177 | H | single bond | Me | *c*-Pr(1-CH₂F) | H | Me | C(=O)Me | O |
| D-13178 | Me | single bond | H | *c*-Pr(1-CH₂F) | H | Me | C(=O)Me | O |
| D-13179 | Me(R) | single bond | H | *c*-Pr(1-CH₂F) | H | Me | C(=O)Me | O |
| D-13180 | Me(S) | single bond | H | *c*-Pr(1-CH₂F) | H | Me | C(=O)Me | O |
| D-13181 | H | single bond | Me | *c*-Pr(1-CHF₂) | H | Cl | C(=O)Me | O |
| D-13182 | Me | single bond | H | *c*-Pr(1-CHF₂) | H | Cl | C(=O)Me | O |
| D-13183 | Me(R) | single bond | H | *c*-Pr(1-CHF₂) | H | Cl | C(=O)Me | O |
| D-13184 | Me(S) | single bond | H | *c*-Pr(1-CHF₂) | H | Cl | C(=O)Me | O |
| D-13185 | H | single bond | Me | *c*-Pr(1-CHF₂) | H | Me | C(=O)Me | O |
| D-13186 | Me | single bond | H | *c*-Pr(1-CHF₂) | H | Me | C(=O)Me | O |
| D-13187 | Me(R) | single bond | H | *c*-Pr(1-CHF₂) | H | Me | C(=O)Me | O |
| D-13188 | Me(S) | single bond | H | *c*-Pr(1-CHF₂) | H | Me | C(=O)Me | O |
| D-13189 | H | single bond | Me | *c*-Pr(1-CF₃) | H | Cl | C(=O)Me | O |
| D-13190 | Me | single bond | H | *c*-Pr(1-CF₃) | H | Cl | C(=O)Me | O |
| D-13191 | Me(R) | single bond | H | *c*-Pr(1-CF₃) | H | Cl | C(=O)Me | O |
| D-13192 | Me(S) | single bond | H | *c*-Pr(1-CF₃) | H | Cl | C(=O)Me | O |
| D-13193 | H | single bond | Me | *c*-Pr(1-CF₃) | H | Me | C(=O)Me | O |
| D-13194 | Me | single bond | H | *c*-Pr(1-CF₃) | H | Me | C(=O)Me | O |
| D-13195 | Me(R) | single bond | H | *c*-Pr(1-CF₃) | H | Me | C(=O)Me | O |
| D-13196 | Me(S) | single bond | H | *c*-Pr(1-CF₃) | H | Me | C(=O)Me | O |
| D-13197 | H | single bond | Me | *c*-Pr(1-C≡CH) | H | Cl | C(=O)Me | O |
| D-13198 | Me | single bond | H | *c*-Pr(1-C≡CH) | H | Cl | C(=O)Me | O |
| D-13199 | Me(R) | single bond | H | *c*-Pr(1-C≡CH) | H | Cl | C(=O)Me | O |
| D-13200 | Me(S) | single bond | H | *c*-Pr(1-C≡CH) | H | Cl | C(=O)Me | O |
| D-13201 | H | single bond | Me | *c*-Pr(1-C≡CH) | H | Me | C(=O)Me | O |
| D-13202 | Me | single bond | H | *c*-Pr(1-C≡CH) | H | Me | C(=O)Me | O |
| D-13203 | Me(R) | single bond | H | *c*-Pr(1-C≡CH) | H | Me | C(=O)Me | O |
| D-13204 | Me(S) | single bond | H | *c*-Pr(1-C≡CH) | H | Me | C(=O)Me | O |
| D-13205 | H | single bond | Me | *c*-Pr[1-C(=O)OEt] | H | Cl | C(=O)Me | O |
| D-13206 | Me | single bond | H | *c*-Pr[1-C(=O)OEt] | H | Cl | C(=O)Me | O |
| D-13207 | Me(R) | single bond | H | *c*-Pr[1-C(=O)OEt] | H | Cl | C(=O)Me | O |
| D-13208 | Me(S) | single bond | H | *c*-Pr[1-C(=O)OEt] | H | Cl | C(=O)Me | O |
| D-13209 | H | single bond | Me | *c*-Pr[1-C(=O)OEt] | H | Me | C(=O)Me | O |
| D-13210 | Me | single bond | H | *c*-Pr[1-C(=O)OEt] | H | Me | C(=O)Me | O |
| D-13211 | Me(R) | single bond | H | *c*-Pr[1-C(=O)OEt] | H | Me | C(=O)Me | O |
| D-13212 | Me(S) | single bond | H | *c*-Pr[1-C(=O)OEt] | H | Me | C(=O)Me | O |
| D-13213 | H | single bond | Me | *c*-Pr[1-C(=O)OH] | H | Cl | C(=O)Me | O |
| D-13214 | Me | single bond | H | *c*-Pr[1-C(=O)OH] | H | Cl | C(=O)Me | O |
| D-13215 | Me(R) | single bond | H | *c*-Pr[1-C(=O)OH] | H | Cl | C(=O)Me | O |
| D-13216 | Me(S) | single bond | H | *c*-Pr[1-C(=O)OH] | H | Cl | C(=O)Me | O |
| D-13217 | H | single bond | Me | *c*-Pr[1-C(=O)OH] | H | Me | C(=O)Me | O |
| D-13218 | Me | single bond | H | *c*-Pr[1-C(=O)OH] | H | Me | C(=O)Me | O |

**[Table 947]**

| Compound Number | Y | W | Z | R¹ | R² | R³ | R¹⁰ | A |
|---|---|---|---|---|---|---|---|---|
| D-13219 | Me(R) | single bond | H | *c*-Pr[1-C(=O)OH] | H | Me | C(=O)Me | O |
| D-13220 | Me(S) | single bond | H | *c*-Pr[1-C(=O)OH] | H | Me | C(=O)Me | O |
| D-13221 | H | single bond | Me | *c*-Pr[1-C(=O)NH₂] | H | Cl | C(=O)Me | O |
| D-13222 | Me | single bond | H | *c*-Pr[1-C(=O)NH₂] | H | Cl | C(=O)Me | O |
| D-13223 | Me(R) | single bond | H | *c*-Pr[1-C(=O)NH₂] | H | Cl | C(=O)Me | O |
| D-13224 | Me(S) | single bond | H | *c*-Pr[1-C(=O)NH₂] | H | Cl | C(=O)Me | O |
| D-13225 | H | single bond | Me | *c*-Pr[1-C(=O)NH₂] | H | Me | C(=O)Me | O |
| D-13226 | Me | single bond | H | *c*-Pr[1-C(=O)NH₂] | H | Me | C(=O)Me | O |
| D-13227 | Me(R) | single bond | H | *c*-Pr[1-C(=O)NH₂] | H | Me | C(=O)Me | O |
| D-13228 | Me(S) | single bond | H | *c*-Pr[1-C(=O)NH₂] | H | Me | C(=O)Me | O |
| D-13229 | H | single bond | Me | *c*-Pr(1-OMe) | H | Cl | C(=O)Me | O |
| D-13230 | Me | single bond | H | *c*-Pr(1-OMe) | H | Cl | C(=O)Me | O |
| D-13231 | Me(R) | single bond | H | *c*-Pr(1-OMe) | H | Cl | C(=O)Me | O |
| D-13232 | Me(S) | single bond | H | *c*-Pr(1-OMe) | H | Cl | C(=O)Me | O |
| D-13233 | H | single bond | Me | *c*-Pr(1-OMe) | H | Me | C(=O)Me | O |
| D-13234 | Me | single bond | H | *c*-Pr(1-OMe) | H | Me | C(=O)Me | O |
| D-13235 | Me(R) | single bond | H | *c*-Pr(1-OMe) | H | Me | C(=O)Me | O |
| D-13236 | Me(S) | single bond | H | *c*-Pr(1-OMe) | H | Me | C(=O)Me | O |
| D-13237 | H | single bond | Me | *c*-Pr(1-SMe) | H | Cl | C(=O)Me | O |
| D-13238 | Me | single bond | H | *c*-Pr(1-SMe) | H | Cl | C(=O)Me | O |
| D-13239 | Me(R) | single bond | H | *c*-Pr(1-SMe) | H | Cl | C(=O)Me | O |
| D-13240 | Me(S) | single bond | H | *c*-Pr(1-SMe) | H | Cl | C(=O)Me | O |
| D-13241 | H | single bond | Me | *c*-Pr(1-SMe) | H | Me | C(=O)Me | O |
| D-13242 | Me | single bond | H | *c*-Pr(1-SMe) | H | Me | C(=O)Me | O |
| D-13243 | Me(R) | single bond | H | *c*-Pr(1-SMe) | H | Me | C(=O)Me | O |
| D-13244 | Me(S) | single bond | H | *c*-Pr(1-SMe) | H | Me | C(=O)Me | O |
| D-13245 | H | single bond | Me | *c*-Pr[1-S(=O)Me] | H | Cl | C(=O)Me | O |
| D-13246 | Me | single bond | H | *c*-Pr[1-S(=O)Me] | H | Cl | C(=O)Me | O |
| D-13247 | Me(R) | single bond | H | *c*-Pr[1-S(=O)Me] | H | Cl | C(=O)Me | O |
| D-13248 | Me(S) | single bond | H | *c*-Pr[1-S(=O)Me] | H | Cl | C(=O)Me | O |
| D-13249 | H | single bond | Me | *c*-Pr[1-S(=O)Me] | H | Me | C(=O)Me | O |
| D-13250 | Me | single bond | H | *c*-Pr[1-S(=O)Me] | H | Me | C(=O)Me | O |
| D-13251 | Me(R) | single bond | H | *c*-Pr[1-S(=O)Me] | H | Me | C(=O)Me | O |
| D-13252 | Me(S) | single bond | H | *c*-Pr[1-S(=O)Me] | H | Me | C(=O)Me | O |
| D-13253 | H | single bond | Me | *c*-Pr[1-S(=O)₂Me] | H | Cl | C(=O)Me | O |
| D-13254 | Me | single bond | H | *c*-Pr[1-S(=O)₂Me] | H | Cl | C(=O)Me | O |
| D-13255 | Me(R) | single bond | H | *c*-Pr[1-S(=O)₂Me] | H | Cl | C(=O)Me | O |
| D-13256 | Me(S) | single bond | H | *c*-Pr[1-S(=O)₂Me] | H | Cl | C(=O)Me | O |
| D-13257 | H | single bond | Me | *c*-Pr[1-S(=O)₂Me] | H | Me | C(=O)Me | O |
| D-13258 | Me | single bond | H | *c*-Pr[1-S(=O)₂Me] | H | Me | C(=O)Me | O |
| D-13259 | Me(R) | single bond | H | *c*-Pr[1-S(=O)₂Me] | H | Me | C(=O)Me | O |
| D-13260 | Me(S) | single bond | H | *c*-Pr[1-S(=O)₂Me] | H | Me | C(=O)Me | O |
| D-13261 | H | single bond | Me | *c*-Pr[1-S(=O)₂CHF₂] | H | Cl | C(=O)Me | O |
| D-13262 | Me | single bond | H | *c*-Pr[1-S(=O)₂CHF₂] | H | Cl | C(=O)Me | O |

**[Table 948]**

| Compound Number | Y | W | Z | R¹ | R² | R³ | R¹⁰ | A |
|---|---|---|---|---|---|---|---|---|
| D-13263 | Me(R) | single bond | H | *c*-Pr[1-S(=O)₂CHF₂] | H | Cl | C(=O)Me | O |
| D-13264 | Me(S) | single bond | H | *c*-Pr[1-S(=O)₂CHF₂] | H | Cl | C(=O)Me | O |
| D-13265 | H | single bond | Me | *c*-Pr[1-S(=O)₂CHF₂] | H | Me | C(=O)Me | O |
| D-13266 | Me | single bond | H | *c*-Pr[1-S(=O)₂CHF₂] | H | Me | C(=O)Me | O |
| D-13267 | Me(R) | single bond | H | *c*-Pr[1-S(=O)₂CHF₂] | H | Me | C(=O)Me | O |
| D-13268 | Me(S) | single bond | H | *c*-Pr[1-S(=O)₂CHF₂] | H | Me | C(=O)Me | O |
| D-13269 | H | single bond | Me | *c*-Pr[1-S(=O)₂CF₃] | H | Cl | C(=O)Me | O |
| D-13270 | Me | single bond | H | *c*-Pr[1-S(=O)₂CF₃] | H | Cl | C(=O)Me | O |
| D-13271 | Me(R) | single bond | H | *c*-Pr[1-S(=O)₂CF₃] | H | Cl | C(=O)Me | O |
| D-13272 | Me(S) | single bond | H | *c*-Pr[1-S(=O)₂CF₃] | H | Cl | C(=O)Me | O |
| D-13273 | H | single bond | Me | *c*-Pr[1-S(=O)₂CF₃] | H | Me | C(=O)Me | O |
| D-13274 | Me | single bond | H | *c*-Pr[1-S(=O)₂CF₃] | H | Me | C(=O)Me | O |
| D-13275 | Me(R) | single bond | H | *c*-Pr[1-S(=O)₂CF₃] | H | Me | C(=O)Me | O |
| D-13276 | Me(S) | single bond | H | *c*-Pr[1-S(=O)₂CF₃] | H | Me | C(=O)Me | O |
| D-13277 | H | single bond | Me | *c*-Pr[1-(5,6-dihydro-1,4,2-dioxazin-3-yl)] | H | Cl | C(=O)Me | O |
| D-13278 | Me | single bond | H | *c*-Pr[1-(5,6-dihydro-1,4,2-dioxazin-3-yl)] | H | Cl | C(=O)Me | O |
| D-13279 | Me(R) | single bond | H | *c*-Pr[1-(5,6-dihydro-1,4,2-dioxazin-3-yl)] | H | Cl | C(=O)Me | O |
| D-13280 | Me(S) | single bond | H | *c*-Pr[1-(5,6-dihydro-1,4,2-dioxazin-3-yl)] | H | Cl | C(=O)Me | O |
| D-13281 | H | single bond | Me | *c*-Pr[1-(5,6-dihydro-1,4,2-dioxazin-3-yl)] | H | Me | C(=O)Me | O |
| D-13282 | Me | single bond | H | *c*-Pr[1-(5,6-dihydro-1,4,2-dioxazin-3-yl)] | H | Me | C(=O)Me | O |
| D-13283 | Me(R) | single bond | H | *c*-Pr[1-(5,6-dihydro-1,4,2-dioxazin-3-yl)] | H | Me | C(=O)Me | O |
| D-13284 | Me(S) | single bond | H | *c*-Pr[1-(5,6-dihydro-1,4,2-dioxazin-3-yl)] | H | Me | C(=O)Me | O |
| D-13285 | H | single bond | Me | *c*-Pr[1-(5-hydroxy-1,3,4-oxadiazol-2-yl)] | H | Cl | C(=O)Me | O |
| D-13286 | Me | single bond | H | *c*-Pr[1-(5-hydroxy-1,3,4-oxadiazol-2-yl)] | H | Cl | C(=O)Me | O |
| D-13287 | Me(R) | single bond | H | *c*-Pr[1-(5-hydroxy-1,3,4-oxadiazol-2-yl)] | H | Cl | C(=O)Me | O |
| D-13288 | Me(S) | single bond | H | *c*-Pr[1-(5-hydroxy-1,3,4-oxadiazol-2-yl)] | H | Cl | C(=O)Me | O |
| D-13289 | H | single bond | Me | *c*-Pr[1-(5-hydroxy-1,3,4-oxadiazol-2-yl)] | H | Me | C(=O)Me | O |
| D-13290 | Me | single bond | H | *c*-Pr[1-(5-hydroxy-1,3,4-oxadiazol-2-yl)] | H | Me | C(=O)Me | O |
| D-13291 | Me(R) | single bond | H | *c*-Pr[1-(5-hydroxy-1,3,4-oxadiazol-2-yl)] | H | Me | C(=O)Me | O |
| D-13292 | Me(S) | single bond | H | *c*-Pr[1-(5-hydroxy-1,3,4-oxadiazol-2-yl)] | H | Me | C(=O)Me | O |
| D-13293 | H | single bond | Me | *c*-Pr[1-(pyrimidin-2-yl)] | H | Cl | C(=O)Me | O |
| D-13294 | Me | single bond | H | *c*-Pr[1-(pyrimidin-2-yl)] | H | Cl | C(=O)Me | O |
| D-13295 | Me(R) | single bond | H | *c*-Pr[1-(pyrimidin-2-yl)] | H | Cl | C(=O)Me | O |
| D-13296 | Me(S) | single bond | H | *c*-Pr[1-(pyrimidin-2-yl)] | H | Cl | C(=O)Me | O |
| D-13297 | H | single bond | Me | *c*-Pr[1-(pyrimidin-2-yl)] | H | Me | C(=O)Me | O |
| D-13298 | Me | single bond | H | *c*-Pr[1-(pyrimidin-2-yl)] | H | Me | C(=O)Me | O |
| D-13299 | Me(R) | single bond | H | *c*-Pr[1-(pyrimidin-2-yl)] | H | Me | C(=O)Me | O |
| D-13300 | Me(S) | single bond | H | *c*-Pr[1-(pyrimidin-2-yl)] | H | Me | C(=O)Me | O |
| D-13301 | H | single bond | Me | *t*-Bu | H | Cl | C(=O)Me | O |
| D-13302 | Me | single bond | H | *t*-Bu | H | Cl | C(=O)Me | O |
| D-13303 | Me(R) | single bond | H | *t*-Bu | H | Cl | C(=O)Me | O |
| D-13304 | Me(S) | single bond | H | *t*-Bu | H | Cl | C(=O)Me | O |
| D-13305 | H | single bond | Me | *t*-Bu | H | Me | C(=O)Me | O |
| D-13306 | Me | single bond | H | *t*-Bu | H | Me | C(=O)Me | O |

**[Table 949]**

| Compound Number | Y | W | Z | R¹ | R² | R³ | R¹⁰ | A |
|---|---|---|---|---|---|---|---|---|
| D-13307 | Me(R) | single bond | H | *t*-Bu | H | Me | C(=O)Me | O |
| D-13308 | Me(S) | single bond | H | *t*-Bu | H | Me | C(=O)Me | O |
| D-13309 | H | single bond | Me | *c*-Bu | H | Cl | C(=O)Me | O |
| D-13310 | Me | single bond | H | *c*-Bu | H | Cl | C(=O)Me | O |
| D-13311 | Me(R) | single bond | H | *c*-Bu | H | Cl | C(=O)Me | O |
| D-13312 | Me(S) | single bond | H | *c*-Bu | H | Cl | C(=O)Me | O |
| D-13313 | H | single bond | Me | *c*-Bu | H | Me | C(=O)Me | O |
| D-13314 | Me | single bond | H | *c*-Bu | H | Me | C(=O)Me | O |
| D-13315 | Me(R) | single bond | H | *c*-Bu | H | Me | C(=O)Me | O |
| D-13316 | Me(S) | single bond | H | *c*-Bu | H | Me | C(=O)Me | O |
| D-13317 | H | single bond | Me | *c*-Bu(1-CN) | H | Cl | C(=O)Me | O |
| D-13318 | Me | single bond | H | *c*-Bu(1-CN) | H | Cl | C(=O)Me | O |
| D-13319 | Me(R) | single bond | H | *c*-Bu(1-CN) | H | Cl | C(=O)Me | O |
| D-13320 | Me(S) | single bond | H | *c*-Bu(1-CN) | H | Cl | C(=O)Me | O |
| D-13321 | H | single bond | Me | *c*-Bu(1-CN) | H | Me | C(=O)Me | O |
| D-13322 | Me | single bond | H | *c*-Bu(1-CN) | H | Me | C(=O)Me | O |
| D-13323 | Me(R) | single bond | H | *c*-Bu(1-CN) | H | Me | C(=O)Me | O |
| D-13324 | Me(S) | single bond | H | *c*-Bu(1-CN) | H | Me | C(=O)Me | O |
| D-13325 | H | single bond | Me | *c*-Bu(3,3-F₂) | H | Cl | C(=O)Me | O |
| D-13326 | Me | single bond | H | *c*-Bu(3,3-F₂) | H | Cl | C(=O)Me | O |
| D-13327 | Me(R) | single bond | H | *c*-Bu(3,3-F₂) | H | Cl | C(=O)Me | O |
| D-13328 | Me(S) | single bond | H | *c*-Bu(3,3-F₂) | H | Cl | C(=O)Me | O |
| D-13329 | H | single bond | Me | *c*-Bu(3,3-F₂) | H | Me | C(=O)Me | O |
| D-13330 | Me | single bond | H | *c*-Bu(3,3-F₂) | H | Me | C(=O)Me | O |
| D-13331 | Me(R) | single bond | H | *c*-Bu(3,3-F₂) | H | Me | C(=O)Me | O |
| D-13332 | Me(S) | single bond | H | *c*-Bu(3,3-F₂) | H | Me | C(=O)Me | O |
| D-13333 | H | single bond | Me | oxetan-3-yl | H | Cl | C(=O)Me | O |
| D-13334 | Me | single bond | H | oxetan-3-yl | H | Cl | C(=O)Me | O |
| D-13335 | Me(R) | single bond | H | oxetan-3-yl | H | Cl | C(=O)Me | O |
| D-13336 | Me(S) | single bond | H | oxetan-3-yl | H | Cl | C(=O)Me | O |
| D-13337 | H | single bond | Me | oxetan-3-yl | H | Me | C(=O)Me | O |
| D-13338 | Me | single bond | H | oxetan-3-yl | H | Me | C(=O)Me | O |
| D-13339 | Me(R) | single bond | H | oxetan-3-yl | H | Me | C(=O)Me | O |
| D-13340 | Me(S) | single bond | H | oxetan-3-yl | H | Me | C(=O)Me | O |
| D-13341 | H | single bond | Me | oxetan(3-CN)-3-yl | H | Cl | C(=O)Me | O |
| D-13342 | Me | single bond | H | oxetan(3-CN)-3-yl | H | Cl | C(=O)Me | O |
| D-13343 | Me(R) | single bond | H | oxetan(3-CN)-3-yl | H | Cl | C(=O)Me | O |
| D-13344 | Me(S) | single bond | H | oxetan(3-CN)-3-yl | H | Cl | C(=O)Me | O |
| D-13345 | H | single bond | Me | oxetan(3-CN)-3-yl | H | Me | C(=O)Me | O |
| D-13346 | Me | single bond | H | oxetan(3-CN)-3-yl | H | Me | C(=O)Me | O |
| D-13347 | Me(R) | single bond | H | oxetan(3-CN)-3-yl | H | Me | C(=O)Me | O |
| D-13348 | Me(S) | single bond | H | oxetan(3-CN)-3-yl | H | Me | C(=O)Me | O |
| D-13349 | H | single bond | Me | azetidin[1-C(=O)Me]-3-yl | H | Cl | C(=O)Me | O |
| D-13350 | Me | single bond | H | azetidin[1-C(=O)Me]-3-yl | H | Cl | C(=O)Me | O |

**[Table 950]**

| Compound Number | Y | W | Z | R¹ | R² | R³ | R¹⁰ | A |
|---|---|---|---|---|---|---|---|---|
| D-13351 | Me(R) | single bond | H | azetidin[1-C(=O)Me]-3-yl | H | Cl | C(=O)Me | O |
| D-13352 | Me(S) | single bond | H | azetidin[1-C(=O)Me]-3-yl | H | Cl | C(=O)Me | O |
| D-13353 | H | single bond | Me | azetidin[1-C(=O)Me]-3-yl | H | Me | C(=O)Me | O |
| D-13354 | Me | single bond | H | azetidin[1-C(=O)Me]-3-yl | H | Me | C(=O)Me | O |
| D-13355 | Me(R) | single bond | H | azetidin[1-C(=O)Me]-3-yl | H | Me | C(=O)Me | O |
| D-13356 | Me(S) | single bond | H | azetidin[1-C(=O)Me]-3-yl | H | Me | C(=O)Me | O |
| D-13357 | H | single bond | Me | azetidin[1-S(=O)₂Me]-3-yl | H | Cl | C(=O)Me | O |
| D-13358 | Me | single bond | H | azetidin[1-S(=O)₂Me]-3-yl | H | Cl | C(=O)Me | O |
| D-13359 | Me(R) | single bond | H | azetidin[1-S(=O)₂Me]-3-yl | H | Cl | C(=O)Me | O |
| D-13360 | Me(S) | single bond | H | azetidin[1-S(=O)₂Me]-3-yl | H | Cl | C(=O)Me | O |
| D-13361 | H | single bond | Me | azetidin[1-S(=O)₂Me]-3-yl | H | Me | C(=O)Me | O |
| D-13362 | Me | single bond | H | azetidin[1-S(=O)₂Me]-3-yl | H | Me | C(=O)Me | O |
| D-13363 | Me(R) | single bond | H | azetidin[1-S(=O)₂Me]-3-yl | H | Me | C(=O)Me | O |
| D-13364 | Me(S) | single bond | H | azetidin[1-S(=O)₂Me]-3-yl | H | Me | C(=O)Me | O |
| D-13365 | H | single bond | Me | azetidin[1-S(=O)₂CHF₂]-3-yl | H | Cl | C(=O)Me | O |
| D-13366 | Me | single bond | H | azetidin[1-S(=O)₂CHF₂]-3-yl | H | Cl | C(=O)Me | O |
| D-13367 | Me(R) | single bond | H | azetidin[1-S(=O)₂CHF₂]-3-yl | H | Cl | C(=O)Me | O |
| D-13368 | Me(S) | single bond | H | azetidin[1-S(=O)₂CHF₂]-3-yl | H | Cl | C(=O)Me | O |
| D-13369 | H | single bond | Me | azetidin[1-S(=O)₂CHF₂]-3-yl | H | Me | C(=O)Me | O |
| D-13370 | Me | single bond | H | azetidin[1-S(=O)₂CHF₂]-3-yl | H | Me | C(=O)Me | O |
| D-13371 | Me(R) | single bond | H | azetidin[1-S(=O)₂CHF₂]-3-yl | H | Me | C(=O)Me | O |
| D-13372 | Me(S) | single bond | H | azetidin[1-S(=O)₂CHF₂]-3-yl | H | Me | C(=O)Me | O |
| D-13373 | H | single bond | Me | *c*-Pen | H | Cl | C(=O)Me | O |
| D-13374 | Me | single bond | H | *c*-Pen | H | Cl | C(=O)Me | O |
| D-13375 | Me(R) | single bond | H | *c*-Pen | H | Cl | C(=O)Me | O |
| D-13376 | Me(S) | single bond | H | *c*-Pen | H | Cl | C(=O)Me | O |
| D-13377 | H | single bond | Me | *c*-Pen | H | Me | C(=O)Me | O |
| D-13378 | Me | single bond | H | *c*-Pen | H | Me | C(=O)Me | O |
| D-13379 | Me(R) | single bond | H | *c*-Pen | H | Me | C(=O)Me | O |
| D-13380 | Me(S) | single bond | H | *c*-Pen | H | Me | C(=O)Me | O |
| D-13381 | H | single bond | Me | tetrahydrofuran-3-yl | H | Cl | C(=O)Me | O |
| D-13382 | Me | single bond | H | tetrahydrofuran-3-yl | H | Cl | C(=O)Me | O |
| D-13383 | Me(R) | single bond | H | tetrahydrofuran-3-yl | H | Cl | C(=O)Me | O |
| D-13384 | Me(S) | single bond | H | tetrahydrofuran-3-yl | H | Cl | C(=O)Me | O |
| D-13385 | H | single bond | Me | tetrahydrofuran-3-yl | H | Me | C(=O)Me | O |
| D-13386 | Me | single bond | H | tetrahydrofuran-3-yl | H | Me | C(=O)Me | O |
| D-13387 | Me(R) | single bond | H | tetrahydrofuran-3-yl | H | Me | C(=O)Me | O |
| D-13388 | Me(S) | single bond | H | tetrahydrofuran-3-yl | H | Me | C(=O)Me | O |
| D-13389 | H | single bond | Me | tetrahydrothiophen-3-yl | H | Cl | C(=O)Me | O |
| D-13390 | Me | single bond | H | tetrahydrothiophen-3-yl | H | Cl | C(=O)Me | O |
| D-13391 | Me(R) | single bond | H | tetrahydrothiophen-3-yl | H | Cl | C(=O)Me | O |
| D-13392 | Me(S) | single bond | H | tetrahydrothiophen-3-yl | H | Cl | C(=O)Me | O |
| D-13393 | H | single bond | Me | tetrahydrothiophen-3-yl | H | Me | C(=O)Me | O |
| D-13394 | Me | single bond | H | tetrahydrothiophen-3-yl | H | Me | C(=O)Me | O |

**[Table 951]**

| Compound Number | Y | W | Z | R¹ | R² | R³ | R¹⁰ | A |
|---|---|---|---|---|---|---|---|---|
| D-13395 | Me(R) | single bond | H | tetrahydrothiophen-3-yl | H | Me | C(=O)Me | O |
| D-13396 | Me(S) | single bond | H | tetrahydrothiophen-3-yl | H | Me | C(=O)Me | O |
| D-13397 | H | single bond | Me | *c*-Pen(1-CN) | H | Cl | C(=O)Me | O |
| D-13398 | Me | single bond | H | *c*-Pen(1-CN) | H | Cl | C(=O)Me | O |
| D-13399 | Me(R) | single bond | H | *c*-Pen(1-CN) | H | Cl | C(=O)Me | O |
| D-13400 | Me(S) | single bond | H | *c*-Pen(1-CN) | H | Cl | C(=O)Me | O |
| D-13401 | H | single bond | Me | *c*-Pen(1-CN) | H | Me | C(=O)Me | O |
| D-13402 | Me | single bond | H | *c*-Pen(1-CN) | H | Me | C(=O)Me | O |
| D-13403 | Me(R) | single bond | H | *c*-Pen(1-CN) | H | Me | C(=O)Me | O |
| D-13404 | Me(S) | single bond | H | *c*-Pen(1-CN) | H | Me | C(=O)Me | O |
| D-13405 | H | single bond | Me | tetrahydrofuran(3-CN)-3-yl | H | Cl | C(=O)Me | O |
| D-13406 | Me | single bond | H | tetrahydrofuran(3-CN)-3-yl | H | Cl | C(=O)Me | O |
| D-13407 | Me(R) | single bond | H | tetrahydrofuran(3-CN)-3-yl | H | Cl | C(=O)Me | O |
| D-13408 | Me(S) | single bond | H | tetrahydrofuran(3-CN)-3-yl | H | Cl | C(=O)Me | O |
| D-13409 | H | single bond | Me | tetrahydrofuran(3-CN)-3-yl | H | Me | C(=O)Me | O |
| D-13410 | Me | single bond | H | tetrahydrofuran(3-CN)-3-yl | H | Me | C(=O)Me | O |
| D-13411 | Me(R) | single bond | H | tetrahydrofuran(3-CN)-3-yl | H | Me | C(=O)Me | O |
| D-13412 | Me(S) | single bond | H | tetrahydrofuran(3-CN)-3-yl | H | Me | C(=O)Me | O |
| D-13413 | H | single bond | Me | tetrahydrothiophen(3-CN)-3-yl | H | Cl | C(=O)Me | O |
| D-13414 | Me | single bond | H | tetrahydrothiophen(3-CN)-3-yl | H | Cl | C(=O)Me | O |
| D-13415 | Me(R) | single bond | H | tetrahydrothiophen(3-CN)-3-yl | H | Cl | C(=O)Me | O |
| D-13416 | Me(S) | single bond | H | tetrahydrothiophen(3-CN)-3-yl | H | Cl | C(=O)Me | O |
| D-13417 | H | single bond | Me | tetrahydrothiophen(3-CN)-3-yl | H | Me | C(=O)Me | O |
| D-13418 | Me | single bond | H | tetrahydrothiophen(3-CN)-3-yl | H | Me | C(=O)Me | O |
| D-13419 | Me(R) | single bond | H | tetrahydrothiophen(3-CN)-3-yl | H | Me | C(=O)Me | O |
| D-13420 | Me(S) | single bond | H | tetrahydrothiophen(3-CN)-3-yl | H | Me | C(=O)Me | O |
| D-13421 | H | single bond | Me | bicyclo[1.1.1]pentan-1-yl | H | Cl | C(=O)Me | O |
| D-13422 | Me | single bond | H | bicyclo[1.1.1]pentan-1-yl | H | Cl | C(=O)Me | O |
| D-13423 | Me(R) | single bond | H | bicyclo[1.1.1]pentan-1-yl | H | Cl | C(=O)Me | O |
| D-13424 | Me(S) | single bond | H | bicyclo[1.1.1]pertan-1-yl | H | Cl | C(=O)Me | O |
| D-13425 | H | single bond | Me | bicyclo[1.1.1]pentan-1-yl | H | Me | C(=O)Me | O |
| D-13426 | Me | single bond | H | bicyclo[1.1.1]pentan-1-yl | H | Me | C(=O)Me | O |
| D-13427 | Me(R) | single bond | H | bicyclo[1.1.1]pentan-1-yl | H | Me | C(=O)Me | O |
| D-13428 | Me(S) | single bond | H | bicyclo[1.1.1]pentan-1-yl | H | Me | C(=O)Me | O |
| D-13429 | H | single bond | Me | *c*-Hex | H | Cl | C(=O)Me | O |
| D-13430 | Me | single bond | H | *c*-Hex | H | Cl | C(=O)Me | O |
| D-13431 | Me(R) | single bond | H | *c*-Hex | H | Cl | C(=O)Me | O |
| D-13432 | Me(S) | single bond | H | *c*-Hex | H | Cl | C(=O)Me | O |
| D-13433 | H | single bond | Me | *c*-Hex | H | Me | C(=O)Me | O |
| D-13434 | Me | single bond | H | *c*-Hex | H | Me | C(=O)Me | O |
| D-13435 | Me(R) | single bond | H | *c*-Hex | H | Me | C(=O)Me | O |
| D-13436 | Me(S) | single bond | H | *c*-Hex | H | Me | C(=O)Me | O |
| D-13437 | H | single bond | Me | tetrahydropyran-4-yl | H | Cl | C(=O)Me | O |
| D-13438 | Me | single bond | H | tetrahydropyran-4-yl | H | Cl | C(=O)Me | O |

**[Table 952]**

| Compound Number | Y | W | Z | R¹ | R² | R³ | R¹⁰ | A |
|---|---|---|---|---|---|---|---|---|
| D-13439 | Me(R) | single bond | H | tetrahydropyran-4-yl | H | Cl | C(=O)Me | O |
| D-13440 | Me(S) | single bond | H | tetrahydropyran-4-yl | H | Cl | C(=O)Me | O |
| D-13441 | H | single bond | Me | tetrahydropyran-4-yl | H | Me | C(=O)Me | O |
| D-13442 | Me | single bond | H | tetrahydropyran-4-yl | H | Me | C(=O)Me | O |
| D-13443 | Me(R) | single bond | H | tetrahydropyran-4-yl | H | Me | C(=O)Me | O |
| D-13444 | Me(S) | single bond | H | tetrahydropyran-4-yl | H | Me | C(=O)Me | O |
| D-13445 | H | single bond | Me | tetrahydrothiopyran-4-yl | H | Cl | C(=O)Me | O |
| D-13446 | Me | single bond | H | tetrahydrothiopyran-4-yl | H | Cl | C(=O)Me | O |
| D-13447 | Me(R) | single bond | H | tetrahydrothiopyran-4-yl | H | Cl | C(=O)Me | O |
| D-13448 | Me(S) | single bond | H | tetrahydrothiopyran-4-yl | H | Cl | C(=O)Me | O |
| D-13449 | H | single bond | Me | tetrahydrothiopyran-4-yl | H | Me | C(=O)Me | O |
| D-13450 | Me | single bond | H | tetrahydrothiopyran-4-yl | H | Me | C(=O)Me | O |
| D-13451 | Me(R) | single bond | H | tetrahydrothiopyran-4-yl | H | Me | C(=O)Me | O |
| D-13452 | Me(S) | single bond | H | tetrahydrothiopyran-4-yl | H | Me | C(=O)Me | O |
| D-13453 | H | single bond | Me | *c*-Hex(1-CN) | H | Cl | C(=O)Me | O |
| D-13454 | Me | single bond | H | *c*-Hex(1-CN) | H | Cl | C(=O)Me | O |
| D-13455 | Me(R) | single bond | H | *c*-Hex(1-CN) | H | Cl | C(=O)Me | O |
| D-13456 | Me(S) | single bond | H | *c*-Hex(1-CN) | H | Cl | C(=O)Me | O |
| D-13457 | H | single bond | Me | *c*-Hex(1-CN) | H | Me | C(=O)Me | O |
| D-13458 | Me | single bond | H | *c*-Hex(1-CN) | H | Me | C(=O)Me | O |
| D-13459 | Me(R) | single bond | H | *c*-Hex(1-CN) | H | Me | C(=O)Me | O |
| D-13460 | Me(S) | single bond | H | *c*-Hex(1-CN) | H | Me | C(=O)Me | O |
| D-13461 | H | single bond | Me | tetrahydropyran(4-CN)-4-yl | H | Cl | C(=O)Me | O |
| D-13462 | Me | single bond | H | tetrahydropyran(4-CN)-4-yl | H | Cl | C(=O)Me | O |
| D-13463 | Me(R) | single bond | H | tetrahydropyran(4-CN)-4-yl | H | Cl | C(=O)Me | O |
| D-13464 | Me(S) | single bond | H | tetrahydropyran(4-CN)-4-yl | H | Cl | C(=O)Me | O |
| D-13465 | H | single bond | Me | tetrahydropyran(4-CN)-4-yl | H | Me | C(=O)Me | O |
| D-13466 | Me | single bond | H | tetrahydropyran(4-CN)-4-yl | H | Me | C(=O)Me | O |
| D-13467 | Me(R) | single bond | H | tetrahydropyran(4-CN)-4-yl | H | Me | C(=O)Me | O |
| D-13468 | Me(S) | single bond | H | tetrahydropyran(4-CN)-4-yl | H | Me | C(=O)Me | O |
| D-13469 | H | single bond | Me | tetrahydrothiopyran(4-CN)-4-yl | H | Cl | C(=O)Me | O |
| D-13470 | Me | single bond | H | tetrahydrothiopyran(4-CN)-4-yl | H | Cl | C(=O)Me | O |
| D-13471 | Me(R) | single bond | H | tetrahydrothiopyran(4-CN)-4-yl | H | Cl | C(=O)Me | O |
| D-13472 | Me(S) | single bond | H | tetrahydrothiopyran(4-CN)-4-yl | H | Cl | C(=O)Me | O |
| D-13473 | H | single bond | Me | tetrahydrothiopyran(4-CN)-4-yl | H | Me | C(=O)Me | O |
| D-13474 | Me | single bond | H | tetrahydrothiopyran(4-CN)-4-yl | H | Me | C(=O)Me | O |
| D-13475 | Me(R) | single bond | H | tetrahydrothiopyran(4-CN)-4-yl | H | Me | C(=O)Me | O |
| D-13476 | Me(S) | single bond | H | tetrahydrothiopyran(4-CN)-4-yl | H | Me | C(=O)Me | O |
| D-13477 | H | single bond | Me | CH₂(*c*-Pr) | H | Cl | C(=O)Me | O |
| D-13478 | Me | single bond | H | CH₂(*c*-Pr) | H | Cl | C(=O)Me | O |
| D-13479 | Me(R) | single bond | H | CH₂(*c*-Pr) | H | Cl | C(=O)Me | O |
| D-13480 | Me(S) | single bond | H | CH₂(*c*-Pr) | H | Cl | C(=O)Me | O |
| D-13481 | H | single bond | Me | CH₂(*c*-Pr) | H | Me | C(=O)Me | O |
| D-13482 | Me | single bond | H | CH₂(*c*-Pr) | H | Me | C(=O)Me | O |

**[Table 953]**

| Compound Number | Y | W | Z | R¹ | R² | R³ | R¹⁰ | A |
|---|---|---|---|---|---|---|---|---|
| D-13483 | Me(R) | single bond | H | CH₂(*c*-Pr) | H | Me | C(=O)Me | O |
| D-13484 | Me(S) | single bond | H | CH₂(*c*-Pr) | H | Me | C(=O)Me | O |
| D-13485 | H | single bond | Me | CH₂[*c*-Pr(1-CN)] | H | Cl | C(=O)Me | O |
| D-13486 | Me | single bond | H | CH₂[*c*-Pr(1-CN)] | H | Cl | C(=O)Me | O |
| D-13487 | Me(R) | single bond | H | CH₂[*c*-Pr(1-CN)] | H | Cl | C(=O)Me | O |
| D-13488 | Me(S) | single bond | H | CH₂[*c*-Pr(1-CN)] | H | Cl | C(=O)Me | O |
| D-13489 | H | single bond | Me | CH₂[*c*-Pr(1-CN)] | H | Me | C(=O)Me | O |
| D-13490 | Me | single bond | H | CH₂[*c*-Pr(1-CN)] | H | Me | C(=O)Me | O |
| D-13491 | Me(R) | single bond | H | CH₂[*c*-Pr(1-CN)] | H | Me | C(=O)Me | O |
| D-13492 | Me(S) | single bond | H | CH₂[*c*-Pr(1-CN)] | H | Me | C(=O)Me | O |
| D-13493 | H | single bond | Me | CH(Me)(*c*-Pr) | H | Cl | C(=O)Me | O |
| D-13494 | Me | single bond | H | CH(Me)(*c*-Pr) | H | Cl | C(=O)Me | O |
| D-13495 | Me(R) | single bond | H | CH(Me)(*c*-Pr) | H | Cl | C(=O)Me | O |
| D-13496 | Me(S) | single bond | H | CH(Me)(*c*-Pr) | H | Cl | C(=O)Me | O |
| D-13497 | H | single bond | Me | CH(Me)(*c*-Pr) | H | Me | C(=O)Me | O |
| D-13498 | Me | single bond | H | CH(Me)(*c*-Pr) | H | Me | C(=O)Me | O |
| D-13499 | Me(R) | single bond | H | CH(Me)(*c*-Pr) | H | Me | C(=O)Me | O |
| D-13500 | Me(S) | single bond | H | CH(Me)(*c*-Pr) | H | Me | C(=O)Me | O |
| D-13501 | H | single bond | Me | CH(Me)CH₂OMe | H | Cl | C(=O)Me | O |
| D-13502 | Me | single bond | H | CH(Me)CH₂OMe | H | Cl | C(=O)Me | O |
| D-13503 | Me(R) | single bond | H | CH(Me)CH₂OMe | H | Cl | C(=O)Me | O |
| D-13504 | Me(S) | single bond | H | CH(Me)CH₂OMe | H | Cl | C(=O)Me | O |
| D-13505 | H | single bond | Me | CH(Me)CH₂OMe | H | Me | C(=O)Me | O |
| D-13506 | Me | single bond | H | CH(Me)CH₂OMe | H | Me | C(=O)Me | O |
| D-13507 | Me(R) | single bond | H | CH(Me)CH₂OMe | H | Me | C(=O)Me | O |
| D-13508 | Me(S) | single bond | H | CH(Me)CH₂OMe | H | Me | C(=O)Me | O |
| D-13509 | H | single bond | Me | CH₂CH=CH₂ | H | Cl | C(=O)Me | O |
| D-13510 | Me | single bond | H | CH₂CH=CH₂ | H | Cl | C(=O)Me | O |
| D-13511 | Me(R) | single bond | H | CH₂CH=CH₂ | H | Cl | C(=O)Me | O |
| D-13512 | Me(S) | single bond | H | CH₂CH=CH₂ | H | Cl | C(=O)Me | O |
| D-13513 | H | single bond | Me | CH₂CH=CH₂ | H | Me | C(=O)Me | O |
| D-13514 | Me | single bond | H | CH₂CH=CH₂ | H | Me | C(=O)Me | O |
| D-13515 | Me(R) | single bond | H | CH₂CH=CH₂ | H | Me | C(=O)Me | O |
| D-13516 | Me(S) | single bond | H | CH₂CH=CH₂ | H | Me | C(=O)Me | O |
| D-13517 | H | single bond | Me | CH₂C≡CH | H | Cl | C(=O)Me | O |
| D-13518 | Me | single bond | H | CH₂C≡CH | H | Cl | C(=O)Me | O |
| D-13519 | Me(R) | single bond | H | CH₂C≡CH | H | Cl | C(=O)Me | O |
| D-13520 | Me(S) | single bond | H | CH₂C≡CH | H | Cl | C(=O)Me | O |
| D-13521 | H | single bond | Me | CH₂C≡CH | H | Me | C(=O)Me | O |
| D-13522 | Me | single bond | H | CH₂C≡CH | H | Me | C(=O)Me | O |
| D-13523 | Me(R) | single bond | H | CH₂C≡CH | H | Me | C(=O)Me | O |
| D-13524 | Me(S) | single bond | H | CH₂C≡CH | H | Me | C(=O)Me | O |
| D-13525 | H | single bond | Me | CH₂CN | H | Cl | C(=O)Me | O |
| D-13526 | Me | single bond | H | CH₂CN | H | Cl | C(=O)Me | O |

**[Table 954]**

| Compound Number | Y | W | Z | R¹ | R² | R³ | R¹⁰ | A |
|---|---|---|---|---|---|---|---|---|
| D-13527 | Me(R) | single bond | H | CH₂CN | H | Cl | C(=O)Me | O |
| D-13528 | Me(S) | single bond | H | CH₂CN | H | Cl | C(=O)Me | O |
| D-13529 | H | single bond | Me | CH₂CN | H | Me | C(=O)Me | O |
| D-13530 | Me | single bond | H | CH₂CN | H | Me | C(=O)Me | O |
| D-13531 | Me(R) | single bond | H | CH₂CN | H | Me | C(=O)Me | O |
| D-13532 | Me(S) | single bond | H | CH₂CN | H | Me | C(=O)Me | O |
| D-13533 | H | single bond | Me | CH(Me)CN | H | Cl | C(=O)Me | O |
| D-13534 | Me | single bond | H | CH(Me)CN | H | Cl | C(=O)Me | O |
| D-13535 | Me(R) | single bond | H | CH(Me)CN | H | Cl | C(=O)Me | O |
| D-13536 | Me(S) | single bond | H | CH(Me)CN | H | Cl | C(=O)Me | O |
| D-13537 | H | single bond | Me | CH(Me)CN | H | Me | C(=O)Me | O |
| D-13538 | Me | single bond | H | CH(Me)CN | H | Me | C(=O)Me | O |
| D-13539 | Me(R) | single bond | H | CH(Me)CN | H | Me | C(=O)Me | O |
| D-13540 | Me(S) | single bond | H | CH(Me)CN | H | Me | C(=O)Me | O |
| D-13541 | H | single bond | Me | C(Me)₂CN | H | Cl | C(=O)Me | O |
| D-13542 | Me | single bond | H | C(Me)₂CN | H | Cl | C(=O)Me | O |
| D-13543 | Me(R) | single bond | H | C(Me)₂CN | H | Cl | C(=O)Me | O |
| D-13544 | Me(S) | single bond | H | C(Me)₂CN | H | Cl | C(=O)Me | O |
| D-13545 | H | single bond | Me | C(Me)₂CN | H | Me | C(=O)Me | O |
| D-13546 | Me | single bond | H | C(Me)₂CN | H | Me | C(=O)Me | O |
| D-13547 | Me(R) | single bond | H | C(Me)₂CN | H | Me | C(=O)Me | O |
| D-13548 | Me(S) | single bond | H | C(Me)₂CN | H | Me | C(=O)Me | O |
| D-13549 | H | single bond | Me | C(Me)(*i*-Pr)CN | H | Cl | C(=O)Me | O |
| D-13550 | Me | single bond | H | C(Me)(*i*-Pr)CN | H | Cl | C(=O)Me | O |
| D-13551 | Me(R) | single bond | H | C(Me)(*i*-Pr)CN | H | Cl | C(=O)Me | O |
| D-13552 | Me(S) | single bond | H | C(Me)(*i*-Pr)CN | H | Cl | C(=O)Me | O |
| D-13553 | H | single bond | Me | C(Me)(*i*-Pr)CN | H | Me | C(=O)Me | O |
| D-13554 | Me | single bond | H | C(Me)(*i*-Pr)CN | H | Me | C(=O)Me | O |
| D-13555 | Me(R) | single bond | H | C(Me)(*i*-Pr)CN | H | Me | C(=O)Me | O |
| D-13556 | Me(S) | single bond | H | C(Me)(*i*-Pr)CN | H | Me | C(=O)Me | O |
| D-13557 | H | single bond | Me | OMe | H | Cl | C(=O)Me | O |
| D-13558 | Me | single bond | H | OMe | H | Cl | C(=O)Me | O |
| D-13559 | Me(R) | single bond | H | OMe | H | Cl | C(=O)Me | O |
| D-13560 | Me(S) | single bond | H | OMe | H | Cl | C(=O)Me | O |
| D-13561 | H | single bond | Me | OMe | H | Me | C(=O)Me | O |
| D-13562 | Me | single bond | H | OMe | H | Me | C(=O)Me | O |
| D-13563 | Me(R) | single bond | H | OMe | H | Me | C(=O)Me | O |
| D-13564 | Me(S) | single bond | H | OMe | H | Me | C(=O)Me | O |
| D-13565 | H | single bond | Me | CH=NOMe | H | Cl | C(=O)Me | O |
| D-13566 | Me | single bond | H | CH=NOMe | H | Cl | C(=O)Me | O |
| D-13567 | Me(R) | single bond | H | CH=NOMe | H | Cl | C(=O)Me | O |
| D-13568 | Me(S) | single bond | H | CH=NOMe | H | Cl | C(=O)Me | O |
| D-13569 | H | single bond | Me | CH=NOMe | H | Me | C(=O)Me | O |
| D-13570 | Me | single bond | H | CH=NOMe | H | Me | C(=O)Me | O |

**[Table 955]**

| Compound Number | Y | W | Z | R¹ | R² | R³ | R¹⁰ | A |
|---|---|---|---|---|---|---|---|---|
| D-13571 | Me(R) | single bond | H | CH=NOMe | H | Me | C(=O)Me | O |
| D-13572 | Me(S) | single bond | H | CH=NOMe | H | Me | C(=O)Me | O |
| D-13573 | H | single bond | Me | morpholin-4-yl | H | Cl | C(=O)Me | O |
| D-13574 | Me | single bond | H | morpholin-4-yl | H | Cl | C(=O)Me | O |
| D-13575 | Me(R) | single bond | H | morpholin-4-yl | H | Cl | C(=O)Me | O |
| D-13576 | Me(S) | single bond | H | morpholin-4-yl | H | Cl | C(=O)Me | O |
| D-13577 | H | single bond | Me | morpholin-4-yl | H | Me | C(=O)Me | O |
| D-13578 | Me | single bond | H | morpholin-4-yl | H | Me | C(=O)Me | O |
| D-13579 | Me(R) | single bond | H | morpholin-4-yl | H | Me | C(=O)Me | O |
| D-13580 | Me(S) | single bond | H | morpholin-4-yl | H | Me | C(=O)Me | O |
| D-13581 | H | single bond | Me | Ph | H | Cl | C(=O)Me | O |
| D-13582 | Me | single bond | H | Ph | H | Cl | C(=O)Me | O |
| D-13583 | Me(R) | single bond | H | Ph | H | Cl | C(=O)Me | O |
| D-13584 | Me(S) | single bond | H | Ph | H | Cl | C(=O)Me | O |
| D-13585 | H | single bond | Me | Ph | H | Me | C(=O)Me | O |
| D-13586 | Me | single bond | H | Ph | H | Me | C(=O)Me | O |
| D-13587 | Me(R) | single bond | H | Ph | H | Me | C(=O)Me | O |
| D-13588 | Me(S) | single bond | H | Ph | H | Me | C(=O)Me | O |
| D-13589 | H | single bond | Me | CH₂Ph | H | Cl | C(=O)Me | O |
| D-13590 | Me | single bond | H | CH₂Ph | H | Cl | C(=O)Me | O |
| D-13591 | Me(R) | single bond | H | CH₂Ph | H | Cl | C(=O)Me | O |
| D-13592 | Me(S) | single bond | H | CH₂Ph | H | Cl | C(=O)Me | O |
| D-13593 | H | single bond | Me | CH₂Ph | H | Me | C(=O)Me | O |
| D-13594 | Me | single bond | H | CH₂Ph | H | Me | C(=O)Me | O |
| D-13595 | Me(R) | single bond | H | CH₂Ph | H | Me | C(=O)Me | O |
| D-13596 | Me(S) | single bond | H | CH₂Ph | H | Me | C(=O)Me | O |
| D-13597 | H | single bond | Me | thiazole-2-yl | H | Cl | C(=O)Me | O |
| D-13598 | Me | single bond | H | thiazole-2-yl | H | Cl | C(=O)Me | O |
| D-13599 | Me(R) | single bond | H | thiazole-2-yl | H | Cl | C(=O)Me | O |
| D-13600 | Me(S) | single bond | H | thiazole-2-yl | H | Cl | C(=O)Me | O |
| D-13601 | H | single bond | Me | thiazole-2-yl | H | Me | C(=O)Me | O |
| D-13602 | Me | single bond | H | thiazole-2-yl | H | Me | C(=O)Me | O |
| D-13603 | Me(R) | single bond | H | thiazole-2-yl | H | Me | C(=O)Me | O |
| D-13604 | Me(S) | single bond | H | thiazole-2-yl | H | Me | C(=O)Me | O |
| D-13605 | H | single bond | Me | 1-Me-pyrazol-3-yl | H | Cl | C(=O)Me | O |
| D-13606 | Me | single bond | H | 1-Me-pyrazol-3-yl | H | Cl | C(=O)Me | O |
| D-13607 | Me(R) | single bond | H | 1-Me-pyrazol-3-yl | H | Cl | C(=O)Me | O |
| D-13608 | Me(S) | single bond | H | 1-Me-pyrazol-3-yl | H | Cl | C(=O)Me | O |
| D-13609 | H | single bond | Me | 1-Me-pyrazol-3-yl | H | Me | C(=O)Me | O |
| D-13610 | Me | single bond | H | 1-Me-pyrazol-3-yl | H | Me | C(=O)Me | O |
| D-13611 | Me(R) | single bond | H | 1-Me-pyrazol-3-yl | H | Me | C(=O)Me | O |
| D-13612 | Me(S) | single bond | H | 1-Me-pyrazol-3-yl | H | Me | C(=O)Me | O |
| D-13613 | H | single bond | Me | 1-Me-pyrazol-5-yl | H | Cl | C(=O)Me | O |
| D-13614 | Me | single bond | H | 1-Me-pyrazol-5-yl | H | Cl | C(=O)Me | O |

**[Table 956]**

| Compound Number | Y | W | Z | R¹ | R² | R³ | R¹⁰ | A |
|---|---|---|---|---|---|---|---|---|
| D-13615 | Me(R) | single bond | H | 1-Me-pyrazol-5-yl | H | Cl | C(=O)Me | O |
| D-13616 | Me(S) | single bond | H | 1-Me-pyrazol-5-yl | H | Cl | C(=O)Me | O |
| D-13617 | H | single bond | Me | 1-Me-pyrazol-5-yl | H | Me | C(=O)Me | O |
| D-13618 | Me | single bond | H | 1-Me-pyrazol-5-yl | H | Me | C(=O)Me | O |
| D-13619 | Me(R) | single bond | H | 1-Me-pyrazol-5-yl | H | Me | C(=O)Me | O |
| D-13620 | Me(S) | single bond | H | 1-Me-pyrazol-5-yl | H | Me | C(=O)Me | O |
| D-13621 | H | single bond | Me | CH₂(pyrimidin-2-yl) | H | Cl | C(=O)Me | O |
| D-13622 | Me | single bond | H | CH₂(pyrimidin-2-yl) | H | Cl | C(=O)Me | O |
| D-13623 | Me(R) | single bond | H | CH₂(pyrimidin-2-yl) | H | Cl | C(=O)Me | O |
| D-13624 | Me(S) | single bond | H | CH₂(pyrimidin-2-yl) | H | Cl | C(=O)Me | O |
| D-13625 | H | single bond | Me | CH₂(pyrimidin-2-yl) | H | Me | C(=O)Me | O |
| D-13626 | Me | single bond | H | CH₂(pyrimidin-2-yl) | H | Me | C(=O)Me | O |
| D-13627 | Me(R) | single bond | H | CH₂(pyrimidin-2-yl) | H | Me | C(=O)Me | O |
| D-13628 | Me(S) | single bond | H | CH₂(pyrimidin-2-yl) | H | Me | C(=O)Me | O |
| D-13629 | H | single bond | Me | -CH₂CH₂CH₂- | | Cl | C(=O)Me | O |
| D-13630 | Me | single bond | H | -CH₂CH₂CH₂- | | Cl | C(=O)Me | O |
| D-13631 | Me(R) | single bond | H | -CH₂CH₂CH₂- | | Cl | C(=O)Me | O |
| D-13632 | Me(S) | single bond | H | -CH₂CH₂CH₂- | | Cl | C(=O)Me | O |
| D-13633 | H | single bond | Me | -CH₂CH₂CH₂- | | Me | C(=O)Me | O |
| D-13634 | Me | single bond | H | -CH₂CH₂CH₂- | | Me | C(=O)Me | O |
| D-13635 | Me(R) | single bond | H | -CH₂CH₂CH₂- | | Me | C(=O)Me | O |
| D-13636 | Me(S) | single bond | H | -CH₂CH₂CH₂- | | Me | C(=O)Me | O |
| D-13637 | H | single bond | Me | -CH₂CH₂OCH₂CH₂- | | Cl | C(=O)Me | O |
| D-13638 | Me | single bond | H | -CH₂CH₂OCH₂CH₂- | | Cl | C(=O)Me | O |
| D-13639 | Me(R) | single bond | H | -CH₂CH₂OCH₂CH₂- | | Cl | C(=O)Me | O |
| D-13640 | Me(S) | single bond | H | -CH₂CH₂OCH₂CH₂- | | Cl | C(=O)Me | O |
| D-13641 | H | single bond | Me | -CH₂CH₂OCH₂CH₂- | | Me | C(=O)Me | O |
| D-13642 | Me | single bond | H | -CH₂CH₂OCH₂CH₂- | | Me | C(=O)Me | O |
| D-13643 | Me(R) | single bond | H | -CH₂CH₂OCH₂CH₂- | | Me | C(=O)Me | O |
| D-13644 | Me(S) | single bond | H | -CH₂CH₂OCH₂CH₂- | | Me | C(=O)Me | O |
| D-13645 | H | single bond | Me | Me | Me | Cl | H | O |
| D-13646 | Me | single bond | H | Me | Me | Cl | H | O |
| D-13647 | Me(R) | single bond | H | Me | Me | Cl | H | O |
| D-13648 | Me(S) | single bond | H | Me | Me | Cl | H | O |
| D-13649 | H | single bond | Me | Me | Me | Me | H | O |
| D-13650 | Me | single bond | H | Me | Me | Me | H | O |
| D-13651 | Me(R) | single bond | H | Me | Me | Me | H | O |
| D-13652 | Me(S) | single bond | H | Me | Me | Me | H | O |
| D-13653 | H | single bond | Me | CH₂CHF₂ | Me | Cl | H | O |
| D-13654 | Me | single bond | H | CH₂CHF₂ | Me | Cl | H | O |
| D-13655 | Me(R) | single bond | H | CH₂CHF₂ | Me | Cl | H | O |
| D-13656 | Me(S) | single bond | H | CH₂CHF₂ | Me | Cl | H | O |
| D-13657 | H | single bond | Me | CH₂CHF₂ | Me | Me | H | O |
| D-13658 | Me | single bond | H | CH₂CHF₂ | Me | Me | H | O |

**[Table 957]**

| Compound Number | Y | W | Z | R¹ | R² | R³ | R¹⁰ | A |
|---|---|---|---|---|---|---|---|---|
| D-13659 | Me(R) | single bond | H | CH₂CHF₂ | Me | Me | H | O |
| D-13660 | Me(S) | single bond | H | CH₂CHF₂ | Me | Me | H | O |
| D-13661 | H | single bond | Me | *n*-Pr | Me | Cl | H | O |
| D-13662 | Me | single bond | H | *n*-Pr | Me | Cl | H | O |
| D-13663 | Me(R) | single bond | H | *n*-Pr | Me | Cl | H | O |
| D-13664 | Me(S) | single bond | H | *n*-Pr | Me | Cl | H | O |
| D-13665 | H | single bond | Me | *n*-Pr | Me | Me | H | O |
| D-13666 | Me | single bond | H | *n*-Pr | Me | Me | H | O |
| D-13667 | Me(R) | single bond | H | *n*-Pr | Me | Me | H | O |
| D-13668 | Me(S) | single bond | H | *n*-Pr | Me | Me | H | O |
| D-13669 | H | single bond | Me | *i*-Pr | Me | Cl | H | O |
| D-13670 | Me | single bond | H | *i*-Pr | Me | Cl | H | O |
| D-13671 | Me(R) | single bond | H | *i*-Pr | Me | Cl | H | O |
| D-13672 | Me(S) | single bond | H | *i*-Pr | Me | Cl | H | O |
| D-13673 | H | single bond | Me | *i*-Pr | Me | Me | H | O |
| D-13674 | Me | single bond | H | *i*-Pr | Me | Me | H | O |
| D-13675 | Me(R) | single bond | H | *i*-Pr | Me | Me | H | O |
| D-13676 | Me(S) | single bond | H | *i*-Pr | Me | Me | H | O |
| D-13677 | H | single bond | Me | *c*-Pr(1-F) | Me | Cl | H | O |
| D-13678 | Me | single bond | H | *c*-Pr(1-F) | Me | Cl | H | O |
| D-13679 | Me(R) | single bond | H | *c*-Pr(1-F) | Me | Cl | H | O |
| D-13680 | Me(S) | single bond | H | *c*-Pr(1-F) | Me | Cl | H | O |
| D-13681 | H | single bond | Me | *c*-Pr(1-F) | Me | Me | H | O |
| D-13682 | Me | single bond | H | *c*-Pr(1-F) | Me | Me | H | O |
| D-13683 | Me(R) | single bond | H | *c*-Pr(1-F) | Me | Me | H | O |
| D-13684 | Me(S) | single bond | H | *c*-Pr(1-F) | Me | Me | H | O |
| D-13685 | H | single bond | Me | *c*-Pr(2,2-F₂) | Me | Cl | H | O |
| D-13686 | Me | single bond | H | *c*-Pr(2,2-F₂) | Me | Cl | H | O |
| D-13687 | Me(R) | single bond | H | *c*-Pr(2,2-F₂) | Me | Cl | H | O |
| D-13688 | Me(S) | single bond | H | *c*-Pr(2,2-F₂) | Me | Cl | H | O |
| D-13689 | H | single bond | Me | *c*-Pr(2,2-F₂) | Me | Me | H | O |
| D-13690 | Me | single bond | H | *c*-Pr(2,2-F₂) | Me | Me | H | O |
| D-13691 | Me(R) | single bond | H | *c*-Pr(2,2-F₂) | Me | Me | H | O |
| D-13692 | Me(S) | single bond | H | *c*-Pr(2,2-F₂) | Me | Me | H | O |
| D-13693 | H | single bond | Me | *c*-Pr(1-Cl) | Me | Cl | H | O |
| D-13694 | Me | single bond | H | *c*-Pr(1-Cl) | Me | Cl | H | O |
| D-13695 | Me(R) | single bond | H | *c*-Pr(1-Cl) | Me | Cl | H | O |
| D-13696 | Me(S) | single bond | H | *c*-Pr(1-Cl) | Me | Cl | H | O |
| D-13697 | H | single bond | Me | *c*-Pr(1-Cl) | Me | Me | H | O |
| D-13698 | Me | single bond | H | *c*-Pr(1-Cl) | Me | Me | H | O |
| D-13699 | Me(R) | single bond | H | *c*-Pr(1-Cl) | Me | Me | H | O |
| D-13700 | Me(S) | single bond | H | *c*-Pr(1-Cl) | Me | Me | H | O |
| D-13701 | H | single bond | Me | *c*-Pr(1-Br) | Me | Cl | H | O |
| D-13702 | Me | single bond | H | *c*-Pr(1-Br) | Me | Cl | H | O |

**[Table 958]**

| Compound Number | Y | W | Z | R¹ | R² | R³ | R¹⁰ | A |
|---|---|---|---|---|---|---|---|---|
| D-13703 | Me(R) | single bond | H | *c*-Pr(1-Br) | Me | Cl | H | O |
| D-13704 | Me(S) | single bond | H | *c*-Pr(1-Br) | Me | Cl | H | O |
| D-13705 | H | single bond | Me | *c*-Pr(1-Br) | Me | Me | H | O |
| D-13706 | Me | single bond | H | *c*-Pr(1-Br) | Me | Me | H | O |
| D-13707 | Me(R) | single bond | H | *c*-Pr(1-Br) | Me | Me | H | O |
| D-13708 | Me(S) | single bond | H | *c*-Pr(1-Br) | Me | Me | H | O |
| D-13709 | H | single bond | Me | *c*-Pr(1-Me) | Me | Cl | H | O |
| D-13710 | Me | single bond | H | *c*-Pr(1-Me) | Me | Cl | H | O |
| D-13711 | Me(R) | single bond | H | *c*-Pr(1-Me) | Me | Cl | H | O |
| D-13712 | Me(S) | single bond | H | *c*-Pr(1-Me) | Me | Cl | H | O |
| D-13713 | H | single bond | Me | *c*-Pr(1-Me) | Me | Me | H | O |
| D-13714 | Me | single bond | H | *c*-Pr(1-Me) | Me | Me | H | O |
| D-13715 | Me(R) | single bond | H | *c*-Pr(1-Me) | Me | Me | H | O |
| D-13716 | Me(S) | single bond | H | *c*-Pr(1-Me) | Me | Me | H | O |
| D-13717 | H | single bond | Me | *c*-Pr(1-CH₂OH) | Me | Cl | H | O |
| D-13718 | Me | single bond | H | *c*-Pr(1-CH₂OH) | Me | Cl | H | O |
| D-13719 | Me(R) | single bond | H | *c*-Pr(1-CH₂OH) | Me | Cl | H | O |
| D-13720 | Me(S) | single bond | H | *c*-Pr(1-CH₂OH) | Me | Cl | H | O |
| D-13721 | H | single bond | Me | *c*-Pr(1-CH₂OH) | Me | Me | H | O |
| D-13722 | Me | single bond | H | *c*-Pr(1-CH₂OH) | Me | Me | H | O |
| D-13723 | Me(R) | single bond | H | *c*-Pr(1-CH₂OH) | Me | Me | H | O |
| D-13724 | Me(S) | single bond | H | *c*-Pr(1-CH₂OH) | Me | Me | H | O |
| D-13725 | H | single bond | Me | *c*-Pr[1-CH₂OS(=O)₂Me] | Me | Cl | H | O |
| D-13726 | Me | single bond | H | *c*-Pr[1-CH₂OS(=O)₂Me] | Me | Cl | H | O |
| D-13727 | Me(R) | single bond | H | *c*-Pr[1-CH₂OS(=O)₂Me] | Me | Cl | H | O |
| D-13728 | Me(S) | single bond | H | *c*-Pr[1-CH₂OS(=O)₂Me] | Me | Cl | H | O |
| D-13729 | H | single bond | Me | *c*-Pr[1-CH₂OS(=O)₂Me] | Me | Me | H | O |
| D-13730 | Me | single bond | H | *c*-Pr[1-CH₂OS(=O)₂Me] | Me | Me | H | O |
| D-13731 | Me(R) | single bond | H | *c*-Pr[1-CH₂OS(=O)₂Me] | Me | Me | H | O |
| D-13732 | Me(S) | single bond | H | *c*-Pr[1-CH₂OS(=O)₂Me] | Me | Me | H | O |
| D-13733 | H | single bond | Me | *c*-Pr(1-CH₂OMe) | Me | Cl | H | O |
| D-13734 | Me | single bond | H | *c*-Pr(1-CH₂OMe) | Me | Cl | H | O |
| D-13735 | Me(R) | single bond | H | *c*-Pr(1-CH₂OMe) | Me | Cl | H | O |
| D-13736 | Me(S) | single bond | H | *c*-Pr(1-CH₂OMe) | Me | Cl | H | O |
| D-13737 | H | single bond | Me | *c*-Pr(1-CH₂OMe) | Me | Me | H | O |
| D-13738 | Me | single bond | H | *c*-Pr(1-CH₂OMe) | Me | Me | H | O |
| D-13739 | Me(R) | single bond | H | *c*-Pr(1-CH₂OMe) | Me | Me | H | O |
| D-13740 | Me(S) | single bond | H | *c*-Pr(1-CH₂OMe) | Me | Me | H | O |
| D-13741 | H | single bond | Me | *c*-Pr(1-CH₂OCN) | Me | Cl | H | O |
| D-13742 | Me | single bond | H | *c*-Pr(1-CH₂OCN) | Me | Cl | H | O |
| D-13743 | Me(R) | single bond | H | *c*-Pr(1-CH₂OCN) | Me | Cl | H | O |
| D-13744 | Me(S) | single bond | H | *c*-Pr(1-CH₂OCN) | Me | Cl | H | O |
| D-13745 | H | single bond | Me | *c*-Pr(1-CH₂OCN) | Me | Me | H | O |
| D-13746 | Me | single bond | H | *c*-Pr(1-CH₂OCN) | Me | Me | H | O |

**[Table 959]**

| Compound Number | Y | W | Z | R¹ | R² | R³ | R¹⁰ | A |
|---|---|---|---|---|---|---|---|---|
| D-13747 | Me(R) | single bond | H | *c*-Pr(1-CH₂OCN) | Me | Me | H | O |
| D-13748 | Me(S) | single bond | H | *c*-Pr(1-CH₂OCN) | Me | Me | H | O |
| D-13749 | H | single bond | Me | *c*-Pr(1-CH₂SMe) | Me | Cl | H | O |
| D-13750 | Me | single bond | H | *c*-Pr(1-CH₂SMe) | Me | Cl | H | O |
| D-13751 | Me(R) | single bond | H | *c*-Pr(1-CH₂SMe) | Me | Cl | H | O |
| D-13752 | Me(S) | single bond | H | *c*-Pr(1-CH₂SMe) | Me | Cl | H | O |
| D-13753 | H | single bond | Me | *c*-Pr(1-CH₂SMe) | Me | Me | H | O |
| D-13754 | Me | single bond | H | *c*-Pr(1-CH₂SMe) | Me | Me | H | O |
| D-13755 | Me(R) | single bond | H | *c*-Pr(1-CH₂SMe) | Me | Me | H | O |
| D-13756 | Me(S) | single bond | H | *c*-Pr(1-CH₂SMe) | Me | Me | H | O |
| D-13757 | H | single bond | Me | *c*-Pr[1-CH₂S(=O)Me] | Me | Cl | H | O |
| D-13758 | Me | single bond | H | *c*-Pr[1-CH₂S(=O)Me] | Me | Cl | H | O |
| D-13759 | Me(R) | single bond | H | *c*-Pr[1-CH₂S(=O)Me] | Me | Cl | H | O |
| D-13760 | Me(S) | single bond | H | *c*-Pr[1-CH₂S(=O)Me] | Me | Cl | H | O |
| D-13761 | H | single bond | Me | *c*-Pr[1-CH₂S(=O)Me] | Me | Me | H | O |
| D-13762 | Me | single bond | H | *c*-Pr[1-CH₂S(=O)Me] | Me | Me | H | O |
| D-13763 | Me(R) | single bond | H | *c*-Pr[1-CH₂S(=O)Me] | Me | Me | H | O |
| D-13764 | Me(S) | single bond | H | *c*-Pr[1-CH₂S(=O)Me] | Me | Me | H | O |
| D-13765 | H | single bond | Me | *c*-Pr[1-CH₂S(=O)₂Me] | Me | Cl | H | O |
| D-13766 | Me | single bond | H | *c*-Pr[1-CH₂S(=O)₂Me] | Me | Cl | H | O |
| D-13767 | Me(R) | single bond | H | *c*-Pr[1-CH₂S(=O)₂Me] | Me | Cl | H | O |
| D-13768 | Me(S) | single bond | H | *c*-Pr[1-CH₂S(=O)₂Me] | Me | Cl | H | O |
| D-13769 | H | single bond | Me | *c*-Pr[1-CH₂S(=O)₂Me] | Me | Me | H | O |
| D-13770 | Me | single bond | H | *c*-Pr[1-CH₂S(=O)₂Me] | Me | Me | H | O |
| D-13771 | Me(R) | single bond | H | *c*-Pr[1-CH₂S(=O)₂Me] | Me | Me | H | O |
| D-13772 | Me(S) | single bond | H | *c*-Pr[1-CH₂S(=O)₂Me] | Me | Me | H | O |
| D-13773 | H | single bond | Me | *c*-Pr(1-CH₂F) | Me | Cl | H | O |
| D-13774 | Me | single bond | H | *c*-Pr(1-CH₂F) | Me | Cl | H | O |
| D-13775 | Me(R) | single bond | H | *c*-Pr(1-CH₂F) | Me | Cl | H | O |
| D-13776 | Me(S) | single bond | H | *c*-Pr(1-CH₂F) | Me | Cl | H | O |
| D-13777 | H | single bond | Me | *c*-Pr(1-CH₂F) | Me | Me | H | O |
| D-13778 | Me | single bond | H | *c*-Pr(1-CH₂F) | Me | Me | H | O |
| D-13779 | Me(R) | single bond | H | *c*-Pr(1-CH₂F) | Me | Me | H | O |
| D-13780 | Me(S) | single bond | H | *c*-Pr(1-CH₂F) | Me | Me | H | O |
| D-13781 | H | single bond | Me | *c*-Pr(1-CHF₂) | Me | Cl | H | O |
| D-13782 | Me | single bond | H | *c*-Pr(1-CHF₂) | Me | Cl | H | O |
| D-13783 | Me(R) | single bond | H | *c*-Pr(1-CHF₂) | Me | Cl | H | O |
| D-13784 | Me(S) | single bond | H | *c*-Pr(1-CHF₂) | Me | Cl | H | O |
| D-13785 | H | single bond | Me | *c*-Pr(1-CHF₂) | Me | Me | H | O |
| D-13786 | Me | single bond | H | *c*-Pr(1-CHF₂) | Me | Me | H | O |
| D-13787 | Me(R) | single bond | H | *c*-Pr(1-CHF₂) | Me | Me | H | O |
| D-13788 | Me(S) | single bond | H | *c*-Pr(1-CHF₂) | Me | Me | H | O |
| D-13789 | H | single bond | Me | *c*-Pr(1-CF₃) | Me | Cl | H | O |
| D-13790 | Me | single bond | H | *c*-Pr(1-CF₃) | Me | Cl | H | O |

**[Table 960]**

| Compound Number | Y | W | Z | R¹ | R² | R³ | R¹⁰ | A |
|---|---|---|---|---|---|---|---|---|
| D-13791 | Me(R) | single bond | H | *c*-Pr(1-CF₃) | Me | Cl | H | O |
| D-13792 | Me(S) | single bond | H | *c*-Pr(1-CF₃) | Me | Cl | H | O |
| D-13793 | H | single bond | Me | *c*-Pr(1-CF₃) | Me | Me | H | O |
| D-13794 | Me | single bond | H | *c*-Pr(1-CF₃) | Me | Me | H | O |
| D-13795 | Me(R) | single bond | H | *c*-Pr(1-CF₃) | Me | Me | H | O |
| D-13796 | Me(S) | single bond | H | *c*-Pr(1-CF₃) | Me | Me | H | O |
| D-13797 | H | single bond | Me | *c*-Pr(1-C≡CH) | Me | Cl | H | O |
| D-13798 | Me | single bond | H | *c*-Pr(1-C≡CH) | Me | Cl | H | O |
| D-13799 | Me(R) | single bond | H | *c*-Pr(1-C≡CH) | Me | Cl | H | O |
| D-13800 | Me(S) | single bond | H | *c*-Pr(1-C≡CH) | Me | Cl | H | O |
| D-13801 | H | single bond | Me | *c*-Pr(1-C≡CH) | Me | Me | H | O |
| D-13802 | Me | single bond | H | *c*-Pr(1-C≡CH) | Me | Me | H | O |
| D-13803 | Me(R) | single bond | H | *c*-Pr(1-C≡CH) | Me | Me | H | O |
| D-13804 | Me(S) | single bond | H | *c*-Pr(1-C≡CH) | Me | Me | H | O |
| D-13805 | H | single bond | Me | *c*-Pr[1-C(=O)OEt] | Me | Cl | H | O |
| D-13806 | Me | single bond | H | *c*-Pr[1-C(=O)OEt] | Me | Cl | H | O |
| D-13807 | Me(R) | single bond | H | *c*-Pr[1-C(=O)OEt] | Me | Cl | H | O |
| D-13808 | Me(S) | single bond | H | *c*-Pr[1-C(=O)OEt] | Me | Cl | H | O |
| D-13809 | H | single bond | Me | *c*-Pr[1-C(=O)OEt] | Me | Me | H | O |
| D-13810 | Me | single bond | H | *c*-Pr[1-C(=O)OEt] | Me | Me | H | O |
| D-13811 | Me(R) | single bond | H | *c*-Pr[1-C(=O)OEt] | Me | Me | H | O |
| D-13812 | Me(S) | single bond | H | *c*-Pr[1-C(=O)OEt] | Me | Me | H | O |
| D-13813 | H | single bond | Me | *c*-Pr[1-C(=O)OH] | Me | Cl | H | O |
| D-13814 | Me | single bond | H | *c*-Pr[1-C(=O)OH] | Me | Cl | H | O |
| D-13815 | Me(R) | single bond | H | *c*-Pr[1-C(=O)OH] | Me | Cl | H | O |
| D-13816 | Me(S) | single bond | H | *c*-Pr[1-C(=O)OH] | Me | Cl | H | O |
| D-13817 | H | single bond | Me | *c*-Pr[1-C(=O)OH] | Me | Me | H | O |
| D-13818 | Me | single bond | H | *c*-Pr[1-C(=O)OH] | Me | Me | H | O |
| D-13819 | Me(R) | single bond | H | *c*-Pr[1-C(=O)OH] | Me | Me | H | O |
| D-13820 | Me(S) | single bond | H | *c*-Pr[1-C(=O)OH] | Me | Me | H | O |
| D-13821 | H | single bond | Me | *c*-Pr[1-C(=O)NH₂] | Me | Cl | H | O |
| D-13822 | Me | single bond | H | *c*-Pr[1-C(=O)NH₂] | Me | Cl | H | O |
| D-13823 | Me(R) | single bond | H | *c*-Pr[1-C(=O)NH₂] | Me | Cl | H | O |
| D-13824 | Me(S) | single bond | H | *c*-Pr[1-C(=O)NH₂] | Me | Cl | H | O |
| D-13825 | H | single bond | Me | *c*-Pr[1-C(=O)NH₂] | Me | Me | H | O |
| D-13826 | Me | single bond | H | *c*-Pr[1-C(=O)NH₂] | Me | Me | H | O |
| D-13827 | Me(R) | single bond | H | *c*-Pr[1-C(=O)NH₂] | Me | Me | H | O |
| D-13828 | Me(S) | single bond | H | *c*-Pr[1-C(=O)NH₂] | Me | Me | H | O |
| D-13829 | H | single bond | Me | *c*-Pr(1-OMe) | Me | Cl | H | O |
| D-13830 | Me | single bond | H | *c*-Pr(1-OMe) | Me | Cl | H | O |
| D-13831 | Me(R) | single bond | H | *c*-Pr(1-OMe) | Me | Cl | H | O |
| D-13832 | Me(S) | single bond | H | *c*-Pr(1-OMe) | Me | Cl | H | O |
| D-13833 | H | single bond | Me | *c*-Pr(1-OMe) | Me | Me | H | O |
| D-13834 | Me | single bond | H | *c*-Pr(1-OMe) | Me | Me | H | O |

**[Table 961]**

| Compound Number | Y | W | Z | R¹ | R² | R³ | R¹⁰ | A |
|---|---|---|---|---|---|---|---|---|
| D-13835 | Me(R) | single bond | H | *c*-Pr(1-OMe) | Me | Me | H | O |
| D-13836 | Me(S) | single bond | H | *c*-Pr(1-OMe) | Me | Me | H | O |
| D-13837 | H | single bond | Me | *c*-Pr(1-SMe) | Me | Cl | H | O |
| D-13838 | Me | single bond | H | *c*-Pr(1-SMe) | Me | Cl | H | O |
| D-13839 | Me(R) | single bond | H | *c*-Pr(1-SMe) | Me | Cl | H | O |
| D-13840 | Me(S) | single bond | H | *c*-Pr(1-SMe) | Me | Cl | H | O |
| D-13841 | H | single bond | Me | *c*-Pr(1-SMe) | Me | Me | H | O |
| D-13842 | Me | single bond | H | *c*-Pr(1-SMe) | Me | Me | H | O |
| D-13843 | Me(R) | single bond | H | *c*-Pr(1-SMe) | Me | Me | H | O |
| D-13844 | Me(S) | single bond | H | *c*-Pr(1-SMe) | Me | Me | H | O |
| D-13845 | H | single bond | Me | *c* -Pr[1-S(=O)Me] | Me | Cl | H | O |
| D-13846 | Me | single bond | H | *c*-pr[1-S(=O)Me] | Me | Cl | H | O |
| D-13847 | Me(R) | single bond | H | *c*-Pr[1-S(=O)Me] | Me | Cl | H | O |
| D-13848 | Me(S) | single bond | H | *c*-pr[1-S(=O)Me] | Me | Cl | H | O |
| D-13849 | H | single bond | Me | *c*-pr[1-S(=O)Me] | Me | Me | H | O |
| D-13850 | Me | single bond | H | *c*-pr[1-S(=O)Me] | Me | Me | H | O |
| D-13851 | Me(R) | single bond | H | *c*-pr[1-S(=O)Me] | Me | Me | H | O |
| D-13852 | Me(S) | single bond | H | *c*-Pr[1-S(=O)Me] | Me | Me | H | O |
| D-13853 | H | single bond | Me | *c*-Pr[1-S(=O)₂Me] | Me | Cl | H | O |
| D-13854 | Me | single bond | H | *c*-Pr[1-S(=O)₂Me] | Me | Cl | H | O |
| D-13855 | Me(R) | single bond | H | *c*-Pr[1-S(=O)₂Me] | Me | Cl | H | O |
| D-13856 | Me(S) | single bond | H | *c*-Pr[1-S(=O)₂Me] | Me | Cl | H | O |
| D-13857 | H | single bond | Me | *c*-Pr[1-S(=O)₂Me] | Me | Me | H | O |
| D-13858 | Me | single bond | H | *c*-Pr[1-S(=O)₂Me] | Me | Me | H | O |
| D-13859 | Me(R) | single bond | H | *c*-Pr[1-S(=O)₂Me] | Me | Me | H | O |
| D-13860 | Me(S) | single bond | H | *c*-Pr[1-S(=O)₂Me] | Me | Me | H | O |
| D-13861 | H | single bond | Me | *c*-Pr[1-S(=O)₂CHF₂] | Me | Cl | H | O |
| D-13862 | Me | single bond | H | *c*-Pr[1-S(=O)₂CHF₂] | Me | Cl | H | O |
| D-13863 | Me(R) | single bond | H | *c*-Pr[1-S(=O)₂CHF₂] | Me | Cl | H | O |
| D-13864 | Me(S) | single bond | H | *c*-Pr[1-S(=O)₂CHF₂] | Me | Cl | H | O |
| D-13865 | H | single bond | Me | *c*-pr[1-S(=O)₂CHF₂] | Me | Me | H | O |
| D-13866 | Me | single bond | H | *c*-Pr[1-S(=O)₂CHF₂] | Me | Me | H | O |
| D-13867 | Me(R) | single bond | H | *c*-Pr[1-S(=O)₂CHF₂] | Me | Me | H | O |
| D-13868 | Me(S) | single bond | H | *c*-pr[1-S(=O)₂CHF₂] | Me | Me | H | O |
| D-13869 | H | single bond | Me | *c*-Pr[1-S(=O)₂CF₃] | Me | Cl | H | O |
| D-13870 | Me | single bond | H | *c*-Pr[1-S(=O)₂CF₃] | Me | Cl | H | O |
| D-13871 | Me(R) | single bond | H | *c*-Pr[1-S(=O)₂CF₃] | Me | Cl | H | O |
| D-13872 | Me(S) | single bond | H | *c*-Pr[1-S(=O)₂CF₃] | Me | Cl | H | O |
| D-13873 | H | single bond | Me | *c*-Pr[1-S(=O)₂CF₃] | Me | Me | H | O |
| D-13874 | Me | single bond | H | *c*-Pr[1-S(=O)₂CF₃] | Me | Me | H | O |
| D-13875 | Me(R) | single bond | H | *c*-Pr[1-S(=O)₂CF₃] | Me | Me | H | O |
| D-13876 | Me(S) | single bond | H | *c*-Pr[1-S(=O)₂CF₃] | Me | Me | H | O |
| D-13877 | H | single bond | Me | *c*-Pr[1-(5,6-dihydro-1,4,2-dioxazin-3-yl)] | Me | Cl | H | O |
| D-13878 | Me | single bond | H | *c*-Pr[1-(5,6-dihydro-1,4,2-dioxazin-3-yl)] | Me | Cl | H | O |

**[Table 962]**

| Compound Number | Y | W | Z | R¹ | R² | R³ | R¹⁰ | A |
|---|---|---|---|---|---|---|---|---|
| D-13879 | Me(R) | single bond | H | *c* -Pr[1-(5,6-dihydro-1,4,2-dioxazin-3-yl)] | Me | Cl | H | O |
| D-13880 | Me(S) | single bond | H | *c* -Pr[1-(5,6-dihydro-1,4,2-dioxazin-3-yl)] | Me | Cl | H | O |
| D-13881 | H | single bond | Me | *c* -Pr[1-(5,6-dihydro-1,4,2-dioxazin-3-yl)] | Me | Me | H | O |
| D-13882 | Me | single bond | H | *c* -Pr[1-(5,6-dihydro-1,4,2-dioxazin-3-yl)] | Me | Me | H | O |
| D-13883 | Me(R) | single bond | H | *c* -Pr[1-(5,6-dihydro-1,4,2-dioxazin-3-yl)] | Me | Me | H | O |
| D-13884 | Me(S) | single bond | H | *c* -Pr[1-(5,6-dihydro-1,4,2-dioxazin-3-yl)] | Me | Me | H | O |
| D-13885 | H | single bond | Me | *c* -Pr[1-(5-hydroxy-1,3,4-oxadiazol-2-yl)] | Me | Cl | H | O |
| D-13886 | Me | single bond | H | *c* -Pr[1-(5-hydroxy-1,3,4-oxadiazol-2-yl)] | Me | Cl | H | O |
| D-13887 | Me(R) | single bond | H | *c* -Pr[1-(5-hydroxy-1,3,4-oxadiazol-2-yl)] | Me | Cl | H | O |
| D-13888 | Me(S) | single bond | H | *c* -Pr[1-(5-hydroxy-1,3,4-oxadiazol-2-yl)] | Me | Cl | H | O |
| D-13889 | H | single bond | Me | *c* -Pr[1-(5-hydroxy-1,3,4-oxadiazol-2-yl)] | Me | Me | H | O |
| D-13890 | Me | single bond | H | *c* -Pr[1-(5-hydroxy-1,3,4-oxadiazol-2-yl)] | Me | Me | H | O |
| D-13891 | Me(R) | single bond | H | *c* -Pr[1-(5-hydroxy-1,3,4-oxadiazol-2-yl)] | Me | Me | H | O |
| D-13892 | Me(S) | single bond | H | *c* -Pr[1-(5-hydroxy-1,3,4-oxadiazol-2-yl)] | Me | Me | H | O |
| D-13893 | H | single bond | Me | *c* -Pr[1-(pyrimidin-2-yl)] | Me | Cl | H | O |
| D-13894 | Me | single bond | H | *c* -Pr[1-(pyrimidin-2-yl)] | Me | Cl | H | O |
| D-13895 | Me(R) | single bond | H | *c* -Pr[1-(pyrimidin-2-yl)] | Me | Cl | H | O |
| D-13896 | Me(S) | single bond | H | *c* -Pr[1-(pyrimidin-2-yl)] | Me | Cl | H | O |
| D-13897 | H | single bond | Me | *c* -Pr[1-(pyrimidin-2-yl)] | Me | Me | H | O |
| D-13898 | Me | single bond | H | *c* -Pr[1-(pyrimidin-2-yl)] | Me | Me | H | O |
| D-13899 | Me(R) | single bond | H | *c* -Pr[1-(pyrimidin-2-yl)] | Me | Me | H | O |
| D-13900 | Me(S) | single bond | H | *c* -Pr[1-(pyrimidin-2-yl)] | Me | Me | H | O |
| D-13901 | H | single bond | Me | *t*-Bu | Me | Cl | H | O |
| D-13902 | Me | single bond | H | *t*-Bu | Me | Cl | H | O |
| D-13903 | Me(R) | single bond | H | *t*-Bu | Me | Cl | H | O |
| D-13904 | Me(S) | single bond | H | *t*-Bu | Me | Cl | H | O |
| D-13905 | H | single bond | Me | *t*-Bu | Me | Me | H | O |
| D-13906 | Me | single bond | H | *t*-Bu | Me | Me | H | O |
| D-13907 | Me(R) | single bond | H | *t*-Bu | Me | Me | H | O |
| D-13908 | Me(S) | single bond | H | *t*-Bu | Me | Me | H | O |
| D-13909 | H | single bond | Me | *c*-Bu | Me | Cl | H | O |
| D-13910 | Me | single bond | H | *c*-Bu | Me | Cl | H | O |
| D-13911 | Me(R) | single bond | H | *c*-Bu | Me | Cl | H | O |
| D-13912 | Me(S) | single bond | H | *c*-Bu | Me | Cl | H | O |
| D-13913 | H | single bond | Me | *c*-Bu | Me | Me | H | O |
| D-13914 | Me | single bond | H | *c*-Bu | Me | Me | H | O |
| D-13915 | Me(R) | single bond | H | *c*-Bu | Me | Me | H | O |
| D-13916 | Me(S) | single bond | H | *c*-Bu | Me | Me | H | O |
| D-13917 | H | single bond | Me | *c*-Bu(1-CN) | Me | Cl | H | O |
| D-13918 | Me | single bond | H | *c*-Bu(1-CN) | Me | Cl | H | O |
| D-13919 | Me(R) | single bond | H | *c*-Bu(1-CN) | Me | Cl | H | O |
| D-13920 | Me(S) | single bond | H | *c*-Bu(1-CN) | Me | Cl | H | O |
| D-13921 | H | single bond | Me | *c*-Bu(1-CN) | Me | Me | H | O |
| D-13922 | Me | single bond | H | *c*-Bu(1-CN) | Me | Me | H | O |

**[Table 963]**

| Compound Number | Y | W | Z | R¹ | R² | R³ | R¹⁰ | A |
|---|---|---|---|---|---|---|---|---|
| D-13923 | Me(R) | single bond | H | *c*-Bu(1-CN) | Me | Me | H | O |
| D-13924 | Me(S) | single bond | H | *c*-Bu(1-CN) | Me | Me | H | O |
| D-13925 | H | single bond | Me | *c*-Bu(3,3-F₂) | Me | Cl | H | O |
| D-13926 | Me | single bond | H | *c*-Bu(3,3-F₂) | Me | Cl | H | O |
| D-13927 | Me(R) | single bond | H | *c*-Bu(3,3-F₂) | Me | Cl | H | O |
| D-13928 | Me(S) | single bond | H | *c*-Bu(3,3-F₂) | Me | Cl | H | O |
| D-13929 | H | single bond | Me | *c*-Bu(3,3-F₂) | Me | Me | H | O |
| D-13930 | Me | single bond | H | *c*-Bu(3,3-F₂) | Me | Me | H | O |
| D-13931 | Me(R) | single bond | H | *c*-Bu(3,3-F₂) | Me | Me | H | O |
| D-13932 | Me(S) | single bond | H | *c*-Bu(3,3-F₂) | Me | Me | H | O |
| D-13933 | H | single bond | Me | oxetan-3-yl | Me | Cl | H | O |
| D-13934 | Me | single bond | H | oxetan-3-yl | Me | Cl | H | O |
| D-13935 | Me(R) | single bond | H | oxetan-3-yl | Me | Cl | H | O |
| D-13936 | Me(S) | single bond | H | oxetan-3-yl | Me | Cl | H | O |
| D-13937 | H | single bond | Me | oxetan-3-yl | Me | Me | H | O |
| D-13938 | Me | single bond | H | oxetan-3-yl | Me | Me | H | O |
| D-13939 | Me(R) | single bond | H | oxetan-3-yl | Me | Me | H | O |
| D-13940 | Me(S) | single bond | H | oxetan-3-yl | Me | Me | H | O |
| D-13941 | H | single bond | Me | oxetan(3-CN)-3-yl | Me | Cl | H | O |
| D-13942 | Me | single bond | H | oxetan(3-CN)-3-yl | Me | Cl | H | O |
| D-13943 | Me(R) | single bond | H | oxetan(3-CN)-3-yl | Me | Cl | H | O |
| D-13944 | Me(S) | single bond | H | oxetan(3-CN)-3-yl | Me | Cl | H | O |
| D-13945 | H | single bond | Me | oxetan(3-CN)-3-yl | Me | Me | H | O |
| D-13946 | Me | single bond | H | oxetan(3-CN)-3-yl | Me | Me | H | O |
| D-13947 | Me(R) | single bond | H | oxetan(3-CN)-3-yl | Me | Me | H | O |
| D-13948 | Me(S) | single bond | H | oxetan(3-CN)-3-yl | Me | Me | H | O |
| D-13949 | H | single bond | Me | azetidin[1-C(=O)Me]-3-yl | Me | Cl | H | O |
| D-13950 | Me | single bond | H | azetidin[1-C(=O)Me]-3-yl | Me | Cl | H | O |
| D-13951 | Me(R) | single bond | H | azetidin[1-C(=O)Me]-3-yl | Me | Cl | H | O |
| D-13952 | Me(S) | single bond | H | azetidin[1-C(=O)Me]-3-yl | Me | Cl | H | O |
| D-13953 | H | single bond | Me | azetidin[1-C(=O)Me]-3-yl | Me | Me | H | O |
| D-13954 | Me | single bond | H | azetidin[1-C(=O)Me]-3-yl | Me | Me | H | O |
| D-13955 | Me(R) | single bond | H | azetidin[1-C(=O)Me]-3-yl | Me | Me | H | O |
| D-13956 | Me(S) | single bond | H | azetidin[1-C(=O)Me]-3-yl | Me | Me | H | O |
| D-13957 | H | single bond | Me | azetidin[1-S(=O)₂Me]-3-yl | Me | Cl | H | O |
| D-13958 | Me | single bond | H | azetidin[1-S(=O)₂Me]-3-yl | Me | Cl | H | O |
| D-13959 | Me(R) | single bond | H | azetidin[1-S(=O)₂Me]-3-yl | Me | Cl | H | O |
| D-13960 | Me(S) | single bond | H | azetidin[1-S(=O)₂Me]-3-yl | Me | Cl | H | O |
| D-13961 | H | single bond | Me | azetidin[1-S(=O)₂Me]-3-yl | Me | Me | H | O |
| D-13962 | Me | single bond | H | azetidin[1-S(=O)₂Me]-3-yl | Me | Me | H | O |
| D-13963 | Me(R) | single bond | H | azetidin[1-S(=O)₂Me]-3-yl | Me | Me | H | O |
| D-13964 | Me(S) | single bond | H | azetidin[1-S(=O)₂Me]-3-yl | Me | Me | H | O |
| D-13965 | H | single bond | Me | azetidin[1-S(=O)₂CHF₂]-3-yl | Me | Cl | H | O |
| D-13966 | Me | single bond | H | azetidin[1-S(=O)₂CHF₂]-3-yl | Me | Cl | H | O |

**[Table 964]**

| Compound Number | Y | W | Z | R¹ | R² | R³ | R¹⁰ | A |
|---|---|---|---|---|---|---|---|---|
| D-13967 | Me(R) | single bond | H | azetidin[1-S(=O)₂CHF₂]-3-yl | Me | Cl | H | O |
| D-13968 | Me(S) | single bond | H | azetidin[1-S(=O)₂CHF₂]-3-yl | Me | Cl | H | O |
| D-13969 | H | single bond | Me | azetidin[1-S(=O)₂CHF₂]-3-yl | Me | Me | H | O |
| D-13970 | Me | single bond | H | azetidin[1-S(=O)₂CHF₂]-3-yl | Me | Me | H | O |
| D-13971 | Me(R) | single bond | H | azetidin[1-S(=O)₂CHF₂]-3-yl | Me | Me | H | O |
| D-13972 | Me(S) | single bond | H | azetidin[1-S(=O)₂CHF₂]-3-yl | Me | Me | H | O |
| D-13973 | H | single bond | Me | *c*-Pen | Me | Cl | H | O |
| D-13974 | Me | single bond | H | *c*-Pen | Me | Cl | H | O |
| D-13975 | Me(R) | single bond | H | *c*-Pen | Me | Cl | H | O |
| D-13976 | Me(S) | single bond | H | *c*-Pen | Me | Cl | H | O |
| D-13977 | H | single bond | Me | *c*-Pen | Me | Me | H | O |
| D-13978 | Me | single bond | H | *c*-Pen | Me | Me | H | O |
| D-13979 | Me(R) | single bond | H | *c*-Pen | Me | Me | H | O |
| D-13980 | Me(S) | single bond | H | *c*-Pen | Me | Me | H | O |
| D-13981 | H | single bond | Me | tetrahydrofuran-3-yl | Me | Cl | H | O |
| D-13982 | Me | single bond | H | tetrahydrofuran-3-yl | Me | Cl | H | O |
| D-13983 | Me(R) | single bond | H | tetrahydrofuran-3-yl | Me | Cl | H | O |
| D-13984 | Me(S) | single bond | H | tetrahydrofuran-3-yl | Me | Cl | H | O |
| D-13985 | H | single bond | Me | tetrahydrofuran-3-yl | Me | Me | H | O |
| D-13986 | Me | single bond | H | tetrahydrofuran-3-yl | Me | Me | H | O |
| D-13987 | Me(R) | single bond | H | tetrahydrofuran-3-yl | Me | Me | H | O |
| D-13988 | Me(S) | single bond | H | tetrahydrofuran-3-yl | Me | Me | H | O |
| D-13989 | H | single bond | Me | tetrahydrothiophen-3-yl | Me | Cl | H | O |
| D-13990 | Me | single bond | H | tetrahydrothiophen-3-yl | Me | Cl | H | O |
| D-13991 | Me(R) | single bond | H | tetrahydrothiophen-3-yl | Me | Cl | H | O |
| D-13992 | Me(S) | single bond | H | tetrahydrothiophen-3-yl | Me | Cl | H | O |
| D-13993 | H | single bond | Me | tetrahydrothiophen-3-yl | Me | Me | H | O |
| D-13994 | Me | single bond | H | tetrahydrothiophen-3-yl | Me | Me | H | O |
| D-13995 | Me(R) | single bond | H | tetrahydrothiophen-3-yl | Me | Me | H | O |
| D-13996 | Me(S) | single bond | H | tetrahydrothiophen-3-yl | Me | Me | H | O |
| D-13997 | H | single bond | Me | *c*-Pen(1-CN) | Me | Cl | H | O |
| D-13998 | Me | single bond | H | *c*-Pen(1-CN) | Me | Cl | H | O |
| D-13999 | Me(R) | single bond | H | *c*-Pen(1-CN) | Me | Cl | H | O |
| D-14000 | Me(S) | single bond | H | *c*-Pen(1-CN) | Me | Cl | H | O |
| D-14001 | H | single bond | Me | *c*-Pen(1-CN) | Me | Me | H | O |
| D-14002 | Me | single bond | H | *c*-Pen(1-CN) | Me | Me | H | O |
| D-14003 | Me(R) | single bond | H | *c*-Pen(1-CN) | Me | Me | H | O |
| D-14004 | Me(S) | single bond | H | *c*-Pen(1-CN) | Me | Me | H | O |
| D-14005 | H | single bond | Me | tetrahydrofuran(3-CN)-3-yl | Me | Cl | H | O |
| D-14006 | Me | single bond | H | tetrahydrofuran(3-CN)-3-yl | Me | Cl | H | O |
| D-14007 | Me(R) | single bond | H | tetrahydrofuran(3-CN)-3-yl | Me | Cl | H | O |
| D-14008 | Me(S) | single bond | H | tetrahydrofuran(3-CN)-3-yl | Me | Cl | H | O |
| D-14009 | H | single bond | Me | tetrahydrofuran(3-CN)-3-yl | Me | Me | H | O |
| D-14010 | Me | single bond | H | tetrahydrofuran(3-CN)-3-yl | Me | Me | H | O |

**[Table 965]**

| Compound Number | Y | W | Z | R¹ | R² | R³ | R¹⁰ | A |
|---|---|---|---|---|---|---|---|---|
| D-14011 | Me(R) | single bond | H | tetrahydrofuran(3-CN)-3-yl | Me | Me | H | O |
| D-14012 | Me(S) | single bond | H | tetrahydrofuran(3-CN)-3-yl | Me | Me | H | O |
| D-14013 | H | single bond | Me | tetrahydrothiophen(3-CN)-3-yl | Me | Cl | H | O |
| D-14014 | Me | single bond | H | tetrahydrothiophen(3-CN)-3-yl | Me | Cl | H | O |
| D-14015 | Me(R) | single bond | H | tetrahydrothiophen(3-CN)-3-yl | Me | Cl | H | O |
| D-14016 | Me(S) | single bond | H | tetrahydrothiophen(3-CN)-3-yl | Me | Cl | H | O |
| D-14017 | H | single bond | Me | tetrahydrothiophen(3-CN)-3-yl | Me | Me | H | O |
| D-14018 | Me | single bond | H | tetrahydrothiophen(3-CN)-3-yl | Me | Me | H | O |
| D-14019 | Me(R) | single bond | H | tetrahydrothiophen(3-CN)-3-yl | Me | Me | H | O |
| D-14020 | Me(S) | single bond | H | tetrahydrothiophen(3-CN)-3-yl | Me | Me | H | O |
| D-14021 | H | single bond | Me | bicyclo[1.1.1]pertan-1-yl | Me | Cl | H | O |
| D-14022 | Me | single bond | H | bicyclo[1.1.1]pertan-1-yl | Me | Cl | H | O |
| D-14023 | Me(R) | single bond | H | bicyclo[1.1.1]pertan-1-yl | Me | Cl | H | O |
| D-14024 | Me(S) | single bond | H | bicyclo[1.1.1]pertan-1-yl | Me | Cl | H | O |
| D-14025 | H | single bond | Me | bicyclo[1.1.1]pertan-1-yl | Me | Me | H | O |
| D-14026 | Me | single bond | H | bicyclo[1.1.1]pertan-1-yl | Me | Me | H | O |
| D-14027 | Me(R) | single bond | H | bicyclo[1.1.1]pertan-1-yl | Me | Me | H | O |
| D-14028 | Me(S) | single bond | H | bicyclo[1.1.1]pertan-1-yl | Me | Me | H | O |
| D-14029 | H | single bond | Me | *c*-Hex | Me | Cl | H | O |
| D-14030 | Me | single bond | H | *c*-Hex | Me | Cl | H | O |
| D-14031 | Me(R) | single bond | H | *c*-Hex | Me | Cl | H | O |
| D-14032 | Me(S) | single bond | H | *c*-Hex | Me | Cl | H | O |
| D-14033 | H | single bond | Me | *c*-Hex | Me | Me | H | O |
| D-14034 | Me | single bond | H | *c*-Hex | Me | Me | H | O |
| D-14035 | Me(R) | single bond | H | *c*-Hex | Me | Me | H | O |
| D-14036 | Me(S) | single bond | H | *c*-Hex | Me | Me | H | O |
| D-14037 | H | single bond | Me | tetrahydropyran-4-yl | Me | Cl | H | O |
| D-14038 | Me | single bond | H | tetrahydropyran-4-yl | Me | Cl | H | O |
| D-14039 | Me(R) | single bond | H | tetrahydropyran-4-yl | Me | Cl | H | O |
| D-14040 | Me(S) | single bond | H | tetrahydropyran-4-yl | Me | Cl | H | O |
| D-14041 | H | single bond | Me | tetrahydropyran-4-yl | Me | Me | H | O |
| D-14042 | Me | single bond | H | tetrahydropyran-4-yl | Me | Me | H | O |
| D-14043 | Me(R) | single bond | H | tetrahydropyran-4-yl | Me | Me | H | O |
| D-14044 | Me(S) | single bond | H | tetrahydropyran-4-yl | Me | Me | H | O |
| D-14045 | H | single bond | Me | tetrahydrothiopyran-4-yl | Me | Cl | H | O |
| D-14046 | Me | single bond | H | tetrahydrothiopyran-4-yl | Me | Cl | H | O |
| D-14047 | Me(R) | single bond | H | tetrahydrothiopyran-4-yl | Me | Cl | H | O |
| D-14048 | Me(S) | single bond | H | tetrahydrothiopyran-4-yl | Me | Cl | H | O |
| D-14049 | H | single bond | Me | tetrahydrothiopyran-4-yl | Me | Me | H | O |
| D-14050 | Me | single bond | H | tetrahydrothiopyran-4-yl | Me | Me | H | O |
| D-14051 | Me(R) | single bond | H | tetrahydrothiopyran-4-yl | Me | Me | H | O |
| D-14052 | Me(S) | single bond | H | tetrahydrothiopyran-4-yl | Me | Me | H | O |
| D-14053 | H | single bond | Me | *c*-Hex(1-CN) | Me | Cl | H | O |
| D-14054 | Me | single bond | H | *c*-Hex(1-CN) | Me | Cl | H | O |

**[Table 966]**

| Compound Number | Y | W | Z | R¹ | R² | R³ | R¹⁰ | A |
|---|---|---|---|---|---|---|---|---|
| 0-14055 | Me(R) | single bond | H | *c*-Hex(1-CN) | Me | Cl | H | O |
| D-14056 | Me(S) | single bond | H | *c*-Hex(1-CN) | Me | Cl | H | O |
| D-14057 | H | single bond | Me | *c*-Hex(1-CN) | Me | Me | H | O |
| D-14058 | Me | single bond | H | *c*-Hex(1-CN) | Me | Me | H | O |
| D-14059 | Me(R) | single bond | H | *c*-Hex(1-CN) | Me | Me | H | O |
| D-14060 | Me(S) | single bond | H | *c*-Hex(1-CN) | Me | Me | H | O |
| D-14061 | H | single bond | Me | tetrahydropyran(4-CN)-4-yl | Me | Cl | H | O |
| D-14062 | Me | single bond | H | tetrahydropyran(4-CN)-4-yl | Me | Cl | H | O |
| D-14063 | Me(R) | single bond | H | tetrahydropyran(4-CN)-4-yl | Me | Cl | H | O |
| D-14064 | Me(S) | single bond | H | tetrahydropyran(4-CN)-4-yl | Me | Cl | H | O |
| D-14065 | H | single bond | Me | tetrahydropyran(4-CN)-4-yl | Me | Me | H | O |
| D-14066 | Me | single bond | H | tetrahydropyran(4-CN)-4-yl | Me | Me | H | O |
| D-14067 | Me(R) | single bond | H | tetrahydropyran(4-CN)-4-yl | Me | Me | H | O |
| D-14068 | Me(S) | single bond | H | tetrahydropyran(4-CN)-4-yl | Me | Me | H | O |
| D-14069 | H | single bond | Me | tetrahydrothiopyran(4-CN)-4-yl | Me | Cl | H | O |
| D-14070 | Me | single bond | H | tetrahydrothiopyran(4-CN)-4-yl | Me | Cl | H | O |
| D-14071 | Me(R) | single bond | H | tetrahydrothiopyran(4-CN)-4-yl | Me | Cl | H | O |
| D-14072 | Me(S) | single bond | H | tetrahydrothiopyran(4-CN)-4-yl | Me | Cl | H | O |
| D-14073 | H | single bond | Me | tetrahydrothiopyran(4-CN)-4-yl | Me | Me | H | O |
| D-14074 | Me | single bond | H | tetrahydrothiopyran(4-CN)-4-yl | Me | Me | H | O |
| 0-14075 | Me(R) | single bond | H | tetrahydrothiopyran(4-CN)-4-yl | Me | Me | H | O |
| 0-14076 | Me(S) | single bond | H | tetrahydrothiopyran(4-CN)-4-yl | Me | Me | H | O |
| D-14077 | H | single bond | Me | CH₂(*c*-Pr) | Me | Cl | H | O |
| D-14078 | Me | single bond | H | CH₂(*c*-Pr) | Me | Cl | H | O |
| 0-14079 | Me(R) | single bond | H | CH₂(*c*-Pr) | Me | Cl | H | O |
| D-14080 | Me(S) | single bond | H | CH₂(*c*-Pr) | Me | Cl | H | O |
| D-14081 | H | single bond | Me | CH₂(*c*-Pr) | Me | Me | H | O |
| D-14082 | Me | single bond | H | CH₂(*c*-Pr) | Me | Me | H | O |
| D-14083 | Me(R) | single bond | H | CH₂(*c*-Pr) | Me | Me | H | O |
| D-14084 | Me(S) | single bond | H | CH₂(*c*-Pr) | Me | Me | H | O |
| D-14085 | H | single bond | Me | CH₂[*c*-Pr(1-CN)] | Me | Cl | H | O |
| D-14086 | Me | single bond | H | CH₂[*c*-Pr(1-CN)] | Me | Cl | H | O |
| 0-14087 | Me(R) | single bond | H | CH₂[*c*-Pr(1-CN)] | Me | Cl | H | O |
| 0-14088 | Me(S) | single bond | H | CH₂[*c*-Pr(1-CN)] | Me | Cl | H | O |
| D-14089 | H | single bond | Me | CH₂[*c*-Pr(1-CN)] | Me | Me | H | O |
| D-14090 | Me | single bond | H | CH₂[*c*-Pr(1-CN)] | Me | Me | H | O |
| D-14091 | Me(R) | single bond | H | CH₂[*c*-Pr(1-CN)] | Me | Me | H | O |
| D-14092 | Me(S) | single bond | H | CH₂[*c*-Pr(1-CN)] | Me | Me | H | O |
| D-14093 | H | single bond | Me | CH(Me)(*c*-Pr) | Me | Cl | H | O |
| D-14094 | Me | single bond | H | CH(Me)(*c*-Pr) | Me | Cl | H | O |
| D-14005 | Me(R) | single bond | H | CH(Me)(*c*-Pr) | Me | Cl | H | O |
| D-14096 | Me(S) | single bond | H | CH(Me)(*c*-Pr) | Me | Cl | H | O |
| D-14097 | H | single bond | Me | CH(Me)(*c*-Pr) | Me | Me | H | O |
| D-14098 | Me | single bond | H | CH(Me)(*c*-Pr) | Me | Me | H | O |

**[Table 967]**

| Compound Number | Y | W | Z | R¹ | R² | R³ | R¹⁰ | A |
|---|---|---|---|---|---|---|---|---|
| 0-14099 | Me(R) | single bond | H | CH(Me)(*c*-Pr) | Me | Me | H | O |
| D-14100 | Me(S) | single bond | H | CH(Me)(*c*-Pr) | Me | Me | H | O |
| D-14101 | H | single bond | Me | CH(Me)CH₂OMe | Me | Cl | H | O |
| D-14102 | Me | single bond | H | CH(Me)CH₂OMe | Me | Cl | H | O |
| D-14103 | Me(R) | single bond | H | CH(Me)CH₂OMe | Me | Cl | H | O |
| D-14104 | Me(S) | single bond | H | CH(Me)CH₂OMe | Me | Cl | H | O |
| D-14105 | H | single bond | Me | CH(Me)CH₂OMe | Me | Me | H | O |
| D-14106 | Me | single bond | H | CH(Me)CH₂OMe | Me | Me | H | O |
| D-14107 | Me(R) | single bond | H | CH(Me)CH₂OMe | Me | Me | H | O |
| D-14108 | Me(S) | single bond | H | CH(Me)CH₂OMe | Me | Me | H | O |
| D-14109 | H | single bond | Me | CH₂CH=CH₂ | Me | Cl | H | O |
| D-14110 | Me | single bond | H | CH₂CH=CH₂ | Me | Cl | H | O |
| D-14111 | Me(R) | single bond | H | CH₂CH=CH₂ | Me | Cl | H | O |
| D-14112 | Me(S) | single bond | H | CH₂CH=CH₂ | Me | Cl | H | O |
| D-14113 | H | single bond | Me | CH₂CH=CH₂ | Me | Me | H | O |
| D-14114 | Me | single bond | H | CH₂CH=CH₂ | Me | Me | H | O |
| D-14115 | Me(R) | single bond | H | CH₂CH=CH₂ | Me | Me | H | O |
| D-14116 | Me(S) | single bond | H | CH₂CH=CH₂ | Me | Me | H | O |
| D-14117 | H | single bond | Me | CH₂C≡CH | Me | Cl | H | O |
| D-14118 | Me | single bond | H | CH₂C≡CH | Me | Cl | H | O |
| D-14119 | Me(R) | single bond | H | CH₂C≡CH | Me | Cl | H | O |
| D-14120 | Me(S) | single bond | H | CH₂C≡CH | Me | Cl | H | O |
| D-14121 | H | single bond | Me | CH₂C≡CH | Me | Me | H | O |
| D-14122 | Me | single bond | H | CH₂C≡CH | Me | Me | H | O |
| D-14123 | Me(R) | single bond | H | CH₂C≡CH | Me | Me | H | O |
| D-14124 | Me(S) | single bond | H | CH₂C≡CH | Me | Me | H | O |
| D-14125 | H | single bond | Me | CH₂CN | Me | Cl | H | O |
| D-14126 | Me | single bond | H | CH₂CN | Me | Cl | H | O |
| D-14127 | Me(R) | single bond | H | CH₂CN | Me | Cl | H | O |
| D-14128 | Me(S) | single bond | H | CH₂CN | Me | Cl | H | O |
| D-14129 | H | single bond | Me | CH₂CN | Me | Me | H | O |
| D-14130 | Me | single bond | H | CH₂CN | Me | Me | H | O |
| D-14131 | Me(R) | single bond | H | CH₂CN | Me | Me | H | O |
| D-14132 | Me(S) | single bond | H | CH₂CN | Me | Me | H | O |
| D-14133 | H | single bond | Me | CH(Me)CN | Me | Cl | H | O |
| D-14134 | Me | single bond | H | CH(Me)CN | Me | Cl | H | O |
| D-14135 | Me(R) | single bond | H | CH(Me)CN | Me | Cl | H | O |
| D-14136 | Me(S) | single bond | H | CH(Me)CN | Me | Cl | H | O |
| D-14137 | H | single bond | Me | CH(Me)CN | Me | Me | H | O |
| D-14138 | Me | single bond | H | CH(Me)CN | Me | Me | H | O |
| D-14139 | Me(R) | single bond | H | CH(Me)CN | Me | Me | H | O |
| D-14140 | Me(S) | single bond | H | CH(Me)CN | Me | Me | H | O |
| D-14141 | H | single bond | Me | C(Me)₂CN | Me | Cl | H | O |
| D-14142 | Me | single bond | H | C(Me)₂CN | Me | Cl | H | O |

**[Table 968]**

| Compound Number | Y | W | Z | R¹ | R² | R³ | R¹⁰ | A |
|---|---|---|---|---|---|---|---|---|
| D-14143 | Me(R) | single bond | H | C(Me)₂CN | Me | Cl | H | O |
| D-14144 | Me(S) | single bond | H | C(Me)₂CN | Me | Cl | H | O |
| D-14145 | H | single bond | Me | C(Me)₂CN | Me | Me | H | O |
| D-14146 | Me | single bond | H | C(Me)₂CN | Me | Me | H | O |
| D-14147 | Me(R) | single bond | H | C(Me)₂CN | Me | Me | H | O |
| D-14148 | Me(S) | single bond | H | C(Me)₂CN | Me | Me | H | O |
| D-14149 | H | single bond | Me | C(Me)(*i*-Pr)CN | Me | Cl | H | O |
| D-14150 | Me | single bond | H | C(Me)(*i*-Pr)CN | Me | Cl | H | O |
| D-14151 | Me(R) | single bond | H | C(Me)(*i*-Pr)CN | Me | Cl | H | O |
| D-14152 | Me(S) | single bond | H | C(Me)(*i*-Pr)CN | Me | Cl | H | O |
| D-14153 | H | single bond | Me | C(Me)(*i*-Pr)CN | Me | Me | H | O |
| D-14154 | Me | single bond | H | C(Me)(*i*-Pr)CN | Me | Me | H | O |
| D-14155 | Me(R) | single bond | H | C(Me)(*i*-Pr)CN | Me | Me | H | O |
| D-14156 | Me(S) | single bond | H | C(Me)(*i*-Pr)CN | Me | Me | H | O |
| D-14157 | H | single bond | Me | OMe | Me | Cl | H | O |
| D-14158 | Me | single bond | H | OMe | Me | Cl | H | O |
| D-14159 | Me(R) | single bond | H | OMe | Me | Cl | H | O |
| D-14160 | Me(S) | single bond | H | OMe | Me | Cl | H | O |
| D-14161 | H | single bond | Me | OMe | Me | Me | H | O |
| D-14162 | Me | single bond | H | OMe | Me | Me | H | O |
| D-14163 | Me(R) | single bond | H | OMe | Me | Me | H | O |
| D-14164 | Me(S) | single bond | H | OMe | Me | Me | H | O |
| D-14165 | H | single bond | Me | CH=NOMe | Me | Cl | H | O |
| D-14166 | Me | single bond | H | CH=NOMe | Me | Cl | H | O |
| D-14167 | Me(R) | single bond | H | CH=NOMe | Me | Cl | H | O |
| D-14168 | Me(S) | single bond | H | CH=NOMe | Me | Cl | H | O |
| D-14169 | H | single bond | Me | CH=NOMe | Me | Me | H | O |
| D-14170 | Me | single bond | H | CH=NOMe | Me | Me | H | O |
| D-14171 | Me(R) | single bond | H | CH=NOMe | Me | Me | H | O |
| D-14172 | Me(S) | single bond | H | CH=NOMe | Me | Me | H | O |
| D-14173 | H | single bond | Me | morpholin-4-yl | Me | Cl | H | O |
| D-14174 | Me | single bond | H | morpholin-4-yl | Me | Cl | H | O |
| D-14175 | Me(R) | single bond | H | morpholin-4-yl | Me | Cl | H | O |
| D-14176 | Me(S) | single bond | H | morpholin-4-yl | Me | Cl | H | O |
| D-14177 | H | single bond | Me | morpholin-4-yl | Me | Me | H | O |
| D-14178 | Me | single bond | H | morpholin-4-yl | Me | Me | H | O |
| D-14179 | Me(R) | single bond | H | morpholin-4-yl | Me | Me | H | O |
| D-14180 | Me(S) | single bond | H | morpholin-4-yl | Me | Me | H | O |
| D-14181 | H | single bond | Me | Ph | Me | Cl | H | O |
| D-14182 | Me | single bond | H | Ph | Me | Cl | H | O |
| D-14183 | Me(R) | single bond | H | Ph | Me | Cl | H | O |
| D-14184 | Me(S) | single bond | H | Ph | Me | Cl | H | O |
| D-14185 | H | single bond | Me | Ph | Me | Me | H | O |
| D-14186 | Me | single bond | H | Ph | Me | Me | H | O |

**[Table 969]**

| Compound Number | Y | W | Z | R¹ | R² | R³ | R¹⁰ | A |
|---|---|---|---|---|---|---|---|---|
| D-14187 | Me(R) | single bond | H | Ph | Me | Me | H | O |
| D-14188 | Me(S) | single bond | H | Ph | Me | Me | H | O |
| D-14189 | H | single bond | Me | CH₂Ph | Me | Cl | H | O |
| D-14190 | Me | single bond | H | CH₂Ph | Me | Cl | H | O |
| D-14191 | Me(R) | single bond | H | CH₂Ph | Me | Cl | H | O |
| D-14192 | Me(S) | single bond | H | CH₂Ph | Me | Cl | H | O |
| D-14193 | H | single bond | Me | CH₂Ph | Me | Me | H | O |
| D-14194 | Me | single bond | H | CH₂Ph | Me | Me | H | O |
| D-14195 | Me(R) | single bond | H | CH₂Ph | Me | Me | H | O |
| D-14196 | Me(S) | single bond | H | CH₂Ph | Me | Me | H | O |
| D-14197 | H | single bond | Me | thiazole-2-yl | Me | Cl | H | O |
| D-14198 | Me | single bond | H | thiazole-2-yl | Me | Cl | H | O |
| D-14199 | Me(R) | single bond | H | thiazole-2-yl | Me | Cl | H | O |
| D-14200 | Me(S) | single bond | H | thiazole-2-yl | Me | Cl | H | O |
| D-14201 | H | single bond | Me | thiazole-2-yl | Me | Me | H | O |
| D-14202 | Me | single bond | H | thiazole-2-yl | Me | Me | H | O |
| D-14203 | Me(R) | single bond | H | thiazole-2-yl | Me | Me | H | O |
| D-14204 | Me(S) | single bond | H | thiazole-2-yl | Me | Me | H | O |
| D-14205 | H | single bond | Me | 1-Me-pyrazol-3-yl | Me | Cl | H | O |
| D-14206 | Me | single bond | H | 1-Me-pyrazol-3-yl | Me | Cl | H | O |
| D-14207 | Me(R) | single bond | H | 1-Me-pyrazol-3-yl | Me | Cl | H | O |
| D-14208 | Me(S) | single bond | H | 1-Me-pyrazol-3-yl | Me | Cl | H | O |
| D-14209 | H | single bond | Me | 1-Me-pyrazol-3-yl | Me | Me | H | O |
| D-14210 | Me | single bond | H | 1-Me-pyrazol-3-yl | Me | Me | H | O |
| D-14211 | Me(R) | single bond | H | 1-Me-pyrazol-3-yl | Me | Me | H | O |
| D-14212 | Me(S) | single bond | H | 1-Me-pyrazol-3-yl | Me | Me | H | O |
| D-14213 | H | single bond | Me | 1-Me-pyrazol-5-yl | Me | Cl | H | O |
| D-14214 | Me | single bond | H | 1-Me-pyrazol-5-yl | Me | Cl | H | O |
| D-14215 | Me(R) | single bond | H | 1-Me-pyrazol-5-yl | Me | Cl | H | O |
| D-14216 | Me(S) | single bond | H | 1-Me-pyrazol-5-yl | Me | Cl | H | O |
| D-14217 | H | single bond | Me | 1-Me-pyrazol-5-yl | Me | Me | H | O |
| D-14218 | Me | single bond | H | 1-Me-pyrazol-5-yl | Me | Me | H | O |
| D-14219 | Me(R) | single bond | H | 1-Me-pyrazol-5-yl | Me | Me | H | O |
| D-14220 | Me(S) | single bond | H | 1-Me-pyrazol-5-yl | Me | Me | H | O |
| D-14221 | H | single bond | Me | CH₂(pyrimidin-2-yl) | Me | Cl | H | O |
| D-14222 | Me | single bond | H | CH₂(pyrimidin-2-yl) | Me | Cl | H | O |
| D-14223 | Me(R) | single bond | H | CH₂(pyrimidin-2-yl) | Me | Cl | H | O |
| D-14224 | Me(S) | single bond | H | CH₂(pyrimidin-2-yl) | Me | Cl | H | O |
| D-14225 | H | single bond | Me | CH₂(pyrimidin-2-yl) | Me | Me | H | O |
| D-14226 | Me | single bond | H | CH₂(pyrimidin-2-yl) | Me | Me | H | O |
| D-14227 | Me(R) | single bond | H | CH₂(pyrimidin-2-yl) | Me | Me | H | O |
| D-14228 | Me(S) | single bond | H | CH₂(pyrimidin-2-yl) | Me | Me | H | O |
| D-14229 | H | single bond | Me | Me | Me | Cl | Me | O |
| D-14230 | Me | single bond | H | Me | Me | Cl | Me | O |

**[Table 970]**

| Compound Number | Y | W | Z | R¹ | R² | R³ | R¹⁰ | A |
|---|---|---|---|---|---|---|---|---|
| D-14231 | Me(R) | single bond | H | Me | Me | Cl | Me | O |
| D-14232 | Me(S) | single bond | H | Me | Me | Cl | Me | O |
| D-14233 | H | single bond | Me | Me | Me | Me | Me | O |
| D-14234 | Me | single bond | H | Me | Me | Me | Me | O |
| D-14235 | Me(R) | single bond | H | Me | Me | Me | Me | O |
| D-14236 | Me(S) | single bond | H | Me | Me | Me | Me | O |
| D-14237 | H | single bond | Me | CH₂CHF₂ | Me | Cl | Me | O |
| D-14238 | Me | single bond | H | CH₂CHF₂ | Me | Cl | Me | O |
| D-14239 | Me(R) | single bond | H | CH₂CHF₂ | Me | Cl | Me | O |
| D-14240 | Me(S) | single bond | H | CH₂CHF₂ | Me | Cl | Me | O |
| D-14241 | H | single bond | Me | CH₂CHF₂ | Me | Me | Me | O |
| D-14242 | Me | single bond | H | CH₂CHF₂ | Me | Me | Me | O |
| D-14243 | Me(R) | single bond | H | CH₂CHF₂ | Me | Me | Me | O |
| D-14244 | Me(S) | single bond | H | CH₂CHF₂ | Me | Me | Me | O |
| D-14245 | H | single bond | Me | *n*-Pr | Me | Cl | Me | O |
| D-14246 | Me | single bond | H | *n*-Pr | Me | Cl | Me | O |
| D-14247 | Me(R) | single bond | H | *n*-Pr | Me | Cl | Me | O |
| D-14248 | Me(S) | single bond | H | *n*-Pr | Me | Cl | Me | O |
| D-14249 | H | single bond | Me | *n*-Pr | Me | Me | Me | O |
| D-14250 | Me | single bond | H | *n*-Pr | Me | Me | Me | O |
| D-14251 | Me(R) | single bond | H | *n*-Pr | Me | Me | Me | O |
| D-14252 | Me(S) | single bond | H | *n*-Pr | Me | Me | Me | O |
| D-14253 | H | single bond | Me | *i*-Pr | Me | Cl | Me | O |
| D-14254 | Me | single bond | H | *i*-Pr | Me | Cl | Me | O |
| D-14255 | Me(R) | single bond | H | *i*-Pr | Me | Cl | Me | O |
| D-14256 | Me(S) | single bond | H | *i*-Pr | Me | Cl | Me | O |
| D-14257 | H | single bond | Me | *i*-Pr | Me | Me | Me | O |
| D-14258 | Me | single bond | H | *i*-Pr | Me | Me | Me | O |
| D-14259 | Me(R) | single bond | H | *i*-Pr | Me | Me | Me | O |
| D-14260 | Me(S) | single bond | H | *i*-Pr | Me | Me | Me | O |
| D-14261 | H | single bond | Me | *c*-Pr(1-F) | Me | Cl | Me | O |
| D-14262 | Me | single bond | H | *c*-Pr(1-F) | Me | Cl | Me | O |
| D-14263 | Me(R) | single bond | H | *c*-Pr(1-F) | Me | Cl | Me | O |
| D-14264 | Me(S) | single bond | H | *c*-Pr(1-F) | Me | Cl | Me | O |
| D-14265 | H | single bond | Me | *c*-Pr(1-F) | Me | Me | Me | O |
| D-14266 | Me | single bond | H | *c*-Pr(1-F) | Me | Me | Me | O |
| D-14267 | Me(R) | single bond | H | *c*-Pr(1-F) | Me | Me | Me | O |
| D-14268 | Me(S) | single bond | H | *c*-Pr(1-F) | Me | Me | Me | O |
| D-14269 | H | single bond | Me | *c*-Pr(2,2-F₂) | Me | Cl | Me | O |
| D-14270 | Me | single bond | H | *c*-Pr(2,2-F₂) | Me | Cl | Me | O |
| D-14271 | Me(R) | single bond | H | *c*-Pr(2,2-F₂) | Me | Cl | Me | O |
| D-14272 | Me(S) | single bond | H | *c*-Pr(2,2-F₂) | Me | Cl | Me | O |
| D-14273 | H | single bond | Me | *c*-Pr(2,2-F₂) | Me | Me | Me | O |
| D-14274 | Me | single bond | H | *c*-Pr(2,2-F₂) | Me | Me | Me | O |

**[Table 971]**

| Compound Number | Y | W | Z | R¹ | R² | R³ | R¹⁰ | A |
|---|---|---|---|---|---|---|---|---|
| D-14275 | Me(R) | single bond | H | *c*-Pr(2,2-F₂) | Me | Me | Me | O |
| D-14276 | Me(S) | single bond | H | *c*-Pr(2,2-F₂) | Me | Me | Me | O |
| D-14277 | H | single bond | Me | *c*-Pr(1-Cl) | Me | Cl | Me | O |
| D-14278 | Me | single bond | H | *c*-Pr(1-Cl) | Me | Cl | Me | O |
| D-14279 | Me(R) | single bond | H | *c*-Pr(1-Cl) | Me | Cl | Me | O |
| D-14280 | Me(S) | single bond | H | *c*-Pr(1-Cl) | Me | Cl | Me | O |
| D-14281 | H | single bond | Me | *c*-Pr(1-Cl) | Me | Me | Me | O |
| D-14282 | Me | single bond | H | *c*-Pr(1-Cl) | Me | Me | Me | O |
| D-14283 | Me(R) | single bond | H | *c*-Pr(1-Cl) | Me | Me | Me | O |
| D-14284 | Me(S) | single bond | H | *c*-Pr(1-Cl) | Me | Me | Me | O |
| D-14285 | H | single bond | Me | *c*-Pr(1-Br) | Me | Cl | Me | O |
| D-14286 | Me | single bond | H | *c*-Pr(1-Br) | Me | Cl | Me | O |
| D-14287 | Me(R) | single bond | H | *c*-Pr(1-Br) | Me | Cl | Me | O |
| D-14288 | Me(S) | single bond | H | *c*-Pr(1-Br) | Me | Cl | Me | O |
| D-14289 | H | single bond | Me | *c*-Pr(1-Br) | Me | Me | Me | O |
| D-14290 | Me | single bond | H | *c*-Pr(1-Br) | Me | Me | Me | O |
| D-14291 | Me(R) | single bond | H | *c*-Pr(1-Br) | Me | Me | Me | O |
| D-14292 | Me(S) | single bond | H | *c*-Pr(1-Br) | Me | Me | Me | O |
| D-14293 | H | single bond | Me | *c*-Pr(1-Me) | Me | Cl | Me | O |
| D-14294 | Me | single bond | H | *c*-Pr(1-Me) | Me | Cl | Me | O |
| D-14295 | Me(R) | single bond | H | *c*-Pr(1-Me) | Me | Cl | Me | O |
| D-14296 | Me(S) | single bond | H | *c*-Pr(1-Me) | Me | Cl | Me | O |
| D-14297 | H | single bond | Me | *c*-Pr(1-Me) | Me | Me | Me | O |
| D-14298 | Me | single bond | H | *c*-Pr(1-Me) | Me | Me | Me | O |
| D-14299 | Me(R) | single bond | H | *c*-Pr(1-Me) | Me | Me | Me | O |
| D-14300 | Me(S) | single bond | H | *c*-Pr(1-Me) | Me | Me | Me | O |
| D-14301 | H | single bond | Me | *c*-Pr(1-CH₂OH) | Me | Cl | Me | O |
| D-14302 | Me | single bond | H | *c*-Pr(1-CH₂OH) | Me | Cl | Me | O |
| D-14303 | Me(R) | single bond | H | *c*-Pr(1-CH₂OH) | Me | Cl | Me | O |
| D-14304 | Me(S) | single bond | H | *c*-Pr(1-CH₂OH) | Me | Cl | Me | O |
| D-14305 | H | single bond | Me | *c*-Pr(1-CH₂OH) | Me | Me | Me | O |
| D-14306 | Me | single bond | H | *c*-Pr(1-CH₂OH) | Me | Me | Me | O |
| D-14307 | Me(R) | single bond | H | *c*-Pr(1-CH₂OH) | Me | Me | Me | O |
| D-14308 | Me(S) | single bond | H | *c*-Pr(1-CH₂OH) | Me | Me | Me | O |
| D-14309 | H | single bond | Me | *c*-Pr[1-CH₂OS(=O)₂Me] | Me | Cl | Me | O |
| D-14310 | Me | single bond | H | *c*-Pr[1-CH₂OS(=O)₂Me] | Me | Cl | Me | O |
| D-14311 | Me(R) | single bond | H | *c*-Pr[1-CH₂OS(=O)₂Me] | Me | Cl | Me | O |
| D-14312 | Me(S) | single bond | H | *c*-Pr[1-CH₂OS(=O)₂Me] | Me | Cl | Me | O |
| D-14313 | H | single bond | Me | *c*-Pr[1-CH₂OS(=O)₂Me] | Me | Me | Me | O |
| D-14314 | Me | single bond | H | *c*-Pr[1-CH₂OS(=O)₂Me] | Me | Me | Me | O |
| D-14315 | Me(R) | single bond | H | *c*-Pr[1-CH₂OS(=O)₂Me] | Me | Me | Me | O |
| D-14316 | Me(S) | single bond | H | *c*-Pr[1-CH₂OS(=O)₂Me] | Me | Me | Me | O |
| D-14317 | H | single bond | Me | *c*-Pr(1-CH₂OMe) | Me | Cl | Me | O |
| D-14318 | Me | single bond | H | *c*-Pr(1-CH₂OMe) | Me | Cl | Me | O |

**[Table 972]**

| Compound Number | Y | W | Z | R¹ | R² | R³ | R¹⁰ | A |
|---|---|---|---|---|---|---|---|---|
| D-14319 | Me(R) | single bond | H | *c*-Pr(1-CH₂OMe) | Me | Cl | Me | O |
| D-14320 | Me(S) | single bond | H | *c*-Pr(1-CH₂OMe) | Me | Cl | Me | O |
| D-14321 | H | single bond | Me | *c*-Pr(1-CH₂OMe) | Me | Me | Me | O |
| D-14322 | Me | single bond | H | *c*-Pr(1-CH₂OMe) | Me | Me | Me | O |
| D-14323 | Me(R) | single bond | H | *c*-Pr(1-CH₂OMe) | Me | Me | Me | O |
| D-14324 | Me(S) | single bond | H | *c*-Pr(1-CH₂OMe) | Me | Me | Me | O |
| D-14325 | H | single bond | Me | *c*-Pr(1-CH₂OCN) | Me | Cl | Me | O |
| D-14326 | Me | single bond | H | *c*-Pr(1-CH₂OCN) | Me | Cl | Me | O |
| D-14327 | Me(R) | single bond | H | *c*-Pr(1-CH₂OCN) | Me | Cl | Me | O |
| D-14328 | Me(S) | single bond | H | *c*-Pr(1-CH₂OCN) | Me | Cl | Me | O |
| D-14329 | H | single bond | Me | *c*-Pr(1-CH₂OCN) | Me | Me | Me | O |
| D-14330 | Me | single bond | H | *c*-Pr(1-CH₂OCN) | Me | Me | Me | O |
| D-14331 | Me(R) | single bond | H | *c*-Pr(1-CH₂OCN) | Me | Me | Me | O |
| D-14332 | Me(S) | single bond | H | *c*-Pr(1-CH₂OCN) | Me | Me | Me | O |
| D-14333 | H | single bond | Me | *c*-Pr(1-CH₂SMe) | Me | Cl | Me | O |
| D-14334 | Me | single bond | H | *c*-Pr(1-CH₂SMe) | Me | Cl | Me | O |
| D-14335 | Me(R) | single bond | H | *c*-Pr(1-CH₂SMe) | Me | Cl | Me | O |
| D-14336 | Me(S) | single bond | H | *c*-Pr(1-CH₂SMe) | Me | Cl | Me | O |
| D-14337 | H | single bond | Me | *c*-Pr(1-CH₂SMe) | Me | Me | Me | O |
| D-14338 | Me | single bond | H | *c*-Pr(1-CH₂SMe) | Me | Me | Me | O |
| D-14339 | Me(R) | single bond | H | *c*-Pr(1-CH₂SMe) | Me | Me | Me | O |
| D-14340 | Me(S) | single bond | H | *c*-Pr(1-CH₂SMe) | Me | Me | Me | O |
| D-14341 | H | single bond | Me | *c*-Pr[1-CH₂S(=O)Me] | Me | Cl | Me | O |
| D-14342 | Me | single bond | H | *c*-Pr[1-CH₂S(=O)Me] | Me | Cl | Me | O |
| D-14343 | Me(R) | single bond | H | *c*-Pr[1-CH₂S(=O)Me] | Me | Cl | Me | O |
| D-14344 | Me(S) | single bond | H | *c*-Pr[1-CH₂S(=O)Me] | Me | Cl | Me | O |
| D-14345 | H | single bond | Me | *c*-Pr[1-CH₂S(=O)Me] | Me | Me | Me | O |
| D-14346 | Me | single bond | H | *c*-Pr[1-CH₂S(=O)Me] | Me | Me | Me | O |
| D-14347 | Me(R) | single bond | H | *c*-Pr[1-CH₂S(=O)Me] | Me | Me | Me | O |
| D-14348 | Me(S) | single bond | H | *c*-Pr[1-CH₂S(=O)Me] | Me | Me | Me | O |
| D-14349 | H | single bond | Me | *c*-Pr[1-CH₂S(=O)₂Me] | Me | Cl | Me | O |
| D-14350 | Me | single bond | H | *c*-Pr[1-CH₂S(=O)₂Me] | Me | Cl | Me | O |
| D-14351 | Me(R) | single bond | H | *c*-Pr[1-CH₂S(=O)₂Me] | Me | Cl | Me | O |
| D-14352 | Me(S) | single bond | H | *c*-Pr[1-CH₂S(=O)₂Me] | Me | Cl | Me | O |
| D-14353 | H | single bond | Me | *c*-Pr[1-CH₂S(=O)₂Me] | Me | Me | Me | O |
| D-14354 | Me | single bond | H | *c*-Pr[1-CH₂S(=O)₂Me] | Me | Me | Me | O |
| D-14355 | Me(R) | single bond | H | *c*-Pr[1-CH₂S(=O)₂Me] | Me | Me | Me | O |
| D-14356 | Me(S) | single bond | H | *c*-Pr[1-CH₂S(=O)₂Me] | Me | Me | Me | O |
| D-14357 | H | single bond | Me | *c*-Pr(1-CH₂F) | Me | Cl | Me | O |
| D-14358 | Me | single bond | H | *c*-Pr(1-CH₂F) | Me | Cl | Me | O |
| D-14359 | Me(R) | single bond | H | *c*-Pr(1-CH₂F) | Me | Cl | Me | O |
| D-14360 | Me(S) | single bond | H | *c*-Pr(1-CH₂F) | Me | Cl | Me | O |
| D-14361 | H | single bond | Me | *c*-Pr(1-CH₂F) | Me | Me | Me | O |
| D-14362 | Me | single bond | H | *c*-Pr(1-CH₂F) | Me | Me | Me | O |

**[Table 973]**

| Compound Number | Y | W | Z | R¹ | R² | R³ | R¹⁰ | A |
|---|---|---|---|---|---|---|---|---|
| D-14363 | Me(R) | single bond | H | *c*-Pr(1-CH₂F) | Me | Me | Me | O |
| D-14364 | Me(S) | single bond | H | *c*-Pr(1-CH₂F) | Me | Me | Me | O |
| D-14365 | H | single bond | Me | *c*-Pr(1-CHF₂) | Me | Cl | Me | O |
| D-14366 | Me | single bond | H | *c*-Pr(1-CHF₂) | Me | Cl | Me | O |
| D-14367 | Me(R) | single bond | H | *c*-Pr(1-CHF₂) | Me | Cl | Me | O |
| D-14368 | Me(S) | single bond | H | *c*-Pr(1-CHF₂) | Me | Cl | Me | O |
| D-14369 | H | single bond | Me | *c*-Pr(1-CHF₂) | Me | Me | Me | O |
| D-14370 | Me | single bond | H | *c*-Pr(1-CHF₂) | Me | Me | Me | O |
| D-14371 | Me(R) | single bond | H | *c*-Pr(1-CHF₂) | Me | Me | Me | O |
| D-14372 | Me(S) | single bond | H | *c*-Pr(1-CHF₂) | Me | Me | Me | O |
| D-14373 | H | single bond | Me | *c*-Pr(1-CF₃) | Me | Cl | Me | O |
| D-14374 | Me | single bond | H | *c*-Pr(1-CF₃) | Me | Cl | Me | O |
| D-14375 | Me(R) | single bond | H | *c*-Pr(1-CF₃) | Me | Cl | Me | O |
| D-14376 | Me(S) | single bond | H | *c*-Pr(1-CF₃) | Me | Cl | Me | O |
| D-14377 | H | single bond | Me | *c*-Pr(1-CF₃) | Me | Me | Me | O |
| D-14378 | Me | single bond | H | *c*-Pr(1-CF₃) | Me | Me | Me | O |
| D-14379 | Me(R) | single bond | H | *c*-Pr(1-CF₃) | Me | Me | Me | O |
| D-14380 | Me(S) | single bond | H | *c*-Pr(1-CF₃) | Me | Me | Me | O |
| D-14381 | H | single bond | Me | *c*-pr(1-C≡CH) | Me | Cl | Me | O |
| D-14382 | Me | single bond | H | *c*-pr(1-C≡CH) | Me | Cl | Me | O |
| D-14383 | Me(R) | single bond | H | *c*-pr(1-C≡CH) | Me | Cl | Me | O |
| D-14384 | Me(S) | single bond | H | *c*-pr(1-C≡CH) | Me | Cl | Me | O |
| D-14385 | H | single bond | Me | *c*-pr(1-C≡CH) | Me | Me | Me | O |
| D-14386 | Me | single bond | H | *c*-pr(1-C≡CH) | Me | Me | Me | O |
| D-14387 | Me(R) | single bond | H | *c*-pr(1-C≡CH) | Me | Me | Me | O |
| D-14388 | Me(S) | single bond | H | *c*-pr(1-C≡CH) | Me | Me | Me | O |
| D-14389 | H | single bond | Me | *c*-Pr[1-C(=O)OEt] | Me | Cl | Me | O |
| D-14390 | Me | single bond | H | *c*-Pr[1-C(=O)OEt] | Me | Cl | Me | O |
| D-14391 | Me(R) | single bond | H | *c*-Pr[1-C(=O)OEt] | Me | Cl | Me | O |
| D-14392 | Me(S) | single bond | H | *c*-Pr[1-C(=O)OEt] | Me | Cl | Me | O |
| D-14393 | H | single bond | Me | *c*-Pr[1-C(=O)OEt] | Me | Me | Me | O |
| D-14394 | Me | single bond | H | *c*-Pr[1-C(=O)OEt] | Me | Me | Me | O |
| D-14395 | Me(R) | single bond | H | *c*-Pr[1-C(=O)OEt] | Me | Me | Me | O |
| D-14396 | Me(S) | single bond | H | *c*-Pr[1-C(=O)OEt] | Me | Me | Me | O |
| D-14397 | H | single bond | Me | *c*-Pr[1-C(=O)OH] | Me | Cl | Me | O |
| D-14398 | Me | single bond | H | *c*-Pr[1-C(=O)OH] | Me | Cl | Me | O |
| D-14399 | Me(R) | single bond | H | *c*-Pr[1-C(=O)OH] | Me | Cl | Me | O |
| D-14400 | Me(S) | single bond | H | *c*-Pr[1-C(=O)OH] | Me | Cl | Me | O |
| D-14401 | H | single bond | Me | *c*-Pr[1-C(=O)OH] | Me | Me | Me | O |
| D-14402 | Me | single bond | H | *c*-Pr[1-C(=O)OH] | Me | Me | Me | O |
| D-14403 | Me(R) | single bond | H | *c*-Pr[1-C(=O)OH] | Me | Me | Me | O |
| D-14404 | Me(S) | single bond | H | *c*-Pr[1-C(=O)OH] | Me | Me | Me | O |
| D-14405 | H | single bond | Me | *c*-Pr[1-C(=O)NH₂] | Me | Cl | Me | O |
| D-14406 | Me | single bond | H | *c*-Pr[1-C(=O)NH₂] | Me | Cl | Me | O |

**[Table 974]**

| Compound Number | Y | W | Z | R¹ | R² | R³ | R¹⁰ | A |
|---|---|---|---|---|---|---|---|---|
| D-14407 | Me(R) | single bond | H | *c*-Pr[1-C(=O)NH₂] | Me | Cl | Me | O |
| D-14408 | Me(S) | single bond | H | *c*-Pr[1-C(=O)NH₂] | Me | Cl | Me | O |
| D-14409 | H | single bond | Me | *c*-Pr[1-C(=O)NH₂] | Me | Me | Me | O |
| D-14410 | Me | single bond | H | *c*-Pr[1-C(=O)NH₂] | Me | Me | Me | O |
| D-14411 | Me(R) | single bond | H | *c*-Pr[1-C(=O)NH₂] | Me | Me | Me | O |
| D-14412 | Me(S) | single bond | H | *c*-Pr[1-C(=O)NH₂] | Me | Me | Me | O |
| D-14413 | H | single bond | Me | *c*-Pr(1-OMe) | Me | Cl | Me | O |
| D-14414 | Me | single bond | H | *c-*Pr(1-OMe) | Me | Cl | Me | O |
| D-14415 | Me(R) | single bond | H | *c*-Pr(1-OMe) | Me | Cl | Me | O |
| D-14416 | Me(S) | single bond | H | *c*-Pr(1-OMe) | Me | Cl | Me | O |
| D-14417 | H | single bond | Me | *c*-Pr(1-OMe) | Me | Me | Me | O |
| D-14418 | Me | single bond | H | *c*-Pr(1-OMe) | Me | Me | Me | O |
| D-14419 | Me(R) | single bond | H | *c*-Pr(1-OMe) | Me | Me | Me | O |
| D-14420 | Me(S) | single bond | H | *c*-Pr(1-OMe) | Me | Me | Me | O |
| D-14421 | H | single bond | Me | *c*-Pr(1-SMe) | Me | Cl | Me | O |
| D-14422 | Me | single bond | H | *c*-Pr(1-SMe) | Me | Cl | Me | O |
| D-14423 | Me(R) | single bond | H | *c*-Pr(1-SMe) | Me | Cl | Me | O |
| D-14424 | Me(S) | single bond | H | *c*-Pr(1-SMe) | Me | Cl | Me | O |
| D-14425 | H | single bond | Me | *c*-Pr(1-SMe) | Me | Me | Me | O |
| D-14426 | Me | single bond | H | *c*-Pr(1-SMe) | Me | Me | Me | O |
| D-14427 | Me(R) | single bond | H | *c*-Pr(1-SMe) | Me | Me | Me | O |
| D-14428 | Me(S) | single bond | H | *c*-Pr(1-SMe) | Me | Me | Me | O |
| D-14429 | H | single bond | Me | *c*-pr[1-S(=O)Me] | Me | Cl | Me | O |
| D-14430 | Me | single bond | H | *c*-pr[1-S(=O)Me] | Me | Cl | Me | O |
| D-14431 | Me(R) | single bond | H | *c*-pr[1-S(=O)Me] | Me | Cl | Me | O |
| D-14432 | Me(S) | single bond | H | *c*-pr[1-S(=O)Me] | Me | Cl | Me | O |
| D-14433 | H | single bond | Me | *c*-pr[1-S(=O)Me] | Me | Me | Me | O |
| D-14434 | Me | single bond | H | *c*-pr[1-S(=O)Me] | Me | Me | Me | O |
| D-14435 | Me(R) | single bond | H | *c*-pr[1-S(=O)Me] | Me | Me | Me | O |
| D-14436 | Me(S) | single bond | H | *c*-pr[1-S(=O)Me] | Me | Me | Me | O |
| D-14437 | H | single bond | Me | *c*-Pr[1-S(=O)₂Me] | Me | Cl | Me | O |
| D-14438 | Me | single bond | H | *c*-Pr[1-S(=O)₂Me] | Me | Cl | Me | O |
| D-14439 | Me(R) | single bond | H | *c*-Pr[1-S(=O)₂Me] | Me | Cl | Me | O |
| D-14440 | Me(S) | single bond | H | *c*-Pr[1-S(=O)₂Me] | Me | Cl | Me | O |
| D-14441 | H | single bond | Me | *c*-Pr[1-S(=O)₂Me] | Me | Me | Me | O |
| D-14442 | Me | single bond | H | *c*-Pr[1-S(=O)₂Me] | Me | Me | Me | O |
| D-14443 | Me(R) | single bond | H | *c*-Pr[1-S(=O)₂Me] | Me | Me | Me | O |
| D-14444 | Me(S) | single bond | H | *c*-Pr[1-S(=O)₂Me] | Me | Me | Me | O |
| D-14445 | H | single bond | Me | *c*-Pr[1-S(=O)₂CHF₂] | Me | Cl | Me | O |
| D-14446 | Me | single bond | H | *c*-Pr[1-S(=O)₂CHF₂] | Me | Cl | Me | O |
| D-14447 | Me(R) | single bond | H | *c*-Pr[1-S(=O)₂CHF₂] | Me | Cl | Me | O |
| D-14448 | Me(S) | single bond | H | *c*-Pr[1-S(=O)₂CHF₂] | Me | Cl | Me | O |
| D-14449 | H | single bond | Me | *c*-Pr[1-S(=O)₂CHF₂] | Me | Me | Me | O |
| D-14450 | Me | single bond | H | *c*-Pr[1-S(=O)₂CHF₂] | Me | Me | Me | O |

**[Table 975]**

| Compound Number | Y | W | Z | R¹ | R² | R³ | R¹⁰ | A |
|---|---|---|---|---|---|---|---|---|
| D-14451 | Me(R) | single bond | H | *c*-Pr[1-S(=O)₂CHF₂] | Me | Me | Me | O |
| D-14452 | Me(S) | single bond | H | *c*-Pr[1-S(=O)₂CHF₂] | Me | Me | Me | O |
| D-14453 | H | single bond | Me | *c*-Pr[1-S(=O)₂CF₃] | Me | Cl | Me | O |
| D-14454 | Me | single bond | H | *c*-Pr[1-S(=O)₂CF₃] | Me | Cl | Me | O |
| D-14455 | Me(R) | single bond | H | *c*-Pr[1-S(=O)₂CF₃] | Me | Cl | Me | O |
| D-14456 | Me(S) | single bond | H | *c*-Pr[1-S(=O)₂CF₃] | Me | Cl | Me | O |
| D-14457 | H | single bond | Me | *c*-Pr[1-S(=O)₂CF₃] | Me | Me | Me | O |
| D-14458 | Me | single bond | H | *c*-Pr[1-S(=O)₂CF₃] | Me | Me | Me | O |
| D-14459 | Me(R) | single bond | H | *c*-Pr[1-S(=O)₂CF₃] | Me | Me | Me | O |
| D-14460 | Me(S) | single bond | H | *c*-Pr[1-S(=O)₂CF₃] | Me | Me | Me | O |
| D-14461 | H | single bond | Me | *c*-Pr[1-(5,6-dihydro-1,4,2-dioxazin-3-yl)] | Me | Cl | Me | O |
| D-14462 | Me | single bond | H | *c*-Pr[1-(5,6-dihydro-1,4,2-dioxazin-3-yl)] | Me | Cl | Me | O |
| D-14463 | Me(R) | single bond | H | *c*-Pr[1-(5,6-dihydro-1,4,2-dioxazin-3-yl)] | Me | Cl | Me | O |
| D-14464 | Me(S) | single bond | H | *c*-Pr[1-(5,6-dihydro-1,4,2-dioxazin-3-yl)] | Me | Cl | Me | O |
| D-14465 | H | single bond | Me | *c*-Pr[1-(5,6-dihydro-1,4,2-dioxazin-3-yl)] | Me | Me | Me | O |
| D-14466 | Me | single bond | H | *c*-Pr[1-(5,6-dihydro-1,4,2-dioxazin-3-yl)] | Me | Me | Me | O |
| D-14467 | Me(R) | single bond | H | *c*-Pr[1-(5,6-dihydro-1,4,2-dioxazin-3-yl)] | Me | Me | Me | O |
| D-1 4468 | Me(S) | single bond | H | *c*-Pr[1-(5,6-dihydro-1,4,2-dioxazin-3-yl)] | Me | Me | Me | O |
| D-14469 | H | single bond | Me | *c*-Pr[1-(5-hydroxy-1,3,4-oxadiazol-2-yl)] | Me | Cl | Me | O |
| D-14470 | Me | single bond | H | *c*-Pr[1-(5-hydroxy-1,3,4-oxadiazol-2-yl)] | Me | Cl | Me | O |
| D-14471 | Me(R) | single bond | H | *c*-Pr[1-(5-hydroxy-1,3,4-oxadiazol-2-yl)] | Me | Cl | Me | O |
| D-14472 | Me(S) | single bond | H | *c*-Pr[1-(5-hydroxy-1,3,4-oxadiazol-2-yl)] | Me | Cl | Me | O |
| D-14473 | H | single bond | Me | *c*-Pr[1-(5-hydroxy-1,3,4-oxadiazol-2-yl)] | Me | Me | Me | O |
| D-14474 | Me | single bond | H | *c*-Pr[1-(5-hydroxy-1,3,4-oxadiazol-2-yl)] | Me | Me | Me | O |
| D-14475 | Me(R) | single bond | H | *c*-Pr[1-(5-hydroxy-1,3,4-oxadiazol-2-yl)] | Me | Me | Me | O |
| D-14476 | Me(S) | single bond | H | *c*-Pr[1-(5-hydroxy-1,3,4-oxadiazol-2-yl)] | Me | Me | Me | O |
| D-14477 | H | single bond | Me | *c*-Pr[1-(pyrimidin-2-yl)] | Me | Cl | Me | O |
| D-14478 | Me | single bond | H | *c*-Pr[1-(pyrimidin-2-yl)] | Me | Cl | Me | O |
| D-14479 | Me(R) | single bond | H | *c*-Pr[1-(pyrimidin-2-yl)] | Me | Cl | Me | O |
| D-14480 | Me(S) | single bond | H | *c*-Pr[1-(pyrimidin-2-yl)] | Me | Cl | Me | O |
| D-14481 | H | single bond | Me | *c*-Pr[1-(pyrimidin-2-yl)] | Me | Me | Me | O |
| D-14482 | Me | single bond | H | *c*-Pr[1-(pyrimidin-2-yl)] | Me | Me | Me | O |
| D-14483 | Me(R) | single bond | H | *c*-Pr[1-(pyrimidin-2-yl)] | Me | Me | Me | O |
| D-14484 | Me(S) | single bond | H | *c*-Pr[1-(pyrimidin-2-yl)] | Me | Me | Me | O |
| D-14485 | H | single bond | Me | *t*-Bu | Me | Cl | Me | O |
| D-14486 | Me | single bond | H | *t*-Bu | Me | Cl | Me | O |
| D-14487 | Me(R) | single bond | H | *t*-Bu | Me | Cl | Me | O |
| D-14488 | Me(S) | single bond | H | *t*-Bu | Me | Cl | Me | O |
| D-14489 | H | single bond | Me | *t*-Bu | Me | Me | Me | O |
| D-14490 | Me | single bond | H | *t*-Bu | Me | Me | Me | O |
| D-14491 | Me(R) | single bond | H | *t*-Bu | Me | Me | Me | O |
| D-14492 | Me(S) | single bond | H | *t*-Bu | Me | Me | Me | O |
| D-14493 | H | single bond | Me | *c*-Bu | Me | Cl | Me | O |
| D-14494 | Me | single bond | H | *c*-Bu | Me | Cl | Me | O |

**[Table 976]**

| Compound Number | Y | W | Z | R¹ | R² | R³ | R¹⁰ | A |
|---|---|---|---|---|---|---|---|---|
| D-14495 | Me(R) | single bond | H | *c*-Bu | Me | Cl | Me | O |
| D-14496 | Me(S) | single bond | H | *c*-Bu | Me | Cl | Me | O |
| D-14497 | H | single bond | Me | *c*-Bu | Me | Me | Me | O |
| D-14498 | Me | single bond | H | *c*-Bu | Me | Me | Me | O |
| D-14499 | Me(R) | single bond | H | *c*-Bu | Me | Me | Me | O |
| D-14500 | Me(S) | single bond | H | *c*-Bu | Me | Me | Me | O |
| D-14501 | H | single bond | Me | c-Bu(1-CN) | Me | Cl | Me | O |
| D-14502 | Me | single bond | H | c-Bu(1-CN) | Me | Cl | Me | O |
| D-14503 | Me(R) | single bond | H | c-Bu(1-CN) | Me | Cl | Me | O |
| D-14504 | Me(S) | single bond | H | c-Bu(1-CN) | Me | Cl | Me | O |
| D-14505 | H | single bond | Me | c-Bu(1-CN) | Me | Me | Me | O |
| D-14506 | Me | single bond | H | c-Bu(1-CN) | Me | Me | Me | O |
| D-14507 | Me(R) | single bond | H | c-Bu(1-CN) | Me | Me | Me | O |
| D-14508 | Me(S) | single bond | H | c-Bu(1-CN) | Me | Me | Me | O |
| D-14509 | H | single bond | Me | *c*-Bu(3,3-F₂) | Me | Cl | Me | O |
| D-14510 | Me | single bond | H | *c*-Bu(3,3-F₂) | Me | Cl | Me | O |
| D-14511 | Me(R) | single bond | H | *c*-Bu(3,3-F₂) | Me | Cl | Me | O |
| D-14512 | Me(S) | single bond | H | *c*-Bu(3,3-F₂) | Me | Cl | Me | O |
| D-14513 | H | single bond | Me | *c*-Bu(3,3-F₂) | Me | Me | Me | O |
| D-14514 | Me | single bond | H | *c*-Bu(3,3-F₂) | Me | Me | Me | O |
| D-14515 | Me(R) | single bond | H | *c*-Bu(3,3-F₂) | Me | Me | Me | O |
| D-14516 | Me(S) | single bond | H | *c*-Bu(3,3-F₂) | Me | Me | Me | O |
| D-14517 | H | single bond | Me | oxetan-3-yl | Me | Cl | Me | O |
| D-14518 | Me | single bond | H | oxetan-3-yl | Me | Cl | Me | O |
| D-14519 | Me(R) | single bond | H | oxetan-3-yl | Me | Cl | Me | O |
| D-14520 | Me(S) | single bond | H | oxetan-3-yl | Me | Cl | Me | O |
| D-14521 | H | single bond | Me | oxetan-3-yl | Me | Me | Me | O |
| D-14522 | Me | single bond | H | oxetan-3-yl | Me | Me | Me | O |
| D-14523 | Me(R) | single bond | H | oxetan-3-yl | Me | Me | Me | O |
| D-14524 | Me(S) | single bond | H | oxetan-3-yl | Me | Me | Me | O |
| D-14525 | H | single bond | Me | oxetan(3-CN)-3-yl | Me | Cl | Me | O |
| D-14526 | Me | single bond | H | oxetan(3-CN)-3-yl | Me | Cl | Me | O |
| D-14527 | Me(R) | single bond | H | oxetan(3-CN)-3-yl | Me | Cl | Me | O |
| D-14528 | Me(S) | single bond | H | oxetan(3-CN)-3-yl | Me | Cl | Me | O |
| D-14529 | H | single bond | Me | oxetan(3-CN)-3-yl | Me | Me | Me | O |
| D-14530 | Me | single bond | H | oxetan(3-CN)-3-yl | Me | Me | Me | O |
| D-14531 | Me(R) | single bond | H | oxetan(3-CN)-3-yl | Me | Me | Me | O |
| D-14532 | Me(S) | single bond | H | oxetan(3-CN)-3-yl | Me | Me | Me | O |
| D-14533 | H | single bond | Me | azetidin[1-C(=O)Me]-3-yl | Me | Cl | Me | O |
| D-14534 | Me | single bond | H | azetidin[1-C(=O)Me]-3-yl | Me | Cl | Me | O |
| D-14535 | Me(R) | single bond | H | azetidin[1-C(=O)Me]-3-yl | Me | Cl | Me | O |
| D-14536 | Me(S) | single bond | H | azetidin[1-C(=O)Me]-3-yl | Me | Cl | Me | O |
| D-14537 | H | single bond | Me | azetidin[1-C(=O)Me]-3-yl | Me | Me | Me | O |
| D-14538 | Me | single bond | H | azetidin[1-C(=O)Me]-3-yl | Me | Me | Me | O |

**[Table 977]**

| Compound Number | Y | W | Z | R¹ | R² | R³ | R¹⁰ | A |
|---|---|---|---|---|---|---|---|---|
| D-14539 | Me(R) | single bond | H | azetidin[1-C(=O)Me]-3-yl | Me | Me | Me | O |
| D-14540 | Me(S) | single bond | H | azetidin[1-C(=O)Me]-3-yl | Me | Me | Me | O |
| D-14541 | H | single bond | Me | azetidin[1-S(=O)₂Me]-3-yl | Me | Cl | Me | O |
| D-14542 | Me | single bond | H | azetidin[1-S(=O)₂Me]-3-yl | Me | Cl | Me | O |
| D-14543 | Me(R) | single bond | H | azetidin[1-S(=O)₂Me]-3-yl | Me | Cl | Me | O |
| D-14544 | Me(S) | single bond | H | azetidin[1-S(=O)₂Me]-3-yl | Me | Cl | Me | O |
| D-14545 | H | single bond | Me | azetidin[1-S(=O)₂Me]-3-yl | Me | Me | Me | O |
| D-14546 | Me | single bond | H | azetidin[1-S(=O)₂Me]-3-yl | Me | Me | Me | O |
| D-14547 | Me(R) | single bond | H | azetidin[1-S(=O)₂Me]-3-yl | Me | Me | Me | O |
| D-14548 | Me(S) | single bond | H | azetidin[1-S(=O)₂Me]-3-yl | Me | Me | Me | O |
| D-14549 | H | single bond | Me | azetidin[1-S(=O)₂CHF₂]-3-yl | Me | Cl | Me | O |
| D-14550 | Me | single bond | H | azetidin[1-S(=O)₂CHF₂]-3-yl | Me | Cl | Me | O |
| D-14551 | Me(R) | single bond | H | azetidin[1-S(=O)₂CHF₂]-3-yl | Me | Cl | Me | O |
| D-14552 | Me(S) | single bond | H | azetidin[1-S(=O)₂CHF₂]-3-yl | Me | Cl | Me | O |
| D-14553 | H | single bond | Me | azetidin[1-S(=O)₂CHF₂]-3-yl | Me | Me | Me | O |
| D-14554 | Me | single bond | H | azetidin[1-S(=O)₂CHF₂]-3-yl | Me | Me | Me | O |
| D-14555 | Me(R) | single bond | H | azetidin[1-S(=O)₂CHF₂]-3-yl | Me | Me | Me | O |
| D-14556 | Me(S) | single bond | H | azetidin[1-S(=O)₂CHF₂]-3-yl | Me | Me | Me | O |
| D-14557 | H | single bond | Me | *c*-Pen | Me | Cl | Me | O |
| D-14558 | Me | single bond | H | *c*-Pen | Me | Cl | Me | O |
| D-14559 | Me(R) | single bond | H | *c*-Pen | Me | Cl | Me | O |
| D-14560 | Me(S) | single bond | H | *c*-Pen | Me | Cl | Me | O |
| D-14561 | H | single bond | Me | *c*-Pen | Me | Me | Me | O |
| D-14562 | Me | single bond | H | *c*-Pen | Me | Me | Me | O |
| D-14563 | Me(R) | single bond | H | *c*-Pen | Me | Me | Me | O |
| D-14564 | Me(S) | single bond | H | *c*-Pen | Me | Me | Me | O |
| D-14565 | H | single bond | Me | tetrahydrofuran-3-yl | Me | Cl | Me | O |
| D-14566 | Me | single bond | H | tetrahydrofuran-3-yl | Me | Cl | Me | O |
| D-14567 | Me(R) | single bond | H | tetrahydrofuran-3-yl | Me | Cl | Me | O |
| D-14568 | Me(S) | single bond | H | tetrahydrofuran-3-yl | Me | Cl | Me | O |
| D-14569 | H | single bond | Me | tetrahydrofuran-3-yl | Me | Me | Me | O |
| D-14570 | Me | single bond | H | tetrahydrofuran-3-yl | Me | Me | Me | O |
| D-14571 | Me(R) | single bond | H | tetrahydrofuran-3-yl | Me | Me | Me | O |
| D-14572 | Me(S) | single bond | H | tetrahydrofuran-3-yl | Me | Me | Me | O |
| D-14573 | H | single bond | Me | tetrahydrothiophen-3-yl | Me | Cl | Me | O |
| D-14574 | Me | single bond | H | tetrahydrothiophen-3-yl | Me | Cl | Me | O |
| D-14575 | Me(R) | single bond | H | tetrahydrothiophen-3-yl | Me | Cl | Me | O |
| D-14576 | Me(S) | single bond | H | tetrahydrothiophen-3-yl | Me | Cl | Me | O |
| D-14577 | H | single bond | Me | tetrahydrothiophen-3-yl | Me | Me | Me | O |
| D-14578 | Me | single bond | H | tetrahydrothiophen-3-yl | Me | Me | Me | O |
| D-14579 | Me(R) | single bond | H | tetrahydrothiophen-3-yl | Me | Me | Me | O |
| D-14580 | Me(S) | single bond | H | tetrahydrothiophen-3-yl | Me | Me | Me | O |
| D-14581 | H | single bond | Me | *c*-Pen(1-CN) | Me | Cl | Me | O |
| D-14582 | Me | single bond | H | *c*-Pen(1-CN) | Me | Cl | Me | O |

**[Table 978]**

| Compound Number | Y | W | Z | R¹ | R² | R³ | R¹⁰ | A |
|---|---|---|---|---|---|---|---|---|
| D-14583 | Me(R) | single bond | H | c-Pen(1-CN) | Me | Cl | Me | O |
| D-14584 | Me(S) | single bond | H | c-Pen(1-CN) | Me | Cl | Me | O |
| D-14585 | H | single bond | Me | c-Pen(1-CN) | Me | Me | Me | O |
| D-14586 | Me | single bond | H | c-Pen(1-CN) | Me | Me | Me | O |
| D-14587 | Me(R) | single bond | H | c-Pen(1-CN) | Me | Me | Me | O |
| D-14588 | Me(S) | single bond | H | c-Pen(1-CN) | Me | Me | Me | O |
| D-14589 | H | single bond | Me | tetrahydrofuran(3-C N)-3-yl | Me | Cl | Me | O |
| D-14590 | Me | single bond | H | tetrahydrofuran(3-CN)-3-yl | Me | Cl | Me | O |
| D-14591 | Me(R) | single bond | H | tetrahydrofuran(3-CN)-3-yl | Me | Cl | Me | O |
| D-14592 | Me(S) | single bond | H | tetrahydrofuran(3-CN)-3-yl | Me | Cl | Me | O |
| D-14593 | H | single bond | Me | tetrahydrofuran(3-CN)-3-yl | Me | Me | Me | O |
| D-14594 | Me | single bond | H | tetrahydrofuran(3-CN)-3-yl | Me | Me | Me | O |
| D-14595 | Me(R) | single bond | H | tetrahydrofuran(3-CN)-3-yl | Me | Me | Me | O |
| D-14596 | Me(S) | single bond | H | tetrahydrofuran(3-CN)-3-yl | Me | Me | Me | O |
| D-14597 | H | single bond | Me | tetrahydrothiophen(3-CN)-3-yl | Me | Cl | Me | O |
| D-14598 | Me | single bond | H | tetrahydrothiophen(3-CN)-3-yl | Me | Cl | Me | O |
| D-14599 | Me(R) | single bond | H | tetrahydrothiophen(3-CN)-3-yl | Me | Cl | Me | O |
| D-14600 | Me(S) | single bond | H | tetrahydrothiophen(3-CN)-3-yl | Me | Cl | Me | O |
| D-14601 | H | single bond | Me | tetrahydrothiophen(3-CN)-3-yl | Me | Me | Me | O |
| D-14602 | Me | single bond | H | tetrahydrothiophen(3-CN)-3-yl | Me | Me | Me | O |
| D-14603 | Me(R) | single bond | H | tetrahydrothiophen(3-CN)-3-yl | Me | Me | Me | O |
| D-14604 | Me(S) | single bond | H | tetrahydrothiophen(3-CN)-3-yl | Me | Me | Me | O |
| D-14605 | H | single bond | Me | bicyclo[1.1.1]pertan-1-yl | Me | Cl | Me | O |
| D-14606 | Me | single bond | H | bicyclo[1.1.1]pertan-1-yl | Me | Cl | Me | O |
| D-14607 | Me(R) | single bond | H | bicyclo[1.1.1]pertan-1-yl | Me | Cl | Me | O |
| D-14608 | Me(S) | single bond | H | bicyclo[1.1.1]pertan-1-yl | Me | Cl | Me | O |
| D-14609 | H | single bond | Me | bicyclo[1.1.1]pertan-1-yl | Me | Me | Me | O |
| D-14610 | Me | single bond | H | bicyclo[1.1.1]pertan-1-yl | Me | Me | Me | O |
| D-14611 | Me(R) | single bond | H | bicyclo[1.1.1]pertan-1-yl | Me | Me | Me | O |
| D-14612 | Me(S) | single bond | H | bicyclo[1.1.1]pertan-1-yl | Me | Me | Me | O |
| D-14613 | H | single bond | Me | *c*-Hex | Me | Cl | Me | O |
| D-14614 | Me | single bond | H | c -Hex | Me | Cl | Me | O |
| D-14615 | Me(R) | single bond | H | c -Hex | Me | Cl | Me | O |
| D-14616 | Me(S) | single bond | H | *c*-Hex | Me | Cl | Me | O |
| D-14617 | H | single bond | Me | c -Hex | Me | Me | Me | O |
| D-14618 | Me | single bond | H | c -Hex | Me | Me | Me | O |
| D-14619 | Me(R) | single bond | H | *c*-Hex | Me | Me | Me | O |
| D-14620 | Me(S) | single bond | H | *c*-Hex | Me | Me | Me | O |
| D-14621 | H | single bond | Me | tetrahydropyran-4-yl | Me | Cl | Me | O |
| D-14622 | Me | single bond | H | tetrahydropyran-4-yl | Me | Cl | Me | O |
| D-14623 | Me(R) | single bond | H | tetrahydropyran-4-yl | Me | Cl | Me | O |
| D-14624 | Me(S) | single bond | H | tetrahydropyran-4-yl | Me | Cl | Me | O |
| D-14625 | H | single bond | Me | tetrahydropyran-4-yl | Me | Me | Me | O |
| D-14626 | Me | single bond | H | tetrahydropyran-4-yl | Me | Me | Me | O |

**[Table 979]**

| Compound Number | Y | W | Z | R¹ | R² | R³ | R¹⁰ | A |
|---|---|---|---|---|---|---|---|---|
| D-14627 | Me(R) | single bond | H | tetrahydropyran-4-yl | Me | Me | Me | O |
| D-14628 | Me(S) | single bond | H | tetrahydropyran-4-yl | Me | Me | Me | O |
| D-14629 | H | single bond | Me | tetrahydrothiopyran-4-yl | Me | Cl | Me | O |
| D-14630 | Me | single bond | H | tetrahydrothiopyran-4-yl | Me | Cl | Me | O |
| D-14631 | Me(R) | single bond | H | tetrahydrothiopyran-4-yl | Me | Cl | Me | O |
| D-14632 | Me(S) | single bond | H | tetrahydrothiopyran-4-yl | Me | Cl | Me | O |
| D-14633 | H | single bond | Me | tetrahydrothiopyran-4-yl | Me | Me | Me | O |
| D-14634 | Me | single bond | H | tetrahydrothiopyran-4-yl | Me | Me | Me | O |
| D-14635 | Me(R) | single bond | H | tetrahydrothiopyran-4-yl | Me | Me | Me | O |
| D-14636 | Me(S) | single bond | H | tetrahydrothiopyran-4-yl | Me | Me | Me | O |
| D-14637 | H | single bond | Me | c-Hex(1-CN) | Me | Cl | Me | O |
| D-14638 | Me | single bond | H | c-Hex(1-CN) | Me | Cl | Me | O |
| D-14639 | Me(R) | single bond | H | c-Hex(1-CN) | Me | Cl | Me | O |
| D-14640 | Me(S) | single bond | H | c-Hex(1-CN) | Me | Cl | Me | O |
| D-14641 | H | single bond | Me | c-Hex(1-CN) | Me | Me | Me | O |
| D-14642 | Me | single bond | H | *c*-Hex(1-CN) | Me | Me | Me | O |
| D-14643 | Me(R) | single bond | H | c-Hex(1-CN) | Me | Me | Me | O |
| D-14644 | Me(S) | single bond | H | c-Hex(1-CN) | Me | Me | Me | O |
| D-14645 | H | single bond | Me | tetrahydropyran(4-CN)-4-yl | Me | Cl | Me | O |
| D-14646 | Me | single bond | H | tetrahydropyran(4-CN)-4-yl | Me | Cl | Me | O |
| D-14647 | Me(R) | single bond | H | tetrahydropyran(4-CN)-4-yl | Me | Cl | Me | O |
| D-14648 | Me(S) | single bond | H | tetrahydropyran(4-CN)-4-yl | Me | Cl | Me | O |
| D-14649 | H | single bond | Me | tetrahydropyran(4-CN)-4-yl | Me | Me | Me | O |
| D-14650 | Me | single bond | H | tetrahydropyran(4-CN)-4-yl | Me | Me | Me | O |
| D-14651 | Me(R) | single bond | H | tetrahydropyran(4-CN)-4-yl | Me | Me | Me | O |
| D-14652 | Me(S) | single bond | H | tetrahydropyran(4-CN)-4-yl | Me | Me | Me | O |
| D-14653 | H | single bond | Me | tetrahydrothiopyran(4-CN)-4-yl | Me | Cl | Me | O |
| D-14654 | Me | single bond | H | tetrahydrothiopyran(4-CN)-4-yl | Me | Cl | Me | O |
| D-14655 | Me(R) | single bond | H | tetrahydrothiopyran(4-CN)-4-yl | Me | Cl | Me | O |
| D-14656 | Me(S) | single bond | H | tetrahydrothiopyran(4-CN)-4-yl | Me | Cl | Me | O |
| D-14657 | H | single bond | Me | tetrahydrothiopyran(4-CN)-4-yl | Me | Me | Me | O |
| D-14658 | Me | single bond | H | tetrahydrothiopyran(4-CN)-4-yl | Me | Me | Me | O |
| D-14659 | Me(R) | single bond | H | tetrahydrothiopyran(4-CN)-4-yl | Me | Me | Me | O |
| D-14660 | Me(S) | single bond | H | tetrahydrothiopyran(4-CN)-4-yl | Me | Me | Me | O |
| D-14661 | H | single bond | Me | CH₂(*c*-Pr) | Me | Cl | Me | O |
| D-14662 | Me | single bond | H | CH₂(*c*-Pr) | Me | Cl | Me | O |
| D-14663 | Me(R) | single bond | H | CH₂(*c*-Pr) | Me | Cl | Me | O |
| D-14664 | Me(S) | single bond | H | CH₂(*c*-Pr) | Me | Cl | Me | O |
| D-14665 | H | single bond | Me | CH₂(*c*-Pr) | Me | Me | Me | O |
| D-14666 | Me | single bond | H | CH₂(*c*-Pr) | Me | Me | Me | O |
| D-14667 | Me(R) | single bond | H | CH₂(*c*-Pr) | Me | Me | Me | O |
| D-14668 | Me(S) | single bond | H | CH₂(*c*-Pr) | Me | Me | Me | O |
| D-14669 | H | single bond | Me | CH₂[*c*-Pr(1-CN)] | Me | Cl | Me | O |
| D-14670 | Me | single bond | H | CH₂[*c*-Pr(1-CN)] | Me | Cl | Me | O |

**[Table 980]**

| Compound Number | Y | W | Z | R¹ | R² | R³ | R¹⁰ | A |
|---|---|---|---|---|---|---|---|---|
| D-14671 | Me(R) | single bond | H | CH₂[*c*-Pr(1-CN)] | Me | Cl | Me | O |
| D-14672 | Me(S) | single bond | H | CH₂[*c*-Pr(1-CN)] | Me | Cl | Me | O |
| D-14673 | H | single bond | Me | CH₂[*c*-Pr(1-CN)] | Me | Me | Me | O |
| D-14674 | Me | single bond | H | CH₂[*c*-Pr(1-CN)] | Me | Me | Me | O |
| D-14675 | Me(R) | single bond | H | CH₂[*c*-Pr(1-CN)] | Me | Me | Me | O |
| D-14676 | Me(S) | single bond | H | CH₂[*c*-Pr(1-CN)] | Me | Me | Me | O |
| D-14677 | H | single bond | Me | CH(Me)(*c*-Pr) | Me | Cl | Me | O |
| D-14678 | Me | single bond | H | CH(Me)(*c*-Pr) | Me | Cl | Me | O |
| D-14679 | Me(R) | single bond | H | CH(Me)(*c*-Pr) | Me | Cl | Me | O |
| D-14680 | Me(S) | single bond | H | CH(Me)(*c*-Pr) | Me | Cl | Me | O |
| D-14681 | H | single bond | Me | CH(Me)(*c*-Pr) | Me | Me | Me | O |
| D-14682 | Me | single bond | H | CH(Me)(*c*-Pr) | Me | Me | Me | O |
| D-14683 | Me(R) | single bond | H | CH(Me)(*c*-Pr) | Me | Me | Me | O |
| 0-14684 | Me(S) | single bond | H | CH(Me)(*c*-Pr) | Me | Me | Me | O |
| D-14685 | H | single bond | Me | CH(Me)CH₂OMe | Me | Cl | Me | O |
| D-14686 | Me | single bond | H | CH(Me)CH₂OMe | Me | Cl | Me | O |
| D-14687 | Me(R) | single bond | H | CH(Me)CH₂OMe | Me | Cl | Me | O |
| D-14688 | Me(S) | single bond | H | CH(Me)CH₂OMe | Me | Cl | Me | O |
| D-14689 | H | single bond | Me | CH(Me)CH₂OMe | Me | Me | Me | O |
| D-14690 | Me | single bond | H | CH(Me)CH₂OMe | Me | Me | Me | O |
| D-14691 | Me(R) | single bond | H | CH(Me)CH₂OMe | Me | Me | Me | O |
| D-14692 | Me(S) | single bond | H | CH(Me)CH₂OMe | Me | Me | Me | O |
| D-14693 | H | single bond | Me | CH₂CH=CH₂ | Me | Cl | Me | O |
| D-14694 | Me | single bond | H | CH₂CH=CH₂ | Me | Cl | Me | O |
| D-14695 | Me(R) | single bond | H | CH₂CH=CH₂ | Me | Cl | Me | O |
| D-14696 | Me(S) | single bond | H | CH₂CH=CH₂ | Me | Cl | Me | O |
| D-14697 | H | single bond | Me | CH₂CH=CH₂ | Me | Me | Me | O |
| D-14698 | Me | single bond | H | CH₂CH=CH₂ | Me | Me | Me | O |
| D-14699 | Me(R) | single bond | H | CH₂CH=CH₂ | Me | Me | Me | O |
| D-14700 | Me(S) | single bond | H | CH₂CH=CH₂ | Me | Me | Me | O |
| D-14701 | H | single bond | Me | CH₂C≡CH | Me | Cl | Me | O |
| D-14702 | Me | single bond | H | CH₂C≡CH | Me | Cl | Me | O |
| D-14703 | Me(R) | single bond | H | CH₂C≡CH | Me | Cl | Me | O |
| D-14704 | Me(S) | single bond | H | CH₂C≡CH | Me | Cl | Me | O |
| D-14705 | H | single bond | Me | CH₂C≡CH | Me | Me | Me | O |
| D-14706 | Me | single bond | H | CH₂C≡CH | Me | Me | Me | O |
| D-14707 | Me(R) | single bond | H | CH₂C≡CH | Me | Me | Me | O |
| D-14708 | Me(S) | single bond | H | CH₂C≡CH | Me | Me | Me | O |
| D-14709 | H | single bond | Me | CH₂CN | Me | Cl | Me | O |
| D-14710 | Me | single bond | H | CH₂CN | Me | Cl | Me | O |
| D-14711 | Me(R) | single bond | H | CH₂CN | Me | Cl | Me | O |
| D-14712 | Me(S) | single bond | H | CH₂CN | Me | Cl | Me | O |
| D-14713 | H | single bond | Me | CH₂CN | Me | Me | Me | O |
| D-14714 | Me | single bond | H | CH₂CN | Me | Me | Me | O |

**[Table 981]**

| Compound Number | Y | W | Z | R¹ | R² | R³ | R¹⁰ | A |
|---|---|---|---|---|---|---|---|---|
| D-14715 | Me(R) | single bond | H | CH₂CN | Me | Me | Me | O |
| D-14716 | Me(S) | single bond | H | CH₂CN | Me | Me | Me | O |
| D-14717 | H | single bond | Me | CH(Me)CN | Me | Cl | Me | O |
| D-14718 | Me | single bond | H | CH(Me)CN | Me | Cl | Me | O |
| D-14719 | Me(R) | single bond | H | CH(Me)CN | Me | Cl | Me | O |
| D-14720 | Me(S) | single bond | H | CH(Me)CN | Me | Cl | Me | O |
| D-14721 | H | single bond | Me | CH(Me)CN | Me | Me | Me | O |
| D-14722 | Me | single bond | H | CH(Me)CN | Me | Me | Me | O |
| D-14723 | Me(R) | single bond | H | CH(Me)CN | Me | Me | Me | O |
| D-14724 | Me(S) | single bond | H | CH(Me)CN | Me | Me | Me | O |
| D-14725 | H | single bond | Me | C(Me)₂CN | Me | Cl | Me | O |
| D-14726 | Me | single bond | H | C(Me)₂CN | Me | Cl | Me | O |
| D-14727 | Me(R) | single bond | H | C(Me)₂CN | Me | Cl | Me | O |
| D-14728 | Me(S) | single bond | H | C(Me)₂CN | Me | Cl | Me | O |
| D-14729 | H | single bond | Me | C(Me)₂CN | Me | Me | Me | O |
| D-14730 | Me | single bond | H | C(Me)₂CN | Me | Me | Me | O |
| D-14731 | Me(R) | single bond | H | C(Me)₂CN | Me | Me | Me | O |
| D-14732 | Me(S) | single bond | H | C(Me)₂CN | Me | Me | Me | O |
| D-14733 | H | single bond | Me | C(Me)(*i*-Pr)CN | Me | Cl | Me | O |
| D-14734 | Me | single bond | H | C(Me)(*i*-Pr)CN | Me | Cl | Me | O |
| D-14735 | Me(R) | single bond | H | C(Me)(*i*-Pr)CN | Me | Cl | Me | O |
| D-14736 | Me(S) | single bond | H | C(Me)(*i*-Pr)CN | Me | Cl | Me | O |
| D-14737 | H | single bond | Me | C(Me)(*i*-Pr)CN | Me | Me | Me | O |
| D-14738 | Me | single bond | H | C(Me)(*i*-Pr)CN | Me | Me | Me | O |
| D-14739 | Me(R) | single bond | H | C(Me)(*i*-Pr)CN | Me | Me | Me | O |
| D-14740 | Me(S) | single bond | H | C(Me)(*i*-Pr)CN | Me | Me | Me | O |
| D-14741 | H | single bond | Me | OMe | Me | Cl | Me | O |
| D-14742 | Me | single bond | H | OMe | Me | Cl | Me | O |
| D-14743 | Me(R) | single bond | H | OMe | Me | Cl | Me | O |
| D-14744 | Me(S) | single bond | H | OMe | Me | Cl | Me | O |
| D-14745 | H | single bond | Me | OMe | Me | Me | Me | O |
| D-14746 | Me | single bond | H | OMe | Me | Me | Me | O |
| D-14747 | Me(R) | single bond | H | OMe | Me | Me | Me | O |
| D-14748 | Me(S) | single bond | H | OMe | Me | Me | Me | O |
| D-14749 | H | single bond | Me | CH=NOMe | Me | Cl | Me | O |
| D-14750 | Me | single bond | H | CH=NOMe | Me | Cl | Me | O |
| D-14751 | Me(R) | single bond | H | CH=NOMe | Me | Cl | Me | O |
| D-14752 | Me(S) | single bond | H | CH=NOMe | Me | Cl | Me | O |
| D-14753 | H | single bond | Me | CH=NOMe | Me | Me | Me | O |
| D-14754 | Me | single bond | H | CH=NOMe | Me | Me | Me | O |
| D-14755 | Me(R) | single bond | H | CH=NOMe | Me | Me | Me | O |
| D-14756 | Me(S) | single bond | H | CH=NOMe | Me | Me | Me | O |
| D-14757 | H | single bond | Me | morpholin-4-yl | Me | Cl | Me | O |
| D-14758 | Me | single bond | H | morpholin-4-yl | Me | Cl | Me | O |

**[Table 982]**

| Compound Number | Y | W | Z | R¹ | R² | R³ | R¹⁰ | A |
|---|---|---|---|---|---|---|---|---|
| D-14759 | Me(R) | single bond | H | morpholin-4-yl | Me | Cl | Me | O |
| D-14760 | Me(S) | single bond | H | morpholin-4-yl | Me | Cl | Me | O |
| D-14761 | H | single bond | Me | morpholin-4-yl | Me | Me | Me | O |
| D-14762 | Me | single bond | H | morpholin-4-yl | Me | Me | Me | O |
| D-14763 | Me(R) | single bond | H | morpholin-4-yl | Me | Me | Me | O |
| D-14764 | Me(S) | single bond | H | morpholin-4-yl | Me | Me | Me | O |
| D-14765 | H | single bond | Me | Ph | Me | Cl | Me | O |
| D-14766 | Me | single bond | H | Ph | Me | Cl | Me | O |
| D-14767 | Me(R) | single bond | H | Ph | Me | Cl | Me | O |
| D-14768 | Me(S) | single bond | H | Ph | Me | Cl | Me | O |
| D-14769 | H | single bond | Me | Ph | Me | Me | Me | O |
| D-14770 | Me | single bond | H | Ph | Me | Me | Me | O |
| D-14771 | Me(R) | single bond | H | Ph | Me | Me | Me | O |
| D-14772 | Me(S) | single bond | H | Ph | Me | Me | Me | O |
| D-14773 | H | single bond | Me | CH₂Ph | Me | Cl | Me | O |
| D-14774 | Me | single bond | H | CH₂Ph | Me | Cl | Me | O |
| D-14775 | Me(R) | single bond | H | CH₂Ph | Me | Cl | Me | O |
| D-14776 | Me(S) | single bond | H | CH₂Ph | Me | Cl | Me | O |
| D-14777 | H | single bond | Me | CH₂Ph | Me | Me | Me | O |
| D-14778 | Me | single bond | H | CH₂Ph | Me | Me | Me | O |
| D-14779 | Me(R) | single bond | H | CH₂Ph | Me | Me | Me | O |
| D-14780 | Me(S) | single bond | H | CH₂Ph | Me | Me | Me | O |
| D-14781 | H | single bond | Me | thiazole-2-yl | Me | Cl | Me | O |
| D-14782 | Me | single bond | H | thiazole-2-yl | Me | Cl | Me | O |
| D-14783 | Me(R) | single bond | H | thiazole-2-yl | Me | Cl | Me | O |
| D-14784 | Me(S) | single bond | H | thiazole-2-yl | Me | Cl | Me | O |
| D-14785 | H | single bond | Me | thiazole-2-yl | Me | Me | Me | O |
| D-14786 | Me | single bond | H | thiazole-2-yl | Me | Me | Me | O |
| D-14787 | Me(R) | single bond | H | thiazole-2-yl | Me | Me | Me | O |
| D-14788 | Me(S) | single bond | H | thiazole-2-yl | Me | Me | Me | O |
| D-14789 | H | single bond | Me | 1-Me-pyrazol-3-yl | Me | Cl | Me | O |
| D-14790 | Me | single bond | H | 1-Me-pyrazol-3-yl | Me | Cl | Me | O |
| D-14791 | Me(R) | single bond | H | 1-Me-pyrazol-3-yl | Me | Cl | Me | O |
| D-14792 | Me(S) | single bond | H | 1-Me-pyrazol-3-yl | Me | Cl | Me | O |
| D-14793 | H | single bond | Me | 1-Me-pyrazol-3-yl | Me | Me | Me | O |
| D-14794 | Me | single bond | H | 1-Me-pyrazol-3-yl | Me | Me | Me | O |
| D-14795 | Me(R) | single bond | H | 1-Me-pyrazol-3-yl | Me | Me | Me | O |
| D-14796 | Me(S) | single bond | H | 1-Me-pyrazol-3-yl | Me | Me | Me | O |
| D-14797 | H | single bond | Me | 1-Me-pyrazol-5-yl | Me | Cl | Me | O |
| D-14798 | Me | single bond | H | 1-Me-pyrazol-5-yl | Me | Cl | Me | O |
| D-14799 | Me(R) | single bond | H | 1-Me-pyrazol-5-yl | Me | Cl | Me | O |
| D-14800 | Me(S) | single bond | H | 1-Me-pyrazol-5-yl | Me | Cl | Me | O |
| D-14801 | H | single bond | Me | 1-Me-pyrazol-5-yl | Me | Me | Me | O |
| D-14802 | Me | single bond | H | 1-Me-pyrazol-5-yl | Me | Me | Me | O |

**[Table 983]**

| Compound Number | Y | W | Z | R¹ | R² | R³ | R¹⁰ | A |
|---|---|---|---|---|---|---|---|---|
| D-14803 | Me(R) | single bond | H | 1-Me-pyrazol-5-yl | Me | Me | Me | O |
| D-14804 | Me(S) | single bond | H | 1-Me-pyrazol-5-yl | Me | Me | Me | O |
| D-14805 | H | single bond | Me | CH₂(pyrimidin-2-yl) | Me | Cl | Me | O |
| D-14806 | Me | single bond | H | CH₂(pyrimidin-2-yl) | Me | Cl | Me | O |
| D-14807 | Me(R) | single bond | H | CH₂(pyrimidin-2-yl) | Me | Cl | Me | O |
| D-14808 | Me(S) | single bond | H | CH₂(pyrimidin-2-yl) | Me | Cl | Me | O |
| D-14809 | H | single bond | Me | CH₂(pyrimidin-2-yl) | Me | Me | Me | O |
| D-14810 | Me | single bond | H | CH₂(pyrimidin-2-yl) | Me | Me | Me | O |
| D-14811 | Me(R) | single bond | H | CH₂(pyrimidin-2-yl) | Me | Me | Me | O |
| D-14812 | Me(S) | single bond | H | CH₂(pyrimidin-2-yl) | Me | Me | Me | O |
| D-14813 | H | single bond | Me | Me | Me | Cl | CH₂OMe | O |
| D-14814 | Me | single bond | H | Me | Me | Cl | CH₂OMe | O |
| D-14815 | Me(R) | single bond | H | Me | Me | Cl | CH₂OMe | O |
| D-14816 | Me(S) | single bond | H | Me | Me | Cl | CH₂OMe | O |
| D-14817 | H | single bond | Me | Me | Me | Me | CH₂OMe | O |
| D-14818 | Me | single bond | H | Me | Me | Me | CH₂OMe | O |
| D-14819 | Me(R) | single bond | H | Me | Me | Me | CH₂OMe | O |
| D-14820 | Me(S) | single bond | H | Me | Me | Me | CH₂OMe | O |
| D-14821 | H | single bond | Me | CH₂CHF₂ | Me | Cl | CH₂OMe | O |
| D-14822 | Me | single bond | H | CH₂CHF₂ | Me | Cl | CH₂OMe | O |
| D-14823 | Me(R) | single bond | H | CH₂CHF₂ | Me | Cl | CH₂OMe | O |
| D-14824 | Me(S) | single bond | H | CH₂CHF₂ | Me | Cl | CH₂OMe | O |
| D-14825 | H | single bond | Me | CH₂CHF₂ | Me | Me | CH₂OMe | O |
| D-14826 | Me | single bond | H | CH₂CHF₂ | Me | Me | CH₂OMe | O |
| D-14827 | Me(R) | single bond | H | CH₂CHF₂ | Me | Me | CH₂OMe | O |
| D-14828 | Me(S) | single bond | H | CH₂CHF₂ | Me | Me | CH₂OMe | O |
| D-14829 | H | single bond | Me | *n*-Pr | Me | Cl | CH₂OMe | O |
| D-14830 | Me | single bond | H | *n*-Pr | Me | Cl | CH₂OMe | O |
| D-14831 | Me(R) | single bond | H | *n*-Pr | Me | Cl | CH₂OMe | O |
| D-14832 | Me(S) | single bond | H | *n*-Pr | Me | Cl | CH₂OMe | O |
| D-14833 | H | single bond | Me | *n*-Pr | Me | Me | CH₂OMe | O |
| D-14834 | Me | single bond | H | *n*-Pr | Me | Me | CH₂OMe | O |
| D-14835 | Me(R) | single bond | H | *n*-Pr | Me | Me | CH₂OMe | O |
| D-14836 | Me(S) | single bond | H | *n*-Pr | Me | Me | CH₂OMe | O |
| D-14837 | H | single bond | Me | *i*-Pr | Me | Cl | CH₂OMe | O |
| D-14838 | Me | single bond | H | *i*-Pr | Me | Cl | CH₂OMe | O |
| D-14839 | Me(R) | single bond | H | *i*-Pr | Me | Cl | CH₂OMe | O |
| D-14840 | Me(S) | single bond | H | *i*-Pr | Me | Cl | CH₂OMe | O |
| D-14841 | H | single bond | Me | *i*-Pr | Me | Me | CH₂OMe | O |
| D-14842 | Me | single bond | H | *i*-Pr | Me | Me | CH₂OMe | O |
| D-14843 | Me(R) | single bond | H | *i*-Pr | Me | Me | CH₂OMe | O |
| D-14844 | Me(S) | single bond | H | *i*-Pr | Me | Me | CH₂OMe | O |
| D-14845 | H | single bond | Me | *c*-Pr(1-F) | Me | Cl | CH₂OMe | O |
| D-14846 | Me | single bond | H | *c*-Pr(1-F) | Me | Cl | CH₂OMe | O |

**[Table 984]**

| Compound Number | Y | W | Z | R¹ | R² | R³ | R¹⁰ | A |
|---|---|---|---|---|---|---|---|---|
| D-14847 | Me(R) | single bond | H | *c*-Pr(1-F) | Me | Cl | CH₂OMe | O |
| D-14848 | Me(S) | single bond | H | *c*-Pr(1-F) | Me | Cl | CH₂OMe | O |
| D-14849 | H | single bond | Me | *c*-Pr(1-F) | Me | Me | CH₂OMe | O |
| D-14850 | Me | single bond | H | *c*-Pr(1-F) | Me | Me | CH₂OMe | O |
| D-14851 | Me(R) | single bond | H | *c*-Pr(1-F) | Me | Me | CH₂OMe | O |
| D-14852 | Me(S) | single bond | H | *c*-Pr(1-F) | Me | Me | CH₂OMe | O |
| D-14853 | H | single bond | Me | *c*-Pr(2,2-F₂) | Me | Cl | CH₂OMe | O |
| D-14854 | Me | single bond | H | *c*-Pr(2,2-F₂) | Me | Cl | CH₂OMe | O |
| D-14855 | Me(R) | single bond | H | *c*-Pr(2,2-F₂) | Me | Cl | CH₂OMe | O |
| D-14856 | Me(S) | single bond | H | *c*-Pr(2,2-F₂) | Me | Cl | CH₂OMe | O |
| D-14857 | H | single bond | Me | *c*-Pr(2,2-F₂) | Me | Me | CH₂OMe | O |
| D-14858 | Me | single bond | H | *c*-Pr(2,2-F₂) | Me | Me | CH₂OMe | O |
| D-14859 | Me(R) | single bond | H | *c*-Pr(2,2-F₂) | Me | Me | CH₂OMe | O |
| D-14860 | Me(S) | single bond | H | *c*-Pr(2,2-F₂) | Me | Me | CH₂OMe | O |
| D-14861 | H | single bond | Me | *c*-Pr(1-Cl) | Me | Cl | CH₂OMe | O |
| D-14862 | Me | single bond | H | *c*-Pr(1-Cl) | Me | Cl | CH₂OMe | O |
| D-14863 | Me(R) | single bond | H | *c*-Pr(1-Cl) | Me | Cl | CH₂OMe | O |
| D-14864 | Me(S) | single bond | H | *c*-Pr(1-Cl) | Me | Cl | CH₂OMe | O |
| D-14865 | H | single bond | Me | *c*-Pr(1-Cl) | Me | Me | CH₂OMe | O |
| D-14866 | Me | single bond | H | *c*-Pr(1-Cl) | Me | Me | CH₂OMe | O |
| D-14867 | Me(R) | single bond | H | *c*-Pr(1-Cl) | Me | Me | CH₂OMe | O |
| D-14868 | Me(S) | single bond | H | *c*-Pr(1-Cl) | Me | Me | CH₂OMe | O |
| D-14869 | H | single bond | Me | *c*-Pr(1-Br) | Me | Cl | CH₂OMe | O |
| D-14870 | Me | single bond | H | *c*-Pr(1-Br) | Me | Cl | CH₂OMe | O |
| D-14871 | Me(R) | single bond | H | *c*-Pr(1-Br) | Me | Cl | CH₂OMe | O |
| D-14872 | Me(S) | single bond | H | *c*-Pr(1-Br) | Me | Cl | CH₂OMe | O |
| D-14873 | H | single bond | Me | *c*-Pr(1-Br) | Me | Me | CH₂OMe | O |
| D-14874 | Me | single bond | H | *c*-Pr(1-Br) | Me | Me | CH₂OMe | O |
| D-14875 | Me(R) | single bond | H | *c*-Pr(1-Br) | Me | Me | CH₂OMe | O |
| D-14876 | Me(S) | single bond | H | *c*-Pr(1-Br) | Me | Me | CH₂OMe | O |
| D-14877 | H | single bond | Me | *c*-Pr(1-Me) | Me | Cl | CH₂OMe | O |
| D-14878 | Me | single bond | H | *c*-Pr(1-Me) | Me | Cl | CH₂OMe | O |
| D-14879 | Me(R) | single bond | H | *c*-Pr(1-Me) | Me | Cl | CH₂OMe | O |
| D-14880 | Me(S) | single bond | H | *c*-Pr(1-Me) | Me | Cl | CH₂OMe | O |
| D-14881 | H | single bond | Me | *c*-Pr(1-Me) | Me | Me | CH₂OMe | O |
| D-14882 | Me | single bond | H | *c*-Pr(1-Me) | Me | Me | CH₂OMe | O |
| D-14883 | Me(R) | single bond | H | *c*-Pr(1-Me) | Me | Me | CH₂OMe | O |
| D-14884 | Me(S) | single bond | H | *c*-Pr(1-Me) | Me | Me | CH₂OMe | O |
| D-14885 | H | single bond | Me | *c*-Pr(1-CH₂OH) | Me | Cl | CH₂OMe | O |
| D-14886 | Me | single bond | H | *c*-Pr(1-CH₂OH) | Me | Cl | CH₂OMe | O |
| D-14887 | Me(R) | single bond | H | *c*-Pr(1-CH₂OH) | Me | Cl | CH₂OMe | O |
| D-14888 | Me(S) | single bond | H | *c*-Pr(1-CH₂OH) | Me | Cl | CH₂OMe | O |
| D-14889 | H | single bond | Me | *c*-Pr(1-CH₂OH) | Me | Me | CH₂OMe | O |
| D-14890 | Me | single bond | H | *c*-Pr(1-CH₂OH) | Me | Me | CH₂OMe | O |

**[Table 985]**

| Compound Number | Y | W | Z | R¹ | R² | R³ | R¹⁰ | A |
|---|---|---|---|---|---|---|---|---|
| D-14891 | Me(R) | single bond | H | *c*-Pr(1-CH₂OH) | Me | Me | CH₂OMe | O |
| D-14892 | Me(S) | single bond | H | *c*-Pr(1-CH₂OH) | Me | Me | CH₂OMe | O |
| D-14893 | H | single bond | Me | *c*-Pr[1-CH₂OS(=O)₂Me] | Me | Cl | CH₂OMe | O |
| D-14894 | Me | single bond | H | *c*-Pr[1-CH₂OS(=O)₂Me] | Me | Cl | CH₂OMe | O |
| D-14895 | Me(R) | single bond | H | *c*-Pr[1-CH₂OS(=O)₂Me] | Me | Cl | CH₂OMe | O |
| D-14896 | Me(S) | single bond | H | *c*-Pr[1-CH₂OS(=O)₂Me] | Me | Cl | CH₂OMe | O |
| D-14897 | H | single bond | Me | *c*-Pr[1-CH₂OS(=O)₂Me] | Me | Me | CH₂OMe | O |
| D-14898 | Me | single bond | H | *c*-Pr[1-CH₂OS(=O)₂Me] | Me | Me | CH₂OMe | O |
| D-14899 | Me(R) | single bond | H | *c*-Pr[1-CH₂OS(=O)₂Me] | Me | Me | CH₂OMe | O |
| D-14900 | Me(S) | single bond | H | *c*-Pr[1-CH₂OS(=O)₂Me] | Me | Me | CH₂OMe | O |
| D-14901 | H | single bond | Me | *c*-Pr(1-CH₂OMe) | Me | Cl | CH₂OMe | O |
| D-14902 | Me | single bond | H | *c*-Pr(1-CH₂OMe) | Me | Cl | CH₂OMe | O |
| D-14903 | Me(R) | single bond | H | *c*-Pr(1-CH₂OMe) | Me | Cl | CH₂OMe | O |
| D-14904 | Me(S) | single bond | H | *c*-Pr(1-CH₂OMe) | Me | Cl | CH₂OMe | O |
| D-14905 | H | single bond | Me | *c*-Pr(1-CH₂OMe) | Me | Me | CH₂OMe | O |
| D-14906 | Me | single bond | H | *c*-Pr(1-CH₂OMe) | Me | Me | CH₂OMe | O |
| D-14907 | Me(R) | single bond | H | *c*-Pr(1-CH₂OMe) | Me | Me | CH₂OMe | O |
| 0-14908 | Me(S) | single bond | H | *c*-Pr(1-CH₂OMe) | Me | Me | CH₂OMe | O |
| 0-14909 | H | single bond | Me | *c*-Pr(1-CH₂OCN) | Me | Cl | CH₂OMe | O |
| D-14910 | Me | single bond | H | *c*-Pr(1-CH₂OCN) | Me | Cl | CH₂OMe | O |
| D-14911 | Me(R) | single bond | H | *c*-Pr(1-CH₂OCN) | Me | Cl | CH₂OMe | O |
| D-14912 | Me(S) | single bond | H | *c*-Pr(1-CH₂OCN) | Me | Cl | CH₂OMe | O |
| D-14913 | H | single bond | Me | *c*-Pr(1-CH₂OCN) | Me | Me | CH₂OMe | O |
| D-14914 | Me | single bond | H | *c*-Pr(1-CH₂OCN) | Me | Me | CH₂OMe | O |
| D-14915 | Me(R) | single bond | H | *c*-Pr(1-CH₂OCN) | Me | Me | CH₂OMe | O |
| D-14916 | Me(S) | single bond | H | *c*-Pr(1-CH₂OCN) | Me | Me | CH₂OMe | O |
| D-14917 | H | single bond | Me | *c*-Pr(1-CH₂SMe) | Me | Cl | CH₂OMe | O |
| D-14918 | Me | single bond | H | *c*-Pr(1-CH₂SMe) | Me | Cl | CH₂OMe | O |
| D-14919 | Me(R) | single bond | H | *c*-Pr(1-CH₂SMe) | Me | Cl | CH₂OMe | O |
| D-14920 | Me(S) | single bond | H | *c*-Pr(1-CH₂SMe) | Me | Cl | CH₂OMe | O |
| D-14921 | H | single bond | Me | *c*-Pr(1-CH₂SMe) | Me | Me | CH₂OMe | O |
| D-14922 | Me | single bond | H | *c*-Pr(1-CH₂SMe) | Me | Me | CH₂OMe | O |
| D-14923 | Me(R) | single bond | H | *c*-Pr(1-CH₂SMe) | Me | Me | CH₂OMe | O |
| D-14924 | Me(S) | single bond | H | *c*-Pr(1-CH₂SMe) | Me | Me | CH₂OMe | O |
| D-14925 | H | single bond | Me | *c*-Pr[1-CH₂S(=O)Me] | Me | Cl | CH₂OMe | O |
| D-14926 | Me | single bond | H | *c*-Pr[1-CH₂S(=O)Me] | Me | Cl | CH₂OMe | O |
| D-14927 | Me(R) | single bond | H | *c*-Pr[1-CH₂S(=O)Me] | Me | Cl | CH₂OMe | O |
| D-14928 | Me(S) | single bond | H | *c*-Pr[1-CH₂S(=O)Me] | Me | Cl | CH₂OMe | O |
| D-14929 | H | single bond | Me | *c*-Pr[1-CH₂S(=O)Me] | Me | Me | CH₂OMe | O |
| D-14930 | Me | single bond | H | *c*-Pr[1-CH₂S(=O)Me] | Me | Me | CH₂OMe | O |
| D-14931 | Me(R) | single bond | H | *c*-Pr[1-CH₂S(=O)Me] | Me | Me | CH₂OMe | O |
| D-14932 | Me(S) | single bond | H | *c*-Pr[1-CH₂S(=O)Me] | Me | Me | CH₂OMe | O |
| D-14933 | H | single bond | Me | *c*-Pr[1-CH₂S(=O)₂Me] | Me | Cl | CH₂OMe | O |
| D-14934 | Me | single bond | H | *c*-Pr[1-CH₂S(=O)₂Me] | Me | Cl | CH₂OMe | O |

**[Table 986]**

| Compound Number | Y | W | Z | R¹ | R² | R³ | R¹⁰ | A |
|---|---|---|---|---|---|---|---|---|
| D-14935 | Me(R) | single bond | H | *c*-Pr[1-CH₂S(=O)₂Me] | Me | Cl | CH₂OMe | O |
| D-14936 | Me(S) | single bond | H | *c*-Pr[1-CH₂S(=O)₂Me] | Me | Cl | CH₂OMe | O |
| D-14937 | H | single bond | Me | *c*-Pr[1-CH₂S(=O)₂Me] | Me | Me | CH₂OMe | O |
| D-14938 | Me | single bond | H | *c*-Pr[1-CH₂S(=O)₂Me] | Me | Me | CH₂OMe | O |
| D-14939 | Me(R) | single bond | H | *c*-Pr[1-CH₂S(=O)₂Me] | Me | Me | CH₂OMe | O |
| D-14940 | Me(S) | single bond | H | *c*-Pr[1-CH₂S(=O)₂Me] | Me | Me | CH₂OMe | O |
| D-14941 | H | single bond | Me | *c*-Pr(1-CH₂F) | Me | Cl | CH₂OMe | O |
| D-14942 | Me | single bond | H | *c*-Pr(1-CH₂F) | Me | Cl | CH₂OMe | O |
| D-14943 | Me(R) | single bond | H | *c*-Pr(1-CH₂F) | Me | Cl | CH₂OMe | O |
| D-14944 | Me(S) | single bond | H | *c*-Pr(1-CH₂F) | Me | Cl | CH₂OMe | O |
| D-14945 | H | single bond | Me | *c*-Pr(1-CH₂F) | Me | Me | CH₂OMe | O |
| D-14946 | Me | single bond | H | *c*-Pr(1-CH₂F) | Me | Me | CH₂OMe | O |
| D-14947 | Me(R) | single bond | H | *c*-Pr(1-CH₂F) | Me | Me | CH₂OMe | O |
| D-14948 | Me(S) | single bond | H | *c*-Pr(1-CH₂F) | Me | Me | CH₂OMe | O |
| D-14949 | H | single bond | Me | *c*-Pr(1-CHF₂) | Me | Cl | CH₂OMe | O |
| D-14950 | Me | single bond | H | *c*-Pr(1-CHF₂) | Me | Cl | CH₂OMe | O |
| D-14951 | Me(R) | single bond | H | *c*-Pr(1-CHF₂) | Me | Cl | CH₂OMe | O |
| D-14952 | Me(S) | single bond | H | *c*-Pr(1-CHF₂) | Me | Cl | CH₂OMe | O |
| D-14953 | H | single bond | Me | *c*-Pr(1-CHF₂) | Me | Me | CH₂OMe | O |
| D-14954 | Me | single bond | H | *c*-Pr(1-CHF₂) | Me | Me | CH₂OMe | O |
| D-14955 | Me(R) | single bond | H | *c*-Pr(1-CHF₂) | Me | Me | CH₂OMe | O |
| D-14956 | Me(S) | single bond | H | *c*-Pr(1-CHF₂) | Me | Me | CH₂OMe | O |
| D-14957 | H | single bond | Me | *c*-Pr(1-CF₃) | Me | Cl | CH₂OMe | O |
| D-14958 | Me | single bond | H | *c*-Pr(1-CF₃) | Me | Cl | CH₂OMe | O |
| D-14959 | Me(R) | single bond | H | *c*-Pr(1-CF₃) | Me | Cl | CH₂OMe | O |
| D-14960 | Me(S) | single bond | H | *c*-Pr(1-CF₃) | Me | Cl | CH₂OMe | O |
| D-14961 | H | single bond | Me | *c*-Pr(1-CF₃) | Me | Me | CH₂OMe | O |
| D-14962 | Me | single bond | H | *c*-Pr(1-CF₃) | Me | Me | CH₂OMe | O |
| D-14963 | Me(R) | single bond | H | *c*-Pr(1-CF₃) | Me | Me | CH₂OMe | O |
| D-14964 | Me(S) | single bond | H | *c*-Pr(1-CF₃) | Me | Me | CH₂OMe | O |
| D-14965 | H | single bond | Me | *c*-pr(1-C≡CH) | Me | Cl | CH₂OMe | O |
| D-14966 | Me | single bond | H | *c*-pr(1-C≡CH) | Me | Cl | CH₂OMe | O |
| D-14967 | Me(R) | single bond | H | *c*-pr(1-C≡CH) | Me | Cl | CH₂OMe | O |
| D-14968 | Me(S) | single bond | H | *c*-pr(1-C≡CH) | Me | Cl | CH₂OMe | O |
| D-14969 | H | single bond | Me | *c*-pr(1-C≡CH) | Me | Me | CH₂OMe | O |
| D-14970 | Me | single bond | H | *c*-pr(1-C≡CH) | Me | Me | CH₂OMe | O |
| D-14971 | Me(R) | single bond | H | *c*-pr(1-C≡CH) | Me | Me | CH₂OMe | O |
| D-14972 | Me(S) | single bond | H | *c*-pr(1-C≡CH) | Me | Me | CH₂OMe | O |
| D-14973 | H | single bond | Me | *c*-Pr[1-C(=O)OEt] | Me | Cl | CH₂OMe | O |
| D-14974 | Me | single bond | H | *c*-Pr[1-C(=O)OEt] | Me | Cl | CH₂OMe | O |
| D-14975 | Me(R) | single bond | H | *c*-Pr[1-C(=O)OEt] | Me | Cl | CH₂OMe | O |
| D-14976 | Me(S) | single bond | H | *c*-Pr[1-C(=O)OEt] | Me | Cl | CH₂OMe | O |
| D-14977 | H | single bond | Me | *c*-Pr[1-C(=O)OEt] | Me | Me | CH₂OMe | O |
| D-14978 | Me | single bond | H | *c*-Pr[1-C(=O)OEt] | Me | Me | CH₂OMe | O |

**[Table 987]**

| Compound Number | Y | W | Z | R¹ | R² | R³ | R¹⁰ | A |
|---|---|---|---|---|---|---|---|---|
| D-14979 | Me(R) | single bond | H | *c*-Pr[1-C(=O)OEt] | Me | Me | CH₂OMe | O |
| D-14980 | Me(S) | single bond | H | *c*-Pr[1-C(=O)OEt] | Me | Me | CH₂OMe | O |
| D-14981 | H | single bond | Me | *c*-Pr[1-C(=O)OH] | Me | Cl | CH₂OMe | O |
| D-14982 | Me | single bond | H | *c*-Pr[1-C(=O)OH] | Me | Cl | CH₂OMe | O |
| D-14983 | Me(R) | single bond | H | *c*-Pr[1-C(=O)OH] | Me | Cl | CH₂OMe | O |
| D-14984 | Me(S) | single bond | H | *c*-Pr[1-C(=O)OH] | Me | Cl | CH₂OMe | O |
| D-14985 | H | single bond | Me | *c*-Pr[1-C(=O)OH] | Me | Me | CH₂OMe | O |
| D-14986 | Me | single bond | H | *c*-Pr[1-C(=O)OH] | Me | Me | CH₂OMe | O |
| D-14987 | Me(R) | single bond | H | *c*-Pr[1-C(=O)OH] | Me | Me | CH₂OMe | O |
| D-14988 | Me(S) | single bond | H | *c*-Pr[1-C(=O)OH] | Me | Me | CH₂OMe | O |
| D-14989 | H | single bond | Me | *c*-Pr[1-C(=O)NH₂] | Me | Cl | CH₂OMe | O |
| D-14990 | Me | single bond | H | *c*-Pr[1-C(=O)NH₂] | Me | Cl | CH₂OMe | O |
| D-14991 | Me(R) | single bond | H | *c*-Pr[1-C(=O)NH₂] | Me | Cl | CH₂OMe | O |
| D-14992 | Me(S) | single bond | H | *c*-Pr[1-C(=O)NH₂] | Me | Cl | CH₂OMe | O |
| D-14993 | H | single bond | Me | *c*-Pr[1-C(=O)NH₂] | Me | Me | CH₂OMe | O |
| D-14994 | Me | single bond | H | *c*-Pr[1-C(=O)NH₂] | Me | Me | CH₂OMe | O |
| D-14995 | Me(R) | single bond | H | *c*-Pr[1-C(=O)NH₂] | Me | Me | CH₂OMe | O |
| D-14996 | Me(S) | single bond | H | *c*-Pr[1-C(=O)NH₂] | Me | Me | CH₂OMe | O |
| D-14997 | H | single bond | Me | *c*-Pr(1-OMe) | Me | Cl | CH₂OMe | O |
| D-14998 | Me | single bond | H | *c*-Pr(1-OMe) | Me | Cl | CH₂OMe | O |
| D-14999 | Me(R) | single bond | H | *c*-Pr(1-OMe) | Me | Cl | CH₂OMe | O |
| D-15000 | Me(S) | single bond | H | *c*-Pr(1-OMe) | Me | Cl | CH₂OMe | O |
| D-15001 | H | single bond | Me | *c*-Pr(1-OMe) | Me | Me | CH₂OMe | O |
| D-15002 | Me | single bond | H | *c*-Pr(1-OMe) | Me | Me | CH₂OMe | O |
| D-15003 | Me(R) | single bond | H | *c*-Pr(1-OMe) | Me | Me | CH₂OMe | O |
| D-15004 | Me(S) | single bond | H | *c*-Pr(1-OMe) | Me | Me | CH₂OMe | O |
| D-15005 | H | single bond | Me | *c*-Pr(1-SMe) | Me | Cl | CH₂OMe | O |
| D-15006 | Me | single bond | H | *c*-Pr(1-SMe) | Me | Cl | CH₂OMe | O |
| D-15007 | Me(R) | single bond | H | *c*-Pr(1-SMe) | Me | Cl | CH₂OMe | O |
| D-15008 | Me(S) | single bond | H | *c*-Pr(1-SMe) | Me | Cl | CH₂OMe | O |
| D-15009 | H | single bond | Me | *c*-Pr(1-SMe) | Me | Me | CH₂OMe | O |
| D-15010 | Me | single bond | H | *c*-Pr(1-SMe) | Me | Me | CH₂OMe | O |
| D-15011 | Me(R) | single bond | H | *c*-Pr(1-SMe) | Me | Me | CH₂OMe | O |
| D-15012 | Me(S) | single bond | H | *c*-Pr(1-SMe) | Me | Me | CH₂OMe | O |
| D-15013 | H | single bond | Me | *c*-Pr[1-S(=O)Me] | Me | Cl | CH₂OMe | O |
| D-15014 | Me | single bond | H | *c*-Pr[1-S(=O)Me] | Me | Cl | CH₂OMe | O |
| D-15015 | Me(R) | single bond | H | *c*-Pr[1-S(=O)Me] | Me | Cl | CH₂OMe | O |
| D-15016 | Me(S) | single bond | H | *c*-Pr[1-S(=O)Me] | Me | Cl | CH₂OMe | O |
| D-15017 | H | single bond | Me | *c*-Pr[1-S(=O)Me] | Me | Me | CH₂OMe | O |
| D-15018 | Me | single bond | H | *c*-Pr[1-S(=O)Me] | Me | Me | CH₂OMe | O |
| D-15019 | Me(R) | single bond | H | *c*-Pr[1-S(=O)Me] | Me | Me | CH₂OMe | O |
| D-15020 | Me(S) | single bond | H | *c*-Pr[1-S(=O)Me] | Me | Me | CH₂OMe | O |
| D-15021 | H | single bond | Me | *c*-Pr[1-S(=O)₂Me] | Me | Cl | CH₂OMe | O |
| D-15022 | Me | single bond | H | *c*-Pr[1-S(=O)₂Me] | Me | Cl | CH₂OMe | O |

**[Table 988]**

| Compound Number | Y | W | Z | R¹ | R² | R³ | R¹⁰ | A |
|---|---|---|---|---|---|---|---|---|
| D-15023 | Me(R) | single bond | H | *c*-Pr[1-S(=O)₂Me] | Me | Cl | CH₂OMe | O |
| D-15024 | Me(S) | single bond | H | *c*-Pr[1-S(=O)₂Me] | Me | Cl | CH₂OMe | O |
| D-15025 | H | single bond | Me | *c*-Pr[1-S(=O)₂Me] | Me | Me | CH₂OMe | O |
| D-15026 | Me | single bond | H | *c*-Pr[1-S(=O)₂Me] | Me | Me | CH₂OMe | O |
| D-15027 | Me(R) | single bond | H | *c*-Pr[1-S(=O)₂Me] | Me | Me | CH₂OMe | O |
| D-15028 | Me(S) | single bond | H | *c*-Pr[1-S(=O)₂Me] | Me | Me | CH₂OMe | O |
| D-15029 | H | single bond | Me | *c*-Pr[1-S(=O)₂CHF₂] | Me | Cl | CH₂OMe | O |
| D-15030 | Me | single bond | H | *c*-Pr[1-S(=O)₂CHF₂] | Me | Cl | CH₂OMe | O |
| D-15031 | Me(R) | single bond | H | *c*-Pr[1-S(=O)₂CHF₂] | Me | Cl | CH₂OMe | O |
| D-15032 | Me(S) | single bond | H | *c*-Pr[1-S(=O)₂CHF₂] | Me | Cl | CH₂OMe | O |
| D-15033 | H | single bond | Me | *c*-Pr[1-S(=O)₂CHF₂] | Me | Me | CH₂OMe | O |
| D-15034 | Me | single bond | H | *c*-Pr[1-S(=O)₂CHF₂] | Me | Me | CH₂OMe | O |
| D-15035 | Me(R) | single bond | H | *c*-Pr[1-S(=O)₂CHF₂] | Me | Me | CH₂OMe | O |
| D-15036 | Me(S) | single bond | H | *c*-Pr[1-S(=O)₂CHF₂] | Me | Me | CH₂OMe | O |
| D-15037 | H | single bond | Me | *c*-Pr[1-S(=O)₂CF₃] | Me | Cl | CH₂OMe | O |
| D-15038 | Me | single bond | H | *c*-Pr[1-S(=O)₂CF₃] | Me | Cl | CH₂OMe | O |
| D-15039 | Me(R) | single bond | H | *c*-Pr[1-S(=O)₂CF₃] | Me | Cl | CH₂OMe | O |
| D-15040 | Me(S) | single bond | H | *c*-Pr[1-S(=O)₂CF₃] | Me | Cl | CH₂OMe | O |
| D-15041 | H | single bond | Me | *c*-Pr[1-S(=O)₂CF₃] | Me | Me | CH₂OMe | O |
| D-15042 | Me | single bond | H | *c*-Pr[1-S(=O)₂CF₃] | Me | Me | CH₂OMe | O |
| D-15043 | Me(R) | single bond | H | *c*-Pr[1-S(=O)₂CF₃] | Me | Me | CH₂OMe | O |
| D-15044 | Me(S) | single bond | H | *c*-Pr[1-S(=O)₂CF₃] | Me | Me | CH₂OMe | O |
| D-15045 | H | single bond | Me | *c*-Pr[1-(5,6-dihydro-1,4,2-dioxazin-3-yl)] | Me | Cl | CH₂OMe | O |
| D-15046 | Me | single bond | H | *c*-Pr[1-(5,6-dihydro-1,4,2-dioxazin-3-yl)] | Me | Cl | CH₂OMe | O |
| D-15047 | Me(R) | single bond | H | *c*-Pr[1-(5,6-dihydro-1,4,2-dioxazin-3-yl)] | Me | Cl | CH₂OMe | O |
| D-15048 | Me(S) | single bond | H | *c*-Pr[1-(5,6-dihydro-1,4,2-dioxazin-3-yl)] | Me | Cl | CH₂OMe | O |
| D-15049 | H | single bond | Me | *c*-Pr[1-(5,6-dihydro-1,4,2-dioxazin-3-yl)] | Me | Me | CH₂OMe | O |
| D-15050 | Me | single bond | H | *c*-Pr[1-(5,6-dihydro-1,4,2-dioxazin-3-yl)] | Me | Me | CH₂OMe | O |
| D-15051 | Me(R) | single bond | H | *c*-Pr[1-(5,6-dihydro-1,4,2-dioxazin-3-yl)] | Me | Me | CH₂OMe | O |
| D-15052 | Me(S) | single bond | H | *c*-Pr[1-(5,6-dihydro-1,4,2-dioxazin-3-yl)] | Me | Me | CH₂OMe | O |
| D-15053 | H | single bond | Me | *c*-Pr[1-(5-hydroxy-1,3,4-oxadiazol-2-yl)] | Me | Cl | CH₂OMe | O |
| D-15054 | Me | single bond | H | *c*-Pr[1-(5-hydroxy-1,3,4-oxadiazol-2-yl)] | Me | Cl | CH₂OMe | O |
| D-15055 | Me(R) | single bond | H | *c*-Pr[1-(5-hydroxy-1,3,4-oxadiazol-2-yl)] | Me | Cl | CH₂OMe | O |
| D-15056 | Me(S) | single bond | H | *c*-Pr[1-(5-hydroxy-1,3,4-oxadiazol-2-yl)] | Me | Cl | CH₂OMe | O |
| D-15057 | H | single bond | Me | *c*-Pr[1-(5-hydroxy-1,3,4-oxadiazol-2-yl)] | Me | Me | CH₂OMe | O |
| D-15058 | Me | single bond | H | *c*-Pr[1-(5-hydroxy-1,3,4-oxadiazol-2-yl)] | Me | Me | CH₂OMe | O |
| D-15059 | Me(R) | single bond | H | *c*-Pr[1-(5-hydroxy-1,3,4-oxadiazol-2-yl)] | Me | Me | CH₂OMe | O |
| D-15060 | Me(S) | single bond | H | *c*-Pr[1-(5-hydroxy-1,3,4-oxadiazol-2-yl)] | Me | Me | CH₂OMe | O |
| D-15061 | H | single bond | Me | *c*-Pr[1-(pyrimidin-2-yl)] | Me | Cl | CH₂OMe | O |
| D-15062 | Me | single bond | H | *c*-Pr[1-(pyrimidin-2-yl)] | Me | Cl | CH₂OMe | O |
| D-15063 | Me(R) | single bond | H | *c*-Pr[1-(pyrimidin-2-yl)] | Me | Cl | CH₂OMe | O |
| D-15064 | Me(S) | single bond | H | *c*-Pr[1-(pyrimidin-2-yl)] | Me | Cl | CH₂OMe | O |
| D-15065 | H | single bond | Me | *c*-Pr[1-(pyrimidin-2-yl)] | Me | Me | CH₂OMe | O |
| D-15066 | Me | single bond | H | *c*-Pr[1-(pyrimidin-2-yl)] | Me | Me | CH₂OMe | O |

**[Table 989]**

| Compound Number | Y | W | Z | R¹ | R² | R³ | R¹⁰ | A |
|---|---|---|---|---|---|---|---|---|
| D-15067 | Me(R) | single bond | H | *c*-Pr[1-(pyrimidin-2-yl)] | Me | Me | CH₂OMe | O |
| D-15068 | Me(S) | single bond | H | *c*-Pr[1-(pyrimidin-2-yl)] | Me | Me | CH₂OMe | O |
| D-15069 | H | single bond | Me | *t*-Bu | Me | Cl | CH₂OMe | O |
| D-15070 | Me | single bond | H | *t*-Bu | Me | Cl | CH₂OMe | O |
| D-15071 | Me(R) | single bond | H | *t*-Bu | Me | Cl | CH₂OMe | O |
| D-15072 | Me(S) | single bond | H | *t*-Bu | Me | Cl | CH₂OMe | O |
| D-15073 | H | single bond | Me | *t*-Bu | Me | Me | CH₂OMe | O |
| D-15074 | Me | single bond | H | *t*-Bu | Me | Me | CH₂OMe | O |
| D-15075 | Me(R) | single bond | H | *t*-Bu | Me | Me | CH₂OMe | O |
| D-15076 | Me(S) | single bond | H | *t*-Bu | Me | Me | CH₂OMe | O |
| D-15077 | H | single bond | Me | *c*-Bu | Me | Cl | CH₂OMe | O |
| D-15078 | Me | single bond | H | *c*-Bu | Me | Cl | CH₂OMe | O |
| D-15079 | Me(R) | single bond | H | *c*-Bu | Me | Cl | CH₂OMe | O |
| D-15080 | Me(S) | single bond | H | *c*-Bu | Me | Cl | CH₂OMe | O |
| D-15081 | H | single bond | Me | *c*-Bu | Me | Me | CH₂OMe | O |
| D-15082 | Me | single bond | H | *c*-Bu | Me | Me | CH₂OMe | O |
| D-15083 | Me(R) | single bond | H | *c*-Bu | Me | Me | CH₂OMe | O |
| D-15084 | Me(S) | single bond | H | *c*-Bu | Me | Me | CH₂OMe | O |
| D-15085 | H | single bond | Me | *c*-Bu(1-CN) | Me | Cl | CH₂OMe | O |
| D-15086 | Me | single bond | H | *c*-Bu(1-CN) | Me | Cl | CH₂OMe | O |
| D-15087 | Me(R) | single bond | H | *c*-Bu(1-CN) | Me | Cl | CH₂OMe | O |
| D-15088 | Me(S) | single bond | H | *c*-Bu(1-CN) | Me | Cl | CH₂OMe | O |
| D-15089 | H | single bond | Me | *c*-Bu(1-CN) | Me | Me | CH₂OMe | O |
| D-15090 | Me | single bond | H | *c*-Bu(1-CN) | Me | Me | CH₂OMe | O |
| D-15091 | Me(R) | single bond | H | *c*-Bu(1-CN) | Me | Me | CH₂OMe | O |
| D-15002 | Me(S) | single bond | H | *c*-Bu(1-CN) | Me | Me | CH₂OMe | O |
| D-15093 | H | single bond | Me | *c*-Bu(3,3-F₂) | Me | Cl | CH₂OMe | O |
| D-15094 | Me | single bond | H | *c*-Bu(3,3-F₂) | Me | Cl | CH₂OMe | O |
| D-15095 | Me(R) | single bond | H | *c*-Bu(3,3-F₂) | Me | Cl | CH₂OMe | O |
| D-15096 | Me(S) | single bond | H | *c*-Bu(3,3-F₂) | Me | Cl | CH₂OMe | O |
| D-15097 | H | single bond | Me | *c*-Bu(3,3-F₂) | Me | Me | CH₂OMe | O |
| D-15098 | Me | single bond | H | *c*-Bu(3,3-F₂) | Me | Me | CH₂OMe | O |
| D-15099 | Me(R) | single bond | H | *c*-Bu(3,3-F₂) | Me | Me | CH₂OMe | O |
| D-15100 | Me(S) | single bond | H | *c*-Bu(3,3-F₂) | Me | Me | CH₂OMe | O |
| D-15101 | H | single bond | Me | oxetan-3-yl | Me | Cl | CH₂OMe | O |
| D-15102 | Me | single bond | H | oxetan-3-yl | Me | Cl | CH₂OMe | O |
| D-15103 | Me(R) | single bond | H | oxetan-3-yl | Me | Cl | CH₂OMe | O |
| D-15104 | Me(S) | single bond | H | oxetan-3-yl | Me | Cl | CH₂OMe | O |
| D-15105 | H | single bond | Me | oxetan-3-yl | Me | Me | CH₂OMe | O |
| D-15106 | Me | single bond | H | oxetan-3-yl | Me | Me | CH₂OMe | O |
| D-15107 | Me(R) | single bond | H | oxetan-3-yl | Me | Me | CH₂OMe | O |
| D-15108 | Me(S) | single bond | H | oxetan-3-yl | Me | Me | CH₂OMe | O |
| D-15109 | H | single bond | Me | oxetan(3-CN)-3-yl | Me | Cl | CH₂OMe | O |
| D-15110 | Me | single bond | H | oxetan(3-CN)-3-yl | Me | Cl | CH₂OMe | O |

**[Table 990]**

| Compound Number | Y | W | Z | R¹ | R² | R³ | R¹⁰ | A |
|---|---|---|---|---|---|---|---|---|
| D-15111 | Me(R) | single bond | H | oxetan(3-CN)-3-yl | Me | Cl | CH₂OMe | O |
| D-15112 | Me(S) | single bond | H | oxetan(3-CN)-3-yl | Me | Cl | CH₂OMe | O |
| D-15113 | H | single bond | Me | oxetan(3-CN)-3-yl | Me | Me | CH₂OMe | O |
| D-15114 | Me | single bond | H | oxetan(3-CN)-3-yl | Me | Me | CH₂OMe | O |
| D-15115 | Me(R) | single bond | H | oxetan(3-CN)-3-yl | Me | Me | CH₂OMe | O |
| D-15116 | Me(S) | single bond | H | oxetan(3-CN)-3-yl | Me | Me | CH₂OMe | O |
| D-15117 | H | single bond | Me | azetidin[1-C(=O)Me]-3-yl | Me | Cl | CH₂OMe | O |
| D-15118 | Me | single bond | H | azetidin[1-C(=O)Me]-3-yl | Me | Cl | CH₂OMe | O |
| D-15119 | Me(R) | single bond | H | azetidin[1-C(=O)Me]-3-yl | Me | Cl | CH₂OMe | O |
| D-15120 | Me(S) | single bond | H | azetidin[1-C(=O)Me]-3-yl | Me | Cl | CH₂OMe | O |
| D-15121 | H | single bond | Me | azetidin[1-C(=O)Me]-3-yl | Me | Me | CH₂OMe | O |
| D-15122 | Me | single bond | H | azetidin[1-C(=O)Me]-3-yl | Me | Me | CH₂OMe | O |
| D-15123 | Me(R) | single bond | H | azetidin[1-C(=O)Me]-3-yl | Me | Me | CH₂OMe | O |
| D-15124 | Me(S) | single bond | H | azetidin[1-C(=O)Me]-3-yl | Me | Me | CH₂OMe | O |
| D-15125 | H | single bond | Me | azetidin[1-S(=O)₂Me]-3-yl | Me | Cl | CH₂OMe | O |
| D-15126 | Me | single bond | H | azetidin[1-S(=O)₂Me]-3-yl | Me | Cl | CH₂OMe | O |
| D-15127 | Me(R) | single bond | H | azetidin[1-S(=O)₂Me]-3-yl | Me | Cl | CH₂OMe | O |
| D-15128 | Me(S) | single bond | H | azetidin[1-S(=O)₂Me]-3-yl | Me | Cl | CH₂OMe | O |
| D-15129 | H | single bond | Me | azetidin[1-S(=O)₂Me]-3-yl | Me | Me | CH₂OMe | O |
| D-15130 | Me | single bond | H | azetidin[1-S(=O)₂Me]-3-yl | Me | Me | CH₂OMe | O |
| D-15131 | Me(R) | single bond | H | azetidin[1-S(=O)₂Me]-3-yl | Me | Me | CH₂OMe | O |
| D-15132 | Me(S) | single bond | H | azetidin[1-S(=O)₂Me]-3-yl | Me | Me | CH₂OMe | O |
| D-15133 | H | single bond | Me | azetidin[1-S(=O)₂CHF₂]-3-yl | Me | Cl | CH₂OMe | O |
| D-15134 | Me | single bond | H | azetidin[1-S(=O)₂CHF₂]-3-yl | Me | Cl | CH₂OMe | O |
| D-15135 | Me(R) | single bond | H | azetidin[1-S(=O)₂CHF₂]-3-yl | Me | Cl | CH₂OMe | O |
| D-15136 | Me(S) | single bond | H | azetidin[1-S(=O)₂CHF₂]-3-yl | Me | Cl | CH₂OMe | O |
| D-15137 | H | single bond | Me | azetidin[1-S(=O)₂CHF₂]-3-yl | Me | Me | CH₂OMe | O |
| D-15138 | Me | single bond | H | azetidin[1-S(=O)₂CHF₂]-3-yl | Me | Me | CH₂OMe | O |
| D-15139 | Me(R) | single bond | H | azetidin[1-S(=O)₂CHF₂]-3-yl | Me | Me | CH₂OMe | O |
| D-15140 | Me(S) | single bond | H | azetidin[1-S(=O)₂CHF₂]-3-yl | Me | Me | CH₂OMe | O |
| D-15141 | H | single bond | Me | *c*-Pen | Me | Cl | CH₂OMe | O |
| D-15142 | Me | single bond | H | *c*-Pen | Me | Cl | CH₂OMe | O |
| D-15143 | Me(R) | single bond | H | *c*-Pen | Me | Cl | CH₂OMe | O |
| D-15144 | Me(S) | single bond | H | *c*-Pen | Me | Cl | CH₂OMe | O |
| D-15145 | H | single bond | Me | *c*-Pen | Me | Me | CH₂OMe | O |
| D-15146 | Me | single bond | H | *c*-Pen | Me | Me | CH₂OMe | O |
| D-15147 | Me(R) | single bond | H | *c*-Pen | Me | Me | CH₂OMe | O |
| D-15148 | Me(S) | single bond | H | *c*-Pen | Me | Me | CH₂OMe | O |
| D-15149 | H | single bond | Me | tetrahydrofuran-3-yl | Me | Cl | CH₂OMe | O |
| D-15150 | Me | single bond | H | tetrahydrofuran-3-yl | Me | Cl | CH₂OMe | O |
| D-15151 | Me(R) | single bond | H | tetrahydrofuran-3-yl | Me | Cl | CH₂OMe | O |
| D-15152 | Me(S) | single bond | H | tetrahydrofuran-3-yl | Me | Cl | CH₂OMe | O |
| D-15153 | H | single bond | Me | tetrahydrofuran-3-yl | Me | Me | CH₂OMe | O |
| D-15154 | Me | single bond | H | tetrahydrofuran-3-yl | Me | Me | CH₂OMe | O |

**[Table 991]**

| Compound Number | Y | W | Z | R¹ | R² | R³ | R¹⁰ | A |
|---|---|---|---|---|---|---|---|---|
| D-15155 | Me(R) | single bond | H | tetrahydrofuran-3-yl | Me | Me | CH₂OMe | O |
| D-15156 | Me(S) | single bond | H | tetrahydrofuran-3-yl | Me | Me | CH₂OMe | O |
| D-15157 | H | single bond | Me | tetrahydrothiophen-3-yl | Me | Cl | CH₂OMe | O |
| D-15158 | Me | single bond | H | tetrahydrothiophen-3-yl | Me | Cl | CH₂OMe | O |
| D-15159 | Me(R) | single bond | H | tetrahydrothiophen-3-yl | Me | Cl | CH₂OMe | O |
| D-15160 | Me(S) | single bond | H | tetrahydrothiophen-3-yl | Me | Cl | CH₂OMe | O |
| D-15161 | H | single bond | Me | tetrahydrothiophen-3-yl | Me | Me | CH₂OMe | O |
| D-15162 | Me | single bond | H | tetrahydrothiophen-3-yl | Me | Me | CH₂OMe | O |
| D-15163 | Me(R) | single bond | H | tetrahydrothiophen-3-yl | Me | Me | CH₂OMe | O |
| D-15164 | Me(S) | single bond | H | tetrahydrothiophen-3-yl | Me | Me | CH₂OMe | O |
| D-15165 | H | single bond | Me | *c*-Pen(1-CN) | Me | Cl | CH₂OMe | O |
| D-15166 | Me | single bond | H | *c*-Pen(1-CN) | Me | Cl | CH₂OMe | O |
| D-15167 | Me(R) | single bond | H | *c*-Pen(1-CN) | Me | Cl | CH₂OMe | O |
| D-15168 | Me(S) | single bond | H | *c*-Pen(1-CN) | Me | Cl | CH₂OMe | O |
| D-15169 | H | single bond | Me | *c*-Pen(1-CN) | Me | Me | CH₂OMe | O |
| D-15170 | Me | single bond | H | *c*-Pen(1-CN) | Me | Me | CH₂OMe | O |
| D-15171 | Me(R) | single bond | H | *c*-Pen(1-CN) | Me | Me | CH₂OMe | O |
| D-15172 | Me(S) | single bond | H | *c*-Pen(1-CN) | Me | Me | CH₂OMe | O |
| D-15173 | H | single bond | Me | tetrahydrofuran(3-CN)-3-yl | Me | Cl | CH₂OMe | O |
| D-15174 | Me | single bond | H | tetrahydrofuran(3-CN)-3-yl | Me | Cl | CH₂OMe | O |
| D-15175 | Me(R) | single bond | H | tetrahydrofuran(3-CN)-3-yl | Me | Cl | CH₂OMe | O |
| D-15176 | Me(S) | single bond | H | tetrahydrofuran(3-CN)-3-yl | Me | Cl | CH₂OMe | O |
| D-15177 | H | single bond | Me | tetrahydrofuran(3-CN)-3-yl | Me | Me | CH₂OMe | O |
| D-15178 | Me | single bond | H | tetrahydrofuran(3-CN)-3-yl | Me | Me | CH₂OMe | O |
| D-15179 | Me(R) | single bond | H | tetrahydrofuran(3-CN)-3-yl | Me | Me | CH₂OMe | O |
| D-15180 | Me(S) | single bond | H | tetrahydrofuran(3-CN)-3-yl | Me | Me | CH₂OMe | O |
| D-15181 | H | single bond | Me | tetrahydrothiophen(3-CN)-3-yl | Me | Cl | CH₂OMe | O |
| D-15182 | Me | single bond | H | tetrahydrothiophen(3-CN)-3-yl | Me | Cl | CH₂OMe | O |
| D-15183 | Me(R) | single bond | H | tetrahydrothiophen(3-CN)-3-yl | Me | Cl | CH₂OMe | O |
| D-15184 | Me(S) | single bond | H | tetrahydrothiophen(3-CN)-3-yl | Me | Cl | CH₂OMe | O |
| D-15185 | H | single bond | Me | tetrahydrothiophen(3-CN)-3-yl | Me | Me | CH₂OMe | O |
| D-15186 | Me | single bond | H | tetrahydrothiophen(3-CN)-3-yl | Me | Me | CH₂OMe | O |
| D-15187 | Me(R) | single bond | H | tetrahydrothiophen(3-CN)-3-yl | Me | Me | CH₂OMe | O |
| D-15188 | Me(S) | single bond | H | tetrahydrothiophen(3-CN)-3-yl | Me | Me | CH₂OMe | O |
| D-15189 | H | single bond | Me | bicyclo[1.1.1]pertan-1-yl | Me | Cl | CH₂OMe | O |
| D-15190 | Me | single bond | H | bicyclo[1.1.1]pertan-1-yl | Me | Cl | CH₂OMe | O |
| D-15191 | Me(R) | single bond | H | bicyclo[1.1.1]pertan-1-yl | Me | Cl | CH₂OMe | O |
| D-15192 | Me(S) | single bond | H | bicyclo[1.1.1]pertan-1-yl | Me | Cl | CH₂OMe | O |
| D-15193 | H | single bond | Me | bicyclo[1.1.1]pentan-1-yl | Me | Me | CH₂OMe | O |
| D-15194 | Me | single bond | H | bicyclo[1.1.1]pentan-1-yl | Me | Me | CH₂OMe | O |
| D-15195 | Me(R) | single bond | H | bicyclo[1.1.1]pertan-1-yl | Me | Me | CH₂OMe | O |
| D-15196 | Me(S) | single bond | H | bicyclo[1.1.1]pertan-1-yl | Me | Me | CH₂OMe | O |
| D-15197 | H | single bond | Me | *c*-Hex | Me | Cl | CH₂OMe | O |
| D-15198 | Me | single bond | H | *c*-Hex | Me | Cl | CH₂OMe | O |

**[Table 992]**

| Compound Number | Y | W | Z | R¹ | R² | R³ | R¹⁰ | A |
|---|---|---|---|---|---|---|---|---|
| D-15199 | Me(R) | single bond | H | *c*-Hex | Me | Cl | CH₂OMe | O |
| D-15200 | Me(S) | single bond | H | *c*-Hex | Me | Cl | CH₂OMe | O |
| D-15201 | H | single bond | Me | *c*-Hex | Me | Me | CH₂OMe | O |
| D-15202 | Me | single bond | H | *c*-Hex | Me | Me | CH₂OMe | O |
| D-15203 | Me(R) | single bond | H | *c*-Hex | Me | Me | CH₂OMe | O |
| D-15204 | Me(S) | single bond | H | *c*-Hex | Me | Me | CH₂OMe | O |
| D-15205 | H | single bond | Me | tetrahydropyran-4-yl | Me | Cl | CH₂OMe | O |
| D-15206 | Me | single bond | H | tetrahydropyran-4-yl | Me | Cl | CH₂OMe | O |
| D-15207 | Me(R) | single bond | H | tetrahydropyran-4-yl | Me | Cl | CH₂OMe | O |
| D-15208 | Me(S) | single bond | H | tetrahydropyran-4-yl | Me | Cl | CH₂OMe | O |
| D-15209 | H | single bond | Me | tetrahydropyran-4-yl | Me | Me | CH₂OMe | O |
| D-15210 | Me | single bond | H | tetrahydropyran-4-yl | Me | Me | CH₂OMe | O |
| D-15211 | Me(R) | single bond | H | tetrahydropyran-4-yl | Me | Me | CH₂OMe | O |
| D-15212 | Me(S) | single bond | H | tetrahydropyran-4-yl | Me | Me | CH₂OMe | O |
| D-15213 | H | single bond | Me | tetrahydrothiopyran-4-yl | Me | Cl | CH₂OMe | O |
| D-15214 | Me | single bond | H | tetrahydrothiopyran-4-yl | Me | Cl | CH₂OMe | O |
| D-15215 | Me(R) | single bond | H | tetrahydrothiopyran-4-yl | Me | Cl | CH₂OMe | O |
| D-15216 | Me(S) | single bond | H | tetrahydrothiopyran-4-yl | Me | Cl | CH₂OMe | O |
| D-15217 | H | single bond | Me | tetrahydrothiopyran-4-yl | Me | Me | CH₂OMe | O |
| D-15218 | Me | single bond | H | tetrahydrothiopyran-4-yl | Me | Me | CH₂OMe | O |
| D-15219 | Me(R) | single bond | H | tetrahydrothiopyran-4-yl | Me | Me | CH₂OMe | O |
| D-15220 | Me(S) | single bond | H | tetrahydrothiopyran-4-yl | Me | Me | CH₂OMe | O |
| D-15221 | H | single bond | Me | *c*-Hex(1-CN) | Me | Cl | CH₂OMe | O |
| D-15222 | Me | single bond | H | *c*-Hex(1-CN) | Me | Cl | CH₂OMe | O |
| D-15223 | Me(R) | single bond | H | c-Hex(1-CN) | Me | Cl | CH₂OMe | O |
| D-15224 | Me(S) | single bond | H | *c*-Hex(1-CN) | Me | Cl | CH₂OMe | O |
| D-15225 | H | single bond | Me | *c*-Hex(1-CN) | Me | Me | CH₂OMe | O |
| D-15226 | Me | single bond | H | *c*-Hex(1-CN) | Me | Me | CH₂OMe | O |
| D-15227 | Me(R) | single bond | H | *c*-Hex(1-CN) | Me | Me | CH₂OMe | O |
| D-15228 | Me(S) | single bond | H | *c*-Hex(1-CN) | Me | Me | CH₂OMe | O |
| D-15229 | H | single bond | Me | tetrahydropyran(4-CN)-4-yl | Me | Cl | CH₂OMe | O |
| D-15230 | Me | single bond | H | tetrahydropyran(4-CN)-4-yl | Me | Cl | CH₂OMe | O |
| D-15231 | Me(R) | single bond | H | tetrahydropyran(4-CN)-4-yl | Me | Cl | CH₂OMe | O |
| D-15232 | Me(S) | single bond | H | tetrahydropyran(4-CN)-4-yl | Me | Cl | CH₂OMe | O |
| D-15233 | H | single bond | Me | tetrahydropyran(4-CN)-4-yl | Me | Me | CH₂OMe | O |
| D-15234 | Me | single bond | H | tetrahydropyran(4-CN)-4-yl | Me | Me | CH₂OMe | O |
| D-15235 | Me(R) | single bond | H | tetrahydropyran(4-CN)-4-yl | Me | Me | CH₂OMe | O |
| D-15236 | Me(S) | single bond | H | tetrahydropyran(4-CN)-4-yl | Me | Me | CH₂OMe | O |
| D-15237 | H | single bond | Me | tetrahydrothiopyran(4-CN)-4-yl | Me | Cl | CH₂OMe | O |
| D-15238 | Me | single bond | H | tetrahydrothiopyran(4-CN)-4-yl | Me | Cl | CH₂OMe | O |
| D-15239 | Me(R) | single bond | H | tetrahydrothiopyran(4-CN)-4-yl | Me | Cl | CH₂OMe | O |
| D-15240 | Me(S) | single bond | H | tetrahydrothiopyran(4-CN)-4-yl | Me | Cl | CH₂OMe | O |
| D-15241 | H | single bond | Me | tetrahydrothiopyran(4-CN)-4-yl | Me | Me | CH₂OMe | O |
| D-15242 | Me | single bond | H | tetrahydrothiopyran(4-CN)-4-yl | Me | Me | CH₂OMe | O |

**[Table 993]**

| Compound Number | Y | W | Z | R¹ | R² | R³ | R¹⁰ | A |
|---|---|---|---|---|---|---|---|---|
| D-15243 | Me(R) | single bond | H | tetrahydrothiopyran(4-CN)-4-yl | Me | Me | CH₂OMe | O |
| D-15244 | Me(S) | single bond | H | tetrahydrothiopyran(4-CN)-4-yl | Me | Me | CH₂OMe | O |
| D-15245 | H | single bond | Me | CH₂(*c*-Pr) | Me | Cl | CH₂OMe | O |
| D-15246 | Me | single bond | H | CH₂(*c*-Pr) | Me | Cl | CH₂OMe | O |
| D-15247 | Me(R) | single bond | H | CH₂(*c*-Pr) | Me | Cl | CH₂OMe | O |
| D-15248 | Me(S) | single bond | H | CH₂(*c*-Pr) | Me | Cl | CH₂OMe | O |
| D-15249 | H | single bond | Me | CH₂(*c*-Pr) | Me | Me | CH₂OMe | O |
| D-15250 | Me | single bond | H | CH₂(*c*-Pr) | Me | Me | CH₂OMe | O |
| D-15251 | Me(R) | single bond | H | CH₂(*c*-Pr) | Me | Me | CH₂OMe | O |
| D-15252 | Me(S) | single bond | H | CH₂(*c*-Pr) | Me | Me | CH₂OMe | O |
| D-15253 | H | single bond | Me | CH₂[*c*-Pr(1-CN)] | Me | Cl | CH₂OMe | O |
| D-15254 | Me | single bond | H | CH₂[*c*-Pr(1-CN)] | Me | Cl | CH₂OMe | O |
| D-15255 | Me(R) | single bond | H | CH₂[*c*-Pr(1-CN)] | Me | Cl | CH₂OMe | O |
| D-15256 | Me(S) | single bond | H | CH₂[*c*-Pr(1-CN)] | Me | Cl | CH₂OMe | O |
| D-15257 | H | single bond | Me | CH₂[*c*-Pr(1-CN)] | Me | Me | CH₂OMe | O |
| D-15258 | Me | single bond | H | CH₂[*c*-Pr(1-CN)] | Me | Me | CH₂OMe | O |
| D-15259 | Me(R) | single bond | H | CH₂[*c*-Pr(1-CN)] | Me | Me | CH₂OMe | O |
| D-15260 | Me(S) | single bond | H | CH₂[*c*-Pr(1-CN)] | Me | Me | CH₂OMe | O |
| D-15261 | H | single bond | Me | CH(Me)(*c*-Pr) | Me | Cl | CH₂OMe | O |
| D-15262 | Me | single bond | H | CH(Me)(*c*-Pr) | Me | Cl | CH₂OMe | O |
| D-15263 | Me(R) | single bond | H | CH(Me)(*c*-Pr) | Me | Cl | CH₂OMe | O |
| D-15264 | Me(S) | single bond | H | CH(Me)(*c*-Pr) | Me | Cl | CH₂OMe | O |
| D-15265 | H | single bond | Me | CH(Me)(*c*-Pr) | Me | Me | CH₂OMe | O |
| D-15266 | Me | single bond | H | CH(Me)(*c*-Pr) | Me | Me | CH₂OMe | O |
| D-15267 | Me(R) | single bond | H | CH(Me)(*c*-Pr) | Me | Me | CH₂OMe | O |
| D-15268 | Me(S) | single bond | H | CH(Me)(*c*-Pr) | Me | Me | CH₂OMe | O |
| D-15269 | H | single bond | Me | CH(Me)CH₂OMe | Me | Cl | CH₂OMe | O |
| D-15270 | Me | single bond | H | CH(Me)CH₂OMe | Me | Cl | CH₂OMe | O |
| D-15271 | Me(R) | single bond | H | CH(Me)CH₂OMe | Me | Cl | CH₂OMe | O |
| D-15272 | Me(S) | single bond | H | CH(Me)CH₂OMe | Me | Cl | CH₂OMe | O |
| D-15273 | H | single bond | Me | CH(Me)CH₂OMe | Me | Me | CH₂OMe | O |
| D-15274 | Me | single bond | H | CH(Me)CH₂OMe | Me | Me | CH₂OMe | O |
| D-15275 | Me(R) | single bond | H | CH(Me)CH₂OMe | Me | Me | CH₂OMe | O |
| D-15276 | Me(S) | single bond | H | CH(Me)CH₂OMe | Me | Me | CH₂OMe | O |
| D-15277 | H | single bond | Me | CH₂CH=CH₂ | Me | Cl | CH₂OMe | O |
| D-15278 | Me | single bond | H | CH₂CH=CH₂ | Me | Cl | CH₂OMe | O |
| D-15279 | Me(R) | single bond | H | CH₂CH=CH₂ | Me | Cl | CH₂OMe | O |
| D-15280 | Me(S) | single bond | H | CH₂CH=CH₂ | Me | Cl | CH₂OMe | O |
| D-15281 | H | single bond | Me | CH₂CH=CH₂ | Me | Me | CH₂OMe | O |
| D-15282 | Me | single bond | H | CH₂CH=CH₂ | Me | Me | CH₂OMe | O |
| D-15283 | Me(R) | single bond | H | CH₂CH=CH₂ | Me | Me | CH₂OMe | O |
| D-15284 | Me(S) | single bond | H | CH₂CH=CH₂ | Me | Me | CH₂OMe | O |
| D-15285 | H | single bond | Me | CH₂C≡CH | Me | Cl | CH₂OMe | O |
| D-15286 | Me | single bond | H | CH₂C≡CH | Me | Cl | CH₂OMe | O |

**[Table 994]**

| Compound Number | Y | W | Z | R¹ | R² | R³ | R¹⁰ | A |
|---|---|---|---|---|---|---|---|---|
| D-15287 | Me(R) | single bond | H | CH₂C≡CH | Me | Cl | CH₂OMe | O |
| D-15288 | Me(S) | single bond | H | CH₂C≡CH | Me | Cl | CH₂OMe | O |
| D-15289 | H | single bond | Me | CH₂C≡CH | Me | Me | CH₂OMe | O |
| D-15290 | Me | single bond | H | CH₂C≡CH | Me | Me | CH₂OMe | O |
| D-15291 | Me(R) | single bond | H | CH₂C≡CH | Me | Me | CH₂OMe | O |
| D-15292 | Me(S) | single bond | H | CH₂C≡CH | Me | Me | CH₂OMe | O |
| D-15293 | H | single bond | Me | CH₂CN | Me | Cl | CH₂OMe | O |
| D-15294 | Me | single bond | H | CH₂CN | Me | Cl | CH₂OMe | O |
| D-15295 | Me(R) | single bond | H | CH₂CN | Me | Cl | CH₂OMe | O |
| D-15296 | Me(S) | single bond | H | CH₂CN | Me | Cl | CH₂OMe | O |
| D-15297 | H | single bond | Me | CH₂CN | Me | Me | CH₂OMe | O |
| D-15298 | Me | single bond | H | CH₂CN | Me | Me | CH₂OMe | O |
| D-15299 | Me(R) | single bond | H | CH₂CN | Me | Me | CH₂OMe | O |
| D-15300 | Me(S) | single bond | H | CH₂CN | Me | Me | CH₂OMe | O |
| D-15301 | H | single bond | Me | CH(Me)CN | Me | Cl | CH₂OMe | O |
| D-15302 | Me | single bond | H | CH(Me)CN | Me | Cl | CH₂OMe | O |
| D-15303 | Me(R) | single bond | H | CH(Me)CN | Me | Cl | CH₂OMe | O |
| D-15304 | Me(S) | single bond | H | CH(Me)CN | Me | Cl | CH₂OMe | O |
| D-15305 | H | single bond | Me | CH(Me)CN | Me | Me | CH₂OMe | O |
| D-15306 | Me | single bond | H | CH(Me)CN | Me | Me | CH₂OMe | O |
| D-15307 | Me(R) | single bond | H | CH(Me)CN | Me | Me | CH₂OMe | O |
| D-15308 | Me(S) | single bond | H | CH(Me)CN | Me | Me | CH₂OMe | O |
| D-15309 | H | single bond | Me | C(Me)₂CN | Me | Cl | CH₂OMe | O |
| D-15310 | Me | single bond | H | C(Me)₂CN | Me | Cl | CH₂OMe | O |
| D-15311 | Me(R) | single bond | H | C(Me)₂CN | Me | Cl | CH₂OMe | O |
| D-15312 | Me(S) | single bond | H | C(Me)₂CN | Me | Cl | CH₂OMe | O |
| D-15313 | H | single bond | Me | C(Me)₂CN | Me | Me | CH₂OMe | O |
| D-15314 | Me | single bond | H | C(Me)₂CN | Me | Me | CH₂OMe | O |
| D-15315 | Me(R) | single bond | H | C(Me)₂CN | Me | Me | CH₂OMe | O |
| D-15316 | Me(S) | single bond | H | C(Me)₂CN | Me | Me | CH₂OMe | O |
| D-15317 | H | single bond | Me | C(Me)(*i*-Pr)CN | Me | Cl | CH₂OMe | O |
| D-15318 | Me | single bond | H | C(Me)(*i*-Pr)CN | Me | Cl | CH₂OMe | O |
| D-15319 | Me(R) | single bond | H | C(Me)(*i*-Pr)CN | Me | Cl | CH₂OMe | O |
| D-15320 | Me(S) | single bond | H | C(Me)(*i*-Pr)CN | Me | Cl | CH₂OMe | O |
| D-15321 | H | single bond | Me | C(Me)(*i*-Pr)CN | Me | Me | CH₂OMe | O |
| D-15322 | Me | single bond | H | C(Me)(*i*-Pr)CN | Me | Me | CH₂OMe | O |
| D-15323 | Me(R) | single bond | H | C(Me)(*i*-Pr)CN | Me | Me | CH₂OMe | O |
| D-15324 | Me(S) | single bond | H | C(Me)(*i*-Pr)CN | Me | Me | CH₂OMe | O |
| D-15325 | H | single bond | Me | OMe | Me | Cl | CH₂OMe | O |
| D-15326 | Me | single bond | H | OMe | Me | Cl | CH₂OMe | O |
| D-15327 | Me(R) | single bond | H | OMe | Me | Cl | CH₂OMe | O |
| D-15328 | Me(S) | single bond | H | OMe | Me | Cl | CH₂OMe | O |
| D-15329 | H | single bond | Me | OMe | Me | Me | CH₂OMe | O |
| D-15330 | Me | single bond | H | OMe | Me | Me | CH₂OMe | O |

**[Table 995]**

| Compound Number | Y | W | Z | R¹ | R² | R³ | R¹⁰ | A |
|---|---|---|---|---|---|---|---|---|
| D-15331 | Me(R) | single bond | H | OMe | Me | Me | CH₂OMe | O |
| D-15332 | Me(S) | single bond | H | OMe | Me | Me | CH₂OMe | O |
| D-15333 | H | single bond | Me | CH=NOMe | Me | Cl | CH₂OMe | O |
| D-15334 | Me | single bond | H | CH=NOMe | Me | Cl | CH₂OMe | O |
| D-15335 | Me(R) | single bond | H | CH=NOMe | Me | Cl | CH₂OMe | O |
| D-15336 | Me(S) | single bond | H | CH=NOMe | Me | Cl | CH₂OMe | O |
| D-15337 | H | single bond | Me | CH=NOMe | Me | Me | CH₂OMe | O |
| D-15338 | Me | single bond | H | CH=NOMe | Me | Me | CH₂OMe | O |
| D-15339 | Me(R) | single bond | H | CH=NOMe | Me | Me | CH₂OMe | O |
| D-15340 | Me(S) | single bond | H | CH=NOMe | Me | Me | CH₂OMe | O |
| D-15341 | H | single bond | Me | morpholin-4-yl | Me | Cl | CH₂OMe | O |
| D-15342 | Me | single bond | H | morpholin-4-yl | Me | Cl | CH₂OMe | O |
| D-15343 | Me(R) | single bond | H | morpholin-4-yl | Me | Cl | CH₂OMe | O |
| D-15344 | Me(S) | single bond | H | morpholin-4-yl | Me | Cl | CH₂OMe | O |
| D-15345 | H | single bond | Me | morpholin-4-yl | Me | Me | CH₂OMe | O |
| D-15346 | Me | single bond | H | morpholin-4-yl | Me | Me | CH₂OMe | O |
| D-15347 | Me(R) | single bond | H | morpholin-4-yl | Me | Me | CH₂OMe | O |
| D-15348 | Me(S) | single bond | H | morpholin-4-yl | Me | Me | CH₂OMe | O |
| D-15349 | H | single bond | Me | Ph | Me | Cl | CH₂OMe | O |
| D-15350 | Me | single bond | H | Ph | Me | Cl | CH₂OMe | O |
| D-15351 | Me(R) | single bond | H | Ph | Me | Cl | CH₂OMe | O |
| D-15352 | Me(S) | single bond | H | Ph | Me | Cl | CH₂OMe | O |
| D-15353 | H | single bond | Me | Ph | Me | Me | CH₂OMe | O |
| D-15354 | Me | single bond | H | Ph | Me | Me | CH₂OMe | O |
| D-15355 | Me(R) | single bond | H | Ph | Me | Me | CH₂OMe | O |
| D-15356 | Me(S) | single bond | H | Ph | Me | Me | CH₂OMe | O |
| D-15357 | H | single bond | Me | CH₂Ph | Me | Cl | CH₂OMe | O |
| D-15358 | Me | single bond | H | CH₂Ph | Me | Cl | CH₂OMe | O |
| D-15359 | Me(R) | single bond | H | CH₂Ph | Me | Cl | CH₂OMe | O |
| D-15360 | Me(S) | single bond | H | CH₂Ph | Me | Cl | CH₂OMe | O |
| D-15361 | H | single bond | Me | CH₂Ph | Me | Me | CH₂OMe | O |
| D-15362 | Me | single bond | H | CH₂Ph | Me | Me | CH₂OMe | O |
| D-15363 | Me(R) | single bond | H | CH₂Ph | Me | Me | CH₂OMe | O |
| D-15364 | Me(S) | single bond | H | CH₂Ph | Me | Me | CH₂OMe | O |
| D-15365 | H | single bond | Me | thiazole-2-yl | Me | Cl | CH₂OMe | O |
| D-15366 | Me | single bond | H | thiazole-2-yl | Me | Cl | CH₂OMe | O |
| D-15367 | Me(R) | single bond | H | thiazole-2-yl | Me | Cl | CH₂OMe | O |
| D-15368 | Me(S) | single bond | H | thiazole-2-yl | Me | Cl | CH₂OMe | O |
| D-15369 | H | single bond | Me | thiazole-2-yl | Me | Me | CH₂OMe | O |
| D-15370 | Me | single bond | H | thiazole-2-yl | Me | Me | CH₂OMe | O |
| D-15371 | Me(R) | single bond | H | thiazole-2-yl | Me | Me | CH₂OMe | O |
| D-15372 | Me(S) | single bond | H | thiazole-2-yl | Me | Me | CH₂OMe | O |
| D-15373 | H | single bond | Me | 1-Me-pyrazol-3-yl | Me | Cl | CH₂OMe | O |
| D-15374 | Me | single bond | H | 1-Me-pyrazol-3-yl | Me | Cl | CH₂OMe | O |

**[Table 996]**

| Compound Number | Y | W | Z | R¹ | R² | R³ | R¹⁰ | A |
|---|---|---|---|---|---|---|---|---|
| D-15375 | Me(R) | single bond | H | 1-Me-pyrazol-3-yl | Me | Cl | CH₂OMe | O |
| D-15376 | Me(S) | single bond | H | 1-Me-pyrazol-3-yl | Me | Cl | CH₂OMe | O |
| D-15377 | H | single bond | Me | 1-Me-pyrazol-3-yl | Me | Me | CH₂OMe | O |
| D-15378 | Me | single bond | H | 1-Me-pyrazol-3-yl | Me | Me | CH₂OMe | O |
| D-15379 | Me(R) | single bond | H | 1-Me-pyrazol-3-yl | Me | Me | CH₂OMe | O |
| D-15380 | Me(S) | single bond | H | 1-Me-pyrazol-3-yl | Me | Me | CH₂OMe | O |
| D-15381 | H | single bond | Me | 1-Me-pyrazol-5-yl | Me | Cl | CH₂OMe | O |
| D-15382 | Me | single bond | H | 1-Me-pyrazol-5-yl | Me | Cl | CH₂OMe | O |
| D-15383 | Me(R) | single bond | H | 1-Me-pyrazol-5-yl | Me | Cl | CH₂OMe | O |
| D-15384 | Me(S) | single bond | H | 1-Me-pyrazol-5-yl | Me | Cl | CH₂OMe | O |
| D-15385 | H | single bond | Me | 1-Me-pyrazol-5-yl | Me | Me | CH₂OMe | O |
| D-15386 | Me | single bond | H | 1-Me-pyrazol-5-yl | Me | Me | CH₂OMe | O |
| D-15387 | Me(R) | single bond | H | 1-Me-pyrazol-5-yl | Me | Me | CH₂OMe | O |
| D-15388 | Me(S) | single bond | H | 1-Me-pyrazol-5-yl | Me | Me | CH₂OMe | O |
| D-15389 | H | single bond | Me | CH₂(pyrimidin-2-yl) | Me | Cl | CH₂OMe | O |
| D-15390 | Me | single bond | H | CH₂(pyrimidin-2-yl) | Me | Cl | CH₂OMe | O |
| D-15391 | Me(R) | single bond | H | CH₂(pyrimidin-2-yl) | Me | Cl | CH₂OMe | O |
| D-15392 | Me(S) | single bond | H | CH₂(pyrimidin-2-yl) | Me | Cl | CH₂OMe | O |
| D-15393 | H | single bond | Me | CH₂(pyrimidin-2-yl) | Me | Me | CH₂OMe | O |
| D-15394 | Me | single bond | H | CH₂(pyrimidin-2-yl) | Me | Me | CH₂OMe | O |
| D-15395 | Me(R) | single bond | H | CH₂(pyrimidin-2-yl) | Me | Me | CH₂OMe | O |
| D-15396 | Me(S) | single bond | H | CH₂(pyrimidin-2-yl) | Me | Me | CH₂OMe | O |
| D-15397 | H | single bond | Me | Me | Me | Cl | C(=O)Me | O |
| D-15398 | Me | single bond | H | Me | Me | Cl | C(=O)Me | O |
| D-15399 | Me(R) | single bond | H | Me | Me | Cl | C(=O)Me | O |
| D-15400 | Me(S) | single bond | H | Me | Me | Cl | C(=O)Me | O |
| D-15401 | H | single bond | Me | Me | Me | Me | C(=O)Me | O |
| D-15402 | Me | single bond | H | Me | Me | Me | C(=O)Me | O |
| D-15403 | Me(R) | single bond | H | Me | Me | Me | C(=O)Me | O |
| D-15404 | Me(S) | single bond | H | Me | Me | Me | C(=O)Me | O |
| D-15405 | H | single bond | Me | CH₂CHF₂ | Me | Cl | C(=O)Me | O |
| D-15406 | Me | single bond | H | CH₂CHF₂ | Me | Cl | C(=O)Me | O |
| D-15407 | Me(R) | single bond | H | CH₂CHF₂ | Me | Cl | C(=O)Me | O |
| D-15408 | Me(S) | single bond | H | CH₂CHF₂ | Me | Cl | C(=O)Me | O |
| D-15409 | H | single bond | Me | CH₂CHF₂ | Me | Me | C(=O)Me | O |
| D-15410 | Me | single bond | H | CH₂CHF₂ | Me | Me | C(=O)Me | O |
| D-15411 | Me(R) | single bond | H | CH₂CHF₂ | Me | Me | C(=O)Me | O |
| D-15412 | Me(S) | single bond | H | CH₂CHF₂ | Me | Me | C(=O)Me | O |
| D-15413 | H | single bond | Me | *n*-Pr | Me | Cl | C(=O)Me | O |
| D-15414 | Me | single bond | H | *n*-Pr | Me | Cl | C(=O)Me | O |
| D-15415 | Me(R) | single bond | H | *n*-Pr | Me | Cl | C(=O)Me | O |
| D-15416 | Me(S) | single bond | H | *n*-Pr | Me | Cl | C(=O)Me | O |
| D-15417 | H | single bond | Me | *n*-Pr | Me | Me | C(=O)Me | O |
| D-15418 | Me | single bond | H | *n*-Pr | Me | Me | C(=O)Me | O |

**[Table 997]**

| Compound Number | Y | W | Z | R¹ | R² | R³ | R¹⁰ | A |
|---|---|---|---|---|---|---|---|---|
| D-15419 | Me(R) | single bond | H | *n*-Pr | Me | Me | C(=O)Me | O |
| D-15420 | Me(S) | single bond | H | *n*-Pr | Me | Me | C(=O)Me | O |
| D-15421 | H | single bond | Me | *i*-Pr | Me | Cl | C(=O)Me | O |
| D-15422 | Me | single bond | H | *i*-Pr | Me | Cl | C(=O)Me | O |
| D-15423 | Me(R) | single bond | H | *i*-Pr | Me | Cl | C(=O)Me | O |
| D-15424 | Me(S) | single bond | H | *i*-Pr | Me | Cl | C(=O)Me | O |
| D-15425 | H | single bond | Me | *i*-Pr | Me | Me | C(=O)Me | O |
| D-15426 | Me | single bond | H | *i*-Pr | Me | Me | C(=O)Me | O |
| D-15427 | Me(R) | single bond | H | *i*-Pr | Me | Me | C(=O)Me | O |
| D-15428 | Me(S) | single bond | H | *i*-Pr | Me | Me | C(=O)Me | O |
| D-15429 | H | single bond | Me | *c*-Pr(1-F) | Me | Cl | C(=O)Me | O |
| D-15430 | Me | single bond | H | *c*-Pr(1-F) | Me | Cl | C(=O)Me | O |
| D-15431 | Me(R) | single bond | H | *c*-Pr(1-F) | Me | Cl | C(=O)Me | O |
| D-15432 | Me(S) | single bond | H | *c*-Pr(1-F) | Me | Cl | C(=O)Me | O |
| D-15433 | H | single bond | Me | *c*-Pr(1-F) | Me | Me | C(=O)Me | O |
| D-15434 | Me | single bond | H | *c*-Pr(1-F) | Me | Me | C(=O)Me | O |
| D-15435 | Me(R) | single bond | H | *c*-Pr(1-F) | Me | Me | C(=O)Me | O |
| D-15436 | Me(S) | single bond | H | *c*-Pr(1-F) | Me | Me | C(=O)Me | O |
| D-15437 | H | single bond | Me | *c*-Pr(2,2-F₂) | Me | Cl | C(=O)Me | O |
| D-15438 | Me | single bond | H | *c*-Pr(2,2-F₂) | Me | Cl | C(=O)Me | O |
| D-15439 | Me(R) | single bond | H | *c*-Pr(2,2-F₂) | Me | Cl | C(=O)Me | O |
| D-15440 | Me(S) | single bond | H | *c*-Pr(2,2-F₂) | Me | Cl | C(=O)Me | O |
| D-15441 | H | single bond | Me | *c*-Pr(2,2-F₂) | Me | Me | C(=O)Me | O |
| D-15442 | Me | single bond | H | *c*-Pr(2,2-F₂) | Me | Me | C(=O)Me | O |
| D-15443 | Me(R) | single bond | H | *c*-Pr(2,2-F₂) | Me | Me | C(=O)Me | O |
| D-15444 | Me(S) | single bond | H | *c*-Pr(2,2-F₂) | Me | Me | C(=O)Me | O |
| D-15445 | H | single bond | Me | *c*-Pr(1-Cl) | Me | Cl | C(=O)Me | O |
| D-15446 | Me | single bond | H | *c*-Pr(1-Cl) | Me | Cl | C(=O)Me | O |
| D-15447 | Me(R) | single bond | H | *c*-Pr(1-Cl) | Me | Cl | C(=O)Me | O |
| D-15448 | Me(S) | single bond | H | *c*-Pr(1-Cl) | Me | Cl | C(=O)Me | O |
| D-15449 | H | single bond | Me | *c*-Pr(1-Cl) | Me | Me | C(=O)Me | O |
| D-15450 | Me | single bond | H | *c*-Pr(1-Cl) | Me | Me | C(=O)Me | O |
| D-15451 | Me(R) | single bond | H | *c*-Pr(1-Cl) | Me | Me | C(=O)Me | O |
| D-15452 | Me(S) | single bond | H | *c*-Pr(1-Cl) | Me | Me | C(=O)Me | O |
| D-15453 | H | single bond | Me | *c*-Pr(1-Br) | Me | Cl | C(=O)Me | O |
| D-15454 | Me | single bond | H | *c*-Pr(1-Br) | Me | Cl | C(=O)Me | O |
| D-15455 | Me(R) | single bond | H | *c*-Pr(1-Br) | Me | Cl | C(=O)Me | O |
| D-15456 | Me(S) | single bond | H | *c*-Pr(1-Br) | Me | Cl | C(=O)Me | O |
| D-15457 | H | single bond | Me | *c*-Pr(1-Br) | Me | Me | C(=O)Me | O |
| D-15458 | Me | single bond | H | *c*-Pr(1-Br) | Me | Me | C(=O)Me | O |
| D-15459 | Me(R) | single bond | H | *c*-Pr(1-Br) | Me | Me | C(=O)Me | O |
| D-15460 | Me(S) | single bond | H | *c*-Pr(1-Br) | Me | Me | C(=O)Me | O |
| D-15461 | H | single bond | Me | *c*-Pr(1-Me) | Me | Cl | C(=O)Me | O |
| D-15462 | Me | single bond | H | *c*-Pr(1-Me) | Me | Cl | C(=O)Me | O |

**[Table 998]**

| Compound Number | Y | W | Z | R¹ | R² | R³ | R¹⁰ | A |
|---|---|---|---|---|---|---|---|---|
| D-15463 | Me(R) | single bond | H | *c*-Pr(1-Me) | Me | Cl | C(=O)Me | O |
| D-15464 | Me(S) | single bond | H | *c*-Pr(1-Me) | Me | Cl | C(=O)Me | O |
| D-15465 | H | single bond | Me | *c*-Pr(1-Me) | Me | Me | C(=O)Me | O |
| D-15466 | Me | single bond | H | *c*-Pr(1-Me) | Me | Me | C(=O)Me | O |
| D-15467 | Me(R) | single bond | H | *c*-Pr(1-Me) | Me | Me | C(=O)Me | O |
| D-15468 | Me(S) | single bond | H | *c*-Pr(1-Me) | Me | Me | C(=O)Me | O |
| D-15469 | H | single bond | Me | *c*-Pr(1-CH₂OH) | Me | Cl | C(=O)Me | O |
| D-15470 | Me | single bond | H | *c*-Pr(1-CH₂OH) | Me | Cl | C(=O)Me | O |
| D-15471 | Me(R) | single bond | H | *c*-Pr(1-CH₂OH) | Me | Cl | C(=O)Me | O |
| D-15472 | Me(S) | single bond | H | *c*-Pr(1-CH₂OH) | Me | Cl | C(=O)Me | O |
| D-15473 | H | single bond | Me | *c*-Pr(1-CH₂OH) | Me | Me | C(=O)Me | O |
| D-15474 | Me | single bond | H | *c*-Pr(1-CH₂OH) | Me | Me | C(=O)Me | O |
| D-15475 | Me(R) | single bond | H | *c*-Pr(1-CH₂OH) | Me | Me | C(=O)Me | O |
| D-15476 | Me(S) | single bond | H | *c*-Pr(1-CH₂OH) | Me | Me | C(=O)Me | O |
| D-15477 | H | single bond | Me | *c*-Pr[1-CH₂OS(=O)₂Me] | Me | Cl | C(=O)Me | O |
| D-15478 | Me | single bond | H | *c*-Pr[1-CH₂OS(=O)₂Me] | Me | Cl | C(=O)Me | O |
| D-15479 | Me(R) | single bond | H | *c*-Pr[1-CH₂OS(=O)₂Me] | Me | Cl | C(=O)Me | O |
| D-15480 | Me(S) | single bond | H | *c*-Pr[1-CH₂OS(=O)₂Me] | Me | Cl | C(=O)Me | O |
| D-15481 | H | single bond | Me | *c*-Pr[1-CH₂OS(=O)₂Me] | Me | Me | C(=O)Me | O |
| D-15482 | Me | single bond | H | *c*-Pr[1-CH₂OS(=O)₂Me] | Me | Me | C(=O)Me | O |
| D-15483 | Me(R) | single bond | H | *c*-Pr[1-CH₂OS(=O)₂Me] | Me | Me | C(=O)Me | O |
| D-15484 | Me(S) | single bond | H | *c*-Pr[1-CH₂OS(=O)₂Me] | Me | Me | C(=O)Me | O |
| D-15485 | H | single bond | Me | *c*-Pr(1-CH₂OMe) | Me | Cl | C(=O)Me | O |
| D-15486 | Me | single bond | H | *c*-Pr(1-CH₂OMe) | Me | Cl | C(=O)Me | O |
| D-15487 | Me(R) | single bond | H | *c*-Pr(1-CH₂OMe) | Me | Cl | C(=O)Me | O |
| D-15488 | Me(S) | single bond | H | *c*-Pr(1-CH₂OMe) | Me | Cl | C(=O)Me | O |
| D-15489 | H | single bond | Me | *c*-Pr(1-CH₂OMe) | Me | Me | C(=O)Me | O |
| D-15490 | Me | single bond | H | *c*-Pr(1-CH₂OMe) | Me | Me | C(=O)Me | O |
| D-15491 | Me(R) | single bond | H | *c*-Pr(1-CH₂OMe) | Me | Me | C(=O)Me | O |
| D-15492 | Me(S) | single bond | H | *c*-Pr(1-CH₂OMe) | Me | Me | C(=O)Me | O |
| D-15493 | H | single bond | Me | *c*-Pr(1-CH₂OCN) | Me | Cl | C(=O)Me | O |
| D-15494 | Me | single bond | H | *c*-Pr(1-CH₂OCN) | Me | Cl | C(=O)Me | O |
| D-15495 | Me(R) | single bond | H | *c*-Pr(1-CH₂OCN) | Me | Cl | C(=O)Me | O |
| D-15496 | Me(S) | single bond | H | *c*-Pr(1-CH₂OCN) | Me | Cl | C(=O)Me | O |
| D-15497 | H | single bond | Me | *c*-Pr(1-CH₂OCN) | Me | Me | C(=O)Me | O |
| D-15498 | Me | single bond | H | *c*-Pr(1-CH₂OCN) | Me | Me | C(=O)Me | O |
| D-15499 | Me(R) | single bond | H | *c*-Pr(1-CH₂OCN) | Me | Me | C(=O)Me | O |
| D-15500 | Me(S) | single bond | H | *c*-Pr(1-CH₂OCN) | Me | Me | C(=O)Me | O |
| D-15501 | H | single bond | Me | *c*-Pr(1-CH₂SMe) | Me | Cl | C(=O)Me | O |
| D-15502 | Me | single bond | H | *c*-Pr(1-CH₂SMe) | Me | Cl | C(=O)Me | O |
| D-15503 | Me(R) | single bond | H | *c*-Pr(1-CH₂SMe) | Me | Cl | C(=O)Me | O |
| D-15504 | Me(S) | single bond | H | *c*-Pr(1-CH₂SMe) | Me | Cl | C(=O)Me | O |
| D-15505 | H | single bond | Me | *c*-Pr(1-CH₂SMe) | Me | Me | C(=O)Me | O |
| D-15506 | Me | single bond | H | *c*-Pr(1-CH₂SMe) | Me | Me | C(=O)Me | O |

**[Table 999]**

| Compound Number | Y | W | Z | R¹ | R² | R³ | R¹⁰ | A |
|---|---|---|---|---|---|---|---|---|
| D-15507 | Me(R) | single bond | H | *c* -Pr(1-CH₂SMe) | Me | Me | C(=O)Me | O |
| D-15508 | Me(S) | single bond | H | *c* -Pr(1-CH₂SMe) | Me | Me | C(=O)Me | O |
| D-15509 | H | single bond | Me | *c* -Pr[1-CH₂S(=O)Me] | Me | Cl | C(=O)Me | O |
| D-15510 | Me | single bond | H | *c* -Pr[1-CH₂S(=O)Me] | Me | Cl | C(=O)Me | O |
| D-15511 | Me(R) | single bond | H | *c* -Pr[1-CH₂S(=O)Me] | Me | Cl | C(=O)Me | O |
| D-15512 | Me(S) | single bond | H | *c* -Pr[1-CH₂S(=O)Me] | Me | Cl | C(=O)Me | O |
| D-15513 | H | single bond | Me | *c* -Pr[1-CH₂S(=O)Me] | Me | Me | C(=O)Me | O |
| D-15514 | Me | single bond | H | *c* -Pr[1-CH₂S(=O)Me] | Me | Me | C(=O)Me | O |
| D-15515 | Me(R) | single bond | H | *c* -Pr[1-CH₂S(=O)Me] | Me | Me | C(=O)Me | O |
| D-15516 | Me(S) | single bond | H | *c* -Pr[1-CH₂S(=O)Me] | Me | Me | C(=O)Me | O |
| D-15517 | H | single bond | Me | *c* -Pr[1-CH₂S(=O)₂Me] | Me | Cl | C(=O)Me | O |
| D-15518 | Me | single bond | H | *c* -Pr[1-C H₂S(=O)₂Me] | Me | Cl | C(=O)Me | O |
| D-15519 | Me(R) | single bond | H | *c* -Pr[1-C H₂S(=O)₂Me] | Me | Cl | C(=O)Me | O |
| D-15520 | Me(S) | single bond | H | *c* -Pr[1-C H₂S(=O)₂Me] | Me | Cl | C(=O)Me | O |
| D-15521 | H | single bond | Me | *c* -Pr[1-C H₂S(=O)₂Me] | Me | Me | C(=O)Me | O |
| D-15522 | Me | single bond | H | *c* -Pr[1-C H₂S(=O)₂Me] | Me | Me | C(=O)Me | O |
| D-15523 | Me(R) | single bond | H | *c* -Pr[1-C H₂S(=O)₂Me] | Me | Me | C(=O)Me | O |
| D-15524 | Me(S) | single bond | H | *c* -Pr[1-C H₂S(=O)₂Me] | Me | Me | C(=O)Me | O |
| D-15525 | H | single bond | Me | *c* -Pr(1-CH₂F) | Me | Cl | C(=O)Me | O |
| D-15526 | Me | single bond | H | *c* -Pr(1-CH₂F) | Me | Cl | C(=O)Me | O |
| D-15527 | Me(R) | single bond | H | *c* -Pr(1-CH₂F) | Me | Cl | C(=O)Me | O |
| D-15528 | Me(S) | single bond | H | *c* -Pr(1-CH₂F) | Me | Cl | C(=O)Me | O |
| D-15529 | H | single bond | Me | *c* -Pr(1-CH₂F) | Me | Me | C(=O)Me | O |
| D-15530 | Me | single bond | H | *c* -Pr(1-CH₂F) | Me | Me | C(=O)Me | O |
| D-15531 | Me(R) | single bond | H | *c* -Pr(1-CH₂F) | Me | Me | C(=O)Me | O |
| D-15532 | Me(S) | single bond | H | *c* -Pr(1-CH₂F) | Me | Me | C(=O)Me | O |
| D-15533 | H | single bond | Me | *c* -Pr(1-CHF₂) | Me | Cl | C(=O)Me | O |
| D-15534 | Me | single bond | H | *c* -Pr(1-CHF₂) | Me | Cl | C(=O)Me | O |
| D-15535 | Me(R) | single bond | H | *c* -Pr(1-CHF₂) | Me | Cl | C(=O)Me | O |
| D-15536 | Me(S) | single bond | H | *c* -Pr(1-CHF₂) | Me | Cl | C(=O)Me | O |
| D-15537 | H | single bond | Me | *c* -Pr(1-CHF₂) | Me | Me | C(=O)Me | O |
| D-15538 | Me | single bond | H | *c* -Pr(1-CHF₂) | Me | Me | C(=O)Me | O |
| D-15539 | Me(R) | single bond | H | *c* -Pr(1-CHF₂) | Me | Me | C(=O)Me | O |
| D-15540 | Me(S) | single bond | H | *c* -Pr(1-CHF₂) | Me | Me | C(=O)Me | O |
| D-15541 | H | single bond | Me | *c* -Pr(1-CF₃) | Me | Cl | C(=O)Me | O |
| D-15542 | Me | single bond | H | *c* -Pr(1-CF₃) | Me | Cl | C(=O)Me | O |
| D-15543 | Me(R) | single bond | H | *c* -Pr(1-CF₃) | Me | Cl | C(=O)Me | O |
| D-15544 | Me(S) | single bond | H | *c* -Pr(1-CF₃) | Me | Cl | C(=O)Me | O |
| D-15545 | H | single bond | Me | *c* -Pr(1-CF₃) | Me | Me | C(=O)Me | O |
| D-15546 | Me | single bond | H | *c* -Pr(1-CF₃) | Me | Me | C(=O)Me | O |
| D-15547 | Me(R) | single bond | H | *c* -Pr(1-CF₃) | Me | Me | C(=O)Me | O |
| D-15548 | Me(S) | single bond | H | *c* -Pr(1-CF₃) | Me | Me | C(=O)Me | O |
| D-15549 | H | single bond | Me | *c -*Pr(1-C≡CH) | Me | Cl | C(=O)Me | O |
| D-15550 | Me | single bond | H | *c* -Pr(1-C≡CH) | Me | Cl | C(=O)Me | O |

**[Table 1000]**

| Compound Number | Y | W | Z | R¹ | R² | R³ | R¹⁰ | A |
|---|---|---|---|---|---|---|---|---|
| D-15551 | Me(R) | single bond | H | *c -*Pr(1-C≡CH) | Me | Cl | C(=O)Me | O |
| D-15552 | Me(S) | single bond | H | *c -*Pr(1-C≡CH) | Me | Cl | C(=O)Me | O |
| D-15553 | H | single bond | Me | *c -*Pr(1-C≡CH) | Me | Me | C(=O)Me | O |
| D-15554 | Me | single bond | H | *c -*Pr(1-C≡CH) | Me | Me | C(=O)Me | O |
| D-15555 | Me(R) | single bond | H | *c -*Pr(1-C≡CH) | Me | Me | C(=O)Me | O |
| D-15556 | Me(S) | single bond | H | *c* -Pr(1-C≡CH) | Me | Me | C(=O)Me | O |
| D-15557 | H | single bond | Me | *c*-Pr[1-C(=O)OEt] | Me | Cl | C(=O)Me | O |
| D-15558 | Me | single bond | H | *c*-Pr[1-C(=O)OEt] | Me | Cl | C(=O)Me | O |
| D-15559 | Me(R) | single bond | H | *c*-Pr[1-C(=O)OEt] | Me | Cl | C(=O)Me | O |
| D-15560 | Me(S) | single bond | H | *c*-Pr[1-C(=O)OEt] | Me | Cl | C(=O)Me | O |
| D-15561 | H | single bond | Me | *c*-Pr[1-C(=O)OEt] | Me | Me | C(=O)Me | O |
| D-15562 | Me | single bond | H | *c*-Pr[1-C(=O)OEt] | Me | Me | C(=O)Me | O |
| D-15563 | Me(R) | single bond | H | *c* -Pr[1-C(=O)OEt] | Me | Me | C(=O)Me | O |
| D-15564 | Me(S) | single bond | H | *c*-Pr[1-C(=O)OEt] | Me | Me | C(=O)Me | O |
| D-15565 | H | single bond | Me | *c* -Pr[1-C(=O)OH] | Me | Cl | C(=O)Me | O |
| D-15566 | Me | single bond | H | *c* -Pr[1-C(=O)OH] | Me | Cl | C(=O)Me | O |
| D-15567 | Me(R) | single bond | H | *c* -Pr[1-C(=O)OH] | Me | Cl | C(=O)Me | O |
| D-15568 | Me(S) | single bond | H | *c* -Pr[1-C(=O)OH] | Me | Cl | C(=O)Me | O |
| D-15569 | H | single bond | Me | *c* -Pr[1-C(=O)OH] | Me | Me | C(=O)Me | O |
| D-15570 | Me | single bond | H | *c* -Pr[1-C(=O)OH] | Me | Me | C(=O)Me | O |
| D-15571 | Me(R) | single bond | H | *c* -Pr[1-C(=O)OH] | Me | Me | C(=O)Me | O |
| D-15572 | Me(S) | single bond | H | *c* -Pr[1-C(=O)OH] | Me | Me | C(=O)Me | O |
| D-15573 | H | single bond | Me | *c* -Pr[1-C(=O)NH₂] | Me | Cl | C(=O)Me | O |
| D-15574 | Me | single bond | H | *c* -Pr[1-C(=O)NH₂] | Me | Cl | C(=O)Me | O |
| D-15575 | Me(R) | single bond | H | *c* -Pr[1-C(=O)NH₂] | Me | Cl | C(=O)Me | O |
| D-15576 | Me(S) | single bond | H | *c* -Pr[1-C(=O)NH₂] | Me | Cl | C(=O)Me | O |
| D-15577 | H | single bond | Me | *c* -Pr[1-C(=O)NH₂] | Me | Me | C(=O)Me | O |
| D-15578 | Me | single bond | H | *c* -Pr[1-C(=O)NH₂] | Me | Me | C(=O)Me | O |
| D-15579 | Me(R) | single bond | H | *c* -Pr[1-C(=O)NH₂] | Me | Me | C(=O)Me | O |
| D-15580 | Me(S) | single bond | H | *c* -Pr[1-C(=O)NH₂] | Me | Me | C(=O)Me | O |
| D-15581 | H | single bond | Me | *c* -Pr(1-OMe) | Me | Cl | C(=O)Me | O |
| D-15582 | Me | single bond | H | *c* -Pr(1-OMe) | Me | Cl | C(=O)Me | O |
| D-15583 | Me(R) | single bond | H | *c* -Pr(1-OMe) | Me | Cl | C(=O)Me | O |
| D-15584 | Me(S) | single bond | H | *c* -Pr(1-OMe) | Me | Cl | C(=O)Me | O |
| D-15585 | H | single bond | Me | *c*-Pr(1-OMe) | Me | Me | C(=O)Me | O |
| D-15586 | Me | single bond | H | *c* -Pr(1-OMe) | Me | Me | C(=O)Me | O |
| D-15587 | Me(R) | single bond | H | *c* -Pr(1-OMe) | Me | Me | C(=O)Me | O |
| D-15588 | Me(S) | single bond | H | *c* -Pr(1-OMe) | Me | Me | C(=O)Me | O |
| D-15589 | H | single bond | Me | *c* -Pr(1-SMe) | Me | Cl | C(=O)Me | O |
| D-15590 | Me | single bond | H | *c* -Pr(1-SMe) | Me | Cl | C(=O)Me | O |
| D-15591 | Me(R) | single bond | H | *c* -Pr(1-SMe) | Me | Cl | C(=O)Me | O |
| D-15592 | Me(S) | single bond | H | *c* -Pr(1-SMe) | Me | Cl | C(=O)Me | O |
| D-15593 | H | single bond | Me | *c*-Pr(1-SMe) | Me | Me | C(=O)Me | O |
| D-15594 | Me | single bond | H | *c*-Pr(1-SMe) | Me | Me | C(=O)Me | O |

**[Table 1001]**

| Compound Number | Y | W | Z | R¹ | R² | R³ | R¹⁰ | A |
|---|---|---|---|---|---|---|---|---|
| D-15595 | Me(R) | single bond | H | *c* -Pr(1-SMe) | Me | Me | C(=O)Me | O |
| D-15596 | Me(S) | single bond | H | *c* -Pr(1-SMe) | Me | Me | C(=O)Me | O |
| D-15597 | H | single bond | Me | *c* -Pr[1-S(=O)Me] | Me | Cl | C(=O)Me | O |
| D-15598 | Me | single bond | H | *c* -Pr[1-S(=O)Me] | Me | Cl | C(=O)Me | O |
| D-15599 | Me(R) | single bond | H | *c*-Pr[1-S(=O)Me] | Me | Cl | C(=O)Me | O |
| D-15600 | Me(S) | single bond | H | *c* -Pr[1 -S(=O)Me] | Me | Cl | C(=O)Me | O |
| D-15601 | H | single bond | Me | *c*-Pr[1-S(=O)Me] | Me | Me | C(=O)Me | O |
| D-15602 | Me | single bond | H | *c* -Pr[1-S(=O)Me] | Me | Me | C(=O)Me | O |
| D-15603 | Me(R) | single bond | H | *c* -Pr[1 -S(=O)Me] | Me | Me | C(=O)Me | O |
| D-15604 | Me(S) | single bond | H | *c*-Pr[1-S(=O)Me] | Me | Me | C(=O)Me | O |
| D-15605 | H | single bond | Me | *c* -Pr[1-S(=O)₂Me] | Me | Cl | C(=O)Me | O |
| D-15606 | Me | single bond | H | *c* -Pr[1-S(=O)₂Me] | Me | Cl | C(=O)Me | O |
| D-15607 | Me(R) | single bond | H | *c* -Pr[1-S(=O)₂Me] | Me | Cl | C(=O)Me | O |
| D-15608 | Me(S) | single bond | H | *c* -Pr[1-S(=O)₂Me] | Me | Cl | C(=O)Me | O |
| D-15609 | H | single bond | Me | *c* -Pr[1-S(=O)₂Me] | Me | Me | C(=O)Me | O |
| D-15610 | Me | single bond | H | *c* -Pr[1-S(=O)₂Me] | Me | Me | C(=O)Me | O |
| D-15611 | Me(R) | single bond | H | *c* -Pr[1-S(=O)₂Me] | Me | Me | C(=O)Me | O |
| D-15612 | Me(S) | single bond | H | *c* -Pr[1-S(=O)₂Me] | Me | Me | C(=O)Me | O |
| D-15613 | H | single bond | Me | *c* -Pr[1-S(=O)₂CHF₂] | Me | Cl | C(=O)Me | O |
| D-15614 | Me | single bond | H | *c* -Pr[1-S(=O)₂CHF₂] | Me | Cl | C(=O)Me | O |
| D-15615 | Me(R) | single bond | H | *c* -Pr[1-S(=O)₂CHF₂] | Me | Cl | C(=O)Me | O |
| D-15616 | Me(S) | single bond | H | *c* -Pr[1-S(=O)₂CHF₂] | Me | Cl | C(=O)Me | O |
| D-15617 | H | single bond | Me | *c* -Pr[1-S(=O)₂CHF₂] | Me | Me | C(=O)Me | O |
| D-15618 | Me | single bond | H | *c* -Pr[1-S(=O)₂CHF₂] | Me | Me | C(=O)Me | O |
| D-15619 | Me(R) | single bond | H | *c* -Pr[1-S(=O)₂CHF₂] | Me | Me | C(=O)Me | O |
| D-15620 | Me(S) | single bond | H | *c* -Pr[1-S(=O)₂CHF₂] | Me | Me | C(=O)Me | O |
| D-15621 | H | single bond | Me | *c* -Pr[1-S(=O)₂CF₃] | Me | Cl | C(=O)Me | O |
| D-15622 | Me | single bond | H | *c* -Pr[1-S(=O)₂CF₃] | Me | Cl | C(=O)Me | O |
| D-15623 | Me(R) | single bond | H | *c* -Pr[1-S(=O)₂CF₃] | Me | Cl | C(=O)Me | O |
| D-15624 | Me(S) | single bond | H | *c* -Pr[1-S(=O)₂CF₃] | Me | Cl | C(=O)Me | O |
| D-15625 | H | single bond | Me | *c* -Pr[1-S(=O)₂CF₃] | Me | Me | C(=O)Me | O |
| D-15626 | Me | single bond | H | *c* -Pr[1-S(=O)₂CF₃] | Me | Me | C(=O)Me | O |
| D-15627 | Me(R) | single bond | H | *c* -Pr[1-S(=O)₂CF₃] | Me | Me | C(=O)Me | O |
| D-15628 | Me(S) | single bond | H | *c* -Pr[1-S(=O)₂CF₃] | Me | Me | C(=O)Me | O |
| D-15629 | H | single bond | Me | *c* -Pr[1-(5,6-dihydro-1,4,2-dioxazin-3-yl)] | Me | Cl | C(=O)Me | O |
| D-15630 | Me | single bond | H | *c* -Pr[1-(5,6-dihydro-1,4,2-dioxazin-3-yl)] | Me | Cl | C(=O)Me | O |
| D-15631 | Me(R) | single bond | H | *c* -Pr[1-(5,6-dihydro-1,4,2-dioxazin-3-yl)] | Me | Cl | C(=O)Me | O |
| D-15632 | Me(S) | single bond | H | *c* -Pr[1-(5,6-dihydro-1,4,2-dioxazin-3-yl)] | Me | Cl | C(=O)Me | O |
| D-15633 | H | single bond | Me | *c* -Pr[1-(5,6-dihydro-1,4,2-dioxazin-3-yl)] | Me | Me | C(=O)Me | O |
| D-15634 | Me | single bond | H | *c* -Pr[1-(5,6-dihydro-1 ,4,2-dioxazin-3-yl)] | Me | Me | C(=O)Me | O |
| D-15635 | Me(R) | single bond | H | *c* -Pr[1-(5,6-dihydro-1,4,2-dioxazin-3-yl)] | Me | Me | C(=O)Me | O |
| D-15636 | Me(S) | single bond | H | *c* -Pr[1-(5,6-dihydro-1,4,2-dioxazin-3-yl)] | Me | Me | C(=O)Me | O |
| D-15637 | H | single bond | Me | *c* -Pr[1-(5-hydroxy-1 ,3,4-oxadiazol-2-yl)] | Me | Cl | C(=O)Me | O |
| D-15638 | Me | single bond | H | *c* -Pr[1-(5-hydroxy-1 ,3,4-oxadiazol-2-yl)] | Me | Cl | C(=O)Me | O |

**[Table 1002]**

| Compound Number | Y | W | Z | R¹ | R² | R³ | R¹⁰ | A |
|---|---|---|---|---|---|---|---|---|
| D-15639 | Me(R) | single bond | H | *c* -Pr[1-(5-hydroxy-1 ,3,4-oxadiazol-2-yl)] | Me | Cl | C(=O)Me | O |
| D-15640 | Me(S) | single bond | H | *c* -Pr[1-(5-hydroxy-1 ,3,4-oxadiazol-2-yl)] | Me | Cl | C(=O)Me | O |
| D-15641 | H | single bond | Me | *c* -Pr[1-(5-hydroxy-1 ,3,4-oxadiazol-2-yl)] | Me | Me | C(=O)Me | O |
| D-15642 | Me | single bond | H | *c* -Pr[1-(5-hydroxy-1 ,3,4-oxadiazol-2-yl)] | Me | Me | C(=O)Me | O |
| D-15643 | Me(R) | single bond | H | *c* -Pr[1-(5-hydroxy-1 ,3,4-oxadiazol-2-yl)] | Me | Me | C(=O)Me | O |
| D-15644 | Me(S) | single bond | H | *c* -Pr[1-(5-hydroxy-1 ,3,4-oxadiazol-2-yl)] | Me | Me | C(=O)Me | O |
| D-15645 | H | single bond | Me | *c* -Pr[1-(pyrimidin-2-yl)] | Me | Cl | C(=O)Me | O |
| D-15646 | Me | single bond | H | *c* -Pr[1-(pyrimidin-2-yl)] | Me | Cl | C(=O)Me | O |
| D-15647 | Me(R) | single bond | H | *c* -Pr[1-(pyrimidin-2-yl)] | Me | Cl | C(=O)Me | O |
| D-15648 | Me(S) | single bond | H | *c* -Pr[1-(pyrimidin-2-yl)] | Me | Cl | C(=O)Me | O |
| D-15649 | H | single bond | Me | *c* -Pr[1-(pyrimidin-2-yl)] | Me | Me | C(=O)Me | O |
| D-15650 | Me | single bond | H | *c* -Pr[1-(pyrimidin-2-yl)] | Me | Me | C(=O)Me | O |
| D-15651 | Me(R) | single bond | H | *c* -Pr[1-(pyrimidin-2-yl)] | Me | Me | C(=O)Me | O |
| D-15652 | Me(S) | single bond | H | *c* -Pr[1-(pyrimidin-2-yl)] | Me | Me | C(=O)Me | O |
| D-15653 | H | single bond | Me | *t*-Bu | Me | Cl | C(=O)Me | O |
| D-15654 | Me | single bond | H | *t*-Bu | Me | Cl | C(=O)Me | O |
| D-15655 | Me(R) | single bond | H | *t*-Bu | Me | Cl | C(=O)Me | O |
| D-15656 | Me(S) | single bond | H | *t*-Bu | Me | Cl | C(=O)Me | O |
| D-15657 | H | single bond | Me | *t*-Bu | Me | Me | C(=O)Me | O |
| D-15658 | Me | single bond | H | *t*-Bu | Me | Me | C(=O)Me | O |
| D-15659 | Me(R) | single bond | H | *t*-Bu | Me | Me | C(=O)Me | O |
| D-15660 | Me(S) | single bond | H | *t*-Bu | Me | Me | C(=O)Me | O |
| D-15661 | H | single bond | Me | *c* -Bu | Me | Cl | C(=O)Me | O |
| D-15662 | Me | single bond | H | *c* -Bu | Me | Cl | C(=O)Me | O |
| D-15663 | Me(R) | single bond | H | *c* -Bu | Me | Cl | C(=O)Me | O |
| D-15664 | Me(S) | single bond | H | *c* -Bu | Me | Cl | C(=O)Me | O |
| D-15665 | H | single bond | Me | *c* -Bu | Me | Me | C(=O)Me | O |
| D-15666 | Me | single bond | H | *c* -Bu | Me | Me | C(=O)Me | O |
| D-15667 | Me(R) | single bond | H | *c* -Bu | Me | Me | C(=O)Me | O |
| D-15668 | Me(S) | single bond | H | *c* -Bu | Me | Me | C(=O)Me | O |
| D-15669 | H | single bond | Me | *c* -Bu(1-CN) | Me | Cl | C(=O)Me | O |
| D-15670 | Me | single bond | H | *c* -Bu(1-CN) | Me | Cl | C(=O)Me | O |
| D-15671 | Me(R) | single bond | H | *c* -Bu(1-CN) | Me | Cl | C(=O)Me | O |
| D-15672 | Me(S) | single bond | H | *c* -Bu(1-CN) | Me | Cl | C(=O)Me | O |
| D-15673 | H | single bond | Me | *c* -Bu(1-CN) | Me | Me | C(=O)Me | O |
| D-15674 | Me | single bond | H | *c* -Bu(1-CN) | Me | Me | C(=O)Me | O |
| D-15675 | Me(R) | single bond | H | *c* -Bu(1-CN) | Me | Me | C(=O)Me | O |
| D-15676 | Me(S) | single bond | H | *c* -Bu(1-CN) | Me | Me | C(=O)Me | O |
| D-15677 | H | single bond | Me | *c* -Bu(3,3-F₂) | Me | Cl | C(=O)Me | O |
| D-15678 | Me | single bond | H | *c* -Bu(3,3-F₂) | Me | Cl | C(=O)Me | O |
| D-15679 | Me(R) | single bond | H | *c* -Bu(3,3-F₂) | Me | Cl | C(=O)Me | O |
| D-15680 | Me(S) | single bond | H | *c* -Bu(3,3-F₂) | Me | Cl | C(=O)Me | O |
| D-15681 | H | single bond | Me | *c* -Bu(3,3-F₂) | Me | Me | C(=O)Me | O |
| D-15682 | Me | single bond | H | *c* -Bu(3,3-F₂) | Me | Me | C(=O)Me | O |

**[Table 1003]**

| Compound Number | Y | W | Z | R¹ | R² | R³ | R¹⁰ | A |
|---|---|---|---|---|---|---|---|---|
| D-15683 | Me(R) | single bond | H | *c* -Bu(3,3-F₂) | Me | Me | C(=O)Me | O |
| D-15684 | Me(S) | single bond | H | *c* -Bu(3,3-F₂) | Me | Me | C(=O)Me | O |
| D-15685 | H | single bond | Me | oxetan-3-yl | Me | Cl | C(=O)Me | O |
| D-15686 | Me | single bond | H | oxetan-3-yl | Me | Cl | C(=O)Me | O |
| D-15687 | Me(R) | single bond | H | oxetan-3-yl | Me | Cl | C(=O)Me | O |
| D-15688 | Me(S) | single bond | H | oxetan-3-yl | Me | Cl | C(=O)Me | O |
| D-15689 | H | single bond | Me | oxetan-3-yl | Me | Me | C(=O)Me | O |
| D-15690 | Me | single bond | H | oxetan-3-yl | Me | Me | C(=O)Me | O |
| D-15691 | Me(R) | single bond | H | oxetan-3-yl | Me | Me | C(=O)Me | O |
| D-15692 | Me(S) | single bond | H | oxetan-3-yl | Me | Me | C(=O)Me | O |
| D-15693 | H | single bond | Me | oxetan(3-CN)-3-yl | Me | Cl | C(=O)Me | O |
| D-15694 | Me | single bond | H | oxetan(3-CN)-3-yl | Me | Cl | C(=O)Me | O |
| D-15695 | Me(R) | single bond | H | oxetan(3-CN)-3-yl | Me | Cl | C(=O)Me | O |
| D-15696 | Me(S) | single bond | H | oxetan(3-CN)-3-yl | Me | Cl | C(=O)Me | O |
| D-15697 | H | single bond | Me | oxetan(3-CN)-3-yl | Me | Me | C(=O)Me | O |
| D-15698 | Me | single bond | H | oxetan(3-CN)-3-yl | Me | Me | C(=O)Me | O |
| D-15699 | Me(R) | single bond | H | oxetan(3-CN)-3-yl | Me | Me | C(=O)Me | O |
| D-15700 | Me(S) | single bond | H | oxetan(3-CN)-3-yl | Me | Me | C(=O)Me | O |
| D-15701 | H | single bond | Me | azetidin[1-C(=O)Me]-3-yl | Me | Cl | C(=O)Me | O |
| D-15702 | Me | single bond | H | azetidin[1-C(=O)Me]-3-yl | Me | Cl | C(=O)Me | O |
| D-15703 | Me(R) | single bond | H | azetidin[1-C(=O)Me]-3-yl | Me | Cl | C(=O)Me | O |
| D-15704 | Me(S) | single bond | H | azetidin[1-C(=O)Me]-3-yl | Me | Cl | C(=O)Me | O |
| D-15705 | H | single bond | Me | azetidin[1-C(=O)Me]-3-yl | Me | Me | C(=O)Me | O |
| D-15706 | Me | single bond | H | azetidin[1-C(=O)Me]-3-yl | Me | Me | C(=O)Me | O |
| D-15707 | Me(R) | single bond | H | azetidin[1-C(=O)Me]-3-yl | Me | Me | C(=O)Me | O |
| D-15708 | Me(S) | single bond | H | azetidin[1-C(=O)Me]-3-yl | Me | Me | C(=O)Me | O |
| D-15709 | H | single bond | Me | azetidin[1-S(=O)₂Me]-3-yl | Me | Cl | C(=O)Me | O |
| D-15710 | Me | single bond | H | azetidin[1-S(=O)₂Me]-3-yl | Me | Cl | C(=O)Me | O |
| D-15711 | Me(R) | single bond | H | azetidin[1-S(=O)₂Me]-3-yl | Me | Cl | C(=O)Me | O |
| D-15712 | Me(S) | single bond | H | azetidin[1-S(=O)₂Me]-3-yl | Me | Cl | C(=O)Me | O |
| D-15713 | H | single bond | Me | azetidin[1-S(=O)₂Me]-3-yl | Me | Me | C(=O)Me | O |
| D-15714 | Me | single bond | H | azetidin[1-S(=O)₂Me]-3-yl | Me | Me | C(=O)Me | O |
| D-15715 | Me(R) | single bond | H | azetidin[1-S(=O)₂Me]-3-yl | Me | Me | C(=O)Me | O |
| D-15716 | Me(S) | single bond | H | azetidin[1-S(=O)₂Me]-3-yl | Me | Me | C(=O)Me | O |
| D-15717 | H | single bond | Me | azetidin[1-S(=O)₂CHF₂]-3-yl | Me | Cl | C(=O)Me | O |
| D-15718 | Me | single bond | H | azetidin[1-S(=O)₂CHF₂]-3-yl | Me | Cl | C(=O)Me | O |
| D-15719 | Me(R) | single bond | H | azetidin[1-S(=O)₂CHF₂]-3-yl | Me | Cl | C(=O)Me | O |
| D-15720 | Me(S) | single bond | H | azetidin[1-S(=O)₂CHF₂]-3-yl | Me | Cl | C(=O)Me | O |
| D-15721 | H | single bond | Me | azetidin[1-S(=O)₂CHF₂]-3-yl | Me | Me | C(=O)Me | O |
| D-15722 | Me | single bond | H | azetidin[1-S(=O)₂CHF₂]-3-yl | Me | Me | C(=O)Me | O |
| D-15723 | Me(R) | single bond | H | azetidin[1-S(=O)₂CHF₂]-3-yl | Me | Me | C(=O)Me | O |
| D-15724 | Me(S) | single bond | H | azetidin[1-S(=O)₂CHF₂]-3-yl | Me | Me | C(=O)Me | O |
| D-15725 | H | single bond | Me | c-Pen | Me | Cl | C(=O)Me | O |
| D-15726 | Me | single bond | H | *c* -Pen | Me | Cl | C(=O)Me | O |

**[Table 1004]**

| Compound Number | Y | W | Z | R¹ | R² | R³ | R¹⁰ | A |
|---|---|---|---|---|---|---|---|---|
| D-15727 | Me(R) | single bond | H | *c*-Pen | Me | Cl | C(=O)Me | O |
| D-15728 | Me(S) | single bond | H | *c*-Pen | Me | Cl | C(=O)Me | O |
| D-15729 | H | single bond | Me | *c*-Pen | Me | Me | C(=O)Me | O |
| D-15730 | Me | single bond | H | *c*-Pen | Me | Me | C(=O)Me | O |
| D-15731 | Me(R) | single bond | H | *c*-Pen | Me | Me | C(=O)Me | O |
| D-15732 | Me(S) | single bond | H | *c* -Pen | Me | Me | C(=O)Me | O |
| D-15733 | H | single bond | Me | tetrahydrofuran-3-yl | Me | Cl | C(=O)Me | O |
| D-15734 | Me | single bond | H | tetrahydrofuran-3-yl | Me | Cl | C(=O)Me | O |
| D-15735 | Me(R) | single bond | H | tetrahydrofuran-3-yl | Me | Cl | C(=O)Me | O |
| D-15736 | Me(S) | single bond | H | tetrahydrofuran-3-yl | Me | Cl | C(=O)Me | O |
| D-15737 | H | single bond | Me | tetrahydrofuran-3-yl | Me | Me | C(=O)Me | O |
| D-15738 | Me | single bond | H | tetrahydrofuran-3-yl | Me | Me | C(=O)Me | O |
| D-15739 | Me(R) | single bond | H | tetrahydrofuran-3-yl | Me | Me | C(=O)Me | O |
| D-15740 | Me(S) | single bond | H | tetrahydrofuran-3-yl | Me | Me | C(=O)Me | O |
| D-15741 | H | single bond | Me | tetrahydrothiophen-3-yl | Me | Cl | C(=O)Me | O |
| D-15742 | Me | single bond | H | tetrahydrothiophen-3-yl | Me | Cl | C(=O)Me | O |
| D-15743 | Me(R) | single bond | H | tetrahydrothiophen-3-yl | Me | Cl | C(=O)Me | O |
| D-15744 | Me(S) | single bond | H | tetrahydrothiophen-3-yl | Me | Cl | C(=O)Me | O |
| D-15745 | H | single bond | Me | tetrahydrothiophen-3-yl | Me | Me | C(=O)Me | O |
| D-15746 | Me | single bond | H | tetrahydrothiophen-3-yl | Me | Me | C(=O)Me | O |
| D-15747 | Me(R) | single bond | H | tetrahydrothiophen-3-yl | Me | Me | C(=O)Me | O |
| D-15748 | Me(S) | single bond | H | tetrahydrothiophen-3-yl | Me | Me | C(=O)Me | O |
| D-15749 | H | single bond | Me | *c*-Pen(1-CN) | Me | Cl | C(=O)Me | O |
| D-15750 | Me | single bond | H | *c*-Pen(1-CN) | Me | Cl | C(=O)Me | O |
| D-15751 | Me(R) | single bond | H | *c*-Pen(1-CN) | Me | Cl | C(=O)Me | O |
| D-15752 | Me(S) | single bond | H | *c*-Pen(1-CN) | Me | Cl | C(=O)Me | O |
| D-15753 | H | single bond | Me | *c*-Pen(1-CN) | Me | Me | C(=O)Me | O |
| D-15754 | Me | single bond | H | *c*-Pen(1-CN) | Me | Me | C(=O)Me | O |
| D-15755 | Me(R) | single bond | H | *c*-Pen(1-CN) | Me | Me | C(=O)Me | O |
| D-15756 | Me(S) | single bond | H | *c*-Pen(1-CN) | Me | Me | C(=O)Me | O |
| D-15757 | H | single bond | Me | tetrahydrofuran(3-CN)-3-yl | Me | Cl | C(=O)Me | O |
| D-15758 | Me | single bond | H | tetrahydrofuran(3-CN)-3-yl | Me | Cl | C(=O)Me | O |
| D-15759 | Me(R) | single bond | H | tetrahydrofuran(3-CN)-3-yl | Me | Cl | C(=O)Me | O |
| D-15760 | Me(S) | single bond | H | tetrahydrofuran(3-CN)-3-yl | Me | Cl | C(=O)Me | O |
| D-15761 | H | single bond | Me | tetrahydrofuran(3-CN)-3-yl | Me | Me | C(=O)Me | O |
| D-15762 | Me | single bond | H | tetrahydrofuran(3-CN)-3-yl | Me | Me | C(=O)Me | O |
| D-15763 | Me(R) | single bond | H | tetrahydrofuran(3-CN)-3-yl | Me | Me | C(=O)Me | O |
| D-15764 | Me(S) | single bond | H | tetrahydrofuran(3-CN)-3-yl | Me | Me | C(=O)Me | O |
| D-15765 | H | single bond | Me | tetrahydrothiophen(3-CN)-3-yl | Me | Cl | C(=O)Me | O |
| D-15766 | Me | single bond | H | tetrahydrothiophen(3-CN)-3-yl | Me | Cl | C(=O)Me | O |
| D-15767 | Me(R) | single bond | H | tetrahydrothiophen(3-CN)-3-yl | Me | Cl | C(=O)Me | O |
| D-15768 | Me(S) | single bond | H | tetrahydrothiophen(3-CN)-3-yl | Me | Cl | C(=O)Me | O |
| D-15769 | H | single bond | Me | tetrahydrothiophen(3-CN)-3-yl | Me | Me | C(=O)Me | O |
| D-15770 | Me | single bond | H | tetrahydrothiophen(3-CN)-3-yl | Me | Me | C(=O)Me | O |

**[Table 1005]**

| Compound Number | Y | W | Z | R¹ | R² | R³ | R¹⁰ | A |
|---|---|---|---|---|---|---|---|---|
| D-15771 | Me(R) | single bond | H | tetrahydrothiophen(3-CN)-3-yl | Me | Me | C(=O)Me | O |
| D-15772 | Me(S) | single bond | H | tetrahydrothiophen(3-CN)-3-yl | Me | Me | C(=O)Me | O |
| D-15773 | H | single bond | Me | bicyclo[1.1.1]pertan-1-yl | Me | Cl | C(=O)Me | O |
| D-15774 | Me | single bond | H | bicyclo[1.1.1]pertan-1-yl | Me | Cl | C(=O)Me | O |
| D-15775 | Me(R) | single bond | H | bicyclo[1.1.1]pertan-1-yl | Me | Cl | C(=O)Me | O |
| D-15776 | Me(S) | single bond | H | bicyclo[1.1.1]pertan-1-yl | Me | Cl | C(=O)Me | O |
| D-15777 | H | single bond | Me | bicyclo[1.1.1]pertan-1-yl | Me | Me | C(=O)Me | O |
| D-15778 | Me | single bond | H | bicyclo[1.1.1 ]pertan-1-yl | Me | Me | C(=O)Me | O |
| D-15779 | Me(R) | single bond | H | bicyclo[1.1.1]pertan-1-yl | Me | Me | C(=O)Me | O |
| D-15780 | Me(S) | single bond | H | bicyclo[1.1.1]pertan-1-yl | Me | Me | C(=O)Me | O |
| D-15781 | H | single bond | Me | *c* -Hex | Me | Cl | C(=O)Me | O |
| D-15782 | Me | single bond | H | *c* -Hex | Me | Cl | C(=O)Me | O |
| D-15783 | Me(R) | single bond | H | *c* -Hex | Me | Cl | C(=O)Me | O |
| D-15784 | Me(S) | single bond | H | *c* -Hex | Me | Cl | C(=O)Me | O |
| D-15785 | H | single bond | Me | *c* -Hex | Me | Me | C(=O)Me | O |
| D-15786 | Me | single bond | H | *c* -Hex | Me | Me | C(=O)Me | O |
| D-15787 | Me(R) | single bond | H | *c* -Hex | Me | Me | C(=O)Me | O |
| D-15788 | Me(S) | single bond | H | *c* -Hex | Me | Me | C(=O)Me | O |
| D-15789 | H | single bond | Me | tetrahydropyran-4-yl | Me | Cl | C(=O)Me | O |
| D-15790 | Me | single bond | H | tetrahydropyran-4-yl | Me | Cl | C(=O)Me | O |
| D-15791 | Me(R) | single bond | H | tetrahydropyran-4-yl | Me | Cl | C(=O)Me | O |
| D-15792 | Me(S) | single bond | H | tetrahydropyran-4-yl | Me | Cl | C(=O)Me | O |
| D-15793 | H | single bond | Me | tetrahydropyran-4-yl | Me | Me | C(=O)Me | O |
| D-15794 | Me | single bond | H | tetrahydropyran-4-yl | Me | Me | C(=O)Me | O |
| D-15795 | Me(R) | single bond | H | tetrahydropyran-4-yl | Me | Me | C(=O)Me | O |
| D-15796 | Me(S) | single bond | H | tetrahydropyran-4-yl | Me | Me | C(=O)Me | O |
| D-15797 | H | single bond | Me | tetrahydrothiopyran-4-yl | Me | Cl | C(=O)Me | O |
| D-15798 | Me | single bond | H | tetrahydrothiopyran-4-yl | Me | Cl | C(=O)Me | O |
| D-15799 | Me(R) | single bond | H | tetrahydrothiopyran-4-yl | Me | Cl | C(=O)Me | O |
| D-15800 | Me(S) | single bond | H | tetrahydrothiopyran-4-yl | Me | Cl | C(=O)Me | O |
| D-15801 | H | single bond | Me | tetrahydrothiopyran-4-yl | Me | Me | C(=O)Me | O |
| D-15802 | Me | single bond | H | tetrahydrothiopyran-4-yl | Me | Me | C(=O)Me | O |
| D-15803 | Me(R) | single bond | H | tetrahydrothiopyran-4-yl | Me | Me | C(=O)Me | O |
| D-15804 | Me(S) | single bond | H | tetrahydrothiopyran-4-yl | Me | Me | C(=O)Me | O |
| D-15805 | H | single bond | Me | *c*-Hex(1-CN) | Me | Cl | C(=O)Me | O |
| D-15806 | Me | single bond | H | *c*-Hex(1-CN) | Me | Cl | C(=O)Me | O |
| D-15807 | Me(R) | single bond | H | *c*-Hex(1-CN) | Me | Cl | C(=O)Me | O |
| D-15808 | Me(S) | single bond | H | *c*-Hex(1-CN) | Me | Cl | C(=O)Me | O |
| D-15809 | H | single bond | Me | *c*-Hex(1-CN) | Me | Me | C(=O)Me | O |
| D-15810 | Me | single bond | H | *c*-Hex(1-CN) | Me | Me | C(=O)Me | O |
| D-15811 | Me(R) | single bond | H | *c*-Hex(1-CN) | Me | Me | C(=O)Me | O |
| D-15812 | Me(S) | single bond | H | *c*-Hex(1-CN) | Me | Me | C(=O)Me | O |
| D-15813 | H | single bond | Me | tetrahydropyran(4-CN)-4-yl | Me | Cl | C(=O)Me | O |
| D-15814 | Me | single bond | H | tetrahydropyran(4-CN)-4-yl | Me | Cl | C(=O)Me | O |

**[Table 1006]**

| Compound Number | Y | W | Z | R¹ | R² | R³ | R¹⁰ | A |
|---|---|---|---|---|---|---|---|---|
| D-15815 | Me(R) | single bond | H | tetrahydropyran(4-CN)-4-yl | Me | Cl | C(=O)Me | O |
| D-15816 | Me(S) | single bond | H | tetrahydropyran(4-CN)-4-yl | Me | Cl | C(=O)Me | O |
| D-15817 | H | single bond | Me | tetrahydropyran(4-CN)-4-yl | Me | Me | C(=O)Me | O |
| D-15818 | Me | single bond | H | tetrahydropyran(4-CN)-4-yl | Me | Me | C(=O)Me | O |
| D-15819 | Me(R) | single bond | H | tetrahydropyran(4-CN)-4-yl | Me | Me | C(=O)Me | O |
| D-15820 | Me(S) | single bond | H | tetrahydropyran(4-CN)-4-yl | Me | Me | C(=O)Me | O |
| D-15821 | H | single bond | Me | tetrahydrothiopyran(4-CN)-4-yl | Me | Cl | C(=O)Me | O |
| D-15822 | Me | single bond | H | tetrahydrothiopyran(4-CN)-4-yl | Me | Cl | C(=O)Me | O |
| D-15823 | Me(R) | single bond | H | tetrahydrothiopyran(4-CN)-4-yl | Me | Cl | C(=O)Me | O |
| D-15824 | Me(S) | single bond | H | tetrahydrothiopyran(4-CN)-4-yl | Me | Cl | C(=O)Me | O |
| D-15825 | H | single bond | Me | tetrahydrothiopyran(4-CN)-4-yl | Me | Me | C(=O)Me | O |
| D-15826 | Me | single bond | H | tetrahydrothiopyran(4-CN)-4-yl | Me | Me | C(=O)Me | O |
| D-15827 | Me(R) | single bond | H | tetrahydrothiopyran(4-CN)-4-yl | Me | Me | C(=O)Me | O |
| D-15828 | Me(S) | single bond | H | tetrahydrothiopyran(4-CN)-4-yl | Me | Me | C(=O)Me | O |
| D-15829 | H | single bond | Me | CH₂(*c* -Pr) | Me | Cl | C(=O)Me | O |
| D-15830 | Me | single bond | H | CH₂(*c* -Pr) | Me | Cl | C(=O)Me | O |
| D-15831 | Me(R) | single bond | H | CH₂(*c* -Pr) | Me | Cl | C(=O)Me | O |
| D-15832 | Me(S) | single bond | H | CH₂(*c* -Pr) | Me | Cl | C(=O)Me | O |
| D-15833 | H | single bond | Me | CH₂(*c* -Pr) | Me | Me | C(=O)Me | O |
| D-15834 | Me | single bond | H | CH₂(*c* -Pr) | Me | Me | C(=O)Me | O |
| D-15835 | Me(R) | single bond | H | CH₂(*c* -Pr) | Me | Me | C(=O)Me | O |
| D-15836 | Me(S) | single bond | H | CH₂(*c* -Pr) | Me | Me | C(=O)Me | O |
| D-15837 | H | single bond | Me | CH₂[*c* -Pr(1-CN)] | Me | Cl | C(=O)Me | O |
| D-15838 | Me | single bond | H | CH₂[*c* -Pr(1-CN)] | Me | Cl | C(=O)Me | O |
| D-15839 | Me(R) | single bond | H | CH₂[*c* -Pr(1-CN)] | Me | Cl | C(=O)Me | O |
| D-15840 | Me(S) | single bond | H | CH₂[*c* -Pr(1-CN)] | Me | Cl | C(=O)Me | O |
| D-15841 | H | single bond | Me | CH₂[*c* -Pr(1-CN)] | Me | Me | C(=O)Me | O |
| D-15842 | Me | single bond | H | CH₂[*c* -Pr(1-CN)] | Me | Me | C(=O)Me | O |
| D-15843 | Me(R) | single bond | H | CH₂[*c* -Pr(1-CN)] | Me | Me | C(=O)Me | O |
| D-15844 | Me(S) | single bond | H | CH₂[*c* -Pr(1-CN)] | Me | Me | C(=O)Me | O |
| D-15845 | H | single bond | Me | CH(Me)(*c*-Pr) | Me | Cl | C(=O)Me | O |
| D-15846 | Me | single bond | H | CH(Me)(*c*-Pr) | Me | Cl | C(=O)Me | O |
| D-15847 | Me(R) | single bond | H | CH(Me)(*c*-Pr) | Me | Cl | C(=O)Me | O |
| D-15848 | Me(S) | single bond | H | CH(Me)(*c*-Pr) | Me | Cl | C(=O)Me | O |
| D-15849 | H | single bond | Me | CH(Me)(*c*-Pr) | Me | Me | C(=O)Me | O |
| D-15850 | Me | single bond | H | CH(Me)(*c*-Pr) | Me | Me | C(=O)Me | O |
| D-15851 | Me(R) | single bond | H | CH(Me)(*c*-Pr) | Me | Me | C(=O)Me | O |
| D-15852 | Me(S) | single bond | H | CH(Me)(*c* -Pr) | Me | Me | C(=O)Me | O |
| D-15853 | H | single bond | Me | CH(Me)CH₂OMe | Me | Cl | C(=O)Me | O |
| D-15854 | Me | single bond | H | CH(Me)CH₂OMe | Me | Cl | C(=O)Me | O |
| D-15855 | Me(R) | single bond | H | CH(Me)CH₂OMe | Me | Cl | C(=O)Me | O |
| D-15856 | Me(S) | single bond | H | CH(Me)CH₂OMe | Me | Cl | C(=O)Me | O |
| D-15857 | H | single bond | Me | CH(Me)CH₂OMe | Me | Me | C(=O)Me | O |
| D-15858 | Me | single bond | H | CH(Me)CH₂OMe | Me | Me | C(=O)Me | O |

**[Table 1007]**

| Compound Number | Y | W | Z | R¹ | R² | R³ | R¹⁰ | A |
|---|---|---|---|---|---|---|---|---|
| D-15859 | Me(R) | single bond | H | CH(Me)CH₂OMe | Me | Me | C(=O)Me | O |
| D-15860 | Me(S) | single bond | H | CH(Me)CH₂OMe | Me | Me | C(=O)Me | O |
| D-15861 | H | single bond | Me | CH₂CH=CH₂ | Me | Cl | C(=O)Me | O |
| D-15862 | Me | single bond | H | CH₂CH=CH₂ | Me | Cl | C(=O)Me | O |
| D-15863 | Me(R) | single bond | H | CH₂CH=CH₂ | Me | Cl | C(=O)Me | O |
| D-15864 | Me(S) | single bond | H | CH₂CH=CH₂ | Me | Cl | C(=O)Me | O |
| D-15865 | H | single bond | Me | CH₂CH=CH₂ | Me | Me | C(=O)Me | O |
| D-15866 | Me | single bond | H | CH₂CH=CH₂ | Me | Me | C(=O)Me | O |
| D-15867 | Me(R) | single bond | H | CH₂CH=CH₂ | Me | Me | C(=O)Me | O |
| D-15868 | Me(S) | single bond | H | CH₂CH=CH₂ | Me | Me | C(=O)Me | O |
| D-15869 | H | single bond | Me | CH₂C≡CH | Me | Cl | C(=O)Me | O |
| D-15870 | Me | single bond | H | CH₂C≡CH | Me | Cl | C(=O)Me | O |
| D-15871 | Me(R) | single bond | H | CH₂C≡CH | Me | Cl | C(=O)Me | O |
| D-15872 | Me(S) | single bond | H | CH₂C≡CH | Me | Cl | C(=O)Me | O |
| D-15873 | H | single bond | Me | CH₂C≡CH | Me | Me | C(=O)Me | O |
| D-15874 | Me | single bond | H | CH₂C≡CH | Me | Me | C(=O)Me | O |
| D-15875 | Me(R) | single bond | H | CH₂C≡CH | Me | Me | C(=O)Me | O |
| D-15876 | Me(S) | single bond | H | CH₂C≡CH | Me | Me | C(=O)Me | O |
| D-15877 | H | single bond | Me | CH₂CN | Me | Cl | C(=O)Me | O |
| D-15878 | Me | single bond | H | CH₂CN | Me | Cl | C(=O)Me | O |
| D-15879 | Me(R) | single bond | H | CH₂CN | Me | Cl | C(=O)Me | O |
| D-15880 | Me(S) | single bond | H | CH₂CN | Me | Cl | C(=O)Me | O |
| D-15881 | H | single bond | Me | CH₂CN | Me | Me | C(=O)Me | O |
| D-15882 | Me | single bond | H | CH₂CN | Me | Me | C(=O)Me | O |
| D-15883 | Me(R) | single bond | H | CH₂CN | Me | Me | C(=O)Me | O |
| D-15884 | Me(S) | single bond | H | CH₂CN | Me | Me | C(=O)Me | O |
| D-15885 | H | single bond | Me | CH(Me)CN | Me | Cl | C(=O)Me | O |
| D-15886 | Me | single bond | H | CH(Me)CN | Me | Cl | C(=O)Me | O |
| D-15887 | Me(R) | single bond | H | CH(Me)CN | Me | Cl | C(=O)Me | O |
| D-15888 | Me(S) | single bond | H | CH(Me)CN | Me | Cl | C(=O)Me | O |
| D-15889 | H | single bond | Me | CH(Me)CN | Me | Me | C(=O)Me | O |
| D-15890 | Me | single bond | H | CH(Me)CN | Me | Me | C(=O)Me | O |
| D-15891 | Me(R) | single bond | H | CH(Me)CN | Me | Me | C(=O)Me | O |
| D-15892 | Me(S) | single bond | H | CH(Me)CN | Me | Me | C(=O)Me | O |
| D-15893 | H | single bond | Me | C(Me)₂CN | Me | Cl | C(=O)Me | O |
| D-15894 | Me | single bond | H | C(Me)₂CN | Me | Cl | C(=O)Me | O |
| D-15895 | Me(R) | single bond | H | C(Me)₂CN | Me | Cl | C(=O)Me | O |
| D-15896 | Me(S) | single bond | H | C(Me)₂CN | Me | Cl | C(=O)Me | O |
| D-15897 | H | single bond | Me | C(Me)₂CN | Me | Me | C(=O)Me | O |
| D-15898 | Me | single bond | H | C(Me)₂CN | Me | Me | C(=O)Me | O |
| D-15899 | Me(R) | single bond | H | C(Me)₂CN | Me | Me | C(=O)Me | O |
| D-15900 | Me(S) | single bond | H | C(Me)₂CN | Me | Me | C(=O)Me | O |
| D-15901 | H | single bond | Me | C(Me)(*i* -Pr)CN | Me | Cl | C(=O)Me | O |
| D-15902 | Me | single bond | H | C(Me)(*i* -Pr)CN | Me | Cl | C(=O)Me | O |

**[Table 1008]**

| Compound Number | Y | W | Z | R¹ | R² | R³ | R¹⁰ | A |
|---|---|---|---|---|---|---|---|---|
| D-15903 | Me(R) | single bond | H | C(Me)(*i* -Pr)CN | Me | Cl | C(=O)Me | O |
| D-15904 | Me(S) | single bond | H | C(Me)(*i* -Pr)CN | Me | Cl | C(=O)Me | O |
| D-15905 | H | single bond | Me | C(Me)(*i* -Pr)CN | Me | Me | C(=O)Me | O |
| D-15906 | Me | single bond | H | C(Me)(*i* -Pr)CN | Me | Me | C(=O)Me | O |
| D-15907 | Me(R) | single bond | H | C(Me)(*i* -Pr)CN | Me | Me | C(=O)Me | O |
| 0-15908 | Me(S) | single bond | H | C(Me)(*i* -Pr)CN | Me | Me | C(=O)Me | O |
| D-15909 | H | single bond | Me | OMe | Me | Cl | C(=O)Me | O |
| D-15910 | Me | single bond | H | OMe | Me | Cl | C(=O)Me | O |
| D-15911 | Me(R) | single bond | H | OMe | Me | Cl | C(=O)Me | O |
| D-15912 | Me(S) | single bond | H | OMe | Me | Cl | C(=O)Me | O |
| D-15913 | H | single bond | Me | OMe | Me | Me | C(=O)Me | O |
| D-15914 | Me | single bond | H | OMe | Me | Me | C(=O)Me | O |
| D-15915 | Me(R) | single bond | H | OMe | Me | Me | C(=O)Me | O |
| D-15916 | Me(S) | single bond | H | OMe | Me | Me | C(=O)Me | O |
| D-15917 | H | single bond | Me | CH=NOMe | Me | Cl | C(=O)Me | O |
| D-15918 | Me | single bond | H | CH=NOMe | Me | Cl | C(=O)Me | O |
| D-15919 | Me(R) | single bond | H | CH=NOMe | Me | Cl | C(=O)Me | O |
| D-15920 | Me(S) | single bond | H | CH=NOMe | Me | Cl | C(=O)Me | O |
| D-15921 | H | single bond | Me | CH=NOMe | Me | Me | C(=O)Me | O |
| D-15922 | Me | single bond | H | CH=NOMe | Me | Me | C(=O)Me | O |
| D-15923 | Me(R) | single bond | H | CH=NOMe | Me | Me | C(=O)Me | O |
| D-15924 | Me(S) | single bond | H | CH=NOMe | Me | Me | C(=O)Me | O |
| D-15925 | H | single bond | Me | morpholin-4-yl | Me | Cl | C(=O)Me | O |
| D-15926 | Me | single bond | H | morpholin-4-yl | Me | Cl | C(=O)Me | O |
| D-15927 | Me(R) | single bond | H | morpholin-4-yl | Me | Cl | C(=O)Me | O |
| D-15928 | Me(S) | single bond | H | morpholin-4-yl | Me | Cl | C(=O)Me | O |
| D-15929 | H | single bond | Me | morpholin-4-yl | Me | Me | C(=O)Me | O |
| D-15930 | Me | single bond | H | morpholin-4-yl | Me | Me | C(=O)Me | O |
| D-15931 | Me(R) | single bond | H | morpholin-4-yl | Me | Me | C(=O)Me | O |
| D-15932 | Me(S) | single bond | H | morpholin-4-yl | Me | Me | C(=O)Me | O |
| D-15933 | H | single bond | Me | Ph | Me | Cl | C(=O)Me | O |
| D-15934 | Me | single bond | H | Ph | Me | Cl | C(=O)Me | O |
| D-15935 | Me(R) | single bond | H | Ph | Me | Cl | C(=O)Me | O |
| D-15936 | Me(S) | single bond | H | Ph | Me | Cl | C(=O)Me | O |
| D-15937 | H | single bond | Me | Ph | Me | Me | C(=O)Me | O |
| D-15938 | Me | single bond | H | Ph | Me | Me | C(=O)Me | O |
| D-15939 | Me(R) | single bond | H | Ph | Me | Me | C(=O)Me | O |
| D-15940 | Me(S) | single bond | H | Ph | Me | Me | C(=O)Me | O |
| D-15941 | H | single bond | Me | CH₂Ph | Me | Cl | C(=O)Me | O |
| D-15942 | Me | single bond | H | CH₂Ph | Me | Cl | C(=O)Me | O |
| D-15943 | Me(R) | single bond | H | CH₂Ph | Me | Cl | C(=O)Me | O |
| D-15944 | Me(S) | single bond | H | CH₂Ph | Me | Cl | C(=O)Me | O |
| D-15945 | H | single bond | Me | CH₂Ph | Me | Me | C(=O)Me | O |
| D-15946 | Me | single bond | H | CH₂Ph | Me | Me | C(=O)Me | O |

**[Table 1009]**

| Compound Number | Y | W | Z | R¹ | R² | R³ | R¹⁰ | A |
|---|---|---|---|---|---|---|---|---|
| D-15947 | Me(R) | single bond | H | CH₂Ph | Me | Me | C(=O)Me | O |
| D-15948 | Me(S) | single bond | H | CH₂Ph | Me | Me | C(=O)Me | O |
| D-15949 | H | single bond | Me | thiazole-2-yl | Me | Cl | C(=O)Me | O |
| D-15950 | Me | single bond | H | thiazole-2-yl | Me | Cl | C(=O)Me | O |
| D-15951 | Me(R) | single bond | H | thiazole-2-yl | Me | Cl | C(=O)Me | O |
| D-15952 | Me(S) | single bond | H | thiazole-2-yl | Me | Cl | C(=O)Me | O |
| D-15953 | H | single bond | Me | thiazole-2-yl | Me | Me | C(=O)Me | O |
| D-15954 | Me | single bond | H | thiazole-2-yl | Me | Me | C(=O)Me | O |
| D-15955 | Me(R) | single bond | H | thiazole-2-yl | Me | Me | C(=O)Me | O |
| D-15956 | Me(S) | single bond | H | thiazole-2-yl | Me | Me | C(=O)Me | O |
| D-15957 | H | single bond | Me | 1-Me-pyrazol-3-yl | Me | Cl | C(=O)Me | O |
| D-15958 | Me | single bond | H | 1-Me-pyrazol-3-yl | Me | Cl | C(=O)Me | O |
| D-15959 | Me(R) | single bond | H | 1-Me-pyrazol-3-yl | Me | Cl | C(=O)Me | O |
| D-15960 | Me(S) | single bond | H | 1-Me-pyrazol-3-yl | Me | Cl | C(=O)Me | O |
| D-15961 | H | single bond | Me | 1-Me-pyrazol-3-yl | Me | Me | C(=O)Me | O |
| D-15962 | Me | single bond | H | 1-Me-pyrazol-3-yl | Me | Me | C(=O)Me | O |
| D-15963 | Me(R) | single bond | H | 1-Me-pyrazol-3-yl | Me | Me | C(=O)Me | O |
| D-15964 | Me(S) | single bond | H | 1-Me-pyrazol-3-yl | Me | Me | C(=O)Me | O |
| D-15965 | H | single bond | Me | 1-Me-pyrazol-5-yl | Me | Cl | C(=O)Me | O |
| D-15966 | Me | single bond | H | 1-Me-pyrazol-5-yl | Me | Cl | C(=O)Me | O |
| D-15967 | Me(R) | single bond | H | 1-Me-pyrazol-5-yl | Me | Cl | C(=O)Me | O |
| D-15968 | Me(S) | single bond | H | 1-Me-pyrazol-5-yl | Me | Cl | C(=O)Me | O |
| D-15969 | H | single bond | Me | 1-Me-pyrazol-5-yl | Me | Me | C(=O)Me | O |
| D-15970 | Me | single bond | H | 1-Me-pyrazol-5-yl | Me | Me | C(=O)Me | O |
| D-15971 | Me(R) | single bond | H | 1-Me-pyrazol-5-yl | Me | Me | C(=O)Me | O |
| D-15972 | Me(S) | single bond | H | 1-Me-pyrazol-5-yl | Me | Me | C(=O)Me | O |
| D-15973 | H | single bond | Me | CH₂(pyrimidin-2-yl) | Me | Cl | C(=O)Me | O |
| D-15974 | Me | single bond | H | CH₂(pyrimidin-2-yl) | Me | Cl | C(=O)Me | O |
| D-15975 | Me(R) | single bond | H | CH₂(pyrimidin-2-yl) | Me | Cl | C(=O)Me | O |
| D-15976 | Me(S) | single bond | H | CH₂(pyrimidin-2-yl) | Me | Cl | C(=O)Me | O |
| D-15977 | H | single bond | Me | CH₂(pyrimidin-2-yl) | Me | Me | C(=O)Me | O |
| D-15978 | Me | single bond | H | CH₂(pyrimidin-2-yl) | Me | Me | C(=O)Me | O |
| D-15979 | Me(R) | single bond | H | CH₂(pyrimidin-2-yl) | Me | Me | C(=O)Me | O |
| D-15980 | Me(S) | single bond | H | CH₂(pyrimidin-2-yl) | Me | Me | C(=O)Me | O |
| D-15981 | H | single bond | Me | Me | C(=O)Me | Cl | H | O |
| D-15982 | Me | single bond | H | Me | C(=O)Me | Cl | H | O |
| D-15983 | Me(R) | single bond | H | Me | C(=O)Me | Cl | H | O |
| D-15984 | Me(S) | single bond | H | Me | C(=O)Me | Cl | H | O |
| D-15985 | H | single bond | Me | Me | C(=O)Me | Me | H | O |
| D-15986 | Me | single bond | H | Me | C(=O)Me | Me | H | O |
| D-15987 | Me(R) | single bond | H | Me | C(=O)Me | Me | H | O |
| D-15988 | Me(S) | single bond | H | Me | C(=O)Me | Me | H | O |
| D-15989 | H | single bond | Me | CH₂CHF₂ | C(=O)Me | Cl | H | O |
| D-15990 | Me | single bond | H | CH₂CHF₂ | C(=O)Me | Cl | H | O |

**[Table 1010]**

| Compound Number | Y | W | Z | R¹ | R² | R³ | R¹⁰ | A |
|---|---|---|---|---|---|---|---|---|
| D-15991 | Me(R) | single bond | H | CH₂CHF₂ | C(=O)Me | Cl | H | O |
| D-15992 | Me(S) | single bond | H | CH₂CHF₂ | C(=O)Me | Cl | H | O |
| D-15993 | H | single bond | Me | CH₂CHF₂ | C(=O)Me | Me | H | O |
| D-15994 | Me | single bond | H | CH₂CHF₂ | C(=O)Me | Me | H | O |
| D-15995 | Me(R) | single bond | H | CH₂CHF₂ | C(=O)Me | Me | H | O |
| D-15996 | Me(S) | single bond | H | CH₂CHF₂ | C(=O)Me | Me | H | O |
| D-15997 | H | single bond | Me | *n*-Pr | C(=O)Me | Cl | H | O |
| D-15998 | Me | single bond | H | *n*-Pr | C(=O)Me | Cl | H | O |
| D-15999 | Me(R) | single bond | H | *n*-Pr | C(=O)Me | Cl | H | O |
| D-16000 | Me(S) | single bond | H | *n*-Pr | C(=O)Me | Cl | H | O |
| D-16001 | H | single bond | Me | *n*-Pr | C(=O)Me | Me | H | O |
| D-16002 | Me | single bond | H | *n*-Pr | C(=O)Me | Me | H | O |
| D-16003 | Me(R) | single bond | H | *n*-Pr | C(=O)Me | Me | H | O |
| D-16004 | Me(S) | single bond | H | *n*-Pr | C(=O)Me | Me | H | O |
| D-16005 | H | single bond | Me | *i*-Pr | C(=O)Me | Cl | H | O |
| D-16006 | Me | single bond | H | *i*-Pr | C(=O)Me | Cl | H | O |
| D-16007 | Me(R) | single bond | H | *i*-Pr | C(=O)Me | Cl | H | O |
| D-16008 | Me(S) | single bond | H | *i*-Pr | C(=O)Me | Cl | H | O |
| D-16009 | H | single bond | Me | *i*-Pr | C(=O)Me | Me | H | O |
| D-16010 | Me | single bond | H | *i*-Pr | C(=O)Me | Me | H | O |
| D-16011 | Me(R) | single bond | H | *i*-Pr | C(=O)Me | Me | H | O |
| D-16012 | Me(S) | single bond | H | *i*-Pr | C(=O)Me | Me | H | O |
| D-16013 | H | single bond | Me | *c*-Pr(1-F) | C(=O)Me | Cl | H | O |
| D-16014 | Me | single bond | H | *c*-Pr(1-F) | C(=O)Me | Cl | H | O |
| D-16015 | Me(R) | single bond | H | *c*-Pr(1-F) | C(=O)Me | Cl | H | O |
| D-16016 | Me(S) | single bond | H | *c*-Pr(1-F) | C(=O)Me | Cl | H | O |
| D-16017 | H | single bond | Me | *c*-Pr(1-F) | C(=O)Me | Me | H | O |
| D-16018 | Me | single bond | H | *c*-Pr(1-F) | C(=O)Me | Me | H | O |
| D-16019 | Me(R) | single bond | H | *c*-Pr(1-F) | C(=O)Me | Me | H | O |
| D-16020 | Me(S) | single bond | H | *c*-Pr(1-F) | C(=O)Me | Me | H | O |
| D-16021 | H | single bond | Me | *c*-Pr(2,2-F₂) | C(=O)Me | Cl | H | O |
| D-16022 | Me | single bond | H | *c*-Pr(2,2-F₂) | C(=O)Me | Cl | H | O |
| D-16023 | Me(R) | single bond | H | *c*-Pr(2,2-F₂) | C(=O)Me | Cl | H | O |
| D-16024 | Me(S) | single bond | H | *c*-Pr(2,2-F₂) | C(=O)Me | Cl | H | O |
| D-16025 | H | single bond | Me | *c*-Pr(2,2-F₂) | C(=O)Me | Me | H | O |
| D-16026 | Me | single bond | H | *c*-Pr(2,2-F₂) | C(=O)Me | Me | H | O |
| D-16027 | Me(R) | single bond | H | *c*-Pr(2,2-F₂) | C(=O)Me | Me | H | O |
| D-16028 | Me(S) | single bond | H | *c*-Pr(2,2-F₂) | C(=O)Me | Me | H | O |
| D-16029 | H | single bond | Me | *c*-Pr(1-Cl) | C(=O)Me | Cl | H | O |
| D-16030 | Me | single bond | H | *c*-Pr(1-Cl) | C(=O)Me | Cl | H | O |
| D-16031 | Me(R) | single bond | H | *c*-Pr(1-Cl) | C(=O)Me | Cl | H | O |
| D-16032 | Me(S) | single bond | H | *c*-Pr(1-Cl) | C(=O)Me | Cl | H | O |
| D-16033 | H | single bond | Me | *c*-Pr(1-Cl) | C(=O)Me | Me | H | O |
| D-16034 | Me | single bond | H | *c*-Pr(1-Cl) | C(=O)Me | Me | H | O |

**[Table 1011]**

| Compound Number | Y | W | Z | R¹ | R² | R³ | R¹⁰ | A |
|---|---|---|---|---|---|---|---|---|
| D-16035 | Me(R) | single bond | H | *c*-Pr(1-Cl) | C(=O)Me | Me | H | O |
| D-16036 | Me(S) | single bond | H | *c*-Pr(1-Cl) | C(=O)Me | Me | H | O |
| D-16037 | H | single bond | Me | *c*-Pr(1-Br) | C(=O)Me | Cl | H | O |
| D-16038 | Me | single bond | H | *c*-Pr(1-Br) | C(=O)Me | Cl | H | O |
| D-16039 | Me(R) | single bond | H | *c*-Pr(1-Br) | C(=O)Me | Cl | H | O |
| D-16040 | Me(S) | single bond | H | *c*-Pr(1-Br) | C(=O)Me | Cl | H | O |
| D-16041 | H | single bond | Me | *c*-Pr(1-Br) | C(=O)Me | Me | H | O |
| D-16042 | Me | single bond | H | *c*-Pr(1-Br) | C(=O)Me | Me | H | O |
| D-16043 | Me(R) | single bond | H | *c*-Pr(1-Br) | C(=O)Me | Me | H | O |
| D-16044 | Me(S) | single bond | H | *c*-Pr(1-Br) | C(=O)Me | Me | H | O |
| D-16045 | H | single bond | Me | *c*-Pr(1-Me) | C(=O)Me | Cl | H | O |
| D-16046 | Me | single bond | H | *c*-Pr(1-Me) | C(=O)Me | Cl | H | O |
| D-16047 | Me(R) | single bond | H | *c*-Pr(1-Me) | C(=O)Me | Cl | H | O |
| D-16048 | Me(S) | single bond | H | *c*-Pr(1-Me) | C(=O)Me | Cl | H | O |
| D-16049 | H | single bond | Me | *c*-Pr(1-Me) | C(=O)Me | Me | H | O |
| D-16050 | Me | single bond | H | *c*-Pr(1-Me) | C(=O)Me | Me | H | O |
| D-16051 | Me(R) | single bond | H | *c*-Pr(1-Me) | C(=O)Me | Me | H | O |
| D-16052 | Me(S) | single bond | H | *c*-Pr(1-Me) | C(=O)Me | Me | H | O |
| D-16053 | H | single bond | Me | *c*-Pr(1-CH₂OH) | C(=O)Me | Cl | H | O |
| D-16054 | Me | single bond | H | *c*-Pr(1-CH₂OH) | C(=O)Me | Cl | H | O |
| D-16055 | Me(R) | single bond | H | *c*-Pr(1-CH₂OH) | C(=O)Me | Cl | H | O |
| D-16056 | Me(S) | single bond | H | *c*-Pr(1-CH₂OH) | C(=O)Me | Cl | H | O |
| D-16057 | H | single bond | Me | *c*-Pr(1-CH₂OH) | C(=O)Me | Me | H | O |
| D-16058 | Me | single bond | H | *c*-Pr(1-CH₂OH) | C(=O)Me | Me | H | O |
| D-16059 | Me(R) | single bond | H | *c*-Pr(1-CH₂OH) | C(=O)Me | Me | H | O |
| D-16060 | Me(S) | single bond | H | *c*-Pr(1-CH₂OH) | C(=O)Me | Me | H | O |
| D-16061 | H | single bond | Me | *c*-Pr[1-CH₂OS(=O)₂Me] | C(=O)Me | Cl | H | O |
| D-16062 | Me | single bond | H | *c*-Pr[1-CH₂OS(=O)₂Me] | C(=O)Me | Cl | H | O |
| D-16063 | Me(R) | single bond | H | *c*-Pr[1-CH₂OS(=O)₂Me] | C(=O)Me | Cl | H | O |
| D-16064 | Me(S) | single bond | H | *c*-Pr[1-CH₂OS(=O)₂Me] | C(=O)Me | Cl | H | O |
| D-16065 | H | single bond | Me | *c*-Pr[1-CH₂OS(=O)₂Me] | C(=O)Me | Me | H | O |
| D-16066 | Me | single bond | H | *c*-Pr[1-CH₂OS(=O)₂Me] | C(=O)Me | Me | H | O |
| D-16067 | Me(R) | single bond | H | *c*-Pr[1-CH₂OS(=O)₂Me] | C(=O)Me | Me | H | O |
| D-16068 | Me(S) | single bond | H | *c*-Pr[1-CH₂OS(=O)₂Me] | C(=O)Me | Me | H | O |
| D-16069 | H | single bond | Me | *c*-Pr(1-CH₂OMe) | C(=O)Me | Cl | H | O |
| D-16070 | Me | single bond | H | *c*-Pr(1-CH₂OMe) | C(=O)Me | Cl | H | O |
| D-16071 | Me(R) | single bond | H | *c*-Pr(1-CH₂OMe) | C(=O)Me | Cl | H | O |
| D-16072 | Me(S) | single bond | H | *c*-Pr(1-CH₂OMe) | C(=O)Me | Cl | H | O |
| D-16073 | H | single bond | Me | *c*-Pr(1-CH₂OMe) | C(=O)Me | Me | H | O |
| D-16074 | Me | single bond | H | *c*-Pr(1-CH₂OMe) | C(=O)Me | Me | H | O |
| D-16075 | Me(R) | single bond | H | *c*-Pr(1-CH₂OMe) | C(=O)Me | Me | H | O |
| 0-16076 | Me(S) | single bond | H | *c*-Pr(1-CH₂OMe) | C(=O)Me | Me | H | O |
| D-16077 | H | single bond | Me | *c*-Pr(1-CH₂OCN) | C(=O)Me | Cl | H | O |
| D-16078 | Me | single bond | H | *c*-Pr(1-CH₂OCN) | C(=O)Me | Cl | H | O |

**[Table 1012]**

| Compound Number | Y | W | Z | R¹ | R² | R³ | R¹⁰ | A |
|---|---|---|---|---|---|---|---|---|
| 0-16079 | Me(R) | single bond | H | *c*-Pr(1-CH₂OCN) | C(=O)Me | Cl | H | O |
| D-16080 | Me(S) | single bond | H | *c*-Pr(1-CH₂OCN) | C(=O)Me | Cl | H | O |
| D-16081 | H | single bond | Me | *c*-Pr(1-CH₂OCN) | C(=O)Me | Me | H | O |
| D-16082 | Me | single bond | H | *c*-Pr(1-CH₂OCN) | C(=O)Me | Me | H | O |
| D-16083 | Me(R) | single bond | H | *c*-Pr(1-CH₂OCN) | C(=O)Me | Me | H | O |
| D-16084 | Me(S) | single bond | H | *c*-Pr(1-CH₂OCN) | C(=O)Me | Me | H | O |
| D-16085 | H | single bond | Me | *c*-Pr(1-CH₂SMe) | C(=O)Me | Cl | H | O |
| D-16086 | Me | single bond | H | *c*-Pr(1-CH₂SMe) | C(=O)Me | Cl | H | O |
| D-16087 | Me(R) | single bond | H | *c*-Pr(1-CH₂SMe) | C(=O)Me | Cl | H | O |
| D-16088 | Me(S) | single bond | H | *c*-Pr(1-CH₂SMe) | C(=O)Me | Cl | H | O |
| D-16089 | H | single bond | Me | *c*-Pr(1-CH₂SMe) | C(=O)Me | Me | H | O |
| D-16090 | Me | single bond | H | *c*-Pr(1-CH₂SMe) | C(=O)Me | Me | H | O |
| D-16091 | Me(R) | single bond | H | *c*-Pr(1-CH₂SMe) | C(=O)Me | Me | H | O |
| D-16092 | Me(S) | single bond | H | *c*-Pr(1-CH₂SMe) | C(=O)Me | Me | H | O |
| D-16093 | H | single bond | Me | *c*-Pr[1-CH₂S(=O)Me] | C(=O)Me | Cl | H | O |
| D-16094 | Me | single bond | H | *c*-Pr[1-CH₂S(=O)Me] | C(=O)Me | Cl | H | O |
| D-16095 | Me(R) | single bond | H | *c*-Pr[1-CH₂S(=O)Me] | C(=O)Me | Cl | H | O |
| D-16096 | Me(S) | single bond | H | *c*-Pr[1-CH₂S(=O)Me] | C(=O)Me | Cl | H | O |
| D-16097 | H | single bond | Me | *c*-Pr[1-CH₂S(=O)Me] | C(=O)Me | Me | H | O |
| D-16098 | Me | single bond | H | *c*-Pr[1-CH₂S(=O)Me] | C(=O)Me | Me | H | O |
| 0-16099 | Me(R) | single bond | H | *c*-Pr[1-CH₂S(=O)Me] | C(=O)Me | Me | H | O |
| D-16100 | Me(S) | single bond | H | *c*-Pr[1-CH₂S(=O)Me] | C(=O)Me | Me | H | O |
| D-16101 | H | single bond | Me | *c*-Pr[1-CH₂S(=O)₂Me] | C(=O)Me | Cl | H | O |
| D-16102 | Me | single bond | H | *c*-Pr[1-CH₂S(=O)₂Me] | C(=O)Me | Cl | H | O |
| D-16103 | Me(R) | single bond | H | *c*-Pr[1-CH₂S(=O)₂Me] | C(=O)Me | Cl | H | O |
| D-16104 | Me(S) | single bond | H | *c*-Pr[1-CH₂S(=O)₂Me] | C(=O)Me | Cl | H | O |
| D-16105 | H | single bond | Me | *c*-Pr[1-CH₂S(=O)₂Me] | C(=O)Me | Me | H | O |
| D-16106 | Me | single bond | H | *c*-Pr[1-CH₂S(=O)₂Me] | C(=O)Me | Me | H | O |
| D-16107 | Me(R) | single bond | H | *c*-Pr[1-CH₂S(=O)₂Me] | C(=O)Me | Me | H | O |
| D-16108 | Me(S) | single bond | H | *c*-Pr[1-CH₂S(=O)₂Me] | C(=O)Me | Me | H | O |
| D-16109 | H | single bond | Me | *c*-Pr(1-CH₂F) | C(=O)Me | Cl | H | O |
| D-16110 | Me | single bond | H | *c*-Pr(1-CH₂F) | C(=O)Me | Cl | H | O |
| D-16111 | Me(R) | single bond | H | *c*-Pr(1-CH₂F) | C(=O)Me | Cl | H | O |
| D-16112 | Me(S) | single bond | H | *c*-Pr(1-CH₂F) | C(=O)Me | Cl | H | O |
| D-16113 | H | single bond | Me | *c*-Pr(1-CH₂F) | C(=O)Me | Me | H | O |
| D-16114 | Me | single bond | H | *c*-Pr(1-CH₂F) | C(=O)Me | Me | H | O |
| D-16115 | Me(R) | single bond | H | *c*-Pr(1-CH₂F) | C(=O)Me | Me | H | O |
| D-16116 | Me(S) | single bond | H | *c*-Pr(1-CH₂F) | C(=O)Me | Me | H | O |
| D-16117 | H | single bond | Me | *c*-Pr(1-CHF₂) | C(=O)Me | Cl | H | O |
| D-16118 | Me | single bond | H | *c*-Pr(1-CHF₂) | C(=O)Me | Cl | H | O |
| D-16119 | Me(R) | single bond | H | *c*-Pr(1-CHF₂) | C(=O)Me | Cl | H | O |
| D-16120 | Me(S) | single bond | H | *c*-Pr(1-CHF₂) | C(=O)Me | Cl | H | O |
| D-16121 | H | single bond | Me | *c*-Pr(1-CHF₂) | C(=O)Me | Me | H | O |
| D-16122 | Me | single bond | H | *c*-Pr(1-CHF₂) | C(=O)Me | Me | H | O |

**[Table 1013]**

| Compound Number | Y | W | Z | R¹ | R² | R³ | R¹⁰ | A |
|---|---|---|---|---|---|---|---|---|
| D-16123 | Me(R) | single bond | H | *c*-Pr(1-CHF₂) | C(=O)Me | Me | H | O |
| D-16124 | Me(S) | single bond | H | *c*-Pr(1-CHF₂) | C(=O)Me | Me | H | O |
| D-16125 | H | single bond | Me | *c*-Pr(1-CF₃) | C(=O)Me | Cl | H | O |
| D-16126 | Me | single bond | H | *c*-Pr(1-CF₃) | C(=O)Me | Cl | H | O |
| D-16127 | Me(R) | single bond | H | *c*-Pr(1-CF₃) | C(=O)Me | Cl | H | O |
| D-16128 | Me(S) | single bond | H | *c*-Pr(1-CF₃) | C(=O)Me | Cl | H | O |
| D-16129 | H | single bond | Me | *c*-Pr(1-CF₃) | C(=O)Me | Me | H | O |
| D-16130 | Me | single bond | H | *c*-Pr(1-CF₃) | C(=O)Me | Me | H | O |
| D-16131 | Me(R) | single bond | H | *c*-Pr(1-CF₃) | C(=O)Me | Me | H | O |
| D-16132 | Me(S) | single bond | H | *c*-Pr(1-CF₃) | C(=O)Me | Me | H | O |
| D-16133 | H | single bond | Me | *c*-Pr(1-C≡CH) | C(=O)Me | Cl | H | O |
| D-16134 | Me | single bond | H | *c*-Pr(1-C≡CH) | C(=O)Me | Cl | H | O |
| D-16135 | Me(R) | single bond | H | *c*-Pr(1-C≡CH) | C(=O)Me | Cl | H | O |
| D-16136 | Me(S) | single bond | H | *c*-Pr(1-C≡CH) | C(=O)Me | Cl | H | O |
| D-16137 | H | single bond | Me | *c*-Pr(1-C≡CH) | C(=O)Me | Me | H | O |
| D-16138 | Me | single bond | H | *c*-Pr(1-C≡CH) | C(=O)Me | Me | H | O |
| D-16139 | Me(R) | single bond | H | *c*-Pr(1-C≡CH) | C(=O)Me | Me | H | O |
| D-16140 | Me(S) | single bond | H | *c*-Pr(1-C≡CH) | C(=O)Me | Me | H | O |
| D-16141 | H | single bond | Me | *c*-Pr[1-C(=O)OEt] | C(=O)Me | Cl | H | O |
| D-16142 | Me | single bond | H | *c*-Pr[1-C(=O)OEt] | C(=O)Me | Cl | H | O |
| D-16143 | Me(R) | single bond | H | *c*-Pr[1-C(=O)OEt] | C(=O)Me | Cl | H | O |
| D-16144 | Me(S) | single bond | H | *c*-Pr[1-C(=O)OEt] | C(=O)Me | Cl | H | O |
| D-16145 | H | single bond | Me | *c*-Pr[1-C(=O)OEt] | C(=O)Me | Me | H | O |
| D-16146 | Me | single bond | H | *c*-Pr[1-C(=O)OEt] | C(=O)Me | Me | H | O |
| D-16147 | Me(R) | single bond | H | *c*-Pr[1-C(=O)OEt] | C(=O)Me | Me | H | O |
| D-16148 | Me(S) | single bond | H | *c*-Pr[1-C(=O)OEt] | C(=O)Me | Me | H | O |
| D-16149 | H | single bond | Me | *c*-Pr[1-C(=O)OH] | C(=O)Me | Cl | H | O |
| D-16150 | Me | single bond | H | *c*-Pr[1-C(=O)OH] | C(=O)Me | Cl | H | O |
| D-16151 | Me(R) | single bond | H | *c*-Pr[1-C(=O)OH] | C(=O)Me | Cl | H | O |
| D-16152 | Me(S) | single bond | H | *c*-Pr[1-C(=O)OH] | C(=O)Me | Cl | H | O |
| D-16153 | H | single bond | Me | *c*-Pr[1-C(=O)OH] | C(=O)Me | Me | H | O |
| D-16154 | Me | single bond | H | *c*-Pr[1-C(=O)OH] | C(=O)Me | Me | H | O |
| D-16155 | Me(R) | single bond | H | *c*-Pr[1-C(=O)OH] | C(=O)Me | Me | H | O |
| D-16156 | Me(S) | single bond | H | *c*-Pr[1-C(=O)OH] | C(=O)Me | Me | H | O |
| D-16157 | H | single bond | Me | *c*-Pr[1-C(=O)NH₂] | C(=O)Me | Cl | H | O |
| D-16158 | Me | single bond | H | *c*-Pr[1-C(=O)NH₂ | C(=O)Me | Cl | H | O |
| D-16159 | Me(R) | single bond | H | *c*-Pr[1-C(=O)NH₂ | C(=O)Me | Cl | H | O |
| D-16160 | Me(S) | single bond | H | *c*-Pr[1-C(=O)NH₂] | C(=O)Me | Cl | H | O |
| D-16161 | H | single bond | Me | *c*-Pr[1-C(=O)NH₂] | C(=O)Me | Me | H | O |
| D-16162 | Me | single bond | H | *c*-Pr[1-C(=O)NH₂ | C(=O)Me | Me | H | O |
| D-16163 | Me(R) | single bond | H | *c*-Pr[1-C(=O)NH₂ | C(=O)Me | Me | H | O |
| D-16164 | Me(S) | single bond | H | *c*-Pr[1-C(=O)NH₂ | C(=O)Me | Me | H | O |
| D-16165 | H | single bond | Me | *c*-Pr(1-OMe) | C(=O)Me | Cl | H | O |
| D-16166 | Me | single bond | H | *c*-Pr(1-OMe) | C(=O)Me | Cl | H | O |

**[Table 1014]**

| Compound Number | Y | W | Z | R¹ | R² | R³ | R¹⁰ | A |
|---|---|---|---|---|---|---|---|---|
| D-16167 | Me(R) | single bond | H | *c*-Pr(1-OMe) | C(=O)Me | Cl | H | O |
| D-16168 | Me(S) | single bond | H | *c*-Pr(1-OMe) | C(=O)Me | Cl | H | O |
| D-16169 | H | single bond | Me | *c-*Pr(1-OMe) | C(=O)Me | Me | H | O |
| D-16170 | Me | single bond | H | *c*-Pr(1-OMe) | C(=O)Me | Me | H | O |
| D-16171 | Me(R) | single bond | H | *c*-Pr(1-OMe) | C(=O)Me | Me | H | O |
| D-16172 | Me(S) | single bond | H | *c*-Pr(1-OMe) | C(=O)Me | Me | H | O |
| D-16173 | H | single bond | Me | *c*-Pr(1-SMe) | C(=O)Me | Cl | H | O |
| D-16174 | Me | single bond | H | *c*-Pr(1-SMe) | C(=O)Me | Cl | H | O |
| D-16175 | Me(R) | single bond | H | *c*-Pr(1-SMe) | C(=O)Me | Cl | H | O |
| D-16176 | Me(S) | single bond | H | *c*-Pr(1-SMe) | C(=O)Me | Cl | H | O |
| D-16177 | H | single bond | Me | *c*-Pr(1-SMe) | C(=O)Me | Me | H | O |
| D-16178 | Me | single bond | H | *c*-Pr(1-SMe) | C(=O)Me | Me | H | O |
| D-16179 | Me(R) | single bond | H | *c*-Pr(1-SMe) | C(=O)Me | Me | H | O |
| D-16180 | Me(S) | single bond | H | *c*-Pr(1-SMe) | C(=O)Me | Me | H | O |
| D-16181 | H | single bond | Me | *c*-Pr[1-S(=O)Me] | C(=O)Me | Cl | H | O |
| D-16182 | Me | single bond | H | *c*-Pr[1-S(=O)Me] | C(=O)Me | Cl | H | O |
| D-16183 | Me(R) | single bond | H | *c*-Pr[1-S(=O)Me] | C(=O)Me | Cl | H | O |
| D-16184 | Me(S) | single bond | H | *c*-Pr[1-S(=O)Me] | C(=O)Me | Cl | H | O |
| D-16185 | H | single bond | Me | *c*-Pr[1-S(=O)Me] | C(=O)Me | Me | H | O |
| D-16186 | Me | single bond | H | *c*-Pr[1-S(=O)Me] | C(=O)Me | Me | H | O |
| D-16187 | Me(R) | single bond | H | *c*-Pr[1-S(=O)Me] | C(=O)Me | Me | H | O |
| D-16188 | Me(S) | single bond | H | *c*-Pr[1-S(=O)Me] | C(=O)Me | Me | H | O |
| D-16189 | H | single bond | Me | *c*-Pr[1-S(=O)₂Me] | C(=O)Me | Cl | H | O |
| D-16190 | Me | single bond | H | *c*-Pr[1-S(=O)₂Me] | C(=O)Me | Cl | H | O |
| D-16191 | Me(R) | single bond | H | *c*-Pr[1-S(=O)₂Me] | C(=O)Me | Cl | H | O |
| D-16192 | Me(S) | single bond | H | *c*-Pr[1-S(=O)₂Me] | C(=O)Me | Cl | H | O |
| D-16193 | H | single bond | Me | *c*-Pr[1-S(=O)₂Me] | C(=O)Me | Me | H | O |
| D-16194 | Me | single bond | H | *c*-Pr[1-S(=O)₂Me] | C(=O)Me | Me | H | O |
| D-16195 | Me(R) | single bond | H | *c*-Pr[1-S(=O)₂Me] | C(=O)Me | Me | H | O |
| D-16196 | Me(S) | single bond | H | *c*-Pr[1-S(=O)₂Me] | C(=O)Me | Me | H | O |
| D-16197 | H | single bond | Me | *c*-Pr[1-S(=O)₂CHF₂] | C(=O)Me | Cl | H | O |
| D-16198 | Me | single bond | H | *c*-Pr[1-S(=O)₂CHF₂] | C(=O)Me | Cl | H | O |
| D-16199 | Me(R) | single bond | H | *c*-Pr[1-S(=O)₂CHF₂] | C(=O)Me | Cl | H | O |
| D-16200 | Me(S) | single bond | H | *c*-Pr[1-S(=O)₂CHF₂] | C(=O)Me | Cl | H | O |
| D-16201 | H | single bond | Me | *c*-Pr[1-S(=O)₂CHF₂] | C(=O)Me | Me | H | O |
| D-16202 | Me | single bond | H | *c*-Pr[1-S(=O)₂CHF₂] | C(=O)Me | Me | H | O |
| D-16203 | Me(R) | single bond | H | *c*-Pr[1-S(=O)₂CHF₂] | C(=O)Me | Me | H | O |
| D-16204 | Me(S) | single bond | H | *c*-Pr[1-S(=O)₂CHF₂] | C(=O)Me | Me | H | O |
| D-16205 | H | single bond | Me | *c*-Pr[1-S(=O)₂CF₃] | C(=O)Me | Cl | H | O |
| D-16206 | Me | single bond | H | *c*-Pr[1-S(=O)₂CF₃] | C(=O)Me | Cl | H | O |
| D-16207 | Me(R) | single bond | H | *c*-Pr[1-S(=O)₂CF₃] | C(=O)Me | Cl | H | O |
| D-16208 | Me(S) | single bond | H | *c*-Pr[1-S(=O)₂CF₃] | C(=O)Me | Cl | H | O |
| D-16209 | H | single bond | Me | *c*-Pr[1-S(=O)₂CF₃] | C(=O)Me | Me | H | O |
| D-16210 | Me | single bond | H | *c*-Pr[1-S(=O)₂CF₃] | C(=O)Me | Me | H | O |

**[Table 1015]**

| Compound Number | Y | W | Z | R¹ | R² | R³ | R¹⁰ | A |
|---|---|---|---|---|---|---|---|---|
| D-16211 | Me(R) | single bond | H | *c*-Pr[1-S(=O)₂CF₃] | C(=O)Me | Me | H | O |
| D-16212 | Me(S) | single bond | H | *c*-Pr[1-S(=O)₂CF₃] | C(=O)Me | Me | H | O |
| D-16213 | H | single bond | Me | *c*-Pr[1-(5,6-dihydro-1,4,2-dioxazin-3-yl)] | C(=O)Me | Cl | H | O |
| D-16214 | Me | single bond | H | *c*-Pr[1-(5,6-dihydro-1,4,2-dioxazin-3-yl)] | C(=O)Me | Cl | H | O |
| D-16215 | Me(R) | single bond | H | *c*-Pr[1-(5,6-dihydro-1,4,2-dioxazin-3-yl)] | C(=O)Me | Cl | H | O |
| D-16216 | Me(S) | single bond | H | *c*-Pr[1-(5,6-dihydro-1,4,2-dioxazin-3-yl)] | C(=O)Me | Cl | H | O |
| D-16217 | H | single bond | Me | *c*-Pr[1-(5,6-dihydro-1,4,2-dioxazin-3-yl)] | C(=O)Me | Me | H | O |
| D-16218 | Me | single bond | H | *c*-Pr[1-(5,6-dihydro-1,4,2-dioxazin-3-yl)] | C(=O)Me | Me | H | O |
| D-16219 | Me(R) | single bond | H | *c*-Pr[1-(5,6-dihydro-1,4,2-dioxazin-3-yl)] | C(=O)Me | Me | H | O |
| D-16220 | Me(S) | single bond | H | *c*-Pr[1-(5,6-dihydro-1,4,2-dioxazin-3-yl)] | C(=O)Me | Me | H | O |
| D-16221 | H | single bond | Me | *c*-Pr[1-(5-hydroxy-1,3,4-oxadiazol-2-yl)] | C(=O)Me | Cl | H | O |
| D-16222 | Me | single bond | H | *c*-Pr[1-(5-hydroxy-1,3,4-oxadiazol-2-yl)] | C(=O)Me | Cl | H | O |
| D-16223 | Me(R) | single bond | H | *c*-Pr[1-(5-hydroxy-1,3,4-oxadiazol-2-yl)] | C(=O)Me | Cl | H | O |
| D-16224 | Me(S) | single bond | H | *c*-Pr[1-(5-hydroxy-1,3,4-oxadiazol-2-yl)] | C(=O)Me | Cl | H | O |
| D-16225 | H | single bond | Me | *c*-Pr[1-(5-hydroxy-1,3,4-oxadiazol-2-yl)] | C(=O)Me | Me | H | O |
| D-16226 | Me | single bond | H | *c*-Pr[1-(5-hydroxy-1,3,4-oxadiazol-2-yl)] | C(=O)Me | Me | H | O |
| D-16227 | Me(R) | single bond | H | *c*-Pr[1-(5-hydroxy-1,3,4-oxadiazol-2-yl)] | C(=O)Me | Me | H | O |
| D-16228 | Me(S) | single bond | H | *c*-Pr[1-(5-hydroxy-1,3,4-oxadiazol-2-yl)] | C(=O)Me | Me | H | O |
| D-16229 | H | single bond | Me | *c*-Pr[1-(pyrimidin-2-yl)] | C(=O)Me | Cl | H | O |
| D-16230 | Me | single bond | H | *c*-Pr[1-(pyrimidin-2-yl)] | C(=O)Me | Cl | H | O |
| D-16231 | Me(R) | single bond | H | *c*-Pr[1-(pyrimidin-2-yl)] | C(=O)Me | Cl | H | O |
| D-16232 | Me(S) | single bond | H | *c*-Pr[1-(pyrimidin-2-yl)] | C(=O)Me | Cl | H | O |
| D-16233 | H | single bond | Me | *c*-Pr[1-(pyrimidin-2-yl)] | C(=O)Me | Me | H | O |
| D-16234 | Me | single bond | H | *c*-Pr[1-(pyrimidin-2-yl)] | C(=O)Me | Me | H | O |
| D-16235 | Me(R) | single bond | H | *c*-Pr[1-(pyrimidin-2-yl)] | C(=O)Me | Me | H | O |
| D-16236 | Me(S) | single bond | H | *c*-Pr[1-(pyrimidin-2-yl)] | C(=O)Me | Me | H | O |
| D-16237 | H | single bond | Me | *t*-Bu | C(=O)Me | Cl | H | O |
| D-16238 | Me | single bond | H | *t*-Bu | C(=O)Me | Cl | H | O |
| D-16239 | Me(R) | single bond | H | *t*-Bu | C(=O)Me | Cl | H | O |
| D-16240 | Me(S) | single bond | H | *t*-Bu | C(=O)Me | Cl | H | O |
| D-16241 | H | single bond | Me | *t*-Bu | C(=O)Me | Me | H | O |
| D-16242 | Me | single bond | H | *t*-Bu | C(=O)Me | Me | H | O |
| D-16243 | Me(R) | single bond | H | *t*-Bu | C(=O)Me | Me | H | O |
| D-16244 | Me(S) | single bond | H | *t*-Bu | C(=O)Me | Me | H | O |
| D-16245 | H | single bond | Me | *c*-Bu | C(=O)Me | Cl | H | O |
| D-16246 | Me | single bond | H | *c*-Bu | C(=O)Me | Cl | H | O |
| D-16247 | Me(R) | single bond | H | *c*-Bu | C(=O)Me | Cl | H | O |
| D-16248 | Me(S) | single bond | H | c-Bu | C(=O)Me | Cl | H | O |
| D-16249 | H | single bond | Me | *c*-Bu | C(=O)Me | Me | H | O |
| D-16250 | Me | single bond | H | *c*-Bu | C(=O)Me | Me | H | O |
| D-16251 | Me(R) | single bond | H | *c*-Bu | C(=O)Me | Me | H | O |
| D-16252 | Me(S) | single bond | H | *c*-Bu | C(=O)Me | Me | H | O |
| D-16253 | H | single bond | Me | *c*-Bu(1-CN) | C(=O)Me | Cl | H | O |
| D-16254 | Me | single bond | H | *c*-Bu(1-CN) | C(=O)Me | Cl | H | O |

**[Table 1016]**

| Compound Number | Y | W | Z | R¹ | R² | R³ | R¹⁰ | A |
|---|---|---|---|---|---|---|---|---|
| D-16255 | Me(R) | single bond | H | *c*-Bu(1-CN) | C(=O)Me | Cl | H | O |
| D-16256 | Me(S) | single bond | H | *c*-Bu(1-CN) | C(=O)Me | Cl | H | O |
| D-16257 | H | single bond | Me | *c-*Bu(1-CN) | C(=O)Me | Me | H | O |
| D-16258 | Me | single bond | H | *c*-Bu(1-CN) | C(=O)Me | Me | H | O |
| D-16259 | Me(R) | single bond | H | *c*-Bu(1-CN) | C(=O)Me | Me | H | O |
| D-16260 | Me(S) | single bond | H | *c*-Bu(1-CN) | C(=O)Me | Me | H | O |
| D-16261 | H | single bond | Me | *c*-Bu(3,3-F₂) | C(=O)Me | Cl | H | O |
| D-16262 | Me | single bond | H | *c*-Bu(3,3-F₂) | C(=O)Me | Cl | H | O |
| D-16263 | Me(R) | single bond | H | *c*-Bu(3,3-F₂) | C(=O)Me | Cl | H | O |
| D-16264 | Me(S) | single bond | H | *c*-Bu(3,3-F₂) | C(=O)Me | Cl | H | O |
| D-16265 | H | single bond | Me | *c*-Bu(3,3-F₂) | C(=O)Me | Me | H | O |
| D-16266 | Me | single bond | H | *c*-Bu(3,3-F₂) | C(=O)Me | Me | H | O |
| D-16267 | Me(R) | single bond | H | *c*-Bu(3,3-F₂) | C(=O)Me | Me | H | O |
| D-16268 | Me(S) | single bond | H | *c*-Bu(3,3-F₂) | C(=O)Me | Me | H | O |
| D-16269 | H | single bond | Me | oxetan-3-yl | C(=O)Me | Cl | H | O |
| D-16270 | Me | single bond | H | oxetan-3-yl | C(=O)Me | Cl | H | O |
| D-16271 | Me(R) | single bond | H | oxetan-3-yl | C(=O)Me | Cl | H | O |
| D-16272 | Me(S) | single bond | H | oxetan-3-yl | C(=O)Me | Cl | H | O |
| D-16273 | H | single bond | Me | oxetan-3-yl | C(=O)Me | Me | H | O |
| D-16274 | Me | single bond | H | oxetan-3-yl | C(=O)Me | Me | H | O |
| D-16275 | Me(R) | single bond | H | oxetan-3-yl | C(=O)Me | Me | H | O |
| D-16276 | Me(S) | single bond | H | oxetan-3-yl | C(=O)Me | Me | H | O |
| D-16277 | H | single bond | Me | oxetan(3-CN)-3-yl | C(=O)Me | Cl | H | O |
| D-16278 | Me | single bond | H | oxetan(3-CN)-3-yl | C(=O)Me | Cl | H | O |
| D-16279 | Me(R) | single bond | H | oxetan(3-CN)-3-yl | C(=O)Me | Cl | H | O |
| D-16280 | Me(S) | single bond | H | oxetan(3-CN)-3-yl | C(=O)Me | Cl | H | O |
| D-16281 | H | single bond | Me | oxetan(3-CN)-3-yl | C(=O)Me | Me | H | O |
| D-16282 | Me | single bond | H | oxetan(3-CN)-3-yl | C(=O)Me | Me | H | O |
| D-16283 | Me(R) | single bond | H | oxetan(3-CN)-3-yl | C(=O)Me | Me | H | O |
| D-16284 | Me(S) | single bond | H | oxetan(3-CN)-3-yl | C(=O)Me | Me | H | O |
| D-16285 | H | single bond | Me | azetidin[1-C(=O)Me]-3-yl | C(=O)Me | Cl | H | O |
| D-16286 | Me | single bond | H | azetidin[1-C(=O)Me]-3-yl | C(=O)Me | Cl | H | O |
| D-16287 | Me(R) | single bond | H | azetidin[1-C(=O)Me]-3-yl | C(=O)Me | Cl | H | O |
| D-16288 | Me(S) | single bond | H | azetidin[1-C(=O)Me]-3-yl | C(=O)Me | Cl | H | O |
| D-16289 | H | single bond | Me | azetidin[1-C(=O)Me]-3-yl | C(=O)Me | Me | H | O |
| D-16290 | Me | single bond | H | azetidin[1-C(=O)Me]-3-yl | C(=O)Me | Me | H | O |
| D-16291 | Me(R) | single bond | H | azetidin[1-C(=O)Me]-3-yl | C(=O)Me | Me | H | O |
| D-16292 | Me(S) | single bond | H | azetidin[1-C(=O)Me]-3-yl | C(=O)Me | Me | H | O |
| D-16293 | H | single bond | Me | azetidin[1-S(=O)₂Me]-3-yl | C(=O)Me | Cl | H | O |
| D-16294 | Me | single bond | H | azetidin[1-S(=O)₂Me]-3-yl | C(=O)Me | Cl | H | O |
| D-16295 | Me(R) | single bond | H | azetidin[1-S(=O)₂Me]-3-yl | C(=O)Me | Cl | H | O |
| D-16296 | Me(S) | single bond | H | azetidin[1-S(=O)₂Me]-3-yl | C(=O)Me | Cl | H | O |
| D-16297 | H | single bond | Me | azetidin[1-S(=O)₂Me]-3-yl | C(=O)Me | Me | H | O |
| D-16298 | Me | single bond | H | azetidin[1-S(=O)₂Me]-3-yl | C(=O)Me | Me | H | O |

**[Table 1017]**

| Compound Number | Y | W | Z | R¹ | R² | R³ | R¹⁰ | A |
|---|---|---|---|---|---|---|---|---|
| D-16299 | Me(R) | single bond | H | azetidin[1-S(=O)₂Me]-3-yl | C(=O)Me | Me | H | O |
| D-16300 | Me(S) | single bond | H | azetidin[1-S(=O)₂Me]-3-yl | C(=O)Me | Me | H | O |
| D-16301 | H | single bond | Me | azetidin[1-S(=O)₂CHF₂]-3-yl | C(=O)Me | Cl | H | O |
| D-16302 | Me | single bond | H | azetidin[1-S(=O)₂CHF₂]-3-yl | C(=O)Me | Cl | H | O |
| D-16303 | Me(R) | single bond | H | azetidin[1-S(=O)₂CHF₂]-3-yl | C(=O)Me | Cl | H | O |
| D-16304 | Me(S) | single bond | H | azetidin[1-S(=O)₂CHF₂]-3-yl | C(=O)Me | Cl | H | O |
| D-16305 | H | single bond | Me | azetidin[1-S(=O)₂CHF₂]-3-yl | C(=O)Me | Me | H | O |
| D-16306 | Me | single bond | H | azetidin[1-S(=O)₂CHF₂]-3-yl | C(=O)Me | Me | H | O |
| D-16307 | Me(R) | single bond | H | azetidin[1-S(=O)₂CHF₂]-3-yl | C(=O)Me | Me | H | O |
| D-16308 | Me(S) | single bond | H | azetidin[1-S(=O)₂CHF₂]-3-yl | C(=O)Me | Me | H | O |
| D-16309 | H | single bond | Me | *c*-Pen | C(=O)Me | Cl | H | O |
| D-16310 | Me | single bond | H | *c*-Pen | C(=O)Me | Cl | H | O |
| D-16311 | Me(R) | single bond | H | *c*-Pen | C(=O)Me | Cl | H | O |
| D-16312 | Me(S) | single bond | H | *c*-Pen | C(=O)Me | Cl | H | O |
| D-16313 | H | single bond | Me | *c*-Pen | C(=O)Me | Me | H | O |
| D-16314 | Me | single bond | H | *c*-Pen | C(=O)Me | Me | H | O |
| D-16315 | Me(R) | single bond | H | *c*-Pen | C(=O)Me | Me | H | O |
| D-16316 | Me(S) | single bond | H | *c*-Pen | C(=O)Me | Me | H | O |
| D-16317 | H | single bond | Me | tetrahydrofuran-3-yl | C(=O)Me | Cl | H | O |
| D-16318 | Me | single bond | H | tetrahydrofuran-3-yl | C(=O)Me | Cl | H | O |
| D-16319 | Me(R) | single bond | H | tetrahydrofuran-3-yl | C(=O)Me | Cl | H | O |
| D-16320 | Me(S) | single bond | H | tetrahydrofuran-3-yl | C(=O)Me | Cl | H | O |
| D-16321 | H | single bond | Me | tetrahydrofuran-3-yl | C(=O)Me | Me | H | O |
| D-16322 | Me | single bond | H | tetrahydrofuran-3-yl | C(=O)Me | Me | H | O |
| D-16323 | Me(R) | single bond | H | tetrahydrofuran-3-yl | C(=O)Me | Me | H | O |
| D-16324 | Me(S) | single bond | H | tetrahydrofuran-3-yl | C(=O)Me | Me | H | O |
| D-16325 | H | single bond | Me | tetrahydrothiophen-3-yl | C(=O)Me | Cl | H | O |
| D-16326 | Me | single bond | H | tetrahydrothiophen-3-yl | C(=O)Me | Cl | H | O |
| D-16327 | Me(R) | single bond | H | tetrahydrothiophen-3-yl | C(=O)Me | Cl | H | O |
| D-16328 | Me(S) | single bond | H | tetrahydrothiophen-3-yl | C(=O)Me | Cl | H | O |
| D-16329 | H | single bond | Me | tetrahydrothiophen-3-yl | C(=O)Me | Me | H | O |
| D-16330 | Me | single bond | H | tetrahydrothiophen-3-yl | C(=O)Me | Me | H | O |
| D-16331 | Me(R) | single bond | H | tetrahydrothiophen-3-yl | C(=O)Me | Me | H | O |
| D-16332 | Me(S) | single bond | H | tetrahydrothiophen-3-yl | C(=O)Me | Me | H | O |
| D-16333 | H | single bond | Me | *c*-Pen(1-CN) | C(=O)Me | Cl | H | O |
| D-16334 | Me | single bond | H | *c*-Pen(1-CN) | C(=O)Me | Cl | H | O |
| D-16335 | Me(R) | single bond | H | *c*-Pen(1-CN) | C(=O)Me | Cl | H | O |
| D-16336 | Me(S) | single bond | H | *c*-Pen(1-CN) | C(=O)Me | Cl | H | O |
| D-16337 | H | single bond | Me | *c*-Pen(1-CN) | C(=O)Me | Me | H | O |
| D-16338 | Me | single bond | H | *c*-Pen(1-CN) | C(=O)Me | Me | H | O |
| D-16339 | Me(R) | single bond | H | *c*-Pen(1-CN) | C(=O)Me | Me | H | O |
| D-16340 | Me(S) | single bond | H | *c*-Pen(1-CN) | C(=O)Me | Me | H | O |
| D-16341 | H | single bond | Me | tetrahydrofuran(3-CN)-3-yl | C(=O)Me | Cl | H | O |
| D-16342 | Me | single bond | H | tetrahydrofuran(3-CN)-3-yl | C(=O)Me | Cl | H | O |

**[Table 1018]**

| Compound Number | Y | W | Z | R¹ | R² | R³ | R¹⁰ | A |
|---|---|---|---|---|---|---|---|---|
| D-16343 | Me(R) | single bond | H | tetrahydrofuran(3-CN)-3-yl | C(=O)Me | Cl | H | O |
| D-16344 | Me(S) | single bond | H | tetrahydrofuran(3-CN)-3-yl | C(=O)Me | Cl | H | O |
| D-16345 | H | single bond | Me | tetrahydrofuran(3-CN)-3-yl | C(=O)Me | Me | H | O |
| D-16346 | Me | single bond | H | tetrahydrofuran(3-CN)-3-yl | C(=O)Me | Me | H | O |
| D-16347 | Me(R) | single bond | H | tetrahydrofuran(3-CN)-3-yl | C(=O)Me | Me | H | O |
| D-16348 | Me(S) | single bond | H | tetrahydrofuran(3-CN)-3-yl | C(=O)Me | Me | H | O |
| D-16349 | H | single bond | Me | tetrahydrothiophen(3-CN)-3-yl | C(=O)Me | Cl | H | O |
| D-16350 | Me | single bond | H | tetrahydrothiophen(3-CN)-3-yl | C(=O)Me | Cl | H | O |
| D-16351 | Me(R) | single bond | H | tetrahydrothiophen(3-CN)-3-yl | C(=O)Me | Cl | H | O |
| D-16352 | Me(S) | single bond | H | tetrahydrothiophen(3-CN)-3-yl | C(=O)Me | Cl | H | O |
| D-16353 | H | single bond | Me | tetrahydrothiophen(3-CN)-3-yl | C(=O)Me | Me | H | O |
| D-16354 | Me | single bond | H | tetrahydrothiophen(3-CN)-3-yl | C(=O)Me | Me | H | O |
| D-16355 | Me(R) | single bond | H | tetrahydrothiophen(3-CN)-3-yl | C(=O)Me | Me | H | O |
| D-16356 | Me(S) | single bond | H | tetrahydrothiophen(3-CN)-3-yl | C(=O)Me | Me | H | O |
| D-16357 | H | single bond | Me | bicyclo[1.1.1]pertan-1-yl | C(=O)Me | Cl | H | O |
| D-16358 | Me | single bond | H | bicyclo[1.1.1]pertan-1-yl | C(=O)Me | Cl | H | O |
| D-16359 | Me(R) | single bond | H | bicyclo[1.1.1]pertan-1-yl | C(=O)Me | Cl | H | O |
| D-16360 | Me(S) | single bond | H | bicyclo[1.1.1]pertan-1-yl | C(=O)Me | Cl | H | O |
| D-16361 | H | single bond | Me | bicyclo[1.1.1]pertan-1-yl | C(=O)Me | Me | H | O |
| D-16362 | Me | single bond | H | bicyclo[1.1.1]pertan-1-yl | C(=O)Me | Me | H | O |
| D-16363 | Me(R) | single bond | H | bicyclo[1.1.1]pertan-1-yl | C(=O)Me | Me | H | O |
| D-16364 | Me(S) | single bond | H | bicyclo[1.1.1]pertan-1-yl | C(=O)Me | Me | H | O |
| D-16365 | H | single bond | Me | *c*-Hex | C(=O)Me | Cl | H | O |
| D-16366 | Me | single bond | H | *c*-Hex | C(=O)Me | Cl | H | O |
| D-16367 | Me(R) | single bond | H | *c*-Hex | C(=O)Me | Cl | H | O |
| D-16368 | Me(S) | single bond | H | *c*-Hex | C(=O)Me | Cl | H | O |
| D-16369 | H | single bond | Me | *c*-Hex | C(=O)Me | Me | H | O |
| D-16370 | Me | single bond | H | *c*-Hex | C(=O)Me | Me | H | O |
| D-16371 | Me(R) | single bond | H | *c*-Hex | C(=O)Me | Me | H | O |
| D-16372 | Me(S) | single bond | H | *c*-Hex | C(=O)Me | Me | H | O |
| D-16373 | H | single bond | Me | tetrahydropyran-4-yl | C(=O)Me | Cl | H | O |
| D-16374 | Me | single bond | H | tetrahydropyran-4-yl | C(=O)Me | Cl | H | O |
| D-16375 | Me(R) | single bond | H | tetrahydropyran-4-yl | C(=O)Me | Cl | H | O |
| D-16376 | Me(S) | single bond | H | tetrahydropyran-4-yl | C(=O)Me | Cl | H | O |
| D-16377 | H | single bond | Me | tetrahydropyran-4-yl | C(=O)Me | Me | H | O |
| D-16378 | Me | single bond | H | tetrahydropyran-4-yl | C(=O)Me | Me | H | O |
| D-16379 | Me(R) | single bond | H | tetrahydropyran-4-yl | C(=O)Me | Me | H | O |
| D-16380 | Me(S) | single bond | H | tetrahydropyran-4-yl | C(=O)Me | Me | H | O |
| D-16381 | H | single bond | Me | tetrahydrothiopyran-4-yl | C(=O)Me | Cl | H | O |
| D-16382 | Me | single bond | H | tetrahydrothiopyran-4-yl | C(=O)Me | Cl | H | O |
| D-16383 | Me(R) | single bond | H | tetrahydrothiopyran-4-yl | C(=O)Me | Cl | H | O |
| D-16384 | Me(S) | single bond | H | tetrahydrothiopyran-4-yl | C(=O)Me | Cl | H | O |
| D-16385 | H | single bond | Me | tetrahydrothiopyran-4-yl | C(=O)Me | Me | H | O |
| D-16386 | Me | single bond | H | tetrahydrothiopyran-4-yl | C(=O)Me | Me | H | O |

**[Table 1019]**

| Compound Number | Y | W | Z | R¹ | R² | R³ | R¹⁰ | A |
|---|---|---|---|---|---|---|---|---|
| D-16387 | Me(R) | single bond | H | tetrahydrothiopyran-4-yl | C(=O)Me | Me | H | O |
| D-16388 | Me(S) | single bond | H | tetrahydrothiopyran-4-yl | C(=O)Me | Me | H | O |
| D-16389 | H | single bond | Me | *c*-Hex(1-CN) | C(=O)Me | Cl | H | O |
| D-16390 | Me | single bond | H | *c*-Hex(1-CN) | C(=O)Me | Cl | H | O |
| D-16391 | Me(R) | single bond | H | *c*-Hex(1-CN) | C(=O)Me | Cl | H | O |
| D-16392 | Me(S) | single bond | H | *c*-Hex(1-CN) | C(=O)Me | Cl | H | O |
| D-16393 | H | single bond | Me | *c*-Hex(1-CN) | C(=O)Me | Me | H | O |
| D-16394 | Me | single bond | H | *c*-Hex(1-CN) | C(=O)Me | Me | H | O |
| D-16395 | Me(R) | single bond | H | *c*-Hex(1-CN) | C(=O)Me | Me | H | O |
| D-16396 | Me(S) | single bond | H | *c*-Hex(1-CN) | C(=O)Me | Me | H | O |
| D-16397 | H | single bond | Me | tetrahydropyran(4-CN)-4-yl | C(=O)Me | Cl | H | O |
| D-16398 | Me | single bond | H | tetrahydropyran(4-CN)-4-yl | C(=O)Me | Cl | H | O |
| D-16399 | Me(R) | single bond | H | tetrahydropyran(4-CN)-4-yl | C(=O)Me | Cl | H | O |
| D-16400 | Me(S) | single bond | H | tetrahydropyran(4-CN)-4-yl | C(=O)Me | Cl | H | O |
| D-16401 | H | single bond | Me | tetrahydropyran(4-CN)-4-yl | C(=O)Me | Me | H | O |
| D-16402 | Me | single bond | H | tetrahydropyran(4-CN)-4-yl | C(=O)Me | Me | H | O |
| D-16403 | Me(R) | single bond | H | tetrahydropyran(4-CN)-4-yl | C(=O)Me | Me | H | O |
| D-16404 | Me(S) | single bond | H | tetrahydropyran(4-CN)-4-yl | C(=O)Me | Me | H | O |
| D-16405 | H | single bond | Me | tetrahydrothiopyran(4-CN)-4-yl | C(=O)Me | Cl | H | O |
| D-16406 | Me | single bond | H | tetrahydrothiopyran(4-CN)-4-yl | C(=O)Me | Cl | H | O |
| D-16407 | Me(R) | single bond | H | tetrahydrothiopyran(4-CN)-4-yl | C(=O)Me | Cl | H | O |
| D-16408 | Me(S) | single bond | H | tetrahydrothiopyran(4-CN)-4-yl | C(=O)Me | Cl | H | O |
| D-16409 | H | single bond | Me | tetrahydrothiopyran(4-CN)-4-yl | C(=O)Me | Me | H | O |
| D-16410 | Me | single bond | H | tetrahydrothiopyran(4-CN)-4-yl | C(=O)Me | Me | H | O |
| D-16411 | Me(R) | single bond | H | tetrahydrothiopyran(4-CN)-4-yl | C(=O)Me | Me | H | O |
| D-16412 | Me(S) | single bond | H | tetrahydrothiopyran(4-CN)-4-yl | C(=O)Me | Me | H | O |
| D-16413 | H | single bond | Me | CH₂(*c*-Pr) | C(=O)Me | Cl | H | O |
| D-16414 | Me | single bond | H | CH₂(*c*-Pr) | C(=O)Me | Cl | H | O |
| D-16415 | Me(R) | single bond | H | CH₂(*c*-Pr) | C(=O)Me | Cl | H | O |
| D-16416 | Me(S) | single bond | H | CH₂(*c*-Pr) | C(=O)Me | Cl | H | O |
| D-16417 | H | single bond | Me | CH₂(*c*-Pr) | C(=O)Me | Me | H | O |
| D-16418 | Me | single bond | H | CH₂(*c*-Pr) | C(=O)Me | Me | H | O |
| D-16419 | Me(R) | single bond | H | CH₂(*c*-Pr) | C(=O)Me | Me | H | O |
| D-16420 | Me(S) | single bond | H | CH₂(*c*-Pr) | C(=O)Me | Me | H | O |
| D-16421 | H | single bond | Me | CH₂[*c*-Pr(1-CN)] | C(=O)Me | Cl | H | O |
| D-16422 | Me | single bond | H | CH₂[*c*-Pr(1-CN)] | C(=O)Me | Cl | H | O |
| D-16423 | Me(R) | single bond | H | CH₂[*c*-Pr(1-CN)] | C(=O)Me | Cl | H | O |
| D-16424 | Me(S) | single bond | H | CH₂[*c*-Pr(1-CN)] | C(=O)Me | Cl | H | O |
| D-16425 | H | single bond | Me | CH₂[*c*-Pr(1-CN)] | C(=O)Me | Me | H | O |
| D-16426 | Me | single bond | H | CH₂[*c*-Pr(1-CN)] | C(=O)Me | Me | H | O |
| D-16427 | Me(R) | single bond | H | CH₂[*c*-Pr(1-CN)] | C(=O)Me | Me | H | O |
| D-16428 | Me(S) | single bond | H | CH₂[*c*-Pr(1-CN)] | C(=O)Me | Me | H | O |
| D-16429 | H | single bond | Me | CH(Me)(*c*-Pr) | C(=O)Me | Cl | H | O |
| D-16430 | Me | single bond | H | CH(Me)(*c*-Pr) | C(=O)Me | Cl | H | O |

**[Table 1020]**

| Compound Number | Y | W | Z | R¹ | R² | R³ | R¹⁰ | A |
|---|---|---|---|---|---|---|---|---|
| D-16431 | Me(R) | single bond | H | CH(Me)(*c*-Pr) | C(=O)Me | Cl | H | O |
| D-16432 | Me(S) | single bond | H | CH(Me)(*c*-Pr) | C(=O)Me | Cl | H | O |
| D-16433 | H | single bond | Me | CH(Me)(*c*-Pr) | C(=O)Me | Me | H | O |
| D-16434 | Me | single bond | H | CH(Me)(*c*-Pr) | C(=O)Me | Me | H | O |
| D-16435 | Me(R) | single bond | H | CH(Me)(*c*-Pr) | C(=O)Me | Me | H | O |
| D-16436 | Me(S) | single bond | H | CH(Me)(*c*-Pr) | C(=O)Me | Me | H | O |
| D-16437 | H | single bond | Me | CH(Me)CH₂OMe | C(=O)Me | Cl | H | O |
| D-16438 | Me | single bond | H | CH(Me)CH₂OMe | C(=O)Me | Cl | H | O |
| D-16439 | Me(R) | single bond | H | CH(Me)CH₂OMe | C(=O)Me | Cl | H | O |
| D-16440 | Me(S) | single bond | H | CH(Me)CH₂OMe | C(=O)Me | Cl | H | O |
| D-16441 | H | single bond | Me | CH(Me)CH₂OMe | C(=O)Me | Me | H | O |
| D-16442 | Me | single bond | H | CH(Me)CH₂OMe | C(=O)Me | Me | H | O |
| D-16443 | Me(R) | single bond | H | CH(Me)CH₂OMe | C(=O)Me | Me | H | O |
| D-16444 | Me(S) | single bond | H | CH(Me)CH₂OMe | C(=O)Me | Me | H | O |
| D-16445 | H | single bond | Me | CH₂CH=CH₂ | C(=O)Me | Cl | H | O |
| D-16446 | Me | single bond | H | CH₂CH=CH₂ | C(=O)Me | Cl | H | O |
| D-16447 | Me(R) | single bond | H | CH₂CH=CH₂ | C(=O)Me | Cl | H | O |
| D-16448 | Me(S) | single bond | H | CH₂CH=CH₂ | C(=O)Me | Cl | H | O |
| D-16449 | H | single bond | Me | CH₂CH=CH₂ | C(=O)Me | Me | H | O |
| D-16450 | Me | single bond | H | CH₂CH=CH₂ | C(=O)Me | Me | H | O |
| D-16451 | Me(R) | single bond | H | CH₂CH=CH₂ | C(=O)Me | Me | H | O |
| D-16452 | Me(S) | single bond | H | CH₂CH=CH₂ | C(=O)Me | Me | H | O |
| D-16453 | H | single bond | Me | CH₂C≡CH | C(=O)Me | Cl | H | O |
| D-16454 | Me | single bond | H | CH₂C≡CH | C(=O)Me | Cl | H | O |
| D-16455 | Me(R) | single bond | H | CH₂C≡CH | C(=O)Me | Cl | H | O |
| D-16456 | Me(S) | single bond | H | CH₂C≡CH | C(=O)Me | Cl | H | O |
| D-16457 | H | single bond | Me | CH₂C≡CH | C(=O)Me | Me | H | O |
| D-16458 | Me | single bond | H | CH₂C≡CH | C(=O)Me | Me | H | O |
| D-16459 | Me(R) | single bond | H | CH₂C≡CH | C(=O)Me | Me | H | O |
| D-16460 | Me(S) | single bond | H | CH₂C≡CH | C(=O)Me | Me | H | O |
| D-16461 | H | single bond | Me | CH₂CN | C(=O)Me | Cl | H | O |
| D-16462 | Me | single bond | H | CH₂CN | C(=O)Me | Cl | H | O |
| D-16463 | Me(R) | single bond | H | CH₂CN | C(=O)Me | Cl | H | O |
| D-16464 | Me(S) | single bond | H | CH₂CN | C(=O)Me | Cl | H | O |
| D-16465 | H | single bond | Me | CH₂CN | C(=O)Me | Me | H | O |
| D-16466 | Me | single bond | H | CH₂CN | C(=O)Me | Me | H | O |
| D-16467 | Me(R) | single bond | H | CH₂CN | C(=O)Me | Me | H | O |
| D-16468 | Me(S) | single bond | H | CH₂CN | C(=O)Me | Me | H | O |
| D-16469 | H | single bond | Me | CH(Me)CN | C(=O)Me | Cl | H | O |
| D-16470 | Me | single bond | H | CH(Me)CN | C(=O)Me | Cl | H | O |
| D-16471 | Me(R) | single bond | H | CH(Me)CN | C(=O)Me | Cl | H | O |
| D-16472 | Me(S) | single bond | H | CH(Me)CN | C(=O)Me | Cl | H | O |
| D-16473 | H | single bond | Me | CH(Me)CN | C(=O)Me | Me | H | O |
| D-16474 | Me | single bond | H | CH(Me)CN | C(=O)Me | Me | H | O |

**[Table 1021]**

| Compound Number | Y | W | Z | R¹ | R² | R³ | R¹⁰ | A |
|---|---|---|---|---|---|---|---|---|
| D-16475 | Me(R) | single bond | H | CH(Me)CN | C(=O)Me | Me | H | O |
| D-16476 | Me(S) | single bond | H | CH(Me)CN | C(=O)Me | Me | H | O |
| D-16477 | H | single bond | Me | C(Me)₂CN | C(=O)Me | Cl | H | O |
| D-16478 | Me | single bond | H | C(Me)₂CN | C(=O)Me | Cl | H | O |
| D-16479 | Me(R) | single bond | H | C(Me)₂CN | C(=O)Me | Cl | H | O |
| D-16480 | Me(S) | single bond | H | C(Me)₂CN | C(=O)Me | Cl | H | O |
| D-16481 | H | single bond | Me | C(Me)₂CN | C(=O)Me | Me | H | O |
| D-16482 | Me | single bond | H | C(Me)₂CN | C(=O)Me | Me | H | O |
| D-16483 | Me(R) | single bond | H | C(Me)₂CN | C(=O)Me | Me | H | O |
| D-16484 | Me(S) | single bond | H | C(Me)₂CN | C(=O)Me | Me | H | O |
| D-16485 | H | single bond | Me | C(Me)(*i*-Pr)CN | C(=O)Me | Cl | H | O |
| D-16486 | Me | single bond | H | C(Me)(*i*-Pr)CN | C(=O)Me | Cl | H | O |
| D-16487 | Me(R) | single bond | H | C(Me)(*i*-Pr)CN | C(=O)Me | Cl | H | O |
| D-16488 | Me(S) | single bond | H | C(Me)(*i*-Pr)CN | C(=O)Me | Cl | H | O |
| D-16489 | H | single bond | Me | C(Me)(*i*-Pr)CN | C(=O)Me | Me | H | O |
| D-16490 | Me | single bond | H | C(Me)(*i*-Pr)CN | C(=O)Me | Me | H | O |
| D-16491 | Me(R) | single bond | H | C(Me)(*i*-Pr)CN | C(=O)Me | Me | H | O |
| D-16492 | Me(S) | single bond | H | C(Me)(*i*-Pr)CN | C(=O)Me | Me | H | O |
| D-16493 | H | single bond | Me | OMe | C(=O)Me | Cl | H | O |
| D-16494 | Me | single bond | H | OMe | C(=O)Me | Cl | H | O |
| D-16495 | Me(R) | single bond | H | OMe | C(=O)Me | Cl | H | O |
| D-16496 | Me(S) | single bond | H | OMe | C(=O)Me | Cl | H | O |
| D-16497 | H | single bond | Me | OMe | C(=O)Me | Me | H | O |
| D-16498 | Me | single bond | H | OMe | C(=O)Me | Me | H | O |
| D-16499 | Me(R) | single bond | H | OMe | C(=O)Me | Me | H | O |
| D-16500 | Me(S) | single bond | H | OMe | C(=O)Me | Me | H | O |
| D-16501 | H | single bond | Me | CH=NOMe | C(=O)Me | Cl | H | O |
| D-16502 | Me | single bond | H | CH=NOMe | C(=O)Me | Cl | H | O |
| D-16503 | Me(R) | single bond | H | CH=NOMe | C(=O)Me | Cl | H | O |
| D-16504 | Me(S) | single bond | H | CH=NOMe | C(=O)Me | Cl | H | O |
| D-16505 | H | single bond | Me | CH=NOMe | C(=O)Me | Me | H | O |
| D-16506 | Me | single bond | H | CH=NOMe | C(=O)Me | Me | H | O |
| D-16507 | Me(R) | single bond | H | CH=NOMe | C(=O)Me | Me | H | O |
| D-16508 | Me(S) | single bond | H | CH=NOMe | C(=O)Me | Me | H | O |
| D-16509 | H | single bond | Me | morpholin-4-yl | C(=O)Me | Cl | H | O |
| D-16510 | Me | single bond | H | morpholin-4-yl | C(=O)Me | Cl | H | O |
| D-16511 | Me(R) | single bond | H | morpholin-4-yl | C(=O)Me | Cl | H | O |
| D-16512 | Me(S) | single bond | H | morpholin-4-yl | C(=O)Me | Cl | H | O |
| D-16513 | H | single bond | Me | morpholin-4-yl | C(=O)Me | Me | H | O |
| D-16514 | Me | single bond | H | morpholin-4-yl | C(=O)Me | Me | H | O |
| D-16515 | Me(R) | single bond | H | morpholin-4-yl | C(=O)Me | Me | H | O |
| D-16516 | Me(S) | single bond | H | morpholin-4-yl | C(=O)Me | Me | H | O |
| D-16517 | H | single bond | Me | Ph | C(=O)Me | Cl | H | O |
| D-16518 | Me | single bond | H | Ph | C(=O)Me | Cl | H | O |

**[Table 1022]**

| Compound Number | Y | W | Z | R¹ | R² | R³ | R¹⁰ | A |
|---|---|---|---|---|---|---|---|---|
| D-16519 | Me(R) | single bond | H | Ph | C(=O)Me | Cl | H | O |
| D-16520 | Me(S) | single bond | H | Ph | C(=O)Me | Cl | H | O |
| D-16521 | H | single bond | Me | Ph | C(=O)Me | Me | H | O |
| D-16522 | Me | single bond | H | Ph | C(=O)Me | Me | H | O |
| D-16523 | Me(R) | single bond | H | Ph | C(=O)Me | Me | H | O |
| D-16524 | Me(S) | single bond | H | Ph | C(=O)Me | Me | H | O |
| D-16525 | H | single bond | Me | CH₂Ph | C(=O)Me | Cl | H | O |
| D-16526 | Me | single bond | H | CH₂Ph | C(=O)Me | Cl | H | O |
| D-16527 | Me(R) | single bond | H | CH₂Ph | C(=O)Me | Cl | H | O |
| D-16528 | Me(S) | single bond | H | CH₂Ph | C(=O)Me | Cl | H | O |
| D-16529 | H | single bond | Me | CH₂Ph | C(=O)Me | Me | H | O |
| D-16530 | Me | single bond | H | CH₂Ph | C(=O)Me | Me | H | O |
| D-16531 | Me(R) | single bond | H | CH₂Ph | C(=O)Me | Me | H | O |
| D-16532 | Me(S) | single bond | H | CH₂Ph | C(=O)Me | Me | H | O |
| D-16533 | H | single bond | Me | thiazole-2-yl | C(=O)Me | Cl | H | O |
| D-16534 | Me | single bond | H | thiazole-2-yl | C(=O)Me | Cl | H | O |
| D-16535 | Me(R) | single bond | H | thiazole-2-yl | C(=O)Me | Cl | H | O |
| D-16536 | Me(S) | single bond | H | thiazole-2-yl | C(=O)Me | Cl | H | O |
| D-16537 | H | single bond | Me | thiazole-2-yl | C(=O)Me | Me | H | O |
| D-16538 | Me | single bond | H | thiazole-2-yl | C(=O)Me | Me | H | O |
| D-16539 | Me(R) | single bond | H | thiazole-2-yl | C(=O)Me | Me | H | O |
| D-16540 | Me(S) | single bond | H | thiazole-2-yl | C(=O)Me | Me | H | O |
| D-16541 | H | single bond | Me | 1-Me-pyrazol-3-yl | C(=O)Me | Cl | H | O |
| D-16542 | Me | single bond | H | 1-Me-pyrazol-3-yl | C(=O)Me | Cl | H | O |
| D-16543 | Me(R) | single bond | H | 1-Me-pyrazol-3-yl | C(=O)Me | Cl | H | O |
| D-16544 | Me(S) | single bond | H | 1-Me-pyrazol-3-yl | C(=O)Me | Cl | H | O |
| D-16545 | H | single bond | Me | 1-Me-pyrazol-3-yl | C(=O)Me | Me | H | O |
| D-16546 | Me | single bond | H | 1-Me-pyrazol-3-yl | C(=O)Me | Me | H | O |
| D-16547 | Me(R) | single bond | H | 1-Me-pyrazol-3-yl | C(=O)Me | Me | H | O |
| D-16548 | Me(S) | single bond | H | 1-Me-pyrazol-3-yl | C(=O)Me | Me | H | O |
| D-16549 | H | single bond | Me | 1-Me-pyrazol-5-yl | C(=O)Me | Cl | H | O |
| D-16550 | Me | single bond | H | 1-Me-pyrazol-5-yl | C(=O)Me | Cl | H | O |
| D-16551 | Me(R) | single bond | H | 1-Me-pyrazol-5-yl | C(=O)Me | Cl | H | O |
| D-16552 | Me(S) | single bond | H | 1-Me-pyrazol-5-yl | C(=O)Me | Cl | H | O |
| D-16553 | H | single bond | Me | 1-Me-pyrazol-5-yl | C(=O)Me | Me | H | O |
| D-16554 | Me | single bond | H | 1-Me-pyrazol-5-yl | C(=O)Me | Me | H | O |
| D-16555 | Me(R) | single bond | H | 1-Me-pyrazol-5-yl | C(=O)Me | Me | H | O |
| D-16556 | Me(S) | single bond | H | 1-Me-pyrazol-5-yl | C(=O)Me | Me | H | O |
| D-16557 | H | single bond | Me | CH₂(pyrimidin-2-yl) | C(=O)Me | Cl | H | O |
| D-16558 | Me | single bond | H | CH₂(pyrimidin-2-yl) | C(=O)Me | Cl | H | O |
| D-16559 | Me(R) | single bond | H | CH₂(pyrimidin-2-yl) | C(=O)Me | Cl | H | O |
| D-16560 | Me(S) | single bond | H | CH₂(pyrimidin-2-yl) | C(=O)Me | Cl | H | O |
| D-16561 | H | single bond | Me | CH₂(pyrimidin-2-yl) | C(=O)Me | Me | H | O |
| D-16562 | Me | single bond | H | CH₂(pyrimidin-2-yl) | C(=O)Me | Me | H | O |

**[Table 1023]**

| Compound Number | Y | W | Z | R¹ | R² | R³ | R¹⁰ | A |
|---|---|---|---|---|---|---|---|---|
| D-16563 | Me(R) | single bond | H | CH₂(pyrimidin-2-yl) | C(=O)Me | Me | H | O |
| D-16564 | Me(S) | single bond | H | CH₂(pyrimidin-2-yl) | C(=O)Me | Me | H | O |
| D-16565 | H | single bond | Me | Me | C(=O)Me | Cl | Me | O |
| D-16566 | Me | single bond | H | Me | C(=O)Me | Cl | Me | O |
| D-16567 | Me(R) | single bond | H | Me | C(=O)Me | Cl | Me | O |
| D-16568 | Me(S) | single bond | H | Me | C(=O)Me | Cl | Me | O |
| D-16569 | H | single bond | Me | Me | C(=O)Me | Me | Me | O |
| D-16570 | Me | single bond | H | Me | C(=O)Me | Me | Me | O |
| D-16571 | Me(R) | single bond | H | Me | C(=O)Me | Me | Me | O |
| D-16572 | Me(S) | single bond | H | Me | C(=O)Me | Me | Me | O |
| D-16573 | H | single bond | Me | CH₂CHF₂ | C(=O)Me | Cl | Me | O |
| D-16574 | Me | single bond | H | CH₂CHF₂ | C(=O)Me | Cl | Me | O |
| D-16575 | Me(R) | single bond | H | CH₂CHF₂ | C(=O)Me | Cl | Me | O |
| D-16576 | Me(S) | single bond | H | CH₂CHF₂ | C(=O)Me | Cl | Me | O |
| D-16577 | H | single bond | Me | CH₂CHF₂ | C(=O)Me | Me | Me | O |
| D-16578 | Me | single bond | H | CH₂CHF₂ | C(=O)Me | Me | Me | O |
| D-16579 | Me(R) | single bond | H | CH₂CHF₂ | C(=O)Me | Me | Me | O |
| D-16580 | Me(S) | single bond | H | CH₂CHF₂ | C(=O)Me | Me | Me | O |
| D-16581 | H | single bond | Me | *n*-Pr | C(=O)Me | Cl | Me | O |
| D-16582 | Me | single bond | H | *n*-Pr | C(=O)Me | Cl | Me | O |
| D-16583 | Me(R) | single bond | H | *n*-Pr | C(=O)Me | Cl | Me | O |
| D-16584 | Me(S) | single bond | H | *n*-Pr | C(=O)Me | Cl | Me | O |
| D-16585 | H | single bond | Me | *n*-Pr | C(=O)Me | Me | Me | O |
| D-16586 | Me | single bond | H | *n*-Pr | C(=O)Me | Me | Me | O |
| D-16587 | Me(R) | single bond | H | *n*-Pr | C(=O)Me | Me | Me | O |
| D-16588 | Me(S) | single bond | H | *n*-Pr | C(=O)Me | Me | Me | O |
| D-16589 | H | single bond | Me | i-Pr | C(=O)Me | Cl | Me | O |
| D-16590 | Me | single bond | H | i-Pr | C(=O)Me | Cl | Me | O |
| D-16591 | Me(R) | single bond | H | i-Pr | C(=O)Me | Cl | Me | O |
| D-16592 | Me(S) | single bond | H | i-Pr | C(=O)Me | Cl | Me | O |
| D-16593 | H | single bond | Me | i-Pr | C(=O)Me | Me | Me | O |
| D-16594 | Me | single bond | H | i-Pr | C(=O)Me | Me | Me | O |
| D-16595 | Me(R) | single bond | H | i-Pr | C(=O)Me | Me | Me | O |
| D-16596 | Me(S) | single bond | H | i-Pr | C(=O)Me | Me | Me | O |
| D-16597 | H | single bond | Me | c-Pr(1-F) | C(=O)Me | Cl | Me | O |
| D-16598 | Me | single bond | H | c-Pr(1-F) | C(=O)Me | Cl | Me | O |
| D-16599 | Me(R) | single bond | H | c-Pr(1-F) | C(=O)Me | Cl | Me | O |
| D-16600 | Me(S) | single bond | H | *c* -Pr(1-F) | C(=O)Me | Cl | Me | O |
| D-16601 | H | single bond | Me | c-Pr(1-F) | C(=O)Me | Me | Me | O |
| D-16602 | Me | single bond | H | c-Pr(1-F) | C(=O)Me | Me | Me | O |
| D-16603 | Me(R) | single bond | H | c-Pr(1-F) | C(=O)Me | Me | Me | O |
| D-16604 | Me(S) | single bond | H | c-Pr(1-F) | C(=O)Me | Me | Me | O |
| D-16605 | H | single bond | Me | *c*-Pr(2,2-F₂) | C(=O)Me | Cl | Me | O |
| D-16606 | Me | single bond | H | *c*-Pr(2,2-F₂) | C(=O)Me | Cl | Me | O |

**[Table 1024]**

| Compound Number | Y | W | Z | R¹ | R² | R³ | R¹⁰ | A |
|---|---|---|---|---|---|---|---|---|
| D-16607 | Me(R) | single bond | H | *c*-Pr(2,2-F₂) | C(=O)Me | Cl | Me | O |
| D-16608 | Me(S) | single bond | H | *c*-Pr(2,2-F₂) | C(=O)Me | Cl | Me | O |
| D-16609 | H | single bond | Me | *c*-Pr(2,2-F₂) | C(=O)Me | Me | Me | O |
| D-16610 | Me | single bond | H | *c*-Pr(2,2-F₂) | C(=O)Me | Me | Me | O |
| D-16611 | Me(R) | single bond | H | *c*-Pr(2,2-F₂) | C(=O)Me | Me | Me | O |
| D-16612 | Me(S) | single bond | H | *c*-Pr(2,2-F₂) | C(=O)Me | Me | Me | O |
| D-16613 | H | single bond | Me | *c*-Pr(1-Cl) | C(=O)Me | Cl | Me | O |
| D-16614 | Me | single bond | H | *c*-Pr(1-Cl) | C(=O)Me | Cl | Me | O |
| D-16615 | Me(R) | single bond | H | *c*-Pr(1-Cl) | C(=O)Me | Cl | Me | O |
| D-16616 | Me(S) | single bond | H | *c*-Pr(1-Cl) | C(=O)Me | Cl | Me | O |
| D-16617 | H | single bond | Me | *c*-Pr(1-Cl) | C(=O)Me | Me | Me | O |
| D-16618 | Me | single bond | H | *c*-Pr(1-Cl) | C(=O)Me | Me | Me | O |
| D-16619 | Me(R) | single bond | H | *c*-Pr(1-Cl) | C(=O)Me | Me | Me | O |
| D-16620 | Me(S) | single bond | H | *c*-Pr(1-Cl) | C(=O)Me | Me | Me | O |
| D-16621 | H | single bond | Me | c-Pr(1-Br) | C(=O)Me | Cl | Me | O |
| D-16622 | Me | single bond | H | c-Pr(1-Br) | C(=O)Me | Cl | Me | O |
| D-16623 | Me(R) | single bond | H | c-Pr(1-Br) | C(=O)Me | Cl | Me | O |
| D-16624 | Me(S) | single bond | H | c-Pr(1-Br) | C(=O)Me | Cl | Me | O |
| D-16625 | H | single bond | Me | c-Pr(1-Br) | C(=O)Me | Me | Me | O |
| D-16626 | Me | single bond | H | c-Pr(1-Br) | C(=O)Me | Me | Me | O |
| D-16627 | Me(R) | single bond | H | c-Pr(1-Br) | C(=O)Me | Me | Me | O |
| D-16628 | Me(S) | single bond | H | c-Pr(1-Br) | C(=O)Me | Me | Me | O |
| D-16629 | H | single bond | Me | *c*-Pr(1-Me) | C(=O)Me | Cl | Me | O |
| D-16630 | Me | single bond | H | *c*-Pr(1-Me) | C(=O)Me | Cl | Me | O |
| D-16631 | Me(R) | single bond | H | *c*-Pr(1-Me) | C(=O)Me | Cl | Me | O |
| D-16632 | Me(S) | single bond | H | *c*-Pr(1-Me) | C(=O)Me | Cl | Me | O |
| D-16633 | H | single bond | Me | *c*-Pr(1-Me) | C(=O)Me | Me | Me | O |
| D-16634 | Me | single bond | H | *c*-Pr(1-Me) | C(=O)Me | Me | Me | O |
| D-16635 | Me(R) | single bond | H | *c*-Pr(1-Me) | C(=O)Me | Me | Me | O |
| D-16636 | Me(S) | single bond | H | *c*-Pr(1-Me) | C(=O)Me | Me | Me | O |
| D-16637 | H | single bond | Me | *c*-Pr(1-CH₂OH) | C(=O)Me | Cl | Me | O |
| D-16638 | Me | single bond | H | *c*-Pr(1-CH₂OH) | C(=O)Me | Cl | Me | O |
| D-16639 | Me(R) | single bond | H | *c*-Pr(1-CH₂OH) | C(=O)Me | Cl | Me | O |
| D-16640 | Me(S) | single bond | H | *c*-Pr(1-CH₂OH) | C(=O)Me | Cl | Me | O |
| D-16641 | H | single bond | Me | *c*-Pr(1-CH₂OH) | C(=O)Me | Me | Me | O |
| D-16642 | Me | single bond | H | *c*-Pr(1-CH₂OH) | C(=O)Me | Me | Me | O |
| D-16643 | Me(R) | single bond | H | *c*-Pr(1-CH₂OH) | C(=O)Me | Me | Me | O |
| D-16644 | Me(S) | single bond | H | *c*-Pr(1-CH₂OH) | C(=O)Me | Me | Me | O |
| D-16645 | H | single bond | Me | *c*-Pr[1-CH₂OS(=O)₂Me] | C(=O)Me | Cl | Me | O |
| D-16646 | Me | single bond | H | *c*-Pr[1-CH₂OS(=O)₂Me] | C(=O)Me | Cl | Me | O |
| D-16647 | Me(R) | single bond | H | *c*-Pr[1-CH₂OS(=O)₂Me] | C(=O)Me | Cl | Me | O |
| D-16648 | Me(S) | single bond | H | *c*-Pr[1-CH₂OS(=O)₂Me] | C(=O)Me | Cl | Me | O |
| D-16649 | H | single bond | Me | *c*-Pr[1-CH₂OS(=O)₂Me] | C(=O)Me | Me | Me | O |
| D-16650 | Me | single bond | H | *c*-Pr[1-CH₂OS(=O)₂Me] | C(=O)Me | Me | Me | O |

**[Table 1025]**

| Compound Number | Y | W | Z | R¹ | R² | R³ | R¹⁰ | A |
|---|---|---|---|---|---|---|---|---|
| D-16651 | Me(R) | single bond | H | *c*-Pr[1-CH₂OS(=O)₂Me] | C(=O)Me | Me | Me | O |
| D-16652 | Me(S) | single bond | H | *c*-Pr[1-CH₂OS(=O)₂Me] | C(=O)Me | Me | Me | O |
| D-16653 | H | single bond | Me | *c*-Pr(1 -CH₂OMe) | C(=O)Me | Cl | Me | O |
| D-16654 | Me | single bond | H | *c*-Pr(1 -CH₂OMe) | C(=O)Me | Cl | Me | O |
| D-16655 | Me(R) | single bond | H | *c*-Pr(1 -CH₂OMe) | C(=O)Me | Cl | Me | O |
| D-16656 | Me(S) | single bond | H | *c*-Pr(1 -CH₂OMe) | C(=O)Me | Cl | Me | O |
| D-16657 | H | single bond | Me | *c*-Pr(1 -CH₂OMe) | C(=O)Me | Me | Me | O |
| D-16658 | Me | single bond | H | *c*-Pr(1 -CH₂OMe) | C(=O)Me | Me | Me | O |
| D-16659 | Me(R) | single bond | H | *c*-Pr(1 -CH₂OMe) | C(=O)Me | Me | Me | O |
| D-16660 | Me(S) | single bond | H | *c*-Pr(1 -CH₂OMe) | C(=O)Me | Me | Me | O |
| D-16661 | H | single bond | Me | *c*-Pr(1-CH₂OCN) | C(=O)Me | Cl | Me | O |
| D-16662 | Me | single bond | H | *c*-Pr(1-CH₂OCN) | C(=O)Me | Cl | Me | O |
| D-16663 | Me(R) | single bond | H | *c* -Pr(1-CH₂OCN) | C(=O)Me | Cl | Me | O |
| D-16664 | Me(S) | single bond | H | *c* -Pr(1-CH₂OCN) | C(=O)Me | Cl | Me | O |
| D-16665 | H | single bond | Me | *c* -Pr(1-CH₂OCN) | C(=O)Me | Me | Me | O |
| D-16666 | Me | single bond | H | *c* -Pr(1-CH₂OCN) | C(=O)Me | Me | Me | O |
| D-16667 | Me(R) | single bond | H | *c* -Pr(1-CH₂OCN) | C(=O)Me | Me | Me | O |
| D-16668 | Me(S) | single bond | H | *c* -Pr(1-CH₂OCN) | C(=O)Me | Me | Me | O |
| D-16669 | H | single bond | Me | *c* -Pr(1-CH₂SMe) | C(=O)Me | Cl | Me | O |
| D-16670 | Me | single bond | H | *c* -Pr(1-CH₂SMe) | C(=O)Me | Cl | Me | O |
| D-16671 | Me(R) | single bond | H | *c* -Pr(1-CH₂SMe) | C(=O)Me | Cl | Me | O |
| D-16672 | Me(S) | single bond | H | *c* -Pr(1-CH₂SMe) | C(=O)Me | Cl | Me | O |
| D-16673 | H | single bond | Me | *c* -Pr(1-CH₂SMe) | C(=O)Me | Me | Me | O |
| D-16674 | Me | single bond | H | *c* -Pr(1-CH₂SMe) | C(=O)Me | Me | Me | O |
| D-16675 | Me(R) | single bond | H | *c* -Pr(1-CH₂SMe) | C(=O)Me | Me | Me | O |
| D-16676 | Me(S) | single bond | H | *c* -Pr(1-CH₂SMe) | C(=O)Me | Me | Me | O |
| D-16677 | H | single bond | Me | *c* -Pr[1-CH₂S(=O)Me] | C(=O)Me | Cl | Me | O |
| D-16678 | Me | single bond | H | *c* -Pr[1-CH₂S(=O)Me] | C(=O)Me | Cl | Me | O |
| D-16679 | Me(R) | single bond | H | *c* -Pr[1-CH₂S(=O)Me] | C(=O)Me | Cl | Me | O |
| D-16680 | Me(S) | single bond | H | *c* -Pr[1-CH₂S(=O)Me] | C(=O)Me | Cl | Me | O |
| D-16681 | H | single bond | Me | *c*-Pr[1-CH₂S(=O)Me] | C(=O)Me | Me | Me | O |
| D-16682 | Me | single bond | H | *c* -Pr[1-CH₂S(=O)Me] | C(=O)Me | Me | Me | O |
| D-16683 | Me(R) | single bond | H | *c* -Pr[1-CH₂S(=O)Me] | C(=O)Me | Me | Me | O |
| D-16684 | Me(S) | single bond | H | *c* -Pr[1-CH₂S(=O)Me] | C(=O)Me | Me | Me | O |
| D-16685 | H | single bond | Me | c -Pr[1-C H₂S(=O)₂Me] | C(=O)Me | Cl | Me | O |
| D-16686 | Me | single bond | H | c -Pr[1-C H₂S(=O)₂Me] | C(=O)Me | Cl | Me | O |
| D-16687 | Me(R) | single bond | H | c -Pr[1-C H₂S(=O)₂Me] | C(=O)Me | Cl | Me | O |
| D-16688 | Me(S) | single bond | H | *c* -Pr[1-CH₂S(=O)₂Me] | C(=O)Me | Cl | Me | O |
| D-16689 | H | single bond | Me | c -Pr[1-C H₂S(=O)₂Me] | C(=O)Me | Me | Me | O |
| D-16690 | Me | single bond | H | c -Pr[1-C H₂S(=O)₂Me] | C(=O)Me | Me | Me | O |
| D-16691 | Me(R) | single bond | H | c -Pr[1-C H₂S(=O)₂Me] | C(=O)Me | Me | Me | O |
| D-16692 | Me(S) | single bond | H | c -Pr[1-C H₂S(=O)₂Me] | C(=O)Me | Me | Me | O |
| D-16693 | H | single bond | Me | *c* -Pr(1-CH₂F) | C(=O)Me | Cl | Me | O |
| D-16694 | Me | single bond | H | *c* -Pr(1-CH₂F) | C(=O)Me | Cl | Me | O |

**[Table 1026]**

| Compound Number | Y | W | Z | R¹ | R² | R³ | R¹⁰ | A |
|---|---|---|---|---|---|---|---|---|
| D-16695 | Me(R) | single bond | H | *c* -Pr(1-CH₂F) | C(=O)Me | Cl | Me | O |
| D-16696 | Me(S) | single bond | H | *c* -Pr(1-CH₂F) | C(=O)Me | Cl | Me | O |
| D-16697 | H | single bond | Me | *c* -Pr(1-CH₂F) | C(=O)Me | Me | Me | O |
| D-16698 | Me | single bond | H | *c* -Pr(1-CH₂F) | C(=O)Me | Me | Me | O |
| D-16699 | Me(R) | single bond | H | *c* -Pr(1-CH₂F) | C(=O)Me | Me | Me | O |
| D-16700 | Me(S) | single bond | H | c -Pr(1-CH₂F) | C(=O)Me | Me | Me | O |
| D-16701 | H | single bond | Me | *c* -Pr(1-CHF₂) | C(=O)Me | Cl | Me | O |
| D-16702 | Me | single bond | H | *c* -Pr(1-CHF₂) | C(=O)Me | Cl | Me | O |
| D-16703 | Me(R) | single bond | H | *c* -Pr(1-CHF₂) | C(=O)Me | Cl | Me | O |
| D-16704 | Me(S) | single bond | H | *c* -Pr(1-CHF₂) | C(=O)Me | Cl | Me | O |
| D-16705 | H | single bond | Me | *c* -Pr(1-CHF₂) | C(=O)Me | Me | Me | O |
| D-16706 | Me | single bond | H | *c* -Pr(1-CHF₂) | C(=O)Me | Me | Me | O |
| D-16707 | Me(R) | single bond | H | *c* -Pr(1-CHF₂) | C(=O)Me | Me | Me | O |
| D-16708 | Me(S) | single bond | H | *c* -Pr(1-CHF₂) | C(=O)Me | Me | Me | O |
| D-16709 | H | single bond | Me | *c* -Pr(1-CF₃) | C(=O)Me | Cl | Me | O |
| D-16710 | Me | single bond | H | *c* -Pr(1-CF₃) | C(=O)Me | Cl | Me | O |
| D-16711 | Me(R) | single bond | H | *c* -Pr(1-CF₃) | C(=O)Me | Cl | Me | O |
| D-16712 | Me(S) | single bond | H | *c* -Pr(1-CF₃) | C(=O)Me | Cl | Me | O |
| D-16713 | H | single bond | Me | *c* -Pr(1-CF₃) | C(=O)Me | Me | Me | O |
| D-16714 | Me | single bond | H | *c* -Pr(1-CF₃) | C(=O)Me | Me | Me | O |
| D-16715 | Me(R) | single bond | H | *c* -Pr(1-CF₃) | C(=O)Me | Me | Me | O |
| D-16716 | Me(S) | single bond | H | *c* -Pr(1-CF₃) | C(=O)Me | Me | Me | O |
| D-16717 | H | single bond | Me | *c* -Pr(1-C≡CH) | C(=O)Me | Cl | Me | O |
| D-16718 | Me | single bond | H | *c* -Pr(1-C≡CH) | C(=O)Me | Cl | Me | O |
| D-16719 | Me(R) | single bond | H | *c* -Pr(1-C≡CH) | C(=O)Me | Cl | Me | O |
| D-16720 | Me(S) | single bond | H | *c* -Pr(1-C≡CH) | C(=O)Me | Cl | Me | O |
| D-16721 | H | single bond | Me | *c* -pr(1-C≡CH) | C(=O)Me | Me | Me | O |
| D-16722 | Me | single bond | H | *c* -Pr(1-C≡CH) | C(=O)Me | Me | Me | O |
| D-16723 | Me(R) | single bond | H | *c* -Pr(1-C≡CH) | C(=O)Me | Me | Me | O |
| D-16724 | Me(S) | single bond | H | *c* -Pr(1-C≡CH) | C(=O)Me | Me | Me | O |
| D-16725 | H | single bond | Me | c-Pr[1-C(=O)OEt] | C(=O)Me | Cl | Me | O |
| D-16726 | Me | single bond | H | c-Pr[1-C(=O)OEt] | C(=O)Me | Cl | Me | O |
| D-16727 | Me(R) | single bond | H | c-Pr[1-C(=O)OEt] | C(=O)Me | Cl | Me | O |
| D-16728 | Me(S) | single bond | H | c-Pr[1-C(=O)OEt] | C(=O)Me | Cl | Me | O |
| D-16729 | H | single bond | Me | c-Pr[1-C(=O)OEt] | C(=O)Me | Me | Me | O |
| D-16730 | Me | single bond | H | c-Pr[1-C(=O)OEt] | C(=O)Me | Me | Me | O |
| D-16731 | Me(R) | single bond | H | c-Pr[1-C(=O)OEt] | C(=O)Me | Me | Me | O |
| D-16732 | Me(S) | single bond | H | c-Pr[1-C(=O)OEt] | C(=O)Me | Me | Me | O |
| D-16733 | H | single bond | Me | *c* -Pr[1-C(=O)OH] | C(=O)Me | Cl | Me | O |
| D-16734 | Me | single bond | H | *c* -Pr[1-C(=O)OH] | C(=O)Me | Cl | Me | O |
| D-16735 | Me(R) | single bond | H | *c* -Pr[1-C(=O)OH] | C(=O)Me | Cl | Me | O |
| D-16736 | Me(S) | single bond | H | *c* -Pr[1-C(=O)OH] | C(=O)Me | Cl | Me | O |
| D-16737 | H | single bond | Me | *c* -Pr[1-C(=O)OH] | C(=O)Me | Me | Me | O |
| D-16738 | Me | single bond | H | *c* -Pr[1-C(=O)OH] | C(=O)Me | Me | Me | O |

**[Table 1027]**

| Compound Number | Y | W | Z | R¹ | R² | R³ | R¹⁰ | A |
|---|---|---|---|---|---|---|---|---|
| D-16739 | Me(R) | single bond | H | *c* -Pr[1-C(=O)OH] | C(=O)Me | Me | Me | O |
| D-16740 | Me(S) | single bond | H | *c* -Pr[1-C(=O)OH] | C(=O)Me | Me | Me | O |
| D-16741 | H | single bond | Me | *c* -Pr[1-C(=O)NH₂] | C(=O)Me | Cl | Me | O |
| D-16742 | Me | single bond | H | *c* -Pr[1-C(=O)NH₂ | C(=O)Me | Cl | Me | O |
| D-16743 | Me(R) | single bond | H | *c* -Pr[1-C(=O)NH₂ | C(=O)Me | Cl | Me | O |
| D-16744 | Me(S) | single bond | H | *c* -Pr[1-C(=O)NH₂] | C(=O)Me | Cl | Me | O |
| D-16745 | H | single bond | Me | *c* -Pr[1-C(=O)NH₂] | C(=O)Me | Me | Me | O |
| D-16746 | Me | single bond | H | *c* -Pr[1-C(=O)NH₂ | C(=O)Me | Me | Me | O |
| D-16747 | Me(R) | single bond | H | *c* -Pr[1-C(=O)NH₂ | C(=O)Me | Me | Me | O |
| D-16748 | Me(S) | single bond | H | *c* -Pr[1-C(=O)NH₂ | C(=O)Me | Me | Me | O |
| D-16749 | H | single bond | Me | c-Pr(1-OMe) | C(=O)Me | Cl | Me | O |
| D-16750 | Me | single bond | H | c-Pr(1-OMe) | C(=O)Me | Cl | Me | O |
| D-16751 | Me(R) | single bond | H | *c* -Pᵣ(1-OMe) | C(=O)Me | Cl | Me | O |
| D-16752 | Me(S) | single bond | H | c-Pr(1-OMe) | C(=O)Me | Cl | Me | O |
| D-16753 | H | single bond | Me | c-Pr(1-OMe) | C(=O)Me | Me | Me | O |
| D-16754 | Me | single bond | H | c-Pr(1-OMe) | C(=O)Me | Me | Me | O |
| D-16755 | Me(R) | single bond | H | c-Pr(1-OMe) | C(=O)Me | Me | Me | O |
| D-16756 | Me(S) | single bond | H | c-Pr(1-OMe) | C(=O)Me | Me | Me | O |
| D-16757 | H | single bond | Me | c-Pr(1-SMe) | C(=O)Me | Cl | Me | O |
| D-16758 | Me | single bond | H | c-Pr(1-SMe) | C(=O)Me | Cl | Me | O |
| D-16759 | Me(R) | single bond | H | c-Pr(1-SMe) | C(=O)Me | Cl | Me | O |
| D-16760 | Me(S) | single bond | H | c-Pr(1-SMe) | C(=O)Me | Cl | Me | O |
| D-16761 | H | single bond | Me | c-Pr(1-SMe) | C(=O)Me | Me | Me | O |
| D-16762 | Me | single bond | H | c-Pr(1-SMe) | C(=O)Me | Me | Me | O |
| D-16763 | Me(R) | single bond | H | c-Pr(1-SMe) | C(=O)Me | Me | Me | O |
| D-16764 | Me(S) | single bond | H | c-Pr(1-SMe) | C(=O)Me | Me | Me | O |
| D-16765 | H | single bond | Me | *c* -Pr[1-S(=O)Me] | C(=O)Me | Cl | Me | O |
| D-16766 | Me | single bond | H | *c* -Pr[1-S(=O)Me] | C(=O)Me | Cl | Me | O |
| D-16767 | Me(R) | single bond | H | *c* -Pr[1-S(=O)Me] | C(=O)Me | Cl | Me | O |
| D-16768 | Me(S) | single bond | H | *c* -Pr[1-S(=O)Me] | C(=O)Me | Cl | Me | O |
| D-16769 | H | single bond | Me | *c* -Pr[1-S(=O)Me] | C(=O)Me | Me | Me | O |
| D-16770 | Me | single bond | H | *c* -Pr[1-S(=O)Me] | C(=O)Me | Me | Me | O |
| D-16771 | Me(R) | single bond | H | *c* -Pr[1-S(=O)Me] | C(=O)Me | Me | Me | O |
| D-16772 | Me(S) | single bond | H | *c* -Pr[1-S(=O)Me] | C(=O)Me | Me | Me | O |
| D-16773 | H | single bond | Me | *c* -Pr[1-S(=O)₂Me] | C(=O)Me | Cl | Me | O |
| D-16774 | Me | single bond | H | *c* -Pr[1-S(=O)₂Me] | C(=O)Me | Cl | Me | O |
| D-16775 | Me(R) | single bond | H | *c* -Pr[1-S(=O)₂Me] | C(=O)Me | Cl | Me | O |
| D-16776 | Me(S) | single bond | H | *c* -Pr[1-S(=O)₂Me] | C(=O)Me | Cl | Me | O |
| D-16777 | H | single bond | Me | *c* -Pr[1-S(=O)₂Me] | C(=O)Me | Me | Me | O |
| D-16778 | Me | single bond | H | *c* -Pr[1-S(=O)₂Me] | C(=O)Me | Me | Me | O |
| D-16779 | Me(R) | single bond | H | *c* -Pr[1-S(=O)₂Me] | C(=O)Me | Me | Me | O |
| D-16780 | Me(S) | single bond | H | *c* -Pr[1-S(=O)₂Me] | C(=O)Me | Me | Me | O |
| D-16781 | H | single bond | Me | *c* -Pr[1-S(=O)₂CHF₂] | C(=O)Me | Cl | Me | O |
| D-16782 | Me | single bond | H | *c* -Pr[1-S(=O)₂CHF₂] | C(=O)Me | Cl | Me | O |

**[Table 1028]**

| Compound Number | Y | W | Z | R¹ | R² | R³ | R¹⁰ | A |
|---|---|---|---|---|---|---|---|---|
| D-16783 | Me(R) | single bond | H | *c* -Pr[1-S(=O)₂CHF₂] | C(=O)Me | Cl | Me | O |
| D-16784 | Me(S) | single bond | H | *c* -Pr[1-S(=O)₂CHF₂] | C(=O)Me | Cl | Me | O |
| D-16785 | H | single bond | Me | *c* -Pr[1-S(=O)₂CHF₂] | C(=O)Me | Me | Me | O |
| D-16786 | Me | single bond | H | *c* -Pr[1-S(=O)₂CHF₂] | C(=O)Me | Me | Me | O |
| D-16787 | Me(R) | single bond | H | *c* -Pr[1-S(=O)₂CHF₂] | C(=O)Me | Me | Me | O |
| D-16788 | Me(S) | single bond | H | *c* -Pr[1-S(=O)₂CHF₂] | C(=O)Me | Me | Me | O |
| D-16789 | H | single bond | Me | *c* -Pr[1-S(=O)₂CF₃] | C(=O)Me | Cl | Me | O |
| D-16790 | Me | single bond | H | *c* -Pr[1-S(=O)₂CF₃] | C(=O)Me | Cl | Me | O |
| D-16791 | Me(R) | single bond | H | *c* -Pr[1-S(=O)₂CF₃] | C(=O)Me | Cl | Me | O |
| D-16792 | Me(S) | single bond | H | *c* -Pr[1-S(=O)₂CF₃] | C(=O)Me | Cl | Me | O |
| D-16793 | H | single bond | Me | *c* -Pr[1-S(=O)₂CF₃] | C(=O)Me | Me | Me | O |
| D-16794 | Me | single bond | H | *c* -Pr[1-S(=O)₂CF₃] | C(=O)Me | Me | Me | O |
| D-16795 | Me(R) | single bond | H | *c* -Pr[1-S(=O)₂CF₃] | C(=O)Me | Me | Me | O |
| D-16796 | Me(S) | single bond | H | *c* -Pr[1-S(=O)₂CF₃] | C(=O)Me | Me | Me | O |
| D-16797 | H | single bond | Me | c -Pr[1-(5,6-dihydro-1 ,4,2-dioxazin-3-yl)] | C(=O)Me | Cl | Me | O |
| D-16798 | Me | single bond | H | c -Pr[1-(5,6-dihydro-1 ,4,2-dioxazin-3-yl)] | C(=O)Me | Cl | Me | O |
| D-16799 | Me(R) | single bond | H | c -Pr[1-(5,6-dihydro-1 ,4,2-dioxazin-3-yl)] | C(=O)Me | Cl | Me | O |
| D-16800 | Me(S) | single bond | H | c-Pr[1-(5,6-dihydro-1,4,2-dioxazin-3-yl)] | C(=O)Me | Cl | Me | O |
| D-16801 | H | single bond | Me | c -Pr[1-(5,6-dihydro-1 ,4,2-dioxazin-3-yl)] | C(=O)Me | Me | Me | O |
| D-16802 | Me | single bond | H | c -Pr[1-(5,6-dihydro-1 ,4,2-dioxazin-3-yl)] | C(=O)Me | Me | Me | O |
| D-16803 | Me(R) | single bond | H | c -Pr[1-(5,6-dihydro-1 ,4,2-dioxazin-3-yl)] | C(=O)Me | Me | Me | O |
| D-16804 | Me(S) | single bond | H | c -Pr[1-(5,6-dihydro-1 ,4,2-dioxazin-3-yl)] | C(=O)Me | Me | Me | O |
| D-16805 | H | single bond | Me | c -Pr[1-(5-hydroxy-1 ,3,4-oxadiazol-2-yl)] | C(=O)Me | Cl | Me | O |
| D-16806 | Me | single bond | H | c -Pr[1-(5-hydroxy-1 ,3,4-oxadiazol-2-yl)] | C(=O)Me | Cl | Me | O |
| D-16807 | Me(R) | single bond | H | c -Pr[1-(5-hydroxy-1 ,3,4-oxadiazol-2-yl)] | C(=O)Me | Cl | Me | O |
| D-16808 | Me(S) | single bond | H | c -Pr[1-(5-hydroxy-1 ,3,4-oxadiazol-2-yl)] | C(=O)Me | Cl | Me | O |
| D-16809 | H | single bond | Me | c -Pr[1-(5-hydroxy-1 ,3,4-oxadiazol-2-yl)] | C(=O)Me | Me | Me | O |
| D-16810 | Me | single bond | H | c -Pr[1-(5-hydroxy-1 ,3,4-oxadiazol-2-yl)] | C(=O)Me | Me | Me | O |
| D-16811 | Me(R) | single bond | H | c -Pr[1-(5-hydroxy-1 ,3,4-oxadiazol-2-yl)] | C(=O)Me | Me | Me | O |
| D-16812 | Me(S) | single bond | H | c -Pr[1-(5-hydroxy-1 ,3,4-oxadiazol-2-yl)] | C(=O)Me | Me | Me | O |
| D-16813 | H | single bond | Me | c -Pr[1-(pyri midin-2-yl)] | C(=O)Me | Cl | Me | O |
| D-16814 | Me | single bond | H | c -Pr[1-(pyri midin-2-yl)] | C(=O)Me | Cl | Me | O |
| D-16815 | Me(R) | single bond | H | c -Pr[1-(pyri midin-2-yl)] | C(=O)Me | Cl | Me | O |
| D-16816 | Me(S) | single bond | H | c -Pr[1-(pyri midin-2-yl)] | C(=O)Me | Cl | Me | O |
| D-16817 | H | single bond | Me | c -Pr[1-(pyri midin-2-yl)] | C(=O)Me | Me | Me | O |
| D-16818 | Me | single bond | H | c -Pr[1-(pyri midin-2-yl)] | C(=O)Me | Me | Me | O |
| D-16819 | Me(R) | single bond | H | c -Pr[1-(pyri midin-2-yl)] | C(=O)Me | Me | Me | O |
| D-16820 | Me(S) | single bond | H | c -Pr[1-(pyri midin-2-yl)] | C(=O)Me | Me | Me | O |
| D-16821 | H | single bond | Me | t-Bu | C(=O)Me | Cl | Me | O |
| D-16822 | Me | single bond | H | t-Bu | C(=O)Me | Cl | Me | O |
| D-16823 | Me(R) | single bond | H | t-Bu | C(=O)Me | Cl | Me | O |
| D-16824 | Me(S) | single bond | H | t-Bu | C(=O)Me | Cl | Me | O |
| D-16825 | H | single bond | Me | t-Bu | C(=O)Me | Me | Me | O |
| D-16826 | Me | single bond | H | t-Bu | C(=O)Me | Me | Me | O |

**[Table 1029]**

| Compound Number | Y | W | Z | R¹ | R² | R³ | R¹⁰ | A |
|---|---|---|---|---|---|---|---|---|
| D-16827 | Me(R) | single bond | H | t-Bu | C(=O)Me | Me | Me | O |
| D-16828 | Me(S) | single bond | H | t-Bu | C(=O)Me | Me | Me | O |
| D-16829 | H | single bond | Me | *c* -Bu | C(=O)Me | Cl | Me | O |
| D-16830 | Me | single bond | H | *c* -Bu | C(=O)Me | Cl | Me | O |
| D-16831 | Me(R) | single bond | H | *c* -Bu | C(=O)Me | Cl | Me | O |
| D-16832 | Me(S) | single bond | H | *c* -Bu | C(=O)Me | Cl | Me | O |
| D-16833 | H | single bond | Me | *c* -Bu | C(=O)Me | Me | Me | O |
| D-16834 | Me | single bond | H | *c* -Bu | C(=O)Me | Me | Me | O |
| D-16835 | Me(R) | single bond | H | *c* -Bu | C(=O)Me | Me | Me | O |
| D-16836 | Me(S) | single bond | H | *c* -Bu | C(=O)Me | Me | Me | O |
| D-16837 | H | single bond | Me | c-Bu(1-CN) | C(=O)Me | Cl | Me | O |
| D-16838 | Me | single bond | H | c-Bu(1-CN) | C(=O)Me | Cl | Me | O |
| D-16839 | Me(R) | single bond | H | *c* -Bu(1-CN) | C(=O)Me | Cl | Me | O |
| D-16840 | Me(S) | single bond | H | c-Bu(1-CN) | C(=O)Me | Cl | Me | O |
| D-16841 | H | single bond | Me | c-Bu(1-CN) | C(=O)Me | Me | Me | O |
| D-16842 | Me | single bond | H | c-Bu(1-CN) | C(=O)Me | Me | Me | O |
| D-16843 | Me(R) | single bond | H | c-Bu(1-CN) | C(=O)Me | Me | Me | O |
| D-16844 | Me(S) | single bond | H | c-Bu(1-CN) | C(=O)Me | Me | Me | O |
| D-16845 | H | single bond | Me | *c* -Bu(3,3-F₂) | C(=O)Me | Cl | Me | O |
| D-16846 | Me | single bond | H | *c* -Bu(3,3-F₂) | C(=O)Me | Cl | Me | O |
| D-16847 | Me(R) | single bond | H | *c* -Bu(3,3-F₂) | C(=O)Me | Cl | Me | O |
| D-16848 | Me(S) | single bond | H | *c* -Bu(3,3-F₂) | C(=O)Me | Cl | Me | O |
| D-16849 | H | single bond | Me | *c* -Bu(3,3-F₂) | C(=O)Me | Me | Me | O |
| D-16850 | Me | single bond | H | *c* -Bu(3,3-F₂) | C(=O)Me | Me | Me | O |
| D-16851 | Me(R) | single bond | H | *c* -Bu(3,3-F₂) | C(=O)Me | Me | Me | O |
| D-16852 | Me(S) | single bond | H | *c* -Bu(3,3-F₂) | C(=O)Me | Me | Me | O |
| D-16853 | H | single bond | Me | oxetan-3-yl | C(=O)Me | Cl | Me | O |
| D-16854 | Me | single bond | H | oxetan-3-yl | C(=O)Me | Cl | Me | O |
| D-16855 | Me(R) | single bond | H | oxetan-3-yl | C(=O)Me | Cl | Me | O |
| D-16856 | Me(S) | single bond | H | oxetan-3-yl | C(=O)Me | Cl | Me | O |
| D-16857 | H | single bond | Me | oxetan-3-yl | C(=O)Me | Me | Me | O |
| D-16858 | Me | single bond | H | oxetan-3-yl | C(=O)Me | Me | Me | O |
| D-16859 | Me(R) | single bond | H | oxetan-3-yl | C(=O)Me | Me | Me | O |
| D-16860 | Me(S) | single bond | H | oxetan-3-yl | C(=O)Me | Me | Me | O |
| D-16861 | H | single bond | Me | oxetan(3-CN)-3-yl | C(=O)Me | Cl | Me | O |
| D-16862 | Me | single bond | H | oxetan(3-CN)-3-yl | C(=O)Me | Cl | Me | O |
| D-16863 | Me(R) | single bond | H | oxetan(3-CN)-3-yl | C(=O)Me | Cl | Me | O |
| D-16864 | Me(S) | single bond | H | oxetan(3-CN)-3-yl | C(=O)Me | Cl | Me | O |
| D-16865 | H | single bond | Me | oxetan(3-CN)-3-yl | C(=O)Me | Me | Me | O |
| D-16866 | Me | single bond | H | oxetan(3-CN)-3-yl | C(=O)Me | Me | Me | O |
| D-16867 | Me(R) | single bond | H | oxetan(3-CN)-3-yl | C(=O)Me | Me | Me | O |
| D-16868 | Me(S) | single bond | H | oxetan(3-CN)-3-yl | C(=O)Me | Me | Me | O |
| D-16869 | H | single bond | Me | azetidin[1-C(=O)Me]-3-yl | C(=O)Me | Cl | Me | O |
| D-16870 | Me | single bond | H | azetidin[1-C(=O)Me]-3-yl | C(=O)Me | Cl | Me | O |

**[Table 1030]**

| Compound Number | Y | W | Z | R¹ | R² | R³ | R¹⁰ | A |
|---|---|---|---|---|---|---|---|---|
| D-16871 | Me(R) | single bond | H | azetidin[1-C(=O)Me]-3-yl | C(=O)Me | Cl | Me | O |
| D-16872 | Me(S) | single bond | H | azetidin[1-C(=O)Me]-3-yl | C(=O)Me | Cl | Me | O |
| D-16873 | H | single bond | Me | azetidin[1-C(=O)Me]-3-yl | C(=O)Me | Me | Me | O |
| D-16874 | Me | single bond | H | azetidin[1-C(=O)Me]-3-yl | C(=O)Me | Me | Me | O |
| D-16875 | Me(R) | single bond | H | azetidin[1-C(=O)Me]-3-yl | C(=O)Me | Me | Me | O |
| D-16876 | Me(S) | single bond | H | azetidin[1-C(=O)Me]-3-yl | C(=O)Me | Me | Me | O |
| D-16877 | H | single bond | Me | azetidin[1-S(=O)₂Me]-3-yl | C(=O)Me | Cl | Me | O |
| D-16878 | Me | single bond | H | azetidin[1-S(=O)₂Me]-3-yl | C(=O)Me | Cl | Me | O |
| D-16879 | Me(R) | single bond | H | azetidin[1-S(=O)₂Me]-3-yl | C(=O)Me | Cl | Me | O |
| D-16880 | Me(S) | single bond | H | azetidin[1-S(=O)₂Me]-3-yl | C(=O)Me | Cl | Me | O |
| D-16881 | H | single bond | Me | azetidin[1-S(=O)₂Me]-3-yl | C(=O)Me | Me | Me | O |
| D-16882 | Me | single bond | H | azetidin[1-S(=O)₂Me]-3-yl | C(=O)Me | Me | Me | O |
| D-16883 | Me(R) | single bond | H | azetidin[1-S(=O)₂Me]-3-yl | C(=O)Me | Me | Me | O |
| D-16884 | Me(S) | single bond | H | azetidin[1-S(=O)₂Me]-3-yl | C(=O)Me | Me | Me | O |
| D-16885 | H | single bond | Me | azetidin[1-S(=O)₂CHF₂]-3-yl | C(=O)Me | Cl | Me | O |
| D-16886 | Me | single bond | H | azetidin[1-S(=O)₂CHF₂]-3-yl | C(=O)Me | Cl | Me | O |
| D-16887 | Me(R) | single bond | H | azetidin[1-S(=O)₂CHF₂]-3-yl | C(=O)Me | Cl | Me | O |
| D-16888 | Me(S) | single bond | H | azetidin[1-S(=O)₂CHF₂]-3-yl | C(=O)Me | Cl | Me | O |
| D-16889 | H | single bond | Me | azetidin[1-S(=O)₂CHF₂]-3-yl | C(=O)Me | Me | Me | O |
| D-16890 | Me | single bond | H | azetidin[1-S(=O)₂CHF₂]-3-yl | C(=O)Me | Me | Me | O |
| D-16891 | Me(R) | single bond | H | azetidin[1-S(=O)₂CHF₂]-3-yl | C(=O)Me | Me | Me | O |
| D-16892 | Me(S) | single bond | H | azetidin[1-S(=O)₂CHF₂]-3-yl | C(=O)Me | Me | Me | O |
| D-16893 | H | single bond | Me | c-Pen | C(=O)Me | Cl | Me | O |
| D-16894 | Me | single bond | H | c-Pen | C(=O)Me | Cl | Me | O |
| D-16895 | Me(R) | single bond | H | c-Pen | C(=O)Me | Cl | Me | O |
| D-16896 | Me(S) | single bond | H | c-Pen | C(=O)Me | Cl | Me | O |
| D-16897 | H | single bond | Me | c-Pen | C(=O)Me | Me | Me | O |
| D-16898 | Me | single bond | H | *c* -Pen | C(=O)Me | Me | Me | O |
| D-16899 | Me(R) | single bond | H | c-Pen | C(=O)Me | Me | Me | O |
| D-16900 | Me(S) | single bond | H | c-Pen | C(=O)Me | Me | Me | O |
| D-16901 | H | single bond | Me | tetrahydrofuran-3-yl | C(=O)Me | Cl | Me | O |
| D-16902 | Me | single bond | H | tetrahydrofuran-3-yl | C(=O)Me | Cl | Me | O |
| D-16903 | Me(R) | single bond | H | tetrahydrofuran-3-yl | C(=O)Me | Cl | Me | O |
| D-16904 | Me(S) | single bond | H | tetrahydrofuran-3-yl | C(=O)Me | Cl | Me | O |
| D-16905 | H | single bond | Me | tetrahydrofuran-3-yl | C(=O)Me | Me | Me | O |
| D-16906 | Me | single bond | H | tetrahydrofuran-3-yl | C(=O)Me | Me | Me | O |
| D-16907 | Me(R) | single bond | H | tetrahydrofuran-3-yl | C(=O)Me | Me | Me | O |
| D-16908 | Me(S) | single bond | H | tetrahydrofuran-3-yl | C(=O)Me | Me | Me | O |
| D-16909 | H | single bond | Me | tetrahydrothiophen-3-yl | C(=O)Me | Cl | Me | O |
| D-16910 | Me | single bond | H | tetrahydrothiophen-3-yl | C(=O)Me | Cl | Me | O |
| D-16911 | Me(R) | single bond | H | tetrahydrothiophen-3-yl | C(=O)Me | Cl | Me | O |
| D-16912 | Me(S) | single bond | H | tetrahydrothiophen-3-yl | C(=O)Me | Cl | Me | O |
| D-16913 | H | single bond | Me | tetrahydrothiophen-3-yl | C(=O)Me | Me | Me | O |
| D-16914 | Me | single bond | H | tetrahydrothiophen-3-yl | C(=O)Me | Me | Me | O |

**[Table 1031]**

| Compound Number | Y | W | Z | R¹ | R² | R³ | R¹⁰ | A |
|---|---|---|---|---|---|---|---|---|
| D-16915 | Me(R) | single bond | H | tetrahydrothiophen-3-yl | C(=O)Me | Me | Me | O |
| D-16916 | Me(S) | single bond | H | tetrahydrothiophen-3-yl | C(=O)Me | Me | Me | O |
| D-16917 | H | single bond | Me | *c*-Pen(1-CN) | C(=O)Me | Cl | Me | O |
| D-16918 | Me | single bond | H | *c*-Pen(1-CN) | C(=O)Me | Cl | Me | O |
| D-16919 | Me(R) | single bond | H | *c*-Pen(1-CN) | C(=O)Me | Cl | Me | O |
| D-16920 | Me(S) | single bond | H | *c*-Pen(1-CN) | C(=O)Me | Cl | Me | O |
| D-16921 | H | single bond | Me | *c*-Pen(1-CN) | C(=O)Me | Me | Me | O |
| D-16922 | Me | single bond | H | *c*-Pen(1-CN) | C(=O)Me | Me | Me | O |
| D-16923 | Me(R) | single bond | H | *c*-Pen(1-CN) | C(=O)Me | Me | Me | O |
| D-16924 | Me(S) | single bond | H | *c*-Pen(1-CN) | C(=O)Me | Me | Me | O |
| D-16925 | H | single bond | Me | tetrahydrofuran(3-CN)-3-yl | C(=O)Me | Cl | Me | O |
| D-16926 | Me | single bond | H | tetrahydrofuran(3-CN)-3-yl | C(=O)Me | Cl | Me | O |
| D-16927 | Me(R) | single bond | H | tetrahydrofuran(3-CN)-3-yl | C(=O)Me | Cl | Me | O |
| D-16928 | Me(S) | single bond | H | tetrahydrofuran(3-CN)-3-yl | C(=O)Me | Cl | Me | O |
| D-16929 | H | single bond | Me | tetrahydrofuran(3-CN)-3-yl | C(=O)Me | Me | Me | O |
| D-16930 | Me | single bond | H | tetrahydrofuran(3-CN)-3-yl | C(=O)Me | Me | Me | O |
| D-16931 | Me(R) | single bond | H | tetrahydrofuran(3-CN)-3-yl | C(=O)Me | Me | Me | O |
| D-16932 | Me(S) | single bond | H | tetrahydrofuran(3-CN)-3-yl | C(=O)Me | Me | Me | O |
| D-16933 | H | single bond | Me | tetrahydrothiophen(3-CN)-3-yl | C(=O)Me | Cl | Me | O |
| D-16934 | Me | single bond | H | tetrahydrothiophen(3-CN)-3-yl | C(=O)Me | Cl | Me | O |
| D-16935 | Me(R) | single bond | H | tetrahydrothiophen(3-CN)-3-yl | C(=O)Me | Cl | Me | O |
| D-16936 | Me(S) | single bond | H | tetrahydrothiophen(3-CN)-3-yl | C(=O)Me | Cl | Me | O |
| D-16937 | H | single bond | Me | tetrahydrothiophen(3-CN)-3-yl | C(=O)Me | Me | Me | O |
| D-16938 | Me | single bond | H | tetrahydrothiophen(3-CN)-3-yl | C(=O)Me | Me | Me | O |
| D-16939 | Me(R) | single bond | H | tetrahydrothiophen(3-CN)-3-yl | C(=O)Me | Me | Me | O |
| D-16940 | Me(S) | single bond | H | tetrahydrothiophen(3-CN)-3-yl | C(=O)Me | Me | Me | O |
| D-16941 | H | single bond | Me | bicyclo[1.1.1]pertan-1-yl | C(=O)Me | Cl | Me | O |
| D-16942 | Me | single bond | H | bicyclo[1.1.1]pertan-1-yl | C(=O)Me | Cl | Me | O |
| D-16943 | Me(R) | single bond | H | bicyclo[1.1.1]pertan-1-yl | C(=O)Me | Cl | Me | O |
| D-16944 | Me(S) | single bond | H | bicyclo[1.1.1]pertan-1-yl | C(=O)Me | Cl | Me | O |
| D-16945 | H | single bond | Me | bicyclo[1.1.1]pertan-1-yl | C(=O)Me | Me | Me | O |
| D-16946 | Me | single bond | H | bicyclo[1.1.1]pertan-1-yl | C(=O)Me | Me | Me | O |
| D-16947 | Me(R) | single bond | H | bicyclo[1.1.1]pertan-1-yl | C(=O)Me | Me | Me | O |
| D-16948 | Me(S) | single bond | H | bicyclo[1.1.1]pertan-1-yl | C(=O)Me | Me | Me | O |
| D-16949 | H | single bond | Me | *c*-Hex | C(=O)Me | Cl | Me | O |
| D-16950 | Me | single bond | H | *c*-Hex | C(=O)Me | Cl | Me | O |
| D-16951 | Me(R) | single bond | H | *c*-Hex | C(=O)Me | Cl | Me | O |
| D-16952 | Me(S) | single bond | H | *c*-Hex | C(=O)Me | Cl | Me | O |
| D-16953 | H | single bond | Me | *c*-Hex | C(=O)Me | Me | Me | O |
| D-16954 | Me | single bond | H | *c*-Hex | C(=O)Me | Me | Me | O |
| D-16955 | Me(R) | single bond | H | *c*-Hex | C(=O)Me | Me | Me | O |
| D-16956 | Me(S) | single bond | H | *c*-Hex | C(=O)Me | Me | Me | O |
| D-16957 | H | single bond | Me | tetrahydropyran-4-yl | C(=O)Me | Cl | Me | O |
| D-16958 | Me | single bond | H | tetrahydropyran-4-yl | C(=O)Me | Cl | Me | O |

**[Table 1032]**

| Compound Number | Y | W | Z | R¹ | R² | R³ | R¹⁰ | A |
|---|---|---|---|---|---|---|---|---|
| D-16959 | Me(R) | single bond | H | tetrahydropyran-4-yl | C(=O)Me | Cl | Me | O |
| D-16960 | Me(S) | single bond | H | tetrahydropyran-4-yl | C(=O)Me | Cl | Me | O |
| D-16961 | H | single bond | Me | tetrahydropyran-4-yl | C(=O)Me | Me | Me | O |
| D-16962 | Me | single bond | H | tetrahydropyran-4-yl | C(=O)Me | Me | Me | O |
| D-16963 | Me(R) | single bond | H | tetrahydropyran-4-yl | C(=O)Me | Me | Me | O |
| D-16964 | Me(S) | single bond | H | tetrahydropyran-4-yl | C(=O)Me | Me | Me | O |
| D-16965 | H | single bond | Me | tetrahydrothiopyran-4-yl | C(=O)Me | Cl | Me | O |
| D-16966 | Me | single bond | H | tetrahydrothiopyran-4-yl | C(=O)Me | Cl | Me | O |
| D-16967 | Me(R) | single bond | H | tetrahydrothiopyran-4-yl | C(=O)Me | Cl | Me | O |
| D-16968 | Me(S) | single bond | H | tetrahydrothiopyran-4-yl | C(=O)Me | Cl | Me | O |
| D-16969 | H | single bond | Me | tetrahydrothiopyran-4-yl | C(=O)Me | Me | Me | O |
| D-16970 | Me | single bond | H | tetrahydrothiopyran-4-yl | C(=O)Me | Me | Me | O |
| D-16971 | Me(R) | single bond | H | tetrahydrothiopyran-4-yl | C(=O)Me | Me | Me | O |
| D-16972 | Me(S) | single bond | H | tetrahydrothiopyran-4-yl | C(=O)Me | Me | Me | O |
| D-16973 | H | single bond | Me | *c*-Hex(1-CN) | C(=O)Me | Cl | Me | O |
| D-16974 | Me | single bond | H | *c*-Hex(1-CN) | C(=O)Me | Cl | Me | O |
| D-16975 | Me(R) | single bond | H | *c*-Hex(1-CN) | C(=O)Me | Cl | Me | O |
| D-16976 | Me(S) | single bond | H | *c*-Hex(1-CN) | C(=O)Me | Cl | Me | O |
| D-16977 | H | single bond | Me | *c*-Hex(1-CN) | C(=O)Me | Me | Me | O |
| D-16978 | Me | single bond | H | *c*-Hex(1-CN) | C(=O)Me | Me | Me | O |
| D-16979 | Me(R) | single bond | H | *c*-Hex(1-CN) | C(=O)Me | Me | Me | O |
| D-16980 | Me(S) | single bond | H | *c*-Hex(1-CN) | C(=O)Me | Me | Me | O |
| D-16981 | H | single bond | Me | tetrahydropyran(4-CN)-4-yl | C(=O)Me | Cl | Me | O |
| D-16982 | Me | single bond | H | tetrahydropyran(4-CN)-4-yl | C(=O)Me | Cl | Me | O |
| D-16983 | Me(R) | single bond | H | tetrahydropyran(4-CN)-4-yl | C(=O)Me | Cl | Me | O |
| D-16984 | Me(S) | single bond | H | tetrahydropyran(4-CN)-4-yl | C(=O)Me | Cl | Me | O |
| D-16985 | H | single bond | Me | tetrahydropyran(4-CN)-4-yl | C(=O)Me | Me | Me | O |
| D-16986 | Me | single bond | H | tetrahydropyran(4-CN)-4-yl | C(=O)Me | Me | Me | O |
| D-16987 | Me(R) | single bond | H | tetrahydropyran(4-CN)-4-yl | C(=O)Me | Me | Me | O |
| D-16988 | Me(S) | single bond | H | tetrahydropyran(4-CN)-4-yl | C(=O)Me | Me | Me | O |
| D-16989 | H | single bond | Me | tetrahydrothiopyran(4-CN)-4-yl | C(=O)Me | Cl | Me | O |
| D-16990 | Me | single bond | H | tetrahydrothiopyran(4-CN)-4-yl | C(=O)Me | Cl | Me | O |
| D-16991 | Me(R) | single bond | H | tetrahydrothiopyran(4-CN)-4-yl | C(=O)Me | Cl | Me | O |
| D-16992 | Me(S) | single bond | H | tetrahydrothiopyran(4-CN)-4-yl | C(=O)Me | Cl | Me | O |
| D-16993 | H | single bond | Me | tetrahydrothiopyran(4-CN)-4-yl | C(=O)Me | Me | Me | O |
| D-16994 | Me | single bond | H | tetrahydrothiopyran(4-CN)-4-yl | C(=O)Me | Me | Me | O |
| D-16995 | Me(R) | single bond | H | tetrahydrothiopyran(4-CN)-4-yl | C(=O)Me | Me | Me | O |
| D-16996 | Me(S) | single bond | H | tetrahydrothiopyran(4-CN)-4-yl | C(=O)Me | Me | Me | O |
| D-16997 | H | single bond | Me | CH₂(*c*-Pr) | C(=O)Me | Cl | Me | O |
| D-16998 | Me | single bond | H | CH₂(*c*-Pr) | C(=O)Me | Cl | Me | O |
| D-16999 | Me(R) | single bond | H | CH₂(*c*-Pr) | C(=O)Me | Cl | Me | O |
| D-17000 | Me(S) | single bond | H | CH₂(*c*-Pr) | C(=O)Me | Cl | Me | O |
| D-17001 | H | single bond | Me | CH₂(*c*-Pr) | C(=O)Me | Me | Me | O |
| D-17002 | Me | single bond | H | CH₂(*c*-Pr) | C(=O)Me | Me | Me | O |

**[Table 1033]**

| Compound Number | Y | W | Z | R¹ | R² | R³ | R¹⁰ | A |
|---|---|---|---|---|---|---|---|---|
| D-17003 | Me(R) | single bond | H | CH₂(*c*-Pr) | C(=O)Me | Me | Me | O |
| D-17004 | Me(S) | single bond | H | CH₂(*c*-Pr) | C(=O)Me | Me | Me | O |
| D-17005 | H | single bond | Me | CH₂[*c*-Pr(1-CN)] | C(=O)Me | Cl | Me | O |
| D-17006 | Me | single bond | H | CH₂[*c*-Pr(1-CN)] | C(=O)Me | Cl | Me | O |
| D-17007 | Me(R) | single bond | H | CH₂[*c*-Pr(1-CN)] | C(=O)Me | Cl | Me | O |
| D-17008 | Me(S) | single bond | H | CH₂[*c*-Pr(1-CN)] | C(=O)Me | Cl | Me | O |
| D-17009 | H | single bond | Me | CH₂[*c*-Pr(1-CN)] | C(=O)Me | Me | Me | O |
| D-17010 | Me | single bond | H | CH₂[*c*-Pr(1-CN)] | C(=O)Me | Me | Me | O |
| D-17011 | Me(R) | single bond | H | CH₂[*c*-Pr(1-CN)] | C(=O)Me | Me | Me | O |
| D-17012 | Me(S) | single bond | H | CH₂[*c*-Pr(1-CN)] | C(=O)Me | Me | Me | O |
| D-17013 | H | single bond | Me | CH(Me)(*c*-Pr) | C(=O)Me | Cl | Me | O |
| D-17014 | Me | single bond | H | CH(Me)(*c*-Pr) | C(=O)Me | Cl | Me | O |
| D-17015 | Me(R) | single bond | H | CH(Me)(*c*-Pr) | C(=O)Me | Cl | Me | O |
| D-17016 | Me(S) | single bond | H | CH(Me)(*c*-Pr) | C(=O)Me | Cl | Me | O |
| D-17017 | H | single bond | Me | CH(Me)(*c*-Pr) | C(=O)Me | Me | Me | O |
| D-17018 | Me | single bond | H | CH(Me)(*c*-Pr) | C(=O)Me | Me | Me | O |
| D-17019 | Me(R) | single bond | H | CH(Me)(*c*-Pr) | C(=O)Me | Me | Me | O |
| D-17020 | Me(S) | single bond | H | CH(Me)(*c*-Pr) | C(=O)Me | Me | Me | O |
| D-17021 | H | single bond | Me | CH(Me)CH₂OMe | C(=O)Me | Cl | Me | O |
| D-17022 | Me | single bond | H | CH(Me)CH₂OMe | C(=O)Me | Cl | Me | O |
| D-17023 | Me(R) | single bond | H | CH(Me)CH₂OMe | C(=O)Me | Cl | Me | O |
| D-17024 | Me(S) | single bond | H | CH(Me)CH₂OMe | C(=O)Me | Cl | Me | O |
| D-17025 | H | single bond | Me | CH(Me)CH₂OMe | C(=O)Me | Me | Me | O |
| D-17026 | Me | single bond | H | CH(Me)CH₂OMe | C(=O)Me | Me | Me | O |
| D-17027 | Me(R) | single bond | H | CH(Me)CH₂OMe | C(=O)Me | Me | Me | O |
| D-17028 | Me(S) | single bond | H | CH(Me)CH₂OMe | C(=O)Me | Me | Me | O |
| D-17029 | H | single bond | Me | CH₂CH=CH₂ | C(=O)Me | Cl | Me | O |
| D-17030 | Me | single bond | H | CH₂CH=CH₂ | C(=O)Me | Cl | Me | O |
| D-17031 | Me(R) | single bond | H | CH₂CH=CH₂ | C(=O)Me | Cl | Me | O |
| D-17032 | Me(S) | single bond | H | CH₂CH=CH₂ | C(=O)Me | Cl | Me | O |
| D-17033 | H | single bond | Me | CH₂CH=CH₂ | C(=O)Me | Me | Me | O |
| D-17034 | Me | single bond | H | CH₂CH=CH₂ | C(=O)Me | Me | Me | O |
| D-17035 | Me(R) | single bond | H | CH₂CH=CH₂ | C(=O)Me | Me | Me | O |
| D-17036 | Me(S) | single bond | H | CH₂CH=CH₂ | C(=O)Me | Me | Me | O |
| D-17037 | H | single bond | Me | CH₂C≡CH | C(=O)Me | Cl | Me | O |
| D-17038 | Me | single bond | H | CH₂C≡CH | C(=O)Me | Cl | Me | O |
| D-17039 | Me(R) | single bond | H | CH₂C≡CH | C(=O)Me | Cl | Me | O |
| D-17040 | Me(S) | single bond | H | CH₂C≡CH | C(=O)Me | Cl | Me | O |
| D-17041 | H | single bond | Me | CH₂C≡CH | C(=O)Me | Me | Me | O |
| D-17042 | Me | single bond | H | CH₂C≡CH | C(=O)Me | Me | Me | O |
| D-17043 | Me(R) | single bond | H | CH₂C≡CH | C(=O)Me | Me | Me | O |
| D-17044 | Me(S) | single bond | H | CH₂C≡CH | C(=O)Me | Me | Me | O |
| D-17045 | H | single bond | Me | CH₂CN | C(=O)Me | Cl | Me | O |
| D-17046 | Me | single bond | H | CH₂CN | C(=O)Me | Cl | Me | O |

**[Table 1034]**

| Compound Number | Y | W | Z | R¹ | R² | R³ | R¹⁰ | A |
|---|---|---|---|---|---|---|---|---|
| D-17047 | Me(R) | single bond | H | CH₂CN | C(=O)Me | Cl | Me | O |
| D-17048 | Me(S) | single bond | H | CH₂CN | C(=O)Me | Cl | Me | O |
| D-17049 | H | single bond | Me | CH₂CN | C(=O)Me | Me | Me | O |
| D-17050 | Me | single bond | H | CH₂CN | C(=O)Me | Me | Me | O |
| D-17051 | Me(R) | single bond | H | CH₂CN | C(=O)Me | Me | Me | O |
| D-17052 | Me(S) | single bond | H | CH₂CN | C(=O)Me | Me | Me | O |
| D-17053 | H | single bond | Me | CH(Me)CN | C(=O)Me | Cl | Me | O |
| D-17054 | Me | single bond | H | CH(Me)CN | C(=O)Me | Cl | Me | O |
| D-17055 | Me(R) | single bond | H | CH(Me)CN | C(=O)Me | Cl | Me | O |
| 0-17056 | Me(S) | single bond | H | CH(Me)CN | C(=O)Me | Cl | Me | O |
| D-17057 | H | single bond | Me | CH(Me)CN | C(=O)Me | Me | Me | O |
| D-17058 | Me | single bond | H | CH(Me)CN | C(=O)Me | Me | Me | O |
| D-17059 | Me(R) | single bond | H | CH(Me)CN | C(=O)Me | Me | Me | O |
| D-17060 | Me(S) | single bond | H | CH(Me)CN | C(=O)Me | Me | Me | O |
| D-17061 | H | single bond | Me | C(Me)₂CN | C(=O)Me | Cl | Me | O |
| D-17062 | Me | single bond | H | C(Me)₂CN | C(=O)Me | Cl | Me | O |
| D-17063 | Me(R) | single bond | H | C(Me)₂CN | C(=O)Me | Cl | Me | O |
| D-17064 | Me(S) | single bond | H | C(Me)₂CN | C(=O)Me | Cl | Me | O |
| D-17065 | H | single bond | Me | C(Me)₂CN | C(=O)Me | Me | Me | O |
| D-17066 | Me | single bond | H | C(Me)₂CN | C(=O)Me | Me | Me | O |
| 0-17067 | Me(R) | single bond | H | C(Me)₂CN | C(=O)Me | Me | Me | O |
| 0-17068 | Me(S) | single bond | H | C(Me)₂CN | C(=O)Me | Me | Me | O |
| D-17069 | H | single bond | Me | C(Me)(*i*-Pr)CN | C(=O)Me | Cl | Me | O |
| D-17070 | Me | single bond | H | C(Me)(*i*-Pr)CN | C(=O)Me | Cl | Me | O |
| D-17071 | Me(R) | single bond | H | C(Me)(*i*-Pr)CN | C(=O)Me | Cl | Me | O |
| D-17072 | Me(S) | single bond | H | C(Me)(*i*-Pr)CN | C(=O)Me | Cl | Me | O |
| D-17073 | H | single bond | Me | C(Me)(*i*-Pr)CN | C(=O)Me | Me | Me | O |
| D-17074 | Me | single bond | H | C(Me)(*i*-Pr)CN | C(=O)Me | Me | Me | O |
| D-17075 | Me(R) | single bond | H | C(Me)(*i*-Pr)CN | C(=O)Me | Me | Me | O |
| 0-17076 | Me(S) | single bond | H | C(Me)(*i*-Pr)CN | C(=O)Me | Me | Me | O |
| D-17077 | H | single bond | Me | OMe | C(=O)Me | Cl | Me | O |
| D-17078 | Me | single bond | H | OMe | C(=O)Me | Cl | Me | O |
| 0-17079 | Me(R) | single bond | H | OMe | C(=O)Me | Cl | Me | O |
| D-17080 | Me(S) | single bond | H | OMe | C(=O)Me | Cl | Me | O |
| D-17081 | H | single bond | Me | OMe | C(=O)Me | Me | Me | O |
| D-17082 | Me | single bond | H | OMe | C(=O)Me | Me | Me | O |
| D-17083 | Me(R) | single bond | H | OMe | C(=O)Me | Me | Me | O |
| D-17084 | Me(S) | single bond | H | OMe | C(=O)Me | Me | Me | O |
| D-17085 | H | single bond | Me | CH=NOMe | C(=O)Me | Cl | Me | O |
| D-17086 | Me | single bond | H | CH=NOMe | C(=O)Me | Cl | Me | O |
| 0-17087 | Me(R) | single bond | H | CH=NOMe | C(=O)Me | Cl | Me | O |
| 0-17088 | Me(S) | single bond | H | CH=NOMe | C(=O)Me | Cl | Me | O |
| D-17089 | H | single bond | Me | CH=NOMe | C(=O)Me | Me | Me | O |
| D-17090 | Me | single bond | H | CH=NOMe | C(=O)Me | Me | Me | O |

**[Table 1035]**

| Compound Number | Y | W | Z | R¹ | R² | R³ | R¹⁰ | A |
|---|---|---|---|---|---|---|---|---|
| D-17091 | Me(R) | single bond | H | CH=NOMe | C(=O)Me | Me | Me | O |
| D-17092 | Me(S) | single bond | H | CH=NOMe | C(=O)Me | Me | Me | O |
| D-17093 | H | single bond | Me | morpholin-4-yl | C(=O)Me | Cl | Me | O |
| D-17094 | Me | single bond | H | morpholin-4-yl | C(=O)Me | Cl | Me | O |
| D-17095 | Me(R) | single bond | H | morpholin-4-yl | C(=O)Me | Cl | Me | O |
| D-17096 | Me(S) | single bond | H | morpholin-4-yl | C(=O)Me | Cl | Me | O |
| D-17097 | H | single bond | Me | morpholin-4-yl | C(=O)Me | Me | Me | O |
| D-17098 | Me | single bond | H | morpholin-4-yl | C(=O)Me | Me | Me | O |
| 0-17099 | Me(R) | single bond | H | morpholin-4-yl | C(=O)Me | Me | Me | O |
| D-17100 | Me(S) | single bond | H | morpholin-4-yl | C(=O)Me | Me | Me | O |
| D-17101 | H | single bond | Me | Ph | C(=O)Me | Cl | Me | O |
| D-17102 | Me | single bond | H | Ph | C(=O)Me | Cl | Me | O |
| D-17103 | Me(R) | single bond | H | Ph | C(=O)Me | Cl | Me | O |
| D-17104 | Me(S) | single bond | H | Ph | C(=O)Me | Cl | Me | O |
| D-17105 | H | single bond | Me | Ph | C(=O)Me | Me | Me | O |
| D-17106 | Me | single bond | H | Ph | C(=O)Me | Me | Me | O |
| D-17107 | Me(R) | single bond | H | Ph | C(=O)Me | Me | Me | O |
| D-17108 | Me(S) | single bond | H | Ph | C(=O)Me | Me | Me | O |
| D-17109 | H | single bond | Me | CH₂Ph | C(=O)Me | Cl | Me | O |
| D-17110 | Me | single bond | H | CH₂Ph | C(=O)Me | Cl | Me | O |
| D-17111 | Me(R) | single bond | H | CH₂Ph | C(=O)Me | Cl | Me | O |
| D-17112 | Me(S) | single bond | H | CH₂Ph | C(=O)Me | Cl | Me | O |
| D-17113 | H | single bond | Me | CH₂Ph | C(=O)Me | Me | Me | O |
| D-17114 | Me | single bond | H | CH₂Ph | C(=O)Me | Me | Me | O |
| D-17115 | Me(R) | single bond | H | CH₂Ph | C(=O)Me | Me | Me | O |
| D-17116 | Me(S) | single bond | H | CH₂Ph | C(=O)Me | Me | Me | O |
| D-17117 | H | single bond | Me | thiazole-2-yl | C(=O)Me | Cl | Me | O |
| D-17118 | Me | single bond | H | thiazole-2-yl | C(=O)Me | Cl | Me | O |
| D-17119 | Me(R) | single bond | H | thiazole-2-yl | C(=O)Me | Cl | Me | O |
| D-17120 | Me(S) | single bond | H | thiazole-2-yl | C(=O)Me | Cl | Me | O |
| D-17121 | H | single bond | Me | thiazole-2-yl | C(=O)Me | Me | Me | O |
| D-17122 | Me | single bond | H | thiazole-2-yl | C(=O)Me | Me | Me | O |
| D-17123 | Me(R) | single bond | H | thiazole-2-yl | C(=O)Me | Me | Me | O |
| D-17124 | Me(S) | single bond | H | thiazole-2-yl | C(=O)Me | Me | Me | O |
| D-17125 | H | single bond | Me | 1-Me-pyrazol-3-yl | C(=O)Me | Cl | Me | O |
| D-17126 | Me | single bond | H | 1-Me-pyrazol-3-yl | C(=O)Me | Cl | Me | O |
| D-17127 | Me(R) | single bond | H | 1-Me-pyrazol-3-yl | C(=O)Me | Cl | Me | O |
| D-17128 | Me(S) | single bond | H | 1-Me-pyrazol-3-yl | C(=O)Me | Cl | Me | O |
| D-17129 | H | single bond | Me | 1-Me-pyrazol-3-yl | C(=O)Me | Me | Me | O |
| D-17130 | Me | single bond | H | 1-Me-pyrazol-3-yl | C(=O)Me | Me | Me | O |
| D-17131 | Me(R) | single bond | H | 1-Me-pyrazol-3-yl | C(=O)Me | Me | Me | O |
| D-17132 | Me(S) | single bond | H | 1-Me-pyrazol-3-yl | C(=O)Me | Me | Me | O |
| D-17133 | H | single bond | Me | 1-Me-pyrazol-5-yl | C(=O)Me | Cl | Me | O |
| D-17134 | Me | single bond | H | 1-Me-pyrazol-5-yl | C(=O)Me | Cl | Me | O |

**[Table 1036]**

| Compound Number | Y | W | Z | R¹ | R² | R³ | R¹⁰ | A |
|---|---|---|---|---|---|---|---|---|
| D-17135 | Me(R) | single bond | H | 1-Me-pyrazol-5-yl | C(=O)Me | Cl | Me | O |
| D-17136 | Me(S) | single bond | H | 1-Me-pyrazol-5-yl | C(=O)Me | Cl | Me | O |
| D-17137 | H | single bond | Me | 1-Me-pyrazol-5-yl | C(=O)Me | Me | Me | O |
| D-17138 | Me | single bond | H | 1-Me-pyrazol-5-yl | C(=O)Me | Me | Me | O |
| D-17139 | Me(R) | single bond | H | 1-Me-pyrazol-5-yl | C(=O)Me | Me | Me | O |
| D-17140 | Me(S) | single bond | H | 1-Me-pyrazol-5-yl | C(=O)Me | Me | Me | O |
| D-17141 | H | single bond | Me | CH₂(pyrimidin-2-yl) | C(=O)Me | Cl | Me | O |
| D-17142 | Me | single bond | H | CH₂(pyrimidin-2-yl) | C(=O)Me | Cl | Me | O |
| D-17143 | Me(R) | single bond | H | CH₂(pyrimidin-2-yl) | C(=O)Me | Cl | Me | O |
| D-17144 | Me(S) | single bond | H | CH₂(pyrimidin-2-yl) | C(=O)Me | Cl | Me | O |
| D-17145 | H | single bond | Me | CH₂(pyrimidin-2-yl) | C(=O)Me | Me | Me | O |
| D-17146 | Me | single bond | H | CH₂(pyrimidin-2-yl) | C(=O)Me | Me | Me | O |
| D-17147 | Me(R) | single bond | H | CH₂(pyrimidin-2-yl) | C(=O)Me | Me | Me | O |
| D-17148 | Me(S) | single bond | H | CH₂(pyrimidin-2-yl) | C(=O)Me | Me | Me | O |
| D-17149 | H | single bond | Me | Me | C(=O)Me | Cl | CH₂OMe | O |
| D-17150 | Me | single bond | H | Me | C(=O)Me | Cl | CH₂OMe | O |
| D-17151 | Me(R) | single bond | H | Me | C(=O)Me | Cl | CH₂OMe | O |
| D-17152 | Me(S) | single bond | H | Me | C(=O)Me | Cl | CH₂OMe | O |
| D-17153 | H | single bond | Me | Me | C(=O)Me | Me | CH₂OMe | O |
| D-17154 | Me | single bond | H | Me | C(=O)Me | Me | CH₂OMe | O |
| D-17155 | Me(R) | single bond | H | Me | C(=O)Me | Me | CH₂OMe | O |
| D-17156 | Me(S) | single bond | H | Me | C(=O)Me | Me | CH₂OMe | O |
| D-17157 | H | single bond | Me | CH₂CHF₂ | C(=O)Me | Cl | CH₂OMe | O |
| D-17158 | Me | single bond | H | CH₂CHF₂ | C(=O)Me | Cl | CH₂OMe | O |
| D-17159 | Me(R) | single bond | H | CH₂CHF₂ | C(=O)Me | Cl | CH₂OMe | O |
| D-17160 | Me(S) | single bond | H | CH₂CHF₂ | C(=O)Me | Cl | CH₂OMe | O |
| D-17161 | H | single bond | Me | CH₂CHF₂ | C(=O)Me | Me | CH₂OMe | O |
| D-17162 | Me | single bond | H | CH₂CHF₂ | C(=O)Me | Me | CH₂OMe | O |
| D-17163 | Me(R) | single bond | H | CH₂CHF₂ | C(=O)Me | Me | CH₂OMe | O |
| D-17164 | Me(S) | single bond | H | CH₂CHF₂ | C(=O)Me | Me | CH₂OMe | O |
| D-17165 | H | single bond | Me | *n*-Pr | C(=O)Me | Cl | CH₂OMe | O |
| D-17166 | Me | single bond | H | *n*-Pr | C(=O)Me | Cl | CH₂OMe | O |
| D-17167 | Me(R) | single bond | H | *n*-Pr | C(=O)Me | Cl | CH₂OMe | O |
| D-17168 | Me(S) | single bond | H | *n*-Pr | C(=O)Me | Cl | CH₂OMe | O |
| D-17169 | H | single bond | Me | *n*-Pr | C(=O)Me | Me | CH₂OMe | O |
| D-17170 | Me | single bond | H | *n*-Pr | C(=O)Me | Me | CH₂OMe | O |
| D-17171 | Me(R) | single bond | H | *n*-Pr | C(=O)Me | Me | CH₂OMe | O |
| D-17172 | Me(S) | single bond | H | *n*-Pr | C(=O)Me | Me | CH₂OMe | O |
| D-17173 | H | single bond | Me | *i*-Pr | C(=O)Me | Cl | CH₂OMe | O |
| D-17174 | Me | single bond | H | *i*-Pr | C(=O)Me | Cl | CH₂OMe | O |
| D-17175 | Me(R) | single bond | H | *i*-Pr | C(=O)Me | Cl | CH₂OMe | O |
| D-17176 | Me(S) | single bond | H | *i*-Pr | C(=O)Me | Cl | CH₂OMe | O |
| D-17177 | H | single bond | Me | *i*-Pr | C(=O)Me | Me | CH₂OMe | O |
| D-17178 | Me | single bond | H | *i*-Pr | C(=O)Me | Me | CH₂OMe | O |

**[Table 1037]**

| Compound Number | Y | W | Z | R¹ | R² | R³ | R¹⁰ | A |
|---|---|---|---|---|---|---|---|---|
| D-17179 | Me(R) | single bond | H | *i*-Pr | C(=O)Me | Me | CH₂OMe | O |
| D-17180 | Me(S) | single bond | H | *i*-Pr | C(=O)Me | Me | CH₂OMe | O |
| D-17181 | H | single bond | Me | *c*-Pr(1-F) | C(=O)Me | Cl | CH₂OMe | O |
| D-17182 | Me | single bond | H | *c*-Pr(1-F) | C(=O)Me | Cl | CH₂OMe | O |
| D-17183 | Me(R) | single bond | H | *c*-Pr(1-F) | C(=O)Me | Cl | CH₂OMe | O |
| D-17184 | Me(S) | single bond | H | *c*-Pr(1-F) | C(=O)Me | Cl | CH₂OMe | O |
| D-17185 | H | single bond | Me | *c*-Pr(1-F) | C(=O)Me | Me | CH₂OMe | O |
| D-17186 | Me | single bond | H | *c*-Pr(1-F) | C(=O)Me | Me | CH₂OMe | O |
| D-17187 | Me(R) | single bond | H | *c*-Pr(1-F) | C(=O)Me | Me | CH₂OMe | O |
| D-17188 | Me(S) | single bond | H | *c*-Pr(1-F) | C(=O)Me | Me | CH₂OMe | O |
| D-17189 | H | single bond | Me | *c*-Pr(2,2-F₂) | C(=O)Me | Cl | CH₂OMe | O |
| D-17190 | Me | single bond | H | *c*-Pr(2,2-F₂) | C(=O)Me | Cl | CH₂OMe | O |
| D-17191 | Me(R) | single bond | H | *c*-Pr(2,2-F₂) | C(=O)Me | Cl | CH₂OMe | O |
| D-17192 | Me(S) | single bond | H | *c*-Pr(2,2-F₂) | C(=O)Me | Cl | CH₂OMe | O |
| D-17193 | H | single bond | Me | *c*-Pr(2,2-F₂) | C(=O)Me | Me | CH₂OMe | O |
| D-17194 | Me | single bond | H | *c*-Pr(2,2-F₂) | C(=O)Me | Me | CH₂OMe | O |
| D-17195 | Me(R) | single bond | H | *c*-Pr(2,2-F₂) | C(=O)Me | Me | CH₂OMe | O |
| D-17196 | Me(S) | single bond | H | *c*-Pr(2,2-F₂) | C(=O)Me | Me | CH₂OMe | O |
| D-17197 | H | single bond | Me | *c*-Pr(1-Cl) | C(=O)Me | Cl | CH₂OMe | O |
| D-17198 | Me | single bond | H | *c*-Pr(1-Cl) | C(=O)Me | Cl | CH₂OMe | O |
| D-17199 | Me(R) | single bond | H | *c*-Pr(1-Cl) | C(=O)Me | Cl | CH₂OMe | O |
| D-17200 | Me(S) | single bond | H | *c*-Pr(1-Cl) | C(=O)Me | Cl | CH₂OMe | O |
| D-17201 | H | single bond | Me | *c*-Pr(1-Cl) | C(=O)Me | Me | CH₂OMe | O |
| D-17202 | Me | single bond | H | *c*-Pr(1-Cl) | C(=O)Me | Me | CH₂OMe | O |
| D-17203 | Me(R) | single bond | H | *c*-Pr(1-Cl) | C(=O)Me | Me | CH₂OMe | O |
| D-17204 | Me(S) | single bond | H | *c*-Pr(1-Cl) | C(=O)Me | Me | CH₂OMe | O |
| D-17205 | H | single bond | Me | *c*-Pr(1-Br) | C(=O)Me | Cl | CH₂OMe | O |
| D-17206 | Me | single bond | H | *c*-Pr(1-Br) | C(=O)Me | Cl | CH₂OMe | O |
| D-17207 | Me(R) | single bond | H | *c*-Pr(1-Br) | C(=O)Me | Cl | CH₂OMe | O |
| D-17208 | Me(S) | single bond | H | *c*-Pr(1-Br) | C(=O)Me | Cl | CH₂OMe | O |
| D-17209 | H | single bond | Me | *c*-Pr(1-Br) | C(=O)Me | Me | CH₂OMe | O |
| D-17210 | Me | single bond | H | *c*-Pr(1-Br) | C(=O)Me | Me | CH₂OMe | O |
| D-17211 | Me(R) | single bond | H | *c*-Pr(1-Br) | C(=O)Me | Me | CH₂OMe | O |
| D-17212 | Me(S) | single bond | H | *c*-Pr(1-Br) | C(=O)Me | Me | CH₂OMe | O |
| D-17213 | H | single bond | Me | *c*-Pr(1-Me) | C(=O)Me | Cl | CH₂OMe | O |
| D-17214 | Me | single bond | H | *c*-Pr(1-Me) | C(=O)Me | Cl | CH₂OMe | O |
| D-17215 | Me(R) | single bond | H | *c*-Pr(1-Me) | C(=O)Me | Cl | CH₂OMe | O |
| D-17216 | Me(S) | single bond | H | *c*-Pr(1-Me) | C(=O)Me | Cl | CH₂OMe | O |
| D-17217 | H | single bond | Me | *c*-Pr(1-Me) | C(=O)Me | Me | CH₂OMe | O |
| D-17218 | Me | single bond | H | *c*-Pr(1-Me) | C(=O)Me | Me | CH₂OMe | O |
| D-17219 | Me(R) | single bond | H | *c*-Pr(1-Me) | C(=O)Me | Me | CH₂OMe | O |
| D-17220 | Me(S) | single bond | H | *c*-Pr(1-Me) | C(=O)Me | Me | CH₂OMe | O |
| D-17221 | H | single bond | Me | *c*-Pr(1-CH₂OH) | C(=O)Me | Cl | CH₂OMe | O |
| D-17222 | Me | single bond | H | *c*-Pr(1-CH₂OH) | C(=O)Me | Cl | CH₂OMe | O |

**[Table 1038]**

| Compound Number | Y | W | Z | R¹ | R² | R³ | R¹⁰ | A |
|---|---|---|---|---|---|---|---|---|
| D-17223 | Me(R) | single bond | H | *c*-Pr(1-CH₂OH) | C(=O)Me | Cl | CH₂OMe | O |
| D-17224 | Me(S) | single bond | H | *c*-Pr(1-CH₂OH) | C(=O)Me | Cl | CH₂OMe | O |
| D-17225 | H | single bond | Me | *c*-Pr(1-CH₂OH) | C(=O)Me | Me | CH₂OMe | O |
| D-17226 | Me | single bond | H | *c*-Pr(1-CH₂OH) | C(=O)Me | Me | CH₂OMe | O |
| D-17227 | Me(R) | single bond | H | *c*-Pr(1-CH₂OH) | C(=O)Me | Me | CH₂OMe | O |
| D-17228 | Me(S) | single bond | H | *c*-Pr(1-CH₂OH) | C(=O)Me | Me | CH₂OMe | O |
| D-17229 | H | single bond | Me | *c*-Pr[1-CH₂OS(=O)₂Me] | C(=O)Me | Cl | CH₂OMe | O |
| D-17230 | Me | single bond | H | *c*-Pr[1-CH₂OS(=O)₂Me] | C(=O)Me | Cl | CH₂OMe | O |
| D-17231 | Me(R) | single bond | H | *c*-Pr[1-CH₂OS(=O)₂Me] | C(=O)Me | Cl | CH₂OMe | O |
| D-17232 | Me(S) | single bond | H | *c*-Pr[1-CH₂OS(=O)₂Me] | C(=O)Me | Cl | CH₂OMe | O |
| D-17233 | H | single bond | Me | *c*-Pr[1-CH₂OS(=O)₂Me] | C(=O)Me | Me | CH₂OMe | O |
| D-17234 | Me | single bond | H | *c*-Pr[1-CH₂OS(=O)₂Me] | C(=O)Me | Me | CH₂OMe | O |
| D-17235 | Me(R) | single bond | H | *c*-Pr[1-CH₂OS(=O)₂Me] | C(=O)Me | Me | CH₂OMe | O |
| D-17236 | Me(S) | single bond | H | *c*-Pr[1-CH₂OS(=O)₂Me] | C(=O)Me | Me | CH₂OMe | O |
| D-17237 | H | single bond | Me | *c*-Pr(1-CH₂OMe) | C(=O)Me | Cl | CH₂OMe | O |
| D-17238 | Me | single bond | H | *c*-Pr(1-CH₂OMe) | C(=O)Me | Cl | CH₂OMe | O |
| D-17239 | Me(R) | single bond | H | *c*-Pr(1-CH₂OMe) | C(=O)Me | Cl | CH₂OMe | O |
| D-17240 | Me(S) | single bond | H | *c*-Pr(1-CH₂OMe) | C(=O)Me | Cl | CH₂OMe | O |
| D-17241 | H | single bond | Me | *c*-Pr(1-CH₂OMe) | C(=O)Me | Me | CH₂OMe | O |
| D-17242 | Me | single bond | H | *c*-Pr(1-CH₂OMe) | C(=O)Me | Me | CH₂OMe | O |
| D-17243 | Me(R) | single bond | H | *c*-Pr(1-CH₂OMe) | C(=O)Me | Me | CH₂OMe | O |
| D-17244 | Me(S) | single bond | H | *c*-Pr(1-CH₂OMe) | C(=O)Me | Me | CH₂OMe | O |
| D-17245 | H | single bond | Me | *c*-Pr(1-CH₂OCN) | C(=O)Me | Cl | CH₂OMe | O |
| D-17246 | Me | single bond | H | *c*-Pr(1-CH₂OCN) | C(=O)Me | Cl | CH₂OMe | O |
| D-17247 | Me(R) | single bond | H | *c*-Pr(1-CH₂OCN) | C(=O)Me | Cl | CH₂OMe | O |
| D-17248 | Me(S) | single bond | H | *c*-Pr(1-CH₂OCN) | C(=O)Me | Cl | CH₂OMe | O |
| D-17249 | H | single bond | Me | *c*-Pr(1-CH₂OCN) | C(=O)Me | Me | CH₂OMe | O |
| D-17250 | Me | single bond | H | *c*-Pr(1-CH₂OCN) | C(=O)Me | Me | CH₂OMe | O |
| D-17251 | Me(R) | single bond | H | *c*-Pr(1-CH₂OCN) | C(=O)Me | Me | CH₂OMe | O |
| D-17252 | Me(S) | single bond | H | *c*-Pr(1-CH₂OCN) | C(=O)Me | Me | CH₂OMe | O |
| D-17253 | H | single bond | Me | *c*-Pr(1-CH₂SMe) | C(=O)Me | Cl | CH₂OMe | O |
| D-17254 | Me | single bond | H | *c*-Pr(1-CH₂SMe) | C(=O)Me | Cl | CH₂OMe | O |
| D-17255 | Me(R) | single bond | H | *c*-Pr(1-CH₂SMe) | C(=O)Me | Cl | CH₂OMe | O |
| D-17256 | Me(S) | single bond | H | *c*-Pr(1-CH₂SMe) | C(=O)Me | Cl | CH₂OMe | O |
| D-17257 | H | single bond | Me | *c*-Pr(1-CH₂SMe) | C(=O)Me | Me | CH₂OMe | O |
| D-17258 | Me | single bond | H | *c*-Pr(1-CH₂SMe) | C(=O)Me | Me | CH₂OMe | O |
| D-17259 | Me(R) | single bond | H | *c*-Pr(1-CH₂SMe) | C(=O)Me | Me | CH₂OMe | O |
| D-17260 | Me(S) | single bond | H | *c*-Pr(1-CH₂SMe) | C(=O)Me | Me | CH₂OMe | O |
| D-17261 | H | single bond | Me | *c*-Pr[1-CH₂S(=O)Me] | C(=O)Me | Cl | CH₂OMe | O |
| D-17262 | Me | single bond | H | *c*-Pr[1-CH₂S(=O)Me] | C(=O)Me | Cl | CH₂OMe | O |
| D-17263 | Me(R) | single bond | H | *c*-Pr[1-CH₂S(=O)Me] | C(=O)Me | Cl | CH₂OMe | O |
| D-17264 | Me(S) | single bond | H | *c*-Pr[1-CH₂S(=O)Me] | C(=O)Me | Cl | CH₂OMe | O |
| D-17265 | H | single bond | Me | *c*-Pr[1-CH₂S(=O)Me] | C(=O)Me | Me | CH₂OMe | O |
| D-17266 | Me | single bond | H | *c*-Pr[1-CH₂S(=O)Me] | C(=O)Me | Me | CH₂OMe | O |

**[Table 1039]**

| Compound Number | Y | W | Z | R¹ | R² | R³ | R¹⁰ | A |
|---|---|---|---|---|---|---|---|---|
| D-17267 | Me(R) | single bond | H | *c*-Pr[1-CH₂S(=O)Me] | C(=O)Me | Me | CH₂OMe | O |
| D-17268 | Me(S) | single bond | H | *c*-Pr[1-CH₂S(=O)Me] | C(=O)Me | Me | CH₂OMe | O |
| D-17269 | H | single bond | Me | *c*-Pr[1-CH₂S(=O)₂Me] | C(=O)Me | Cl | CH₂OMe | O |
| D-17270 | Me | single bond | H | *c*-Pr[1-CH₂S(=O)₂Me] | C(=O)Me | Cl | CH₂OMe | O |
| D-17271 | Me(R) | single bond | H | *c*-Pr[1-CH₂S(=O)₂Me] | C(=O)Me | Cl | CH₂OMe | O |
| D-17272 | Me(S) | single bond | H | *c*-Pr[1-CH₂S(=O)₂Me] | C(=O)Me | Cl | CH₂OMe | O |
| D-17273 | H | single bond | Me | *c*-Pr[1-CH₂S(=O)₂Me] | C(=O)Me | Me | CH₂OMe | O |
| D-17274 | Me | single bond | H | *c*-Pr[1-CH₂S(=O)₂Me] | C(=O)Me | Me | CH₂OMe | O |
| D-17275 | Me(R) | single bond | H | *c*-Pr[1-CH₂S(=O)₂Me] | C(=O)Me | Me | CH₂OMe | O |
| D-17276 | Me(S) | single bond | H | *c*-Pr[1-CH₂S(=O)₂Me] | C(=O)Me | Me | CH₂OMe | O |
| D-17277 | H | single bond | Me | *c*-Pr(1-CH₂F) | C(=O)Me | Cl | CH₂OMe | O |
| D-17278 | Me | single bond | H | *c*-Pr(1-CH₂F) | C(=O)Me | Cl | CH₂OMe | O |
| D-17279 | Me(R) | single bond | H | *c*-Pr(1-CH₂F) | C(=O)Me | Cl | CH₂OMe | O |
| D-17280 | Me(S) | single bond | H | *c*-Pr(1-CH₂F) | C(=O)Me | Cl | CH₂OMe | O |
| D-17281 | H | single bond | Me | *c*-Pr(1-CH₂F) | C(=O)Me | Me | CH₂OMe | O |
| D-17282 | Me | single bond | H | *c*-Pr(1-CH₂F) | C(=O)Me | Me | CH₂OMe | O |
| D-17283 | Me(R) | single bond | H | *c*-Pr(1-CH₂F) | C(=O)Me | Me | CH₂OMe | O |
| D-17284 | Me(S) | single bond | H | *c*-Pr(1-CH₂F) | C(=O)Me | Me | CH₂OMe | O |
| D-17285 | H | single bond | Me | *c*-Pr(1-CHF₂) | C(=O)Me | Cl | CH₂OMe | O |
| D-17286 | Me | single bond | H | *c*-Pr(1-CHF₂) | C(=O)Me | Cl | CH₂OMe | O |
| D-17287 | Me(R) | single bond | H | *c*-Pr(1-CHF₂) | C(=O)Me | Cl | CH₂OMe | O |
| D-17288 | Me(S) | single bond | H | *c*-Pr(1-CHF₂) | C(=O)Me | Cl | CH₂OMe | O |
| D-17289 | H | single bond | Me | *c*-Pr(1-CHF₂) | C(=O)Me | Me | CH₂OMe | O |
| D-17290 | Me | single bond | H | *c*-Pr(1-CHF₂) | C(=O)Me | Me | CH₂OMe | O |
| D-17291 | Me(R) | single bond | H | *c*-Pr(1-CHF₂) | C(=O)Me | Me | CH₂OMe | O |
| D-17292 | Me(S) | single bond | H | *c*-Pr(1-CHF₂) | C(=O)Me | Me | CH₂OMe | O |
| D-17293 | H | single bond | Me | *c*-Pr(1-CF₃) | C(=O)Me | Cl | CH₂OMe | O |
| D-17294 | Me | single bond | H | *c*-Pr(1-CF₃) | C(=O)Me | Cl | CH₂OMe | O |
| D-17295 | Me(R) | single bond | H | *c*-Pr(1-CF₃) | C(=O)Me | Cl | CH₂OMe | O |
| D-17296 | Me(S) | single bond | H | *c*-Pr(1-CF₃) | C(=O)Me | Cl | CH₂OMe | O |
| D-17297 | H | single bond | Me | *c*-Pr(1-CF₃) | C(=O)Me | Me | CH₂OMe | O |
| D-17298 | Me | single bond | H | *c*-Pr(1-CF₃) | C(=O)Me | Me | CH₂OMe | O |
| D-17299 | Me(R) | single bond | H | *c*-Pr(1-CF₃) | C(=O)Me | Me | CH₂OMe | O |
| D-17300 | Me(S) | single bond | H | *c*-Pr(1-CF₃) | C(=O)Me | Me | CH₂OMe | O |
| D-17301 | H | single bond | Me | *c*-Pr(1-C≡CH) | C(=O)Me | Cl | CH₂OMe | O |
| D-17302 | Me | single bond | H | *c*-Pr(1-C≡CH) | C(=O)Me | Cl | CH₂OMe | O |
| D-17303 | Me(R) | single bond | H | *c*-Pr(1-C≡CH) | C(=O)Me | Cl | CH₂OMe | O |
| D-17304 | Me(S) | single bond | H | *c*-Pr(1-C≡CH) | C(=O)Me | Cl | CH₂OMe | O |
| D-17305 | H | single bond | Me | *c*-Pr(1-C≡CH) | C(=O)Me | Me | CH₂OMe | O |
| D-17306 | Me | single bond | H | *c*-Pr(1-C≡CH) | C(=O)Me | Me | CH₂OMe | O |
| D-17307 | Me(R) | single bond | H | *c*-Pr(1-C≡CH) | C(=O)Me | Me | CH₂OMe | O |
| D-17308 | Me(S) | single bond | H | *c*-Pr(1-C≡CH) | C(=O)Me | Me | CH₂OMe | O |
| D-17309 | H | single bond | Me | *c*-Pr[1-C(=O)OEt] | C(=O)Me | Cl | CH₂OMe | O |
| D-17310 | Me | single bond | H | *c*-Pr[1-C(=O)OEt] | C(=O)Me | Cl | CH₂OMe | O |

**[Table 1040]**

| Compound Number | Y | W | Z | R¹ | R² | R³ | R¹⁰ | A |
|---|---|---|---|---|---|---|---|---|
| D-17311 | Me(R) | single bond | H | *c*-Pr[1-C(=O)OEt] | C(=O)Me | Cl | CH₂OMe | O |
| D-17312 | Me(S) | single bond | H | *c*-Pr[1-C(=O)OEt] | C(=O)Me | Cl | CH₂OMe | O |
| D-17313 | H | single bond | Me | *c*-Pr[1-C(=O)OEt] | C(=O)Me | Me | CH₂OMe | O |
| D-17314 | Me | single bond | H | *c*-Pr[1-C(=O)OEt] | C(=O)Me | Me | CH₂OMe | O |
| D-17315 | Me(R) | single bond | H | *c*-Pr[1-C(=O)OEt] | C(=O)Me | Me | CH₂OMe | O |
| D-17316 | Me(S) | single bond | H | *c*-Pr[1-C(=O)Et] | C(=O)Me | Me | CH₂OMe | O |
| D-17317 | H | single bond | Me | *c*-Pr[1-C(=O)OH] | C(=O)Me | Cl | CH₂OMe | O |
| D-17318 | Me | single bond | H | *c*-Pr[1-C(=O)OH] | C(=O)Me | Cl | CH₂OMe | O |
| D-17319 | Me(R) | single bond | H | *c*-Pr[1-C(=O)OH] | C(=O)Me | Cl | CH₂OMe | O |
| D-17320 | Me(S) | single bond | H | *c*-Pr[1-C(=O)OH] | C(=O)Me | Cl | CH₂OMe | O |
| D-17321 | H | single bond | Me | *c*-Pr[1-C(=O)OH] | C(=O)Me | Me | CH₂OMe | O |
| D-17322 | Me | single bond | H | *c*-Pr[1-C(=O)OH] | C(=O)Me | Me | CH₂OMe | O |
| D-17323 | Me(R) | single bond | H | *c*-Pr[1-C(=O)OH] | C(=O)Me | Me | CH₂OMe | O |
| D-17324 | Me(S) | single bond | H | *c*-Pr[1-C(=O)OH] | C(=O)Me | Me | CH₂OMe | O |
| D-17325 | H | single bond | Me | *c*-Pr[1-C(=O)NH₂] | C(=O)Me | Cl | CH₂OMe | O |
| D-17326 | Me | single bond | H | *c*-Pr[1-C(=O)NH₂] | C(=O)Me | Cl | CH₂OMe | O |
| D-17327 | Me(R) | single bond | H | *c*-Pr[1-C(=O)NH₂] | C(=O)Me | Cl | CH₂OMe | O |
| D-17328 | Me(S) | single bond | H | *c*-Pr[1-C(=O)NH₂] | C(=O)Me | Cl | CH₂OMe | O |
| D-17329 | H | single bond | Me | *c*-Pr[1-C(=O)NH₂] | C(=O)Me | Me | CH₂OMe | O |
| D-17330 | Me | single bond | H | *c*-Pr[1-C(=O)NH₂] | C(=O)Me | Me | CH₂OMe | O |
| D-17331 | Me(R) | single bond | H | *c*-Pr[1-C(=O)NH₂] | C(=O)Me | Me | CH₂OMe | O |
| D-17332 | Me(S) | single bond | H | *c*-Pr[1-C(=O)NH₂] | C(=O)Me | Me | CH₂OMe | O |
| D-17333 | H | single bond | Me | c-Pr(1-OMe) | C(=O)Me | Cl | CH₂OMe | O |
| D-17334 | Me | single bond | H | c-Pr(1-OMe) | C(=O)Me | Cl | CH₂OMe | O |
| D-17335 | Me(R) | single bond | H | c-Pr(1-OMe) | C(=O)Me | Cl | CH₂OMe | O |
| D-17336 | Me(S) | single bond | H | c-Pr(1-OMe) | C(=O)Me | Cl | CH₂OMe | O |
| D-17337 | H | single bond | Me | c-Pr(1-OMe) | C(=O)Me | Me | CH₂OMe | O |
| D-17338 | Me | single bond | H | c-Pr(1-OMe) | C(=O)Me | Me | CH₂OMe | O |
| D-17339 | Me(R) | single bond | H | c-Pr(1-OMe) | C(=O)Me | Me | CH₂OMe | O |
| D-17340 | Me(S) | single bond | H | c-Pr(1-OMe) | C(=O)Me | Me | CH₂OMe | O |
| D-17341 | H | single bond | Me | c-Pr(1-SMe) | C(=O)Me | Cl | CH₂OMe | O |
| D-17342 | Me | single bond | H | c-Pr(1-SMe) | C(=O)Me | Cl | CH₂OMe | O |
| D-17343 | Me(R) | single bond | H | c-Pr(1-SMe) | C(=O)Me | Cl | CH₂OMe | O |
| D-17344 | Me(S) | single bond | H | c-Pr(1-SMe) | C(=O)Me | Cl | CH₂OMe | O |
| D-17345 | H | single bond | Me | c-Pr(1-SMe) | C(=O)Me | Me | CH₂OMe | O |
| D-17346 | Me | single bond | H | c-Pr(1-SMe) | C(=O)Me | Me | CH₂OMe | O |
| D-17347 | Me(R) | single bond | H | c-Pr(1-SMe) | C(=O)Me | Me | CH₂OMe | O |
| D-17348 | Me(S) | single bond | H | c-Pr(1-SMe) | C(=O)Me | Me | CH₂OMe | O |
| D-17349 | H | single bond | Me | *c*-Pr[1-S(=O)Me] | C(=O)Me | Cl | CH₂OMe | O |
| D-17350 | Me | single bond | H | *c*-Pr[1-S(=O)Me] | C(=O)Me | Cl | CH₂OMe | O |
| D-17351 | Me(R) | single bond | H | *c*-Pr[1-S(=O)Me] | C(=O)Me | Cl | CH₂OMe | O |
| D-17352 | Me(S) | single bond | H | *c*-Pr[1-S(=O)Me] | C(=O)Me | Cl | CH₂OMe | O |
| D-17353 | H | single bond | Me | *c*-Pr[1-S(=O)Me] | C(=O)Me | Me | CH₂OMe | O |
| D-17354 | Me | single bond | H | *c*-Pr[1-S(=O)Me] | C(=O)Me | Me | CH₂OMe | O |

**[Table 1041]**

| Compound Number | Y | W | Z | R¹ | R² | R³ | R¹⁰ | A |
|---|---|---|---|---|---|---|---|---|
| D-17355 | Me(R) | single bond | H | *c*-Pr[1-S(=O)Me] | C(=O)Me | Me | CH₂OMe | O |
| D-17356 | Me(S) | single bond | H | *c*-Pr[1-S(=O)Me] | C(=O)Me | Me | CH₂OMe | O |
| D-17357 | H | single bond | Me | *c*-Pr[1-S(=O)₂Me] | C(=O)Me | Cl | CH₂OMe | O |
| D-17358 | Me | single bond | H | *c*-Pr[1-S(=O)₂Me] | C(=O)Me | Cl | CH₂OMe | O |
| D-17359 | Me(R) | single bond | H | *c*-Pr[1-S(=O)₂Me] | C(=O)Me | Cl | CH₂OMe | O |
| D-17360 | Me(S) | single bond | H | *c*-Pr[1-S(=O)₂Me] | C(=O)Me | Cl | CH₂OMe | O |
| D-17361 | H | single bond | Me | *c*-Pr[1-S(=O)₂Me] | C(=O)Me | Me | CH₂OMe | O |
| D-17362 | Me | single bond | H | *c*-Pr[1-S(=O)₂Me] | C(=O)Me | Me | CH₂OMe | O |
| D-17363 | Me(R) | single bond | H | *c*-Pr[1-S(=O)₂Me] | C(=O)Me | Me | CH₂OMe | O |
| D-17364 | Me(S) | single bond | H | *c*-Pr[1-S(=O)₂Me] | C(=O)Me | Me | CH₂OMe | O |
| D-17365 | H | single bond | Me | *c*-Pr[1-S(=O)₂CHF₂] | C(=O)Me | Cl | CH₂OMe | O |
| D-17366 | Me | single bond | H | *c*-Pr[1-S(=O)₂CHF₂] | C(=O)Me | Cl | CH₂OMe | O |
| D-17367 | Me(R) | single bond | H | *c*-Pr[1-S(=O)₂CHF₂] | C(=O)Me | Cl | CH₂OMe | O |
| D-17368 | Me(S) | single bond | H | *c*-Pr[1-S(=O)₂CHF₂] | C(=O)Me | Cl | CH₂OMe | O |
| D-17369 | H | single bond | Me | *c*-Pr[1-S(=O)₂CHF₂] | C(=O)Me | Me | CH₂OMe | O |
| D-17370 | Me | single bond | H | *c*-Pr[1-S(=O)₂CHF₂] | C(=O)Me | Me | CH₂OMe | O |
| D-17371 | Me(R) | single bond | H | *c*-Pr[1-S(=O)₂CHF₂] | C(=O)Me | Me | CH₂OMe | O |
| D-17372 | Me(S) | single bond | H | *c*-Pr[1-S(=O)₂CHF₂] | C(=O)Me | Me | CH₂OMe | O |
| D-17373 | H | single bond | Me | *c*-Pr[1-S(=O)₂CF₃] | C(=O)Me | Cl | CH₂OMe | O |
| D-17374 | Me | single bond | H | *c*-Pr[1-S(=O)₂CF₃] | C(=O)Me | Cl | CH₂OMe | O |
| D-17375 | Me(R) | single bond | H | *c*-Pr[1-S(=O)₂CF₃] | C(=O)Me | Cl | CH₂OMe | O |
| D-17376 | Me(S) | single bond | H | *c*-Pr[1-S(=O)₂CF₃] | C(=O)Me | Cl | CH₂OMe | O |
| D-17377 | H | single bond | Me | *c*-Pr[1-S(=O)₂CF₃] | C(=O)Me | Me | CH₂OMe | O |
| D-17378 | Me | single bond | H | *c*-Pr[1-S(=O)₂CF₃] | C(=O)Me | Me | CH₂OMe | O |
| D-17379 | Me(R) | single bond | H | *c*-Pr[1-S(=O)₂CF₃] | C(=O)Me | Me | CH₂OMe | O |
| D-17380 | Me(S) | single bond | H | *c*-Pr[1-S(=O)₂CF₃] | C(=O)Me | Me | CH₂OMe | O |
| D-17381 | H | single bond | Me | c -Pr[1-(5,6-dihydro-1 ,4,2-dioxazin-3-yl)] | C(=O)Me | Cl | CH₂OMe | O |
| D-17382 | Me | single bond | H | c -Pr[1-(5,6-dihydro-1 ,4,2-dioxazin-3-yl)] | C(=O)Me | Cl | CH₂OMe | O |
| D-17383 | Me(R) | single bond | H | c -Pr[1-(5,6-dihydro-1 ,4,2-dioxazin-3-yl)] | C(=O)Me | Cl | CH₂OMe | O |
| D-17384 | Me(S) | single bond | H | c -Pr[1-(5,6-dihydro-1 ,4,2-dioxazin-3-yl)] | C(=O)Me | Cl | CH₂OMe | O |
| D-17385 | H | single bond | Me | c -Pr[1-(5,6-dihydro-1 ,4,2-dioxazin-3-yl)] | C(=O)Me | Me | CH₂OMe | O |
| D-17386 | Me | single bond | H | c -Pr[1-(5,6-dihydro-1 ,4,2-dioxazin-3-yl)] | C(=O)Me | Me | CH₂OMe | O |
| D-17387 | Me(R) | single bond | H | c -Pr[1-(5,6-dihydro-1 ,4,2-dioxazin-3-yl)] | C(=O)Me | Me | CH₂OMe | O |
| D-17388 | Me(S) | single bond | H | c -Pr[1-(5,6-dihydro-1 ,4,2-dioxazin-3-yl)] | C(=O)Me | Me | CH₂OMe | O |
| D-17389 | H | single bond | Me | c -Pr[1-(5-hydroxy-1 ,3,4-oxadiazol-2-yl)] | C(=O)Me | Cl | CH₂OMe | O |
| D-17390 | Me | single bond | H | c -Pr[1-(5-hydroxy-1 ,3,4-oxadiazol-2-yl)] | C(=O)Me | Cl | CH₂OMe | O |
| D-17391 | Me(R) | single bond | H | c -Pr[1-(5-hydroxy-1 ,3,4-oxadiazol-2-yl)] | C(=O)Me | Cl | CH₂OMe | O |
| D-17392 | Me(S) | single bond | H | c -Pr[1-(5-hydroxy-1 ,3,4-oxadiazol-2-yl)] | C(=O)Me | Cl | CH₂OMe | O |
| D-17393 | H | single bond | Me | c -Pr[1-(5-hydroxy-1 ,3,4-oxadiazol-2-yl)] | C(=O)Me | Me | CH₂OMe | O |
| D-17394 | Me | single bond | H | c -Pr[1-(5-hydroxy-1 ,3,4-oxadiazol-2-yl)] | C(=O)Me | Me | CH₂OMe | O |
| D-17395 | Me(R) | single bond | H | c -Pr[1-(5-hydroxy-1 ,3,4-oxadiazol-2-yl)] | C(=O)Me | Me | CH₂OMe | O |
| D-17396 | Me(S) | single bond | H | c -Pr[1-(5-hydroxy-1 ,3,4-oxadiazol-2-yl)] | C(=O)Me | Me | CH₂OMe | O |
| D-17397 | H | single bond | Me | c -Pr[1-(pyri midin-2-yl)] | C(=O)Me | Cl | CH₂OMe | O |
| D-17398 | Me | single bond | H | c -Pr[1-(pyri midin-2-yl)] | C(=O)Me | Cl | CH₂OMe | O |

**[Table 1042]**

| Compound Number | Y | W | Z | R¹ | R² | R³ | R¹⁰ | A |
|---|---|---|---|---|---|---|---|---|
| D-17399 | Me(R) | single bond | H | c -Pr[1-(pyri midin-2-yl)] | C(=O)Me | Cl | CH₂OMe | O |
| D-17400 | Me(S) | single bond | H | c -Pr[1-(pyri midin-2-yl)] | C(=O)Me | Cl | CH₂OMe | O |
| D-17401 | H | single bond | Me | c -Pr[1-(pyri midin-2-yl)] | C(=O)Me | Me | CH₂OMe | O |
| D-17402 | Me | single bond | H | c -Pr[1-(pyri midin-2-yl)] | C(=O)Me | Me | CH₂OMe | O |
| D-17403 | Me(R) | single bond | H | c -Pr[1-(pyri midin-2-yl)] | C(=O)Me | Me | CH₂OMe | O |
| D-17404 | Me(S) | single bond | H | c -Pr[1-(pyri midin-2-yl)] | C(=O)Me | Me | CH₂OMe | O |
| D-17405 | H | single bond | Me | t-Bu | C(=O)Me | Cl | CH₂OMe | O |
| D-17406 | Me | single bond | H | t-Bu | C(=O)Me | Cl | CH₂OMe | O |
| D-17407 | Me(R) | single bond | H | t-Bu | C(=O)Me | Cl | CH₂OMe | O |
| D-17408 | Me(S) | single bond | H | t-Bu | C(=O)Me | Cl | CH₂OMe | O |
| D-17409 | H | single bond | Me | t-Bu | C(=O)Me | Me | CH₂OMe | O |
| D-17410 | Me | single bond | H | t-Bu | C(=O)Me | Me | CH₂OMe | O |
| D-17411 | Me(R) | single bond | H | t-Bu | C(=O)Me | Me | CH₂OMe | O |
| D-17412 | Me(S) | single bond | H | t-Bu | C(=O)Me | Me | CH₂OMe | O |
| D-17413 | H | single bond | Me | *c*-Bu | C(=O)Me | Cl | CH₂OMe | O |
| D-17414 | Me | single bond | H | *c*-Bu | C(=O)Me | Cl | CH₂OMe | O |
| D-17415 | Me(R) | single bond | H | *c*-Bu | C(=O)Me | Cl | CH₂OMe | O |
| D-17416 | Me(S) | single bond | H | c-Bu | C(=O)Me | Cl | CH₂OMe | O |
| D-17417 | H | single bond | Me | *c*-Bu | C(=O)Me | Me | CH₂OMe | O |
| D-17418 | Me | single bond | H | *c*-Bu | C(=O)Me | Me | CH₂OMe | O |
| D-17419 | Me(R) | single bond | H | *c*-Bu | C(=O)Me | Me | CH₂OMe | O |
| D-17420 | Me(S) | single bond | H | *c*-Bu | C(=O)Me | Me | CH₂OMe | O |
| D-17421 | H | single bond | Me | c-Bu(1-CN) | C(=O)Me | Cl | CH₂OMe | O |
| D-17422 | Me | single bond | H | c-Bu(1-CN) | C(=O)Me | Cl | CH₂OMe | O |
| D-17423 | Me(R) | single bond | H | c-Bu(1-CN) | C(=O)Me | Cl | CH₂OMe | O |
| D-17424 | Me(S) | single bond | H | c-Bu(1-CN) | C(=O)Me | Cl | CH₂OMe | O |
| D-17425 | H | single bond | Me | c-Bu(1-CN) | C(=O)Me | Me | CH₂OMe | O |
| D-17426 | Me | single bond | H | c-Bu(1-CN) | C(=O)Me | Me | CH₂OMe | O |
| D-17427 | Me(R) | single bond | H | c-Bu(1-CN) | C(=O)Me | Me | CH₂OMe | O |
| D-17428 | Me(S) | single bond | H | c-Bu(1-CN) | C(=O)Me | Me | CH₂OMe | O |
| D-17429 | H | single bond | Me | *c*-Bu(3,3-F₂) | C(=O)Me | Cl | CH₂OMe | O |
| D-17430 | Me | single bond | H | *c*-Bu(3,3-F₂) | C(=O)Me | Cl | CH₂OMe | O |
| D-17431 | Me(R) | single bond | H | *c*-Bu(3,3-F₂) | C(=O)Me | Cl | CH₂OMe | O |
| D-17432 | Me(S) | single bond | H | *c*-Bu(3,3-F₂) | C(=O)Me | Cl | CH₂OMe | O |
| D-17433 | H | single bond | Me | *c*-Bu(3,3-F₂) | C(=O)Me | Me | CH₂OMe | O |
| D-17434 | Me | single bond | H | *c*-Bu(3,3-F₂) | C(=O)Me | Me | CH₂OMe | O |
| D-17435 | Me(R) | single bond | H | *c*-Bu(3,3-F₂) | C(=O)Me | Me | CH₂OMe | O |
| D-17436 | Me(S) | single bond | H | *c*-Bu(3,3-F₂) | C(=O)Me | Me | CH₂OMe | O |
| D-17437 | H | single bond | Me | oxetan-3-yl | C(=O)Me | Cl | CH₂OMe | O |
| D-17438 | Me | single bond | H | oxetan-3-yl | C(=O)Me | Cl | CH₂OMe | O |
| D-17439 | Me(R) | single bond | H | oxetan-3-yl | C(=O)Me | Cl | CH₂OMe | O |
| D-17440 | Me(S) | single bond | H | oxetan-3-yl | C(=O)Me | Cl | CH₂OMe | O |
| D-17441 | H | single bond | Me | oxetan-3-yl | C(=O)Me | Me | CH₂OMe | O |
| D-17442 | Me | single bond | H | oxetan-3-yl | C(=O)Me | Me | CH₂OMe | O |

**[Table 1043]**

| Compound Number | Y | W | Z | R¹ | R² | R³ | R¹⁰ | A |
|---|---|---|---|---|---|---|---|---|
| D-17443 | Me(R) | single bond | H | oxetan-3-yl | C(=O)Me | Me | CH₂OMe | O |
| D-17444 | Me(S) | single bond | H | oxetan-3-yl | C(=O)Me | Me | CH₂OMe | O |
| D-17445 | H | single bond | Me | oxetan(3-CN)-3-yl | C(=O)Me | Cl | CH₂OMe | O |
| D-17446 | Me | single bond | H | oxetan(3-CN)-3-yl | C(=O)Me | Cl | CH₂OMe | O |
| D-17447 | Me(R) | single bond | H | oxetan(3-CN)-3-yl | C(=O)Me | Cl | CH₂OMe | O |
| D-17448 | Me(S) | single bond | H | oxetan(3-CN)-3-yl | C(=O)Me | Cl | CH₂OMe | O |
| D-17449 | H | single bond | Me | oxetan(3-CN)-3-yl | C(=O)Me | Me | CH₂OMe | O |
| D-17450 | Me | single bond | H | oxetan(3-CN)-3-yl | C(=O)Me | Me | CH₂OMe | O |
| D-17451 | Me(R) | single bond | H | oxetan(3-CN)-3-yl | C(=O)Me | Me | CH₂OMe | O |
| D-17452 | Me(S) | single bond | H | oxetan(3-CN)-3-yl | C(=O)Me | Me | CH₂OMe | O |
| D-17453 | H | single bond | Me | azetidin[1-C(=O)Me]-3-yl | C(=O)Me | Cl | CH₂OMe | O |
| D-17454 | Me | single bond | H | azetidin[1-C(=O)Me]-3-yl | C(=O)Me | Cl | CH₂OMe | O |
| D-17455 | Me(R) | single bond | H | azetidin[1-C(=O)Me]-3-yl | C(=O)Me | Cl | CH₂OMe | O |
| D-17456 | Me(S) | single bond | H | azetidin[1-C(=O)Me]-3-yl | C(=O)Me | Cl | CH₂OMe | O |
| D-17457 | H | single bond | Me | azetidin[1-C(=O)Me]-3-yl | C(=O)Me | Me | CH₂OMe | O |
| D-17458 | Me | single bond | H | azetidin[1-C(=O)Me]-3-yl | C(=O)Me | Me | CH₂OMe | O |
| D-17459 | Me(R) | single bond | H | azetidin[1-C(=O)Me]-3-yl | C(=O)Me | Me | CH₂OMe | O |
| D-17460 | Me(S) | single bond | H | azetidin[1-C(=O)Me]-3-yl | C(=O)Me | Me | CH₂OMe | O |
| D-17461 | H | single bond | Me | azetidin[1-S(=O)₂Me]-3-yl | C(=O)Me | Cl | CH₂OMe | O |
| D-17462 | Me | single bond | H | azetidin[1-S(=O)₂Me]-3-yl | C(=O)Me | Cl | CH₂OMe | O |
| D-17463 | Me(R) | single bond | H | azetidin[1-S(=O)₂Me]-3-yl | C(=O)Me | Cl | CH₂OMe | O |
| D-17464 | Me(S) | single bond | H | azetidin[1-S(=O)₂Me]-3-yl | C(=O)Me | Cl | CH₂OMe | O |
| D-17465 | H | single bond | Me | azetidin[1-S(=O)₂Me]-3-yl | C(=O)Me | Me | CH₂OMe | O |
| D-17466 | Me | single bond | H | azetidin[1-S(=O)₂Me]-3-yl | C(=O)Me | Me | CH₂OMe | O |
| D-17467 | Me(R) | single bond | H | azetidin[1-S(=O)₂Me]-3-yl | C(=O)Me | Me | CH₂OMe | O |
| D-17468 | Me(S) | single bond | H | azetidin[1-S(=O)₂Me]-3-yl | C(=O)Me | Me | CH₂OMe | O |
| D-17469 | H | single bond | Me | azetidin[1-S(=O)₂CHF₂]-3-yl | C(=O)Me | Cl | CH₂OMe | O |
| D-17470 | Me | single bond | H | azetidin[1-S(=O)₂CHF₂]-3-yl | C(=O)Me | Cl | CH₂OMe | O |
| D-17471 | Me(R) | single bond | H | azetidin[1-S(=O)₂CHF₂]-3-yl | C(=O)Me | Cl | CH₂OMe | O |
| D-17472 | Me(S) | single bond | H | azetidin[1-S(=O)₂CHF₂]-3-yl | C(=O)Me | Cl | CH₂OMe | O |
| D-17473 | H | single bond | Me | azetidin[1-S(=O)₂CHF₂]-3-yl | C(=O)Me | Me | CH₂OMe | O |
| D-17474 | Me | single bond | H | azetidin[1-S(=O)₂CHF₂]-3-yl | C(=O)Me | Me | CH₂OMe | O |
| D-17475 | Me(R) | single bond | H | azetidin[1-S(=O)₂CHF₂]-3-yl | C(=O)Me | Me | CH₂OMe | O |
| D-17476 | Me(S) | single bond | H | azetidin[1-S(=O)₂CHF₂]-3-yl | C(=O)Me | Me | CH₂OMe | O |
| D-17477 | H | single bond | Me | c-Pen | C(=O)Me | Cl | CH₂OMe | O |
| D-17478 | Me | single bond | H | c-Pen | C(=O)Me | Cl | CH₂OMe | O |
| D-17479 | Me(R) | single bond | H | c-Pen | C(=O)Me | Cl | CH₂OMe | O |
| D-17480 | Me(S) | single bond | H | c-Pen | C(=O)Me | Cl | CH₂OMe | O |
| D-17481 | H | single bond | Me | c-Pen | C(=O)Me | Me | CH₂OMe | O |
| D-17482 | Me | single bond | H | c-Pen | C(=O)Me | Me | CH₂OMe | O |
| D-17483 | Me(R) | single bond | H | c-Pen | C(=O)Me | Me | CH₂OMe | O |
| D-17484 | Me(S) | single bond | H | c-Pen | C(=O)Me | Me | CH₂OMe | O |
| D-17485 | H | single bond | Me | tetrahydrofuran-3-yl | C(=O)Me | Cl | CH₂OMe | O |
| D-17486 | Me | single bond | H | tetrahydrofuran-3-yl | C(=O)Me | Cl | CH₂OMe | O |

**[Table 1044]**

| Compound Number | Y | W | Z | R¹ | R² | R³ | R¹⁰ | A |
|---|---|---|---|---|---|---|---|---|
| D-17487 | Me(R) | single bond | H | tetrahydrofuran-3-yl | C(=O)Me | Cl | CH₂OMe | O |
| D-17488 | Me(S) | single bond | H | tetrahydrofuran-3-yl | C(=O)Me | Cl | CH₂OMe | O |
| D-17489 | H | single bond | Me | tetrahydrofuran-3-yl | C(=O)Me | Me | CH₂OMe | O |
| D-17490 | Me | single bond | H | tetrahydrofuran-3-yl | C(=O)Me | Me | CH₂OMe | O |
| D-17491 | Me(R) | single bond | H | tetrahydrofuran-3-yl | C(=O)Me | Me | CH₂OMe | O |
| D-17492 | Me(S) | single bond | H | tetrahydrofuran-3-yl | C(=O)Me | Me | CH₂OMe | O |
| D-17493 | H | single bond | Me | tetrahydrothiophen-3-yl | C(=O)Me | Cl | CH₂OMe | O |
| D-17494 | Me | single bond | H | tetrahydrothiophen-3-yl | C(=O)Me | Cl | CH₂OMe | O |
| D-17495 | Me(R) | single bond | H | tetrahydrothiophen-3-yl | C(=O)Me | Cl | CH₂OMe | O |
| D-17496 | Me(S) | single bond | H | tetrahydrothiophen-3-yl | C(=O)Me | Cl | CH₂OMe | O |
| D-17497 | H | single bond | Me | tetrahydrothiophen-3-yl | C(=O)Me | Me | CH₂OMe | O |
| D-17498 | Me | single bond | H | tetrahydrothiophen-3-yl | C(=O)Me | Me | CH₂OMe | O |
| D-17499 | Me(R) | single bond | H | tetrahydrothiophen-3-yl | C(=O)Me | Me | CH₂OMe | O |
| D-17500 | Me(S) | single bond | H | tetrahydrothiophen-3-yl | C(=O)Me | Me | CH₂OMe | O |
| D-17501 | H | single bond | Me | c-Pen(1-CN) | C(=O)Me | Cl | CH₂OMe | O |
| D-17502 | Me | single bond | H | c-Pen(1-CN) | C(=O)Me | Cl | CH₂OMe | O |
| D-17503 | Me(R) | single bond | H | *c*-Pen(1-CN) | C(=O)Me | Cl | CH₂OMe | O |
| 0-17504 | Me(S) | single bond | H | *c*-Pen(1-CN) | C(=O)Me | Cl | CH₂OMe | O |
| D-17505 | H | single bond | Me | c-Pen(1-CN) | C(=O)Me | Me | CH₂OMe | O |
| D-17506 | Me | single bond | H | c-Pen(1-CN) | C(=O)Me | Me | CH₂OMe | O |
| D-17507 | Me(R) | single bond | H | c-Pen(1-CN) | C(=O)Me | Me | CH₂OMe | O |
| D-17508 | Me(S) | single bond | H | c-Pen(1-CN) | C(=O)Me | Me | CH₂OMe | O |
| D-17509 | H | single bond | Me | tetrahydrofuran(3-CN)-3-yl | C(=O)Me | Cl | CH₂OMe | O |
| D-17510 | Me | single bond | H | tetrahydrofuran(3-CN)-3-yl | C(=O)Me | Cl | CH₂OMe | O |
| D-17511 | Me(R) | single bond | H | tetrahydrofuran(3-CN)-3-yl | C(=O)Me | Cl | CH₂OMe | O |
| D-17512 | Me(S) | single bond | H | tetrahydrofuran(3-CN)-3-yl | C(=O)Me | Cl | CH₂OMe | O |
| D-17513 | H | single bond | Me | tetrahydrofuran(3-CN)-3-yl | C(=O)Me | Me | CH₂OMe | O |
| D-17514 | Me | single bond | H | tetrahydrofuran(3-CN)-3-yl | C(=O)Me | Me | CH₂OMe | O |
| D-17515 | Me(R) | single bond | H | tetrahydrofuran(3-CN)-3-yl | C(=O)Me | Me | CH₂OMe | O |
| D-17516 | Me(S) | single bond | H | tetrahydrofuran(3-CN)-3-yl | C(=O)Me | Me | CH₂OMe | O |
| D-17517 | H | single bond | Me | tetrahydrothiophen(3-CN)-3-yl | C(=O)Me | Cl | CH₂OMe | O |
| D-17518 | Me | single bond | H | tetrahydrothiophen(3-CN)-3-yl | C(=O)Me | Cl | CH₂OMe | O |
| D-17519 | Me(R) | single bond | H | tetrahydrothiophen(3-CN)-3-yl | C(=O)Me | Cl | CH₂OMe | O |
| D-17520 | Me(S) | single bond | H | tetrahydrothiophen(3-CN)-3-yl | C(=O)Me | Cl | CH₂OMe | O |
| D-17521 | H | single bond | Me | tetrahydrothiophen(3-CN)-3-yl | C(=O)Me | Me | CH₂OMe | O |
| D-17522 | Me | single bond | H | tetrahydrothiophen(3-CN)-3-yl | C(=O)Me | Me | CH₂OMe | O |
| D-17523 | Me(R) | single bond | H | tetrahydrothiophen(3-CN)-3-yl | C(=O)Me | Me | CH₂OMe | O |
| D-17524 | Me(S) | single bond | H | tetrahydrothiophen(3-CN)-3-yl | C(=O)Me | Me | CH₂OMe | O |
| D-17525 | H | single bond | Me | bicyclo[1.1.1]pertan-1-yl | C(=O)Me | Cl | CH₂OMe | O |
| D-17526 | Me | single bond | H | bicyclo[1.1.1]pertan-1-yl | C(=O)Me | Cl | CH₂OMe | O |
| D-17527 | Me(R) | single bond | H | bicyclo[1.1.1]pertan-1-yl | C(=O)Me | Cl | CH₂OMe | O |
| D-17528 | Me(S) | single bond | H | bicyclo[1.1.1]pertan-1-yl | C(=O)Me | Cl | CH₂OMe | O |
| D-17529 | H | single bond | Me | bicyclo[1.1.1]pertan-1-yl | C(=O)Me | Me | CH₂OMe | O |
| D-17530 | Me | single bond | H | bicyclo[1.1.1]pertan-1-yl | C(=O)Me | Me | CH₂OMe | O |

**[Table 1045]**

| Compound Number | Y | W | Z | R¹ | R² | R³ | R¹⁰ | A |
|---|---|---|---|---|---|---|---|---|
| D-17531 | Me(R) | single bond | H | bicyclo[1.1.1]pentan-1-yl | C(=O)Me | Me | CH₂OMe | O |
| D-17532 | Me(S) | single bond | H | bicyclo[1.1.1]pentan-1-yl | C(=O)Me | Me | CH₂OMe | O |
| D-17533 | H | single bond | Me | c -Hex | C(=O)Me | Cl | CH₂OMe | O |
| D-17534 | Me | single bond | H | c -Hex | C(=O)Me | Cl | CH₂OMe | O |
| D-17535 | Me(R) | single bond | H | c -Hex | C(=O)Me | Cl | CH₂OMe | O |
| D-17536 | Me(S) | single bond | H | c -Hex | C(=O)Me | Cl | CH₂OMe | O |
| D-17537 | H | single bond | Me | c -Hex | C(=O)Me | Me | CH₂OMe | O |
| D-17538 | Me | single bond | H | c -Hex | C(=O)Me | Me | CH₂OMe | O |
| D-17539 | Me(R) | single bond | H | c -Hex | C(=O)Me | Me | CH₂OMe | O |
| D-17540 | Me(S) | single bond | H | c -Hex | C(=O)Me | Me | CH₂OMe | O |
| D-17541 | H | single bond | Me | tetrahydropyran-4-yl | C(=O)Me | Cl | CH₂OMe | O |
| D-17542 | Me | single bond | H | tetrahydropyran-4-yl | C(=O)Me | Cl | CH₂OMe | O |
| D-17543 | Me(R) | single bond | H | tetrahydropyran-4-yl | C(=O)Me | Cl | CH₂OMe | O |
| D-17544 | Me(S) | single bond | H | tetrahydropyran-4-yl | C(=O)Me | Cl | CH₂OMe | O |
| D-17545 | H | single bond | Me | tetrahydropyran-4-yl | C(=O)Me | Me | CH₂OMe | O |
| D-17546 | Me | single bond | H | tetrahydropyran-4-yl | C(=O)Me | Me | CH₂OMe | O |
| D-17547 | Me(R) | single bond | H | tetrahydropyran-4-yl | C(=O)Me | Me | CH₂OMe | O |
| D-17548 | Me(S) | single bond | H | tetrahydropyran-4-yl | C(=O)Me | Me | CH₂OMe | O |
| D-17549 | H | single bond | Me | tetrahydrothiopyran-4-yl | C(=O)Me | Cl | CH₂OMe | O |
| D-17550 | Me | single bond | H | tetrahydrothiopyran-4-yl | C(=O)Me | Cl | CH₂OMe | O |
| D-17551 | Me(R) | single bond | H | tetrahydrothiopyran-4-yl | C(=O)Me | Cl | CH₂OMe | O |
| D-17552 | Me(S) | single bond | H | tetrahydrothiopyran-4-yl | C(=O)Me | Cl | CH₂OMe | O |
| D-17553 | H | single bond | Me | tetrahydrothiopyran-4-yl | C(=O)Me | Me | CH₂OMe | O |
| D-17554 | Me | single bond | H | tetrahydrothiopyran-4-yl | C(=O)Me | Me | CH₂OMe | O |
| D-17555 | Me(R) | single bond | H | tetrahydrothiopyran-4-yl | C(=O)Me | Me | CH₂OMe | O |
| D-17556 | Me(S) | single bond | H | tetrahydrothiopyran-4-yl | C(=O)Me | Me | CH₂OMe | O |
| D-17557 | H | single bond | Me | c-Hex(1-CN) | C(=O)Me | Cl | CH₂OMe | O |
| D-17558 | Me | single bond | H | c-Hex(1-CN) | C(=O)Me | Cl | CH₂OMe | O |
| D-17559 | Me(R) | single bond | H | c-Hex(1-CN) | C(=O)Me | Cl | CH₂OMe | O |
| D-17560 | Me(S) | single bond | H | c-Hex(1-CN) | C(=O)Me | Cl | CH₂OMe | O |
| D-17561 | H | single bond | Me | c-Hex(1-CN) | C(=O)Me | Me | CH₂OMe | O |
| D-17562 | Me | single bond | H | c-Hex(1-CN) | C(=O)Me | Me | CH₂OMe | O |
| D-17563 | Me(R) | single bond | H | c-Hex(1-CN) | C(=O)Me | Me | CH₂OMe | O |
| D-17564 | Me(S) | single bond | H | c-Hex(1-CN) | C(=O)Me | Me | CH₂OMe | O |
| D-17565 | H | single bond | Me | tetrahydropyran(4-CN)-4-yl | C(=O)Me | Cl | CH₂OMe | O |
| D-17566 | Me | single bond | H | tetrahydropyran(4-CN)-4-yl | C(=O)Me | Cl | CH₂OMe | O |
| D-17567 | Me(R) | single bond | H | tetrahydropyran(4-CN)-4-yl | C(=O)Me | Cl | CH₂OMe | O |
| D-17568 | Me(S) | single bond | H | tetrahydropyran(4-CN)-4-yl | C(=O)Me | Cl | CH₂OMe | O |
| D-17569 | H | single bond | Me | tetrahydropyran(4-CN)-4-yl | C(=O)Me | Me | CH₂OMe | O |
| D-17570 | Me | single bond | H | tetrahydropyran(4-CN)-4-yl | C(=O)Me | Me | CH₂OMe | O |
| D-17571 | Me(R) | single bond | H | tetrahydropyran(4-CN)-4-yl | C(=O)Me | Me | CH₂OMe | O |
| D-17572 | Me(S) | single bond | H | tetrahydropyran(4-CN)-4-yl | C(=O)Me | Me | CH₂OMe | O |
| D-17573 | H | single bond | Me | tetrahydrothiopyran(4-CN)-4-yl | C(=O)Me | Cl | CH₂OMe | O |
| D-17574 | Me | single bond | H | tetrahydrothiopyran(4-CN)-4-yl | C(=O)Me | Cl | CH₂OMe | O |

**[Table 1046]**

| Compound Number | Y | W | Z | R¹ | R² | R³ | R¹⁰ | A |
|---|---|---|---|---|---|---|---|---|
| D-17575 | Me(R) | single bond | H | tetrahydrothiopyran(4-CN)-4-yl | C(=O)Me | Cl | CH₂OMe | O |
| D-17576 | Me(S) | single bond | H | tetrahydrothiopyran(4-CN)-4-yl | C(=O)Me | Cl | CH₂OMe | O |
| D-17577 | H | single bond | Me | tetrahydrothiopyran(4-CN)-4-yl | C(=O)Me | Me | CH₂OMe | O |
| D-17578 | Me | single bond | H | tetrahydrothiopyran(4-CN)-4-yl | C(=O)Me | Me | CH₂OMe | O |
| D-17579 | Me(R) | single bond | H | tetrahydrothiopyran(4-CN)-4-yl | C(=O)Me | Me | CH₂OMe | O |
| D-17580 | Me(S) | single bond | H | tetrahydrothiopyran(4-CN)-4-yl | C(=O)Me | Me | CH₂OMe | O |
| D-17581 | H | single bond | Me | CH₂(*c* -Pr) | C(=O)Me | Cl | CH₂OMe | O |
| D-17582 | Me | single bond | H | CH₂(*c* -Pr) | C(=O)Me | Cl | CH₂OMe | O |
| D-17583 | Me(R) | single bond | H | CH₂(*c* -Pr) | C(=O)Me | Cl | CH₂OMe | O |
| D-17584 | Me(S) | single bond | H | CH₂(*c* -Pr) | C(=O)Me | Cl | CH₂OMe | O |
| D-17585 | H | single bond | Me | CH₂(*c* -Pr) | C(=O)Me | Me | CH₂OMe | O |
| D-17586 | Me | single bond | H | CH₂(*c* -Pr) | C(=O)Me | Me | CH₂OMe | O |
| D-17587 | Me(R) | single bond | H | CH₂(*c* -Pr) | C(=O)Me | Me | CH₂OMe | O |
| D-17588 | Me(S) | single bond | H | CH₂(*c* -Pr) | C(=O)Me | Me | CH₂OMe | O |
| D-17589 | H | single bond | Me | CH₂[*c* -Pr(1-CN)] | C(=O)Me | Cl | CH₂OMe | O |
| D-17590 | Me | single bond | H | CH₂[*c* -Pr(1-CN)] | C(=O)Me | Cl | CH₂OMe | O |
| D-17591 | Me(R) | single bond | H | CH₂[*c* -Pr(1-CN)] | C(=O)Me | Cl | CH₂OMe | O |
| D-17592 | Me(S) | single bond | H | CH₂[*c* -Pr(1-CN)] | C(=O)Me | Cl | CH₂OMe | O |
| D-17593 | H | single bond | Me | CH₂[*c* -Pr(1-CN)] | C(=O)Me | Me | CH₂OMe | O |
| D-17594 | Me | single bond | H | CH₂[*c* -Pr(1-CN)] | C(=O)Me | Me | CH₂OMe | O |
| D-17595 | Me(R) | single bond | H | CH₂[*c* -Pr(1-CN)] | C(=O)Me | Me | CH₂OMe | O |
| D-17596 | Me(S) | single bond | H | CH₂[*c* -Pr(1-CN)] | C(=O)Me | Me | CH₂OMe | O |
| D-17597 | H | single bond | Me | CH(Me)(*c*-Pr) | C(=O)Me | Cl | CH₂OMe | O |
| D-17598 | Me | single bond | H | CH(Me)(*c*-Pr) | C(=O)Me | Cl | CH₂OMe | O |
| D-17599 | Me(R) | single bond | H | CH(Me)(*c*-Pr) | C(=O)Me | Cl | CH₂OMe | O |
| D-17600 | Me(S) | single bond | H | CH(Me)(*c*-Pr) | C(=O)Me | Cl | CH₂OMe | O |
| D-17601 | H | single bond | Me | CH(Me)(*c*-Pr) | C(=O)Me | Me | CH₂OMe | O |
| D-17602 | Me | single bond | H | CH(Me)(*c*-Pr) | C(=O)Me | Me | CH₂OMe | O |
| D-17603 | Me(R) | single bond | H | CH(Me)(*c*-Pr) | C(=O)Me | Me | CH₂OMe | O |
| D-17604 | Me(S) | single bond | H | CH(Me)(*c*-Pr) | C(=O)Me | Me | CH₂OMe | O |
| D-17605 | H | single bond | Me | CH(Me)CH₂OMe | C(=O)Me | Cl | CH₂OMe | O |
| D-17606 | Me | single bond | H | CH(Me)CH₂OMe | C(=O)Me | Cl | CH₂OMe | O |
| D-17607 | Me(R) | single bond | H | CH(Me)CH₂OMe | C(=O)Me | Cl | CH₂OMe | O |
| D-17608 | Me(S) | single bond | H | CH(Me)CH₂OMe | C(=O)Me | Cl | CH₂OMe | O |
| D-17609 | H | single bond | Me | CH(Me)CH₂OMe | C(=O)Me | Me | CH₂OMe | O |
| D-17610 | Me | single bond | H | CH(Me)CH₂OMe | C(=O)Me | Me | CH₂OMe | O |
| D-17611 | Me(R) | single bond | H | CH(Me)CH₂OMe | C(=O)Me | Me | CH₂OMe | O |
| D-17612 | Me(S) | single bond | H | CH(Me)CH₂OMe | C(=O)Me | Me | CH₂OMe | O |
| D-17613 | H | single bond | Me | CH₂CH=CH₂ | C(=O)Me | Cl | CH₂OMe | O |
| D-17614 | Me | single bond | H | CH₂CH=CH₂ | C(=O)Me | Cl | CH₂OMe | O |
| D-17615 | Me(R) | single bond | H | CH₂CH=CH₂ | C(=O)Me | Cl | CH₂OMe | O |
| D-17616 | Me(S) | single bond | H | CH₂CH=CH₂ | C(=O)Me | Cl | CH₂OMe | O |
| D-17617 | H | single bond | Me | CH₂CH=CH₂ | C(=O)Me | Me | CH₂OMe | O |
| D-17618 | Me | single bond | H | CH₂CH=CH₂ | C(=O)Me | Me | CH₂OMe | O |

**[Table 1047]**

| Compound Number | Y | W | Z | R¹ | R² | R³ | R¹⁰ | A |
|---|---|---|---|---|---|---|---|---|
| D-17619 | Me(R) | single bond | H | CH₂CH=CH₂ | C(=O)Me | Me | CH₂OMe | O |
| D-17620 | Me(S) | single bond | H | CH₂CH=CH₂ | C(=O)Me | Me | CH₂OMe | O |
| D-17621 | H | single bond | Me | CH₂C≡CH | C(=O)Me | Cl | CH₂OMe | O |
| D-17622 | Me | single bond | H | CH₂C≡CH | C(=O)Me | Cl | CH₂OMe | O |
| D-17623 | Me(R) | single bond | H | CH₂C≡CH | C(=O)Me | Cl | CH₂OMe | O |
| D-17624 | Me(S) | single bond | H | CH₂C≡CH | C(=O)Me | Cl | CH₂OMe | O |
| D-17625 | H | single bond | Me | CH₂C≡CH | C(=O)Me | Me | CH₂OMe | O |
| D-17626 | Me | single bond | H | CH₂C≡CH | C(=O)Me | Me | CH₂OMe | O |
| D-17627 | Me(R) | single bond | H | CH₂C≡CH | C(=O)Me | Me | CH₂OMe | O |
| D-17628 | Me(S) | single bond | H | CH₂C≡CH | C(=O)Me | Me | CH₂OMe | O |
| D-17629 | H | single bond | Me | CH₂CN | C(=O)Me | Cl | CH₂OMe | O |
| D-17630 | Me | single bond | H | CH₂CN | C(=O)Me | Cl | CH₂OMe | O |
| D-17631 | Me(R) | single bond | H | CH₂CN | C(=O)Me | Cl | CH₂OMe | O |
| D-17632 | Me(S) | single bond | H | CH₂CN | C(=O)Me | Cl | CH₂OMe | O |
| D-17633 | H | single bond | Me | CH₂CN | C(=O)Me | Me | CH₂OMe | O |
| D-17634 | Me | single bond | H | CH₂CN | C(=O)Me | Me | CH₂OMe | O |
| D-17635 | Me(R) | single bond | H | CH₂CN | C(=O)Me | Me | CH₂OMe | O |
| D-17636 | Me(S) | single bond | H | CH₂CN | C(=O)Me | Me | CH₂OMe | O |
| D-17637 | H | single bond | Me | CH(Me)CN | C(=O)Me | Cl | CH₂OMe | O |
| D-17638 | Me | single bond | H | CH(Me)CN | C(=O)Me | Cl | CH₂OMe | O |
| D-17639 | Me(R) | single bond | H | CH(Me)CN | C(=O)Me | Cl | CH₂OMe | O |
| D-17640 | Me(S) | single bond | H | CH(Me)CN | C(=O)Me | Cl | CH₂OMe | O |
| D-17641 | H | single bond | Me | CH(Me)CN | C(=O)Me | Me | CH₂OMe | O |
| D-17642 | Me | single bond | H | CH(Me)CN | C(=O)Me | Me | CH₂OMe | O |
| D-17643 | Me(R) | single bond | H | CH(Me)CN | C(=O)Me | Me | CH₂OMe | O |
| D-17644 | Me(S) | single bond | H | CH(Me)CN | C(=O)Me | Me | CH₂OMe | O |
| D-17645 | H | single bond | Me | C(Me)₂CN | C(=O)Me | Cl | CH₂OMe | O |
| D-17646 | Me | single bond | H | C(Me)₂CN | C(=O)Me | Cl | CH₂OMe | O |
| D-17647 | Me(R) | single bond | H | C(Me)₂CN | C(=O)Me | Cl | CH₂OMe | O |
| D-17648 | Me(S) | single bond | H | C(Me)₂CN | C(=O)Me | Cl | CH₂OMe | O |
| D-17649 | H | single bond | Me | C(Me)₂CN | C(=O)Me | Me | CH₂OMe | O |
| D-17650 | Me | single bond | H | C(Me)₂CN | C(=O)Me | Me | CH₂OMe | O |
| D-17651 | Me(R) | single bond | H | C(Me)₂CN | C(=O)Me | Me | CH₂OMe | O |
| D-17652 | Me(S) | single bond | H | C(Me)₂CN | C(=O)Me | Me | CH₂OMe | O |
| D-17653 | H | single bond | Me | C(Me)(*i* -Pr)CN | C(=O)Me | Cl | CH₂OMe | O |
| D-17654 | Me | single bond | H | C(Me)(*i* -Pr)CN | C(=O)Me | Cl | CH₂OMe | O |
| D-17655 | Me(R) | single bond | H | C(Me)(*i* -Pr)CN | C(=O)Me | Cl | CH₂OMe | O |
| D-17656 | Me(S) | single bond | H | C(Me)(*i* -Pr)CN | C(=O)Me | Cl | CH₂OMe | O |
| D-17657 | H | single bond | Me | C(Me)(*i* -Pr)CN | C(=O)Me | Me | CH₂OMe | O |
| D-17658 | Me | single bond | H | C(Me)(*i* -Pr)CN | C(=O)Me | Me | CH₂OMe | O |
| D-17659 | Me(R) | single bond | H | C(Me)(*i* -Pr)CN | C(=O)Me | Me | CH₂OMe | O |
| D-17660 | Me(S) | single bond | H | C(Me)(*i* -Pr)CN | C(=O)Me | Me | CH₂OMe | O |
| D-17661 | H | single bond | Me | OMe | C(=O)Me | Cl | CH₂OMe | O |
| D-17662 | Me | single bond | H | OMe | C(=O)Me | Cl | CH₂OMe | O |

**[Table 1048]**

| Compound Number | Y | W | Z | R¹ | R² | R³ | R¹⁰ | A |
|---|---|---|---|---|---|---|---|---|
| D-17663 | Me(R) | single bond | H | OMe | C(=O)Me | Cl | CH₂OMe | O |
| D-17664 | Me(S) | single bond | H | OMe | C(=O)Me | Cl | CH₂OMe | O |
| D-17665 | H | single bond | Me | OMe | C(=O)Me | Me | CH₂OMe | O |
| D-17666 | Me | single bond | H | OMe | C(=O)Me | Me | CH₂OMe | O |
| D-17667 | Me(R) | single bond | H | OMe | C(=O)Me | Me | CH₂OMe | O |
| D-17668 | Me(S) | single bond | H | OMe | C(=O)Me | Me | CH₂OMe | O |
| D-17669 | H | single bond | Me | CH=NOMe | C(=O)Me | Cl | CH₂OMe | O |
| D-17670 | Me | single bond | H | CH=NOMe | C(=O)Me | Cl | CH₂OMe | O |
| D-17671 | Me(R) | single bond | H | CH=NOMe | C(=O)Me | Cl | CH₂OMe | O |
| D-17672 | Me(S) | single bond | H | CH=NOMe | C(=O)Me | Cl | CH₂OMe | O |
| D-17673 | H | single bond | Me | CH=NOMe | C(=O)Me | Me | CH₂OMe | O |
| D-17674 | Me | single bond | H | CH=NOMe | C(=O)Me | Me | CH₂OMe | O |
| D-17675 | Me(R) | single bond | H | CH=NOMe | C(=O)Me | Me | CH₂OMe | O |
| D-17676 | Me(S) | single bond | H | CH=NOMe | C(=O)Me | Me | CH₂OMe | O |
| D-17677 | H | single bond | Me | morpholin-4-yl | C(=O)Me | Cl | CH₂OMe | O |
| D-17678 | Me | single bond | H | morpholin-4-yl | C(=O)Me | Cl | CH₂OMe | O |
| D-17679 | Me(R) | single bond | H | morpholin-4-yl | C(=O)Me | Cl | CH₂OMe | O |
| D-17680 | Me(S) | single bond | H | morpholin-4-yl | C(=O)Me | Cl | CH₂OMe | O |
| D-17681 | H | single bond | Me | morpholin-4-yl | C(=O)Me | Me | CH₂OMe | O |
| D-17682 | Me | single bond | H | morpholin-4-yl | C(=O)Me | Me | CH₂OMe | O |
| D-17683 | Me(R) | single bond | H | morpholin-4-yl | C(=O)Me | Me | CH₂OMe | O |
| D-17684 | Me(S) | single bond | H | morpholin-4-yl | C(=O)Me | Me | CH₂OMe | O |
| D-17685 | H | single bond | Me | Ph | C(=O)Me | Cl | CH₂OMe | O |
| D-17686 | Me | single bond | H | Ph | C(=O)Me | Cl | CH₂OMe | O |
| D-17687 | Me(R) | single bond | H | Ph | C(=O)Me | Cl | CH₂OMe | O |
| D-17688 | Me(S) | single bond | H | Ph | C(=O)Me | Cl | CH₂OMe | O |
| D-17689 | H | single bond | Me | Ph | C(=O)Me | Me | CH₂OMe | O |
| D-17690 | Me | single bond | H | Ph | C(=O)Me | Me | CH₂OMe | O |
| D-17691 | Me(R) | single bond | H | Ph | C(=O)Me | Me | CH₂OMe | O |
| D-17692 | Me(S) | single bond | H | Ph | C(=O)Me | Me | CH₂OMe | O |
| D-17693 | H | single bond | Me | CH₂Ph | C(=O)Me | Cl | CH₂OMe | O |
| D-17694 | Me | single bond | H | CH₂Ph | C(=O)Me | Cl | CH₂OMe | O |
| D-17695 | Me(R) | single bond | H | CH₂Ph | C(=O)Me | Cl | CH₂OMe | O |
| D-17696 | Me(S) | single bond | H | CH₂Ph | C(=O)Me | Cl | CH₂OMe | O |
| D-17697 | H | single bond | Me | CH₂Ph | C(=O)Me | Me | CH₂OMe | O |
| D-17698 | Me | single bond | H | CH₂Ph | C(=O)Me | Me | CH₂OMe | O |
| D-17699 | Me(R) | single bond | H | CH₂Ph | C(=O)Me | Me | CH₂OMe | O |
| D-17700 | Me(S) | single bond | H | CH₂Ph | C(=O)Me | Me | CH₂OMe | O |
| D-17701 | H | single bond | Me | thiazole-2-yl | C(=O)Me | Cl | CH₂OMe | O |
| D-17702 | Me | single bond | H | thiazole-2-yl | C(=O)Me | Cl | CH₂OMe | O |
| D-17703 | Me(R) | single bond | H | thiazole-2-yl | C(=O)Me | Cl | CH₂OMe | O |
| D-17704 | Me(S) | single bond | H | thiazole-2-yl | C(=O)Me | Cl | CH₂OMe | O |
| D-17705 | H | single bond | Me | thiazole-2-yl | C(=O)Me | Me | CH₂OMe | O |
| D-17706 | Me | single bond | H | thiazole-2-yl | C(=O)Me | Me | CH₂OMe | O |

**[Table 1049]**

| Compound Number | Y | W | Z | R¹ | R² | R³ | R¹⁰ | A |
|---|---|---|---|---|---|---|---|---|
| D-17707 | Me(R) | single bond | H | thiazole-2-yl | C(=O)Me | Me | CH₂OMe | O |
| D-17708 | Me(S) | single bond | H | thiazole-2-yl | C(=O)Me | Me | CH₂OMe | O |
| D-17709 | H | single bond | Me | 1-Me-pyrazol-3-yl | C(=O)Me | Cl | CH₂OMe | O |
| D-17710 | Me | single bond | H | 1-Me-pyrazol-3-yl | C(=O)Me | Cl | CH₂OMe | O |
| D-17711 | Me(R) | single bond | H | 1-Me-pyrazol-3-yl | C(=O)Me | Cl | CH₂OMe | O |
| D-17712 | Me(S) | single bond | H | 1-Me-pyrazol-3-yl | C(=O)Me | Cl | CH₂OMe | O |
| D-17713 | H | single bond | Me | 1-Me-pyrazol-3-yl | C(=O)Me | Me | CH₂OMe | O |
| D-17714 | Me | single bond | H | 1-Me-pyrazol-3-yl | C(=O)Me | Me | CH₂OMe | O |
| D-17715 | Me(R) | single bond | H | 1-Me-pyrazol-3-yl | C(=O)Me | Me | CH₂OMe | O |
| D-17716 | Me(S) | single bond | H | 1-Me-pyrazol-3-yl | C(=O)Me | Me | CH₂OMe | O |
| D-17717 | H | single bond | Me | 1-Me-pyrazol-5-yl | C(=O)Me | Cl | CH₂OMe | O |
| D-17718 | Me | single bond | H | 1-Me-pyrazol-5-yl | C(=O)Me | Cl | CH₂OMe | O |
| D-17719 | Me(R) | single bond | H | 1-Me-pyrazol-5-yl | C(=O)Me | Cl | CH₂OMe | O |
| D-17720 | Me(S) | single bond | H | 1-Me-pyrazol-5-yl | C(=O)Me | Cl | CH₂OMe | O |
| D-17721 | H | single bond | Me | 1-Me-pyrazol-5-yl | C(=O)Me | Me | CH₂OMe | O |
| D-17722 | Me | single bond | H | 1-Me-pyrazol-5-yl | C(=O)Me | Me | CH₂OMe | O |
| D-17723 | Me(R) | single bond | H | 1-Me-pyrazol-5-yl | C(=O)Me | Me | CH₂OMe | O |
| D-17724 | Me(S) | single bond | H | 1-Me-pyrazol-5-yl | C(=O)Me | Me | CH₂OMe | O |
| D-17725 | H | single bond | Me | CH₂(pyrimidin-2-yl) | C(=O)Me | Cl | CH₂OMe | O |
| D-17726 | Me | single bond | H | CH₂(pyrimidin-2-yl) | C(=O)Me | Cl | CH₂OMe | O |
| D-17727 | Me(R) | single bond | H | CH₂(pyrimidin-2-yl) | C(=O)Me | Cl | CH₂OMe | O |
| D-17728 | Me(S) | single bond | H | CH₂(pyrimidin-2-yl) | C(=O)Me | Cl | CH₂OMe | O |
| D-17729 | H | single bond | Me | CH₂(pyrimidin-2-yl) | C(=O)Me | Me | CH₂OMe | O |
| D-17730 | Me | single bond | H | CH₂(pyrimidin-2-yl) | C(=O)Me | Me | CH₂OMe | O |
| D-17731 | Me(R) | single bond | H | CH₂(pyrimidin-2-yl) | C(=O)Me | Me | CH₂OMe | O |
| D-17732 | Me(S) | single bond | H | CH₂(pyrimidin-2-yl) | C(=O)Me | Me | CH₂OMe | O |
| D-17733 | H | single bond | Me | Me | C(=O)Me | Cl | C(=O)Me | O |
| D-17734 | Me | single bond | H | Me | C(=O)Me | Cl | C(=O)Me | O |
| D-17735 | Me(R) | single bond | H | Me | C(=O)Me | Cl | C(=O)Me | O |
| D-17736 | Me(S) | single bond | H | Me | C(=O)Me | Cl | C(=O)Me | O |
| D-17737 | H | single bond | Me | Me | C(=O)Me | Me | C(=O)Me | O |
| D-17738 | Me | single bond | H | Me | C(=O)Me | Me | C(=O)Me | O |
| D-17739 | Me(R) | single bond | H | Me | C(=O)Me | Me | C(=O)Me | O |
| D-17740 | Me(S) | single bond | H | Me | C(=O)Me | Me | C(=O)Me | O |
| D-17741 | H | single bond | Me | CH₂CHF₂ | C(=O)Me | Cl | C(=O)Me | O |
| D-17742 | Me | single bond | H | CH₂CHF₂ | C(=O)Me | Cl | C(=O)Me | O |
| D-17743 | Me(R) | single bond | H | CH₂CHF₂ | C(=O)Me | Cl | C(=O)Me | O |
| D-17744 | Me(S) | single bond | H | CH₂CHF₂ | C(=O)Me | Cl | C(=O)Me | O |
| D-17745 | H | single bond | Me | CH₂CHF₂ | C(=O)Me | Me | C(=O)Me | O |
| D-17746 | Me | single bond | H | CH₂CHF₂ | C(=O)Me | Me | C(=O)Me | O |
| D-17747 | Me(R) | single bond | H | CH₂CHF₂ | C(=O)Me | Me | C(=O)Me | O |
| D-17748 | Me(S) | single bond | H | CH₂CHF₂ | C(=O)Me | Me | C(=O)Me | O |
| D-17749 | H | single bond | Me | *n* -Pr | C(=O)Me | Cl | C(=O)Me | O |
| D-17750 | Me | single bond | H | *n* -Pr | C(=O)Me | Cl | C(=O)Me | O |

**[Table 1050]**

| Compound Number | Y | W | Z | R¹ | R² | R³ | R¹⁰ | A |
|---|---|---|---|---|---|---|---|---|
| D-17751 | Me(R) | single bond | H | *n*-Pr | C(=O)Me | Cl | C(=O)Me | O |
| D-17752 | Me(S) | single bond | H | *n*-Pr | C(=O)Me | Cl | C(=O)Me | O |
| D-17753 | H | single bond | Me | *n* -Pr | C(=O)Me | Me | C(=O)Me | O |
| D-17754 | Me | single bond | H | *n*-Pr | C(=O)Me | Me | C(=O)Me | O |
| D-17755 | Me(R) | single bond | H | *n*-Pr | C(=O)Me | Me | C(=O)Me | O |
| D-17756 | Me(S) | single bond | H | *n* -Pr | C(=O)Me | Me | C(=O)Me | O |
| D-17757 | H | single bond | Me | *i*-Pr | C(=O)Me | Cl | C(=O)Me | O |
| D-17758 | Me | single bond | H | *i*-Pr | C(=O)Me | Cl | C(=O)Me | O |
| D-17759 | Me(R) | single bond | H | *i*-Pr | C(=O)Me | Cl | C(=O)Me | O |
| D-17760 | Me(S) | single bond | H | *i*-Pr | C(=O)Me | Cl | C(=O)Me | O |
| D-17761 | H | single bond | Me | *i*-Pr | C(=O)Me | Me | C(=O)Me | O |
| D-17762 | Me | single bond | H | *i*-Pr | C(=O)Me | Me | C(=O)Me | O |
| D-17763 | Me(R) | single bond | H | *i*-Pr | C(=O)Me | Me | C(=O)Me | O |
| D-17764 | Me(S) | single bond | H | *i*-Pr | C(=O)Me | Me | C(=O)Me | O |
| D-17765 | H | single bond | Me | *c*-Pr(1-F) | C(=O)Me | Cl | C(=O)Me | O |
| D-17766 | Me | single bond | H | *c*-Pr(1-F) | C(=O)Me | Cl | C(=O)Me | O |
| D-17767 | Me(R) | single bond | H | *c*-Pr(1-F) | C(=O)Me | Cl | C(=O)Me | O |
| D-17768 | Me(S) | single bond | H | *c*-Pr(1-F) | C(=O)Me | Cl | C(=O)Me | O |
| D-17769 | H | single bond | Me | *c*-Pr(1-F) | C(=O)Me | Me | C(=O)Me | O |
| D-17770 | Me | single bond | H | *c*-Pr(1-F) | C(=O)Me | Me | C(=O)Me | O |
| D-17771 | Me(R) | single bond | H | c-Pr(1-F) | C(=O)Me | Me | C(=O)Me | O |
| D-17772 | Me(S) | single bond | H | c-Pr(1-F) | C(=O)Me | Me | C(=O)Me | O |
| D-17773 | H | single bond | Me | *c*-Pr(2,2-F₂) | C(=O)Me | Cl | C(=O)Me | O |
| D-17774 | Me | single bond | H | *c*-Pr(2,2-F₂) | C(=O)Me | Cl | C(=O)Me | O |
| D-17775 | Me(R) | single bond | H | *c*-Pr(2,2-F₂) | C(=O)Me | Cl | C(=O)Me | O |
| D-17776 | Me(S) | single bond | H | *c*-Pr(2,2-F₂) | C(=O)Me | Cl | C(=O)Me | O |
| D-17777 | H | single bond | Me | *c*-Pr(2,2-F₂) | C(=O)Me | Me | C(=O)Me | O |
| D-17778 | Me | single bond | H | *c*-Pr(2,2-F₂) | C(=O)Me | Me | C(=O)Me | O |
| D-17779 | Me(R) | single bond | H | *c*-Pr(2,2-F₂) | C(=O)Me | Me | C(=O)Me | O |
| D-17780 | Me(S) | single bond | H | *c*-Pr(2,2-F₂) | C(=O)Me | Me | C(=O)Me | O |
| D-17781 | H | single bond | Me | *c*-Pr(1-Cl) | C(=O)Me | Cl | C(=O)Me | O |
| D-17782 | Me | single bond | H | *c*-Pr(1-Cl) | C(=O)Me | Cl | C(=O)Me | O |
| D-17783 | Me(R) | single bond | H | *c*-Pr(1-Cl) | C(=O)Me | Cl | C(=O)Me | O |
| D-17784 | Me(S) | single bond | H | *c*-Pr(1-Cl) | C(=O)Me | Cl | C(=O)Me | O |
| D-17785 | H | single bond | Me | *c*-Pr(1-Cl) | C(=O)Me | Me | C(=O)Me | O |
| D-17786 | Me | single bond | H | *c*-Pr(1-Cl) | C(=O)Me | Me | C(=O)Me | O |
| D-17787 | Me(R) | single bond | H | *c*-Pr(1-Cl) | C(=O)Me | Me | C(=O)Me | O |
| D-17788 | Me(S) | single bond | H | *c*-Pr(1-Cl) | C(=O)Me | Me | C(=O)Me | O |
| D-17789 | H | single bond | Me | *c* -Pr(1-Br) | C(=O)Me | Cl | C(=O)Me | O |
| D-17790 | Me | single bond | H | *c* -Pr(1-Br) | C(=O)Me | Cl | C(=O)Me | O |
| D-17791 | Me(R) | single bond | H | *c* -Pr(1-Br) | C(=O)Me | Cl | C(=O)Me | O |
| D-17792 | Me(S) | single bond | H | *c* -Pr(1-Br) | C(=O)Me | Cl | C(=O)Me | O |
| D-17793 | H | single bond | Me | *c* -Pr(1-Br) | C(=O)Me | Me | C(=O)Me | O |
| D-17794 | Me | single bond | H | *c* -Pr(1-Br) | C(=O)Me | Me | C(=O)Me | O |

**[Table 1051]**

| Compound Number | Y | W | Z | R¹ | R² | R³ | R¹⁰ | A |
|---|---|---|---|---|---|---|---|---|
| D-17795 | Me(R) | single bond | H | *c* -Pr(1-Br) | C(=O)Me | Me | C(=O)Me | O |
| D-17796 | Me(S) | single bond | H | *c* -Pr(1-Br) | C(=O)Me | Me | C(=O)Me | O |
| D-17797 | H | single bond | Me | *c*-Pr(1-Me) | C(=O)Me | Cl | C(=O)Me | O |
| D-17798 | Me | single bond | H | *c*-Pr(1-Me) | C(=O)Me | Cl | C(=O)Me | O |
| D-17799 | Me(R) | single bond | H | *c*-Pr(1-Me) | C(=O)Me | Cl | C(=O)Me | O |
| D-17800 | Me(S) | single bond | H | *c*-Pr(1-Me) | C(=O)Me | Cl | C(=O)Me | O |
| D-17801 | H | single bond | Me | *c*-Pr(1-Me) | C(=O)Me | Me | C(=O)Me | O |
| D-17802 | Me | single bond | H | *c*-Pr(1-Me) | C(=O)Me | Me | C(=O)Me | O |
| D-17803 | Me(R) | single bond | H | *c*-Pr(1-Me) | C(=O)Me | Me | C(=O)Me | O |
| D-17804 | Me(S) | single bond | H | *c*-Pr(1-Me) | C(=O)Me | Me | C(=O)Me | O |
| D-17805 | H | single bond | Me | *c*-Pr(1-CH₂OH) | C(=O)Me | Cl | C(=O)Me | O |
| D-17806 | Me | single bond | H | *c*-Pr(1-CH₂OH) | C(=O)Me | Cl | C(=O)Me | O |
| D-17807 | Me(R) | single bond | H | *c*-Pr(1-CH₂OH) | C(=O)Me | Cl | C(=O)Me | O |
| D-17808 | Me(S) | single bond | H | *c*-Pr(1-CH₂OH) | C(=O)Me | Cl | C(=O)Me | O |
| D-17809 | H | single bond | Me | *c*-Pr(1-CH₂OH) | C(=O)Me | Me | C(=O)Me | O |
| D-17810 | Me | single bond | H | *c*-Pr(1-CH₂OH) | C(=O)Me | Me | C(=O)Me | O |
| D-17811 | Me(R) | single bond | H | *c*-Pr(1-CH₂OH) | C(=O)Me | Me | C(=O)Me | O |
| D-17812 | Me(S) | single bond | H | *c*-Pr(1-CH₂OH) | C(=O)Me | Me | C(=O)Me | O |
| D-17813 | H | single bond | Me | *c*-Pr[1-CH₂OS(=O)₂Me] | C(=O)Me | Cl | C(=O)Me | O |
| D-17814 | Me | single bond | H | *c*-Pr[1-CH₂OS(=O)₂Me] | C(=O)Me | Cl | C(=O)Me | O |
| D-17815 | Me(R) | single bond | H | *c*-Pr[1-CH₂OS(=O)₂Me] | C(=O)Me | Cl | C(=O)Me | O |
| D-17816 | Me(S) | single bond | H | *c*-Pr[1-CH₂OS(=O)₂Me] | C(=O)Me | Cl | C(=O)Me | O |
| D-17817 | H | single bond | Me | *c*-Pr[1-CH₂OS(=O)₂Me] | C(=O)Me | Me | C(=O)Me | O |
| D-17818 | Me | single bond | H | *c*-Pr[1-CH₂OS(=O)₂Me] | C(=O)Me | Me | C(=O)Me | O |
| D-17819 | Me(R) | single bond | H | *c*-Pr[1-CH₂OS(=O)₂Me] | C(=O)Me | Me | C(=O)Me | O |
| D-17820 | Me(S) | single bond | H | *c*-Pr[1-CH₂OS(=O)₂Me] | C(=O)Me | Me | C(=O)Me | O |
| D-17821 | H | single bond | Me | *c*-Pr(1-CH₂OMe) | C(=O)Me | Cl | C(=O)Me | O |
| D-17822 | Me | single bond | H | *c*-Pr(1-CH₂OMe) | C(=O)Me | Cl | C(=O)Me | O |
| D-17823 | Me(R) | single bond | H | *c*-Pr(1-CH₂OMe) | C(=O)Me | Cl | C(=O)Me | O |
| D-17824 | Me(S) | single bond | H | *c*-Pr(1-CH₂OMe) | C(=O)Me | Cl | C(=O)Me | O |
| D-17825 | H | single bond | Me | *c*-Pr(1-CH₂OMe) | C(=O)Me | Me | C(=O)Me | O |
| D-17826 | Me | single bond | H | *c*-Pr(1-CH₂OMe) | C(=O)Me | Me | C(=O)Me | O |
| D-17827 | Me(R) | single bond | H | *c*-Pr(1-CH₂OMe) | C(=O)Me | Me | C(=O)Me | O |
| D-17828 | Me(S) | single bond | H | *c*-Pr(1-CH₂OMe) | C(=O)Me | Me | C(=O)Me | O |
| D-17829 | H | single bond | Me | *c*-Pr(1-CH₂OCN) | C(=O)Me | Cl | C(=O)Me | O |
| D-17830 | Me | single bond | H | *c*-Pr(1-CH₂OCN) | C(=O)Me | Cl | C(=O)Me | O |
| D-17831 | Me(R) | single bond | H | *c*-Pr(1-CH₂OCN) | C(=O)Me | Cl | C(=O)Me | O |
| D-17832 | Me(S) | single bond | H | *c*-Pr(1-CH₂OCN) | C(=O)Me | Cl | C(=O)Me | O |
| D-17833 | H | single bond | Me | *c*-Pr(1-CH₂OCN) | C(=O)Me | Me | C(=O)Me | O |
| D-17834 | Me | single bond | H | *c*-Pr(1-CH₂OCN) | C(=O)Me | Me | C(=O)Me | O |
| D-17835 | Me(R) | single bond | H | *c*-Pr(1-CH₂OCN) | C(=O)Me | Me | C(=O)Me | O |
| D-17836 | Me(S) | single bond | H | *c*-Pr(1-CH₂OCN) | C(=O)Me | Me | C(=O)Me | O |
| D-17837 | H | single bond | Me | *c*-Pr(1-CH₂SMe) | C(=O)Me | Cl | C(=O)Me | O |
| D-17838 | Me | single bond | H | *c*-Pr(1-CH₂SMe) | C(=O)Me | Cl | C(=O)Me | O |

**[Table 1052]**

| Compound Number | Y | W | Z | R¹ | R² | R³ | R¹⁰ | A |
|---|---|---|---|---|---|---|---|---|
| D-17839 | Me(R) | single bond | H | *c*-Pr(1-CH₂SMe) | C(=O)Me | Cl | C(=O)Me | O |
| D-17840 | Me(S) | single bond | H | *c*-Pr(1-CH₂SMe) | C(=O)Me | Cl | C(=O)Me | O |
| D-17841 | H | single bond | Me | *c*-Pr(1-CH₂SMe) | C(=O)Me | Me | C(=O)Me | O |
| D-17842 | Me | single bond | H | *c*-Pr(1-CH₂SMe) | C(=O)Me | Me | C(=O)Me | O |
| D-17843 | Me(R) | single bond | H | *c*-Pr(1-CH₂SMe) | C(=O)Me | Me | C(=O)Me | O |
| D-17844 | Me(S) | single bond | H | *c*-Pr(1-CH₂SMe) | C(=O)Me | Me | C(=O)Me | O |
| D-17845 | H | single bond | Me | *c*-Pr[1-CH₂S(=O)Me] | C(=O)Me | Cl | C(=O)Me | O |
| D-17846 | Me | single bond | H | *c*-Pr[1-CH₂S(=O)Me] | C(=O)Me | Cl | C(=O)Me | O |
| D-17847 | Me(R) | single bond | H | *c*-Pr[1-CH₂S(=O)Me] | C(=O)Me | Cl | C(=O)Me | O |
| D-17848 | Me(S) | single bond | H | *c*-Pr[1-CH₂S(=O)Me] | C(=O)Me | Cl | C(=O)Me | O |
| D-17849 | H | single bond | Me | *c*-Pr[1-CH₂S(=O)Me] | C(=O)Me | Me | C(=O)Me | O |
| D-17850 | Me | single bond | H | *c*-Pr[1-CH₂S(=O)Me] | C(=O)Me | Me | C(=O)Me | O |
| D-17851 | Me(R) | single bond | H | *c*-Pr[1-CH₂S(=O)Me] | C(=O)Me | Me | C(=O)Me | O |
| D-17852 | Me(S) | single bond | H | *c*-Pr[1-CH₂S(=O)Me] | C(=O)Me | Me | C(=O)Me | O |
| D-17853 | H | single bond | Me | *c* -Pr[1-CH₂S(=O)₂Me] | C(=O)Me | Cl | C(=O)Me | O |
| D-17854 | Me | single bond | H | *c* -Pr[1-CH₂S(=O)₂Me] | C(=O)Me | Cl | C(=O)Me | O |
| D-17855 | Me(R) | single bond | H | *c* -Pr[1-CH₂S(=O)₂Me] | C(=O)Me | Cl | C(=O)Me | O |
| D-17856 | Me(S) | single bond | H | *c* -Pr[1-CH₂S(=O)₂Me] | C(=O)Me | Cl | C(=O)Me | O |
| D-17857 | H | single bond | Me | *c* -Pr[1-CH₂S(=O)₂Me] | C(=O)Me | Me | C(=O)Me | O |
| D-17858 | Me | single bond | H | *c* -Pr[1-CH₂S(=O)₂Me] | C(=O)Me | Me | C(=O)Me | O |
| D-17859 | Me(R) | single bond | H | *c* -Pr[1-CH₂S(=O)₂Me] | C(=O)Me | Me | C(=O)Me | O |
| D-17860 | Me(S) | single bond | H | *c* -Pr[1-CH₂S(=O)₂Me] | C(=O)Me | Me | C(=O)Me | O |
| D-17861 | H | single bond | Me | *c*-Pr(1-CH₂F) | C(=O)Me | Cl | C(=O)Me | O |
| D-17862 | Me | single bond | H | *c*-Pr(1-CH₂F) | C(=O)Me | Cl | C(=O)Me | O |
| D-17863 | Me(R) | single bond | H | *c*-Pr(1-CH₂F) | C(=O)Me | Cl | C(=O)Me | O |
| D-17864 | Me(S) | single bond | H | *c*-Pr(1-CH₂F) | C(=O)Me | Cl | C(=O)Me | O |
| D-17865 | H | single bond | Me | *c*-Pr(1-CH₂F) | C(=O)Me | Me | C(=O)Me | O |
| D-17866 | Me | single bond | H | *c*-Pr(1-CH₂F) | C(=O)Me | Me | C(=O)Me | O |
| D-17867 | Me(R) | single bond | H | *c*-Pr(1-CH₂F) | C(=O)Me | Me | C(=O)Me | O |
| D-17868 | Me(S) | single bond | H | *c*-Pr(1-CH₂F) | C(=O)Me | Me | C(=O)Me | O |
| D-17869 | H | single bond | Me | *c*-Pr(1-CHF₂) | C(=O)Me | Cl | C(=O)Me | O |
| D-17870 | Me | single bond | H | *c*-Pr(1-CHF₂) | C(=O)Me | Cl | C(=O)Me | O |
| D-17871 | Me(R) | single bond | H | *c*-Pr(1-CHF₂) | C(=O)Me | Cl | C(=O)Me | O |
| D-17872 | Me(S) | single bond | H | *c*-Pr(1-CHF₂) | C(=O)Me | Cl | C(=O)Me | O |
| D-17873 | H | single bond | Me | *c*-Pr(1-CHF₂) | C(=O)Me | Me | C(=O)Me | O |
| D-17874 | Me | single bond | H | *c*-Pr(1-CHF₂) | C(=O)Me | Me | C(=O)Me | O |
| D-17875 | Me(R) | single bond | H | *c*-Pr(1-CHF₂) | C(=O)Me | Me | C(=O)Me | O |
| D-17876 | Me(S) | single bond | H | *c*-Pr(1-CHF₂) | C(=O)Me | Me | C(=O)Me | O |
| D-17877 | H | single bond | Me | *c*-Pr(1-CF₃) | C(=O)Me | Cl | C(=O)Me | O |
| D-17878 | Me | single bond | H | *c*-Pr(1-CF₃) | C(=O)Me | Cl | C(=O)Me | O |
| D-17879 | Me(R) | single bond | H | *c*-Pr(1-CF₃) | C(=O)Me | Cl | C(=O)Me | O |
| D-17880 | Me(S) | single bond | H | *c*-Pr(1-CF₃) | C(=O)Me | Cl | C(=O)Me | O |
| D-17881 | H | single bond | Me | *c*-Pr(1-CF₃) | C(=O)Me | Me | C(=O)Me | O |
| D-17882 | Me | single bond | H | *c*-Pr(1-CF₃) | C(=O)Me | Me | C(=O)Me | O |

**[Table 1053]**

| Compound Number | Y | W | Z | R¹ | R² | R³ | R¹⁰ | A |
|---|---|---|---|---|---|---|---|---|
| D-17883 | Me(R) | single bond | H | *c*-Pr(1-CF₃) | C(=O)Me | Me | C(=O)Me | O |
| D-17884 | Me(S) | single bond | H | *c*-Pr(1-CF₃) | C(=O)Me | Me | C(=O)Me | O |
| D-17885 | H | single bond | Me | *c*-Pr(1-C≡CH) | C(=O)Me | Cl | C(=O)Me | O |
| D-17886 | Me | single bond | H | *c*-Pr(1-C≡CH) | C(=O)Me | Cl | C(=O)Me | O |
| D-17887 | Me(R) | single bond | H | *c*-Pr(1-C≡CH) | C(=O)Me | Cl | C(=O)Me | O |
| D-17888 | Me(S) | single bond | H | *c*-Pr(1-C≡CH) | C(=O)Me | Cl | C(=O)Me | O |
| D-17889 | H | single bond | Me | *c*-Pr(1-C≡CH) | C(=O)Me | Me | C(=O)Me | O |
| D-17890 | Me | single bond | H | *c*-Pr(1-C≡CH) | C(=O)Me | Me | C(=O)Me | O |
| D-17891 | Me(R) | single bond | H | *c*-Pr(1-C≡CH) | C(=O)Me | Me | C(=O)Me | O |
| D-17892 | Me(S) | single bond | H | *c*-Pr(1-C≡CH) | C(=O)Me | Me | C(=O)Me | O |
| D-17893 | H | single bond | Me | *c*-Pr[1-C(=O)OEt] | C(=O)Me | Cl | C(=O)Me | O |
| D-17894 | Me | single bond | H | *c*-Pr[1-C(=O)OEt] | C(=O)Me | Cl | C(=O)Me | O |
| D-17895 | Me(R) | single bond | H | *c*-Pr[1-C(=O)OEt] | C(=O)Me | Cl | C(=O)Me | O |
| D-17896 | Me(S) | single bond | H | *c*-Pr[1-C(=O)OEt] | C(=O)Me | Cl | C(=O)Me | O |
| D-17897 | H | single bond | Me | *c*-Pr[1-C(=O)OEt] | C(=O)Me | Me | C(=O)Me | O |
| D-17898 | Me | single bond | H | *c*-Pr[1-C(=O)OEt] | C(=O)Me | Me | C(=O)Me | O |
| D-17899 | Me(R) | single bond | H | *c*-Pr[1-C(=O)OEt] | C(=O)Me | Me | C(=O)Me | O |
| D-17900 | Me(S) | single bond | H | *c*-Pr[1-C(=O)OEt] | C(=O)Me | Me | C(=O)Me | O |
| D-17901 | H | single bond | Me | *c*-Pr[1-C(=O)OH] | C(=O)Me | Cl | C(=O)Me | O |
| D-17902 | Me | single bond | H | *c*-Pr[1-C(=O)OH] | C(=O)Me | Cl | C(=O)Me | O |
| D-17903 | Me(R) | single bond | H | *c*-Pr[1-C(=O)OH] | C(=O)Me | Cl | C(=O)Me | O |
| D-17904 | Me(S) | single bond | H | *c*-Pr[1-C(=O)OH] | C(=O)Me | Cl | C(=O)Me | O |
| D-17905 | H | single bond | Me | *c*-Pr[1-C(=O)OH] | C(=O)Me | Me | C(=O)Me | O |
| D-17906 | Me | single bond | H | *c*-Pr[1-C(=O)OH] | C(=O)Me | Me | C(=O)Me | O |
| D-17907 | Me(R) | single bond | H | *c*-Pr[1-C(=O)OH] | C(=O)Me | Me | C(=O)Me | O |
| D-17908 | Me(S) | single bond | H | *c*-Pr[1-C(=O)OH] | C(=O)Me | Me | C(=O)Me | O |
| D-17909 | H | single bond | Me | *c*-Pr[1-C(=O)NH₂ | C(=O)Me | Cl | C(=O)Me | O |
| D-17910 | Me | single bond | H | *c*-Pr[1-C(=O)NH₂ | C(=O)Me | Cl | C(=O)Me | O |
| D-17911 | Me(R) | single bond | H | *c*-Pr[1-C(=O)NH₂ | C(=O)Me | Cl | C(=O)Me | O |
| D-17912 | Me(S) | single bond | H | *c*-Pr[1-C(=O)NH₂ | C(=O)Me | Cl | C(=O)Me | O |
| D-17913 | H | single bond | Me | *c*-Pr[1-C(=O)NH₂] | C(=O)Me | Me | C(=O)Me | O |
| D-17914 | Me | single bond | H | *c*-Pr[1-C(=O)NH₂ | C(=O)Me | Me | C(=O)Me | O |
| D-17915 | Me(R) | single bond | H | *c*-Pr[1-C(=O)NH₂ | C(=O)Me | Me | C(=O)Me | O |
| D-17916 | Me(S) | single bond | H | *c*-Pr[1-C(=O)NH₂ | C(=O)Me | Me | C(=O)Me | O |
| D-17917 | H | single bond | Me | *c*-Pr(1-OMe) | C(=O)Me | Cl | C(=O)Me | O |
| D-17918 | Me | single bond | H | *c*-Pr(1-OMe) | C(=O)Me | Cl | C(=O)Me | O |
| D-17919 | Me(R) | single bond | H | *c*-Pr(1-OMe) | C(=O)Me | Cl | C(=O)Me | O |
| D-17920 | Me(S) | single bond | H | *c*-Pr(1-OMe) | C(=O)Me | Cl | C(=O)Me | O |
| D-17921 | H | single bond | Me | *c*-Pr(1-OMe) | C(=O)Me | Me | C(=O)Me | O |
| D-17922 | Me | single bond | H | *c*-Pr(1-OMe) | C(=O)Me | Me | C(=O)Me | O |
| D-17923 | Me(R) | single bond | H | *c*-Pr(1-OMe) | C(=O)Me | Me | C(=O)Me | O |
| D-17924 | Me(S) | single bond | H | *c*-Pr(1-OMe) | C(=O)Me | Me | C(=O)Me | O |
| D-17925 | H | single bond | Me | *c*-Pr(1-SMe) | C(=O)Me | Cl | C(=O)Me | O |
| D-17926 | Me | single bond | H | *c*-Pr(1-SMe) | C(=O)Me | Cl | C(=O)Me | O |

**[Table 1054]**

| Compound Number | Y | W | Z | R¹ | R² | R³ | R¹⁰ | A |
|---|---|---|---|---|---|---|---|---|
| D-17927 | Me(R) | single bond | H | *c*-Pr(1-SMe) | C(=O)Me | Cl | C(=O)Me | O |
| D-17928 | Me(S) | single bond | H | *c*-Pr(1-SMe) | C(=O)Me | Cl | C(=O)Me | O |
| D-17929 | H | single bond | Me | *c*-Pr(1-SMe) | C(=O)Me | Me | C(=O)Me | O |
| D-17930 | Me | single bond | H | *c*-Pr(1-SMe) | C(=O)Me | Me | C(=O)Me | O |
| D-17931 | Me(R) | single bond | H | *c*-Pr(1-SMe) | C(=O)Me | Me | C(=O)Me | O |
| D-17932 | Me(S) | single bond | H | *c*-Pr(1-SMe) | C(=O)Me | Me | C(=O)Me | O |
| D-17933 | H | single bond | Me | *c*-Pr[1-S(=O)Me] | C(=O)Me | Cl | C(=O)Me | O |
| D-17934 | Me | single bond | H | *c*-Pr[1-S(=O)Me] | C(=O)Me | Cl | C(=O)Me | O |
| D-17935 | Me(R) | single bond | H | *c*-Pr[1-S(=O)Me] | C(=O)Me | Cl | C(=O)Me | O |
| D-17936 | Me(S) | single bond | H | *c*-Pr[1-S(=O)Me] | C(=O)Me | Cl | C(=O)Me | O |
| D-17937 | H | single bond | Me | *c*-Pr[1-S(=O)Me] | C(=O)Me | Me | C(=O)Me | O |
| D-17938 | Me | single bond | H | *c*-Pr[1-S(=O)Me] | C(=O)Me | Me | C(=O)Me | O |
| D-17939 | Me(R) | single bond | H | *c*-Pr[1-S(=O)Me] | C(=O)Me | Me | C(=O)Me | O |
| D-17940 | Me(S) | single bond | H | *c*-Pr[1-S(=O)Me] | C(=O)Me | Me | C(=O)Me | O |
| D-17941 | H | single bond | Me | *c*-Pr[1-S(=O)₂Me] | C(=O)Me | Cl | C(=O)Me | O |
| D-17942 | Me | single bond | H | *c*-Pr[1-S(=O)₂Me] | C(=O)Me | Cl | C(=O)Me | O |
| D-17943 | Me(R) | single bond | H | *c*-Pr[1-S(=O)₂Me] | C(=O)Me | Cl | C(=O)Me | O |
| D-17944 | Me(S) | single bond | H | *c*-Pr[1-S(=O)₂Me] | C(=O)Me | Cl | C(=O)Me | O |
| D-17945 | H | single bond | Me | *c*-Pr[1-S(=O)₂Me] | C(=O)Me | Me | C(=O)Me | O |
| D-17946 | Me | single bond | H | *c*-Pr[1-S(=O)₂Me] | C(=O)Me | Me | C(=O)Me | O |
| D-17947 | Me(R) | single bond | H | *c*-Pr[1-S(=O)₂Me] | C(=O)Me | Me | C(=O)Me | O |
| D-17948 | Me(S) | single bond | H | *c*-Pr[1-S(=O)₂Me] | C(=O)Me | Me | C(=O)Me | O |
| D-17949 | H | single bond | Me | *c*-Pr[1-S(=O)₂CHF₂] | C(=O)Me | Cl | C(=O)Me | O |
| D-17950 | Me | single bond | H | *c*-Pr[1-S(=O)₂CHF₂] | C(=O)Me | Cl | C(=O)Me | O |
| D-17951 | Me(R) | single bond | H | *c*-Pr[1-S(=O)₂CHF₂] | C(=O)Me | Cl | C(=O)Me | O |
| D-17952 | Me(S) | single bond | H | *c*-Pr[1-S(=O)₂CHF₂] | C(=O)Me | Cl | C(=O)Me | O |
| D-17953 | H | single bond | Me | *c*-Pr[1-S(=O)₂CHF₂] | C(=O)Me | Me | C(=O)Me | O |
| D-17954 | Me | single bond | H | *c*-Pr[1-S(=O)₂CHF₂] | C(=O)Me | Me | C(=O)Me | O |
| D-17955 | Me(R) | single bond | H | *c*-Pr[1-S(=O)₂CHF₂] | C(=O)Me | Me | C(=O)Me | O |
| D-17956 | Me(S) | single bond | H | *c*-Pr[1-S(=O)₂CHF₂] | C(=O)Me | Me | C(=O)Me | O |
| D-17957 | H | single bond | Me | *c*-Pr[1-S(=O)₂CF₃] | C(=O)Me | Cl | C(=O)Me | O |
| D-17958 | Me | single bond | H | *c*-Pr[1-S(=O)₂CF₃] | C(=O)Me | Cl | C(=O)Me | O |
| D-17959 | Me(R) | single bond | H | *c*-Pr[1-S(=O)₂CF₃] | C(=O)Me | Cl | C(=O)Me | O |
| D-17960 | Me(S) | single bond | H | *c*-Pr[1-S(=O)₂CF₃] | C(=O)Me | Cl | C(=O)Me | O |
| D-17961 | H | single bond | Me | *c*-Pr[1-S(=O)₂CF₃] | C(=O)Me | Me | C(=O)Me | O |
| D-17962 | Me | single bond | H | *c*-Pr[1-S(=O)₂CF₃] | C(=O)Me | Me | C(=O)Me | O |
| D-17963 | Me(R) | single bond | H | *c*-Pr[1-S(=O)₂CF₃] | C(=O)Me | Me | C(=O)Me | O |
| D-17964 | Me(S) | single bond | H | *c*-Pr[1-S(=O)₂CF₃] | C(=O)Me | Me | C(=O)Me | O |
| D-17965 | H | single bond | Me | *c* -Pr[1-(5,6-dihydro-1 ,4,2-dioxazin-3-yl)] | C(=O)Me | Cl | C(=O)Me | O |
| D-17966 | Me | single bond | H | *c* -Pr[1-(5,6-dihydro-1 ,4,2-dioxazin-3-yl)] | C(=O)Me | Cl | C(=O)Me | O |
| D-17967 | Me(R) | single bond | H | *c* -Pr[1-(5,6-dihydro-1 ,4,2-dioxazin-3-yl)] | C(=O)Me | Cl | C(=O)Me | O |
| D-17968 | Me(S) | single bond | H | *c* -Pr[1-(5,6-dihydro-1 ,4,2-dioxazin-3-yl)] | C(=O)Me | Cl | C(=O)Me | O |
| D-17969 | H | single bond | Me | *c* -Pr[1-(5,6-dihydro-1 ,4,2-dioxazin-3-yl)] | C(=O)Me | Me | C(=O)Me | O |
| D-17970 | Me | single bond | H | *c* -Pr[1-(5,6-dihydro-1 ,4,2-dioxazin-3-yl)] | C(=O)Me | Me | C(=O)Me | O |

**[Table 1055]**

| Compound Number | Y | W | Z | R¹ | R² | R³ | R¹⁰ | A |
|---|---|---|---|---|---|---|---|---|
| D-17971 | Me(R) | single bond | H | *c*-Pr[1-(5,6-dihydro-1,4,2-dioxazin-3-yl)] | C(=O)Me | Me | C(=O)Me | O |
| D-17972 | Me(S) | single bond | H | *c*-Pr[1-(5,6-dihydro-1,4,2-dioxazin-3-yl)] | C(=O)Me | Me | C(=O)Me | O |
| D-17973 | H | single bond | Me | *c*-Pr[1-(5-hydroxy-1,3,4-oxadiazol-2-yl)] | C(=O)Me | Cl | C(=O)Me | O |
| D-17974 | Me | single bond | H | *c*-Pr[1-(5-hydroxy-1,3,4-oxadiazol-2-yl)] | C(=O)Me | Cl | C(=O)Me | O |
| D-17975 | Me(R) | single bond | H | *c*-Pr[1-(5-hydroxy-1,3,4-oxadiazol-2-yl)] | C(=O)Me | Cl | C(=O)Me | O |
| D-17976 | Me(S) | single bond | H | *c*-Pr[1-(5-hydroxy-1,3,4-oxadiazol-2-yl)] | C(=O)Me | Cl | C(=O)Me | O |
| D-17977 | H | single bond | Me | *c*-Pr[1-(5-hydroxy-1,3,4-oxadiazol-2-yl)] | C(=O)Me | Me | C(=O)Me | O |
| D-17978 | Me | single bond | H | *c*-Pr[1-(5-hydroxy-1,3,4-oxadiazol-2-yl)] | C(=O)Me | Me | C(=O)Me | O |
| D-17979 | Me(R) | single bond | H | *c*-Pr[1-(5-hydroxy-1,3,4-oxadiazol-2-yl)] | C(=O)Me | Me | C(=O)Me | O |
| D-17980 | Me(S) | single bond | H | *c*-Pr[1-(5-hydroxy-1,3,4-oxadiazol-2-yl)] | C(=O)Me | Me | C(=O)Me | O |
| D-17981 | H | single bond | Me | *c* -Pr[1-(pyrimidin-2-yl)] | C(=O)Me | Cl | C(=O)Me | O |
| D-17982 | Me | single bond | H | *c* -Pr[1-(pyrimidin-2-yl)] | C(=O)Me | Cl | C(=O)Me | O |
| D-17983 | Me(R) | single bond | H | *c* -Pr[1-(pyrimidin-2-yl)] | C(=O)Me | Cl | C(=O)Me | O |
| D-17984 | Me(S) | single bond | H | *c* -Pr[1-(pyrimidin-2-yl)] | C(=O)Me | Cl | C(=O)Me | O |
| D-17985 | H | single bond | Me | *c* -Pr[1-(pyrimidin-2-yl)] | C(=O)Me | Me | C(=O)Me | O |
| D-17986 | Me | single bond | H | *c* -Pr[1-(pyrimidin-2-yl)] | C(=O)Me | Me | C(=O)Me | O |
| D-17987 | Me(R) | single bond | H | *c* -Pr[1-(pyrimidin-2-yl)] | C(=O)Me | Me | C(=O)Me | O |
| D-17988 | Me(S) | single bond | H | *c* -Pr[1-(pyrimidin-2-yl)] | C(=O)Me | Me | C(=O)Me | O |
| D-17989 | H | single bond | Me | *t*-Bu | C(=O)Me | Cl | C(=O)Me | O |
| D-17990 | Me | single bond | H | *t*-Bu | C(=O)Me | Cl | C(=O)Me | O |
| D-17991 | Me(R) | single bond | H | *t*-Bu | C(=O)Me | Cl | C(=O)Me | O |
| D-17992 | Me(S) | single bond | H | *t*-Bu | C(=O)Me | Cl | C(=O)Me | O |
| D-17993 | H | single bond | Me | *t*-Bu | C(=O)Me | Me | C(=O)Me | O |
| D-17994 | Me | single bond | H | *t*-Bu | C(=O)Me | Me | C(=O)Me | O |
| D-17995 | Me(R) | single bond | H | *t*-Bu | C(=O)Me | Me | C(=O)Me | O |
| D-17996 | Me(S) | single bond | H | *t*-Bu | C(=O)Me | Me | C(=O)Me | O |
| D-17997 | H | single bond | Me | *c*-Bu | C(=O)Me | Cl | C(=O)Me | O |
| D-17998 | Me | single bond | H | *c*-Bu | C(=O)Me | Cl | C(=O)Me | O |
| D-17999 | Me(R) | single bond | H | *c*-Bu | C(=O)Me | Cl | C(=O)Me | O |
| D-18000 | Me(S) | single bond | H | *c*-Bu | C(=O)Me | Cl | C(=O)Me | O |
| D-18001 | H | single bond | Me | *c*-Bu | C(=O)Me | Me | C(=O)Me | O |
| D-18002 | Me | single bond | H | *c*-Bu | C(=O)Me | Me | C(=O)Me | O |
| D-18003 | Me(R) | single bond | H | *c*-Bu | C(=O)Me | Me | C(=O)Me | O |
| D-18004 | Me(S) | single bond | H | *c*-Bu | C(=O)Me | Me | C(=O)Me | O |
| D-18005 | H | single bond | Me | *c*-Bu(1-CN) | C(=O)Me | Cl | C(=O)Me | O |
| D-18006 | Me | single bond | H | *c*-Bu(1-CN) | C(=O)Me | Cl | C(=O)Me | O |
| D-18007 | Me(R) | single bond | H | *c*-Bu(1-CN) | C(=O)Me | Cl | C(=O)Me | O |
| D-18008 | Me(S) | single bond | H | *c*-Bu(1-CN) | C(=O)Me | Cl | C(=O)Me | O |
| D-18009 | H | single bond | Me | *c*-Bu(1-CN) | C(=O)Me | Me | C(=O)Me | O |
| D-18010 | Me | single bond | H | *c*-Bu(1-CN) | C(=O)Me | Me | C(=O)Me | O |
| D-18011 | Me(R) | single bond | H | *c*-Bu(1-CN) | C(=O)Me | Me | C(=O)Me | O |
| D-18012 | Me(S) | single bond | H | *c*-Bu(1-CN) | C(=O)Me | Me | C(=O)Me | O |
| D-18013 | H | single bond | Me | *c*-Bu(3,3-F₂) | C(=O)Me | Cl | C(=O)Me | O |
| D-18014 | Me | single bond | H | *c*-Bu(3,3-F₂) | C(=O)Me | Cl | C(=O)Me | O |

**[Table 1056]**

| Compound Number | Y | W | Z | R¹ | R² | R³ | R¹⁰ | A |
|---|---|---|---|---|---|---|---|---|
| D-18015 | Me(R) | single bond | H | *c*-Bu(3,3-F₂) | C(=O)Me | Cl | C(=O)Me | O |
| D-18016 | Me(S) | single bond | H | *c*-Bu(3,3-F₂) | C(=O)Me | Cl | C(=O)Me | O |
| D-18017 | H | single bond | Me | *c*-Bu(3,3-F₂) | C(=O)Me | Me | C(=O)Me | O |
| D-18018 | Me | single bond | H | *c*-Bu(3,3-F₂) | C(=O)Me | Me | C(=O)Me | O |
| D-18019 | Me(R) | single bond | H | *c*-Bu(3,3-F₂) | C(=O)Me | Me | C(=O)Me | O |
| D-18020 | Me(S) | single bond | H | *c*-Bu(3,3-F₂) | C(=O)Me | Me | C(=O)Me | O |
| D-18021 | H | single bond | Me | oxetan-3-yl | C(=O)Me | Cl | C(=O)Me | O |
| D-18022 | Me | single bond | H | oxetan-3-yl | C(=O)Me | Cl | C(=O)Me | O |
| D-18023 | Me(R) | single bond | H | oxetan-3-yl | C(=O)Me | Cl | C(=O)Me | O |
| D-18024 | Me(S) | single bond | H | oxetan-3-yl | C(=O)Me | Cl | C(=O)Me | O |
| D-18025 | H | single bond | Me | oxetan-3-yl | C(=O)Me | Me | C(=O)Me | O |
| D-18026 | Me | single bond | H | oxetan-3-yl | C(=O)Me | Me | C(=O)Me | O |
| D-18027 | Me(R) | single bond | H | oxetan-3-yl | C(=O)Me | Me | C(=O)Me | O |
| D-18028 | Me(S) | single bond | H | oxetan-3-yl | C(=O)Me | Me | C(=O)Me | O |
| D-18029 | H | single bond | Me | oxetan(3-CN)-3-yl | C(=O)Me | Cl | C(=O)Me | O |
| D-18030 | Me | single bond | H | oxetan(3-CN)-3-yl | C(=O)Me | Cl | C(=O)Me | O |
| D-18031 | Me(R) | single bond | H | oxetan(3-CN)-3-yl | C(=O)Me | Cl | C(=O)Me | O |
| D-18032 | Me(S) | single bond | H | oxetan(3-CN)-3-yl | C(=O)Me | Cl | C(=O)Me | O |
| D-18033 | H | single bond | Me | oxetan(3-CN)-3-yl | C(=O)Me | Me | C(=O)Me | O |
| D-18034 | Me | single bond | H | oxetan(3-CN)-3-yl | C(=O)Me | Me | C(=O)Me | O |
| D-18035 | Me(R) | single bond | H | oxetan(3-CN)-3-yl | C(=O)Me | Me | C(=O)Me | O |
| D-18036 | Me(S) | single bond | H | oxetan(3-CN)-3-yl | C(=O)Me | Me | C(=O)Me | O |
| D-18037 | H | single bond | Me | azetidin[1-C(=O)Me]-3-yl | C(=O)Me | Cl | C(=O)Me | O |
| D-18038 | Me | single bond | H | azetidin[1-C(=O)Me]-3-yl | C(=O)Me | Cl | C(=O)Me | O |
| D-18039 | Me(R) | single bond | H | azetidin[1-C(=O)Me]-3-yl | C(=O)Me | Cl | C(=O)Me | O |
| D-18040 | Me(S) | single bond | H | azetidin[1-C(=O)Me]-3-yl | C(=O)Me | Cl | C(=O)Me | O |
| D-18041 | H | single bond | Me | azetidin[1-C(=O)Me]-3-yl | C(=O)Me | Me | C(=O)Me | O |
| D-18042 | Me | single bond | H | azetidin[1-C(=O)Me]-3-yl | C(=O)Me | Me | C(=O)Me | O |
| D-18043 | Me(R) | single bond | H | azetidin[1-C(=O)Me]-3-yl | C(=O)Me | Me | C(=O)Me | O |
| D-18044 | Me(S) | single bond | H | azetidin[1-C(=O)Me]-3-yl | C(=O)Me | Me | C(=O)Me | O |
| D-18045 | H | single bond | Me | azetidin[1-S(=O)₂Me]-3-yl | C(=O)Me | Cl | C(=O)Me | O |
| D-18046 | Me | single bond | H | azetidin[1-S(=O)₂Me]-3-yl | C(=O)Me | Cl | C(=O)Me | O |
| D-18047 | Me(R) | single bond | H | azetidin[1-S(=O)₂Me]-3-yl | C(=O)Me | Cl | C(=O)Me | O |
| D-18048 | Me(S) | single bond | H | azetidin[1-S(=O)₂Me]-3-yl | C(=O)Me | Cl | C(=O)Me | O |
| D-18049 | H | single bond | Me | azetidin[1-S(=O)₂Me]-3-yl | C(=O)Me | Me | C(=O)Me | O |
| D-18050 | Me | single bond | H | azetidin[1-S(=O)₂Me]-3-yl | C(=O)Me | Me | C(=O)Me | O |
| D-18051 | Me(R) | single bond | H | azetidin[1-S(=O)₂Me]-3-yl | C(=O)Me | Me | C(=O)Me | O |
| D-18052 | Me(S) | single bond | H | azetidin[1-S(=O)₂Me]-3-yl | C(=O)Me | Me | C(=O)Me | O |
| D-18053 | H | single bond | Me | azetidin[1-S(=O)₂CHF₂]-3-yl | C(=O)Me | Cl | C(=O)Me | O |
| D-18054 | Me | single bond | H | azetidin[1-S(=O)₂CHF₂]-3-yl | C(=O)Me | Cl | C(=O)Me | O |
| D-18055 | Me(R) | single bond | H | azetidin[1-S(=O)₂CHF₂]-3-yl | C(=O)Me | Cl | C(=O)Me | O |
| D-18056 | Me(S) | single bond | H | azetidin[1-S(=O)₂CHF₂]-3-yl | C(=O)Me | Cl | C(=O)Me | O |
| D-18057 | H | single bond | Me | azetidin[1-S(=O)₂CHF₂]-3-yl | C(=O)Me | Me | C(=O)Me | O |
| D-18058 | Me | single bond | H | azetidin[1-S(=O)₂CHF₂]-3-yl | C(=O)Me | Me | C(=O)Me | O |

**[Table 1057]**

| Compound Number | Y | W | Z | R¹ | R² | R³ | R¹⁰ | A |
|---|---|---|---|---|---|---|---|---|
| D-18059 | Me(R) | single bond | H | azetidin[1-S(=O)₂CHF₂]-3-yl | C(=O)Me | Me | C(=O)Me | O |
| D-18060 | Me(S) | single bond | H | azetidin[1-S(=O)₂CHF₂]-3-yl | C(=O)Me | Me | C(=O)Me | O |
| D-18061 | H | single bond | Me | c-Pen | C(=O)Me | Cl | C(=O)Me | O |
| D-18062 | Me | single bond | H | c-Pen | C(=O)Me | Cl | C(=O)Me | O |
| D-18063 | Me(R) | single bond | H | c-Pen | C(=O)Me | Cl | C(=O)Me | O |
| D-18064 | Me(S) | single bond | H | *c*-Pen | C(=O)Me | Cl | C(=O)Me | O |
| D-18065 | H | single bond | Me | c-Pen | C(=O)Me | Me | C(=O)Me | O |
| D-18066 | Me | single bond | H | c-Pen | C(=O)Me | Me | C(=O)Me | O |
| D-18067 | Me(R) | single bond | H | c-Pen | C(=O)Me | Me | C(=O)Me | O |
| D-18068 | Me(S) | single bond | H | c-Pen | C(=O)Me | Me | C(=O)Me | O |
| D-18069 | H | single bond | Me | tetrahydrofuran-3-yl | C(=O)Me | Cl | C(=O)Me | O |
| D-18070 | Me | single bond | H | tetrahydrofuran-3-yl | C(=O)Me | Cl | C(=O)Me | O |
| D-18071 | Me(R) | single bond | H | tetrahydrofuran-3-yl | C(=O)Me | Cl | C(=O)Me | O |
| D-18072 | Me(S) | single bond | H | tetrahydrofuran-3-yl | C(=O)Me | Cl | C(=O)Me | O |
| D-18073 | H | single bond | Me | tetrahydrofuran-3-yl | C(=O)Me | Me | C(=O)Me | O |
| D-18074 | Me | single bond | H | tetrahydrofuran-3-yl | C(=O)Me | Me | C(=O)Me | O |
| D-18075 | Me(R) | single bond | H | tetrahydrofuran-3-yl | C(=O)Me | Me | C(=O)Me | O |
| D-18076 | Me(S) | single bond | H | tetrahydrofuran-3-yl | C(=O)Me | Me | C(=O)Me | O |
| D-18077 | H | single bond | Me | tetrahydrothiophen-3-yl | C(=O)Me | Cl | C(=O)Me | O |
| D-18078 | Me | single bond | H | tetrahydrothiophen-3-yl | C(=O)Me | Cl | C(=O)Me | O |
| D-18079 | Me(R) | single bond | H | tetrahydrothiophen-3-yl | C(=O)Me | Cl | C(=O)Me | O |
| D-18080 | Me(S) | single bond | H | tetrahydrothiophen-3-yl | C(=O)Me | Cl | C(=O)Me | O |
| D-18081 | H | single bond | Me | tetrahydrothiophen-3-yl | C(=O)Me | Me | C(=O)Me | O |
| D-18082 | Me | single bond | H | tetrahydrothiophen-3-yl | C(=O)Me | Me | C(=O)Me | O |
| D-18083 | Me(R) | single bond | H | tetrahydrothiophen-3-yl | C(=O)Me | Me | C(=O)Me | O |
| D-18084 | Me(S) | single bond | H | tetrahydrothiophen-3-yl | C(=O)Me | Me | C(=O)Me | O |
| D-18085 | H | single bond | Me | *c*-Pen(1-CN) | C(=O)Me | Cl | C(=O)Me | O |
| D-18086 | Me | single bond | H | *c*-Pen(1-CN) | C(=O)Me | Cl | C(=O)Me | O |
| D-18087 | Me(R) | single bond | H | *c*-Pen(1-CN) | C(=O)Me | Cl | C(=O)Me | O |
| D-18088 | Me(S) | single bond | H | *c*-Pen(1-CN) | C(=O)Me | Cl | C(=O)Me | O |
| D-18089 | H | single bond | Me | *c*-Pen(1-CN) | C(=O)Me | Me | C(=O)Me | O |
| D-18090 | Me | single bond | H | *c*-Pen(1-CN) | C(=O)Me | Me | C(=O)Me | O |
| D-18091 | Me(R) | single bond | H | *c*-Pen(1-CN) | C(=O)Me | Me | C(=O)Me | O |
| D-18092 | Me(S) | single bond | H | *c*-Pen(1-CN) | C(=O)Me | Me | C(=O)Me | O |
| D-18093 | H | single bond | Me | tetrahydrofuran(3-CN)-3-yl | C(=O)Me | Cl | C(=O)Me | O |
| D-18094 | Me | single bond | H | tetrahydrofuran(3-CN)-3-yl | C(=O)Me | Cl | C(=O)Me | O |
| D-18095 | Me(R) | single bond | H | tetrahydrofuran(3-CN)-3-yl | C(=O)Me | Cl | C(=O)Me | O |
| D-18096 | Me(S) | single bond | H | tetrahydrofuran(3-CN)-3-yl | C(=O)Me | Cl | C(=O)Me | O |
| D-18097 | H | single bond | Me | tetrahydrofuran(3-CN)-3-yl | C(=O)Me | Me | C(=O)Me | O |
| D-18098 | Me | single bond | H | tetrahydrofuran(3-CN)-3-yl | C(=O)Me | Me | C(=O)Me | O |
| D-18099 | Me(R) | single bond | H | tetrahydrofuran(3-CN)-3-yl | C(=O)Me | Me | C(=O)Me | O |
| D-18100 | Me(S) | single bond | H | tetrahydrofuran(3-CN)-3-yl | C(=O)Me | Me | C(=O)Me | O |
| D-18101 | H | single bond | Me | tetrahydrothiophen(3-CN)-3-yl | C(=O)Me | Cl | C(=O)Me | O |
| D-18102 | Me | single bond | H | tetrahydrothiophen(3-CN)-3-yl | C(=O)Me | Cl | C(=O)Me | O |

**[Table 1058]**

| Compound Number | Y | W | Z | R¹ | R² | R³ | R¹⁰ | A |
|---|---|---|---|---|---|---|---|---|
| D-18103 | Me(R) | single bond | H | tetrahydrothiophen(3-CN)-3-yl | C(=O)Me | Cl | C(=O)Me | O |
| D-18104 | Me(S) | single bond | H | tetrahydrothiophen(3-CN)-3-yl | C(=O)Me | Cl | C(=O)Me | O |
| D-18105 | H | single bond | Me | tetrahydrothiophen(3-CN)-3-yl | C(=O)Me | Me | C(=O)Me | O |
| D-18106 | Me | single bond | H | tetrahydrothiophen(3-CN)-3-yl | C(=O)Me | Me | C(=O)Me | O |
| D-18107 | Me(R) | single bond | H | tetrahydrothiophen(3-CN)-3-yl | C(=O)Me | Me | C(=O)Me | O |
| D-18108 | Me(S) | single bond | H | tetrahydrothiophen(3-CN)-3-yl | C(=O)Me | Me | C(=O)Me | O |
| D-18109 | H | single bond | Me | bicyclo[1.1.1]pertan-1-yl | C(=O)Me | Cl | C(=O)Me | O |
| D-18110 | Me | single bond | H | bicyclo[1.1.1]pentan-1-yl | C(=O)Me | Cl | C(=O)Me | O |
| D-18111 | Me(R) | single bond | H | bicyclo[1.1.1]pentan-1-yl | C(=O)Me | Cl | C(=O)Me | O |
| D-18112 | Me(S) | single bond | H | bicyclo[1.1.1]pertan-1-yl | C(=O)Me | Cl | C(=O)Me | O |
| D-18113 | H | single bond | Me | bicyclo[1.1.1]pentan-1-yl | C(=O)Me | Me | C(=O)Me | O |
| D-18114 | Me | single bond | H | bicyclo[1.1.1]pentan-1-yl | C(=O)Me | Me | C(=O)Me | O |
| D-18115 | Me(R) | single bond | H | bicyclo[1.1.1]pertan-1-yl | C(=O)Me | Me | C(=O)Me | O |
| D-18116 | Me(S) | single bond | H | bicyclo[1.1.1]pentan-1-yl | C(=O)Me | Me | C(=O)Me | O |
| D-18117 | H | single bond | Me | *c*-Hex | C(=O)Me | Cl | C(=O)Me | O |
| D-18118 | Me | single bond | H | *c*-Hex | C(=O)Me | Cl | C(=O)Me | O |
| D-18119 | Me(R) | single bond | H | *c*-Hex | C(=O)Me | Cl | C(=O)Me | O |
| D-18120 | Me(S) | single bond | H | *c*-Hex | C(=O)Me | Cl | C(=O)Me | O |
| D-18121 | H | single bond | Me | *c*-Hex | C(=O)Me | Me | C(=O)Me | O |
| D-18122 | Me | single bond | H | *c*-Hex | C(=O)Me | Me | C(=O)Me | O |
| D-18123 | Me(R) | single bond | H | *c*-Hex | C(=O)Me | Me | C(=O)Me | O |
| D-18124 | Me(S) | single bond | H | *c*-Hex | C(=O)Me | Me | C(=O)Me | O |
| D-18125 | H | single bond | Me | tetrahydropyran-4-yl | C(=O)Me | Cl | C(=O)Me | O |
| D-18126 | Me | single bond | H | tetrahydropyran-4-yl | C(=O)Me | Cl | C(=O)Me | O |
| D-18127 | Me(R) | single bond | H | tetrahydropyran-4-yl | C(=O)Me | Cl | C(=O)Me | O |
| D-18128 | Me(S) | single bond | H | tetrahydropyran-4-yl | C(=O)Me | Cl | C(=O)Me | O |
| D-18129 | H | single bond | Me | tetrahydropyran-4-yl | C(=O)Me | Me | C(=O)Me | O |
| D-18130 | Me | single bond | H | tetrahydropyran-4-yl | C(=O)Me | Me | C(=O)Me | O |
| D-18131 | Me(R) | single bond | H | tetrahydropyran-4-yl | C(=O)Me | Me | C(=O)Me | O |
| D-18132 | Me(S) | single bond | H | tetrahydropyran-4-yl | C(=O)Me | Me | C(=O)Me | O |
| D-18133 | H | single bond | Me | tetrahydrothiopyran-4-yl | C(=O)Me | Cl | C(=O)Me | O |
| D-18134 | Me | single bond | H | tetrahydrothiopyran-4-yl | C(=O)Me | Cl | C(=O)Me | O |
| D-18135 | Me(R) | single bond | H | tetrahydrothiopyran-4-yl | C(=O)Me | Cl | C(=O)Me | O |
| D-18136 | Me(S) | single bond | H | tetrahydrothiopyran-4-yl | C(=O)Me | Cl | C(=O)Me | O |
| D-18137 | H | single bond | Me | tetrahydrothiopyran-4-yl | C(=O)Me | Me | C(=O)Me | O |
| D-18138 | Me | single bond | H | tetrahydrothiopyran-4-yl | C(=O)Me | Me | C(=O)Me | O |
| D-18139 | Me(R) | single bond | H | tetrahydrothiopyran-4-yl | C(=O)Me | Me | C(=O)Me | O |
| D-18140 | Me(S) | single bond | H | tetrahydrothiopyran-4-yl | C(=O)Me | Me | C(=O)Me | O |
| D-18141 | H | single bond | Me | *c*-Hex(1-CN) | C(=O)Me | Cl | C(=O)Me | O |
| D-18142 | Me | single bond | H | *c*-Hex(1-CN) | C(=O)Me | Cl | C(=O)Me | O |
| D-18143 | Me(R) | single bond | H | *c*-Hex(1-CN) | C(=O)Me | Cl | C(=O)Me | O |
| D-18144 | Me(S) | single bond | H | *c*-Hex(1-CN) | C(=O)Me | Cl | C(=O)Me | O |
| D-18145 | H | single bond | Me | *c*-Hex(1-CN) | C(=O)Me | Me | C(=O)Me | O |
| D-18146 | Me | single bond | H | *c*-Hex(1-CN) | C(=O)Me | Me | C(=O)Me | O |

**[Table 1059]**

| Compound Number | Y | W | Z | R¹ | R² | R³ | R¹⁰ | A |
|---|---|---|---|---|---|---|---|---|
| D-18147 | Me(R) | single bond | H | *c*-Hex(1-CN) | C(=O)Me | Me | C(=O)Me | O |
| D-18148 | Me(S) | single bond | H | *c*-Hex(1-CN) | C(=O)Me | Me | C(=O)Me | O |
| D-18149 | H | single bond | Me | tetrahydropyran(4-CN)-4-yl | C(=O)Me | Cl | C(=O)Me | O |
| D-18150 | Me | single bond | H | tetrahydropyran(4-CN)-4-yl | C(=O)Me | Cl | C(=O)Me | O |
| D-18151 | Me(R) | single bond | H | tetrahydropyran(4-CN)-4-yl | C(=O)Me | Cl | C(=O)Me | O |
| D-18152 | Me(S) | single bond | H | tetrahydropyran(4-CN)-4-yl | C(=O)Me | Cl | C(=O)Me | O |
| D-18153 | H | single bond | Me | tetrahydropyran(4-CN)-4-yl | C(=O)Me | Me | C(=O)Me | O |
| D-18154 | Me | single bond | H | tetrahydropyran(4-CN)-4-yl | C(=O)Me | Me | C(=O)Me | O |
| D-18155 | Me(R) | single bond | H | tetrahydropyran(4-CN)-4-yl | C(=O)Me | Me | C(=O)Me | O |
| D-18156 | Me(S) | single bond | H | tetrahydropyran(4-CN)-4-yl | C(=O)Me | Me | C(=O)Me | O |
| D-18157 | H | single bond | Me | tetrahydrothiopyran(4-CN)-4-yl | C(=O)Me | Cl | C(=O)Me | O |
| D-18158 | Me | single bond | H | tetrahydrothiopyran(4-CN)-4-yl | C(=O)Me | Cl | C(=O)Me | O |
| D-18159 | Me(R) | single bond | H | tetrahydrothiopyran(4-CN)-4-yl | C(=O)Me | Cl | C(=O)Me | O |
| D-18160 | Me(S) | single bond | H | tetrahydrothiopyran(4-CN)-4-yl | C(=O)Me | Cl | C(=O)Me | O |
| D-18161 | H | single bond | Me | tetrahydrothiopyran(4-CN)-4-yl | C(=O)Me | Me | C(=O)Me | O |
| D-18162 | Me | single bond | H | tetrahydrothiopyran(4-CN)-4-yl | C(=O)Me | Me | C(=O)Me | O |
| D-18163 | Me(R) | single bond | H | tetrahydrothiopyran(4-CN)-4-yl | C(=O)Me | Me | C(=O)Me | O |
| D-18164 | Me(S) | single bond | H | tetrahydrothiopyran(4-CN)-4-yl | C(=O)Me | Me | C(=O)Me | O |
| D-18165 | H | single bond | Me | CH₂(*c*-Pr) | C(=O)Me | Cl | C(=O)Me | O |
| D-18166 | Me | single bond | H | CH₂(*c*-Pr) | C(=O)Me | Cl | C(=O)Me | O |
| D-18167 | Me(R) | single bond | H | CH₂(*c*-Pr) | C(=O)Me | Cl | C(=O)Me | O |
| D-18168 | Me(S) | single bond | H | CH₂(*c*-Pr) | C(=O)Me | Cl | C(=O)Me | O |
| D-18169 | H | single bond | Me | CH₂(*c*-Pr) | C(=O)Me | Me | C(=O)Me | O |
| D-18170 | Me | single bond | H | CH₂(*c*-Pr) | C(=O)Me | Me | C(=O)Me | O |
| D-18171 | Me(R) | single bond | H | CH₂(*c*-Pr) | C(=O)Me | Me | C(=O)Me | O |
| D-18172 | Me(S) | single bond | H | CH₂(*c*-Pr) | C(=O)Me | Me | C(=O)Me | O |
| D-18173 | H | single bond | Me | CH₂[*c*-Pr(1-CN)] | C(=O)Me | Cl | C(=O)Me | O |
| D-18174 | Me | single bond | H | CH₂[*c*-Pr(1-CN)] | C(=O)Me | Cl | C(=O)Me | O |
| D-18175 | Me(R) | single bond | H | CH₂[*c*-Pr(1-CN)] | C(=O)Me | Cl | C(=O)Me | O |
| D-18176 | Me(S) | single bond | H | CH₂[*c*-Pr(1-CN)] | C(=O)Me | Cl | C(=O)Me | O |
| D-18177 | H | single bond | Me | CH₂[*c*-Pr(1-CN)] | C(=O)Me | Me | C(=O)Me | O |
| D-18178 | Me | single bond | H | CH₂[*c*-Pr(1-CN)] | C(=O)Me | Me | C(=O)Me | O |
| D-18179 | Me(R) | single bond | H | CH₂[*c*-Pr(1-CN)] | C(=O)Me | Me | C(=O)Me | O |
| D-18180 | Me(S) | single bond | H | CH₂[*c*-Pr(1-CN)] | C(=O)Me | Me | C(=O)Me | O |
| D-18181 | H | single bond | Me | CH(Me)(*c*-Pr) | C(=O)Me | Cl | C(=O)Me | O |
| D-18182 | Me | single bond | H | CH(Me)(*c*-Pr) | C(=O)Me | Cl | C(=O)Me | O |
| D-18183 | Me(R) | single bond | H | CH(Me)(*c*-Pr) | C(=O)Me | Cl | C(=O)Me | O |
| D-18184 | Me(S) | single bond | H | CH(Me)(*c*-Pr) | C(=O)Me | Cl | C(=O)Me | O |
| D-18185 | H | single bond | Me | CH(Me)(*c*-Pr) | C(=O)Me | Me | C(=O)Me | O |
| D-18186 | Me | single bond | H | CH(Me)(*c*-Pr) | C(=O)Me | Me | C(=O)Me | O |
| D-18187 | Me(R) | single bond | H | CH(Me)(*c*-Pr) | C(=O)Me | Me | C(=O)Me | O |
| D-18188 | Me(S) | single bond | H | CH(Me)(*c*-Pr) | C(=O)Me | Me | C(=O)Me | O |
| D-18189 | H | single bond | Me | CH(Me)CH₂OMe | C(=O)Me | Cl | C(=O)Me | O |
| D-18190 | Me | single bond | H | CH(Me)CH₂OMe | C(=O)Me | Cl | C(=O)Me | O |

**[Table 1060]**

| Compound Number | Y | W | Z | R¹ | R² | R³ | R¹⁰ | A |
|---|---|---|---|---|---|---|---|---|
| D-18191 | Me(R) | single bond | H | CH(Me)CH₂OMe | C(=O)Me | Cl | C(=O)Me | O |
| D-18192 | Me(S) | single bond | H | CH(Me)CH₂OMe | C(=O)Me | Cl | C(=O)Me | O |
| D-18193 | H | single bond | Me | CH(Me)CH₂OMe | C(=O)Me | Me | C(=O)Me | O |
| D-18194 | Me | single bond | H | CH(Me)CH₂OMe | C(=O)Me | Me | C(=O)Me | O |
| D-18195 | Me(R) | single bond | H | CH(Me)CH₂OMe | C(=O)Me | Me | C(=O)Me | O |
| D-18196 | Me(S) | single bond | H | CH(Me)CH₂OMe | C(=O)Me | Me | C(=O)Me | O |
| D-18197 | H | single bond | Me | CH₂CH=CH₂ | C(=O)Me | Cl | C(=O)Me | O |
| D-18198 | Me | single bond | H | CH₂CH=CH₂ | C(=O)Me | Cl | C(=O)Me | O |
| D-18199 | Me(R) | single bond | H | CH₂CH=CH₂ | C(=O)Me | Cl | C(=O)Me | O |
| D-18200 | Me(S) | single bond | H | CH₂CH=CH₂ | C(=O)Me | Cl | C(=O)Me | O |
| D-18201 | H | single bond | Me | CH₂CH=CH₂ | C(=O)Me | Me | C(=O)Me | O |
| D-18202 | Me | single bond | H | CH₂CH=CH₂ | C(=O)Me | Me | C(=O)Me | O |
| D-18203 | Me(R) | single bond | H | CH₂CH=CH₂ | C(=O)Me | Me | C(=O)Me | O |
| D-18204 | Me(S) | single bond | H | CH₂CH=CH₂ | C(=O)Me | Me | C(=O)Me | O |
| D-18205 | H | single bond | Me | CH₂C≡CH | C(=O)Me | Cl | C(=O)Me | O |
| D-1 8206 | Me | single bond | H | CH₂C≡CH | C(=O)Me | Cl | C(=O)Me | O |
| D-18207 | Me(R) | single bond | H | CH₂C≡CH | C(=O)Me | Cl | C(=O)Me | O |
| D-18208 | Me(S) | single bond | H | CH₂C≡CH | C(=O)Me | Cl | C(=O)Me | O |
| D-18209 | H | single bond | Me | CH₂C≡CH | C(=O)Me | Me | C(=O)Me | O |
| D-18210 | Me | single bond | H | CH₂C≡CH | C(=O)Me | Me | C(=O)Me | O |
| D-18211 | Me(R) | single bond | H | CH₂C≡CH | C(=O)Me | Me | C(=O)Me | O |
| D-18212 | Me(S) | single bond | H | CH₂C≡CH | C(=O)Me | Me | C(=O)Me | O |
| D-18213 | H | single bond | Me | CH₂CN | C(=O)Me | Cl | C(=O)Me | O |
| D-18214 | Me | single bond | H | CH₂CN | C(=O)Me | Cl | C(=O)Me | O |
| D-18215 | Me(R) | single bond | H | CH₂CN | C(=O)Me | Cl | C(=O)Me | O |
| D-18216 | Me(S) | single bond | H | CH₂CN | C(=O)Me | Cl | C(=O)Me | O |
| D-18217 | H | single bond | Me | CH₂CN | C(=O)Me | Me | C(=O)Me | O |
| D-18218 | Me | single bond | H | CH₂CN | C(=O)Me | Me | C(=O)Me | O |
| D-18219 | Me(R) | single bond | H | CH₂CN | C(=O)Me | Me | C(=O)Me | O |
| D-18220 | Me(S) | single bond | H | CH₂CN | C(=O)Me | Me | C(=O)Me | O |
| D-18221 | H | single bond | Me | CH(Me)CN | C(=O)Me | Cl | C(=O)Me | O |
| D-18222 | Me | single bond | H | CH(Me)CN | C(=O)Me | Cl | C(=O)Me | O |
| D-18223 | Me(R) | single bond | H | CH(Me)CN | C(=O)Me | Cl | C(=O)Me | O |
| D-18224 | Me(S) | single bond | H | CH(Me)CN | C(=O)Me | Cl | C(=O)Me | O |
| D-18225 | H | single bond | Me | CH(Me)CN | C(=O)Me | Me | C(=O)Me | O |
| D-18226 | Me | single bond | H | CH(Me)CN | C(=O)Me | Me | C(=O)Me | O |
| D-18227 | Me(R) | single bond | H | CH(Me)CN | C(=O)Me | Me | C(=O)Me | O |
| D-18228 | Me(S) | single bond | H | CH(Me)CN | C(=O)Me | Me | C(=O)Me | O |
| D-18229 | H | single bond | Me | C(Me)₂CN | C(=O)Me | Cl | C(=O)Me | O |
| D-18230 | Me | single bond | H | C(Me)₂CN | C(=O)Me | Cl | C(=O)Me | O |
| D-18231 | Me(R) | single bond | H | C(Me)₂CN | C(=O)Me | Cl | C(=O)Me | O |
| D-18232 | Me(S) | single bond | H | C(Me)₂CN | C(=O)Me | Cl | C(=O)Me | O |
| D-18233 | H | single bond | Me | C(Me)₂CN | C(=O)Me | Me | C(=O)Me | O |
| D-18234 | Me | single bond | H | C(Me)₂CN | C(=O)Me | Me | C(=O)Me | O |

**[Table 1061]**

| Compound Number | Y | W | Z | R¹ | R² | R³ | R¹⁰ | A |
|---|---|---|---|---|---|---|---|---|
| D-18235 | Me(R) | single bond | H | C(Me)₂CN | C(=O)Me | Me | C(=O)Me | O |
| D-18236 | Me(S) | single bond | H | C(Me)₂CN | C(=O)Me | Me | C(=O)Me | O |
| D-18237 | H | single bond | Me | C(Me)(*i*-Pr)CN | C(=O)Me | Cl | C(=O)Me | O |
| D-18238 | Me | single bond | H | C(Me)(*i*-Pr)CN | C(=O)Me | Cl | C(=O)Me | O |
| D-18239 | Me(R) | single bond | H | C(Me)(*i*-Pr)CN | C(=O)Me | Cl | C(=O)Me | O |
| D-18240 | Me(S) | single bond | H | C(Me)(*i*-Pr)CN | C(=O)Me | Cl | C(=O)Me | O |
| D-18241 | H | single bond | Me | C(Me)(*i*-Pr)CN | C(=O)Me | Me | C(=O)Me | O |
| D-18242 | Me | single bond | H | C(Me)(*i*-Pr)CN | C(=O)Me | Me | C(=O)Me | O |
| D-18243 | Me(R) | single bond | H | C(Me)(*i*-Pr)CN | C(=O)Me | Me | C(=O)Me | O |
| D-18244 | Me(S) | single bond | H | C(Me)(*i*-Pr)CN | C(=O)Me | Me | C(=O)Me | O |
| D-18245 | H | single bond | Me | OMe | C(=O)Me | Cl | C(=O)Me | O |
| D-18246 | Me | single bond | H | OMe | C(=O)Me | Cl | C(=O)Me | O |
| D-18247 | Me(R) | single bond | H | OMe | C(=O)Me | Cl | C(=O)Me | O |
| D-18248 | Me(S) | single bond | H | OMe | C(=O)Me | Cl | C(=O)Me | O |
| D-18249 | H | single bond | Me | OMe | C(=O)Me | Me | C(=O)Me | O |
| D-18250 | Me | single bond | H | OMe | C(=O)Me | Me | C(=O)Me | O |
| D-18251 | Me(R) | single bond | H | OMe | C(=O)Me | Me | C(=O)Me | O |
| D-18252 | Me(S) | single bond | H | OMe | C(=O)Me | Me | C(=O)Me | O |
| D-18253 | H | single bond | Me | CH=NOMe | C(=O)Me | Cl | C(=O)Me | O |
| D-18254 | Me | single bond | H | CH=NOMe | C(=O)Me | Cl | C(=O)Me | O |
| D-18255 | Me(R) | single bond | H | CH=NOMe | C(=O)Me | Cl | C(=O)Me | O |
| D-18256 | Me(S) | single bond | H | CH=NOMe | C(=O)Me | Cl | C(=O)Me | O |
| D-18257 | H | single bond | Me | CH=NOMe | C(=O)Me | Me | C(=O)Me | O |
| D-18258 | Me | single bond | H | CH=NOMe | C(=O)Me | Me | C(=O)Me | O |
| D-18259 | Me(R) | single bond | H | CH=NOMe | C(=O)Me | Me | C(=O)Me | O |
| D-18260 | Me(S) | single bond | H | CH=NOMe | C(=O)Me | Me | C(=O)Me | O |
| D-18261 | H | single bond | Me | morpholin-4-yl | C(=O)Me | Cl | C(=O)Me | O |
| D-18262 | Me | single bond | H | morpholin-4-yl | C(=O)Me | Cl | C(=O)Me | O |
| D-18263 | Me(R) | single bond | H | morpholin-4-yl | C(=O)Me | Cl | C(=O)Me | O |
| D-18264 | Me(S) | single bond | H | morpholin-4-yl | C(=O)Me | Cl | C(=O)Me | O |
| D-18265 | H | single bond | Me | morpholin-4-yl | C(=O)Me | Me | C(=O)Me | O |
| D-18266 | Me | single bond | H | morpholin-4-yl | C(=O)Me | Me | C(=O)Me | O |
| D-18267 | Me(R) | single bond | H | morpholin-4-yl | C(=O)Me | Me | C(=O)Me | O |
| D-18268 | Me(S) | single bond | H | morpholin-4-yl | C(=O)Me | Me | C(=O)Me | O |
| D-18269 | H | single bond | Me | Ph | C(=O)Me | Cl | C(=O)Me | O |
| D-18270 | Me | single bond | H | Ph | C(=O)Me | Cl | C(=O)Me | O |
| D-18271 | Me(R) | single bond | H | Ph | C(=O)Me | Cl | C(=O)Me | O |
| D-18272 | Me(S) | single bond | H | Ph | C(=O)Me | Cl | C(=O)Me | O |
| D-18273 | H | single bond | Me | Ph | C(=O)Me | Me | C(=O)Me | O |
| D-18274 | Me | single bond | H | Ph | C(=O)Me | Me | C(=O)Me | O |
| D-18275 | Me(R) | single bond | H | Ph | C(=O)Me | Me | C(=O)Me | O |
| D-18276 | Me(S) | single bond | H | Ph | C(=O)Me | Me | C(=O)Me | O |
| D-18277 | H | single bond | Me | CH₂Ph | C(=O)Me | Cl | C(=O)Me | O |
| D-18278 | Me | single bond | H | CH₂Ph | C(=O)Me | Cl | C(=O)Me | O |

**[Table 1062]**

| Compound Number | Y | W | Z | R¹ | R² | R³ | R¹⁰ | A |
|---|---|---|---|---|---|---|---|---|
| D-18279 | Me(R) | single bond | H | CH₂Ph | C(=O)Me | Cl | C(=O)Me | O |
| D-18280 | Me(S) | single bond | H | CH₂Ph | C(=O)Me | Cl | C(=O)Me | O |
| D-18281 | H | single bond | Me | CH₂Ph | C(=O)Me | Me | C(=O)Me | O |
| D-18282 | Me | single bond | H | CH₂Ph | C(=O)Me | Me | C(=O)Me | O |
| D-18283 | Me(R) | single bond | H | CH₂Ph | C(=O)Me | Me | C(=O)Me | O |
| D-18284 | Me(S) | single bond | H | CH₂Ph | C(=O)Me | Me | C(=O)Me | O |
| D-18285 | H | single bond | Me | thiazole-2-yl | C(=O)Me | Cl | C(=O)Me | O |
| D-18286 | Me | single bond | H | thiazole-2-yl | C(=O)Me | Cl | C(=O)Me | O |
| D-18287 | Me(R) | single bond | H | thiazole-2-yl | C(=O)Me | Cl | C(=O)Me | O |
| D-18288 | Me(S) | single bond | H | thiazole-2-yl | C(=O)Me | Cl | C(=O)Me | O |
| D-18289 | H | single bond | Me | thiazole-2-yl | C(=O)Me | Me | C(=O)Me | O |
| D-18290 | Me | single bond | H | thiazole-2-yl | C(=O)Me | Me | C(=O)Me | O |
| D-18291 | Me(R) | single bond | H | thiazole-2-yl | C(=O)Me | Me | C(=O)Me | O |
| D-18292 | Me(S) | single bond | H | thiazole-2-yl | C(=O)Me | Me | C(=O)Me | O |
| D-18293 | H | single bond | Me | 1-Me-pyrazol-3-yl | C(=O)Me | Cl | C(=O)Me | O |
| D-18294 | Me | single bond | H | 1-Me-pyrazol-3-yl | C(=O)Me | Cl | C(=O)Me | O |
| D-18295 | Me(R) | single bond | H | 1-Me-pyrazol-3-yl | C(=O)Me | Cl | C(=O)Me | O |
| D-18296 | Me(S) | single bond | H | 1-Me-pyrazol-3-yl | C(=O)Me | Cl | C(=O)Me | O |
| D-18297 | H | single bond | Me | 1-Me-pyrazol-3-yl | C(=O)Me | Me | C(=O)Me | O |
| D-18298 | Me | single bond | H | 1-Me-pyrazol-3-yl | C(=O)Me | Me | C(=O)Me | O |
| D-18299 | Me(R) | single bond | H | 1-Me-pyrazol-3-yl | C(=O)Me | Me | C(=O)Me | O |
| D-18300 | Me(S) | single bond | H | 1-Me-pyrazol-3-yl | C(=O)Me | Me | C(=O)Me | O |
| D-18301 | H | single bond | Me | 1-Me-pyrazol-5-yl | C(=O)Me | Cl | C(=O)Me | O |
| D-18302 | Me | single bond | H | 1-Me-pyrazol-5-yl | C(=O)Me | Cl | C(=O)Me | O |
| D-18303 | Me(R) | single bond | H | 1-Me-pyrazol-5-yl | C(=O)Me | Cl | C(=O)Me | O |
| D-18304 | Me(S) | single bond | H | 1-Me-pyrazol-5-yl | C(=O)Me | Cl | C(=O)Me | O |
| D-18305 | H | single bond | Me | 1-Me-pyrazol-5-yl | C(=O)Me | Me | C(=O)Me | O |
| D-18306 | Me | single bond | H | 1-Me-pyrazol-5-yl | C(=O)Me | Me | C(=O)Me | O |
| D-18307 | Me(R) | single bond | H | 1-Me-pyrazol-5-yl | C(=O)Me | Me | C(=O)Me | O |
| D-18308 | Me(S) | single bond | H | 1-Me-pyrazol-5-yl | C(=O)Me | Me | C(=O)Me | O |
| D-18309 | H | single bond | Me | CH₂(pyrimidin-2-yl) | C(=O)Me | Cl | C(=O)Me | O |
| D-18310 | Me | single bond | H | CH₂(pyrimidin-2-yl) | C(=O)Me | Cl | C(=O)Me | O |
| D-18311 | Me(R) | single bond | H | CH₂(pyrimidin-2-yl) | C(=O)Me | Cl | C(=O)Me | O |
| D-18312 | Me(S) | single bond | H | CH₂(pyrimidin-2-yl) | C(=O)Me | Cl | C(=O)Me | O |
| D-18313 | H | single bond | Me | CH₂(pyrimidin-2-yl) | C(=O)Me | Me | C(=O)Me | O |
| D-18314 | Me | single bond | H | CH₂(pyrimidin-2-yl) | C(=O)Me | Me | C(=O)Me | O |
| D-18315 | Me(R) | single bond | H | CH₂(pyrimidin-2-yl) | C(=O)Me | Me | C(=O)Me | O |
| D-18316 | Me(S) | single bond | H | CH₂(pyrimidin-2-yl) | C(=O)Me | Me | C(=O)Me | O |

**[Table 1063]**

| 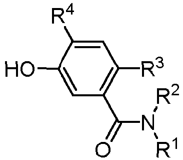 | | | | |
|---|---|---|---|---|
| Compound Number | R¹ | R² | R³ | R⁴ |
| E-0001 | CH₂CF₃ | H | F | H |
| E-0002 | CH₂CF₃ | H | F | F |
| E-0003 | CH₂CF₃ | H | F | Cl |
| E-0004 | CH₂CF₃ | H | F | Me |
| E-0005 | CH₂CF₃ | H | Cl | H |
| E-0006 | CH₂CF₃ | H | Cl | F |
| E-0007 | CH₂CF₃ | H | Cl | Cl |
| E-0008 | CH₂CF₃ | H | Cl | Me |
| E-0009 | CH₂CF₃ | H | Me | H |
| E-0010 | CH₂CF₃ | H | Me | F |
| E-0011 | CH₂CF₃ | H | Me | Cl |
| E-0012 | CH₂CF₃ | H | Me | Me |
| E-0013 | Et | H | F | H |
| E-0014 | Et | H | F | F |
| E-0015 | Et | H | F | Cl |
| E-0016 | Et | H | F | Me |
| E-0017 | Et | H | Cl | H |
| E-0018 | Et | H | Cl | F |
| E-0019 | Et | H | Cl | Cl |
| E-0020 | Et | H | Cl | Me |
| E-0021 | Et | H | Me | H |
| E-0022 | Et | H | Me | F |
| E-0023 | Et | H | Me | Cl |
| E-0024 | Et | H | Me | Me |
| E-0025 | *c*-Pr | H | F | H |
| E-0026 | *c*-Pr | H | F | F |
| E-0027 | *c*-Pr | H | F | Cl |
| E-0028 | *c*-Pr | H | F | Me |
| E-0029 | *c*-Pr | H | Cl | H |
| E-0030 | *c*-Pr | H | Cl | F |
| E-0031 | *c*-Pr | H | Cl | Cl |
| E-0032 | *c*-Pr | H | Cl | Me |
| E-0033 | *c*-Pr | H | Me | H |
| E-0034 | *c*-Pr | H | Me | F |
| E-0035 | *c*-Pr | H | Me | Cl |
| E-0036 | *c*-Pr | H | Me | Me |
| E-0037 | *c*-Pr(1-CN) | H | F | H |
| E-0038 | *c*-Pr(1-CN) | H | F | F |

**[Table 1064]**

| Compound Number | R¹ | R² | R³ | R⁴ |
|---|---|---|---|---|
| E-0039 | *c*-Pr(1-CN) | H | F | Cl |
| E-0040 | *c*-Pr(1-CN) | H | F | Me |
| E-0041 | *c*-Pr(1-CN) | H | Cl | H |
| E-0042 | *c*-Pr(1-CN) | H | Cl | F |
| E-0043 | *c*-Pr(1-CN) | H | Cl | Cl |
| E-0044 | *c*-Pr(1-CN) | H | Cl | Me |
| E-0045 | *c*-Pr(1-CN) | H | Me | H |
| E-0046 | *c*-Pr(1-CN) | H | Me | F |
| E-0047 | *c*-Pr(1-CN) | H | Me | Cl |
| E-0048 | *c*-Pr(1-CN) | H | Me | Me |

**[Table 1065]**

| 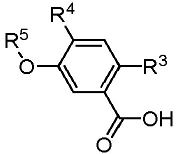 | | | |
|---|---|---|---|
| Compound Number | R³ | R⁴ | R⁵ |
| F-0001 | Cl | F | Me |
| F-0002 | Cl | F | Et |
| F-0003 | Cl | F | *n*-Pr |
| F-0004 | Cl | F | *i*-Pr |
| F-0005 | Cl | F | *n*-Bu |
| F-0006 | Cl | F | *n*-Pen |
| F-0007 | Cl | F | *n-Hex* |
| F-0008 | Cl | F | *n*-Heptyl |
| F-0009 | Cl | F | *n*-Octyl |
| F-0010 | Cl | F | *n-Nonyl* |
| F-0011 | Cl | F | *n*-Decyl |
| F-0012 | Cl | F | *c*-Pr |
| F-0013 | Cl | F | *c*-Pen |
| F-0014 | Cl | F | *c*-Hex |
| F-0015 | Cl | F | CH₂C(Me)=CH₂ |
| F-0016 | Cl | F | CH₂CH₂CH=CH₂ |
| F-0017 | Cl | F | CH₂CH₂CH=C(CH₃)₂ |
| F-0018 | Cl | F | CH₂CH₂CH₂CH=CH₂ |
| F-0019 | Cl | F | CH₂CH₂CH₂CH=C(CH₃)₂ |
| F-0020 | Cl | F | CH₂CH₂CH₂CH₂CH=CH₂ |
| F-0021 | Cl | F | CH₂CH₂CH₂CH₂CH=C(CH₃)₂ |
| F-0022 | Cl | F | CH₂CH₂CH≡CH |
| F-0023 | Cl | F | CH₂CH₂CH₂CH≡CH |
| F-0024 | Cl | F | CH₂CH₂CH₂CH₂CH≡CH |
| F-0025 | Cl | F | CH₂CH₂CH₂Cl |
| F-0026 | Cl | F | CH₂CH₂CH₂Br |
| F-0027 | Cl | F | CH₂CH₂CH₂CH₂Cl |
| F-0028 | Cl | F | CH₂CH₂CH₂CH₂Br |
| F-0029 | Cl | F | CH₂CH₂CH₂CH₂CH₂Cl |
| F-0030 | Cl | F | CH₂CH₂CH₂CH₂CH₂Br |
| F-0031 | Cl | F | CH₂CH₂CH₂CH₂CH₂CH₂Cl |
| F-0032 | Cl | F | CH₂CH₂CH₂CH₂CH₂CH₂Br |
| F-0033 | Cl | F | CH₂CH₂CH₂CH₂CH₂CH₂CH₂Cl |
| F-0034 | Cl | F | CH₂CH₂CH₂CH₂CH₂CH₂CH₂Br |
| F-0035 | Cl | F | CH₂CH₂CH₂CH₂CH₂CH₂CH₂CH₂Br |
| F-0036 | Cl | F | CH₂CH(Me)CH₂Cl |
| F-0037 | Cl | F | CH₂CH(Me)CH₂Br |
| F-0038 | Cl | F | CH₂CH₂CH(Me)CH₂CH₂Cl |

**[Table 1066]**

| Compound Number | R³ | R⁴ | R⁵ |
|---|---|---|---|
| F-0039 | Cl | F | CH₂CH₂CH(Me)CH₂CH₂Br |
| F-0040 | Cl | F | CH₂CH₂CF=CF₂ |
| F-0041 | Cl | F | CH₂CH=C(Cl)CF₃ |
| F-0042 | Cl | F | CH₂CH₂CH₂CF=CF₂ |
| F-0043 | Cl | F | CH₂CH₂CH₂CH₂CF=CF₂ |
| F-0044 | Cl | F | CH₂CH₂CH₂CH₂CH₂CF=CF₂ |
| F-0045 | Cl | F | CH₂(*t*-Bu) |
| F-0046 | Cl | F | CH₂CH₂(*t*-Bu) |
| F-0047 | Cl | F | CH₂CH₂CH₂(*t*-Bu) |
| F-0048 | Cl | F | CH₂CH₂CH₂CH₂(*t*-Bu) |
| F-0049 | Cl | F | CH₂CH₂CH₂CH₂CH₂(*t*-Bu) |
| F-0050 | Cl | F | CH₂CH₂CH₂CH₂CH₂CH₂(*t*-Bu) |
| F-0051 | Cl | F | CH₂CF₃ |
| F-0052 | Cl | F | CH₂CH₂CF₃ |
| F-0053 | Cl | F | CH₂CH₂CH₂CF₃ |
| F-0054 | Cl | F | CH₂CH₂CH₂CH₂CF₃ |
| F-0055 | Cl | F | CH₂CH₂CH₂CH₂CH₂CF₃ |
| F-0056 | Cl | F | CH₂CH₂CH₂CH₂CH₂CH₂CF₃ |
| F-0057 | Cl | F | CH₂CH₂CH₂CH₂CH₂CH₂CH₂CF₃ |
| F-0058 | Cl | F | CH₂CF₂CF₃ |
| F-0059 | Cl | F | CF₂CHFCF₃ |
| F-0060 | Cl | F | CF₂CF₂CF₃ |
| F-0061 | Cl | F | CH₂CF₂CF₂CF₃ |
| F-0062 | Cl | F | CH₂CF₂CF₂CF₂CF₃ |
| F-0063 | Cl | F | CH₂CF₂CF₂CF₂CHF₂ |
| F-0064 | Cl | F | CH(CF₃)CF₃ |
| F-0065 | Cl | F | CH₂CF(CF₃)CF₃ |
| F-0066 | Cl | F | CH₂CF₂CF(CF₃)CF₃ |
| F-0067 | Cl | F | CH₂CF(CF₃)CF₂CF₃ |
| F-0068 | Cl | F | CH₂CF₂CF₂CF(CF₃)CF₃ |
| F-0069 | Cl | F | CH₂CF₂CF(CF₃)CF₂CF₃ |
| F-0070 | Cl | F | CH₂CF(CF₃)CF₂CF₂CF₃ |
| F-0071 | Cl | F | CH₂(*c*-Pr) |
| F-0072 | Cl | F | CH₂[*c*-Pr(1-Me)] |
| F-0073 | Cl | F | CH₂[*c*-Pr(1-Ph)] |
| F-0074 | Cl | F | CH₂{*c*-Pr[1-NHS(=O)₂CF₃]} |
| F-0075 | Cl | F | CH₂{*c*-Pr[1-Ph(4-CF₃)]} |
| F-0076 | Cl | F | CH₂{*c*-Pr[1-Ph(3,4,5-F₃)]} |
| F-0077 | Cl | F | CH₂{*c*-Hex[4-(*t*-Bu)]} |
| F-0078 | Cl | F | CH₂[*c*-Hex(4-CF₃)] |
| F-0079 | Cl | F | CH₂CH₂(*c*-Pr) |
| F-0080 | Cl | F | CH₂CH₂(*c*-Hex) |
| F-0081 | Cl | F | CH₂CH₂[*c*-Hex(4-CF₃)] |
| F-0082 | Cl | F | CH₂CH₂[*c*-Hex(4-SCF₃)] |

**[Table 1067]**

| Compound Number | R³ | R⁴ | R⁵ |
|---|---|---|---|
| F-0083 | Cl | F | CH₂CH₂CH₂(*c*-Pr) |
| F-0084 | Cl | F | CH₂CH₂CH₂(*c*-Hex) |
| F-0085 | Cl | F | CH₂CH₂CH₂{*c*-Hex[4-(*t*-Bu)]} |
| F-0086 | Cl | F | CH₂CH₂CH₂[*c*-Hex(4-CF₃)] |
| F-0087 | Cl | F | CH₂CH₂CH₂[*c*-Hex(4-SCF₃)] |
| F-0088 | Cl | F | CH₂CH₂CH₂CH₂(*c*-Pr) |
| F-0089 | Cl | F | CH₂CH₂CH₂CH₂(*c*-Hex) |
| F-0090 | Cl | F | CH₂CH₂CH₂CH₂[*c*-Hex(4-CF₃)] |
| F-0091 | Cl | F | CH₂[*c*-Pr(2,2-F₂)] |
| F-0092 | Cl | F | CH₂[*c*-Hex(4,4-F₂)] |
| F-0093 | Cl | F | CH₂CH₂[*c*-Pr(2,2-F₂)] |
| F-0094 | Cl | F | CH₂CH₂[*c*-Hex(4,4-F₂)] |
| F-0095 | Cl | F | CH₂CH₂CH₂[*c*-Pr(2,2-F₂)] |
| F-0096 | Cl | F | CH₂CH₂CH₂CH₂[*c*-Pr(2,2-F₂)] |
| F-0097 | Cl | F | CH₂CH₂OH |
| F-0098 | Cl | F | CH₂CH₂CH₂OH |
| F-0099 | Cl | F | CH₂CH₂CH₂CH₂OH |
| F-0100 | Cl | F | CH₂CH₂CH₂CH₂CH₂OH |
| F-0101 | Cl | F | CH₂CH₂CH₂CH₂CH₂CH₂OH |
| F-0102 | Cl | F | CH₂C(=O)Me |
| F-0103 | Cl | F | CH₂CH(OH)Me |
| F-0104 | Cl | F | CH₂CH(OH)CF₃ |
| F-0105 | Cl | F | CH₂C(OH)₂CF₃ |
| F-0106 | Cl | F | CH₂C(CF₃)=NOH |
| F-0107 | Cl | F | CH₂C(CF₃)=NOMe |
| F-0108 | Cl | F | CH₂CH(CF₃)NH₂ |
| F-0109 | Cl | F | CH₂CH(CF₃)NHSO₂CF₃ |
| F-0110 | Cl | F | CH₂CH₂OMe |
| F-0111 | Cl | F | CH₂CH₂CH₂OMe |
| F-0112 | Cl | F | CH₂CH₂CH₂CH₂OMe |
| F-0113 | Cl | F | CH₂CH₂CH₂CH₂CH₂OMe |
| F-0114 | Cl | F | CH₂CH₂CH₂CH₂CH₂CH₂OMe |
| F-0115 | Cl | F | CH₂CH₂OCHF₂ |
| F-0116 | Cl | F | CH₂CH₂CH₂OCHF₂ |
| F-0117 | Cl | F | CH₂CH₂CH₂CH₂OCHF₂ |
| F-0118 | Cl | F | CH₂CH₂CH₂CH₂CH₂OCHF₂ |
| F-0119 | Cl | F | CH₂CH₂CH₂CH₂CH₂CH₂OCHF₂ |
| F-0120 | Cl | F | CH₂CH₂OCH₂CF₃ |
| F-0121 | Cl | F | CH₂CH₂CH₂OCH₂CF₃ |
| F-0122 | Cl | F | CH₂CH₂CH₂CH₂OCH₂CF₃ |
| F-0123 | Cl | F | CH₂CH₂CH₂CH₂CH₂OCH₂CF₃ |
| F-0124 | Cl | F | CH₂CH₂CH₂CH₂CH₂CH₂OCH₂CF₃ |
| F-0125 | Cl | F | CH₂CH₂CH₂CH₂CH₂CH₂CH₂OCH₂CF₃ |
| F-0126 | Cl | F | CH₂CH₂OC(CF₃)₃ |

**[Table 1068]**

| Compound Number | R³ | R⁴ | R⁵ |
|---|---|---|---|
| F-0127 | Cl | F | CH₂CH₂CH₂OC(CF₃)₃ |
| F-0128 | Cl | F | CH₂CH₂CH₂CH₂OC(CF₃)₃ |
| F-0129 | Cl | F | CH₂CH₂CH₂CH₂CH₂OC(CF₃)₃ |
| F-0130 | Cl | F | CF₂CHFOCF₂CF₂CF₃ |
| F-0131 | Cl | F | CH₂CH₂O(*c*-Pr) |
| F-0132 | Cl | F | CH₂CH₂O(*c*-Pen) |
| F-0133 | Cl | F | CH₂CH₂O(*c*-Hex) |
| F-0134 | Cl | F | CH₂CH₂CH₂O(*c*-Pr) |
| F-0135 | Cl | F | CH₂CH₂CH₂O(*c*-Pen) |
| F-0136 | Cl | F | CH₂CH₂CH₂O(*c*-Hex) |
| F-0137 | Cl | F | CH₂CH₂CH₂CH₂O(*c*-Pr) |
| F-0138 | Cl | F | CH₂CH₂CH₂CH₂O(*c*-Pen) |
| F-0139 | Cl | F | CH₂CH₂CH₂CH₂O(*c*-Hex) |
| F-0140 | Cl | F | CH₂CH₂O[*c*-Pr(2,2-F₂)] |
| F-0141 | Cl | F | CH₂CH₂CH₂O[*c*-Pr(2,2-F₂)] |
| F-0142 | Cl | F | CH₂CH₂CH₂CH₂O[*c*-Pr(2,2-F₂)] |
| F-0143 | Cl | F | CH₂CH₂OCH₂CH₂OCH₃ |
| F-0144 | Cl | F | CH₂CH₂OCH₂CH₂OCH₂CF₃ |
| F-0145 | Cl | F | CH₂CF₂OCF₂CF₂OCF₃ |
| F-0146 | Cl | F | CF₂CHFOCF₂CF₂OCF₃ |
| F-0147 | Cl | F | CF₂CHFOCF₂CF(CF₃)OCF₂CF₂CF₃ |
| F-0148 | Cl | F | CH₂CH₂SH |
| F-0149 | Cl | F | CH₂CH₂CH₂SH |
| F-0150 | Cl | F | CH₂CH₂CH₂CH₂SH |
| F-0151 | Cl | F | CH₂CH₂CH₂CH₂CH₂SH |
| F-0152 | Cl | F | CH₂CH₂CH₂CH₂CH₂CH₂SH |
| F-0153 | Cl | F | CH₂CH₂S(*t*-Bu) |
| F-0154 | Cl | F | CH₂CH₂CH₂S(*t*-Bu) |
| F-0155 | Cl | F | CH₂CH₂CH₂CH₂S(*t*-Bu) |
| F-0156 | Cl | F | CH₂CH₂CH₂CH₂CH₂SCH₃ |
| F-0157 | Cl | F | CH₂CH₂CH₂CH₂CH₂SCH(CH₃)₂ |
| F-0158 | Cl | F | CH₂CH₂CH₂CH₂CH₂S(*t*-Bu) |
| F-0159 | Cl | F | CH₂CH₂CH₂CH₂CH₂CH₂SCH(CH₃)₂ |
| F-0160 | Cl | F | CH₂CH₂CH₂CH₂CH₂CH₂S(*t*-Bu) |
| F-0161 | Cl | F | CH₂CH₂CH₂CH₂CH₂CH₂CH₂S(*t*-Bu) |
| F-0162 | Cl | F | CH₂CH₂SCHF₂ |
| F-0163 | Cl | F | CH₂CH₂CH₂SCHF₂ |
| F-0164 | Cl | F | CH₂CH₂CH₂CH₂SCHF₂ |
| F-0165 | Cl | F | CH₂CH₂CH₂CH₂CH₂SCHF₂ |
| F-0166 | Cl | F | CH₂CH₂CH₂CH₂CH₂CH₂SCHF₂ |
| F-0167 | Cl | F | CH₂CH₂CH₂CH₂CH₂CH₂CH₂SCHF₂ |
| F-0168 | Cl | F | CH₂CH₂SCF₃ |
| F-0169 | Cl | F | CH₂CH₂CH₂SCF₃ |
| F-0170 | Cl | F | CH₂CH₂CH₂CH₂SCF₃ |

**[Table 1069]**

| Compound Number | R³ | R⁴ | R⁵ |
|---|---|---|---|
| F-0171 | Cl | F | CH₂CH₂CH₂CH₂CH₂SCF₃ |
| F-0172 | Cl | F | CH₂CH₂CH₂CH₂CH₂CH₂SCF₃ |
| F-0173 | Cl | F | CH₂CH₂CH₂CH₂CH₂CH₂CH₂SCF₃ |
| F-0174 | Cl | F | CH₂CH₂CH₂CH₂CH₂CH₂CH₂CH₂SCF₃ |
| F-0175 | Cl | F | CH₂CH(Me)SCF₃ |
| F-0176 | Cl | F | CH(Me)CH₂SCF₃ |
| F-0177 | Cl | F | CH₂CH₂CH(Me)SCF₃ |
| F-0178 | Cl | F | CH₂CH(Me)CH₂SCF₃ |
| F-0179 | Cl | F | CH(Me)CH₂CH₂SCF₃ |
| F-0180 | Cl | F | CH₂CH₂CH₂CH(Me)SCF₃ |
| F-0181 | Cl | F | CH₂CH₂CH(Me)CH₂SCF₃ |
| F-0182 | Cl | F | CH₂CH(Me)CH₂CH₂SCF₃ |
| F-0183 | Cl | F | CH(Me)CH₂CH₂CH₂SCF₃ |
| F-0184 | Cl | F | CH₂CH₂SCH₂CF₃ |
| F-0185 | Cl | F | CH₂CH₂CH₂SCH₂CF₃ |
| F-0186 | Cl | F | CH₂CH₂CH₂CH₂SCH₂CF₃ |
| F-0187 | Cl | F | CH₂CH₂CH₂CH₂CH₂SCH₂CF₃ |
| F-0188 | Cl | F | CH₂CH₂CH₂CH₂CH₂CH₂SCH₂CF₃ |
| F-0189 | Cl | F | CH₂CH₂CH₂CH₂CH₂CH₂CH₂SCH₂CF₃ |
| F-0190 | Cl | F | CH₂CH₂SCH(CF₃)₂ |
| F-0191 | Cl | F | CH₂CH₂CH₂SCH(CF₃)₂ |
| F-0192 | Cl | F | CH₂CH₂CH₂CH₂SCH(CF₃)₂ |
| F-0193 | Cl | F | CH₂CH₂CH₂CH₂CH₂SCH(CF₃)₂ |
| F-0194 | Cl | F | CH₂CH₂CH₂CH₂CH₂CH₂SCH(CF₃)₂ |
| F-0195 | Cl | F | CH₂CH₂CH₂CH₂CH₂SCF₂CF₃ |
| F-0196 | Cl | F | CH₂CH₂CH₂CH₂CH₂CH₂SCF₂CF₃ |
| F-0197 | Cl | F | CH₂CH₂CH₂CH₂CH₂CH₂SCF(CF₃)₂ |
| F-0198 | Cl | F | CH₂CH₂CH₂CH₂CH₂CH₂CH₂SCF(CF₃)₂ |
| F-0199 | Cl | F | CH₂CH₂CH₂CH₂CH₂SCCl₃ |
| F-0200 | Cl | F | CH₂CH₂CH₂CH₂CH₂CH₂SCCl₃ |
| F-0201 | Cl | F | CH₂CH₂SCH₂CH=CH₂ |
| F-0202 | Cl | F | CH₂CH₂CH₂SCH₂CH=CH₂ |
| F-0203 | Cl | F | CH₂CH₂CH₂CH₂SCH₂CH=CH₂ |
| F-0204 | Cl | F | CH₂CH₂SCF=CFCF₃ |
| F-0205 | Cl | F | CH₂CH₂CH₂SCF=CFCF₃ |
| F-0206 | Cl | F | CH₂CH₂CH₂CH₂SCF=CFCF₃ |
| F-0207 | Cl | F | CH₂CH₂CH₂S(*c*-Pr) |
| F-0208 | Cl | F | CH₂CH₂CH₂S(*c*-Hex) |
| F-0209 | Cl | F | CH₂CH₂CH₂CH₂S(*c*-Pr) |
| F-0210 | Cl | F | CH₂CH₂CH₂CH₂S(*c*-Hex) |
| F-0211 | Cl | F | CH₂CH₂CH₂CH₂CH₂S(*c*-Pr) |
| F-0212 | Cl | F | CH₂CH₂CH₂CH₂CH₂S(*c*-Hex) |
| F-0213 | Cl | F | CH₂CH₂CH₂S[*c*-Hex(4,4-F₂)] |
| F-0214 | Cl | F | CH₂CH₂CH₂CH₂S[*c*-Hex(4,4-F₂)] |

**[Table 1070]**

| Compound Number | R³ | R⁴ | R⁵ |
|---|---|---|---|
| F-0215 | Cl | F | CH₂CH₂CH₂CH₂CH₂S[*c*-Hex(4,4-F₂)] |
| F-0216 | Cl | F | CH₂CH₂CH₂SCH₂(*c*-Pr) |
| F-0217 | Cl | F | CH₂CH₂CH₂SCH₂(*c-*Hex) |
| F-0218 | Cl | F | CH₂CH₂CH₂CH₂SCH₂(*c*-Pr) |
| F-0219 | Cl | F | CH₂CH₂CH₂CH₂SCH₂(*c*-Hex) |
| F-0220 | Cl | F | CH₂CH₂CH₂CH₂CH₂SCH₂(*c*-Pr) |
| F-0221 | Cl | F | CH₂CH₂CH₂CH₂CH₂SCH₂(*c*-Hex) |
| F-0222 | Cl | F | CH₂CH₂CH₂SCH₂[*c*-Hex(4,4-F₂)] |
| F-0223 | Cl | F | CH₂CH₂CH₂CH₂SCH₂[*c*-Hex(4,4-F₂)] |
| F-0224 | Cl | F | CH₂CH₂CH₂CH₂CH₂SCH₂[*c*-Hex(4,4-F₂)] |
| F-0225 | Cl | F | CH₂CH₂CH₂SCH₂SiMe₃ |
| F-0226 | Cl | F | CH₂CH₂CH₂CH₂SCH₂SiMe₃ |
| F-0227 | Cl | F | CH₂CH₂CH₂CH₂CH₂SCH₂SiMe₃ |
| F-0228 | Cl | F | CH₂CH₂OCH₂CH₂SCH₃ |
| F-0229 | Cl | F | CH₂CH₂OCH₂CH₂SCHF₂ |
| F-0230 | Cl | F | CH₂CH₂OCH₂CH₂SCF₃ |
| F-0231 | Cl | F | CH₂CH₂OCH₂CH₂SCH₂CF₃ |
| F-0232 | Cl | F | CH₂CH₂CH₂S(=O)(t-Bu) |
| F-0233 | Cl | F | CH₂CH₂CH₂CH₂S(=O)(t-Bu) |
| F-0234 | Cl | F | CH₂CH₂CH₂CH₂CH₂CH₂S(=O)(*t*-Bu) |
| F-0235 | Cl | F | CH₂CH₂S(=O)CHF₂ |
| F-0236 | Cl | F | CH₂CH₂CH₂S(=O)CHF₂ |
| F-0237 | Cl | F | CH₂CH₂CH₂CH₂S(=O)CHF₂ |
| F-0238 | Cl | F | CH₂CH₂CH₂CH₂CH₂S(=O)CHF₂ |
| F-0239 | Cl | F | CH₂CH₂CH₂CH₂CH₂CH₂S(=O)CHF₂ |
| F-0240 | Cl | F | CH₂CH₂CH₂CH₂CH₂CH₂CH₂S(=O)CHF₂ |
| F-0241 | Cl | F | CH₂CH₂S(=O)CF₃ |
| F-0242 | Cl | F | CH₂CH₂CH₂S(=O)CF₃ |
| F-0243 | Cl | F | CH₂CH₂CH₂CH₂S(=O)CF₃ |
| F-0244 | Cl | F | CH₂CH₂CH₂CH₂CH₂S(=O)CF₃ |
| F-0245 | Cl | F | CH₂CH₂CH₂CH₂CH₂CH₂S(=O)CF₃ |
| F-0246 | Cl | F | CH₂CH₂CH₂CH₂CH₂CH₂CH₂S(=O)CF₃ |
| F-0247 | Cl | F | CH₂CH₂S(=O)CH₂CF₃ |
| F-0248 | Cl | F | CH₂CH₂CH₂S(=O)CH₂CF₃ |
| F-0249 | Cl | F | CH₂CH₂CH₂CH₂S(=O)CH₂CF₃ |
| F-0250 | Cl | F | CH₂CH₂CH₂CH₂CH₂S(=O)CH₂CF₃ |
| F-0251 | Cl | F | CH₂CH₂CH₂CH₂CH₂CH₂S(=O)CH₂CF₃ |
| F-0252 | Cl | F | CH₂CH₂CH₂CH₂CH₂CH₂CH₂S(=O)CH₂CF₃ |
| F-0253 | Cl | F | CH₂CH₂CH₂S(=O)(*c*-Pr) |
| F-0254 | Cl | F | CH₂CH₂CH₂CH₂S(=O)(*c*-Pr) |
| F-0255 | Cl | F | CH₂CH₂CH₂CH₂S(=O)(*c*-Pen) |
| F-0256 | Cl | F | CH₂CH₂CH₂CH₂S(=O)(*c*-Hex) |
| F-0257 | Cl | F | CH₂CH₂CH₂CH₂CH₂S(=O)(*c*-Pr) |
| F-0258 | Cl | F | CH₂CH₂CH₂CH₂CH₂S(=O)(*c*-Hex) |

**[Table 1071]**

| Compound Number | R³ | R⁴ | R⁵ |
|---|---|---|---|
| F-0259 | Cl | F | CH₂CH₂CH₂S(=O)[*c*-Hex(4,4-F₂)] |
| F-0260 | Cl | F | CH₂CH₂CH₂CH₂S(=O)[*c*-Hex(4,4-F₂)] |
| F-0261 | Cl | F | CH₂CH₂CH₂CH₂CH₂S(=O)[*c*-Hex(4,4-F₂)] |
| F-0262 | Cl | F | CH₂CH₂CH₂CH₂S(=O)₂Me |
| F-0263 | Cl | F | CH₂CH₂CH₂CH₂CH₂S(=O)₂Me |
| F-0264 | Cl | F | CH₂CH₂CH₂CH₂CH₂S(=O)₂(t-Bu) |
| F-0265 | Cl | F | CH₂CH₂S(=O)₂CHF₂ |
| F-0266 | Cl | F | CH₂CH₂CH₂S(=O)₂CHF₂ |
| F-0267 | Cl | F | CH₂CH₂CH₂CH₂S(=O)₂CHF₂ |
| F-0268 | Cl | F | CH₂CH₂CH₂CH₂CH₂S(=O)₂CHF₂ |
| F-0269 | Cl | F | CH₂CH₂CH₂CH₂CH₂CH₂S(=O)₂CHF₂ |
| F-0270 | Cl | F | CH₂CH₂S(=O)₂CF₃ |
| F-0271 | Cl | F | CH₂CH₂CH₂S(=O)₂CF₃ |
| F-0272 | Cl | F | CH₂CH₂CH₂CH₂S(=O)₂CF₃ |
| F-0273 | Cl | F | CH₂CH₂CH₂CH₂CH₂S(=O)₂CF₃ |
| F-0274 | Cl | F | CH₂CH₂CH₂CH₂CH₂CH₂S(=O)₂CF₃ |
| F-0275 | Cl | F | CH₂CH₂CH₂CH₂CH₂CH₂CH₂S(=O)₂CF₃ |
| F-0276 | Cl | F | CH₂CH₂CH₂CH₂CH₂CH₂CH₂CH₂S(=O)₂CF₃ |
| F-0277 | Cl | F | CH₂CH₂S(=O)₂CH₂CF₃ |
| F-0278 | Cl | F | CH₂CH₂CH₂S(=O)₂CH₂CF₃ |
| F-0279 | Cl | F | CH₂CH₂CH₂CH₂S(=O)₂CH₂CF₃ |
| F-0280 | Cl | F | CH₂CH₂CH₂CH₂CH₂S(=O)₂CH₂CF₃ |
| F-0281 | Cl | F | CH₂CH₂CH₂CH₂CH₂CH₂S(=O)₂CH₂CF₃ |
| F-0282 | Cl | F | CH₂CH₂CH₂S(=O)₂(*c*-pr) |
| F-0283 | Cl | F | CH₂CH₂CH₂CH₂S(=O)₂(*c*-Pr) |
| F-0284 | Cl | F | CH₂CH₂CH₂CH₂CH₂S(=O)₂(*c*-Pr) |
| F-0285 | Cl | F | CH₂CH₂CH₂S(=O)₂[*c*-Hex(4,4-F₂)] |
| F-0286 | Cl | F | CH₂CH₂CH₂CH₂S(=O)2[*c*-Hex(4,4-F₂)] |
| F-0287 | Cl | F | CH₂CH₂CH₂CH₂CH₂S(=O)₂[*c*-Hex(4,4-F₂)] |
| F-0288 | Cl | F | CH₂CH₂CH₂C(=O)H |
| F-0289 | Cl | F | CH₂CH₂CH₂CH₂C(=O)H |
| F-0290 | Cl | F | CH₂CH₂CH₂CH₂CH₂C(=O)H |
| F-0291 | Cl | F | CH₂CH₂CH₂C(=O)(t-Bu) |
| F-0292 | Cl | F | CH₂CH₂CH₂CH₂C(=O)(t-Bu) |
| F-0293 | Cl | F | CH₂CH₂CH₂CH₂CH₂C(=O)(*t*-Bu) |
| F-0294 | Cl | F | CH₂CH₂CH₂C(=O)CF₃ |
| F-0295 | Cl | F | CH₂CH₂CH₂CH₂C(=O)CF₃ |
| F-0296 | Cl | F | CH₂CH₂CH₂CH₂CH₂C(=O)CF₃ |
| F-0297 | Cl | F | CH₂C(=O)Ph |
| F-0298 | Cl | F | CH₂C(=O)Ph(4-Cl) |
| F-0299 | Cl | F | CH₂C(=O)Ph(4-CF₃) |
| F-0300 | Cl | F | CH₂CH₂C(=O)Ph(4-CF₃) |
| F-0301 | Cl | F | CH₂CH₂CH₂C(=O)Ph(4-CF₃) |
| F-0302 | Cl | F | CH₂CH₂CH₂CH₂C(=O)ph(4-CF₃) |

**[Table 1072]**

| Compound Number | R³ | R⁴ | R⁵ |
|---|---|---|---|
| F-0303 | Cl | F | CH₂CH₂CH₂CH₂CH₂C(=O)Ph(4-CF₃) |
| F-0304 | Cl | F | CH₂C(=O)OEt |
| F-0305 | Cl | F | CH₂CH₂C(=O)O(*t*-Bu) |
| F-0306 | Cl | F | CH₂CH₂CH₂C(=O)OEt |
| F-0307 | Cl | F | CH₂CH₂CH₂C(=O)O(t-Bu) |
| F-0308 | Cl | F | CH₂CH₂CH₂CH₂C(=O)O(t-Bu) |
| F-0309 | Cl | F | CH₂CH₂CH₂CH₂CH₂C(=O)O(*t*-Bu) |
| F-0310 | Cl | F | CH₂CH₂CH₂CH₂CH₂CH₂C(=O)O(*t*-Bu) |
| F-0311 | Cl | F | CH₂CH₂CH₂CH₂CH₂CH₂CH₂C(=O)OEt |
| F-0312 | Cl | F | CH₂CH₂C(=O)OCH₂CF₃ |
| F-0313 | Cl | F | CH₂CH₂CH₂C(=O)OCH₂CF₃ |
| F-0314 | Cl | F | CH₂CH₂CH₂CH₂C(=O)OCH₂CF₃ |
| F-0315 | Cl | F | CH₂CH₂CH₂CH₂CH₂C(=O)OCH₂CF₃ |
| F-0316 | Cl | F | CH₂CH₂C(=O)NH(*t*-Bu) |
| F-0317 | Cl | F | CH₂CH₂CH₂C(=O)NH(*t*-Bu) |
| F-0318 | Cl | F | CH₂CH₂CH₂C(=O)NH(*t*-Pen) |
| F-0319 | Cl | F | CH₂CH₂CH₂CH₂C(=O)NH(*t*-Bu) |
| F-0320 | Cl | F | CH₂CH₂C(=O)NHCH₂CF₃ |
| F-0321 | Cl | F | CH₂CH₂CH₂C(=O)NHCH₂CF₃ |
| F-0322 | Cl | F | CH₂CH₂CH₂CH₂C(=O)NHCH₂CF₃ |
| F-0323 | Cl | F | CH₂CH₂SC(=O)N(Me)₂ |
| F-0324 | Cl | F | CH₂CH₂CH₂SC(=O)N(Me)₂ |
| F-0325 | Cl | F | CH₂CH₂CH₂SC(=O)NH(*t*-Bu) |
| F-0326 | Cl | F | CH₂CH₂CH₂CH₂CH₂SC(=O)NH(*t*-Bu) |
| F-0327 | Cl | F | CH₂CH₂SC(=O)NHCH₂CF₃ |
| F-0328 | Cl | F | CH₂CH₂CH₂SC(=O)NHCH₂CF₃ |
| F-0329 | Cl | F | CH₂CH₂CH₂CH₂SC(=O)NHCH₂CF₃ |
| F-0330 | Cl | F | CH₂CH₂CH₂OC(=O)H |
| F-0331 | Cl | F | CH₂CH₂CH₂CH₂OC(=O)H |
| F-0332 | Cl | F | CH₂CH₂CH₂CH₂CH₂OC(=O)H |
| F-0333 | Cl | F | CH₂CH₂CH₂OC(=O)Me |
| F-0334 | Cl | F | CH₂CH₂CH₂CH₂OC(=O)Me |
| F-0335 | Cl | F | CH₂CH₂CH₂CH₂CH₂OC(=O)Me |
| F-0336 | Cl | F | CH₂CH₂OC(=O)CF₃ |
| F-0337 | Cl | F | CH₂CH₂CH₂OC(=O)CF₃ |
| F-0338 | Cl | F | CH₂CH₂CH₂CH₂OC(=O)CF₃ |
| F-0339 | Cl | F | CH₂CH₂CH₂CH₂CH₂OC(=O)Ph(4-CF₃) |
| F-0340 | Cl | F | CH₂CH₂OC(=O)Ph |
| F-0341 | Cl | F | CH₂CH₂OC(=O)Ph(4-CF₃) |
| F-0342 | Cl | F | CH₂CH₂CH₂OC(=O)Ph(4-CF₃) |
| F-0343 | Cl | F | CH₂CH₂CH₂CH₂OC(=O)Ph(4-CF₃) |
| F-0344 | Cl | F | CH₂CH₂CH₂CH₂CH₂OC(=O)Ph(4-CF₃) |
| F-0345 | Cl | F | CH₂CH₂OS(=O)Me |
| F-0346 | Cl | F | CH₂CH₂CH₂OS(=O)Me |

**[Table 1073]**

| Compound Number | R³ | R⁴ | R⁵ |
|---|---|---|---|
| F-0347 | Cl | F | CH₂CH₂CH₂CH₂OS(=O)Me |
| F-0348 | Cl | F | CH₂CH₂CH₂CH₂CH₂OS(=O)Me |
| F-0349 | Cl | F | CH₂CH₂CH₂CH₂CH₂CH₂OS(=O)Me |
| F-0350 | Cl | F | CH₂CH₂OS(=O)CF₃ |
| F-0351 | Cl | F | CH₂CH₂CH₂OS(=O)CF₃ |
| F-0352 | Cl | F | CH₂CH₂CH₂CH₂OS(=O)CF₃ |
| F-0353 | Cl | F | CH₂CH₂CH₂CH₂CH₂OS(=O)CF₃ |
| F-0354 | Cl | F | CH₂CH₂CH₂CH₂CH₂CH₂OS(=O)CF₃ |
| F-0355 | Cl | F | CH₂CH₂OS(=O)Ph |
| F-0356 | Cl | F | CH₂CH₂OS(=O)Ph(4-Me) |
| F-0357 | Cl | F | CH₂CH₂CH₂OS(=O)Ph |
| F-0358 | Cl | F | CH₂CH₂CH₂OS(=O)Ph(4-Me) |
| F-0359 | Cl | F | CH₂CH₂OS(=O)₂Me |
| F-0360 | Cl | F | CH₂CH₂CH₂OS(=O)₂Me |
| F-0361 | Cl | F | CH₂CH₂CH₂OS(=O)₂CF₃ |
| F-0362 | Cl | F | CH₂CH₂CH₂CH₂OS(=O)₂CF₃ |
| F-0363 | Cl | F | CH₂CH₂CH₂CH₂CH₂OS(=O)₂CF₃ |
| F-0364 | Cl | F | CH₂CH₂CH₂CH₂CH₂OS(=O)₂CF₂CF₂CF₂CF₃ |
| F-0365 | Cl | F | CH₂CH₂OS(=O)₂Ph |
| F-0366 | Cl | F | CH₂CH₂OS(=O)₂Ph(4-Me) |
| F-0367 | Cl | F | CH₂CH₂CH₂OS(=O)₂Ph |
| F-0368 | Cl | F | CH₂CH₂CH₂OS(=O)₂Ph(4-Me) |
| F-0369 | Cl | F | CH₂CH₂NH₂ |
| F-0370 | Cl | F | CH₂CH₂CH₂NH₂ |
| F-0371 | Cl | F | CH₂CH₂CH₂CH₂NH₂ |
| F-0372 | Cl | F | CH₂CH₂CH₂CH₂CH₂NH₂ |
| F-0373 | Cl | F | CH₂CH₂CH₂CH₂CH₂CH₂NH₂ |
| F-0374 | Cl | F | CH₂CH₂CH₂CH₂CH₂CH₂CH₂NH₂ |
| F-0375 | Cl | F | CH₂CH(Me)NH₂ |
| F-0376 | Cl | F | CH₂C*H(Me)NH₂ : (R) |
| F-0377 | Cl | F | CH₂C*H(Me)NH₂ : (S) |
| F-0378 | Cl | F | CH₂CH(Et)NH₂ |
| F-0379 | Cl | F | CH₂CH(*i*-Pr)NH₂ |
| F-0380 | Cl | F | CH(Me)CH₂NH₂ |
| F-0381 | Cl | F | CH(Et)CH₂NH₂ |
| F-0382 | Cl | F | CH(*i*-Pr)CH₂NH₂ |
| F-0383 | Cl | F | CH₂CH₂CH(Me)NH₂ |
| F-0384 | Cl | F | CH₂CH(Me)CH₂NH₂ |
| F-0385 | Cl | F | CH(Me)CH₂CH₂NH₂ |
| F-0386 | Cl | F | CH₂CH(Me)NH(*c*-Pr) |
| F-0387 | Cl | F | CH₂CH(Me)N(*c*-Pr)S(=O)₂CF₃ |
| F-0388 | Cl | F | CH₂CH(Me)NHOMe |
| F-0389 | Cl | F | CH₂CH(Me)N(OMe)S(=O)₂CF₃ |
| F-0390 | Cl | F | CH₂CH(Me)N(OMe)S(=O)₂Ph(2-NO₂) |

**[Table 1074]**

| Compound Number | R³ | R⁴ | R⁵ |
|---|---|---|---|
| F-0391 | Cl | F | CH₂CH(Me)N(OMe)S(=O)₂Ph(4-NO₂) |
| F-0392 | Cl | F | CH₂CH₂N(Me)(*t*-Bu) |
| F-0393 | Cl | F | CH₂CH₂CH₂N(Me)(*t*-Bu) |
| F-0394 | Cl | F | CH₂CH₂CH₂CH₂N(Me)(*t*-Bu) |
| F-0395 | Cl | F | CH₂CH₂NHCH₂CF₃ |
| F-0396 | Cl | F | CH₂CH₂CH₂NHCH₂CF₃ |
| F-0397 | Cl | F | CH₂CH₂CH₂CH₂NHCH₂CF₃ |
| F-0398 | Cl | F | CH₂CH₂NHC(=O)C(Me)(CF₃)₂ |
| F-0399 | Cl | F | CH₂CH₂NHC(=O)Me |
| F-0400 | Cl | F | CH₂CH₂NHC(=O)(*t*-Bu) |
| F-0401 | Cl | F | CH₂CH₂CH₂CH₂NHC(=O)CH(CH₃)₂ |
| F-0402 | Cl | F | CH₂CH₂CH₂CH₂NHC(=O)(*t*-Bu) |
| F-0403 | Cl | F | CH₂CH₂CH₂CH₂NHC(=O)CH₂(*t*-Bu) |
| F-0404 | Cl | F | CH₂CH₂NHC(=O)CF₃ |
| F-0405 | Cl | F | CH₂CH₂NHC(=O)CH₂CF₃ |
| F-0406 | Cl | F | CH₂CH₂NHC(=O)CF₂CF₃ |
| F-0407 | Cl | F | CH₂CH₂CH₂NHC(=O)CF₃ |
| F-0408 | Cl | F | CH₂CH₂CH₂NHC(=O)CH₂CF₃ |
| F-0409 | Cl | F | CH₂CH₂CH₂NHC(=O)CF₂CF₃ |
| F-0410 | Cl | F | CH₂CH₂CH₂CH₂NHC(=O)CF₃ |
| F-0411 | Cl | F | CH₂CH₂CH₂CH₂NHC(=O)CH₂CF₃ |
| F-0412 | Cl | F | CH₂CH₂CH₂CH₂NHC(=O)CF₂CF₃ |
| F-0413 | Cl | F | CH₂CH₂CH₂CH₂NHC(=O)CF(CF₃)₂ |
| F-0414 | Cl | F | CH₂CH₂CH₂CH₂NHC(=O)CCl₃ |
| F-0415 | Cl | F | CH₂CH₂NHC(=O)Ph |
| F-0416 | Cl | F | CH₂CH₂NHC(=O)Ph(4-CF₃) |
| F-0417 | Cl | F | CH₂CH₂CH₂NHC(=O)Ph(4-CF₃) |
| F-0418 | Cl | F | CH₂CH₂CH₂CH₂NHC(=O)Ph(4-CF₃) |
| F-0419 | Cl | F | CH₂CH₂NHC(=O)O(*t*-Bu) |
| F-0420 | Cl | F | CH₂CH₂CH₂NHC(=O)O(*t*-Bu) |
| F-0421 | Cl | F | CH₂CH₂CH₂CH₂NHC(=O)O(*t*-Bu) |
| F-0422 | Cl | F | CH₂CH₂CH₂CH₂NHC(=O)OCH(CH₃)₂ |
| F-0423 | Cl | F | CH₂CH(Me)NHC(=O)O(t-Bu) |
| F-0424 | Cl | F | CH₂C*H(Me)NHC(=O)O(t-Bu) : (R) |
| F-0425 | Cl | F | CH₂C*H(Me)NHC(=O)O(t-Bu) : (S) |
| F-0426 | Cl | F | CH₂CH(Et)NHC(=O)O(*t*-Bu) |
| F-0427 | Cl | F | CH₂CH(*i*-Pr)NHC(=O)O(*t*-Bu) |
| F-0428 | Cl | F | CH(Me)CH₂NHC(=O)O(*t*-Bu) |
| F-0429 | Cl | F | CH(Et)CH₂NHC(=O)O(*t*-Bu) |
| F-0430 | Cl | F | CH(*i*-Pr)CH₂NHC(=O)O(*t*-Bu) |
| F-0431 | Cl | F | CH₂CH(Me)CH₂NHC(=O)O(*t*-Bu) |
| F-0432 | Cl | F | C H₂CH₂NHC(=O)OC(Me)₂CF₃ |
| F-0433 | Cl | F | CH₂CH₂CH₂NHC(=O)OCH₂CF₃ |
| F-0434 | Cl | F | CH₂CH₂CH₂NHC(=O)OCH₂CH₂CF₃ |

**[Table 1075]**

| Compound Number | R³ | R⁴ | R⁵ |
|---|---|---|---|
| F-0435 | Cl | F | CH₂CH₂CH₂CH₂NHC(=O)OCH₂CCl₃ |
| F-0436 | Cl | F | CH₂CH₂CH₂CH₂NHC(=O)OCH₂CF₃ |
| F-0437 | Cl | F | CH₂CH₂NHC(=O)NH(*t*-Bu) |
| F-0438 | Cl | F | CH₂CH₂CH₂NHC(=O)NH(*t*-Bu) |
| F-0439 | Cl | F | CH₂CH₂CH₂CH₂NHC(=O)NHEt |
| F-0440 | Cl | F | CH₂CH₂CH₂CH₂NHC(=O)NH(*t*-Bu) |
| F-0441 | Cl | F | CH₂CH₂CH₂NHC(=O)NHCH₂CCl₃ |
| F-0442 | Cl | F | CH₂CH₂CH₂NHC(=O)NHCH₂CH₂F |
| F-0443 | Cl | F | CH₂CH₂CH₂NHC(=O)NHCH₂CF₃ |
| F-0444 | Cl | F | CH₂CH₂CH₂CH₂NHC(=O)NHCH₂CCl₃ |
| F-0445 | Cl | F | CH₂CH₂CH₂CH₂NHC(=O)NHCH₂CH₂F |
| F-0446 | Cl | F | CH₂CH₂CH₂CH₂NHC(=O)NHCH₂CF₃ |
| F-0447 | Cl | F | CH₂CH₂NHS(=O)Me |
| F-0448 | Cl | F | CH₂CH₂CH₂NHS(=O)Me |
| F-0449 | Cl | F | CH₂CH₂CH₂CH₂NHS(=O)Me |
| F-0450 | Cl | F | CH₂CH₂CH₂CH₂NHS(=O)CH(CH₃)₂ |
| F-0451 | Cl | F | CH₂CH₂CH₂CH₂CH₂NHS(=O)Me |
| F-0452 | Cl | F | CH₂CH₂NHS(=O)CHF₂ |
| F-0453 | Cl | F | CH₂CH₂CH₂NHS(=O)CHF₂ |
| F-0454 | Cl | F | CH₂CH₂CH₂CH₂NHS(=O)CHF₂ |
| F-0455 | Cl | F | CH₂CH₂CH₂CH₂CH₂NHS(=O)CHF₂ |
| F-0456 | Cl | F | CH₂CH(Me)NHS(=O)CHF₂ |
| F-0457 | Cl | F | CH(Me)CH₂NHS(=O)CHF₂ |
| F-0458 | Cl | F | CH₂CH₂CH(Me)NHS(=O)CHF₂ |
| F-0459 | Cl | F | CH₂CH(Me)CH₂NHS(=O)CHF₂ |
| F-0460 | Cl | F | CH(Me)CH₂CH₂NHS(=O)CHF₂ |
| F-0461 | Cl | F | CH₂CH₂NHS(=O)CF₃ |
| F-0462 | Cl | F | CH₂CH₂CH₂NHS(=O)CF₃ |
| F-0463 | Cl | F | CH₂CH₂CH₂CH₂NHS(=O)CF₃ |
| F-0464 | Cl | F | CH₂CH₂CH₂CH₂CH₂NHS(=O)CF₃ |
| F-0465 | Cl | F | CH₂CH₂CH₂NHS(=O)Ph |
| F-0466 | Cl | F | CH₂CH₂CH₂CH₂NHS(=O)Ph |
| F-0467 | Cl | F | CH₂CH₂CH₂CH₂CH₂NHS(=O)Ph |
| F-0468 | Cl | F | CH₂CH₂CH₂CH₂CH₂NHS(=O)Ph(4-CF₃) |
| F-0469 | Cl | F | CH₂CH(Me)NHS(=O)Ph(4-CF₃) |
| F-0470 | Cl | F | CH₂CH(Et)NHS(=O)Ph(4-CF₃) |
| F-0471 | Cl | F | CH(Me)CH₂NHS(=O)Ph(4-CF₃) |
| F-0472 | Cl | F | CH(Et)CH₂NHS(=O)Ph(4-CF₃) |
| F-0473 | Cl | F | CH₂CH(Et)CH₂NHS(=O)Ph(4-CF₃) |
| F-0474 | Cl | F | CH₂CH₂NHS(=O)₂Me |
| F-0475 | Cl | F | CH₂CH₂CH₂NHS(=O)₂Me |
| F-0476 | Cl | F | CH₂CH₂CH₂CH₂NHS(=O)₂Me |
| F-0477 | Cl | F | CH₂CH₂CH₂CH₂NHS(=O)₂CH(CH₃)₂ |
| F-0478 | Cl | F | CH₂CH₂CH₂CH₂CH₂NHS(=O)₂Me |

**[Table 1076]**

| Compound Number | R³ | R⁴ | R⁵ |
|---|---|---|---|
| F-0479 | Cl | F | CH₂CH₂NHS(=O)₂CHF₂ |
| F-0480 | Cl | F | CH₂CH₂CH₂NHS(=O)₂CHF₂ |
| F-0481 | Cl | F | CH₂CH₂CH₂CH₂NHS(=O)₂CHF₂ |
| F-0482 | Cl | F | CH₂CH₂CH₂CH₂CH₂NHS(=O)₂CHF₂ |
| F-0483 | Cl | F | CH₂CH(Me)NHS(=O)₂CHF₂ |
| F-0484 | Cl | F | CH(Me)CH₂NHS(=O)₂CHF₂ |
| F-0485 | Cl | F | CH₂CH₂CH(Me)NHS(=O)₂CHF₂ |
| F-0486 | Cl | F | CH₂CH(Me)CH₂NHS(=O)₂CHF₂ |
| F-0487 | Cl | F | CH(Me)CH₂CH₂NHS(=O)₂CHF₂ |
| F-0488 | Cl | F | CH₂CH(Me)N(Me)S(=O)₂CHF₂ |
| F-0489 | Cl | F | CH(Me)CH₂N(Me)S(=O)₂CHF₂ |
| F-0490 | Cl | F | CH₂CH₂CH(Me)N(Me)S(=O)₂CHF₂ |
| F-0491 | Cl | F | CH₂CH(Me)CH₂N(Me)S(=O)₂CHF₂ |
| F-0492 | Cl | F | CH(Me)CH₂CH₂N(Me)S(=O)₂CHF₂ |
| F-0493 | Cl | F | CH₂CH₂NHS(=O)₂CF₃ |
| F-0494 | Cl | F | CH₂CH₂CH₂NHS(=O)₂CF₃ |
| F-0495 | Cl | F | CH₂CH₂CH₂CH₂NHS(=O)₂CF₃ |
| F-0496 | Cl | F | CH₂CH₂CH₂CH₂CH₂NHS(=O)₂CF₃ |
| F-0497 | Cl | F | CH₂CH₂N(Me)S(=O)₂CF₃ |
| F-0498 | Cl | F | CH₂CH₂N(Et)S(=O)₂CF₃ |
| F-0499 | Cl | F | CH₂CH₂N(CH₂CN)S(=O)₂CF₃ |
| F-0500 | Cl | F | CH₂CH₂N(CH₂OMe)S(=O)₂CF₃ |
| F-0501 | Cl | F | CH₂CH₂CH₂N(Me)S(=O)₂CF₃ |
| F-0502 | Cl | F | CH₂CH₂CH₂N(Et)S(=O)₂CF₃ |
| F-0503 | Cl | F | CH₂CH₂CH₂N(CH₂CN)S(=O)₂CF₃ |
| F-0504 | Cl | F | CH₂CH₂CH₂N(CH₂OMe)S(=O)₂CF₃ |
| F-0505 | Cl | F | CH₂CH₂CH₂CH₂N(Me)S(=O)₂CF₃ |
| F-0506 | Cl | F | CH₂CH₂CH₂CH₂N(Et)S(=O)₂CF₃ |
| F-0507 | Cl | F | CH₂CH₂CH₂CH₂N(CH₂CN)S(=O)₂CF₃ |
| F-0508 | Cl | F | CH₂CH₂CH₂CH₂N(CH₂OMe)S(=O)₂CF₃ |
| F-0509 | Cl | F | CH₂CH(Me)NHS(=O)₂CF₃ |
| F-0510 | Cl | F | CH₂C*H(Me)NHS(=O)₂CF₃ : (R) |
| F-0511 | Cl | F | CH₂C*H(Me)NHS(=O)₂CF₃ : (S) |
| F-0512 | Cl | F | CH(Me)CH₂NHS(=O)₂CF₃ |
| F-0513 | Cl | F | CH₂CH₂CH(Me)NHS(=O)₂CF₃ |
| F-0514 | Cl | F | CH₂CH(Me)CH₂NHS(=O)₂CF₃ |
| F-0515 | Cl | F | CH(Me)CH₂CH₂NHS(=O)₂CF₃ |
| F-0516 | Cl | F | CH₂CH(Me)N(Me)S(=O)₂CF₃ |
| F-0517 | Cl | F | CH(Me)CH₂N(Me)S(=O)₂CF₃ |
| F-0518 | Cl | F | CH₂CH₂CH(Me)N(Me)S(=O)₂CF₃ |
| F-0519 | Cl | F | CH₂CH(Me)CH₂N(Me)S(=O)₂CF₃ |
| F-0520 | Cl | F | CH(Me)CH₂CH₂N(Me)S(=O)₂CF₃ |
| F-0521 | Cl | F | CH₂CH₂CH₂NHS(=O)₂Ph |
| F-0522 | Cl | F | CH₂CH₂CH₂CH₂NHS(=O)₂Ph |

**[Table 1077]**

| Compound Number | R³ | R⁴ | R⁵ |
|---|---|---|---|
| F-0523 | Cl | F | CH₂CH₂CH₂CH₂CH₂NHS(=O)₂Ph |
| F-0524 | Cl | F | CH₂CH₂CH₂CH₂CH₂NHS(=O)₂Ph(4-CF₃) |
| F-0525 | Cl | F | CH₂CH(Me)NHS(=O)₂Ph(4-CF₃) |
| F-0526 | Cl | F | CH₂CH(Et)NHS(=O)₂Ph(4-CF₃) |
| F-0527 | Cl | F | CH(Me)CH₂NHS(=O)₂Ph(4-CF₃) |
| F-0528 | Cl | F | CH(Et)CH₂NHS(=O)₂Ph(4-CF₃) |
| F-0529 | Cl | F | CH₂CH(Me)CH₂NHS(=O)₂Ph(4-CF₃) |
| F-0530 | Cl | F | CH₂CH₂SiMe₃ |
| F-0531 | Cl | F | CH₂CH₂CH₂SiMe₃ |
| F-0532 | Cl | F | CH₂CH₂CH₂CH₂SiMe₃ |
| F-0533 | Cl | F | CH₂Ph |
| F-0534 | Cl | F | CH₂Ph(2-CF₃) |
| F-0535 | Cl | F | CH₂Ph(3-CF₃) |
| F-0536 | Cl | F | CH₂Ph(4-CF₃) |
| F-0537 | Cl | F | CH₂Ph(2-OCF₃) |
| F-0538 | Cl | F | CH₂Ph(3-OCF₃) |
| F-0539 | Cl | F | CH₂Ph(4-OCF₃) |
| F-0540 | Cl | F | CH₂Ph(2-SCF₃) |
| F-0541 | Cl | F | CH₂Ph(3-SCF₃) |
| F-0542 | Cl | F | CH₂Ph(4-SCF₃) |
| F-0543 | Cl | F | CH₂Ph(3-CH₂SCF₃) |
| F-0544 | Cl | F | CH₂Ph(4-F) |
| F-0545 | Cl | F | CH₂Ph(4-Cl) |
| F-0546 | Cl | F | CH₂Ph(4-Br) |
| F-0547 | Cl | F | CH₂Ph(4-Me) |
| F-0548 | Cl | F | CH₂Ph[4-(t-Bu)] |
| F-0549 | Cl | F | CH₂Ph(4-CN) |
| F-0550 | Cl | F | CH₂Ph(4-NO₂) |
| F-0551 | Cl | F | CH₂Ph(4-OCHF₂) |
| F-0552 | Cl | F | CH₂Ph(4-SCHF₂) |
| F-0553 | Cl | F | CH₂Ph(4-CH₂SCF₃) |
| F-0554 | Cl | F | CH₂Ph[4-CF(CF₃)₂] |
| F-0555 | Cl | F | CH₂Ph(4-CH₂SCF₃) |
| F-0556 | Cl | F | CH₂Ph[4-Ph(4-CF₃)] |
| F-0557 | Cl | F | CH₂Ph(2,4-Cl₂) |
| F-0558 | Cl | F | CH₂Ph[2,5-(CF₃)₂] |
| F-0559 | Cl | F | CH₂Ph(3,4-Cl₂) |
| F-0560 | Cl | F | CH₂Ph(3-CF₃-4-F) |
| F-0561 | Cl | F | CH₂Ph(3-CF₃-4-Cl) |
| F-0562 | Cl | F | CH₂Ph(3-F-4-CF₃) |
| F-0563 | Cl | F | CH₂Ph(2,4,6-F₃) |
| F-0564 | Cl | F | CH₂Ph(3,4,5-F₃) |
| F-0565 | Cl | F | CH₂Ph(2,3,4-F₃) |
| F-0566 | Cl | F | CH₂Ph(3,4,5-Cl₃) |

**[Table 1078]**

| Compound Number | R³ | R⁴ | R⁵ |
|---|---|---|---|
| F-0567 | Cl | F | CH₂CH₂Ph |
| F-0568 | Cl | F | CH₂CH₂Ph(4-F) |
| F-0569 | Cl | F | CH₂CH₂Ph(4-Cl) |
| F-0570 | Cl | F | CH₂CH₂Ph(4-Br) |
| F-0571 | Cl | F | CH₂CH₂Ph[4-(t-Bu)] |
| F-0572 | Cl | F | CH₂CH₂Ph(2-CF₃) |
| F-0573 | Cl | F | CH₂CH₂Ph(3-CF₃) |
| F-0574 | Cl | F | CH₂CH₂Ph(4-CF₃) |
| F-0575 | Cl | F | CH₂CH₂Ph[4-CF(CF₃)₂] |
| F-0576 | Cl | F | CH₂CH₂Ph[4-(*c*-Pr)] |
| F-0577 | Cl | F | CH₂CH₂Ph{4-[*c*-Pr(2,2-F₂)]} |
| F-0578 | Cl | F | CH₂CH₂Ph(4-OMe) |
| F-0579 | Cl | F | CH₂CH₂Ph(3-OCF₃) |
| F-0580 | Cl | F | CH₂CH₂Ph(4-OCHF₂) |
| F-0581 | Cl | F | CH₂CH₂Ph(4-OCF₃) |
| F-0582 | Cl | F | CH₂CH₂Ph(4-SMe) |
| F-0583 | Cl | F | CH₂CH₂Ph(4-SCHF₂) |
| F-0584 | Cl | F | CH₂CH₂Ph(4-SCF₃) |
| F-0585 | Cl | F | CH₂CH₂Ph[4-S(=O)Me] |
| F-0586 | Cl | F | CH₂CH₂Ph[4-S(=O)CF₃] |
| F-0587 | Cl | F | CH₂CH₂Ph[4-S(=O)₂Me] |
| F-0588 | Cl | F | CH₂CH₂Ph[4-S(=O)₂CF₃] |
| F-0589 | Cl | F | CH₂CH₂Ph(4-CH₂SMe) |
| F-0590 | Cl | F | CH₂CH₂Ph(4-CH₂SCF₃) |
| F-0591 | Cl | F | CH₂CH₂Ph[4-OS(=O)₂Me] |
| F-0592 | Cl | F | CH₂CH₂Ph[4-OS(=O)₂CF₃] |
| F-0593 | Cl | F | CH₂CH₂Ph[4-Ph(4-CF₃)] |
| F-0594 | Cl | F | CH₂CH₂Ph(4-CH₂Ph) |
| F-0595 | Cl | F | CH₂CH₂Ph(4-OCH₂Ph) |
| F-0596 | Cl | F | CH₂CH₂Ph(4-CN) |
| F-0597 | Cl | F | CH₂CH₂Ph(4-NO₂) |
| F-0598 | Cl | F | CH₂CH₂Ph(2,4-Cl₂) |
| F-0599 | Cl | F | CH₂CH₂Ph(3,4-Cl₂) |
| F-0600 | Cl | F | CH₂CH₂Ph(3-CF₃-4-F) |
| F-0601 | Cl | F | CH₂CH₂Ph(2-CF₃-4-F) |
| F-0602 | Cl | F | CH₂CH₂Ph(3-F-4-CF₃) |
| F-0603 | Cl | F | CH₂CH₂Ph(2-F-4-CF₃) |
| F-0604 | Cl | F | CH₂CH₂Ph(3-Cl-4-OCHF₂) |
| F-0605 | Cl | F | CH₂CH₂Ph(3,4,5-Cl₃) |
| F-0606 | Cl | F | CH₂CH₂Ph(2,3,4-F₃) |
| F-0607 | Cl | F | CH₂CH₂Ph(2,4,5-F₃) |
| F-0608 | Cl | F | CH₂CH₂Ph(3,4,5-F₃) |
| F-0609 | Cl | F | CH₂CH₂Ph(2,4,6-F₃) |
| F-0610 | Cl | F | CH₂CH₂CH₂Ph |

**[Table 1079]**

| Compound Number | R³ | R⁴ | R⁵ |
|---|---|---|---|
| F-0611 | Cl | F | CH₂CH₂CH₂Ph(3-CF₃) |
| F-0612 | Cl | F | CH₂CH₂CH₂Ph(4-CF₃) |
| F-0613 | Cl | F | CH₂CH₂CH₂Ph(4-F) |
| F-0614 | Cl | F | CH₂CH₂CH₂Ph[4-(t-Bu)] |
| F-0615 | Cl | F | CH₂CH₂CH₂Ph(4-CN) |
| F-0616 | Cl | F | CH₂CH₂CH₂Ph(4-CF₃) |
| F-0617 | Cl | F | CH₂CH₂CH₂Ph(4-OCHF₂) |
| F-0618 | Cl | F | CH₂CH₂CH₂Ph(4-OCF₃) |
| F-0619 | Cl | F | CH₂CH₂CH₂Ph(4-SCHF₂) |
| F-0620 | Cl | F | CH₂CH₂CH₂Ph(4-SCF₃) |
| F-0621 | Cl | F | CH₂CH₂CH₂Ph[4-CF(CF₃)₂) |
| F-0622 | Cl | F | CH₂CH₂CH₂Ph(3,4,5-F₃) |
| F-0623 | Cl | F | CH₂CH₂CH₂Ph(2,4,6-F₃) |
| F-0624 | Cl | F | CH₂CH₂CH₂CH₂Ph |
| F-0625 | Cl | F | CH₂CH₂CH₂CH₂Ph(4-F) |
| F-0626 | Cl | F | CH₂CH₂CH₂CH₂Ph(4-CF₃) |
| F-0627 | Cl | F | CH₂CH₂CH₂CH₂Ph(4-OCF₃) |
| F-0628 | Cl | F | CH₂CH₂CH₂CH₂Ph(4-SCF₃) |
| F-0629 | Cl | F | CH₂CH₂CH₂CH₂CH₂Ph |
| F-0630 | Cl | F | CH₂CH₂CH₂CH₂CH₂CH₂Ph |
| F-0631 | Cl | F | CH₂CF₂Ph(4-F) |
| F-0632 | Cl | F | CH₂CF₂Ph(4-CF₃) |
| F-0633 | Cl | F | CH₂CF₂Ph(3,4,5-F₃) |
| F-0634 | Cl | F | CH₂CH₂OPh |
| F-0635 | Cl | F | CH₂CH₂OPh(4-F) |
| F-0636 | Cl | F | CH₂CH₂OPh(4-CF₃) |
| F-0637 | Cl | F | CH₂CH₂CH₂OPh |
| F-0638 | Cl | F | CH₂CH₂CH₂OPh(4-Cl) |
| F-0639 | Cl | F | CH₂CH₂CH₂OPh(4-CF₃) |
| F-0640 | Cl | F | CH₂CH₂CH₂CH₂OPh(4-CF₃) |
| F-0641 | Cl | F | CH₂CH₂CH₂CH₂OPh(4-OCF₃) |
| F-0642 | Cl | F | CH₂CH₂CH₂CH₂CH₂OPh(4-CF₃) |
| F-0643 | Cl | F | CH₂CH₂OCH₂Ph |
| F-0644 | Cl | F | CH₂CH₂CH₂OCH₂Ph |
| F-0645 | Cl | F | CH₂CH₂CH₂CH₂OCH₂Ph |
| F-0646 | Cl | F | CH₂CH₂CH₂CH₂CH₂OCH₂Ph |
| F-0647 | Cl | F | CH₂CH₂SPh |
| F-0648 | Cl | F | CH₂CH₂CH₂SPh |
| F-0649 | Cl | F | CH₂CH₂CH₂SPh(4-F) |
| F-0650 | Cl | F | CH₂CH₂CH₂SPh(4-Cl) |
| F-0651 | Cl | F | CH₂CH₂CH₂SPh(4-Br) |
| F-0652 | Cl | F | CH₂CH₂CH₂SPh[4-(t-Bu)] |
| F-0653 | Cl | F | CH₂CH₂CH₂SPh(3-CF₃) |
| F-0654 | Cl | F | CH₂CH₂CH₂SPh(4-CF₃) |

**[Table 1080]**

| Compound Number | R³ | R⁴ | R⁵ |
|---|---|---|---|
| F-0655 | Cl | F | CH₂CH₂CH₂SPh(3-SCF₃) |
| F-0656 | Cl | F | CH₂CH₂CH₂SPh(4-SCF₃) |
| F-0657 | Cl | F | CH₂CH₂CH₂CH₂SPh |
| F-0658 | Cl | F | CH₂CH₂CH₂CH₂SPh(4-F) |
| F-0659 | Cl | F | CH₂CH₂CH₂CH₂SPh(4- Cl ) |
| F-0660 | Cl | F | CH₂CH₂CH₂CH₂SPh[4-(t-Bu)] |
| F-0661 | Cl | F | CH₂CH₂CH₂CH₂SPh(4-CF₃) |
| F-0662 | Cl | F | CH₂CH₂CH₂CH₂CH₂SPh |
| F-0663 | Cl | F | CH₂CH₂CH₂CH₂CH₂SPh(4-F) |
| F-0664 | Cl | F | CH₂CH₂CH₂CH₂CH₂SPh(4-Cl) |
| F-0665 | Cl | F | CH₂CH₂CH₂CH₂CH₂SPh(4-CF₃) |
| F-0666 | Cl | F | CH₂CH₂S(=O)Ph |
| F-0667 | Cl | F | CH₂CH₂CH₂S(=O)Ph |
| F-0668 | Cl | F | CH₂CH₂CH₂S(=O)Ph(4-F) |
| F-0669 | Cl | F | CH₂CH₂CH₂S(=O)Ph[4-(t-Bu)] |
| F-0670 | Cl | F | CH₂CH₂CH₂S(=O)Ph(4-CF₃) |
| F-0671 | Cl | F | CH₂CH₂S(⁼O)₂Ph |
| F-0672 | Cl | F | CH₂CH₂S(=O)₂Ph(4-CF₃) |
| F-0673 | Cl | F | CH₂CH₂CH₂S(=O)₂Ph(4-F) |
| F-0674 | Cl | F | CH₂CH₂CH₂S(=O)₂Ph(4-Cl) |
| F-0675 | Cl | F | CH₂CH₂CH₂S(=O)₂Ph(4-CF₃) |
| F-0676 | Cl | F | CH₂CH₂CH₂CH₂S(=O)₂Ph |
| F-0677 | Cl | F | CH₂CH₂CH₂CH₂S(=O)₂Ph(4-Cl) |
| F-0678 | Cl | F | CH₂CH₂CH₂CH₂S(=O)₂Ph(4-CF₃) |
| F-0679 | Cl | F | CH₂CH₂SCH₂Ph |
| F-0680 | Cl | F | CH₂CH₂CH₂SCH₂Ph |
| F-0681 | Cl | F | CH₂CH₂CH₂SCH₂Ph(2-Cl) |
| F-0682 | Cl | F | CH₂CH₂CH₂SCH₂Ph(3-Cl) |
| F-0683 | Cl | F | CH₂CH₂CH₂SCH₂Ph(4-Cl) |
| F-0684 | Cl | F | CH₂CH₂CH₂SCH₂Ph(2-SCF₃) |
| F-0685 | Cl | F | CH₂CH₂CH₂SCH₂Ph(3-CF₃) |
| F-0686 | Cl | F | CH₂CH₂CH₂SCH₂Ph(4-CF₃) |
| F-0687 | Cl | F | CH₂CH₂CH₂SCH₂Ph(4-NO₂) |
| F-0688 | Cl | F | CH₂CH₂CH₂CH₂SCH₂Ph |
| F-0689 | Cl | F | CH₂CH₂CH₂CH₂SCH₂Ph(4-Cl) |
| F-0690 | Cl | F | CH₂CH₂CH₂CH₂SCH₂Ph(4-CF₃) |
| F-0691 | Cl | F | CH₂CH₂CH₂CH₂SCH₂Ph(4-CN) |
| F-0692 | Cl | F | CH₂CH₂CH₂CH₂CH₂SCH₂Ph |
| F-0693 | Cl | F | CH₂CH₂SCH₂CH₂Ph |
| F-0694 | Cl | F | CH₂CH₂CH₂SCH₂CH₂Ph |
| F-0695 | Cl | F | CH₂CH₂CH₂CH₂SCH₂CH₂Ph |
| F-0696 | Cl | F | CH₂CH₂SCH(Me)Ph |
| F-0697 | Cl | F | CH₂CH₂CH₂SCH(Me)Ph |
| F-0698 | Cl | F | CH₂CH₂CH₂CH₂SCH(Me)Ph |

**[Table 1081]**

| Compound Number | R³ | R⁴ | R⁵ |
|---|---|---|---|
| F-0699 | Cl | F | CH₂CH₂S(=O)CH₂Ph |
| F-0700 | Cl | F | CH₂CH₂S(=O)CH₂Ph(4-CF₃) |
| F-0701 | Cl | F | CH₂CH₂CH₂S(=O)CH₂Ph |
| F-0702 | Cl | F | CH₂CH₂CH₂S(=O)CH₂Ph(2-SCF₃) |
| F-0703 | Cl | F | CH₂CH₂CH₂S(=O)CH₂Ph(4-SCF₃) |
| F-0704 | Cl | F | CH₂CH₂CH₂S(=O)CH₂Ph(4-Cl) |
| F-0705 | Cl | F | CH₂CH₂CH₂S(=O)CH₂Ph(4-CF₃) |
| F-0706 | Cl | F | CH₂CH₂CH₂CH₂S(=O)CH₂Ph |
| F-0707 | Cl | F | CH₂CH₂CH₂CH₂S(=O)CH₂Ph(4-CF₃) |
| F-0708 | Cl | F | CH₂CH₂CH₂CH₂CH₂S(=O)CH₂Ph(4-CF₃) |
| F-0709 | Cl | F | CH₂CH₂S(=O)₂CH₂Ph |
| F-0710 | Cl | F | CH₂CH₂S(=O)₂CH₂Ph(4-CF₃) |
| F-0711 | Cl | F | CH₂CH₂CH₂S(=O)₂CH₂Ph |
| F-0712 | Cl | F | CH₂CH₂CH₂S(=O)₂CH₂Ph(4-Cl) |
| F-0713 | Cl | F | CH₂CH₂CH₂S(=O)₂CH₂Ph(4-CF₃) |
| F-0714 | Cl | F | CH₂CH₂CH₂CH₂S(=O)₂CH₂Ph |
| F-0715 | Cl | F | CH₂CH₂CH₂CH₂S(=O)₂CH₂Ph(4-CF₃) |
| F-0716 | Cl | F | CH₂CH₂CH₂CH₂CH₂S(=O)₂CH₂Ph |
| F-0717 | Cl | F | CH₂CH₂CH₂CH₂CH₂S(=O)₂CH₂Ph(4-CF₃) |
| F-0718 | Cl | F | CH₂CH₂ON=CH(*t*-Bu) |
| F-0719 | Cl | F | CH₂CH₂CH₂ON=CH(*t*-Bu) |
| F-0720 | Cl | F | CH₂CH₂CH₂CH₂O_{N}=C(Me)(*c*-Pr) |
| F-0721 | Cl | F | CH₂CH₂ON=C(Me)CF₃ |
| F-0722 | Cl | F | CH₂CH₂CH₂ON=CHCF₃ |
| F-0723 | Cl | F | CH₂CH₂CH₂ON=C(Me)CF₃ |
| F-0724 | Cl | F | CH₂CH₂CH₂ON=C(Me)CCl₃ |
| F-0725 | Cl | F | CH₂CH₂CH₂CH₂ON=CHCF₃ |
| F-0726 | Cl | F | CH₂CH₂CH₂CH₂ON=C(Me)CF₃ |
| F-0727 | Cl | F | CH₂CH₂CH₂CH₂CH₂ON=C(Me)CF₃ |
| F-0728 | Cl | F | CH₂CH₂ON=CHPh |
| F-0729 | Cl | F | CH₂CH₂ON=CHPh(4-CF₃) |
| F-0730 | Cl | F | CH₂CH₂ON=CHPh(4-SCF₃) |
| F-0731 | Cl | F | CH₂CH₂CH₂ON=CHPh(3-CF₃) |
| F-0732 | Cl | F | CH₂CH₂CH₂ON=CHPh(4-CF₃) |
| F-0733 | Cl | F | CH₂CH₂CH₂ON=CHPh(4-SCF₃) |
| F-0734 | Cl | F | CH₂CH₂CH₂ON=C(Me)Ph(4-CF₃) |
| F-0735 | Cl | F | CH₂CH₂CH₂CH₂ON=CHPh(4-SCF₃) |
| F-0736 | Cl | F | CH₂CH₂(adamant-1-yl) |
| F-0737 | Cl | F | CH₂CH₂CH₂(3-CF₃-pyrazol-1-yl) |
| F-0738 | Cl | F | CH₂CH₂CH₂CH₂(3-CF₃-pyrazol-1-yl) |
| F-0739 | Cl | F | CH₂CH₂CH₂CH₂CH₂(3-CF₃-pyrazol-1-yl) |
| F-0740 | Cl | F | CH₂(1-Me-4-CF₃-imidazol-5-yl) |
| F-0741 | Cl | F | CH₂CH₂(1-Me-4-CF₃-imidazol-5-yl) |
| F-0742 | Cl | F | CH₂CH₂CH₂(1-Me-4-CF₃-imidazol-5-yl) |

**[Table 1082]**

| Compound Number | R³ | R⁴ | R⁵ |
|---|---|---|---|
| F-0743 | Cl | F | CH₂CH₂CH₂(3-CF₃-1,2,4-triazol-1-yl) |
| F-0744 | Cl | F | CH₂CH₂CH₂CH₂(3-CF₃-1,2,4-triazol-1-yl) |
| F-0745 | Cl | F | CH₂CH₂CH₂CH₂CH₂(3-CF₃-1,2,4-triazol-1-yl) |
| F-0746 | Cl | F | CH₂CH₂CH₂(4-CF₃-thiazol-2-yl) |
| F-0747 | Cl | F | CH₂CH₂CH₂CH₂(4-CF₃-thiazol-2-yl) |
| F-0748 | Cl | F | CH₂CH₂CH₂CH₂CH₂(4-CF₃-thiazol-2-yl) |
| F-0749 | Cl | F | CH₂CH₂CH₂(2-CF₃-pyridin-5-yl) |
| F-0750 | Cl | F | CH₂CH₂CH₂CH₂(2-CF₃-pyridin-5-yl) |
| F-0751 | Cl | F | CH₂CH₂CH₂CH₂CH₂(2-CF₃-pyridin-5-yl) |
| F-0752 | Cl | F | CH₂(4-CF₃-pyrimidin-2-yl) |
| F-0753 | Cl | F | CH₂CH₂(4-CF₃-pyrimidin-2-yl) |
| F-0754 | Cl | F | CH₂CH₂CH₂(4-CF₃-pyrimidin-2-yl) |
| F-0755 | Cl | F | CH₂CH₂O(3-Cl-5-CF₃-pyridin-2-yl) |
| F-0756 | Cl | F | CH₂CH₂CH₂O(3-Cl-5-CF₃-pyridin-2-yl) |
| F-0757 | Cl | F | CH₂CH₂CH₂CH₂O(3-Cl-5-CF₃-pyridin-2-yl) |
| F-0758 | Cl | F | CH₂CH₂CH₂CH₂CH₂O(3-Cl-5-CF₃-pyridin-2-yl) |
| F-0759 | Cl | F | CH₂CH₂N(Phth) |
| F-0760 | Cl | F | CH₂CH₂CH₂N(Phth) |
| F-0761 | Cl | F | CH₂CH₂CH₂CH₂N(Phth) |
| F-0762 | Cl | F | CH₂CH₂CH₂CH₂CH₂N(Phth) |
| F-0763 | Cl | F | CH₂CH(Me)CH₂N(Phth) |
| F-0764 | Cl | F | CH₂CH₂CH₂CN |
| F-0765 | Cl | F | CH₂CH₂CH₂CH₂CN |
| F-0766 | Cl | F | CH₂CH₂CH₂CH₂CH₂CN |
| F-0767 | Cl | F | CH₂CH₂CH₂CH₂CH₂CH₂CN |
| F-0768 | Cl | F | CH₂CH₂CH₂C(=O)OH |
| F-0769 | Cl | F | CH₂CH₂CH₂CH₂C(=O)OH |
| F-0770 | Cl | F | CH₂CH₂CH₂CH₂CH₂C(=O)OH |
| F-0771 | Cl | F | CH₂CH₂SCN |
| F-0772 | Cl | F | CH₂CH₂CH₂SCN |
| F-0773 | Cl | F | CH₂CH₂CH₂CH₂SCN |
| F-0774 | Cl | F | CH₂CH₂CH₂CH₂CH₂SCN |
| F-0775 | Cl | F | CH₂CH₂CH₂CH₂CH₂CH₂SCN |
| F-0776 | Cl | F | CH₂CH₂CH₂CH₂CH₂CH₂CH₂SCN |
| F-0777 | Cl | F | CH₂CH₂CH₂CH₂CH₂CH₂CH₂CH₂SCN |
| F-0778 | Cl | F | CH₂CH₂CH(Me)SCN |
| F-0779 | Cl | F | CH₂CH(Me)CH₂SCN |
| F-0780 | Cl | F | CH(Me)CH₂CH₂SCN |
| F-0781 | Cl | F | CH₂CH₂CH₂CH(Me)SCN |
| F-0782 | Cl | F | CH₂CH₂CH(Me)CH₂SCN |
| F-0783 | Cl | F | CH₂CH(Me)CH₂CH₂SCN |
| F-0784 | Cl | F | CH(Me)CH₂CH₂CH₂SCN |
| F-0785 | Cl | F | CH₂CH₂CH₂CH₂CH(Me)SCN |
| F-0786 | Cl | F | CH₂CH₂CH₂CH(Me)CH₂SCN |

**[Table 1083]**

| Compound Number | R³ | R⁴ | R⁵ |
|---|---|---|---|
| F-0787 | Cl | F | CH₂CH₂CH(Me)CH₂CH₂SCN |
| F-0788 | Cl | F | CH₂CH(Me)CH₂CH₂CH₂SCN |
| F-0789 | Cl | F | CH(Me)CH₂CH₂CH₂CH₂SCN |
| F-0790 | Cl | F | CH₂CH₂ONH₂ |
| F-0791 | Cl | F | CH₂CH₂CH₂ONH₂ |
| F-0792 | Cl | F | CH₂CH₂CH₂CH₂ONH₂ |
| F-0793 | Cl | F | CH₂CH₂CH₂CH₂CH₂ONH₂ |
| F-0794 | Cl | F | CH₂CH₂CH₂CH₂CH₂CH₂ONH₂ |
| F-0795 | Me | F | Me |
| F-0796 | Me | F | Et |
| F-0797 | Me | F | *n-*Pr |
| F-0798 | Me | F | i-Pr |
| F-0799 | Me | F | *n*-Bu |
| F-0800 | Me | F | *n*-Pen |
| F-0801 | Me | F | *n -Hex* |
| F-0802 | Me | F | n -Heptyl |
| F-0803 | Me | F | *n*-Octyl |
| F-0804 | Me | F | n -Nonyl |
| F-0805 | Me | F | n -Decyl |
| F-0806 | Me | F | c-Pr |
| F-0807 | Me | F | *c*-Pen |
| F-0808 | Me | F | c- Hex |
| F-0809 | Me | F | CH₂C(Me)=CH₂ |
| F-0810 | Me | F | CH₂CH₂CH=CH₂ |
| F-0811 | Me | F | CH₂CH₂CH=C(CH₃)₂ |
| F-0812 | Me | F | CH₂CH₂CH₂CH=CH₂ |
| F-0813 | Me | F | CH₂CH₂CH₂CH=C(CH₃)₂ |
| F-0814 | Me | F | CH₂CH₂CH₂CH₂CH=CH₂ |
| F-0815 | Me | F | CH₂CH₂CH₂CH₂CH=C(CH₃)₂ |
| F-0816 | Me | F | CH₂CH₂CH≡CH |
| F-0817 | Me | F | CH₂CH₂CH₂CH≡CH |
| F-0818 | Me | F | CH₂CH₂CH₂CH₂CH≡CH |
| F-0819 | Me | F | CH₂CH₂CH₂Cl |
| F-0820 | Me | F | CH₂CH₂CH₂Br |
| F-0821 | Me | F | CH₂CH₂CH₂CH₂Cl |
| F-0822 | Me | F | CH₂CH₂CH₂CH₂Br |
| F-0823 | Me | F | CH₂CH₂CH₂CH₂CH₂Cl |
| F-0824 | Me | F | CH₂CH₂CH₂CH₂CH₂Br |
| F-0825 | Me | F | CH₂CH₂CH₂CH₂CH₂CH₂Cl |
| F-0826 | Me | F | CH₂CH₂CH₂CH₂CH₂CH₂Br |
| F-0827 | Me | F | CH₂CH₂CH₂CH₂CH₂CH₂CH₂Cl |
| F-0828 | Me | F | CH₂CH₂CH₂CH₂CH₂CH₂CH₂Br |
| F-0829 | Me | F | CH₂CH₂CH₂CH₂CH₂CH₂CH₂CH₂Br |
| F-0830 | Me | F | CH₂CH(Me)CH₂Cl |

**[Table 1084]**

| Compound Number | R³ | R⁴ | R⁵ |
|---|---|---|---|
| F-0831 | Me | F | CH₂CH(Me)CH₂Br |
| F-0832 | Me | F | CH₂CH₂CH(Me)CH₂CH₂Cl |
| F-0833 | Me | F | CH₂CH₂CH(Me)CH₂CH₂Br |
| F-0834 | Me | F | CH₂CH₂CF=CF₂ |
| F-0835 | Me | F | CH₂CH=C(Cl)CF₃ |
| F-0836 | Me | F | CH₂CH₂CH₂CF=CF₂ |
| F-0837 | Me | F | CH₂CH₂CH₂CH₂CF=CF₂ |
| F-0838 | Me | F | CH₂CH₂CH₂CH₂CH₂CF=CF₂ |
| F-0839 | Me | F | CH₂(*t*-Bu) |
| F-0840 | Me | F | CH₂CH₂(*t*-Bu) |
| F-0841 | Me | F | CH₂CH₂CH₂(*t*-Bu) |
| F-0842 | Me | F | CH₂CH₂CH₂CH₂(*t*-Bu) |
| F-0843 | Me | F | CH₂CH₂CH₂CH₂CH₂(*t*-Bu) |
| F-0844 | Me | F | CH₂CH₂CH₂CH₂CH₂CH₂(*t*-Bu) |
| F-0845 | Me | F | CH₂CF₃ |
| F-0846 | Me | F | CH₂CH₂CF₃ |
| F-0847 | Me | F | CH₂CH₂CH₂CF₃ |
| F-0848 | Me | F | CH₂CH₂CH₂CH₂CF₃ |
| F-0849 | Me | F | CH₂CH₂CH₂CH₂CH₂CF₃ |
| F-0850 | Me | F | CH₂CH₂CH₂CH₂CH₂CH₂CF₃ |
| F-0851 | Me | F | CH₂CH₂CH₂CH₂CH₂CH₂CH₂CF₃ |
| F-0852 | Me | F | CH₂CF₂CF₃ |
| F-0853 | Me | F | CF₂CHFCF₃ |
| F-0854 | Me | F | CF₂CF₂CF₃ |
| F-0855 | Me | F | CH₂CF₂CF₂CF₃ |
| F-0856 | Me | F | CH₂CF₂CF₂CF₂CF₃ |
| F-0857 | Me | F | CH₂CF₂CF₂CF₂CHF₂ |
| F-0858 | Me | F | CH(CF₃)CF₃ |
| F-0859 | Me | F | CH₂CF(CF₃)CF₃ |
| F-0860 | Me | F | CH₂CF₂CF(CF₃)CF₃ |
| F-0861 | Me | F | CH₂CF(CF₃)CF₂CF₃ |
| F-0862 | Me | F | CH₂CF₂CF₂CF(CF₃)CF₃ |
| F-0863 | Me | F | CH₂CF₂CF(CF₃)CF₂CF₃ |
| F-0864 | Me | F | CH₂CF(CF₃)CF₂CF₂CF₃ |
| F-0865 | Me | F | CH₂(*c*-Pr) |
| F-0866 | Me | F | CH₂[*c*-Pr(1-Me)] |
| F-0867 | Me | F | CH₂[*c*-Pr(1-Ph)] |
| F-0868 | Me | F | CH₂{*c*-Pr[1-NHS(=O)₂CF₃]} |
| F-0869 | Me | F | CH₂{*c*-Pr[1-Ph(4-CF₃)]} |
| F-0870 | Me | F | CH₂{*c*-Pr[1-Ph(3,4,5-F₃)]} |
| F-0871 | Me | F | CH₂{*c*-Hex[4-(t-Bu)]} |
| F-0872 | Me | F | CH₂[*c*-Hex(4-CF₃)] |
| F-0873 | Me | F | CH₂CH₂(*c*-Pr) |
| F-0874 | Me | F | CH₂CH₂(*c*-Hex) |

**[Table 1085]**

| Compound Number | R³ | R⁴ | R⁵ |
|---|---|---|---|
| F-0875 | Me | F | CH₂CH₂[*c*-Hex(4-CF₃)] |
| F-0876 | Me | F | CH₂CH₂[*c*-Hex(4-SCF₃)] |
| F-0877 | Me | F | CH₂CH₂CH₂(*c*-Pr) |
| F-0878 | Me | F | CH₂CH₂CH₂(*c*-Hex) |
| F-0879 | Me | F | CH₂CH₂CH₂{*c*-Hex[4-(*t*-Bu)]} |
| F-0880 | Me | F | CH₂CH₂CH2[*c*-Hex(4-CF₃)] |
| F-0881 | Me | F | CH₂CH₂CH₂[*c*-Hex(4-SCF₃)] |
| F-0882 | Me | F | CH₂CH₂CH₂CH₂(*c*-Pr) |
| F-0883 | Me | F | CH₂CH₂CH₂CH₂(*c*-Hex) |
| F-0884 | Me | F | CH₂CH₂CH₂CH₂[*c*-Hex(4-CF₃)] |
| F-0885 | Me | F | CH₂[*c*-Pr(2,2-F₂)] |
| F-0886 | Me | F | CH₂[*c*-Hex(4,4-F₂)] |
| F-0887 | Me | F | CH₂CH₂[*c*-Pr(2,2-F₂)] |
| F-0888 | Me | F | CH₂CH₂[*c*-Hex(4,4-F₂)] |
| F-0889 | Me | F | CH₂CH₂CH₂[*c*-Pr(2,2-F₂)] |
| F-0890 | Me | F | CH₂CH₂CH₂CH₂[*c*-Pr(2,2-F₂)] |
| F-0891 | Me | F | CH₂CH₂OH |
| F-0892 | Me | F | CH₂CH₂CH₂OH |
| F-0893 | Me | F | CH₂CH₂CH₂CH₂OH |
| F-0894 | Me | F | CH₂CH₂CH₂CH₂CH₂OH |
| F-0895 | Me | F | CH₂CH₂CH₂CH₂CH₂CH₂OH |
| F-0896 | Me | F | CH₂C(=O)Me |
| F-0897 | Me | F | CH₂CH(OH)Me |
| F-0898 | Me | F | CH₂CH(OH)CF₃ |
| F-0899 | Me | F | CH₂C(OH)₂CF₃ |
| F-0900 | Me | F | CH₂C(CF₃)=NOH |
| F-0901 | Me | F | CH₂C(CF₃)=NOMe |
| F-0902 | Me | F | CH₂CH(CF₃)NH₂ |
| F-0903 | Me | F | CH₂CH(CF₃)NHSO₂CF₃ |
| F-0904 | Me | F | CH₂CH₂OMe |
| F-0905 | Me | F | CH₂CH₂CH₂OMe |
| F-0906 | Me | F | CH₂CH₂CH₂CH₂OMe |
| F-0907 | Me | F | CH₂CH₂CH₂CH₂CH₂OMe |
| F-0908 | Me | F | CH₂CH₂CH₂CH₂CH₂CH₂OMe |
| F-0909 | Me | F | CH₂CH₂OCHF₂ |
| F-0910 | Me | F | CH₂CH₂CH₂OCHF₂ |
| F-0911 | Me | F | CH₂CH₂CH₂CH₂OCHF₂ |
| F-0912 | Me | F | CH₂CH₂CH₂CH₂CH₂OCHF₂ |
| F-0913 | Me | F | CH₂CH₂CH₂CH₂CH₂CH₂OCHF₂ |
| F-0914 | Me | F | CH₂CH₂OCH₂CF₃ |
| F-0915 | Me | F | CH₂CH₂CH₂OCH₂CF₃ |
| F-0916 | Me | F | CH₂CH₂CH₂CH₂OCH₂CF₃ |
| F-0917 | Me | F | CH₂CH₂CH₂CH₂CH₂OCH₂CF₃ |
| F-0918 | Me | F | CH₂CH₂CH₂CH₂CH₂CH₂OCH₂CF₃ |

**[Table 1086]**

| Compound Number | R³ | R⁴ | R⁵ |
|---|---|---|---|
| F-0919 | Me | F | CH₂CH₂CH₂CH₂CH₂CH₂CH₂OCH₂CF₃ |
| F-0920 | Me | F | CH₂CH₂OC(CF₃)₃ |
| F-0921 | Me | F | CH₂CH₂CH₂OC(CF₃)₃ |
| F-0922 | Me | F | CH₂CH₂CH₂CH₂OC(CF₃)₃ |
| F-0923 | Me | F | CH₂CH₂CH₂CH₂CH₂OC(CF₃)₃ |
| F-0924 | Me | F | CF₂CHFOCF₂CF₂CF₃ |
| F-0925 | Me | F | CH₂CH₂₀(*c*-Pr) |
| F-0926 | Me | F | CH₂CH₂₀(*c*-Pen) |
| F-0927 | Me | F | CH₂CH₂₀(*c*-Hex) |
| F-0928 | Me | F | CH₂CH₂CH₂O(*c*-Pr) |
| F-0929 | Me | F | CH₂CH₂CH₂O(*c*-Pen) |
| F-0930 | Me | F | CH₂CH₂CH₂O(*c*-Hex) |
| F-0931 | Me | F | CH₂CH₂CH₂CH₂O(*c*-Pr) |
| F-0932 | Me | F | CH₂CH₂CH₂CH₂O(*c*-Pen) |
| F-0933 | Me | F | CH₂CH₂CH₂CH₂O(*c*-Hex) |
| F-0934 | Me | F | CH₂CH₂O[*c*-Pr(2,2-F₂)] |
| F-0935 | Me | F | CH₂CH₂CH₂O[*c*-Pr(2,2-F₂)] |
| F-0936 | Me | F | CH₂CH₂CH₂CH₂O[*c*-Pr(2,2-F₂)] |
| F-0937 | Me | F | CH₂CH₂OCH₂CH₂OCH₃ |
| F-0938 | Me | F | CH₂CH₂OCH₂CH₂OCH₂CF₃ |
| F-0939 | Me | F | CH₂CF₂OCF₂CF₂OCF₃ |
| F-0940 | Me | F | CF₂CHFOCF₂CF₂OCF₃ |
| F-0941 | Me | F | CF₂CHFOCF₂CF(CF₃)OCF₂CF₂CF₃ |
| F-0942 | Me | F | CH₂CH₂SH |
| F-0943 | Me | F | CH₂CH₂CH₂SH |
| F-0944 | Me | F | CH₂CH₂CH₂CH₂SH |
| F-0945 | Me | F | CH₂CH₂CH₂CH₂CH₂SH |
| F-0946 | Me | F | CH₂CH₂CH₂CH₂CH₂CH₂SH |
| F-0947 | Me | F | CH₂CH₂S(*t*-Bu) |
| F-0948 | Me | F | CH₂CH₂CH₂S(*t*-Bu) |
| F-0949 | Me | F | CH₂CH₂CH₂CH₂S(*t*-Bu) |
| F-0950 | Me | F | CH₂CH₂CH₂CH₂CH₂SCH₃ |
| F-0951 | Me | F | CH₂CH₂CH₂CH₂CH₂SCH(CH₃)₂ |
| F-0952 | Me | F | CH₂CH₂CH₂CH₂CH₂S(*t*-Bu) |
| F-0953 | Me | F | CH₂CH₂CH₂CH₂CH₂CH₂SCH(CH₃)₂ |
| F-0954 | Me | F | CH₂CH₂CH₂CH₂CH₂CH₂S(*t*-Bu) |
| F-0955 | Me | F | CH₂CH₂CH₂CH₂CH₂CH₂CH₂S(*t*-Bu) |
| F-0956 | Me | F | CH₂CH₂SCHF₂ |
| F-0957 | Me | F | CH₂CH₂CH₂SCHF₂ |
| F-0958 | Me | F | CH₂CH₂CH₂CH₂SCHF₂ |
| F-0959 | Me | F | CH₂CH₂CH₂CH₂CH₂SCHF₂ |
| F-0960 | Me | F | CH₂CH₂CH₂CH₂CH₂CH₂SCHF₂ |
| F-0961 | Me | F | CH₂CH₂CH₂CH₂CH₂CH₂CH₂SCHF₂ |
| F-0962 | Me | F | CH₂CH₂SCF₃ |

**[Table 1087]**

| Compound Number | R³ | R⁴ | R⁵ |
|---|---|---|---|
| F-0963 | Me | F | CH₂CH₂CH₂SCF₃ |
| F-0964 | Me | F | CH₂CH₂CH₂CH₂SCF₃ |
| F-0965 | Me | F | CH₂CH₂CH₂CH₂CH₂SCF₃ |
| F-0966 | Me | F | CH₂CH₂CH₂CH₂CH₂CH₂SCF₃ |
| F-0967 | Me | F | CH₂CH₂CH₂CH₂CH₂CH₂CH₂SCF₃ |
| F-0968 | Me | F | CH₂CH₂CH₂CH₂CH₂CH₂CH₂CH₂SCF₃ |
| F-0969 | Me | F | CH₂CH(Me)SCF₃ |
| F-0970 | Me | F | CH(Me)CH₂SCF₃ |
| F-0971 | Me | F | CH₂CH₂CH(Me)SCF₃ |
| F-0972 | Me | F | CH₂CH(Me)CH₂SCF₃ |
| F-0973 | Me | F | CH(Me)CH₂CH₂SCF₃ |
| F-0974 | Me | F | CH₂CH₂CH₂CH(Me)SCF₃ |
| F-0975 | Me | F | CH₂CH₂CH(Me)CH₂SCF₃ |
| F-0976 | Me | F | CH₂CH(Me)CH₂CH₂SCF₃ |
| F-0977 | Me | F | CH(Me)CH₂CH₂CH₂SCF₃ |
| F-0978 | Me | F | CH₂CH₂SCH₂CF₃ |
| F-0979 | Me | F | CH₂CH₂CH₂SCH₂CF₃ |
| F-0980 | Me | F | CH₂CH₂CH₂CH₂SCH₂CF₃ |
| F-0981 | Me | F | CH₂CH₂CH₂CH₂CH₂SCH₂CF₃ |
| F-0982 | Me | F | CH₂CH₂CH₂CH₂CH₂CH₂SCH₂CF₃ |
| F-0983 | Me | F | CH₂CH₂CH₂CH₂CH₂CH₂CH₂SCH₂CF₃ |
| F-0984 | Me | F | CH₂CH₂SCH(CF₃)₂ |
| F-0985 | Me | F | CH₂CH₂CH₂SCH(CF₃)₂ |
| F-0986 | Me | F | CH₂CH₂CH₂CH₂SCH(CF₃)₂ |
| F-0987 | Me | F | CH₂CH₂CH₂CH₂CH₂SCH(CF₃)₂ |
| F-0988 | Me | F | CH₂CH₂CH₂CH₂CH₂CH₂SCH(CF₃)₂ |
| F-0989 | Me | F | CH₂CH₂CH₂CH₂CH₂SCF₂CF₃ |
| F-0990 | Me | F | CH₂CH₂CH₂CH₂CH₂CH₂SCF₂CF₃ |
| F-0991 | Me | F | CH₂CH₂CH₂CH₂CH₂CH₂SCF(CF₃)₂ |
| F-0992 | Me | F | CH₂CH₂CH₂CH₂CH₂CH₂CH₂SCF(CF₃)₂ |
| F-0993 | Me | F | CH₂CH₂CH₂CH₂CH₂SCCl₃ |
| F-0994 | Me | F | CH₂CH₂CH₂CH₂CH₂CH₂SCCl₃ |
| F-0995 | Me | F | CH₂CH₂SCH₂CH=CH₂ |
| F-0996 | Me | F | CH₂CH₂CH₂SCH₂CH=CH₂ |
| F-0997 | Me | F | CH₂CH₂CH₂CH₂SCH₂CH=CH₂ |
| F-0998 | Me | F | CH₂CH₂SCF=CFCF₃ |
| F-0999 | Me | F | CH₂CH₂CH₂SCF=CFCF₃ |
| F-1000 | Me | F | CH₂CH₂CH₂CH₂SCF=CFCF₃ |
| F-1001 | Me | F | CH₂CH₂CH₂S(*c*-Pr) |
| F-1002 | Me | F | CH₂CH₂CH₂S(*c*-Hex) |
| F-1003 | Me | F | CH₂CH₂CH₂CH₂S(*c*-Pr) |
| F-1004 | Me | F | CH₂CH₂CH₂CH₂S(*c*-Hex) |
| F-1005 | Me | F | CH₂CH₂CH₂CH₂CH₂S(*c*-Pr) |
| F-1006 | Me | F | CH₂CH₂CH₂CH₂CH₂S(*c*-Hex) |

**[Table 1088]**

| Compound Number | R³ | R⁴ | R⁵ |
|---|---|---|---|
| F-1007 | Me | F | CH₂CH₂CH₂S[*c*-Hex(4,4-F₂)] |
| F-1008 | Me | F | CH₂CH₂CH₂CH₂S[*c*-Hex(4,4-F₂)] |
| F-1009 | Me | F | CH₂CH₂CH₂CH₂CH₂S[*c*-Hex(4,4-F₂)] |
| F-1010 | Me | F | CH₂CH₂CH₂SCH₂(*c*-Pr) |
| F-1011 | Me | F | CH₂CH₂CH₂SCH₂(*c*-Hex) |
| F-1012 | Me | F | CH₂CH₂CH₂CH₂SCH₂(*c*-Pr) |
| F-1013 | Me | F | CH₂CH₂CH₂CH₂SCH₂(*c*-Hex) |
| F-1014 | Me | F | CH2CH2CH2CH2CH2SCH2(c- Pr) |
| F-1015 | Me | F | CH₂CH₂CH₂CH₂CH₂SCH₂(*c*-Hex) |
| F-1016 | Me | F | CH₂CH₂CH₂SCH₂[*c*-Hex(4,4-F₂)] |
| F-1017 | Me | F | CH₂CH₂CH₂CH₂SCH₂[*c*-Hex(4,4-F₂)] |
| F-1018 | Me | F | CH₂CH₂CH₂CH₂CH₂SCH2[*c*-Hex(4,4-F₂)] |
| F-1019 | Me | F | CH₂CH₂CH₂SCH₂SiMe₃ |
| F-1020 | Me | F | CH₂CH₂CH₂CH₂SCH₂SiMe₃ |
| F-1021 | Me | F | CH₂CH₂CH₂CH₂CH₂SCH₂SiMe₃ |
| F-1022 | Me | F | CH₂CH₂OCH₂CH₂SCH₃ |
| F-1023 | Me | F | CH₂CH₂OCH₂CH₂SCHF₂ |
| F-1024 | Me | F | CH₂CH₂OCH₂CH₂SCF₃ |
| F-1025 | Me | F | CH₂CH₂OCH₂CH₂SCH₂CF₃ |
| F-1026 | Me | F | CH₂CH₂CH₂S(=O)(*t*-Bu) |
| F-1027 | Me | F | CH₂CH₂CH₂CH₂S(=O)(*t*-Bu) |
| F-1028 | Me | F | CH₂CH₂CH₂CH₂CH₂CH₂S(=O)(*t*-Bu) |
| F-1029 | Me | F | CH₂CH₂S(=O)CHF₂ |
| F-1030 | Me | F | CH₂CH₂CH₂S(=O)CHF₂ |
| F-1031 | Me | F | CH₂CH₂CH₂CH₂S(=O)CHF₂ |
| F-1032 | Me | F | CH₂CH₂CH₂CH₂CH₂S(=O)CHF₂ |
| F-1033 | Me | F | CH₂CH₂CH₂CH₂CH₂CH₂S(=O)CHF₂ |
| F-1034 | Me | F | CH₂CH₂CH₂CH₂CH₂CH₂CH₂S(=O)CHF₂ |
| F-1035 | Me | F | CH₂CH₂S(=O)CF₃ |
| F-1036 | Me | F | CH₂CH₂CH₂S(=O)CF₃ |
| F-1037 | Me | F | CH₂CH₂CH₂CH₂S(=O)CF₃ |
| F-1038 | Me | F | CH₂CH₂CH₂CH₂CH₂S(=O)CF₃ |
| F-1039 | Me | F | CH₂CH₂CH₂CH₂CH₂CH₂S(=O)CF₃ |
| F-1040 | Me | F | CH₂CH₂CH₂CH₂CH₂CH₂CH₂S(=O)CF₃ |
| F-1041 | Me | F | CH₂CH₂S(=O)CH₂CF₃ |
| F-1042 | Me | F | CH₂CH₂CH₂S(=O)CH₂CF₃ |
| F-1043 | Me | F | CH₂CH₂CH₂CH₂S(=O)CH₂CF₃ |
| F-1044 | Me | F | CH₂CH₂CH₂CH₂CH₂S(=O)CH₂CF₃ |
| F-1045 | Me | F | CH₂CH₂CH₂CH₂CH₂CH₂S(=O)CH₂CF₃ |
| F-1046 | Me | F | CH₂CH₂CH₂CH₂CH₂CH₂CH₂S(=O)CH₂CF₃ |
| F-1047 | Me | F | CH₂CH₂CH₂S(=O)(*c*-Pr) |
| F-1048 | Me | F | CH₂CH₂CH₂CH₂S(=O)(*c*-Pr) |
| F-1049 | Me | F | CH₂CH₂CH₂CH₂S(=O)(*c*-Pen) |
| F-1050 | Me | F | CH₂CH₂CH₂CH₂S(=O)(*c*-Hex) |

**[Table 1089]**

| Compound Number | R³ | R⁴ | R⁵ |
|---|---|---|---|
| F-1051 | Me | F | CH₂CH₂CH₂CH₂CH₂S(=O)(*c*-Pr) |
| F-1052 | Me | F | CH₂CH₂CH₂CH₂CH₂S(=O)(*c*-Hex) |
| F-1053 | Me | F | CH₂CH₂CH₂S(=O)[*c*-Hex(4,4-F₂)] |
| F-1054 | Me | F | CH₂CH₂CH₂CH₂S(=O)[*c*-Hex(4,4-F₂)] |
| F-1055 | Me | F | CH₂CH₂CH₂CH₂CH₂S(=O)[*c*-Hex(4,4-F₂)] |
| F-1056 | Me | F | CH₂CH₂CH₂CH₂S(=O)₂Me |
| F-1057 | Me | F | CH₂CH₂CH₂CH₂CH₂S(=O)₂Me |
| F-1058 | Me | F | CH₂CH₂CH₂CH₂CH₂S(=O)₂(*t*-Bu) |
| F-1059 | Me | F | CH₂CH₂S(=O)₂CHF₂ |
| F-1060 | Me | F | CH₂CH₂CH₂S(=O)₂CHF₂ |
| F-1061 | Me | F | CH₂CH₂CH₂CH₂S(=O)₂CHF₂ |
| F-1062 | Me | F | CH₂CH₂CH₂CH₂CH₂S(=O)₂CHF₂ |
| F-1063 | Me | F | CH₂CH₂CH₂CH₂CH₂CH₂S(=O)₂CHF₂ |
| F-1064 | Me | F | CH₂CH₂S(=O)₂CF₃ |
| F-1065 | Me | F | CH₂CH₂CH₂S(=O)₂CF₃ |
| F-1066 | Me | F | CH₂CH₂CH₂CH₂S(=O)₂CF₃ |
| F-1067 | Me | F | CH₂CH₂CH₂CH₂CH₂S(=O)₂CF₃ |
| F-1068 | Me | F | CH₂CH₂CH₂CH₂CH₂CH₂S(=O)₂CF₃ |
| F-1069 | Me | F | CH₂CH₂CH₂CH₂CH₂CH₂CH₂S(=O)₂CF₃ |
| F-1070 | Me | F | CH₂CH₂CH₂CH₂CH₂CH₂CH₂CH₂S(=O)₂CF₃ |
| F-1071 | Me | F | CH₂CH₂S(=O)₂CH₂CF₃ |
| F-1072 | Me | F | CH₂CH₂CH₂S(=O)₂CH₂CF₃ |
| F-1073 | Me | F | CH₂CH₂CH₂CH₂S(=O)₂CH₂CF₃ |
| F-1074 | Me | F | CH₂CH₂CH₂CH₂CH₂S(=O)₂CH₂CF₃ |
| F-1075 | Me | F | CH₂CH₂CH₂CH₂CH₂CH₂S(=O)₂CH₂CF₃ |
| F-1076 | Me | F | CH₂CH₂CH₂S(=O)₂(*c*-Pr) |
| F-1077 | Me | F | CH₂CH₂CH₂CH₂S(=O)₂(*c*-Pr) |
| F-1078 | Me | F | CH₂CH₂CH₂CH₂CH₂S(=O)₂(*c*-Pr) |
| F-1079 | Me | F | CH₂CH₂CH₂S(=O)₂[*c*-Hex(4,4-F₂)] |
| F-1080 | Me | F | CH₂CH₂CH₂CH₂S(=O)₂[*c*-Hex(4,4-F₂)] |
| F-1081 | Me | F | CH₂CH₂CH₂CH₂CH₂S(=O)₂[*c*-Hex(4,4-F₂)] |
| F-1082 | Me | F | CH₂CH₂CH₂C(=O)H |
| F-1083 | Me | F | CH₂CH₂CH₂CH₂C(=O)H |
| F-1084 | Me | F | CH₂CH₂CH₂CH₂CH₂C(=O)H |
| F-1085 | Me | F | CH₂CH₂CH₂C(=O)(*t*-Bu) |
| F-1086 | Me | F | CH₂CH₂CH₂CH₂C(=O)(*t*-Bu) |
| F-1087 | Me | F | CH₂CH₂CH₂CH₂CH₂C(=O)(*t*-Bu) |
| F-1088 | Me | F | CH₂CH₂CH₂C(=O)CF₃ |
| F-1089 | Me | F | CH₂CH₂CH₂CH₂C(=O)CF₃ |
| F-1090 | Me | F | CH₂CH₂CH₂CH₂CH₂C(=O)CF₃ |
| F-1091 | Me | F | CH₂C(=O)Ph |
| F-1092 | Me | F | CH₂C(=O)Ph(4-Cl) |
| F-1093 | Me | F | CH₂C(=O)Ph(4-CF₃) |
| F-1094 | Me | F | CH₂CH₂C(=O)Ph(4-CF₃) |

**[Table 1090]**

| Compound Number | R³ | R⁴ | R⁵ |
|---|---|---|---|
| F-1095 | Me | F | CH₂CH₂CH₂C(=O)Ph(4-CF₃) |
| F-1096 | Me | F | CH₂CH₂CH₂CH₂C(=O)Ph(4-CF₃) |
| F-1097 | Me | F | CH₂CH₂CH₂CH₂CH₂C(=O)Ph(4-CF₃) |
| F-1098 | Me | F | CH₂C(=O)OEt |
| F-1099 | Me | F | CH₂CH₂C(=O)O(*t*-Bu) |
| F-1100 | Me | F | CH₂CH₂CH₂C(=O)OEt |
| F-1101 | Me | F | CH₂CH₂CH₂C(=O)O(*t*-Bu) |
| F-1102 | Me | F | CH₂CH₂CH₂CH₂C(=O)O(*t*-Bu) |
| F-1103 | Me | F | CH₂CH₂CH₂CH₂CH₂C(=O)O(*t*-Bu) |
| F-1104 | Me | F | CH₂CH₂CH₂CH₂CH₂CH₂C(=O)O(*t*-Bu) |
| F-1105 | Me | F | CH₂CH₂CH₂CH₂CH₂CH₂CH₂C(=O)OEt |
| F-1106 | Me | F | CH₂CH₂C(=O)OCH₂CF₃ |
| F-1107 | Me | F | CH₂CH₂CH₂C(=O)OCH₂CF₃ |
| F-1108 | Me | F | CH₂CH₂CH₂CH₂C(=O)OCH₂CF₃ |
| F-1109 | Me | F | CH₂CH₂CH₂CH₂CH₂C(=O)OCH₂CF₃ |
| F-1110 | Me | F | CH₂CH₂C(=O)NH(*t*-Bu) |
| F-1111 | Me | F | CH₂CH₂CH₂C(=O)NH(*t*-Bu) |
| F-1112 | Me | F | CH₂CH₂CH₂C(=O)NH(*t*-Pen) |
| F-1113 | Me | F | CH₂CH₂CH₂CH₂C(=O)NH(*t*-Bu) |
| F-1114 | Me | F | CH₂CH₂C(=O)NHCH₂CF₃ |
| F-1115 | Me | F | CH₂CH₂CH₂C(=O)NHCH₂CF₃ |
| F-1116 | Me | F | CH₂CH₂CH₂CH₂C(=O)NHCH₂CF₃ |
| F-1117 | Me | F | CH₂CH₂SC(=O)N(Me)₂ |
| F-1118 | Me | F | CH₂CH₂CH₂SC(=O)N(Me)₂ |
| F-1119 | Me | F | CH₂CH₂CH₂SC(=O)NH(*t*-Bu) |
| F-1120 | Me | F | CH₂CH₂CH₂CH₂CH₂SC(=O)NH(*t*-Bu) |
| F-1121 | Me | F | CH₂CH₂SC(=O)NHCH₂CF₃ |
| F-1122 | Me | F | CH₂CH₂CH₂SC(=O)NHCH₂CF₃ |
| F-1123 | Me | F | CH₂CH₂CH₂CH₂SC(=O)NHCH₂CF₃ |
| F-1124 | Me | F | CH₂CH₂CH₂OC(=O)H |
| F-1125 | Me | F | CH₂CH₂CH₂CH₂OC(=O)H |
| F-1126 | Me | F | CH₂CH₂CH₂CH₂CH₂OC(=O)H |
| F-1127 | Me | F | CH₂CH₂CH₂OC(=O)Me |
| F-1128 | Me | F | CH₂CH₂CH₂CH₂OC(=O)Me |
| F-1129 | Me | F | CH₂CH₂CH₂CH₂CH₂OC(=O)Me |
| F-1130 | Me | F | CH₂CH₂OC(=O)CF₃ |
| F-1131 | Me | F | CH₂CH₂CH₂OC(=O)CF₃ |
| F-1132 | Me | F | CH₂CH₂CH₂CH₂OC(=O)CF₃ |
| F-1133 | Me | F | CH₂CH₂CH₂CH₂CH₂OC(=O)Ph(4-CF₃) |
| F-1134 | Me | F | CH₂CH₂OC(=O)Ph |
| F-1135 | Me | F | CH₂CH₂OC(=O)Ph(4-CF₃) |
| F-1136 | Me | F | CH₂CH₂CH₂OC(=O)Ph(4-CF₃) |
| F-1137 | Me | F | CH₂CH₂CH₂CH₂OC(=O)Ph(4-CF₃) |
| F-1138 | Me | F | CH₂CH₂CH₂CH₂CH₂OC(=O)Ph(4-CF₃) |

**[Table 1091]**

| Compound Number | R³ | R⁴ | R⁵ |
|---|---|---|---|
| F-1139 | Me | F | CH₂CH₂OS(=O)Me |
| F-1140 | Me | F | CH₂CH₂CH₂OS(=O)Me |
| F-1141 | Me | F | CH₂CH₂CH₂CH₂OS(=O)Me |
| F-1142 | Me | F | CH₂CH₂CH₂CH₂CH₂OS(=O)Me |
| F-1143 | Me | F | CH₂CH₂CH₂CH₂CH₂CH₂OS(=O)Me |
| F-1144 | Me | F | CH₂CH₂OS(=O)CF₃ |
| F-1145 | Me | F | CH₂CH₂CH₂OS(=O)CF₃ |
| F-1146 | Me | F | CH₂CH₂CH₂CH₂OS(=O)CF₃ |
| F-1147 | Me | F | CH₂CH₂CH₂CH₂CH₂OS(=O)CF₃ |
| F-1148 | Me | F | CH₂CH₂CH₂CH₂CH₂CH₂OS(=O)CF₃ |
| F-1149 | Me | F | CH₂CH₂OS(=O)Ph |
| F-1150 | Me | F | CH₂CH₂OS(=O)Ph(4-Me) |
| F-1151 | Me | F | CH₂CH₂CH₂OS(=O)Ph |
| F-1152 | Me | F | CH₂CH₂CH₂OS(=O)Ph(4-Me) |
| F-1153 | Me | F | CH₂CH₂OS(=O)₂Me |
| F-1154 | Me | F | CH₂CH₂CH₂OS(=O)₂Me |
| F-1155 | Me | F | CH₂CH₂CH₂OS(=O)₂CF₃ |
| F-1156 | Me | F | CH₂CH₂CH₂CH₂OS(=O)₂CF₃ |
| F-1157 | Me | F | CH₂CH₂CH₂CH₂CH₂OS(=O)₂CF₃ |
| F-1158 | Me | F | CH₂CH₂CH₂CH₂CH₂OS(=O)₂CF₂CF₂CF₂CF₃ |
| F-1159 | Me | F | CH₂CH₂OS(=O)₂Ph |
| F-1160 | Me | F | CH₂CH₂OS(=O)₂Ph(4-Me) |
| F-1161 | Me | F | CH₂CH₂CH₂OS(=O)₂Ph |
| F-1162 | Me | F | CH₂CH₂CH₂OS(=O)₂Ph(4-Me) |
| F-1163 | Me | F | CH₂CH₂NH₂ |
| F-1164 | Me | F | CH₂CH₂CH₂NH₂ |
| F-1165 | Me | F | CH₂CH₂CH₂CH₂NH₂ |
| F-1166 | Me | F | CH₂CH₂CH₂CH₂CH₂NH₂ |
| F-1167 | Me | F | CH₂CH₂CH₂CH₂CH₂CH₂NH₂ |
| F-1168 | Me | F | CH₂CH₂CH₂CH₂CH₂CH₂CH₂NH₂ |
| F-1169 | Me | F | CH₂CH(Me)NH₂ |
| F-1170 | Me | F | CH₂C*H(Me)NH₂ : (R) |
| F-1171 | Me | F | CH₂C*H(Me)NH₂ : (S) |
| F-1172 | Me | F | CH₂CH(Et)NH₂ |
| F-1173 | Me | F | CH₂CH(*i*-Pr)NH₂ |
| F-1174 | Me | F | CH(Me)CH₂NH₂ |
| F-1175 | Me | F | CH(Et)CH₂NH₂ |
| F-1176 | Me | F | CH(*i*-Pr)CH₂NH₂ |
| F-1177 | Me | F | CH₂CH₂CH(Me)NH₂ |
| F-1178 | Me | F | CH₂CH(Me)CH₂NH₂ |
| F-1179 | Me | F | CH(Me)CH₂CH₂NH₂ |
| F-1180 | Me | F | CH₂CH(Me)NH(*c*-Pr) |
| F-1181 | Me | F | CH₂CH(Me)N(*c*-Pr)S(=O)₂CF₃ |
| F-1182 | Me | F | CH₂CH(Me)NHOMe |

**[Table 1092]**

| Compound Number | R³ | R⁴ | R⁵ |
|---|---|---|---|
| F-1183 | Me | F | CH₂CH(Me)N(OMe)S(=O)₂CF₃ |
| F-1184 | Me | F | CH₂CH(Me)N(OMe)S(=O)₂Ph(2-NO₂) |
| F-1185 | Me | F | CH₂CH(Me)N(OMe)S(=O)₂Ph(4-NO₂) |
| F-1186 | Me | F | CH₂CH₂N(Me)(*t*-Bu) |
| F-1187 | Me | F | CH₂CH₂CH₂N(Me)(*t*-Bu) |
| F-1188 | Me | F | CH₂CH₂CH₂CH₂N(Me)(*t*-Bu) |
| F-1189 | Me | F | CH₂CH₂NHCH₂CF₃ |
| F-1190 | Me | F | CH₂CH₂CH₂NHCH₂CF₃ |
| F-1191 | Me | F | CH₂CH₂CH₂CH₂NHCH₂CF₃ |
| F-1192 | Me | F | CH₂CH₂NHC(=O)C(Me)(CF₃)₂ |
| F-1193 | Me | F | CH₂CH₂NHC(=O)Me |
| F-1194 | Me | F | CH₂CH₂NHC(=O)(*t*-Bu) |
| F-1195 | Me | F | CH₂CH₂CH₂CH₂NHC(=O)CH(CH₃)₂ |
| F-1196 | Me | F | CH₂CH₂CH₂CH₂NHC(=O)(*t*-Bu) |
| F-1197 | Me | F | CH₂CH₂CH₂CH₂NHC(=O)CH₂(*t*-Bu) |
| F-1198 | Me | F | CH₂CH₂NHC(=O)CF₃ |
| F-1199 | Me | F | CH₂CH₂NHC(=O)CH₂CF₃ |
| F-1200 | Me | F | CH₂CH₂NHC(=O)CF₂CF₃ |
| F-1201 | Me | F | CH₂CH₂CH₂NHC(=O)CF₃ |
| F-1202 | Me | F | CH₂CH₂CH₂NHC(=O)CH₂CF₃ |
| F-1203 | Me | F | CH₂CH₂CH₂NHC(=O)CF₂CF₃ |
| F-1204 | Me | F | CH₂CH₂CH₂CH₂NHC(=O)CF₃ |
| F-1205 | Me | F | CH₂CH₂CH₂CH₂NHC(=O)CH₂CF₃ |
| F-1206 | Me | F | CH₂CH₂CH₂CH₂NHC(=O)CF₂CF₃ |
| F-1207 | Me | F | CH₂CH₂CH₂CH₂NHC(=O)CF(CF₃)₂ |
| F-1208 | Me | F | CH₂CH₂CH₂CH₂NHC(=O)CCl₃ |
| F-1209 | Me | F | CH₂CH₂NHC(=O)Ph |
| F-1210 | Me | F | CH₂CH₂NHC(=O)Ph(4-CF₃) |
| F-1211 | Me | F | CH₂CH₂CH₂NHC(=O)Ph(4-CF₃) |
| F-1212 | Me | F | CH₂CH₂CH₂CH₂NHC(=O)Ph(4-CF₃) |
| F-1213 | Me | F | CH₂CH₂NHC(=O)O(*t*-Bu) |
| F-1214 | Me | F | CH₂CH₂CH₂NHC(=O)O(*t*-Bu) |
| F-1215 | Me | F | CH₂CH₂CH₂CH₂NHC(=O)O(*t*-Bu) |
| F-1216 | Me | F | CH₂CH₂CH₂CH₂NHC(=O)OCH(CH₃)₂ |
| F-1217 | Me | F | CH₂CH(Me)NHC(=O)O(*t*-Bu) |
| F-1218 | Me | F | CH₂C*H(Me)NHC(=O)O(*t*-Bu) : (R) |
| F-1219 | Me | F | CH₂C*H(Me)NHC(=O)O(*t*-Bu) : (S) |
| F-1220 | Me | F | CH₂CH(Et)NHC(=O)O(*t*-Bu) |
| F-1221 | Me | F | CH₂CH(*i*-Pr)NHC(=O)O(*t*-Bu) |
| F-1222 | Me | F | CH(Me)CH₂NHC(=O)O(*t*-Bu) |
| F-1223 | Me | F | CH(Et)CH₂NHC(=O)O(*t*-Bu) |
| F-1224 | Me | F | CH(*i*-Pr)CH₂NHC(=O)O(*t*-Bu) |
| F-1225 | Me | F | CH₂CH(Me)CH₂NHC(=O)O(*t*-Bu) |
| F-1226 | Me | F | CH₂CH₂NHC(=O)OC(Me)₂CF₃ |

**[Table 1093]**

| Compound Number | R³ | R⁴ | R⁵ |
|---|---|---|---|
| F-1227 | Me | F | CH₂CH₂CH₂NHC(=O)OCH₂CF₃ |
| F-1228 | Me | F | CH₂CH₂CH₂NHC(=O)OCH₂CH₂CF₃ |
| F-1229 | Me | F | CH₂CH₂CH₂CH₂NHC(=O)OCH₂CCl₃ |
| F-1230 | Me | F | CH₂CH₂CH₂CH₂NHC(=O)OCH₂CF₃ |
| F-1231 | Me | F | CH₂CH₂NHC(=O)NH(*t*-Bu) |
| F-1232 | Me | F | CH₂CH₂CH₂NHC(=O)NH(*t*-Bu) |
| F-1233 | Me | F | CH₂CH₂CH₂CH₂NHC(=O)NHEt |
| F-1234 | Me | F | CH₂CH₂CH₂CH₂NHC(=O)NH(*t*-Bu) |
| F-1235 | Me | F | CH₂CH₂CH₂NHC(=O)NHCH₂CCl₃ |
| F-1236 | Me | F | CH₂CH₂CH₂NHC(=O)NHCH₂CH₂F |
| F-1237 | Me | F | CH₂CH₂CH₂NHC(=O)NHCH₂CF₃ |
| F-1238 | Me | F | CH₂CH₂CH₂CH₂NHC(=O)NHCH₂CCl₃ |
| F-1239 | Me | F | CH₂CH₂CH₂CH₂NHC(=O)NHCH₂CH₂F |
| F-1240 | Me | F | CH₂CH₂CH₂CH₂NHC(=O)NHCH₂CF₃ |
| F-1241 | Me | F | CH₂CH₂NHS(=O)Me |
| F-1242 | Me | F | CH₂CH₂CH₂NHS(=O)Me |
| F-1243 | Me | F | CH₂CH₂CH₂CH₂NHS(=O)Me |
| F-1244 | Me | F | CH₂CH₂CH₂CH₂NHS(=O)CH(CH₃)₂ |
| F-1245 | Me | F | CH₂CH₂CH₂CH₂CH₂NHS(=O)Me |
| F-1246 | Me | F | CH₂CH₂NHS(=O)CHF₂ |
| F-1247 | Me | F | CH₂CH₂CH₂NHS(=O)CHF₂ |
| F-1248 | Me | F | CH₂CH₂CH₂CH₂NHS(=O)CHF₂ |
| F-1249 | Me | F | CH₂CH₂CH₂CH₂CH₂NHS(=O)CHF₂ |
| F-1250 | Me | F | CH₂CH(Me)NHS(=O)CHF₂ |
| F-1251 | Me | F | CH(Me)CH₂NHS(=O)CHF₂ |
| F-1252 | Me | F | CH₂CH₂CH(Me)NHS(=O)CHF₂ |
| F-1253 | Me | F | CH₂CH(Me)CH₂NHS(=O)CHF₂ |
| F-1254 | Me | F | CH(Me)CH₂CH₂NHS(=O)CHF₂ |
| F-1255 | Me | F | CH₂CH₂NHS(=O)CF₃ |
| F-1256 | Me | F | CH₂CH₂CH₂NHS(=O)CF₃ |
| F-1257 | Me | F | CH₂CH₂CH₂CH₂NHS(=O)CF₃ |
| F-1258 | Me | F | CH₂CH₂CH₂CH₂CH₂NHS(=O)CF₃ |
| F-1259 | Me | F | CH₂CH₂CH₂NHS(=O)Ph |
| F-1260 | Me | F | CH₂CH₂CH₂CH₂NHS(=O)Ph |
| F-1261 | Me | F | CH₂CH₂CH₂CH₂CH₂NHS(=O)Ph |
| F-1262 | Me | F | CH₂CH₂CH₂CH₂CH₂NHS(=O)Ph(4-CF₃) |
| F-1263 | Me | F | CH₂CH(Me)NHS(=O)Ph(4-CF₃) |
| F-1264 | Me | F | CH₂CH(Et)NHS(=O)Ph(4-CF₃) |
| F-1265 | Me | F | CH(Me)CH₂NHS(=O)Ph(4-CF₃) |
| F-1266 | Me | F | CH(Et)CH₂NHS(=O)Ph(4-CF₃) |
| F-1267 | Me | F | CH₂CH(Et)CH₂NHS(=O)Ph(4-CF₃) |
| F-1268 | Me | F | CH₂CH₂NHS(=O)₂Me |
| F-1269 | Me | F | CH₂CH₂CH₂NHS(=O)₂Me |
| F-1270 | Me | F | CH₂CH₂CH₂CH₂NHS(=O)₂Me |

**[Table 1094]**

| Compound Number | R³ | R⁴ | R⁵ |
|---|---|---|---|
| F-1271 | Me | F | CH₂CH₂CH₂CH₂NHS(=O)₂CH(CH₃)₂ |
| F-1272 | Me | F | CH₂CH₂CH₂CH₂CH₂NHS(=O)₂Me |
| F-1273 | Me | F | CH₂CH₂NHS(=O)₂CHF₂ |
| F-1274 | Me | F | CH₂CH₂CH₂NHS(=O)₂CHF₂ |
| F-1275 | Me | F | CH₂CH₂CH₂CH₂NHS(=O)₂CHF₂ |
| F-1276 | Me | F | CH₂CH₂CH₂CH₂CH₂NHS(=O)₂CHF₂ |
| F-1277 | Me | F | CH₂CH(Me)NHS(=O)₂CHF₂ |
| F-1278 | Me | F | CH(Me)CH₂NHS(=O)₂CHF₂ |
| F-1279 | Me | F | CH₂CH₂CH(Me)NHS(=O)₂CHF₂ |
| F-1280 | Me | F | CH₂CH(Me)CH₂NHS(=O)₂CHF₂ |
| F-1281 | Me | F | CH(Me)CH₂CH₂NHS(=O)₂CHF₂ |
| F-1282 | Me | F | CH₂CH(Me)N(Me)S(=O)₂CHF₂ |
| F-1283 | Me | F | CH(Me)CH₂N(Me)S(=O)₂CHF₂ |
| F-1284 | Me | F | CH₂CH₂CH(Me)N(Me)S(=O)₂CHF₂ |
| F-1285 | Me | F | CH₂CH(Me)CH₂N(Me)S(=O)₂CHF₂ |
| F-1286 | Me | F | CH(Me)CH₂CH₂N(Me)S(=O)₂CHF₂ |
| F-1287 | Me | F | CH₂CH₂NHS(=O)₂CF₃ |
| F-1288 | Me | F | CH₂CH₂CH₂NHS(=O)₂CF₃ |
| F-1289 | Me | F | CH₂CH₂CH₂CH₂NHS(=O)₂CF₃ |
| F-1290 | Me | F | CH₂CH₂CH₂CH₂CH₂NHS(=O)₂CF₃ |
| F-1291 | Me | F | CH₂CH₂N(Me)S(=O)₂CF₃ |
| F-1292 | Me | F | CH₂CH₂N(Et)S(=O)₂CF₃ |
| F-1293 | Me | F | CH₂CH₂N(CH₂CN)S(=O)₂CF₃ |
| F-1294 | Me | F | CH₂CH₂N(CH₂OMe)S(=O)₂CF₃ |
| F-1295 | Me | F | CH₂CH₂CH₂N(Me)S(=O)₂CF₃ |
| F-1296 | Me | F | CH₂CH₂CH₂N(Et)S(=O)₂CF₃ |
| F-1297 | Me | F | CH₂CH₂CH₂N(CH₂CN)S(=O)₂CF₃ |
| F-1298 | Me | F | CH₂CH₂CH₂N(CH₂OMe)S(=O)₂CF₃ |
| F-1299 | Me | F | CH₂CH₂CH₂CH₂N(Me)S(=O)₂CF₃ |
| F-1300 | Me | F | CH₂CH₂CH₂CH₂N(Et)S(=O)₂CF₃ |
| F-1301 | Me | F | CH₂CH₂CH₂CH₂N(CH₂CN)S(=O)₂CF₃ |
| F-1302 | Me | F | CH₂CH₂CH₂CH₂N(CH₂OMe)S(=O)₂CF₃ |
| F-1303 | Me | F | CH₂CH(Me)NHS(=O)₂CF₃ |
| F-1304 | Me | F | CH₂CH(Me)NHS(=O)₂CF₃: (R) |
| F-1305 | Me | F | CH₂CH(Me)NHS(=O)₂CF₃ : (S) |
| F-1306 | Me | F | CH(Me)CH₂NHS(=O)₂CF₃ |
| F-1307 | Me | F | CH₂CH₂CH(Me)NHS(=O)₂CF₃ |
| F-1308 | Me | F | CH₂CH(Me)CH₂NHS(=O)₂CF₃ |
| F-1309 | Me | F | CH(Me)CH₂CH₂NHS(=O)₂CF₃ |
| F-1310 | Me | F | CH₂CH(Me)N(Me)S(=O)₂CF₃ |
| F-1311 | Me | F | CH(Me)CH₂N(Me)S(=O)₂CF₃ |
| F-1312 | Me | F | CH₂CH₂CH(Me)N(Me)S(=O)₂CF₃ |
| F-1313 | Me | F | CH₂CH(Me)CH₂N(Me)S(=O)₂CF₃ |
| F-1314 | Me | F | CH(Me)CH₂CH₂N(Me)S(=O)₂CF₃ |

**[Table 1095]**

| Compound Number | R³ | R⁴ | R⁵ |
|---|---|---|---|
| F-1315 | Me | F | CH₂CH₂CH₂NHS(=O)₂Ph |
| F-1316 | Me | F | CH₂CH₂CH₂CH₂NHS(=O)₂Ph |
| F-1317 | Me | F | CH₂CH₂CH₂CH₂CH₂NHS(=O)₂Ph |
| F-1318 | Me | F | CH₂CH₂CH₂CH₂CH₂NHS(=O)₂Ph(4-CF₃) |
| F-1319 | Me | F | CH₂CH(Me)NHS(=O)₂Ph(4-CF₃) |
| F-1320 | Me | F | CH₂CH(Et)NHS(=O)₂Ph(4-CF₃) |
| F-1321 | Me | F | CH(Me)CH₂NHS(=O)₂Ph(4-CF₃) |
| F-1322 | Me | F | CH(Et)CH₂NHS(=O)₂Ph(4-CF₃) |
| F-1323 | Me | F | CH₂CH(Me)CH₂NHS(=O)₂Ph(4-CF₃) |
| F-1324 | Me | F | CH₂CH₂SiMe₃ |
| F-1325 | Me | F | CH₂CH₂CH₂SiMe₃ |
| F-1326 | Me | F | CH₂CH₂CH₂CH₂SiMe₃ |
| F-1327 | Me | F | CH₂Ph |
| F-1328 | Me | F | CH₂Ph(2-CF₃) |
| F-1329 | Me | F | CH₂Ph(3-CF₃) |
| F-1330 | Me | F | CH₂Ph(4-CF₃) |
| F-1331 | Me | F | CH₂Ph(2-OCF₃) |
| F-1332 | Me | F | CH₂Ph(3-OCF₃) |
| F-1333 | Me | F | CH₂Ph(4-OCF₃) |
| F-1334 | Me | F | CH₂Ph(2-SCF₃) |
| F-1335 | Me | F | CH₂Ph(3-SCF₃) |
| F-1336 | Me | F | CH₂Ph(4-SCF₃) |
| F-1337 | Me | F | CH₂Ph(3-CH₂SCF₃) |
| F-1338 | Me | F | CH₂Ph(4-F) |
| F-1339 | Me | F | CH₂Ph(4-Cl) |
| F-1340 | Me | F | CH₂Ph(4-Br) |
| F-1341 | Me | F | CH₂Ph(4-Me) |
| F-1342 | Me | F | CH₂Ph[4-(*t*-Bu)] |
| F-1343 | Me | F | CH₂Ph(4-CN) |
| F-1344 | Me | F | CH₂Ph(4-NO₂) |
| F-1345 | Me | F | CH₂Ph(4-OCHF₂) |
| F-1346 | Me | F | CH₂Ph(4-SCHF₂) |
| F-1347 | Me | F | CH₂Ph(4-CH₂SCF₃) |
| F-1348 | Me | F | CH₂Ph[4-CF(CF₃)₂] |
| F-1349 | Me | F | CH₂Ph(4-CH₂SCF₃) |
| F-1350 | Me | F | CH₂Ph[4-Ph(4-CF₃)] |
| F-1351 | Me | F | CH₂Ph(2,4-Cl₂) |
| F-1352 | Me | F | CH₂Ph[2,5-(CF₃)₂] |
| F-1353 | Me | F | CH₂Ph(3,4-Cl₂) |
| F-1354 | Me | F | CH₂Ph(3-CF₃-4-F) |
| F-1355 | Me | F | CH₂Ph(3-CF₃-4-Cl) |
| F-1356 | Me | F | CH₂Ph(3-F-4-CF₃) |
| F-1357 | Me | F | CH₂Ph(2,4,6-F₃) |
| F-1358 | Me | F | CH₂Ph(3,4,5-F₃) |

**[Table 1096]**

| Compound Number | R³ | R⁴ | R⁵ |
|---|---|---|---|
| F-1359 | Me | F | CH₂Ph(2,3,4-F₃) |
| F-1360 | Me | F | CH₂Ph(3,4,5-Cl₃) |
| F-1361 | Me | F | CH₂CH₂Ph |
| F-1362 | Me | F | CH₂CH₂Ph(4-F) |
| F-1363 | Me | F | CH₂CH₂Ph(4-Cl) |
| F-1364 | Me | F | CH₂CH₂Ph(4-Br) |
| F-1365 | Me | F | CH₂CH₂Ph[4-(*t*-Bu)] |
| F-1366 | Me | F | CH₂CH₂Ph(2-CF₃) |
| F-1367 | Me | F | CH₂CH₂Ph(3-CF₃) |
| F-1368 | Me | F | CH₂CH₂Ph(4-CF₃) |
| F-1369 | Me | F | CH₂CH₂Ph[4-CF(CF₃)₂] |
| F-1370 | Me | F | CH₂CH₂Ph[4-(*c*-Pr)] |
| F-1371 | Me | F | CH₂CH₂Ph{4-[*c*-Pr(2,2-F₂)]} |
| F-1372 | Me | F | CH₂CH₂Ph(4-OMe) |
| F-1373 | Me | F | CH₂CH₂Ph(3-OCF₃) |
| F-1374 | Me | F | CH₂CH₂Ph(4-OCHF₂) |
| F-1375 | Me | F | CH₂CH₂Ph(4-OCF₃) |
| F-1376 | Me | F | CH₂CH₂Ph(4-SMe) |
| F-1377 | Me | F | CH₂CH₂Ph(4-SCHF₂) |
| F-1378 | Me | F | CH₂CH₂Ph(4-SCF₃) |
| F-1379 | Me | F | CH₂CH₂Ph[4-S(=O)Me] |
| F-1380 | Me | F | CH₂CH₂Ph[4-S(=O)CF₃] |
| F-1381 | Me | F | CH₂CH₂Ph[4-S(=O)₂Me] |
| F-1382 | Me | F | CH₂CH₂Ph[4-S(=O)₂CF₃ |
| F-1383 | Me | F | CH₂CH₂Ph(4-CH₂SMe) |
| F-1384 | Me | F | CH₂CH₂Ph(4-CH₂SCF₃) |
| F-1385 | Me | F | CH₂CH₂Ph[4-OS(=O)₂Me] |
| F-1386 | Me | F | CH₂CH₂Ph[4-OS(=O)₂CF₃] |
| F-1387 | Me | F | CH₂CH₂Ph[4-Ph(4-CF₃)] |
| F-1388 | Me | F | CH₂CH₂Ph(4-CH₂Ph) |
| F-1389 | Me | F | CH₂CH₂Ph(4-OCH₂Ph) |
| F-1390 | Me | F | CH₂CH₂Ph(4-CN) |
| F-1391 | Me | F | CH₂CH₂Ph(4-NO₂) |
| F-1392 | Me | F | CH₂CH₂Ph(2,4-Cl₂) |
| F-1393 | Me | F | CH₂CH₂Ph(3,4-Cl₂) |
| F-1394 | Me | F | CH₂CH₂Ph(3-CF₃-4-F) |
| F-1395 | Me | F | CH₂CH₂Ph(2-CF₃-4-F) |
| F-1396 | Me | F | CH₂CH₂Ph(3-F-4-CF₃) |
| F-1397 | Me | F | CH₂CH₂Ph(2-F-4-CF₃) |
| F-1398 | Me | F | CH₂CH₂Ph(3-Cl-4-OCHF₂) |
| F-1399 | Me | F | CH₂CH₂Ph(3,4,5-Cl₃) |
| F-1400 | Me | F | CH₂CH₂Ph(2,3,4-F₃) |
| F-1401 | Me | F | CH₂CH₂Ph(2,4,5-F₃) |
| F-1402 | Me | F | CH₂CH₂Ph(3,4,5-F₃) |

**[Table 1097]**

| Compound Number | R³ | R⁴ | R⁵ |
|---|---|---|---|
| F-1403 | Me | F | CH₂CH₂Ph(2,4,6-F₃) |
| F-1404 | Me | F | CH₂CH₂CH₂Ph |
| F-1405 | Me | F | CH₂CH₂CH₂Ph(3-CF₃) |
| F-1406 | Me | F | CH₂CH₂CH₂Ph(4-CF₃) |
| F-1407 | Me | F | CH₂CH₂CH₂Ph(4-F) |
| F-1408 | Me | F | CH₂CH₂CH₂Ph[4-(t-Bu)] |
| F-1409 | Me | F | CH₂CH₂CH₂Ph(4-CN) |
| F-1410 | Me | F | CH₂CH₂CH₂Ph(4-CF₃) |
| F-1411 | Me | F | CH₂CH₂CH₂Ph(4-OCHF₂) |
| F-1412 | Me | F | CH₂CH₂CH₂Ph(4-OCF₃) |
| F-1413 | Me | F | CH₂CH₂CH₂Ph(4-SCHF₂) |
| F-1414 | Me | F | CH₂CH₂CH₂Ph(4-SCF₃) |
| F-1415 | Me | F | CH₂CH₂CH₂Ph[4-CF(CF₃)₂] |
| F-1416 | Me | F | CH₂CH₂CH₂Ph(3,4,5-F₃) |
| F-1417 | Me | F | CH₂CH₂CH₂Ph(2,4,6-F₃) |
| F-1418 | Me | F | CH₂CH₂CH₂CH₂Ph |
| F-1419 | Me | F | CH₂CH₂CH₂CH₂Ph(4-F) |
| F-1420 | Me | F | CH₂CH₂CH₂CH₂Ph(4-CF₃) |
| F-1421 | Me | F | CH₂CH₂CH₂CH₂Ph(4-OCF₃) |
| F-1422 | Me | F | CH₂CH₂CH₂CH₂Ph(4-SCF₃) |
| F-1423 | Me | F | CH₂CH₂CH₂CH₂CH₂Ph |
| F-1424 | Me | F | CH₂CH₂CH₂CH₂CH₂CH₂Ph |
| F-1425 | Me | F | CH₂CF₂Ph(4-F) |
| F-1426 | Me | F | CH₂CF₂Ph(4-CF₃) |
| F-1427 | Me | F | CH₂CF₂Ph(3,4,5-F₃) |
| F-1428 | Me | F | CH₂CH₂OPh |
| F-1429 | Me | F | CH₂CH₂OPh(4-F) |
| F-1430 | Me | F | CH₂CH₂OPh(4-CF₃) |
| F-1431 | Me | F | CH₂CH₂CH₂OPh |
| F-1432 | Me | F | CH₂CH₂CH₂OPh(4-Cl) |
| F-1433 | Me | F | CH₂CH₂CH₂OPh(4-CF₃) |
| F-1434 | Me | F | CH₂CH₂CH₂CH₂OPh(4-CF₃) |
| F-1435 | Me | F | CH₂CH₂CH₂CH₂OPh(4-OCF₃) |
| F-1436 | Me | F | CH₂CH₂CH₂CH₂CH₂OPh(4-CF₃) |
| F-1437 | Me | F | CH₂CH₂OCH₂Ph |
| F-1438 | Me | F | CH₂CH₂CH₂OCH₂Ph |
| F-1439 | Me | F | CH₂CH₂CH₂CH₂OCH₂Ph |
| F-1440 | Me | F | CH₂CH₂CH₂CH₂CH₂OCH₂Ph |
| F-1441 | Me | F | CH₂CH₂SPh |
| F-1442 | Me | F | CH₂CH₂CH₂SPh |
| F-1443 | Me | F | CH₂CH₂CH₂SPh(4-F) |
| F-1444 | Me | F | CH₂CH₂CH₂SPh(4-Cl) |
| F-1445 | Me | F | CH₂CH₂CH₂SPh(4-Br) |
| F-1446 | Me | F | CH₂CH₂CH₂SPh[4-(*t*-Bu)] |

**[Table 1098]**

| Compound Number | R³ | R⁴ | R⁵ |
|---|---|---|---|
| F-1447 | Me | F | CH₂CH₂CH₂SPh(3-CF₃) |
| F-1448 | Me | F | CH₂CH₂CH₂SPh(4-CF₃) |
| F-1449 | Me | F | CH₂CH₂CH₂SPh(3-SCF₃) |
| F-1450 | Me | F | CH₂CH₂CH₂SPh(4-SCF₃) |
| F-1451 | Me | F | CH₂CH₂CH₂CH₂SPh |
| F-1452 | Me | F | CH₂CH₂CH₂CH₂SPh(4-F) |
| F-1453 | Me | F | CH₂CH₂CH₂CH₂SPh(4-Cl) |
| F-1454 | Me | F | CH₂CH₂CH₂CH₂SPh[4-(*t*-Bu)] |
| F-1455 | Me | F | CH₂CH₂CH₂CH₂SPh(4-CF₃) |
| F-1456 | Me | F | CH₂CH₂CH₂CH₂CH₂SPh |
| F-1457 | Me | F | CH₂CH₂CH₂CH₂CH₂SPh(4-F) |
| F-1458 | Me | F | CH₂CH₂CH₂CH₂CH₂SPh(4-Cl) |
| F-1459 | Me | F | CH₂CH₂CH₂CH₂CH₂SPh(4-CF₃) |
| F-1460 | Me | F | CH₂CH₂S(=O)Ph |
| F-1461 | Me | F | CH₂CH₂CH₂S(=O)Ph |
| F-1462 | Me | F | CH₂CH₂CH₂S(=O)Ph(4-F) |
| F-1463 | Me | F | CH₂CH₂CH₂S(=O)Ph[4-(*t*-Bu)] |
| F-1464 | Me | F | CH₂CH₂CH₂S(=O)Ph(4-CF₃) |
| F-1465 | Me | F | CH₂CH₂S(=O)₂Ph |
| F-1466 | Me | F | CH₂CH₂S(=O)₂Ph(4-CF₃) |
| F-1467 | Me | F | CH₂CH₂CH₂S(=O)₂Ph(4-F) |
| F-1468 | Me | F | CH₂CH₂CH₂S(=O)₂Ph(4-Cl) |
| F-1469 | Me | F | C H₂CH₂CH₂S(=O)₂Ph(4-CF₃) |
| F-1470 | Me | F | CH₂CH₂CH₂CH₂S(=O)₂Ph |
| F-1471 | Me | F | CH₂CH₂CH₂CH₂S(=O)₂Ph(4-Cl) |
| F-1472 | Me | F | CH₂CH₂CH₂CH₂S(=O)₂Ph(4-CF₃) |
| F-1473 | Me | F | CH₂CH₂SCH₂Ph |
| F-1474 | Me | F | CH₂CH₂CH₂SCH₂Ph |
| F-1475 | Me | F | CH₂CH₂CH₂SCH₂Ph(2-Cl) |
| F-1476 | Me | F | CH₂CH₂CH₂SCH₂Ph(3-Cl) |
| F-1477 | Me | F | CH₂CH₂CH₂SCH₂Ph(4-Cl) |
| F-1478 | Me | F | CH₂CH₂CH₂SCH₂Ph(2-SCF₃) |
| F-1479 | Me | F | CH₂CH₂CH₂SCH₂Ph(3-CF₃) |
| F-1480 | Me | F | CH₂CH₂CH₂SCH₂Ph(4-CF₃) |
| F-1481 | Me | F | CH₂CH₂CH₂SCH₂Ph(4-NO₂) |
| F-1482 | Me | F | CH₂CH₂CH₂CH₂SCH₂Ph |
| F-1483 | Me | F | CH₂CH₂CH₂CH₂SCH₂Ph(4-Cl) |
| F-1484 | Me | F | CH₂CH₂CH₂CH₂SCH₂Ph(4-CF₃) |
| F-1485 | Me | F | CH₂CH₂CH₂CH₂SCH₂Ph(4-CN) |
| F-1486 | Me | F | CH₂CH₂CH₂CH₂CH₂SCH₂Ph |
| F-1487 | Me | F | CH₂CH₂SCH₂CH₂Ph |
| F-1488 | Me | F | CH₂CH₂CH₂SCH₂CH₂Ph |
| F-1489 | Me | F | CH₂CH₂CH₂CH₂SCH₂CH₂Ph |
| F-1490 | Me | F | CH₂CH₂SCH(Me)Ph |

**[Table 1099]**

| Compound Number | R³ | R⁴ | R⁵ |
|---|---|---|---|
| F-1491 | Me | F | CH₂CH₂CH₂SCH(Me)Ph |
| F-1492 | Me | F | CH₂CH₂CH₂CH₂SCH(Me)Ph |
| F-1493 | Me | F | CH₂CH₂S(=O)CH₂Ph |
| F-1494 | Me | F | CH₂CH₂S(=O)CH₂Ph(4-CF₃) |
| F-1495 | Me | F | CH₂CH₂CH₂S(=O)CH₂Ph |
| F-1496 | Me | F | CH₂CH₂CH₂S(=O)CH₂Ph(2-SCF₃) |
| F-1497 | Me | F | CH₂CH₂CH₂S(=O)CH₂Ph(4-SCF₃) |
| F-1498 | Me | F | CH₂CH₂CH₂S(=O)CH₂Ph(4-Cl) |
| F-1499 | Me | F | CH₂CH₂CH₂S(=O)CH₂Ph(4-CF₃) |
| F-1500 | Me | F | CH₂CH₂CH₂CH₂S(=O)CH₂Ph |
| F-1501 | Me | F | CH₂CH₂CH₂CH₂S(=O)CH₂Ph(4-CF₃) |
| F-1502 | Me | F | CH₂CH₂CH₂CH₂CH₂S(=O)CH₂Ph(4-CF₃) |
| F-1503 | Me | F | CH₂CH₂S(=O)₂CH₂Ph |
| F-1504 | Me | F | CH₂CH₂S(=O)₂CH₂Ph(4-CF₃) |
| F-1505 | Me | F | CH₂CH₂CH₂S(=O)₂CH₂Ph |
| F-1506 | Me | F | CH₂CH₂CH₂S(=O)₂CH₂Ph(4-Cl) |
| F-1507 | Me | F | CH₂CH₂CH₂S(=O)₂CH₂Ph(4-CF₃) |
| F-1508 | Me | F | CH₂CH₂CH₂CH₂S(=O)₂CH₂Ph |
| F-1509 | Me | F | CH₂CH₂CH₂CH₂S(=O)₂CH₂Ph(4-CF₃) |
| F-1510 | Me | F | CH₂CH₂CH₂CH₂CH₂S(=O)₂CH₂Ph |
| F-1511 | Me | F | CH₂CH₂CH₂CH₂CH₂S(=O)₂CH₂Ph(4-CF₃) |
| F-1512 | Me | F | CH₂CH₂₀N=CH(*t*-Bu) |
| F-1513 | Me | F | CH₂CH₂CH₂₀N=CH(*t*-Bu) |
| F-1514 | Me | F | CH₂CH₂CH₂CH₂₀N=C(Me)(*c*-Pr) |
| F-1515 | Me | F | CH₂CH₂₀N=C(Me)CF₃ |
| F-1516 | Me | F | CH₂CH₂CH₂₀N=CHCF₃ |
| F-1517 | Me | F | CH₂CH₂CH₂₀N=C(Me)CF₃ |
| F-1518 | Me | F | CH₂CH₂CH₂₀N=C(Me)CCl₃ |
| F-1519 | Me | F | CH₂CH₂CH₂CH₂ON=CHCF₃ |
| F-1520 | Me | F | CH₂CH₂CH₂CH₂ON=C(Me)CF₃ |
| F-1521 | Me | F | CH₂CH₂CH₂CH₂CH₂ON=C(Me)CF₃ |
| F-1522 | Me | F | CH₂CH₂ON=CHPh |
| F-1523 | Me | F | CH₂CH₂ON=CHPh(4-CF₃) |
| F-1524 | Me | F | CH₂CH₂ON=CHPh(4-SCF₃) |
| F-1525 | Me | F | CH₂CH₂CH₂ON=CHPh(3-CF₃) |
| F-1526 | Me | F | CH₂CH₂CH₂ON=CHPh(4-CF₃) |
| F-1527 | Me | F | CH₂CH₂CH₂ON=CHPh(4-SCF₃) |
| F-1528 | Me | F | CH₂CH₂CH₂ON=C(Me)Ph(4-CF₃) |
| F-1529 | Me | F | CH₂CH₂CH₂CH₂ON=CHPh(4-SCF₃) |
| F-1530 | Me | F | CH₂CH₂(adamant-1-yl) |
| F-1531 | Me | F | CH₂CH₂CH₂(3-CF₃-pyrazol-1-yl) |
| F-1532 | Me | F | CH₂CH₂CH₂CH₂(3-CF₃-pyrazol-1-yl) |
| F-1533 | Me | F | CH₂CH₂CH₂CH₂CH₂(3-CF₃-pyrazol-1-yl) |
| F-1534 | Me | F | CH₂(1-Me-4-CF₃-imidazol-5-yl) |

**[Table 1100]**

| Compound Number | R³ | R⁴ | R⁵ |
|---|---|---|---|
| F-1535 | Me | F | CH₂CH₂(1-Me-4-CF₃-imidazol-5-yl) |
| F-1536 | Me | F | CH₂CH₂CH₂(1-Me-4-CF₃-imidazol-5-yl) |
| F-1537 | Me | F | CH₂CH₂CH₂(3-CF₃-1,2,4-triazol-1-yl) |
| F-1538 | Me | F | CH₂CH₂CH₂CH₂(3-CF₃-1,2,4-triazol-1-yl) |
| F-1539 | Me | F | CH₂CH₂CH₂CH₂CH₂(3-CF₃-1,2,4-triazol-1-yl) |
| F-1540 | Me | F | CH₂CH₂CH₂(4-CF₃-thiazol-2-yl) |
| F-1541 | Me | F | CH₂CH₂CH₂CH₂(4-CF₃-thiazol-2-yl) |
| F-1542 | Me | F | CH₂CH₂CH₂CH₂CH₂(4-CF₃-thiazol-2-yl) |
| F-1543 | Me | F | CH₂CH₂CH₂(2-CF₃-pyridin-5-yl) |
| F-1544 | Me | F | CH₂CH₂CH₂CH₂(2-CF₃-pyridin-5-yl) |
| F-1545 | Me | F | CH₂CH₂CH₂CH₂CH₂(2-CF₃-pyridin-5-yl) |
| F-1546 | Me | F | CH₂(4-CF₃-pyrimidin-2-yl) |
| F-1547 | Me | F | CH₂CH₂(4-CF₃-pyrimidin-2-yl) |
| F-1548 | Me | F | CH₂CH₂CH₂(4-CF₃-pyrimidin-2-yl) |
| F-1549 | Me | F | CH₂CH₂O(3-Cl-5-CF₃-pyridin-2-yl) |
| F-1550 | Me | F | CH₂CH₂CH₂O(3-Cl-5-CF₃-pyridin-2-yl) |
| F-1551 | Me | F | CH₂CH₂CH₂CH₂O(3-Cl-5-CF₃-pyridin-2-yl) |
| F-1552 | Me | F | CH₂CH₂CH₂CH₂CH₂O(3-Cl-5-CF₃-pyridin-2-yl) |
| F-1553 | Me | F | CH₂CH₂N(Phth) |
| F-1554 | Me | F | CH₂CH₂CH₂N(Phth) |
| F-1555 | Me | F | CH₂CH₂CH₂CH₂N(Phth) |
| F-1556 | Me | F | CH₂CH₂CH₂CH₂CH₂N(Phth) |
| F-1557 | Me | F | CH₂CH(Me)CH₂N(Phth) |
| F-1558 | Me | F | CH₂CH₂CH₂CN |
| F-1559 | Me | F | CH₂CH₂CH₂CH₂CN |
| F-1560 | Me | F | CH₂CH₂CH₂CH₂CH₂CN |
| F-1561 | Me | F | CH₂CH₂CH₂CH₂CH₂CH₂CN |
| F-1562 | Me | F | CH₂CH₂CH₂C(=O)OH |
| F-1563 | Me | F | CH₂CH₂CH₂CH₂C(=O)OH |
| F-1564 | Me | F | CH₂CH₂CH₂CH₂CH₂C(=O)OH |
| F-1565 | Me | F | CH₂CH₂SCN |
| F-1566 | Me | F | CH₂CH₂CH₂SCN |
| F-1567 | Me | F | CH₂CH₂CH₂CH₂SCN |
| F-1568 | Me | F | CH₂CH₂CH₂CH₂CH₂SCN |
| F-1569 | Me | F | CH₂CH₂CH₂CH₂CH₂CH₂SCN |
| F-1570 | Me | F | CH₂CH₂CH₂CH₂CH₂CH₂CH₂SCN |
| F-1571 | Me | F | CH₂CH₂CH₂CH₂CH₂CH₂CH₂CH₂SCN |
| F-1572 | Me | F | CH₂CH₂CH(Me)SCN |
| F-1573 | Me | F | CH₂CH(Me)CH₂SCN |
| F-1574 | Me | F | CH(Me)CH₂CH₂SCN |
| F-1575 | Me | F | CH₂CH₂CH₂CH(Me)SCN |
| F-1576 | Me | F | CH₂CH₂CH(Me)CH₂SCN |
| F-1577 | Me | F | CH₂CH(Me)CH₂CH₂SCN |
| F-1578 | Me | F | CH(Me)CH₂CH₂CH₂SCN |

**[Table 1101]**

| Compound Number | R³ | R⁴ | R⁵ |
|---|---|---|---|
| F-1579 | Me | F | CH₂CH₂CH₂CH₂CH(Me)SCN |
| F-1580 | Me | F | CH₂CH₂CH₂CH(Me)CH₂SCN |
| F-1581 | Me | F | CH₂CH₂CH(Me)CH₂CH₂SCN |
| F-1582 | Me | F | CH₂CH(Me)CH₂CH₂CH₂SCN |
| F-1583 | Me | F | CH(Me)CH₂CH₂CH₂CH₂SCN |
| F-1584 | Me | F | CH₂CH₂ONH₂ |
| F-1585 | Me | F | CH₂CH₂CH₂ONH₂ |
| F-1586 | Me | F | CH₂CH₂CH₂CH₂ONH₂ |
| F-1587 | Me | F | CH₂CH₂CH₂CH₂CH₂ONH₂ |
| F-1588 | Me | F | CH₂CH₂CH₂CH₂CH₂CH₂ONH₂ |
| F-1589 | Cl | H | CH₂C(=O)Me |
| F-1590 | Cl | H | CH₂CH(OH)Me |
| F-1591 | Cl | H | CH₂CH(OH)CF₃ |
| F-1592 | Cl | H | CH₂C(OH)₂CF₃ |
| F-1593 | Cl | H | CH₂C(CF₃)=NOH |
| F-1594 | Cl | H | CH₂C(CF₃)=NOMe |
| F-1595 | Cl | H | CH₂CH(CF₃)NH₂ |
| F-1596 | Cl | H | CH₂CH(CF₃)NHSO₂CF₃ |
| F-1597 | Cl | H | CH₂{*c*-Pr[1-NHS(=O)₂CF₃]} |
| F-1598 | Cl | H | CH₂CH(Me)NH(*c*-Pᵣ) |
| F-1599 | Cl | H | CH₂CH(Me)N(*c*-pr)S(=O)₂CF₃ |
| F-1600 | Cl | H | CH₂CH(Me)NHOMe |
| F-1601 | Cl | H | CH₂CH(Me)N(OMe)S(=O)₂CF₃ |
| F-1602 | Cl | H | CH₂CH(Me)N(OMe)S(=O)₂Ph(2-NO₂) |
| F-1603 | Cl | H | CH₂CH(Me)N(OMe)S(=O)₂Ph(4-NO₂) |
| F-1604 | Cl | H | CH₂CH₂SCHF₂ |
| F-1605 | Cl | H | CH₂CH₂CH₂SCHF₂ |
| F-1606 | Cl | H | CH₂CH₂CH₂CH₂SCHF₂ |
| F-1607 | Cl | H | CH₂CH₂CH₂CH₂CH₂SCHF₂ |
| F-1608 | Cl | H | CH₂CH₂CH₂CH₂CH₂CH₂SCHF₂ |
| F-1609 | Cl | H | CH₂CH₂CH₂CH₂CH₂CH₂CH₂SCHF₂ |
| F-1610 | Cl | H | CH₂CH₂SCF₃ |
| F-1611 | Cl | H | CH₂CH₂CH₂SCF₃ |
| F-1612 | Cl | H | CH₂CH₂CH₂CH₂SCF₃ |
| F-1613 | Cl | H | CH₂CH₂CH₂CH₂CH₂SCF₃ |
| F-1614 | Cl | H | CH₂CH₂CH₂CH₂CH₂CH₂SCF₃ |
| F-1615 | Cl | H | CH₂CH₂CH₂CH₂CH₂CH₂CH₂SCF₃ |
| F-1616 | Cl | H | CH₂CH₂CH₂CH₂CH₂CH₂CH₂CH₂SCF₃ |
| F-1617 | Cl | H | CH₂CH(Me)SCF₃ |
| F-1618 | Cl | H | CH(Me)CH₂SCF₃ |
| F-1619 | Cl | H | CH₂CH₂CH(Me)SCF₃ |
| F-1620 | Cl | H | CH₂CH(Me)CH₂SCF₃ |
| F-1621 | Cl | H | CH(Me)CH₂CH₂SCF₃ |
| F-1622 | Cl | H | CH₂CH₂CH₂CH(Me)SCF₃ |

**[Table 1102]**

| Compound Number | R³ | R⁴ | R⁵ |
|---|---|---|---|
| F-1623 | Cl | H | CH₂CH₂CH(Me)CH₂SCF₃ |
| F-1624 | Cl | H | CH₂CH(Me)CH₂CH₂SCF₃ |
| F-1625 | Cl | H | CH(Me)CH₂CH₂CH₂SCF₃ |
| F-1626 | Cl | H | CH₂CH₂NHS(=O)₂CF₃ |
| F-1627 | Cl | H | CH₂CH₂CH₂NHS(=O)₂CF₃ |
| F-1628 | Cl | H | CH₂CH₂CH₂CH₂NHS(=O)₂CF₃ |
| F-1629 | Cl | H | CH₂CH₂CH₂CH₂CH₂NHS(=O)₂CF₃ |
| F-1630 | Cl | H | CH₂CH₂N(Me)S(=O)₂CF₃ |
| F-1631 | Cl | H | CH₂CH₂N(Et)S(=O)₂CF₃ |
| F-1632 | Cl | H | CH₂CH₂N(CH₂CN)S(=O)₂CF₃ |
| F-1633 | Cl | H | CH₂CH₂N(CH₂OMe)S(=O)₂CF₃ |
| F-1634 | Cl | H | CH₂CH₂CH₂N(Me)S(=O)₂CF₃ |
| F-1635 | Cl | H | CH₂CH₂CH₂N(Et)S(=O)₂CF₃ |
| F-1636 | Cl | H | CH₂CH₂CH₂N(CH₂CN)S(=O)₂CF₃ |
| F-1637 | Cl | H | CH₂CH₂CH₂N(CH₂OMe)S(=O)₂CF₃ |
| F-1638 | Cl | H | CH₂CH₂CH₂CH₂N(Me)S(=O)₂CF₃ |
| F-1639 | Cl | H | CH₂CH₂CH₂CH₂N(Et)S(=O)₂CF₃ |
| F-1640 | Cl | H | CH₂CH₂CH₂CH₂N(CH₂CN)S(=O)₂CF₃ |
| F-1641 | Cl | H | CH₂CH₂CH₂CH₂N(CH₂OMe)S(=O)₂CF₃ |
| F-1642 | Cl | H | CH₂CH(Me)NHS(=O)₂CF₃ |
| F-1643 | Cl | H | CH₂C*H(Me)NHS(=O)₂CF₃ : (R) |
| F-1644 | Cl | H | CH₂C*H(Me)NHS(=O)₂CF₃ : (S) |
| F-1645 | Cl | H | CH(Me)CH₂NHS(=O)₂CF₃ |
| F-1646 | Cl | H | CH₂CH₂CH(Me)NHS(=O)₂CF₃ |
| F-1647 | Cl | H | CH₂CH(Me)CH₂NHS(=O)₂CF₃ |
| F-1648 | Cl | H | CH(Me)CH₂CH₂NHS(=O)₂CF₃ |
| F-1649 | Cl | H | CH₂CH(Me)N(Me)S(=O)₂CF₃ |
| F-1650 | Cl | H | CH(Me)CH₂N(Me)S(=O)₂CF₃ |
| F-1651 | Cl | H | CH₂CH₂CH(Me)N(Me)S(=O)₂CF₃ |
| F-1652 | Cl | H | CH₂CH(Me)CH₂N(Me)S(=O)₂CF₃ |
| F-1653 | Cl | H | CH(Me)CH₂CH₂N(Me)S(=O)₂CF₃ |
| F-1654 | Cl | H | CH₂Ph |
| F-1655 | Cl | H | CH₂Ph(2-CF₃) |
| F-1656 | Cl | H | CH₂Ph(3-CF₃) |
| F-1657 | Cl | H | CH₂Ph(4-CF₃) |
| F-1658 | Cl | H | CH₂Ph(2-OCF₃) |
| F-1659 | Cl | H | CH₂Ph(3-OCF₃) |
| F-1660 | Cl | H | CH₂Ph(4-OCF₃) |
| F-1661 | Cl | H | CH₂Ph(2-SCF₃) |
| F-1662 | Cl | H | CH₂Ph(3-SCF₃) |
| F-1663 | Cl | H | CH₂Ph(4-SCF₃) |
| F-1664 | Cl | H | CH₂Ph(3-CH₂SCF₃) |
| F-1665 | Cl | H | CH₂Ph(4-F) |
| F-1666 | Cl | H | CH₂Ph(4-Cl) |

**[Table 1103]**

| Compound Number | R³ | R⁴ | R⁵ |
|---|---|---|---|
| F-1667 | Cl | H | CH₂Ph(4-Br) |
| F-1668 | Cl | H | CH₂Ph(4-Me) |
| F-1669 | Cl | H | CH₂Ph[4-(*t*-Bu)] |
| F-1670 | Cl | H | CH₂Ph(4-CN) |
| F-1671 | Cl | H | CH₂Ph(4-NO₂) |
| F-1672 | Cl | H | CH₂Ph(4-OCHF₂) |
| F-1673 | Cl | H | CH₂Ph(4-SCHF₂) |
| F-1674 | Cl | H | CH₂Ph(4-CH₂SCF₃) |
| F-1675 | Cl | H | CH₂Ph[4-CF(CF₃)₂] |
| F-1676 | Cl | H | CH₂Ph(4-CH₂SCF₃) |
| F-1677 | Cl | H | CH₂Ph[4-Ph(4-CF₃)] |
| F-1678 | Cl | H | CH₂Ph(2,4-Cl₂) |
| F-1679 | Cl | H | CH₂Ph[2,5-(CF₃)₂] |
| F-1680 | Cl | H | CH₂Ph(3,4-Cl₂) |
| F-1681 | Cl | H | CH₂Ph(3-CF₃-4-F) |
| F-1682 | Cl | H | CH₂Ph(3-CF₃-4-Cl) |
| F-1683 | Cl | H | CH₂Ph(3-F-4-CF₃) |
| F-1684 | Cl | H | CH₂Ph(2,4,6-F₃) |
| F-1685 | Cl | H | CH₂Ph(3,4,5-F₃) |
| F-1686 | Cl | H | CH₂Ph(2,3,4-F₃) |
| F-1687 | Cl | H | CH₂Ph(3,4,5-Cl₃) |
| F-1688 | Cl | H | CH₂CH₂Ph |
| F-1689 | Cl | H | CH₂CH₂Ph(4-F) |
| F-1690 | Cl | H | CH₂CH₂Ph(4-Cl) |
| F-1691 | Cl | H | CH₂CH₂Ph(4-Br) |
| F-1692 | Cl | H | CH₂CH₂Ph[4-(*t*-Bu)] |
| F-1693 | Cl | H | CH₂CH₂Ph(2-CF₃) |
| F-1694 | Cl | H | CH₂CH₂Ph(3-CF₃) |
| F-1695 | Cl | H | CH₂CH₂Ph(4-CF₃) |
| F-1696 | Cl | H | CH₂CH₂Ph[4-CF(CF₃)₂] |
| F-1697 | Cl | H | CH₂CH₂Ph[4-(*c*-Pr)] |
| F-1698 | Cl | H | CH₂CH₂Ph{4-[*c*-Pr(2,2-F₂)]} |
| F-1699 | Cl | H | CH₂CH₂Ph(4-OMe) |
| F-1700 | Cl | H | CH₂CH₂Ph(3-OCF₃) |
| F-1701 | Cl | H | CH₂CH₂Ph(4-OCHF₂) |
| F-1702 | Cl | H | CH₂CH₂Ph(4-OCF₃) |
| F-1703 | Cl | H | CH₂CH₂Ph(4-SMe) |
| F-1704 | Cl | H | CH₂CH₂Ph(4-SCHF₂) |
| F-1705 | Cl | H | CH₂CH₂Ph(4-SCF₃) |
| F-1706 | Cl | H | CH₂CH₂Ph[4-S(=O)Me] |
| F-1707 | Cl | H | CH₂CH₂Ph[4-S(=O)CF₃] |
| F-1708 | Cl | H | CH₂CH₂Ph[4-S(=O)₂Me] |
| F-1709 | Cl | H | CH₂CH₂Ph[4-S(=O)₂CF₃] |
| F-1710 | Cl | H | CH₂CH₂Ph(4-CH₂SMe) |

**[Table 1104]**

| Compound Number | R³ | R⁴ | R⁵ |
|---|---|---|---|
| F-1711 | Cl | H | CH₂CH₂Ph(4-CH₂SCF₃) |
| F-1712 | Cl | H | CH₂CH₂Ph[4-OS(=O)₂Me] |
| F-1713 | Cl | H | CH₂CH₂Ph[4-OS(=O)₂CF₃] |
| F-1714 | Cl | H | CH₂CH₂Ph[4-Ph(4-CF₃)] |
| F-1715 | Cl | H | CH₂CH₂Ph(4-CH₂Ph) |
| F-1716 | Cl | H | CH₂CH₂Ph(4-OCH₂Ph) |
| F-1717 | Cl | H | CH₂CH₂Ph(4-CN) |
| F-1718 | Cl | H | CH₂CH₂Ph(4-NO₂) |
| F-1719 | Cl | H | CH₂CH₂Ph(2,4-Cl₂) |
| F-1720 | Cl | H | CH₂CH₂Ph(3,4-Cl₂) |
| F-1721 | Cl | H | CH₂CH₂Ph(3-CF₃-4-F) |
| F-1722 | Cl | H | CH₂CH₂Ph(2-CF₃-4-F) |
| F-1723 | Cl | H | CH₂CH₂Ph(3-F-4-CF₃) |
| F-1724 | Cl | H | CH₂CH₂Ph(2-F-4-CF₃) |
| F-1725 | Cl | H | CH₂CH₂Ph(3-Cl-4-OCHF₂) |
| F-1726 | Cl | H | CH₂CH₂Ph(3,4,5-Cl₃) |
| F-1727 | Cl | H | CH₂CH₂Ph(2,3,4-F₃) |
| F-1728 | Cl | H | CH₂CH₂Ph(2,4,5-F₃) |
| F-1729 | Cl | H | CH₂CH₂Ph(3,4,5-F₃) |
| F-1730 | Cl | H | CH₂CH₂Ph(2,4,6-F₃) |
| F-1731 | Cl | H | CH₂CH₂CH₂Ph |
| F-1732 | Cl | H | CH₂CH₂CH₂Ph(3-CF₃) |
| F-1733 | Cl | H | CH₂CH₂CH₂Ph(4-CF₃) |
| F-1734 | Cl | H | CH₂CH₂CH₂Ph(4-F) |
| F-1735 | Cl | H | CH₂CH₂CH₂Ph[4-(*t*-Bu)] |
| F-1736 | Cl | H | CH₂CH₂CH₂Ph(4-CN) |
| F-1737 | Cl | H | CH₂CH₂CH₂Ph(4-CF₃) |
| F-1738 | Cl | H | CH₂CH₂CH₂Ph(4-OCHF₂) |
| F-1739 | Cl | H | CH₂CH₂CH₂Ph(4-OCF₃) |
| F-1740 | Cl | H | CH₂CH₂CH₂Ph(4-SCHF₂) |
| F-1741 | Cl | H | CH₂CH₂CH₂Ph(4-SCF₃) |
| F-1742 | Cl | H | CH₂CH₂CH₂Ph[4-CF(CF₃)₂] |
| F-1743 | Cl | H | CH₂CH₂CH₂Ph(3,4,5-F₃) |
| F-1744 | Cl | H | CH₂CH₂CH₂Ph(2,4,6-F₃) |
| F-1745 | Cl | H | CH₂CH₂CH₂CH₂Ph |
| F-1746 | Cl | H | CH₂CH₂CH₂CH₂Ph(4-F) |
| F-1747 | Cl | H | CH₂CH₂CH₂CH₂Ph(4-CF₃) |
| F-1748 | Cl | H | CH₂CH₂CH₂CH₂Ph(4-OCF₃) |
| F-1749 | Cl | H | CH₂CH₂CH₂CH₂Ph(4-SCF₃) |
| F-1750 | Cl | H | CH₂CH₂CH₂CH₂CH₂Ph |
| F-1751 | Cl | H | CH₂CH₂CH₂CH₂CH₂CH₂Ph |
| F-1752 | Cl | H | CH₂CF₂Ph(4-F) |
| F-1753 | Cl | H | CH₂CF₂Ph(4-CF₃) |
| F-1754 | Cl | H | CH₂CF₂Ph(3,4,5-F₃) |

**[Table 1105]**

| Compound Number | R³ | R⁴ | R⁵ |
|---|---|---|---|
| F-1755 | Me | H | CH₂C(=O)Me |
| F-1756 | Me | H | CH₂CH(OH)Me |
| F-1757 | Me | H | CH₂CH(OH)CF₃ |
| F-1758 | Me | H | CH₂C(OH)₂CF₃ |
| F-1759 | Me | H | CH₂C(CF₃)=NOH |
| F-1760 | Me | H | CH₂C(CF₃)=NOMe |
| F-1761 | Me | H | CH₂CH(CF₃)NH₂ |
| F-1762 | Me | H | CH₂CH(CF₃)NHSO₂CF₃ |
| F-1763 | Me | H | CH₂{*c*-Pr[1-NHS(=O)₂CF₃]} |
| F-1764 | Me | H | CH₂CH(Me)NH(*c*-Pr) |
| F-1765 | Me | H | CH₂CH(Me)N(*c*-Pr)S(=O)₂CF₃ |
| F-1766 | Me | H | CH₂CH(Me)NHOMe |
| F-1767 | Me | H | CH₂CH(Me)N(OMe)S(=O)₂CF₃ |
| F-1768 | Me | H | CH₂CH(Me)N(OMe)S(=O)₂Ph(2-NO₂) |
| F-1769 | Me | H | CH₂CH(Me)N(OMe)S(=O)₂Ph(4-NO₂) |
| F-1770 | Me | H | CH₂CH₂SCHF₂ |
| F-1771 | Me | H | CH₂CH₂CH₂SCHF₂ |
| F-1772 | Me | H | CH₂CH₂CH₂CH₂SCHF₂ |
| F-1773 | Me | H | CH₂CH₂CH₂CH₂CH₂SCHF₂ |
| F-1774 | Me | H | CH₂CH₂CH₂CH₂CH₂CH₂SCHF₂ |
| F-1775 | Me | H | CH₂CH₂CH₂CH₂CH₂CH₂CH₂SCHF₂ |
| F-1776 | Me | H | CH₂CH₂SCF₃ |
| F-1777 | Me | H | CH₂CH₂CH₂SCF₃ |
| F-1778 | Me | H | CH₂CH₂CH₂CH₂SCF₃ |
| F-1779 | Me | H | CH₂CH₂CH₂CH₂CH₂SCF₃ |
| F-1780 | Me | H | CH₂CH₂CH₂CH₂CH₂CH₂SCF₃ |
| F-1781 | Me | H | CH₂CH₂CH₂CH₂CH₂CH₂CH₂SCF₃ |
| F-1782 | Me | H | CH₂CH₂CH₂CH₂CH₂CH₂CH₂CH₂SCF₃ |
| F-1783 | Me | H | CH₂CH(Me)SCF₃ |
| F-1784 | Me | H | CH(Me)CH₂SCF₃ |
| F-1785 | Me | H | CH₂CH₂CH(Me)SCF₃ |
| F-1786 | Me | H | CH₂CH(Me)CH₂SCF₃ |
| F-1787 | Me | H | CH(Me)CH₂CH₂SCF₃ |
| F-1788 | Me | H | CH₂CH₂CH₂CH(Me)SCF₃ |
| F-1789 | Me | H | CH₂CH₂CH(Me)CH₂SCF₃ |
| F-1790 | Me | H | CH₂CH(Me)CH₂CH₂SCF₃ |
| F-1791 | Me | H | CH(Me)CH₂CH₂CH₂SCF₃ |
| F-1792 | Me | H | CH₂CH₂NHS(=O)₂CF₃ |
| F-1793 | Me | H | CH₂CH₂CH₂NHS(=O)₂CF₃ |
| F-1794 | Me | H | CH₂CH₂CH₂CH₂NHS(=O)₂CF₃ |
| F-1795 | Me | H | CH₂CH₂CH₂CH₂CH₂NHS(=O)₂CF₃ |
| F-1796 | Me | H | CH₂CH₂N(Me)S(=O)₂CF₃ |
| F-1797 | Me | H | CH₂CH₂N(Et)S(=O)₂CF₃ |
| F-1798 | Me | H | CH₂CH₂N(CH₂CN)S(=O)₂CF₃ |

**[Table 1106]**

| Compound Number | R³ | R⁴ | R⁵ |
|---|---|---|---|
| F-1799 | Me | H | CH₂CH₂N(CH₂OMe)S(=O)₂CF₃ |
| F-1800 | Me | H | CH₂CH₂CH₂N(Me)S(=O)₂CF₃ |
| F-1801 | Me | H | CH₂CH₂CH₂N(Et)S(=O)₂CF₃ |
| F-1802 | Me | H | CH₂CH₂CH₂N(CH₂CN)S(=O)₂CF₃ |
| F-1803 | Me | H | CH₂CH₂CH₂N(CH₂OMe)S(=O)₂CF₃ |
| F-1804 | Me | H | CH₂CH₂CH₂CH₂N(Me)S(=O)₂CF₃ |
| F-1805 | Me | H | CH₂CH₂CH₂CH₂N(Et)S(=O)₂CF₃ |
| F-1806 | Me | H | CH₂CH₂CH₂CH₂N(CH₂CN)S(=O)₂CF₃ |
| F-1807 | Me | H | CH₂CH₂CH₂CH₂N(CH₂OMe)S(=O)₂CF₃ |
| F-1808 | Me | H | CH₂CH(Me)NHS(=O)₂CF₃ |
| F-1809 | Me | H | CH₂C*H(Me)NHS(=O)₂CF₃ : (R) |
| F-1810 | Me | H | CH₂C*H(Me)NHS(=O)₂CF₃ : (S) |
| F-1811 | Me | H | CH(Me)CH₂NHS(=O)₂CF₃ |
| F-1812 | Me | H | CH₂CH₂CH(Me)NHS(=O)₂CF₃ |
| F-1813 | Me | H | CH₂CH(Me)CH₂NHS(=O)₂CF₃ |
| F-1814 | Me | H | CH(Me)CH₂CH₂NHS(=O)₂CF₃ |
| F-1815 | Me | H | CH₂CH(Me)N(Me)S(=O)₂CF₃ |
| F-1816 | Me | H | CH(Me)CH₂N(Me)S(=O)₂CF₃ |
| F-1817 | Me | H | CH₂CH₂CH(Me)N(Me)S(=O)₂CF₃ |
| F-1818 | Me | H | CH₂CH(Me)CH₂N(Me)S(=O)₂CF₃ |
| F-1819 | Me | H | CH(Me)CH₂CH₂N(Me)S(=O)₂CF₃ |
| F-1820 | Me | H | CH₂Ph |
| F-1821 | Me | H | CH₂Ph(2-CF₃) |
| F-1822 | Me | H | CH₂Ph(3-CF₃) |
| F-1823 | Me | H | CH₂Ph(4-CF₃) |
| F-1824 | Me | H | CH₂Ph(2-OCF₃) |
| F-1825 | Me | H | CH₂Ph(3-OCF₃) |
| F-1826 | Me | H | CH₂Ph(4-OCF₃) |
| F-1827 | Me | H | CH₂Ph(2-SCF₃) |
| F-1828 | Me | H | CH₂Ph(3-SCF₃) |
| F-1829 | Me | H | CH₂Ph(4-SCF₃) |
| F-1830 | Me | H | CH₂Ph(3-CH₂SCF₃) |
| F-1831 | Me | H | CH₂Ph(4-F) |
| F-1832 | Me | H | CH₂Ph(4-Cl) |
| F-1833 | Me | H | CH₂Ph(4-Br) |
| F-1834 | Me | H | CH₂Ph(4-Me) |
| F-1835 | Me | H | CH₂Ph[4-(*t*-Bu)] |
| F-1836 | Me | H | CH₂Ph(4-CN) |
| F-1837 | Me | H | CH₂Ph(4-NO₂) |
| F-1838 | Me | H | CH₂Ph(4-OCHF₂) |
| F-1839 | Me | H | CH₂Ph(4-SCHF₂) |
| F-1840 | Me | H | CH₂Ph(4-CH₂SCF₃) |
| F-1841 | Me | H | CH₂Ph[4-CF(CF₃)₂] |
| F-1842 | Me | H | CH₂Ph(4-CH₂SCF₃) |

**[Table 1107]**

| Compound Number | R³ | R⁴ | R⁵ |
|---|---|---|---|
| F-1843 | Me | H | CH₂Ph[4-Ph(4-CF₃)] |
| F-1844 | Me | H | CH₂Ph(2,4-Cl₂) |
| F-1845 | Me | H | CH₂Ph[2,5-(CF₃)₂] |
| F-1846 | Me | H | CH₂Ph(3,4-Cl₂) |
| F-1847 | Me | H | CH₂Ph(3-CF₃-4-F) |
| F-1848 | Me | H | CH₂Ph(3-CF₃-4-Cl) |
| F-1849 | Me | H | CH₂Ph(3-F-4-CF₃) |
| F-1850 | Me | H | CH₂Ph(2,4,6-F₃) |
| F-1851 | Me | H | CH₂Ph(3,4,5-F₃) |
| F-1852 | Me | H | CH₂Ph(2,3,4-F₃) |
| F-1853 | Me | H | CH₂Ph(3,4,5-Cl₃) |
| F-1854 | Me | H | CH₂CH₂Ph |
| F-1855 | Me | H | CH₂CH₂Ph(4-F) |
| F-1856 | Me | H | CH₂CH₂Ph(4-Cl) |
| F-1857 | Me | H | CH₂CH₂Ph(4-Br) |
| F-1858 | Me | H | CH₂CH₂Ph[4-(*t*-Bu)] |
| F-1859 | Me | H | CH₂CH₂Ph(2-CF₃) |
| F-1860 | Me | H | CH₂CH₂Ph(3-CF₃) |
| F-1861 | Me | H | CH₂CH₂Ph(4-CF₃) |
| F-1862 | Me | H | CH₂CH₂Ph[4-CF(CF₃)₂] |
| F-1863 | Me | H | CH₂CH₂Ph[4-(*c*-Pr)] |
| F-1864 | Me | H | CH₂CH₂Ph{4-[*c*-Pr(2,2-F₂)]} |
| F-1865 | Me | H | CH₂CH₂Ph(4-OMe) |
| F-1866 | Me | H | CH₂CH₂Ph(3-OCF₃) |
| F-1867 | Me | H | CH₂CH₂Ph(4-OCHF₂) |
| F-1868 | Me | H | CH₂CH₂Ph(4-OCF₃) |
| F-1869 | Me | H | CH₂CH₂Ph(4-SMe) |
| F-1870 | Me | H | CH₂CH₂Ph(4-SCHF₂) |
| F-1871 | Me | H | CH₂CH₂Ph(4-SCF₃) |
| F-1872 | Me | H | CH₂CH₂Ph[4-S(=O)Me] |
| F-1873 | Me | H | CH₂CH₂Ph[4-S(=O)CF₃] |
| F-1874 | Me | H | CH₂CH₂Ph[4-S(=O)₂Me] |
| F-1875 | Me | H | CH₂CH₂Ph[4-S(=O)₂CF₃] |
| F-1876 | Me | H | CH₂CH₂Ph(4-CH₂SMe) |
| F-1877 | Me | H | CH₂CH₂Ph(4-CH₂SCF₃) |
| F-1878 | Me | H | CH₂CH₂Ph[4-OS(=O)₂Me] |
| F-1879 | Me | H | CH₂CH₂Ph[4-OS(=O)₂CF₃ |
| F-1880 | Me | H | CH₂CH₂Ph[4-Ph(4-CF₃)] |
| F-1881 | Me | H | CH₂CH₂Ph(4-CH₂Ph) |
| F-1882 | Me | H | CH₂CH₂Ph(4-OCH₂Ph) |
| F-1883 | Me | H | CH₂CH₂Ph(4-CN) |
| F-1884 | Me | H | CH₂CH₂Ph(4-NO₂) |
| F-1885 | Me | H | CH₂CH₂Ph(2,4-Cl₂) |
| F-1886 | Me | H | CH₂CH₂Ph(3,4-Cl₂) |

**[Table 1108]**

| Compound Number | R³ | R⁴ | R⁵ |
|---|---|---|---|
| F-1887 | Me | H | CH₂CH₂Ph(3-CF₃-4-F) |
| F-1888 | Me | H | CH₂CH₂Ph(2-CF₃-4-F) |
| F-1889 | Me | H | CH₂CH₂Ph(3-F-4-CF₃) |
| F-1890 | Me | H | CH₂CH₂Ph(2-F-4-CF₃) |
| F-1891 | Me | H | CH₂CH₂Ph(3-Cl-4-OCHF₂) |
| F-1892 | Me | H | CH₂CH₂Ph(3,4,5-Cl₃) |
| F-1893 | Me | H | CH₂CH₂Ph(2,3,4-F₃) |
| F-1894 | Me | H | CH₂CH₂Ph(2,4,5-F₃) |
| F-1895 | Me | H | CH₂CH₂Ph(3,4,5-F₃) |
| F-1896 | Me | H | CH₂CH₂Ph(2,4,6-F₃) |
| F-1897 | Me | H | CH₂CH₂CH₂Ph |
| F-1898 | Me | H | CH₂CH₂CH₂Ph(3-CF₃) |
| F-1899 | Me | H | CH₂CH₂CH₂Ph(4-CF₃) |
| F-1900 | Me | H | CH₂CH₂CH₂Ph(4-F) |
| F-1901 | Me | H | CH₂CH₂CH₂Ph[4-(*t*-Bu)] |
| F-1902 | Me | H | CH₂CH₂CH₂Ph(4-CN) |
| F-1903 | Me | H | CH₂CH₂CH₂Ph(4-CF₃) |
| F-1904 | Me | H | CH₂CH₂CH₂Ph(4-OCHF₂) |
| F-1905 | Me | H | CH₂CH₂CH₂Ph(4-OCF₃) |
| F-1906 | Me | H | CH₂CH₂CH₂Ph(4-SCHF₂) |
| F-1907 | Me | H | CH₂CH₂CH₂Ph(4-SCF₃) |
| F-1908 | Me | H | CH₂CH₂CH₂Ph[4-CF(CF₃)₂] |
| F-1909 | Me | H | CH₂CH₂CH₂Ph(3,4,5-F₃) |
| F-1910 | Me | H | CH₂CH₂CH₂Ph(2,4,6-F₃) |
| F-1911 | Me | H | CH₂CH₂CH₂CH₂Ph |
| F-1912 | Me | H | CH₂CH₂CH₂CH₂Ph(4-F) |
| F-1913 | Me | H | CH₂CH₂CH₂CH₂Ph(4-CF₃) |
| F-1914 | Me | H | CH₂CH₂CH₂CH₂Ph(4-OCF₃) |
| F-1915 | Me | H | CH₂CH₂CH₂CH₂Ph(4-SCF₃) |
| F-1916 | Me | H | CH₂CH₂CH₂CH₂CH₂Ph |
| F-1917 | Me | H | CH₂CH₂CH₂CH₂CH₂CH₂Ph |
| F-1918 | Me | H | CH₂CF₂Ph(4-F) |
| F-1919 | Me | H | CH₂CF₂Ph(4-CF₃) |
| F-1920 | Me | H | CH₂CF₂Ph(3,4,5-F₃) |
| F-1921 | CF₃ | H | CH₂CH(Me)NHS(=O)₂CF₃ |
| F-1922 | CF₃ | H | CH₂C*H(Me)NHS(=O)₂CF₃ : (R) |
| F-1923 | CF₃ | H | CH₂C*H(Me)NHS(=O)₂CF₃ : (S) |
| F-1924 | OCF₃ | H | CH₂CH(Me)NHS(=O)₂CF₃ |
| F-1925 | OCF₃ | H | CH₂C*H(Me)NHS(=O)₂CF₃ : (R) |
| F-1926 | OCF₃ | H | CH₂C*H(Me)NHS(=O)₂CF₃ : (S) |
| F-1927 | Cl | Cl | CH₂CH₂SCHF₂ |
| F-1928 | Cl | Cl | CH₂CH₂CH₂SCHF₂ |
| F-1929 | Cl | Cl | CH₂CH₂CH₂CH₂SCHF₂ |
| F-1930 | Cl | Cl | CH₂CH₂CH₂CH₂CH₂SCHF₂ |

**[Table 1109]**

| Compound Number | R³ | R⁴ | R⁵ |
|---|---|---|---|
| F-1931 | Cl | Cl | CH₂CH₂CH₂CH₂CH₂CH₂SCHF₂ |
| F-1932 | Cl | Cl | CH₂CH₂CH₂CH₂CH₂CH₂CH₂SCHF₂ |
| F-1933 | Cl | Cl | CH₂CH₂SCF₃ |
| F-1934 | Cl | Cl | CH₂CH₂CH₂SCF₃ |
| F-1935 | Cl | Cl | CH₂CH₂CH₂CH₂SCF₃ |
| F-1936 | Cl | Cl | CH₂CH₂CH₂CH₂CH₂SCF₃ |
| F-1937 | Cl | Cl | CH₂CH₂CH₂CH₂CH₂CH₂SCF₃ |
| F-1938 | Cl | Cl | CH₂CH₂CH₂CH₂CH₂CH₂CH₂SCF₃ |
| F-1939 | Cl | Cl | CH₂CH₂CH₂CH₂CH₂CH₂CH₂CH₂SCF₃ |
| F-1940 | Cl | Cl | CH₂CH(Me)SCF₃ |
| F-1941 | Cl | Cl | CH(Me)CH₂SCF₃ |
| F-1942 | Cl | Cl | CH₂CH₂CH(Me)SCF₃ |
| F-1943 | Cl | Cl | CH₂CH(Me)CH₂SCF₃ |
| F-1944 | Cl | Cl | CH(Me)CH₂CH₂SCF₃ |
| F-1945 | Cl | Cl | CH₂CH₂CH₂CH(Me)SCF₃ |
| F-1946 | Cl | Cl | CH₂CH₂CH(Me)CH₂SCF₃ |
| F-1947 | Cl | Cl | CH₂CH(Me)CH₂CH₂SCF₃ |
| F-1948 | Cl | Cl | CH(Me)CH₂CH₂CH₂SCF₃ |
| F-1949 | Cl | Cl | CH₂CH₂NHS(=O)₂CF₃ |
| F-1950 | Cl | Cl | CH₂CH₂CH₂NHS(=O)₂CF₃ |
| F-1951 | Cl | Cl | CH₂CH₂CH₂CH₂NHS(=O)₂CF₃ |
| F-1952 | Cl | Cl | CH₂CH₂CH₂CH₂CH₂NHS(=O)₂CF₃ |
| F-1953 | Cl | Cl | CH₂CH₂N(Me)S(=O)₂CF₃ |
| F-1954 | Cl | Cl | CH₂CH₂N(Et)S(=O)₂CF₃ |
| F-1955 | Cl | Cl | C H₂CH₂N(CH₂CN)S(=O)₂CF₃ |
| F-1956 | Cl | Cl | CH₂CH₂N(CH₂OMe)S(=O)₂CF₃ |
| F-1957 | Cl | Cl | CH₂CH₂CH₂N(Me)S(=O)₂CF₃ |
| F-1958 | Cl | Cl | CH₂CH₂CH₂N(Et)S(=O)₂CF₃ |
| F-1959 | Cl | Cl | CH₂CH₂CH₂N(CH₂CN)S(=O)₂CF₃ |
| F-1960 | Cl | Cl | CH₂CH₂CH₂N(CH₂OMe)S(=O)₂CF₃ |
| F-1961 | Cl | Cl | CH₂CH₂CH₂CH₂N(Me)S(=O)₂CF₃ |
| F-1962 | Cl | Cl | CH₂CH₂CH₂CH₂N(Et)S(=O)₂CF₃ |
| F-1963 | Cl | Cl | CH₂CH₂CH₂CH₂N(CH₂CN)S(=O)₂CF₃ |
| F-1964 | Cl | Cl | CH₂CH₂CH₂CH₂N(CH₂OMe)S(=O)₂CF₃ |
| F-1965 | Cl | Cl | CH₂CH(Me)NHS(=O)₂CF₃ |
| F-1966 | Cl | Cl | CH(Me)CH₂NHS(=O)₂CF₃ |
| F-1967 | Cl | Cl | CH₂CH₂CH(Me)NHS(=O)₂CF₃ |
| F-1968 | Cl | Cl | CH₂CH(Me)CH₂NHS(=O)₂CF₃ |
| F-1969 | Cl | Cl | CH(Me)CH₂CH₂NHS(=O)₂CF₃ |
| F-1970 | Cl | Cl | CH₂CH(Me)N(Me)S(=O)₂CF₃ |
| F-1971 | Cl | Cl | CH(Me)CH₂N(Me)S(=O)₂CF₃ |
| F-1972 | Cl | Cl | CH₂CH₂CH(Me)N(Me)S(=O)₂CF₃ |
| F-1973 | Cl | Cl | CH₂CH(Me)CH₂N(Me)S(=O)₂CF₃ |
| F-1974 | Cl | Cl | CH(Me)CH₂CH₂N(Me)S(=O)₂CF₃ |

**[Table 1110]**

| Compound Number | R³ | R⁴ | R⁵ |
|---|---|---|---|
| F-1975 | Cl | Cl | CH₂Ph |
| F-1976 | Cl | Cl | CH₂Ph(2-CF₃) |
| F-1977 | Cl | Cl | CH₂Ph(3-CF₃) |
| F-1978 | Cl | Cl | CH₂Ph(4-CF₃) |
| F-1979 | Cl | Cl | CH₂Ph(2-OCF₃) |
| F-1980 | Cl | Cl | CH₂Ph(3-OCF₃) |
| F-1981 | Cl | Cl | CH₂Ph(4-OCF₃) |
| F-1982 | Cl | Cl | CH₂Ph(2-SCF₃) |
| F-1983 | Cl | Cl | CH₂Ph(3-SCF₃) |
| F-1984 | Cl | Cl | CH₂Ph(4-SCF₃) |
| F-1985 | Cl | Cl | CH₂Ph(3-CH₂SCF₃) |
| F-1986 | Cl | Cl | CH₂Ph(4-F) |
| F-1987 | Cl | Cl | CH₂Ph(4-Cl) |
| F-1988 | Cl | Cl | CH₂Ph(4-Br) |
| F-1989 | Cl | Cl | CH₂Ph(4-Me) |
| F-1990 | Cl | Cl | CH₂Ph[4-(*t*-Bu)] |
| F-1991 | Cl | Cl | CH₂Ph(4-CN) |
| F-1992 | Cl | Cl | CH₂Ph(4-NO₂) |
| F-1993 | Cl | Cl | CH₂Ph(4-OCHF₂) |
| F-1994 | Cl | Cl | CH₂Ph(4-SCHF₂) |
| F-1995 | Cl | Cl | CH₂Ph(4-CH₂SCF₃) |
| F-1996 | Cl | Cl | CH₂Ph[4-CF(CF₃)₂] |
| F-1997 | Cl | Cl | CH₂Ph(4-CH₂SCF₃) |
| F-1998 | Cl | Cl | CH₂Ph[4-Ph(4-CF₃)] |
| F-1999 | Cl | Cl | CH₂Ph(2,4-Cl₂) |
| F-2000 | Cl | Cl | CH₂Ph[2,5-(CF₃)₂] |
| F-2001 | Cl | Cl | CH₂Ph(3,4-Cl₂) |
| F-2002 | Cl | Cl | CH₂Ph(3-CF₃-4-F) |
| F-2003 | Cl | Cl | CH₂Ph(3-CF₃-4-Cl) |
| F-2004 | Cl | Cl | CH₂Ph(3-F-4-CF₃) |
| F-2005 | Cl | Cl | CH₂Ph(2,4,6-F₃) |
| F-2006 | Cl | Cl | CH₂Ph(3,4,5-F₃) |
| F-2007 | Cl | Cl | CH₂Ph(2,3,4-F₃) |
| F-2008 | Cl | Cl | CH₂Ph(3,4,5-Cl₃) |
| F-2009 | Cl | Cl | CH₂CH₂Ph |
| F-2010 | Cl | Cl | CH₂CH₂Ph(4-F) |
| F-2011 | Cl | Cl | CH₂CH₂Ph(4-Cl) |
| F-2012 | Cl | Cl | CH₂CH₂Ph(4-Br) |
| F-2013 | Cl | Cl | CH₂CH₂Ph[4-(*t*-Bu)] |
| F-2014 | Cl | Cl | CH₂CH₂Ph(2-CF₃) |
| F-2015 | Cl | Cl | CH₂CH₂Ph(3-CF₃) |
| F-2016 | Cl | Cl | CH₂CH₂Ph(4-CF₃) |
| F-2017 | Cl | Cl | CH₂CH₂Ph[4-CF(CF₃)₂] |
| F-2018 | Cl | Cl | CH₂CH₂Ph[4-(*c*-Pr)] |

**[Table 1111]**

| Compound Number | R³ | R⁴ | R⁵ |
|---|---|---|---|
| F-2019 | Cl | Cl | CH₂CH₂Ph{4-[*c*-Pr(2,2-F₂)]} |
| F-2020 | Cl | Cl | CH₂CH₂Ph(4-OMe) |
| F-2021 | Cl | Cl | CH₂CH₂Ph(3-OCF₃) |
| F-2022 | Cl | Cl | CH₂CH₂Ph(4-OCHF₂) |
| F-2023 | Cl | Cl | CH₂CH₂Ph(4-OCF₃) |
| F-2024 | Cl | Cl | CH₂CH₂Ph(4-SMe) |
| F-2025 | Cl | Cl | CH₂CH₂Ph(4-SCHF₂) |
| F-2026 | Cl | Cl | CH₂CH₂Ph(4-SCF₃) |
| F-2027 | Cl | Cl | CH₂CH₂Ph[4-S(=O)Me] |
| F-2028 | Cl | Cl | CH₂CH₂Ph[4-S(=O)CF₃] |
| F-2029 | Cl | Cl | CH₂CH₂Ph[4-S(=O)₂Me] |
| F-2030 | Cl | Cl | CH₂CH₂Ph[4-S(=O)₂CF₃] |
| F-2031 | Cl | Cl | CH₂CH₂Ph(4-CH₂SMe) |
| F-2032 | Cl | Cl | CH₂CH₂Ph(4-CH₂SCF₃) |
| F-2033 | Cl | Cl | CH₂CH₂Ph[4-OS(=O)₂Me] |
| F-2034 | Cl | Cl | CH₂CH₂Ph[4-OS(=O)₂CF₃ |
| F-2035 | Cl | Cl | CH₂CH₂Ph[4-Ph(4-CF₃)] |
| F-2036 | Cl | Cl | CH₂CH₂Ph(4-CH₂Ph) |
| F-2037 | Cl | Cl | CH₂CH₂Ph(4-OCH₂Ph) |
| F-2038 | Cl | Cl | CH₂CH₂Ph(4-CN) |
| F-2039 | Cl | Cl | CH₂CH₂Ph(4-NO₂) |
| F-2040 | Cl | Cl | CH₂CH₂Ph(2,4-Cl₂) |
| F-2041 | Cl | Cl | CH₂CH₂Ph(3,4-Cl₂) |
| F-2042 | Cl | Cl | CH₂CH₂Ph(3-CF₃-4-F) |
| F-2043 | Cl | Cl | CH₂CH₂Ph(2-CF₃-4-F) |
| F-2044 | Cl | Cl | CH₂CH₂Ph(3-F-4-CF₃) |
| F-2045 | Cl | Cl | CH₂CH₂Ph(2-F-4-CF₃) |
| F-2046 | Cl | Cl | CH₂CH₂Ph(3-Cl-4-OCHF₂) |
| F-2047 | Cl | Cl | CH₂CH₂Ph(3,4,5-Cl₃) |
| F-2048 | Cl | Cl | CH₂CH₂Ph(2,3,4-F₃) |
| F-2049 | Cl | Cl | CH₂CH₂Ph(2,4,5-F₃) |
| F-2050 | Cl | Cl | CH₂CH₂Ph(3,4,5-F₃) |
| F-2051 | Cl | Cl | CH₂CH₂Ph(2,4,6-F₃) |
| F-2052 | Cl | Cl | CH₂CH₂CH₂Ph |
| F-2053 | Cl | Cl | CH₂CH₂CH₂Ph(3-CF₃) |
| F-2054 | Cl | Cl | CH₂CH₂CH₂Ph(4-CF₃) |
| F-2055 | Cl | Cl | CH₂CH₂CH₂Ph(4-F) |
| F-2056 | Cl | Cl | CH₂CH₂CH₂Ph[4-(*t*-Bu)] |
| F-2057 | Cl | Cl | CH₂CH₂CH₂Ph(4-CN) |
| F-2058 | Cl | Cl | CH₂CH₂CH₂Ph(4-CF₃) |
| F-2059 | Cl | Cl | CH₂CH₂CH₂Ph(4-OCHF₂) |
| F-2060 | Cl | Cl | CH₂CH₂CH₂Ph(4-OCF₃) |
| F-2061 | Cl | Cl | CH₂CH₂CH₂Ph(4-SCHF₂) |
| F-2062 | Cl | Cl | CH₂CH₂CH₂Ph(4-SCF₃) |

**[Table 1112]**

| Compound Number | R³ | R⁴ | R⁵ |
|---|---|---|---|
| F-2063 | Cl | Cl | CH₂CH₂CH₂Ph[4-CF(CF₃)₂] |
| F-2064 | Cl | Cl | CH₂CH₂CH₂Ph(3,4,5-F₃) |
| F-2065 | Cl | Cl | CH₂CH₂CH₂Ph(2,4,6-F₃) |
| F-2066 | Cl | Cl | CH₂CH₂CH₂CH₂Ph |
| F-2067 | Cl | Cl | CH₂CH₂CH₂CH₂Ph(4-F) |
| F-2068 | Cl | Cl | CH₂CH₂CH₂CH₂Ph(4-CF₃) |
| F-2069 | Cl | Cl | CH₂CH₂CH₂CH₂Ph(4-OCF₃) |
| F-2070 | Cl | Cl | CH₂CH₂CH₂CH₂Ph(4-SCF₃) |
| F-2071 | Cl | Cl | CH₂CH₂CH₂CH₂CH₂Ph |
| F-2072 | Cl | Cl | CH₂CH₂CH₂CH₂CH₂CH₂Ph |
| F-2073 | Cl | Cl | CH₂CF₂Ph(4-F) |
| F-2074 | Cl | Cl | CH₂CF₂Ph(4-CF₃) |
| F-2075 | Cl | Cl | CH₂CF₂Ph(3,4,5-F₃) |
| F-2076 | Me | Cl | CH₂CH₂SCHF₂ |
| F-2077 | Me | Cl | CH₂CH₂CH₂SCHF₂ |
| F-2078 | Me | Cl | CH₂CH₂CH₂CH₂SCHF₂ |
| F-2079 | Me | Cl | CH₂CH₂CH₂CH₂CH₂SCHF₂ |
| F-2080 | Me | Cl | CH₂CH₂CH₂CH₂CH₂CH₂SCHF₂ |
| F-2081 | Me | Cl | CH₂CH₂CH₂CH₂CH₂CH₂CH₂SCHF₂ |
| F-2082 | Me | Cl | CH₂CH₂SCF₃ |
| F-2083 | Me | Cl | CH₂CH₂CH₂SCF₃ |
| F-2084 | Me | Cl | CH₂CH₂CH₂CH₂SCF₃ |
| F-2085 | Me | Cl | CH₂CH₂CH₂CH₂CH₂SCF₃ |
| F-2086 | Me | Cl | CH₂CH₂CH₂CH₂CH₂CH₂SCF₃ |
| F-2087 | Me | Cl | CH₂CH₂CH₂CH₂CH₂CH₂CH₂SCF₃ |
| F-2088 | Me | Cl | CH₂CH₂CH₂CH₂CH₂CH₂CH₂CH₂SCF₃ |
| F-2089 | Me | Cl | CH₂CH(Me)SCF₃ |
| F-2090 | Me | Cl | CH(Me)CH₂SCF₃ |
| F-2091 | Me | Cl | CH₂CH₂CH(Me)SCF₃ |
| F-2092 | Me | Cl | CH₂CH(Me)CH₂SCF₃ |
| F-2093 | Me | Cl | CH(Me)CH₂CH₂SCF₃ |
| F-2094 | Me | Cl | CH₂CH₂CH₂CH(Me)SCF₃ |
| F-2095 | Me | Cl | CH₂CH₂CH(Me)CH₂SCF₃ |
| F-2096 | Me | Cl | CH₂CH(Me)CH₂CH₂SCF₃ |
| F-2097 | Me | Cl | CH(Me)CH₂CH₂CH₂SCF₃ |
| F-2098 | Me | Cl | CH₂CH₂NHS(=O)₂CF₃ |
| F-2099 | Me | Cl | CH₂CH₂CH₂NHS(=O)₂CF₃ |
| F-2100 | Me | Cl | CH₂CH₂CH₂CH₂NHS(=O)₂CF₃ |
| F-2101 | Me | Cl | CH₂CH₂CH₂CH₂CH₂NHS(=O)₂CF₃ |
| F-2102 | Me | Cl | CH₂CH₂N(Me)S(=O)₂CF₃ |
| F-2103 | Me | Cl | CH₂CH₂N(Et)S(=O)₂CF₃ |
| F-2104 | Me | Cl | CH₂CH₂N(CH₂CN)S(=O)₂CF₃ |
| F-2105 | Me | Cl | CH₂CH₂N(CH₂OMe)S(=O)₂CF₃ |
| F-2106 | Me | Cl | CH₂CH₂CH₂N(Me)S(=O)₂CF₃ |

**[Table 1113]**

| Compound Number | R³ | R⁴ | R⁵ |
|---|---|---|---|
| F-2107 | Me | Cl | CH₂CH₂CH₂N(Et)S(=O)₂CF₃ |
| F-2108 | Me | Cl | CH₂CH₂CH₂N(CH₂CN)S(=O)₂CF₃ |
| F-2109 | Me | Cl | CH₂CH₂CH₂N(CH₂OMe)S(=O)₂CF₃ |
| F-2110 | Me | Cl | CH₂CH₂CH₂CH₂N(Me)S(=O)₂CF₃ |
| F-2111 | Me | Cl | CH₂CH₂CH₂CH₂N(Et)S(=O)₂CF₃ |
| F-2112 | Me | Cl | CH₂CH₂CH₂CH₂N(CH₂CN)S(=O)₂CF₃ |
| F-2113 | Me | Cl | CH₂CH₂CH₂CH₂N(CH₂OMe)S(=O)₂CF₃ |
| F-2114 | Me | Cl | CH₂CH(Me)NHS(=O)₂CF₃ |
| F-2115 | Me | Cl | CH(Me)CH₂NHS(=O)₂CF₃ |
| F-2116 | Me | Cl | CH₂CH₂CH(Me)NHS(=O)₂CF₃ |
| F-2117 | Me | Cl | CH₂CH(Me)CH₂NHS(=O)₂CF₃ |
| F-2118 | Me | Cl | CH(Me)CH₂CH₂NHS(=O)₂CF₃ |
| F-2119 | Me | Cl | CH₂CH(Me)N(Me)S(=O)₂CF₃ |
| F-2120 | Me | Cl | CH(Me)CH₂N(Me)S(=O)₂CF₃ |
| F-2121 | Me | Cl | CH₂CH₂CH(Me)N(Me)S(=O)₂CF₃ |
| F-2122 | Me | Cl | CH₂CH(Me)CH₂N(Me)S(=O)₂CF₃ |
| F-2123 | Me | Cl | CH(Me)CH₂CH₂N(Me)S(=O)₂CF₃ |
| F-2124 | Me | Cl | CH₂Ph |
| F-2125 | Me | Cl | CH₂Ph(2-CF₃) |
| F-2126 | Me | Cl | CH₂Ph(3-CF₃) |
| F-2127 | Me | Cl | CH₂Ph(4-CF₃) |
| F-2128 | Me | Cl | CH₂Ph(2-OCF₃) |
| F-2129 | Me | Cl | CH₂Ph(3-OCF₃) |
| F-2130 | Me | Cl | CH₂Ph(4-OCF₃) |
| F-2131 | Me | Cl | CH₂Ph(2-SCF₃) |
| F-2132 | Me | Cl | CH₂Ph(3-SCF₃) |
| F-2133 | Me | Cl | CH₂Ph(4-SCF₃) |
| F-2134 | Me | Cl | CH₂Ph(3-CH₂SCF₃) |
| F-2135 | Me | Cl | CH₂Ph(4-F) |
| F-2136 | Me | Cl | CH₂Ph(4-Cl) |
| F-2137 | Me | Cl | CH₂Ph(4-Br) |
| F-2138 | Me | Cl | CH₂Ph(4-Me) |
| F-2139 | Me | Cl | CH₂Ph[4-(*t*-Bu)] |
| F-2140 | Me | Cl | CH₂Ph(4-CN) |
| F-2141 | Me | Cl | CH₂Ph(4-NO₂) |
| F-2142 | Me | Cl | CH₂Ph(4-OCHF₂) |
| F-2143 | Me | Cl | CH₂Ph(4-SCHF₂) |
| F-2144 | Me | Cl | CH₂Ph(4-CH₂SCF₃) |
| F-2145 | Me | Cl | CH₂Ph[4-CF(CF₃)₂] |
| F-2146 | Me | Cl | CH₂Ph(4-CH₂SCF₃) |
| F-2147 | Me | Cl | CH₂Ph[4-Ph(4-CF₃)] |
| F-2148 | Me | Cl | CH₂Ph(2,4-Cl₂) |
| F-2149 | Me | Cl | CH₂Ph[2,5-(CF₃)₂] |
| F-2150 | Me | Cl | CH₂Ph(3,4-Cl₂) |

**[Table 1114]**

| Compound Number | R³ | R⁴ | R⁵ |
|---|---|---|---|
| F-2151 | Me | Cl | CH₂Ph(3-CF₃-4-F) |
| F-2152 | Me | Cl | CH₂Ph(3-CF₃-4-Cl) |
| F-2153 | Me | Cl | CH₂Ph(3-F-4-CF₃) |
| F-2154 | Me | Cl | CH₂Ph(2,4,6-F₃) |
| F-2155 | Me | Cl | CH₂Ph(3,4,5-F₃) |
| F-2156 | Me | Cl | CH₂Ph(2,3,4-F₃) |
| F-2157 | Me | Cl | CH₂Ph(3,4,5-Cl₃) |
| F-2158 | Me | Cl | CH₂CH₂Ph |
| F-2159 | Me | Cl | CH₂CH₂Ph(4-F) |
| F-2160 | Me | Cl | CH₂CH₂Ph(4-Cl) |
| F-2161 | Me | Cl | CH₂CH₂Ph(4-Br) |
| F-2162 | Me | Cl | CH₂CH₂Ph[4-(*t*-Bu)] |
| F-2163 | Me | Cl | CH₂CH₂Ph(2-CF₃) |
| F-2164 | Me | Cl | CH₂CH₂Ph(3-CF₃) |
| F-2165 | Me | Cl | CH₂CH₂Ph(4-CF₃) |
| F-2166 | Me | Cl | CH₂CH₂Ph[4-CF(CF₃)₂] |
| F-2167 | Me | Cl | CH₂CH₂Ph[4-(*c*-Pr)] |
| F-2168 | Me | Cl | CH₂CH₂Ph{4-[*c*-Pr(2,2-F₂)]} |
| F-2169 | Me | Cl | CH₂CH₂Ph(4-OMe) |
| F-2170 | Me | Cl | CH₂CH₂Ph(3-OCF₃) |
| F-2171 | Me | Cl | CH₂CH₂Ph(4-OCHF₂) |
| F-2172 | Me | Cl | CH₂CH₂Ph(4-OCF₃) |
| F-2173 | Me | Cl | CH₂CH₂Ph(4-SMe) |
| F-2174 | Me | Cl | CH₂CH₂Ph(4-SCHF₂) |
| F-2175 | Me | Cl | CH₂CH₂Ph(4-SCF₃) |
| F-2176 | Me | Cl | CH₂CH₂Ph[4-S(=O)Me] |
| F-2177 | Me | Cl | CH₂CH₂Ph[4-S(=O)CF₃] |
| F-2178 | Me | Cl | CH₂CH₂Ph[4-S(=O)₂Me] |
| F-2179 | Me | Cl | CH₂CH₂Ph[4-S(=O)₂CF₃ |
| F-2180 | Me | Cl | CH₂CH₂Ph(4-CH₂SMe) |
| F-2181 | Me | Cl | CH₂CH₂Ph(4-CH₂SCF₃) |
| F-2182 | Me | Cl | CH₂CH₂Ph[4-OS(=O)₂Me] |
| F-2183 | Me | Cl | CH₂CH₂Ph[4-OS(=O)₂CF₃] |
| F-2184 | Me | Cl | CH₂CH₂Ph[4-Ph(4-CF₃) |
| F-2185 | Me | Cl | CH₂CH₂Ph(4-CH₂Ph) |
| F-2186 | Me | Cl | CH₂CH₂Ph(4-OCH₂Ph) |
| F-2187 | Me | Cl | CH₂CH₂Ph(4-CN) |
| F-2188 | Me | Cl | CH₂CH₂Ph(4-NO₂) |
| F-2189 | Me | Cl | CH₂CH₂Ph(2,4-Cl₂) |
| F-2190 | Me | Cl | CH₂CH₂Ph(3,4-Cl₂) |
| F-2191 | Me | Cl | CH₂CH₂Ph(3-CF₃-4-F) |
| F-2192 | Me | Cl | CH₂CH₂Ph(2-CF₃-4-F) |
| F-2193 | Me | Cl | CH₂CH₂Ph(3-F-4-CF₃) |
| F-2194 | Me | Cl | CH₂CH₂Ph(2-F-4-CF₃) |

**[Table 1115]**

| Compound Number | R³ | R⁴ | R⁵ |
|---|---|---|---|
| F-2195 | Me | Cl | CH₂CH₂Ph(3-Cl-4-OCHF₂) |
| F-2196 | Me | Cl | CH₂CH₂Ph(3,4,5-Cl₃) |
| F-2197 | Me | Cl | CH₂CH₂Ph(2,3,4-F₃) |
| F-2198 | Me | Cl | CH₂CH₂Ph(2,4,5-F₃) |
| F-2199 | Me | Cl | CH₂CH₂Ph(3,4,5-F₃) |
| F-2200 | Me | Cl | CH₂CH₂Ph(2,4,6-F₃) |
| F-2201 | Me | Cl | CH₂CH₂CH₂Ph |
| F-2202 | Me | Cl | CH₂CH₂CH₂Ph(3-CF₃) |
| F-2203 | Me | Cl | CH₂CH₂CH₂Ph(4-CF₃) |
| F-2204 | Me | Cl | CH₂CH₂CH₂Ph(4-F) |
| F-2205 | Me | Cl | CH₂CH₂CH₂Ph[4-(*t*-Bu)] |
| F-2206 | Me | Cl | CH₂CH₂CH₂Ph(4-CN) |
| F-2207 | Me | Cl | CH₂CH₂CH₂Ph(4-CF₃) |
| F-2208 | Me | Cl | CH₂CH₂CH₂Ph(4-OCHF₂) |
| F-2209 | Me | Cl | CH₂CH₂CH₂Ph(4-OCF₃) |
| F-2210 | Me | Cl | CH₂CH₂CH₂Ph(4-SCHF₂) |
| F-2211 | Me | Cl | CH₂CH₂CH₂Ph(4-SCF₃) |
| F-2212 | Me | Cl | CH₂CH₂CH₂Ph[4-CF(CF₃)₂] |
| F-2213 | Me | Cl | CH₂CH₂CH₂Ph(3,4,5-F₃) |
| F-2214 | Me | Cl | CH₂CH₂CH₂Ph(2,4,6-F₃) |
| F-2215 | Me | Cl | CH₂CH₂CH₂CH₂Ph |
| F-2216 | Me | Cl | CH₂CH₂CH₂CH₂Ph(4-F) |
| F-2217 | Me | Cl | CH₂CH₂CH₂CH₂Ph(4-CF₃) |
| F-2218 | Me | Cl | CH₂CH₂CH₂CH₂Ph(4-OCF₃) |
| F-2219 | Me | Cl | CH₂CH₂CH₂CH₂Ph(4-SCF₃) |
| F-2220 | Me | Cl | CH₂CH₂CH₂CH₂CH₂Ph |
| F-2221 | Me | Cl | CH₂CH₂CH₂CH₂CH₂CH₂Ph |
| F-2222 | Me | Cl | CH₂CF₂Ph(4-F) |
| F-2223 | Me | Cl | CH₂CF₂Ph(4-CF₃) |
| F-2224 | Me | Cl | CH₂CF₂Ph(3,4,5-F₃) |
| F-2225 | Me | Me | CH₂CH₂SCHF₂ |
| F-2226 | Me | Me | CH₂CH₂CH₂SCHF₂ |
| F-2227 | Me | Me | CH₂CH₂CH₂CH₂SCHF₂ |
| F-2228 | Me | Me | CH₂CH₂CH₂CH₂CH₂SCHF₂ |
| F-2229 | Me | Me | CH₂CH₂CH₂CH₂CH₂CH₂SCHF₂ |
| F-2230 | Me | Me | CH₂CH₂CH₂CH₂CH₂CH₂CH₂SCHF₂ |
| F-2231 | Me | Me | CH₂CH₂SCF₃ |
| F-2232 | Me | Me | CH₂CH₂CH₂SCF₃ |
| F-2233 | Me | Me | CH₂CH₂CH₂CH₂SCF₃ |
| F-2234 | Me | Me | CH₂CH₂CH₂CH₂CH₂SCF₃ |
| F-2235 | Me | Me | CH₂CH₂CH₂CH₂CH₂CH₂SCF₃ |
| F-2236 | Me | Me | CH₂CH₂CH₂CH₂CH₂CH₂CH₂SCF₃ |
| F-2237 | Me | Me | CH₂CH₂CH₂CH₂CH₂CH₂CH₂CH₂SCF₃ |
| F-2238 | Me | Me | CH₂CH(Me)SCF₃ |

**[Table 1116]**

| Compound Number | R³ | R⁴ | R⁵ |
|---|---|---|---|
| F-2239 | Me | Me | CH(Me)CH₂SCF₃ |
| F-2240 | Me | Me | CH₂CH₂CH(Me)SCF₃ |
| F-2241 | Me | Me | CH₂CH(Me)CH₂SCF₃ |
| F-2242 | Me | Me | CH(Me)CH₂CH₂SCF₃ |
| F-2243 | Me | Me | CH₂CH₂CH₂CH(Me)SCF₃ |
| F-2244 | Me | Me | CH₂CH₂CH(Me)CH₂SCF₃ |
| F-2245 | Me | Me | CH₂CH(Me)CH₂CH₂SCF₃ |
| F-2246 | Me | Me | CH(Me)CH₂CH₂CH₂SCF₃ |
| F-2247 | Me | Me | CH₂CH₂NHS(=O)₂CF₃ |
| F-2248 | Me | Me | CH₂CH₂CH₂NHS(=O)₂CF₃ |
| F-2249 | Me | Me | CH₂CH₂CH₂CH₂NHS(=O)₂CF₃ |
| F-2250 | Me | Me | CH₂CH₂CH₂CH₂CH₂NHS(=O)₂CF₃ |
| F-2251 | Me | Me | CH₂CH₂N(Me)S(=O)₂CF₃ |
| F-2252 | Me | Me | CH₂CH₂N(Et)S(=O)₂CF₃ |
| F-2253 | Me | Me | CH₂CH₂N(CH₂CN)S(=O)₂CF₃ |
| F-2254 | Me | Me | CH₂CH₂N(CH₂OMe)S(=O)₂CF₃ |
| F-2255 | Me | Me | CH₂CH₂CH₂N(Me)S(=O)₂CF₃ |
| F-2256 | Me | Me | CH₂CH₂CH₂N(Et)S(=O)₂CF₃ |
| F-2257 | Me | Me | CH₂CH₂CH₂N(CH₂CN)S(=O)₂CF₃ |
| F-2258 | Me | Me | CH₂CH₂CH₂N(CH₂OMe)S(=O)₂CF₃ |
| F-2259 | Me | Me | CH₂CH₂CH₂CH₂N(Me)S(=O)₂CF₃ |
| F-2260 | Me | Me | CH₂CH₂CH₂CH₂N(Et)S(=O)₂CF₃ |
| F-2261 | Me | Me | CH₂CH₂CH₂CH₂N(CH₂CN)S(=O)₂CF₃ |
| F-2262 | Me | Me | CH₂CH₂CH₂CH₂N(CH₂OMe)S(=O)₂CF₃ |
| F-2263 | Me | Me | CH₂CH(Me)NHS(=O)₂CF₃ |
| F-2264 | Me | Me | CH(Me)CH₂NHS(=O)₂CF₃ |
| F-2265 | Me | Me | CH₂CH₂CH(Me)NHS(=O)₂CF₃ |
| F-2266 | Me | Me | CH₂CH(Me)CH₂NHS(=O)₂CF₃ |
| F-2267 | Me | Me | CH(Me)CH₂CH₂NHS(=O)₂CF₃ |
| F-2268 | Me | Me | CH₂CH(Me)N(Me)S(=O)₂CF₃ |
| F-2269 | Me | Me | CH(Me)CH₂N(Me)S(=O)₂CF₃ |
| F-2270 | Me | Me | CH₂CH₂CH(Me)N(Me)S(=O)₂CF₃ |
| F-2271 | Me | Me | CH₂CH(Me)CH₂N(Me)S(=O)₂CF₃ |
| F-2272 | Me | Me | CH(Me)CH₂CH₂N(Me)S(=O)₂CF₃ |
| F-2273 | Me | Me | CH₂Ph |
| F-2274 | Me | Me | CH₂Ph(2-CF₃) |
| F-2275 | Me | Me | CH₂Ph(3-CF₃) |
| F-2276 | Me | Me | CH₂Ph(4-CF₃) |
| F-2277 | Me | Me | CH₂Ph(2-OCF₃) |
| F-2278 | Me | Me | CH₂Ph(3-OCF₃) |
| F-2279 | Me | Me | CH₂Ph(4-OCF₃) |
| F-2280 | Me | Me | CH₂Ph(2-SCF₃) |
| F-2281 | Me | Me | CH₂Ph(3-SCF₃) |
| F-2282 | Me | Me | CH₂Ph(4-SCF₃) |

**[Table 1117]**

| Compound Number | R³ | R⁴ | R⁵ |
|---|---|---|---|
| F-2283 | Me | Me | CH₂Ph(3-CH₂SCF₃) |
| F-2284 | Me | Me | CH₂Ph(4-F) |
| F-2285 | Me | Me | CH₂Ph(4-Cl) |
| F-2286 | Me | Me | CH₂Ph(4-Br) |
| F-2287 | Me | Me | CH₂Ph(4-Me) |
| F-2288 | Me | Me | CH₂Ph[4-(*t*-Bu)] |
| F-2289 | Me | Me | CH₂Ph(4-CN) |
| F-2290 | Me | Me | CH₂Ph(4-NO₂) |
| F-2291 | Me | Me | CH₂Ph(4-OCHF₂) |
| F-2292 | Me | Me | CH₂Ph(4-SCHF₂) |
| F-2293 | Me | Me | CH₂Ph(4-CH₂SCF₃) |
| F-2294 | Me | Me | CH₂Ph[4-CF(CF₃)₂] |
| F-2295 | Me | Me | CH₂Ph(4-CH₂SCF₃) |
| F-2296 | Me | Me | CH₂Ph[4-Ph(4-CF₃)] |
| F-2297 | Me | Me | CH₂Ph(2,4-Cl₂) |
| F-2298 | Me | Me | CH₂Ph[2,5-(CF₃)₂] |
| F-2299 | Me | Me | CH₂Ph(3,4-Cl₂) |
| F-2300 | Me | Me | CH₂Ph(3-CF₃-4-F) |
| F-2301 | Me | Me | CH₂Ph(3-CF₃-4-Cl) |
| F-2302 | Me | Me | CH₂Ph(3-F-4-CF₃) |
| F-2303 | Me | Me | CH₂Ph(2,4,6-F₃) |
| F-2304 | Me | Me | CH₂Ph(3,4,5-F₃) |
| F-2305 | Me | Me | CH₂Ph(2,3,4-F₃) |
| F-2306 | Me | Me | CH₂Ph(3,4,5-Cl₃) |
| F-2307 | Me | Me | CH₂CH₂Ph |
| F-2308 | Me | Me | CH₂CH₂Ph(4-F) |
| F-2309 | Me | Me | CH₂CH₂Ph(4-Cl) |
| F-2310 | Me | Me | CH₂CH₂Ph(4-Br) |
| F-2311 | Me | Me | CH₂CH₂Ph[4-(*t*-Bu)] |
| F-2312 | Me | Me | CH₂CH₂Ph(2-CF₃) |
| F-2313 | Me | Me | CH₂CH₂Ph(3-CF₃) |
| F-2314 | Me | Me | CH₂CH₂Ph(4-CF₃) |
| F-2315 | Me | Me | CH₂CH₂Ph[4-CF(CF₃)₂] |
| F-2316 | Me | Me | CH₂CH₂Ph[4-(*c*-Pr)] |
| F-2317 | Me | Me | CH₂CH₂Ph{4-[*c*-Pr(2,2-F₂)]} |
| F-2318 | Me | Me | CH₂CH₂Ph(4-OMe) |
| F-2319 | Me | Me | CH₂CH₂Ph(3-OCF₃) |
| F-2320 | Me | Me | CH₂CH₂Ph(4-OCHF₂) |
| F-2321 | Me | Me | CH₂CH₂Ph(4-OCF₃) |
| F-2322 | Me | Me | CH₂CH₂Ph(4-SMe) |
| F-2323 | Me | Me | CH₂CH₂Ph(4-SCHF₂) |
| F-2324 | Me | Me | CH₂CH₂Ph(4-SCF₃) |
| F-2325 | Me | Me | CH₂CH₂Ph[4-S(=O)Me] |
| F-2326 | Me | Me | CH₂CH₂Ph[4-S(=O)CF₃] |

**[Table 1118]**

| Compound Number | R³ | R⁴ | R⁵ |
|---|---|---|---|
| F-2327 | Me | Me | CH₂CH₂Ph[4-S(=O)₂Me] |
| F-2328 | Me | Me | CH₂CH₂Ph[4-S(=O)₂CF₃ |
| F-2329 | Me | Me | CH₂CH₂Ph(4-CH₂SMe) |
| F-2330 | Me | Me | CH₂CH₂Ph(4-CH₂SCF₃) |
| F-2331 | Me | Me | CH₂CH₂Ph[4-OS(=O)₂Me] |
| F-2332 | Me | Me | CH₂CH₂Ph[4-OS(=O)₂CF₃] |
| F-2333 | Me | Me | CH₂CH₂Ph[4-Ph(4-CF₃)] |
| F-2334 | Me | Me | CH₂CH₂Ph(4-CH₂Ph) |
| F-2335 | Me | Me | CH₂CH₂Ph(4-OCH₂Ph) |
| F-2336 | Me | Me | CH₂CH₂Ph(4-CN) |
| F-2337 | Me | Me | CH₂CH₂Ph(4-NO₂) |
| F-2338 | Me | Me | CH₂CH₂Ph(2,4-Cl₂) |
| F-2339 | Me | Me | CH₂CH₂Ph(3,4-Cl₂) |
| F-2340 | Me | Me | CH₂CH₂Ph(3-CF₃-4-F) |
| F-2341 | Me | Me | CH₂CH₂Ph(2-CF₃-4-F) |
| F-2342 | Me | Me | CH₂CH₂Ph(3-F-4-CF₃) |
| F-2343 | Me | Me | CH₂CH₂Ph(2-F-4-CF₃) |
| F-2344 | Me | Me | CH₂CH₂Ph(3-Cl-4-OCHF₂) |
| F-2345 | Me | Me | CH₂CH₂Ph(3,4,5-Cl₃) |
| F-2346 | Me | Me | CH₂CH₂Ph(2,3,4-F₃) |
| F-2347 | Me | Me | CH₂CH₂Ph(2,4,5-F₃) |
| F-2348 | Me | Me | CH₂CH₂Ph(3,4,5-F₃) |
| F-2349 | Me | Me | CH₂CH₂Ph(2,4,6-F₃) |
| F-2350 | Me | Me | CH₂CH₂CH₂Ph |
| F-2351 | Me | Me | CH₂CH₂CH₂Ph(3-CF₃) |
| F-2352 | Me | Me | CH₂CH₂CH₂Ph(4-CF₃) |
| F-2353 | Me | Me | CH₂CH₂CH₂Ph(4-F) |
| F-2354 | Me | Me | CH₂CH₂CH₂Ph[4-(*t*-Bu)] |
| F-2355 | Me | Me | CH₂CH₂CH₂Ph(4-CN) |
| F-2356 | Me | Me | CH₂CH₂CH₂Ph(4-CF₃) |
| F-2357 | Me | Me | CH₂CH₂CH₂Ph(4-OCHF₂) |
| F-2358 | Me | Me | CH₂CH₂CH₂Ph(4-OCF₃) |
| F-2359 | Me | Me | CH₂CH₂CH₂Ph(4-SCHF₂) |
| F-2360 | Me | Me | CH₂CH₂CH₂Ph(4-SCF₃) |
| F-2361 | Me | Me | CH₂CH₂CH₂Ph[4-CF(CF₃)₂] |
| F-2362 | Me | Me | CH₂CH₂CH₂Ph(3,4,5-F₃) |
| F-2363 | Me | Me | CH₂CH₂CH₂Ph(2,4,6-F₃) |
| F-2364 | Me | Me | CH₂CH₂CH₂CH₂Ph |
| F-2365 | Me | Me | CH₂CH₂CH₂CH₂Ph(4-F) |
| F-2366 | Me | Me | CH₂CH₂CH₂CH₂Ph(4-CF₃) |
| F-2367 | Me | Me | CH₂CH₂CH₂CH₂Ph(4-OCF₃) |
| F-2368 | Me | Me | CH₂CH₂CH₂CH₂Ph(4-SCF₃) |
| F-2369 | Me | Me | CH₂CH₂CH₂CH₂CH₂Ph |
| F-2370 | Me | Me | CH₂CH₂CH₂CH₂CH₂CH₂Ph |

**[Table 1119]**

| Compound Number | R³ | R⁴ | R⁵ |
|---|---|---|---|
| F-2371 | Me | Me | CH₂CF₂Ph(4-F) |
| F-2372 | Me | Me | CH₂CF₂Ph(4-CF₃) |
| F-2373 | Me | Me | CH₂CF₂Ph(3,4,5-F₃) |

**[Table 1120]**

| 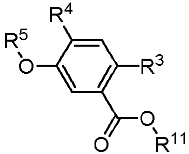 | | | | |
|---|---|---|---|---|
| Compound Number | R³ | R⁴ | R⁵ | R¹¹ |
| G-0001 | Cl | F | Me | Me |
| G-0002 | Cl | F | Et | Me |
| G-0003 | Cl | F | *n*-Pr | Me |
| G-0004 | Cl | F | *i*-Pr | Me |
| G-0005 | Cl | F | *n*-Bu | Me |
| G-0006 | Cl | F | *n*-Pen | Me |
| G-0007 | Cl | F | *n*-Hex | Me |
| G-0008 | Cl | F | *n*-Heptyl | Me |
| G-0009 | Cl | F | *n*-Octyl | Me |
| G-0010 | Cl | F | *n*-Nonyl | Me |
| G-0011 | Cl | F | *n*-Decyl | Me |
| G-0012 | Cl | F | *c* -Pr | Me |
| G-0013 | Cl | F | *c* -Pen | Me |
| G-0014 | Cl | F | *c* -Hex | Me |
| G-0015 | Cl | F | CH₂C(Me)=CH₂ | Me |
| G-0016 | Cl | F | CH₂CH₂CH=CH₂ | Me |
| G-0017 | Cl | F | CH₂CH₂CH=C(CH₃)₂ | Me |
| G-0018 | Cl | F | CH₂CH₂CH₂CH=CH₂ | Me |
| G-0019 | Cl | F | CH₂CH₂CH₂CH=C(CH₃)₂ | Me |
| G-0020 | Cl | F | CH₂CH₂CH₂CH₂CH=CH₂ | Me |
| G-0021 | Cl | F | CH₂CH₂CH₂CH₂CH=C(CH₃)₂ | Me |
| G-0022 | Cl | F | CH₂CH₂CH≡CH | Me |
| G-0023 | Cl | F | CH₂CH₂CH₂CH≡CH | Me |
| G-0024 | Cl | F | CH₂CH₂CH₂CH₂CH≡CH | Me |
| G-0025 | Cl | F | CH₂CH₂CH₂Cl | Me |
| G-0026 | Cl | F | CH₂CH₂CH₂Br | Me |
| G-0027 | Cl | F | CH₂CH₂CH₂CH₂Cl | Me |
| G-0028 | Cl | F | CH₂CH₂CH₂CH₂Br | Me |
| G-0029 | Cl | F | CH₂CH₂CH₂CH₂CH₂Cl | Me |
| G-0030 | Cl | F | CH₂CH₂CH₂CH₂CH₂Br | Me |
| G-0031 | Cl | F | CH₂CH₂CH₂CH₂CH₂CH₂Cl | Me |
| G-0032 | Cl | F | CH₂CH₂CH₂CH₂CH₂CH₂Br | Me |
| G-0033 | Cl | F | CH₂CH₂CH₂CH₂CH₂CH₂CH₂Cl | Me |
| G-0034 | Cl | F | CH₂CH₂CH₂CH₂CH₂CH₂CH₂Br | Me |
| G-0035 | Cl | F | CH₂CH₂CH₂CH₂CH₂CH₂CH₂CH₂Br | Me |
| G-0036 | Cl | F | CH₂CH(Me)CH₂Cl | Me |
| G-0037 | Cl | F | CH₂CH(Me)CH₂Br | Me |
| G-0038 | Cl | F | CH₂CH₂CH(Me)CH₂CH₂Cl | Me |

**[Table 1121]**

| Compound Number | R³ | R⁴ | R⁵ | R¹¹ |
|---|---|---|---|---|
| G-0039 | Cl | F | CH₂CH₂CH(Me)CH₂CH₂Br | Me |
| G-0040 | Cl | F | CH₂CH₂CF=CF₂ | Me |
| G-0041 | Cl | F | CH₂CH=C(Cl)CF₃ | Me |
| G-0042 | Cl | F | CH₂CH₂CH₂CF=CF₂ | Me |
| G-0043 | Cl | F | CH₂CH₂CH₂CH₂CF=CF₂ | Me |
| G-0044 | Cl | F | CH₂CH₂CH₂CH₂CH₂CF=CF₂ | Me |
| G-0045 | Cl | F | CH₂(*t*-Bu) | Me |
| G-0046 | Cl | F | CH₂CH₂(*t*-Bu) | Me |
| G-0047 | Cl | F | CH₂CH₂CH₂(*t*-Bu) | Me |
| G-0048 | Cl | F | CH₂CH₂CH₂CH₂(*t*-Bu) | Me |
| G-0049 | Cl | F | CH₂CH₂CH₂CH₂CH₂(*t*-Bu) | Me |
| G-0050 | Cl | F | CH₂CH₂CH₂CH₂CH₂CH₂(*t*-Bu) | Me |
| G-0051 | Cl | F | CH₂CF₃ | Me |
| G-0052 | Cl | F | CH₂CH₂CF₃ | Me |
| G-0053 | Cl | F | CH₂CH₂CH₂CF₃ | Me |
| G-0054 | Cl | F | CH₂CH₂CH₂CH₂CF₃ | Me |
| G-0055 | Cl | F | CH₂CH₂CH₂CH₂CH₂CF₃ | Me |
| G-0056 | Cl | F | CH₂CH₂CH₂CH₂CH₂CH₂CF₃ | Me |
| G-0057 | Cl | F | CH₂CH₂CH₂CH₂CH₂CH₂CH₂CF₃ | Me |
| G-0058 | Cl | F | CH₂CF₂CF₃ | Me |
| G-0059 | Cl | F | CF₂CHFCF₃ | Me |
| G-0060 | Cl | F | CF₂CF₂CF₃ | Me |
| G-0061 | Cl | F | CH₂CF₂CF₂CF₃ | Me |
| G-0062 | Cl | F | CH₂CF₂CF₂CF₂CF₃ | Me |
| G-0063 | Cl | F | CH₂CF₂CF₂CF₂CHF₂ | Me |
| G-0064 | Cl | F | CH(CF₃)CF₃ | Me |
| G-0065 | Cl | F | CH₂CF(CF₃)CF₃ | Me |
| G-0066 | Cl | F | CH₂CF₂CF(CF₃)CF₃ | Me |
| G-0067 | Cl | F | CH₂CF(CF₃)CF₂CF₃ | Me |
| G-0068 | Cl | F | CH₂CF₂CF₂CF(CF₃)CF₃ | Me |
| G-0069 | Cl | F | CH₂CF₂CF(CF₃)CF₂CF₃ | Me |
| G-0070 | Cl | F | CH₂CF(CF₃)CF₂CF₂CF₃ | Me |
| G-0071 | Cl | F | CH₂(*c*-Pr) | Me |
| G-0072 | Cl | F | CH₂[*c*-Pr(1-Me)] | Me |
| G-0073 | Cl | F | CH₂[*c*-Pr(1-Ph)] | Me |
| G-0074 | Cl | F | CH₂[*c*-Pr(1-NH₂)] | Me |
| G-0075 | Cl | F | CH₂[*c*-Pr(1-NHC(=O)O(*t*-Bu)] | Me |
| G-0076 | Cl | F | CH₂{*c*-Pr[1-NHS(=O)₂CF₃]} | Me |
| G-0077 | Cl | F | CH₂{*c*-Pr[1-Ph(4-CF₃)]} | Me |
| G-0078 | Cl | F | CH₂{*c*-Pr[1-Ph(3,4,5-F₃)]} | Me |
| G-0079 | Cl | F | CH₂{*c*-Hex[4-(*t*-Bu)]} | Me |
| G-0080 | Cl | F | CH₂[*c*-Hex(4-CF₃)] | Me |
| G-0081 | Cl | F | CH₂CH₂(*c*-Pr) | Me |
| G-0082 | Cl | F | CH₂CH₂(*c*-Hex) | Me |

**[Table 1122]**

| Compound Number | R³ | R⁴ | R⁵ | R¹¹ |
|---|---|---|---|---|
| G-0083 | Cl | F | CH₂CH₂[*c*-Hex(4-CF₃)] | Me |
| G-0084 | Cl | F | CH₂CH₂[*c*-Hex(4-SCF₃)] | Me |
| G-0085 | Cl | F | CH₂CH₂CH₂(*c*-Pr) | Me |
| G-0086 | Cl | F | CH₂CH₂CH₂(*c*-Hex) | Me |
| G-0087 | Cl | F | CH₂CH₂CH₂{*c*-Hex[4-(*t*-Bu)]} | Me |
| G-0088 | Cl | F | CH₂CH₂CH₂[*c*-Hex(4-CF₃)] | Me |
| G-0089 | Cl | F | CH₂CH₂CH₂[*c*-Hex(4-SCF₃)] | Me |
| G-0090 | Cl | F | CH₂CH₂CH₂CH₂(*c*-Pr) | Me |
| G-0091 | Cl | F | CH₂CH₂CH₂CH₂(*c*-Hex) | Me |
| G-0092 | Cl | F | CH₂CH₂CH₂CH₂[*c*-Hex(4-CF₃)] | Me |
| G-0093 | Cl | F | CH₂[*c*-Pr(2,2-F₂)] | Me |
| G-0094 | Cl | F | CH₂[*c*-Hex(4,4-F₂)] | Me |
| G-0095 | Cl | F | CH₂CH₂[*c*-Pr(2,2-F₂)] | Me |
| G-0096 | Cl | F | CH₂CH₂[*c*-Hex(4,4-F₂) | Me |
| G-0097 | Cl | F | CH₂CH₂CH₂[*c*-Pr(2,2-F₂)] | Me |
| G-0098 | Cl | F | CH₂CH₂CH₂CH₂[*c*-Pr(2,2-F₂)] | Me |
| G-0099 | Cl | F | CH₂CH₂OH | Me |
| G-0100 | Cl | F | CH₂CH₂CH₂OH | Me |
| G-0101 | Cl | F | CH₂CH₂CH₂CH₂OH | Me |
| G-0102 | Cl | F | CH₂CH₂CH₂CH₂CH₂OH | Me |
| G-0103 | Cl | F | CH₂CH₂CH₂CH₂CH₂CH₂OH | Me |
| G-0104 | Cl | F | CH₂C(=O)Me | Me |
| G-0105 | Cl | F | CH₂CH(OH)Me | Me |
| G-0106 | Cl | F | CH₂CH(OH)CF₃ | Me |
| G-0107 | Cl | F | CH₂C(OH)₂CF₃ | Me |
| G-0108 | Cl | F | CH₂C(CF₃)=NOH | Me |
| G-0109 | Cl | F | CH₂C(CF₃)=NOMe | Me |
| G-0110 | Cl | F | CH₂CH(CF₃)NH₂ | Me |
| G-0111 | Cl | F | CH₂CH(CF₃)NHSO₂CF₃ | Me |
| G-0112 | Cl | F | CH₂CH₂OMe | Me |
| G-0113 | Cl | F | CH₂CH₂CH₂OMe | Me |
| G-0114 | Cl | F | CH₂CH₂CH₂CH₂OMe | Me |
| G-0115 | Cl | F | CH₂CH₂CH₂CH₂CH₂OMe | Me |
| G-0116 | Cl | F | CH₂CH₂CH₂CH₂CH₂CH₂OMe | Me |
| G-0117 | Cl | F | CH₂CH₂OCHF₂ | Me |
| G-0118 | Cl | F | CH₂CH₂CH₂OCHF₂ | Me |
| G-0119 | Cl | F | CH₂CH₂CH₂CH₂OCHF₂ | Me |
| G-0120 | Cl | F | CH₂CH₂CH₂CH₂CH₂OCHF₂ | Me |
| G-0121 | Cl | F | CH₂CH₂CH₂CH₂CH₂CH₂OCHF₂ | Me |
| G-0122 | Cl | F | CH₂CH₂OCH₂CF₃ | Me |
| G-0123 | Cl | F | CH₂CH₂CH₂OCH₂CF₃ | Me |
| G-0124 | Cl | F | CH₂CH₂CH₂CH₂OCH₂CF₃ | Me |
| G-0125 | Cl | F | CH₂CH₂CH₂CH₂CH₂OCH₂CF₃ | Me |
| G-0126 | Cl | F | CH₂CH₂CH₂CH₂CH₂CH₂OCH₂CF₃ | Me |

**[Table 1123]**

| Compound Number | R³ | R⁴ | R⁵ | R¹¹ |
|---|---|---|---|---|
| G-0127 | Cl | F | CH₂CH₂CH₂CH₂CH₂CH₂CH₂OCH₂CF₃ | Me |
| G-0128 | Cl | F | CH₂CH₂OC(CF₃)₃ | Me |
| G-0129 | Cl | F | CH₂CH₂CH₂OC(CF₃)₃ | Me |
| G-0130 | Cl | F | CH₂CH₂CH₂CH₂OC(CF₃)₃ | Me |
| G-0131 | Cl | F | CH₂CH₂CH₂CH₂CH₂OC(CF₃)₃ | Me |
| G-0132 | Cl | F | CF₂CHFOCF₂CF₂CF₃ | Me |
| G-0133 | Cl | F | CH₂CH₂O(*c* -Pr) | Me |
| G-0134 | Cl | F | CH₂CH₂O(*c* -Pen) | Me |
| G-0135 | Cl | F | CH₂CH₂O(*c* -Hex) | Me |
| G-0136 | Cl | F | CH₂CH₂CH₂O(*c* -Pr) | Me |
| G-0137 | Cl | F | CH₂CH₂CH₂O(*c* -Pen) | Me |
| G-0138 | Cl | F | CH₂CH₂CH₂O(*c* -Hex) | Me |
| G-0139 | Cl | F | CH₂CH₂CH₂CH₂O(*c* -Pr) | Me |
| G-0140 | Cl | F | CH₂CH₂CH₂CH₂O(*c* -Pen) | Me |
| G-0141 | Cl | F | CH₂CH₂CH₂CH₂O(*c* -Hex) | Me |
| G-0142 | Cl | F | CH₂CH₂O[*c*-Pr(2,2-F₂)] | Me |
| G-0143 | Cl | F | CH₂CH₂CH₂O[*c*-Pr(2,2-F₂)] | Me |
| G-0144 | Cl | F | CH₂CH₂CH₂CH₂O[*c*-Pr(2,2-F₂)] | Me |
| G-0145 | Cl | F | CH₂CH₂OCH₂CH₂OCH₃ | Me |
| G-0146 | Cl | F | CH₂CH₂OCH₂CH₂OCH₂CF₃ | Me |
| G-0147 | Cl | F | CH₂CF₂OCF₂CF₂OCF₃ | Me |
| G-0148 | Cl | F | CF₂CHFOCF₂CF₂OCF₃ | Me |
| G-0149 | Cl | F | CF₂CHFOCF₂CF(CF₃)OCF₂CF₂CF₃ | Me |
| G-0150 | Cl | F | CH₂CH₂SH | Me |
| G-0151 | Cl | F | CH₂CH₂CH₂SH | Me |
| G-0152 | Cl | F | CH₂CH₂CH₂CH₂SH | Me |
| G-0153 | Cl | F | CH₂CH₂CH₂CH₂CH₂SH | Me |
| G-0154 | Cl | F | CH₂CH₂CH₂CH₂CH₂CH₂SH | Me |
| G-0155 | Cl | F | CH₂CH₂S(*t*-Bu) | Me |
| G-0156 | Cl | F | CH₂CH₂CH₂S(*t*-Bu) | Me |
| G-0157 | Cl | F | CH₂CH₂CH₂CH₂S(*t*-Bu) | Me |
| G-0158 | Cl | F | CH₂CH₂CH₂CH₂CH₂SCH₃ | Me |
| G-0159 | Cl | F | CH₂CH₂CH₂CH₂CH₂SCH(CH₃)₂ | Me |
| G-0160 | Cl | F | CH₂CH₂CH₂CH₂CH₂S(*t*-Bu) | Me |
| G-0161 | Cl | F | CH₂CH₂CH₂CH₂CH₂CH₂SCH(CH₃)₂ | Me |
| G-0162 | Cl | F | CH₂CH₂CH₂CH₂CH₂CH₂S(*t*-Bu) | Me |
| G-0163 | Cl | F | CH₂CH₂CH₂CH₂CH₂CH₂CH₂S(*t*-Bu) | Me |
| G-0164 | Cl | F | CH₂CH₂SCHF₂ | Me |
| G-0165 | Cl | F | CH₂CH₂CH₂SCHF₂ | Me |
| G-0166 | Cl | F | CH₂CH₂CH₂CH₂SCHF₂ | Me |
| G-0167 | Cl | F | CH₂CH₂CH₂CH₂CH₂SCHF₂ | Me |
| G-0168 | Cl | F | CH₂CH₂CH₂CH₂CH₂CH₂SCHF₂ | Me |
| G-0169 | Cl | F | CH₂CH₂CH₂CH₂CH₂CH₂CH₂SCHF₂ | Me |
| G-0170 | Cl | F | CH₂CH₂SCF₃ | Me |

**[Table 1124]**

| Compound Number | R³ | R⁴ | R⁵ | R¹¹ |
|---|---|---|---|---|
| G-0171 | Cl | F | CH₂CH₂CH₂SCF₃ | Me |
| G-0172 | Cl | F | CH₂CH₂CH₂CH₂SCF₃ | Me |
| G-0173 | Cl | F | CH₂CH₂CH₂CH₂CH₂SCF₃ | Me |
| G-0174 | Cl | F | CH₂CH₂CH₂CH₂CH₂CH₂SCF₃ | Me |
| G-0175 | Cl | F | CH₂CH₂CH₂CH₂CH₂CH₂CH₂SCF₃ | Me |
| G-0176 | Cl | F | CH₂CH₂CH₂CH₂CH₂CH₂CH₂CH₂SCF₃ | Me |
| G-0177 | Cl | F | CH₂CH(Me)SCF₃ | Me |
| G-0178 | Cl | F | CH(Me)CH₂SCF₃ | Me |
| G-0179 | Cl | F | CH₂CH₂CH(Me)SCF₃ | Me |
| G-0180 | Cl | F | CH₂CH(Me)CH₂SCF₃ | Me |
| G-0181 | Cl | F | CH(Me)CH₂CH₂SCF₃ | Me |
| G-0182 | Cl | F | CH₂CH₂CH₂CH(Me)SCF₃ | Me |
| G-0183 | Cl | F | CH₂CH₂CH(Me)CH₂SCF₃ | Me |
| G-0184 | Cl | F | CH₂CH(Me)CH₂CH₂SCF₃ | Me |
| G-0185 | Cl | F | CH(Me)CH₂CH₂CH₂SCF₃ | Me |
| G-0186 | Cl | F | CH₂CH₂SCH₂CF₃ | Me |
| G-0187 | Cl | F | CH₂CH₂CH₂SCH₂CF₃ | Me |
| G-0188 | Cl | F | CH₂CH₂CH₂CH₂SCH₂CF₃ | Me |
| G-0189 | Cl | F | CH₂CH₂CH₂CH₂CH₂SCH₂CF₃ | Me |
| G-0190 | Cl | F | CH₂CH₂CH₂CH₂CH₂CH₂SCH₂CF₃ | Me |
| G-0191 | Cl | F | CH₂CH₂CH₂CH₂CH₂CH₂CH₂SCH₂CF₃ | Me |
| G-0192 | Cl | F | CH₂CH₂SCH(CF₃)₂ | Me |
| G-0193 | Cl | F | CH₂CH₂CH₂SCH(CF₃)₂ | Me |
| G-0194 | Cl | F | CH₂CH₂CH₂CH₂SCH(CF₃)₂ | Me |
| G-0195 | Cl | F | CH₂CH₂CH₂CH₂CH₂SCH(CF₃)₂ | Me |
| G-0196 | Cl | F | CH₂CH₂CH₂CH₂CH₂CH₂SCH(CF₃)₂ | Me |
| G-0197 | Cl | F | CH₂CH₂CH₂CH₂CH₂SCF₂CF₃ | Me |
| G-0198 | Cl | F | CH₂CH₂CH₂CH₂CH₂CH₂SCF₂CF₃ | Me |
| G-0199 | Cl | F | CH₂CH₂CH₂CH₂CH₂CH₂SCF(CF₃)₂ | Me |
| G-0200 | Cl | F | CH₂CH₂CH₂CH₂CH₂CH₂CH₂SCF(CF₃)₂ | Me |
| G-0201 | Cl | F | CH₂CH₂CH₂CH₂CH₂SCCl₃ | Me |
| G-0202 | Cl | F | CH₂CH₂CH₂CH₂CH₂CH₂SCCl₃ | Me |
| G-0203 | Cl | F | CH₂CH₂SCH₂CH=CH₂ | Me |
| G-0204 | Cl | F | CH₂CH₂CH₂SCH₂CH=CH₂ | Me |
| G-0205 | Cl | F | CH₂CH₂CH₂CH₂SCH₂CH=CH₂ | Me |
| G-0206 | Cl | F | CH₂CH₂SCF=CFCF₃ | Me |
| G-0207 | Cl | F | CH₂CH₂CH₂SCF=CFCF₃ | Me |
| G-0208 | Cl | F | CH₂CH₂CH₂CH₂SCF=CFCF₃ | Me |
| G-0209 | Cl | F | CH₂CH₂CH₂S(*c* -Pr) | Me |
| G-0210 | Cl | F | CH₂CH₂CH₂S(*c* -Hex) | Me |
| G-0211 | Cl | F | CH₂CH₂CH₂CH₂S(*c* -Pr) | Me |
| G-0212 | Cl | F | CH₂CH₂CH₂CH₂S(*c* -Hex) | Me |
| G-0213 | Cl | F | CH₂CH₂CH₂CH₂CH₂S(*c* -Pr) | Me |
| G-0214 | Cl | F | CH₂CH₂CH₂CH₂CH₂S(*c* -Hex) | Me |

**[Table 1125]**

| Compound Number | R³ | R⁴ | R⁵ | R¹¹ |
|---|---|---|---|---|
| G-0215 | Cl | F | CH₂CH₂CH₂S[*c*-Hex(4,4-F₂)] | Me |
| G-0216 | Cl | F | CH₂CH₂CH₂CH₂S[*c*-Hex(4,4-F₂)] | Me |
| G-0217 | Cl | F | CH₂CH₂CH₂CH₂CH₂S[*c* -Hex(4,4-F₂)] | Me |
| G-0218 | Cl | F | CH₂CH₂CH₂SCH₂(*c* -Pr) | Me |
| G-0219 | Cl | F | CH₂CH₂CH₂SCH₂(*c* -Hex) | Me |
| G-0220 | Cl | F | CH₂CH₂CH₂CH₂SCH₂(*c* -Pr) | Me |
| G-0221 | Cl | F | CH₂CH₂CH₂CH₂SCH₂(*c* -Hex) | Me |
| G-0222 | Cl | F | CH₂CH₂CH₂CH₂CH₂SCH₂(*c* -Pr) | Me |
| G-0223 | Cl | F | CH₂CH₂CH₂CH₂CH₂SCH₂(*c* -Hex) | Me |
| G-0224 | Cl | F | CH₂CH₂CH₂SCH₂[*c* -Hex(4,4-F₂)] | Me |
| G-0225 | Cl | F | CH₂CH₂CH₂CH₂SCH₂[*c* -Hex(4,4-F₂)] | Me |
| G-0226 | Cl | F | CH₂CH₂CH₂CH₂CH₂SCH₂[*c* -Hex(4,4-F₂)] | Me |
| G-0227 | Cl | F | CH₂CH₂CH₂SCH₂SiMe₃ | Me |
| G-0228 | Cl | F | CH₂CH₂CH₂CH₂SCH₂SiMe₃ | Me |
| G-0229 | Cl | F | CH₂CH₂CH₂CH₂CH₂SCH₂SiMe₃ | Me |
| G-0230 | Cl | F | CH₂CH₂OCH₂CH₂SCH₃ | Me |
| G-0231 | Cl | F | CH₂CH₂OCH₂CH₂SCHF₂ | Me |
| G-0232 | Cl | F | CH₂CH₂OCH₂CH₂SCF₃ | Me |
| G-0233 | Cl | F | CH₂CH₂OCH₂CH₂SCH₂CF₃ | Me |
| G-0234 | Cl | F | CH₂CH₂CH₂S(=O)(*t*-Bu) | Me |
| G-0235 | Cl | F | CH₂CH₂CH₂CH₂S(=O)(*t*-Bu) | Me |
| G-0236 | Cl | F | CH₂CH₂CH₂CH₂CH₂CH₂S(=O)(*t*-Bu) | Me |
| G-0237 | Cl | F | CH₂CH₂S(=O)CHF₂ | Me |
| G-0238 | Cl | F | CH₂CH₂CH₂S(=O)CHF₂ | Me |
| G-0239 | Cl | F | CH₂CH₂CH₂CH₂S(=O)CHF₂ | Me |
| G-0240 | Cl | F | CH₂CH₂CH₂CH₂CH₂S(=O)CHF₂ | Me |
| G-0241 | Cl | F | CH₂CH₂CH₂CH₂CH₂CH₂S(=O)CHF₂ | Me |
| G-0242 | Cl | F | CH₂CH₂CH₂CH₂CH₂CH₂CH₂S(=O)CHF₂ | Me |
| G-0243 | Cl | F | CH₂CH₂S(=O)CF₃ | Me |
| G-0244 | Cl | F | CH₂CH₂CH₂S(=O)CF₃ | Me |
| G-0245 | Cl | F | CH₂CH₂CH₂CH₂S(=O)CF₃ | Me |
| G-0246 | Cl | F | CH₂CH₂CH₂CH₂CH₂S(=O)CF₃ | Me |
| G-0247 | Cl | F | CH₂CH₂CH₂CH₂CH₂CH₂S(=O)CF₃ | Me |
| G-0248 | Cl | F | CH₂CH₂CH₂CH₂CH₂CH₂CH₂S(=O)CF₃ | Me |
| G-0249 | Cl | F | CH₂CH₂S(=O)CH₂CF₃ | Me |
| G-0250 | Cl | F | CH₂CH₂CH₂S(=O)CH₂CF₃ | Me |
| G-0251 | Cl | F | CH₂CH₂CH₂CH₂S(=O)CH₂CF₃ | Me |
| G-0252 | Cl | F | CH₂CH₂CH₂CH₂CH₂S(=O)CH₂CF₃ | Me |
| G-0253 | Cl | F | CH₂CH₂CH₂CH₂CH₂CH₂S(=O)CH₂CF₃ | Me |
| G-0254 | Cl | F | CH₂CH₂CH₂CH₂CH₂CH₂CH₂S(=O)CH₂CF₃ | Me |
| G-0255 | Cl | F | CH₂CH₂CH₂S(=O)(*c* -Pr) | Me |
| G-0256 | Cl | F | CH₂CH₂CH₂CH₂S(=O)(*c* -Pr) | Me |
| G-0257 | Cl | F | CH₂CH₂CH₂CH₂S(=O)(*c* -Pen) | Me |
| G-0258 | Cl | F | CH₂CH₂CH₂CH₂S(=O)(*c* -Hex) | Me |

**[Table 1126]**

| Compound Number | R³ | R⁴ | R⁵ | R¹¹ |
|---|---|---|---|---|
| G-0259 | Cl | F | CH₂CH₂CH₂CH₂CH₂S(=O)(*c*-Pr) | Me |
| G-0260 | Cl | F | CH₂CH₂CH₂CH₂CH₂S(=O)(*c*-Hex) | Me |
| G-0261 | Cl | F | CH₂CH₂CH₂S(=O)[*c*-Hex(4,4-F₂)] | Me |
| G-0262 | Cl | F | CH₂CH₂CH₂CH₂S(=O)[*c*-Hex(4,4-F₂)] | Me |
| G-0263 | Cl | F | CH₂CH₂CH₂CH₂CH₂S(=O)[*c*-Hex(4,4-F₂)] | Me |
| G-0264 | Cl | F | CH₂CH₂CH₂CH₂S(=O)₂Me | Me |
| G-0265 | Cl | F | CH₂CH₂CH₂CH₂CH₂S(=O)₂Me | Me |
| G-0266 | Cl | F | CH₂CH₂CH₂CH₂CH₂S(=O)₂(*t*-Bu) | Me |
| G-0267 | Cl | F | CH₂CH₂S(=O)₂CHF₂ | Me |
| G-0268 | Cl | F | CH₂CH₂CH₂S(=O)₂CHF₂ | Me |
| G-0269 | Cl | F | CH₂CH₂CH₂CH₂S(=O)₂CHF₂ | Me |
| G-0270 | Cl | F | CH₂CH₂CH₂CH₂CH₂S(=O)₂CHF₂ | Me |
| G-0271 | Cl | F | CH₂CH₂CH₂CH₂CH₂CH₂S(=O)₂CHF₂ | Me |
| G-0272 | Cl | F | CH₂CH₂S(=O)₂CF₃ | Me |
| G-0273 | Cl | F | CH₂CH₂CH₂S(=O)₂CF₃ | Me |
| G-0274 | Cl | F | CH₂CH₂CH₂CH₂S(=O)₂CF₃ | Me |
| G-0275 | Cl | F | CH₂CH₂CH₂CH₂CH₂S(=O)₂CF₃ | Me |
| G-0276 | Cl | F | CH₂CH₂CH₂CH₂CH₂CH₂S(=O)₂CF₃ | Me |
| G-0277 | Cl | F | CH₂CH₂CH₂CH₂CH₂CH₂CH₂S(=O)₂CF₃ | Me |
| G-0278 | Cl | F | CH₂CH₂CH₂CH₂CH₂CH₂CH₂CH₂S(=O)₂CF₃ | Me |
| G-0279 | Cl | F | CH₂CH₂S(=O)₂CH₂CF₃ | Me |
| G-0280 | Cl | F | CH₂CH₂CH₂S(=O)₂CH₂CF₃ | Me |
| G-0281 | Cl | F | CH₂CH₂CH₂CH₂S(=O)₂CH₂CF₃ | Me |
| G-0282 | Cl | F | CH₂CH₂CH₂CH₂CH₂S(=O)₂CH₂CF₃ | Me |
| G-0283 | Cl | F | CH₂CH₂CH₂CH₂CH₂CH₂S(=O)₂CH₂CF₃ | Me |
| G-0284 | Cl | F | CH₂CH₂CH₂S(=O)₂(*c*-Pr) | Me |
| G-0285 | Cl | F | CH₂CH₂CH₂CH₂S(=O)₂(*c*-Pr) | Me |
| G-0286 | Cl | F | CH₂CH₂CH₂CH₂CH₂S(=O)₂(*c*-Pr) | Me |
| G-0287 | Cl | F | CH₂CH₂CH₂S(=O)₂[*c*-Hex(4,4-F₂)] | Me |
| G-0288 | Cl | F | CH₂CH₂CH₂CH₂S(=O)₂[*c*-Hex(4,4-F₂)] | Me |
| G-0289 | Cl | F | CH₂CH₂CH₂CH₂CH₂S(=O)₂[*c*-Hex(4,4-F₂)] | Me |
| G-0290 | Cl | F | CH₂CH₂CH₂C(=O)H | Me |
| G-0291 | Cl | F | CH₂CH₂CH₂CH₂C(=O)H | Me |
| G-0292 | Cl | F | CH₂CH₂CH₂CH₂CH₂C(=O)H | Me |
| G-0293 | Cl | F | CH₂CH₂CH₂C(=O)(*t*-Bu) | Me |
| G-0294 | Cl | F | CH₂CH₂CH₂CH₂C(=O)(*t*-Bu) | Me |
| G-0295 | Cl | F | CH₂CH₂CH₂CH₂CH₂C(=O)(*t*-Bu) | Me |
| G-0296 | Cl | F | CH₂CH₂CH₂C(=O)CF₃ | Me |
| G-0297 | Cl | F | CH₂CH₂CH₂CH₂C(=O)CF₃ | Me |
| G-0298 | Cl | F | CH₂CH₂CH₂CH₂CH₂C(=O)CF₃ | Me |
| G-0299 | Cl | F | CH₂C(=O)Ph | Me |
| G-0300 | Cl | F | CH₂C(=O)Ph(4-Cl) | Me |
| G-0301 | Cl | F | CH₂C(=O)Ph(4-CF₃) | Me |
| G-0302 | Cl | F | CH₂CH₂C(=O)Ph(4-CF₃) | Me |

**[Table 1127]**

| Compound Number | R³ | R⁴ | R⁵ | R¹¹ |
|---|---|---|---|---|
| G-0303 | Cl | F | CH₂CH₂CH₂C(=O)Ph(4-CF₃) | Me |
| G-0304 | Cl | F | CH₂CH₂CH₂CH₂C(=O)Ph(4-CF₃) | Me |
| G-0305 | Cl | F | CH₂CH₂CH₂CH₂CH₂C(=O)Ph(4-CF₃) | Me |
| G-0306 | Cl | F | CH₂C(=O)OEt | Me |
| G-0307 | Cl | F | CH₂CH₂C(=O)O(*t*-Bu) | Me |
| G-0308 | Cl | F | CH₂CH₂CH₂C(=O)OEt | Me |
| G-0309 | Cl | F | CH₂CH₂CH₂C(=O)O(*t*-Bu) | Me |
| G-0310 | Cl | F | CH₂CH₂CH₂CH₂C(=O)O(*t*-Bu) | Me |
| G-0311 | Cl | F | CH₂CH₂CH₂CH₂CH₂C(=O)O(*t-*Bu) | Me |
| G-0312 | Cl | F | CH₂CH₂CH₂CH₂CH₂CH₂C(=O)O(*t*-Bu) | Me |
| G-0313 | Cl | F | CH₂CH₂CH₂CH₂CH₂CH₂CH₂C(=O)OEt | Me |
| G-0314 | Cl | F | CH₂CH₂C(=O)OCH₂CF₃ | Me |
| G-0315 | Cl | F | CH₂CH₂CH₂C(=O)OCH₂CF₃ | Me |
| G-0316 | Cl | F | CH₂CH₂CH₂CH₂C(=O)OCH₂CF₃ | Me |
| G-0317 | Cl | F | CH₂CH₂CH₂CH₂CH₂C(=O)OCH₂CF₃ | Me |
| G-0318 | Cl | F | CH₂CH₂C(=O)NH(*t*-Bu) | Me |
| G-0319 | Cl | F | CH₂CH₂CH₂C(=O)NH(*t*-Bu) | Me |
| G-0320 | Cl | F | CH₂CH₂CH₂C(=O)NH(*t*-Pen) | Me |
| G-0321 | Cl | F | CH₂CH₂CH₂CH₂C(=O)NH(*t*-Bu) | Me |
| G-0322 | Cl | F | CH₂CH₂C(=O)NHCH₂CF₃ | Me |
| G-0323 | Cl | F | CH₂CH₂CH₂C(=O)NHCH₂CF₃ | Me |
| G-0324 | Cl | F | CH₂CH₂CH₂CH₂C(=O)NHCH₂CF₃ | Me |
| G-0325 | Cl | F | CH₂CH₂SC(=O)N(Me)₂ | Me |
| G-0326 | Cl | F | CH₂CH₂CH₂SC(=O)N(Me)₂ | Me |
| G-0327 | Cl | F | CH₂CH₂CH₂SC(=O)NH(*t*-Bu) | Me |
| G-0328 | Cl | F | CH₂CH₂CH₂CH₂CH₂SC(=O)NH(*t*-Bu) | Me |
| G-0329 | Cl | F | CH₂CH₂SC(=O)NHCH₂CF₃ | Me |
| G-0330 | Cl | F | CH₂CH₂CH₂SC(=O)NHCH₂CF₃ | Me |
| G-0331 | Cl | F | CH₂CH₂CH₂CH₂SC(=O)NHCH₂CF₃ | Me |
| G-0332 | Cl | F | CH₂CH₂CH₂OC(=O)H | Me |
| G-0333 | Cl | F | CH₂CH₂CH₂CH₂OC(=O)H | Me |
| G-0334 | Cl | F | CH₂CH₂CH₂CH₂CH₂OC(=O)H | Me |
| G-0335 | Cl | F | CH₂CH₂CH₂OC(=O)Me | Me |
| G-0336 | Cl | F | CH₂CH₂CH₂CH₂OC(=O)Me | Me |
| G-0337 | Cl | F | CH₂CH₂CH₂CH₂CH₂OC(=O)Me | Me |
| G-0338 | Cl | F | CH₂CH₂OC(=O)CF₃ | Me |
| G-0339 | Cl | F | CH₂CH₂CH₂OC(=O)CF₃ | Me |
| G-0340 | Cl | F | CH₂CH₂CH₂CH₂OC(=O)CF₃ | Me |
| G-0341 | Cl | F | CH₂CH₂CH₂CH₂CH₂OC(=O)Ph(4-CF₃) | Me |
| G-0342 | Cl | F | CH₂CH₂OC(=O)Ph | Me |
| G-0343 | Cl | F | CH₂CH₂OC(=O)Ph(4-CF₃) | Me |
| G-0344 | Cl | F | CH₂CH₂CH₂OC(=O)Ph(4-CF₃) | Me |
| G-0345 | Cl | F | CH₂CH₂CH₂CH₂OC(=O)Ph(4-CF₃) | Me |
| G-0346 | Cl | F | CH₂CH₂CH₂CH₂CH₂OC(=O)Ph(4-CF₃) | Me |

**[Table 1128]**

| Compound Number | R³ | R⁴ | R⁵ | R¹¹ |
|---|---|---|---|---|
| G-0347 | Cl | F | CH₂CH₂OS(=O)Me | Me |
| G-0348 | Cl | F | CH₂CH₂CH₂OS(=O)Me | Me |
| G-0349 | Cl | F | CH₂CH₂CH₂CH₂OS(=O)Me | Me |
| G-0350 | Cl | F | CH₂CH₂CH₂CH₂CH₂OS(=O)Me | Me |
| G-0351 | Cl | F | CH₂CH₂CH₂CH₂CH₂CH₂OS(=O)Me | Me |
| G-0352 | Cl | F | CH₂CH₂OS(=O)CF₃ | Me |
| G-0353 | Cl | F | CH₂CH₂CH₂OS(=O)CF₃ | Me |
| G-0354 | Cl | F | CH₂CH₂CH₂CH₂OS(=O)CF₃ | Me |
| G-0355 | Cl | F | CH₂CH₂CH₂CH₂CH₂OS(=O)CF₃ | Me |
| G-0356 | Cl | F | CH₂CH₂CH₂CH₂CH₂CH₂OS(=O)CF₃ | Me |
| G-0357 | Cl | F | CH₂CH₂OS(=O)Ph | Me |
| G-0358 | Cl | F | CH₂CH₂OS(=O)Ph(4-Me) | Me |
| G-0359 | Cl | F | CH₂CH₂CH₂OS(=O)Ph | Me |
| G-0360 | Cl | F | CH₂CH₂CH₂OS(=O)Ph(4-Me) | Me |
| G-0361 | Cl | F | CH₂CH₂OS(=O)₂Me | Me |
| G-0362 | Cl | F | CH₂CH₂CH₂OS(=O)₂Me | Me |
| G-0363 | Cl | F | CH₂CH₂CH₂OS(=O)₂CF₃ | Me |
| G-0364 | Cl | F | CH₂CH₂CH₂CH₂OS(=O)₂CF₃ | Me |
| G-0365 | Cl | F | CH₂CH₂CH₂CH₂CH₂OS(=O)₂CF₃ | Me |
| G-0366 | Cl | F | CH₂CH₂CH₂CH₂CH₂OS(=O)₂CF₂CF₂CF₂CF₃ | Me |
| G-0367 | Cl | F | CH₂CH₂OS(=O)₂Ph | Me |
| G-0368 | Cl | F | CH₂CH₂OS(=O)₂Ph(4-Me) | Me |
| G-0369 | Cl | F | CH₂CH₂CH₂OS(=O)₂Ph | Me |
| G-0370 | Cl | F | CH₂CH₂CH₂OS(=O)₂Ph(4-Me) | Me |
| G-0371 | Cl | F | CH₂CH₂NH₂ | Me |
| G-0372 | Cl | F | CH₂CH₂NH₂, HCl | Me |
| G-0373 | Cl | F | CH₂CH₂CH₂NH₂ | Me |
| G-0374 | Cl | F | CH₂CH₂CH₂CH₂NH₂ | Me |
| G-0375 | Cl | F | CH₂CH₂CH₂CH₂CH₂NH₂ | Me |
| G-0376 | Cl | F | CH₂CH₂CH₂CH₂CH₂CH₂NH₂ | Me |
| G-0377 | Cl | F | CH₂CH₂CH₂CH₂CH₂CH₂CH₂NH₂ | Me |
| G-0378 | Cl | F | CH₂CH(Me)NH₂ | Me |
| G-0379 | Cl | F | CH₂C*H(Me)NH₂,HCl | Me |
| G-0380 | Cl | F | CH₂C*H(Me)NH₂: (R) | Me |
| G-0381 | Cl | F | CH₂C*H(Me)NH₂,HCl: (R) | Me |
| G-0382 | Cl | F | CH₂C*H(Me)NH₂: (S) | Me |
| G-0383 | Cl | F | CH₂C*H(Me)NH₂,HCl: (S) | Me |
| G-0384 | Cl | F | CH₂CH(Et)NH₂ | Me |
| G-0385 | Cl | F | CH₂CH(*i*-Pr)NH₂ | Me |
| G-0386 | Cl | F | CH(Me)CH₂NH₂ | Me |
| G-0387 | Cl | F | CH(Me)CH₂NH₂, HCl | Me |
| G-0388 | Cl | F | CH(Et)CH₂NH₂ | Me |
| G-0389 | Cl | F | CH(*i*-Pr)CH₂NH₂ | Me |
| G-0390 | Cl | F | CH₂CH₂CH(Me)NH₂ | Me |

**[Table 1129]**

| Compound Number | R³ | R⁴ | R⁵ | R¹¹ |
|---|---|---|---|---|
| G-0391 | Cl | F | CH₂CH(Me)CH₂NH₂ | Me |
| G-0392 | Cl | F | CH(Me)CH₂CH₂NH₂ | Me |
| G-0393 | Cl | F | CH₂CH(Me)NH(*c*-Pr) | Me |
| G-0394 | Cl | F | CH₂CH(Me)N(*c*-Pr)S(=O)₂CF₃ | Me |
| G-0395 | Cl | F | CH₂CH(Me)NHOMe | Me |
| G-0396 | Cl | F | CH₂CH(Me)N(OMe)S(=O)₂CF₃ | Me |
| G-0397 | Cl | F | CH₂CH(Me)N(OMe)S(=O)₂Ph(2-NO₂) | Me |
| G-0398 | Cl | F | CH₂CH(Me)N(OMe)S(=O)₂Ph(4-NO₂) | Me |
| G-0399 | Cl | F | CH₂CH₂N(Me)(*t*-Bu) | Me |
| G-0400 | Cl | F | CH₂CH₂CH₂N(Me)(*t*-Bu) | Me |
| G-0401 | Cl | F | CH₂CH₂CH₂CH₂N(Me)(*t*-Bu) | Me |
| G-0402 | Cl | F | CH₂CH₂NHCH₂CF₃ | Me |
| G-0403 | Cl | F | CH₂CH₂CH₂NHCH₂CF₃ | Me |
| G-0404 | Cl | F | CH₂CH₂CH₂CH₂NHCH₂CF₃ | Me |
| G-0405 | Cl | F | CH₂CH₂NHC(=O)C(Me)(CF₃)₂ | Me |
| G-0406 | Cl | F | CH₂CH₂NHC(=O)Me | Me |
| G-0407 | Cl | F | CH₂CH₂NHC(=O)(*t*-Bu) | Me |
| G-0408 | Cl | F | CH₂CH₂CH₂CH₂NHC(=O)CH(CH₃)₂ | Me |
| G-0409 | Cl | F | CH₂CH₂CH₂CH₂NHC(=O)(*t*-Bu) | Me |
| G-0410 | Cl | F | CH₂CH₂CH₂CH₂NHC(=O)CH₂(*t*-Bu) | Me |
| G-0411 | Cl | F | CH₂CH₂NHC(=O)CF₃ | Me |
| G-0412 | Cl | F | CH₂CH₂NHC(=O)CH₂CF₃ | Me |
| G-0413 | Cl | F | CH₂CH₂NHC(=O)CF₂CF₃ | Me |
| G-0414 | Cl | F | CH₂CH₂CH₂NHC(=O)CF₃ | Me |
| G-0415 | Cl | F | CH₂CH₂CH₂NHC(=O)CH₂CF₃ | Me |
| G-0416 | Cl | F | CH₂CH₂CH₂NHC(=O)CF₂CF₃ | Me |
| G-0417 | Cl | F | CH₂CH₂CH₂CH₂NHC(=O)CF₃ | Me |
| G-0418 | Cl | F | CH₂CH₂CH₂CH₂NHC(=O)CH₂CF₃ | Me |
| G-0419 | Cl | F | CH₂CH₂CH₂CH₂NHC(=O)CF₂CF₃ | Me |
| G-0420 | Cl | F | CH₂CH₂CH₂CH₂NHC(=O)CF(CF₃)₂ | Me |
| G-0421 | Cl | F | CH₂CH₂CH₂CH₂NHC(=O)CCl₃ | Me |
| G-0422 | Cl | F | CH₂CH₂NHC(=O)Ph | Me |
| G-0423 | Cl | F | CH₂CH₂NHC(=O)Ph(4-CF₃) | Me |
| G-0424 | Cl | F | CH₂CH₂CH₂NHC(=O)Ph(4-CF₃) | Me |
| G-0425 | Cl | F | CH₂CH₂CH₂CH₂NHC(=O)Ph(4-CF₃) | Me |
| G-0426 | Cl | F | CH₂CH₂NHC(=O)O(*t*-Bu) | Me |
| G-0427 | Cl | F | CH₂CH₂CH₂NHC(=O)O(*t*-Bu) | Me |
| G-0428 | Cl | F | CH₂CH₂CH₂CH₂NHC(=O)O(*t*-Bu) | Me |
| G-0429 | Cl | F | CH₂CH₂CH₂CH₂NHC(=O)OCH(CH₃)₂ | Me |
| G-0430 | Cl | F | CH₂CH(Me)NHC(=O)O(*t*-Bu) | Me |
| G-0431 | Cl | F | CH₂C*H(Me)NHC(=O)O(*t*-Bu) : (R) | Me |
| G-0432 | Cl | F | CH₂C*H(Me)NHC(=O)O(*t*-Bu) : (S) | Me |
| G-0433 | Cl | F | CH₂CH(Et)NHC(=O)O(*t*-Bu) | Me |
| G-0434 | Cl | F | CH₂CH(*i*-Pr)NHC(=O)O(*t*-Bu) | Me |

**[Table 1130]**

| Compound Number | R³ | R⁴ | R⁵ | R¹¹ |
|---|---|---|---|---|
| G-0435 | Cl | F | CH(Me)CH₂NHC(=O)O(*t*-Bu) | Me |
| G-0436 | Cl | F | CH(Et)CH₂NHC(=O)O(*t*-Bu) | Me |
| G-0437 | Cl | F | CH(*i*-Pr)CH₂NHC(=O)O(*t*-Bu) | Me |
| G-0438 | Cl | F | CH₂CH(Me)CH₂NHC(=O)O(*t*-Bu) | Me |
| G-0439 | Cl | F | CH₂CH₂NHC(=O)OC(Me)₂CF₃ | Me |
| G-0440 | Cl | F | CH₂CH₂CH₂NHC(=O)OCH₂CF₃ | Me |
| G-0441 | Cl | F | CH₂CH₂CH₂NHC(=O)OCH₂CH₂CF₃ | Me |
| G-0442 | Cl | F | CH₂CH₂CH₂CH₂NHC(=O)OCH₂CCl₃ | Me |
| G-0443 | Cl | F | CH₂CH₂CH₂CH₂NHC(=O)OCH₂CF₃ | Me |
| G-0444 | Cl | F | CH₂CH₂NHC(=O)NH(*t*-Bu) | Me |
| G-0445 | Cl | F | CH₂CH₂CH₂NHC(=O)NH(*t*-Bu) | Me |
| G-0446 | Cl | F | CH₂CH₂CH₂CH₂NHC(=O)NHEt | Me |
| G-0447 | Cl | F | CH₂CH₂CH₂CH₂NHC(=O)NH(*t*-Bu) | Me |
| G-0448 | Cl | F | CH₂CH₂CH₂NHC(=O)NHCH₂CCl₃ | Me |
| G-0449 | Cl | F | CH₂CH₂CH₂NHC(=O)NHCH₂CH₂F | Me |
| G-0450 | Cl | F | CH₂CH₂CH₂NHC(=O)NHCH₂CF₃ | Me |
| G-0451 | Cl | F | CH₂CH₂CH₂CH₂NHC(=O)NHCH₂CCl₃ | Me |
| G-0452 | Cl | F | CH₂CH₂CH₂CH₂NHC(=O)NHCH₂CH₂F | Me |
| G-0453 | Cl | F | CH₂CH₂CH₂CH₂NHC(=O)NHCH₂CF₃ | Me |
| G-0454 | Cl | F | CH₂CH₂NHS(=O)Me | Me |
| G-0455 | Cl | F | CH₂CH₂CH₂NHS(=O)Me | Me |
| G-0456 | Cl | F | CH₂CH₂CH₂CH₂NHS(=O)Me | Me |
| G-0457 | Cl | F | CH₂CH₂CH₂CH₂NHS(=O)CH(CH₃)₂ | Me |
| G-0458 | Cl | F | CH₂CH₂CH₂CH₂CH₂NHS(=O)Me | Me |
| G-0459 | Cl | F | CH₂CH₂NHS(=O)CHF₂ | Me |
| G-0460 | Cl | F | CH₂CH₂CH₂NHS(=O)CHF₂ | Me |
| G-0461 | Cl | F | CH₂CH₂CH₂CH₂NHS(=O)CHF₂ | Me |
| G-0462 | Cl | F | CH₂CH₂CH₂CH₂CH₂NHS(=O)CHF₂ | Me |
| G-0463 | Cl | F | CH₂CH(Me)NHS(=O)CHF₂ | Me |
| G-0464 | Cl | F | CH(Me)CH₂NHS(=O)CHF₂ | Me |
| G-0465 | Cl | F | CH₂CH₂CH(Me)NHS(=O)CHF₂ | Me |
| G-0466 | Cl | F | CH₂CH(Me)CH₂NHS(=O)CHF₂ | Me |
| G-0467 | Cl | F | CH(Me)CH₂CH₂NHS(=O)CHF₂ | Me |
| G-0468 | Cl | F | CH₂CH₂NHS(=O)CF₃ | Me |
| G-0469 | Cl | F | CH₂CH₂CH₂NHS(=O)CF₃ | Me |
| G-0470 | Cl | F | CH₂CH₂CH₂CH₂NHS(=O)CF₃ | Me |
| G-0471 | Cl | F | CH₂CH₂CH₂CH₂CH₂NHS(=O)CF₃ | Me |
| G-0472 | Cl | F | CH₂CH₂CH₂NHS(=O)Ph | Me |
| G-0473 | Cl | F | CH₂CH₂CH₂CH₂NHS(=O)Ph | Me |
| G-0474 | Cl | F | CH₂CH₂CH₂CH₂CH₂NHS(=O)Ph | Me |
| G-0475 | Cl | F | CH₂CH₂CH₂CH₂CH₂NHS(=O)Ph(4-CF₃) | Me |
| G-0476 | Cl | F | CH₂CH(Me)NHS(=O)Ph(4-CF₃) | Me |
| G-0477 | Cl | F | CH₂CH(Et)NHS(=O)Ph(4-CF₃) | Me |
| G-0478 | Cl | F | CH(Me)CH₂NHS(=O)Ph(4-CF₃) | Me |

**[Table 1131]**

| Compound Number | R³ | R⁴ | R⁵ | R¹¹ |
|---|---|---|---|---|
| G-0479 | Cl | F | CH(Et)CH₂NHS(=O)Ph(4-CF₃) | Me |
| G-0480 | Cl | F | CH₂CH(Et)CH₂NHS(=O)Ph(4-CF₃) | Me |
| G-0481 | Cl | F | CH₂CH₂NHS(=O)₂Me | Me |
| G-0482 | Cl | F | CH₂CH₂CH₂NHS(=O)₂Me | Me |
| G-0483 | Cl | F | CH₂CH₂CH₂CH₂NHS(=O)₂Me | Me |
| G-0484 | Cl | F | CH₂CH₂CH₂CH₂NHS(=O)₂CH(CH₃)₂ | Me |
| G-0485 | Cl | F | CH₂CH₂CH₂CH₂CH₂NHS(=O)₂Me | Me |
| G-0486 | Cl | F | CH₂CH₂NHS(=O)₂CHF₂ | Me |
| G-0487 | Cl | F | CH₂CH₂CH₂NHS(=O)₂CHF₂ | Me |
| G-0488 | Cl | F | CH₂CH₂CH₂CH₂NHS(=O)₂CHF₂ | Me |
| G-0489 | Cl | F | CH₂CH₂CH₂CH₂CH₂NHS(=O)₂CHF₂ | Me |
| G-0490 | Cl | F | CH₂CH(Me)NHS(=O)₂CHF₂ | Me |
| G-0491 | Cl | F | CH(Me)CH₂NHS(=O)₂CHF₂ | Me |
| G-0492 | Cl | F | CH₂CH₂CH(Me)NHS(=O)₂CHF₂ | Me |
| G-0493 | Cl | F | CH₂CH(Me)CH₂NHS(=O)₂CHF₂ | Me |
| G-0494 | Cl | F | CH(Me)CH₂CH₂NHS(=O)₂CHF₂ | Me |
| G-0495 | Cl | F | CH₂CH(Me)N(Me)S(=O)₂CHF₂ | Me |
| G-0496 | Cl | F | CH(Me)CH₂N(Me)S(=O)₂CHF₂ | Me |
| G-0497 | Cl | F | CH₂CH₂CH(Me)N(Me)S(=O)₂CHF₂ | Me |
| G-0498 | Cl | F | CH₂CH(Me)CH₂N(Me)S(=O)₂CHF₂ | Me |
| G-0499 | Cl | F | CH(Me)CH₂CH₂N(Me)S(=O)₂CHF₂ | Me |
| G-0500 | Cl | F | CH₂CH₂NHS(=O)₂CF₃ | Me |
| G-0501 | Cl | F | CH₂CH₂CH₂NHS(=O)₂CF₃ | Me |
| G-0502 | Cl | F | CH₂CH₂CH₂CH₂NHS(=O)₂CF₃ | Me |
| G-0503 | Cl | F | CH₂CH₂CH₂CH₂CH₂NHS(=O)₂CF₃ | Me |
| G-0504 | Cl | F | CH₂CH₂N(Me)S(=O)₂CF₃ | Me |
| G-0505 | Cl | F | CH₂CH₂N(Et)S(=O)₂CF₃ | Me |
| G-0506 | Cl | F | CH₂CH₂N(CH₂CN)S(=O)₂CF₃ | Me |
| G-0507 | Cl | F | CH₂CH₂N(CH₂OMe)S(=O)₂CF₃ | Me |
| G-0508 | Cl | F | CH₂CH₂CH₂N(Me)S(=O)₂CF₃ | Me |
| G-0509 | Cl | F | CH₂CH₂CH₂N(Et)S(=O)₂CF₃ | Me |
| G-0510 | Cl | F | CH₂CH₂CH₂N(CH₂CN)S(=O)₂CF₃ | Me |
| G-0511 | Cl | F | CH₂CH₂CH₂N(CH₂OMe)S(=O)₂CF₃ | Me |
| G-0512 | Cl | F | CH₂CH₂CH₂CH₂N(Me)S(=O)₂CF₃ | Me |
| G-0513 | Cl | F | CH₂CH₂CH₂CH₂N(Et)S(=O)₂CF₃ | Me |
| G-0514 | Cl | F | CH₂CH₂CH₂CH₂N(CH₂CN)S(=O)₂CF₃ | Me |
| G-0515 | Cl | F | CH₂CH₂CH₂CH₂N(CH₂OMe)S(=O)₂CF₃ | Me |
| G-0516 | Cl | F | CH₂CH(Me)NHS(=O)₂CF₃ | Me |
| G-0517 | Cl | F | CH₂C*H(Me)NHS(=O)₂CF₃: (R) | Me |
| G-0518 | Cl | F | CH₂C*H(Me)NHS(=O)₂CF₃ : (S) | Me |
| G-0519 | Cl | F | CH(Me)CH₂NHS(=O)₂CF₃ | Me |
| G-0520 | Cl | F | CH₂CH₂CH(Me)NHS(=O)₂CF₃ | Me |
| G-0521 | Cl | F | CH₂CH(Me)CH₂NHS(=O)₂CF₃ | Me |
| G-0522 | Cl | F | CH(Me)CH₂CH₂NHS(=O)₂CF₃ | Me |

**[Table 1132]**

| Compound Number | R³ | R⁴ | R⁵ | R¹¹ |
|---|---|---|---|---|
| G-0523 | Cl | F | CH₂CH(Me)N(Me)S(=O)₂CF₃ | Me |
| G-0524 | Cl | F | CH(Me)CH₂N(Me)S(=O)₂CF₃ | Me |
| G-0525 | Cl | F | CH₂CH₂CH(Me)N(Me)S(=O)₂CF₃ | Me |
| G-0526 | Cl | F | CH₂CH(Me)CH₂N(Me)S(=O)₂CF₃ | Me |
| G-0527 | Cl | F | CH(Me)CH₂CH₂N(Me)S(=O)₂CF₃ | Me |
| G-0528 | Cl | F | CH₂CH₂CH₂NHS(=O)₂Ph | Me |
| G-0529 | Cl | F | CH₂CH₂CH₂CH₂NHS(=O)₂Ph | Me |
| G-0530 | Cl | F | CH₂CH₂CH₂CH₂CH₂NHS(=O)₂Ph | Me |
| G-0531 | Cl | F | CH₂CH₂CH₂CH₂CH₂NHS(=O)₂Ph(4-CF₃) | Me |
| G-0532 | Cl | F | CH₂CH(Me)NHS(=O)₂Ph(4-CF₃) | Me |
| G-0533 | Cl | F | CH₂CH(Et)NHS(=O)₂Ph(4-CF₃) | Me |
| G-0534 | Cl | F | CH(Me)CH₂NHS(=O)₂Ph(4-CF₃) | Me |
| G-0535 | Cl | F | CH(Et)CH₂NHS(=O)₂Ph(4-CF₃) | Me |
| G-0536 | Cl | F | CH₂CH(Me)CH₂NHS(=O)₂Ph(4-CF₃) | Me |
| G-0537 | Cl | F | CH₂CH₂SiMe₃ | Me |
| G-0538 | Cl | F | CH₂CH₂CH₂SiMe₃ | Me |
| G-0539 | Cl | F | CH₂CH₂CH₂CH₂SiMe₃ | Me |
| G-0540 | Cl | F | CH₂Ph | Me |
| G-0541 | Cl | F | CH₂Ph(2-CF₃) | Me |
| G-0542 | Cl | F | CH₂Ph(3-CF₃) | Me |
| G-0543 | Cl | F | CH₂Ph(4-CF₃) | Me |
| G-0544 | Cl | F | CH₂Ph(2-OCF₃) | Me |
| G-0545 | Cl | F | CH₂Ph(3-OCF₃) | Me |
| G-0546 | Cl | F | CH₂Ph(4-OCF₃) | Me |
| G-0547 | Cl | F | CH₂Ph(2-SCF₃) | Me |
| G-0548 | Cl | F | CH₂Ph(3-SCF₃) | Me |
| G-0549 | Cl | F | CH₂Ph(4-SCF₃) | Me |
| G-0550 | Cl | F | CH₂Ph(3-CH₂SCF₃) | Me |
| G-0551 | Cl | F | CH₂Ph(4-F) | Me |
| G-0552 | Cl | F | CH₂Ph(4-Cl) | Me |
| G-0553 | Cl | F | CH₂Ph(4-Br) | Me |
| G-0554 | Cl | F | CH₂Ph(4-Me) | Me |
| G-0555 | Cl | F | CH₂Ph[4-(*t*-Bu)] | Me |
| G-0556 | Cl | F | CH₂Ph(4-CN) | Me |
| G-0557 | Cl | F | CH₂Ph(4-NO₂) | Me |
| G-0558 | Cl | F | CH₂Ph(4-OCHF₂) | Me |
| G-0559 | Cl | F | CH₂Ph(4-SCHF₂) | Me |
| G-0560 | Cl | F | CH₂Ph(4-CH₂SCF₃) | Me |
| G-0561 | Cl | F | CH₂Ph[4-CF(CF₃)₂] | Me |
| G-0562 | Cl | F | CH₂Ph(4-CH₂SCF₃) | Me |
| G-0563 | Cl | F | CH₂Ph[4-Ph(4-CF₃)] | Me |
| G-0564 | Cl | F | CH₂Ph(2,4-Cl₂) | Me |
| G-0565 | Cl | F | CH₂Ph[2,5-(CF₃)₂] | Me |
| G-0566 | Cl | F | CH₂Ph(3,4-Cl₂) | Me |

**[Table 1133]**

| Compound Number | R³ | R⁴ | R⁵ | R¹¹ |
|---|---|---|---|---|
| G-0567 | Cl | F | CH₂Ph(3-CF₃-4-F) | Me |
| G-0568 | Cl | F | CH₂Ph(3-CF₃-4-Cl) | Me |
| G-0569 | Cl | F | CH₂Ph(3-F-4-CF₃) | Me |
| G-0570 | Cl | F | CH₂Ph(2,4,6-F₃) | Me |
| G-0571 | Cl | F | CH₂Ph(3,4,5-F₃) | Me |
| G-0572 | Cl | F | CH₂Ph(2,3,4-F₃) | Me |
| G-0573 | Cl | F | CH₂Ph(3,4,5-Cl₃) | Me |
| G-0574 | Cl | F | CH₂CH₂Ph | Me |
| G-0575 | Cl | F | CH₂CH₂Ph(4-F) | Me |
| G-0576 | Cl | F | CH₂CH₂Ph(4-Cl) | Me |
| G-0577 | Cl | F | CH₂CH₂Ph(4-Br) | Me |
| G-0578 | Cl | F | CH₂CH₂Ph[4-(*t*-Bu)] | Me |
| G-0579 | Cl | F | CH₂CH₂Ph(2-CF₃) | Me |
| G-0580 | Cl | F | CH₂CH₂Ph(3-CF₃) | Me |
| G-0581 | Cl | F | CH₂CH₂Ph(4-CF₃) | Me |
| G-0582 | Cl | F | CH₂CH₂Ph[4-CF(CF₃)₂] | Me |
| G-0583 | Cl | F | CH₂CH₂Ph[4-(*c*-Pr)] | Me |
| G-0584 | Cl | F | CH₂CH₂Ph{4-[*c-*Pr(2,2-F₂)]} | Me |
| G-0585 | Cl | F | CH₂CH₂Ph(4-OMe) | Me |
| G-0586 | Cl | F | CH₂CH₂Ph(3-OCF₃) | Me |
| G-0587 | Cl | F | CH₂CH₂Ph(4-OCHF₂) | Me |
| G-0588 | Cl | F | CH₂CH₂Ph(4-OCF₃) | Me |
| G-0589 | Cl | F | CH₂CH₂Ph(4-SMe) | Me |
| G-0590 | Cl | F | CH₂CH₂Ph(4-SCHF₂) | Me |
| G-0591 | Cl | F | CH₂CH₂Ph(4-SCF₃) | Me |
| G-0592 | Cl | F | CH₂CH₂Ph[4-S(=O)Me] | Me |
| G-0593 | Cl | F | CH₂CH₂Ph[4-S(=O)CF₃] | Me |
| G-0594 | Cl | F | CH₂CH₂Ph[4-S(=O)₂Me] | Me |
| G-0595 | Cl | F | CH₂CH₂Ph[4-S(=O)₂CF₃] | Me |
| G-0596 | Cl | F | CH₂CH₂Ph(4-CH₂SMe) | Me |
| G-0597 | Cl | F | CH₂CH₂Ph(4-CH₂SCF₃) | Me |
| G-0598 | Cl | F | CH₂CH₂Ph[4-OS(=O)₂Me] | Me |
| G-0599 | Cl | F | CH₂CH₂Ph[4-OS(=O)₂CF₃] | Me |
| G-0600 | Cl | F | CH₂CH₂Ph[4-Ph(4-CF₃)] | Me |
| G-0601 | Cl | F | CH₂CH₂Ph(4-CH₂Ph) | Me |
| G-0602 | Cl | F | CH₂CH₂Ph(4-OCH₂Ph) | Me |
| G-0603 | Cl | F | CH₂CH₂Ph(4-CN) | Me |
| G-0604 | Cl | F | CH₂CH₂Ph(4-NO₂) | Me |
| G-0605 | Cl | F | CH₂CH₂Ph(2,4-Cl₂) | Me |
| G-0606 | Cl | F | CH₂CH₂Ph(3,4-Cl₂) | Me |
| G-0607 | Cl | F | CH₂CH₂Ph(3-CF₃-4-F) | Me |
| G-0608 | Cl | F | CH₂CH₂Ph(2-CF₃-4-F) | Me |
| G-0609 | Cl | F | CH₂CH₂Ph(3-F-4-CF₃) | Me |
| G-0610 | Cl | F | CH₂CH₂Ph(2-F-4-CF₃) | Me |

**[Table 1134]**

| Compound Number | R³ | R⁴ | R⁵ | R¹¹ |
|---|---|---|---|---|
| G-0611 | Cl | F | CH₂CH₂Ph(3-Cl-4-OCHF₂) | Me |
| G-0612 | Cl | F | CH₂CH₂Ph(3,4,5-Cl₃) | Me |
| G-0613 | Cl | F | CH₂CH₂Ph(2,3,4-F₃) | Me |
| G-0614 | Cl | F | CH₂CH₂Ph(2,4,5-F₃) | Me |
| G-0615 | Cl | F | CH₂CH₂Ph(3,4,5-F₃) | Me |
| G-0616 | Cl | F | CH₂CH₂Ph(2,4,6-F₃) | Me |
| G-0617 | Cl | F | CH₂CH₂CH₂Ph | Me |
| G-0618 | Cl | F | CH₂CH₂CH₂Ph(3-CF₃) | Me |
| G-0619 | Cl | F | CH₂CH₂CH₂Ph(4-CF₃) | Me |
| G-0620 | Cl | F | CH₂CH₂CH₂Ph(4-F) | Me |
| G-0621 | Cl | F | CH₂CH₂CH₂Ph[4-(*t*-Bu)] | Me |
| G-0622 | Cl | F | CH₂CH₂CH₂Ph(4-CN) | Me |
| G-0623 | Cl | F | CH₂CH₂CH₂Ph(4-CF₃) | Me |
| G-0624 | Cl | F | CH₂CH₂CH₂Ph(4-OCHF₂) | Me |
| G-0625 | Cl | F | CH₂CH₂CH₂Ph(4-OCF₃) | Me |
| G-0626 | Cl | F | CH₂CH₂CH₂Ph(4-SCHF₂) | Me |
| G-0627 | Cl | F | CH₂CH₂CH₂Ph(4-SCF₃) | Me |
| G-0628 | Cl | F | CH₂CH₂CH₂Ph[4-CF(CF₃)₂] | Me |
| G-0629 | Cl | F | CH₂CH₂CH₂Ph(3,4,5-F₃) | Me |
| G-0630 | Cl | F | CH₂CH₂CH₂Ph(2,4,6-F₃) | Me |
| G-0631 | Cl | F | CH₂CH₂CH₂CH₂Ph | Me |
| G-0632 | Cl | F | CH₂CH₂CH₂CH₂Ph(4-F) | Me |
| G-0633 | Cl | F | CH₂CH₂CH₂CH₂Ph(4-CF₃) | Me |
| G-0634 | Cl | F | CH₂CH₂CH₂CH₂Ph(4-OCF₃) | Me |
| G-0635 | Cl | F | CH₂CH₂CH₂CH₂Ph(4-SCF₃) | Me |
| G-0636 | Cl | F | CH₂CH₂CH₂CH₂CH₂Ph | Me |
| G-0637 | Cl | F | CH₂CH₂CH₂CH₂CH₂CH₂Ph | Me |
| G-0638 | Cl | F | CH₂CF₂Ph(4-F) | Me |
| G-0639 | Cl | F | CH₂CF₂Ph(4-CF₃) | Me |
| G-0640 | Cl | F | CH₂CF₂Ph(3,4,5-F₃) | Me |
| G-0641 | Cl | F | CH₂CH₂OPh | Me |
| G-0642 | Cl | F | CH₂CH₂OPh(4-F) | Me |
| G-0643 | Cl | F | CH₂CH₂OPh(4-CF₃) | Me |
| G-0644 | Cl | F | CH₂CH₂CH₂OPh | Me |
| G-0645 | Cl | F | CH₂CH₂CH₂OPh(4-Cl) | Me |
| G-0646 | Cl | F | CH₂CH₂CH₂OPh(4-CF₃) | Me |
| G-0647 | Cl | F | CH₂CH₂CH₂CH₂OPh(4-CF₃) | Me |
| G-0648 | Cl | F | CH₂CH₂CH₂CH₂OPh(4-OCF₃) | Me |
| G-0649 | Cl | F | CH₂CH₂CH₂CH₂CH₂OPh(4-CF₃) | Me |
| G-0650 | Cl | F | CH₂CH₂OCH₂Ph | Me |
| G-0651 | Cl | F | CH₂CH₂CH₂OCH₂Ph | Me |
| G-0652 | Cl | F | CH₂CH₂CH₂CH₂OCH₂Ph | Me |
| G-0653 | Cl | F | CH₂CH₂CH₂CH₂CH₂OCH₂Ph | Me |
| G-0654 | Cl | F | CH₂CH₂SPh | Me |

**[Table 1135]**

| Compound Number | R³ | R⁴ | R⁵ | R¹¹ |
|---|---|---|---|---|
| G-0655 | Cl | F | CH₂CH₂CH₂SPh | Me |
| G-0656 | Cl | F | CH₂CH₂CH₂SPh(4-F) | Me |
| G-0657 | Cl | F | CH₂CH₂CH₂SPh(4-Cl) | Me |
| G-0658 | Cl | F | CH₂CH₂CH₂SPh(4-Br) | Me |
| G-0659 | Cl | F | CH₂CH₂CH₂SPh[4-(*t*-Bu)] | Me |
| G-0660 | Cl | F | CH₂CH₂CH₂SPh(3-CF₃) | Me |
| G-0661 | Cl | F | CH₂CH₂CH₂SPh(4-CF₃) | Me |
| G-0662 | Cl | F | CH₂CH₂CH₂SPh(3-SCF₃) | Me |
| G-0663 | Cl | F | CH₂CH₂CH₂SPh(4-SCF₃) | Me |
| G-0664 | Cl | F | CH₂CH₂CH₂CH₂SPh | Me |
| G-0665 | Cl | F | CH₂CH₂CH₂CH₂SPh(4-F) | Me |
| G-0666 | Cl | F | CH₂CH₂CH₂CH₂SPh(4-Cl) | Me |
| G-0667 | Cl | F | CH₂CH₂CH₂CH₂SPh[4-(*t*-Bu)] | Me |
| G-0668 | Cl | F | CH₂CH₂CH₂CH₂SPh(4-CF₃) | Me |
| G-0669 | Cl | F | CH₂CH₂CH₂CH₂CH₂SPh | Me |
| G-0670 | Cl | F | CH₂CH₂CH₂CH₂CH₂SPh(4-F) | Me |
| G-0671 | Cl | F | CH₂CH₂CH₂CH₂CH₂SPh(4-Cl) | Me |
| G-0672 | Cl | F | CH₂CH₂CH₂CH₂CH₂SPh(4-CF₃) | Me |
| G-0673 | Cl | F | CH₂CH₂S(=O)Ph | Me |
| G-0674 | Cl | F | CH₂CH₂CH₂S(=O)Ph | Me |
| G-0675 | Cl | F | CH₂CH₂CH₂S(=O)Ph(4-F) | Me |
| G-0676 | Cl | F | CH₂CH₂CH₂S(=O)Ph[4-(*t*-Bu)] | Me |
| G-0677 | Cl | F | CH₂CH₂CH₂S(=O)Ph(4-CF₃) | Me |
| G-0678 | Cl | F | CH₂CH₂S(=O)₂Ph | Me |
| G-0679 | Cl | F | CH₂CH₂S(=O)₂Ph(4-CF₃) | Me |
| G-0680 | Cl | F | CH₂CH₂CH₂S(=O)₂Ph(4-F) | Me |
| G-0681 | Cl | F | CH₂CH₂CH₂S(=O)₂Ph(4-Cl) | Me |
| G-0682 | Cl | F | CH₂CH₂CH₂S(=O)₂Ph(4-CF₃) | Me |
| G-0683 | Cl | F | CH₂CH₂CH₂CH₂S(=O)₂Ph | Me |
| G-0684 | Cl | F | CH₂CH₂CH₂CH₂S(=O)₂Ph(4-Cl) | Me |
| G-0685 | Cl | F | CH₂CH₂CH₂CH₂S(=O)₂Ph(4-CF₃) | Me |
| G-0686 | Cl | F | CH₂CH₂SCH₂Ph | Me |
| G-0687 | Cl | F | CH₂CH₂CH₂SCH₂Ph | Me |
| G-0688 | Cl | F | CH₂CH₂CH₂SCH₂Ph(2-Cl) | Me |
| G-0689 | Cl | F | CH₂CH₂CH₂SCH₂Ph(3-Cl) | Me |
| G-0690 | Cl | F | CH₂CH₂CH₂SCH₂Ph(4-Cl) | Me |
| G-0691 | Cl | F | CH₂CH₂CH₂SCH₂Ph(2-SCF₃) | Me |
| G-0692 | Cl | F | CH₂CH₂CH₂SCH₂Ph(3-CF₃) | Me |
| G-0693 | Cl | F | CH₂CH₂CH₂SCH₂Ph(4-CF₃) | Me |
| G-0694 | Cl | F | CH₂CH₂CH₂SCH₂Ph(4-NO₂) | Me |
| G-0695 | Cl | F | CH₂CH₂CH₂CH₂SCH₂Ph | Me |
| G-0696 | Cl | F | CH₂CH₂CH₂CH₂SCH₂Ph(4-Cl) | Me |
| G-0697 | Cl | F | CH₂CH₂CH₂CH₂SCH₂Ph(4-CF₃) | Me |
| G-0698 | Cl | F | CH₂CH₂CH₂CH₂SCH₂Ph(4-CN) | Me |

**[Table 1136]**

| Compound Number | R³ | R⁴ | R⁵ | R¹¹ |
|---|---|---|---|---|
| G-0699 | Cl | F | CH₂CH₂CH₂CH₂CH₂SCH₂Ph | Me |
| G-0700 | Cl | F | CH₂CH₂SCH₂CH₂Ph | Me |
| G-0701 | Cl | F | CH₂CH₂CH₂SCH₂CH₂Ph | Me |
| G-0702 | Cl | F | CH₂CH₂CH₂CH₂SCH₂CH₂Ph | Me |
| G-0703 | Cl | F | CH₂CH₂SCH(Me)Ph | Me |
| G-0704 | Cl | F | CH₂CH₂CH₂SCH(Me)Ph | Me |
| G-0705 | Cl | F | CH₂CH₂CH₂CH₂SCH(Me)Ph | Me |
| G-0706 | Cl | F | CH₂CH₂S(=O)CH₂Ph | Me |
| G-0707 | Cl | F | CH₂CH₂S(=O)CH₂Ph(4-CF₃) | Me |
| G-0708 | Cl | F | CH₂CH₂CH₂S(=O)CH₂Ph | Me |
| G-0709 | Cl | F | CH₂CH₂CH₂S(=O)CH₂Ph(2-SCF₃) | Me |
| G-0710 | Cl | F | CH₂CH₂CH₂S(=O)CH₂Ph(4-SCF₃) | Me |
| G-0711 | Cl | F | CH₂CH₂CH₂S(=O)CH₂Ph(4-Cl) | Me |
| G-0712 | Cl | F | CH₂CH₂CH₂S(=O)CH₂Ph(4-CF₃) | Me |
| G-0713 | Cl | F | CH₂CH₂CH₂CH₂S(=O)CH₂Ph | Me |
| G-0714 | Cl | F | CH₂CH₂CH₂CH₂S(=O)CH₂Ph(4-CF₃) | Me |
| G-0715 | Cl | F | CH₂CH₂CH₂CH₂CH₂S(=O)CH₂Ph(4-CF₃) | Me |
| G-0716 | Cl | F | CH₂CH₂S(=O)₂CH₂Ph | Me |
| G-0717 | Cl | F | CH₂CH₂S(=O)₂CH₂Ph(4-CF₃) | Me |
| G-0718 | Cl | F | CH₂CH₂CH₂S(=O)₂CH₂Ph | Me |
| G-0719 | Cl | F | CH₂CH₂CH₂S(=O)₂CH₂Ph(4-Cl) | Me |
| G-0720 | Cl | F | CH₂CH₂CH₂S(=O)₂CH₂Ph(4-CF₃) | Me |
| G-0721 | Cl | F | CH₂CH₂CH₂CH₂S(=O)₂CH₂Ph | Me |
| G-0722 | Cl | F | CH₂CH₂CH₂CH₂S(=O)₂CH₂Ph(4-CF₃) | Me |
| G-0723 | Cl | F | CH₂CH₂CH₂CH₂CH₂S(=O)₂CH₂Ph | Me |
| G-0724 | Cl | F | CH₂CH₂CH₂CH₂CH₂S(=O)₂CH₂Ph(4-CF₃) | Me |
| G-0725 | Cl | F | CH₂CH₂ON=CH(*t*-Bu) | Me |
| G-0726 | Cl | F | CH₂CH₂CH₂ON=CH(*t*-Bu) | Me |
| G-0727 | Cl | F | CH₂CH₂CH₂CH₂ON=C(Me)(*c*-Pr) | Me |
| G-0728 | Cl | F | CH₂CH₂ON=C(Me)CF₃ | Me |
| G-0729 | Cl | F | CH₂CH₂CH₂ON=CHCF₃ | Me |
| G-0730 | Cl | F | CH₂CH₂CH₂ON=C(Me)CF₃ | Me |
| G-0731 | Cl | F | CH₂CH₂CH₂ON=C(Me)CCl₃ | Me |
| G-0732 | Cl | F | CH₂CH₂CH₂CH₂ON=CHCF₃ | Me |
| G-0733 | Cl | F | CH₂CH₂CH₂CH₂ON=C(Me)CF₃ | Me |
| G-0734 | Cl | F | CH₂CH₂CH₂CH₂CH₂ON=C(Me)CF₃ | Me |
| G-0735 | Cl | F | CH₂CH₂ON=CHPh | Me |
| G-0736 | Cl | F | CH₂CH₂ON=CHPh(4-CF₃) | Me |
| G-0737 | Cl | F | CH₂CH₂ON=CHPh(4-SCF₃) | Me |
| G-0738 | Cl | F | CH₂CH₂CH₂ON=CHPh(3-CF₃) | Me |
| G-0739 | Cl | F | CH₂CH₂CH₂ON=CHPh(4-CF₃) | Me |
| G-0740 | Cl | F | CH₂CH₂CH₂ON=CHPh(4-SCF₃) | Me |
| G-0741 | Cl | F | CH₂CH₂CH₂ON=C(Me)Ph(4-CF₃) | Me |
| G-0742 | Cl | F | CH₂CH₂CH2CH₂ON=CHPh(4-SCF3) | Me |

**[Table 1137]**

| Compound Number | R³ | R⁴ | R⁵ | R¹¹ |
|---|---|---|---|---|
| G-0743 | Cl | F | CH₂CH₂(adamant-1-yl) | Me |
| G-0744 | Cl | F | CH₂CH₂CH₂(3-CF₃-pyrazol-1-yl) | Me |
| G-0745 | Cl | F | CH₂CH₂CH₂CH₂(3-CF₃-pyrazol-1-yl) | Me |
| G-0746 | Cl | F | CH₂CH₂CH₂CH₂CH₂(3-CF₃-pyrazol-1-yl) | Me |
| G-0747 | Cl | F | CH₂(1-Me-4-CF₃-imidazol-5-yl) | Me |
| G-0748 | Cl | F | CH₂CH₂(1-Me-4-CF₃-imidazol-5-yl) | Me |
| G-0749 | Cl | F | CH₂CH₂CH₂(1-Me-4-CF₃-imidazol-5-yl) | Me |
| G-0750 | Cl | F | CH₂CH₂CH₂(3-CF₃-1,2,4-triazol-1-yl) | Me |
| G-0751 | Cl | F | CH₂CH₂CH₂CH₂(3-CF₃-1,2,4-triazol-1-yl) | Me |
| G-0752 | Cl | F | CH₂CH₂CH₂CH₂CH₂(3-CF₃-1,2,4-triazol-1-yl) | Me |
| G-0753 | Cl | F | CH₂CH₂CH₂(4-CF₃-thiazol-2-yl) | Me |
| G-0754 | Cl | F | CH₂CH₂CH₂CH₂(4-CF₃-thiazol-2-yl) | Me |
| G-0755 | Cl | F | CH₂CH₂CH₂CH₂CH₂(4-CF₃-thiazol-2-yl) | Me |
| G-0756 | Cl | F | CH₂CH₂CH₂(2-CF₃-pyridin-5-yl) | Me |
| G-0757 | Cl | F | CH₂CH₂CH₂CH₂(2-CF₃-pyridin-5-yl) | Me |
| G-0758 | Cl | F | CH₂CH₂CH₂CH₂CH₂(2-CF₃-pyridin-5-yl) | Me |
| G-0759 | Cl | F | CH₂(4-CF₃-pyrimidin-2-yl) | Me |
| G-0760 | Cl | F | CH₂CH₂(4-CF₃-pyrimidin-2-yl) | Me |
| G-0761 | Cl | F | CH₂CH₂CH₂(4-CF₃-pyrimidin-2-yl) | Me |
| G-0762 | Cl | F | CH₂CH₂O(3-Cl-5-CF₃-pyridin-2-yl) | Me |
| G-0763 | Cl | F | CH₂CH₂CH₂O(3-Cl-5-CF₃-pyridin-2-yl) | Me |
| G-0764 | Cl | F | CH₂CH₂CH₂CH₂O(3-Cl-5-CF₃-pyridin-2-yl) | Me |
| G-0765 | Cl | F | CH₂CH₂CH₂CH₂CH₂O(3-Cl-5-CF₃-pyridin-2-yl) | Me |
| G-0766 | Cl | F | CH₂CH₂N(Phth) | Me |
| G-0767 | Cl | F | CH₂CH₂CH₂N(Phth) | Me |
| G-0768 | Cl | F | CH₂CH₂CH₂CH₂N(Phth) | Me |
| G-0769 | Cl | F | CH₂CH₂CH₂CH₂CH₂N(Phth) | Me |
| G-0770 | Cl | F | CH₂CH(Me)CH₂N(Phth) | Me |
| G-0771 | Cl | F | CH₂[1,3-dioxolan(2-CF₃)-2-yl] | Me |
| G-0772 | Cl | F | CH₂(azetidin-3-yl) | Me |
| G-0773 | Cl | F | CH₂(pyrrolidin-3-yl) | Me |
| G-0774 | Cl | F | CH₂(piperidin-3-yl) | Me |
| G-0775 | Cl | F | CH₂{azetidin[1-C(=O)O(*t*-Bu)]-3-yl} | Me |
| G-0776 | Cl | F | CH₂{pyrrolidin[1-C(=O)O(*t*-Bu)]-3-yl} | Me |
| G-0777 | Cl | F | CH₂{piperidin[1-C(=O)O(*t*-Bu)]-3-yl} | Me |
| G-0778 | Cl | F | CH₂{azetidin[1-C(=O)CF₃]-3-yl} | Me |
| G-0779 | Cl | F | CH₂{azetidin[1-S(=O)₂CF₃]-3-yl} | Me |
| G-0780 | Cl | F | CH₂{pyrrolidin[1-C(=O)CF₃]-3-yl} | Me |
| G-0781 | Cl | F | CH₂{pyrrolidin[1-S(=O)₂CF₃]-3-yl} | Me |
| G-0782 | Cl | F | CH₂{piperidin[1-C(=O)CF₃]-3-yl} | Me |
| G-0783 | Cl | F | CH₂{piperidin[1-S(=O)₂CF₃]-3-yl} | Me |
| G-0784 | Cl | F | CH₂CH₂CH₂CN | Me |
| G-0785 | Cl | F | CH₂CH₂CH₂CH₂CN | Me |
| G-0786 | Cl | F | CH₂CH₂CH₂CH₂CH₂CN | Me |

**[Table 1138]**

| Compound Number | R³ | R⁴ | R⁵ | R¹¹ |
|---|---|---|---|---|
| G-0787 | Cl | F | CH₂CH₂CH₂CH₂CH₂CH₂CN | Me |
| G-0788 | Cl | F | CH₂CH₂CH₂C(=O)OH | Me |
| G-0789 | Cl | F | CH₂CH₂CH₂CH₂C(=O)OH | Me |
| G-0790 | Cl | F | CH₂CH₂CH₂CH₂CH₂C(=O)OH | Me |
| G-0791 | Cl | F | CH₂CH₂SCN | Me |
| G-0792 | Cl | F | CH₂CH₂CH₂SCN | Me |
| G-0793 | Cl | F | CH₂CH₂CH₂CH₂SCN | Me |
| G-0794 | Cl | F | CH₂CH₂CH₂CH₂CH₂SCN | Me |
| G-0795 | Cl | F | CH₂CH₂CH₂CH₂CH₂CH₂SCN | Me |
| G-0796 | Cl | F | CH₂CH₂CH₂CH₂CH₂CH₂CH₂SCN | Me |
| G-0797 | Cl | F | CH₂CH₂CH₂CH₂CH₂CH₂CH₂CH₂SCN | Me |
| G-0798 | Cl | F | CH₂CH₂CH(Me)SCN | Me |
| G-0799 | Cl | F | CH₂CH(Me)CH₂SCN | Me |
| G-0800 | Cl | F | CH(Me)CH₂CH₂SCN | Me |
| G-0801 | Cl | F | CH₂CH₂CH₂CH(Me)SCN | Me |
| G-0802 | Cl | F | CH₂CH₂CH(Me)CH₂SCN | Me |
| G-0803 | Cl | F | CH₂CH(Me)CH₂CH₂SCN | Me |
| G-0804 | Cl | F | CH(Me)CH₂CH₂CH₂SCN | Me |
| G-0805 | Cl | F | CH₂CH₂CH₂CH₂CH(Me)SCN | Me |
| G-0806 | Cl | F | CH₂CH₂CH₂CH(Me)CH₂SCN | Me |
| G-0807 | Cl | F | CH₂CH₂CH(Me)CH₂CH₂SCN | Me |
| G-0808 | Cl | F | CH₂CH(Me)CH₂CH₂CH₂SCN | Me |
| G-0809 | Cl | F | CH(Me)CH₂CH₂CH₂CH₂SCN | Me |
| G-0810 | Cl | F | CH₂CH₂ONH₂ | Me |
| G-0811 | Cl | F | CH₂CH₂CH₂ONH₂ | Me |
| G-0812 | Cl | F | CH₂CH₂CH₂CH₂ONH₂ | Me |
| G-0813 | Cl | F | CH₂CH₂CH₂CH₂CH₂ONH₂ | Me |
| G-0814 | Cl | F | CH₂CH₂CH₂CH₂CH₂CH₂ONH₂ | Me |
| G-0815 | Me | F | Me | Me |
| G-0816 | Me | F | Et | Me |
| G-0817 | Me | F | *n*-Pr | Me |
| G-0818 | Me | F | *i*-Pr | Me |
| G-0819 | Me | F | *n*-Bu | Me |
| G-0820 | Me | F | *n*-Pen | Me |
| G-0821 | Me | F | *n*-Hex | Me |
| G-0822 | Me | F | *n*-Heptyl | Me |
| G-0823 | Me | F | *n*-Octyl | Me |
| G-0824 | Me | F | *n*-Nonyl | Me |
| G-0825 | Me | F | *n*-Decyl | Me |
| G-0826 | Me | F | *c*-Pr | Me |
| G-0827 | Me | F | *c*-Pen | Me |
| G-0828 | Me | F | *c*-Hex | Me |
| G-0829 | Me | F | CH₂C(Me)=CH₂ | Me |
| G-0830 | Me | F | CH₂CH₂CH=CH₂ | Me |

**[Table 1139]**

| Compound Number | R³ | R⁴ | R⁵ | R¹¹ |
|---|---|---|---|---|
| G-0831 | Me | F | CH₂CH₂CH=C(CH₃)₂ | Me |
| G-0832 | Me | F | CH₂CH₂CH₂CH=CH₂ | Me |
| G-0833 | Me | F | CH₂CH₂CH₂CH=C(CH₃)₂ | Me |
| G-0834 | Me | F | CH₂CH₂CH₂CH₂CH=CH₂ | Me |
| G-0835 | Me | F | CH₂CH₂CH₂CH₂CH=C(CH₃)₂ | Me |
| G-0836 | Me | F | CH₂CH₂CH≡CH | Me |
| G-0837 | Me | F | CH₂CH₂CH₂CH≡CH | Me |
| G-0838 | Me | F | CH₂CH₂CH₂CH₂CH≡CH | Me |
| G-0839 | Me | F | CH₂CH₂CH₂Cl | Me |
| G-0840 | Me | F | CH₂CH₂CH₂Br | Me |
| G-0841 | Me | F | CH₂CH₂CH₂CH₂Cl | Me |
| G-0842 | Me | F | CH₂CH₂CH₂CH₂Br | Me |
| G-0843 | Me | F | CH₂CH₂CH₂CH₂CH₂Cl | Me |
| G-0844 | Me | F | CH₂CH₂CH₂CH₂CH₂Br | Me |
| G-0845 | Me | F | CH₂CH₂CH₂CH₂CH₂CH₂Cl | Me |
| G-0846 | Me | F | CH₂CH₂CH₂CH₂CH₂CH₂Br | Me |
| G-0847 | Me | F | CH₂CH₂CH₂CH₂CH₂CH₂CH₂Cl | Me |
| G-0848 | Me | F | CH₂CH₂CH₂CH₂CH₂CH₂CH₂Br | Me |
| G-0849 | Me | F | CH₂CH₂CH₂CH₂CH₂CH₂CH₂CH₂Br | Me |
| G-0850 | Me | F | CH₂CH(Me)CH₂Cl | Me |
| G-0851 | Me | F | CH₂CH(Me)CH₂Br | Me |
| G-0852 | Me | F | CH₂CH₂CH(Me)CH₂CH₂Cl | Me |
| G-0853 | Me | F | CH₂CH₂CH(Me)CH₂CH₂Br | Me |
| G-0854 | Me | F | CH₂CH₂CF=CF₂ | Me |
| G-0855 | Me | F | CH₂CH=C(Cl)CF₃ | Me |
| G-0856 | Me | F | CH₂CH₂CH₂CF=CF₂ | Me |
| G-0857 | Me | F | CH₂CH₂CH₂CH₂CF=CF₂ | Me |
| G-0858 | Me | F | CH₂CH₂CH₂CH₂CH₂CF=CF₂ | Me |
| G-0859 | Me | F | CH₂(*t*-Bu) | Me |
| G-0860 | Me | F | CH₂CH₂(*t*-Bu) | Me |
| G-0861 | Me | F | CH₂CH₂CH₂(*t*-Bu) | Me |
| G-0862 | Me | F | CH₂CH₂CH₂CH₂(*t*-Bu) | Me |
| G-0863 | Me | F | CH₂CH₂CH₂CH₂CH₂(*t*-Bu) | Me |
| G-0864 | Me | F | CH₂CH₂CH₂CH₂CH₂CH₂(*t*-Bu) | Me |
| G-0865 | Me | F | CH₂CF₃ | Me |
| G-0866 | Me | F | CH₂CH₂CF₃ | Me |
| G-0867 | Me | F | CH₂CH₂CH₂CF₃ | Me |
| G-0868 | Me | F | CH₂CH₂CH₂CH₂CF₃ | Me |
| G-0869 | Me | F | CH₂CH₂CH₂CH₂CH₂CF₃ | Me |
| G-0870 | Me | F | CH₂CH₂CH₂CH₂CH₂CH₂CF₃ | Me |
| G-0871 | Me | F | CH₂CH₂CH₂CH₂CH₂CH₂CH₂CF₃ | Me |
| G-0872 | Me | F | CH₂CF₂CF₃ | Me |
| G-0873 | Me | F | CF₂CHFCF₃ | Me |
| G-0874 | Me | F | CF₂CF₂CF₃ | Me |

**[Table 1140]**

| Compound Number | R³ | R⁴ | R⁵ | R¹¹ |
|---|---|---|---|---|
| G-0875 | Me | F | CH₂CF₂CF₂CF₃ | Me |
| G-0876 | Me | F | CH₂CF₂CF₂CF₂CF₃ | Me |
| G-0877 | Me | F | CH₂CF₂CF₂CF₂CHF₂ | Me |
| G-0878 | Me | F | CH(CF₃)CF₃ | Me |
| G-0879 | Me | F | CH₂CF(CF₃)CF₃ | Me |
| G-0880 | Me | F | CH₂CF₂CF(CF₃)CF₃ | Me |
| G-0881 | Me | F | CH_{2C}F(CF₃)CF₂CF₃ | Me |
| G-0882 | Me | F | CH₂CF₂CF₂CF(CF₃)CF₃ | Me |
| G-0883 | Me | F | CH₂CF₂CF(CF₃)CF₂CF₃ | Me |
| G-0884 | Me | F | CH₂CF(CF₃)CF₂CF₂CF₃ | Me |
| G-0885 | Me | F | CH₂(*c*-Pr) | Me |
| G-0886 | Me | F | CH₂[*c*-Pr(1-Me)] | Me |
| G-0887 | Me | F | CH₂[*c*-Pr(1-Ph)] | Me |
| G-0888 | Me | F | CH₂[*c*-Pr(1-NH₂)] | Me |
| G-0889 | Me | F | CH₂[*c*-Pr(1-NHC(=O)O(*t*-Bu)] | Me |
| G-0890 | Me | F | CH₂{*c*-Pr[1-NHS(=O)₂CF₃]} | Me |
| G-0891 | Me | F | CH₂{*c*-Pr[1-Ph(4-CF₃)]} | Me |
| G-0892 | Me | F | CH₂{*c*-Pr[1-Ph(3,4,5-F₃)]} | Me |
| G-0893 | Me | F | CH₂{*c*-Hex[4-(*t*-Bu)]} | Me |
| G-0894 | Me | F | CH₂[*c*-Hex(4-CF₃)] | Me |
| G-0895 | Me | F | CH₂CH₂(*c*-Pr) | Me |
| G-0896 | Me | F | CH₂CH₂(*c*-Hex) | Me |
| G-0897 | Me | F | CH₂CH₂[*c*-Hex(4-CF₃)] | Me |
| G-0898 | Me | F | CH₂CH₂[*c*-Hex(4-SCF₃)] | Me |
| G-0899 | Me | F | CH₂CH₂CH₂(*c*-Pr) | Me |
| G-0900 | Me | F | CH₂CH₂CH₂(*c*-Hex) | Me |
| G-0901 | Me | F | CH₂CH₂CH₂{*c*-Hex[4-(*t*-Bu)]} | Me |
| G-0902 | Me | F | CH₂CH₂CH₂[*c*-Hex(4-CF₃)] | Me |
| G-0903 | Me | F | CH₂CH₂CH₂[*c*-Hex(4-SCF₃)] | Me |
| G-0904 | Me | F | CH₂CH₂CH₂CH₂(*c*-Pr) | Me |
| G-0905 | Me | F | CH₂CH₂CH₂CH₂(*c*-Hex) | Me |
| G-0906 | Me | F | CH₂CH₂CH₂CH₂[*c*-Hex(4-CF₃)] | Me |
| G-0907 | Me | F | CH₂[*c*-Pr(2,2-F₂)] | Me |
| G-0908 | Me | F | CH₂[*c*-Hex(4,4-F₂)] | Me |
| G-0909 | Me | F | CH₂CH₂[*c*-Pr(2,2-F₂)] | Me |
| G-0910 | Me | F | CH₂CH₂[*c*-Hex(4,4-F₂)] | Me |
| G-0911 | Me | F | CH₂CH₂CH₂[*c*-Pr(2,2-F₂)] | Me |
| G-0912 | Me | F | CH₂CH₂CH₂CH₂[*c*-Pr(2,2-F₂) | Me |
| G-0913 | Me | F | CH₂CH₂OH | Me |
| G-0914 | Me | F | CH₂CH₂CH₂OH | Me |
| G-0915 | Me | F | CH₂CH₂CH₂CH₂OH | Me |
| G-0916 | Me | F | CH₂CH₂CH₂CH₂CH₂OH | Me |
| G-0917 | Me | F | CH₂CH₂CH₂CH₂CH₂CH₂OH | Me |
| G-0918 | Me | F | CH₂C(=O)Me | Me |

**[Table 1141]**

| Compound Number | R³ | R⁴ | R⁵ | R¹¹ |
|---|---|---|---|---|
| G-0919 | Me | F | CH₂CH(OH)Me | Me |
| G-0920 | Me | F | CH₂CH(OH)CF₃ | Me |
| G-0921 | Me | F | CH₂C(OH)₂CF₃ | Me |
| G-0922 | Me | F | CH₂C(CF₃)=NOH | Me |
| G-0923 | Me | F | CH₂C(CF₃)=NOMe | Me |
| G-0924 | Me | F | CH₂CH(CF₃)NH₂ | Me |
| G-0925 | Me | F | CH₂CH(CF₃)NHSO₂CF₃ | Me |
| G-0926 | Me | F | CH₂CH₂OMe | Me |
| G-0927 | Me | F | CH₂CH₂CH₂OMe | Me |
| G-0928 | Me | F | CH₂CH₂CH₂CH₂OMe | Me |
| G-0929 | Me | F | CH₂CH₂CH₂CH₂CH₂OMe | Me |
| G-0930 | Me | F | CH₂CH₂CH₂CH₂CH₂CH₂OMe | Me |
| G-0931 | Me | F | CH₂CH₂OCHF₂ | Me |
| G-0932 | Me | F | CH₂CH₂CH₂OCHF₂ | Me |
| G-0933 | Me | F | CH₂CH₂CH₂CH₂OCHF₂ | Me |
| G-0934 | Me | F | CH₂CH₂CH₂CH₂CH₂OCHF₂ | Me |
| G-0935 | Me | F | CH₂CH₂CH₂CH₂CH₂CH₂OCHF₂ | Me |
| G-0936 | Me | F | CH₂CH₂OCH₂CF₃ | Me |
| G-0937 | Me | F | CH₂CH₂CH₂OCH₂CF₃ | Me |
| G-0938 | Me | F | CH₂CH₂CH₂CH₂OCH₂CF₃ | Me |
| G-0939 | Me | F | CH₂CH₂CH₂CH₂CH₂OCH₂CF₃ | Me |
| G-0940 | Me | F | CH₂CH₂CH₂CH₂CH₂CH₂OCH₂CF₃ | Me |
| G-0941 | Me | F | CH₂CH₂CH₂CH₂CH₂CH₂CH₂OCH₂CF₃ | Me |
| G-0942 | Me | F | CH₂CH₂OC(CF₃)₃ | Me |
| G-0943 | Me | F | CH₂CH₂CH₂OC(CF₃)₃ | Me |
| G-0944 | Me | F | CH_{2C}H₂CH₂CH₂OC(CF₃)₃ | Me |
| G-0945 | Me | F | CH₂CH₂CH₂CH₂CH₂OC(CF₃)₃ | Me |
| G-0946 | Me | F | CF₂CHFOCF₂CF₂CF₃ | Me |
| G-0947 | Me | F | CH₂CH₂O(*c*-Pr) | Me |
| G-0948 | Me | F | CH₂CH₂O(*c*-Pen) | Me |
| G-0949 | Me | F | CH₂CH₂O(*c*-Hex) | Me |
| G-0950 | Me | F | CH₂CH₂CH₂O(*c*-Pr) | Me |
| G-0951 | Me | F | CH₂CH₂CH₂O(*c*-Pen) | Me |
| G-0952 | Me | F | CH₂CH₂CH₂O(*c*-Hex) | Me |
| G-0953 | Me | F | CH₂CH₂CH₂CH₂O(*c*-Pr) | Me |
| G-0954 | Me | F | CH₂CH₂CH₂CH₂O(*c*-Pen) | Me |
| G-0955 | Me | F | CH₂CH₂CH₂CH₂O(*c*-Hex) | Me |
| G-0956 | Me | F | CH₂CH₂O[*c*-Pr(2,2-F₂)] | Me |
| G-0957 | Me | F | CH₂CH₂CH₂O[*c*-Pr(2,2-F₂)] | Me |
| G-0958 | Me | F | CH₂CH₂CH₂CH₂O[*c*-Pr(2,2-F₂)] | Me |
| G-0959 | Me | F | CH₂CH₂OCH₂CH₂OCH₃ | Me |
| G-0960 | Me | F | CH₂CH₂OCH₂CH₂OCH₂CF₃ | Me |
| G-0961 | Me | F | CH₂CF₂OCF₂CF₂OCF₃ | Me |
| G-0962 | Me | F | CF₂CHFOCF₂CF₂OCF₃ | Me |

**[Table 1142]**

| Compound Number | R³ | R⁴ | R⁵ | R¹¹ |
|---|---|---|---|---|
| G-0963 | Me | F | CF₂CHFOCF₂CF(CF₃)OCF₂CF₂CF₃ | Me |
| G-0964 | Me | F | CH₂CH₂SH | Me |
| G-0965 | Me | F | CH₂CH₂CH₂SH | Me |
| G-0966 | Me | F | CH₂CH₂CH₂CH₂SH | Me |
| G-0967 | Me | F | CH₂CH₂CH₂CH₂CH₂SH | Me |
| G-0968 | Me | F | CH₂CH₂CH₂CH₂CH₂CH₂SH | Me |
| G-0969 | Me | F | CH₂CH₂S(t-Bu) | Me |
| G-0970 | Me | F | CH₂CH₂CH₂S(t-Bu) | Me |
| G-0971 | Me | F | CH₂CH₂CH₂CH₂S(t-Bu) | Me |
| G-0972 | Me | F | CH₂CH₂CH₂CH₂CH₂SCH₃ | Me |
| G-0973 | Me | F | CH₂CH₂CH₂CH₂CH₂SCH(CH₃)₂ | Me |
| G-0974 | Me | F | CH₂CH₂CH₂CH₂CH₂S(*t*-Bu) | Me |
| G-0975 | Me | F | CH₂CH₂CH₂CH₂CH₂CH₂SCH(CH₃)₂ | Me |
| G-0976 | Me | F | CH₂CH₂CH₂CH₂CH₂CH₂S(*t*-Bu) | Me |
| G-0977 | Me | F | CH₂CH₂CH₂CH₂CH₂CH₂CH₂S(*t*-Bu) | Me |
| G-0978 | Me | F | CH₂CH₂SCHF₂ | Me |
| G-0979 | Me | F | CH₂CH₂CH₂SCHF₂ | Me |
| G-0980 | Me | F | CH₂CH₂CH₂CH₂SCHF₂ | Me |
| G-0981 | Me | F | CH₂CH₂CH₂CH₂CH₂SCHF₂ | Me |
| G-0982 | Me | F | CH₂CH₂CH₂CH₂CH₂CH₂SCHF₂ | Me |
| G-0983 | Me | F | CH₂CH₂CH₂CH₂CH₂CH₂CH₂SCHF₂ | Me |
| G-0984 | Me | F | CH₂CH₂SCF₃ | Me |
| G-0985 | Me | F | CH₂CH₂CH₂SCF₃ | Me |
| G-0986 | Me | F | CH₂CH₂CH₂CH₂SCF₃ | Me |
| G-0987 | Me | F | CH₂CH₂CH₂CH₂CH₂SCF₃ | Me |
| G-0988 | Me | F | CH₂CH₂CH₂CH₂CH₂CH₂SCF₃ | Me |
| G-0989 | Me | F | CH₂CH₂CH₂CH₂CH₂CH₂CH₂SCF₃ | Me |
| G-0990 | Me | F | CH₂CH₂CH₂CH₂CH₂CH₂CH₂CH₂SCF₃ | Me |
| G-0991 | Me | F | CH₂CH(Me)SCF₃ | Me |
| G-0992 | Me | F | CH(Me)CH₂SCF₃ | Me |
| G-0993 | Me | F | CH₂CH₂CH(Me)SCF₃ | Me |
| G-0994 | Me | F | CH₂CH(Me)CH₂SCF₃ | Me |
| G-0995 | Me | F | CH(Me)CH₂CH₂SCF₃ | Me |
| G-0996 | Me | F | CH₂CH₂CH₂CH(Me)SCF₃ | Me |
| G-0997 | Me | F | CH₂CH₂CH(Me)CH₂SCF₃ | Me |
| G-0998 | Me | F | CH₂CH(Me)CH₂CH₂SCF₃ | Me |
| G-0999 | Me | F | CH(Me)CH₂CH₂CH₂SCF₃ | Me |
| G-1000 | Me | F | CH₂CH₂SCH₂CF₃ | Me |
| G-1001 | Me | F | CH₂CH₂CH₂SCH₂CF₃ | Me |
| G-1002 | Me | F | CH₂CH₂CH₂CH₂SCH₂CF₃ | Me |
| G-1003 | Me | F | CH₂CH₂CH₂CH₂CH₂SCH₂CF₃ | Me |
| G-1004 | Me | F | CH₂CH₂CH₂CH₂CH₂CH₂SCH₂CF₃ | Me |
| G-1005 | Me | F | CH₂CH₂CH₂CH₂CH₂CH₂CH₂SCH₂CF₃ | Me |
| G-1006 | Me | F | CH₂CH₂SCH(CF₃)₂ | Me |

**[Table 1143]**

| Compound Number | R³ | R⁴ | R⁵ | R¹¹ |
|---|---|---|---|---|
| G-1007 | Me | F | CH₂CH₂CH₂SCH(CF₃)₂ | Me |
| G-1008 | Me | F | CH₂CH₂CH₂CH₂SCH(CF₃)₂ | Me |
| G-1009 | Me | F | CH₂CH₂CH₂CH₂CH₂SCH(CF₃)₂ | Me |
| G-1010 | Me | F | CH₂CH₂CH₂CH₂CH₂CH₂SCH(CF₃)₂ | Me |
| G-1011 | Me | F | CH₂CH₂CH₂CH₂CH₂SCF₂CF₃ | Me |
| G-1012 | Me | F | CH₂CH₂CH₂CH₂CH_{2C}H₂SCF₂CF₃ | Me |
| G-1013 | Me | F | CH₂CH₂CH₂CH₂CH₂CH₂SCF(CF₃)₂ | Me |
| G-1014 | Me | F | CH₂CH₂CH₂CH₂CH₂CH₂CH₂SCF(CF₃)₂ | Me |
| G-1015 | Me | F | CH₂CH₂CH₂CH₂CH₂SCCl₃ | Me |
| G-1016 | Me | F | CH₂CH₂CH₂CH₂CH₂CH₂SCCl₃ | Me |
| G-1017 | Me | F | CH₂CH₂SCH₂CH=CH₂ | Me |
| G-1018 | Me | F | CH₂CH₂CH₂SCH₂CH=CH₂ | Me |
| G-1019 | Me | F | CH₂CH_{2C}H₂CH₂SCH₂CH=CH₂ | Me |
| G-1020 | Me | F | CH₂CH₂SCF=CFCF₃ | Me |
| G-1021 | Me | F | CH₂CH₂CH₂SCF=CFCF₃ | Me |
| G-1022 | Me | F | CH₂CH₂CH₂CH₂SCF=CFCF₃ | Me |
| G-1023 | Me | F | CH₂CH₂CH₂S(*c*-Pr) | Me |
| G-1024 | Me | F | CH₂CH₂CH₂S(*c*-Hex) | Me |
| G-1025 | Me | F | CH₂CH₂CH₂CH₂S(*c*-Pr) | Me |
| G-1026 | Me | F | CH₂CH₂CH₂CH₂S(*c*-Hex) | Me |
| G-1027 | Me | F | CH₂CH₂CH₂CH₂CH₂S(*c*-Pr) | Me |
| G-1028 | Me | F | CH₂CH₂CH₂CH₂CH₂S(*c*-Hex) | Me |
| G-1029 | Me | F | CH₂CH₂CH₂S[*c*-Hex(4,4-F₂)] | Me |
| G-1030 | Me | F | CH₂CH₂CH₂cH₂S[*c*-Hex(4,4-F₂)] | Me |
| G-1031 | Me | F | CH₂CH₂CH₂CH₂CH₂S[*c*-Hex(4,4-F₂)] | Me |
| G-1032 | Me | F | CH₂CH₂CH₂SCH₂(*c*-Pr) | Me |
| G-1033 | Me | F | CH₂CH₂CH₂SCH₂(*c*-Hex) | Me |
| G-1034 | Me | F | CH₂CH₂CH₂CH₂SCH₂(*c*-Pr) | Me |
| G-1035 | Me | F | CH₂CH₂CH₂CH₂SCH₂(*c*-Hex) | Me |
| G-1036 | Me | F | CH₂CH₂CH₂CH₂CH₂SCH₂(*c*-Pr) | Me |
| G-1037 | Me | F | CH₂CH₂CH₂CH₂CH₂SCH₂(*c*-Hex) | Me |
| G-1038 | Me | F | CH₂CH₂CH₂SCH₂[*c*-Hex(4,4-F₂)] | Me |
| G-1039 | Me | F | CH₂CH₂CH₂CH₂SCH₂[*c*-Hex(4,4-F₂)] | Me |
| G-1040 | Me | F | CH₂CH₂CH₂CH₂CH₂SCH₂[C-Hex(4,4-F₂)] | Me |
| G-1041 | Me | F | CH₂CH₂CH₂SCH₂SiMe₃ | Me |
| G-1042 | Me | F | CH₂CH₂CH₂CH₂SCH₂SiMe₃ | Me |
| G-1043 | Me | F | CH₂CH₂CH₂CH₂CH₂SCH₂SiMe₃ | Me |
| G-1044 | Me | F | CH₂CH₂OCH₂CH₂SCH₃ | Me |
| G-1045 | Me | F | CH₂CH₂OCH₂CH₂SCHF₂ | Me |
| G-1046 | Me | F | CH₂CH₂OCH₂CH₂SCF₃ | Me |
| G-1047 | Me | F | CH₂CH₂OCH₂CH₂SCH₂CF₃ | Me |
| G-1048 | Me | F | CH₂CH₂CH₂S(=O)(*t*-Bu) | Me |
| G-1049 | Me | F | CH₂CH₂CH₂CH₂S(=O)(*t*-Bu) | Me |
| G-1050 | Me | F | CH₂CH₂CH₂CH₂CH₂CH₂S(=O)(*t*-Bu) | Me |

**[Table 1144]**

| Compound Number | R³ | R⁴ | R⁵ | R11 |
|---|---|---|---|---|
| G-1051 | Me | F | CH₂CH₂S(=O)CHF₂ | Me |
| G-1052 | Me | F | CH₂CH₂CH₂S(=O)CHF₂ | Me |
| G-1053 | Me | F | CH₂CH₂CH₂CH₂S(=O)CHF₂ | Me |
| G-1054 | Me | F | CH₂CH₂CH₂CH₂CH₂S(=O)CHF₂ | Me |
| G-1055 | Me | F | CH₂CH₂CH₂CH₂CH₂CH₂S(=O)CHF₂ | Me |
| G-1056 | Me | F | CH₂CH₂CH₂CH₂CH₂CH₂CH₂S(=O)CHF₂ | Me |
| G-1057 | Me | F | CH₂CH₂S(=O)CF₃ | Me |
| G-1058 | Me | F | CH₂CH₂CH₂S(=O)CF₃ | Me |
| G-1059 | Me | F | CH₂CH₂CH₂CH₂S(=O)CF₃ | Me |
| G-1060 | Me | F | CH₂CH₂CH₂CH₂CH₂S(=O)CF₃ | Me |
| G-1061 | Me | F | CH₂CH₂CH₂CH₂CH₂CH₂S(=O)CF₃ | Me |
| G-1062 | Me | F | CH₂CH₂CH₂CH₂CH₂CH₂CH₂S(=O)CF₃ | Me |
| G-1063 | Me | F | CH₂CH₂S(=O)CH₂CF₃ | Me |
| G-1064 | Me | F | CH₂CH₂CH₂S(=O)CH₂CF₃ | Me |
| G-1065 | Me | F | CH₂CH₂CH₂CH₂S(=O)CH₂CF₃ | Me |
| G-1066 | Me | F | CH₂CH₂CH₂CH₂CH₂S(=O)CH₂CF₃ | Me |
| G-1067 | Me | F | CH₂CH₂CH₂CH₂CH₂CH₂S(=O)CH₂CF₃ | Me |
| G-1068 | Me | F | CH₂CH₂CH₂CH₂CH₂CH₂CH₂S(=O)CH₂CF₃ | Me |
| G-1069 | Me | F | CH₂CH₂CH₂S(=O)(*c*-Pr) | Me |
| G-1070 | Me | F | CH₂CH₂CH₂CH₂S(=O)(*c*-Pr) | Me |
| G-1071 | Me | F | CH₂CH₂CH₂CH₂S(=O)(*c*-Pen) | Me |
| G-1072 | Me | F | CH₂CH₂CH₂CH₂S(=O)(*c*-Hex) | Me |
| G-1073 | Me | F | CH₂CH₂CH₂CH₂CH₂S(=O)(*c*-Pr) | Me |
| G-1074 | Me | F | CH₂CH₂CH_{2C}H₂CH₂S(=O)(*c*-Hex) | Me |
| G-1075 | Me | F | CH₂CH₂CH₂S(=O)[*c*-Hex(4,4-F₂)] | Me |
| G-1076 | Me | F | CH₂CH₂CH₂CH₂S(=O)[*c*-Hex(4,4-F₂)] | Me |
| G-1077 | Me | F | CH₂CH₂CH₂CH₂CH₂S(=O)[c-Hex(4,4-F₂)] | Me |
| G-1078 | Me | F | CH₂CH₂CH₂CH₂S(=O)₂Me | Me |
| G-1079 | Me | F | CH₂CH₂CH₂CH₂CH₂S(=O)₂Me | Me |
| G-1080 | Me | F | CH₂CH₂CH₂CH₂CH₂S(=O)₂(*t*-Bu) | Me |
| G-1081 | Me | F | CH₂CH₂S(=O)₂CHF₂ | Me |
| G-1082 | Me | F | CH₂CH₂CH₂S(=O)₂CHF₂ | Me |
| G-1083 | Me | F | CH₂CH₂CH₂CH₂S(=O)₂CHF₂ | Me |
| G-1084 | Me | F | CH₂CH₂CH₂CH₂CH₂S(=O)₂CHF₂ | Me |
| G-1085 | Me | F | CH₂CH₂CH₂CH₂CH₂CH₂S(=O)₂CHF₂ | Me |
| G-1086 | Me | F | CH₂CH₂S(=O)₂CF₃ | Me |
| G-1087 | Me | F | CH₂CH₂CH₂S(=O)₂CF₃ | Me |
| G-1088 | Me | F | CH₂CH₂CH₂CH₂S(=O)₂CF₃ | Me |
| G-1089 | Me | F | CH₂CH₂CH₂CH₂CH₂S(=O)₂CF₃ | Me |
| G-1090 | Me | F | CH₂CH₂CH₂CH₂CH₂CH₂S(=O)₂CF₃ | Me |
| G-1091 | Me | F | CH₂CH₂CH₂CH₂CH₂CH₂CH₂S(=O)₂CF₃ | Me |
| G-1092 | Me | F | CH₂CH₂CH₂CH₂CH₂CH₂CH₂CH₂S(=O)₂CF₃ | Me |
| G-1093 | Me | F | CH₂CH₂S(=O)₂CH₂CF₃ | Me |
| G-1094 | Me | F | CH₂CH₂CH₂S(=O)₂CH₂CF₃ | Me |

**[Table 1145]**

| Compound Number | R³ | R⁴ | R⁵ | R¹¹ |
|---|---|---|---|---|
| G-1095 | Me | F | CH₂CH₂CH₂CH₂S(=O)₂CH₂CF₃ | Me |
| G-1096 | Me | F | CH₂CH₂CH₂CH₂CH₂S(=O)₂CH₂CF₃ | Me |
| G-1097 | Me | F | CH₂CH₂CH₂CH₂CH₂CH₂S(=O)₂CH₂CF₃ | Me |
| G-1098 | Me | F | CH₂CH₂CH₂S(=O)₂(*c*-Pr) | Me |
| G-1099 | Me | F | CH₂CH₂CH₂CH₂S(=O)₂(*c*-Pr) | Me |
| G-1100 | Me | F | CH₂CH₂CH₂CH₂CH₂S(=O)₂(*c*-Pr) | Me |
| G-1101 | Me | F | CH₂CH₂CH₂S(=O)₂[*c*-Hex(4,4-F₂)] | Me |
| G-1102 | Me | F | CH₂CH₂CH₂CH₂S(=O)_{2[}c-Hex(4,4-F₂)] | Me |
| G-1103 | Me | F | CH₂CH₂CH₂CH₂CH₂S(=O)₂[*c*-Hex(4,4-F₂)] | Me |
| G-1104 | Me | F | CH₂CH₂CH₂C(=O)H | Me |
| G-1105 | Me | F | CH₂CH₂CH₂CH₂C(=O)H | Me |
| G-1106 | Me | F | CH₂CH₂CH₂CH₂CH₂C(=O)H | Me |
| G-1107 | Me | F | CH₂CH₂CH₂C(=O)(*t*-Bu) | Me |
| G-1108 | Me | F | CH₂CH₂CH₂CH₂C(=O)(*t*-Bu) | Me |
| G-1109 | Me | F | CH₂CH₂CH₂CH₂CH₂C(=O)(*t*-Bu) | Me |
| G-1110 | Me | F | CH₂CH₂CH₂C(=O)CF₃ | Me |
| G-1111 | Me | F | CH₂CH₂CH₂CH₂C(=O)CF₃ | Me |
| G-1112 | Me | F | CH₂CH₂CH₂CH₂CH₂C(=O)CF₃ | Me |
| G-1113 | Me | F | CH₂C(=O)Ph | Me |
| G-1114 | Me | F | CH₂C(=O)Ph(4-Cl) | Me |
| G-1115 | Me | F | CH₂C(=O)Ph(4-CF₃) | Me |
| G-1116 | Me | F | CH₂CH₂C(=O)Ph(4-CF₃) | Me |
| G-1117 | Me | F | CH₂CH₂CH₂C(=O)Ph(4-CF₃) | Me |
| G-1118 | Me | F | CH₂CH₂CH₂CH₂C(=O)Ph(4-CF₃) | Me |
| G-1119 | Me | F | CH₂CH₂CH₂CH₂CH₂C(=O)Ph(4-CF₃) | Me |
| G-1120 | Me | F | CH₂C(=O)OEt | Me |
| G-1121 | Me | F | CH₂CH₂C(=O)O(*t*-Bu) | Me |
| G-1122 | Me | F | CH₂CH₂CH₂C(=O)OEt | Me |
| G-1123 | Me | F | CH₂CH₂CH₂C(=O)O(*t*-Bu) | Me |
| G-1124 | Me | F | CH₂CH₂CH₂CH₂C(=O)O(*t*-Bu) | Me |
| G-1125 | Me | F | CH₂CH₂CH₂CH₂CH₂C(=O)O(*^{t}*-Bu) | Me |
| G-1126 | Me | F | CH₂CH₂CH₂CH₂CH₂CH₂C(=O)O(*t*-Bu) | Me |
| G-1127 | Me | F | CH₂CH₂CH₂CH₂CH₂CH₂CH₂C(=O)OEt | Me |
| G-1128 | Me | F | CH₂CH₂C(=O)OCH₂CF₃ | Me |
| G-1129 | Me | F | CH₂CH₂CH₂C(=O)OCH₂CF₃ | Me |
| G-1130 | Me | F | CH₂CH₂CH₂CH₂C(=O)OCH₂CF₃ | Me |
| G-1131 | Me | F | CH₂CH₂CH₂CH₂CH₂C(=O)OCH₂CF₃ | Me |
| G-1132 | Me | F | CH₂CH₂C(=O)NH(*t*-Bu) | Me |
| G-1133 | Me | F | CH₂CH₂CH₂C(=O)NH(*t*-Bu) | Me |
| G-1134 | Me | F | CH₂CH₂CH₂C(=O)NH(*t*-Pen) | Me |
| G-1135 | Me | F | CH2CH2CH2CH2C(=O)NH(*t*-Bu) | Me |
| G-1136 | Me | F | CH₂CH₂C(=O)NHCH₂CF₃ | Me |
| G-1137 | Me | F | CH₂CH₂CH₂C(=O)NHCH₂CF₃ | Me |
| G-1138 | Me | F | CH₂CH₂CH₂CH₂C(=O)NHCH₂CF₃ | Me |

**[Table 1146]**

| Compound Number | R³ | R⁴ | R⁵ | R¹¹ |
|---|---|---|---|---|
| G-1139 | Me | F | CH₂CH₂SC(=O)N(Me)₂ | Me |
| G-1140 | Me | F | CH₂CH₂CH₂SC(=O)N(Me)₂ | Me |
| G-1141 | Me | F | CH₂CH₂CH₂SC(=O)NH(*t*-Bu) | Me |
| G-1142 | Me | F | CH₂CH₂CH₂CH₂CH₂SC(=O)NH(*t*-Bu) | Me |
| G-1143 | Me | F | CH₂CH₂SC(=O)NHCH₂CF₃ | Me |
| G-1144 | Me | F | CH₂CH₂CH₂SC(=O)NHCH₂CF₃ | Me |
| G-1145 | Me | F | CH₂CH₂CH₂CH₂SC(=O)NHCH₂CF₃ | Me |
| G-1146 | Me | F | CH₂CH₂CH₂OC(=O)H | Me |
| G-1147 | Me | F | CH₂CH₂CH₂CH₂OC(=O)H | Me |
| G-1148 | Me | F | CH₂CH₂CH₂CH₂CH₂OC(=O)H | Me |
| G-1149 | Me | F | CH₂CH₂CH₂OC(=O)Me | Me |
| G-1150 | Me | F | CH₂CH₂CH₂CH₂OC(=O)Me | Me |
| G-1151 | Me | F | CH₂CH₂CH₂CH₂CH₂OC(=O)Me | Me |
| G-1152 | Me | F | CH₂CH₂OC(=O)CF₃ | Me |
| G-1153 | Me | F | CH₂CH₂CH₂OC(=O)CF₃ | Me |
| G-1154 | Me | F | CH₂CH₂CH₂CH₂OC(=O)CF₃ | Me |
| G-1155 | Me | F | CH₂CH₂CH₂CH₂CH₂OC(=O)Ph(4-CF₃) | Me |
| G-1156 | Me | F | CH₂CH₂OC(=O)Ph | Me |
| G-1157 | Me | F | CH₂CH₂OC(=O)Ph(4-CF₃) | Me |
| G-1158 | Me | F | CH₂CH₂CH₂OC(=O)Ph(4-CF₃) | Me |
| G-1159 | Me | F | CH₂CH₂CH₂CH₂OC(=O)Ph(4-CF₃) | Me |
| G-1160 | Me | F | CH₂CH₂CH₂CH₂CH₂OC(=O)Ph(4-CF₃) | Me |
| G-1161 | Me | F | CH₂CH₂OS(=O)Me | Me |
| G-1162 | Me | F | CH₂CH₂CH₂OS(=O)Me | Me |
| G-1163 | Me | F | CH₂CH₂CH₂CH₂OS(=O)Me | Me |
| G-1164 | Me | F | CH₂CH₂CH₂CH₂CH₂OS(=O)Me | Me |
| G-1165 | Me | F | CH₂CH₂CH₂CH₂CH₂CH₂OS(=O)Me | Me |
| G-1166 | Me | F | CH₂CH₂OS(=O)CF₃ | Me |
| G-1167 | Me | F | CH₂CH₂CH₂OS(=O)CF₃ | Me |
| G-1168 | Me | F | CH₂CH₂CH₂CH₂OS(=O)CF₃ | Me |
| G-1169 | Me | F | CH₂CH₂CH₂CH₂CH₂OS(=O)CF₃ | Me |
| G-1170 | Me | F | CH₂CH₂CH₂CH_{z2}CH₂CH₂OS(=O)CF₃ | Me |
| G-1171 | Me | F | CH₂CH₂OS(=O)Ph | Me |
| G-1172 | Me | F | CH₂CH₂OS(=O)Ph(4-Me) | Me |
| G-1173 | Me | F | CH₂CH₂CH₂OS(=O)Ph | Me |
| G-1174 | Me | F | CH₂CH₂CH₂OS(=O)Ph(4-Me) | Me |
| G-1175 | Me | F | CH₂CH₂OS(=O)₂Me | Me |
| G-1176 | Me | F | CH₂CH₂CH₂OS(=O)₂Me | Me |
| G-1177 | Me | F | CH₂CH₂CH₂OS(=O)₂CF₃ | Me |
| G-1178 | Me | F | CH₂CH₂CH₂CH₂OS(=O)₂CF₃ | Me |
| G-1179 | Me | F | CH₂CH₂CH₂CH₂CH₂OS(=O)₂CF₃ | Me |
| G-1180 | Me | F | CH₂CH₂CH₂CH₂CH₂OS(=O)₂CF₂CF₂CF₂CF₃ | Me |
| G-1181 | Me | F | CH₂CH₂OS(=O)₂Ph | Me |
| G-1182 | Me | F | CH₂CH₂OS(=O)₂Ph(4-Me) | Me |

**[Table 1147]**

| Compound Number | R³ | R⁴ | R⁵ | R¹¹ |
|---|---|---|---|---|
| G-1183 | Me | F | CH₂CH₂CH₂OS(=O)₂Ph | Me |
| G-1184 | Me | F | CH₂CH₂CH₂OS(=O)₂Ph(4-Me) | Me |
| G-1185 | Me | F | CH₂CH₂NH₂ | Me |
| G-1186 | Me | F | CH₂CH₂NH₂, HCl | Me |
| G-1187 | Me | F | CH₂CH₂CH₂NH₂ | Me |
| G-1188 | Me | F | CH₂CH₂CH₂CH₂NH₂ | Me |
| G-1189 | Me | F | CH₂CH₂CH₂CH₂CH₂NH₂ | Me |
| G-1190 | Me | F | CH₂CH₂CH₂CH₂CH₂CH₂NH₂ | Me |
| G-1191 | Me | F | CH₂CH₂CH₂CH₂CH₂CH₂CH₂NH₂ | Me |
| G-1192 | Me | F | CH₂CH(Me)NH₂ | Me |
| G-1193 | Me | F | CH₂C*H{Me)NH₂,HCl | Me |
| G-1194 | Me | F | CH₂C*H(Me)NH₂: (R) | Me |
| G-1195 | Me | F | CH₂C*H(Me)NH₂,HCl: (R) | Me |
| G-1196 | Me | F | CH₂C*H(Me)NH₂: (S) | Me |
| G-1197 | Me | F | CH₂C*H(Me)NH₂,HCl: (S) | Me |
| G-1198 | Me | F | CH₂CH(Et)NH₂ | Me |
| G-1199 | Me | F | CH₂CH(*i*-Pr)NH₂ | Me |
| G-1200 | Me | F | CH(Me)CH₂NH₂ | Me |
| G-1201 | Me | F | CH(Me)CH₂NH₂, HCl | Me |
| G-1202 | Me | F | CH(Et)CH₂NH₂ | Me |
| G-1203 | Me | F | CH(*i*-Pr)CH₂NH₂ | Me |
| G-1204 | Me | F | CH₂CH₂CH(Me)NH₂ | Me |
| G-1205 | Me | F | CH₂CH(Me)CH₂NH₂ | Me |
| G-1206 | Me | F | CH(Me)CH₂CH₂NH₂ | Me |
| G-1207 | Me | F | CH₂CH(Me)NH(*c*-Pr) | Me |
| G-1208 | Me | F | CH₂CH(Me)N(*c*-Pr)S(=O)₂CF₃ | Me |
| G-1209 | Me | F | CH₂CH(Me)NHOMe | Me |
| G-1210 | Me | F | CH₂CH(Me)N(OMe)S(=O)₂CF₃ | Me |
| G-1211 | Me | F | CH₂CH(Me)N(OMe)S(=O)₂Ph(2-NO₂) | Me |
| G-1212 | Me | F | CH₂CH(Me)N(OMe)S(=O)₂Ph(4-NO₂) | Me |
| G-1213 | Me | F | CH₂CH₂N(Me)(*t*-Bu) | Me |
| G-1214 | Me | F | CH₂CH₂CH₂N(Me)(*t*-Bu) | Me |
| G-1215 | Me | F | CH₂CH₂CH₂CH₂N(Me)(*t*-Bu) | Me |
| G-1216 | Me | F | CH₂CH₂NHCH₂CF₃ | Me |
| G-1217 | Me | F | CH₂CH₂CH₂NHCH₂CF₃ | Me |
| G-1218 | Me | F | CH₂CH₂CH₂CH₂NHCH₂CF₃ | Me |
| G-1219 | Me | F | CH₂CH₂NHC(=O)C(Me)(CF₃)₂ | Me |
| G-1220 | Me | F | CH₂CH₂NHC(=O)Me | Me |
| G-1221 | Me | F | CH₂CH₂NHC(=O)(*t*-Bu) | Me |
| G-1222 | Me | F | CH₂CH₂CH₂CH₂NHC(=O)CH(CH₃)₂ | Me |
| G-1223 | Me | F | CH2CH2CH2CH2NHC(=O)(*t*-Bu) | Me |
| G-1224 | Me | F | CH₂CH₂CH₂CH₂NHC(=O)CH₂(*t*-Bu) | Me |
| G-1225 | Me | F | CH₂CH₂NHC(=O)CF₃ | Me |
| G-1226 | Me | F | CH₂CH₂NHC(=O)CH₂CF₃ | Me |

**[Table 1148]**

| Compound Number | R³ | R⁴ | R⁵ | R¹¹ |
|---|---|---|---|---|
| G-1227 | Me | F | CH₂CH₂NHC(=O)CF₂CF₃ | Me |
| G-1228 | Me | F | CH₂CH₂CH₂NHC(=O)CF₃ | Me |
| G-1229 | Me | F | CH₂CH₂CH₂NHC(=O)CH₂CF₃ | Me |
| G-1 230 | Me | F | CH₂CH₂CH₂NHC(=O)CF₂CF₃ | Me |
| G-1231 | Me | F | CH₂CH₂CH₂CH₂NHC(=O)CF₃ | Me |
| G-1232 | Me | F | CH₂CH₂CH₂CH₂NHC(=O)CH₂CF₃ | Me |
| G-1233 | Me | F | CH₂CH₂CH₂CH₂NHC(=O)CF₂CF₃ | Me |
| G-1234 | Me | F | CH₂CH₂CH₂CH₂NHC(=O)CF(CF₃)₂ | Me |
| G-1235 | Me | F | CH₂CH₂CH₂CH₂NHC(=O)CCl₃ | Me |
| G-1236 | Me | F | CH₂CH₂NHC(=O)Ph | Me |
| G-1237 | Me | F | CH₂CH₂NHC(=O)Ph(4-CF₃) | Me |
| G-1238 | Me | F | CH₂CH₂CH₂NHC(=O)Ph(4-CF₃) | Me |
| G-1239 | Me | F | CH₂CH₂CH₂CH₂NHC=O)Ph(4-CF₃) | Me |
| G-1240 | Me | F | CH₂CH₂NHC(=O)O(*t*-Bu) | Me |
| G-1241 | Me | F | CH₂CH₂CH₂NHC(=O)O(*t*-Bu) | Me |
| G-1242 | Me | F | CH₂CH₂CH₂CH₂NHC(=O)O(*t*-Bu) | Me |
| G-1243 | Me | F | CH₂CH₂CH₂CH₂NHC(=O)OCH(CH₃)₂ | Me |
| G-1244 | Me | F | CH₂CH(Me)NHC(=O)O(*t*-Bu) | Me |
| G-1245 | Me | F | CH₂C*H(Me)NHC(=O)O(*t*-Bu) : (R) | Me |
| G-1246 | Me | F | CH₂C*H(Me)NHC(=O)O(*t*-Bu) : (S) | Me |
| G-1247 | Me | F | CH₂CH(Et)NHC(=O)O(*t*-Bu) | Me |
| G-1248 | Me | F | CH₂CH(*i*-Pr)NHC(=O)O(*t*-Bu) | Me |
| G-1249 | Me | F | CH(Me)CH₂NHC(=O)O(*t*-Bu) | Me |
| G-1250 | Me | F | CH(Et)CH₂NHC(=O)O(*t*-Bu) | Me |
| G-1251 | Me | F | CH(*i*-Pr)CH₂NHC(=O)O(*t*-Bu) | Me |
| G-1252 | Me | F | CH₂CH(Me)CH₂NHC(=O)O(*t*-Bu) | Me |
| G-1253 | Me | F | CH₂CH₂NHC(=O)OC(Me)₂CF₃ | Me |
| G-1254 | Me | F | CH₂CH₂CH₂NHC(=O)OCH₂CF₃ | Me |
| G-1255 | Me | F | CH₂CH₂CH₂NHC(=O)OCH₂CH₂CF₃ | Me |
| G-1256 | Me | F | CH₂CH₂CH₂cH₂NHC(=O)OCH₂CCl₃ | Me |
| G-1257 | Me | F | CH₂CH₂CH₂CH₂NHC(=O)OCH₂CF₃ | Me |
| G-1258 | Me | F | CH₂CH₂NHC(=O)NH(*t*-Bu) | Me |
| G-1259 | Me | F | CH₂CH₂CH₂NHC(=O)NH(*t*-Bu) | Me |
| G-1260 | Me | F | CH₂CH₂CH₂CH₂NHC(=O)NHEt | Me |
| G-1261 | Me | F | CH₂CH₂CH₂CH₂NHC(=O)NH(*t*-Bu) | Me |
| G-1262 | Me | F | CH₂CH₂CH₂NHC(=O)NHCH₂CCl₃ | Me |
| G-1263 | Me | F | CH₂CH₂CH₂NHC(=O)NHCH₂CH₂F | Me |
| G-1264 | Me | F | CH₂CH₂CH₂NHC(=O)NHCH₂CF₃ | Me |
| G-1265 | Me | F | CH₂CH₂CH₂CH₂NHC(=O)NHCH₂CCl₃ | Me |
| G-1266 | Me | F | CH₂CH₂CH₂CH₂NHC(=O)NHCH₂CH₂F | Me |
| G-1267 | Me | F | CH₂CH₂CH₂CH₂NHC(=O)NHCH₂CF₃ | Me |
| G-1268 | Me | F | CH₂CH₂NHS(=O)Me | Me |
| G-1269 | Me | F | CH₂CH₂CH₂NHS(=O)Me | Me |
| G-1270 | Me | F | CH₂CH₂CH₂CH₂NHS(=O)Me | Me |

**[Table 1149]**

| Compound Number | R³ | R⁴ | R⁵ | R¹¹ |
|---|---|---|---|---|
| G-1271 | Me | F | CH₂CH₂CH₂CH₂NHS(=O)CH(CH₃)₂ | Me |
| G-1272 | Me | F | CH₂CH₂CH₂CH₂CH₂NHS(=O)Me | Me |
| G-1273 | Me | F | CH₂CH₂NHS(=O)CHF₂ | Me |
| G-1274 | Me | F | CH₂CH₂CH₂NHS(=O)CHF₂ | Me |
| G-1275 | Me | F | CH₂CH₂CH₂CH₂NHS(=O)CHF₂ | Me |
| G-1276 | Me | F | CH₂CH₂CH₂CH₂CH₂NHS(=O)CHF₂ | Me |
| G-1277 | Me | F | CH₂CH(Me)NHS(=O)CHF₂ | Me |
| G-1278 | Me | F | CH(Me)CH₂NHS(=O)CHF₂ | Me |
| G-1279 | Me | F | CH₂CH₂CH(Me)NHS(=O)CHF₂ | Me |
| G-1280 | Me | F | CH₂CH(Me)CH₂NHS(=O)CHF₂ | Me |
| G-1281 | Me | F | CH(Me)CH_{2C}H₂NHS(=O)CHF₂ | Me |
| G-1282 | Me | F | CH₂CH₂NHS(=O)CF₃ | Me |
| G-1283 | Me | F | CH₂CH₂CH₂NHS(=O)CF₃ | Me |
| G-1284 | Me | F | CH₂CH₂CH₂CH₂NHS(=O)CF₃ | Me |
| G-1285 | Me | F | CH₂CH₂CH₂CH₂CH₂NHS(=O)CF₃ | Me |
| G-1286 | Me | F | CH₂CH₂CH₂NHS(=O)Ph | Me |
| G-1287 | Me | F | CH₂CH₂CH₂CH₂NHS(=O)Ph | Me |
| G-1288 | Me | F | CH₂CH₂CH₂CH₂CH₂NHS(=O)Ph | Me |
| G-1289 | Me | F | CH₂CH₂CH₂CH₂CH₂NHS(=O)Ph(4-CF₃) | Me |
| G-1290 | Me | F | CH₂CH(Me)NHS(=O)Ph(4-CF₃) | Me |
| G-1291 | Me | F | CH₂CH(Et)NHS(=O)Ph(4-CF₃) | Me |
| G-1292 | Me | F | CH(Me)CH₂NHS(=O)Ph(4-CF₃) | Me |
| G-1293 | Me | F | CH(Et)CH₂NHS(=O)Ph(4-CF₃) | Me |
| G-1294 | Me | F | CH₂CH(Et)CH₂NHS(=O)Ph(4-CF₃) | Me |
| G-1295 | Me | F | CH₂CH₂NHS(=O)₂Me | Me |
| G-1296 | Me | F | CH₂CH₂CH₂NHS(=O)₂Me | Me |
| G-1297 | Me | F | CH₂CH₂CH₂CH₂NHS(=O)₂Me | Me |
| G-1298 | Me | F | CH₂CH₂CH₂CH₂NHS(=O)₂CH(CH₃)₂ | Me |
| G-1299 | Me | F | CH₂CH₂CH₂CH₂CH₂NHS(=O)₂Me | Me |
| G-1300 | Me | F | CH₂CH₂NHS(=O)₂CHF₂ | Me |
| G-1301 | Me | F | CH₂CH₂CH₂NHS(=O)₂CHF₂ | Me |
| G-1302 | Me | F | CH₂CH₂CH₂CH₂NHS(=O)₂CHF₂ | Me |
| G-1303 | Me | F | CH₂CH₂CH₂CH₂CH₂NHS(=O)₂CHF₂ | Me |
| G-1304 | Me | F | CH₂CH(Me)NHS(=O)₂CHF₂ | Me |
| G-1305 | Me | F | CH(Me)CH₂NHS(=O)₂CHF₂ | Me |
| G-1306 | Me | F | CH₂CH₂CH(Me)NHS(=O)₂CHF₂ | Me |
| G-1307 | Me | F | CH₂CH(Me)CH₂NHS(=O)₂CHF₂ | Me |
| G-1308 | Me | F | CH(Me)CH₂CH₂NHS(=O)₂CHF₂ | Me |
| G-1309 | Me | F | CH₂CH(Me)N(Me)S(=O)₂CHF₂ | Me |
| G-1310 | Me | F | CH(Me)CH₂N(Me)S(=OhCHF₂ | Me |
| G-1311 | Me | F | CH₂CH₂CH(Me)N(Me)S(=O)₂CHF₂ | Me |
| G-1312 | Me | F | CH₂CH(Me)CH₂N(Me)S(=O)₂CHF₂ | Me |
| G-1313 | Me | F | CH(Me)CH₂CH₂N(Me)S(=O)₂CHF₂ | Me |
| G-1314 | Me | F | CH₂CH₂NHS(=O)₂CF₃ | Me |

**[Table 1150]**

| Compound Number | R³ | R⁴ | R⁵ | R¹¹ |
|---|---|---|---|---|
| G-1315 | Me | F | CH₂CH₂CH₂NHS(=O)₂CF₃ | Me |
| G-1316 | Me | F | CH₂CH₂CH₂CH₂NHS(=O)₂CF₃ | Me |
| G-1317 | Me | F | CH₂CH₂CH₂CH₂CH₂NHS(=O)₂CF₃ | Me |
| G-1318 | Me | F | CH₂CH₂N(Me)S(=O)₂CF₃ | Me |
| G-1319 | Me | F | CH₂CH₂N(Et)S(=O)₂CF₃ | Me |
| G-1320 | Me | F | CH₂CH₂N(CH₂CN)S(=O)₂CF₃ | Me |
| G-1321 | Me | F | CH₂CH₂N(CH₂OMe)S(=O)₂CF₃ | Me |
| G-1322 | Me | F | CH₂CH₂CH₂N(Me)S(=O)₂CF₃ | Me |
| G-1323 | Me | F | CH₂CH₂CH₂N(Et)S(=O)₂CF₃ | Me |
| G-1324 | Me | F | CH₂CH₂CH₂N(CH₂CN)S(=O)₂CF₃ | Me |
| G-1325 | Me | F | CH₂CH₂CH₂N(CH₂OMe)S(=O)₂CF₃ | Me |
| G-1326 | Me | F | CH₂CH₂CH₂CH₂N(Me)S(=O)₂CF₃ | Me |
| G-1327 | Me | F | CH₂CH₂CH₂CH₂N(Et)S(=O)₂CF₃ | Me |
| G-1328 | Me | F | CH₂CH₂CH₂CH₂N(CH₂CN)S(=O)₂CF₃ | Me |
| G-1329 | Me | F | CH₂CH₂CH₂CH₂N(CH₂OMe)S(=O)₂CF₃ | Me |
| G-1330 | Me | F | CH₂CH(Me)NHS(=O)₂CF₃ | Me |
| G-1331 | Me | F | CH₂CH(Me)NHS(=O)₂CF₃ : (R) | Me |
| G-1332 | Me | F | CH₂CH(Me)NHS(=O)₂CF₃ : (S) | Me |
| G-1333 | Me | F | CH(Me)CH₂NHS(=O)₂CF₃ | Me |
| G-1334 | Me | F | CH₂CH₂CH(Me)NHS(=O)₂CF₃ | Me |
| G-1335 | Me | F | CH₂CH(Me)CH₂NHS(=O)₂CF₃ | Me |
| G-1336 | Me | F | CH(Me)CH₂CH₂NHS(=O)₂CF₃ | Me |
| G-1337 | Me | F | CH₂CH(Me)N(Me)S(=O)₂CF₃ | Me |
| G-1338 | Me | F | CH(Me)CH₂N(Me)S(=O)₂CF₃ | Me |
| G-1339 | Me | F | CH₂CH₂CH(Me)N(Me)S(=O)₂CF₃ | Me |
| G-1340 | Me | F | CH₂CH(Me)CH₂N(Me)S(=O)₂CF₃ | Me |
| G-1341 | Me | F | CH(Me)CH₂CH₂N(Me)S(=O)₂CF₃ | Me |
| G-1342 | Me | F | CH₂CH₂CH₂NHS(=O)₂Ph | Me |
| G-1343 | Me | F | CH₂CH₂CH₂CH₂NHS(=O)₂Ph | Me |
| G-1344 | Me | F | CH₂CH₂CH₂CH₂CH₂NHS(=O)₂Ph | Me |
| G-1345 | Me | F | CH₂CH₂CH₂CH₂CH₂NHS(=O)₂Ph(4-CF₃) | Me |
| G-1346 | Me | F | CH₂CH(Me)NHS(=O)₂Ph(4-CF₃) | Me |
| G-1347 | Me | F | CH₂CH(Et)NHS(=O)₂Ph(4-CF₃) | Me |
| G-1348 | Me | F | CH(Me)CH₂NHS(=O)₂Ph(4-CF₃) | Me |
| G-1349 | Me | F | CH(Et)CH₂NHS(=O)₂Ph(4-CF₃) | Me |
| G-1350 | Me | F | CH₂CH(Me)CH₂NHS(=O)₂Ph(4-CF₃) | Me |
| G-1351 | Me | F | CH₂CH₂SiMe₃ | Me |
| G-1352 | Me | F | CH₂CH₂CH₂SiMe₃ | Me |
| G-1353 | Me | F | CH₂CH₂CH₂CH₂SiMe₃ | Me |
| G-1354 | Me | F | CH₂Ph | Me |
| G-1355 | Me | F | CH₂Ph(2-CF₃) | Me |
| G-1356 | Me | F | CH₂Ph(3-CF₃) | Me |
| G-1357 | Me | F | CH₂Ph(4-CF₃) | Me |
| G-1358 | Me | F | CH₂Ph(2-OCF₃) | Me |

**[Table 1151]**

| Compound Number | R³ | R⁴ | R⁵ | R¹¹ |
|---|---|---|---|---|
| G-1359 | Me | F | CH₂Ph(3-OCF₃) | Me |
| G-1360 | Me | F | CH₂Ph(4-OCF₃) | Me |
| G-1361 | Me | F | CH₂Ph(2-SCF₃) | Me |
| G-1362 | Me | F | CH₂Ph(3-SCF₃) | Me |
| G-1363 | Me | F | CH₂Ph(4-SCF₃) | Me |
| G-1364 | Me | F | CH₂Ph(3-CH₂SCF₃) | Me |
| G-1365 | Me | F | CH₂Ph(4-F) | Me |
| G-1366 | Me | F | CH₂Ph(4-Cl) | Me |
| G-1367 | Me | F | CH₂Ph(4-Br) | Me |
| G-1368 | Me | F | CH₂Ph(4-Me) | Me |
| G-1369 | Me | F | CH₂Ph[4-(*t*-Bu)] | Me |
| G-1370 | Me | F | CH₂Ph(4-CN) | Me |
| G-1371 | Me | F | CH₂Ph(4-NO₂) | Me |
| G-1372 | Me | F | CH₂Ph(4-OCHF₂) | Me |
| G-1373 | Me | F | CH₂Ph(4-SCHF₂) | Me |
| G-1374 | Me | F | CH₂Ph(4-CH₂SCF₃) | Me |
| G-1375 | Me | F | CH₂Ph[4-CF(CF₃)₂₁ | Me |
| G-1376 | Me | F | CH₂Ph(4-CH₂SCF₃) | Me |
| G-1377 | Me | F | CH₂Ph[4-Ph(4-CF₃)] | Me |
| G-1378 | Me | F | CH₂Ph(2,4-Cl₂) | Me |
| G-1379 | Me | F | CH₂Ph[2,5-(CF₃)₂] | Me |
| G-1380 | Me | F | CH₂Ph(3,4-Cl₂) | Me |
| G-1381 | Me | F | CH₂Ph(3-CF₃-4-F) | Me |
| G-1382 | Me | F | CH₂Ph(3-CF₃-4-Cl) | Me |
| G-1383 | Me | F | CH₂Ph(3-F-4-CF₃) | Me |
| G-1384 | Me | F | CH₂Ph(2,4,6-F₃) | Me |
| G-1385 | Me | F | CH₂Ph(3,4,5-F₃) | Me |
| G-1386 | Me | F | CH₂Ph(2,3,4-F₃) | Me |
| G-1387 | Me | F | CH₂Ph(3,4,5-Cl₃) | Me |
| G-1388 | Me | F | CH₂CH₂Ph | Me |
| G-1389 | Me | F | CH₂CH₂Ph(4-F) | Me |
| G-1390 | Me | F | CH₂CH₂Ph(4-Cl) | Me |
| G-1391 | Me | F | CH₂CH₂Ph(4-Br) | Me |
| G-1392 | Me | F | CH₂CH₂Ph[4-(*t*-Bu)] | Me |
| G-1393 | Me | F | CH₂CH₂Ph(2-CF₃) | Me |
| G-1394 | Me | F | CH₂CH₂Ph(3-CF₃) | Me |
| G-1395 | Me | F | CH₂CH₂Ph(4-CF₃) | Me |
| G-1396 | Me | F | CH₂CH₂Ph[4-CF(CF₃)₂] | Me |
| G-1397 | Me | F | CH₂CH₂Ph[4-(C-Pr)] | Me |
| G-1398 | Me | F | CH₂CH₂Ph{4-[*c*-Pr(2,2-F₂)]} | Me |
| G-1399 | Me | F | CH₂CH₂Ph(4-OMe) | Me |
| G-1400 | Me | F | CH₂CH₂Ph(3-OCF₃) | Me |
| G-1401 | Me | F | CH₂CH₂Ph(4-OCHF₂) | Me |
| G-1402 | Me | F | CH₂CH₂Ph(4-OCF₃) | Me |

**[Table 1152]**

| Compound Number | R³ | R⁴ | R⁵ | R¹¹ |
|---|---|---|---|---|
| G-1403 | Me | F | CH₂CH₂Ph(4-SMe) | Me |
| G-1404 | Me | F | CH₂CH₂Ph(4-SCHF₂) | Me |
| G-1405 | Me | F | CH₂CH₂Ph(4-SCF₃) | Me |
| G-1406 | Me | F | CH₂CH₂Ph[4-S(=O)Me] | Me |
| G-1407 | Me | F | CH₂CH₂Ph[4-S(=O)CF₃] | Me |
| G-1408 | Me | F | CH₂CH₂Ph[4-S(=O)₂Me] | Me |
| G-1409 | Me | F | CH₂CH₂Ph[4-S(=O)₂CF₃] | Me |
| G-1410 | Me | F | CH₂CH₂Ph(4-CH₂SMe) | Me |
| G-1411 | Me | F | CH₂CH₂Ph(4-CH₂SCF₃) | Me |
| G-1412 | Me | F | CH₂CH₂Ph[4-OS(=O)₂Me] | Me |
| G-1413 | Me | F | CH₂CH₂Ph[4-OS(=O)₂CF₃] | Me |
| G-1414 | Me | F | CH₂CH₂Ph[4-Ph(4-CF₃)] | Me |
| G-1415 | Me | F | CH₂CH₂Ph(4-CH₂Ph) | Me |
| G-1416 | Me | F | CH₂CH₂Ph(4-OCH₂Ph) | Me |
| G-1417 | Me | F | CH₂CH₂Ph(4-CN) | Me |
| G-1418 | Me | F | CH₂CH₂Ph(4-NO₂) | Me |
| G-1419 | Me | F | CH₂CH₂Ph(2,4-Cl₂) | Me |
| G-1420 | Me | F | CH₂CH₂Ph(3,4-Cl₂) | Me |
| G-1421 | Me | F | CH₂CH₂Ph(3-CF₃-4-F) | Me |
| G-1422 | Me | F | CH₂CH₂Ph(2-CF₃-4-F) | Me |
| G-1423 | Me | F | CH₂cH₂Ph(3-F-4-CF₃) | Me |
| G-1424 | Me | F | CH₂CH₂Ph(2-F-4-CF₃) | Me |
| G-1425 | Me | F | CH₂CH₂Ph(3-Cl-4-OCHF₂) | Me |
| G-1426 | Me | F | CH₂CH₂Ph(3,4,5-Cl₃) | Me |
| G-1427 | Me | F | CH₂CH₂Ph(2,3,4-F₃) | Me |
| G-1428 | Me | F | CH₂CH₂Ph(2,4,5-F₃) | Me |
| G-1429 | Me | F | CH₂CH₂Ph(3,4,5-F₃) | Me |
| G-1430 | Me | F | CH₂CH₂Ph(2,4,6-F₃) | Me |
| G-1431 | Me | F | CH₂CH₂CH₂Ph | Me |
| G-1432 | Me | F | CH₂CH₂CH₂Ph(3-CF₃) | Me |
| G-1433 | Me | F | CH₂CH₂CH₂Ph(4-CF₃) | Me |
| G-1434 | Me | F | CH₂CH₂CH₂Ph(4-F) | Me |
| G-1435 | Me | F | CH₂CH₂CH₂Ph[4-(*t*-Bu)] | Me |
| G-1436 | Me | F | CH₂CH₂CH₂Ph(4-CN) | Me |
| G-1437 | Me | F | CH₂CH₂CH₂Ph(4-CF₃) | Me |
| G-1438 | Me | F | CH₂CH₂CH₂Ph(4-OCHF₂) | Me |
| G-1439 | Me | F | CH₂CH₂CH₂Ph(4-OCF₃) | Me |
| G-1440 | Me | F | CH₂CH₂CH₂Ph(4-SCHF₂) | Me |
| G-1441 | Me | F | CH₂CH₂CH₂Ph(4-SCF₃) | Me |
| G-1442 | Me | F | CH₂CH₂CH₂Ph[4-CF(CF₃)₂] | Me |
| G-1443 | Me | F | CH₂CH₂CH₂Ph(3,4,5-F₃) | Me |
| G-1444 | Me | F | CH₂CH₂CH₂Ph(2,4,6-F₃) | Me |
| G-1445 | Me | F | CH₂CH₂CH₂CH₂Ph | Me |
| G-1446 | Me | F | CH₂CH₂CH₂CH₂Ph(4-F) | Me |

**[Table 1153]**

| Compound Number | R³ | R⁴ | R⁵ | R¹¹ |
|---|---|---|---|---|
| G-1447 | Me | F | CH₂CH₂CH₂CH₂Ph(4-CF₃) | Me |
| G-1448 | Me | F | CH₂CH₂CH₂CH₂Ph(4-OCF₃) | Me |
| G-1449 | Me | F | CH₂CH₂CH₂CH₂Ph(4-SCF₃) | Me |
| G-1450 | Me | F | CH₂CH₂CH₂CH₂CH₂Ph | Me |
| G-1451 | Me | F | CH₂CH₂CH₂CH₂CH₂CH₂Ph | Me |
| G-1452 | Me | F | CH₂CF₂Ph(4-F) | Me |
| G-1453 | Me | F | CH₂CF₂Ph(4-CF₃) | Me |
| G-1454 | Me | F | CH₂CF₂Ph(3,4,5-F₃) | Me |
| G-1455 | Me | F | CH₂CH₂OPh | Me |
| G-1456 | Me | F | CH₂CH₂OPh(4-F) | Me |
| G-1457 | Me | F | CH₂CH₂OPh(4-CF₃) | Me |
| G-1458 | Me | F | CH₂CH₂CH₂OPh | Me |
| G-1459 | Me | F | CH₂CH₂CH₂OPh(4-Cl) | Me |
| G-1460 | Me | F | CH₂CH₂CH₂OPh(4-CF₃) | Me |
| G-1461 | Me | F | CH₂CH₂CH₂CH₂OPh(4-CF₃) | Me |
| G-1462 | Me | F | CH₂CH₂CH₂CH₂OPh(4-OCF₃) | Me |
| G-1463 | Me | F | CH₂CH₂CH₂CH₂CH₂OPh(4-CF₃) | Me |
| G-1464 | Me | F | CH₂CH₂OCH₂Ph | Me |
| G-1465 | Me | F | CH₂CH₂CH₂OCH₂Ph | Me |
| G-1466 | Me | F | CH₂CH₂CH₂CH₂OCH₂Ph | Me |
| G-1467 | Me | F | CH₂CH₂CH₂CH₂CH₂OCH₂Ph | Me |
| G-1468 | Me | F | CH₂CH₂SPh | Me |
| G-1469 | Me | F | CH₂CH₂CH₂SPh | Me |
| G-1470 | Me | F | CH₂CH₂CH₂SPh(4-F) | Me |
| G-1471 | Me | F | CH₂CH₂CH₂SPh(4-Cl) | Me |
| G-1472 | Me | F | CH₂CH₂CH₂SPh(4-Br) | Me |
| G-1473 | Me | F | CH₂CH₂CH₂SPh[4-(*t*-Bu)] | Me |
| G-1474 | Me | F | CH₂CH₂CH₂SPh(3-CF₃) | Me |
| G-1475 | Me | F | CH₂CH₂CH₂SPh(4-CF₃) | Me |
| G-1476 | Me | F | CH₂CH₂CH₂SPh(3-SCF₃) | Me |
| G-1477 | Me | F | CH₂CH₂CH₂SPh(4-SCF₃) | Me |
| G-1478 | Me | F | CH₂CH₂CH₂CH₂SPh | Me |
| G-1479 | Me | F | CH₂CH₂CH₂CH₂SPh(4-F) | Me |
| G-1480 | Me | F | CH₂CH₂CH₂CH₂SPh(4-Cl) | Me |
| G-1481 | Me | F | CH₂CH₂CH₂CH₂SPh[4-(*t*-Bu)] | Me |
| G-1482 | Me | F | CH₂CH₂CH₂CH₂SPh(4-CF₃) | Me |
| G-1483 | Me | F | CH₂CH₂CH₂CH₂CH₂SPh | Me |
| G-1484 | Me | F | CH₂CH₂CH₂CH₂CH₂SPh(4-F) | Me |
| G-1485 | Me | F | CH₂CH₂CH₂CH₂CH₂SPh(4-Cl) | Me |
| G-1486 | Me | F | CH₂CH₂CH₂CH₂CH₂SPh(4-CF₃) | Me |
| G-1487 | Me | F | CH₂CH₂S(=O)Ph | Me |
| G-1488 | Me | F | CH₂CH₂CH₂S(=O)Ph | Me |
| G-1489 | Me | F | CH₂CH₂CH₂S(=O)Ph(4-F) | Me |
| G-1490 | Me | F | CH₂CH₂CH₂S(=O)Ph[4-(*t*-Bu)] | Me |

**[Table 1154]**

| Compound Number | R³ | R⁴ | R⁵ | R¹¹ |
|---|---|---|---|---|
| G-1491 | Me | F | CH₂CH₂CH₂S(=O)Ph(4-CF₃) | Me |
| G-1492 | Me | F | CH₂CH₂S(=O)₂Ph | Me |
| G-1493 | Me | F | CH₂CH₂S(=O)₂Ph(4-CF₃) | Me |
| G-1494 | Me | F | CH₂CH₂CH₂S(=O)₂Ph(4-F) | Me |
| G-1495 | Me | F | CH₂CH₂CH₂S(=O)₂Ph(4-Cl) | Me |
| G-1496 | Me | F | CH₂CH₂CH₂S(=O)₂Ph(4-CF₃) | Me |
| G-1497 | Me | F | CH₂CH₂CH₂CH₂S(=O)₂Ph | Me |
| G-1498 | Me | F | CH₂CH₂CH₂CH₂S(=O)₂Ph(4-Cl) | Me |
| G-1499 | Me | F | CH₂CH₂CH₂CH₂S(=O)₂Ph(4-CF₃) | Me |
| G-1500 | Me | F | CH₂CH₂SCH₂Ph | Me |
| G-1501 | Me | F | CH₂CH₂CH₂SCH₂Ph | Me |
| G-1502 | Me | F | CH₂CH₂CH₂SCH₂Ph(2-Cl) | Me |
| G-1503 | Me | F | CH₂CH₂CH₂SCH₂Ph(3-Cl) | Me |
| G-1504 | Me | F | CH₂CH₂CH₂SCH₂Ph(4-Cl) | Me |
| G-1505 | Me | F | CH₂CH₂CH₂SCH₂Ph(2-SCF₃) | Me |
| G-1506 | Me | F | CH₂CH₂CH₂SCH₂Ph(3-CF₃) | Me |
| G-1507 | Me | F | CH₂CH₂CH₂SCH₂Ph(4-CF₃) | Me |
| G-1508 | Me | F | CH₂CH₂CH₂SCH₂Ph(4-NO₂) | Me |
| G-1509 | Me | F | CH₂CH₂CH₂CH₂SCH₂Ph | Me |
| G-1510 | Me | F | CH₂CH₂CH₂CH₂SCH₂Ph(4-Cl) | Me |
| G-1511 | Me | F | CH₂CH₂CH₂CH₂SCH₂Ph(4-CF₃) | Me |
| G-1512 | Me | F | CH₂CH₂CH₂CH₂SCH₂Ph(4-CN) | Me |
| G-1513 | Me | F | CH₂CH₂CH₂CH₂CH₂SCH₂Ph | Me |
| G-1514 | Me | F | CH₂CH₂SCH₂CH₂Ph | Me |
| G-1515 | Me | F | CH₂CH₂CH₂SCH₂CH₂Ph | Me |
| G-1516 | Me | F | CH₂CH₂CH₂CH₂SCH₂CH₂Ph | Me |
| G-1517 | Me | F | CH₂CH₂SCH(Me)Ph | Me |
| G-1518 | Me | F | CH₂CH₂CH₂SCH(Me)Ph | Me |
| G-1519 | Me | F | CH₂CH₂CH₂CH₂SCH(Me)Ph | Me |
| G-1520 | Me | F | CH₂CH₂S(=O)CH₂Ph | Me |
| G-1521 | Me | F | CH₂CH₂S(=O)CH₂Ph(4-CF₃) | Me |
| G-1522 | Me | F | CH₂CH₂CH₂S(=O)CH₂Ph | Me |
| G-1523 | Me | F | CH₂CH₂CH₂S(=O)CH₂Ph(2-SCF₃) | Me |
| G-1524 | Me | F | CH₂CH₂CH₂S(=O)CH₂Ph(4-SCF₃) | Me |
| G-1525 | Me | F | CH₂CH₂CH₂S(=O)CH₂Ph(4-Cl) | Me |
| G-1526 | Me | F | CH₂CH₂CH₂S(=O)CH₂Ph(4-CF₃) | Me |
| G-1527 | Me | F | CH₂CH₂CH₂CH₂S(=O)CH₂Ph | Me |
| G-1528 | Me | F | CH₂CH₂CH₂CH₂S(=O)CH₂Ph(4-CF₃) | Me |
| G-1529 | Me | F | CH₂CH₂CH₂CH₂CH₂S(=O)CH₂Ph(4-CF₃) | Me |
| G-1530 | Me | F | CH₂CH₂S(=O)₂CH₂Ph | Me |
| G-1531 | Me | F | CH₂CH₂S(=O)₂CH₂Ph(4-CF₃) | Me |
| G-1532 | Me | F | CH₂CH₂CH₂S(=O)₂CH₂Ph | Me |
| G-1533 | Me | F | CH₂CH₂CH₂S(=O)₂CH₂Ph(4-Cl) | Me |
| G-1534 | Me | F | CH₂CH₂CH₂S(=O)₂CH₂Ph(4-CF₃) | Me |

**[Table 1155]**

| Compound Number | R³ | R⁴ | R⁵ | R¹¹ |
|---|---|---|---|---|
| G-1535 | Me | F | CH₂CH₂CH₂CH₂S(=O)₂CH₂Ph | Me |
| G-1536 | Me | F | CH₂CH₂CH₂CH₂S(=O)₂CH₂Ph(4-CF₃) | Me |
| G-1537 | Me | F | CH₂CH₂CH₂CH₂CH₂S(=O)₂CH₂Ph | Me |
| G-1538 | Me | F | CH₂CH₂CH₂CH₂CH₂S(=O)₂CH₂Ph(4-CF₃) | Me |
| G-1539 | Me | F | CH₂CH₂ON=CH(*t*-Bu) | Me |
| G-1540 | Me | F | CH₂CH₂CH₂ON=CH(*t*-Bu) | Me |
| G-1541 | Me | F | CH₂CH₂CH₂CH₂ON=C(Me)(*c*-Pr) | Me |
| G-1542 | Me | F | CH₂CH₂ON=C(Me)CF₃ | Me |
| G-1543 | Me | F | CH₂CH₂CH₂ON=CHCF₃ | Me |
| G-1544 | Me | F | CH₂CH₂CH₂ON=C(Me)CF₃ | Me |
| G-1545 | Me | F | CH₂CH₂CH₂ON=C(Me)CCl₃ | Me |
| G-1546 | Me | F | CH₂CH₂CH₂CH₂ON=CHCF₃ | Me |
| G-1547 | Me | F | CH₂CH₂CH₂CH₂ON=C(Me)CF₃ | Me |
| G-1548 | Me | F | CH₂CH₂CH₂CH₂CH₂ON=C(Me)CF₃ | Me |
| G-1549 | Me | F | CH₂CH₂ON=CHPh | Me |
| G-1550 | Me | F | CH₂CH₂ON=CHPh(4-CF₃) | Me |
| G-1551 | Me | F | CH₂CH₂ON=CHPh(4-SCF₃) | Me |
| G-1552 | Me | F | CH₂CH₂CH₂ON=CHPh(3-CF₃) | Me |
| G-1553 | Me | F | CH₂CH₂CH₂ON=CHPh(4-CF₃) | Me |
| G-1554 | Me | F | CH₂CH₂CH₂ON=CHPh(4-SCF₃) | Me |
| G-1555 | Me | F | CH₂CH₂CH₂ON=C(Me)Ph(4-CF₃) | Me |
| G-1556 | Me | F | CH₂CH₂CH₂CH₂ON=CHPh(4-SCF₃) | Me |
| G-1557 | Me | F | CH₂CH₂(adamant-1-yl) | Me |
| G-1558 | Me | F | CH₂CH₂CH₂(3-CF₃-pyrazol-1-yl) | Me |
| G-1559 | Me | F | CH₂CH₂CH₂CH₂(3-CF₃-pyrazol-1-yl) | Me |
| G-1560 | Me | F | CH₂CH₂CH₂CH₂CH₂(3-CF₃-pyrazol-1-yl) | Me |
| G-1561 | Me | F | CH₂(1-Me-4-CF₃-imidazol-5-yl) | Me |
| G-1562 | Me | F | CH₂CH₂(1-Me-4-CF₃-imidazol-5-yl) | Me |
| G-1563 | Me | F | CH₂CH₂CH₂(1-Me-4-CF₃-imidazol-5-yl) | Me |
| G-1564 | Me | F | CH₂CH₂CH₂(3-CF₃-1,2,4-triazol-1-yl) | Me |
| G-1565 | Me | F | CH₂CH₂CH₂CH₂(3-CF₃-1,2,4-triazol-1-yl) | Me |
| G-1566 | Me | F | CH₂CH₂CH₂CH₂CH₂(3-CF₃-1,2,4-triazol-1-yl) | Me |
| G-1567 | Me | F | CH₂CH₂CH₂(4-CF₃-thiazol-2-yl) | Me |
| G-1568 | Me | F | CH₂CH₂CH₂CH₂(4-CF₃-thiazol-2-yl) | Me |
| G-1569 | Me | F | CH₂CH₂CH₂CH₂CH₂(4-CF₃-thiazol-2-yl) | Me |
| G-1570 | Me | F | CH₂CH₂CH₂(2-CF₃-pyridin-5-yl) | Me |
| G-1571 | Me | F | CH₂CH₂CH₂CH₂(2-CF₃-pyridin-5-yl) | Me |
| G-1572 | Me | F | CH₂CH₂CH₂CH₂CH₂(2-CF₃-pyridin-5-yl) | Me |
| G-1573 | Me | F | CH₂(4-CF₃-pyrimidin-2-yl) | Me |
| G-1574 | Me | F | CH₂CH₂(4-CF₃-pyrimidin-2-yl) | Me |
| G-1575 | Me | F | CH₂CH₂CH₂(4-CF₃-pyrimidin-2-yl) | Me |
| G-1576 | Me | F | CH₂CH₂O(3-Cl-5-CF₃-pyridin-2-yl) | Me |
| G-1577 | Me | F | CH₂CH₂CH₂O(3-Cl-5-CF₃-pyridin-2-yl) | Me |
| G-1578 | Me | F | CH₂CH₂CH₂CH₂O(3-Cl-5-CF₃-pyridin-2-yl) | Me |

**[Table 1156]**

| Compound Number | R³ | R⁴ | R⁵ | R¹¹ |
|---|---|---|---|---|
| G-1579 | Me | F | CH₂CH₂CH₂CH₂CH₂O(3-Cl-5-CF₃-pyridin-2-yl) | Me |
| G-1580 | Me | F | CH₂CH₂N(Phth) | Me |
| G-1581 | Me | F | CH₂CH₂CH₂N(Phth) | Me |
| G-1582 | Me | F | CH₂CH₂CH₂CH₂N(Phth) | Me |
| G-1583 | Me | F | CH₂CH₂CH₂CH₂CH₂N(Phth) | Me |
| G-1584 | Me | F | CH₂CH(Me)CH₂N(Phth) | Me |
| G-1585 | Me | F | CH₂[1,3-dioxolan(2-CF₃)-2-yl] | Me |
| G-1586 | Me | F | CH₂(azetidin-3-yl) | Me |
| G-1587 | Me | F | CH₂(pyrrolidin-3-yl) | Me |
| G-1588 | Me | F | CH₂(piperidin-3-yl) | Me |
| G-1589 | Me | F | CH₂{azetidin[1-C(=O)O(*t*-Bu)]-3-yl} | Me |
| G-1590 | Me | F | CH₂{pyrrolidin[1-C(=O)O(*t*-Bu)]-3-yl} | Me |
| G-1591 | Me | F | CH₂{piperidin[1-C(=O)O(*t*-Bu)]-3-yl} | Me |
| G-1592 | Me | F | CH₂{azetidin[1-C(=O)CF₃]-3-yl} | Me |
| G-1593 | Me | F | CH₂{azetidin[1-S(=O)₂CF₃]-3-yl} | Me |
| G-1594 | Me | F | CH₂{pyrrolidin[1-C(=O)CF₃]-3-yl} | Me |
| G-1595 | Me | F | CH₂{pyrrolidin[1-S(=O)₂CF₃]-3-yl} | Me |
| G-1596 | Me | F | CH₂{piperidin[1-C(=O)CF₃]-3-yl} | Me |
| G-1597 | Me | F | CH₂{piperidin[1-S(=O)₂CF₃]-3-yl} | Me |
| G-1598 | Me | F | CH₂CH₂CH₂CN | Me |
| G-1599 | Me | F | CH₂CH₂CH₂CH₂CN | Me |
| G-1600 | Me | F | CH₂CH₂CH₂CH₂CH₂CN | Me |
| G-1601 | Me | F | CH₂CH₂CH₂CH₂CH₂CH₂CN | Me |
| G-1602 | Me | F | CH₂CH₂CH₂C(=O)OH | Me |
| G-1603 | Me | F | CH₂CH₂CH₂CH₂C(=O)OH | Me |
| G-1604 | Me | F | CH₂CH₂CH₂CH₂CH₂C(=O)OH | Me |
| G-1605 | Me | F | CH₂CH₂SCN | Me |
| G-1606 | Me | F | CH₂CH₂CH₂SCN | Me |
| G-1607 | Me | F | CH₂CH₂CH₂CH₂SCN | Me |
| G-1608 | Me | F | CH₂CH₂CH₂CH₂CH₂SCN | Me |
| G-1609 | Me | F | CH₂CH₂CH₂CH₂CH₂CH₂SCN | Me |
| G-1610 | Me | F | CH₂CH₂CH₂CH₂CH₂CH₂CH₂SCN | Me |
| G-1611 | Me | F | CH₂CH₂CH₂CH₂CH₂CH₂CH₂CH₂SCN | Me |
| G-1612 | Me | F | CH₂CH₂CH(Me)SCN | Me |
| G-1613 | Me | F | CH₂CH(Me)CH₂SCN | Me |
| G-1614 | Me | F | CH(Me)CH₂CH₂SCN | Me |
| G-1615 | Me | F | CH₂CH₂CH₂CH(Me)SCN | Me |
| G-1616 | Me | F | CH₂CH₂CH(Me)CH₂SCN | Me |
| G-1617 | Me | F | CH₂CH(Me)CH₂CH₂SCN | Me |
| G-1618 | Me | F | CH(Me)CH₂CH₂CH₂SCN | Me |
| G-1619 | Me | F | CH₂CH₂CH₂CH₂CH(Me)SCN | Me |
| G-1620 | Me | F | CH₂CH₂CH₂CH(Me)CH₂SCN | Me |
| G-1621 | Me | F | CH₂CH₂CH(Me)CH₂CH₂SCN | Me |
| G-1622 | Me | F | CH₂CH(Me)CH₂CH₂CH₂SCN | Me |

**[Table 1157]**

| Compound Number | R³ | R⁴ | R⁵ | R¹¹ |
|---|---|---|---|---|
| G-1623 | Me | F | CH(Me)CH₂CH₂CH₂CH₂SCN | Me |
| G-1624 | Me | F | CH₂CH₂ONH₂ | Me |
| G-1625 | Me | F | CH₂CH₂CH₂ONH₂ | Me |
| G-1626 | Me | F | CH₂CH₂CH₂CH₂ONH₂ | Me |
| G-1627 | Me | F | CH₂CH₂CH₂CH₂CH₂ONH₂ | Me |
| G-1628 | Me | F | CH₂CH₂CH₂CH₂CH₂CH₂ONH₂ | Me |
| G-1629 | Cl | H | CH₂C(=O)Me | Me |
| G-1630 | Cl | H | CH₂CH(OH)Me | Me |
| G-1631 | Cl | H | CH₂CH(OH)CF₃ | Me |
| G-1632 | Cl | H | CH₂C(OH)₂CF₃ | Me |
| G-1633 | Cl | H | CH₂C(CF₃)=NOH | Me |
| G-1634 | Cl | H | CH₂C(CF₃)=NOMe | Me |
| G-1635 | Cl | H | CH₂CH(CF₃)NH₂ | Me |
| G-1636 | Cl | H | CH₂CH(CF₃)NHSO₂CF₃ | Me |
| G-1637 | Cl | H | CH₂[*c*-Pr(1-NH₂)] | Me |
| G-1638 | Cl | H | CH₂[*c*-Pr(1-NHC(=O)O(*t*-Bu)] | Me |
| G-1639 | Cl | H | CH₂{*c*-Pr[1-NHS(=O)₂CF₃]} | Me |
| G-1640 | Cl | H | CH₂CH₂NH₂ | Me |
| G-1641 | Cl | H | CH₂CH₂NH₂, HCl | Me |
| G-1642 | Cl | H | CH₂CH₂CH₂NH₂ | Me |
| G-1643 | Cl | H | CH₂CH₂CH₂CH₂NH₂ | Me |
| G-1644 | Cl | H | CH₂CH₂CH₂CH₂CH₂NH₂ | Me |
| G-1645 | Cl | H | CH₂CH₂CH₂CH₂CH₂CH₂NH₂ | Me |
| G-1646 | Cl | H | CH₂CH₂CH₂CH₂CH₂CH₂CH₂NH₂ | Me |
| G-1647 | Cl | H | CH₂CH(Me)NH₂ | Me |
| G-1648 | Cl | H | CH₂C*H(Me)NH₂, HCl | Me |
| G-1649 | Cl | H | CH₂C*H(Me)NH₂: (R) | Me |
| G-1650 | Cl | H | CH₂C*H(Me)NH₂, HCl: (R) | Me |
| G-1651 | Cl | H | CH₂C*H(Me)NH₂: (S) | Me |
| G-1652 | Cl | H | CH₂C*H(Me)NH₂, HCl: (S) | Me |
| G-1653 | Cl | H | CH₂CH(Et)NH₂ | Me |
| G-1654 | Cl | H | CH₂CH(*i*-Pr)NH₂ | Me |
| G-1655 | Cl | H | CH(Me)CH₂NH₂ | Me |
| G-1656 | Cl | H | CH(Me)CH₂NH₂, HCl | Me |
| G-1657 | Cl | H | CH(Et)CH₂NH₂ | Me |
| G-1658 | Cl | H | CH(*i*-Pr)CH₂NH₂ | Me |
| G-1659 | Cl | H | CH₂CH₂CH(Me)NH₂ | Me |
| G-1660 | Cl | H | CH₂CH(Me)CH₂NH₂ | Me |
| G-1661 | Cl | H | CH(Me)CH₂CH₂NH₂ | Me |
| G-1662 | Cl | H | CH₂CH(Me)NH(*c*-Pr) | Me |
| G-1663 | Cl | H | CH₂CH(Me)N(*c*-Pr)S(=O)₂CF₃ | Me |
| G-1664 | Cl | H | CH₂CH(Me)NHOMe | Me |
| G-1665 | Cl | H | CH₂CH(Me)N(OMe)S(=O)₂CF₃ | Me |
| G-1666 | Cl | H | CH₂CH(Me)N(OMe)S(=O)₂Ph(2-NO₂) | Me |

**[Table 1158]**

| Compound Number | R³ | R⁴ | R⁵ | R¹¹ |
|---|---|---|---|---|
| G-1667 | Cl | H | CH₂CH(Me)N(OMe)S(=O)₂Ph(4-NO₂) | Me |
| G-1668 | Cl | H | CH₂CH₂NHC(=O)O(*t*-Bu) | Me |
| G-1669 | Cl | H | CH₂CH₂CH₂NHC(=O)O(*t*-Bu) | Me |
| G-1670 | Cl | H | CH₂CH₂CH₂CH₂NHC(=O)O(*t*-Bu) | Me |
| G-1671 | Cl | H | CH₂CH₂CH₂CH₂NHC(=O)OCH(CH₃)₂ | Me |
| G-1672 | Cl | H | CH₂CH(Me)NHC(=O)O(*t*-Bu) | Me |
| G-1673 | Cl | H | CH₂C*H(Me)NHC(=O)O(*t*-Bu) : (R) | Me |
| G-1674 | Cl | H | CH₂C*H(Me)NHC(=O)O(*t*-Bu) : (S) | Me |
| G-1675 | Cl | H | CH₂CH(Et)NHC(=O)O(*t*-Bu) | Me |
| G-1676 | Cl | H | CH₂CH(*i*-Pr)NHC(=O)O(*t*-Bu) | Me |
| G-1677 | Cl | H | CH(Me)CH₂NHC(=O)O(*t*-Bu) | Me |
| G-1678 | Cl | H | CH(Et)CH₂NHC(=O)O(*t*-Bu) | Me |
| G-1679 | Cl | H | CH(*i*-Pr)CH₂NHC(=O)O(*t*-Bu) | Me |
| G-1680 | Cl | H | CH₂CH(Me)CH₂NHC(=O)O(*t*-Bu) | Me |
| G-1681 | Cl | H | CH₂CH₂SCHF₂ | Me |
| G-1682 | Cl | H | CH₂CH₂CH₂SCHF₂ | Me |
| G-1683 | Cl | H | CH₂CH₂CH₂CH₂SCHF₂ | Me |
| G-1684 | Cl | H | CH₂CH₂CH₂CH₂CH₂SCHF₂ | Me |
| G-1685 | Cl | H | CH₂CH₂CH₂CH₂CH₂CH₂SCHF₂ | Me |
| G-1686 | Cl | H | CH₂CH₂CH₂CH₂CH₂CH₂CH₂SCHF₂ | Me |
| G-1687 | Cl | H | CH₂CH₂SCF₃ | Me |
| G-1688 | Cl | H | CH₂CH₂CH₂SCF₃ | Me |
| G-1689 | Cl | H | CH₂CH₂CH₂CH₂SCF₃ | Me |
| G-1690 | Cl | H | CH₂CH₂CH₂CH₂CH₂SCF₃ | Me |
| G-1691 | Cl | H | CH₂CH₂CH₂CH₂CH₂CH₂SCF₃ | Me |
| G-1692 | Cl | H | CH₂CH₂CH₂CH₂CH₂CH₂CH₂SCF₃ | Me |
| G-1693 | Cl | H | CH₂CH₂CH₂CH₂CH₂CH₂CH₂CH₂SCF₃ | Me |
| G-1694 | Cl | H | CH₂CH(Me)SCF₃ | Me |
| G-1695 | Cl | H | CH(Me)CH₂SCF₃ | Me |
| G-1696 | Cl | H | CH₂CH₂CH(Me)SCF₃ | Me |
| G-1697 | Cl | H | CH₂CH(Me)CH₂SCF₃ | Me |
| G-1698 | Cl | H | CH(Me)CH₂CH₂SCF₃ | Me |
| G-1699 | Cl | H | CH₂CH₂CH₂CH(Me)SCF₃ | Me |
| G-1700 | Cl | H | CH₂CH₂CH(Me)CH₂SCF₃ | Me |
| G-1701 | Cl | H | CH₂CH(Me)CH₂CH₂SCF₃ | Me |
| G-1702 | Cl | H | CH(Me)CH₂CH₂CH₂SCF₃ | Me |
| G-1703 | Cl | H | CH₂CH₂NHS(=O)₂CF₃ | Me |
| G-1704 | Cl | H | CH₂CH₂CH₂NHS(=O)₂CF₃ | Me |
| G-1705 | Cl | H | CH₂CH₂CH₂CH₂NHS(=O)₂CF₃ | Me |
| G-1706 | Cl | H | CH₂CH₂CH₂CH₂CH₂NHS(=O)₂CF₃ | Me |
| G-1707 | Cl | H | CH₂CH₂N(Me)S(=O)₂CF₃ | Me |
| G-1708 | Cl | H | CH₂CH₂N(Et)S(=O)₂CF₃ | Me |
| G-1709 | Cl | H | CH₂CH₂N(CH₂CN)S(=O)₂CF₃ | Me |
| G-1710 | Cl | H | CH₂CH₂N(CH₂OMe)S(=O)₂CF₃ | Me |

**[Table 1159]**

| Compound Number | R³ | R⁴ | R⁵ | R¹¹ |
|---|---|---|---|---|
| G-1711 | Cl | H | CH₂CH₂CH₂N(Me)S(=O)₂CF₃ | Me |
| G-1712 | Cl | H | CH₂CH₂CH₂N(Et)S(=O)₂CF₃ | Me |
| G-1713 | Cl | H | CH₂CH₂CH₂N(CH₂CN)S(=O)₂CF₃ | Me |
| G-1714 | Cl | H | CH₂CH₂CH₂N(CH₂OMe)S(=O)₂CF₃ | Me |
| G-1715 | Cl | H | CH₂CH₂CH₂CH₂N(Me)S(=O)₂CF₃ | Me |
| G-1716 | Cl | H | CH₂CH₂CH₂CH₂N(Et)S(=O)₂CF₃ | Me |
| G-1717 | Cl | H | CH₂CH₂CH₂CH₂N(CH₂CN)S(=O)₂CF₃ | Me |
| G-1718 | Cl | H | CH₂CH₂CH₂CH₂N(CH₂OMe)S(=O)₂CF₃ | Me |
| G-1719 | Cl | H | CH₂CH(Me)NHS(=O)₂CF₃ | Me |
| G-1720 | Cl | H | CH₂CH(Me)NHS(=O)₂CF₃: (R) | Me |
| G-1721 | Cl | H | CH₂CH(Me)NHS(=O)₂CF₃ : (S) | Me |
| G-1722 | Cl | H | CH(Me)CH₂NHS(=O)₂CF₃ | Me |
| G-1723 | Cl | H | CH₂CH₂CH(Me)NHS(=O)₂CF₃ | Me |
| G-1724 | Cl | H | CH₂CH(Me)CH₂NHS(=O)₂CF₃ | Me |
| G-1725 | Cl | H | CH(Me)CH₂CH₂NHS(=O)₂CF₃ | Me |
| G-1726 | Cl | H | CH₂CH(Me)N(Me)S(=O)₂CF₃ | Me |
| G-1727 | Cl | H | CH(Me)CH₂N(Me)S(=O)₂CF₃ | Me |
| G-1728 | Cl | H | CH₂CH₂CH(Me)N(Me)S(=O)₂CF₃ | Me |
| G-1729 | Cl | H | CH₂CH(Me)CH₂N(Me)S(=O)₂CF₃ | Me |
| G-1730 | Cl | H | CH(Me)CH₂CH₂N(Me)S(=O)₂CF₃ | Me |
| G-1731 | Cl | H | CH₂Ph | Me |
| G-1732 | Cl | H | CH₂Ph(2-CF₃) | Me |
| G-1733 | Cl | H | CH₂Ph(3-CF₃) | Me |
| G-1734 | Cl | H | CH₂Ph(4-CF₃) | Me |
| G-1735 | Cl | H | CH₂Ph(2-OCF₃) | Me |
| G-1736 | Cl | H | CH₂Ph(3-OCF₃) | Me |
| G-1737 | Cl | H | CH₂Ph(4-OCF₃) | Me |
| G-1738 | Cl | H | CH₂Ph(2-SCF₃) | Me |
| G-1739 | Cl | H | CH₂Ph(3-SCF₃) | Me |
| G-1740 | Cl | H | CH₂Ph(4-SCF₃) | Me |
| G-1741 | Cl | H | CH₂Ph(3-CH₂SCF₃) | Me |
| G-1742 | Cl | H | CH₂Ph(4-F) | Me |
| G-1743 | Cl | H | CH₂Ph(4-Cl) | Me |
| G-1744 | Cl | H | CH₂Ph(4-Br) | Me |
| G-1745 | Cl | H | CH₂Ph(4-Me) | Me |
| G-1746 | Cl | H | CH₂Ph[4-(*t*-Bu)] | Me |
| G-1747 | Cl | H | CH₂Ph(4-CN) | Me |
| G-1748 | Cl | H | CH₂Ph(4-NO₂) | Me |
| G-1749 | Cl | H | CH₂Ph(4-OCHF₂) | Me |
| G-1750 | Cl | H | CH₂Ph(4-SCHF₂) | Me |
| G-1751 | Cl | H | CH₂Ph(4-CH₂SCF₃) | Me |
| G-1752 | Cl | H | CH₂Ph[4-CF(CF₃)₂] | Me |
| G-1753 | Cl | H | CH₂Ph(4-CH₂SCF₃) | Me |
| G-1754 | Cl | H | CH₂Ph[4-Ph(4-CF₃)] | Me |

**[Table 1160]**

| Compound Number | R³ | R⁴ | R⁵ | R¹¹ |
|---|---|---|---|---|
| G-1755 | Cl | H | CH₂Ph(2,4-Cl₂) | Me |
| G-1756 | Cl | H | CH₂Ph[2,5-(CF₃)₂] | Me |
| G-1757 | Cl | H | CH₂Ph(3,4-Cl₂) | Me |
| G-1758 | Cl | H | CH₂Ph(3-CF₃-4-F) | Me |
| G-1759 | Cl | H | CH₂Ph(3-CF₃-4-Cl) | Me |
| G-1760 | Cl | H | CH₂Ph(3-F-4-CF₃) | Me |
| G-1761 | Cl | H | CH₂Ph(2,4,6-F₃) | Me |
| G-1762 | Cl | H | CH₂Ph(3,4,5-F₃) | Me |
| G-1763 | Cl | H | CH₂Ph(2,3,4-F₃) | Me |
| G-1764 | Cl | H | CH₂Ph(3,4,5-Cl₃) | Me |
| G-1765 | Cl | H | CH₂CH₂Ph | Me |
| G-1766 | Cl | H | CH₂CH₂Ph(4-F) | Me |
| G-1767 | Cl | H | CH₂CH₂Ph(4-Cl) | Me |
| G-1768 | Cl | H | CH₂CH₂Ph(4-Br) | Me |
| G-1769 | Cl | H | CH₂CH₂Ph[4-(*t*-Bu)] | Me |
| G-1770 | Cl | H | CH₂CH₂Ph(2-CF₃) | Me |
| G-1771 | Cl | H | CH₂CH₂Ph(3-CF₃) | Me |
| G-1772 | Cl | H | CH₂CH₂Ph(4-CF₃) | Me |
| G-1773 | Cl | H | CH₂CH₂Ph[4-CF(CF₃)₂] | Me |
| G-1774 | Cl | H | CH₂CH₂Ph[4-(*c*-Pr)] | Me |
| G-1775 | Cl | H | CH₂CH₂Ph{4-[*c*-Pr(2,2-F₂)]} | Me |
| G-1776 | Cl | H | CH₂CH₂Ph(4-OMe) | Me |
| G-1777 | Cl | H | CH₂CH₂Ph(3-OCF₃) | Me |
| G-1778 | Cl | H | CH₂CH₂Ph(4-OCHF₂) | Me |
| G-1779 | Cl | H | CH₂CH₂Ph(4-OCF₃) | Me |
| G-1780 | Cl | H | CH₂CH₂Ph(4-SMe) | Me |
| G-1781 | Cl | H | CH₂CH₂Ph(4-SCHF₂) | Me |
| G-1782 | Cl | H | CH₂CH₂Ph(4-SCF₃) | Me |
| G-1783 | Cl | H | CH₂CH₂Ph[4-S(=O)Me] | Me |
| G-1784 | Cl | H | CH₂CH₂Ph[4-S(=O)CF₃] | Me |
| G-1785 | Cl | H | CH₂CH₂Ph[4-S(=O)₂Me] | Me |
| G-1786 | Cl | H | CH₂CH₂Ph[4-S(=O)₂CF₃] | Me |
| G-1787 | Cl | H | CH₂CH₂Ph(4-CH₂SMe) | Me |
| G-1788 | Cl | H | CH₂CH₂Ph(4-CH₂SCF₃) | Me |
| G-1789 | Cl | H | CH₂CH₂Ph[4-OS(=O)₂Me] | Me |
| G-1790 | Cl | H | CH₂CH₂Ph[4-OS(=O)₂CF₃] | Me |
| G-1791 | Cl | H | CH₂CH₂Ph[4-Ph(4-CF₃)] | Me |
| G-1792 | Cl | H | CH₂CH₂Ph(4-CH₂Ph) | Me |
| G-1793 | Cl | H | CH₂CH₂Ph(4-OCH₂Ph) | Me |
| G-1794 | Cl | H | CH₂CH₂Ph(4-CN) | Me |
| G-1795 | Cl | H | CH₂CH₂Ph(4-NO₂) | Me |
| G-1796 | Cl | H | CH₂CH₂Ph(2,4-Cl₂) | Me |
| G-1797 | Cl | H | CH₂CH₂Ph(3,4-Cl₂) | Me |
| G-1798 | Cl | H | CH₂CH₂Ph(3-CF₃-4-F) | Me |

**[Table 1161]**

| Compound Number | R³ | R⁴ | R⁵ | R¹¹ |
|---|---|---|---|---|
| G-1799 | Cl | H | CH₂CH₂Ph(2-CF₃-4-F) | Me |
| G-1800 | Cl | H | CH₂CH₂Ph(3-F-4-CF₃) | Me |
| G-1801 | Cl | H | CH₂CH₂Ph(2-F-4-CF₃) | Me |
| G-1802 | Cl | H | CH₂CH₂Ph(3-Cl-4-OCHF₂) | Me |
| G-1803 | Cl | H | CH₂CH₂Ph(3,4,5-Cl₃) | Me |
| G-1804 | Cl | H | CH₂CH₂Ph(2,3,4-F₃) | Me |
| G-1805 | Cl | H | CH₂CH₂Ph(2,4,5-F₃) | Me |
| G-1806 | Cl | H | CH₂CH₂Ph(3,4,5-F₃) | Me |
| G-1807 | Cl | H | CH₂CH₂Ph(2,4,6-F₃) | Me |
| G-1808 | Cl | H | CH₂CH₂CH₂Ph | Me |
| G-1809 | Cl | H | CH₂CH₂CH₂Ph(3-CF₃) | Me |
| G-1810 | Cl | H | CH₂CH₂CH₂Ph(4-CF₃) | Me |
| G-1811 | Cl | H | CH₂CH₂CH₂Ph(4-F) | Me |
| G-1812 | Cl | H | CH₂CH₂CH₂Ph[4-(*t*-Bu)] | Me |
| G-1813 | Cl | H | CH₂CH₂CH₂Ph(4-CN) | Me |
| G-1814 | Cl | H | CH₂CH₂CH₂Ph(4-CF₃) | Me |
| G-1815 | Cl | H | CH₂CH₂CH₂Ph(4-OCHF₂) | Me |
| G-1816 | Cl | H | CH₂CH₂CH₂Ph(4-OCF₃) | Me |
| G-1817 | Cl | H | CH₂CH₂CH₂Ph(4-SCHF₂) | Me |
| G-1818 | Cl | H | CH₂CH₂CH₂Ph(4-SCF₃) | Me |
| G-1819 | Cl | H | CH₂CH₂CH₂Ph[4-CF(CF₃)₂] | Me |
| G-1820 | Cl | H | CH₂CH₂CH₂Ph(3,4,5-F₃) | Me |
| G-1821 | Cl | H | CH₂CH₂CH₂Ph(2,4,6-F₃) | Me |
| G-1822 | Cl | H | CH₂CH₂CH₂CH₂Ph | Me |
| G-1823 | Cl | H | CH₂CH₂CH₂CH₂Ph(4-F) | Me |
| G-1824 | Cl | H | CH₂CH₂CH₂CH₂Ph(4-CF₃) | Me |
| G-1825 | Cl | H | CH₂CH₂CH₂CH₂Ph(4-OCF₃) | Me |
| G-1826 | Cl | H | CH₂CH₂CH₂CH₂Ph(4-SCF₃) | Me |
| G-1827 | Cl | H | CH₂CH₂CH₂CH₂CH₂Ph | Me |
| G-1828 | Cl | H | CH₂CH₂CH₂CH₂CH₂CH₂Ph | Me |
| G-1829 | Cl | H | CH₂CF₂Ph(4-F) | Me |
| G-1830 | Cl | H | CH₂CF₂Ph(4-CF₃) | Me |
| G-1831 | Cl | H | CH₂CF₂Ph(3,4,5-F₃) | Me |
| G-1832 | Me | H | CH₂C(=O)Me | Me |
| G-1833 | Me | H | CH₂CH(OH)Me | Me |
| G-1834 | Me | H | CH₂CH(OH)CF₃ | Me |
| G-1835 | Me | H | CH₂C(OH)₂CF₃ | Me |
| G-1836 | Me | H | CH₂C(CF₃)=NOH | Me |
| G-1837 | Me | H | CH₂C(CF₃)=NOMe | Me |
| G-1838 | Me | H | CH₂CH(CF₃)NH₂ | Me |
| G-1839 | Me | H | CH₂CH(CF₃)NHSO₂CF₃ | Me |
| G-1840 | Me | H | CH₂[*c*-Pr(1-NH₂) | Me |
| G-1841 | Me | H | CH₂[*c*-Pr(1-NHC(=O)O(*t*-Bu)] | Me |
| G-1842 | Me | H | CH₂{*c*-Pr[1-NHS(=O)₂CF₃]} | Me |

**[Table 1162]**

| Compound Number | R³ | R⁴ | R⁵ | R¹¹ |
|---|---|---|---|---|
| G-1843 | Me | H | CH₂CH₂NH₂ | Me |
| G-1844 | Me | H | CH₂CH₂NH₂, HCl | Me |
| G-1845 | Me | H | CH₂CH₂CH₂NH₂ | Me |
| G-1846 | Me | H | CH₂CH₂CH₂CH₂NH₂ | Me |
| G-1847 | Me | H | CH₂CH₂CH₂CH₂CH₂NH₂ | Me |
| G-1848 | Me | H | CH₂CH₂CH₂CH₂CH₂CH₂NH₂ | Me |
| G-1849 | Me | H | CH₂CH₂CH₂CH₂CH₂CH₂CH₂NH₂ | Me |
| G-1850 | Me | H | CH₂CH(Me)NH₂ | Me |
| G-1851 | Me | H | CH₂C*H(Me)NH₂, HCl | Me |
| G-1852 | Me | H | CH₂C*H(Me)NH₂: (R) | Me |
| G-1853 | Me | H | CH₂C*H(Me)NH₂, HCl: (R) | Me |
| G-1854 | Me | H | CH₂C*H(Me)NH₂: (S) | Me |
| G-1855 | Me | H | CH₂C*H(Me)NH₂, HCl: (S) | Me |
| G-1856 | Me | H | CH₂CH(Et)NH₂ | Me |
| G-1857 | Me | H | CH₂CH(*i*-Pr)NH₂ | Me |
| G-1858 | Me | H | CH(Me)CH₂NH₂ | Me |
| G-1859 | Me | H | CH(Me)CH₂NH₂, HCl | Me |
| G-1860 | Me | H | CH(Et)CH₂NH₂ | Me |
| G-1861 | Me | H | CH(*i*-Pr)CH₂NH₂ | Me |
| G-1862 | Me | H | CH₂CH₂CH(Me)NH₂ | Me |
| G-1863 | Me | H | CH₂CH(Me)CH₂NH₂ | Me |
| G-1864 | Me | H | CH(Me)CH₂CH₂NH₂ | Me |
| G-1865 | Me | H | CH₂CH(Me)NH(*c*-Pr) | Me |
| G-1866 | Me | H | CH₂CH(Me)N(*c*-Pr)S(=O)₂CF₃ | Me |
| G-1867 | Me | H | CH₂CH(Me)NHOMe | Me |
| G-1868 | Me | H | CH₂CH(Me)N(OMe)S(=O)₂CF₃ | Me |
| G-1869 | Me | H | CH₂CH(Me)N(OMe)S(=O)₂Ph(2-NO₂) | Me |
| G-1870 | Me | H | CH₂CH(Me)N(OMe)S(=O)₂Ph(4-NO₂) | Me |
| G-1871 | Me | H | CH₂CH₂NHC(=O)O(*t*-Bu) | Me |
| G-1872 | Me | H | CH₂CH₂CH₂NHC(=O)O(*t*-Bu) | Me |
| G-1873 | Me | H | CH₂CH₂CH₂CH₂NHC(=O)O(*t*-Bu) | Me |
| G-1874 | Me | H | CH₂CH₂CH₂CH₂NHC(=O)OCH(CH₃)₂ | Me |
| G-1875 | Me | H | CH₂CH(Me)NHC(=O)O(*t*-Bu) | Me |
| G-1876 | Me | H | CH₂C*H(Me)NHC(=O)O(*t*-Bu) : (R) | Me |
| G-1877 | Me | H | CH₂C*H(Me)NHC(=O)O(*t*-Bu) : (S) | Me |
| G-1878 | Me | H | CH₂CH(Et)NHC(=O)O(*t*-Bu) | Me |
| G-1879 | Me | H | CH₂CH(*i*-Pr)NHC(=O)O(*t*-Bu) | Me |
| G-1880 | Me | H | CH(Me)CH₂NHC(=O)O(*t*-Bu) | Me |
| G-1881 | Me | H | CH(Et)CH₂NHC(=O)O(*t*-Bu) | Me |
| G-1882 | Me | H | CH(*i*-Pr)CH₂NHC(=O)O(*t*-Bu) | Me |
| G-1883 | Me | H | CH₂CH(Me)CH₂NHC(=O)O(*t*-Bu) | Me |
| G-1884 | Me | H | CH₂CH₂SCHF₂ | Me |
| G-1885 | Me | H | CH₂CH₂CH₂SCHF₂ | Me |
| G-1886 | Me | H | CH₂CH₂CH₂CH₂SCHF₂ | Me |

**[Table 1163]**

| Compound Number | R³ | R⁴ | R⁵ | R¹¹ |
|---|---|---|---|---|
| G-1887 | Me | H | CH₂CH₂CH₂CH₂CH₂SCHF₂ | Me |
| G-1888 | Me | H | CH₂CH₂CH₂CH₂CH₂CH₂SCHF₂ | Me |
| G-1889 | Me | H | CH₂CH₂CH₂CH₂CH₂CH₂CH₂SCHF₂ | Me |
| G-1890 | Me | H | CH₂CH₂SCF₃ | Me |
| G-1891 | Me | H | CH₂CH₂CH₂SCF₃ | Me |
| G-1892 | Me | H | CH₂CH₂CH₂CH₂SCF₃ | Me |
| G-1893 | Me | H | CH₂CH₂CH₂CH₂CH₂SCF₃ | Me |
| G-1894 | Me | H | CH₂CH₂CH₂CH₂CH₂CH₂SCF₃ | Me |
| G-1895 | Me | H | CH₂CH₂CH₂CH₂CH₂CH₂CH₂SCF₃ | Me |
| G-1896 | Me | H | CH₂CH₂CH₂CH₂CH₂CH₂CH₂CH₂SCF₃ | Me |
| G-1897 | Me | H | CH₂CH(Me)SCF₃ | Me |
| G-1898 | Me | H | CH(Me)CH₂SCF₃ | Me |
| G-1899 | Me | H | CH₂CH₂CH(Me)SCF₃ | Me |
| G-1900 | Me | H | CH₂CH(Me)CH₂SCF₃ | Me |
| G-1901 | Me | H | CH(Me)CH₂CH₂SCF₃ | Me |
| G-1902 | Me | H | CH₂CH₂CH₂CH(Me)SCF₃ | Me |
| G-1903 | Me | H | CH₂CH₂CH(Me)CH₂SCF₃ | Me |
| G-1904 | Me | H | CH₂CH(Me)CH₂CH₂SCF₃ | Me |
| G-1905 | Me | H | CH(Me)CH₂CH₂CH₂SCF₃ | Me |
| G-1906 | Me | H | CH₂CH₂NHS(=O)₂CF₃ | Me |
| G-1907 | Me | H | CH₂CH₂CH₂NHS(=O)₂CF₃ | Me |
| G-1908 | Me | H | CH₂CH₂CH₂CH₂NHS(=O)₂CF₃ | Me |
| G-1909 | Me | H | CH₂CH₂CH₂CH₂CH₂NHS(=O)₂CF₃ | Me |
| G-1910 | Me | H | CH₂CH₂N(Me)S(=O)₂CF₃ | Me |
| G-1911 | Me | H | CH₂CH₂N(Et)S(=O)₂CF₃ | Me |
| G-1912 | Me | H | CH₂CH₂N(CH₂CN)S(=O)₂CF₃ | Me |
| G-1913 | Me | H | CH₂CH₂N(CH₂OMe)S(=O)₂CF₃ | Me |
| G-1914 | Me | H | CH₂CH₂CH₂N(Me)S(=O)₂CF₃ | Me |
| G-1915 | Me | H | CH₂CH₂CH₂N(Et)S(=O)₂CF₃ | Me |
| G-1916 | Me | H | CH₂CH₂CH₂N(CH₂CN)S(=O)₂CF₃ | Me |
| G-1917 | Me | H | CH₂CH₂CH₂N(CH₂OMe)S(=O)₂CF₃ | Me |
| G-1918 | Me | H | CH₂CH₂CH₂CH₂N(Me)S(=O)₂CF₃ | Me |
| G-1919 | Me | H | CH₂CH₂CH₂CH₂N(Et)S(=O)₂CF₃ | Me |
| G-1920 | Me | H | CH₂CH₂CH₂CH₂N(CH₂CN)S(=O)₂CF₃ | Me |
| G-1921 | Me | H | CH₂CH₂CH₂CH₂N(CH₂OMe)S(=O)₂CF₃ | Me |
| G-1922 | Me | H | CH₂CH(Me)NHS(=O)₂CF₃ | Me |
| G-1923 | Me | H | CH₂CH(Me)NHS(=O)₂CF₃ : (R) | Me |
| G-1924 | Me | H | CH₂CH(Me)NHS(=O)₂CF₃ : (S) | Me |
| G-1925 | Me | H | CH(Me)CH₂NHS(=O)₂CF₃ | Me |
| G-1926 | Me | H | CH₂CH₂CH(Me)NHS(=O)₂CF₃ | Me |
| G-1927 | Me | H | CH₂CH(Me)CH₂NHS(=O)₂CF₃ | Me |
| G-1928 | Me | H | CH(Me)CH₂CH₂NHS(=O)₂CF₃ | Me |
| G-1929 | Me | H | CH₂CH(Me)N(Me)S(=O)₂CF₃ | Me |
| G-1930 | Me | H | CH(Me)CH₂N(Me)S(=O)₂CF₃ | Me |

**[Table 1164]**

| Compound Number | R³ | R⁴ | R⁵ | R¹¹ |
|---|---|---|---|---|
| G-1931 | Me | H | CH₂CH₂CH(Me)N(Me)S(=O)₂CF₃ | Me |
| G-1932 | Me | H | CH₂CH(Me)CH₂N(Me)S(=O)₂CF₃ | Me |
| G-1933 | Me | H | CH(Me)CH₂CH₂N(Me)S(=O)₂CF₃ | Me |
| G-1934 | Me | H | CH₂Ph | Me |
| G-1935 | Me | H | CH₂Ph(2-CF₃) | Me |
| G-1936 | Me | H | CH₂Ph(3-CF₃) | Me |
| G-1937 | Me | H | CH₂Ph(4-CF₃) | Me |
| G-1938 | Me | H | CH₂Ph(2-OCF₃) | Me |
| G-1939 | Me | H | CH₂Ph(3-OCF₃) | Me |
| G-1940 | Me | H | CH₂Ph(4-OCF₃) | Me |
| G-1941 | Me | H | CH₂Ph(2-SCF₃) | Me |
| G-1942 | Me | H | CH₂Ph(3-SCF₃) | Me |
| G-1943 | Me | H | CH₂Ph(4-SCF₃) | Me |
| G-1944 | Me | H | CH₂Ph(3-CH₂SCF₃) | Me |
| G-1945 | Me | H | CH₂Ph(4-F) | Me |
| G-1946 | Me | H | CH₂Ph(4-Cl) | Me |
| G-1947 | Me | H | CH₂Ph(4-Br) | Me |
| G-1948 | Me | H | CH₂Ph(4-Me) | Me |
| G-1949 | Me | H | CH₂Ph[4-(*t*-Bu)] | Me |
| G-1950 | Me | H | CH₂Ph(4-CN) | Me |
| G-1951 | Me | H | CH₂Ph(4-NO₂) | Me |
| G-1952 | Me | H | CH₂Ph(4-OCHF₂) | Me |
| G-1953 | Me | H | CH₂Ph(4-SCHF₂) | Me |
| G-1954 | Me | H | CH₂Ph(4-CH₂SCF₃) | Me |
| G-1955 | Me | H | CH₂Ph[4-CF(CF₃)₂] | Me |
| G-1956 | Me | H | CH₂Ph(4-CH₂SCF₃) | Me |
| G-1957 | Me | H | CH₂Ph[4-Ph(4-CF₃)] | Me |
| G-1958 | Me | H | CH₂Ph(2,4-Cl₂) | Me |
| G-1959 | Me | H | CH₂Ph[2,5-(CF₃)₂] | Me |
| G-1960 | Me | H | CH₂Ph(3,4-Cl₂) | Me |
| G-1961 | Me | H | CH₂Ph(3-CF₃-4-F) | Me |
| G-1962 | Me | H | CH₂Ph(3-CF₃-4-Cl) | Me |
| G-1963 | Me | H | CH₂Ph(3-F-4-CF₃) | Me |
| G-1964 | Me | H | CH₂Ph(2,4,6-F₃) | Me |
| G-1965 | Me | H | CH₂Ph(3,4,5-F₃) | Me |
| G-1966 | Me | H | CH₂Ph(2,3,4-F₃) | Me |
| G-1967 | Me | H | CH₂Ph(3,4,5-Cl₃) | Me |
| G-1968 | Me | H | CH₂CH₂Ph | Me |
| G-1969 | Me | H | CH₂CH₂Ph(4-F) | Me |
| G-1970 | Me | H | CH₂CH₂Ph(4-Cl) | Me |
| G-1971 | Me | H | CH₂CH₂Ph(4-Br) | Me |
| G-1972 | Me | H | CH₂CH₂Ph[4-(*t*-Bu)] | Me |
| G-1973 | Me | H | CH₂CH₂Ph(2-CF₃) | Me |
| G-1974 | Me | H | CH₂CH₂Ph(3-CF₃) | Me |

**[Table 1165]**

| Compound Number | R³ | R⁴ | R⁵ | R¹¹ |
|---|---|---|---|---|
| G-1975 | Me | H | CH₂CH₂Ph(4-CF₃) | Me |
| G-1976 | Me | H | CH₂CH₂Ph[4-CF(CF₃)₂] | Me |
| G-1977 | Me | H | CH₂CH₂Ph[4-(*c*-Pr)] | Me |
| G-1978 | Me | H | CH₂CH₂Ph{4-[*c*-Pr(2,2-F₂)]} | Me |
| G-1979 | Me | H | CH₂CH₂Ph(4-OMe) | Me |
| G-1980 | Me | H | CH₂CH₂Ph(3-OCF₃) | Me |
| G-1981 | Me | H | CH₂CH₂Ph(4-OCHF₂) | Me |
| G-1982 | Me | H | CH₂CH₂Ph(4-OCF₃) | Me |
| G-1983 | Me | H | CH₂CH₂Ph(4-SMe) | Me |
| G-1984 | Me | H | CH₂CH₂Ph(4-SCHF₂) | Me |
| G-1985 | Me | H | CH₂CH₂Ph(4-SCF₃) | Me |
| G-1986 | Me | H | CH₂CH₂Ph[4-S(=O)Me] | Me |
| G-1987 | Me | H | CH₂CH₂Ph[4-S(=O)CF₃] | Me |
| G-1988 | Me | H | CH₂CH₂Ph[4-S(=O)₂Me] | Me |
| G-1989 | Me | H | CH₂CH₂Ph[4-S(=O)₂CF₃] | Me |
| G-1990 | Me | H | CH₂CH₂Ph(4-CH₂SMe) | Me |
| G-1991 | Me | H | CH₂CH₂Ph(4-CH₂SCF₃) | Me |
| G-1992 | Me | H | CH₂CH₂Ph[4-OS(=O)₂Me] | Me |
| G-1993 | Me | H | CH₂CH₂Ph[4-OS(=O)₂CF₃] | Me |
| G-1994 | Me | H | CH₂CH₂Ph[4-Ph(4-CF₃)] | Me |
| G-1995 | Me | H | CH₂CH₂Ph(4-CH₂Ph) | Me |
| G-1996 | Me | H | CH₂CH₂Ph(4-OCH₂Ph) | Me |
| G-1997 | Me | H | CH₂CH₂Ph(4-CN) | Me |
| G-1998 | Me | H | CH₂CH₂Ph(4-NO₂) | Me |
| G-1999 | Me | H | CH₂CH₂Ph(2,4-Cl₂) | Me |
| G-2000 | Me | H | CH₂CH₂Ph(3,4-Cl₂) | Me |
| G-2001 | Me | H | CH₂CH₂Ph(3-CF₃-4-F) | Me |
| G-2002 | Me | H | CH₂CH₂Ph(2-CF₃-4-F) | Me |
| G-2003 | Me | H | CH₂CH₂Ph(3-F-4-CF₃) | Me |
| G-2004 | Me | H | CH₂CH₂Ph(2-F-4-CF₃) | Me |
| G-2005 | Me | H | CH₂CH₂Ph(3-Cl-4-OCHF₂) | Me |
| G-2006 | Me | H | CH₂CH₂Ph(3,4,5-Cl₃) | Me |
| G-2007 | Me | H | CH₂CH₂Ph(2,3,4-F₃) | Me |
| G-2008 | Me | H | CH₂CH₂Ph(2,4,5-F₃) | Me |
| G-2009 | Me | H | CH₂CH₂Ph(3,4,5-F₃) | Me |
| G-2010 | Me | H | CH₂CH₂Ph(2,4,6-F₃) | Me |
| G-2011 | Me | H | CH₂CH₂CH₂Ph | Me |
| G-2012 | Me | H | CH₂CH₂CH₂Ph(3-CF₃) | Me |
| G-2013 | Me | H | CH₂CH₂CH₂Ph(4-CF₃) | Me |
| G-2014 | Me | H | CH₂CH₂CH₂Ph(4-F) | Me |
| G-2015 | Me | H | CH₂CH₂CH₂Ph[4-(*t*-Bu)] | Me |
| G-2016 | Me | H | CH₂CH₂CH₂Ph(4-CN) | Me |
| G-2017 | Me | H | CH₂CH₂CH₂Ph(4-CF₃) | Me |
| G-2018 | Me | H | CH₂CH₂CH₂Ph(4-OCHF₂) | Me |

**[Table 1166]**

| Compound Number | R³ | R⁴ | R⁵ | R¹¹ |
|---|---|---|---|---|
| G-2019 | Me | H | CH₂CH₂CH₂Ph(4-OCF₃) | Me |
| G-2020 | Me | H | CH₂CH₂CH₂Ph(4-SCHF₂) | Me |
| G-2021 | Me | H | CH₂CH₂CH₂Ph(4-SCF₃) | Me |
| G-2022 | Me | H | CH₂CH₂CH₂Ph[4-CF(CF₃)₂] | Me |
| G-2023 | Me | H | CH₂CH₂CH₂Ph(3,4,5-F₃) | Me |
| G-2024 | Me | H | CH₂CH₂CH₂Ph(2,4,6-F₃) | Me |
| G-2025 | Me | H | CH₂CH₂CH₂CH₂Ph | Me |
| G-2026 | Me | H | CH₂CH₂CH₂CH₂Ph(4-F) | Me |
| G-2027 | Me | H | CH₂CH₂CH₂CH₂Ph(4-CF₃) | Me |
| G-2028 | Me | H | CH₂CH₂CH₂CH₂Ph(4-OCF₃) | Me |
| G-2029 | Me | H | CH₂CH₂CH₂CH₂Ph(4-SCF₃) | Me |
| G-2030 | Me | H | CH₂CH₂CH₂CH₂CH₂Ph | Me |
| G-2031 | Me | H | CH₂CH₂CH₂CH₂CH₂CH₂Ph | Me |
| G-2032 | Me | H | CH₂CF₂Ph(4-F) | Me |
| G-2033 | Me | H | CH₂CF₂Ph(4-CF₃) | Me |
| G-2034 | Me | H | CH₂CF₂Ph(3,4,5-F₃) | Me |
| G-2035 | CF₃ | H | CH₂CH(Me)NH₂ | Me |
| G-2036 | CF₃ | H | CH₂C*H(Me)NH₂, HCl | Me |
| G-2037 | CF₃ | H | CH₂C*H(Me)NH₂: (R) | Me |
| G-2038 | CF₃ | H | CH₂C*H(Me)NH₂, HCl: (R) | Me |
| G-2039 | CF₃ | H | CH₂C*H(Me)NH₂: (S) | Me |
| G-2040 | CF₃ | H | CH₂C*H(Me)NH₂, HCl: (S) | Me |
| G-2041 | CF₃ | H | CH₂CH(Me)NHC(=O)O(*t*-Bu) | Me |
| G-2042 | CF₃ | H | CH₂C*H(Me)NHC(=O)O(*t*-Bu) : (R) | Me |
| G-2043 | CF₃ | H | CH₂C*H(Me)NHC(=O)O(*t*-Bu) : (S) | Me |
| G-2044 | CF₃ | H | CH₂CH(Me)NHS(=O)₂CF₃ | Me |
| G-2045 | CF₃ | H | CH₂CH(Me)NHS(=O)₂CF₃ : (R) | Me |
| G-2046 | CF₃ | H | CH₂CH(Me)NHS(=O)₂CF₃ : (S) | Me |
| G-2047 | OCF₃ | H | CH₂CH(Me)NH₂ | Me |
| G-2048 | OCF₃ | H | CH₂C*H(Me)NH₂, HCl | Me |
| G-2049 | OCF₃ | H | CH₂C*H(Me)NH₂: (R) | Me |
| G-2050 | OCF₃ | H | CH₂C*H(Me)NH₂, HCl: (R) | Me |
| G-2051 | OCF₃ | H | CH₂C*H(Me)NH₂: (S) | Me |
| G-2052 | OCF₃ | H | CH₂C*H(Me)NH₂, HCl: (S) | Me |
| G-2053 | OCF₃ | H | CH₂CH(Me)NHC(=O)O(*t*-Bu) | Me |
| G-2054 | OCF₃ | H | CH₂C*H(Me)NHC(=O)O(*t*-Bu) : (R) | Me |
| G-2055 | OCF₃ | H | CH₂C*H(Me)NHC(=O)O(*t*-Bu) : (S) | Me |
| G-2056 | OCF₃ | H | CH₂CH(Me)NHS(=O)₂CF₃ | Me |
| G-2057 | OCF₃ | H | CH₂CH(Me)NHS(=O)₂CF₃: (R) | Me |
| G-2058 | OCF₃ | H | CH₂CH(Me)NHS(=O)₂CF₃ : (S) | Me |
| G-2059 | Cl | Cl | CH₂CH₂SCHF₂ | Me |
| G-2060 | Cl | Cl | CH₂CH₂CH₂SCHF₂ | Me |
| G-2061 | Cl | Cl | CH₂CH₂CH₂CH₂SCHF₂ | Me |
| G-2062 | Cl | Cl | CH₂CH₂CH₂CH₂CH₂SCHF₂ | Me |

**[Table 1167]**

| Compound Number | R³ | R⁴ | R⁵ | R¹¹ |
|---|---|---|---|---|
| G-2063 | Cl | Cl | CH₂CH₂CH₂CH₂CH₂CH₂SCHF₂ | Me |
| G-2064 | Cl | Cl | CH₂CH₂CH_{2C}H₂CH₂CH₂CH₂SCHF₂ | Me |
| G-2065 | Cl | Cl | CH₂CH₂SCF₃ | Me |
| G-2066 | Cl | Cl | CH₂CH₂CH₂SCF₃ | Me |
| G-2067 | Cl | Cl | CH₂CH₂CH₂CH₂SCF₃ | Me |
| G-2068 | Cl | Cl | CH₂CH₂CH₂CH₂CH₂SCF₃ | Me |
| G-2069 | Cl | Cl | CH₂CH₂CH₂CH₂CH₂CH₂SCF₃ | Me |
| G-2070 | Cl | Cl | CH₂CH₂CH₂CH₂CH₂CH₂CH₂SCF₃ | Me |
| G-2071 | Cl | Cl | CH₂CH₂CH₂CH₂CH₂CH₂CH₂CH₂SCF₃ | Me |
| G-2072 | Cl | Cl | CH₂CH(Me)SCF₃ | Me |
| G-2073 | Cl | Cl | CH(Me)CH₂SCF₃ | Me |
| G-2074 | Cl | Cl | CH₂CH₂CH(Me)SCF₃ | Me |
| G-2075 | Cl | Cl | CH₂CH(Me)CH₂SCF₃ | Me |
| G-2076 | Cl | Cl | CH(Me)CH₂CH₂SCF₃ | Me |
| G-2077 | Cl | Cl | CH₂CH₂CH₂CH(Me)SCF₃ | Me |
| G-2078 | Cl | Cl | CH₂CH₂CH(Me)CH₂SCF₃ | Me |
| G-2079 | Cl | Cl | CH₂CH(Me)CH₂CH₂SCF₃ | Me |
| G-2080 | Cl | Cl | CH(Me)CH₂CH₂CH₂SCF₃ | Me |
| G-2081 | Cl | Cl | CH₂CH₂NHS(=O)₂CF₃ | Me |
| G-2082 | Cl | Cl | CH₂CH₂CH₂NHS(=O)₂CF₃ | Me |
| G-2083 | Cl | Cl | CH₂CH₂CH₂CH₂NHS(=O)₂CF₃ | Me |
| G-2084 | Cl | Cl | CH₂CH₂CH₂CH₂CH₂NHS(=O)₂CF₃ | Me |
| G-2085 | Cl | Cl | CH₂CH₂N(Me)S(=O)₂CF₃ | Me |
| G-2086 | Cl | Cl | CH₂CH₂N(Et)S(=O)₂CF₃ | Me |
| G-2087 | Cl | Cl | CH₂CH₂N(CH₂CN)S(=O)₂CF₃ | Me |
| G-2088 | Cl | Cl | CH₂CH₂N(CH₂OMe)S(=O)₂CF₃ | Me |
| G-2089 | Cl | Cl | CH₂CH₂CH₂N(Me)S(=O)₂CF₃ | Me |
| G-2090 | Cl | Cl | CH₂CH₂CH₂N(Et)S(=O)₂CF₃ | Me |
| G-2091 | Cl | Cl | CH₂CH₂CH₂N(CH₂CN)S(=O)₂CF₃ | Me |
| G-2092 | Cl | Cl | CH₂CH₂CH₂N(CH₂OMe)S(=O)₂CF₃ | Me |
| G-2093 | Cl | Cl | CH₂CH₂CH₂CH₂N(Me)S(=O)₂CF₃ | Me |
| G-2094 | Cl | Cl | CH₂CH₂CH₂CH₂N(Et)S(=O)₂CF₃ | Me |
| G-2095 | Cl | Cl | CH₂CH₂CH₂CH₂N(CH₂CN)S(=O)₂CF₃ | Me |
| G-2096 | Cl | Cl | CH₂CH₂CH₂CH₂N(CH₂OMe)S(=O)₂CF₃ | Me |
| G-2097 | Cl | Cl | CH₂CH(Me)NHS(=O)₂CF₃ | Me |
| G-2098 | Cl | Cl | CH(Me)CH₂NHS(=O)₂CF₃ | Me |
| G-2099 | Cl | Cl | CH₂CH₂CH(Me)NHS(=O)₂CF₃ | Me |
| G-2100 | Cl | Cl | CH₂CH(Me)CH₂NHS(=O)₂CF₃ | Me |
| G-2101 | Cl | Cl | CH(Me)CH₂CH₂NHS(=O)₂CF₃ | Me |
| G-2102 | Cl | Cl | CH₂CH(Me)N(Me)S(=O)₂CF₃ | Me |
| G-2103 | Cl | Cl | CH(Me)CH₂N(Me)S(=O)₂CF₃ | Me |
| G-2104 | Cl | Cl | CH₂CH₂CH(Me)N(Me)S(=O)₂CF₃ | Me |
| G-2105 | Cl | Cl | CH₂CH(Me)CH₂N(Me)S(=O)₂CF₃ | Me |
| G-2106 | Cl | Cl | CH(Me)CH₂CH₂N(Me)S(=O)₂CF₃ | Me |

**[Table 1168]**

| Compound Number | R³ | R⁴ | R⁵ | R¹¹ |
|---|---|---|---|---|
| G-2107 | Cl | Cl | CH₂Ph | Me |
| G-2108 | Cl | Cl | CH₂Ph(2-CF₃) | Me |
| G-2109 | Cl | Cl | CH₂Ph(3-CF₃) | Me |
| G-2110 | Cl | Cl | CH₂Ph(4-CF₃) | Me |
| G-2111 | Cl | Cl | CH₂Ph(2-OCF₃) | Me |
| G-2112 | Cl | Cl | CH₂Ph(3-OCF₃) | Me |
| G-2113 | Cl | Cl | CH₂Ph(4-OCF₃) | Me |
| G-2114 | Cl | Cl | CH₂Ph(2-SCF₃) | Me |
| G-2115 | Cl | Cl | CH₂Ph(3-SCF₃) | Me |
| G-2116 | Cl | Cl | CH₂Ph(4-SCF₃) | Me |
| G-2117 | Cl | Cl | CH₂Ph(3-CH₂SCF₃) | Me |
| G-2118 | Cl | Cl | CH₂Ph(4-F) | Me |
| G-2119 | Cl | Cl | CH₂Ph(4-Cl) | Me |
| G-2120 | Cl | Cl | CH₂Ph(4-Br) | Me |
| G-2121 | Cl | Cl | CH₂Ph(4-Me) | Me |
| G-2122 | Cl | Cl | CH₂Ph[4-(*t*-Bu)] | Me |
| G-2123 | Cl | Cl | CH₂Ph(4-CN) | Me |
| G-2124 | Cl | Cl | CH₂Ph(4-NO₂) | Me |
| G-2125 | Cl | Cl | CH₂Ph(4-OCHF₂) | Me |
| G-2126 | Cl | Cl | CH₂Ph(4-SCHF₂) | Me |
| G-2127 | Cl | Cl | CH₂Ph(4-CH₂SCF₃) | Me |
| G-2128 | Cl | Cl | CH₂Ph[4-CF(CF₃)₂] | Me |
| G-2129 | Cl | Cl | CH₂Ph(4-CH₂SCF₃) | Me |
| G-2130 | Cl | Cl | CH₂Ph[4-Ph(4-CF₃)] | Me |
| G-2131 | Cl | Cl | CH₂Ph(2,4-Cl₂) | Me |
| G-2132 | Cl | Cl | CH₂Ph[2,5-(CF₃)₂] | Me |
| G-2133 | Cl | Cl | CH₂Ph(3,4-Cl₂) | Me |
| G-2134 | Cl | Cl | CH₂Ph(3-CF₃-4-F) | Me |
| G-2135 | Cl | Cl | CH₂Ph(3-CF₃-4-Cl) | Me |
| G-2136 | Cl | Cl | CH₂Ph(3-F-4-CF₃) | Me |
| G-2137 | Cl | Cl | CH₂Ph(2,4,6-F₃) | Me |
| G-2138 | Cl | Cl | CH₂Ph(3,4,5-F₃) | Me |
| G-2139 | Cl | Cl | CH₂Ph(2,3,4-F₃) | Me |
| G-2140 | Cl | Cl | CH₂Ph(3,4,5-Cl₃) | Me |
| G-2141 | Cl | Cl | CH₂CH₂Ph | Me |
| G-2142 | Cl | Cl | CH₂CH₂Ph(4-F) | Me |
| G-2143 | Cl | Cl | CH₂CH₂Ph(4-Cl) | Me |
| G-2144 | Cl | Cl | CH₂CH₂Ph(4-Br) | Me |
| G-2145 | Cl | Cl | CH₂CH₂Ph[4-(*t*-Bu)] | Me |
| G-2146 | Cl | Cl | CH₂CH₂Ph(2-CF₃) | Me |
| G-2147 | Cl | Cl | CH₂CH₂Ph(3-CF₃) | Me |
| G-2148 | Cl | Cl | CH₂CH₂Ph(4-CF₃) | Me |
| G-2149 | Cl | Cl | CH₂CH₂Ph[4-CF(CF₃)₂] | Me |
| G-2150 | Cl | Cl | CH₂CH₂Ph[4-(*c*-Pr)] | Me |

**[Table 1169]**

| Compound Number | R³ | R⁴ | R⁵ | R¹¹ |
|---|---|---|---|---|
| G-2151 | Cl | Cl | CH₂CH₂Ph{4-[*c*-Pr(2,2-F₂)]} | Me |
| G-2152 | Cl | Cl | CH₂CH₂Ph(4-OMe) | Me |
| G-2153 | Cl | Cl | CH₂CH₂Ph(3-OCF₃) | Me |
| G-2154 | Cl | Cl | CH₂CH₂Ph(4-OCHF₂) | Me |
| G-2155 | Cl | Cl | CH₂CH₂Ph(4-OCF₃) | Me |
| G-2156 | Cl | Cl | CH₂CH₂Ph(4-SMe) | Me |
| G-2157 | Cl | Cl | CH₂CH₂Ph(4-SCHF₂) | Me |
| G-2158 | Cl | Cl | CH₂CH₂Ph(4-SCF₃) | Me |
| G-2159 | Cl | Cl | CH₂CH₂Ph[4-S(=O)Me] | Me |
| G-2160 | Cl | Cl | CH₂CH₂Ph[4-S(=O)CF₃] | Me |
| G-2161 | Cl | Cl | CH₂CH₂Ph[4-S(=O)₂Me] | Me |
| G-2162 | Cl | Cl | CH₂CH₂Ph[4-S(=O)₂CF₃] | Me |
| G-2163 | Cl | Cl | CH₂CH₂Ph(4-CH₂SMe) | Me |
| G-2164 | Cl | Cl | CH₂CH₂Ph(4-CH₂SCF₃) | Me |
| G-2165 | Cl | Cl | CH₂CH₂Ph[4-OS(=O)₂Me] | Me |
| G-2166 | Cl | Cl | CH₂CH₂Ph[4-OS(=O)₂CF₃] | Me |
| G-2167 | Cl | Cl | CH₂CH₂Ph[4-Ph(4-CF₃)] | Me |
| G-2168 | Cl | Cl | CH₂CH₂Ph(4-CH₂Ph) | Me |
| G-2169 | Cl | Cl | CH₂CH₂Ph(4-OCH₂Ph) | Me |
| G-2170 | Cl | Cl | CH₂CH₂Ph(4-CN) | Me |
| G-2171 | Cl | Cl | CH₂CH₂Ph(4-NO₂) | Me |
| G-2172 | Cl | Cl | CH₂CH₂Ph(2,4-Cl₂) | Me |
| G-2173 | Cl | Cl | CH₂CH₂Ph(3,4-Cl₂) | Me |
| G-2174 | Cl | Cl | CH₂CH₂Ph(3-CF₃-4-F) | Me |
| G-2175 | Cl | Cl | CH₂CH₂Ph(2-CF₃-4-F) | Me |
| G-2176 | Cl | Cl | CH₂CH₂Ph(3-F-4-CF₃) | Me |
| G-2177 | Cl | Cl | CH₂CH₂Ph(2-F-4-CF₃) | Me |
| G-2178 | Cl | Cl | CH₂CH₂Ph(3-Cl-4-OCHF₂) | Me |
| G-2179 | Cl | Cl | CH₂CH₂Ph(3,4,5-Cl₃) | Me |
| G-2180 | Cl | Cl | CH₂CH₂Ph(2,3,4-F₃) | Me |
| G-2181 | Cl | Cl | CH₂CH₂Ph(2,4,5-F₃) | Me |
| G-2182 | Cl | Cl | CH₂CH₂Ph(3,4,5-F₃) | Me |
| G-2183 | Cl | Cl | CH₂CH₂Ph(2,4,6-F₃) | Me |
| G-2184 | Cl | Cl | CH₂CH₂CH₂Ph | Me |
| G-2185 | Cl | Cl | CH₂CH₂CH₂Ph(3-CF₃) | Me |
| G-2186 | Cl | Cl | CH₂CH₂CH₂Ph(4-CF₃) | Me |
| G-2187 | Cl | Cl | CH₂CH₂CH₂Ph(4-F) | Me |
| G-2188 | Cl | Cl | CH₂CH₂CH₂Ph[4-(t-Bu)] | Me |
| G-2189 | Cl | Cl | CH₂CH₂CH₂Ph(4-CN) | Me |
| G-2190 | Cl | Cl | CH₂CH₂CH₂Ph(4-CF₃) | Me |
| G-2191 | Cl | Cl | CH₂CH₂CH₂Ph(4-OCHF₂) | Me |
| G-2192 | Cl | Cl | CH₂CH₂CH₂Ph(4-OCF₃) | Me |
| G-2193 | Cl | Cl | CH₂CH₂CH₂Ph(4-SCHF₂) | Me |
| G-2194 | Cl | Cl | CH₂CH₂CH₂Ph(4-SCF₃) | Me |

**[Table 1170]**

| Compound Number | R³ | R⁴ | R⁵ | R¹¹ |
|---|---|---|---|---|
| G-2195 | Cl | Cl | CH₂CH₂CH₂Ph[4-CF(CF₃)₂] | Me |
| G-2196 | Cl | Cl | CH₂CH₂CH₂Ph(3,4,5-F₃) | Me |
| G-2197 | Cl | Cl | CH₂CH₂CH₂Ph(2,4,6-F₃) | Me |
| G-2198 | Cl | Cl | CH₂CH₂CH₂CH₂Ph | Me |
| G-2199 | Cl | Cl | CH₂CH₂CH₂CH₂Ph(4-F) | Me |
| G-2200 | Cl | Cl | CH₂CH₂CH₂CH₂Ph(4-CF₃) | Me |
| G-2201 | Cl | Cl | CH₂CH₂CH₂CH₂Ph(4-OCF₃) | Me |
| G-2202 | Cl | Cl | CH₂CH₂CH₂CH₂Ph(4-SCF₃) | Me |
| G-2203 | Cl | Cl | CH₂CH₂CH₂CH₂CH₂Ph | Me |
| G-2204 | Cl | Cl | CH₂CH₂CH₂CH₂CH₂CH₂Ph | Me |
| G-2205 | Cl | Cl | CH₂CF₂Ph(4-F) | Me |
| G-2206 | Cl | Cl | CH₂CF₂Ph(4-CF₃) | Me |
| G-2207 | Cl | Cl | CH₂CF₂Ph(3,4,5-F₃) | Me |
| G-2208 | Me | Cl | CH₂CH₂SCHF₂ | Me |
| G-2209 | Me | Cl | CH₂CH₂CH₂SCHF₂ | Me |
| G-2210 | Me | Cl | CH₂CH₂CH₂CH₂SCHF₂ | Me |
| G-2211 | Me | Cl | CH₂CH₂CH₂CH₂CH₂SCHF₂ | Me |
| G-2212 | Me | Cl | CH₂CH₂CH₂CH₂CH₂CH₂SCHF₂ | Me |
| G-2213 | Me | Cl | CH₂CH₂CH₂CH₂CH₂CH₂CH₂SCHF₂ | Me |
| G-2214 | Me | Cl | CH₂CH₂SCF₃ | Me |
| G-2215 | Me | Cl | CH₂CH₂CH₂SCF₃ | Me |
| G-2216 | Me | Cl | CH₂CH₂CH₂CH₂SCF₃ | Me |
| G-2217 | Me | Cl | CH₂CH₂CH₂CH₂CH₂SCF₃ | Me |
| G-2218 | Me | Cl | CH₂CH₂CH₂CH₂CH₂CH₂SCF₃ | Me |
| G-2219 | Me | Cl | CH₂CH₂CH₂CH₂CH₂CH₂CH₂SCF₃ | Me |
| G-2220 | Me | Cl | CH₂CH₂CH₂CH₂CH₂CH₂CH₂CH₂SCF₃ | Me |
| G-2221 | Me | Cl | CH₂CH(Me)SCF₃ | Me |
| G-2222 | Me | Cl | CH(Me)CH₂SCF₃ | Me |
| G-2223 | Me | Cl | CH₂CH₂CH(Me)SCF₃ | Me |
| G-2224 | Me | Cl | CH₂CH(Me)CH₂SCF₃ | Me |
| G-2225 | Me | Cl | CH(Me)CH₂CH₂SCF₃ | Me |
| G-2226 | Me | Cl | CH₂CH₂CH₂CH(Me)SCF₃ | Me |
| G-2227 | Me | Cl | CH₂CH₂CH(Me)CH₂SCF₃ | Me |
| G-2228 | Me | Cl | CH₂CH(Me)CH₂CH₂SCF₃ | Me |
| G-2229 | Me | Cl | CH(Me)CH₂CH₂CH₂SCF₃ | Me |
| G-2230 | Me | Cl | CH₂CH₂NHS(=O)₂CF₃ | Me |
| G-2231 | Me | Cl | CH₂CH₂CH₂NHS(=O)₂CF₃ | Me |
| G-2232 | Me | Cl | CH₂CH₂CH₂CH₂NHS(=O)₂CF₃ | Me |
| G-2233 | Me | Cl | CH₂CH₂CH₂CH₂CH₂NHS(=O)₂CF₃ | Me |
| G-2234 | Me | Cl | CH₂CH₂N(Me)S(=O)₂CF₃ | Me |
| G-2235 | Me | Cl | CH₂CH₂N(Et)S(=O)₂CF₃ | Me |
| G-2236 | Me | Cl | CH₂CH₂N(CH₂CN)S(=O)₂CF₃ | Me |
| G-2237 | Me | Cl | CH₂CH₂N(CH₂OMe)S(=O)₂CF₃ | Me |
| G-2238 | Me | Cl | CH₂CH₂CH₂N(Me)S(=O)₂CF₃ | Me |

**[Table 1171]**

| Compound Number | R³ | R⁴ | R⁵ | R¹¹ |
|---|---|---|---|---|
| G-2239 | Me | Cl | CH₂CH₂CH₂N(Et)S(=O)₂CF₃ | Me |
| G-2240 | Me | Cl | CH₂CH₂CH₂N(CH₂CN)S(=O)₂CF₃ | Me |
| G-2241 | Me | Cl | CH₂CH₂CH₂N(CH₂OMe)S(=O)₂CF₃ | Me |
| G-2242 | Me | Cl | CH₂CH₂CH₂CH₂N(Me)S(=O)₂CF₃ | Me |
| G-2243 | Me | Cl | CH₂CH₂CH₂CH₂N(Et)S(=O)₂CF₃ | Me |
| G-2244 | Me | Cl | CH₂CH₂CH₂CH₂N(CH₂CN)S(=O)₂CF₃ | Me |
| G-2245 | Me | Cl | CH₂CH₂CH₂CH₂N(CH₂OMe)S(=O)₂CF₃ | Me |
| G-2246 | Me | Cl | CH₂CH(Me)NHS(=O)₂CF₃ | Me |
| G-2247 | Me | Cl | CH(Me)CH₂NHS(=O)₂CF₃ | Me |
| G-2248 | Me | Cl | CH₂CH₂CH(Me)NHS(=O)₂CF₃ | Me |
| G-2249 | Me | Cl | CH₂CH(Me)CH₂NHS(=O)₂CF₃ | Me |
| G-2250 | Me | Cl | CH(Me)CH₂CH₂NHS(=O)₂CF₃ | Me |
| G-2251 | Me | Cl | CH₂CH(Me)N(Me)S(=O)₂CF₃ | Me |
| G-2252 | Me | Cl | CH(Me)CH₂N(Me)S(=O)₂CF₃ | Me |
| G-2253 | Me | Cl | CH₂CH₂CH(Me)N(Me)S(=O)₂CF₃ | Me |
| G-2254 | Me | Cl | CH₂CH(Me)CH₂N(Me)S(=O)₂CF₃ | Me |
| G-2255 | Me | Cl | CH(Me)CH₂CH₂N(Me)S(=O)₂CF₃ | Me |
| G-2256 | Me | Cl | CH₂Ph | Me |
| G-2257 | Me | Cl | CH₂Ph(2-CF₃) | Me |
| G-2258 | Me | Cl | CH₂Ph(3-CF₃) | Me |
| G-2259 | Me | Cl | CH₂Ph(4-CF₃) | Me |
| G-2260 | Me | Cl | CH₂Ph(2-OCF₃) | Me |
| G-2261 | Me | Cl | CH₂Ph(3-OCF₃) | Me |
| G-2262 | Me | Cl | CH₂Ph(4-OCF₃) | Me |
| G-2263 | Me | Cl | CH₂Ph(2-SCF₃) | Me |
| G-2264 | Me | Cl | CH₂Ph(3-SCF₃) | Me |
| G-2265 | Me | Cl | CH₂Ph(4-SCF₃) | Me |
| G-2266 | Me | Cl | CH₂Ph(3-CH₂SCF₃) | Me |
| G-2267 | Me | Cl | CH₂Ph(4-F) | Me |
| G-2268 | Me | Cl | CH₂Ph(4-Cl) | Me |
| G-2269 | Me | Cl | CH₂Ph(4-Br) | Me |
| G-2270 | Me | Cl | CH₂Ph(4-Me) | Me |
| G-2271 | Me | Cl | CH₂Ph[4-(*t*-Bu)] | Me |
| G-2272 | Me | Cl | CH₂Ph(4-CN) | Me |
| G-2273 | Me | Cl | CH₂Ph(4-NO₂) | Me |
| G-2274 | Me | Cl | CH₂Ph(4-OCHF₂) | Me |
| G-2275 | Me | Cl | CH₂Ph(4-SCHF₂) | Me |
| G-2276 | Me | Cl | CH₂Ph(4-CH₂SCF₃) | Me |
| G-2277 | Me | Cl | CH₂Ph[4-CF(CF₃)₂] | Me |
| G-2278 | Me | Cl | CH₂Ph(4-CH₂SCF₃) | Me |
| G-2279 | Me | Cl | CH₂Ph[4-Ph(4-CF₃)] | Me |
| G-2280 | Me | Cl | CH₂Ph(2,4-Cl₂) | Me |
| G-2281 | Me | Cl | CH₂Ph[2,5-(CF₃)₂] | Me |
| G-2282 | Me | Cl | CH₂Ph(3,4-Cl₂) | Me |

**[Table 1172]**

| Compound Number | R³ | R⁴ | R⁵ | R¹¹ |
|---|---|---|---|---|
| G-2283 | Me | Cl | CH₂Ph(3-CF₃-4-F) | Me |
| G-2284 | Me | Cl | CH₂Ph(3-CF₃-4-Cl) | Me |
| G-2285 | Me | Cl | CH₂Ph(3-F-4-CF₃) | Me |
| G-2286 | Me | Cl | CH₂Ph(2,4,6-F₃) | Me |
| G-2287 | Me | Cl | CH₂Ph(3,4,5-F₃) | Me |
| G-2288 | Me | Cl | CH₂Ph(2,3,4-F₃) | Me |
| G-2289 | Me | Cl | CH₂Ph(3,4,5-Cl₃) | Me |
| G-2290 | Me | Cl | CH₂CH₂Ph | Me |
| G-2291 | Me | Cl | CH₂CH₂Ph(4-F) | Me |
| G-2292 | Me | Cl | CH₂CH₂Ph(4-Cl) | Me |
| G-2293 | Me | Cl | CH₂CH₂Ph(4-Br) | Me |
| G-2294 | Me | Cl | CH₂CH₂Ph[4-(*t*-Bu)] | Me |
| G-2295 | Me | Cl | CH₂CH₂Ph(2-CF₃) | Me |
| G-2296 | Me | Cl | CH₂CH₂Ph(3-CF₃) | Me |
| G-2297 | Me | Cl | CH₂CH₂Ph(4-CF₃) | Me |
| G-2298 | Me | Cl | CH₂CH₂Ph[4-CF(CF₃)₂] | Me |
| G-2299 | Me | Cl | CH₂CH₂Ph[4-(*c*-Pr)] | Me |
| G-2300 | Me | Cl | CH₂CH₂Ph{4-[*c*-Pr(2,2-F₂)]} | Me |
| G-2301 | Me | Cl | CH₂CH₂Ph(4-OMe) | Me |
| G-2302 | Me | Cl | CH₂CH₂Ph(3-OCF₃) | Me |
| G-2303 | Me | Cl | CH₂CH₂Ph(4-OCHF₂) | Me |
| G-2304 | Me | Cl | CH₂CH₂Ph(4-OCF₃) | Me |
| G-2305 | Me | Cl | CH₂CH₂Ph(4-SMe) | Me |
| G-2306 | Me | Cl | CH₂CH₂Ph(4-SCHF₂) | Me |
| G-2307 | Me | Cl | CH₂CH₂Ph(4-SCF₃) | Me |
| G-2308 | Me | Cl | CH₂CH₂Ph[4-S(=O)Me] | Me |
| G-2309 | Me | Cl | CH₂CH₂Ph[4-S(=O)CF₃] | Me |
| G-2310 | Me | Cl | CH₂CH₂Ph[4-S(=O)₂Me] | Me |
| G-2311 | Me | Cl | CH₂CH₂Ph[4-S(=O)₂CF₃] | Me |
| G-2312 | Me | Cl | CH₂CH₂Ph(4-CH₂SMe) | Me |
| G-2313 | Me | Cl | CH₂CH₂Ph(4-CH₂SCF₃) | Me |
| G-2314 | Me | Cl | CH₂CH₂Ph[4-OS(=O)₂Me] | Me |
| G-2315 | Me | Cl | CH₂CH₂Ph[4-OS(=O)₂CF₃] | Me |
| G-2316 | Me | Cl | CH₂CH₂Ph[4-Ph(4-CF₃)] | Me |
| G-2317 | Me | Cl | CH₂CH₂Ph(4-CH₂Ph) | Me |
| G-2318 | Me | Cl | CH₂CH₂Ph(4-OCH₂Ph) | Me |
| G-2319 | Me | Cl | CH₂CH₂Ph(4-CN) | Me |
| G-2320 | Me | Cl | CH₂CH₂Ph(4-NO₂) | Me |
| G-2321 | Me | Cl | CH₂CH₂Ph(2,4-Cl₂) | Me |
| G-2322 | Me | Cl | CH₂CH₂Ph(3,4-Cl₂) | Me |
| G-2323 | Me | Cl | CH₂CH₂Ph(3-CF₃-4-F) | Me |
| G-2324 | Me | Cl | CH₂CH₂Ph(2-CF₃-4-F) | Me |
| G-2325 | Me | Cl | CH₂CH₂Ph(3-F-4-CF₃) | Me |
| G-2326 | Me | Cl | CH₂CH₂Ph(2-F-4-CF₃) | Me |

**[Table 1173]**

| Compound Number | R³ | R⁴ | R⁵ | R¹¹ |
|---|---|---|---|---|
| G-2327 | Me | Cl | CH₂CH₂Ph(3-Cl-4-OCHF₂) | Me |
| G-2328 | Me | Cl | CH₂CH₂Ph(3,4,5-Cl₃) | Me |
| G-2329 | Me | Cl | CH₂CH₂Ph(2,3,4-F₃) | Me |
| G-2330 | Me | Cl | CH₂CH₂Ph(2,4,5-F₃) | Me |
| G-2331 | Me | Cl | CH₂CH₂Ph(3,4,5-F₃) | Me |
| G-2332 | Me | Cl | CH₂CH₂Ph(2,4,6-F₃) | Me |
| G-2333 | Me | Cl | CH₂CH₂CH₂Ph | Me |
| G-2334 | Me | Cl | CH₂CH₂CH₂Ph(3-CF₃) | Me |
| G-2335 | Me | Cl | CH₂CH₂CH₂Ph(4-CF₃) | Me |
| G-2336 | Me | Cl | CH₂CH₂CH₂Ph(4-F) | Me |
| G-2337 | Me | Cl | CH₂CH₂CH₂Ph[4-(*t*-Bu)] | Me |
| G-2338 | Me | Cl | CH₂CH₂CH₂Ph(4-CN) | Me |
| G-2339 | Me | Cl | CH₂CH₂CH₂Ph(4-CF₃) | Me |
| G-2340 | Me | Cl | CH₂CH₂CH₂Ph(4-OCHF₂) | Me |
| G-2341 | Me | Cl | CH₂CH₂CH₂Ph(4-OCF₃) | Me |
| G-2342 | Me | Cl | CH₂CH₂CH₂Ph(4-SCHF₂) | Me |
| G-2343 | Me | Cl | CH₂CH₂CH₂Ph(4-SCF₃) | Me |
| G-2344 | Me | Cl | CH₂CH₂CH₂Ph[4-CF(CF₃)₂] | Me |
| G-2345 | Me | Cl | CH₂CH₂CH₂Ph(3,4,5-F₃) | Me |
| G-2346 | Me | Cl | CH₂CH₂CH₂Ph(2,4,6-F₃) | Me |
| G-2347 | Me | Cl | CH₂CH₂CH₂CH₂Ph | Me |
| G-2348 | Me | Cl | CH₂CH₂CH₂CH₂Ph(4-F) | Me |
| G-2349 | Me | Cl | CH₂CH₂CH₂CH₂Ph(4-CF₃) | Me |
| G-2350 | Me | Cl | CH₂CH₂CH₂CH₂Ph(4-OCF₃) | Me |
| G-2351 | Me | Cl | CH₂CH₂CH₂CH₂Ph(4-SCF₃) | Me |
| G-2352 | Me | Cl | CH₂CH₂CH₂CH₂CH₂Ph | Me |
| G-2353 | Me | Cl | CH₂CH₂CH₂CH₂CH₂CH₂Ph | Me |
| G-2354 | Me | Cl | CH₂CF₂Ph(4-F) | Me |
| G-2355 | Me | Cl | CH₂CF₂Ph(4-CF₃) | Me |
| G-2356 | Me | Cl | CH₂CF₂Ph(3,4,5-F₃) | Me |
| G-2357 | Me | Me | CH₂CH₂SCHF₂ | Me |
| G-2358 | Me | Me | CH₂CH₂CH₂SCHF₂ | Me |
| G-2359 | Me | Me | CH₂CH₂CH₂CH₂SCHF₂ | Me |
| G-2360 | Me | Me | CH₂CH₂CH₂CH₂CH₂SCHF₂ | Me |
| G-2361 | Me | Me | CH₂CH₂CH₂CH₂CH₂CH₂SCHF₂ | Me |
| G-2362 | Me | Me | CH₂CHCH₂CH₂CH₂CHCH₂SCHF₂ | Me |
| G-2363 | Me | Me | CH₂CH₂SCF₃ | Me |
| G-2364 | Me | Me | CH₂CH₂CH₂SCF₃ | Me |
| G-2365 | Me | Me | CH₂CH₂CH₂CH₂SCF₃ | Me |
| G-2366 | Me | Me | CH₂CH₂CH₂CH₂CH₂SCF₃ | Me |
| G-2367 | Me | Me | CH₂CH₂CH₂CH₂CH₂CH₂SCF₃ | Me |
| G-2368 | Me | Me | CH₂CH₂CH₂CH₂CH₂CH₂CH₂SCF₃ | Me |
| G-2369 | Me | Me | CH₂CH₂CH₂CH₂CH₂CH₂CH₂CH₂SCF₃ | Me |
| G-2370 | Me | Me | CH₂CH(Me)SCF₃ | Me |

**[Table 1174]**

| Compound Number | R³ | R⁴ | R⁵ | R¹¹ |
|---|---|---|---|---|
| G-2371 | Me | Me | CH(Me)CH₂SCF₃ | Me |
| G-2372 | Me | Me | CH₂CH₂CH(Me)SCF₃ | Me |
| G-2373 | Me | Me | CH₂CH(Me)CH₂SCF₃ | Me |
| G-2374 | Me | Me | CH(Me)CH₂CH₂SCF₃ | Me |
| G-2375 | Me | Me | CH₂CH₂CH₂CH(Me)SCF₃ | Me |
| G-2376 | Me | Me | CH₂CH₂CH(Me)CH₂SCF₃ | Me |
| G-2377 | Me | Me | CH₂CH(Me)CH₂CH₂SCF₃ | Me |
| G-2378 | Me | Me | CH(Me)CH₂CH₂CH₂SCF₃ | Me |
| G-2379 | Me | Me | CH₂CH₂NHS(=O)₂CF₃ | Me |
| G-2380 | Me | Me | CH₂CH₂CH₂NHS(=O)₂CF₃ | Me |
| G-2381 | Me | Me | CH₂CH₂CH₂CH₂NHS(=O)₂CF₃ | Me |
| G-2382 | Me | Me | CH₂CH₂CH₂CH₂CH₂NHS(=O)₂CF₃ | Me |
| G-2383 | Me | Me | CH₂CH₂N(Me)S(=O)₂CF₃ | Me |
| G-2384 | Me | Me | CH₂CH₂N(Et)S(=O)₂CF₃ | Me |
| G-2385 | Me | Me | CH₂CH₂N(CH₂CN)S(=O)₂CF₃ | Me |
| G-2386 | Me | Me | CH₂CH₂N(CH₂OMe)S(=O)₂CF₃ | Me |
| G-2387 | Me | Me | CH₂CH₂CH₂N(Me)S(=O)₂CF₃ | Me |
| G-2388 | Me | Me | CH₂CH₂CH₂N(Et)S(=O)₂CF₃ | Me |
| G-2389 | Me | Me | CH₂CH₂CH₂N(CH₂CN)S(=O)₂CF₃ | Me |
| G-2390 | Me | Me | CH₂CH₂CH₂N(CH₂OMe)S(=O)₂CF₃ | Me |
| G-2391 | Me | Me | CH₂CH₂CH₂CH₂N(Me)S(=O)₂CF₃ | Me |
| G-2392 | Me | Me | CH₂CH₂CH₂CH₂N(Et)S(=O)₂CF₃ | Me |
| G-2393 | Me | Me | CH₂CH₂CH₂CH₂N(CH₂CN)S(=O)₂CF₃ | Me |
| G-2394 | Me | Me | CH₂CH₂CH₂CH₂N(CH₂OMe)S(=O)₂CF₃ | Me |
| G-2395 | Me | Me | CH₂CH(Me)NHS(=O)₂CF₃ | Me |
| G-2396 | Me | Me | CH(Me)CH₂NHS(=O)₂CF₃ | Me |
| G-2397 | Me | Me | CH₂CH₂CH(Me)NHS(=O)₂CF₃ | Me |
| G-2398 | Me | Me | CH₂CH(Me)CH₂NHS(=O)₂CF₃ | Me |
| G-2399 | Me | Me | CH(Me)CH₂CH₂NHS(=O)₂CF₃ | Me |
| G-2400 | Me | Me | CH₂CH(Me)N(Me)S(=O)₂CF₃ | Me |
| G-2401 | Me | Me | CH(Me)CH₂N(Me)S(=O)₂CF₃ | Me |
| G-2402 | Me | Me | CH₂CH₂CH(Me)N(Me)S(=O)₂CF₃ | Me |
| G-2403 | Me | Me | CH₂CH(Me)CH₂N(Me)S(=O)₂CF₃ | Me |
| G-2404 | Me | Me | CH(Me)CH₂CH₂N(Me)S(=O)₂CF₃ | Me |
| G-2405 | Me | Me | CH₂Ph | Me |
| G-2406 | Me | Me | CH₂Ph(2-CF₃) | Me |
| G-2407 | Me | Me | CH₂Ph(3-CF₃) | Me |
| G-2408 | Me | Me | CH₂Ph(4-CF₃) | Me |
| G-2409 | Me | Me | CH₂Ph(2-OCF₃) | Me |
| G-2410 | Me | Me | CH₂Ph(3-OCF₃) | Me |
| G-2411 | Me | Me | CH₂Ph(4-OCF₃) | Me |
| G-2412 | Me | Me | CH₂Ph(2-SCF₃) | Me |
| G-2413 | Me | Me | CH₂Ph(3-SCF₃) | Me |
| G-2414 | Me | Me | CH₂Ph(4-SCF₃) | Me |

**[Table 1175]**

| Compound Number | R³ | R⁴ | R⁵ | R¹¹ |
|---|---|---|---|---|
| G-2415 | Me | Me | CH₂Ph(3-CH₂SCF₃) | Me |
| G-2416 | Me | Me | CH₂Ph(4-F) | Me |
| G-2417 | Me | Me | CH₂Ph(4-Cl) | Me |
| G-2418 | Me | Me | CH₂Ph(4-Br) | Me |
| G-2419 | Me | Me | CH₂Ph(4-Me) | Me |
| G-2420 | Me | Me | CH₂Ph[4-(*t*-Bu)] | Me |
| G-2421 | Me | Me | CH₂Ph(4-CN) | Me |
| G-2422 | Me | Me | CH₂Ph(4-NO₂) | Me |
| G-2423 | Me | Me | CH₂Ph(4-OCHF₂) | Me |
| G-2424 | Me | Me | CH₂Ph(4-SCHF₂) | Me |
| G-2425 | Me | Me | CH₂Ph(4-CH₂SCF₃) | Me |
| G-2426 | Me | Me | CH₂Ph[4-CF(CF₃)₂] | Me |
| G-2427 | Me | Me | CH₂Ph(4-CH₂SCF₃) | Me |
| G-2428 | Me | Me | CH₂Ph[4-Ph(4-CF₃)] | Me |
| G-2429 | Me | Me | CH₂Ph(2,4-Cl₂) | Me |
| G-2430 | Me | Me | CH₂Ph[2,5-(CF₃)₂] | Me |
| G-2431 | Me | Me | CH₂Ph(3,4-Cl₂) | Me |
| G-2432 | Me | Me | CH₂Ph(3-CF₃-4-F) | Me |
| G-2433 | Me | Me | CH₂Ph(3-CF₃-4-Cl) | Me |
| G-2434 | Me | Me | CH₂Ph(3-F-4-CF₃) | Me |
| G-2435 | Me | Me | CH₂Ph(2,4,6-F₃) | Me |
| G-2436 | Me | Me | CH₂Ph(3,4,5-F₃) | Me |
| G-2437 | Me | Me | CH₂Ph(2,3,4-F₃) | Me |
| G-2438 | Me | Me | CH₂Ph(3,4,5-Cl₃) | Me |
| G-2439 | Me | Me | CH₂CH₂Ph | Me |
| G-2440 | Me | Me | CH₂CH₂Ph(4-F) | Me |
| G-2441 | Me | Me | CH₂CH₂Ph(4-Cl) | Me |
| G-2442 | Me | Me | CH₂CH₂Ph(4-Br) | Me |
| G-2443 | Me | Me | CH₂CH₂Ph[4-(*t*-Bu)] | Me |
| G-2444 | Me | Me | CH₂CH₂Ph(2-CF₃) | Me |
| G-2445 | Me | Me | CH₂CH₂Ph(3-CF₃) | Me |
| G-2446 | Me | Me | CH₂CH₂Ph(4-CF₃) | Me |
| G-2447 | Me | Me | CH₂CH₂Ph[4-CF(CF₃)₂] | Me |
| G-2448 | Me | Me | CH₂CH₂Ph[4-(*c*-Pr)] | Me |
| G-2449 | Me | Me | CH₂CH₂Ph{4-[*c*-Pr(2,2-F₂)]} | Me |
| G-2450 | Me | Me | CH₂CH₂Ph(4-OMe) | Me |
| G-2451 | Me | Me | CH₂CH₂Ph(3-OCF₃) | Me |
| G-2452 | Me | Me | CH₂CH₂Ph(4-OCHF₂) | Me |
| G-2453 | Me | Me | CH₂CH₂Ph(4-OCF₃) | Me |
| G-2454 | Me | Me | CH₂CH₂Ph(4-SMe) | Me |
| G-2455 | Me | Me | CH₂CH₂Ph(4-SCHF₂) | Me |
| G-2456 | Me | Me | CH₂CH₂Ph(4-SCF₃) | Me |
| G-2457 | Me | Me | CH₂CH₂Ph[4-S(=O)Me] | Me |
| G-2458 | Me | Me | CH₂CH₂Ph[4-S(=O)CF₃] | Me |

**[Table 1176]**

| Compound Number | R³ | R⁴ | R⁵ | R¹¹ |
|---|---|---|---|---|
| G-2459 | Me | Me | CH₂CH₂Ph[4-S(=O)₂Me] | Me |
| G-2460 | Me | Me | CH₂CH₂Ph[4-S(=O)₂CF₃] | Me |
| G-2461 | Me | Me | CH₂CH₂Ph(4-CH₂SMe) | Me |
| G-2462 | Me | Me | CH₂CH₂Ph(4-CH₂SCF₃) | Me |
| G-2463 | Me | Me | CH₂CH₂Ph[4-OS(=O)₂Me] | Me |
| G-2464 | Me | Me | CH₂CH₂Ph[4-OS(=O)₂CF₃] | Me |
| G-2465 | Me | Me | CH₂CH₂Ph[4-Ph(4-CF₃)] | Me |
| G-2466 | Me | Me | CH₂CH₂Ph(4-CH₂Ph) | Me |
| G-2467 | Me | Me | CH₂CH₂Ph(4-OCH₂Ph) | Me |
| G-2468 | Me | Me | CH₂CH₂Ph(4-CN) | Me |
| G-2469 | Me | Me | CH₂CH₂Ph(4-NO₂) | Me |
| G-2470 | Me | Me | CH₂CH₂Ph(2,4-Cl₂) | Me |
| G-2471 | Me | Me | CH₂CH₂Ph(3,4-Cl₂) | Me |
| G-2472 | Me | Me | CH₂CH₂Ph(3-CF₃-4-F) | Me |
| G-2473 | Me | Me | CH₂CH₂Ph(2-CF₃-4-F) | Me |
| G-2474 | Me | Me | CH₂CH₂Ph(3-F-4-CF₃) | Me |
| G-2475 | Me | Me | CH₂CH₂Ph(2-F-4-CF₃) | Me |
| G-2476 | Me | Me | CH₂CH₂Ph(3-Cl-4-OCHF₂) | Me |
| G-2477 | Me | Me | CH₂CH₂Ph(3,4,5-Cl₃) | Me |
| G-2478 | Me | Me | CH₂CH₂Ph(2,3,4-F₃) | Me |
| G-2479 | Me | Me | CH₂CH₂Ph(2,4,5-F₃) | Me |
| G-2480 | Me | Me | CH₂CH₂Ph(3,4,5-F₃) | Me |
| G-2481 | Me | Me | CH₂CH₂Ph(2,4,6-F₃) | Me |
| G-2482 | Me | Me | CH₂CH₂CH₂Ph | Me |
| G-2483 | Me | Me | CH₂CH₂CH₂Ph(3-CF₃) | Me |
| G-2484 | Me | Me | CH₂CH₂CH₂Ph(4-CF₃) | Me |
| G-2485 | Me | Me | CH₂CH₂CH₂Ph(4-F) | Me |
| G-2486 | Me | Me | CH₂CH₂CH₂Ph[4-(*t*-Bu)] | Me |
| G-2487 | Me | Me | CH₂CH₂CH₂Ph(4-CN) | Me |
| G-2488 | Me | Me | CH₂CH₂CH₂Ph(4-CF₃) | Me |
| G-2489 | Me | Me | CH₂CH₂CH₂Ph(4-OCHF₂) | Me |
| G-2490 | Me | Me | CH₂CH₂CH₂Ph(4-OCF₃) | Me |
| G-2491 | Me | Me | CH₂CH₂CH₂Ph(4-SCHF₂) | Me |
| G-2492 | Me | Me | CH₂CH₂CH₂Ph(4-SCF₃) | Me |
| G-2493 | Me | Me | CH₂CH₂CH₂Ph[4-CF(CF₃)₂] | Me |
| G-2494 | Me | Me | CH₂CH₂CH₂Ph(3,4,5-F₃) | Me |
| G-2495 | Me | Me | CH₂CH₂CH₂Ph(2,4,6-F₃) | Me |
| G-2496 | Me | Me | CH₂CH₂CH₂CH₂Ph | Me |
| G-2497 | Me | Me | CH₂CH₂CH₂CH₂Ph(4-F) | Me |
| G-2498 | Me | Me | CH₂CH₂CH₂CH₂Ph(4-CF₃) | Me |
| G-2499 | Me | Me | CH₂CH₂CH₂CH₂Ph(4-OCF₃) | Me |
| G-2500 | Me | Me | CH₂CH₂CH₃CH₂Ph(4-SCF₃) | Me |
| G-2501 | Me | Me | CH₂CH₂CH₂CH₂CH₂Ph | Me |
| G-2502 | Me | Me | CH₂CH₂CH₂CH₂CH₂CH₂Ph | Me |

**[Table 1177]**

| Compound Number | R³ | R⁴ | R⁵ | R¹¹ |
|---|---|---|---|---|
| G-2503 | Me | Me | CH₂CF₂Ph(4-F) | Me |
| G-2504 | Me | Me | CH₂CF₂Ph(4-CF₃) | Me |
| G-2505 | Me | Me | CH₂CF₂Ph(3,4,5-F₃) | Me |
| G-2506 | Cl | F | Me | Et |
| G-2507 | Cl | F | Et | Et |
| G-2508 | Cl | F | *n*-Pr | Et |
| G-2509 | Cl | F | *i*-Pr | Et |
| G-2510 | Cl | F | *n*-Bu | Et |
| G-2511 | Cl | F | *n*-Pen | Et |
| G-2512 | Cl | F | *n-Hex* | Et |
| G-2513 | Cl | F | *n*-Heptyl | Et |
| G-2514 | Cl | F | *n*-Octyl | Et |
| G-2515 | Cl | F | *n*-Nonyl | Et |
| G-2516 | Cl | F | *n*-Decyl | Et |
| G-2517 | Cl | F | *c*-Pr | Et |
| G-2518 | Cl | F | *c*-Pen | Et |
| G-2519 | Cl | F | *c*-Hex | Et |
| G-2520 | Cl | F | CH₂C(Me)=CH₂ | Et |
| G-2521 | Cl | F | CH₂CH₂CH=CH₂ | Et |
| G-2522 | Cl | F | CH₂CH₂CH=C(CH₃)₂ | Et |
| G-2523 | Cl | F | CH₂CH₂CH₂CH=CH₂ | Et |
| G-2524 | Cl | F | CH₂CH₂CH₂CH=C(CH₃)₂ | Et |
| G-2525 | Cl | F | CH₂CH₂CH₂CH₂CH=CH₂ | Et |
| G-2526 | Cl | F | CH₂CH₂CH₂CH₂CH=C(CH₃)₂ | Et |
| G-2527 | Cl | F | CH₂CH₂CH≡CH | Et |
| G-2528 | Cl | F | CH₂CH₂CH₂CH≡CH | Et |
| G-2529 | Cl | F | CH₂CH₂CH₂CH₂CH≡CH | Et |
| G-2530 | Cl | F | CH₂CH₂CH₂Cl | Et |
| G-2531 | Cl | F | CH₂CH₂CH₂Br | Et |
| G-2532 | Cl | F | CH₂CH₂CH₂CH₂Cl | Et |
| G-2533 | Cl | F | CH₂CH₂CH₂CH₂Br | Et |
| G-2534 | Cl | F | CH₂CH₂CH₂CH₂CH₂Cl | Et |
| G-2535 | Cl | F | CH₂CH₂CH₂CH₂CH₂Br | Et |
| G-2536 | Cl | F | CH₂CH₂CH₂CH₂CH₂CH₂Cl | Et |
| G-2537 | Cl | F | CH₂CH₂CH₂CH₂CH₂CH₂Br | Et |
| G-2538 | Cl | F | CH₂CH₂CH₂CH₂CH₂CH₂CH₂Cl | Et |
| G-2539 | Cl | F | CH₂CH₂CH₂CH₂CH₂CH₂CH₂Br | Et |
| G-2540 | Cl | F | CH₂CH₂CH₂CH₂CH₂CH₂CH₂CH₂Br | Et |
| G-2541 | Cl | F | CH₂CH(Me)CH₂Cl | Et |
| G-2542 | Cl | F | CH₂CH(Me)CH₂Br | Et |
| G-2543 | Cl | F | CH₂CH₂CH(Me)CH₂CH₂Cl | Et |
| G-2544 | Cl | F | CH₂CH₂CH(Me)CH₂CH₂Br | Et |
| G-2545 | Cl | F | CH₂CH₂CF=CF₂ | Et |
| G-2546 | Cl | F | CH₂CH=C(Cl)CF₃ | Et |

**[Table 1178]**

| Compound Number | R³ | R⁴ | R⁵ | R¹¹ |
|---|---|---|---|---|
| G-2547 | Cl | F | CH₂CH₂CH₂CF=CF₂ | Et |
| G-2548 | Cl | F | CH₂CH₂CH₂CH₂CF=CF₂ | Et |
| G-2549 | Cl | F | CH₂CH₂CH₂CH₂CH₂CF=CF₂ | Et |
| G-2550 | Cl | F | CH₂(*t*-Bu) | Et |
| G-2551 | Cl | F | CH₂CH₂(*t*-Bu) | Et |
| G-2552 | Cl | F | CH₂CH₂CH₂(*t*-Bu) | Et |
| G-2553 | Cl | F | CH₂CH₂CH₂CH₂(*t*-Bu) | Et |
| G-2554 | Cl | F | CH₂CH₂CH₂CH₂CH₂(*t*-Bu) | Et |
| G-2555 | Cl | F | CH₂CH₂CH₂CH₂CH₂CH₂(*t*-Bu) | Et |
| G-2556 | Cl | F | CH₂CF₃ | Et |
| G-2557 | Cl | F | CH₂CH₂CF₃ | Et |
| G-2558 | Cl | F | CH₂CH₂CH₂CF₃ | Et |
| G-2559 | Cl | F | CH₂CH₂CH₂CH₂CF₃ | Et |
| G-2560 | Cl | F | CH₂CH₂CH₂CH₂CH₂CF₃ | Et |
| G-2561 | Cl | F | CH₂CH₂CH₂CH₂CH₂CH₂CF₃ | Et |
| G-2562 | Cl | F | CH₂CH₂CH₂CH₂CH₂CH₂CH₂CF₃ | Et |
| G-2563 | Cl | F | CH₂CF₂CF₃ | Et |
| G-2564 | Cl | F | CF₂CHFCF₃ | Et |
| G-2565 | Cl | F | CF₂CF₂CF₃ | Et |
| G-2566 | Cl | F | CH₂CF₂CF₂CF₃ | Et |
| G-2567 | Cl | F | CH₂CF₂CF₂CF₂CF₃ | Et |
| G-2568 | Cl | F | CH₂CF₂CF₂CF₂CHF₂ | Et |
| G-2569 | Cl | F | CH(CF₃)CF₃ | Et |
| G-2570 | Cl | F | CH₂CF(CF₃)CF₃ | Et |
| G-2571 | Cl | F | CH₂CF₂CF(CF₃)CF₃ | Et |
| G-2572 | Cl | F | CH₂CF(CF₃)CF₂CF₃ | Et |
| G-2573 | Cl | F | CH₂CF₂CF₂CF(CF₃)CF₃ | Et |
| G-2574 | Cl | F | CH₂CF₂CF(CF₃)CF₂CF₃ | Et |
| G-2575 | Cl | F | CH₂CF(CF₃)CF₂CF₂CF₃ | Et |
| G-2576 | Cl | F | CH₂(*c*-Pr) | Et |
| G-2577 | Cl | F | CH₂[*c*-Pr(1-Me)] | Et |
| G-2578 | Cl | F | CH₂[*c*-Pr(1-Ph)] | Et |
| G-2579 | Cl | F | CH₂[*c*-Pr(1-NH₂)] | Et |
| G-2580 | Cl | F | CH₂[*c*-Pr(1-NHC(=O)O(*t*-Bu)] | Et |
| G-2581 | Cl | F | CH₂{*c*-Pr[1-NHS(=O)₂CF₃]} | Et |
| G-2582 | Cl | F | CH₂{*c*-Pr[1-Ph(4-CF₃)]} | Et |
| G-2583 | Cl | F | CH₂{*c*-Pr[1-Ph(3,4,5-F₃)]} | Et |
| G-2584 | Cl | F | CH₂{*c*-Hex[4-(*t*-Bu)]} | Et |
| G-2585 | Cl | F | CH₂[*c*-Hex(4-CF₃)] | Et |
| G-2586 | Cl | F | CH₂CH₂(*c*-Pr) | Et |
| G-2587 | Cl | F | CH₂CH₂(*c*-Hex) | Et |
| G-2588 | Cl | F | CH₂CH₂[*c*-Hex(4-CF₃)] | Et |
| G-2589 | Cl | F | CH₂CH₂[*c*-Hex(4-SCF₃)] | Et |
| G-2590 | Cl | F | CH₂CH₂CH₂(*c*-Pr) | Et |

**[Table 1179]**

| Compound Number | R³ | R⁴ | R⁵ | R¹¹ |
|---|---|---|---|---|
| G-2591 | Cl | F | CH₂CH₂CH₂(*c*-Hex) | Et |
| G-2592 | Cl | F | CH₂CH₂CH₂{*c*-Hex[4-(*t*-Bu)]} | Et |
| G-2593 | Cl | F | CH₂CH₂CH₂[*c*-Hex(4-CF₃)] | Et |
| G-2594 | Cl | F | CH₂CH₂CH₂[*c*-Hex(4-SCF₃)] | Et |
| G-2595 | Cl | F | CH₂CH₂CH₂CH₂(*c*-Pr) | Et |
| G-2596 | Cl | F | CH₂CH₂CH₂CH₂(*c*-Hex) | Et |
| G-2597 | Cl | F | CH₂CH₂CH₂CH₂[*c*-Hex(4-CF₃)] | Et |
| G-2598 | Cl | F | CH₂[*c*-Pr(2,2-F₂)] | Et |
| G-2599 | Cl | F | CH₂[*c*-Hex(4,4-F₂)] | Et |
| G-2600 | Cl | F | CH₂CH₂[*c*-Pr(2,2-F₂)] | Et |
| G-2601 | Cl | F | CH₂CH₂[*c*-Hex(4,4-F₂)] | Et |
| G-2602 | Cl | F | CH₂CH₂CH₂[*c*-Pr(2,2-F₂) | Et |
| G-2603 | Cl | F | CH₂CH₂CH₂CH₂[*c*-Pr(2,2-F₂) | Et |
| G-2604 | Cl | F | CH₂CH₂OH | Et |
| G-2605 | Cl | F | CH₂CH₂CH₂OH | Et |
| G-2606 | Cl | F | CH₂CH₂CH₂CH₂OH | Et |
| G-2607 | Cl | F | CH₂CH₂CH₂CH₂CH₂OH | Et |
| G-2608 | Cl | F | CH₂CH₂CH₂CH₂CH₂CH₂OH | Et |
| G-2609 | Cl | F | CH₂C(=O)Me | Et |
| G-2610 | Cl | F | CH₂CH(OH)Me | Et |
| G-2611 | Cl | F | CH₂CH(OH)CF₃ | Et |
| G-2612 | Cl | F | CH₂C(OH)₂CF₃ | Et |
| G-2613 | Cl | F | CH₂C(CF₃)=NOH | Et |
| G-2614 | Cl | F | CH₂C(CF₃)=NOMe | Et |
| G-2615 | Cl | F | CH₂CH(CF₃)NH₂ | Et |
| G-2616 | Cl | F | CH₂CH(CF₃)NHSO₂CF₃ | Et |
| G-2617 | Cl | F | CH₂CH₂OMe | Et |
| G-2618 | Cl | F | CH₂CH₂CH₂OMe | Et |
| G-2619 | Cl | F | CH₂CH₂CH₂CH₂OMe | Et |
| G-2620 | Cl | F | CH₂CH₂CH₂CH₂CH₂OMe | Et |
| G-2621 | Cl | F | CH₂CH₂CH₂CH₂CH₂CH₂OMe | Et |
| G-2622 | Cl | F | CH₂CH₂OCHF₂ | Et |
| G-2623 | Cl | F | CH₂CH₂CH₂OCHF₂ | Et |
| G-2624 | Cl | F | CH₂CH₂CH₂CH₂OCHF₂ | Et |
| G-2625 | Cl | F | CH₂CH₂CH₂CH₂CH₂OCHF₂ | Et |
| G-2626 | Cl | F | CH₂CH₂CH₂CH₂CH₂CH₂OCHF₂ | Et |
| G-2627 | Cl | F | CH₂CH₂OCH₂CF₃ | Et |
| G-2628 | Cl | F | CH₂CH₂CH₂OCH₂CF₃ | Et |
| G-2629 | Cl | F | CH₂CH₂CH₂CH₂OCH₂CF₃ | Et |
| G-2630 | Cl | F | CH₂CH₂CH₂CH₂CH₂OCH₂CF₃ | Et |
| G-2631 | Cl | F | CH₂CH₂CH₂CH₂CH₂CH₂OCH₂CF₃ | Et |
| G-2632 | Cl | F | CH₂CH₂CH₂CH₂CH₂CH₂CH₂OCH₂CF₃ | Et |
| G-2633 | Cl | F | CH₂CH₂OC(CF₃)₃ | Et |
| G-2634 | Cl | F | CH₂CH₂CH₂OC(CF₃)₃ | Et |

**[Table 1180]**

| Compound Number | R³ | R⁴ | R⁵ | R¹¹ |
|---|---|---|---|---|
| G-2635 | Cl | F | CH₂CH₂CH₂CH₂OC(CF₃)₃ | Et |
| G-2636 | Cl | F | CH₂CH₂CH₂CH₂CH₂OC(CF₃)₃ | Et |
| G-2637 | Cl | F | CF₂CHFOCF₂CF₂CF₃ | Et |
| G-2638 | Cl | F | CH₂CH₂O(*c*-Pr) | Et |
| G-2639 | Cl | F | CH₂CH₂O(*c*-Pen) | Et |
| G-2640 | Cl | F | CH₂CH₂O(*c*-Hex) | Et |
| G-2641 | Cl | F | CH₂CH₂CH₂O(*c*-Pr) | Et |
| G-2642 | Cl | F | CH₂CH₂CH₂O(*c*-Pen) | Et |
| G-2643 | Cl | F | CH₂CH₂CH₂O(*c*-Hex) | Et |
| G-2644 | Cl | F | CH₂CH₂CH₂CH₂O(*c*-Pr) | Et |
| G-2645 | Cl | F | CH₂CH₂CH₂CH₂O(*c*-Pen) | Et |
| G-2646 | Cl | F | CH₂CH₂CH₂CH₂O(*c*-Hex) | Et |
| G-2647 | Cl | F | CH₂CH₂O[*c*-Pr(2,2-F₂)] | Et |
| G-2648 | Cl | F | CH₂CH₂CH₂O[*c*-Pr(2,2-F₂)] | Et |
| G-2649 | Cl | F | CH₂CH₂CH₂CH₂O[*c*-Pr(2,2-F₂)] | Et |
| G-2650 | Cl | F | CH₂CH₂OCH₂CH₂OCH₃ | Et |
| G-2651 | Cl | F | CH₂CH₂OCH₂CH₂OCH₂CF₃ | Et |
| G-2652 | Cl | F | CH₂CF₂OCF₂CF₂OCF₃ | Et |
| G-2653 | Cl | F | CF₂CHFOCF₂CF₂OCF₃ | Et |
| G-2654 | Cl | F | CF₂CHFOCF₂CF(CF₃)OCF₂CF₂CF₃ | Et |
| G-2655 | Cl | F | CH₂CH₂SH | Et |
| G-2656 | Cl | F | CH₂CH₂CH₂SH | Et |
| G-2657 | Cl | F | CH₂CH₂CH₂CH₂SH | Et |
| G-2658 | Cl | F | CH₂CH₂CH₂CH₂CH₂SH | Et |
| G-2659 | Cl | F | CH₂CH₂CH₂CH₂CH₂CH₂SH | Et |
| G-2660 | Cl | F | CH₂CH₂S(*t*-Bu) | Et |
| G-2661 | Cl | F | CH₂CH₂CH₂S(*t*-Bu) | Et |
| G-2662 | Cl | F | CH₂CH₂CH₂CH₂S(*t*-Bu) | Et |
| G-2663 | Cl | F | CH₂CH₂CH₂CH₂CH₂SCH₃ | Et |
| G-2664 | Cl | F | CH₂CH₂CH₂CH₂CH₂SCH(CH₃)₂ | Et |
| G-2665 | Cl | F | CH₂CH₂CH₂CH₂CH₂S(*t*-Bu) | Et |
| G-2666 | Cl | F | CH₂CH₂CH₂CH₂CH₂CH₂SCH(CH₃)₂ | Et |
| G-2667 | Cl | F | CH₂CH₂CH₂CH₂CH₂CH₂S(*t*-Bu) | Et |
| G-2668 | Cl | F | CH₂CH₂CH₂CH₂CH₂CH₂CH₂S(*t*-Bu) | Et |
| G-2669 | Cl | F | CH₂CH₂SCHF₂ | Et |
| G-2670 | Cl | F | CH₂CH₂CH₂SCHF₂ | Et |
| G-2671 | Cl | F | CH₂CH₂CH₂CH₂SCHF₂ | Et |
| G-2672 | Cl | F | CH₂CH₂CH₂CH₂CH₂SCHF₂ | Et |
| G-2673 | Cl | F | CH₂CH₂CH₂CH₂CH₂CH₂SCHF₂ | Et |
| G-2674 | Cl | F | CH₂CH₂CH₂CH₂CH₂CH₂CH₂SCHF₂ | Et |
| G-2675 | Cl | F | CH₂CH₂SCF₃ | Et |
| G-2676 | Cl | F | CH₂CH₂CH₂SCF₃ | Et |
| G-2677 | Cl | F | CH₂CH₂CH₂CH₂SCF₃ | Et |
| G-2678 | Cl | F | CH₂CH₂CH₂CH₂CH₂SCF₃ | Et |

**[Table 1181]**

| Compound Number | R³ | R⁴ | R⁵ | R¹¹ |
|---|---|---|---|---|
| G-2679 | Cl | F | CH₂CH₂CH₂CH₂CH₂CH₂SCF₃ | Et |
| G-2680 | Cl | F | CH₂CH₂CH₂CH₂CH₂CH₂CH₂SCF₃ | Et |
| G-2681 | Cl | F | CH₂CH₂CH₂CH₂CH₂CH₂CH₂CH₂SCF₃ | Et |
| G-2682 | Cl | F | CH₂CH(Me)SCF₃ | Et |
| G-2683 | Cl | F | CH(Me)CH₂SCF₃ | Et |
| G-2684 | Cl | F | CH₂CH₂CH(Me)SCF₃ | Et |
| G-2685 | Cl | F | CH₂CH(Me)CH₂SCF₃ | Et |
| G-2686 | Cl | F | CH(Me)CH₂CH₂SCF₃ | Et |
| G-2687 | Cl | F | CH₂CH₂CH₂CH(Me)SCF₃ | Et |
| G-2688 | Cl | F | CH₂CH₂CH(Me)CH₂SCF₃ | Et |
| G-2689 | Cl | F | CH₂CH(Me)CH₂CH₂SCF₃ | Et |
| G-2690 | Cl | F | CH(Me)CH₂CH₂CH₂SCF₃ | Et |
| G-2691 | Cl | F | CH₂CH₂SCH₂CF₃ | Et |
| G-2692 | Cl | F | CH₂CH₂CH₂SCH₂CF₃ | Et |
| G-2693 | Cl | F | CH₂CH₂CH₂CH₂SCH₂CF₃ | Et |
| G-2694 | Cl | F | CH₂CH₂CH₂CH₂CH₂SCH₂CF₃ | Et |
| G-2695 | Cl | F | CH₂CH₂CH₂CH₂CH₂CH₂SCH₂CF₃ | Et |
| G-2696 | Cl | F | CH₂CH₂CH₂CH₂CH₂CH₂CH₂SCH₂CF₃ | Et |
| G-2697 | Cl | F | CH_{2C}H₂SCH(CF₃)₂ | Et |
| G-2698 | Cl | F | CH₂CH₂CH₂SCH(CF₃)₂ | Et |
| G-2699 | Cl | F | CH₂CH₂CH₂CH₂SCH(CF₃)₂ | Et |
| G-2700 | Cl | F | CH₂CH₂CH₂CH₂CH₂SCH(CF₃)₂ | Et |
| G-2701 | Cl | F | CH₂CH₂CH₂CH₂CH₂CH₂SCH(CF₃)₂ | Et |
| G-2702 | Cl | F | CH₂CH₂CH₂CH₂CH₂SCF₂CF₃ | Et |
| G-2703 | Cl | F | CH₂CH₂CH₂CH₂CH₂CH₂SCF₂CF₃ | Et |
| G-2704 | Cl | F | CH₂CH₂CH₂CH₂CH₂CH₂SCF(CF₃)₂ | Et |
| G-2705 | Cl | F | CH₂CH₂CH₂CH₂CH₂CH₂CH₂SCF(CF₃)₂ | Et |
| G-2706 | Cl | F | CH₂CH₂CH₂CH₂CH₂SCCl₃ | Et |
| G-2707 | Cl | F | CH₂CH₂CH₂CH₂CH₂CH₂SCCl₃ | Et |
| G-2708 | Cl | F | CH₂CH₂SCH₂CH=CH₂ | Et |
| G-2709 | Cl | F | CH₂CH₂CH₂SCH₂CH=CH₂ | Et |
| G-2710 | Cl | F | CH₂CH₂CH₂CH₂SCH₂CH=CH₂ | Et |
| G-2711 | Cl | F | CH₂CH₂SCF=CFCF₃ | Et |
| G-2712 | Cl | F | CH₂CH₂CH₂SCF=CFCF₃ | Et |
| G-2713 | Cl | F | CH₂CH₂CH₂CH₂SCF=CFCF₃ | Et |
| G-2714 | Cl | F | CH₂CH₂CH₂S(*c*-Pr) | Et |
| G-2715 | Cl | F | CH₂CH₂CH₂S(*c*-Hex) | Et |
| G-2716 | Cl | F | CH₂CH₂CH₂CH₂S(*c*-Pr) | Et |
| G-2717 | Cl | F | CH₂CH₂CH₂CH₂S(*c*-Hex) | Et |
| G-2718 | Cl | F | CH₂CH₂CH₂CH₂CH₂S(*c*-Pr) | Et |
| G-2719 | Cl | F | CH₂CH₂CH₂CH₂CH₂S(*c*-Hex) | Et |
| G-2720 | Cl | F | CH₂CH₂CH₂S[*c*-Hex(4,4-F₂)] | Et |
| G-2721 | Cl | F | CH₂CH₂CH₂CH₂S[*c*-Hex(4,4-F₂) | Et |
| G-2722 | Cl | F | CH₂CH₂CH₂CH₂CH₂S[*c*-Hex(4,4-F₂)] | Et |

**[Table 1182]**

| Compound Number | R³ | R⁴ | R⁵ | R¹¹ |
|---|---|---|---|---|
| G-2723 | Cl | F | CH₂CH₂CH₂SCH₂(*c*-Pr) | Et |
| G-2724 | Cl | F | CH₂CH₂CH₂SCH₂(*c*-Hex) | Et |
| G-2725 | Cl | F | CH₂CH₂CH₂CH₂SCH₂(*c*-Pr) | Et |
| G-2726 | Cl | F | CH₂CH₂CH₂CH₂SCH₂(*c*-Hex) | Et |
| G-2727 | Cl | F | CH₂CH₂CH₂CH₂CH₂SCH₂(*c*-Pr) | Et |
| G-2728 | Cl | F | CH₂CH₂CH₂CH₂CH₂SCH₂(*c*-Hex) | Et |
| G-2729 | Cl | F | CH₂CH₂CH₂SCH₂[*c*-Hex(4,4-F₂)] | Et |
| G-2730 | Cl | F | CH₂CH₂CH₂CH₂SCH₂[*c*-Hex(4,4-F₂)] | Et |
| G-2731 | Cl | F | CH₂CH₂CH₂CH₂CH₂SCH₂[*c*-Hex(4,4-F₂)] | Et |
| G-2732 | Cl | F | CH₂CH₂CH₂SCH₂SiMe₃ | Et |
| G-2733 | Cl | F | CH₂CH₂CH₂CH₂SCH₂SiMe₃ | Et |
| G-2734 | Cl | F | CH₂CH₂CH₂CH₂CH₂SCH₂SiMe₃ | Et |
| G-2735 | Cl | F | CH₂CH₂OCH₂CH₂SCH₃ | Et |
| G-2736 | Cl | F | CH₂CH₂OCH₂CH₂SCHF₂ | Et |
| G-2737 | Cl | F | CH₂CH₂OCH₂CH₂SCF₃ | Et |
| G-2738 | Cl | F | CH₂CH₂OCH₂CH₂SCH₂CF₃ | Et |
| G-2739 | Cl | F | CH₂CH₂CH₂S(=O)(*t*-Bu) | Et |
| G-2740 | Cl | F | CH₂CH₂CH₂CH₂S(=O)(*t*-Bu) | Et |
| G-2741 | Cl | F | CH₂CH₂CH₂CH₂CH₂CH₂S(=O)(*t*-Bu) | Et |
| G-2742 | Cl | F | CH₂CH₂S(=O)CHF₂ | Et |
| G-2743 | Cl | F | CH₂CH_{2C}H₂S(=O)CHF₂ | Et |
| G-2744 | Cl | F | CH₂CH₂CH₂CH₂S(=O)CHF₂ | Et |
| G-2745 | Cl | F | CH₂CH₂CH_{2C}H₂CH₂S(=O)CHF₂ | Et |
| G-2746 | Cl | F | CH₂CH₂CH₂CH₂CH₂CH₂S(=O)CHF₂ | Et |
| G-2747 | Cl | F | CH₂CH₂CH₂CH_{2C}H₂CH₂CH₂S(=O)CHF₂ | Et |
| G-2748 | Cl | F | CH₂CH₂S(=O)CF₃ | Et |
| G-2749 | Cl | F | CH₂CH₂CH₂S(=O)CF₃ | Et |
| G-2750 | Cl | F | CH₂CH₂CH₂CH₂S(=O)CF₃ | Et |
| G-2751 | Cl | F | CH₂CH₂CH₂CH₂CH₂S(=O)CF₃ | Et |
| G-2752 | Cl | F | CH₂CH₂CH₂CH₂CH₂CH₂S(=O)CF₃ | Et |
| G-2753 | Cl | F | CH₂CH₂CH₂CH₂CH₂CH₂CH₂S(=O)CF₃ | Et |
| G-2754 | Cl | F | CH₂CH₂S(=O)CH₂CF₃ | Et |
| G-2755 | Cl | F | CH₂CH₂CH₂S(=O)CH₂CF₃ | Et |
| G-2756 | Cl | F | CH₂CH₂CH₂CH₂S(=O)CH₂CF₃ | Et |
| G-2757 | Cl | F | CH₂CH₂CH₂CH₂CH₂S(=O)CH₂CF₃ | Et |
| G-2758 | Cl | F | CH₂CH₂CH₂CH₂CH₂CH₂S(=O)CH₂CF₃ | Et |
| G-2759 | Cl | F | CH₂CH₂CH₂CH₂CH₂CH₂CH₂S(=O)CH₂CF₃ | Et |
| G-2760 | Cl | F | CH₂CH₂CH₂S(=O)(*c*-Pr) | Et |
| G-2761 | Cl | F | CH₂CH₂CH₂CH₂S(=O)(*c*-Pr) | Et |
| G-2762 | Cl | F | CH₂CH₂CH₂CH₂S(=O)(*c*-Pen) | Et |
| G-2763 | Cl | F | CH₂CH₂CH₂CH₂S(=O)(*c*-Hex) | Et |
| G-2764 | Cl | F | CH₂CH₂CH₂CH₂CH₂S(=O)(*c*-Pr) | Et |
| G-2765 | Cl | F | CH₂CH₂CH₂CH₂CH₂S(=O)(*c*-Hex) | Et |
| G-2766 | Cl | F | CH₂CH₂CH₂S(=O)[*c*-Hex(4,4-F₂)] | Et |

**[Table 1183]**

| Compound Number | R³ | R⁴ | R⁵ | R¹¹ |
|---|---|---|---|---|
| G-2767 | Cl | F | CH₂CH₂CH₂CH₂S(=O)[*c*-Hex(4,4-F₂) | Et |
| G-2768 | Cl | F | CH₂CH₂CH₂CH₂CH₂S(=O)[*c*-Hex(4,4-F₂)] | Et |
| G-2769 | Cl | F | CH₂CH₂CH₂CH₂S(=O)₂Me | Et |
| G-2770 | Cl | F | CH₂CH₂CH₂CH₂CH₂S(=O)₂Me | Et |
| G-2771 | Cl | F | CH₂CH₂CH₂CH₂CH₂S(=O)₂(*t*-Bu) | Et |
| G-2772 | Cl | F | CH₂CH₂S(=O)₂CHF₂ | Et |
| G-2773 | Cl | F | CH₂CH₂CH₂S(=O)₂CHF₂ | Et |
| G-2774 | Cl | F | CH₂CH₂CH₂CH₂S(=O)₂CHF₂ | Et |
| G-2775 | Cl | F | CH₂CH₂CH₂CH₂CH₂S(=O)₂CHF₂ | Et |
| G-2776 | Cl | F | CH₂CH₂CH₂CH₂CH₂CH₂S(=O)₂CHF₂ | Et |
| G-2777 | Cl | F | CH₂CH₂S(=O)₂CF₃ | Et |
| G-2778 | Cl | F | CH₂CH₂CH₂S(=O)₂CF₃ | Et |
| G-2779 | Cl | F | CH₂CH₂CH₂CH₂S(=O)₂CF₃ | Et |
| G-2780 | Cl | F | CH₂CH₂CH₂CH₂CH₂S(=O)₂CF₃ | Et |
| G-2781 | Cl | F | CH₂CH₂CH₂CH₂CH₂CH₂S(=O)₂CF₃ | Et |
| G-2782 | Cl | F | CH₂CH₂CH₂CH₂CH₂CH₂CH₂S(=O)₂CF₃ | Et |
| G-2783 | Cl | F | CH₂CH₂CH₂CH₂CH₂CH₂CH₂CH₂S(=O)₂CF₃ | Et |
| G-2784 | Cl | F | CH₂CH₂S(=O)₂CH₂CF₃ | Et |
| G-2785 | Cl | F | CH₂CH₂CH₂S(=O)₂CH₂CF₃ | Et |
| G-2786 | Cl | F | CH₂CH₂CH₂CH₂S(=O)₂CH₂CF₃ | Et |
| G-2787 | Cl | F | CH₂CH₂CH₂CH₂CH₂S(=O)₂CH₂CF₃ | Et |
| G-2788 | Cl | F | CH₂CH₂CH₂CH₂CH₂CH₂S(=O)₂CH₂CF₃ | Et |
| G-2789 | Cl | F | CH₂CH₂CH₂S(=O)₂(*c*-Pr) | Et |
| G-2790 | Cl | F | CH₂CH₂CH₂CH₂S(=O)₂(*c*-Pr) | Et |
| G-2791 | Cl | F | CH₂CH₂CH₂CH₂CH₂S(=O)₂(*c*-Pr) | Et |
| G-2792 | Cl | F | CH₂CH₂CH₂S(=O)₂[*c*-Hex(4,4-F₂)] | Et |
| G-2793 | Cl | F | CH₂CH₂CH₂CH₂S(=O)₂[*c*-Hex(4,4-F₂)] | Et |
| G-2794 | Cl | F | iCH₂CH₂CH₂CH₂CH₂S(=O)₂[*c*-Hex(4,4-F₂)] | Et |
| G-2795 | Cl | F | CH₂CH₂CH₂C(=O)H | Et |
| G-2796 | Cl | F | CH₂CH₂CH₂CH₂C(=O)H | Et |
| G-2797 | Cl | F | CH₂CH₂CH₂CH₂CH₂C(=O)H | Et |
| G-2798 | Cl | F | CH₂CH₂CH₂C(=O)(*t*-Bu) | Et |
| G-2799 | Cl | F | CH₂CH₂CH₂CH₂C(=O)(*t*-Bu) | Et |
| G-2800 | Cl | F | CH₂CH₂CH₂CH₂CH₂C(=O)(*t*-Bu) | Et |
| G-2801 | Cl | F | CH₂CH₂CH₂C(=O)CF₃ | Et |
| G-2802 | Cl | F | CH₂CH₂CH₂CH₂C(=O)CF₃ | Et |
| G-2803 | Cl | F | CH₂CH₂CH₂CH₂CH₂C(=O)CF₃ | Et |
| G-2804 | Cl | F | CH₂C(=O)Ph | Et |
| G-2805 | Cl | F | CH₂C(=O)Ph(4-Cl) | Et |
| G-2806 | Cl | F | CH₂C(=O)Ph(4-CF₃) | Et |
| G-2807 | Cl | F | CH₂CH₂C(=O)Ph(4-CF₃) | Et |
| G-2808 | Cl | F | CH₂CH₂CH₂C(=O)Ph(4-CF₃) | Et |
| G-2809 | Cl | F | CH_{Z}CH₂CH₂CH₂C(=O)Ph(4-CF₃) | Et |
| G-2810 | Cl | F | CH₂CH₂CH₂CH₂CH₂C(=O)Ph(4-CF₃) | Et |

**[Table 1184]**

| Compound Number | R³ | R⁴ | R⁵ | R¹¹ |
|---|---|---|---|---|
| G-2811 | Cl | F | CH₂C(=O)OEt | Et |
| G-2812 | Cl | F | CH₂CH₂C(=O)O(*t*-Bu) | Et |
| G-2813 | Cl | F | CH₂CH₂CH₂C(=O)OEt | Et |
| G-2814 | Cl | F | CH₂CH₂CH₂C(=O)O(*t*-Bu) | Et |
| G-2815 | Cl | F | CH₂CH₂CH₂CH₂C(=O)O(*t*-Bu) | Et |
| G-2816 | Cl | F | CH₂CH₂CH₂CH₂CH₂C(=O)O(*t*-Bu) | Et |
| G-2817 | Cl | F | CH₂CH₂CH₂CH₂CH₂CH₂C(=O)O(*t*-Bu) | Et |
| G-2818 | Cl | F | CH₂CH₂CH₂CH₂CH₂CH₂CH₂C(=O)OEt | Et |
| G-2819 | Cl | F | CH₂CH₂C(=O)OCH₂CF₃ | Et |
| G-2820 | Cl | F | CH₂CH₂CH₂C(=O)OCH₂CF₃ | Et |
| G-2821 | Cl | F | CH₂CH₂CH₂CH₂C(=O)OCH₂CF₃ | Et |
| G-2822 | Cl | F | CH₂CH₂CH₂CH₂CH₂C(=O)OCH₂CF₃ | Et |
| G-2823 | Cl | F | CH₂CH₂C(=O)NH(*t*-Bu) | Et |
| G-2824 | Cl | F | CH₂CH₂CH₂C(=O)NH(*t*-Bu) | Et |
| G-2825 | Cl | F | CH₂CH₂CH₂C(=O)NH(*t*-Pen) | Et |
| G-2826 | Cl | F | CH₂CH₂CH₂CH₂C(=O)NH(*t*-Bu) | Et |
| G-2827 | Cl | F | CH₂CH₂C(=O)NHCH₂CF₃ | Et |
| G-2828 | Cl | F | CH₂CH₂CH₂C(=O)NHCH₂CF₃ | Et |
| G-2829 | Cl | F | CH₂CH₂CH₂CH₂C(=O)NHCH₂CF₃ | Et |
| G-2830 | Cl | F | CH₂CH₂SC(=O)N(Me)₂ | Et |
| G-2831 | Cl | F | CH₂CH₂CH₂SC(=O)N(Me)₂ | Et |
| G-2832 | Cl | F | CH₂CH₂CH₂SC(=O)NH(*t*-Bu) | Et |
| G-2833 | Cl | F | CH₂CH₂CH₂CH₂CH₂SC(=O)NH(*t*-Bu) | Et |
| G-2834 | Cl | F | CH₂CH₂SC(=O)NHCH₂CF₃ | Et |
| G-2835 | Cl | F | CH₂CH₂CH₂SC(=O)NHCH₂CF₃ | Et |
| G-2836 | Cl | F | CH₂CH₂CH₂CH₂SC(=O)NHCH₂CF₃ | Et |
| G-2837 | Cl | F | CH₂CH₂CH₂OC(=O)H | Et |
| G-2838 | Cl | F | CH₂CH₂CH₂CH₂OC(=O)H | Et |
| G-2839 | Cl | F | CH₂CH₂CH₂CH₂CH₂OC(=O)H | Et |
| G-2840 | Cl | F | CH₂CH₂CH₂OC(=O)Me | Et |
| G-2841 | Cl | F | CH₂CH₂CH₂CH₂OC(=O)Me | Et |
| G-2842 | Cl | F | CH₂CH₂CH₂CH₂CH₂OC(=O)Me | Et |
| G-2843 | Cl | F | CH₂CH₂OC(=O)CF₃ | Et |
| G-2844 | Cl | F | CH₂CH₂CH₂OC(=O)CF₃ | Et |
| G-2845 | Cl | F | CH₂CH₂CH₂CH₂OC(=O)CF₃ | Et |
| G-2846 | Cl | F | CH₂CH₂CH₂CH₂CH₂OC(=O)Ph(4-CF₃) | Et |
| G-2847 | Cl | F | CH₂CH₂OC(=O)Ph | Et |
| G-2848 | Cl | F | CH₂CH₂OC(=O)Ph(4-CF₃) | Et |
| G-2849 | Cl | F | CH₂CH₂CH₂OC(=O)Ph(4-CF₃) | Et |
| G-2850 | Cl | F | CH₂CH₂CH₂CH₂OC(=O)Ph(4-CF₃) | Et |
| G-2851 | Cl | F | CH₂CH₂CH₂CH₂CH₂OC(=O)Ph(4-CF₃) | Et |
| G-2852 | Cl | F | CH₂CH₂OS(=O)Me | Et |
| G-2853 | Cl | F | CH₂CH₂CH₂OS(=O)Me | Et |
| G-2854 | Cl | F | CH₂CH₂CH₂CH₂OS(=O)Me | Et |

**[Table 1185]**

| Compound Number | R³ | R⁴ | R⁵ | R¹¹ |
|---|---|---|---|---|
| G-2855 | Cl | F | CH₂CH₂CH₂CH₂CH₂OS(=O)Me | Et |
| G-2856 | Cl | F | CH₂CH₂CH₂CH₂CH₂CH₂OS(=O)Me | Et |
| G-2857 | Cl | F | CH₂CH₂OS(=O)CF₃ | Et |
| G-2858 | Cl | F | CH₂CH₂CH₂OS(=O)CF₃ | Et |
| G-2859 | Cl | F | CH₂CH₂CH₂CH₂OS(=O)CF₃ | Et |
| G-2860 | Cl | F | CH₂CH₂CH₂CH₂CH₂OS(=O)CF₃ | Et |
| G-2861 | Cl | F | CH₂CH₂CH₂CH₂CH₂CH₂OS(=O)CF₃ | Et |
| G-2862 | Cl | F | CH₂CH₂OS(=O)Ph | Et |
| G-2863 | Cl | F | CH₂CH₂OS(=O)Ph(4-Me) | Et |
| G-2864 | Cl | F | CH₂CH₂CH₂OS(=O)Ph | Et |
| G-2865 | Cl | F | CH₂CH₂CH₂OS(=O)Ph(4-Me) | Et |
| G-2866 | Cl | F | CH₂CH₂OS(=O)₂Me | Et |
| G-2867 | Cl | F | CH₂CH₂CH₂OS(=O)₂Me | Et |
| G-2868 | Cl | F | CH₂CH₂CH₂OS(=O)₂CF₃ | Et |
| G-2869 | Cl | F | CH₂CH₂CH₂CH₂OS(=O)₂CF₃ | Et |
| G-2870 | Cl | F | CH₂CH₂CH₂CH₂CH₂OS(=O)₂CF₃ | Et |
| G-2871 | Cl | F | CH₂CH₂CH₂CH₂CH₂OS(=O)₂CF₂CF₂CF₂CF₃ | Et |
| G-2872 | Cl | F | CH₂CH₂OS(=O)₂Ph | Et |
| G-2873 | Cl | F | CH₂CH₂OS(=O)₂Ph(4-Me) | Et |
| G-2874 | Cl | F | CH₂CH₂CH₂OS(=O)₂Ph | Et |
| G-2875 | Cl | F | CH₂CH₂CH₂OS(=O)₂Ph(4-Me) | Et |
| G-2876 | Cl | F | CH₂CH₂NH₂ | Et |
| G-2877 | Cl | F | CH₂CH₂NH₂, HCl | Et |
| G-2878 | Cl | F | CH₂CH₂CH₂NH₂ | Et |
| G-2879 | Cl | F | CH₂CH₂CH₂CH₂NH₂ | Et |
| G-2880 | Cl | F | CH₂CH₂CH₂CH₂CH₂NH₂ | Et |
| G-2881 | Cl | F | CH₂CH₂CH₂CH₂CH₂CH₂NH₂ | Et |
| G-2882 | Cl | F | CH₂CH₂CH₂CH₂CH₂CH₂CH₂NH₂ | Et |
| G-2883 | Cl | F | CH₂CH(Me)NH₂ | Et |
| G-2884 | Cl | F | CH₂C*H(Me)NH₂, HCl | Et |
| G-2885 | Cl | F | CH₂C*H(Me)NH₂: (R) | Et |
| G-2886 | Cl | F | CH₂C*H(Me)NH₂, HCl: (R) | Et |
| G-2887 | Cl | F | CH₂C*H(Me)NH₂: (S) | Et |
| G-2888 | Cl | F | CH₂C*H(Me)NH₂, HCl: (S) | Et |
| G-2889 | Cl | F | CH₂CH(Et)NH₂ | Et |
| G-2890 | Cl | F | CH₂CH(*i*-Pr)NH₂ | Et |
| G-2891 | Cl | F | CH(Me)CH₂NH₂ | Et |
| G-2892 | Cl | F | CH(Me)CH₂NH₂, HCl | Et |
| G-2893 | Cl | F | CH(Et)CH₂NH₂ | Et |
| G-2894 | Cl | F | CH(*i*-Pr)CH₂NH₂ | Et |
| G-2895 | Cl | F | CH₂CH₂CH(Me)NH₂ | Et |
| G-2896 | Cl | F | CH₂CH(Me)CH₂NH₂ | Et |
| G-2897 | Cl | F | CH(Me)CH₂CH₂NH₂ | Et |
| G-2898 | Cl | F | CH₂CH(Me)NH(*c*-Pr) | Et |

**[Table 1186]**

| Compound Number | R³ | R⁴ | R⁵ | R¹¹ |
|---|---|---|---|---|
| G-2899 | Cl | F | CH₂CH(Me)N(*c*-Pr)S(=O)₂CF₃ | Et |
| G-2900 | Cl | F | CH₂CH(Me)NHOMe | Et |
| G-2901 | Cl | F | CH₂CH(Me)N(OMe)S(=O)₂CF₃ | Et |
| G-2902 | Cl | F | CH₂CH(Me)N(OMe)S(=O)₂Ph(2-NO₂) | Et |
| G-2903 | Cl | F | CH₂CH(Me)N(OMe)S(=O)₂Ph(4-NO₂) | Et |
| G-2904 | Cl | F | CH₂CH₂N(Me)(*t*-Bu) | Et |
| G-2905 | Cl | F | CH₂CH₂CH₂N(Me)(*t*-Bu) | Et |
| G-2906 | Cl | F | CH₂CH₂CH₂CH₂N(Me)(*t*-Bu) | Et |
| G-2907 | Cl | F | CH₂CH₂NHCH₂CF₃ | Et |
| G-2908 | Cl | F | CH₂CH₂CH₂NHCH₂CF₃ | Et |
| G-2909 | Cl | F | CH₂CH₂CH₂CH₂NHCH₂CF₃ | Et |
| G-2910 | Cl | F | CH₂CH₂NHC(=O)C(Me)(CF₃)₂ | Et |
| G-2911 | Cl | F | CH₂CH₂NHC(=O)Me | Et |
| G-2912 | Cl | F | CH₂CH₂NHC(=O)(*t*-Bu) | Et |
| G-2913 | Cl | F | CH₂CH₂CH₂CH₂NHC(=O)CH(CH₃)₂ | Et |
| G-2914 | Cl | F | CH₂CH₂CH₂CH₂NHC(=O)(*t*-Bu) | Et |
| G-2915 | Cl | F | CH₂CH₂CH₂CH₂NHC(=O)CH₂(*t*-Bu) | Et |
| G-2916 | Cl | F | CH₂CH₂NHC(=O)CF₃ | Et |
| G-2917 | Cl | F | CH₂CH₂NHC(=O)CH₂CF₃ | Et |
| G-2918 | Cl | F | CH₂CH₂NHC(=O)CF₂CF₃ | Et |
| G-2919 | Cl | F | CH₂CH₂CH₂NHC(=O)CF₃ | Et |
| G-2920 | Cl | F | CH₂CH₂CH₂NHC(=O)CH₂CF₃ | Et |
| G-2921 | Cl | F | CH₂CH₂CH₂NHC(=O)CF₂CF₃ | Et |
| G-2922 | Cl | F | CH₂CH₂CH₂CH₂NHC(=O)CF₃ | Et |
| G-2923 | Cl | F | CH₂CH₂CH₂CH₂NHC(=O)CH₂CF₃ | Et |
| G-2924 | Cl | F | CH₂CH₂CH₂CH₂NHC(=O)CF₂CF₃ | Et |
| G-2925 | Cl | F | CH₂CH₂CH₂CH₂NHC(=O)CF(CF₃)₂ | Et |
| G-2926 | Cl | F | CH₂CH₂CH₂CH₂NHC(=O)CCl₃ | Et |
| G-2927 | Cl | F | CH₂CH₂NHC(=O)Ph | Et |
| G-2928 | Cl | F | CH₂CH₂NHC(=O)Ph(4-CF₃) | Et |
| G-2929 | Cl | F | CH₂CH₂CH₂NHC(=O)Ph(4-CF₃) | Et |
| G-2930 | Cl | F | CH₂CH₂CH₂CH₂NHC(=O)Ph(4-CF₃) | Et |
| G-2931 | Cl | F | CH₂CH₂NHC(=O)O(*t*-Bu) | Et |
| G-2932 | Cl | F | CH₂CH₂CH₂NHC(=O)O(*t*-Bu) | Et |
| G-2933 | Cl | F | CH₂CH₂CH₂CH₂NHC(=O)O(*t*-Bu) | Et |
| G-2934 | Cl | F | CH₂CH₂CH₂CH₂NHC(=O)OCH(CH₃)₂ | Et |
| G-2935 | Cl | F | CH₂CH(Me)NHC(=O)O(*t*-Bu) | Et |
| G-2936 | Cl | F | CH₂C*H(Me)NHC(=O)O(*t*-Bu) : (R) | Et |
| G-2937 | Cl | F | CH₂C*H(Me)NHC(=O)O(*t*-Bu) : (S) | Et |
| G-2938 | Cl | F | CH₂CH(Et)NHC(=O)O(*t*-Bu) | Et |
| G-2939 | Cl | F | CH₂CH(*i*-Pr)NHC(=O)O(t-Bu) | Et |
| G-2940 | Cl | F | CH(Me)CH₂NHC(=O)O(*t*-Bu) | Et |
| G-2941 | Cl | F | CH(Et)CH₂NHC(=O)O(*t*-Bu) | Et |
| G-2942 | Cl | F | CH(*i*-Pr)CH₂NHC(=O)O(*t*-Bu) | Et |

**[Table 1187]**

| Compound Number | R³ | R⁴ | R⁵ | R¹¹ |
|---|---|---|---|---|
| G-2943 | Cl | F | CH₂CH(Me)CH₂NHC(=O)O(*t*-Bu) | Et |
| G-2944 | Cl | F | CH₂CH₂NHC(=O)OC(Me)₂CF₃ | Et |
| G-2945 | Cl | F | CH₂CH₂CH₂NHC(=O)OCH₂CF₃ | Et |
| G-2946 | Cl | F | CH₂CH₂CH₂NHC(=O)OCH₂CH₂CF₃ | Et |
| G-2947 | Cl | F | CH₂CH₂CH₂CH₂NHC(=O)OCH₂CCl₃ | Et |
| G-2948 | Cl | F | CH₂CH₂CH₂CH₂NHC(=O)OCH₂CF₃ | Et |
| G-2949 | Cl | F | CH₂CH₂NHC(=O)NH(*t*-Bu) | Et |
| G-2950 | Cl | F | CH₂CH₂CH₂NHC(=O)NH(*t*-Bu) | Et |
| G-2951 | Cl | F | CH₂CH₂CH₂CH₂NHC(=O)NHEt | Et |
| G-2952 | Cl | F | CH₂CH₂CH₂CH₂NHC(=O)NH(*t*-Bu) | Et |
| G-2953 | Cl | F | CH₂CH₂CH₂NHC(=O)NHCH₂CCl₃ | Et |
| G-2954 | Cl | F | CH₂CH₂CH₂NHC(=O)NHCH₂CH₂F | Et |
| G-2955 | Cl | F | CH₂CH₂CH₂NHC(=O)NHCH₂CF₃ | Et |
| G-2956 | Cl | F | CH₂CH₂CH₂CH₂NHC(=O)NHCH₂CCl₃ | Et |
| G-2957 | Cl | F | CH₂CH₂CH₂CH₂NHC(=O)NHCH₂CH₂F | Et |
| G-2958 | Cl | F | CH₂CH₂CH₂CH₂NHC(=O)NHCH₂CF₃ | Et |
| G-2959 | Cl | F | CH₂CH₂NHS(=O)Me | Et |
| G-2960 | Cl | F | CH₂CH₂CH₂NHS(=O)Me | Et |
| G-2961 | Cl | F | CH₂CH₂CH₂CH₂NHS(=O)Me | Et |
| G-2962 | Cl | F | CH₂CH₂CH₂CH₂NHS(=O)CH(CH₃)₂ | Et |
| G-2963 | Cl | F | CH₂CH₂CH₂CH₂CH₂NHS(=O)Me | Et |
| G-2964 | Cl | F | CH₂CH₂NHS(=O)CHF₂ | Et |
| G-2965 | Cl | F | CH₂CH₂CH₂NHS(=O)CHF₂ | Et |
| G-2966 | Cl | F | CH₂CH₂CH₂CH₂NHS(=O)CHF₂ | Et |
| G-2967 | Cl | F | CH₂CH₂CH₂CH₂CH₂NHS(=O)CHF₂ | Et |
| G-2968 | Cl | F | CH₂CH(Me)NHS(=O)CHF₂ | Et |
| G-2969 | Cl | F | CH(Me)CH₂NHS(=O)CHF₂ | Et |
| G-2970 | Cl | F | CH₂CH₂CH(Me)NHS(=O)CHF₂ | Et |
| G-2971 | Cl | F | CH₂CH(Me)CH₂NHS(=O)CHF₂ | Et |
| G-2972 | Cl | F | CH(Me)CH₂CH₂NHS(=O)CHF₂ | Et |
| G-2973 | Cl | F | CH₂CH₂NHS(=O)CF₃ | Et |
| G-2974 | Cl | F | CH₂CH₂CH₂NHS(=O)CF₃ | Et |
| G-2975 | Cl | F | CH₂CH₂CH₂CH₂NHS(=O)CF₃ | Et |
| G-2976 | Cl | F | CH₂CH₂CH₂CH₂CH₂NHS(=O)CF₃ | Et |
| G-2977 | Cl | F | CH₂CH₂CH₂NHS(=O)Ph | Et |
| G-2978 | Cl | F | CH₂CH₂CH₂CH₂NHS(=O)Ph | Et |
| G-2979 | Cl | F | CH₂CH₂CH₂CH₂CH₂NHS(=O)Ph | Et |
| G-2980 | Cl | F | CH₂CH₂CH₂CH₂CH₂NHS(=O)Ph(4-CF₃) | Et |
| G-2981 | Cl | F | CH₂CH(Me)NHS(=O)Ph(4-CF₃) | Et |
| G-2982 | Cl | F | CH₂CH(Et)NHS(=O)Ph(4-CF₃) | Et |
| G-2983 | Cl | F | CH(Me)CH₂NHS(=O)Ph(4-CF₃) | Et |
| G-2984 | Cl | F | CH(Et)CH₂NHS(=O)Ph(4-CF₃) | Et |
| G-2985 | Cl | F | CH₂CH(Et)CH₂NHS(=O)Ph(4-CF₃) | Et |
| G-2986 | Cl | F | CH₂CH₂NHS(=O)₂Me | Et |

**[Table 1188]**

| Compound Number | R³ | R⁴ | R⁵ | R¹¹ |
|---|---|---|---|---|
| G-2987 | Cl | F | CH₂CH₂CH₂NHS(=O)₂Me | Et |
| G-2988 | Cl | F | CH₂CH₂CH₂CH₂NHS(=O)₂Me | Et |
| G-2989 | Cl | F | CH₂CH₂CH₂CH₂NHS(=O)₂CH(CH₃)₂ | Et |
| G-2990 | Cl | F | CH₂CH₂CH₂CH₂CH₂NHS(=O)₂Me | Et |
| G-2991 | Cl | F | CH₂CH₂NHS(=O)₂CHF₂ | Et |
| G-2992 | Cl | F | CH₂CH₂CH₂NHS(=O)₂CHF₂ | Et |
| G-2993 | Cl | F | CH₂CH₂CH₂CH₂NHS(=O)₂CHF₂ | Et |
| G-2994 | Cl | F | CH₂CH₂CH₂CH₂CH₂NHS(=O)₂CHF₂ | Et |
| G-2995 | Cl | F | CH₂CH(Me)NHS(=O)₂CHF₂ | Et |
| G-2996 | Cl | F | CH(Me)CH₂NHS(=O)₂CHF₂ | Et |
| G-2997 | Cl | F | CH₂CH₂CH(Me)NHS(=O)₂CHF₂ | Et |
| G-2998 | Cl | F | CH₂CH(Me)CH₂NHS(=O)₂CHF₂ | Et |
| G-2999 | Cl | F | CH(Me)CH₂CH₂NHS(=O)₂CHF₂ | Et |
| G-3000 | Cl | F | CH₂CH(Me)N(Me)S(=O)₂CHF₂ | Et |
| G-3001 | Cl | F | CH(Me)CH₂N(Me)S(=O)₂CHF₂ | Et |
| G-3002 | Cl | F | CH₂CH₂CH(Me)N(Me)S(=O)₂CHF₂ | Et |
| G-3003 | Cl | F | CH₂CH(Me)CH₂N(Me)S(=O)₂CHF₂ | Et |
| G-3004 | Cl | F | CH(Me)CH₂CH₂N(Me)S(=O)₂CHF₂ | Et |
| G-3005 | Cl | F | CH₂CH₂NHS(=O)₂CF₃ | Et |
| G-3006 | Cl | F | CH₂CH₂CH₂NHS(=O)₂CF₃ | Et |
| G-3007 | Cl | F | CH₂CH₂CH₂CH₂NHS(=O)₂CF₃ | Et |
| G-3008 | Cl | F | CH₂CH₂CH₂CH₂CH₂NHS(=O)₂CF₃ | Et |
| G-3009 | Cl | F | CH₂CH₂N(Me)S(=O)₂CF₃ | Et |
| G-3010 | Cl | F | CH₂CH₂N(Et)S(=O)₂CF₃ | Et |
| G-3011 | Cl | F | CH₂CH₂N(CH₂CN)S(=O)₂CF₃ | Et |
| G-3012 | Cl | F | CH₂CH₂N(CH₂OMe)S(=O)₂CF₃ | Et |
| G-3013 | Cl | F | CH₂CH₂CH₂N(Me)S(=O)₂CF₃ | Et |
| G-3014 | Cl | F | CH₂CH₂CH₂N(Et)S(=O)₂CF₃ | Et |
| G-3015 | Cl | F | CH₂CH₂CH₂N(CH₂CN)S(=O)₂CF₃ | Et |
| G-3016 | Cl | F | CH₂CH₂CH₂N(CH₂OMe)S(=O)₂CF₃ | Et |
| G-3017 | Cl | F | CH₂CH₂CH₂CH₂N(Me)S(=O)₂CF₃ | Et |
| G-3018 | Cl | F | CH₂CH₂CH₂CH₂N(Et)S(=O)₂CF₃ | Et |
| G-3019 | Cl | F | CH₂CH₂CH₂CH₂N(CH₂CN)S(=O)₂CF₃ | Et |
| G-3020 | Cl | F | CH₂CH₂CH₂CH₂N(CH₂OMe)S(=O)₂CF₃ | Et |
| G-3021 | Cl | F | CH₂CH(Me)NHS(=O)₂CF₃ | Et |
| G-3022 | Cl | F | CH₂CH(Me)NHS(=O)₂CF₃: (R) | Et |
| G-3023 | Cl | F | CH₂CH(Me)NHS(=O)₂CF₃ : (S) | Et |
| G-3024 | Cl | F | CH(Me)CH₂NHS(=O)₂CF₃ | Et |
| G-3025 | Cl | F | CH₂CH₂CH(Me)NHS(=O)₂CF₃ | Et |
| G-3026 | Cl | F | CH₂CH(Me)CH₂NHS(=O)₂CF₃ | Et |
| G-3027 | Cl | F | CH(Me)CH₂CH₂NHS(=O)₂CF₃ | Et |
| G-3028 | Cl | F | CH₂CH(Me)N(Me)S(=O)₂CF₃ | Et |
| G-3029 | Cl | F | CH(Me)CH₂N(Me)S(=O)₂CF₃ | Et |
| G-3030 | Cl | F | CH₂CH₂CH(Me)N(Me)S(=O)₂CF₃ | Et |

**[Table 1189]**

| Compound Number | R³ | R⁴ | R⁵ | R¹¹ |
|---|---|---|---|---|
| G-3031 | Cl | F | CH₂CH(Me)CH₂N(Me)S(=O)_{2C}F₃ | Et |
| G-3032 | Cl | F | CH(Me)CH₂CH₂N(Me)S(=O)₂CF₃ | Et |
| G-3033 | Cl | F | CH₂CH₂CH₂NHS(=O)₂Ph | Et |
| G-3034 | Cl | F | CH₂CH₂CH₂CH₂NHS(=O)₂Ph | Et |
| G-3035 | Cl | F | CH₂CH₂CH₂CH₂CH₂NHS(=O)₂Ph | Et |
| G-3036 | Cl | F | CH₂CH₂CH₂CH_{2C}H₂NHS(=O)₂Ph(4-CF₃) | Et |
| G-3037 | Cl | F | CH₂CH(Me)NHS(=O)₂Ph(4-CF₃) | Et |
| G-3038 | Cl | F | CH₂CH(Et)NHS(=O)₂Ph(4-CF₃) | Et |
| G-3039 | Cl | F | CH(Me)CH₂NHS(=O)₂Ph(4-CF₃) | Et |
| G-3040 | Cl | F | CH(Et)CH₂NHS(=O)₂Ph(4-CF₃) | Et |
| G-3041 | Cl | F | CH₂CH(Me)CH₂NHS(=O)₂Ph(4-CF₃) | Et |
| G-3042 | Cl | F | CH₂CH₂SiMe₃ | Et |
| G-3043 | Cl | F | CH₂CH₂CH₂SiMe₃ | Et |
| G-3044 | Cl | F | CH₂CH₂CH₂CH₂SiMe₃ | Et |
| G-3045 | Cl | F | CH₂Ph | Et |
| G-3046 | Cl | F | CH₂Ph(2-CF₃) | Et |
| G-3047 | Cl | F | CH₂Ph(3-CF₃) | Et |
| G-3048 | Cl | F | CH₂Ph(4-CF₃) | Et |
| G-3049 | Cl | F | CH₂Ph(2-OCF₃) | Et |
| G-3050 | Cl | F | CH₂Ph(3-OCF₃) | Et |
| G-3051 | Cl | F | CH₂Ph(4-OCF₃) | Et |
| G-3052 | Cl | F | CH₂Ph(2-SCF₃) | Et |
| G-3053 | Cl | F | CH₂Ph(3-SCF₃) | Et |
| G-3054 | Cl | F | CH₂Ph(4-SCF₃) | Et |
| G-3055 | Cl | F | CH₂Ph(3-CH₂SCF₃) | Et |
| G-3056 | Cl | F | CH₂Ph(4-F) | Et |
| G-3057 | Cl | F | CH₂Ph(4- Cl ) | Et |
| G-3058 | Cl | F | CH₂Ph(4-Br) | Et |
| G-3059 | Cl | F | CH₂Ph(4-Me) | Et |
| G-3060 | Cl | F | CH₂Ph[4-(*t*-Bᵤ)] | Et |
| G-3061 | Cl | F | CH₂Ph(4-CN) | Et |
| G-3062 | Cl | F | CH₂Ph(4-NO₂) | Et |
| G-3063 | Cl | F | CH₂Ph(4-OCHF₂) | Et |
| G-3064 | Cl | F | CH₂Ph(4-SCHF₂) | Et |
| G-3065 | Cl | F | CH₂Ph(4-CH₂SCF₃) | Et |
| G-3066 | Cl | F | CH₂Ph[4-CF(CF₃)₂] | Et |
| G-3067 | Cl | F | CH₂Ph(4-CH₂SCF₃) | Et |
| G-3068 | Cl | F | CH₂Ph[4-Ph(4-CF₃)] | Et |
| G-3069 | Cl | F | CH₂Ph(2,4-Cl₂) | Et |
| G-3070 | Cl | F | CH₂Ph[2,5-(CF₃)₂) | Et |
| G-3071 | Cl | F | CH₂Ph(3,4-Cl₂) | Et |
| G-3072 | Cl | F | CH₂Ph(3-CF₃-4-F) | Et |
| G-3073 | Cl | F | CH₂Ph(3-CF₃-4-Cl) | Et |
| G-3074 | Cl | F | CH₂Ph(3-F-4-CF₃) | Et |

**[Table 1190]**

| Compound Number | R³ | R⁴ | R⁵ | R¹¹ |
|---|---|---|---|---|
| G-3075 | Cl | F | CH₂Ph(2,4,6-F₃) | Et |
| G-3076 | Cl | F | CH₂Ph(3,4,5-F₃) | Et |
| G-3077 | Cl | F | CH₂Ph(2,3,4-F₃) | Et |
| G-3078 | Cl | F | CH₂Ph(3,4,5-Cl₃) | Et |
| G-3079 | Cl | F | CH₂CH₂Ph | Et |
| G-3080 | Cl | F | CH₂CH₂Ph(4-F) | Et |
| G-3081 | Cl | F | CH₂CH₂Ph(4-Cl) | Et |
| G-3082 | Cl | F | CH₂CH₂Ph(4-Br) | Et |
| G-3083 | Cl | F | CH₂CH₂Ph[4-(*t*-Bu)] | Et |
| G-3084 | Cl | F | CH₂CH₂Ph(2-CF₃) | Et |
| G-3085 | Cl | F | CH₂CH₂Ph(3-CF₃) | Et |
| G-3086 | Cl | F | CH₂CH₂Ph(4-CF₃) | Et |
| G-3087 | Cl | F | CH₂CH₂Ph[4-CF(CF₃)₂] | Et |
| G-3088 | Cl | F | CH₂CH₂Ph[4-(C-Pr)] | Et |
| G-3089 | Cl | F | CH₂CH₂Ph{4-[*c*-Pr(2,2-F₂)]} | Et |
| G-3090 | Cl | F | CH₂CH₂Ph(4-OMe) | Et |
| G-3091 | Cl | F | CH₂CH₂Ph(3-OCF₃) | Et |
| G-3092 | Cl | F | CH₂CH₂Ph(4-OCHF₂) | Et |
| G-3093 | Cl | F | CH₂CH₂Ph(4-OCF₃) | Et |
| G-3094 | Cl | F | CH₂CH₂Ph(4-SMe) | Et |
| G-3095 | Cl | F | CH₂CH₂Ph(4-SCHF₂) | Et |
| G-3096 | Cl | F | CH₂CH₂Ph(4-SCF₃) | Et |
| G-3097 | Cl | F | CH₂CH₂Ph[4-S(=O)Me] | Et |
| G-3098 | Cl | F | CH₂CH₂Ph[4-S(=O)CF_{3]} | Et |
| G-3099 | Cl | F | CH₂CH₂Ph[4-S(=O)₂Me] | Et |
| G-3100 | Cl | F | CH₂CH₂Ph[4-S(=O)₂CF₃] | Et |
| G-3101 | Cl | F | CH₂CH₂Ph(4-CH₂SMe) | Et |
| G-3102 | Cl | F | CH₂CH₂Ph(4-CH₂SCF₃) | Et |
| G-3103 | Cl | F | CH₂CH₂Ph[4-OS(=O)₂Me] | Et |
| G-3104 | Cl | F | CH₂CH₂Ph[4-OS(=O)₂CF_{3]} | Et |
| G-3105 | Cl | F | CH₂CH₂Ph[4-Ph(4-CF₃)] | Et |
| G-3106 | Cl | F | CH₂CH₂Ph(4-CH₂Ph) | Et |
| G-3107 | Cl | F | CH₂CH₂Ph(4-OCH₂Ph) | Et |
| G-3108 | Cl | F | CH₂CH₂Ph(4-CN) | Et |
| G-3109 | Cl | F | CH₂CH₂Ph(4-NO₂) | Et |
| G-3110 | Cl | F | CH₂CH₂Ph(2,4-Cl₂) | Et |
| G-3111 | Cl | F | CH₂CH₂Ph(3,4-Cl₂) | Et |
| G-3112 | Cl | F | CH₂CH₂Ph(3-CF₃-4-F) | Et |
| G-3113 | Cl | F | CH₂CH₂Ph(2-CF₃-4-F) | Et |
| G-3114 | Cl | F | CH₂CH₂Ph(3-F-4-CF₃) | Et |
| G-3115 | Cl | F | CH₂CH₂Ph(2-F-4-CF₃) | Et |
| G-3116 | Cl | F | CH₂CH₂Ph(3- Cl -4-OCHF₂) | Et |
| G-3117 | Cl | F | CH₂CH₂Ph(3,4,5-Cl₃) | Et |
| G-3118 | Cl | F | CH₂CH₂Ph(2,3,4-F₃) | Et |

**[Table 1191]**

| Compound Number | R³ | R⁴ | R⁵ | R¹¹ |
|---|---|---|---|---|
| G-3119 | Cl | F | CH₂CH₂Ph(2,4,5-F₃) | Et |
| G-3120 | Cl | F | CH₂CH₂Ph(3,4,5-F₃) | Et |
| G-3121 | Cl | F | CH₂CH₂Ph(2,4,6-F₃) | Et |
| G-3122 | Cl | F | CH₂CH₂CH₂Ph | Et |
| G-3123 | Cl | F | CH₂CH₂CH₂Ph(3-CF₃) | Et |
| G-3124 | Cl | F | CH₂CH₂CH₂Ph(4-CF₃) | Et |
| G-3125 | Cl | F | CH₂CH₂CH₂Ph(4-F) | Et |
| G-3126 | Cl | F | CH₂CH₂CH₂Ph[4-(*t*-Bu)] | Et |
| G-3127 | Cl | F | CH₂CH₂CH₂Ph(4-CN) | Et |
| G-3128 | Cl | F | CH₂CH₂CH₂Ph(4-CF₃) | Et |
| G-3129 | Cl | F | CH₂CH₂CH₂Ph(4-OCHF₂) | Et |
| G-3130 | Cl | F | CH₂CH₂CH₂Ph(4-OCF₃) | Et |
| G-3131 | Cl | F | CH₂CH₂CH₂Ph(4-SCHF₂) | Et |
| G-3132 | Cl | F | CH₂CH₂CH₂Ph(4-SCF₃) | Et |
| G-3133 | Cl | F | CH₂CH₂CH₂Ph[4-CF(CF₃)₂] | Et |
| G-3134 | Cl | F | CH₂CH₂CH₂Ph(3,4,5-F₃) | Et |
| G-3135 | Cl | F | CH₂CH₂CH₂Ph(2,4,6-F₃) | Et |
| G-3136 | Cl | F | CH₂CH₂CH₂CH₂Ph | Et |
| G-3137 | Cl | F | CH₂CH₂CH₂CH₂Ph(4-F) | Et |
| G-3138 | Cl | F | CH₂CH₂CH₂CH₂Ph(4-CF₃) | Et |
| G-3139 | Cl | F | CH₂CH₂CH₂CH₂Ph(4-OCF₃) | Et |
| G-3140 | Cl | F | CH₂CH₂CH₂CH₂Ph(4-SCF₃) | Et |
| G-3141 | Cl | F | CH₂CH₂CH₂CH₂CH₂Ph | Et |
| G-3142 | Cl | F | CH₂CH₂CH₂CH₂CH₂CH₂Ph | Et |
| G-3143 | Cl | F | CH₂CF₂Ph(4-F) | Et |
| G-3144 | Cl | F | CH₂CF₂Ph(4-CF₃) | Et |
| G-3145 | Cl | F | CH₂CF₂Ph(3,4,5-F₃) | Et |
| G-3146 | Cl | F | CH₂CH₂OPh | Et |
| G-3147 | Cl | F | CH₂CH₂OPh(4-F) | Et |
| G-3148 | Cl | F | CH₂CH₂OPh(4-CF₃) | Et |
| G-3149 | Cl | F | CH₂CH₂CH₂OPh | Et |
| G-3150 | Cl | F | CH₂CH₂CH₂OPh(4-Cl) | Et |
| G-3151 | Cl | F | CH₂CH₂CH₂OPh(4-CF₃) | Et |
| G-3152 | Cl | F | CH₂CH₂CH₂CH₂OPh(4-CF₃) | Et |
| G-3153 | Cl | F | CH₂CH₂CH₂CH₂OPh(4-OCF₃) | Et |
| G-3154 | Cl | F | CH₂CH₂CH₂CH₂CH₂OPh(4-CF₃) | Et |
| G-3155 | Cl | F | CH₂CH₂OCH₂Ph | Et |
| G-3156 | Cl | F | CH₂CH₂CH₂OCH₂Ph | Et |
| G-3157 | Cl | F | CH₂CH₂CH₂CH₂OCH₂Ph | Et |
| G-3158 | Cl | F | CH₂CH₂CH₂CH₂CH₂OCH₂Ph | Et |
| G-3159 | Cl | F | CH₂CH₂SPh | Et |
| G-3160 | Cl | F | CH₂CH₂CH₂SPh | Et |
| G-3161 | Cl | F | CH₂CH₂CH₂SPh(4-F) | Et |
| G-3162 | Cl | F | CH₂CH₂CH₂SPh(4-Cl) | Et |

**[Table 1192]**

| Compound Number | R³ | R⁴ | R⁵ | R¹¹ |
|---|---|---|---|---|
| G-3163 | Cl | F | CH₂CH₂CH₂SPh(4-Br) | Et |
| G-3164 | Cl | F | CH₂CH₂CH₂SPh[4-(*t*-Bu)] | Et |
| G-3165 | Cl | F | CH₂CH₂CH₂SPh(3-CF₃) | Et |
| G-3166 | Cl | F | CH₂CH₂CH₂SPh(4-CF₃) | Et |
| G-3167 | Cl | F | CH₂CH₂CH₂SPh(3-SCF₃) | Et |
| G-3168 | Cl | F | CH₂CH₂CH₂SPh(4-SCF₃) | Et |
| G-3169 | Cl | F | CH₂CH₂CH₂CH₂SPh | Et |
| G-3170 | Cl | F | CH₂CH₂CH₂CH₂SPh(4-F) | Et |
| G-3171 | Cl | F | CH₂CH₂CH₂CH₂SPh(4-Cl) | Et |
| G-3172 | Cl | F | CH₂CH₂CH₂CH₂SPh[4-(*t*-Bu)] | Et |
| G-3173 | Cl | F | CH₂CH₂CH₂CH₂SPh(4-CF₃) | Et |
| G-3174 | Cl | F | CH₂CH₂CH₂CH₂CH₂SPh | Et |
| G-3175 | Cl | F | CH₂CH₂CH₂CH₂CH₂SPh(4-F) | Et |
| G-3176 | Cl | F | CH₂CH₂CH₂CH₂CH₂SPh(4-Cl) | Et |
| G-3177 | Cl | F | CH₂CH₂CH₂CH₂CH₂SPh(4-CF₃) | Et |
| G-3178 | Cl | F | CH₂CH₂S(=O)Ph | Et |
| G-3179 | Cl | F | CH₂CH₂CH₂S(=O)Ph | Et |
| G-3180 | Cl | F | CH₂CH₂CH₂S(=O)Ph(4-F) | Et |
| G-3181 | Cl | F | CH₂CH₂CH₂S(=O)Ph[4-(*t*-Bu)] | Et |
| G-3182 | Cl | F | CH₂CH₂CH₂S(=O)Ph(4-CF₃) | Et |
| G-3183 | Cl | F | CH₂CH₂S(=O)₂Ph | Et |
| G-3184 | Cl | F | CH₂CH₂S(=O)₂Ph(4-CF₃) | Et |
| G-3185 | Cl | F | CH₂CH₂CH₂S(=O)₂Ph(4-F) | Et |
| G-3186 | Cl | F | CH₂CH₂CH₂S(=O)₂Ph(4-Cl) | Et |
| G-3187 | Cl | F | CH₂CH₂CH₂S(=O)₂Ph(4-CF₃) | Et |
| G-3188 | Cl | F | CH₂CH₂CH₂CH₂S(=O)₂Ph | Et |
| G-3189 | Cl | F | CH₂CH₂CH₂CH₂S(=O)₂Ph(4-Cl) | Et |
| G-3190 | Cl | F | CH₂CH₂CH₂CH₂S(=O)₂Ph(4-CF₃) | Et |
| G-3191 | Cl | F | CH₂CH₂SCH₂Ph | Et |
| G-3192 | Cl | F | CH₂CH₂CH₂SCH₂Ph | Et |
| G-3193 | Cl | F | CH₂CH₂CH₂SCH₂Ph(2-Cl) | Et |
| G-3194 | Cl | F | CH₂CH₂CH₂SCH₂Ph(3-Cl) | Et |
| G-3195 | Cl | F | CH₂CH₂CH₂SCH₂Ph(4-Cl) | Et |
| G-3196 | Cl | F | CH₂CH₂CH₂SCH₂Ph(2-SCF₃) | Et |
| G-3197 | Cl | F | CH₂CH₂CH₂SCH₂Ph(3-CF₃) | Et |
| G-3198 | Cl | F | CH₂CH₂CH₂SCH₂Ph(4-CF₃) | Et |
| G-3199 | Cl | F | CH₂CH₂CH₂SCH₂Ph(4-NO₂) | Et |
| G-3200 | Cl | F | CH₂CH₂CH₂CH₂SCH₂Ph | Et |
| G-3201 | Cl | F | CH₂CH₂CH₂CH₂SCH₂Ph(4-Cl) | Et |
| G-3202 | Cl | F | CH₂CH₂CH₂CH₂SCH₂Ph(4-CF₃) | Et |
| G-3203 | Cl | F | CH₂CH₂CH₂CH₂SCH₂Ph(4-CN) | Et |
| G-3204 | Cl | F | CH₂CH₂CH₂CH₂CH₂SCH₂Ph | Et |
| G-3205 | Cl | F | CH₂CH₂SCH₂CH₂Ph | Et |
| G-3206 | Cl | F | CH₂CH₂CH₂SCH₂CH₂Ph | Et |

**[Table 1193]**

| Compound Number | R³ | R⁴ | R⁵ | R¹¹ |
|---|---|---|---|---|
| G-3207 | Cl | F | CH₂CH₂CH₂CH₂SCH₂CH₂Ph | Et |
| G-3208 | Cl | F | CH₂CH₂SCH(Me)Ph | Et |
| G-3209 | Cl | F | CH₂CH₂CH₂SCH(Me)Ph | Et |
| G-3210 | Cl | F | CH₂CH₂CH₂CH₂SCH(Me)Ph | Et |
| G-3211 | Cl | F | CH₂CH₂S(=O)CH₂Ph | Et |
| G-3212 | Cl | F | CH₂CH₂S(=O)CH₂Ph(4-CF₃) | Et |
| G-3213 | Cl | F | CH₂CH₂CH₂S(=O)CH₂Ph | Et |
| G-3214 | Cl | F | CH₂CH₂CH₂S(=O)CH₂Ph(2-SCF₃) | Et |
| G-3215 | Cl | F | CH₂CH₂CH₂S(=O)CH₂Ph(4-SCF₃) | Et |
| G-3216 | Cl | F | CH₂CH₂CH₂S(=O)CH₂Ph(4-Cl) | Et |
| G-3217 | Cl | F | CH₂CH₂CH₂S(=O)CH₂Ph(4-CF₃) | Et |
| G-3218 | Cl | F | CH₂CH₂CH₂CH₂S(=O)CH₂Ph | Et |
| G-3219 | Cl | F | CH₂CH₂CH₂CH₂S(=O)CH₂Ph(4-CF₃) | Et |
| G-3220 | Cl | F | CH₂CH₂CH₂CH₂CH₂S(=O)CH₂Ph(4-CF₃) | Et |
| G-3221 | Cl | F | CH₂CH₂S(=O)₂CH₂Ph | Et |
| G-3222 | Cl | F | CH₂CH₂S(=O)₂CH₂Ph(4-CF₃) | Et |
| G-3223 | Cl | F | CH₂CH₂CH₂S(=O)₂CH₂Ph | Et |
| G-3224 | Cl | F | CH₂CH₂CH₂S(=O)₂CH₂Ph(4-Cl) | Et |
| G-3225 | Cl | F | CH₂CH₂CH₂S(=O)₂CH₂Ph(4-CF₃) | Et |
| G-3226 | Cl | F | CH₂CH₂CH₂CH₂S(=O)₂CH₂Ph | Et |
| G-3227 | Cl | F | CH₂CH₂CH₂CH₂S(=O)₂CH₂Ph(4-CF₃) | Et |
| G-3228 | Cl | F | CH₂CH₂CH₂CH₂CH₂S(=O)₂CH₂Ph | Et |
| G-3229 | Cl | F | CH₂CH₂CH₂CH₂CH₂S(=O)₂CH₂Ph(4-CF₃) | Et |
| G-3230 | Cl | F | CH₂CH₂ON=CH(*t*-Bᵤ) | Et |
| G-3231 | Cl | F | CH₂CH₂CH₂ON=CH(*t*-Bu) | Et |
| G-3232 | Cl | F | CH₂CH₂CH₂CH₂ON=C(Me)(*c*-Pr) | Et |
| G-3233 | Cl | F | CH₂CH₂ON=C(Me)CF₃ | Et |
| G-3234 | Cl | F | CH₂CH₂CH₂ON=CHCF₃ | Et |
| G-3235 | Cl | F | CH₂CH₂CH₂ON=C(Me)CF₃ | Et |
| G-3236 | Cl | F | CH₂CH₂CH₂ON=C(Me)CCl₃ | Et |
| G-3237 | Cl | F | CH₂CH₂CH₂CH₂ON=CHCF₃ | Et |
| G-3238 | Cl | F | CH₂CH₂CH₂CH₂ON=C(Me)CF₃ | Et |
| G-3239 | Cl | F | CH₂CH₂CH₂CH₂CH₂ON=C(Me)CF3 | Et |
| G-3240 | Cl | F | CH₂CH₂ON=CHPh | Et |
| G-3241 | Cl | F | CH₂CH₂ON=CHPh(4-CF₃) | Et |
| G-3242 | Cl | F | CH₂CH₂ON=CHPh(4-SCF₃) | Et |
| G-3243 | Cl | F | CH₂CH₂CH₂ON=CHPh(3-CF₃) | Et |
| G-3244 | Cl | F | CH₂CH₂CH₂ON=CHPh(4-CF₃) | Et |
| G-3245 | Cl | F | CH₂CH₂CH₂ON=CHPh(4-SCF₃) | Et |
| G-3246 | Cl | F | CH₂CH₂CH₂ON=C(Me)Ph(4-CF₃) | Et |
| G-3247 | Cl | F | CH₂CH₂CH₂CH₂ON=CHPh(4-SCF₃) | Et |
| G-3248 | Cl | F | CH₂CH₂(adamant-1-yl) | Et |
| G-3249 | Cl | F | CH₂CH₂CH₂(3-CF₃-pyrazol-1-yl) | Et |
| G-3250 | Cl | F | CH₂CH₂CH₂CH₂(3-CF₃-pyrazol-1-yl) | Et |

**[Table 1194]**

| Compound Number | R³ | R⁴ | R⁵ | R¹¹ |
|---|---|---|---|---|
| G-3251 | Cl | F | CH₂CH₂CH₂CH₂CH₂(3-CF₃-pyrazol-1-yl) | Et |
| G-3252 | Cl | F | CH₂(1-Me-4-CF₃-imidazol-5-yl) | Et |
| G-3253 | Cl | F | CH₂CH₂(1-Me-4-CF₃-imidazol-5-yl) | Et |
| G-3254 | Cl | F | CH₂CH₂CH₂(1-Me-4-CF₃-imidazol-5-yl) | Et |
| G-3255 | Cl | F | CH₂CH₂CH₂(3-CF₃-1,2,4-triazol-1-yl) | Et |
| G-3256 | Cl | F | CH₂CH₂CH₂CH₂(3-CF₃-1,2,4-triazol-1-yl) | Et |
| G-3257 | Cl | F | CH₂CH₂CH₂CH₂CH₂(3-CF₃-1,2,4-triazol-1-yl) | Et |
| G-3258 | Cl | F | CH₂CH₂CH₂(4-CF₃-thiazol-2-yl) | Et |
| G-3259 | Cl | F | CH₂CH₂CH₂CH₂(4-CF₃-thiazol-2-yl) | Et |
| G-3260 | Cl | F | CH₂CH₂CH₂CH₂CH₂(4-CF₃-thiazol-2-yl) | Et |
| G-3261 | Cl | F | CH₂CH₂CH₂(2-CF₃-pyridin-5-yl) | Et |
| G-3262 | Cl | F | CH₂CH₂CH₂CH₂(2-CF₃-pyridin-5-yl) | Et |
| G-3263 | Cl | F | CH₂CH₂CH₂CH₂CH₂(2-CF₃-pyridin-5-yl) | Et |
| G-3264 | Cl | F | CH₂(4-CF₃-pyrimidin-2-yl) | Et |
| G-3265 | Cl | F | CH₂CH₂(4-CF₃-pyrimidin-2-yl) | Et |
| G-3266 | Cl | F | CH₂CH₂CH₂(4-CF₃-pyrimidin-2-yl) | Et |
| G-3267 | Cl | F | CH₂CH₂O(3-Cl-5-CF₃-pyridin-2-yl) | Et |
| G-3268 | Cl | F | CH₂CH₂CH₂O(3-Cl-5-CF₃-pyridin-2-yl) | Et |
| G-3269 | Cl | F | CH₂CH₂CH₂CH₂O(3-Cl-5-CF₃-pyridin-2-yl) | Et |
| G-3270 | Cl | F | CH₂CH₂CH₂CH₂CH₂O(3-Cl-5-CF₃-pyridin-2-yl) | Et |
| G-3271 | Cl | F | CH₂CH₂N(Phth) | Et |
| G-3272 | Cl | F | CH₂CH₂CH₂N(Phth) | Et |
| G-3273 | Cl | F | CH₂CH₂CH₂CH₂N(Phth) | Et |
| G-3274 | Cl | F | CH₂CH₂CH₂CH₂CH₂N(Phth) | Et |
| G-3275 | Cl | F | CH₂CH(Me)CH₂N(Phth) | Et |
| G-3276 | Cl | F | CH₂[1,3-dioxolan(2-CF₃)-2-yl] | Et |
| G-3277 | Cl | F | CH₂(azetidin-3-yl) | Et |
| G-3278 | Cl | F | CH₂(pyrrolidin-3-yl) | Et |
| G-3279 | Cl | F | CH₂(piperidin-3-yl) | Et |
| G-3280 | Cl | F | CH₂{azetidin[1-C(=O)O(*t*-Bu)]-3-yl} | Et |
| G-3281 | Cl | F | CH₂{pyrrolidin[1-C(=O)O(*t*-Bu)]-3-yl} | Et |
| G-3282 | Cl | F | CH₂{piperidin[1-C(=O)O(*t*-Bu)]-3-yl} | Et |
| G-3283 | Cl | F | CH₂{azetidin[1-C(=O)CF₃]-3-yl} | Et |
| G-3284 | Cl | F | CH₂{azetidin[1-S(=O)₂CF₃]-3-yl} | Et |
| G-3285 | Cl | F | CH₂{pyrrolidin[1-C(=O)CF₃]-3-yl} | Et |
| G-3286 | Cl | F | CH₂{pyrrolidin[1-S(=O)₂CF₃]-3-yl} | Et |
| G-3287 | Cl | F | CH₂{piperidin[1-C(=O)CF₃]-3-yl} | Et |
| G-3288 | Cl | F | CH₂{piperidin[1-S(=O)₂CF₃]-3-yl} | Et |
| G-3289 | Cl | F | CH₂CH₂CH₂CN | Et |
| G-3290 | Cl | F | CH₂CH₂CH₂CH₂CN | Et |
| G-3291 | Cl | F | CH₂CH₂CH₂CH₂CH₂CN | Et |
| G-3292 | Cl | F | CH₂CH₂CH₂CH₂CH₂CH₂CN | Et |
| G-3293 | Cl | F | CH₂CH₂CH₂C(=O)OH | Et |
| G-3294 | Cl | F | CH₂CH₂CH₂CH₂C(=O)OH | Et |

**[Table 1195]**

| Compound Number | R³ | R⁴ | R⁵ | R¹¹ |
|---|---|---|---|---|
| G-3295 | Cl | F | CH₂CH₂CH₂CH₂CH₂C(=O)OH | Et |
| G-3296 | Cl | F | CH₂CH₂SCN | Et |
| G-3297 | Cl | F | CH₂CH₂CH₂SCN | Et |
| G-3298 | Cl | F | CH₂CH₂CH₂CH₂SCN | Et |
| G-3299 | Cl | F | CH₂CH₂CH₂CH₂CH₂SCN | Et |
| G-3300 | Cl | F | CH₂CH₂CH₂CH₂CH₂CH₂SCN | Et |
| G-3301 | Cl | F | CH₂CH₂CH₂CH₂CH₂CH₂CH₂SCN | Et |
| G-3302 | Cl | F | CH₂CH₂CH₂CH₂CH₂CH₂CH₂CH₂SCN | Et |
| G-3303 | Cl | F | CH₂CH₂CH(Me)SCN | Et |
| G-3304 | Cl | F | CH₂CH(Me)CH₂SCN | Et |
| G-3305 | Cl | F | CH(Me)CH₂CH₂SCN | Et |
| G-3306 | Cl | F | CH₂CH₂CH₂CH(Me)SCN | Et |
| G-3307 | Cl | F | CH₂CH₂CH(Me)CH₂SCN | Et |
| G-3308 | Cl | F | CH₂CH(Me)CH₂CH₂SCN | Et |
| G-3309 | Cl | F | CH(Me)CH₂CH₂CH₂SCN | Et |
| G-3310 | Cl | F | CH₂CH₂CH₂CH₂CH(Me)SCN | Et |
| G-3311 | Cl | F | CH₂CH₂CH₂CH(Me)CH₂SCN | Et |
| G-3312 | Cl | F | CH₂CH₂CH(Me)CH₂CH₂SCN | Et |
| G-3313 | Cl | F | CH₂CH(Me)CH₂CH₂CH₂SCN | Et |
| G-3314 | Cl | F | CH(Me)CH₂CH₂CH₂CH₂SCN | Et |
| G-3315 | Cl | F | CH₂CH₂ONH₂ | Et |
| G-3316 | Cl | F | CH₂CH₂CH₂ONH₂ | Et |
| G-3317 | Cl | F | CH₂CH₂CH₂CH₂ONH₂ | Et |
| G-3318 | Cl | F | CH₂CH₂CH₂CH₂CH₂ONH₂ | Et |
| G-3319 | Cl | F | CH₂CH₂CH₂CH₂CH₂CH₂ONH₂ | Et |
| G-3320 | Me | F | Me | Et |
| G-3321 | Me | F | Et | Et |
| G-3322 | Me | F | *n*-Pr | Et |
| G-3323 | Me | F | *i*-Pr | Et |
| G-3324 | Me | F | *n*-Bu | Et |
| G-3325 | Me | F | *n*-Pen | Et |
| G-3326 | Me | F | *n*-Hex | Et |
| G-3327 | Me | F | *n*-Heptyl | Et |
| G-3328 | Me | F | *n*-Octyl | Et |
| G-3329 | Me | F | *n*-Nonyl | Et |
| G-3330 | Me | F | *n*-Decyl | Et |
| G-3331 | Me | F | *c*-Pr | Et |
| G-3332 | Me | F | *c*-Pen | Et |
| G-3333 | Me | F | *c*-Hex | Et |
| G-3334 | Me | F | CH₂C(Me)=CH₂ | Et |
| G-3335 | Me | F | CH₂CH₂CH=CH₂ | Et |
| G-3336 | Me | F | CH₂CH₂CH=C(CH₃)₂ | Et |
| G-3337 | Me | F | CH₂CH₂CH₂CH=CH₂ | Et |
| G-3338 | Me | F | CH₂CH₂CH₂CH=C(CH₃)₂ | Et |

**[Table 1196]**

| Compound Number | R³ | R⁴ | R⁵ | R¹¹ |
|---|---|---|---|---|
| G-3339 | Me | F | CH₂CH₂CH₂CH₂CH=CH₂ | Et |
| G-3340 | Me | F | CH₂CH₂CH₂CH₂CH=C(CH₃)₂ | Et |
| G-3341 | Me | F | CH₂CH₂CH≡CH | Et |
| G-3342 | Me | F | CH₂CH₂CH₂CH≡CH | Et |
| G-3343 | Me | F | CH₂CH₂CH₂CH₂CH≡CH | Et |
| G-3344 | Me | F | CH₂CH₂CH₂Cl | Et |
| G-3345 | Me | F | CH₂CH₂CH₂Br | Et |
| G-3346 | Me | F | CH₂CH₂CH₂CH₂Cl | Et |
| G-3347 | Me | F | CH₂CH₂CH₂CH₂Br | Et |
| G-3348 | Me | F | CH₂CH₂CH₂CH₂CH₂Cl | Et |
| G-3349 | Me | F | CH₂CH₂CH₂CH₂CH₂Br | Et |
| G-3350 | Me | F | CH₂CH₂CH₂CH₂CH₂CH₂Cl | Et |
| G-3351 | Me | F | CH₂CH₂CH₂CH₂CH₂CH₂Br | Et |
| G-3352 | Me | F | CH₂CH₂CH₂CH₂CH₂CH₂CH₂Cl | Et |
| G-3353 | Me | F | CH₂CH₂CH₂CH₂CH₂CH₂CH₂Br | Et |
| G-3354 | Me | F | CH₂CH₂CH₂CH₂CH₂CH₂CH₂CH₂Br | Et |
| G-3355 | Me | F | CH₂CH(Me)CH₂Cl | Et |
| G-3356 | Me | F | CH₂CH(Me)CH₂Br | Et |
| G-3357 | Me | F | CH₂CH₂CH(Me)CH₂CH₂Cl | Et |
| G-3358 | Me | F | CH₂CH₂CH(Me)CH₂CH₂Br | Et |
| G-3359 | Me | F | CH₂CH₂CF=CF₂ | Et |
| G-3360 | Me | F | CH₂CH=C(Cl)CF₃ | Et |
| G-3361 | Me | F | CH₂CH₂CH₂CF=CF₂ | Et |
| G-3362 | Me | F | CH₂CH₂CH₂CH₂CF=CF₂ | Et |
| G-3363 | Me | F | CH₂CH₂CH₂CH₂CH₂CF=CF₂ | Et |
| G-3364 | Me | F | CH₂(*t*-Bu) | Et |
| G-3365 | Me | F | CH₂CH₂(*t*-Bu) | Et |
| G-3366 | Me | F | CH₂CH₂CH₂(*t*-Bu) | Et |
| G-3367 | Me | F | CH₂CH₂CH₂CH₂(*t*-Bu) | Et |
| G-3368 | Me | F | CH₂CH₂CH₂CH₂CH₂(*t*-Bu) | Et |
| G-3369 | Me | F | CH₂CH₂CH₂CH₂CH₂CH₂(*t*-Bu) | Et |
| G-3370 | Me | F | CH₂CF₃ | Et |
| G-3371 | Me | F | CH₂CH₂CF₃ | Et |
| G-3372 | Me | F | CH₂CH₂CH₂CF₃ | Et |
| G-3373 | Me | F | CH₂CH₂CH₂CH₂CF₃ | Et |
| G-3374 | Me | F | CH₂CH₂CH₂CH₂CH₂CF₃ | Et |
| G-3375 | Me | F | CH₂CH₂CH₂CH₂CH₂CH₂CF₃ | Et |
| G-3376 | Me | F | CH₂CH₂CH₂CH₂CH₂CH₂CH₂CF₃ | Et |
| G-3377 | Me | F | CH₂CF₂CF₃ | Et |
| G-3378 | Me | F | CF₂CHFCF₃ | Et |
| G-3379 | Me | F | CF₂CF₂CF₃ | Et |
| G-3380 | Me | F | CH₂CF₂CF₂CF₃ | Et |
| G-3381 | Me | F | CH₂CF₂CF₂CF₂CF₃ | Et |
| G-3382 | Me | F | CH₂CF₂CF₂CF₂CHF₂ | Et |

**[Table 1197]**

| Compound Number | R³ | R⁴ | R⁵ | R¹¹ |
|---|---|---|---|---|
| G-3383 | Me | F | CH(CF₃)CF₃ | Et |
| G-3384 | Me | F | CH₂CF(CF₃)CF₃ | Et |
| G-3385 | Me | F | CH₂CF₂CF(CF₃)CF₃ | Et |
| G-3386 | Me | F | CH₂CF(CF₃)CF₂CF₃ | Et |
| G-3387 | Me | F | CH₂CF₂CF₂CF(CF₃)CF₃ | Et |
| G-3388 | Me | F | CH₂CF₂CF(CF₃)CF₂CF₃ | Et |
| G-3389 | Me | F | CH₂CF(CF₃)CF₂CF₂CF₃ | Et |
| G-3390 | Me | F | CH₂(*c*-Pr) | Et |
| G-3391 | Me | F | CH₂[*c*-Pr(1-Me)] | Et |
| G-3392 | Me | F | CH₂[*c*-Pr(1-Ph)] | Et |
| G-3393 | Me | F | CH₂[*c*-Pr(1-NH₂)] | Et |
| G-3394 | Me | F | CH₂[*c*-Pr(1-NHC(=O)O(*t*-Bu)] | Et |
| G-3395 | Me | F | CH₂{*c*-Pr[1-NHS(=O)₂CF₃]} | Et |
| G-3396 | Me | F | CH₂{*c*-Pr[1-Ph(4-CF₃)]} | Et |
| G-3397 | Me | F | CH₂{*c*-Pr[1-Ph(3,4,5-F₃)]} | Et |
| G-3398 | Me | F | CH₂{*c*-Hex[4-(*t*-Bu)]} | Et |
| G-3399 | Me | F | CH₂[*c*-Hex(4-CF₃)] | Et |
| G-3400 | Me | F | CH₂CH₂(*c*-Pr) | Et |
| G-3401 | Me | F | CH₂CH₂(*c*-Hex) | Et |
| G-3402 | Me | F | CH₂CH₂[*c*-Hex(4-CF₃)] | Et |
| G-3403 | Me | F | CH₂CH₂[*c*-Hex(4-SCF₃)] | Et |
| G-3404 | Me | F | CH₂CH₂CH₂(*c*-Pr) | Et |
| G-3405 | Me | F | CH₂CH₂CH₂(*c*-Hex) | Et |
| G-3406 | Me | F | CH₂CH₂CH₂{*c*-Hex[4-(*t*-Bu)]} | Et |
| G-3407 | Me | F | CH₂CH₂CH₂[*c*-Hex(4-CF₃)] | Et |
| G-3408 | Me | F | CH₂CH₂CH₂[*c*-Hex(4-SCF₃)] | Et |
| G-3409 | Me | F | CH₂CH₂CH₂CH₂(*c*-Pr) | Et |
| G-3410 | Me | F | CH₂CH₂CH₂CH₂(*c*-Hex) | Et |
| G-3411 | Me | F | CH₂CH₂CH₂CH₂[*c*-Hex(4-CF₃)] | Et |
| G-3412 | Me | F | CH₂[*c*-Pr(2,2-F₂)] | Et |
| G-3413 | Me | F | CH₂[*c*-Hex(4,4-F₂)] | Et |
| G-3414 | Me | F | CH₂CH₂[*c*-Pr(2,2-F₂)] | Et |
| G-3415 | Me | F | CH₂CH₂[*c*-Hex(4,4-F₂)] | Et |
| G-3416 | Me | F | CH₂CH₂CH₂[*c*-Pr(2,2-F₂)] | Et |
| G-3417 | Me | F | CH₂CH₂CH₂CH₂[*c*-Pr(2,2-F₂)] | Et |
| G-3418 | Me | F | CH₂CH₂OH | Et |
| G-3419 | Me | F | CH₂CH₂CH₂OH | Et |
| G-3420 | Me | F | CH₂CH₂CH₂CH₂OH | Et |
| G-3421 | Me | F | CH₂CH₂CH₂CH₂CH₂OH | Et |
| G-3422 | Me | F | CH₂CH₂CH₂CH₂CH₂CH₂OH | Et |
| G-3423 | Me | F | CH₂C(=O)Me | Et |
| G-3424 | Me | F | CH₂CH(OH)Me | Et |
| G-3425 | Me | F | CH₂CH(OH)CF₃ | Et |
| G-3426 | Me | F | CH₂C(OH)₂CF₃ | Et |

**[Table 1198]**

| Compound Number | R³ | R⁴ | R⁵ | R¹¹ |
|---|---|---|---|---|
| G-3427 | Me | F | CH₂C(CF₃)=NOH | Et |
| G-3428 | Me | F | CH₂C(CF₃)=NOMe | Et |
| G-3429 | Me | F | CH₂CH(CF₃)NH₂ | Et |
| G-3430 | Me | F | CH₂CH(CF₃)NHSO₂CF₃ | Et |
| G-3431 | Me | F | CH₂CH₂OMe | Et |
| G-3432 | Me | F | CH₂CH₂CH₂OMe | Et |
| G-3433 | Me | F | CH₂CH₂CH₂CH₂OMe | Et |
| G-3434 | Me | F | CH₂CH₂CH₂CH₂CH₂OMe | Et |
| G-3435 | Me | F | CH₂CH₂CH₂CH₂CH₂CH₂OMe | Et |
| G-3436 | Me | F | CH₂CH₂OCHF₂ | Et |
| G-3437 | Me | F | CH₂CH₂CH₂OCHF₂ | Et |
| G-3438 | Me | F | CH₂CH₂CH₂CH₂OCHF₂ | Et |
| G-3439 | Me | F | CH₂CH₂CH₂CH₂CH₂OCHF₂ | Et |
| G-3440 | Me | F | CH₂CH₂CH₂CH₂CH₂CH₂OCHF₂ | Et |
| G-3441 | Me | F | CH₂CH₂OCH₂CF₃ | Et |
| G-3442 | Me | F | CH₂CH₂CH₂OCH₂CF₃ | Et |
| G-3443 | Me | F | CH₂CH₂CH₂CH₂OCH₂CF₃ | Et |
| G-3444 | Me | F | CH₂CH₂CH₂CH₂CH₂OCH₂CF₃ | Et |
| G-3445 | Me | F | CH₂CH₂CH₂CH₂CH₂CH₂OCH₂CF₃ | Et |
| G-3446 | Me | F | CH₂CH₂CH₂CH₂CH₂CH₂CH₂OCH₂CF₃ | Et |
| G-3447 | Me | F | CH₂CH₂OC(CF₃)₃ | Et |
| G-3448 | Me | F | CH₂CH₂CH₂OC(CF₃)₃ | Et |
| G-3449 | Me | F | CH₂CH₂CH₂CH₂OC(CF₃)₃ | Et |
| G-3450 | Me | F | CH₂CH₂CH₂CH₂CH₂OC(CF₃)₃ | Et |
| G-3451 | Me | F | CF₂CHFOCF₂CF₂CF₃ | Et |
| G-3452 | Me | F | CH₂CH₂O(*c*-Pr) | Et |
| G-3453 | Me | F | CH₂CH₂O(*c*-Pen) | Et |
| G-3454 | Me | F | CH₂CH₂O(*c*-Hex) | Et |
| G-3455 | Me | F | CH₂CH₂CH₂O(*c*-Pr) | Et |
| G-3456 | Me | F | CH₂CH₂CH₂O(*c*-Pen) | Et |
| G-3457 | Me | F | CH₂CH₂CH₂O(*c*-Hex) | Et |
| G-3458 | Me | F | CH₂CH₂CH₂CH₂O(*c*-Pr) | Et |
| G-3459 | Me | F | CH₂CH₂CH₂CH₂O(*c*-Pen) | Et |
| G-3460 | Me | F | CH₂CH₂CH₂CH₂O(*c*-Hex) | Et |
| G-3461 | Me | F | CH₂CH₂O[*c*-Pr(2,2-F₂)] | Et |
| G-3462 | Me | F | CH₂CH₂CH₂O[*c*-Pr(2,2-F₂)] | Et |
| G-3463 | Me | F | CH₂CH₂CH₂CH₂O[*c*-Pr(2,2-F₂)] | Et |
| G-3464 | Me | F | CH₂CH₂OCH₂CH₂OCH₃ | Et |
| G-3465 | Me | F | CH₂CH₂OCH₂CH₂OCH₂CF₃ | Et |
| G-3466 | Me | F | CH₂CF₂OCF₂CF₂OCF₃ | Et |
| G-3467 | Me | F | CF₂CHFOCF₂CF₂OCF₃ | Et |
| G-3468 | Me | F | CF₂CHFOCF₂CF(CF₃)OCF₂CF₂CF₃ | Et |
| G-3469 | Me | F | CH₂CH₂SH | Et |
| G-3470 | Me | F | CH₂CH₂CH₂SH | Et |

**[Table 1199]**

| Compound Number | R³ | R⁴ | R⁵ | R¹¹ |
|---|---|---|---|---|
| G-3471 | Me | F | CH₂CH₂CH₂CH₂SH | Et |
| G-3472 | Me | F | CH₂CH₂CH₂CH₂CH₂SH | Et |
| G-3473 | Me | F | CH₂CH₂CH₂CH₂CH₂CH₂SH | Et |
| G-3474 | Me | F | CH₂CH₂S(*t*-Bu) | Et |
| G-3475 | Me | F | CH₂CH₂CH₂S(*t*-Bu) | Et |
| G-3476 | Me | F | CH₂CH₂CH₂CH₂S(*t*-Bu) | Et |
| G-3477 | Me | F | CH₂CH₂CH₂CH₂CH₂SCH₃ | Et |
| G-3478 | Me | F | CH₂CH₂CH₂CH₂CH₂SCH(CH₃)₂ | Et |
| G-3479 | Me | F | CH₂CH₂CH₂CH₂CH₂S(*t*-Bu) | Et |
| G-3480 | Me | F | CH₂CH₂CH₂CH₂CH₂CH₂SCH(CH₃)₂ | Et |
| G-3481 | Me | F | CH₂CH₂CH₂CH₂CH₂CH₂S(*t*-Bu) | Et |
| G-3482 | Me | F | CH₂CH₂CH₂CH₂CH₂CH₂CH₂S(*t*-Bu) | Et |
| G-3483 | Me | F | CH₂CH₂SCHF₂ | Et |
| G-3484 | Me | F | CH₂CH₂CH₂SCHF₂ | Et |
| G-3485 | Me | F | CH₂CH₂CH₂CH₂SCHF₂ | Et |
| G-3486 | Me | F | CH₂CH₂CH₂CH₂CH₂SCHF₂ | Et |
| G-3487 | Me | F | CH₂CH₂CH₂CH₂CH₂CH₂SCHF₂ | Et |
| G-3488 | Me | F | CH₂CH₂CH₂CH₂CH₂CH₂CH₂SCHF₂ | Et |
| G-3489 | Me | F | CH₂CH₂SCF₃ | Et |
| G-3490 | Me | F | CH₂CH₂CH₂SCF₃ | Et |
| G-3491 | Me | F | CH₂CH₂CH₂CH₂SCF₃ | Et |
| G-3492 | Me | F | CH₂CH₂CH₂CH₂CH₂SCF₃ | Et |
| G-3493 | Me | F | CH₂CH₂CH₂CH₂CH₂CH₂SCF₃ | Et |
| G-3494 | Me | F | CH₂CH₂CH₂CH₂CH₂CH₂CH₂SCF₃ | Et |
| G-3495 | Me | F | CH₂CH₂CH₂CH₂CH₂CH₂CH₂CH₂SCF₃ | Et |
| G-3496 | Me | F | CH₂CH(Me)SCF₃ | Et |
| G-3497 | Me | F | CH(Me)CH₂SCF₃ | Et |
| G-3498 | Me | F | CH₂CH₂CH(Me)SCF₃ | Et |
| G-3499 | Me | F | CH₂CH(Me)CH₂SCF₃ | Et |
| G-3500 | Me | F | CH(Me)CH₂CH₂SCF₃ | Et |
| G-3501 | Me | F | CH₂CH₂CH₂CH(Me)SCF₃ | Et |
| G-3502 | Me | F | CH₂CH₂CH(Me)CH₂SCF₃ | Et |
| G-3503 | Me | F | CH₂CH(Me)CH₂CH₂SCF₃ | Et |
| G-3504 | Me | F | CH(Me)CH₂CH₂CH₂SCF₃ | Et |
| G-3505 | Me | F | CH₂CH₂SCH₂CF₃ | Et |
| G-3506 | Me | F | CH₂CH₂CH₂SCH₂CF₃ | Et |
| G-3507 | Me | F | CH₂CH₂CH₂CH₂SCH₂CF₃ | Et |
| G-3508 | Me | F | CH₂CH₂CH₂CH₂CH₂SCH₂CF₃ | Et |
| G-3509 | Me | F | CH₂CH₂CH₂CH₂CH₂CH₂SCH₂CF₃ | Et |
| G-3510 | Me | F | CH₂CH₂CH₂CH₂CH₂CH₂CH₂SCH₂CF₃ | Et |
| G-3511 | Me | F | CH₂CH₂SCH(CF₃)₂ | Et |
| G-3512 | Me | F | CH₂CH₂CH₂SCH(CF₃)₂ | Et |
| G-3513 | Me | F | CH₂CH₂CH₂CH₂SCH(CF₃)₂ | Et |
| G-3514 | Me | F | CH₂CH₂CH₂CH₂CH₂SCH(CF₃)₂ | Et |

**[Table 1200]**

| Compound Number | R³ | R⁴ | R⁵ | R¹¹ |
|---|---|---|---|---|
| G-3515 | Me | F | CH₂CH₂CH₂CH₂CH₂CH₂SCH(CF₃)₂ | Et |
| G-3516 | Me | F | CH₂CH₂CH₂CH₂CH₂SCF₂CF₃ | Et |
| G-3517 | Me | F | CH₂CH₂CH₂CH₂CH₂CH₂SCF₂CF₃ | Et |
| G-3518 | Me | F | CH₂CH₂CH₂CH₂CH₂CH₂SCF(CF₃)₂ | Et |
| G-3519 | Me | F | CH₂CH₂CH₂CH₂CH₂CH₂CH₂SCF(CF₃)₂ | Et |
| G-3520 | Me | F | CH₂CH₂CH₂CH₂CH₂SCCl₃ | Et |
| G-3521 | Me | F | CH₂CH₂CH₂CH₂CH₂CH₂SCCl₃ | Et |
| G-3522 | Me | F | CH₂CH₂SCH₂CH=CH₂ | Et |
| G-3523 | Me | F | CH₂CH₂CH₂SCH₂CH=CH₂ | Et |
| G-3524 | Me | F | CH₂CH₂CH₂CH₂SCH₂CH=CH₂ | Et |
| G-3525 | Me | F | CH₂CH₂SCF=CFCF₃ | Et |
| G-3526 | Me | F | CH₂CH₂CH₂SCF=CFCF₃ | Et |
| G-3527 | Me | F | CH₂CH₂CH₂CH₂SCF=CFCF₃ | Et |
| G-3528 | Me | F | CH₂CH₂CH₂S(*c*-Pr) | Et |
| G-3529 | Me | F | CH₂CH₂CH₂S(*c*-Hex) | Et |
| G-3530 | Me | F | CH₂CH₂CH₂CH₂S(*c*-Pr) | Et |
| G-3531 | Me | F | CH₂CH₂CH₂CH₂S(*c*-Hex) | Et |
| G-3532 | Me | F | CH₂CH₂CH₂CH₂CH₂S(*c*-Pr) | Et |
| G-3533 | Me | F | CH₂CH₂CH₂CH₂CH₂S(*c*-Hex) | Et |
| G-3534 | Me | F | CH₂CH₂CH₂S[*c*-Hex(4,4-F₂)] | Et |
| G-3535 | Me | F | CH₂CH₂CH₂CH₂S[*c*-Hex(4,4-F₂)] | Et |
| G-3536 | Me | F | CH₂CH₂CH₂CH₂CH₂S[*c*-Hex(4,4-F₂)] | Et |
| G-3537 | Me | F | CH₂CH₂CH₂SCH₂(*c*-Pr) | Et |
| G-3538 | Me | F | CH₂CH₂CH₂SCH₂(*c*-Hex) | Et |
| G-3539 | Me | F | CH₂CH₂CH₂CH₂SCH₂(*c*-Pr) | Et |
| G-3540 | Me | F | CH₂CH₂CH₂CH₂SCH₂(*c*-Hex) | Et |
| G-3541 | Me | F | CH₂CH₂CH₂CH₂CH₂SCH₂(*c*-Pr) | Et |
| G-3542 | Me | F | CH₂CH₂CH₂CH₂CH₂SCH₂(*c*-Hex) | Et |
| G-3543 | Me | F | CH₂CH₂CH₂SCH₂[*c*-Hex(4,4-F₂)] | Et |
| G-3544 | Me | F | CH₂CH₂CH₂CH₂SCH₂[*c*-Hex(4,4-F₂)] | Et |
| G-3545 | Me | F | CH₂CH₂CH₂CH₂CH₂SCH₂[*c*-Hex(4,4-F₂)] | Et |
| G-3546 | Me | F | CH₂CH₂CH₂SCH₂SiMe₃ | Et |
| G-3547 | Me | F | CH₂CH₂CH₂CH₂SCH₂SiMe₃ | Et |
| G-3548 | Me | F | CH₂CH₂CH₂CH₂CH₂SCH₂SiMe₃ | Et |
| G-3549 | Me | F | CH₂CH₂OCH₂CH₂SCH₃ | Et |
| G-3550 | Me | F | CH₂CH₂OCH₂CH₂SCHF₂ | Et |
| G-3551 | Me | F | CH₂CH₂OCH₂CH₂SCF₃ | Et |
| G-3552 | Me | F | CH₂CH₂OCH₂CH₂SCH₂CF₃ | Et |
| G-3553 | Me | F | CH₂CH₂CH₂S(=O)(*t*-Bu) | Et |
| G-3554 | Me | F | CH₂CH₂CH₂CH₂S(=O)(*t*-Bu) | Et |
| G-3555 | Me | F | CH₂CH₂CH₂CH₂CH₂CH₂S(=O)(*t*-Bu) | Et |
| G-3556 | Me | F | CH₂CH₂S(=O)CHF₂ | Et |
| G-3557 | Me | F | CH₂CH₂CH₂S(=O)CHF₂ | Et |
| G-3558 | Me | F | CH₂CH₂CH₂CH₂S(=O)CHF₂ | Et |

**[Table 1201]**

| Compound Number | R³ | R⁴ | R⁵ | R¹¹ |
|---|---|---|---|---|
| G-3559 | Me | F | CH₂CH₂CH₂CH₂CH₂S(=O)CHF₂ | Et |
| G-3560 | Me | F | CH₂CH₂CH₂CH₂CH₂CH₂S(=O)CHF₂ | Et |
| G-3561 | Me | F | CH₂CH₂CH₂CH₂CH₂CH₂CH₂S(=O)CHF₂ | Et |
| G-3562 | Me | F | CH₂CH₂S(=O)CF₃ | Et |
| G-3563 | Me | F | CH₂CH₂CH₂S(=O)CF₃ | Et |
| G-3564 | Me | F | CH₂CH₂CH₂CH₂S(=O)CF₃ | Et |
| G-3565 | Me | F | CH₂CH₂CH₂CH₂CH₂S(=O)CF₃ | Et |
| G-3566 | Me | F | CH₂CH₂CH₂CH₂CH₂CH₂S(=O)CF₃ | Et |
| G-3567 | Me | F | CH₂CH₂CH₂CH₂CH₂CH₂CH₂S(=O)CF₃ | Et |
| G-3568 | Me | F | CH₂CH₂S(=O)CH₂CF₃ | Et |
| G-3569 | Me | F | CH₂CH₂CH₂S(=O)CH₂CF₃ | Et |
| G-3570 | Me | F | CH₂CH₂CH₂CH₂S(=O)CH₂CF₃ | Et |
| G-3571 | Me | F | CH₂CH₂CH₂CH₂CH₂S(=O)CH₂CF₃ | Et |
| G-3572 | Me | F | CH₂CH₂CH₂CH₂CH₂CH₂S(=O)CH₂CF₃ | Et |
| G-3573 | Me | F | CH₂CH₂CH₂CH₂CH₂CH₂CH₂S(=O)CH₂CF₃ | Et |
| G-3574 | Me | F | CH₂CH₂CH₂S(=O)(*c*-Pr) | Et |
| G-3575 | Me | F | CH₂CH₂CH₂CH₂S(=O)(*c*-Pr) | Et |
| G-3576 | Me | F | CH₂CH₂CH₂CH₂S(=O)(*c*-Pen) | Et |
| G-3577 | Me | F | CH₂CH₂CH₂CH₂S(=O)(*c*-Hex) | Et |
| G-3578 | Me | F | CH₂CH₂CH₂CH₂CH₂S(=O)(*c*-Pr) | Et |
| G-3579 | Me | F | CH₂CH₂CH₂CH₂CH₂S(=O)(*c*-Hex) | Et |
| G-3580 | Me | F | CH₂CH₂CH₂S(=O)[*c*-Hex(4,4-F₂)] | Et |
| G-3581 | Me | F | CH₂CH₂CH₂CH₂S(=O)[*c*-Hex(4,4-F₂)] | Et |
| G-3582 | Me | F | CH₂CH₂CH₂CH₂CH₂S(=O)[*c*-Hex(4,4-F₂)] | Et |
| G-3583 | Me | F | CH₂CH₂CH₂CH₂S(=O)₂Me | Et |
| G-3584 | Me | F | CH₂CH₂CH₂CH₂CH₂S(=O)₂Me | Et |
| G-3585 | Me | F | CH₂CH₂CH₂CH₂CH₂S(=O)₂(*t*-Bu) | Et |
| G-3586 | Me | F | CH₂CH₂S(=O)₂CHF₂ | Et |
| G-3587 | Me | F | CH₂CH₂CH₂S(=O)₂CHF₂ | Et |
| G-3588 | Me | F | CH₂CH₂CH₂CH₂S(=O)₂CHF₂ | Et |
| G-3589 | Me | F | CH₂CH₂CH₂CH₂CH₂S(=O)₂CHF₂ | Et |
| G-3590 | Me | F | CH₂CH₂CH₂CH₂CH₂CH₂S(=O)₂CHF₂ | Et |
| G-3591 | Me | F | CH₂CH₂S(=O)₂CF₃ | Et |
| G-3592 | Me | F | CH₂CH₂CH₂S(=O)₂CF₃ | Et |
| G-3593 | Me | F | CH₂CH₂CH₂CH₂S(=O)₂CF₃ | Et |
| G-3594 | Me | F | CH₂CH₂CH₂CH₂CH₂S(=O)₂CF₃ | Et |
| G-3595 | Me | F | CH₂CH₂CH₂CH₂CH₂CH₂S(=O)₂CF₃ | Et |
| G-3596 | Me | F | CH₂CH₂CH₂CH₂CH₂CH₂CH₂S(=O)₂CF₃ | Et |
| G-3597 | Me | F | CH₂CH₂CH₂CH₂CH₂CH₂CH₂CH₂S(=O)₂CF₃ | Et |
| G-3598 | Me | F | CH₂CH₂S(=O)₂CH₂CF₃ | Et |
| G-3599 | Me | F | CH₂CH₂CH₂S(=O)₂CH₂CF₃ | Et |
| G-3600 | Me | F | CH₂CH₂CH₂CH₂S(=O)₂CH₂CF₃ | Et |
| G-3601 | Me | F | CH₂CH₂CH₂CH₂CH₂S(=O)₂CH₂CF₃ | Et |
| G-3602 | Me | F | CH₂CH₂CH₂CH₂CH₂CH₂S(=O)₂CH₂CF₃ | Et |

**[Table 1202]**

| Compound Number | R³ | R⁴ | R⁵ | R¹¹ |
|---|---|---|---|---|
| G-3603 | Me | F | CH₂CH₂CH₂S(=O)₂(*c*-Pr) | Et |
| G-3604 | Me | F | CH₂CH₂CH₂CH₂S(=O)₂(*c*-Pr) | Et |
| G-3605 | Me | F | CH₂CH₂CH₂CH₂CH_{2S}(=O)₂(*c*-Pr) | Et |
| G-3606 | Me | F | CH₂CH₂CH₂S(=O)₂[*c*-Hex(4,4-F₂)] | Et |
| G-3607 | Me | F | CH₂CH₂CH₂CH₂S(=O)₂[*c*-Hex(4,4-F₂)] | Et |
| G-3608 | Me | F | CH₂CH₂CH₂CH₂CH₂S(=O)₂[*c*-Hex(4,4-F₂)] | Et |
| G-3609 | Me | F | CH₂CH₂CH₂C(=O)H | Et |
| G-3610 | Me | F | CH₂CH₂CH₂CH₂C(=O)H | Et |
| G-3611 | Me | F | CH₂CH₂CH₂CH₂CH₂C(=O)H | Et |
| G-3612 | Me | F | CH₂CH₂CH₂C(=O)(*t*-Bu) | Et |
| G-3613 | Me | F | CH₂CH₂CH₂CH₂C(=O)(*t*-Bu) | Et |
| G-3614 | Me | F | CH₂CH₂CH₂CH₂CH₂C(=O)(*t*-Bu) | Et |
| G-3615 | Me | F | CH₂CH₂CH₂C(=O)CF₃ | Et |
| G-3616 | Me | F | CH₂CH₂CH₂CH₂C(=O)CF₃ | Et |
| G-3617 | Me | F | CH₂CH₂CH₂CH₂CH₂C(=O)CF₃ | Et |
| G-3618 | Me | F | CH₂C(=O)Ph | Et |
| G-3619 | Me | F | CH₂C(=O)Ph(4-Cl) | Et |
| G-3620 | Me | F | CH₂C(=O)Ph(4-CF₃) | Et |
| G-3621 | Me | F | CH₂CH₂C(=O)Ph(4-CF₃) | Et |
| G-3622 | Me | F | CH₂CH₂CH₂C(=O)Ph(4-CF₃) | Et |
| G-3623 | Me | F | CH₂CH₂CH₂CH₂C(=O)Ph(4-CF₃) | Et |
| G-3624 | Me | F | CH₂CH₂CH₂CH₂CH₂C(=O)Ph(4-CF₃) | Et |
| G-3625 | Me | F | CH₂C(=O)OEt | Et |
| G-3626 | Me | F | CH₂CH₂C(=O)O(*t*-Bu) | Et |
| G-3627 | Me | F | CH₂CH₂CH₂C(=O)OEt | Et |
| G-3628 | Me | F | CH₂CH₂CH₂C(=O)O(*t*-Bu) | Et |
| G-3629 | Me | F | CH₂CH₂CH₂CH₂C(=O)O(*t*-Bu) | Et |
| G-3630 | Me | F | CH₂CH₂CH₂CH₂CH₂C(=O)O(*t*-Bu) | Et |
| G-3631 | Me | F | CH₂CH₂CH₂CH₂CH₂CH₂C(=O)O(*t*-Bu) | Et |
| G-3632 | Me | F | CH₂CH₂CH₂CH₂CH₂CH₂CH₂C(=O)OEt | Et |
| G-3633 | Me | F | CH₂CH₂C(=O)OCH₂CF₃ | Et |
| G-3634 | Me | F | CH₂CH₂CH₂C(=O)OCH₂CF₃ | Et |
| G-3635 | Me | F | CH₂CH₂CH₂CH₂C(=O)OCH₂CF₃ | Et |
| G-3636 | Me | F | CH₂CH₂CH₂CH₂CH₂C(=O)OCH₂CF₃ | Et |
| G-3637 | Me | F | CH₂CH₂C(=O)NH(*t*-Bu) | Et |
| G-3638 | Me | F | CH₂CH₂CH₂C(=O)NH(*t*-Bu) | Et |
| G-3639 | Me | F | CH₂CH₂CH₂C(=O)NH(*t*-Pen) | Et |
| G-3640 | Me | F | CH₂CH₂CH₂CH₂C(=O)NH(*t*-Bu) | Et |
| G-3641 | Me | F | CH₂CH₂C(=O)NHCH₂CF₃ | Et |
| G-3642 | Me | F | CH₂CH₂CH₂C(=O)NHCH₂CF₃ | Et |
| G-3643 | Me | F | CH₂CH₂CH₂CH₂C(=O)NHCH₂CF₃ | Et |
| G-3644 | Me | F | CH₂CH₂SC(=O)N(Me)₂ | Et |
| G-3645 | Me | F | CH₂CH₂CH₂SC(=O)N(Me)₂ | Et |
| G-3646 | Me | F | CH₂CH₂CH₂SC(=O)NH(*t*-Bu) | Et |

**[Table 1203]**

| Compound Number | R³ | R⁴ | R⁵ | R¹¹ |
|---|---|---|---|---|
| G-3647 | Me | F | CH₂CH₂CH₂CH₂CH₂SC(=O)NH(*t*-Bu) | Et |
| G-3648 | Me | F | CH₂CH₂SC(=O)NHCH₂CF₃ | Et |
| G-3649 | Me | F | CH₂CH₂CH₂SC(=O)NHCH₂CF₃ | Et |
| G-3650 | Me | F | CH₂CH₂CH₂CH₂SC(=O)NHCH₂CF₃ | Et |
| G-3651 | Me | F | CH₂CH₂CH₂OC(=O)H | Et |
| G-3652 | Me | F | CH₂CH₂CH₂CH₂OC(=O)H | Et |
| G-3653 | Me | F | CH₂CH₂CH₂CH₂CH₂OC(=O)H | Et |
| G-3654 | Me | F | CH₂CH₂CH₂OC(=O)Me | Et |
| G-3655 | Me | F | CH₂CH₂CH₂CH₂OC(=O)Me | Et |
| G-3656 | Me | F | CH₂CH₂CH₂CH₂CH₂OC(=O)Me | Et |
| G-3657 | Me | F | CH₂CH₂OC(=O)CF₃ | Et |
| G-3658 | Me | F | CH₂CH₂CH₂OC(=O)CF₃ | Et |
| G-3659 | Me | F | CH₂CH₂CH₂CH₂OC(=O)CF₃ | Et |
| G-3660 | Me | F | CH₂CH₂CH₂CH₂CH₂OC(=O)Ph(4-CF₃) | Et |
| G-3661 | Me | F | CH₂CH₂OC(=O)Ph | Et |
| G-3662 | Me | F | CH₂CH₂OC(=O)Ph(4-CF₃) | Et |
| G-3663 | Me | F | CH₂CH₂CH₂OC(=O)Ph(4-CF₃) | Et |
| G-3664 | Me | F | CH₂CH₂CH₂CH₂OC(=O)Ph(4-CF₃) | Et |
| G-3665 | Me | F | CH₂CH₂CH₂CH₂CH₂OC(=O)Ph(4-CF₃) | Et |
| G-3666 | Me | F | CH₂CH₂OS(=O)Me | Et |
| G-3667 | Me | F | CH₂CH₂CH₂OS(=O)Me | Et |
| G-3668 | Me | F | CH₂CH₂CH₂CH₂OS(=O)Me | Et |
| G-3669 | Me | F | CH₂CH₂CH₂CH₂CH₂OS(=O)Me | Et |
| G-3670 | Me | F | CH₂CH₂CH₂CH₂CH₂CH₂OS(=O)Me | Et |
| G-3671 | Me | F | CH₂CH₂OS(=O)CF₃ | Et |
| G-3672 | Me | F | CH₂CH₂CH₂OS(=O)CF₃ | Et |
| G-3673 | Me | F | CH₂CH₂CH₂CH₂OS(=O)CF₃ | Et |
| G-3674 | Me | F | CH₂CH₂CH₂CH₂CH₂OS(=O)CF₃ | Et |
| G-3675 | Me | F | CH₂CH₂CH₂CH₂CH₂CH₂OS(=O)CF₃ | Et |
| G-3676 | Me | F | CH₂CH₂OS(=O)Ph | Et |
| G-3677 | Me | F | CH₂CH₂OS(=O)Ph(4-Me) | Et |
| G-3678 | Me | F | CH₂CH₂CH₂OS(=O)Ph | Et |
| G-3679 | Me | F | CH₂CH₂CH₂OS(=O)Ph(4-Me) | Et |
| G-3680 | Me | F | CH₂CH₂OS(=O)₂Me | Et |
| G-3681 | Me | F | CH₂CH₂CH₂OS(=O)₂Me | Et |
| G-3682 | Me | F | CH₂CH₂CH₂OS(=O)₂CF₃ | Et |
| G-3683 | Me | F | CH₂CH₂CH₂CH₂OS(=O)₂CF₃ | Et |
| G-3684 | Me | F | CH₂CH₂CH₂CH₂CH₂OS(=O)₂CF₃ | Et |
| G-3685 | Me | F | CH₂CH₂CH₂CH₂CH₂OS(=O)₂CF₂CF₂CF₂CF₃ | Et |
| G-3686 | Me | F | CH₂CH₂OS(=O)₂Ph | Et |
| G-3687 | Me | F | CH₂CH₂OS(=O)₂Ph(4-Me) | Et |
| G-3688 | Me | F | CH₂CH₂CH₂OS(=O)₂Ph | Et |
| G-3689 | Me | F | CH₂CH₂CH₂OS(=O)₂Ph(4-Me) | Et |
| G-3690 | Me | F | CH₂CH₂NH₂ | Et |

**[Table 1204]**

| Compound Number | R³ | R⁴ | R⁵ | R¹¹ |
|---|---|---|---|---|
| G-3691 | Me | F | CH₂CH₂NH₂, HCl | Et |
| G-3692 | Me | F | CH₂CH₂CH₂NH₂ | Et |
| G-3693 | Me | F | CH₂CH₂CH₂CH₂NH₂ | Et |
| G-3694 | Me | F | CH₂CH₂CH₂CH₂CH₂NH₂ | Et |
| G-3695 | Me | F | CH₂CH₂CH₂CH₂CH₂CH₂NH₂ | Et |
| G-3696 | Me | F | CH₂CH₂CH₂CH₂CH₂CH₂CH₂NH₂ | Et |
| G-3697 | Me | F | CH₂CH(Me)NH₂ | Et |
| G-3698 | Me | F | CH₂C*H(Me)NH₂, HCl | Et |
| G-3699 | Me | F | CH₂C*H(Me)NH₂: (R) | Et |
| G-3700 | Me | F | CH₂C*H(Me)NH₂, HCl : (R) | Et |
| G-3701 | Me | F | CH₂C*H(Me)NH₂ : (S) | Et |
| G-3702 | Me | F | CH₂C*H(Me)NH₂, HCl : (S) | Et |
| G-3703 | Me | F | CH₂CH(Et)NH₂ | Et |
| G-3704 | Me | F | CH₂CH(*i*-Pr)NH₂ | Et |
| G-3705 | Me | F | CH(Me)CH₂NH₂ | Et |
| G-3706 | Me | F | CH(Me)CH₂NH₂, HCl | Et |
| G-3707 | Me | F | CH(Et)CH₂NH₂ | Et |
| G-3708 | Me | F | CH(*i*-Pr)CH₂NH₂ | Et |
| G-3709 | Me | F | CH₂CH₂CH(Me)NH₂ | Et |
| G-3710 | Me | F | CH₂CH(Me)CH₂NH₂ | Et |
| G-3711 | Me | F | CH(Me)CH₂CH₂NH₂ | Et |
| G-3712 | Me | F | CH₂CH(Me)NH(*c*-Pr) | Et |
| G-3713 | Me | F | CH₂CH(Me)N(*c*-Pr)S(=O)₂CF₃ | Et |
| G-3714 | Me | F | CH₂CH(Me)NHOMe | Et |
| G-3715 | Me | F | CH₂CH(Me)N(OMe)S(=O)₂CF₃ | Et |
| G-3716 | Me | F | CH₂CH(Me)N(OMe)S(=O)₂Ph(2-NO₂) | Et |
| G-3717 | Me | F | CH₂CH(Me)N(OMe)S(=O)₂Ph(4-NO₂) | Et |
| G-3718 | Me | F | CH₂CH₂N(Me)(*t*-Bu) | Et |
| G-3719 | Me | F | CH₂CH₂CH₂N(Me)(*t*-Bu) | Et |
| G-3720 | Me | F | CH₂CH₂CH₂CH₂N(Me)(*t*-Bu) | Et |
| G-3721 | Me | F | CH₂CH₂NHCH₂CF₃ | Et |
| G-3722 | Me | F | CH₂CH₂CH₂NHCH₂CF₃ | Et |
| G-3723 | Me | F | CH₂CH₂CH₂CH₂NHCH₂CF₃ | Et |
| G-3724 | Me | F | CH₂CH₂NHC(=O)C(Me)(CF₃)₂ | Et |
| G-3725 | Me | F | CH₂CH₂NHC(=O)Me | Et |
| G-3726 | Me | F | CH₂CH₂NHC(=O)(t-Bu) | Et |
| G-3727 | Me | F | CH₂CH₂CH₂CH₂NHC(=O)CH(CH₃)₂ | Et |
| G-3728 | Me | F | CH₂CH₂CH₂CH₂NHC(=O)(*t*-Bu) | Et |
| G-3729 | Me | F | CH₂CH₂CH₂CH₂NHC(=O)CH₂(*t*-Bu) | Et |
| G-3730 | Me | F | CH₂CH₂NHC(=O)CF₃ | Et |
| G-3731 | Me | F | CH₂CH₂NHC(=O)CH₂CF₃ | Et |
| G-3732 | Me | F | CH₂CH₂NHC(=O)CF₂CF₃ | Et |
| G-3733 | Me | F | CH₂CH₂CH₂NHC(=O)CF₃ | Et |
| G-3734 | Me | F | CH₂CH₂CH₂NHC(=O)CH₂CF₃ | Et |

**[Table 1205]**

| Compound Number | R³ | R⁴ | R⁵ | R¹¹ |
|---|---|---|---|---|
| G-3735 | Me | F | CH₂CH₂CH₂NHC(=O)CF₂CF₃ | Et |
| G-3736 | Me | F | CH₂CH₂CH₂CH₂NHC(=O)CF₃ | Et |
| G-3737 | Me | F | CH₂CH₂CH₂CH₂NHC(=O)CH₂CF₃ | Et |
| G-3738 | Me | F | CH₂CH₂CH₂CH₂NHC(=O)CF₂CF₃ | Et |
| G-3739 | Me | F | CH₂CH₂CH₂CH₂NHC(=O)CF(CF₃)₂ | Et |
| G-3740 | Me | F | CH₂CH₂CH₂CH₂NHC(=O)CCl₃ | Et |
| G-3741 | Me | F | CH₂CH₂NHC(=O)Ph | Et |
| G-3742 | Me | F | CH₂CH₂NHC(=O)Ph(4-CF₃) | Et |
| G-3743 | Me | F | CH₂CH₂CH₂NHC(=O)Ph(4-CF₃) | Et |
| G-3744 | Me | F | CH₂CH₂CH₂CH₂NHC(=O)Ph(4-CF₃) | Et |
| G-3745 | Me | F | CH₂CH₂NHC(=O)O(*t*-Bu) | Et |
| G-3746 | Me | F | CH₂CH₂CH₂NHC(=O)O(*t*-Bu) | Et |
| G-3747 | Me | F | CH₂CH₂CH₂CH₂NHC(=O)O(*t*-Bu) | Et |
| G-3748 | Me | F | CH₂CH₂CH₂CH₂NHC(=O)OCH(CH₃)₂ | Et |
| G-3749 | Me | F | CH₂CH(Me)NHC(=O)O(*t*-Bu) | Et |
| G-3750 | Me | F | CH₂C*H(Me)NHC(=O)O(*t*-Bu) : (R) | Et |
| G-3751 | Me | F | CH₂C*H(Me)NHC(=O)O(*t*-Bu) : (S) | Et |
| G-3752 | Me | F | CH₂CH(Et)NHC(=O)O(*t*-Bu) | Et |
| G-3753 | Me | F | CH₂CH(*i*-Pr)NHC(=O)O(t-Bu) | Et |
| G-3754 | Me | F | CH(Me)CH₂NHC(=O)O(*t*-Bu) | Et |
| G-3755 | Me | F | CH(Et)CH₂NHC(=O)O(*t*-Bu) | Et |
| G-3756 | Me | F | CH(*i*-Pr)CH₂NHC(=O)O(*t*-Bu) | Et |
| G-3757 | Me | F | CH₂CH(Me)CH₂NHC(=O)O(*t*-Bu) | Et |
| G-3758 | Me | F | CH₂CH₂NHC(=O)OC(Me)₂CF₃ | Et |
| G-3759 | Me | F | CH₂CH₂CH₂NHC(=O)OCH₂CF₃ | Et |
| G-3760 | Me | F | CH₂CH₂CH₂NHC(=O)OCH₂CH₂CF₃ | Et |
| G-3761 | Me | F | CH₂CH₂CH₂CH₂NHC(=O)OCH₂CCl₃ | Et |
| G-3762 | Me | F | CH₂CH₂CH₂CH₂NHC(=O)OCH₂CF₃ | Et |
| G-3763 | Me | F | CH₂CH₂NHC(=O)NH(*t*-Bu) | Et |
| G-3764 | Me | F | CH₂CH₂CH₂NHC(=O)NH(*t*-Bu) | Et |
| G-3765 | Me | F | CH₂CH₂CH₂CH₂NHC(=O)NHEt | Et |
| G-3766 | Me | F | CH₂CH₂CH₂CH₂NHC(=O)NH(*t*-Bu) | Et |
| G-3767 | Me | F | CH₂CH₂CH₂NHC(=O)NHCH₂CCl₃ | Et |
| G-3768 | Me | F | CH₂CH₂CH₂NHC(=O)NHCH₂CH₂F | Et |
| G-3769 | Me | F | CH₂CH₂CH₂NHC(=O)NHCH₂CF₃ | Et |
| G-3770 | Me | F | CH₂CH₂CH₂CH₂NHC(=O)NHCH₂CCl₃ | Et |
| G-3771 | Me | F | CH₂CH₂CH₂CH₂NHC(=O)NHCH₂CH₂F | Et |
| G-3772 | Me | F | CH₂CH₂CH₂CH_{2N}HC(=O)NHCH₂CF₃ | Et |
| G-3773 | Me | F | CH₂CH₂NHS(=O)Me | Et |
| G-3774 | Me | F | CH₂CH₂CH₂NHS(=O)Me | Et |
| G-3775 | Me | F | CH₂CH₂CH₂CH₂NHS(=O)Me | Et |
| G-3776 | Me | F | CH₂CH₂CH₂CH₂NHS(=O)CH(CH₃)₂ | Et |
| G-3777 | Me | F | CH₂CH₂CH₂CH₂CH₂NHS(=O)Me | Et |
| G-3778 | Me | F | CH₂CH₂NHS(=O)CHF₂ | Et |

**[Table 1206]**

| Compound Number | R³ | R⁴ | R⁵ | R¹¹ |
|---|---|---|---|---|
| G-3779 | Me | F | CH₂CH₂CH₂NHS(=O)CHF₂ | Et |
| G-3780 | Me | F | CH₂CH₂CH₂CH₂NHS(=O)CHF₂ | Et |
| G-3781 | Me | F | CH₂CH₂CH₂CH₂CH₂NHS(=O)CHF₂ | Et |
| G-3782 | Me | F | CH₂CH(Me)NHS(=O)CHF₂ | Et |
| G-3783 | Me | F | CH(Me)CH₂NHS(=O)CHF₂ | Et |
| G-3784 | Me | F | CH₂CH₂CH(Me)NHS(=O)CHF₂ | Et |
| G-3785 | Me | F | CH₂CH(Me)CH₂NHS(=O)CHF₂ | Et |
| G-3786 | Me | F | CH(Me)CH₂CH₂NHS(=O)CHF₂ | Et |
| G-3787 | Me | F | CH₂CH₂NHS(=O)CF₃ | Et |
| G-3788 | Me | F | CH₂CH₂CH₂NHS(=O)CF₃ | Et |
| G-3789 | Me | F | CH₂CH₂CH₂CH₂NHS(=O)CF₃ | Et |
| G-3790 | Me | F | CH₂CH₂CH₂CH₂CH₂NHS(=O)CF₃ | Et |
| G-3791 | Me | F | CH₂CH₂CH₂NHS(=O)Ph | Et |
| G-3792 | Me | F | CH₂CH₂CH₂CH₂NHS(=O)Ph | Et |
| G-3793 | Me | F | CH₂CH₂CH₂CH₂CH₂NHS(=O)Ph | Et |
| G-3794 | Me | F | CH₂CH₂CH₂CH₂CH₂NHS(=O)Ph(4-CF₃) | Et |
| G-3795 | Me | F | CH₂CH(Me)NHS(=O)Ph(4-CF₃) | Et |
| G-3796 | Me | F | CH₂CH(Et)NHS(=O)Ph(4-CF₃) | Et |
| G-3797 | Me | F | CH(Me)CH₂NHS(=O)Ph(4-CF₃) | Et |
| G-3798 | Me | F | CH(Et)CH₂NHS(=O)Ph(4-CF₃) | Et |
| G-3799 | Me | F | CH₂CH(Et)CH₂NHS(=O)Ph(4-CF₃) | Et |
| G-3800 | Me | F | CH₂CH₂NHS(=O)₂Me | Et |
| G-3801 | Me | F | CH₂CH₂CH₂NHS(=O)₂Me | Et |
| G-3802 | Me | F | CH₂CH₂CH₂CH₂NHS(=O)₂Me | Et |
| G-3803 | Me | F | CH₂CH₂CH₂CH₂NHS(=O)₂CH(CH₃)₂ | Et |
| G-3804 | Me | F | CH₂CH₂CH₂CH₂CH₂NHS(=O)₂Me | Et |
| G-3805 | Me | F | CH₂CH₂NHS(=O)₂CHF₂ | Et |
| G-3806 | Me | F | CH₂CH₂CH₂NHS(=O)₂CHF₂ | Et |
| G-3807 | Me | F | CH₂CH₂CH₂CH₂NHS(=O)₂CHF₂ | Et |
| G-3808 | Me | F | CH₂CH₂CH₂CH₂CH₂NHS(=O)₂CHF₂ | Et |
| G-3809 | Me | F | CH₂CH(Me)NHS(=O)₂CHF₂ | Et |
| G-3810 | Me | F | CH(Me)CH₂NHS(=O)₂CHF₂ | Et |
| G-3811 | Me | F | CH₂CH₂CH(Me)NHS(=O)₂CHF₂ | Et |
| G-3812 | Me | F | CH₂CH(Me)CH₂NHS(=O)₂CHF₂ | Et |
| G-3813 | Me | F | CH(Me)CH₂CH₂NHS(=O)₂CHF₂ | Et |
| G-3814 | Me | F | CH₂CH(Me)N(Me)S(=O)₂CHF₂ | Et |
| G-3815 | Me | F | CH(Me)CH₂N(Me)S(=O)₂CHF₂ | Et |
| G-3816 | Me | F | CH₂CH₂CH(Me)N(Me)S(=O)₂CHF₂ | Et |
| G-3817 | Me | F | CH₂CH(Me)CH₂N(Me)S(=O)₂CHF₂ | Et |
| G-3818 | Me | F | CH(Me)CH₂CH₂N(Me)S(=O)₂CHF₂ | Et |
| G-3819 | Me | F | CH₂CH₂NHS(=O)₂CF₃ | Et |
| G-3820 | Me | F | CH₂CH₂CH₂NHS(=O)₂CF₃ | Et |
| G-3821 | Me | F | CH₂CH₂CH₂CH₂NHS(=O)₂CF₃ | Et |
| G-3822 | Me | F | CH₂CH₂CH₂CH₂CH₂NHS(=O)₂CF₃ | Et |

**[Table 1207]**

| Compound Number | R³ | R⁴ | R⁵ | R¹¹ |
|---|---|---|---|---|
| G-3823 | Me | F | CH₂CH₂N(Me)S(=O)₂CF₃ | Et |
| G-3824 | Me | F | CH₂CH₂N(Et)S(=O)₂CF₃ | Et |
| G-3825 | Me | F | CH₂CH₂N(CH₂CN)S(=O)₂CF₃ | Et |
| G-3826 | Me | F | CH₂CH₂N(CH₂OMe)S(=O)₂CF₃ | Et |
| G-3827 | Me | F | CH₂CH₂CH₂N(Me)S(=O)₂CF₃ | Et |
| G-3828 | Me | F | CH₂CH₂CH₂N(Et)S(=O)₂CF₃ | Et |
| G-3829 | Me | F | CH₂CH₂CH₂N(CH₂CN)S(=O)₂CF₃ | Et |
| G-3830 | Me | F | CH₂CH₂CH₂N(CH₂OMe)S(=O)₂CF₃ | Et |
| G-3831 | Me | F | CH₂CH₂CH₂CH₂N(Me)S(=O)₂CF₃ | Et |
| G-3832 | Me | F | CH₂CH₂CH₂CH₂N(Et)S(=O)₂CF₃ | Et |
| G-3833 | Me | F | CH₂CH₂CH₂CH₂N(CH₂CN)S(=O)₂CF₃ | Et |
| G-3834 | Me | F | CH₂CH₂CH₂CH₂N(CH₂OMe)S(=O)₂CF₃ | Et |
| G-3835 | Me | F | CH₂CH(Me)NHS(=O)₂CF₃ | Et |
| G-3836 | Me | F | CH₂CH(Me)NHS(=O)₂CF₃ : (R) | Et |
| G-3837 | Me | F | CH₂CH(Me)NHS(=O)₂CF₃ : (S) | Et |
| G-3838 | Me | F | CH(Me)CH₂NHS(=O)₂CF₃ | Et |
| G-3839 | Me | F | CH₂CH₂CH(Me)NHS(=O)₂CF₃ | Et |
| G-3840 | Me | F | CH₂CH(Me)CH₂NHS(=O)₂CF₃ | Et |
| G-3841 | Me | F | CH(Me)CH₂CH₂NHS(=O)₂CF₃ | Et |
| G-3842 | Me | F | CH₂CH(Me)N(Me)S(=O)₂CF₃ | Et |
| G-3843 | Me | F | CH(Me)CH₂N(Me)S(=O)₂CF₃ | Et |
| G-3844 | Me | F | CH₂CH₂CH(Me)N(Me)S(=O)₂CF₃ | Et |
| G-3845 | Me | F | CH₂CH(Me)CH₂N(Me)S(=O)₂CF₃ | Et |
| G-3846 | Me | F | CH(Me)CH₂CH₂N(Me)S(=O)₂CF₃ | Et |
| G-3847 | Me | F | CH₂CH₂CH₂NHS(=O)₂Ph | Et |
| G-3848 | Me | F | CH₂CH₂CH₂CH₂NHS(=O)₂Ph | Et |
| G-3849 | Me | F | CH₂CH₂CH₂CH₂CH₂NHS(=O)₂Ph | Et |
| G-3850 | Me | F | CH₂CH₂CH₂CH₂CH₂NHS(=O)₂Ph(4-CF₃) | Et |
| G-3851 | Me | F | CH₂CH(Me)NHS(=O)₂Ph(4-CF₃) | Et |
| G-3852 | Me | F | CH₂CH(Et)NHS(=O)₂Ph(4-CF₃) | Et |
| G-3853 | Me | F | CH(Me)CH₂NHS(=O)₂Ph(4-CF₃) | Et |
| G-3854 | Me | F | CH(Et)CH₂NHS(=O)₂Ph(4-CF₃) | Et |
| G-3855 | Me | F | CH₂CH(Me)CH₂NHS(=O)₂Ph(4-CF₃) | Et |
| G-3856 | Me | F | CH₂CH₂SiMe₃ | Et |
| G-3857 | Me | F | CH₂CH₂CH₂SiMe₃ | Et |
| G-3858 | Me | F | CH₂CH₂CH₂CH₂SiMe₃ | Et |
| G-3859 | Me | F | CH₂Ph | Et |
| G-3860 | Me | F | CH₂Ph(2-CF₃) | Et |
| G-3861 | Me | F | CH₂Ph(3-CF₃) | Et |
| G-3862 | Me | F | CH₂Ph(4-CF₃) | Et |
| G-3863 | Me | F | CH₂Ph(2-OCF₃) | Et |
| G-3864 | Me | F | CH₂Ph(3-OCF₃) | Et |
| G-3865 | Me | F | CH₂Ph(4-OCF₃) | Et |
| G-3866 | Me | F | CH₂Ph(2-SCF₃) | Et |

**[Table 1208]**

| Compound Number | R³ | R⁴ | R⁵ | R¹¹ |
|---|---|---|---|---|
| G-3867 | Me | F | CH₂Ph(3-SCF₃) | Et |
| G-3868 | Me | F | CH₂Ph(4-SCF₃) | Et |
| G-3869 | Me | F | CH₂Ph(3-CH₂SCF₃) | Et |
| G-3870 | Me | F | CH₂Ph(4-F) | Et |
| G-3871 | Me | F | CH₂Ph(4-Cl) | Et |
| G-3872 | Me | F | CH₂Ph(4-Br) | Et |
| G-3873 | Me | F | CH₂Ph(4-Me) | Et |
| G-3874 | Me | F | CH₂Ph[4-(*t*-Bu)] | Et |
| G-3875 | Me | F | CH₂Ph(4-CN) | Et |
| G-3876 | Me | F | CH₂Ph(4-NO₂) | Et |
| G-3877 | Me | F | CH₂Ph(4-OCHF₂) | Et |
| G-3878 | Me | F | CH₂Ph(4-SCHF₂) | Et |
| G-3879 | Me | F | CH₂Ph(4-CH₂SCF₃) | Et |
| G-3880 | Me | F | CH₂Ph[4-CF(CF₃)₂] | Et |
| G-3881 | Me | F | CH₂Ph(4-CH₂SCF₃) | Et |
| G-3882 | Me | F | CH₂Ph[4-Ph(4-CF₃)] | Et |
| G-3883 | Me | F | CH₂Ph(2,4-Cl₂) | Et |
| G-3884 | Me | F | CH₂Ph[2,5-(CF₃)₂] | Et |
| G-3885 | Me | F | CH₂Ph(3,4-Cl₂) | Et |
| G-3886 | Me | F | CH₂Ph(3-CF₃-4-F) | Et |
| G-3887 | Me | F | CH₂Ph(3-CF₃-4-Cl) | Et |
| G-3888 | Me | F | CH₂Ph(3-F-4-CF₃) | Et |
| G-3889 | Me | F | CH₂Ph(2,4,6-F₃) | Et |
| G-3890 | Me | F | CH₂Ph(3,4,5-F₃) | Et |
| G-3891 | Me | F | CH₂Ph(2,3,4-F₃) | Et |
| G-3892 | Me | F | CH₂Ph(3,4,5-Cl₃) | Et |
| G-3893 | Me | F | CH₂CH₂Ph | Et |
| G-3894 | Me | F | CH₂CH₂Ph(4-F) | Et |
| G-3895 | Me | F | CH₂CH₂Ph(4-Cl) | Et |
| G-3896 | Me | F | CH₂CH₂Ph(4-Br) | Et |
| G-3897 | Me | F | CH₂CH₂Ph[4-(*t*-Bu)] | Et |
| G-3898 | Me | F | CH₂CH₂Ph(2-CF₃) | Et |
| G-3899 | Me | F | CH₂CH₂Ph(3-CF₃) | Et |
| G-3900 | Me | F | CH₂CH₂Ph(4-CF₃) | Et |
| G-3901 | Me | F | CH₂CH₂Ph[4-CF(CF₃)₂] | Et |
| G-3902 | Me | F | CH₂CH₂Ph[4-(*c*-Pr)] | Et |
| G-3903 | Me | F | CH₂CH₂Ph{4-[*c*-Pr(2,2-F₂)]} | Et |
| G-3904 | Me | F | CH₂CH₂Ph(4-OMe) | Et |
| G-3905 | Me | F | CH₂CH₂Ph(3-OCF₃) | Et |
| G-3906 | Me | F | CH₂CH₂Ph(4-OCHF₂) | Et |
| G-3907 | Me | F | CH₂CH₂Ph(4-OCF₃) | Et |
| G-3908 | Me | F | CH₂CH₂Ph(4-SMe) | Et |
| G-3909 | Me | F | CH₂CH₂Ph(4-SCHF₂) | Et |
| G-3910 | Me | F | CH₂CH₂Ph(4-SCF₃) | Et |

**[Table 1209]**

| Compound Number | R³ | R⁴ | R⁵ | R¹¹ |
|---|---|---|---|---|
| G-3911 | Me | F | CH₂CH₂Ph[4-S(=O)Me] | Et |
| G-3912 | Me | F | CH₂CH₂Ph[4-S(=O)CF₃] | Et |
| G-3913 | Me | F | CH₂CH₂Ph[4-S(=O)₂Me] | Et |
| G-3914 | Me | F | CH₂CH₂Ph[4-S(=O)₂CF₃] | Et |
| G-3915 | Me | F | CH₂CH₂Ph(4-CH₂SMe) | Et |
| G-3916 | Me | F | CH₂CH₂Ph(4-CH₂SCF₃) | Et |
| G-3917 | Me | F | CH₂CH₂Ph[4-OS(=O)₂Me] | Et |
| G-3918 | Me | F | CH₂CH₂Ph[4-OS(=O)₂CF₃] | Et |
| G-3919 | Me | F | CH₂CH₂Ph[4-Ph(4-CF₃)] | Et |
| G-3920 | Me | F | CH₂CH₂Ph(4-CH₂Ph) | Et |
| G-3921 | Me | F | CH₂CH₂Ph(4-OCH₂Ph) | Et |
| G-3922 | Me | F | CH₂CH₂Ph(4-CN) | Et |
| G-3923 | Me | F | CH₂CH₂Ph(4-NO₂) | Et |
| G-3924 | Me | F | CH₂CH₂Ph(2,4-Cl₂) | Et |
| G-3925 | Me | F | CH₂CH₂Ph(3,4-Cl₂) | Et |
| G-3926 | Me | F | CH₂CH₂Ph(3-CF₃-4-F) | Et |
| G-3927 | Me | F | CH₂CH₂Ph(2-CF₃-4-F) | Et |
| G-3928 | Me | F | CH₂CH₂Ph(3-F-4-CF₃) | Et |
| G-3929 | Me | F | CH₂CH₂Ph(2-F-4-CF₃) | Et |
| G-3930 | Me | F | CH₂CH₂Ph(3-Cl-4-OCHF₂) | Et |
| G-3931 | Me | F | CH₂CH₂Ph(3,4,5-Cl₃) | Et |
| G-3932 | Me | F | CH₂CH₂Ph(2,3,4-F₃) | Et |
| G-3933 | Me | F | CH₂CH₂Ph(2,4,5-F₃) | Et |
| G-3934 | Me | F | CH₂CH₂Ph(3,4,5-F₃) | Et |
| G-3935 | Me | F | CH₂CH₂Ph(2,4,6-F₃) | Et |
| G-3936 | Me | F | CH₂CH₂CH₂Ph | Et |
| G-3937 | Me | F | CH₂CH₂CH₂Ph(3-CF₃) | Et |
| G-3938 | Me | F | CH₂CH₂CH₂Ph(4-CF₃) | Et |
| G-3939 | Me | F | CH₂CH₂CH₂Ph(4-F) | Et |
| G-3940 | Me | F | CH₂CH₂CH₂Ph[4-(*t*-Bu)] | Et |
| G-3941 | Me | F | CH₂CH₂CH₂Ph(4-CN) | Et |
| G-3942 | Me | F | CH₂CH₂CH₂Ph(4-CF₃) | Et |
| G-3943 | Me | F | CH₂CH₂CH₂Ph(4-OCHF₂) | Et |
| G-3944 | Me | F | CH₂CH₂CH₂Ph(4-OCF₃) | Et |
| G-3945 | Me | F | CH₂CH₂CH₂Ph(4-SCHF₂) | Et |
| G-3946 | Me | F | CH₂CH₂CH₂Ph(4-SCF₃) | Et |
| G-3947 | Me | F | CH₂CH₂CH₂Ph[4-CF(CF₃)₂] | Et |
| G-3948 | Me | F | CH₂CH₂CH₂Ph(3,4,5-F₃) | Et |
| G-3949 | Me | F | CH₂CH₂CH₂Ph(2,4,6-F₃) | Et |
| G-3950 | Me | F | CH₂CH₂CH₂CH₂Ph | Et |
| G-3951 | Me | F | CH₂CH₂CH₂CH₂Ph(4-F) | Et |
| G-3952 | Me | F | CH₂CH₂CH₂CH₂Ph(4-CF₃) | Et |
| G-3953 | Me | F | CH₂CH₂CH₂CH₂Ph(4-OCF₃) | Et |
| G-3954 | Me | F | CH₂CH₂CH₂CH₂Ph(4-SCF₃) | Et |

**[Table 1210]**

| Compound Number | R³ | R⁴ | R⁵ | R¹¹ |
|---|---|---|---|---|
| G-3955 | Me | F | CH₂CH₂CH₂CH₂CH₂Ph | Et |
| G-3956 | Me | F | CH₂CH₂CH₂CH₂CH₂CH₂Ph | Et |
| G-3957 | Me | F | CH₂CF₂Ph(4-F) | Et |
| G-3958 | Me | F | CH₂CF₂Ph(4-CF₃) | Et |
| G-3959 | Me | F | CH₂CF₂Ph(3,4,5-F₃) | Et |
| G-3960 | Me | F | CH₂CH₂OPh | Et |
| G-3961 | Me | F | CH₂CH₂OPh(4-F) | Et |
| G-3962 | Me | F | CH₂CH₂OPh(4-CF₃) | Et |
| G-3963 | Me | F | CH₂CH₂CH₂OPh | Et |
| G-3964 | Me | F | CH₂CH₂CH₂OPh(4-Cl) | Et |
| G-3965 | Me | F | CH₂CH₂CH₂OPh(4-CF₃) | Et |
| G-3966 | Me | F | CH₂CH₂CH₂CH₂OPh(4-CF₃) | Et |
| G-3967 | Me | F | CH₂CH₂CH₂CH₂OPh(4-OCF₃) | Et |
| G-3968 | Me | F | CH₂CH₂CH₂CH₂CH₂OPh(4-CF₃) | Et |
| G-3969 | Me | F | CH₂CH₂OCH₂Ph | Et |
| G-3970 | Me | F | CH₂CH₂CH₂OCH₂Ph | Et |
| G-3971 | Me | F | CH₂CH₂CH₂CH₂OCH₂Ph | Et |
| G-3972 | Me | F | CH₂CH₂CH₂CH₂CH₂OCH₂Ph | Et |
| G-3973 | Me | F | CH₂CH₂SPh | Et |
| G-3974 | Me | F | CH₂CH₂CH₂SPh | Et |
| G-3975 | Me | F | CH₂CH₂CH₂SPh(4-F) | Et |
| G-3976 | Me | F | CH₂CH₂CH₂SPh(4-Cl) | Et |
| G-3977 | Me | F | CH₂CH₂CH₂SPh(4-Br) | Et |
| G-3978 | Me | F | CH₂CH₂CH₂SPh[4-(*t*-Bu)] | Et |
| G-3979 | Me | F | CH₂CH₂CH₂SPh(3-CF₃) | Et |
| G-3980 | Me | F | CH₂CH₂CH₂SPh(4-CF₃) | Et |
| G-3981 | Me | F | CH₂CH₂CH₂SPh(3-SCF₃) | Et |
| G-3982 | Me | F | CH₂CH₂CH₂SPh(4-SCF₃) | Et |
| G-3983 | Me | F | CH₂CH₂CH₂CH₂SPh | Et |
| G-3984 | Me | F | CH₂CH₂CH₂CH₂SPh(4-F) | Et |
| G-3985 | Me | F | CH₂CH₂CH₂CH₂SPh(4-Cl) | Et |
| G-3986 | Me | F | CH₂CH₂CH₂CH₂SPh[4-(*t*-Bu)] | Et |
| G-3987 | Me | F | CH₂CH₂CH₂CH₂SPh(4-CF₃) | Et |
| G-3988 | Me | F | CH₂CH₂CH₂CH₂CH₂SPh | Et |
| G-3989 | Me | F | CH₂CH₂CH₂CH₂CH₂SPh(4-F) | Et |
| G-3990 | Me | F | CH₂CH₂CH₂CH₂CH₂SPh(4-Cl) | Et |
| G-3991 | Me | F | CH₂CH₂CH₂CH₂CH₂SPh(4-CF₃) | Et |
| G-3992 | Me | F | CH₂CH₂S(=O)Ph | Et |
| G-3993 | Me | F | CH₂CH₂CH₂S(=O)Ph | Et |
| G-3994 | Me | F | CH₂CH₂CH₂S(=O)Ph(4-F) | Et |
| G-3995 | Me | F | CH₂CH₂CH₂S(=O)Ph[4-(*t*-Bu)] | Et |
| G-3996 | Me | F | CH₂CH₂CH₂S(=O)Ph(4-CF₃) | Et |
| G-3997 | Me | F | CH₂CH₂S(=O)₂Ph | Et |
| G-3998 | Me | F | CH₂CH₂S(=O)₂Ph(4-CF₃) | Et |

**[Table 1211]**

| Compound Number | R³ | R⁴ | R⁵ | R¹¹ |
|---|---|---|---|---|
| G-3999 | Me | F | CH₂CH₂CH₂S(=O)₂Ph(4-F) | Et |
| G-4000 | Me | F | CH₂CH₂CH₂S(=O)₂Ph(4-Cl) | Et |
| G-4001 | Me | F | CH₂CH₂CH₂S(=O)₂Ph(4-CF₃) | Et |
| G-4002 | Me | F | CH₂CH₂CH₂CH₂S(=O)₂Ph | Et |
| G-4003 | Me | F | CH₂CH₂CH₂CH₂S(=O)₂Ph(4-Cl) | Et |
| G-4004 | Me | F | CH₂CH₂CH₂CH₂S(=O)₂Ph(4-CF₃) | Et |
| G-4005 | Me | F | CH₂CH₂SCH₂Ph | Et |
| G-4006 | Me | F | CH₂CH₂CH₂SCH₂Ph | Et |
| G-4007 | Me | F | CH₂CH₂CH₂SCH₂Ph(2-Cl) | Et |
| G-4008 | Me | F | CH₂CH₂CH₂SCH₂Ph(3-Cl) | Et |
| G-4009 | Me | F | CH₂CH₂CH₂SCH₂Ph(4-Cl) | Et |
| G-4010 | Me | F | CH₂CH₂CH₂SCH₂Ph(2-SCF₃) | Et |
| G-4011 | Me | F | CH₂CH₂CH₂SCH₂Ph(3-CF₃) | Et |
| G-4012 | Me | F | CH₂CH₂CH₂SCH₂Ph(4-CF₃) | Et |
| G-4013 | Me | F | CH₂CH₂CH₂SCH₂Ph(4-NO₂) | Et |
| G-4014 | Me | F | CH₂CH₂CH₂CH₂SCH₂Ph | Et |
| G-4015 | Me | F | CH₂CH₂CH₂CH₂SCH₂Ph(4-Cl) | Et |
| G-4016 | Me | F | CH₂CH₂CH₂CH₂SCH₂Ph(4-CF₃) | Et |
| G-4017 | Me | F | CH₂CH₂CH₂CH₂SCH₂Ph(4-CN) | Et |
| G-4018 | Me | F | CH₂CH₂CH₂CH₂CH₂SCH₂Ph | Et |
| G-4019 | Me | F | CH₂CH₂SCH₂CH₂Ph | Et |
| G-4020 | Me | F | CH₂CH₂CH₂SCH₂CH₂Ph | Et |
| G-4021 | Me | F | CH₂CH₂CH₂CH₂SCH₂CH₂Ph | Et |
| G-4022 | Me | F | CH₂CH₂SCH(Me)Ph | Et |
| G-4023 | Me | F | CH₂CH₂CH₂SCH(Me)Ph | Et |
| G-4024 | Me | F | CH₂CH₂CH₂CH₂SCH(Me)Ph | Et |
| G-4025 | Me | F | CH₂CH₂S(=O)CH₂Ph | Et |
| G-4026 | Me | F | CH₂CH₂S(=O)CH₂Ph(4-CF₃) | Et |
| G-4027 | Me | F | CH₂CH₂CH₂S(=O)CH₂Ph | Et |
| G-4028 | Me | F | CH₂CH₂CH₂S(=O)CH₂Ph(2-SCF₃) | Et |
| G-4029 | Me | F | CH₂CH₂CH₂S(=O)CH₂Ph(4-SCF₃) | Et |
| G-4030 | Me | F | CH₂CH₂CH₂S(=O)CH₂Ph(4-Cl) | Et |
| G-4031 | Me | F | CH₂CH₂CH₂S(=O)CH₂Ph(4-CF₃) | Et |
| G-4032 | Me | F | CH₂CH₂CH₂CH₂S(=O)CH₂Ph | Et |
| G-4033 | Me | F | CH₂CH₂CH₂CH₂S(=O)CH₂Ph(4-CF₃) | Et |
| G-4034 | Me | F | CH₂CH₂CH₂CH₂CH₂S(=O)CH₂Ph(4-CF₃) | Et |
| G-4035 | Me | F | CH₂CH₂S(=O)₂CH₂Ph | Et |
| G-4036 | Me | F | CH₂CH₂S(=O)₂CH₂Ph(4-CF₃) | Et |
| G-4037 | Me | F | CH₂CH₂CH₂S(=O)₂CH₂Ph | Et |
| G-4038 | Me | F | CH₂CH₂CH₂S(=O)₂CH₂Ph(4-Cl) | Et |
| G-4039 | Me | F | CH₂CH₂CH₂S(=O)₂CH₂Ph(4-CF₃) | Et |
| G-4040 | Me | F | CH₂CH₂CH₂CH₂S(=O)₂CH₂Ph | Et |
| G-4041 | Me | F | CH₂CH₂CH₂CH₂S(=O)₂CH₂Ph(4-CF₃) | Et |
| G-4042 | Me | F | CH₂CH₂CH₂CH₂CH₂S(=O)₂CH₂Ph | Et |

**[Table 1212]**

| Compound Number | R³ | R⁴ | R⁵ | R¹¹ |
|---|---|---|---|---|
| G-4043 | Me | F | CH₂CH₂CH₂CH₂CH₂S(=O)₂CH₂Ph(4-CF₃) | Et |
| G-4044 | Me | F | CH₂CH₂ON=CH(*t*-Bu) | Et |
| G-4045 | Me | F | CH₂CH₂CH₂ON=CH(*t*-Bu) | Et |
| G-4046 | Me | F | CH₂CH₂CH₂CH₂ON=C(Me)(*c*-Pr) | Et |
| G-4047 | Me | F | CH₂CH₂ON=C(Me)CF₃ | Et |
| G-4048 | Me | F | CH₂CH₂CH₂ON=CHCF₃ | Et |
| G-4049 | Me | F | CH₂CH₂CH₂ON=C(Me)CF₃ | Et |
| G-4050 | Me | F | CH₂CH₂CH₂ON=C(Me)CCl₃ | Et |
| G-4051 | Me | F | CH₂CH₂CH₂CH₂ON=CHCF₃ | Et |
| G-4052 | Me | F | CH₂CH₂CH₂CH₂ON=C(Me)CF₃ | Et |
| G-4053 | Me | F | CH₂CH₂CH₂CH₂CH₂ON=C(Me)CF₃ | Et |
| G-4054 | Me | F | CH₂CH₂ON=CHPh | Et |
| G-4055 | Me | F | CH₂CH₂ON=CHPh(4-CF₃) | Et |
| G-4056 | Me | F | CH₂CH₂ON=CHPh(4-SCF₃) | Et |
| G-4057 | Me | F | CH₂CH₂CH₂ON=CHPh(3-CF₃) | Et |
| G-4058 | Me | F | CH₂CH₂CH₂ON=CHPh(4-CF₃) | Et |
| G-4059 | Me | F | CH₂CH₂CH₂ON=CHPh(4-SCF₃) | Et |
| G-4060 | Me | F | CH₂CH₂CH₂ON=C(Me)Ph(4-CF₃) | Et |
| G-4061 | Me | F | CH₂CH₂CH₂CH₂ON=CHPh(4-SCF₃) | Et |
| G-4062 | Me | F | CH₂CH₂(adamant-1-yl) | Et |
| G-4063 | Me | F | CH₂CH₂CH₂(3-CF₃-pyrazol-1-yl) | Et |
| G-4064 | Me | F | CH₂CH₂CH₂CH₂(3-CF₃-pyrazol-1-yl) | Et |
| G-4065 | Me | F | CH₂CH₂CH₂CH₂CH₂(3-CF₃-pyrazol-1-yl) | Et |
| G-4066 | Me | F | CH₂(1-Me-4-CF₃-imidazol-5-yl) | Et |
| G-4067 | Me | F | CH₂CH₂(1-Me-4-CF₃-imidazol-5-yl) | Et |
| G-4068 | Me | F | CH₂CH₂CH₂(1-Me-4-CF₃-imidazol-5-yl) | Et |
| G-4069 | Me | F | CH₂CH₂CH₂(3-CF₃-1,2,4-triazol-1-yl) | Et |
| G-4070 | Me | F | CH₂CH₂CH₂CH₂(3-CF₃-1,2,4-triazol-1-yl) | Et |
| G-4071 | Me | F | CH₂CH₂CH₂CH₂CH₂(3-CF₃-1,2,4-triazol-1-yl) | Et |
| G-4072 | Me | F | CH₂CH₂CH₂(4-CF₃-thiazol-2-yl) | Et |
| G-4073 | Me | F | CH₂CH₂CH₂CH₂(4-CF₃-thiazol-2-yl) | Et |
| G-4074 | Me | F | CH₂CH₂CH₂CH₂CH₂(4-CF₃-thiazol-2-yl) | Et |
| G-4075 | Me | F | CH₂CH₂CH₂(2-CF₃-pyridin-5-yl) | Et |
| G-4076 | Me | F | CH₂CH₂CH₂CH₂(2-CF₃-pyridin-5-yl) | Et |
| G-4077 | Me | F | CH₂CH₂CH₂CH₂CH₂(2-CF₃-pyridin-5-yl) | Et |
| G-4078 | Me | F | CH₂(4-CF₃-pyrimidin-2-yl) | Et |
| G-4079 | Me | F | CH₂CH₂(4-CF₃-pyrimidin-2-yl) | Et |
| G-4080 | Me | F | CH₂CH₂CH₂(4-CF₃-pyrimidin-2-yl) | Et |
| G-4081 | Me | F | CH₂CH₂O(3-Cl-5-CF₃-pyridin-2-yl) | Et |
| G-4082 | Me | F | CH₂CH₂CH₂O(3-Cl-5-CF₃-pyridin-2-yl) | Et |
| G-4083 | Me | F | CH₂CH₂CH₂CH₂O(3-Cl-5-CF₃-pyridin-2-yl) | Et |
| G-4084 | Me | F | CH₂CH₂CH₂CH₂CH₂O(3- Cl -5-CF₃-pyridin-2-yl) | Et |
| G-4085 | Me | F | CH₂CH₂N(Phth) | Et |
| G-4086 | Me | F | CH₂CH₂CH₂N(Phth) | Et |

**[Table 1213]**

| Compound Number | R³ | R⁴ | R⁵ | R¹¹ |
|---|---|---|---|---|
| G-4087 | Me | F | CH₂CH₂CH₂CH₂N(Phth) | Et |
| G-4088 | Me | F | CH₂CH₂CH₂CH₂CH₂N(Phth) | Et |
| G-4089 | Me | F | CH₂CH(Me)CH₂N(Phth) | Et |
| G-4090 | Me | F | CH₂[1,3-dioxolan(2-CF₃)-2-yl] | Et |
| G-4091 | Me | F | CH₂(azetidin-3-yl) | Et |
| G-4092 | Me | F | CH₂(pyrrolidin-3-yl) | Et |
| G-4093 | Me | F | CH₂(piperidin-3-yl) | Et |
| G-4094 | Me | F | CH₂{azeti din[1-C(=O)O(*t*-Bu)]-3-yl} | Et |
| G-4095 | Me | F | CH₂{pyrrolidin[1-C(=O)O(*t*-Bu)]-3-yl} | Et |
| G-4096 | Me | F | CH₂{piperidin[1-C(=O)O(*t*-Bu)]-3-yl} | Et |
| G-4097 | Me | F | CH₂{azetidin[1-C(=O)CF₃]-3-yl} | Et |
| G-4098 | Me | F | CH₂{azetidin[1-S(=O)₂CF₃]-3-yl} | Et |
| G-4099 | Me | F | CH₂{pyrrolidin[1-C(=O)CF₃]-3-yl} | Et |
| G-4100 | Me | F | CH₂{pyrrolidin[1-S(=O)₂CF₃]-3-yl} | Et |
| G-4101 | Me | F | CH₂{piperidin[1-C(=O)CF₃]-3-yl} | Et |
| G-4102 | Me | F | CH₂{piperidin[1-S(=O)₂CF₃]-3-yl} | Et |
| G-4103 | Me | F | CH₂CH₂CH₂CN | Et |
| G-4104 | Me | F | CH₂CH₂CH₂CH₂CN | Et |
| G-4105 | Me | F | CH₂CH₂CH₂CH₂CH₂CN | Et |
| G-4106 | Me | F | CH₂CH₂CH₂CH₂CH₂CH₂CN | Et |
| G-4107 | Me | F | CH₂CH₂CH₂C(=O)OH | Et |
| G-4108 | Me | F | CH₂CH₂CH₂CH₂C(=O)OH | Et |
| G-4109 | Me | F | CH₂CH₂CH₂CH₂CH₂C(=O)OH | Et |
| G-4110 | Me | F | CH₂CH₂SCN | Et |
| G-4111 | Me | F | CH₂CH₂CH₂SCN | Et |
| G-4112 | Me | F | CH₂CH₂CH₂CH₂SCN | Et |
| G-4113 | Me | F | CH₂CH₂CH₂CH₂CH₂SCN | Et |
| G-4114 | Me | F | CH₂CH₂CH₂CH₂CH₂CH₂SCN | Et |
| G-4115 | Me | F | CH₂CH₂CH₂CH₂CH₂CH₂CH₂SCN | Et |
| G-4116 | Me | F | CH₂CH₂CH₂CH₂CH₂CH₂CH₂CH₂SCN | Et |
| G-4117 | Me | F | CH₂CH₂CH(Me)SCN | Et |
| G-4118 | Me | F | CH₂CH(Me)CH₂SCN | Et |
| G-4119 | Me | F | CH(Me)CH₂CH₂SCN | Et |
| G-4120 | Me | F | CH₂CH₂CH₂CH(Me)SCN | Et |
| G-4121 | Me | F | CH₂CH₂CH(Me)CH₂SCN | Et |
| G-4122 | Me | F | CH₂CH(Me)CH₂CH₂SCN | Et |
| G-4123 | Me | F | CH(Me)CH₂CH₂CH₂SCN | Et |
| G-4124 | Me | F | CH₂CH₂CH₂CH₂CH(Me)SCN | Et |
| G-4125 | Me | F | CH₂CH₂CH₂CH(Me)CH₂SCN | Et |
| G-4126 | Me | F | CH₂CH₂CH(Me)CH₂CH₂SCN | Et |
| G-4127 | Me | F | CH₂CH(Me)CH₂CH₂CH₂SCN | Et |
| G-4128 | Me | F | CH(Me)CH₂CH₂CH₂CH₂SCN | Et |
| G-4129 | Me | F | CH₂CH₂ONH₂ | Et |
| G-4130 | Me | F | CH₂CH₂CH₂ONH₂ | Et |

**[Table 1214]**

| Compound Number | R³ | R⁴ | R⁵ | R11 |
|---|---|---|---|---|
| G-4131 | Me | F | CH₂CH₂CH₂CH₂ONH₂ | Et |
| G-4132 | Me | F | CH₂CH₂CH₂CH₂CH₂ONH₂ | Et |
| G-4133 | Me | F | CH₂CH₂CH₂CH₂CH₂CH₂ONH₂ | Et |
| G-4134 | Cl | H | CH₂C(=O)Me | Et |
| G-4135 | Cl | H | CH₂CH(OH)Me | Et |
| G-4136 | Cl | H | CH₂CH(OH)CF₃ | Et |
| G-4137 | Cl | H | CH₂C(OH)₂CF₃ | Et |
| G-4138 | Cl | H | CH₂C(CF₃)=NOH | Et |
| G-4139 | Cl | H | CH₂C(CF₃)=NOMe | Et |
| G-4140 | Cl | H | CH₂CH(CF₃)NH₂ | Et |
| G-4141 | Cl | H | CH₂CH(CF₃)NHSO₂CF₃ | Et |
| G-4142 | Cl | H | CH₂[*c*-Pr(1-NH₂)] | Et |
| G-4143 | Cl | H | CH₂[*c*-Pr(1-NHC(=O)O(*t*-Bu)] | Et |
| G-4144 | Cl | H | CH₂{*c*-Pr[1-NHS(=O)₂CF₃]} | Et |
| G-4145 | Cl | H | CH₂CH₂NH₂ | Et |
| G-4146 | Cl | H | CH₂CH₂NH₂, HCl | Et |
| G-4147 | Cl | H | CH₂CH₂CH₂NH₂ | Et |
| G-4148 | Cl | H | CH₂CH₂CH₂CH₂NH₂ | Et |
| G-4149 | Cl | H | CH₂CH₂CH₂CH₂CH₂NH₂ | Et |
| G-4150 | Cl | H | CH₂CH₂CH₂CH₂CH₂CH₂NH₂ | Et |
| G-4151 | Cl | H | CH₂CH₂CH₂CH₂CH₂CH₂CH₂NH₂ | Et |
| G-4152 | Cl | H | CH₂CH(Me)NH₂ | Et |
| G-4153 | Cl | H | CH₂C*H(Me)NH₂, HCl | Et |
| G-4154 | Cl | H | CH₂C*H(Me)NH₂: (R) | Et |
| G-4155 | Cl | H | CH₂C*H(Me)NH₂, HCl : (R) | Et |
| G-4156 | Cl | H | CH₂C*H(Me)NH₂: (S) | Et |
| G-4157 | Cl | H | CH₂C*H(Me)NH₂, HCl : (S) | Et |
| G-4158 | Cl | H | CH₂CH(Et)NH₂ | Et |
| G-4159 | Cl | H | CH₂CH(*i*-Pr)NH₂ | Et |
| G-4160 | Cl | H | CH(Me)CH₂NH₂ | Et |
| G-4161 | Cl | H | CH(Me)CH₂NH₂, HCl | Et |
| G-4162 | Cl | H | CH(Et)CH₂NH₂ | Et |
| G-4163 | Cl | H | CH(*i*-Pr)CH₂NH₂ | Et |
| G-4164 | Cl | H | CH₂CH₂CH(Me)NH₂ | Et |
| G-4165 | Cl | H | CH₂CH(Me)CH₂NH₂ | Et |
| G-4166 | Cl | H | CH(Me)CH₂CH₂NH₂ | Et |
| G-4167 | Cl | H | CH₂CH(Me)NH(*c*-Pr) | Et |
| G-4168 | Cl | H | CH₂CH(Me)N(*c*-Pr)S(=O)₂CF₃ | Et |
| G-4169 | Cl | H | CH₂CH(Me)NHOMe | Et |
| G-4170 | Cl | H | CH₂CH(Me)N(OMe)S(=O)₂CF₃ | Et |
| G-4171 | Cl | H | CH₂CH(Me)N(OMe)S(=O)₂Ph(2-NO₂) | Et |
| G-4172 | Cl | H | CH₂CH(Me)N(OMe)S(=O)₂Ph(4-NO₂) | Et |
| G-4173 | Cl | H | CH₂CH₂NHC(=O)O(*t*-Bu) | Et |
| G-4174 | Cl | H | CH₂CH₂CH₂NHC(=O)O(*t*-Bu) | Et |

**[Table 1215]**

| Compound Number | R³ | R⁴ | R⁵ | R¹¹ |
|---|---|---|---|---|
| G-4175 | Cl | H | CH₂CH₂CH₂CH₂NHC(=O)O(*t*-Bu) | Et |
| G-4176 | Cl | H | CH₂CH₂CH₂CH₂NHC(=O)OCH(CH₃)₂ | Et |
| G-4177 | Cl | H | CH₂CH(Me)NHC(=O)O(*t*-Bu) | Et |
| G-4178 | Cl | H | CH₂C*H(Me)NHC(=O)O(*t*-Bu) : (R) | Et |
| G-4179 | Cl | H | CH₂C*H(Me)NHC(=O)O(*t*-Bu) : (S) | Et |
| G-4180 | Cl | H | CH₂CH(Et)NHC(=O)O(*t*-Bu) | Et |
| G-4181 | Cl | H | CH₂CH(*i*-Pr)NHC(=O)O(*t*-Bu) | Et |
| G-4182 | Cl | H | CH(Me)CH₂NHC(=O)O(*t*-Bu) | Et |
| G-4183 | Cl | H | CH(Et)CH₂NHC(=O)O(*t*-Bu) | Et |
| G-4184 | Cl | H | CH(*i*-Pr)CH₂NHC(=O)O(*t*-Bu) | Et |
| G-4185 | Cl | H | CH₂CH(Me)CH₂NHC(=O)O(*t*-Bu) | Et |
| G-4186 | Cl | H | CH₂CH₂SCHF₂ | Et |
| G-4187 | Cl | H | CH₂CH₂CH₂SCHF₂ | Et |
| G-4188 | Cl | H | CH₂CH₂CH₂CH₂SCHF₂ | Et |
| G-4189 | Cl | H | CH₂CH₂CH₂CH₂CH₂SCHF₂ | Et |
| G-4190 | Cl | H | CH₂CH₂CH₂CH₂CH₂CH₂SCHF₂ | Et |
| G-4191 | Cl | H | CH₂CH₂CH₂CH₂CH₂CH₂CH₂SCHF₂ | Et |
| G-4192 | Cl | H | CH₂CH₂SCF₃ | Et |
| G-4193 | Cl | H | CH₂CH₂CH₂SCF₃ | Et |
| G-4194 | Cl | H | CH₂CH₂CH₂CH₂SCF₃ | Et |
| G-4195 | Cl | H | CH₂CH₂CH₂CH₂CH₂SCF₃ | Et |
| G-4196 | Cl | H | CH₂CH₂CH₂CH₂CH₂CH₂SCF₃ | Et |
| G-4197 | Cl | H | CH₂CH₂CH₂CH₂CH₂CH₂CH₂SCF₃ | Et |
| G-4198 | Cl | H | CH₂CH₂CH₂CH₂CH₂CH₂CH₂CH₂SCF₃ | Et |
| G-4199 | Cl | H | CH₂CH(Me)SCF₃ | Et |
| G-4200 | Cl | H | CH(Me)CH₂SCF₃ | Et |
| G-4201 | Cl | H | CH₂CH₂CH(Me)SCF₃ | Et |
| G-4202 | Cl | H | CH₂CH(Me)CH₂SCF₃ | Et |
| G-4203 | Cl | H | CH(Me)CH₂CH₂SCF₃ | Et |
| G-4204 | Cl | H | CH₂CH₂CH₂CH(Me)SCF₃ | Et |
| G-4205 | Cl | H | CH₂CH₂CH(Me)CH₂SCF₃ | Et |
| G-4206 | Cl | H | CH₂CH(Me)CH₂CH₂SCF₃ | Et |
| G-4207 | Cl | H | CH(Me)CH₂CH₂CH₂SCF₃ | Et |
| G-4208 | Cl | H | CH₂CH₂NHS(=O)₂CF₃ | Et |
| G-4209 | Cl | H | CH₂CH₂CH₂NHS(=O)₂CF₃ | Et |
| G-4210 | Cl | H | CH₂CH₂CH₂CH₂NHS(=O)₂CF₃ | Et |
| G-4211 | Cl | H | CH₂CH₂CH₂CH₂CH₂NHS(=O)₂CF₃ | Et |
| G-4212 | Cl | H | CH₂CH₂N(Me)S(=O)₂CF₃ | Et |
| G-4213 | Cl | H | CH₂CH₂N(Et)S(=O)₂CF₃ | Et |
| G-4214 | Cl | H | CH₂CH₂N(CH₂CN)S(=O)₂CF₃ | Et |
| G-4215 | Cl | H | CH₂CH₂N(CH₂OMe)S(=O)₂CF₃ | Et |
| G-4216 | Cl | H | CH₂CH₂CH₂N(Me)S(=O)₂CF₃ | Et |
| G-4217 | Cl | H | CH₂CH₂CH₂N(Et)S(=O)₂CF₃ | Et |
| G-4218 | Cl | H | CH₂CH₂CH₂N(CH₂CN)S(=O)₂CF₃ | Et |

**[Table 1216]**

| Compound Number | R³ | R⁴ | R⁵ | R¹¹ |
|---|---|---|---|---|
| G-4219 | Cl | H | CH₂CH₂CH₂N(CH₂OMe)S(=O)₂CF₃ | Et |
| G-4220 | Cl | H | CH₂CH₂CH₂CH₂N(Me)S(=O)₂CF₃ | Et |
| G-4221 | Cl | H | CH₂CH₂CH₂CH₂N(Et)S(=O)₂CF₃ | Et |
| G-4222 | Cl | H | CH₂CH₂CH₂CH₂N(CH₂CN)S(=O)₂CF₃ | Et |
| G-4223 | Cl | H | CH₂CH₂CH₂CH₂N(CH₂OMe)S(=O)₂CF₃ | Et |
| G-4224 | Cl | H | CH₂CH(Me)NHS(=O)₂CF₃ | Et |
| G-4225 | Cl | H | CH₂CH(Me)NHS(=O)₂CF₃: (R) | Et |
| G-4226 | Cl | H | CH₂CH(Me)NHS(=O)₂CF₃ : (S) | Et |
| G-4227 | Cl | H | CH(Me)CH₂NHS(=O)₂CF₃ | Et |
| G-4228 | Cl | H | CH₂CH₂CH(Me)NHS(=O)₂CF₃ | Et |
| G-4229 | Cl | H | CH₂CH(Me)CH₂NHS(=O)₂CF₃ | Et |
| G-4230 | Cl | H | CH(Me)CH₂CH₂NHS(=O)₂CF₃ | Et |
| G-4231 | Cl | H | CH₂CH(Me)N(Me)S(=O)₂CF₃ | Et |
| G-4232 | Cl | H | CH(Me)CH₂N(Me)S(=O)₂CF₃ | Et |
| G-4233 | Cl | H | CH₂CH₂CH(Me)N(Me)S(=O)₂CF₃ | Et |
| G-4234 | Cl | H | CH₂CH(Me)CH₂N(Me)S(=O)₂CF₃ | Et |
| G-4235 | Cl | H | CH(Me)CH₂CH₂N(Me)S(=O)₂CF₃ | Et |
| G-4236 | Cl | H | CH₂Ph | Et |
| G-4237 | Cl | H | CH₂Ph(2-CF₃) | Et |
| G-4238 | Cl | H | CH₂Ph(3-CF₃) | Et |
| G-4239 | Cl | H | CH₂Ph(4-CF₃) | Et |
| G-4240 | Cl | H | CH₂Ph(2-OCF₃) | Et |
| G-4241 | Cl | H | CH₂Ph(3-OCF₃) | Et |
| G-4242 | Cl | H | CH₂Ph(4-OCF₃) | Et |
| G-4243 | Cl | H | CH₂Ph(2-SCF₃) | Et |
| G-4244 | Cl | H | CH₂Ph(3-SCF₃) | Et |
| G-4245 | Cl | H | CH₂Ph(4-SCF₃) | Et |
| G-4246 | Cl | H | CH₂Ph(3-CH₂SCF₃) | Et |
| G-4247 | Cl | H | CH₂Ph(4-F) | Et |
| G-4248 | Cl | H | CH₂Ph(4-Cl) | Et |
| G-4249 | Cl | H | CH₂Ph(4-Br) | Et |
| G-4250 | Cl | H | CH₂Ph(4-Me) | Et |
| G-4251 | Cl | H | CH₂Ph[4-(*t*-Bu)] | Et |
| G-4252 | Cl | H | CH₂Ph(4-CN) | Et |
| G-4253 | Cl | H | CH₂Ph(4-NO₂) | Et |
| G-4254 | Cl | H | CH₂Ph(4-OCHF₂) | Et |
| G-4255 | Cl | H | CH₂Ph(4-SCHF₂) | Et |
| G-4256 | Cl | H | CH₂Ph(4-CH₂SCF₃) | Et |
| G-4257 | Cl | H | CH₂Ph[4-CF(CF₃)₂] | Et |
| G-4258 | Cl | H | CH₂Ph(4-CH₂SCF₃) | Et |
| G-4259 | Cl | H | CH₂Ph[4-Ph(4-CF₃)] | Et |
| G-4260 | Cl | H | CH₂Ph(2,4-Cl₂) | Et |
| G-4261 | Cl | H | CH₂Ph[2,5-(CF₃)₂] | Et |
| G-4262 | Cl | H | CH₂Ph(3,4-Cl₂) | Et |

**[Table 1217]**

| Compound Number | R³ | R⁴ | R⁵ | R¹¹ |
|---|---|---|---|---|
| G-4263 | Cl | H | CH₂Ph(3-CF₃-4-F) | Et |
| G-4264 | Cl | H | CH₂Ph(3-CF₃-4-Cl) | Et |
| G-4265 | Cl | H | CH₂Ph(3-F-4-CF₃) | Et |
| G-4266 | Cl | H | CH₂Ph(2,4,6-F₃) | Et |
| G-4267 | Cl | H | CH₂Ph(3,4,5-F₃) | Et |
| G-4268 | Cl | H | CH₂Ph(2,3,4-F₃) | Et |
| G-4269 | Cl | H | CH₂Ph(3,4,5-Cl₃) | Et |
| G-4270 | Cl | H | CH₂CH₂Ph | Et |
| G-4271 | Cl | H | CH₂CH₂Ph(4-F) | Et |
| G-4272 | Cl | H | CH₂CH₂Ph(4-Cl) | Et |
| G-4273 | Cl | H | CH₂CH₂Ph(4-Br) | Et |
| G-4274 | Cl | H | CH₂CH₂Ph[4-(*t*-Bu)] | Et |
| G-4275 | Cl | H | CH₂CH₂Ph(2-CF₃) | Et |
| G-4276 | Cl | H | CH₂CH₂Ph(3-CF₃) | Et |
| G-4277 | Cl | H | CH₂CH₂Ph(4-CF₃) | Et |
| G-4278 | Cl | H | CH₂CH₂Ph[4-CF(CF₃)₂] | Et |
| G-4279 | Cl | H | CH₂CH₂Ph[4-(*c*-Pr)] | Et |
| G-4280 | Cl | H | CH₂CH₂Ph{4-[*c*-Pr(2,2-F₂)]} | Et |
| G-4281 | Cl | H | CH₂CH₂Ph(4-OMe) | Et |
| G-4282 | Cl | H | CH₂CH₂Ph(3-OCF₃) | Et |
| G-4283 | Cl | H | CH₂CH₂Ph(4-OCHF₂) | Et |
| G-4284 | Cl | H | CH₂CH₂Ph(4-OCF₃) | Et |
| G-4285 | Cl | H | CH₂CH₂Ph(4-SMe) | Et |
| G-4286 | Cl | H | CH₂CH₂Ph(4-SCHF₂) | Et |
| G-4287 | Cl | H | CH₂CH₂Ph(4-SCF₃) | Et |
| G-4288 | Cl | H | CH₂CH₂Ph[4-S(=O)Me] | Et |
| G-4289 | Cl | H | CH₂CH₂Ph[4-S(=O)CF₃] | Et |
| G-4290 | Cl | H | CH₂CH₂Ph[4-S(=O)₂Me] | Et |
| G-4291 | Cl | H | CH₂CH₂Ph[4-S(=O)₂CF₃] | Et |
| G-4292 | Cl | H | CH₂CH₂Ph(4-CH₂SMe) | Et |
| G-4293 | Cl | H | CH₂CH₂Ph(4-CH₂SCF₃) | Et |
| G-4294 | Cl | H | CH₂CH₂Ph[4-OS(=O)₂Me] | Et |
| G-4295 | Cl | H | CH₂CH₂Ph[4-OS(=O)₂CF₃] | Et |
| G-4296 | Cl | H | CH₂CH₂Ph[4-Ph(4-CF₃)] | Et |
| G-4297 | Cl | H | CH₂CH₂Ph(4-CH₂Ph) | Et |
| G-4298 | Cl | H | CH₂CH₂Ph(4-OCH₂Ph) | Et |
| G-4299 | Cl | H | CH₂CH₂Ph(4-CN) | Et |
| G-4300 | Cl | H | CH₂CH₂Ph(4-NO₂) | Et |
| G-4301 | Cl | H | CH₂CH₂Ph(2,4-Cl₂) | Et |
| G-4302 | Cl | H | CH₂CH₂Ph(3,4-Cl₂) | Et |
| G-4303 | Cl | H | CH₂CH₂Ph(3-CF₃-4-F) | Et |
| G-4304 | Cl | H | CH₂CH₂Ph(2-CF₃-4-F) | Et |
| G-4305 | Cl | H | CH₂CH₂Ph(3-F-4-CF₃) | Et |
| G-4306 | Cl | H | CH₂CH₂Ph(2-F-4-CF₃) | Et |

**[Table 1218]**

| Compound Number | R³ | R⁴ | R⁵ | R¹¹ |
|---|---|---|---|---|
| G-4307 | Cl | H | CH₂CH₂Ph(3-Cl-4-OCHF₂) | Et |
| G-4308 | Cl | H | CH₂CH₂Ph(3,4,5-Cl₃) | Et |
| G-4309 | Cl | H | CH₂CH₂Ph(2,3,4-F₃) | Et |
| G-4310 | Cl | H | CH₂CH₂Ph(2,4,5-F₃) | Et |
| G-4311 | Cl | H | CH₂CH₂Ph(3,4,5-F₃) | Et |
| G-4312 | Cl | H | CH₂CH₂Ph(2,4,6-F₃) | Et |
| G-4313 | Cl | H | CH₂CH₂CH₂Ph | Et |
| G-4314 | Cl | H | CH₂CH₂CH₂Ph(3-CF₃) | Et |
| G-4315 | Cl | H | CH₂CH₂CH₂Ph(4-CF₃) | Et |
| G-4316 | Cl | H | CH₂CH₂CH₂Ph(4-F) | Et |
| G-4317 | Cl | H | CH₂CH₂CH₂Ph[4-(*t*-Bu)] | Et |
| G-4318 | Cl | H | CH₂CH₂CH₂Ph(4-CN) | Et |
| G-4319 | Cl | H | CH₂CH₂CH₂Ph(4-CF₃) | Et |
| G-4320 | Cl | H | CH₂CH₂CH₂Ph(4-OCHF₂) | Et |
| G-4321 | Cl | H | CH₂CH₂CH₂Ph(4-OCF₃) | Et |
| G-4322 | Cl | H | CH₂CH₂CH₂Ph(4-SCHF₂) | Et |
| G-4323 | Cl | H | CH₂CH₂CH₂Ph(4-SCF₃) | Et |
| G-4324 | Cl | H | CH₂CH₂CH₂Ph[4-CF(CF₃)₂] | Et |
| G-4325 | Cl | H | CH₂CH₂CH₂Ph(3,4,5-F₃) | Et |
| G-4326 | Cl | H | CH₂CH₂CH₂Ph(2,4,6-F₃) | Et |
| G-4327 | Cl | H | CH₂CH₂CH₂CH₂Ph | Et |
| G-4328 | Cl | H | CH₂CH₂CH₂CH₂Ph(4-F) | Et |
| G-4329 | Cl | H | CH₂CH₂CH₂CH₂Ph(4-CF₃) | Et |
| G-4330 | Cl | H | CH₂CH₂CH₂CH₂Ph(4-OCF₃) | Et |
| G-4331 | Cl | H | CH₂CH₂CH₂CH₂Ph(4-SCF₃) | Et |
| G-4332 | Cl | H | CH₂CH₂CH₂CH₂CH₂Ph | Et |
| G-4333 | Cl | H | CH₂CH₂CH₂CH₂CH₂CH₂Ph | Et |
| G-4334 | Cl | H | CH₂CF₂Ph(4-F) | Et |
| G-4335 | Cl | H | CH₂CF₂Ph(4-CF₃) | Et |
| G-4336 | Cl | H | CH₂CF₂Ph(3,4,5-F₃) | Et |
| G-4337 | Me | H | CH₂C(=O)Me | Et |
| G-4338 | Me | H | CH₂CH(OH)Me | Et |
| G-4339 | Me | H | CH₂CH(OH)CF₃ | Et |
| G-4340 | Me | H | CH₂C(OH)₂CF₃ | Et |
| G-4341 | Me | H | CH₂C(CF₃)=NOH | Et |
| G-4342 | Me | H | CH₂C(CF₃)=NOMe | Et |
| G-4343 | Me | H | CH₂CH(CF₃)NH₂ | Et |
| G-4344 | Me | H | CH₂CH(CF₃)NHSO₂CF₃ | Et |
| G-4345 | Me | H | CH₂[*c*-Pr(1-NH₂) | Et |
| G-4346 | Me | H | CH₂[*c*-Pr(1-NHC(=O)O(*t*-Bu)] | Et |
| G-4347 | Me | H | CH₂{*c*-Pr[1-NHS(=OhCF₃]} | Et |
| G-4348 | Me | H | CH₂CH₂NH₂ | Et |
| G-4349 | Me | H | CH₂CH₂NH₂, HCl | Et |
| G-4350 | Me | H | CH₂CH₂CH₂NH₂ | Et |

**[Table 1219]**

| Compound Number | R³ | R⁴ | R⁵ | R¹¹ |
|---|---|---|---|---|
| G-4351 | Me | H | CH₂CH₂CH₂CH₂NH₂ | Et |
| G-4352 | Me | H | CH₂CH₂CH₂CH₂CH₂NH₂ | Et |
| G-4353 | Me | H | CH₂CH₂CH₂CH₂CH₂CH₂NH₂ | Et |
| G-4354 | Me | H | CH₂CH₂CH₂CH₂CH₂CH₂CH₂NH₂ | Et |
| G-4355 | Me | H | CH₂CH(Me)NH₂ | Et |
| G-4356 | Me | H | CH₂C*H(Me)NH₂, HCl | Et |
| G-4357 | Me | H | CH₂C*H(Me)NH₂: (R) | Et |
| G-4358 | Me | H | CH₂C*H(Me)NH₂, HCl : (R) | Et |
| G-4359 | Me | H | CH₂C*H(Me)NH₂: (S) | Et |
| G-4360 | Me | H | CH₂C*H(Me)NH₂, HCl : (S) | Et |
| G-4361 | Me | H | CH₂CH(Et)NH₂ | Et |
| G-4362 | Me | H | CH₂CH(*i*-Pr)NH₂ | Et |
| G-4363 | Me | H | CH(Me)CH₂NH₂ | Et |
| G-4364 | Me | H | CH(Me)CH₂NH₂, HCl | Et |
| G-4365 | Me | H | CH(Et)CH₂NH₂ | Et |
| G-4366 | Me | H | CH(*i*-Pr)CH₂NH₂ | Et |
| G-4367 | Me | H | CH₂CH₂CH(Me)NH₂ | Et |
| G-4368 | Me | H | CH₂CH(Me)CH₂NH₂ | Et |
| G-4369 | Me | H | CH(Me)CH₂CH₂NH₂ | Et |
| G-4370 | Me | H | CH₂CH(Me)NH(*c*-Pr) | Et |
| G-4371 | Me | H | CH₂CH(Me)N(*c*-Pr)S(=O)₂CF₃ | Et |
| G-4372 | Me | H | CH₂CH(Me)NHOMe | Et |
| G-4373 | Me | H | CH₂CH(Me)N(OMe)S(=O)₂CF₃ | Et |
| G-4374 | Me | H | CH₂CH(Me)N(OMe)S(=O)₂Ph(2-NO₂) | Et |
| G-4375 | Me | H | CH₂CH(Me)N(OMe)S(=O)₂Ph(4-NO₂) | Et |
| G-4376 | Me | H | CH₂CH₂NHC(=O)O(*t*-Bᵤ) | Et |
| G-4377 | Me | H | CH₂CH₂CH₂NHC(=O)O(*t*-Bu) | Et |
| G-4378 | Me | H | CH₂CH₂CH₂CH₂NHC(=O)O(*t*-Bu) | Et |
| G-4379 | Me | H | CH₂CH₂CH₂CH₂NHC(=O)OCH(CH₃)₂ | Et |
| G-4380 | Me | H | CH₂CH(Me)NHC(=O)O(*t*-Bu) | Et |
| G-4381 | Me | H | CH₂C*H(Me)NHC(=O)O(*t*-Bu) : (R) | Et |
| G-4382 | Me | H | CH₂C*H(Me)NHC(=O)O(*t*-Bu) : (S) | Et |
| G-4383 | Me | H | CH₂CH(Et)NHC(=O)O(*t*-Bu) | Et |
| G-4384 | Me | H | CH₂CH(*i* -Pr)NHC(=O)O(*t*-Bu) | Et |
| G-4385 | Me | H | CH(Me)CH₂NHC(=O)O(*t*-Bu) | Et |
| G-4386 | Me | H | CH(Et)CH₂NHC(=O)O(*t*-Bu) | Et |
| G-4387 | Me | H | CH(*i*-Pr)CH₂NHC(=O)O(*t*-Bu) | Et |
| G-4388 | Me | H | CH₂CH(Me)CH₂NHC(=O)O(*t*-Bu) | Et |
| G-4389 | Me | H | CH₂CH₂SCHF₂ | Et |
| G-4390 | Me | H | CH₂CH₂CH₂SCHF₂ | Et |
| G-4391 | Me | H | CH₂CH₂CH₂CH₂SCHF₂ | Et |
| G-4392 | Me | H | CH₂CH₂CH₂CH₂CH₂SCHF₂ | Et |
| G-4393 | Me | H | CH₂CH₂CH₂CH₂CH₂CH₂SCHF₂ | Et |
| G-4394 | Me | H | CH₂CH₂CH₂CH₂CH₂CH₂CH₂SCHF₂ | Et |

**[Table 1220]**

| Compound Number | R³ | R⁴ | R⁵ | R¹¹ |
|---|---|---|---|---|
| G-4395 | Me | H | CH₂CH₂SCF₃ | Et |
| G-4396 | Me | H | CH₂CH₂CH₂SCF₃ | Et |
| G-4397 | Me | H | CH₂CH₂CH₂CH₂SCF₃ | Et |
| G-4398 | Me | H | CH₂CH₂CH₂CH₂CH₂SCF₃ | Et |
| G-4399 | Me | H | CH₂CH₂CH₂CH₂CH₂CH₂SCF₃ | Et |
| G-4400 | Me | H | CH₂CH₂CH₂CH₂CH₂CH₂CH₂SCF₃ | Et |
| G-4401 | Me | H | CH₂CH₂CH₂CH₂CH₂CH₂CH₂CH₂SCF₃ | Et |
| G-4402 | Me | H | CH₂CH(Me)SCF₃ | Et |
| G-4403 | Me | H | CH(Me)CH₂SCF₃ | Et |
| G-4404 | Me | H | CH₂CH₂CH(Me)SCF₃ | Et |
| G-4405 | Me | H | CH₂CH(Me)CH₂SCF₃ | Et |
| G-4406 | Me | H | CH(Me)CH₂CH₂SCF₃ | Et |
| G-4407 | Me | H | CH₂CH₂CH₂CH(Me)SCF₃ | Et |
| G-4408 | Me | H | CH₂CH₂CH(Me)CH₂SCF₃ | Et |
| G-4409 | Me | H | CH₂CH(Me)CH₂CH₂SCF₃ | Et |
| G-4410 | Me | H | CH(Me)CH₂CH₂CH₂SCF₃ | Et |
| G-4411 | Me | H | CH₂CH₂NHS(=O)₂CF₃ | Et |
| G-4412 | Me | H | CH₂CH₂CH₂NHS(=O)₂CF₃ | Et |
| G-4413 | Me | H | CH₂CH₂CH₂CH₂NHS(=O)₂CF₃ | Et |
| G-4414 | Me | H | CH₂CH₂CH₂CH₂CH₂NHS(=O)₂CF₃ | Et |
| G-4415 | Me | H | CH₂CH₂N(Me)S(=O)₂CF₃ | Et |
| G-4416 | Me | H | CH₂CH₂N(Et)S(=O)₂CF₃ | Et |
| G-4417 | Me | H | CH₂CH₂N(CH₂CN)S(=O)₂CF₃ | Et |
| G-4418 | Me | H | CH₂CH₂N(CH₂OMe)S(=O)₂CF₃ | Et |
| G-4419 | Me | H | CH₂CH₂CH₂N(Me)S(=O)₂CF₃ | Et |
| G-4420 | Me | H | CH₂CH₂CH₂N(Et)S(=O)₂CF₃ | Et |
| G-4421 | Me | H | CH₂CH₂CH₂N(CH₂CN)S(=O)₂CF₃ | Et |
| G-4422 | Me | H | CH₂CH₂CH₂N(CH₂OMe)S(=O)₂CF₃ | Et |
| G-4423 | Me | H | CH₂CH₂CH₂CH₂N(Me)S(=O)₂CF₃ | Et |
| G-4424 | Me | H | CH₂CH₂CH₂CH₂N(Et)S(=O)₂CF₃ | Et |
| G-4425 | Me | H | CH₂CH₂CH₂CH₂N(CH₂CN)S(=O)₂CF₃ | Et |
| G-4426 | Me | H | CH₂CH₂CH₂CH₂N(CH₂OMe)S(=O)₂CF₃ | Et |
| G-4427 | Me | H | CH₂CH(Me)NHS(=O)₂CF₃ | Et |
| G-4428 | Me | H | CH₂CH(Me)NHS(=O)₂CF₃: (R) | Et |
| G-4429 | Me | H | CH₂CH(Me)NHS(=O)₂CF₃ : (S) | Et |
| G-4430 | Me | H | CH(Me)CH₂NHS(=O)₂CF₃ | Et |
| G-4431 | Me | H | CH₂CH₂CH(Me)NHS(=O)₂CF₃ | Et |
| G-4432 | Me | H | CH₂CH(Me)CH₂NHS(=O)₂CF₃ | Et |
| G-4433 | Me | H | CH(Me)CH₂CH₂NHS(=O)₂CF₃ | Et |
| G-4434 | Me | H | CH₂CH(Me)N(Me)S(=O)₂CF₃ | Et |
| G-4435 | Me | H | CH(Me)CH₂N(Me)S(=O)₂CF₃ | Et |
| G-4436 | Me | H | CH₂CH₂CH(Me)N(Me)S(=O)₂CF₃ | Et |
| G-4437 | Me | H | CH₂CH(Me)CH₂N(Me)S(=O)₂CF₃ | Et |
| G-4438 | Me | H | CH(Me)CH₂CH₂N(Me)S(=O)₂CF₃ | Et |

**[Table 1221]**

| Compound Number | R³ | R⁴ | R⁵ | R¹¹ |
|---|---|---|---|---|
| G-4439 | Me | H | CH₂Ph | Et |
| G-4440 | Me | H | CH₂Ph(2-CF₃) | Et |
| G-4441 | Me | H | CH₂Ph(3-CF₃) | Et |
| G-4442 | Me | H | CH₂Ph(4-CF₃) | Et |
| G-4443 | Me | H | CH₂Ph(2-OCF₃) | Et |
| G-4444 | Me | H | CH₂Ph(3-OCF₃) | Et |
| G-4445 | Me | H | CH₂Ph(4-OCF₃) | Et |
| G-4446 | Me | H | CH₂Ph(2-SCF₃) | Et |
| G-4447 | Me | H | CH₂Ph(3-SCF₃) | Et |
| G-4448 | Me | H | CH₂Ph(4-SCF₃) | Et |
| G-4449 | Me | H | CH₂Ph(3-CH₂SCF₃) | Et |
| G-4450 | Me | H | CH₂Ph(4-F) | Et |
| G-4451 | Me | H | CH₂Ph(4-Cl) | Et |
| G-4452 | Me | H | CH₂Ph(4-Br) | Et |
| G-4453 | Me | H | CH₂Ph(4-Me) | Et |
| G-4454 | Me | H | CH₂Ph[4-(*t*-Bu)] | Et |
| G-4455 | Me | H | CH₂Ph(4-CN) | Et |
| G-4456 | Me | H | CH₂Ph(4-NO₂) | Et |
| G-4457 | Me | H | CH₂Ph(4-OCHF₂) | Et |
| G-4458 | Me | H | CH₂Ph(4-SCHF₂) | Et |
| G-4459 | Me | H | CH₂Ph(4-CH₂SCF₃) | Et |
| G-4460 | Me | H | CH₂Ph[4-CF(CF₃)₂] | Et |
| G-4461 | Me | H | CH₂Ph(4-CH₂SCF₃) | Et |
| G-4462 | Me | H | CH₂Ph[4-Ph(4-CF₃)] | Et |
| G-4463 | Me | H | CH₂Ph(2,4-Cl₂) | Et |
| G-4464 | Me | H | CH₂Ph[2,5-(CF₃)₂] | Et |
| G-4465 | Me | H | CH₂Ph(3,4-Cl₂) | Et |
| G-4466 | Me | H | CH₂Ph(3-CF₃-4-F) | Et |
| G-4467 | Me | H | CH₂Ph(3-CF₃-4-Cl) | Et |
| G-4468 | Me | H | CH₂Ph(3-F-4-CF₃) | Et |
| G-4469 | Me | H | CH₂Ph(2,4,6-F₃) | Et |
| G-4470 | Me | H | CH₂Ph(3,4,5-F₃) | Et |
| G-4471 | Me | H | CH₂Ph(2,3,4-F₃) | Et |
| G-4472 | Me | H | CH₂Ph(3,4,5-Cl₃) | Et |
| G-4473 | Me | H | CH₂CH₂Ph | Et |
| G-4474 | Me | H | CH₂CH₂Ph(4-F) | Et |
| G-4475 | Me | H | CH₂CH₂Ph(4-Cl) | Et |
| G-4476 | Me | H | CH₂CH₂Ph(4-Br) | Et |
| G-4477 | Me | H | CH₂CH₂Ph[4-(*t*-Bu)] | Et |
| G-4478 | Me | H | CH₂CH₂Ph(2-CF₃) | Et |
| G-4479 | Me | H | CH₂CH₂Ph(3-CF₃) | Et |
| G-4480 | Me | H | CH₂CH₂Ph(4-CF₃) | Et |
| G-4481 | Me | H | CH₂CH₂Ph[4-CF(CF₃)₂] | Et |
| G-4482 | Me | H | CH₂CH₂Ph[4-(*c*-Pr)] | Et |

**[Table 1222]**

| Compound Number | R³ | R⁴ | R⁵ | R¹¹ |
|---|---|---|---|---|
| G-4483 | Me | H | CH₂CH₂Ph{4-[*c*-Pr(2,2-F₂)]} | Et |
| G-4484 | Me | H | CH₂CH₂Ph(4-OMe) | Et |
| G-4485 | Me | H | CH₂CH₂Ph(3-OCF₃) | Et |
| G-4486 | Me | H | CH₂CH₂Ph(4-OCHF₂) | Et |
| G-4487 | Me | H | CH₂CH₂Ph(4-OCF₃) | Et |
| G-4488 | Me | H | CH₂CH₂Ph(4-SMe) | Et |
| G-4489 | Me | H | CH₂CH₂Ph(4-SCHF₂) | Et |
| G-4490 | Me | H | CH₂CH₂Ph(4-SCF₃) | Et |
| G-4491 | Me | H | CH₂CH₂Ph[4-S(=O)Me] | Et |
| G-4492 | Me | H | CH₂CH₂Ph[4-S(=O)CF₃] | Et |
| G-4493 | Me | H | CH₂CH₂Ph[4-S(=O)₂Me] | Et |
| G-4494 | Me | H | CH₂CH₂Ph[4-S(=O)₂CF₃] | Et |
| G-4495 | Me | H | CH₂CH₂Ph(4-CH₂SMe) | Et |
| G-4496 | Me | H | CH₂CH₂Ph(4-CH₂SCF₃) | Et |
| G-4497 | Me | H | CH₂CH₂Ph[4-OS(=O)₂Me] | Et |
| G-4498 | Me | H | CH₂CH₂Ph[4-OS(=O)₂CF₃] | Et |
| G-4499 | Me | H | CH₂CH₂Ph[4-Ph(4-CF₃)] | Et |
| G-4500 | Me | H | CH₂CH₂Ph(4-CH₂Ph) | Et |
| G-4501 | Me | H | CH₂CH₂Ph(4-OCH₂Ph) | Et |
| G-4502 | Me | H | CH₂CH₂Ph(4-CN) | Et |
| G-4503 | Me | H | CH₂CH₂Ph(4-NO₂) | Et |
| G-4504 | Me | H | CH₂CH₂Ph(2,4-Cl₂) | Et |
| G-4505 | Me | H | CH₂CH₂Ph(3,4-Cl₂) | Et |
| G-4506 | Me | H | CH₂CH₂Ph(3-CF₃-4-F) | Et |
| G-4507 | Me | H | CH₂CH₂Ph(2-CF₃-4-F) | Et |
| G-4508 | Me | H | CH₂CH₂Ph(3-F-4-CF₃) | Et |
| G-4509 | Me | H | CH₂CH₂Ph(2-F-4-CF₃) | Et |
| G-4510 | Me | H | CH₂CH₂Ph(3-Cl-4-OCHF₂) | Et |
| G-4511 | Me | H | CH₂CH₂Ph(3,4,5-Cl₃) | Et |
| G-4512 | Me | H | CH₂CH₂Ph(2,3,4-F₃) | Et |
| G-4513 | Me | H | CH₂CH₂Ph(2,4,5-F₃) | Et |
| G-4514 | Me | H | CH₂CH₂Ph(3,4,5-F₃) | Et |
| G-4515 | Me | H | CH₂CH₂Ph(2,4,6-F₃) | Et |
| G-4516 | Me | H | CH₂CH₂CH₂Ph | Et |
| G-4517 | Me | H | CH₂CH₂CH₂Ph(3-CF₃) | Et |
| G-4518 | Me | H | CH₂CH₂CH₂Ph(4-CF₃) | Et |
| G-4519 | Me | H | CH₂CH₂CH₂Ph(4-F) | Et |
| G-4520 | Me | H | CH₂CH₂CH₂Ph[4-(*t*-Bu)] | Et |
| G-4521 | Me | H | CH₂CH₂CH₂Ph(4-CN) | Et |
| G-4522 | Me | H | CH₂CH₂CH₂Ph(4-CF₃) | Et |
| G-4523 | Me | H | CH₂CH₂CH₂Ph(4-OCHF₂) | Et |
| G-4524 | Me | H | CH₂CH₂CH₂Ph(4-OCF₃) | Et |
| G-4525 | Me | H | CH₂CH₂CH₂Ph(4-SCHF₂) | Et |
| G-4526 | Me | H | CH₂CH₂CH₂Ph(4-SCF₃) | Et |

**[Table 1223]**

| Compound Number | R³ | R⁴ | R⁵ | R¹¹ |
|---|---|---|---|---|
| G-4527 | Me | H | CH₂CH₂CH₂Ph[4-CF(CF₃)₂] | Et |
| G-4528 | Me | H | CH₂CH₂CH₂Ph(3,4,5-F₃) | Et |
| G-4529 | Me | H | CH₂CH₂CH₂Ph(2,4,6-F₃) | Et |
| G-4530 | Me | H | CH₂CH₂CH₂CH₂Ph | Et |
| G-4531 | Me | H | CH₂CH₂CH₂CH₂Ph(4-F) | Et |
| G-4532 | Me | H | CH₂CH₂CH₂CH₂Ph(4-CF₃) | Et |
| G-4533 | Me | H | CH₂CH₂CH₂CH₂Ph(4-OCF₃) | Et |
| G-4534 | Me | H | CH₂CH₂CH₂CH₂Ph(4-SCF₃) | Et |
| G-4535 | Me | H | CH₂CH₂CH₂CH₂CH₂Ph | Et |
| G-4536 | Me | H | CH₂CH₂CH₂CH₂CH₂CH₂Ph | Et |
| G-4537 | Me | H | CH₂CF₂Ph(4-F) | Et |
| G-4538 | Me | H | CH₂CF₂Ph(4-CF₃) | Et |
| G-4539 | Me | H | CH₂CF₂Ph(3,4,5-F₃) | Et |
| G-4540 | CF₃ | H | CH₂CH(Me)NH₂ | Et |
| G-4541 | CF₃ | H | CH₂C*H(Me)NH₂, HCl | Et |
| G-4542 | CF₃ | H | CH₂C*H(Me)NH₂: (R) | Et |
| G-4543 | CF₃ | H | CH₂C*H(Me)NH₂, HCl : (R) | Et |
| G-4544 | CF₃ | H | CH₂C*H(Me)NH₂: (S) | Et |
| G-4545 | CF₃ | H | CH₂C*H(Me)NH₂, HCl : (S) | Et |
| G-4546 | CF₃ | H | CH₂CH(Me)NHC(=O)O(*t*-Bu) | Et |
| G-4547 | CF₃ | H | CH₂C*H(Me)NHC(=O)O(*t*-Bu) : (R) | Et |
| G-4548 | CF₃ | H | CH₂C*H(Me)NHC(=O)O(*t*-Bu) : (S) | Et |
| G-4549 | CF₃ | H | CH₂CH(Me)NHS(=O)₂CF₃ | Et |
| G-4550 | CF₃ | H | CH₂CH(Me)NHS(=O)₂CF₃: (R) | Et |
| G-4551 | CF₃ | H | CH₂CH(Me)NHS(=O)₂CF₃ : (S) | Et |
| G-4552 | OCF₃ | H | CH₂CH(Me)NH₂ | Et |
| G-4553 | OCF₃ | H | CH₂C*H(Me)NH₂, HCl | Et |
| G-4554 | OCF₃ | H | CH₂C*H(Me)NH₂: (R) | Et |
| G-4555 | OCF₃ | H | CH₂C*H(Me)NH₂, HCl : (R) | Et |
| G-4556 | OCF₃ | H | CH₂C*H(Me)NH₂: (S) | Et |
| G-4557 | OCF₃ | H | CH₂C*H(Me)NH₂, HCl : (S) | Et |
| G-4558 | OCF₃ | H | CH₂CH(Me)NHC(=O)O(*t*-Bu) | Et |
| G-4559 | OCF₃ | H | CH₂C*H(Me)NHC(=O)O(*t*-Bu) : (R) | Et |
| G-4560 | OCF₃ | H | CH₂C*H(Me)NHC(=O)O(*t*-Bu) : (S) | Et |
| G-4561 | OCF₃ | H | CH₂CH(Me)NHS(=O)₂CF₃ | Et |
| G-4562 | OCF₃ | H | CH₂CH(Me)NHS(=O)₂CF₃: (R) | Et |
| G-4563 | OCF₃ | H | CH₂CH(Me)NHS(=O)₂CF₃ : (S) | Et |
| G-4564 | Cl | Cl | CH₂CH₂SCHF₂ | Et |
| G-4565 | Cl | Cl | CH₂CH₂CH₂SCHF₂ | Et |
| G-4566 | Cl | Cl | CH₂CH₂CH₂CH₂SCHF₂ | Et |
| G-4567 | Cl | Cl | CH₂CH₂CH₂CH₂CH₂SCHF₂ | Et |
| G-4568 | Cl | Cl | CH₂CH₂CH₂CH₂CH₂CH₂SCHF₂ | Et |
| G-4569 | Cl | Cl | CH₂CH₂CH₂CH₂CH₂CH₂CH₂SCHF₂ | Et |
| G-4570 | Cl | Cl | CH₂CH₂SCF₃ | Et |

**[Table 1224]**

| Compound Number | R³ | R⁴ | R⁵ | R¹¹ |
|---|---|---|---|---|
| G-4571 | Cl | Cl | CH₂CH₂CH₂SCF₃ | Et |
| G-4572 | Cl | Cl | CH₂CH₂CH₂CH₂SCF₃ | Et |
| G-4573 | Cl | Cl | CH₂CH₂CH₂CH₂CH₂SCF₃ | Et |
| G-4574 | Cl | Cl | CH₂CH₂CH₂CH₂CH₂CH₂SCF₃ | Et |
| G-4575 | Cl | Cl | CH₂CH₂CH₂CH₂CH₂CH₂CH₂SCF₃ | Et |
| G-4576 | Cl | Cl | CH₂CH₂CH₂CH₂CH₂CH₂CH₂CH₂SCF₃ | Et |
| G-4577 | Cl | Cl | CH₂CH(Me)SCF₃ | Et |
| G-4578 | Cl | Cl | CH(Me)CH₂SCF₃ | Et |
| G-4579 | Cl | Cl | CH₂CH₂CH(Me)SCF₃ | Et |
| G-4580 | Cl | Cl | CH₂CH(Me)CH₂SCF₃ | Et |
| G-4581 | Cl | Cl | CH(Me)CH₂CH₂SCF₃ | Et |
| G-4582 | Cl | Cl | CH₂CH₂CH₂CH(Me)SCF₃ | Et |
| G-4583 | Cl | Cl | CH₂CH₂CH(Me)CH₂SCF₃ | Et |
| G-4584 | Cl | Cl | CH₂CH(Me)CH₂CH₂SCF₃ | Et |
| G-4585 | Cl | Cl | CH(Me)CH₂CH₂CH₂SCF₃ | Et |
| G-4586 | Cl | Cl | CH₂CH₂NHS(=O)₂CF₃ | Et |
| G-4587 | Cl | Cl | CH₂CH₂CH₂NHS(=O)₂CF₃ | Et |
| G-4588 | Cl | Cl | CH₂CH₂CH₂CH₂NHS(=O)₂CF₃ | Et |
| G-4589 | Cl | Cl | CH₂CH₂CH₂CH₂CH₂NHS(=O)₂CF₃ | Et |
| G-4590 | Cl | Cl | CH₂CH₂N(Me)S(=O)₂CF₃ | Et |
| G-4591 | Cl | Cl | CH₂CH₂N(Et)S(= O)₂CF₃ | Et |
| G-4592 | Cl | Cl | CH₂CH₂N(CH₂CN)S(=O)₂CF₃ | Et |
| G-4593 | Cl | Cl | CH₂CH₂N(CH₂OMe)S(=O)₂CF₃ | Et |
| G-4594 | Cl | Cl | CH₂CH₂CH₂N(Me)S(=O)₂CF₃ | Et |
| G-4595 | Cl | Cl | CH₂CH₂CH₂N(Et)S(=O)₂CF₃ | Et |
| G-4596 | Cl | Cl | CH₂CH₂CH₂N(CH₂CN)S(=O)₂CF₃ | Et |
| G-4597 | Cl | Cl | CH₂CH₂CH₂N(CH₂OMe)S(=O)₂CF₃ | Et |
| G-4598 | Cl | Cl | CH₂CH₂CH₂CH₂N(Me)S(=O)₂CF₃ | Et |
| G-4599 | Cl | Cl | CH₂CH₂CH₂CH₂N(Et)S(=O)₂CF₃ | Et |
| G-4600 | Cl | Cl | CH₂CH₂CH₂CH₂N(CH₂CN)S(=O)₂CF₃ | Et |
| G-4601 | Cl | Cl | CH₂CH₂CH₂CH₂N(CH₂OMe)S(=O)₂CF₃ | Et |
| G-4602 | Cl | Cl | CH₂CH(Me)NHS(=O)₂CF₃ | Et |
| G-4603 | Cl | Cl | CH(Me)CH₂NHS(=O)₂CF₃ | Et |
| G-4604 | Cl | Cl | CH₂CH₂CH(Me)NHS(=O)₂CF₃ | Et |
| G-4605 | Cl | Cl | CH₂CH(Me)CH₂NHS(=O)₂CF₃ | Et |
| G-4606 | Cl | Cl | CH(Me)CH₂CH₂NHS(=O)₂CF₃ | Et |
| G-4607 | Cl | Cl | CH₂CH(Me)N(Me)S(=O)₂CF₃ | Et |
| G-4608 | Cl | Cl | CH(Me)CH₂N(Me)S(=O)₂CF₃ | Et |
| G-4609 | Cl | Cl | CH₂CH₂CH(Me)N(Me)S(=O)₂CF₃ | Et |
| G-4610 | Cl | Cl | CH₂CH(Me)CH₂N(Me)S(=O)₂CF₃ | Et |
| G-4611 | Cl | Cl | CH(Me)CH₂CH₂N(Me)S(=O)₂CF₃ | Et |
| G-4612 | Cl | Cl | CH₂Ph | Et |
| G-4613 | Cl | Cl | CH₂Ph(2-CF₃) | Et |
| G-4614 | Cl | Cl | CH₂Ph(3-CF₃) | Et |

**[Table 1225]**

| Compound Number | R³ | R⁴ | R⁵ | R¹¹ |
|---|---|---|---|---|
| G-4615 | Cl | Cl | CH₂Ph(4-CF₃) | Et |
| G-4616 | Cl | Cl | CH₂Ph(2-OCF₃) | Et |
| G-4617 | Cl | Cl | CH₂Ph(3-OCF₃) | Et |
| G-4618 | Cl | Cl | CH₂Ph(4-OCF₃) | Et |
| G-4619 | Cl | Cl | CH₂Ph(2-SCF₃) | Et |
| G-4620 | Cl | Cl | CH₂Ph(3-SCF₃) | Et |
| G-4621 | Cl | Cl | CH₂Ph(4-SCF₃) | Et |
| G-4622 | Cl | Cl | CH₂Ph(3-CH₂SCF₃) | Et |
| G-4623 | Cl | Cl | CH₂Ph(4-F) | Et |
| G-4624 | Cl | Cl | CH₂Ph(4-Cl) | Et |
| G-4625 | Cl | Cl | CH₂Ph(4-Br) | Et |
| G-4626 | Cl | Cl | CH₂Ph(4-Me) | Et |
| G-4627 | Cl | Cl | CH₂Ph[4-(*t*-Bu)] | Et |
| G-4628 | Cl | Cl | CH₂Ph(4-CN) | Et |
| G-4629 | Cl | Cl | CH₂Ph(4-NO₂) | Et |
| G-4630 | Cl | Cl | CH₂Ph(4-OCHF₂) | Et |
| G-4631 | Cl | Cl | CH₂Ph(4-SCHF₂) | Et |
| G-4632 | Cl | Cl | CH₂Ph(4-CH₂SCF₃) | Et |
| G-4633 | Cl | Cl | CH₂Ph[4-CF(CF₃)₂] | Et |
| G-4634 | Cl | Cl | CH₂Ph(4-CH₂SCF₃) | Et |
| G-4635 | Cl | Cl | CH₂Ph[4-Ph(4-CF₃)] | Et |
| G-4636 | Cl | Cl | CH₂Ph(2,4-Cl₂) | Et |
| G-4637 | Cl | Cl | CH₂Ph[2,5-(CF₃)₂] | Et |
| G-4638 | Cl | Cl | CH₂Ph(3,4-Cl₂) | Et |
| G-4639 | Cl | Cl | CH₂Ph(3-CF₃-4-F) | Et |
| G-4640 | Cl | Cl | CH₂Ph(3-CF₃-4-Cl) | Et |
| G-4641 | Cl | Cl | CH₂Ph(3-F-4-CF₃) | Et |
| G-4642 | Cl | Cl | CH₂Ph(2,4,6-F₃) | Et |
| G-4643 | Cl | Cl | CH₂Ph(3,4,5-F₃) | Et |
| G-4644 | Cl | Cl | CH₂Ph(2,3,4-F₃) | Et |
| G-4645 | Cl | Cl | CH₂Ph(3,4,5-Cl₃) | Et |
| G-4646 | Cl | Cl | CH₂CH₂Ph | Et |
| G-4647 | Cl | Cl | CH₂CH₂Ph(4-F) | Et |
| G-4648 | Cl | Cl | CH₂CH₂Ph(4-Cl) | Et |
| G-4649 | Cl | Cl | CH₂CH₂Ph(4-Br) | Et |
| G-4650 | Cl | Cl | CH₂CH₂Ph[4-(*t*-Bu)] | Et |
| G-4651 | Cl | Cl | CH₂CH₂Ph(2-CF₃) | Et |
| G-4652 | Cl | Cl | CH₂CH₂Ph(3-CF₃) | Et |
| G-4653 | Cl | Cl | CH₂CH₂Ph(4-CF₃) | Et |
| G-4654 | Cl | Cl | CH₂CH₂Ph[4-CF(CF₃)₂] | Et |
| G-4655 | Cl | Cl | CH₂CH₂Ph[4-(*c*-Pr)] | Et |
| G-4656 | Cl | Cl | CH₂CH₂Ph{4-[*c*-Pr(2,2-F₂)]} | Et |
| G-4657 | Cl | Cl | CH₂CH₂Ph(4-OMe) | Et |
| G-4658 | Cl | Cl | CH₂CH₂Ph(3-OCF₃) | Et |

**[Table 1226]**

| Compound Number | R³ | R⁴ | R⁵ | R¹¹ |
|---|---|---|---|---|
| G-4659 | Cl | Cl | CH₂CH₂Ph(4-OCHF₂) | Et |
| G-4660 | Cl | Cl | CH₂CH₂Ph(4-OCF₃) | Et |
| G-4661 | Cl | Cl | CH₂CH₂Ph(4-SMe) | Et |
| G-4662 | Cl | Cl | CH₂CH₂Ph(4-SCHF₂) | Et |
| G-4663 | Cl | Cl | CH₂CH₂Ph(4-SCF₃) | Et |
| G-4664 | Cl | Cl | CH₂CH₂Ph[4-S(=O)Me] | Et |
| G-4665 | Cl | Cl | CH₂CH₂Ph[4-S(=O)CF₃] | Et |
| G-4666 | Cl | Cl | CH₂CH₂Ph[4-S(=O)₂Me] | Et |
| G-4667 | Cl | Cl | CH₂CH₂Ph[4-S(=O)₂CF₃] | Et |
| G-4668 | Cl | Cl | CH₂CH₂Ph(4-CH₂SMe) | Et |
| G-4669 | Cl | Cl | CH₂CH₂Ph(4-CH₂SCF₃) | Et |
| G-4670 | Cl | Cl | CH₂CH₂Ph[4-OS(=O)₂Me] | Et |
| G-4671 | Cl | Cl | CH₂CH₂Ph[4-OS(=O)₂CF₃] | Et |
| G-4672 | Cl | Cl | CH₂CH₂Ph[4-Ph(4-CF₃)] | Et |
| G-4673 | Cl | Cl | CH₂CH₂Ph(4-CH₂Ph) | Et |
| G-4674 | Cl | Cl | CH₂CH₂Ph(4-OCH₂Ph) | Et |
| G-4675 | Cl | Cl | CH₂CH₂Ph(4-CN) | Et |
| G-4676 | Cl | Cl | CH₂CH₂Ph(4-NO₂) | Et |
| G-4677 | Cl | Cl | CH₂CH₂Ph(2,4-Cl₂) | Et |
| G-4678 | Cl | Cl | CH₂CH₂Ph(3,4-Cl₂) | Et |
| G-4679 | Cl | Cl | CH₂CH₂Ph(3-CF₃-4-F) | Et |
| G-4680 | Cl | Cl | CH₂CH₂Ph(2-CF₃-4-F) | Et |
| G-4681 | Cl | Cl | CH₂CH₂Ph(3-F-4-CF₃) | Et |
| G-4682 | Cl | Cl | CH₂CH₂Ph(2-F-4-CF₃) | Et |
| G-4683 | Cl | Cl | CH₂CH₂Ph(3-Cl-4-OCHF₂) | Et |
| G-4684 | Cl | Cl | CH₂CH₂Ph(3,4,5-Cl₃) | Et |
| G-4685 | Cl | Cl | CH₂CH₂Ph(2,3,4-F₃) | Et |
| G-4686 | Cl | Cl | CH₂CH₂Ph(2,4,5-F₃) | Et |
| G-4687 | Cl | Cl | CH₂CH₂Ph(3,4,5-F₃) | Et |
| G-4688 | Cl | Cl | CH₂CH₂Ph(2,4,6-F₃) | Et |
| G-4689 | Cl | Cl | CH₂CH₂CH₂Ph | Et |
| G-4690 | Cl | Cl | CH₂CH₂CH₂Ph(3-CF₃) | Et |
| G-4691 | Cl | Cl | CH₂CH₂CH₂Ph(4-CF₃) | Et |
| G-4692 | Cl | Cl | CH₂CH₂CH₂Ph(4-F) | Et |
| G-4693 | Cl | Cl | CH₂CH₂CH₂Ph[4-(*t*-Bu)] | Et |
| G-4694 | Cl | Cl | CH₂CH₂CH₂Ph(4-CN) | Et |
| G-4695 | Cl | Cl | CH₂CH₂CH₂Ph(4-CF₃) | Et |
| G-4696 | Cl | Cl | CH₂CH₂CH₂Ph(4-OCHF₂) | Et |
| G-4697 | Cl | Cl | CH₂CH₂CH₂Ph(4-OCF₃) | Et |
| G-4698 | Cl | Cl | CH₂CH₂CH₂Ph(4-SCHF₂) | Et |
| G-4699 | Cl | Cl | CH₂CH₂CH₂Ph(4-SCF₃) | Et |
| G-4700 | Cl | Cl | CH₂CH₂CH₂Ph[4-CF(CF₃)₂] | Et |
| G-4701 | Cl | Cl | CH₂CH₂CH₂Ph(3,4,5-F₃) | Et |
| G-4702 | Cl | Cl | CH₂CH₂CH₂Ph(2,4,6-F₃) | Et |

**[Table 1227]**

| Compound Number | R³ | R⁴ | R⁵ | R¹¹ |
|---|---|---|---|---|
| G-4703 | Cl | Cl | CH₂CH₂CH₂CH₂Ph | Et |
| G-4704 | Cl | Cl | CH₂CH₂CH₂CH₂Ph(4-F) | Et |
| G-4705 | Cl | Cl | CH₂CH₂CH₂CH₂Ph(4-CF₃) | Et |
| G-4706 | Cl | Cl | CH₂CH₂CH₂CH₂Ph(4-OCF₃) | Et |
| G-4707 | Cl | Cl | CH₂CH₂CH₂CH₂Ph(4-SCF₃) | Et |
| G-4708 | Cl | Cl | CH₂CH₂CH₂CH₂CH₂Ph | Et |
| G-4709 | Cl | Cl | CH₂CH₂CH₂CH₂CH₂CH₂Ph | Et |
| G-4710 | Cl | Cl | CH₂CF₂Ph(4-F) | Et |
| G-4711 | Cl | Cl | CH₂CF₂Ph(4-CF₃) | Et |
| G-4712 | Cl | Cl | CH₂CF₂Ph(3,4,5-F₃) | Et |
| G-4713 | Me | Cl | CH₂CH₂SCHF₂ | Et |
| G-4714 | Me | Cl | CH₂CH₂CH₂SCHF₂ | Et |
| G-4715 | Me | Cl | CH₂CH₂CH₂CH₂SCHF₂ | Et |
| G-4716 | Me | Cl | CH₂CH₂CH₂CH₂CH₂SCHF₂ | Et |
| G-4717 | Me | Cl | CH₂CH₂CH₂CH₂CH₂CH₂SCHF₂ | Et |
| G-4718 | Me | Cl | CH₂CH₂CH₂CH₂CH₂CH₂CH₂SCHF₂ | Et |
| G-4719 | Me | Cl | CH₂CH₂SCF₃ | Et |
| G-4720 | Me | Cl | CH₂CH₂CH₂SCF₃ | Et |
| G-4721 | Me | Cl | CH₂CH₂CH₂CH₂SCF₃ | Et |
| G-4722 | Me | Cl | CH₂CH₂CH₂CH₂CH₂SCF₃ | Et |
| G-4723 | Me | Cl | CH₂CH₂CH₂CH₂CH₂CH₂SCF₃ | Et |
| G-4724 | Me | Cl | CH₂CH₂CH₂CH₂CH₂CH₂CH₂SCF₃ | Et |
| G-4725 | Me | Cl | CH₂CH₂CH₂CH₂CH₂CH₂CH₂CH₂SCF₃ | Et |
| G-4726 | Me | Cl | CH₂CH(Me)SCF₃ | Et |
| G-4727 | Me | Cl | CH(Me)CH₂SCF₃ | Et |
| G-4728 | Me | Cl | CH₂CH₂CH(Me)SCF₃ | Et |
| G-4729 | Me | Cl | CH₂CH(Me)CH₂SCF₃ | Et |
| G-4730 | Me | Cl | CH(Me)CH₂CH₂SCF₃ | Et |
| G-4731 | Me | Cl | CH₂CH₂CH₂CH(Me)SCF₃ | Et |
| G-4732 | Me | Cl | CH₂CH₂CH(Me)CH₂SCF₃ | Et |
| G-4733 | Me | Cl | CH₂CH(Me)CH₂CH₂SCF₃ | Et |
| G-4734 | Me | Cl | CH(Me)CH₂CH₂CH₂SCF₃ | Et |
| G-4735 | Me | Cl | CH₂CH₂NHS(=O)₂CF₃ | Et |
| G-4736 | Me | Cl | CH₂CH₂CH₂NHS(=O)₂CF₃ | Et |
| G-4737 | Me | Cl | CH₂CH₂CH₂CH₂NHS(=O)₂CF₃ | Et |
| G-4738 | Me | Cl | CH₂CH₂CH₂CH₂CH₂NHS(=O)₂CF₃ | Et |
| G-4739 | Me | Cl | CH₂CH₂N(Me)S(=O)₂CF₃ | Et |
| G-4740 | Me | Cl | CH₂CH₂N(Et)S(=O)₂CF₃ | Et |
| G-4741 | Me | Cl | CH₂CH₂N(CH₂CN)S(=O)₂CF₃ | Et |
| G-4742 | Me | Cl | CH₂CH₂N(CH₂OMe)S(=O)₂CF₃ | Et |
| G-4743 | Me | Cl | CH₂CH₂CH₂N(Me)S(=O)₂CF₃ | Et |
| G-4744 | Me | Cl | CH₂CH₂CH₂N(Et)S(=O)₂CF₃ | Et |
| G-4745 | Me | Cl | CH₂CH₂CH₂N(CH₂CN)S(=O)₂CF₃ | Et |
| G-4746 | Me | Cl | CH₂CH₂CH₂N(CH₂OMe)S(=O)₂CF₃ | Et |

**[Table 1228]**

| Compound Number | R³ | R⁴ | R⁵ | R¹¹ |
|---|---|---|---|---|
| G-4747 | Me | Cl | CH₂CH₂CH₂CH₂N(Me)S(=O)₂CF₃ | Et |
| G-4748 | Me | Cl | CH₂CH₂CH₂CH₂N(Et)S(=O)₂CF₃ | Et |
| G-4749 | Me | Cl | CH₂CH₂CH₂CH₂N(CH₂CN)S(=O)₂CF₃ | Et |
| G-4750 | Me | Cl | CH₂CH₂CH₂CH₂N(CH₂OMe)S(=O)₂CF₃ | Et |
| G-4751 | Me | Cl | CH₂CH(Me)NHS(=O)₂CF₃ | Et |
| G-4752 | Me | Cl | CH(Me)CH₂NHS(=O)₂CF₃ | Et |
| G-4753 | Me | Cl | CH₂CH₂CH(Me)NHS(=O)₂CF₃ | Et |
| G-4754 | Me | Cl | CH₂CH(Me)CH₂NHS(=O)₂CF₃ | Et |
| G-4755 | Me | Cl | CH(Me)CH₂CH₂NHS(=O)₂CF₃ | Et |
| G-4756 | Me | Cl | CH₂CH(Me)N(Me)S(=O)₂CF₃ | Et |
| G-4757 | Me | Cl | CH(Me)CH₂N(Me)S(=O)₂CF₃ | Et |
| G-4758 | Me | Cl | CH₂CH₂CH(Me)N(Me)S(=O)₂CF₃ | Et |
| G-4759 | Me | Cl | CH₂CH(Me)CH₂N(Me)S(=O)₂CF₃ | Et |
| G-4760 | Me | Cl | CH(Me)CH₂CH₂N(Me)S(=O)₂CF₃ | Et |
| G-4761 | Me | Cl | CH₂Ph | Et |
| G-4762 | Me | Cl | CH₂Ph(2-CF₃) | Et |
| G-4763 | Me | Cl | CH₂Ph(3-CF₃) | Et |
| G-4764 | Me | Cl | CH₂Ph(4-CF₃) | Et |
| G-4765 | Me | Cl | CH₂Ph(2-OCF₃) | Et |
| G-4766 | Me | Cl | CH₂Ph(3-OCF₃) | Et |
| G-4767 | Me | Cl | CH₂Ph(4-OCF₃) | Et |
| G-4768 | Me | Cl | CH₂Ph(2-SCF₃) | Et |
| G-4769 | Me | Cl | CH₂Ph(3-SCF₃) | Et |
| G-4770 | Me | Cl | CH₂Ph(4-SCF₃) | Et |
| G-4771 | Me | Cl | CH₂Ph(3-CH₂SCF₃) | Et |
| G-4772 | Me | Cl | CH₂Ph(4-F) | Et |
| G-4773 | Me | Cl | CH₂Ph(4-Cl) | Et |
| G-4774 | Me | Cl | CH₂Ph(4-Br) | Et |
| G-4775 | Me | Cl | CH₂Ph(4-Me) | Et |
| G-4776 | Me | Cl | CH₂Ph[4-(*t*-Bu)] | Et |
| G-4777 | Me | Cl | CH₂Ph(4-CN) | Et |
| G-4778 | Me | Cl | CH₂Ph(4-NO₂) | Et |
| G-4779 | Me | Cl | CH₂Ph(4-OCHF₂) | Et |
| G-4780 | Me | Cl | CH₂Ph(4-SCHF₂) | Et |
| G-4781 | Me | Cl | CH₂Ph(4-CH₂SCF₃) | Et |
| G-4782 | Me | Cl | CH₂Ph[4-CF(CF₃)₂] | Et |
| G-4783 | Me | Cl | CH₂Ph(4-CH₂SCF₃) | Et |
| G-4784 | Me | Cl | CH₂Ph[4-Ph(4-CF₃)] | Et |
| G-4785 | Me | Cl | CH₂Ph(2,4-Cl₂) | Et |
| G-4786 | Me | Cl | CH₂Ph[2,5-(CF₃)₂] | Et |
| G-4787 | Me | Cl | CH₂Ph(3,4-Cl₂) | Et |
| G-4788 | Me | Cl | CH₂Ph(3-CF₃-4-F) | Et |
| G-4789 | Me | Cl | CH₂Ph(3-CF₃-4-Cl) | Et |
| G-4790 | Me | Cl | CH₂Ph(3-F-4-CF₃) | Et |

**[Table 1229]**

| Compound Number | R³ | R⁴ | R⁵ | R¹¹ |
|---|---|---|---|---|
| G-4791 | Me | Cl | CH₂Ph(2,4,6-F₃) | Et |
| G-4792 | Me | Cl | CH₂Ph(3,4,5-F₃) | Et |
| G-4793 | Me | Cl | CH₂Ph(2,3,4-F₃) | Et |
| G-4794 | Me | Cl | CH₂Ph(3,4,5-Cl₃) | Et |
| G-4795 | Me | Cl | CH₂CH₂Ph | Et |
| G-4796 | Me | Cl | CH₂CH₂Ph(4-F) | Et |
| G-4797 | Me | Cl | CH₂CH₂Ph(4-Cl) | Et |
| G-4798 | Me | Cl | CH₂CH₂Ph(4-Br) | Et |
| G-4799 | Me | Cl | CH₂CH₂Ph[4-(*t*-Bu)] | Et |
| G-4800 | Me | Cl | CH₂CH₂Ph(2-CF₃) | Et |
| G-4801 | Me | Cl | CH₂CH₂Ph(3-CF₃) | Et |
| G-4802 | Me | Cl | CH₂CH₂Ph(4-CF₃) | Et |
| G-4803 | Me | Cl | CH₂CH₂Ph[4-CF(CF₃)₂] | Et |
| G-4804 | Me | Cl | CH₂CH₂Ph[4-(*c*-Pr)] | Et |
| G-4805 | Me | Cl | CH₂CH₂Ph{4-[*c*-Pr(2,2-F₂)]} | Et |
| G-4806 | Me | Cl | CH₂CH₂Ph(4-OMe) | Et |
| G-4807 | Me | Cl | CH₂CH₂Ph(3-OCF₃) | Et |
| G-4808 | Me | Cl | CH₂CH₂Ph(4-OCHF₂) | Et |
| G-4809 | Me | Cl | CH₂CH₂Ph(4-OCF₃) | Et |
| G-4810 | Me | Cl | CH₂CH₂Ph(4-SMe) | Et |
| G-4811 | Me | Cl | CH₂CH₂Ph(4-SCHF₂) | Et |
| G-4812 | Me | Cl | CH₂CH₂Ph(4-SCF₃) | Et |
| G-4813 | Me | Cl | CH₂CH₂Ph[4-S(=O)Me] | Et |
| G-4814 | Me | Cl | CH₂CH₂Ph[4-S(=O)CF₃] | Et |
| G-4815 | Me | Cl | CH₂CH₂Ph[4-S(=O)₂Me] | Et |
| G-4816 | Me | Cl | CH₂CH₂Ph[4-S(=O)₂CF₃] | Et |
| G-4817 | Me | Cl | CH₂CH₂Ph(4-CH₂SMe) | Et |
| G-4818 | Me | Cl | CH₂CH₂Ph(4-CH₂SCF₃) | Et |
| G-4819 | Me | Cl | CH₂CH₂Ph[4-OS(=O)₂Me] | Et |
| G-4820 | Me | Cl | CH₂CH₂Ph[4-OS(=O)₂CF₃] | Et |
| G-4821 | Me | Cl | CH₂CH₂Ph[4-Ph(4-CF₃)] | Et |
| G-4822 | Me | Cl | CH₂CH₂Ph(4-CH₂Ph) | Et |
| G-4823 | Me | Cl | CH₂CH₂Ph(4-OCH₂Ph) | Et |
| G-4824 | Me | Cl | CH₂CH₂Ph(4-CN) | Et |
| G-4825 | Me | Cl | CH₂CH₂Ph(4-NO₂) | Et |
| G-4826 | Me | Cl | CH₂CH₂Ph(2,4-Cl₂) | Et |
| G-4827 | Me | Cl | CH₂CH₂Ph(3,4-Cl₂) | Et |
| G-4828 | Me | Cl | CH₂CH₂Ph(3-CF₃-4-F) | Et |
| G-4829 | Me | Cl | CH₂CH₂Ph(2-CF₃-4-F) | Et |
| G-4830 | Me | Cl | CH₂CH₂Ph(3-F-4-CF₃) | Et |
| G-4831 | Me | Cl | CH₂cH₂Ph(2-F-4-CF₃) | Et |
| G-4832 | Me | Cl | CH₂CH₂Ph(3-Cl-4-OCHF₂) | Et |
| G-4833 | Me | Cl | CH₂CH₂Ph(3,4,5-Cl₃) | Et |
| G-4834 | Me | Cl | CH₂CH₂Ph(2,3,4-F₃) | Et |

**[Table 1230]**

| Compound Number | R³ | R⁴ | R⁵ | R¹¹ |
|---|---|---|---|---|
| G-4835 | Me | Cl | CH₂CH₂Ph(2,4,5-F₃) | Et |
| G-4836 | Me | Cl | CH₂CH₂Ph(3,4,5-F₃) | Et |
| G-4837 | Me | Cl | CH₂CH₂Ph(2,4,6-F₃) | Et |
| G-4838 | Me | Cl | CH₂CH₂CH₂Ph | Et |
| G-4839 | Me | Cl | CH₂CH₂CH₂Ph(3-CF₃) | Et |
| G-4840 | Me | Cl | CH₂CH₂CH₂Ph(4-CF₃) | Et |
| G-4841 | Me | Cl | CH₂CH₂CH₂Ph(4-F) | Et |
| G-4842 | Me | Cl | CH₂CH₂CH₂Ph[4-(*t*-Bu)] | Et |
| G-4843 | Me | Cl | CH₂CH₂CH₂Ph(4-CN) | Et |
| G-4844 | Me | Cl | CH₂CH₂CH₂Ph(4-CF₃) | Et |
| G-4845 | Me | Cl | CH₂CH₂CH₂Ph(4-OCHF₂) | Et |
| G-4846 | Me | Cl | CH₂CH₂CH₂Ph(4-OCF₃) | Et |
| G-4847 | Me | Cl | CH₂CH₂CH₂Ph(4-SCHF₂) | Et |
| G-4848 | Me | Cl | CH₂CH₂CH₂Ph(4-SCF₃) | Et |
| G-4849 | Me | Cl | CH₂CH₂CH₂Ph[4-CF(CF₃)₂] | Et |
| G-4850 | Me | Cl | CH₂CH₂CH₂Ph(3,4,5-F₃) | Et |
| G-4851 | Me | Cl | CH₂CH₂CH₂Ph(2,4,6-F₃) | Et |
| G-4852 | Me | Cl | CH₂CH₂CH₂CH₂Ph | Et |
| G-4853 | Me | Cl | CH₂CH₂CH₂CH₂Ph(4-F) | Et |
| G-4854 | Me | Cl | CH₂CH₂CH₂CH₂Ph(4-CF₃) | Et |
| G-4855 | Me | Cl | CH₂CH₂CH₂CH₂Ph(4-OCF₃) | Et |
| G-4856 | Me | Cl | CH₂CH₂CH₂CH₂Ph(4-SCF₃) | Et |
| G-4857 | Me | Cl | CH₂CH₂CH₂CH₂CH₂Ph | Et |
| G-4858 | Me | Cl | CH₂CH₂CH₂CH₂CH₂CH₂Ph | Et |
| G-4859 | Me | Cl | CH₂CF₂Ph(4-F) | Et |
| G-4860 | Me | Cl | CH₂CF₂Ph(4-CF₃) | Et |
| G-4861 | Me | Cl | CH₂CF₂Ph(3,4,5-F₃) | Et |
| G-4862 | Me | Me | CH₂CH₂SCHF₂ | Et |
| G-4863 | Me | Me | CH₂CH₂CH₂SCHF₂ | Et |
| G-4864 | Me | Me | CH₂CH₂CH₂CH₂SCHF₂ | Et |
| G-4865 | Me | Me | CH₂CH₂CH₂CH₂CH₂SCHF₂ | Et |
| G-4866 | Me | Me | CH₂CH₂CH₂CH₂CH₂CH₂SCHF₂ | Et |
| G-4867 | Me | Me | CH₂CH₂CH₂CH₂CH₂CH₂CH₂SCHF₂ | Et |
| G-4868 | Me | Me | CH₂CH₂SCF₃ | Et |
| G-4869 | Me | Me | CH₂CH₂CH₂SCF₃ | Et |
| G-4870 | Me | Me | CH₂CH₂CH₂CH₂SCF₃ | Et |
| G-4871 | Me | Me | CH₂CH₂CH₂CH₂CH₂SCF₃ | Et |
| G-4872 | Me | Me | CH₂CH₂CH₂CH₂CH₂CH₂SCF₃ | Et |
| G-4873 | Me | Me | CH₂CH₂CH₂CH₂CH₂CH₂CH₂SCF₃ | Et |
| G-4874 | Me | Me | CH₂CH₂CH₂CH₂CH₂CH₂CH₂CH₂SCF₃ | Et |
| G-4875 | Me | Me | CH₂CH(Me)SCF₃ | Et |
| G-4876 | Me | Me | CH(Me)CH₂SCF₃ | Et |
| G-4877 | Me | Me | CH₂CH₂CH(Me)SCF₃ | Et |
| G-4878 | Me | Me | CH₂CH(Me)CH₂SCF₃ | Et |

**[Table 1231]**

| Compound Number | R³ | R⁴ | R⁵ | R¹¹ |
|---|---|---|---|---|
| G-4879 | Me | Me | CH(Me)CH₂CH₂SCF₃ | Et |
| G-4880 | Me | Me | CH₂CH₂CH₂CH(Me)SCF₃ | Et |
| G-4881 | Me | Me | CH₂CH₂CH(Me)CH₂SCF₃ | Et |
| G-4882 | Me | Me | CH₂CH(Me)CH₂CH₂SCF₃ | Et |
| G-4883 | Me | Me | CH(Me)CH₂CH₂CH₂SCF₃ | Et |
| G-4884 | Me | Me | CH₂CH₂NHS(=O)₂CF₃ | Et |
| G-4885 | Me | Me | CH₂CH₂CH₂NHS(=O)₂CF₃ | Et |
| G-4886 | Me | Me | CH₂CH₂CH₂CH₂NHS(=O)₂CF₃ | Et |
| G-4887 | Me | Me | CH₂CH₂CH₂CH₂CH₂NHS(=O)₂CF₃ | Et |
| G-4888 | Me | Me | CH₂CH₂N(Me)S(=O)₂CF₃ | Et |
| G-4889 | Me | Me | CH₂CH₂N(Et)S(=O)₂CF₃ | Et |
| G-4890 | Me | Me | CH₂CH₂N(CH₂CN)S(=O)₂CF₃ | Et |
| G-4891 | Me | Me | CH₂CH₂N(CH₂OMe)S(=O)₂CF₃ | Et |
| G-4892 | Me | Me | CH₂CH₂CH₂N(Me)S(=O)₂CF₃ | Et |
| G-4893 | Me | Me | CH₂CH₂CH₂N(Et)S(=O)₂CF₃ | Et |
| G-4894 | Me | Me | CH₂CH₂CH₂N(CH₂CN)S(=O)₂CF₃ | Et |
| G-4895 | Me | Me | CH₂CH₂CH₂N(CH₂OMe)S(=O)₂CF₃ | Et |
| G-4896 | Me | Me | CH₂CH₂CH₂CH₂N(Me)S(=O)₂CF₃ | Et |
| G-4897 | Me | Me | CH₂CH₂CH₂CH₂N(Et)S(=O)₂CF₃ | Et |
| G-4898 | Me | Me | CH₂CH₂CH₂CH₂N(CH₂CN)S(=O)₂CF₃ | Et |
| G-4899 | Me | Me | CH₂CH₂CH₂CH₂N(CH₂OMe)S(=O)₂CF₃ | Et |
| G-4900 | Me | Me | CH₂CH(Me)NHS(=O)₂CF₃ | Et |
| G-4901 | Me | Me | CH(Me)CH₂NHS(=O)₂CF₃ | Et |
| G-4902 | Me | Me | CH₂CH₂CH(Me)NHS(=O)₂CF₃ | Et |
| G-4903 | Me | Me | CH₂CH(Me)CH₂NHS(=O)₂CF₃ | Et |
| G-4904 | Me | Me | CH(Me)CH₂CH₂NHS(=O)₂CF₃ | Et |
| G-4905 | Me | Me | CH₂CH(Me)N(Me)S(=O)₂CF₃ | Et |
| G-4906 | Me | Me | CH(Me)CH₂N(Me)S(=O)₂CF₃ | Et |
| G-4907 | Me | Me | CH₂CH₂CH(Me)N(Me)S(=O)₂CF₃ | Et |
| G-4908 | Me | Me | CH₂CH(Me)CH₂N(Me)S(=O)₂CF₃ | Et |
| G-4909 | Me | Me | CH(Me)CH₂CH₂N(Me)S(=O)₂CF₃ | Et |
| G-4910 | Me | Me | CH₂Ph | Et |
| G-4911 | Me | Me | CH₂Ph(2-CF₃) | Et |
| G-4912 | Me | Me | CH₂Ph(3-CF₃) | Et |
| G-4913 | Me | Me | CH₂Ph(4-CF₃) | Et |
| G-4914 | Me | Me | CH₂Ph(2-OCF₃) | Et |
| G-4915 | Me | Me | CH₂Ph(3-OCF₃) | Et |
| G-4916 | Me | Me | CH₂Ph(4-OCF₃) | Et |
| G-4917 | Me | Me | CH₂Ph(2-SCF₃) | Et |
| G-4918 | Me | Me | CH₂Ph(3-SCF₃) | Et |
| G-4919 | Me | Me | CH₂Ph(4-SCF₃) | Et |
| G-4920 | Me | Me | CH₂Ph(3-CH₂SCF₃) | Et |
| G-4921 | Me | Me | CH₂Ph(4-F) | Et |
| G-4922 | Me | Me | CH₂Ph(4-Cl) | Et |

**[Table 1232]**

| Compound Number | R³ | R⁴ | R⁵ | R¹¹ |
|---|---|---|---|---|
| G-4923 | Me | Me | CH₂Ph(4-Br) | Et |
| G-4924 | Me | Me | CH₂Ph(4-Me) | Et |
| G-4925 | Me | Me | CH₂Ph[4-(*t*-Bu)] | Et |
| G-4926 | Me | Me | CH₂Ph(4-CN) | Et |
| G-4927 | Me | Me | CH₂Ph(4-NO₂) | Et |
| G-4928 | Me | Me | CH₂Ph(4-OCHF₂) | Et |
| G-4929 | Me | Me | CH₂Ph(4-SCHF₂) | Et |
| G-4930 | Me | Me | CH₂Ph(4-CH₂SCF₃) | Et |
| G-4931 | Me | Me | CH₂Ph[4-CF(CF₃)₂] | Et |
| G-4932 | Me | Me | CH₂Ph(4-CH₂SCF₃) | Et |
| G-4933 | Me | Me | CH₂Ph[4-Ph(4-CF₃)] | Et |
| G-4934 | Me | Me | CH₂Ph(2,4-Cl₂) | Et |
| G-4935 | Me | Me | CH₂Ph[2,5-(CF₃)₂] | Et |
| G-4936 | Me | Me | CH₂Ph(3,4-Cl₂) | Et |
| G-4937 | Me | Me | CH₂Ph(3-CF₃-4-F) | Et |
| G-4938 | Me | Me | CH₂Ph(3-CF₃-4-Cl) | Et |
| G-4939 | Me | Me | CH₂Ph(3-F-4-CF₃) | Et |
| G-4940 | Me | Me | CH₂Ph(2,4,6-F₃) | Et |
| G-4941 | Me | Me | CH₂Ph(3,4,5-F₃) | Et |
| G-4942 | Me | Me | CH₂Ph(2,3,4-F₃) | Et |
| G-4943 | Me | Me | CH₂Ph(3,4,5-Cl₃) | Et |
| G-4944 | Me | Me | CH₂CH₂Ph | Et |
| G-4945 | Me | Me | CH₂CH₂Ph(4-F) | Et |
| G-4946 | Me | Me | CH₂CH₂Ph(4-Cl) | Et |
| G-4947 | Me | Me | CH₂CH₂Ph(4-Br) | Et |
| G-4948 | Me | Me | CH₂CH₂Ph[4-(*t*-Bu)] | Et |
| G-4949 | Me | Me | CH₂CH₂Ph(2-CF₃) | Et |
| G-4950 | Me | Me | CH₂CH₂Ph(3-CF₃) | Et |
| G-4951 | Me | Me | CH₂CH₂Ph(4-CF₃) | Et |
| G-4952 | Me | Me | CH₂CH₂Ph[4-CF(CF₃)₂] | Et |
| G-4953 | Me | Me | CH₂CH₂Ph[4-(*c*-Pr)] | Et |
| G-4954 | Me | Me | CH₂CH₂Ph{4-[*c*-Pr(2,2-F₂)]} | Et |
| G-4955 | Me | Me | CH₂CH₂Ph(4-OMe) | Et |
| G-4956 | Me | Me | CH₂CH₂Ph(3-OCF₃) | Et |
| G-4957 | Me | Me | CH₂CH₂Ph(4-OCHF₂) | Et |
| G-4958 | Me | Me | CH₂CH₂Ph(4-OCF₃) | Et |
| G-4959 | Me | Me | CH₂CH₂Ph(4-SMe) | Et |
| G-4960 | Me | Me | CH₂CH₂Ph(4-SCHF₂) | Et |
| G-4961 | Me | Me | CH₂CH₂Ph(4-SCF₃) | Et |
| G-4962 | Me | Me | CH₂CH₂Ph[4-S(=O)Me] | Et |
| G-4963 | Me | Me | CH₂CH₂Ph[4-S(=O)CF₃] | Et |
| G-4964 | Me | Me | CH₂CH₂Ph[4-S(=O)₂Me] | Et |
| G-4965 | Me | Me | CH₂CH₂Ph[4-S(=O)₂CF₃] | Et |
| G-4966 | Me | Me | CH₂CH₂Ph(4-CH₂SMe) | Et |

**[Table 1233]**

| Compound Number | R³ | R⁴ | R⁵ | R¹¹ |
|---|---|---|---|---|
| G-4967 | Me | Me | CH₂CH₂Ph(4-CH₂SCF₃) | Et |
| G-4968 | Me | Me | CH₂CH₂Ph[4-OS(=O)₂Me] | Et |
| G-4969 | Me | Me | CH₂CH₂Ph[4-OS(=O)₂CF₃] | Et |
| G-4970 | Me | Me | CH₂CH₂Ph[4-Ph(4-CF₃)] | Et |
| G-4971 | Me | Me | CH₂CH₂Ph(4-CH₂Ph) | Et |
| G-4972 | Me | Me | CH₂CH₂Ph(4-OCH₂Ph) | Et |
| G-4973 | Me | Me | CH₂CH₂Ph(4-CN) | Et |
| G-4974 | Me | Me | CH₂CH₂Ph(4-NO₂) | Et |
| G-4975 | Me | Me | CH₂CH₂Ph(2,4-Cl₂) | Et |
| G-4976 | Me | Me | CH₂CH₂Ph(3,4-Cl₂) | Et |
| G-4977 | Me | Me | CH₂CH₂Ph(3-CF₃-4-F) | Et |
| G-4978 | Me | Me | CH₂CH₂Ph(2-CF₃-4-F) | Et |
| G-4979 | Me | Me | CH₂CH₂Ph(3-F-4-CF₃) | Et |
| G-4980 | Me | Me | CH₂CH₂Ph(2-F-4-CF₃) | Et |
| G-4981 | Me | Me | CH₂CH₂Ph(3-Cl-4-OCHF₂) | Et |
| G-4982 | Me | Me | CH₂CH₂Ph(3,4,5-Cl₃) | Et |
| G-4983 | Me | Me | CH₂CH₂Ph(2,3,4-F₃) | Et |
| G-4984 | Me | Me | CH₂CH₂Ph(2,4,5-F₃) | Et |
| G-4985 | Me | Me | CH₂CH₂Ph(3,4,5-F₃) | Et |
| G-4986 | Me | Me | CH₂CH₂Ph(2,4,6-F₃) | Et |
| G-4987 | Me | Me | CH₂CH₂CH₂Ph | Et |
| G-4988 | Me | Me | CH₂CH₂CH₂Ph(3-CF₃) | Et |
| G-4989 | Me | Me | CH₂CH₂CH₂Ph(4-CF₃) | Et |
| G-4990 | Me | Me | CH₂CH₂CH₂Ph(4-F) | Et |
| G-4991 | Me | Me | CH₂CH₂CH₂Ph[4-(*t*-Bu)] | Et |
| G-4992 | Me | Me | CH₂CH₂CH₂Ph(4-CN) | Et |
| G-4993 | Me | Me | CH₂CH₂CH₂Ph(4-CF₃) | Et |
| G-4994 | Me | Me | CH₂CH₂CH₂Ph(4-OCHF₂) | Et |
| G-4995 | Me | Me | CH₂CH₂CH₂Ph(4-OCF₃) | Et |
| G-4996 | Me | Me | CH₂CH₂CH₂Ph(4-SCHF₂) | Et |
| G-4997 | Me | Me | CH₂CH₂CH₂Ph(4-SCF₃) | Et |
| G-4998 | Me | Me | CH₂CH₂CH₂Ph[4-CF(CF₃)₂] | Et |
| G-4999 | Me | Me | CH₂CH₂CH₂Ph(3,4,5-F₃) | Et |
| G-5000 | Me | Me | CH₂CH₂CH₂Ph(2,4,6-F₃) | Et |
| G-5001 | Me | Me | CH₂CH₂CH₂CH₂Ph | Et |
| G-5002 | Me | Me | CH₂CH₂CH₂CH₂Ph(4-F) | Et |
| G-5003 | Me | Me | CH₂CH₂CH₂CH₂Ph(4-CF₃) | Et |
| G-5004 | Me | Me | CH₂CH₂CH₂CH₂Ph(4-OCF₃) | Et |
| G-5005 | Me | Me | CH₂CH₂CH₂CH₂Ph(4-SCF₃) | Et |
| G-5006 | Me | Me | CH₂CH₂CH₂CH₂CH₂Ph | Et |
| G-5007 | Me | Me | CH₂CH₂CH₂CH₂CH₂CH₂Ph | Et |
| G-5008 | Me | Me | CH₂CF₂Ph(4-F) | Et |
| G-5009 | Me | Me | CH₂CF₂Ph(4-CF₃) | Et |
| G-5010 | Me | Me | CH₂CF₂Ph(3,4,5-F₃) | Et |

Meanwhile, the compound of the present invention of formula [I] as defined in the claims can be produced according to the following production method, but the production method is not limited to these methods. Hereinafter, for example, "the compound represented by general formula [I]", "the compound of formula [I]", and "the compound [I]" are synonymous.

### <Production method 1>

A compound of formula [III-2] among the compounds of the present invention can be produced according to a method based on the reaction scheme exemplified below. 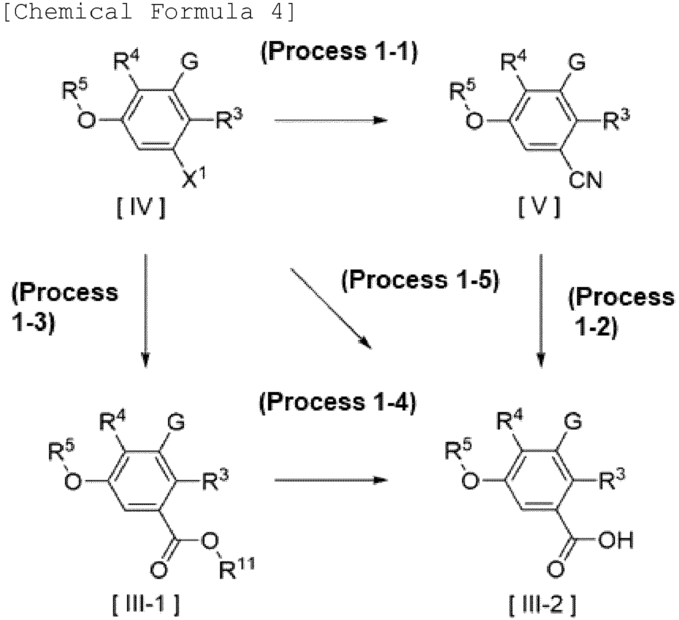 (In the formula, X¹ represents a halogen atom, R¹¹ represents a (C1-C6)alkyl, and G, R³, R⁴, and R⁵ are as defined above.)

That is, the compound of formula [III-2] can be produced by (Process 1-1) converting a compound of formula [IV] into a compound [V] using a cyanide in an appropriate solvent in the presence or absence of an appropriate catalyst, and then (Process 1-2) hydrolyzing the compound of formula [V] in an appropriate solvent in the presence of an appropriate acid or base. In addition, the compound [III-2] can be produced by (Process 1-3) subjecting a compound of formula [IV] to a carbon monoxide insertion reaction in an appropriate solvent in the presence of an appropriate catalyst, an appropriate base, and an appropriate alcohol to obtain a compound of formula [III-1] among the compounds of the present invention, and then (Process 1-4) hydrolyzing the compound of formula [III-1] in an appropriate solvent in the presence of an appropriate acid or base. Furthermore, the compound [III-2] can be produced by (Process 1-5) subjecting a compound of formula [IV] to a carbon monoxide insertion reaction in water or a mixed solvent of water and an appropriate solvent in the presence of an appropriate catalyst and an appropriate base.

### (Process 1-1)

Examples of the cyanide used in the current reaction include sodium cyanide, potassium cyanide, ammonium cyanide, zinc cyanide, copper cyanide, and a mixture thereof. The amount of use of the cyanide may be appropriately selected from the range of usually 1 to 100 mol, and is preferably 1.0 to 5.0 mol per 1 mol of the compound [IV].

Examples of the catalyst used in the current reaction include tetrakis(triphenylphosphine)palladium and [1,1-bis(diphenylphosphino)ferrocene]palladium (II) dichloride dichloromethane adduct. The amount of use of the catalyst may be appropriately selected from the range of usually 0.001 to 0.5 mol, and is preferably 0.01 to 0.2 mol per 1 mol of the compound [IV].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; halogenated hydrocarbons such as dichloromethane, chloroform, and 1,2-dichloroethane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; nitriles such as acetonitrile and propionitrile; esters such as ethyl acetate, butyl acetate, and ethyl propionate; aliphatic hydrocarbons such as pentane, hexane, cyclohexane, and heptane; pyridines such as pyridine and picoline; water, and a mixed solvent thereof. The amount of use of the solvent is 0.1 to 500 liters, and is preferably 0.3 to 50 liters per 1 mol of the compound [IV].

The reaction temperature of the current reaction may be selected from an arbitrary temperature range usually from - 30°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 72 hours.

After completion of the reaction, the compound [V] can be isolated by conducting an operation of pouring the reaction mixture into water and collecting the precipitated solid by filtration, or subjecting the reaction mixture to extraction with an organic solvent, followed by concentration. The isolated compound [V] also can be further purified by column chromatography, recrystallization, or the like as necessary.

### (Process 1-2)

Examples of the acid that can be used in the current reaction include mineral acids such as hydrochloric acid, hydrobromic acid, and sulfuric acid; carboxylic acids such as acetic acid, propionic acid, and trifluoroacetic acid; and sulfonic acids such as methanesulfonic acid and trifluoromethanesulfonic acid. The amount of use of the acid may be appropriately selected from the range of 1 to moles corresponding to the amount of the solvent, and is preferably 1.0 to 100.0 moles per 1 mole of the compound [V].

Examples of the base that can be used in the current reaction include inorganic bases such as hydroxides of alkali metals, such as lithium hydroxide, sodium hydroxide, and potassium hydroxide; hydroxides of alkaline earth metals, such as calcium hydroxide and magnesium hydroxide; carbonates of alkali metals, such as sodium carbonate and potassium carbonate; and alkali metal bicarbonates such as sodium bicarbonate and potassium bicarbonate. The amount of use of the base may be appropriately selected from the range of 0.1 to 50.0 mol, and is preferably 0.5 to 20.0 mol per 1 mol of the compound [V].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; ketones such as acetone, methyl ethyl ketone, and cyclohexanone; water, and a mixed solvent thereof. The amount of use of the solvent is 0.1 to 500 liters, and is preferably 0.3 to 30 liters per 1 mol of the compound [V].

The reaction temperature of the current reaction may be selected from an arbitrary temperature range usually from - 30°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 72 hours.

After completion of the current reaction, the compound [III-2] can be isolated by conducting an operation of pouring the reaction mixture into water to neutralize the mixture and collecting the precipitated solid by filtration, or subjecting the reaction mixture to extraction with an organic solvent, followed by concentration. The isolated compound [III-2] also can be further purified by column chromatography, recrystallization, or the like as necessary.

### (Process 1-3)

As the catalyst that can be used in the current reaction, a transition metal complex or a combination of a transition metal salt and a ligand may be used. Examples of the transition metal complex include tetrakis(triphenylphosphine)palladium, dichloro[1,1'-bis(diphenylphosphino)ferrocene]palladium-dichloromethane adduct, and tris(dibenzylideneacetone)dipalladium(0). Examples of the transition metal salt include palladium chloride, palladium bromide, and palladium acetate. Examples of the ligand include trimethylphosphine, triethylphosphine, tributylphosphine, triphenylphosphine, 1,3-bis(dimethylphosphino)propane, 1,2-bis(diphenylphosphino)ethane, and 1,3-bis(diphenylphosphino)propane. The amount of use of the catalyst is 0.001 to 0.1 mol, and is preferably 0.01 to 0.05 mol per 1 mol of the compound [IV].

Examples of the base that can be used in the current reaction include inorganic bases such as hydroxides of alkali metals, such as lithium hydroxide, sodium hydroxide, and potassium hydroxide; hydroxides of alkaline earth metals, such as calcium hydroxide and magnesium hydroxide; carbonates of alkali metals, such as sodium carbonate and potassium carbonate; and alkali metal bicarbonates such as sodium bicarbonate and potassium bicarbonate; and organic bases such as triethylamine, 4-methylmorpholine, N-ethyldiisopropylamine, 1,8-diazabicyclo[5.4.0]-7-undecene, pyridine, 4-(N,N-dimethylamino)pyridine, and 2,6-lutidine. The amount of use of the base is 0.1 to 10.0 mol, and is preferably 0.1 to 2.0 mol per 1 mol of the compound [IV]. In the case of an organic base, the organic base can also be used as a solvent.

Examples of the alcohol that can be used in the current reaction include methanol, ethanol, and 2-propanol. The amount of use of the alcohols is 0.1 to 500 liters, and is preferably 0.3 to 30 liters per 1 mol of the compound [IV].

The alcohol can also be used as a solvent.

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; nitriles such as acetonitrile and propionitrile; ketones such as acetone, methyl ethyl ketone, and cyclohexanone; and a mixed solvent thereof. The amount of use of the solvent is 0.1 to 500 liters, and is preferably 0.3 to 30 liters per 1 mol of the compound [IV].

The reaction temperature of the current reaction may be selected from an arbitrary temperature range usually from - 30°C to the reflux temperature in the reaction system, and is preferably in the range of 0°C to 150°C.

The reaction pressure of the current reaction may be selected from an arbitrary pressure range usually from the atmospheric pressure to 50 atm, and is preferably in the range of 1 atm to less than 10 atm.

The reaction time of the current reaction varies depending on the reaction temperature, the reaction pressure, the reaction substrate, the reaction amount, and the like, but is usually 10 minutes to 72 hours.

After completion of the current reaction, the compound [III-1] can be isolated by conducting an operation of pouring the reaction mixture into water to neutralize the mixture and collecting the precipitated solid by filtration, or subjecting the reaction mixture to extraction with an organic solvent, followed by concentration. The isolated compound [III-1] also can be further purified by column chromatography, recrystallization, or the like as necessary.

### (Process 1-4)

The acid, base, and solvent that can be used in the current reaction, the reaction temperature, and the reaction time are the same as those in Process 1-2.

After completion of the current reaction, the compound [III-2] can be isolated by conducting an operation of pouring the reaction mixture into water to neutralize the mixture and collecting the precipitated solid by filtration, or subjecting the reaction mixture to extraction with an organic solvent, followed by concentration. The isolated compound [III-2] also can be further purified by column chromatography, recrystallization, or the like as necessary.

### (Process 1-5)

The base and solvent that can be used in the current reaction, the reaction temperature, and the reaction time are the same as those in Process 1-3.

After completion of the current reaction, the compound [III-2] can be isolated by conducting an operation of pouring the reaction mixture into water to neutralize the mixture and collecting the precipitated solid by filtration, or subjecting the reaction mixture to extraction with an organic solvent, followed by concentration. The isolated compound [III-2] also can be further purified by column chromatography, recrystallization, or the like as necessary.

Furthermore, the compound [III-2] can also be produced by reacting a compound of formula [IV] with an appropriate carbon monoxide equivalent in an appropriate solvent, in the presence of an appropriate acid anhydride, an appropriate base, an appropriate inorganic salt, and an appropriate catalyst, according to the method described in Org. Lett., Vol. 5 (No. 23), page 4269 to 4272 (2003) or Synthesis, Vol. 18, page 3106 to 3110 (2006).

### <Production method 2>

A compound of formula [I-1] among the compounds of the present invention can be produced using a compound of formula [III-3] according to a method based on the reaction scheme exemplified below. 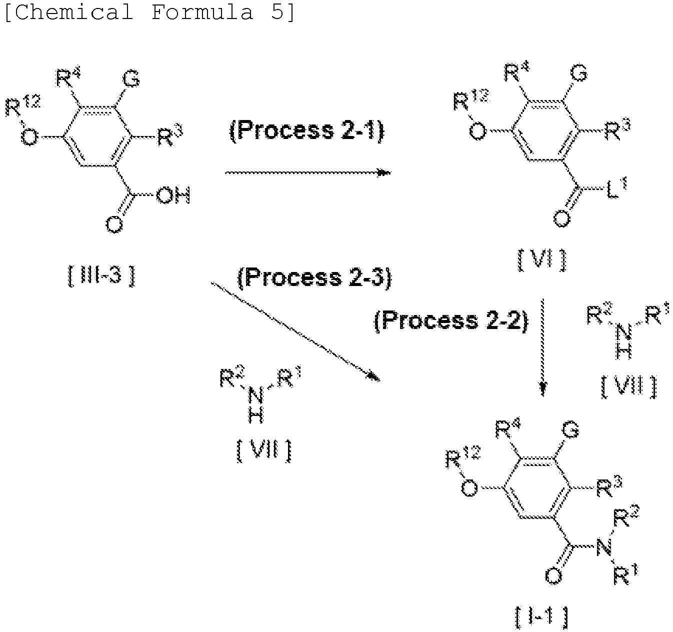 (In the formula, L¹ represents a leaving group such as a halogen atom, OC(=O)OR¹¹, a methanesulfonyloxy, a trifluoromethanesulfonyloxy, a nonafluorobutanesulfonyloxy, a benzenesulfonyloxy, or a p-toluenesulfonyloxy, R¹² represents a hydrogen atom or R⁵, and G, R¹, R², R³, R⁴, R⁵, and R¹¹ are as defined above.)

That is, the compound of formula [I-1] can be produced by (Process 2-1) converting a compound [III-3] into a compound [VI] which is an active derivative such as an acid halide or a mixed acid anhydride in an appropriate solvent, in the presence or absence of an appropriate catalyst, and then (Process 2-2) reacting the compound [VI] with a compound [VII], in an appropriate solvent, in the presence or absence of an appropriate base, in the presence or absence of an appropriate catalyst. Furthermore, the compound of formula [I-1] can also be produced by (Process 2-3) reacting a compound [III-3] and a compound [VII] in an appropriate solvent, in the presence or absence of an appropriate condensing agent and an appropriate additive, in the presence or absence of an appropriate catalyst, in the presence or absence of an appropriate base.

### (Process 2-1)

Examples of the reagent for converting the compound [II-2] into the compound [VI] used in the current reaction include thionyl chloride, oxalyl chloride, phosphoryl chloride, isobutyl chlorocarbonate, methanesulfonyl chloride, trifluoromethanesulfonyl chloride, and p-toluenesulfonyl chloride. The amount of use of the reagent for converting the compound [II-2] into the compound [VI] may be appropriately selected from the range of 0.5 to 30.0 mol, and is preferably 0.5 to 6.0 mol per 1 mol of the compound [III-3].

Examples of the catalyst that can be used in the current reaction include N,N-dimethylformamide. The amount of use of the catalyst may be appropriately selected from the range of 0.01 to 1.0 mol, and is preferably 0.01 to 0.1 mol per 1 mol of the compound [III-3].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; halogenated hydrocarbons such as dichloromethane, chloroform, and 1,2-dichloroethane; nitriles such as acetonitrile and propionitrile; esters such as ethyl acetate, butyl acetate, and ethyl propionate; aliphatic hydrocarbons such as pentane, hexane, cyclohexane, and heptane; and a mixed solvent thereof. The amount of use of the solvent is 0.1 to 100 liters, and is preferably 0.3 to 10 liters per 1 mol of the compound [III-3].

The reaction temperature of the current reaction may be selected from an arbitrary temperature range usually from - 30°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 24 hours.

After completion of the reaction, the compound [VI] can be isolated by conducting an operation such as concentration.

### (Process 2-2)

The amount of use of the compound [VII] used in the current reaction may be appropriately selected from the range of usually 1 to 5 mol, and is preferably 1.0 to 2.5 mol per 1 mol of the compound [VI].

Examples of the base that can be used in the current reaction include organic bases such as triethylamine, 4-methylmorpholine, N-ethyldiisopropylamine, 1,8-diazabicyclo[5.4.0]-7-undecene, pyridine, 4-(N,N-dimethylamino)pyridine, and 2,6-lutidine; and inorganic bases such as hydroxides of alkali metals, such as lithium hydroxide, sodium hydroxide, and potassium hydroxide; carbonates of alkali metals, such as sodium carbonate and potassium carbonate; hydroxides of alkaline earth metals, such as calcium hydroxide and magnesium hydroxide; and bicarbonates of alkali metals, such as sodium bicarbonate and potassium bicarbonate. The amount of use of the base may be appropriately selected from the range of 1 to 10 mol, and is preferably 1.0 to 5 mol per 1 mol of the compound [VI].

Examples of the solvent that can be used in the current reaction include halogenated hydrocarbons such as dichloromethane, chloroform, and 1,2-dichloroethane; aliphatic hydrocarbons such as pentane, hexane, cyclohexane, and heptane; aromatic hydrocarbons such as benzene, toluene, xylene, and chlorobenzene; esters such as ethyl acetate, butyl acetate, and ethyl propionate; nitriles such as acetonitrile and propionitrile; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; ethers such as diethyl ether, cyclopentyl methyl ether, tetrahydrofuran, 2-methyltetrahydrofuran, and 1,4-dioxane; water, and a mixed solvent thereof. The amount of use of the solvent is 0.1 to 100 liters, and is preferably 0.3 to 50 liters per 1 mol of the compound [VI].

The current reaction can be carried out in the presence of a catalyst as necessary, and examples of the catalyst include tetrabutylammonium bromide, tetrabutylammonium chloride, 15-crown-5, and 18-crown-6. The amount of use of the catalyst may be appropriately selected from the range of 0.00 to 1 mol, and is preferably 0.01 to 0.1 mol per 1 mol of the compound [VI].

The reaction temperature of the current reaction may be selected from an arbitrary temperature range from -20°C to the reflux temperature in the reaction system, and is preferably in the range of 0°C to 80°C.

The reaction time of the current reaction varies depending on the reaction temperature, the reaction substrate, the reaction amount, and the like, but is usually 1 minute to 48 hours.

After completion of the reaction, the compound [I-1] can be isolated by conducting an operation of pouring the reaction mixture into water and collecting the precipitated solid by filtration, or subjecting the reaction mixture to extraction with an organic solvent, followed by concentration. The isolated compound [I-1] also can be further purified by column chromatography, recrystallization, or the like as necessary.

### (Process 2-3)

The amount of use of the compound [VII] used in the current reaction may be appropriately selected from the range of 1 to 3 mol, and is preferably 1.0 to 1.5 mol per 1 mol of the compound [III-3].

Examples of the condensing agent that can be used in the current reaction include 1-ethyl-3-(3-dimethylaminopropyl)carbodiimide hydrochloride, 1-ethyl-3-(3-dimethylaminopropyl)carbodiimide, N,N'-dicyclohexylcarbodiimide, N,N'-diisopropylcarbodiimide, N,N'-carbonyldiimidazole, 4-(4,6-dimethoxy-1,3,5-triazine-2-yl)-4-methylmorpholinium chloride, 1H-benzotriazole-1-yloxytris(dimethylamino)phosphonium hexafluorophosphate, {{[(1-cyano-2-ethoxy-2-oxoethylidene)amino]oxy}-4-morpholinomethylene}dimethylammonium hexafluorophosphate, and O-(7-azabenzotriazole-1-yl)-N,N,N',N'-tetramethyluronium hexafluorophosphate. The amount of use of the condensing agent may be appropriately selected from the range of 1.0 to 5.0 mol, and is preferably 1.0 to 3.0 mol per 1 mol of the compound [III-3].

When an additive is used in the current reaction, examples of the additive that can be used include 1-hydroxybenzotriazole, N-hydroxysuccinimide, and 1-hydroxy-7-azabenzotriazole. The amount of use of the additive may be appropriately selected from the range of 0.01 to 3.0 mol, and is preferably 1.0 to 1.5 mol per 1 mol of the compound [III-3].

When a base is used in the current reaction, examples of the base that can be used include organic bases such as triethylamine, 4-methylmorpholine, N-ethyldiisopropylamine, 1,8-diazabicyclo[5.4.0]-7-undecene, pyridine, 4-(N,N-dimethylamino)pyridine, and 2, 6-lutidine. The amount of use of the base may be appropriately selected from the range of 0.1 to 5.0 mol, and is preferably 0.1 to 3.0 mol per 1 mol of the compound [III-3].

When a catalyst is used in the current reaction, examples of the catalyst that can be used include 4-(N,N-dimethylamino)pyridine. The amount of use of the catalyst may be appropriately selected from the range of 0.001 to 1.0 mol, and is preferably 0.01 to 0.1 mol per 1 mol of the compound [III-3].

Examples of the solvent that can be used in the current reaction include halogenated hydrocarbons such as dichloromethane, chloroform, and 1,2-dichloroethane; aliphatic hydrocarbons such as pentane, hexane, cyclohexane, and heptane; esters such as ethyl acetate, butyl acetate, and ethyl propionate; nitriles such as acetonitrile and propionitrile; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; ethers such as diethyl ether, cyclopentyl methyl ether, tetrahydrofuran, 2-methyltetrahydrofuran, and 1,4-dioxane; and a mixed solvent thereof. The amount of use of the solvent is 0.1 to 100 liters, and is preferably 0.3 to 50 liters per 1 mol of the compound [III-3].

The reaction temperature of the current reaction may be selected from an arbitrary temperature range from -20°C to the reflux temperature in the reaction system, and is preferably in the range of 0°C to 80°C.

The reaction time of the current reaction varies depending on the reaction temperature, the reaction substrate, the reaction amount, and the like, but is usually 1 minute to 96 hours.

After completion of the reaction, the compound [I-1] can be isolated by conducting an operation of pouring the reaction mixture into water and collecting the precipitated solid by filtration, or subjecting the reaction mixture to extraction with an organic solvent, followed by concentration. The isolated compound [I-1] also can be further purified by column chromatography, recrystallization, or the like as necessary.

### <Production method 3>

A compound of formula [I-2] among the compounds of the present invention can be produced using a compound of formula [II-1] according to a method based on the reaction scheme exemplified below. 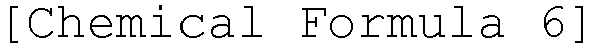 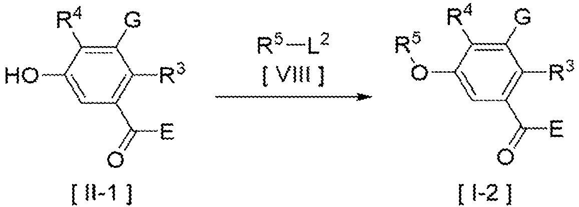 (In the formula, E represents a (C1-C6)alkoxy or a group NR¹R², L² represents a leaving group such as a halogen atom, a methanesulfonyloxy, a trifluoromethanesulfonyloxy, a nonafluorobutanesulfonyloxy, a benzenesulfonyloxy, or a p-toluenesulfonyloxy, and G, R¹, R², R³, R⁴, and R⁵ are as defined above). That is, the compound of the present invention of formula [I-2] can be produced by reacting a compound [II-1] and a compound [VIII] in an appropriate solvent, in the presence of an appropriate base, in the presence or absence of an appropriate catalyst.)

The amount of use of the compound [VIII] used in the current reaction may be appropriately selected from the range of 1.0 to 15.0 mol, and is preferably 1.0 to 6.0 mol per 1 mol of the compound [II-1].

Examples of the base that can be used in the current reaction include inorganic bases such as hydroxides of alkali metals, such as sodium hydroxide and potassium hydroxide; hydroxides of alkaline earth metals, such as calcium hydroxide and magnesium hydroxide; carbonates of alkali metals, such as sodium carbonate and potassium carbonate; and alkali metal bicarbonates such as sodium bicarbonate and potassium bicarbonate; metal hydrides such as sodium hydride and potassium hydride; metal salts of alcohols, such as sodium methoxide, sodium ethoxide, potassium tert-butoxide; and organic bases such as triethylamine, 4-methylmorpholine, N-ethyldiisopropylamine, 1,8-diazabicyclo[5.4.0]-7-undecene, pyridine, 4-(N,N-dimethylamino)pyridine, and 2, 6-lutidine. The amount of use of the base may be appropriately selected from the range of 1.0 to 5.0 mol, and is preferably 1.0 to 1.5 mol per 1 mol of the compound [II-1].

Examples of the catalyst that can be used in the current reaction include tetra-n-butylammonium bromide and tetra-n-butylammonium iodide. The amount of use of the catalyst may be appropriately selected from the range of 0.01 to 1.0 mol, and is preferably 0.05 to 0.5 mol per 1 mol of the compound [II-1].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; halogenated hydrocarbons such as dichloromethane, chloroform, and 1,2-dichloroethane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; nitriles such as acetonitrile and propionitrile; esters such as ethyl acetate, butyl acetate, and ethyl propionate; aliphatic hydrocarbons such as pentane, hexane, cyclohexane, and heptane; pyridines such as pyridine and picoline; water, and a mixed solvent thereof. The amount of use of the solvent is 0.1 to 100 liters, and is preferably 0.1 to 15 liters per 1 mol of the compound [II-1].

The reaction temperature of the current reaction may be selected from an arbitrary temperature range usually from - 30°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 24 hours.

After completion of the reaction, the compound [I-2] can be isolated by conducting an operation of pouring the reaction mixture into water or the like and subjecting the mixture to extraction with an organic solvent, followed by concentration. The isolated compound [I-2] also can be further purified by column chromatography, recrystallization, or the like as necessary.

### <Production method 4>

A compound of formula [I-2] among the compounds of the present invention can also be produced using a compound of formula [II-1] according to a method based on the reaction scheme exemplified below. 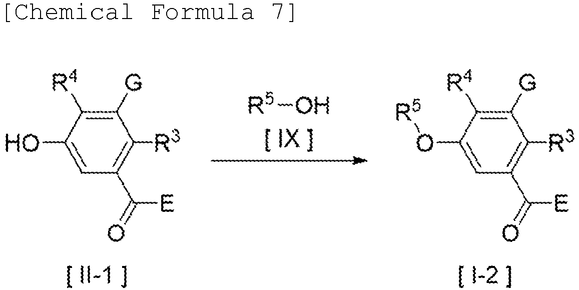 (In the formula, G, R³, R⁴, R⁵, and E are as defined above.)

That is, the compound of the present invention of formula [I-2] can be produced by reacting a compound [II-1] and a compound [IX] in an appropriate solvent, in the presence of a trisubstituted phosphine and an azodicarboxylic acid derivative, or in the presence of phosphorane.

The amount of use of the compound [IX] used in the current reaction may be appropriately selected from the range of 1.0 to 5.0 mol, and is preferably 1.0 to 3.0 mol per 1 mol of the compound [II-1].

Examples of the trisubstituted phosphine that can be used in the current reaction include triphenylphosphine, tributylphosphine, and trimethylphosphine. The amount of use of the trisubstituted phosphine may be appropriately selected from the range of 1.0 to 5.0 mol, and is preferably 1.0 to 3.0 mol per 1 mol of the compound [II-1].

Examples of the azodicarboxylic acid derivative that can be used in the current reaction include diethyl azodicarboxylate, diisopropyl azodicarboxylate, dimethoxyethyl azodicarboxylate, and N,N,N',N'-tetramethylazodicarboxamide. The amount of use of the azodicarboxylic acid derivative may be appropriately selected from the range of 1.0 to 5.0 mol, and is preferably 1.0 to 3.0 mol per 1 mol of the compound [II-1].

Examples of the phosphorane that can be used in the current reaction include cyanomethylenetrimethylphosphorane and cyanomethylenetributylphosphorane. The amount of use of the phosphorane may be appropriately selected from the range of 1.0 to 5.0 mol, and is preferably 1.0 to 3.0 mol per 1 mol of the compound [II-1].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; halogenated hydrocarbons such as dichloromethane, chloroform, and 1,2-dichloroethane; aliphatic hydrocarbons such as pentane, hexane, cyclohexane, and heptane; ketones such as acetone, methyl ethyl ketone, and cyclohexanone; acetic acid, water, and a mixed solvent thereof. The amount of use of the solvent is 0.1 to 100 liters, and is preferably 0.3 to 15 liters per 1 mol of the compound [II-1].

The reaction temperature of the current reaction may be selected from an arbitrary temperature range usually from - 30°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 24 hours.

After completion of the reaction, the compound [I-2] can be isolated by conducting an operation of concentrating the reaction mixture as it is, or pouring the reaction mixture into water or the like and subjecting the mixture to extraction with an organic solvent, followed by concentration.

The isolated compound [I-2] also can be further purified by column chromatography, recrystallization, or the like as necessary.

### <Production method 5>

A compound of formula [I-4] among the compounds of the present invention can be produced using a compound of formula [I-3] according to a method based on the reaction scheme exemplified below. 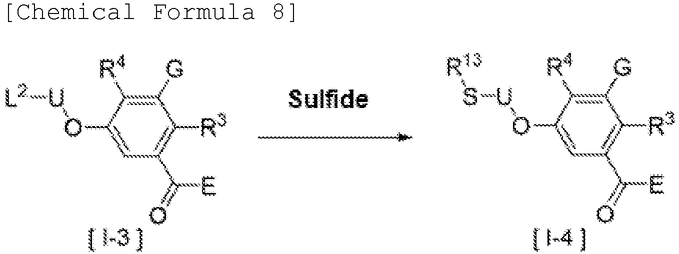 (In the formula, R¹³ represents a hydrogen atom; a cyano; a (C1-C6)alkyl; a (C1-C6)haloalkyl; a (C2-C6)alkenyl; a (C2-C6)haloalkenyl; a (C3-C6)cycloalkyl (C1-C6)alkyl; a (C3-C6)halocycloalkyl (C1-C6)alkyl; a (C3-C6)cycloalkyl; a (C3-C6)halocycloalkyl; a tri(C₁-C₆ alkyl)silyl (C1-C6)alkyl; or a phenyl, U represents (C1-C12)alkylene; (C3-C6)cycloalkylene; (C2-C6)alkenylene; (C1-C6)alkyleneoxy (C1-C12)alkylene; or (C2-C6)alkynylene, and G, L², R³, R⁴, and E are as defined above.)

That is, the compound of formula [I-4] can be produced by reacting a compound [I-3] and a sulfide in an appropriate solvent, in the presence or absence of an appropriate base, in the presence or absence of an appropriate catalyst.

Examples of the sulfide that can be used in the current reaction include hydrosulfides of alkali metals, such as sodium hydrosulfide and potassium hydrosulfide; thiocyanates such as sodium thiocyanate, potassium thiocyanate, and ammonium thiocyanate; alkyl mercaptans such as methyl mercaptan, ethyl mercaptan, and tert-butyl mercaptan; haloalkyl mercaptans such as 2,2,2-trifluoroethyl mercaptan; cycloalkylalkyl mercaptans such as cyclopropylmethyl mercaptan; and thioureas such as thiourea. The amount of use of the sulfide may be appropriately selected from the range of 1.0 to 10.0 mol, and is preferably 1.0 to 5.0 mol per 1 mol of the compound [I-3].

Examples of the base that can be used in the current reaction include inorganic bases such as hydroxides of alkali metals, such as sodium hydroxide and potassium hydroxide; hydroxides of alkaline earth metals, such as calcium hydroxide and magnesium hydroxide; carbonates of alkali metals, such as sodium carbonate and potassium carbonate; and alkali metal bicarbonates such as sodium bicarbonate and potassium bicarbonate; metal hydrides such as sodium hydride and potassium hydride; metal salts of alcohols, such as sodium methoxide, sodium ethoxide, potassium tert-butoxide; and organic bases such as triethylamine, 4-methylmorpholine, N-ethyldiisopropylamine, 1,8-diazabicyclo[5.4.0]-7-undecene, pyridine, 4-(N,N-dimethylamino)pyridine, and 2, 6-lutidine. The amount of use of the base may be appropriately selected from the range of 1.0 to 5.0 mol, and is preferably 1.0 to 2.0 mol per 1 mol of the compound [I-3].

Examples of the catalyst that can be used in the current reaction include sodium iodide and potassium iodide. The amount of use of the catalyst may be appropriately selected from the range of 0.01 to 1.0 mol, and is preferably 0.1 to 0.5 mol per 1 mol of the compound [I-3].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; nitriles such as acetonitrile and propionitrile; aliphatic hydrocarbons such as pentane, hexane, cyclohexane, and heptane; pyridines such as pyridine and picoline; and a mixed solvent thereof. The amount of use of the solvent is 0.1 to 100 liters, and is preferably 0.3 to 15 liters per 1 mol of the compound [I-3].

The reaction temperature of the current reaction may be selected from an arbitrary temperature range usually from 0°C to the reflux temperature in the reaction system, and is preferably in the range of room temperature to 150°C.

The reaction time of the current reaction varies depending on the reaction temperature, the reaction substrate, the reaction amount, and the like, but is usually 10 minutes to 96 hours.

After completion of the reaction, the compound [I-4] can be isolated by conducting an operation of concentrating the reaction mixture as it is, or pouring the reaction mixture into water or the like and subjecting the mixture to extraction with an organic solvent, followed by concentration.

The isolated compound [I-4] also can be further purified by column chromatography, recrystallization, or the like as necessary.

### <Production method 6>

A compound of formula [I-6] among the compounds of the present invention can be produced using a compound of formula [I-5] according to a method based on the reaction scheme exemplified below. 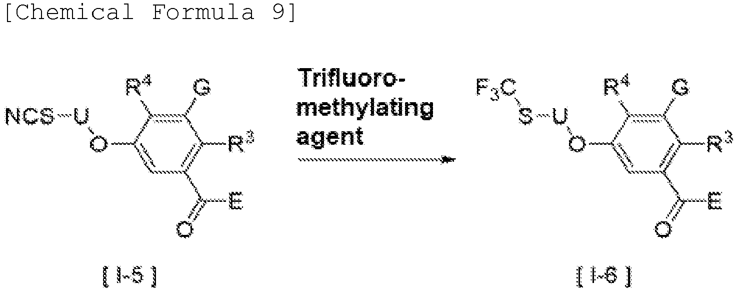 (In the formula, G, R³, R⁴, E, and U are as defined above.)

That is, the compound of formula [I-6] can be produced by reacting a compound [I-5] and a trifluoromethylating agent in an appropriate solvent in the presence of an appropriate catalyst.

Examples of the trifluoromethylating agent used in the current reaction include (trifluoromethyl)trimethylsilane, triethyl(trifluoromethyl)silane, and the trifluoromethylating agent described in WO 2015/199109. The amount of use of the trifluoromethylating agent may be appropriately selected from the range of 1.0 to 10.0 mol, and is preferably 1.0 to 5.0 mol per 1 mol of the compound [I-5].

Examples of the catalyst that can be used in the current reaction include tetra-n-butylammonium fluoride, cesium fluoride, and potassium fluoride. The amount of use of the catalyst may be appropriately selected from the range of 0.01 to 1.0 mol, and is preferably 0.05 to 0.3 mol per 1 mol of the compound [I-5].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; halogenated hydrocarbons such as dichloromethane, chloroform, and 1,2-dichloroethane; nitriles such as acetonitrile and propionitrile; aliphatic hydrocarbons such as pentane, hexane, cyclohexane, and heptane; and a mixed solvent thereof. The amount of use of the solvent is 0.1 to 100 liters, and is preferably 0.3 to 15 liters per 1 mol of the compound [I-5].

The reaction temperature of the current reaction may be selected from an arbitrary temperature range usually from - 30°C to the reflux temperature in the reaction system, and is preferably in the range of 0°C to room temperature.

The reaction time of the current reaction varies depending on the reaction temperature, the reaction substrate, the reaction amount, and the like, but is usually 10 minutes to 24 hours.

After completion of the reaction, the compound [I-6] can be isolated by conducting an operation of concentrating the reaction mixture as it is, or pouring the reaction mixture into water or the like and subjecting the mixture to extraction with an organic solvent, followed by concentration.

The isolated compound [I-6] also can be further purified by column chromatography, recrystallization, or the like as necessary.

### <Production method 7>

A compound of formula [I-8] among the compounds of the present invention can be produced using a compound of formula [I-7] according to a method based on the reaction scheme exemplified below. 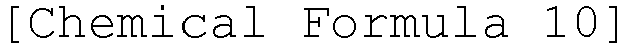 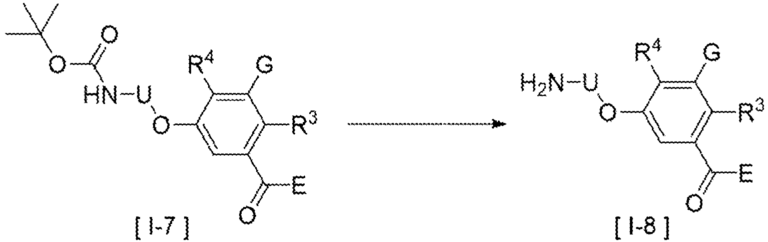 (In the formula, G, R³, R⁴, E, and U are as defined above.)

That is, the compound of formula [I-8] can be produced by reacting a compound [I-7] in an appropriate solvent, in the presence of an appropriate acid, under conditions used for deprotection of a tert-butoxycarbonyl group, for example, according to the method described in GREEN'S PROTECTIVE GROUPS in Organic Synthesis; the 5th edition (John Wiley and Sons, 2014, Peter G. M. Wuts).

### <Production method 8>

A compound of formula [I-9] among the compounds of the present invention can be produced using a compound of formula [I-8] according to a method based on the reaction scheme exemplified below. 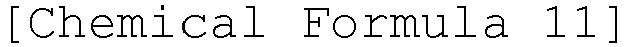 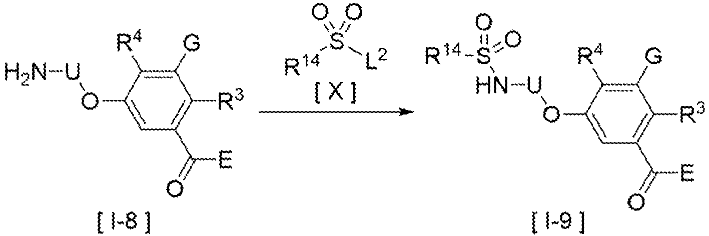 [In the formula, R¹⁴ represents a (C1-C6)alkyl; a (C1-C6)haloalkyl; or a phenyl (the phenyl is optionally monosubstituted or poly-substituted with R⁷), and G, L², R³, R⁴, R⁷, E, and U are as defined above.]

That is, the compound of formula [I-9] can be produced by reacting a compound [I-8] and a compound [X] in an appropriate solvent, in the presence of an appropriate base, in the presence or absence of an appropriate additive.

The amount of use of the compound [X] used in the current reaction may be appropriately selected from the range of 1.0 to 5.0 mol, and is preferably 1.0 to 2.0 mol per 1 mol of the compound [I-8].

Examples of the base that can be used in the current reaction include inorganic bases such as hydroxides of alkali metals, such as lithium hydroxide, sodium hydroxide, and potassium hydroxide; hydroxides of alkaline earth metals, such as calcium hydroxide and magnesium hydroxide; carbonates of alkali metals, such as sodium carbonate and potassium carbonate; and alkali metal bicarbonates such as sodium bicarbonate and potassium bicarbonate; and organic bases such as triethylamine, 4-methylmorpholine, N-ethyldiisopropylamine, 1,8-diazabicyclo[5.4.0]-7-undecene, pyridine, 4-(N,N-dimethylamino)pyridine, and 2,6-lutidine. The amount of use of the base may be appropriately selected from the range of 1.0 to 10.0 mol, and is preferably 1.0 to 3.0 mol per 1 mol of the compound [I-8].

When an additive is used in the current reaction, examples of the additive that can be used include molecular sieve 3A, molecular sieve 4A, and molecular sieve 5A.

The amount of use of the additive may be appropriately selected from the range of 0.01 to 10 g, and is preferably 0.1 to 1.0 g per 1 g of the compound [I-8].

Examples of the solvent that can be used in the current reaction include ethers such as diethyl ether, cyclopentyl methyl ether, tetrahydrofuran, 2-methyltetrahydrofuran, and 1,4-dioxane; halogenated hydrocarbons such as dichloromethane, chloroform, and 1,2-dichloroethane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; nitriles such as acetonitrile and propionitrile; aliphatic hydrocarbons such as pentane, hexane, cyclohexane, and heptane; and a mixed solvent thereof. The amount of use of the solvent is 0.1 to 100 liters, and is preferably 0.3 to 30 liters per 1 mol of the compound [I-8].

The reaction temperature of the current reaction may be selected from an arbitrary temperature range usually from - 70°C to the reflux temperature in the reaction system, and is preferably in the range of -20°C to room temperature.

The reaction time of the current reaction varies depending on the reaction temperature, the reaction substrate, the reaction amount, and the like, but is usually 10 minutes to 24 hours.

After completion of the reaction, the compound [I-9] can be isolated by conducting an operation of filtering off an unnecessary substance, concentrating the resulting substance as necessary, and then subjecting the concentrated resultant to extraction with an organic solvent, or pouring the reaction mixture into water or the like and subjecting the mixture with an organic solvent, followed by concentration. The isolated compound [I-9] also can be further purified by column chromatography, recrystallization, or the like as necessary.

### <Production method 9>

A compound of formula [I-10] among the compounds of the present invention can be produced using a compound of formula [I-9] according to a method based on the reaction scheme exemplified below. 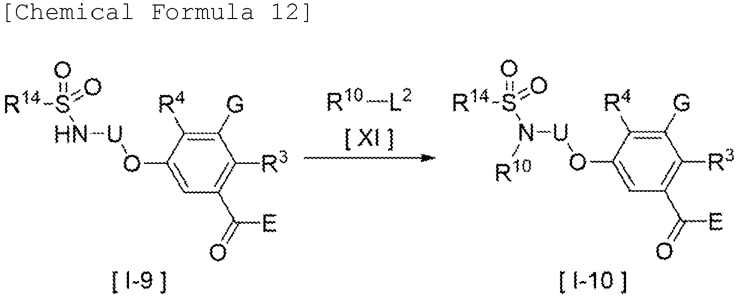 (In the formula, G, L², R³, R⁴, R¹⁰, R¹⁴, E, and U are as defined above.)

That is, the compound of formula [I-10] can be produced by reacting a compound [I-9] with a compound [XI] or di-tert-butyl dicarbonate in an appropriate solvent in the presence of an appropriate base.

The amount of use of the compound [XI] or di-tert-butyl dicarbonate used in the current reaction may be appropriately selected from the range of 1.0 to 5.0 mol, and is preferably 1.0 to 2.0 mol per 1 mol of the compound [I-9].

Examples of the base that can be used in the current reaction include inorganic bases such as hydroxides of alkali metals, such as sodium hydroxide and potassium hydroxide; hydroxides of alkaline earth metals, such as calcium hydroxide and magnesium hydroxide; carbonates of alkali metals, such as sodium carbonate and potassium carbonate; and alkali metal bicarbonates such as sodium bicarbonate and potassium bicarbonate; and organic bases such as triethylamine, 4-methylmorpholine, N-ethyldiisopropylamine, 1,8-diazabicyclo[5.4.0]-7-undecene, pyridine, 4-(N,N-dimethylamino)pyridine, and 2,6-lutidine. The amount of use of the base may be appropriately selected from the range of 1.0 to 10 mol, and is preferably 1.0 to 5.0 mol per 1 mol of the compound [I-9].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; halogenated hydrocarbons such as dichloromethane, chloroform, and 1,2-dichloroethane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; nitriles such as acetonitrile and propionitrile; esters such as ethyl acetate and ethyl propionate; aliphatic hydrocarbons such as pentane, hexane, cyclohexane, and heptane; pyridines such as pyridine and picoline; water, and a mixed solvent thereof. The amount of use of the solvent is 0.1 to 300 liters, and is preferably 0.1 to 100 liters per 1 mol of the compound [I-9].

The reaction temperature of the current reaction may be selected from an arbitrary temperature range usually from - 30°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 24 hours.

After completion of the reaction, the compound [I-10] can be isolated by conducting an operation of concentrating the reaction mixture as it is, or pouring the reaction mixture into water or the like and subjecting the mixture to extraction with an organic solvent, followed by concentration. The isolated compound [I-10] also can be further purified by column chromatography, recrystallization, or the like as necessary.

### <Production method 10>

A compound of formula [I-12] among the compounds of the present invention can be produced using a compound of formula [I-11] according to a method based on the reaction scheme exemplified below. 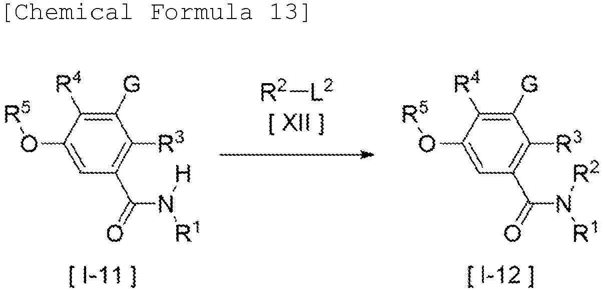 (In the formula, G, L², R¹, R², R³, R⁴, and R⁵ are as defined above.)

That is, the compound of formula [I-12] can be produced by reacting a compound [I-11] and a compound [XII] in an appropriate solvent in the presence of an appropriate base.

The amount of use of the compound [XII] used in the current reaction may be appropriately selected from the range of 1.0 to 30.0 mol, and is preferably 1.0 to 6.0 mol per 1 mol of the compound [I-11].

Examples of the base that can be used in the current reaction include inorganic bases such as hydroxides of alkali metals, such as sodium hydroxide and potassium hydroxide; hydroxides of alkaline earth metals, such as calcium hydroxide and magnesium hydroxide; carbonates of alkali metals, such as sodium carbonate and potassium carbonate; and alkali metal bicarbonates such as sodium bicarbonate and potassium bicarbonate; metal hydrides such as sodium hydride and potassium hydride; and metal salts of alcohols, such as sodium methoxide, sodium ethoxide, and potassium tert-butoxide. The amount of use of the base may be appropriately selected from the range of 1.0 to 10.0 mol, and is preferably 1.0 to 5.0 mol per 1 mol of the compound [I-11].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; halogenated hydrocarbons such as dichloromethane, chloroform, and 1,2-dichloroethane; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; nitriles such as acetonitrile and propionitrile; esters such as ethyl acetate, butyl acetate, and ethyl propionate; aliphatic hydrocarbons such as pentane, hexane, cyclohexane, and heptane; pyridines such as pyridine and picoline; water, and a mixed solvent thereof. The amount of use of the solvent is 0.1 to 100 liters, and is preferably 0.1 to 25 liters per 1 mol of the compound [I-11].

The reaction temperature of the current reaction may be selected from an arbitrary temperature range usually from - 30°C to the reflux temperature in the reaction system, and is preferably in the range of -10°C to 150°C.

The reaction time of the current reaction varies depending on the reaction temperature, the reaction substrate, the reaction amount, and the like, but is usually 10 minutes to 24 hours.

After completion of the reaction, the compound [I-12] can be isolated by conducting an operation of pouring the reaction mixture into water or the like and subjecting the mixture to extraction with an organic solvent, followed by concentration. The isolated compound [I-12] also can be further purified by column chromatography, recrystallization, or the like as necessary.

### <Production method 11>

A compound of formula [I-13] among the compounds of the present invention can be produced using a compound of formula [I-1] according to a method based on the reaction scheme exemplified below. 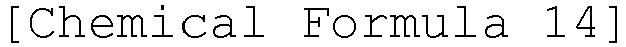 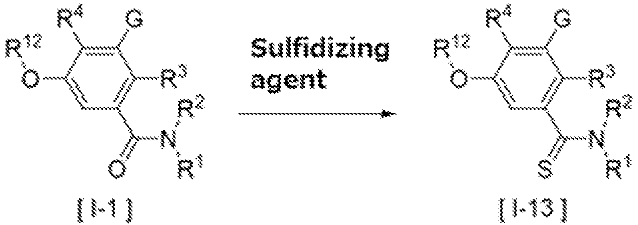 (In the formula, G, R¹, R², R³, R⁴, and R¹² are as defined above.)

That is, the compound of formula [I-13] can be produced by reacting a compound [I-1] and an appropriate sulfidizing agent in an appropriate solvent in the presence or absence of an appropriate base.

Examples of the sulfurizing agent that can be used in the current reaction include phosphorus pentasulfide and a Lawesson's reagent (2,4-bis(4-methoxyphenyl)-1,3,2,4-dithiadiphosphetane-2,4-disulfide). The amount of use of the sulfurizing agent may be appropriately selected from the range of 0.5 to 30.0 mol, and is preferably 0.5 to 5.0 mol per 1 mol of the compound [I-1].

Although addition of a base is not necessarily required in the current reaction, when the base is used, examples of the base that can be used include inorganic bases such as carbonates of alkali metals, such as sodium carbonate and potassium carbonate; and organic bases such as triethylamine, 4-methylmorpholine, N-ethyldiisopropylamine, 1,8-diazabicyclo[5.4.0]-7-undecene, pyridine, 4-(N,N-dimethylamino)pyridine, and 2, 6-lutidine. The amount of use of the base may be appropriately selected from the range of 0.01 to 10.0 mol, and is preferably 0.1 to 2.0 mol per 1 mol of the compound [I-1].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; halogenated hydrocarbons such as dichloromethane, chloroform, and 1,2-dichloroethane; aliphatic hydrocarbons such as pentane, hexane, cyclohexane, and heptane; water, and a mixed solvent thereof. The amount of use of the solvent is 0.1 to 300 liters, and is preferably 0.3 to 50 liters per 1 mol of the compound [I-1].

The reaction temperature of the current reaction may be selected from an arbitrary temperature range usually from - 70°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 24 hours.

After completion of the reaction, the compound [I-13] can be isolated by conducting an operation of filtering off an insoluble substance from the reaction mixture and concentrating the resulting substance, or pouring the reaction mixture into water and subjecting the mixture to extraction with an organic solvent, followed by concentration. Alternatively, the compound [I-13] can also be isolated from the reaction mixture by concentrating the solvent. The isolated compound [I-13] also can be further purified by column chromatography, recrystallization, or the like as necessary.

### <Production method 12>

A compound of formula [II-1] among the compounds of the present invention can be produced using a compound of formula [XIII] according to a method based on the reaction scheme exemplified below. 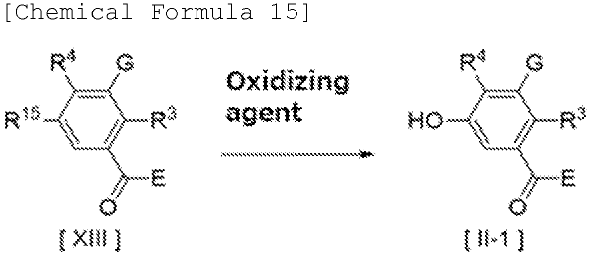 (In the formula, R¹⁵ represents a dihydroxyboryl (-B(OH)₂) or a pinacolato borane-2-yl, and G, R³, R⁴, and E are as defined above.)

That is, the compound of formula [II-1] can be produced by reacting a compound [XIII] with an appropriate oxidizing agent in an appropriate solvent.

Examples of the oxidizing agent that can be used in the current reaction include hydrogen peroxide and 4-methylmorpholine-N-oxide. The amount of use of the oxidizing agent may be appropriately selected from the range of 1.0 to 20.0 mol, and is preferably 1.0 to 6.0 mol per 1 mol of the compound [XIII].

Examples of the solvent that can be used in the current reaction include aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; halogenated hydrocarbons such as dichloromethane, chloroform, and 1,2-dichloroethane; aliphatic hydrocarbons such as pentane, hexane, cyclohexane, and heptane; ketones such as acetone, methyl ethyl ketone, and cyclohexanone; acetic acid, water, and a mixed solvent thereof. The amount of use of the solvent is 0.1 to 100 liters, and is preferably 0.3 to 15 liters per 1 mol of the compound [XIII].

The reaction temperature of the current reaction may be selected from an arbitrary temperature range from -30°C to the reflux temperature in the reaction system, and is preferably in the range of -10°C to 150°C.

The reaction time of the current reaction varies depending on the reaction temperature, the reaction substrate, the reaction amount, and the like, but is usually 10 minutes to 24 hours.

After completion of the reaction, the compound [II-1] can be isolated by conducting an operation of pouring the reaction mixture into water or the like and subjecting the mixture to extraction with an organic solvent, followed by concentration. The isolated compound [II-1] also can be further purified by column chromatography, recrystallization, or the like as necessary.

### <Production method 13>

A compound of formula [I-15] among the compounds of the present invention can be produced according to a method based on the reaction scheme exemplified below. 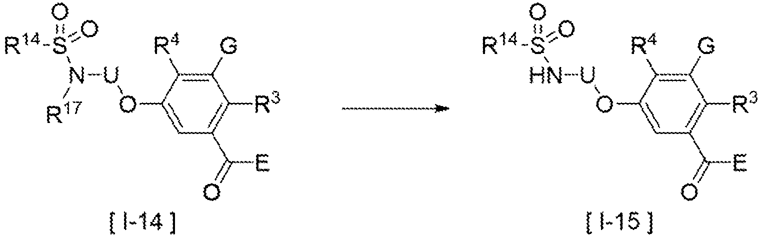 (In the formula, R¹⁷ represents a (C1-C6)alkoxymethyl or a tert-butoxycarbonyl, and E, G, R³, R⁴, R¹⁴ and U are as defined above.)

That is, the compound of formula [I-15] can be produced by reacting a compound [I-14] in an appropriate solvent in the presence of an appropriate acid.

Examples of the acid that can be used in the current reaction include mineral acids such as hydrochloric acid, hydrobromic acid, and sulfuric acid; carboxylic acids such as acetic acid, propionic acid, and trifluoroacetic acid; and sulfonic acids such as methanesulfonic acid and trifluoromethanesulfonic acid. The amount of use of the acid may be appropriately selected from the range of 0.1 to moles corresponding to the amount of the solvent, and is preferably 1.0 to 100.0 moles per 1 mole of the compound [I-14].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; alcohols such as methanol, ethanol, and 2-propanol; halogenated hydrocarbons such as dichloromethane, chloroform, and 1,2-dichloroethane; aliphatic hydrocarbons such as pentane, hexane, cyclohexane, and heptane; ketones such as acetone, methyl ethyl ketone, and cyclohexanone; water, and a mixed solvent thereof. The amount of use of the solvent is 0.1 to 500 liters, and is preferably 0.3 to 30 liters per 1 mol of the compound [I-14].

The reaction temperature of the current reaction may be selected from an arbitrary temperature range usually from - 30°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 72 hours.

After completion of the current reaction, the compound [I-15] can be isolated by conducting an operation of concentrating the reaction mixture as it is, or pouring the reaction mixture into water or the like and subjecting the mixture to extraction with an organic solvent, followed by concentration. The isolated compound [I-15] also can be further purified by column chromatography, recrystallization, or the like as necessary.

### <Production method 1 of intermediate>

A compound of formula [XVI] can be produced using a compound of formula [XIV] according to a method based on the reaction scheme exemplified below. 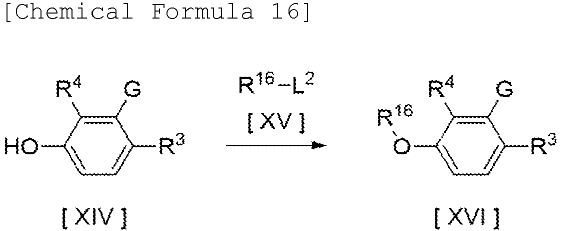 (In the formula, R¹⁶ represents a protecting group such as a (C1-C6)alkylcarbonyl, a (C1-C6)alkoxycarbonyl, a methanesulfonyl, a trifluoromethanesulfonyl, a nonafluorobutanesulfonyl, a benzenesulfonyl, or a p-toluenesulfonyl, and G, L², R³, and R⁴ are as defined above.)

That is, the compound of formula [XVI] can be produced by reacting a compound [XIV] with a compound [XV] in an appropriate solvent, in the presence of an appropriate base, under conditions used for protecting a phenolic hydroxyl group, for example, according to the method described in GREEN'S PROTECTIVE GROUPS in Organic Synthesis; the 5th edition (John Wiley and Sons, 2014, Peter G. M. Wuts).

### <Production method 2 of intermediate>

A compound of formula [XVII] can be produced using a compound of formula [XVI] according to a method based on the reaction scheme exemplified below. 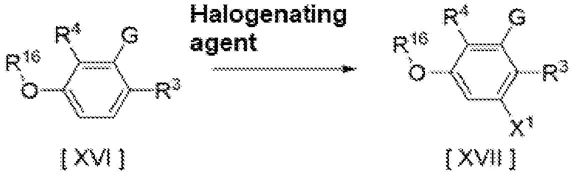 (In the formula, G, X¹, R³, R⁴, and R¹⁶ are as defined above.)

That is, the compound of formula [XVII] can be produced by reacting a compound [XVI] with a halogenating agent in an appropriate solvent.

Examples of the halogenating agent that can be used in the current reaction include chlorine, sulfuryl chloride, N-chlorosuccinimide, bromine, N-bromosuccinimide, 1,3-dibromo-5,5-dimethylhydantoin, iodine, N-iodosuccinimide, 1,3-diiodo-5,5-dimethylhydantoin, potassium iodate, and a mixture thereof. The amount of use of the halogenating agent may be appropriately selected from the range of 0.01 to 10.0 mol, and is preferably 0.02 to 2.0 mol per 1 mol of the compound [XVI].

Examples of the solvent that can be used in the current reaction include halogenated hydrocarbons such as dichloromethane, chloroform, and 1,2-dichloroethane; aliphatic hydrocarbons such as pentane, hexane, cyclohexane, and heptane; nitriles such as acetonitrile and propionitrile; carboxylic acids such as acetic acid, propionic acid, and trifluoroacetic acid; sulfuric acid, water, and a mixed solvent thereof. The amount of use of the solvent is 0.1 to 300 liters, and is preferably 0.3 to 20 liters per 1 mol of the compound [XVI].

The reaction temperature of the current reaction may be selected from an arbitrary temperature range usually from - 70°C to the reflux temperature in the reaction system, and is preferably in the range of -20°C to 100°C.

The reaction time of the current reaction varies depending on the reaction temperature, the reaction substrate, the reaction amount, and the like, but is usually 10 minutes to 24 hours.

After completion of the reaction, the compound [XVII] can be isolated by conducting an operation of pouring the reaction mixture into water and collecting the precipitated solid by filtration, or subjecting the reaction mixture to extraction with an organic solvent, followed by concentration. The isolated compound [XVII] also can be further purified by column chromatography, recrystallization, or the like as necessary.

### <Production method 3 of intermediate>

A compound of formula [XVIII] can be produced using a compound of formula [XVII] according to a method based on the reaction scheme exemplified below. 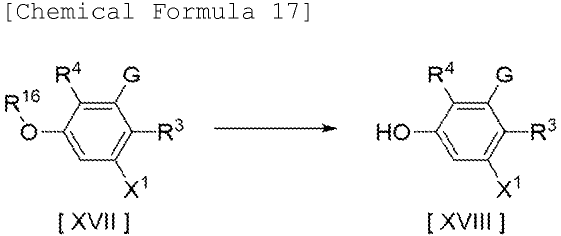 (In the formula, G, X¹, R³, R⁴, and R¹⁶ are as defined above.)

That is, the compound of formula [XVIII] can be produced by hydrolyzing a compound [XVII] in an appropriate solvent in the presence of an appropriate acid or base.

The acid, base, and solvent that can be used in the current reaction, the reaction temperature, and the reaction time are the same as those in Process 1-2 of the production method 1.

After completion of the current reaction, the compound [XVIII] can be isolated by conducting an operation of pouring the reaction mixture into water to neutralize the mixture and collecting the precipitated solid by filtration, or subjecting the reaction mixture to extraction with an organic solvent, followed by concentration. The isolated compound [XVIII] also can be further purified by column chromatography, recrystallization, or the like as necessary.

### <Production method 4 of intermediate>

A compound of formula [XIX] can be produced using a compound of formula [XVIII] according to a method based on the reaction scheme exemplified below. 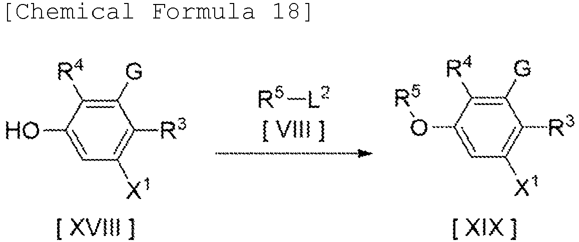 (In the formula, G, L², R³, R⁴, R⁵ and X¹ are as defined above.)

That is, the compound of formula [XIX] can be produced by reacting a compound [XVIII] and a compound [VIII] in an appropriate solvent, in the presence of an appropriate base, in the presence or absence of an appropriate catalyst.

The base and catalyst used in the current reaction, the solvent that can be used in the reaction, the reaction temperature, and the reaction time are the same as those in the production method 3.

After completion of the reaction, the compound [XIX] can be isolated by conducting an operation of pouring the reaction mixture into water and collecting the precipitated solid by filtration, or subjecting the reaction mixture to extraction with an organic solvent, followed by concentration. The isolated compound [XIX] also can be further purified by column chromatography, recrystallization, or the like as necessary.

### <Production method 5 of intermediate>

A compound of formula [XIX] can be produced using a compound of formula [XVIII] according to a method based on the reaction scheme exemplified below. 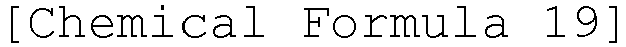 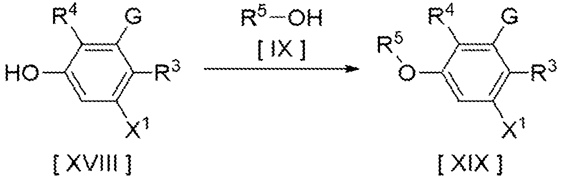 (In the formula, G, R³, R⁴, R⁵, and X¹ are as defined above.)

That is, the compound of formula [XIX] can be produced by reacting a compound [XVIII] and a compound [IX] in an appropriate solvent, in the presence of a trisubstituted phosphine and an azodicarboxylic acid derivative, or in the presence of phosphorane.

The trisubstituted phosphine, azodicarboxylic acid derivative, and phosphorane used in the current reaction, the solvent that can be used in the reaction, the reaction temperature, and the reaction time are the same as those in the production method 4.

After completion of the reaction, the compound [XIX] can be isolated by conducting an operation of pouring the reaction mixture into water and collecting the precipitated solid by filtration, or subjecting the reaction mixture to extraction with an organic solvent, followed by concentration. The isolated compound [XIX] also can be further purified by column chromatography, recrystallization, or the like as necessary.

### <Production method 6 of intermediate>

A compound of formula [XXI] can be produced using a compound of formula [XX] according to a method based on the reaction scheme exemplified below. 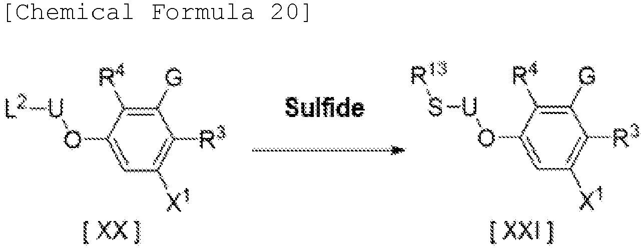 (In the formula, G, L², R³, R⁴, R¹³, U, and X¹ are as defined above.)

That is, the compound of formula [XXI] can be produced by reacting a compound [XX] and a sulfide in an appropriate solvent, in the presence or absence of an appropriate base, in the presence or absence of an appropriate catalyst.

The sulfide and base used in the current reaction, the solvent that can be used in the reaction, the reaction temperature, and the reaction time are the same as those in the production method 5.

After completion of the reaction, the compound [XXI] can be isolated by conducting an operation of pouring the reaction mixture into water and collecting the precipitated solid by filtration, or subjecting the reaction mixture to extraction with an organic solvent, followed by concentration. The isolated compound [XXI] also can be further purified by column chromatography, recrystallization, or the like as necessary.

### <Production method 7 of intermediate>

A compound of formula [XXIII] can be produced using a compound of formula [XXII] according to a method based on the reaction scheme exemplified below. 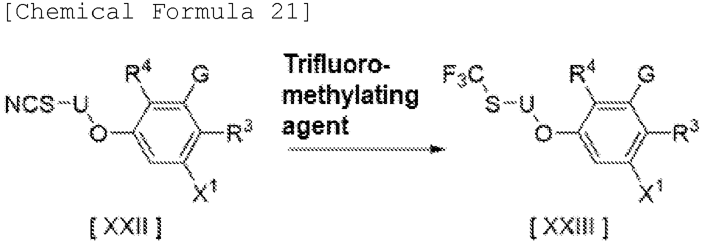 (In the formula, G, R³, R⁴, U, and X¹ are as defined above.)

That is, the compound of formula [XXIII] can be produced by reacting a compound [XXII] and a trifluoromethylating agent in an appropriate solvent in the presence of an appropriate catalyst.

The trifluoromethylating agent and catalyst used in the current reaction, the solvent that can be used in the reaction, the reaction temperature, and the reaction time are the same as those in the production method 6.

After completion of the reaction, the compound [XXIII] can be isolated by conducting an operation of pouring the reaction mixture into water and collecting the precipitated solid by filtration, or subjecting the reaction mixture to extraction with an organic solvent, followed by concentration. The isolated compound [XXIII] also can be further purified by column chromatography, recrystallization, or the like as necessary.

### <Production method 8 of intermediate>

A compound of formula [XXVI] can be produced using a compound of formula [XVII] according to a method based on the reaction scheme exemplified below. 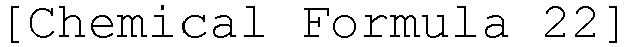 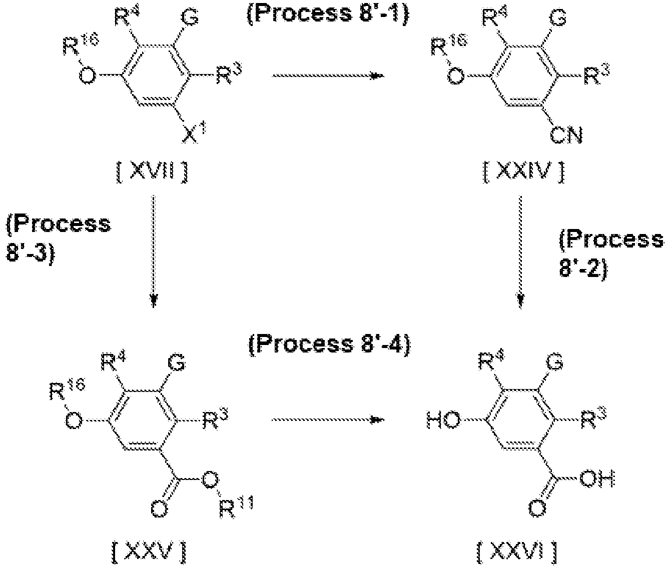 (In the formula, G, R³, R⁴, R¹¹, R¹⁶, and X¹ are as defined above.)

That is, the compound of formula [XXVI] can be produced by (Process 8'-1) converting a compound of formula [XVII] into a compound [XXIV] using a cyanide in an appropriate solvent in the presence or absence of an appropriate catalyst, and then (Process 8'-2) hydrolyzing the compound of formula [XXVI] in an appropriate solvent in the presence of an appropriate acid or base. In addition, the compound [XXVI] can be produced by (Process 8'-3) subjecting a compound of formula [XVII] to a carbon monoxide insertion reaction in an appropriate solvent in the presence of an appropriate catalyst, an appropriate base, and an appropriate alcohol to obtain a compound represented by [XXV] among the compounds of the present invention, and then (Process 8'-4) hydrolyzing the compound of formula [XXV] in an appropriate solvent in the presence of an appropriate acid or base.

The cyanide, catalyst, acid, base, and alcohol used in the current reaction, the solvent that can be used in the reaction, the reaction temperature, and the reaction time are the same as those in the production method 1.

After completion of the reaction, the compound [XXVI] can be isolated by conducting an operation of pouring the reaction mixture into water and collecting the precipitated solid by filtration, or subjecting the reaction mixture to extraction with an organic solvent, followed by concentration. The isolated compound [XXVI] also can be further purified by column chromatography, recrystallization, or the like as necessary.

### <Production method 9 of intermediate>

A compound of formula [XXVIII] can be produced using a compound of formula [XXVII] according to a method based on the reaction scheme exemplified below. 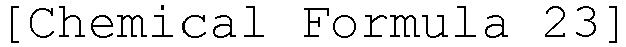 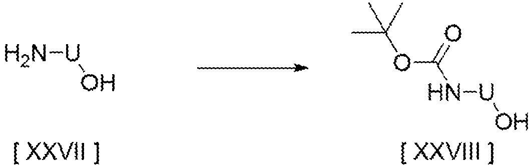 (In the formula, U is as defined above.)

That is, the compound of formula [XXVIII] can be produced by reacting a compound [XXVII] with an appropriate acid anhydride, in an appropriate solvent, in the presence of an appropriate base, under conditions used for protection of an amino group by a tert-butoxycarbonyl group, for example, according to the method described in GREEN'S PROTECTIVE GROUPS in Organic Synthesis; the 5th edition (John Wiley and Sons, 2014, Peter G. M. Wuts).

### <Production method 10 of intermediate>

A compound of formula [XXX] can be produced using a compound of formula [XXIX] according to a method based on the reaction scheme exemplified below. 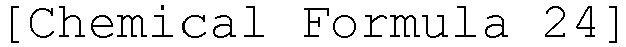 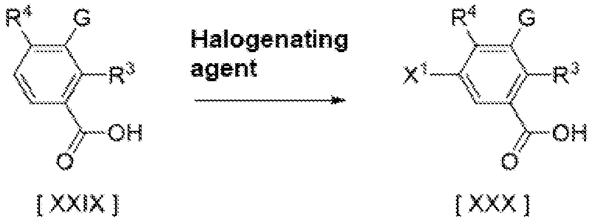 (In the formula, G, R³, R⁴, and X¹ are as defined above.)

That is, the compound of formula [XXX] can be produced by reacting a compound [XXIX] with a halogenating agent in an appropriate solvent.

The halogenating agent used in the current reaction, the solvent that can be used in the reaction, the reaction temperature, and the reaction time are the same as those in the production method 2 of intermediate.

After completion of the reaction, the compound [XXX] can be isolated by conducting an operation of pouring the reaction mixture into water and collecting the precipitated solid by filtration, or subjecting the reaction mixture to extraction with an organic solvent, followed by concentration. The isolated compound [XXX] also can be further purified by column chromatography, recrystallization, or the like as necessary.

### <Production method 11 of intermediate>

A compound of formula [XXXII] can be produced using a compound of formula [XXX] according to a method based on the reaction scheme exemplified below. 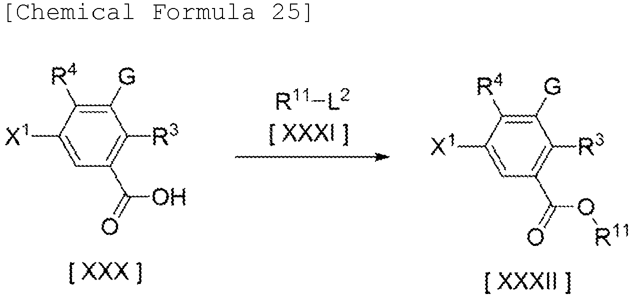 (In the formula, G, L², R³, R⁴, R¹¹, and X¹ are as defined above.)

That is, the compound of formula [XXXII] can be produced by reacting a compound [XXX] and a compound [XXXI] in an appropriate solvent, in the presence of an appropriate base, in the presence or absence of an appropriate catalyst.

The base and catalyst used in the current reaction, the solvent that can be used in the reaction, the reaction temperature, and the reaction time are the same as those in the production method 3.

After completion of the reaction, the compound [XXXII] can be isolated by conducting an operation of pouring the reaction mixture into water and collecting the precipitated solid by filtration, or subjecting the reaction mixture to extraction with an organic solvent, followed by concentration. The isolated compound [XXXII] also can be further purified by column chromatography, recrystallization, or the like as necessary.

### <Production method 12 of intermediate>

A compound of formula [XXXIV] can be produced using a compound of formula [XXXII] according to a method based on the reaction scheme exemplified below. 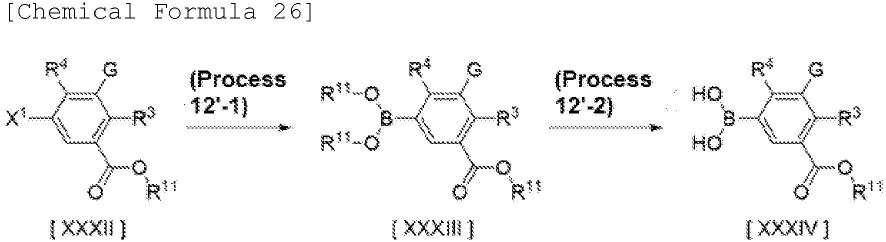 (In the formula, G, R³, R⁴, R¹¹, and X¹ are as defined above.)

That is, the compound of formula [XXXIV] can be produced by (Process 12'-1) reacting a compound [XXXII] with an appropriate organometallic compound in an appropriate solvent, then reacting the resultant with an borate ester to obtain a compound [XXXIII], and (Process 12'-2) reacting the compound [XXXIII] with an acid in an appropriate solvent.

### (Process 12'-1)

Examples of the organometallic compound that can be used in the current reaction include isopropylmagnesium chloride. The amount of use of the organometallic compound may be appropriately selected from the range of 1.0 to 3.0 mol, and is preferably 1.0 to 1.2 mol per 1 mol of the compound [XXXII].

Examples of the borate ester that can be used in the current reaction include trimethyl borate, triethyl borate, triisopropyl borate, and tributyl borate. The amount of use of the borate ester may be appropriately selected from the range of 1.0 to 3.0 mol, and is preferably 1.0 to 1.2 mol per 1 mol of the compound [XXXII].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aliphatic hydrocarbons such as pentane, hexane, cyclohexane, and heptane; pyridines such as pyridine and picoline; and a mixed solvent thereof. The amount of use of the solvent is 0.1 to 100 liters, and is preferably 0.1 to 5 liters per 1 mol of the compound [XXXII].

The reaction temperature of the current reaction may be selected from an arbitrary range usually from -100°C to the reflux temperature in the reaction system, and is preferably in the range of -80°C to room temperature.

The reaction time of the current reaction varies depending on the reaction temperature, the reaction substrate, the reaction amount, and the like, but is usually 5 minutes to 24 hours.

After completion of the reaction, the compound [XXXIII] can be used as it is for the next reaction without isolation.

### (Process 12'-2)

Examples of the acid that can be used in the current reaction include mineral acids such as sulfuric acid and hydrochloric acid. The amount of use of the acid may be appropriately selected from the range of 1.0 to 5.0 mol, and is preferably 0.5 to 2.0 mol per 1 mol of the compound [XXXIII].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; halogenated hydrocarbons such as dichloromethane, chloroform, and 1,2-dichloroethane; alcohols such as methanol, ethanol, and 2-propanol; aliphatic hydrocarbons such as pentane, hexane, cyclohexane, and heptane; ketones such as acetone, methyl ethyl ketone, and cyclohexanone; water, and a mixed solvent thereof. The amount of use of the solvent is 0.1 to 100 liters, and is preferably 0.1 to 5 liters per 1 mol of the compound [XXXIII].

The reaction temperature of the current reaction may be usually selected from an arbitrary range from 0°C to the reflux temperature in the reaction system, and is preferably in the range of 0°C to 100°C.

The reaction time of the current reaction varies depending on the reaction temperature, the reaction substrate, the reaction amount, and the like, but is usually 10 minutes to 24 hours.

After completion of the reaction, the compound [XXXIV] can be isolated by conducting an operation of pouring the reaction mixture into water and collecting the precipitated solid by filtration, or subjecting the reaction mixture to extraction with an organic solvent, followed by concentration. The isolated compound [XXXIV] also can be further purified by column chromatography, recrystallization, or the like as necessary.

### <Production method 13 of intermediate>

A compound of formula [XXXVI] can be produced using a compound of formula [XXXV] according to a method based on the reaction scheme exemplified below. 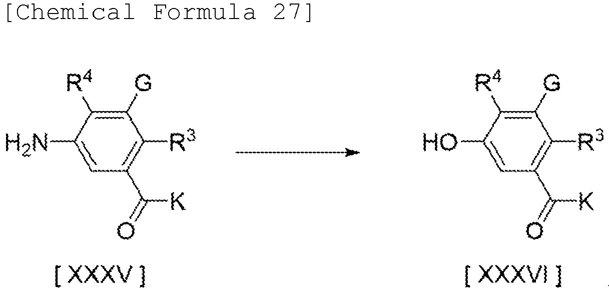 (In the formula, G, R³, R⁴, and K are as defined above.)

That is, the compound of formula [XXXVI] can be produced by reacting a compound [XXXV] with an acid and a nitrous acid derivative in an appropriate solvent, and then reacting the resultant with a metal salt as necessary.

Examples of the acid that can be used in the current reaction include mineral acids such as sulfuric acid and nitric acid, and organic acids such as trifluoroacetic acid and trifluoromethanesulfonic acid. The amount of use of the acid may be appropriately selected from the range of 1 to 20 mol, and is preferably 1.0 to 5.0 mol per 1 mol of the compound [XXXV]. However, when an acid is used as a solvent, the amount of use as the solvent is appropriately selected.

Examples of the nitrous acid derivative that can be used in the current reaction include nitrites such as sodium nitrite and potassium nitrite, and alkyl nitrites such as n-butyl nitrite, isopentyl nitrite, and tert-butyl nitrite. The amount of use of the nitrous acid derivative may be appropriately selected from the range of 1 to 3 mol, and is preferably 1.0 to 1.5 mol per 1 mol of the compound [XXXV].

Examples of the metal salt used as necessary in the current reaction include copper sulfate, copper nitrate, and copper oxide. The amount of use of the metal salt may be appropriately selected from the range of 0 to 2 mol, and is preferably 0 to 1.1 mol per 1 mol of the compound [XXXV].

Examples of the solvent that can be used in the current reaction include ethers such as diethyl ether, cyclopentyl methyl ether, tetrahydrofuran, 2-methyltetrahydrofuran, and 1,4-dioxane; aromatic hydrocarbons such as benzene, toluene, xylene, and chlorobenzene; aprotic polar solvents such as N,N-dimethylformamide, N,N-dimethylacetamide, N-methyl-2-pyrrolidone, dimethyl sulfoxide, sulfolane, and 1,3-dimethyl-2-imidazolidinone; halogenated hydrocarbons such as dichloromethane, chloroform, and 1,2-dichloroethane; alcohols such as methanol, ethanol, and 2-propanol; nitriles such as acetonitrile and propionitrile; esters such as ethyl acetate, butyl acetate, and ethyl propionate; aliphatic hydrocarbons such as pentane, hexane, cyclohexane, and heptane; pyridines such as pyridine and picoline; mineral acids such as acetic acid, hydrochloric acid, sulfuric acid, and nitric acid; organic acids such as trifluoroacetic acid and trifluoromethanesulfonic acid; water, and a mixed solvent thereof. The amount of use of the solvent is 0.1 to 100 liters, and is preferably 0.3 to 10 liters per 1 mol of the compound [XXXV].

The reaction temperature of the current reaction may be selected from an arbitrary range usually from -30°C to the reflux temperature in the reaction system, and is preferably in the range of 0°C to 150°C.

The reaction time of the current reaction varies depending on the reaction temperature, the reaction substrate, the reaction amount, and the like, but is usually 10 minutes to 24 hours.

After completion of the reaction, the compound [XXXVI] can be isolated by conducting an operation of pouring the reaction mixture into water or the like and collecting the precipitated solid by filtration, or subjecting the reaction mixture to extraction with an organic solvent, followed by concentration. The isolated compound [XXXVI] also can be further purified by column chromatography, recrystallization, or the like as necessary. Alternatively, the compound [XXXVI] can be directly used for the next reaction without isolation.

### <Production method 14 of intermediate>

A compound of formula [XXXVIII] can be produced using a compound of formula [XXXVII] according to a method based on the reaction scheme exemplified below. 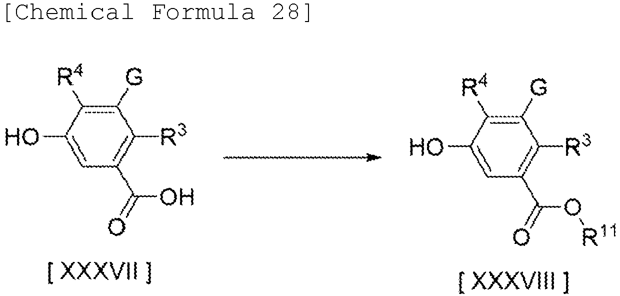 (In the formula, G, R³, R⁴, and R¹¹ are as defined above.)

That is, the compound of formula [XXXVIII] can be produced from a compound [XXXVII] under conditions used for esterification of a carboxy group, for example, according to the method described in GREEN'S PROTECTIVE GROUPS in Organic Synthesis; the 5th edition (John Wiley and Sons, 2014, Peter G. M. Wuts).

The hydroxybenzamide derivative of formula [II] of the present invention is a compound useful as an intermediate in the production of the 3-alkoxybenzamide derivative of formula [I] or agriculturally acceptable salt thereof according to the present invention.

The alkoxybenzoic acid derivative of formula [III] of the present invention or an ester thereof is a compound useful as an intermediate in the production of the 3-alkoxybenzamide derivative of formula [I] or agriculturally acceptable salt thereof according to the present invention.

The agrochemical composition of the present invention contains the 3-alkoxybenzamide derivative of formula [I] or agriculturally acceptable salt thereof according to the present invention as an active ingredient.

The agrochemical composition of the present invention can contain an additive component (carrier) usually used in agrochemical formulations as necessary.

The pest control agent of the present invention contains the 3-alkoxybenzamide derivative of formula [I] or agriculturally acceptable salt thereof according to the present invention as an active ingredient. The pest control agent of the present invention is typically an insecticide, an acaricide, and a nematicide.

The pest control agent of the present invention can contain an additive component (carrier) usually used in an agrochemical formulation as necessary.

Examples of the additive component include a carrier such as a solid carrier or a liquid carrier, a surfactant, a binder or a tackifier, a thickener, a colorant, a spreader, a sticker, an anti-freezing agent, an anti-caking agent, a disintegrant, and a degradation preventing agent. In addition, a preservative, a plant piece, or the like may be used as the additive component as necessary. These additive components may be used alone or in combination of two or more types thereof.

Examples of the solid carrier include mineral carriers such as pyrophyllite clay, kaolin clay, silica clay, talc, diatomaceous earth, zeolite, bentonite, acid clay, activated clay, attapulgus clay, vermiculite, perlite, pumice, white carbon (synthetic silicic acid, synthetic silicate, and the like), and titanium dioxide; plant carriers such as wood powder, cornstalks, walnut shells, fruit cores, chaff, sawdust, bran, soybean flour, powdered cellulose, starch, dextrin, and saccharides; inorganic salt carriers such as calcium carbonate, ammonium sulfate, sodium sulfate, or potassium chloride; polymer carriers such as polyethylene, polypropylene, polyvinyl chloride, polyvinyl acetate, an ethylene-vinyl acetate copolymer, and a urea-aldehyde resin.

Examples of the liquid carrier include monohydric alcohols such as methanol, ethanol, propanol, 2-propanol, butanol, and cyclohexanol; polyhydric alcohols such as ethylene glycol, diethylene glycol, propylene glycol, hexylene glycol, polyethylene glycol, polypropylene glycol, and glycerin; polyhydric alcohol derivatives such as propylene-based glycol ether; ketones such as acetone, methyl ethyl ketone, methyl isobutyl ketone, diisobutyl ketone, cyclohexanone, and isophorone; ethers such as ethyl ether, 1,4-dioxane, cellosolve, dipropyl ether, and tetrahydrofuran; aliphatic hydrocarbons such as normal paraffin, naphthene, isoparaffin, kerosene, and mineral oil; aromatic hydrocarbons such as toluene, C₉-C₁₀ alkylbenzene, xylene, solvent naphtha, alkylnaphthalene, and high-boiling-point aromatic hydrocarbon; halogenated hydrocarbons such as 1,2-dichloroethane, chloroform, and carbon tetrachloride; esters such as ethyl acetate, diisopropyl phthalate, dibutyl phthalate, dioctyl phthalate, and dimethyl adipate; lactones such as γ-butyrolactone; amides such as N,N-dimethylformamide, N,N-diethylformamide, N,N-dimethylacetamide, and N-methyl-2-pyrrolidone; nitriles such as acetonitrile; sulfur compounds such as dimethyl sulfoxide; vegetable oils such as soybean oil, rapeseed oil, cottonseed oil, coconut oil, and castor oil; lower alkyl esters of fatty acids derived from the vegetable oils; and water.

Examples of the surfactant include nonionic surfactants such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkylphenyl ether, polyoxyethylene dialkyl phenyl ether, polyoxyethylene alkyl phenyl ether formalin condensate, polyoxyethylene polyoxypropylene block polymer, alkyl polyoxyethylene polypropylene block polymer ether, polyoxyethylene alkylamine, polyoxyethylene fatty acid amide, polyoxyethylene fatty acid bisphenyl ether, polyalkylene benzylphenyl ether, polyoxyalkylene styrylphenyl ether, acetylene diol, polyoxyalkylene-added acetylene diol, polyoxyethylene ether-type silicone, ester-type silicone, fluorine-based surfactant, polyoxyethylene castor oil, and polyoxyethylene hydrogenated castor oil; anionic surfactants such as alkyl sulfates, polyoxyethylene alkyl ether sulfates, polyoxyethylene alkyl phenyl ether sulfates, polyoxyethylene styryl phenyl ether sulfates, alkylbenzene sulfonates, lignin sulfonates, alkyl sulfosuccinates, naphthalene sulfonates, alkyl naphthalene sulfonates, salts of formalin condensates of naphthalene sulfonates, salts of formalin condensates of alkyl naphthalene sulfonates, fatty acid salts, polycarboxylates, N-methyl-fatty acid sarcosinates, resin acid salts, polyoxyethylene alkyl ether phosphates, and polyoxyethylene alkyl phenyl ether phosphates; cationic surfactants including alkylamine salts such as laurylamine hydrochloride, stearylamine hydrochloride, oleylamine hydrochloride, stearylamine acetate, stearylaminopropylamine acetate, alkyltrimethylammonium chloride, and alkyldimethylbenzalkonium chloride; and amphoteric surfactants including betaine-type surfactants such as dialkyldiaminoethylbetaine and alkyldimethylbenzylbetaine, and amino acid-type surfactants such as dialkylaminoethylglycine and alkyldimethylbenzylglycine.

Examples of the binder and the tackifier include carboxymethyl cellulose and salts thereof, dextrin, water-soluble starch, xanthan gum, guar gum, sucrose, polyvinyl pyrrolidone, gum arabic, polyvinyl alcohol, polyvinyl acetate, sodium polyacrylate, polyethylene glycol, polyethylene oxide, and natural phospholipid (for example, cephalin acid, and lecithin).

Examples of the thickener include water-soluble polymers such as xanthan gum, guar gum, carboxymethyl cellulose, polyvinylpyrrolidone, carboxyvinyl polymers, acrylic polymers, starch derivatives, and polysaccharides; inorganic fine powders such as high-purity bentonite and white carbon; and organic fine powders such as organic bentonite.

Examples of the colorant include inorganic pigments such as iron oxide, titanium oxide, and Prussian blue; and organic dyes such as an alizarin dye, an azo dye, and a metal phthalocyanine dye.

Examples of the spreader include a silicone-based surfactant, cellulose powder, dextrin, processed starch, a polyaminocarboxylic acid chelate compound, crosslinked polyvinyl pyrrolidone, a maleic acid/styrene copolymer, a methacrylic acid copolymer, a half ester of a polymer of a polyhydric alcohol and a dicarboxylic anhydride, a water-soluble salt of polystyrene sulfonic acid, polyoxyethylene alkanediols, polyoxyethylene alkyne diols, and alkyne diols.

Examples of the sticker include various surfactants such as sodium dialkyl sulfosuccinate, polyoxyethylene alkyl ether, polyoxyethylene alkyl phenyl ether, and polyoxyethylene fatty acid ester; paraffin, terpene, polyamide resin, polyacrylate, polyoxyethylene, wax, polyvinyl alkyl ether, alkylphenol formalin condensate, and synthetic resin emulsion.

Examples of the anti-freezing agent include polyhydric alcohols such as ethylene glycol, diethylene glycol, propylene glycol, and glycerin.

Examples of the anti-caking agent include polysaccharides such as starch, alginic acid, mannose, and galactose; polyvinylpyrrolidone, white carbon, ester gum, and petroleum resin.

Examples of the disintegrant include sodium tripolyphosphate, sodium hexametaphosphate, metal stearate, cellulose powder, dextrin, a copolymer of methacrylic acid ester, polyvinylpyrrolidone, a polyaminocarboxylic acid chelate compound, a sulfonated styrene-isobutylene-maleic anhydride copolymer, and a starch-polyacrylonitrile graft copolymer.

Examples of the degradation preventing agent include desiccants such as zeolite, quicklime, and magnesium oxide; antioxidants such as phenol-based, amine-based, sulfur-based, and phosphoric acid-based antioxidants; and ultraviolet absorbers such as salicylic acid-based and benzophenone-based ultraviolet absorbers.

Examples of the preservative include potassium sorbate and 1,2-benzothiazolin-3-one.

Examples of the plant piece include sawdust, coconut shell, corn cob, and tobacco stalk.

On the other hand, when the above-described additive component is contained in the pest control agent of the present invention, the content thereof is usually selected in the range of 5 to 95%, preferably 20 to 90% in the case of a carrier such as a solid carrier or a liquid carrier, usually selected in the range of 0.1% to 30%, preferably 0.5 to 10% in the case of a surfactant, and selected in the range of 0.1 to 30%, preferably 0.5 to 10% in the case of other additives, on a mass basis.

The pest control agent of the present invention is used by being formulated in any formulation such as a powder, a powder granule, a granule, a wettable powder, a water-soluble agent, a water-dispersible granule, a tablet, a jumbo agent, an emulsifiable concentrate, an oil, a liquid, a flowable agent, an emulsion, a microemulsion, a suspoemulsion, a trace spray agent, a microcapsule, a smoking agent, an aerosol agent, a bait agent, or a paste agent.

In actual use of these formulations, they can be used as they are or diluted to a predetermined concentration with a diluent such as water. The application of various formulations containing the compound of the present invention or dilutions thereof can be carried out by an application method that is usually carried out. In other words, the application can be carried out by dispersion (for example, spraying, misting, atomizing, dusting powder, dusting granules, water surface application, and box application), soil application (for example, mixing and irrigation), surface application (for example, application, and powder coating, and coating), seed treatment (for example, smearing, powder coating treatment, and the like), immersion, poison bait, smoking application, and the like. In addition, it is also possible to control the generation and growth of harmful bugs, particularly harmful insects in excrement of domestic animals by feeding the animals with the active ingredient mixed.

The method for controlling a pest of the present invention can be carried out by using the 3-alkoxybenzamide derivative of formula [I] or agriculturally acceptable salt thereof according to the present invention in an amount of an active ingredient by the application method described above.

The blending ratio (mass%) of the active ingredient in the pest control agent of the present invention is appropriately selected as necessary. For example, the blending ratio may be appropriately selected from the range of 0.01 to 20%, preferably 0.05 to 10% in the case of a powder, a powder granule, a fine powder, or the like. The blending ratio may be appropriately selected from the range of 0.1 to 30%, preferably 0.5 to 20% in the case of a granule, or the like. The blending ratio may be appropriately selected from the range of 1 to 70%, preferably 5 to 50% in the case of a wettable powder, a water-dispersible granule, or the like. The blending ratio may be appropriately selected from the range of 1 to 95%, preferably 10 to 80% in the case of a water-soluble agent, a liquid, or the like. The blending ratio may be appropriately selected from the range of 5 to 90%, preferably 10 to 80% in the case of an emulsifiable concentrate, or the like. The blending ratio may be appropriately selected from the range of 1 to 50%, preferably 5 to 30% in the case of an oil, or the like. The blending ratio may be appropriately selected from the range of 5 to 60%, preferably 10 to 50% in the case of a flowable agent, or the like. The blending ratio may be appropriately selected from the range of 5 to 70%, preferably 10 to 60% in the case of an emulsion, a microemulsion, a suspoemulsion, or the like. The blending ratio may be appropriately selected from the range of 1 to 80%, preferably 5 to 50% in the case of a tablet, a bait agent, a paste agent, or the like. The blending ratio may be appropriately selected from the range of 0.1 to 50%, preferably 1 to 30% in the case of a smoking agent or the like. The blending ratio may be appropriately selected from the range of 0.05 to 20%, preferably 0.1 to 10% in the case of an aerosol agent or the like.

These formulations are diluted to an appropriate concentration and dispersed, or directly applied.

When the pest control agent of the present invention is used by being diluted with a diluent, the application is generally performed at an active ingredient concentration of 0.1 to 5,000 ppm. When the formulation is used as it is, the amount of application per unit area is 0.1 to 5,000 g per 1 ha as the active ingredient compound, but the amount of application is not limited thereto.

Although it is needless to say that the pest control agent of the present invention is sufficiently effective even when the compound of the present invention is used alone as an active ingredient, the pest control agent can be mixed with or used in combination with other fertilizers and pesticides, for example, insecticides, acaricides, nematicides, synergists, fungicides, antiviral agents, attractants, herbicides, and plant growth regulators as necessary, and in this case, a more excellent effect may be exhibited.

Next, known insecticides (insecticidal active ingredients), acaricides (acaricidal active ingredients), nematicides (nematicidal active ingredients), and synergist compounds (synergistic active ingredients) which may be mixed or used in combination are exemplified below.

Insecticidal active ingredients, acaricidal active ingredients, nematicidal active ingredients, synergistic active ingredients:
acrinathrin, azadirachtin, azamethiphos, acynonapyr, azinphos-ethyl, azinphos-methyl, acequinocyl, acetamiprid, acetoprole, acephate, azocyclotin, abamectin, afidopyropen, afoxolaner, amidoflumet, amitraz, alanycarb, aldicarb, aldoxycarb, allethrin [including a d-cis-trans-form and a d-trans-form], isazophos, isamidofos, isocarbophos, isoxathion, isocycloseram, isofenphos-methyl, isoprocarb, epsilon-metofluthrin, epsilon-momfluorothrin, ivermectin, imicyafos, imidacloprid, imiprothrin, indoxacarb, esfenvalerate, ethiofencarb, ethion, ethiprole, ethylene dibromide, etoxazole, etofenprox, ethoprophos, etrimfos, emamectin, emamectin benzoate, endosulfan, empenthrin, oxazosulfyl, oxamyl, oxydemeton-methyl, oxydeprofos, omethoate, Nuclear polyhedrosis virus, cadusafos, kappa-tefluthrin, kappa-bifenthrin, karanjin, cartap, Granulosis virus, carbaryl, carbosulfan, carbofuran, gamma-BHC, xylylcarb, quinalphos, kinoprene, chinomethionat, Entero virus, coumaphos, cryolite, clothianidin, clofentezine, chromafenozide, chlorantraniliprole, chlorethoxyfos, chlordane, chloropicrin, chlorpyrifos, chlorpyrifos-methyl, chlorfenapyr, chlorfenvinphos, chlorfluazuron, chlormephos, chloroprallethrin, Entomopoxi virus, Irido virus, cyazypyr, cyanophos, diafenthiuron, diamidafos, cyantraniliprole, cyetpyrafen, dienochlor, cyenopyrafen, dioxabenzofos, diofenolan, Sigma virus, cyclaniliprole, cycloxaprid, dicrotophos, dichlofenthion, cyclobutrifluram, cycloprothrin, dichlorvos, dicloromezotiaz, dicofol, dicyclanil, disulfoton, dinotefuran, dinobuton, cyhalodiamide, cyhalothrin [including a gamma-form and a lambda-form], cyphenothrin [including a 1R-trans-form], cyfluthrin [including a beta-form], diflubenzuron, cyflumetofen, diflovidazin, cyproflanilide, cyhexatin, cypermethrin [including an alpha-form, a beta-form, a theta-form, and a zeta-form], dimpropyridaz, dimethyl-2,2,2-trichloro-1-hydroxyethylphosphonate (DEP), dimethylvinphos, dimethoate, dimefluthrin, jasmone, cis-jasmone, jasmonic acid, methyl jasmonate, silafluofen, cyromazine, *Steinernema carpocapsae, Steinernema kushidai, Steinernema glaseri,* spinetoram, spinosad, spirodiclofen, spirotetramat, spiropidion, spiromesifen, sulcofuron-sodium, sulfluramid, sulfoxaflor, sulfotep, diazinon, thiacloprid, thiamethoxam, tioxazafen, thiodicarb, thiocyclam, thiosultap, thionazin, thiofanox, thiometon, tyclopyrazoflor, tetrachlorantraniliprole, tetrachlorvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, tralomethrin, transfluthrin, triazamate, triazophos, trichlorfon, *Trichoderma asperellum, Trichoderma paecilomyces, Trichoderma harzianum,* triflumuron, triflumezopyrim, trimethacarb, tolfenpyrad, naled, nicotine, nicofluprole, nitenpyram, nemadectin, Denso virus, novaluron, noviflumuron,
*Paecilomyces lilacinus, Barkholderia cepacia, Barkholderia rinojensis, Verticillium lecanii,* hydroprene, *Pasteuria nishizawae, Pasteuria penetrans, Bacillus amyloliquefaciens, Bacillus firmus, Bacillus sphaericus, Bacillus subtillis, Bacillus thuringiensis,* insect toxin produced by *Bacillus thuringiensis, Bacillus thuringiensis subsp. Aizawai, Bacillus thuringiensis subsp. Israelensis, Bacillus thuringiensis subsp. Kurstaki, Bacillus thuringiensis subsp. Tenebrionis, Bacillus popilliae, Bacillus licheniformis,* vamidothion, parathion, parathion-methyl, halfenprox, halofenozide, bioallethrin, bioallethrin S-cyclopentenyl, bioresmethrin, bis-(2-chloro-1-methyl)ethylether (DCIP), bistrifluron, hydramethylnon, bifenazate, bifenthrin, pyflubumide, piperonyl butoxide, pymetrozine, pyraclofos, pyrafluprole, pyridaphenthion, pyridaben, pyridalyl, pyrifluquinazon, pyriprole, pyriproxyfen, pirimicarb, pyrimidifen, pyriminostrobin, pirimiphos-methyl, pyrethrin, famphur, fipronil, fenazaquin, fenamiphos, fenitrothion, fenoxycarb, fenothiocarb, phenothrin [including a 1R-trans-form], fenobucarb, fenthion, phenthoate, fenvalerate, fenpyroximate, fenbutatin oxide, fenpropathrin, fonofos, sulfuryl fluoride, butocarboxim, butoxycarboxim, buprofezin, furathiocarb, prallethrin, fluacrypyrim, fluazaindolizine, fluazuron, fluensulfone, fluopyram, sodium fluoroacetate, fluxametamide, flucycloxuron, flucythrinate, flusulfamide, fluthrin, fluvalinate [including a tau-form], flupyradifurone, flupyrazofos, flupyrimin, flufiprole, flufenerim, flufenoxystrobin, flufenoxuron, fluhexafon, flubendiamide, flupentiofenox, flumethrin, fluralaner, flurimfen, prothiofos, protrifenbute, flonicamid, propaphos, propargite, prohydrojasmon, profenofos, broflanilide, profluthrin, propetamphos, propoxur, flometoquin, bromopropylate, hexythiazox, hexaflumuron, Pacilimyces tenuipes, Paecilomyces fumosoroceus, Paecilomyces lilacinus, heptafluthrin, heptenophos, permethrin, benclothiaz, benzpyrimoxan, bensultap, benzoximate, bendiocarb, benfuracarb, *Pochonia chlamydosporia, Beauveria tenella, Beauveria bassiana, Beauveria brongniartii,* phoxim, phosalone, fosthiazate, fosthietan, phosphamidon, phosmet, polynactins, formetanate, phorate,
machine oil, malathion, milbemectin, mecarbam, mesulfenfos, methomyl, metaldehyde, metaflumizone, methamidophos, metham, methiocarb, methidathion, methyl isothiocyanate, methyl bromide, methoxychlor, methoxyfenozide, methothrin, metofluthrin, methoprene, metolcarb, mevinphos, meperfluthrin, *Monacrosporium phymatophagum, Monacrosporium phymatophagum,* monocrotophos, momfluorothrin, *Trichoderma harzianum,* litlure-A, litlure-B, aluminium phosphide, zinc phosphide, phosphine, lufenuron, rescalure, resmethrin, lepimectin, rotenone, occlusion body of Cytoplasmic polyhedrosis virus, fenbutatin oxide, calcium cyanide, organotins, nicotine-sulfate, (Z)-11-tetradecenyl=acetate, (Z)-11-hexadecenyl, (Z)-11-hexadecenyl=acetate, (Z)-9,12-tetradecadienyl=acetate, (Z)-9-tetradecene-1-ol, (Z,E)-9,11-tetradecadienyl=acetate, (Z,E)-9,12-tetradecadienyl=acetate, 1,1,1-trichloro-2,2-bis(4-chlorophenyl)ethane (DDT), 1,3-dichloropropene, 2,4-dichloro-5-{2-[4-(trifluoromethyl)phenyl]ethoxy}phenyl-2,2,2-trifluoroethyl sulfoxide (Chemical Name, CAS Registry Number: 1472052-11-1), 2,4-dimethyl-5-[6-(trifluoromethylthio)hexyloxylphenyl-2,2,2-trifluoroethyl sulfoxide (Chemical Name, CAS Registry Number: 1472050-34-2), 2-{2-fluoro-4-methyl-5-[(2,2,2-trifluoroethyl) sulfinyl]phenoxy}-5-(trifluoromethyl)pyridine (Chemical Name, CAS Registry Number: 1448758-62-0), 3-chloro-2-{2-fluoro-4-methyl-5-[(2,2,2-trifluoroethyl)sulfinyl]phenoxy}-5-(trifluoromethyl)pyridine (Chemical Name, CAS Registry Number: 1448761-28-1), 4,6-dinitro-o-cresol (DNOC), 4-fluoro-2-methyl-5-(5,5-dimethylhexyloxy)phenyl-2,2,2-trifluoroethyl sulfoxide (Chemical Name, CAS Registry Number: 1472047-71-4), Bt protein (Cry1Ab, Cry1Ac, Cry1Fa, Cry2Ab, mCry3A, Cry3Ab, Cry3Bb, Cry34/35Ab1), methyl eugenol, 4-(p-acetoxyphenyl)-2-butanone, (Z)-10-tetradecenyl=acetate, (E,Z)-4,10-tetradecadienyl=acetate, (Z)-8-dodecenyl=acetate, (Z)-11-tetradecenyl=acetate, (Z)-13-icosene-10-one, 14-methyl-1-octadecene, AKD-1193 (code number), BCS-AA10147 (code number), CL900167 (code number), NA-85 (code number), NI-30 (code number), O-O-diethyl-O-[4-(dimethylsulfamoyl)phenyl]-phosphorothionate (DSP), O-ethyl-O-4-(nitrophenyl)phenylphosphonothioate (EPN), RU15525 (code number), XMC, Z-13-icosene-10-one, ZXI8901 (code number), and F4260 (code number).

Next, known fungicides (fungicidal active ingredients) or disease control agent compounds which may be mixed or used in combination are exemplified below.

Fungicidal active ingredients or disease control agent compounds:
*Agrobacterim radiobacter,* azaconazole, acibenzolar-S-methyl, azoxystrobin, anilazine, amisulbrom, aminopyrifen, ametoctradin, aldimorph, isotianil, isopyrazam, isofetamid, isoflucypram, isoprothiolane, ipconazole, ipflufenoquin, ipfentrifluconazole, iprodione, iprovalicarb, iprobenfos, imazalil, iminoctadine-albesilate, iminoctadine-triacetate, imibenconazole, inpyrfluxam, imprimatin A, imprimatin B, edifenphos, etaconazole, ethaboxam, ethirimol, ethoxyquin, etridiazole, enestroburin, enoxastrobin, epoxiconazole, organic oils, oxadixyl, oxazinylazole, oxathiapiprolin, oxycarboxin, oxine-copper, oxytetracycline, oxpoconazole-fumarate, oxolinic acid, copper dioctanoate, octhilinone, ofurace, orysastrobin, o-phenylphenol, kasugamycin, captafol, carpropamid, carbendazim, carboxin, carvone, *Candida oleophila, Candida saitoana,* quinoxyfen, quinofumelin, chinomethionat, captan, quinconazole, quintozene, guazatine, cufraneb, coumethoxystrobin, coumoxystrobin, *Gliocradium catenulatum, Cryptococcus albidus,* kresoxim-methyl, clozylacon, *Clonostachys rosea,* chlozolinate, chloroinconazide, chlorothalonil, chloroneb, *Chaetomium cupreum, Coniothyrium minitans,* cyazofamid, diethofencarb, diclocymet, dichlofluanid, dichlobentiazox, diclomezine, dicloran, dichlorophen, dithianon, diniconazole, diniconazole-M, zineb, dinocap, dipymetitrone, diphenylamine, difenoconazole, cyflufenamid, diflumetorim, cyproconazole, cyprodinil, simeconazole, dimethirimol, dimethyl disulfide, dimethomorph, cymoxanil, dimoxystrobin, *Pseudozyma flocculosa, Pseudomonas aureofaciens, Pseudomonas chlororaphis, Pseudomonas syringae, Pseudomonas flurorescens, Pseudomonas rhodesiae,* ziram, silthiofam, Zucchini yellow mosaic virus, streptomycin, *Streptomyces griseoviridis, Streptomyces lygicus,* spiroxamine, sedaxane, zoxamide, solatenol, dazomet, *Talaromyces flavus,* tiadinil, thiabendazole, thiram, thiophanate, thiophanate-methyl, thifluzamide, thiram, tecnazene, tecloftalam, tetraconazole, debacarb, tebuconazole, tebufloquin, terbinafine, dodine, dodemorph, triadimenol, triadimefon, triazoxide, trichlamide, triclopyricarb, *Trichoderma asperellum, Trichoderma atroviride, Trichoderma gamsii, Trichoderma stromaticum, Trichoderma paecilomyces, Trichoderma harzianum, Trichoderma viride, Trichoderma virens, Trichoderma polysporum, Trichoderma harzianum rifai, Trichoderma lignorum,* tricyclazole, triticonazole, tridemorph, triflumizole, trifloxystrobin, triforine, tolylfluanid, tolclofos-methyl, tolnifanide, tolprocarb, nabam, natamycin, naftifine, nitrapyrin, nitrothal-isopropyl, nuarimol, copper nonyl phenol sulfonate,
*Paenibacillus polymyxa, Barkholderia cepacia, Bacillus amyloliquefaciens, Bacillus simplex, Bacillus subtilis, Bacillus pumilus, Bacillus licheniformis,* harpin protein, *Variovorax paradoxus,* validamycin, valifenalate, *Pantoea agglomerans,* picarbutrazox, bixafen, picoxystrobin, *Pythium oligandrum,* pydiflumetofen, bitertanol, binapacryl, hinokitiol, *Erwinia carotovora, Rhizobium vitis,* biphenyl, piperalin, hymexazol, pyraoxystrobin, pyraclostrobin, pyraziflumid, pyrazophos, pyrapropoyne, pyrametostrobin, pyriofenone, pyrisoxazole, pyridachlometyl, pyrifenox, pyributicarb, pyribencarb, pyrimethanil, pyroquilon, vinclozolin, ferbam, famoxadone, phenazine oxide, fenamidone, fenaminstrobin, fenarimol, fenoxanil, ferimzone, fenpiclonil, fenpicoxamid, fenpyrazamine, fenbuconazole, fenfuram, fenpropidin, fenpropimorph, fenhexamid, folpet, phthalide, Fusarium oxysporum, bupirimate, fuberidazole, blasticidin-S, furametpyr, furalaxyl, furancarboxylic acid, fluazinam, fluindapyr, fluoxastrobin, fluoxapiprolin, fluopicolide, fluopimomide, fluopyram, fluoroimide, fluxapyroxad, fluquinconazole, furconazole, furconazole-cis, fludioxonil, flusilazole, flusulfamide, flutianil, flutolanil, flutriafol, flufenoxystrobin, flubeneteram, flumetover, flumorph, Phlebiopsis gigantea, proquinazid, prochloraz, procymidone, prothiocarb, prothioconazole, bronopol, propamocarb-hydrochloride, propiconazole, propineb, probenazole, bromuconazole, flometoquin, florylpicoxamid, hexaconazole, benalaxyl, benalaxyl-M, benodanil, benomyl, pefurazoate, penconazole, pencycuron, benzovindiflupyr, benthiazole, benthiavalicarb-isopropyl, penthiopyrad, penflufen, boscalid, fosetyl (including a salt of aluminum, calcium, sodium, or the like), polyoxin, polycarbamate, Bordeaux mixture, mancopper, mancozeb, mandipropamid, mandestrobin, maneb, myclobutanil, mineral oils, mildiomycin, methasulfocarb, metam, metalaxyl, metalaxyl-M, metiram, metyltetraprole, metconazole, metominostrobin, metrafenone, mepanipyrim, mefentrifluconazole, meptyldinocap, mepronil, iodocarb, laminarin, phosphorous acid and salts, copper oxychloride, silver, copper (II) sulfate, cuprous oxide, copper hydroxide, potassium bicarbonate, sodium bicarbonate, sulfur, oxyquinoline sulfate, copper sulfate, (3,4-dichloroisothiazole-5-yl)methyl-4-(tert-butyl) benzoic acid ester (Chemical Name, CAS Registry Number: 1231214-23-5), BAF-045 (code number), BAG-010 (code number), UK-2A (code number), dodecylbenzenesulfonic acid bisethylenediamine copper complex [II] (DBEDC), MIF-1002 (code number), NF-180 (code number), triphenyltin acetate (TPTA), triphenyltin chloride (TPTC), triphenyltin hydroxide (TPTH), and F9650 (code number).

Next, known herbicidal compounds, herbicidal active ingredients, or plant growth regulator compounds which may be mixed or used in combination are exemplified below, but are not limited to these examples.

Herbicidal compounds or herbicidal active ingredients:
ioxynil (including lithium salt, sodium salt, a salt with octanoic acid or the like), aclonifen, acrolein, azafenidin, acifluorfen (including a salt with sodium or the like), azimsulfuron, asulam, acetochlor, atrazine, anilofos, amicarbazone, amidosulfuron, amitrole, aminocyclopyrachlor, aminopyralid, amiprofos-methyl, ametryn, Araujia Mosaic Virus, alachlor, *Alternaria destruens,* alloxydim (including a salt with sodium or the like), ancymidol, isouron, isoxachlortole, isoxaflutole, isoxaben, isodecylalcohol ethoxylate, isoproturon, ipfencarbazone, imazaquin, imazapic (including a salt with amine or the like), imazapyr (including a salt of isopropylamine or the like), imazamethabenz, imazamethabenz-methyl, imazamox, imazethapyr, imazosulfuron, indaziflam, indanofan, eglinazine-ethyl, esprocarb, ethametsulfuron-methyl, ethalfluralin, ethidimuron, ethoxysulfuron, ethoxyfen, ethoxyfen-ethyl, ethofumesate, etobenzanid, epyrifenacil, endothal-disodium, oxadiazon, oxadiargyl, oxaziclomefone, oxasulfuron, oxyfluorfen, oryzalin, Obuda Pepper virus, orthosulfamuron, orbencarb, oleic acid, cafenstrole, caprylic acid, capric acid, carfentrazone-ethyl, karbutilate, carbetamide, quizalofop, quizalofop-ethyl, quizalofop-P-ethyl, quizalofop-P-tefuryl, *Xanthomonas campestris,* quinoclamine, quinclorac, quinmerac, citric acid, cumyluron, clacyfos, glyphosate (including a salt of sodium, potassium, amine, propylamine, isopropylamine, ammonium, isopropyl ammonium, guanidine, monoethanolamine, choline, BAPMA (N,N-bis-(aminopropyl)methylamine), dimethylamine, or trimesium or the like), glufosinate (including a salt of amine, or sodium or the like), glufosinate-P, glufosinate-P-sodium, clethodim, clodinafop, clodinafop-propargyl, clopyralid (including a monoethanolamine salt), clomazone, chlomethoxyfen, clomeprop, cloransulam-methyl, chloramben, chloridazon, chlorimuron, chlorimuron-ethyl, chlorsulfuron, chlorthal-dimethyl, chlorthiamid, chlorphthalim, chlorflurenol-methyl, chlorpropham, chlorbromuron, chloroxuron, chlorotoluron, ketospiradox (including a salt of sodium, calcium, or ammonia or the like), *Colletotrichum orbiculare, Colletotrichum gloeosporioides, Colletotrichum truncatum, Chondrostercum purpureum,* saflufenacil, sarmentine, cyanazine, cyanamide, diuron, diethatyl-ethyl, dicamba (including a salt of amine, diethylamine, isopropylamine, diglycolamine, dimethyl ammonium, diolamine, isopropyl ammonium, auramine, potassium, trolamine, BAPMA (N,N-bis-(aminopropyl)methylamine), choline, sodium, or lithium or the like, or an ester such as methyl ester), cycloate, cycloxydim, diclosulam, cyclosulfamuron, cyclopyranil, cyclopyrimorate, dichlobenil, diclofop, diclofop-P-methyl, diclofop-methyl, dichlorprop, dichlorprop-P (including a salt of dimethyl ammonium, potassium, sodium, choline or the like, or an ester such as butotyl ester, 2-ethylhexyl ester, isoctyl ester, or methyl ester), diquat, diquat dibromide, dithiopyr, siduron, dinitramine, cinidon-ethyl, cinosulfuron, dinoseb (including acetate), dinoterb, cyhalofop, cyhalofop-butyl, cypyrafluone, diphenamid, difenzoquat, diflufenican, diflufenzopyr, simazine, dimesulfazet, dimethachlor, dimethametryn, dimethenamid, dimethenamid-P, simetryn, dimepiperate, dimefuron, *Pseudomonas fluorescens,* cinmethylin, swep, sulcotrione, sulfentrazone, sulfosate, sulfosulfuron, sulfometuron-methyl, sethoxydim, *Scelerothinia minor,* terbacil, daimuron, thaxtomin A, Tobacco Mild Green Mosaic Tobamovirus, Tobacco Rattle Virus, dalapon, thiazopyr, tiafenacil, thiencarbazone (including sodium salt, methyl ester, or the like), tiocarbazil, thiobencarb, thidiazimin, thidiazuron, thifensulfuron, thifensulfuron-methyl, desmedipham, desmetryne, tetflupyrolimet, thenylchlor, tebutam, tebuthiuron, tepraloxydim, tefuryltrione, terbuthylazine, terbutryn, terbumeton, tembotrione, topramezone, tralkoxydim, triaziflam, triasulfuron, triafamone, tri-allate, trietazine, triclopyr, triclopyr-butotyl, triclopyr-triethylammonium, tritosulfuron, tripyrasulfone, trifludimoxazin, triflusulfuron-methyl, trifluralin, trifloxysulfuron (including a salt of sodium or the like), tribenuron-methyl, tolpyralate, naptalam (including a salt with sodium or the like), naproanilide, napropamide, napropamide-M, nicosulfuron, lactic acid, neburon, norflurazon,
*Barkholderia rinojensis,* vernolate, paraquat, paraquat dichloride, halauxifen, halauxifen-benzyl, halauxifen-methyl, *Burkholderia rinojensis,* haloxyfop, haloxyfop-P, haloxyfop-etotyl, haloxyfop-P-methyl, halosafen, halosulfuron-methyl, bixlozone, picloram (including a salt with dichloro ammonium, trolamine or the like), picolinafen, bicyclopyrone, bispyribac-sodium, pinoxaden, bipyrazone, bifenox, piperophos, pyraclonil, pyrasulfotole, pyrazoxyfen, pyrazosulfuron-ethyl, pyrazolynate, bilanafos, pyraflufen, pyraflufen-ethyl, pyridafol, pyrithiobac-sodium, pyridate, pyriftalid, pyributicarb, pyribenzoxim, pyrimisulfan, pyriminobac-methyl, pyroxasulfone, pyroxsulam, *Phytophthora palmivora,* phenisopham, fenuron, fenoxasulfone, fenoxaprop (including methyl, ethyl, or isopropyl ester), fenoxaprop-P (including methyl, ethyl, or isopropyl ester), fenquinotrione, fenthiaprop-ethyl, fentrazamide, fenpyrazone, phenmedipham, *Phoma chenopodicola, Phoma herbarum, Phoma macrostoma,* butachlor, butafenacil, butamifos, butylate, *Puccinia canaliculata, Puccinia thlaspeos,* butenachlor, butralin, butroxydim, flazasulfuron, flamprop (including methyl, ethyl, or isopropyl ester), flamprop-M (including methyl, ethyl, or isopropyl ester), primisulfuron, primisulfuron-methyl, fluazifop-butyl, fluazifop-P, fluazifop-P-butyl, fluazolate, fluometuron, fluoroglycofen-ethyl, flucarbazone-sodium, fluchloralin, flucetosulfuron, fluthiacet-methyl, flupyrsulfuron-methyl (including a salt of sodium, calcium, or ammonia or the like), flufenacet, flufenpyr-ethyl, flupropanate (including sodium salt), flupoxame, flumioxazin, flumiclorac-pentyl, flumetsulam, fluridone, flurtamone, fluroxypyr (including an ester of butometyl, meptyl or the like, or a salt of sodium, calcium, ammonia or the like), flurochloridone, pretilachlor, procarbazone (including a salt with sodium or the like), prodiamine, prosulfuron, prosulfocarb, propaquizafop, propachlor, propazine, propanil, propyzamide, propisochlor, propyrisulfuron, propham, profluazol, prohexadione-calcium, propoxycarbazone, propoxycarbazone-sodium, profoxydim, bromacil, brompyrazon, prometryn, prometon, bromoxynil (including an ester of butyric acid, octanoic acid, or heptanoic acid or the like), bromofenoxim, bromobutide, florasulam, florpyrauxifen, florpyrauxifen-benzyl, hexazinone, pethoxamid, benazolin, benazolin-ethyl, penoxsulam, Pepino Mosaic Virus, heptamaloxyloglucan, beflubutamid, beflubutamid-M, pebulate, pelargonic acid, bencarbazone, benquitrione, benzfendizone, bensulide, bensulfuron, bensulfuron-methyl, benzobicyclon, benzofenap, bentazone, pentanochlor, pendimethalin, pentoxazone, benfluralin, benfuresate, fosamine, fomesafen, foramsulfuron, forchlorfenuron,
mecoprop (including a salt of sodium, potassium, isopropylamine, triethanolamine, dimethylamine, diolamine, trolamine, choline or the like, or an ester such as ethadyl ester, 2-ethylhexyl ester, isoctyl ester, or methyl ester), mecoprop-P-potassium, mesosulfuron (including an ester of methyl or the like), mesotrione, metazachlor, metazosulfuron, methabenzthiazuron, metamitron, metamifop, metam (including a salt of sodium or the like), disodium methanearsonate (DSMA), methiozolin, methyldymuron, metoxuron, metosulam, metsulfuron-methyl, metobromuron, metobenzuron, metolachlor, metribuzin, mepiquat chloride, mefenacet, monosulfuron (including methyl, ethyl, or isopropyl ester), monolinuron, molinate, iodosulfuron, iodosulfulon-methyl-sodium, iofensulfuron, iofensulfuron-sodium, lactofen, lancotrione, linuron, rimsulfuron, lenacil, 2,2,2-trichloroacetic acid (TCA) (including a salt of sodium, calcium, or ammonia or the like), 2,3,6-trichlorobenzoic acid (2,3,6-TBA), 2,4,5-trichlorophenoxyacetic acid (2,4,5-T), 2,4-dichlorophenoxyacetic acid (2,4-D) (including a salt of amine, diethylamine, triethanolamine, isopropylamine, dimethylammonium, diolamine, dodecylammonium, heptylammonium, tetradecylammonium, triethylammonium, tris(2-hydroxypropyl)ammonium, trolamine, choline, sodium, lithium or the like, or an ester such as butotyl ester, 2-butoxypropyl ester, 2-ethylhexyl ester, methyl ester, ethylester, butyl ester, isobutyl ester, octyl ester, pentyl ester, propylester, isoctyl ester, isopropyl ester, meptyl ester, or tefuryl ester), 2,4-dichlorophenoxybutyric acid (2,4-DB) (including a salt of amine, diethylamine, triethanolamine, isopropylamine, dimethylammonium, choline, sodium, lithium or the like, or an ester such as isoctyl ester), 2-amino-3-chloro-1,4-naphthoquinone (ACN), 2-methyl-4-chlorophenoxyacetic acid (MCPA) (including a salt of sodium, dimethylammonium, choline or the like, or an ester such as 2-ethylhexyl ester, isoctyl ester, ethyl ester or the like), 2-methyl-4-chlorophenoxybutyric acid (MCPB) (including sodium salt, ethyl ester, or the like), 4-(2,4-dichlorophenoxy)butyric acid (2,4-DB), 4,6-dinitro-O-cresol (DNOC) (including a salt of amine or sodium or the like), (5S)-3-(3,5-difluorophenyl)-N-[rel-(3R,5R)-5-(trifluoromethylsulfonylcarbamoyl)tetrahydrofuran-3-yl]-5-vinyl-4H-isoxazole-5-carboxamid,
(Chemical Name, CAS Registry Number: 2266183-40-6) (WO 2018/228986 A, WO 2020/114934 A), N4-(2,6-difluorophenyl)-6-(1-fluoro-1-methyl-ethyl)-1,3,5-triazine-2,4-diamine (Chemical Name, CAS Registry Number: 1606999-43-2) (WO 2014/064094 A, WO 2015/162164 A), (5S)-3-(3,5-difluorophenyl)-N-[(3R)-5-(methylsulfonylcarbamoyl)-2,3-dihydrofuran-3-yl]-5-vinyl-4H-isoxazole-5-carboxamid (Chemical Name, CAS Registry Number: 2266190-06-9) (WO 2018/228986 A, WO 2020/114934 A), (5R)-3-(3,5-difluorophenyl)-5-methyl-N-[rel-(3R,5R)-5-(methylsulfonylcarbamoyl)tetrahydrofuran-3-yl]-4H-isoxazole-5-carboxamid (Chemical Name, CAS Registry Number: 2266164-36-5) (WO 2018/228986 A, WO 2020/114934 A), (5R)-3-(3,5-difluorophenyl)-N-[(3R)-5-(methoxycarbamoyl)-2,3-dihydrofuran-3-yl]-5-methyl-4H-isoxazole-5 carboxyamide (Chemical Name, CAS Registry Number: 2266170-31-2) (WO 2018/228986 A, WO 2020/114934 A), 2-[2-(3,4-dimethoxyphenyl)-6-methyl-3-oxo-pyridazine-4-carbonyl]cyclohexane-1,3-dione (Chemical Name, CAS Registry Number: 2138855-12-4) (WO 2017/178582 A, WO 2018/015476 A), 4-hydroxy-1-methyl-3-[4-(trifluoromethyl)-2-pyridyl]imidazolidin-2-one (Chemical Name, CAS Registry Number: 1708087-22-2) (WO 2015/059262 A, WO 2018/015476 A), 6-(1-fluorocyclopentyl)-N4-(2,3,5,6-tetrafluorophenyl)-1,3,5-triazine-2,4-diamine (Chemical Name, CAS Registry Number: 1820807-75-7) (WO 2015/162164 A), 6-(1-fluoro-1-methyl-ethyl)-N4-(2,3,5,6-tetrafluorophenyl)-1,3,5-triazine-2,4-diamine (Chemical Name, CAS Registry Number: 1606999-21-6) (WO 2014/064094 A, WO 2015/162164 A), (5S)-3-(3-fluoro-5-methyl-phenyl)-N-[rel-(3R,5R)-5-(methoxycarbamoyl)tetrahydrofuran-3-yl]-5-vinyl-4H-isoxazole-5-carboxamid (Chemical Name, CAS Registry Number: 2266292-43-5) (WO 2018/228986 A, WO 2020/114934 A), 6-(1-methylcyclobutyl)-N4-(2,3,5,6-tetrafluorophenyl)-1,3,5-triazine-2,4-diamine (Chemical Name, CAS Registry Number: 1607001-97-7) (WO 2014/064094 A, WO 2015/162164 A), AE-F-150944 (code number), F9960 (code number), IR-6396 (code number), MCPA-thioethyl, NC-656 (code number), SYP-298 (code number), SYP-300 (code number), S-ethyldipropylthiocarbamate (EPTC), S-metolachlor, S-9750 (code number), MSMA, and HW-02 (code number).

Plant growth regulators:
1-naphthylacetamide, 1-methylcyclopropene, 1,3-diphenylurea, 2,3,5-triiodobenzoic acid, 2-methyl-4-chlorophenoxybutyric acid (MCPB) [including sodium salt, ethyl ester, or the like], 2-(naphthalene-1-yl)acetamide, 2,6-diisopropylnaphthalene, 3-[(6-chloro-4-phenylquinazoline-2-yl)amino]propane-1-ol, 4-oxo-4-(2-phenylethyl) amino butyric acid (Chemical Name, CAS Registry Number: 1083-55-2), 4-chlorophenoxyacetic acid (4-CPA), 5-aminolevulinic acid hydrochloride, methyl 5-(trifluoromethyl)benzo[b]thiophene-2-carboxylate, aminoethoxyvinylglycine (AVG), n-decanol, aviglycine, ancymidol, abscisic acid, isoprothiolane, inabenfide, indole acetic acid, indole butyric acid, uniconazole, uniconazole-P, Ecolyst, ethychlozate, ethephon, epocholeone, calcium chloride, choline chloride, oxine-sulfate, kinetin, calcium peroxide, carvone, calcium formate, cloxyfonac, cloxyfonac-potassium, cloprop, chlormequat, chlormequat-chloride, chlorpropham, choline, cytokinins, oxydized glutathione, cyanamide, sodium cyanate, cyclanilide, dichlorprop (including a salt of dimethylammonium, potassium, sodium, choline or the like, or an ester such as butotyl ester, 2-ethylhexyl ester, isoctyl ester, or methyl ester), dichlorprop-P (including a salt of sodium, potassium, dimethylammonium or the like, or 2-ethylhexyl ester), diquat, diquat dibromide, dikegulac, gibberellic acid, gibberellin A4, gibberellin A7, dimethipin, sintofen, jasmone, cis-jasmone, jasmonic acid, methyl jasmonate, streptomycin, calcium polysulfide, daminozide, calcium carbonate, thidiazuron, decan-1-ol, triacontanol, triapenthenol, trinexapac-ethyl, tribufos, paclobutrazol, paraffin, bispyribac-sodium, hymexazol, butralin, fluthiacet-methyl, pyraflufen-ethyl, flumetralin, flurprimidol, flurenol, pronitridine, prohydrojasmon, prohexadione-calcium, heptamaloxyloglucan, 6-benzylaminopurine, pendimethalin, forchlorfenuron, formononetin, maleic hydrazide, mepiquat chloride, mefluidide, lipochitooligosaccharides, for example, lipochitooligosaccharides SP104, and calcium sulfate.

Next, known phytotoxicity reducing compounds which may be mixed or used in combination are exemplified.

Phytotoxicity reducing compounds:
isoxadifen, isoxadifen-ethyl, oxabetrinil, octane-1,8-diamine, cloquintocet, cloquintcet-mexyl, dietholate, cyometrinil, dichlormid, dicyclonone, cyprosulfamide, daimuron, 1,8-naphthalic anhydride, fenchlorazole, fenchlorazole-O-ethyl, fenclorim, furilazole, fluxofenim, flurazole, benoxacor, metcamifen, mephenate, mefenpyr, mefenpyr-ethyl, mefenpyr-diethyl, lower alkyl-substituted benzoic acid, 2,2-dichloro-N-(1,3-dioxolan-2-ylmethyl)-N-(2-propenyl)acetamide (PPG-1292), 2-dichloromethyl-2-methyl-1,3-dioxane (MG-191), 3-dichloroacetyl-2,2,5-trimethyl-1,3-oxazolidine (R-29148), 4-dichloroacetyl-1-oxa-4-azaspiro[4.5]decane (AD-67), 4-carboxy-3,4-dihydro-2H-1-benzopyran-4-acetic acid (CL-304415, code number), MON4660 (code number), metcamifen, N1,N2-diallyl-N2-dichloroacetylglycinamide (DKA-24, code number), 1-bromo-4-[(chloromethyl)sulfonyl]benzene (CSB), 2-propenyl-1-oxa-4-azaspiro[4,5]decane-4-carbodithioate (MG-838, code number), 3-(dichloroacetyl)-2,2-dimethyl-1,3-oxazolidine (R-28725, code number), R-29148 (code number), and 1-(dichloroacetyl)azepane (TI-35, code number).

Next, known biological pesticides which may be mixed or used in combination are exemplified.

Biological pesticides:
*Haplothrips brevitubus, Franklinothrips vespiformis, Diglyphus isaea, Encarsia formosa, Amblyseius cucumeris, Pseudaphycus malinus, Amblyseius womersleyi, Aphidius colemani, Eretmocerus eremicus, Aphidoletes aphidimyza, Amblyseius swirskii, Orius strigicollis, Phytoseiulus persimilis, Amblyseius degenerans, Phytoseiulus persimilis, Orius sauteri, Dacnusa sibirica, Amblyseius californicus, Chrysoperla nipponensis,* and *Anicetus beneficus.*

Next, known agricultural materials which may be mixed or used in combination will be exemplified.

Agricultural materials:
ethylene, hypochlorous acid solution (limited to those obtained by electrolysis of hydrochloric acid or aqueous potassium chloride solution), sodium bicarbonate, vinegar, humic substances, humic acid, fulvic acid, seaweed extracts, polysaccharides, amino acid, microbial materials, functional components derived from animals and plants, microbial metabolites, microbial activation materials, soil stickers, soil permeability control materials, soil water retention materials, and the like, as well as bio-stimulants.

Next, known agricultural fertilizer components which may be mixed or used in combination are exemplified.

Examples of the fertilizer include an inorganic fertilizer and an organic fertilizer. Specific examples thereof include ammonium chloride, ammonium sulfate, ammonium nitrate, ammonium dihydrogen phosphate, ammonium urea nitrate, urea, calcium cyanamide, potassium nitrate, superphosphate of lime, double superphosphate of lime, potassium dihydrogen phosphate, potassium chloride, potassium sulfate, potassium carbonate, potassium silicate, oil cake, fish meal, rice bran, bat guano, and fermented chicken manure.

The pest control agent of the present invention configured as described above exhibits an excellent control effect on pests such as *Orthoptera* pests, *Thysanoptera* pests, *Hemiptera* pests, *Coleoptera* pests, *Diptera* pests, *Lepidoptera* pests, *Hymenoptera* pests, *Collembola* pests, *Thysanura* pests, *Blattodea* pests, *Psocoptera* pests, *Mallophaga* pests, *Phthiraptera* pests, plant parasitic mites, plant parasitic nematodes, plant parasitic mollusks, other harmful animals, unpleasant animals, hygiene pests, and parasites. Examples of such pests include the following species.

Examples of the *Orthoptera* pests include *Tettigoniidae* pests such as *Ruspolia lineosa; Gryllidae* pests such as *Teleogryllus emma* and *Truljalia hibinonis; Gryllotalpidae* pests such as *Gryllotalpa orientalis; Acrididae* pests such as *Oxya hyla intricate, Locusta migratoria, Melanoplus sanguinipes, Melanoplus differentialis,* and *Melanoplus femurrubrum; Pyrgomorphidae* pests such as *Atractomorpha lata; Eneopteridae* pests such as *Euscyrtus japonicus;* and *Tridactylidae* pests such as *Xya japonicus.*

Examples of the *Thysanoptera* pests include *Thripidae* pests such as *Frankliniella intonsa, Frankliniella occidentalis, Scirtothrips dorsalis, Thrips palmi, Thrips tabaci, Thrips setosus, Heliothrips haemorrhoidalis,* and *Stenchaetothrips biformis;* and *Phlaeothripidae* pests such as *Ponticulothrips diospyrosi, Liothrips wasabiae,* and *Haplothrips aculeatus.*

Examples of the *Hemiptera* pests include *Cicadidae* pests such as *Mogannia minuta; Aphrophoridae* pests such as *Aphrophora intermedia* and *Mahanarva fimbriolata; Membracidae* pests such as *Machaerotypus sibiricus; Cicadellidae* pests such as *Arboridia apicalis, Empoasca onukii, Nephotettix cincticeps, Nephotettix malayanus, Nephotettix virescens, Nephotettix nigropictus, Recilia dorsalis, Amrasca biguttula, Idioscopus nitidulus, Idioscopus clypealis, Amritodus atkinsoni, Empoasca fabae,* and *Dalbulus maidis; Cixiidae* pests such as *Pentastiridius apicalis; Delphacidae* pests such as *Laodelphax striatellus, Nilaparvata lugens, Sogatella furcifera,* and *Peregrinus maidis; Meenoplidae* pests such as *Nisia nervosa; Derbidae* pests such as *Kamendaka saccharivora; Achilidae* pests such as *Achilus flammeus; Ricaniidae* pests such as *Orosanga japonicus; Flatidae* pests such as *Mimophantia maritima; Psyllidae* pests such as *Cacopsylla pyrisuga* and *Diaphorina citri; Calophyidae* pests such as *Calophya mangiferae; Phylloxeridae* pests such as *Daktulosphaira vitifoliae; Adelgidae* pests such as *Adelges laricis* and *Adelges tsugae; Aphididae* pests such as *Acyrthosiphon pisum, Aphis gossypii, Aphis spiraecola, Lipaphis erysimi, Brevicoryne brassicae, Myzus persicae, Schizaphis graminum, Rhopalosiphum padi, Toxoptera aurautii, Aulacorthum solani, Macrosiphum euphorbiae, Nasonovia ribisnigri, Sitobion avenae,* and *Aphis glycines; Aleyrodidae* pests such as *Aleurocanthus camelliae, Aleurocanthus spiniferus, Bemisia tabaci, Bemisia argentifolii,* and *Trialeurodes vaporariorum; Monophlebidae* pests such as *Drosicha corpulenta* and *Icerya purchasi; Pseudococcidae* pests such as *Dysmicoccus brevipes, Planococcus citri,* and *Pseudococcus comstocki; Coccidae* pests such as *Ceroplastes ceriferus* and *Ceroplastes rubens; Aclerdidae* pests such as *Aclerda takahashii; Diaspididae* pests such as *Aonidiella aurantii, Diaspidiotus perniciosus, Pseudaulacaspis pentagoa,* and *Unaspis yanonensis; Miridae* pests such as *Lygus lineolaris, Trigonotylus caelestialium, Apolygus lucorum, Nesidiocoris tenuis,* and *Halticus bractatus; Tingidae* pests such as *Stephanitis pyrioides* and *Stephanitis nashi; Pentatomidae* pests such as *Eurydema rugosa, Eysarcoris lewisi, Eysarcoris aeneus, Lagynotomus elongatus, Nezara viridula, Plautia crossota, Nezara antennata, Eushistus heros, Piezodorus guildini, Tibraca limbativentris,* and *Dichelops furcatus; Plataspidae* pests such as *Megacopta cribraria; Urostylididae* pests such as *Urochela luteovoria; Lygaeidae* pests such as *Cavelerius saccharivorus; Malcidae* pests such as *Malcus japonicus; Pyrrhocoridae* pests such as *Dysdercus cingulatus; Alydidae* pests such as *Leptocorisa acuta* and *Leptocorisa chinensis; Coreidae* pests such as *Anacanthocoris striicornis; Rhopalidae* pests such as *Rhopalus maculatus; Cydnidae* pests such as *Scaptocoris castanea;* and *Cimicidae* pests such as *Cimex lectularis.*

Examples of the *Coleoptera* pests include *Scarabaeidae* pests such as *Anomara cuprea, Anomara rufocuprea, Popillia japonica, Oxycetonia jucunda, Anomala geniculata, Oryctes rhinoceros, Heptophylla picea,* and *Phyllophaga cuyabana; Elateridae* pests such as *Agriotes ogurae, Agriotes lineatus, Agriotes obscurus, Melanotus okinawensis,* and *Melanotus fortnumi; Dermestidae* pests such as *Anthrenus verbasci; Bostrychidae* pest such as *Heterobostrychus hamatipennis; Anobiidae* pests such as *Stegobium paniceum; Ptinidae* pests such as *Pitinus clavipes; Trogossitidae* pests such as *Tenebroides mauritanicus; Cleridae* pests such as *Necrobia rufipes; Nitidulidae* pests such as *Carpophilus hemipterus* and *Meligethes aeneus; Silvanidae* pests such as *Ahasverus advena; Laemophloeidae* pests such as *Cryptolestes ferrugineus; Coccinellidae* pests such as *Epilachna varivestis* and *Henosepilachna vigintioctopunctata; Tenebrionidae* pests such as *Tenebrio molitor* and *Tribolium castaneum; Meloidae* pest such as *Epicauta gorhami; Cerambycidae* pests such as *Anoplophora glabripennis, Xylotrechus pyrrhoderus, Monochamus alternatus,* and *Dectes texanus; Bruchinae* pests such as *Callosobruchus chinensis; Chrysomelidae* pests such as *Leptinotarsa decemlineata, Diabrotica virgifera, Diabrotica barberi, Diabrotica undecimpunctata howardi, Aulacophora femoralis, Phaedon brassicae, Cassida nebulosa, Oulema oryzae, Epilachna varivestis, Phyllotreta striolata, Demotina fasciculata, Psylliodes chrysocephala, Cerotoma trifurcate, Colaspis brunnea, Colaspis crinnicornis, Odontota horni, Chaetocnema pulicaria,* and *Diabrotica balteata; Brentidae* pests such as *Cylas formicarius; Curculionidae* pests such as *Hypera postica, Listroderes costirostris, Euscepes postfasciatus, Curculio sikkimensis, Anthonomus grandis,* and *Sternechus subsignatus; Erirhinidae* pests such as *Echinocnemus bipunctatus, Lissorhoptrus oryzophilus,* and *Oryzophagus oryzae; Rhynchophoridae* pests such as *Sitophilus zeamais, Sphenophrus venatus,* and *Sphenophorus levis; Scolytidae* pests such as *Tomicus piniperda; Platypodidae* pests such as *Crossotarsus niponicus;* and *Lyctinae* pests such as *Lyctus brunneus.*

Examples of the *Diptera* pests include *Tipulidae* pests such as *Tipula aino; Bibionidae* pests such as *Plecia nearctica; Mycetophilidae* pests such as *Exechia shiitakevora; Sciaridae* pests such as *Pnyxia scabiei* and *Bradysia agrestis; Cecidomyiidae* pests such as *Asphondylia yushimai, Mayetiola destructor,* and *Dasineura oxycoccana; Culicidae* pests such as *Aedes aegypti* and *Culex pipiens pallens; Simuliidae* pests such as *Simulium takahashii; Chironomidae* pests such as *Chironomus oryzae; Tabanidae* pests such as *Chrysops suavis* and *Tabanus trigonus; Syrphidae* pests such as *Eumerus strigatus; Tephritidae* pests such as *Bactrocera dorsalis, Euphranta japonica,* and *Ceratitis capitata; Agromyzidae* pests such as *Liriomyza trifolii, Liriomyza sativae, Agromyza oryzae, Liriomyza bryoniae, Chromatomyia horticola,* and *Liriomyza chinensis; Chloropidae* pests such as *Meromyza nigriventris; Drosophilidae* pests such as *Drosophila suzukii* and *Drosophila melanogaster; Ephydridae* pests such as *Hydrellia griseola; Hippoboscidae* pests such as *Hippobosca equina; Scathophagidae* pests such as *Parallelpmma sasakawae; Anthomyiidae* pests such as *Delia antiqua* and *Delia platura; Fanniidae* pests such as *Fannia canicularis; Muscidae* pest such as *Musca domestica* and *Stomoxys calcitrans; Sarcophagidae* pests such as *Sarcophaga peregrina; Gasterophilidae* pests such as *Gasterophilus intestinalis; Hypodermatidae* pests such as *Hypoderma lineatum;* and *Oestridae* pests such as *Oestrus ovis.*

Examples of the *Lepidoptera* pests include *Hepialidae* pests such as *Endoclita excrescens; Heliozelidae* pests such as *Antispila ampelopsia; Cossidae* pests such as *Zeuzera leuconotum* and *Cossus insularis; Tortricidae* pests such as *Archips fuscocupreanus, Adoxophyes orana fasciata, Grapholita molesta, Homona magnanima, Leguminivora glycinivorella, Cydia pomonella,* and *Lobesia botrana; Tortricidae* pests such as *Eupoecilia ambiguella; Psychidae* pests such as *Bambalina sp.* and *Eumeta minuscula; Tineidae* pests such as *Nemapogon granella* and *Tinea translucens; Bucculatricidae* pest such as *Bucculatrix pyrivorella; Tischeriidae* pests such as *Lyonetia clerkella* and *Lyonetiaprunifoliella malinella; Gracillariidae* pests such as *Caloptilia theivora* and *Phyllonorycter ringoniella; Phyllocnistidae* pests such as *Phyllocnistis citrella; Acrolepiidae* pests such as *Acrolepiopsis sapporensis; Plutellidae* pests such as *Plutella xylostella; Yponomeutidae* pests such as *Yponomeuta orientalis; Argyresthidae* pests such as *Argyresthia conjugella; Sesiidae* pests such as *Nokona regalis* and *Synanthedin hector; Gelechiidae* pests such as *Phthorimaea operculella, Sitotroga cerealella, Pectinophora gossypiella,* and *Tuta absoluta; Carposinidae* pests such as *Carposina sasakii; Zygaenidae* pest such as *Illiberis pruni; Limacodidae* pests such as *Monema flavescens; Crambidae* pests such as *Ancylolomia japonica, Chilo suppressalis, Cnaphalocrocis medinalis, Ostrinia furnacalis, Hellulla undalis, Conogethes punctiferlis, Diaphania indica, Parapediasia teterrella,* and *Ostrinia nubilalis; Diatraea saccharalis, Pyralidae* pests such as *Cadra cautella* and *Galleria mellonella; Pterophoridae* pests such as *Nippoptilia vitis; Papilionidae* pests such as *Papilio xuthus; Pieridae* pests such as *Pieris rapae; Hesperiidae* pests such as *Parnara guttata; Geometridae* pests such as *Ascotis selenaria; Lasiocampidae* pests such as *Dendrolimus spectabilis* and *Malacosoma neustrium testaceum; Sphingidae* pests such as *Agrius convolvuli; Lymantriinae* pests such as *Arna pseudoconspersa, Orygia recens approximans,* and *Lymantria dispar; Arctiidae* pests such as *Hyphantria cunea;* and *Noctuidae* pests such as *Agrotis ipsilon, Agrotis segetum, Autographa nigrisigna, Helicoverpa armigera, Helicoverpa zea, Heliothis virescens, Spodoptera exigua, Spodoptera litura, Chrysodeix includens, Spodoptera frugiperda,* and *Nephelodes minians.*

Examples of the *Hymenoptera* pests include *Argidae* pests such as *Arge pagana; Tenthredinidae* pests such as *Apethymus kuri* and *Athalia rosae ruficornis; Cynipidae* pests such as *Dryocosmus kuriphilus; Vespidae* pests such as *Vespa simillima xanthoptera; Formicidae* pests such as *Solenopsis invicta* and *Linepithema humile;* and *Megachilidae* pests such as *Megachile nipponica.*

Examples of the *Collembola* pests include *Sminthuridae* pests such as *Bourletiella hortensis.*

Examples of the *Thysanura* pests include *Lepismatidae* pests such as *Lepisma saccharina* and *Ctenolepisma villosa.*

Examples of the *Blattodea* pests include *Blattidae* pests such as *Periplaneta americana; Blattellidae* pests such as *Blattella germanica; Termitidae* pests such as *Odontotermes formosanus; Kalotermitidae* pests such as *Incisitermes minor* and *Cryptotermes domesticus;* and *Rhinotermitidae* pests such as *Coptotermes formosanus* and *Reticulitermes speratus.*

Examples of the *Psocoptera* pests include *Trogiomorpha* pests such as *Trogium pulsatorium;* and *Troctomorpha* pests such as *Liposcelis corrodens.*

Examples of the *Dermaptera* pests include *Labiduridae* pests such as *Labodura riparia.*

Examples of the *Mallophaga* pests include *Menoponidae* pests such as *Lipeurus caponis;* and *Trichodectidae* pests such as *Damalinia bovis.*

Examples of the *Phthiraptera* pests include *Haematopinidae* pests such as *Haematopinus suis; Pediculidae* pests such as *Pediculus humanus; Linognathidae* pests such as *Linognathus setosus;* and *Pthiridae* pests such as *Pthirus pubis.*

Examples of the *Acari* pests include *Eupodidae* pests such as *Penthaleus major; Tarsonemidae* pests such as *Phytonemus pallidus* and *Polyphagotarsonemus latus; Pyemotidae* pests such as *Siteroptes sp.; Tenuipalpidae* pests such as *Brevipalpus lewisi; Tuckerellidae* pests such as *Tuckerella pavoniformis; Tetranychidae* pests such as *Eotetranychus boreus, Panonychus citri, Panonychus ulmi, Tetranychus urticae,* and *Tetranychus kanzawai; Nalepellidae* pests such as *Trisetacus pini; Eriophyidae* pests such as *Aculops pelekassi, Epitrimerus pyri, Phyllocoptruta oleivora,* and *Aculops lycopersici; Diptilomiopidae* pests such as *Diptacus crenatae; Acaridae* pests such as *Aleuroglyphus ovatus, Tyrophagus putrescentiae,* and *Rhizoglyphus robini; Varroidae* pests such as *Varroa jacobsoni; Dermanyssidae* pests such as *Dermanyssus gallinae; Macronyssidae* pests such as *Ornithonyssus sylviarum; Ixodidae* pests such as *Boophilus microplus, Rhipicephalus sanguineus,* and *Haemaphysalis longicornis;* and *Sarcoptidae* pests such as *Sarcoptes scabiei.*

Examples of the plant parasitic nematodes include those belonging to the family *Longidoridae* such as *Xiphinema index;* those belonging to the family *Trichodoridae* such as *Paratrichodorus minor;* those belonging to the family *Rhabditidae* such as *Rhabditella sp.;* those belonging to the family *Tylenchidae* such as *Aglenchus sp.;* those belonging to the family *Tylodoridae* such as *Cephalenchus sp.;* those belonging to the family *Anguinidae* such as *Nothotylenchus acris* and *Ditylenchus destructor;* those belonging to the family *Hoplolaimidae* such as *Rotylenchulus reniformis* and *Helicotylenchus dihystera;* those belonging to the family *Paratylenchidae* such as *Paratylenchus curvitatus;* those belonging to the family *Meloidogynidae* such as *Meloidogyne incognita, Meloidogyne hapla, Meloidogyne javanica, Meloidogyne chitwoodi,* and *Meloidogyne fallax;* those belonging to the family *Heteroderidae* such as *Globodera rostochiensis, Globodera pallida, Heterodera glycines,* and *Heterodera Schachtii;* those belonging to the family *Telotylenchidae* such as *Tylenchorhynchus claytoni;* those belonging to the family *Psilenchidae* such as *Psilenchus sp.*; those belonging to the family *Criconematidae* such as *Criconemoides sp.;* those belonging to the family *Tylenchulidae* such as *Tylenchulus semipenetrans;* those belonging to the family *Sphaeronematinae* such as *Sphaeronema camelliae;* those belonging to the family *Pratylenchidae* such as *Radopholus citrophilus, Radopholus similis, Nacobbus aberrans, Pratylenchus penetrans, Pratylenchus coffeae, Pratylenchus zeae,* and *Pratylenchus brachyurus;* those belonging to the family *lotonchiidae* such as *Iotonchium ungulatum;* those belonging to the family *Aphelenchus avenae* such as *Aphelenchus avenae;* those belonging to the family *Aphelenchoididae* such as *Aphelenchoides besseyi* and *Aphelenchoides fragariae;* and those belonging to the family *Parasitaphelenchidae* such as *Bursaphelenchus xylophilus.*

Examples of the plant parasitic mollusks include those belonging to the family *Pilinidae* such as *Pomacea canaliculata;* those belonging to the family *Veronicellidae* such as *Leavicaulis alte;* those belonging to the family *Achatinidae* such as *Achatina fulica;* those belonging to the family *Philomycidae* such as *Meghimatium bilineatum;* those belonging to the family *Succineidae* such as *Succinealauta;* those belonging to the family *Discidae* such as *Discus pauper;* those belonging to the family *Zonitidae* such as *Zonitoides yessoensis;* those belonging to the family *Limacidae* such as *Limax flavus, Lehmannia valentiana,* and *Deroceras reticulatum;* those belonging to the family *Helicarionidae* such as *Parakaliella harimensis;* and those belonging to the family *Bradybaenidae* such as *Acusta despecta sieboldiana* and *Bradybaena similaris.*

Examples of the other pests such as harmful animals, unpleasant animals, hygiene pests, pests harmful to domestic animals, and parasites include *Procambarus clarkii* of the family *Cambaridae* of the order *Decapoda, Porcellio scaber* of the family *Porcellionidae* of the order *Isopoda, Armadillidium vulgare* of the family *Armadillidiidae, Chilopoda* pests such as *Thereuonema tuberculata* of the family *Scutigeridae* of the order *Scutigeromorpha,* and *Scolopendra subspinipes* of the order *Scolopendromorpha, Diplopoda* pests such as *Oxidus gracilis* of the family *Paradoxosomatidae* of the order *Polydesmida, Theridiidae hasseltii* of the family *Theridiidae* of the order *Araneae, Cheiracanthium japonicum* of the family *Clubionidae* of the order *Araneae, Androctonus crassicauda* of the order *Scorpiones,* endoparasitic nematodes such as *Ascaris lumbricoides, Syphacia sp.,* and *Wuchereria bancrofti,* and endoparasitic platyhelminthes such as *Distomum sp., Paragonimus westermanii, Metagonimus yokokawai, Schistosoma japonicum, Taenia solium, Taeniarhynchus saginatus, Echinococcus sp.,* and *Diphyllobothrium latum.*

The pest control agent of the present invention also exhibits a control effect on the pests and the like exemplified above which have acquired resistance to existing pest control agents. In addition, the pest control agent of the present invention can also be used for plants which have acquired characteristics such as pest resistance, disease resistance, and herbicide resistance by genetic recombination, artificial crossing, and the like.

The "plant imparted with resistance by breeding methods or gene recombination techniques" of the present invention includes not only plants imparted with resistance by classical variety crossing and resistance imparted by gene recombination techniques, but also plants imparted with resistance by new plant breeding techniques (NBTs) in which a molecular biological technique is combined with a conventional crossing technique. The new plant breeding techniques (NBTs) are described in a book "Understanding new plant breeding techniques" (International Academic Publishing Co., Ltd., written by Ryo Ohsawa and Hiroshi Ezura), a review article "Genome Editing Tools in Plants" (Genes 2017, 8, 399, written by Tapan Kumar Mohanta, Tufail Bashir, Abeer Hashem, Elsayed Fathi Abd_Allah, and Hanhong Bae), and the like.

Next, the production method, formulation method, and use of the compound of the present invention will be described in detail in the following Examples, but the present invention is not limited to these Examples at all. The melting point, which is a physical property value of the compound of the present invention, was measured with an MP-500V micro melting point measuring apparatus manufactured by Yanaco Technical Science. The refractive index was measured using an Abbe refractometer manufactured by ATAGO Co., Ltd. The ¹H NMR spectrum was measured using JNM-LA400 (400 MHz), JNM-LA300 (300 MHz), or JNM-ECS300 (300 MHz) manufactured by JEOL Ltd. with tetramethylsilane (TMS) as an internal standard. The high resolution mass spectrometry spectrum (HRMS) was measured using a Q Exactive Focus Hybrid Quadrupole-Orbitrap LC-MS/MS System (ESI, positive mode) manufactured by Thermo Fisher Scientific Inc.

The production example of a production intermediate of the compound of the present invention is also described.

### Examples

### [Example 1]

### Production of (R)-2-chloro-4-fluoro-5-{2-[(1,1,1-trifluoro-N-methylmethyl)sulfonamido]propoxy}-N-(2,2,2-trifluoroethyl) benzamide (compound of present invention No.: C-0191)

### (1) Production of (R)-2-(tert-butoxycarbonylamino)-1-propanol

To a mixture of 5.00 g (66.6 mmol) of (R)-2-amino-1-propanol, 7.40 g (73.1 mmol) of triethylamine, and 70 mL of tetrahydrofuran, 14.53 g (66.6 mmol) of di-tert-butyl dicarbonate was added under ice cooling, and the mixture was stirred at room temperature for 15 hours. Ethyl acetate was added thereto, and the organic layer was washed with 2N hydrochloric acid and brine, and dried over anhydrous magnesium sulfate. The solvent was distilled off in vacuo to obtain 13.24 g (yield: quantitative) of a crude target product. This was directly used in the next process without purification.
¹H-NMR data (400 MHz, CDCl₃/TMS δ (ppm)): 1.15 (3H, d), 1.45 (9H, s), 0.72 (1H, brs), 3.51 (1H, dd), 3.64 (1H, dd), 3.77 (1H, brs), 4.65 (1H, brs)

### (2) Production of (R)-(2-aminopropoxy)-2-chloro-4-fluoro-N-(2,2,2-trifluoroethyl) benzamide (compound of present invention No.: A-0368)

To a mixture of 3.00 g (11.0 mmol) of 2-chloro-4-fluoro-5-hydroxy-N-(2,2,2-trifluoroethyl) benzamide, 2.52 g (14.4 mmol) of (R)-2-(tert-butoxycarbonylamino)-1-propanol, 3.78 g (14.4 mmol) of triphenylphosphine, and 70 mL of tetrahydrofuran, 2.91 g (14.4 mmol) of diisopropyl azodicarboxylate was added under ice cooling, and the mixture was stirred at room temperature for 16 hours. Further, 1.89 g (7.21 mmol) of triphenylphosphine and 1.46 g (7.22 mmol) of diisopropyl azodicarboxylate were added thereto at room temperature, and the mixture was stirred at room temperature for 24 hours. The solvent was distilled off in vacuo, and the residue was purified by silica gel column chromatography (developing solvent, n-hexane : ethyl acetate = 3 : 1) to obtain (R)-tert-butyl-(1-{4-chloro-2-fluoro-5-[(2,2,2-trifluoroethyl)carbamoyl]phenoxy}propan-2-yl)carbamate. To a mixture of the obtained (R)-tert-butyl-(1-{4-chloro-2-fluoro-5-[(2,2,2-trifluoroethyl)carbamoyl]phenoxy}propan-2-yl)carbamate and 30 mL of dichloromethane, 14 mL of trifluoroacetic acid was added at room temperature, and the mixture was stirred for 19 hours. Further, 2N hydrochloric acid was added thereto, and the aqueous layer was washed with isopropyl ether. Then, an aqueous sodium hydroxide solution was added to the obtained aqueous layer. The mixture was subjected to extraction with ethyl acetate, and the obtained organic layer was dried over anhydrous sodium sulfate. The solvent was distilled off in vacuo to obtain 3.50 g (yield: 96%) of a crude target product. This was directly used in the next process without purification.
¹H-NMR data (400 MHz, CDCl₃/TMS δ (ppm)): 1.16 (3H, d), 3.34-3.41 (1H, m), 3.73 (1H, dd), 3.94 (1H, dd), 4.08-4.17 (2H, m), 6.97 (1H, brs), 7.22 (1H, d), 7.40 (1H, d)

### (3) Production of (R)-2-chloro-4-fluoro-N-(2,2,2-trifluoroethyl)-5-{2-[(trifluoromethyl)sulfonamido]propoxy}benzamide (compound of present invention No.: C-0005)

To a mixture of 3.30 g (10.0 mmol) of crude (R)-(2-aminopropoxy)-2-chloro-4-fluoro-N-(2,2,2-trifluoroethyl)benzamide, 1.52 g (15.0 mmol) of triethylamine, 1.50 g of molecular sieve (4A), 50 mL of tetrahydrofuran, and 50 mL of dichloromethane, 2.54 g (15.1 mmol) of trifluoromethanesulfonyl chloride was added under ice cooling, and the mixture was stirred at room temperature for 17 hours. Chloroform was added to thereto, and the organic layer was washed with 2N hydrochloric acid and brine, and dried over anhydrous sodium sulfate. The solvent was distilled off in vacuo, and the residue was purified by silica gel column chromatography (developing solvent, n-hexane : ethyl acetate = 3 : 1) to obtain 2.34 g (yield: 51%) of a target product.
¹H-NMR data (400 MHz, DMSO-d6/TMS δ (ppm)): 1.26 (3H, d), 3.88 (1H, m), 3.96-4.14 (4H, m), 7.28 (1H, d), 7.55 (1H, d), 9.12 (1H, t), 9.62 (1H, brs)

### (4) Production of (R)-2-chloro-4-fluoro-5-{2-[(1,1,1-trifluoro-N-methylmethyl)sulfonamido]propoxy}-N-(2,2,2-trifluoroethyl)benzamide (compound of present invention No.: C-0191)

To a mixture of 1.18 g (2.56 mmol) of (R)-2-chloro-4-fluoro-N-(2,2,2-trifluoroethyl)-5-{2-[(trifluoromethyl)sulfonamido]propoxy}benzamide and 0.46 g (3.3 mmol) of potassium carbonate in 30 mL of acetonitrile, 0.47 g (3.3 mmol) of methyl iodide was added at room temperature, and the mixture was stirred for 23 hours. The mixture was filtered, and the solvent was distilled off in vacuo. The residue was purified by silica gel column chromatography (developing solvent, n-hexane : ethyl acetate = 3 : 1) to obtain 0.81 g (yield: 67%) of a target product.
¹H-NMR data (300 MHz, CDCl₃/TMS δ (ppm)): 1.40 (3H, d), 3.05 (3H, s), 4.05-4.21 (4H, m), 4.40-4.51 (1H, m), 6.81 (1H, brs), 7.20 (1H, d), 7.43 (1H, d)

### [Example 2]

Production of (S)-2-chloro-4-fluoro-5-{2-[(1,1,1-trifluoro-N-methylmethyl)sulfonamido]propoxy}-N-(2,2,2-trifluoroethyl)benzamide (compound of present invention No.: C-0192)

### (1) Production of (S)-(2-aminopropoxy)-2-chloro-4-fluoro-N-(2,2,2-trifluoroethyl)benzamide (compound of present invention No.: A-0369)

To a mixture of 3.00 g (11.0 mmol) of 2-chloro-4-fluoro-5-hydroxy-N-(2,2,2-trifluoroethyl)benzamide, 2.13 g (12.2 mmol) of (S)-2-(tert-butoxycarbonylamino)-1-propanol, 3.19 g (12.5 mmol) of triphenylphosphine, and 60 mL of tetrahydrofuran, 2.46 g (12.17 mmol) of diisopropyl azodicarboxylate was added under ice cooling, and the mixture was stirred at room temperature for 13 hours. Further, 3.19 g (12.2 mmol) of triphenylphosphine and 2.46 g (12.2 mmol) of diisopropyl azodicarboxylate were added thereto at room temperature, and the mixture was stirred at room temperature for 8 hours. The solvent was distilled off in vacuo, and the residue was purified by silica gel column chromatography (developing solvent, n-hexane : ethyl acetate = 3 : 1) to obtain (S)-tert-butyl-(1-{4-chloro-2-fluoro-5-[(2,2,2-trifluoroethyl)carbamoyl]phenoxy}propan-2-yl)carbamate. To a mixture of the obtained (S)-tert-butyl-(1-{4-chloro-2-fluoro-5-[(2,2,2-trifluoroethyl)carbamoyl]phenoxy}propan-2-yl)carbamate and 31 mL of dichloromethane, 14 mL of trifluoroacetic acid was added at room temperature, and the mixture was stirred for 1.5 hours. The solvent was distilled off in vacuo, 2N hydrochloric acid was added to the residue, and the aqueous layer was washed with isopropyl ether. Then, an aqueous sodium hydroxide solution was added to the obtained aqueous layer. The mixture was subjected to extraction with ethyl acetate, and the obtained organic layer was dried over anhydrous sodium sulfate. The solvent was distilled off in vacuo to obtain 3.90 g (yield: quantitative) of a crude target product. This was directly used in the next process without purification.
¹H-NMR data (400 MHz, CDCl₃/TMS δ (ppm)): 1.16 (3H, d), 3.34-3.39 (1H, m), 3.71 (1H, dd), 3.93 (1H, dd), 4.12 (2H, m), 7.14 (1H, brs), 7.15 (1H, d), 7.36 (1H, d)

### (2) Production of (S)-2-chloro-4-fluoro-N-(2,2,2-trifluoroethyl)-5-{2-[(trifluoromethyl)sulfonamido]propoxy}benzamide (compound of present invention No.: C-0006)

To a mixture of 1.50 g (4.56 mmol) of crude (S)-(2-aminopropoxy)-2-chloro-4-fluoro-N-(2,2,2-trifluoroethyl)benzamide, 0.69 g (6.8 mmol) of triethylamine, 0.75 g of molecular sieve (4A), 45 mL of tetrahydrofuran, and 45 mL of dichloromethane, 1.15 g (6.82 mmol) of trifluoromethanesulfonyl chloride was added under ice cooling, and the mixture was stirred at room temperature for 17 hours. The mixture was filtered, ethyl acetate was added thereto, and the organic layer was washed with a saturated aqueous ammonium chloride solution and dried over anhydrous sodium sulfate. The solvent was distilled off in vacuo, and the residue was purified by silica gel column chromatography (developing solvent, n-hexane : ethyl acetate = 3 : 1) to obtain 1.07 g (yield: 51%) of a target product.
¹H-NMR data (400 MHz, DMSO-d6/TMS δ (ppm)): 1.26 (3H, d), 3.88 (1H, brs), 3.96-4.15 (4H, m), 7.29 (1H, d), 7.55 (1H, d), 9.12 (1H, t), 9.62 (1H, brs)

### (3) Production of (S)-2-chloro-4-fluoro-5-{2-[(1,1,1-trifluoro-N-methylmethyl)sulfonamido]propoxy}-N-(2,2,2-trifluoroethyl)benzamide (compound of present invention No.: C-0192)

To a mixture of 0.86 g (1.9 mmol) of (S)-2-chloro-4-fluoro-N-(2,2,2-trifluoroethyl)-5-{2-[(trifluoromethyl)sulfonamido]propoxy}benzamide and 0.34 g (2.5 mmol) of potassium carbonate in 20 mL of acetonitrile, 0.34 g (2.4 mmol) of methyl iodide was added at room temperature, and the mixture was stirred for 23 hours. The mixture was filtered, and the solvent was distilled off in vacuo. The residue was purified by silica gel column chromatography (developing solvent, n-hexane : ethyl acetate = 3 : 1) to obtain 0.86 g (yield: 97%) of a target product.
¹H-NMR data (300 MHz, CDCl₃/TMS δ (ppm)): 1.41 (3H, d), 3.05 (3H, s), 4.03-4.22 (4H, m), 4.40-4.51 (1H, m), 6.80 (1H, brs), 7.20 (1H, d), 7.44 (1H, d)

### [Example 3]

### Production of 2-chloro-4-fluoro-5-{2-methyl-3-[(trifluoromethyl)thio]propoxy}-N-(2,2,2-trifluoroethyl)benzamide (compound of present invention No.: B-0009)

### (1) Production of 2-chloro-5-(3-chloro-2-methylpropoxy)-4-fluoro-N-(2,2,2-trifluoroethyl)benzamide (compound of present invention No.: A-0037)

To a mixture of 3.00 g (11.0 mmol) of 2-chloro-4-fluoro-5-hydroxy-N-(2,2,2-trifluoroethyl)benzamide and 100 mL of N,N-dimethylformamide, 1.99 g (14.4 mmol) of potassium carbonate, 0.36 g (1.1 mmol) of tetrabutylammonium bromide, and 3.79 g (22.1 mmol) of 1-bromo-3-chloro-2-methylpropane were added at room temperature, and the mixture was stirred at 70°C for 7 hours. The mixture was allowed to cool at room temperature and then filtered, and ethyl acetate and hexane were added thereto. The mixture was washed with water, and dried over anhydrous sodium sulfate. The solvent was distilled off in vacuo, and the residue was purified by silica gel column chromatography (developing solvent, n-hexane : ethyl acetate = 5 : 1) to obtain 3.71 g (purity: about 50%, yield: 46%) of a target product.
¹H-NMR data (400 MHz, CDCl₃/TMS δ (ppm)): 1.15 (3H, d), 2.32-2.48 (1H, m), 3.68 (2H, ddd), 3.98-4.19 (4H, m), 6.79 (1H, brs), 7.16 (1H, d), 7.44 (1H, d)

### (2) Production of 2-chloro-4-fluoro-5-(2-methyl-3-thiocyanatopropoxy)-N-(2,2,2-trifluoroethyl)benzamide (compound of present invention No.: A-0758)

To a mixture of 3.71 g (purity: about 50%, about 5.12 mmol) of 2-chloro-5-(3-chloro-2-methylpropoxy)-4-fluoro-N-(2,2,2-trifluoroethyl)benzamide and 60 mL of ethanol, 1.99 g (20.5 mmol) of potassium thiocyanate and 0.17 g (1.0 mmol) of potassium iodide were added at room temperature, and the mixture was stirred under reflux for 3 days. The mixture was allowed to cool at room temperature and then filtered, and the solvent was distilled off in vacuo. The residue was purified by silica gel column chromatography (developing solvent, n-hexane : ethyl acetate = 3 : 1) to obtain 0.84 g (yield: 43%) of a target product.
¹H-NMR data (300 MHz, CDCl₃/TMS δ (ppm)): 1.24 (3H, d), 2.40-2.55 (1H, m), 3.06 (1H, dd), 3.23 (1H, dd), 3.99 (1H, dd), 4.07-4.18 (3H, m), 6.83 (1H, brs), 7.17 (1H, d), 7.42 (1H, d)

### (3) Production of 2-chloro-4-fluoro-5-{2-methyl-3-[(trifluoromethyl)thio]propoxy}-N-(2,2,2-trifluoroethyl)benzamide (compound of present invention No.: B-0009)

To a mixture of 0.76 g (2.0 mmol) of 2-chloro-4-fluoro-5-(2-methyl-3-thiocyanatopropoxy)-N-(2,2,2-trifluoroethyl)benzamide, 0.70 g (4.9 mmol) of (trifluoromethyl)trimethylsilane, and 20 mL of tetrahydrofuran, 0.2 mL (0.2 mmol) of tetrabutylammonium fluoride (about 1 mol/L tetrahydrofuran solution) was added under ice cooling, and the mixture was stirred at 0°C for 4 hours. The solvent was distilled off in vacuo, ethyl acetate was added to the residue. The mixture was washed with a saturated aqueous sodium bicarbonate solution, and dried over anhydrous sodium sulfate. The solvent was distilled off in vacuo, and the residue was purified by silica gel column chromatography (developing solvent, n-hexane : ethyl acetate = 5 : 1) to obtain 0.47 g (yield: 56%) of a target product.
¹H-NMR data (300 MHz, CDCl₃/TMS δ (ppm)): 1.18 (3H, d), 2.27-2.40 (1H, m), 2.94 (1H, dd), 3.13 (1H, dd), 3.95 (1H, dd), 4.06 (1H, dd), 4.14 (2H, m), 6.81 (1H, brs), 7.17 (1H, d), 7.42 (1H, d)

### [Example 4]

### Production of N-acetyl-2-chloro-4-fluoro-N-(2,2,2-trifluoroethyl)-5-({5-[(trifluoromethyl)thio]pentyl}oxy)benzamide (compound of present invention No.: A-6814)

To a mixture of 0.30 g (0.68 mmol) of 2-chloro-4-fluoro-N-(2,2,2-trifluoroethyl)-5-({5-[(trifluoromethyl)thio]pentyl}oxy)benzamide in 12 mL of N,N-dimethylformamide, 0.038 g (purity: 55%, 0.87 mmol) of sodium hydride was added at room temperature, and the mixture was stirred at room temperature for 30 minutes. Thereafter, 0.069 g (0.88 mmol) of acetyl chloride was added under ice cooling, and the mixture was further stirred at room temperature for 1.5 hours. Ethyl acetate and hexane were added thereto, and the mixture was washed with a saturated aqueous sodium bicarbonate solution and brine, and dried over anhydrous magnesium sulfate. The solvent was distilled off in vacuo, and the residue was purified by silica gel column chromatography (developing solvent, n-hexane : ethyl acetate = 5 : 1) to obtain 0.22 g (yield: 67%) of a target product.
¹H-NMR data (300 MHz, CDCl₃/TMS δ (ppm)): 1.56-1.66 (2H, m), 1.74-1.91 (4H, m), 2.37 (3H, s), 2.91 (2H, t), 4.04 (2H, t), 4.40 (2H, brd), 7.02 (1H, d), 7.18 (1H, d)

### [Example 5]

### Production of 2-chloro-N-(cyanomethyl)-4-fluoro-N-(2,2,2-trifluoroethyl)-5-({5-[(trifluoromethyl)thio]pentyl}oxy)benzamide (compound of present invention No.: A-6692)

To a mixture of 0.40 g (0.91 mmol) of 2-chloro-4-fluoro-N-(2,2,2-trifluoroethyl)-5-((5-((trifluoromethyl)thio)pentyl)oxy)benzamide in 20 mL of N,N-dimethylformamide, 0.124 g (purity: 55%, 2.84 mmol) of sodium hydride was added at room temperature, and the mixture was stirred at room temperature for 30 minutes.

Thereafter, 0.55 g (4.6 mmol) of bromoacetonitrile was added thereto at room temperature, and the mixture was further stirred at room temperature for 3 hours. Ethyl acetate and hexane were added thereto, and the mixture was washed with a saturated aqueous sodium bicarbonate solution and brine, and dried over anhydrous magnesium sulfate. The solvent was distilled off in vacuo, and the residue was purified by silica gel column chromatography (developing solvent, n-hexane : ethyl acetate = 5 : 1) to obtain 0.18 g (yield: 41%) of a target product.
¹H-NMR data (300 MHz, CDCl₃/TMS δ (ppm)): 1.60-1.66 (2H, m), 1.72-1.88 (4H, m), 2.91 (2H, t), 3.86-4.91 (6H, m), 6.89-6.97 (1H, m), 7.15-7.26 (1H, m)

### [Example 6]

### Production of 4-fluoro-2-methyl-N-(2,2,2-trifluoroethyl)-5-({5-[(trifluoromethyl)thio]pentyl}oxy)benzamide (compound of present invention No.: A-4712)

### (1) Production of 2-fluoro-4-methylphenyl methanesulfonate

To a mixture of 6.03 g (47.8 mmol) of 2-fluoro-4-methylphenol, 5.31 g (52.5 mmol) of triethylamine, and 70 mL of dichloromethane, 6.02 g (52.6 mmol) of methanesulfonyl chloride was added under ice cooling, and the mixture was stirred at room temperature for 5 hours.

Then, 2N hydrochloric acid was added thereto, and the mixture was subjected to extraction with chloroform. The obtained organic layer was washed with brine, and dried over anhydrous magnesium sulfate. The solvent was distilled off in vacuo, and the residue was purified by silica gel column chromatography (developing solvent, n-hexane : ethyl acetate = 5 : 1) to obtain 9.76 g (yield: quantitative) of a target product.
¹H-NMR data (400 MHz, CDCl₃/TMS δ (ppm)): 2.36 (3H, s), 3.19 (3H, s), 6.97 (1H, d), 7.02 (1H, d), 7.25 (1H, t)

### (2) Production of 2-fluoro-5-iodo-4-methylphenyl methanesulfonate

A mixture of 2.18 g (8.59 mmol) of powdery iodine, 1.00 g (4.67 mmol) of potassium iodate, and 90 mL of a 90% aqueous sulfuric acid solution was stirred at 50°C for 1 hour. Further, a mixture of 4.00 g (19.6 mmol) of 2-fluoro-4-methylphenyl methanesulfonate and 10 mL of 90% sulfuric acid was added dropwise at 0°C. The mixture was stirred for 1.5 hours at 0°C. The mixture was added dropwise to ice water, and this was subjected to extraction with toluene. The obtained organic layer was washed with an aqueous sodium thiosulfate solution, a saturated aqueous sodium bicarbonate solution, and brine, and dried over anhydrous magnesium sulfate. The solid obtained by distilling off the solvent in vacuo was washed with a mixed solution of hexane and isopropyl ether, and then filtered, followed by drying to obtain 6.14 g (yield: 95%) of a target product.
¹H-NMR data (400 MHz, CDCl₃/TMS δ (ppm)): 2.43 (3H, s), 3.22 (3H, s), 7.12 (1H, d), 7.78 (1H, d)

### (3) Production of 2-fluoro-5-iodo-4-methylphenol

To a mixture of 8.05 g (24.4 mmol) of 2-fluoro-5-iodo-4-methylphenyl methanesulfonate and 30 mL of water, 2.93 g (73.3 mmol) of sodium hydroxide was added at room temperature, and the mixture was stirred under reflux for 3 hours. Then, 2N hydrochloric acid was added thereto, and the mixture was subjected to extraction with ethyl acetate. The obtained organic layer was washed with brine, and dried over anhydrous magnesium sulfate. The solvent was distilled off in vacuo, and the residue was purified by silica gel column chromatography (developing solvent, n-hexane : ethyl acetate = 7 : 1) to obtain 6.18 g (yield: quantitative) of a target product.
¹H-NMR data (400 MHz, CDCl₃/TMS δ (ppm)): 2.33 (3H, s), 5.47 (1H, brs), 6.96 (1H, d), 7.44 (1H, d)

### (4) Production of 1-[(5-bromopentyl)oxy]-2-fluoro-5-iodo-4-methylbenzene

To a solution of 5.00 g (19.8 mmol) of 2-fluoro-5-iodo-4-methylphenol, 22.8 g (99.2 mmol) of 1,5-dibromopentane, and 250 mL of acetonitrile, 3.56 g (25.8 mmol) of potassium carbonate and 0.64 g (1.99 mmol) of tetrabutylammonium bromide were added at room temperature, and the mixture was stirred under reflux for 2 hours. The mixture was allowed to cool at room temperature and then filtered, and the solvent was distilled off in vacuo (0.1 mmHg, 65°C). The residue was purified by silica gel column chromatography (developing solvent, n-hexane : ethyl acetate = 20 : 1) to obtain 7.17 g (yield: 90%) of a target product.
¹H-NMR data (300 MHz, CDCl₃/TMS δ (ppm)): 1.60-1.68 (2H, m), 1.78-1.88 (2H, m), 1.89-1.99 (2H, m), 2.34 (3H, s), 3.44 (2H, t), 3.99 (2H, t), 6.96 (1H, d), 7.34 (1H, d)

### (5) Production of 1-fluoro-4-iodo-5-methyl-2-((5-thiocyanatopentyl)oxy)benzene

To a mixed solution of 7.07 g (17.6 mmol) of 1-((5-bromopentyl)oxy)-2-fluoro-5-iodo-4-methylbenzene and 80 mL of ethanol, 3.47 g (35.7 mmol) of potassium thiocyanate was added at room temperature, and the mixture was stirred under reflux for 4 hours.

The mixture was allowed to cool at room temperature and then filtered, and the solvent was distilled off in vacuo. Ethyl acetate was added to the residue, and this was washed with brine and dried over anhydrous magnesium sulfate. The solvent was distilled off in vacuo, and the residue was purified by silica gel column chromatography (developing solvent, n-hexane : ethyl acetate = 9 : 1) to obtain 6.10 g (yield: 91%) of a target product.
¹H-NMR data (300 MHz, CDCl₃/TMS δ (ppm)): 1.60-1.70 (2H, m), 1.80-1.97 (4H, m), 2.34 (3H, s), 2.98 (2H, t), 4.00 (2H, t), 6.96 (1H, d), 7.34 (1H, d)

### (6) Production of [5-(2-fluoro-5-iodo-4-methylphenoxy)pentyl](trifluoromethyl)sulfane

To a mixture of 5.99 g (15.8 mmol) of 1-fluoro-4-iodo-5-methyl-2-[(5-thiocyanatopentyl)oxy]benzene, 8.98 g (63.2 mmol) of (trifluoromethyl)trimethylsilane, and 80 mL of tetrahydrofuran, 1.6 mL (1.6 mmol) of tetrabutylammonium fluoride (about 1 mol/L tetrahydrofuran solution) was added under ice cooling, and the mixture was stirred for 2.5 hours while the same temperature was maintained. The solvent was distilled off in vacuo, ethyl acetate was added to the residue, and this was washed with brine and dried over anhydrous magnesium sulfate. The solvent was distilled off in vacuo, and the residue was purified by silica gel column chromatography (developing solvent, n-hexane : ethyl acetate = 15 : 1) to obtain 6.44 g (yield: 97%) of a target product.
¹H-NMR data (300 MHz, CDCl₃/TMS δ (ppm)): 1.54-1.65 (2H, m), 1.73-1.87 (4H, m), 2.34 (3H, s), 2.91 (2H, t), 3.98 (2H, t), 6.96 (1H, d), 7.33 (1H, d)

### (7) Production of 4-fluoro-2-methyl-5-({5-[(trifluoromethyl)thio]pentyl}oxy)benzoic acid (compound of present invention No.: F-0965)

A mixed solution of 1.93 g (28.4 mmol) of sodium formate, 1.93 g (18.9 mmol) of acetic anhydride, 2.45 g (19.0 mmol) of N,N-diisopropylethylamine, and 17 mL of N,N-dimethylformamide was stirred at room temperature for 1 hour under a nitrogen stream.

The resulting mixed solution was added to a mixed solution of 2.00 g (4.74 mmol) of (5-(2-fluoro-5-iodo-4-methylphenoxy)pentyl) (trifluoromethyl)sulfane, 0.22 g (0.24 mmol) of tris(dibenzylideneacetone)dipalladium, 0.60 g (14 mmol) of lithium chloride, and 20 mL of N,N-dimethylformamide at room temperature, and the mixture was stirred at 80°C for 3 hours. After the mixture was allowed to cool to room temperature, a mixed solution obtained by stirring 1.93 g (28.4 mmol) of sodium formate, 1.93 g (18.9 mmol) of acetic anhydride, 2.45 g (19.0 mmol) of N,N-diisopropylethylamine, and 17 mL of N,N-dimethylformamide at room temperature for 0.5 hours under a nitrogen stream was added thereto. The mixture was stirred at 80°C for 1 hour. After the mixture was allowed to cool to room temperature, ethyl acetate was added thereto. The mixture was washed with 2N hydrochloric acid and brine, and dried over anhydrous magnesium sulfate. The solvent was distilled off in vacuo to obtain 2.12 g of a crude target product.
¹H-NMR data (300 MHz, CDCl₃/TMS δ (ppm)): 1.57-1.66 (2H, m), 1.74-1.91 (4H, m), 2.57 (3H, s), 2.92 (2H, t), 4.06 (2H, t), 7.10 (1H, d), 7.67 (1H, d)

### (8) Production of 4-fluoro-2-methyl-N-(2,2,2-trifluoroethyl)-5-({5-[(trifluoromethyl)thio]pentyl}oxy)benzamide (compound of present invention No.: A-4712)

To a mixture of 2.12 g of crude 4-fluoro-2-methyl-5-({5-[(trifluoromethyl)thio]pentyl}oxy)benzoic acid, 1.09 g (5.69 mmol) of 1-ethyl-3-(3-dimethylaminopropyl)carbodiimide hydrochloride, 0.87 g (5.7 mmol) of 1-hydroxybenzotriazole monohydrate, 0.94 g (9.5 mmol) of 2,2,2-trifluoroethylamine, 15 mL of tetrahydrofuran, and 15 mL of dichloromethane, 0.96 g (9.5 mmol) of triethylamine was added under ice cooling, and the mixture was stirred at room temperature for 20 hours. The solvent was distilled off from the reaction mixture in vacuo, and ethyl acetate was added to the residue. The organic layer was washed with a saturated aqueous sodium bicarbonate solution and brine, and dried over anhydrous magnesium sulfate. The solvent was distilled off in vacuo, and the residue was purified by silica gel column chromatography (developing solvent, n-hexane : ethyl acetate = 5 : 1) to obtain 0.97 g (yield: 49%, 2 processes) of a target product.
¹H-NMR data (300 MHz, CDCl₃/TMS δ (ppm)): 1.56-1.66 (2H, m), 1.73-1.89 (4H, m), 2.36 (3H, s), 2.92 (2H, t), 4.02 (2H, t), 4.04-4.15 (2H, m), 5.95 (1H, brs), 6.95 (1H, d), 7.00 (1H, d)

### [Example 7]

### Production of 2-chloro-4-fluoro-N-(2,2,2-trifluoroethyl)-5-({5-[(trifluoromethyl)thio]pentyl}oxy)benzamide (compound of present invention No.: A-4689)

### (1) Production of 4-chloro-2-fluorophenyl methanesulfonate

To a mixture of 40.0 g (273 mmol) of 4-chloro-2-fluorophenol, 30.4 g (300 mmol) of triethylamine, and 400 mL of dichloromethane, 34.4 g (300 mmol) of methanesulfonyl chloride was added under ice cooling, and the mixture was stirred at room temperature for 19 hours. Then, 2N hydrochloric acid was added thereto, and the mixture was subjected to extraction with chloroform. The obtained organic layer was washed with brine, and dried over anhydrous magnesium sulfate. The solvent was distilled off in vacuo to obtain 64.47 g of a crude target product.
¹H-NMR data (300 MHz, CDCl₃/TMS δ (ppm)): 7.16-7.21 (1H, m), 7.26 (1H, dd), 7.33 (1H, t)

### (2) Production of 4-chloro-2-fluoro-5-iodophenyl methanesulfonate

A mixture of 30.48 g (120.1 mmol) of powdery iodine, 14.02 g (65.5 mmol) of potassium iodate, and 1,200 mL of a 90% aqueous sulfuric acid solution was stirred at 50°C for 1 hour. Further, a mixture of 64.47 g of crude 4-chloro-2-fluorophenyl methanesulfonate and 50 mL of 90% sulfuric acid was added dropwise at 0°C. After the dropwise addition, the mixture was stirred for 3 hours at 0°C. The mixture was added dropwise to ice water, and this was subjected to extraction with toluene. The obtained organic layer was washed with a saturated aqueous sodium bicarbonate solution and brine, and dried over anhydrous magnesium sulfate. The solid obtained by distilling off the solvent in vacuo was washed with a mixed solution of hexane and isopropyl ether, and then filtered, followed by drying to obtain 67.05 g (yield: 70%, 2 processes) of a target product.
¹H-NMR data (400 MHz, CDCl₃/TMS δ (ppm)): 3.25 (3H, s), 7.37 (1H, d), 7.84 (1H, d)

### (3) Production of 4-chloro-5-cyano-2-fluorophenyl methanesulfonate

To 15.0 g (42.8 mmol) of 4-chloro-2-fluoro-5 iodophenyl methanesulfonate, 5.02 g (42.8 mmol) of zinc cyanide, and 250 mL of N,N-dimethylformamide, 4.50 g (3.89 mmol) of tetrakis(triphenylphosphine)palladium was added at room temperature, and the mixture was stirred at 80°C for 4.5 hours. After the mixture was allowed to cool at room temperature, ethyl acetate and hexane were added. The mixture was washed with a saturated aqueous sodium bicarbonate solution and brine, and then dried over anhydrous magnesium sulfate. The solvent was distilled off in vacuo, and the residue was purified by silica gel column chromatography (developing solvent, n-hexane : ethyl acetate = 8 : 1) to obtain 9.02 g (yield: 84%) of a target product.
¹H-NMR data (400 MHz, CDCl₃/TMS δ (ppm)): 3.31 (3H, s), 7.44 (1H, d), 7.73 (1H, d)

### (4) Production of 2-chloro-4-fluoro-5-hydroxybenzoic acid

A mixed solution of 21.80 g (87.33 mmol) of 4-chloro-5-cyano-2-fluorophenyl methanesulfonate and 320 mL of a 60% aqueous sulfuric acid solution was stirred at 120°C for 27 hours. The mixed solution was allowed to cool at room temperature, and then added to ice water. This was subjected to extraction with toluene, and the obtained organic layer was dried over anhydrous magnesium sulfate. The solid obtained by distilling off the solvent in vacuo was washed with a chloroform solution, and then filtered, followed by drying to obtain 10.39 g (yield: 62%) of a target product.
¹H-NMR data (400 MHz, DMSO-d6/TMS δ (ppm)): 7.42 (1H, d), 7.44 (1H, d)

### (5) Production of 2-chloro-4-fluoro-5-hydroxy-N-(2,2,2-trifluoroethyl)benzamide (compound of present invention No.: E-0006)

To a mixture of 9.46 g (49.6 mmol) of 2-chloro-4-fluoro-5-hydroxybenzoic acid, 11.42 g (59.57 mmol) of 1-ethyl-3-(3-dimethylaminopropyl)carbodiimide hydrochloride, 9.11 g (59.5 mmol) of 1-hydroxybenzotriazole monohydrate, 9.84 g (99.3 mmol) of 2,2,2-trifluoroethylamine, and 500 mL of N,N-dimethylformamide, 10.05 g (99.32 mmol) of triethylamine was added under ice cooling, and the mixture was stirred at room temperature for 14 hours. Ethyl acetate was added thereto, and the mixture was washed with 2N hydrochloric acid, and dried over anhydrous magnesium sulfate. The solvent was distilled off in vacuo, and the residue was purified by silica gel column chromatography (developing solvent, n-hexane : ethyl acetate = 1 : 3) to obtain 12.55 g (yield: 93%) of a target product.
¹H-NMR data (400 MHz, DMSO/TMS δ (ppm)): 4.00-4.09 (2H, m), 6.99 (1H, d), 7.44 (1H, d), 9.09 (1H, t), 10.48 (1H, s)

### (6) Production of 5-[(5-bromopentyl)oxy]-2-chloro-4-fluoro-N-(2,2,2-trifluoroethyl)benzamide (compound of present invention No.: A-0030)

To a solution of 1.09 g (4.01 mmol) of 2-chloro-4-fluoro-5-hydroxy-N-(2,2,2-trifluoroethyl)benzamide, 4.99 g (21.7 mmol) of 1,5-dibromopentane, and 50 mL of N,N-dimethylformamide, 0.78 g (5.6 mmol) of potassium carbonate and 0.14 g (0.43 mmol) of tetrabutylammonium bromide were added at room temperature, and the mixture was stirred at 100°C for 2.5 hours. The mixture was allowed to cool to room temperature and then filtered, and ethyl acetate was added thereto. The mixture was washed with water, and dried over anhydrous magnesium sulfate. The solvent was distilled off in vacuo, and the residue was purified by silica gel column chromatography (developing solvent, n-hexane : ethyl acetate = 5 : 1) to obtain 0.87 g (yield: 52%) of a target product.
¹H-NMR data (400 MHz, CDCl₃/TMS δ (ppm)): 1.60-1.67 (2H, m), 1.83-1.98 (4H, m), 3.44 (2H, t), 4.06 (2H, t), 4.09-4.17 (2H, m), 6.79 (1H, brs), 7.15 (1H, d), 7.41 (1H, d)

### (7) Production of 2-chloro-4-fluoro-5-[(5-thiocyanatopentyl)oxy]-N-(2,2,2-trifluoroethyl)benzamide (compound of present invention No.: A-0752)

To a mixed solution of 0.79 g (1.9 mmol) of 5-[(5-bromopentyl)oxy]-2-chloro-4-fluoro-N-(2,2,2-trifluoroethyl)benzamide and 20 mL of ethanol, 0.37 g (3.8 mmol) of potassium thiocyanate was added at room temperature, and the mixture was stirred under reflux for 4 hours. The mixture was allowed to cool at room temperature and then filtered, and the solvent was distilled off in vacuo. Ethyl acetate was added to the residue, and this was washed with brine and dried over anhydrous magnesium sulfate. The solvent was distilled off in vacuo, and the residue was purified by silica gel column chromatography (developing solvent, n-hexane : ethyl acetate = 5 : 1) to obtain 0.66 g (yield: 88%) of a target product.
¹H-NMR data (300 MHz, CDCl₃/TMS δ (ppm)): 1.61-1.71 (2H, m), 1.84-1.97 (4H, m), 2.99 (2H, t), 4.06-4.19 (4H, m), 6.79 (1H, brs), 7.17 (1H, d), 7.42 (1H, d)

### (8) Production of 2-chloro-4-fluoro-N-(2,2,2-trifluoroethyl)-5-({5-[(trifluoromethyl)thio]pentyl}oxy)benzamide (compound of present invention No.: A-4689)

To a mixture of 0.58 g (1.5 mmol) of 2-chloro-4-fluoro-5-[(5-thiocyanatopentyl)oxy]-N-(2,2,2-trifluoroethyl)benzamide, 0.88 g (6.2 mmol) of (trifluoromethyl)trimethylsilane, and 10 mL of tetrahydrofuran, 0.16 mL (0.16 mmol) of tetrabutylammonium fluoride (about 1 mol/L tetrahydrofuran solution) was added under ice cooling, and the mixture was stirred for 3 hours. The solvent was distilled off in vacuo, and the residue was purified by silica gel column chromatography (developing solvent, n-hexane : ethyl acetate = 7 : 1) to obtain 0.60 g (yield: 93%) of a target product.
¹H-NMR data (400 MHz, CDCl₃/TMS δ (ppm)): 1.57-1.64 (2H, m), 1.75-1.89 (4H, m), 2.91 (2H, t), 4.06 (2H, t), 4.09-4.18 (2H, m), 6.77 (1H, brs), 7.16 (1H, d), 7.43 (1H, d)

### [Example 8]

### Production of 2-chloro-4-fluoro-N-(2,2,2,-trifluoroethyl)-5-({5-[(trifluoromethyl)thio]pentyl}oxy)thiobenzamide (compound of present invention No.: A-5172)

A mixture of 0.16 g (0.36 mmol) of 2-chloro-4-fluoro-N-(2,2,2-trifluoroethyl)-5-({5-[(trifluoromethyl)thio]pentyl}oxy)benzamide, 0.15 g (0.37 mmol) of a Lawesson's reagent, and 15 mL of toluene was heated reflux for 15 hours. After the mixture was cooled at room temperature, the precipitated solid was removed by filtration, and the filtrate was concentrated in vacuo. The residue was purified by silica gel column chromatography (developing solvent, n-hexane : ethyl acetate = 4 : 1) to obtain 0.15 g (yield: 90%) of a target product.
¹H-NMR data (400 MHz, CDCl₃/TMS δ (ppm)): 1.54-1.64 (2H, m), 1.74-1.89 (4H, m), 2.91 (2H, t), 4.05 (2H, t), 4.55-4.63 (2H, m), 7.12 (1H, d), 7.25 (1H, d), 7.52 (1H, brs)

### [Example 9]

### Production of ethyl 4-fluoro-5-hydroxy-2-methylbenzoate

### (1) Production of 5-bromo-4-fluoro-2-methylbenzoic acid

To a mixture of 20.00 g (129.8 mmol) of 4-fluoro-2-methylbenzoic acid and 100 mL of concentrated sulfuric acid, 24.24 g (136.2 mmol) of N-bromosuccinimide was added at 0°C or lower, and the mixture was stirred at room temperature overnight. The mixture was poured into ice water, and the precipitated solid was filtered and washed with hexane and water. The obtained solid was dissolved in ethyl acetate, washed with water and brine, and dried over anhydrous magnesium sulfate. The solvent was distilled off in vacuo to obtain 30.30 g of a crude target product (including regioisomers). This was directly used in the next process without purification.
¹H-NMR data (400 MHz, CDCl₃/TMS δ (ppm)): 2.51 (3H, t), 7.37 (1H, d), 8.07 (1H, d), 13.23 (1H, brs)

### (2) Production of ethyl 5-bromo-4-fluoro-2-methylbenzoate

To a mixture of 30.30 g of crude 5-bromo-4-fluoro-2-methylbenzoic acid, 19.76 g (0.143 mol) of potassium carbonate, and 200 mL of N,N-dimethylformamide, 22.25 g (0.143 mol) of ethyl iodide was added under ice cooling. The mixture was stirred at room temperature overnight, and ethyl acetate and hexane were added thereto. The mixture was washed with water and brine, and dried over anhydrous magnesium sulfate. The solvent was distilled off in vacuo and purified by recrystallization with hexane to obtain 16.97 g (yield: 49%, 2 processes) of a target product.
¹H-NMR data (400 MHz, CDCl₃/TMS δ (ppm)): 1.40 (3H, t), 2.57 (3H, s), 4.35 (2H, q), 7.00 (1H, d), 8.14 (1H, d)

### (3) Production of [5-(ethoxycarbonyl)-2-fluoro-4-methylphenyl]boronic acid

To a mixture of 2.0 g (7.7 mmol) of ethyl 5-bromo-4-fluoro-2-methylbenzoate and 20 mL of tert-butyl methyl ether, 17.01 mL (1.0 M THF solution, 17.01 mmol) of isopropylmagnesium chloride was added at -60°C. After the mixture was stirred at room temperature for 1 hour, 2.16 g (11.5 mmol) of triisopropyl borate was added thereto at - 60°C, and the mixture was stirred at room temperature overnight. A 15% aqueous sulfuric acid solution was added under ice cooling, and this was further stirred at room temperature for 4 hours. Ethyl acetate was added thereto, and the mixture was washed with water and brine, and dried over anhydrous magnesium sulfate. The solvent was distilled off in vacuo, and the obtained solid was washed with hexane to obtain 1.23 g (yield: 71%) of a target product.
¹H-NMR data (400 MHz, CDCl₃/TMS δ (ppm)): 1.40 (3H, t), 2.63 (3H, s), 4.36 (2H, q), 5.29 (2H, d), 6.93 (1H, d), 8.40 (1H, d)

### (4) Production of ethyl 4-fluoro-5-hydroxy-2-methylbenzoate

To a mixture of 4.03 g (17.8 mmol) of [5-(ethoxycarbonyl)-2-fluoro-4-methylphenyl]boronic acid and 70 mL of toluene, 19.61 mL (50% aqueous solution, about 4.8 mol/L, 94 mmol) of 4-methylmorpholine-N-oxide was added at room temperature, and the mixture was stirred under reflux for 8 hours. After the mixture was allowed to cool at room temperature, 2N hydrochloric acid and brine were added thereto. The obtained organic layer was dried over anhydrous magnesium sulfate. The solvent was distilled off in vacuo, and the residue was purified by silica gel column chromatography (developing solvent, n-hexane : ethyl acetate = 5 : 1) to obtain 2.89 g (yield: 82%) of a target product.
¹H-NMR data (400 MHz, CDCl₃/TMS δ (ppm)): 1.38 (3H, t), 2.52 (3H, s), 4.33 (2H, q), 5.13 (1H, brs), 6.94 (1H, d), 7.62 (1H, d)

### [Example 10]

### Production of N-acetyl-2-chloro-4-fluoro-N-(2,2,2-trifluoroethyl)-5-{2-[(trifluoromethyl)sulfonamido]propoxy} benzamide (compound of present invention No.: C-4214)

### (1) Production of tert-butyl(1-{4-chloro-2-fluoro-5-[(2,2,2-trifluoroethyl)carbamoyl]phenoxy}propan-2-yl)[(trifluoromethyl)sulfonyl]carbamate (compound of present invention No.: C-9422)

To a mixture of 0.60 g (1.3 mmol) of 2-chloro-4-fluoro-N-(2,2,2-trifluoroethyl)-5-{2-[(trifluoromethyl)sulfonamido]propoxy}benzamide (compound of present invention No.: C-0004) produced by a similar method to that of Example 2 (1) and (2), 0.16 g (1.6 mmol) of triethylamine, 15.9 mg (0.130 mmol) of 4-dimethylaminopyridine, and 4 mL of tetrahydrofuran, 0.28 g (1.3 mmol) of di-tert-butyl dicarbonate was added at room temperature, and the mixture was stirred at room temperature for 24 hours. An aqueous citric acid solution was added to the mixture, and the mixture was subjected extraction with ethyl acetate. The obtained organic layer was washed with water and brine, and dried over anhydrous magnesium sulfate. The solvent was distilled off in vacuo, and the residue was purified by silica gel column chromatography (developing solvent, n-hexane : ethyl acetate = 2 : 1) to obtain 0.45 g (yield: 62%) of a target product.
¹H-NMR data (400 MHz, CDCl₃/TMS δ (ppm)): 1.50 (9H, s), 1.53 (3H, d), 4.08-4.22 (3H, m), 4.48 (1H, t), 4.71 (1H, m), 6.74 (1H, brs), 7.18 (1H, d), 7.43 (1H, d)

### (2) Production of tert-butyl(1-{5-[acetyl(2,2,2-trifluoroethyl)carbamoyl]-4-chloro-2-fluorophenoxy}propan-2-yl)[(trifluoromethyl)sulfonyl]carbamate (compound of present invention No.: C-10702)

To a mixture of 0.44 g (0.78 mmol) of tert-butyl(1-{4-chloro-2-fluoro-5-[(2,2,2-trifluoroethyl)carbamoyl]phenoxy}propan-2-yl)[(trifluoromethyl)sulfonyl]carbamate and 10 mL of tetrahydrofuran, 44.5 mg (purity: 55%, 1.02 mmol) of sodium hydride was added at room temperature, and the mixture was stirred at room temperature for 10 minutes. Then, 0.08 g (1 mmol) of acetyl chloride was added thereto at room temperature, and the mixture was stirred at room temperature for 2 hours. An aqueous citric acid solution was added to the mixture, and the mixture was subjected extraction with ethyl acetate. The obtained organic layer was washed with water and brine, and dried over anhydrous magnesium sulfate. The solvent was distilled off in vacuo, and the residue was purified by silica gel column chromatography (developing solvent, n-hexane : ethyl acetate = 3 : 1) to obtain 0.36 g (yield: 76%) of a target product.
¹H-NMR data (400 MHz, CDCl₃/TMS δ (ppm)): 1.50 (9H, s), 1.53 (3H, d), 2.37 (3H, s), 4.17 (1H, m), 4.31-4.49 (3H, m), 4.70 (1H, m), 7.04 (1H, d), 7.19 (1H, d)

### (3) Production of N-acetyl-2-chloro-4-fluoro-N-(2,2,2-trifluoroethyl)-5-{2-[(trifluoromethyl)sulfonamido]propoxy} benzamide (compound of present invention No.: C-4214)

To a mixture of 0.30 g (0.50 mmol) of tert-butyl(1-{5-[acetyl(2,2,2-trifluoroethyl)carbamoyl]-4-chloro-2-fluorophenoxy}propan-2-yl)[(trifluoromethyl)sulfonyl]carbamate and 6 mL of chloroform, 3 mL of trifluoroacetic acid was added at room temperature, and the mixture was stirred at room temperature for 4 hours. The solvent was distilled off in vacuo, and the residue was purified by silica gel column chromatography (developing solvent, n-hexane : ethyl acetate = 3 : 1) to obtain 0.19 g (yield: 76%) of a target product.
¹H-NMR data (400 MHz, CDCl₃/TMS δ (ppm)): 1.48 (3H, d), 2.39 (3H, s), 3.99 (3H, m), 4.40 (2H, q), 5.28 (1H, d), 7.03 (1H, d), 7.22 (1H, d)

### [Example 11]

### Production of (S)-2-chloro-N-cyclopropyl-4-fluoro-5-(2-{[1,1,1-trifluoro-N-(methoxymethyl)methyl]sulfonamido}propoxy)benzamide (compound of present invention No.: C-0664)

### (1) Production of 4-chloro-2-fluorophenyl methyl carbonate

To a mixture of 50.00 g (341.2 mmol) of 4-chloro-2-fluorophenol, 15.01 g (375.3 mmol) of sodium hydroxide, and 340 mL of water, 38.69 g (409.5 mmol) of methyl chloroformate was added in an ice bath, and the mixture was stirred at room temperature for 2 hours. Water was added to the mixture, and the mixture was subjected to extraction with hexane. The obtained organic layer was washed with brine, and dried over anhydrous magnesium sulfate. The solvent was distilled off in vacuo to obtain 69.40 g (yield: 99%) of a target product.
¹H-NMR data (400 MHz, CDCl₃/TMS δ (ppm)): 3.94 (3H, s), 7.12-7.20 (2H, m), 7.22 (1H, dd)

### (2) Production of 5-bromo-4-chloro-2-fluorophenyl methyl carbonate

To a mixture of 34.13 g (166.8 mmol) of 4-chloro-2-fluorophenyl methyl carbonate and 140 mL of concentrated sulfuric acid, 26.24 g (91.77 mmol) of 1,3-dibromo-5,5-dimethylhydantoin was added little by little in an ice bath, and the mixture was stirred in an ice bath for 2 hours. The mixture was poured into ice, followed by extraction with dichloromethane. The obtained organic layer was washed with a saturated aqueous sodium bicarbonate solution, an aqueous sodium thiosulfate solution, and brine, and dried over anhydrous magnesium sulfate. The solvent was distilled off in vacuo to obtain an oil, and n-pentane was added to the oil to obtain crystals. The crystals were collected by filtration to obtain 34.06 g (yield: 72%) of a target product.
¹H-NMR data (300 MHz, CDCl₃/TMS δ (ppm)): 3.94 (3H, s), 7.34 (1H, d), 7.52 (1H, d)

### (3) Production of 5-bromo-4-chloro-2-fluorophenol

To a mixture of 22.00 g (77.61 mmol) of 5-bromo-4-chloro-2-fluorophenyl methyl carbonate and 220 mL of methanol, 6.53 g (purity: 85.5%, 99.5 mmol) of potassium hydroxide was added in an ice bath, and the mixture was stirred at room temperature for 1 hour. The solvent was distilled off in vacuo, 1N hydrochloric acid was added to the residue, and the mixture was subjected to extraction with dichloromethane. The obtained organic layer was washed with water and brine, and dried over anhydrous magnesium sulfate. The solvent was distilled off in vacuo to obtain 17.08 g (yield: 98%) of a target product.
¹H-NMR data (300 MHz, CDCl₃/TMS δ (ppm)): 5.36 (1H, brs), 7.22 (1H, d), 7.29 (1H, d)

### (4) Production of (S)-tert-butyl-[1-(5-bromo-4-chloro-2-fluorophenoxy)propan-2-yl]carbamate

To a mixture of 17.08 g (75.76 mmol) of 5-bromo-4-chloro-2-fluorophenol, 15.93 g (90.91 mmol) of (S)-2-(tert-butoxycarbonylamino)-1-propanol, 23.85 g (90.93 mmol) of triphenylphosphine, and 230 mL of tetrahydrofuran, 18.38 g (90.90 mmol) of diisopropyl azodicarboxylate was added under ice cooling, and the mixture was stirred at room temperature for 15 hours. The solvent was distilled off in vacuo, and the residue was purified by silica gel column chromatography (developing solvent, n-hexane : ethyl acetate = 4 : 1). The mixed solution of n-hexane and ethyl acetate after purification was washed with a 0.5 M aqueous sodium hydroxide solution. The solvent was distilled off in vacuo to obtain 24.32 g (yield: 84%) of a target product.
¹H-NMR data (300 MHz, CDCl₃/TMS δ (ppm)): 1.30 (3H, d), 1.45 (9H, s), 3.90-4.10 (3H, m), 4.72 (1H, brs), 7.20 (1H, d), 7.21 (1H, d)

### (5) Production of methyl (S)-5-[2-(tert-butoxycarbonylamino)propoxy]-2-chloro-4-fluorobenzoate (compound of present invention No.: G-0432)

A mixture of 11.70 g (30.58 mmol) of (S)-tert-butyl-[1-(5-bromo-4-chloro-2-fluorophenoxy)propan-2-yl]carbamate, 0.25 g (0.31 mmol) of [1,1'-bis(diphenylphosphino)ferrocene]palladium (II) dichloride dichloromethane adduct, 6.19 g (61.2 mmol) of triethylamine, and 50 mL of methanol was heated with stirring at 100°C for 8.5 hours under a carbon monoxide atmosphere (9.0 kgf/cm²). After the mixture was allowed to cool at room temperature, ethyl acetate was added thereto. Then, insoluble substances were removed by filtration, and the solvent was distilled off in vacuo. The residue was purified by silica gel column chromatography (developing solvent, n-hexane : ethyl acetate = 4 : 1) to obtain 9.91 g (yield: 90%) of a target product.
¹H-NMR data (300 MHz, CDCl₃/TMS δ (ppm)): 1.31 (3H, d), 1.45 (9H, s), 3.92 (3H, s), 3.98-4.13 (3H, m), 4.75 (1H, brs), 7.19 (1H, d), 7.50 (1H, d)

### (6) Production of methyl (S)-5-(2-aminopropoxy)-2-chloro-4-fluorobenzoate hydrochloride (compound of present invention No.: G-0383)

To a mixture of 19.15 g (52.93 mmol) of methyl (S)-5-[2-(tert-butoxycarbonylamino)propoxy]-2-chloro-4-fluorobenzoate and 80 mL of 1,4-dioxane, 80 mL of a 1,4-dioxane solution of 4N hydrogen chloride was added at room temperature, and the mixture was stirred at room temperature for 6 hours. The solvent was distilled off in vacuo to obtain 15.10 g (yield: 96%) of a target product.
¹H-NMR data (300 MHz, DMSO-d6/TMS δ (ppm)): 1.31 (3H, d), 3.61 (1H, m), 3.87 (3H, s), 4.18-4.30 (2H, m), 7.66 (1H, d), 7.67 (1H, d), 8.38 (3H, brs)

### (7) Production of methyl (S)-2-chloro-4-fluoro-5-{2-[(trifluoromethyl)sulfonamido]propoxy}benzoate (compound of present invention No.: G-0518)

To a mixture of 15.10 g (50.65 mmol) of methyl (S)-5-(2-aminopropoxy)-2-chloro-4-fluorobenzoate hydrochloride, 15.38 g (152.0 mmol) of triethylamine, 6.34 g of molecular sieve (4A), 200 mL of tetrahydrofuran, and 200 mL of dichloromethane, 11.10 g (65.87 mmol) of trifluoromethanesulfonyl chloride was added under ice cooling, and the mixture was stirred at room temperature for 24 hours. The mixture was filtered, the solvent was distilled off in vacuo, and then ethyl acetate was added to the residue. The organic layer was washed with 1N hydrochloric acid, water, and brine, and dried over anhydrous magnesium sulfate. The solvent was distilled off in vacuo, and the residue was purified by silica gel column chromatography (developing solvent, n-hexane : ethyl acetate = 3 : 1) to obtain 13.84 g (yield: 69%) of a target product.
¹H-NMR data (300 MHz, CDCl₃/TMS δ (ppm)): 1.49 (3H, d), 3.93 (3H, s), 4.02 (3H, m) 5.42 (1H, brs), 7.23 (1H, s), 7.49 (1H, d)

### (8) Production of (S)-2-chloro-4-fluoro-5-{2-[(trifluoromethyl)sulfonamido]propoxy}benzoic acid (compound of present invention No.: F-0511)

To a mixture of 13.84 g (35.15 mmol) of methyl (S)-2-chloro-4-fluoro-5-{2-[(trifluoromethyl)sulfonamido]propoxy}benzoate, 30 mL of tetrahydrofuran, 150 mL of ethanol, and 40 mL of water, 4.42 g (105 mmol) of lithium hydroxide monohydrate was added at room temperature, and the mixture was stirred at room temperature for 15 hours. The solvent was distilled off in vacuo, 1N hydrochloric acid was added to the residue, and the mixture was subjected to extraction with ethyl acetate. The obtained organic layer was washed with water and brine, and dried over anhydrous magnesium sulfate. The solvent was distilled off in vacuo to obtain 12.07 g (yield: 90%) of a target product.
¹H-NMR data (300 MHz, DMSO-d6/TMS δ (ppm)): 1.25 (3H, d), 3.87 (1H, m), 3.99 (1H, m), 4.16 (1H, dd), 7.57 (1H, d), 7.59 (1H, d), 9.65 (1H, brs)

### (9) Production of (S)-2-chloro-N-cyclopropyl-4-fluoro-5-{2-[(trifluoromethyl)sulfonamido]propoxy}benzamide (compound of present invention No.: C-0130)

To a mixture of 5.00 g (13.2 mmol) of (S)-2-chloro-4-fluoro-5-{2-[(trifluoromethyl)sulfonamido]propoxy}benzoic acid, 27.6 mg (0.378 mmol) of N,N-dimethylformamide, and 60 mL of dichloromethane, 3.34 g (26.3 mmol) of oxalyl chloride was added at room temperature, and the mixture was stirred for 1 hour. The mixture was concentrated in vacuo, and the residue was dissolved in 20 mL of dichloromethane. The obtained mixture was added dropwise to a mixture of 1.50 g (26.3 mmol) of cyclopropylamine, 2.67 g (26.4 mmol) of triethylamine, 60 mL of dichloromethane, and 60 mL of tetrahydrofuran under ice cooling, followed by stirring at room temperature for 18 hours. The solvent was distilled off in vacuo, 1N hydrochloric acid was added to the residue, and the mixture was subjected to extraction with ethyl acetate. The obtained organic layer was washed with water and brine, and dried over anhydrous magnesium sulfate. The solvent was distilled off in vacuo, and the residue was purified by silica gel column chromatography (developing solvent, n-hexane : ethyl acetate = 1 : 1) to obtain 4.59 g (yield: 83%) of a target product.
¹H-NMR data (300 MHz, DMSO-d6/TMS δ (ppm)): 0.53 (2H, m), 0.69 (2H, m), 1.25 (3H, d), 2.79 (1H, m), 3.87 (1H, m), 3.97 (1H, dd), 4.11 (1H, dd), 7.26 (1H, d), 7.49 (1H, d), 8.45 (1H, brs), 9.63 (1H, brs)

### (10) Production of (S)-2-chloro-N-cyclopropyl-4-fluoro-5-(2-{[1,1,1-trifluoro-N-(methoxymethyl)methyl]sulfonamido}propoxy)benzamide (compound of present invention No.: C-0664)

To a mixture of 1.50 g (3.58 mmol) of (S)-2-chloro-N-cyclopropyl-4-fluoro-5-{2-[(trifluoromethyl)sulfonamido]propoxy}benzamide, 0.54 g (5.3 mmol) of triethylamine, and 30 mL of acetonitrile, 0.43 g (5.3 mmol) of chloromethyl methyl ether was added dropwise under ice cooling, and the mixture was stirred at room temperature for 15 hours. Water was added thereto, and the mixture was subjected to extraction with ethyl acetate. The obtained organic layer was washed with water and brine, and dried over anhydrous magnesium sulfate. The solvent was distilled off in vacuo, and the residue was purified by silica gel column chromatography (developing solvent, n-hexane : ethyl acetate = 1 : 1) to obtain 1.60 g (yield: 97%) of a target product.
¹H-NMR data (300 MHz, CDCl₃/TMS δ (ppm)): 0.66 (2H, m), 0.90 (2H, m), 1.46 (3H, d), 2.92 (1H, m), 3.38 (3H, s), 4.10 (1H, dd), 4.24 (1H, dd), 4.37-4.48 (1H, m), 4.80-4.91 (2H, m), 6.52 (1H, brs), 7.13 (1H, d), 7.40 (1H, d)

### [Example 12]

### Production of (S)-2-chloro-N-(1-cyanocyclopropyl)-4-fluoro-5-(2-{[1,1,1-trifluoro-N-(methoxymethyl)methyl]sulfonamido}propoxy)benzamide (compound of present invention No.: C-3076)

### (1) Production of (S)-2-chloro-N-(1-cyanocyclopropyl)-4-fluoro-5-{2-[(trifluoromethyl)sulfonamido]propoxy}benzamide (compound of present invention No.: C-2980)

To a mixture of 0.14 g (0.37 mmol) of (S)-2-chloro-4-fluoro-5-{2-[(trifluoromethyl)sulfonamido]propoxy}benzoic acid produced in Example 11 (8), 2.7 mg (0.037 mmol) of N,N-dimethylformamide, and 10 mL of dichloromethane, 0.10 g (0.79 mmol) of oxalyl chloride was added at room temperature, and the mixture was stirred for 1 hour. The mixture was concentrated in vacuo, and the residue was dissolved in 3 mL of dichloromethane. The obtained mixture was added dropwise to a mixture of 0.13 g (1.1 mmol) of 1-cyanocyclopropylamine hydrochloride, 0.22 g (2.2 mmol) of triethylamine, and 10 mL of dichloromethane under ice cooling, followed by stirring at room temperature for 18 hours. Then, 1N hydrochloric acid was added thereto, and the mixture was subjected to extraction with dichloromethane. The obtained organic layer was washed with brine, and dried over anhydrous magnesium sulfate. The solvent was distilled off in vacuo, and the residue was purified by silica gel column chromatography (developing solvent, n-hexane : ethyl acetate = 1 : 1) to obtain 0.14 g (yield: 86%) of a target product.
¹H-NMR data (300 MHz, CDCl₃/TMS δ (ppm)): 1.39 (2H, m), 1.46 (3H, d), 1.67 (2H, m), 4.06-4.15 (3H, m), 5.77 (1H, brs), 7.14 (1H, brs), 7.16 (1H, d), 7.43 (1H, d)

### (2) Production of (S)-2-chloro-N-(1-cyanocyclopropyl)-4-fluoro-5-(2-{[1,1,1-trifluoro-N-(methoxymethyl)methyl]sulfonamido}propoxy)benzamide (compound of present invention No.: C-3076)

To a mixture of 0.20 g (0.45 mmol) of (S)-2-chloro-N-(1-cyanocyclopropyl)-4-fluoro-5-{2-[(trifluoromethyl)sulfonamido]propoxy}benzamide, 0.12 g (0.93 mmol) of N,N-diisopropylethylamine, and 5 mL of acetonitrile, 0.07 g (0.9 mmol) of chloromethyl methyl ether was added dropwise under ice cooling, and the mixture was stirred at room temperature for 15 hours. Water was added thereto, and the mixture was subjected to extraction with ethyl acetate. The obtained organic layer was washed with brine, and dried over anhydrous magnesium sulfate. The solvent was distilled off in vacuo, and the residue was purified by silica gel column chromatography (developing solvent, n-hexane : ethyl acetate = 1 : 1) to obtain 0.19 g (yield: 86%) of a target product.
¹H-NMR data (300 MHz, CDCl₃/TMS δ (ppm)): 1.39 (2H, m), 1.49 (3H, d), 1.68 (2H, m), 3.38 (3H, s), 4.11 (1H, dd), 4.25 (1H, dd), 4.44 (1H, m), 4.86 (2H, m), 7.04 (1H, brs), 7.16 (1H, d), 7.47 (1H, d) [0366]

### [Example 13]

### Production of (S)-2-chloro-N-(1-cyanocyclopropyl)-3-methyl-5-{2-[(trifluoromethyl)sulfonamido]propoxy)benzamide

### (1) Production of methyl 2-chloro-5-hydroxy-3-methylbenzoate

To a mixture of 3.5 g (18 mmol) of methyl 5-amino-2-chloro-3-methylbenzoate produced by the method described in WO 2020/117877, 50 mL of concentrated sulfuric acid, and 50 mL of water, 1.5 g (22 mmol) of sodium nitrite was added under ice cooling, and the mixture was stirred at room temperature for 2 hours and then stirred under reflux for 3 hours. The reaction mixture was cooled to room temperature, then ice water was added thereto, and the mixture was subjected to extraction with ethyl acetate. The organic layer was washed with water, and dried over anhydrous magnesium sulfate. The solvent was distilled off in vacuo to obtain a crude mixture containing a target product and 2-chloro-5-hydroxy-3-methylbenzoic acid. The obtained crude mixture containing a target product and 2-chloro-5-hydroxy-3-methylbenzoic acid was dissolved in 100 mL of methanol. Then, 3.7 g (38 mmol) of concentrated sulfuric acid was added thereto at room temperature, and the mixture was stirred under reflux for 8 hours. The solvent was distilled off in vacuo, water was added to the residue, and the mixture was subjected to extraction with ethyl acetate. The organic layer was washed with water, and dried over anhydrous magnesium sulfate. The solvent was distilled off in vacuo, and the residue was purified by silica gel column chromatography (developing solvent, n-hexane : ethyl acetate = 2 : 1) to obtain 1.8 g (yield: 50%) of a target product.
¹H-NMR data (300 MHz, CDCl₃/TMS δ (ppm)): 2.36 (3H, s), 3.92 (3H, s), 5.50 (1H, brs), 6.88 (1H, d), 7.07 (1H, d)

### (2) Production of methyl (S)-5-[2-(tert-butoxycarbonylamino)propoxy]-2-chloro-3-methylbenzoate (compound of present invention No.: H-0001)

To a mixture of 1.8 g (9.0 mmol) of methyl 2-chloro-5-hydroxy-3-methylbenzoate, 1.9 g (11 mmol) of (S)-2-(tert-butoxycarbonylamino)-1-propanol, 2.8 g (11 mmol) of triphenylphosphine, and 100 mL of tetrahydrofuran, 2.2 g (11 mmol) of diisopropyl azodicarboxylate was added under ice cooling, and the mixture was stirred at room temperature for 15 hours. The solvent was distilled off in vacuo, and the residue was purified by silica gel column chromatography (developing solvent, n-hexane : ethyl acetate = 2 : 1) to obtain 1.3 g (yield: 40%) of a target product.
¹H-NMR data (300 MHz, CDCl₃/TMS δ (ppm)): 1.27 (3H, d), 1.45 (9H, s), 2.39 (3H, s), 3.90 (2H, d), 3.92 (3H, s), 4.05 (1H, m), 4.71 (1H, brs), 6.94 (1H, d), 7.10 (1H, d)

### (3) Production of methyl (S)-5-(2-aminopropoxy)-2-chloro-3-methylbenzoate hydrochloride (compound of present invention No.: H-0002)

To a mixture of 1.3 g (3.6 mmol) of methyl (S)-5-[2-(tert-butoxycarbonylamino)propoxy]-2-chloro-3-methylbenzoate and 10 mL of 1,4-dioxane, 3.7 g of a 1,4-dioxane solution of 4N hydrogen chloride was added at room temperature, and the mixture was stirred at room temperature for 16 hours. The solvent was distilled off in vacuo, and the obtained white crystals were washed with ethyl acetate to obtain 880 mg (yield: 82%) of a target product.
¹H-NMR data (300 MHz, DMSO-d6/TMS δ (ppm)): 1.27 (3H, d), 2.36 (3H, s), 3.57 (1H, m), 3.86 (3H, s), 4.02 (1H, dd), 4.15 (1H, dd), 7.19 (1H, d), 7.22 (1H, d), 8.18 (3H, brs)

### (4) Production of methyl (S)-2-chloro-3-methyl-5-{2-[(trifluoromethyl)sulfonamido]propoxy}benzoate (compound of present invention No.: H-0003)

To a mixture of 880 mg (2.99 mmol) of methyl (S)-5-(2-aminopropoxy)-2-chloro-3-methylbenzoate hydrochloride, 908 mg (8.97 mmol) of triethylamine, 100 mg of molecular sieve (4A), 30 mL of tetrahydrofuran, and 60 mL of dichloromethane, 756 mg (4.49 mmol) of trifluoromethanesulfonyl chloride was added under ice cooling, and the mixture was stirred at room temperature for 17 hours. The mixture was filtered, a saturated aqueous sodium bicarbonate solution was added thereto, and the mixture was subjected to extraction with ethyl acetate. The organic layer was washed with 1N hydrochloric acid, and dried over anhydrous magnesium sulfate. The solvent was distilled off in vacuo, and the residue was purified by silica gel column chromatography (developing solvent, n-hexane : ethyl acetate = 3 : 1) to obtain 390 mg (yield: 33%) of a target product.
¹H-NMR data (300 MHz, CDCl₃/TMS δ (ppm)): 1.46 (3H, d), 2.37 (3H, s), 3.94 (3H, s), 3.95 (1H, m), 4.06 (2H, m), 5.14 (1H, brs), 6.94 (1H, d), 7.10 (1H, d)

### (5) Production of (S)-2-chloro-3-methyl-5-{2-[(trifluoromethyl)sulfonamido]propoxy}benzoic acid (compound of present invention No.: H-0004)

To a mixture of 390 mg (1.00 mmol) of methyl (S)-2-chloro-3-methyl-5-{2-[(trifluoromethyl)sulfonamido]propoxy}benzoate, 20 mL of methanol, and 20 mL of water, 126 mg (3.00 mmol) of lithium hydroxide monohydrate was added at room temperature, and the mixture was stirred at room temperature for 15 hours. Then, 1N hydrochloric acid was added thereto, and the mixture was subjected to extraction with ethyl acetate. The obtained organic layer was washed with water, and dried over anhydrous magnesium sulfate. The solvent was distilled off in vacuo to obtain 370 mg (yield: 98%) of a target product.
¹H-NMR data (300 MHz, DMSO-d6/TMS δ (ppm)): 1.23 (3H, d), 2.33 (3H, s), 3.83 (1H, m), 3.85 (1H, dd), 3.99 (1H, dd), 7.06 (1H, d), 7.10 (1H, d)

### (6) Production of (S)-2-chloro-N-(1-cyanocyclopropyl)-3-methyl-5-{2-[(trifluoromethyl)sulfonamido]propoxy) benzamide (compound of present invention No.: H-0005)

To a mixture of 430 mg (1.14 mmol) of (S)-2-chloro-3-methyl-5-{2-[ (trifluoromethyl)sulfonamido]propoxy}benzoic acid, 8.2 mg (0.11 mmol) of N,N-dimethylformamide, and 30 mL of dichloromethane, 436 mg (3.44 mmol) of oxalyl chloride was added at room temperature, and the mixture was stirred for 3 hours. The mixture was concentrated in vacuo, and the residue was dissolved in 10 mL of dichloromethane. The obtained mixture was added dropwise to a mixture of 407 mg (3.43 mmol) of 1-cyanocyclopropylamine hydrochloride, 695 mg (6.87 mmol) of triethylamine, and 30 mL of dichloromethane under ice cooling, followed by stirring at room temperature for 18 hours. Then, 1N hydrochloric acid was added thereto, and the mixture was subjected to extraction with ethyl acetate. The obtained organic layer was washed with a saturated aqueous sodium bicarbonate solution, and dried over anhydrous magnesium sulfate. The solvent was distilled off in vacuo, and the residue was purified by silica gel column chromatography (developing solvent, n-hexane : ethyl acetate = 3 : 2) to obtain 280 mg (yield: 56%) of a target product.
¹H-NMR data (300 MHz, CDCl₃/TMS δ (ppm)): 1.39 (5H, m), 1.63 (2H, dd), 2.34 (3H, s), 3.98 (3H, m), 5.78 (1H, brs), 6.87 (1H, d), 6.93 (1H, d), 6.97 (1H, brs)

### (7) Production of (S)-2-chloro-N-(1-cyanocyclopropyl)-3-methyl-5-(2-{[1,1,1-trifluoro-N-(methoxymethyl)methyl]sulfonamido}propoxy)benzamide (compound of present invention No.: H-0006)

To a mixture of 150 mg (0.341 mmol) of (S)-2-chloro-N-(1-cyanocyclopropyl)-3-methyl-5-{2-[(trifluoromethyl)sulfonamido]propoxy}benzamide, 138 mg (1.36 mmol) of triethylamine, and 30 mL of acetonitrile, 54.9 mg (0.682 mmol) of chloromethyl methyl ether was added dropwise at room temperature, and the mixture was stirred at room temperature for 15 hours. Water was added thereto, and the mixture was subjected to extraction with ethyl acetate. The obtained organic layer was dried over anhydrous magnesium sulfate. The solvent was distilled off in vacuo, and the residue was purified by silica gel column chromatography (developing solvent, n-hexane : ethyl acetate = 1 : 1) to obtain 110.1 mg (yield: 67%) of a target product.
¹H-NMR data (300 MHz, CDCl₃/TMS δ (ppm)): 1.43 (5H, m), 1.67 (2H, dd), 2.37 (3H, s), 3.37 (3H, s), 4.01 (1H, m), 4.14 (1H, m), 4.40 (1H, m), 4.86 (2H, m), 6.67 (1H, brs), 6.90 (1H, d), 7.01 (1H, d) [0374]

Physical property values of the compound [I] of the present invention synthesized in accordance with the above-described Examples, including the values in the above-described Examples, are shown in the following Tables 1234 to 1244, physical property values of the compound [II] of the present invention are similarly shown in Tables 1245 to 1246, and physical property values of the compound [III] of the present invention are similarly shown in Tables 1247 to 1253.

The compound numbers and symbols in the tables are the same as described above.

**[Table 1234]**

| Compound Number | Physical property value | |
|---|---|---|
| | Melting point (°C) | Refractive ndex (n_{D}²⁰) |
| A-0008 | 91-92 | |
| A-0015 | 64-66 | |
| A-0028 | 107-110 | |
| A-0030 | 86-88 | |
| A-0031 | 110-113 | |
| A-0035 | 78-79 | |
| A-0037 | 82-85 | |
| A-0049 | 88-90 | |
| A-0052 | 71-72 | |
| A-0056 | 90-93 | |
| A-0059 | 79-80 | |
| A-0060 | 47-48 | |
| A-0063 | 55-56 | |
| A-0064 | 48-50 | |
| A-0105 | 129-130 | |
| A-0106 | 92-95 | |
| A-0170 | 82-84 | |
| A-0179 | | 1.4858 |
| A-0243 | 117-120 | |
| A-0273 | | 1.4781 |
| A-0367 | 100-103 | |
| A-0368 | 75-78 | |
| A-0369 | | 1.4932 |
| A-0370 | 68-71 | |
| A-0371 | | 1.4941 |
| A-0373 | 153-155 | |
| A-0405 | 63-66 | |
| A-0419 | 111-113 | |
| A-0447 | | 1.4881 |
| A-0474 | 116-119 | |
| A-0475 | | 1.4862 |
| A-0479 | | 1.4862 |
| A-0480 | | 1.4852 |
| A-0507 | 119-121 | |
| A-0512 | 142-144 | |
| A-0538 | 107-109 | |
| A-0598 | 129-130 | |
| A-0724 | 187-190 | |
| A-0726 | 154-156 | |
| A-0751 | 107-108 | |
| A-0752 | 85-87 | |
| A-0753 | 85-87 | |
| A-0809 | 82-83 | |

**[Table 1235]**

| Compound Number | Physical property value | |
|---|---|---|
| | Melting point (°C) | Refractive index (n_{D}²⁰) |
| A-0942 | 66-68 | |
| A-1310 | 122-124 | |
| A-1477 | 73-75 | |
| A-1483 | 90-93 | |
| A-1523 | 100-101 | |
| A-1529 | 62-63 | |
| A-1960 | 145-146 | |
| A-3459 | 97-99 | |
| A-3506 | 161-162 | |
| A-3821 | 152-153 | |
| A-3828 | 131-134 | |
| A-4665 | 64-67 | |
| A-4687 | 96-98 | |
| A-4688 | 74-75 | |
| A-4689 | 88-89 | |
| A-4690 | 81-83 | |
| A-4691 | 81-84 | |
| A-4693 | 103-106 | |
| A-4710 | 91-94 | |
| A-4712 | 88-89 | |
| A-4715 | 76-77 | |
| A-4735 | 94-95 | |
| A-4758 | 88-89 | |
| A-4781 | 75-78 | |
| A-4850 | 56-58 | |
| A-4969 | | 1.5141 |
| A-5011 | 82-83 | |
| A-5172 | 97-100 | |
| A-5797 | 180-183 | |
| A-5977 | 85-86 | |
| A-6161 | 41-43 | |
| A-6482 | | 1.4748 |
| A-6497 | | 1.4718 |
| A-6542 | | 1.4709 |
| A-6572 | | 1.4723 |
| A-6602 | | 1.4641 |
| A-6632 | | 1.4540 |
| A-6662 | | 1.4801 |
| A-6692 | | 1.4820 |
| A-6708 | | 1.4911 |
| A-6709 | | 1.4804 |
| A-6724 | | 1.4809 |
| A-6754 | | 1.4711 |

**[Table 1236]**

| Compound Number | Physical property value | |
|---|---|---|
| | Melting point (°C) | Refractive index (n_{D}²⁰) |
| A-6784 | | 1.4730 |
| A-6814 | | 1.4810 |
| A-6844 | | 1.4760 |
| A-6859 | | 1.4714 |
| A-6889 | | 1.4751 |
| A-6934 | 63-64 | |
| A-7024 | | 1.4698 |
| A-7054 | | 1.4739 |

**[Table 1237]**

| Compound Number | Physical property value [¹H-NMR data TMS δ (ppm)] | |
|---|---|---|
| A-0061 | 300MHz, CDCl₃ | 4.02-4.23 (2H, m), 6.75 (1H, brs), 7.35 (1H, d), 7.73 (1H, d) |
| A-0155 | 300MHz, CDCl₃ | 1.44 (3H, d), 3.33 (1H, q), 3.99-4.06 (2H, m), 4.08-4.20 (2H, m), 6.78 (1H, brs), 7.18 (1H, d), 7.45 (1H, d) |
| A-0156 | 300MHz, CDCl₃ | 1.43 (3H, d), 2.35 (3H, s), 3.88-4.03 (2H, m), 4.08-4.20 (3H, m), 6.85 (1H, brs), 7.18 (1H, d), 7.51 (1H, d) |
| A-0372 | 300MHz, CDCl₃ | 1.17 (3H, d), 1.72 (2H, brs), 3.33-3.45 (1H, m), 3.73 (1H, t), 3.93 (1H, dd), 4.05-4.19 (2H, m), 7.00 (1H, brs), 7.16 (1H, d), 7.38 (1H, d) |
| A-0414 | 300MHz, CDCl₃ | 1.25 (3H, d), 1.43 (9H, s), 3.95-4.19 (3H, m), 4.69 (1H, brs), 4.96 (2H, q), 6.83 (1H, brs), 7.15 (1H, d), 7.44 (1H, d) |
| A-0417 | 300MHz, CDCl₃ | 0.99 (3H, t), 1.20-1.32 (2H, m), 1.44 (9H, s), 3.75-3.92 (1H, m), 4.09-4.20 (2H, m), 4.71 (1H, brs), 4.98 (2H, q), 6.83 (1H, brs), 7.17 (1H, d), 7.46 (1H, d) |
| A-0418 | 400MHz, CDCl₃ | 0.98 (6H, d), 1.44 (9H, s), 1.94-2.10 (1H, m), 3.32-3.78 (1H, m), 4.10-4.23 (2H, m), 4.73 (1H, brs), 4.97 (2H, q), 6.83 (1H, brs), 7.17 (1H, d), 7.44 (1H, d) |
| A-0491 | 400MHz, CDCl₃ | 0.93 (3H, t), 1.55-1.83 (2H, m), 3.17-3.39 (2H, m), 4.04-4.24 (2H, m), 4.27- 4.39 (1H, m), 5.12 (1H, brs), 6.81 (1H, brs), 7.14 (1H, d), 7.42 (1H, d), 7.76 (2H, d), 7.98 (2H, d) |
| A-0758 | 300MHz, CDCl₃ | 1.24 (3H, d), 2.40-2.55 (1H, m), 3.06 (1H, dd), 3.23 (1H, dd), 3.99 (1H, dd), 4.07-4.18 (3H, m), 6.83 (1H, brs), 7.17 (1H, d), 7.42 (1H, d) |
| A-0799 | 300MHz, CDCl₃ | 2.18-2.31 (2H, m), 2.36 (3H, s), 3.75 (2H, t), 4.11 (2H, t), 4.07-4.22 (2H, m), 5.94 (1H, brs), 6.95 (1H, d), 7.30 (1H, d) |
| A-3167 | 300MHz, CDCl₃ | 0.61-0.67 (4H, m), 0.75-0.79 (2H, m), 0.86-0.92 (2H, m), 1.72 (2H, brs), 2.91 (1H, m), 3.92 (2H, s), 651 (1H, brs), 7.12 (1H, d), 7.38 (1H, d) |
| A-3168 | 300MHz, CDCl₃ | 0.62-0.67 (2H, m), 0.83-0.97 (6H, m), 1.45 (9H, s), 2.91 (1H, m), 4.05 (2H, s), 5.15 (1H, brs), 6.42 (1H, brs), 7.11 (1H, d), 7.33 (1H, d) |

**[Table 1238]**

| Com pound Number | Physical property value [¹H-NMR data TMS δ (ppm)] | |
|---|---|---|
| A-3822 | 300MHz, CDCl₃ | 0.62-0.68 (2H, m), 0.86-0.93 (2H, m), 1.88 (1H, brs), 2.28 (1H, m), 2.91 (1H, m), 3.80 (2H, d), 4.09-4.21 (4H, m), 6.53 (1H, brs), 7.12 (1H, d), 7.44 (1H, d) |
| A-3825 | 300MHz, CDCl₃ | 0.62-0.68 (2H, m), 0.86-0.93 (2H, m), 1.45 (9H, s), 2.92 (1H, m), 3.77 (2H, m), 3.99 (1H, dd), 4.06-4.19 (4H, m), 6.57 (1H, brs), 7.12 (1H, d), 7.42 (1H, d) |
| A-3829 | 300MHz, CDCl₃ | 0.62-0.68 (2H, m), 0.86-0.93 (2H, m), 2.55 (1H, m), 2.89 (1H, m), 3.56-3.62 (2H, m), 3.71-3.81 (2H, m), 4.18-4.26 (2H, m), 6.58 (1H, brs), 7.11 (1H, d), 7.39 (1H, d) |
| A-3129 | 300MHz, CDCl₃ | 0.62-0.67 (2H, m), 0.86-0.93 (2H, m), 1.40 (3H, d), 2.39 (1H, m), 2.92 (1H, m), 3.62-3.73 (2H, m), 3.99-4.03 (2H, m), 6.48 (1H, brs), 7.11 (1H, d), 7.42 (1H, d) |
| A-3169 | 300MHz, CDCl₃ | 0.62-0.68 (2H, m), 0.86-0.93 (2H, m), 0.98-1.03 (2H, m), 1.24-1.29 (2H, m), 2.91 (1H, m), 4.04 (2H, s), 6.02 (1H, brs), 6,51 (1H, brs), 7.14 (1H, d), 7.35 (1H, d) |
| A-3820 | 300MHz, CDCl₃ | 0.62-0.67 (2H, m), 0.85-0.92 (2H, m), 1.10 (3H, d), 2.50 (1H, m), 2.91 (1H, m), 3.70 (1H, dd), 3.84 (1 H, dd), 3.92-4.00 (2H, m), 6.46 (1H, brs), 7.03 (1H, d), 7.32 (1H, d), 7.70-7.74 (2H, m), 7.80-7.84 (2H, m) |
| A-7142 | 300MHz, DMSO-d₆ | 0.97-1.02 (4H, m), 1.23-1.27 (2H, m), 1.56-1.60 (2H, m), 4.06 (2H, s), 7.29 (1H, d), 7.57 (1H, d), 9.36 (1H, brs), 10.16 (1H, brs) |

**[Table 1239]**

| Compound Number | Physical property value | |
|---|---|---|
| | Melting point (°C) | Refractive index (n_{D}²⁰) |
| B-0009 | | 1.4759 |

**[Table 1240]**

| Compound Number | Physical property value | |
|---|---|---|
| | Melting point (°C) | Refractive index (n_{D}²⁰) |
| C-0001 | | 1.4715 |
| C-0002 | | 1.4705 |
| C-0004 | 120-123 | |
| C-0005 | 119-122 | |
| C-0006 | 112-115 | |
| C-0010 | 129-132 | |
| C-0011 | 160-162 | |
| C-0035 | 129-130 | |
| C-0066 | 127-128 | |
| C-0068 | | 1.4962 |
| C-0128 | 92-95 | |
| C-0129 | 120-123 | |
| C-0130 | | 1.5023 |
| C-0188 | | 1.4692 |
| C-0190 | | 1.4699 |
| C-0192 | | 1.4691 |
| C-0196 | | 1.4731 |
| C-0197 | | 1.4731 |
| C-0221 | | 1.4619 |
| C-0314 | | 1.5051 |
| C-0315 | | 1.5009 |
| C-0316 | | 1.5039 |
| C-0448 | | 1.3495 |
| C-0590 | | 1.4699 |
| C-0664 | | 1.5002 |
| C-0698 | | 1.4749 |
| C-0806 | 67-70 | |
| C-0880 | 82-85 | |
| C-0988 | 108-111 | |
| C-1023 | 123-125 | |
| C-1349 | | 1.4760 |
| C-1367 | | 1.4744 |
| C-1403 | | 1.3779 |
| C-1439 | | 1.4764 |
| C-1457 | | 1.4732 |
| C-1475 | | 1.4720 |
| C-1511 | | 1.4695 |
| C-2980 | 54-57 | |
| C-3076 | | 1.4963 |
| C-4162 | 119-121 | |
| C-4214 | | 1.4694 |
| C-9400 | 112-114 | |
| C-9422 | 109-110 | |

**[Table 1241]**

| Compound Number | Physical property value | |
|---|---|---|
| | Melting point (°C) | Refractive index (n_{D}²⁰) |
| C-9432 | 118-120 | |
| C-9464 | 51-54 | |
| C-9496 | | 1.5176 |
| C-9528 | 163-165 | |
| C-9688 | | 1.4945 |
| C-9752 | | 1.4948 |
| C-9784 | | 1.4919 |
| C-9944 | 66-69 | |
| C-11248 | | 1.4974 |
| C-11256 | 117-120 | |
| C-11264 | | 1.4880 |
| C-11272 | | 1.4892 |
| C-11408 | 56-59 | |
| C-11416 | 109-111 | |
| C-11512 | | 1.5022 |
| C-11528 | 135-138 | |
| C-11536 | 173-176 | |
| C-11568 | 132-133 | |
| C-11656 | 72-75 | |
| C-11664 | 90-93 | |
| C-11680 | | 1.5005 |
| C-11688 | | 1.5033 |
| C-11704 | | 1.4877 |
| C-11712 | | 1.5010 |
| C-11720 | 106-107 | |
| C-11728 | 143-146 | |
| C-11752 | 82-85 | |
| C-11776 | 110-113 | |
| C-11784 | | 1.5327 |
| C-11792 | | 1.5266 |
| C-11800 | 180-183 | |
| C-11808 | 96-99 | |
| C-11816 | 164-166 | |
| C-11824 | 141-142 | |
| C-11840 | 90-93 | |
| C-13648 | | 1.4891 |
| C-3550 | | 1.5011 |
| C-8914 | | 1.4874 |
| C-8950 | | 1.4889 |
| C-9334 | | 1.5080 |
| C-9338 | 59-62 | |
| C-9848 | | 1.4952 |
| C-11392 | 109-112 | |
| C-11584 | 57-60 | |

**[Table 1242]**

| Compound Number | Physical property value [¹H-NMR data TMS δ (ppm)] | |
|---|---|---|
| C-0191 | 300MHz, CDCl₃ | 1.40 (3H, d), 3.05 (3H, s), 4.05-4.21 (4H, m), 4.40-4.51 (1H, m), 6.81 (1H, brs), 7.20 (1H, d), 7.43 (1H, d) |
| C-0374 | 300MHz, CDCl₃ | 1.27 (3H, t), 1.45 (3H, d), 3.38-3.63 (2H, m), 3.98-4.24 (4H, m), 4.28-4.44 (1H, m), 6.80 (1H, brs), 7.20 (1H, d), 7.43 (1H, d) |
| C-1022 | 400MHz, CDCl₃ | 0.95 (3H, s), 1.58-1.76 (2H, m), 2.48 (3H, s), 2.83-3.04 (3H, m), 3.09 (3H, s), 3.46-3.72 (2H, m), 4.68 (1H, brs), 7.42 (1H, d), 7.48 (1H, d) |
| C-9560 | 300MHz, CDCl₃ | 0.63-0.68 (2H, m), 0.86-0.93 (2H, m), 1.44 (3H, d), 2.10 (3H, s), 2.91 (1H, m), 4.05 (1H, dd), 4.28-4.42 (4H, m), 6.42 (1H, brs), 7.12 (1H, d), 7.25 (1H, d) |
| C-9592 | 300MHz, CDCl₃ | 0.63-0.68 (2H, m), 0.87-0.93 (2H, m), 1.48 (3H, d), 2.91 (1H, m), 3.63 (3H, s), 4.12 (1H, dd), 4.28 (2H, s), 4.32 (1H, m), 4.43 (1H, m), 6.46 (1H, brs), 7.13 (1H, d), 7.32 (1H, d) |
| C-9656 | 300MHz, CDCl₃ | 0.63-0.68 (2H, m), 0.87-0.93 (2H, m), 1.48 (3H, d), 2.05 (3H, s), 2.92 (1H, m), 4.15 (2H, m), 4.45 (1H, m), 5.40 (2H, m), 6.53 (1H, brs), 7.15 (1H, d), 7.40 (1H, d) |
| C-9720 | 300MHz, CDCl₃ | 0.62-0.67 (2H, m), 0.86-0.93 (2H, m), 1.16 (9H, s), 1.48 (3H, d), 2.92 (1H, m), 4.11-4.20 (2H, m), 4.44-4.50 (1H, m), 5.56 (2H, m), 6.52 (1H, brs), 7.14 (1H, d), 7.38 (1H, d) |
| C-10702 | 300MHz, CDCl₃ | 1.50 (9H, s), 1.53 (3H, d), 2.37 (3H, s), 4.19 (1H, m), 4.31-4.49 (3H, m), 4.70 (1H, m), 7.04 (1H, d), 7.19 (1H, d) |
| C-11312 | 300MHz, CDCl₃ | 0.73-0.77 (2H, m), 0.86-0.90 (2H, m), 1.46 (3H, d), 1.49 (3H, s), 4.01-4.14 (3H, m), 5.73 (1H, brs), 6.64 (1H, brs), 7.13 (1H, d), 7.34 (1H, d) |
| C-11400 | 300MHz, CDCl₃ | 1.20-1.26 (2H, m), 1.37-1.42 (2H, m), 1.47 (3H, d), 2.22 (1H, s), 5.54 (1H, brs), 6.94 (1H, brs), 7.15 (1H, d), 7.46 (1H, d) |
| C-11520 | 300MHz, CDCl₃ | 1.46 (3H, d), 2.14-2.33 (2H, m), 2.43-2.53 (2H, m), 2.84-2.93 (2H, m), 4.03- 4.15 (3H, m), 5.60 (1H, brs), 7.12 (1H, brs), 7.17 (1H, d), 7.48 (1H, d) |
| C-11552 | 300MHz, DMSO-d₆ | 1.26 (3H, d), 1.76 (3H, s), 3.80 (1H, dd), 3.88 (1H, m), 3.95-4.05 (2H, m), 4.10-4.15 (2H, m), 4.42 (1H, dd), 4.58 (1H, m), 7.36 (1H, d), 7.54 (1H, d), 9.08 (1H, brs), 9.64 (1H, brs) |

**[Table 1243]**

| Compound Number | Physical property value [¹H-NMR data TMS δ (ppm)] | |
|---|---|---|
| C-11560 | 300MHz, DMSO-d₆ | 1.27 (3H, d), 2.99 (3H, s), 3.19 (1H, ddd), 3.76 (1H, m), 3.85-4.01 (3H, m), 4.09-4.66 (3H, m), 7.32 (1H, d), 7.54 (1H, d), 8.84 (1H, brs), 9.65 (1H, brs) |
| C-11624 | 300MHz, CDCl₃ | 1.46 (3H, d), 2.20 (6H, s), 3.99-4.16 (3H, m), 5.59 (1H, brs), 6.34 (1H, brs), 7.14 (1H, d), 7.43 (1H, d) |
| C-11672 | 300MHz, CDCl₃ | 1.47 (3H, d), 2.11-2.21 (2H, m), 2.71-2.82 (4H, m), 2.96-3.09 (2H, m), 4.03- 4.16 (3H, m), 5.50 (1H, brs), 6.68 (1H, brs), 7.19 (1H, d), 7.49 (1H, d) |
| C-11696 | 300MHz, CDCl₃ | 0.32 (1H, m), 0.41 (1H, m), 0.48-0.62 (2H, m), 0.92 (1H, m), 1.32 (3H, d), 1.45 (3H, d), 3.59 (1H, m), 4.01-4.12 (3H, m), 5.93 (1H, brs), 6.38 (1H, brs), 7.15 (1H, d), 7.35 (1H, d) |
| C-11744 | 300MHz, CDCl₃ | 1.46 (3H, d), 1.82 (6H, s), 4.05-4.16 (3H, m), 5.64 (1H, brs), 6.77 (1H, brs), 7.16 (1H, d), 7.46 (1H, d) |
| C-11760 | 300MHz, CDCl₃ | 1.46 (3H, d), 3.91 (3H, s), 4.04-4.16 (3H, m), 5.85 (1H, brs), 7.17 (1H, d), 7.34 (1H, d), 9.08 (1H, brs) |

**[Table 1244]**

| Compound Number | Physical property value | |
|---|---|---|
| | Melting point (°C) | Refractive index (n_{D}²⁰) |
| D-0006 | 130-132 | |
| D-0125 | | 1.5130 |
| D-0130 | | 1.5077 |
| D-0592 | 69-70 | |
| D-0664 | | 1.5043 |
| D-2977 | 65-68 | |
| D-2980 | 195-198 | |
| D-2992 | 203-206 | |
| D-3076 | | 1.5029 |
| D-4423 | | 1.4248 |
| D-4495 | | 1.4657 |

**[Table 1245]**

| Compound Number | Physical property value | |
|---|---|---|
| | Melting point (°C) | **Refractive** index (n_{D}²⁰) |
| E-0002 | 127-130 | |
| E-0006 | 124-126 | |
| E-0007 | 101-102 | |
| E-0011 | 108-109 | |
| E-0012 | 136-138 | |

**[Table 1246]**

| Compound Number | Physical property value [¹H-NMR data TMS δ (ppm)] | |
|---|---|---|
| E-0010 | 400MHz, DMSO-d₆ | 2.21 (3H, s), 4.04 (2H, q), 6.95 (1H, d), 7.05 (1H, d), 8.86 (1H, brs), 9.88 (1H, brs) |
| E-0030 | 300MHz, DMSO-d₆ | 0.46-0.52 (2H, m), 0.64-0.71 (2H, m), 2.78 (1H, m), 6.93 (1H, d), 7.38 (1H, d), 8.40 (1H, brs), 10.39 (1H, brs) |

**[Table 1247]**

| Compound Number | Physical property value | |
|---|---|---|
| | Melting point (°C) | Refractive index (n_{D}²⁰) |
| F-0423 | 160-161 | |
| F-0510 | 133-136 | |
| F-1 861 | 103-104 | |

**[Table 1248]**

| Compound Number | Physical property value [¹H-NMR data TMS δ (ppm)] | |
|---|---|---|
| F-0165 | 400MHz, CDCl₃ | 1.58-1.64 (2H, m), 1.73-1.90 (4H, m), 2.83 (2H, t), 4.06 (2H, t), 6.81 (1H, t), 7.20 (1H, d), 7.62 (1H, d) |
| F-0171 | 400MHz, CDCl₃ | 1.57-1.65 (2H, m), 1.75-1.90 (4H, m), 2.92 (2H, t), 4.06 (2H, t), 7.20 (1H, d), 7.62 (1H, d) |
| F-0509 | 300MHz, CDCl₃ | 1.50 (3H, d), 4.04-4.20 (3H, m), 5.32 (1H, brs), 7.28 (1H, d), 7.65 (1H, d) |
| F-0511 | 300MHz, CDCl₃ | 1.50 (3H, d), 4.04-4.20 (3H, m), 5.34 (1H, brs), 7.28 (1H, d), 7.64 (1H, d) |
| F-0965 | 400MHz, CDCl₃ | 1.57-1.66 (2H, m), 1.73-1.91 (4H, m), 2.57 (3H, s), 2.92 (2H, t), 4.06 (2H, t), 7.10 (1H, d), 7.67 (1H, d) |
| F-1303 | 400MHz, DMSO-d₆ | 1.26 (3H, d), 2.46 (3H, s), 3.83-3.92 (1H, m), 3.96 (1H, t), 4.10 (1H, dd), 7.21 (1H, d), 7.57 (1H, d), 9.61(1H, brs), 12.93 (1H, brs) |
| F-1626 | 300MHz, DMSO-d₆ | 3.53 (2H, t), 4.10 (2H, t), 7.12 (1H, dd), 7.30 (1H, d), 7.46 (1H, d) |
| F-1644 | 300MHz, DMSO-d₆ | 1.23 (3H, d), 3.82-3.94 (2H, m), 4.04 (1H, m), 7.11 (1H, dd), 7.30 (1H, d), 7.46 (1H, d) |
| F-1645 | 300MHz, DMSO-d₆ | 1.34 (3H, d), 3.44 (1H, dd), 3.62 (1H, d), 4.57 (1H, m), 5.42 (1H, s), 7.03 (1H, d), 7.41 (1H, d), 7.52 (1H, d) |
| F-1773 | 400MHz, CDCl₃ | 1.56-1.63 (2H, m), 1.72-1.83 (4H, m), 2.57 (3H, s), 2.84 (2H, t), 3.99 (2H, t), 6.81 (1H, t), 7.01 (1H, dd), 7.17 (1H, d), 7.67 (1H, d) |
| F-1779 | 400MHz, CDCl₃ | 1.57-1.64 (2H, m), 1.75-1.86 (4H, m), 2.58 (3H, s), 2.92 (2H, t), 3.99 (2H, t), 7.01 (1H, dd), 7.17 (1H, d), 7.58 (1H, d), 11.71 (1H, brs) |
| F-1810 | 400MHz, CDCl₃ | 1.48 (3H, d), 2.58 (3H, s), 3.97 (1H, m), 4.11 (2H, m), 5.23 (1H, brs), 7.02 (1H, dd), 7.21 (1H, d), 7.56 (1H, dd) |

**[Table 1249]**

| Compound Number | Physical property value [¹H-NMR data TMS δ (ppm)] | |
|---|---|---|
| F-1923 | 300MHz, DMSO-d₆ | 1.52 (3H, d), 4.14 (3H, m), 5.77 (1H, brs), 7.38 (1H, s), 7.83 (1H, s), 8.00 (1H, s) |
| F-1926 | 300MHz, DMSO-d₆ | 1.48 (3H, d), 4.00 (1H, m), 4.13 (2H, m), 5.50 (1H, brs), 7.11 (1H, dd), 7.29 (1H, d), 7.56 (1H, d) |
| F-0074 | 300M H z, DMSO-d₆ | 1.00 (4H, s), 4.09 (2H, s), 7.54 (1H, d), 7.51 (1H, d) |
| F-0387 | 300M H z, DMSO-d₆ | 0.84-1.02 (4H, m), 1.38 (3H, d), 2.67 (1H, m), 4.22-4.43 (3H, m), 7.57 (1H, d), 7.63 (1H, d) |

**[Table 1250]**

| Compound Number | Physical property value | |
|---|---|---|
| | Melting point (°C) | **Refractive** index (n_{D}²⁰) |
| G-0430 | | 1.5010 |
| G-1 893 | | 1.4862 |
| G-3809 | | 1.4978 |
| G-4392 | | 1.5055 |
| G-4480 | | 1.5072 |

**[Table 1251]**

| Compound Number | Physical property value [¹H-NMR data TMS δ (ppm)] | |
|---|---|---|
| G-0036 | 300MHz, CDCl₃ | 1.15 (3H, d), 2.39 (1H, m), 3.65 (1H, dd), 3.71 (1H, dd), 3.92 (3H, s), 3.96- 4.05 (2H, m), 7.18 (1H, d), 7.48 (1H, d) |
| G-0104 | 300MHz, CDCl₃ | 2.31 (3H, s), 3.92 (3H, s), 4.64 (2H, s), 7.34 (1H, d), 7.42 (1H, d) |
| G-0378 | 300MHz, DMSO-d₆ | 1.09 (3H, d), 3.18 (2H, brs), 3.23 (1H, m), 3.86 (3H, s), 3.88-3.96 (2H, m), 7.59 (1H, d), 7.63(1H, d) |
| G-0381 | 300MHz, DMSO-d₆ | 1.28 (3H, d), 3.61 (1H, m), 3.87 (3H, s), 4.16 (1H, dd), 4.25 (1H, dd), 7.65 (1H, d), 7.68(1H, d), 8.14 (3H, brs) |
| G-0382 | 300MHz, CDCl₃ | 1.32 (3H, d), 3.52 (1H, m), 3.91 (3H, s), 3.97-4.12 (2H, m), 6.03 (2H, brs), 7.25 (1H, d), 7.55 (1H, d) |
| G-0383 | 300MHz, DMSO-d₆ | 1.29 (3H, d), 3.63 (1H, m), 3.87 (3H, s), 4.17 (1H, dd), 4.27 (1H, dd), 7.66 (1H, d), 7.68(1H, d), 8.25 (3H, brs) |
| G-0393 | 300MHz, CDCl₃ | 0.31-0.41 (2H, m), 0.44-0.56 (2H, m), 1.23 (3H, d), 2.17 (1H, m), 3.26 (1H, m), 3.92 (3H, s), 3.94-4.01 (3H, m), 7.19 (1H, d), 7.50 (1H, d) |
| G-0431 | 300MHz, CDCl₃ | 1.31 (3H, d), 1.45 (9H, s), 3.92 (3H, s), 3.96-4.09 (3H, m), 4.75 (1H, brs), 7.19 (1H, d), 7.50 (1H, d) |
| G-0432 | 300MHz, CDCl₃ | 1.29 (3H, d), 1.43 (9H, s), 3.91 (3H, s), 3.96-4.09 (3H, m), 4.72 (1H, brs), 7.18 (1H, d), 7.49 (1H, d) |
| G-0516 | 300MHz, CDCl₃ | 1.49 (3H, d), 3.93 (3H, s), 4.02-4.18 (3H, m), 5.28 (1H, brs), 7.24 (1H, d), 7.49 (1H, d) |
| G-0517 | 300MHz, CDCl₃ | 1.49 (3H, d), 3.93 (3H, s), 4.02-4.17 (3H, m), 5.49 (1H, brs), 7.23 (1H, d), 7.49 (1H, d) |
| G-0518 | 300MHz, CDCl₃ | 1.49 (3H, d), 3.94 (3H, s), 4.02-4.18 (3H, m), 5.24 (1H, brs), 7.24 (1H, d), 7.49 (1H, d) |

**[Table 1252]**

| Compound Number | Physical property value [¹H-NMR data TMS δ (ppm)] | |
|---|---|---|
| G-1641 | 300M H z, DMSO-d₆ | 3.20 (2H, t), 3.86 (3H, s), 4.23 (2H, t), 7.21 (1H, dd), 7.38 (1H, d), 7.52 (1H, d), 8.20 (3H, brs) |
| G-1652 | 300MHz, DMSO-d₆ | 1.28 (3H, d), 3.60 (1H, m), 3.87 (3H, s), 4.03 (1H, dd), 4.19 (1H, dd), 7.22 (1H, dd), 7.40 (1H, d), 7.53 (1H, d), 8.23 (1H, brs) |
| G-1656 | 300MHz, DMSO-d₆ | 1.27 (3H,d), 3.01 (1H, dd), 3.12 (1H, dd), 3.86 (3H, s), 4.72 (1H, s), 7.24 (1H, d), 7.40 (1H, d), 7.51 (1H, d), 8.17 (1H, brs) |
| G-1668 | 300MHz, CDCl₃ | 1.45 (9H, s), 3.54 (2H, m), 3.93 (3H, s), 4.21 (2H, t), 4.93 (1H brs), 6.96 (1H, dd), 7.32 (1H, d), 7.35(1H, d) |
| G-1674 | 300MHz, CDCl₃ | 1.29 (3H, d), 1.45 (9H, s), 3.93 (3H, s), 3.94-4.11 (3H, m), 4.71 (1H, brs), 6.98 (1H, dd), 7.34 (1H, d), 7.35 (1H, d) |
| G-1677 | 300MHz, CDCl₃ | 1.27 (3H, d), 1.44 (9H, s) 3.24 (1H, m), 3.46 (1H, m), 3.927 (3H, s), 4.49 (1H, m), 4.89 (1H, brs), 6.97 (1H, dd), 7.34 (d, 1H), 7.35 (1H, d,) |
| G-1703 | 300MHz, CDCl₃ | 3.70 (2H, t), 3.93 (1H, s), 4.12 (2H, t), 5.40 (1H, s), 6.96 (1H, dd), 7.33 (1H, d), 7.36 (1H, d) |
| G-1721 | 300MHz, CDCl₃ | 1.46 (3H, d), 3.94 (3H, s), 3.96 (1H, m), 4.05-4.13 (2H, m), 5.24 (1H, brs), 6.97 (1H, dd), 7.34 (1H, d), 7.37 (1H, d) |
| G-1722 | 300MHz, CDCl₃ | 1.32 (3H, d), 3.42 (1H, dd), 3.60 (1H, dd), 4.55 (1H, m), 5.35 (1H, brs), 6.97 (1H, d), 7.35 (1H, d), 7.37 (1H, d) |
| G-1855 | 300MHz, DMSO-d₆ | 1.29 (3H, d), 2.44 (3H, s), 3.58 (1H, m), 3.83 (3H, s), 4.00 (1H, dd), 4.14 (1H, dd), 7.13 (1H, dd), 7.28 (1H, d), 7.41 (1H, d), 8.17 (3H, brs) |
| G-1 877 | 300MHz, CDCl₃ | 1.29 (3H, d), 1.45 (9H, s), 2.52 (3H, s), 3.88 (3H, s), 3.93 (2H, m), 4.04 (1H, m), 4.76 (1H, brs), 6.96 (1H, dd), 7.14 (1H, d), 7.44(1H, d) |
| G-1924 | 300MHz, CDCl₃ | 1.46 (3H, d), 2.53 (3H, s), 3.90 (3H, s), 3.95 (1H, m), 4.07 (1H, m), 5.25 (1H, brs), 6.95 (1H, dd), 7.18 (1H, d), 7.43 (1H, d) |

**[Table 1253]**

| Compound Number | Physical property value [¹H-NMR data TMS δ (ppm)] | |
|---|---|---|
| G-2040 | 300MHz, DMSO-d₆ | 1.30 (3H, d), 3.63, (1H, m), 3.91 (3H, s), 4.15 (1H, dd), 4.34 (1H, dd), 7.63 (1H, m), 7.81 (2H, m), 8.22 (3H, brs) |
| G-2043 | 300MHz, CDCl₃ | 1.31 (3H, d), 1.45 (9H, s), 3.93 (3H, s), 4.01 (2H, d), 4.05 (1H, m), 4.72 (1H, brs), 7.26 (1H, m), 7.73 (1H, m), 7.90 (1H, m) |
| G-2046 | 300MHz, CDCl₃ | 1.49 (3H, d), 3.94 (3H, s), 4.11 (3H, m), 5.18 (1H, brs), 7.34 (1H, m), 7.73 (1H, m), 7.95 (1H, m) |
| G-2052 | 300MHz, DMSO-d₆ | 1.28 (3H, d), 3.62 (1H, m), 3.87 (3H, s), 4.04 (1H, dd), 4.22 (1H, dd), 7.34 (1H, dd), 7.50 (2H. m), 8.10 (3H, brs) |
| G-2055 | 300MHz, CDCl₃ | 1.28 (3H, d), 1.45 (9H, s), 3.93 (3H, s), 3.96 (2H, d), 4.07 (1H, m), 4.67 (1H, brs), 7.07 (1H, dd), 7.23 (1H, d), 7.44 (1H, d) |
| G-2058 | 300MHz, CDCl₃ | 1.47 (3H, d), 3.94 (3H, s), 3.98 (1H, m), 4.11 (2H, m), 5.17 (1H, brs), 7.07 (1H, dd), 7.28 (1H, d), 7.44 (1H, d) |
| G-3697 | 300MHz, CDCl₃ | 1.19 (3H, s), 1.39 (3H, t), 2.04 (2H, brs), 2.52 (3H, s), 3.40 (1H, m), 3.77 (1H, dd), 3.96 (1H, dd), 4.35 (2H, q), 6.94 (1H, d), 7.55 (1H, d) |
| G-3749 | 300MHz, CDCl₃ | 1.31 (3H, d), 1.39 (3H, t), 1.45 (9H, s), 2.52 (3H, s), 3.91-4.21 (3H, m), 4.35 (2H, q), 4.81 (1H, brs), 6.94 (1H, d), 7.56 (1H, d) |
| G-3835 | 300MHz, CDCl₃ | 1.42 (3H, t), 1.47 (3H, d), 2.53 (3H, s), 4.02-4.15 (3H, m), 4.35 (2H, q), 5.55 (1H, brs), 6.96 (1H, d), 7.54 (1H, d) |
| G-0074 | 300MHz, DMSO-d₆ | 0.93-0.98 (2H, m), 1.09-1.15 (2H, m), 3.86 (3H, s), 4.25 (2H, s), 7.63 (1H, s), 7.67 (1H, s), 8.69 (3H, brs) |
| G-0075 | 300MHz, CDCl₃ | 0.88-0.99 (4H, m), 1.43 (9H, s), 3.92 (3H, s), 4.05 (2H, s), 5.18 (1H, brs), 7.19 (1H, d), 7.47 (1H, d) |
| G-0076 | 300MHz, CDCl₃ | 1.01-1.05 (2H, m), 1.26-1.30 (2H, m), 3.93 (3H, s), 4.05 (2H, s), 5.77 (1H, brs), 7.23 (1H, d), 7.46 (1H, d) |
| G-0106 | 300MHz, CDCl₃ | 2.98 (1H, brs), 3.94 (3H, s), 4.24 (1H, dd), 4.33 (1H, dd), 4.43 (1H, m), 7.23 (1H, d), 7.54 (1H, d) |
| G-0107 | 300MHz, CDCl₃ | 3.77 (2H, brs), 3.94 (3H, s), 4.27 (2H, s), 7.25 (1H, d), 7.59 (1H, d) |
| G-0108 | 300MHz, CDCl₃ | 3.94 (3H, s), 5.05 (2H, s), 7.23 (1H, d), 7.61 (1H, d), 8.79 (1H, brs) |
| G-0394 | 300MHz, CDCl₃ | 0.84-0.94 (2H, m), 1.01 (1H, m), 1.15 (1H, m), 1.50 (3H, d), 2.63 (1H, m), 3.94 (3H, s), 4.07 (1H, dd), 4.28 (1H, dd), 4.48 (1H, m), 7.22 (1H, d), 7.48 (1H, d) |
| G-0770 | 300MHz, CDCl₃ | 1.12 (3H, d), 2.57 (1H, m), 3.72 (1H, dd), 3.84 (1H, dd), 3.91 (3H, s), 3.94-4.01 (2H, m), 7.11 (1H, d), 7.43 (1H, d), 7.70-7.73 (2H, m), 7.82-7.85 (2H, m) |

Next, formulation examples of the pest control agent of the present invention using the 3-alkoxybenzamide derivative or agriculturally acceptable salt thereof according to the present invention produced as described above will be specifically described. However, the type and blending ratio of the compound and the additive are not limited thereto, and can be changed in a wide range. In the following description, "part" means part by mass.

### [Formulation Example 1] Emulsifiable concentrate

Each compound listed in Tables 1 to 1062 and Tables 1234 to 1244: 10 parts
Cyclohexanone: 30 parts
Polyoxyethylene alkyl aryl ether: 11 parts
Calcium alkylbenzenesulfonate: 4 parts
Methylnaphthalene: 45 parts

The above components were uniformly dissolved to obtain an emulsifiable concentrate.

### [Formulation Example 2] Wettable powder

Each compound listed in Tables 1 to 1062 and Tables 1234 to 1244: 10 parts
Naphthalenesulfonic acid formalin condensate sodium salt: 0.5 parts
Polyoxyethylene alkyl aryl ether: 0.5 parts
Diatomaceous earth: 24 parts
Clay: 65 parts

The above components were uniformly mixed and pulverized to obtain a wettable powder.

### [Formulation Example 3] Powder

Each compound listed in Tables 1 to 1062 and Tables 1234 to 1244: 2 parts
Diatomaceous earth: 5 parts
Clay: 93 parts

The above components were uniformly mixed and pulverized to obtain a powder.

### [Formulation Example 4] Granule

Each compound listed in Tables 1 to 1062 and Tables 1234 to 1244: 5 parts
Sodium salt of lauryl alcohol sulfate: 2 parts
Sodium lignin sulfonate: 5 parts
Carboxymethyl cellulose: 2 parts
Clay: 86 parts

The above components were uniformly mixed and pulverized. An amount corresponding to 20 parts of water was added to the mixture. The mixture was kneaded and processed into a granular shape of 14 to 32 mesh using an extrusion granulator, followed by drying to obtain a granule.

### [Formulation Example 5] Flowable agent

Each compound listed in Tables 1 to 1062 and Tables 1234 to 1244: 20 parts
Polyoxyethylene styrenated phenyl ether sulfate: 4 parts
Ethylene glycol: 7 parts
Silicone AF-118N (manufactured by Asahi Kasei Corporation): 0.02 parts
Water: 68.98 parts

The above components were mixed for 30 minutes with a high-speed stirrer, and pulverized with a wet pulverizer to obtain a flowable agent.

### [Formulation Example 6] Water-dispersible granule

Each compound listed in Tables 1 to 1062 and Tables 1234 to 1244: 10 parts
Sodium lignin sulfonate: 5 parts
Polyoxyethylene alkyl aryl ether: 1 part
Sodium polycarboxylate: 3 parts
White carbon: 5 parts
Pregelatinized starch: 1 part
Calcium carbonate: 65 parts
Water: 10 parts

The above components were mixed, kneaded, and pressed to obtain a granule. The obtained granule was dried in a fluidized bed drier to obtain a water-dispersible granule.

Next, effects of the pest control agent of the present invention will be described with reference to test examples.

### [Test Example 1] Test for insecticidal activity against Plutella xylostella

The wettable powder prepared in accordance with Formulation Example 2 was diluted with water to a concentration of 500 ppm as an active ingredient. Cabbage leaves were immersed in the chemical solution, air-dried, and then placed in a plastic cup. Ten second instar larvae of *Plutella xylostella* were released into the cup, and the cup was capped. Thereafter, the cup was placed in a room at constant temperature of 25°C. The number of dead insects was examined at 6 days after the treatment, and the mortality rate was caluculated by the calculation formula of Equation 1. The number of replicate is one in each test.$Mortality rate \left(%\right) = \left(1-\frac{The number of surviving insects}{The number of insects provided for the test}\right) ∗ 100$

The compound numbers of the compounds showing a mortality rate of 90% or more in this test are listed below.
A-0052, A-0056, A-0059, A-0060, A-0061, A-0063, A-0243, A-0405, A-0447, A-0475, A-0480, A-0491, A-0598, A-1310, A-3829, A-4710, A-4715, A-4969, A-6572, A-6692, A-6724, A-6754, A-6889, B-0009, C-0001, C-0002, C-0004, C-0006, C-0010, C-0011, C-0068, C-0128, C-0130, C-0188, C-0190, C-0192, C-0196, C-0197, C-0314, C-0316, C-0374, C-0448, C-0590, C-0664, C-0698, C-0806, C-0880, C-0988, C-1023, C-11248, C-11256, C-11264, C-11272, C-11312, C-11392, C-11400, C-11512, C-11520, C-11528, C-11536, C-11568, C-11624, C-11664, C-11680, C-11696, C-11712, C-11720, C-11744, C-11752, C-11784, C-11792, C-11800, C-1349, C-1367, C-1403, C-1439, C-1457, C-1475, C-1511, C-2980, C-3076, C-3550, C-4162, C-4214, C-8914, C-8950, C-9334, C-9338, C-9400, C-9422, C-9432, C-9464, C-9496, C-9528, C-9560, C-9592, C-9656, C-9688, C-9720, C-9752, C-9784, C-9848, C-9944, D-0006, D-0125, D-0130, D-0592, D-0664, D-2980, D-3076, D-4495

### [Test Example 2] Test for insecticidal activity against Helicoverpa armigera

The wettable powder prepared in accordance with Formulation Example 2 was diluted with water to a concentration of 500 ppm as an active ingredient. Cabbage leaves were immersed in the chemical solution, air-dried, and then placed in a plastic cup. Five hatching larvae of *Helicoverpa armigera* were released into the cup, and the cup was capped. Thereafter, the cup was placed in a room at constant temperature of 25°C. The number of dead insects was examined at 6 days after the treatment, and the mortality rate was caluculated by the calculation formula of Equation 1. The number of replicate is two in each test.

The compound numbers of the compounds showing a mortality rate of 90% or more in this test are listed below.
A-0059, A-0063, A-0243, A-0405, A-0491, A-1913, A-3459, A-3506, A-3829, A-4969, B-0009, C-0002, C-0004, C-0006, C-0068, C-0128, C-0130, C-0190, C-0314, C-0316, C-0374, C-0448, C-0590, C-0664, C-0698, C-0880, C-0988, C-1349, C-1403, C-1439, C-1457, C-1475, C-1511, C-4162, C-4214, C-9400, C-9422, C-9432, C-9464, C-9496, C-9656, C-9688, C-9720, C-9752, C-9784, C-9944, C-11256, C-11264, C-11272, C-11312, C-11400, C-11512, C-11520, C-11528, C-11568, C-11624, C-11664, C-11680, C-11688, C-11696, C-11712, C-11720, C-11752, C-11784, C-11792, D-0006, D-0125, D-0130, D-0592, D-0664, D-2992, D-3076, D-4495

### [Test Example 3] Test for insecticidal activity against Nilaparvata lugens

The wettable powder prepared in accordance with Formulation Example 2 was diluted with water to a concentration of 500 ppm as an active ingredient. The rice sprouts were immersed in the chemical solution, and placed in a plastic cup. Ten second instar larvae of *Nilaparvata lugens* were released into the plastic cup, and the cup was capped. Thereafter, the cup was placed in a room at constant temperature of 25°C. The number of dead insects was examined at 6 days after the treatment, and the mortality rate was caluculated by the calculation formula of Equation 1. The number of replicate is one in each test.

The compound numbers of the compounds showing a mortality rate of 90% or more in this test are listed below.
A-0015, A-0049, A-0052, A-0056, A-0059, A-0060, A-0063, A-0064, A-0179, A-0370, A-0447, A-0474, A-0479, A-0752, A-0809, A-1483, A-1529, A-4688, A-4690, A-4710, A-4850, A-4969, A-6161, A-6482, A-6572, A-6602, A-6632, A-6662, A-6708, A-6709, A-6754, A-6814, A-6889, A-7024, B-0009, C-0001, C-0004, C-0006, C-0035, C-0066, C-0068, C-0128, C-0129, C-0130, C-0190, C-0191, C-0192, C-0314, C-0315, C-0316, C-0374, C-0448, C-0590, C-0664, C-0698, C-0806, C-0880, C-0988, C-11248, C-11256, C-11264, C-11272, C-11312, C-11392, C-11400, C-11408, C-11512, C-11520, C-11528, C-11536, C-11552, C-11584, C-11624, C-11656, C-11664, C-11680, C-11688, C-11696, C-11704, C-11712, C-11720, C-11728, C-11744, C-11752, C-11760, C-11776, C-11792, C-11808, C-11816, C-11824, C-11840, C-1349, C-13648, C-1367, C-1403, C-1439, C-1457, C-1475, C-1511, C-2980, C-3076, C-3550, C-4162, C-4214, C-8914, C-8950, C-9334, C-9338, C-9400, C-9422, C-9432, C-9464, C-9496, C-9528, C-9560, C-9592, C-9656, C-9688, C-9720, C-9752, C-9784, C-9848, C-9944, D-0006, D-0125, D-0130, D-0592, D-0664, D-2980, D-2992, D-3076, D-4495, G-3809, G-4480

### [Test Example 4] Test for control effect against Aphis gossypii

The wettable powder prepared in accordance with Formulation Example 2 was diluted with water to a concentration of 500 ppm as an active ingredient. A cucumber seedling previously inoculated with *Aphis gossypii* nymphs was immersed in the chemical solution, and air-dried. The treated cucumber seedling was placed in a room at constant temperature of 25°C. The number of surviving insects was counted at 3 days after the treatment, and the mortality rate was caluculated by the calculation formula of Equation 1. The number of replicate is one in each test.

The compound numbers of the compounds showing a mortality rate of 90% or more in this test are listed below.
A-0052, A-0059, A-0060, A-0063, A-0064, A-0179, A-0405, A-0447, A-0474, A-3829, A-6572, A-6934, B-0009, C-0001, C-0004, C-0006, C-0066, C-0068, C-0128, C-0129, C-0130, C-0190, C-0191, C-0192, C-0314, C-0315, C-0316, C-0374, C-0448, C-0590, C-0664, C-0698, C-0806, C-0880, C-0988, C-1023, C-11248, C-11256, C-11264, C-11272, C-11312, C-11392, C-11400, C-11408, C-11512, C-11520, C-11528, C-11536, C-11568, C-11584, C-11624, C-11656, C-11664, C-11680, C-11688, C-11696, C-11704, C-11712, C-11720, C-11728, C-11744, C-11752, C-11760, C-11776, C-11784, C-11808, C-11816, C-11824, C-11840, C-1349, C-13648, C-1367, C-1403, C-1439, C-1457, C-1475, C-1511, C-2980, C-3076, C-3550, C-4162, C-4214, C-8914, C-9334, C-9338, C-9400, C-9422, C-9432, C-9464, C-9496, C-9528, C-9560, C-9592, C-9656, C-9688, C-9720, C-9752, C-9784, C-9848, C-9944, D-0006, D-0130, D-0592, D-0664, D-2980, D-2992, D-3076, D-4423, D-4495

### [Test Example 5] Test for control effect against Tetranychus urticae

The wettable powder prepared in accordance with Formulation Example 2 was diluted with water to a concentration of 500 ppm as an active ingredient. A soybean seedling previously inoculated with 35 female adults of *Tetranychus urticae* was immersed in the chemical solution, and air-dried. The treated soybean seedling was placed in a room at constant temperature of 25°C The number of surviving female adults was examined at 13 days after the treatment, and the controlling value was determined by the calculation formula of Equation 2. The number of replicate is one in each test.$\begin{matrix}Controlling value \\ = \left(1-\frac{The number of surviving female adults on treated seedling}{The number of surviving female adults on untreated seedling}\right) \times 100\end{matrix}$

The compound numbers of the compounds showing a controlling value of 90 or more in this test are listed below.
A-0008, A-0015, A-0030, A-0037, A-0049, A-0052, A-0056, A-0059, A-0060, A-0063, A-0064, A-0170, A-0179, A-0243, A-0273, A-0405, A-0447, A-0474, A-0475, A-0479, A-0480, A-0491, A-0507, A-0538, A-0598, A-0753, A-0758, A-0942, A-1310, A-1477, A-1483, A-1529, A-1960, A-3828, A-3829, A-4665, A-4687, A-4688, A-4689, A-4691, A-4693, A-4710, A-4712, A-4715, A-4735, A-4758, A-4850, A-4969, A-5011, A-5172, A-5977, A-6161, A-6482, A-6497, A-6572, A-6662, A-6692, A-6708, A-6754, A-6814, A-6844, A-6859, A-6889, A-6934, B-0009, C-0001, C-0002, C-0004, C-0005, C-0006, C-0035, C-0066, C-0068, C-0128, C-0129, C-0130, C-0188, C-0190, C-0191, C-0192, C-0196, C-0221, C-0314, C-0315, C-0316, C-0374, C-0448, C-0590, C-0664, C-0698, C-0806, C-0880, C-0988, C-1022, C-1349, C-1367, C-1403, C-1439, C-1457, C-1475, C-1511, C-2980, C-3076, C-4162, C-4214, C-9400, C-9422, C-9432, C-9464, C-9496, C-9656, C-9688, C-9720, C-9752, C-9784, C-9944, C-11248, C-11256, C-11264, C-11272, C-11312, C-11400, C-11512, C-11520, C-11528, C-11568, C-11624, C-11656, C-11664, C-11680, C-11688, C-11696, C-11704, C-11712, C-11720, C-11728, C-11744, C-11752, C-11760, C-11776, C-11784, C-11792, C-11816, C-11824, D-0006, D-0125, D-0130, D-0592, D-0664, D-2977, D-2980, D-2992, D-3076, D-4495

### Industrial Applicability

The present invention provides a novel compound having excellent insecticidal activity and a production intermediate thereof, is useful in the field of pesticides and the field of agriculture, and has industrial applicability.

## Claims

1. A 3-alkoxybenzamide derivative of formula [I], or an agriculturally acceptable salt thereof, 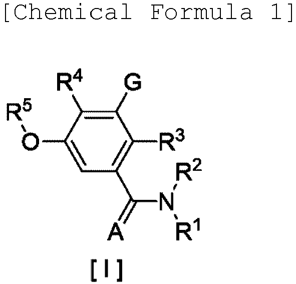 wherein in the formula [I],
A represents an oxygen atom; or a sulfur atom,
G represents a hydrogen atom,
R¹ represents a (C1-C6)alkyl; a (C1-C6)haloalkyl; a (C3-C6)cycloalkyl; a (C3-C6)halocycloalkyl; a (C2-C6)alkenyl; a (C2-C6)alkynyl; a (C1-C6)alkoxy; a (C1-C6)haloalkoxy; a (C1-C6)alkoxy (C1-C6)alkyl; a (C3-C6)cycloalkyl (C1-C6)alkyl; a cyano (C1-C6)alkyl; a cyano (C3-C6)cycloalkyl; a cyano (C3-C6)cycloalkyl (C1-C6)alkyl; a cyano heterocycloalkyl; a (C2-C6)alkynyl (C3-C6)cycloalkyl; a (C1-C6)alkoxycarbonyl (C3-C6)cycloalkyl; a carboxy (C3-C6)cycloalkyl; a carbamoyl (C3-C6)cycloalkyl; a (C4-C8)bicycloalkyl; a phenyl (the phenyl is optionally mono-substituted or poly-substituted with R⁷); a phenyl (C1-C6)alkyl (the phenyl moiety is optionally mono-substituted or poly-substituted with R⁷); a heteroaryl (C1-C3)alkyl (the heteroaryl moiety is optionally mono-substituted or poly-substituted with R⁷); or a heterocycloalkyl (the heterocycloalkyl is optionally substituted with R¹⁰),
R² represents a hydrogen atom; a (C1-C6)alkyl; a (C1-C6)haloalkyl; a (C2-C6)alkynyl; a cyano (C1-C6)alkyl; a hydroxy (C1-C6)alkyl; a (C1-C6)alkoxy (C1-C6)alkyl; a (C1-C6)alkylcarbonyl; a (C1-C6)alkoxycarbonyl; a (C1-C6)haloalkoxycarbonyl; a (C1-C6)alkoxycarbonyl (C1-C6)alkyl; an aminocarbonyl (C1-C6)alkyl; a (C1-C6)alkylaminocarbonyl (C1-C6)alkyl (the amino moiety is optionally substituted with R¹⁰); a (C1-C6)alkylcarbonyloxy (C1-C6)alkyl; or a (C1-C6)alkoxycarbonyloxy (C1-C6)alkyl, or
R¹ and R², together with the nitrogen atom to which R¹ and R² are attached form a 3- to 8-membered heterocyclic ring or a 3- to 8-membered heterocyclic ring having 1 to 4 heteroatoms selected from an oxygen atom, a sulfur atom, and a nitrogen atom, wherein the formed heterocyclic ring is optionally substituted with a halogen atom, a cyano, a nitro, a (C1-C6)alkyl, a (C1-C6)alkoxy, a (C1-C6)haloalkyl, or an oxo,
R³ represents a halogen atom; a (C1-C6)alkyl; a (C1-C6)haloalkyl; or a (C1-C6)haloalkoxy,
R⁴ represents a hydrogen atom; a halogen atom; or a (Cl-C6)alkyl,
R⁵ represents an ethyl, an n-propyl, an isopropyl, an n-butyl, a sec-butyl, an isobutyl, a tert-butyl, an n-pentyl, a 1-methylbutyl, a 2-methylbutyl, a 3-methylbutyl, a 1-ethylpropyl, a 1,1-dimethylpropyl, a 1,2-dimethylpropyl, a neopentyl, an n-hexyl, a 1-methylpentyl, a 2-methylpentyl, a 3-methylpentyl, a 4-methylpentyl, a 1-ethylbutyl, a 2-ethylbutyl, a 1,1-dimethylbutyl, a 1,2-dimethylbutyl, a 1,3-dimethylbutyl, a 2,2-dimethylbutyl, a 2,3-dimethylbutyl, a 3,3-dimethylbutyl, a 1,1,2-trimethylpropyl, a 1,2,2-trimethylpropyl, a 1-ethyl-1-methylpropyl, a 1-ethyl-2-methylpropyl, an n-heptyl, an n-octyl, an n-nonyl, an n-decyl, an n-undecyl, an n-dodecyl, a 4,4-dimethylpentyl, a 5-methylhexyl, a 5,5-dimethylhexyl, a 3,5,5-trimethylhexyl, a 6-methylheptyl, a 6,6-dimethylheptyl, a 3,6,6-trimethylheptyl, a 7-methyloctyl, a 7,7-dimethyloctyl, an 8-methylnonyl, an 8,8-dimethylnonyl, a 9-methyldecyl, a 9,9-dimethyldecyl, or a 10-methylundecyl (the ethyl, the n-propyl, the isopropyl, the n-butyl, the sec-butyl, the isobutyl, the tert-butyl, the n-pentyl, the 1-methylbutyl, the 2-methylbutyl, the 3-methylbutyl, the 1-ethylpropyl, the 1,1-dimethylpropyl, the 1,2-dimethylpropyl, the neopentyl, the n-hexyl, the 1-methylpentyl, the 2-methylpentyl, the 3-methylpentyl, the 4-methylpentyl, the 1-ethylbutyl, the 2-ethylbutyl, the 1,1-dimethylbutyl, the 1,2-dimethylbutyl, the 1,3-dimethylbutyl, the 2,2-dimethylbutyl, the 2,3-dimethylbutyl, the 3,3-dimethylbutyl, the 1,1,2-trimethylpropyl, the 1,2,2-trimethylpropyl, the 1-ethyl-1-methylpropyl, the 1-ethyl-2-methylpropyl, the n-heptyl, the n-octyl, the n-nonyl, the n-decyl, the n-undecyl, the n-dodecyl, the 4,4-dimethylpentyl, the 5-methylhexyl, the 5,5-dimethylhexyl, the 3,5,5-trimethylhexyl, the 6-methylheptyl, the 6,6-dimethylheptyl, the 3,6,6-trimethylheptyl, the 7-methyloctyl, the 7,7-dimethyloctyl, the 8-methylnonyl, the 8,8-dimethylnonyl, the 9-methyldecyl, the 9,9-dimethyldecyl, or the 10-methylundecyl is optionally mono-substituted or poly-substituted with R⁶); or a (C2-C6)alkenyl (the (C2-C6)alkenyl is optionally mono-substituted or poly-substituted with R⁶),
R⁶ represents a halogen atom; a (C1-C6)alkyl; a (C1-C6)haloalkyl; a (C3-C6)cycloalkyl; a hydroxy; a thiol; a (C1-C6)alkylthio; a (C1-C6)haloalkylthio; a (C1-C6)alkylsulfinyl; a (C1-C6)haloalkylsulfinyl; a (C1-C6)alkylsulfonyl; a (C1-C6)haloalkylsulfonyl; a (C1-C6)alkylcarbonyl; an amino; a (C1-C6)alkylcarbonylamino (the amino moiety is optionally substituted with R¹⁰); a (C1-C6)haloalkylcarbonylamino (the amino moiety is optionally substituted with R¹⁰); a (C1-C6)alkoxycarbonylamino (the amino moiety is optionally substituted with R¹⁰); a (C1-C6)haloalkoxycarbonylamino (the amino moiety is optionally substituted with R¹⁰); a (C1-C6)haloalkylsulfinylamino (the amino moiety is optionally substituted with R¹⁰); a (C1-C6)haloalkylsulfonylamino (the amino moiety is optionally substituted with R¹⁰); a phenylsulfonylamino (the phenyl moiety is optionally mono-substituted or poly-substituted with R⁷, and the amino moiety is optionally substituted with R¹⁰); a phenyl (the phenyl is optionally mono-substituted or poly-substituted with R⁷); an azetidinyl (a nitrogen atom of the azetidinyl is optionally substituted with R¹⁰); a 1,3-dioxolanyl; a pyrazolyl (the pyrazolyl is optionally mono-substituted or poly-substituted with R⁷); a triazolyl (the triazolyl is optionally mono-substituted or poly-substituted with R⁷); a 1,3-dioxoisoindolinyl (the 1,3-dioxoisoindolinyl is optionally mono-substituted or poly-substituted with R⁷); or a thiocyanato,
R⁷ represents a halogen atom; a (C1-C6)alkyl; a (C1-C6)haloalkyl; or a cyano, and
R¹⁰ represents a (C1-C6)alkyl; a (C1-C6)haloalkyl; a (C3-C6)cycloalkyl; a (C1-C6)alkoxy (C1-C6)alkyl; a (C1-C6)alkoxy (C1-C6)alkoxy (C1-C6)alkyl; a (C1-C6)alkylthio (C1-C6)alkyl; a (C1-C6)alkylsulfonyl (C1-C6)alkyl; a cyano (C1-C6)alkyl; a (C1-C6)alkylcarbonyl; a (C1-C6)alkoxycarbonyl; a (C1-C6)haloalkylsulfonyl; a (C1-C6)alkylcarbonyl (C1-C6)alkyl; a (C1-C6)alkoxycarbonyl (C1-C6)alkyl; a (C1-C6)alkylcarbonyloxy (C1-C6)alkyl; a (C1-C6)alkoxycarbonyloxy (C1-C6)alkyl; or a 5-methyl-1,3-dioxole-2-one-4-ylmethyl.

2. An agrochemical composition comprising the 3-alkoxybenzamide derivative or the agriculturally acceptable salt thereof according to claim 1 as an active ingredient.

3. The agrochemical composition according to claim 2, which further comprises a surfactant.

4. A pest control agent comprising the 3-alkoxybenzamide derivative or the agriculturally acceptable salt thereof according to claim 1 as an active ingredient.

5. The pest control agent according to claim 4, wherein the pest control agent is an insecticide, a nematicide, and an acaricide.

6. The pest control agent according to claim 5, wherein the pest control agent is capable of controlling pests in a paddy field, a dry field, a lawn, an orchard, a non-crop land, a greenhouse, a raising seeding facility, and a plant factory where an agricultural or horticultural plant is cultivated.

7. The pest control agent according to claim 6, wherein the agricultural or horticultural plant is a plant to which resistance is imparted by a breeding or genetic modification techniques.

8. A process for controlling a pest, comprising a step of applying an effective amount of the 3-alkoxybenzamide derivative or the agriculturally acceptable salt thereof according to claim 1.

9. A process for controlling a pest, comprising a step of applying an agrochemical composition comprising the 3-alkoxybenzamide derivative or the agriculturally acceptable salt thereof according to claim 1 as an active ingredient to an agricultural or horticultural crop or a place where the agricultural or horticultural crop is to be grown or being grown, in a single or divided dose.

10. The process for controlling a pest according to claim 8 or 9, wherein a place where a pest control agent is applied is a paddy field, a dry field, a lawn, an orchard, a non-crop land, a greenhouse, a raising seeding facility, and a plant factory.

11. The process for controlling a pest according to any one of claims 8 to 10, wherein the 3-alkoxybenzamide derivative or the agriculturally acceptable salt thereof according to claim 1 is used as an insecticide, a nematicide, and an acaricide.

12. A process for using a pest control agent, **characterized in that**, in the process, the pest control agent according to any one of claims 4 to 7 is applied for controlling a pest harmful to an agricultural or horticultural crop.

13. A hydroxybenzamide derivative of formula [II], or a salt thereof, 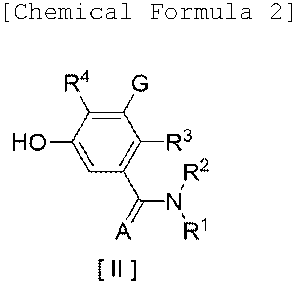 wherein in the formula [II],
A represents an oxygen atom or a sulfur atom,
G represents a hydrogen atom,
R¹ represents a (C1-C6)alkyl; a (C1-C6)haloalkyl; a (C3-C6)cycloalkyl; a (C3-C6)halocycloalkyl; a (C2-C6)alkenyl; a (C2-C6)alkynyl; a (C1-C6)alkoxy; a (C1-C6)haloalkoxy; a (C1-C6)alkoxy (C1-C6)alkyl; a (C3-C6)cycloalkyl (C1-C6)alkyl; a cyano (C1-C6)alkyl; a cyano (C3-C6)cycloalkyl; a cyano (C3-C6)cycloalkyl (C1-C6)alkyl; a cyano heterocycloalkyl a (C2-C6)alkynyl (C3-C6)cycloalkyl; a (C1-C6)alkoxycarbonyl (C3-C6)cycloalkyl; a carboxy (C3-C6)cycloalkyl; a carbamoyl (C3-C6)cycloalkyl; a (C4-C8)bicycloalkyl; a phenyl (the phenyl is optionally mono-substituted or poly-substituted with R⁷); a phenyl (C1-C6)alkyl (the phenyl moiety is optionally mono-substituted or poly-substituted with R⁷); a heteroaryl (C1-C3)alkyl (the heteroaryl moiety is optionally mono-substituted or poly-substituted with R⁷); or a heterocycloalkyl (the heterocycloalkyl is optionally substituted with R¹⁰),
R² represents a hydrogen atom; a (C1-C6)alkyl; a (C1-C6)haloalkyl; a (C2-C6)alkynyl; a cyano (C1-C6)alkyl; a hydroxy (C1-C6)alkyl; a (C1-C6)alkoxy (C1-C6)alkyl; a (C1-C6)alkylcarbonyl; a (C1-C6)alkoxycarbonyl; a (C1-C6)haloalkoxycarbonyl; a (C1-C6)alkoxycarbonyl (C1-C6)alkyl; an aminocarbonyl (C1-C6)alkyl; a (C1-C6)alkylaminocarbonyl (C1-C6)alkyl (the amino moiety is optionally substituted with R¹⁰); a (C1-C6)alkylcarbonyloxy (C1-C6)alkyl; or a (C1-C6)alkoxycarbonyloxy (C1-C6)alkyl, or
R¹ and R², together with the nitrogen atom to which R¹ and R² are attached form a 3- to 8-membered heterocyclic ring or a 3- to 8-membered heterocyclic ring having 1 to 4 heteroatoms selected from an oxygen atom, a sulfur atom, and a nitrogen atom, and the heterocyclic ring is optionally substituted with a halogen atom, a cyano, a nitro, a (C1-C6)alkyl, a (C1-C6)alkoxy, a (C1-C6)haloalkyl, or an oxo,
R³ represents a halogen atom; a (C1-C6)alkyl; a (C1-C6)haloalkyl; or a (C1-C6)haloalkoxy,
R⁴ represents a hydrogen atom; a halogen atom; or a (C1-C6)alkyl,
R⁷ represents a halogen atom; a (C1-C6)alkyl; a (C1-C6)haloalkyl; or a cyano, and
R¹⁰ represents a (C1-C6)alkyl; a (C1-C6)haloalkyl; a (C3-C6)cycloalkyl; a (C1-C6)alkoxy (C1-C6)alkyl; a (C1-C6)alkoxy (C1-C6)alkoxy (C1-C6)alkyl; a (C1-C6)alkylthio (C1-C6)alkyl; a (C1-C6)alkylsulfonyl (C1-C6)alkyl; a cyano (C1-C6)alkyl; a (C1-C6)alkylcarbonyl; a (C1-C6)alkoxycarbonyl; a (C1-C6)haloalkylsulfonyl; a (C1-C6)alkylcarbonyl (C1-C6)alkyl; a (C1-C6)alkoxycarbonyl (C1-C6)alkyl; a (C1-C6)alkylcarbonyloxy (C1-C6)alkyl; a (C1-C6)alkoxycarbonyloxy (C1-C6)alkyl; or a 5-methyl-1,3-dioxole-2-one-4-ylmethyl.

## Patentansprüche

1. 3-Alkoxybenzamid-Derivat der Formel [I] oder ein landwirtschaftlich akzeptables Salz davon, 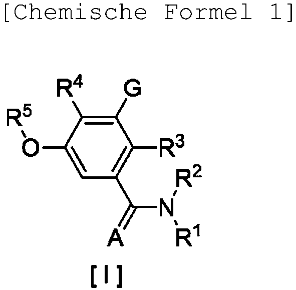 wobei in der Formel [I] gilt:
A steht für ein Sauerstoffatom oder ein Schwefelatom,
G steht für ein Wasserstoffatom,
R¹ steht für ein (C1-C6)-Alkyl; ein (C1-C6)-Halogenalkyl; ein (C3-C6)-Cycloalkyl; ein (C3-C6)-Halogencycloalkyl; ein (C2-C6)-Alkenyl; ein (C2-C6)-Alkinyl; ein (C1-C6)-Alkoxy; ein (C1-C6)-Halogenalkoxy; ein (C1-C6)-Alkoxy(C1-C6)-alkyl; ein (C3-C6)-Cycloalkyl-(C1-C6)-alkyl; ein Cyano-(C1-C6)-alkyl; ein Cyano-(C3-C6)-cycloalkyl; ein Cyano-(C3-C6)-cycloalkyl-(C1-C6)-alkyl; ein Cyano-Heterocycloalkyl; ein (C2-C6)Alkinyl(C3-C6)cycloalkyl; ein (C1-C6)Alkoxycarbonyl(C3-C6)cycloalkyl; ein Carboxy(C3-C6)cycloalkyl; ein Carbamoyl(C3-C6)cycloalkyl; ein (C4-C8)-Bicycloalkyl; ein Phenyl (das Phenyl ist gegebenenfalls mit R⁷ monosubstituiert oder polysubstituiert); ein Phenyl-(C1-C6)-alkyl (der Phenylrest ist gegebenenfalls mit R⁷ monosubstituiert oder polysubstituiert); ein Heteroaryl-(C1-C3)alkyl (der Heteroarylrest ist gegebenenfalls mit R⁷ monosubstituiert oder polysubstituiert); oder ein Heterocycloalkyl (das Heterocycloalkyl ist gegebenenfalls mit R¹⁰ substituiert),
R² steht für ein Wasserstoffatom; ein (C1-C6)-Alkyl; ein (C1-C6)-Halogenalkyl; ein (C2-C6)-Alkinyl; ein Cyano-(C1-C6)-alkyl; ein Hydroxy-(C1-C6)-alkyl; ein (C1-C6)-Alkoxy-(C1-C6)-alkyl; ein (C1-C6)-Alkylcarbonyl; ein (C1-C6)-Alkoxycarbonyl; ein (C1-C6)-Halogenalkoxycarbonyl; ein (C1-C6)-Alkoxycarbonyl-(C1-C6)-alkyl; ein Aminocarbonyl-(C1-C6)-alkyl; ein (C1-C6)Alkylaminocarbonyl(C1-C6)alkyl (die Aminogruppe ist gegebenenfalls mit R¹⁰ substituiert); ein (C1-C6)Alkylcarbonyloxy(C1-C6)alkyl; oder ein (C1-C6)Alkoxycarbonyloxy(C1-C6)alkyl, oder
R¹ und R² bilden zusammen mit dem Stickstoffatom, an das R¹ und R² gebunden sind, einen 3- bis 8-gliedrigen heterocyclischen Ring oder einen 3- bis 8-gliedrigen heterocyclischen Ring mit 1 bis 4 Heteroatomen, ausgewählt aus einem Sauerstoffatom, einem Schwefelatom und einem Stickstoffatom, wobei der gebildete heterocyclische Ring gegebenenfalls mit einem Halogenatom, einem Cyano, einem Nitro, einem (C1-C6)-Alkyl, einem (C1-C6)-Alkoxy, einem (C1-C6)-Halogenalkyl oder einem Oxo substituiert ist,
R³ steht für ein Halogenatom, ein (C1-C6)-Alkyl, ein (C1-C6)-Halogenalkyl oder ein (C1-C6)-Halogenalkoxy,
R⁴ steht für ein Wasserstoffatom, ein Halogenatom oder ein (C1-C6)-Alkyl,
R⁵ steht für ein Ethyl, ein n-Propyl, ein Isopropyl, ein n-Butyl, ein sec-Butyl, ein Isobutyl, ein tert-Butyl, ein n-Pentyl, ein 1-Methylbutyl, ein 2-Methylbutyl, ein 3-Methylbutyl, ein 1-Ethylpropyl, ein 1,1-Dimethylpropyl, ein 1,2-Dimethylpropyl, ein Neopentyl, ein n-Hexyl, ein 1-Methylpentyl, ein 2-Methylpentyl, ein 3-Methylpentyl, ein 4-Methylpentyl, ein 1-Ethylbutyl, ein 2-Ethylbutyl, ein 1,1-Dimethylbutyl, ein 1,2-Dimethylbutyl, ein 1,3-Dimethylbutyl, ein 2,2-Dimethylbutyl, ein 2,3-Dimethylbutyl, ein 3,3-Dimethylbutyl, ein 1,1,2-Trimethylpropyl, ein 1,2,2-Trimethylpropyl, ein 1-Ethyl-1-methylpropyl, ein 1-Ethyl-2-methylpropyl, ein n-Heptyl, ein n-Octyl, ein n-Nonyl, ein n-Decyl, ein n-Undecyl, ein n-Dodecyl, ein 4,4-Dimethylpentyl, ein 5-Methylhexyl, ein 5,5-Dimethylhexyl, ein 3,5,5-Trimethylhexyl, ein 6-Methylheptyl, ein 6,6-Dimethylheptyl, ein 3,6,6-Trimethylheptyl, ein 7-Methyloctyl, ein 7,7-Dimethyloctyl, ein 8-Methylnonyl, ein 8,8-Dimethylnonyl, ein 9-Methyldecyl, ein 9,9-Dimethyldecyl oder ein 10-Methylundecyl (das Ethyl, das n-Propyl, das Isopropyl, das n-Butyl, das sec-Butyl, das Isobutyl, das tert-Butyl, das n-Pentyl, das 1-Methylbutyl, das 2-Methylbutyl, das 3-Methylbutyl, das 1-Ethylpropyl, das 1,1-Dimethylpropyl, das 1,2-Dimethylpropyl, das Neopentyl, das n-Hexyl, das 1-Methylpentyl, das 2-Methylpentyl, das 3-Methylpentyl, das 4-Methylpentyl, das 1-Ethylbutyl, das 2-Ethylbutyl, das 1,1-Dimethylbutyl, das 1,2-Dimethylbutyl, das 1,3-Dimethylbutyl, das 2,2-Dimethylbutyl, das 2,3-Dimethylbutyl, das 3,3-Dimethylbutyl, das 1,1,2-Trimethylpropyl, das 1,2,2-Trimethylpropyl, das 1-Ethyl-1-methylpropyl, das 1-Ethyl-2-methylpropyl, das n-Heptyl, das n-Octyl, das n-Nonyl, das n-Decyl, das n-Undecyl, das n-Dodecyl, das 4,4-Dimethylpentyl, das 5-Methylhexyl, das 5,5-Dimethylhexyl, das 3,5,5-Trimethylhexyl, das 6-Methylheptyl, das 6,6-Dimethylheptyl, das 3,6,6-Trimethylheptyl, das 7-Methyloctyl, das 7,7-Dimethyloctyl, das 8-Methylnonyl, das 8,8-Dimethylnonyl, das 9-Methyldecyl, das 9,9-Dimethyldecyl oder das 10-Methylundecyl ist gegebenenfalls mit R⁶ monosubstituiert oder polysubstituiert); oder ein (C2- C6)Alkenyl (das (C2-C6)Alkenyl ist gegebenenfalls mit R⁶ monosubstituiert oder polysubstituiert),
R⁶ steht für ein Halogenatom; ein (C1-C6)-Alkyl; ein (C1-C6)-Halogenalkyl; ein (C3-C6)-Cycloalkyl; eine Hydroxy; ein Thiol; ein (C1-C6)-Alkylthio; ein (C1-C6)-Halogenalkylthio; ein (C1-C6)-Alkylsulfinyl; ein (C1-C6)-Halogenalkylsulfinyl; ein (C1-C6)-Alkylsulfonyl; ein (C1-C6)-Halogenalkylsulfonyl; ein (C1-C6)-Alkylcarbonyl; ein Amino; ein (C1-C6)-Alkylcarbonylamino (der Aminorest ist gegebenenfalls mit R¹⁰ substituiert); ein (C1-C6)-Halogenalkylcarbonylamino (der Aminorest ist gegebenenfalls mit R¹⁰ substituiert); ein (C1-C6)-Alkoxycarbonylamino (der Aminorest ist gegebenenfalls mit R¹⁰ substituiert); ein (C1-C6)-Halogenalkoxycarbonylamino (die Aminogruppe ist gegebenenfalls mit R¹⁰ substituiert); ein (C1-C6)-Halogenalkylsulfinylamino (die Aminogruppe ist gegebenenfalls mit R¹⁰ substituiert); ein (C1-C6)-Halogenalkylsulfonylamino (der Aminorest ist gegebenenfalls mit R¹⁰ substituiert); ein Phenylsulfonylamino (der Phenylrest ist gegebenenfalls mit R⁷ monosubstituiert oder polysubstituiert und der Aminorest ist gegebenenfalls mit R¹⁰ substituiert); ein Phenyl (das Phenyl ist gegebenenfalls mit R⁷ monosubstituiert oder polysubstituiert); ein Azetidinyl (ein Stickstoffatom des Azetidinyls ist gegebenenfalls mit R¹⁰ substituiert); ein 1,3-Dioxolanyl; ein Pyrazolyl (das Pyrazolyl ist gegebenenfalls mit R⁷ monosubstituiert oder polysubstituiert); ein Triazolyl (das Triazolyl ist gegebenenfalls mit R⁷ monosubstituiert oder polysubstituiert); ein 1,3-Dioxoisoindolinyl (das 1,3-Dioxoisoindolinyl ist gegebenenfalls mit R⁷ monosubstituiert oder polysubstituiert); oder ein Thiocyanato,
R⁷ steht für ein Halogenatom; ein (C1-C6)-Alkyl; ein (C1-C6)-Halogenalkyl; oder ein Cyano, und
R¹⁰ steht für ein (C1-C6)-Alkyl; ein (C1-C6)-Halogenalkyl; ein (C3-C6)-Cycloalkyl; ein (C1-C6)-Alkoxy(C1-C6)-alkyl; ein (C1-C6)Alkoxy(C1-C6)alkoxy(C1-C6)alkyl; ein (C1-C6)Alkylthio(C1-C6)alkyl; ein (C1-C6)Alkylsulfonyl(C1-C6)alkyl; ein Cyano(C1-C6)alkyl; ein (C1-C6)Alkylcarbonyl; ein (C1-C6)Alkoxycarbonyl; ein (C1-C6)Halogenalkylsulfonyl; ein (C1-C6)Alkylcarbonyl(C1-C6)alkyl; ein (C1-C6)Alkoxycarbonyl(C1-C6)alkyl; ein (C1-C6)Alkylcarbonyloxy(C1-C6)alkyl; ein (C1-C6)Alkoxycarbonyloxy(C1-C6)alkyl; oder ein 5-Methyl-1,3-dioxol-2-on-4-ylmethyl.

2. Agrochemische Zusammensetzung, die das 3-Alkoxybenzamid-Derivat oder dessen landwirtschaftlich akzeptables Salz gemäß Anspruch 1 als Wirkstoff enthält.

3. Agrochemische Zusammensetzung gemäß Anspruch 2, die ferner ein Tensid umfasst.

4. Schädlingsbekämpfungsmittel, das das 3-Alkoxybenzamid-Derivat oder dessen landwirtschaftlich verträgliches Salz gemäß Anspruch 1 als Wirkstoff enthält.

5. Schädlingsbekämpfungsmittel nach Anspruch 4, wobei das Schädlingsbekämpfungsmittel ein Insektizid, ein Nematizid und ein Akarizid ist.

6. Schädlingsbekämpfungsmittel gemäß Anspruch 5, wobei das Schädlingsbekämpfungsmittel in der Lage ist, Schädlinge in einem Reisfeld, einem Trockenfeld, einem Rasen, einem Obstgarten, einem Nichtanbaugebiet, einem Gewächshaus, einer Anzuchtanlage und einer Pflanzenfabrik, in denen landwirtschaftliche oder gartenbauliche Pflanzen angebaut werden, zu bekämpfen.

7. Schädlingsbekämpfungsmittel nach Anspruch 6, wobei die landwirtschaftliche oder gartenbauliche Pflanze eine Pflanze ist, der durch Züchtung oder genetische Modifikationstechniken eine Resistenz verliehen wurde.

8. Verfahren zur Bekämpfung eines Schädlings, umfassend einen Schritt des Aufbringens einer wirksamen Menge des 3-Alkoxybenzamid-Derivats oder des landwirtschaftlich akzeptablen Salzes davon gemäß Anspruch 1.

9. Verfahren zur Bekämpfung eines Schädlings, umfassend den Schritt des Aufbringens einer agrochemischen Zusammensetzung, die das 3-Alkoxybenzamid-Derivat oder dessen landwirtschaftlich akzeptables Salz gemäß Anspruch 1 als Wirkstoff enthält, auf eine landwirtschaftliche oder gartenbauliche Kulturpflanze oder einen Ort, an dem die landwirtschaftliche oder gartenbauliche Kulturpflanze angebaut werden soll oder angebaut wird, in einer einzigen oder in mehreren Dosen.

10. Verfahren zur Bekämpfung eines Schädlings gemäß Anspruch 8 oder 9, wobei ein Ort, an dem ein Schädlingsbekämpfungsmittel aufgebracht wird, ein Reisfeld, ein Trockenfeld, ein Rasen, ein Obstgarten, ein Nichtanbaugebiet, ein Gewächshaus, eine Anzuchtanlage und eine Pflanzenfabrik ist.

11. Verfahren zur Bekämpfung eines Schädlings gemäß einem der Ansprüche 8 bis 10, wobei das 3-Alkoxybenzamid-Derivat oder dessen landwirtschaftlich akzeptables Salz gemäß Anspruch 1 als Insektizid, Nematizid und Akarizid verwendet wird.

12. Verfahren zur Verwendung eines Schädlingsbekämpfungsmittels, **dadurch gekennzeichnet, dass** in dem Verfahren das Schädlingsbekämpfungsmittel gemäß einem der Ansprüche 4 bis 7 zur Bekämpfung eines Schädlings, der eine landwirtschaftliche oder gartenbauliche Kulturpflanze schädigt, angewendet wird.

13. Hydroxybenzamid-Derivat der Formel [II] oder ein Salz davon, 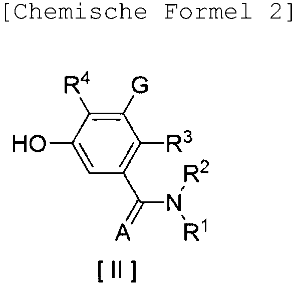 wobei in der Formel [II] gilt:
A steht für ein Sauerstoffatom oder ein Schwefelatom,
G steht für ein Wasserstoffatom,
R¹ steht für ein (C1-C6)-Alkyl; ein (C1-C6)-Halogenalkyl; ein (C3-C6)-Cycloalkyl; ein (C3-C6)-Halogencycloalkyl; ein (C2-C6)-Alkenyl; ein (C2-C6)-Alkinyl; ein (C1-C6)-Alkoxy; ein (C1-C6)-Halogenalkoxy; ein (C1-C6)-Alkoxy(C1-C6)-alkyl; ein (C3-C6)-Cycloalkyl-(C1-C6)-alkyl; ein Cyano-(C1-C6)-alkyl; ein Cyano-(C3-C6)-cycloalkyl; ein Cyano-(C3-C6)-cycloalkyl-(C1-C6)-alkyl; ein Cyano-Heterocycloalkyl; ein (C2-C6)Alkinyl(C3-C6)cycloalkyl; ein (C1-C6)Alkoxycarbonyl(C3-C6)cycloalkyl; ein Carboxy(C3-C6)cycloalkyl; ein Carbamoyl(C3-C6)cycloalkyl; ein (C4-C8)-Bicycloalkyl; ein Phenyl (das Phenyl ist gegebenenfalls mit R⁷ monosubstituiert oder polysubstituiert); ein Phenyl-(C1-C6)-alkyl (der Phenylrest ist gegebenenfalls mit R⁷ monosubstituiert oder polysubstituiert); ein Heteroaryl-(C1-C3)alkyl (der Heteroarylrest ist gegebenenfalls mit R⁷ monosubstituiert oder polysubstituiert); oder ein Heterocycloalkyl (das Heterocycloalkyl ist gegebenenfalls mit R¹⁰ substituiert),
R² steht für ein Wasserstoffatom; ein (C1-C6)-Alkyl; ein (C1-C6)-Halogenalkyl; ein (C2-C6)-Alkinyl; ein Cyano-(C1-C6)-alkyl; ein Hydroxy-(C1-C6)-alkyl; ein (C1-C6)-Alkoxy-(C1-C6)-alkyl; ein (C1-C6)-Alkylcarbonyl; ein (C1-C6)-Alkoxycarbonyl; ein (C1-C6)-Halogenalkoxycarbonyl; ein (C1-C6)-Alkoxycarbonyl-(C1-C6)-alkyl; ein Aminocarbonyl-(C1-C6)-alkyl; ein (C1-C6)Alkylaminocarbonyl(C1-C6)alkyl (die Aminogruppe ist gegebenenfalls mit R¹⁰ substituiert); ein (C1-C6)Alkylcarbonyloxy(C1-C6)alkyl; oder ein (C1-C6)Alkoxycarbonyloxy(C1-C6)alkyl, oder
R¹ und R² bilden zusammen mit dem Stickstoffatom, an das R¹ und R² gebunden sind, einen 3- bis 8-gliedrigen heterocyclischen Ring oder einen 3- bis 8-gliedrigen heterocyclischen Ring mit 1 bis 4 Heteroatomen, ausgewählt aus einem Sauerstoffatom, einem Schwefelatom und einem Stickstoffatom, und der heterocyclische Ring gegebenenfalls mit einem Halogenatom, einem Cyano, einem Nitro, einem (C1-C6)-Alkyl, einem (C1-C6)-Alkoxy, einem (C1-C6)-Halogenalkyl oder einem Oxo substituiert ist,
R³ steht für ein Halogenatom, ein (C1-C6)-Alkyl, ein (C1-C6)-Halogenalkyl oder ein (C1-C6)-Halogenalkoxy,
R⁴ steht für ein Wasserstoffatom, ein Halogenatom oder ein (C1-C6)-Alkyl,
R⁷ steht für ein Halogenatom; ein (C1-C6)-Alkyl; ein (C1-C6)-Halogenalkyl; oder ein Cyano, und
R¹⁰ steht für ein (C1-C6)-Alkyl; ein (C1-C6)-Halogenalkyl; ein (C3-C6)-Cycloalkyl; ein (C1-C6)-Alkoxy(C1-C6)-alkyl; ein (C1-C6)Alkoxy(C1-C6)alkoxy(C1-C6)alkyl; ein (C1-C6)Alkylthio(C1-C6)alkyl; ein (C1-C6)Alkylsulfonyl(C1-C6)alkyl; ein Cyano(C1-C6)alkyl; ein (C1-C6)Alkylcarbonyl; ein (C1-C6)Alkoxycarbonyl; ein (C1-C6)Halogenalkylsulfonyl; ein (C1-C6)Alkylcarbonyl(C1-C6)alkyl; ein (C1-C6)Alkoxycarbonyl(C1-C6)alkyl; ein (C1-C6)Alkylcarbonyloxy(C1-C6)alkyl; ein (C1-C6)Alkoxycarbonyloxy(C1-C6)alkyl; oder ein 5-Methyl-1,3-dioxol-2-on-4-ylmethyl.

## Revendications

1. Dérivé 3-alcoxybenzamide de formule [I], ou un sel acceptable en agriculture de celui-ci, 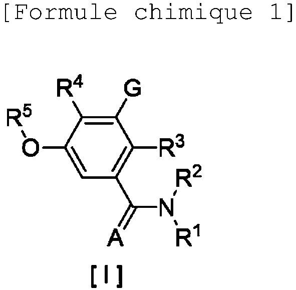 dans laquelle, dans la formule [I],
A représente un atome d'oxygène ou un atome de soufre,
G représente un atome d'hydrogène,
R¹ représente un (C1-C6)alkyle ; un halogéno(C1-C6)alkyle ; un (C3-C6)cycloalkyle ; un halogéno(C3-C6)cycloalkyle ; un (C2-C6)alcényle ; un (C2-C6)alcynyle ; un (C1-C6)alcoxy ; un halogéno(C1-C6)alcoxy ; un (C1-C6)alcoxy (C1-C6)alkyle ; un (C3-C6)cycloalkyle (C1-C6)alkyle ; un cyano(C1-C6)alkyle ; un cyano(C3-C6)cycloalkyle ; un cyano(C3-C6)cycloalkyle (C1-C6)alkyle ; un cyano hétérocycloalkyle ; un (C2-C6)alcynyl(C3-C6)cycloalkyle ; un (C1-C6)alcoxycarbonyl(C3-C6)cycloalkyle ; un carboxy(C3-C6)cycloalkyle ; un carbamoyl(C3-C6)cycloalkyle ; un (C4-C8)bicycloalkyle ; un phényle (le phényle étant éventuellement mono-substitué ou poly-substitué par R⁷) ; un phényl(C1-C6)alkyle (le phényle étant éventuellement mono-substitué ou poly-substitué par R⁷) ; un hétéroaryl(C1-C3)alkyle (le fragment hétéroaryle étant éventuellement mono-substitué ou poly-substitué par R⁷) ; ou un hétérocycloalkyle (le hétérocycloalkyle étant éventuellement substitué par R¹⁰),
R² représente un atome d'hydrogène ; un (C1-C6)alkyle ; un halogéno(C1-C6)alkyle ; un (C2-C6)alcynyle ; un cyano(C1-C6)alkyle ; un hydroxy(C1-C6)alkyle ; un alcoxy(C1-C6) (C1-C6)alkyle ; un (C1-C6)alkylcarbonyle ; un (C1-C6)alcoxycarbonyle ; un (C1-C6)halogénoalcoxycarbonyle ; un (C1-C6)alcoxycarbonyl(C1-C6)alkyle ; un aminocarbonyl(C1-C6)alkyle ; un (C1-C6)alkylaminocarbonyle (C1-C6)alkyle (le fragment amino étant éventuellement substitué par R¹⁰); un (C1-C6)alkylcarbonyloxy(C1-C6)alkyle ; ou un (C1-C6)alcoxycarbonyloxy(C1-C6)alkyle, ou
R¹ et R² , conjointement avec l'atome d'azote auquel R¹ et R² sont liés, forment un cycle hétérocyclique de 3 à 8 chaînons ou un cycle hétérocyclique de 3 à 8 chaînons comportant 1 à 4 hétéroatomes choisis parmi un atome d'oxygène, un atome de soufre et un atome d'azote, dans lequel le cycle hétérocyclique formé est éventuellement substitué par un atome d'halogène, un cyano, un nitro, un (C1-C6)alkyle, un (C1-C6)alcoxy, un halogéno(C1-C6)alkyle ou un oxo,
R³ représente un atome d'halogène ; un (C1-C6)alkyle ; un halogéno(C1-C6)alkyle ; ou un (C1-C6)halogénoalcoxy,
R⁴ représente un atome d'hydrogène ; un atome d'halogène ; ou un (C1-C6)alkyle,
R⁵ représente un éthyle, un n-propyle, un isopropyle, un n-butyle, un sec-butyle, un isobutyle, un tert-butyle, un n-pentyle, un 1-méthylbutyle, un 2-méthylbutyle, un 3-méthylbutyle, un 1-éthylpropyle, un 1,1-diméthylpropyle, un 1,2-diméthylpropyle, un néopentyle, un n-hexyle, un 1-méthylpentyle, un 2-méthylpentyle, un 3-méthylpentyle, un 4-méthylpentyle, un 1-éthylbutyle, un 2-éthylbutyle, un 1,1- diméthylbutyle, un 1,2-diméthylbutyle, un 1,3-diméthylbutyle, un 2,2-diméthylbutyle, un 2,3-diméthylbutyle, un 3,3-diméthylbutyle, un 1,1,2-triméthylpropyle, un 1,2,2-triméthylpropyle, un 1-éthyl-1-méthylpropyle, un 1-éthyl-2-méthylpropyle, un n-heptyle, un n-octyle, un n-nonyle, un n-décyle, un n-undécyle, un n-dodécyle, un 4,4-diméthylpentyle, un 5-méthylhexyl, un 5,5-diméthylhexyl, un 3,5,5-triméthylhexyl, un 6-méthylheptyl, un 6,6-diméthylheptyl, un 3,6,6-triméthylheptyl, un 7-méthyloctyl, un 7,7-diméthyloctyl, un 8-méthylnonyl, un 8,8-diméthylnonyl, un 9-méthyldécyl, un 9,9-diméthyldécyl ou un 10-méthylundécyl (l'éthyl, le n-propyl, l'isopropyl, le n-butyl, le sec-butyl, l'isobutyl, le tert-butyl, le n-pentyle, le 1-méthylbutyle, le 2-méthylbutyle, le 3-méthylbutyle, le 1-éthylpropyle, le 1,1-diméthylpropyle, le 1,2-diméthylpropyle, le néopentyle, le n-hexyle, le 1-méthylpentyle, le 2-méthylpentyle, le 3-méthylpentyle, le 4-méthylpentyle, le 1-éthylbutyle, le 2-éthylbutyle, le 1,1-diméthylbutyle, le 1,2-diméthylbutyle, le 1,3-diméthylbutyle, le 2,2-diméthylbutyle, le 2,3-diméthylbutyle, le 3,3-diméthylbutyle, le 1,1,2-triméthylpropyle, le 1,2,2-triméthylpropyle, le 1-éthyl-1-méthylpropyle, le 1-éthyl-2-méthylpropyle, le n-heptyle, le n-octyle, le n-nonyle, le n-décyle, le n-undécyle, le n-dodécyle, le 4,4-diméthylpentyle, le 5-méthylhexyle, le 5,5-diméthylhexyle, le 3,5,5-triméthylhexyle, le 6-méthylheptyle, le 6,6-diméthylheptyle, le 3,6,6-triméthylheptyle, le 7-méthyloctyle, le 7,7-diméthyloctyle, le 8-méthylnonyle, le 8,8-diméthylnonyle, le 9-méthyldécyle, le 9,9-diméthyldécyle ou le 10-méthylundécyle est éventuellement mono-substitué ou poly-substitué par R⁶) ; ou un (C2- C6)alcényle (le (C2-C6)alcényle étant éventuellement mono-substitué ou poly-substitué par R⁶),
R⁶ représente un atome d'halogène ; un (C1-C6)alkyle ; un halogéno(C1-C6)alkyle ; un (C3-C6)cycloalkyle ; un hydroxy ; un thiol ; un (C1-C6)alkylthio ; un halogéno(C1-C6)alkylthio ; un (C1-C6)alkylsulfinyle ; un halogéno(C1-C6)alkylsulfinyle ; un C1-C6)alkylsulfonyle ; un halogénoC1-C6)alkylsulfonyle ; un (C1-C6)alkylcarbonyle ; un amino ; un (C1-C6)alkylcarbonylamino (le fragment amino étant éventuellement substitué par R¹⁰) ; un (C1-C6)halogénoalkylcarbonylamino (le fragment amino étant éventuellement substitué par R¹⁰) ; un (C1-C6)alcoxycarbonylamino (le fragment amino étant éventuellement substitué par R¹⁰) ; un (C1-C6)halogénoalcoxycarbonylamino (le fragment amino étant éventuellement substitué par R¹⁰) ; un (C1-C6)halogénoalkylsulfinylamino (le fragment amino étant éventuellement substitué par R¹⁰) ; un (C1-C6)halogénoalkylsulfonylamino (le fragment amino étant éventuellement substitué par R¹⁰) ; un phénylsulfonylamino (le fragment phényle étant éventuellement mono-substitué ou poly-substitué par R⁷, et le fragment amino étant éventuellement substitué par R¹⁰) ; un phényle (le phényle est éventuellement mono-substitué ou poly-substitué par R⁷) ; un azétidinyle (un atome d'azote du groupe azétidinyle est éventuellement substitué par R¹⁰) ; un 1,3-dioxolanyle ; un pyrazolyle (le pyrazolyle est éventuellement mono-substitué ou poly-substitué par R⁷) ; un triazolyle (le triazolyle étant éventuellement mono- ou polysubstitué par R⁷) ; un 1,3-dioxoisoindolinyle (le 1,3-dioxoisoindolinyle étant éventuellement mono- ou polysubstitué par R⁷) ; ou un thiocyanato,
R⁷ représente un atome d'halogène ; un (C1-C6)alkyle ; un halogéno(C1-C6)alkyle ; ou un cyano, et
R¹⁰ représente un (C1-C6)alkyle ; un halogéno(C1-C6)alkyle ; un (C3-C6)cycloalkyle ; un (C1-C6)alcoxy (C1-C6)alkyle ; un (C1-C6)alcoxy (C1-C6)alcoxy (C1-C6)alkyle ; un (C1-C6)alkylthio (C1-C6)alkyle ; un C1-C6)alkylsulfonyle (C1-C6)alkyle ; un cyano(C1-C6)alkyle ; un (C1-C6)alkylcarbonyle ; un (C1-C6)alcoxycarbonyle ; un (C1-C6)halogénoalkylsulfonyle ; un (C1-C6)alkylcarbonyle (C1-C6)alkyle ; un (C1-C6)alcoxycarbonyle (C1-C6)alkyle ; un (C1-C6)alkylcarbonyloxy(C1-C6)alkyle ; un (C1-C6)alcoxycarbonyloxy(C1-C6)alkyle ; ou un 5-méthyl-1,3-dioxole-2-one-4-ylméthyle.

2. Composition agrochimique comprenant le dérivé 3-alcoxybenzamide ou son sel acceptable en agriculture selon la revendication 1 en tant qu'ingrédient actif.

3. Composition agrochimique selon la revendication 2, qui comprend en outre un tensioactif.

4. Agent antiparasitaire comprenant le dérivé 3-alcoxybenzamide ou son sel acceptable en agriculture selon la revendication 1 en tant qu'ingrédient actif.

5. Agent antiparasitaire selon la revendication 4, dans lequel l'agent antiparasitaire est un insecticide, un nématicide et un acaricide.

6. Agent antiparasitaire selon la revendication 5, dans lequel l'agent antiparasitaire est capable de lutter contre les parasites dans une rizière, un champ sec, une pelouse, un verger, une terre non cultivée, une serre, une installation de semis et une usine végétale où sont cultivées des plantes agricoles ou horticoles.

7. Agent antiparasitaire selon la revendication 6, dans lequel la plante agricole ou horticole est une plante à laquelle une résistance est conférée par des techniques de sélection ou de modification génétique.

8. Procédé de lutte contre un ravageur, comprenant une étape consistant à appliquer une quantité efficace du dérivé 3-alcoxybenzamide ou de son sel acceptable en agriculture selon la revendication 1.

9. Procédé de lutte contre un parasite, comprenant une étape consistant à appliquer une composition agrochimique comprenant le dérivé 3-alcoxybenzamide ou son sel acceptable en agriculture selon la revendication 1 en tant qu'ingrédient actif à une culture agricole ou horticole ou à un endroit où la culture agricole ou horticole doit être cultivée ou est en cours de culture, en une seule dose ou en doses fractionnées.

10. Procédé de lutte contre un parasite selon la revendication 8 ou 9, dans lequel l'endroit où l'agent antiparasitaire est appliqué est une rizière, un champ sec, une pelouse, un verger, une terre non cultivée, une serre, une installation de semis et une usine végétale.

11. Procédé de lutte contre un parasite selon l'une quelconque des revendications 8 à 10, dans lequel le dérivé 3-alcoxybenzamide ou son sel acceptable en agriculture selon la revendication 1 est utilisé comme insecticide, nématicide et acaricide.

12. Procédé d'utilisation d'un agent de lutte contre les parasites, **caractérisé en ce que**, dans le procédé, l'agent de lutte contre les parasites selon l'une quelconque des revendications 4 à 7 est appliqué pour lutter contre un parasite nuisible à une culture agricole ou horticole.

13. Dérivé d'hydroxybenzamide de formule [II], ou un sel de celui-ci, 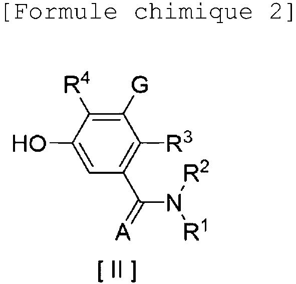 dans laquelle, dans la formule [II],
A représente un atome d'oxygène ou un atome de soufre,
G représente un atome d'hydrogène,
R¹ représente un (C1-C6)alkyle ; un halogéno(C1-C6)alkyle ; un (C3-C6)cycloalkyle ; un halogéno(C3-C6)cycloalkyle ; un (C2-C6)alcényle ; un (C2-C6)alcynyle ; un (C1-C6)alcoxy ; un halogéno(C1-C6)alcoxy ; un (C1-C6)alcoxy (C1-C6)alkyle ; un (C3-C6)cycloalkyle (C1-C6)alkyle ; un cyano(C1-C6)alkyle ; un cyano(C3-C6)cycloalkyle ; un cyano(C3-C6)cycloalkyle (C1-C6)alkyle ; un cyano hétérocycloalkyle ; un (C2-C6)alcynyl (C3-C6)cycloalkyle ; un (C1-C6)alcoxycarbonyl(C3-C6)cycloalkyle ; un carboxy(C3-C6)cycloalkyle ; un carbamoyl(C3-C6)cycloalkyle ; un (C4-C8)bicycloalkyle ; un phényle (le phényle étant éventuellement mono-substitué ou poly-substitué par R⁷) ; un phényl(C1-C6)alkyle (le phényle étant éventuellement mono-substitué ou poly-substitué par R⁷) ; un hétéroaryl(C1-C3)alkyle (le fragment hétéroaryle étant éventuellement mono-substitué ou poly-substitué par R⁷) ; ou un hétérocycloalkyle (le hétérocycloalkyle étant éventuellement substitué par R¹⁰),
R² représente un atome d'hydrogène ; un (C1-C6)alkyle ; un halogéno(C1-C6)alkyle ; un (C2-C6)alcynyle ; un cyano(C1-C6)alkyle ; un hydroxy(C1-C6)alkyle ; un alcoxy(C1-C6) (C1-C6)alkyle ; un (C1-C6)alkylcarbonyle ; un (C1-C6)alcoxycarbonyle ; un (C1-C6)halogénoalcoxycarbonyle ; un (C1-C6)alcoxycarbonyl(C1-C6)alkyle ; un aminocarbonyl(C1-C6)alkyle ; un (C1-C6)alkylaminocarbonyle (C1-C6)alkyle (le fragment amino étant éventuellement substitué par R¹⁰); un (C1-C6)alkylcarbonyloxy(C1-C6)alkyle ; ou un (C1-C6)alcoxycarbonyloxy(C1-C6)alkyle, ou
R¹ et R² , conjointement avec l'atome d'azote auquel R¹ et R² sont liés, forment un cycle hétérocyclique de 3 à 8 chaînons ou un cycle hétérocyclique de 3 à 8 chaînons comportant 1 à 4 hétéroatomes choisis parmi un atome d'oxygène, un atome de soufre et un atome d'azote, et le cycle hétérocyclique est éventuellement substitué par un atome d'halogène, un cyano, un nitro, un (C1-C6)alkyle, un (C1-C6)alcoxy, un halogéno(C1-C6)alkyle ou un oxo,
R³ représente un atome d'halogène ; un (C1-C6)alkyle ; un halogéno(C1-C6)alkyle ; ou un (C1-C6)halogénoalcoxy,
R⁴ représente un atome d'hydrogène ; un atome d'halogène ; ou un (C1-C6)alkyle,
R⁷ représente un atome d'halogène ; un (C1-C6)alkyle ; un halogéno(C1-C6)alkyle ; ou un cyano, et
R¹⁰ représente un (C1-C6)alkyle ; un halogéno(C1-C6)alkyle ; un (C3-C6)cycloalkyle ; un (C1-C6)alcoxy (C1-C6)alkyle ; un (C1-C6)alcoxy (C1-C6)alcoxy (C1-C6)alkyle ; un (C1-C6)alkylthio (C1-C6)alkyle ; un C1-C6)alkylsulfonyle (C1-C6)alkyle ; un cyano(C1-C6)alkyle ; un (C1-C6)alkylcarbonyle ; un (C1-C6)alcoxycarbonyle ; un (C1-C6)halogénoalkylsulfonyle ; un (C1-C6)alkylcarbonyle (C1-C6)alkyle ; un (C1-C6)alcoxycarbonyle (C1-C6)alkyle ; un (C1-C6)alkylcarbonyloxy(C1-C6)alkyle ; un (C1-C6)alcoxycarbonyloxy(C1-C6)alkyle ; ou un 5-méthyl-1,3-dioxole-2-one-4-ylméthyle.
